# EUROPEAN PATENT APPLICATION

(11) **EP 4 029 940 A1**
(43) Date of publication of application: **20.07.2022**
(21) Application number: 20862815.6
(22) Date of filing: 08.09.2020
(51) Int. Cl.: C12N 15/10, C12Q 1/6806, C12Q 1/6827, C12Q 1/6837

(54) **BODY FLUID EXTRACT CONTAINING MICRO RNA**

(30) Priority: 09.09.2019 JP 2019193847
(71) Applicant: Craif Inc., Tokyo 1130033 (JP)
(72) Inventor: YASUI, Takao, Nagoya-shi, Aichi 464-8601 (JP); BABA, Yoshinobu, Nagoya-shi, Aichi 464-8601 (JP); ICHIKAWA, Yuki, Tokyo 113-0033 (JP)
(74) Representative: Michalski Hüttermann & Partner Patentanwälte mbB
(86) International application number: PCT/JP2020/037487
(87) International publication number: WO 2021/049671

(57) **Abstract**

The present disclosure provides a body fluid extract containing micro RNAs. According to the present disclosure, there is provided a body fluid extract containing any of the micro RNAs shown in Data S1 or Table 2, or in any of Tables 4-1 to 4-15.

## Description

### TECHNICAL FIELD

The present disclosure relates to body fluid extracts comprising microRNA

### BACKGROUND OF THE INVENTION

The inclusion of micro-RNAs (miRNA) in the interior of extracellular vesicles (hereinafter, simply referred to as "EVs") (Non-Patent Documents 1 to 4), such as exosomes, microvesicles, and apoptotic bodies, has been found in various body fluids, including healthy and sick individuals (Non-Patent Documents 6 to 20). Differences in the EV-inclusion miRNAs between the two groups of humans can be a sign of warning of various diseases [20].It is believed that the inclusion of miRNAs into EVs is advantageous in that the effect of ribonuclease on RNA degradation can be reduced (Non-Patent Document 21), and it is believed that miRNAs in EVs are more stable than free floating miRNAs.

Until now, 3 techniques have been used for capture of EVs : ultracentrifugation or differential centrifugation, capture based on immunoaffinity, and size exclusion chromatography (Non-Patent Document 4). Expected alternatives have been reported, such as polymer precipitation methods (Non-Patent Document 22), platforms based on microfluidics (Non-Patent Documents 23 to 26), and filtration methods based on size (Non-Patent Document 27). However, these existing methods of capturing EV-containing miRNA have not been adequate for recovering EV from urines containing EV at very low concentrations (<0.01 vol %) (28). For example, ultracentrifugation is the most commonly used method for the capture of EV in urine. Ultracentrifugation has identified between 200 and 300 miRNA types in urine [29-31]. More than 2,000 human miRNAs have been reported. The remaining 90% did not appear to be present or absent in the urine.

### Prior Art Documents

### Non-Patent Document

Non-Patent Document 1: G.Raposo, W.Stoorvogel, J.Cell Biol. 200,373-383 (2013).
Non-Patent Document 2: I. Evans-Osses, L. H. Reichembach, M. I., Parasitol. Res. 114, 3567-3575(2015).
Non-Patent Document 3: P. Ma, Y.et al., J. Hematol. Oncol. 10, 57 (2017).
Non-Patent Document 4: R.Szatanek et al., Int. J. Mol. Med. 36, 11-17 (2015).
Non-Patent Document 5: D. K. Jeppesen et al., J. Extracell. Vesicles 3, 25011 (2014).
Non-Patent Document 6: J.A.Weber et al., Clin. Chem. 56, 1733-1741 (2010).
Non-Patent Document 7: L.-L.Lv et al., Int. J. Biol. Sci.9, 1021-1031 (2013).
Non-Patent Document 8: M.L.Alvarez et al.,Kidney Int. 82, 1024-1032 (2012).
Non-Patent Document 9: J.Zhang et al., Genomics Proteomics Bioinformatics 13, 17-24 (2015).
Non-Patent Document 10: N.Kosaka et al., Cancer Sci. 101, 2087-2092 (2010).
Non-Patent Document 11: M.Iero et al., Cell Death Differ. 15, 80-88 (2008).
Non-Patent Document 12: D. D. Taylor, C. Gercel-Taylor, Gynecol. Oncol. 110, 13-21 (2008).
Non-Patent Document 13: K.Al-Nedawi et al., Nat.Cell Biol. 10, 619-624 (2008).
Non-Patent Document 14: Y.Yoshioka et al., Nat. Commun.5, 3591 (2014).
Non-Patent Document 15: H.Peinado et al., Nat. Med. 18, 883-891 (2012).
Non-Patent Document 16: C.Y.Chen et al., Methods Enzymol. 524, 225-241 (2013).
Non-Patent Document 17: J.Webber et al., Cancer Res. 70, 9621-9630 (2010).
Non-Patent Document 18: J.Skog et al., Nat.Cell Biol. 10, 1470-1476 (2008).
Non-Patent Document 19: A.V.Vlassov et al., Biochim. Biophys. Acta 1820, 910-948 (2012).
Non-Patent Document 20: C.H.Arnaud, Chem. Eng. News 93, 30-32 (2015).
Non-Patent Document 21: M. B. Kirschner et al., Front. Genet. 4, 94 (2013).
Non-Patent Document 22: D. D. Taylor et al., Methods Mol. Biol. 728, 235-246 (2011).
Non-Patent Document 23: S.Jeong et al., ACS Nano 10, 1802-1809 (2016).
Non-Patent Document 24: V. Sunkara et al., Analyst 141, 371-381 (2016).
Non-Patent Document 25: P. Zhang, M. He, Y. Zeng, Lab Chip 16, 3033-3042 (2016).
Non-Patent Document 26: B. H. Wunsch et al., Nat. Nanotechnol. 11, 936-940 (2016).
Non-Patent Document 27: H.-K. Woo et al., ACS Nano 11, 1360-1370 (2017).
Non-Patent Document 28: F. Barutta et al., PLOS ONE 8, e73798 (2013).
Non-Patent Document 29: C. Thery et al., Cell Biol. Chapter 3, Unit 3. 22 (2006).
Non-Patent Document 30: K. E. Petersen et al., Anal. Bioanal. Chem. 406 (30), 7855-7866 (2014).

### SUMMARY OF THE INVENTION

The present disclosure provides a body fluid extract comprising microRNA.

The present inventors have found that upon contacting nanowires (nanorods) with a solution containing extracellular vesicles (EVs) having a positive charge in solution (particularly at the pH of urine), EVs can be efficiently captured on the nanowires. The present inventors have found that by contacting urine with the nanowires, EVs and miRNAs in urine can be effectively captured, and thus a urine extract containing types of miRNAs which cannot be extracted by a conventional method can be obtained. The present inventors also succeeded in detecting various types of miRNAs that were not considered to be detected in urine or urine extracts by analyzing urine of patients with various diseases. These miRNAs may be useful as predictive markers of disease conditions and the like. The present disclosure is based on such findings.

According to the present disclosure, the following industrially available inventions can be provided.
(1) An extract of urine, comprising a microRNA selected from the group consisting of (i) to (xvi) below:
   (i) any of the microRNAs listed in data S1 or Table 2;
   (ii) any of the microRNAs listed in Table 4-1;
   (iii) any of the microRNAs listed in Table 4-2;
   (iv) any of the microRNAs listed in Table 4-3;
   (v) any of the microRNAs listed in Table 4-4;
   (vi) any of the microRNAs listed in Table 4-5;
   (vii) any of the microRNAs listed in Table 4-6;
   (viii) any of the microRNAs listed in Table 4-7;
   (ix) any of the microRNAs listed in Table 4-8;
   (x)any of the microRNAs listed in Table 4-9;
   (xi) any of the microRNAs listed in Table 4-10;
   (xii) any of the microRNAs listed in Table 4-11;
   (xiii) any of the microRNAs listed in Table 4-12;
   (xiv) any of the microRNAs listed in Table 4-13;
   (xv) any of the microRNAs listed in Table 4-14; and
   (xvi) any of the microRNAs listed in Table 4-15.
(2) The extract of urine according to (1) above, comprising an extracellular vesicle, wherein said microRNA is contained in the extracellular vesicle, or wherein said microRNA is extracted from the extracellular vesicle.
(3) The extract of urine according to (1) above, wherein said RNA is in the form of free microRNA.
(4) An extract of urine according to any one of (1) to (3) above, wherein the microRNA is at least 1 or all microRNAs selected from the group consisting of miR-3117-5p, miR-3118, miR-3121-3p, miR-3121-5p, miR-3126-5p, miR-3128, miR-3133, miR-3134, miR-3136-3p, miR-3136-5p, miR-3139, miR-3142, miR-3143, miR-3145-3p, miR-3163, miR-3166, miR-3167, miR-16-1-3p, miR-424-3p, miR-519c-5p, miR-525-5p, miR-551b-5p, miR-558, miR-921, miR-942-3p, miR-3126-3p, miR-3127-5p, miR-3129-5p, miR-3144-5p, miR-3150a-5p, miR-3152-5p, miR-3155a, miR-3157-3p, miR-3159, miR-3165, miR-3678-3p, miR-4321, miR-4521, miR-4800-3p, miR-4999-5p, miR-5096, miR-5187-5p, miR-6874-5p, miR-3127-3p, miR-3130-5p, miR-3131, miR-3141, miR-3150b-5p, miR-3151-3p, miR-3151-5p, miR-3154, miR-3160-3p, miR-3160-5p, miR-378a-5p, miR-520c-3p, miR-526b-3p, miR-3150a-3p, miR-3162-5p, and miR-4254.
(5) The extract of urine according to any one of (1) to (4) above, wherein the microRNA is at least one microRNA or all microRNAs selected from the group consisting of miR-3163, miR-16-1-3p, miR-424-3p, miR-558, miR-3127-5p, and miR-4521.
(6) The extract of urine according to any one of (1) to (4) above, wherein the microRNA is at least one microRNA or all microRNAs selected from the group consisting of miR-378a-5p, miR-520c-3p, and miR-526b-3p.
(7) The extract of urine according to any one of (1) to (6) above, wherein the urine is urine of a subject having lung cancer.
(8) The extract of urine according to any one of (1) to (3) above, wherein the microRNA is at least 1 or all microRNAs selected from the group consisting of let-7i-3p, miR-183-5p, miR-202-5p, miR-409-5p, miR-4661-5p, miR-4800-3p, miR-5587-5p, miR-372-3p, miR-378b, miR-520b, miR-1266-3p, miR-3605-5p, miR-3612, miR-4645-3p, miR-4694-3p, miR-4752, miR-6816-3p, miR-8087, let-7f-2-3p, miR-15a-3p, miR-20a-3p, miR-33b-3p, miR-34c-5p, miR-93-5p, miR-130a-5p, miR-135a-5p, miR-135b-5p, miR-185-5p, miR-203a-3p, miR-302d-5p, miR-337-3p, miR-378c, miR-422a, miR-449c-5p, miR-483-5p, miR-506-3p, miR-511-5p, miR-520c-3p, miR-654-3p, miR-668-5p, miR-670-5p, miR-671-3p, miR-744-3p, miR-1178-3p, miR-1254, miR-1284, miR-1323, miR-2116-5p, miR-2355-3p, miR-3132, miR-3138, miR-3164, miR-3186-3p, miR-3189-3p, miR-3198, miR-3200-5p, miR-3657, miR-3667-5p, miR-3680-5p, miR-3692-5p, miR-3713, miR-3921, miR-3936, miR-4273, miR-4299, miR-4306, miR-4316, miR-4319, miR-4421, miR-4429, miR-4435, miR-4441, miR-4473, miR-4506, miR-4633-5p, miR-4658, miR-4733-5p, miR-4733-3p, miR-5004-3p, miR-5194, miR-5197-5p, miR-5571-5p, miR-6083, miR-6717-5p, miR-6720-5p, miR-6767-3p, miR-6781-3p, miR-6811-3p, miR-6821-3p, miR-6828-5p, miR-6832-5p, miR-6837-3p, miR-6841-5p, miR-6853-5p, miR-6871-3p, miR-6875-5p, miR-6878-5p, miR-7112-3p, miR-7703, miR-7848-3p, and miR-7856-5p.
(9) The extract of urine according to (8) above, wherein the microRNA is at least 1 or all microRNAs selected from the group consisting of miR-183-5p, miR-202-5p, and miR-409-5p.
(10) The extract of urine according to (8) above, wherein the microRNA is at least 1 or all microRNAs selected from the group consisting of miR-372-3p, miR-520b, miR-15a-3p, miR-34c-5p, miR-135a-5p, miR-185-5p, miR-337-3p, miR-422a, miR-506-3p, miR-520c-3p, miR-1284, miR-1323, and miR-4273.
(11) The extract of urine according to any one of (8) to (10) above, wherein the urine is urine of a subject having pancreatic cancer.
(12) The extract of urine according to any one of (1) to (3) above, wherein the microRNA is at least 1 or all microRNAs selected from the group consisting of miR-4521, let-7c-3p, let-7i-5p, miR-16-1-3p, miR-26a-1-3p, miR-28-5p, miR-105-5p, miR-195-3p, miR-200b-5p, miR-219a-2-3p, miR-297, miR-300, miR-330-3p, miR-374b-5p, miR-431-5p, miR-454-5p, miR-513c-5p, miR-548ax, miR-593-5p, miR-623, miR-664a-5p, miR-942-3p, miR-1205, miR-1276, miR-1288-3p, miR-1297, miR-3678-3p, miR-4283, miR-4295, miR-4439, miR-4524b-5p, miR-4703-3p, miR-4768-5p, miR-4800-3p, miR-5187-5p, miR-5696, miR-7161-5p, let-7i-2-3p, and miR-520c-3p.
(13) The extract of urine according to (12) above, wherein the microRNA is at least 1 or all microRNAs selected from the group consisting of miR-16-1-3p, miR-28-5p, miR-297, miR-300, miR-330-3p, miR-454-5p, miR-1297, and miR-4295.
(14) The extract of urine according to (12) above, wherein the microRNA is miR-520c-3p.
(15) The extract of urine according to any one of (12) to (14) above, wherein the urine is urine of a subject having liver cancer.
(16) The extract of urine according to any one of (1) to (3) above, wherein the microRNA is at least 1 or all microRNAs selected from the group consisting of miR-16-1-3p, miR-23b-3p, miR-28-5p, miR-92a-2-5p, miR-142-3p, miR-195-3p, miR-196b-5p, miR-299-3p, miR-492, miR-513b-5p, miR-601, miR-619-5p, miR-1285-3p, miR-3155a, miR-3162-5p, miR-3678-3p, miR-4283, miR-4295, miR-4311, miR-4531, miR-5096, miR-5187-5p, let-7f-2-3p, miR-520c-3p, and miR-4783-5p.
(17) The extract of urine according to (16) above, wherein the microRNA is at least one or all microRNAs selected from the group consisting of miR-16-1-3p, miR-23b-3p, miR-28-5p, miR-142-3p, miR-195-3p, miR-299-3p, and miR-4295.
(18) The extract of urine according to (16) above, wherein the microRNA is miR-520c-3p.
(19) The extract of urine according to any of (16) to (18) above, wherein the urine is urine of a subject having bladder cancer.
(20) The extract of urine according to any one of (1) to (3) above, wherein the microRNA is at least 1 or all microRNAs selected from the group consisting of miR-4531, miR-28-5p, miR-103a-2-5p, miR-105-5p, miR-124-3p, miR-151a-5p, miR-151b, miR-200a-5p, miR-300, miR-424-3p, miR-519c-5p, miR-551b-5p, miR-617, miR-873-3p, miR-921, miR-1288-3p, miR-3124-5p, miR-3155a, miR-3917, miR-4283, miR-4727-3p, miR-5096, miR-5187-5p, miR-6074, miR-6874-5p, miR-6892-5p, miR-15a-3p, miR-135b-5p, miR-520c-3p, miR-4783-5p, and miR-7849-3p.
(21) The extract of urine according to (20) above, wherein the microRNA is at least 1 or all microRNAs selected from the group consisting of miR-28-5 p, miR-105-5p, miR-124-3p, miR-151a-5p, and miR-300.
(22) The extract of urine according to (20) above, wherein the microRNA is at least 1 or all microRNAs selected from the group consisting of miR-15a-3p and miR-520c-3p.
(23) The extract of urine according to any of (20) to (22) above, wherein the urine is urine of a subject having prostate cancer.
(24) The extract of urine according to any one of (1) to (6), (8) to (10), (12) to (14), (16 to (18), and (20) to (22), wherein the urine is urine of a healthy person.
(25) A method of testing for the likelihood that a subject has cancer, the method comprising one or more selected from the group consisting of (a) to (e):
   (a) detecting at least one microRNA or all microRNAs selected from the group consisting of miR-3163, miR-16-1-3p, miR-424-3p, miR-558, miR-3127-5p, and miR-4521 in a urine or urine extract obtained from a subject, and if the amount of microRNA is greater than a predetermined value, indicating that the subject may have lung cancer;
   (b) detecting at least one or all microRNAs selected from the group consisting of miR-183-5p, miR-202-5p, and miR-409-5p in a urine or urine extract obtained from a subject, and if the amount of microRNA is greater than a predetermined value, indicating that the subject may have pancreatic cancer;
   (c) detecting at least one or all microRNAs selected from the group consisting of miR-16-1-3p, miR-28-5p, miR-297, miR-300, miR-330-3p, miR-454-5p, miR-1297, and miR-4295 in a urine or urine extract obtained from a subject, and if the amount of microRNA is greater than a predetermined value, indicating that the subject may have liver cancer;
   (d) detecting at least one or all microRNAs selected from the group consisting of miR-16-1-3p, miR-23b-3p, miR-28-5p, miR-142-3p, miR-195-3p, miR-299-3p, and miR-4295 in a urine or urine extract obtained from a subject, and if the amount of microRNA is greater than a predetermined value, indicating that the subject may have bladder cancer;
   (e) detecting at least one or all microRNAs selected from the group consisting of miR-28-5p, miR-105-5p, miR-124-3p, miR-151a-5p, and miR-300 in a urine or urine extract obtained from a subject, and if the amount of microRNA is greater than a predetermined value, indicating that the subject may have prostate cancer.
(26) The method according to (25) above, the method comprising 1 or more selected from the group consisting of (A) to (E):
   (A) further detecting at least one microRNA or all microRNAs selected from the group consisting of miR-378a-5p, miR-520c-3p, and miR-526b-3p in a urine or urine extract obtained from the subject in said (a), and if the amount of microRNA is smaller than a predetermined value, indicating that the subject may be lung cancer;
   (B) further detecting at least one or all microRNAs selected from the group consisting of miR-372-3p, miR-520b, miR-15a-3p, miR-34c-5p, miR-135a-5p, miR-185-5p, miR-337-3p, miR-422a, miR-506-3p, miR-520c-3p, miR-1284, miR-1323, and miR-4273 in a urine of urine extract obtained from the subject in said (b), and if the amount of microRNA is smaller than a predetermined value, indicating that the subject may have pancreatic cancer;
   (C) further detecting miR-520c-3p in a urine or urine extract obtained from the subject in said (c), and if the amount of microRNA is smaller than a predetermined value, indicating that the subject may have liver cancer;
   (D) further detecting miR-520c-3p in the urine or urine extract obtained from the subject in said (d) above, where the amount of microRNA is smaller than a predetermined value, indicating that the subject may have bladder cancer;
   (E) further detecting at least one or all of the microRNAs selected from the group consisting of miR-15a-3p and miR-520c-3p in the urine or urine extract obtained from the subject in said (e), and if the amount of the microRNA is smaller than a predetermined value, indicating that the subject may have prostate cancer.
(27) An extract of urine obtained by contacting a nanowire with urine and extracting a constituent bound to said nanowire.
   (27a) The extract of urine according to (27) above, wherein the pH of the urine to be contacted with the nanowire is a numerical range having a lower limit value of 2, 3, 4, or 5 and an upper limit value of 11, 10, 9, 8, 7, 6, or 5.
   (27b) The extract of urine according to (27) or (27a) above, wherein the nanowire is a nanowire of a metal oxide or a nanowire whose surface is coated with a metal oxide.
   (27c) An extract of urine according to (27b) above, wherein the metal oxide is an oxide of a transition metal.
   (27d) The extract of urine according to (27), (27a), (27b) or (27c) above, wherein the nanowire is a zinc oxide nanowire or a nanowire whose surface is coated with zinc oxide.
   (27e) The extract of urine according to (27), (27a), (27b), (27c), (27d) or (27e) above, wherein the nanowire and urine are contacted under a neutral pH condition.
(28) The extract of urine according to (27) above, comprising a microRNA listed in any of data S1 or Table 2
(29) The extract of urine according to (27), (27a), (27b), (27c), (27d) or (27e) above, comprising 20% or more of the extracellular vesicles contained in the urine.
   (29') The extract of urine according to (27), (27a), (27b), (27c), (27d) or (27e) above, comprising 25% or more of the extracellular vesicles contained in the urine.
   (29a) The extract of urine according to (27), (27a), (27b), (27c), (27d) or (27e) above, comprising 30% or more of the extracellular vesicles contained in the urine.
   (29a') The extract of urine according to (27), (27a), (27b), (27c), (27d) or (27e) above, comprising 35% or more of the extracellular vesicles contained in the urine.
   (29b) The extract of urine according to (27), (27a), (27b), (27c), (27d) or (27e) above, comprising 40% or more of the extracellular vesicles in the urine.
   (29b') The extract of urine according to (27), (27a), (27b), (27c), (27d) or (27e) above, comprising 45% or more of the extracellular vesicles contained in the urine.
   (29c) The extract of urine according to (27), (27a), (27b), (27c), (27d) or (27e) above, comprising 50% or more of the extracellular vesicles contained in the urine.
   (29c') The extract of urine according to (27), (27a), (27b), (27c), (27d) or (27e) above, comprising 55% or more of the extracellular vesicles contained in the urine.
   (29d) The extract of urine according to (27), (27a), (27b), (27c), (27d) or (27e) above, comprising at least 60% of the extracellular vesicles contained in the urine.
   (29d') The extract of urine according to (27), (27a), (27b), (27c), (27d) or (27e) above, comprising 65% or more of the extracellular vesicles contained in the urine.
   (29e) The extract of urine according to (27), (27a), (27b), (27c), (27d) or (27e) above, comprising 70% or more of the extracellular vesicles contained in the urine.
   (29e') The extract of urine according to (27), (27a), (27b), (27c), (27d) or (27e) above, comprising 75% or more of the extracellular vesicles contained in the urine.
   (29f) The extract of urine according to (27), (27a), (27b), (27c), (27d) or (27e) above, comprising 80% or more of the extracellular vesicles contained in the urine.
   (29f) The extract of urine according to (27), (27a), (27b), (27c), (27d) or (27e) above, comprising 85% or more of the extracellular vesicles contained in the urine.
   (29g) The extract of urine according to (27), (27a), (27b), (27c), (27d) or (27e) above, comprising 90% or more of the extracellular vesicles contained in the urine.
   (29h) The extract of urine according to (27), (27a), (27b), (27c), (27d) or (27e) above, comprising 95% or more of the extracellular vesicles contained in the urine.
   (29i) The extract of urine according to (27), (27a), (27b), (27c), (27d) or (27e) above, comprising 97% or more of the extracellular vesicles contained in the urine.
   (29j) An extract of urine according to (27), (27a), (27b), (27c), (27d) or (27e) above, comprising 99% or more of the extracellular vesicles contained in the urine.
(30) The extract of urine according to (1) above, comprising said microRNA concentrated.
(31) The extract of urine containing at least 500 types of microRNAs present in the urine.
   (31A) The extract of urine according to (1) or (31) above, comprising 500 or more types of microRNAs present in the urine.

According to the present disclosure, there are further provided industrially available inventions below.
(1) An extract of urine, comprising one or more microRNAs selected from the group consisting of: hsa-let-7a-3p, hsa-miR-103a-2-5p, hsa-miR-103a-3p, hsa-miR-105-3p, hsa-miR-106a-3p, hsa-miR-1180-3p, hsa-miR-1185-2-3p, hsa-miR-1193, hsa-miR-127-3p, hsa-miR-1245b-5p, hsa-miR-1247-3p, hsa-miR-125b-2-3p, hsa-miR-1263, hsa-miR-173g-3p, hsa-miR-129-2-3p, hsa-miR-1293, hsa-miR-1301-5p, hsa-miR-130a-3p, hsa-miR-130a-5p, hsa-miR-1469, hsa-miR-149-5p, hsa-miR-152-5p, hsa-miR-15b-3p, hsa-miR-16-1-3p, hsa-miR-181a-3p, hsa-miR-184, hsa-miR-186-5p, hsa-miR-18a-5p, hsa-miR-193b-3p, hsa-miR-200a-3p, hsa-miR-200c-5p, hsa-miR-208b-5p, hsa-miR-216a-3p, hsa-miR-7b-5p, hsa-miR-2861, hsa-miR-2909, hsa-miR-298, hsa-miR-29a-5p, hsa-miR-302b-3p, hsa-miR-30b-3p, hsa-miR-30d-5p, hsa-miR-3116, hsa-miR-3122, hsa-miR-3125, hsa-miR-3126-3p, hsa-miR-3129-3p, hsa-miR-3130-3p, hsa-miR-3133, hsa-miR-3136-3p, hsa-miR-3137, hsa-miR-3143, hsa-miR-3150a-3p, hsa-miR-3174, hsa-miR-3177-5p, hsa-miR-3179, hsa-miR-3180, hsa-miR-3186-3p, hsa-miR-3186-5p, hsa-miR-3187-3p, hsa-miR-3189-3p, hsa-miR-3190-3p, hsa-miR-3190-5p, hsa-miR-3194-5p, hsa-miR-3195, hsa-miR-3197, hsa-miR-3198, hsa-miR-3200-5p, hsa-miR-320a, hsa-miR-320c, hsa-miR-320d, hsa-miR-323b-5p, hsa-miR-324-3p, hsa-miR-325, hsa-miR-32-5p, hsa-miR-329-3p, hsa-miR-331-3p, hsa-miR-331-5p, hsa-miR-335-3p, hsa-miR-337-3p, hsa-miR-337-5p, hsa-miR-33a-3p, hsa-miR-33a-5p, hsa-miR-342-3p, hsa-miR-345-5p, hsa-miR-346, hsa-miR-34a-5p, hsa-miR-3606-3p, hsa-miR-3607-5p, hsa-miR-3613-3p, hsa-miR-3613-5p, hsa-miR-361-3p, hsa-miR-3616-3p, hsa-miR-3616-5p, hsa-miR-3621, hsa-miR-3622a-5p, hsa-miR-363-3p, hsa-miR-3658, hsa-miR-3659, hsa-miR-367-3p, hsa-miR-3674, hsa-miR-3678-3p, hsa-miR-3681-5p, hsa-miR-3683, hsa-miR-3684, hsa-miR-3690, hsa-miR-374c-5p, hsa-miR-376a-3p, hsa-miR-376a-5p, hsa-miR-376b-3p, hsa-miR-384, hsa-miR-3913-3p, hsa-miR-3940-3p, hsa-miR-3960, hsa-miR-3976, hsa-miR-4269, hsa-miR-477, hsa-miR-4302, hsa-miR-4303, hsa-miR-4304, hsa-miR-4307, hsa-miR-4316, hsa-miR-4319, hsa-miR-432-3p, hsa-miR-432-5p, hsa-miR-4326, hsa-miR-4421, hsa-miR-4433b-5p, hsa-miR-4456, hsa-miR-4472, hsa-miR-4479, hsa-miR-4521, hsa-miR-4639-5p, hsa-miR-4646-5p, hsa-miR-4660, hsa-miR-4662a-3p, hsa-miR-4688, hsa-miR-4694-3p, hsa-miR-4695-3p, hsa-miR-4695-5p, hsa-miR-4699-3p, hsa-miR-4701-5p, hsa-miR-4704-3p, hsa-miR-4716-5p, hsa-miR-4719, hsa-miR-4721, hsa-miR-4724-5p, hsa-miR-4736, hsa-miR-4738-3p, hsa-miR-4740-3p, hsa-miR-4750-5p, hsa-miR-4753-5p, hsa-miR-4759, hsa-miR-4776-5p, hsa-miR-4778-3p, hsa-miR-4793-5p, hsa-miR-4796-5p, hsa-miR-4799-3p, hsa-miR-4804-5p, hsa-miR-487b-5p, hsa-miR-497-5p, hsa-miR-5008-3p, hsa-miR-5011-5p, hsa-miR-509-5p, hsa-miR-514a-3p, hsa-miR-514b-5p, hsa-miR-515-3p, hsa-miR-518f-3p, hsa-miR-5194, hsa-miR-525-3p, hsa-miR-541-3p, hsa-miR-548aa, hsa-miR-548t-3p, hsa-miR-548ae-3p, hsa-miR-548c-3p, hsa-miR-548c-5p, hsa-miR-548o-5p, hsa-miR-548am-5p, hsa-miR-548f-5p, hsa-miR-548j-3p, hsa-miR-548n, hsa-miR-548v, hsa-miR-548x-3p, hsa-miR-548z, hsa-miR-548h-3p, hsa-miR-549a, hsa-miR-5571-5p, hsa-miR-5572, hsa-miR-5580-5p, hsa-miR-5586-3p, hsa-miR-5586-5p, hsa-miR-561-3p, hsa-miR-571, hsa-miR-574-3p, hsa-miR-578, hsa-miR-582-3p, hsa-miR-593-5p, hsa-miR-598-3p, hsa-miR-601, hsa-miR-606, hsa-miR-6068, hsa-miR-6077, hsa-miR-6083, hsa-miR-6089, hsa-miR-6090, hsa-miR-6132, hsa-miR-6133, hsa-miR-616-5p, hsa-miR-617, hsa-miR-620, hsa-miR-622, hsa-miR-623, hsa-miR-625-5p, hsa-miR-626, hsa-miR-636, hsa-miR-642b-5p, hsa-miR-649, hsa-miR-6504-5p, hsa-miR-6508-3p, hsa-miR-6511b-3p, hsa-miR-6513-5p, hsa-miR-651-5p, hsa-miR-6516-5p, hsa-miR-652-3p, hsa-miR-661, hsa-miR-664a-3p, hsa-miR-664a-5p, hsa-miR-665, hsa-miR-671-3p, hsa-miR-6715b-3p, hsa-miR-6720-5p, hsa-miR-6721-5p, hsa-miR-6726-3p, hsa-miR-6734-3p, hsa-miR-6735-5p, hsa-miR-6742-3p, hsa-miR-6755-3p, hsa-miR-6760-3p, hsa-miR-6767-5p, hsa-miR-6777-5p, hsa-miR-6782-5p, hsa-miR-6890-3p, hsa-miR-8084, hsa-miR-888-3p, hsa-miR-92b-5p, hsa-miR-935, hsa-miR-93-5p, and hsa-miR-942-3p.
(2) The extract of urine according to (1) above, comprising one or more microRNAs selected from the group consisting of:
   hsa-miR-103a-3p, hsa-miR-1193, hsa-miR-127-3p, hsa-miR-1247-3p, hsa-miR-1263, hsa-miR-173g-3p, hsa-miR-129-2-3p, hsa-miR-1293, hsa-miR-130a-5p, hsa-miR-1469, hsa-miR-15b-3p, hsa-miR-193b-3p, hsa-miR-2861, hsa-miR-298, hsa-miR-30d-5p, hsa-miR-3122, hsa-miR-3137, hsa-miR-3174, hsa-miR-3177-5p, hsa-miR-3186-3p, hsa-miR-3187-3p, hsa-miR-3189-3p, hsa-miR-3190-5p, hsa-miR-3194-5p, hsa-miR-3198, hsa-miR-320c, hsa-miR-329-3p, hsa-miR-337-3p, hsa-miR-337-5p, hsa-miR-33a-3p, hsa-miR-345-5p, hsa-miR-346, hsa-miR-34a-5p, hsa-miR-3613-3p, hsa-miR-361-3p, hsa-miR-3616-3p, hsa-miR-3622a-5p, hsa-miR-363-3p, hsa-miR-3659, hsa-miR-3678-3p, hsa-miR-3681-5p, hsa-miR-374c-5p, hsa-miR-3960, hsa-miR-3976, hsa-miR-4269, hsa-miR-4304, hsa-miR-4307, hsa-miR-432-3p, hsa-miR-4433b-5p, hsa-miR-4456, hsa-miR-4472, hsa-miR-4479, hsa-miR-4521, hsa-miR-4646-5p, hsa-miR-4694-3p, hsa-miR-4699-3p, hsa-miR-4701-5p, hsa-miR-4719, hsa-miR-4721, hsa-miR-4724-5p, hsa-miR-4776-5p, hsa-miR-4796-5p, hsa-miR-4804-5p, hsa-miR-5011-5p, hsa-miR-509-5p, hsa-miR-515-3p, hsa-miR-5571-5p, hsa-miR-5580-5p, hsa-miR-574-3p, hsa-miR-578, hsa-miR-582-3p, hsa-miR-598-3p, hsa-miR-6068, hsa-miR-6077, hsa-miR-6089, hsa-miR-6133, hsa-miR-616-5p, hsa-miR-622, hsa-miR-626, hsa-miR-636, hsa-miR-6504-5p, hsa-miR-6516-5p, hsa-miR-661, hsa-miR-664a-3p, hsa-miR-6715b-3p, hsa-miR-6720-5p, hsa-miR-6734-3p, hsa-miR-6742-3p, hsa-miR-6760-3p, hsa-miR-6767-5p, hsa-miR-6777-5p, hsa-miR-6890-3p, and hsa-miR-93-5p.
(3) The extract of urine according to (1) above, comprising one more microRNAs selected from the group consisting of:
   hsa-miR-103a-2-5p, hsa-miR-1193, hsa-miR-3622a-5p, hsa-miR-363-3p, hsa-miR-4521, hsa-miR-4796-5p, hsa-miR-518f-3p, hsa-miR-5580-5p, hsa-miR-3125, hsa-miR-3130-3p, hsa-miR-3678-3p, hsa-miR-4750-5p, hsa-miR-5194, hsa-miR-578, hsa-miR-625-5p, hsa-miR-6755-3p, hsa-miR-1293, hsa-miR-3137, hsa-miR-4646-5p, hsa-miR-514b-5p, hsa-miR-598-3p, hsa-miR-200c-5p, hsa-miR-3150a-3p, hsa-miR-3186-3p, hsa-miR-3187-3p, hsa-miR-3189-3p, hsa-miR-4303, hsa-miR-320a, hsa-miR-4421, hsa-miR-515-3p, hsa-miR-665, hsa-miR-6767-5p, hsa-miR-6782-5p, hsa-miR-3659, hsa-miR-3690, hsa-miR-4721, hsa-miR-4776-5p, hsa-miR-622, hsa-miR-2909, hsa-miR-3200-5p, hsa-miR-323b-5p, hsa-miR-34a-5p, hsa-miR-4688, hsa-miR-3190-3p, hsa-miR-3613-3p, hsa-miR-4793-5p, hsa-miR-6083, hsa-miR-4316, hsa-miR-4738-3p, hsa-miR-5572, hsa-miR-661, hsa-miR-3116, hsa-miR-3621, hsa-miR-4326, hsa-miR-623, hsa-miR-6504-5p, hsa-miR-1301-5p, hsa-miR-487b-5p, hsa-miR-3126-3p, hsa-miR-4304, hsa-miR-3186-5p, hsa-miR-342-3p, hsa-miR-4695-3p, hsa-miR-5008-3p, hsa-miR-616-5p, hsa-miR-125b-2-3p, hsa-miR-4479, hsa-miR-4740-3p, hsa-miR-103a-3p, hsa-miR-181a-3p, hsa-miR-337-5p, hsa-miR-1263, hsa-miR-374c-5p, hsa-miR-1180-3p, hsa-miR-29a-5p, hsa-miR-509-5p, hsa-miR-15b-3p, hsa-miR-32-5p, hsa-miR-376a-3p, hsa-miR-4804-5p, hsa-miR-5011-5p, hsa-miR-582-3p, hsa-miR-320d, hsa-miR-130a-3p, hsa-miR-200a-3p, hsa-miR-4639-5p, hsa-miR-571, hsa-miR-33a-5p, hsa-miR-4719, hsa-miR-3681-5p, hsa-miR-4699-3p, hsa-miR-626, hsa-miR-105-3p, hsa-miR-384, hsa-miR-3976, hsa-miR-186-5p, hsa-miR-18a-5p, hsa-miR-3616-5p, hsa-miR-4302, hsa-miR-548c-5p, hsa-miR-548o-5p, hsa-miR-548am-5p, hsa-miR-548v, hsa-let-7a-3p, hsa-miR-3143, hsa-miR-367-3p, hsa-miR-376a-5p, hsa-miR-548ae-3p, hsa-miR-5586-5p, hsa-miR-6715b-3p, hsa-miR-548aa, hsa-miR-548t-3p, hsa-miR-652-3p, hsa-miR-3607-5p, hsa-miR-3683, hsa-miR-477, hsa-miR-4704-3p, hsa-miR-4799-3p, hsa-miR-548x-3p, hsa-miR-549a, and hsa-miR-6508-3p.
(4) The extract of urine according to (1) above, comprising one or more microRNAs selected from the group consisting of:
   hsa-miR-103a-2-5p,hsa-miR-106a-3p,hsa-miR-1185-2-3p,hsa-miR-1193,hsa-miR-1273g-3 p,hsa-miR-1293,hsa-miR-1301-5p,hsa-miR-152-5p,hsa-miR-184,hsa-miR-200c-5p,hsa-miR -2909,hsa-miR-298,hsa-miR-302b-3p,hsa-miR-3116,hsa-miR-3122,hsa-miR-3125,hsa-miR-3126-3p,hsa-miR-3129-3p,hsa-miR-3130-3p,hsa-miR-3133,hsa-miR-3137,hsa-miR-3150a-3 p,hsa-miR-3174,hsa-miR-3177-5p,hsa-miR-3179,hsa-miR-3186-3p,hsa-miR-3186-5p,hsa-m iR-3187-3p,hsa-miR-3189-3p,hsa-miR-3190-3p,hsa-miR-3200-5p,hsa-miR-320a,hsa-miR-3 20c,hsa-miR-323b-5p,hsa-miR-325,hsa-miR-331-5p,hsa-miR-335-3p,hsa-miR-33a-3p,hsa-miR-342-3p,hsa-miR-34a-5p,hsa-miR-3606-3p,hsa-miR-3616-3p,hsa-miR-3621,hsa-miR-3 622a-5p,hsa-miR-3659,hsa-miR-3690,hsa-miR-376b-3p,hsa-miR-3913-3p,hsa-miR-4269,hsa -miR-4303,hsa-miR-4304,hsa-miR-4307,hsa-miR-4316,hsa-miR-4326,hsa-miR-4421,hsa-mi R-4472,hsa-miR-4479,hsa-miR-4646-5p,hsa-miR-4660,hsa-miR-4688,hsa-miR-4695-3p,hsa -miR-4695-5p,hsa-miR-4721,hsa-miR-4724-5p,hsa-miR-4738-3p,hsa-miR-4740-3p,hsa-mi R-4750-5p,hsa-miR-4753-5p,hsa-miR-4759,hsa-miR-4776-5p,hsa-miR-4778-3p,hsa-miR-4 796-5p,hsa-miR-487b-5p,hsa-miR-5008-3p,hsa-miR-514b-5p,hsa-miR-518f-3p,hsa-miR-51 94,hsa-miR-541-3p,hsa-miR-548c-3p,hsa-miR-548j-3p,hsa-miR-548n,hsa-miR-548z,hsa-mi R-548h-3p,hsa-miR-5572,hsa-miR-5580-5p,hsa-miR-5586-3p,hsa-miR-601,hsa-miR-6068,h sa-miR-6083,hsa-miR-6133,hsa-miR-617,hsa-miR-622,hsa-miR-625-5p,hsa-miR-649,hsa-m iR-6504-5p,hsa-miR-6513-5p,hsa-miR-651-5p,hsa-miR-6516-5p,hsa-miR-661,hsa-miR-664 a-5p,hsa-miR-665,hsa-miR-6755-3p,hsa-miR-6767-5p,hsa-miR-6777-5p,hsa-miR-6782-5p, hsa-miR-8084,hsa-miR-888-3p,and hsa-miR-942-3p.
(5) A method of predicting the likelihood that a subject has lung cancer, comprising:
   detecting in the urine of the subject one or more microRNAs selected from the group consisting of the below; and predicting the likelihood that the subject is cancerous using the expression level of the microRNA as an indicator: hsa-miR-4443, hsa-miR-4515, hsa-miR-4743-5p, hsa-miR-1908-3p, hsa-miR-4314, hsa-miR-296-3p, hsa-miR-6772-5p, hsa-miR-370-3p, hsa-miR-4708-3p, hsa-miR-4499, hsa-miR-6759-5p, hsa-miR-3160-3p, hsa-miR-219a-2-3p, hsa-miR-564, hsa-miR-4269, hsa-let-7b-5p, hsa-miR-4535, hsa-miR-187-3p, hsa-miR-5588-3p, hsa-miR-194-3p, hsa-miR-3153, hsa-miR-3074-5p, hsa-miR-4721, hsa-miR-173h-5p, hsa-miR-1471, hsa-miR-936, hsa-miR-622, hsa-miR-3622a-5p, hsa-miR-4252, hsa-miR-4533, hsa-miR-3917, hsa-miR-520d-5p, hsa-miR-4639-3p, hsa-miR-4496, hsa-miR-3156-5p, hsa-miR-3689b-3p, hsa-miR-3689c, hsa-miR-4669, hsa-miR-6072, hsa-miR-6751-5p, hsa-miR-378b, hsa-miR-520b, hsa-miR-1307-3p, hsa-miR-8075, hsa-miR-4448, hsa-miR-4487, hsa-miR-5002-3p, hsa-miR-4518, hsa-miR-4769-5p, hsa-miR-4724-5p, hsa-miR-397-5p, hsa-miR-494-5p, hsa-miR-6884-5p, hsa-miR-6796-5p, hsa-miR-181b-3p, hsa-miR-526b-5p, hsa-miR-1226-5p, hsa-miR-6776-5p, hsa-miR-4529-3p, hsa-miR-7851-3p, hsa-miR-3189-3p, hsa-miR-5010-5p, hsa-miR-4638-5p, hsa-miR-3177-3p, hsa-miR-6872-5p, hsa-miR-6745, hsa-miR-6871-5p, hsa-miR-138-1-3p, hsa-miR-4539, hsa-miR-3652, hsa-miR-505-5p, hsa-miR-173g-3p, hsa-miR-4701-3p, hsa-miR-3160-5p, hsa-miR-6815-5p, hsa-miR-6499-5p, hsa-miR-5589-5p, hsa-miR-4489, hsa-miR-212-5p, hsa-miR-5698, hsa-miR-3124-5p, hsa-miR-3176, hsa-miR-3612, hsa-miR-6849-5p, hsa-miR-6760-5p, hsa-miR-6833-5p, hsa-miR-8077, hsa-miR-277-3p, hsa-miR-7975, hsa-miR-6859-5p, hsa-miR-3187-5p, hsa-miR-3145-5p, hsa-miR-3689a-3p, hsa-miR-4800-5p, hsa-miR-1224-5p, hsa-miR-193a-5p, hsa-miR-6076, hsa-miR-4260, hsa-miR-298, hsa-miR-34a-5p, hsa-miR-6131, hsa-miR-5708, hsa-miR-4776-3p, hsa-miR-378e, hsa-miR-3659, hsa-miR-3120-5p, hsa-miR-3122, hsa-miR-3177-5p, hsa-miR-4420, hsa-miR-323a-5p, hsa-miR-466, hsa-miR-276-3p, hsa-miR-3138, hsa-miR-6834-5p, hsa-miR-185-5p, hsa-miR-2467-3p, hsa-miR-6847-5p, hsa-miR-608, hsa-miR-6504-5p, hsa-miR-3922-5p, hsa-miR-877-5p, hsa-miR-4307, hsa-miR-4512, hsa-miR-6892-5p, hsa-miR-4776-5p, hsa-miR-3065-5p, hsa-miR-6129, hsa-miR-4676-5p, hsa-miR-603, hsa-miR-6876-5p, hsa-miR-4635, hsa-miR-4525, hsa-miR-1468-5p, hsa-miR-519c-5p, hsa-miR-523-5p, hsa-miR-518e-5p, hsa-miR-522-5p, hsa-miR-519a-5p, hsa-miR-519b-5p, hsa-miR-4711-3p, hsa-miR-1293, hsa-miR-6068, hsa-miR-1254, hsa-miR-4306, hsa-miR-3186-3p, hsa-miR-4691-5p, hsa-miR-1321, hsa-miR-4540, hsa-miR-6823-5p, hsa-miR-3935, hsa-miR-5006-5p, hsa-miR-4756-3p, hsa-miR-4785, hsa-miR-8082, hsa-miR-6788-5p, hsa-miR-6500-3p, hsa-miR-5189-5p, hsa-miR-7151-3p, hsa-miR-891a-5p, hsa-miR-3126-5p, hsa-miR-8083, hsa-miR-4428, hsa-miR-4474-3p, hsa-miR-7850-5p, hsa-miR-4700-5p, hsa-miR-6828-5p, hsa-miR-668-5p, hsa-miR-3934-5p, hsa-miR-4654, hsa-miR-4436a, hsa-miR-173f, hsa-miR-3655, hsa-miR-650, hsa-miR-3137, hsa-miR-4285, hsa-miR-171-5p, hsa-miR-3064-3p, hsa-miR-6801-5p, hsa-miR-6817-5p, hsa-miR-4686, hsa-miR-173g-5p, hsa-miR-6807-3p, hsa-miR-6747-5p, hsa-miR-5580-5p, hsa-miR-4796-5p, hsa-miR-3174, hsa-miR-7157-5p, hsa-miR-614, hsa-miR-4502, hsa-miR-3164, hsa-miR-4647, hsa-miR-378i, hsa-miR-6515-5p, hsa-miR-656-5p, hsa-miR-449c-5p, hsa-miR-3591-3p, hsa-miR-424-3p, hsa-miR-3149, hsa-miR-6868-5p, hsa-miR-173d, hsa-miR-4644, hsa-miR-4754, hsa-miR-477-3p, hsa-miR-4445-3p, hsa-miR-4436b-3p, hsa-miR-4262, hsa-miR-1306-3p, hsa-miR-4514, hsa-miR-4296, hsa-miR-4458, hsa-miR-4520-5p, hsa-miR-766-5p, hsa-miR-548ao-3p, hsa-miR-758-5p, hsa-miR-4451, hsa-miR-4300, hsa-miR-4453, hsa-miR-4784, hsa-miR-6809-5p, hsa-miR-4692, hsa-miR-4437, hsa-miR-4681, hsa-miR-921, hsa-miR-1250-5p, hsa-miR-5093, hsa-miR-551b-5p, hsa-miR-4529-5p, hsa-miR-4538, hsa-miR-176, hsa-miR-1322, hsa-miR-473, hsa-miR-106a-5p, hsa-miR-297, hsa-miR-1910-3p, hsa-miR-4470, hsa-miR-3691-5p, hsa-miR-4999-5p, hsa-miR-3192-5p, hsa-miR-548au-3p, hsa-miR-5007-5p, hsa-miR-4657, hsa-miR-4768-3p, hsa-miR-1265, hsa-miR-4321, hsa-miR-4423-5p, hsa-miR-1294, and hsa-miR-4520-3p.
   (5A) The method according to (4) above, wherein the miRNA to be detected is one or more microRNAs selected from the group consisting of: hsa-miR-6788-5p, hsa-miR-6500-3p, hsa-miR-5189-5p, hsa-miR-7151-3p, hsa-miR-891a-5p, hsa-miR-3126-5p, hsa-miR-8083, hsa-miR-4428, hsa-miR-4474-3p, hsa-miR-7850-5p, hsa-miR-4700-5p, hsa-miR-6828-5p, hsa-miR-668-5p, hsa-miR-3934-5p, hsa-miR-4654, hsa-miR-4436a, hsa-miR-173f, hsa-miR-3655, hsa-miR-650, hsa-miR-3137, hsa-miR-4285, hsa-miR-171-5p, hsa-miR-3064-3p, hsa-miR-6801-5p, hsa-miR-6817-5p, hsa-miR-4686, hsa-miR-173g-5p, hsa-miR-6807-3p, hsa-miR-6747-5p, hsa-miR-5580-5p, hsa-miR-4796-5p, hsa-miR-3174, hsa-miR-7157-5p, hsa-miR-614, hsa-miR-4502, hsa-miR-3164, hsa-miR-4647, hsa-miR-378i, hsa-miR-6515-5p, hsa-miR-656-5p, hsa-miR-449c-5p, hsa-miR-3591-3p, hsa-miR-424-3p, hsa-miR-3149, hsa-miR-6868-5p, hsa-miR-173d, hsa-miR-4644, hsa-miR-4754, hsa-miR-477-3p, hsa-miR-4445-3p, hsa-miR-4436b-3p, hsa-miR-4262, hsa-miR-1306-3p, hsa-miR-4514, hsa-miR-4296, hsa-miR-4458, hsa-miR-4520-5p, hsa-miR-766-5p, hsa-miR-548ao-3p, hsa-miR-758-5p, hsa-miR-4451, hsa-miR-4300, hsa-miR-4453, hsa-miR-4784, hsa-miR-6809-5p, hsa-miR-4692, hsa-miR-4437, hsa-miR-4681, hsa-miR-921, hsa-miR-1250-5p, hsa-miR-5093, hsa-miR-551b-5p, hsa-miR-4529-5p, hsa-miR-4538, hsa-miR-176, hsa-miR-1322, hsa-miR-473, hsa-miR-106a-5p, hsa-miR-297, hsa-miR-1910-3p, hsa-miR-4470, hsa-miR-3691-5p, hsa-miR-4999-5p, hsa-miR-3192-5p, hsa-miR-548au-3p, hsa-miR-5007-5p, hsa-miR-4657, hsa-miR-4768-3p, hsa-miR-1265, hsa-miR-4321, hsa-miR-4423-5p, hsa-miR-1294, and hsa-miR-4520-3p.
   (5B) The method according to (4) above, wherein the miRNA to be detected is one or more microRNAs selected from the group consisting of: hsa-miR-4644, hsa-miR-4754, hsa-miR-477-3p, hsa-miR-4445-3p, hsa-miR-4436b-3p, hsa-miR-4262, hsa-miR-1306-3p, hsa-miR-4514, hsa-miR-4296, hsa-miR-4458, hsa-miR-4520-5p, hsa-miR-766-5p, hsa-miR-548ao-3p, hsa-miR-758-5p, hsa-miR-4451, hsa-miR-4300, hsa-miR-4453, hsa-miR-4784, hsa-miR-6809-5p, hsa-miR-4692, hsa-miR-4437, hsa-miR-4681, hsa-miR-921, hsa-miR-1250-5p, hsa-miR-5093, hsa-miR-551b-5p, hsa-miR-4529-5p, hsa-miR-4538, hsa-miR-176, hsa-miR-1322, hsa-miR-473, hsa-miR-106a-5p, hsa-miR-297, hsa-miR-1910-3p, hsa-miR-4470, hsa-miR-3691-5p, hsa-miR-4999-5p, hsa-miR-3192-5p, hsa-miR-548au-3p, hsa-miR-5007-5p, hsa-miR-4657, hsa-miR-4768-3p, hsa-miR-1265, hsa-miR-4321, hsa-miR-4423-5p, hsa-miR-1294, and hsa-miR-4520-3p.
   (5C) The method of (4) above, wherein the miRNA to be detected is one or more microRNAs selected from the group consisting of: hsa-miR-4768-3p, hsa-miR-1265, hsa-miR-4321, hsa-miR-4423-5p, hsa-miR-1294, and hsa-miR-4520-3p.
   (5D) The method according to any one of (5), (5A), (5B), and (5C) above, wherein the miRNA to be detected are 5 or more microRNAs selected from the group consisting of the below.
   (5E) The method according to any one of (5), (5A), (5B), and (5C) above, wherein the miRNA to be detected are 10 or more microRNAs selected from the group consisting of the below.
   (5F) The method according to any one of (5), (5A), (5B), and (5C) above, wherein the miRNA to be detected are 15 or more microRNAs selected from the group consisting of the below.
   (5G) The method according to any one of (5), (5A), (5B), and (5C) above, wherein the miRNA to be detected are 20 or more microRNAs selected from the group consisting of the below.
   (5H) The method according to any one of the above (5D) to (5G), wherein the predictive accuracy and specificity exceed 50%.
   (5I) The method according to any one of the above (5D) to (5G), wherein the predictive accuracy and specificity exceed 60%.
   (5J) The method according to any one of the above (5D) to (5G), wherein the predictive accuracy and specificity exceed 70%.
   (5K) The method according to any one of the above (5D) to (5G), wherein the predictive accuracy and specificity exceed 80%.
(6) A method of predicting the likelihood that a subject has lung cancer, the method comprising detecting in the urine of the subject one or more microRNAs selected from the group consisting of the below and predicting the likelihood that the subject has cancer using the expressions level of the microRNAs as an indicator: hsa-miR-147b, hsa-miR-3912-5p, hsa-miR-148a-5p, hsa-miR-6773-5p, hsa-miR-3681-5p, hsa-miR-3976, hsa-miR-3121-5p, hsa-miR-6082, hsa-miR-106b-5p, hsa-miR-758-3p, hsa-miR-4418, hsa-miR-216a-5p, hsa-miR-34a-3p, hsa-miR-516b-5p, hsa-miR-140-5p, hsa-miR-7154-5p, hsa-miR-616-5p, hsa-miR-5701, hsa-miR-582-3p, hsa-miR-626, hsa-miR-3653-3p, hsa-miR-4320, hsa-miR-3689a-5p, hsa-miR-3689b-5p, hsa-miR-3689e, hsa-miR-8058, hsa-miR-107, hsa-miR-10a-5p, hsa-miR-624-3p, hsa-miR-4683, hsa-miR-30a-5p, hsa-miR-222-3p, hsa-miR-580-5p, hsa-miR-5581-5p, hsa-miR-200c-3p, hsa-miR-2052, hsa-miR-5089-5p, hsa-miR-17-5p, hsa-miR-452-3p, hsa-miR-329-3p, hsa-miR-5092, hsa-miR-585-3p, hsa-miR-4503, hsa-miR-5011-5p, hsa-miR-372-5p, hsa-miR-5589-3p, hsa-miR-2114-5p, hsa-miR-4799-5p, hsa-miR-4422, hsa-miR-520d-3p, hsa-miR-6864-5p, hsa-miR-182-5p, hsa-miR-363-3p, hsa-miR-215-3p, hsa-miR-2681-3p, hsa-miR-922, hsa-miR-450a-2-3p, hsa-miR-520f-3p, hsa-miR-4501, hsa-miR-660-5p, hsa-miR-1911-5p, hsa-miR-20a-3p, hsa-miR-362-5p, hsa-miR-380-5p, hsa-miR-597-3p, hsa-miR-448, hsa-miR-3182, hsa-miR-99a-3p, hsa-miR-616-3p, hsa-miR-22-5p, hsa-miR-3591-5p, hsa-miR-4293, hsa-miR-433-3p, hsa-miR-1183, hsa-miR-662, hsa-miR-195-5p, hsa-miR-96-5p, hsa-miR-630, hsa-miR-7a-3p, hsa-miR-30e-3p, hsa-miR-361-5p, hsa-miR-6715b-3p, hsa-miR-563, hsa-miR-1263, hsa-miR-16-5p, hsa-miR-425-5p, hsa-miR-519a-3p, hsa-miR-655-5p, hsa-miR-429, hsa-miR-576-5p, hsa-miR-517-5p, hsa-miR-659-5p, hsa-miR-375, hsa-miR-4719, hsa-miR-891a-3p, hsa-miR-653-5p, hsa-miR-598-3p, hsa-miR-181a-5p, hsa-miR-15b-3p, hsa-miR-431-5p, hsa-miR-143-5p, hsa-miR-3157-5p, hsa-miR-181d-5p, hsa-miR-3978, hsa-miR-539-5p, hsa-let-7e-5p, hsa-miR-938, hsa-miR-369-5p, hsa-miR-656-3p, hsa-miR-132-3p, hsa-miR-23a-3p, hsa-miR-4699-3p, hsa-miR-624-5p, hsa-miR-7843-3p, hsa-miR-4524a-3p, hsa-miR-611, hsa-miR-5002-5p, hsa-miR-1286, hsa-miR-3144-3p, hsa-miR-4650-3p, hsa-miR-7162-5p, hsa-miR-548ba, hsa-miR-221-3p, hsa-miR-628-3p, hsa-miR-6715a-3p, hsa-miR-1297, hsa-miR-3907, hsa-miR-23b-5p, hsa-miR-374b-5p, hsa-miR-371a-3p, hsa-miR-372-3p, hsa-miR-7158-3p, hsa-miR-10b-5p, hsa-miR-554, hsa-miR-4325, hsa-miR-196a-5p, hsa-miR-5702, hsa-miR-519b-3p, hsa-miR-542-5p, hsa-miR-1298-5p, hsa-miR-20b-5p, hsa-miR-4661-5p, hsa-miR-172, hsa-miR-708-5p, hsa-miR-509-5p, hsa-miR-450a-5p, hsa-miR-7153-5p, hsa-miR-135a-5p, hsa-miR-4752, hsa-miR-578, hsa-miR-492, hsa-miR-93-3p, hsa-miR-1180-5p, hsa-miR-500a-5p, hsa-miR-181d-3p, hsa-miR-516b-3p, hsa-miR-516a-3p, hsa-miR-489-3p, hsa-miR-21-3p, hsa-miR-96-3p, hsa-miR-383-3p, hsa-miR-3921, hsa-miR-4762-5p, hsa-miR-1912, hsa-miR-3613-3p, hsa-miR-203b-5p, hsa-miR-135b-5p, hsa-miR-591, hsa-miR-521, hsa-miR-631, hsa-miR-4677-5p, hsa-miR-4804-5p, hsa-miR-5571-3p, hsa-miR-122-3p, hsa-miR-30c-5p, hsa-miR-6837-3p, hsa-miR-145-5p, hsa-miR-4643, hsa-miR-4522, hsa-miR-5588-5p, hsa-miR-888-5p, hsa-miR-4781-5p, hsa-miR-520a-3p, hsa-miR-28-5p, hsa-miR-580-3p, hsa-miR-4774-3p, hsa-miR-512-3p, hsa-miR-605-3p, hsa-miR-511-5p, hsa-miR-6768-3p, hsa-miR-552-5p, hsa-miR-491-3p, hsa-miR-173a, hsa-miR-635, hsa-let-7g-3p, hsa-miR-378a-5p, hsa-miR-200b-3p, hsa-miR-337-5p, hsa-miR-4694-3p, hsa-miR-3163, hsa-let-7f-2-3p, hsa-miR-4659a-3p, hsa-miR-5696, hsa-miR-103a-3p, hsa-miR-376c-3p, hsa-miR-4650-5p, hsa-miR-892c-3p, hsa-miR-203a-3p, hsa-miR-8055, hsa-miR-5004-3p, hsa-miR-339-5p, hsa-miR-7156-5p, hsa-miR-345-5p, hsa-miR-526b-3p, hsa-miR-16-2-3p, hsa-miR-552-3p, hsa-miR-340-3p, hsa-miR-3193, hsa-miR-517a-3p, hsa-miR-517b-3p, hsa-miR-1257, hsa-miR-3529-5p, hsa-miR-29a-3p, hsa-miR-566, hsa-miR-1205, hsa-miR-3622b-3p, hsa-miR-1-5p, hsa-miR-5187-3p, hsa-miR-130a-5p, hsa-miR-15a-3p, hsa-miR-146a-5p, hsa-miR-337-3p, hsa-miR-4691-3p, hsa-miR-205-5p, hsa-miR-374c-5p, hsa-miR-374c-3p, hsa-miR-551b-3p, hsa-miR-4694-5p, hsa-miR-276-5p, hsa-miR-520g-3p, hsa-miR-519e-3p, hsa-miR-299-5p, hsa-miR-25-3p, hsa-miR-8079, hsa-miR-6510-3p, hsa-miR-223-5p, hsa-miR-216b-3p, hsa-miR-99b-5p, hsa-miR-1289, hsa-miR-8086, hsa-miR-195-3p, hsa-miR-7853-5p, hsa-miR-4794, hsa-miR-1911-3p, hsa-miR-6811-3p, hsa-miR-605-5p, hsa-miR-4704-5p, hsa-miR-204-5p, hsa-miR-302d-5p, hsa-miR-4266, hsa-miR-519d-3p, hsa-miR-5684, hsa-miR-4755-5p, hsa-miR-589-5p, hsa-miR-125b-5p, hsa-miR-1291, hsa-miR-1200, hsa-miR-4724-3p, hsa-miR-93-5p, hsa-miR-942-5p, hsa-miR-432-3p, hsa-miR-7-2-3p, hsa-miR-642a-5p, hsa-miR-370-5p, hsa-miR-8074, hsa-miR-512-5p, hsa-miR-506-3p, hsa-miR-6774-3p, hsa-miR-1269b, hsa-miR-150-5p, hsa-miR-3155a, hsa-miR-208a-5p, hsa-miR-501-5p, hsa-miR-30c-1-3p, hsa-miR-5571-5p, hsa-miR-6758-3p, hsa-miR-1184, hsa-miR-454-5p, hsa-miR-34b-5p, hsa-miR-6838-5p, hsa-miR-92b-3p, hsa-miR-4652-3p, hsa-miR-4747-3p, hsa-miR-1255b-2-3p, hsa-miR-1282, hsa-miR-173h-3p, hsa-miR-7703, hsa-miR-3919, hsa-miR-324-5p, hsa-miR-1251-3p, hsa-miR-671-5p, hsa-miR-6508-5p, hsa-miR-23b-3p, hsa-miR-3184-5p, hsa-miR-6744-3p, hsa-miR-642a-3p, hsa-miR-98-3p, hsa-miR-4288, hsa-miR-1203, hsa-miR-3181, hsa-miR-6500-5p, hsa-miR-380-3p, hsa-miR-574-5p, hsa-miR-4328, hsa-miR-5584-3p, hsa-miR-6781-3p, hsa-miR-632, hsa-miR-193b-3p, and hsa-miR-6841-3p.
   (6A) The method according to (6) above, wherein the miRNA to be detected is one or more microRNAs selected from the group consisting of: hsa-miR-147b, hsa-miR-3912-5p, hsa-miR-148a-5p, hsa-miR-6773-5p, hsa-miR-3681-5p, hsa-miR-3976, hsa-miR-3121-5p, hsa-miR-6082, hsa-miR-106b-5p, hsa-miR-758-3p, hsa-miR-4418, hsa-miR-216a-5p, hsa-miR-34a-3p, hsa-miR-516b-5p, hsa-miR-140-5p, hsa-miR-7154-5p, hsa-miR-616-5p, hsa-miR-5701, hsa-miR-582-3p, hsa-miR-626, hsa-miR-3653-3p, hsa-miR-4320, hsa-miR-3689a-5p, hsa-miR-3689b-5p, hsa-miR-3689e, hsa-miR-8058, hsa-miR-107, hsa-miR-10a-5p, hsa-miR-624-3p, hsa-miR-4683, hsa-miR-30a-5p, hsa-miR-222-3p, hsa-miR-580-5p, hsa-miR-5581-5p, hsa-miR-200c-3p, hsa-miR-2052, hsa-miR-5089-5p, hsa-miR-17-5p, hsa-miR-452-3p, hsa-miR-329-3p, hsa-miR-5092, hsa-miR-585-3p, hsa-miR-4503, hsa-miR-5011-5p, hsa-miR-372-5p, hsa-miR-5589-3p, hsa-miR-2114-5p, hsa-miR-4799-5p, hsa-miR-4422, hsa-miR-520d-3p, hsa-miR-6864-5p, hsa-miR-182-5p, hsa-miR-363-3p, hsa-miR-215-3p, hsa-miR-2681-3p, hsa-miR-922, hsa-miR-450a-2-3p, hsa-miR-520f-3p, hsa-miR-4501, hsa-miR-660-5p, hsa-miR-1911-5p, hsa-miR-20a-3p, hsa-miR-362-5p, hsa-miR-380-5p, hsa-miR-597-3p, hsa-miR-448, hsa-miR-3182, hsa-miR-99a-3p, hsa-miR-616-3p, hsa-miR-22-5p, hsa-miR-3591-5p, hsa-miR-4293, hsa-miR-433-3p, hsa-miR-1183, hsa-miR-662, hsa-miR-195-5p, hsa-miR-96-5p, hsa-miR-630, hsa-miR-7a-3p, hsa-miR-30e-3p, hsa-miR-361-5p, hsa-miR-6715b-3p, hsa-miR-563, hsa-miR-1263, hsa-miR-16-5p, hsa-miR-425-5p, hsa-miR-519a-3p, hsa-miR-655-5p, hsa-miR-429, hsa-miR-576-5p, hsa-miR-517-5p, hsa-miR-659-5p, hsa-miR-375, hsa-miR-4719, hsa-miR-891a-3p, hsa-miR-653-5p, hsa-miR-598-3p, hsa-miR-181a-5p, hsa-miR-15b-3p, hsa-miR-431-5p, hsa-miR-143-5p, hsa-miR-3157-5p, hsa-miR-181d-5p, hsa-miR-3978, hsa-miR-539-5p, hsa-let-7e-5p, hsa-miR-938, hsa-miR-369-5p, hsa-miR-656-3p, hsa-miR-132-3p, hsa-miR-23a-3p, hsa-miR-4699-3p, hsa-miR-624-5p, hsa-miR-7843-3p, hsa-miR-4524a-3p, hsa-miR-611, hsa-miR-5002-5p, hsa-miR-1286, hsa-miR-3144-3p, hsa-miR-4650-3p, hsa-miR-7162-5p, hsa-miR-548ba, hsa-miR-221-3p, hsa-miR-628-3p, hsa-miR-6715a-3p, hsa-miR-1297, hsa-miR-3907, hsa-miR-23b-5p, hsa-miR-374b-5p, hsa-miR-371a-3p, hsa-miR-372-3p, hsa-miR-7158-3p, hsa-miR-10b-5p, hsa-miR-554, hsa-miR-4325, hsa-miR-196a-5p, hsa-miR-5702, hsa-miR-519b-3p, hsa-miR-542-5p, hsa-miR-1298-5p, hsa-miR-20b-5p, hsa-miR-4661-5p, hsa-miR-172, hsa-miR-708-5p, hsa-miR-509-5p, hsa-miR-450a-5p, hsa-miR-7153-5p, hsa-miR-135a-5p, hsa-miR-4752, hsa-miR-578, hsa-miR-492, hsa-miR-93-3p, hsa-miR-1180-5p, hsa-miR-500a-5p, hsa-miR-181d-3p, hsa-miR-516b-3p, hsa-miR-516a-3p, hsa-miR-489-3p, hsa-miR-21-3p, hsa-miR-96-3p, hsa-miR-383-3p, hsa-miR-3921, hsa-miR-4762-5p, hsa-miR-1912, hsa-miR-3613-3p, hsa-miR-203b-5p, hsa-miR-135b-5p, hsa-miR-591, hsa-miR-521, hsa-miR-631, hsa-miR-4677-5p, hsa-miR-4804-5p, hsa-miR-5571-3p, hsa-miR-122-3p, and hsa-miR-30c-5p.
   (6B) The method according to (6) above, wherein the miRNA to be detected is one or more microRNAs selected from the group consisting of: hsa-miR-147b, hsa-miR-3912-5p, hsa-miR-148a-5p, hsa-miR-6773-5p, hsa-miR-3681-5p, hsa-miR-3976, hsa-miR-3121-5p, hsa-miR-6082, hsa-miR-106b-5p, hsa-miR-758-3p, hsa-miR-4418, hsa-miR-216a-5p, hsa-miR-34a-3p, hsa-miR-516b-5p, hsa-miR-140-5p, hsa-miR-7154-5p, hsa-miR-616-5p, hsa-miR-5701, hsa-miR-582-3p, hsa-miR-626, hsa-miR-3653-3p, hsa-miR-4320, hsa-miR-3689a-5p, hsa-miR-3689b-5p, hsa-miR-3689e, hsa-miR-8058, hsa-miR-107, hsa-miR-10a-5p, hsa-miR-624-3p, hsa-miR-4683, hsa-miR-30a-5p, hsa-miR-222-3p, hsa-miR-580-5p, hsa-miR-5581-5p, hsa-miR-200c-3p, hsa-miR-2052, hsa-miR-5089-5p, hsa-miR-17-5p, hsa-miR-452-3p, hsa-miR-329-3p, hsa-miR-5092, hsa-miR-585-3p, hsa-miR-4503, hsa-miR-5011-5p, hsa-miR-372-5p, hsa-miR-5589-3p, hsa-miR-2114-5p, hsa-miR-4799-5p, hsa-miR-4422, hsa-miR-520d-3p, hsa-miR-6864-5p, hsa-miR-182-5p, hsa-miR-363-3p, hsa-miR-215-3p, hsa-miR-2681-3p, hsa-miR-922, hsa-miR-450a-2-3p, hsa-miR-520f-3p, hsa-miR-4501, hsa-miR-660-5p, hsa-miR-1911-5p, hsa-miR-20a-3p, hsa-miR-362-5p, hsa-miR-380-5p, hsa-miR-597-3p, hsa-miR-448, hsa-miR-3182, hsa-miR-99a-3p, hsa-miR-616-3p, hsa-miR-22-5p, hsa-miR-3591-5p, hsa-miR-4293, hsa-miR-433-3p, hsa-miR-1183, hsa-miR-662, hsa-miR-195-5p, hsa-miR-96-5p, hsa-miR-630, hsa-miR-7a-3p, hsa-miR-30e-3p, hsa-miR-361-5p, hsa-miR-6715b-3p, hsa-miR-563, hsa-miR-1263, hsa-miR-16-5p, hsa-miR-425-5p, hsa-miR-519a-3p, hsa-miR-655-5p, hsa-miR-429, hsa-miR-576-5p, hsa-miR-517-5p, hsa-miR-659-5p, hsa-miR-375, hsa-miR-4719, hsa-miR-891a-3p, hsa-miR-653-5p, hsa-miR-598-3p, hsa-miR-181a-5p, hsa-miR-15b-3p, hsa-miR-431-5p, hsa-miR-143-5p, hsa-miR-3157-5p, hsa-miR-181d-5p, hsa-miR-3978, hsa-miR-539-5p, and hsa-let-7e-5p.
   (6C) The method according to (6) above, wherein the miRNA to be detected is one or more microRNAs selected from the group consisting of: hsa-miR-147b, hsa-miR-3912-5p, hsa-miR-148a-5p, hsa-miR-6773-5p, hsa-miR-3681-5p, hsa-miR-3976, hsa-miR-3121-5p, hsa-miR-6082, hsa-miR-106b-5p, hsa-miR-758-3p, hsa-miR-4418, hsa-miR-216a-5p, hsa-miR-34a-3p, hsa-miR-516b-5p, hsa-miR-140-5p, hsa-miR-7154-5p, hsa-miR-616-5p, hsa-miR-5701, hsa-miR-582-3p, hsa-miR-626, hsa-miR-3653-3p, hsa-miR-4320, hsa-miR-3689a-5p, hsa-miR-3689b-5p, hsa-miR-3689e, hsa-miR-8058, hsa-miR-107, hsa-miR-10a-5p, hsa-miR-624-3p, hsa-miR-4683, hsa-miR-30a-5p, hsa-miR-222-3p, hsa-miR-580-5p, hsa-miR-5581-5p, hsa-miR-200c-3p, hsa-miR-2052, hsa-miR-5089-5p, hsa-miR-17-5p, and hsa-miR-452-3p.
   (6D) The method according to any one of (6), (6A), (6B), and (6C) above, wherein the miRNA to be detected are 5 or more microRNAs selected from the group consisting of the below.
   (6E) The method according to any one of (6), (6A), (6B), and (6C) above, wherein the miRNA to be detected are 10 or more microRNAs selected from the group consisting of the below.
   (6F) The method according to any one of (6), (6A), (6B), and (6C) above, wherein the miRNA to be detected are 15 or more microRNAs selected from the group consisting of the below.
   (6G) The method according to any one of (6), (6A), (6B), and (6C) above, wherein the miRNA to be detected are 20 or more microRNAs selected from the group consisting of the below.
   (6H) The method of any of the above (6D) to (6G), wherein any or all of the predictive accuracy, sensitivity, and specificity exceeds 50%.
   (6I) The method according to any one of the above (6D) to (6G), wherein any or all of the predictive accuracy, sensitivity, and specificity exceeds 60%.
   (6J) The method according to any of the above (6D) to (6G), wherein any or all of the predictive accuracy, sensitivity, and specificity exceeds 70%.
   (6K) The method according to any one of the above (6D) to (6G), wherein any or all of the predictive accuracy, sensitivity, and specificity exceeds 80%.
   (6L) The method according to any of (6D) to (6K), wherein the area under the curve (AUC) of the receiver operating property curve (ROC) is greater than 0.5, greater than 0.6, greater than 0.7, greater than 0.8, greater than 0.9, or greater than 0.95.
   (6M) The method according to (6L) above, wherein it is predicted whether or not the subject has a likelihood of lung cancer, based on a cut-off value of true positive rate > false positive rate.
(7) A method of predicting a likelihood that a subject has lung cancer, comprising detecting at least one, two, three, four, five, six, seven, eight, nine, ten or more, fifteen or more, or twenty or more, or all microRNAs selected from the group consisting of the below in the subject urine: hsa-let-7a-3p, hsa-1et-7g-5p, hsa-miR-100-3p, hsa-miR-101-3p, hsa-miR-105-3p, hsa-miR-10b-3p, hsa-miR-1185-5p, hsa-miR-1197, hsa-miR-1206, hsa-miR-1243, hsa-miR-1245b-3p, hsa-miR-1246, hsa-miR-1252-3p, hsa-miR-1252-5p, hsa-miR-1258, hsa-miR-126-3p, hsa-miR-126-5p, hsa-miR-1277-3p, hsa-miR-1277-5p, hsa-miR-1279, hsa-miR-1295b-5p, hsa-miR-136-5p, hsa-miR-1-3p, hsa-miR-145-3p, hsa-miR-1468-3p, hsa-miR-152-3p, hsa-miR-153-3p, hsa-miR-155-5p, hsa-miR-186-5p, hsa-miR-18a-5p, hsa-miR-190a-5p, hsa-miR-190b, hsa-miR-19a-5p, hsa-miR-19b-1-5p, hsa-miR-19b-2-5p, hsa-miR-19b-3p, hsa-miR-2053, hsa-miR-205-3p, hsa-miR-2054, hsa-miR-208a-3p, hsa-miR-20a-5p, hsa-miR-219a-1-3p, hsa-miR-219b-3p, hsa-miR-301a-3p, hsa-miR-30d-3p, hsa-miR-30e-5p, hsa-miR-3118, hsa-miR-3123, hsa-miR-3129-5p, hsa-miR-3135a, hsa-miR-3140-3p, hsa-miR-3143, hsa-miR-3152-3p, hsa-miR-3167, hsa-miR-3169, hsa-miR-3183, hsa-miR-3201, hsa-miR-335-5p, hsa-miR-339-3p, hsa-miR-3607-5p, hsa-miR-3616-5p, hsa-miR-3617-5p, hsa-miR-3618, hsa-miR-3662, hsa-miR-3668, hsa-miR-3683, hsa-miR-3686, hsa-miR-3688-3p, hsa-miR-369-3p, hsa-miR-374a-3p, hsa-miR-374a-5p, hsa-miR-376a-5p, hsa-miR-3927-3p, hsa-miR-3942-5p, hsa-miR-4277, hsa-miR-4302, hsa-miR-4432, hsa-miR-4452, hsa-miR-4457, hsa-miR-4474-5p, hsa-miR-4477b, hsa-miR-4480, hsa-miR-4495, hsa-miR-4504, hsa-miR-4509, hsa-miR-450a-1-3p, hsa-miR-450b-3p, hsa-miR-455-5p, hsa-miR-4633-3p, hsa-miR-4639-5p, hsa-miR-4668-3p, hsa-miR-4671-3p, hsa-miR-4679, hsa-miR-4693-3p, hsa-miR-4699-5p, hsa-miR-4704-3p, hsa-miR-4714-3p, hsa-miR-4720-3p, hsa-miR-4735-5p, hsa-miR-4757-3p, hsa-miR-4760-5p, hsa-miR-4772-5p, hsa-miR-4777-5p, hsa-miR-4781-3p, hsa-miR-4782-3p, hsa-miR-4782-5p, hsa-miR-4790-3p, hsa-miR-4791, hsa-miR-4795-5p, hsa-miR-4796-3p, hsa-miR-4798-5p, hsa-miR-4799-3p, hsa-miR-4802-5p, hsa-miR-499a-5p, hsa-miR-5009-3p, hsa-miR-5009-5p, hsa-miR-506-5p, hsa-miR-507, hsa-miR-510-3p, hsa-miR-513b-3p, hsa-miR-514a-5p, hsa-miR-5191, hsa-miR-544a, hsa-miR-545-5p, hsa-miR-548a-3p, hsa-miR-548a-5p, hsa-miR-548aa, hsa-miR-548t-3p, hsa-miR-548ac, hsa-miR-548ad-5p, hsa-miR-548ae-5p, hsa-miR-548ae-3p, hsa-miR-548ag, hsa-miR-548ah-5p, hsa-miR-548ak, hsa-miR-548al, hsa-miR-548am-3p, hsa-miR-548ao-5p, hsa-miR-548ap-5p, hsa-miR-548aq-5p, hsa-miR-548at-3p, hsa-miR-548au-5p, hsa-miR-548az-5p, hsa-miR-548b-5p, hsa-miR-548c-5p, hsa-miR-548o-5p, hsa-miR-548am-5p, hsa-miR-548g-5p, hsa-miR-548x-5p, hsa-miR-548aj-5p, hsa-miR-548j-5p, hsa-miR-548k, hsa-miR-548m, hsa-miR-548p, hsa-miR-548t-5p, hsa-miR-548u, hsa-miR-548v, hsa-miR-548x-3p, hsa-miR-549a, hsa-miR-553, hsa-miR-556-3p, hsa-miR-5579-5p, hsa-miR-5583-5p, hsa-miR-559, hsa-miR-5590-3p, hsa-miR-5590-5p, hsa-miR-5591-5p, hsa-miR-561-5p, hsa-miR-568, hsa-miR-5687, hsa-miR-569, hsa-miR-5692c, hsa-miR-5697, hsa-miR-5700, hsa-miR-570-3p, hsa-miR-577, hsa-miR-582-5p, hsa-miR-587, hsa-miR-590-3p, hsa-miR-6079, hsa-miR-6128, hsa-miR-618, hsa-miR-621, hsa-miR-628-5p, hsa-miR-633, hsa-miR-648, hsa-miR-6508-3p, hsa-miR-651-3p, hsa-miR-652-3p, hsa-miR-6733-5p, hsa-miR-6811-5p, hsa-miR-6844, hsa-miR-6854-5p, hsa-miR-7161-3p, hsa-miR-759, hsa-miR-7-5p, hsa-miR-7852-3p, hsa-miR-873-5p, hsa-miR-876-5p, hsa-miR-9-3p, hsa-miR-95-3p, hsa-miR-95-5p, hsa-miR-9-5p, hsa-miR-4525, hsa-miR-6760-5p, hsa-miR-4538, hsa-miR-5698, hsa-miR-5189-5p, hsa-miR-671-5p, hsa-miR-936, hsa-miR-4307, hsa-miR-6815-5p, hsa-miR-6076, hsa-miR-708-5p, hsa-miR-3187-5p, hsa-miR-3144-3p, hsa-miR-4691-5p, hsa-miR-4700-5p, hsa-miR-576-5p, hsa-miR-3652, hsa-miR-598-3p, hsa-miR-7151-3p, hsa-miR-7162-5p, hsa-miR-1229-5p, hsa-miR-6746-5p, hsa-miR-4762-5p, hsa-miR-1307-3p, hsa-miR-1273g-3p, hsa-miR-4428, hsa-miR-6515-5p, hsa-miR-1273g-5p, hsa-miR-433-3p, hsa-miR-452-3p, hsa-miR-3917, hsa-miR-1224-5p, hsa-miR-6892-5p, hsa-miR-520d-5p, hsa-miR-5092, hsa-miR-30c-1-3p, hsa-miR-1293, hsa-miR-7851-3p, hsa-miR-3934-5p, hsa-miR-3122, hsa-miR-3177-3p, hsa-miR-4800-5p, hsa-miR-4436b-3p, hsa-miR-3928-3p, hsa-miR-630, hsa-miR-628-3p, hsa-miR-6796-5p, hsa-miR-3605-5p, hsa-miR-30e-3p, hsa-miR-6801-5p, hsa-miR-6131, hsa-miR-448, hsa-miR-6834-5p, hsa-miR-3156-5p, hsa-miR-517-5p, hsa-miR-4520-5p, hsa-miR-2276-3p, hsa-miR-3591-3p, hsa-miR-4727-3p, hsa-miR-4499, hsa-miR-1471, hsa-miR-1273h-5p, hsa-miR-4539, hsa-miR-3655, hsa-miR-4690-5p, hsa-miR-6871-5p, hsa-miR-4496, hsa-miR-6772-5p, hsa-miR-4686, hsa-miR-1297, hsa-miR-450a-2-3p, hsa-miR-6745, hsa-miR-3153, hsa-miR-27a-3p, hsa-miR-106b-5p, hsa-miR-519c-5p, hsa-miR-523-5p, hsa-miR-518e-5p, hsa-miR-522-5p, hsa-miR-519a-5p, hsa-miR-519b-5p, hsa-miR-4676-5p, hsa-miR-650, hsa-miR-3922-5p, hsa-miR-4489, hsa-miR-4638-5p, hsa-miR-185-3p, hsa-miR-2467-3p, hsa-miR-4453, hsa-let-7e-5p, hsa-miR-296-3p, hsa-miR-8074, hsa-miR-6876-5p, hsa-miR-6086, hsa-miR-4474-3p, hsa-miR-4776-3p, hsa-miR-1250-5p, hsa-miR-4260, hsa-miR-6747-5p, hsa-miR-181d-3p, hsa-miR-4535, hsa-miR-3177-5p, hsa-miR-4533, hsa-miR-4635, hsa-miR-4776-5p, hsa-miR-4257, hsa-miR-3160-3p, hsa-miR-5702, hsa-miR-4306, hsa-miR-4483, hsa-miR-4472, hsa-miR-3064-3p, hsa-miR-4710, hsa-miR-4293, hsa-miR-449c-5p, hsa-miR-5589-5p, hsa-miR-4443, hsa-miR-4448, hsa-miR-3935, hsa-miR-16-5p, hsa-miR-181a-5p, hsa-miR-147b, hsa-miR-4767, hsa-miR-4677-5p, hsa-miR-6776-5p, hsa-miR-7109-5p, hsa-miR-4669, hsa-miR-4486, hsa-miR-1587, hsa-miR-363-3p, hsa-miR-5701, hsa-miR-921, hsa-miR-5581-5p, hsa-miR-4259, hsa-miR-203b-5p, hsa-miR-3653-3p, hsa-miR-4657, hsa-miR-5589-3p, hsa-miR-2681-3p, hsa-miR-6826-5p, hsa-miR-4647, hsa-miR-4540, hsa-miR-361-5p, hsa-miR-4784, hsa-miR-6861-5p, hsa-miR-6812-5p, hsa-miR-608, hsa-miR-1912, hsa-miR-554, hsa-miR-519a-3p, hsa-miR-891a-3p, hsa-miR-6872-5p, hsa-miR-4781-5p, hsa-miR-4273, hsa-miR-4769-5p, hsa-miR-505-5p, hsa-miR-7158-3p, hsa-miR-527, hsa-miR-518a-5p, hsa-miR-1273f, hsa-miR-624-5p, hsa-miR-125a-3p, hsa-miR-6830-5p, hsa-miR-520d-3p, hsa-miR-6757-5p, hsa-miR-4785, hsa-miR-6068, hsa-miR-1272, hsa-miR-4654, hsa-miR-564, hsa-miR-563, hsa-miR-4300, hsa-miR-4665-5p, hsa-miR-892c-3p, hsa-miR-6778-5p, hsa-miR-4419b, hsa-miR-329-3p, hsa-miR-212-5p, hsa-miR-616-3p, hsa-miR-4269, hsa-miR-4487, hsa-miR-1180-5p, hsa-miR-7850-5p, hsa-miR-492, hsa-miR-718, hsa-miR-1972, hsa-miR-4296, hsa-miR-516b-3p, hsa-miR-516a-3p, hsa-miR-4320, hsa-miR-4529-5p, hsa-miR-656-3p, hsa-miR-6500-3p, hsa-miR-766-5p, hsa-miR-466, hsa-miR-6777-5p, hsa-miR-1909-5p, hsa-miR-187-3p, hsa-miR-8079, hsa-miR-30a-5p, hsa-miR-5002-3p, hsa-miR-6833-5p, hsa-miR-4756-3p, hsa-let-7f-2-3p, hsa-miR-5708, hsa-miR-4721, hsa-miR-8085, hsa-miR-3591-5p, hsa-miR-6859-5p, hsa-miR-5006-5p, hsa-miR-4747-5p, hsa-miR-6827-5p, hsa-miR-3612, hsa-miR-4701-3p, hsa-miR-4418, hsa-miR-4449, hsa-miR-181d-5p, hsa-miR-3619-3p, hsa-miR-6817-5p, hsa-miR-6759-5p, hsa-miR-3654, hsa-miR-3164, hsa-miR-491-3p, hsa-miR-3176, hsa-miR-4683, hsa-miR-6807-5p, hsa-miR-5588-5p, hsa-miR-302c-5p, hsa-miR-4522, hsa-miR-1199-5p, hsa-miR-372-3p, hsa-miR-891a-5p, hsa-miR-1306-3p, hsa-miR-6894-5p, hsa-miR-3613-3p, hsa-miR-548ba, hsa-miR-6795-5p, hsa-miR-6794-5p, hsa-miR-585-3p, hsa-miR-194-3p, hsa-miR-5010-5p, hsa-miR-2052, hsa-miR-614, hsa-miR-6751-5p, hsa-miR-5089-3p, hsa-miR-1289, hsa-miR-668-5p, hsa-miR-3622b-5p, hsa-miR-6887-5p, hsa-miR-4524a-3p, hsa-miR-3689a-3p, hsa-miR-4650-5p, hsa-miR-3689b-3p, hsa-miR-3689c, hsa-miR-624-3p, hsa-miR-6129, hsa-miR-216a-5p, hsa-miR-520b, hsa-miR-4755-5p, hsa-miR-323a-5p, hsa-miR-6726-5p, hsa-miR-516b-5p, hsa-miR-7106-5p, hsa-miR-653-5p, hsa-miR-494-5p, hsa-miR-6780b-5p, hsa-miR-1910-3p, hsa-miR-3160-5p, hsa-miR-5093, hsa-miR-99a-3p, hsa-miR-8071, hsa-miR-4659a-3p, hsa-miR-597-3p, hsa-miR-4458, hsa-miR-6847-5p, hsa-miR-4436a, hsa-miR-17-5p, hsa-miR-185-5p, hsa-miR-4681, hsa-miR-8077, hsa-miR-4440, hsa-let-7g-3p, hsa-miR-375, hsa-miR-10a-5p, hsa-miR-520e, hsa-miR-6072, hsa-miR-3145-5p, hsa-miR-6723-5p, hsa-miR-4692, hsa-miR-4796-5p, hsa-miR-6849-5p, hsa-miR-4430, hsa-miR-6499-5p, hsa-miR-3151-5p, hsa-miR-182-5p, hsa-miR-195-5p, hsa-miR-3681-5p, hsa-miR-6864-5p, hsa-miR-4711-3p, hsa-miR-380-3p, hsa-miR-374c-5p, hsa-miR-758-5p, hsa-miR-6842-5p, hsa-miR-8080, hsa-miR-520f-3p, hsa-miR-4646-5p, hsa-miR-103a-3p, hsa-miR-3189-3p, hsa-miR-4691-3p, hsa-miR-877-5p, hsa-miR-6769b-5p, hsa-miR-5580-5p, hsa-miR-1911-5p, hsa-miR-519b-3p, hsa-miR-660-5p, hsa-miR-4644, hsa-miR-6133, hsa-miR-138-1-3p, hsa-miR-193a-5p, hsa-miR-222-3p, hsa-miR-603, hsa-miR-23b-3p, hsa-miR-5089-5p, hsa-miR-6799-5p, hsa-miR-6715a-3p, hsa-miR-6869-3p, hsa-miR-4445-3p, hsa-miR-542-5p, hsa-miR-1286, hsa-miR-96-3p, hsa-miR-4743-5p, hsa-miR-4724-3p, hsa-miR-4262, hsa-miR-4507, hsa-miR-7160-5p, hsa-miR-605-3p, hsa-miR-200c-3p, hsa-miR-582-3p, hsa-miR-6868-5p, hsa-miR-6773-5p, hsa-miR-1185-1-3p, hsa-miR-3182, hsa-miR-6082, hsa-miR-150-3p, hsa-miR-5002-5p, hsa-miR-487a-5p, hsa-miR-8058, hsa-miR-4529-3p, hsa-miR-3713, hsa-miR-8075, hsa-miR-6877-5p, hsa-miR-4325, hsa-miR-3130-5p, hsa-miR-616-5p, hsa-miR-1322, hsa-miR-6793-5p, hsa-miR-4694-5p, hsa-miR-29a-3p, hsa-miR-6127, hsa-miR-758-3p, hsa-miR-2114-5p, hsa-miR-515-5p, hsa-miR-196a-5p, hsa-miR-4639-3p, hsa-miR-4799-5p, hsa-miR-4498, hsa-miR-6766-5p, hsa-miR-1255b-2-3p, hsa-miR-380-5p, hsa-miR-4419a, hsa-miR-3622a-5p, hsa-let-7b-5p, hsa-miR-4650-3p, hsa-miR-29b-1-5p, hsa-miR-3137, hsa-miR-4447, hsa-miR-6500-5p, hsa-miR-3663-5p, hsa-miR-4451, hsa-miR-4422, hsa-miR-662, hsa-miR-122-3p, hsa-miR-6828-5p, hsa-miR-6884-5p, hsa-miR-4502, hsa-miR-374b-5p, hsa-miR-4462, hsa-miR-4478, hsa-miR-431-3p, hsa-miR-23b-5p, hsa-miR-7975, hsa-miR-5011-5p, hsa-miR-3691-5p, hsa-miR-6768-3p, hsa-miR-591, hsa-miR-8086, hsa-miR-372-5p, hsa-miR-5189-3p, hsa-miR-107, hsa-miR-6891-5p, hsa-miR-3138, hsa-miR-6503-3p, hsa-miR-297, hsa-miR-370-5p, hsa-miR-3944-3p, hsa-miR-425-5p, hsa-miR-3978, hsa-miR-148a-5p, hsa-miR-4684-5p, hsa-miR-34a-3p, hsa-miR-1263, hsa-miR-769-5p, hsa-miR-4437, hsa-miR-2277-3p, hsa-miR-659-5p, hsa-miR-661, hsa-miR-6788-5p, hsa-miR-15b-3p, hsa-miR-1205, hsa-miR-1468-5p, hsa-miR-34a-5p, hsa-miR-3120-5p, hsa-miR-1247-3p, hsa-miR-454-5p, hsa-miR-656-5p, hsa-miR-626, hsa-miR-4441, hsa-miR-6780a-5p, hsa-miR-6769a-5p, hsa-miR-1298-5p, hsa-miR-3157-5p, hsa-miR-6814-5p, hsa-miR-500a-5p, hsa-miR-1321, hsa-miR-4481, hsa-miR-611, hsa-miR-1273a, hsa-miR-337-5p, hsa-miR-203a-5p, hsa-miR-4479, hsa-miR-4719, hsa-miR-8083, hsa-miR-132-3p, hsa-miR-20b-5p, hsa-miR-4252, hsa-miR-429, hsa-miR-378b, hsa-miR-134-5p, hsa-miR-6741-5p, hsa-miR-1193, hsa-miR-143-5p, hsa-miR-515-3p, hsa-miR-4774-3p, hsa-miR-8064, hsa-miR-1183, hsa-miR-5684, hsa-miR-1226-5p, hsa-miR-539-5p, hsa-miR-580-5p, hsa-miR-622, hsa-miR-3907, hsa-miR-6504-5p, hsa-miR-4653-3p, hsa-miR-4706, hsa-miR-4515, hsa-miR-3919, hsa-miR-3187-3p, hsa-miR-1271-5p, hsa-miR-6767-5p, hsa-miR-4804-5p, hsa-miR-1257, hsa-miR-3126-5p, hsa-miR-6731-5p, hsa-miR-3149, hsa-miR-4717-3p, hsa-miR-509-5p, hsa-miR-215-3p, hsa-miR-1178-3p, hsa-miR-1282, hsa-miR-3174, hsa-miR-197-5p, hsa-miR-376c-3p, hsa-miR-5696, hsa-miR-3121-5p, hsa-miR-8082, hsa-miR-6823-5p, hsa-miR-4704-5p, hsa-miR-3616-3p, hsa-miR-6881-5p, hsa-miR-4661-5p, hsa-miR-22-5p, hsa-miR-6730-5p, hsa-let-7e-3p, hsa-miR-1238-5p, hsa-miR-526b-5p, hsa-miR-3163, hsa-miR-4514, hsa-miR-4699-3p, hsa-miR-7154-5p, hsa-miR-4521, hsa-miR-96-5p, hsa-miR-4754, hsa-miR-450a-5p, hsa-miR-4459, hsa-miR-922, hsa-miR-7153-5p, hsa-miR-29b-2-5p, hsa-miR-6738-3p, hsa-miR-6790-5p, hsa-miR-4470, hsa-miR-4732-3p, hsa-miR-4709-3p, hsa-miR-5588-3p, hsa-miR-93-3p, hsa-miR-6820-5p, hsa-miR-655-5p, hsa-miR-6514-3p, hsa-miR-1-5p, hsa-miR-10b-5p, hsa-miR-3193, hsa-miR-3976, hsa-miR-3678-3p, hsa-miR-3912-5p, hsa-miR-4708-3p, hsa-miR-5192, hsa-miR-140-5p, hsa-miR-4285, hsa-miR-3124-5p, hsa-miR-198, hsa-miR-3659, hsa-miR-3689a-5p, hsa-miR-3689b-5p, hsa-miR-3689e, hsa-miR-4643, hsa-miR-552-3p, hsa-miR-3186-3p, hsa-miR-424-3p, hsa-miR-578, hsa-miR-4304, hsa-miR-504-3p, hsa-miR-595, hsa-miR-4501, hsa-miR-4746-3p, hsa-miR-362-5p, hsa-miR-4297, hsa-miR-6821-3p, hsa-miR-3074-5p, hsa-miR-4503, hsa-miR-4518, hsa-miR-6715b-3p, hsa-miR-6736-5p, hsa-miR-7843-3p, hsa-miR-4420, hsa-miR-3927-5p, and hsa-miR-7157-5p.
(8) An extract of urine obtained by contacting a nanowire with a urine of a stage I cancer patient and extracting a constituent bound to said nanowire.
   (8A) The extract of urine according to (8) above, wherein the PH of the urine contacted with the nanowire is a numerical range having a lower limit of 2, 3, 4, or 5 and an upper limit of 11, 10, 9, 8, 7, 6, or 5.
   (8B) The extract of urinary according to (8) or (8A) above, wherein the nanowire is a metal oxide nanowire or a nanowire whose surface is coated with a metal oxide.
   (8C) The extract of urine according to (8B) above, wherein the metal oxide is an oxide of a transition metal.
   (8D) The extract of urine according to (8), (8A), (8B) or (8C) above, wherein the nanowire is a zinc oxide nanowire or a nanowire whose surface is coated with zinc oxide.
   (8E) The extract of urine according to (8), (8A), (8B), (8C), or (8D) above, wherein the nanowire and urine are contacted under a neutral PH condition.
(9) The extract of urine according to (8), (8A), (8B), (8C), (8D) or (8E) above, comprising microRNAs defined in (1) to (6) above.
(10) The extract of urine according to (9), (9A), (9B), (9C), (9D) or (9E) above, comprising 20% or more of the extracellular vesicles contained in the urine.
   (10') The extract of urine according to (9), (9A), (9B), (9C), (9D) or (9E) above, comprising 25% or more of the extracellular vesicles contained in the urine.
   (10A) The extract of urine according to (9), (9A), (9B), (9C), (9D) or (9E) above, comprising 30% or more of the extracellular vesicles contained in the urine.
   (10A') The extract of urine according to (9), (9A), (9B), (9C), (9D) or (9E) above, comprising 35% or more of the extracellular vesicles contained in the urine.
   (10B) The extract of urine according to (9), (9A), (9B), (9C), (9D) or (9E) above, comprising 40% or more of the extracellular vesicles contained in the urine.
   (10B') The extract of urine according to (9), (9A), (9B), (9C), (9D) or (9E) above, comprising 45% or more of the extracellular vesicles contained in the urine.
   (10C) The extract of urine according to (9), (9A), (9B), (9C), (9D) or (9E) above, comprising 50% or more of the extracellular vesicles contained in the urine.
   (10C') An extract of urine according to (9), (9A), (9B), (9C), (9D) or (9E) above, comprising 55% or more of the extracellular vesicles contained in the urine.
   (10D) An extract of urine according to (9), (9A), (9B), (9C), (9D) or (9E) above, comprising at least 60% of the extracellular vesicles contained in the urine.
   (10D') An extract of urine according to (9), (9A), (9B), (9C), (9D) or (9E) above, comprising 65% or more of the extracellular vesicles contained in the urine.
   (10E) An extract of urine according to (9), (9A), (9B), (9C), (9D) or (9E) above, comprising 70% or more of the extracellular vesicles contained in the urine.
   (10E') The extract of urine according to (9), (9A), (9B), (9C), (9D) or (9E) above, comprising 75% or more of the extracellular vesicles contained in the urine.
   (10F) The extract of urine according to (9), (9A), (9B), (9C), (9D) or (9E) above, comprising 80% or more of the extracellular vesicles contained in the urine.
   (10F') The extract of urine according to (9), (9A), (9B), (9C), (9D) or (9E) above, comprising 85% or more of the extracellular vesicles contained in the urine.
   (10G) The extract of urine as described in (9), (9A), (9B), (9C), (9D) or (9E) above, comprising 90% or more of the extracellular vesicles contained in the urine.
   (10H) The extract of urine according to (9), (9A), (9B), (9C), (9D) or (9E) above, comprising 95% or more of the extracellular vesicles contained in the urine.
   (10I) The extract of urine according to (9), (9A), (9B), (9C), (9D) or (9E) above, comprising 97% or more of the extracellular vesicles contained in the urine.
   (10J) The extract of urine according to (9), (9A), (9B), (9C), (9D) or (9E) above, comprising 99% or more of the extracellular vesicles contained in the urine.
(11) The extract of urine according to any one of (1) to (4) above, comprising said microRNA concentrated.
   (11A) The extract of urine according to any one of (1) to (4), (8), (8A), (8B), (8C), (8D) and (8E) above, comprising 500 or more types of microRNAs present in the urine.
(12) A method of detecting microRNAs in a body fluid of a subject, the method comprising detecting at least one microRNA or all microRNAs selected from the group consisting of the below, by contacting the body fluid sample obtained from the subject with a detecting agent for the microRNA to be detected.

[1] An extract of urine extract, comprising a microRNA selected from the group consisting of (i) to (xvi):
   (i) any of the microRNAs listed in data S1 or Table 2;
   (ii) any of the microRNAs listed in Table 4-1;
   (iii) any of the microRNAs listed in Table 4-2;
   (iv) any of the microRNAs listed in Table 4-3;
   (v) any of the microRNAs listed in Table 4-4;
   (vi) any of the microRNAs listed in Table 4-5;
   (vii) any of the microRNAs listed in Table 4-6;
   (viii) any of the microRNAs listed in Table 4-7;
   (ix) any of the microRNAs listed in Table 4-8;
   (x) any of the microRNAs listed in Table 4-9;
   (xi) any of the microRNAs listed in Table 4-10;
   (xii) any of the microRNAs listed in Table 4-11;
   (xiii) any of the microRNAs listed in Table 4-12;
   (xiv) any of the microRNAs listed in Table 4-13;
   (xv) any of the microRNAs listed in Table 4-14; and
   (xvi) any of the microRNAs listed in Table 4-15,
   {The microRNAs of each table may each independently comprise 1 or more, 2 or more, 3 or more, 4 or more, 5 or more, 10 or more, 15 or more, or 20 or more of the microRNAs. That is, the urine extract may comprise a combination of a plurality of microRNAs from a particular table and may comprise a plurality of such combinations.}
[2] A urine extract according to [1] above, comprising a microRNA selected from the group consisting of the following (ii) to (xvi):
   (ii) any of the microRNA with a p-value less than 0.005, listed in Table 4-1;
   (iii) any of the microRNA with a p-value less than 0.005, listed in Table 4-2;
   (iv) any of the microRNA with a p-value less than 0.005, listed in Table 4-3;
   (v) any of the microRNA with a p-value less than 0.005, listed in Table 4-4;
   (vi) any of the microRNA with a p-value less than 0.005, listed in Table 4-5;
   (vii) any of the microRNA with a p-value less than 0.005, listed in Table 4-6;
   (viii) any of the microRNA with a p-value less than 0.005, listed in Table 4-7;
   (ix) any of the microRNA with a p-value less than 0.005, listed in Table 4-8;
   (x) any of the microRNA with a p-value less than 0.005, listed in Table 4-9;
   (xi) any of the microRNA with a p-value less than 0.005, listed in Table 4-10;
   (xii) any of the microRNA with a p value less than 0.005, listed in Table 4-11; and
   (xiii) any of the microRNA with a p-value less than 0.005, listed in Table 4-12;
   (xiv) any of the microRNA with a p-value less than 0.005, listed in Table 4-13;
   (xv) any of the microRNA with a p value less than 0.005, listed in Table 4-14; and
   (xvi) any of the microRNA with a p-value less than 0.005, listed in Table 4-15,
   {The microRNAs of each table may each independently comprise 1 or more, 2 or more, 3 or more, 4 or more, 5 or more, 10 or more, 15 or more, or 20 or more of the microRNAs.}
[3] The urine extract according to the above [1],
   comprising (ii) any of the microRNAs listed in Table 4-1 {may be one or more, two or more, three or more, four or more, five or more, 10 or more, 15 or more, or 20 or more microRNAs},
   wherein the urine extract is a urine extract of a lung cancer patient.
[4] The urine extract according to the above [1],
   comprising (iii) any of the microRNAs listed in Table 4-2{may be one or more, two or more, three or more, four or more, five or more, 10 or more, 15 or more, or 20 or more microRNAs},
   wherein the urine extract is a urine extract of a breast cancer patient.
[5] The urine extract according to the above [1],
   comprising (iv) any of the microRNAs listed in Table 4-3 {may be one or more, two or more, three or more, four or more, five or more, 10 or more, 15 or more, or 20 or more microRNAs},
   wherein the urine extract is a urine extract of a kidney cancer patient.
[6] The urine extract according to the above [1],
   comprising (v) any of the microRNAs listed in Table 4-4 {may be one or more, two or more, three or more, four or more, five or more, 10 or more, 15 or more, or 20 or more microRNAs},
   wherein the urine extract is a urine extract of a leukemia patient.
[7] The urine extract according to the above [1],
   comprising (vi) any of the microRNAs listed in Table 4-5 {may be one or more, two or more, three or more, four or more, five or more, 10 or more, 15 or more, or 20 or more microRNAs},
   wherein the urine extract is a urine extract of a lymphoma patient.
[8] The urine extract according to the above [1],
   comprising (vii) any of the microRNAs listed in Table 4-6 {may be one or more, two or more, three or more, four or more, five or more, 10 or more, 15 or more, or 20 or more microRNAs},
   wherein the urine extract is a urine extract of a pancreatic cancer patient.
[9] The urine extract according to the above [1],
   comprising (viii) any of the microRNAs listed in Table 4-7 {may be one or more, two or more, three or more, four or more, five or more, 10 or more, 15 or more, or 20 or more microRNAs},
   wherein the urine extract is a urine extract of a prostate cancer patient.
[10] The urine extract according to the above [1],
   comprising (ix) any of the microRNAs listed in Table 4-8 {may be one or more, two or more, three or more, four or more, five or more, 10 or more, 15 or more, or 20 or more microRNAs},
   wherein the urine extract is a urine extract of a gastric cancer patient.
[11] The urine extract according to the above [1],
   comprising (x) any of the microRNAs listed in Table 4-9 {may be one or more, two or more, three or more, four or more, five or more, 10 or more, 15 or more, or 20 or more microRNAs},
   wherein the urine extract is a urine extract of a urothelial carcinoma patient.
[12] The urine extract according to the above [1],
   comprising (xi) any of the microRNAs listed in Table 4-10 {may be one or more, two or more, three or more, four or more, five or more, 10 or more, 15 or more, or 20 or more microRNAs},
   wherein the urine extract is a urine extract of a melanoma patient.
[13] The urine extract according to the above [1],
   comprising (xii) any of the microRNA listed in Table 4-11 {may be one or more, two or more, three or more, four or more, five or more, 10 or more, 15 or more, or 20 or more microRNAs},
   wherein the urine extract is a urine extract of an ovarian cancer patient.
[14] The urine extract according to the above [1],
   comprising (xiii) any of the microRNAs listed in Table 4-12 {may be one or more, two or more, three or more, four or more, five or more, 10 or more, 15 or more, or 20 or more microRNAs}, wherein the urine extract is a urine extract of a thyroid cancer patient.
[15] The urine extract according to the above [1],
   comprising (xiii) any of the microRNA listed in Table 4-13 {may be one or more, two or more, three or more, four or more, five or more, 10 or more, 15 or more, or 20 or more microRNAs},
   wherein the urine extract is a urine extract of a cervical cancer patient.
[16] The urine extract according to the above [1],
   comprising (xiii) any of the microRNA listed in Table 4-14 {may be one or more, two or more, three or more, four or more, five or more, 10 or more, 15 or more, or 20 or more microRNAs}, wherein the urine extract is a urine extract of a colorectal cancer patient.
[17] The urine extract according to the above [1],
   comprising (xiii) any of the microRNA listed in Table 4-15 {may be one or more, two or more, three or more, four or more, five or more, 10 or more, 15 or more, or 20 or more microRNAs},
   wherein the urine extract is a urine extract of an endometrial cancer patient.
[18] The extract of urine according to any one of [1] to [17] above, comprising an extracellular vesicle, wherein said microRNA is contained in the extracellular vesicle, or wherein said microRNA is extracted from the extracellular vesicle.
[19] A method for predicting the likelihood that a subject has cancer in a subject having or suspected of having cancer,
   wherein the method may comprise providing a urine extract of the subject,
   the method comprising predicting the likelihood that a subject has cancer as an indicator, using one or more of microRNAs selected from the group consisting of the below, in the urine extract obtained from the subject, as an indicator {In each table, said microRNA may be one or more, two or more, three or more, four or more, five or more, 10 or more, 15 or more, or 20 or more microRNAs }.
      (i) any of the microRNAs listed in data S1 or in Table 2;
      (ii) any of the microRNAs listed in Table 4-1;
      (iii) any of the microRNAs listed in Table 4-2;
      (iv) any of the microRNAs listed in Table 4-3;
      (v) any of the microRNAs listed in Table 4-4;
      (vi) any of the microRNAs listed in Table 4-5;
      (vii) any of the microRNAs listed in Table 4-6;
      (viii) any of the microRNAs listed in Table 4-7;
      (ix) any of the microRNAs listed in Table 4-8;
      (x) any of the microRNAs listed in Table 4-9;
      (xi) any of the microRNAs listed in Table 4-10;
      (xii) any of the microRNAs listed in Table 4-11;
      (xiii) any of the microRNAs listed in Table 4-12;
      (xiv) any of the microRNAs listed in Table 4-13;
      (xv) any of the microRNAs listed in Table 4-14; and
      (xvi) any of the microRNAs listed in Table 4-15/.
[20] The method according to [19] above, wherein
   if the expression level of any one or more of the microRNAs in the urine extract obtained from the
   subject, listed in each table {in each table, the microRNA may be one or more, two or more, three or more, four or more, five or more, 10 or more, 15 or more, or 20 or more microRNAs} is greater than or equal to the first threshold set for each microRNA, indicating a likelihood that the urine is derived from a cancer patient.
[21] The method according to [20] above, wherein
   if the expression level of any one or more of the microRNAs in the urine extract obtained from the subject, listed in each table {in each table, the microRNA may be one or more, two or more, three or more, four or more, five or more, 10 or more, 15 or more, or 20 or more microRNAs} is greater than or equal to the first threshold set for each microRNA, indicating a likelihood that the urine is derived from a patient having a cancer selected from the group consisting of: lung cancer, breast cancer, kidney cancer, leukemia, lymphoma, pancreatic cancer, prostate cancer, melanoma, ovarian cancer, thyroid cancer, cervical cancer, colorectal cancer, and endometrial cancer.
[22] The method according to [20] or [21] above, wherein the first threshold is a value less than or equal to a third quartile value of the corresponding microRNA of a healthy person.
[23] The method according to any one of [20] to [22] above, wherein if the expression level of any one or more of the microRNAs in the urine extract obtained from the subject, selected from the group consisting of miR-17-5p, miR-33a-5p, miR-95-3p, miR-9-5p, miR-155-5p, miR-496, miR-544a, miR-556-5p, miR-577, miR-607, miR-19a-5p, "miR-548c-5p, miR-548o-5p, miR-548am-5p", miR-1278, miR-3117-3p, miR-3128, miR-3139, miR-3145-3p, miR-3201, miR-4282, miR-548ad-3p, miR-548ag, miR-4471, miR-4678, miR-4699-5p, miR-4704-3p, miR-1245b-5p, miR-5582-5p, miR-5582-3p, miR-5590-5p, miR-548aw, miR-5683, miR-580-5p, miR-1252-3p, miR-8058, and miR-548bb-5p {may be one or more, two or more, three or more, four or more, five or more, 10 or more, 15 or more, or 20 or more microRNAs} is greater than or equal to the first threshold set for each microRNA, indicating a likelihood that the urine is derived from a patient having lung cancer.
[24] The method according to any one of [20] to [22] above, wherein if the expression level of any one or more of the microRNAs in the urine extract obtained from the subject, selected from the group consisting of selected from the group consisting of miR-22-3p, miR-380-5p, miR-93-3p, miR-99a-3p, miR-543, miR-5706, and miR-6739-5p {may be one or more, two or more, three or more, four or more, five or more, 10 or more, 15 or more, or 20 or more microRNAs} is greater than or equal to the first threshold set for each microRNA, indicating a likelihood that the urine is derived from a patient having breast cancer.
[25] The method according to any one of [20] to [22] above, wherein if the expression level of any one or more of the microRNAs in the urine extract obtained from the subject, selected from the group consisting of selected from the group consisting of miR-22-3p, miR-380-5p, miR-93-3p, miR-99a-3p, miR-543, miR-5706, and miR-6739-5p {may be one or more, two or more, three or more, four or more, five or more, 10 or more, 15 or more, or 20 or more microRNAs} is greater than or equal to the first threshold set for each microRNA, indicating a likelihood that the urine is derived from a patient having kidney cancer.
[26] The method according to any one of [20] to [22] above, wherein if the expression level of any one or more of the microRNAs in the urine extract obtained from the subject, selected from the group consisting of selected from the group consisting of miR-301a-3p and miR-2114-3p {may be one or more, two or more, three or more, four or more, five or more, 10 or more, 15 or more, or 20 or more microRNAs} is greater than or equal to the first threshold set for each microRNA, indicating a likelihood that the urine is derived from a patient having leukemia.
[27] The method according to any one of [20] to [22] above, wherein if the expression level of any one or more of the microRNAs in the urine extract obtained from the subject, selected from the group consisting of selected from the group consisting of miR-30e-3p, miR-3166, miR-3942-5p, miR-4482-5p, miR-4493, and miR-5008-3p {may be one or more, two or more, three or more, four or more, five or more, 10 or more, 15 or more, or 20 or more microRNAs} is greater than or equal to the first threshold set for each microRNA, indicating a likelihood that the urine is derived from a patient having lymphoma.
[28] The method according to any one of [20] to [22] above, wherein if the expression level of any one or more of the microRNAs in the urine extract obtained from the subject, selected from the group consisting of selected from the group consisting of miR-16-5p, miR-218-5p, let-7g-5p, miR-195-5p, miR-619-3p, miR-643, miR-502-3p, miR-450b-5p, miR-3974, miR-4771, miR-5009-5p, miR-548at-3p, miR-372-5p, and miR-519d-5p {may be one or more, two or more, three or more, four or more, five or more, 10 or more, 15 or more, or 20 or more microRNAs} is greater than or equal to the first threshold set for each microRNA, indicating a likelihood that the urine is derived from a patient having pancreatic cancer.
[29] The method according to any one of [20] to [22] above, wherein if the expression level of miR-1324 in the urine extract obtained from the subject is greater than or equal to the first threshold set for each microRNA, indicating a likelihood that the urine is derived from a patient having prostate cancer.
[30] The method according to any one of [20] to [22] above, wherein if the expression level of any one or more of the microRNAs in the urine extract obtained from the subject, selected from the group consisting of selected from the group consisting of miR-1243 and miR-4715-3p {may be one or more, two or more, three or more, four or more, five or more, 10 or more, 15 or more, or 20 or more microRNAs} is greater than or equal to the first threshold set for each microRNA, indicating a likelihood that the urine is derived from a patient having gastric cancer.
[31] The method according to any one of [20] to [22] above, wherein if the expression level of any one or more of the microRNAs in the urine extract obtained from the subject, selected from the group consisting of selected from the group consisting of miR-4795-3p, miR-5707, and miR-655-5p {may be one or more, two or more, three or more, four or more, five or more, 10 or more, 15 or more, or 20 or more microRNAs} is greater than or equal to the first threshold set for each microRNA, indicating a likelihood that the urine is derived from a patient having urothelial carcinoma.
[32] The method according to any one of [20] to [22] above, wherein if the expression level of any one or more of the microRNAs in the urine extract obtained from the subject, selected from the group consisting of selected from the group consisting of miR-18b-5p, miR-562, miR-582-3p, miR-216b-5p, miR-1251-5p, miR-1252-5p, miR-2053, miR-3915, miR-4418, miR-4659a-3p, miR-651-3p, miR-1468-3p, and miR-9903 {may be one or more, two or more, three or more, four or more, five or more, 10 or more, 15 or more, or 20 or more microRNAs} is greater than or equal to the first threshold set for each microRNA, indicating a likelihood that the urine is derived from a patient having melanoma.
[33] The method according to any one of [20] to [22] above, wherein if the expression level of any one or more of the microRNAs in the urine extract obtained from the subject, selected from the group consisting of selected from the group consisting of miR-335-5p, miR-27a-5p, miR-499a-3p, and miR-8084 {may be one or more, two or more, three or more, four or more, five or more, 10 or more, 15 or more, or 20 or more microRNAs} is greater than or equal to the first threshold set for each microRNA, indicating a likelihood that the urine is derived from a patient having ovarian cancer.
[34] The method according to any one of [20] to [22] above, wherein if the expression level of any one or more of the microRNAs in the urine extract obtained from the subject, selected from the group consisting of selected from the group consisting of miR-376a-5p, miR-374b-3p, miR-4477b, miR-548am-3p, miR-4536-5p, miR-4639-5p, and miR-4798-3p {may be one or more, two or more, three or more, four or more, five or more, 10 or more, 15 or more, or 20 or more microRNAs} is greater than or equal to the first threshold set for each microRNA, indicating a likelihood that the urine is derived from a patient having thyroid cancer.
[35] The method according to any one of [20] to [22] above, wherein if the expression level of any one or more of the microRNAs in the urine extract obtained from the subject, selected from the group consisting of selected from the group consisting of miR-143-5p, miR-3182, miR-4746-5p, miR-548ao-3p, miR-514a-5p, miR-376c-5p, miR-376a-2-5p, miR-6773-5p, miR-6807-3p, miR-8065, and miR-301b-5p {may be one or more, two or more, three or more, four or more, five or more, 10 or more, 15 or more, or 20 or more microRNAs} is greater than or equal to the first threshold set for each microRNA, indicating a likelihood that the urine is derived from a patient having cervical cancer.
[36] The method according to any one of [20] to [22] above, wherein if the expression level of any one or more of the microRNAs in the urine extract obtained from the subject, selected from the group consisting of selected from the group consisting of miR-10b-5p, miR-337-5p, miR-6888-3p, and miR-9983-3p {may be one or more, two or more, three or more, four or more, five or more, 10 or more, 15 or more, or 20 or more microRNAs} is greater than or equal to the first threshold set for each microRNA, indicating a likelihood that the urine is derived from a patient having rectal colon cancer.
[37] The method according to any one of [20] to [22] above, wherein if the expression level of any one or more of the microRNAs in the urine extract obtained from the subject, selected from the group consisting of selected from the group consisting of miR-199a-3p, miR-199b-3p, miR-518e-3p, miR-626, miR-548x-3p, miR-4659a-5p, miR-4671-3p, miR-4666b, and miR-8068 {may be one or more, two or more, three or more, four or more, five or more, 10 or more, 15 or more, or 20 or more microRNAs} is greater than or equal to the first threshold set for each microRNA, indicating a likelihood that the urine is derived from a patient having endometrial cancer.
[38] The method according to [23] or [27] above, wherein the first threshold is a value less than or equal to a third quartile value of the corresponding microRNA of a healthy person.
[39] The method according to [19] above, wherein if the expression level of any one or more of the microRNAs in the urine extract obtained from the subject, listed in each table {may be one or more, two or more, three or more, four or more, five or more, 10 or more, 15 or more, or 20 or more microRNAs} is less than or equal to the second threshold set for each microRNA, indicating a likelihood that the urine is derived from a patient having thyroid cancer.
[40] A method for predicting a likelihood that a subject having or suspected of having cancer does not have cancer, wherein if any one or more of the microRNAs in the urine extract obtained from the subject, selected from the group consisting of miR-17-5p, miR-33a-5p, miR-95-3p, miR-9-5p, miR-155-5p, miR-496, miR-544a, miR-556-5p, miR-577, miR-607, miR-19a-5p, "miR-548c-5p, miR-548o-5p, miR-548am-5p", miR-1278, miR-3117-3p, miR-3128, miR-3139, miR-3145-3p, miR-3201, miR-4282, miR-548ad-3p, miR-548ag, miR-4471, miR-4678, miR-4699-5p, miR-4704-3p, miR-1245b-5p, miR-5582-5p, miR-5582-3p, miR-5590-5p, miR-548aw, miR-5683, miR-580-5p, miR-1252-3p, miR-8058, and miR-548bb-5plet-7a-5p, let-7c-5p, let-7d-5p, let-7f-5p, miR-18a-5p, miR-19a-3p, miR-20a-5p, miR-21-5p, miR-24-1-5p, miR-26a-5p, miR-26b-5p, miR-31-5p, miR-96-5p, miR-98-5p, miR-99a-5p, miR-100-5p, miR-101-3p, miR-106a-5p, miR-148a-3p, miR-7-5p, miR-181c-5p, miR-182-3p, miR-216a-5p, miR-217-5p, miR-219a-5p, miR-222-3p, miR-1-3p, miR-130a-3p, miR-137-3p, miR-142-5p, miR-144-3p, miR-152-3p, miR-153-3p, miR-9-3p, miR-126-5p, miR-126-3p, miR-127-3p, miR-136-5p, miR-150-5p, miR-186-5p, miR-190a-5p, miR-200a-3p, miR-302a-5p, miR-30e-5p, miR-362-5p, miR-302b-5p, miR-302b-3p, miR-302c-3p, miR-302d-3p, miR-367-3p, miR-376c-3p, miR-369-3p, miR-376a-3p, miR-377-3p, miR-384, miR-409-5p, miR-410-3p, miR-376b-3p, miR-146b-5p, miR-520f-3p, miR-519c-3p, miR-523-3p, miR-518c-3p, miR-524-3p, miR-518d-3p, miR-499a-5p, miR-505-3p, miR-507, miR-508-3p, miR-514a-3p, miR-455-5p, miR-545-3p, miR-553, miR-559, miR-561-3p, miR-567, miR-568, miR-569, miR-570-3p, miR-573, miR-582-5p, miR-548a-3p, miR-586, miR-548b-3p, miR-590-5p, miR-592, miR-597-5p, miR-599, miR-606, miR-548c-3p, miR-618, miR-620, miR-621, miR-33b-5p, miR-633, miR-648, miR-651-5p, miR-411-5p, miR-655-3p, miR-549a-3p, miR-660-5p, miR-542-3p, miR-758-3p, miR-454-3p, miR-802, miR-19b-1-5p, miR-19b-2-5p, miR-28-3p, miR-100-3p, miR-105-3p, miR-30d-3p, miR-10a-3p, miR-10b-3p, miR-34a-3p, miR-196a-3p, miR-218-1-3p, miR-218-2-3p, miR-221-5p, miR-27b-5p, miR-195-3p, miR-26a-2-3p, miR-367-5p, miR-374a-3p, miR-340-5p, miR-135b-3p, miR-488-3p, miR-497-3p, miR-455-3p, miR-545-5p, miR-556-3p, miR-576-3p, miR-548b-5p, miR-590-3p, miR-548a-5p, miR-628-5p, miR-548d-5p, miR-450b-3p, miR-890, miR-889-3p, miR-875-5p, miR-876-5p, miR-708-3p, miR-190b-5p, miR-873-5p, miR-301b-3p, miR-208b-3p, miR-922, miR-934, miR-944, miR-1185-5p, miR-1283, miR-1179, miR-1183, miR-1206, miR-548e-3p, miR-548j-5p, miR-548k, miR-1295a, miR-1302, miR-1305, miR-1244, miR-1245a, miR-1256, miR-1258, miR-1261, miR-1262, miR-548n, miR-548m, miR-548h-5p, miR-302e, miR-302f, miR-1277-3p, miR-548i, miR-1282, miR-1197, miR-1537-3p, miR-205-3p, miR-1973, miR-2054, miR-759, miR-2115-3p, miR-3115, miR-3118, miR-3121-3p, miR-3123, miR-3129-5p, miR-3134, miR-3135a, miR-544b, miR-3140-3p, miR-548t-5p, miR-3143, miR-548u, miR-3146, miR-3152-3p, miR-3159, miR-3167, miR-3171, miR-548w, miR-3183, miR-3065-5p, miR-4302, miR-4309, miR-4263, miR-4272, miR-4277, miR-4291, miR-3606-5p, miR-3609, miR-3611, miR-3613-5p, miR-3616-5p, miR-3618, miR-23c, miR-3658, miR-3662, miR-3664-5p, miR-3668, miR-3671, miR-3672, miR-3674, miR-3683, miR-3684, miR-3686, miR-3689a-5p, miR-3689b-5p, miR-3689e, miR-3912-3p, miR-3914, miR-3920, miR-3923, miR-3927-3p, miR-3938, miR-548y, miR-3939, miR-548z, miR-548h-3p, miR-4422, miR-548ac, miR-4424, miR-4426, miR-4427, miR-548ae-3p, miR-4445-5p, miR-4452, miR-4457, miR-4464, miR-548ai, miR-570-5p, miR-548aj-3p, miR-4469, miR-4477a, miR-548ak, miR-4490, miR-4500, miR-4503, miR-4504, miR-4509, miR-4517, miR-4528, miR-548an, miR-3117-5p, miR-3121-5p, miR-3124-3p, miR-3136-3p, miR-3140-5p, miR-3664-3p, miR-3688-5p, miR-3942-3p, miR-4474-5p, miR-3976, miR-3977, miR-4636, miR-4662a-5p, miR-4662a-3p, miR-4659b-5p, miR-4663, miR-4662b, miR-4666a-5p, miR-4666a-3p, miR-4668-3p, miR-219b-3p, miR-4671-5p, miR-4677-3p, miR-4679, miR-4680-5p, miR-4680-3p, miR-4683, miR-4693-5p, miR-4693-3p, miR-4696, miR-4699-3p, miR-4703-5p, miR-4705, miR-203b-3p, miR-4711-5p, miR-4714-3p, miR-4720-3p, miR-4735-5p, miR-4735-3p, miR-4742-5p, miR-4744, miR-499b-5p, miR-4757-5p, miR-4757-3p, miR-4760-5p, miR-4760-3p, miR-4765, miR-4766-3p, miR-4772-5p, miR-4772-3p, miR-4774-5p, miR-4774-3p, miR-4777-5p, miR-4777-3p, miR-4778-3p, miR-4782-5p, miR-4782-3p, miR-1245b-3p, miR-4789-5p, miR-4789-3p, miR-4790-3p, miR-4795-5p, miR-4797-5p, miR-4798-5p, miR-4799-3p, miR-4520-2-3p, miR-5047, miR-548ah-3p, miR-548ao-5p, miR-548ap-5p, miR-548ap-3p, miR-5186, miR-5191, miR-5197-3p, miR-548aq-5p, miR-548aq-3p, miR-548ar-5p, miR-548ar-3p, miR-5583-5p, miR-5583-3p, miR-5586-5p, miR-548au-5p, miR-5590-3p, miR-5591-5p, miR-548av-5p, miR-5682, miR-5692c, miR-5687, miR-5688, miR-5691, miR-5692a, miR-5697, miR-5700, miR-5701, miR-5692b, miR-450a-1-3p, miR-506-5p, miR-539-3p, miR-561-5p, miR-1271-3p, miR-548g-5p, miR-548x-5p, miR-548aj-5p, miR-1277-5p, miR-513c-3p, miR-1178-5p, miR-3606-3p, miR-6079, miR-6128, miR-548ay-5p, miR-548az-5p, miR-548az-3p, miR-6502-3p, miR-6508-3p, miR-95-5p, miR-215-3p, miR-153-5p, miR-190a-3p, miR-412-5p, miR-520g-5p, miR-510-3p, miR-653-3p, miR-670-3p, miR-548e-5p, miR-548f-5p, miR-513b-3p, miR-1537-5p, miR-6733-5p, miR-6811-5p, miR-6828-3p, miR-6838-3p, miR-6844, miR-6854-5p, miR-6864-3p, miR-6866-5p, miR-7153-5p, miR-7153-3p, miR-7154-5p, miR-7159-3p, miR-7160-3p, miR-7161-3p, miR-7705, miR-6516-3p, miR-8053, miR-8056, miR-8061, miR-8066, miR-8067, miR-8076, miR-181b-2-3p, miR-548ad-5p, miR-548ae-5p, miR-548bb-3p, miR-520e-5p, miR-549a-5p, miR-147b-5p, miR-9900, miR-548bc, miR-10393-5p, miR-10393-3p, miR-10397-3p, miR-10399-5p, miR-10525-3p, miR-12123, miR-12129, miR-12130, and miR-12132 {may be one or more, two or more, three or more, four or more, five or more, 10 or more, 15 or more, or 20 or more microRNAs} is less than or equal to the first threshold for a microRNA marker which shows an expression higher for cancer patients than non-cancer patients, or is greater than or equal to the second threshold for a microRNA marker which shows an expression lower for cancer patients than non-cancer patients, indicating a likelihood that the urine is derived from a patient not having cancer.
[41] A method for predicting a likelihood that a subject having or suspected of having cancer has early-stage cancer, the method comprising predicting the likelihood that a subject has early-stage cancer, by using the expression of the microRNA in the urine extract of the subject, listed in in any one of tables of Tables 22-1 and Tables 23-1 to 23-14 {In each table, said microRNA may be one or more, two or more, three or more, four or more, five or more, 10 or more, 15 or more, or 20 or more microRNAs} as an indicator.
[42] A method for predicting a likelihood that a subject having or suspected of having cancer has stage-I early-stage cancer, the method comprising predicting the likelihood that a subject has early stage cancer, by using the expression of the microRNA in any one of tables of Tables 22-1 to 22-13 {In each table, said microRNA may be one or more, two or more, three or more, four or more, five or more, 10 or more, 15 or more, or 20 or more microRNAs} as an indicator.
[43] A method of analyzing a urine sample, comprising:
   contacting a urine sample with a nanowire {which can be a nanowire in a nanowire-embedded fluidic device having nanowires};
   extracting microRNAs captured on the nanowires; and
   confirming whether the extracted microRNAs contain a specific microRNA by contacting the microRNA with a nucleic acid containing a sequence which hybridizes to the microRNA, wherein the specific microRNA is one or more microRNAs selected from the group consisting of {in each table, each independently may be one or more, two or more, three or more, four or more, five or more, 10 or more, 15 or more, or 20 or more microRNAs}:
      (i) any of the microRNAs listed in data S1 or Table 2;
      (ii) any of the microRNAs listed in Table 4-1;
      (iii) any of the microRNAs listed in Table 4-2;
      (iv) any of the microRNAs listed in Table 4-3;
      (v) any of the microRNAs listed in Table 4-4;
      (vi) any of the microRNAs listed in Table 4-5;
      (vii) any of the microRNAs listed in Table 4-6;
      (viii) any of the microRNAs listed in Table 4-7;
      (ix) any of the microRNAs listed in Table 4-8;
      (x) any of the microRNAs listed in Table 4-9;
      (xi) any of the microRNAs listed in Table 4-10;
      (xii) any of the microRNAs listed in Table 4-11;
      (xiii) any of the microRNAs listed in Table 4-12;
      (xiv) any of the microRNAs listed in Table 4-13;
      (xv) any of the microRNAs listed in Table 4-14; and
      (xvi) Any of the microRNAs listed in Table 4-15.
[44] The method according to [43] above,
   wherein the specific microRNA is one or more microRNAs selected from the group consisting of {in each table, each independently may be one or more, two or more, three or more, four or more, five or more, 10 or more, 15 or more, or 20 or more microRNAs}:
   the below (ii) to (xiii):
      (ii) any of the microRNA with a p-value less than 0.005, listed in Table 4-1;
      (iii) any of the microRNA with a p-value less than 0.005, listed in Table 4-2;
      (iv) any of the microRNA with a p-value less than 0.005, listed in Table 4-3;
      (v) any of the microRNA with a p-value less than 0.005, listed in Table 4-4;
      (vi) any of the microRNA with a p-value less than 0.005, listed in Table 4-5;
      (vii) any of the microRNA with a p-value less than 0.005, listed in Table 4-6;
      (viii) any of the microRNA with a p-value less than 0.005, listed in Table 4-7;
      (ix) any of the microRNA with a p-value less than 0.005, listed in Table 4-8;
      (x) any of the microRNA with a p-value less than 0.005, listed in Table 4-9;
      (xi) any of the microRNA with a p-value less than 0.005, listed in Table 4-10;
      (xii) any of the microRNAs with a p value less than 0.005, listed in Table 4-11; and
      (xiii) any of the microRNA with a p-value less than 0.005, listed in Table 4-12;
      (xiv) any of the microRNA with a p-value less than 0.005, listed in Table 4-13;
      (xv) any of the microRNAs with a p value of less than 0.005, listed in Table 4-14; and
      (xvi) any of the microRNA with a p-value less than 0.005, listed in Table 4-15.
[45] The method according to [43] or [44] above,
   wherein the urine is the urine of a subject having or suspected of having lung cancer,
   wherein the specific microRNAs is
      (ii) any of the microRNAs listed in Table 4-1 {may be one or more, two or more, three or more, four or more, five or more, 10 or more, 15 or more, or 20 or more microRNAs}.
[46] The method according to [43] or [44] above,
   wherein the urine is the urine of a subject having or suspected of having breast cancer,
   wherein the specific microRNAs is
      (iii) any of the microRNAs listed in Table 4-2 {may be one or more, two or more, three or more, four or more, five or more, 10 or more, 15 or more, or 20 or more microRNAs}.
[47] The method according to [43] or [44] above,
   wherein the urine is the urine of a subject having kidney cancer or suspected of having breast cancer, wherein the specific microRNAs is
   (iv) any of the microRNAs listed in Table 4-3 {may be one or more, two or more, three or more, four or more, five or more, 10 or more, 15 or more, or 20 or more microRNAs}.
[48] The method according to [43] or [44] above,
   wherein the urine is the urine of a subject having or suspected of having leukemia,
   wherein the specific microRNAs is
   (v) any of the microRNAs listed in Table 4-4 {may be one or more, two or more, three or more, four or more, five or more, 10 or more, 15 or more, or 20 or more microRNAs}.
[49] The method according to [43] or [44] above,
   wherein the urine is the urine of a subject having or suspected of having lymphoma,
   wherein the specific microRNAs is
   (vi) any of the microRNAs listed in Table 4-5 {may be one or more, two or more, three or more, four or more, five or more, 10 or more, 15 or more, or 20 or more microRNAs}.
[50] The method according to [43] or [44] above,
   wherein the urine is the urine of a subject having or suspected of having pancreatic cancer,
   wherein the specific microRNAs is
   (vii) any of the microRNAs listed in Table 4-6 {may be one or more, two or more, three or more, four or more, five or more, 10 or more, 15 or more, or 20 or more microRNAs}.
[51] The method according to [43] or [44] above,
   wherein the urine is the urine of a subject having or suspected of having prostate cancer,
   wherein the specific microRNAs is
   (viii) any of the microRNAs listed in Table 4-7 {may be one or more, two or more, three or more, four or more, five or more, 10 or more, 15 or more, or 20 or more microRNAs}.
[52] The method according to [43] or [44] above,
   wherein the urine is the urine of a subject having or suspected of having gastric cancer,
   wherein the specific microRNAs is
   (ix) any of the microRNAs listed in Table 4-8 {may be one or more, two or more, three or more, four or more, five or more, 10 or more, 15 or more, or 20 or more microRNAs}.
[53] The method according to [43] or [44] above,
   wherein the urine is the urine of a subject having or suspected of having urothelial carcinoma,
   wherein the specific microRNAs is
   (x) any of the microRNAs listed in Table 4-9 {may be one or more, two or more, three or more, four or more, five or more, 10 or more, 15 or more, or 20 or more microRNAs}.
[54] The method according to [43] or [44] above,
   wherein the urine is the urine of a subject having or suspected of having melanoma,
   wherein the specific microRNAs is
   (xi) any of the microRNAs listed in Table 4-10 {may be one or more, two or more, three or more, four or more, five or more, 10 or more, 15 or more, or 20 or more microRNAs}.
[55] The method according to [43] or [44] above,
   wherein the urine is the urine of a subject having or suspected of having ovarian cancer,
   wherein the specific microRNAs is
   (xii) any of the microRNAs listed in Table 4-11 {may be one or more, two or more, three or more, four or more, five or more, 10 or more, 15 or more, or 20 or more microRNAs}.
[56] The method according to [43] or [44] above,
   wherein the urine is the urine of a subject having or suspected of having thyroid cancer,
   wherein the specific microRNAs is
   (xiii) any of the microRNAs listed in Table 4-12 {may be one or more, two or more, three or more, four or more, five or more, 10 or more, 15 or more, or 20 or more microRNAs}.
[57] The method according to [43] or [44] above,
   wherein the urine is the urine of a subject having or suspected of having cervical cancer,
   wherein the specific microRNAs is
   (xiii) any of the microRNAs listed in Table 4-13 {may be one or more, two or more, three or more, four or more, five or more, 10 or more, 15 or more, or 20 or more microRNAs}.
[58] The method according to [43] or [44] above,
   wherein the urine is the urine of a subject having or suspected of having colorectal cancer,
   wherein the specific microRNAs is
   (xiii) any of the microRNAs listed in Table 4-14 {may be one or more, two or more, three or more, four or more, five or more, 10 or more, 15 or more, or 20 or more microRNAs}.
[59] The method according to [43] or [44] above,
   wherein the urine is the urine of a subject having or suspected of having endometrial cancer,
   wherein the specific microRNAs is
   (xiii) any of the microRNAs listed in Table 4-15 {may be one or more, two or more, three or more, four or more, five or more, 10 or more, 15 or more, or 20 or more microRNAs}.
[60] The method according to [45] to [59] above, wherein the microRNA has a p value less than 0.005 {In each table, the microRNA may be one or more, two or more, three or more, four or more, five or more, 10 or more, 15 or more, or 20 or more microRNAs
[61] A method of analyzing a urine sample, comprising:
   contacting a urine sample with a nanowire;
   extracting microRNAs captured on the nanowire; and
   confirming whether the extracted microRNAs contain a specific microRNA,
   wherein the specific microRNAs is
      (a) the microRNAs listed in any one of Tables 22-1 and 23-1 to 23-14 {in each table, each may independently be one or more, two or more, three or more, four or more, five or more, 10 or more, 15 or more, or 20 or more microRNAs}.
[62] The method of analyzing a urine sample according to [61] above,
   wherein the specific microRNAs is
   (b) the microRNAs listed in any one of Tables 22-1 to 22-13 {in each table, each may independently be one or more, two or more, three or more, four or more, five or more, 10 or more, 15 or more, or 20 or more microRNAs}.
[63] The method of analyzing a urine sample according to [61] above,
   wherein the specific microRNAs is
      (a) the microRNAs listed in any one of Tables 22-1 and 23-1 to 23-14 {in each table, each may independently be one or more, two or more, three or more, four or more, five or more, 10 or more, 15 or more, or 20 or more microRNAs},
   wherein the microRNA has an accuracy of 70% or higher.
[64] The method of analyzing a urine sample according to [61] above,
   wherein the specific microRNAs is
      (a) the microRNAs listed in any one of Tables 22-1 and 23-1 to 23-14 {in each table, each may independently be one or more, two or more, three or more, four or more, five or more, 10 or more, 15 or more, or 20 or more microRNAs},
   wherein the microRNA has an accuracy of 80% or higher.
[65] The method of analyzing a urine sample according to [61] above,
   wherein the specific microRNAs is
      (a) the microRNAs listed in any one of Tables 22-1 and 23-1 to 23-14 {in each table, each may independently be one or more, two or more, three or more, four or more, five or more, 10 or more, 15 or more, or 20 or more microRNAs},
   wherein the microRNA has an accuracy of an accuracy of 90% or higher.
[66] The method of analyzing a urine sample according to [61] above,
   wherein the specific microRNAs is
   (b) the microRNAs listed in any one of Tables 22-1 to 22-13 {in each table, each may independently be one or more, two or more, three or more, four or more, five or more, 10 or more, 15 or more, or 20 or more microRNAs},
   wherein the microRNA has an accuracy of 70% or higher.
[67] The method of analyzing a urine sample according to [62] above,
   wherein the specific microRNAs is
   (b) the microRNAs listed in any one of Tables 22-1 to 22-13 {in each table, each may independently be one or more, two or more, three or more, four or more, five or more, 10 or more, 15 or more, or
   20 or more microRNAs},
   wherein the microRNA has an accuracy of 80% or higher.
[68] The method of analyzing a urine sample according to [62] above,
   wherein the specific microRNAs is
   (b) the microRNAs listed in any one of Tables 22-1 to 22-13 {in each table, each may independently be one or more, two or more, three or more, four or more, five or more, 10 or more, 15 or more, or 20 or more microRNAs},
   wherein the microRNA has an accuracy of 90% or higher.
[69] A method of analyzing a urine sample, comprising:
   contacting a urine sample with a nanowire;
   extracting microRNAs captured on the nanowire; and
   confirming whether the extracted microRNAs contain a specific microRNA by contacting the microRNA with a nucleic acid containing a sequence which hybridizes to the microRNA,
   wherein the specific microRNAs is
   (c) the microRNAs listed in any one of Tables 22-1 to 22-13 and Tables 23-1 to 23-14 {in each table, each may independently be one or more, two or more, three or more, four or more, five or more, 10 or more, 15 or more, or 20 or more microRNAs}.
[70] The method of analyzing urine according to [69] above,
   wherein the specific microRNAs is
   (c) the microRNAs listed in any one of Tables 22-1 to 22-13 and Tables 23-1 to 23-14 {in each table, each may independently be one or more, two or more, three or more, four or more, five or more, 10 or more, 15 or more, or 20 or more microRNAs}, and is microRNA having an accuracy of 70% or higher in the table.
[71] The method for analyzing urine according to [69] above,
   wherein the specific microRNAs is
   (c) the microRNAs listed in any one of Tables 22-1 to 22-13 and Tables 23-1 to 23-14 {in each table, each may independently be one or more, two or more, three or more, four or more, five or more, 10 or more, 15 or more, or 20 or more microRNAs},
   and is microRNA having an accuracy of 70% or higher in the table.
[72] The method for analyzing urine according to [69] above,
   wherein the specific microRNAs is
   (c) the microRNAs listed in any one of Tables 22-1 to 22-13 and Tables 23-1 to 23-14 {in each table, each may independently be one or more, two or more, three or more, four or more, five or more, 10 or more, 15 or more, or 20 or more microRNAs},
   and is microRNA having an accuracy of 90% or higher in the table.
[73] A method for analyzing a urine sample, comprising:
   contacting a urine sample with a nanowire in a nanowire-embedded fluidic device having nanowires of metal oxide (e.g., zinc oxide);
   extracting microRNAs captured on the nanowire; and
   confirming whether the extracted microRNAs contain a specific microRNA by contacting the microRNA with a nucleic acid containing a sequence which hybridizes to the microRNA, wherein the specific microRNAs is selected from the group (ALL) consisting of miR-134-5p, miR-346, miR-564, miR-574-3p, miR-575, miR-632, miR-661, miR-663a, miR-658, miR-297, miR-139-3p, miR-149-3p, miR-20b-3p, miR-431-3p, miR-550a-5p, miR-885-5p, miR-936, miR-937-3p, miR-943, miR-1228-5p, miR-1184, miR-1204, miR-1538, miR-1909-5p, miR-1910-5p, miR-1912-3p, miR-196b-3p, miR-1972, miR-3153, miR-3162-5p, miR-1193, miR-4260, miR-4253, miR-4326, miR-4269, miR-4276, miR-3622a-3p, miR-3646, miR-3652, miR-3180, miR-550b-3p, miR-4428, miR-4433a-3p, miR-4440, miR-4444, miR-4447, miR-4449, miR-4455, miR-3155b, miR-4483, miR-4498, miR-2392, miR-4535, miR-4539, miR-3173-5p, miR-4529-5p, miR-4638-5p, miR-4655-5p, miR-4685-3p, miR-1343-3p, miR-4701-3p, miR-4717-3p, miR-4725-5p, miR-4726-3p, miR-4732-5p, miR-4738-3p, miR-4748, miR-4750-5p, miR-371b-5p, miR-4436b-5p, miR-4793-3p, miR-5001-3p, miR-5002-3p, miR-5006-5p, miR-5090, miR-5572, miR-5584-3p, miR-5705, miR-1237-5p, miR-6072, miR-6131, miR-6503-3p, miR-6511b-5p, miR-1296-3p, miR-5189-3p, miR-6728-5p, miR-6729-5p, miR-6736-3p, miR-6738-5p, miR-6738-3p, miR-6740-3p, miR-6753-5p, miR-6754-3p, miR-6756-5p, miR-6759-5p, miR-6765-5p, miR-6768-5p, miR-6772-3p, miR-6773-3p, miR-6787-3p, miR-6790-5p, miR-6792-5p, miR-6798-5p, miR-6804-3p, miR-6805-5p, miR-6809-3p, miR-6816-5p, miR-6820-5p, miR-6821-5p, miR-6821-3p, miR-6822-5p, miR-6830-3p, miR-6832-3p, miR-6833-5p, miR-6833-3p, miR-6834-5p, miR-6780b-3p, miR-6854-3p, miR-6862-5p, miR-6867-5p, miR-6868-3p, miR-6871-5p, miR-6875-3p, miR-6877-5p, miR-6878-3p, miR-6882-3p, miR-6883-5p, miR-6883-3p, miR-6893-3p, miR-6894-3p, miR-7109-3p, miR-7152-5p, miR-7160-5p, miR-7843-5p, miR-8059, miR-8077, miR-7977, miR-4485-5p, miR-8485, miR-9718, miR-10396a-5p, miR-10398-5p, miR-10396b-5p, and miR-11400.
[74] A method for analyzing a urine sample, comprising:
   contacting a urine sample with a nanowire in a nanowire-embedded fluidic device having nanowires of metal oxide (e.g., zinc oxide);
   extracting microRNAs captured on the nanowire; and
   confirming whether the extracted microRNAs contain a specific microRNA by contacting the microRNA with a nucleic acid containing a sequence which hybridizes to the microRNA, wherein the specific microRNAs is selected from the group (GI) consisting of let-7e-5p, miR-23a-3p, miR-24-3p, miR-25-3p, miR-28-5p, miR-29a-3p, miR-30a-3p, miR-92a-3p, miR-29b-3p, miR-105-5p, miR-192-5p, miR-199a-5p, miR-129-5p, miR-30c-5p, miR-181a-5p, miR-181b-5p, miR-182-5p, miR-187-3p, miR-205-5p, miR-210-3p, miR-211-5p, miR-212-3p, miR-214-3p, miR-221-3p, miR-200b-3p, let-7i-5p, miR-15b-5p, miR-23b-3p, miR-27b-3p, miR-138-5p, miR-143-3p, miR-134-5p, miR-146a-5p, miR-154-5p, miR-184, miR-194-5p, miR-34b-5p, miR-34c-5p, miR-299-3p, miR-99b-5p, miR-296-5p, miR-361-5p, miR-370-3p, miR-371a-3p, miR-373-5p, miR-378a-3p, miR-381-3p, miR-382-5p, miR-340-3p, miR-330-3p, miR-328-3p, miR-342-3p, miR-323a-3p, miR-338-3p, miR-325, miR-346, miR-196b-5p, miR-422a, miR-449a, miR-200a-5p, miR-433-3p, miR-323b-5p, miR-484, miR-485-5p, miR-485-3p, miR-486-5p, miR-487a-3p, miR-488-5p, miR-489-3p, miR-491-5p, miR-511-5p, miR-202-3p, miR-492, miR-432-5p, miR-494-3p, miR-497-5p, miR-512-5p, miR-512-3p, miR-498-5p, miR-515-5p, miR-515-3p, miR-519e-3p, miR-520a-3p, miR-526b-5p, miR-525-5p, miR-525-3p, miR-517a-3p, miR-517b-3p, miR-519d-3p, miR-521, miR-520d-3p, miR-520g-3p, miR-516b-3p, miR-516a-3p, miR-517c-3p, miR-520h, miR-519a-3p, miR-500a-3p, miR-501-5p, miR-513a-5p, miR-506-3p, miR-509-3p, miR-510-5p, miR-299-5p, miR-18a-3p, miR-493-3p, miR-487b-3p, miR-551a, miR-554, miR-92b-3p, miR-557, miR-558, miR-564, miR-551b-3p, miR-572, miR-574-3p, miR-575, miR-579-3p, miR-584-5p, miR-589-3p, miR-550a-3p, miR-591, miR-593-5p, miR-595, miR-601, miR-602, miR-603, miR-604, miR-608, miR-609, miR-610, miR-612, miR-614, miR-615-3p, miR-616-5p, miR-617, miR-622, miR-623, miR-628-3p, miR-629-3p, miR-631, miR-632, miR-634, miR-635, miR-636, miR-637, miR-638, miR-639, miR-642a-5p, miR-644a, miR-645, miR-649, miR-650, miR-661, miR-663a, miR-449b-5p, miR-654-5p, miR-657, miR-658, miR-542-5p, miR-363-5p, miR-671-5p, miR-668-3p, miR-454-5p, miR-769-5p, miR-769-3p, miR-766-3p, miR-765, miR-770-5p, miR-675-5p, miR-297, let-7d-3p, miR-15a-3p, miR-16-1-3p, miR-21-3p, miR-23a-5p, miR-25-5p, miR-26a-1-3p, miR-26b-3p, miR-92a-2-5p, miR-16-2-3p, miR-139-3p, miR-7-1-3p, miR-181a-2-3p, miR-181c-3p, miR-183-3p, miR-187-5p, miR-214-5p, miR-222-5p, miR-200b-5p, miR-15b-3p, miR-30b-3p, miR-124-5p, miR-125b-1-3p, miR-135a-3p, miR-138-2-3p, miR-141-5p, miR-125a-3p, miR-125b-2-3p, miR-138-1-3p, miR-149-3p, miR-185-3p, miR-186-3p, miR-193a-5p, miR-200c-5p, miR-194-3p, miR-106b-3p, miR-29c-5p, miR-219a-2-3p, miR-34b-3p, miR-34c-3p, miR-99b-3p, miR-296-3p, miR-130b-5p, miR-371a-5p, miR-377-5p, miR-330-5p, miR-342-5p, miR-323a-5p, miR-151a-5p, miR-338-5p, miR-339-3p, miR-335-3p, miR-423-5p, miR-424-3p, miR-18b-3p, miR-20b-3p, miR-431-3p, miR-483-5p, miR-486-3p, miR-146b-3p, miR-193b-5p, miR-500a-5p, miR-505-5p, miR-508-5p, miR-532-3p, miR-92b-5p, miR-551b-5p, miR-574-5p, miR-589-5p, miR-550a-5p, miR-593-3p, miR-615-5p, miR-625-3p, miR-629-5p, miR-411-3p, miR-654-3p, miR-671-3p, miR-891a-5p, miR-300, miR-892a, miR-874-3p, miR-888-3p, miR-892b, miR-541-5p, miR-541-3p, miR-875-3p, miR-708-5p, miR-147b-3p, miR-744-5p, miR-744-3p, miR-885-5p, miR-885-3p, miR-877-5p, miR-877-3p, miR-887-3p, miR-665, miR-760, miR-920, miR-921, miR-935, miR-936, miR-937-3p, miR-938, miR-939-5p, miR-940, miR-943, miR-1224-5p, miR-1224-3p, miR-1225-5p, miR-1225-3p, miR-1226-5p, miR-1228-5p, miR-1228-3p, miR-1229-3p, miR-1231, miR-1233-3p, miR-1234-3p, miR-1236-3p, miR-1237-3p, miR-1238-3p, miR-513b-5p, miR-513c-5p, miR-320c, miR-1271-5p, miR-1298-5p, miR-1178-3p, miR-1181, miR-1182, miR-1184, miR-1203, miR-663b, miR-1204, miR-1207-5p, miR-1207-3p, miR-1285-3p, miR-1286, miR-1287-5p, miR-1291, miR-1293, miR-1294, miR-1297, miR-1303, miR-1304-5p, miR-1249-3p, miR-1250-5p, miR-1260a, miR-1263, miR-1265, miR-1266-5p, miR-1268a, miR-1269a, miR-1270, miR-1276, miR-1281, miR-1284, miR-1288-3p, miR-1292-5p, miR-1306-3p, miR-1307-3p, miR-1321, miR-1322, miR-1825, miR-1468-5p, miR-1469, miR-1470, miR-1471, miR-1538, miR-103b, miR-1908-5p, miR-1909-5p, miR-1909-3p, miR-1910-5p, miR-1911-3p, miR-1912-3p, miR-1913, miR-1914-3p, miR-1915-5p, miR-1915-3p, miR-103a-2-5p, miR-224-3p, miR-365a-5p, miR-196b-3p, miR-449b-3p, miR-2113, miR-1972, miR-1976, miR-2110, miR-151b, miR-449c-5p, miR-762, miR-761, miR-764, miR-548q, miR-2276-3p, miR-711, miR-718, miR-2681-5p, miR-2682-5p, miR-2682-3p, miR-449c-3p, miR-2861, miR-2909, miR-3122, miR-3124-5p, miR-3125, miR-3127-5p, miR-3130-3p, miR-3130-5p, miR-378b, miR-466, miR-3137, miR-3138, miR-3141, miR-3142, miR-1273c, miR-3147, miR-3148, miR-3150a-3p, miR-3151-5p, miR-3153, miR-3074-3p, miR-3154, miR-3155a, miR-3158-3p, miR-3160-3p, miR-3161, miR-3162-5p, miR-3163, miR-3165, miR-1260b, miR-3170, miR-3173-3p, miR-1193, miR-3174, miR-3175, miR-3176, miR-3178, miR-3180-3p, miR-3185, miR-3186-5p, miR-3186-3p, miR-3187-3p, miR-3189-3p, miR-320e, miR-3191-3p, miR-3192-5p, miR-3193, miR-3194-5p, miR-3195, miR-3196, miR-3197, miR-3198, miR-3199, miR-3200-3p, miR-514b-5p, miR-514b-3p, miR-3126-3p, miR-3065-3p, miR-4296, miR-4297, miR-378c, miR-4293, miR-4294, miR-4299, miR-4298, miR-4300, miR-4304, miR-4303, miR-4305, miR-4308, miR-4311, miR-4315, miR-4316, miR-4314, miR-4318, miR-4319, miR-4317, miR-4322, miR-4321, miR-4323, miR-4324, miR-4257, miR-4258, miR-4259, miR-4260, miR-4253, miR-4254, miR-4255, miR-4252, miR-4326, miR-4327, miR-4261, miR-4265, miR-4262, miR-4268, miR-4269, miR-4264, miR-4271, miR-4273, miR-4276, miR-4274, miR-4279, miR-4278, miR-4280, miR-4285, miR-4283, miR-4287, miR-4289, miR-4329, miR-4328, miR-2277-5p, miR-3605-5p, miR-3610, miR-3614-5p, miR-3615, miR-3616-3p, miR-3619-5p, miR-3620-3p, miR-3621, miR-3622a-5p, miR-3622a-3p, miR-3622b-5p, miR-3646, miR-3648, miR-3649, miR-3650, miR-3651, miR-3652, miR-3654, miR-3655, miR-3657, miR-3659, miR-3660, miR-3661, miR-3663-5p, miR-3663-3p, miR-3665, miR-3666, miR-3667-5p, miR-3675-5p, miR-3675-3p, miR-3677-3p, miR-3678-3p, miR-3679-5p, miR-3679-3p, miR-3680-3p, miR-3681-3p, miR-3682-3p, miR-3685, miR-3689a-3p, miR-3690, miR-3691-5p, miR-3692-5p, miR-3713, miR-3180, miR-3907, miR-3689b-3p, miR-3689c, miR-3911, miR-3913-5p, miR-3917, miR-3918, miR-3919, miR-3150b-3p, miR-3925-5p, miR-676-5p, miR-3928-3p, miR-3929, miR-3934-5p, miR-3936, miR-3937, miR-3940-3p, miR-3944-3p, miR-374c-5p, miR-642b-3p, miR-550b-3p, miR-1268b, miR-378e, miR-4420, miR-4421, miR-378g, miR-4425, miR-4428, miR-4429, miR-4430, miR-4431, miR-4433a-3p, miR-4435, miR-4436a, miR-4437, miR-4438, miR-4439, miR-4440, miR-4441, miR-4444, miR-4445-3p, miR-4446-3p, miR-4447, miR-4448, miR-4449, miR-4450, miR-4451, miR-4453, miR-4454, miR-4455, miR-4456, miR-4458, miR-3135b, miR-4462, miR-4463, miR-4465, miR-4466, miR-4467, miR-4470, miR-4472, miR-4473, miR-4474-3p, miR-4475, miR-4476, miR-4478, miR-3689d, miR-3689f, miR-4479, miR-3155b, miR-4481, miR-4483, miR-4484, miR-4486, miR-4487, miR-4488, miR-4489, miR-4491, miR-4492, miR-4494, miR-4495, miR-4497, miR-4498, miR-4502, miR-4505, miR-4506, miR-2392, miR-4507, miR-4508, miR-4510, miR-4511, miR-4513, miR-4514, miR-4515, miR-4516, miR-4521, miR-4523, miR-4524a-5p, miR-4526, miR-4527, miR-4529-3p, miR-4530, miR-4533, miR-378i, miR-4535, miR-1587, miR-4537, miR-4538, miR-4539, miR-4540, miR-3120-5p, miR-3129-3p, miR-3145-5p, miR-3150a-5p, miR-3152-5p, miR-3074-5p, miR-3157-3p, miR-3158-5p, miR-3160-5p, miR-3173-5p, miR-3187-5p, miR-3691-3p, miR-3913-3p, miR-3922-5p, miR-3925-3p, miR-3940-5p, miR-3944-5p, miR-4423-5p, miR-4520-5p, miR-4529-5p, miR-3960, miR-3975, miR-4634, miR-4635, miR-4638-5p, miR-4639-3p, miR-4640-5p, miR-4641, miR-4642, miR-4644, miR-4645-3p, miR-4646-5p, miR-4646-3p, miR-4647, miR-4648, miR-4649-5p, miR-4650-3p, miR-4651, miR-4652-5p, miR-4653-5p, miR-4654, miR-4655-5p, miR-4656, miR-4657, miR-4658, miR-4661-3p, miR-4664-5p, miR-4664-3p, miR-4665-5p, miR-4665-3p, miR-4667-5p, miR-4667-3p, miR-219b-5p, miR-4669, miR-4670-5p, miR-4672, miR-4673, miR-4674, miR-4676-5p, miR-4676-3p, miR-4681, miR-4682, miR-4684-5p, miR-4684-3p, miR-4685-5p, miR-4685-3p, miR-4686, miR-4687-5p, miR-4687-3p, miR-1343-3p, miR-4688, miR-4690-5p, miR-4691-5p, miR-4691-3p, miR-4692, miR-4694-3p, miR-4695-5p, miR-4697-5p, miR-4697-3p, miR-4698, miR-4700-5p, miR-4700-3p, miR-4701-5p, miR-4701-3p, miR-4706, miR-4707-5p, miR-4707-3p, miR-4708-5p, miR-4708-3p, miR-4709-5p, miR-4709-3p, miR-4710, miR-4711-3p, miR-4712-5p, miR-4713-5p, miR-4713-3p, miR-4716-5p, miR-4716-3p, miR-3529-5p, miR-4717-5p, miR-4717-3p, miR-4718, miR-4721, miR-4722-5p, miR-4722-3p, miR-4723-3p, miR-4724-5p, miR-4724-3p, miR-4725-5p, miR-4725-3p, miR-4726-5p, miR-4726-3p, miR-4727-3p, miR-4728-3p, miR-4730, miR-4731-5p, miR-4731-3p, miR-4732-5p, miR-4732-3p, miR-4733-5p, miR-4733-3p, miR-4734, miR-4736, miR-4737, miR-3064-5p, miR-3064-3p, miR-4738-3p, miR-4739, miR-4740-5p, miR-4741, miR-4743-5p, miR-122b-3p, miR-4745-5p, miR-4746-3p, miR-4747-5p, miR-4747-3p, miR-4748, miR-4749-5p, miR-4749-3p, miR-4750-5p, miR-4753-3p, miR-371b-5p, miR-371b-3p, miR-4754, miR-4755-3p, miR-4756-5p, miR-4756-3p, miR-4758-5p, miR-4758-3p, miR-4759, miR-4761-3p, miR-4763-5p, miR-4763-3p, miR-4764-3p, miR-4766-5p, miR-4767, miR-4768-5p, miR-4768-3p, miR-4769-5p, miR-4769-3p, miR-4773, miR-4776-5p, miR-4776-3p, miR-4778-5p, miR-4780, miR-4436b-5p, miR-4436b-3p, miR-4783-5p, miR-4783-3p, miR-4784, miR-4785, miR-2467-3p, miR-4786-5p, miR-4786-3p, miR-4787-5p, miR-4787-3p, miR-4788, miR-4793-5p, miR-4793-3p, miR-4794, miR-4796-5p, miR-4797-3p, miR-4800-5p, miR-4800-3p, miR-4801, miR-4804-3p, miR-642a-3p, miR-550a-3-5p, miR-4433a-5p, miR-4482-3p, miR-4999-5p, miR-4999-3p, miR-5000-5p, miR-5001-5p, miR-5001-3p, miR-5002-5p, miR-5002-3p, miR-5004-5p, miR-5004-3p, miR-5006-5p, miR-5006-3p, miR-5007-5p, miR-5008-5p, miR-5010-5p, miR-5088-5p, miR-5090, miR-5091, miR-5093, miR-5187-5p, miR-5188, miR-5189-5p, miR-5190, miR-5192, miR-5194, miR-5196-5p, miR-5196-3p, miR-5197-5p, miR-4524b-5p, miR-4524b-3p, miR-5571-3p, miR-5100, miR-5572, miR-664b-5p, miR-664b-3p, miR-5580-5p, miR-5580-3p, miR-5581-3p, miR-5584-3p, miR-5585-3p, miR-1295b-3p, miR-5588-5p, miR-5588-3p, miR-5589-5p, miR-5591-3p, miR-5684, miR-5685, miR-5681b, miR-5690, miR-5694, miR-5696, miR-5698, miR-5702, miR-5703, miR-5704, miR-5705, miR-5708, miR-197-5p, miR-204-3p, miR-211-3p, miR-212-5p, miR-301a-5p, miR-345-3p, miR-584-3p, miR-652-5p, miR-660-3p, miR-1185-2-3p, miR-766-5p, miR-1285-5p, miR-1304-3p, miR-1247-3p, miR-1255b-2-3p, miR-1306-5p, miR-3184-3p, miR-3191-5p, miR-642b-5p, miR-550b-2-5p, miR-365b-5p, miR-1185-1-3p, miR-3190-3p, miR-216a-3p, miR-381-5p, miR-495-5p, miR-503-3p, miR-937-5p, miR-939-3p, miR-1227-5p, miR-1229-5p, miR-1233-5p, miR-1236-5p, miR-1237-5p, miR-1238-5p, miR-3617-3p, miR-3620-5p, miR-3927-5p, miR-3934-3p, miR-4632-5p, miR-4743-3p, miR-5089-3p, miR-5739, miR-5787, miR-6068, miR-6069, miR-6071, miR-6072, miR-6074, miR-6075, miR-6076, miR-6078, miR-6080, miR-6081, miR-6083, miR-6085, miR-6086, miR-6088, miR-6089, miR-6090, miR-6124, miR-6125, miR-6126, miR-6127, miR-6129, miR-6130, miR-6131, miR-6132, miR-6133, miR-6134, miR-6165, miR-6499-5p, miR-6499-3p, miR-548ay-3p, miR-6500-5p, miR-6500-3p, miR-6501-5p, miR-6501-3p, miR-6502-5p, miR-6503-3p, miR-6504-5p, miR-6504-3p, miR-6505-3p, miR-6506-5p, miR-6508-5p, miR-6509-5p, miR-6510-5p, miR-6511a-5p, miR-6512-3p, miR-6513-5p, miR-6513-3p, miR-6514-5p, miR-6514-3p, miR-6515-5p, miR-6515-3p, miR-6715a-3p, miR-6715b-5p, miR-6716-5p, miR-6716-3p, miR-6717-5p, miR-6511b-5p, miR-6511b-3p, miR-6718-5p, miR-6719-3p, miR-6721-5p, miR-6722-5p, miR-6722-3p, miR-6724-5p, miR-892c-5p, miR-892c-3p, miR-208a-5p, miR-210-5p, miR-128-1-5p, miR-134-3p, miR-370-5p, miR-328-5p, miR-433-5p, miR-487a-5p, miR-489-5p, miR-494-5p, miR-504-3p, miR-487b-5p, miR-579-5p, miR-585-5p, miR-598-5p, miR-619-5p, miR-656-5p, miR-668-5p, miR-1296-3p, miR-1301-5p, miR-1298-3p, miR-891a-3p, miR-874-5p, miR-889-5p, miR-887-5p, miR-216b-3p, miR-942-3p, miR-1180-5p, miR-1287-3p, miR-1251-3p, miR-1266-3p, miR-1288-5p, miR-1908-3p, miR-1910-3p, miR-3151-3p, miR-3192-3p, miR-500b-3p, miR-3928-5p, miR-1343-5p, miR-5088-3p, miR-5189-3p, miR-6720-5p, miR-6726-5p, miR-6726-3p, miR-6727-5p, miR-6727-3p, miR-6728-5p, miR-6728-3p, miR-6729-5p, miR-6729-3p, miR-6730-5p, miR-6730-3p, miR-6731-5p, miR-6732-5p, miR-6732-3p, miR-6733-3p, miR-6734-5p, miR-6735-5p, miR-6735-3p, miR-6736-3p, miR-6737-5p, miR-6738-5p, miR-6738-3p, miR-6739-3p, miR-6740-5p, miR-6740-3p, miR-6741-5p, miR-6741-3p, miR-6742-5p, miR-6743-5p, miR-6743-3p, miR-6744-3p, miR-6747-5p, miR-6747-3p, miR-6748-5p, miR-6748-3p, miR-6749-5p, miR-6749-3p, miR-6750-5p, miR-6750-3p, miR-6751-5p, miR-6751-3p, miR-6752-5p, miR-6752-3p, miR-6753-5p, miR-6754-5p, miR-6754-3p, miR-6755-5p, miR-6755-3p, miR-6756-5p, miR-6756-3p, miR-6758-5p, miR-6758-3p, miR-6759-5p, miR-6759-3p, miR-6760-5p, miR-6760-3p, miR-6761-5p, miR-6762-5p, miR-6762-3p, miR-6763-5p, miR-6763-3p, miR-6764-5p, miR-6764-3p, miR-6765-5p, miR-6766-5p, miR-6766-3p, miR-6767-5p, miR-6768-5p, miR-6769a-5p, miR-6769a-3p, miR-6770-5p, miR-6770-3p, miR-6771-5p, miR-6772-5p, miR-6772-3p, miR-6773-3p, miR-6774-5p, miR-6774-3p, miR-6775-5p, miR-6776-5p, miR-6776-3p, miR-6777-5p, miR-6777-3p, miR-6778-5p, miR-6778-3p, miR-6779-5p, miR-6779-3p, miR-6781-5p, miR-6781-3p, miR-6782-5p, miR-6782-3p, miR-6783-5p, miR-6783-3p, miR-6784-5p, miR-6784-3p, miR-6785-3p, miR-6786-5p, miR-6786-3p, miR-6787-5p, miR-6787-3p, miR-6788-5p, miR-6789-5p, miR-6789-3p, miR-6790-5p, miR-6791-5p, miR-6791-3p, miR-6792-5p, miR-6792-3p, miR-6793-5p, miR-6794-5p, miR-6796-5p, miR-6796-3p, miR-6797-5p, miR-6797-3p, miR-6798-5p, miR-6798-3p, miR-6799-5p, miR-6800-5p, miR-6800-3p, miR-6801-5p, miR-6801-3p, miR-6802-3p, miR-6803-5p, miR-6803-3p, miR-6804-5p, miR-6804-3p, miR-6805-5p, miR-6805-3p, miR-6806-5p, miR-6807-5p, miR-6808-5p, miR-6809-5p, miR-6809-3p, miR-6810-5p, miR-6810-3p, miR-6812-5p, miR-6813-5p, miR-6813-3p, miR-6814-5p, miR-6815-3p, miR-6816-5p, miR-6816-3p, miR-6817-5p, miR-6817-3p, miR-6818-3p, miR-6819-3p, miR-6820-5p, miR-6820-3p, miR-6821-5p, miR-6821-3p, miR-6822-5p, miR-6822-3p, miR-6823-5p, miR-6823-3p, miR-6824-5p, miR-6824-3p, miR-6825-5p, miR-6825-3p, miR-6826-3p, miR-6827-5p, miR-6829-5p, miR-6829-3p, miR-6830-5p, miR-6830-3p, miR-6831-5p, miR-6832-5p, miR-6832-3p, miR-6833-5p, miR-6833-3p, miR-6834-5p, miR-6780b-5p, miR-6780b-3p, miR-6836-5p, miR-6836-3p, miR-6837-5p, miR-6838-5p, miR-6839-5p, miR-6840-3p, miR-6842-5p, miR-6842-3p, miR-6843-3p, miR-6845-5p, miR-6845-3p, miR-6846-5p, miR-6846-3p, miR-6847-5p, miR-6847-3p, miR-6848-5p, miR-6848-3p, miR-6849-5p, miR-6849-3p, miR-6850-5p, miR-6850-3p, miR-6851-5p, miR-6852-5p, miR-6852-3p, miR-6853-5p, miR-6854-3p, miR-6855-5p, miR-6855-3p, miR-6856-5p, miR-6856-3p, miR-6857-5p, miR-6858-5p, miR-6858-3p, miR-6859-5p, miR-6859-3p, miR-6769b-5p, miR-6860, miR-6861-5p, miR-6861-3p, miR-6862-5p, miR-6862-3p, miR-6863, miR-6866-3p, miR-6867-5p, miR-6867-3p, miR-6868-5p, miR-6868-3p, miR-6869-5p, miR-6870-5p, miR-6870-3p, miR-6871-5p, miR-6873-5p, miR-6874-5p, miR-6874-3p, miR-6875-5p, miR-6875-3p, miR-6876-5p, miR-6876-3p, miR-6877-5p, miR-6877-3p, miR-6878-3p, miR-6879-5p, miR-6879-3p, miR-6880-5p, miR-6880-3p, miR-6881-5p, miR-6882-3p, miR-6883-5p, miR-6883-3p, miR-6884-5p, miR-6884-3p, miR-6885-3p, miR-6886-5p, miR-6886-3p, miR-6887-3p, miR-6888-5p, miR-6889-5p, miR-6889-3p, miR-6890-5p, miR-6890-3p, miR-6891-5p, miR-6891-3p, miR-6892-5p, miR-6892-3p, miR-6893-5p, miR-6893-3p, miR-6894-5p, miR-6894-3p, miR-6895-5p, miR-6895-3p, miR-7106-5p, miR-7106-3p, miR-7107-5p, miR-7108-5p, miR-7108-3p, miR-7109-5p, miR-7109-3p, miR-7110-5p, miR-7110-3p, miR-7111-5p, miR-7111-3p, miR-7112-5p, miR-7113-5p, miR-7113-3p, miR-7114-5p, miR-7114-3p, miR-7150, miR-7151-5p, miR-7151-3p, miR-7152-5p, miR-7154-3p, miR-7155-5p, miR-7155-3p, miR-7156-5p, miR-7156-3p, miR-7158-5p, miR-7160-5p, miR-7162-3p, miR-7515, miR-7702, miR-7704, miR-7706, miR-7843-5p, miR-4433b-5p, miR-4433b-3p, miR-1273h-5p, miR-6516-5p, miR-7844-5p, miR-7845-5p, miR-7846-3p, miR-7847-3p, miR-7848-3p, miR-7850-5p, miR-7851-3p, miR-7853-5p, miR-7854-3p, miR-7856-5p, miR-8052, miR-8055, miR-8057, miR-8059, miR-8060, miR-8062, miR-8063, miR-8064, miR-8069, miR-8070, miR-8071, miR-8072, miR-8073, miR-8074, miR-8075, miR-8077, miR-8078, miR-8079, miR-8080, miR-8081, miR-8082, miR-8083, miR-8085, miR-8089, miR-128-2-5p, miR-7973, miR-7974, miR-7975, miR-7976, miR-7977, miR-1249-5p, miR-4485-5p, miR-8485, miR-9500, miR-103a-1-5p, miR-217-3p, miR-135a-2-3p, miR-375-5p, miR-498-3p, miR-9718, miR-9899, miR-9902, miR-1843, miR-9986, miR-10392-5p, miR-10392-3p, miR-10394-5p, miR-10394-3p, miR-10395-5p, miR-10396a-5p, miR-10396a-3p, miR-10397-5p, miR-10398-5p, miR-10398-3p, miR-10400-5p, miR-10400-3p, miR-10401-5p, miR-10401-3p, miR-10396b-5p, miR-10396b-3p, miR-10522-5p, miR-10523-5p, miR-10527-5p, miR-11181-3p, miR-11399, miR-11400, miR-11401, miR-3059-5p, miR-3059-3p, miR-3085-3p, miR-6529-5p, miR-6529-3p, miR-9851-5p, miR-12114, miR-12115, miR-12116, miR-12117, miR-12118, miR-12119, miR-12120, miR-12121, miR-12122, miR-12124, miR-12125, miR-12127, miR-12128, and miR-12131.
[75] A method for analyzing a urine sample, comprising:
   contacting a urine sample with a nanowire in a nanowire-embedded fluidic device having nanowires of metal oxide (e.g., zinc oxide);
   extracting microRNAs captured on the nanowire; and
   confirming whether the extracted microRNAs contain a specific microRNA by contacting the microRNA with a nucleic acid containing a sequence which hybridizes to the microRNA,
   wherein the specific microRNAs is
   selected from the group (Hemo) consisting of miR-105-5p, miR-192-5p, miR-198, miR-129-5p, miR-187-3p, miR-210-3p, miR-212-3p, miR-214-3p, miR-200b-3p, miR-125b-5p, miR-138-5p, miR-134-5p, miR-184, miR-185-5p, miR-206, miR-106b-5p, miR-34c-5p, miR-99b-5p, miR-296-5p, miR-130b-3p, miR-302c-5p, miR-370-3p, miR-373-3p, miR-378a-3p, miR-382-5p, miR-340-3p, miR-342-3p, miR-337-3p, miR-323a-3p, miR-324-5p, miR-346, miR-422a, miR-425-3p, miR-369-5p, miR-323b-5p, miR-409-3p, miR-412-3p, miR-485-5p, miR-485-3p, miR-488-5p, miR-491-5p, miR-202-3p, miR-492, miR-432-3p, miR-494-3p, miR-512-5p, miR-512-3p, miR-498-5p, miR-520e-3p, miR-515-3p, miR-519c-5p, miR-523-5p, miR-518e-5p, miR-522-5p, miR-519a-5p, miR-519b-5p, miR-526b-5p, miR-525-5p, miR-518f-3p, miR-520b-3p, miR-526a-5p, miR-520c-5p, miR-518d-5p, miR-518c-5p, miR-519d-3p, miR-520d-5p, miR-516b-3p, miR-516a-3p, miR-501-5p, miR-513a-5p, miR-510-5p, miR-18a-3p, miR-551a, miR-92b-3p, miR-558, miR-564, miR-551b-3p, miR-572, miR-574-3p, miR-575, miR-583, miR-550a-3p, miR-595, miR-596, miR-601, miR-602, miR-604, miR-608, miR-610, miR-612, miR-614, miR-615-3p, miR-616-5p, miR-617, miR-622, miR-623, miR-629-3p, miR-632, miR-636, miR-637, miR-638, miR-639, miR-646, miR-650, miR-661, miR-663a, miR-654-5p, miR-657, miR-658, miR-421, miR-542-5p, miR-363-5p, miR-668-3p, miR-769-3p, miR-766-3p, miR-770-5p, miR-297, 1et-7d-3p, miR-15a-3p, miR-21-3p, miR-23a-5p, miR-25-5p, miR-26b-3p, miR-92a-2-5p, miR-30c-2-3p, miR-139-3p, miR-183-3p, miR-187-5p, miR-214-5p, miR-200b-5p, miR-30b-3p, miR-130a-5p, miR-135a-3p, miR-138-2-3p, miR-125a-3p, miR-125b-2-3p, miR-138-1-3p, miR-149-3p, miR-150-3p, miR-185-3p, miR-186-3p, miR-193a-5p, miR-200c-5p, miR-155-3p, miR-194-3p, miR-34b-3p, miR-34c-3p, miR-296-3p, miR-130b-5p, miR-361-3p, miR-302d-5p, miR-377-5p, miR-342-5p, miR-323a-5p, miR-338-5p, miR-424-3p, miR-18b-3p, miR-20b-3p, miR-431-3p, miR-483-5p, miR-486-3p, miR-193b-5p, miR-505-5p, miR-508-5p, miR-92b-5p, miR-551b-5p, miR-574-5p, miR-550a-5p, miR-593-3p, miR-615-5p, miR-625-3p, miR-629-5p, miR-671-3p, miR-891a-5p, miR-300, miR-892b, miR-541-5p, miR-541-3p, miR-744-5p, miR-885-5p, miR-885-3p, miR-877-5p, miR-887-3p, miR-665, miR-760, miR-920, miR-921, miR-935, miR-936, miR-937-3p, miR-938, miR-939-5p, miR-940, miR-943, miR-1224-5p, miR-1224-3p, miR-1225-5p, miR-1225-3p, miR-1226-5p, miR-1228-5p, miR-1228-3p, miR-1231, miR-1233-3p, miR-1237-3p, miR-1238-3p, miR-513b-5p, miR-320b, miR-320c, miR-1271-5p, miR-1301-3p, miR-1298-5p, miR-1178-3p, miR-1181, miR-1182, miR-1184, miR-1203, miR-1204, miR-1207-5p, miR-1285-3p, miR-1293, miR-1303, miR-1249-3p, miR-1250-5p, miR-548g-3p, miR-1266-5p, miR-1268a, miR-1269a, miR-1270, miR-1276, miR-1292-5p, miR-1306-3p, miR-1307-3p, miR-1321, miR-1468-5p, miR-675-3p, miR-1469, miR-1470, miR-1471, miR-1538, miR-1908-5p, miR-1909-5p, miR-1909-3p, miR-1910-5p, miR-1912-3p, miR-1913, miR-1914-3p, miR-1915-3p, miR-103a-2-5p, miR-365a-5p, miR-196b-3p, miR-449b-3p, miR-1972, miR-2110, miR-449c-5p, miR-762, miR-670-5p, miR-761, miR-764, miR-2116-5p, miR-548q, miR-2276-3p, miR-2277-3p, miR-711, miR-718, miR-2682-5p, miR-449c-3p, miR-3122, miR-3124-5p, miR-3126-5p, miR-3130-3p, miR-3130-5p, miR-3131, miR-3132, miR-3133, miR-466, miR-3137, miR-3138, miR-3141, miR-3147, miR-3150a-3p, miR-3151-5p, miR-3153, miR-3074-3p, miR-3155a, miR-3158-3p, miR-3161, miR-3162-5p, miR-3163, miR-3164, miR-3165, miR-1260b, miR-3173-3p, miR-1193, miR-3176, miR-3177-3p, miR-3178, miR-3180-3p, miR-3185, miR-3186-3p, miR-3187-3p, miR-3189-3p, miR-3191-3p, miR-3192-5p, miR-3195, miR-3196, miR-3197, miR-3198, miR-3199, miR-3202, miR-3126-3p, miR-4296, miR-4297, miR-4294, miR-4299, miR-4300, miR-4304, miR-4305, miR-4306, miR-4307, miR-4308, miR-4313, miR-4316, miR-4314, miR-4319, miR-4317, miR-4322, miR-4321, miR-4323, miR-4324, miR-4257, miR-4258, miR-4259, miR-4260, miR-4253, miR-4251, miR-4255, miR-4252, miR-4326, miR-4327, miR-4261, miR-4265, miR-4266, miR-4267, miR-4262, miR-4268, miR-4269, miR-4276, miR-4274, miR-4280, miR-4285, miR-4289, miR-2277-5p, miR-3200-5p, miR-3605-5p, miR-3605-3p, miR-3610, miR-3612, miR-3614-5p, miR-3616-3p, miR-3619-5p, miR-3621, miR-3622a-5p, miR-3622a-3p, miR-3622b-5p, miR-3646, miR-3648, miR-3650, miR-3651, miR-3652, miR-3654, miR-3655, miR-3659, miR-3660, miR-3663-5p, miR-3663-3p, miR-3665, miR-3667-5p, miR-3667-3p, miR-3675-3p, miR-3677-3p, miR-3679-5p, miR-3679-3p, miR-3682-3p, miR-3685, miR-3689a-3p, miR-3691-5p, miR-3713, miR-3714, miR-3180, miR-3689b-3p, miR-3689c, miR-3917, miR-3918, miR-3150b-3p, miR-3925-5p, miR-3928-3p, miR-3934-5p, miR-3935, miR-3936, miR-3937, miR-3944-3p, miR-3945, miR-550b-3p, miR-1268b, miR-4421, miR-4428, miR-4429, miR-4430, miR-4431, miR-4433a-3p, miR-4435, miR-4436a, miR-4439, miR-4440, miR-4441, miR-4443, miR-4444, miR-4446-3p, miR-4447, miR-4448, miR-4449, miR-4450, miR-4451, miR-4453, miR-4454, miR-4455, miR-4456, miR-4458, miR-3135b, miR-4462, miR-4463, miR-4465, miR-4466, miR-4467, miR-4470, miR-4472, miR-4474-3p, miR-4475, miR-4476, miR-3689d, miR-4479, miR-3155b, miR-4481, miR-4483, miR-4484, miR-4485-3p, miR-4486, miR-4487, miR-4488, miR-4489, miR-4492, miR-4496, miR-4497, miR-4498, miR-4499, miR-4502, miR-4505, miR-4506, miR-2392, miR-4507, miR-4508, miR-4510, miR-4512, miR-4513, miR-4514, miR-4515, miR-4516, miR-4521, miR-4525, miR-4526, miR-4530, miR-4531, miR-4533, miR-4534, miR-4535, miR-1587, miR-4538, miR-4539, miR-4540, miR-3120-5p, miR-3150a-5p, miR-3074-5p, miR-3156-3p, miR-3160-5p, miR-3162-3p, miR-3173-5p, miR-3187-5p, miR-3619-3p, miR-3922-5p, miR-3940-5p, miR-3944-5p, miR-4520-5p, miR-4529-5p, miR-3960, miR-4634, miR-4635, miR-4638-5p, miR-4639-3p, miR-4640-5p, miR-4642, miR-4646-5p, miR-4647, miR-4648, miR-4650-3p, miR-4651, miR-4652-5p, miR-4653-5p, miR-4653-3p, miR-4654, miR-4655-5p, miR-4657, miR-4658, miR-4664-5p, miR-4664-3p, miR-4665-5p, miR-4665-3p, miR-4667-5p, miR-219b-5p, miR-4669, miR-4670-5p, miR-4673, miR-4674, miR-4676-5p, miR-4681, miR-4682, miR-4684-5p, miR-4684-3p, miR-4685-3p, miR-4687-5p, miR-1343-3p, miR-4688, miR-4690-5p, miR-4690-3p, miR-4691-5p, miR-4692, miR-4695-5p, miR-4700-5p, miR-4701-3p, miR-4706, miR-4707-5p, miR-4707-3p, miR-4708-5p, miR-4708-3p, miR-203b-5p, miR-4710, miR-4711-3p, miR-4714-5p, miR-4716-5p, miR-4716-3p, miR-4717-5p, miR-4717-3p, miR-4720-5p, miR-4721, miR-4722-3p, miR-4724-5p, miR-4725-5p, miR-4725-3p, miR-4726-3p, miR-4727-3p, miR-4728-5p, miR-4728-3p, miR-4730, miR-4731-3p, miR-4732-5p, miR-4732-3p, miR-4734, miR-4736, miR-3064-5p, miR-3064-3p, miR-4738-3p, miR-4740-5p, miR-4740-3p, miR-4741, miR-4743-5p, miR-122b-3p, miR-4745-5p, miR-4746-3p, miR-4747-5p, miR-4747-3p, miR-4748, miR-4749-5p, miR-4749-3p, miR-4750-5p, miR-371b-5p, miR-4754, miR-4755-3p, miR-4758-5p, miR-4763-3p, miR-4764-3p, miR-4767, miR-4769-5p, miR-4776-5p, miR-4776-3p, miR-4780, miR-4436b-5p, miR-4436b-3p, miR-4781-5p, miR-4783-5p, miR-4783-3p, miR-4785, miR-2467-3p, miR-4786-5p, miR-4786-3p, miR-4787-5p, miR-4793-3p, miR-4800-5p, miR-4800-3p, miR-4801, miR-4804-3p, miR-550a-3-5p, miR-4433a-5p, miR-4482-3p, miR-4999-5p, miR-5001-5p, miR-5001-3p, miR-5002-3p, miR-5004-5p, miR-5004-3p, miR-5006-5p, miR-5009-3p, miR-5010-5p, miR-5088-5p, miR-5090, miR-5093, miR-5187-5p, miR-5188, miR-5189-5p, miR-5190, miR-5193, miR-5195-5p, miR-5196-5p, miR-5571-3p, miR-5100, miR-5572, miR-664b-5p, miR-664b-3p, miR-5580-3p, miR-5581-3p, miR-5584-3p, miR-5585-3p, miR-5587-5p, miR-5588-5p, miR-5588-3p, miR-5589-5p, miR-5685, miR-5681b, miR-5698, miR-5703, miR-5705, miR-197-5p, miR-204-3p, miR-211-3p, miR-301a-5p, miR-345-3p, miR-584-3p, miR-659-5p, miR-1185-2-3p, miR-766-5p, miR-873-3p, miR-1285-5p, miR-1247-3p, miR-1255b-2-3p, miR-3191-5p, miR-642b-5p, miR-550b-2-5p, miR-365b-5p, miR-1185-1-3p, miR-3190-3p, miR-937-5p, miR-1227-5p, miR-1236-5p, miR-1237-5p, miR-1238-5p, miR-1292-3p, miR-3617-3p, miR-3620-5p, miR-3934-3p, miR-4632-5p, miR-4750-3p, miR-5739, miR-5787, miR-6068, miR-6070, miR-6071, miR-6072, miR-6074, miR-6075, miR-6076, miR-6080, miR-6081, miR-6083, miR-6084, miR-6085, miR-6086, miR-6089, miR-6090, miR-6124, miR-6125, miR-6126, miR-6127, miR-6129, miR-6130, miR-6131, miR-6132, miR-6133, miR-6134, miR-6165, miR-6500-5p, miR-6500-3p, miR-6501-5p, miR-6501-3p, miR-6503-3p, miR-6504-3p, miR-6508-5p, miR-6509-3p, miR-6511a-5p, miR-6512-3p, miR-6513-5p, miR-6514-5p, miR-6514-3p, miR-6515-5p, miR-6515-3p, miR-6715b-5p, miR-6716-3p, miR-6717-5p, miR-6511b-5p, miR-6718-5p, miR-6721-5p, miR-6722-5p, miR-6722-3p, miR-6724-5p, miR-210-5p, miR-128-1-5p, miR-134-3p, miR-433-5p, miR-487a-5p, miR-489-5p, miR-504-3p, miR-487b-5p, miR-585-5p, miR-605-3p, miR-619-5p, miR-656-5p, miR-668-5p, miR-1296-3p, miR-1301-5p, miR-1298-3p, miR-874-5p, miR-887-5p, miR-1180-5p, miR-548j-3p, miR-1250-3p, miR-1266-3p, miR-1288-5p, miR-1908-3p, miR-1910-3p, miR-3192-3p, miR-500b-3p, miR-3928-5p, miR-1343-5p, miR-5088-3p, miR-5189-3p, miR-6720-5p, miR-6726-5p, miR-6727-5p, miR-6728-5p, miR-6728-3p, miR-6729-5p, miR-6729-3p, miR-6730-5p, miR-6730-3p, miR-6731-5p, miR-6731-3p, miR-6732-5p, miR-6734-5p, miR-6735-3p, miR-6736-5p, miR-6736-3p, miR-6737-5p, miR-6738-5p, miR-6738-3p, miR-6740-5p, miR-6740-3p, miR-6741-5p, miR-6742-5p, miR-6743-5p, miR-6745, miR-6746-5p, miR-6747-5p, miR-6748-5p, miR-6749-5p, miR-6750-5p, miR-6751-5p, miR-6752-5p, miR-6752-3p, miR-6753-5p, miR-6753-3p, miR-6754-3p, miR-6756-5p, miR-6758-5p, miR-6759-5p, miR-6760-5p, miR-6760-3p, miR-6761-5p, miR-6761-3p, miR-6762-5p, miR-6762-3p, miR-6763-5p, miR-6763-3p, miR-6765-5p, miR-6765-3p, miR-6766-5p, miR-6766-3p, miR-6767-5p, miR-6768-5p, miR-6769a-5p, miR-6770-5p, miR-6770-3p, miR-6772-5p, miR-6772-3p, miR-6773-3p, miR-6774-3p, miR-6776-5p, miR-6776-3p, miR-6777-5p, miR-6777-3p, miR-6778-5p, miR-6778-3p, miR-6779-3p, miR-6780a-5p, miR-6780a-3p, miR-6781-5p, miR-6781-3p, miR-6782-5p, miR-6783-5p, miR-6783-3p, miR-6784-5p, miR-6784-3p, miR-6785-3p, miR-6786-5p, miR-6787-5p, miR-6787-3p, miR-6788-5p, miR-6789-5p, miR-6789-3p, miR-6790-5p, miR-6791-5p, miR-6791-3p, miR-6792-5p, miR-6792-3p, miR-6793-5p, miR-6794-5p, miR-6795-5p, miR-6795-3p, miR-6796-5p, miR-6796-3p, miR-6797-5p, miR-6798-5p, miR-6798-3p, miR-6800-5p, miR-6800-3p, miR-6803-3p, miR-6804-5p, miR-6804-3p, miR-6805-5p, miR-6805-3p, miR-6806-5p, miR-6808-5p, miR-6809-5p, miR-6809-3p, miR-6810-5p, miR-6810-3p, miR-6812-5p, miR-6813-5p, miR-6813-3p, miR-6814-5p, miR-6815-5p, miR-6816-5p, miR-6816-3p, miR-6817-5p, miR-6819-3p, miR-6820-5p, miR-6820-3p, miR-6821-5p, miR-6821-3p, miR-6822-5p, miR-6823-5p, miR-6824-5p, miR-6825-5p, miR-6827-5p, miR-6829-5p, miR-6829-3p, miR-6830-5p, miR-6830-3p, miR-6831-5p, miR-6832-5p, miR-6832-3p, miR-6833-5p, miR-6833-3p, miR-6834-5p, miR-6780b-5p, miR-6780b-3p, miR-6836-5p, miR-6836-3p, miR-6837-5p, miR-6837-3p, miR-6838-5p, miR-6839-5p, miR-6840-3p, miR-6842-5p, miR-6843-3p, miR-6845-5p, miR-6845-3p, miR-6846-5p, miR-6847-5p, miR-6847-3p, miR-6848-5p, miR-6849-5p, miR-6849-3p, miR-6850-5p, miR-6850-3p, miR-6852-5p, miR-6852-3p, miR-6854-3p, miR-6855-5p, miR-6855-3p, miR-6856-5p, miR-6856-3p, miR-6857-5p, miR-6859-5p, miR-6859-3p, miR-6769b-5p, miR-6860, miR-6861-5p, miR-6862-5p, miR-6867-5p, miR-6867-3p, miR-6868-3p, miR-6869-5p, miR-6870-5p, miR-6871-5p, miR-6872-5p, miR-6873-5p, miR-6874-5p, miR-6875-5p, miR-6875-3p, miR-6876-5p, miR-6877-5p, miR-6877-3p, miR-6878-3p, miR-6879-5p, miR-6879-3p, miR-6880-5p, miR-6880-3p, miR-6881-5p, miR-6882-3p, miR-6883-5p, miR-6883-3p, miR-6886-5p, miR-6887-5p, miR-6887-3p, miR-6889-5p, miR-6891-5p, miR-6891-3p, miR-6892-5p, miR-6892-3p, miR-6893-5p, miR-6893-3p, miR-6894-5p, miR-6894-3p, miR-6895-5p, miR-7106-5p, miR-7107-5p, miR-7108-5p, miR-7108-3p, miR-7109-3p, miR-7110-5p, miR-7111-5p, miR-7112-5p, miR-7113-5p, miR-7114-5p, miR-7114-3p, miR-7150, miR-7151-3p, miR-7152-5p, miR-7154-3p, miR-7155-5p, miR-7156-3p, miR-7160-5p, miR-7162-3p, miR-7515, miR-7702, miR-7704, miR-7843-5p, miR-4433b-5p, miR-4433b-3p, miR-1273h-5p, miR-6516-5p, miR-7845-5p, miR-7846-3p, miR-7851-3p, miR-7854-3p, miR-7855-5p, miR-7856-5p, miR-8052, miR-8057, miR-8059, miR-8060, miR-8063, miR-8064, miR-8069, miR-8070, miR-8071, miR-8072, miR-8073, miR-8074, miR-8075, miR-8077, miR-8078, miR-8079, miR-8082, miR-8085, miR-8087, miR-8088, miR-8089, miR-7974, miR-7975, miR-7976, miR-7977, miR-4485-5p, miR-8485, miR-103a-1-5p, miR-196a-1-3p, miR-217-3p, miR-135a-2-3p, miR-9718, miR-9898, miR-9902, miR-1843, miR-10226, miR-10392-5p, miR-10392-3p, miR-10394-3p, miR-10395-5p, miR-10396a-5p, miR-10396a-3p, miR-10398-5p, miR-10398-3p, miR-10400-5p, miR-10400-3p, miR-10401-5p, miR-10401-3p, miR-10396b-5p, miR-10396b-3p, miR-10522-5p, miR-10526-3p, miR-10527-5p, miR-11181-3p, miR-11399, miR-11400, miR-11401, miR-3059-3p, miR-3085-5p, miR-3085-3p, miR-6529-5p, miR-9851-5p, miR-12114, miR-12115, miR-12116, miR-12118, miR-12119, miR-12120, miR-12122, miR-12124, miR-12126, miR-12127, and miR-12128.
[76] A method for analyzing a urine sample, comprising:
   contacting a urine sample with a nanowire in a nanowire-embedded fluidic device having nanowires of metal oxide (e.g., zinc oxide);
   extracting microRNAs captured on the nanowire; and
   confirming whether the extracted microRNAs contain a specific microRNA by contacting the microRNA with a nucleic acid containing a sequence which hybridizes to the microRNA, wherein the specific microRNAs is selected from the group (Urp) consisting of let-7b-5p, miR-25-3p, miR-28-5p, miR-29a-3p, miR-29b-3p, miR-105-5p, miR-192-5p, miR-196a-5p, miR-197-3p, miR-198, miR-199a-5p, miR-129-5p, miR-30d-5p, miR-147a, miR-10a-5p, miR-34a-5p, miR-181a-5p, miR-181b-5p, miR-187-3p, miR-199b-5p, miR-204-5p, miR-210-3p, miR-211-5p, miR-212-3p, miR-214-3p, miR-221-3p, miR-223-3p, miR-200b-3p, miR-23b-3p, miR-30b-5p, miR-122-5p, miR-124-3p, miR-125b-5p, miR-138-5p, miR-125a-5p, miR-134-5p, miR-149-5p, miR-184, miR-185-5p, miR-194-5p, miR-206, miR-106b-5p, miR-34c-5p, miR-299-3p, miR-99b-5p, miR-296-5p, miR-130b-3p, miR-365a-3p, miR-365b-3p, miR-302c-5p, miR-370-3p, miR-373-5p, miR-373-3p, miR-378a-3p, miR-380-3p, miR-382-5p, miR-340-3p, miR-330-3p, miR-328-3p, miR-342-3p, miR-337-3p, miR-324-5p, miR-324-3p, miR-338-3p, miR-133b, miR-325, miR-346, miR-196b-5p, miR-422a, miR-425-3p, miR-448, miR-449a, miR-191-3p, miR-200a-5p, miR-369-5p, miR-433-3p, miR-323b-5p, miR-409-3p, miR-412-3p, miR-483-3p, miR-484, miR-485-5p, miR-485-3p, miR-486-5p, miR-488-5p, miR-489-3p, miR-491-5p, miR-202-3p, miR-492, miR-432-3p, miR-494-3p, miR-497-5p, miR-512-5p, miR-498-5p, miR-515-5p, miR-519c-5p, miR-523-5p, miR-518e-5p, miR-522-5p, miR-519a-5p, miR-519b-5p, miR-526b-5p, miR-525-5p, miR-525-3p, miR-518b, miR-518c-5p, miR-519d-3p, miR-520d-3p, miR-520g-3p, miR-516b-5p, miR-516b-3p, miR-516a-3p, miR-518a-3p, miR-502-5p, miR-504-5p, miR-513a-5p, miR-506-3p, miR-510-5p, miR-532-5p, miR-299-5p, miR-18a-3p, miR-493-3p, miR-487b-3p, miR-551a, miR-554, miR-92b-3p, miR-557, miR-558, miR-563, miR-564, miR-551b-3p, miR-572, miR-574-3p, miR-575, miR-579-3p, miR-580-3p, miR-583, miR-584-5p, miR-585-3p, miR-588, miR-589-3p, miR-550a-3p, miR-595, miR-601, miR-603, miR-604, miR-605-5p, miR-608, miR-610, miR-612, miR-614, miR-615-3p, miR-616-5p, miR-617, miR-622, miR-628-3p, miR-629-3p, miR-632, miR-634, miR-635, miR-636, miR-637, miR-638, miR-639, miR-642a-5p, miR-644a, miR-650, miR-548d-3p, miR-661, miR-663a, miR-449b-5p, miR-654-5p, miR-657, miR-658, miR-421, miR-542-5p, miR-671-5p, miR-668-3p, miR-767-3p, miR-454-5p, miR-769-5p, miR-769-3p, miR-766-3p, miR-765, miR-770-5p, miR-297, let-7b-3p, let-7d-3p, 1et-7f-1-3p, miR-21-3p, miR-23a-5p, miR-25-5p, miR-26b-3p, miR-92a-2-5p, miR-29b-2-5p, miR-106a-3p, miR-16-2-3p, miR-192-3p, miR-129-1-3p, miR-139-3p, miR-183-3p, miR-187-5p, miR-214-5p, miR-222-5p, miR-200b-5p, miR-15b-3p, miR-30b-3p, miR-124-5p, miR-125b-1-3p, miR-135a-3p, miR-138-2-3p, miR-140-3p, miR-125a-3p, miR-125b-2-3p, miR-127-5p, miR-129-2-3p, miR-138-1-3p, miR-149-3p, miR-150-3p, miR-185-3p, miR-186-3p, miR-193a-5p, miR-200c-5p, miR-194-3p, miR-30c-1-3p, miR-219a-2-3p, miR-34b-3p, miR-34c-3p, miR-99b-3p, miR-296-3p, miR-130b-5p, miR-361-3p, miR-302d-5p, miR-371a-5p, miR-330-5p, miR-342-5p, miR-323a-5p, miR-151a-5p, miR-338-5p, miR-423-5p, miR-424-3p, miR-18b-3p, miR-20b-3p, miR-431-3p, miR-483-5p, miR-486-3p, miR-490-5p, miR-146b-3p, miR-193b-5p, miR-516a-5p, miR-505-5p, miR-508-5p, miR-532-3p, miR-92b-5p, miR-551b-5p, miR-574-5p, miR-550a-5p, miR-593-3p, miR-615-5p, miR-625-3p, miR-629-5p, miR-654-3p, miR-671-3p, miR-891a-5p, miR-300, miR-874-3p, miR-888-3p, miR-892b, miR-541-5p, miR-541-3p, miR-876-3p, miR-708-5p, miR-147b-3p, miR-744-5p, miR-744-3p, miR-885-5p, miR-885-3p, miR-877-5p, miR-877-3p, miR-887-3p, miR-665, miR-760, miR-920, miR-921, miR-933, miR-935, miR-936, miR-937-3p, miR-938, miR-939-5p, miR-940, miR-943, miR-1224-5p, miR-1224-3p, miR-1225-5p, miR-1225-3p, miR-1226-5p, miR-1226-3p, miR-1227-3p, miR-1228-5p, miR-1228-3p, miR-1229-3p, miR-1231, miR-1233-3p, miR-1234-3p, miR-1236-3p, miR-1237-3p, miR-1238-3p, miR-513b-5p, miR-513c-5p, miR-320b, miR-320c, miR-1271-5p, miR-1301-3p, miR-1298-5p, miR-1178-3p, miR-1181, miR-1182, miR-1184, miR-1202, miR-1203, miR-1204, miR-1207-5p, miR-1207-3p, miR-1285-3p, miR-1286, miR-1287-5p, miR-1289, miR-1293, miR-1294, miR-1303, miR-1304-5p, miR-1249-3p, miR-1250-5p, miR-1257, miR-1260a, miR-548g-3p, miR-1263, miR-1266-5p, miR-1267, miR-1268a, miR-1269a, miR-1270, miR-1275, miR-1276, miR-1281, miR-1284, miR-1288-3p, miR-1292-5p, miR-1255b-5p, miR-664a-3p, miR-1306-3p, miR-1307-3p, miR-1321, miR-1322, miR-320d, miR-1825, miR-1468-5p, miR-675-3p, miR-1469, miR-1470, miR-1471, miR-1538, miR-1539, miR-103b, miR-1908-5p, miR-1909-5p, miR-1909-3p, miR-1910-5p, miR-1912-3p, miR-1913, miR-1914-3p, miR-1915-3p, miR-103a-2-5p, miR-365a-5p, miR-196b-3p, miR-449b-3p, miR-2113, miR-1972, miR-1976, miR-2110, 1et-7a-2-3p, miR-151b, miR-762, miR-670-5p, miR-761, miR-764, miR-2115-5p, miR-2116-5p, miR-2116-3p, miR-548q, miR-2276-3p, miR-2277-3p, miR-2278, miR-711, miR-718, miR-2681-3p, miR-2682-5p, miR-2682-3p, miR-449c-3p, miR-2909, miR-3116, miR-3119, miR-3120-3p, miR-3122, miR-3124-5p, miR-3125, miR-3126-5p, miR-3127-5p, miR-3130-3p, miR-3130-5p, miR-3131, miR-3132, miR-3133, miR-466, miR-3136-5p, miR-3137, miR-3138, miR-3141, miR-3144-5p, miR-1273c, miR-3147, miR-3148, miR-3149, miR-3150a-3p, miR-3151-5p, miR-3153, miR-3074-3p, miR-3154, miR-3155a, miR-3156-5p, miR-3157-5p, miR-3158-3p, miR-3160-3p, miR-3161, miR-3162-5p, miR-3163, miR-3165, miR-1260b, miR-3173-3p, miR-1193, miR-3175, miR-3176, miR-3177-3p, miR-3178, miR-3179, miR-3180-5p, miR-3180-3p, miR-3185, miR-3186-5p, miR-3186-3p, miR-3187-3p, miR-3188, miR-3189-3p, miR-320e, miR-3190-5p, miR-3191-3p, miR-3192-5p, miR-3195, miR-3196, miR-3197, miR-3198, miR-3199, miR-3200-3p, miR-514b-5p, miR-3202, miR-3126-3p, miR-4295, miR-4296, miR-4297, miR-4293, miR-4294, miR-4301, miR-4299, miR-4298, miR-4300, miR-4304, miR-4303, miR-4305, miR-4306, miR-4307, miR-4308, miR-4311, miR-4312, miR-4313, miR-4316, miR-4314, miR-4319, miR-4317, miR-4322, miR-4321, miR-4323, miR-4324, miR-4256, miR-4257, miR-4258, miR-4259, miR-4260, miR-4253, miR-4251, miR-4254, miR-4255, miR-4252, miR-4326, miR-4327, miR-4261, miR-4265, miR-4266, miR-4262, miR-2355-5p, miR-4268, miR-4269, miR-4271, miR-4276, miR-4274, miR-4279, miR-4278, miR-4280, miR-4285, miR-4283, miR-4284, miR-4286, miR-4288, miR-4292, miR-4289, miR-4290, miR-4329, miR-2277-5p, miR-3605-5p, miR-3605-3p, miR-3610, miR-3612, miR-3614-5p, miR-3615, miR-3616-3p, miR-3619-5p, miR-3620-3p, miR-3621, miR-3622a-5p, miR-3622a-3p, miR-3622b-5p, miR-3646, miR-3648, miR-3649, miR-3650, miR-3651, miR-3652, miR-3654, miR-3655, miR-3659, miR-3660, miR-3661, miR-3663-5p, miR-3663-3p, miR-3665, miR-3667-5p, miR-3667-3p, miR-3675-5p, miR-3675-3p, miR-3679-5p, miR-3679-3p, miR-3682-3p, miR-3685, miR-3689a-3p, miR-3691-5p, miR-3692-5p, miR-3713, miR-3714, miR-3180, miR-3689b-3p, miR-3689c, miR-3911, miR-3917, miR-3918, miR-3919, miR-3150b-3p, miR-3922-3p, miR-3925-5p, miR-676-3p, miR-3928-3p, miR-3929, miR-3934-5p, miR-3935, miR-3936, miR-3937, miR-3940-3p, miR-3943, miR-3944-3p, miR-3945, miR-642b-3p, miR-550b-3p, miR-1268b, miR-4420, miR-4421, miR-378g, miR-4425, miR-4428, miR-4429, miR-4430, miR-4431, miR-4433a-3p, miR-4435, miR-4436a, miR-4437, miR-4438, miR-4439, miR-4440, miR-4441, miR-4443, miR-4444, miR-4445-3p, miR-4446-3p, miR-4447, miR-4448, miR-4449, miR-4450, miR-4451, miR-4453, miR-4454, miR-4455, miR-4456, miR-4458, miR-378h, miR-3135b, miR-4462, miR-4463, miR-4465, miR-4466, miR-4467, miR-4470, miR-4472, miR-4474-3p, miR-4475, miR-4476, miR-4478, miR-3689d, miR-3689f, miR-4479, miR-3155b, miR-4481, miR-4483, miR-4484, miR-4486, miR-4487, miR-4488, miR-4489, miR-4491, miR-4492, miR-4494, miR-4496, miR-4497, miR-4498, miR-4499, miR-4502, miR-4505, miR-4506, miR-2392, miR-4507, miR-4508, miR-4510, miR-4513, miR-4514, miR-4516, miR-4518, miR-4520-3p, miR-4521, miR-1269b, miR-4522, miR-4523, miR-4524a-3p, miR-4525, miR-4526, miR-4529-3p, miR-4530, miR-4531, miR-4533, miR-4534, miR-378i, miR-4535, miR-1587, miR-4537, miR-4538, miR-4539, miR-4540, miR-3120-5p, miR-3127-3p, miR-3129-3p, miR-3150a-5p, miR-3152-5p, miR-3074-5p, miR-3156-3p, miR-3158-5p, miR-3160-5p, miR-3162-3p, miR-3173-5p, miR-3187-5p, miR-3189-5p, miR-3619-3p, miR-3691-3p, miR-3150b-5p, miR-3922-5p, miR-3940-5p, miR-3944-5p, miR-4423-5p, miR-4446-5p, miR-4520-5p, miR-4529-5p, miR-3960, miR-3972, miR-4633-3p, miR-4634, miR-4635, miR-4638-5p, miR-4639-3p, miR-4640-5p, miR-4640-3p, miR-4641, miR-4642, miR-4644, miR-4646-5p, miR-4646-3p, miR-4647, miR-4648, miR-4649-3p, miR-4650-5p, miR-4650-3p, miR-4651, miR-4652-5p, miR-4653-5p, miR-4653-3p, miR-4654, miR-4655-5p, miR-4656, miR-4657, miR-4658, miR-4660, miR-4661-3p, miR-4664-5p, miR-4664-3p, miR-4665-5p, miR-4665-3p, miR-4667-5p, miR-4667-3p, miR-219b-5p, miR-4669, miR-4672, miR-4673, miR-4674, miR-4676-5p, miR-4676-3p, miR-4677-5p, miR-4681, miR-4682, miR-4684-5p, miR-4684-3p, miR-4685-5p, miR-4685-3p, miR-4686, miR-4687-5p, miR-1343-3p, miR-4688, miR-4689, miR-4690-5p, miR-4691-5p, miR-4692, miR-4695-5p, miR-4695-3p, miR-4697-5p, miR-4697-3p, miR-4698, miR-4700-5p, miR-4700-3p, miR-4701-5p, miR-4701-3p, miR-4706, miR-4707-5p, miR-4707-3p, miR-4708-5p, miR-4708-3p, miR-4709-5p, miR-4709-3p, miR-4710, miR-4711-3p, miR-4713-5p, miR-4713-3p, miR-4714-5p, miR-4716-5p, miR-4716-3p, miR-3529-5p, miR-4717-5p, miR-4717-3p, miR-4718, miR-4720-5p, miR-4721, miR-4722-5p, miR-4722-3p, miR-4723-5p, miR-4723-3p, miR-4724-5p, miR-4725-5p, miR-4725-3p, miR-4726-5p, miR-4726-3p, miR-4727-3p, miR-4728-5p, miR-4728-3p, miR-4730, miR-4731-5p, miR-4731-3p, miR-4732-5p, miR-4732-3p, miR-4734, miR-4736, miR-4737, miR-3064-5p, miR-3064-3p, miR-4738-3p, miR-4740-5p, miR-4740-3p, miR-4741, miR-4743-5p, miR-122b-3p, miR-4745-5p, miR-4746-3p, miR-4747-5p, miR-4748, miR-4749-5p, miR-4749-3p, miR-4750-5p, miR-4751, miR-4753-5p, miR-4753-3p, miR-371b-5p, miR-371b-3p, miR-4754, miR-4755-3p, miR-4756-5p, miR-4756-3p, miR-4758-5p, miR-4758-3p, miR-4759, miR-4763-5p, miR-4763-3p, miR-4764-5p, miR-4766-5p, miR-4769-5p, miR-4769-3p, miR-4773, miR-4776-5p, miR-4776-3p, miR-4778-5p, miR-4779, miR-4780, miR-4436b-5p, miR-4436b-3p, miR-4781-5p, miR-4783-3p, miR-4784, miR-4785, miR-2467-3p, miR-4786-5p, miR-4786-3p, miR-4787-5p, miR-4787-3p, miR-4788, miR-4793-3p, miR-4794, miR-4800-5p, miR-4800-3p, miR-4801, miR-4804-3p, miR-642a-3p, miR-550a-3-5p, miR-4433a-5p, miR-4482-3p, miR-4999-5p, miR-5000-5p, miR-5000-3p, miR-5001-5p, miR-5001-3p, miR-5002-5p, miR-5002-3p, miR-5003-3p, miR-5004-5p, miR-5004-3p, miR-5006-5p, miR-5006-3p, miR-5007-5p, miR-5009-3p, miR-5010-5p, miR-5010-3p, miR-5087, miR-5088-5p, miR-5089-5p, miR-5090, miR-5093, miR-5187-5p, miR-5187-3p, miR-5188, miR-5189-5p, miR-5190, miR-5192, miR-5193, miR-5194, miR-5195-5p, miR-5196-5p, miR-5196-3p, miR-4524b-5p, miR-4524b-3p, miR-5571-5p, miR-5571-3p, miR-5100, miR-5572, miR-664b-5p, miR-664b-3p, miR-5580-5p, miR-5580-3p, miR-5581-5p, miR-5581-3p, miR-5584-5p, miR-5584-3p, miR-5585-3p, miR-5587-3p, miR-548au-3p, miR-5588-5p, miR-5588-3p, miR-5589-5p, miR-5684, miR-5685, miR-5681b, miR-5690, miR-5694, miR-5698, miR-5702, miR-5703, miR-5704, miR-5705, miR-5708, miR-197-5p, miR-204-3p, miR-211-3p, miR-345-3p, miR-584-3p, miR-652-5p, miR-660-3p, miR-1185-2-3p, miR-766-5p, miR-873-3p, miR-1285-5p, miR-1304-3p, miR-1247-3p, miR-1255b-2-3p, miR-1306-5p, miR-3184-3p, miR-3191-5p, miR-642b-5p, miR-550b-2-5p, miR-365b-5p, miR-1185-1-3p, miR-3190-3p, miR-98-3p, miR-216a-3p, miR-381-5p, miR-495-5p, miR-503-3p, miR-758-5p, miR-937-5p, miR-939-3p, miR-1227-5p, miR-1229-5p, miR-1233-5p, miR-1236-5p, miR-1237-5p, miR-1238-5p, miR-1292-3p, miR-3617-3p, miR-3620-5p, miR-3927-5p, miR-3934-3p, miR-4632-5p, miR-4750-3p, miR-5089-3p, miR-5739, miR-5787, miR-6068, miR-6069, miR-6071, miR-6072, miR-6074, miR-6075, miR-6076, miR-6077, miR-6080, miR-6081, miR-6083, miR-6084, miR-6085, miR-6086, miR-6089, miR-6090, miR-6124, miR-6125, miR-6126, miR-6127, miR-378j, miR-6129, miR-6130, miR-6131, miR-6132, miR-6133, miR-6165, miR-6499-3p, miR-6500-5p, miR-6500-3p, miR-6501-5p, miR-6501-3p, miR-6503-5p, miR-6503-3p, miR-6504-5p, miR-6504-3p, miR-6505-3p, miR-6506-5p, miR-6507-3p, miR-6508-5p, miR-6509-5p, miR-6509-3p, miR-6510-5p, miR-6511a-5p, miR-6511a-3p, miR-6512-3p, miR-6513-5p, miR-6513-3p, miR-6514-5p, miR-6514-3p, miR-6515-5p, miR-6515-3p, miR-6715a-3p, miR-6715b-5p, miR-6716-5p, miR-6716-3p, miR-6717-5p, miR-6511b-5p, miR-6511b-3p, miR-6718-5p, miR-6719-3p, miR-6721-5p, miR-6722-5p, miR-6722-3p, miR-6724-5p, miR-892c-5p, miR-892c-3p, 1et-7c-3p, miR-210-5p, miR-128-1-5p, miR-134-3p, miR-370-5p, miR-433-5p, miR-329-5p, miR-410-5p, miR-487a-5p, miR-489-5p, miR-494-5p, miR-504-3p, miR-487b-5p, miR-579-5p, miR-585-5p, miR-598-5p, miR-605-3p, miR-619-5p, miR-656-5p, miR-668-5p, miR-1296-3p, miR-1301-5p, miR-1298-3p, miR-874-5p, miR-889-5p, miR-887-5p, miR-1250-3p, miR-1251-3p, miR-1266-3p, miR-1288-5p, miR-1910-3p, miR-3151-3p, miR-3192-3p, miR-500b-3p, miR-3928-5p, miR-1343-5p, miR-5088-3p, miR-5189-3p, miR-5699-5p, miR-6720-5p, miR-6726-5p, miR-6726-3p, miR-6727-5p, miR-6727-3p, miR-6728-5p, miR-6728-3p, miR-6729-5p, miR-6729-3p, miR-6730-5p, miR-6730-3p, miR-6731-5p, miR-6731-3p, miR-6732-5p, miR-6732-3p, miR-6734-5p, miR-6735-5p, miR-6735-3p, miR-6736-5p, miR-6736-3p, miR-6737-5p, miR-6737-3p, miR-6738-5p, miR-6738-3p, miR-6739-3p, miR-6740-5p, miR-6740-3p, miR-6741-5p, miR-6741-3p, miR-6742-5p, miR-6742-3p, miR-6743-5p, miR-6743-3p, miR-6744-5p, miR-6744-3p, miR-6745, miR-6746-5p, miR-6746-3p, miR-6747-5p, miR-6747-3p, miR-6748-5p, miR-6748-3p, miR-6749-5p, miR-6749-3p, miR-6750-5p, miR-6750-3p, miR-6751-5p, miR-6751-3p, miR-6752-5p, miR-6752-3p, miR-6753-5p, miR-6753-3p, miR-6754-5p, miR-6754-3p, miR-6755-5p, miR-6756-5p, miR-6756-3p, miR-6757-5p, miR-6757-3p, miR-6758-5p, miR-6759-5p, miR-6759-3p, miR-6760-5p, miR-6760-3p, miR-6761-5p, miR-6761-3p, miR-6762-5p, miR-6762-3p, miR-6763-5p, miR-6763-3p, miR-6764-3p, miR-6765-5p, miR-6766-5p, miR-6766-3p, miR-6767-5p, miR-6768-5p, miR-6769a-5p, miR-6769a-3p, miR-6770-5p, miR-6770-3p, miR-6771-3p, miR-6772-5p, miR-6772-3p, miR-6773-3p, miR-6774-5p, miR-6774-3p, miR-6775-5p, miR-6775-3p, miR-6776-5p, miR-6776-3p, miR-6777-5p, miR-6777-3p, miR-6778-5p, miR-6778-3p, miR-6779-5p, miR-6779-3p, miR-6780a-5p, miR-6780a-3p, miR-6781-5p, miR-6781-3p, miR-6782-5p, miR-6782-3p, miR-6783-5p, miR-6783-3p, miR-6784-5p, miR-6784-3p, miR-6785-3p, miR-6786-5p, miR-6786-3p, miR-6787-5p, miR-6787-3p, miR-6788-5p, miR-6789-5p, miR-6789-3p, miR-6790-5p, miR-6790-3p, miR-6791-5p, miR-6791-3p, miR-6792-5p, miR-6792-3p, miR-6793-5p, miR-6793-3p, miR-6794-5p, miR-6794-3p, miR-6795-5p, miR-6795-3p, miR-6796-5p, miR-6796-3p, miR-6797-5p, miR-6797-3p, miR-6798-5p, miR-6798-3p, miR-6799-5p, miR-6799-3p, miR-6800-5p, miR-6800-3p, miR-6801-5p, miR-6801-3p, miR-6802-3p, miR-6803-5p, miR-6803-3p, miR-6804-5p, miR-6804-3p, miR-6805-5p, miR-6805-3p, miR-6806-5p, miR-6807-5p, miR-6808-5p, miR-6809-5p, miR-6809-3p, miR-6810-5p, miR-6810-3p, miR-6812-5p, miR-6812-3p, miR-6813-5p, miR-6813-3p, miR-6814-5p, miR-6814-3p, miR-6815-5p, miR-6815-3p, miR-6816-5p, miR-6816-3p, miR-6817-5p, miR-6817-3p, miR-6819-5p, miR-6819-3p, miR-6820-5p, miR-6820-3p, miR-6821-5p, miR-6821-3p, miR-6822-5p, miR-6822-3p, miR-6823-5p, miR-6823-3p, miR-6824-5p, miR-6824-3p, miR-6825-5p, miR-6825-3p, miR-6826-3p, miR-6827-5p, miR-6827-3p, miR-6828-5p, miR-6829-5p, miR-6829-3p, miR-6830-5p, miR-6830-3p, miR-6831-5p, miR-6831-3p, miR-6832-5p, miR-6832-3p, miR-6833-5p, miR-6833-3p, miR-6834-5p, miR-6834-3p, miR-6780b-5p, miR-6780b-3p, miR-6836-5p, miR-6836-3p, miR-6837-5p, miR-6838-5p, miR-6839-5p, miR-6840-5p, miR-6840-3p, miR-6841-3p, miR-6842-5p, miR-6842-3p, miR-6843-3p, miR-6845-5p, miR-6845-3p, miR-6846-5p, miR-6846-3p, miR-6847-5p, miR-6847-3p, miR-6848-5p, miR-6848-3p, miR-6849-5p, miR-6849-3p, miR-6850-5p, miR-6851-5p, miR-6851-3p, miR-6852-5p, miR-6852-3p, miR-6853-5p, miR-6854-3p, miR-6855-5p, miR-6855-3p, miR-6856-5p, miR-6857-5p, miR-6857-3p, miR-6858-5p, miR-6858-3p, miR-6859-5p, miR-6859-3p, miR-6769b-5p, miR-6860, miR-6861-5p, miR-6861-3p, miR-6862-5p, miR-6862-3p, miR-6863, miR-6865-3p, miR-6867-5p, miR-6867-3p, miR-6868-5p, miR-6868-3p, miR-6869-5p, miR-6870-5p, miR-6870-3p, miR-6871-5p, miR-6871-3p, miR-6872-3p, miR-6873-3p, miR-6874-5p, miR-6875-5p, miR-6875-3p, miR-6876-5p, miR-6876-3p, miR-6877-5p, miR-6877-3p, miR-6878-3p, miR-6879-5p, miR-6879-3p, miR-6880-5p, miR-6880-3p, miR-6881-5p, miR-6881-3p, miR-6882-5p, miR-6882-3p, miR-6883-5p, miR-6883-3p, miR-6884-5p, miR-6884-3p, miR-6885-3p, miR-6886-5p, miR-6886-3p, miR-6887-5p, miR-6887-3p, miR-6888-5p, miR-6889-5p, miR-6889-3p, miR-6890-3p, miR-6891-5p, miR-6891-3p, miR-6892-5p, miR-6892-3p, miR-6893-5p, miR-6893-3p, miR-6894-5p, miR-6894-3p, miR-6895-5p, miR-6895-3p, miR-7106-5p, miR-7106-3p, miR-7107-5p, miR-7108-5p, miR-7108-3p, miR-7109-5p, miR-7109-3p, miR-7110-5p, miR-7110-3p, miR-7111-5p, miR-7111-3p, miR-7112-5p, miR-7113-5p, miR-7113-3p, miR-7114-5p, miR-7114-3p, miR-7150, miR-7151-5p, miR-7151-3p, miR-7152-5p, miR-7152-3p, miR-7
   154-3p, miR-7155-5p, miR-7155-3p, miR-7156-3p, miR-7157-5p, miR-7157-3p, miR-7158-5p, miR-7160-5p, miR-7162-3p, miR-7515, miR-7702, miR-7843-5p, miR-4433b-5p, miR-4433b-3p, miR-1273h-5p, miR-1273h-3p, miR-6516-5p, miR-7845-5p, miR-7846-3p, miR-7847-3p, miR-7850-5p, miR-7851-3p, miR-7854-3p, miR-7855-5p, miR-7856-5p, miR-8052, miR-8054, miR-8057, miR-8059, miR-8060, miR-8062, miR-8063, miR-8064, miR-8069, miR-8070, miR-8071, miR-8072, miR-8073, miR-8074, miR-8075, miR-8077, miR-8078, miR-8079, miR-8080, miR-8082, miR-8083, miR-8085, miR-8086, miR-8087, miR-8088, miR-8089, miR-1199-3p, miR-7975, miR-7976, miR-7977, miR-7978, miR-1249-5p, miR-4485-5p, miR-8485, miR-103a-1-5p, miR-196a-1-3p, miR-135a-2-3p, miR-375-5p, miR-526a-3p, miR-9718, miR-9898, miR-9902, miR-1843, miR-9986, miR-10226, miR-10392-5p, miR-10392-3p, miR-10394-3p, miR-10395-5p, miR-10395-3p, miR-10396a-5p, miR-10396a-3p, miR-10398-5p, miR-10398-3p, miR-10400-3p, miR-10401-5p, miR-10401-3p, miR-10396b-5p, miR-10396b-3p, miR-10522-5p, miR-10523-5p, miR-10526-3p, miR-10527-5p, miR-11181-5p, miR-11181-3p, miR-11399, miR-11400, miR-11401, miR-3059-5p, miR-3059-3p, miR-3085-5p, miR-3085-3p, miR-6529-5p, miR-12114, miR-12115, miR-12116, miR-12117, miR-12118, miR-12119, miR-12120, miR-12121, miR-12122, miR-12124, miR-12125, miR-12126, miR-12127, miR-12128, miR-12131, and miR-12136.
[77] A method according to any one of [73] to [76] above, comprising detecting 5 or more microRNAs.
[78] A method according to any one of [73] to [76] above, comprising detecting 10 or more, 15 or more, or 20 or more microRNAs.
[79] The method according to any one of [73] to [76] above, wherein the microRNA further satisfies the condition that the expression is upregulated in a cancer patient in Tables 4-1 to 4-15 {in each table, the microRNA may be one or more, two or more, three or more, four or more, five or more, 10 or more, 15 or more, or 20 or more microRNAs}.
[80] The method according to any one of [73] to [76] above, wherein the microRNA further satisfies the condition that the expression is the condition in Tables 4-1 to 4-15 that expression is downregulated in a cancer patient {in each table, the microRNA may be one or more, two or more, three or more, four or more, five or more, 10 or more, 15 or more, or 20 or more microRNAs}.
[81] A method for predicting a likelihood that a subject having or suspected of having cancer has early-stage cancer, comprising:
   executing the method of [73], wherein the microRNA further satisfies the condition that the expression is the condition in Tables 4-1 to 4-15 that expression is upregulated in a cancer patient {in each table, the microRNA may be one or more, two or more, three or more, four or more, five or more, 10 or more, 15 or more, or 20 or more microRNAs},
   wherein the presence of microRNAs exhibiting expression higher than the first threshold indicates a likelihood that the subject from which the urine is derived has early-stage cancer.
[82] A method for predicting a likelihood that a subject having or suspected of having cancer has early-stage cancer, comprising:
   executing the method of [74], wherein the microRNA further satisfies the condition that the expression is the condition in Tables 4-1 to 4-15 that expression is upregulated in a cancer patient {in each table, the microRNA may be one or more, two or more, three or more, four or more, five or more, 10 or more, 15 or more, or 20 or more microRNAs},
   wherein the presence of microRNAs exhibiting expression higher than the first threshold indicates a likelihood that the subject from which the urine is derived has gastrointestinal cancer.
[83] The method according to [82] above, wherein the subject having or suspected of having cancer is a subject having or suspected of having gastrointestinal cancer.
[84] A method for predicting a likelihood that a subject having or suspected of having cancer has early-stage cancer, comprising:
   executing the method of [75], wherein the microRNA further satisfies the condition that the expression is the condition in Tables 4-1 to 4-15 that expression is upregulated in a cancer patient {in each table, the microRNA may be one or more, two or more, three or more, four or more, five or more, 10 or more, 15 or more, or 20 or more microRNAs},
   wherein the presence of microRNAs exhibiting expression higher than the first threshold indicates a likelihood that the subject from which the urine is derived has hematopoietic cancer.
[85] The method according to [84] above, wherein the subject having or suspected of having cancer is a subject having or suspected of having hematopoietic cancer.
[86] A method for predicting a likelihood that a subject having or suspected of having cancer has early-stage cancer, comprising:
   executing the method of [76], wherein the microRNA further satisfies the condition that the expression is the condition in Tables 4-1 to 4-15 that expression is upregulated in a cancer patient {in each table, the microRNA may be one or more, two or more, three or more, four or more, five or more, 10 or more, 15 or more, or 20 or more microRNAs},
   wherein the presence of microRNAs exhibiting expression higher than the first threshold indicates a likelihood that the subject from which the urine is derived has urologic cancer.
[87] The method according to [86] above, wherein the subject having or suspected of having cancer is a subject having or suspected of having urological cancer.
[88] A method for predicting a likelihood that a subject having or suspected of having cancer has early-stage cancer, comprising:
   executing the method of [73], wherein the microRNA further satisfies the condition that the expression is the condition in Tables 4-1 to 4-15 that expression is downregulated in a cancer patient {in each table, the microRNA may be one or more, two or more, three or more, four or more, five or more, 10 or more, 15 or more, or 20 or more microRNAs},
   wherein the presence of microRNAs exhibiting expression lower than the first threshold indicates a likelihood that the subject from which the urine is derived has cancer.
[89] A method for predicting a likelihood that a subject having or suspected of having cancer has early-stage cancer, comprising:
   executing the method of [74], wherein the microRNA further satisfies the condition that the expression is the condition in Tables 4-1 to 4-15 that expression is downregulated in a cancer patient {in each table, the microRNA may be one or more, two or more, three or more, four or more, five or more, 10 or more, 15 or more, or 20 or more microRNAs},
   wherein the presence of microRNAs exhibiting expression lower than healthy persons indicates a likelihood that the subject from which the urine is derived has gastrointestinal cancer.
[90] The method according to [89] above, wherein the subject having or suspected of having cancer is a subject having or suspected of having gastrointestinal cancer.
[91] A method for predicting a likelihood that a subject having or suspected of having cancer has early-stage cancer, comprising:
   executing the method of [75], wherein the microRNA further satisfies the condition that the expression is the condition in Tables 4-1 to 4-15 that expression is downregulated in a cancer patient {in each table, the microRNA may be one or more, two or more, three or more, four or more, five or more, 10 or more, 15 or more, or 20 or more microRNAs},
   wherein the presence of microRNAs exhibiting expression lower than healthy persons indicates a likelihood that the subject from which the urine is derived has hematopoietic cancer.
[92] The method according to [91] above, wherein the subject having or suspected of having cancer is a subject having or suspected of having hematopoietic cancer.
[93] A method for predicting a likelihood that a subject having or suspected of having cancer has early-stage cancer, comprising:
   executing the method of [75], wherein the microRNA further satisfies the condition that the expression is the condition in Tables 4-1 to 4-15 that expression is downregulated in a cancer patient {in each table, the microRNA may be one or more, two or more, three or more, four or more, five or more, 10 or more, 15 or more, or 20 or more microRNAs},
   wherein the presence of microRNAs exhibiting expression lower than the second threshold indicates a likelihood that the subject from which the urine is derived has urologic cancer.
[94] The method according to [93] above, wherein the subject having or suspected of having cancer is a subject having or suspected of having urological cancer.
[95] The method according to any one of [81] to [87] above, wherein the microRNA exhibiting higher expression than the first threshold is 5 or more types.
[96] The method according to any one of [81] to [87] above, wherein the microRNA exhibiting higher expression than the first threshold is 10 or more, 15 or more or 20 or more types.
[97] The method according to any one of [88] to [94] above, wherein the microRNA exhibiting expression lower than the second threshold is 5 or more types.
[98] The method according to any one of [88] to [94] above, wherein the microRNA exhibiting expression lower than the second threshold is 10 or more, 15 or more or 20 or more types.
[99] The method of any one of the above, which is an in vitro method.
[100] The method of any one of the above, wherein the nanowire is a nanowire having a surface of a metal oxide.
[101] The method of [100], wherein the nanowire is a nanowire having a zinc oxide surface.
[102] The method of any one of the above, wherein the first threshold is any number between two values (statistical value or index value) selected from the group consisting of a mean, a median, a third quartile, a first quartile, and a minimum value of the micro-RNA levels in a group of cancerous subjects {where the thresholds may be different for each microRNAs}.
[103] The method of any one of the above, wherein the first threshold is any number between two values selected from the group consisting of a maximum, a third quartile, a mean, a median, and a first quartile of the microRNA levels in a group of non-cancerous subjects, {where the thresholds may be different for each microRNAs}.
[104] The method of any one of the above, wherein the first threshold is any number (e.g., an intermediate value) between a mean value in a cancerous control group and a mean value in healthy persons {where the thresholds may be different for each microRNAs}.
[105] The method of any one of the above, wherein the second threshold is any number between two values (statistical value or index value) selected from the group consisting of a mean, a median, a third quartile, a first quartile, and a minimum value of the micro-RNA levels in a group of cancerous subjects {where the thresholds may be different for each microRNAs}.
[106] The method of any one of the above, wherein the second threshold is any number between two values selected from the group consisting of a maximum, a third quartile, a mean, a median, and a first quartile of the microRNA levels in a group of non-cancerous subjects, {where the thresholds may be different for each microRNAs}.
[107] The method of any one of the above, wherein the second threshold is any number (e.g., an intermediate value) between a mean value in a cancerous control group and a mean value in healthy persons {where the thresholds may be different for each microRNAs}.
[108] The method of any one above, wherein the first threshold is a value between a mean value of the microRNA levels in a cancerous control group and a mean value of the microRNA levels in a non-cancerous {where the thresholds may be different for each microRNAs}.
[109] The method of any one above, wherein the first threshold is an intermediate value between a mean value of the microRNA levels in a cancerous control group and a mean value of the microRNA levels in a non-cancerous control group {where the thresholds may be different for each microRNAs} .
[110] The method of any one above, wherein the second threshold is a value between a mean value of the microRNA levels in a cancerous control group and a mean value of the microRNA levels in a non-cancerous control group {where the thresholds may be different for each microRNAs}.
[111] The method of any one above, wherein the second threshold is an intermediate value between a mean value of the microRNA levels in a cancerous control group and a mean value of the microRNA levels in a non-cancerous control group {where the thresholds may be different for each microRNAs} .
[112] The method of any one above, wherein the first threshold is a value between the mean and the median of the cancerous control group.
[113] The method of any one above, wherein the first threshold is a value between the mean and the third quartile of the cancerous control group.
[114] The method of any one above, wherein the first threshold is a value between the mean and the first quartile of the cancerous control group.
[115] The method of any one above, wherein the first threshold is a value between the first quartile and the minimum value of the cancerous control group.
[116] The method of any one above, wherein the first threshold is a value between the mean and the median of the non-cancerous control group.
[117] The method of any one above, wherein the first threshold is a value between the mean and the third quartile of the non-cancer control group.
[118] The method of any one above, wherein the first threshold is a value between the mean and the first quartile of the non-cancer control group.
[119] The method of any one above, wherein the first threshold is a value between the third quartile and the maximum of the non-cancer control group.
[120] The method of any one above, wherein the second threshold is a value between the mean and the median of the cancerous control group.
[121] The method of any one above, wherein the second threshold is a value between the mean and the third quartile of the cancerous control group.
[122] The method of any one above, wherein the second threshold is a value between the mean and the third quartile of the cancerous control group.
[123] The method of any one above, wherein the second threshold is a value between the first quartile and the minimum value of the cancerous control group.
[124] The method of any one above, wherein the second threshold is a value between the mean and the median of the non-cancerous control group.
[125] The method of any one above, wherein the second threshold is a value between the mean and the third quartile of the non-cancerous control group.
[126] The method of any one above, wherein the second threshold is a value between the mean and the first quartile of the non-cancerous control group.
[127] The method of any one above, wherein the second threshold is a value between the third quartile and the maximum of the non-cancerous control group.
[128] The method according to [61] above, wherein the confirmation is performed by a method of detecting a miRNA selected from the group consisting of quantitative PCR, microarray for miRNA detection, RNA-Seq method, and multiplex miRNA profiling method.
[129] The method of any one of the above wherein the nanowire is a nanowire in a nanowire-embedded fluid device having nanowires.

### BRIEF DESCRIPTION OF THE DRAWINGS

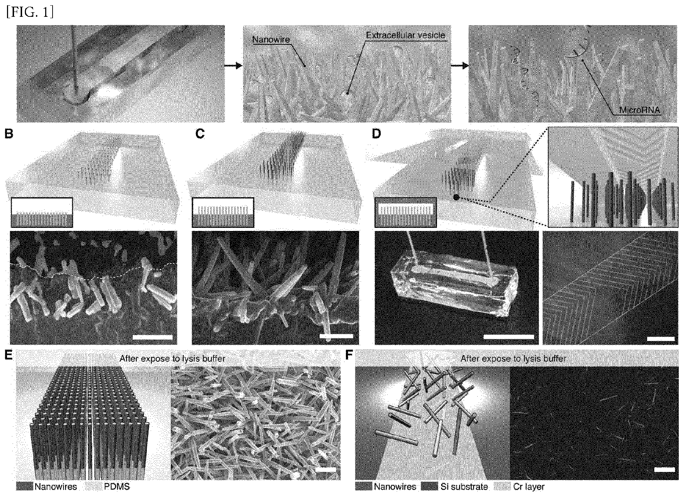

FIG. 1 relates to electrostatic collection of urinary EV by nanowires and subsequent in-situ extraction of the EV-inclusion miRNA.
FIG. 1A shows a schematic diagram of the collection (capture) of EV in urine using a nanowire-incorporated microdevice and in-situ extraction of EV- inclusion miRNA.
FIG. 1B shows a schematic diagram of the embedded nanowires after pouring, curing and peeling of PDMS and a schematic inset of the vertical cross-sectional FESEM image of the embedded nanowires (nanowires shown by rods, PDMS shown as the transparent area) and a cut-off plane image at the lower left.
   Yellow (light area on gray scale) indicates nanowires, blue indicates PDMS, and dotted white lines indicate the edge of PDMS.
   The scale bar indicates 1 µm.
FIG. 1C shows a schematic diagram showing a cross-sectional image of nanowire growth from embedded nanowires (nanowire-incorporated PDMS) and a lower left schematic inset and a vertical cross-sectional image of the nanowire embedded PDMS. The scale bar indicates 1 µm.
FIG. 1D shows a schematic cross-sectional view of bonding of the nanowire-incorporated PDMS to a PDMS substrate having a microfluidic herringbone structure, a schematic inset at the bottom left, a photograph of a nanowire-embedded microfluidic device with polyetheretherketone (PEEK) tubes for inlet and outlet (the nanowire-incorporated PDMS and the PDMS substrate of the microfluidic herringbone structure are bonded to each other), and a laser micrograph of the microfluidic herringbone structure on the PDMS substrate. The scale bar indicates 1 µm.
FIG. IE shows a schematic view of a nanowire-incorporated device and FESEM image from the top surface of a nanowire-incorporated PDMS after being exposed to the lysis buffer. The scale bar indicates 1 µm.
FIG. IF shows a schematic view of nanowires on a Si substrate and a FESEM image from the top surface of the nanowires on the Si substrate after being exposed to a dissolution buffer.
FIG. 2 relates to in the situ extraction of miRNA using the nanowire-incorporated microfluidic device.
FIG. 2A shows a scatter plot of the normalized intensities of miRNA extracted from the EVS collected by the nanowire-incorporated devices of the present disclosure and by ultracentrifugation.
FIG. 2B shows a histogram of miRNA species that could be extracted by the method using the nanowire-incorporated devices of the present disclosure and by ultracentrifugation (n=3).
FIG. 2C shows a scatter plot of the normalized intensities of miRNA extracted from the EVs collected by the nanowire-incorporated devices of the present disclosure and by commercially available kits.
FIG. 2D shows a histogram of miRNA species that could be extracted by the method using the nanowire-incorporated devices of the present disclosure and by ultracentrifugation (n=3). The error bars indicate SD for the measurements (n=3).
   "a.u." denotes arbitrary units (arbitrary unit).
FIG. 3 relates to the collection of EVs onto nanowires.
FIG. 3A is a schematic diagram relating to the experimental process and the calculation of the collection efficiency.
FIG. 3B shows the size-distribution of free suspended solids of urine in untreated urine.
   The error bars indicate SD for the measurements (n=3).
FIG. 3C shows the flow-through fractionation of urine processed by the nanowire-incorporated device of the present disclosure and the size-distribution of the free-suspended solids of urine in ultracentrifuged urine. The error bars indicate SD for the measurements (n=3).
FIG. 3D shows EVs collected with nanowires and fluorescent (RKH26) labeled. In the figure, PKH26 labeled EVs on nanowires are shown.
   The scale bar indicates 500 µm.
FIG. 3E shows a FESEM image of the nanowires after introduction of PKH26 labeled EVs. The white arrows indicate collected EVs. The scale bar indicates 200 nm.
FIG. 3F shows the results of detecting EVs on nanowires and 96-well plates using antibodies against CD63 or CD81. The measured concentration of free suspended solids in urine was 1.4×10⁸m1-1. "N.D." indicates that no fluorescence was observed. The dotted line indicates the 3SD signal level above background. The error bars show SD for the measurements (n=24 for nanowires; n=3 for 96-well plates).
FIG. 4 shows the results of in situ extractions of cancer-related miRNA using nanowire-incorporated devices.

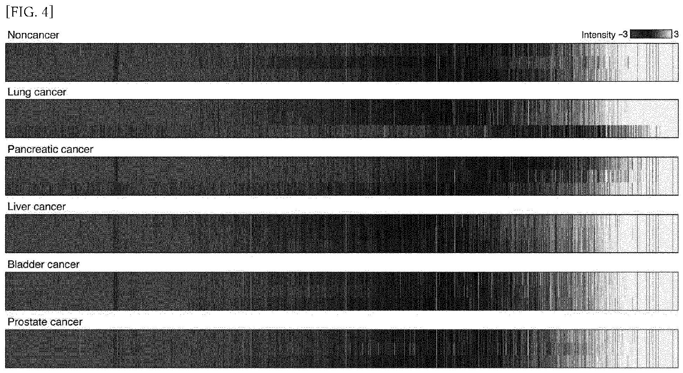

FIG. 4 shows the heat maps of miRNA expression arrays for each of the urine samples of non-cancerous donors, lung cancer donors, liver cancer donors, bladder cancer donors, and prostate cancer donors, respectively. Gradients of colors based on signal intensities have been used to intuitively understand the expression of each miRNA and the comparisons between the groups. If the logarithmic signal intensity is 5, it is black; 2 or less is blue; and 8 or more is yellow. Each column of the heat map represents the logarithmic signal intensity of miRNA (specific numerical values are shown in S1).

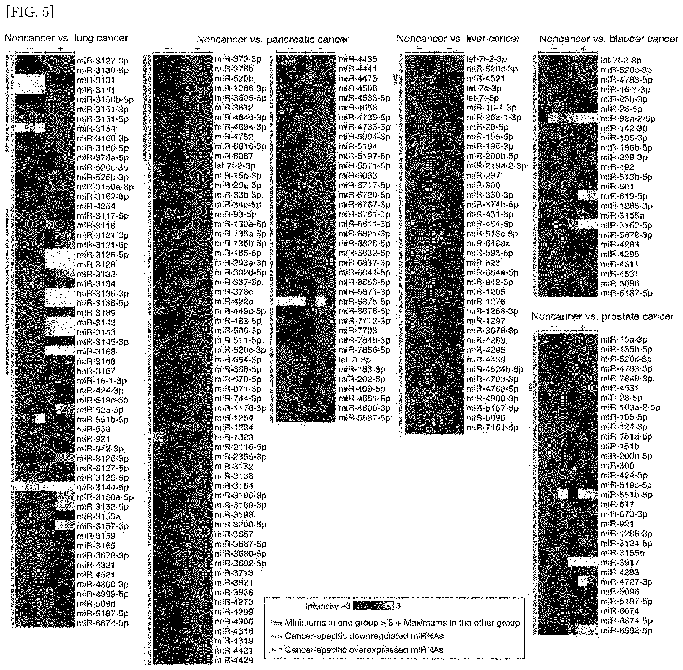

FIG. 5 shows down-regulation and over-expression of miRNA extracted from FIG. 4 between non-cancerous donors and donors with various cancers. The extracted miRNAs were set to have a log signal intensity of the second smallest of one group greater than the second largest of the other group. The symbols "-" and "+" indicate non-cancer donors and cancer donors, respectively. The part where the left line is doubled indicates that one of the smallest logarithmic signal intensities was greater than the other's largest logarithmic signal intensity. Otherwise, one logarithmic signal intensity is higher or lower than the other logarithmic signal intensity.

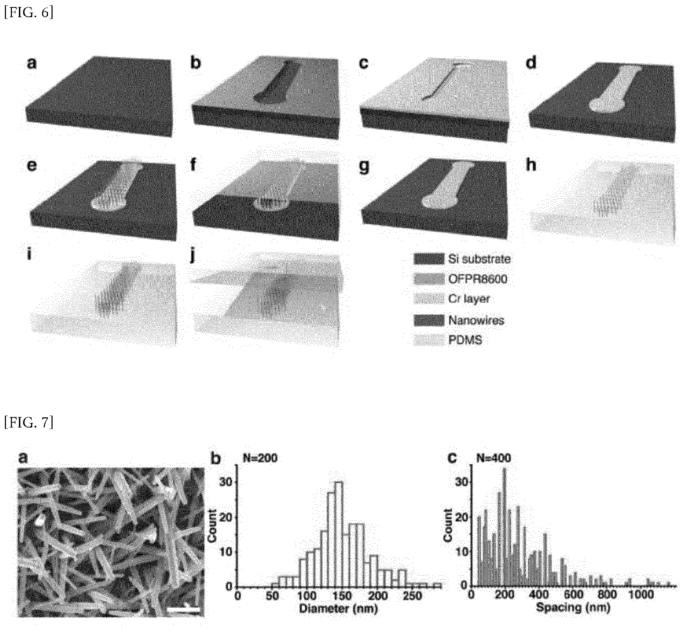

FIG. 6 shows a schematic view of the fabrication process of the nanowires incorporated in PDMS.
FIGS. 6a-g show that nanowires are incorporated into PDMS by lithography and PDMS curing processes. Components include a Si-substrate and OFPR8600 photoresist, a Cr-layer, nanowires, and PDMS. In FIG. 6a, a Si substrate is prepared, and in FIG. 6a, the photoresist is laminated in FIG. 6b. In FIG. 6c the Cr layer is further laminated, in FIG. 6d the photoresist is removed, and in
FIG. 6e the nanowires are grown the Cr layer. In FIG. 6f PDMS is poured and cured. In FIG. 6g the cured PDMS layer is peeled from the substrate.
FIG 6h shows a schematic view of the embedded nanowires after pouring, curing, and peeling of PDMS.
FIG. 6i shows a schematic view of the process of growing nanowires from the embedded nanowires.
FIG. 6j shows a schematic view of the process of bonding the nanowire-embedded PDMS substrate to a microfluidic herringbone-structured PDMS substrate.
FIG. 7 relates to the incorporation of nanowires into the PDMS.
FIG. 7a is a FESEM image from the top surface of the nanowires. The scale bar indicates 1 µm.
FIG. 7b shows the distribution of the diameter of nanowires. The average diameter of the nanowires was 150 nm.
FIG. 7c shows the distribution of the space between the nanowires. The space was defined as the shortest distance between the two nanowires and determined from the SEM image of the cut surface. The average space between the nanowires was 200 nm.

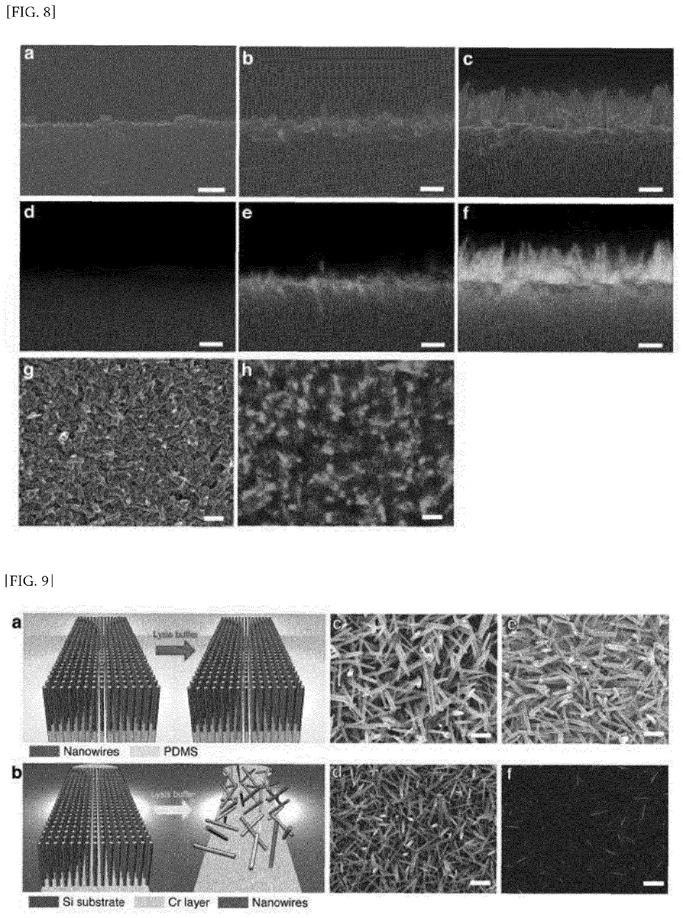

FIG. 8 relates to FESEM images of a PDMS substrate without nanowires, a PDMS substrate with embedded nanowires, and a nanowire-incorporated PDMS substrate, respectively, and corresponding EDS-element mappings.
FIGS. 8a-c are FESEM images of vertical cut planes of a PDMS substrate without nanowires, a PDMS substrate with embedded nanowires, and a nanowire-embedded PDMS substrate, respectively. FIGS. 8d-f show element mappings corresponding to the FESEM images of the cut surface of FIGS. 8a-c. Si and Zn are represented by blue (dark areas) and green (areas indicated by arrowheads and bright areas), respectively. In FIG. 8e, it was observed that slightly nanowires (green) are exposed on the surface of PDMS substrate (blue). Further, in FIG. 8f, it was observed that the thickness of the nanowires is increased.
FIG. 8g is a FESEM image from the top surface of PDMS with embedded nanowires.
FIG. 8h shows an EDS-element mapping corresponding to the FESEM images from the top surface of FIG. 8g. Si and Zn are represented by blue and green (dotted bright areas on the gray scale), respectively.
FIG. 9 relates to the mechanical stability of the incorporated nanowires and the non-incorporated nanowires.
FIGS. 9a and b are schematic views of nanowires incorporated into PDMS before being exposed into lysis buffer and nanowires on a Si-substrate. Components include nanowires, PDMS, a Si-substrate, and a Cr-layer. Non-incorporated nanowires were fabricated on top of a thermally oxidized chromium layer on the Si substrate.
FIGS. 9c and d are FESEM images of the nanowires incorporated into PDMS and the top surface of the nanowires on the Si-substrate, respectively. The scale bars indicate 1 µm.
FIGS. 9e and f are FESEM images from the nanowires incorporated into PDMS and the nanowires on the Si-substrate, respectively, when exposed to lysis buffer. The scale bars indicate 1 µm. After exposure to the lysis buffer, the nanowires on the Si-substrate peeled off, whereas the nanowires incorporated into PDMS did not peel off.

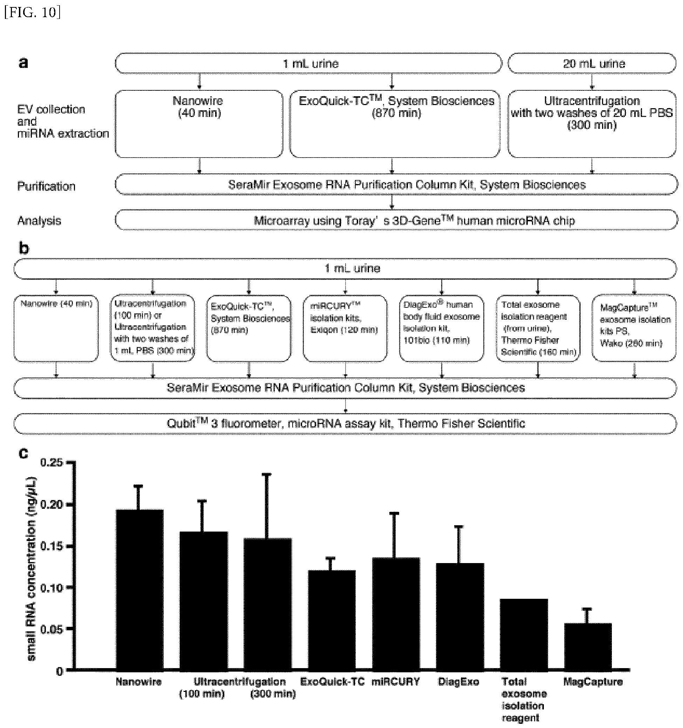

FIG. 10 shows an example of processes for extracting urinary miRNA using nanowire incorporated devices, ultracentrifugation, and commercially available kits.
FIG. 10a shows the experimental procedure of microarray analysis of the expression of miRNA.
FIG. 10b shows the experimental procedure for the quantitation of small-molecule RNAs using Qubit(trademark) microRNA assay kit (Thermo Fisher Scientific). RNA generation and quantitation of small RNAs were carried out after extracting miRNA by various techniques.
FIG. 10c shows the quantification of small molecule RNA using various methods. In Qubit(trademark) microRNA assay kit (Thermo Fisher Scientific), since not only miRNA but also small molecular RNAs (~20 nucleotides or base pairs) can be quantified, the concentration of small molecular RNAs is set on the Y-axis. According to these data, the washing step did not affect the RNA collection efficiency. The error bars indicate SD for the measurements (N ≧ 3).

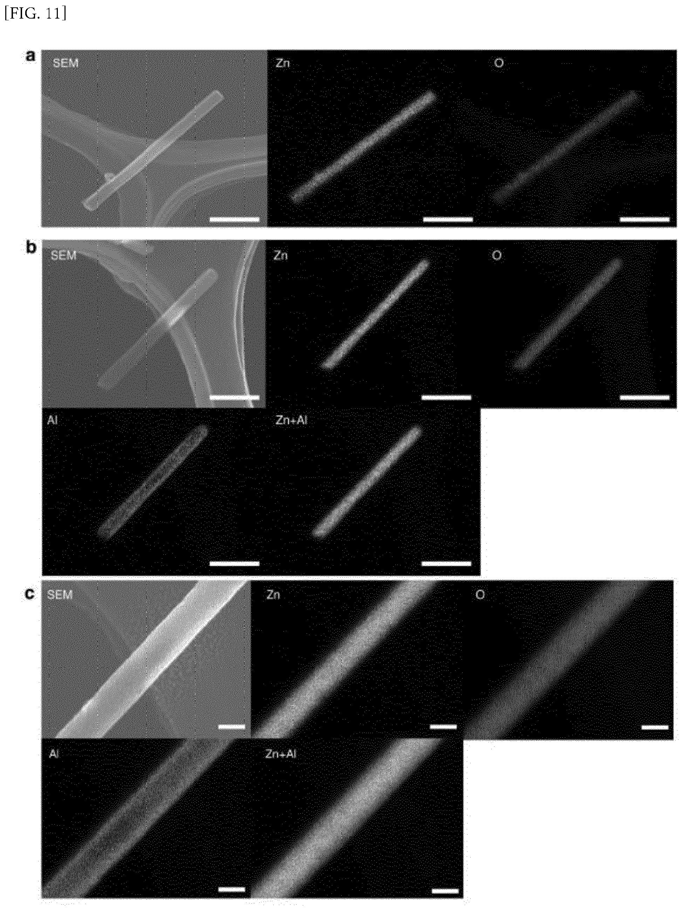

FIG. 11 shows FESEM images of one nanowire and the EDS-element mapping of STEM image. Zn, O, and Al are shown in green, red, and orange, respectively.
FIG. 11a shows a nanowire of ZnO alone. The scale bar indicates 500 nm.
FIG. 11b shows a ZnO/Al₂O₃ core-shell nanowire.
   The scale bar indicates 500 nm. In FIG. 11b, A1 was seen to cover the Zn core.
FIG. 11c shows a ZnO/Al₂O₃ core-shell nanowire. The scale bar indicates 100 nm. In FIG. 11b, Al was seen to cover the core of Zn, and O was seen to overlap Al and Zn.

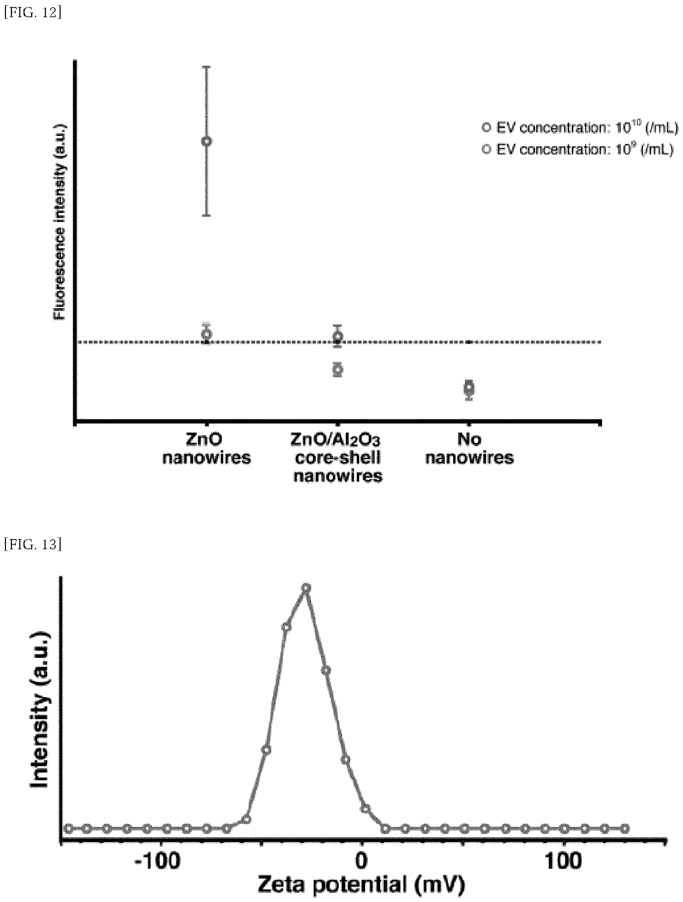

FIG. 12 relates to detection of EV on ZnO nanowires, ZnO/ Al₂O₃ core-shell nanowires, and a surface without nanowires, with anti-CD9 antibody. The dotted line indicates the 3SD signal intensity above background. The error bars are standard deviations (SDs) of the measurements of ZnO nanowires, ZnO/Al₂O₃ core-shell nanowires, and a surface without nanowires (n=40, 24, and 24, respectively). The points on the graph show the signal intensities when miRNA were extracted on ZnO nanowires, ZnO/Al₂O₃ core-shell nanowires, and a surface without nanowires for samples with EV concentrations of 10⁹/mL and 10¹⁰/mL. After introducing EVs, PBS was perfused for these three devices to remove EVs that could not be collected.
   The first device had ZnO nanowires, the second device had ZnO/Al₂O₃ core-shell nanowires, and the third device had no nanowires.
   1%BSA solution was introduced into these devices and allowed to stand for 15 minutes. After washing these devices with PBS, mouse anti-human CD9 antibody (10µg/mL, Abcam, Plc.) was introduced into each device, after which the device was allowed to rest for 15 minutes. CD9 is known as membrane proteins expressed on exosomes.
   In addition, each device was washed using PBS, and each device was introduced with AlexaFluor488 labeled goat polyclonal anti-mouse IgG antibody (5µg/mL, Abcam, Plc.), after which the device was allowed to rest for 15 minutes. Finally, each device was washed with PBS and fluorescence intensity was observed by fluorescence microscopy (Olympus, Co. Ltd.). ZnO nanowires effectively collected EV, whereas ZnO/Al₂O₃ core-shell nanowires only slightly collected EV. This suggests the importance of the surface charge of the nanowires.
FIG. 13 shows the zeta potential of EV in urine. The zeta-potential of the EVs was measured using a dynamic-light-scattering device (Zetasizer Nano-ZS, Malvern Instruments, Ltd.). The average zeta potential of the EV was -28 mV.

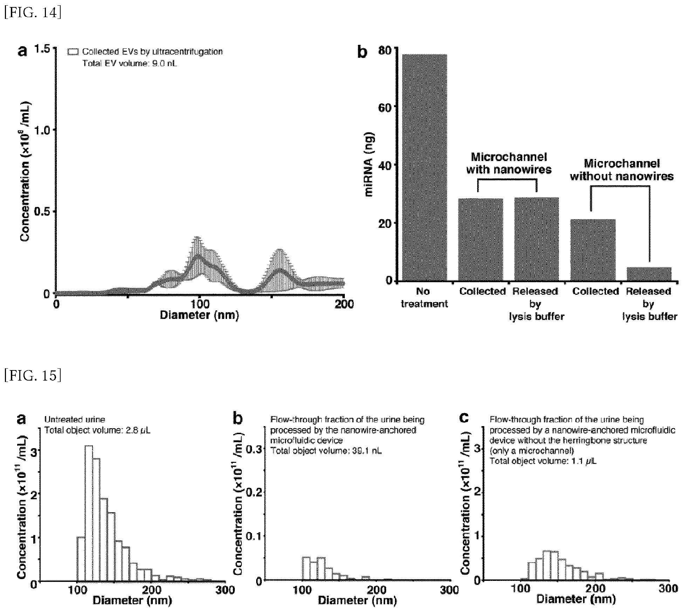

FIG. 14 shows the size distribution of EV collected by ultracentrifugation. FIG. 14 also shows the presence of EV-free miRNA collected on the nanowires.
   In FIG. 14a, the concentration of the collected EV was 1.6×10⁹ mL-1. The error bars indicate the standard deviation for the measurements (n=3).
FIG. 14b shows the amount of miRNA collected and miRNA released by quantitative reverse transcription-polymerase chain reaction (qRT-PCR). As an EV-free miRNA, 100 nM miRNA (sequence: miRNA(sequence, uugcauagucacaaaagugauc) was used. "Untreated" indicates the quantity of 100 nM miRNA. The collection rates of microchannels with or without nanowires were 100% and 23%, respectively.
FIG. 15 shows the size distributions of the free suspended solids of urine. The size-distribution and concentration of urinary free suspended solids were determined using a nanoparticle detector (qNano, Meiwafosis Co, Ltd.) equipped with a 100 nm nanopore membrane (NP100 ,Meiwafosis Co, Ltd).
FIG. 15a is data on free suspended solids of untreated urine and the concentration was 1.4 × 10¹² mL-1.
FIG. 15b is data on free suspended solids in the flow-through fraction of urine processed by the nanowire-incorporated device, with a concentration of 2.4 × 10¹⁰ mL-1 (capture efficiency of 99%).
FIG. 15c is data on free suspended solids in the flow-through fraction of urine processed with a nanowire-incorporated device without herringbone structure, with a concentration of 4.3 × 10¹¹ mL-1 (capture efficiency of 61%).

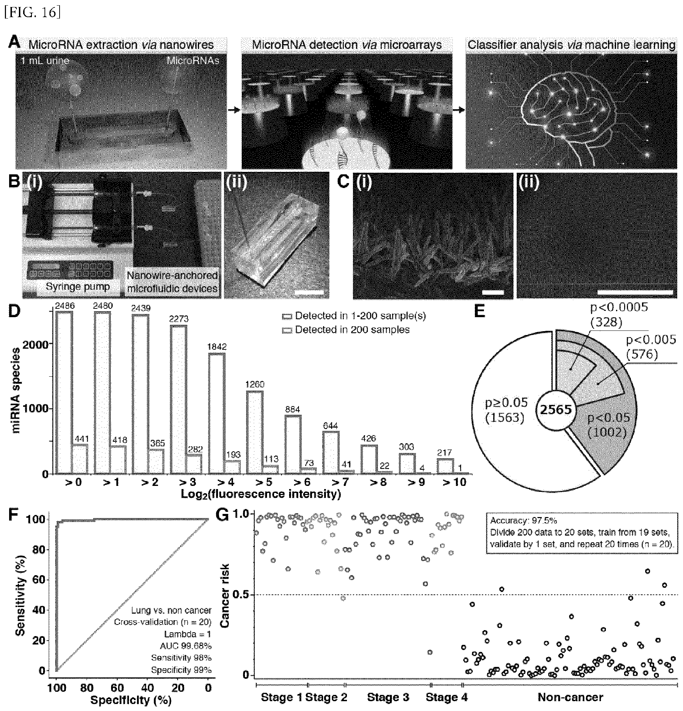

FIG. 16 shows: schematic diagrams and photographs of the present microRNA extracting system(A-C); the numbers of miRNA detected (D); the numbers of miRNA showing significant differences between the urine of a lung cancer patients and the urine of healthy persons (E); a ROC curve based on data obtained by randomly dividing the patients and the healthy persons into 20 groups, deriving a criterion formula from the results of 19 groups obtained randomly, and predicting the remaining group for 20 cycles (F); and a plot of Cancer risk of each subject divided by stage (Cancer risk) derived by the criterion formula on the vertical axis (G).

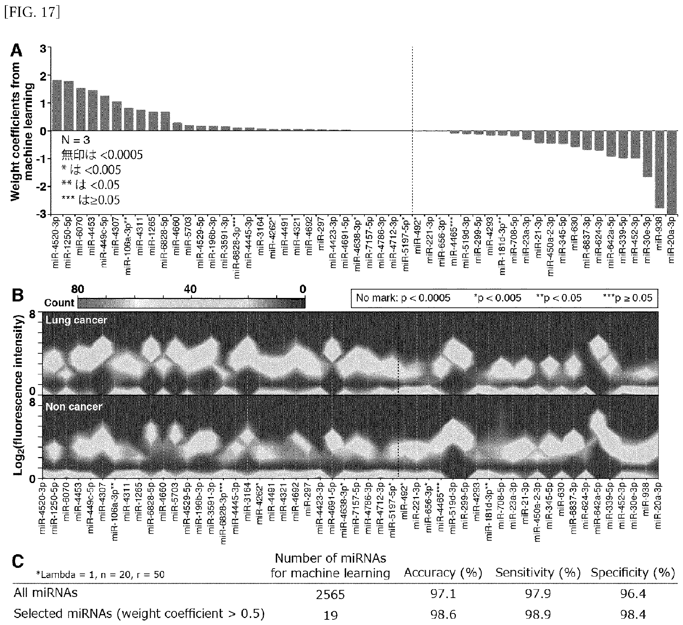

FIG. 17 shows: a weighting coefficient for miRNA in which the absolute value of the weighting coefficient is larger than 0.01 in the criterion formula obtained in FIG. 16 (A); a heat map produced from the relation between log2 (fluorescent intensity) and miRNA shown in FIG. 17A (B); and accuracy (correct answer rate), sensitivity (sensitivity), and specificity (specificity) of lung cancer prediction using total miRNA, and accuracy (correct answer rate), sensitivity (sensitivity), and specificity (specificity) of lung cancer prediction using miRNA in which the absolute value of the weighting coefficient is larger than 0.05 (C).

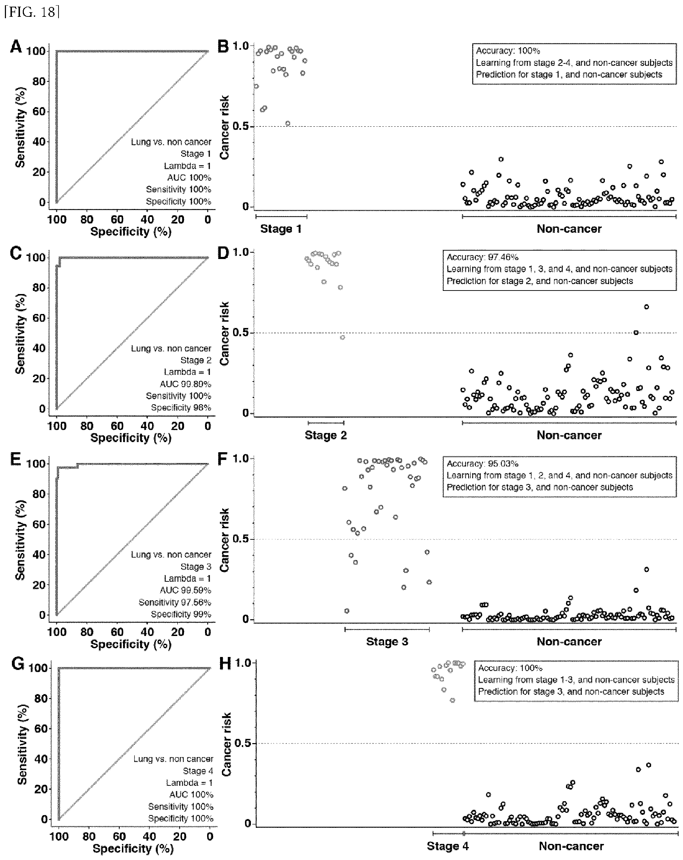

FIG. 18 shows: the results of lung cancer prediction for stage I patients (A and B), lung cancer prediction for stage II patients (C and D), lung cancer prediction for stage III patients (E and F), and lung cancer prediction for stage IV patients (G and H).

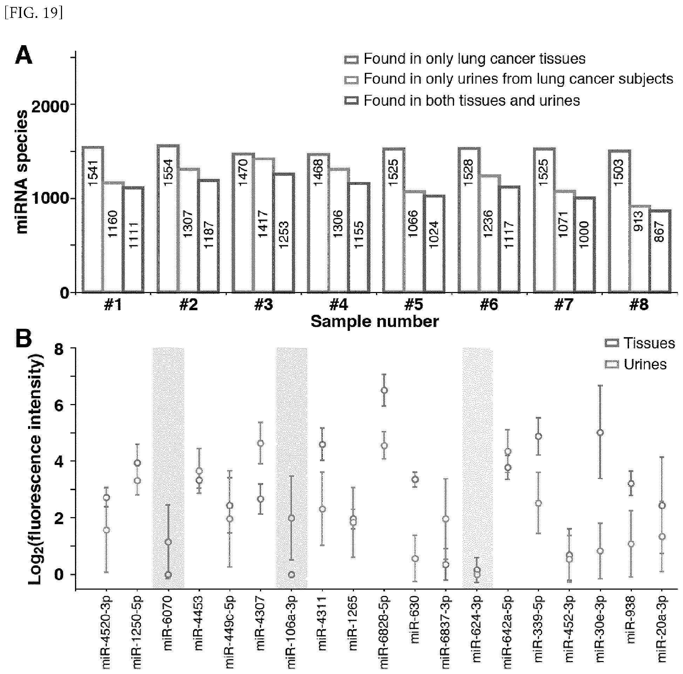

FIG. 19 shows the relation of the number of miRNA detected in the urine of lung cancer tissue and lung cancer patients (A), and the strength of tissue expression in miRNA where the absolute value of the weighting factor exceeds 0.5 (B).

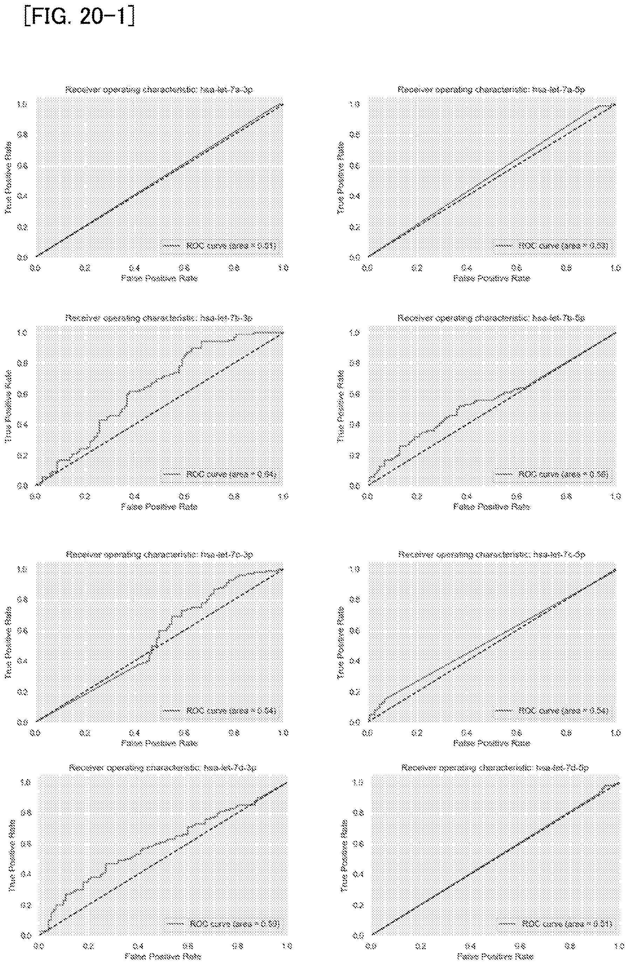

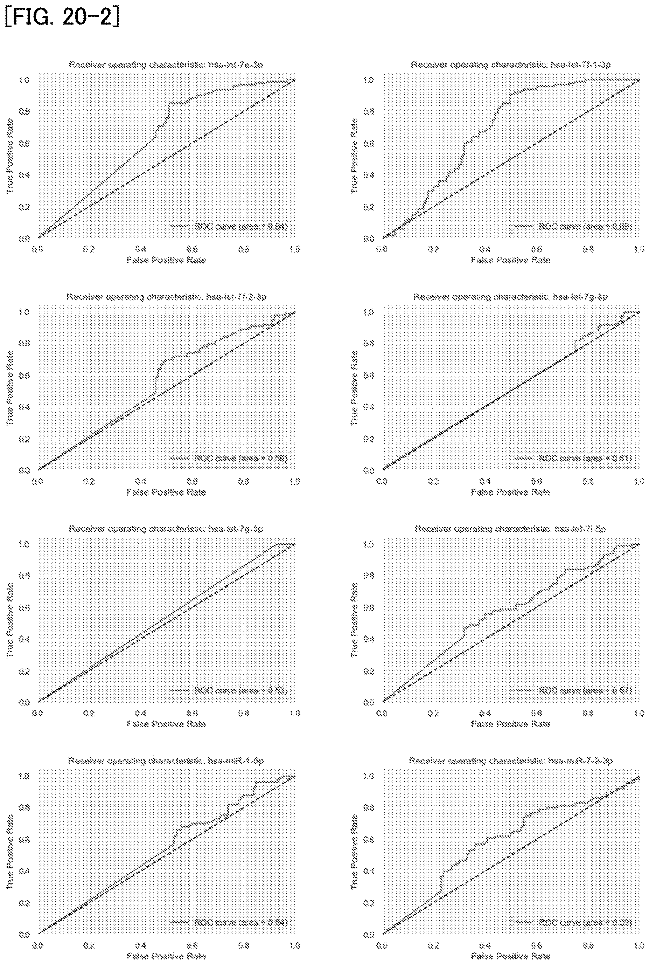

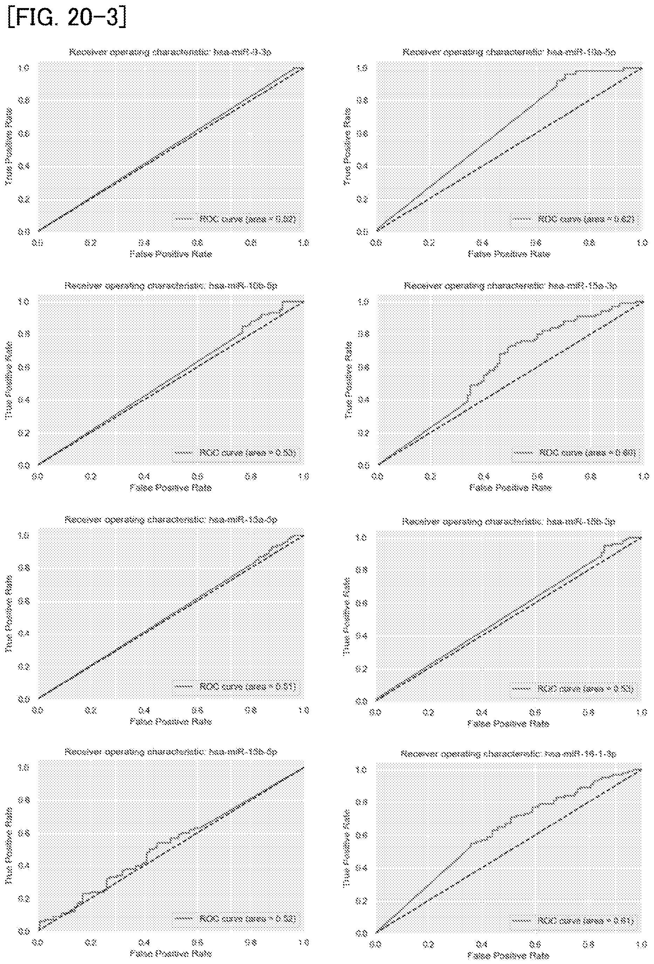

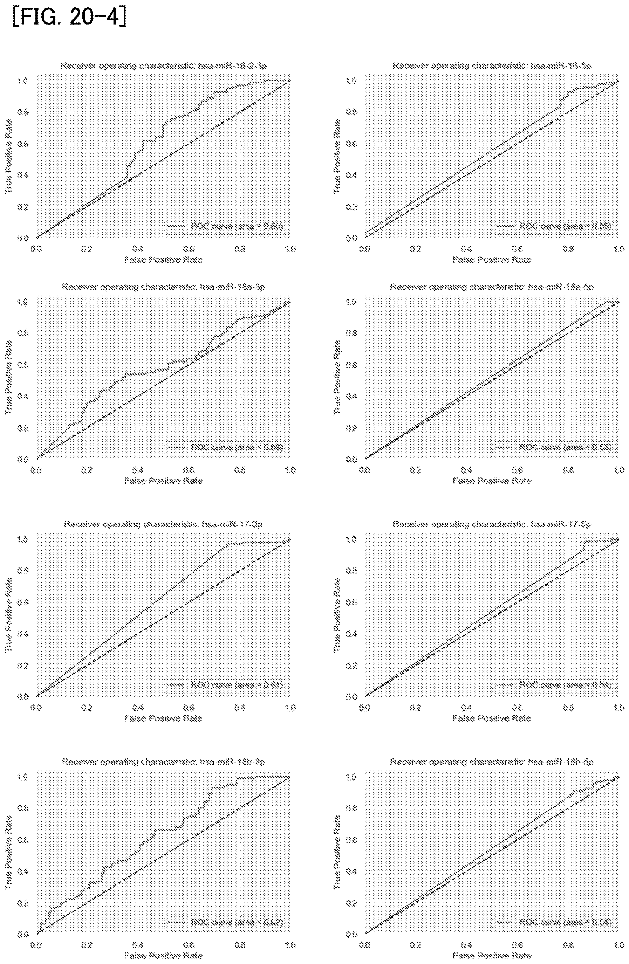

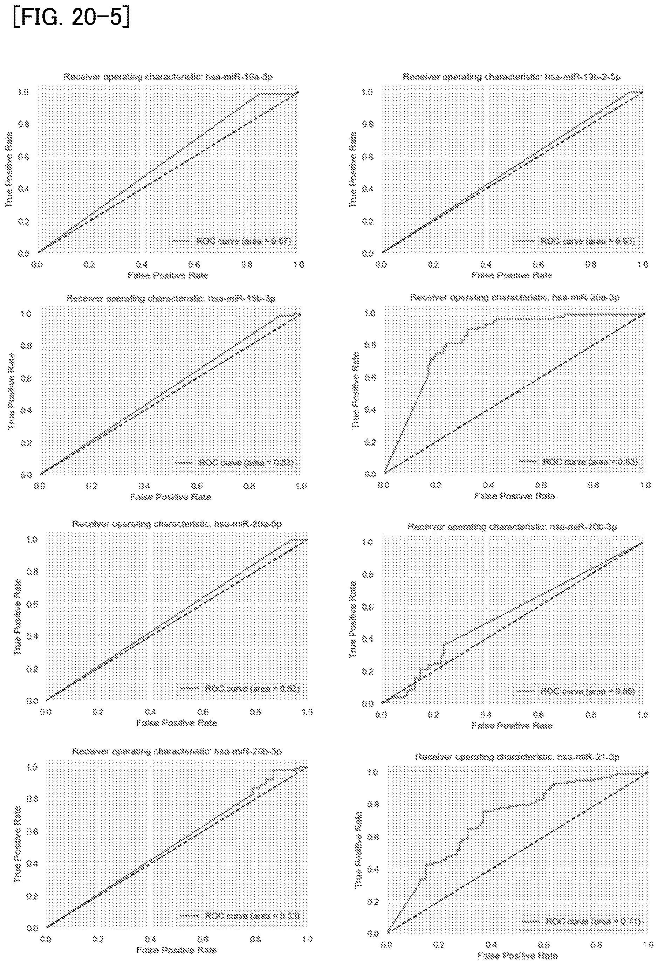

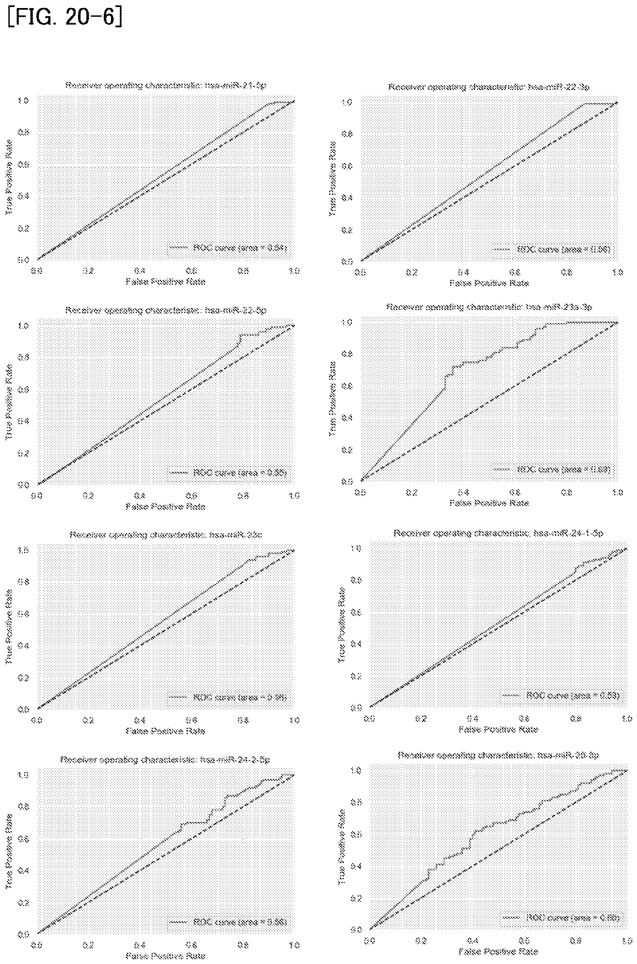

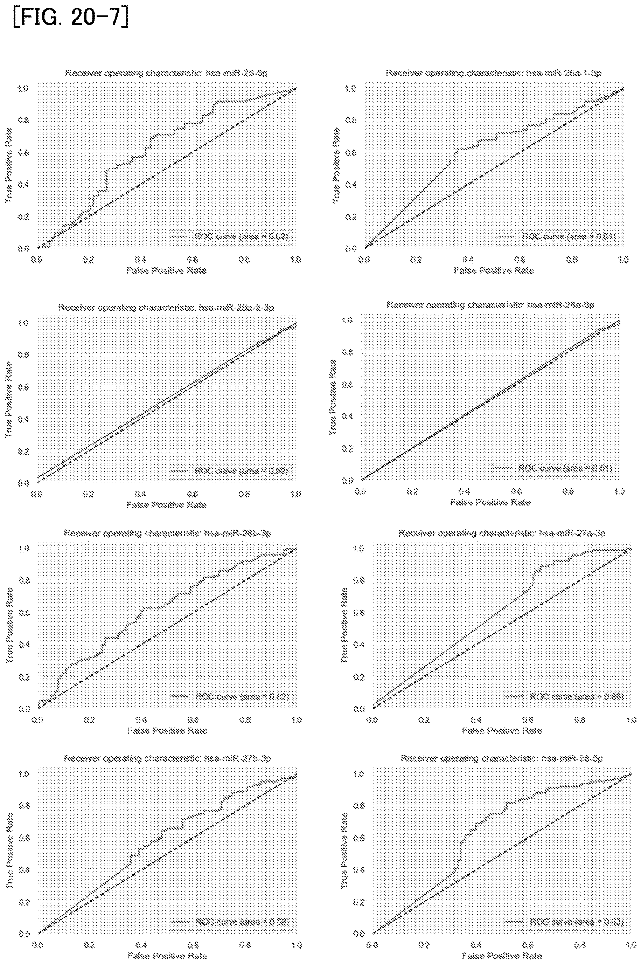

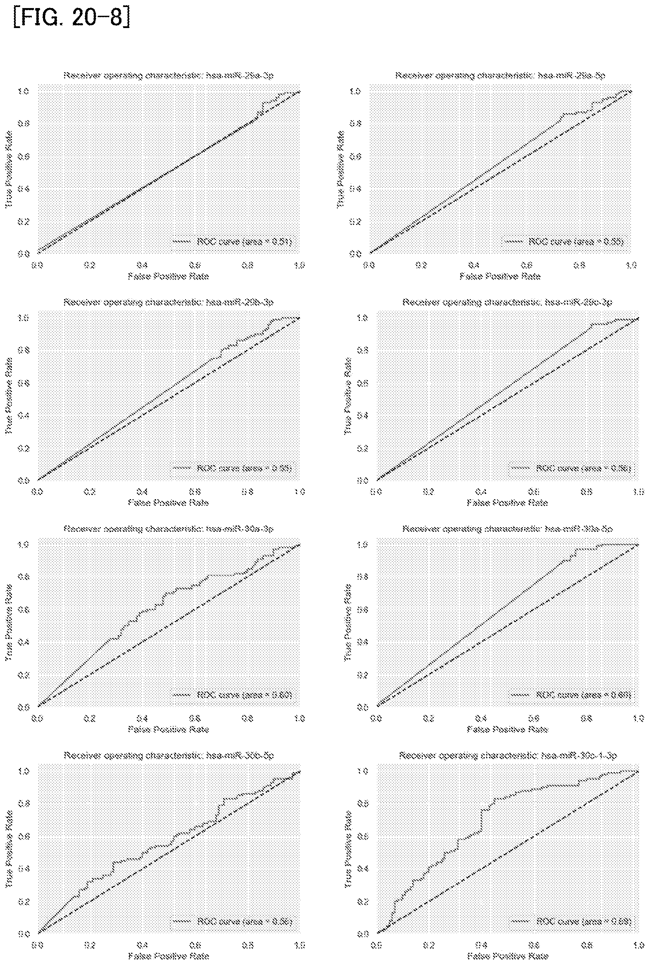

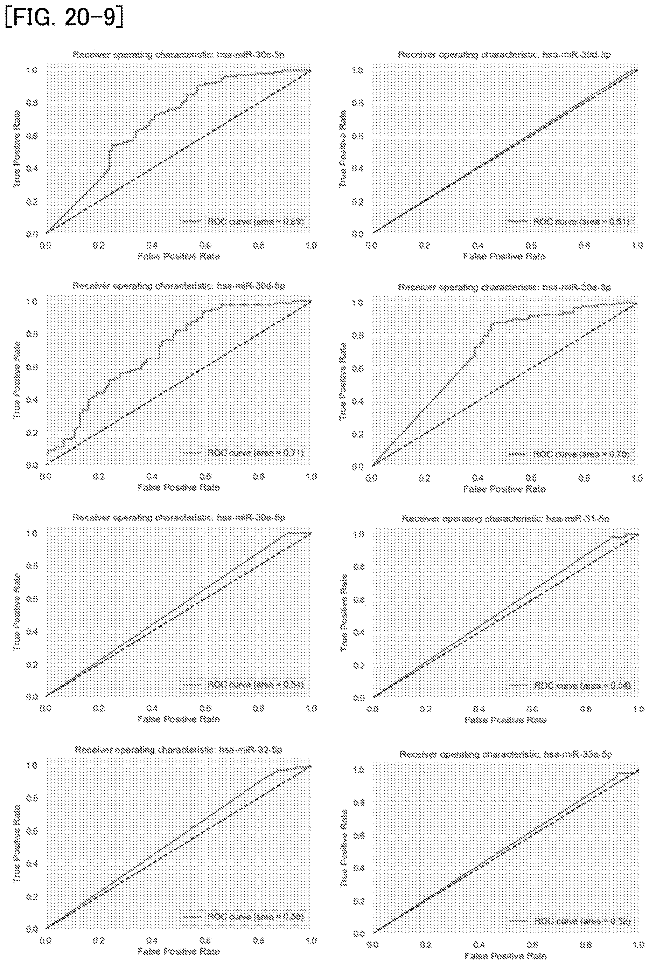

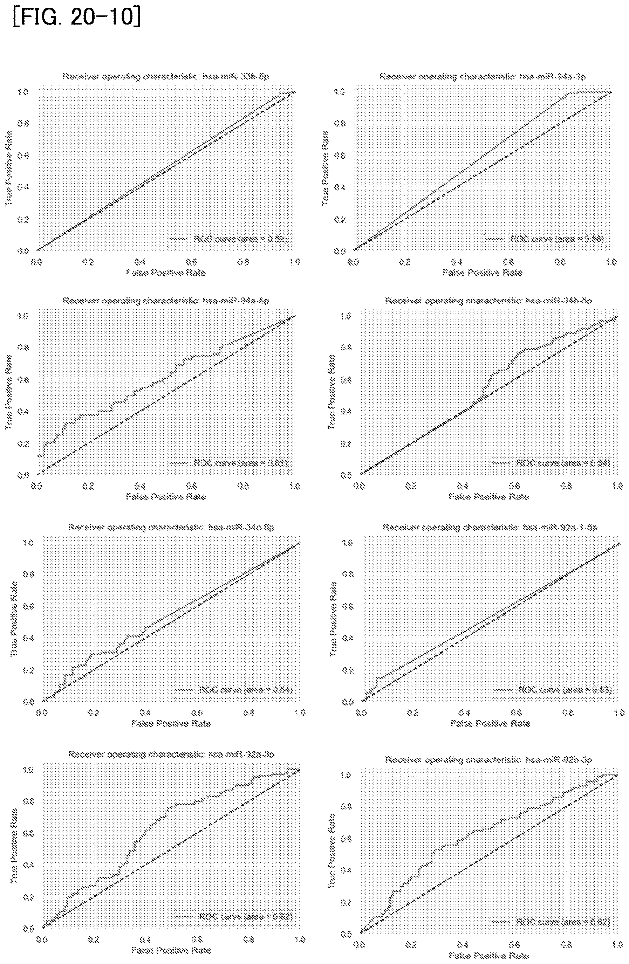

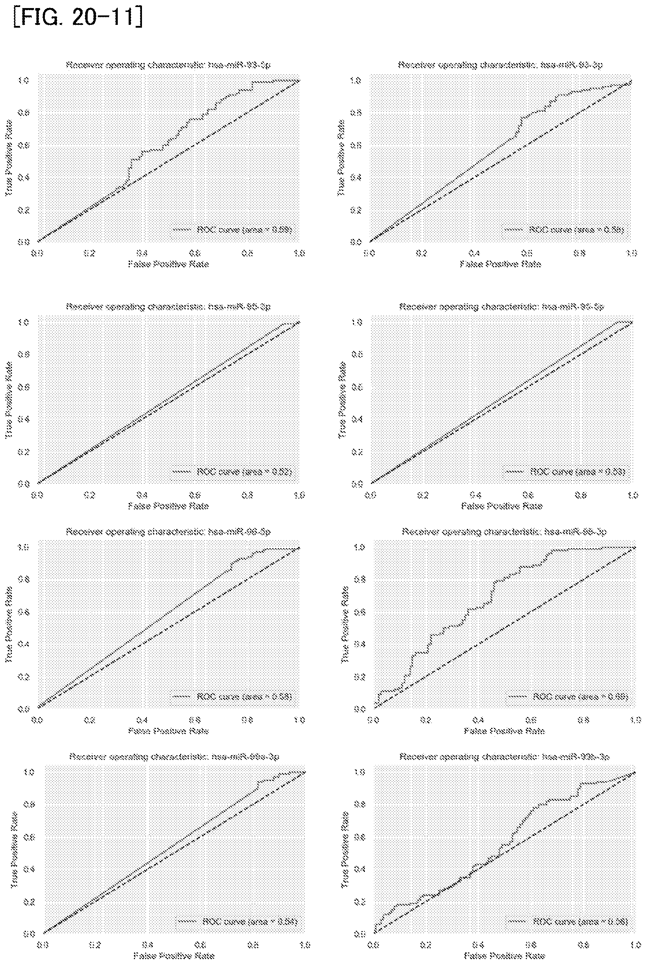

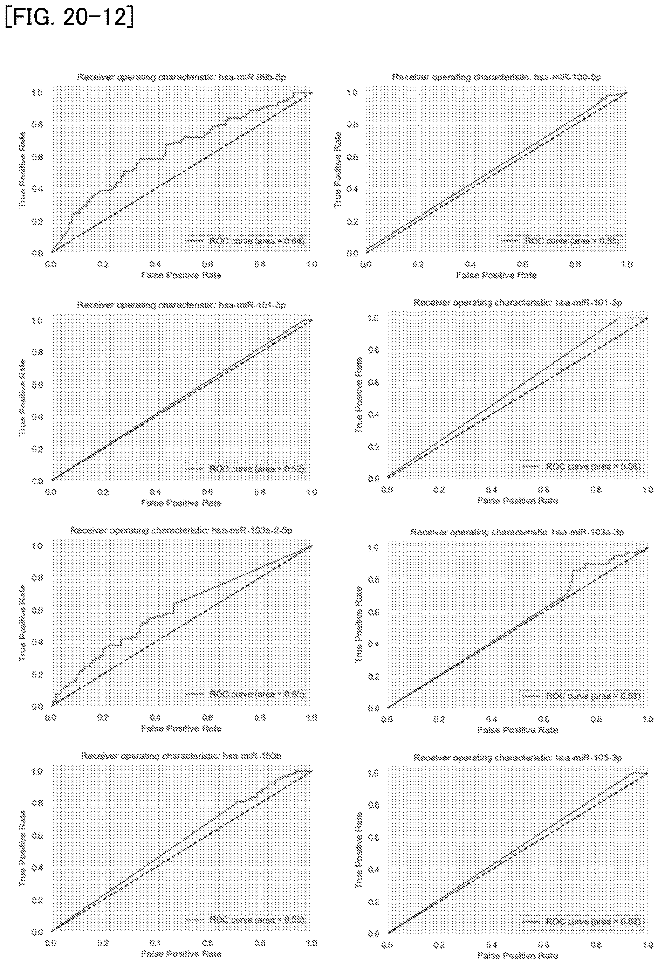

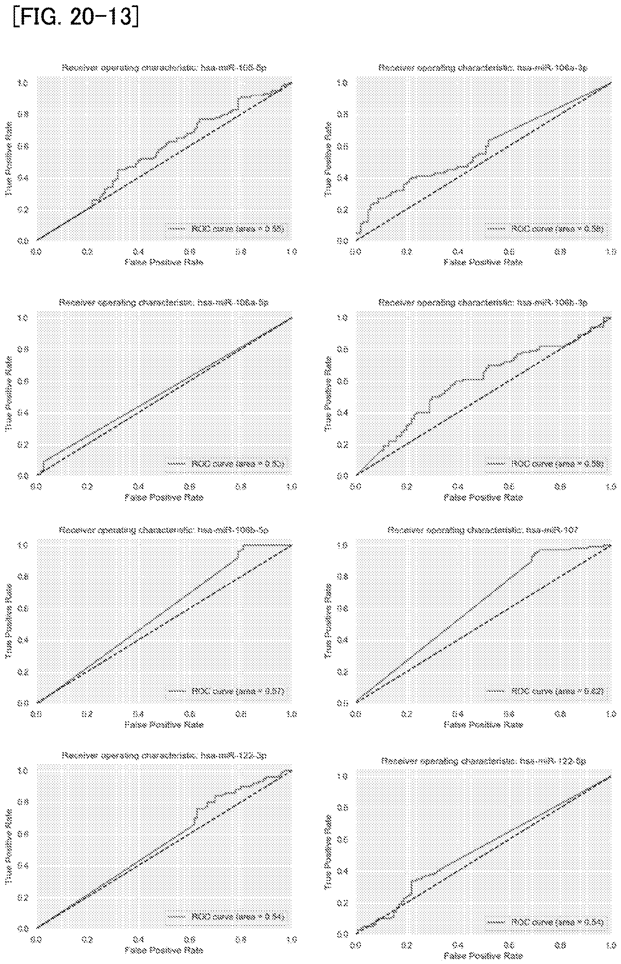

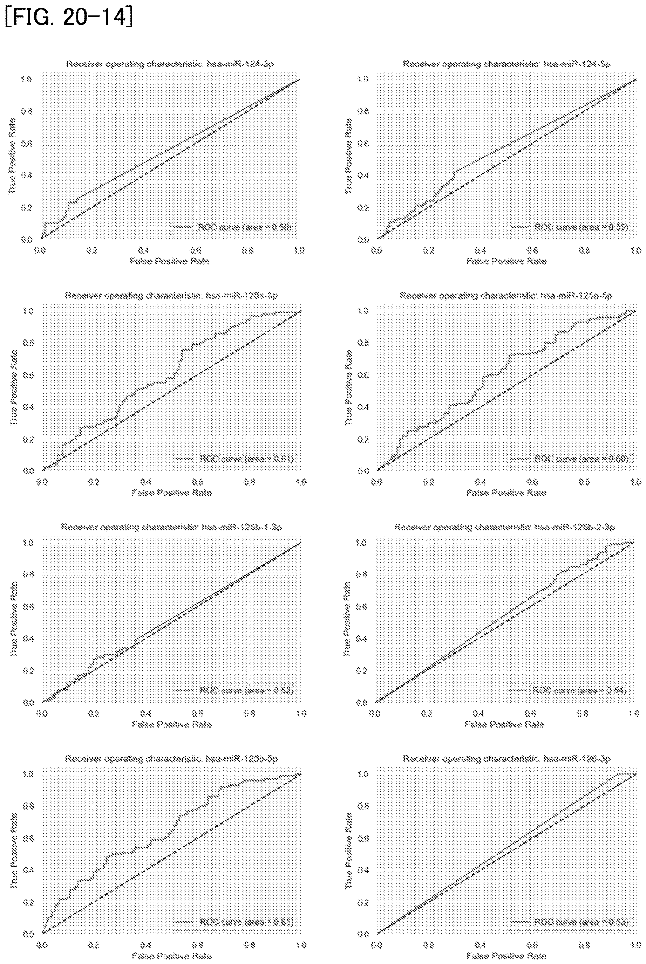

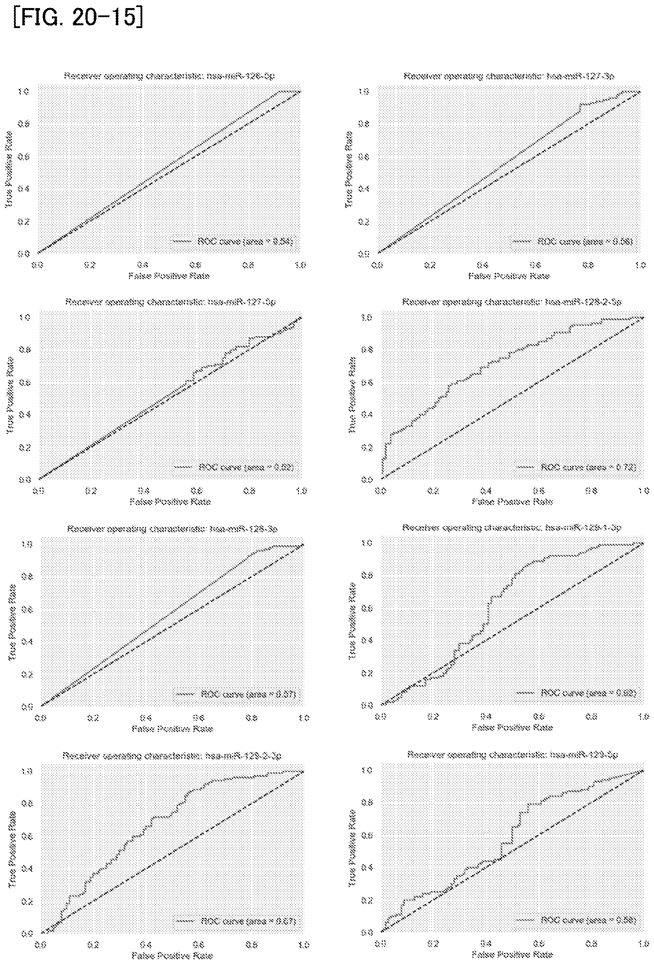

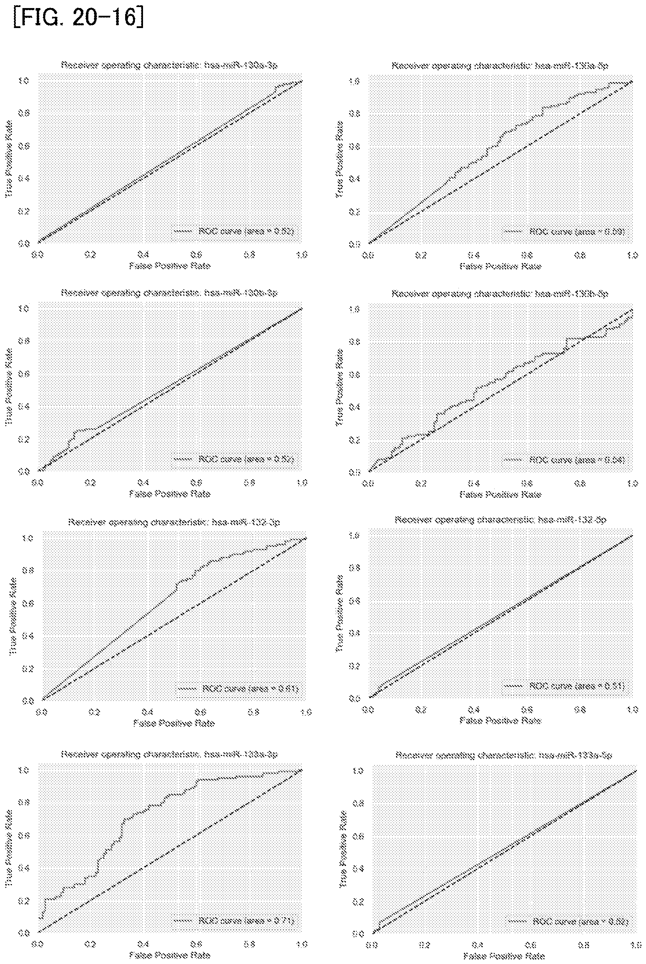

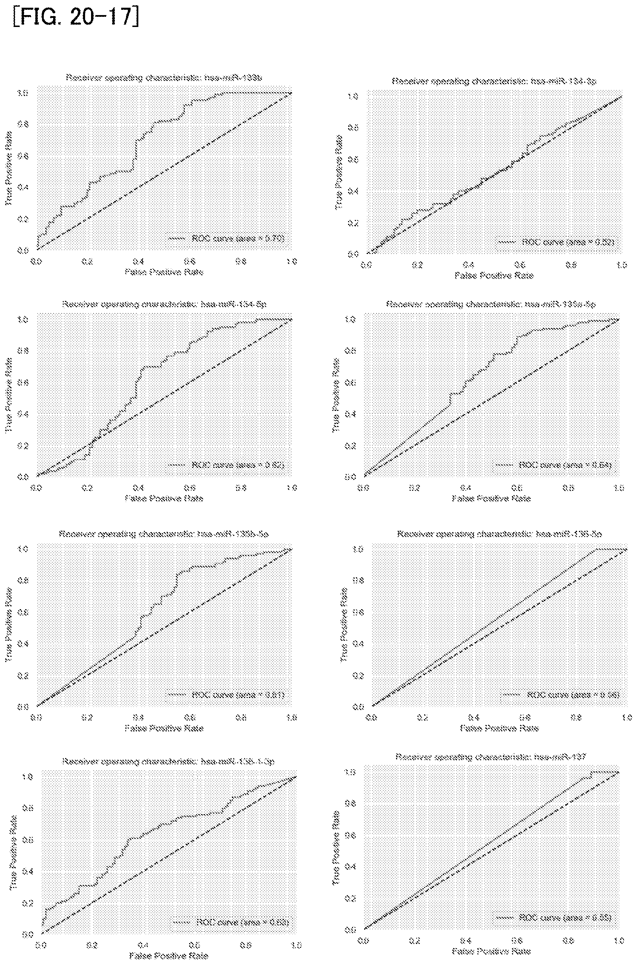

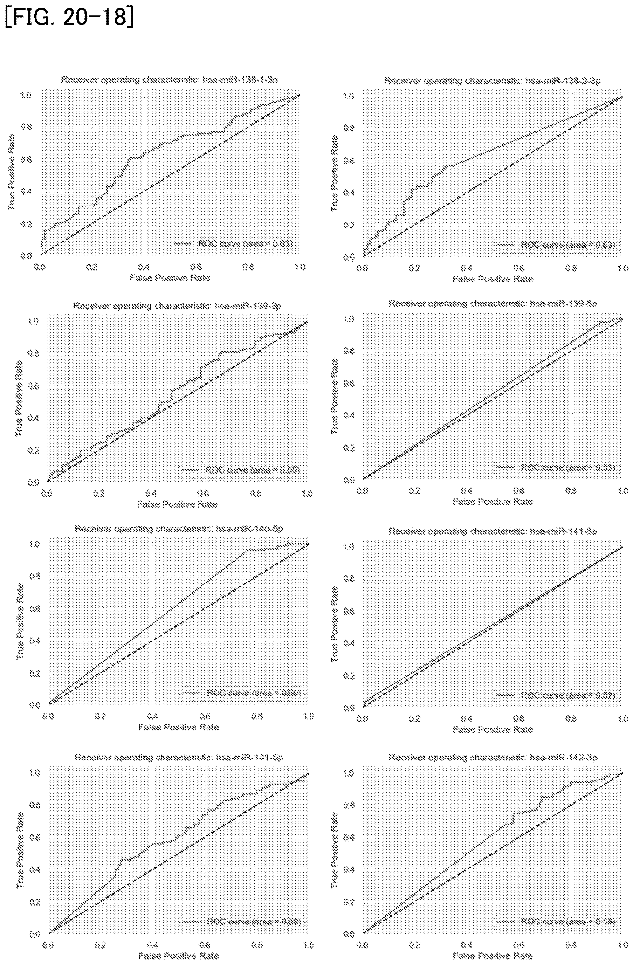

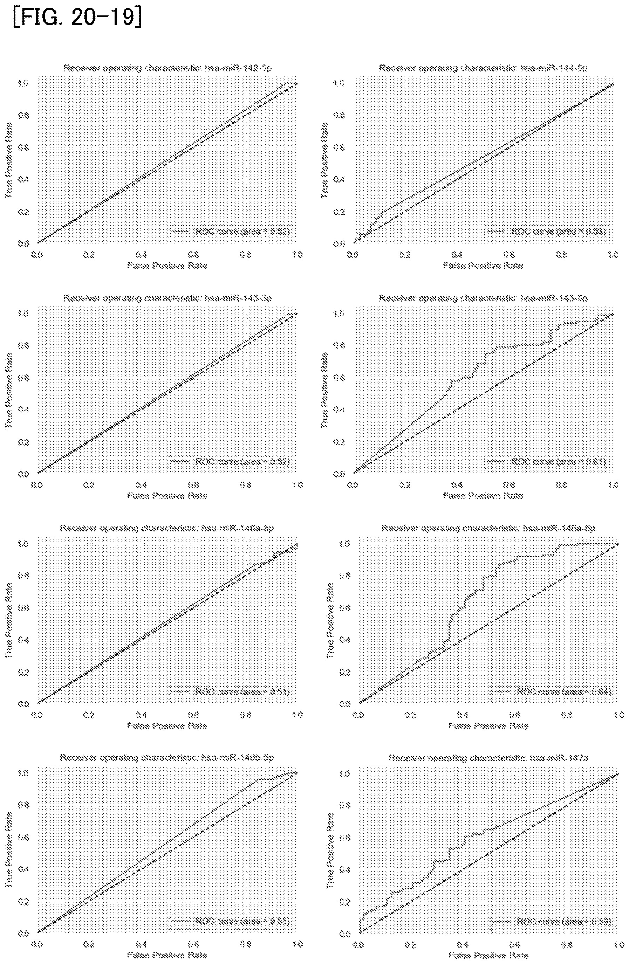

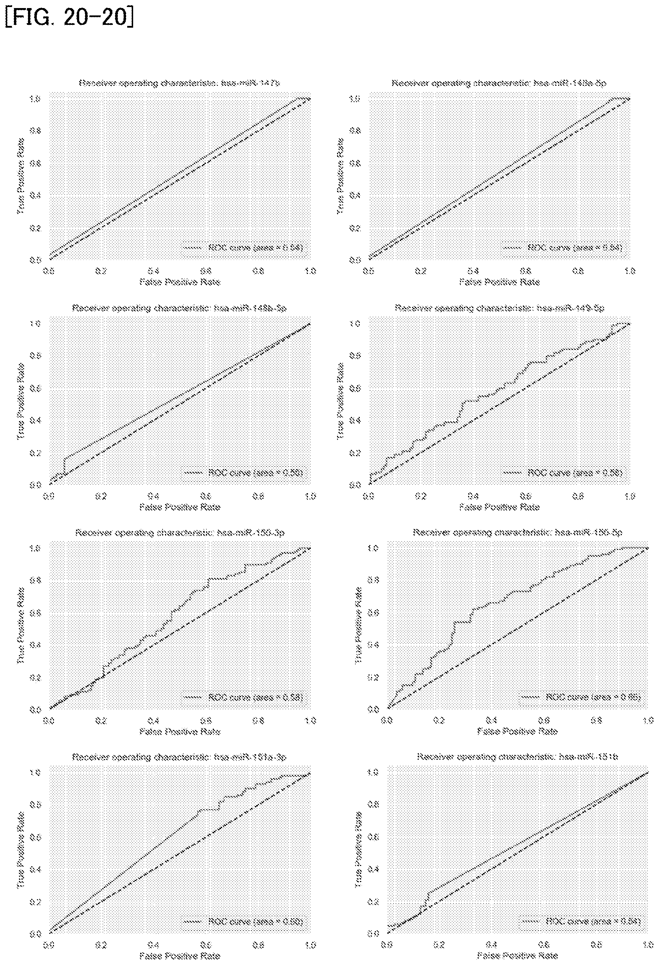

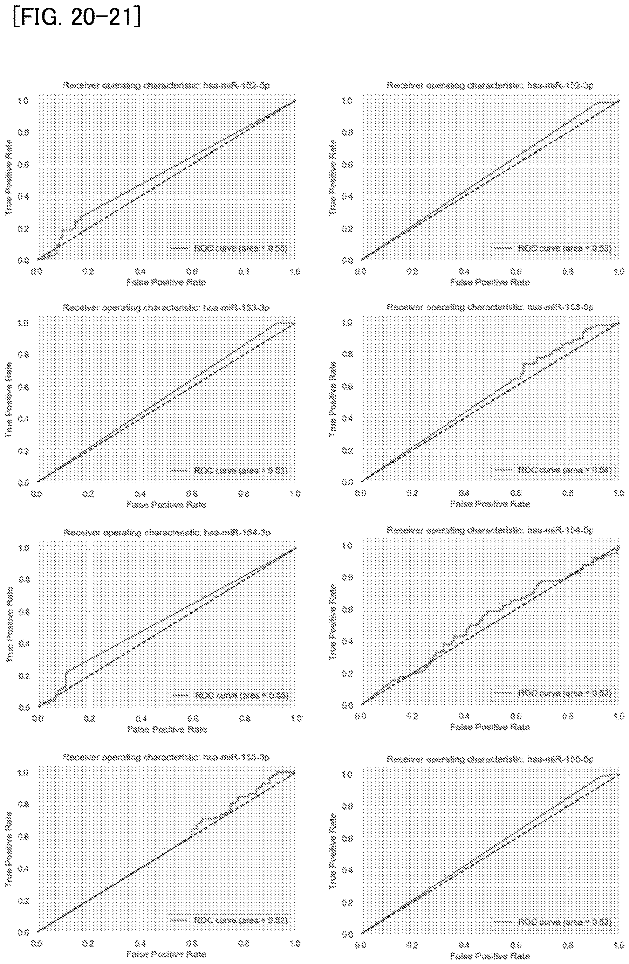

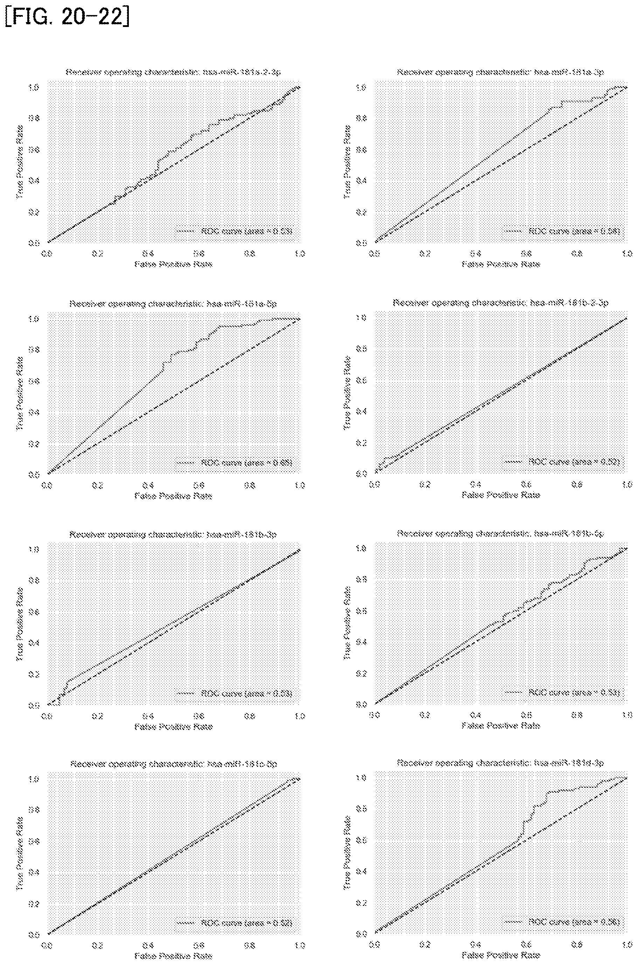

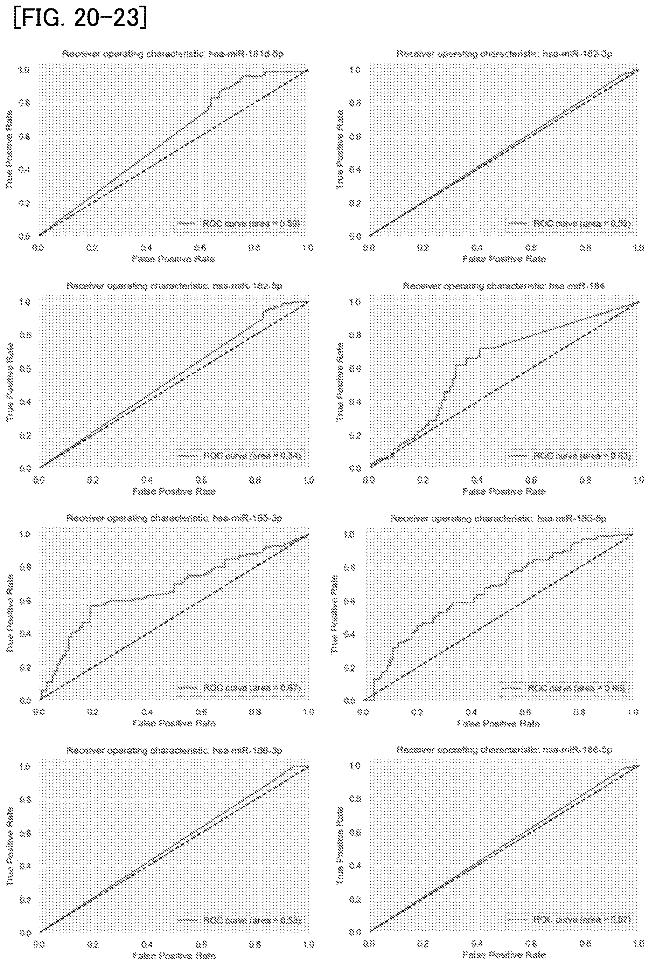

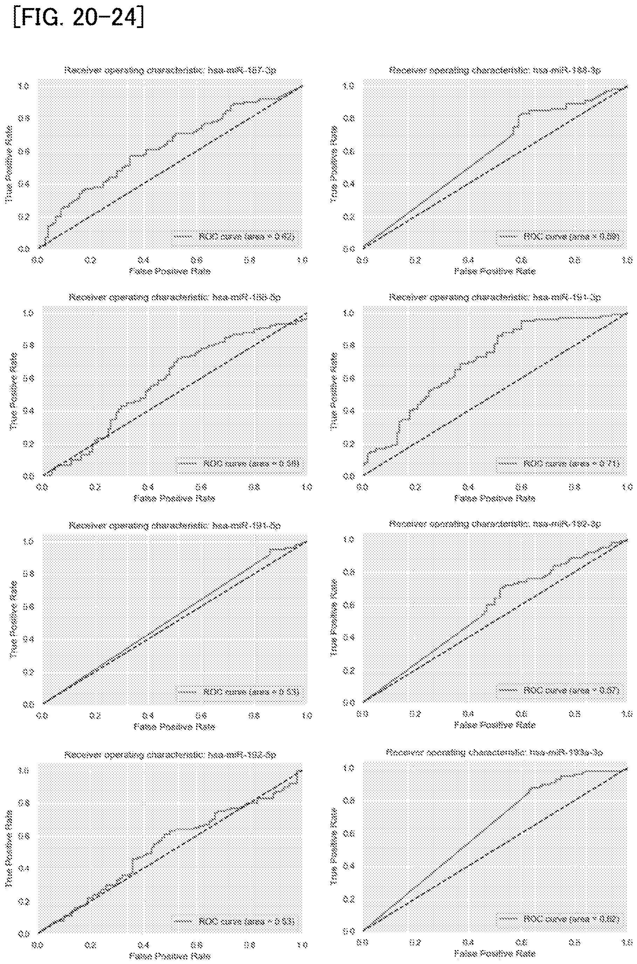

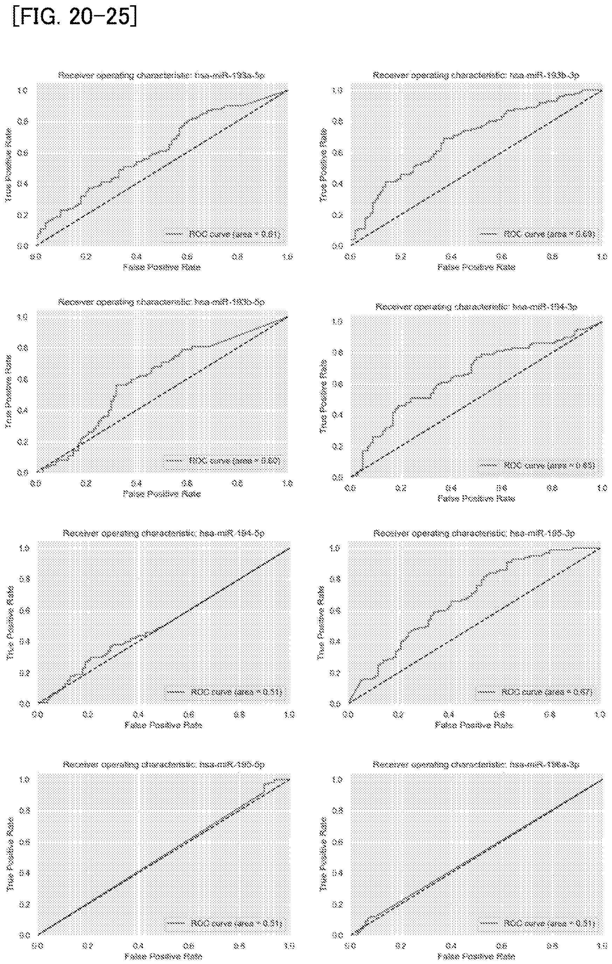

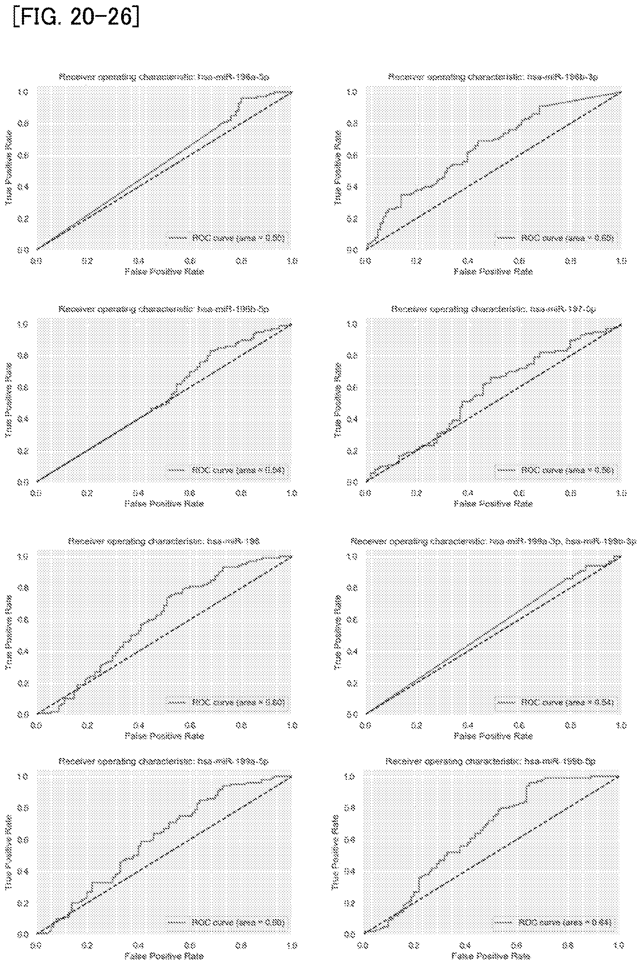

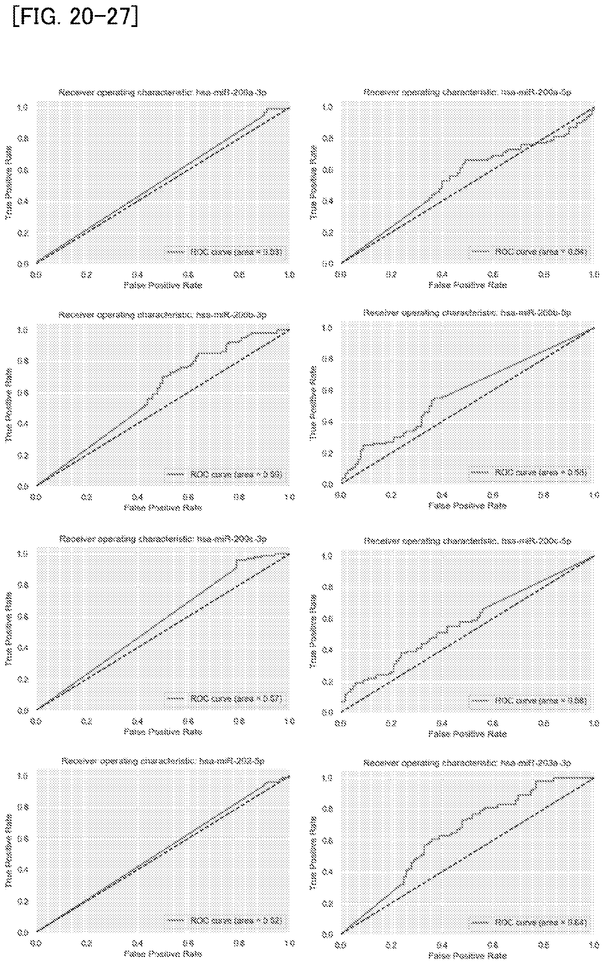

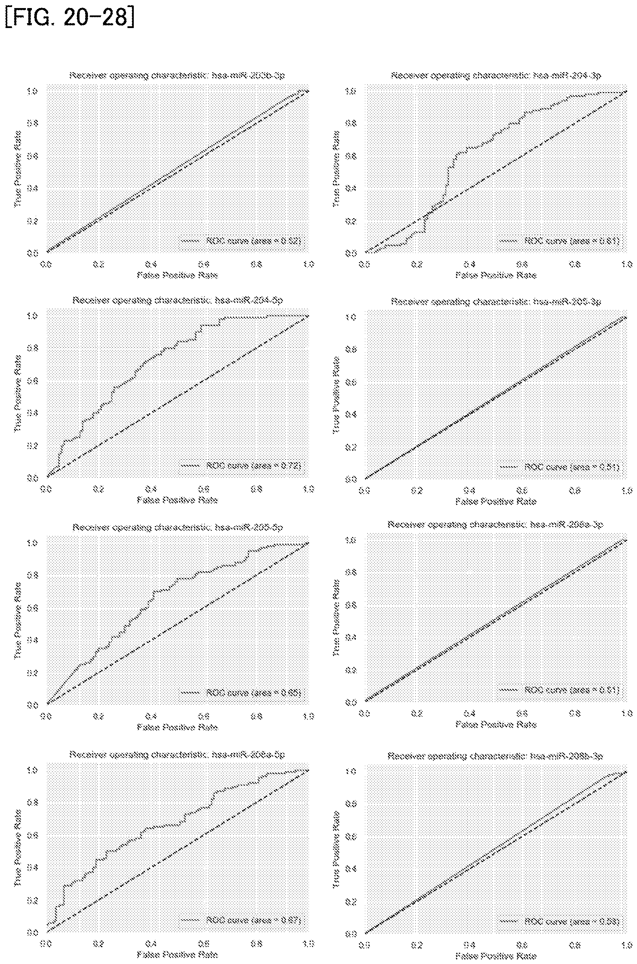

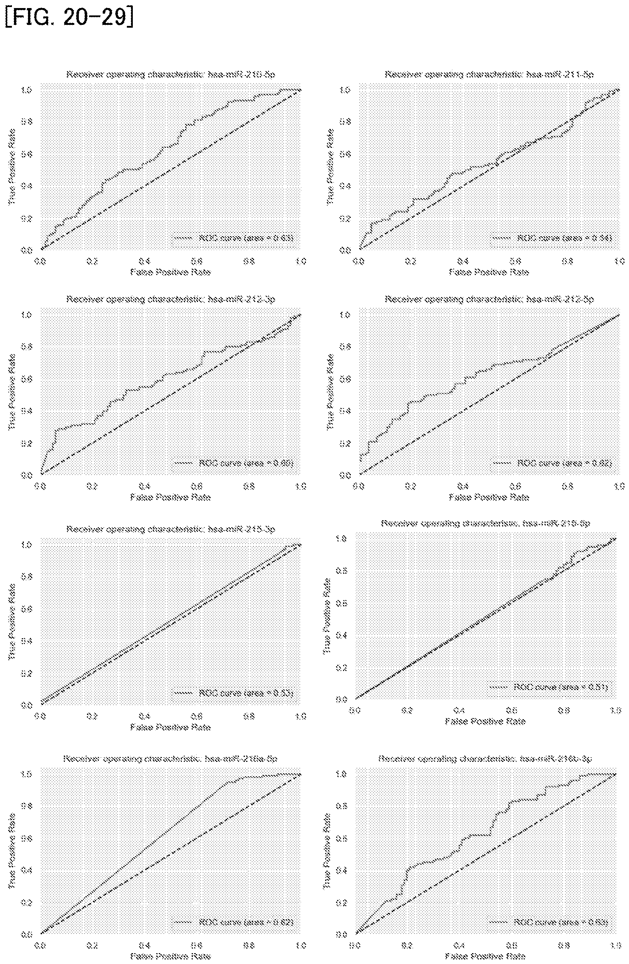

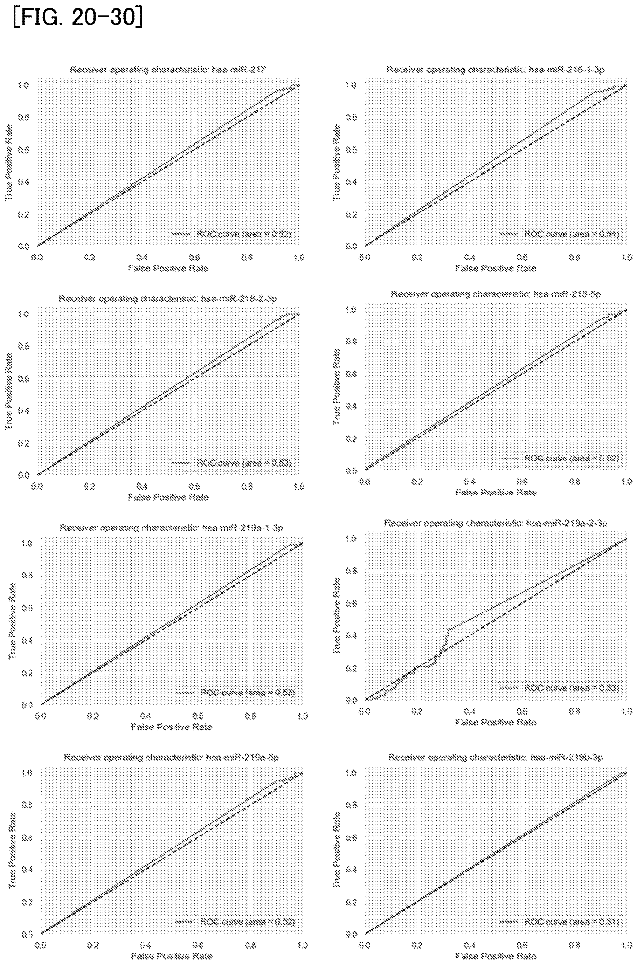

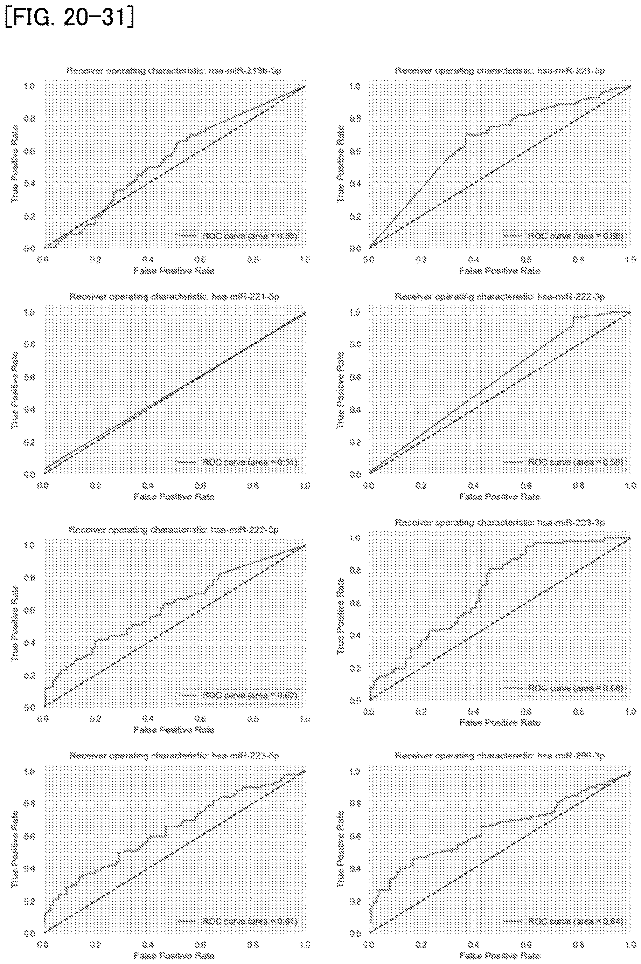

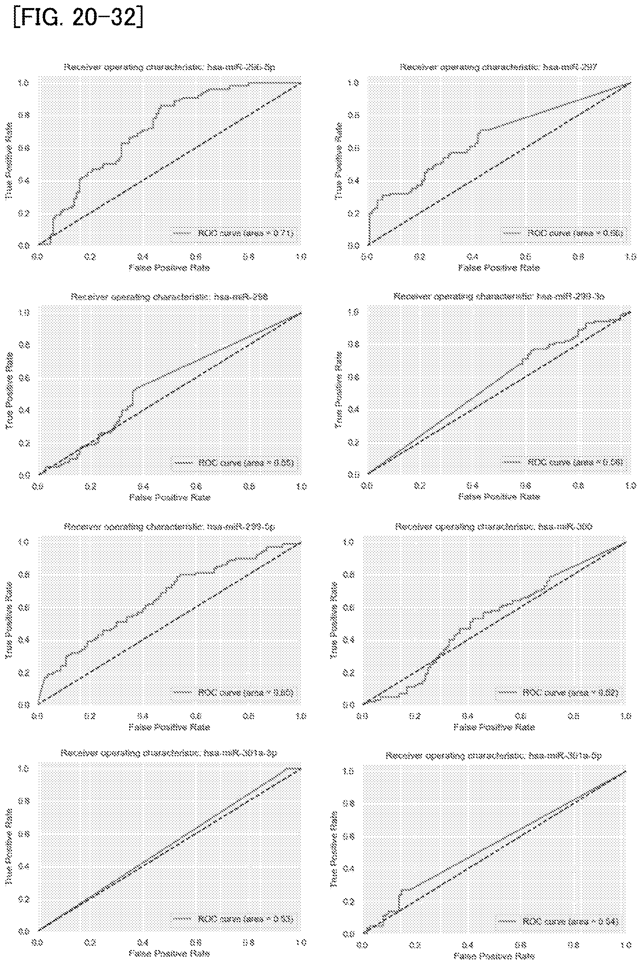

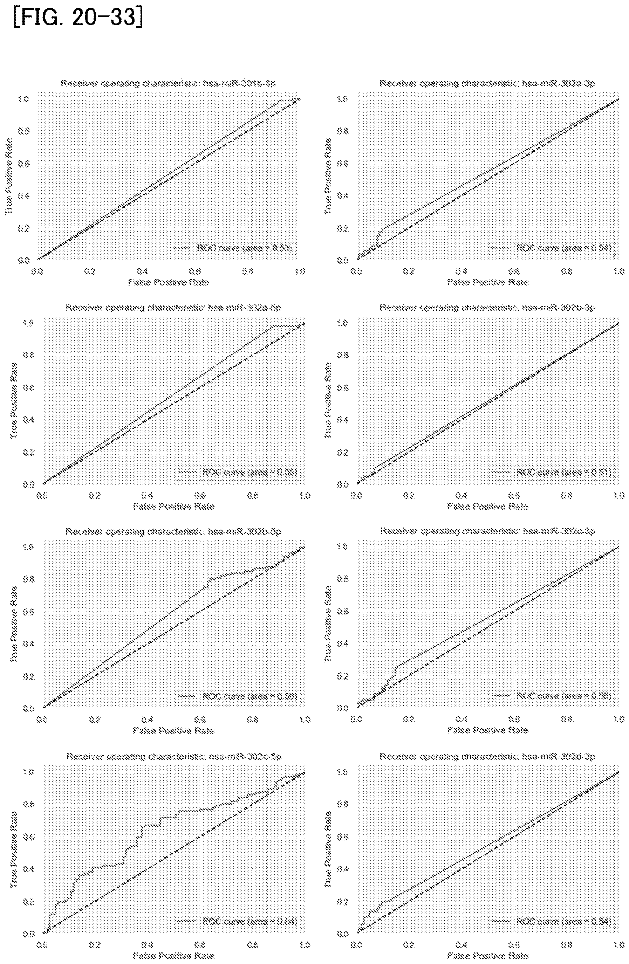

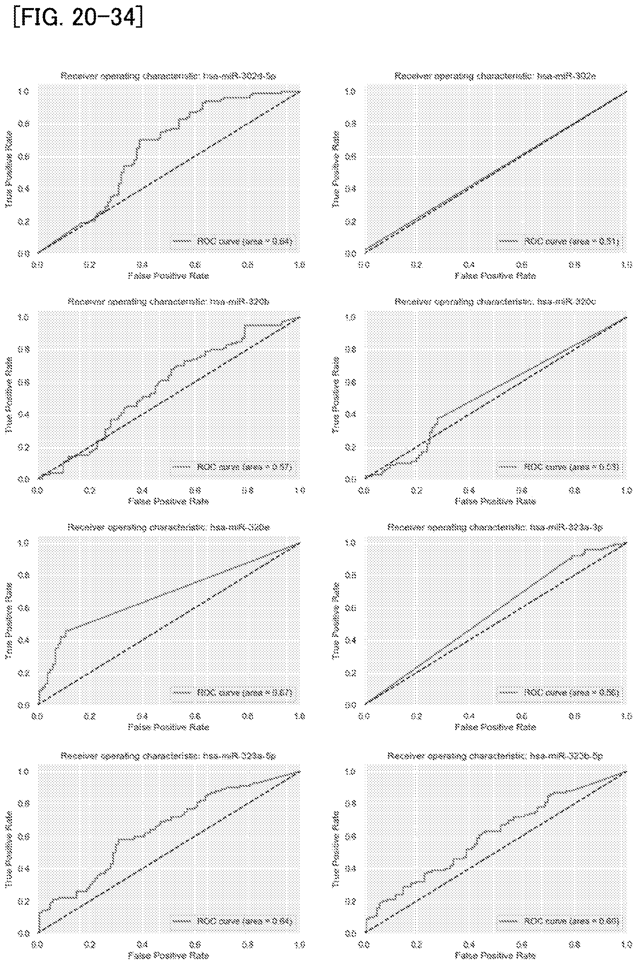

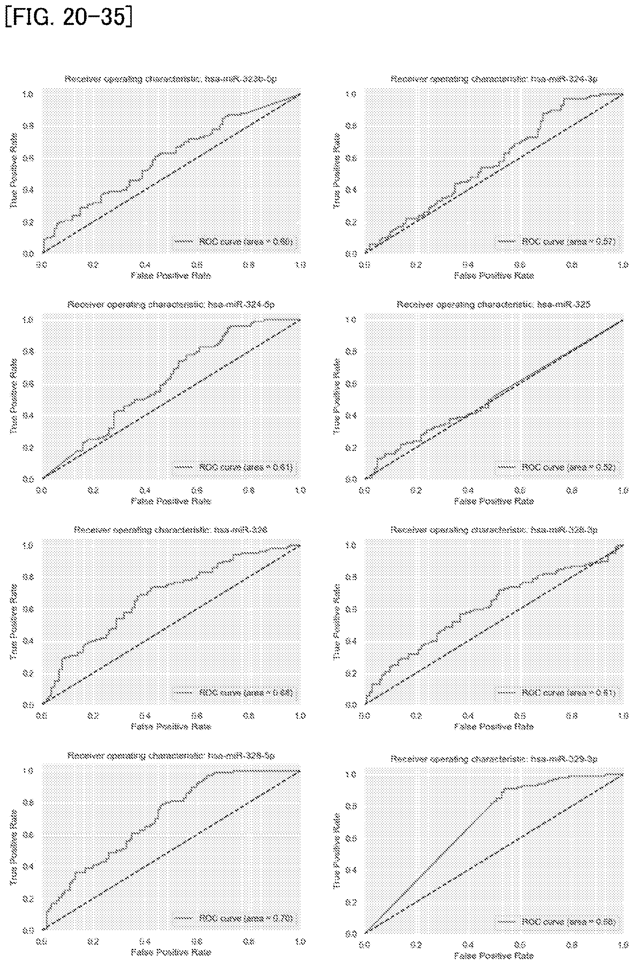

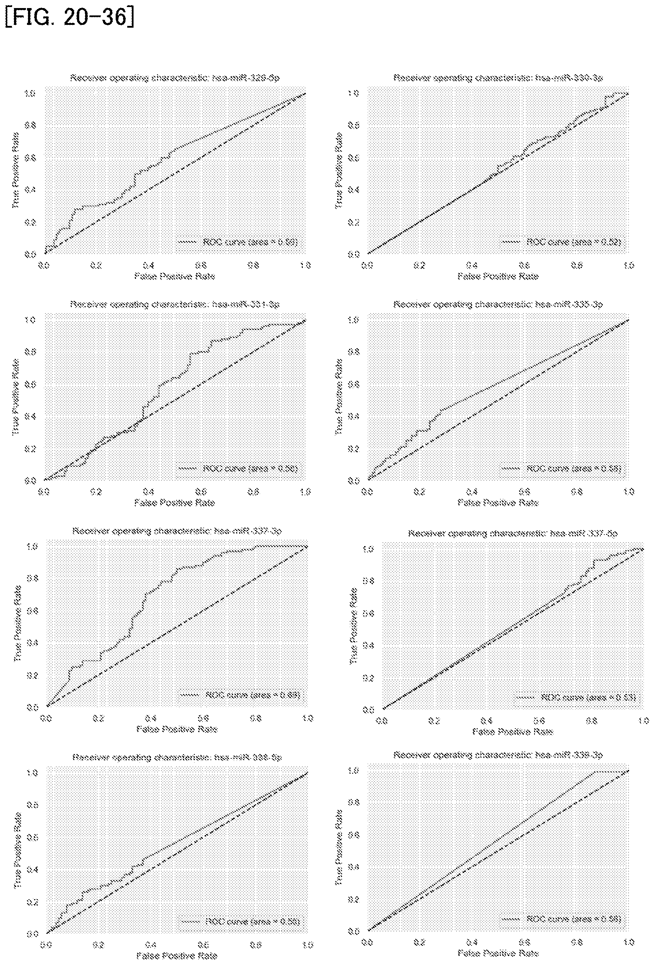

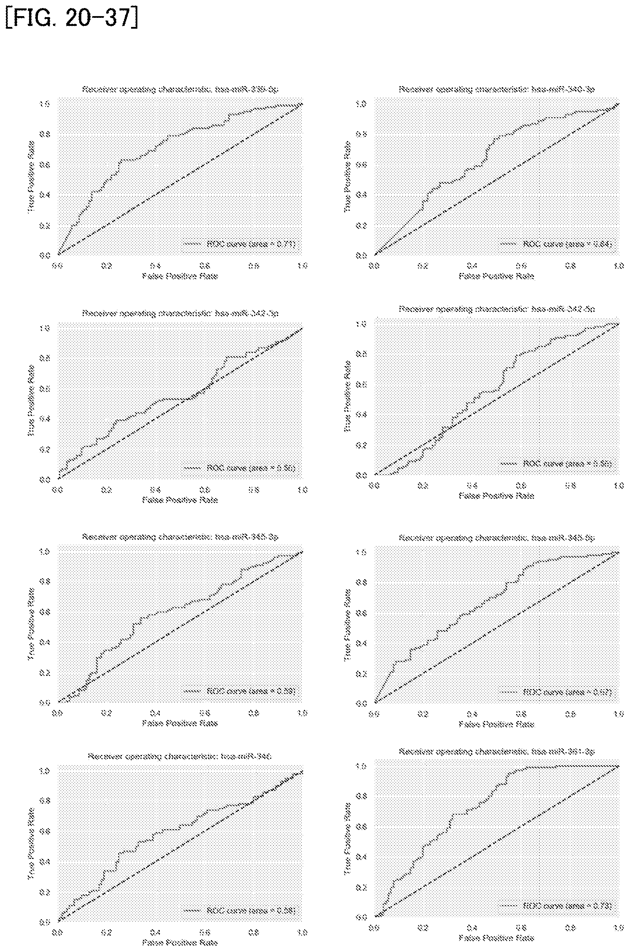

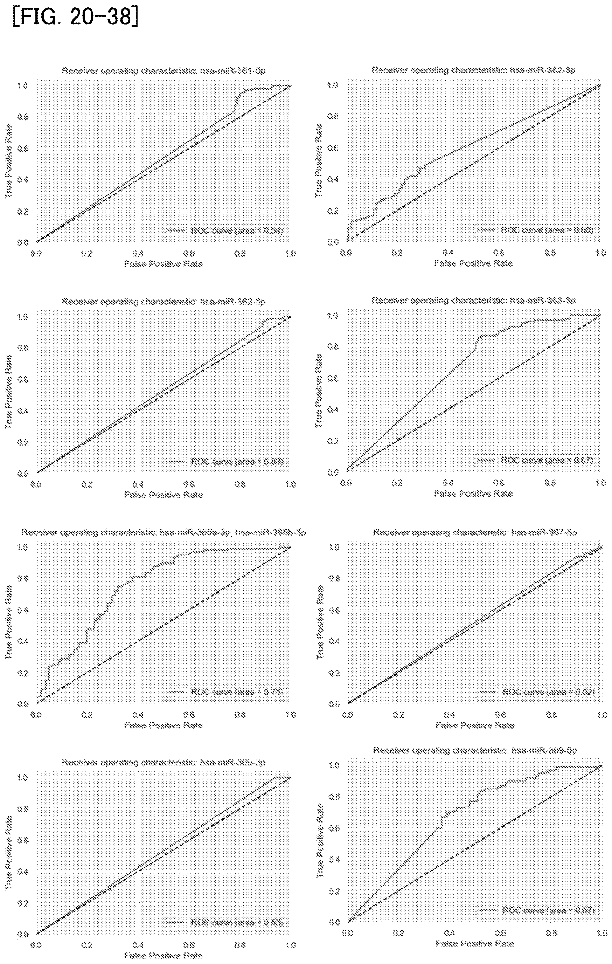

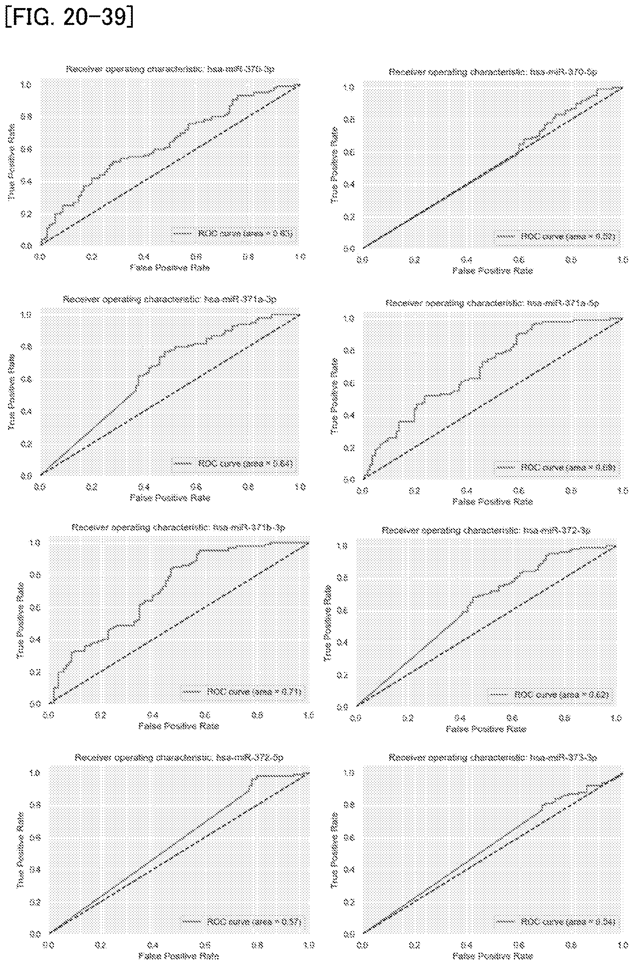

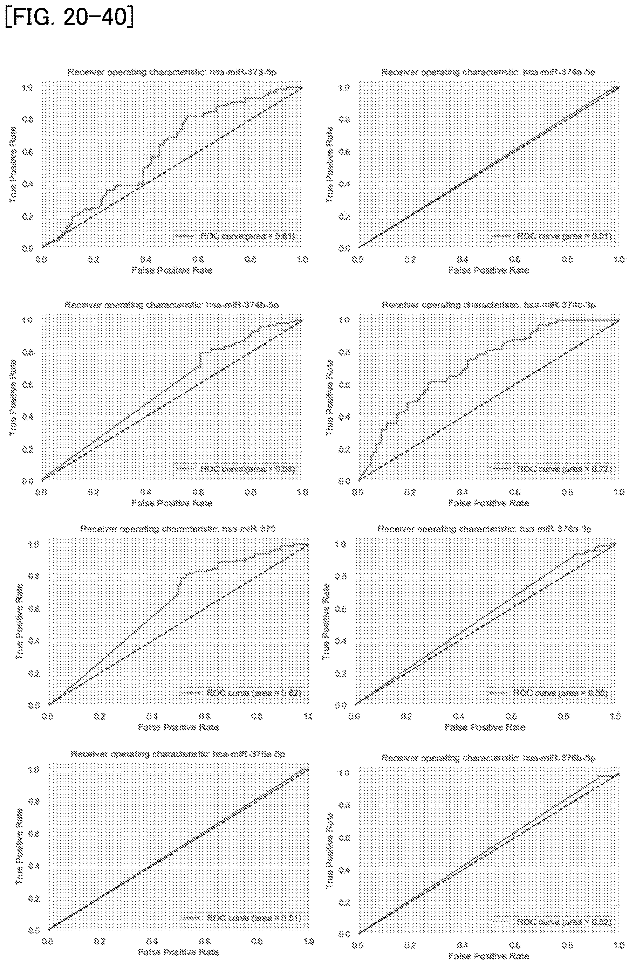

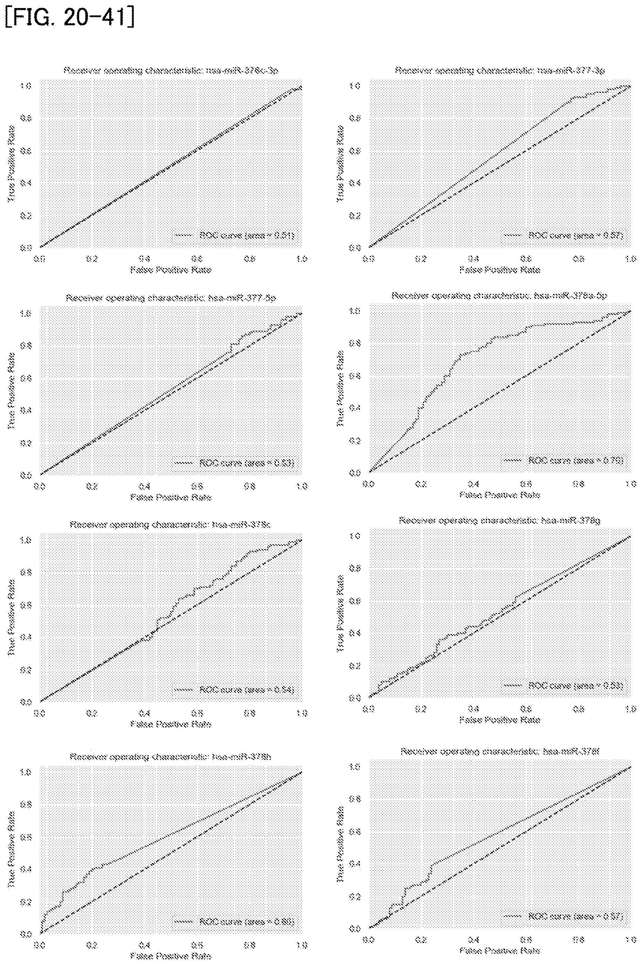

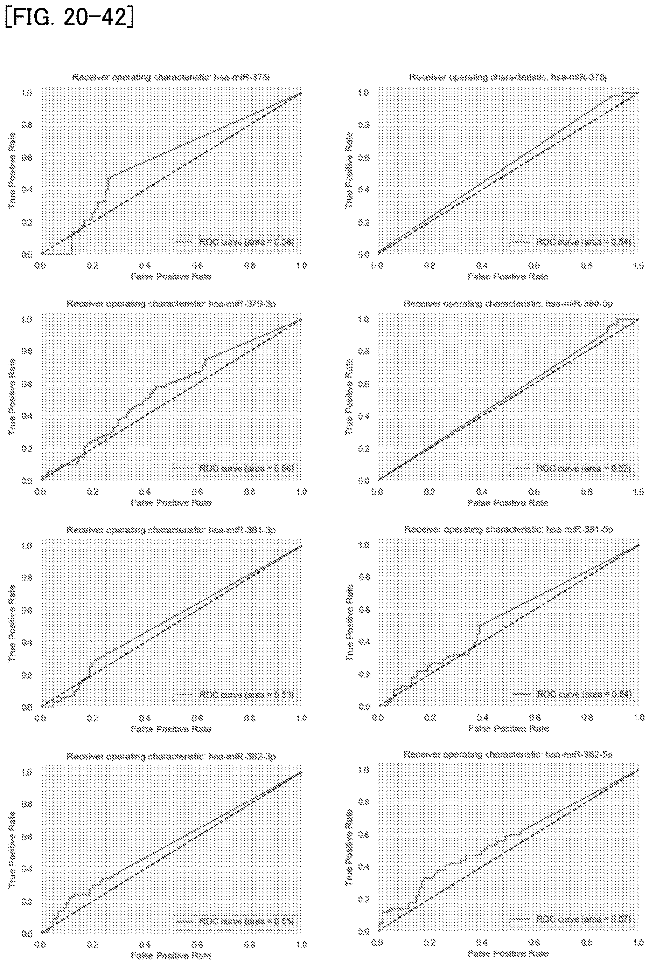

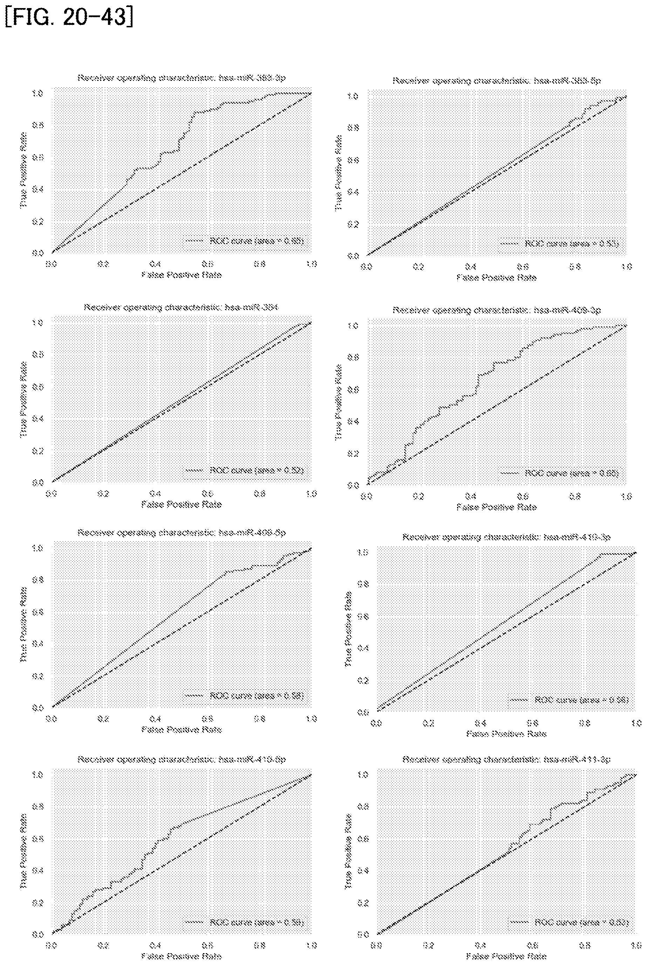

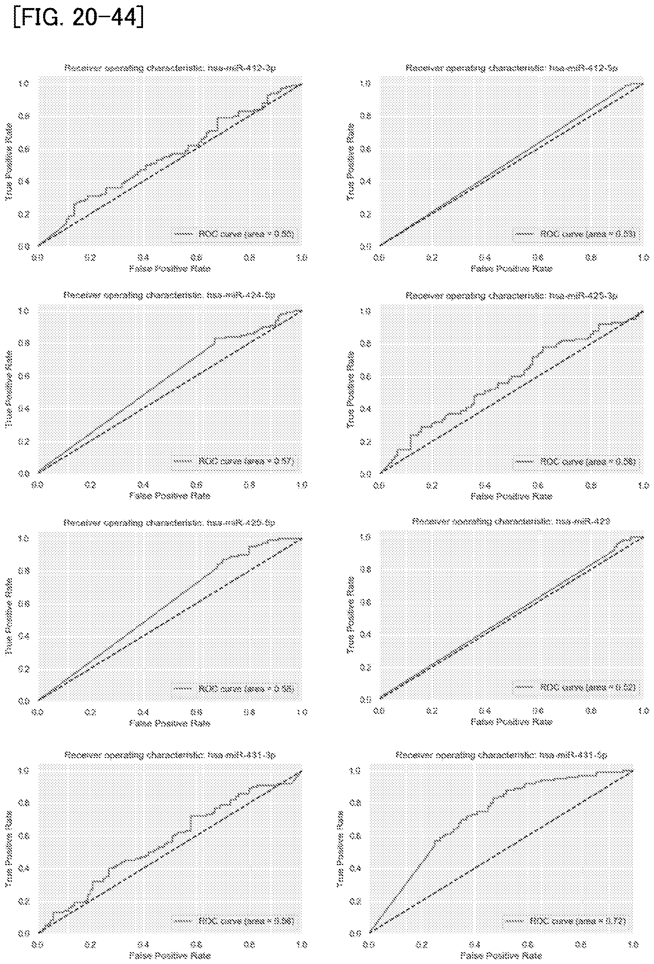

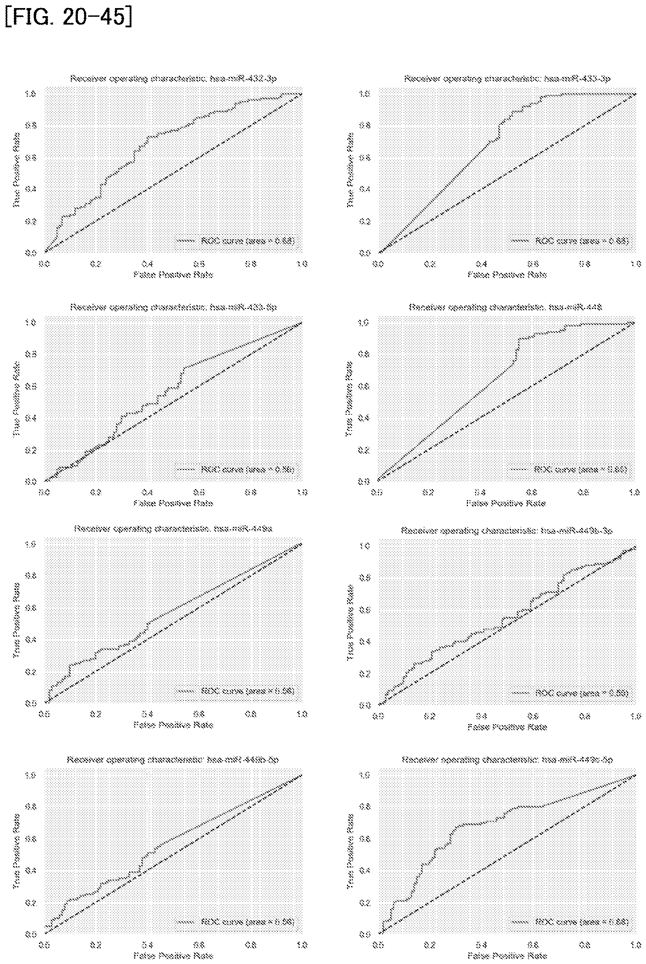

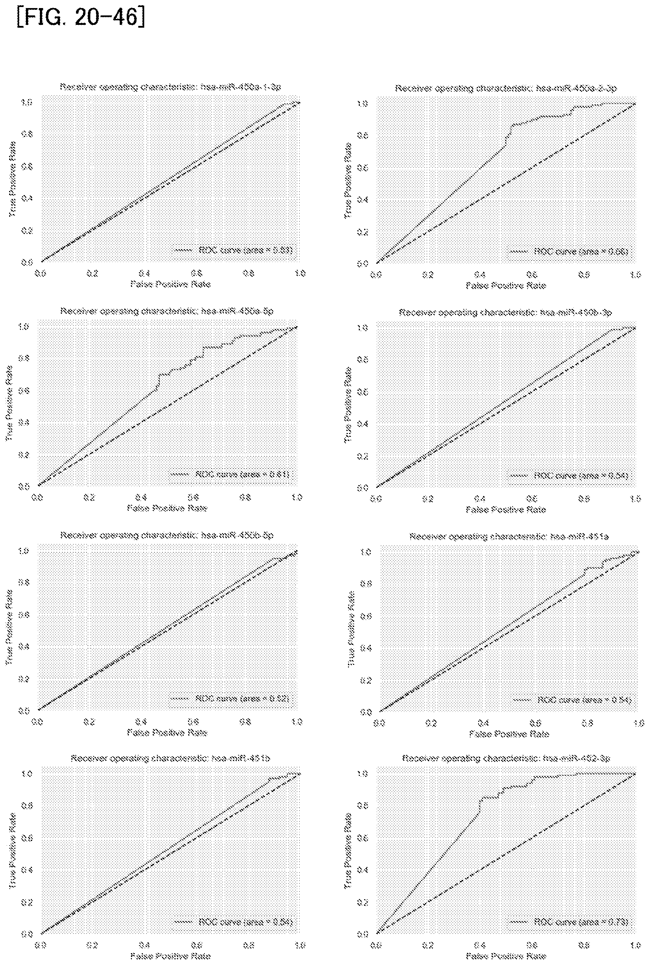

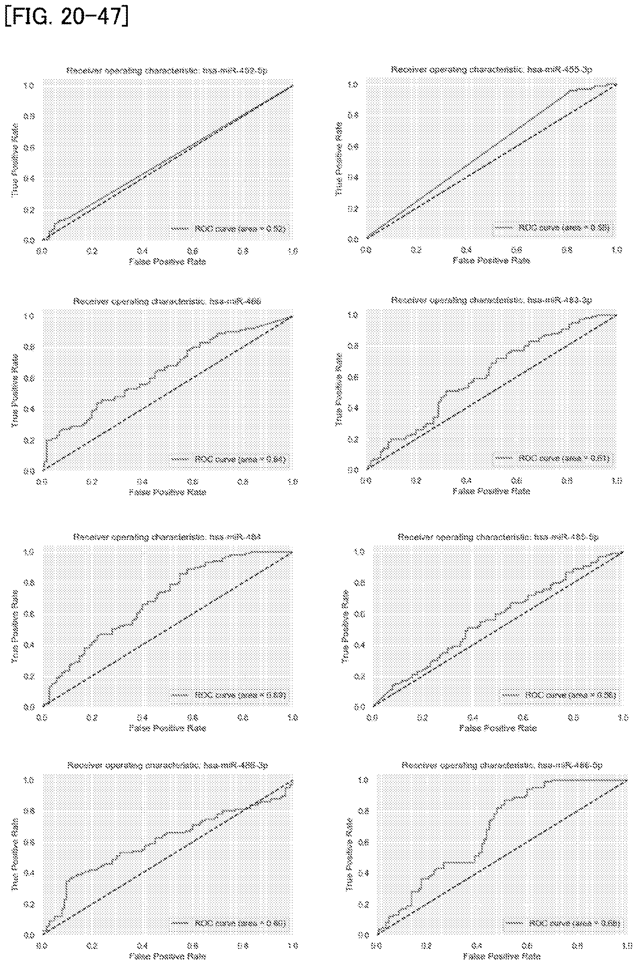

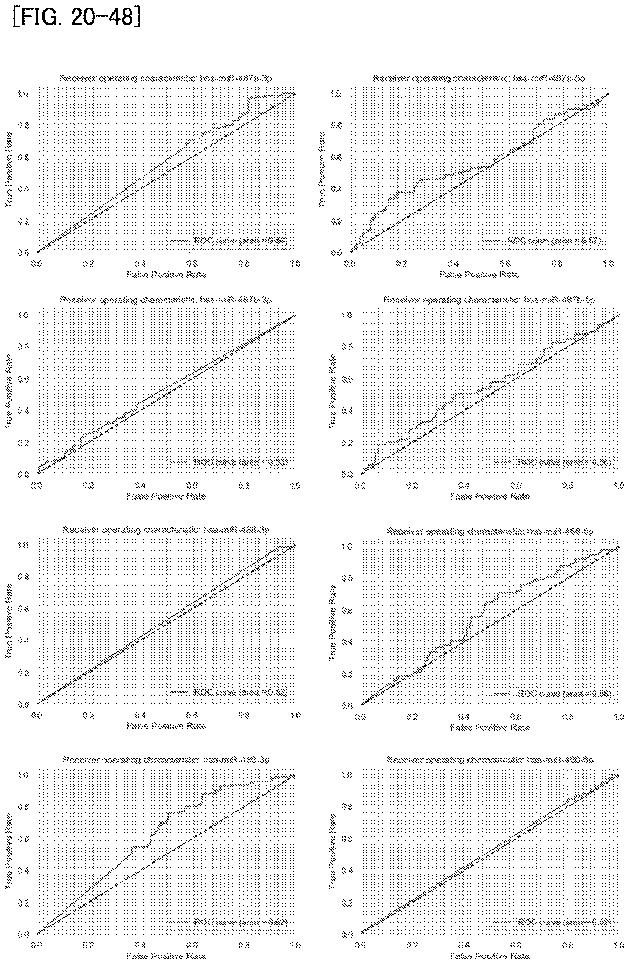

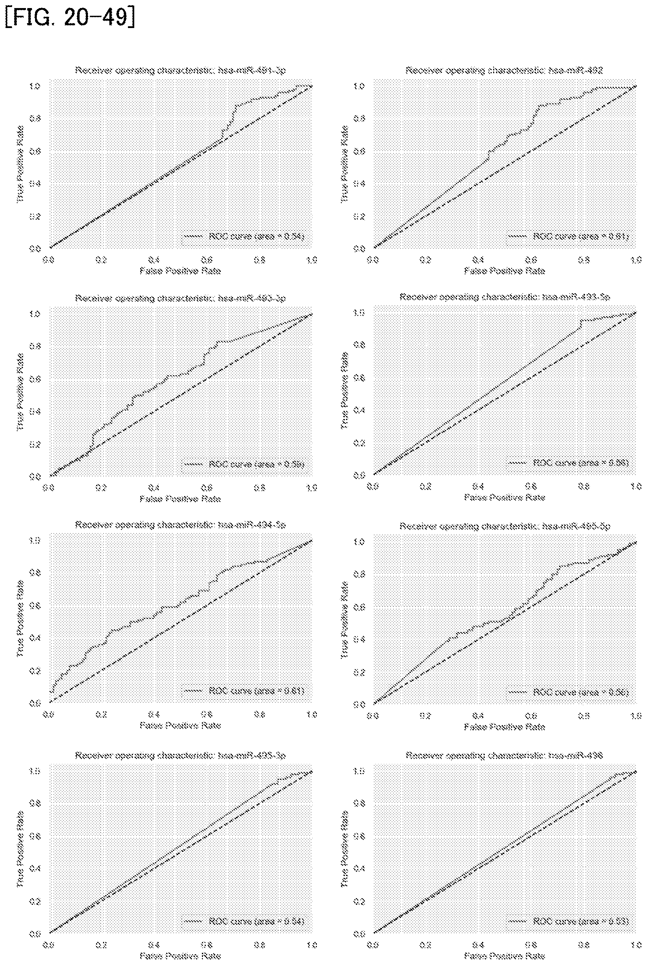

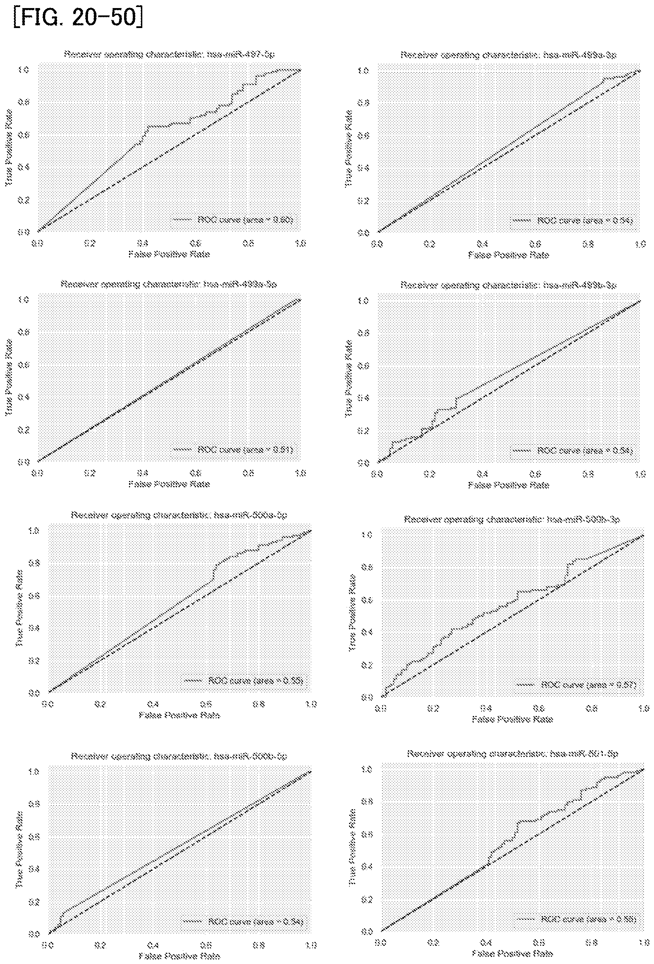

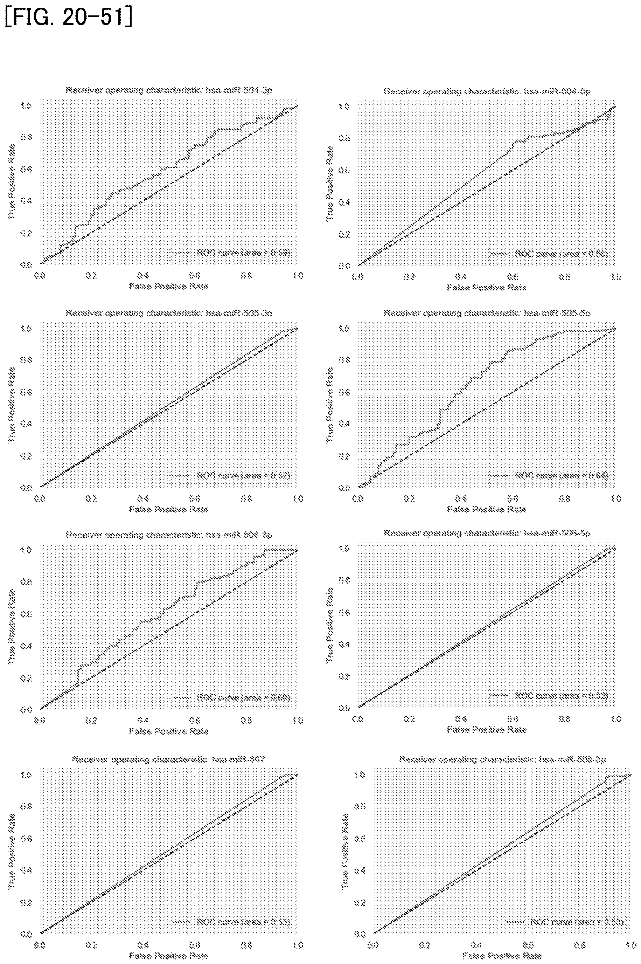

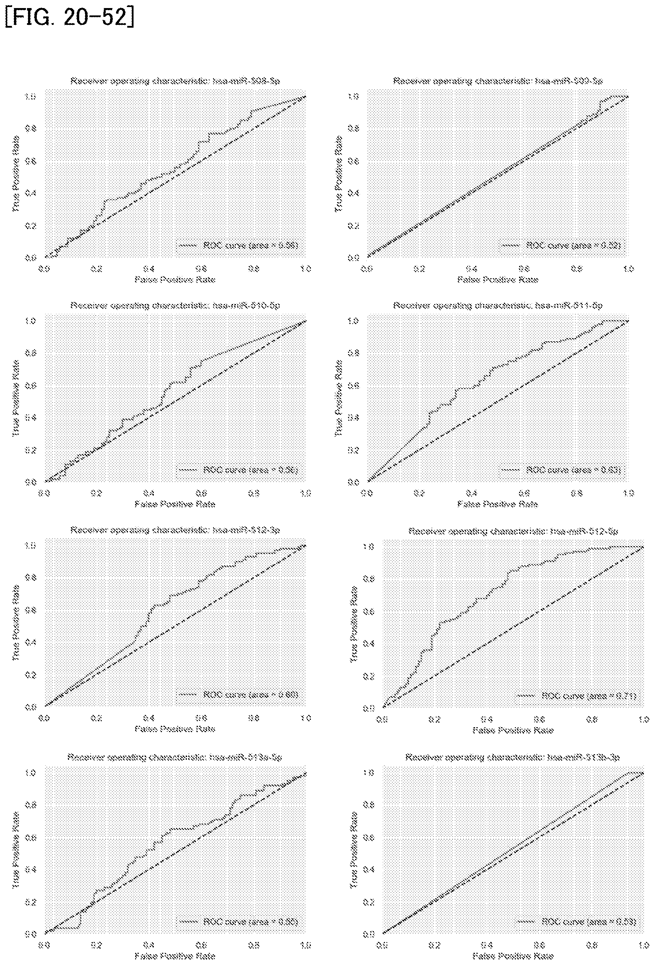

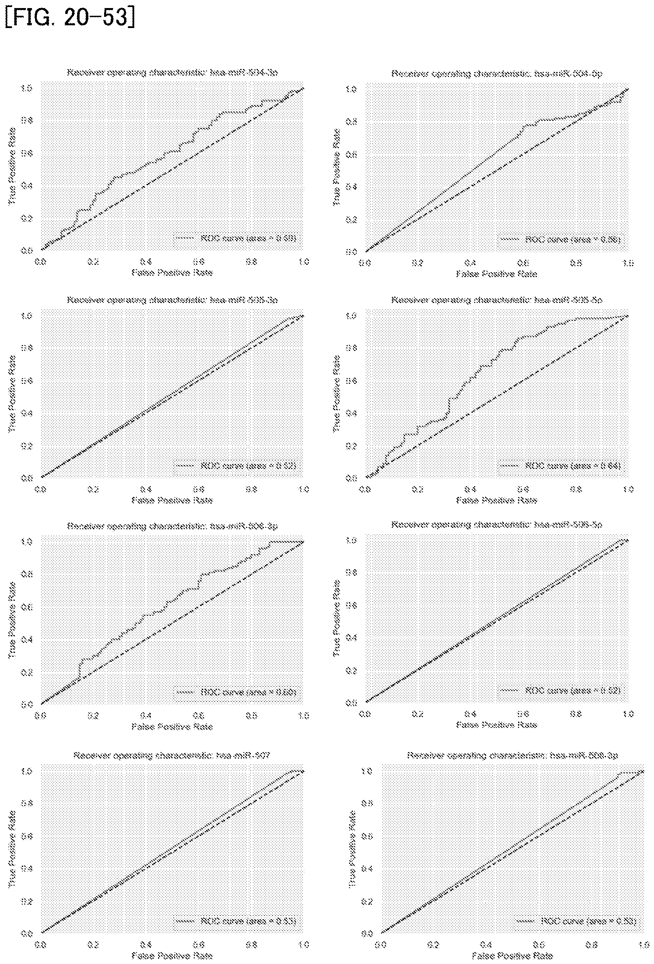

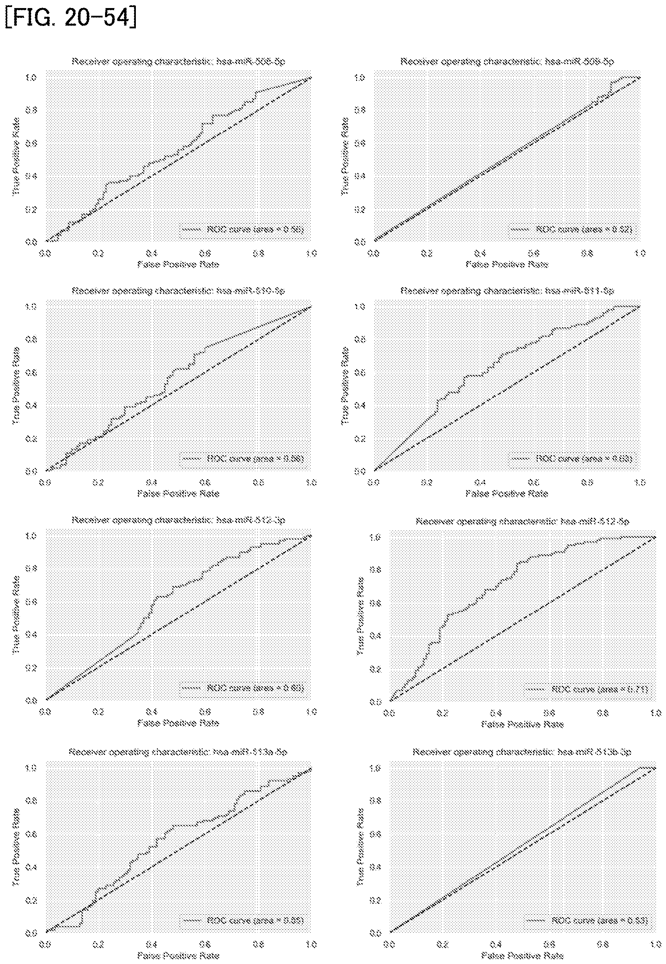

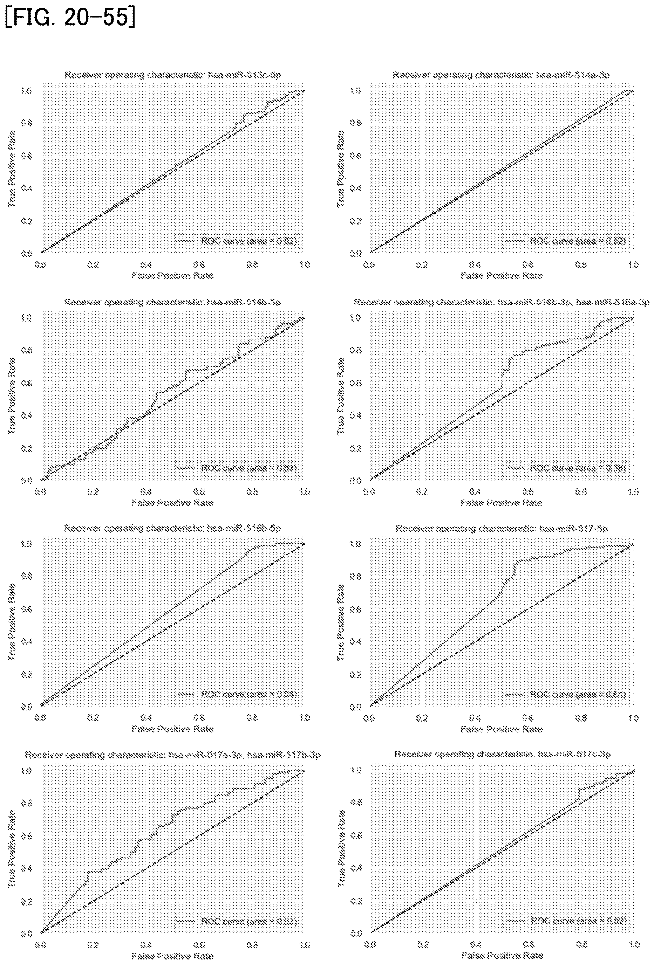

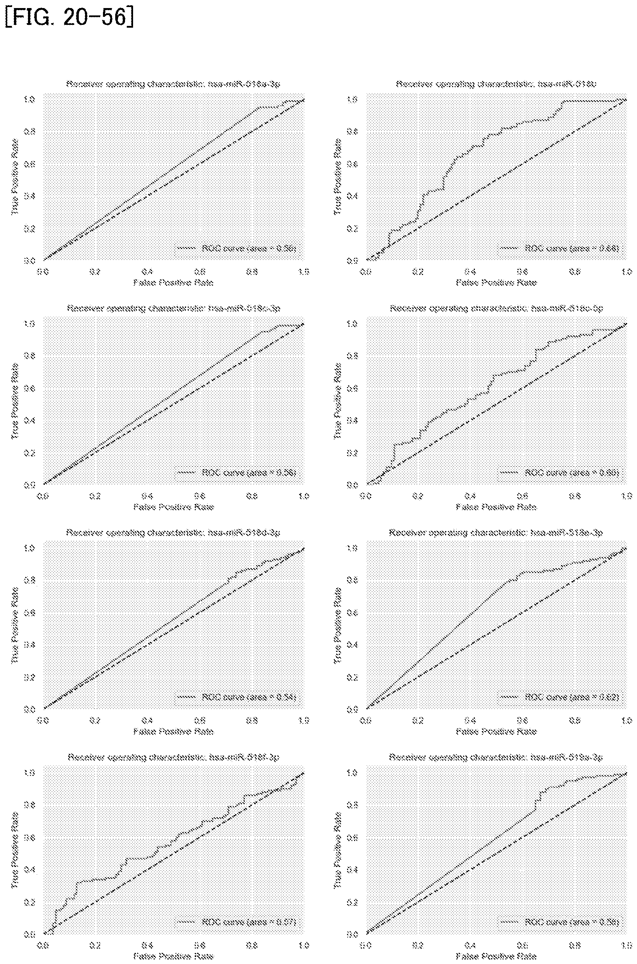

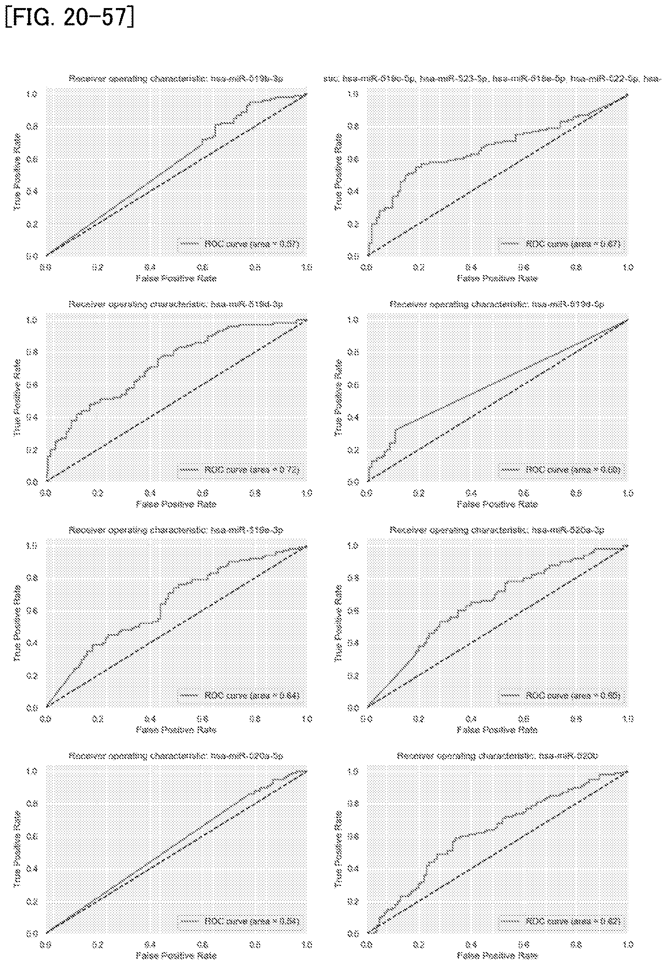

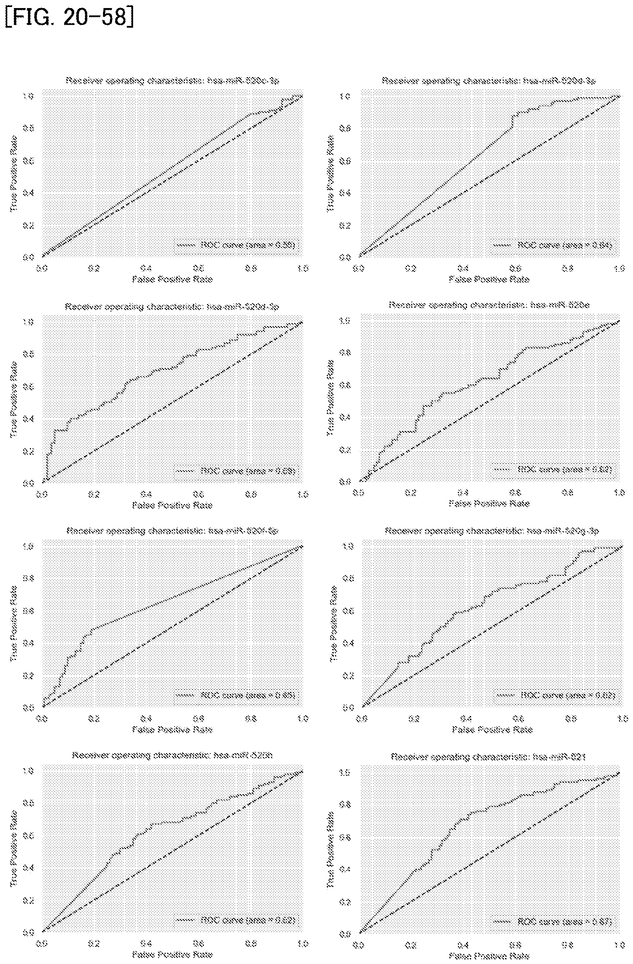

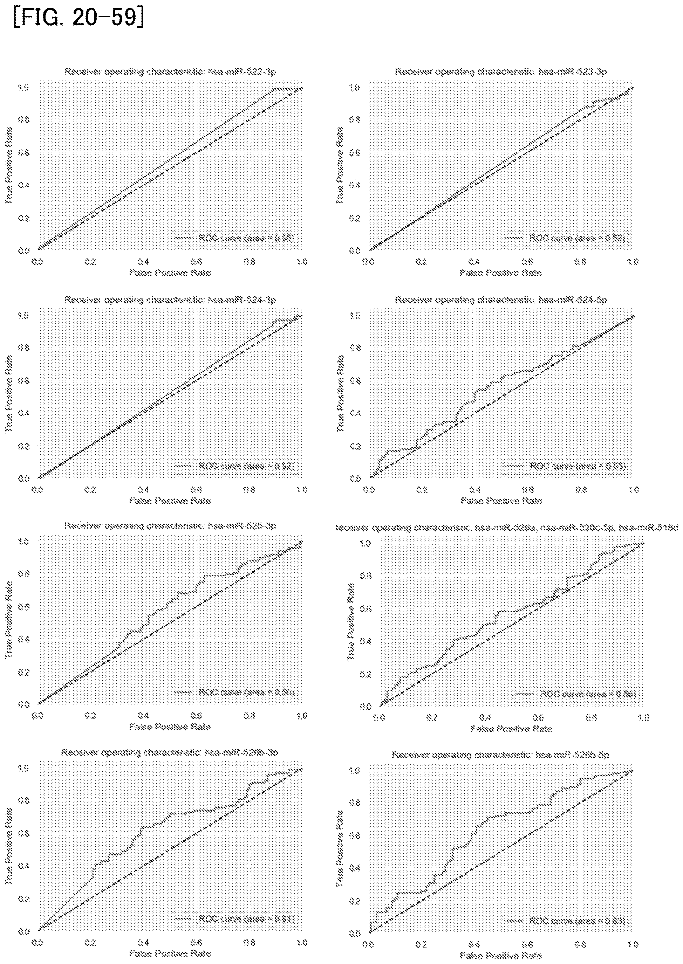

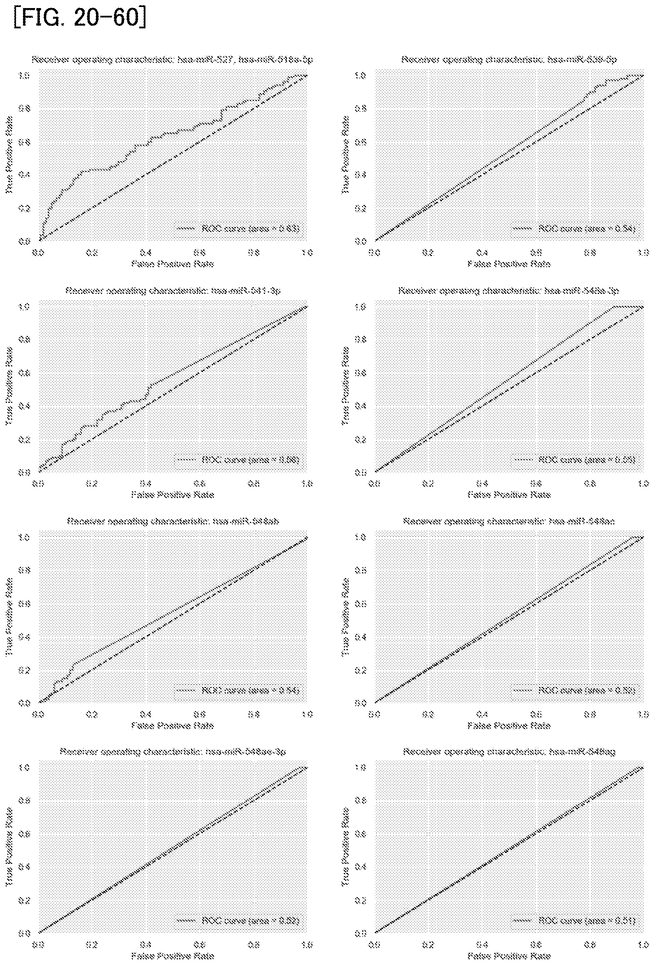

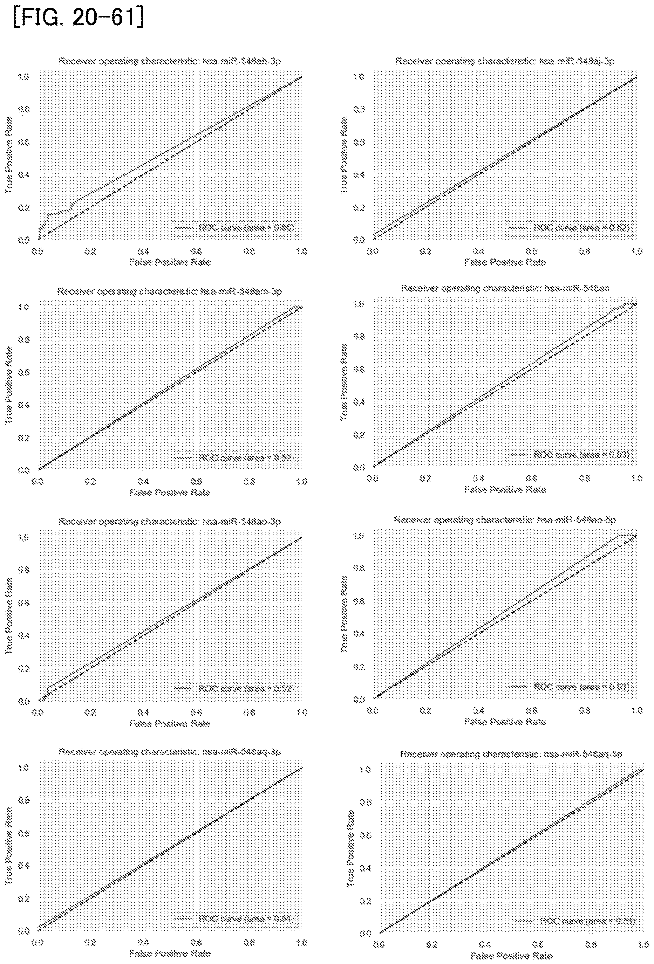

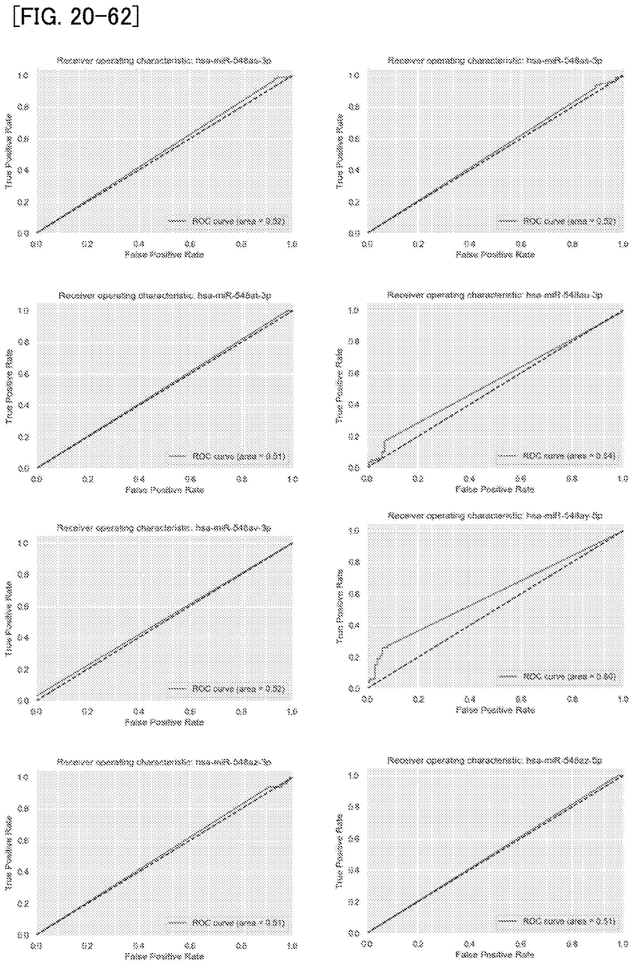

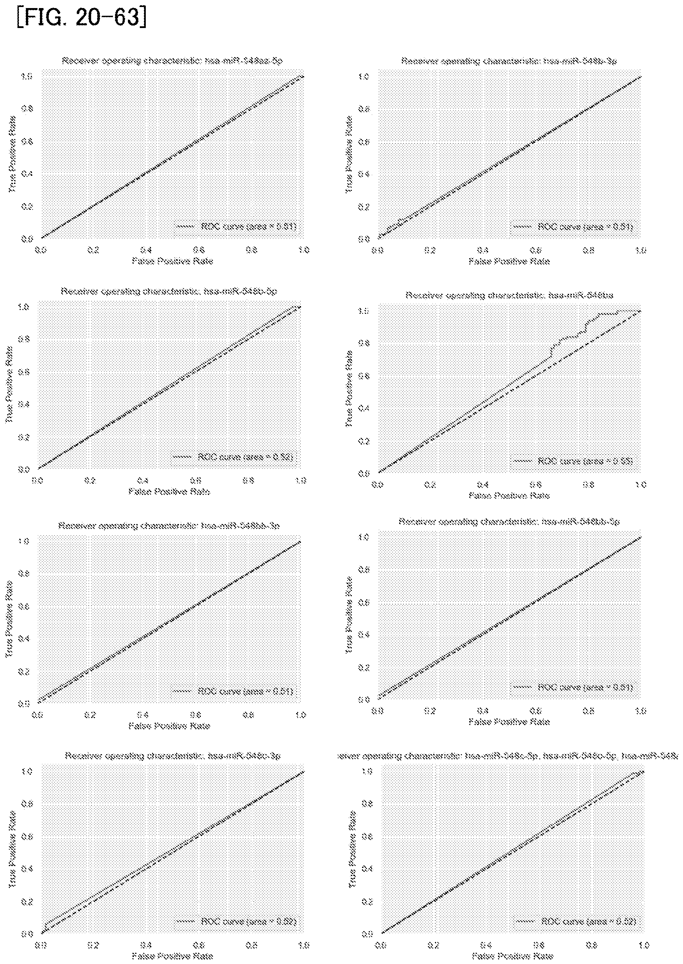

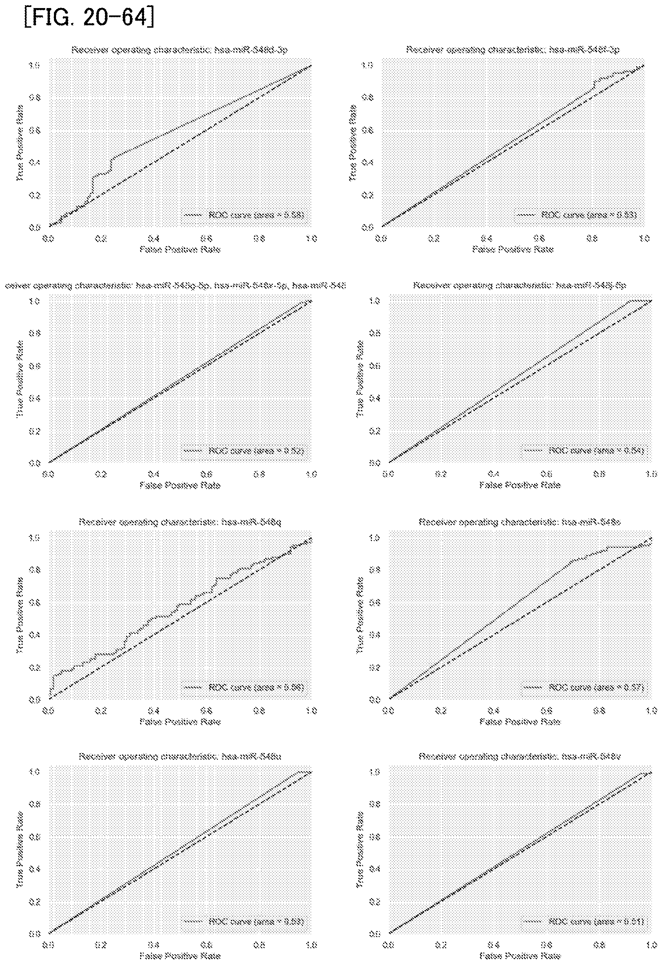

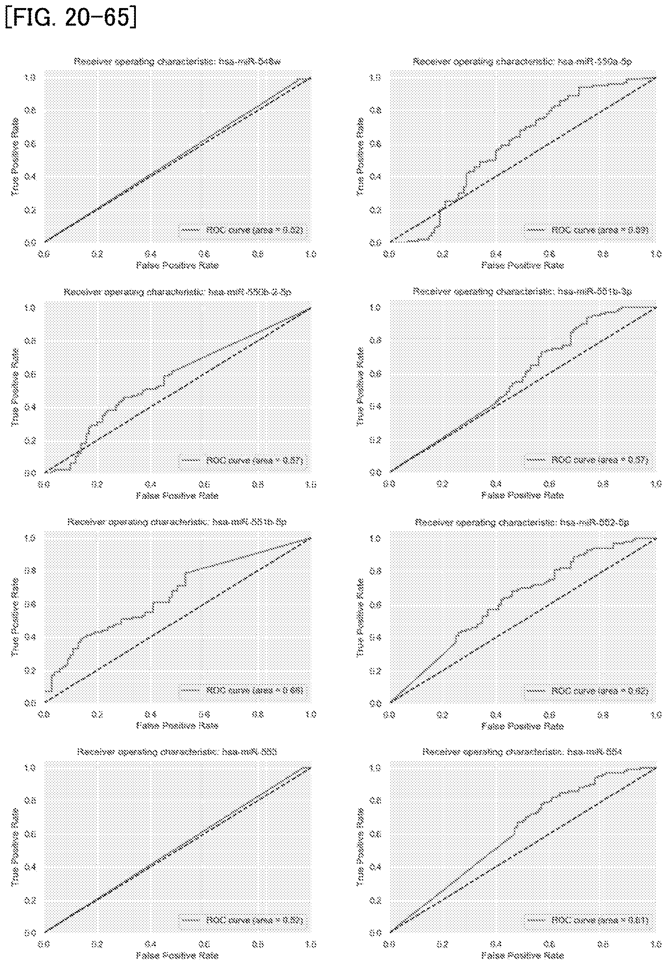

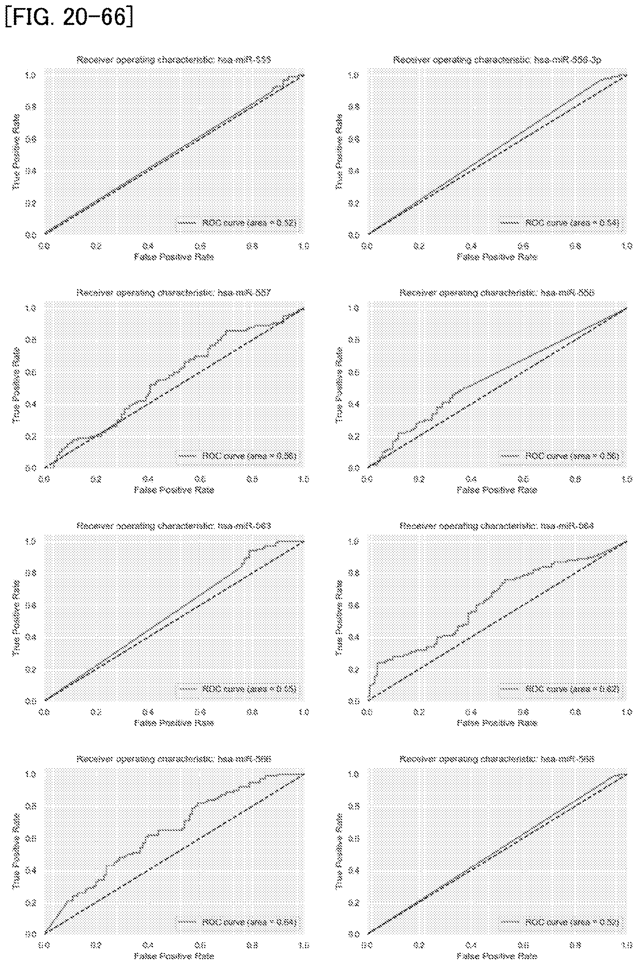

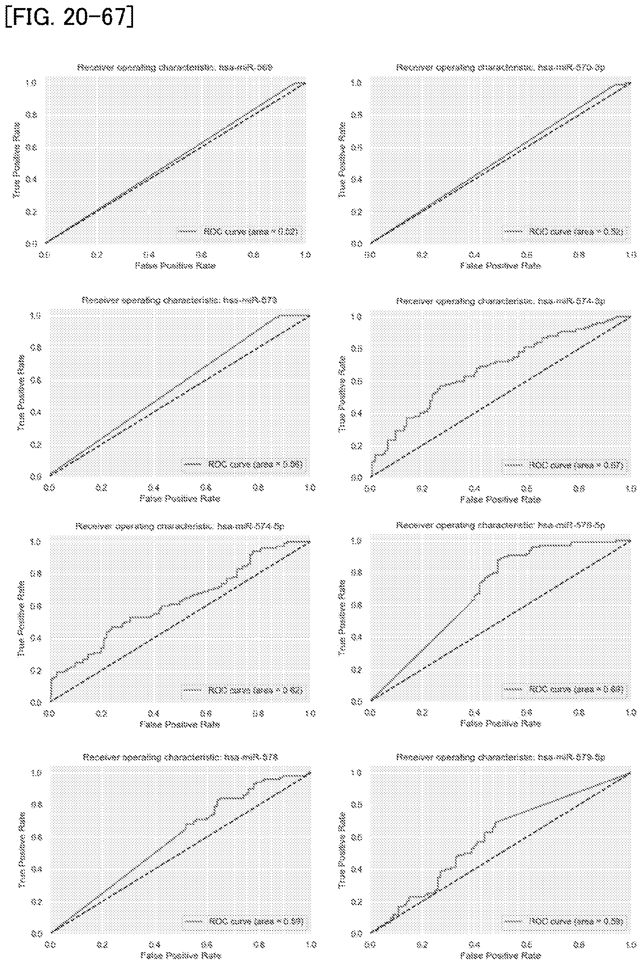

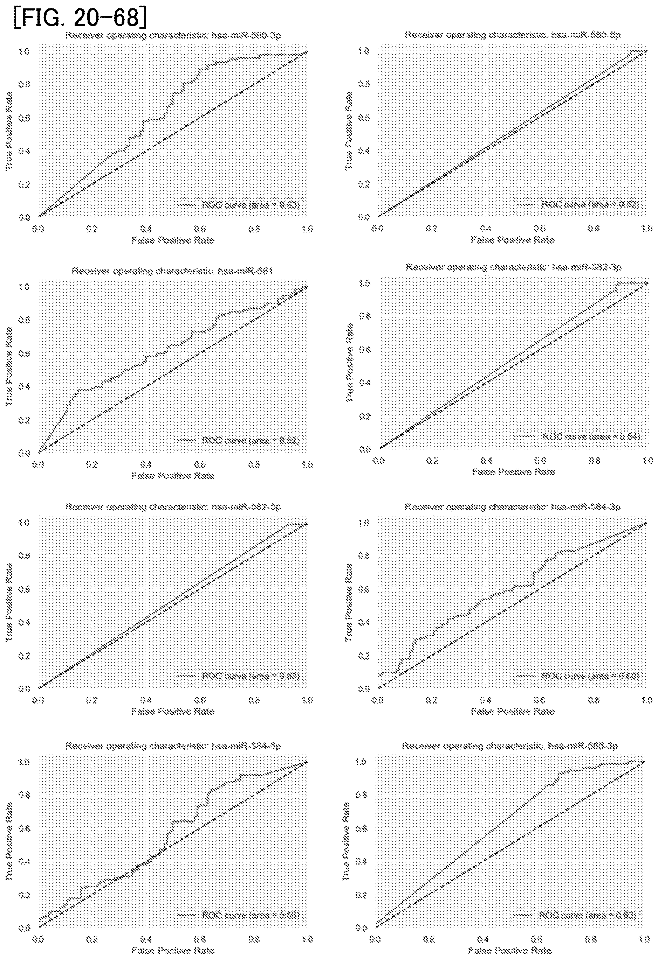

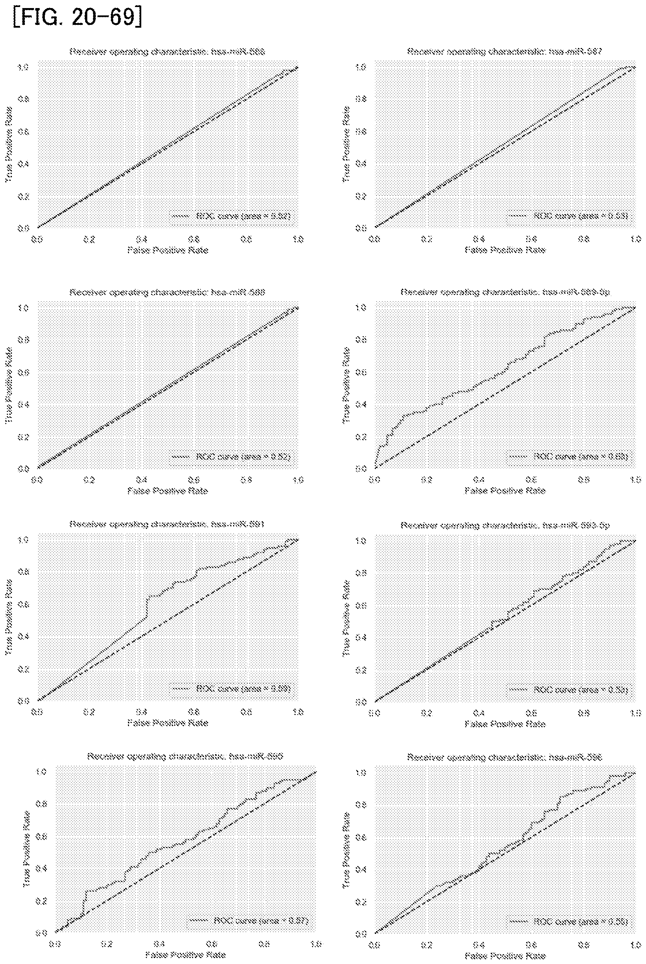

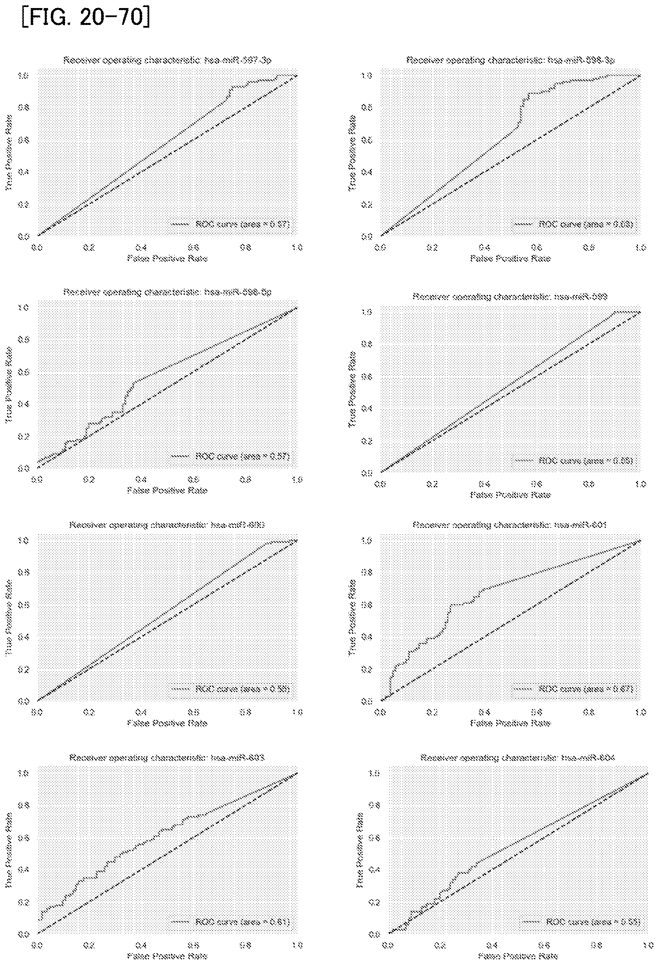

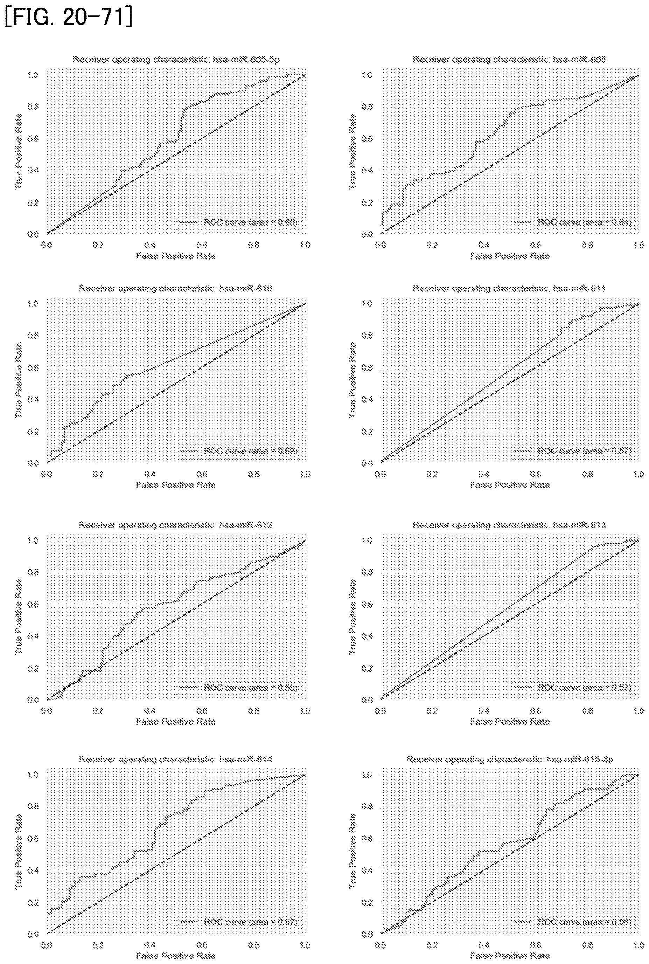

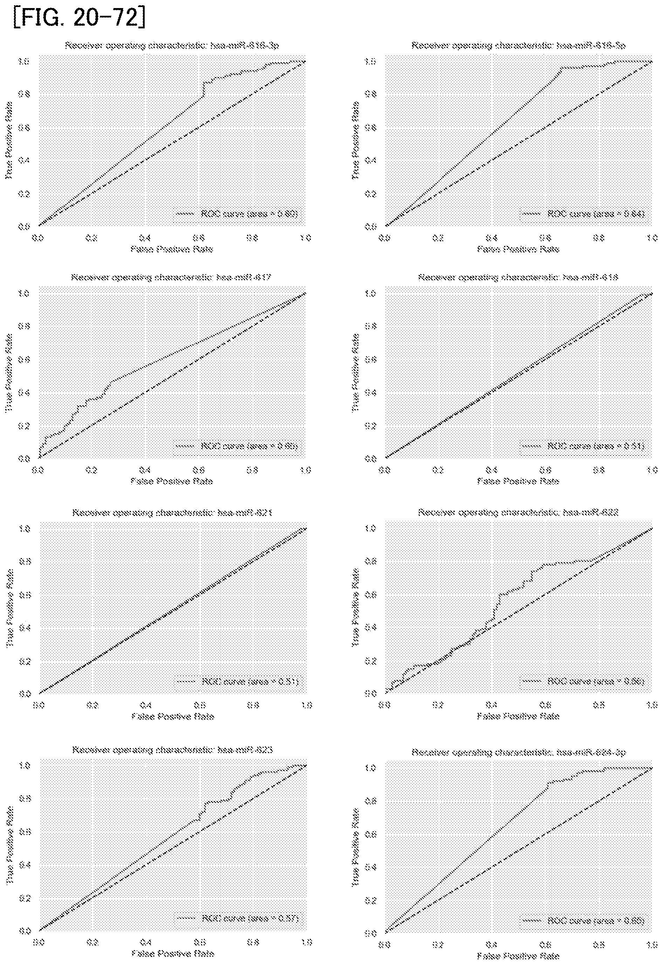

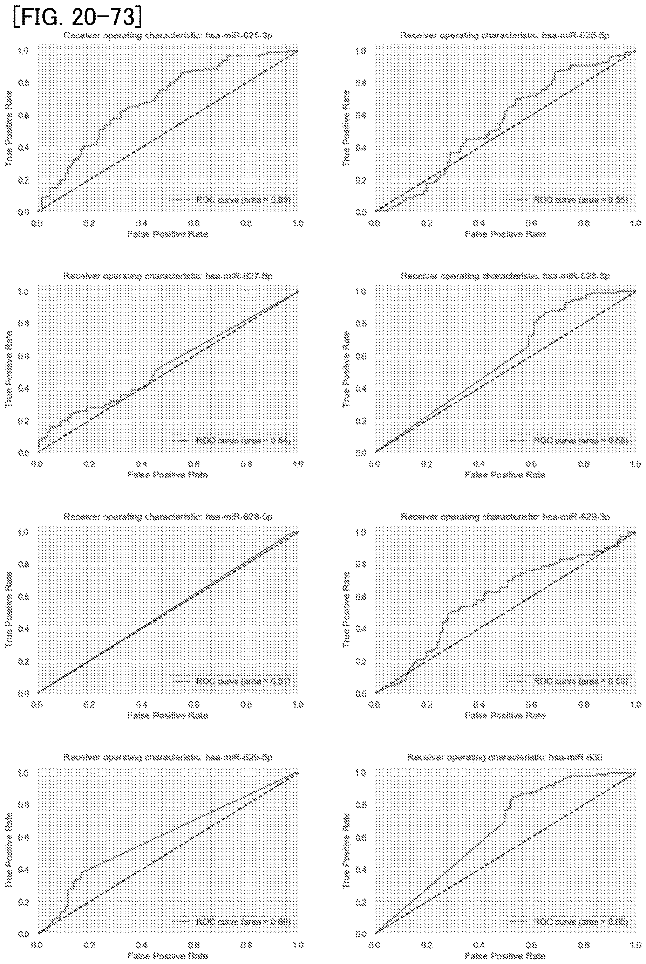

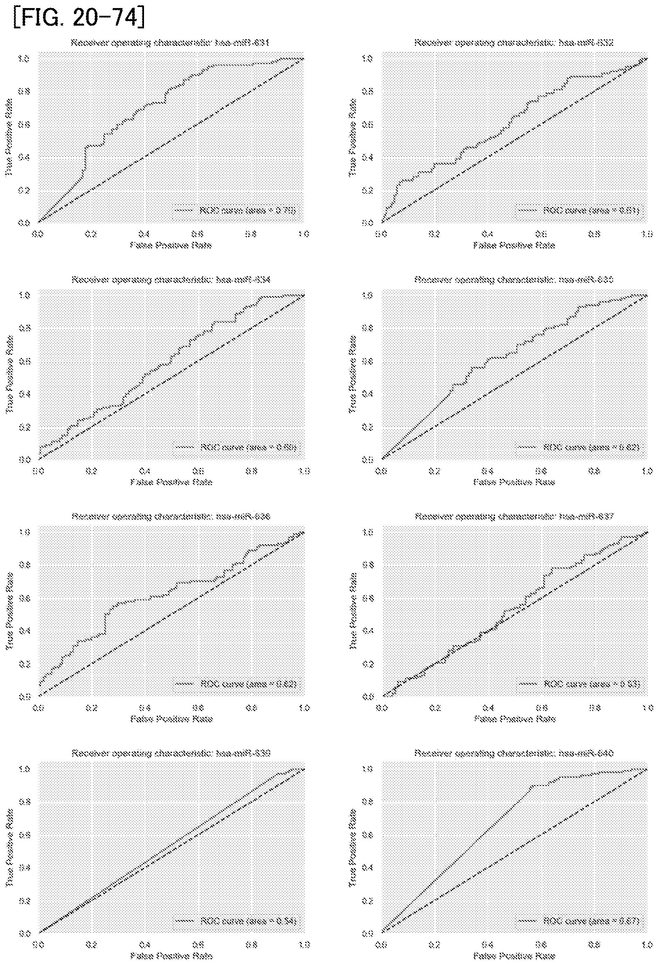

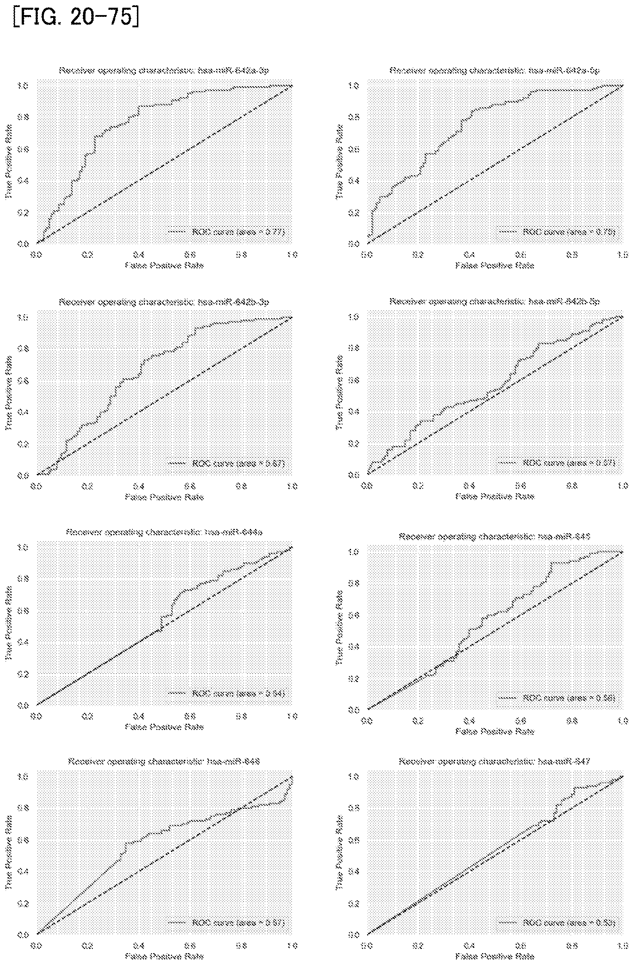

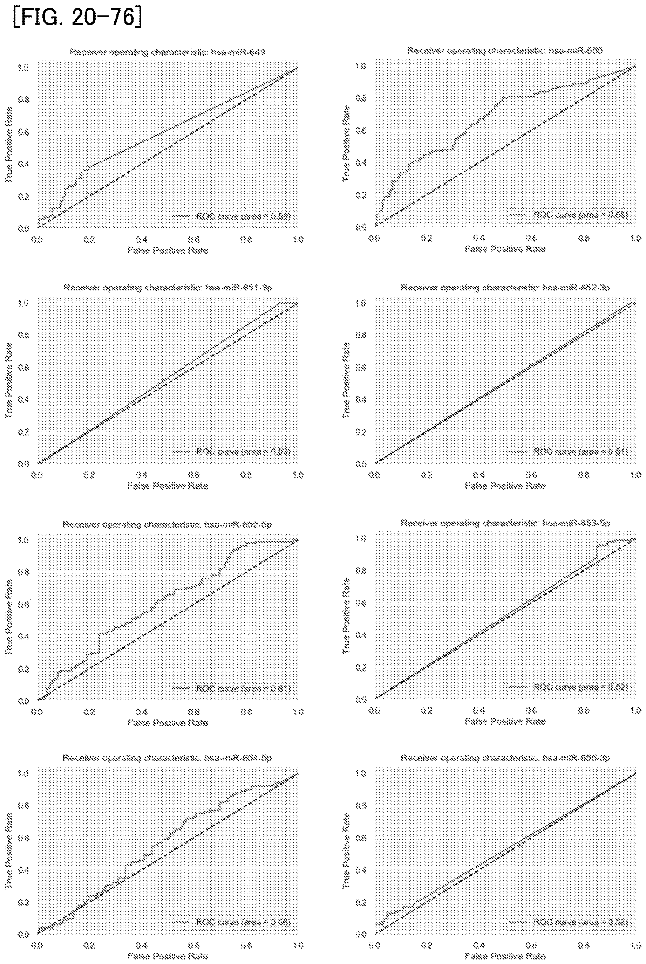

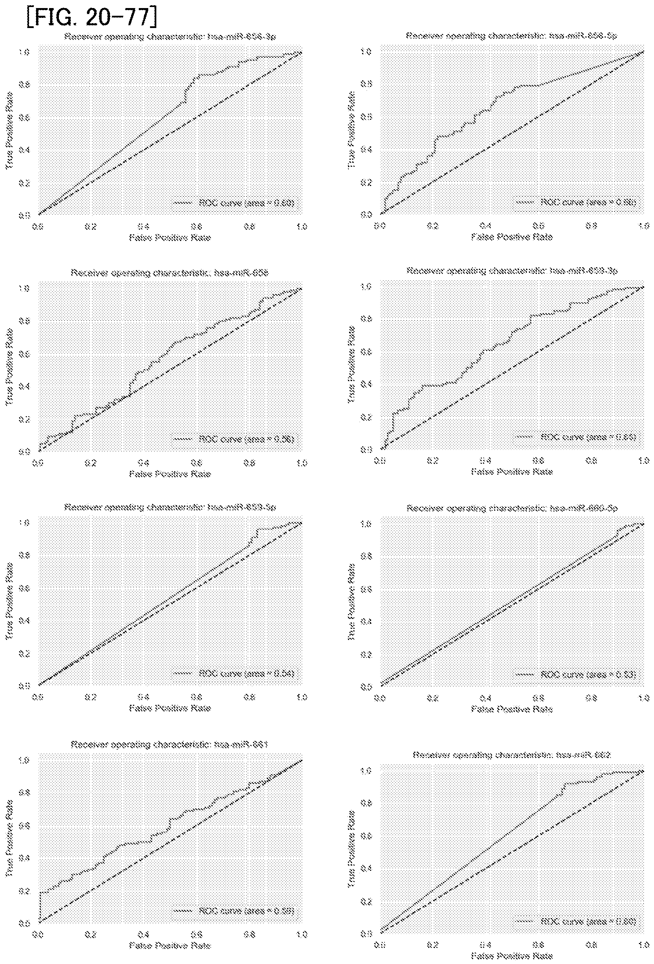

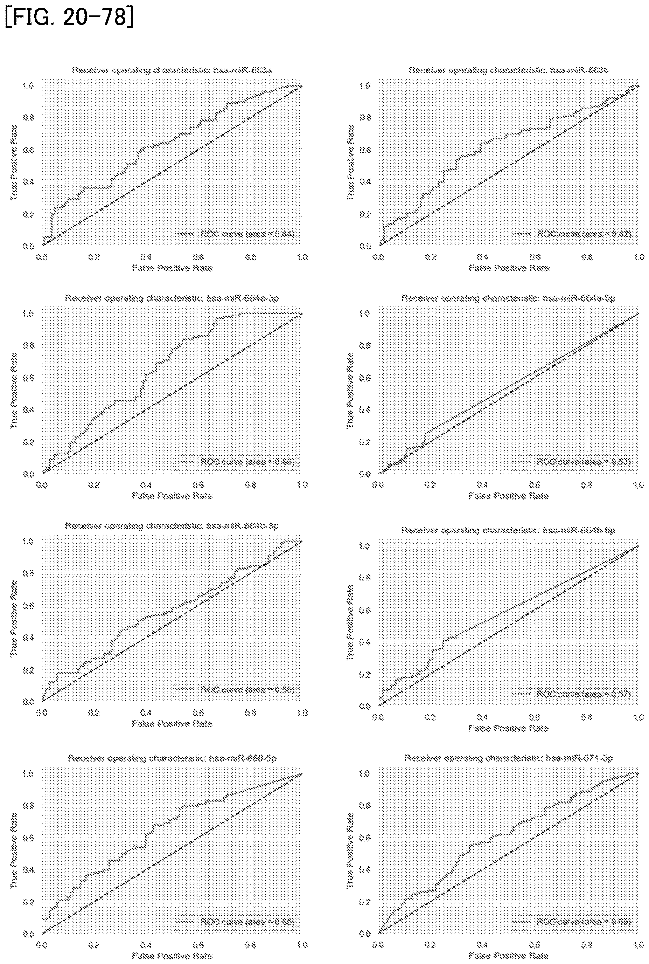

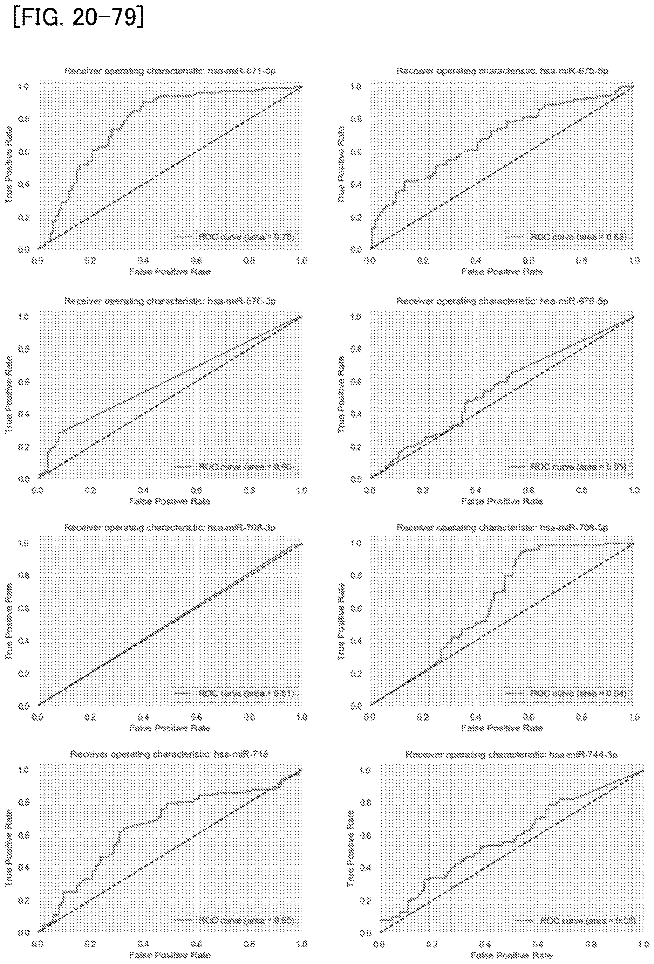

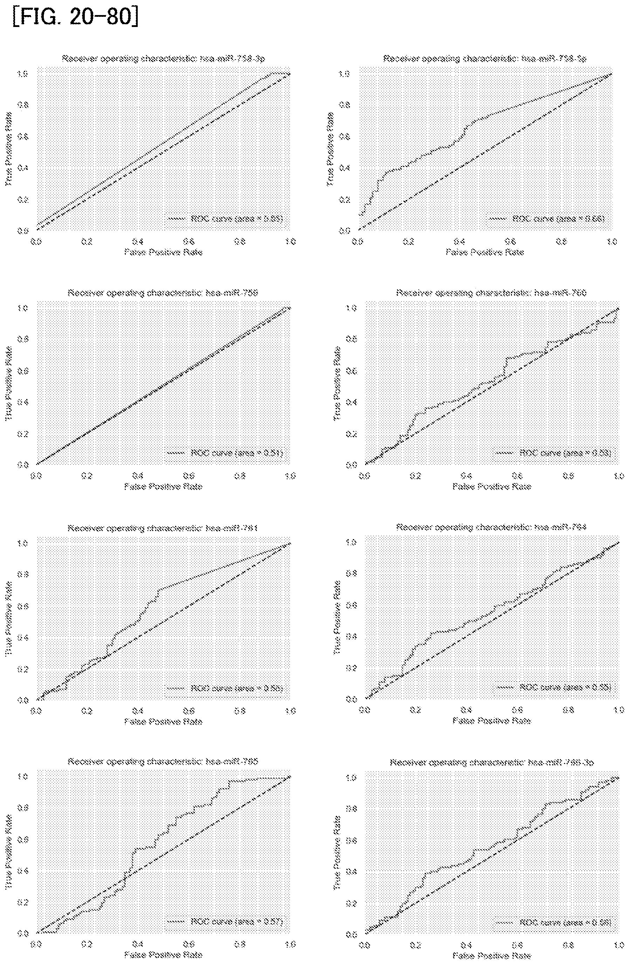

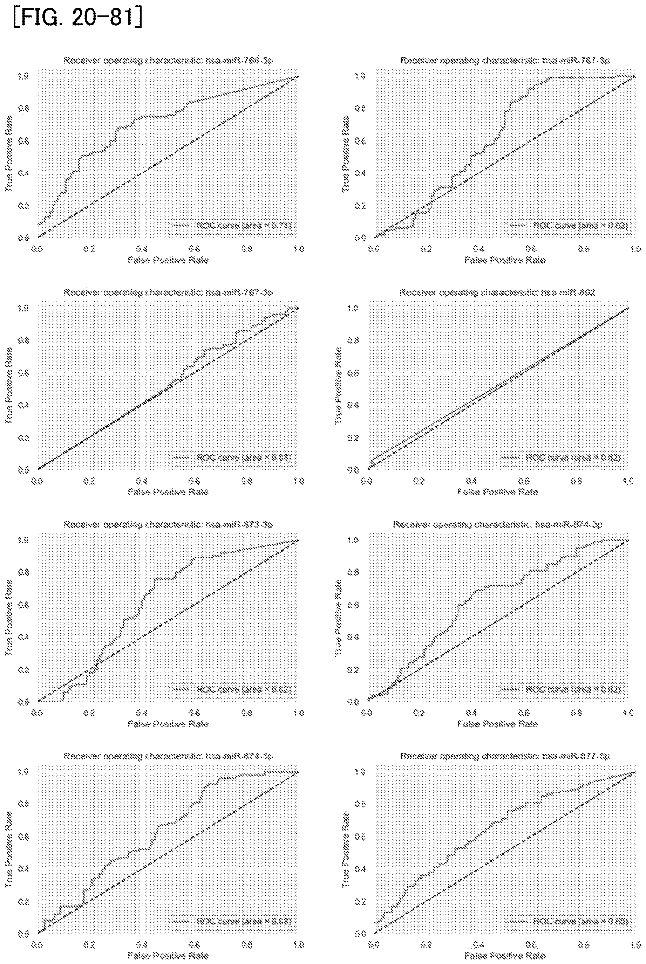

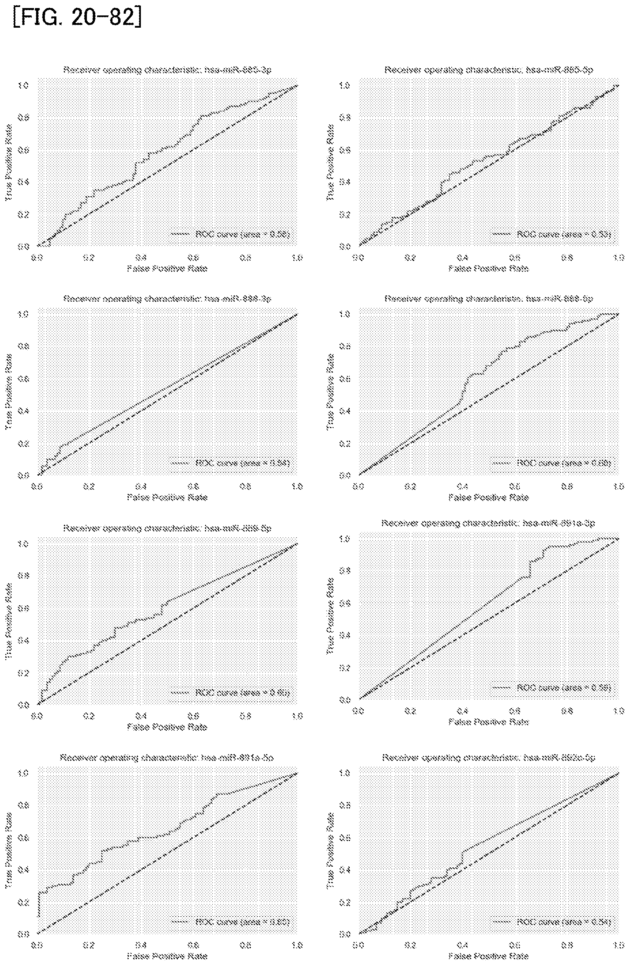

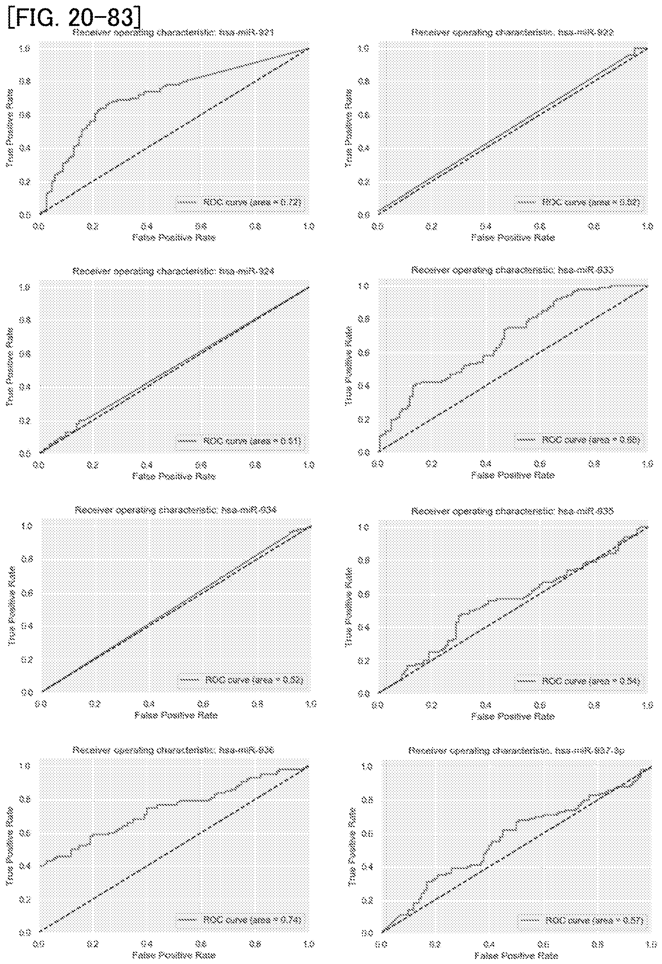

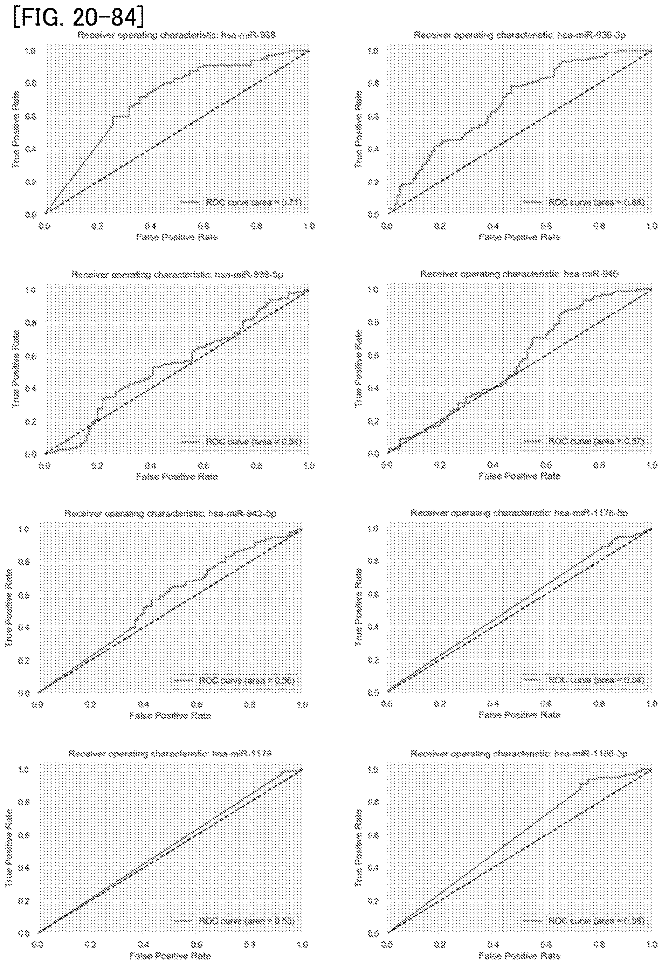

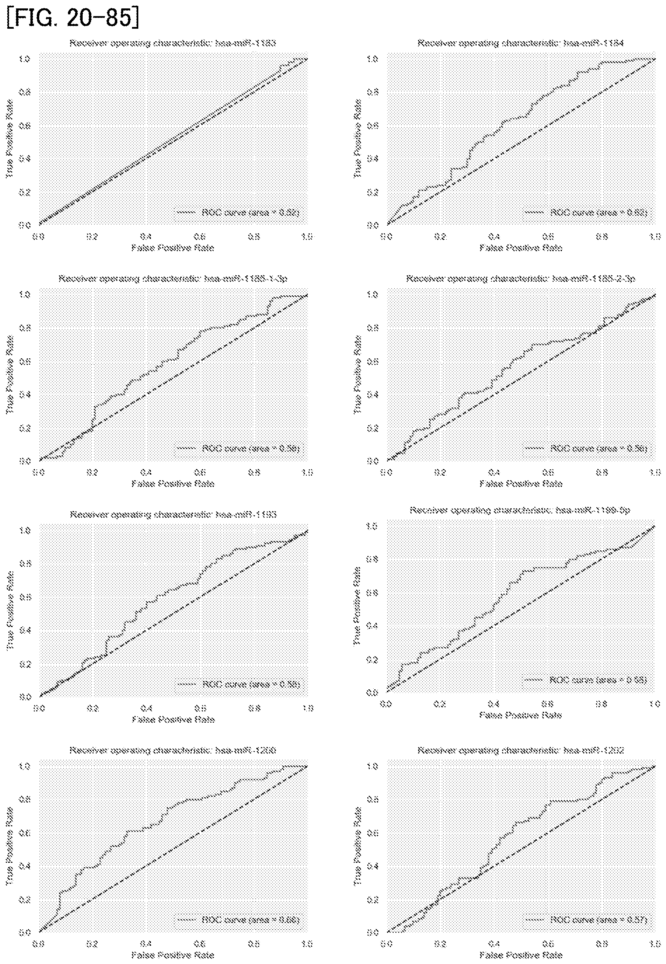

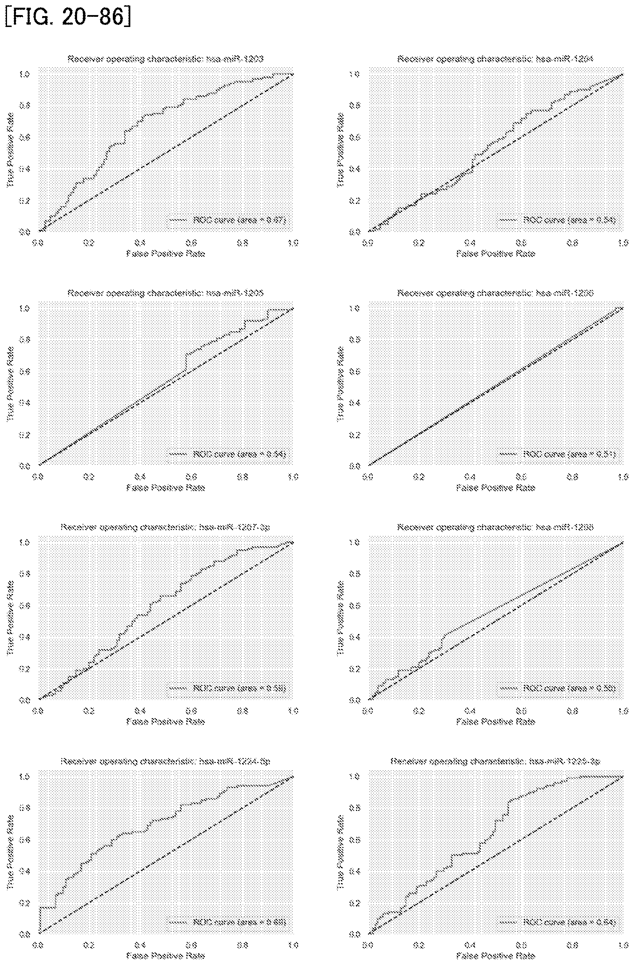

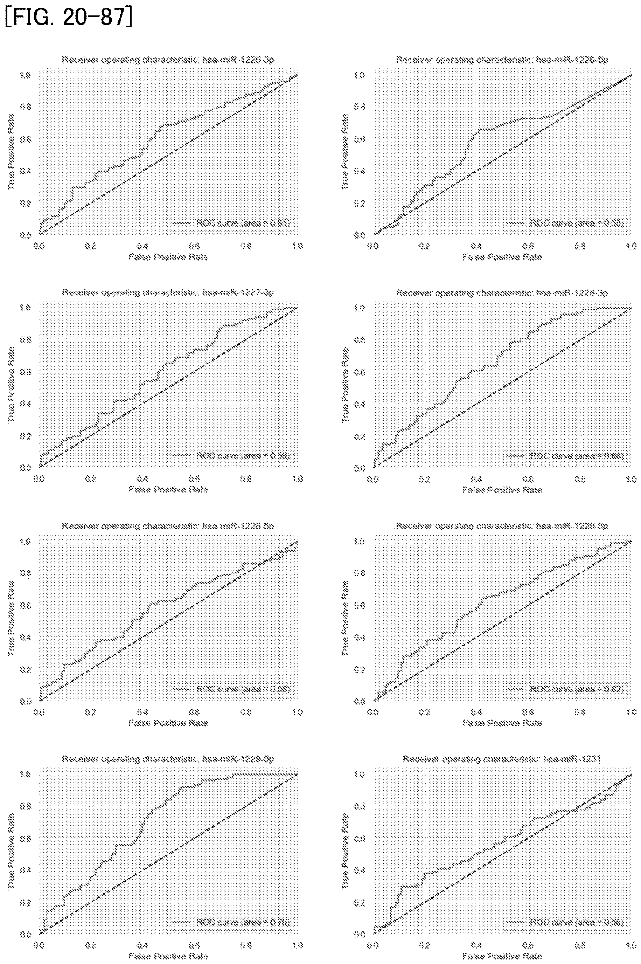

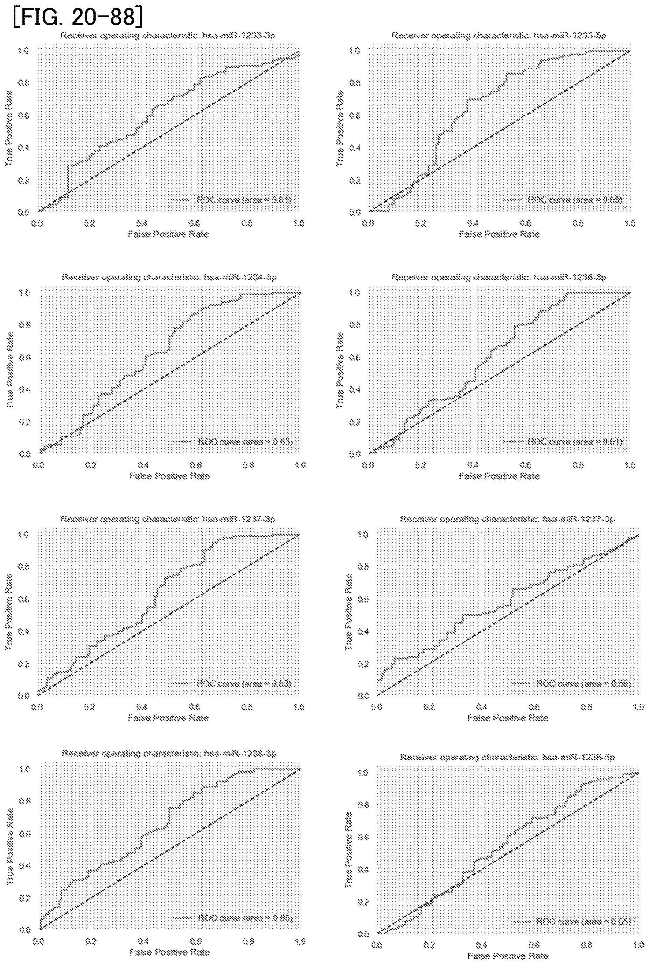

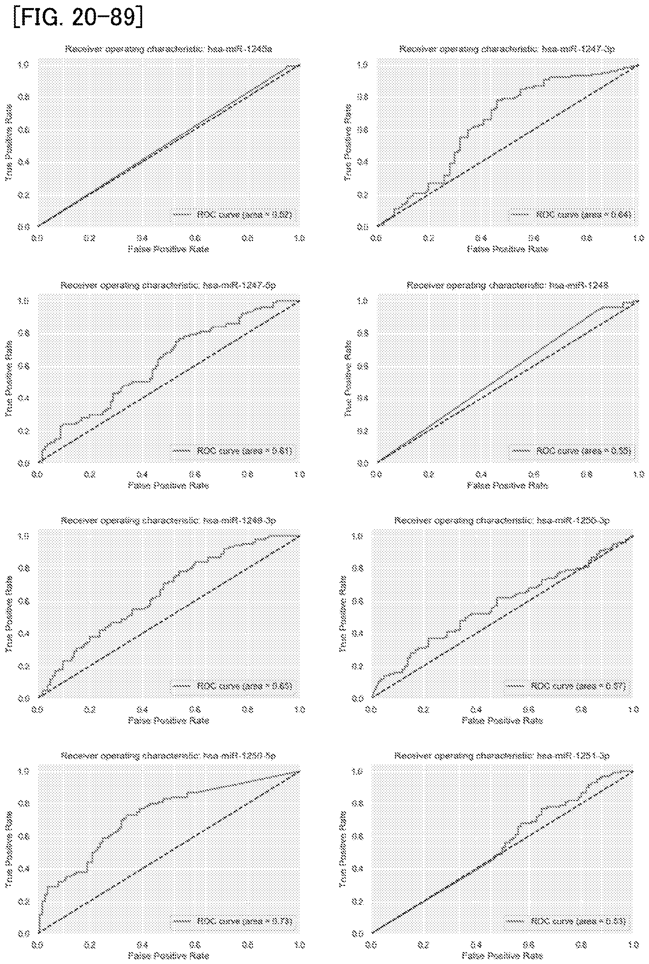

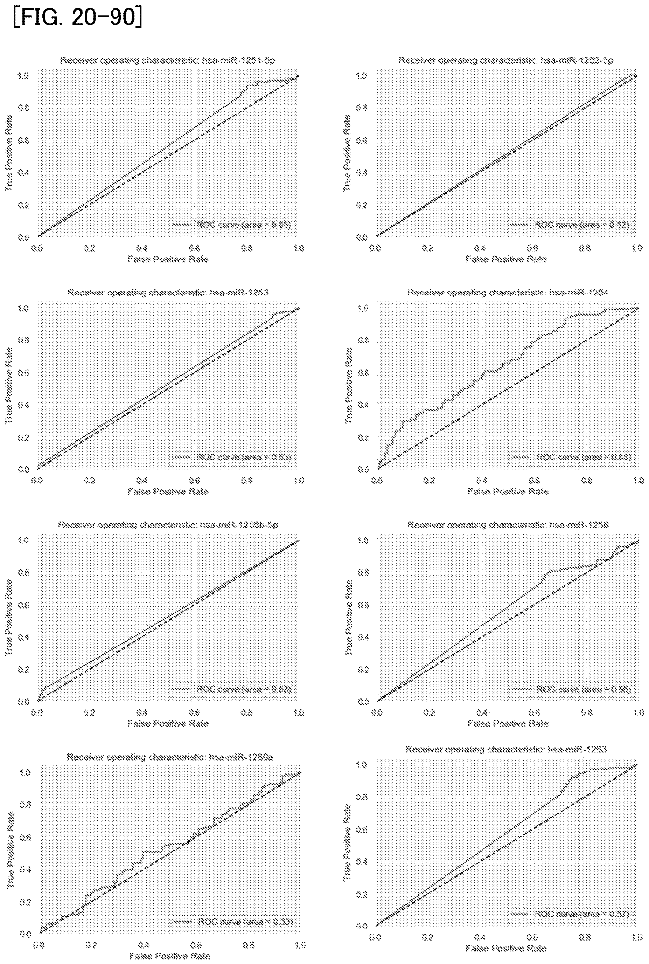

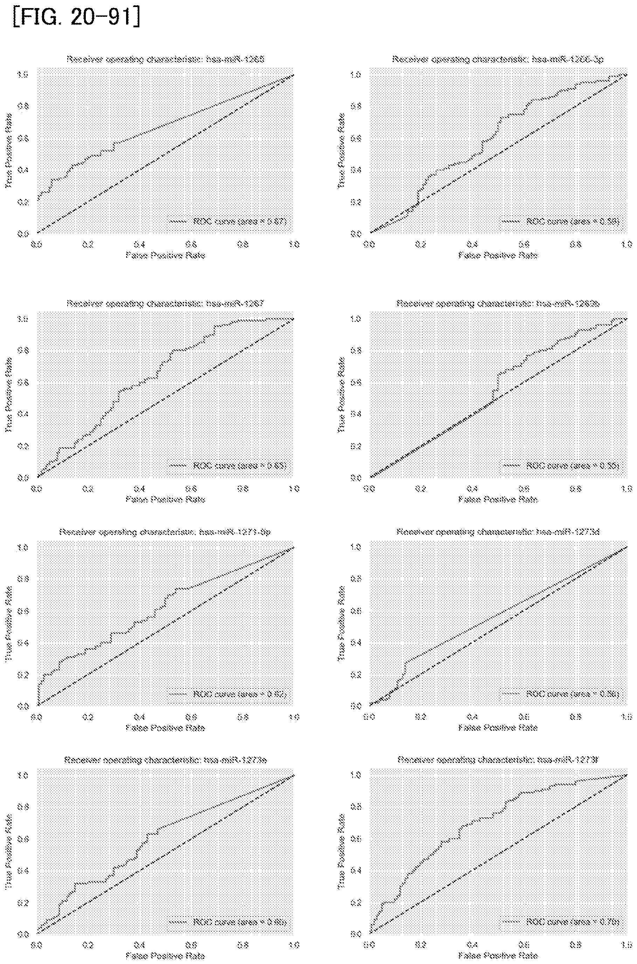

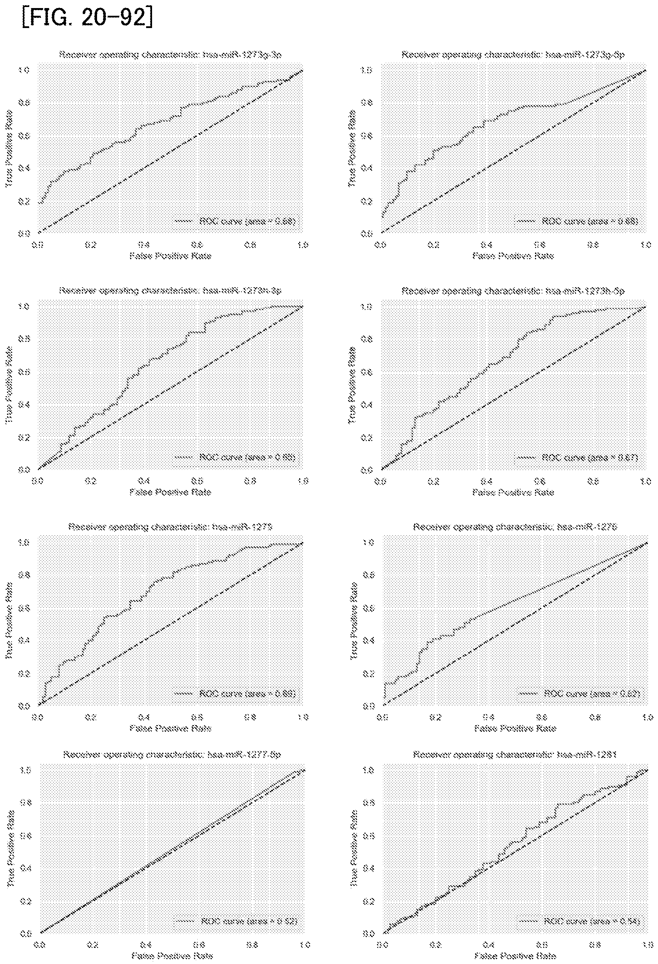

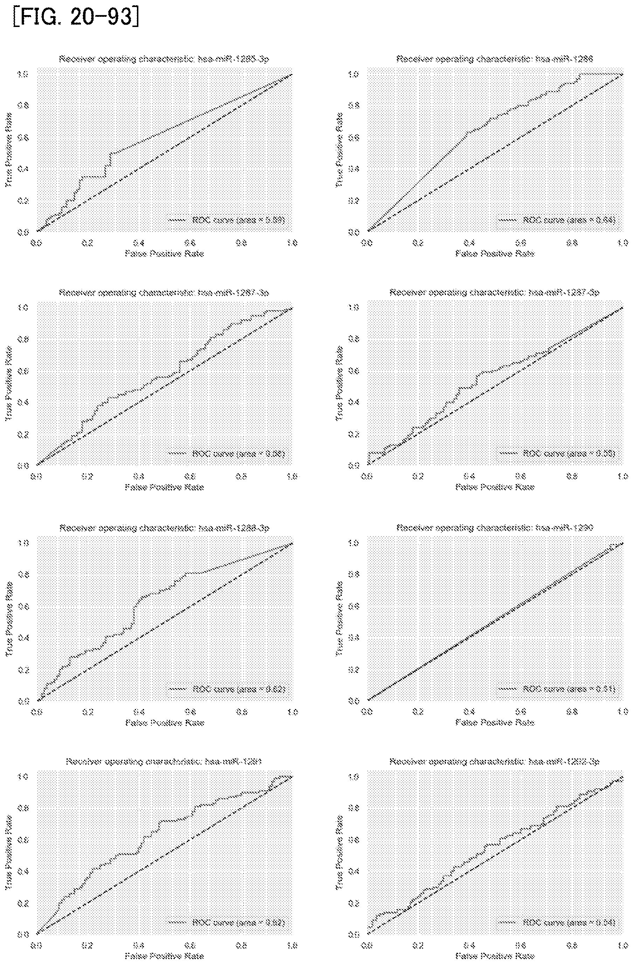

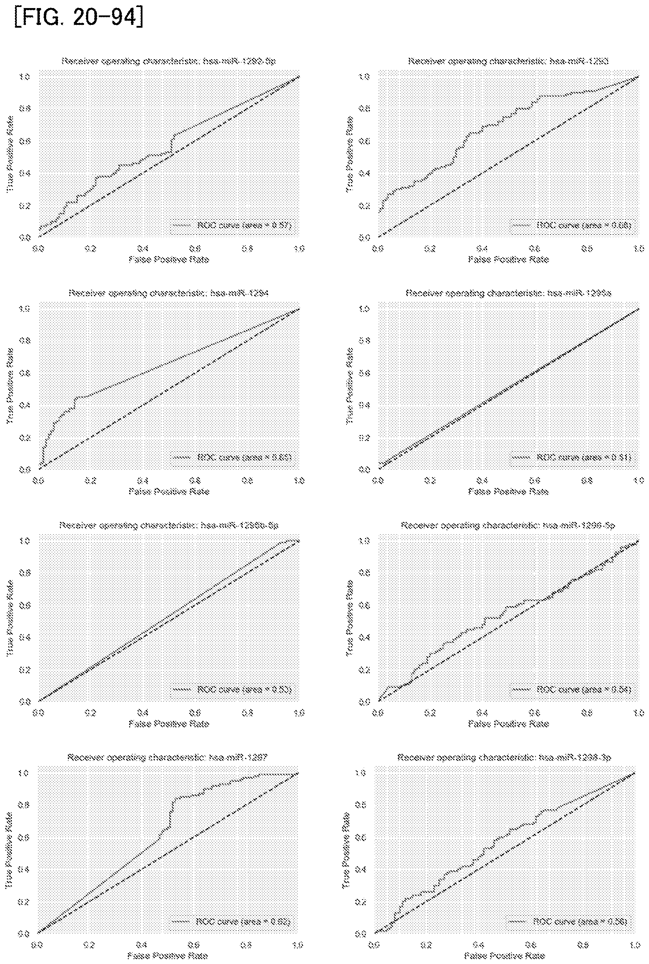

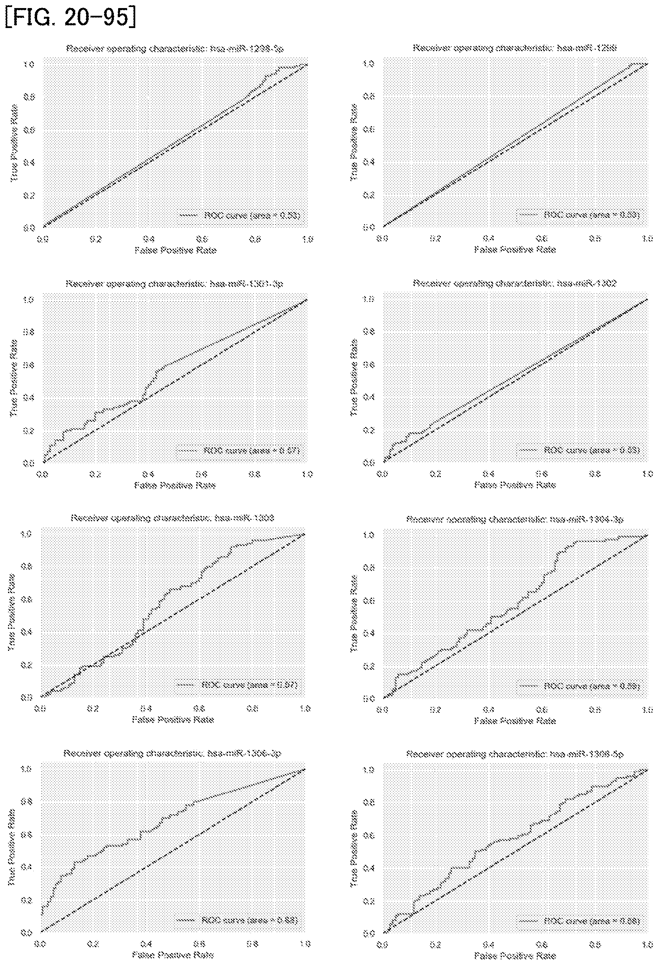

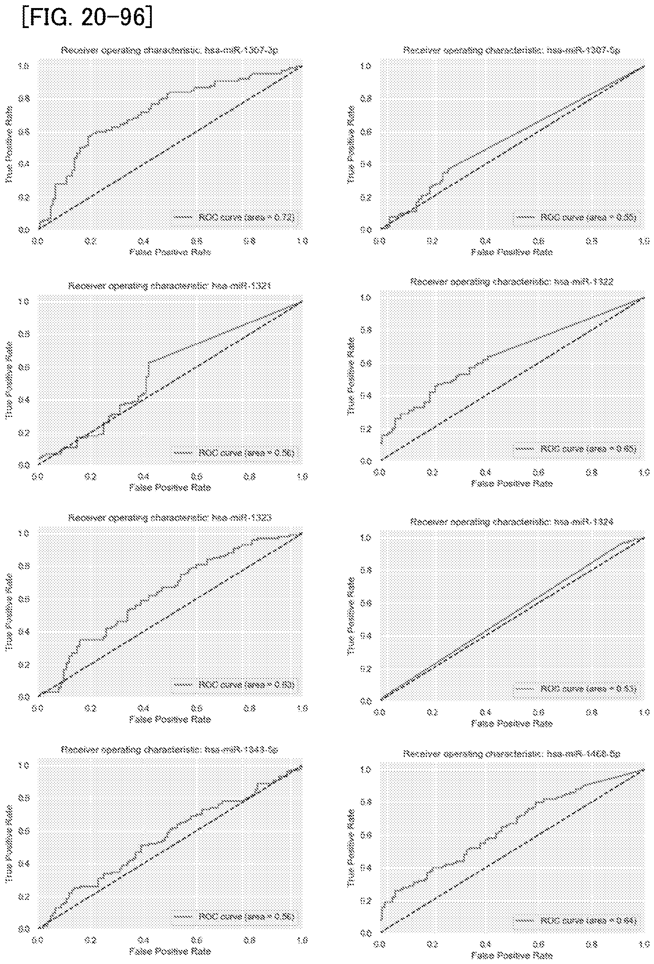

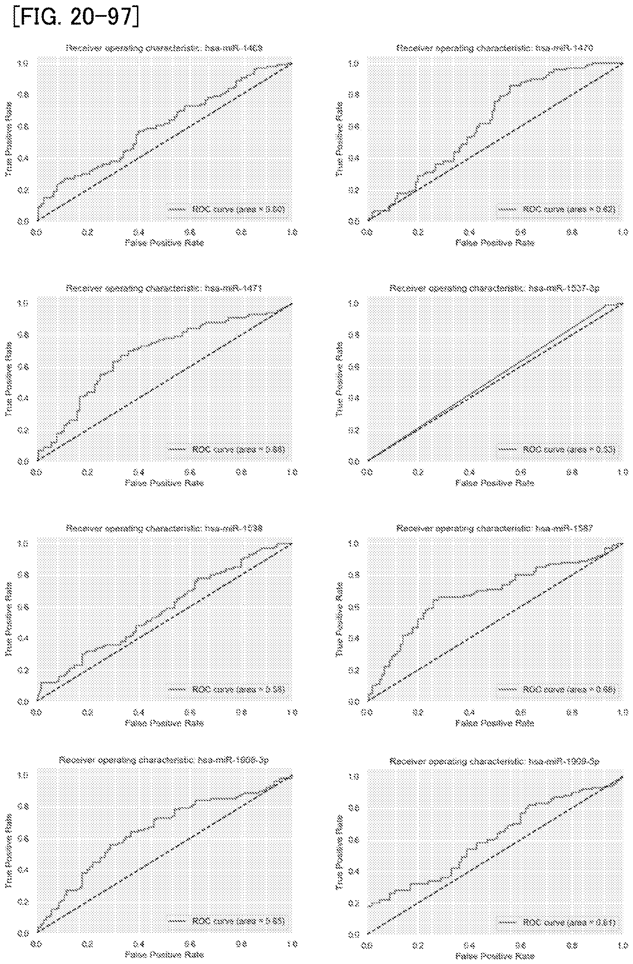

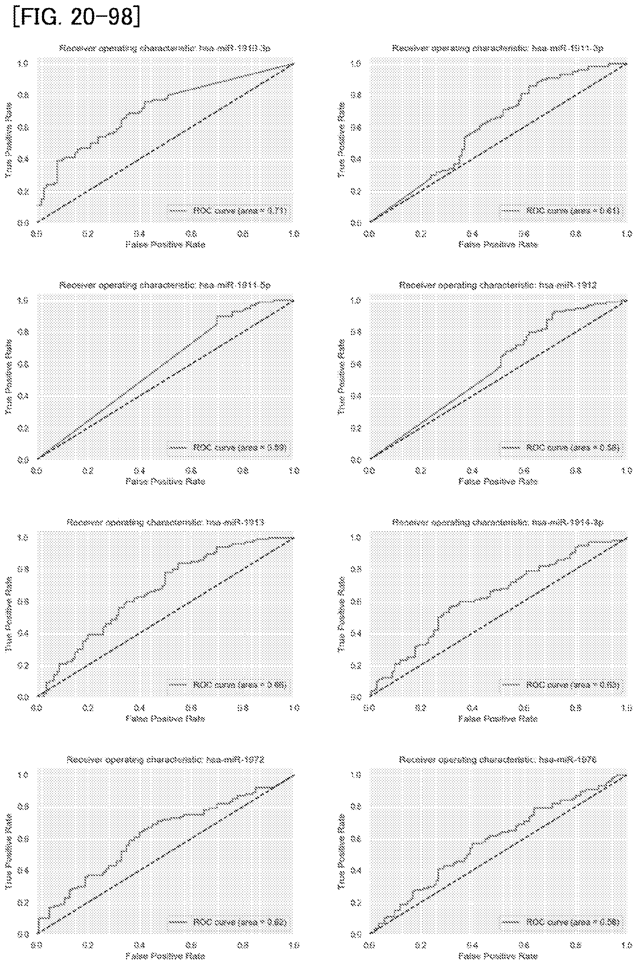

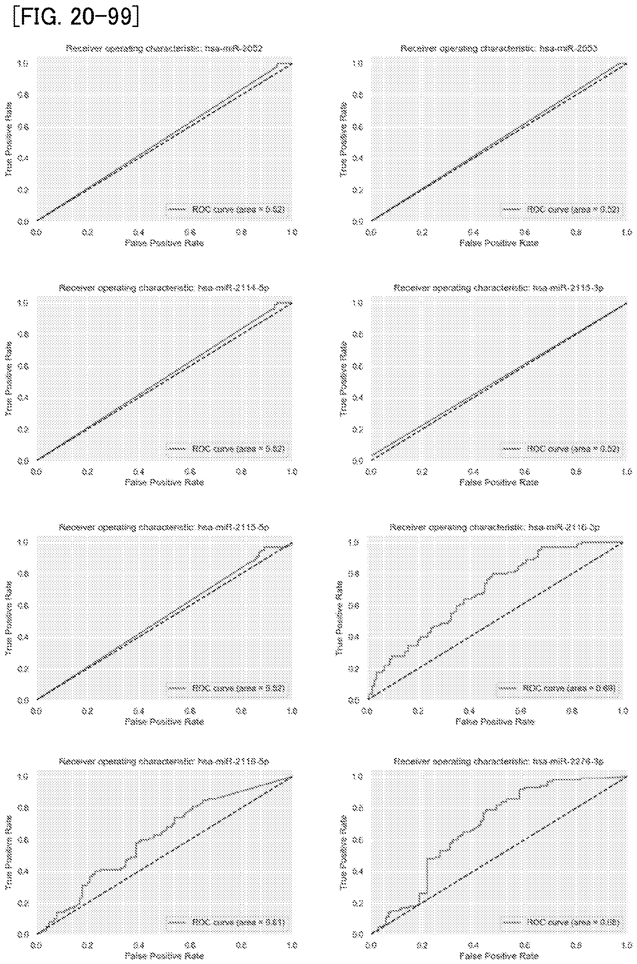

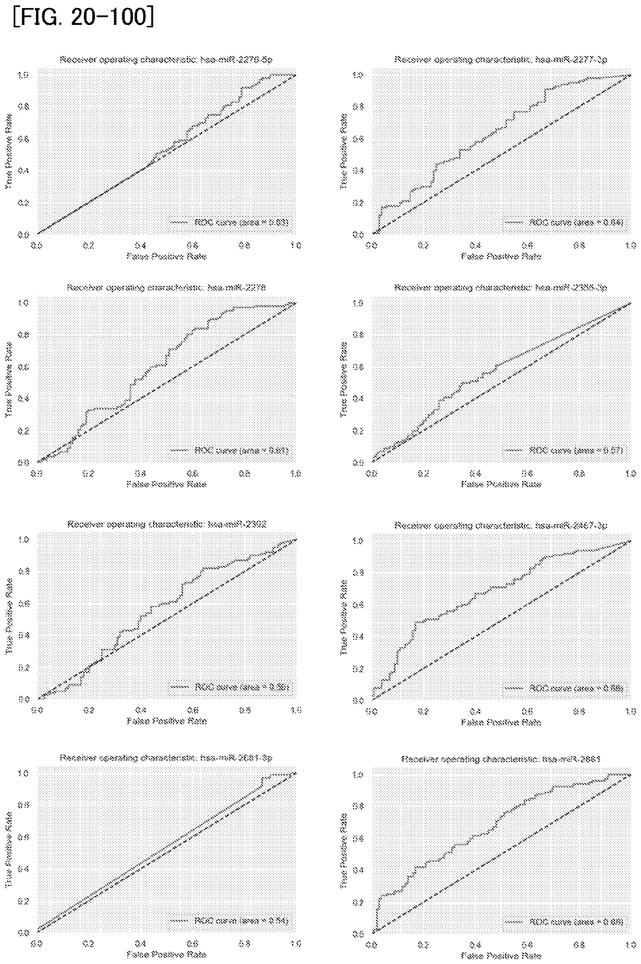

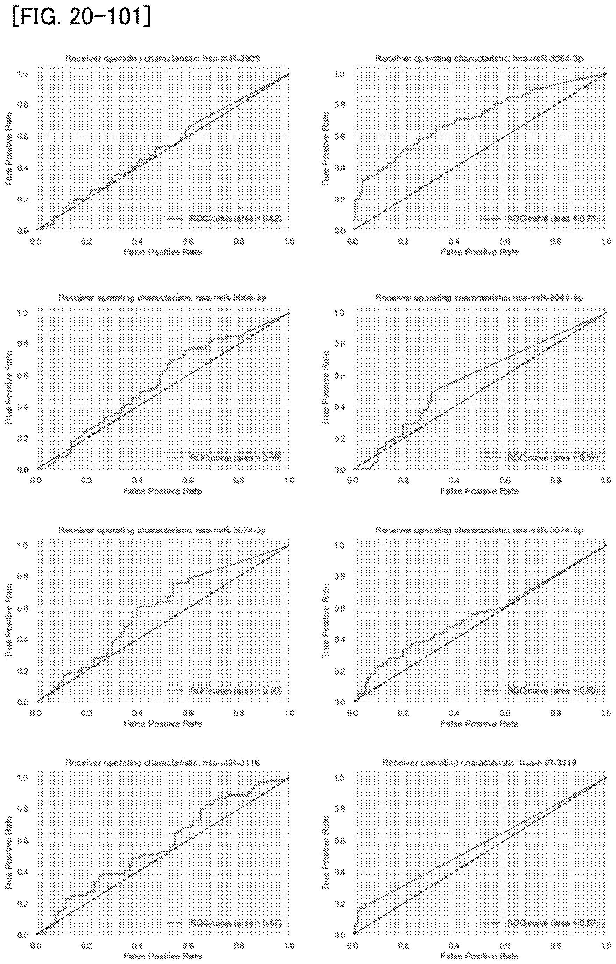

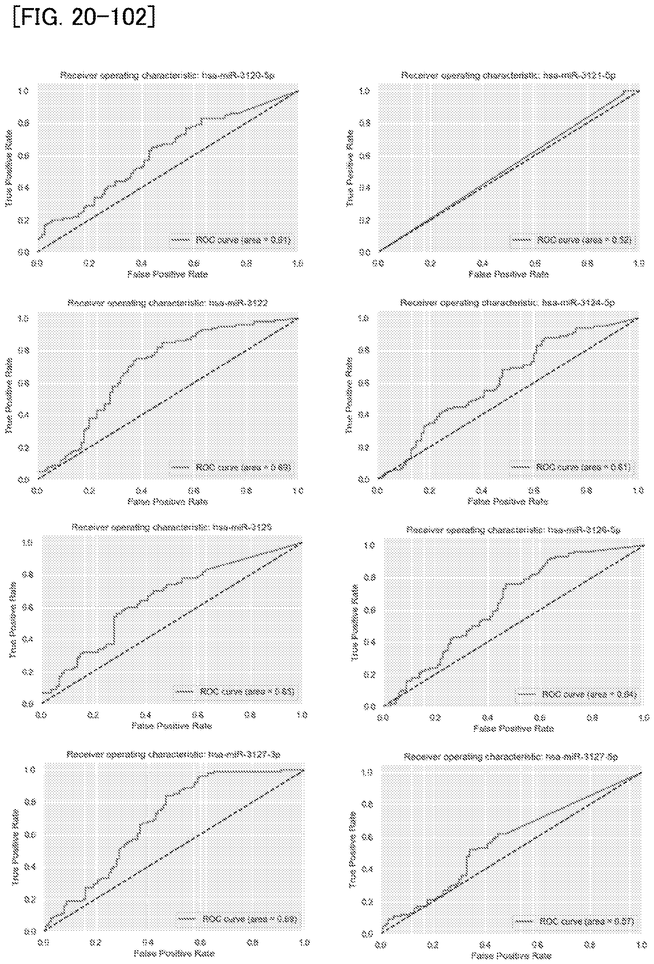

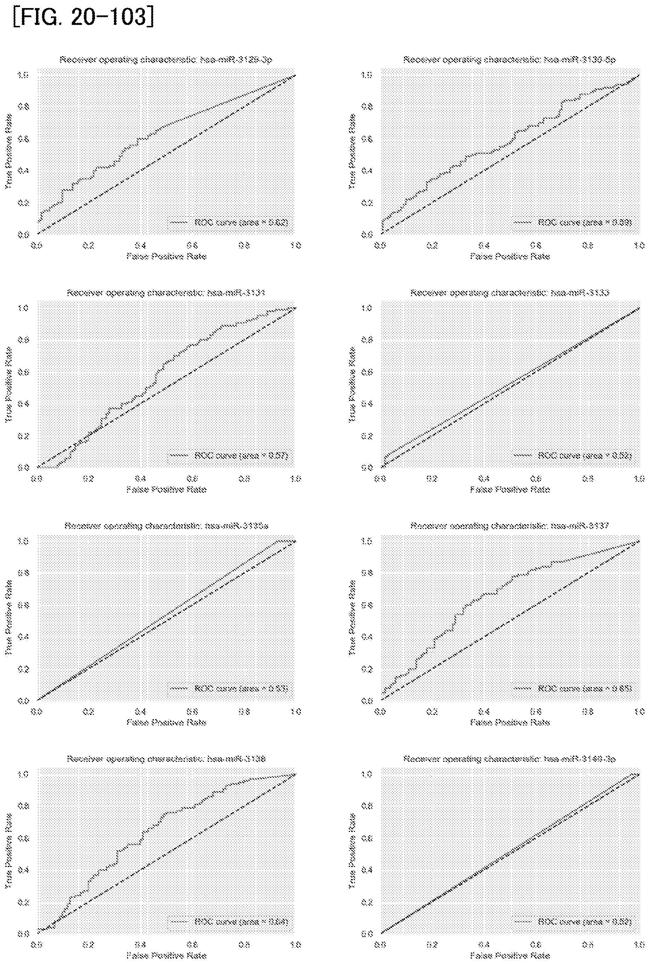

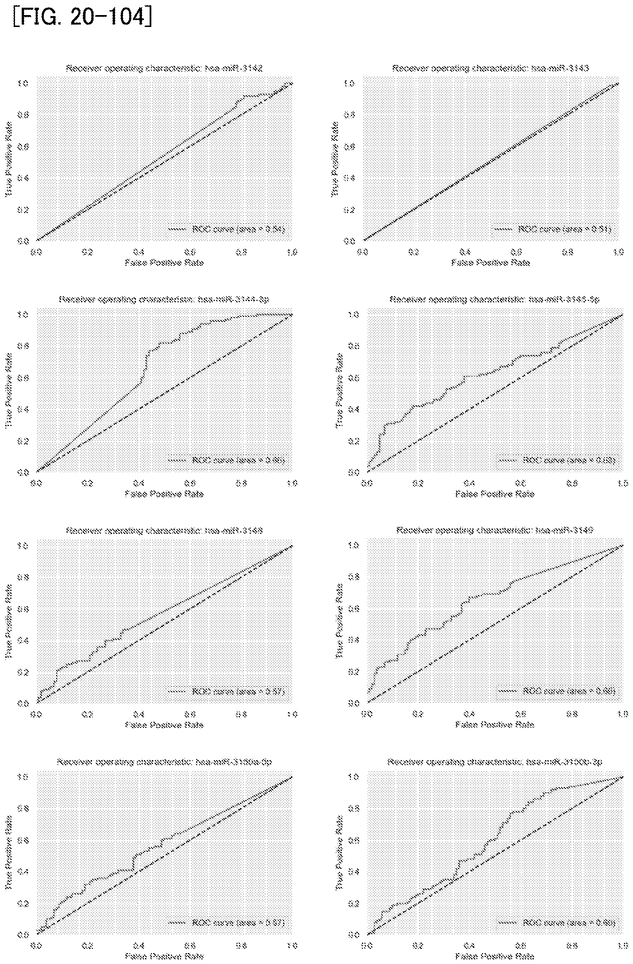

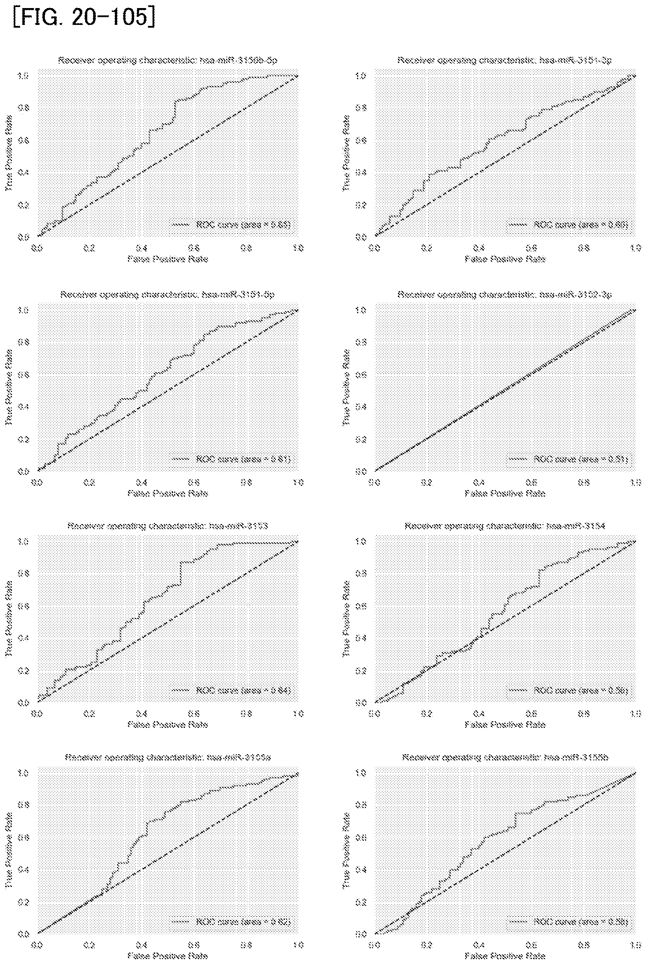

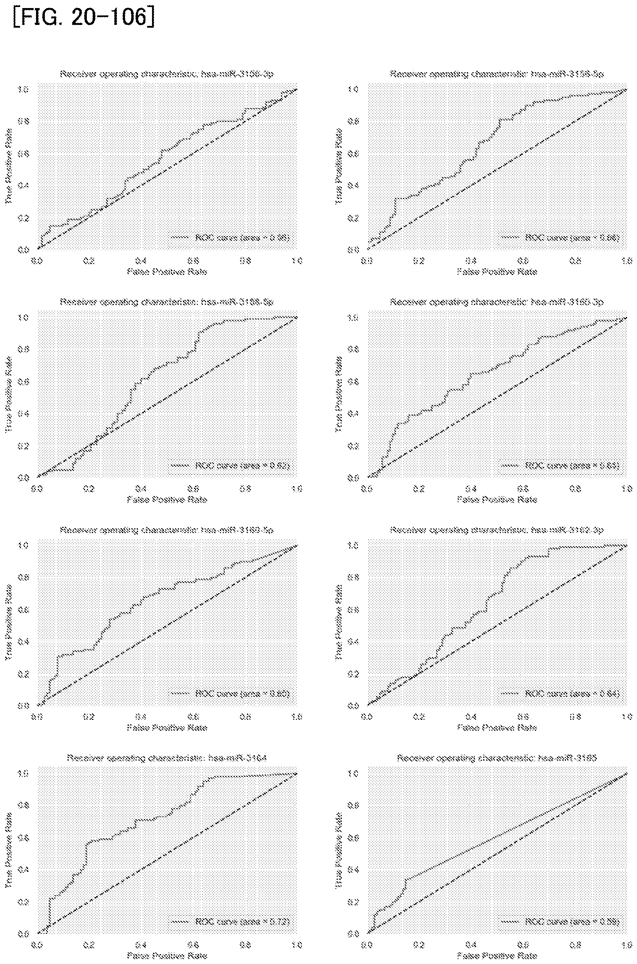

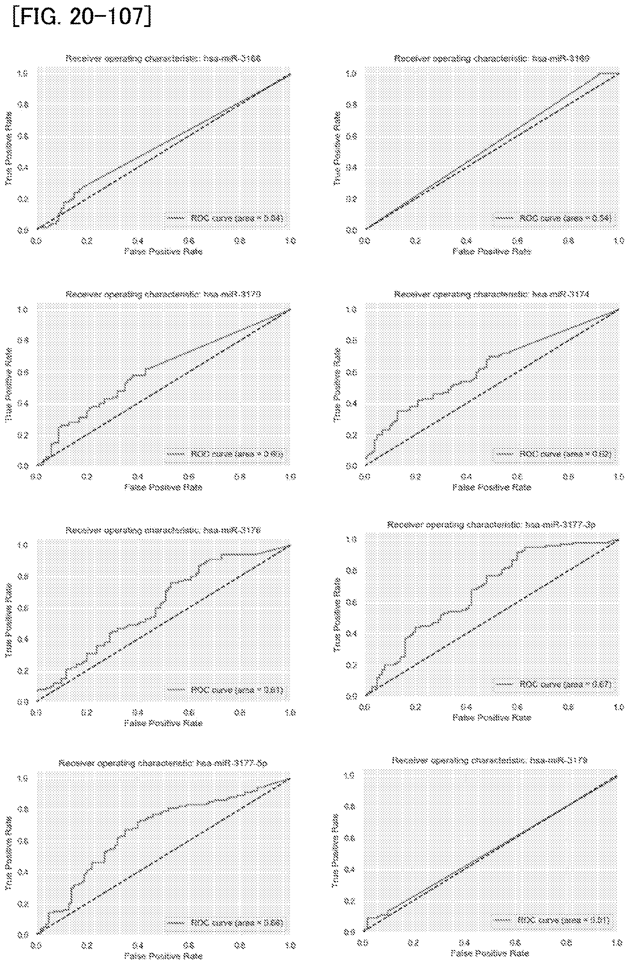

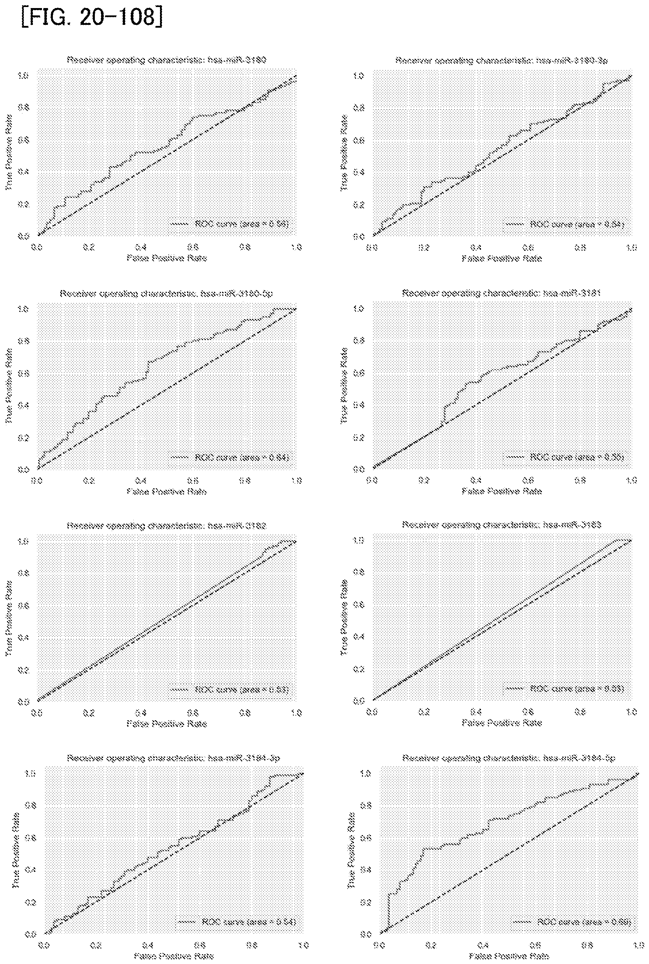

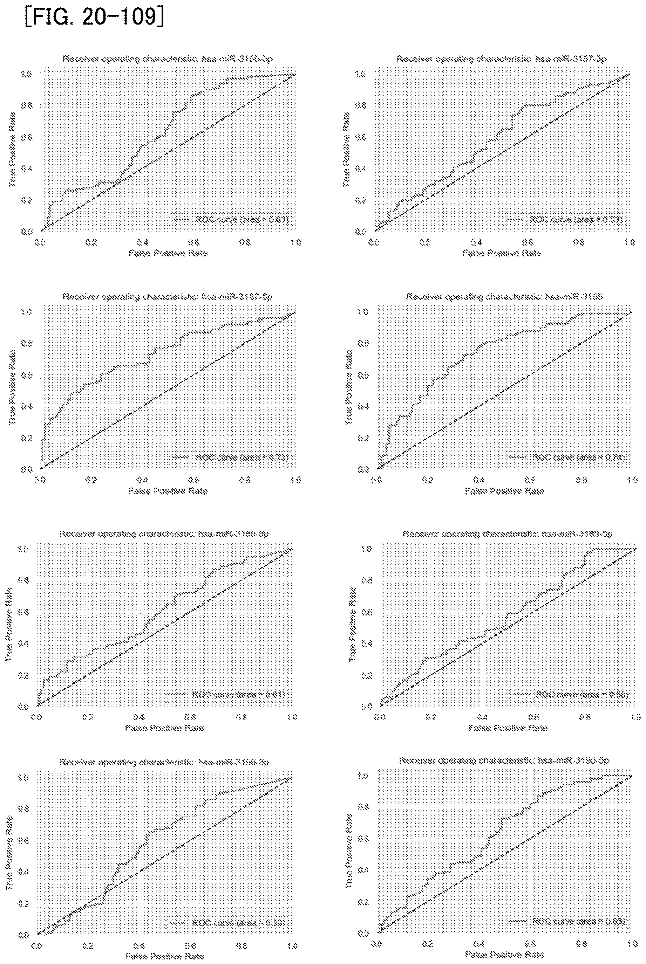

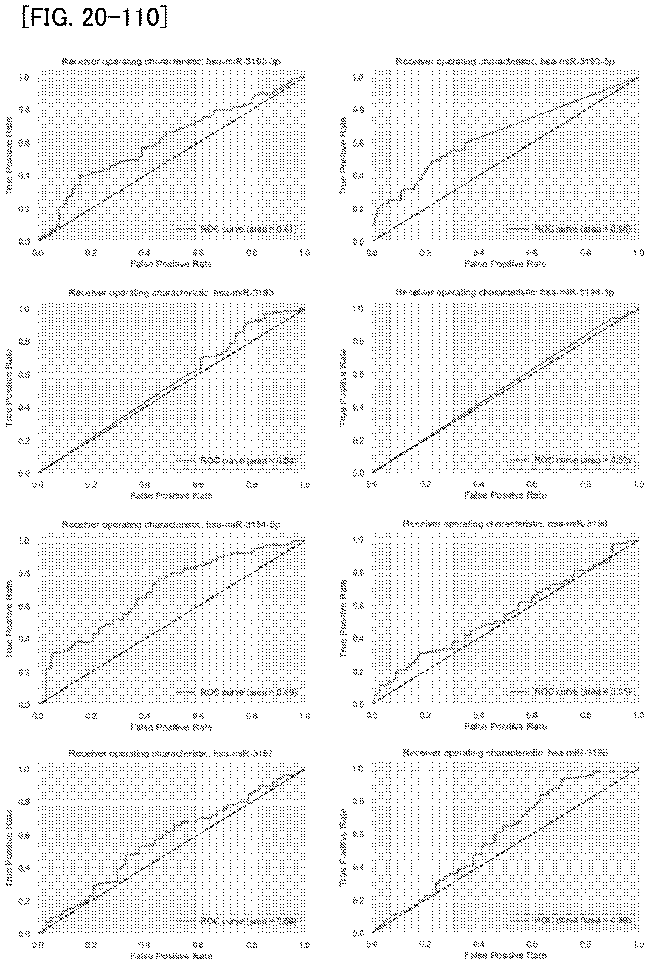

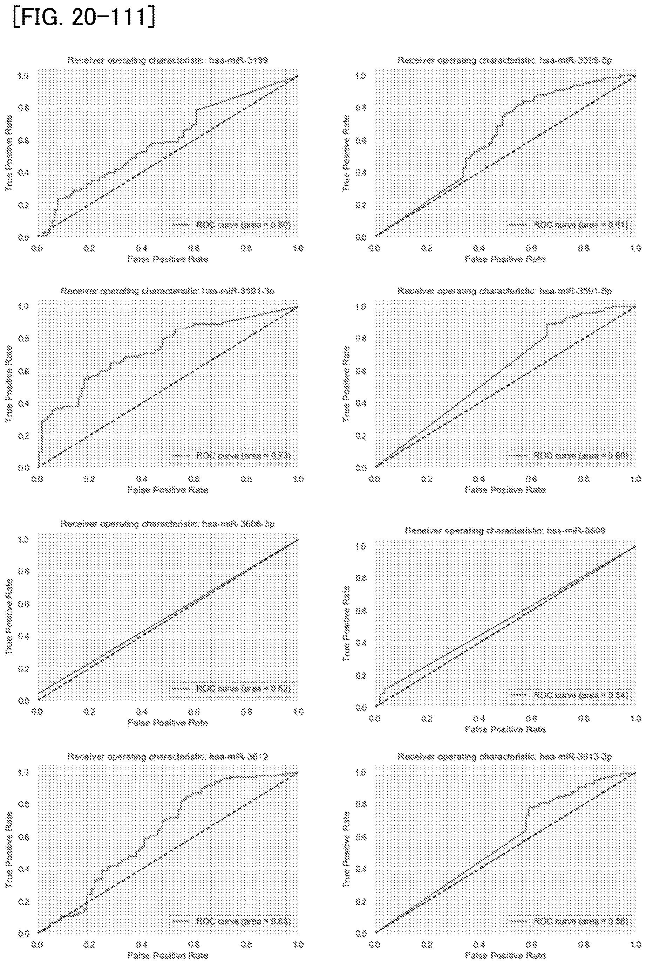

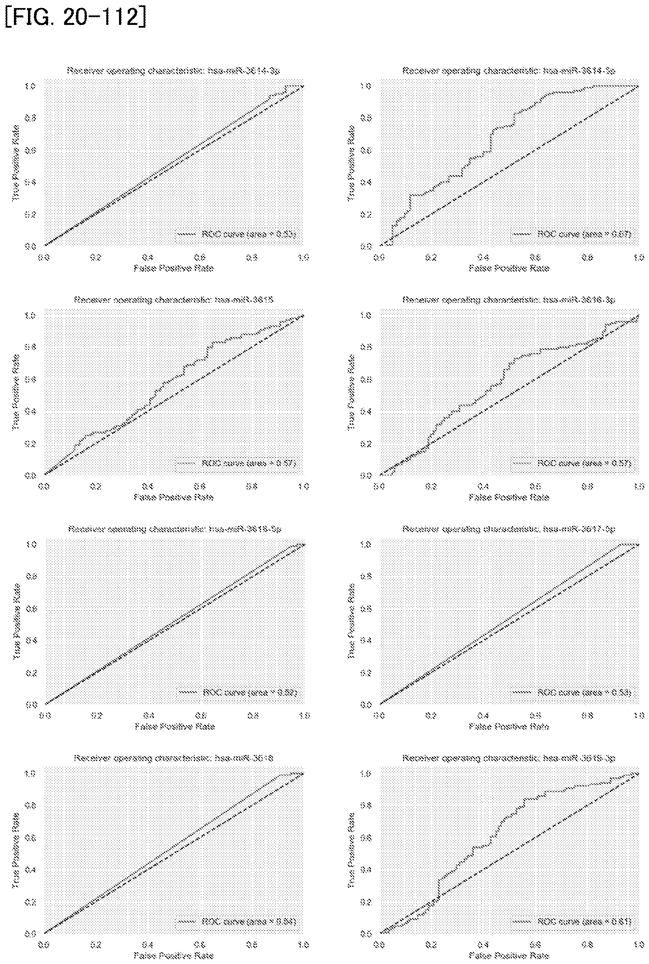

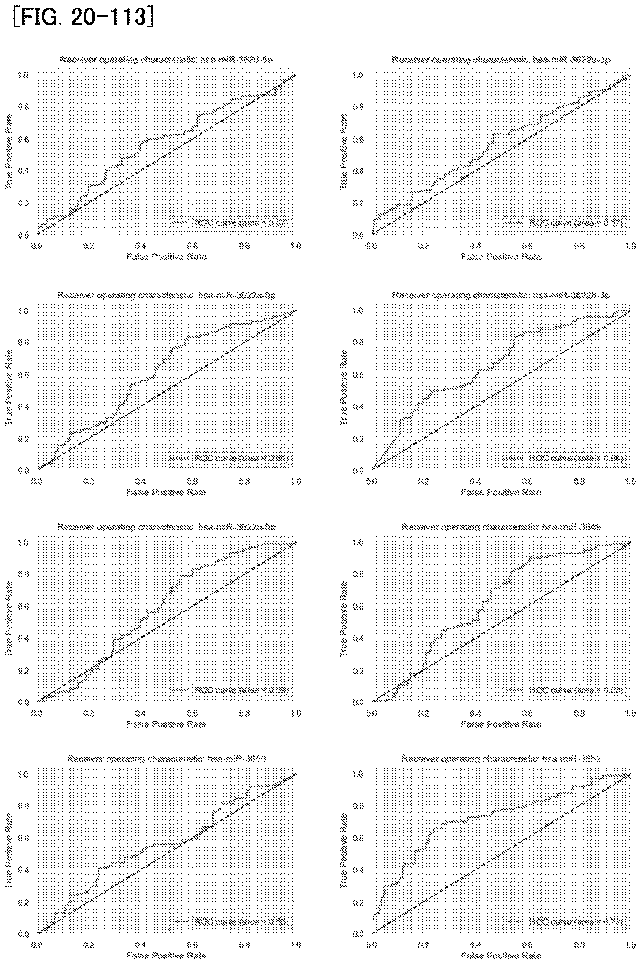

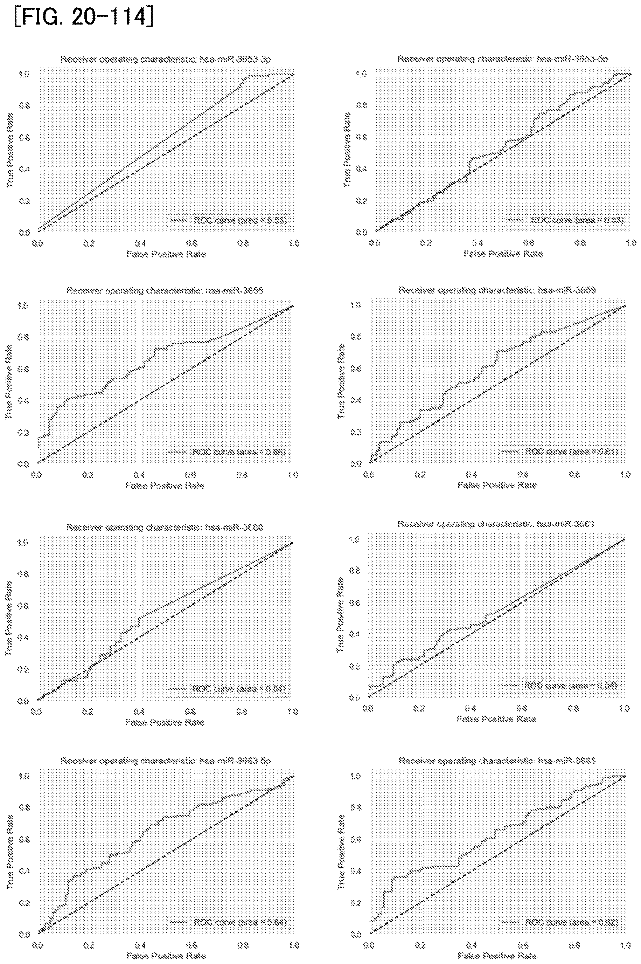

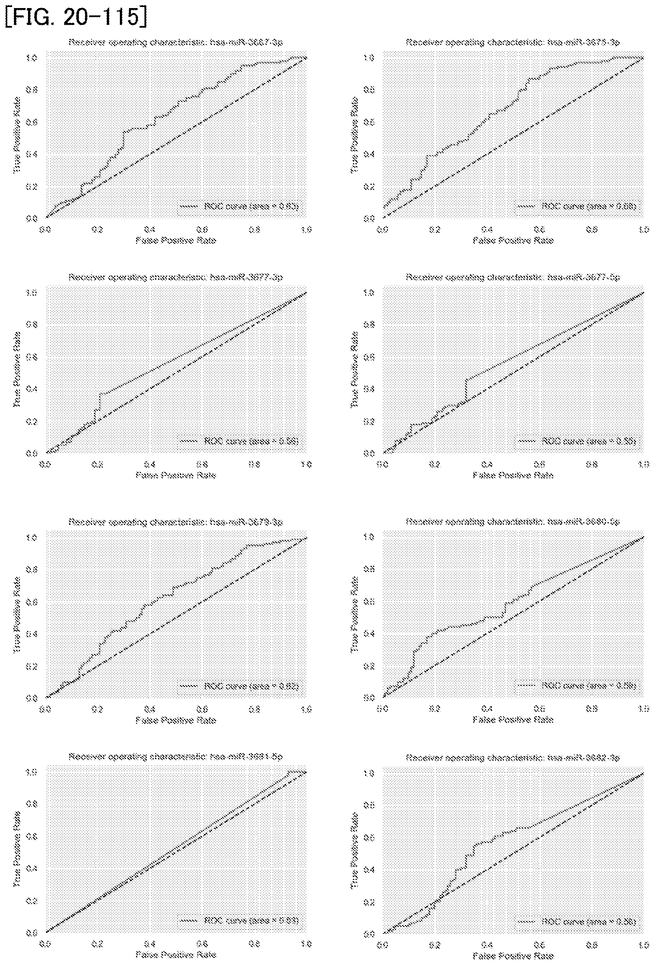

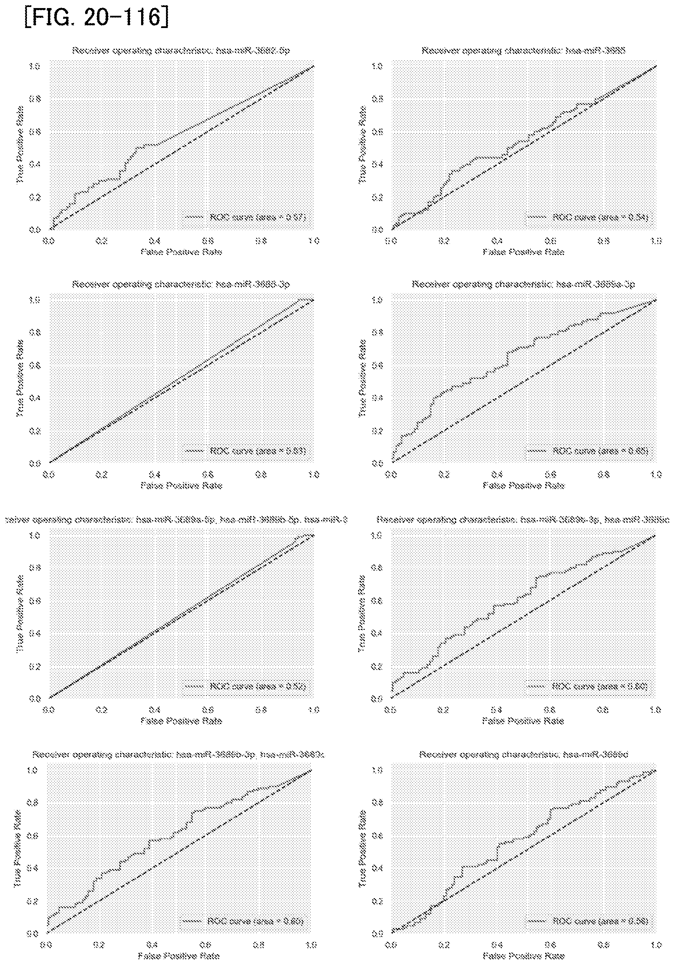

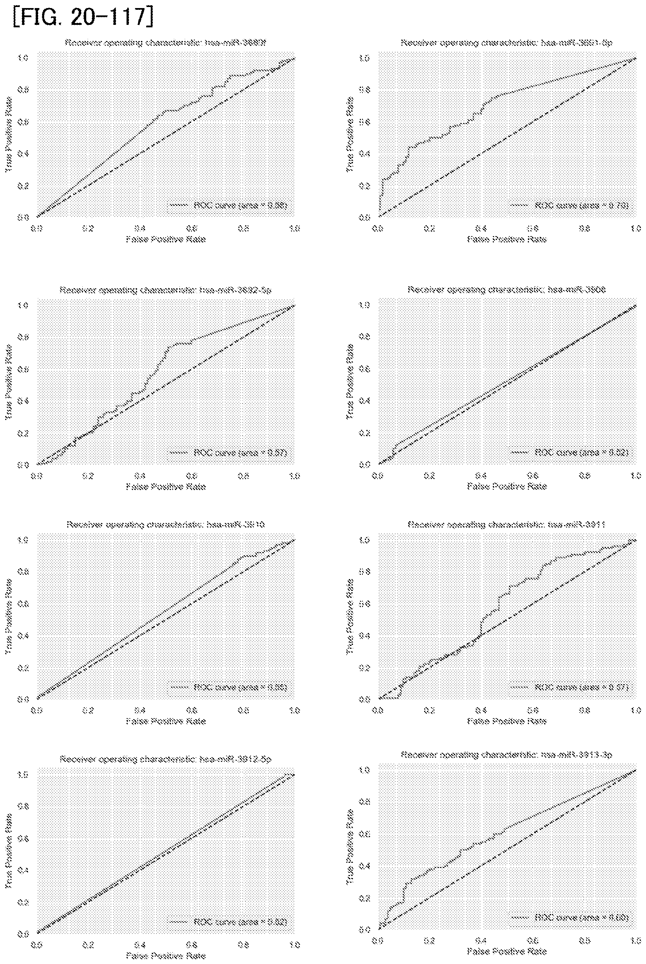

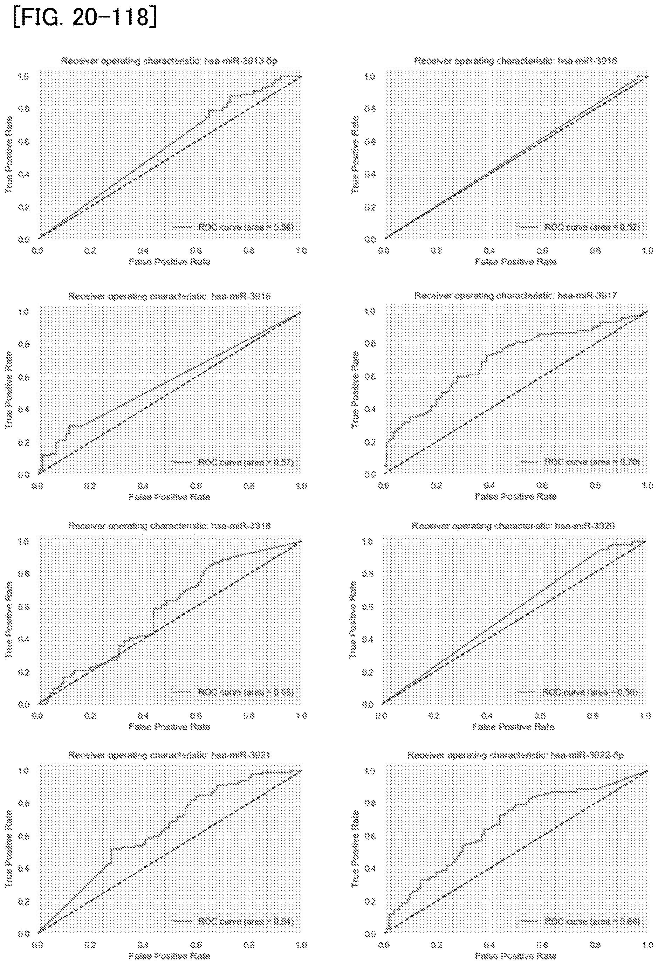

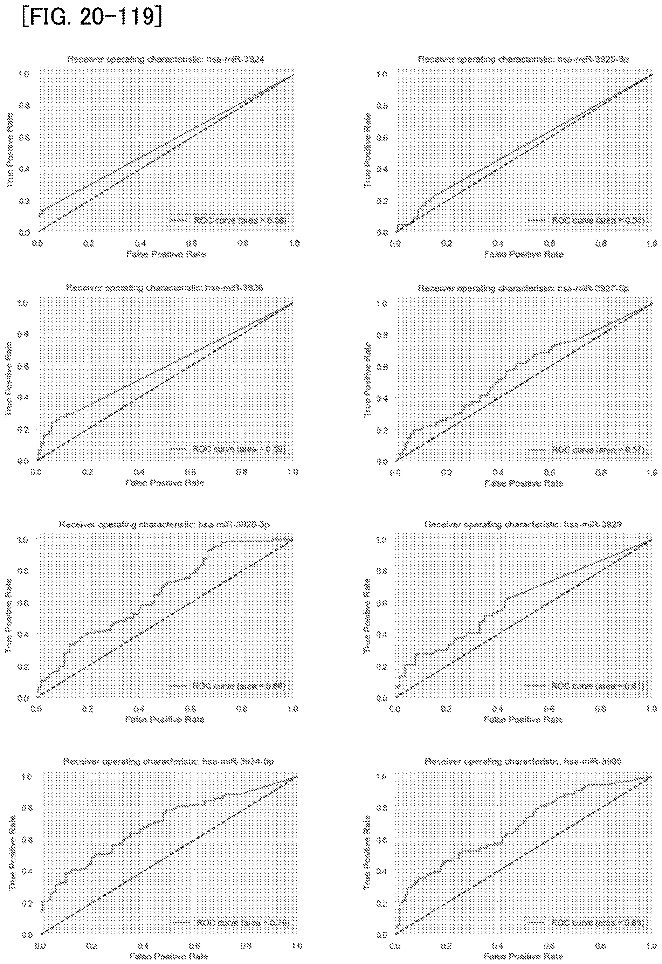

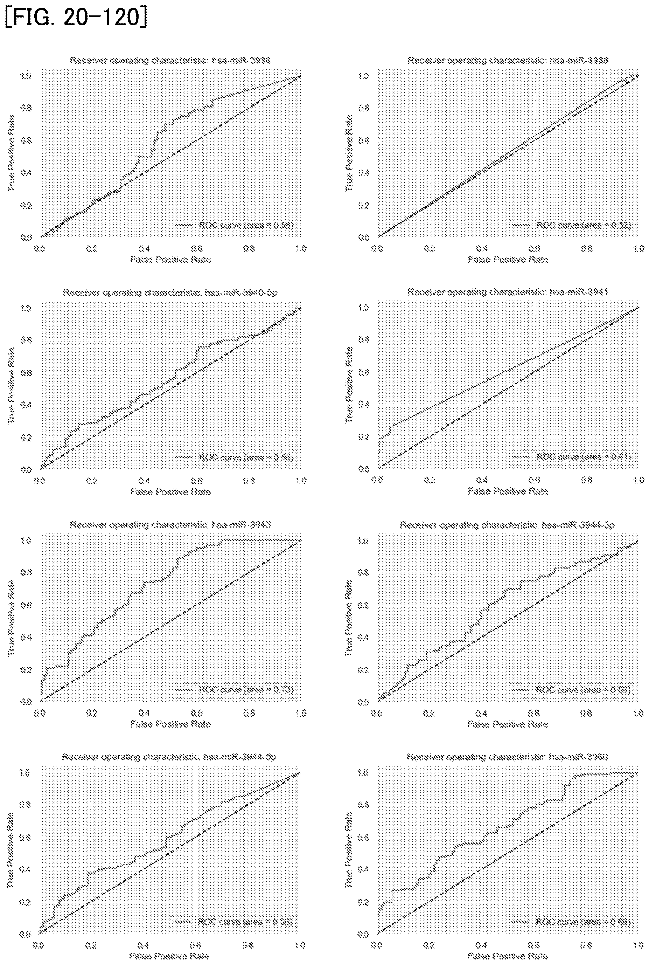

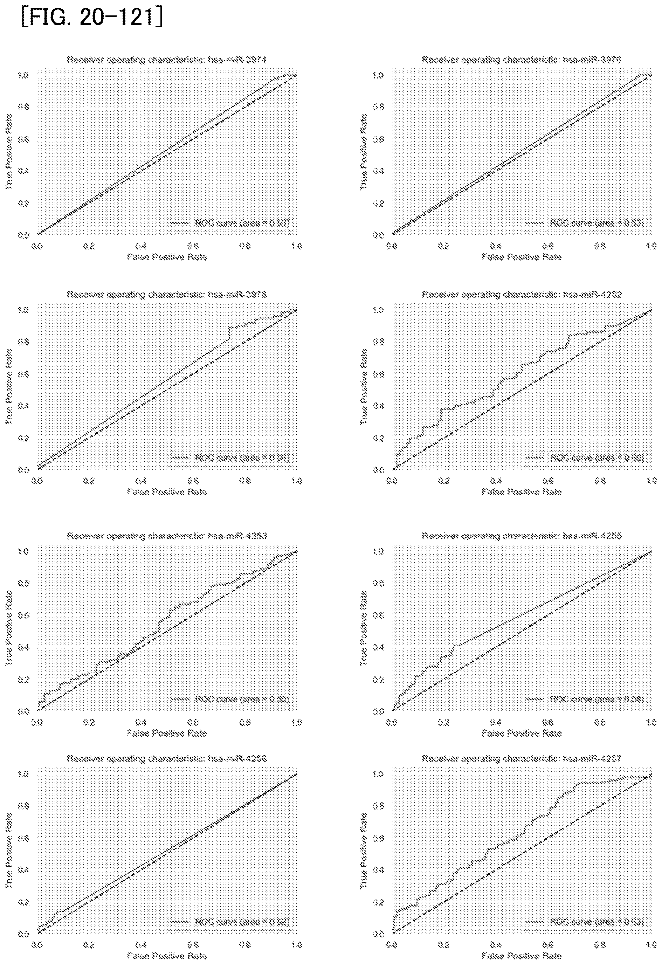

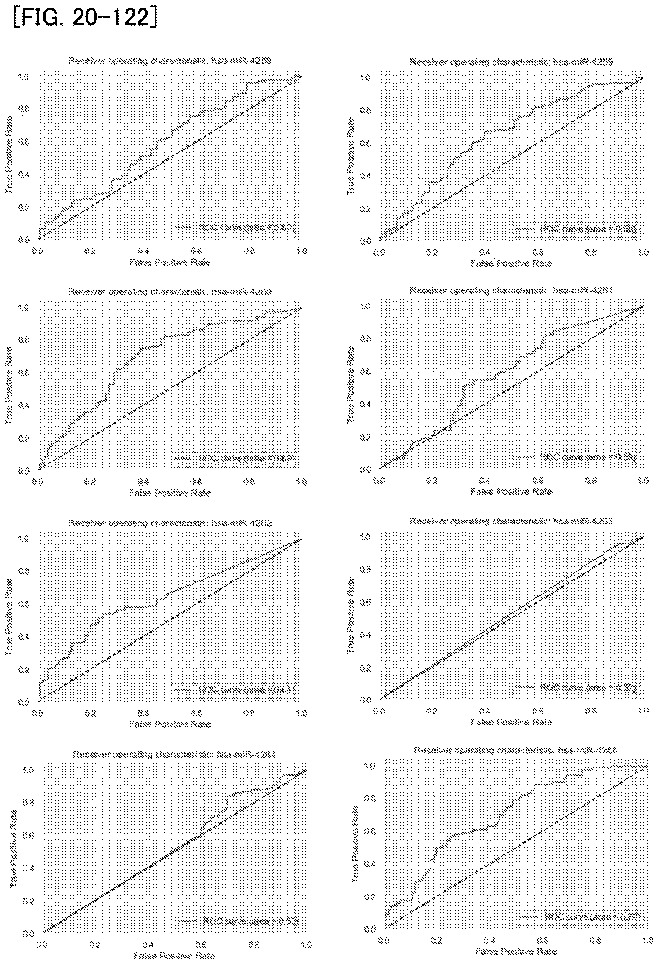

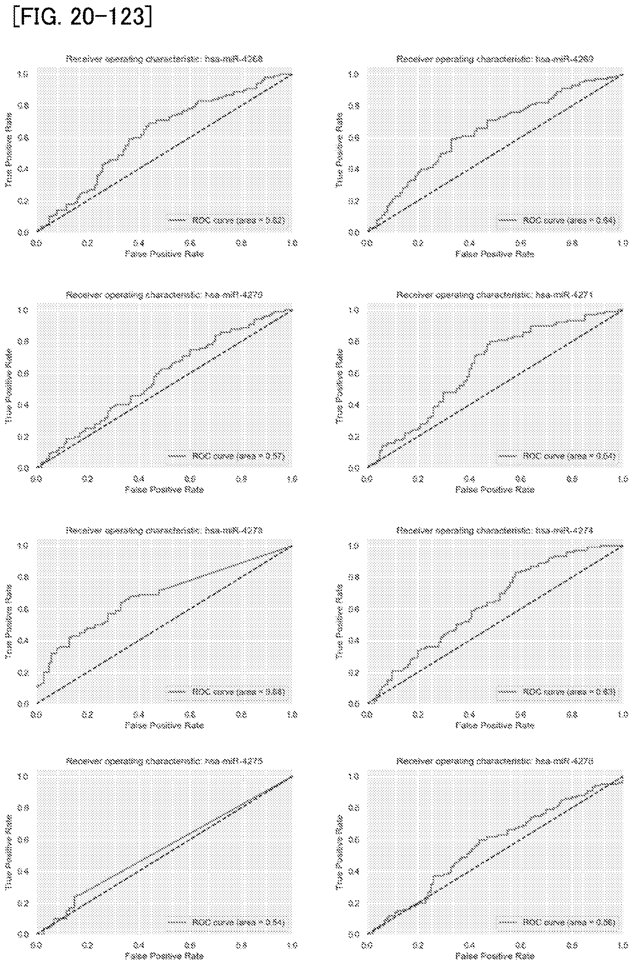

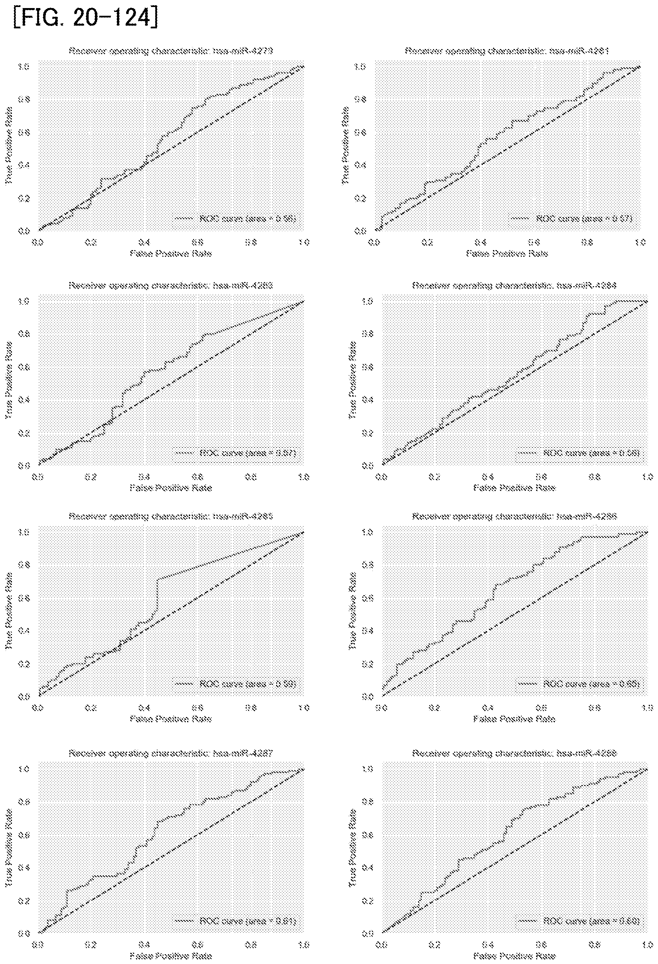

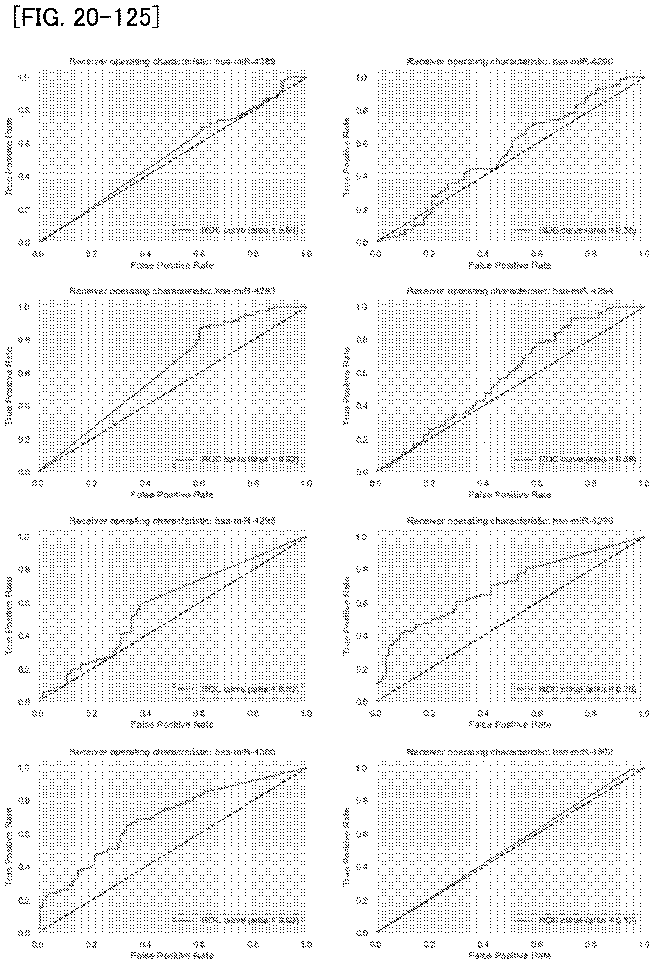

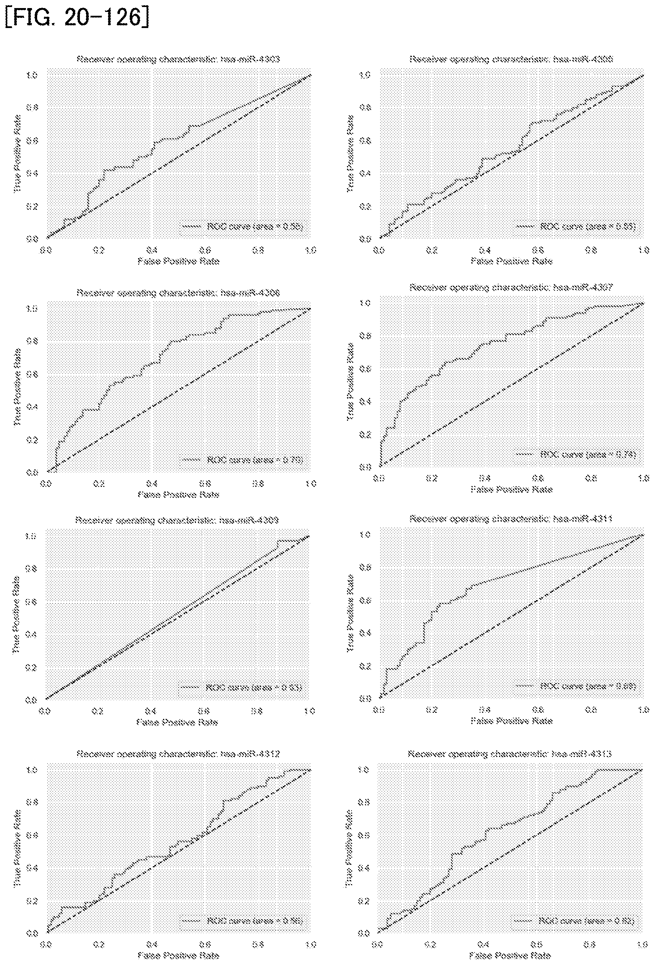

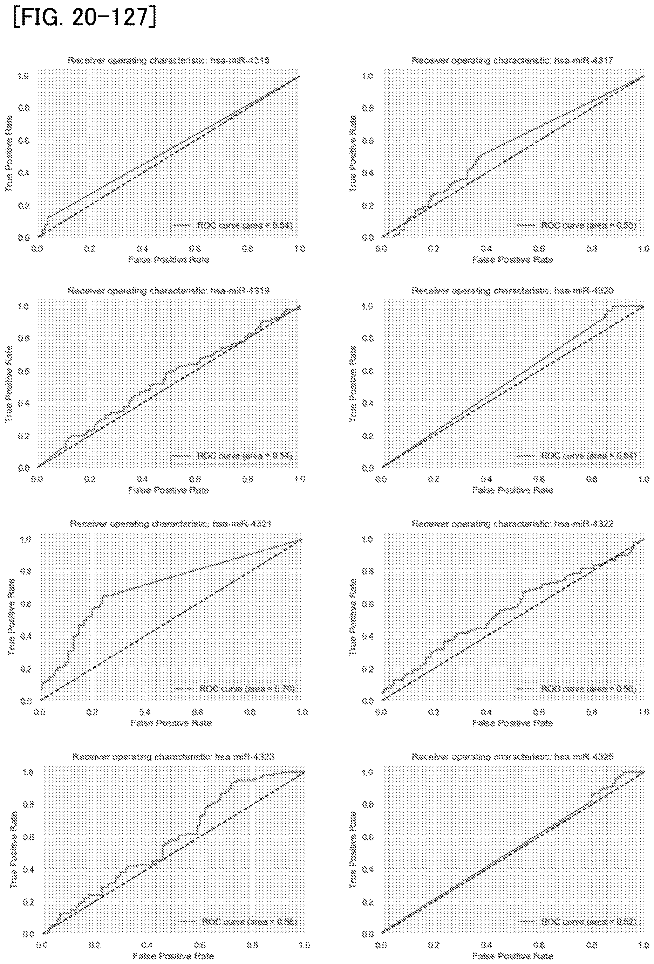

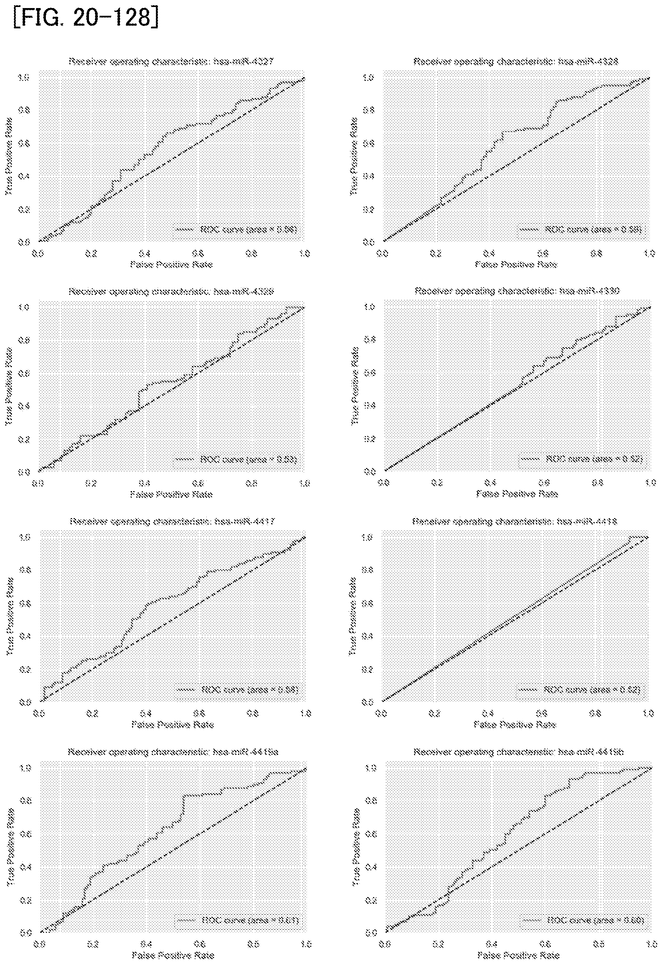

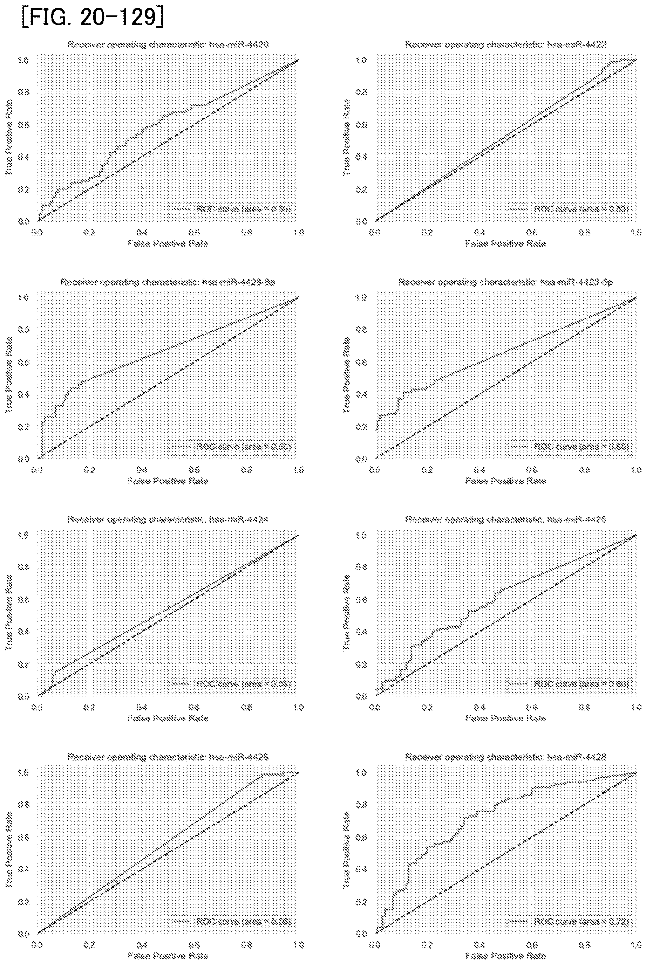

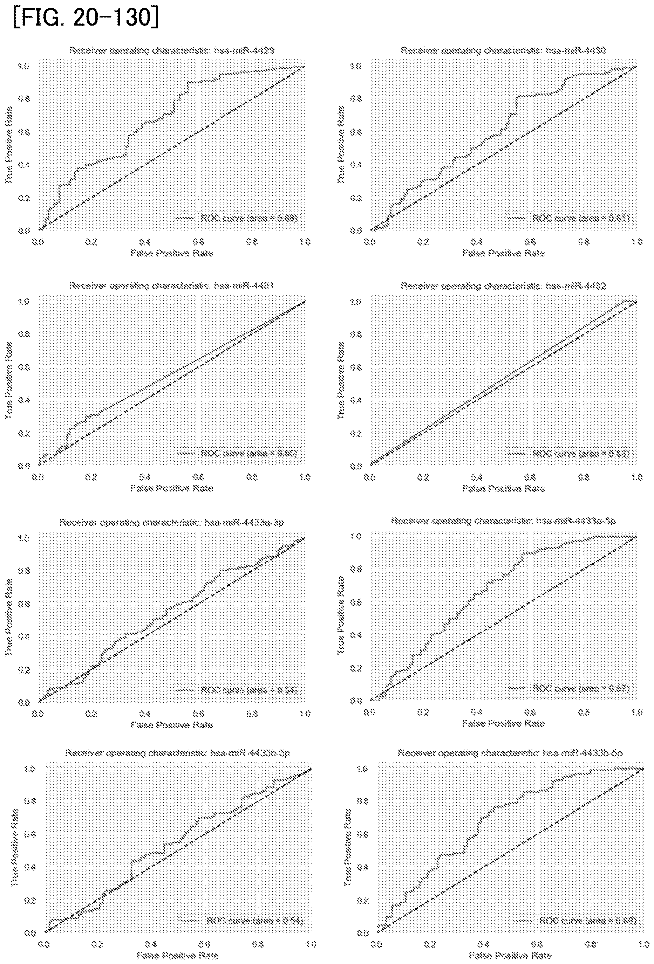

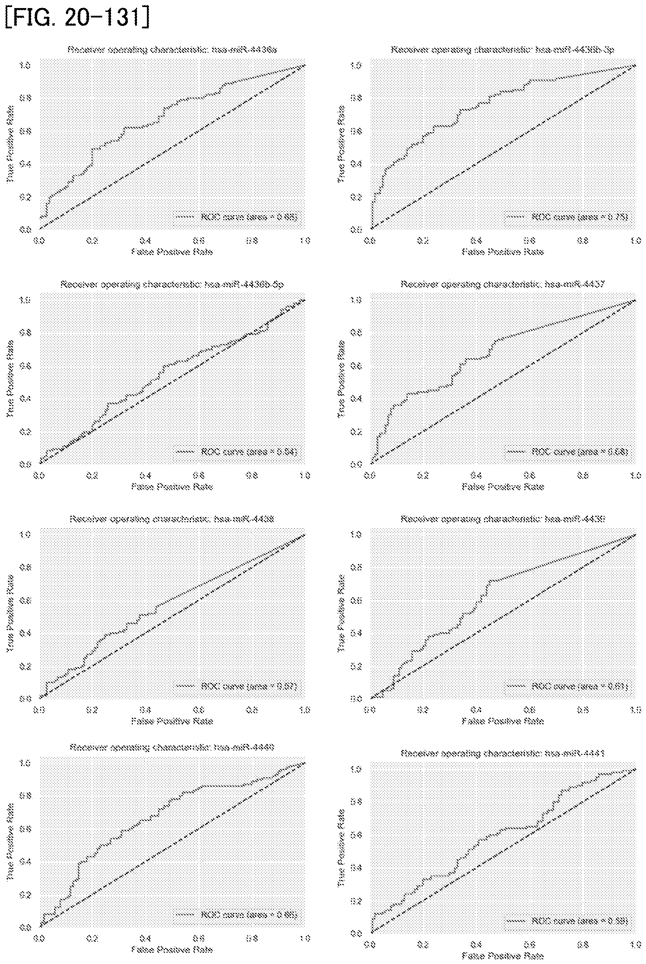

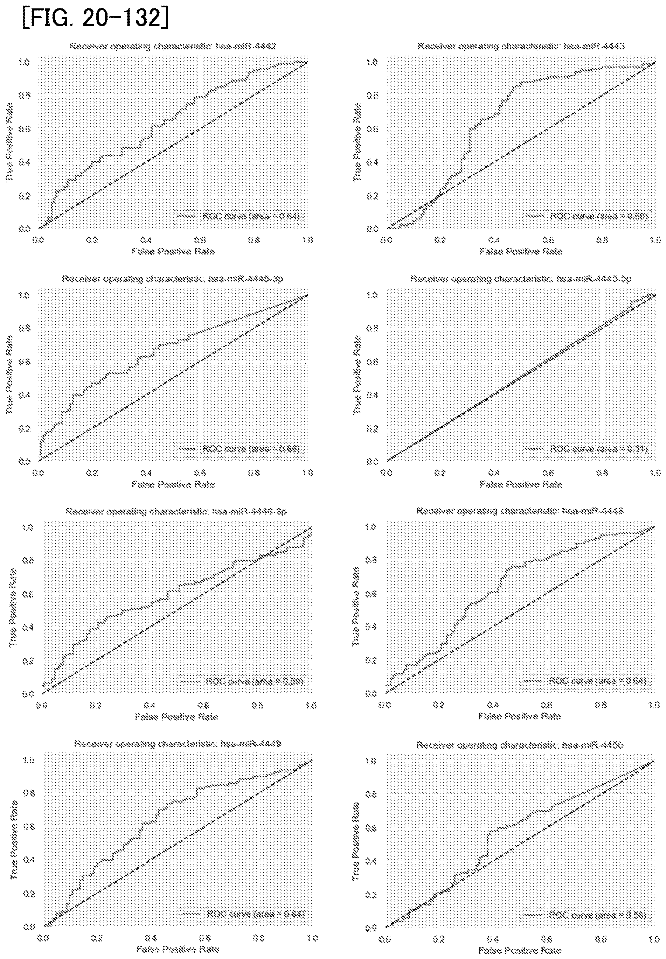

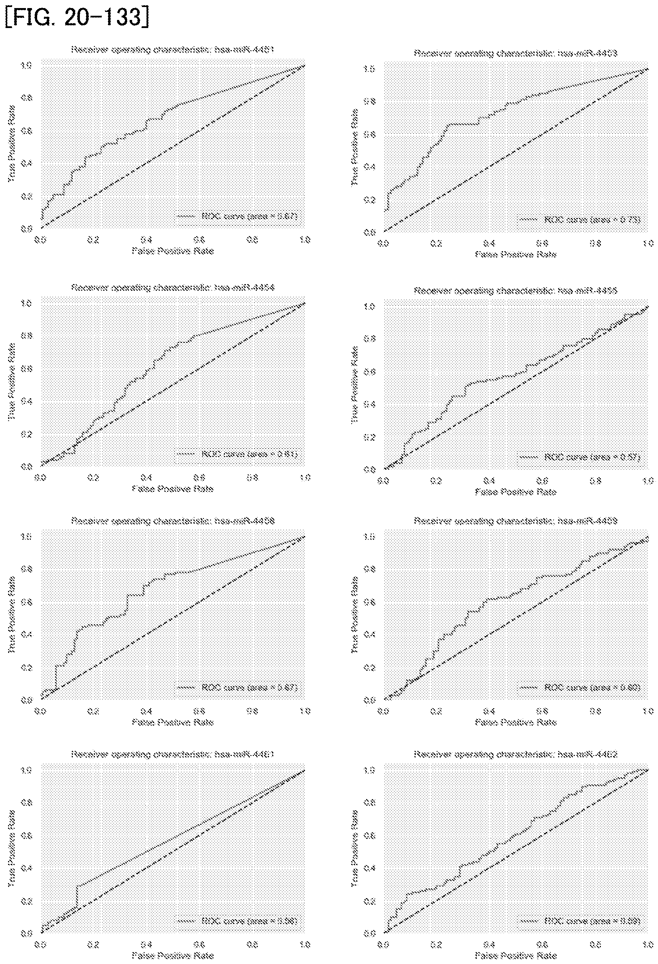

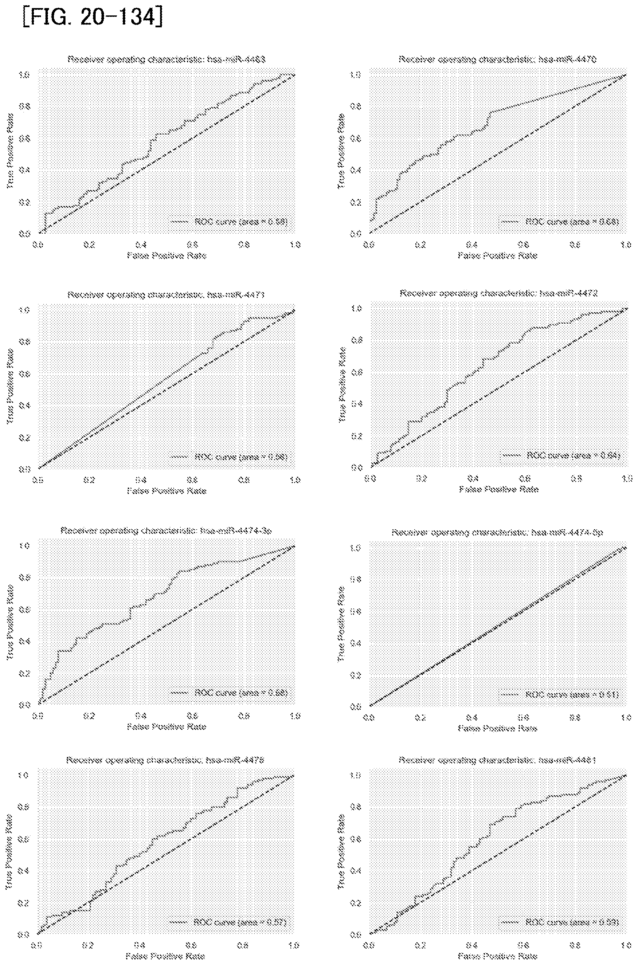

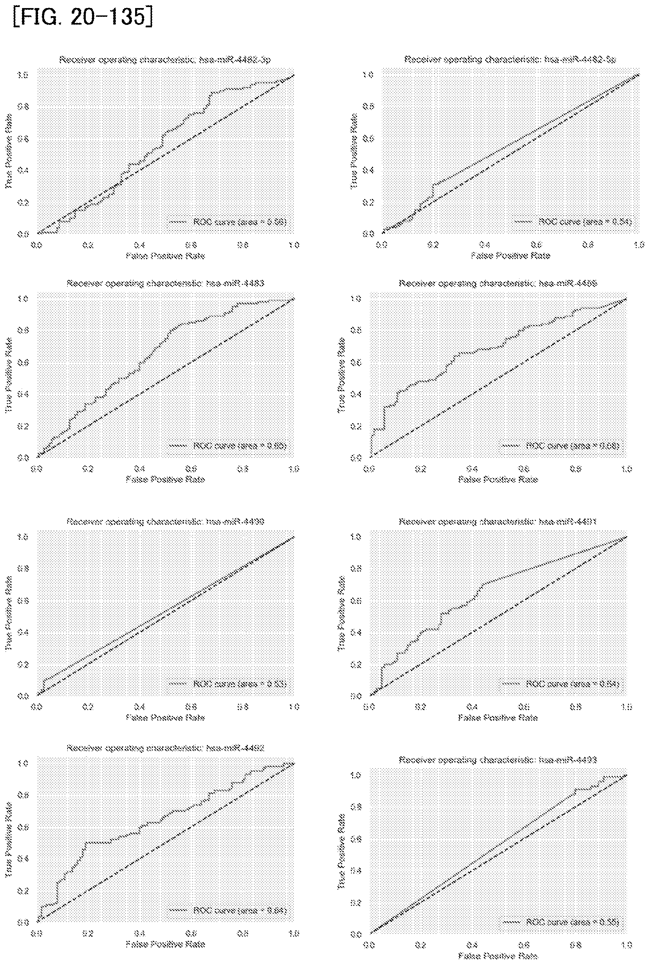

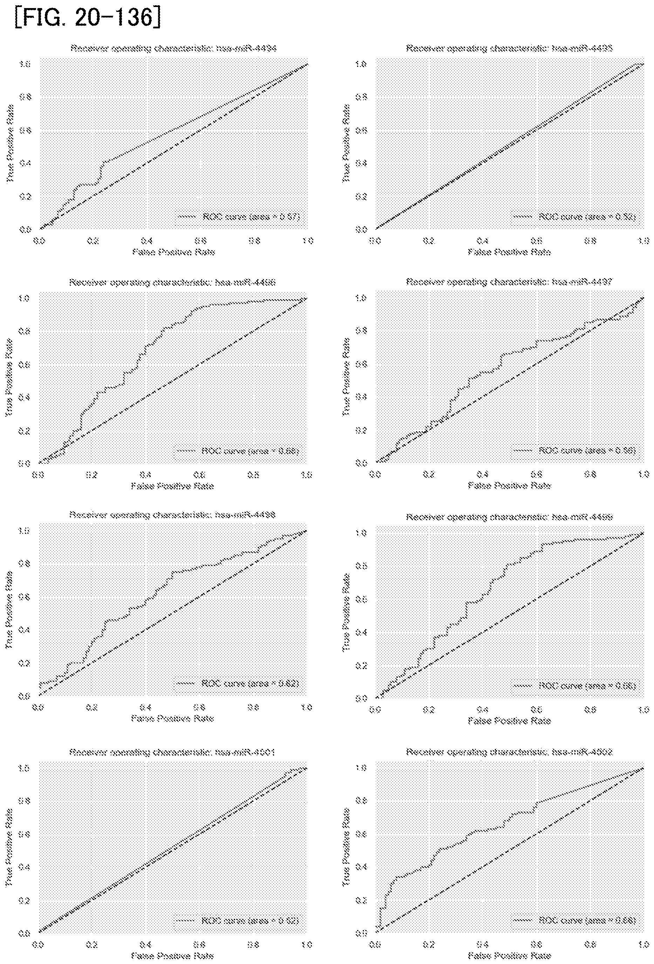

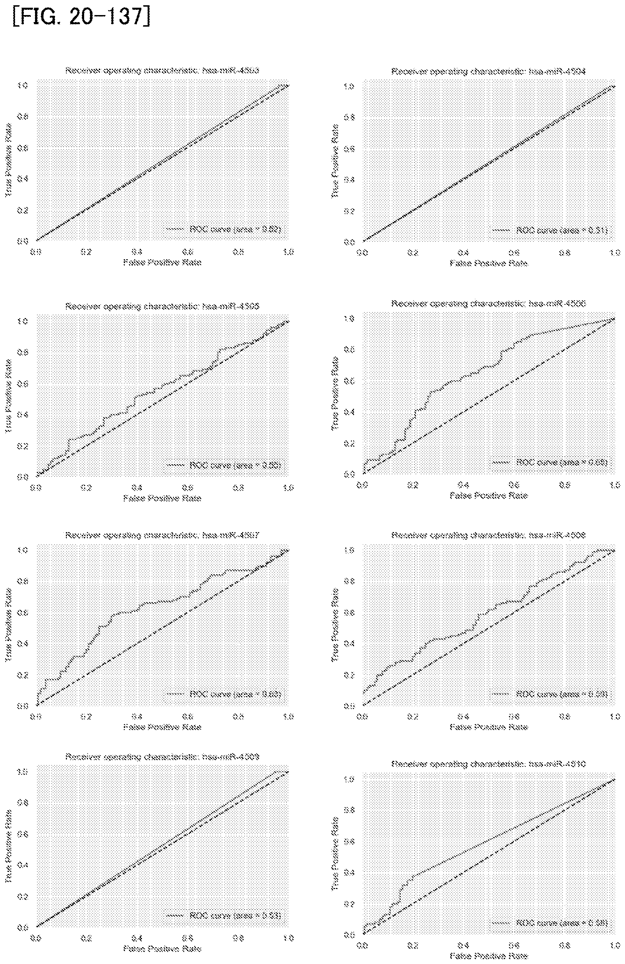

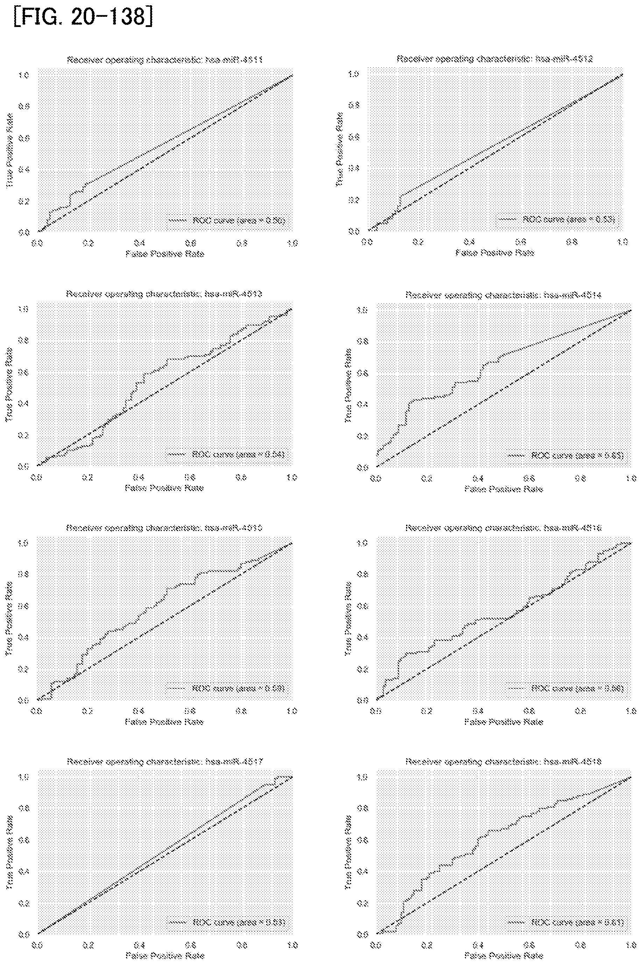

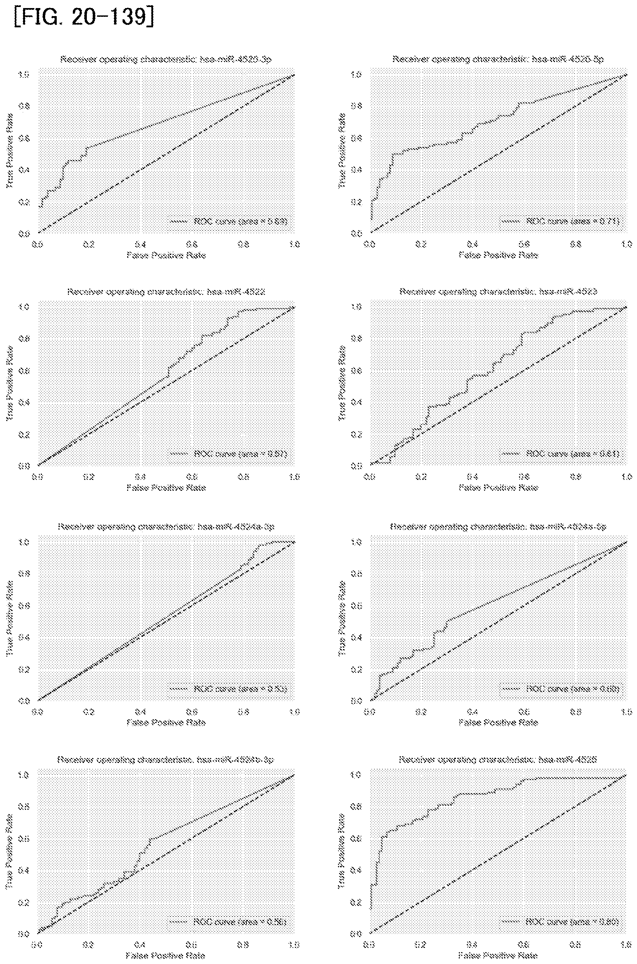

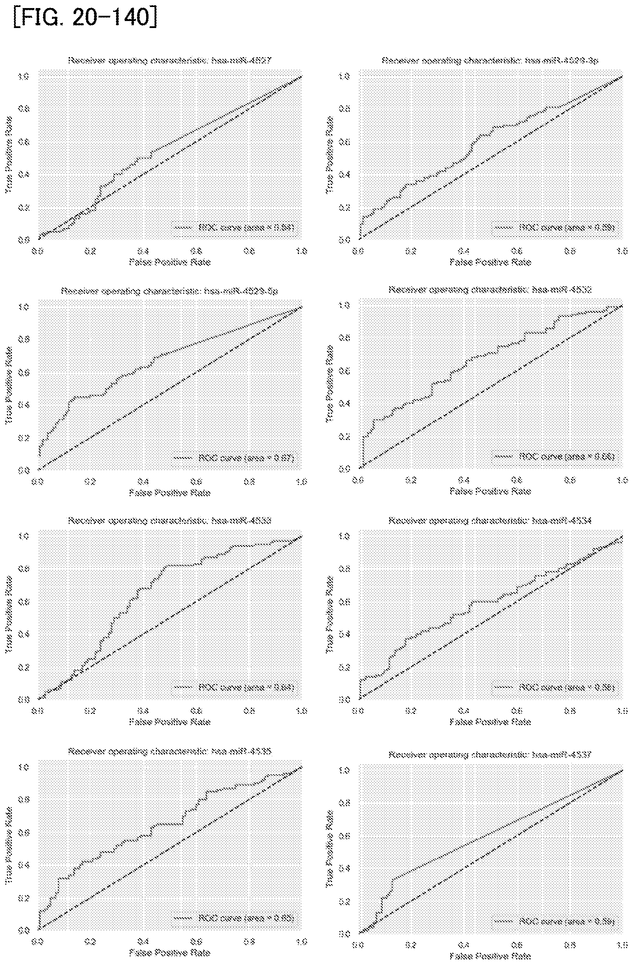

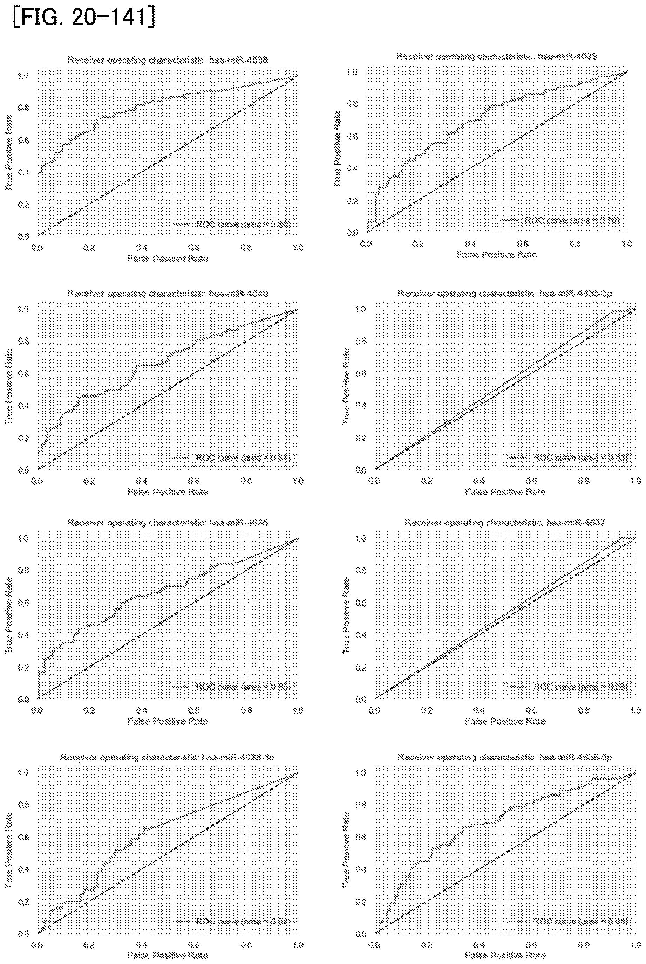

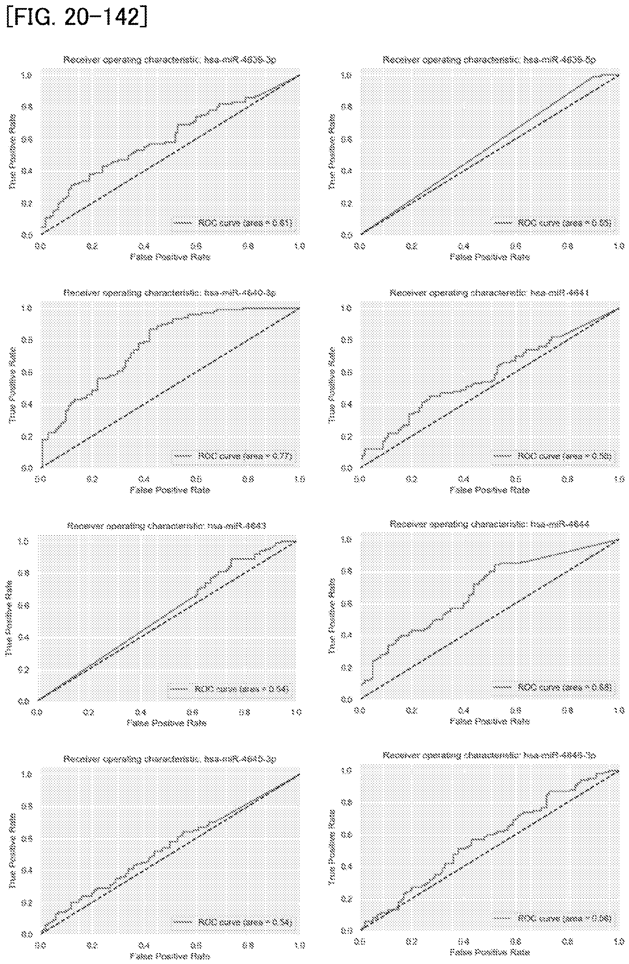

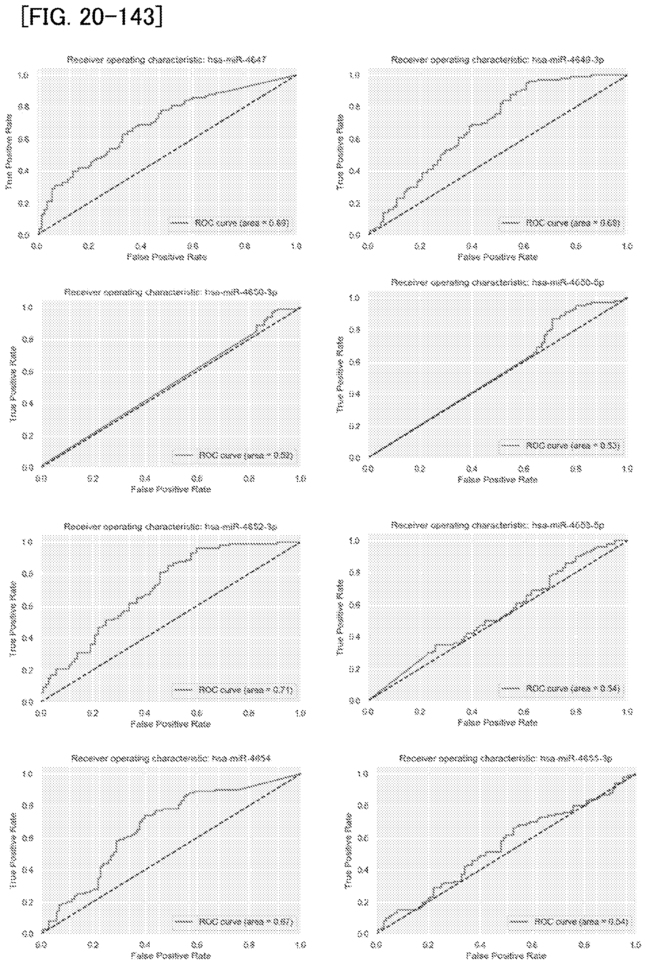

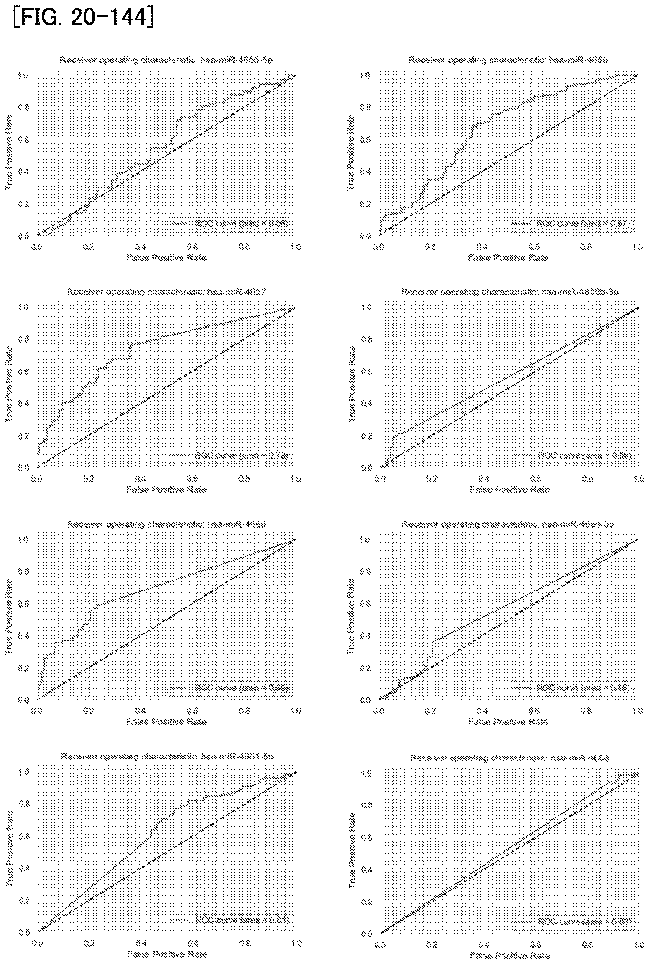

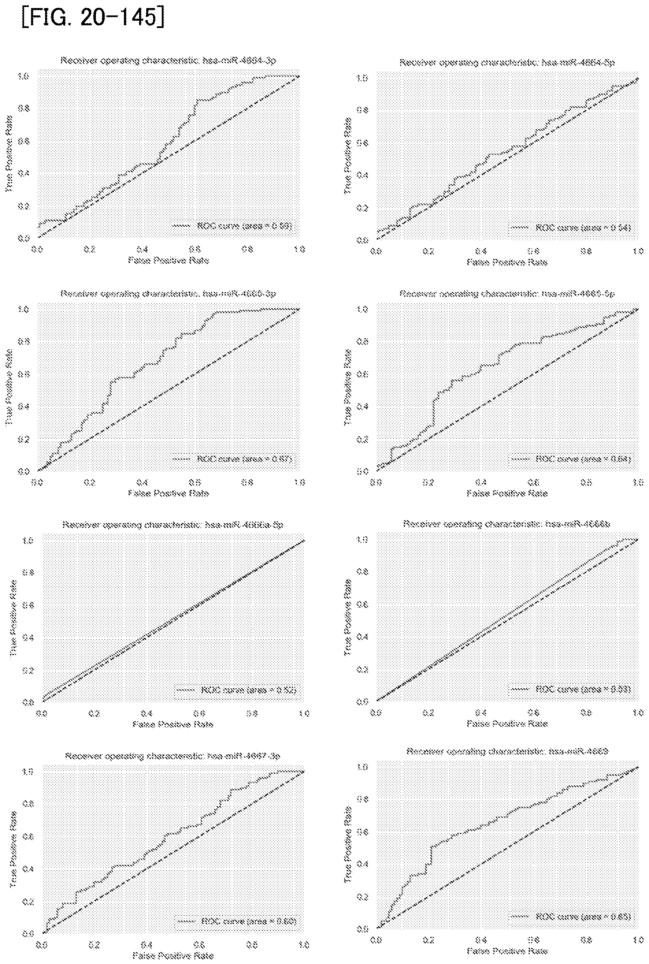

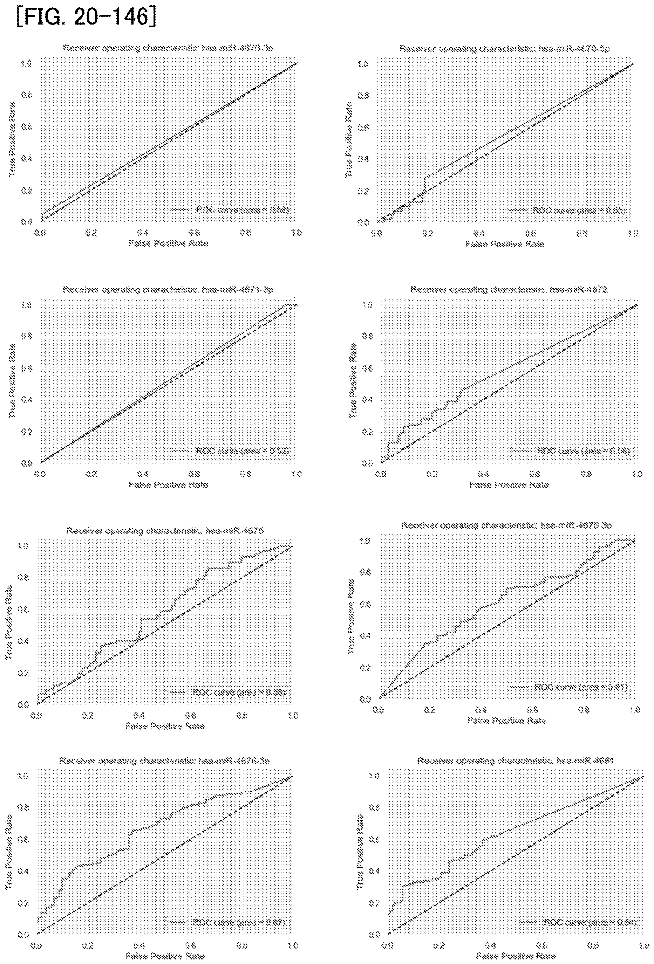

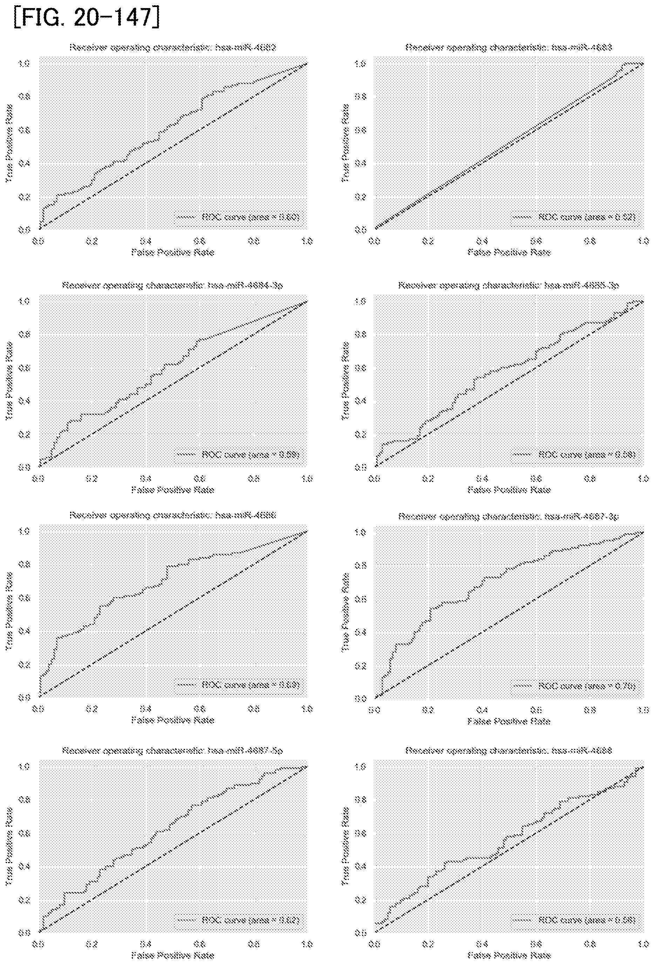

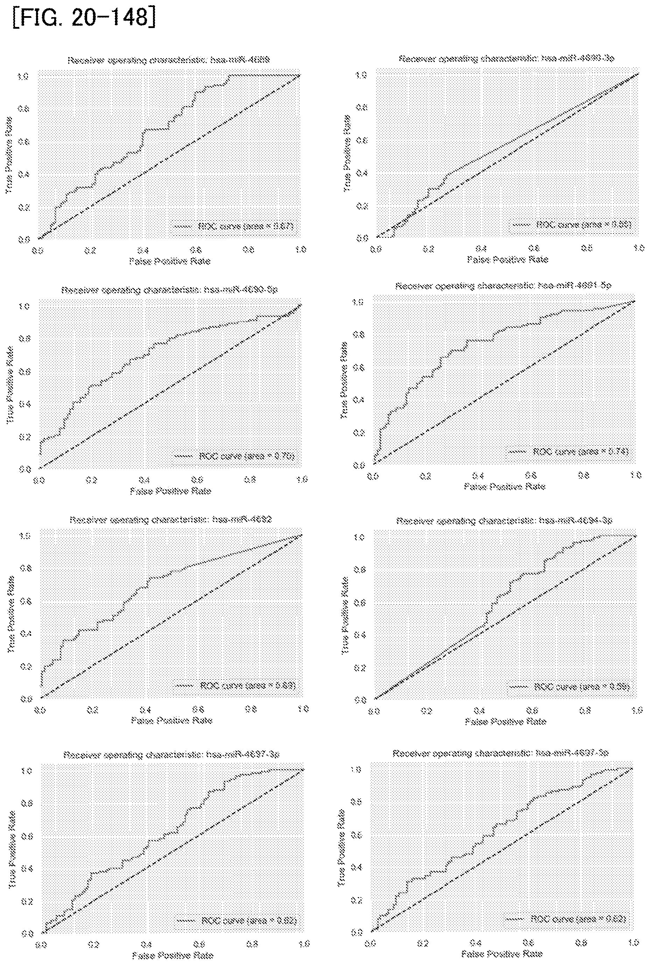

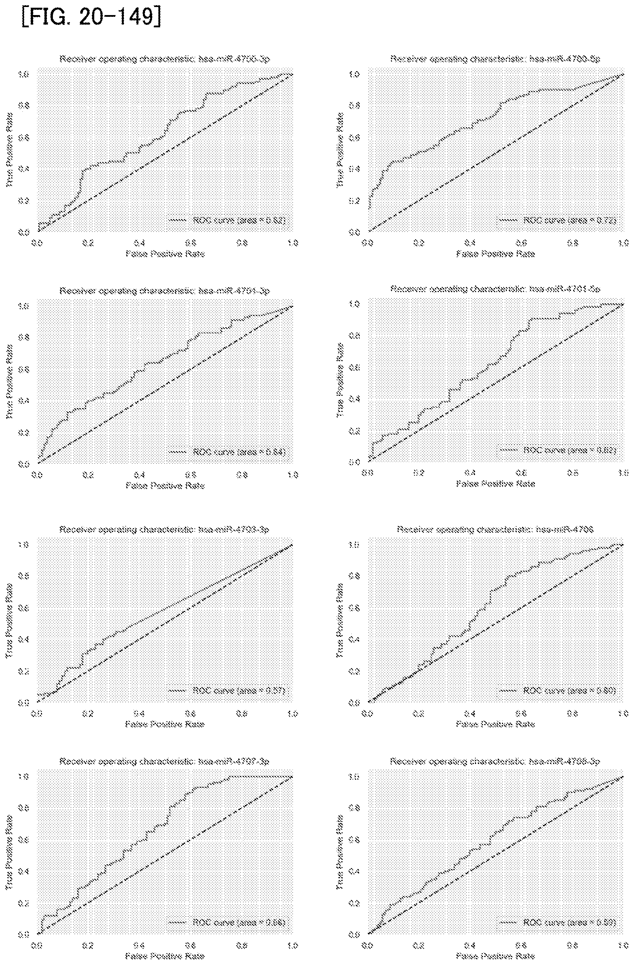

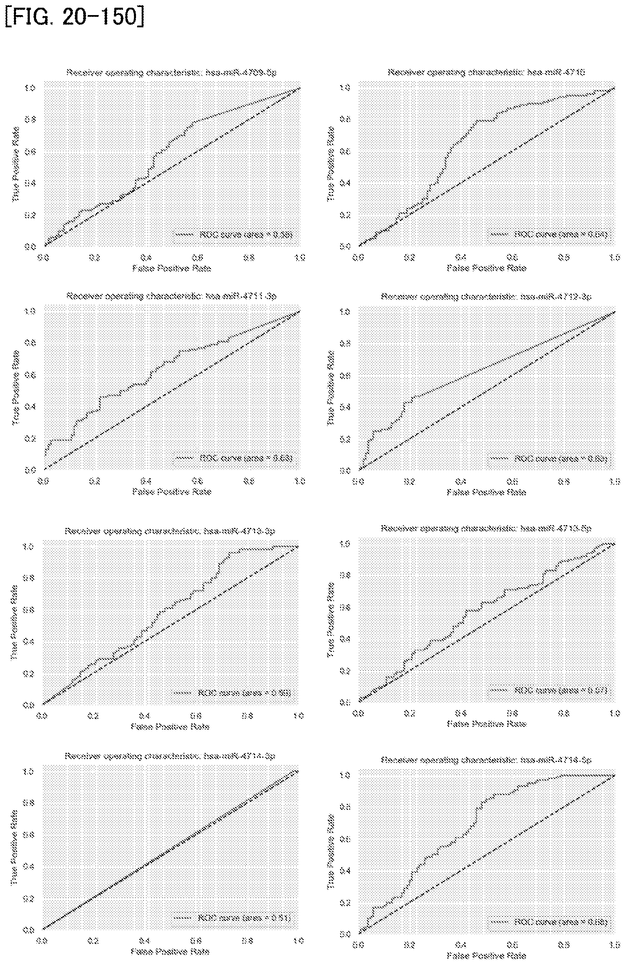

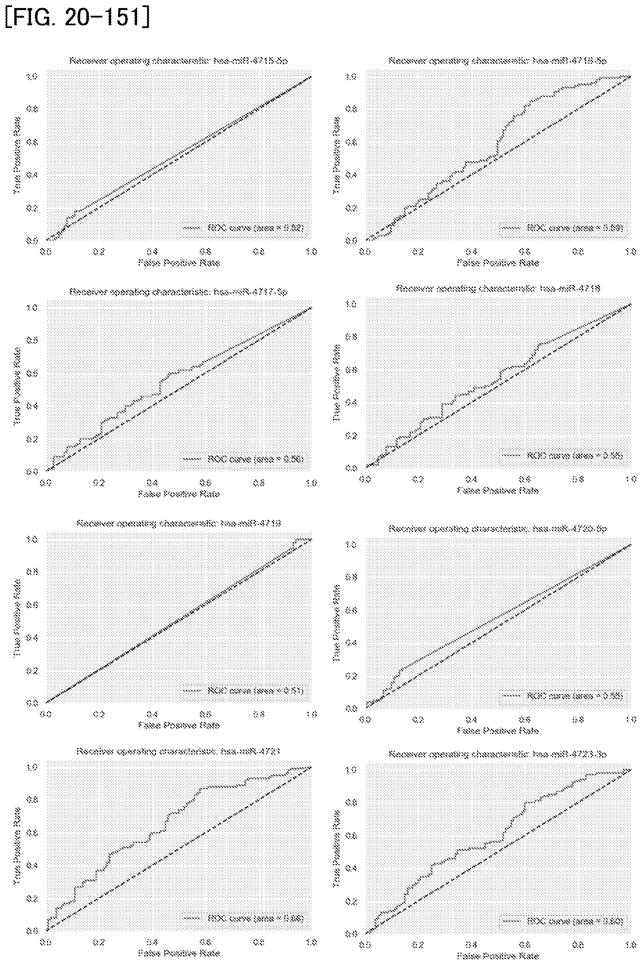

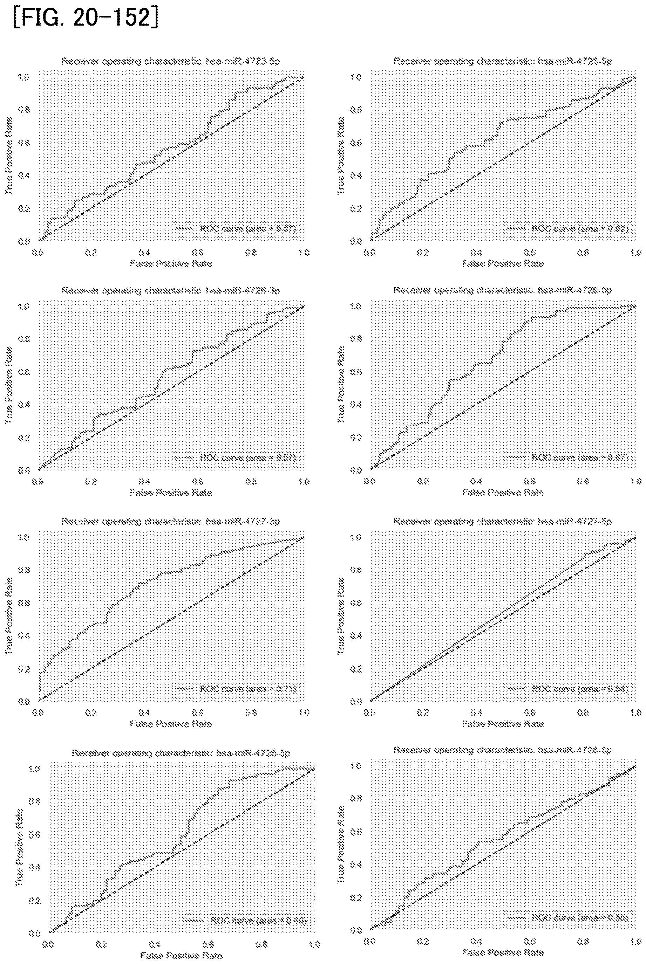

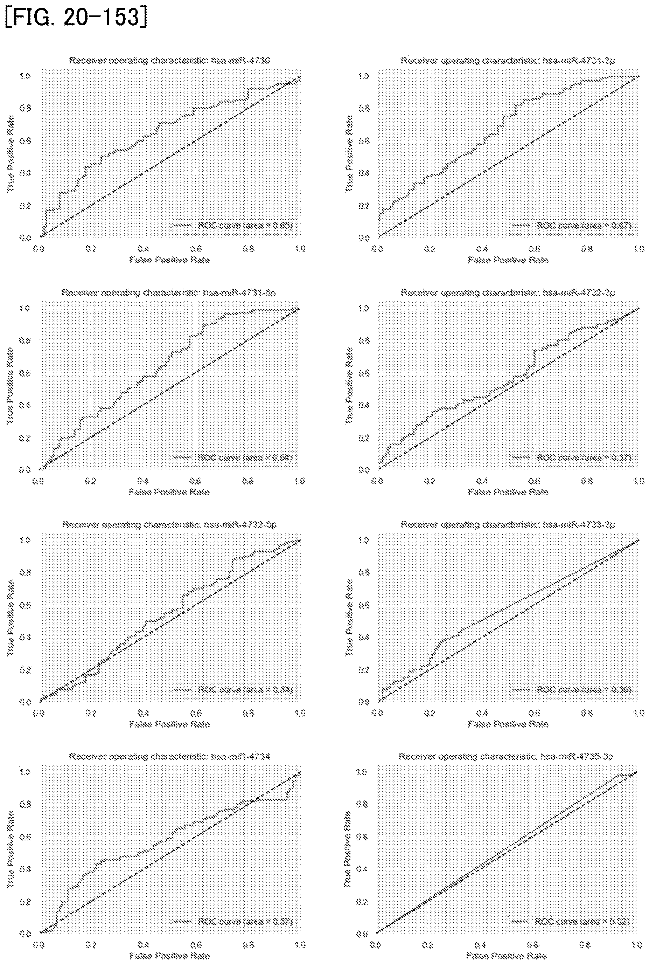

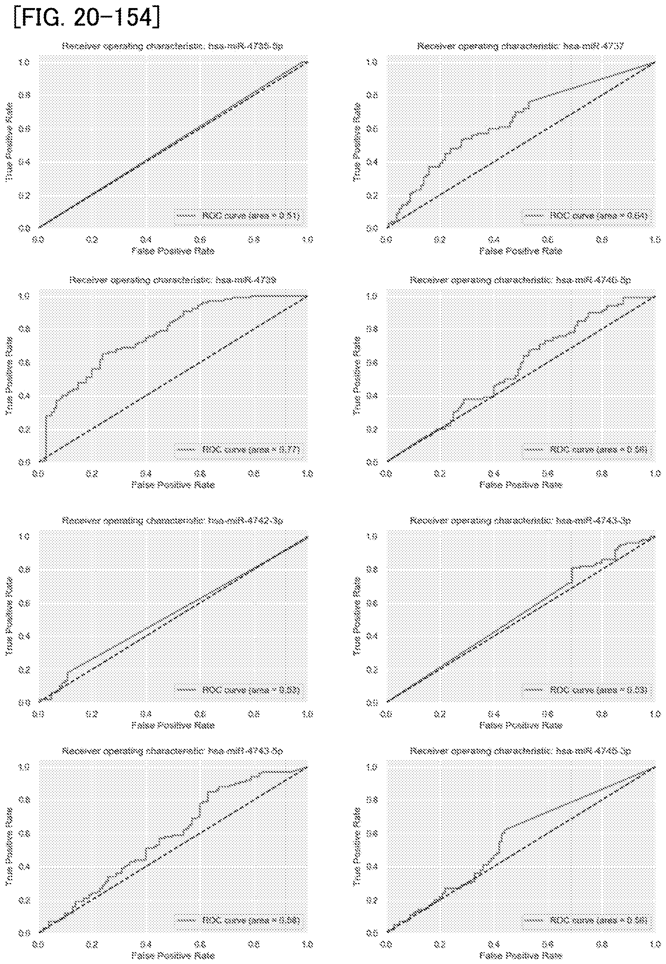

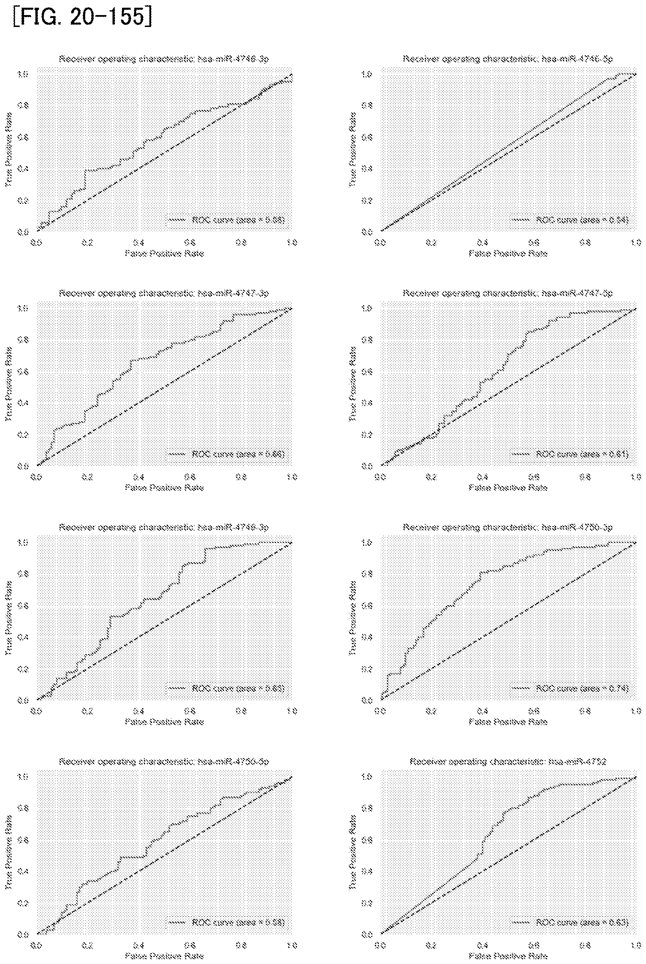

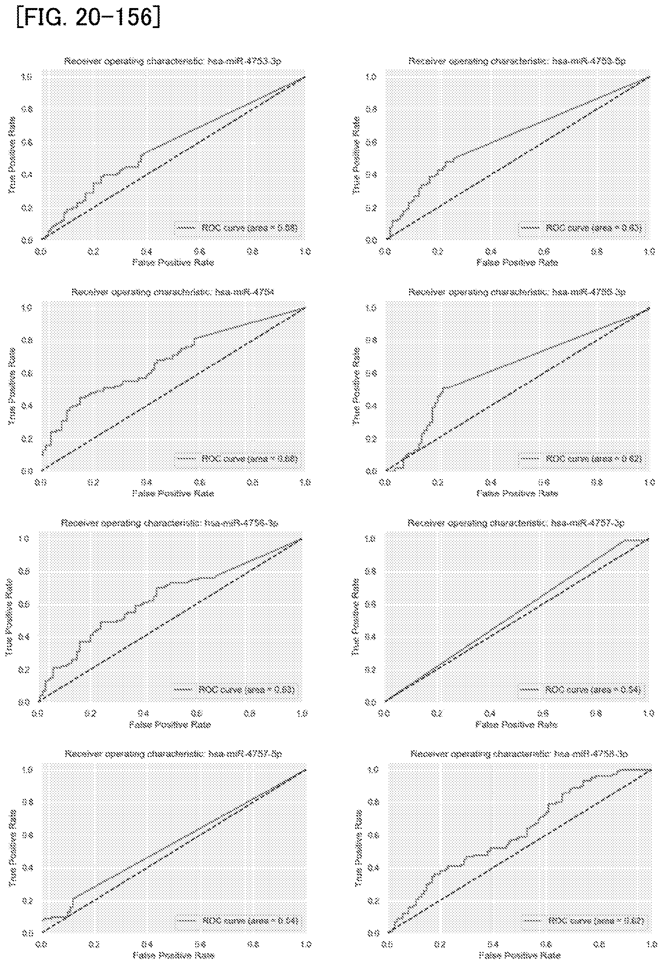

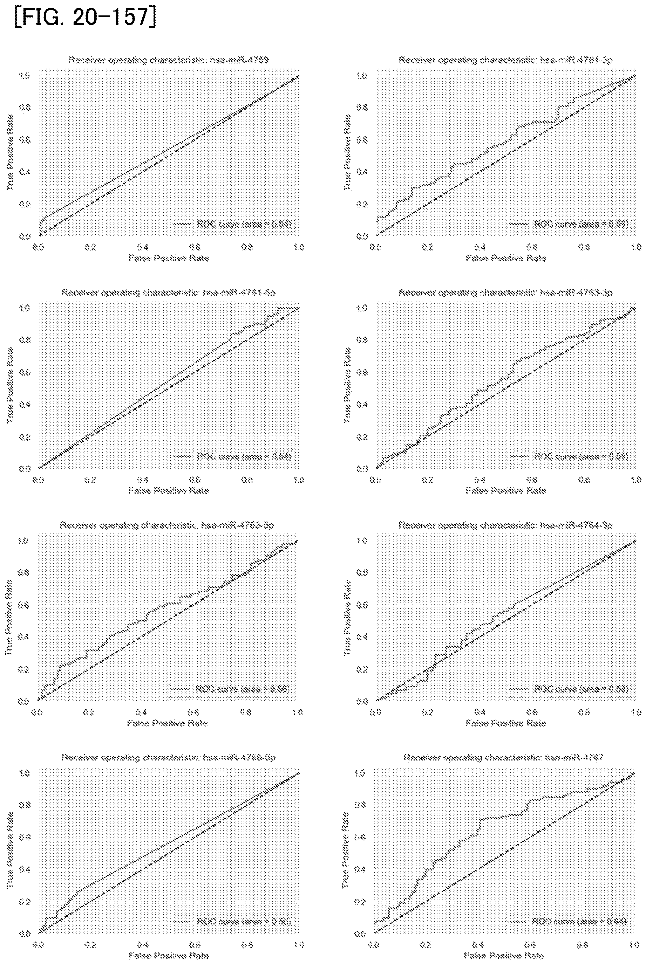

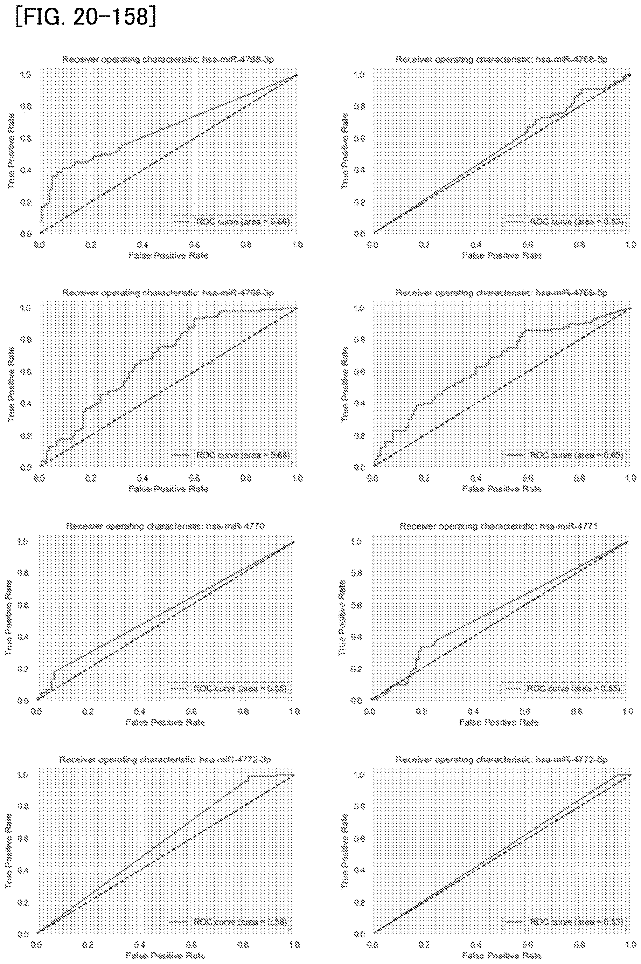

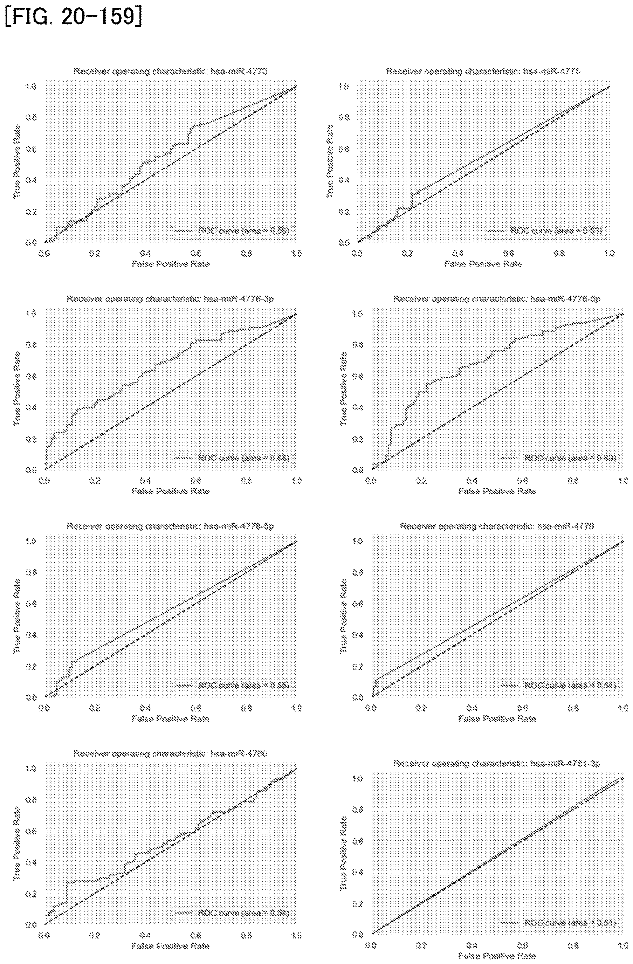

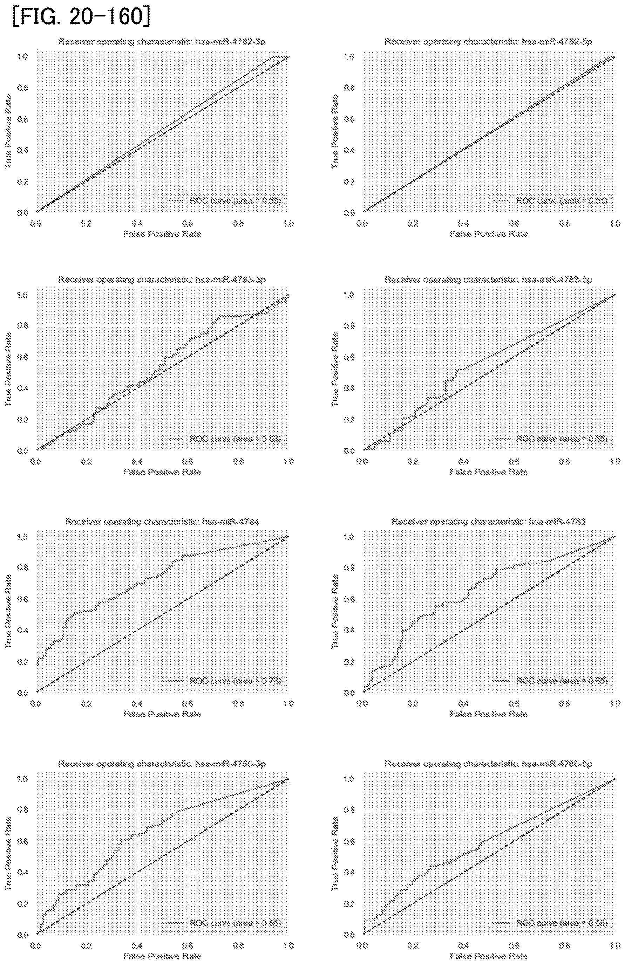

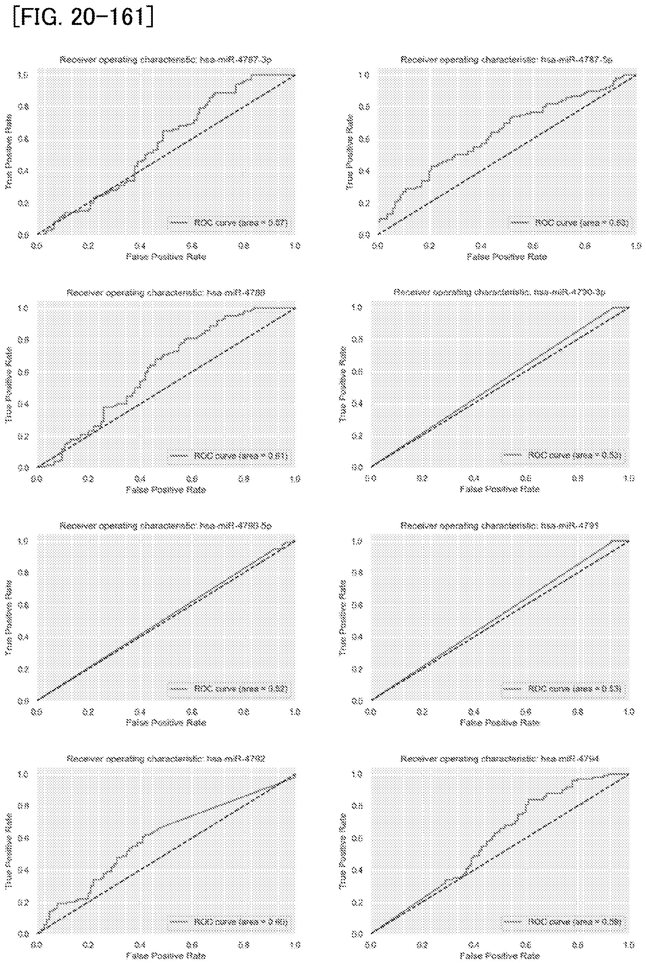

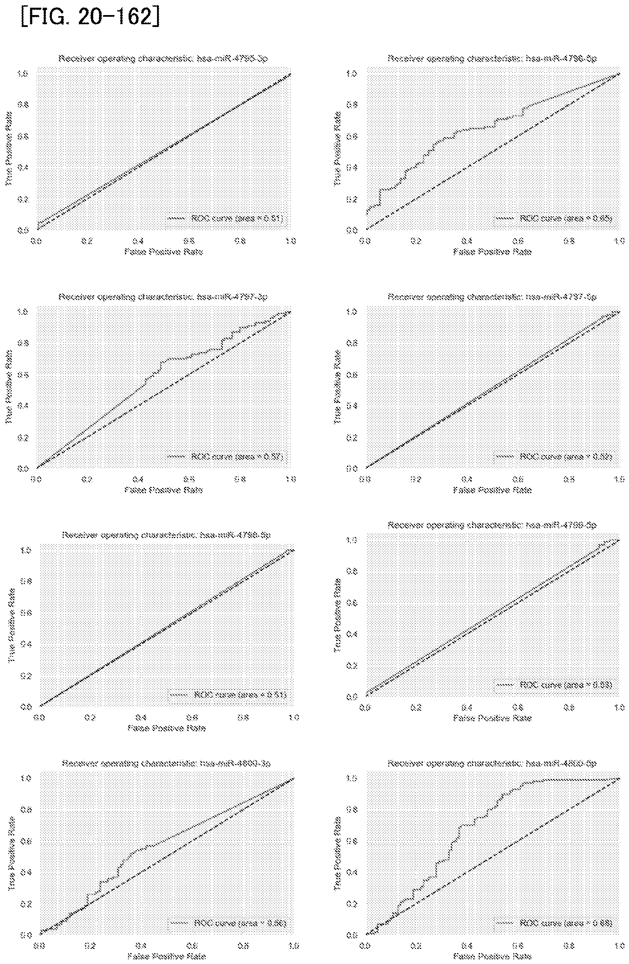

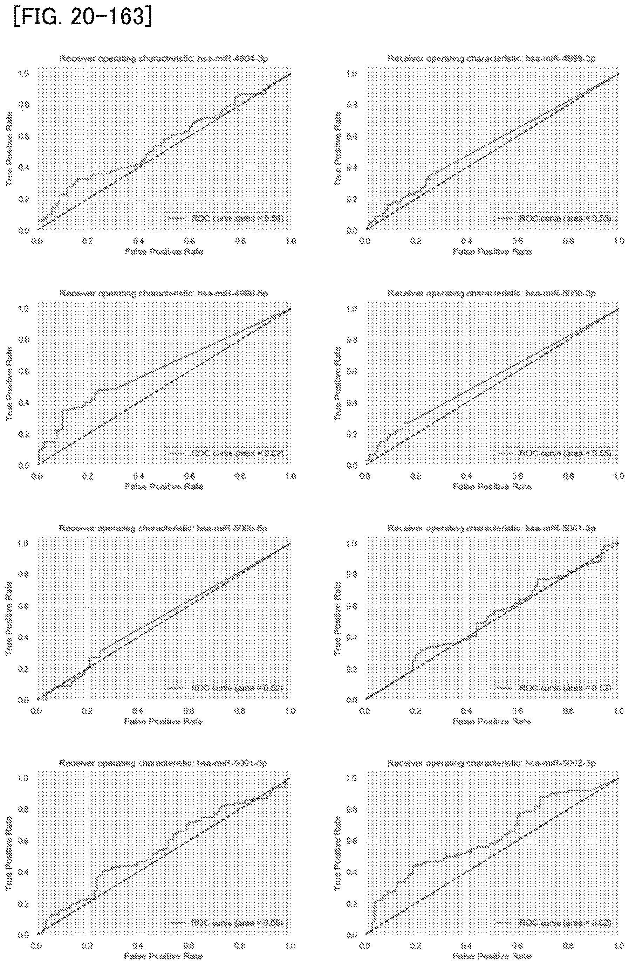

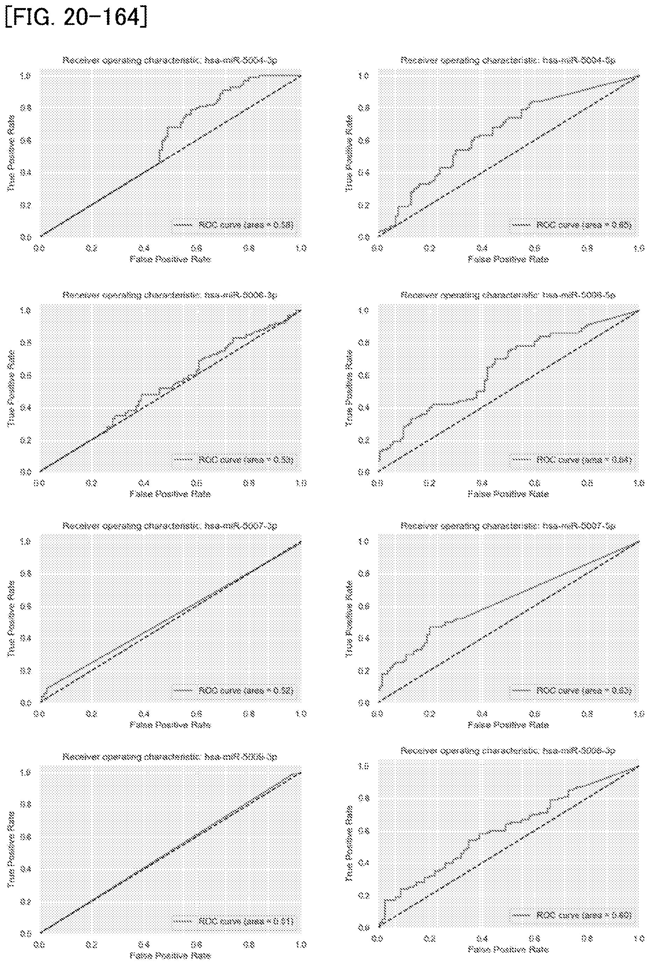

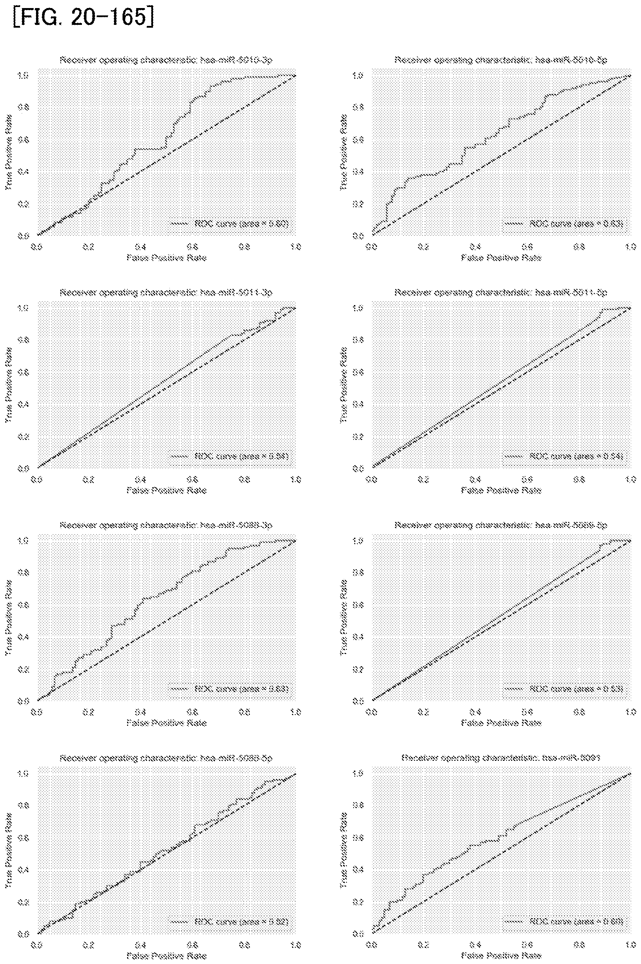

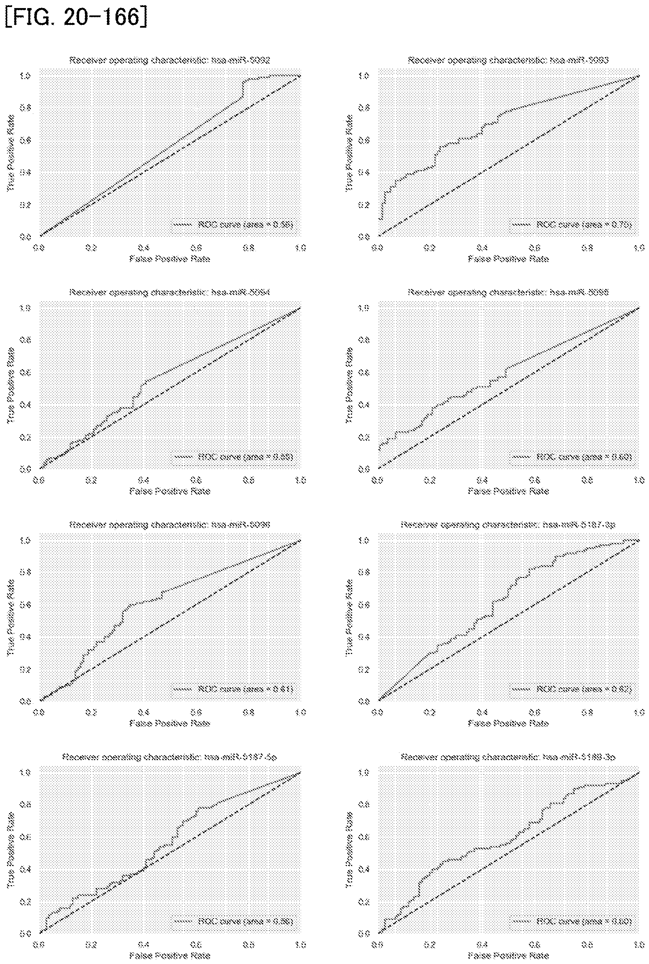

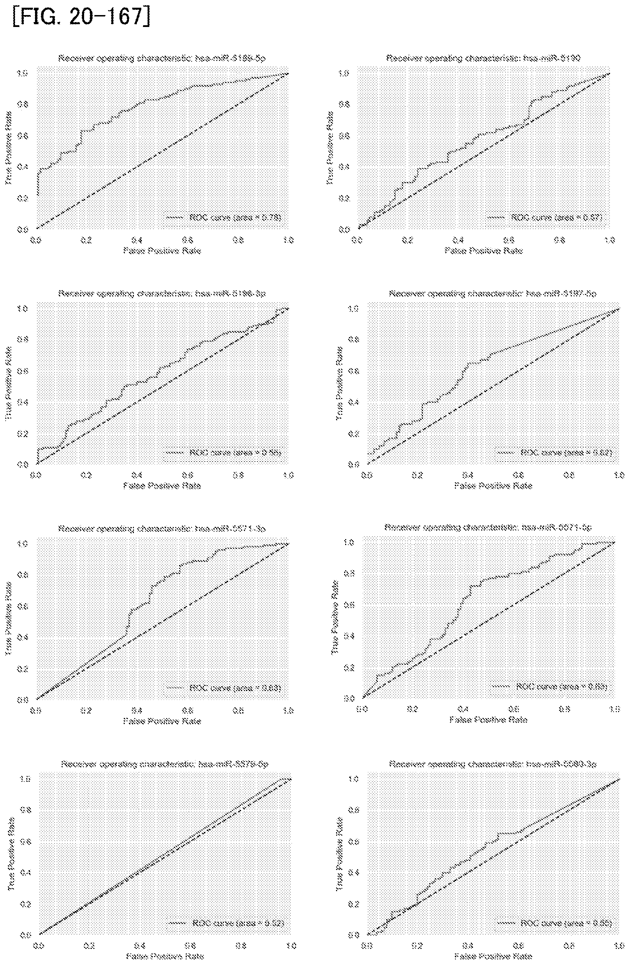

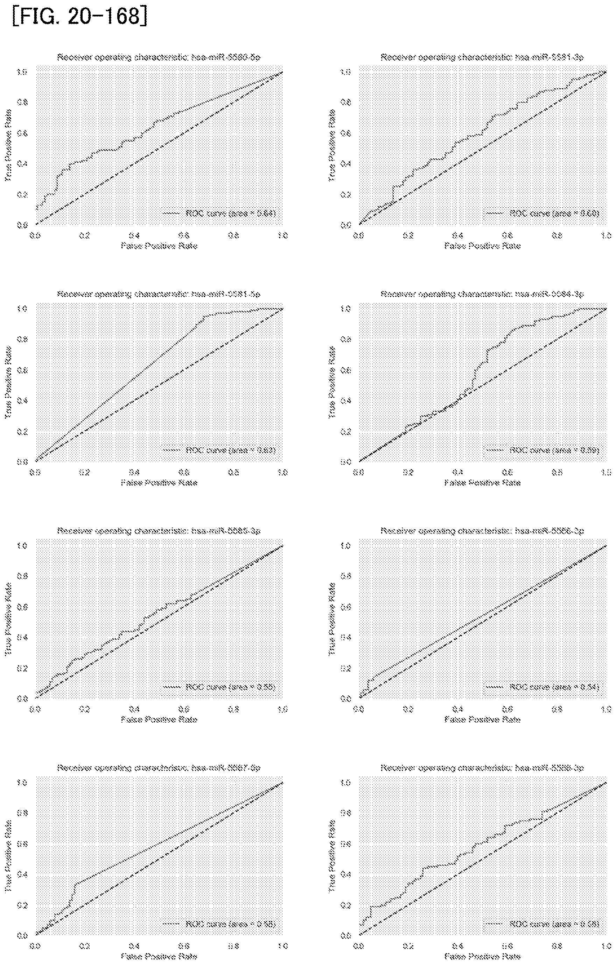

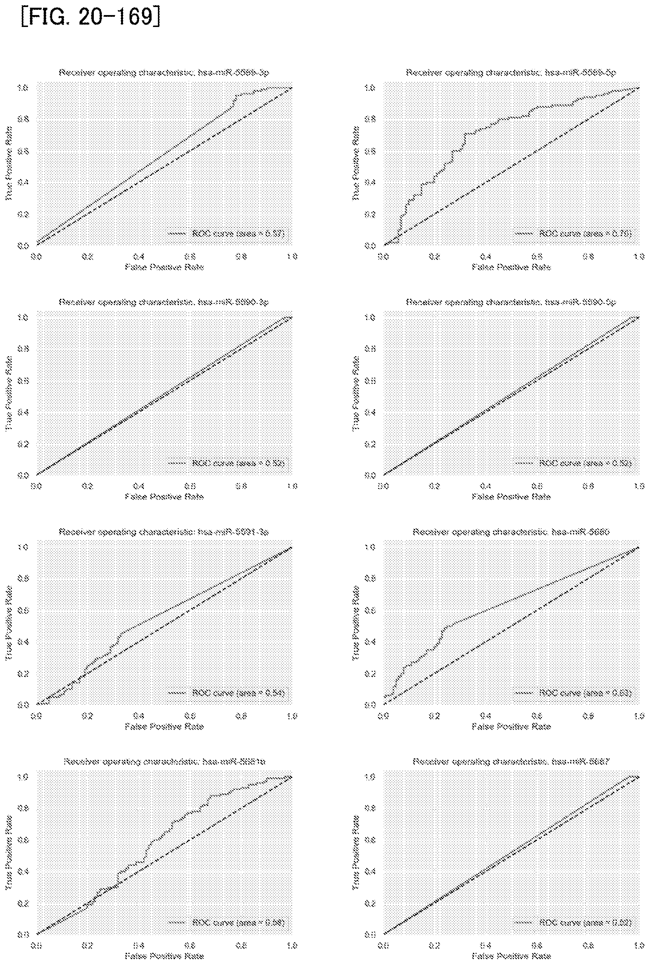

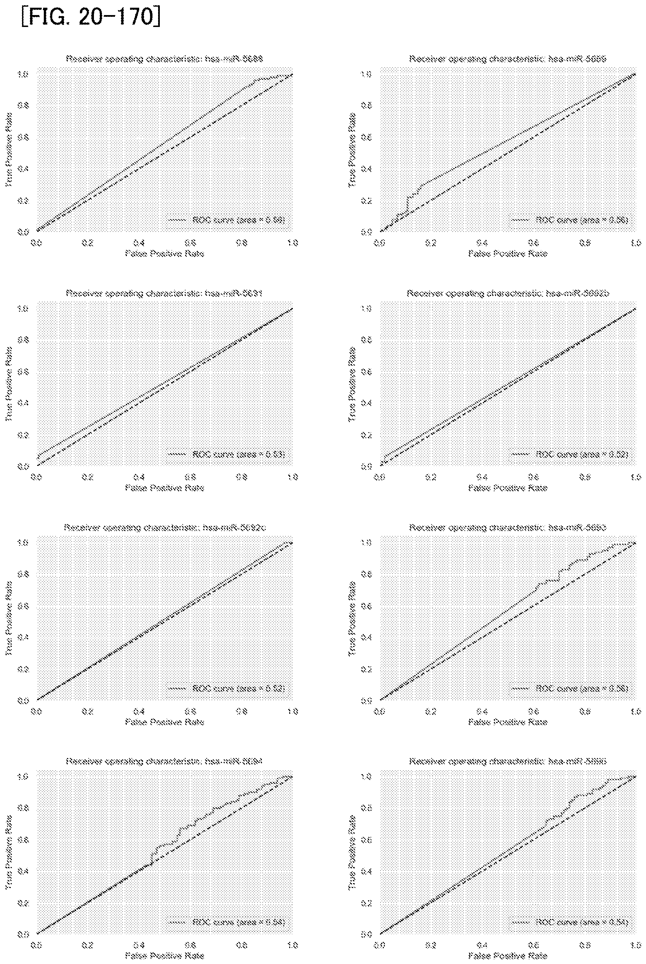

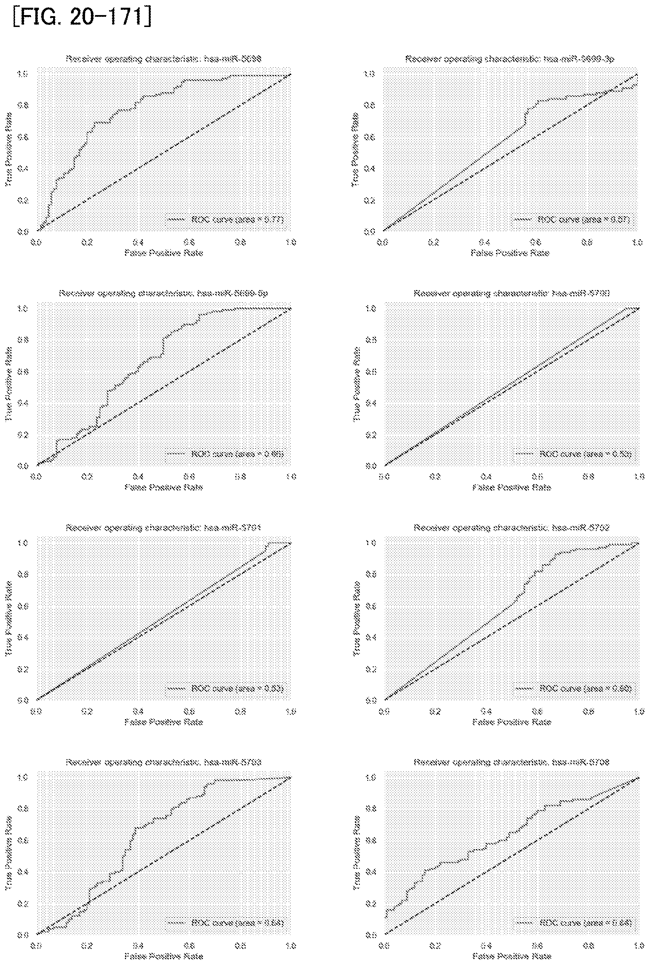

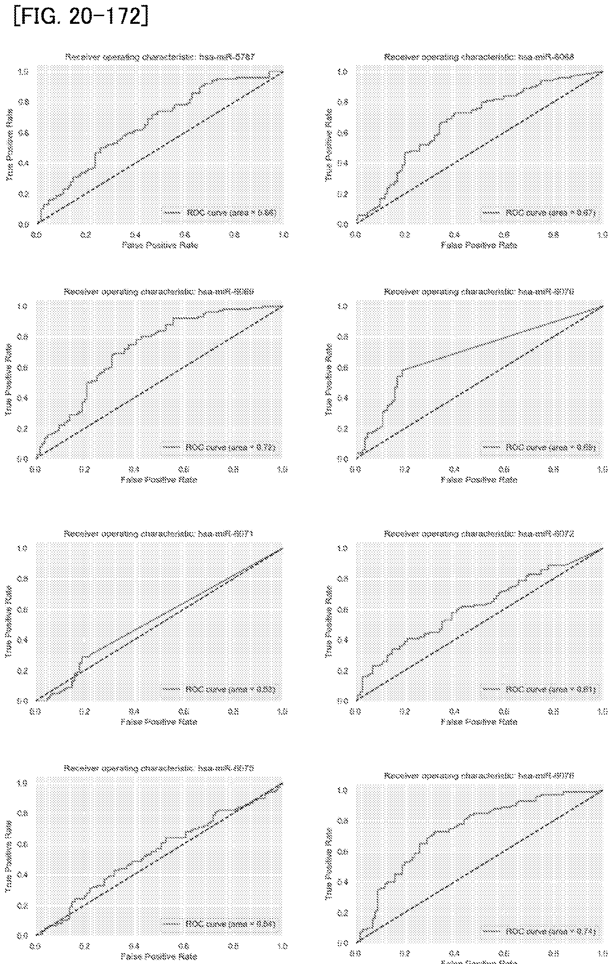

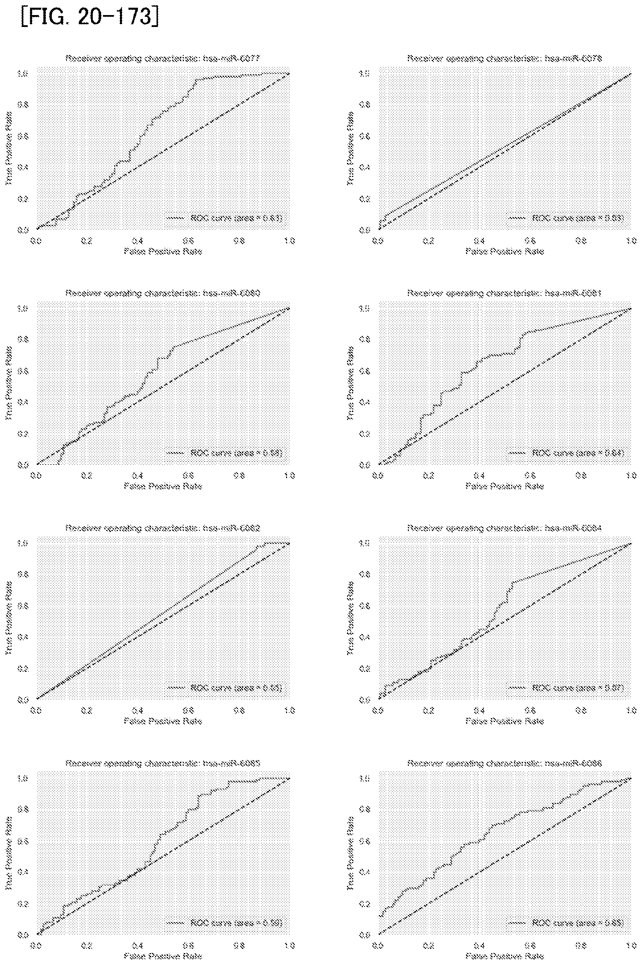

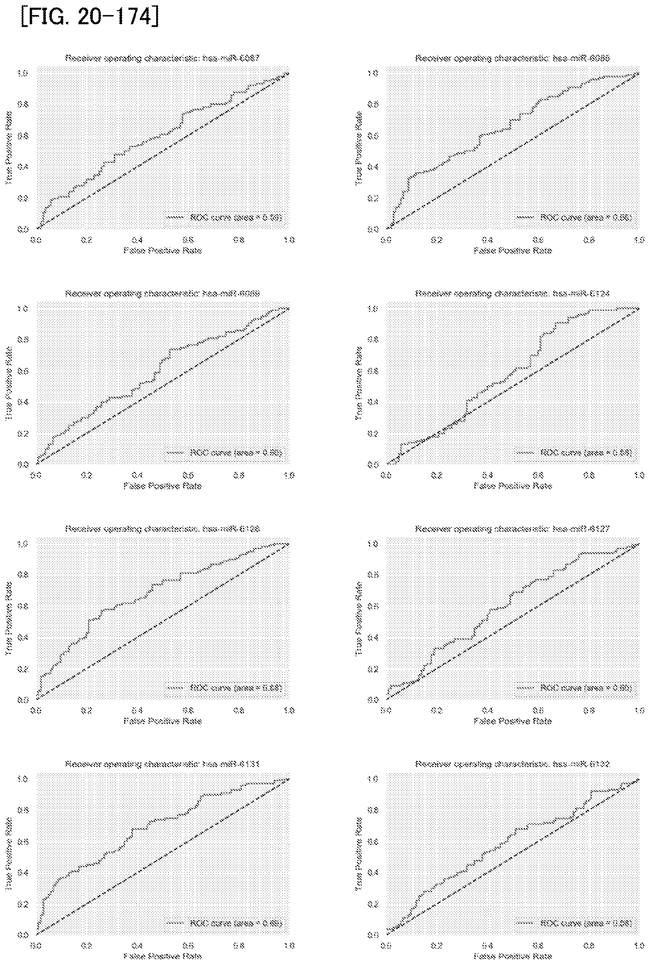

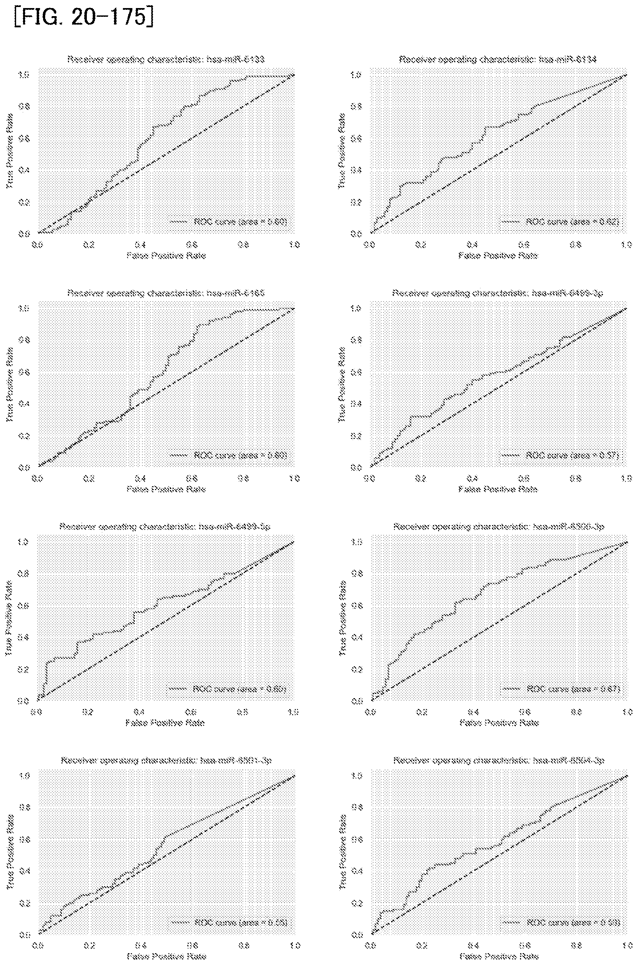

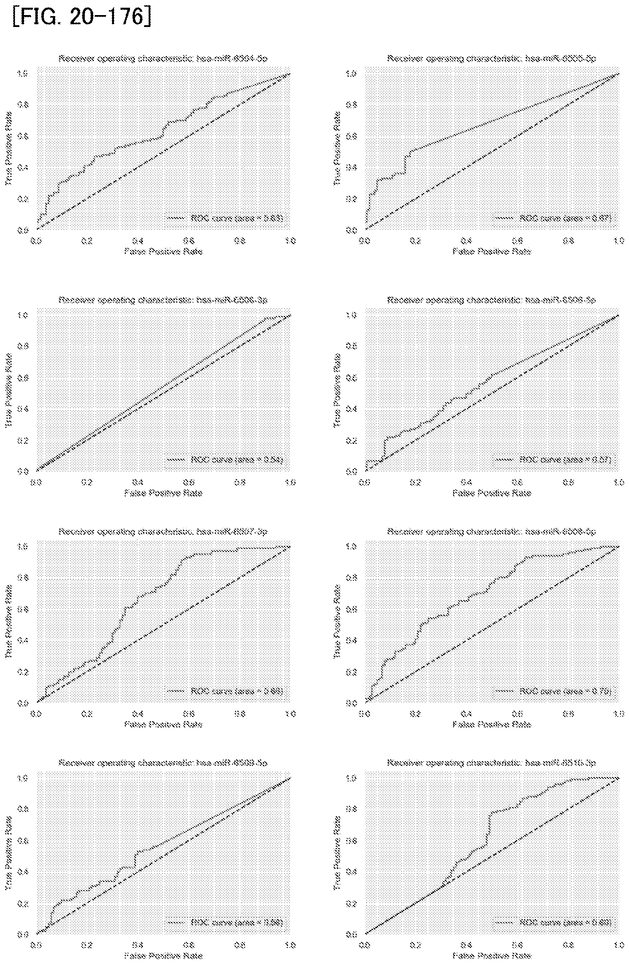

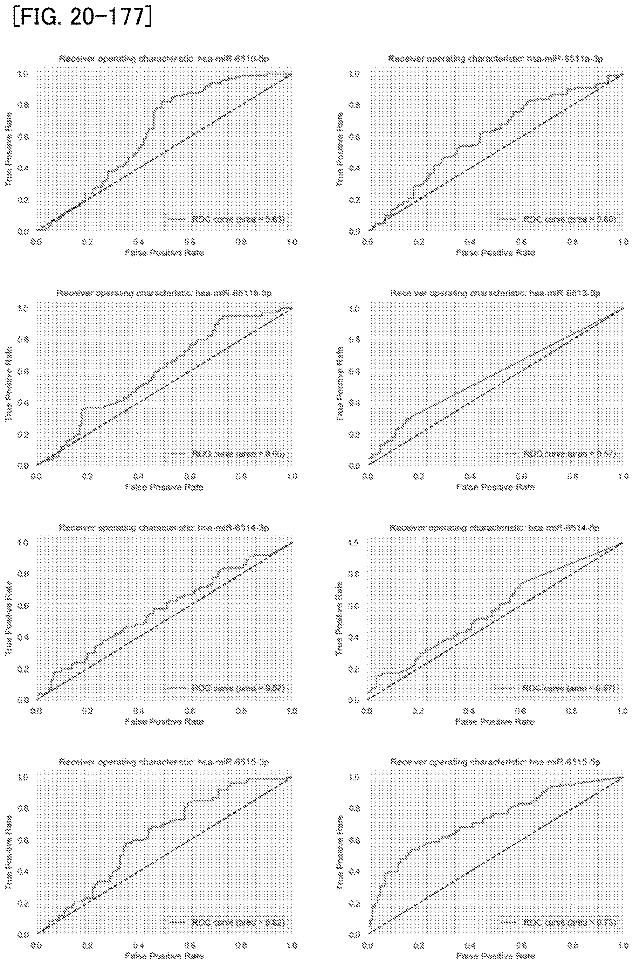

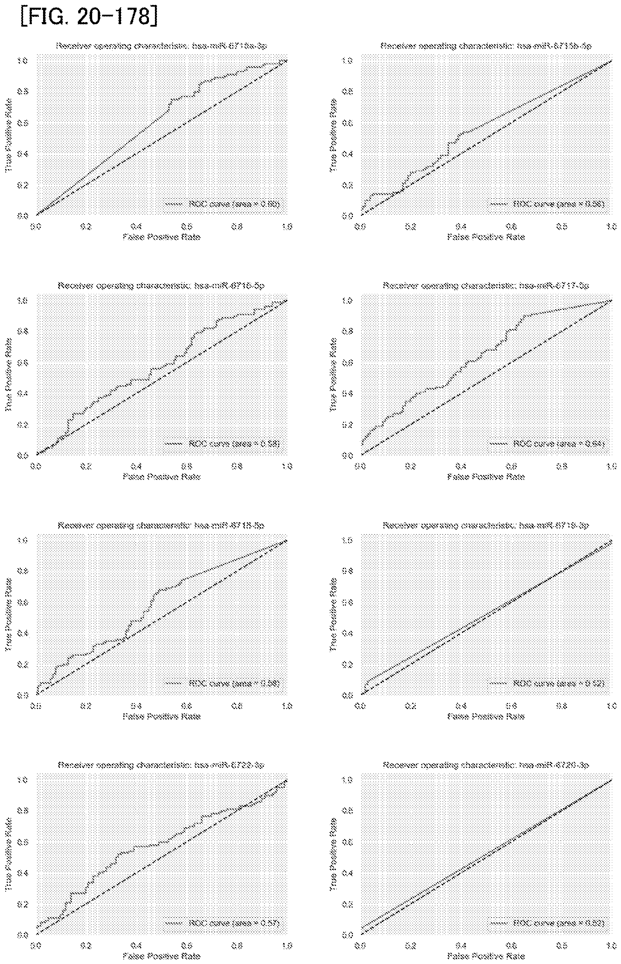

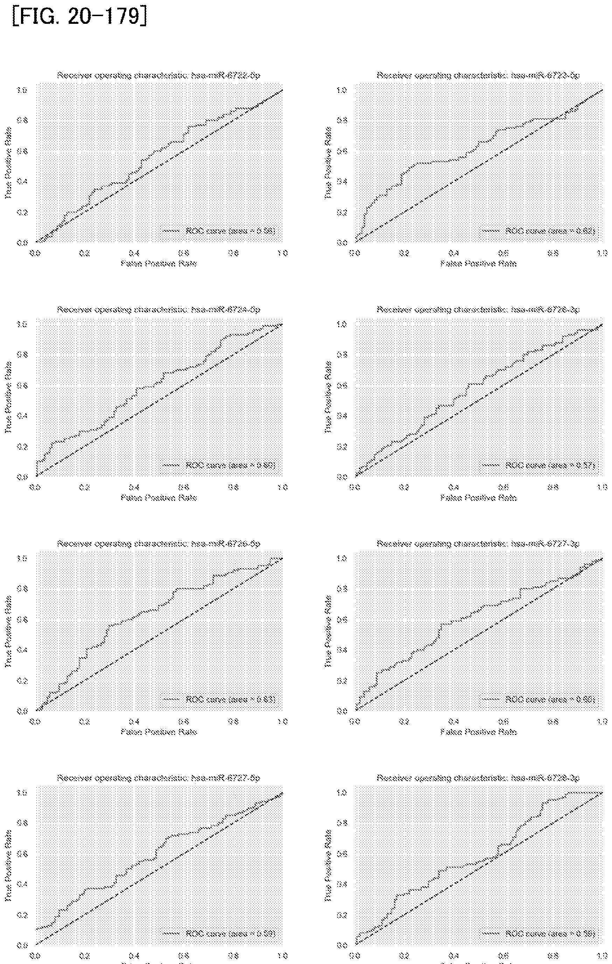

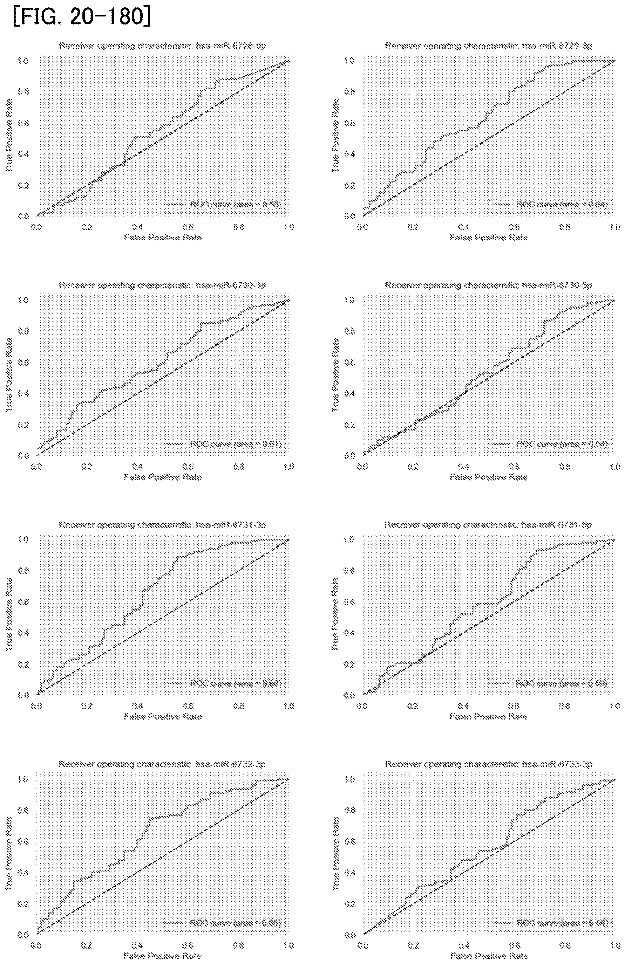

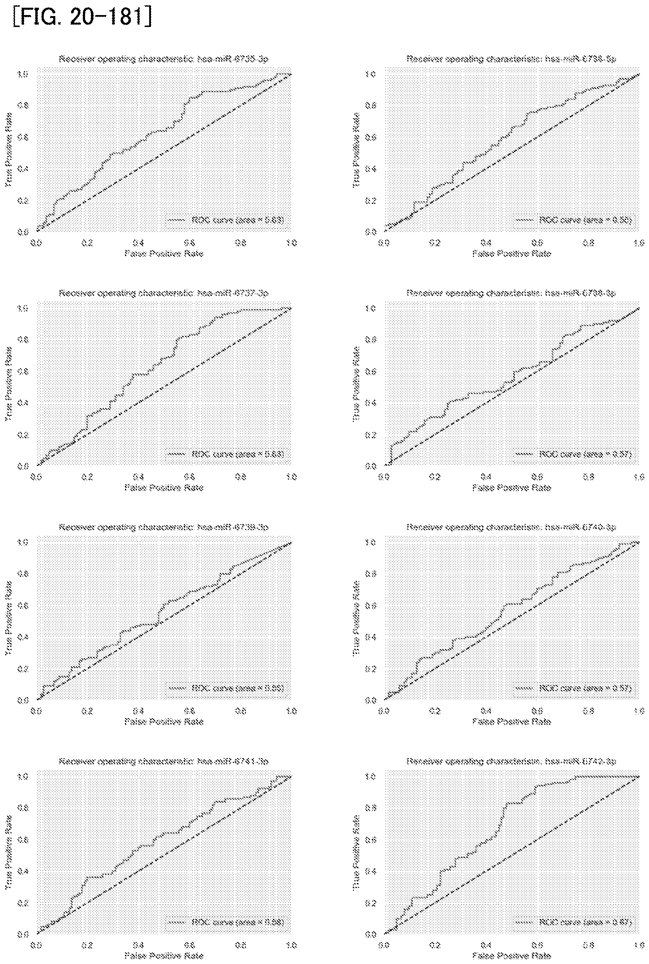

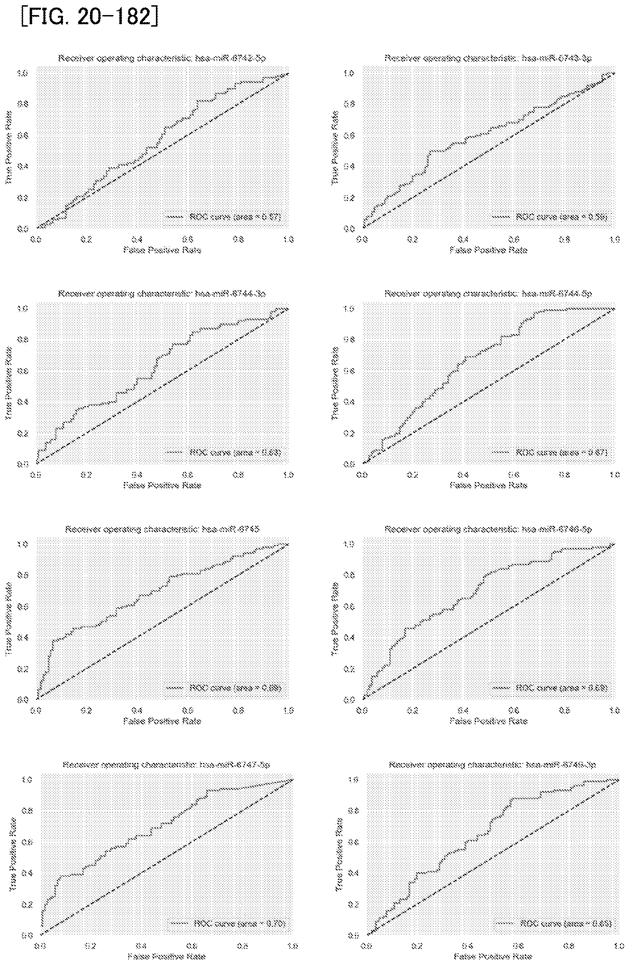

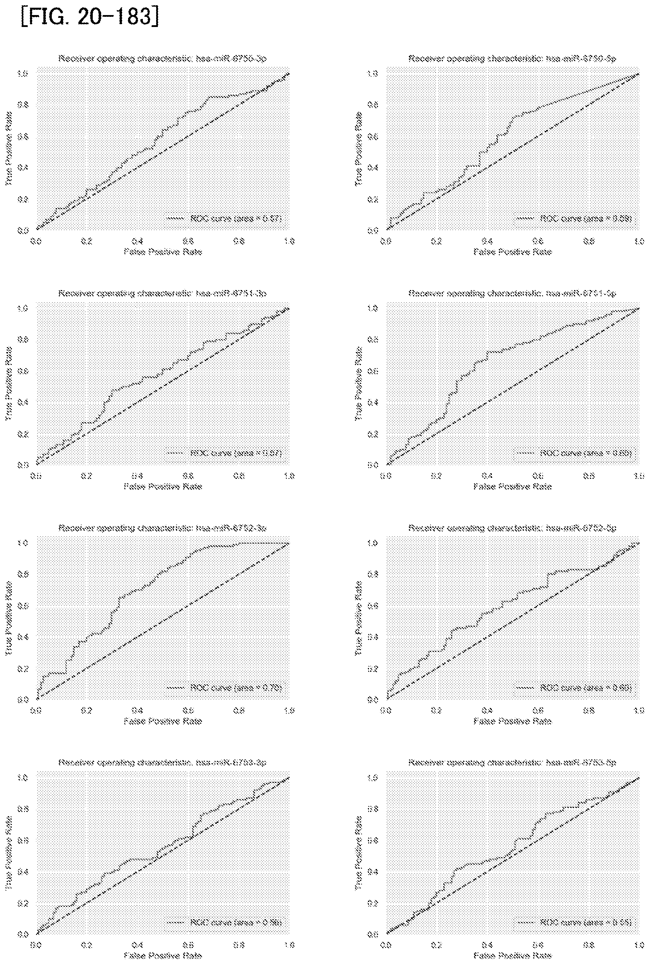

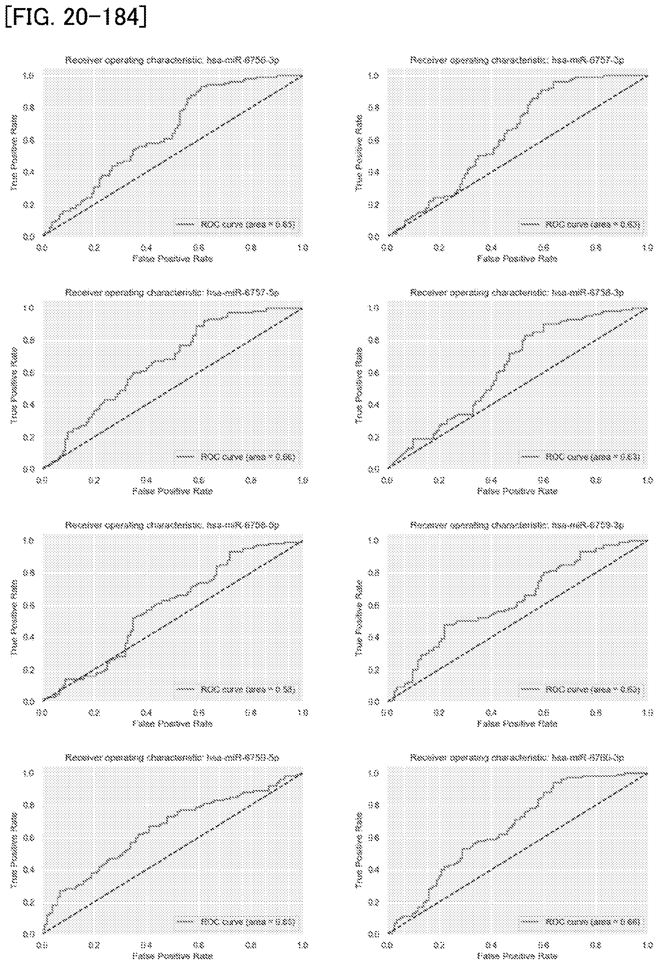

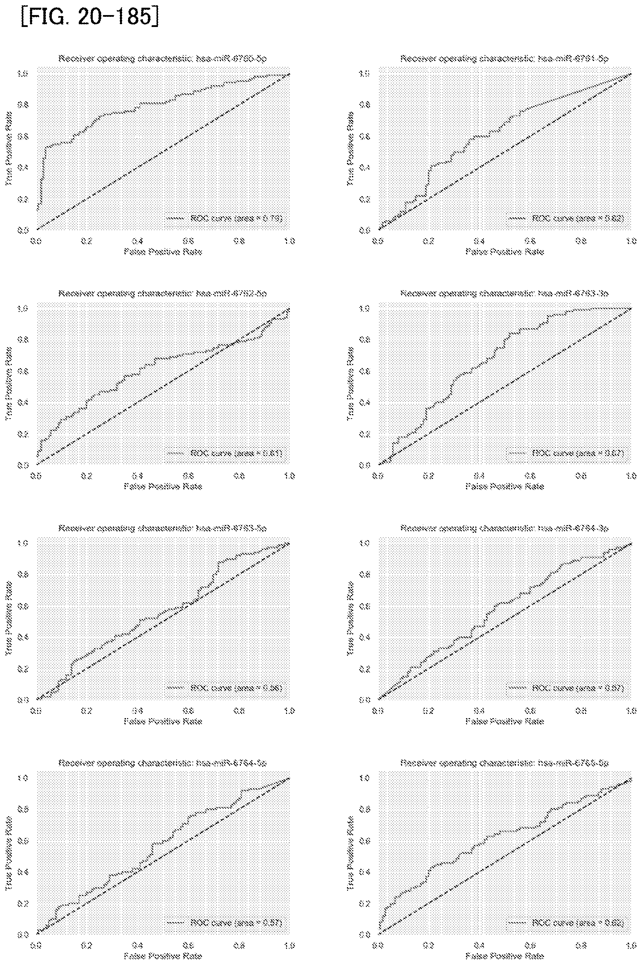

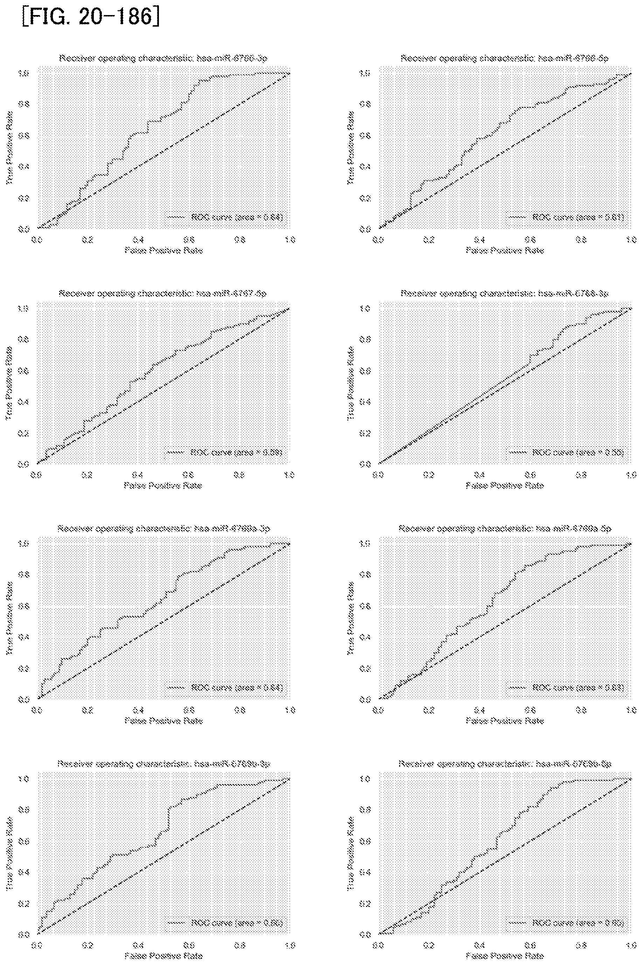

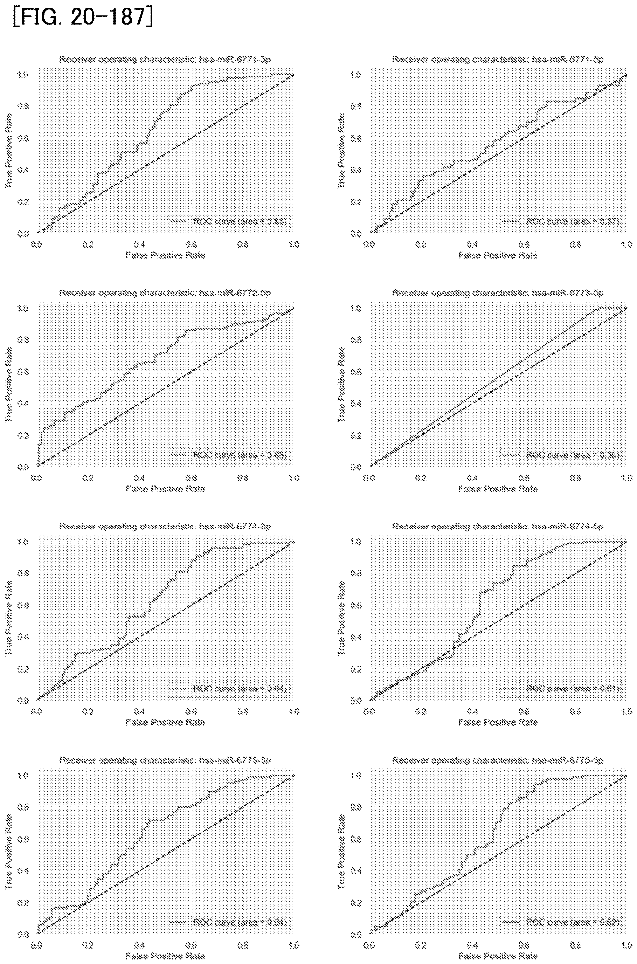

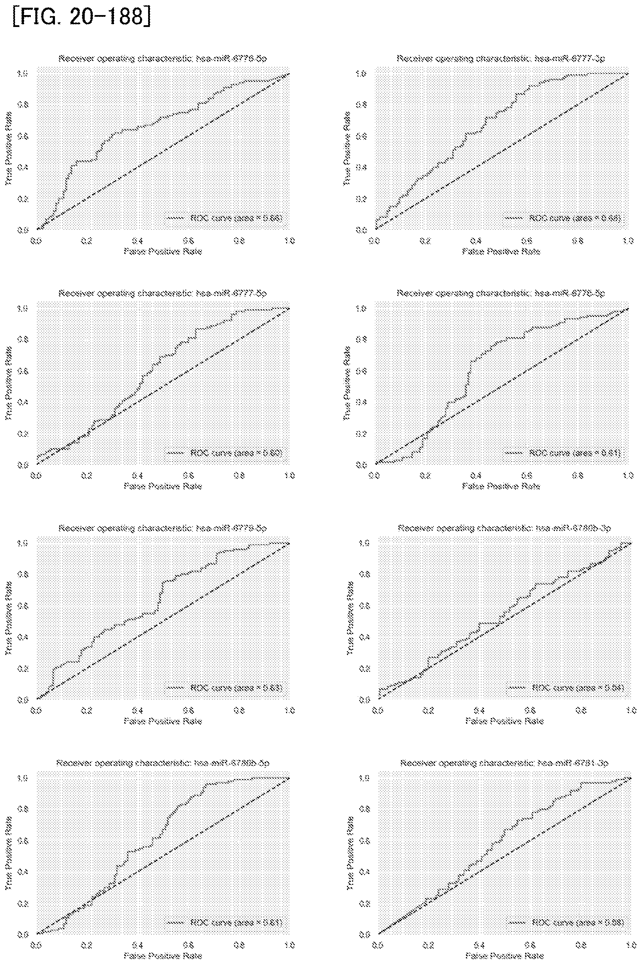

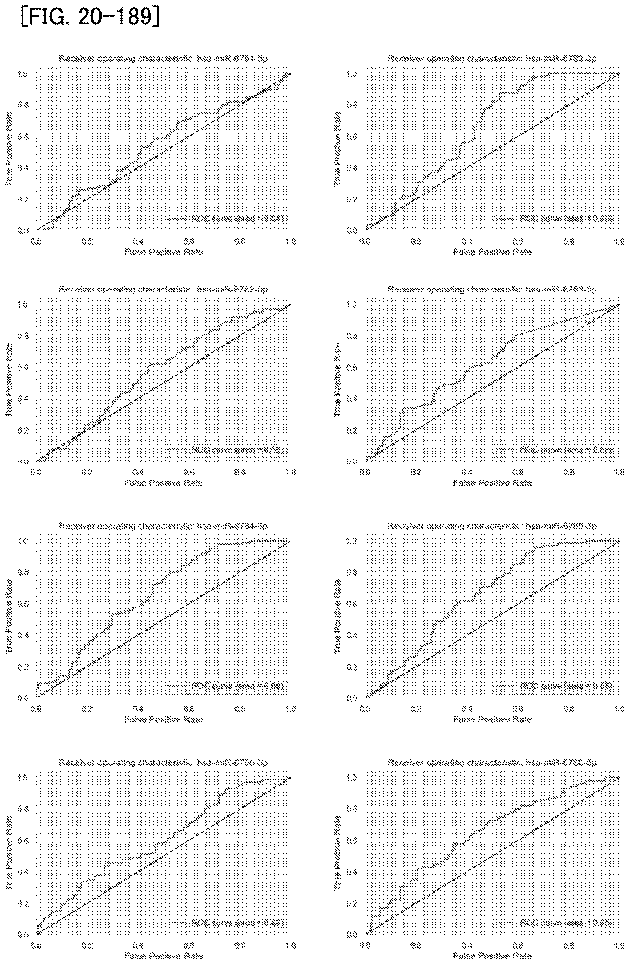

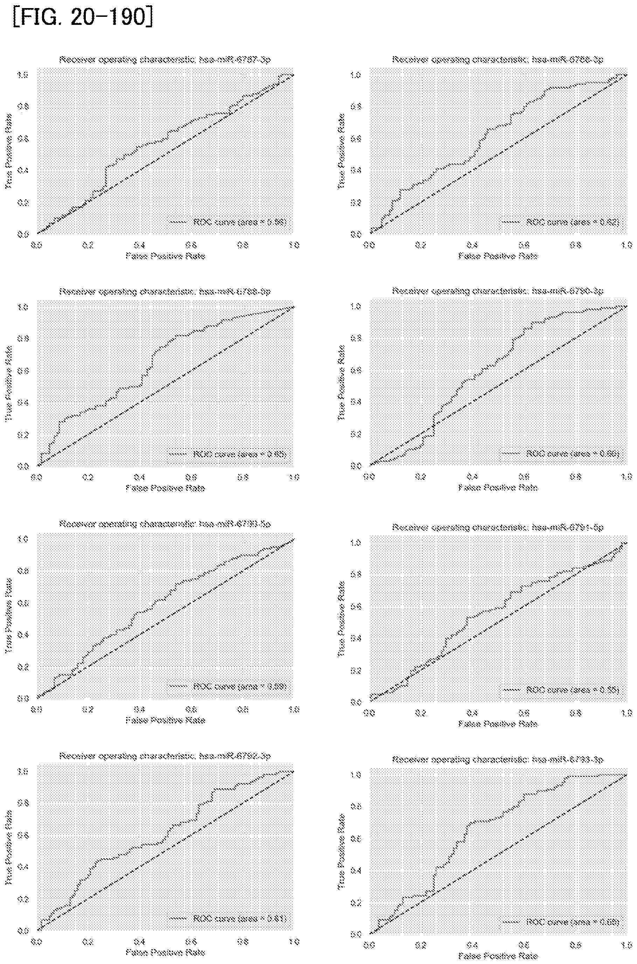

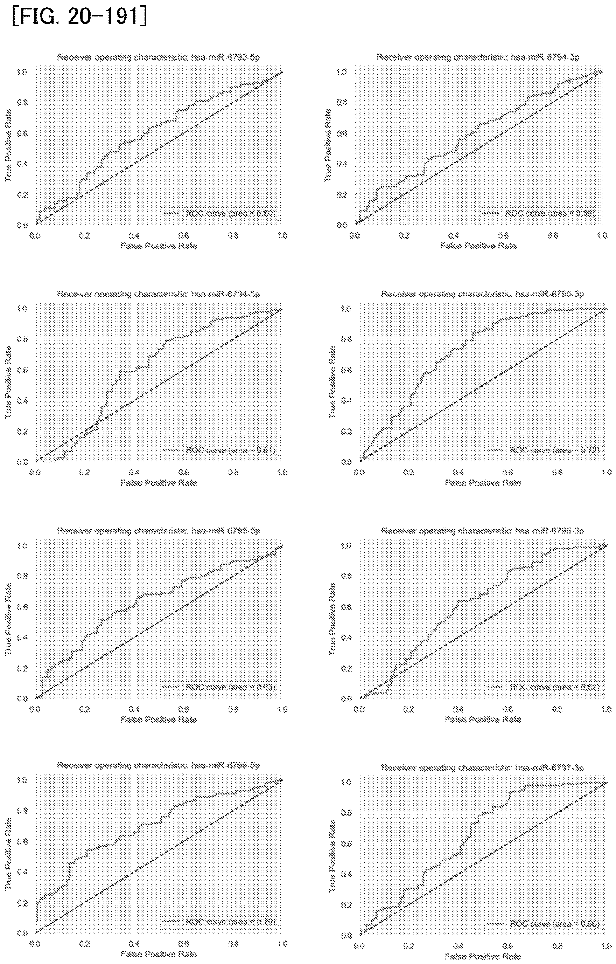

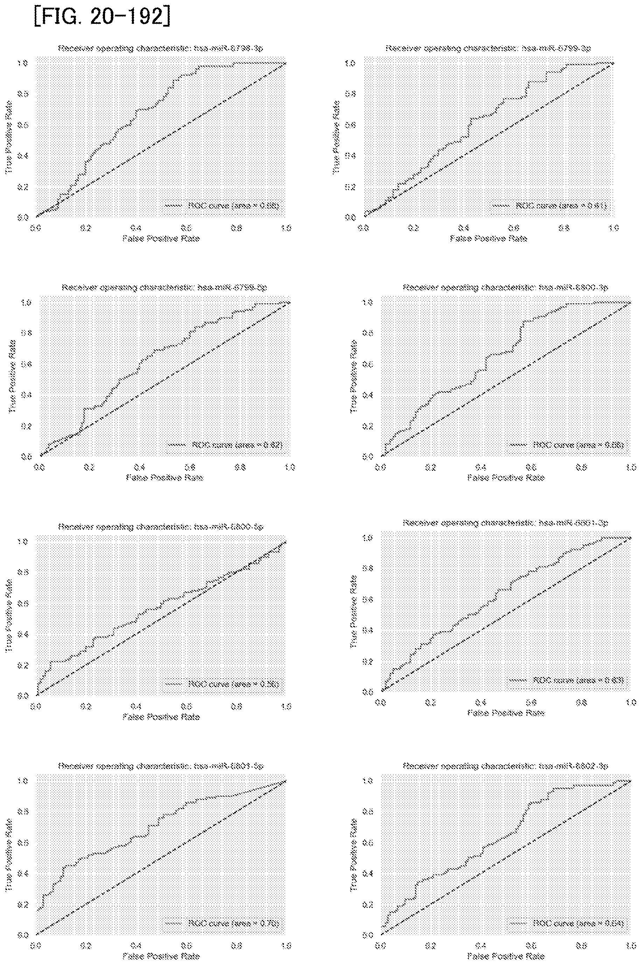

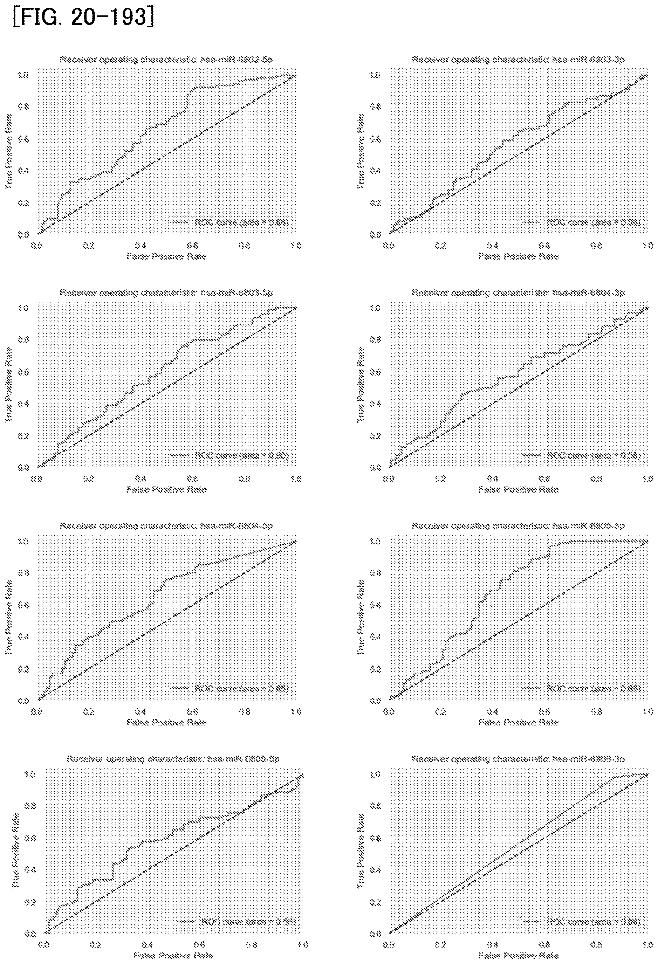

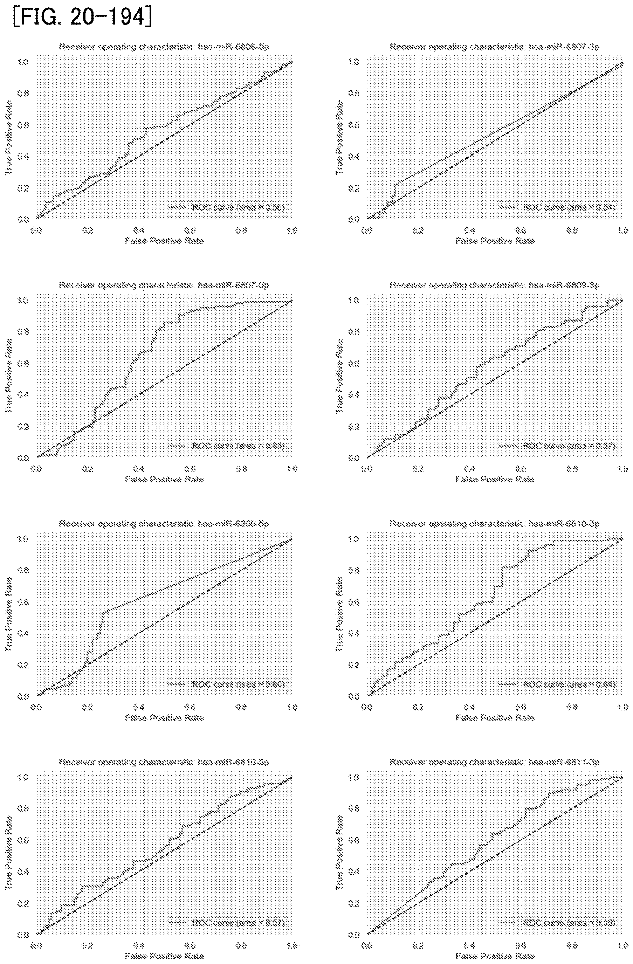

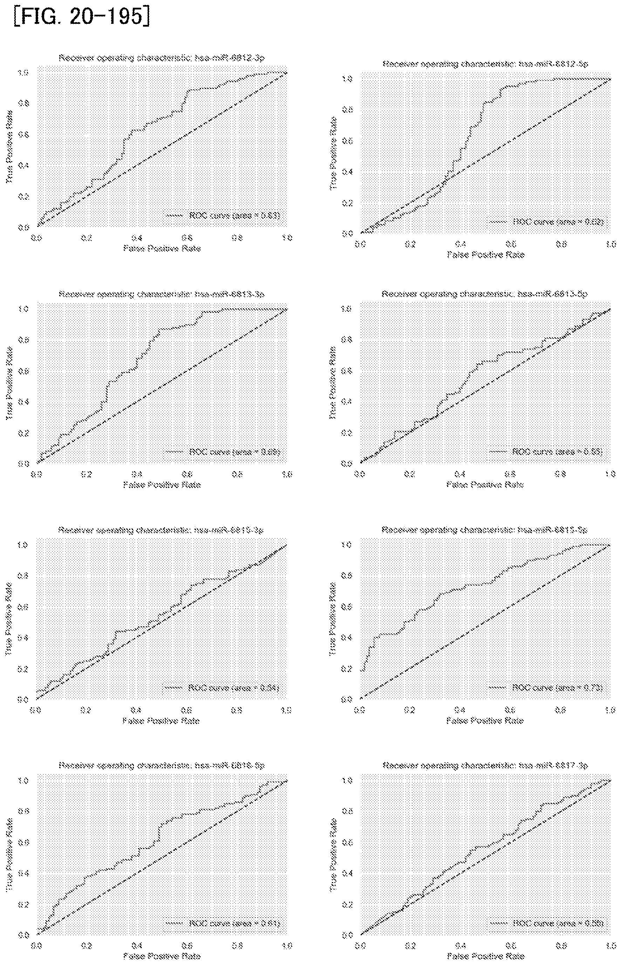

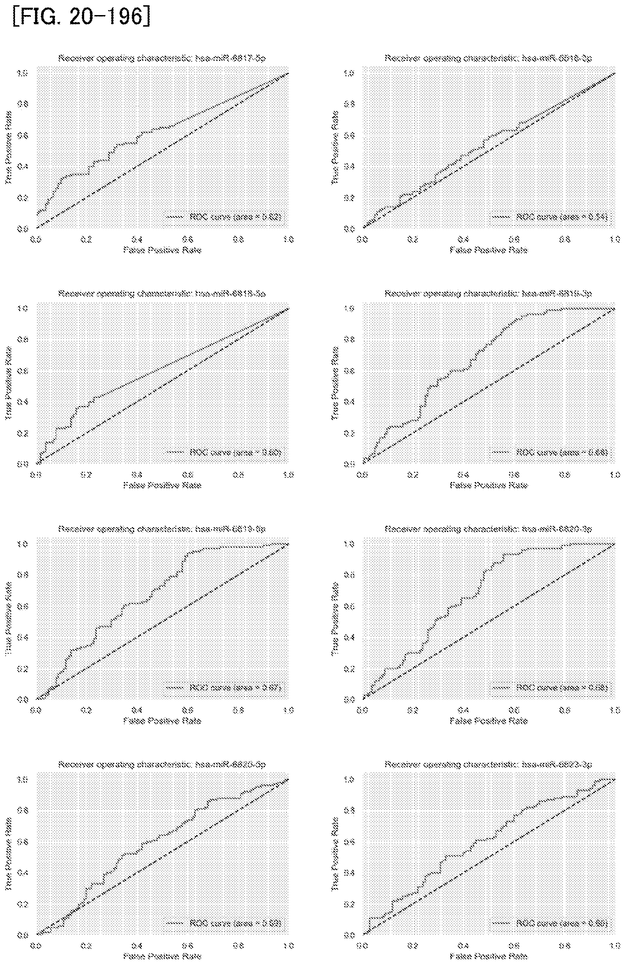

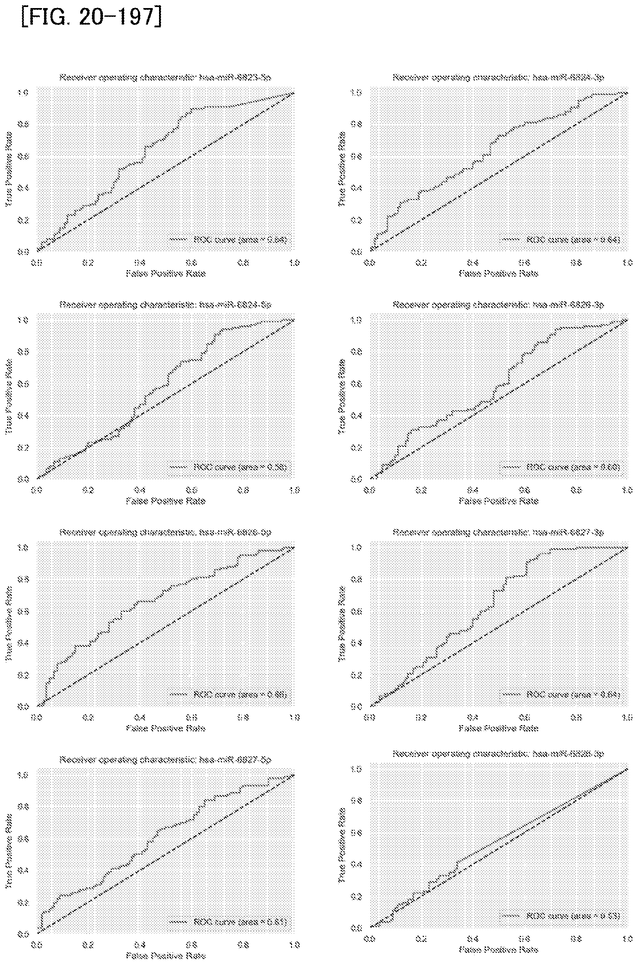

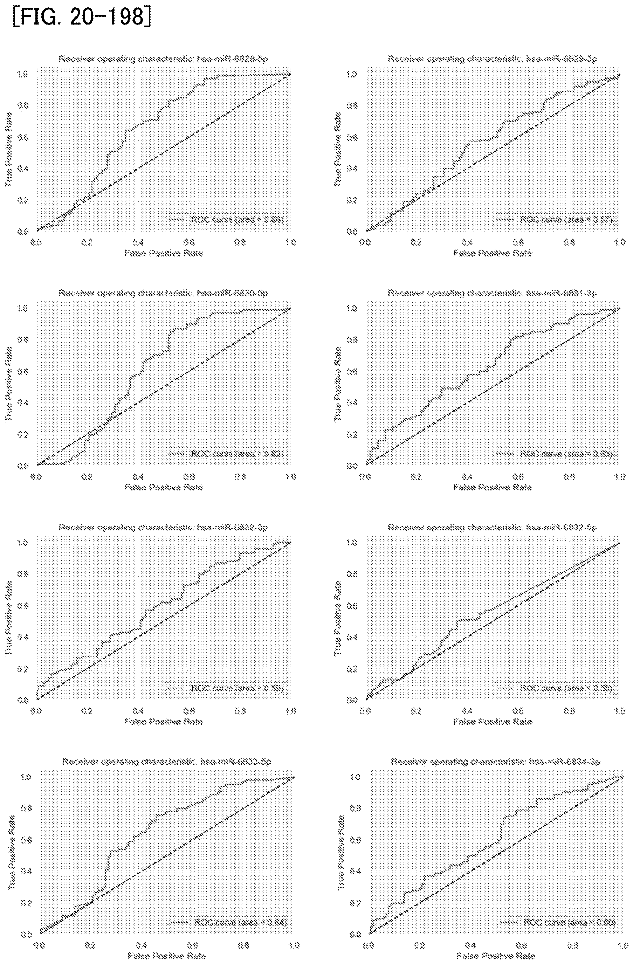

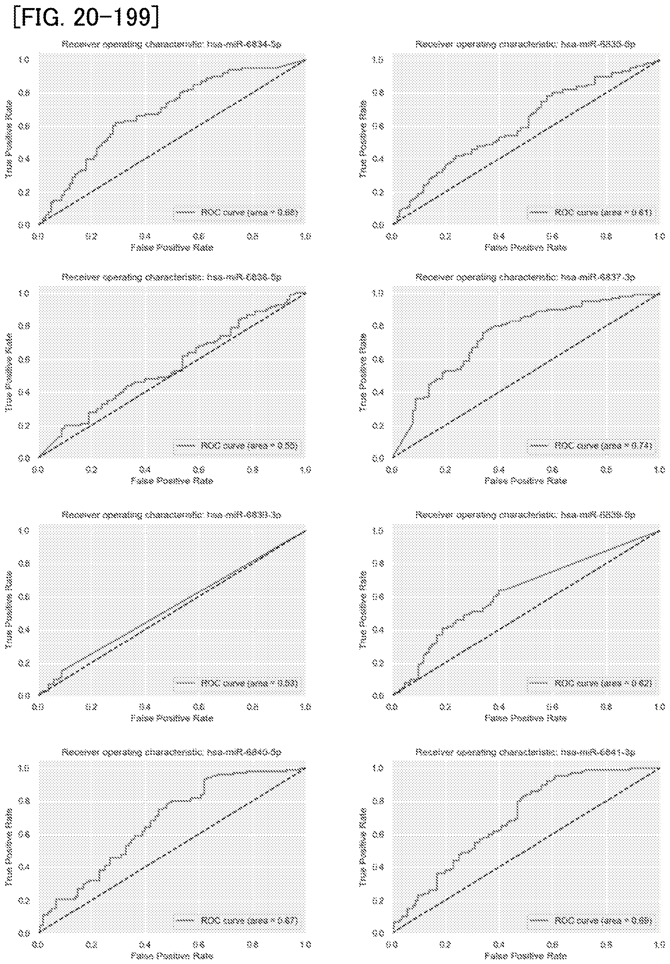

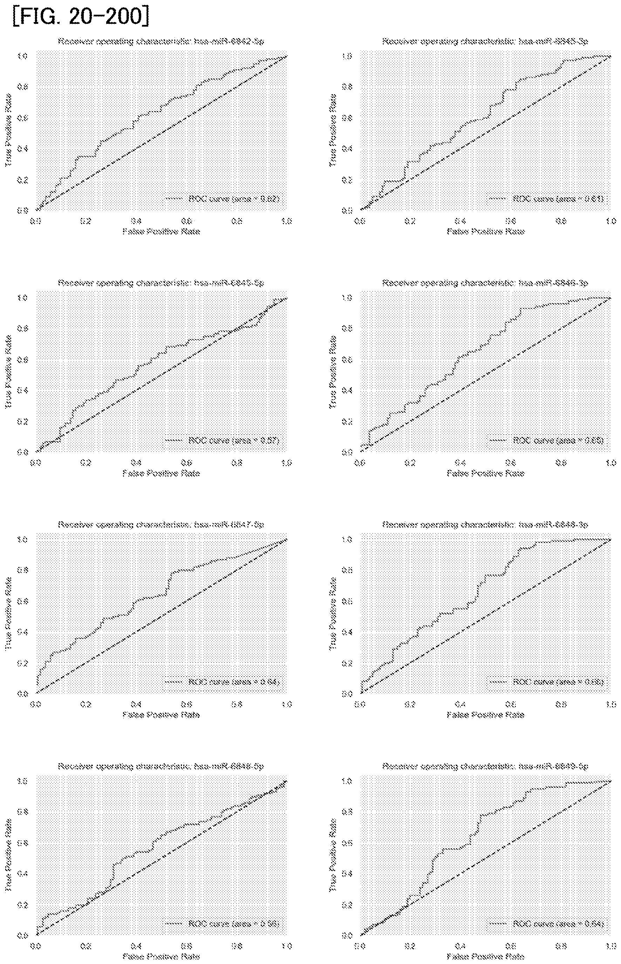

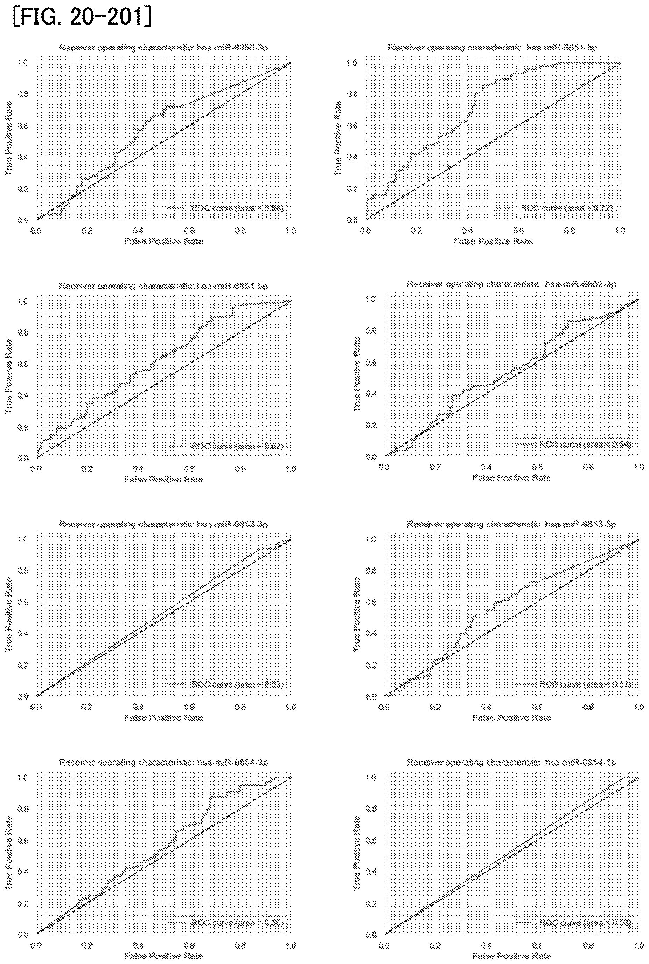

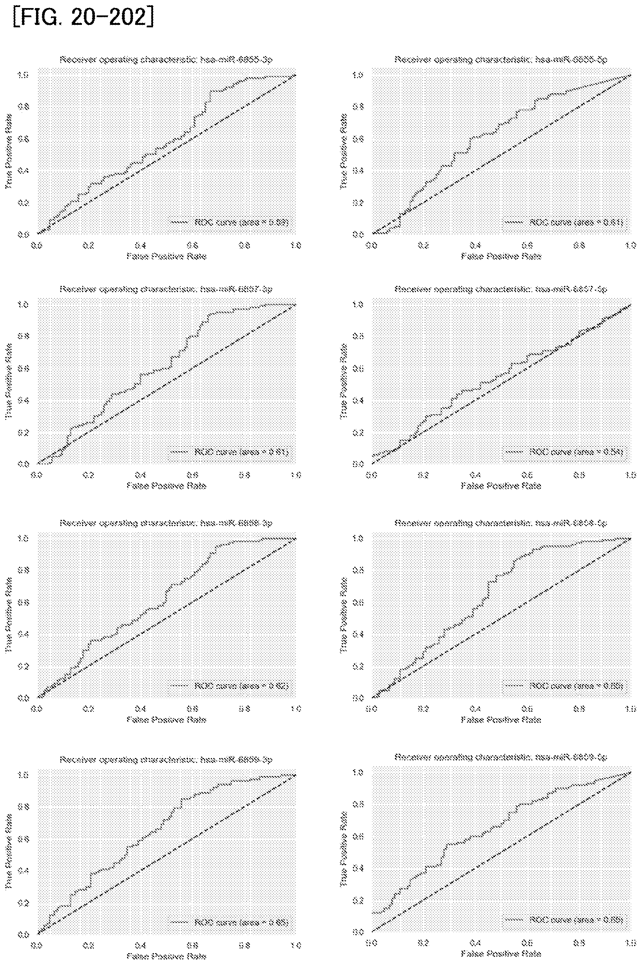

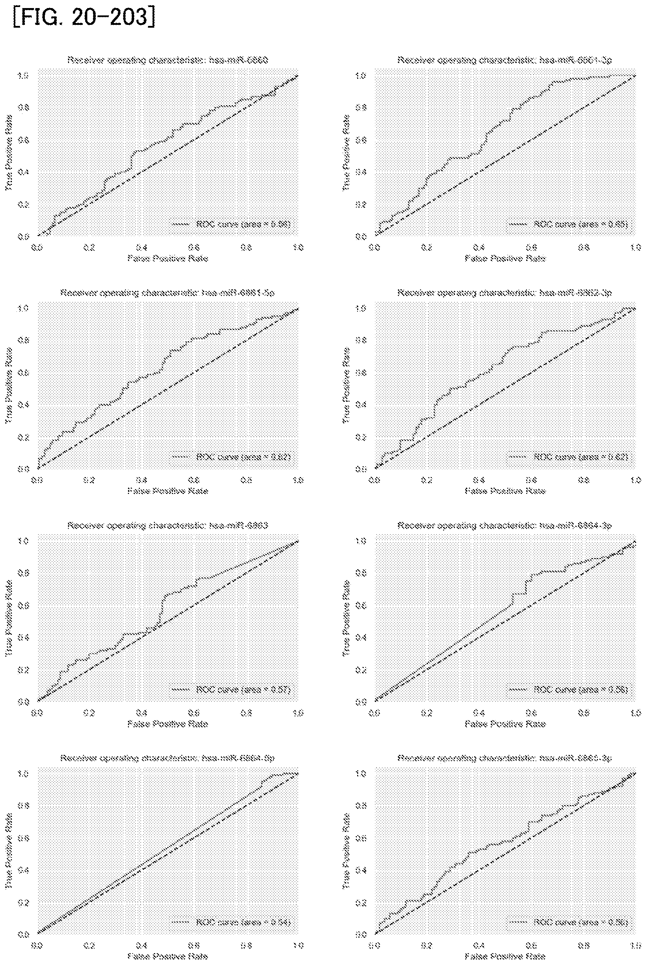

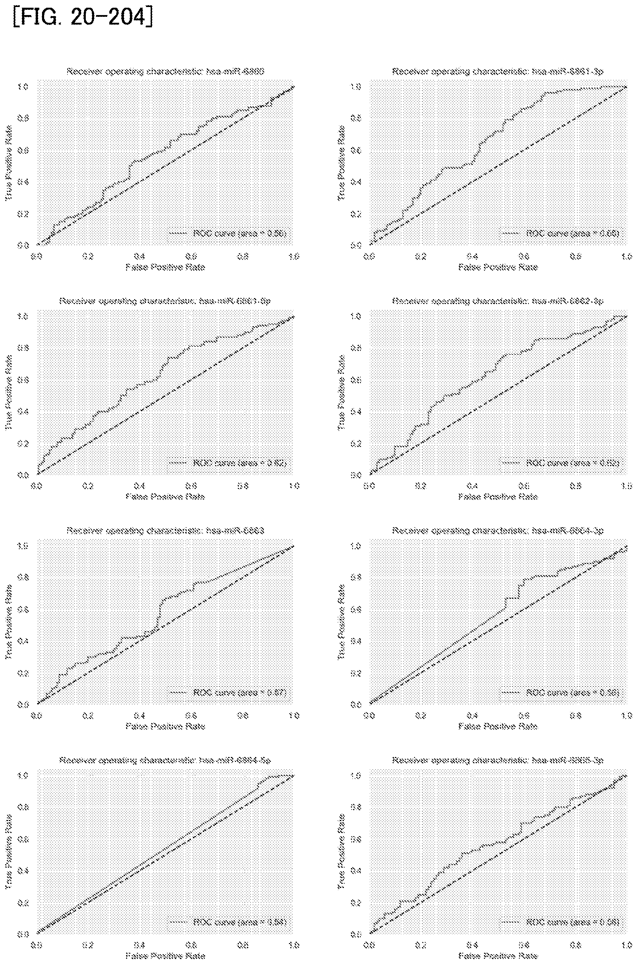

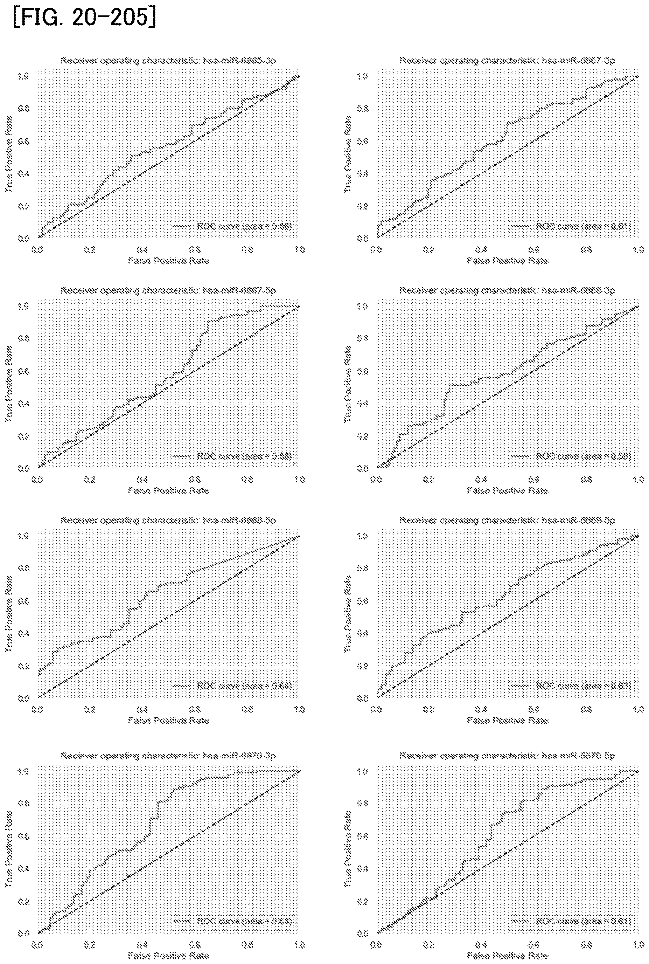

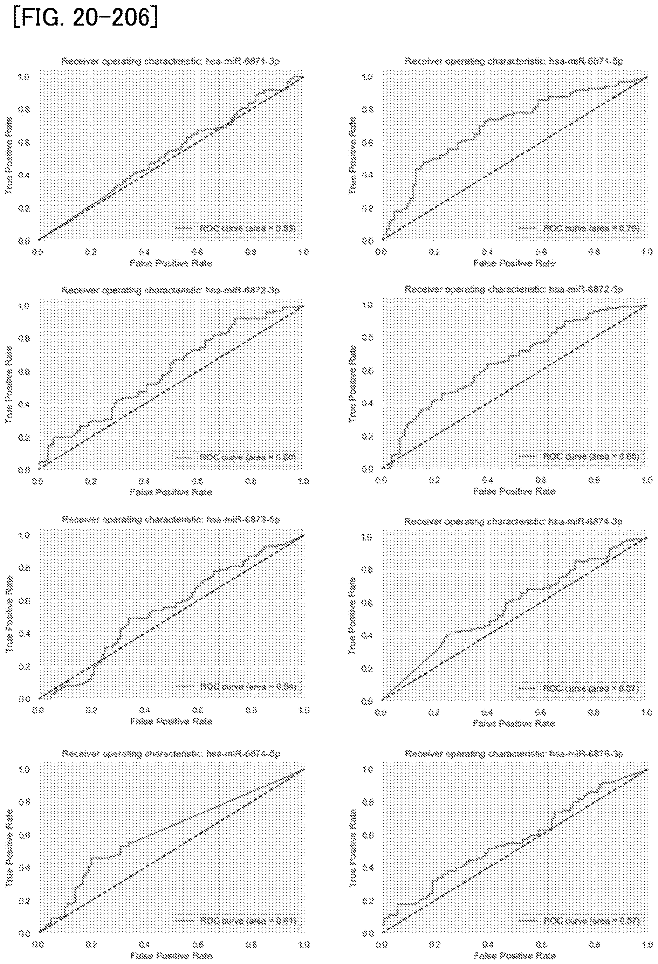

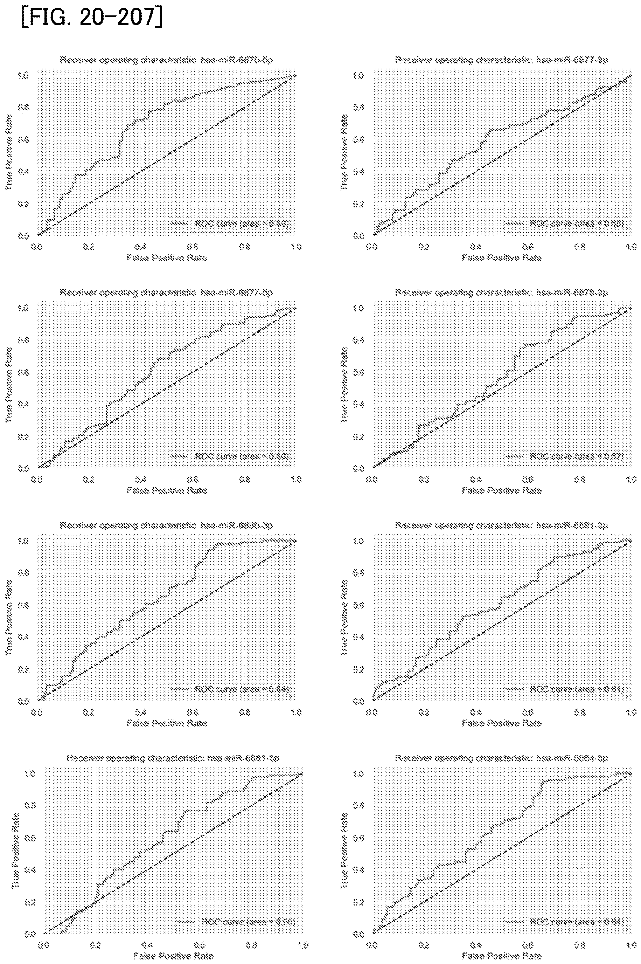

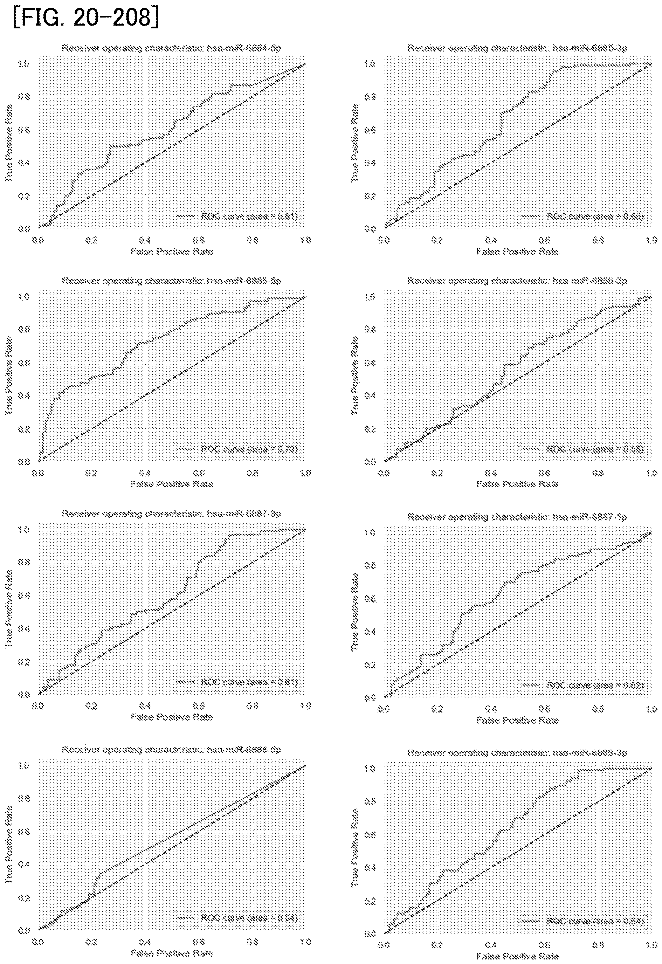

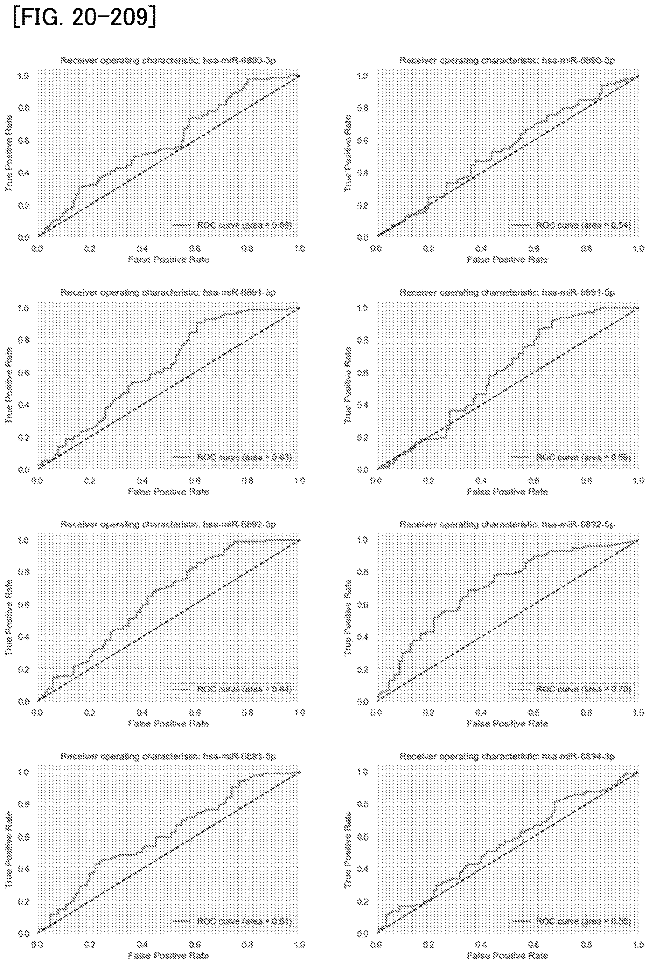

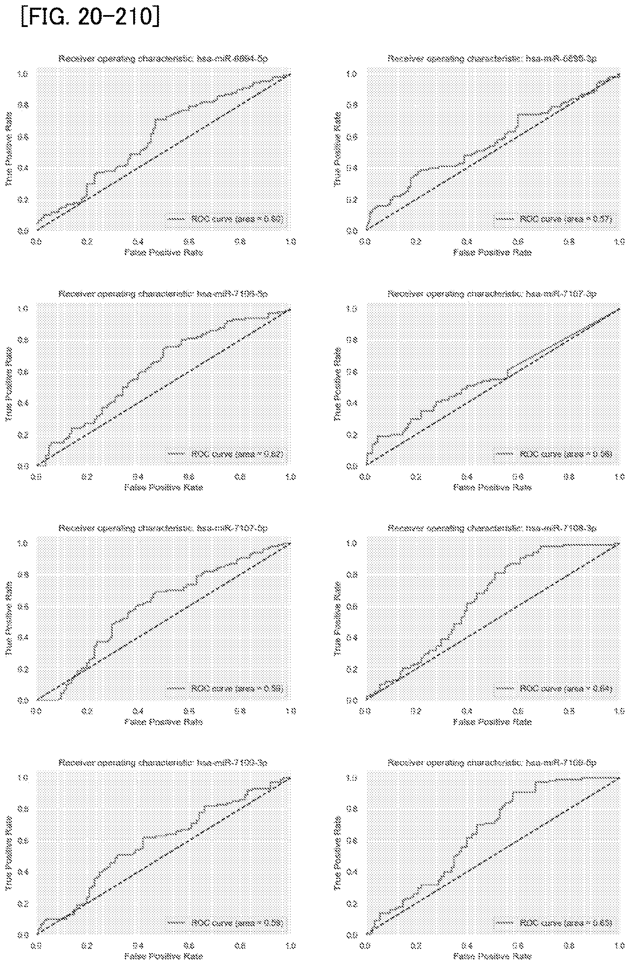

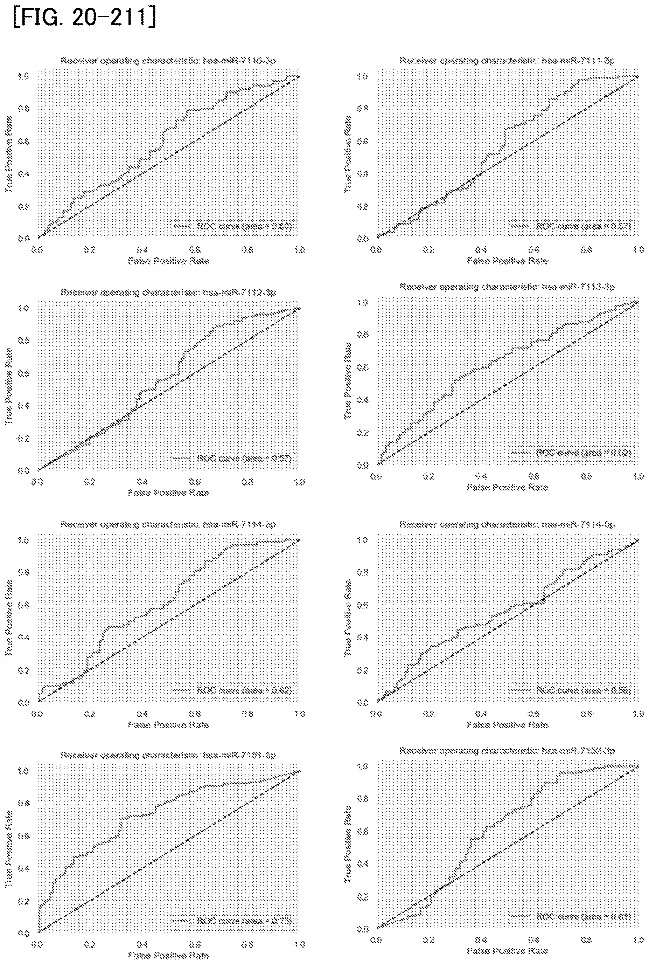

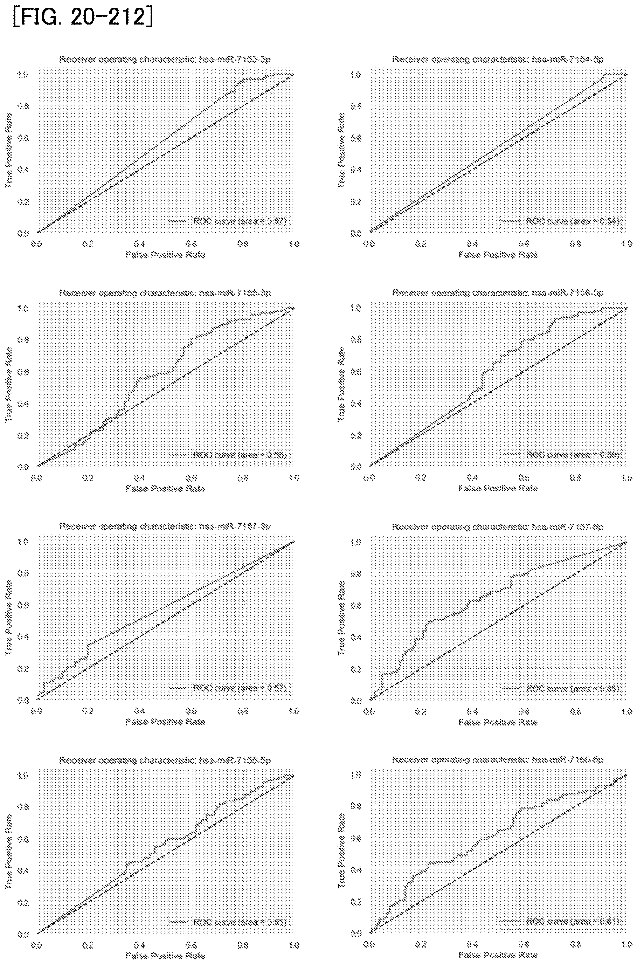

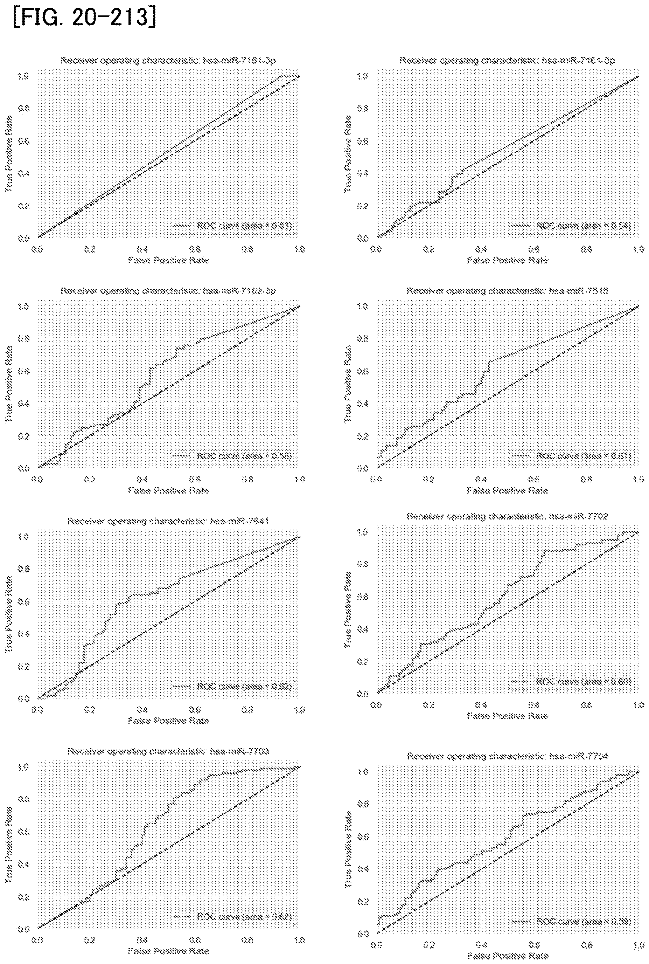

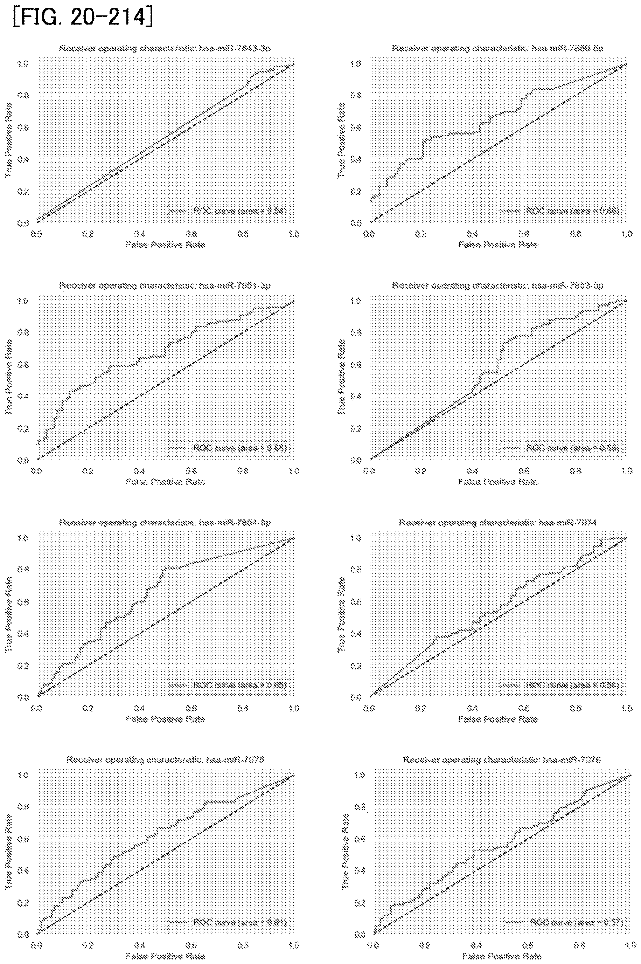

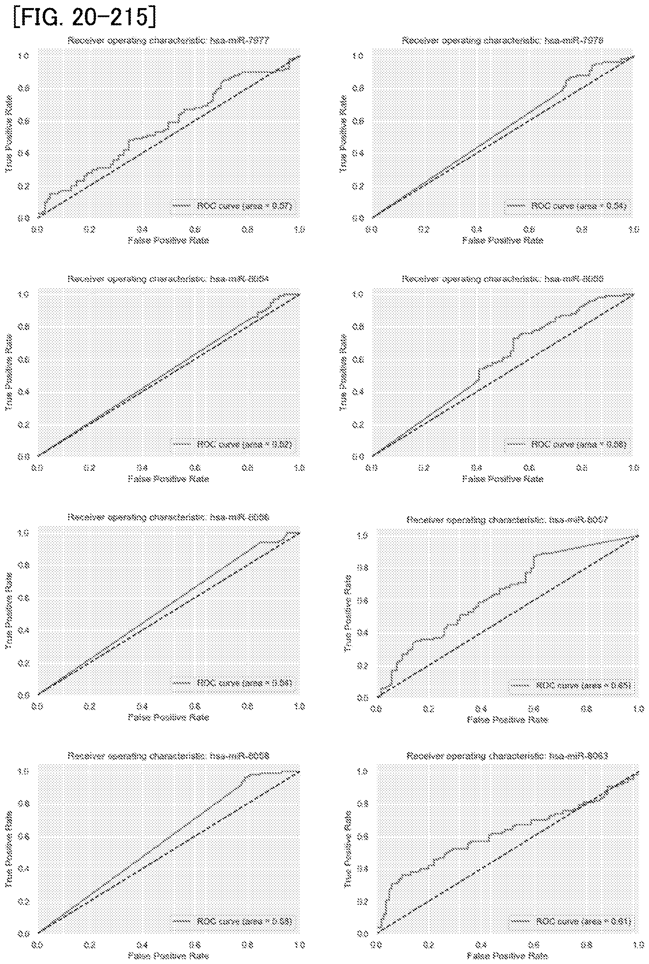

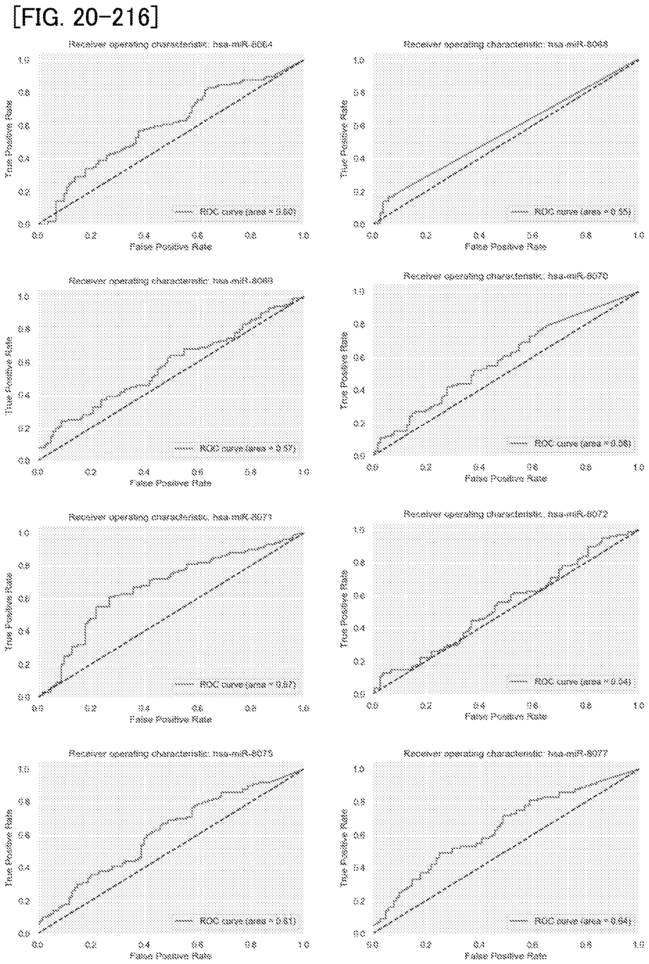

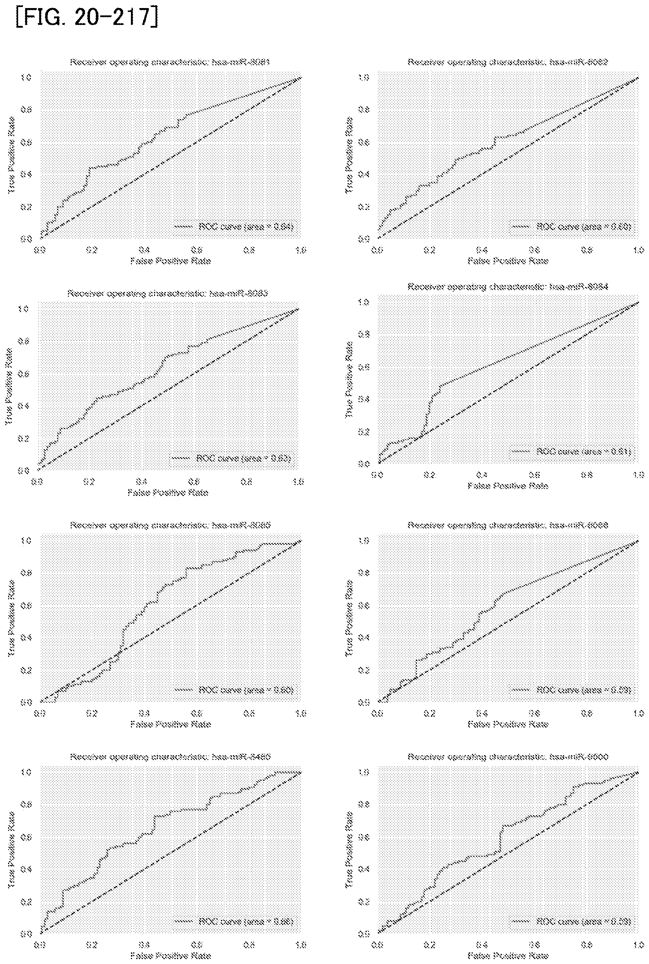

FIGS. 20-1 to 20-217 show ROCs curves for predicting lung cancer in humans using a single miRNA.

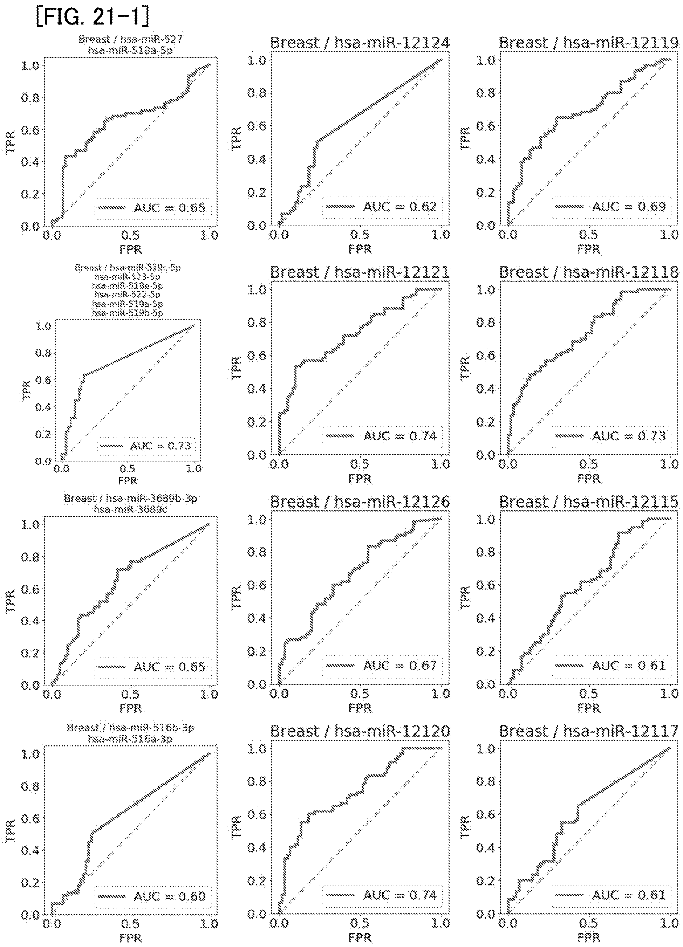

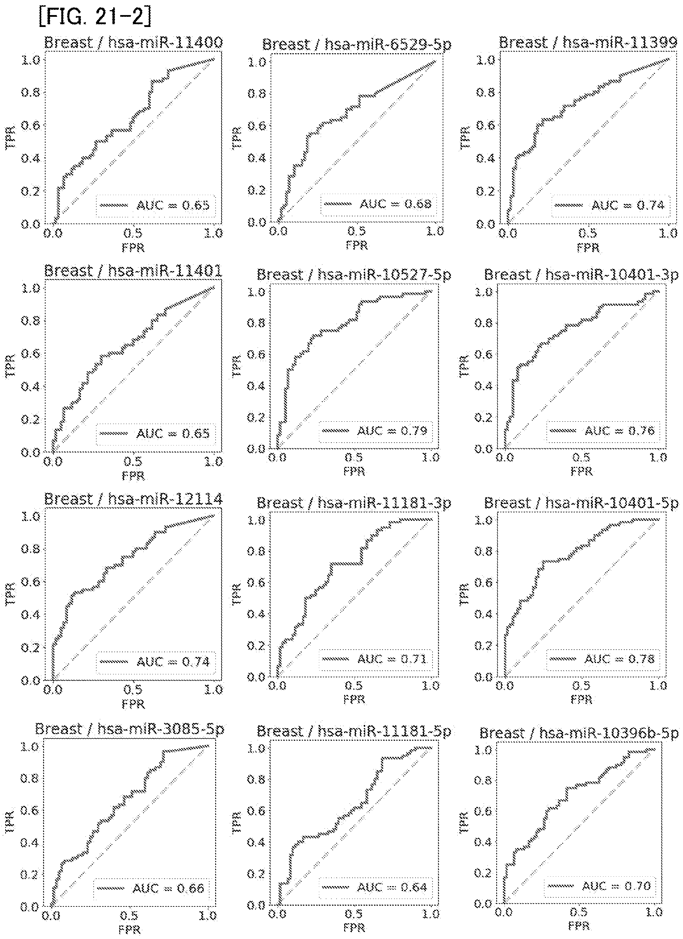

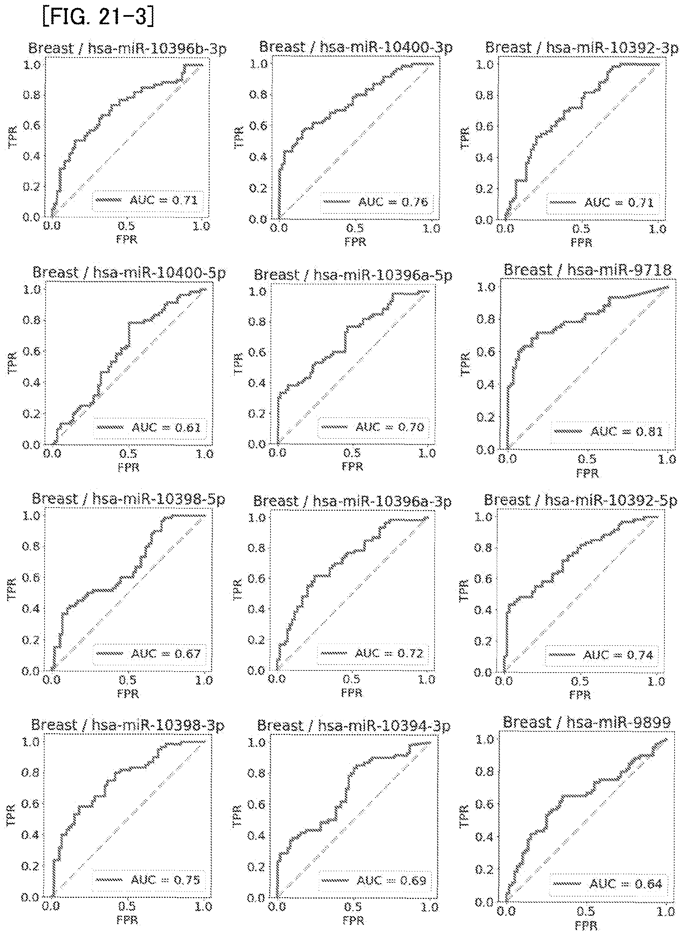

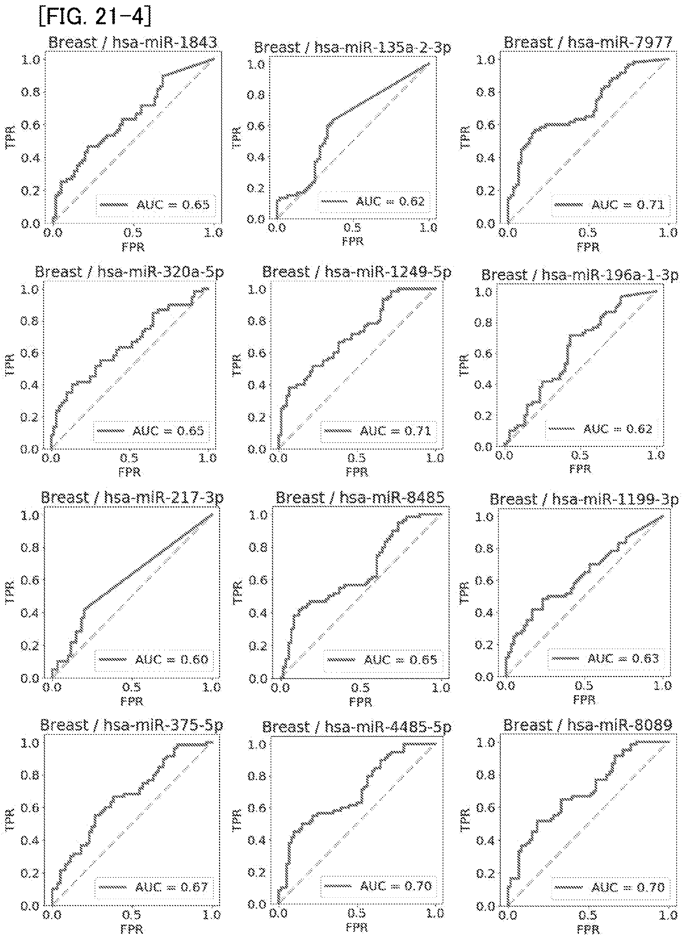

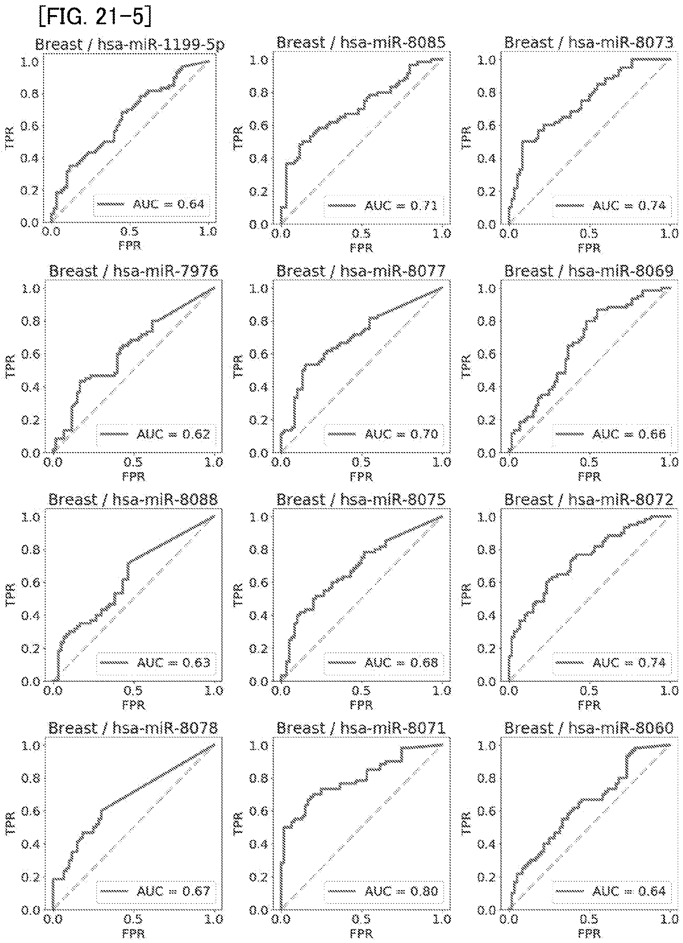

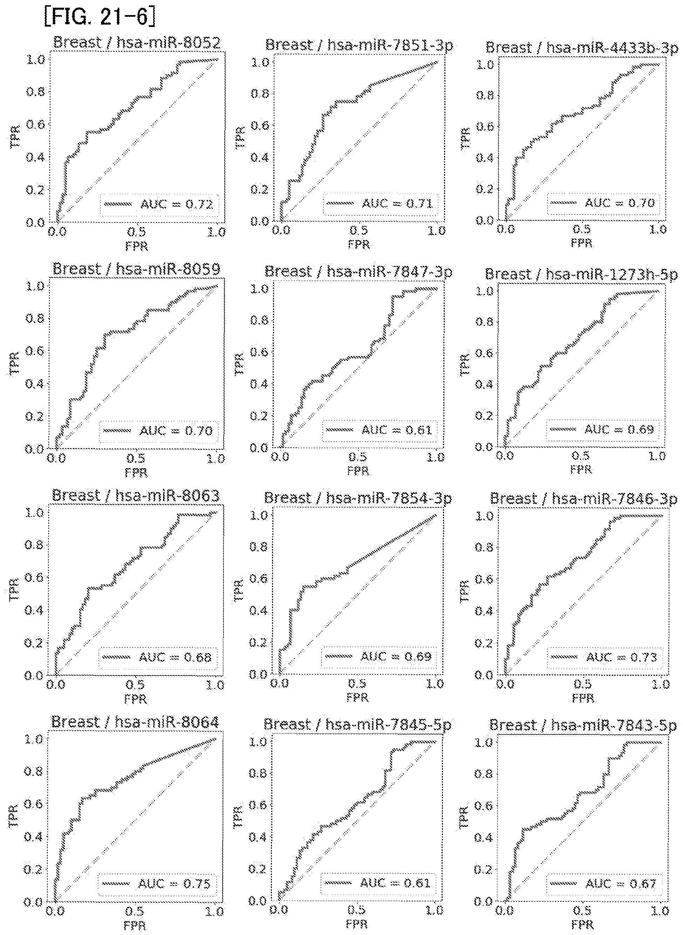

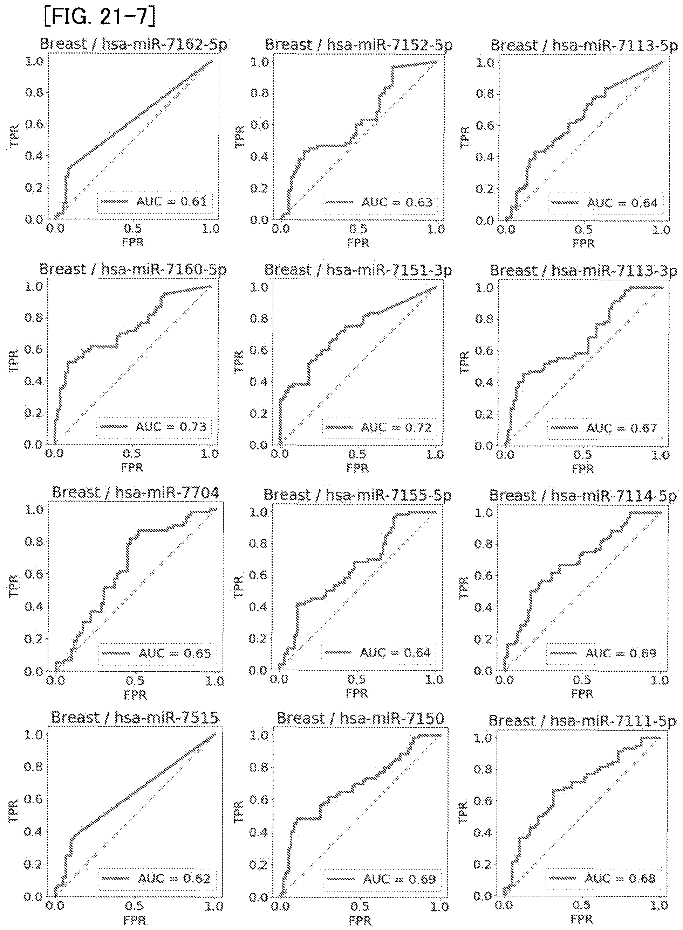

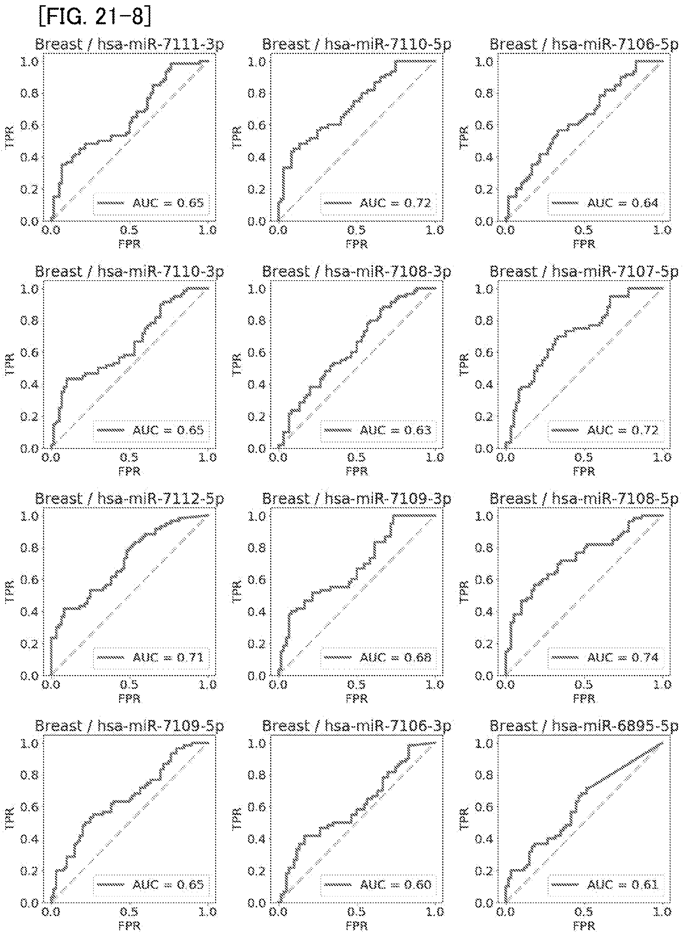

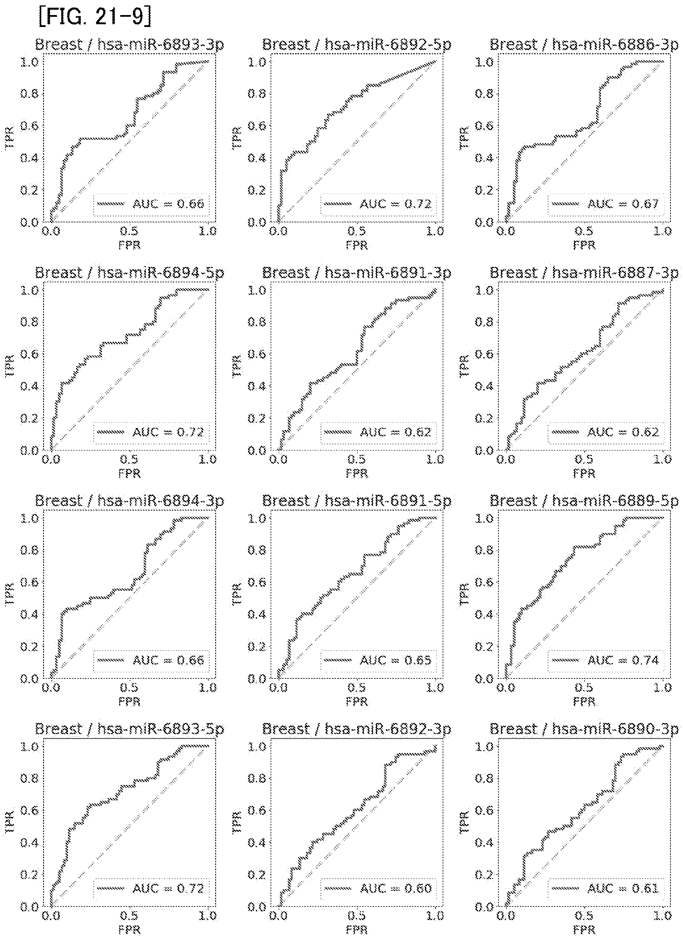

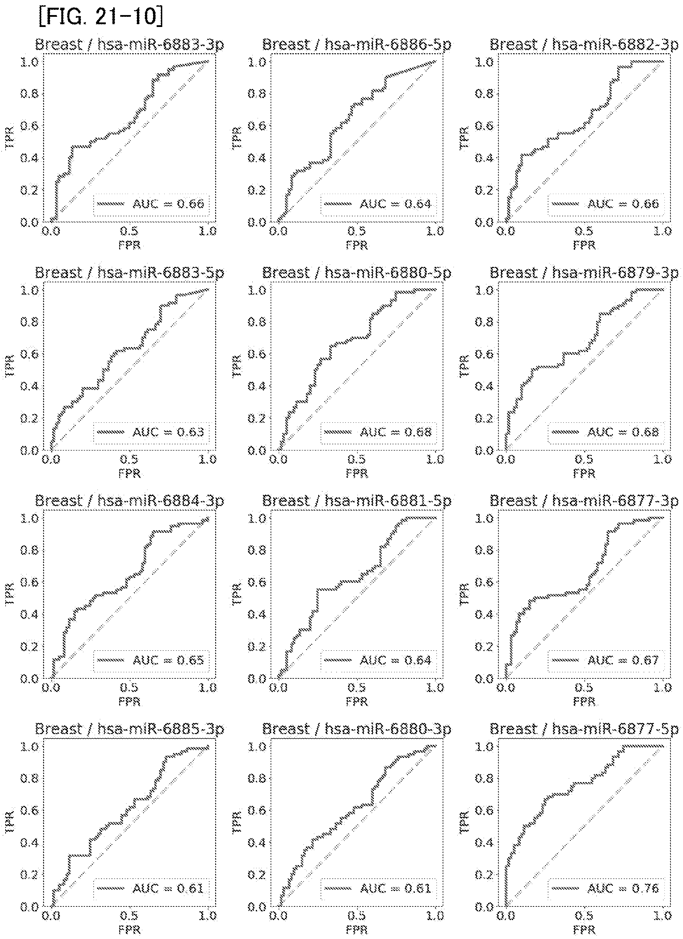

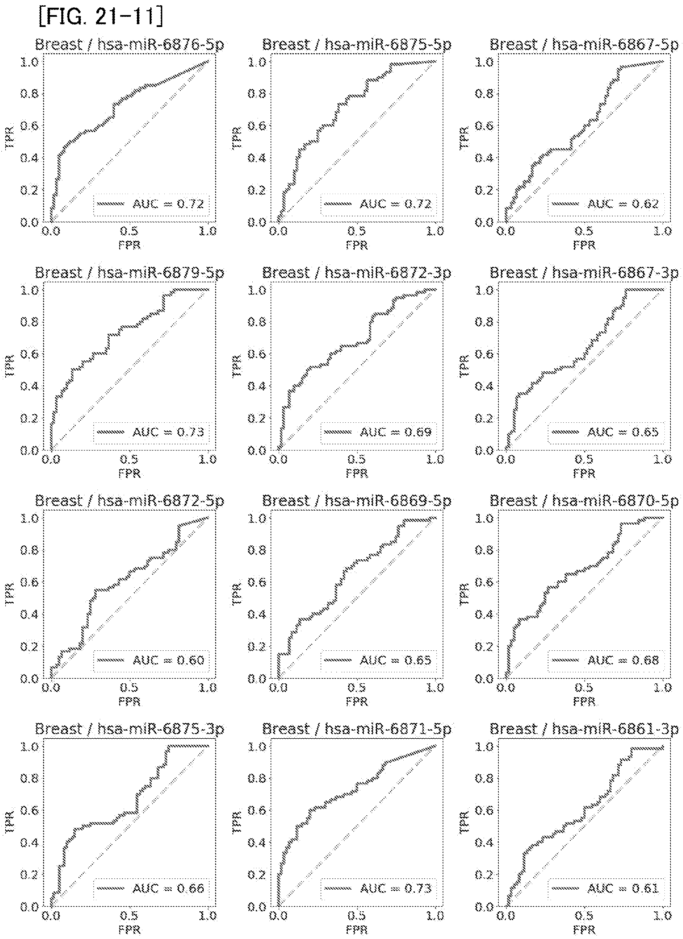

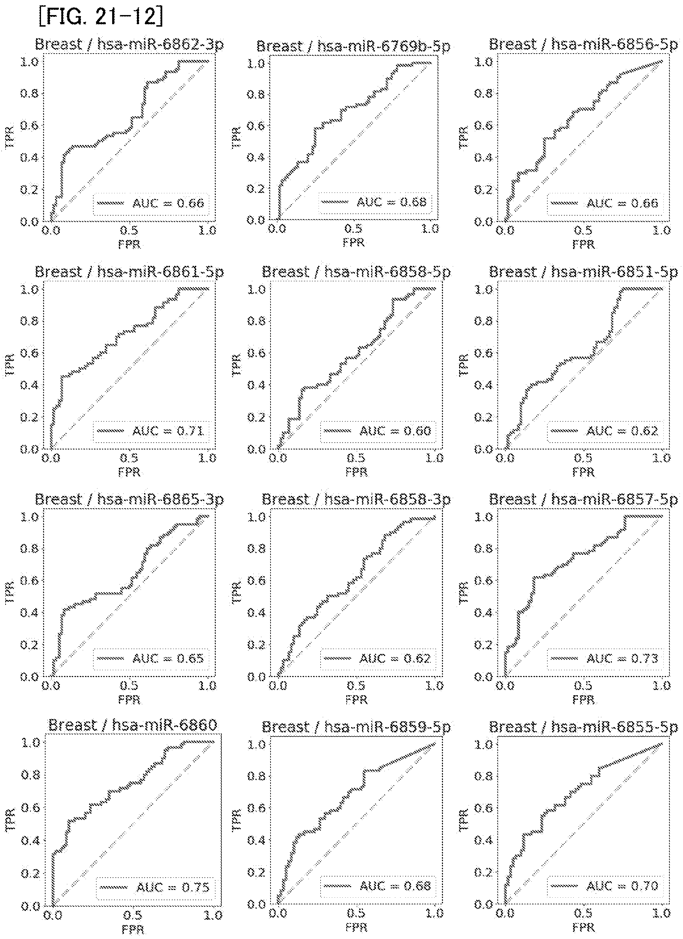

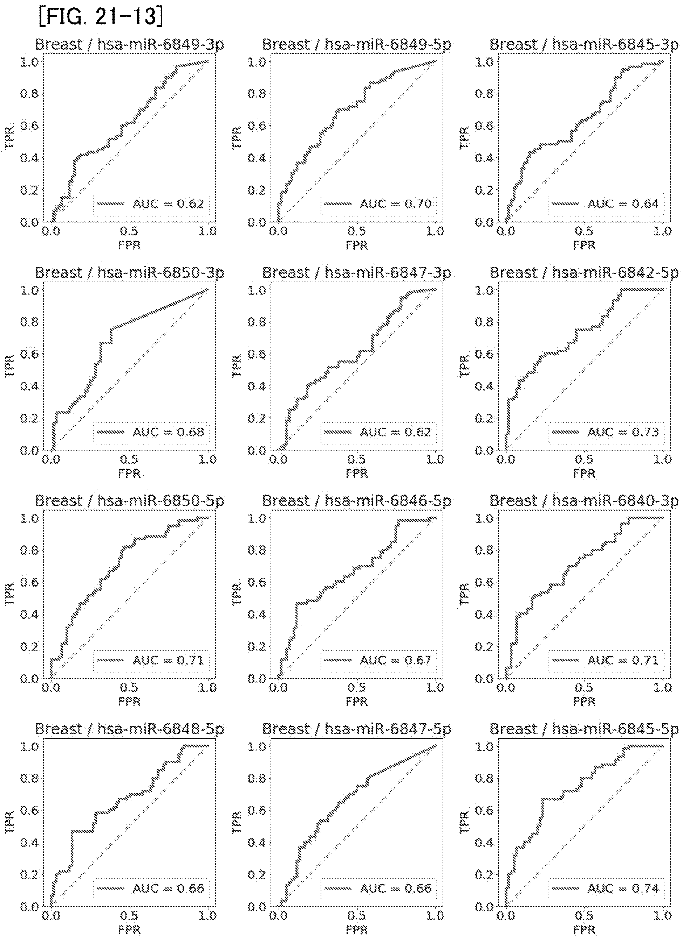

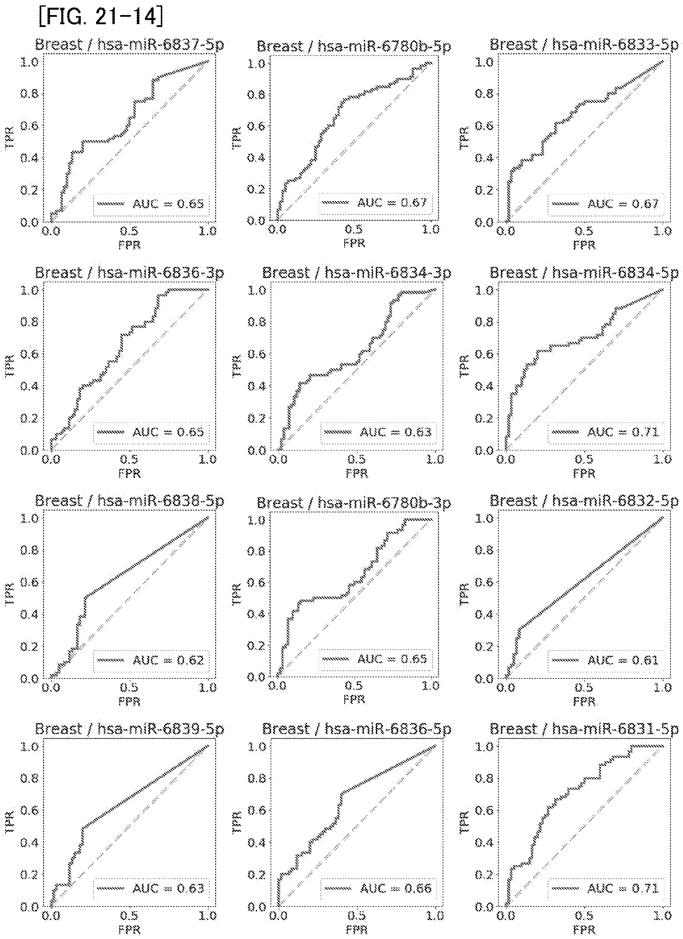

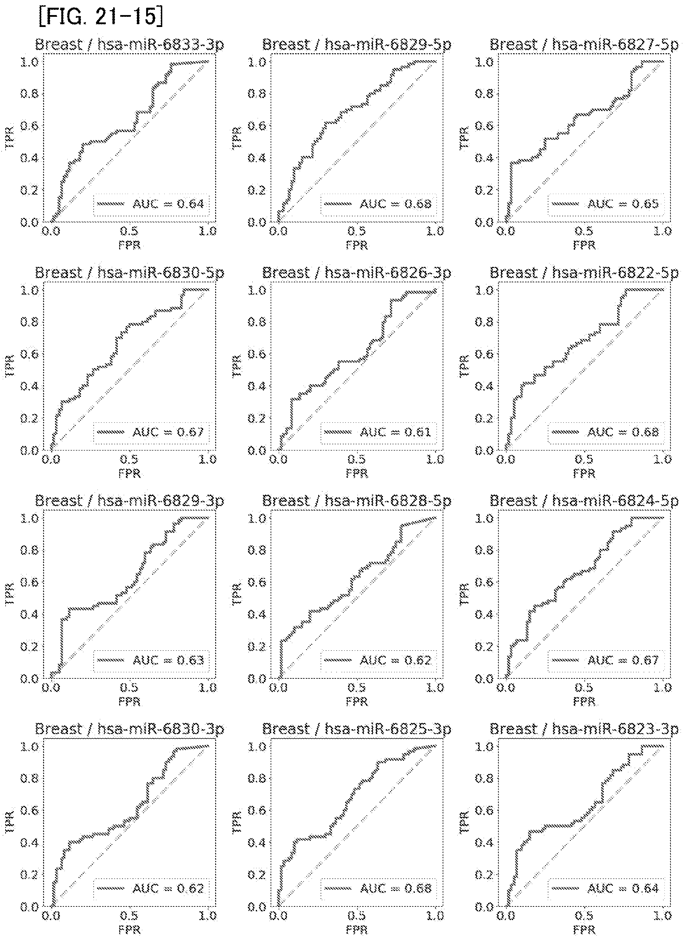

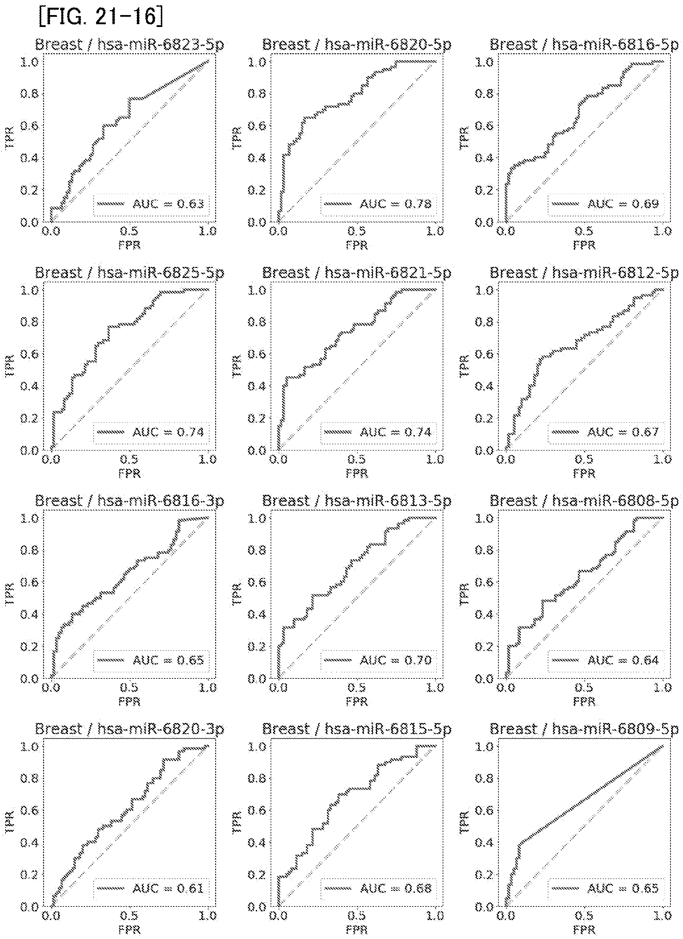

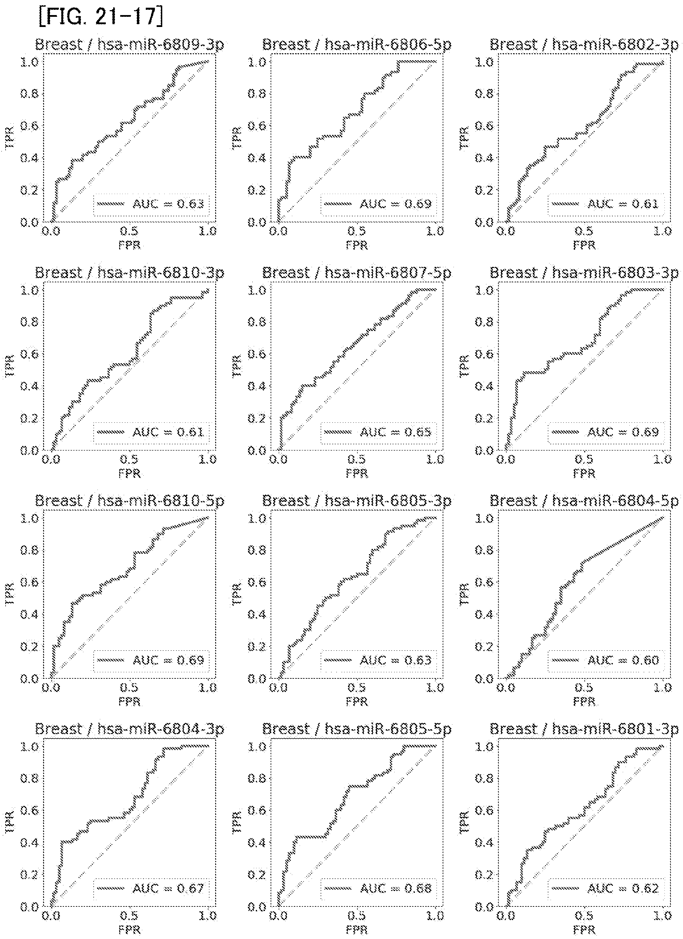

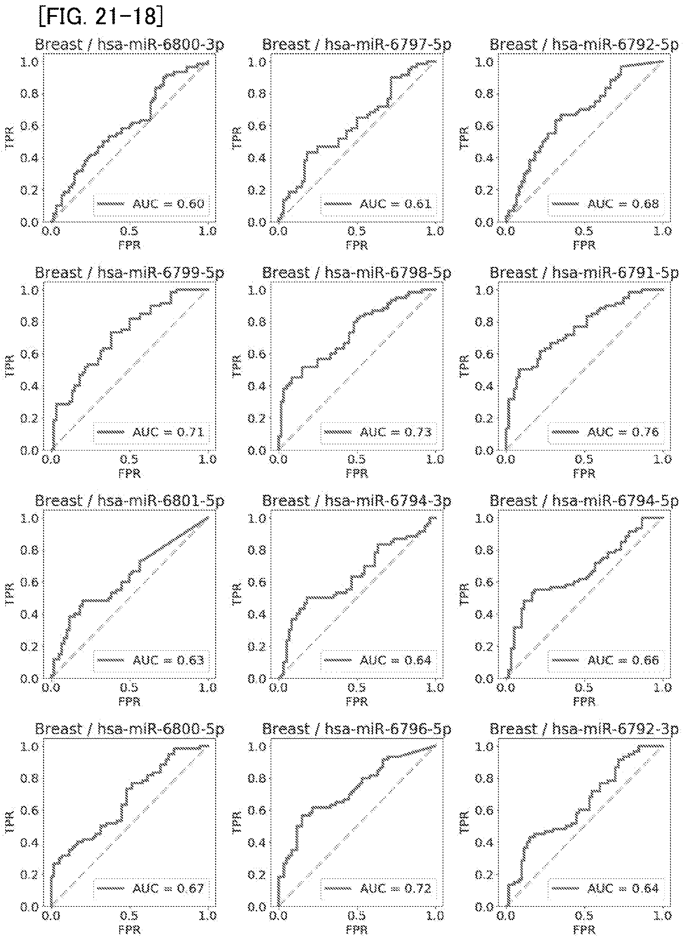

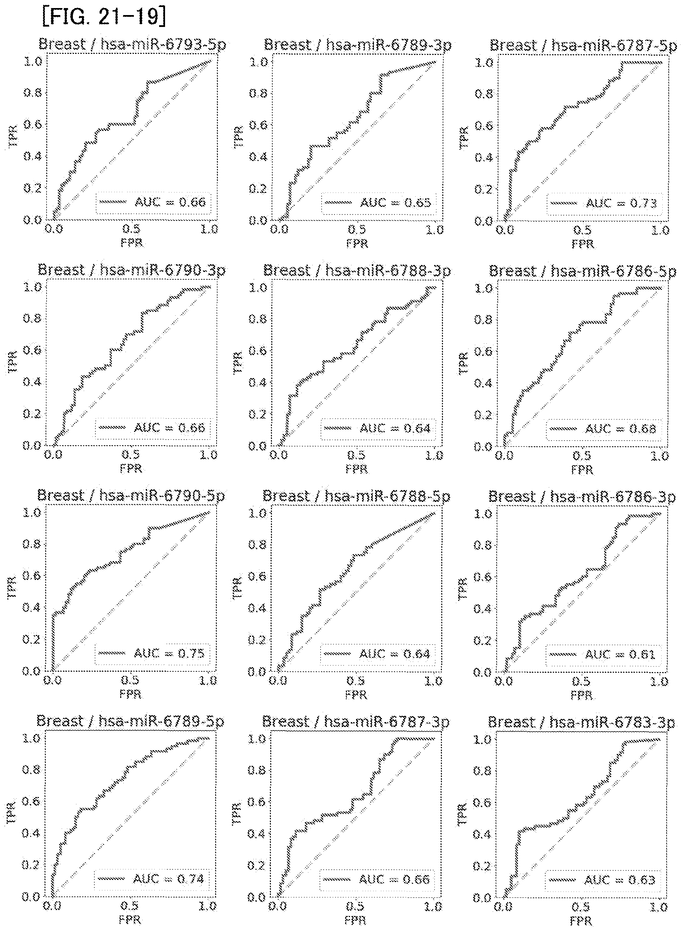

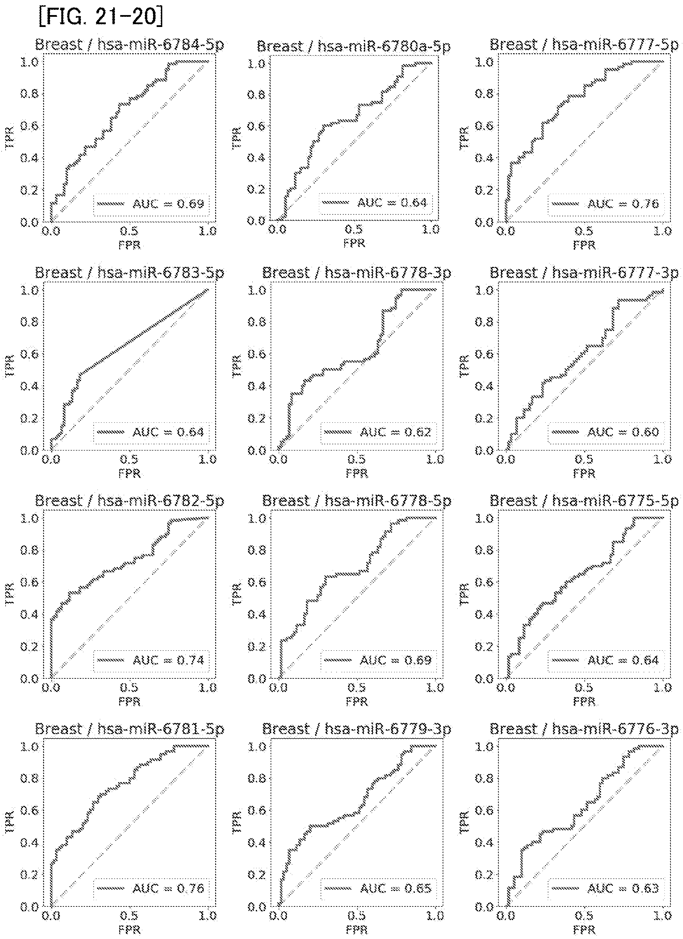

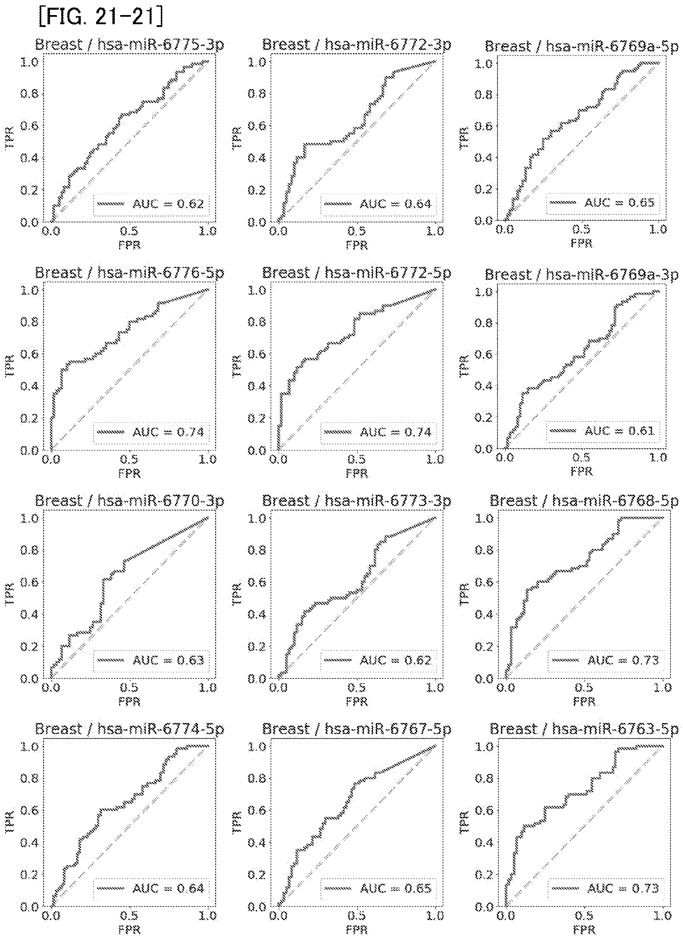

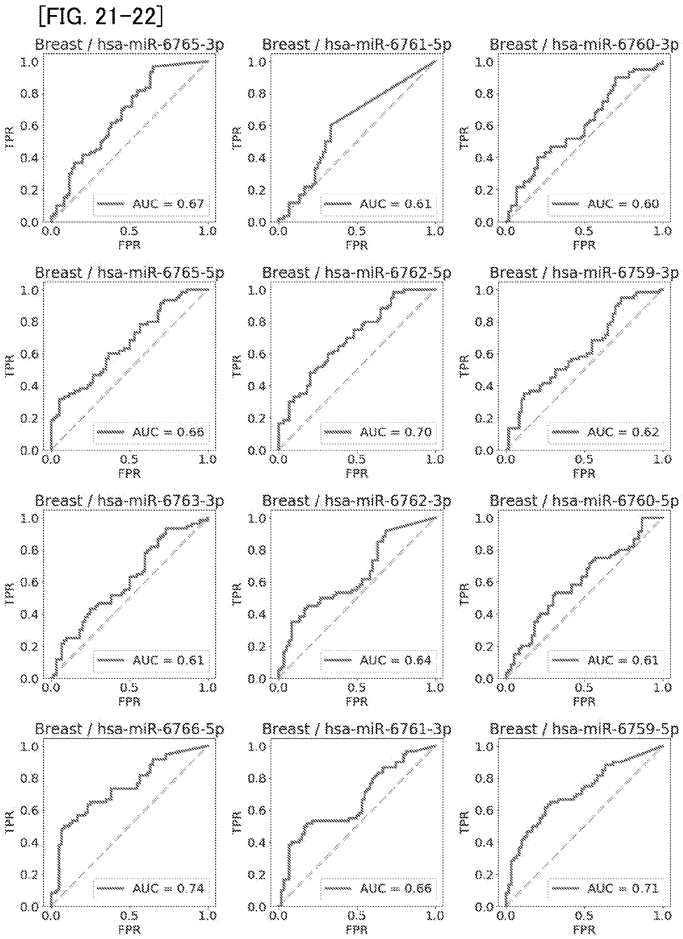

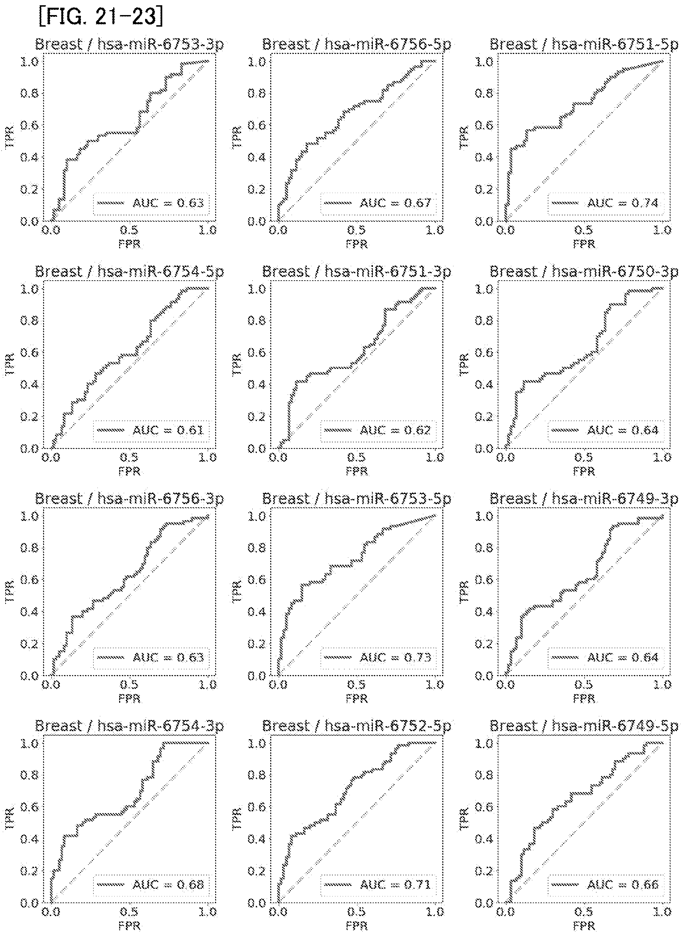

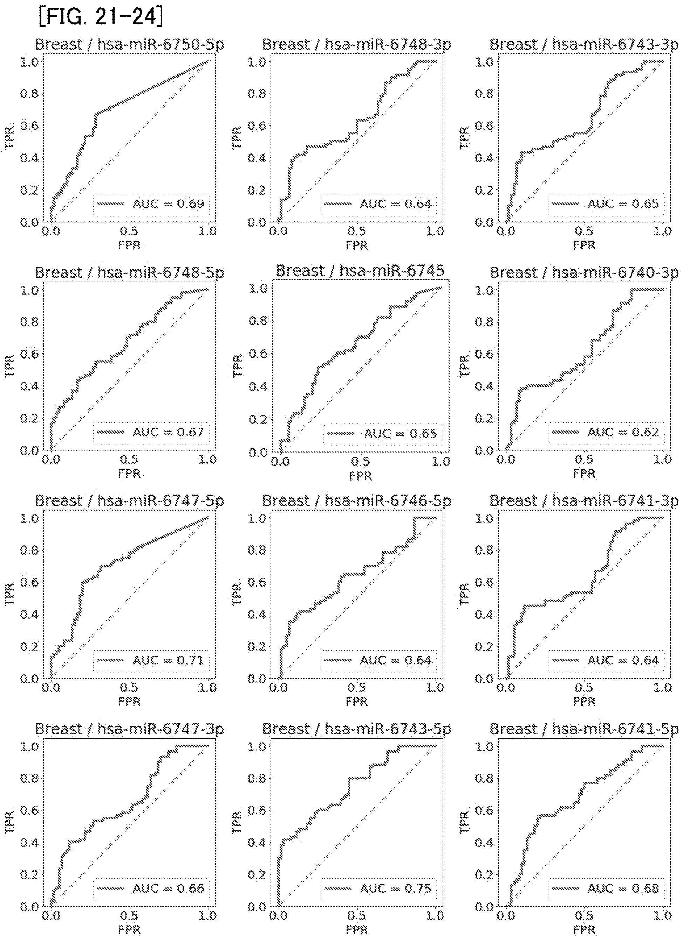

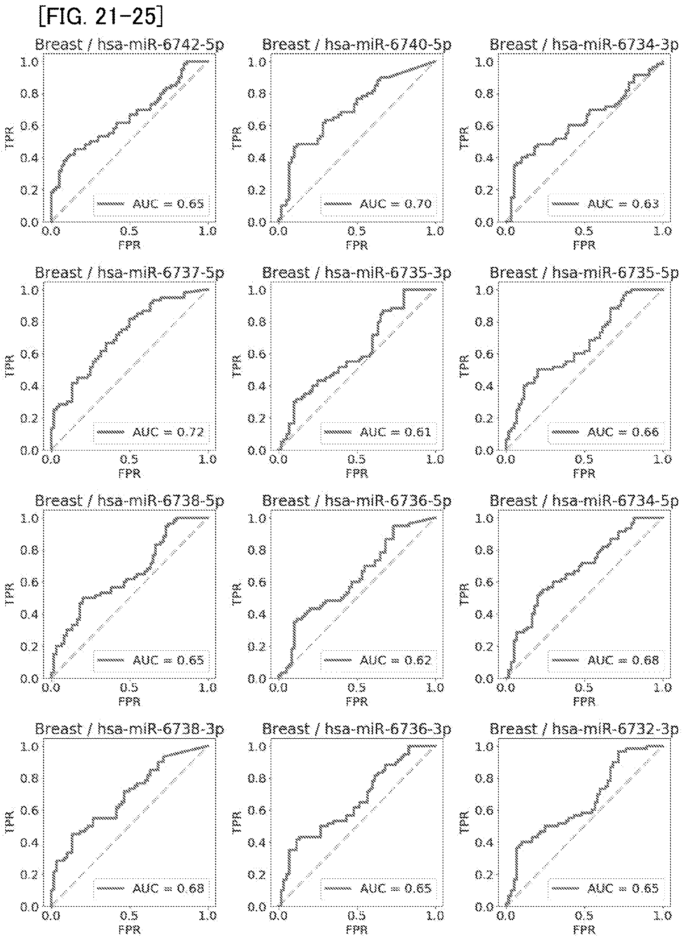

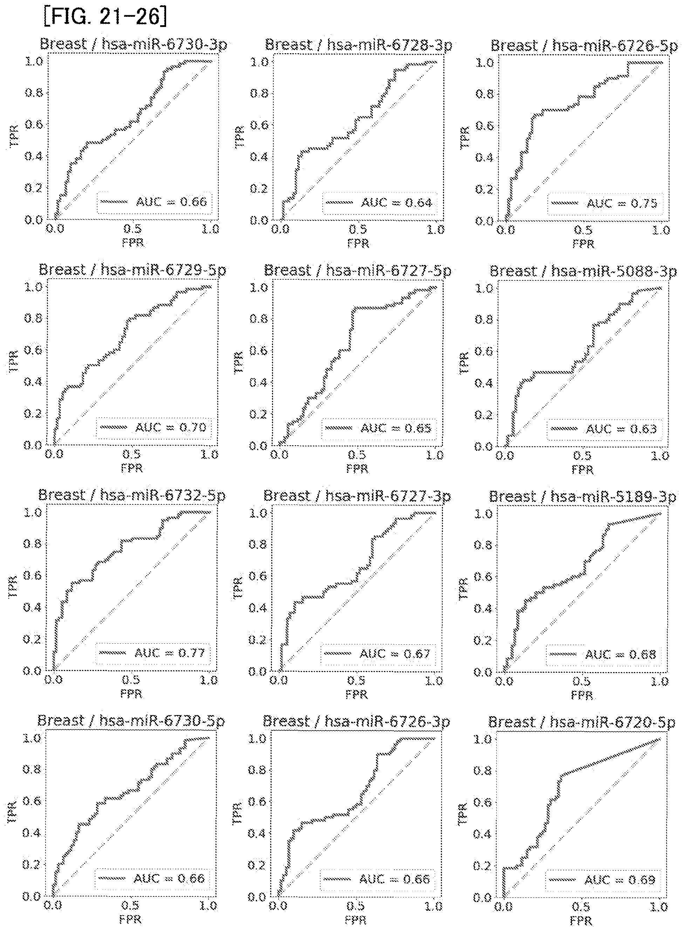

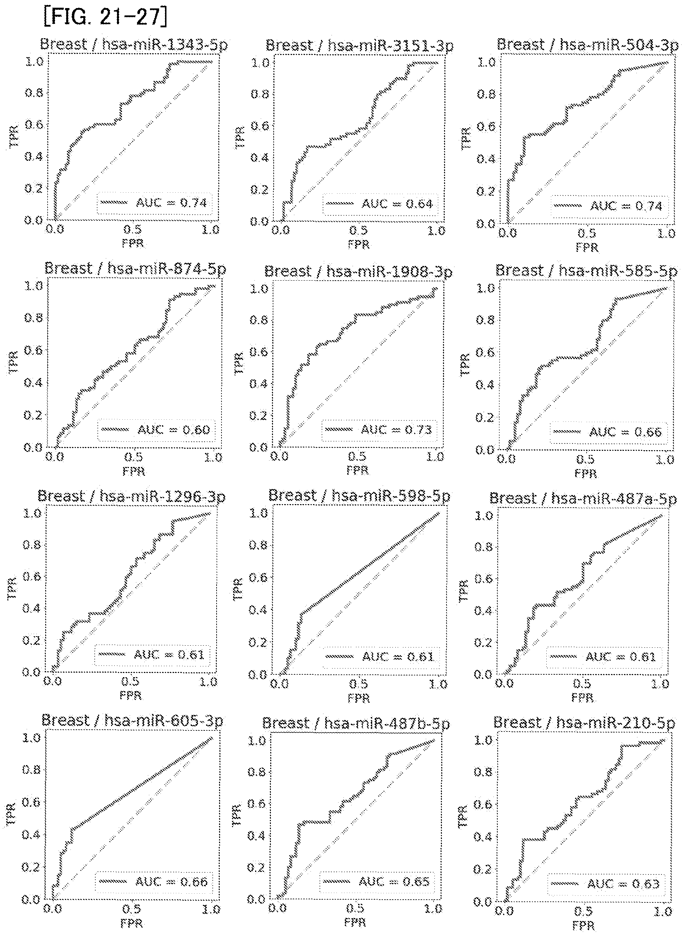

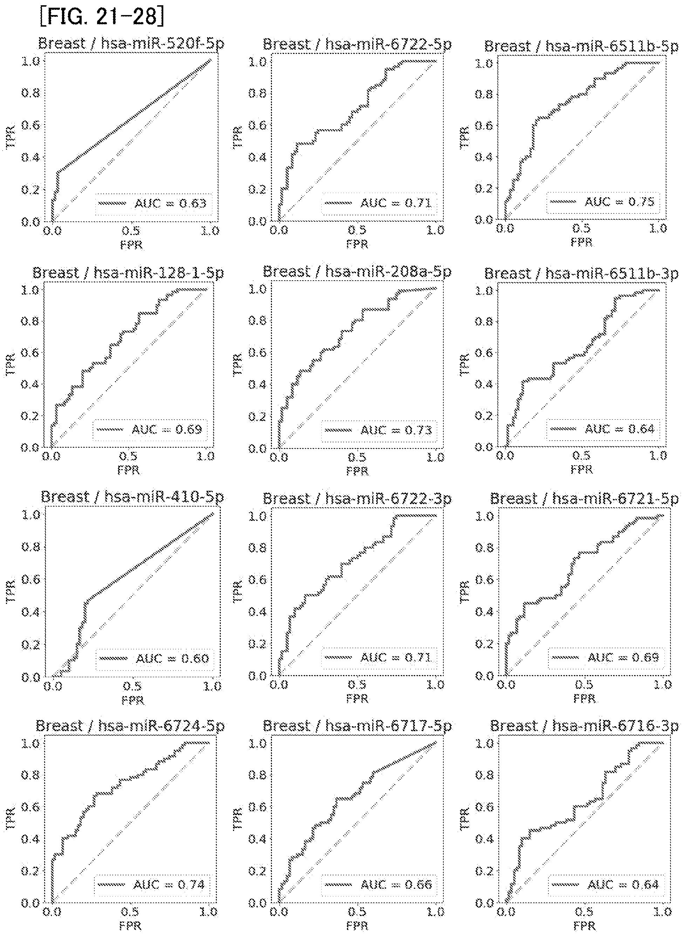

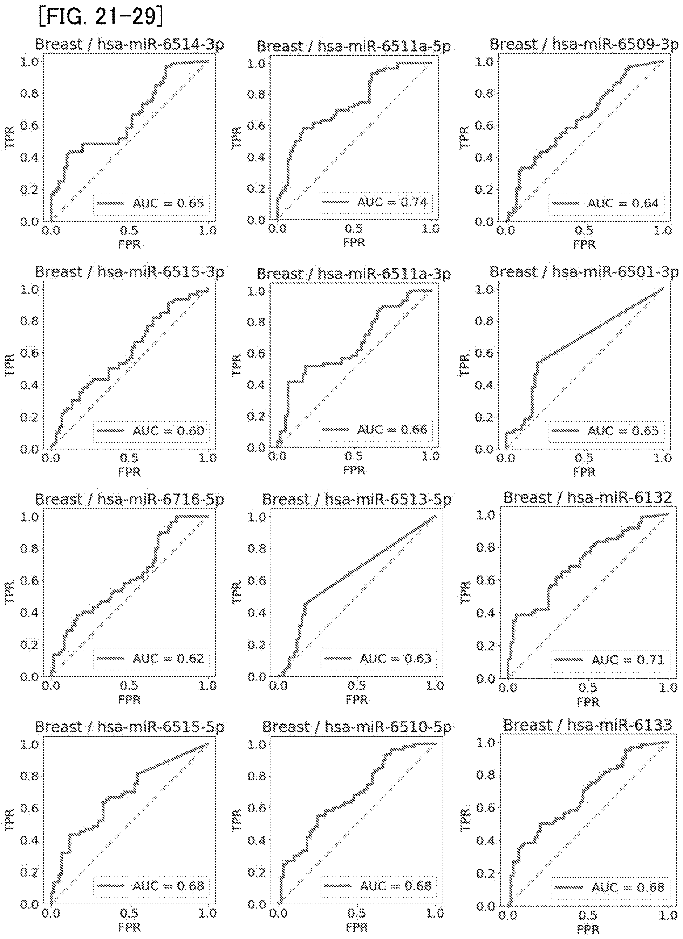

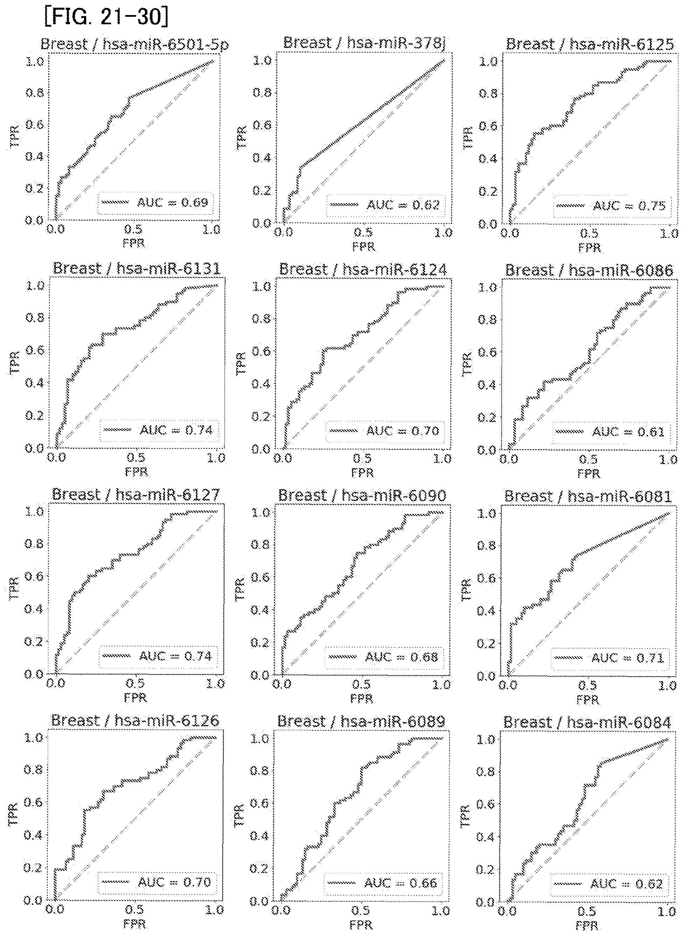

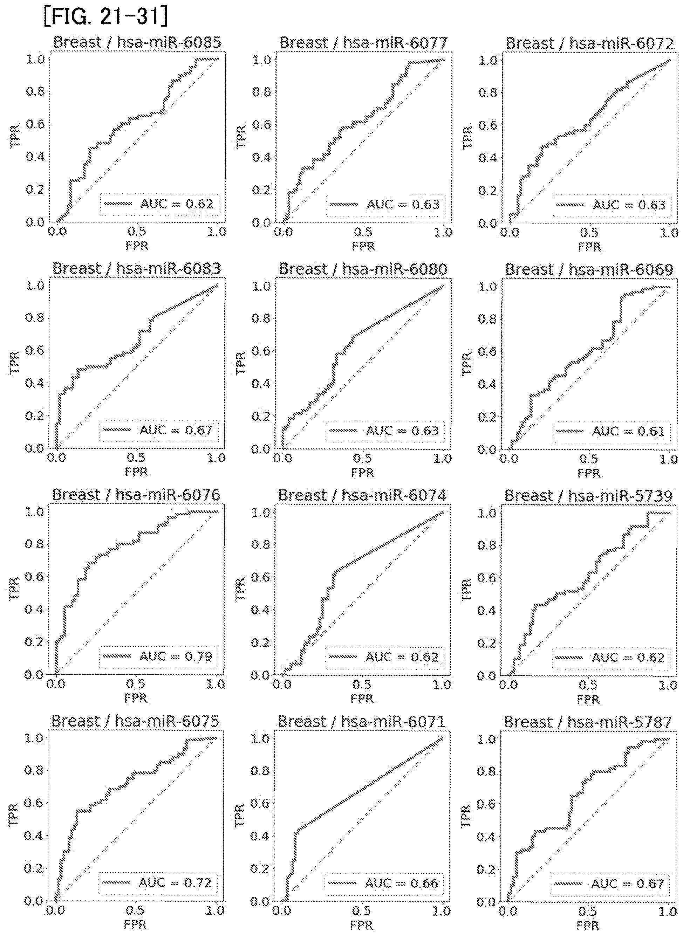

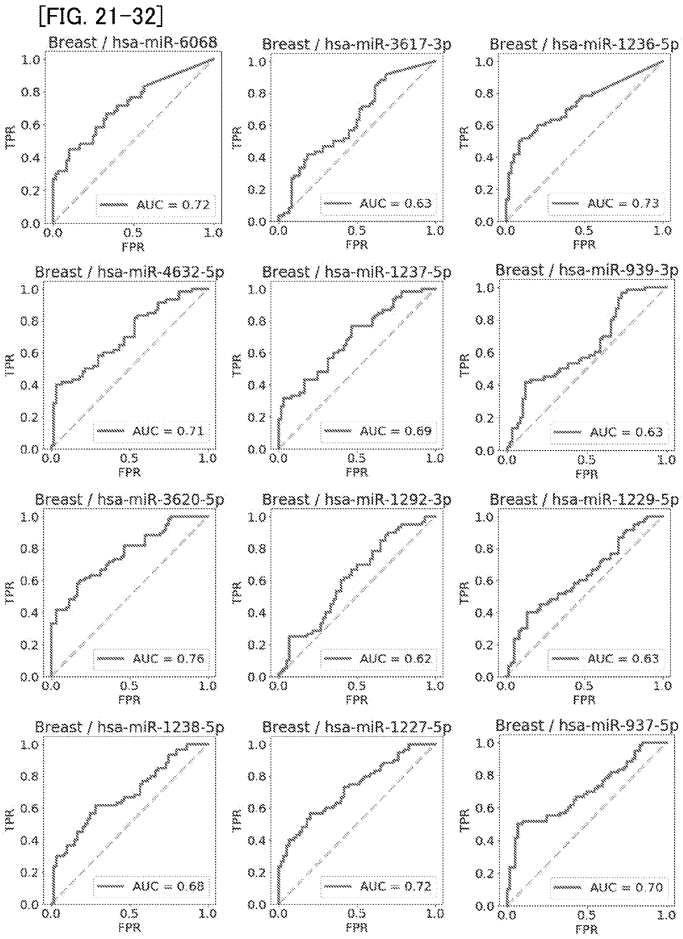

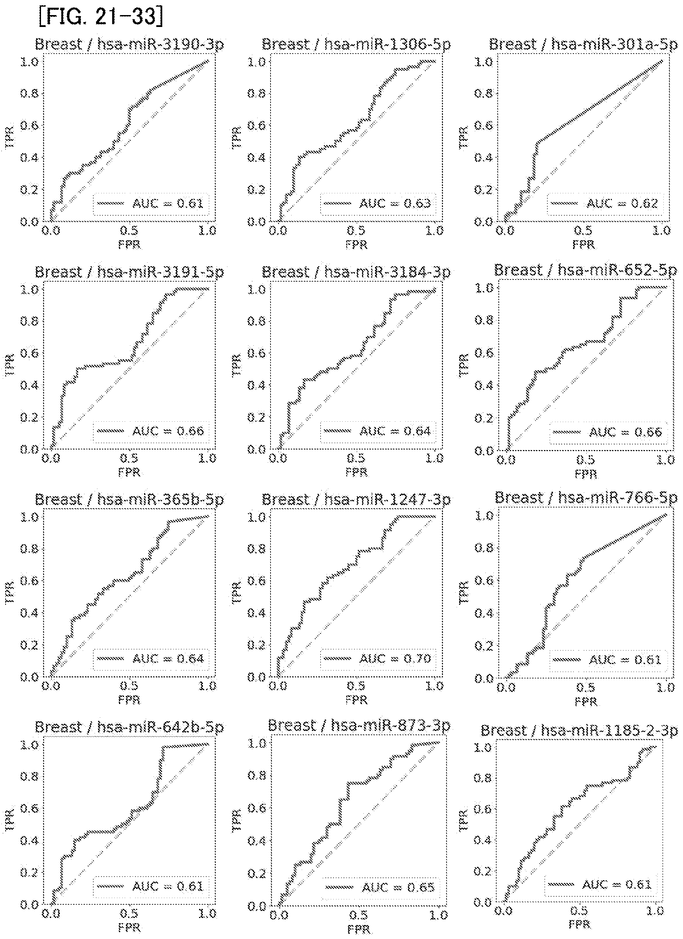

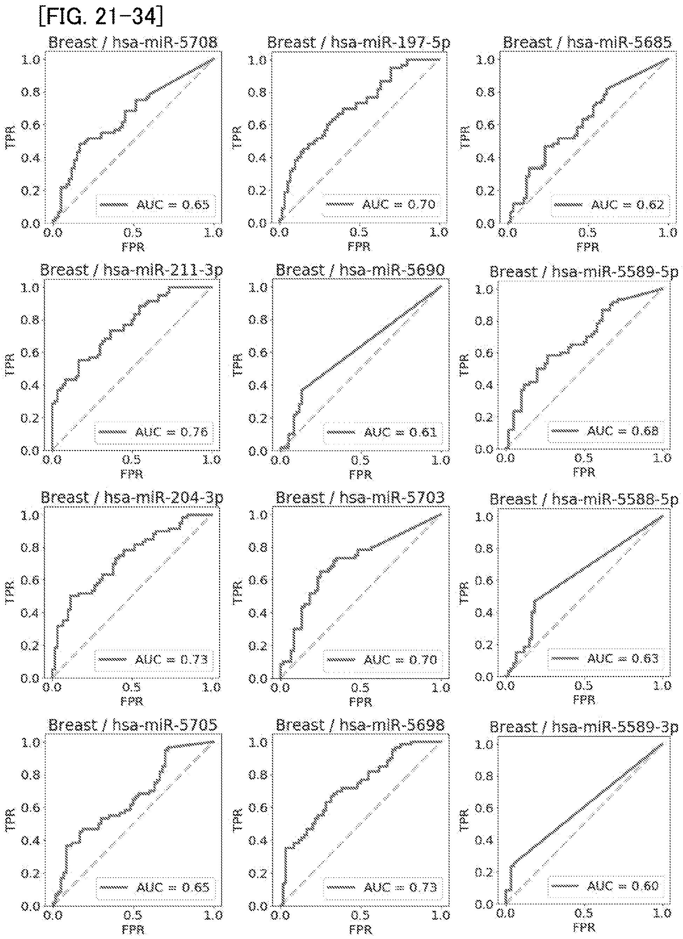

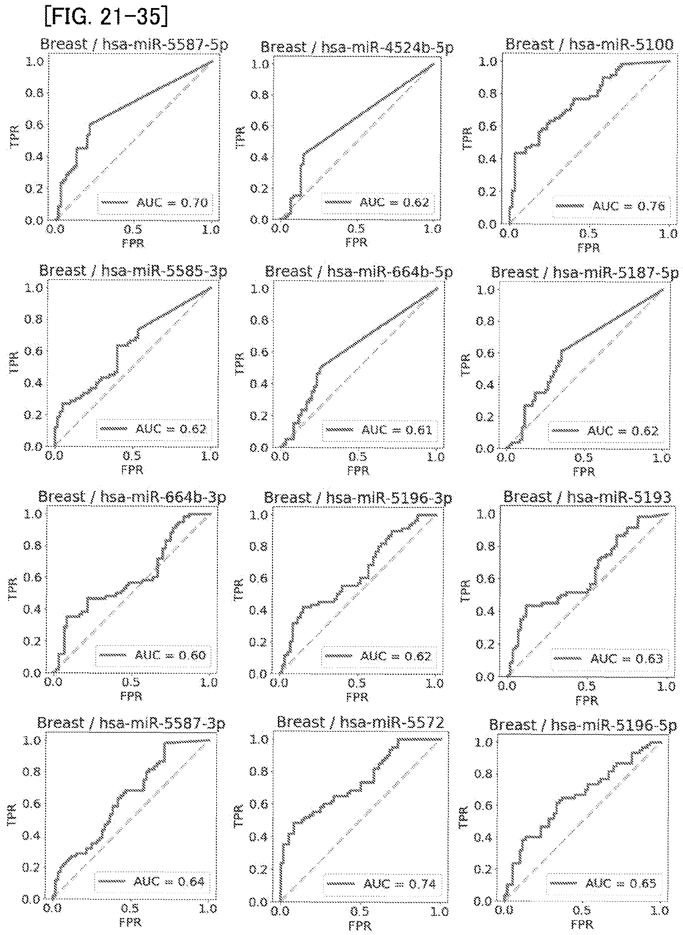

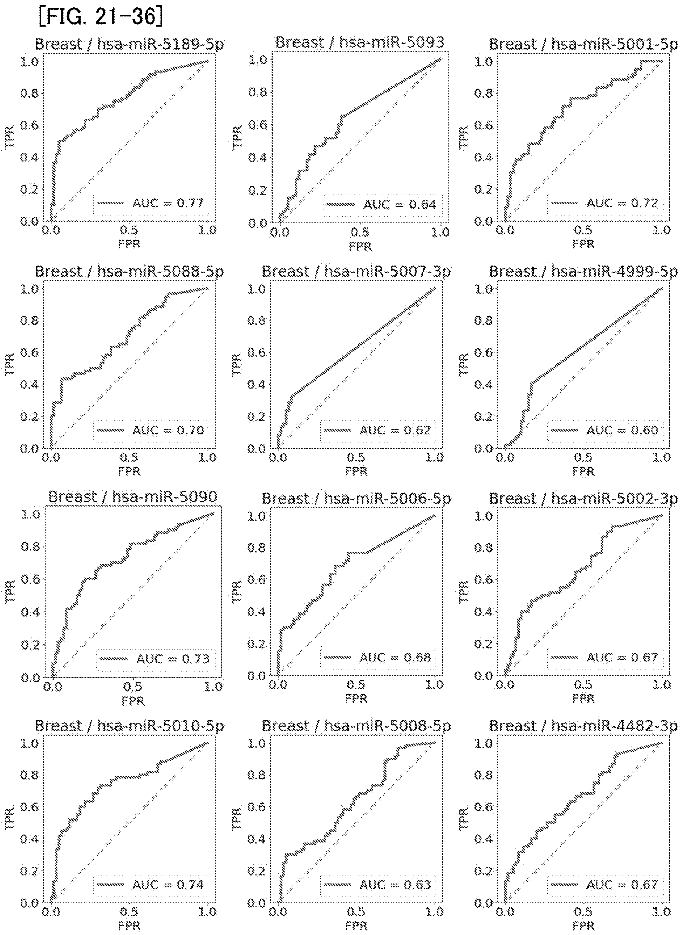

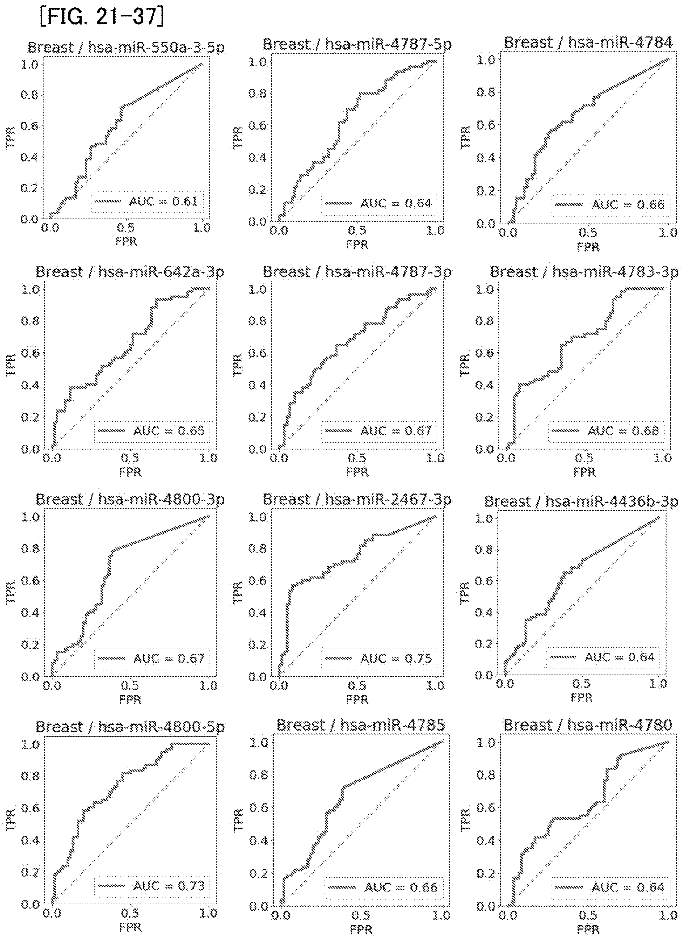

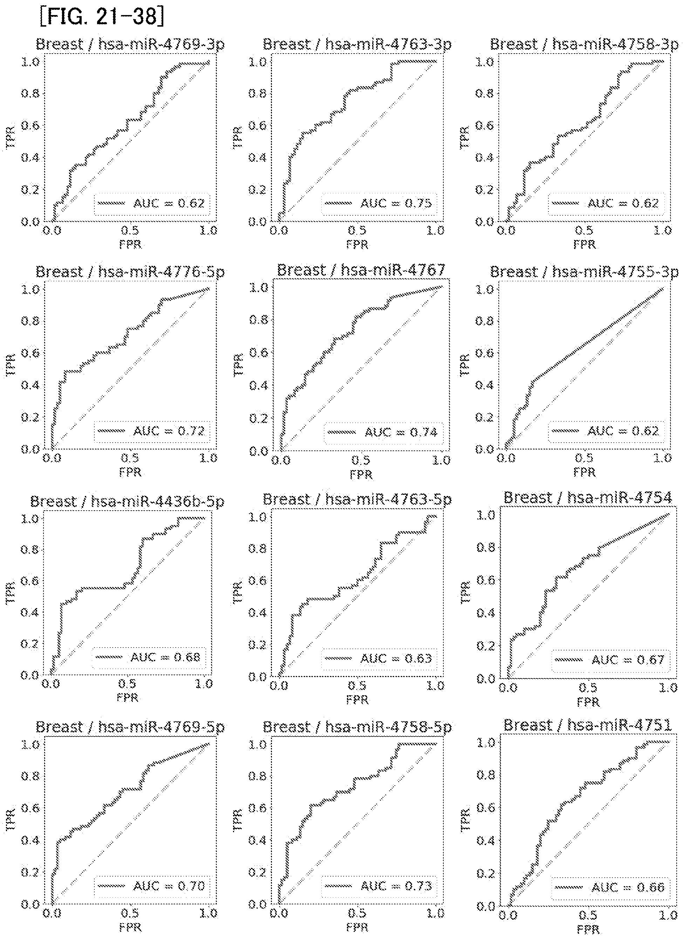

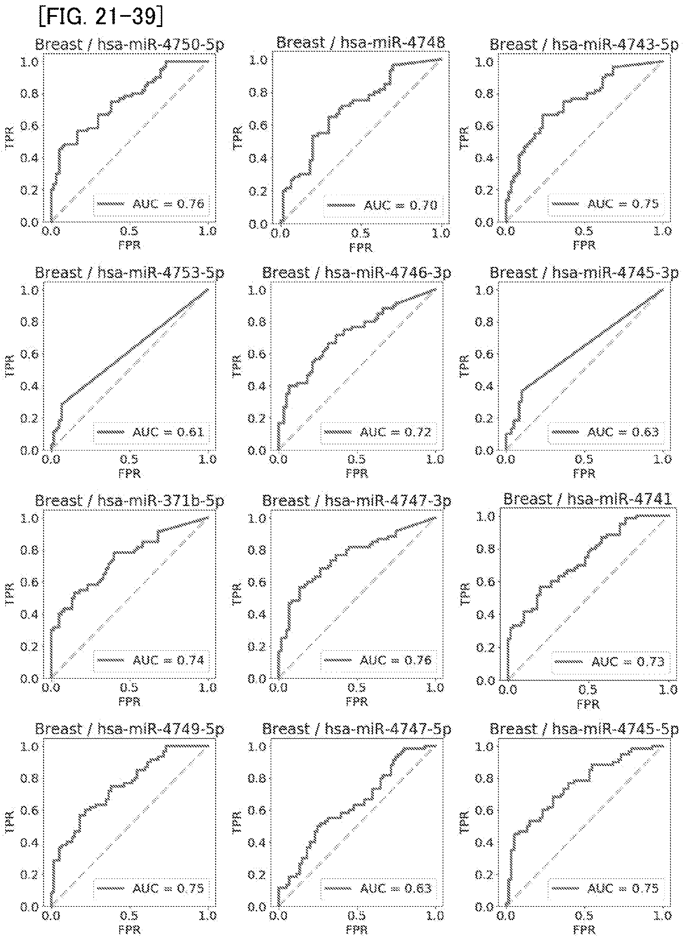

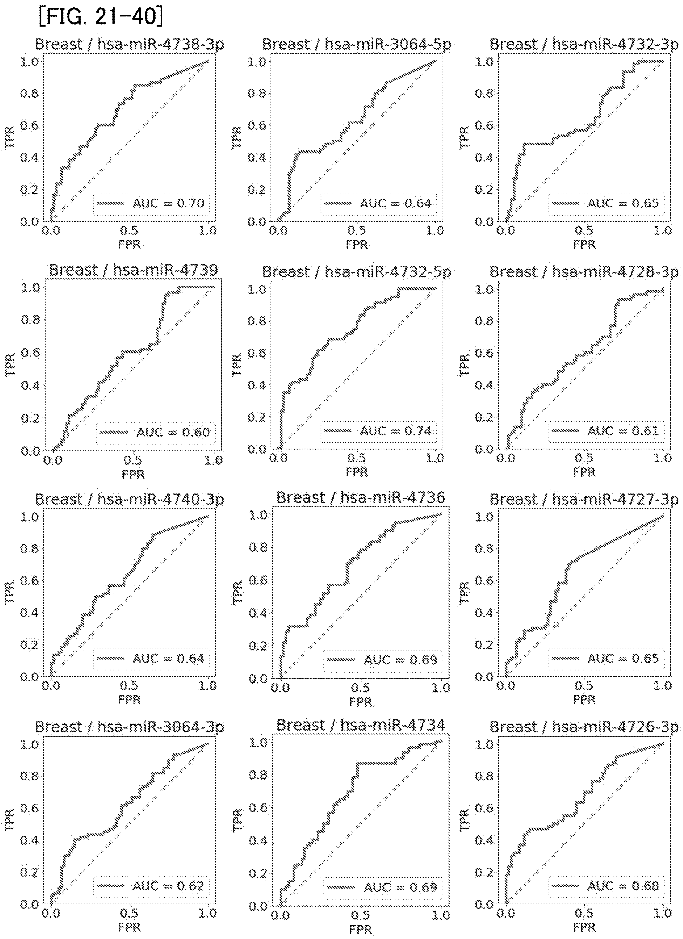

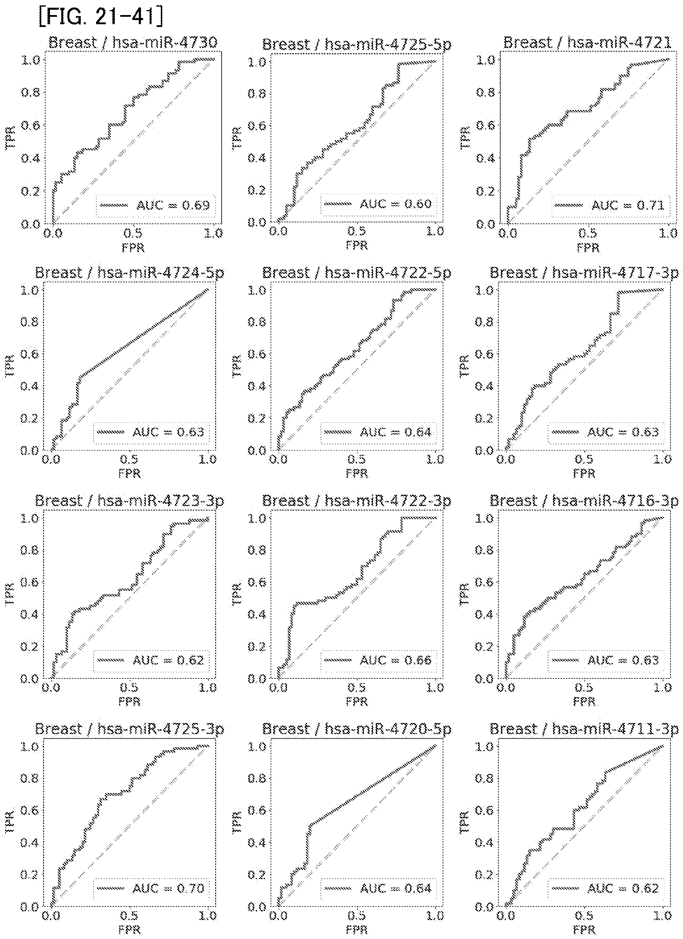

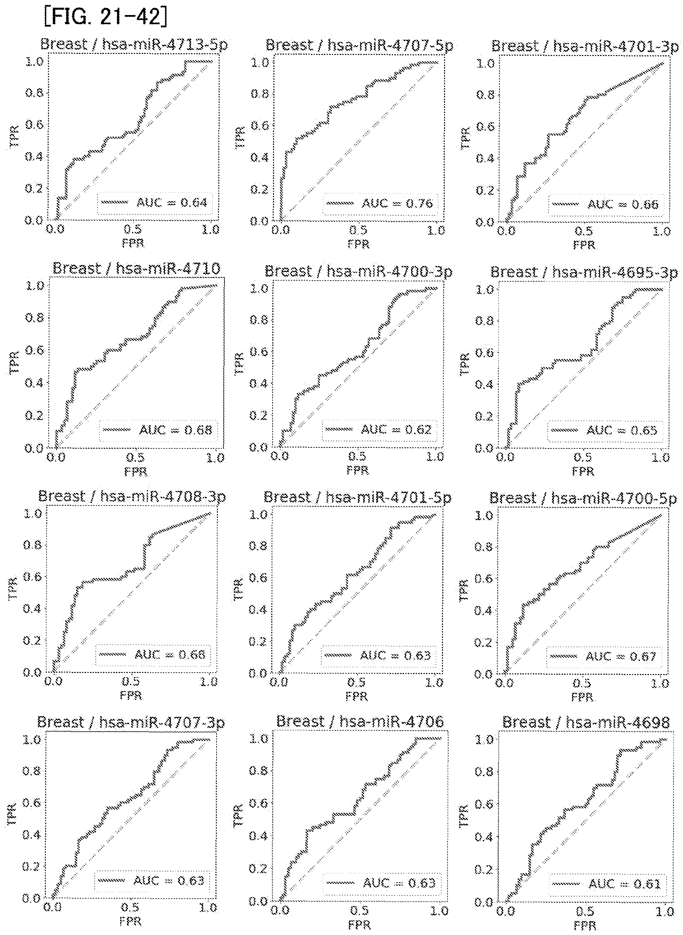

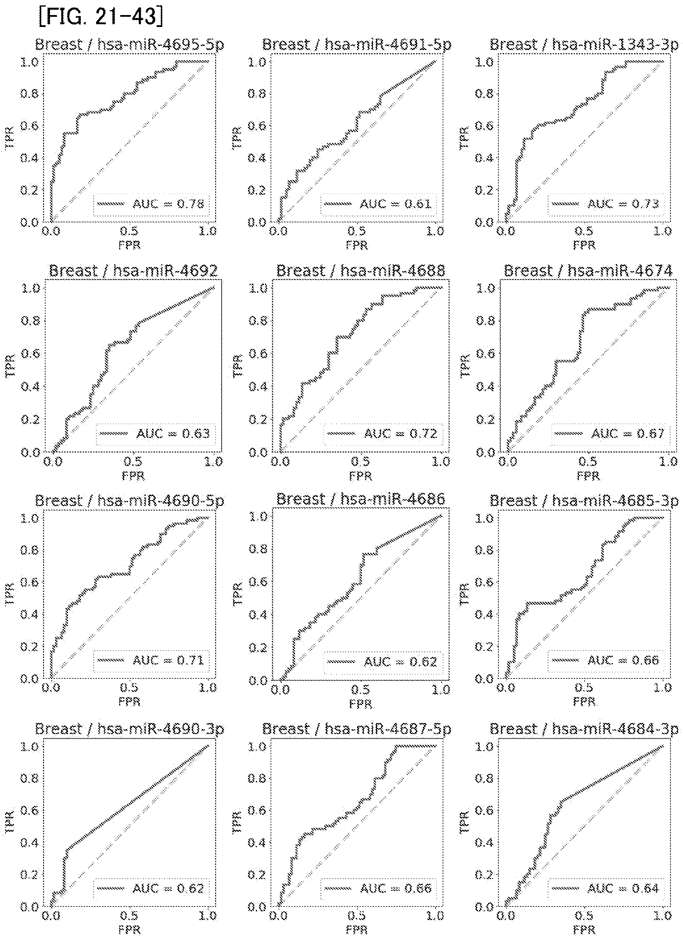

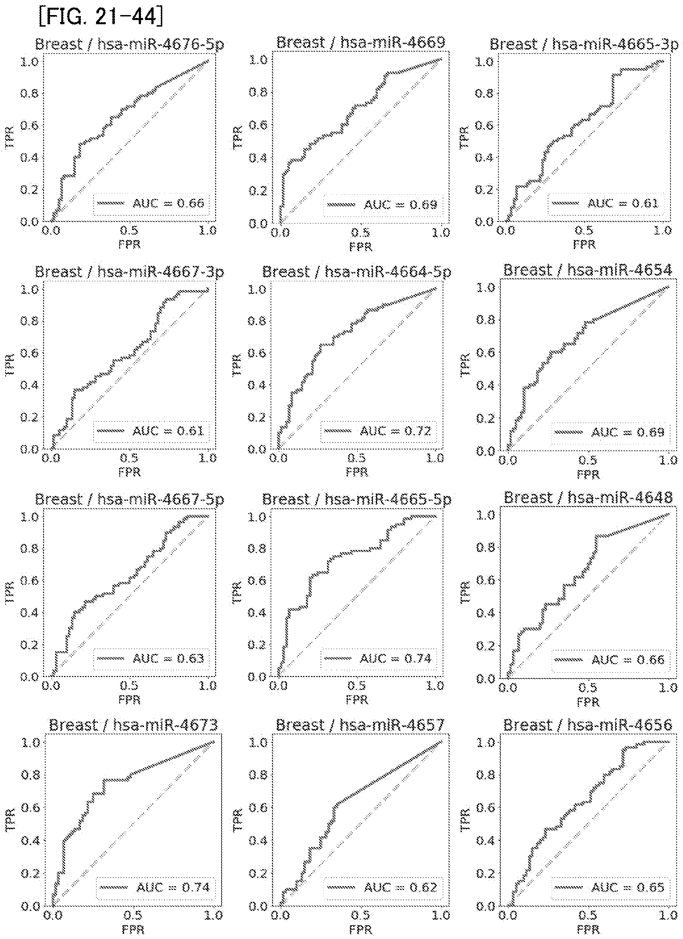

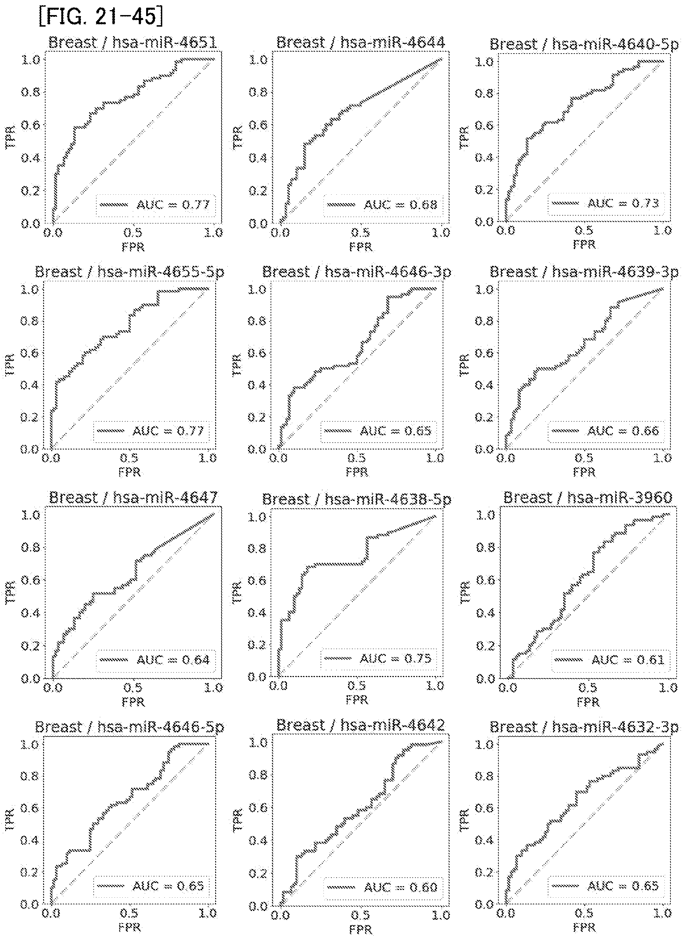

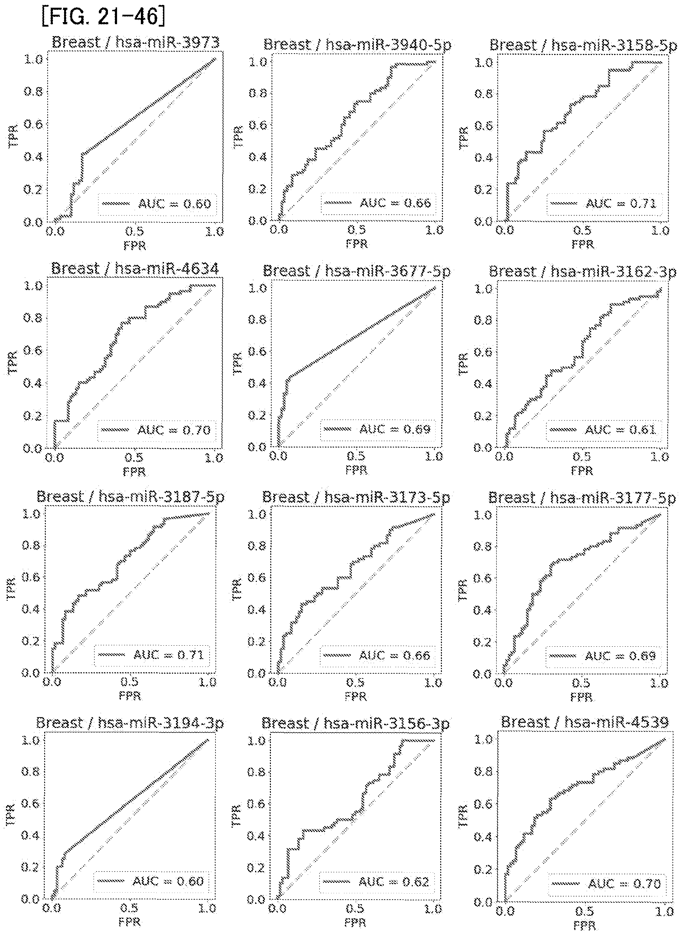

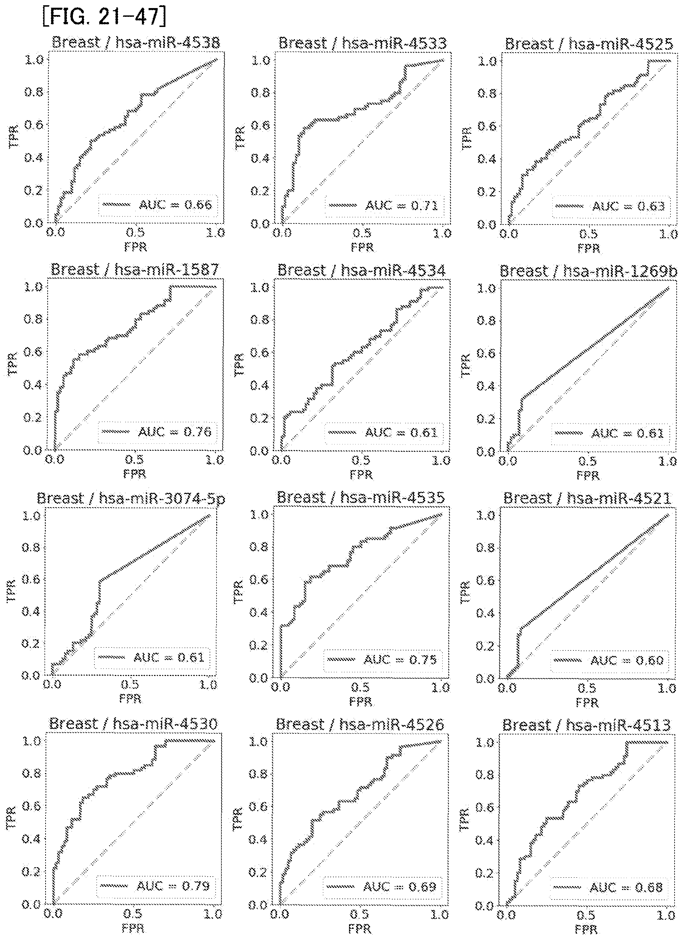

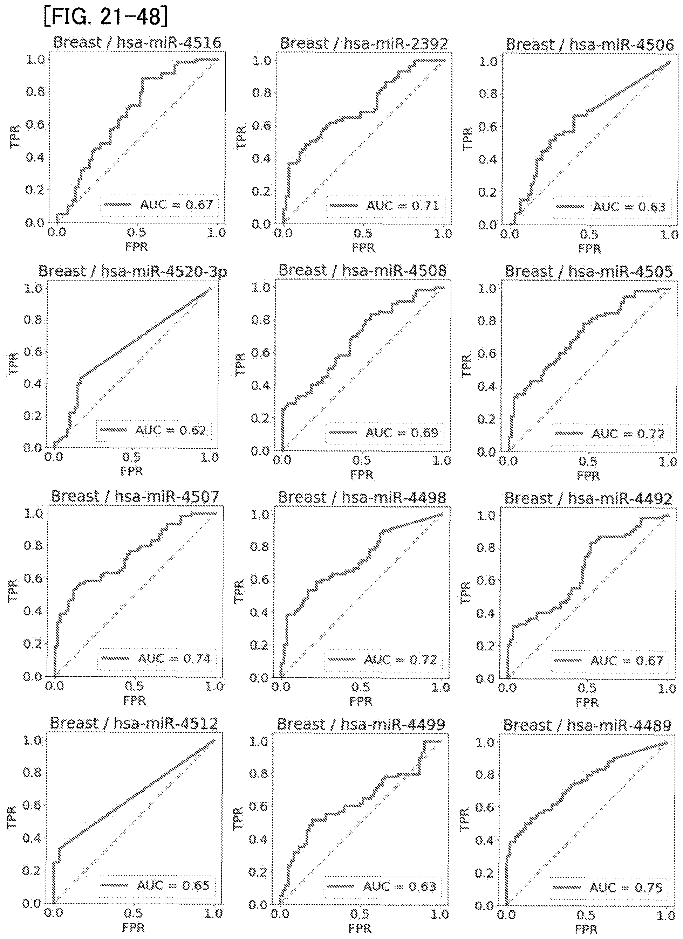

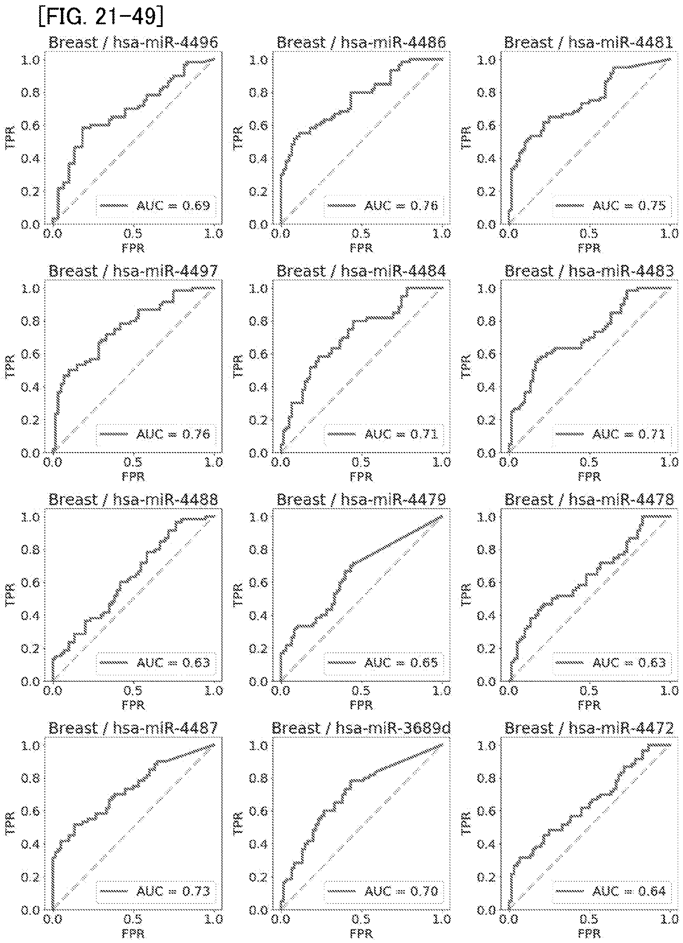

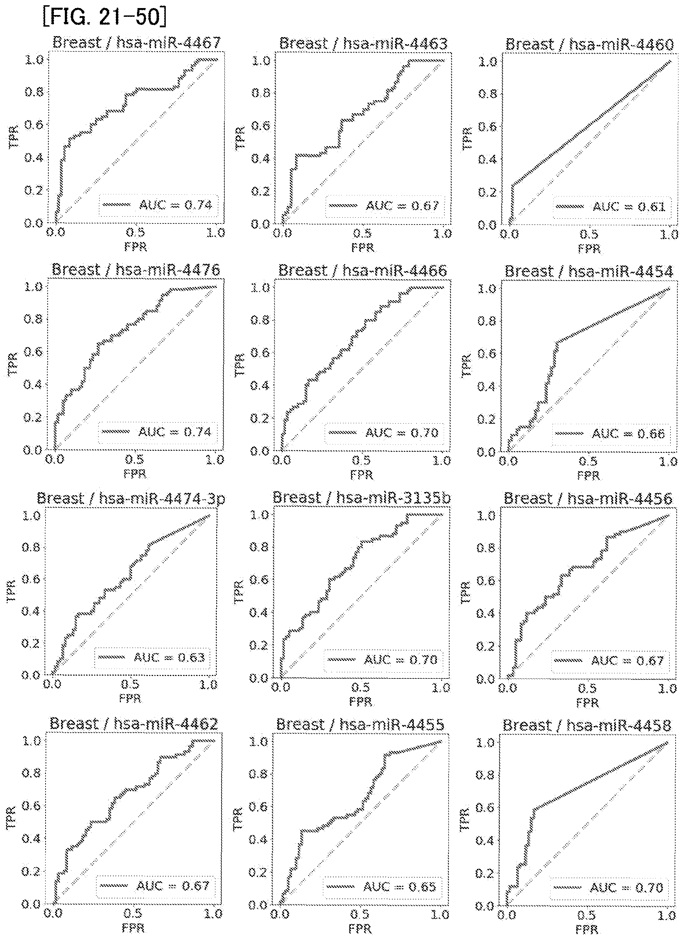

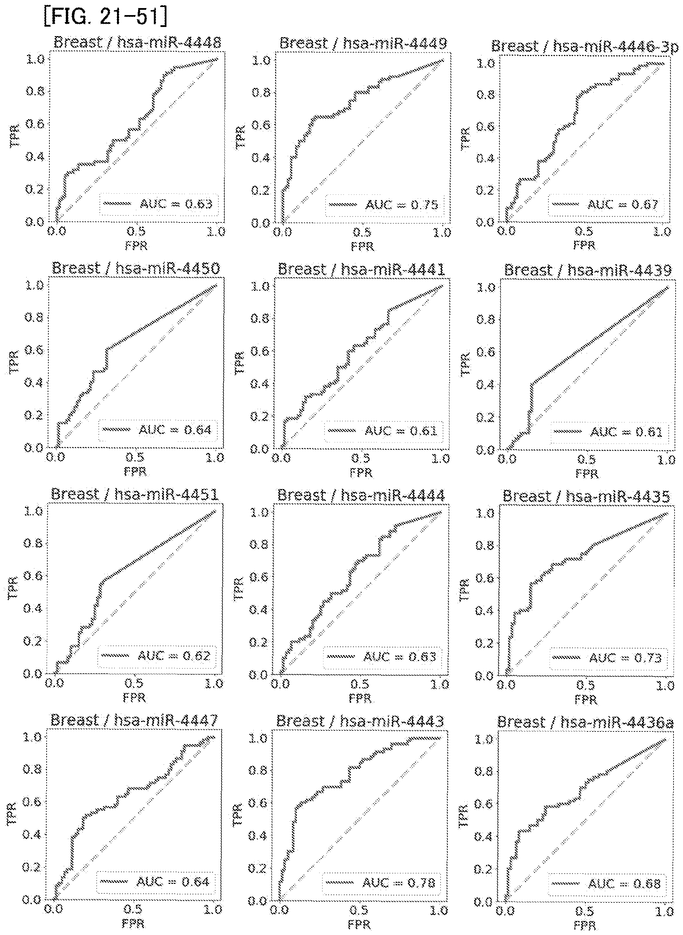

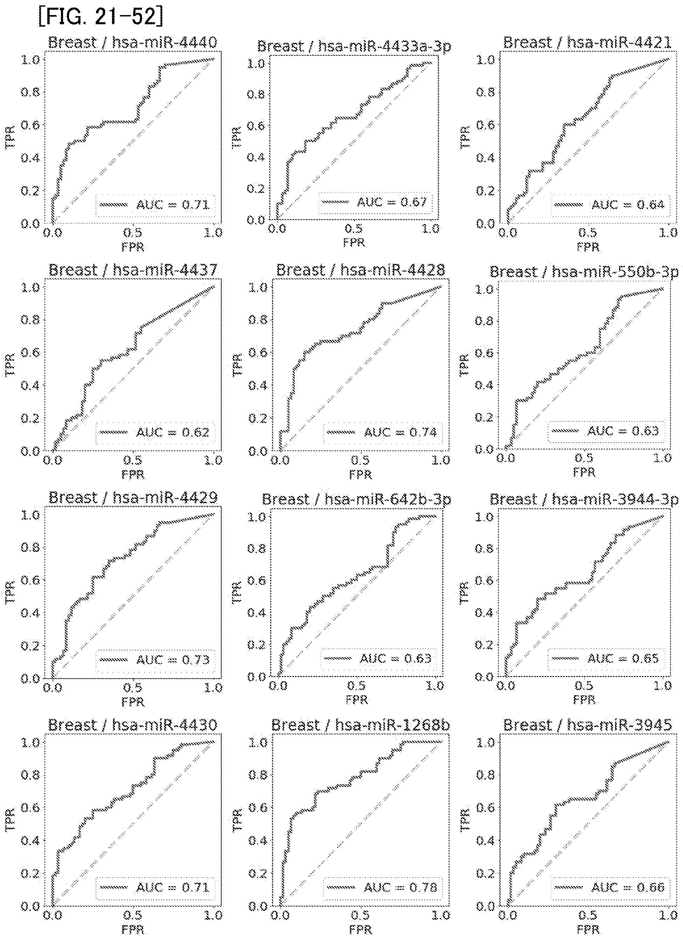

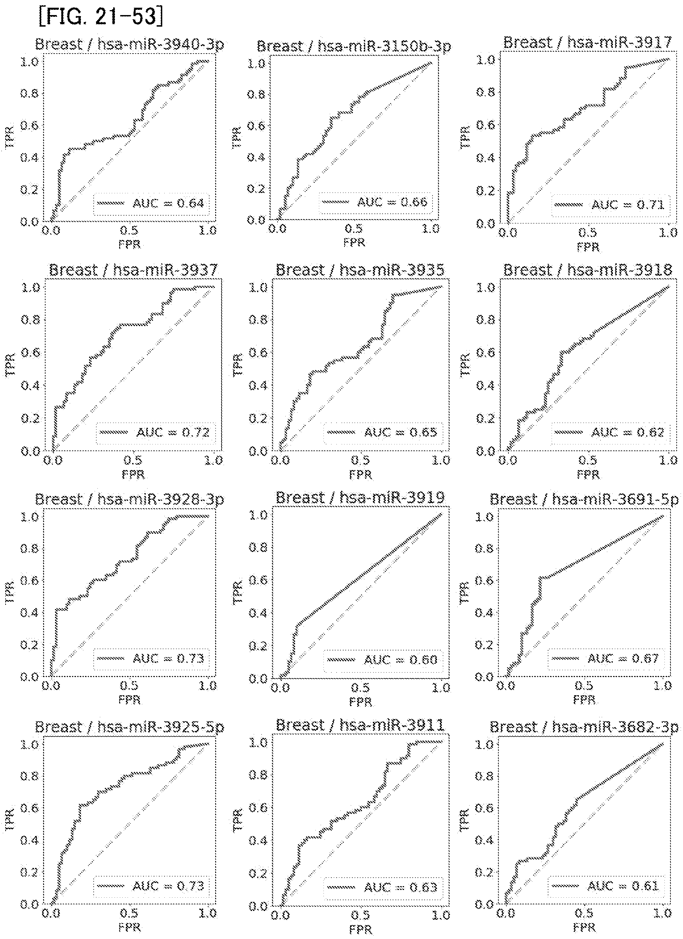

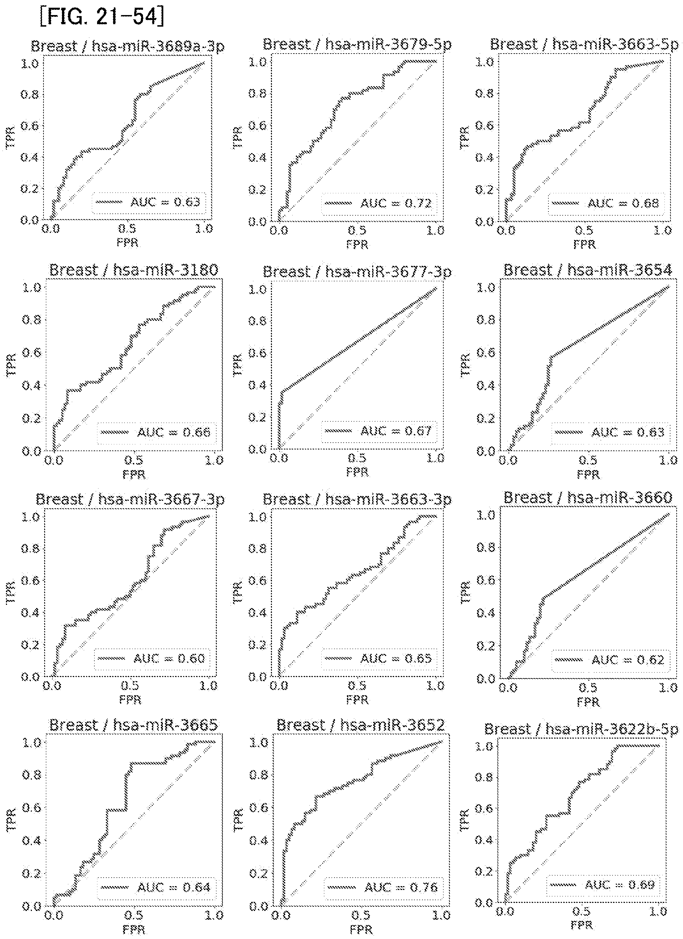

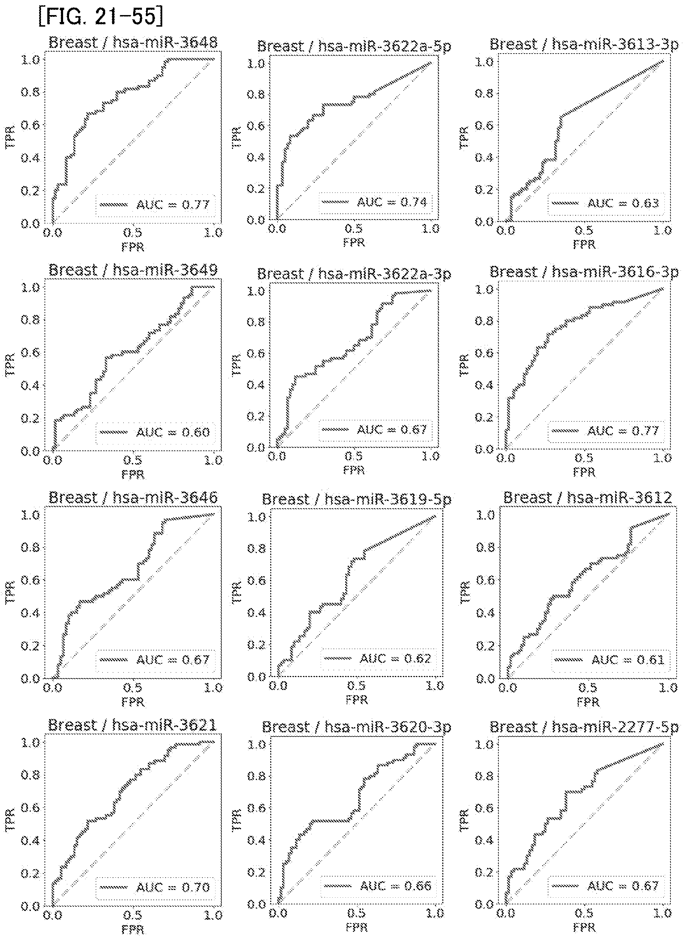

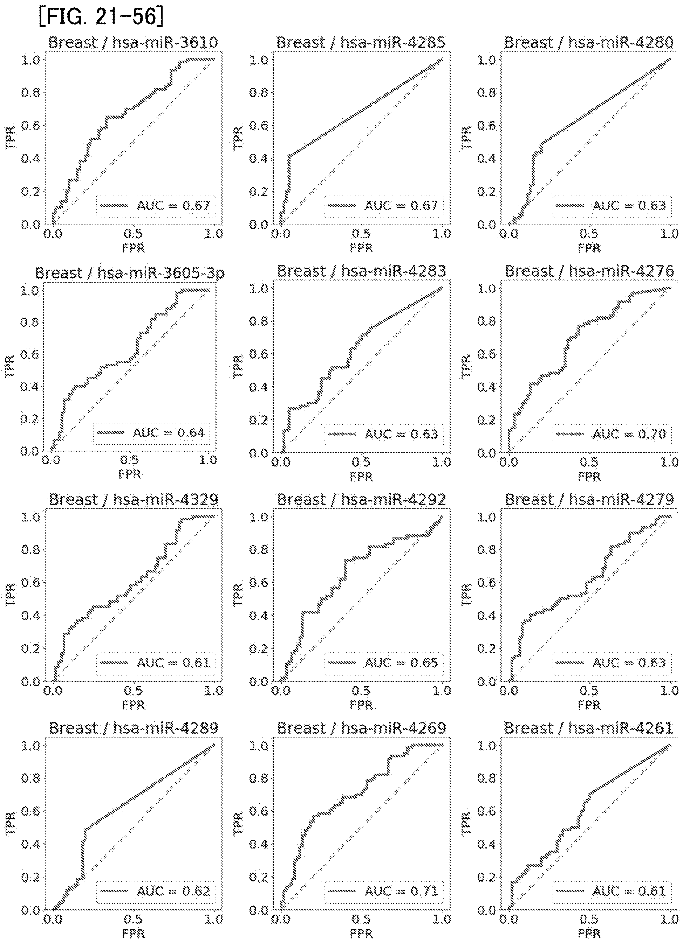

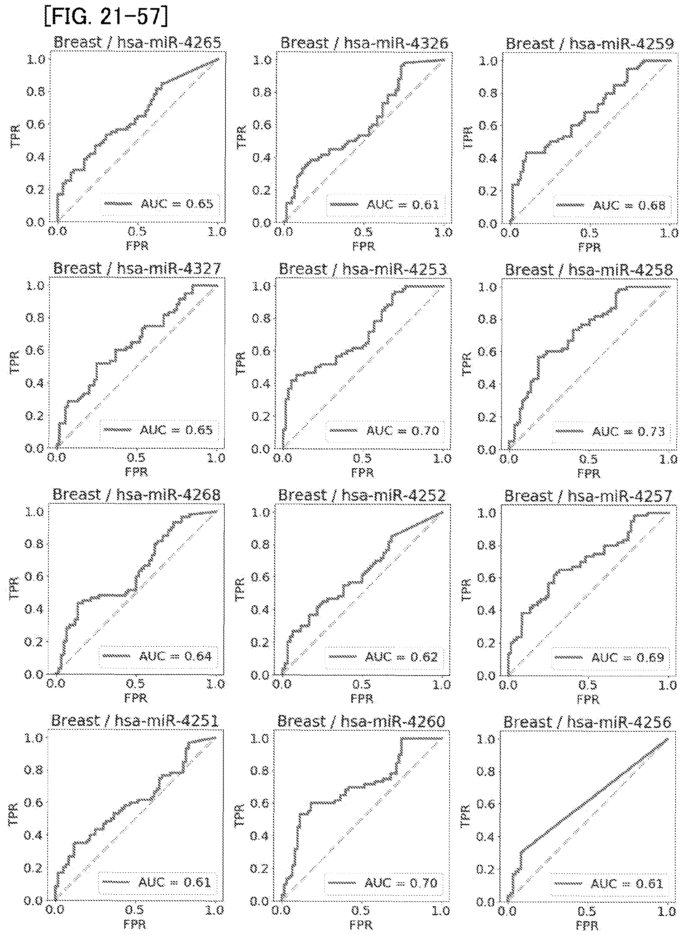

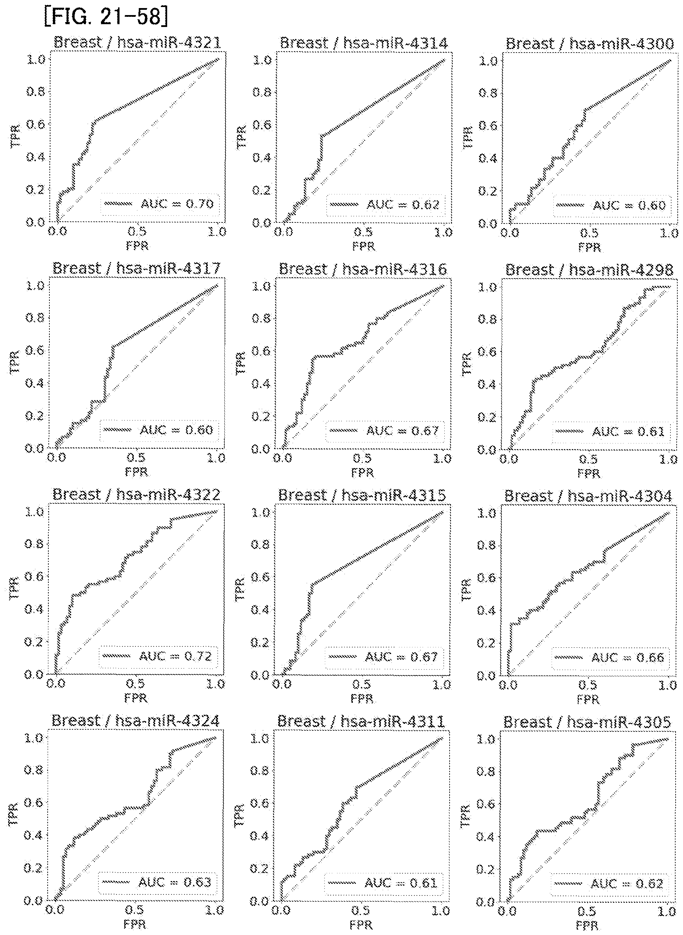

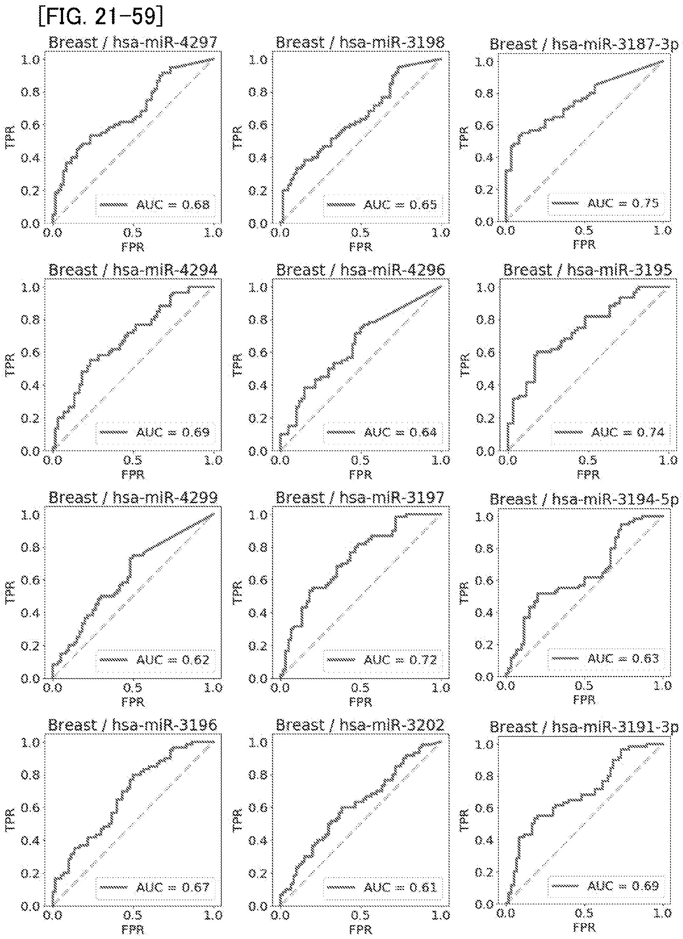

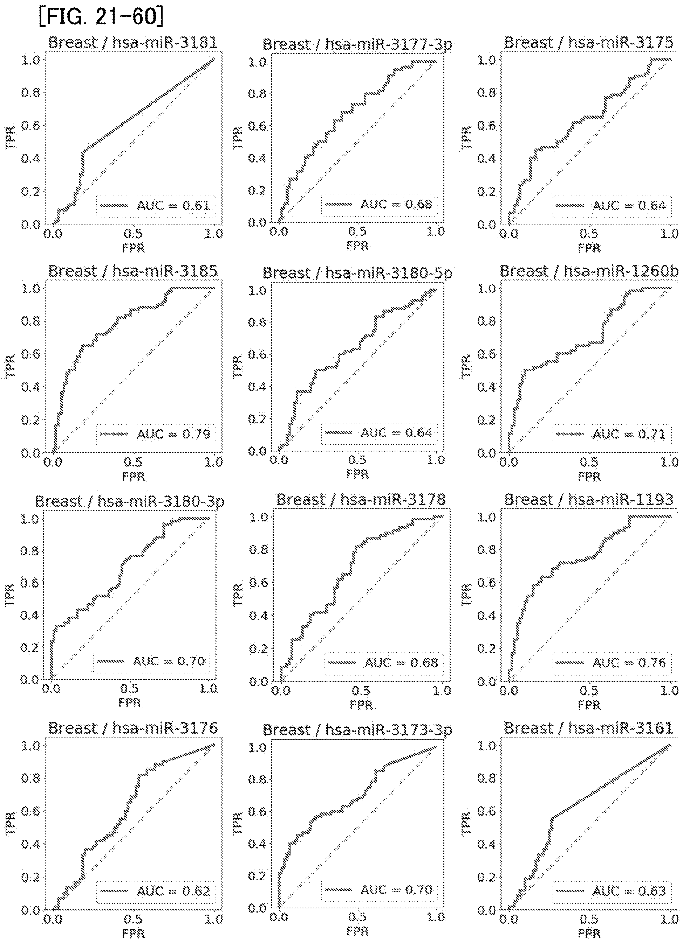

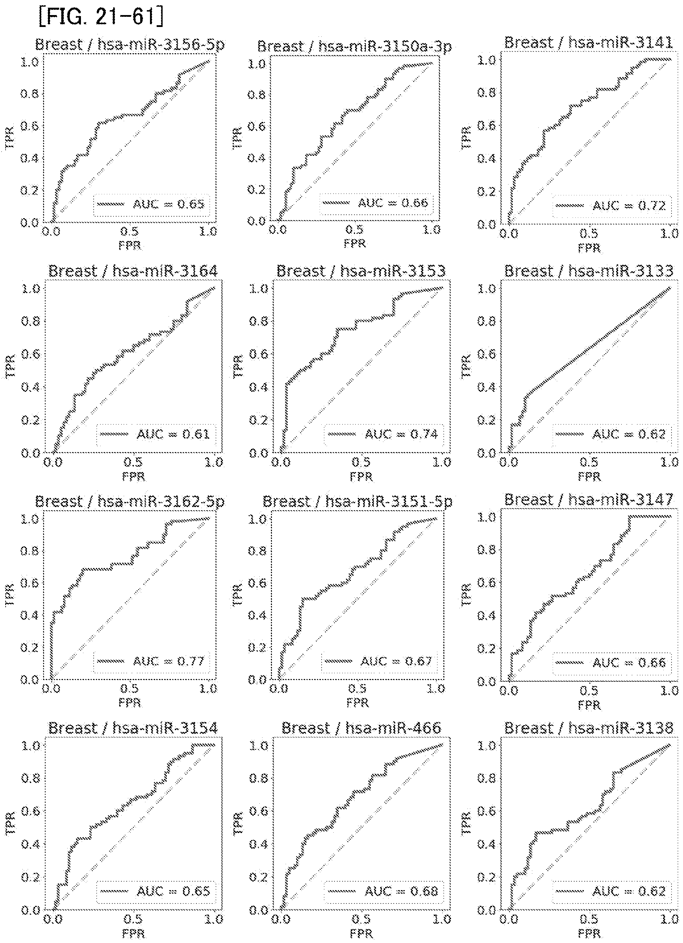

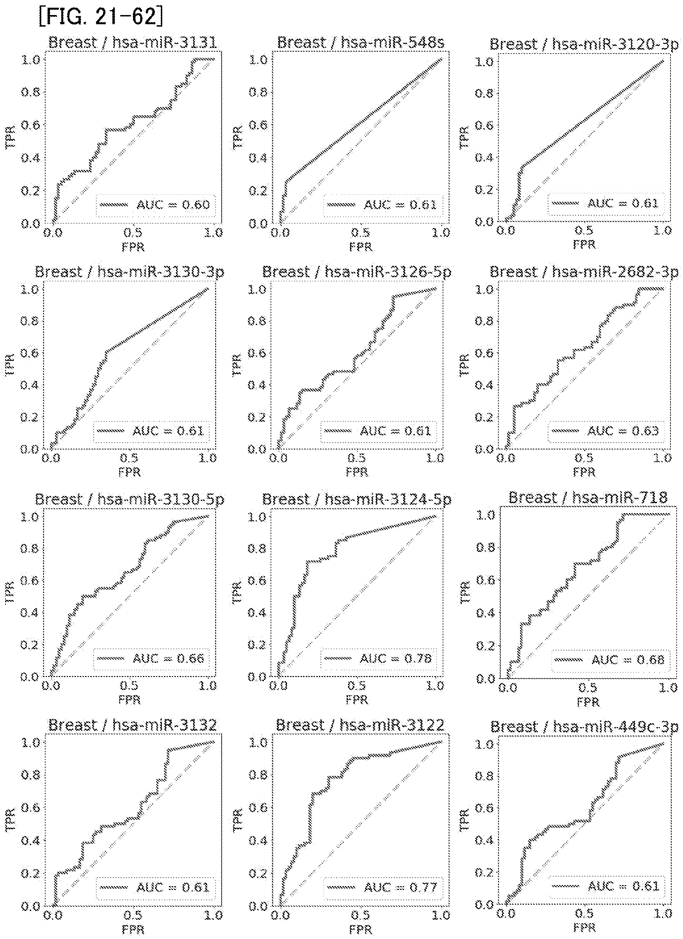

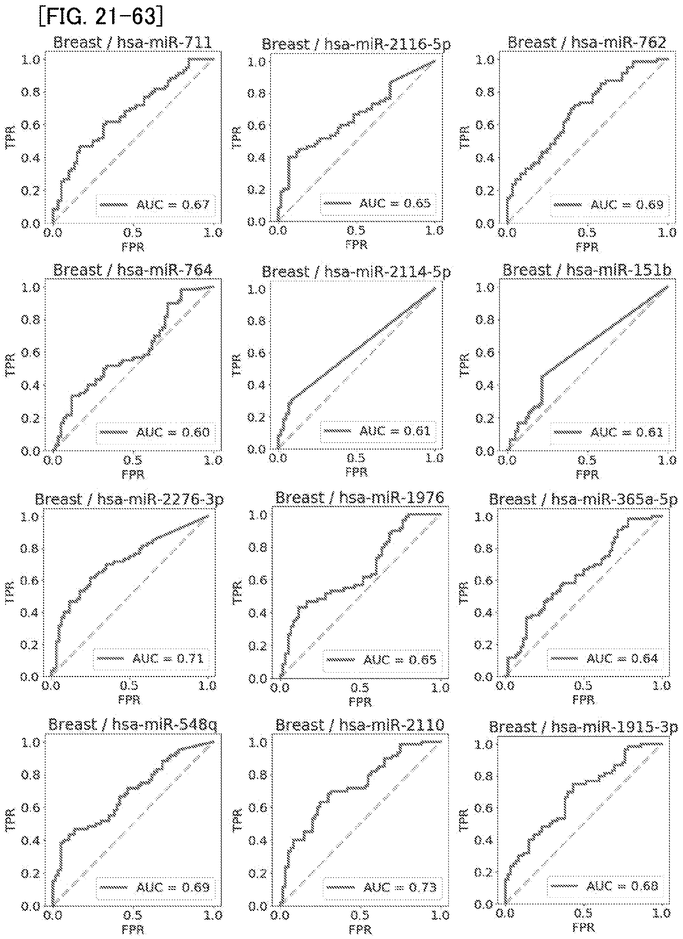

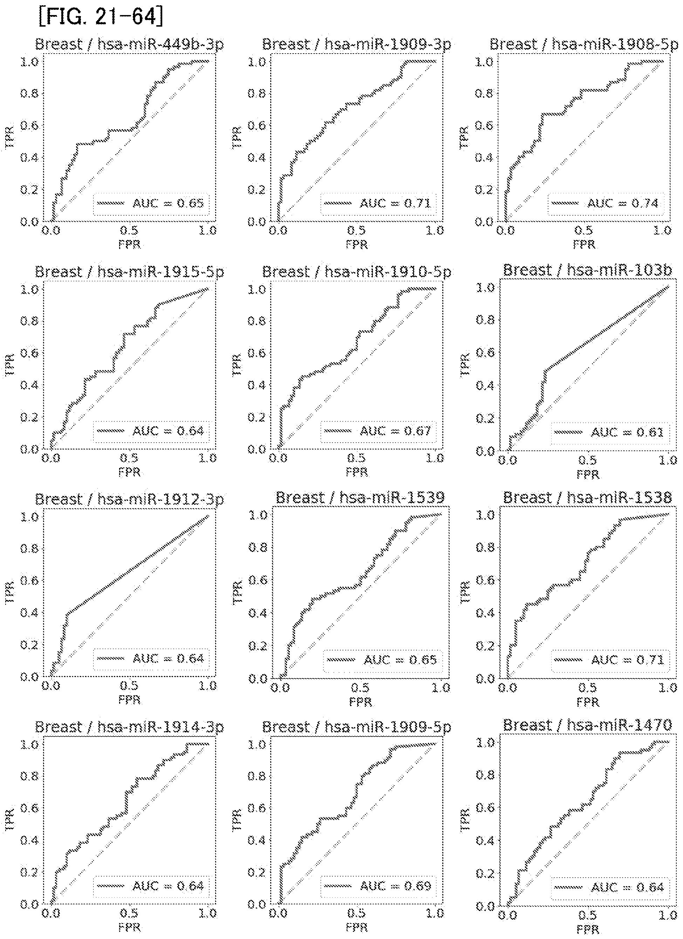

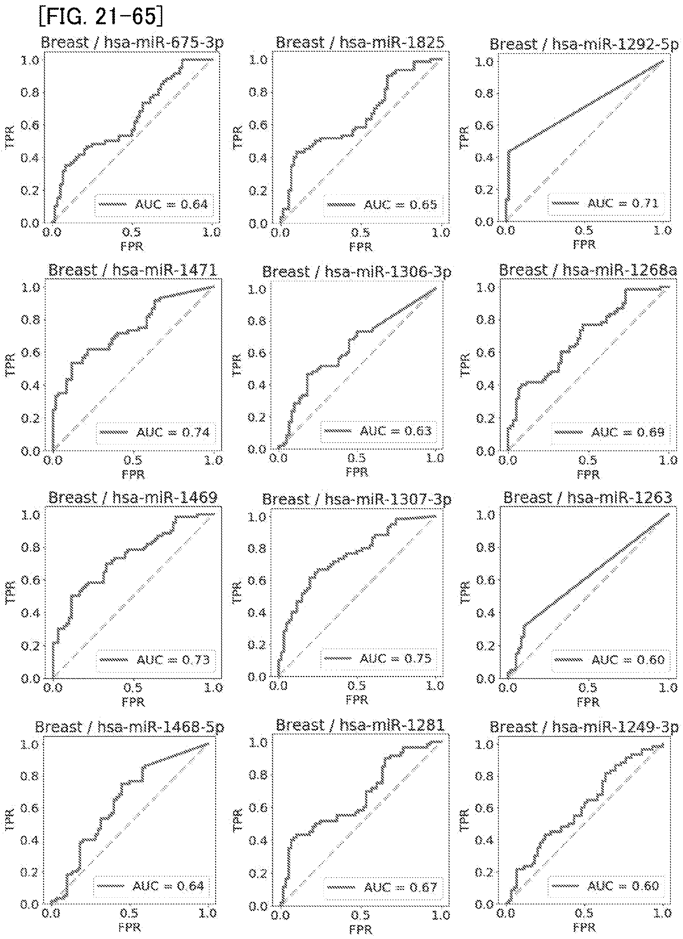

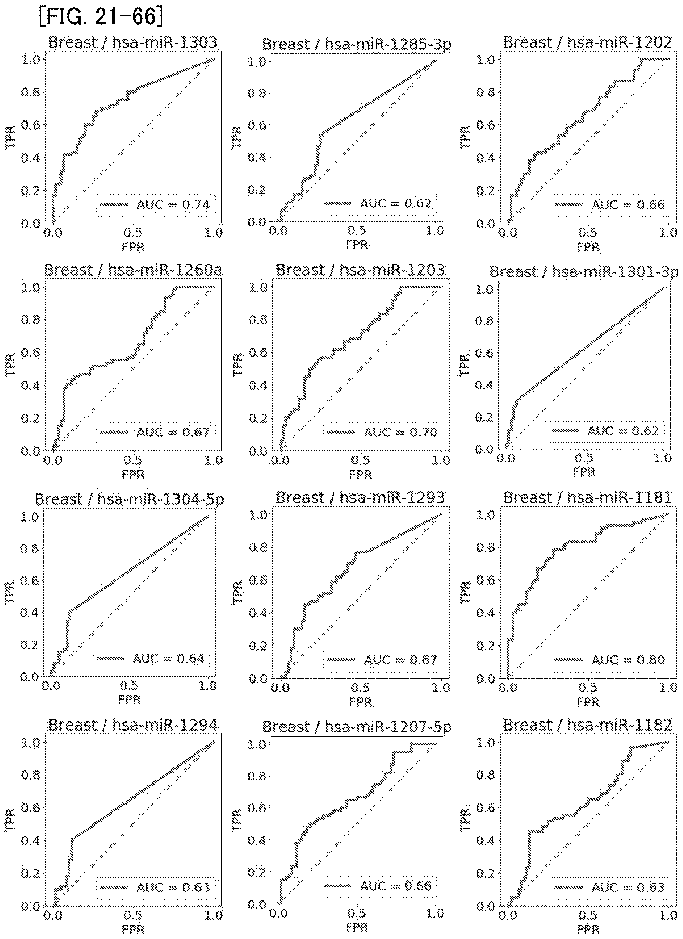

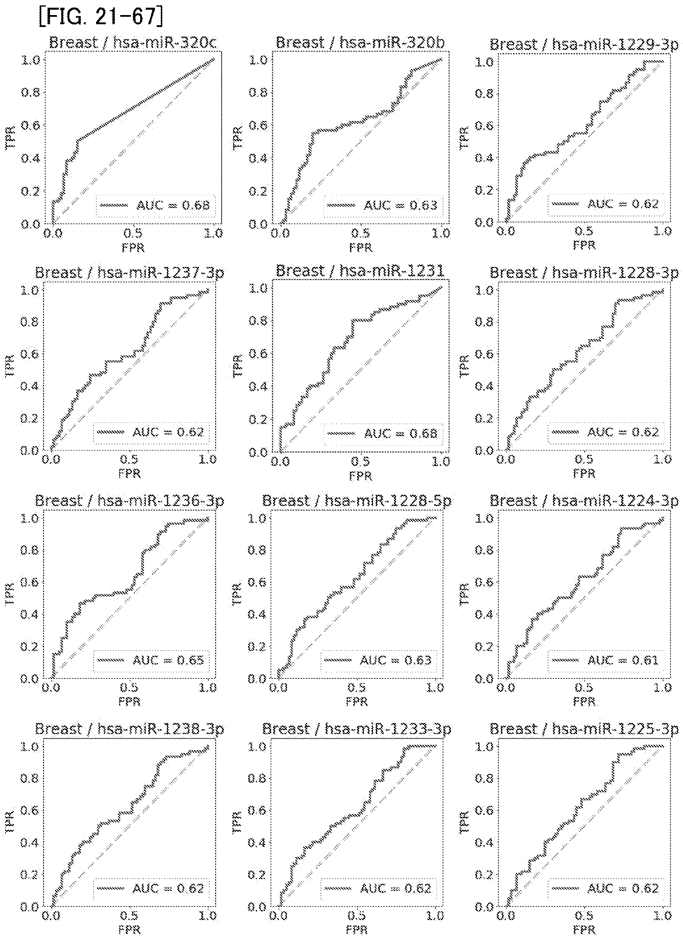

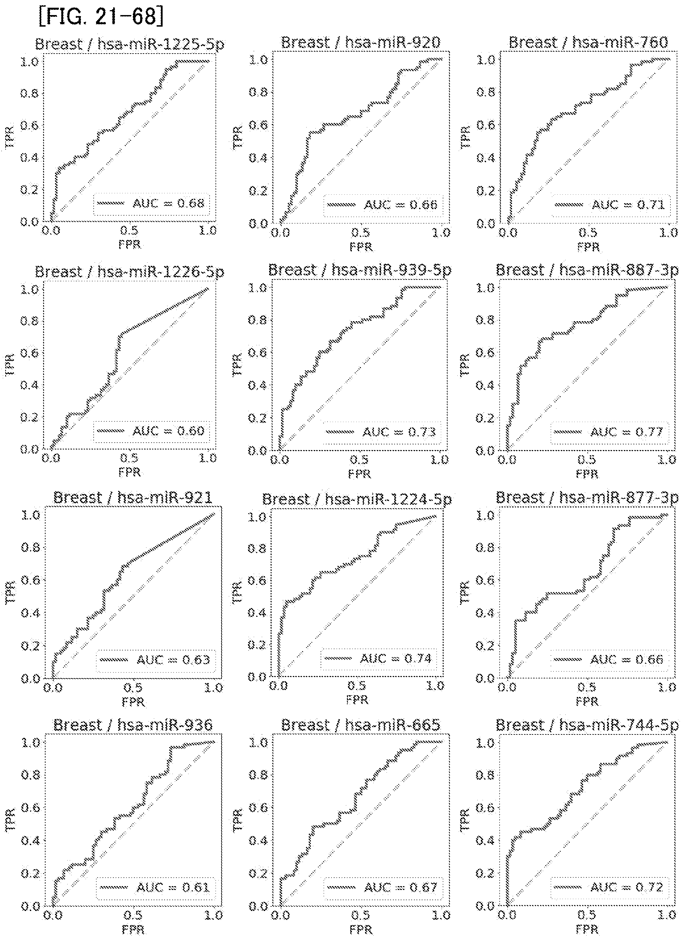

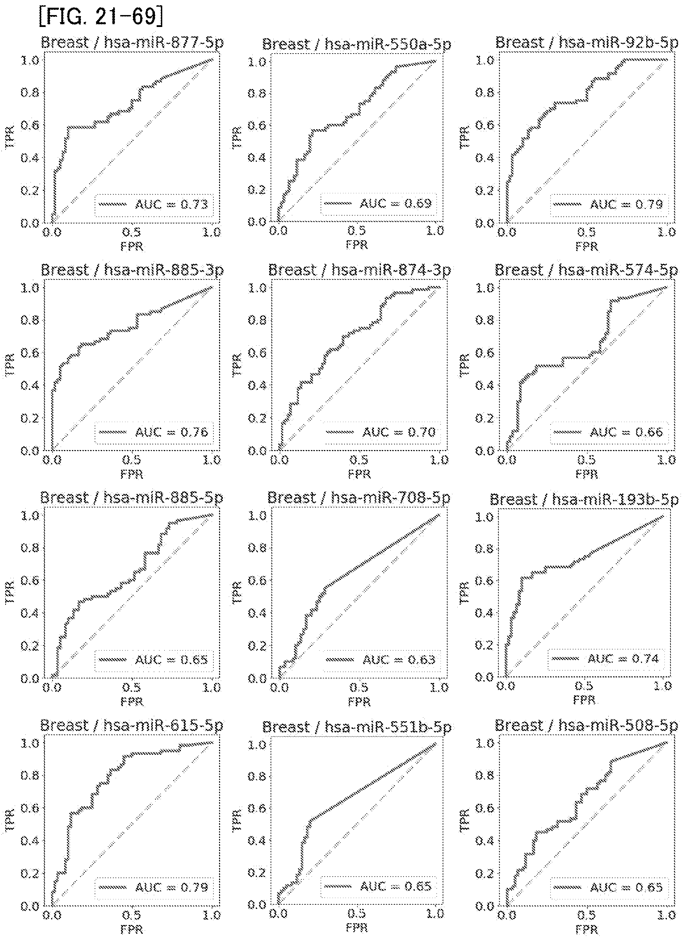

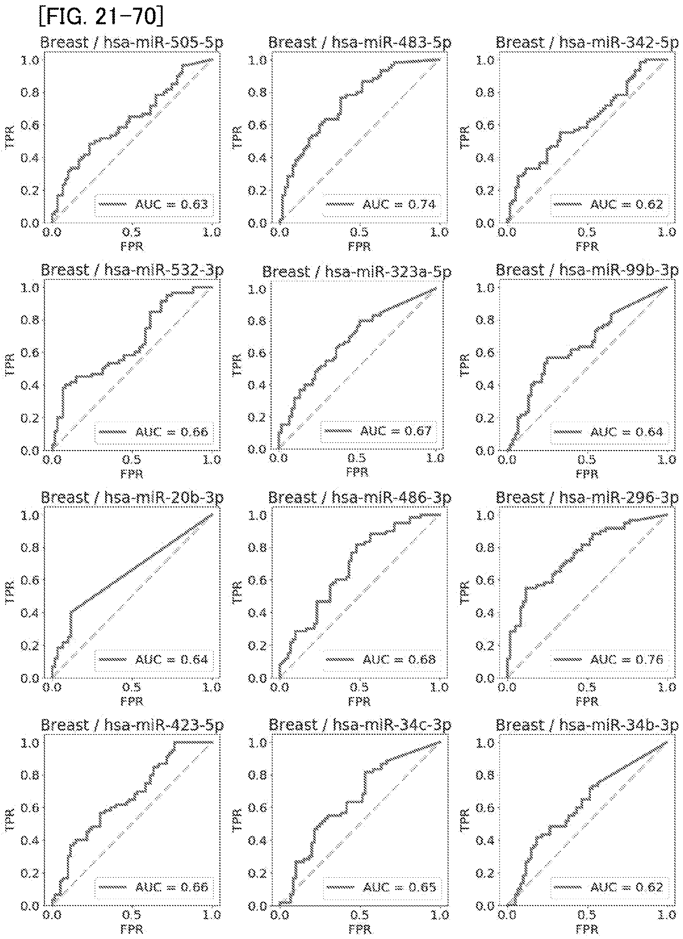

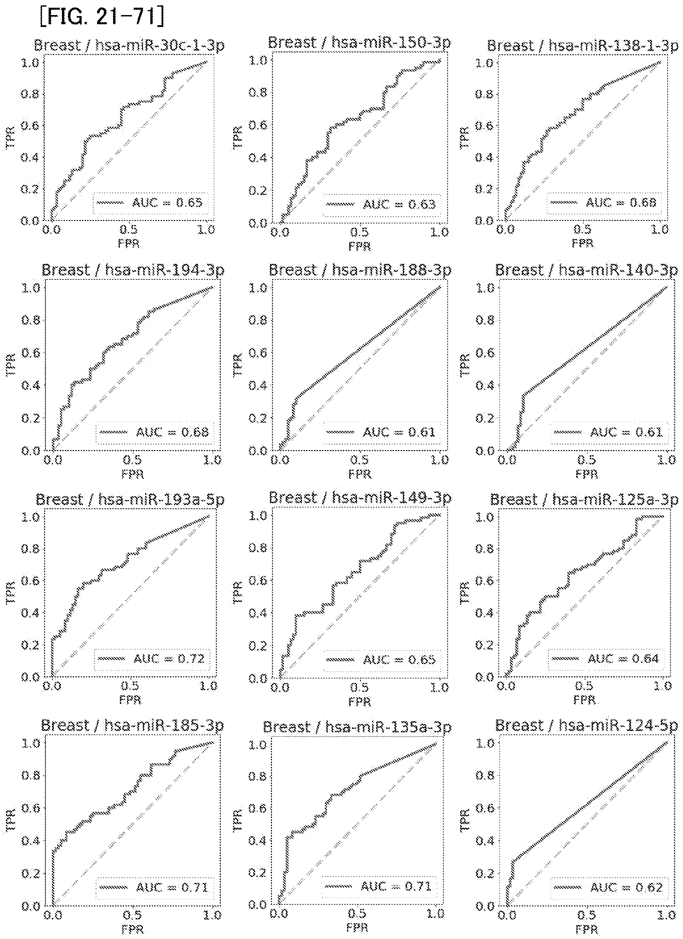

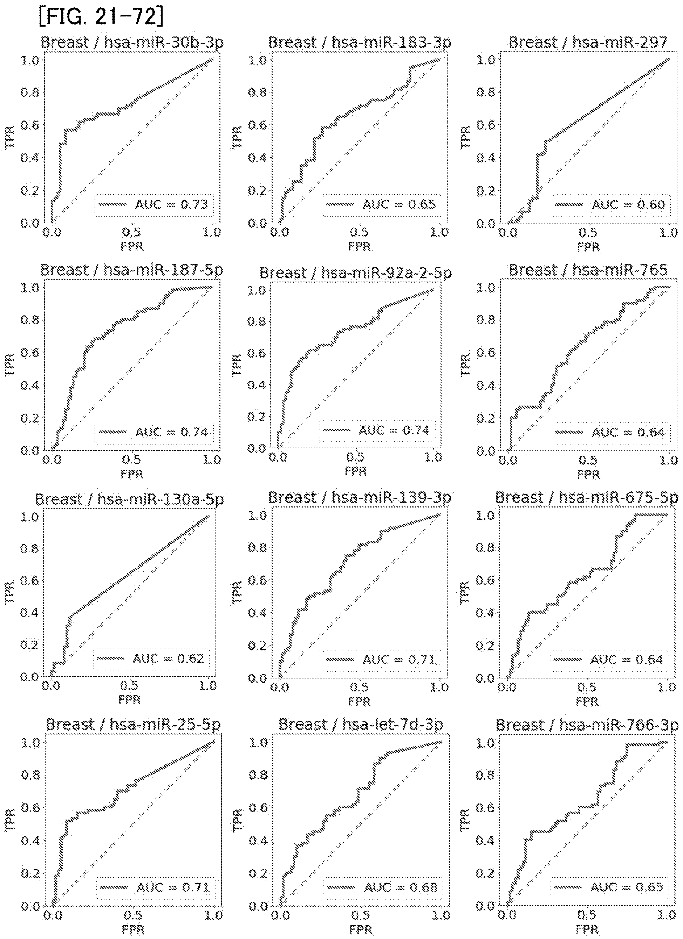

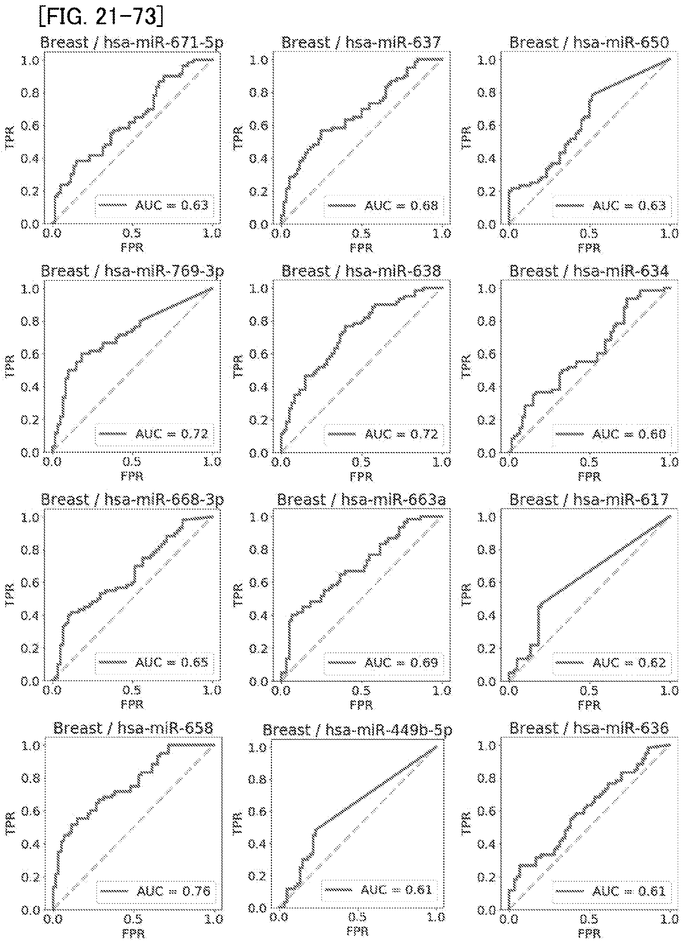

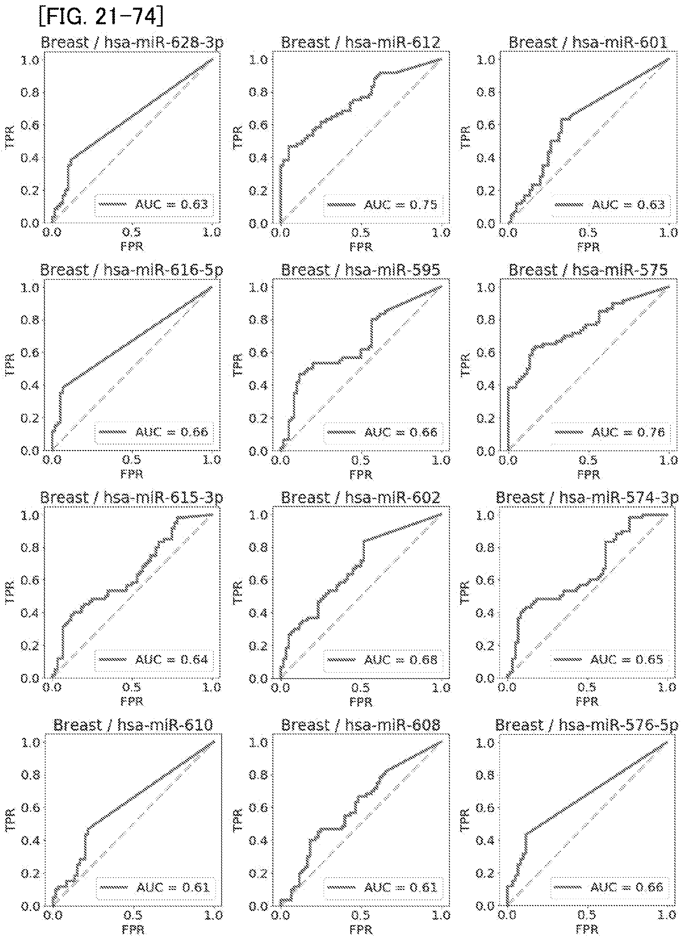

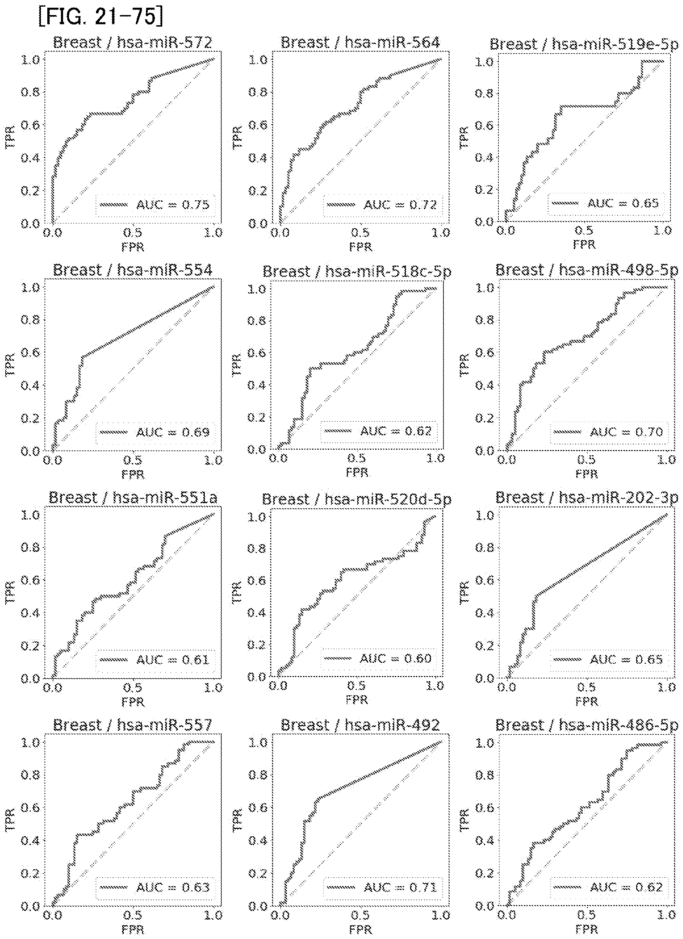

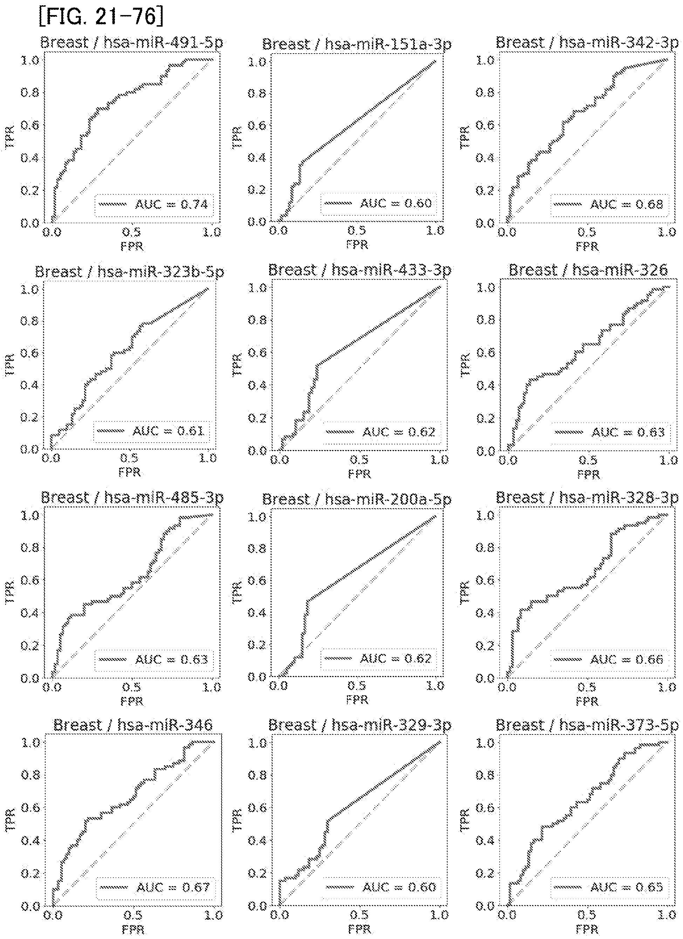

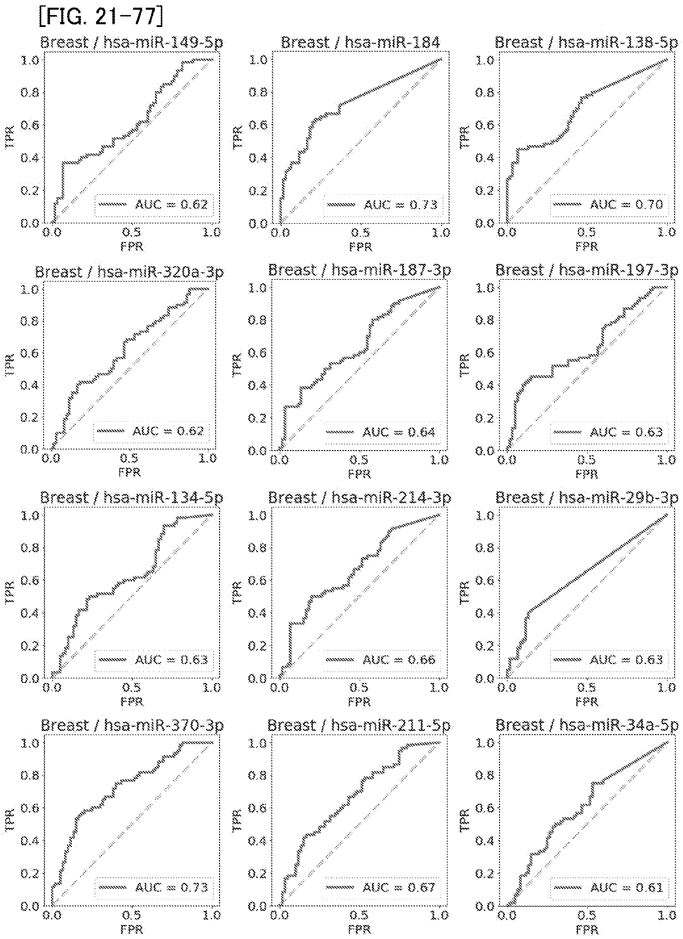

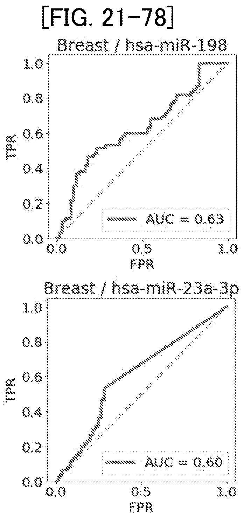

FIGS. 21-1 to 21-78 show ROCs curves for predicting human breast cancer using a single miRNA.

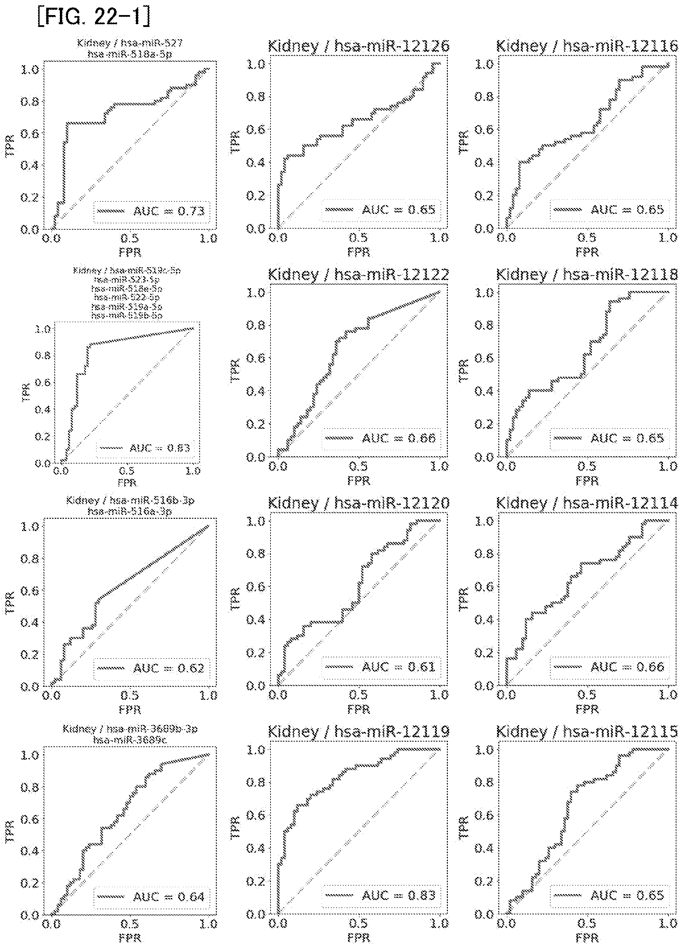

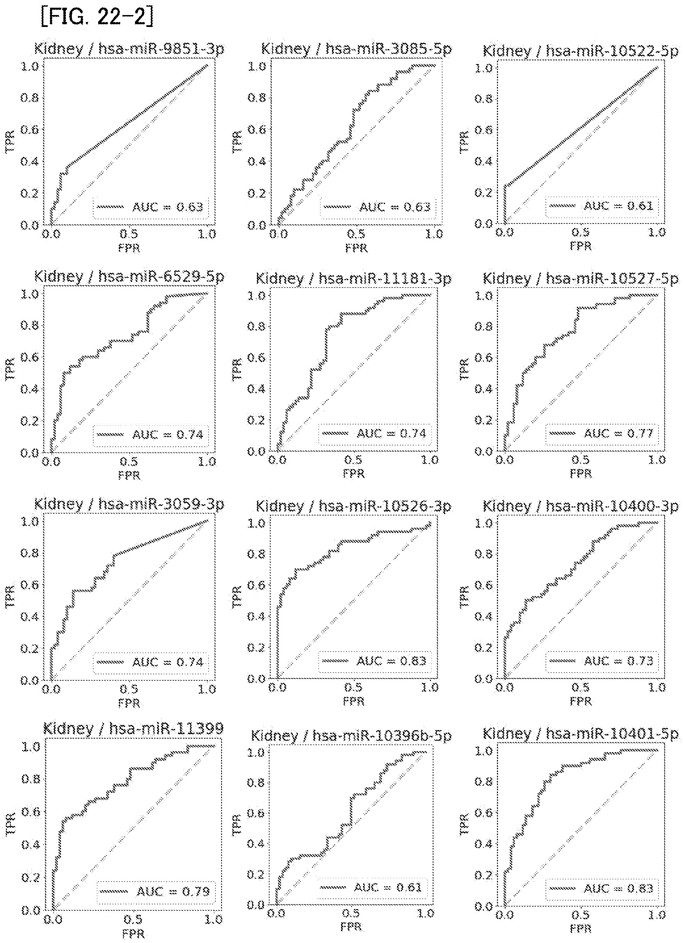

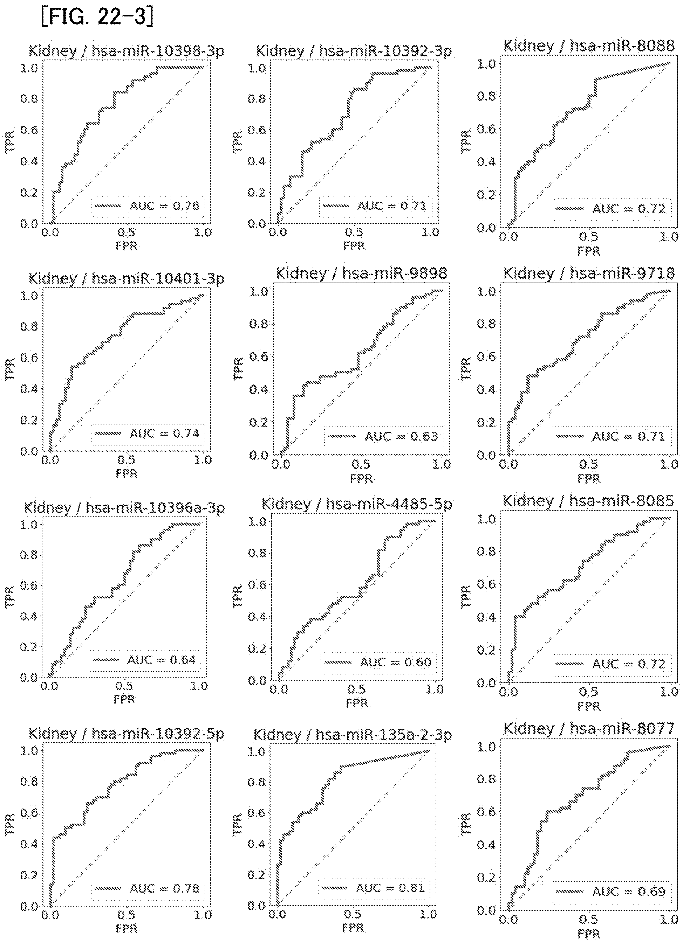

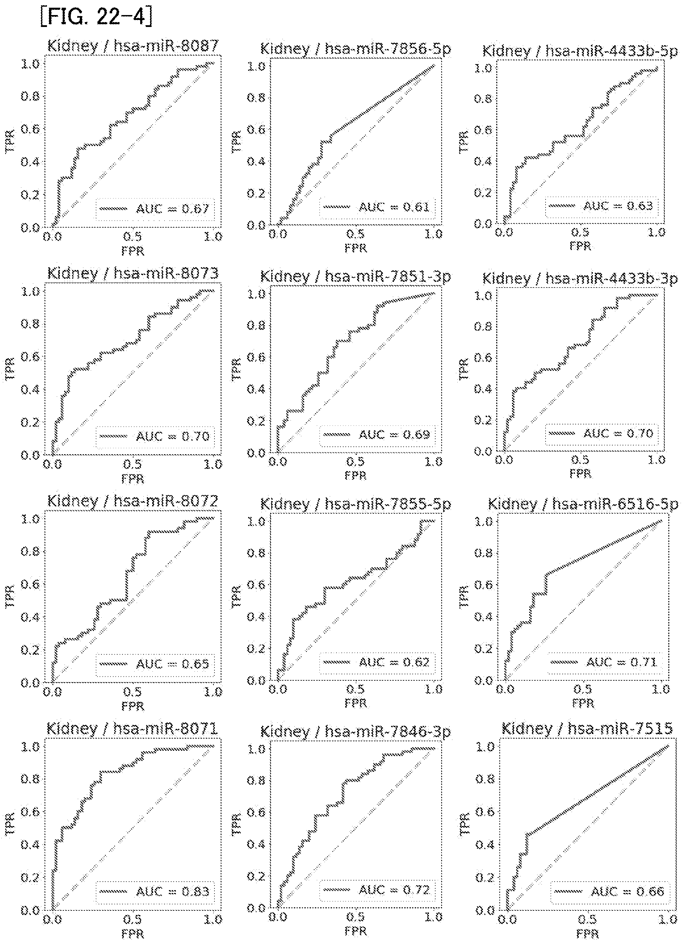

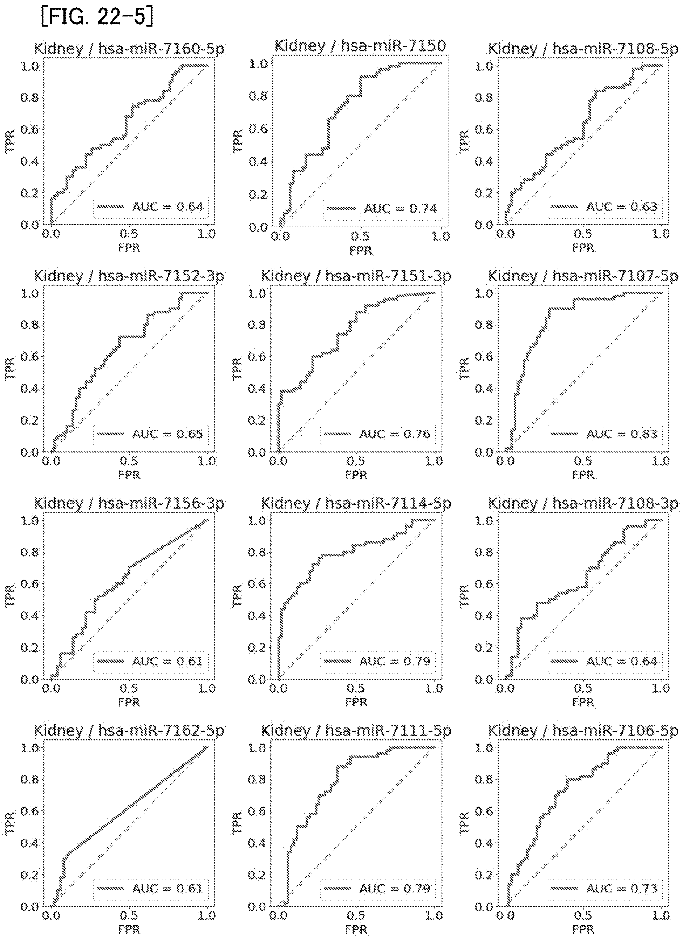

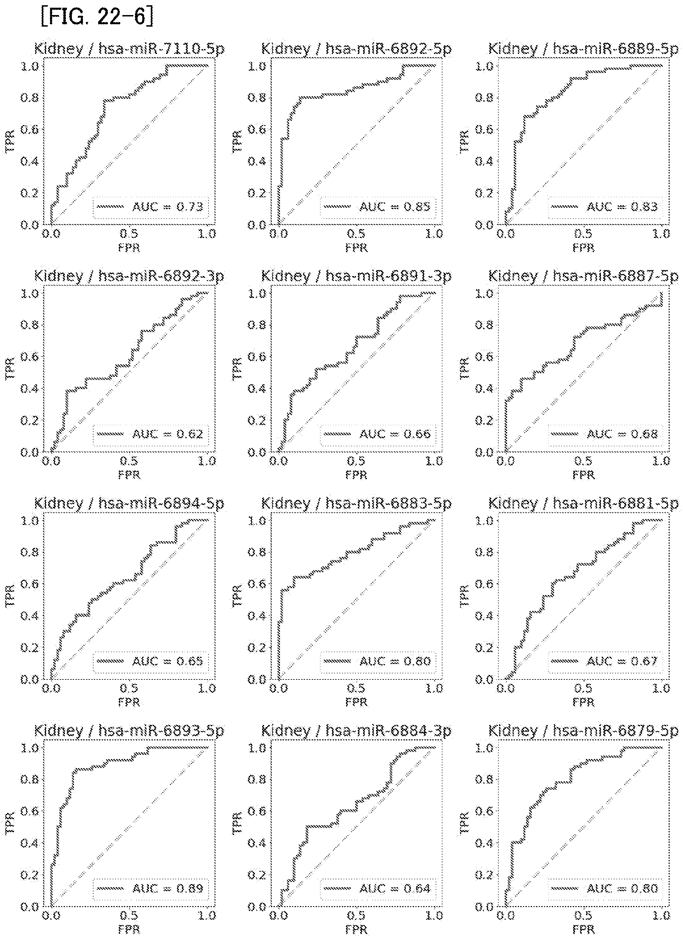

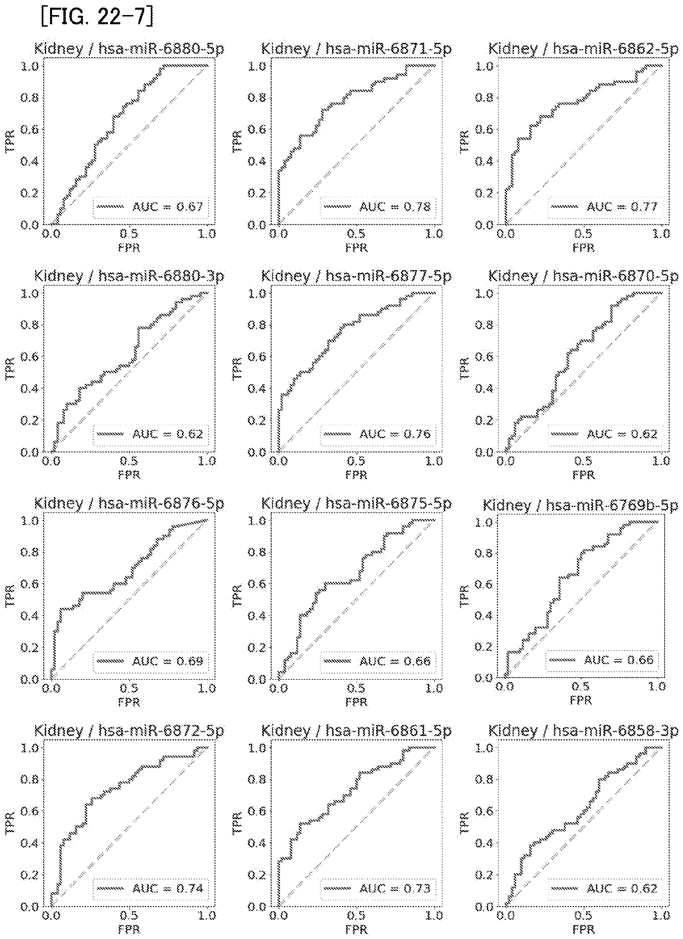

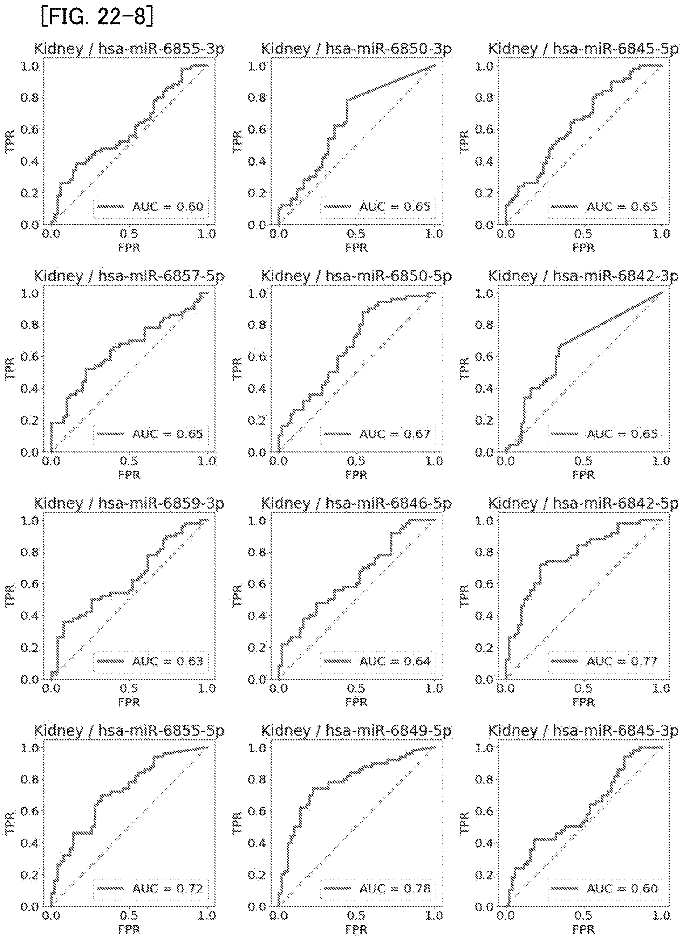

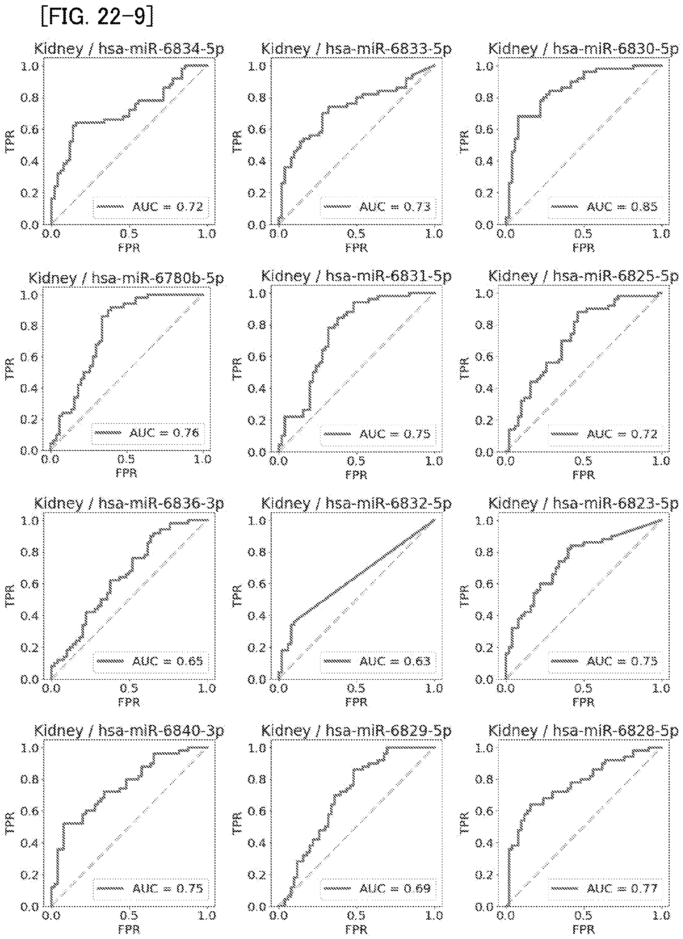

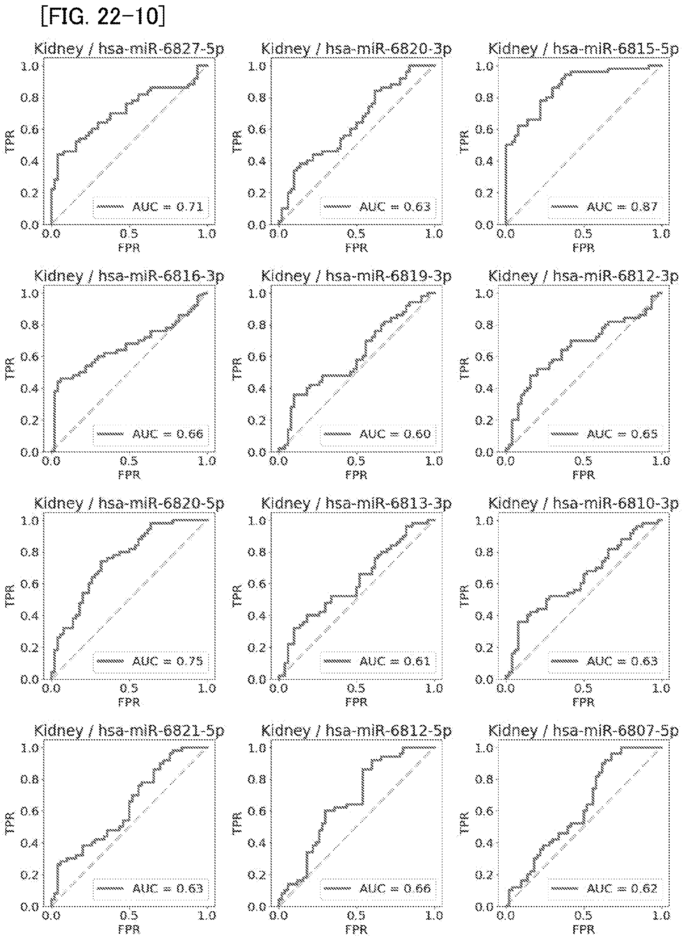

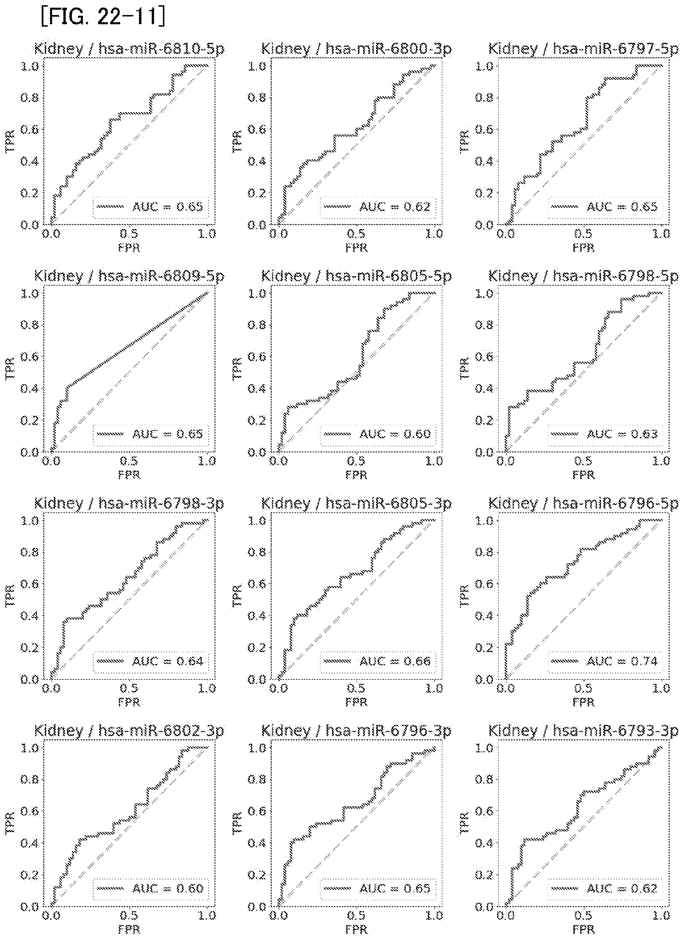

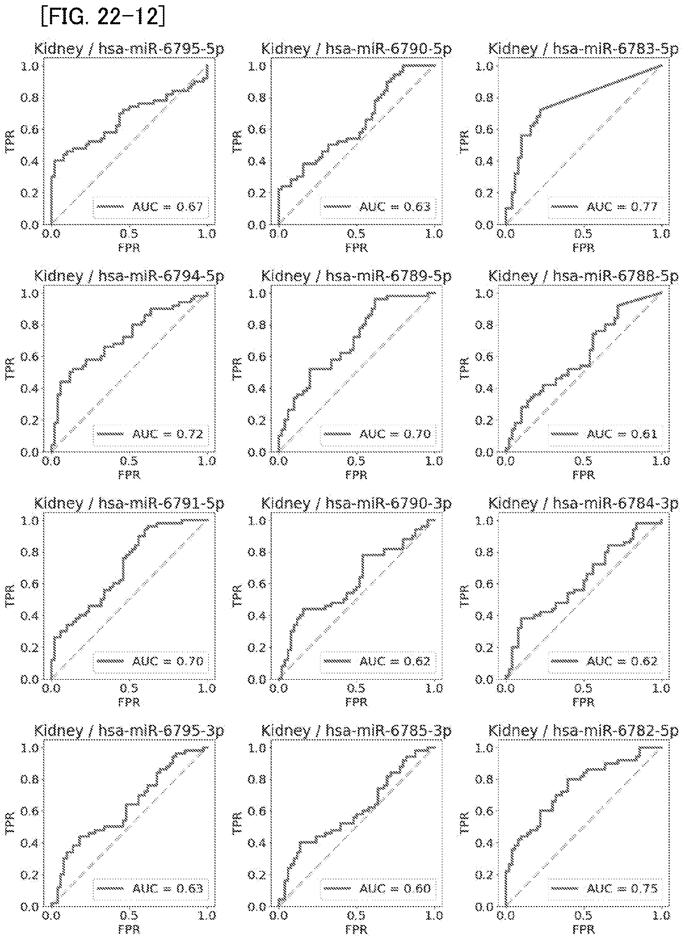

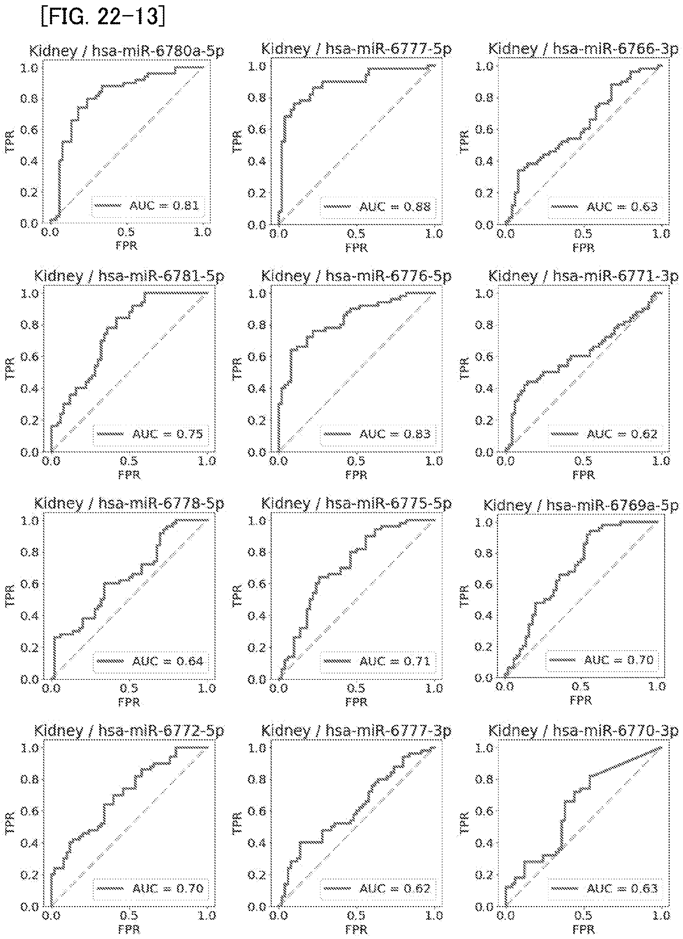

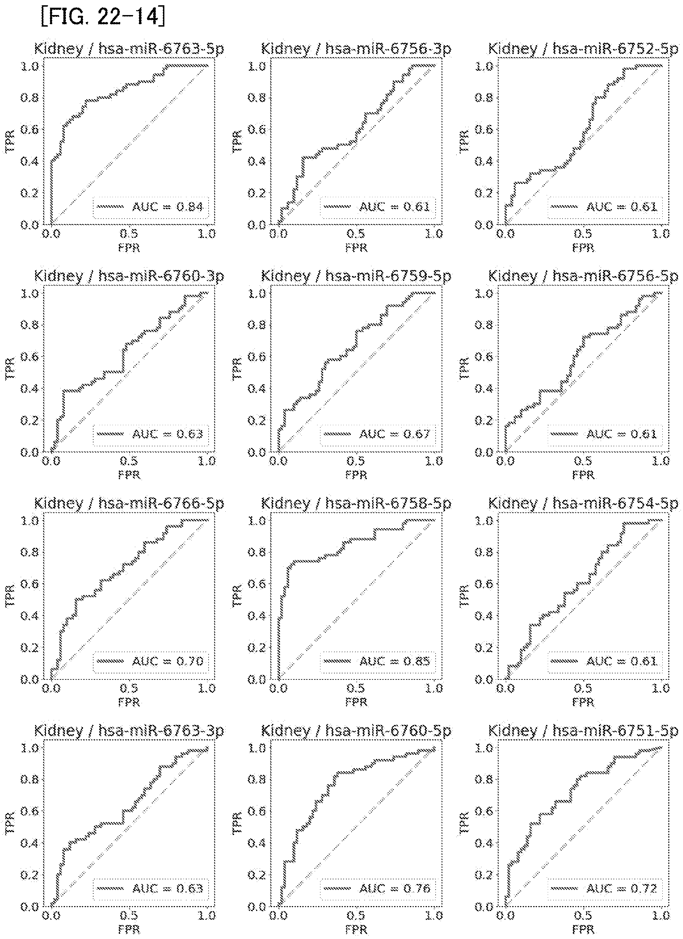

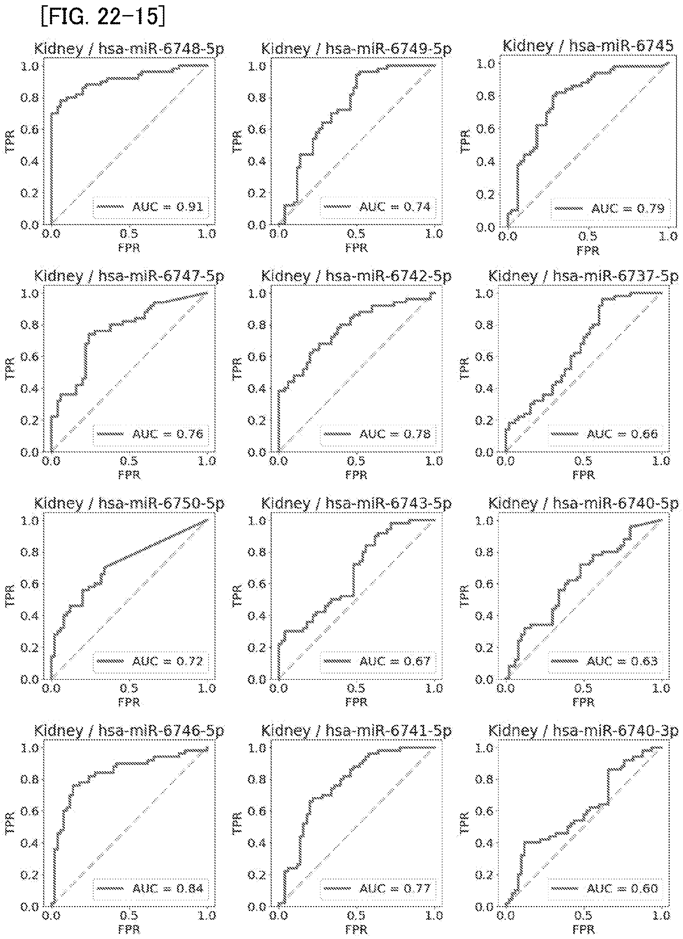

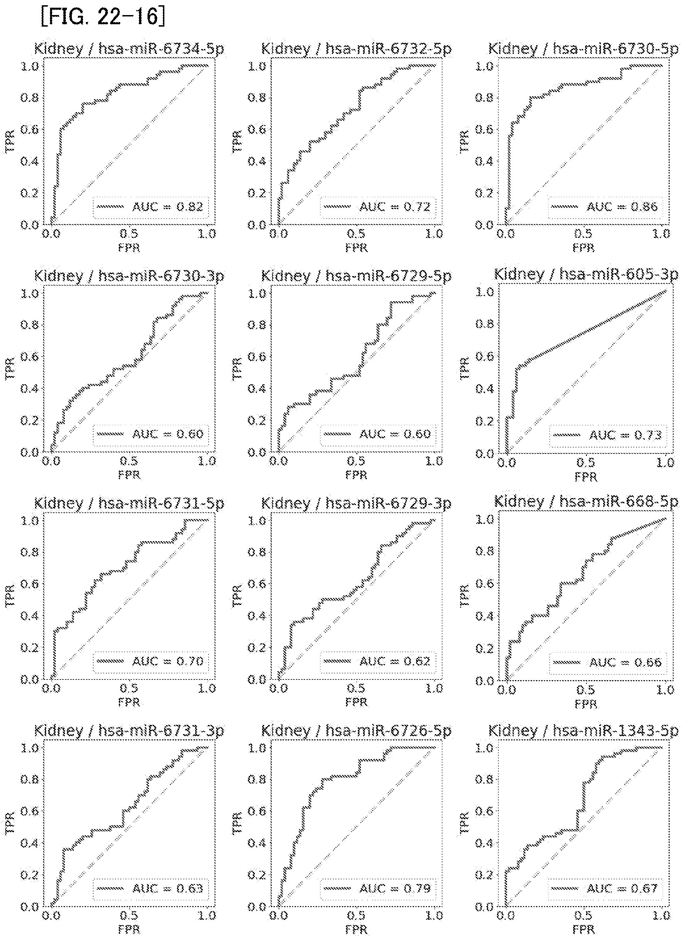

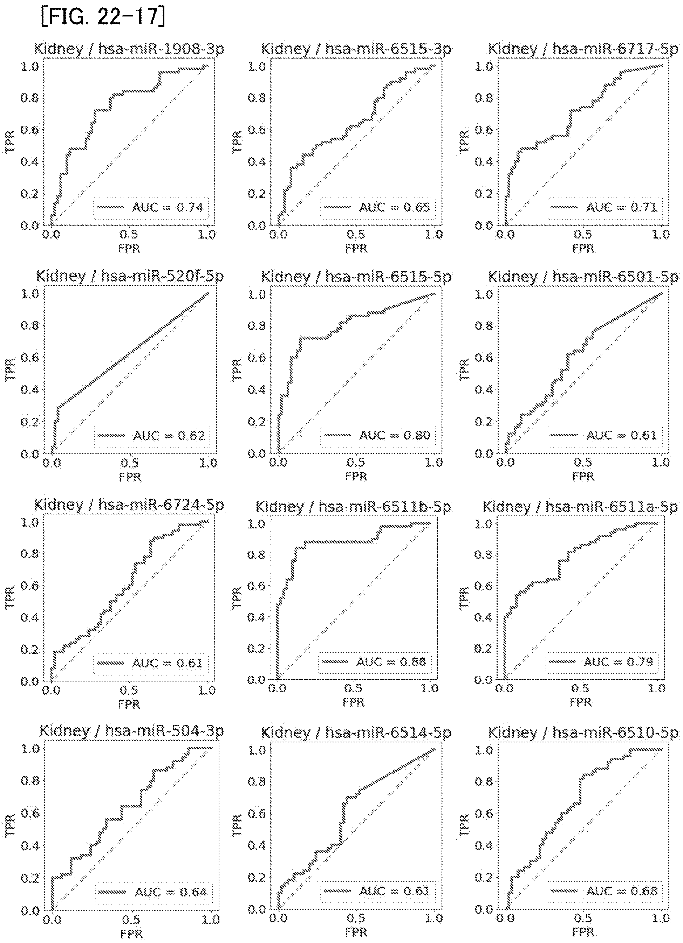

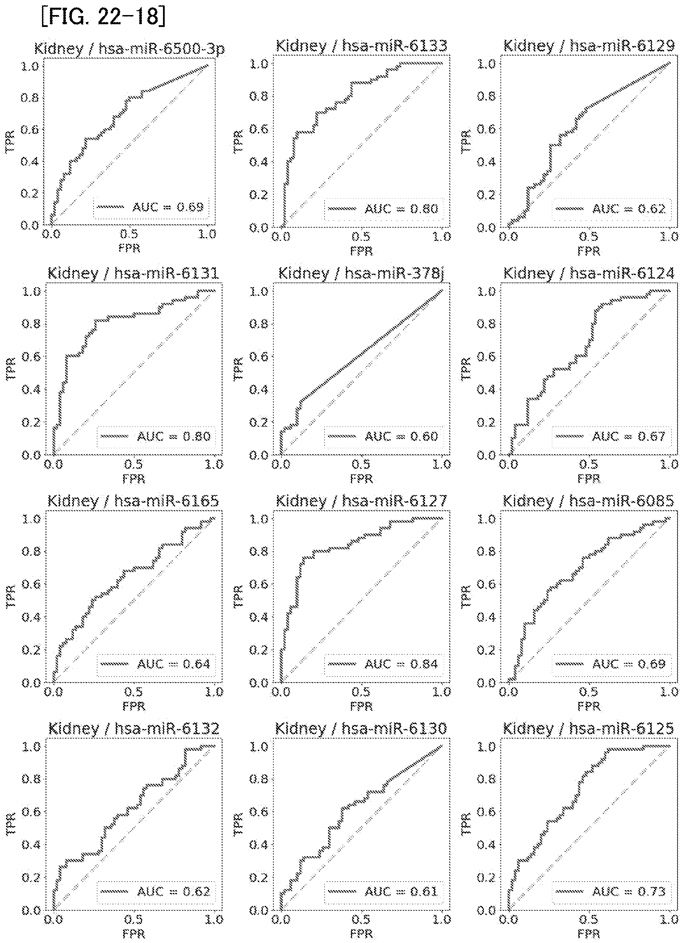

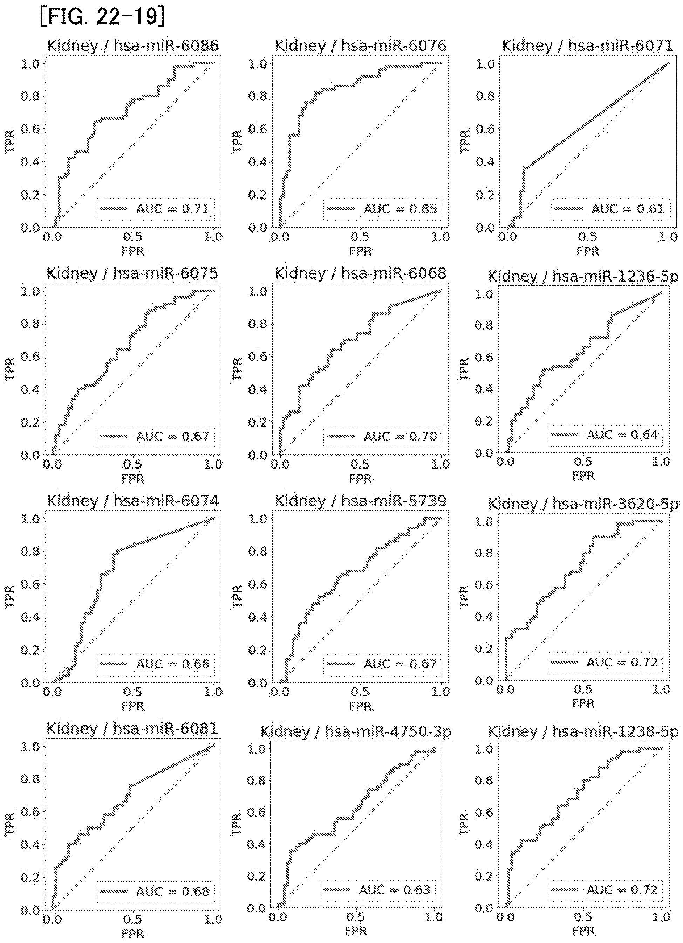

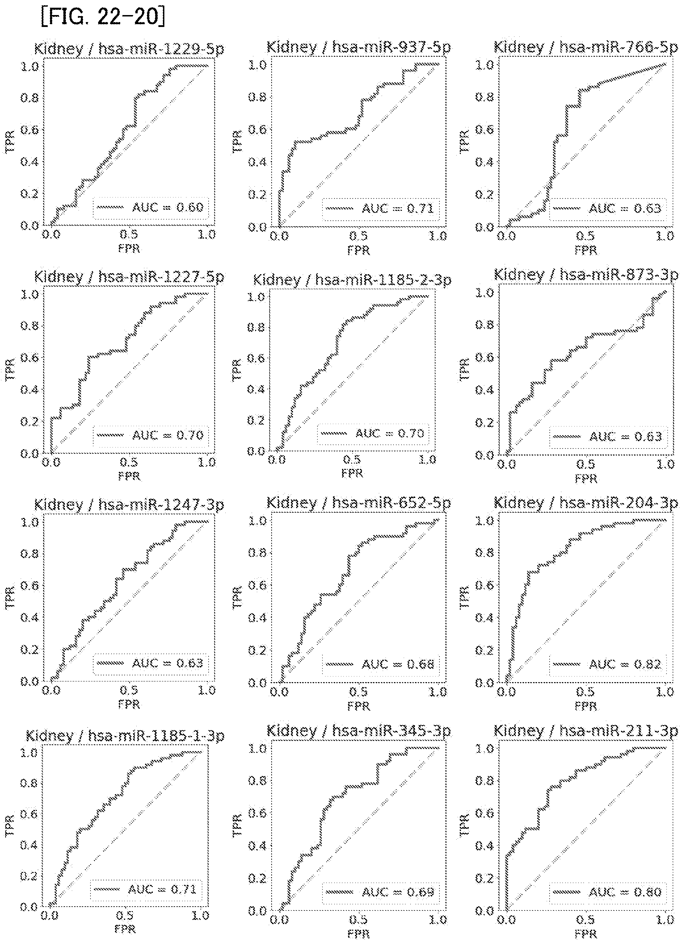

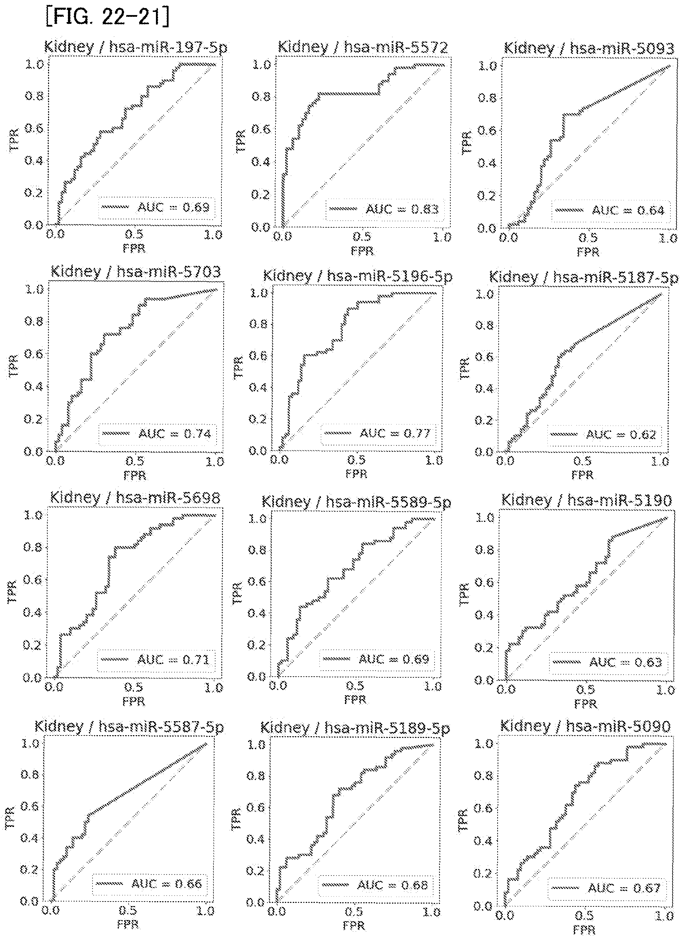

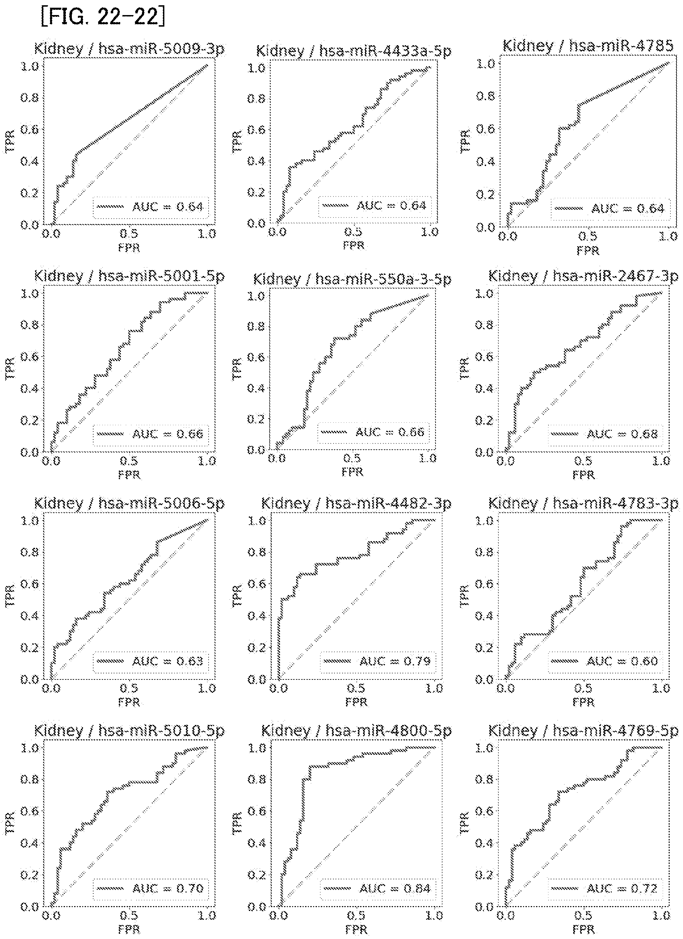

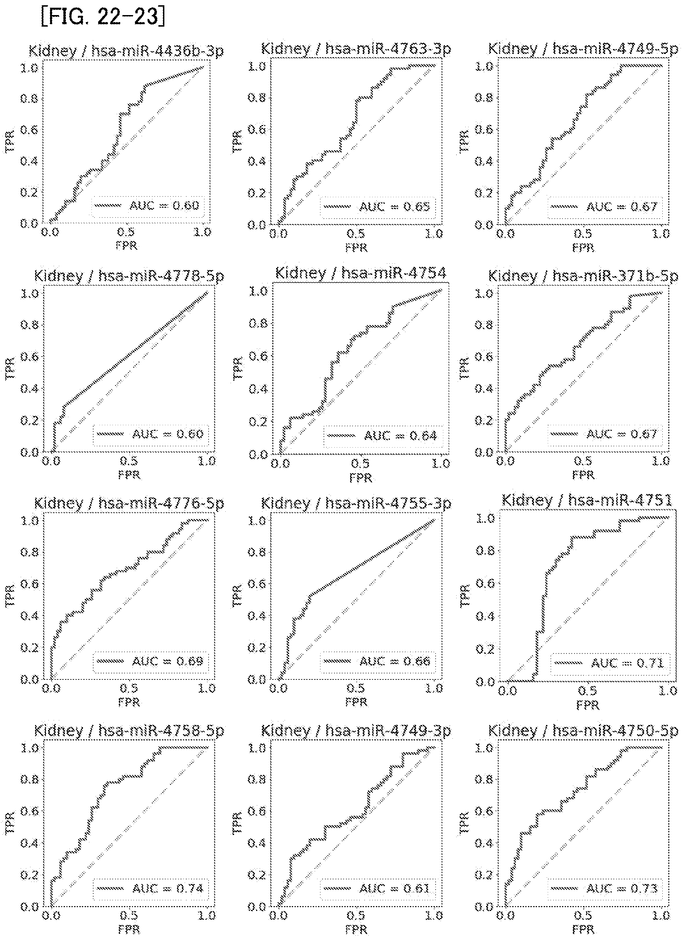

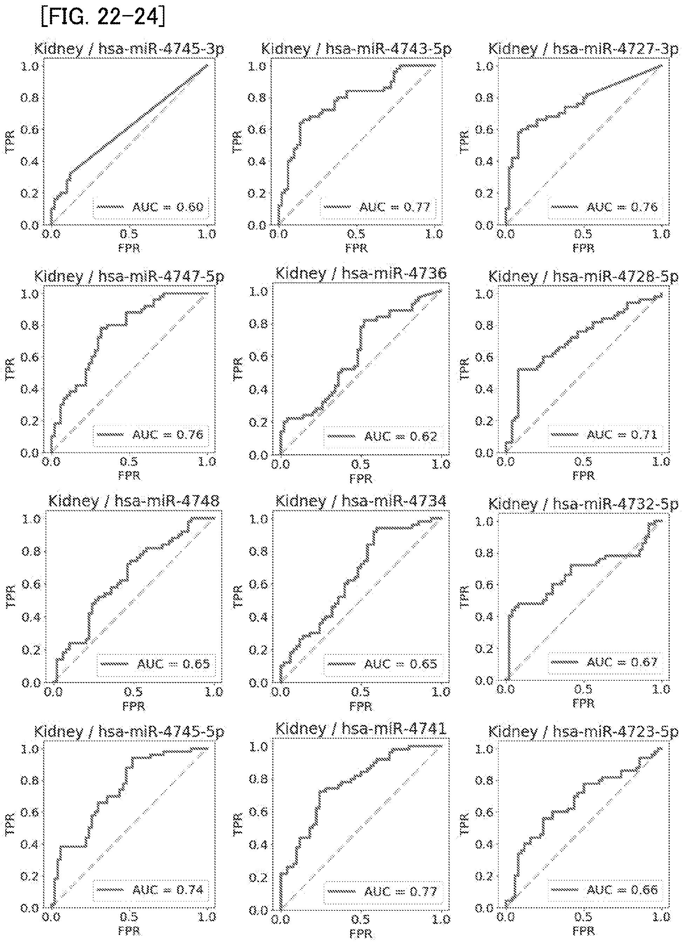

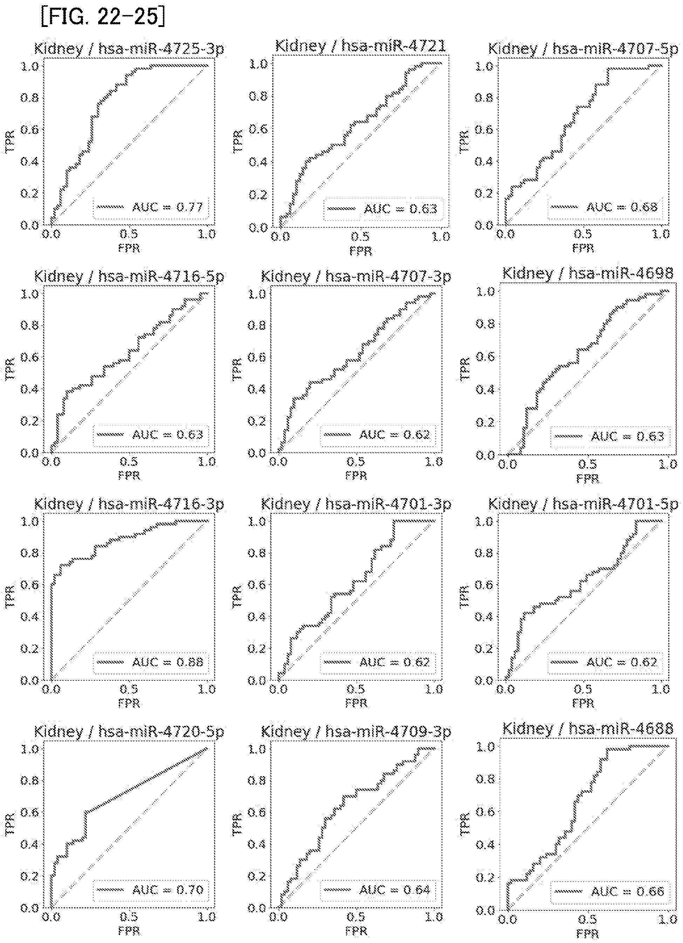

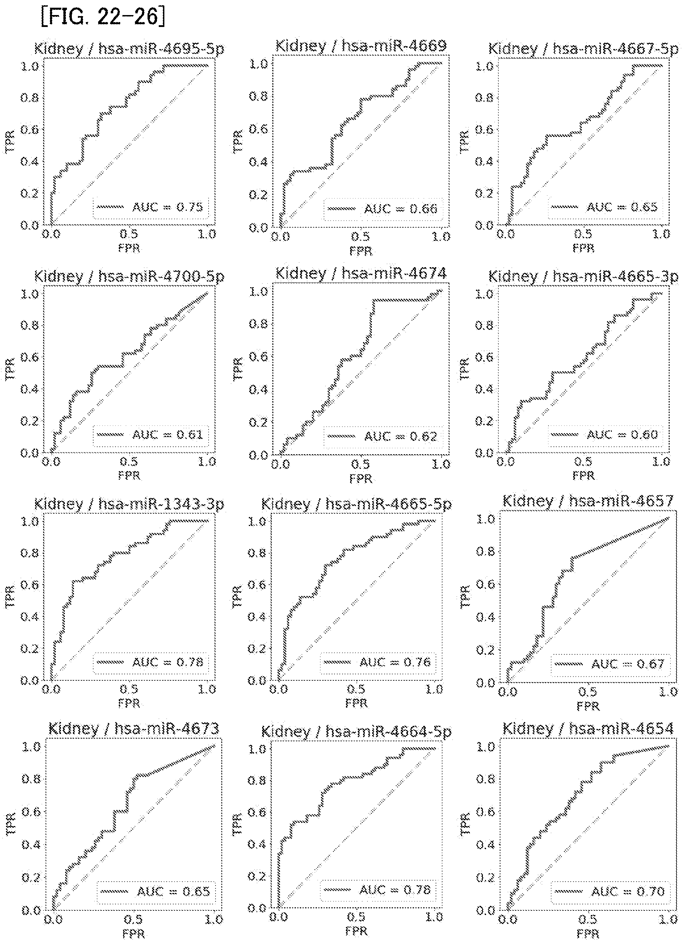

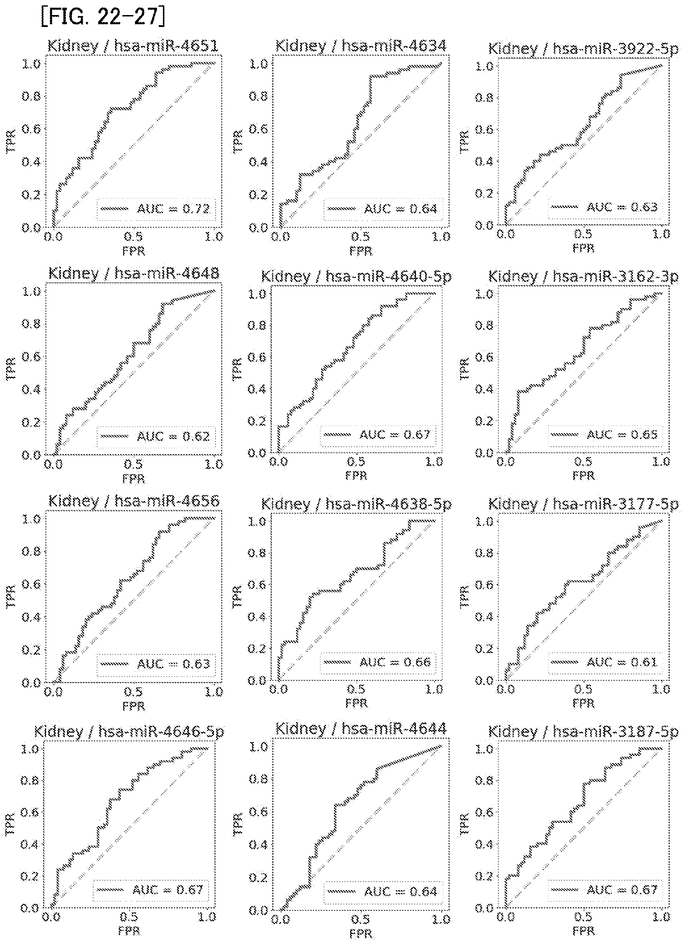

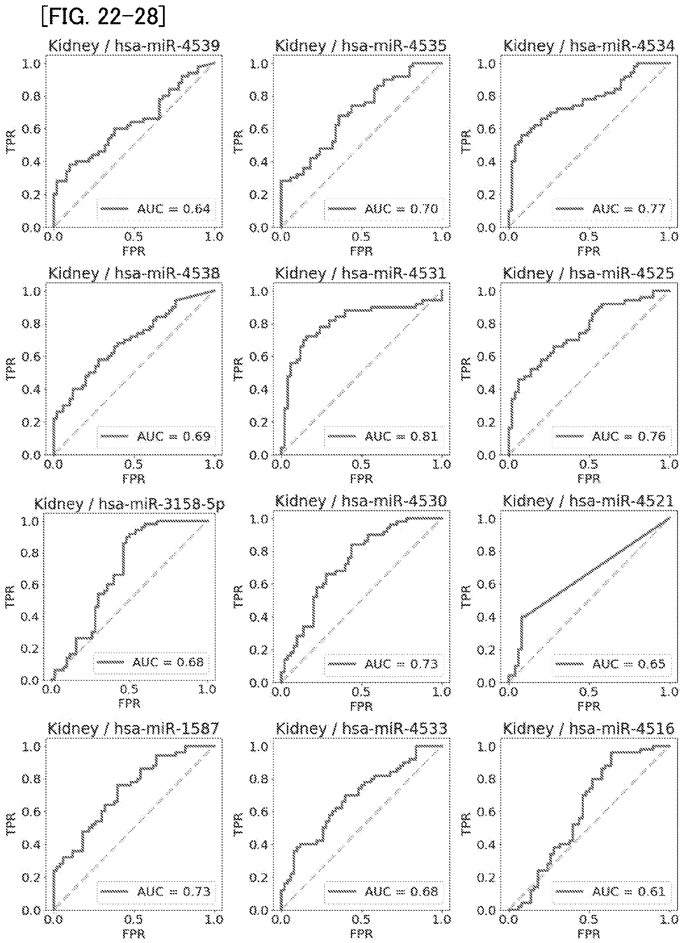

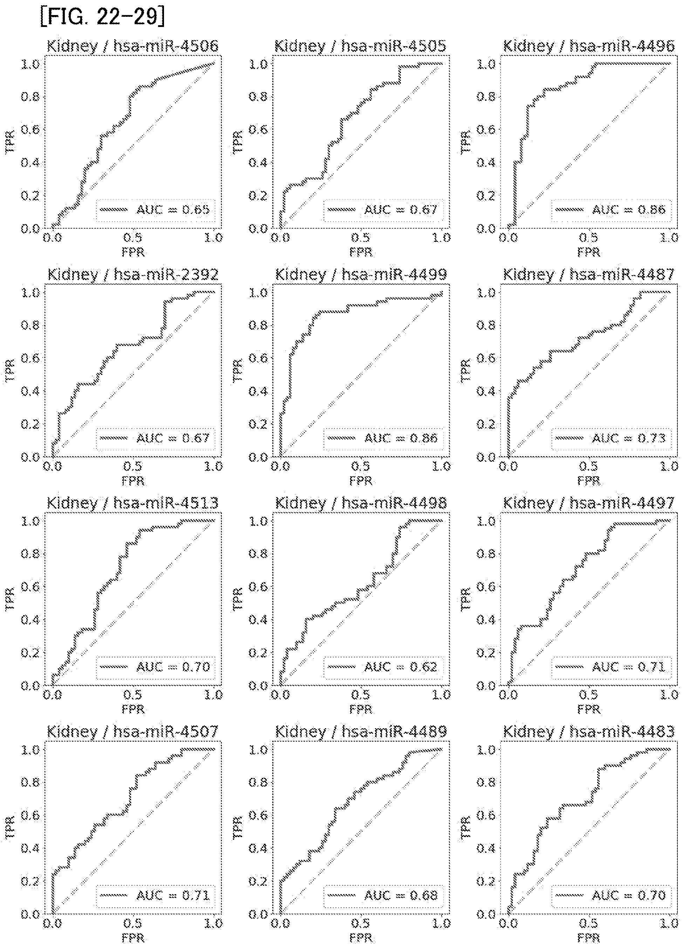

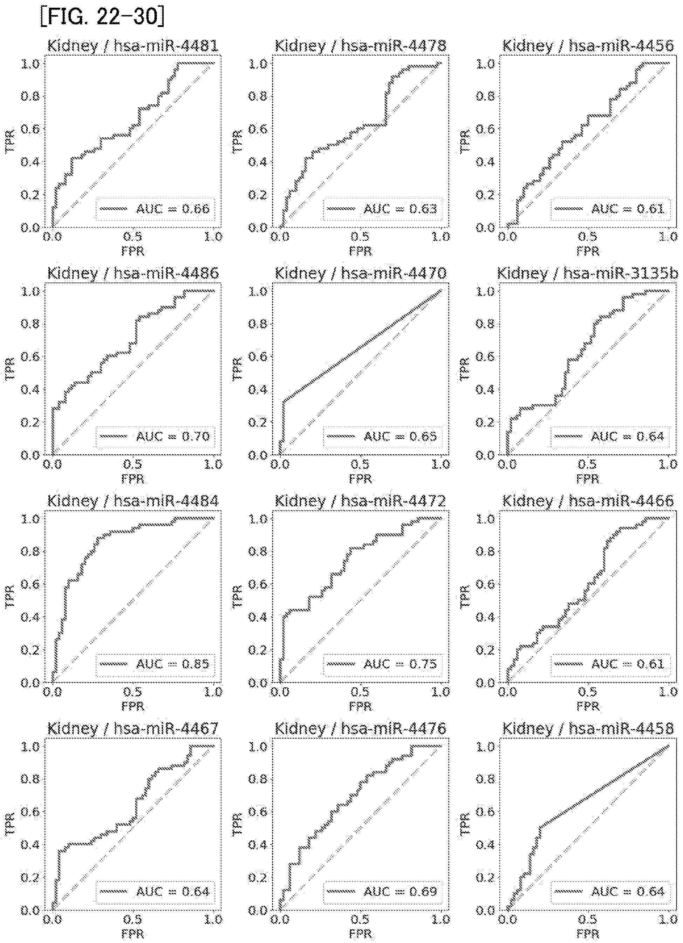

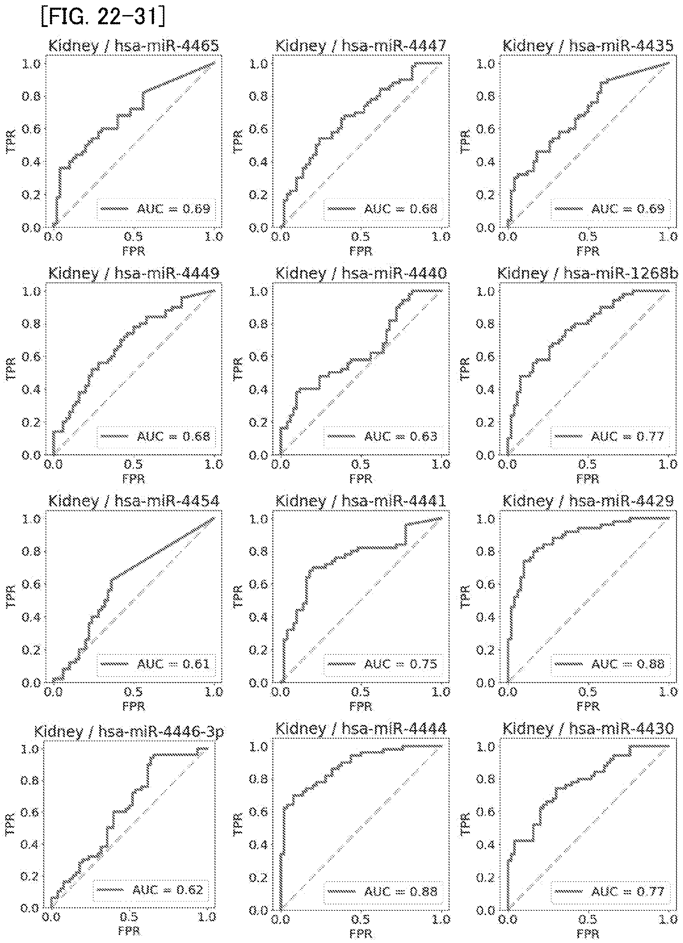

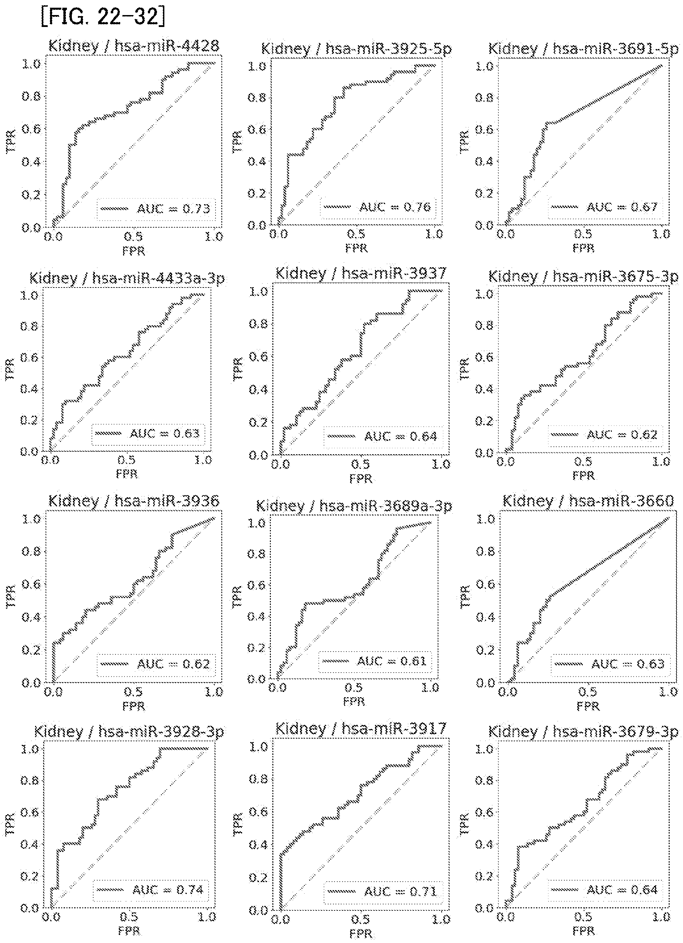

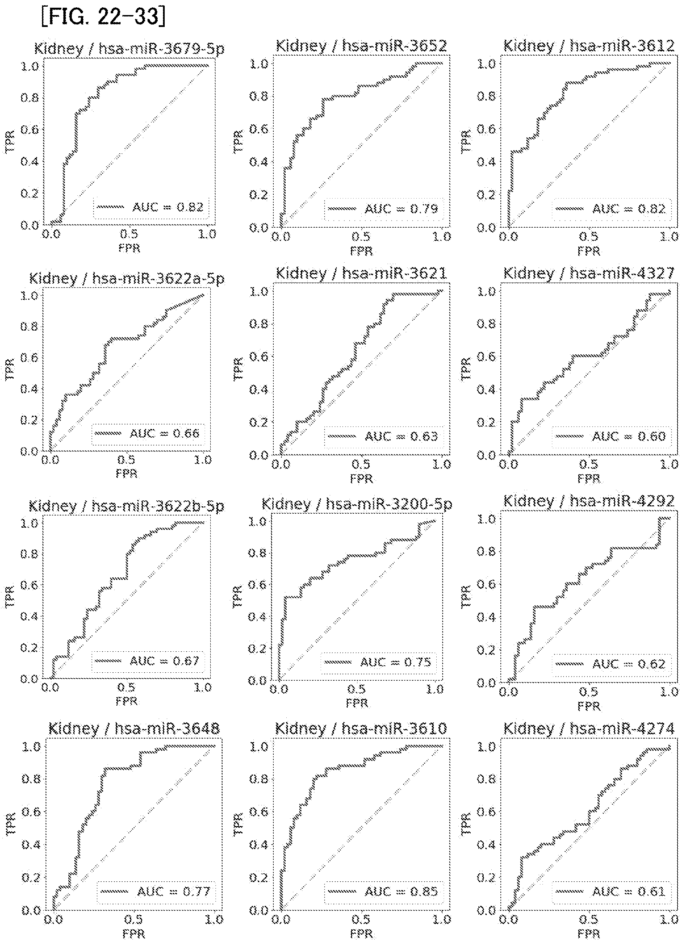

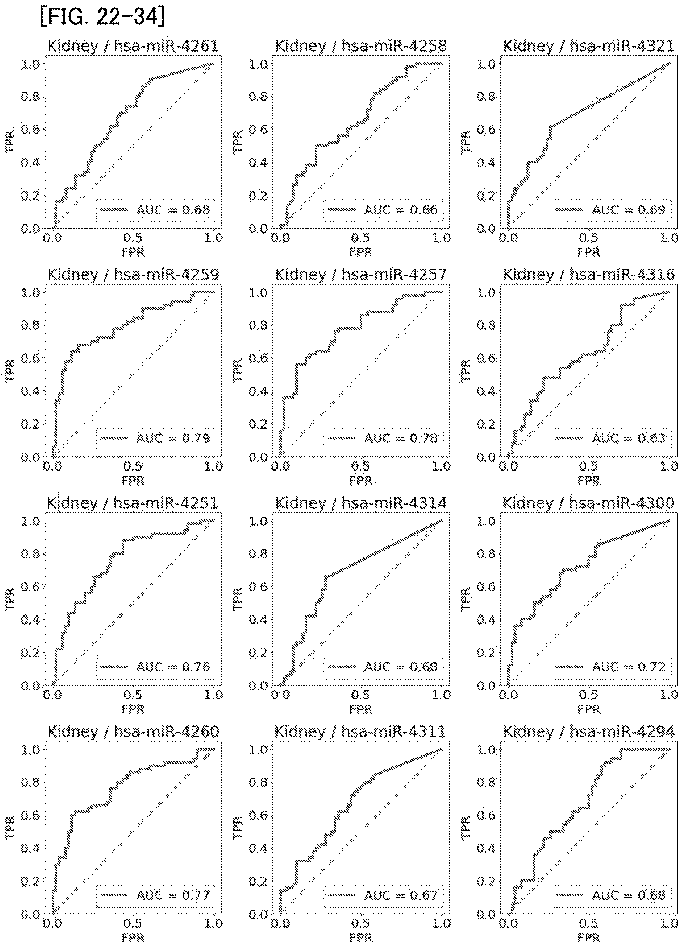

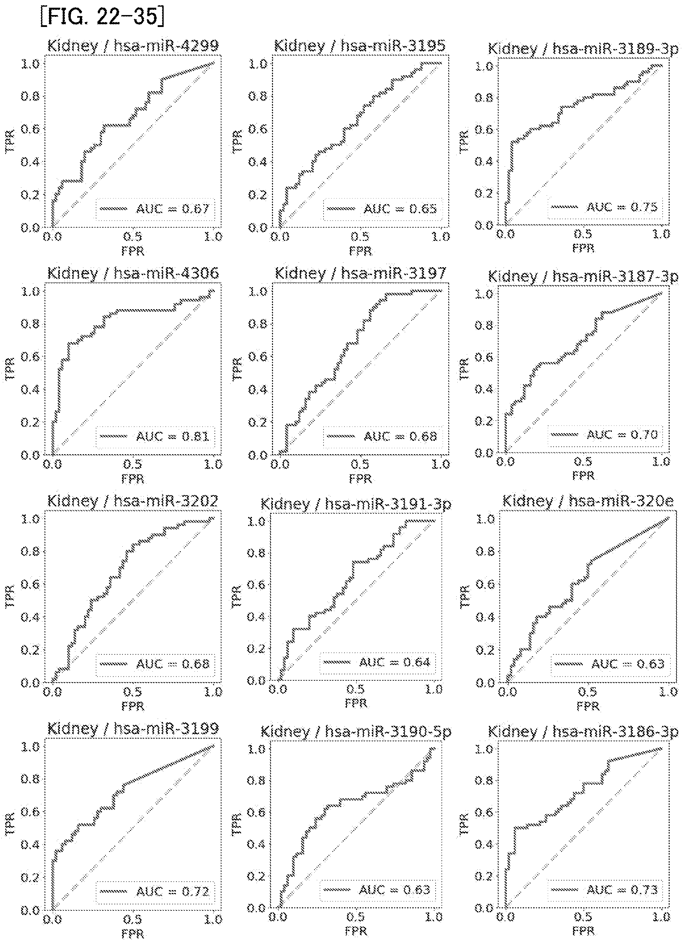

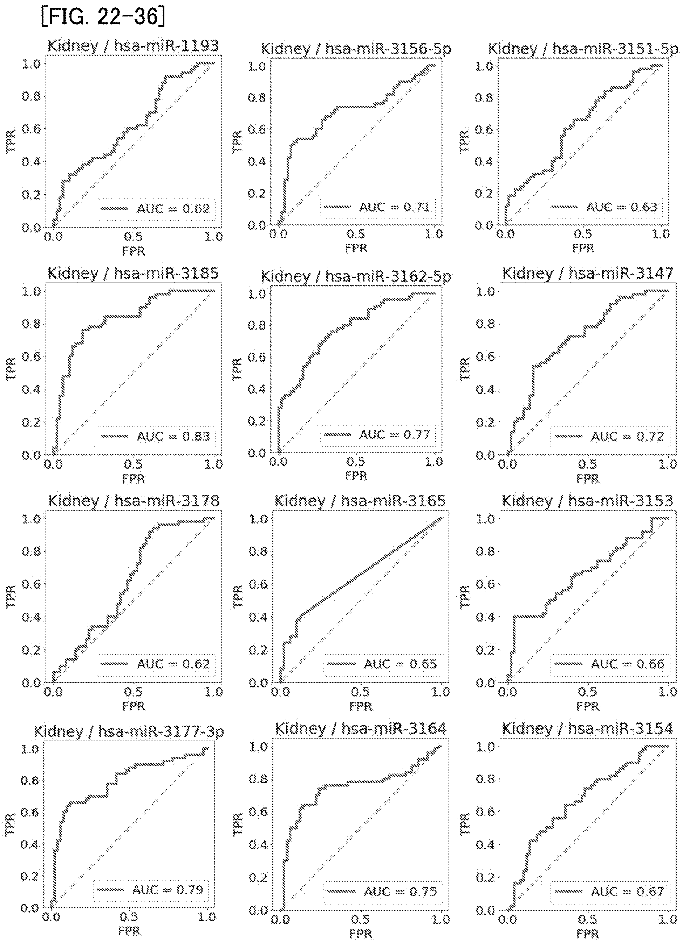

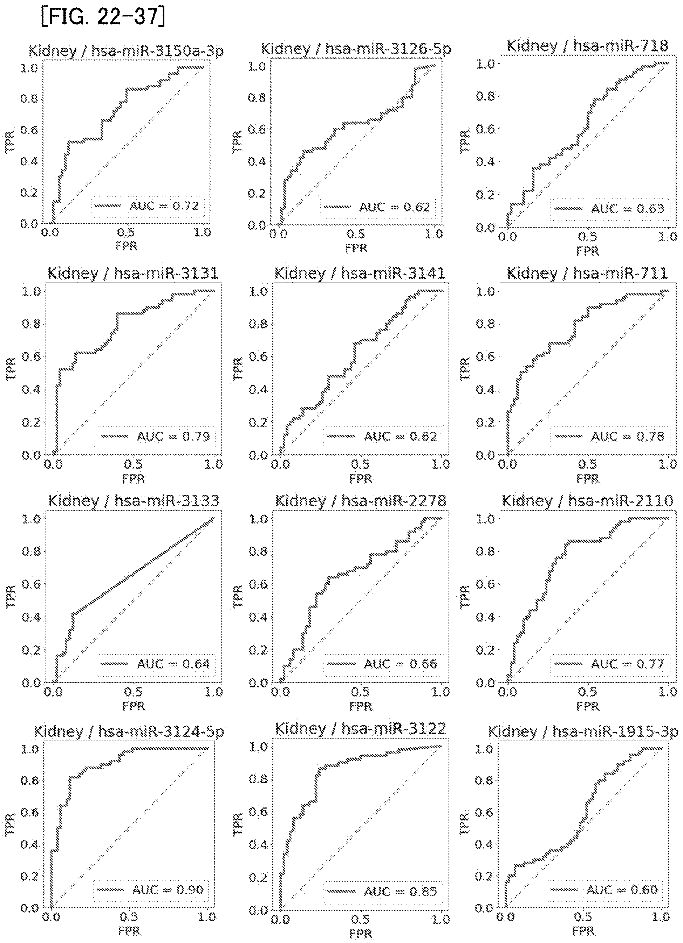

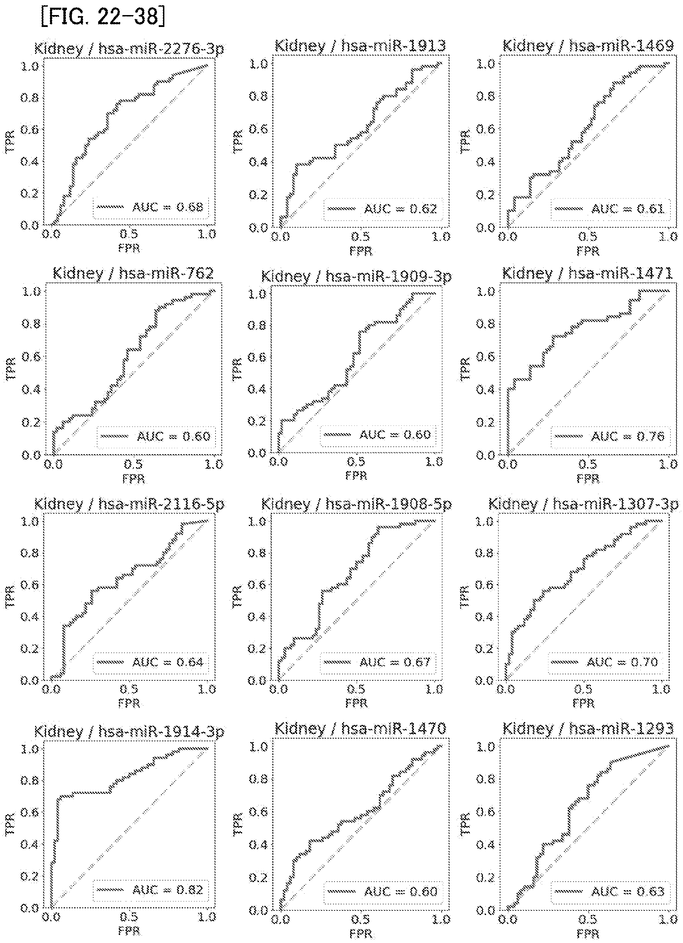

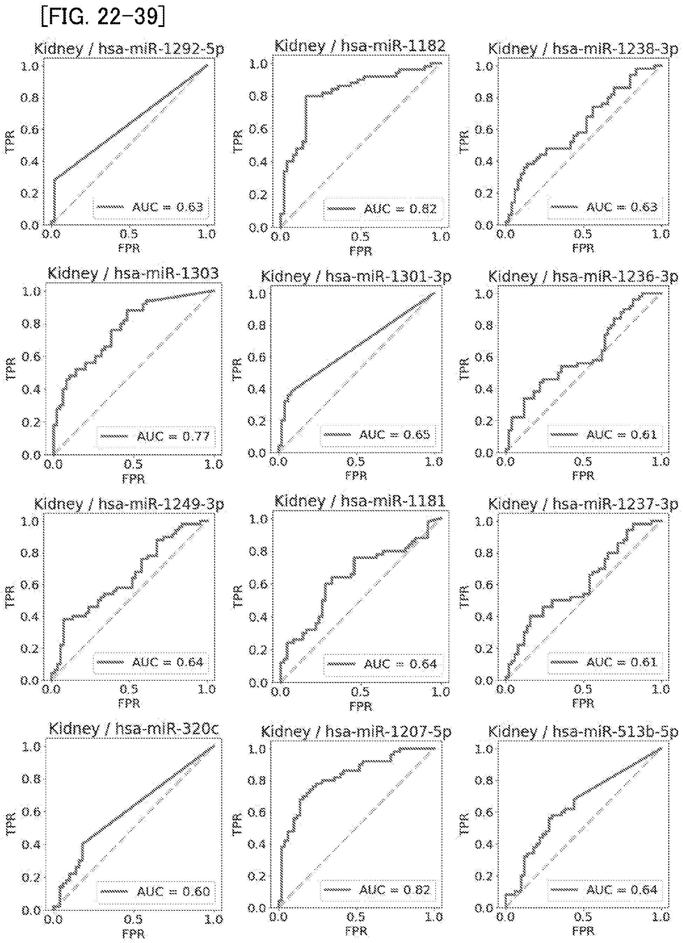

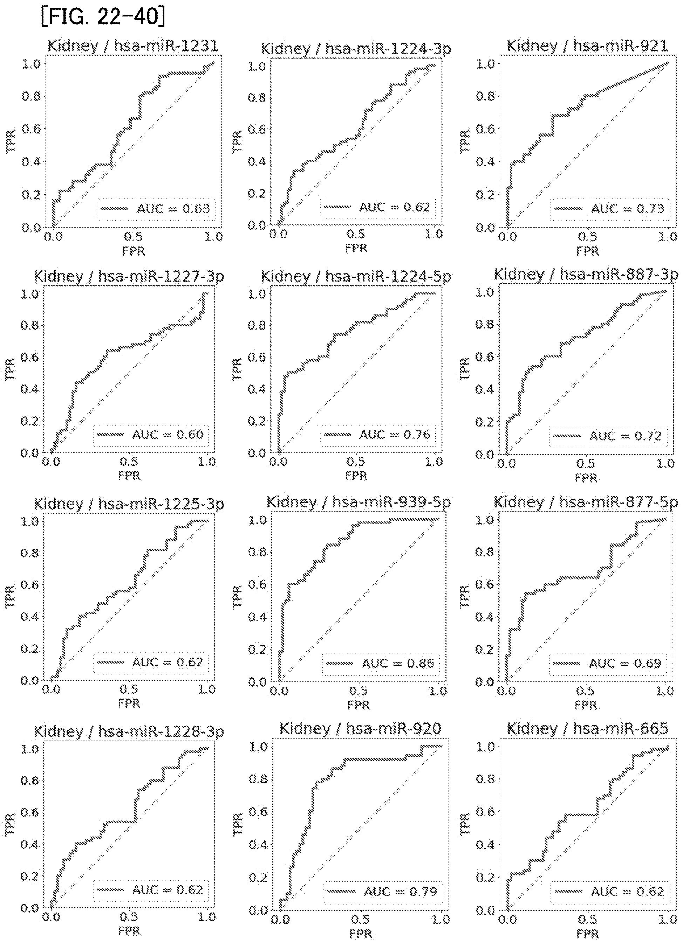

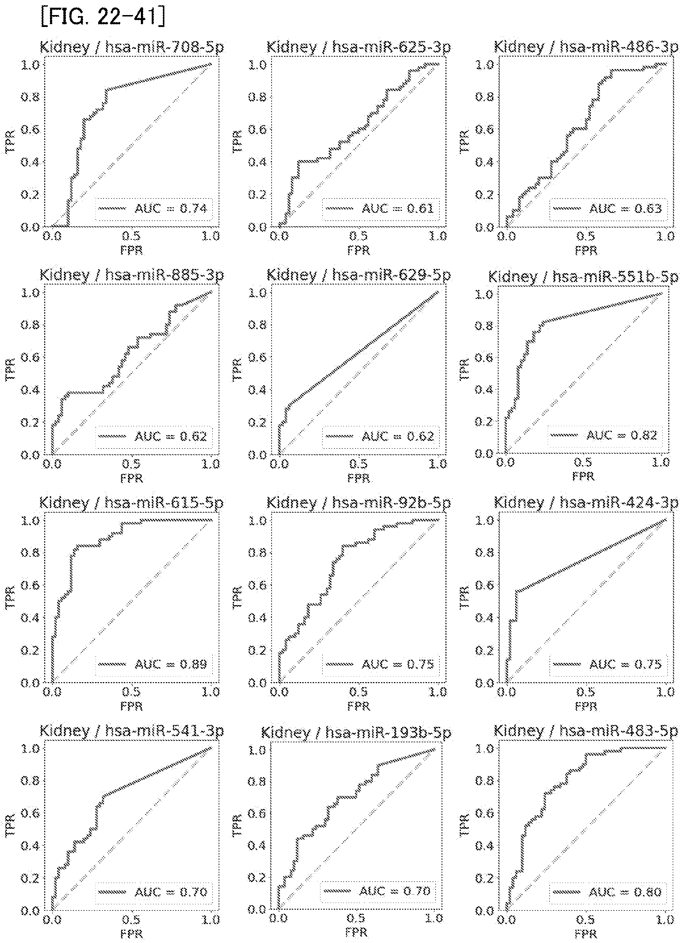

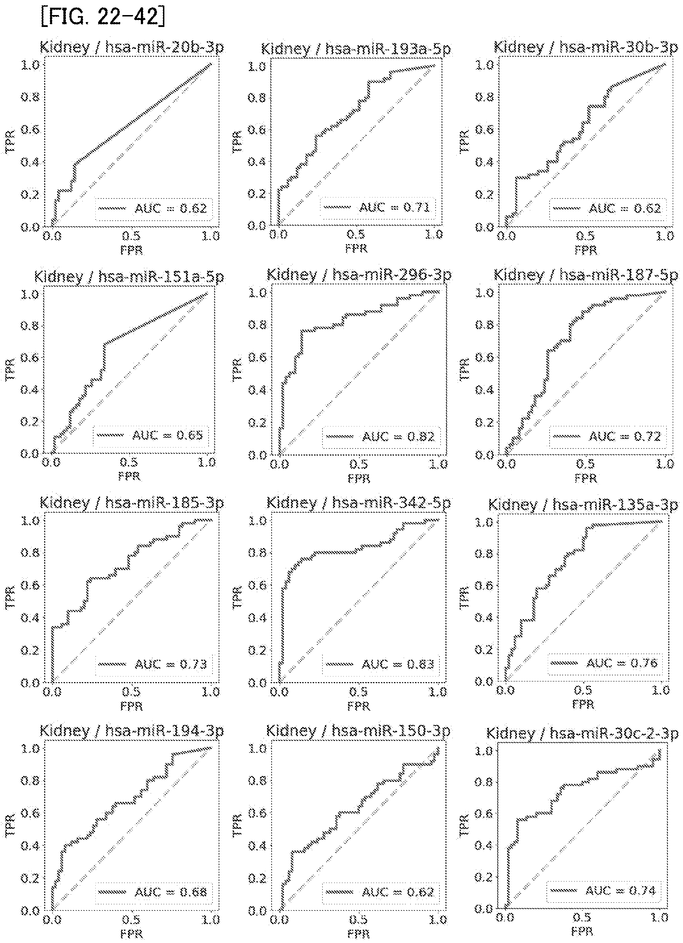

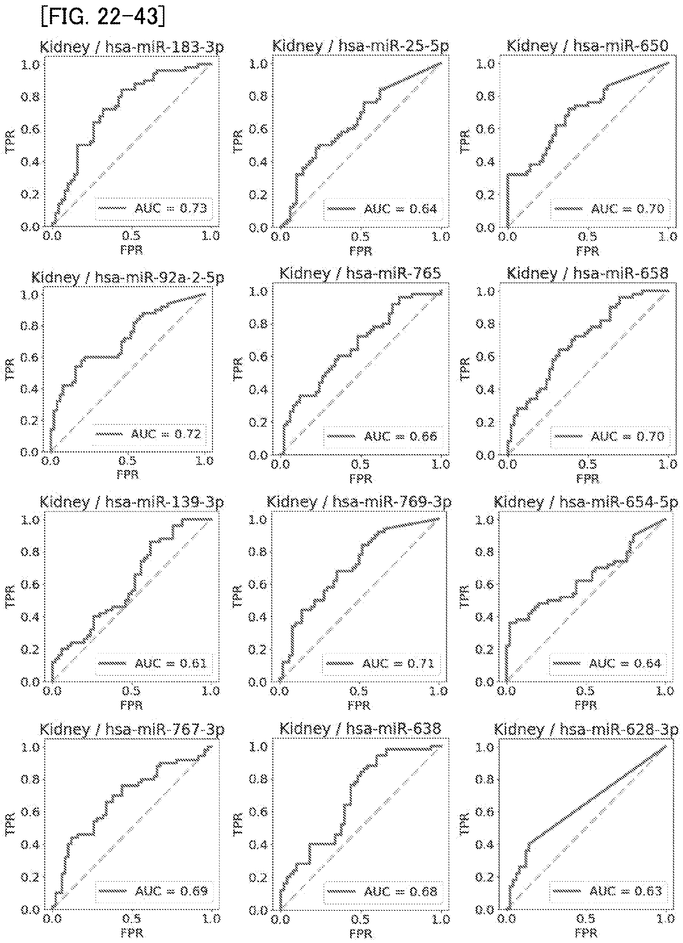

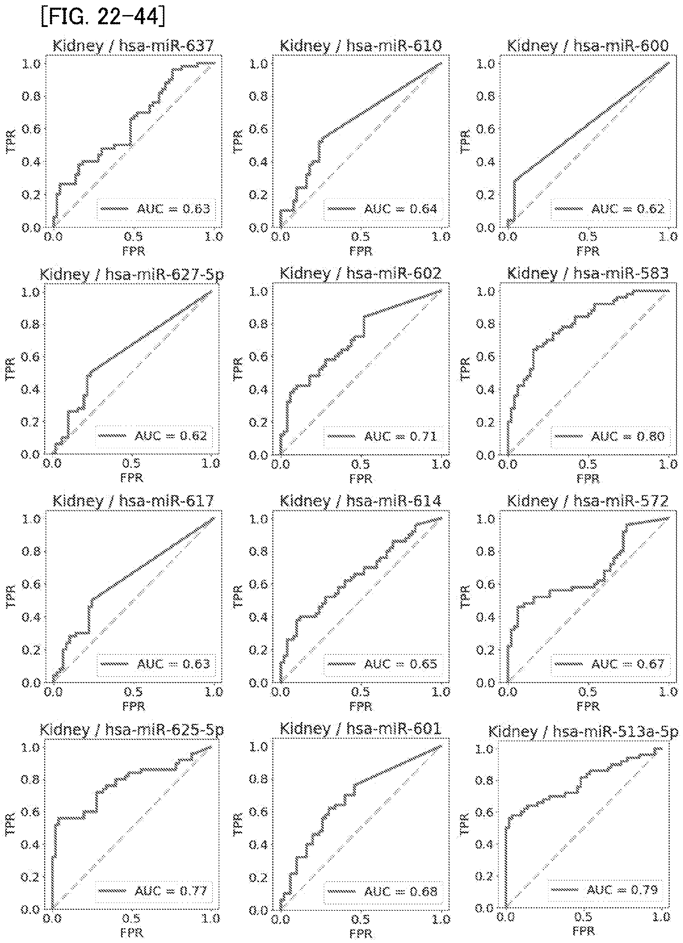

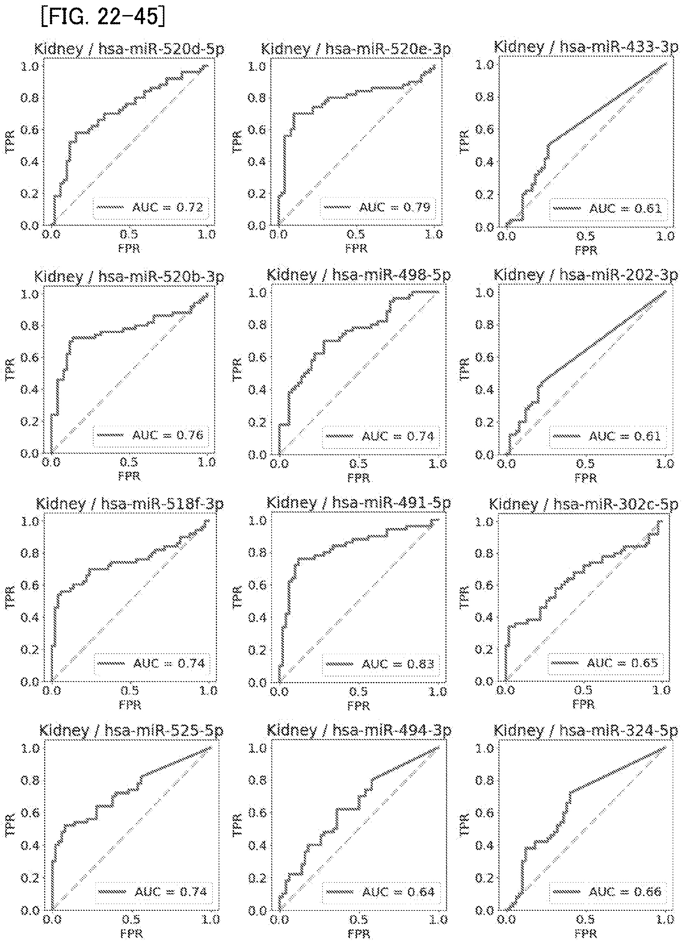

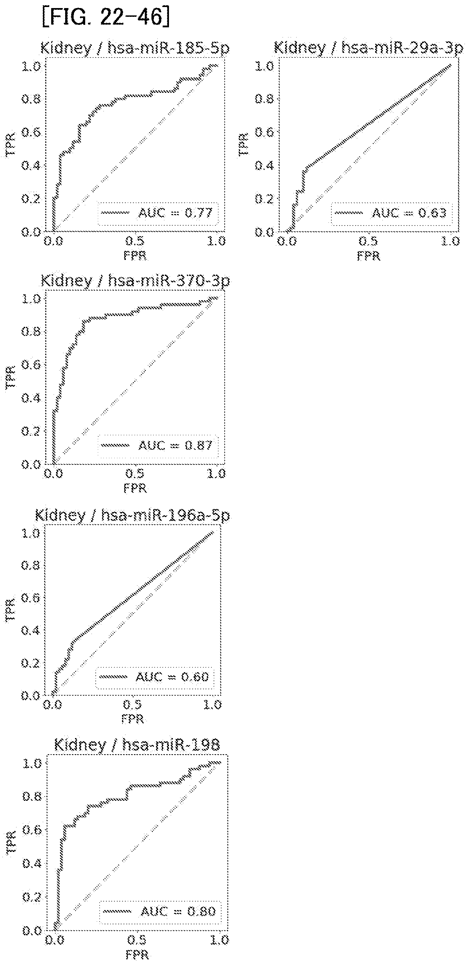

FIGS. 22-1 to 22-46 show ROCs curves for predicting kidney cancer in humans using a single miRNA.

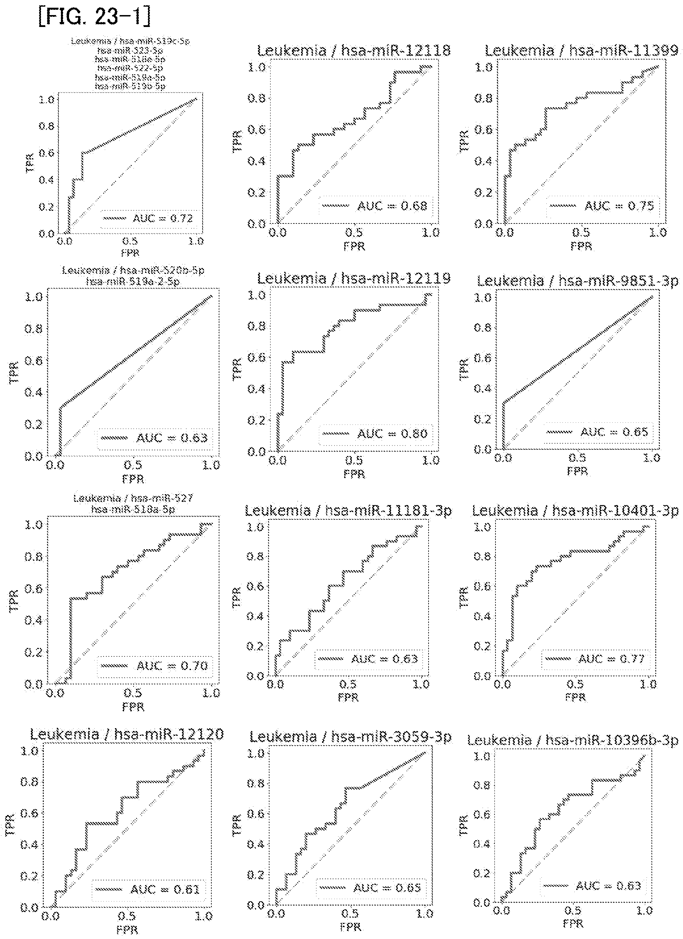

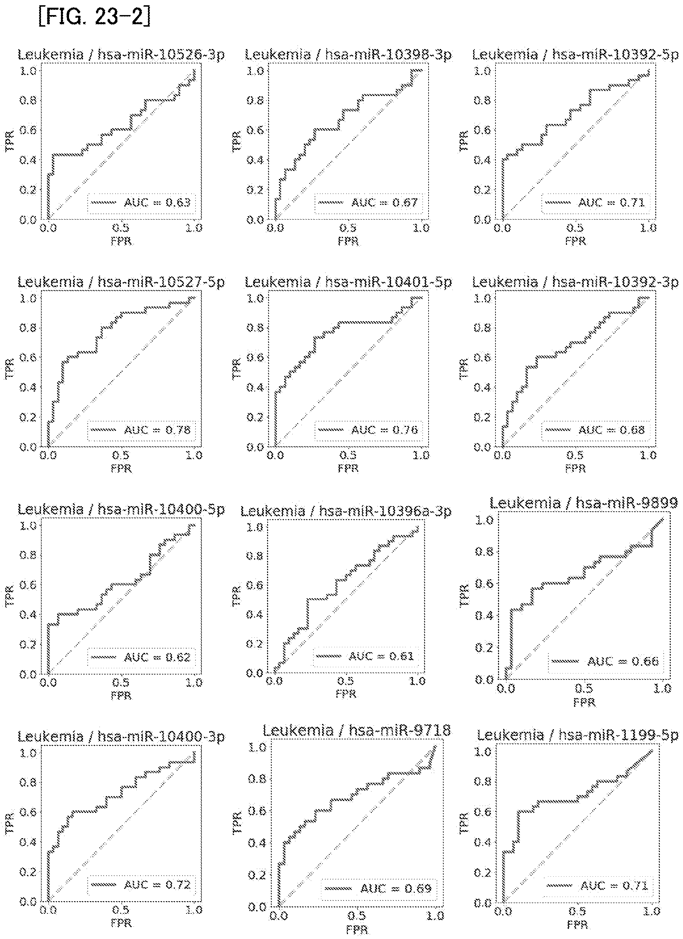

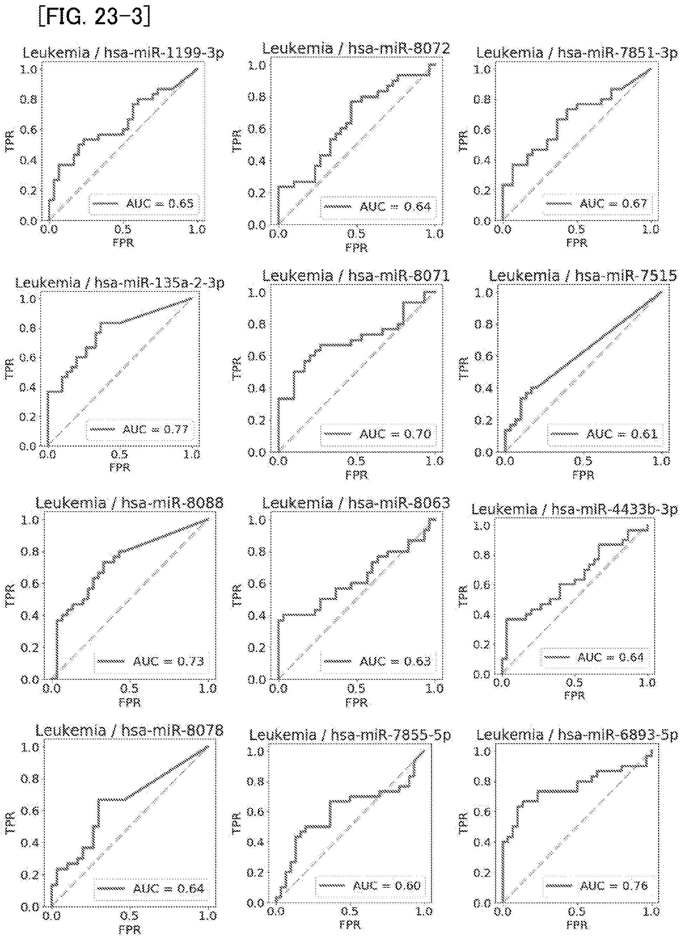

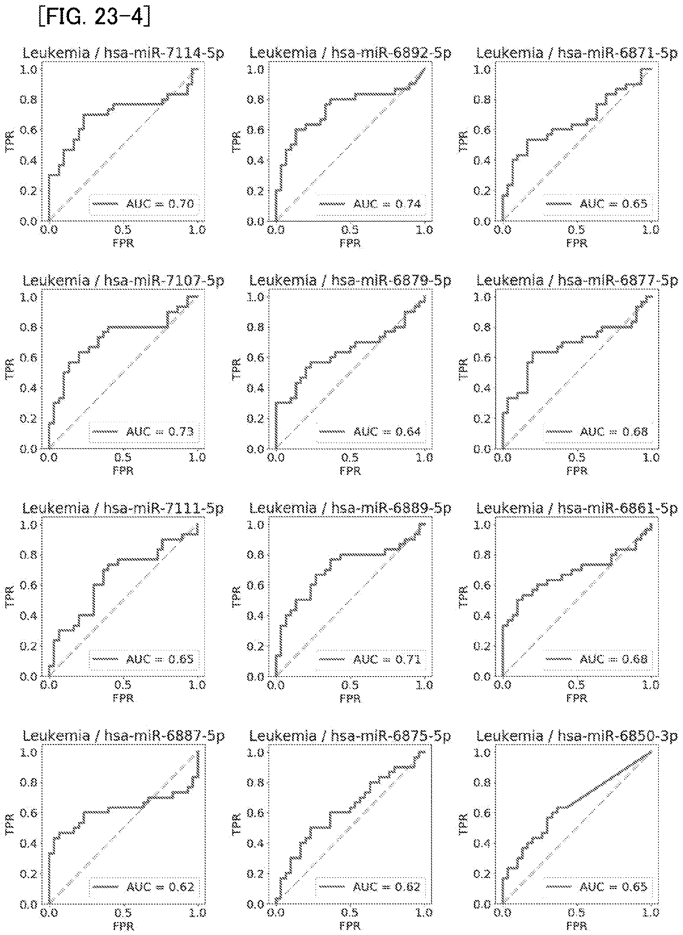

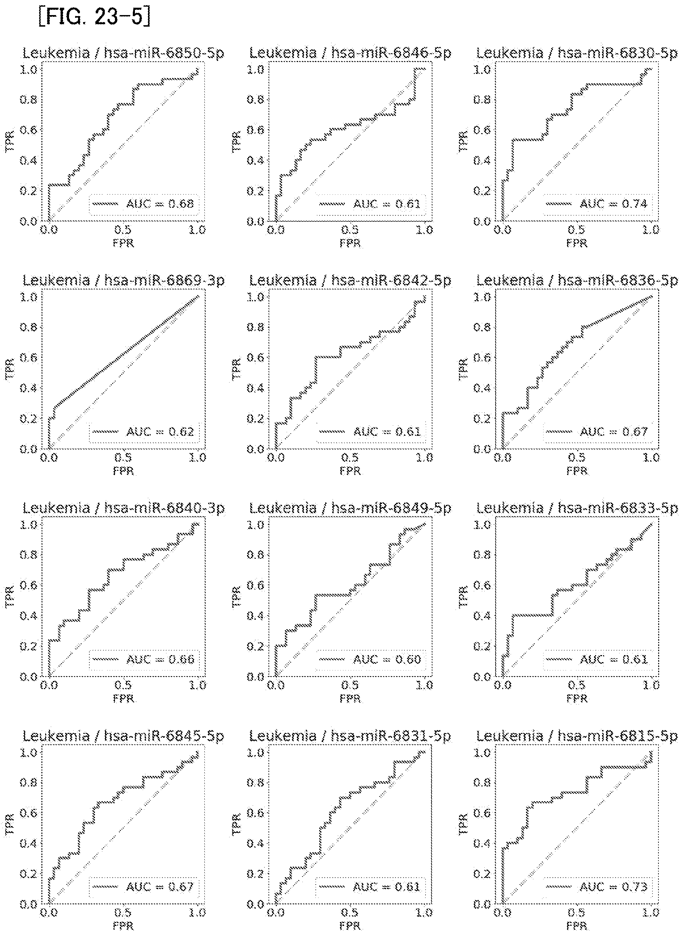

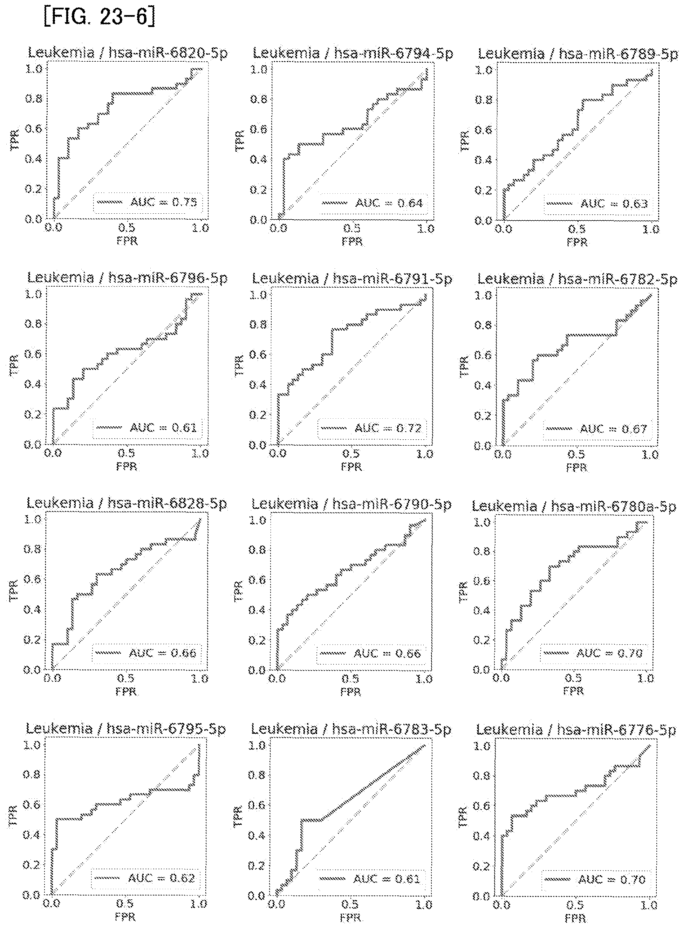

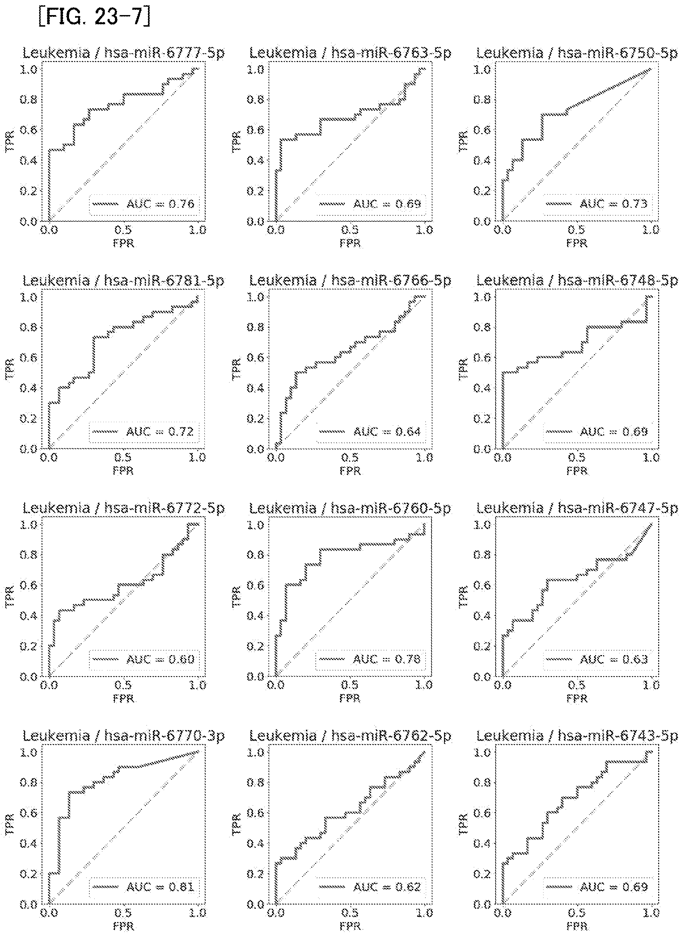

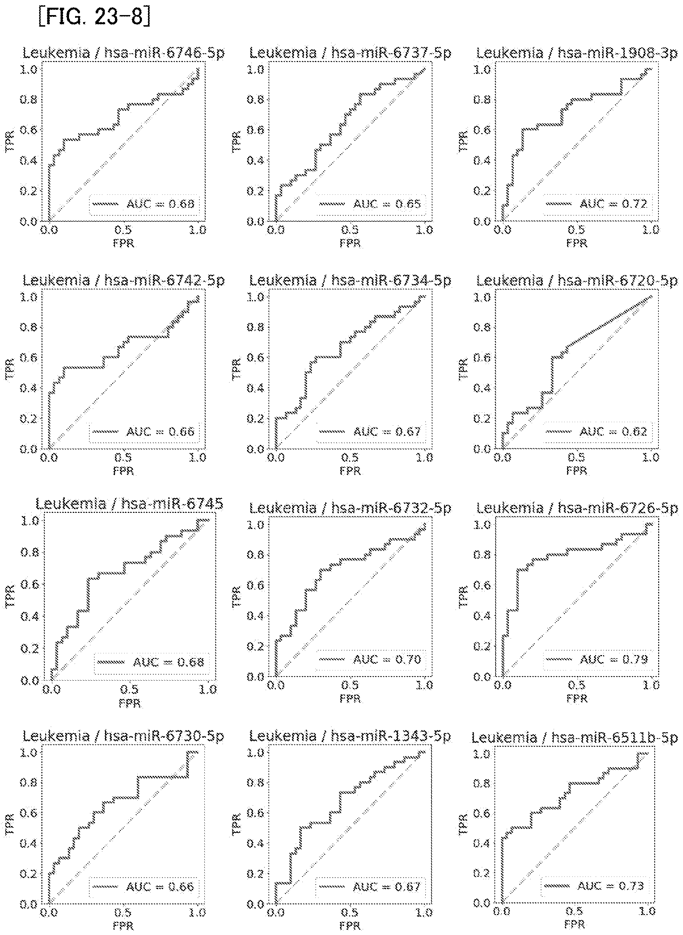

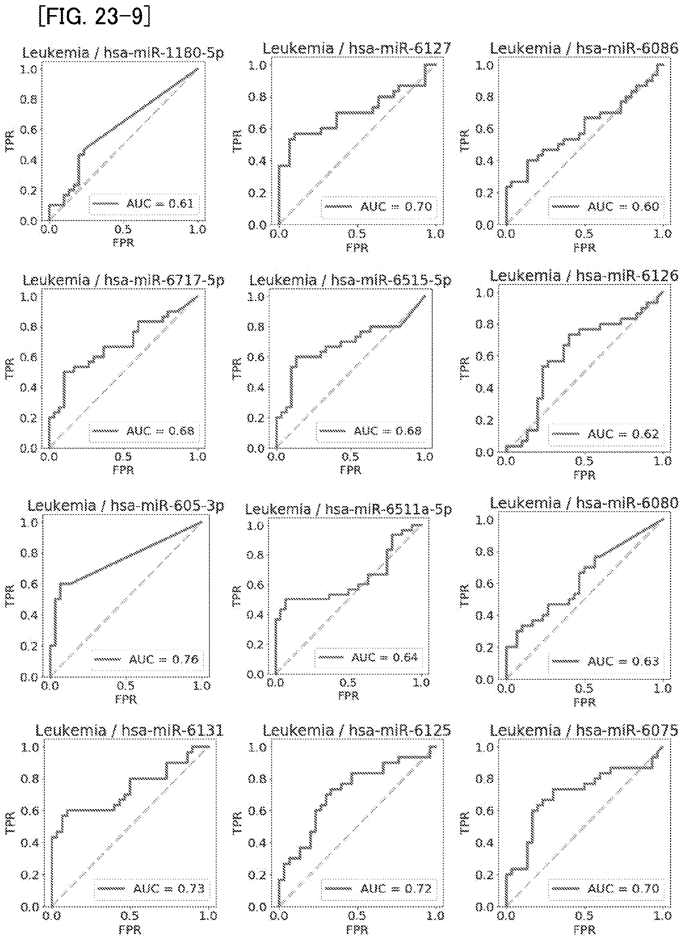

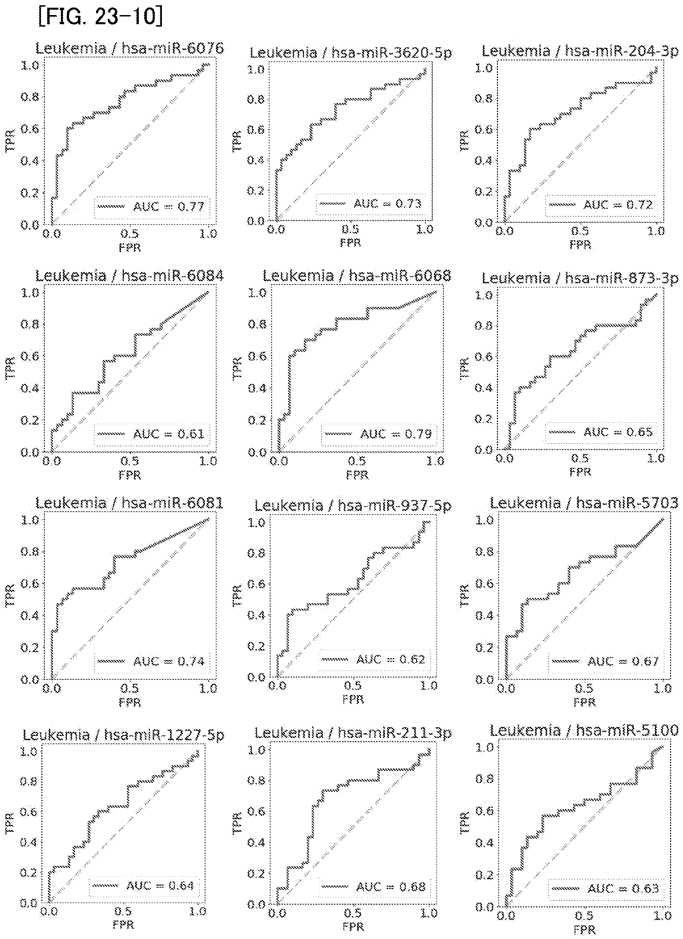

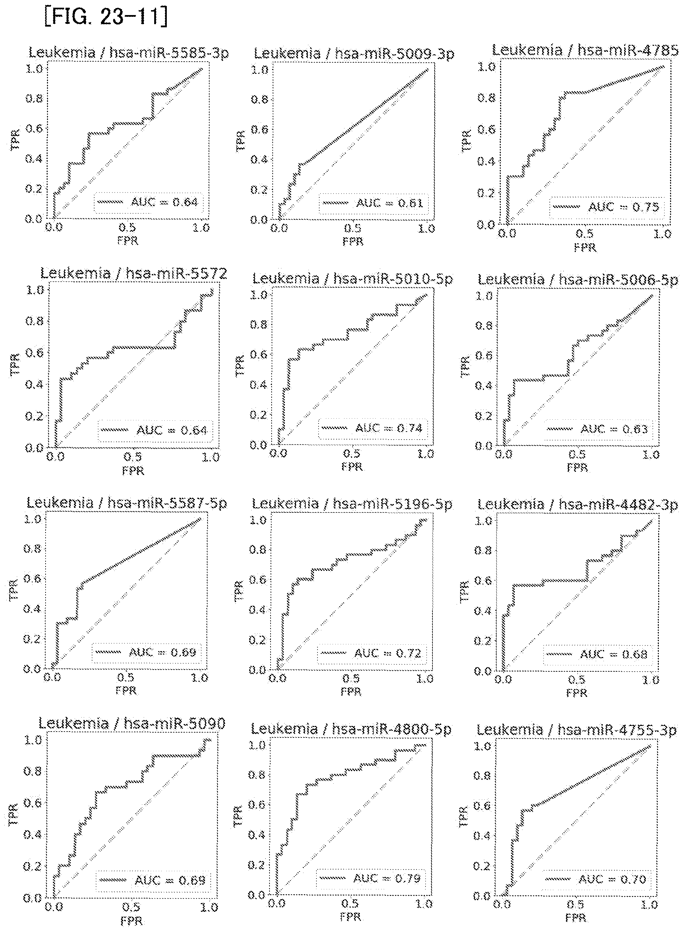

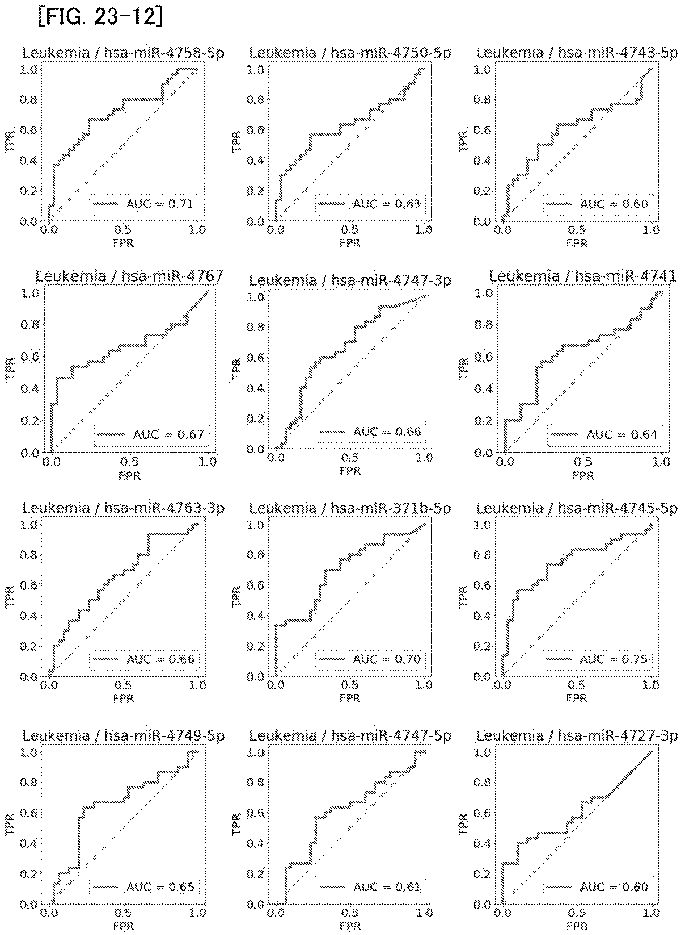

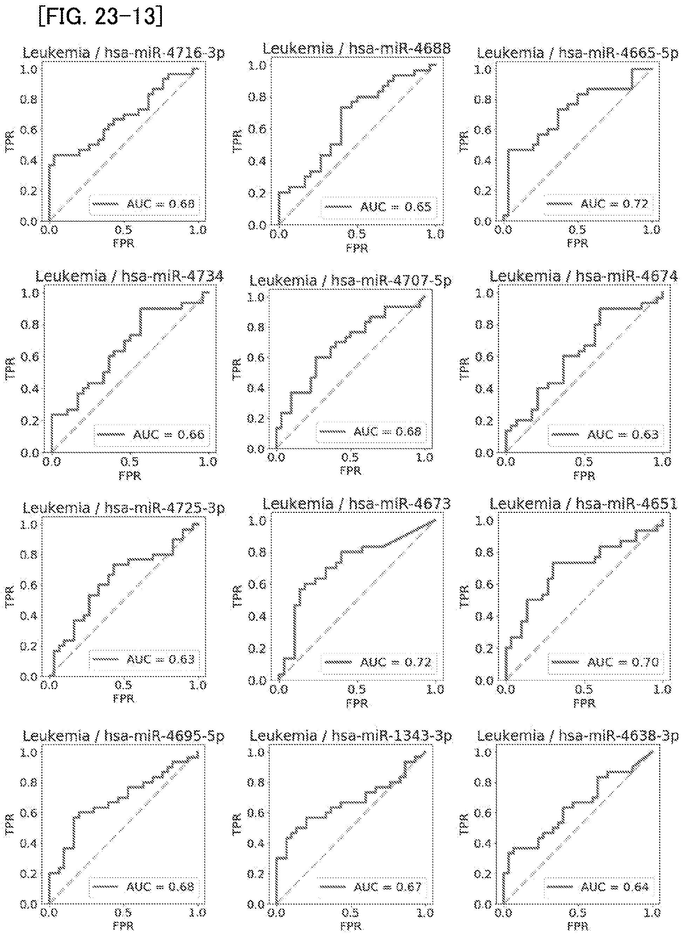

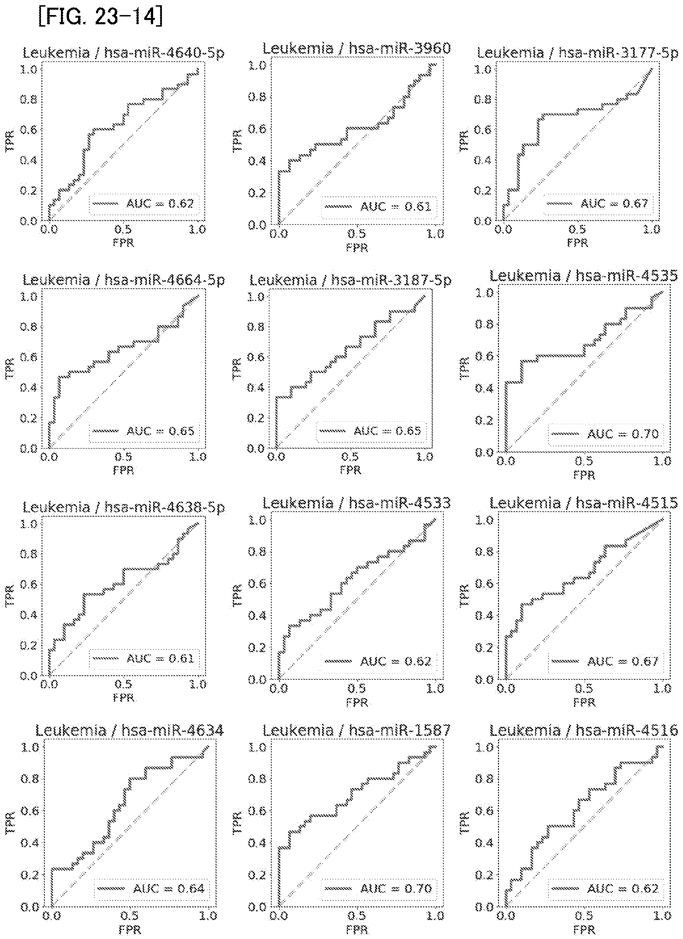

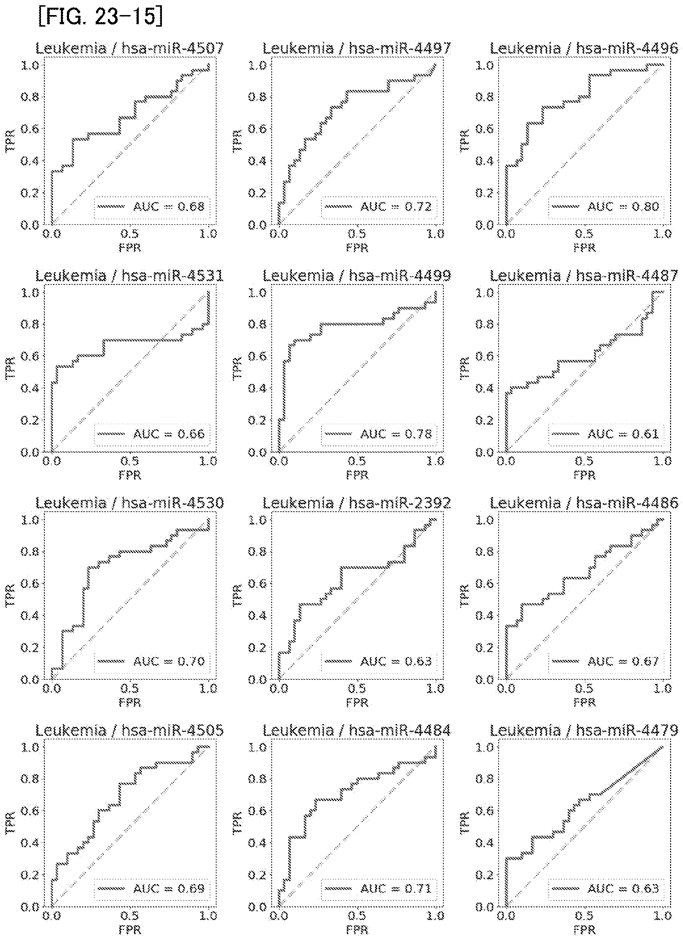

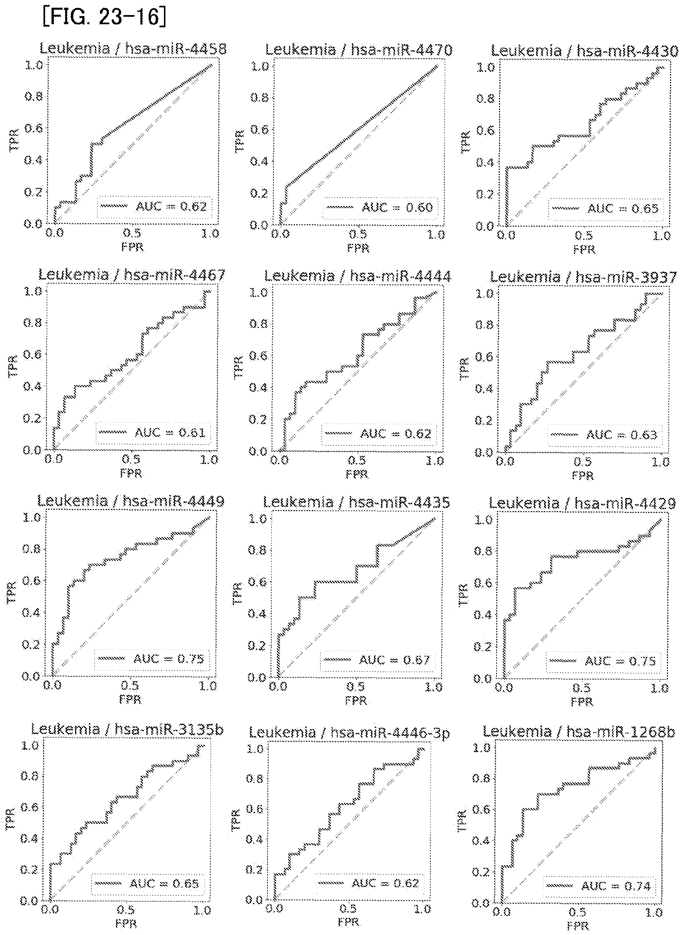

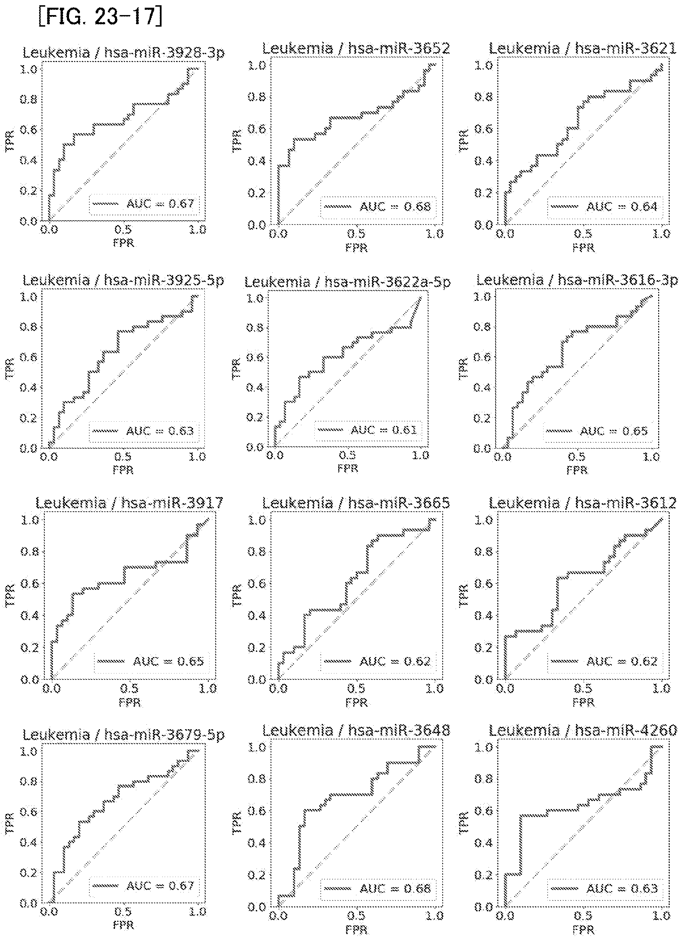

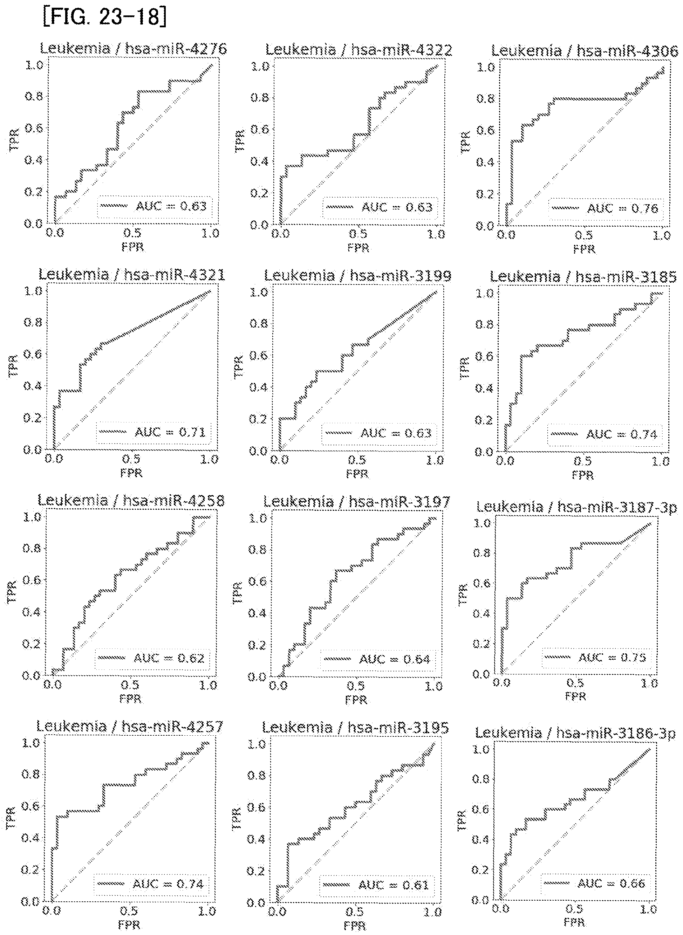

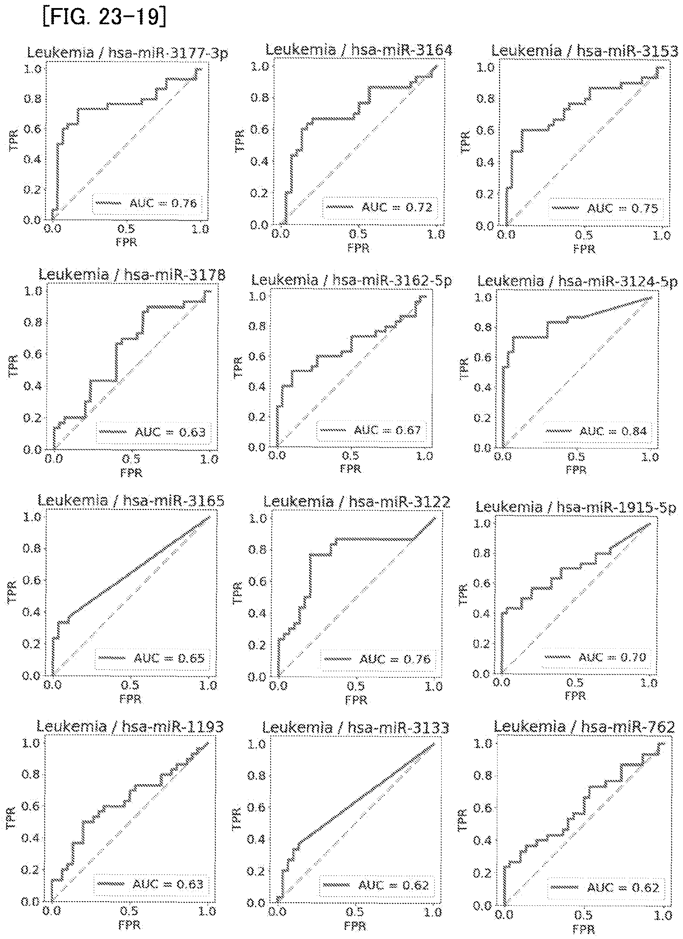

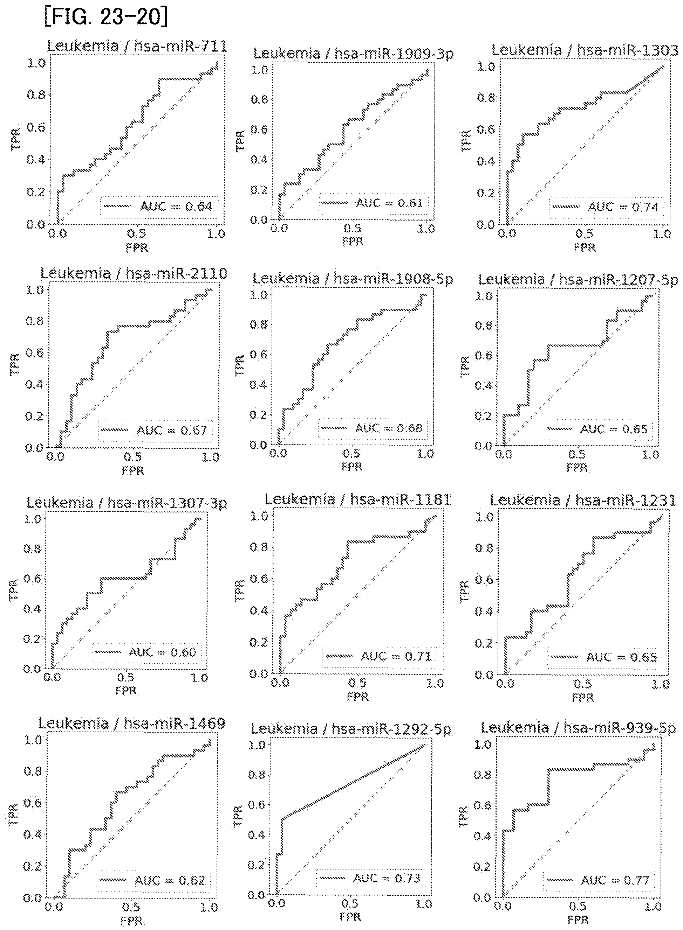

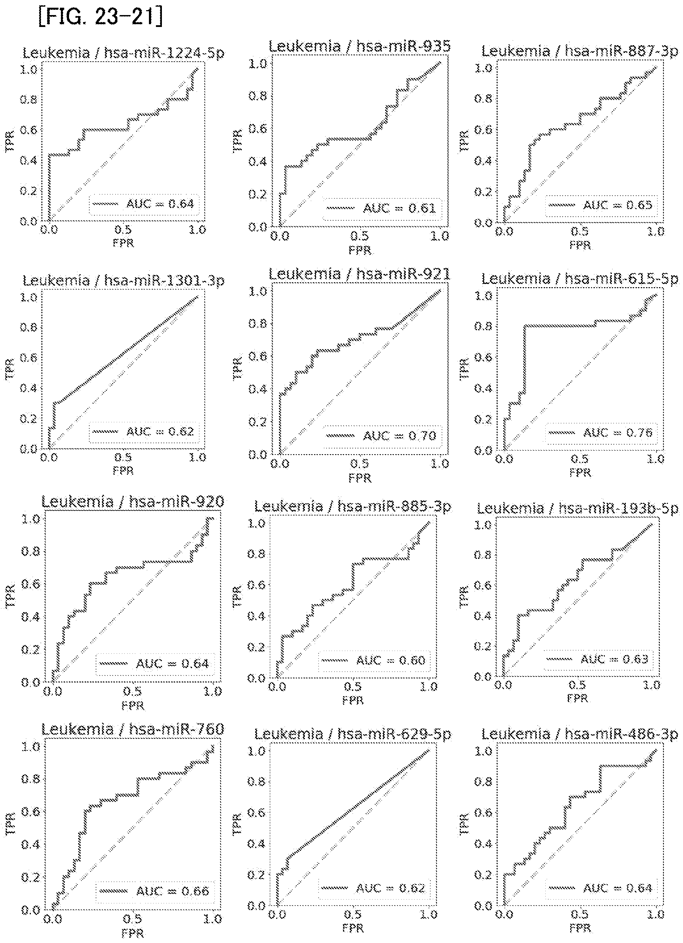

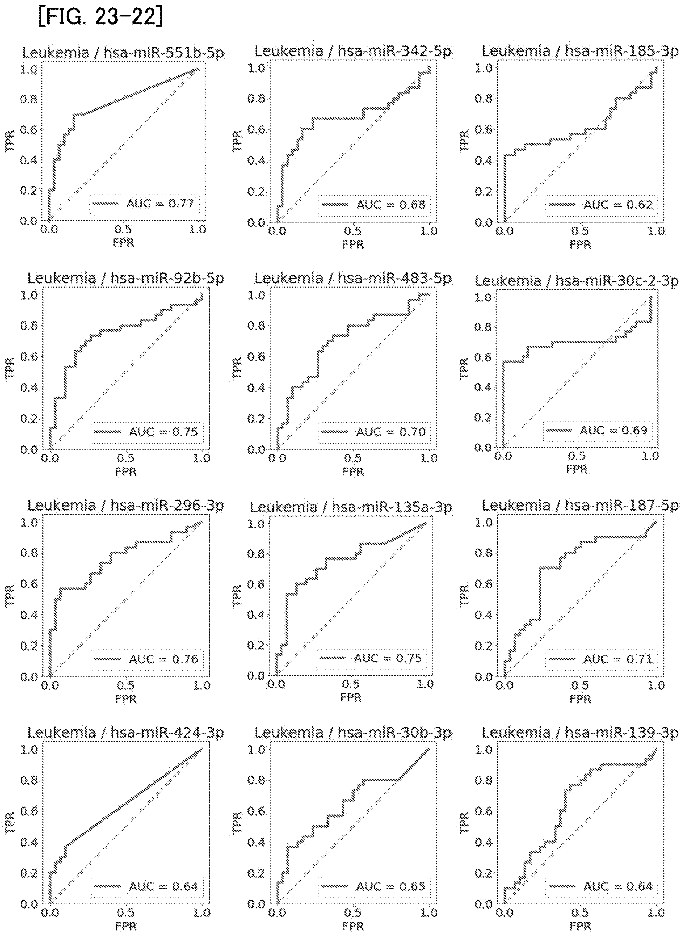

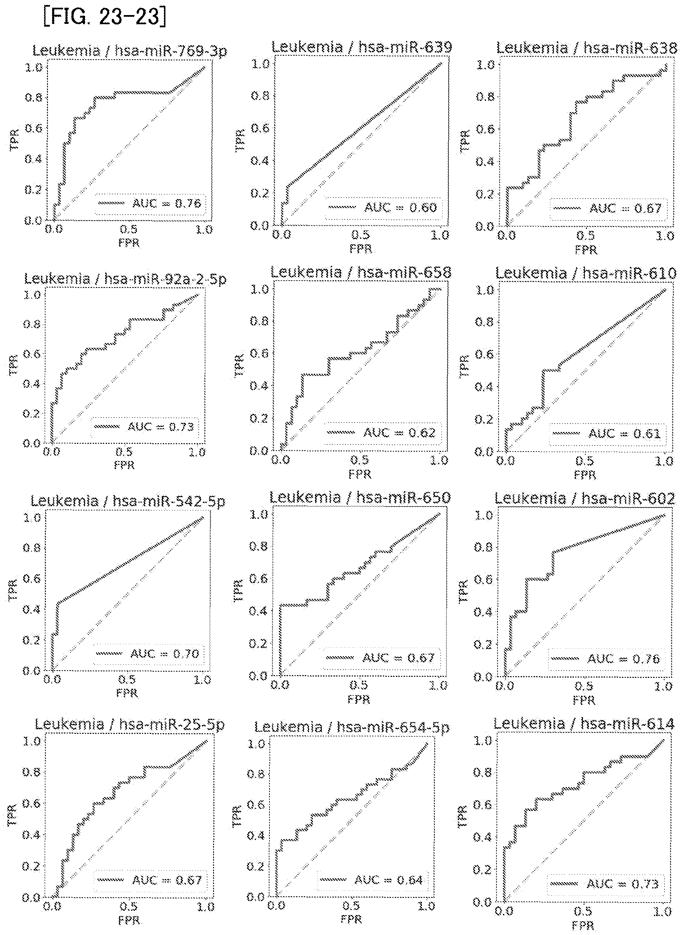

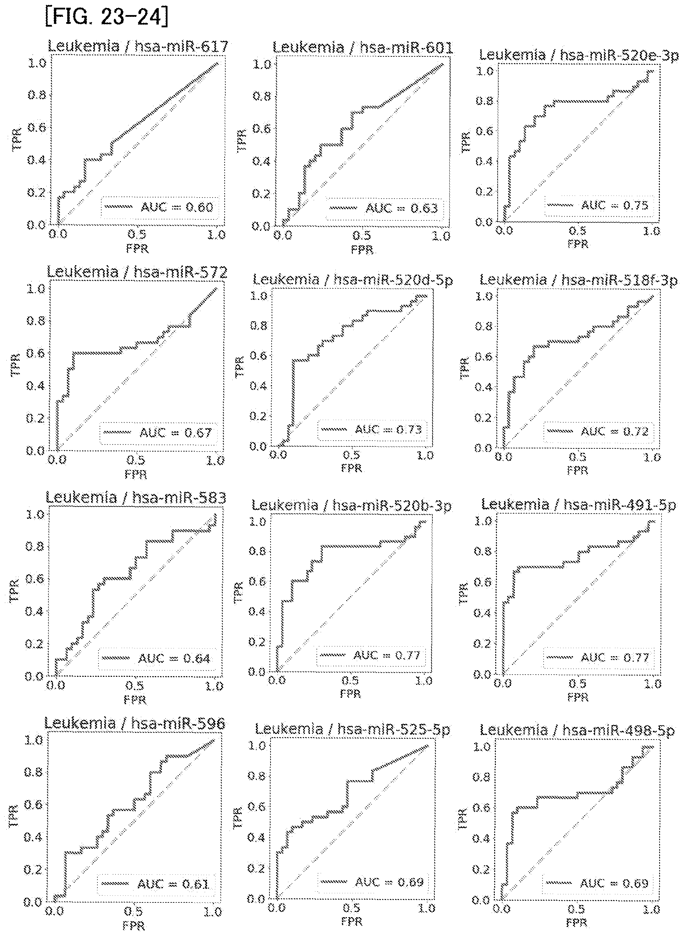

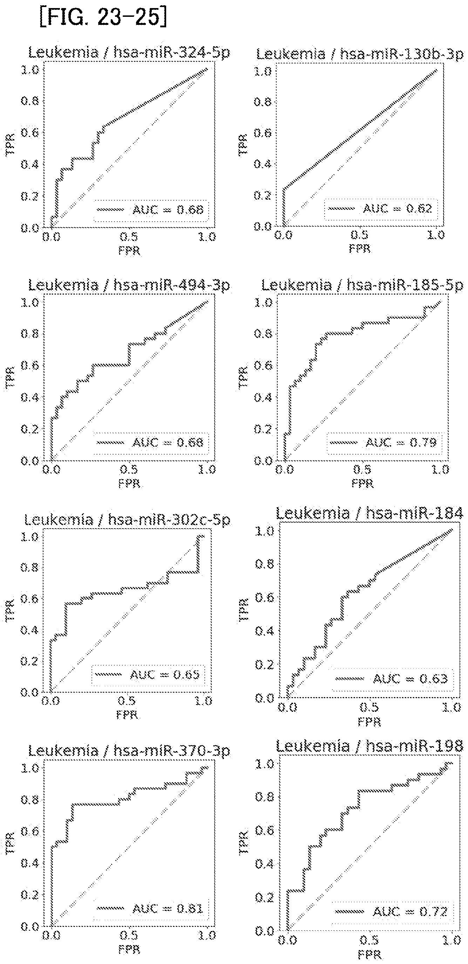

FIGS. 23-1 to 23-25 show ROC-curves for predicting leukemias in humans using a single miRNA.

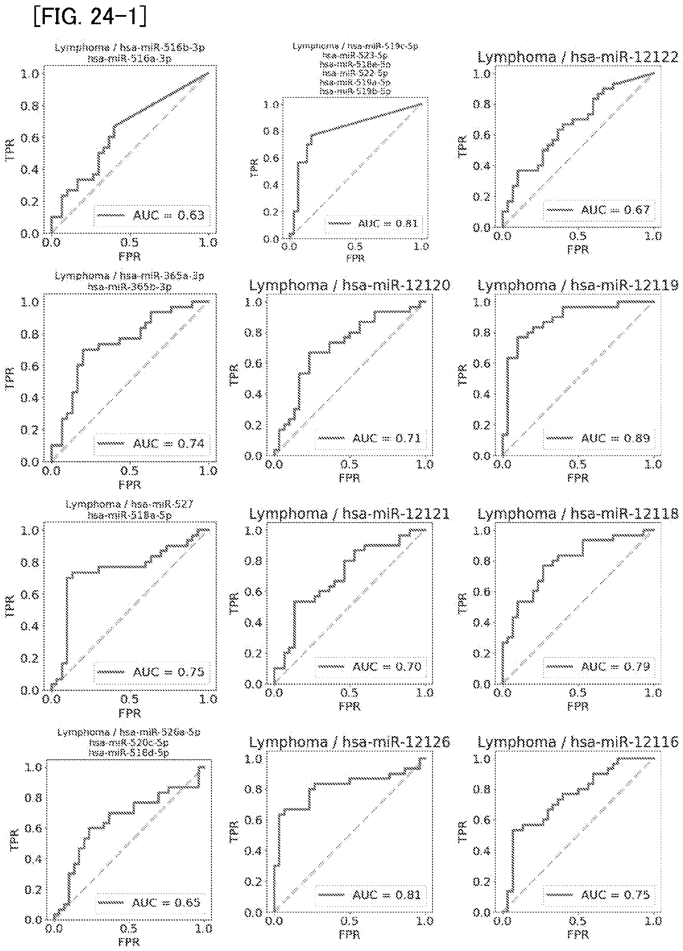

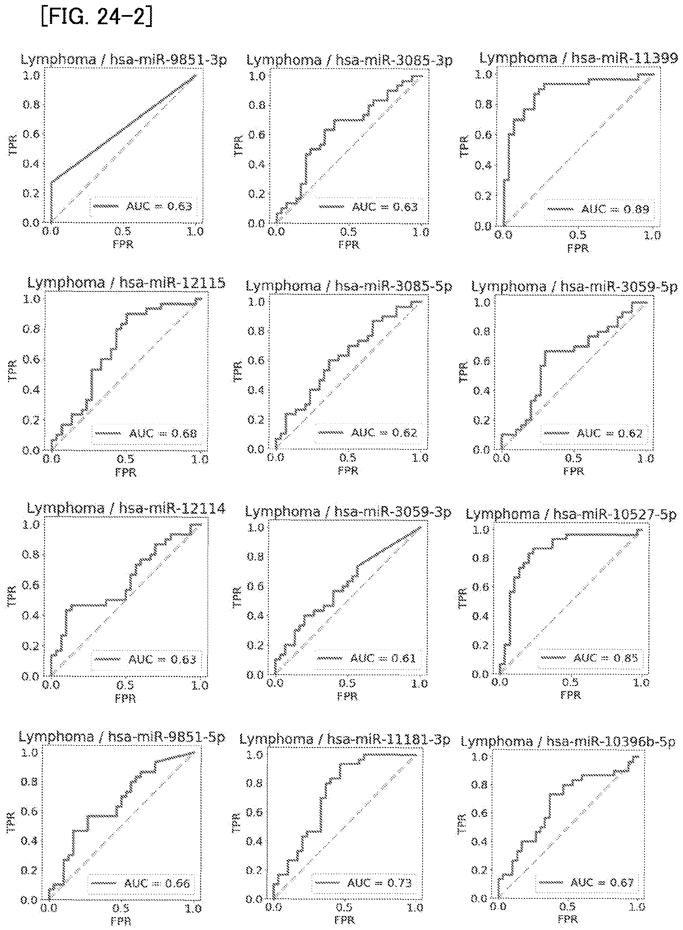

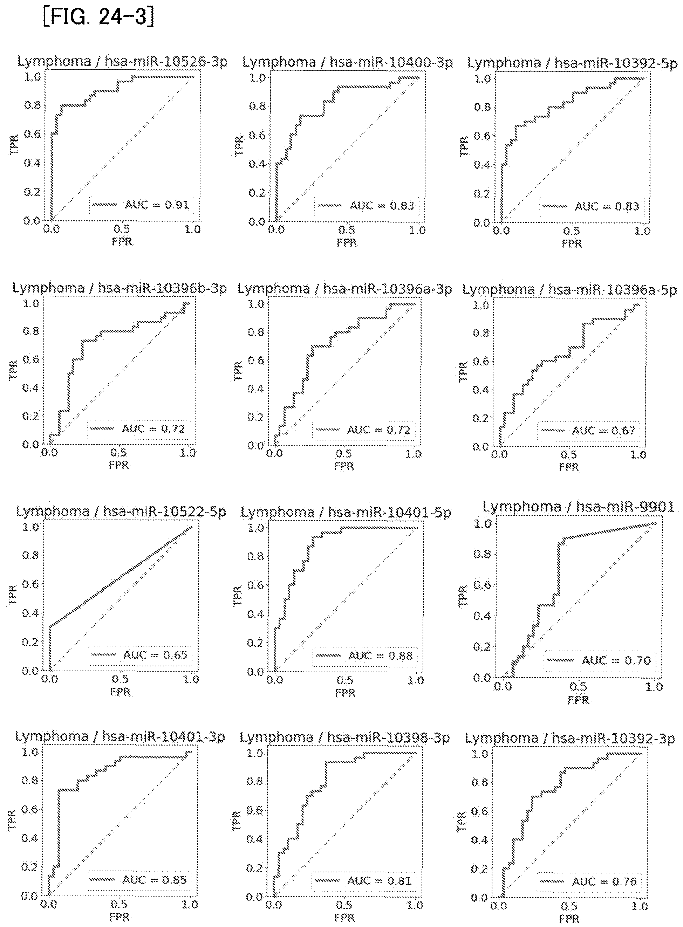

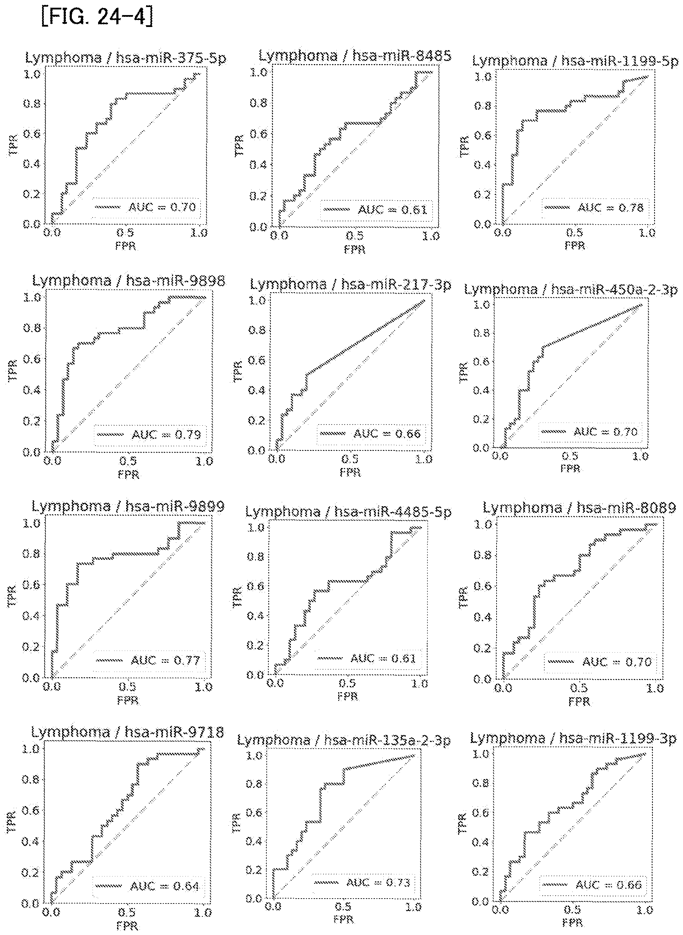

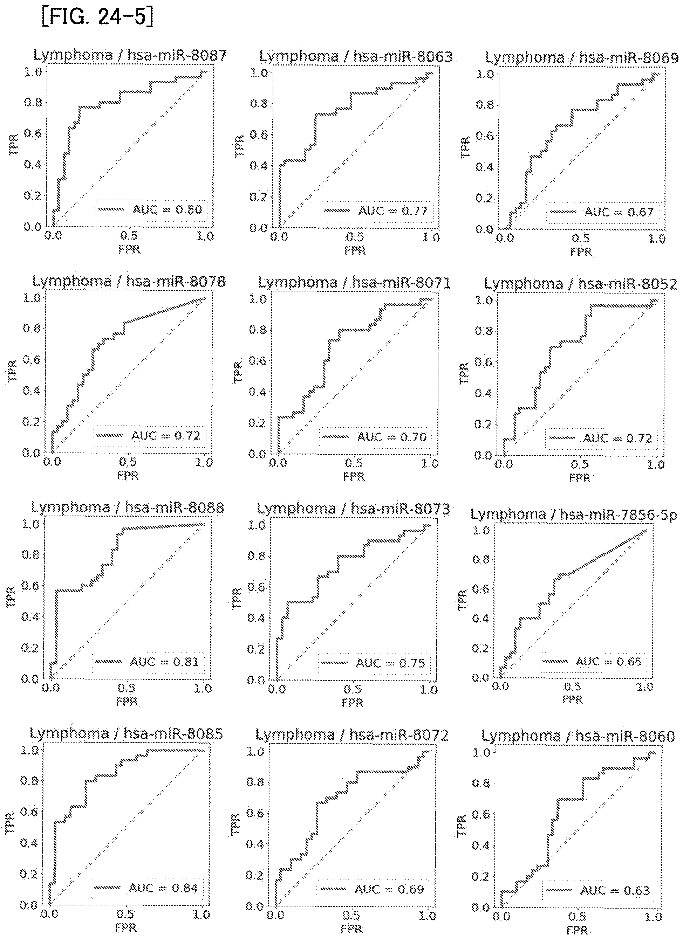

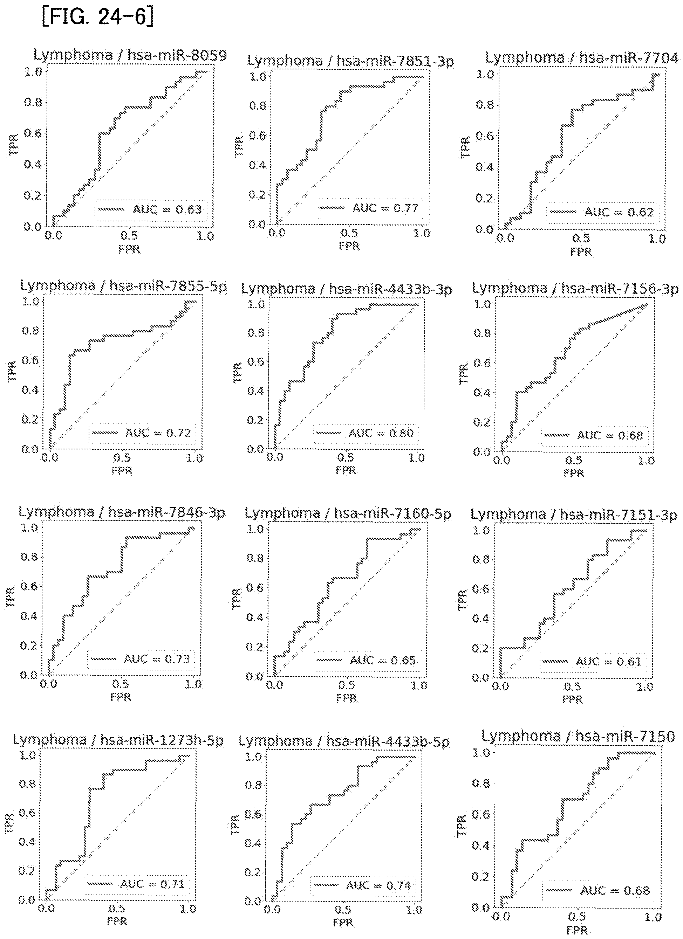

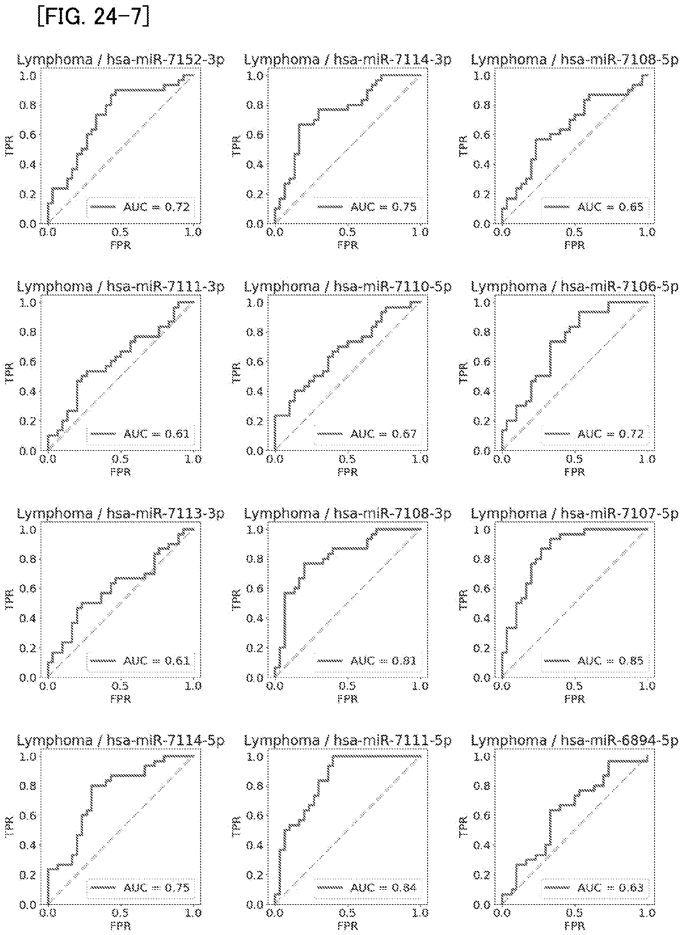

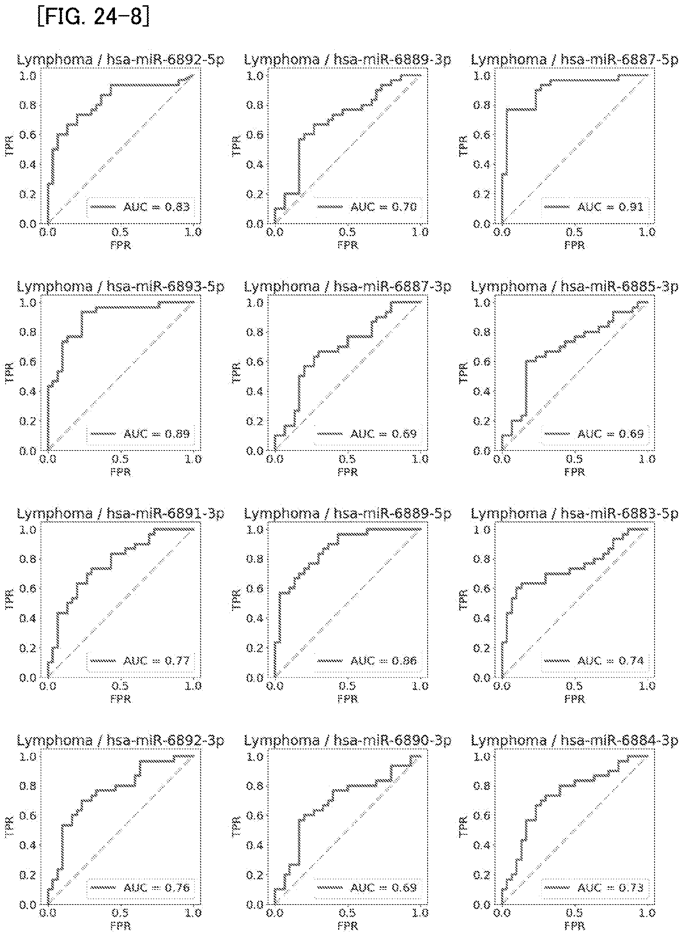

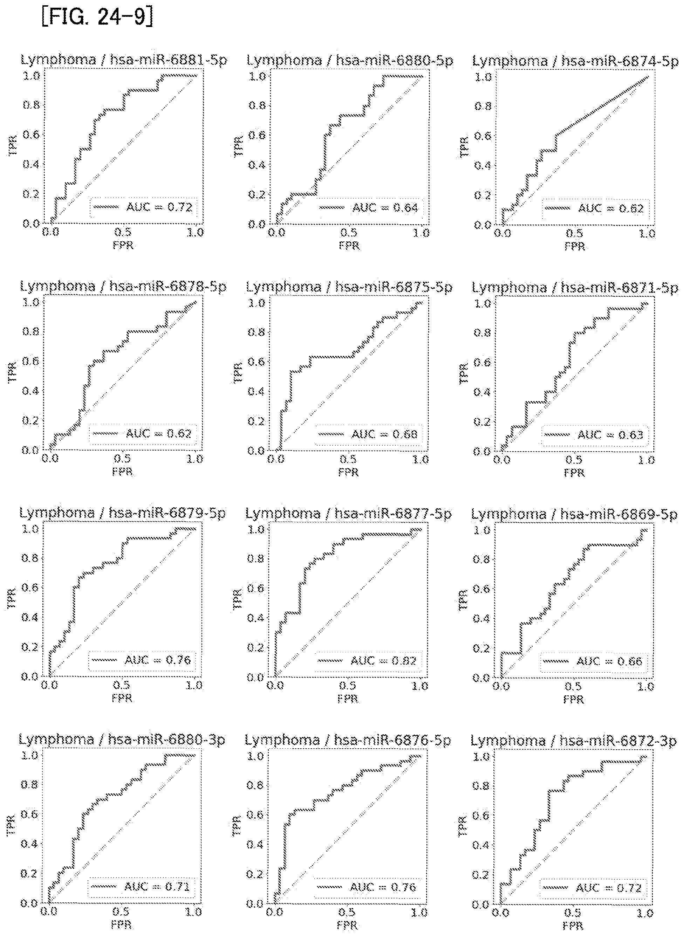

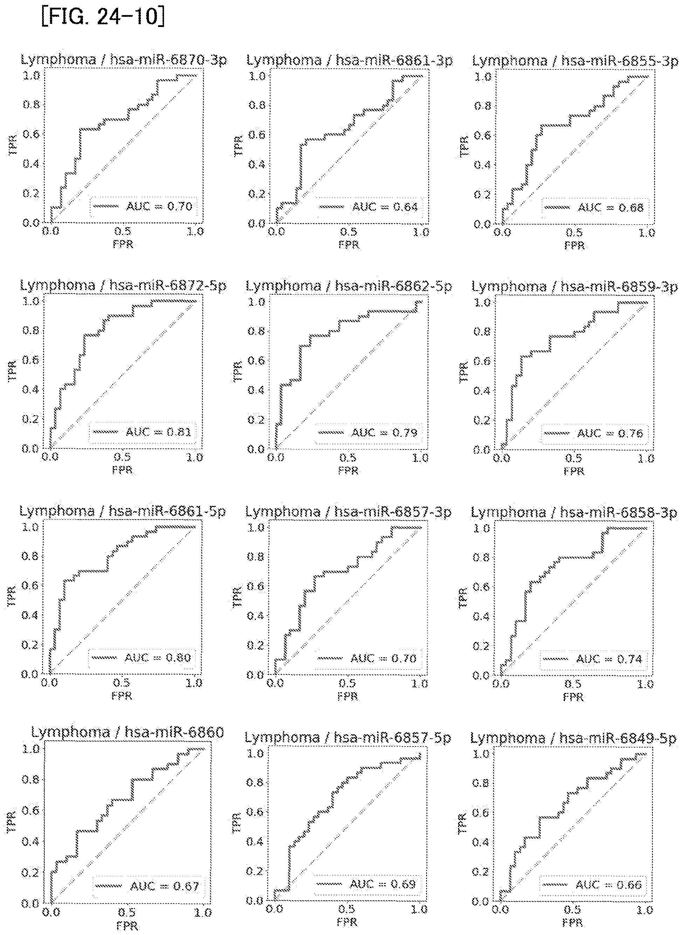

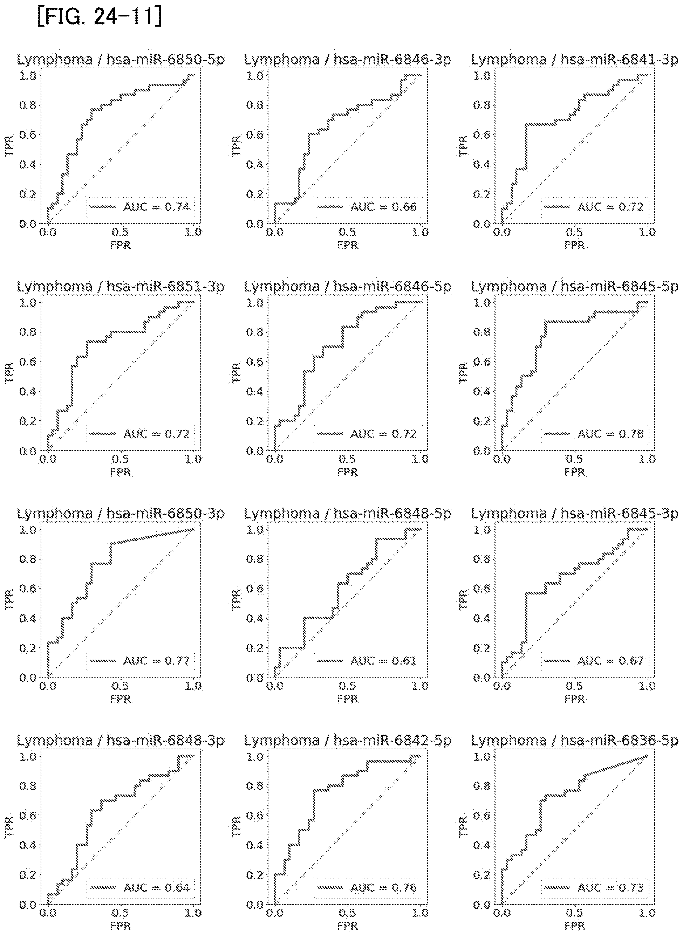

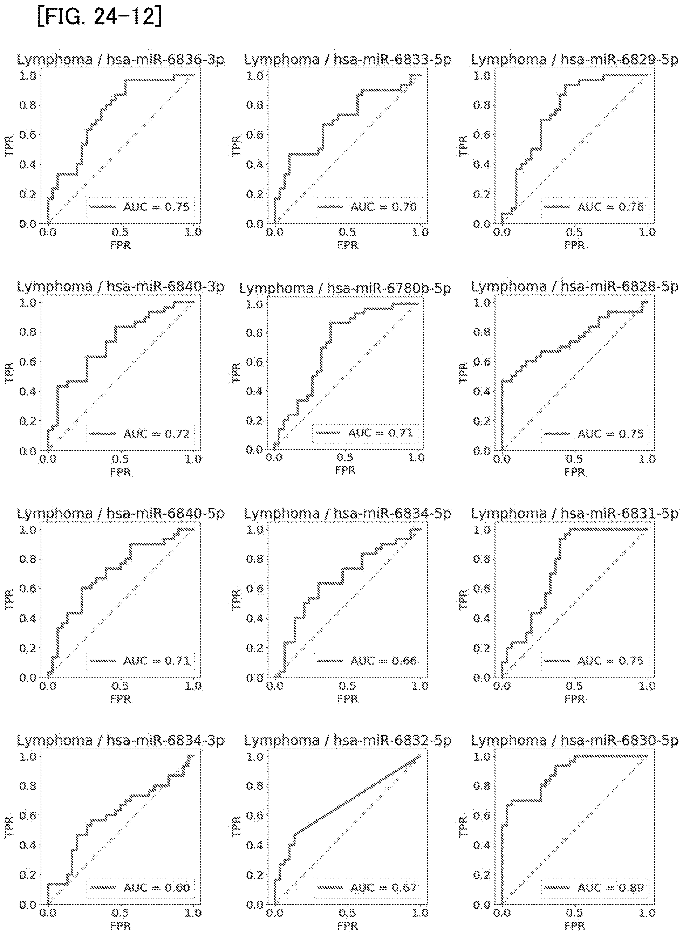

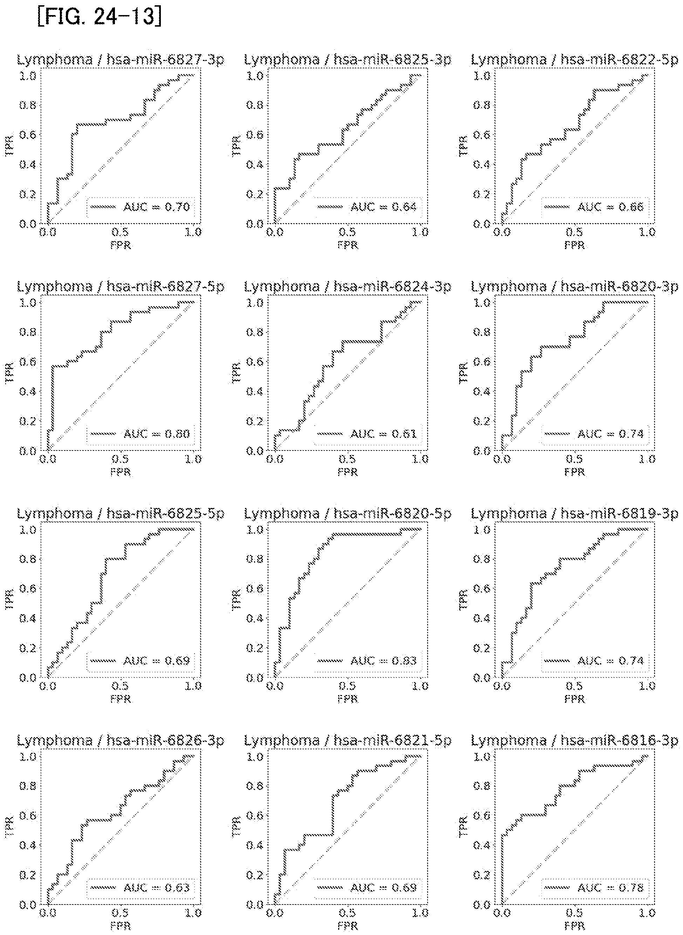

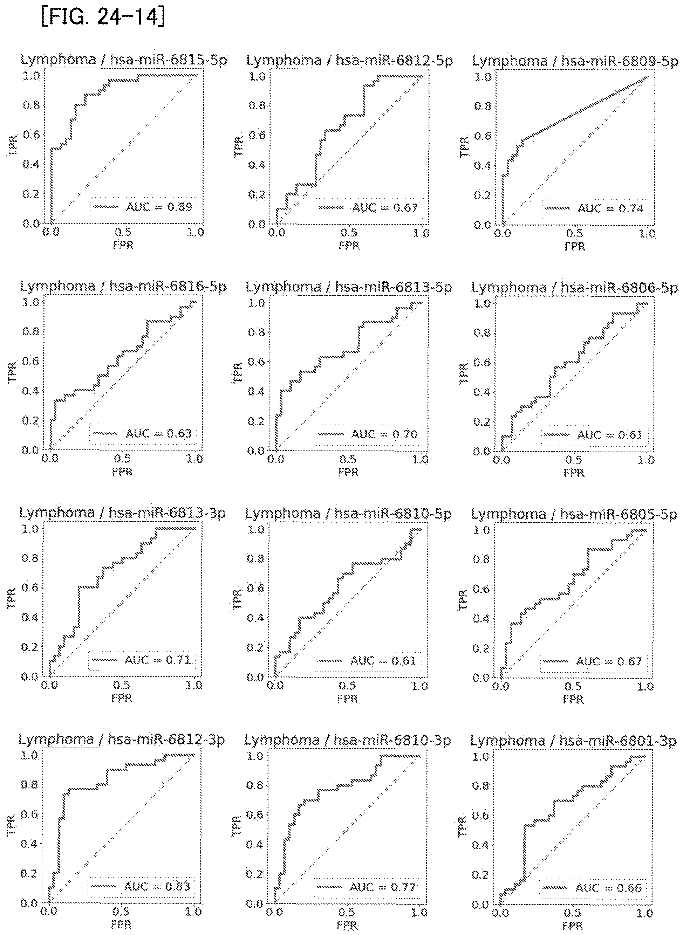

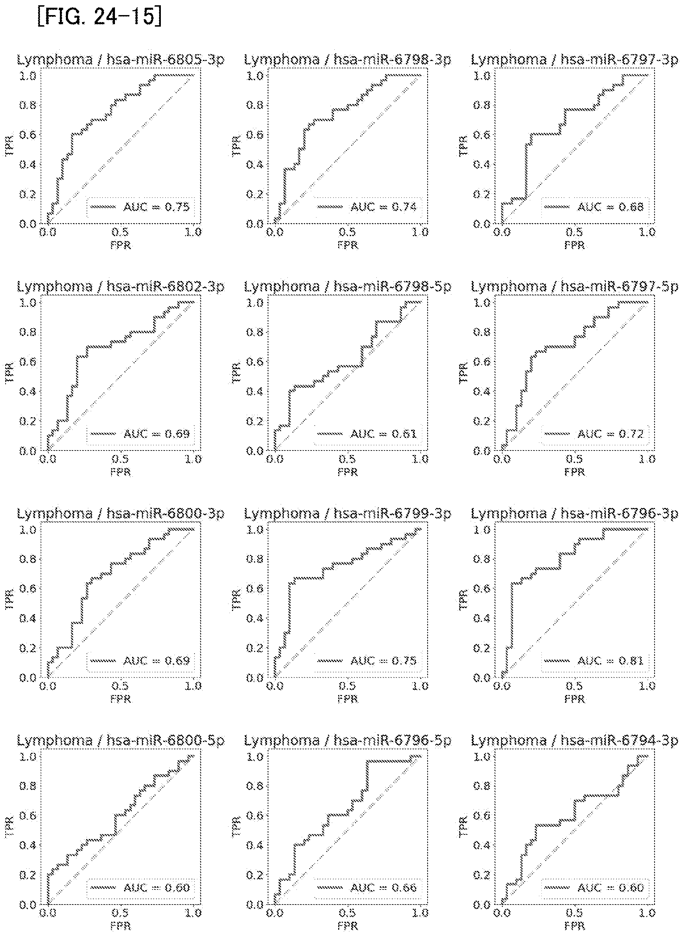

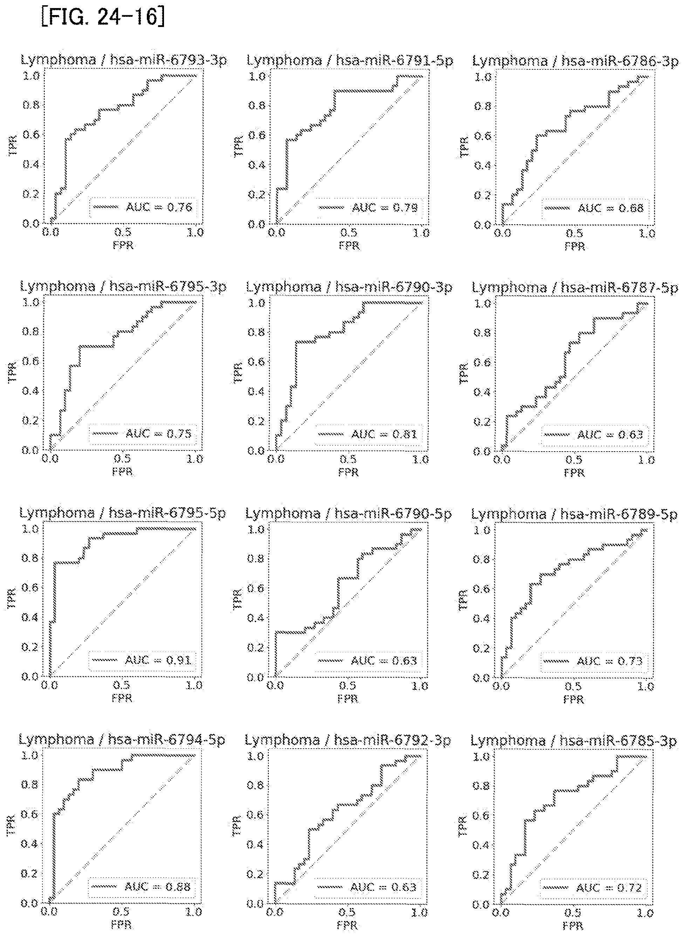

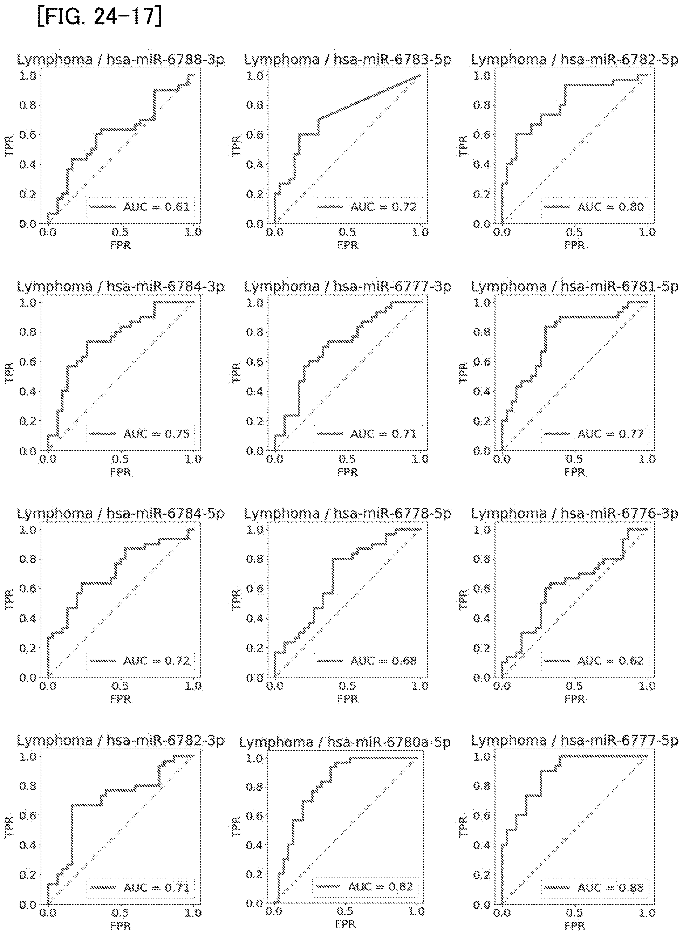

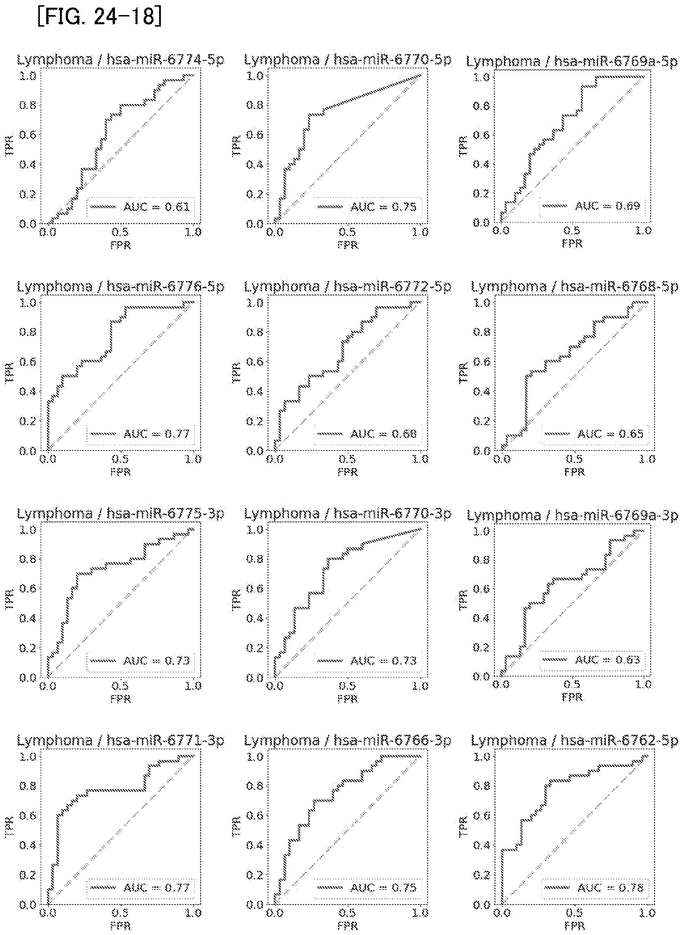

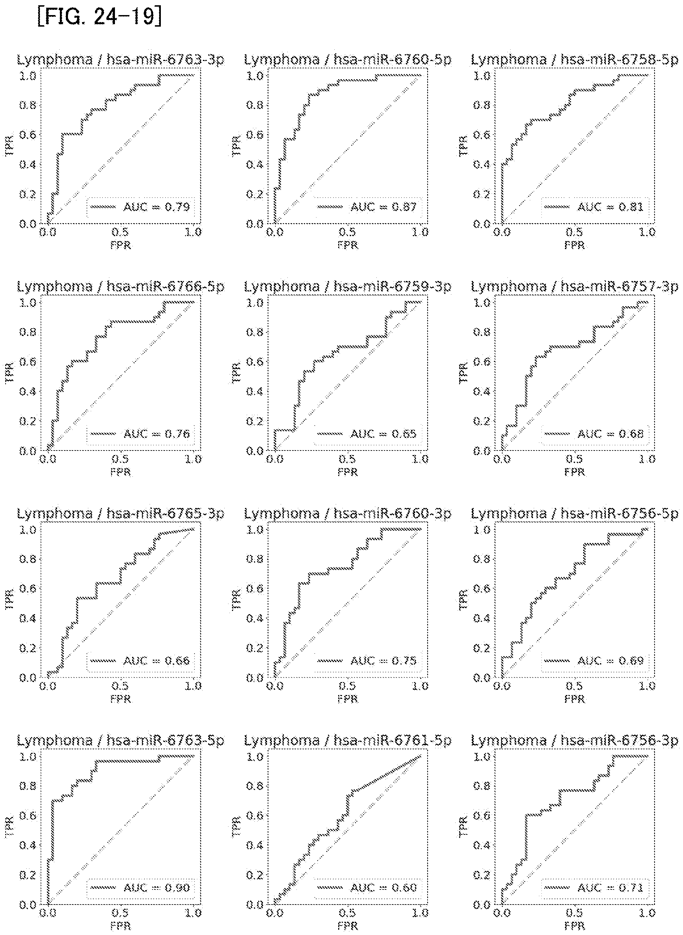

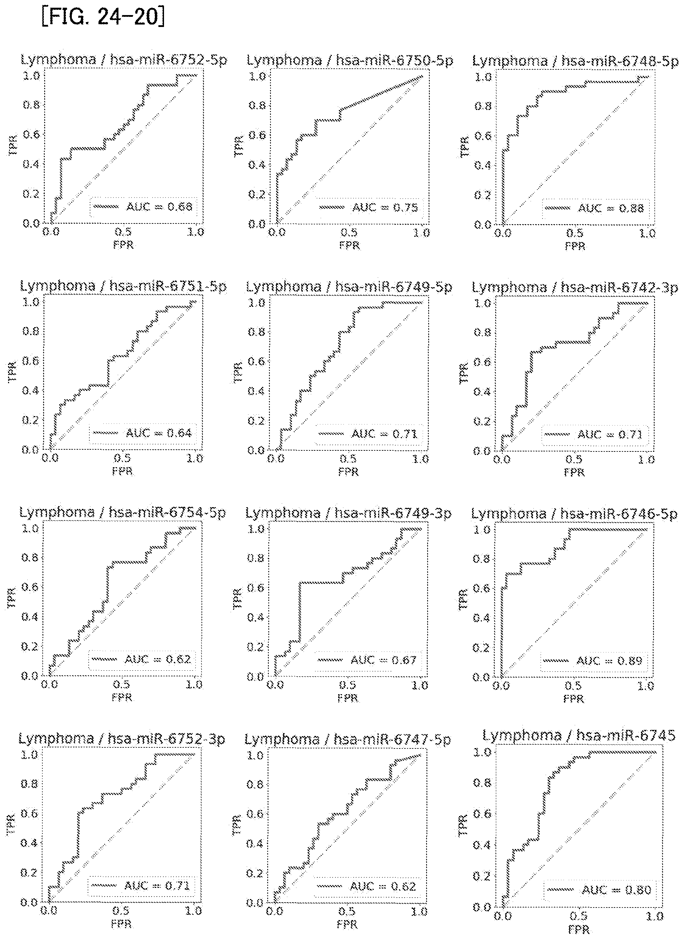

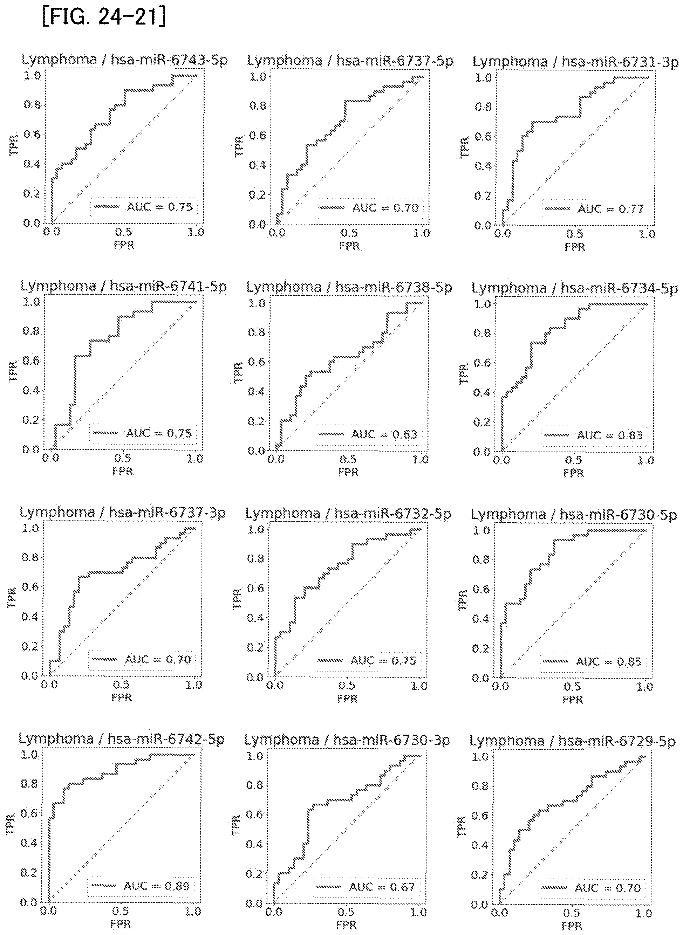

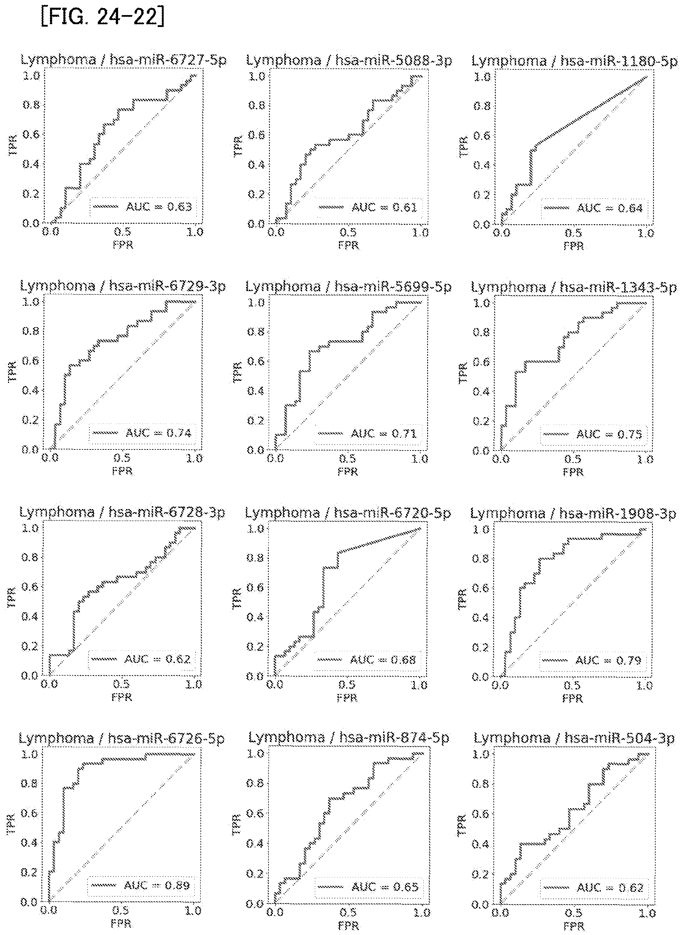

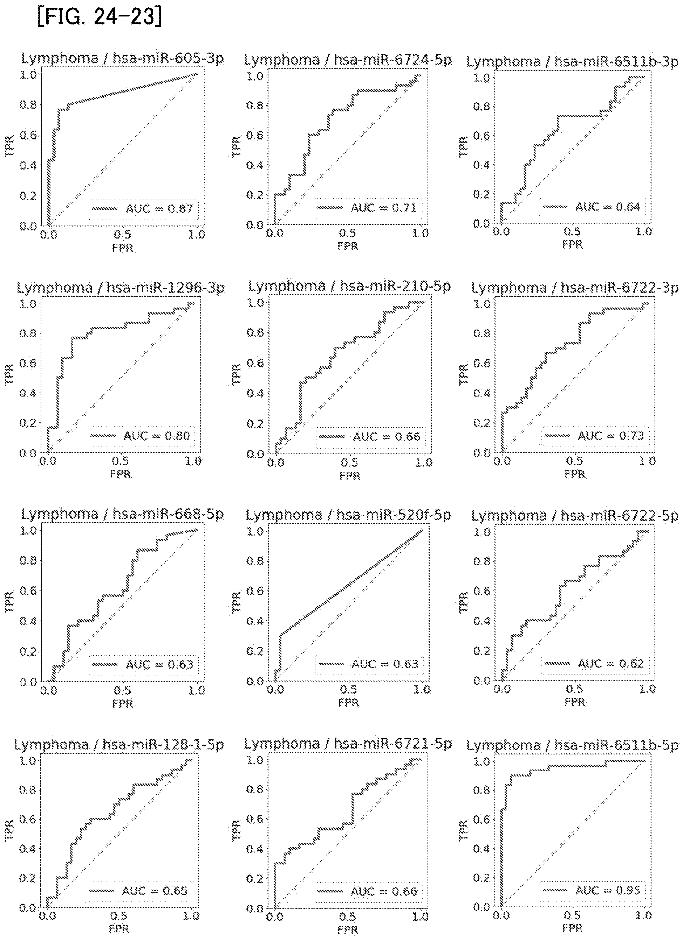

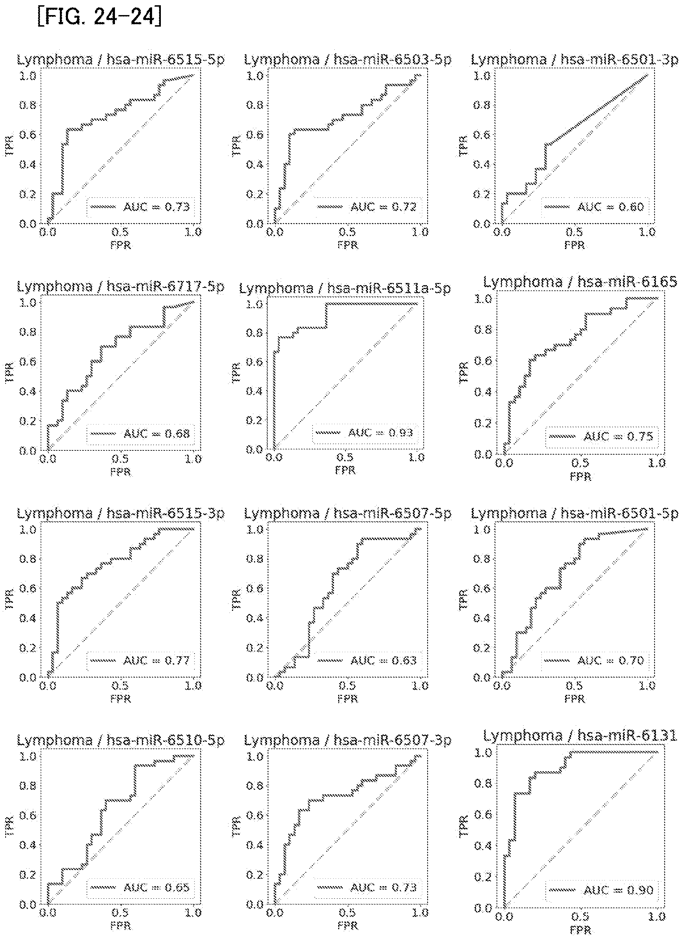

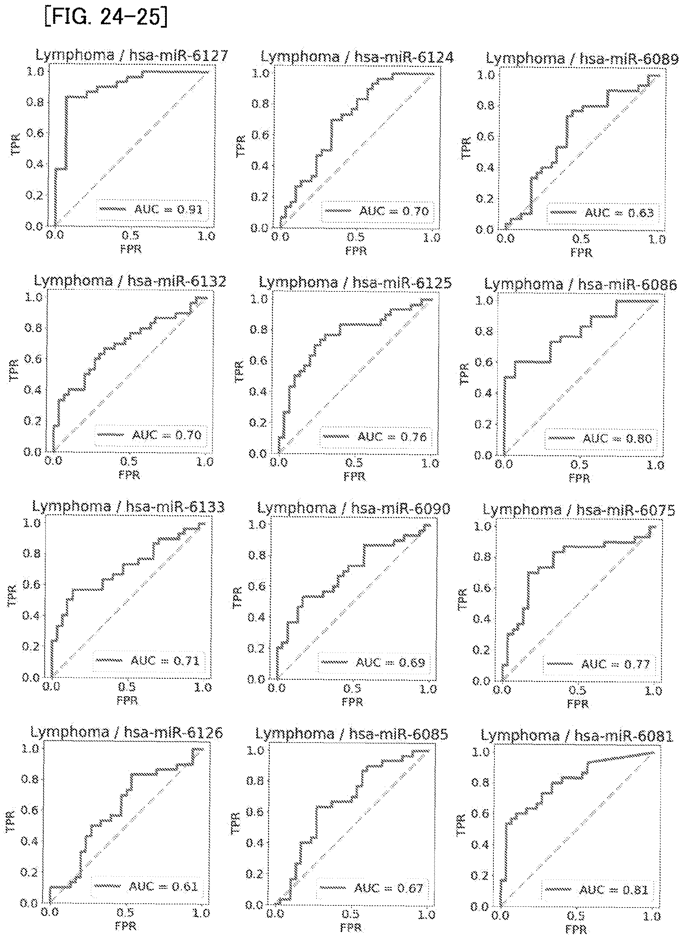

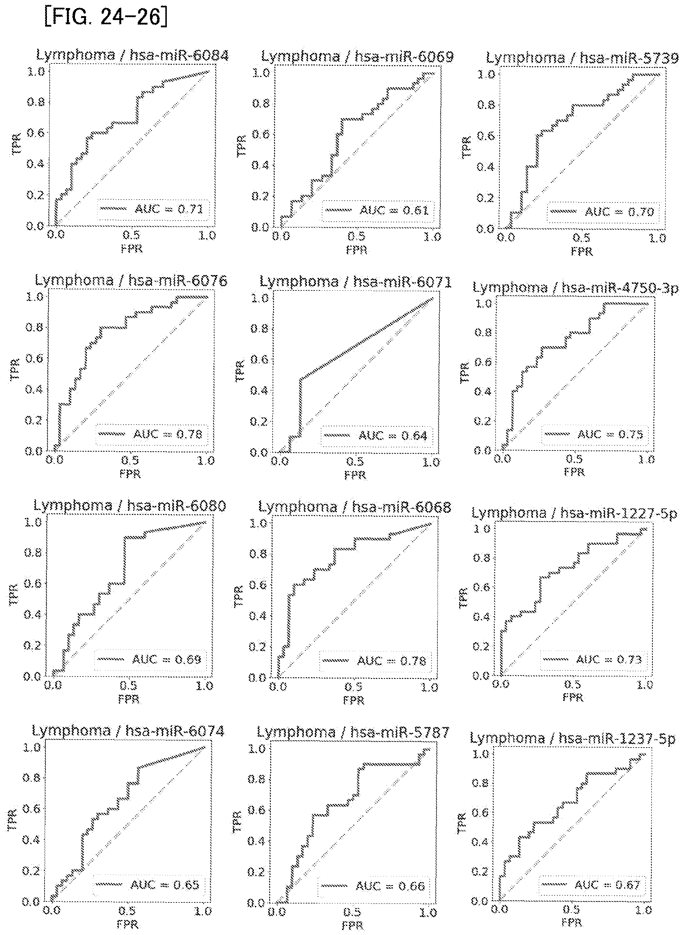

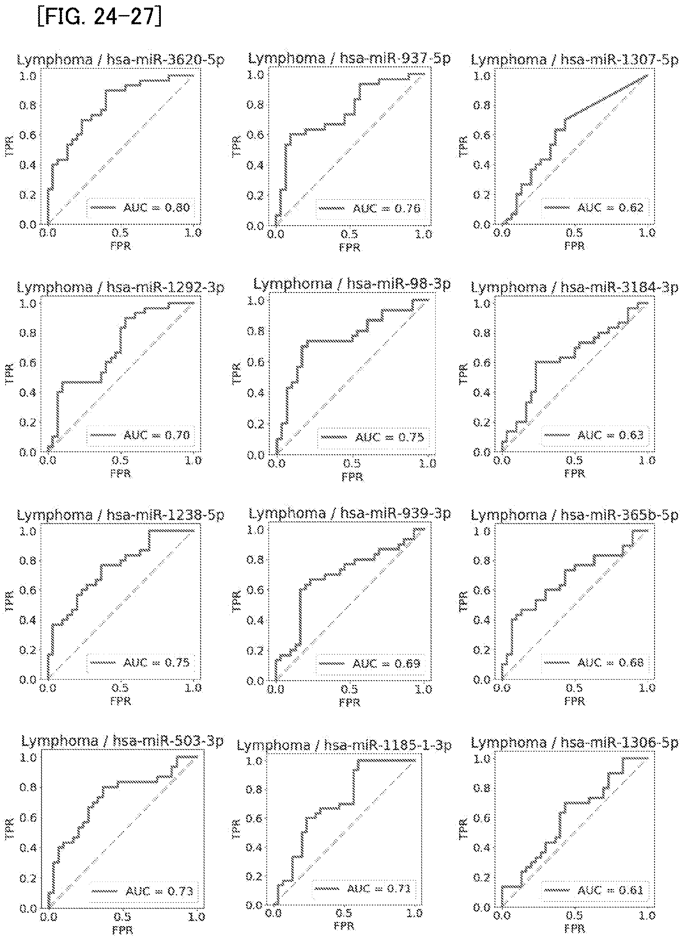

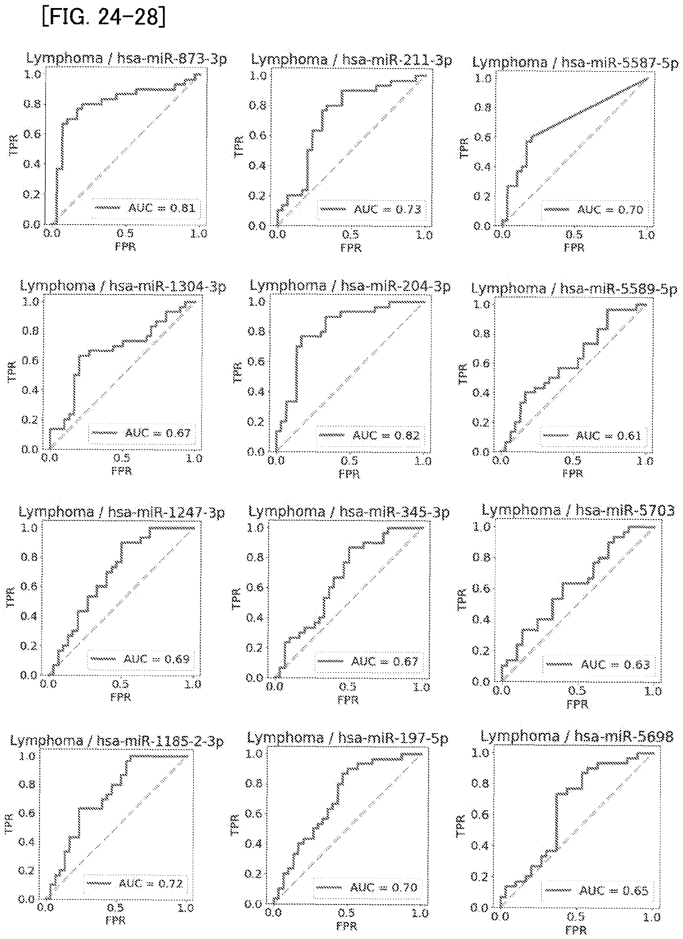

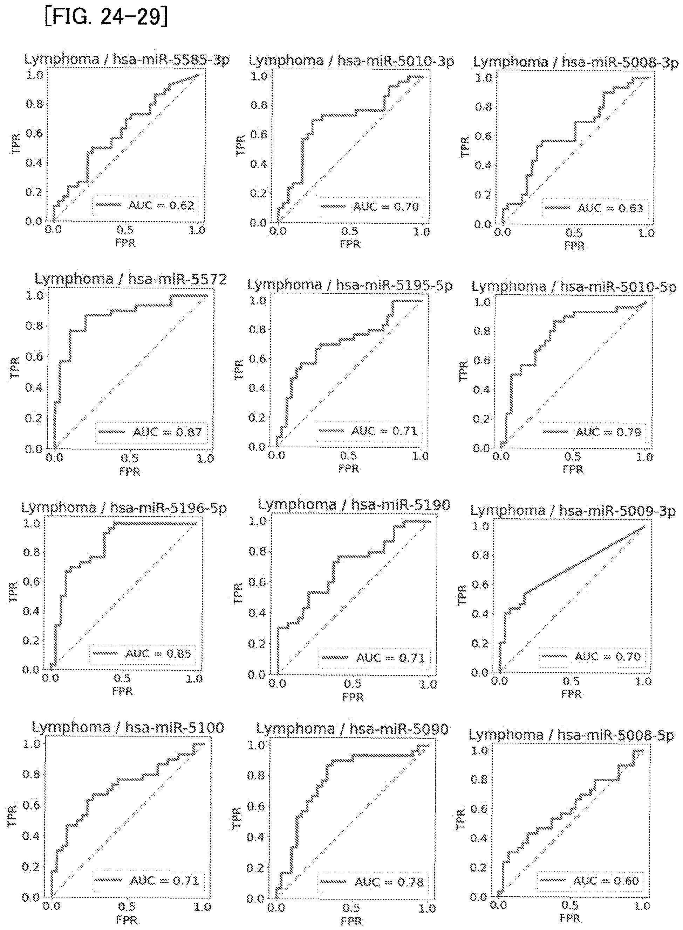

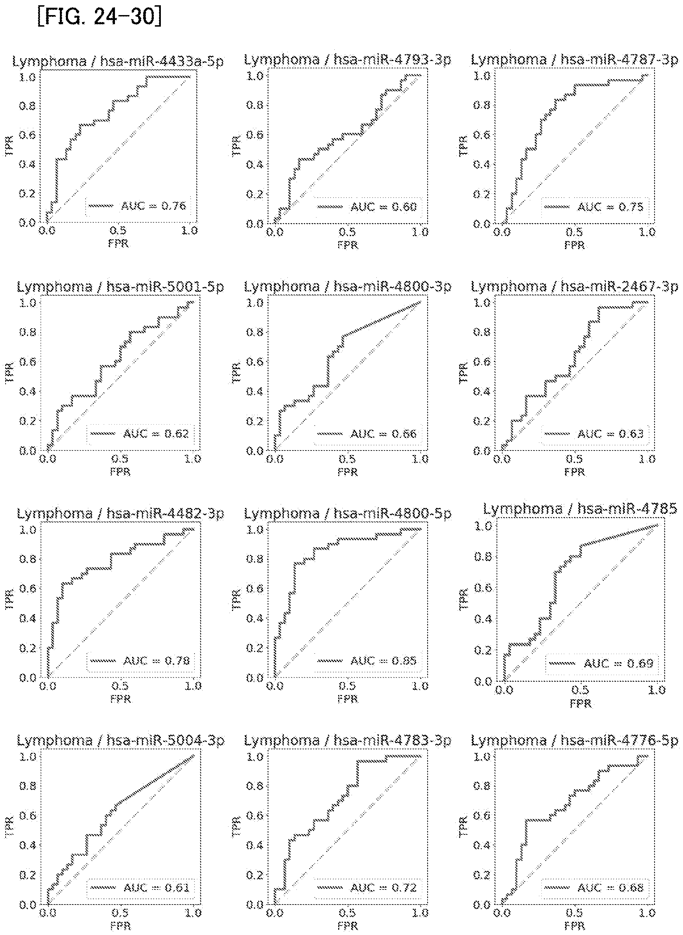

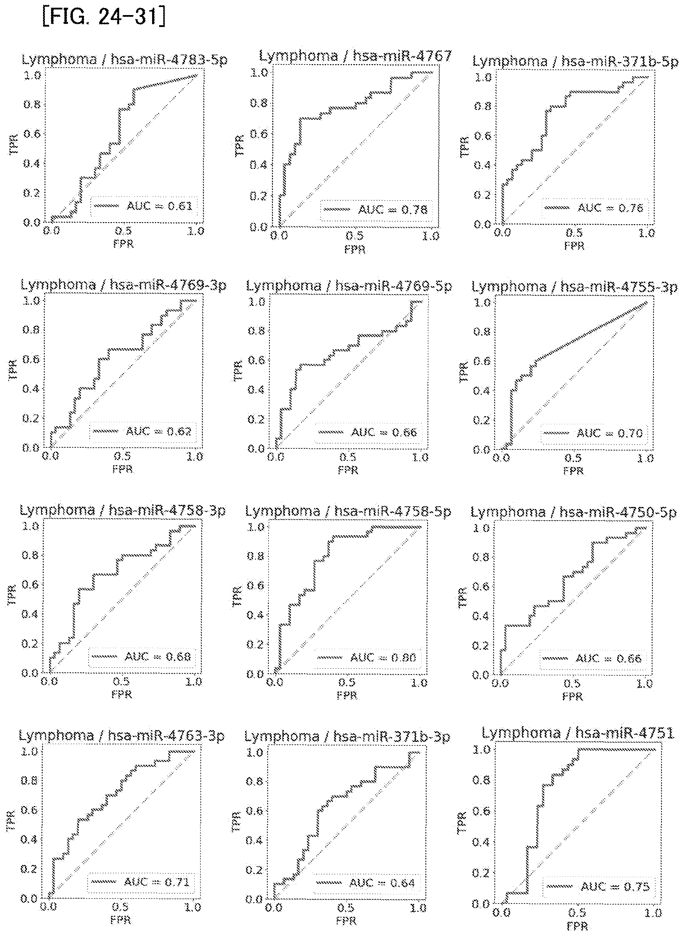

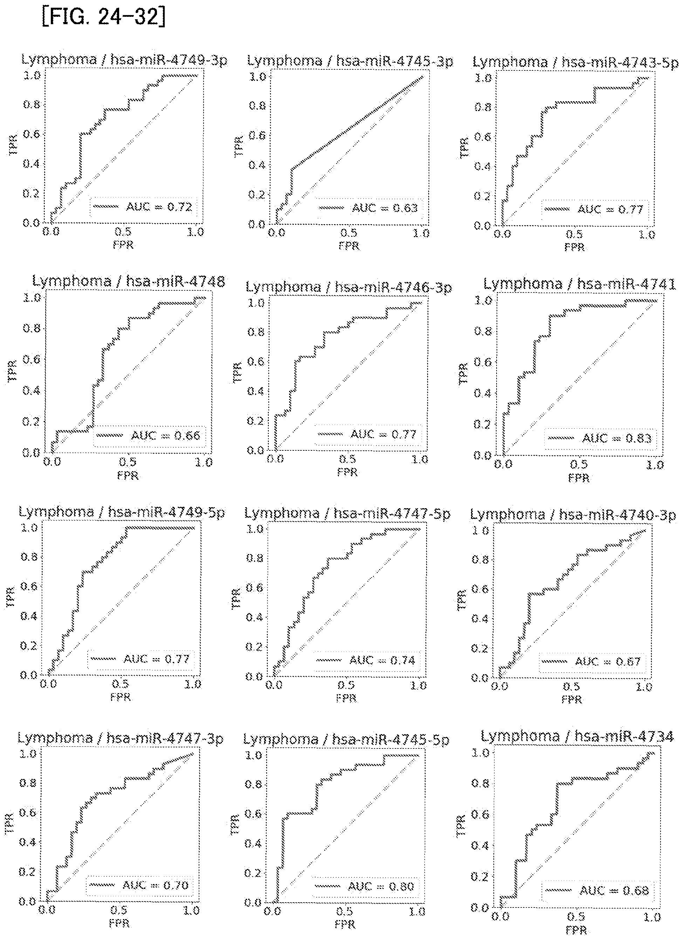

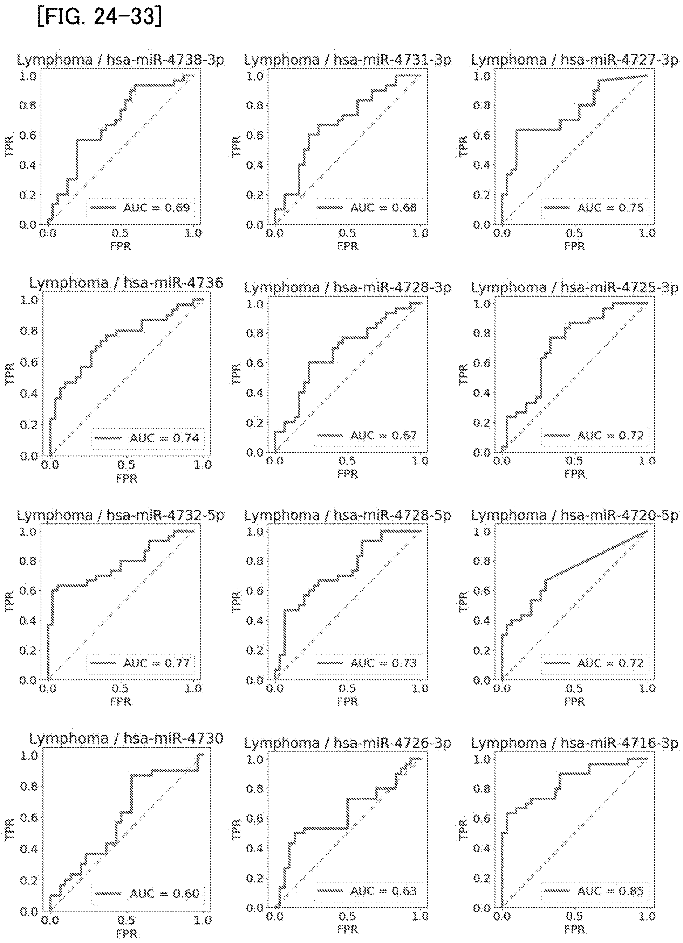

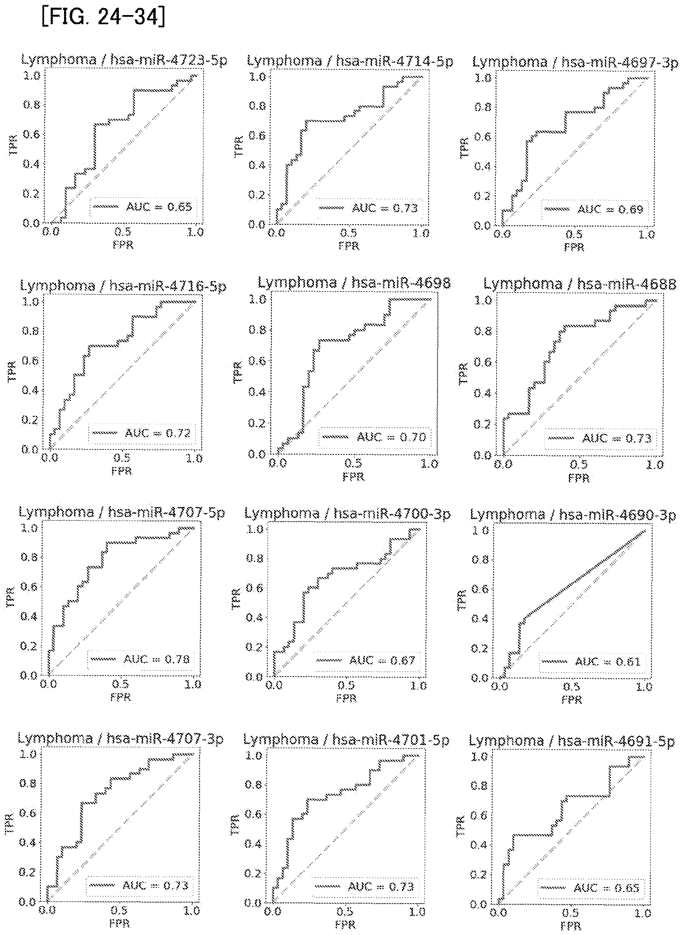

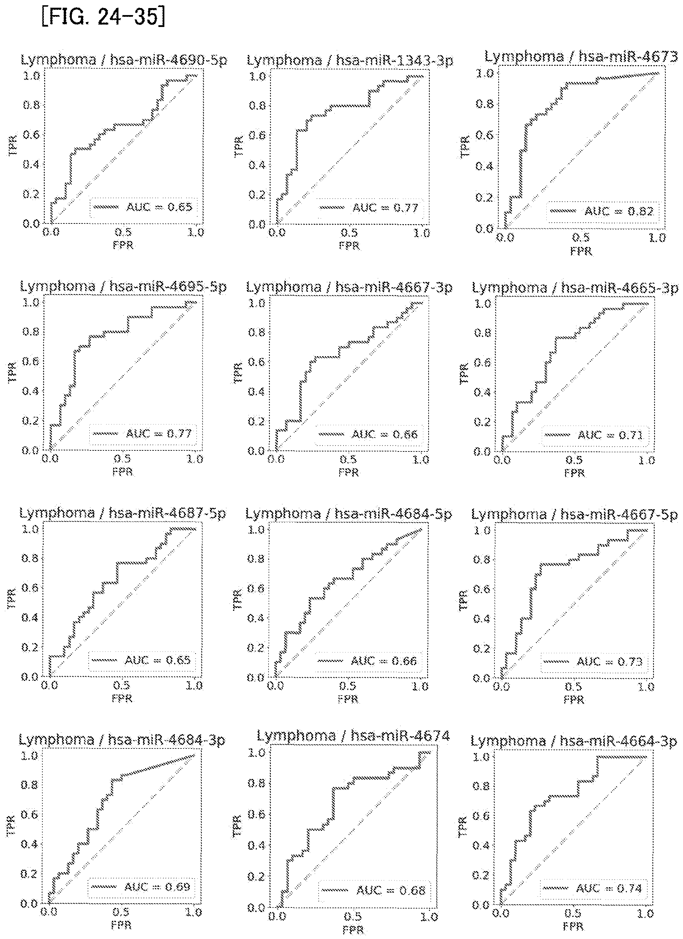

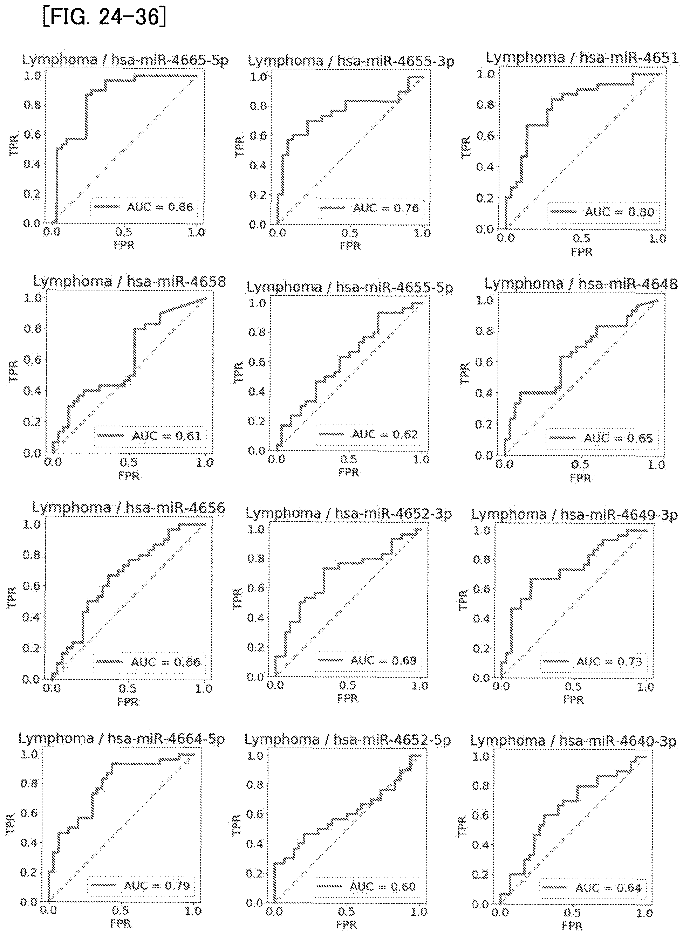

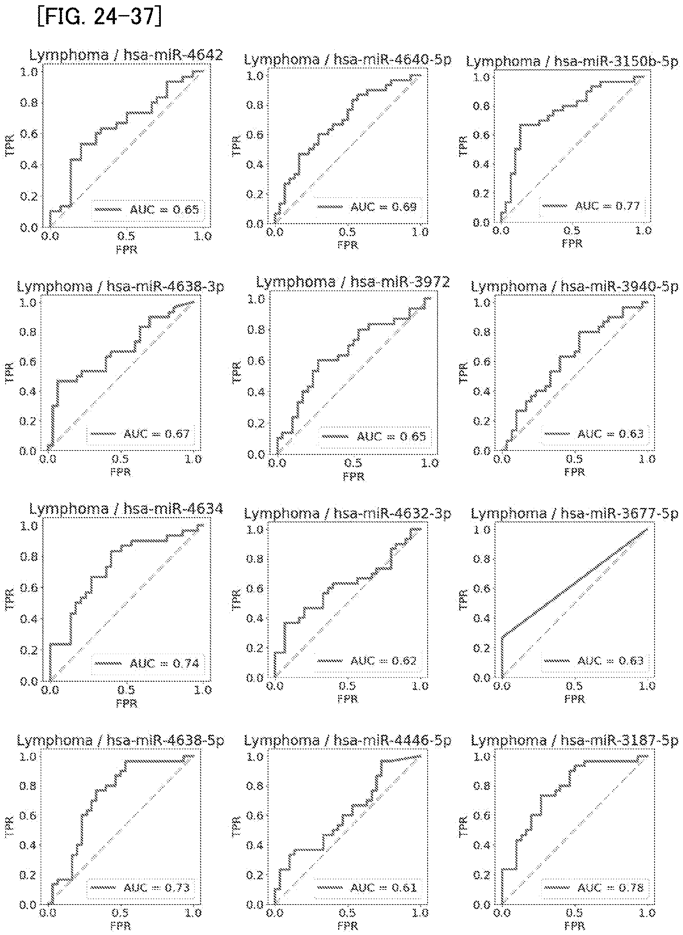

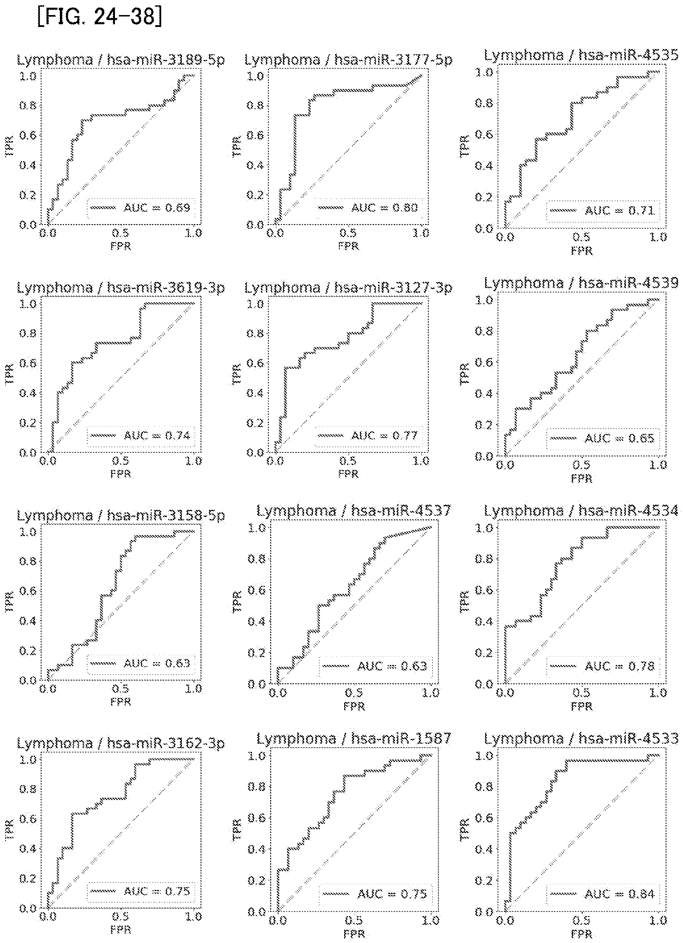

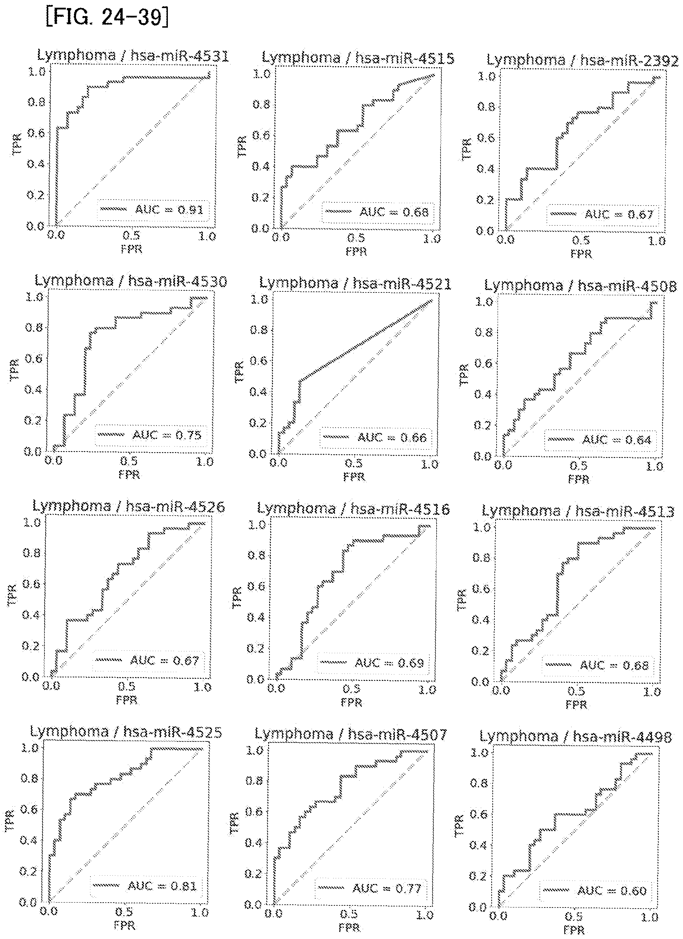

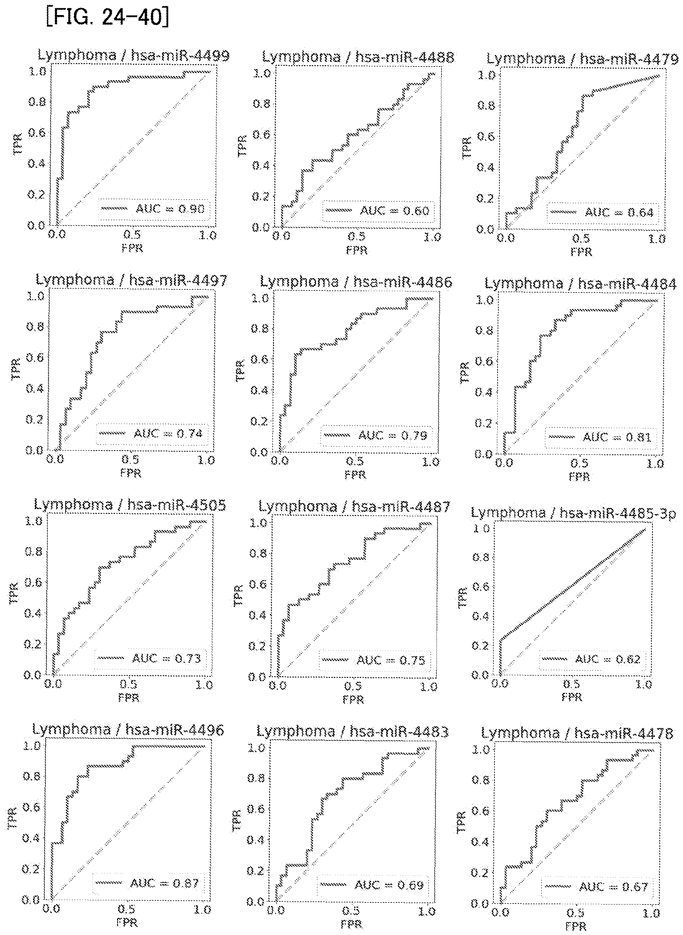

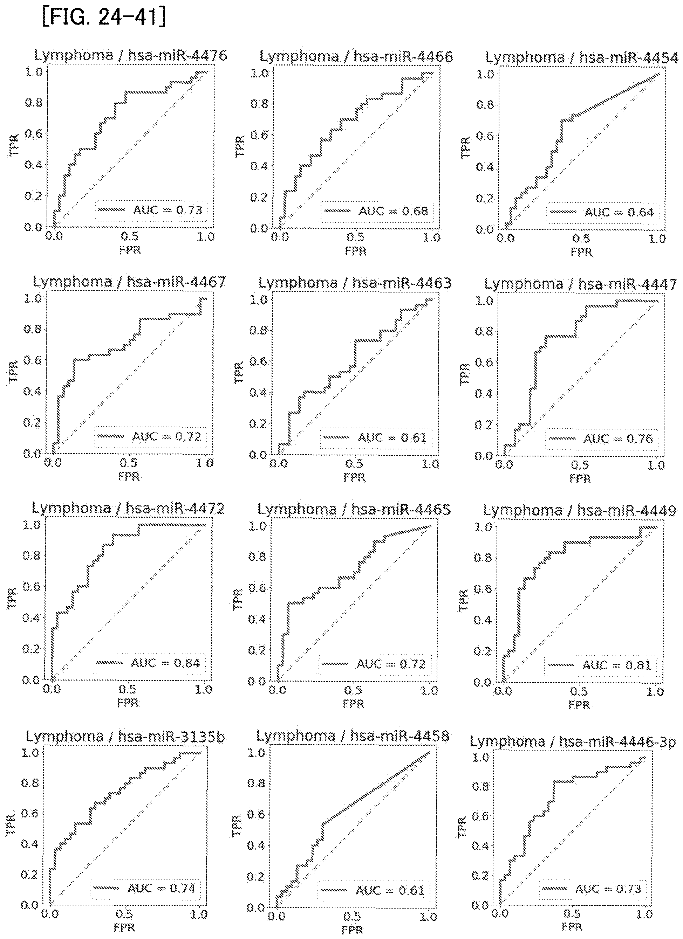

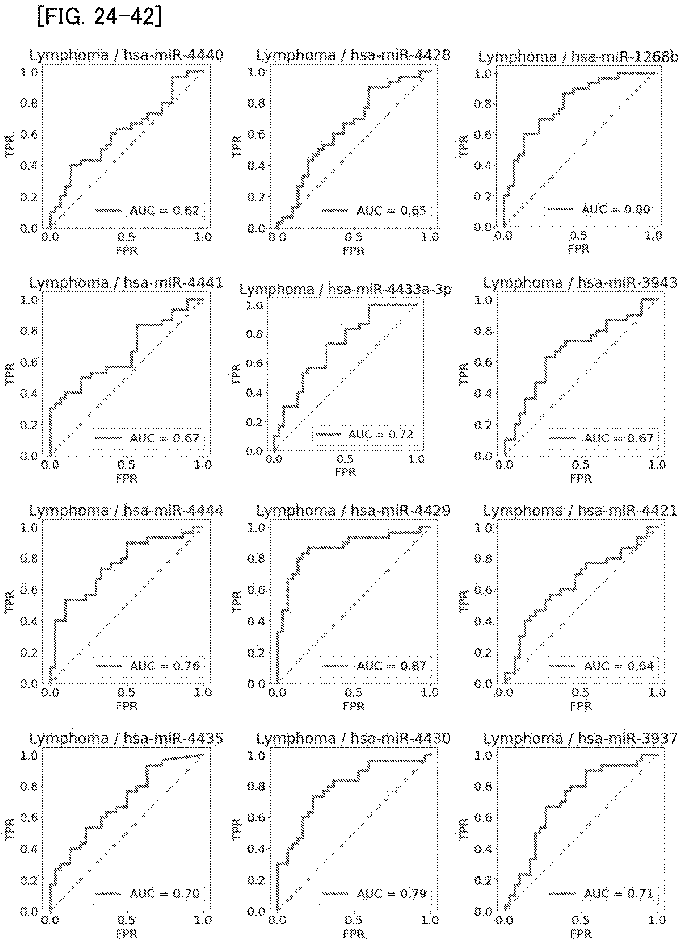

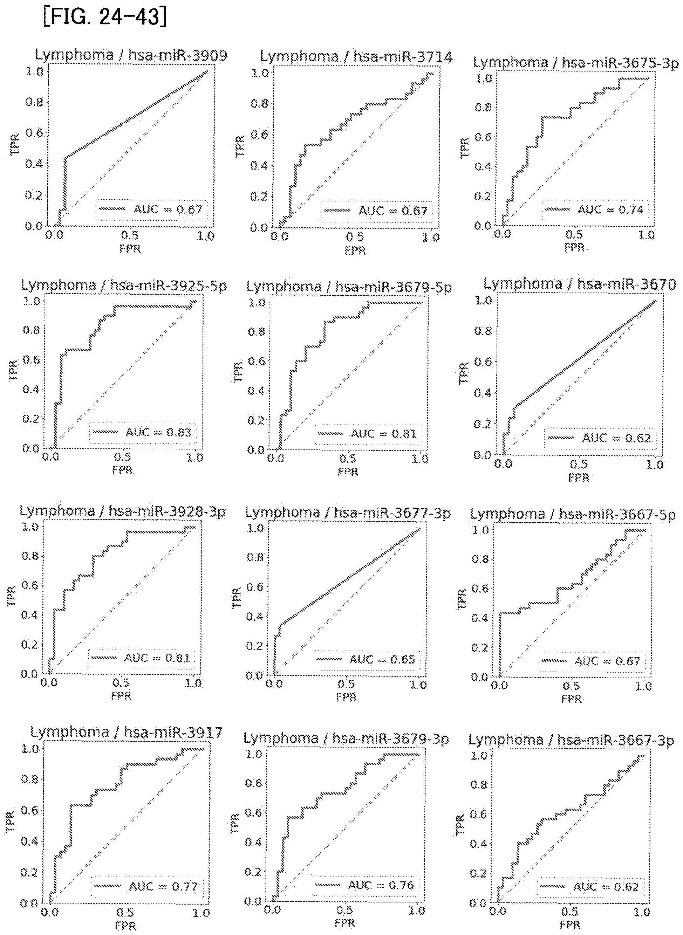

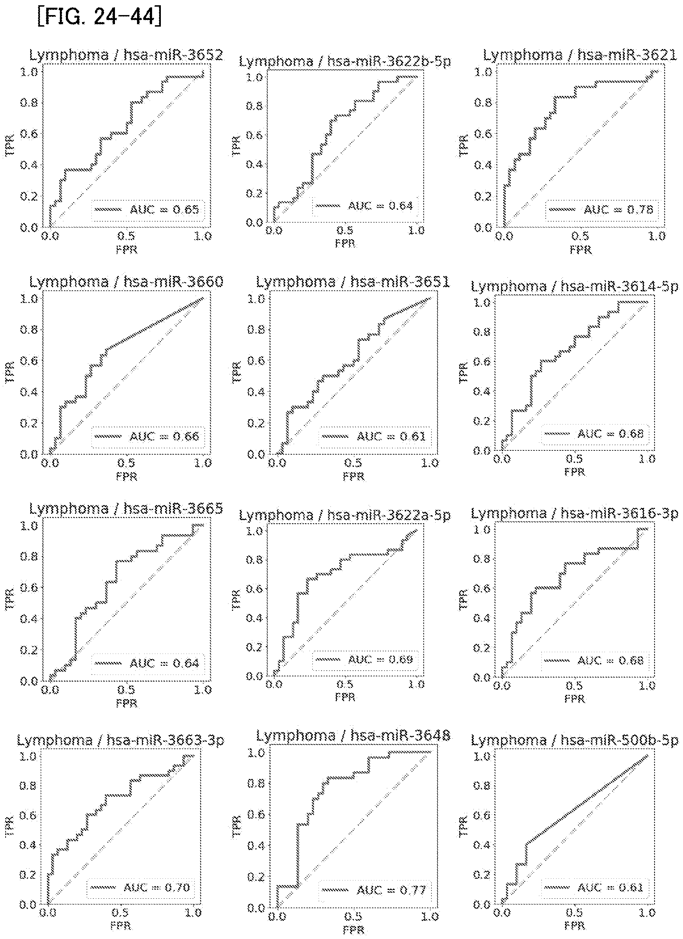

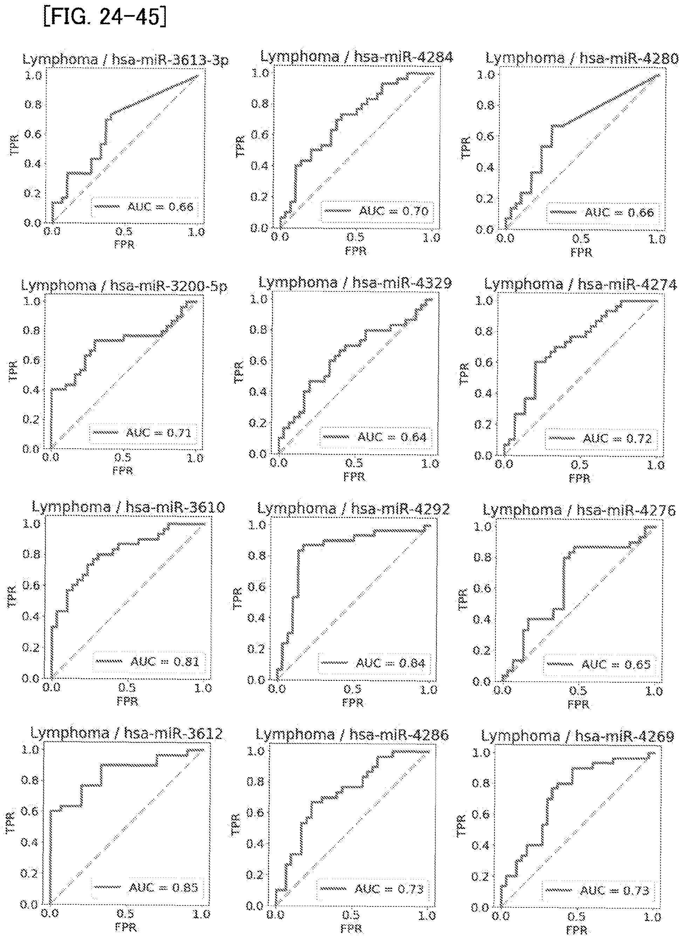

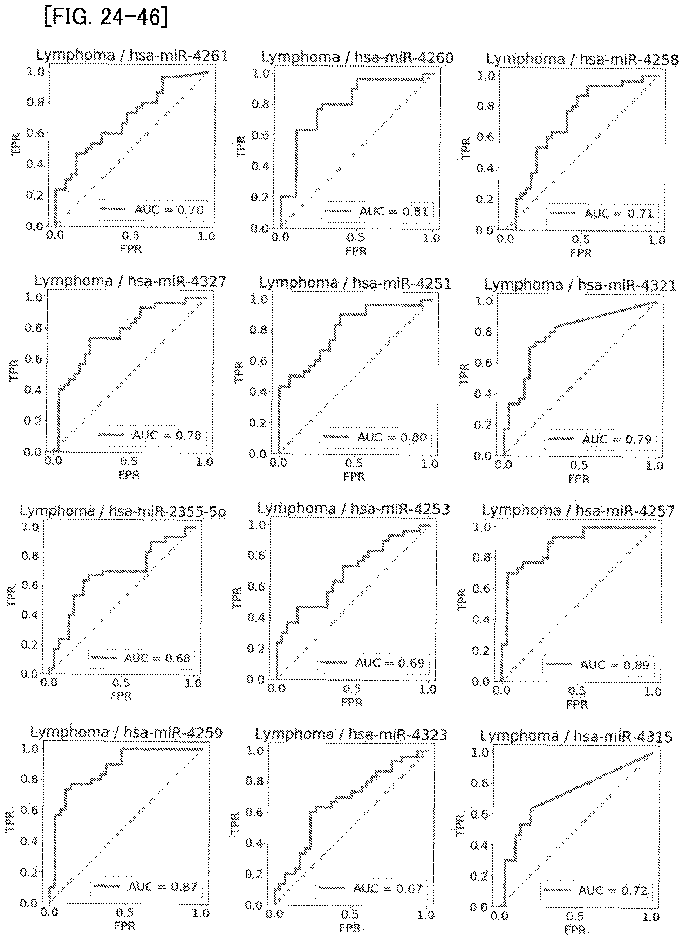

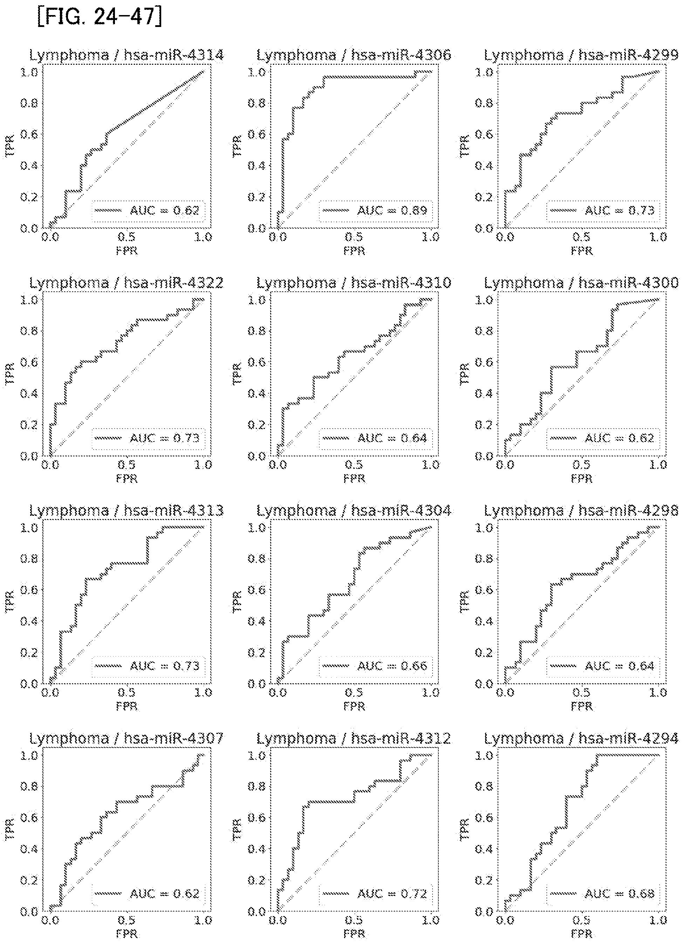

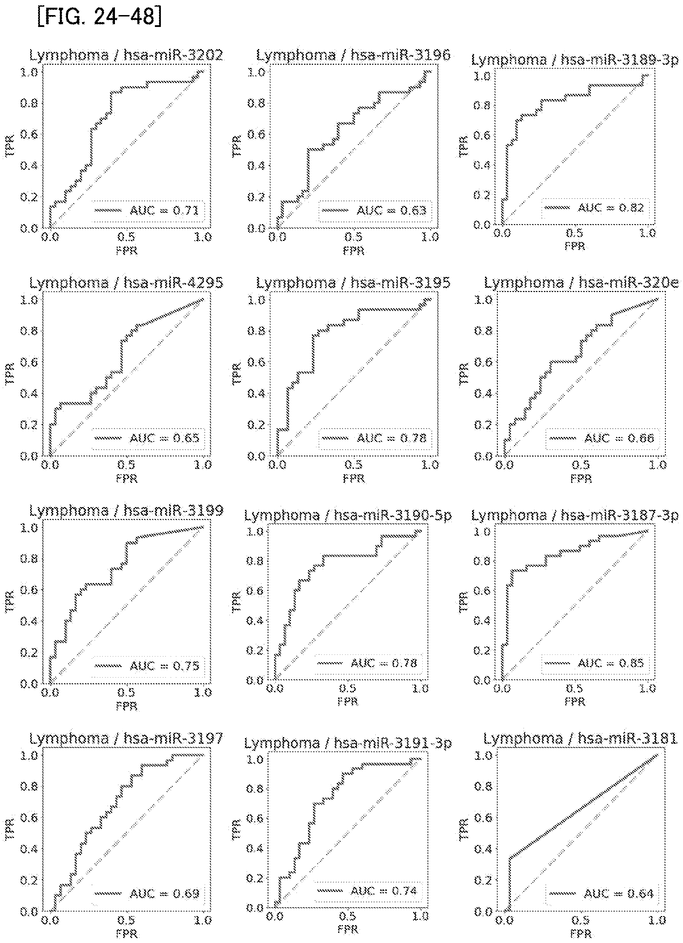

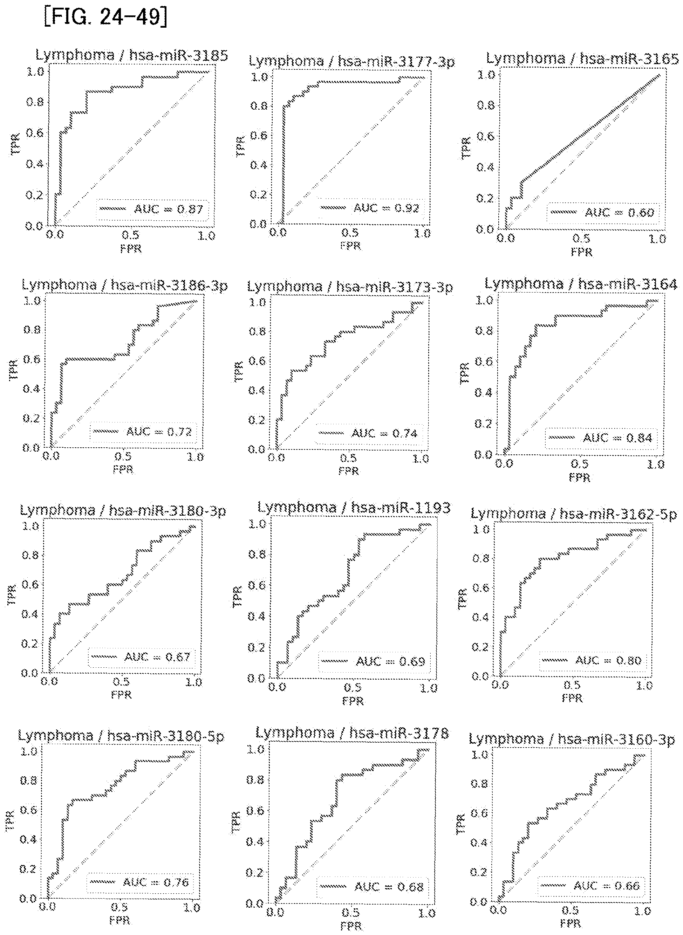

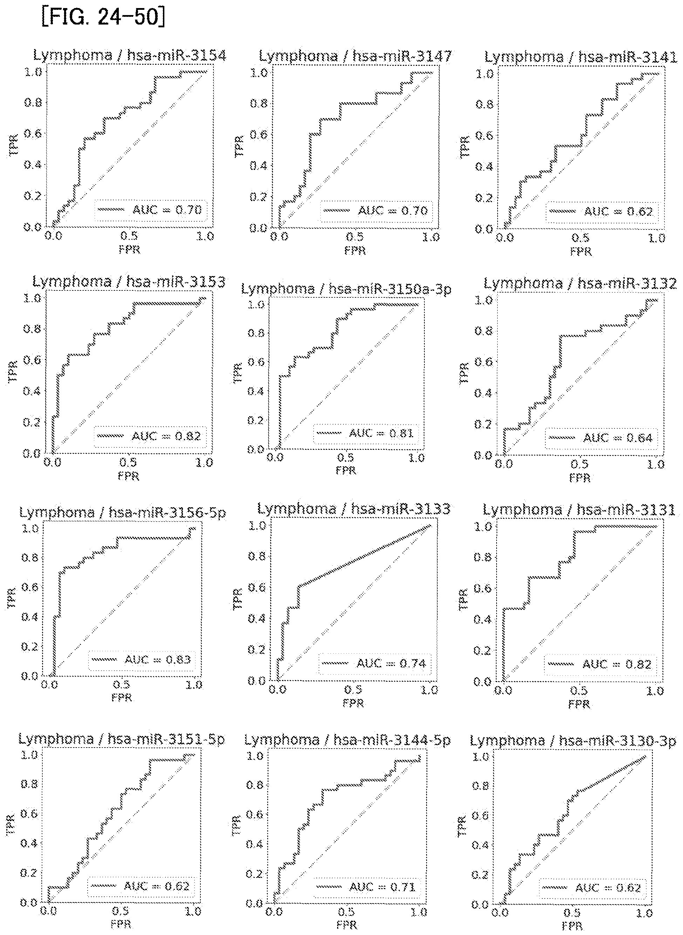

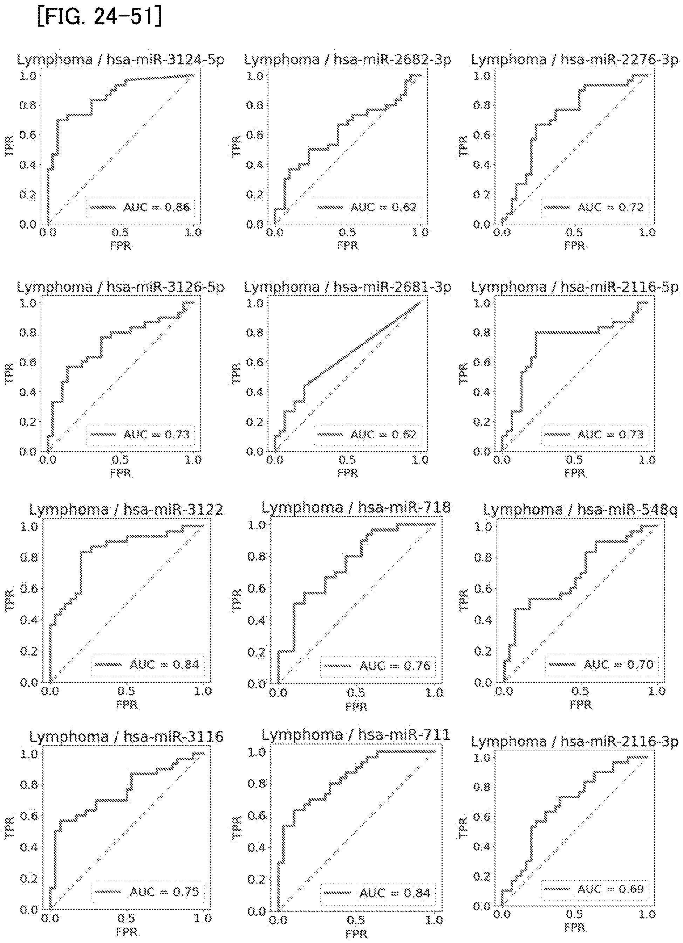

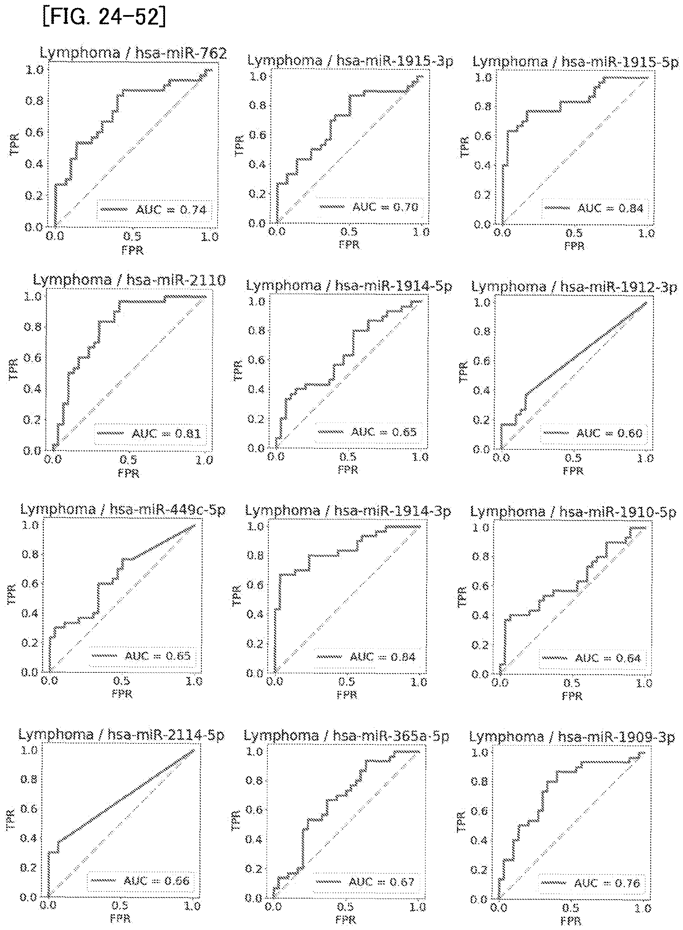

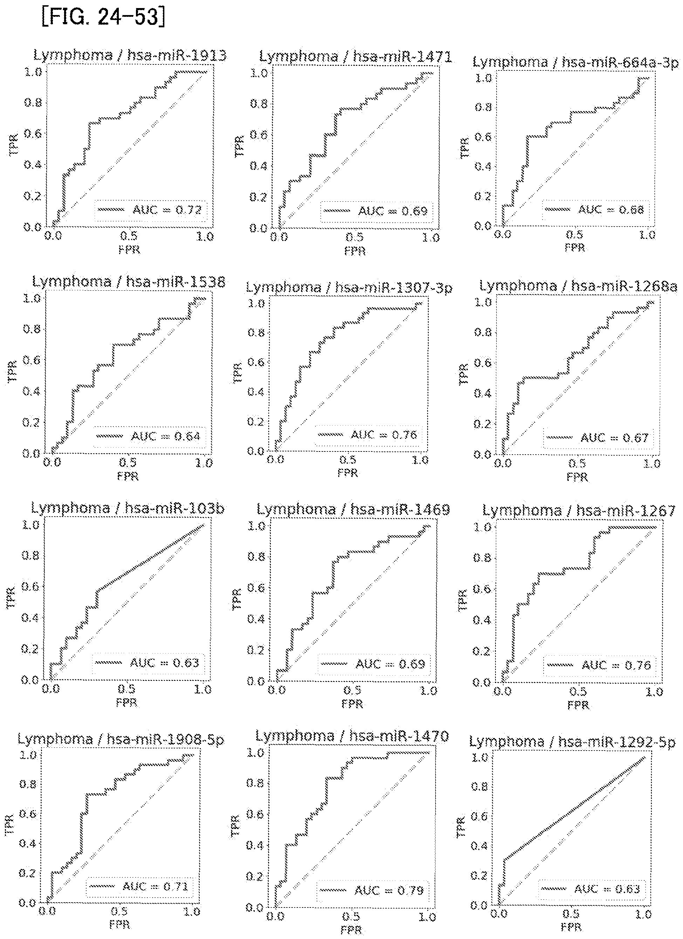

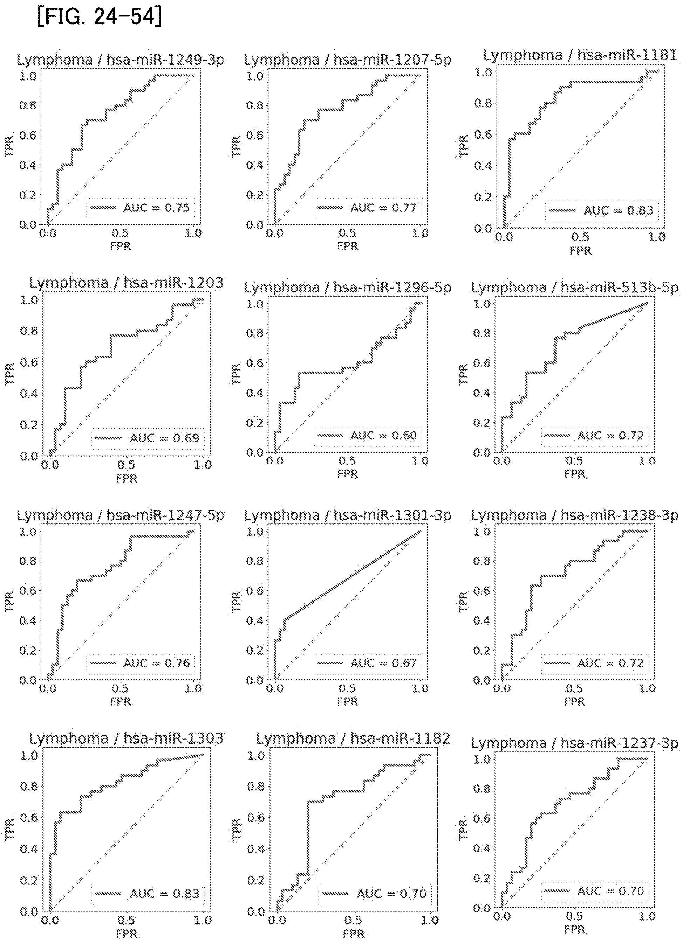

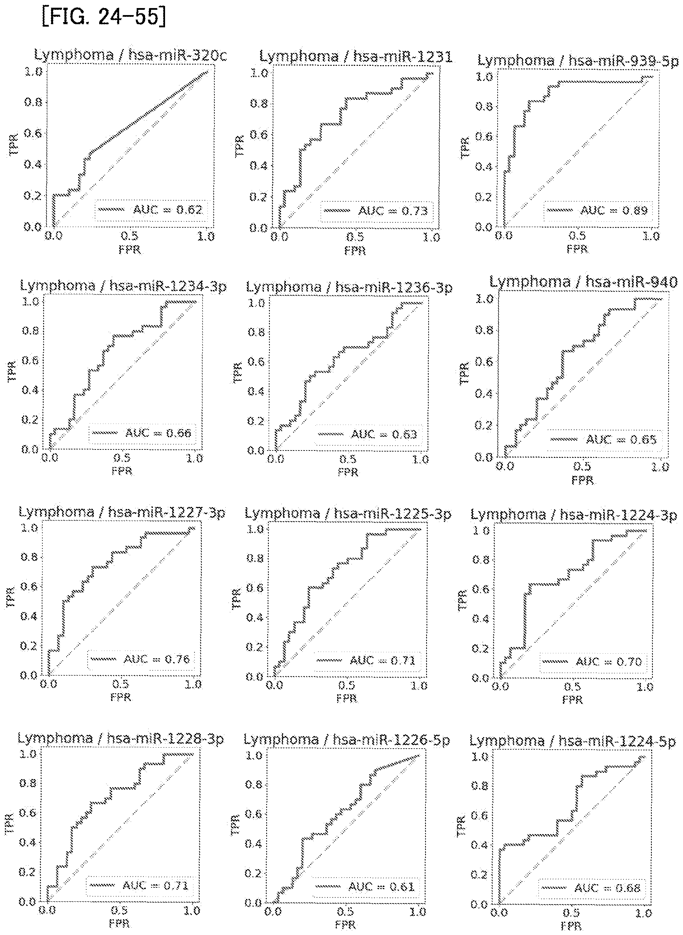

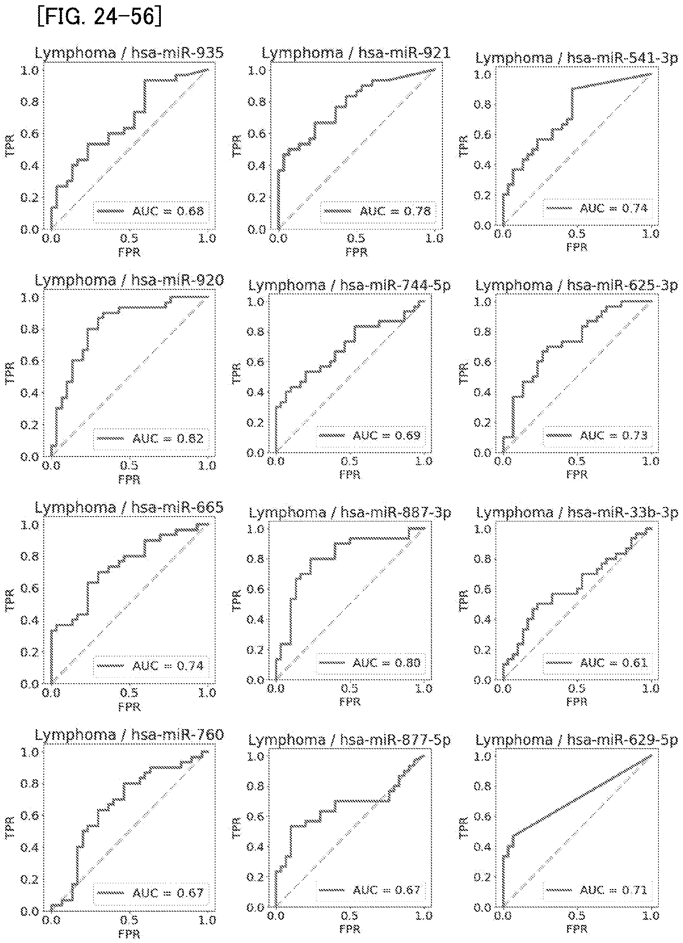

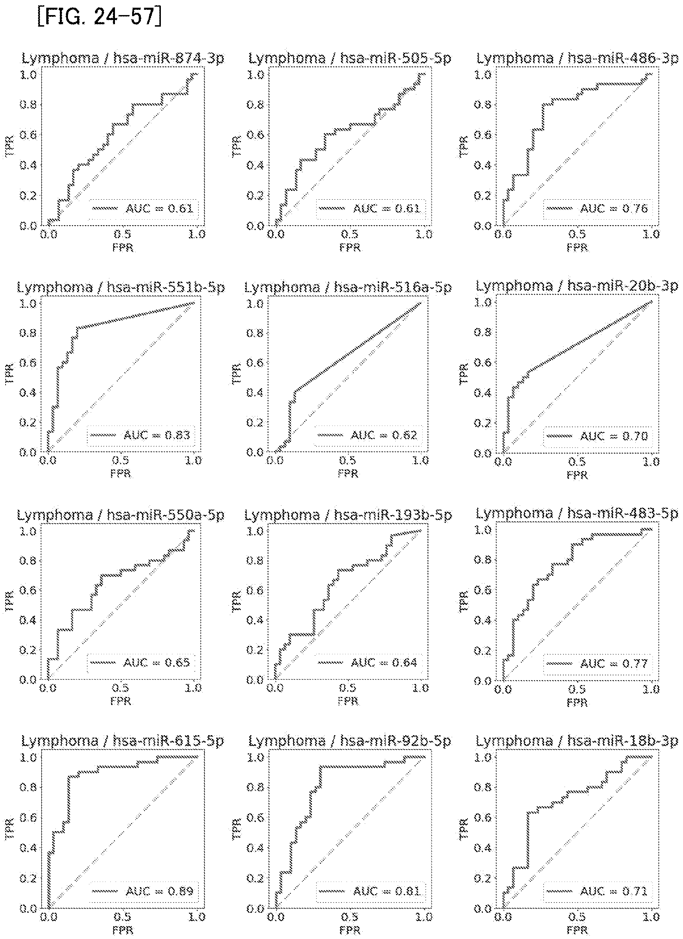

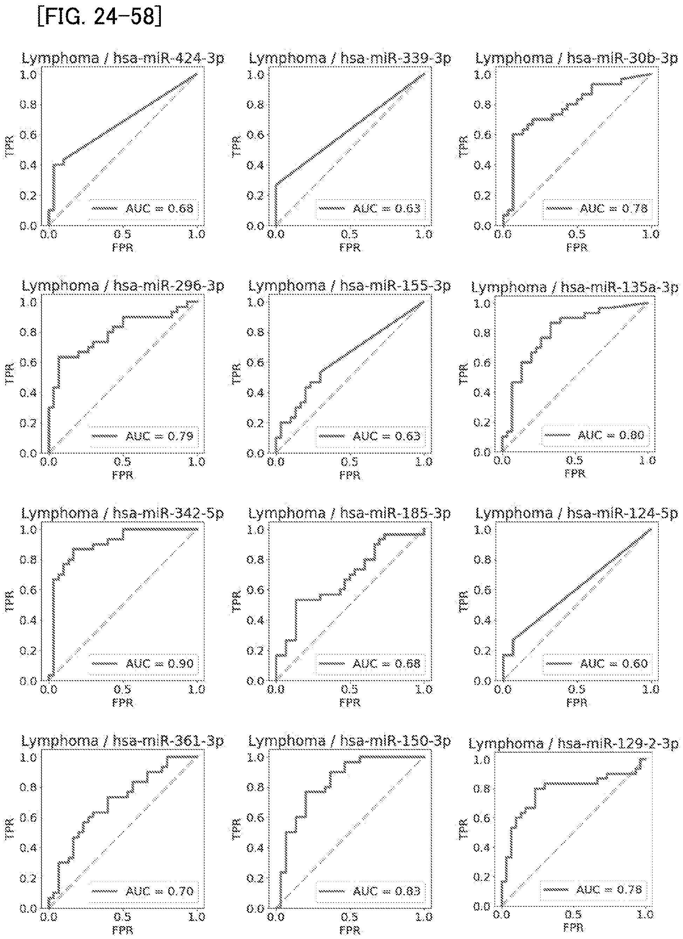

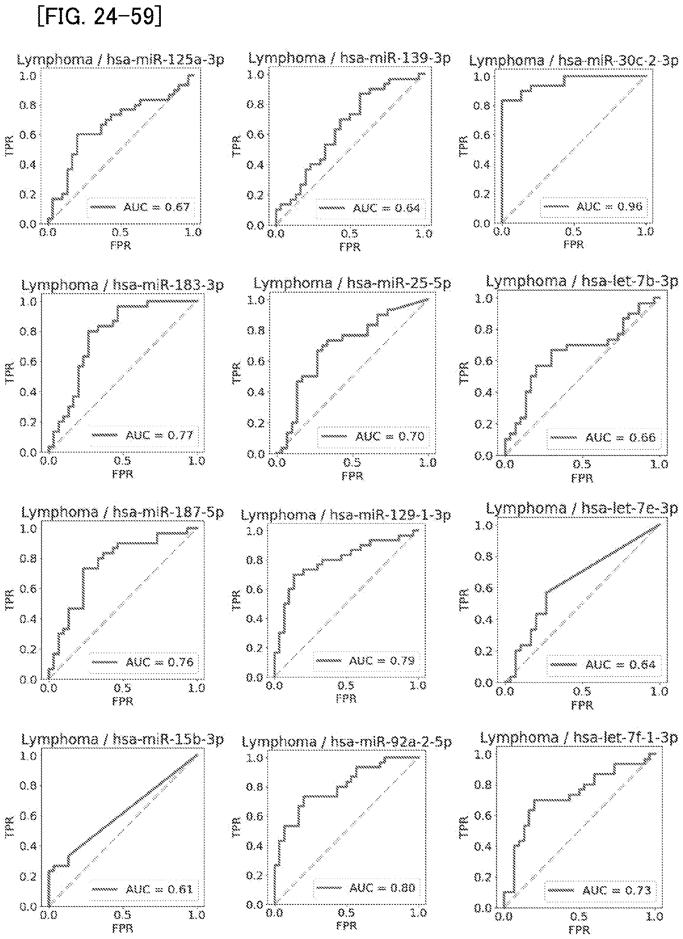

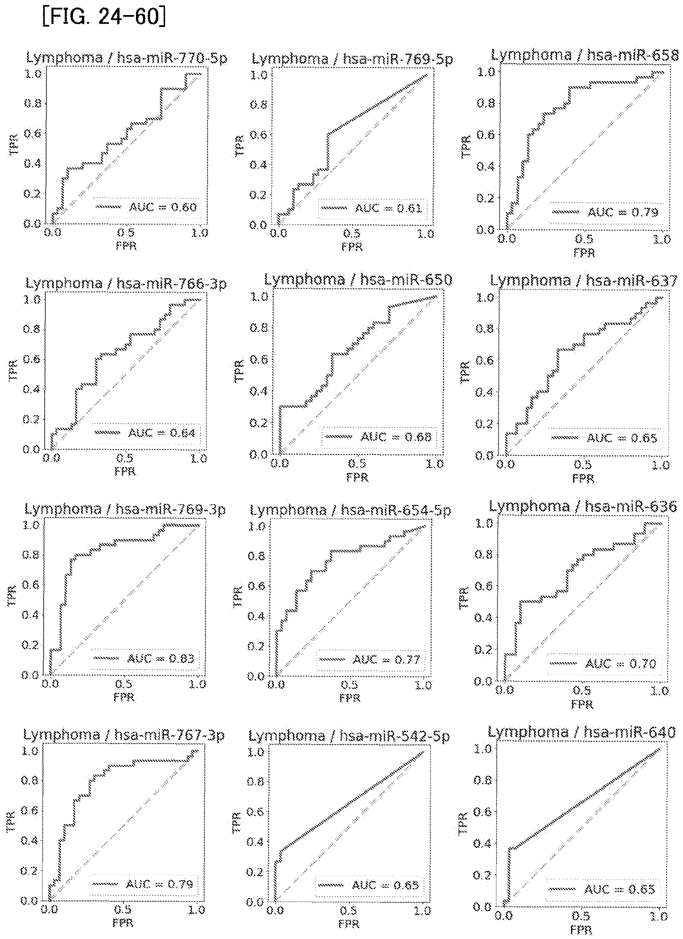

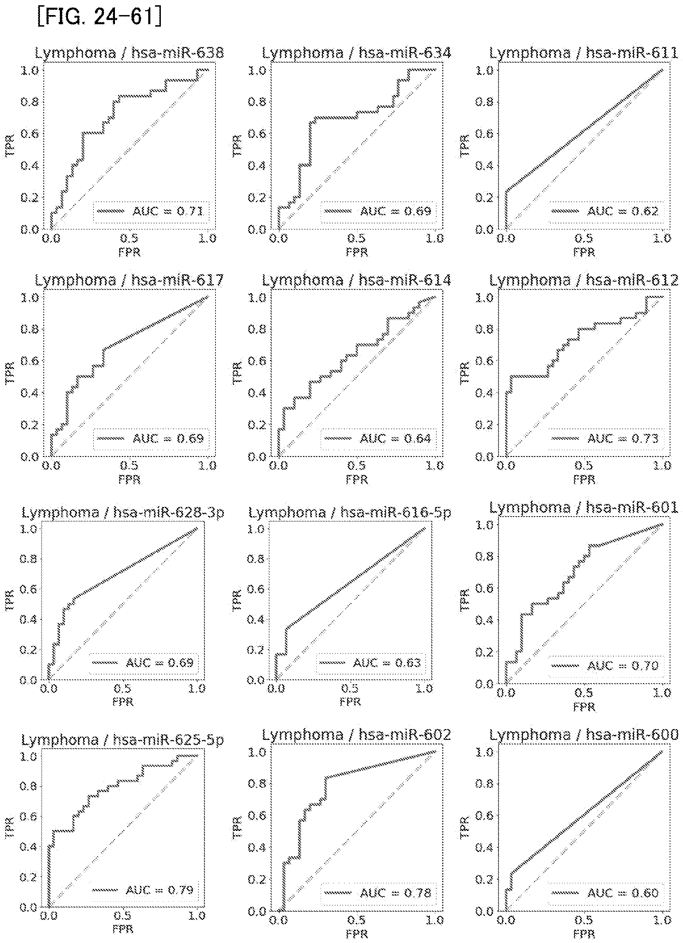

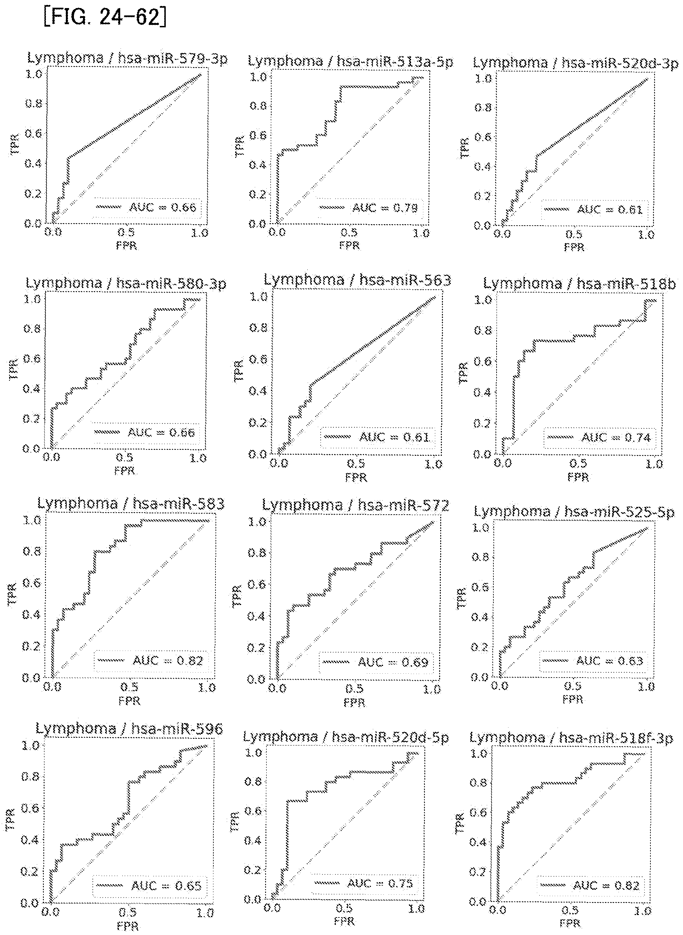

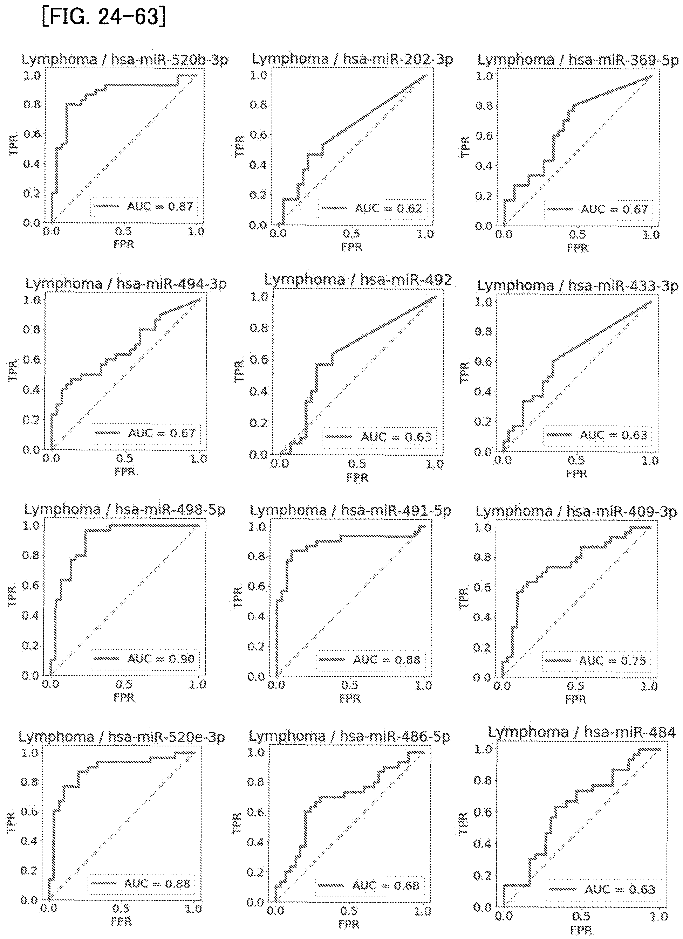

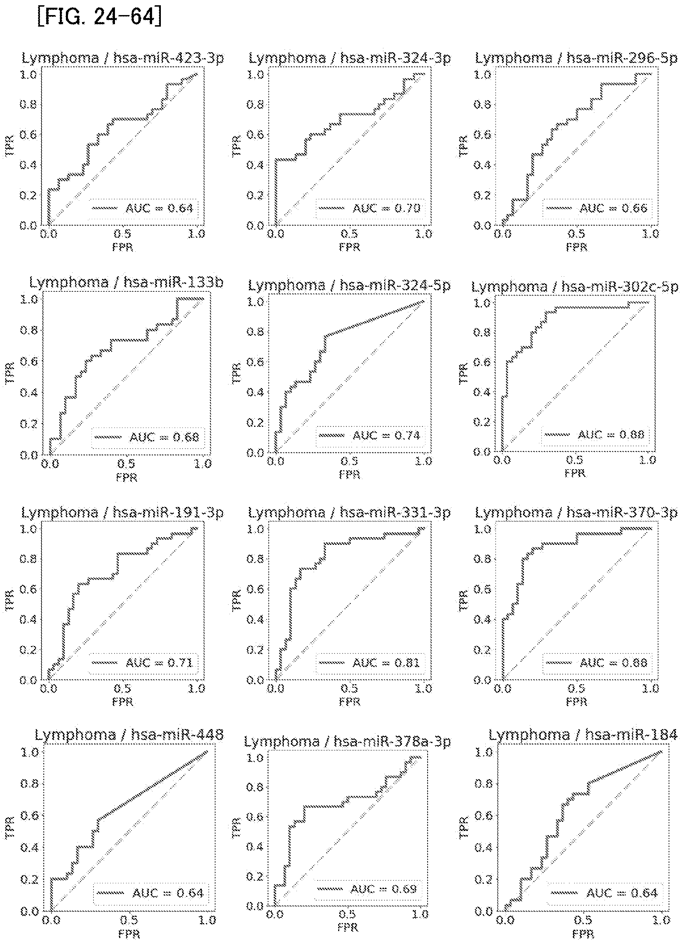

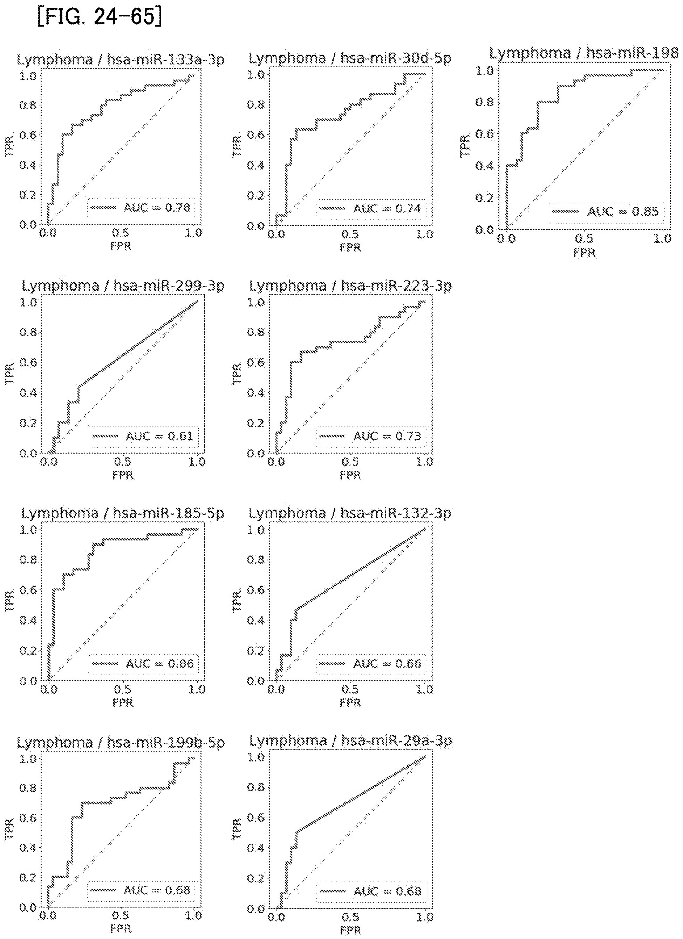

FIGS. 24-1 to 24-65 show ROC-curves for predicting lymphomas in humans using a single miRNA.

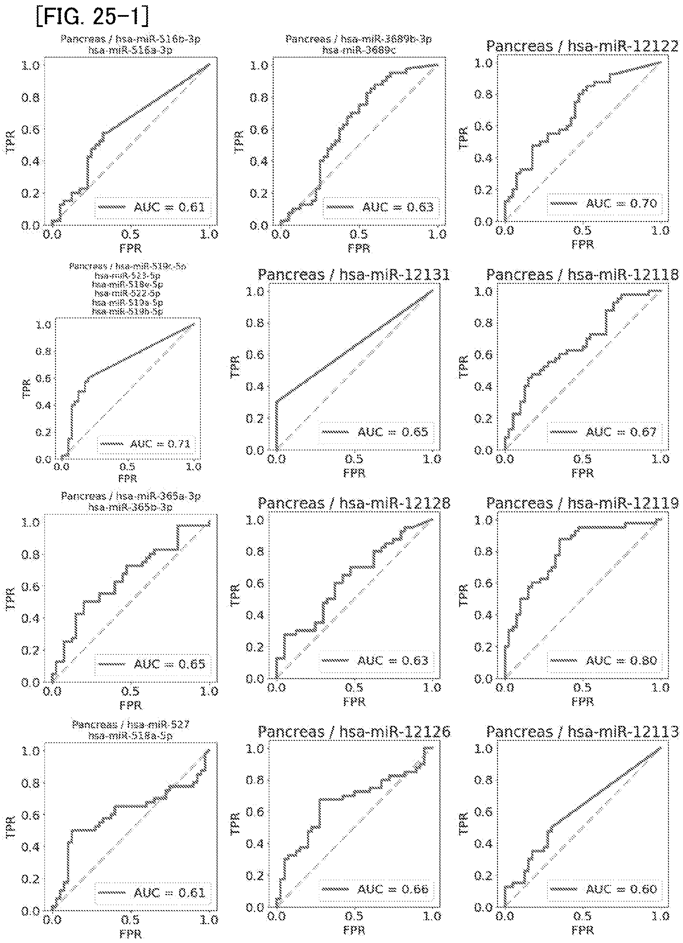

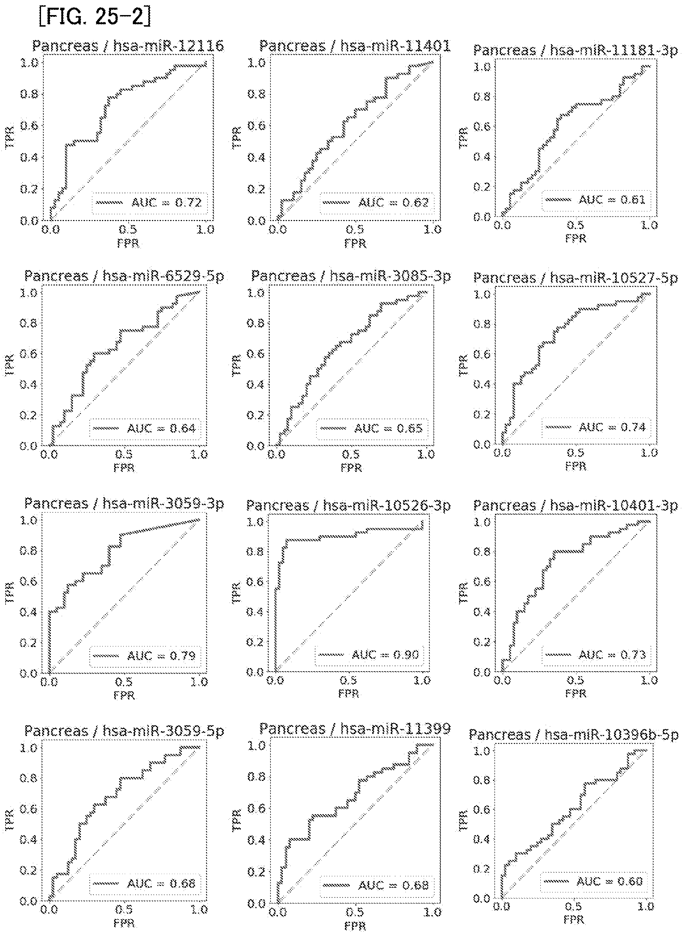

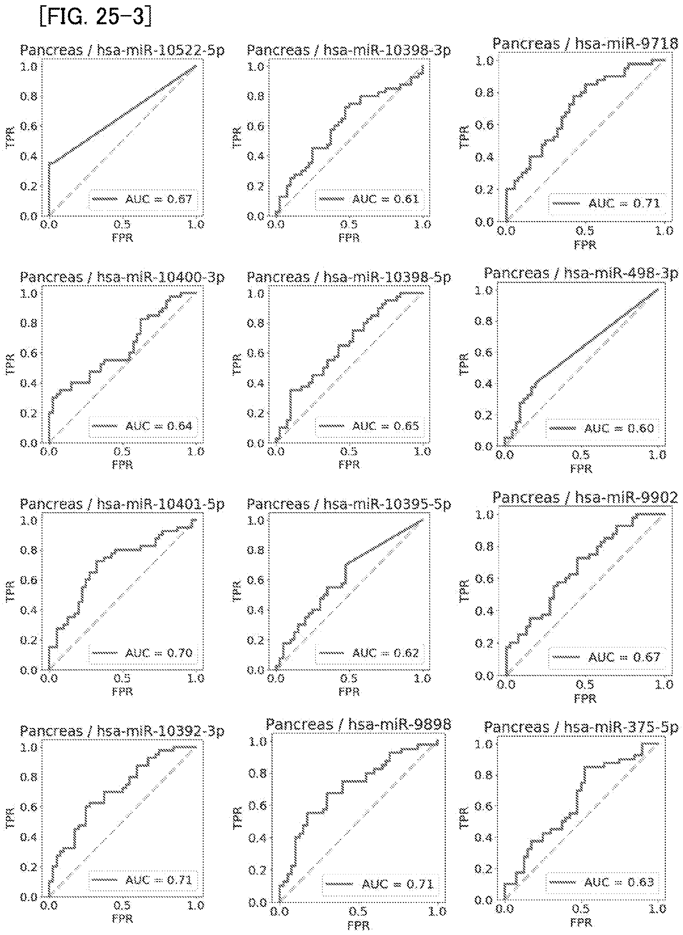

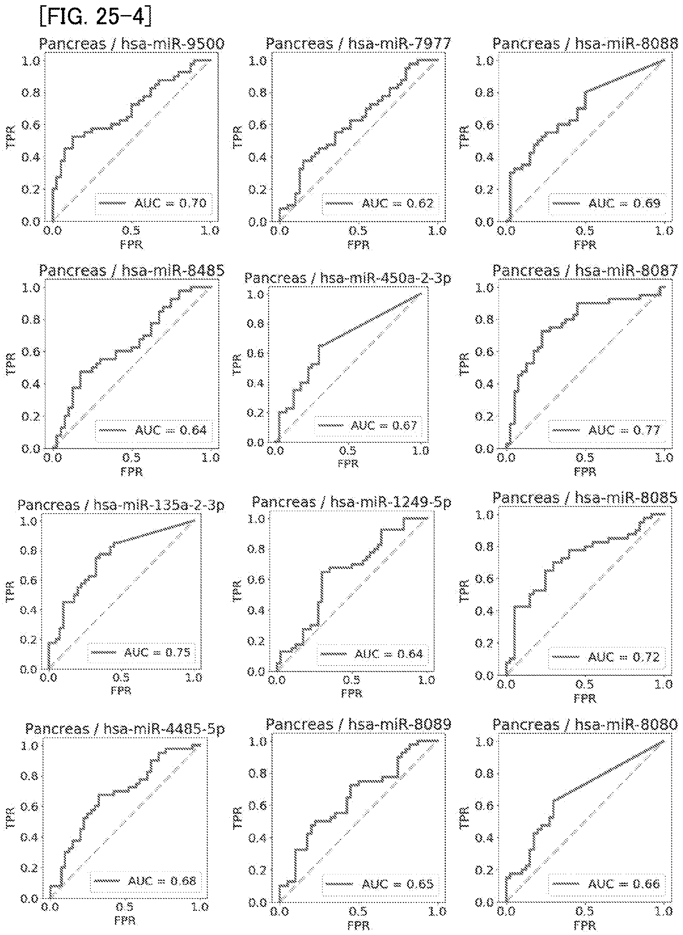

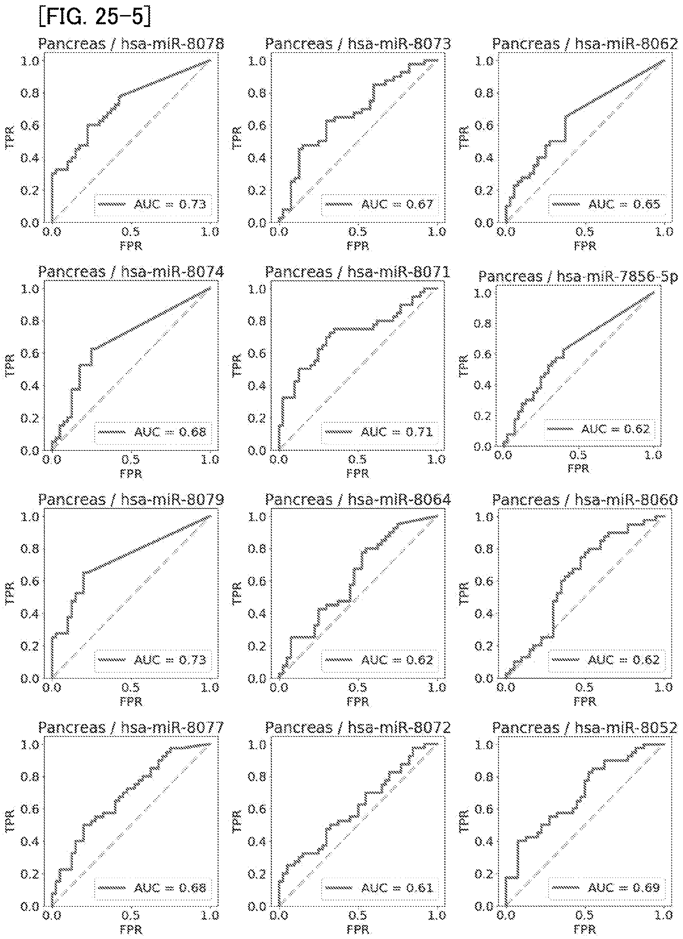

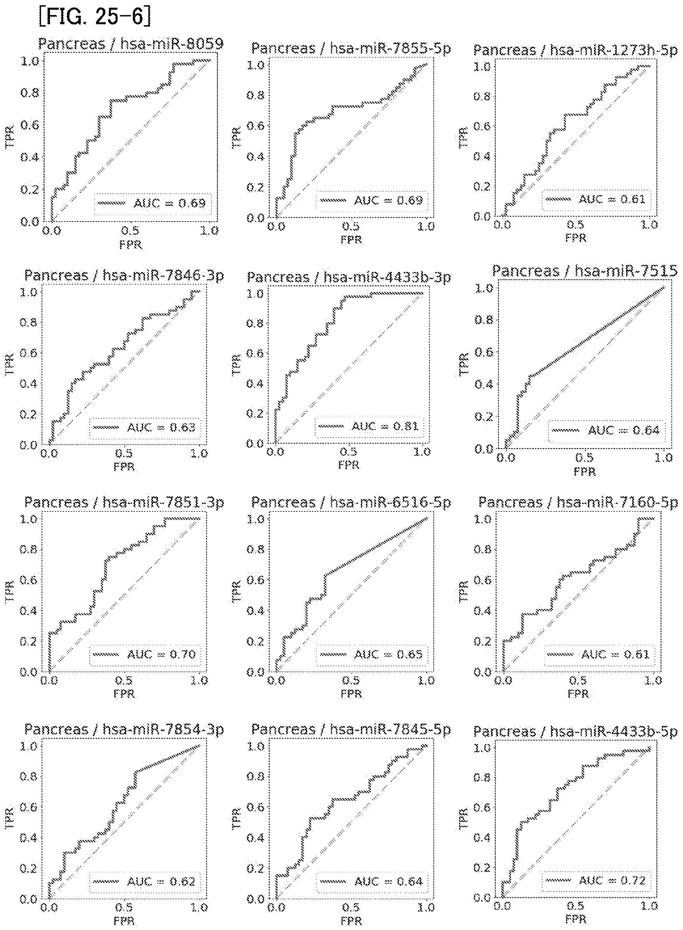

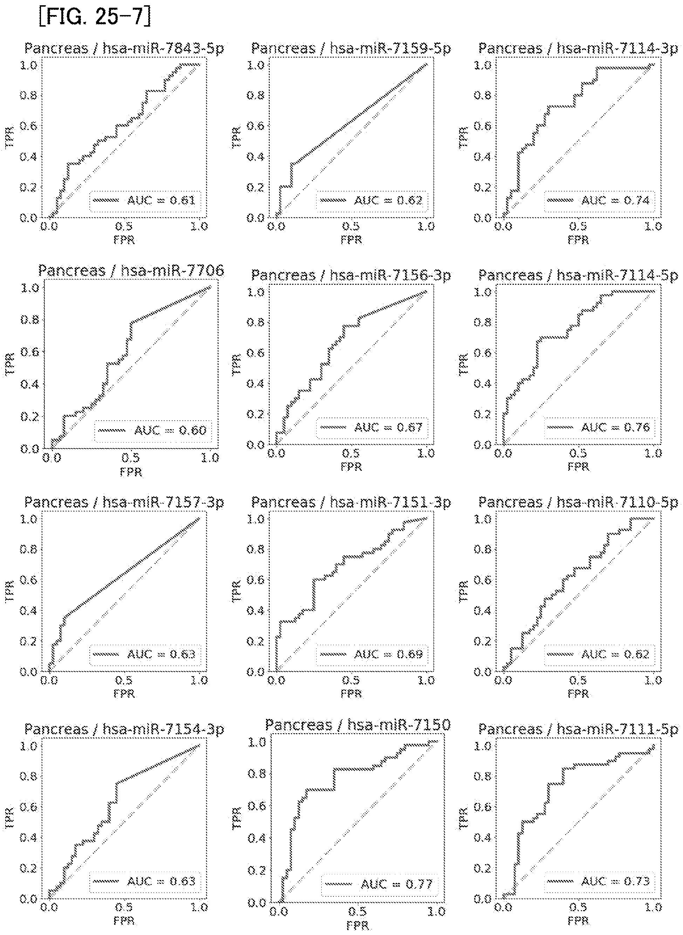

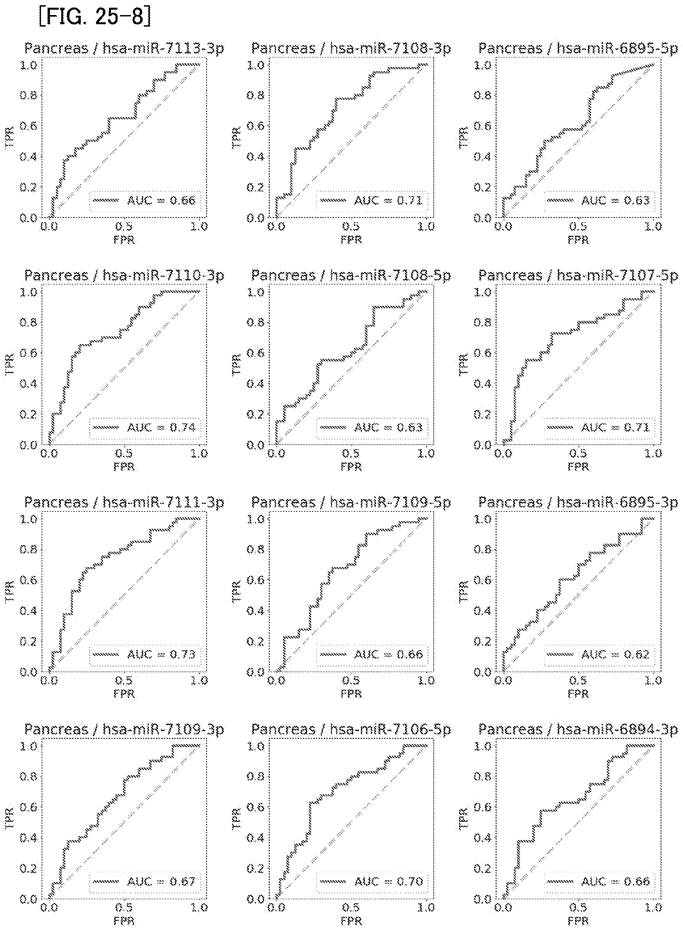

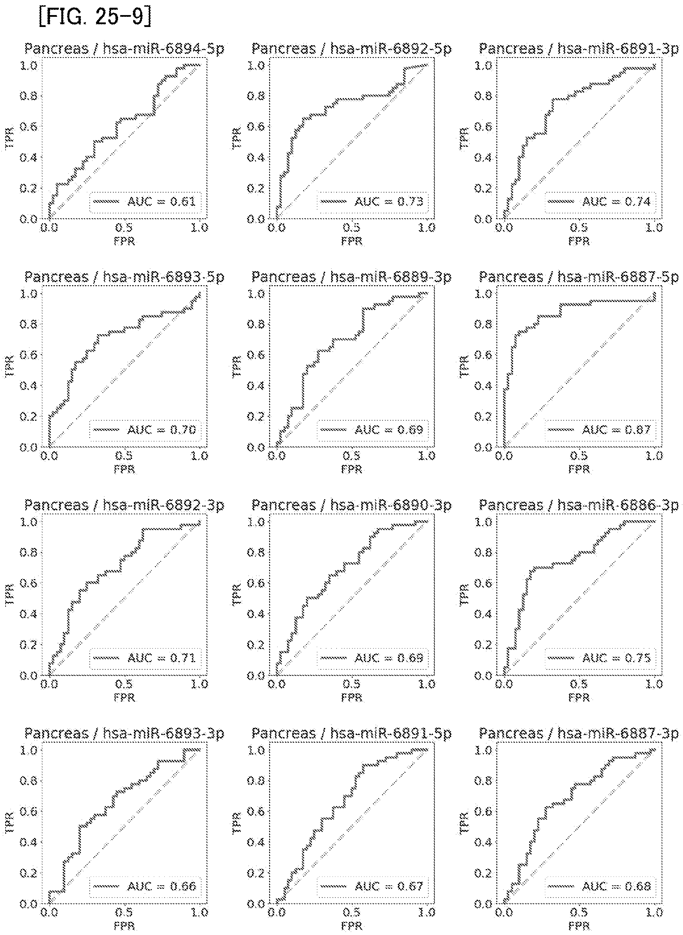

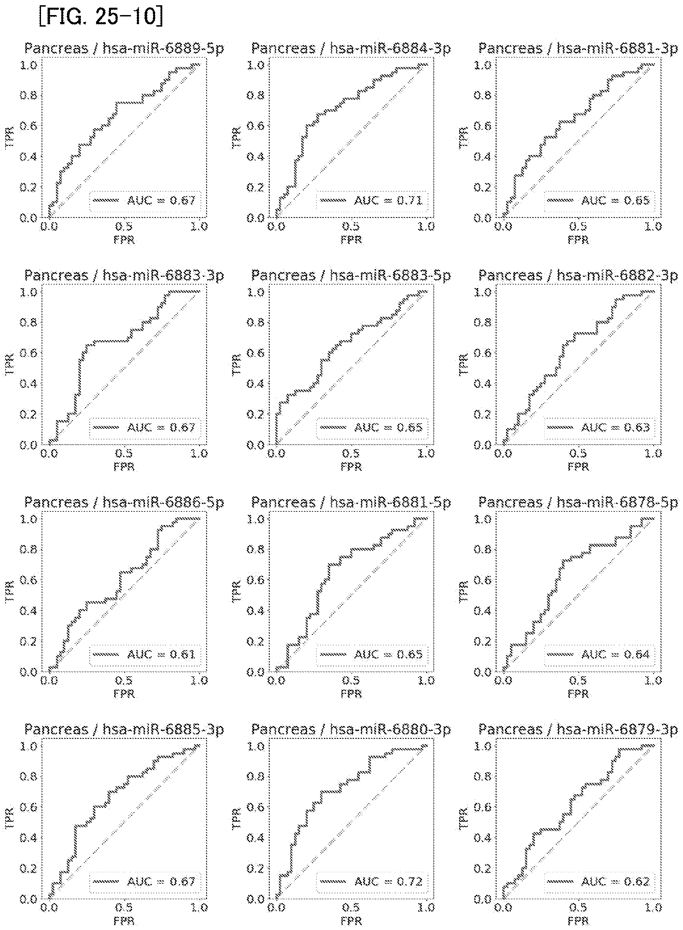

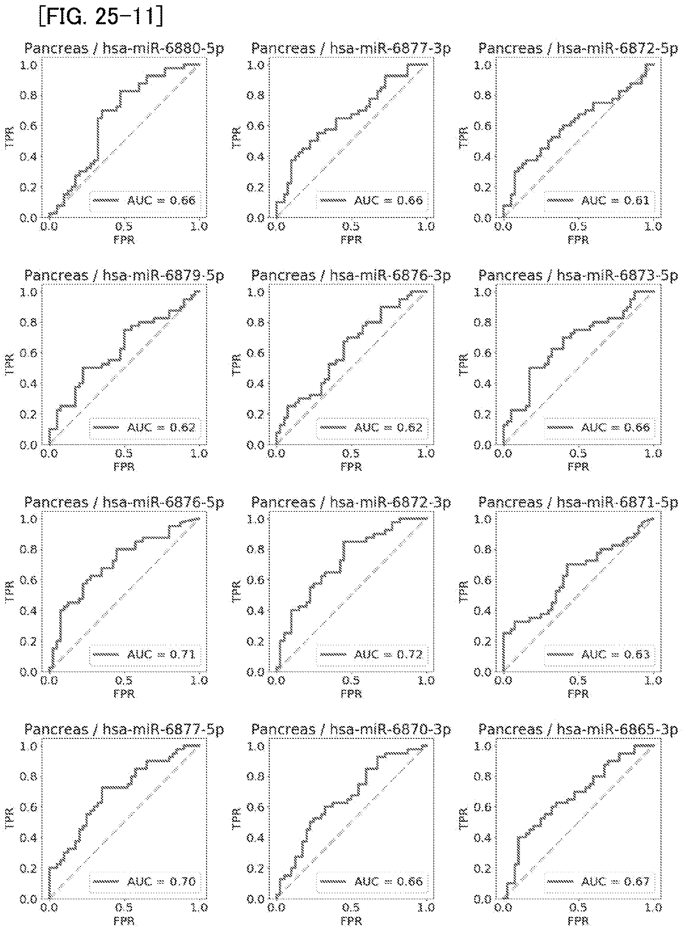

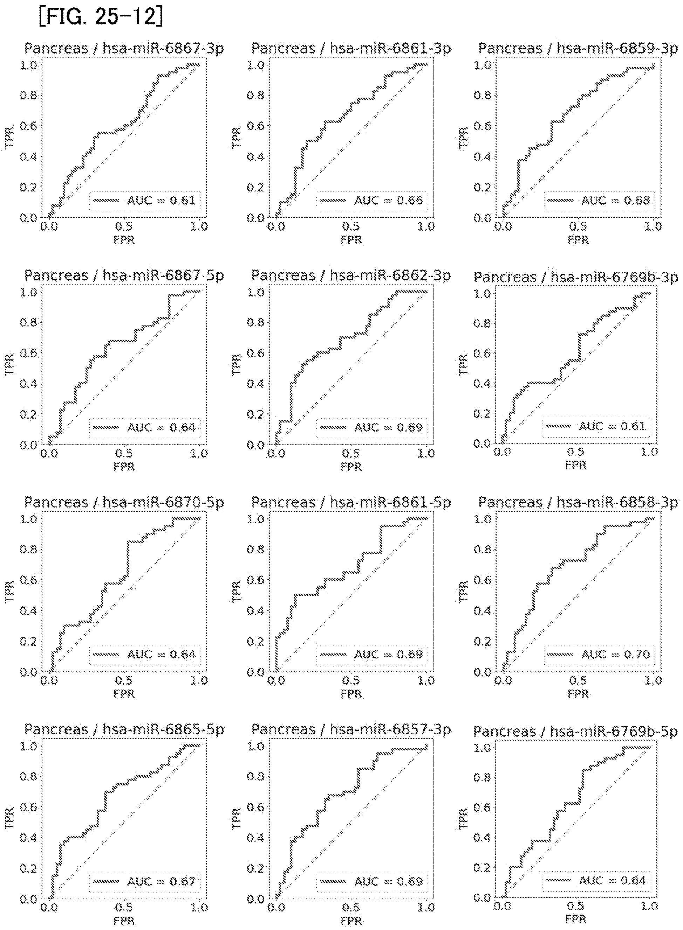

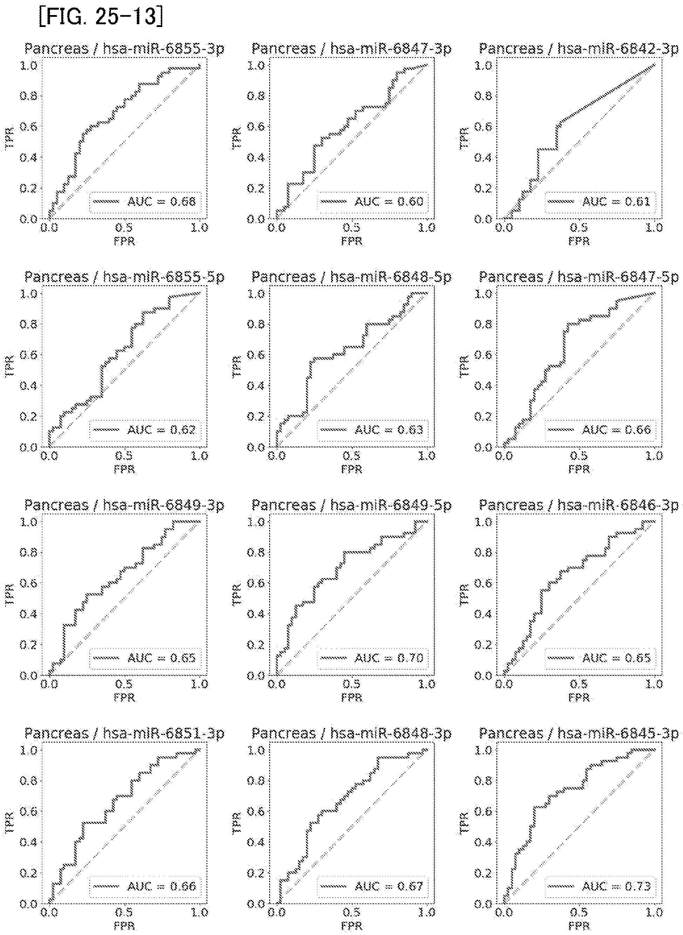

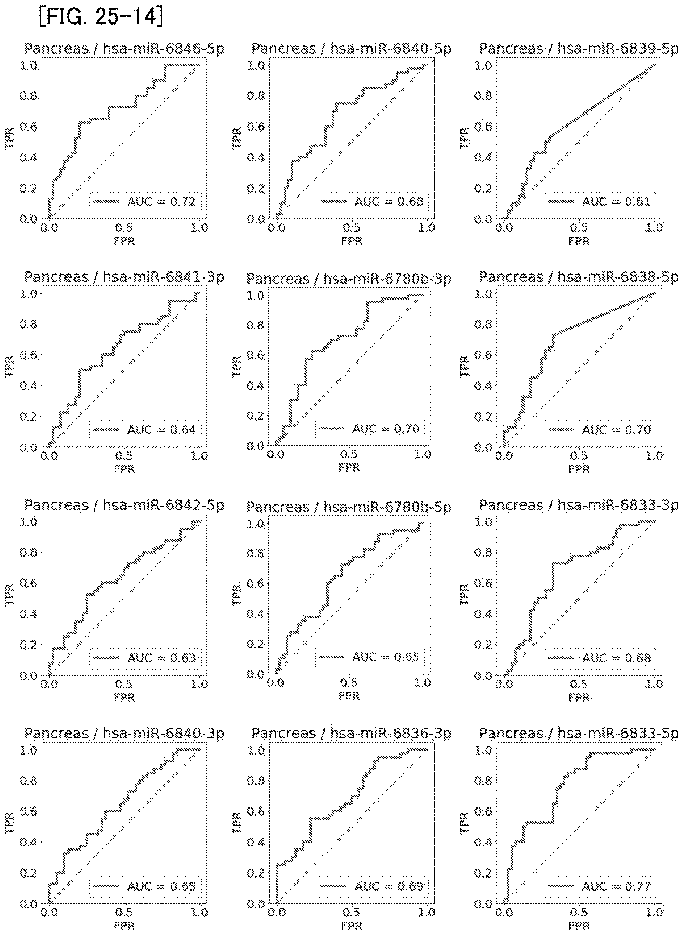

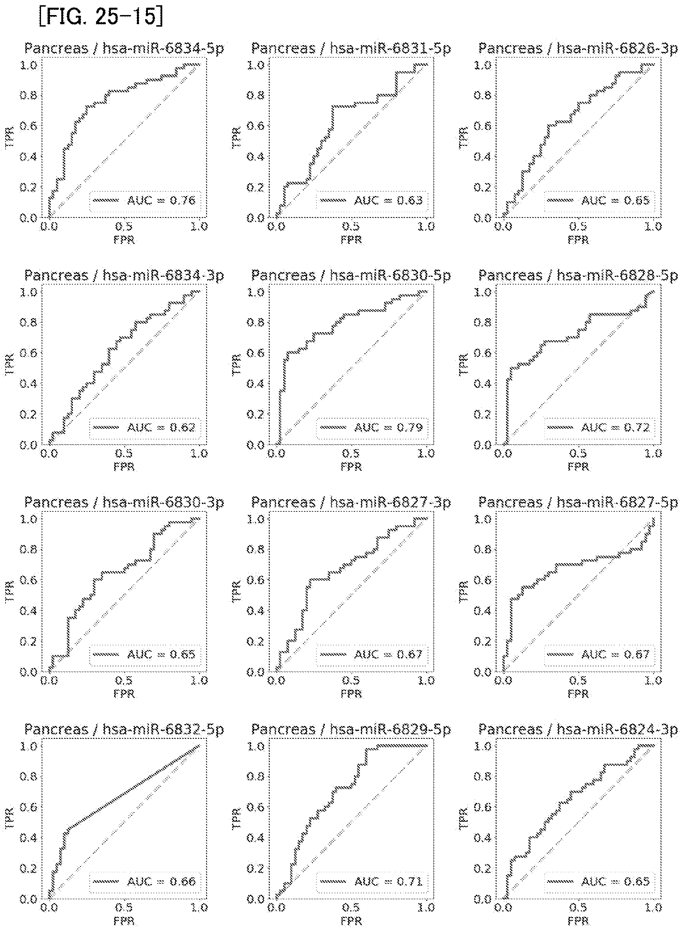

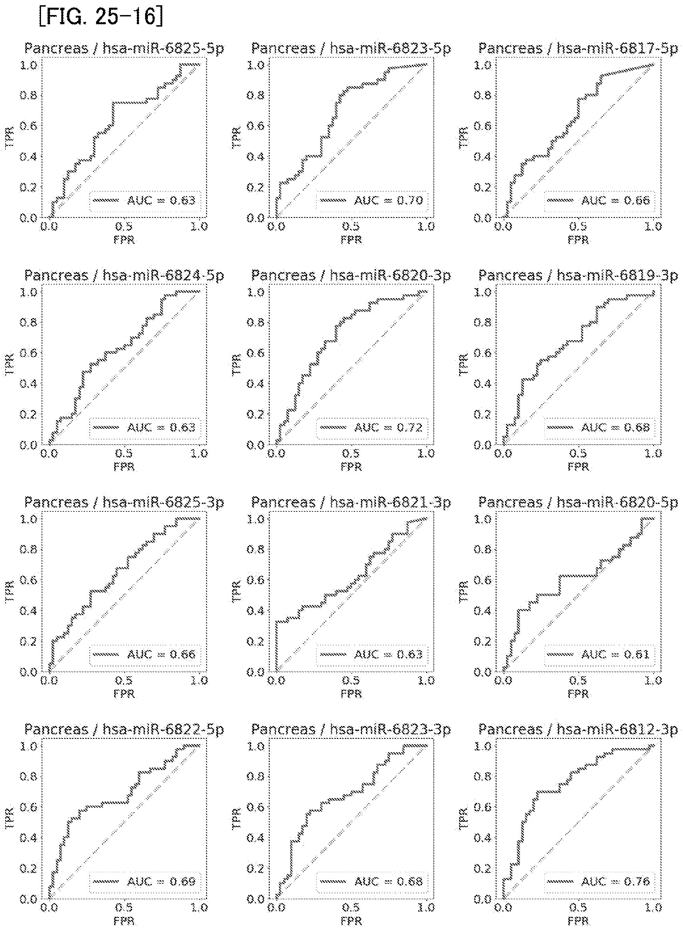

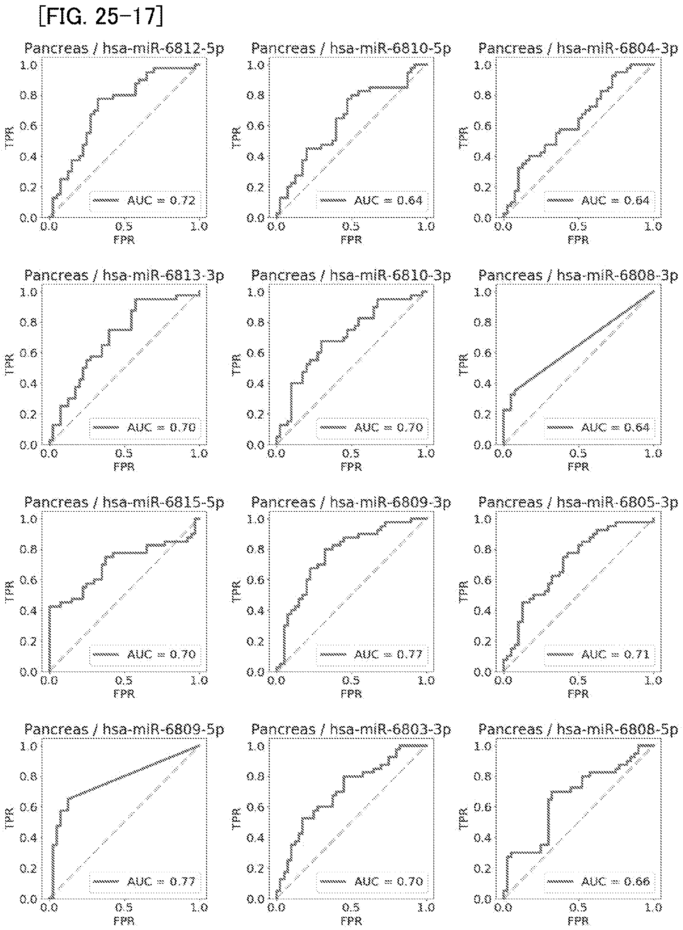

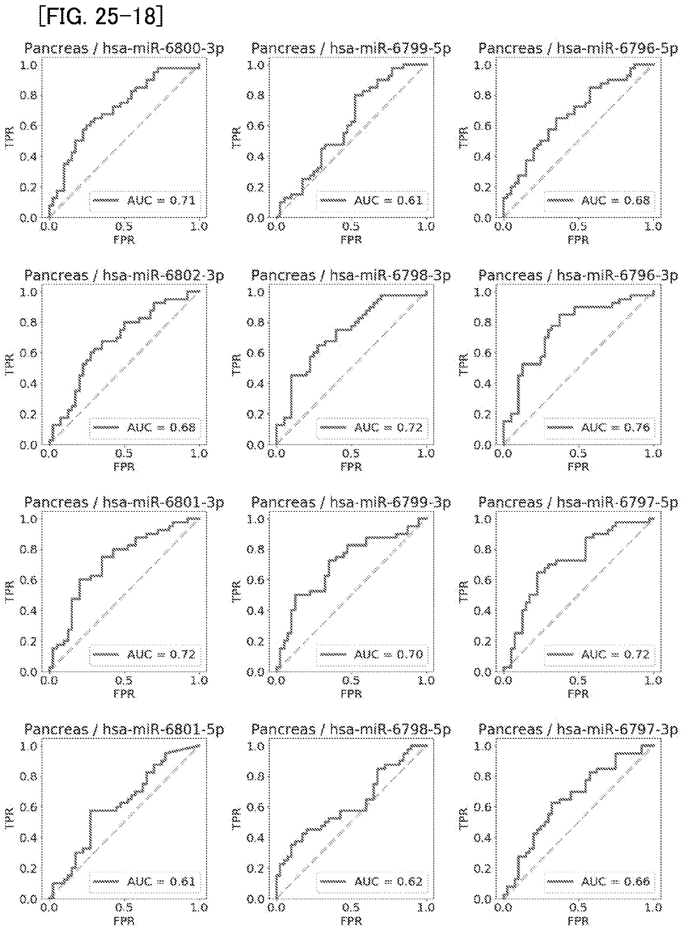

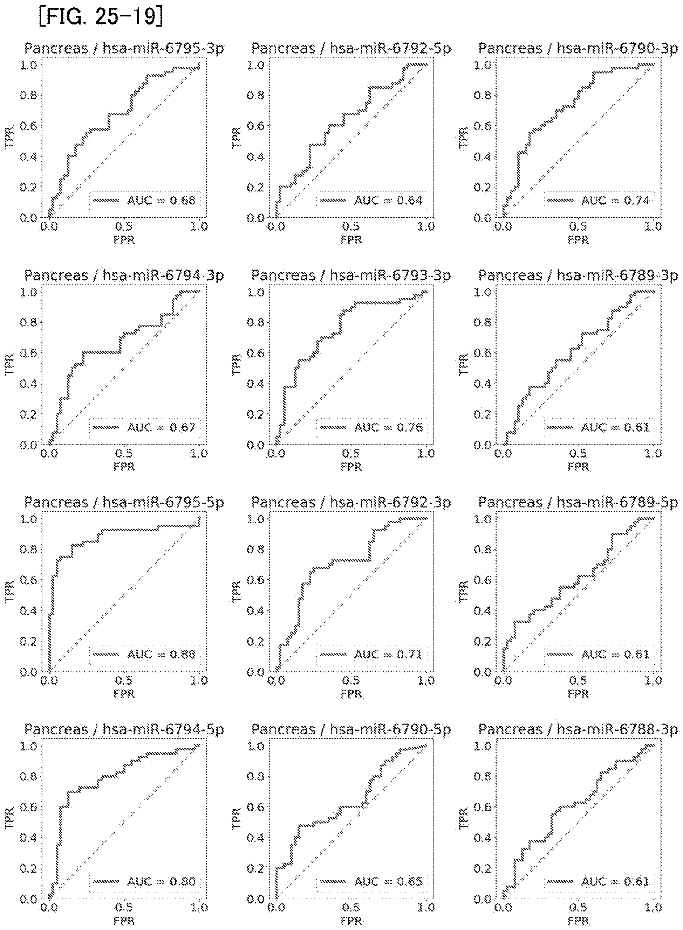

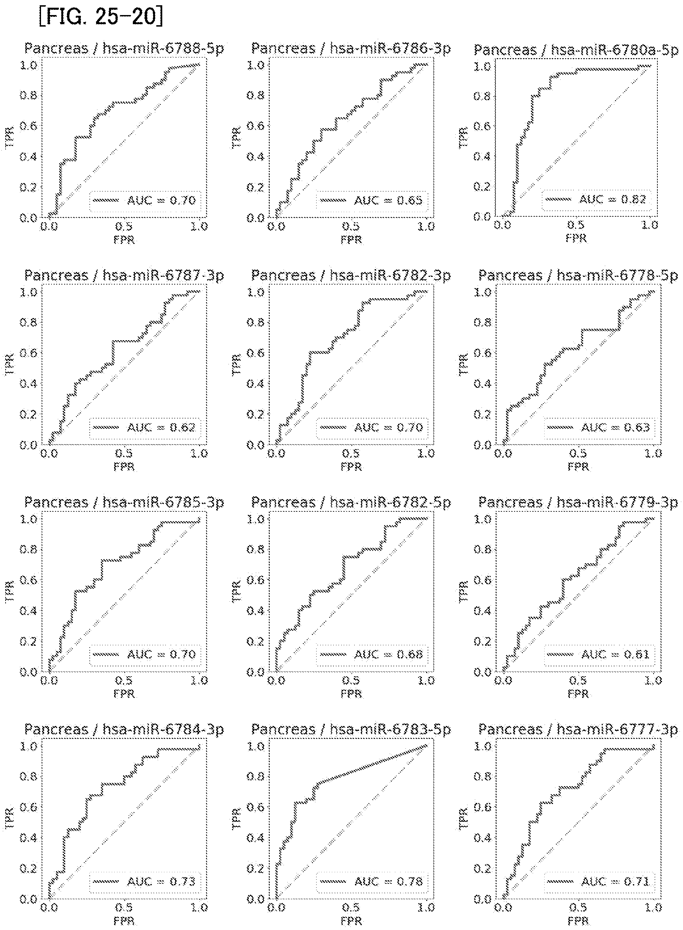

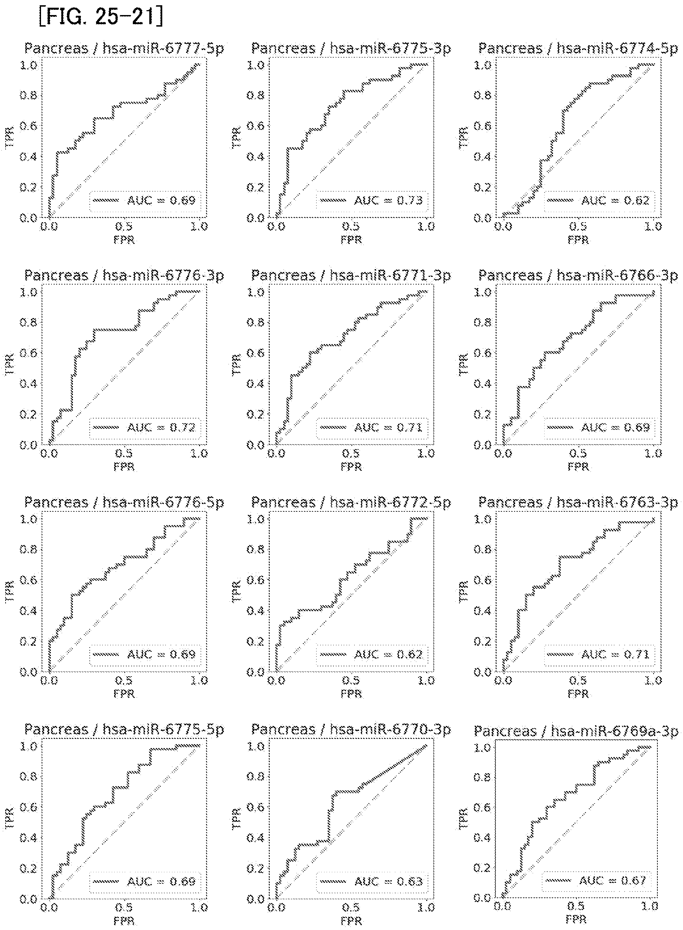

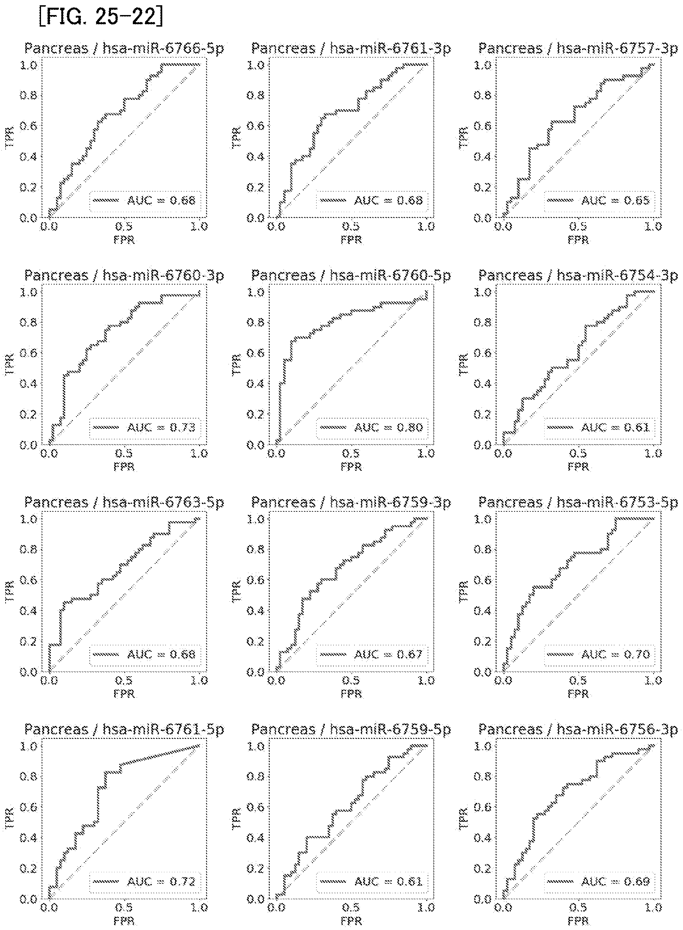

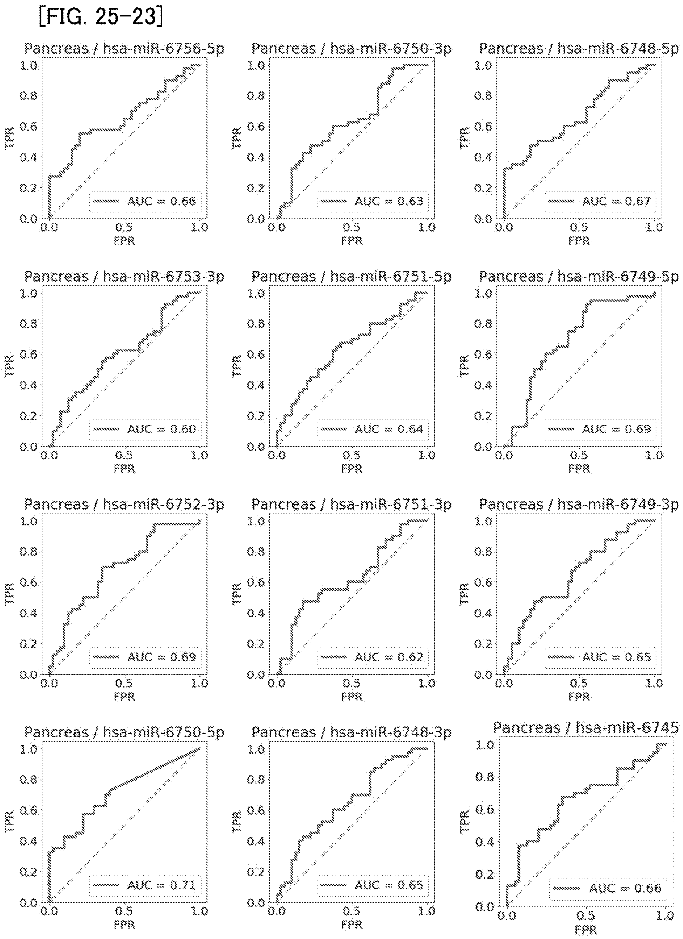

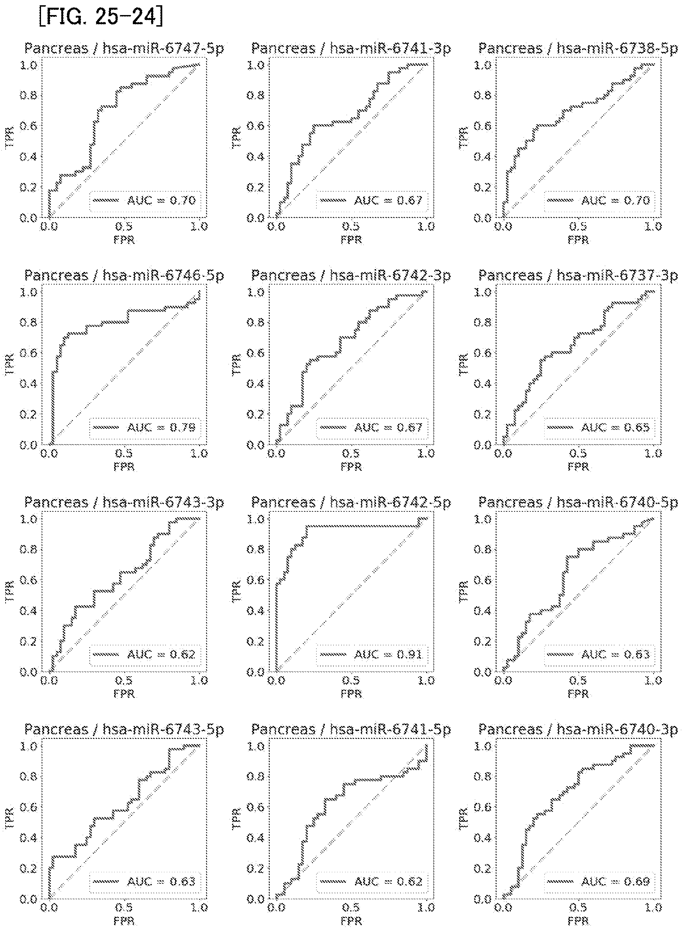

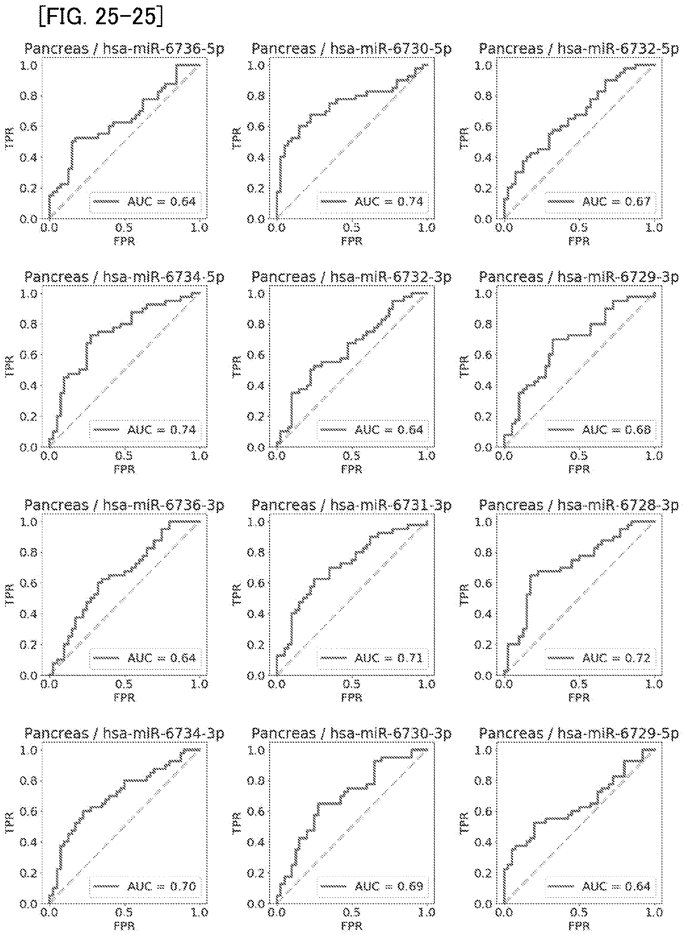

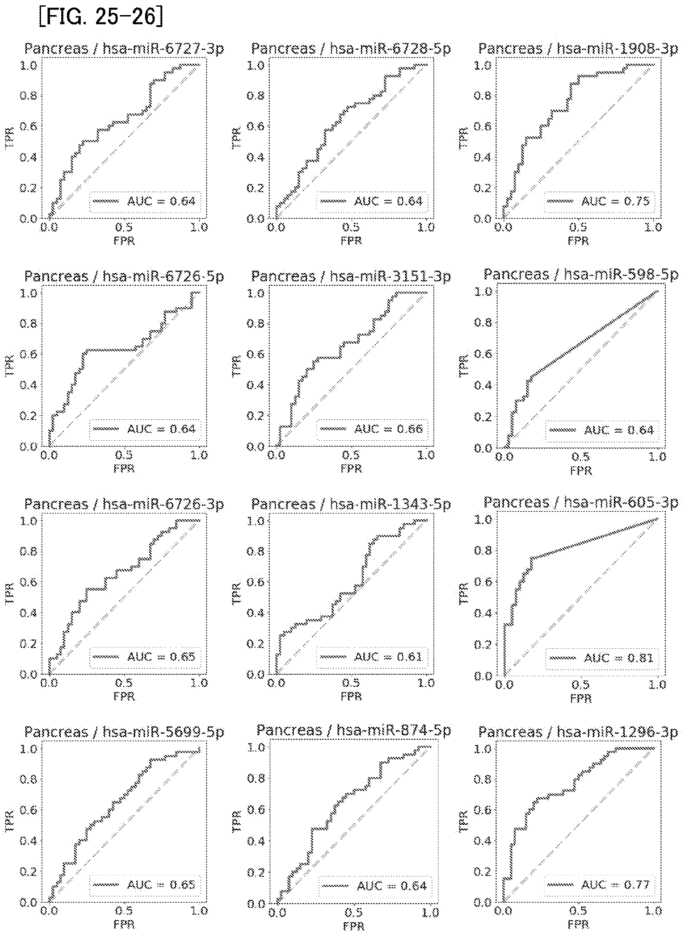

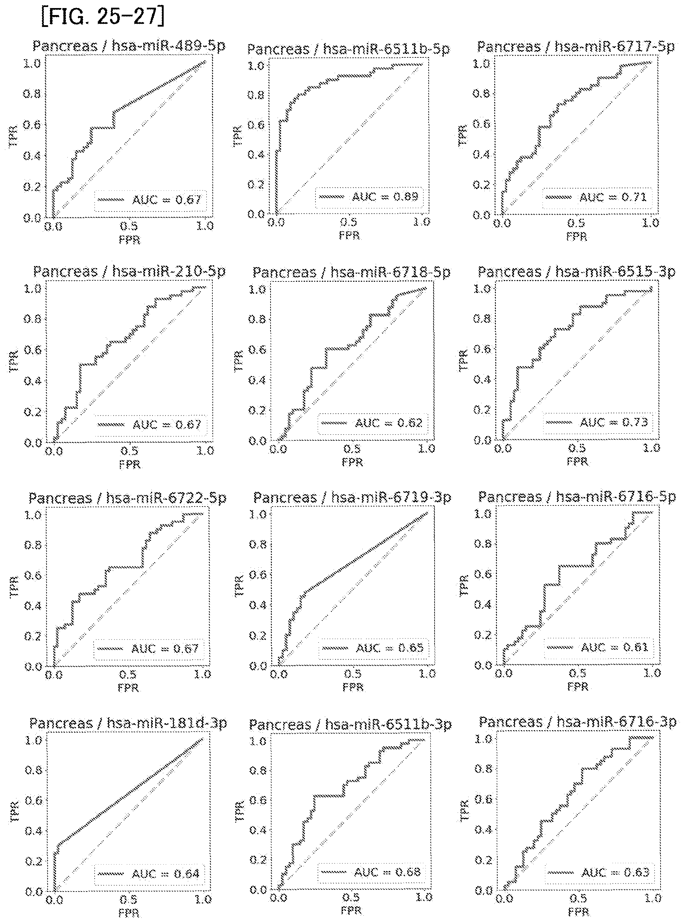

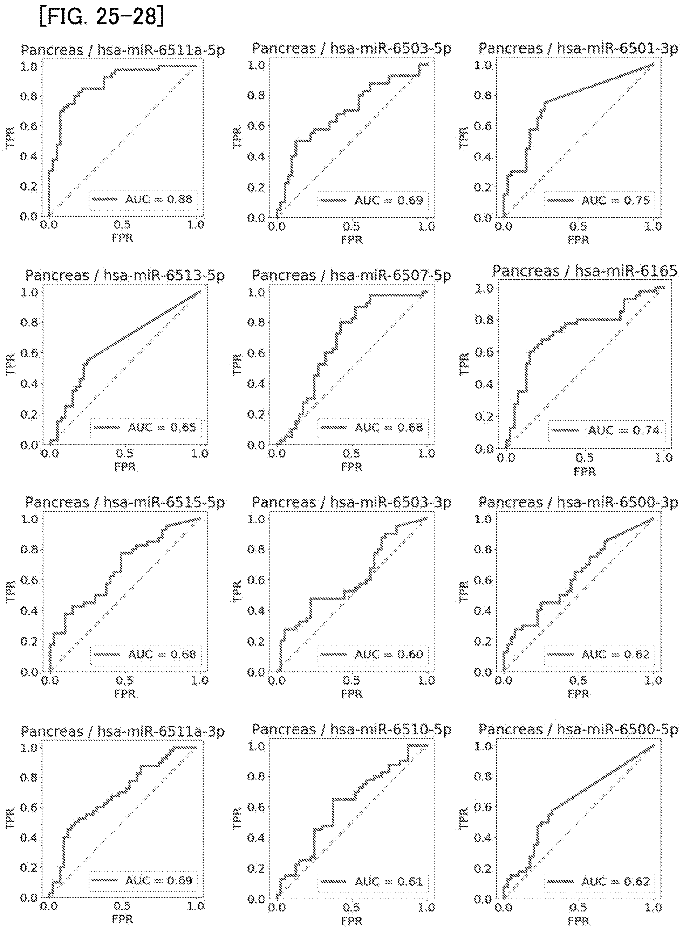

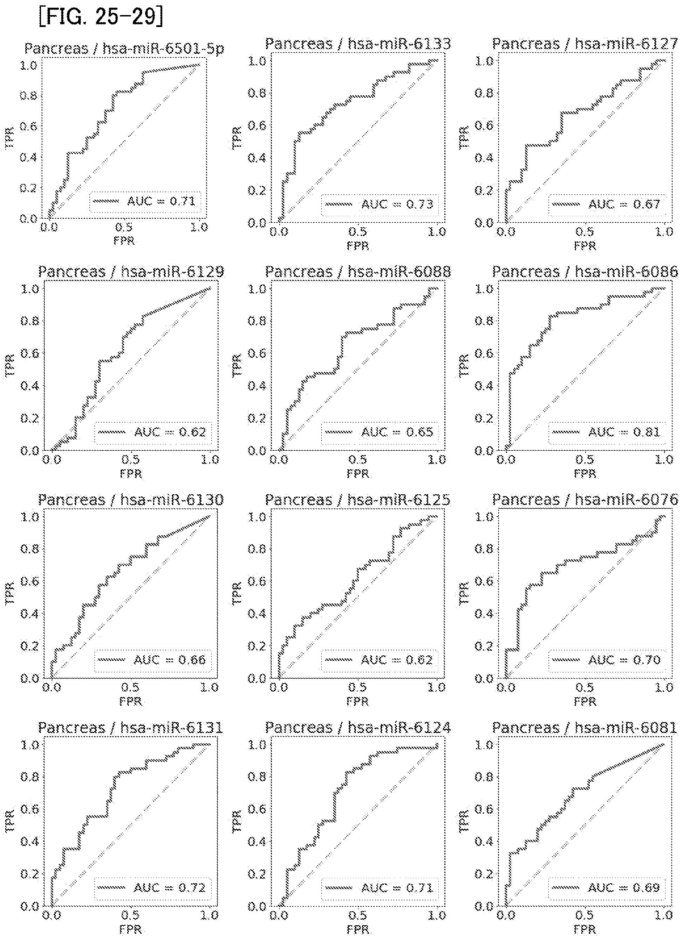

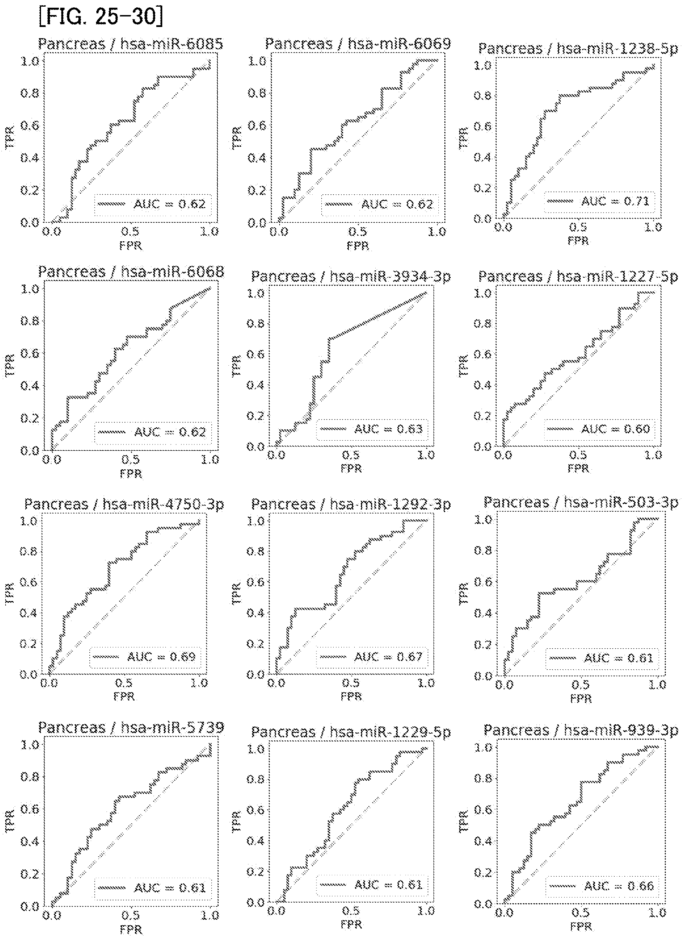

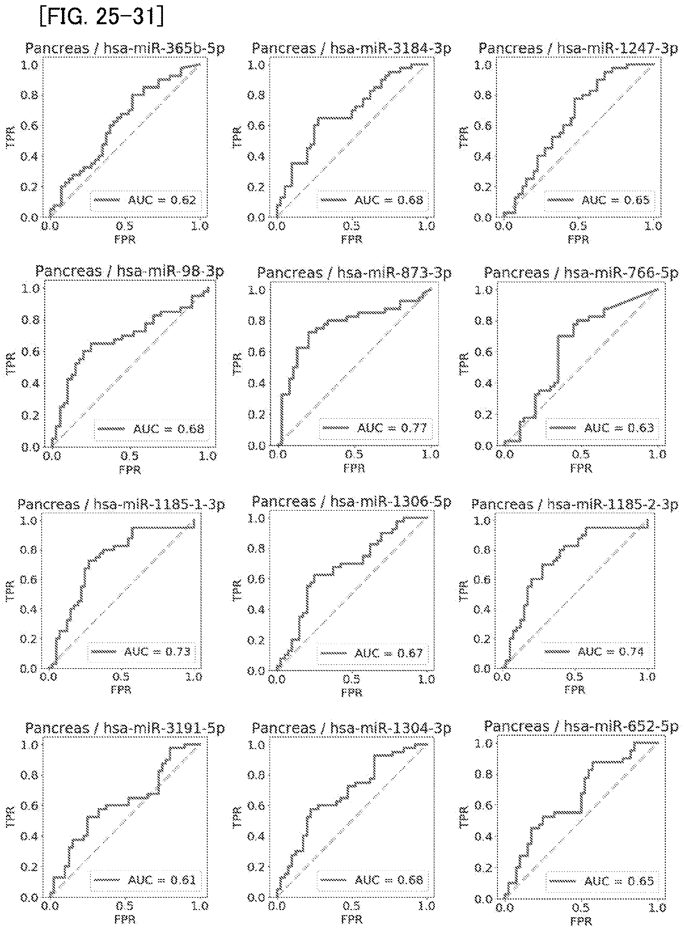

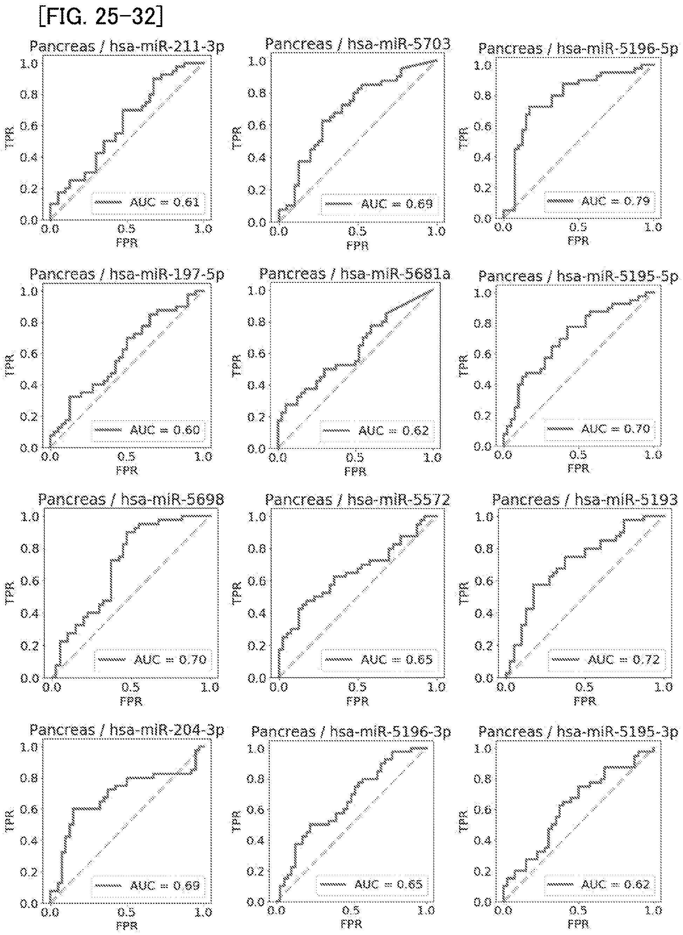

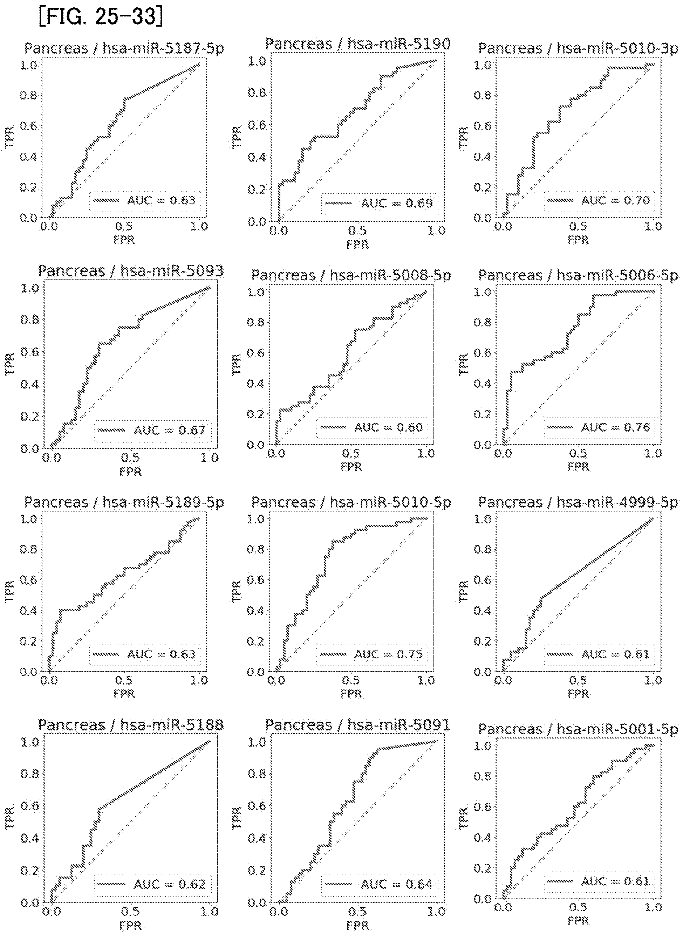

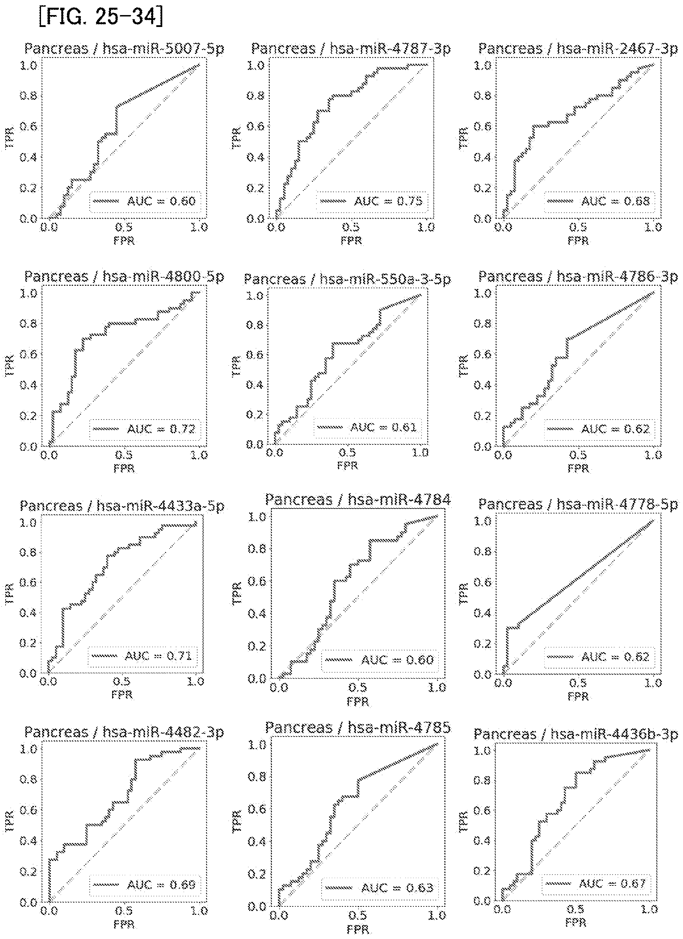

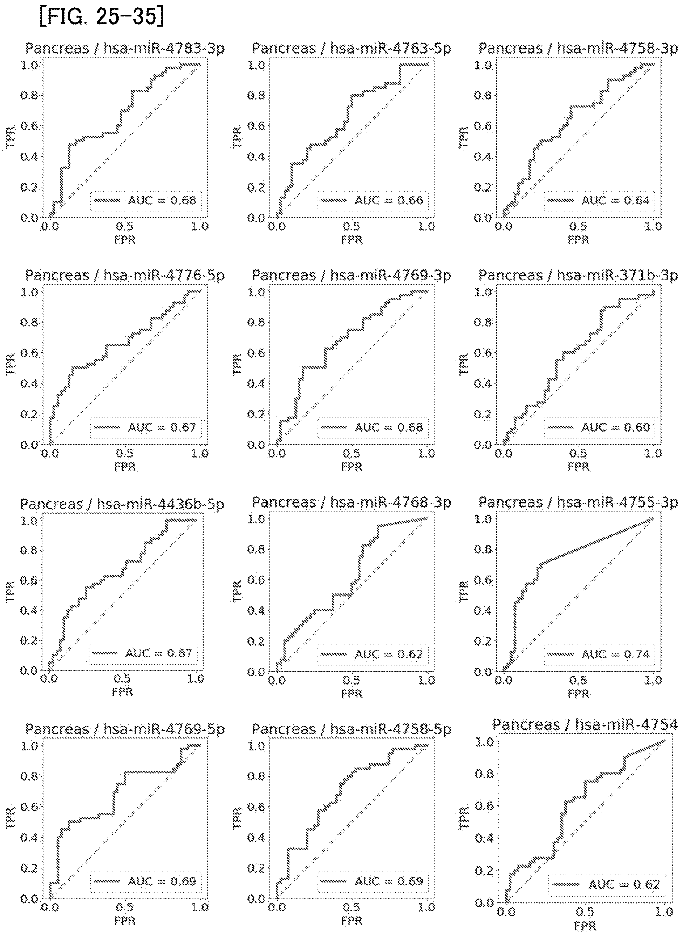

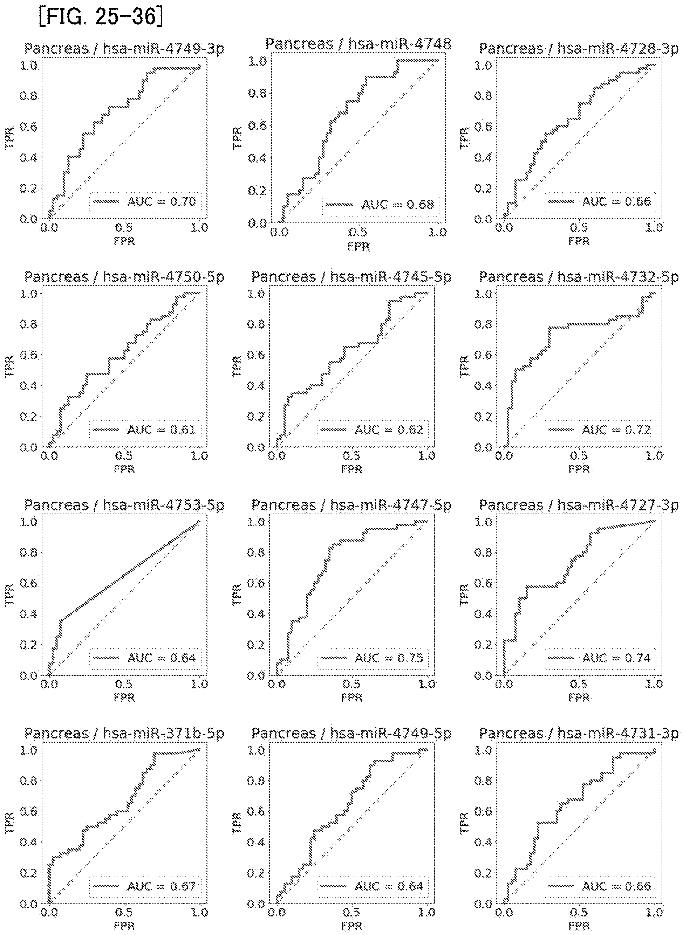

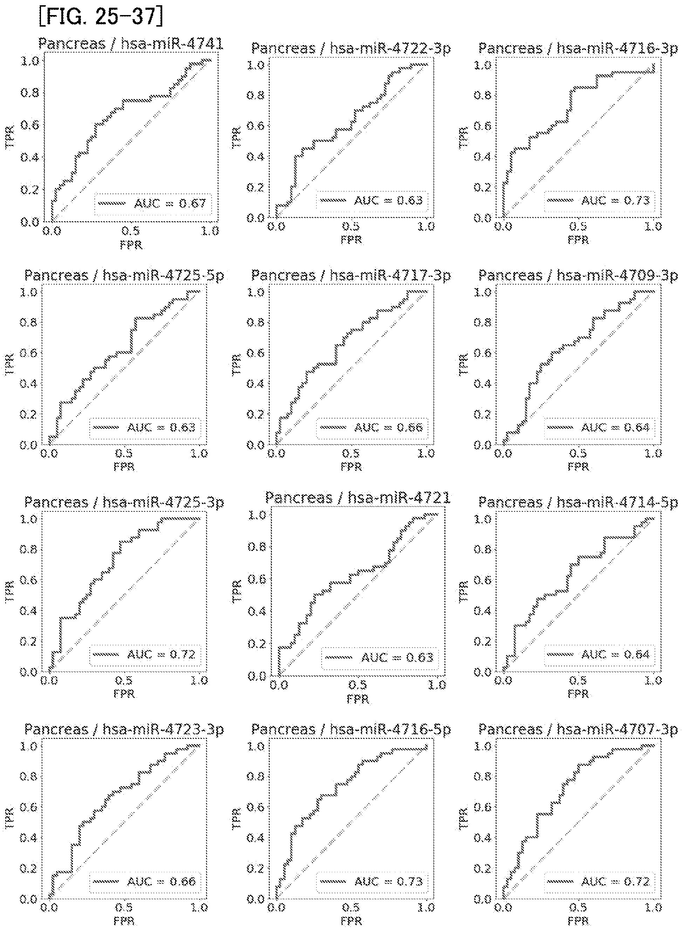

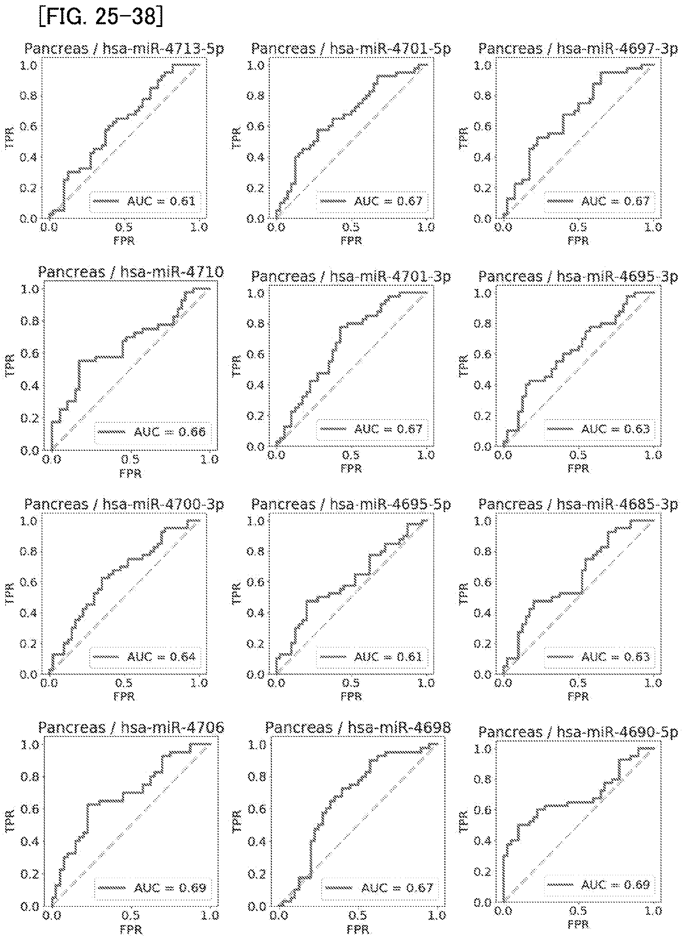

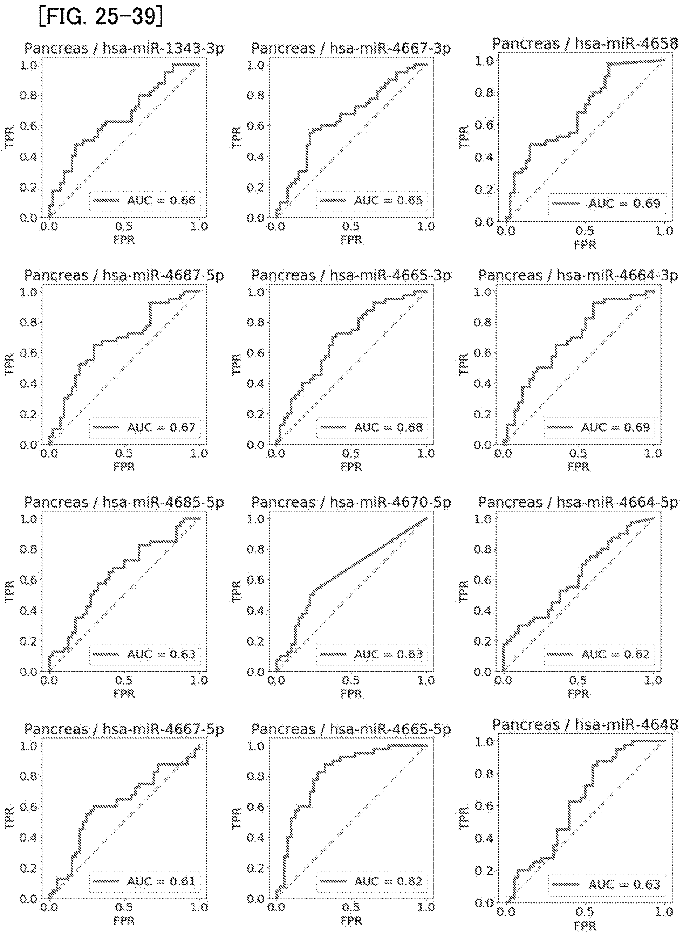

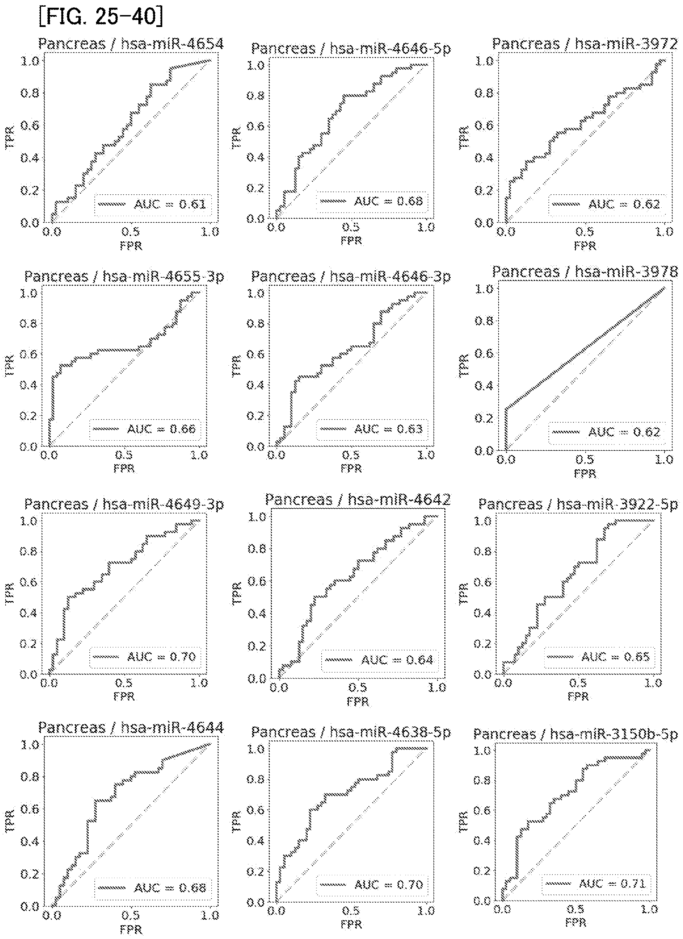

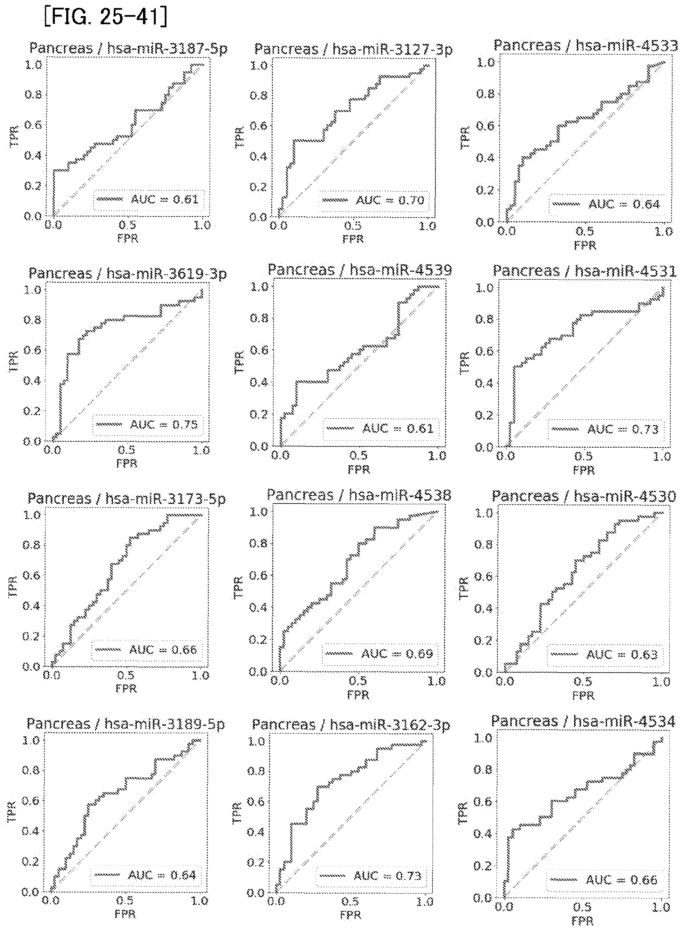

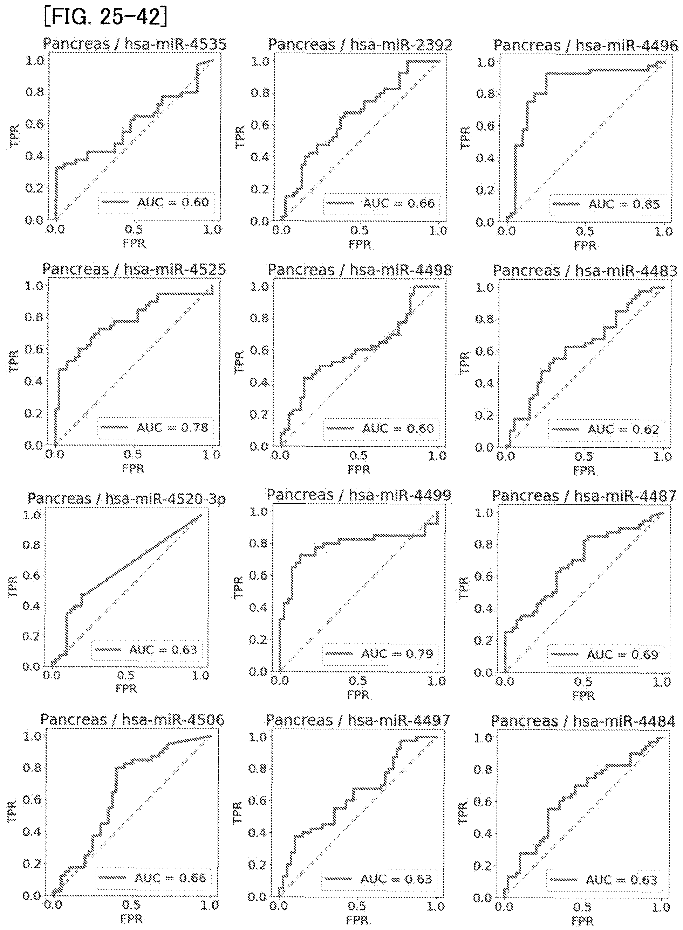

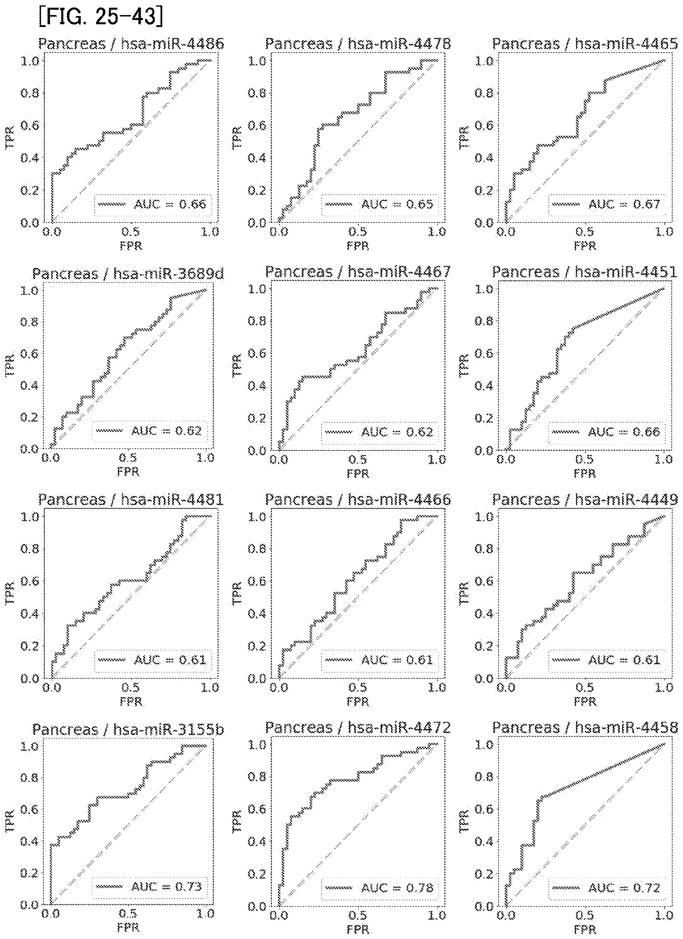

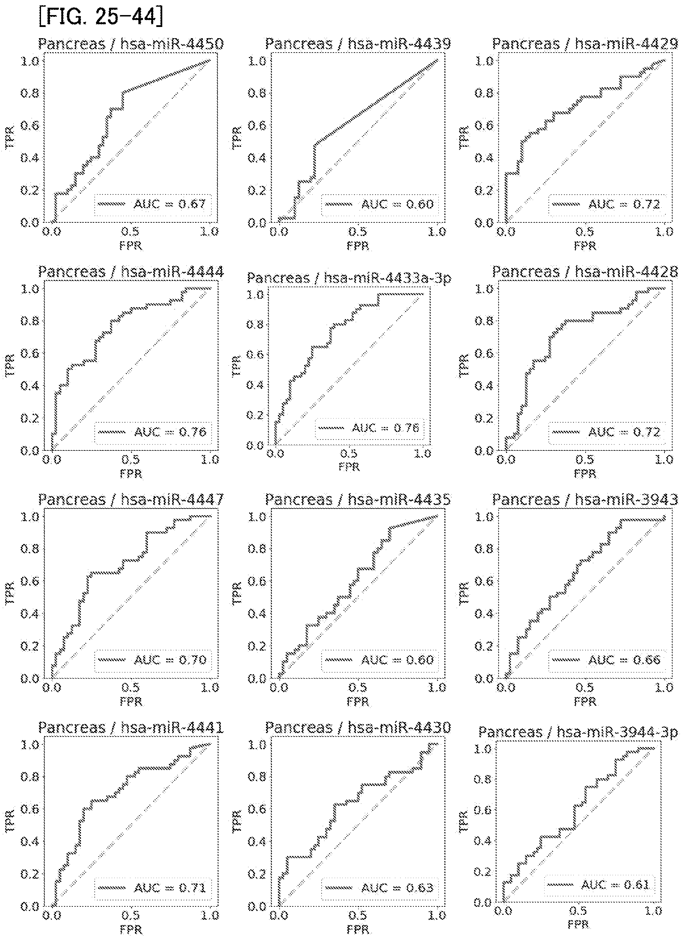

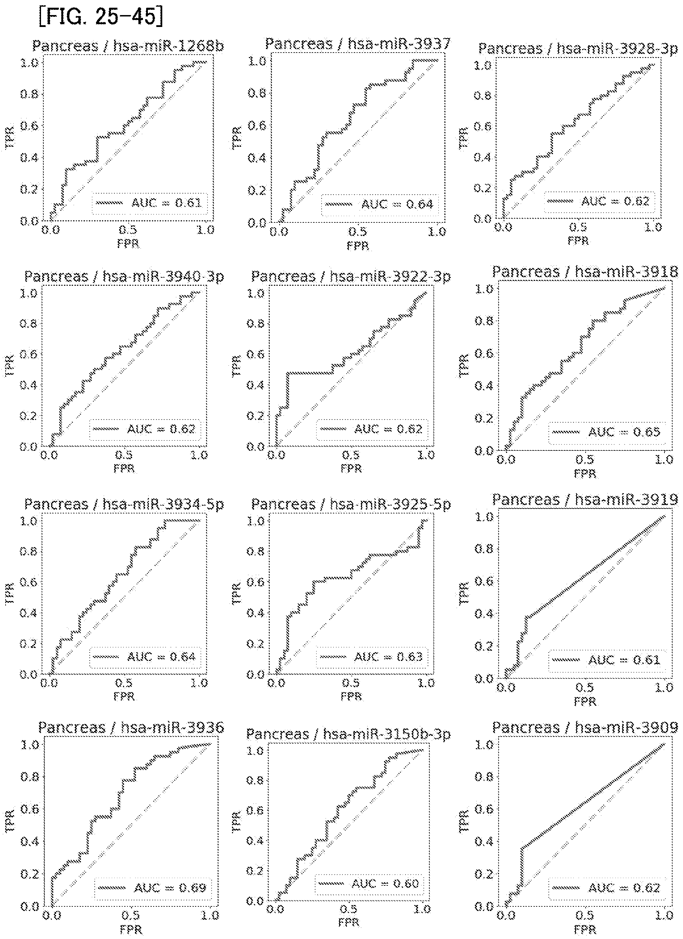

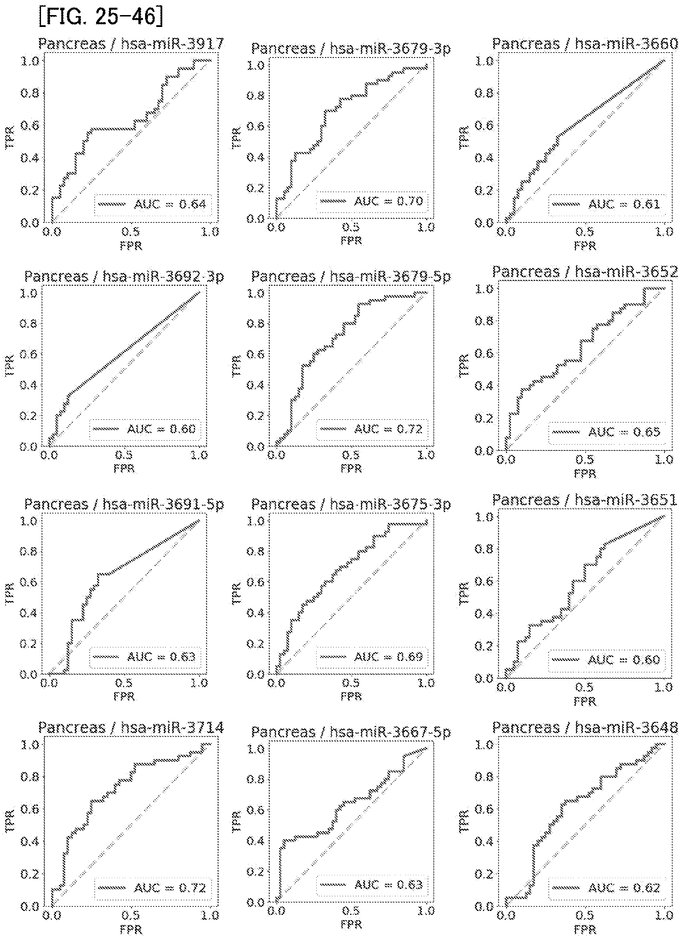

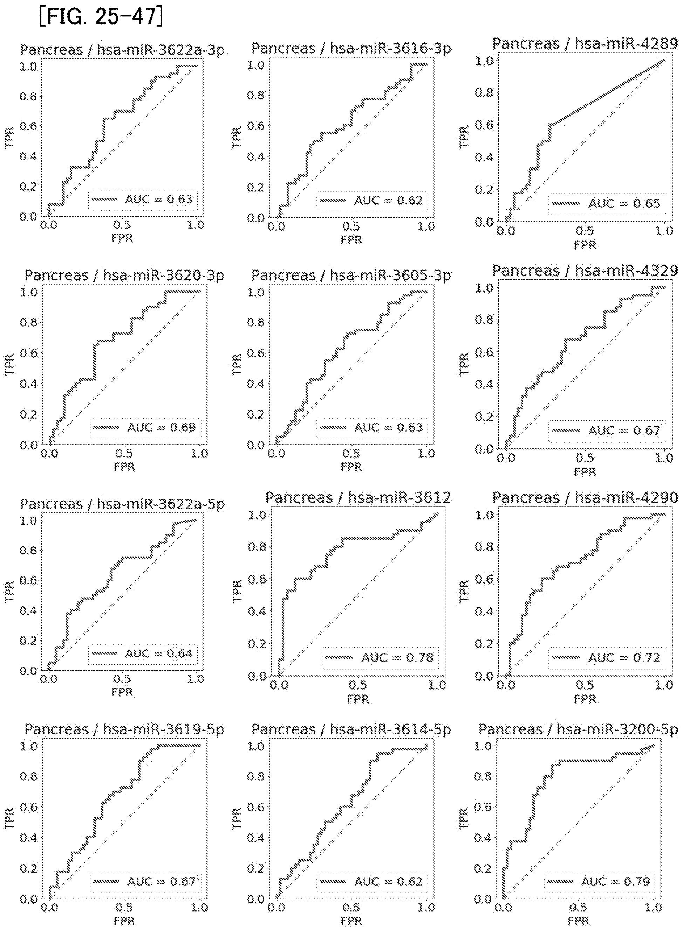

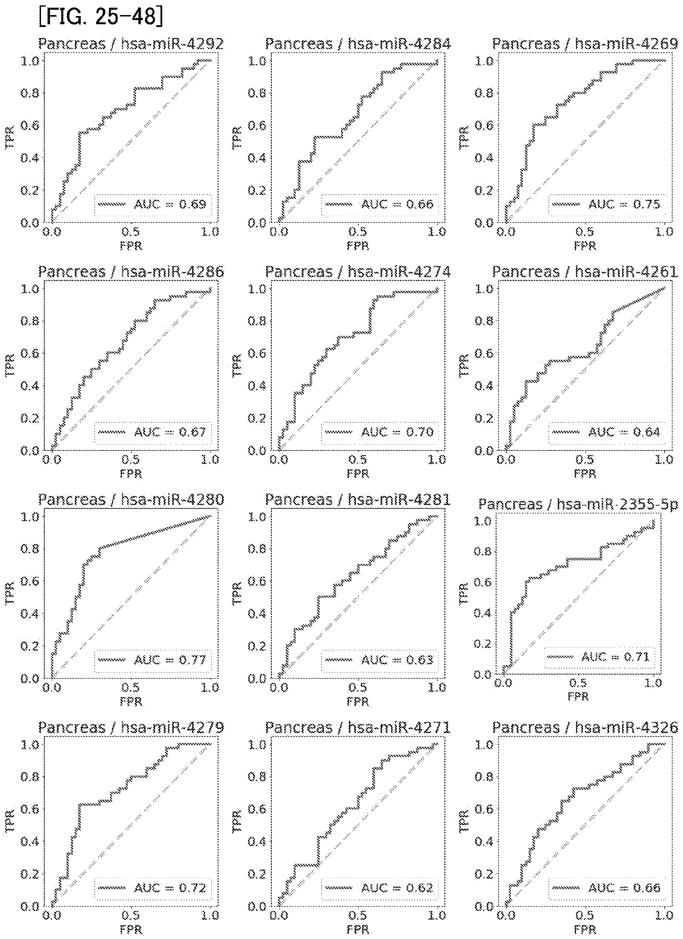

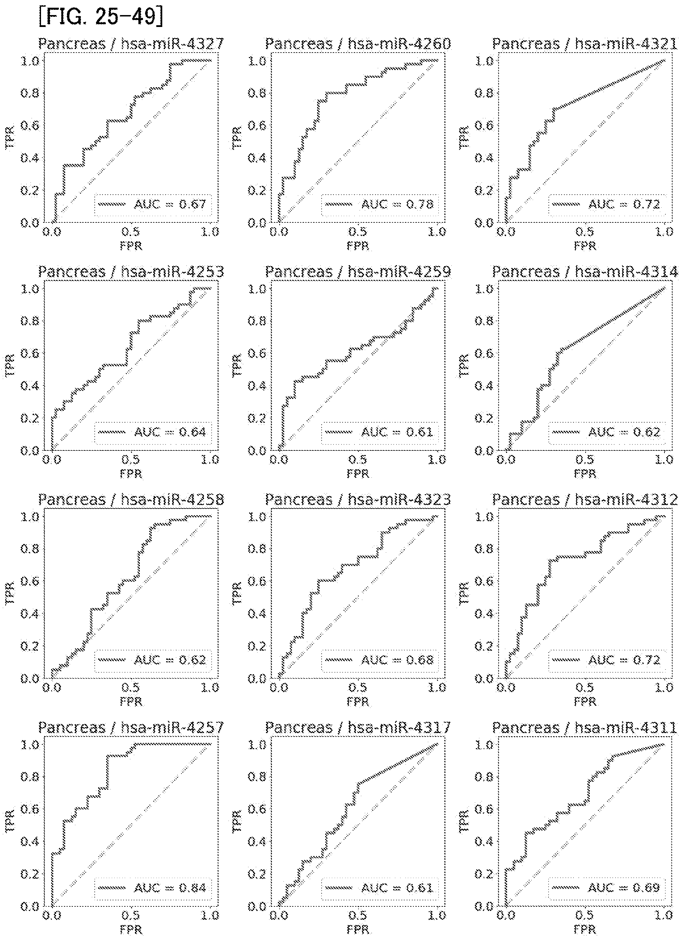

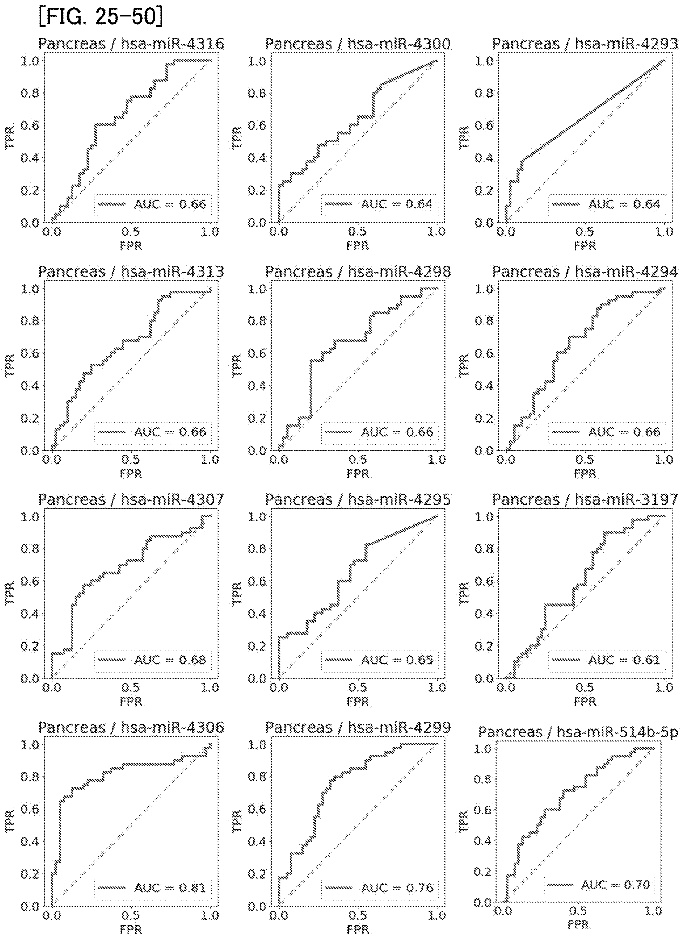

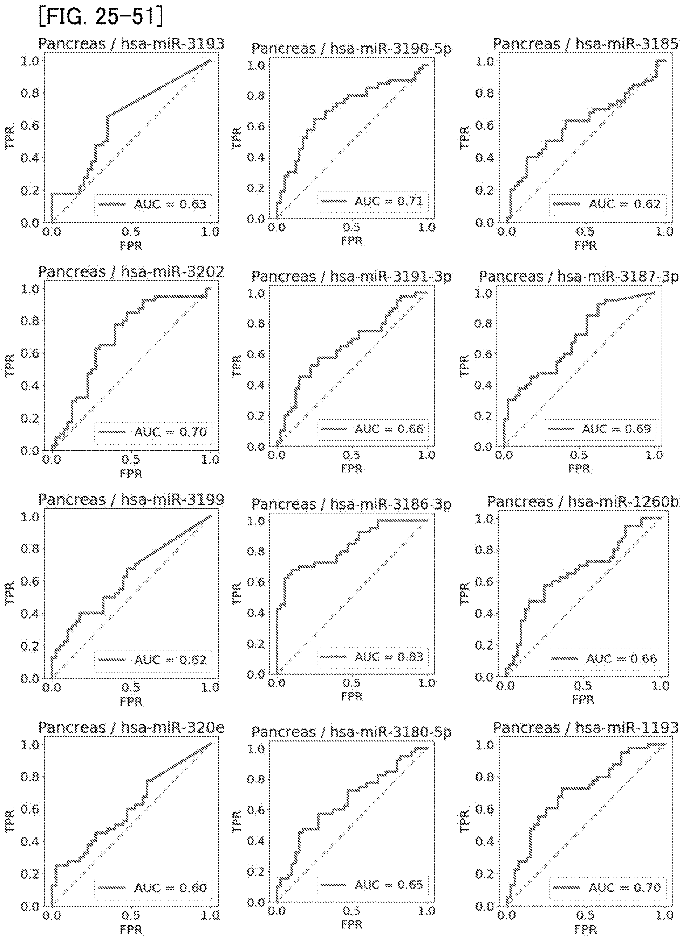

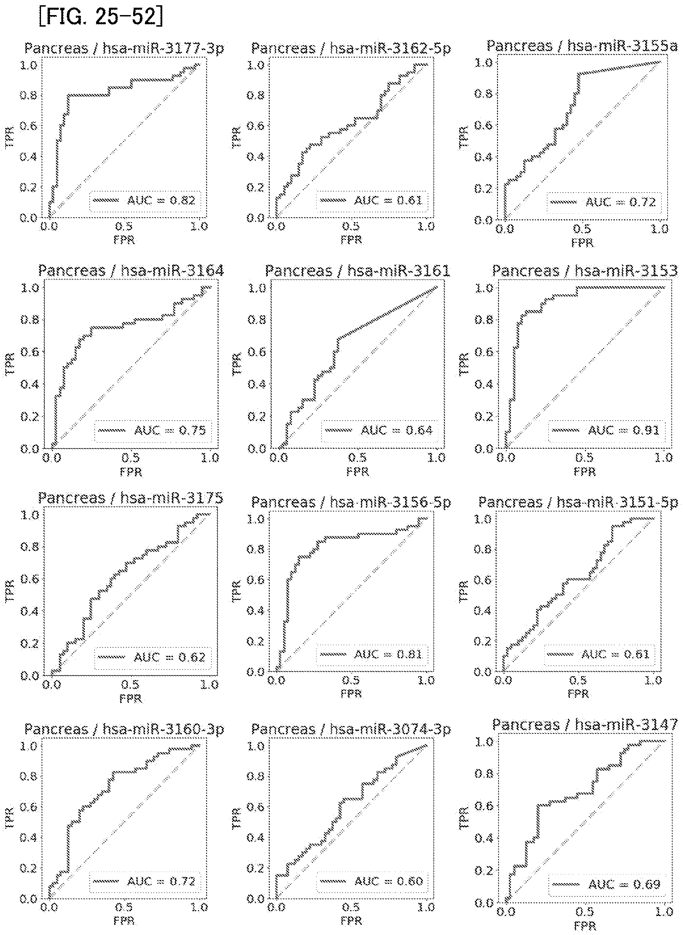

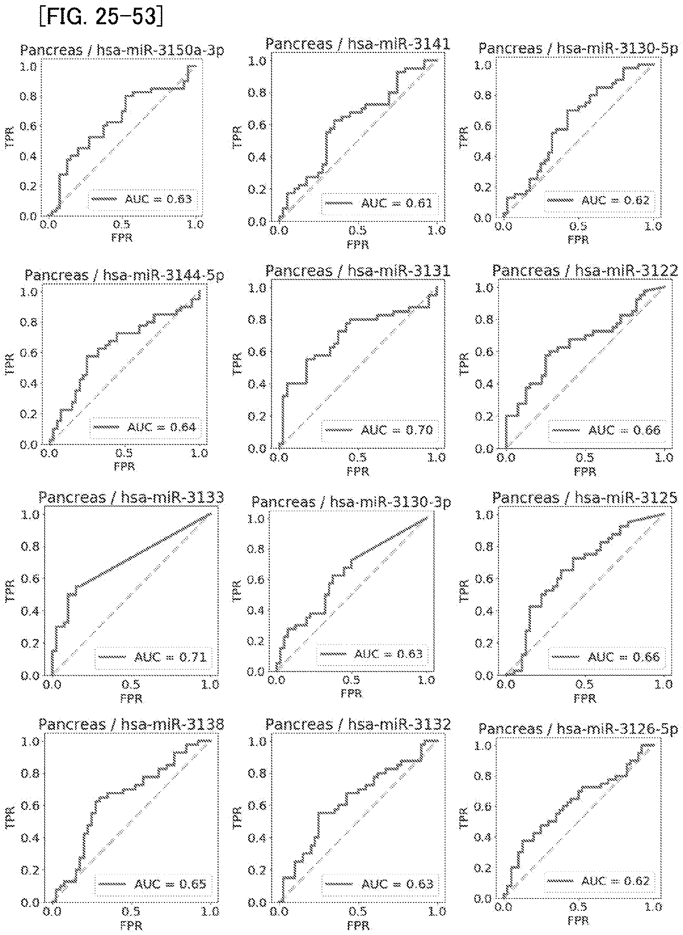

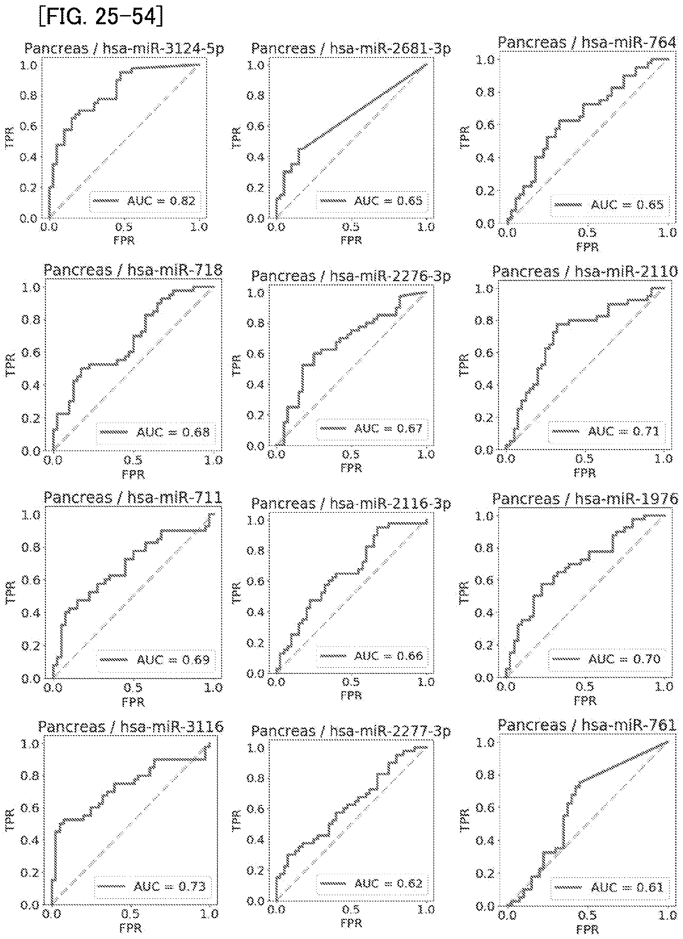

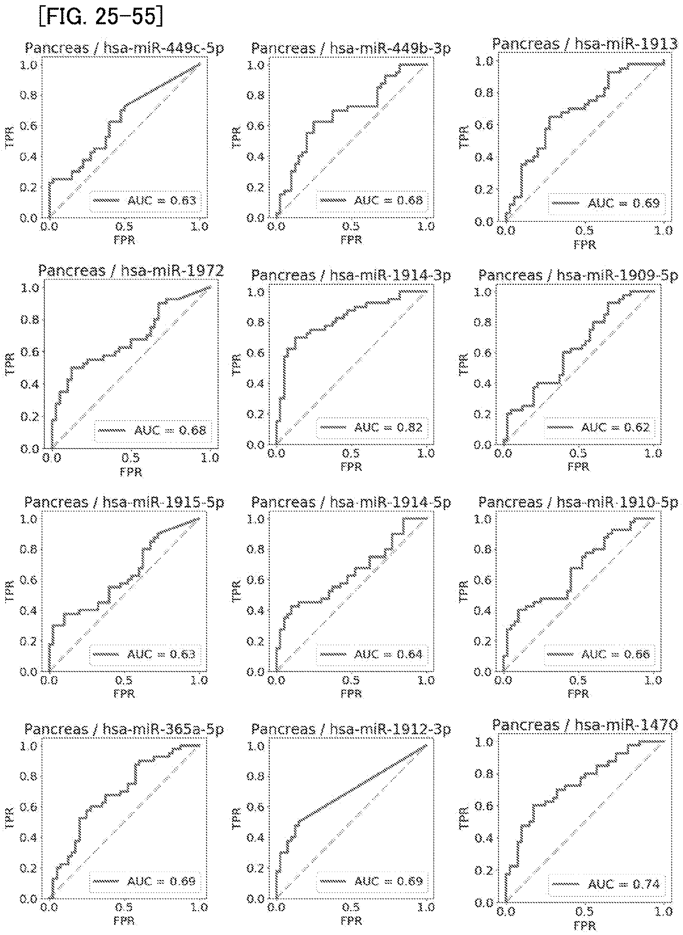

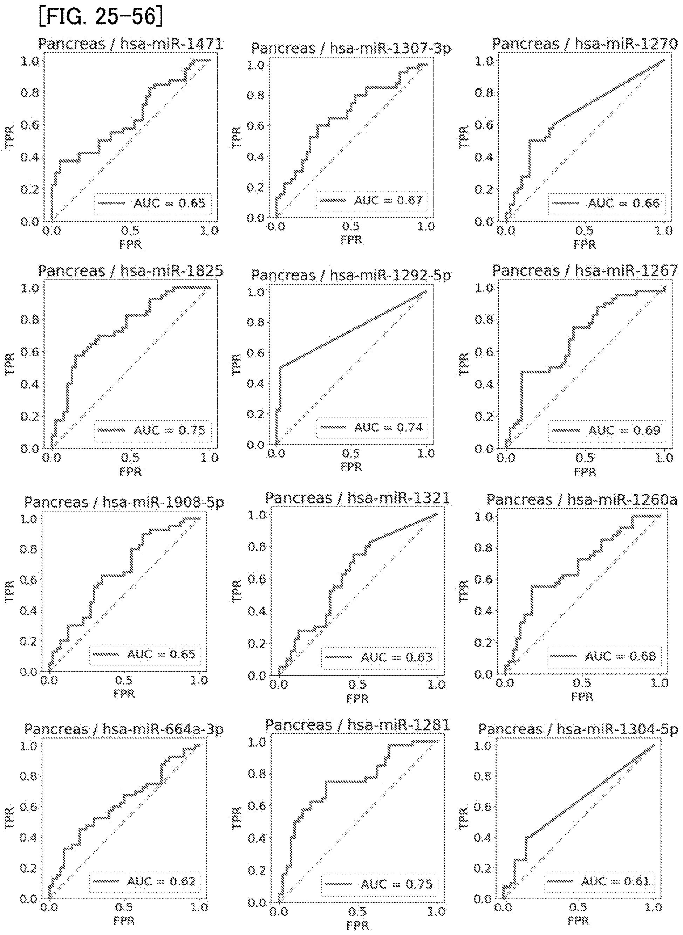

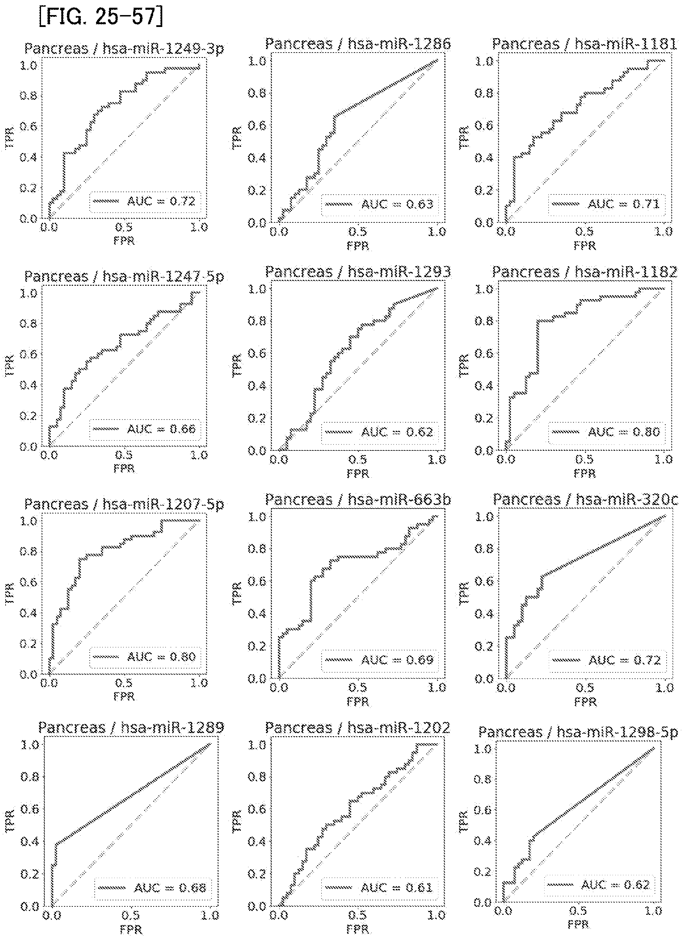

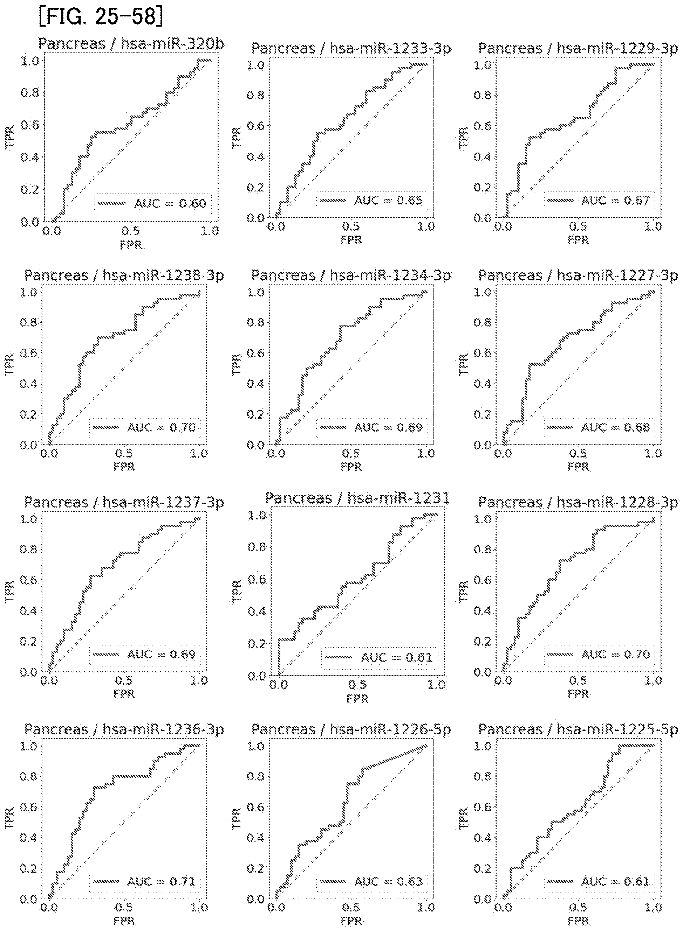

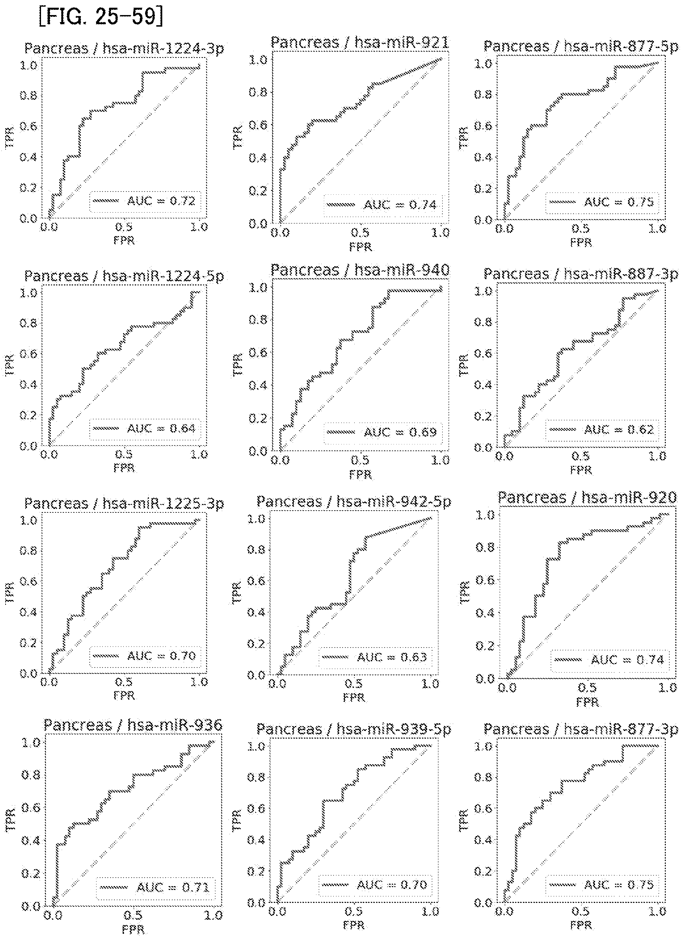

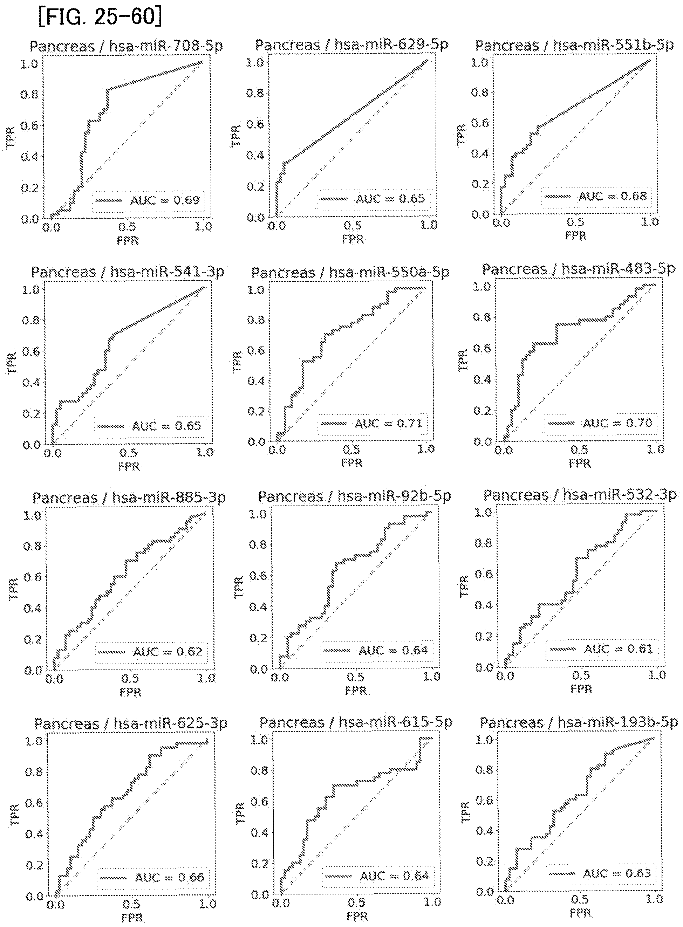

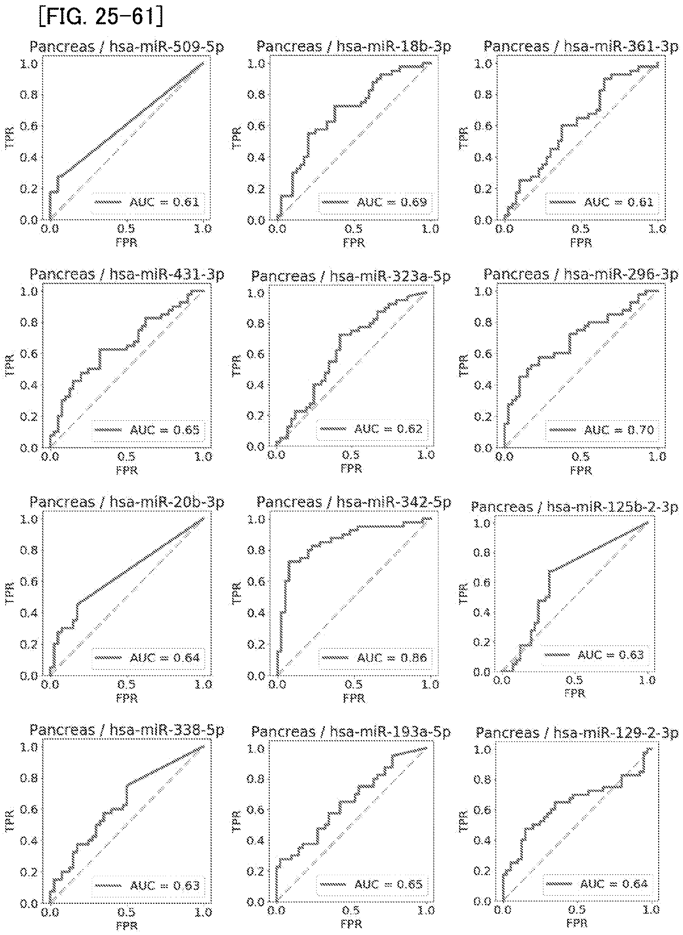

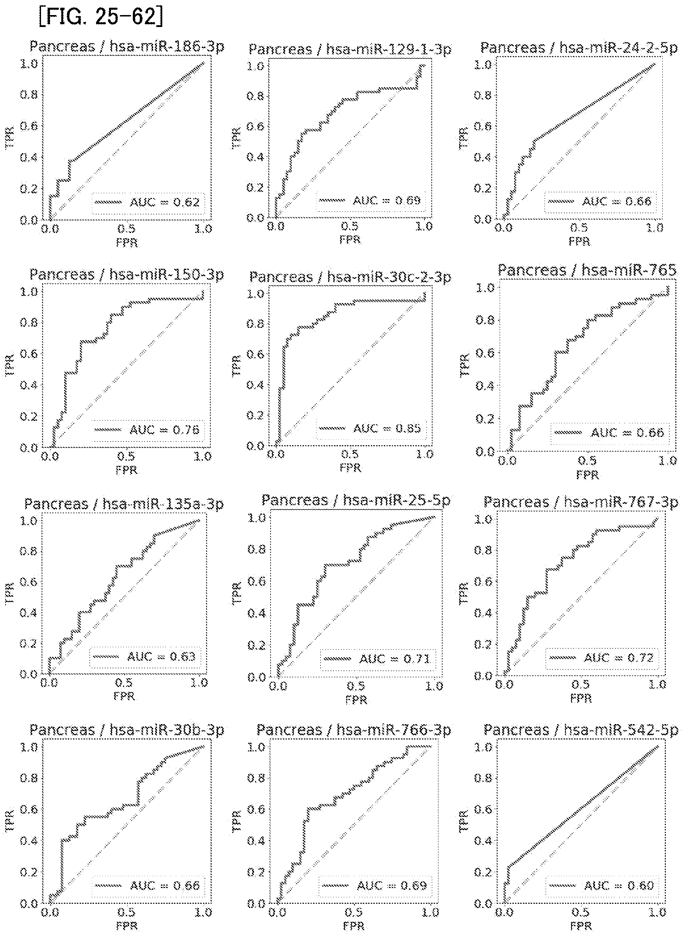

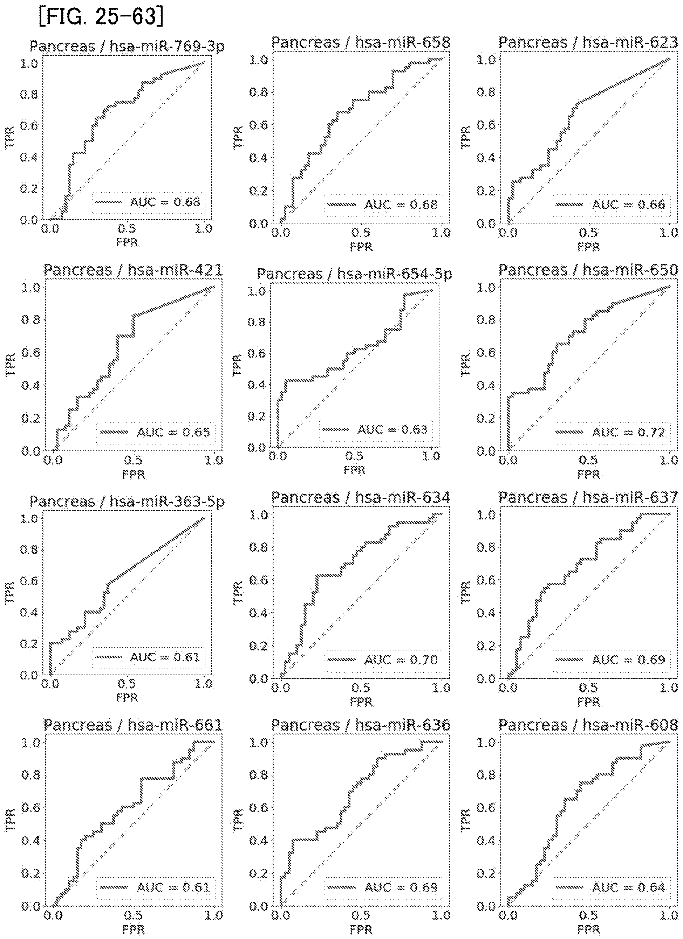

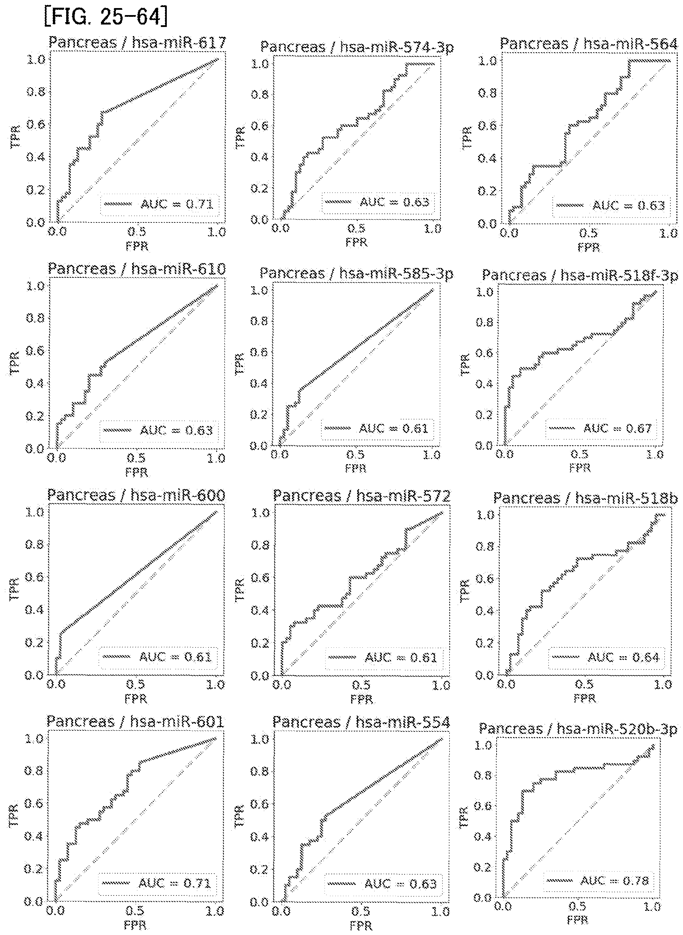

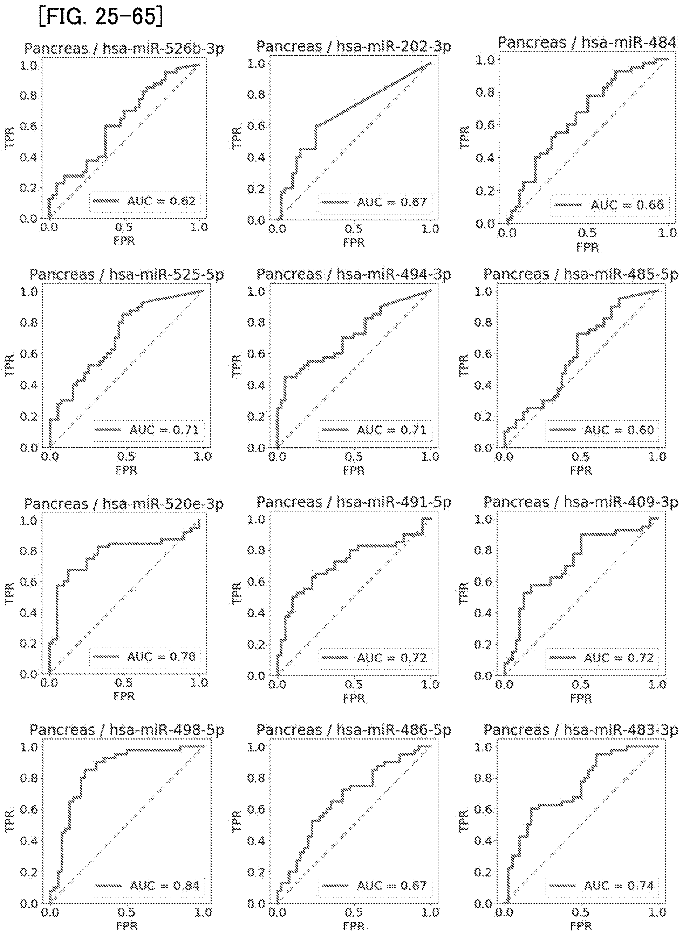

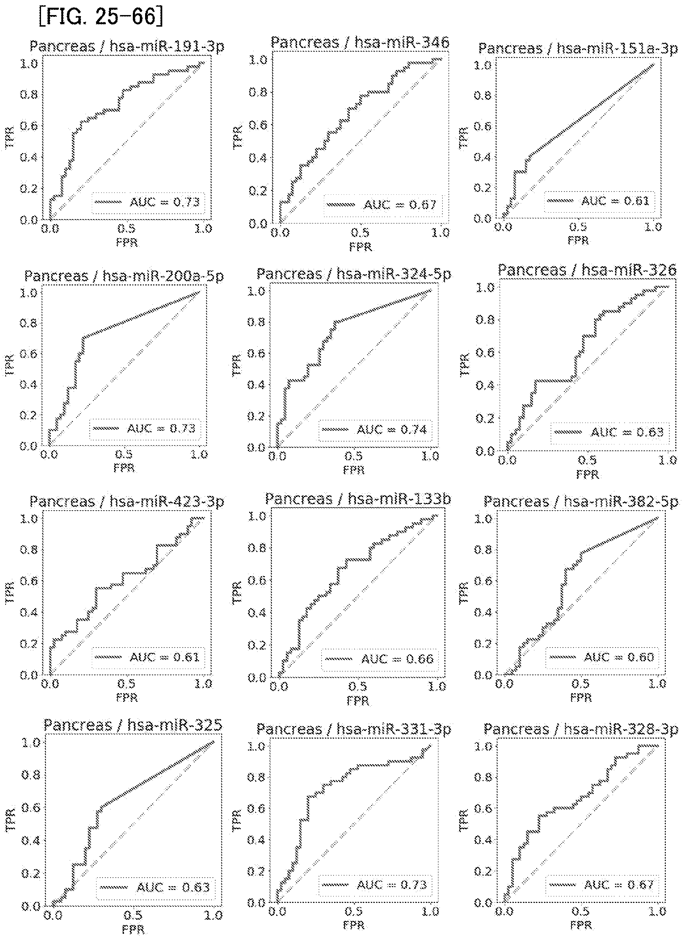

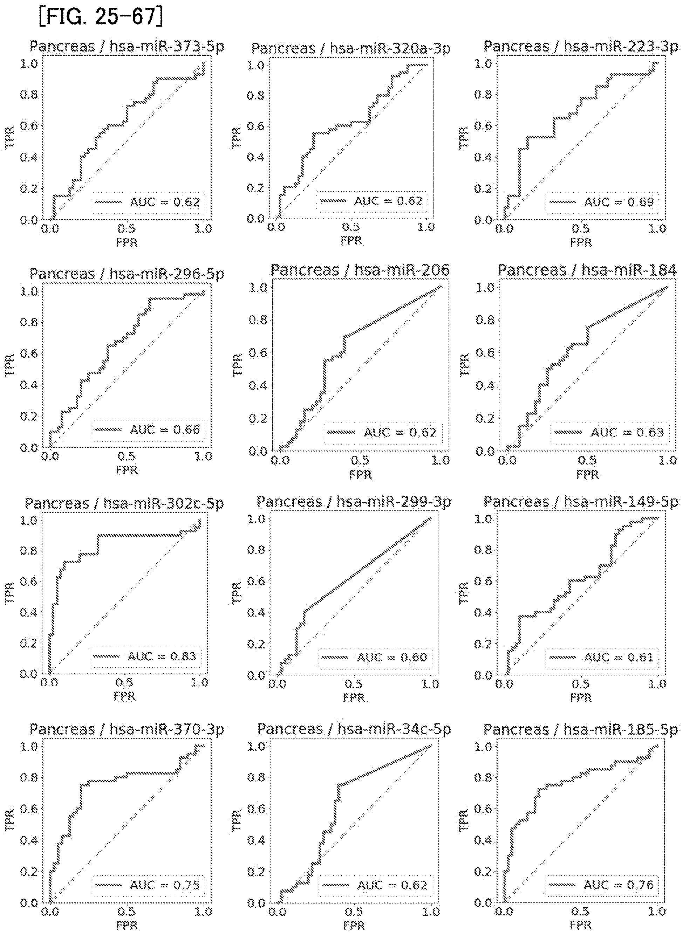

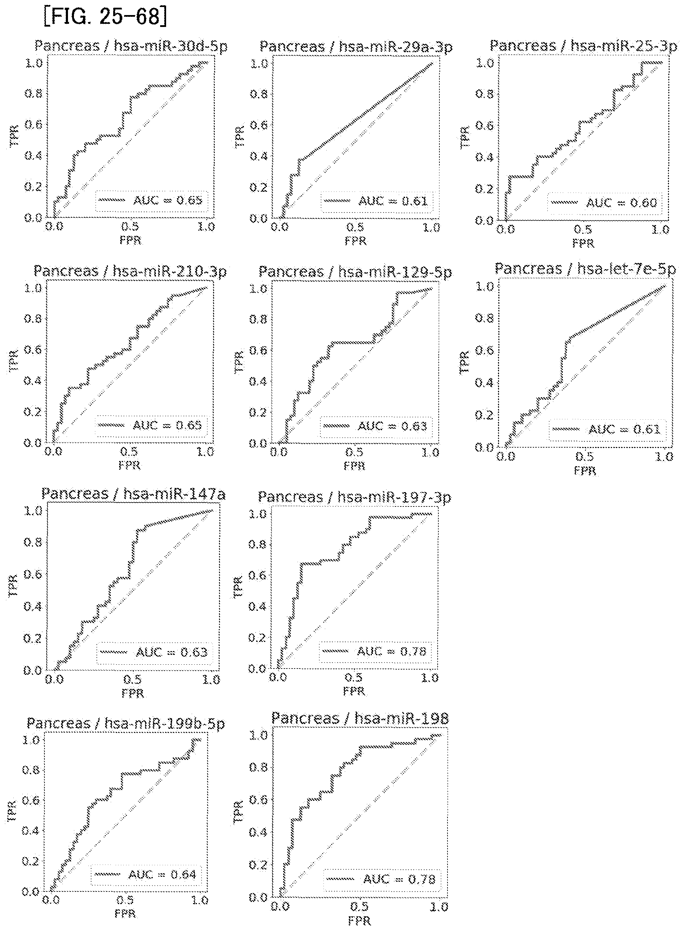

FIGS. 25-1 to 25-68 show ROC-curves for predicting pancreatic cancer in humans using a single miRNA.

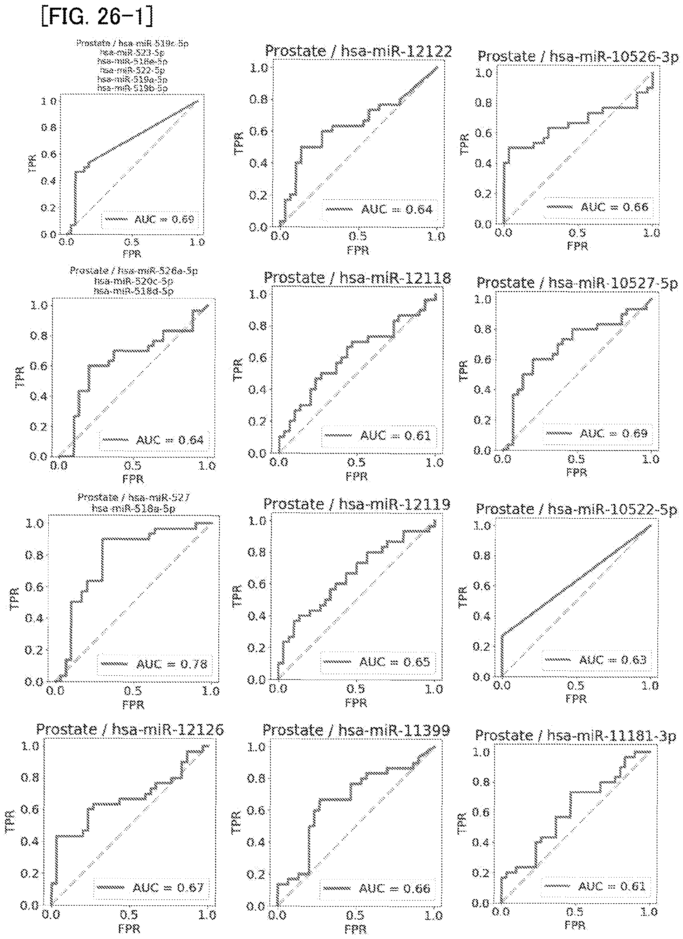

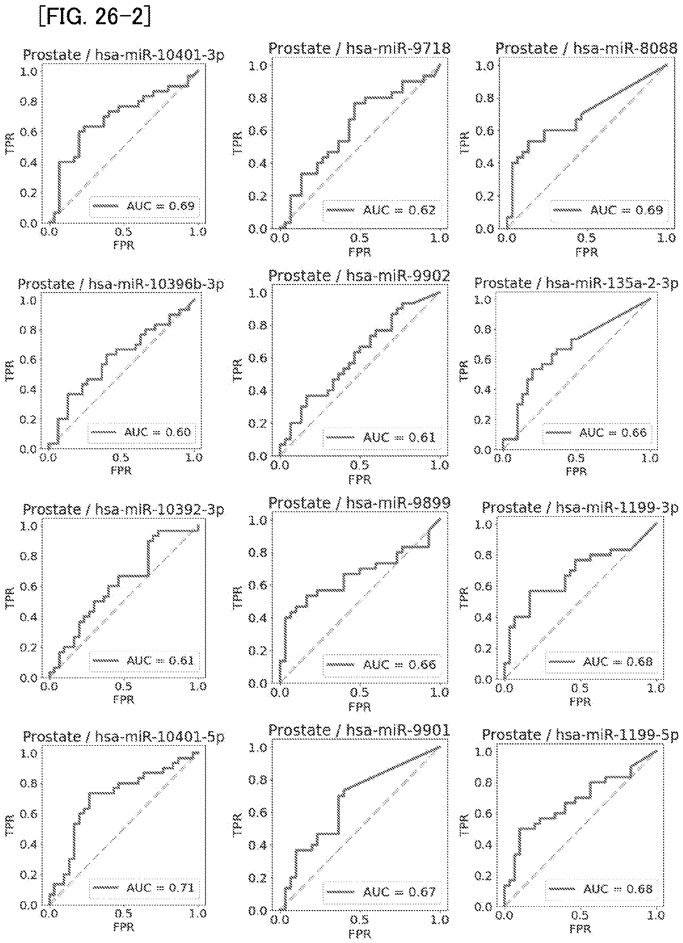

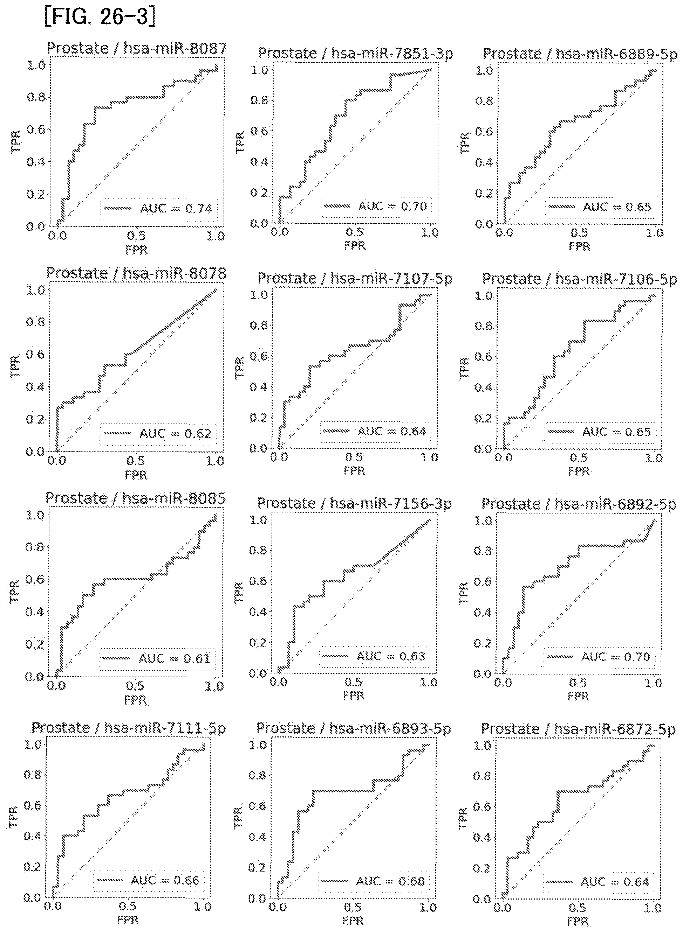

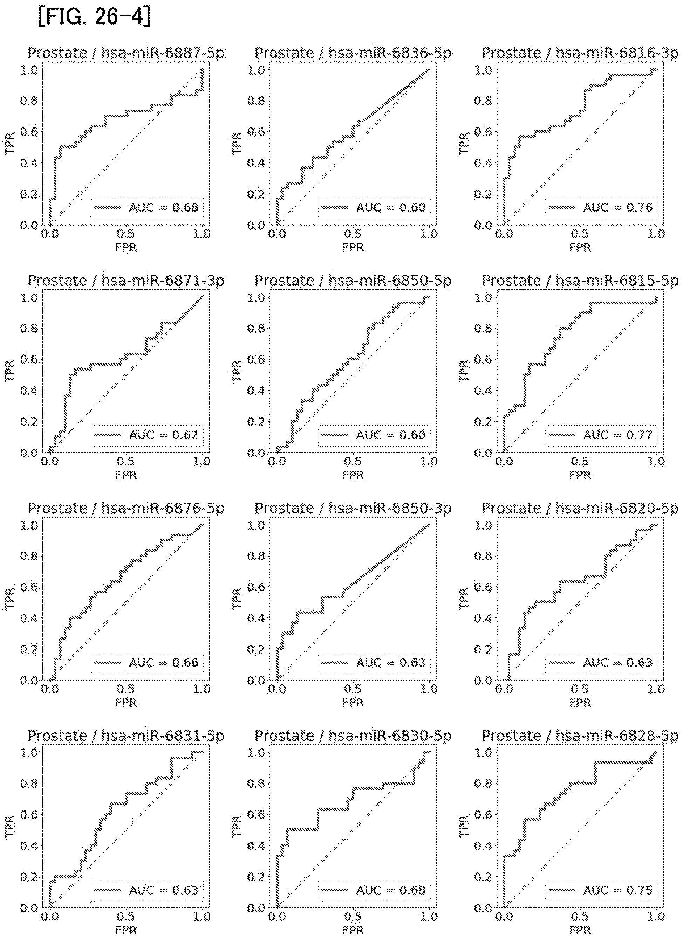

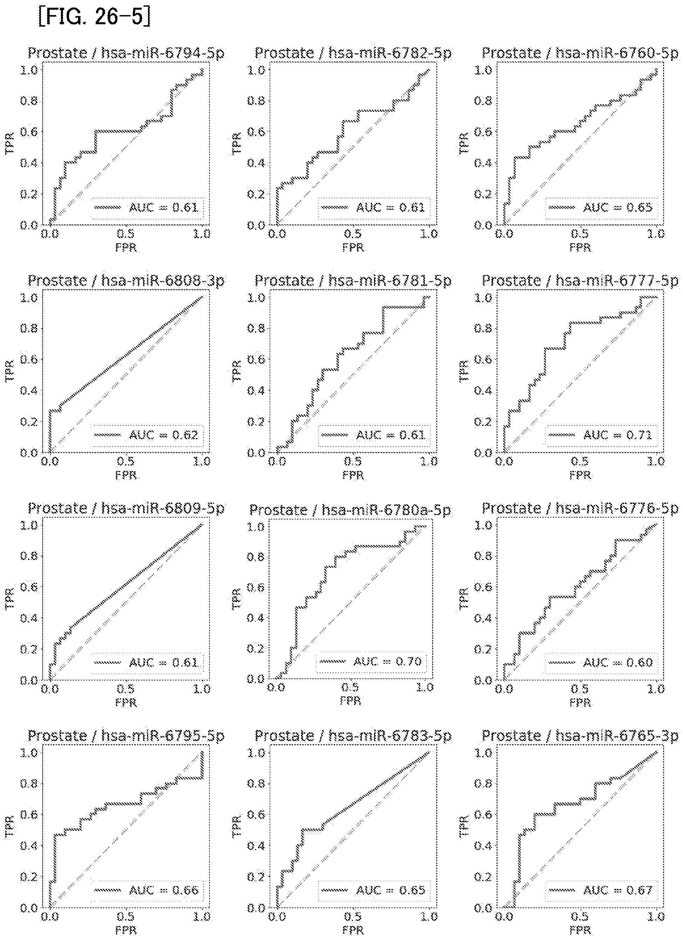

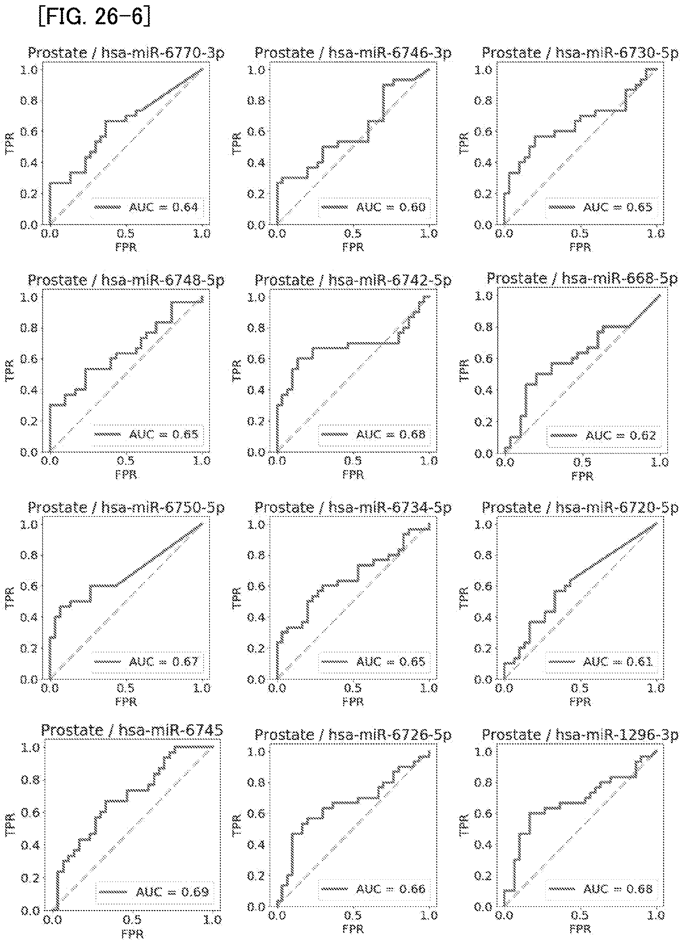

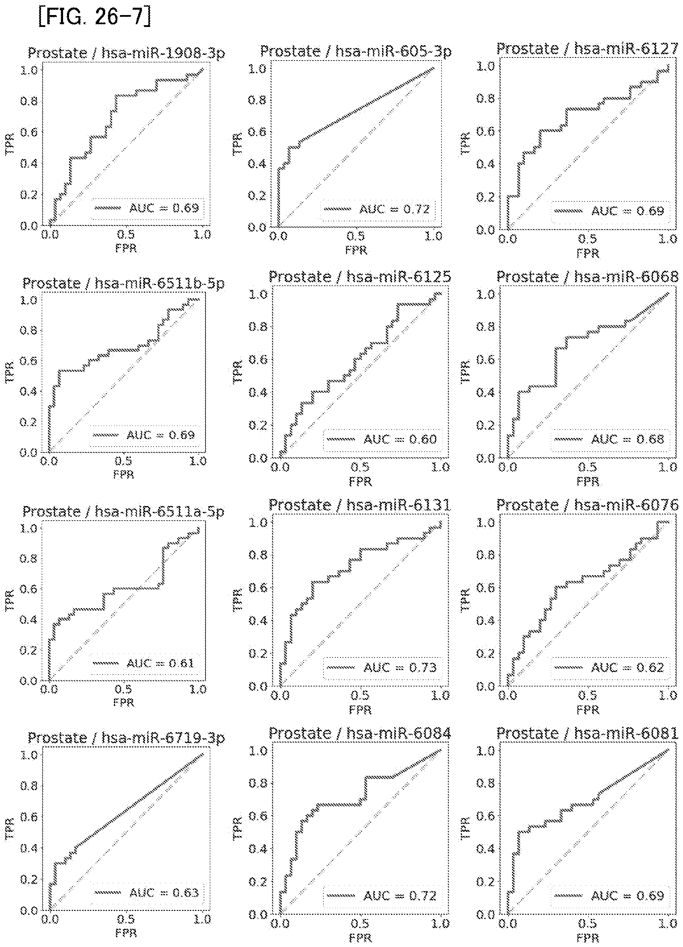

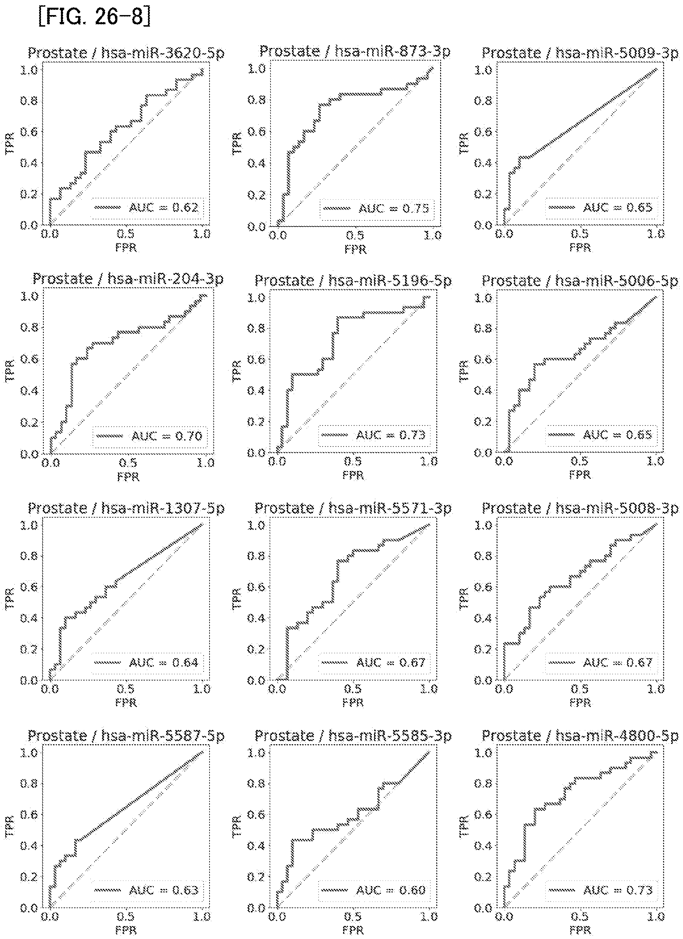

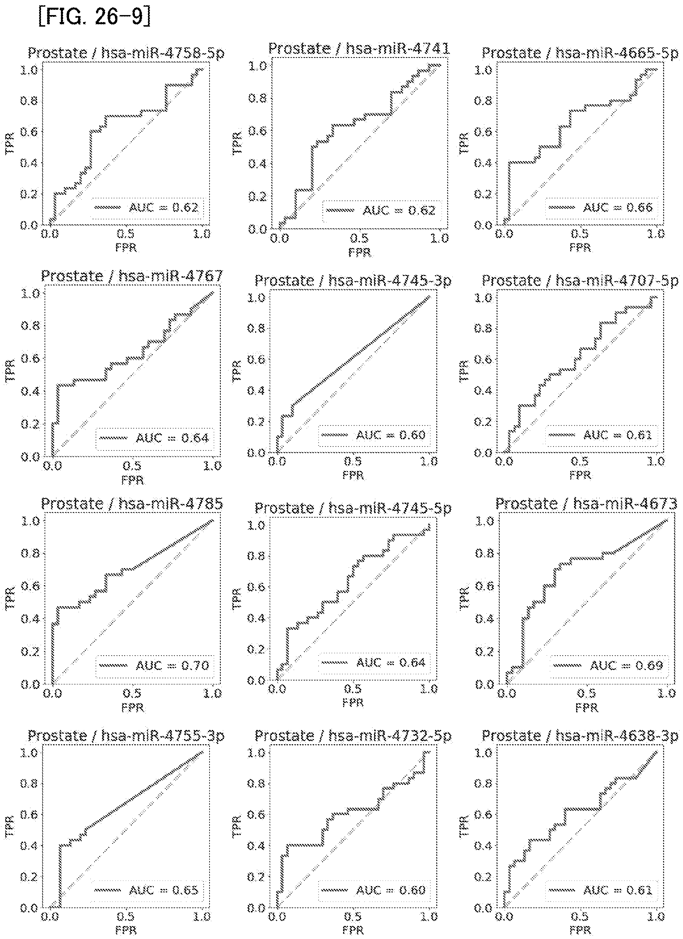

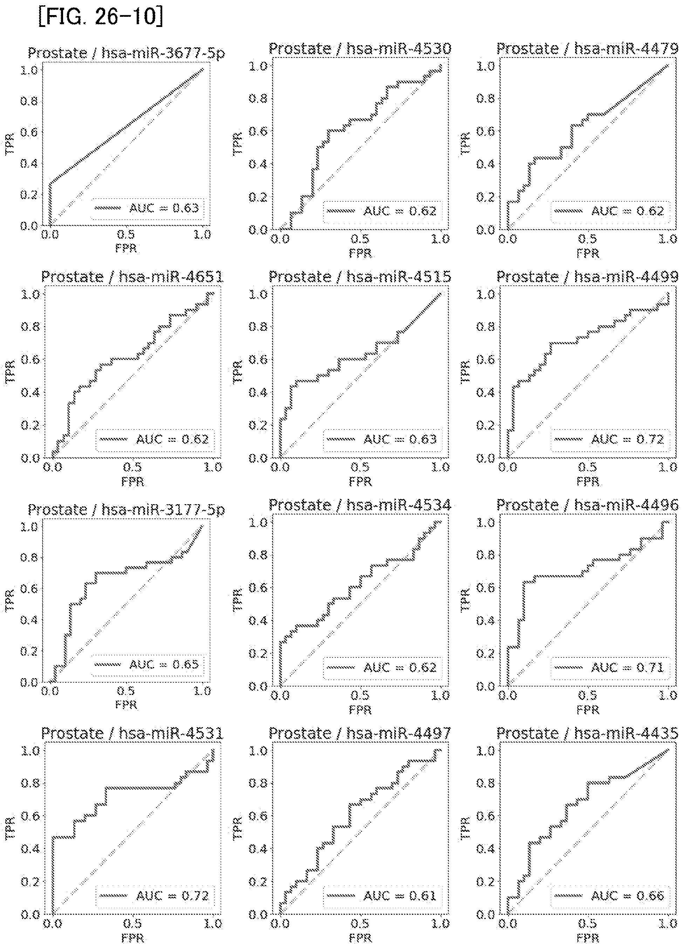

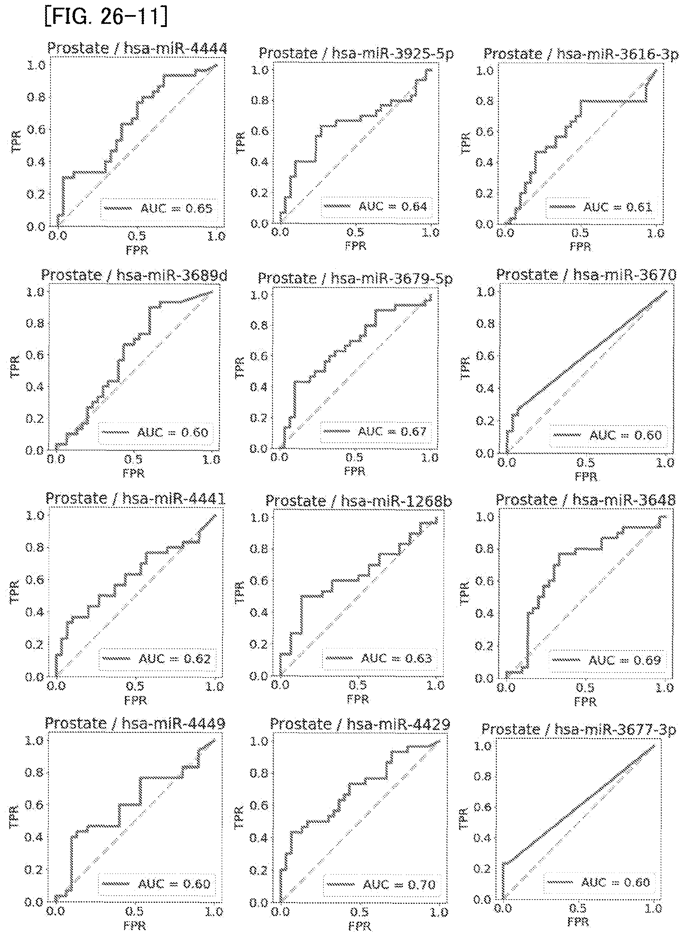

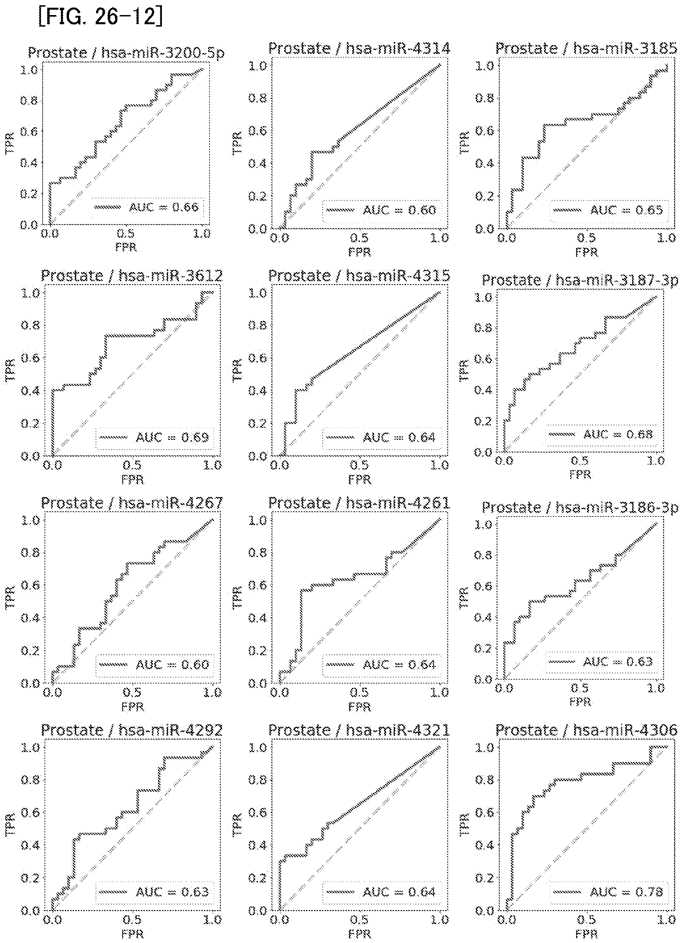

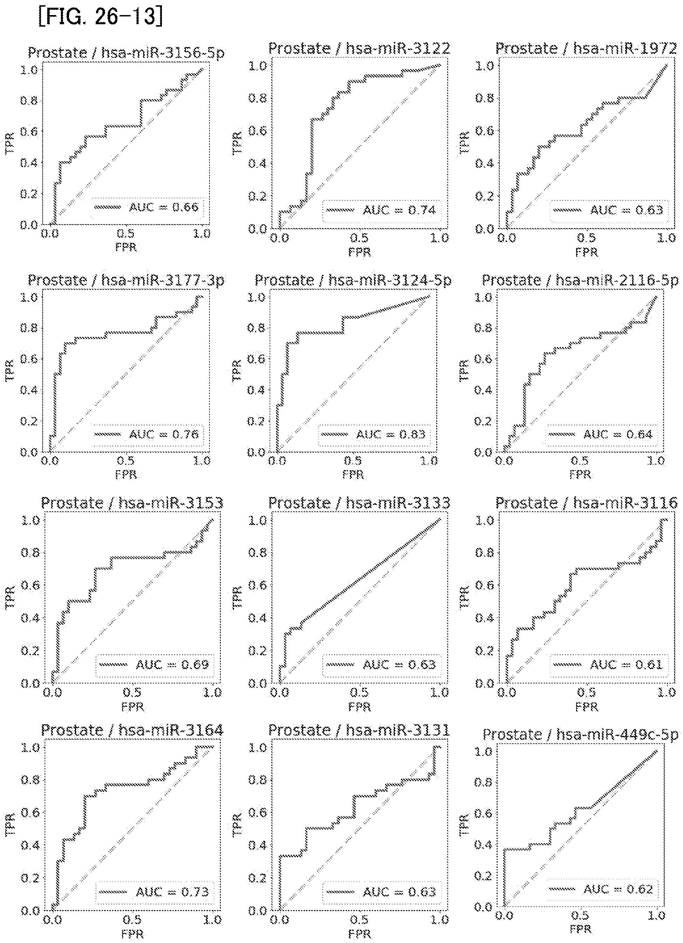

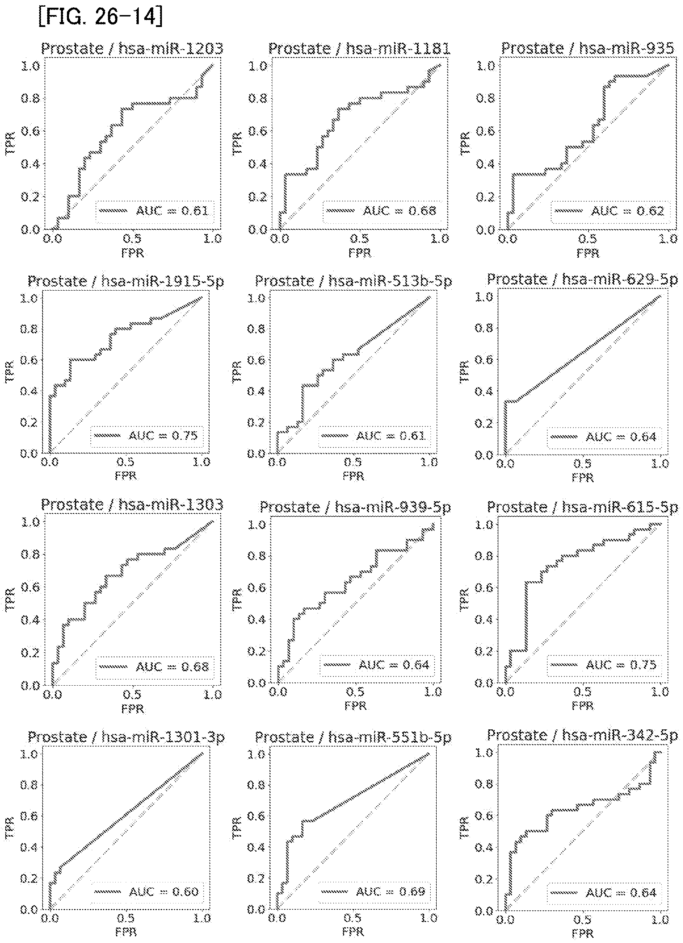

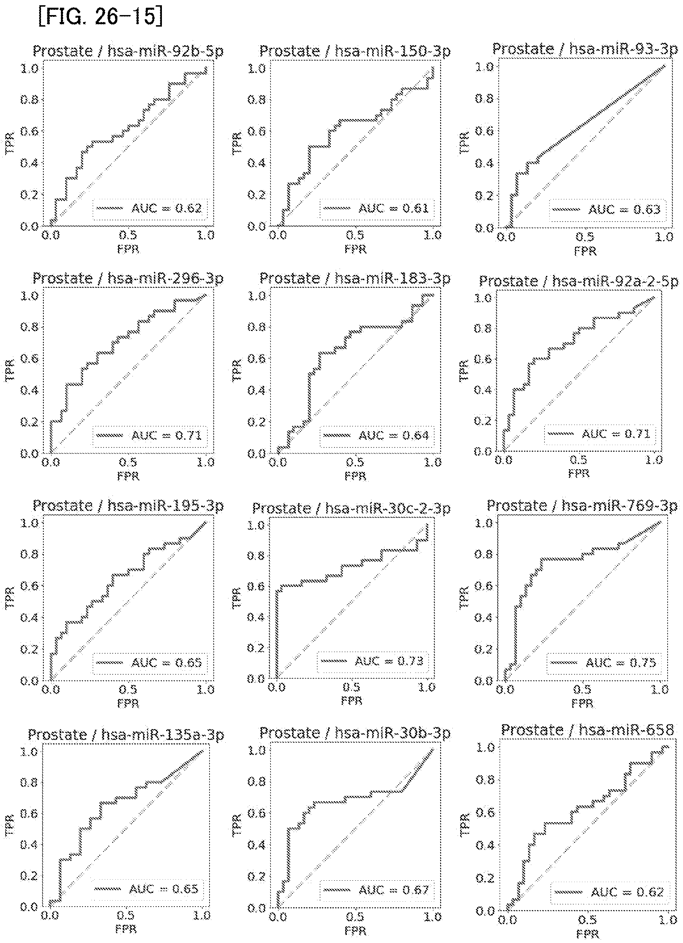

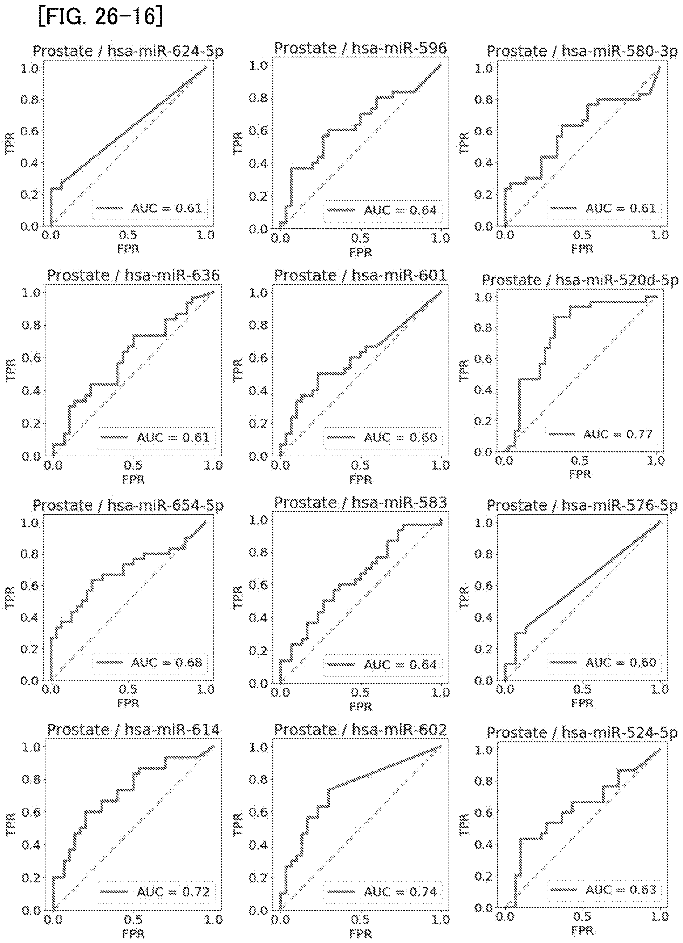

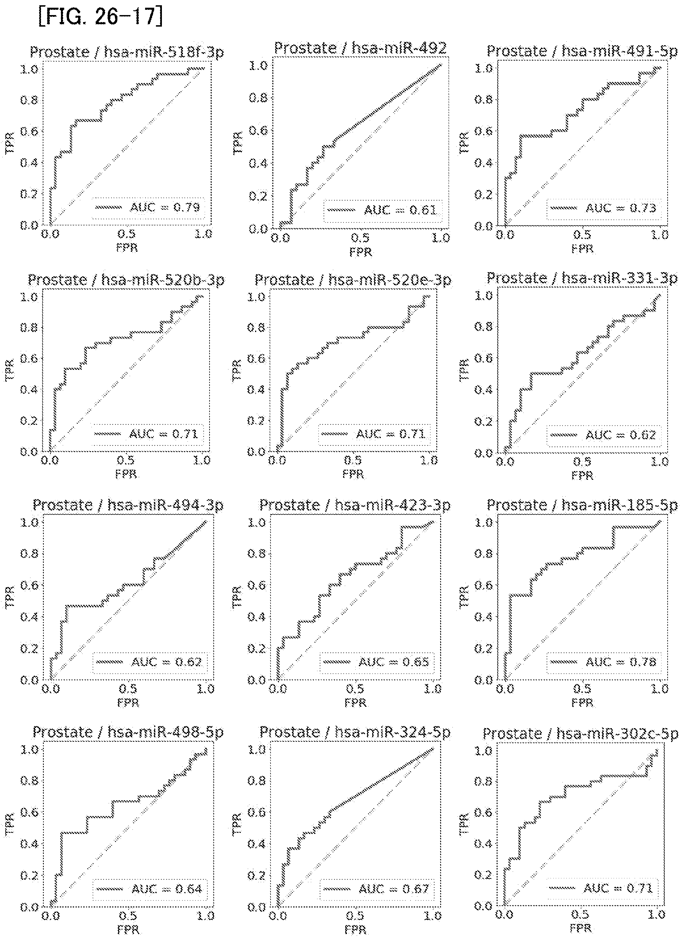

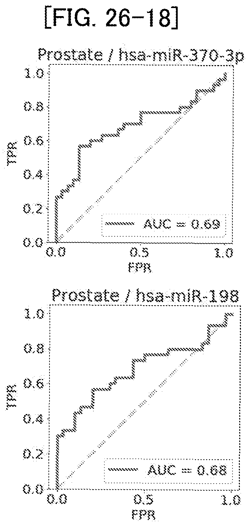

FIGS. 26-1 to 26-18 show ROC-curves for predicting prostatic cancer in humans using a single miRNA.

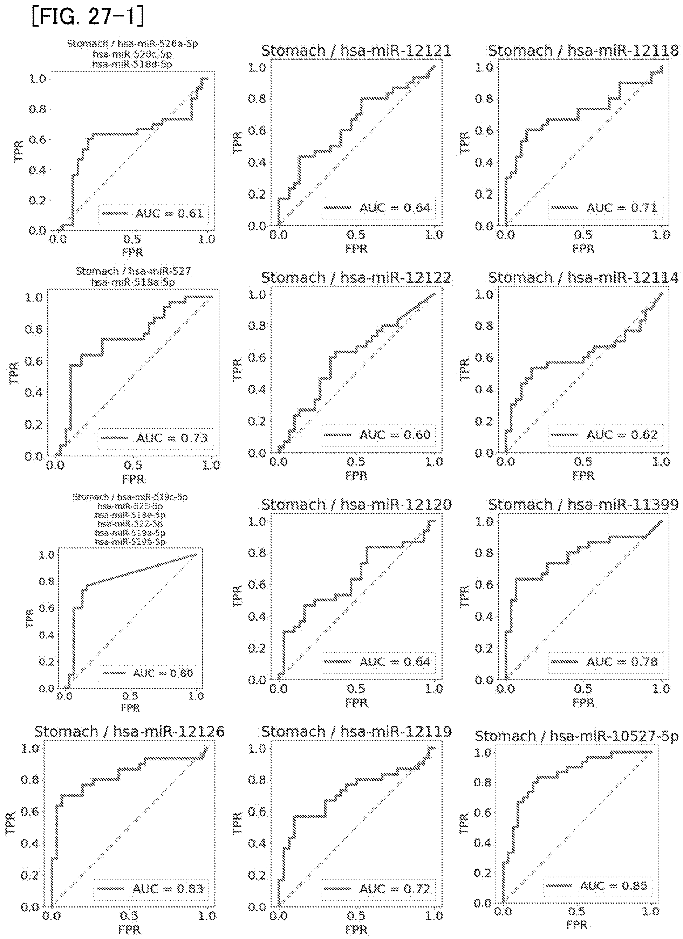

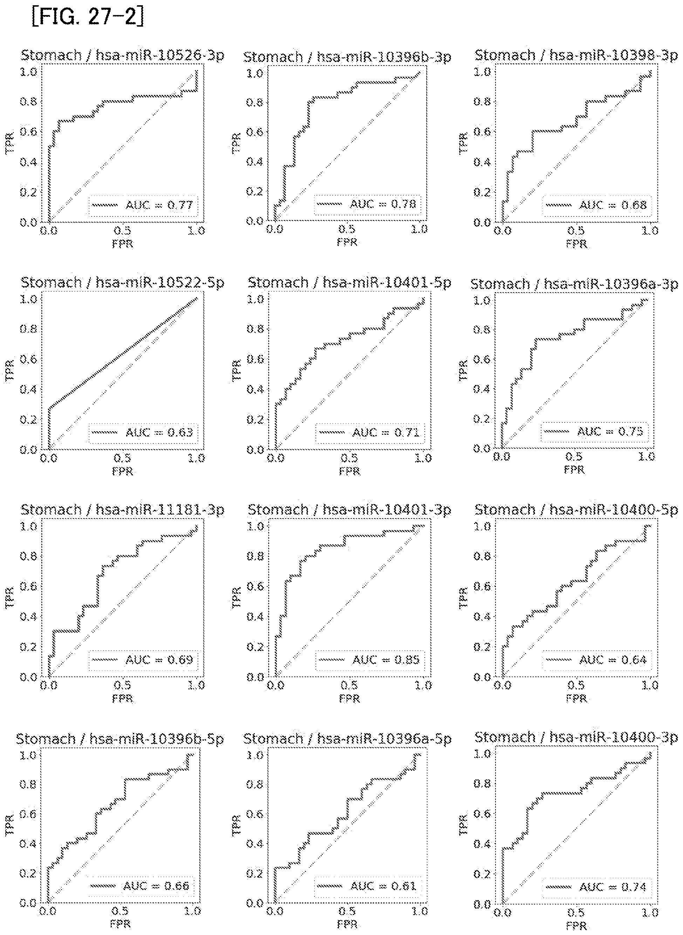

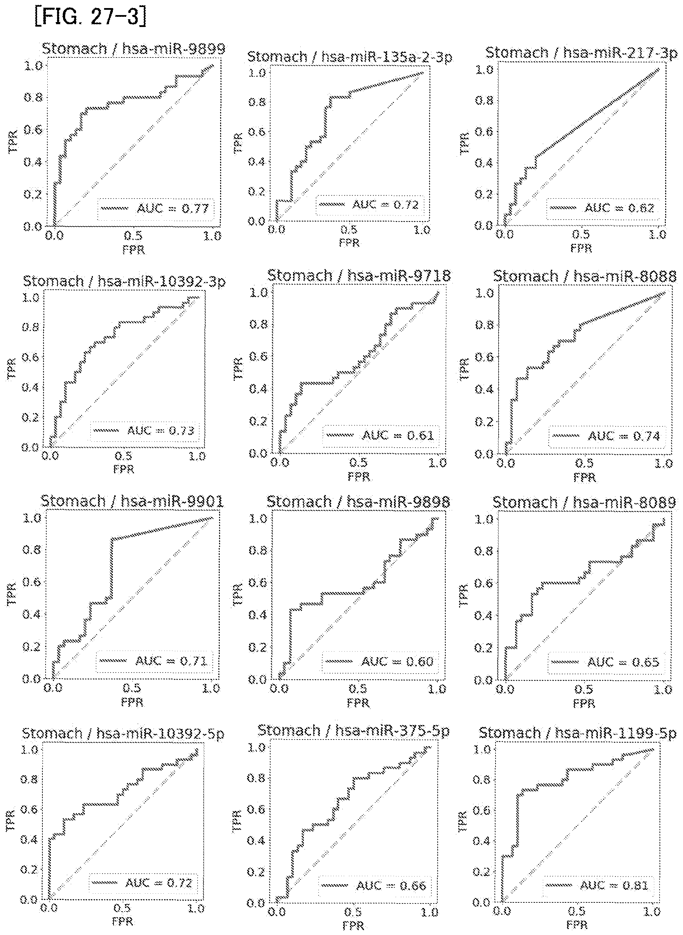

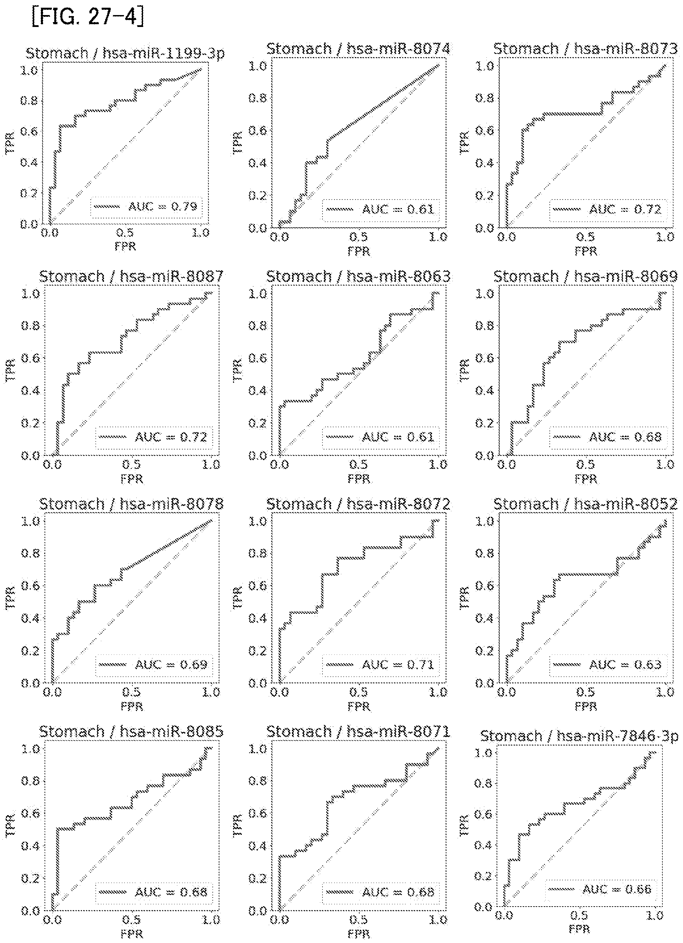

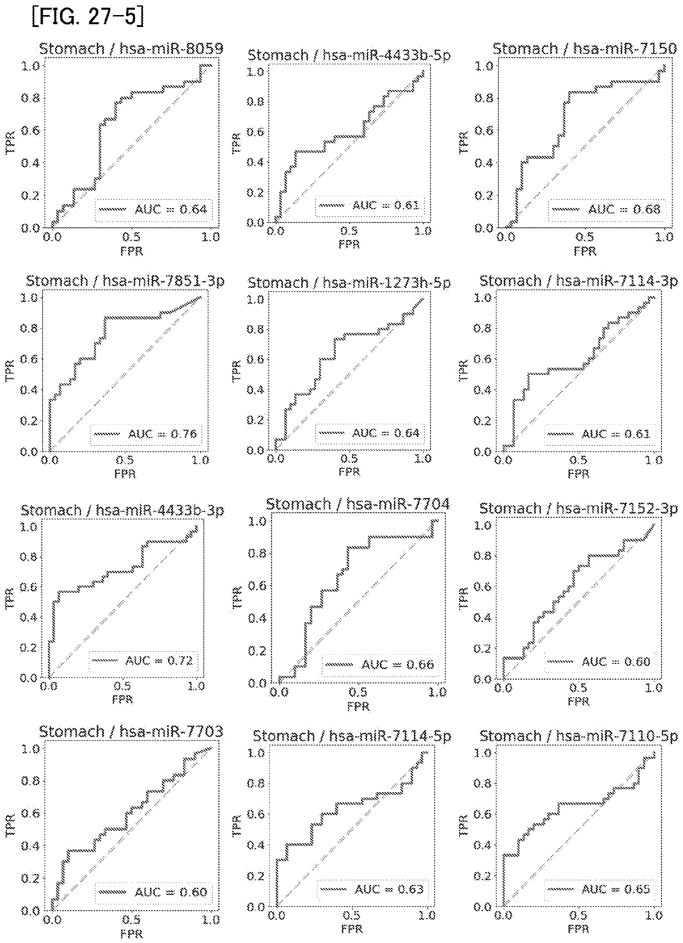

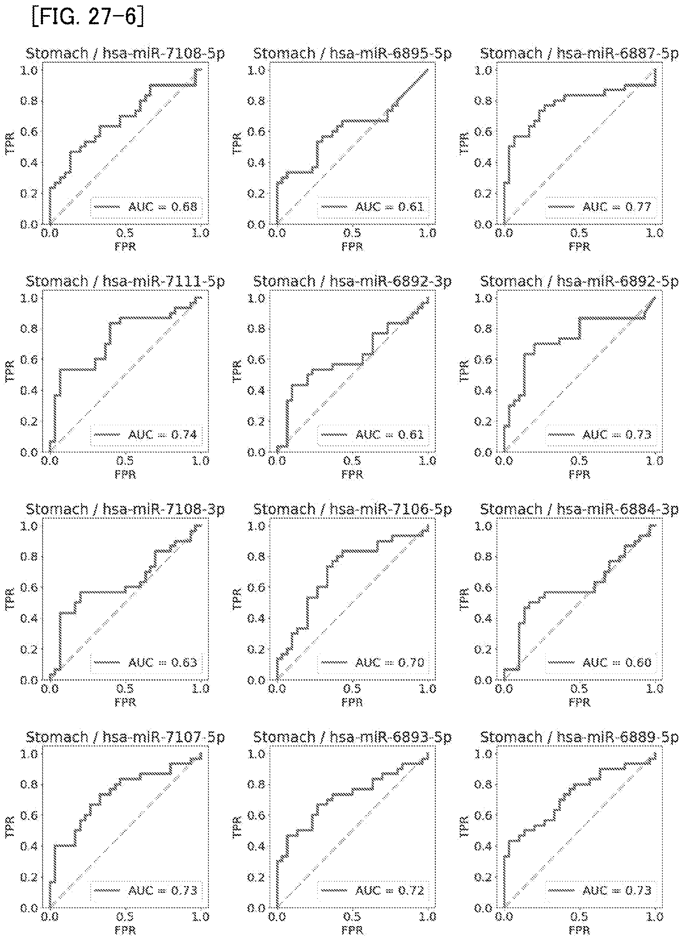

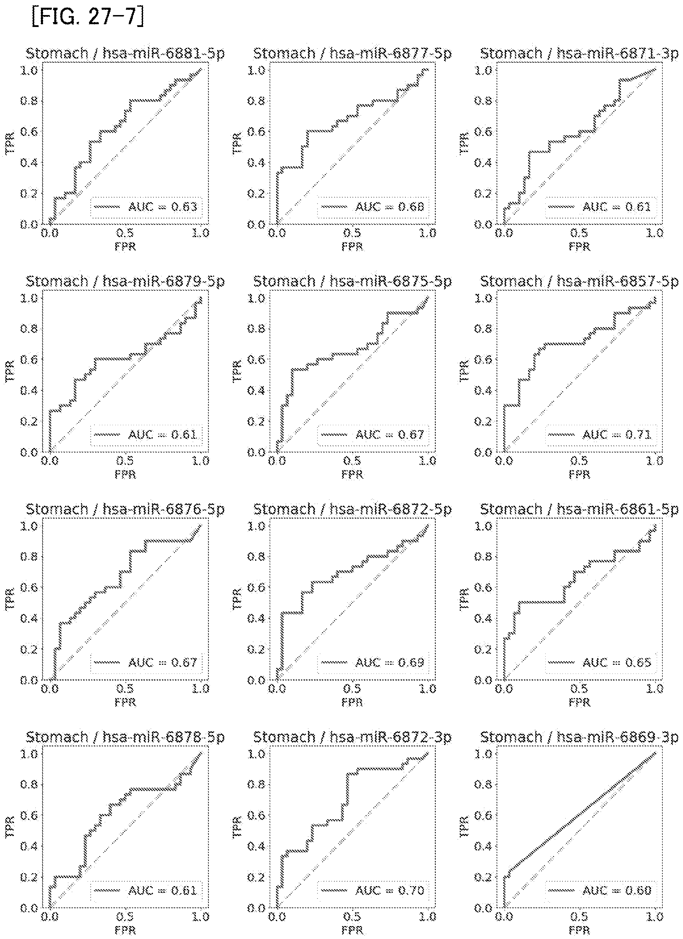

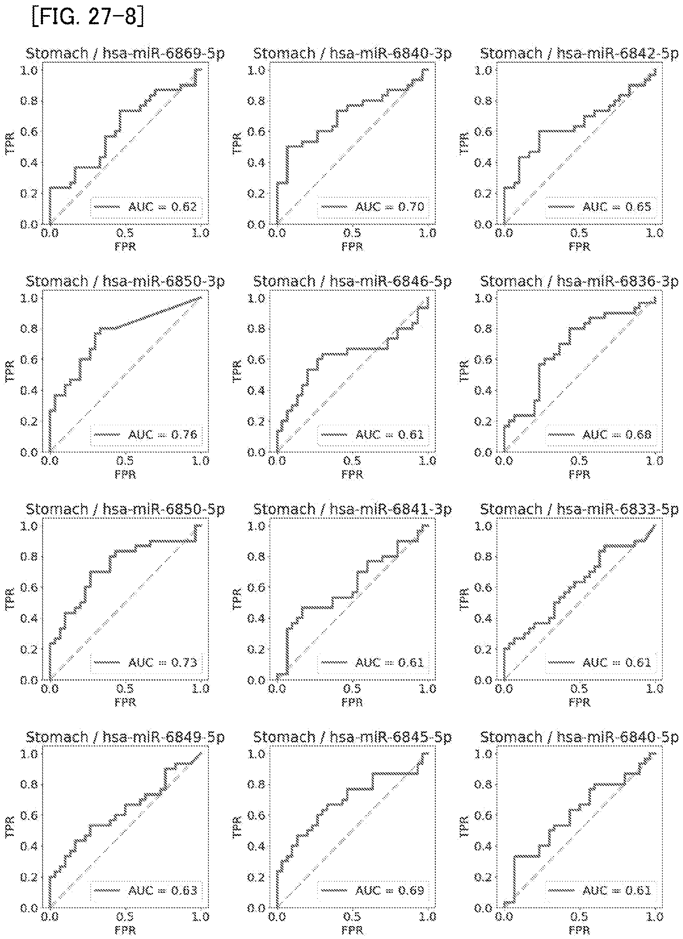

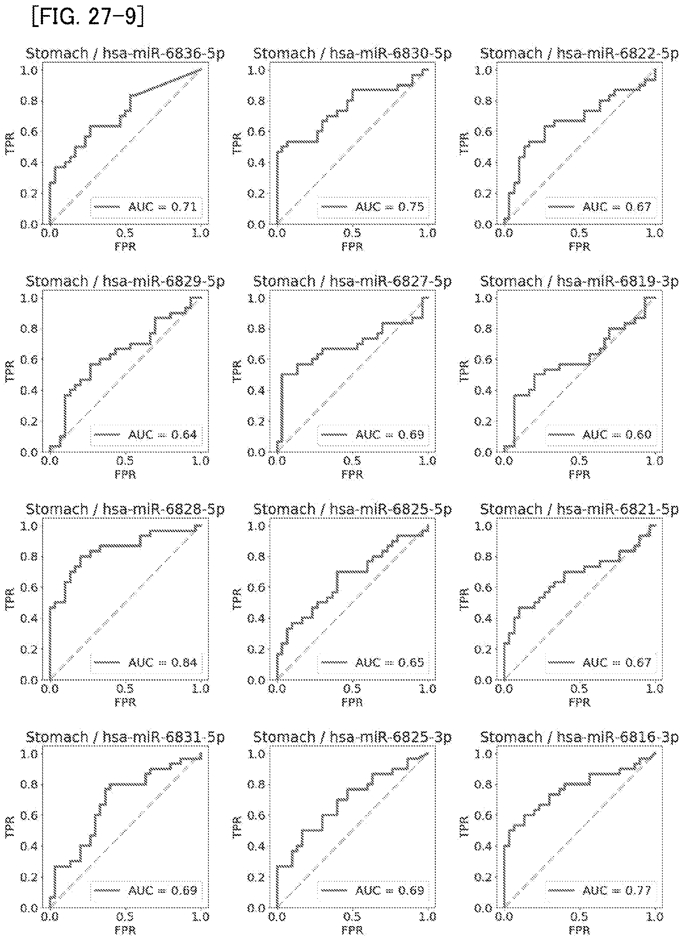

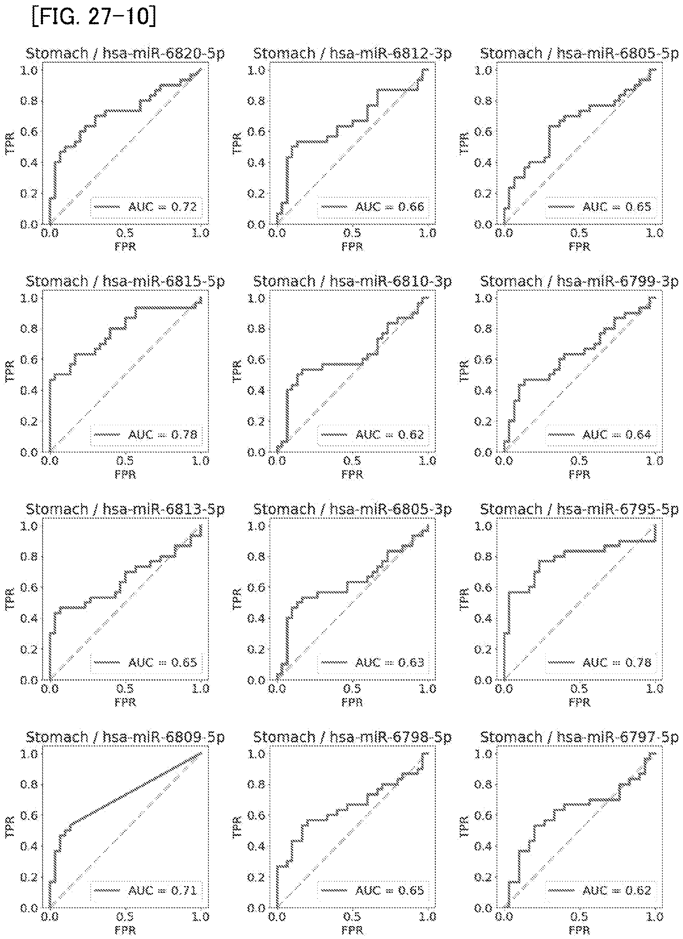

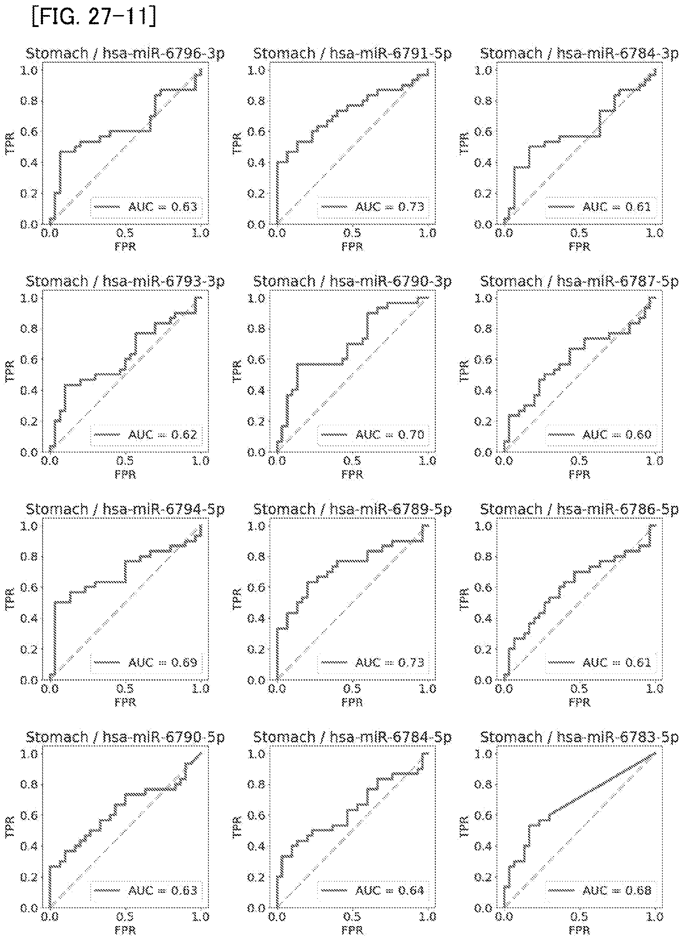

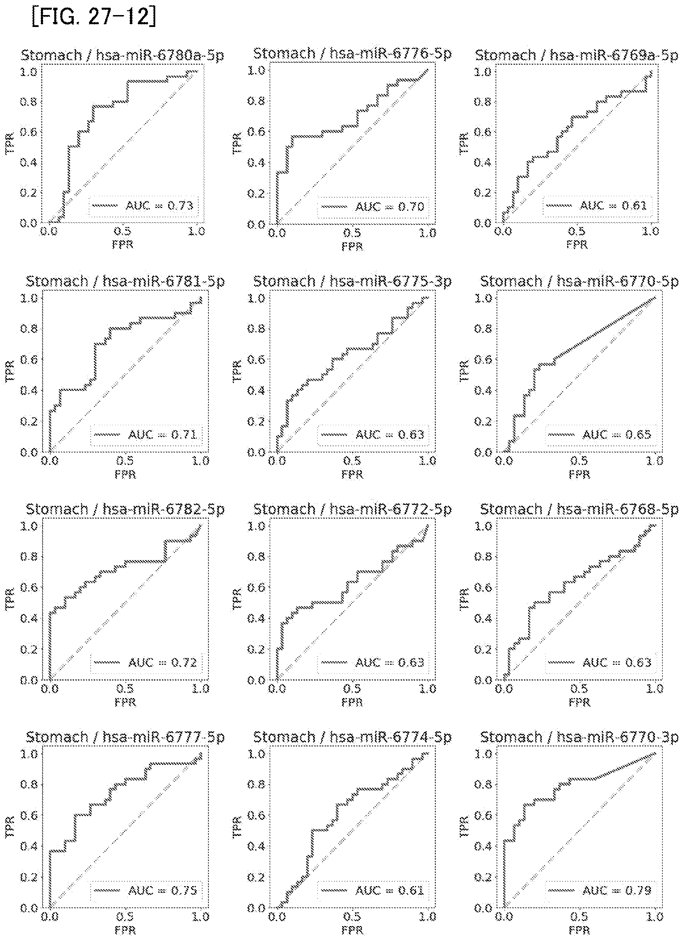

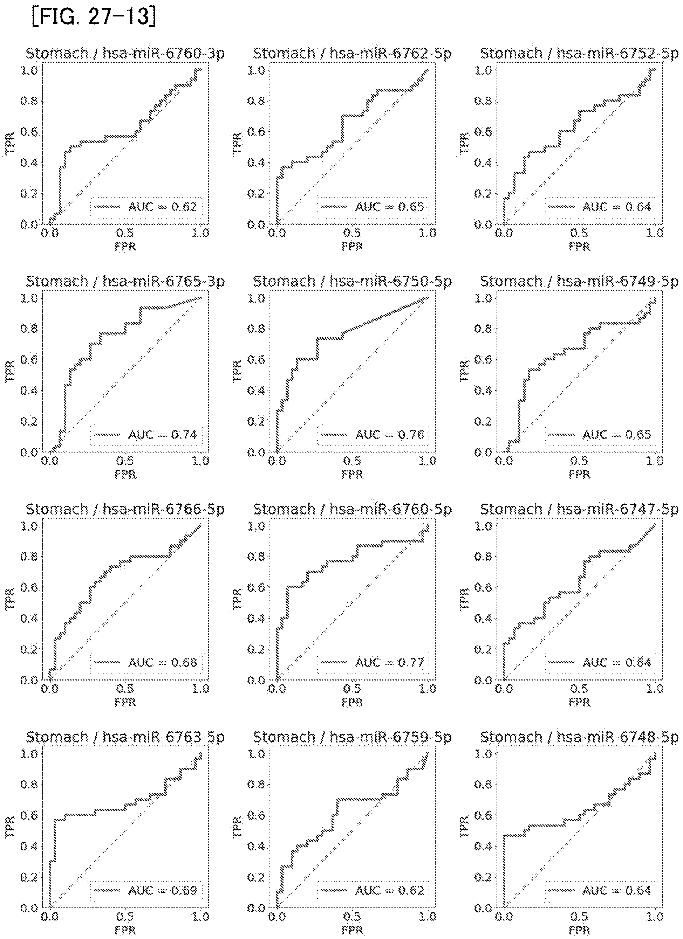

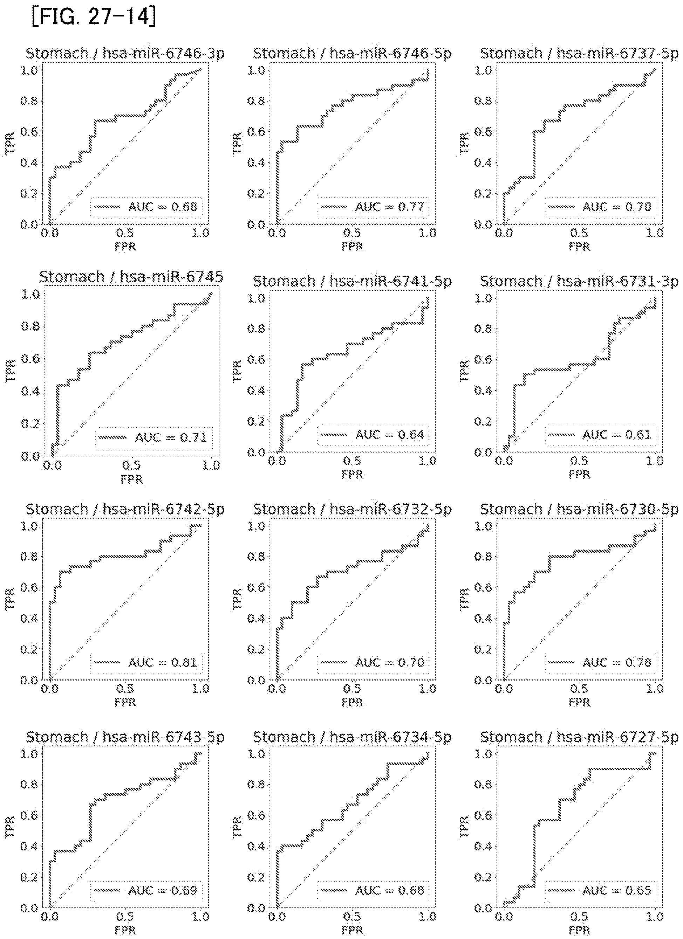

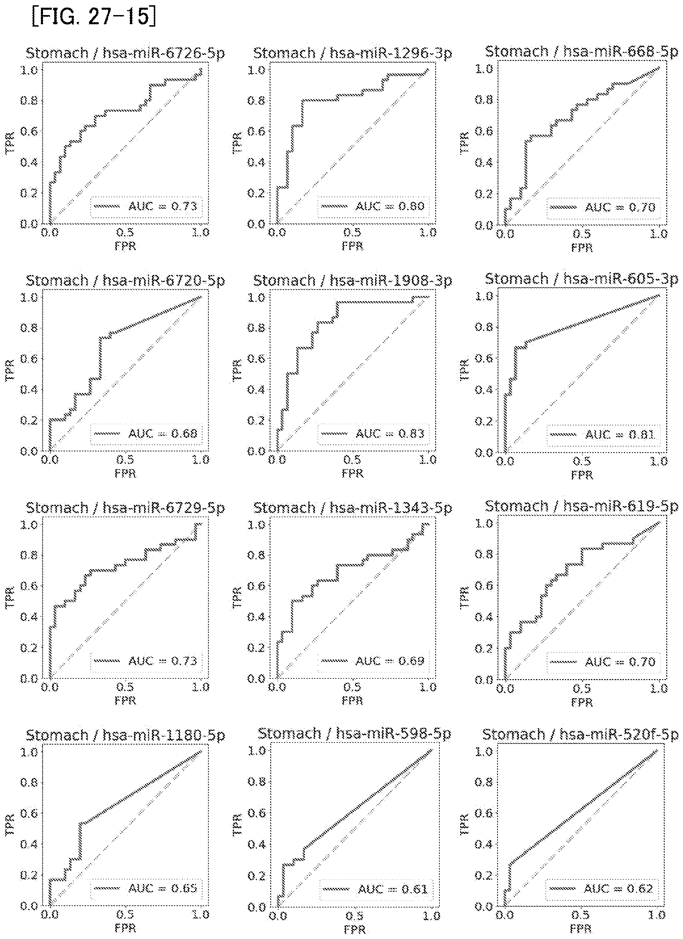

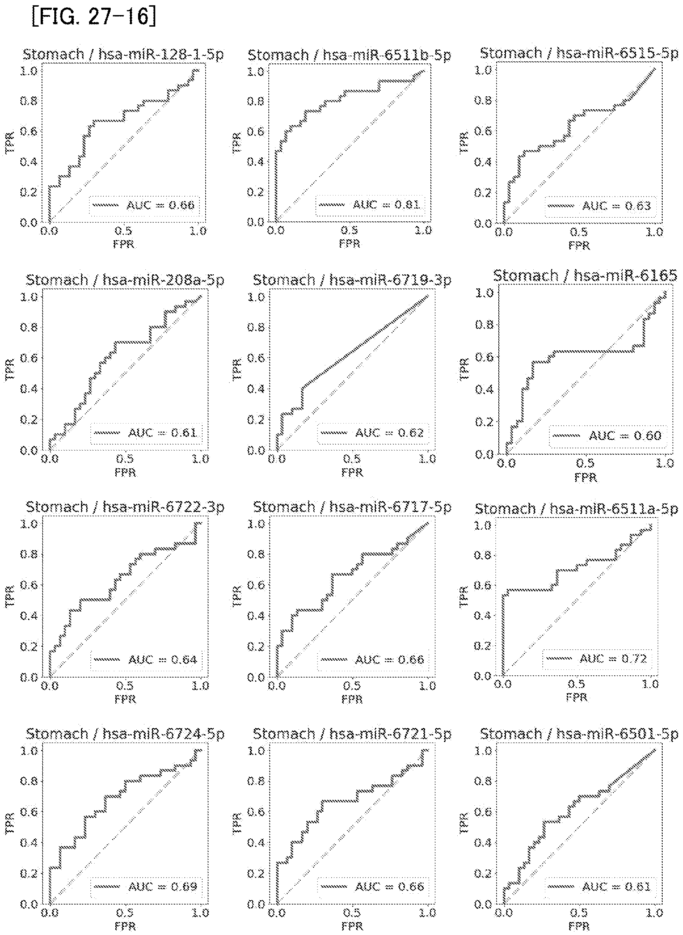

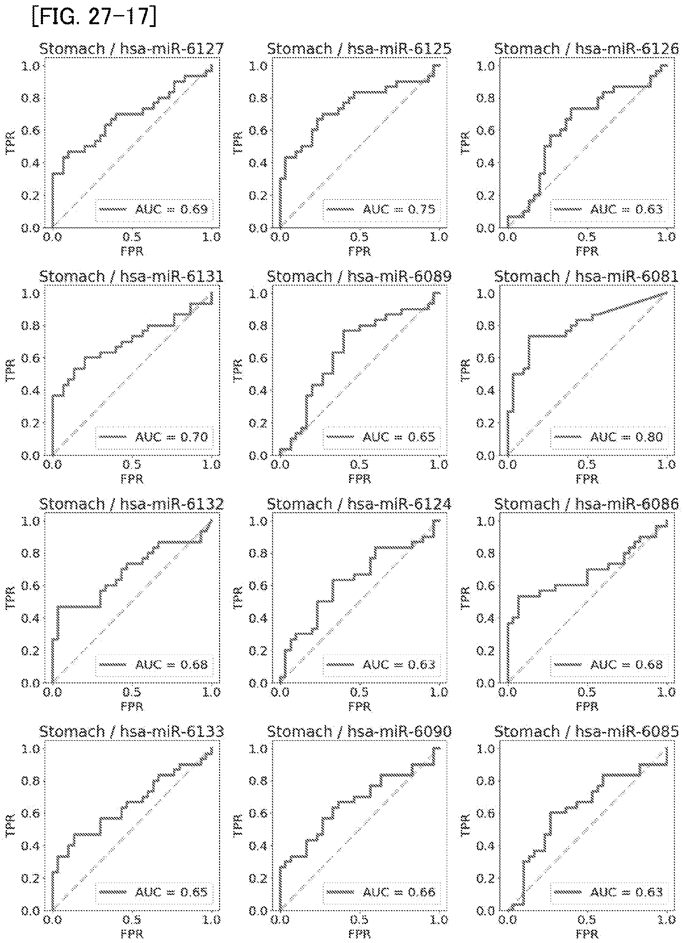

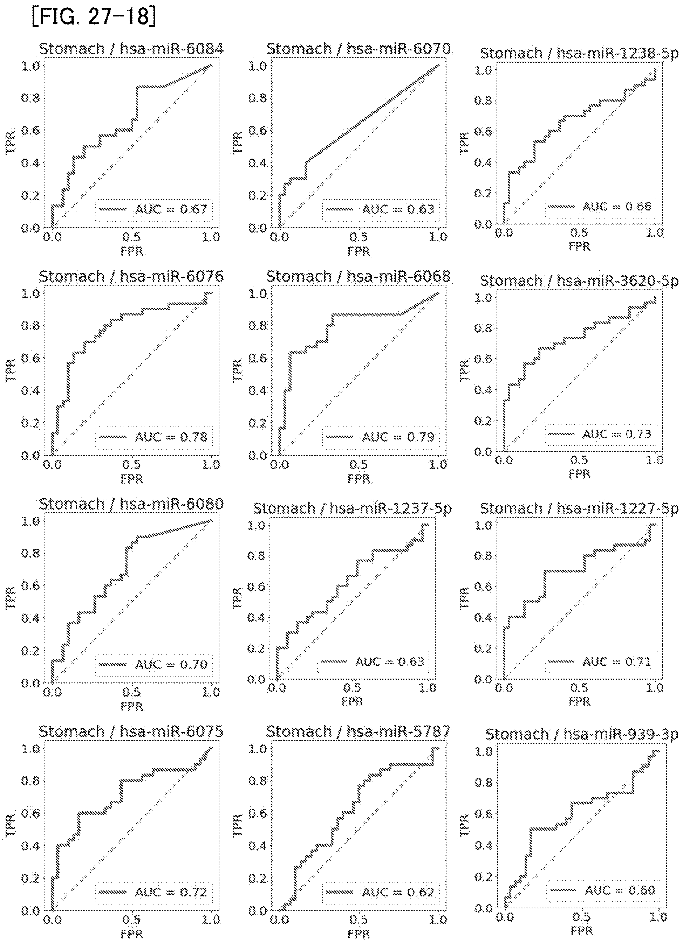

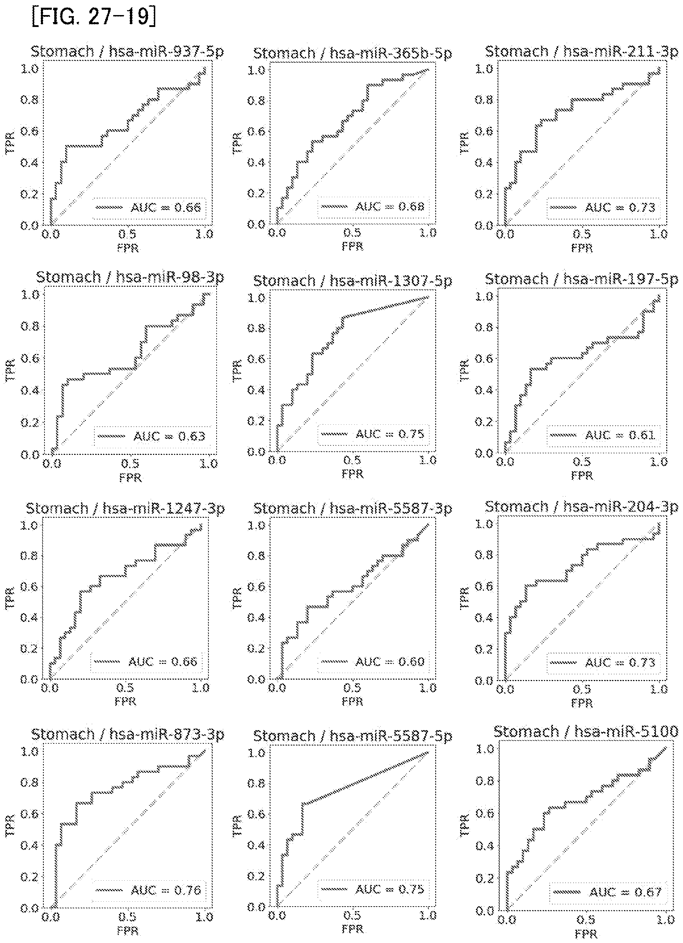

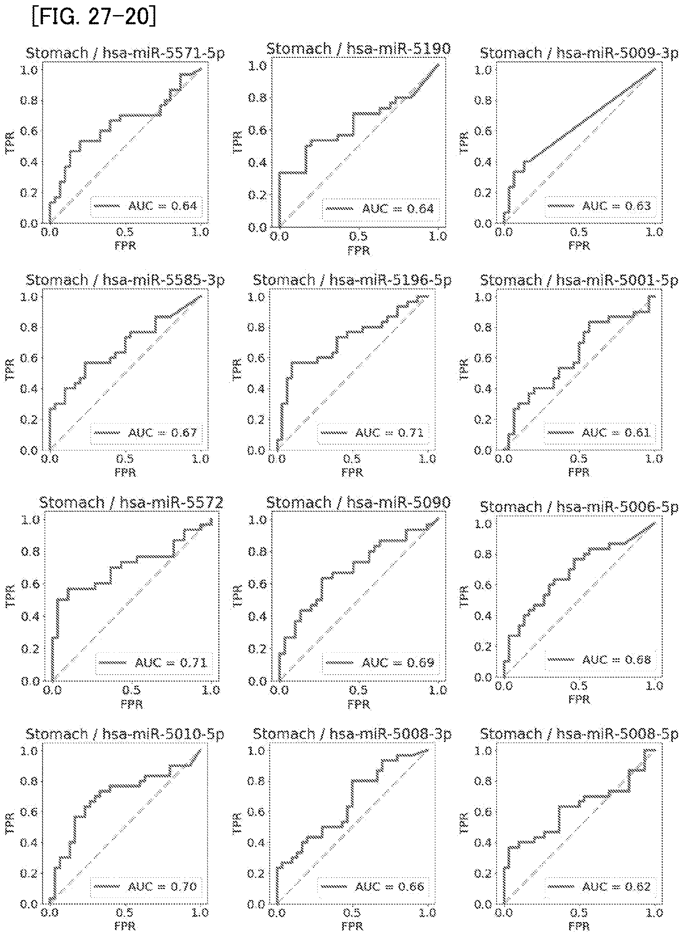

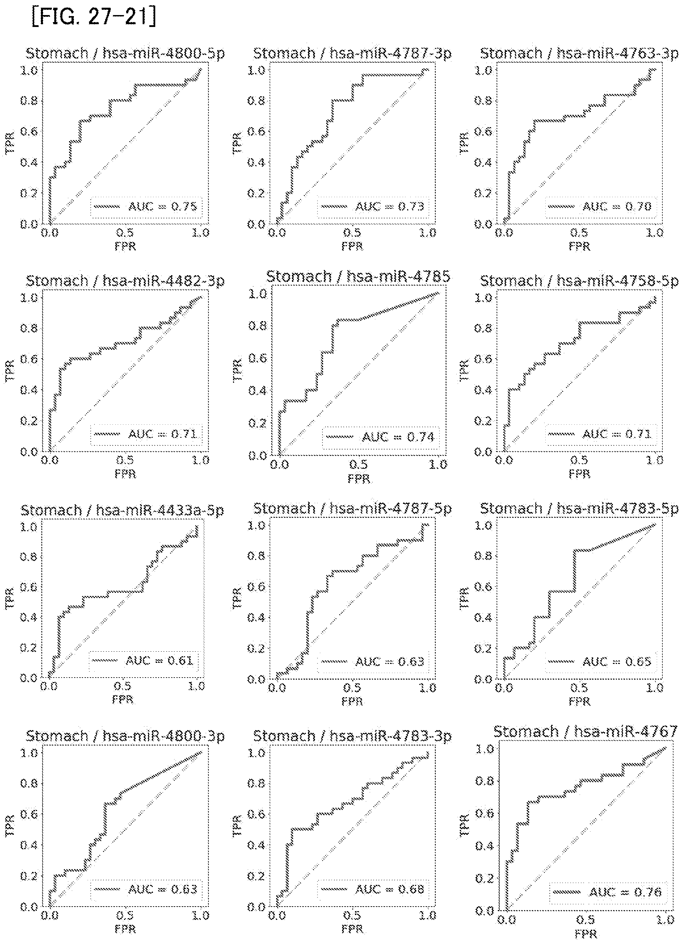

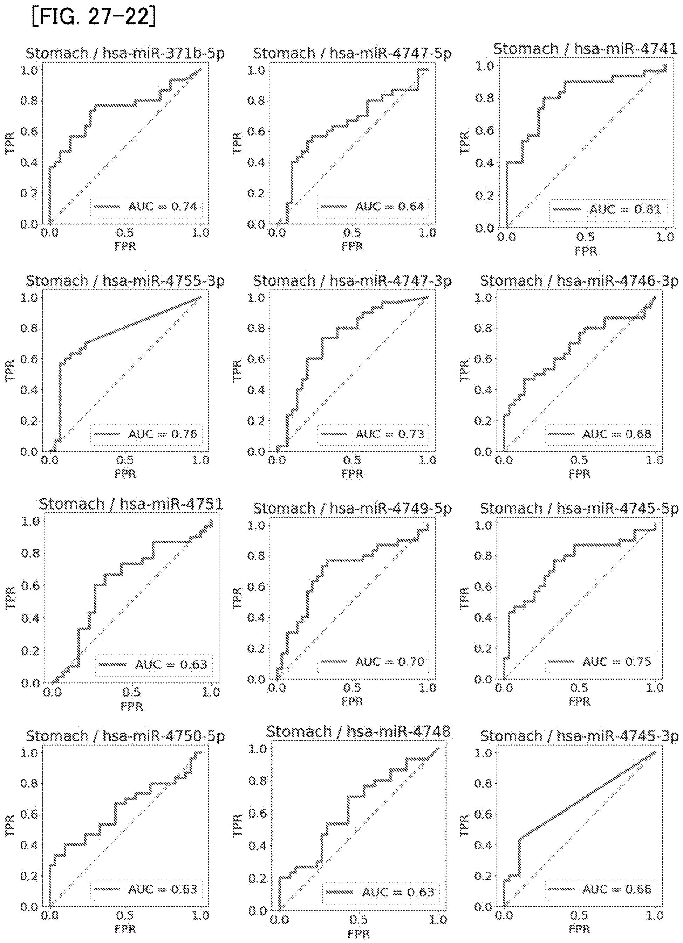

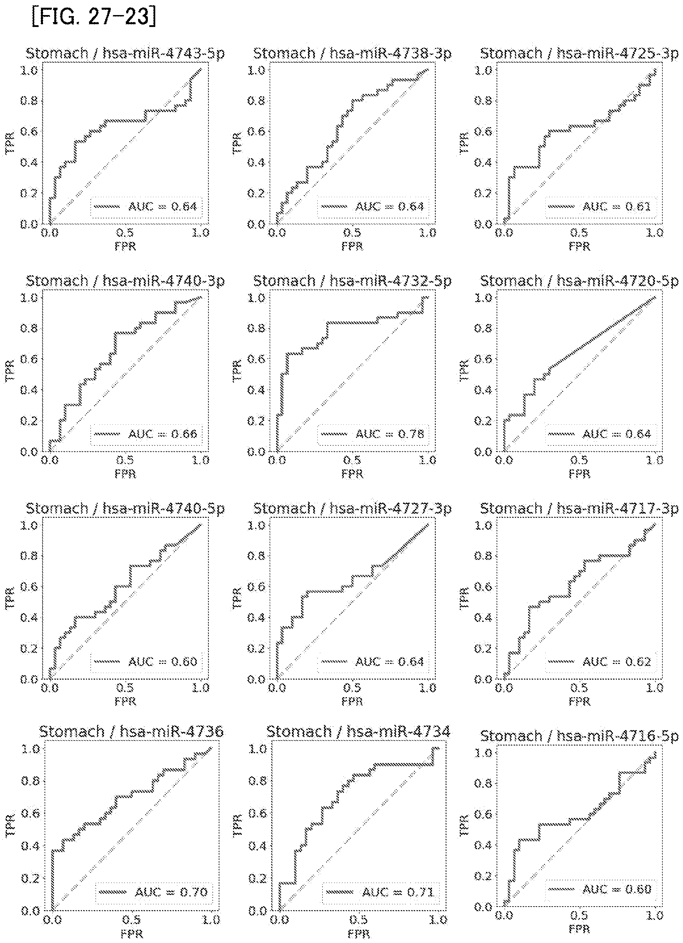

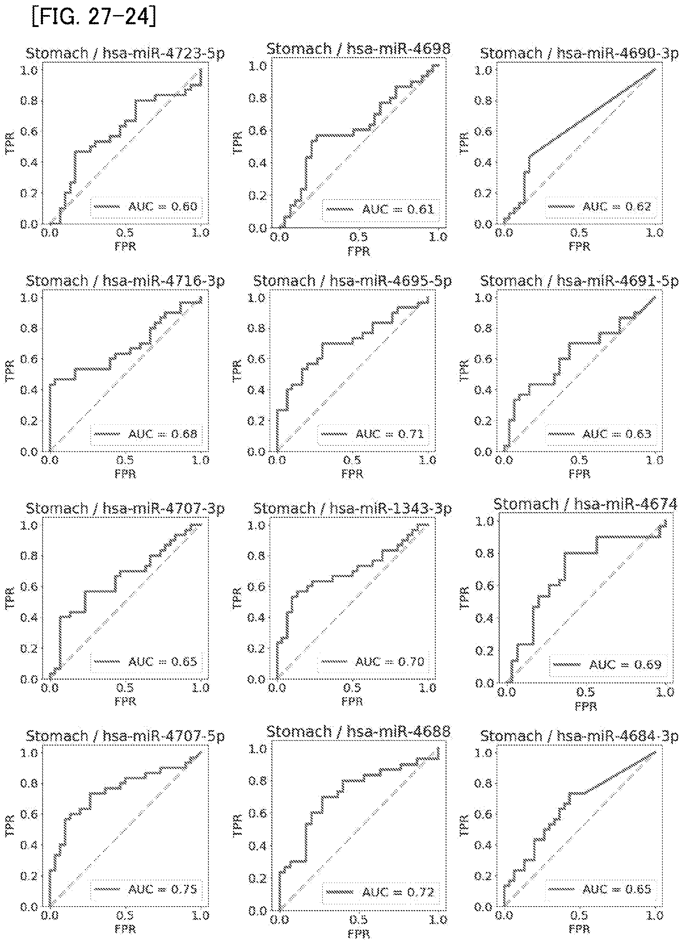

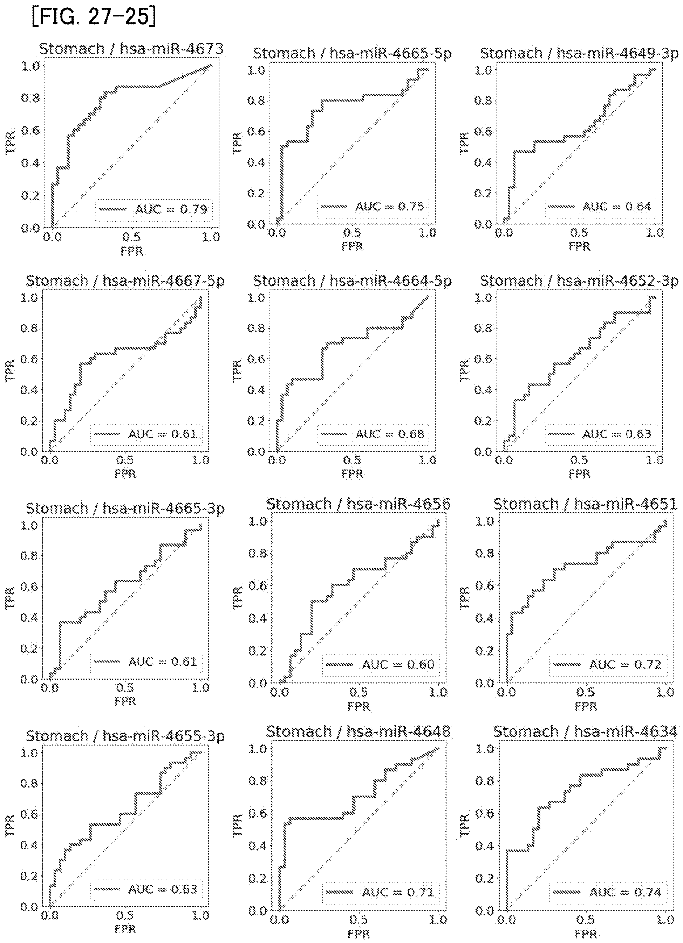

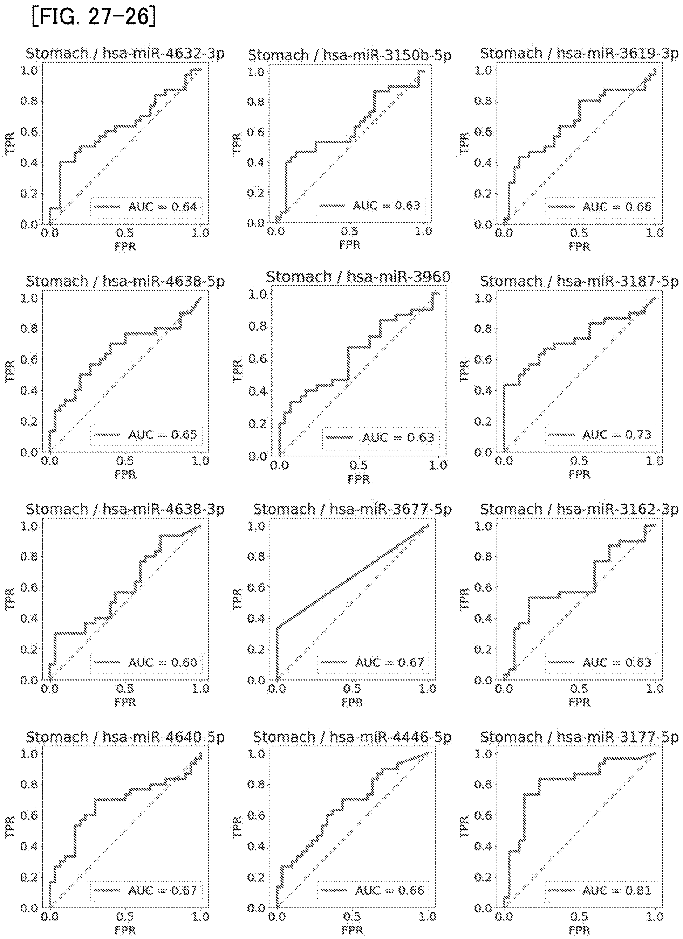

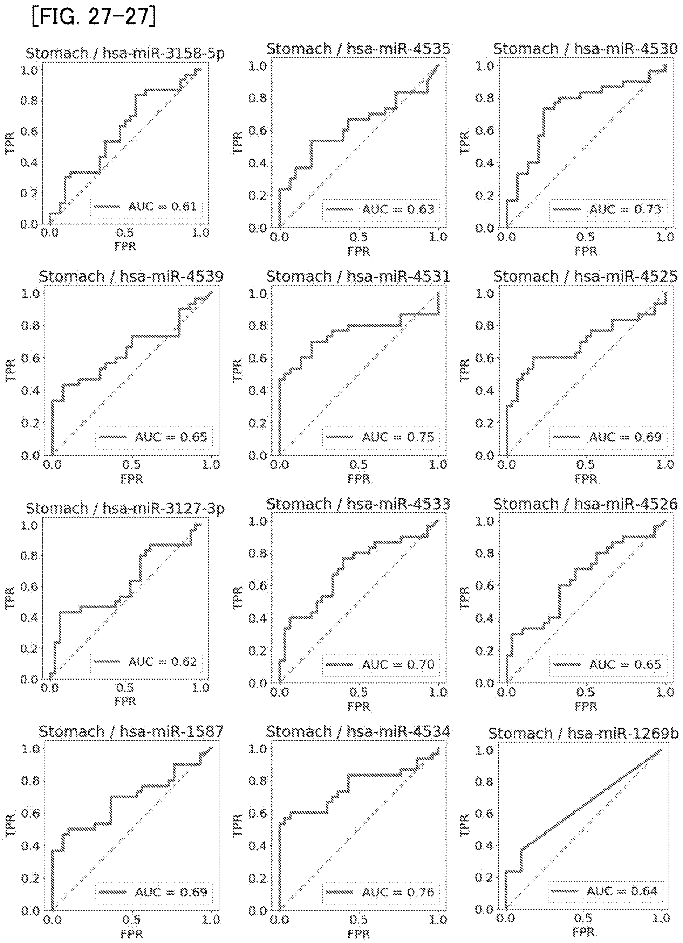

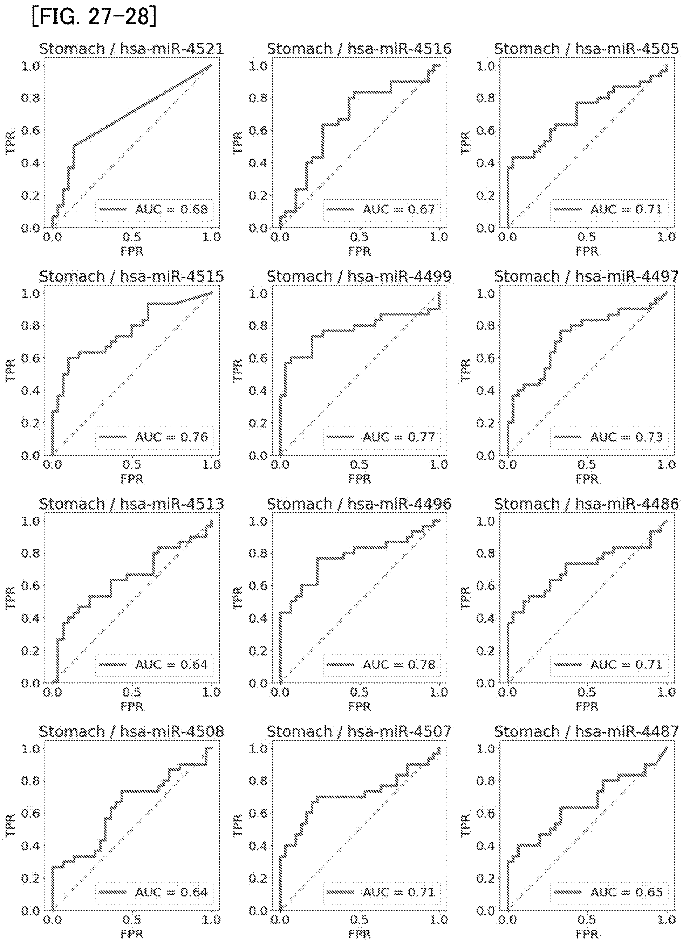

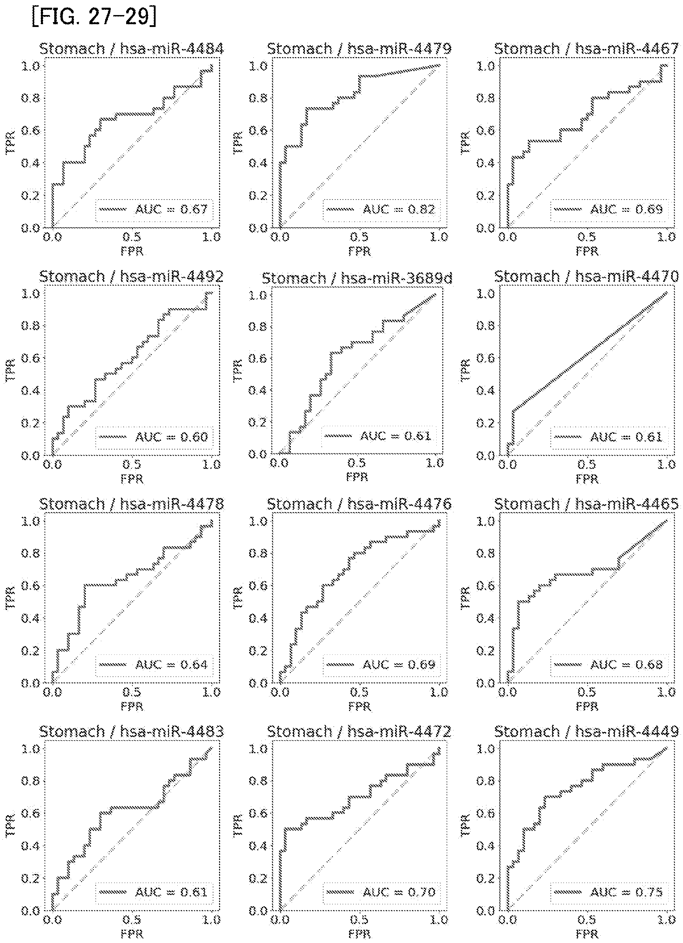

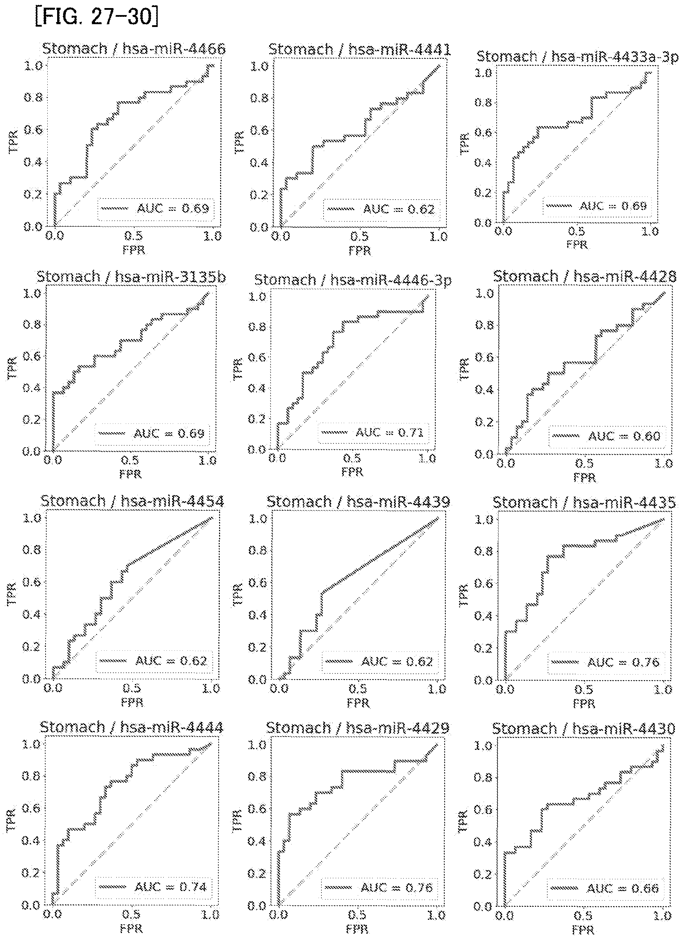

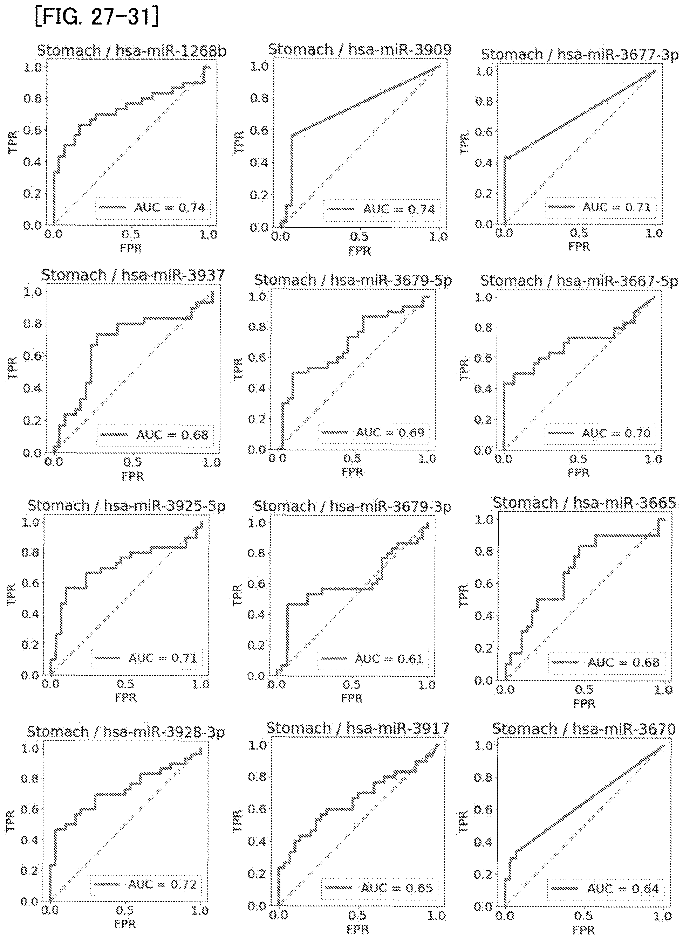

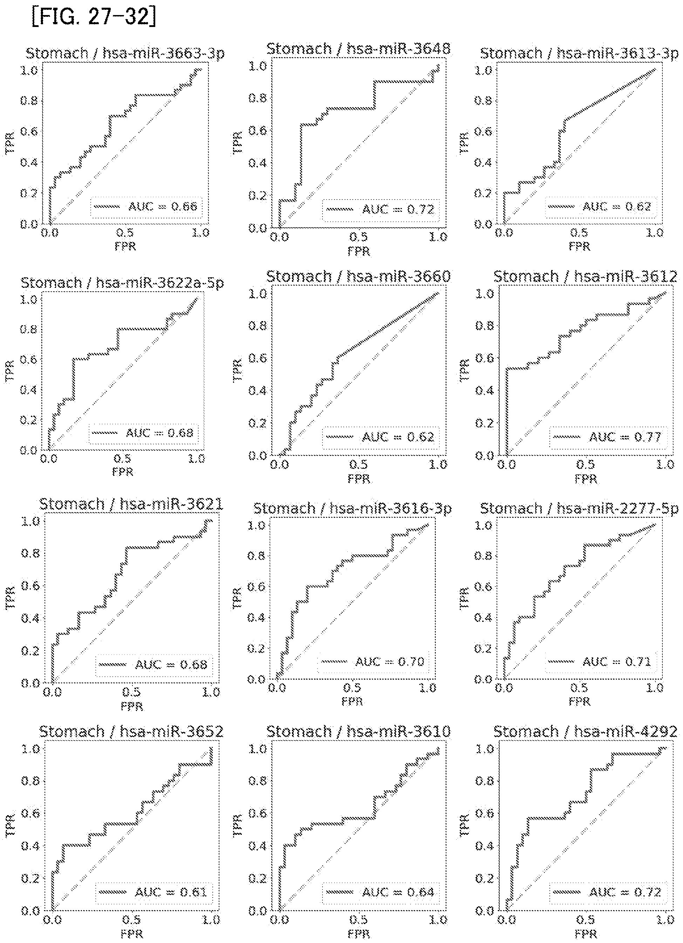

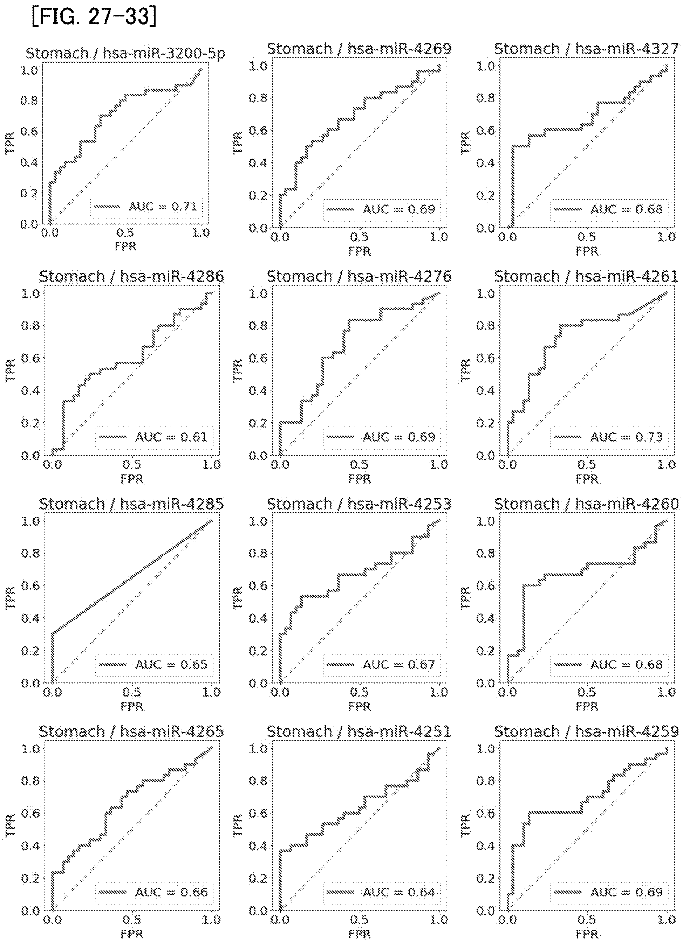

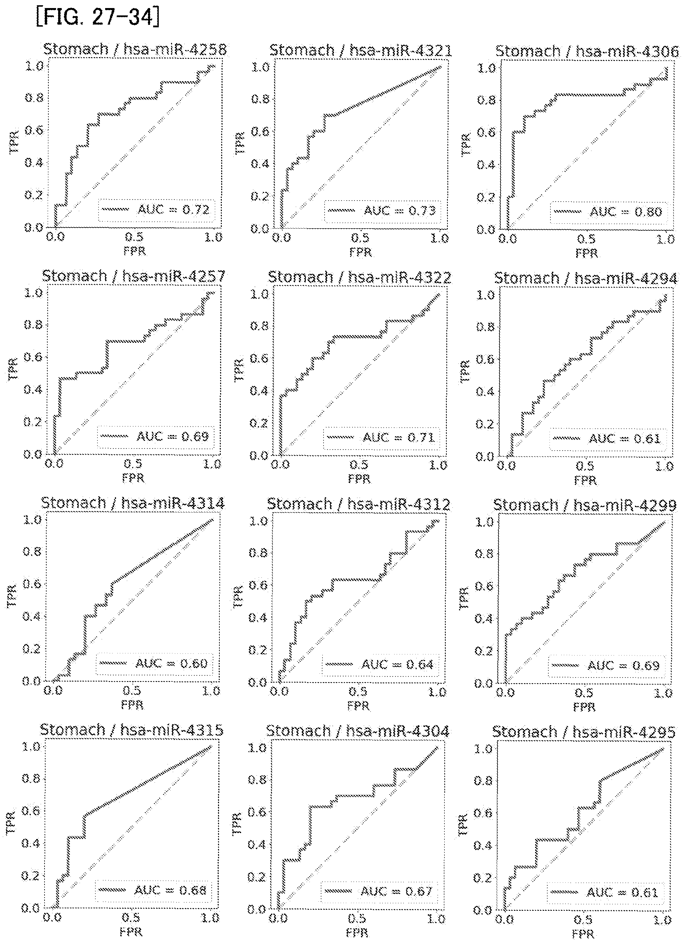

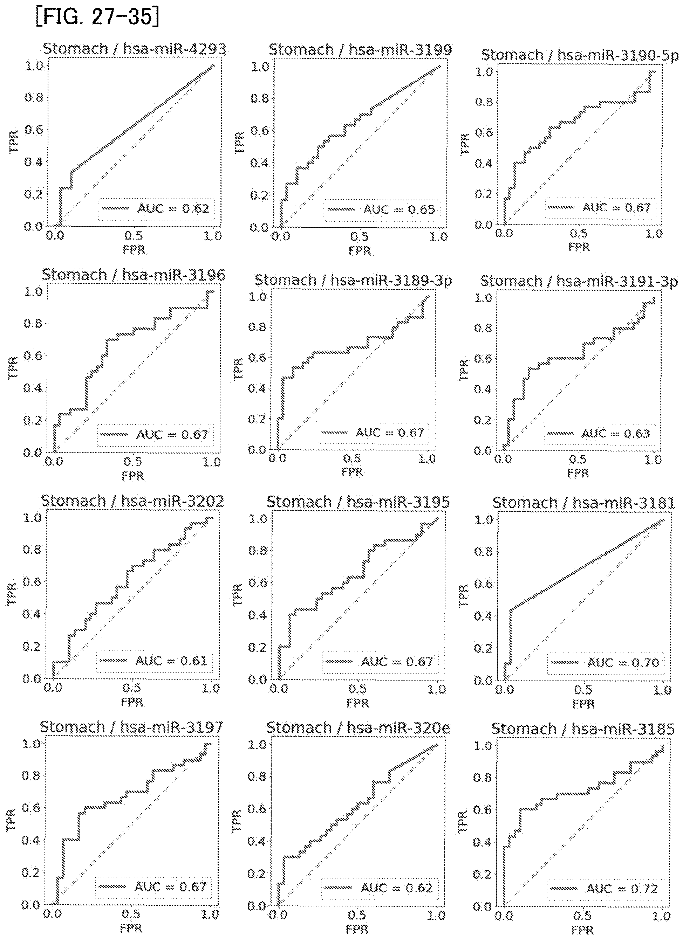

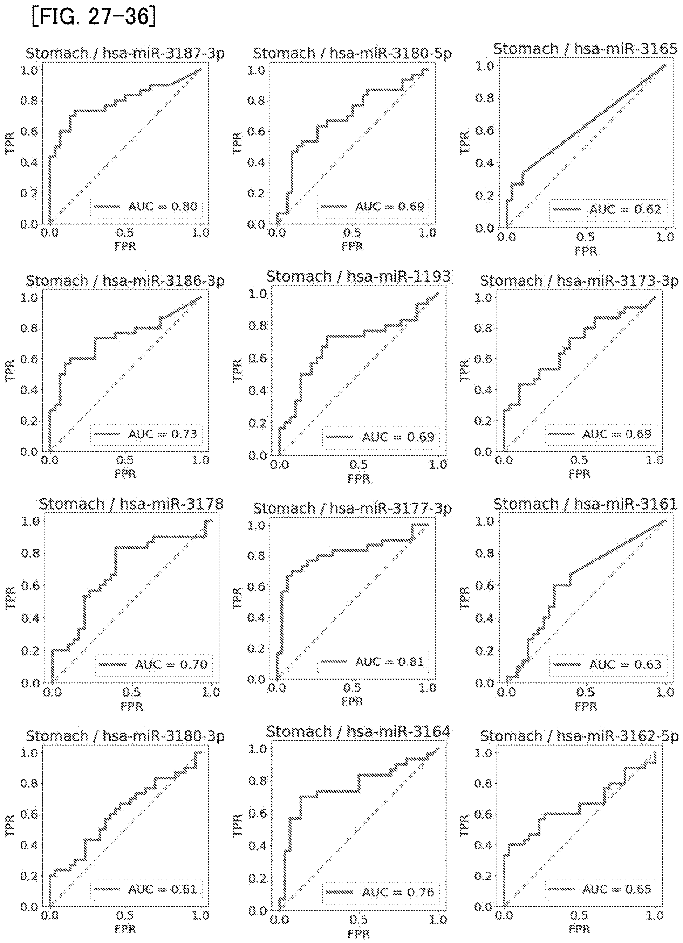

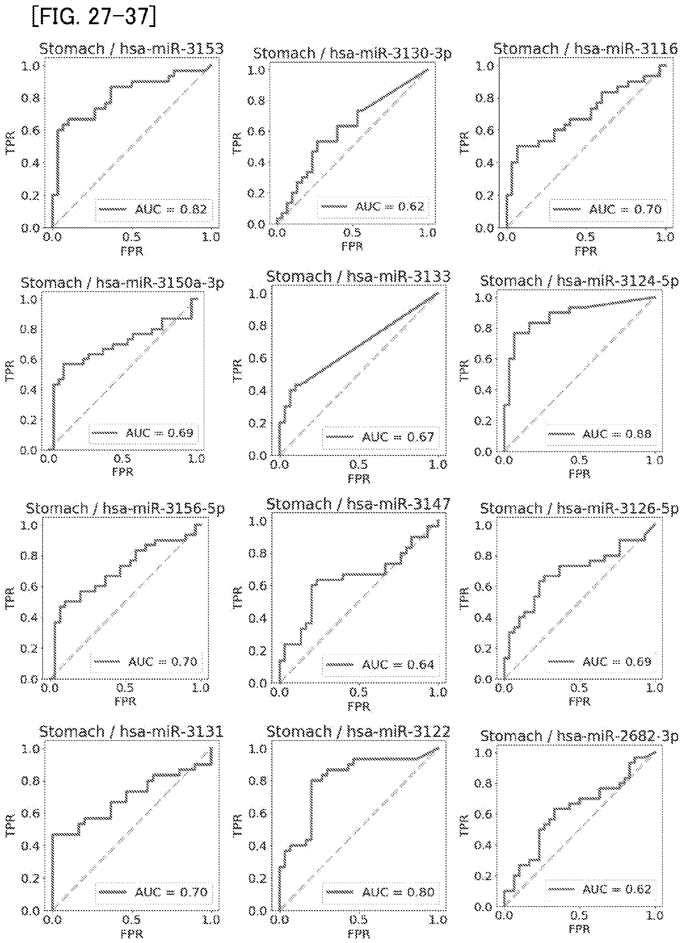

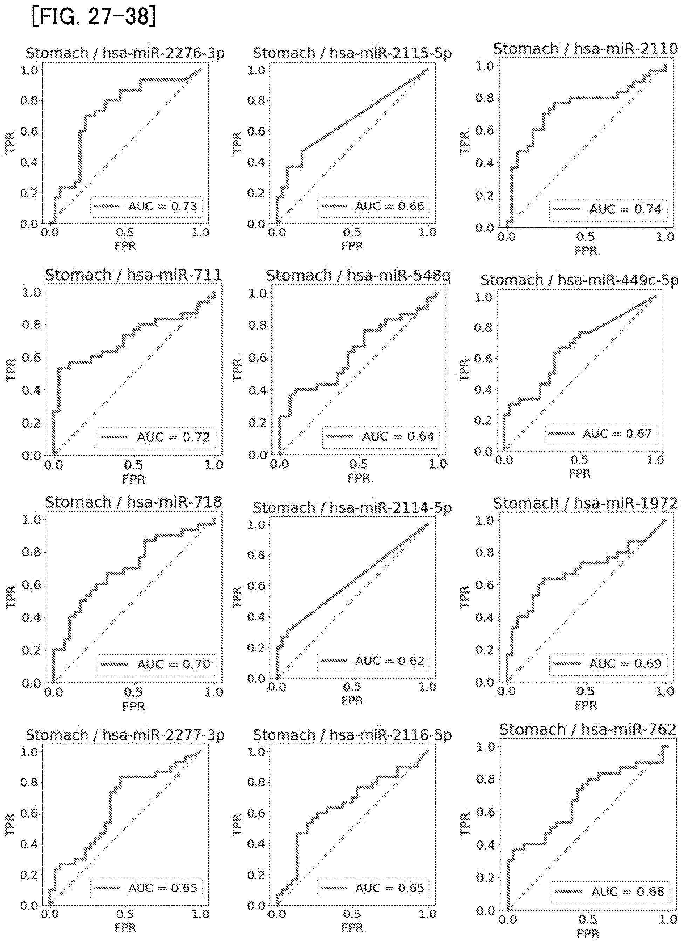

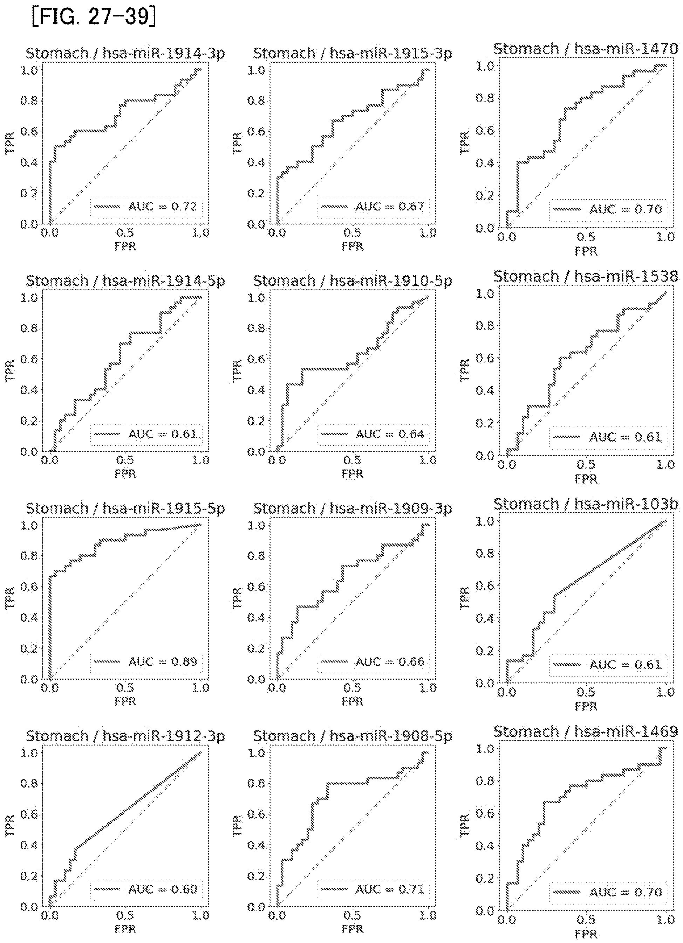

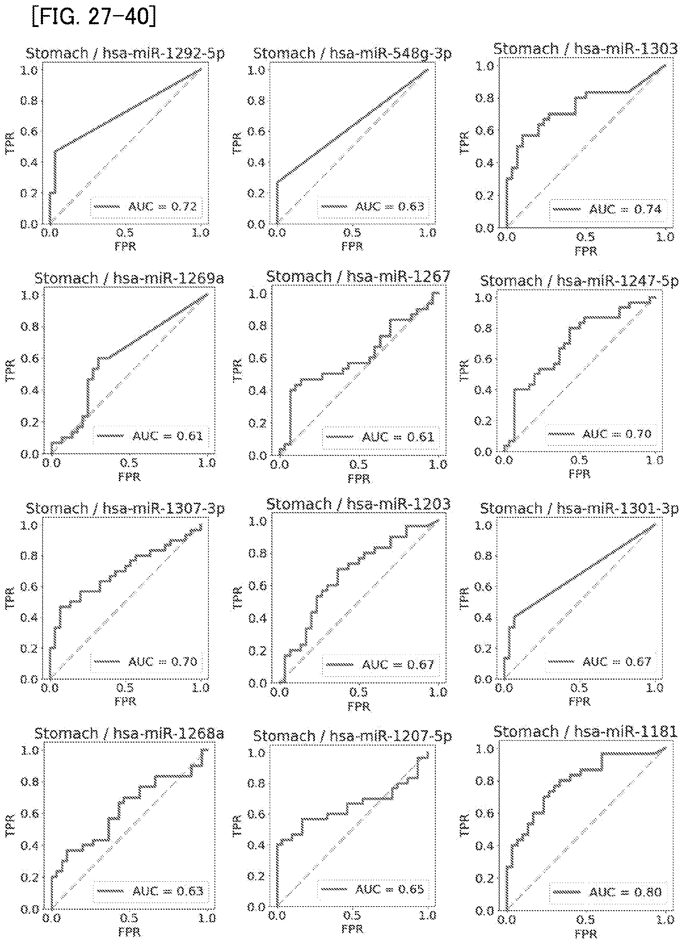

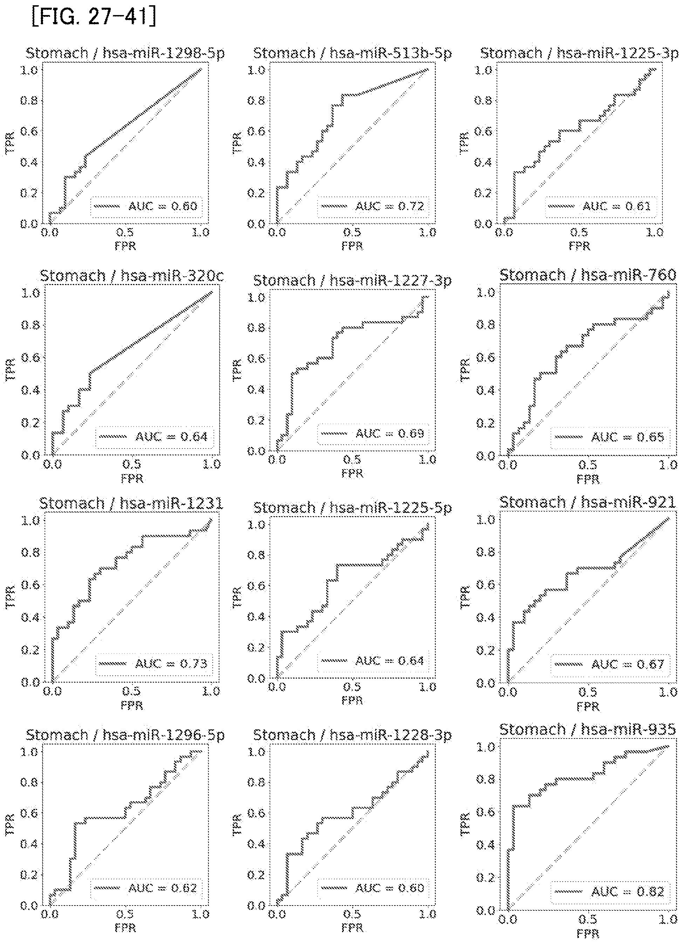

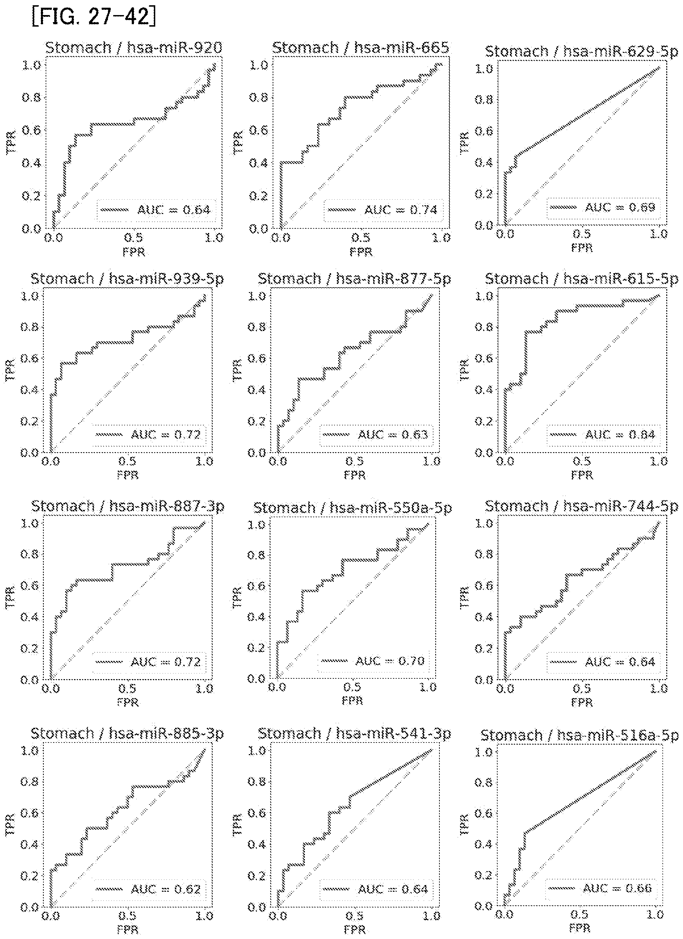

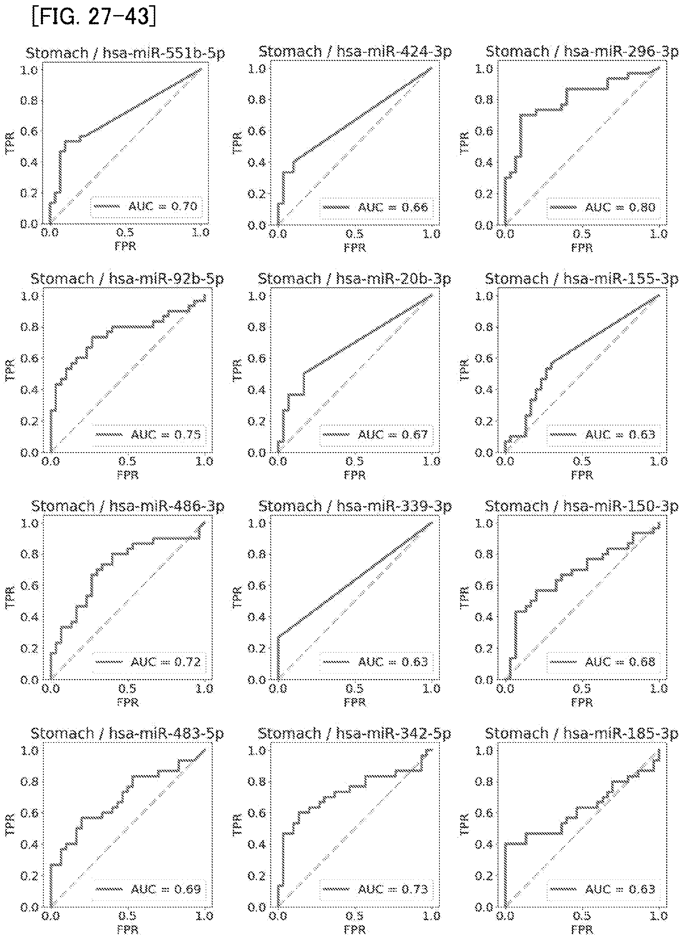

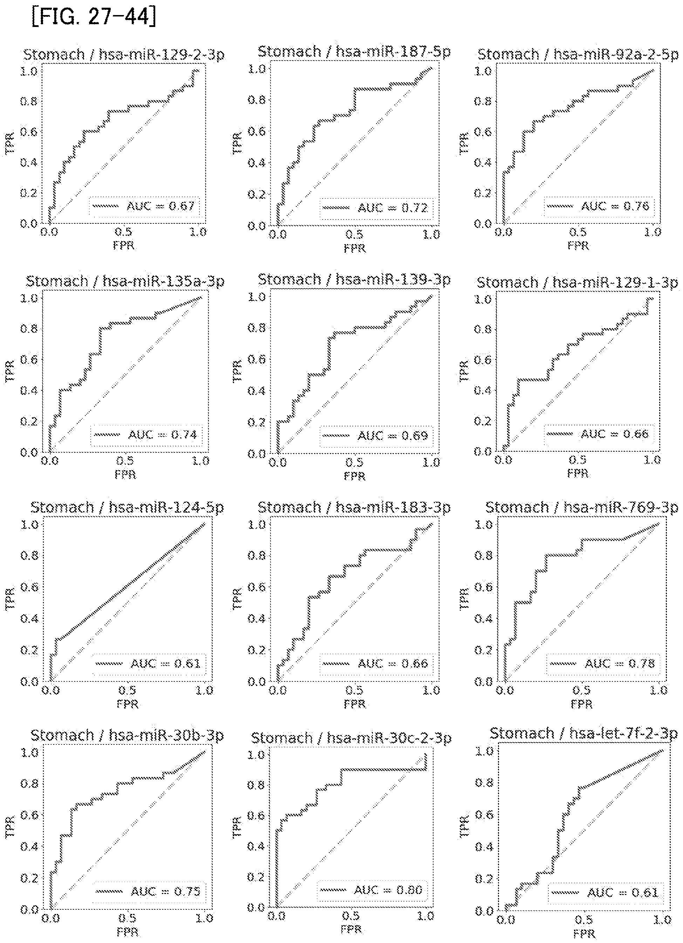

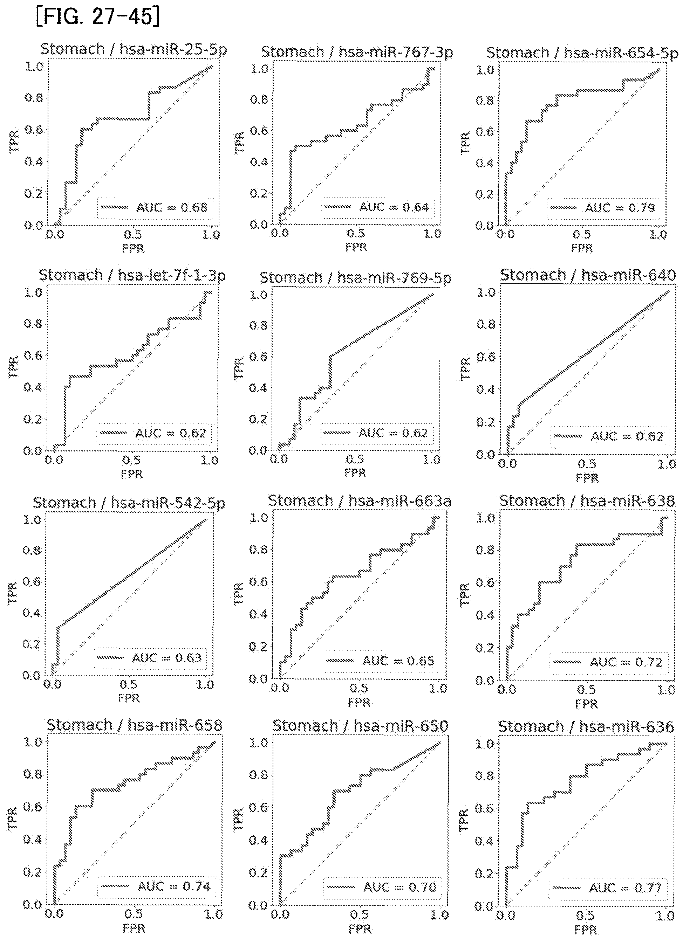

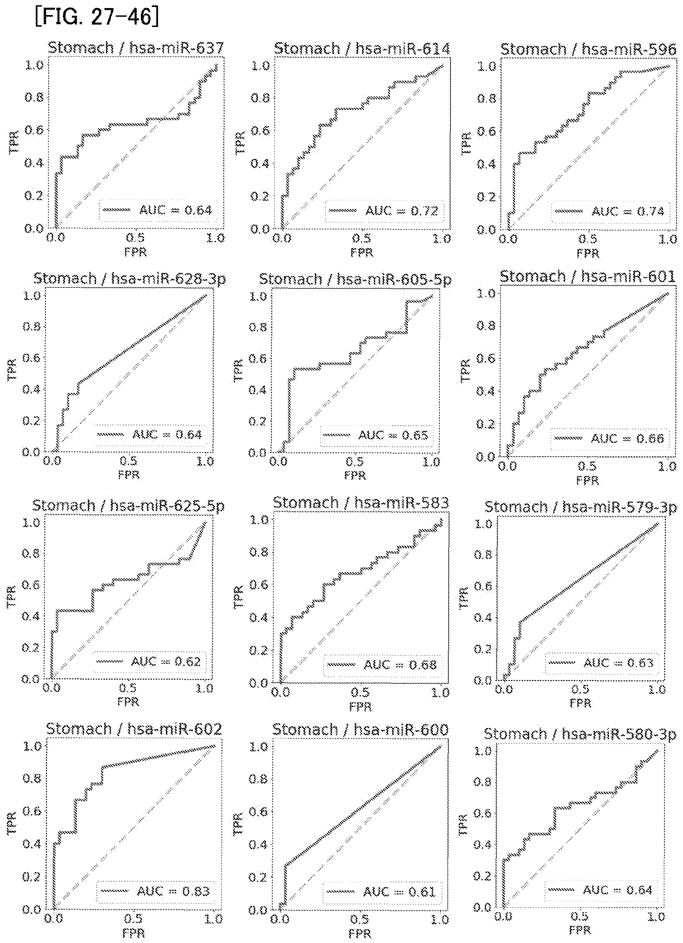

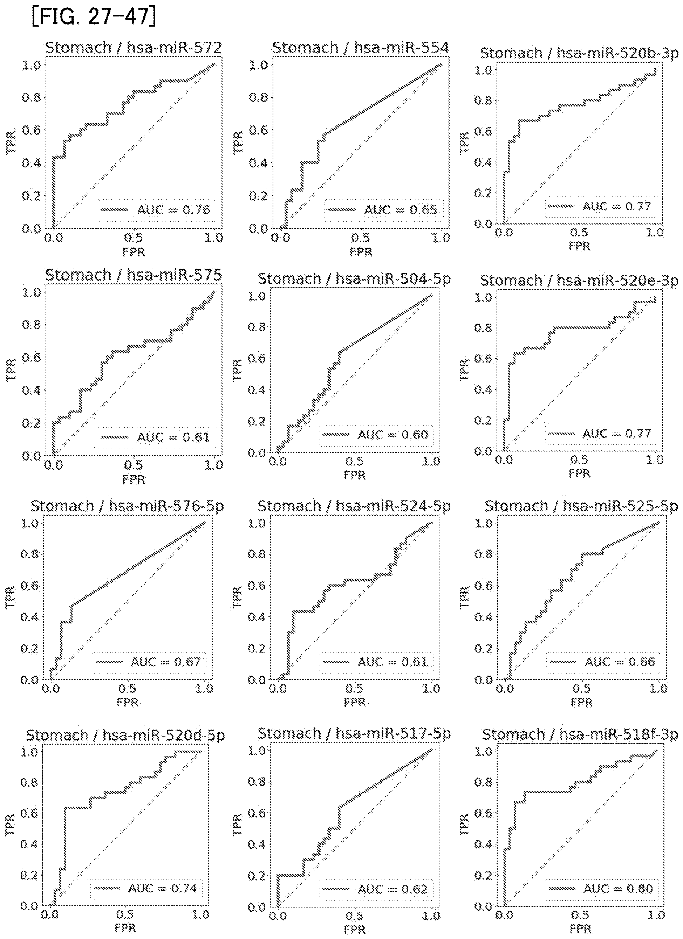

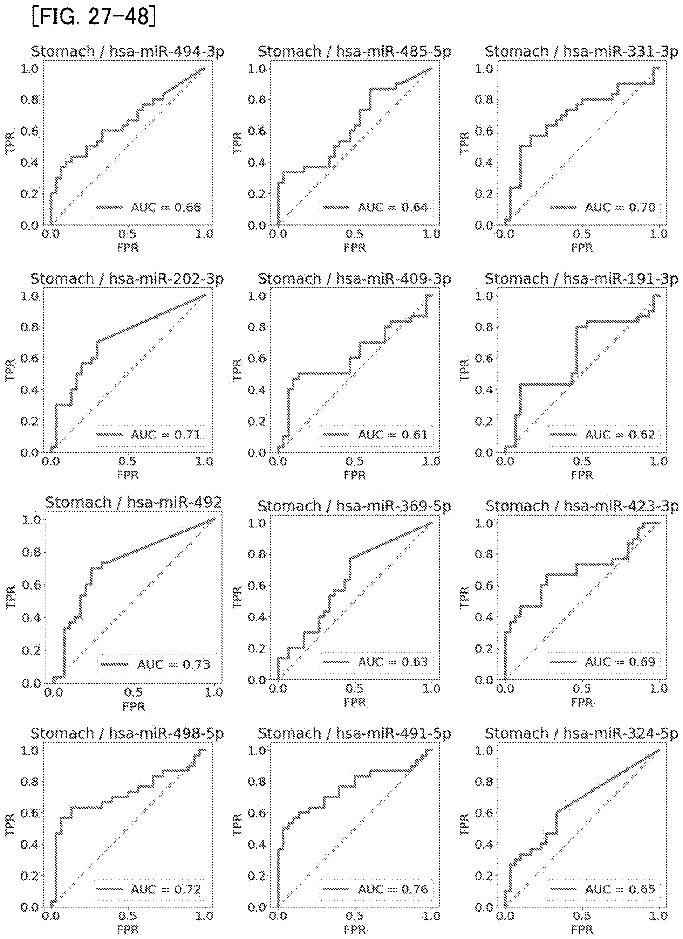

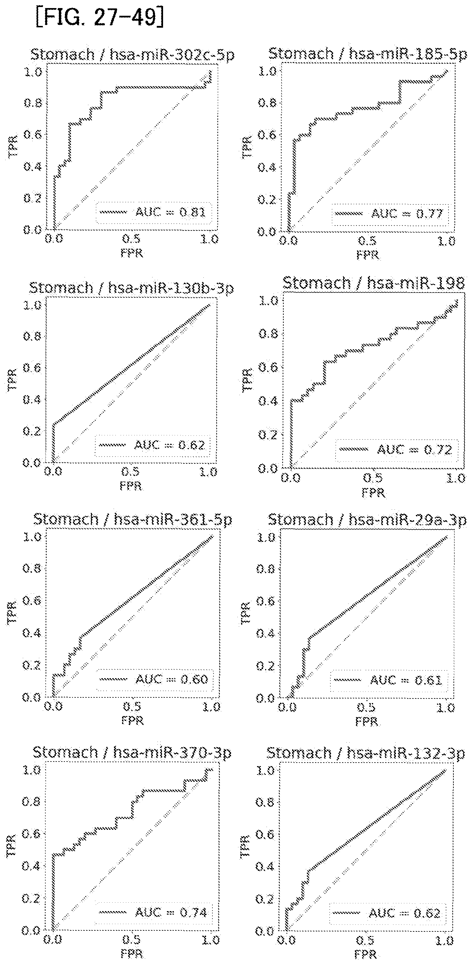

FIGS. 27-1 to 27-49 show ROCs curves for predicting gastric cancer in humans using a single miRNA.

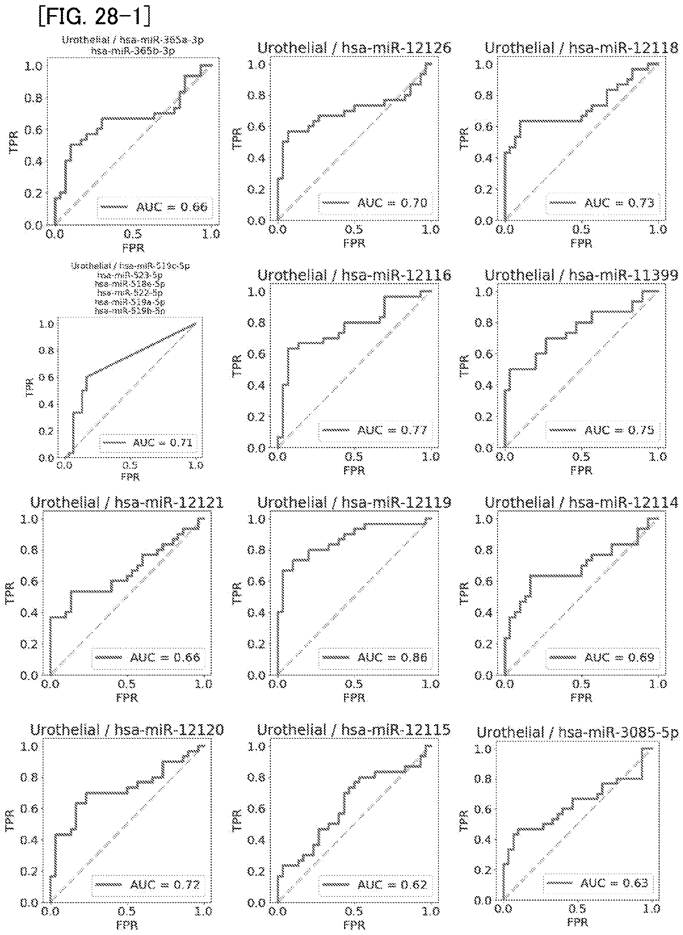

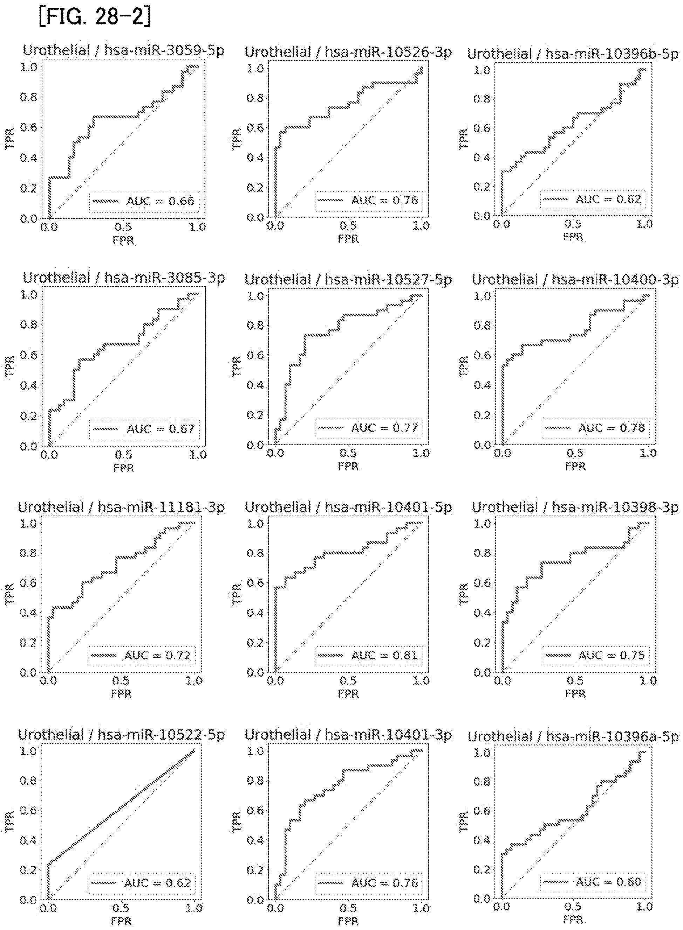

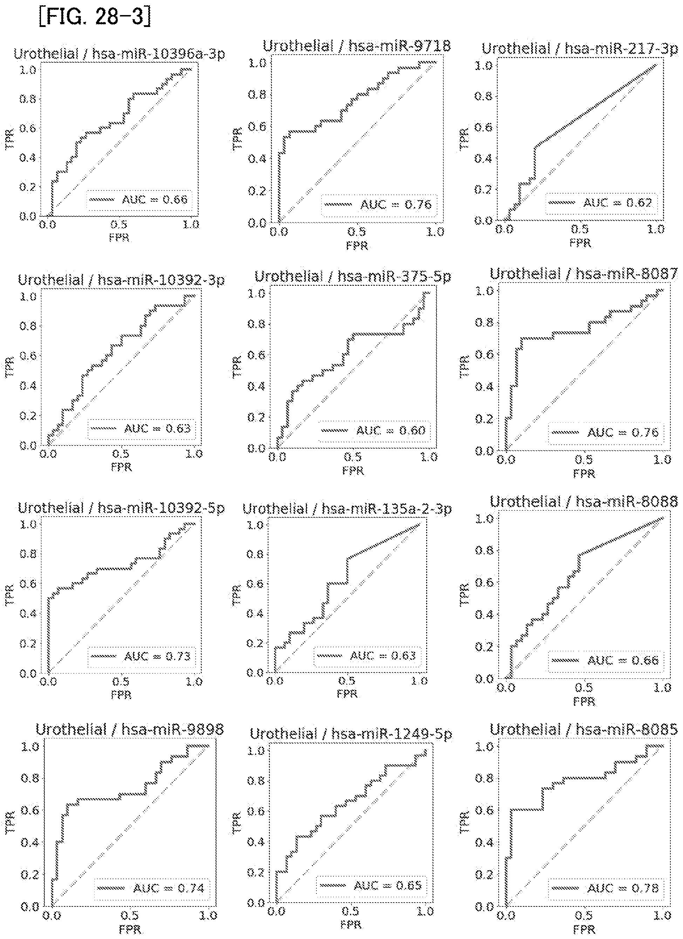

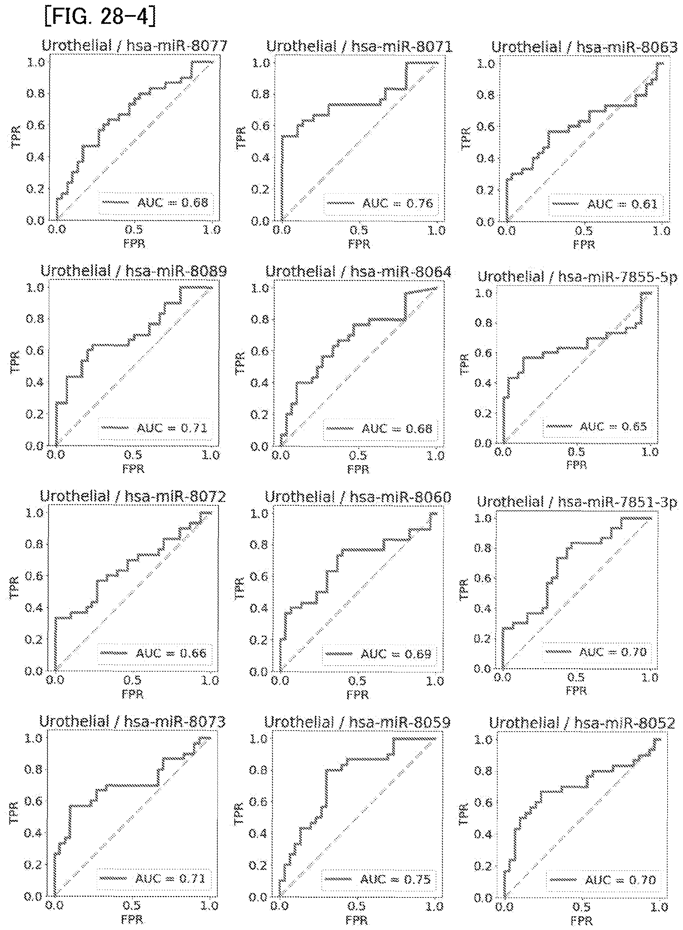

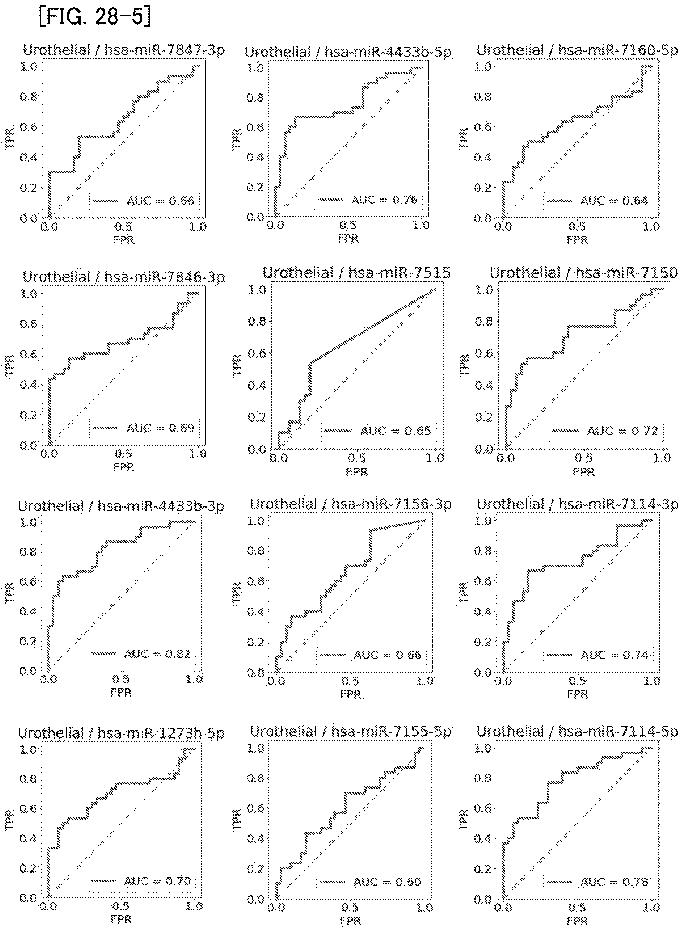

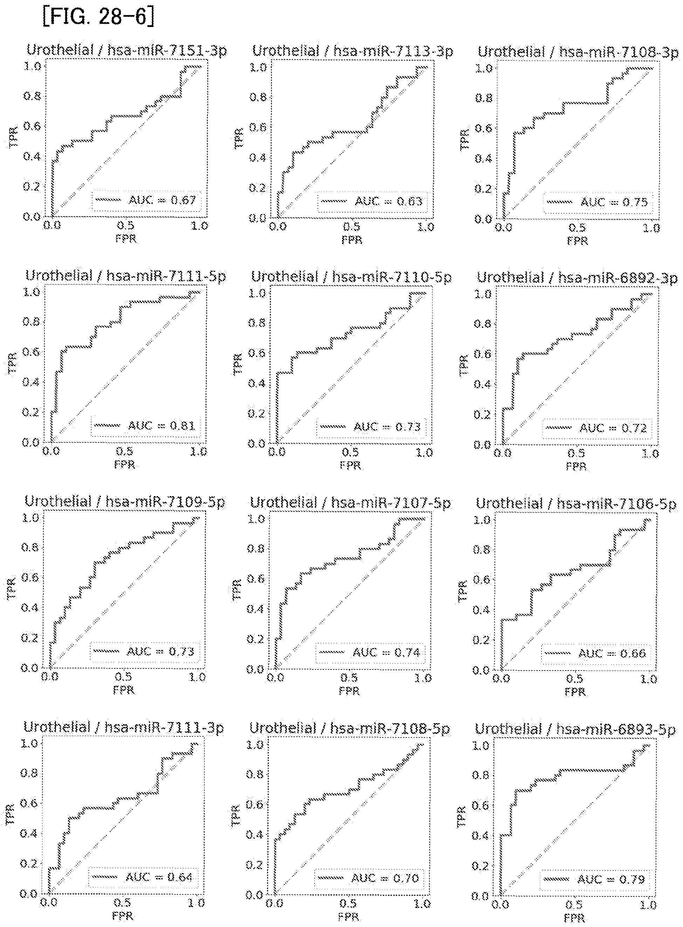

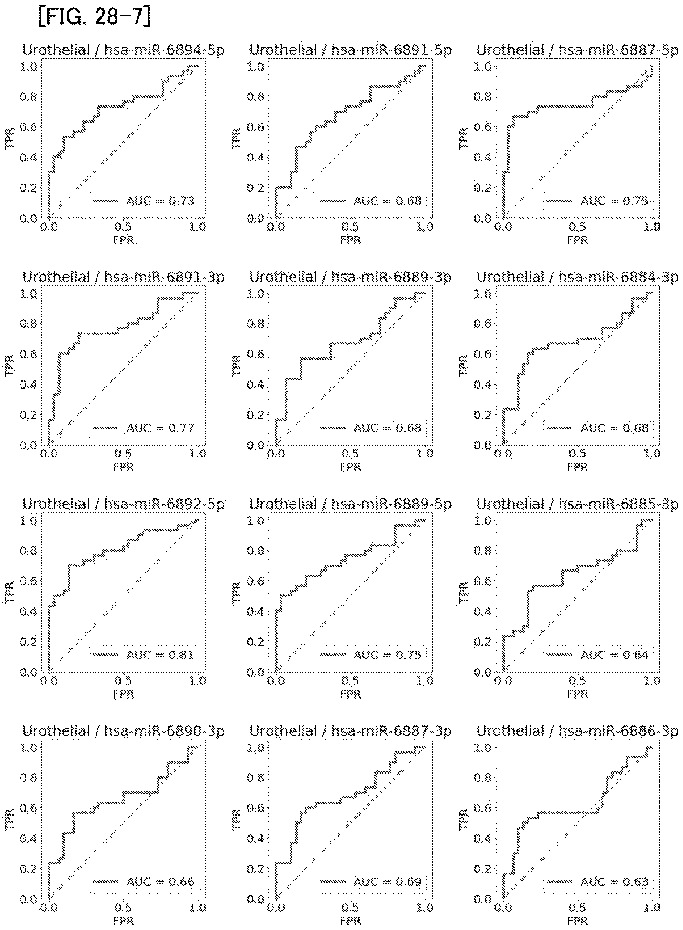

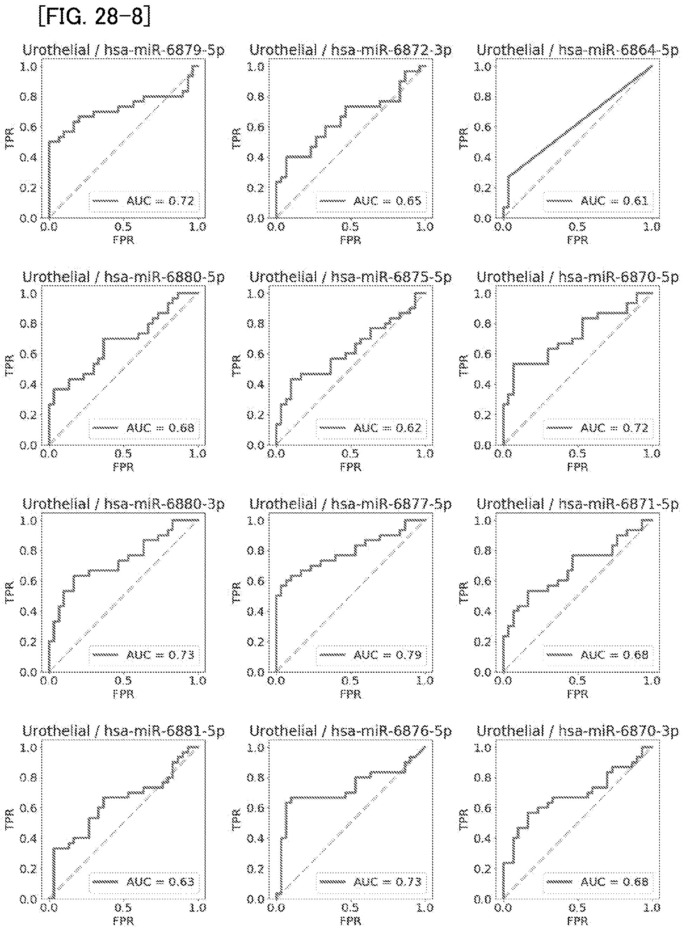

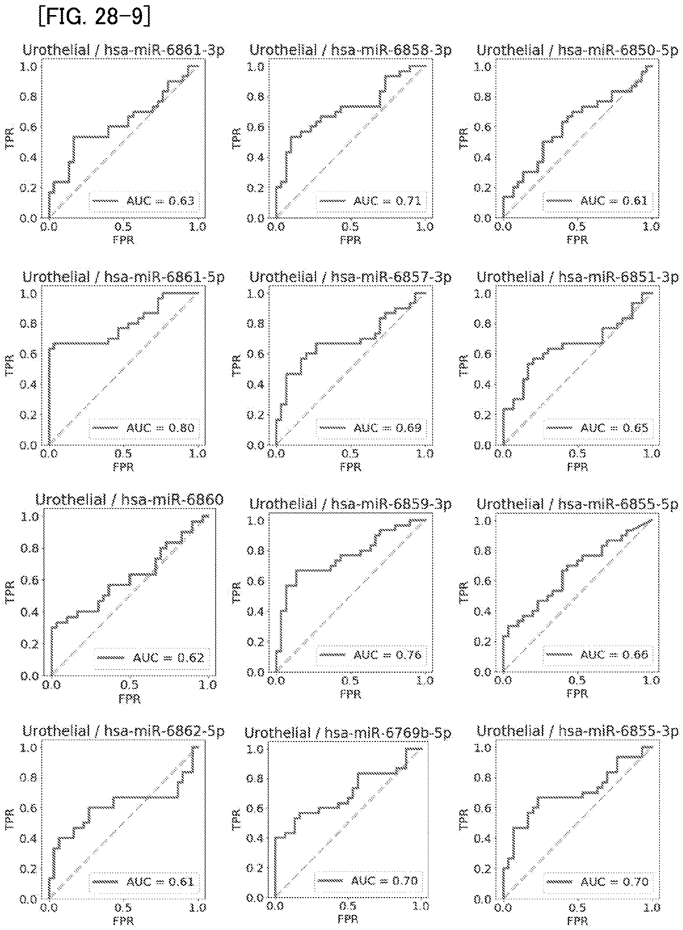

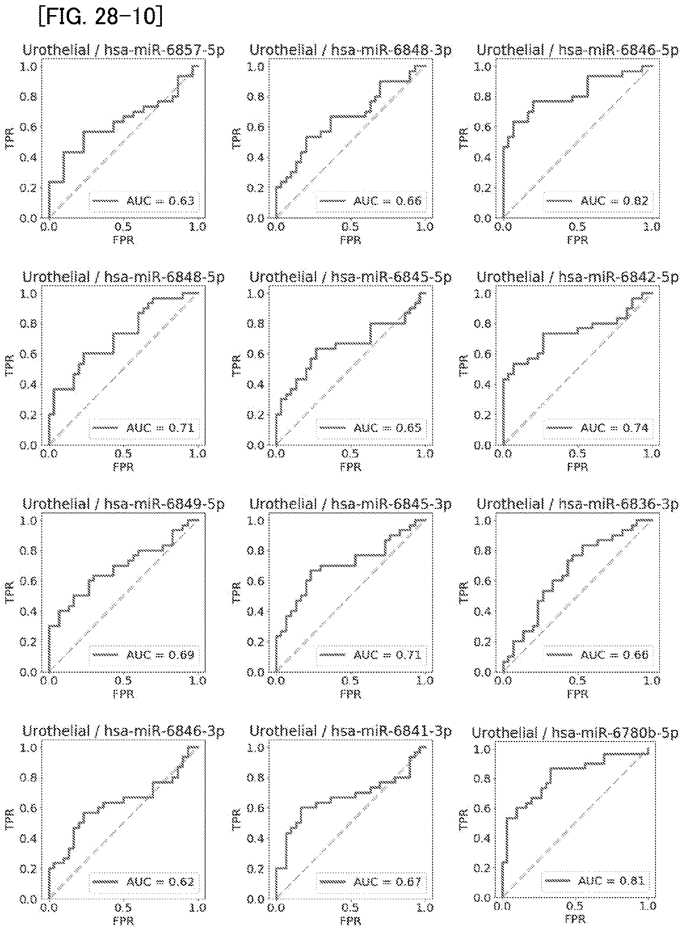

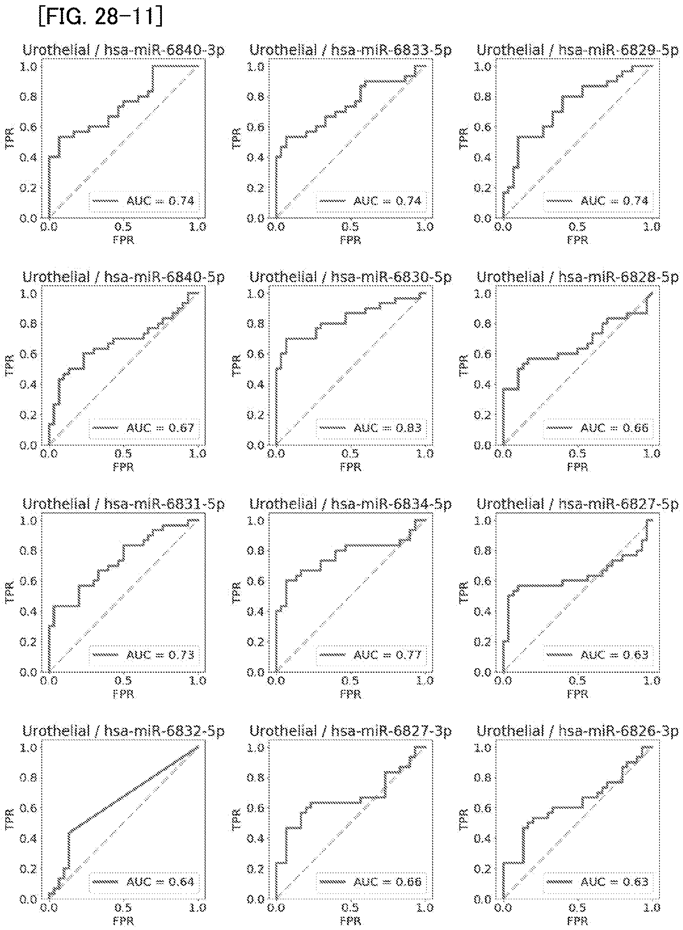

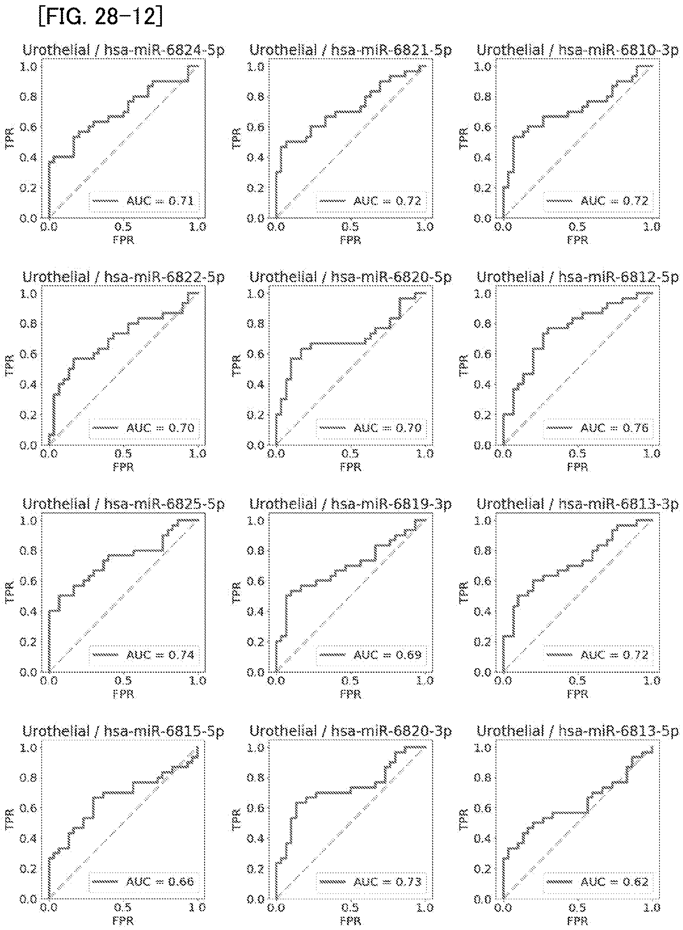

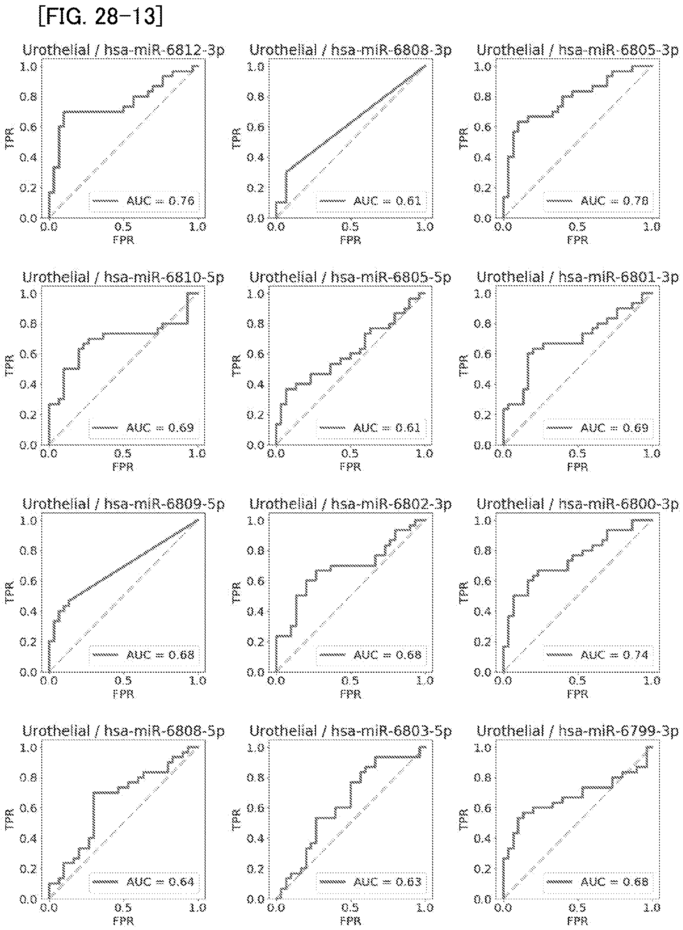

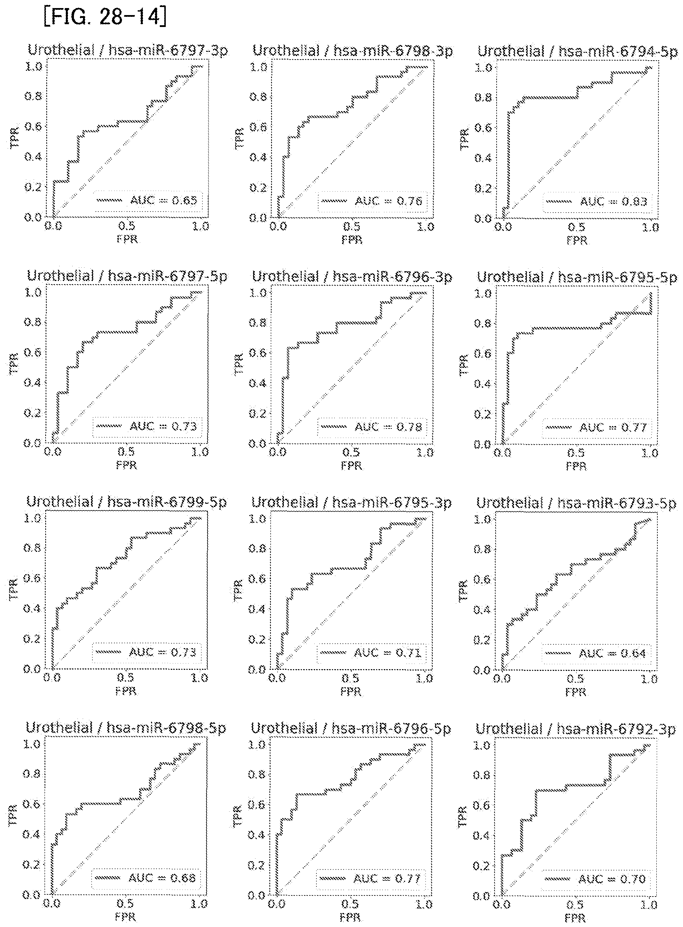

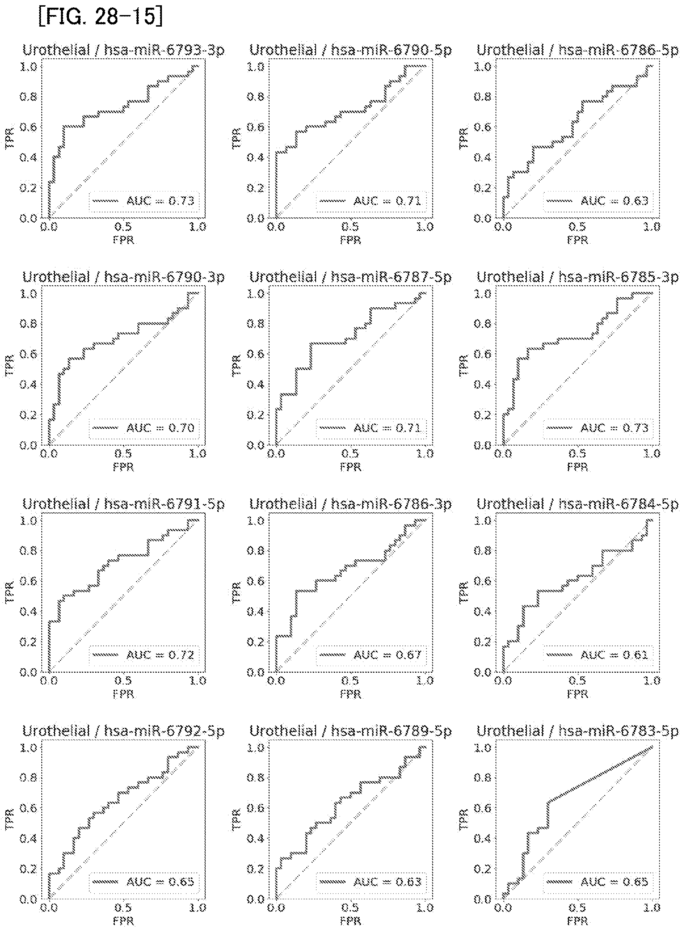

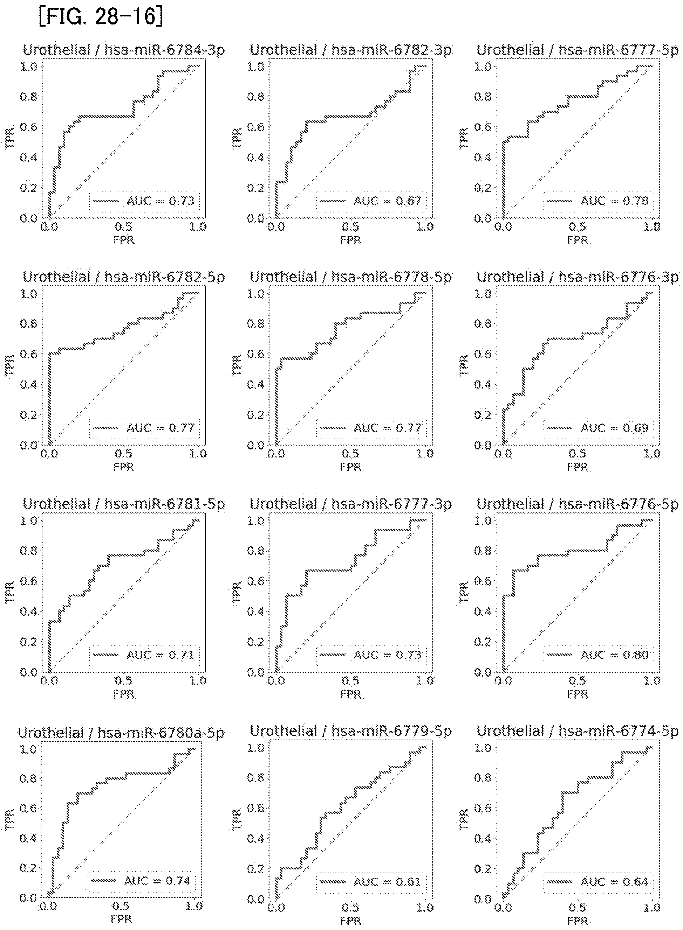

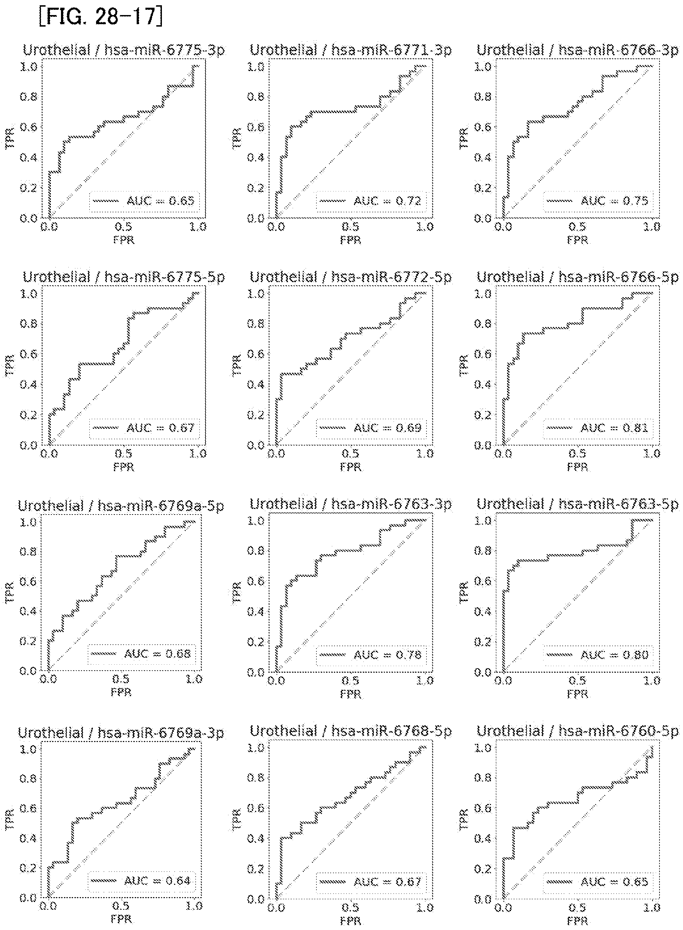

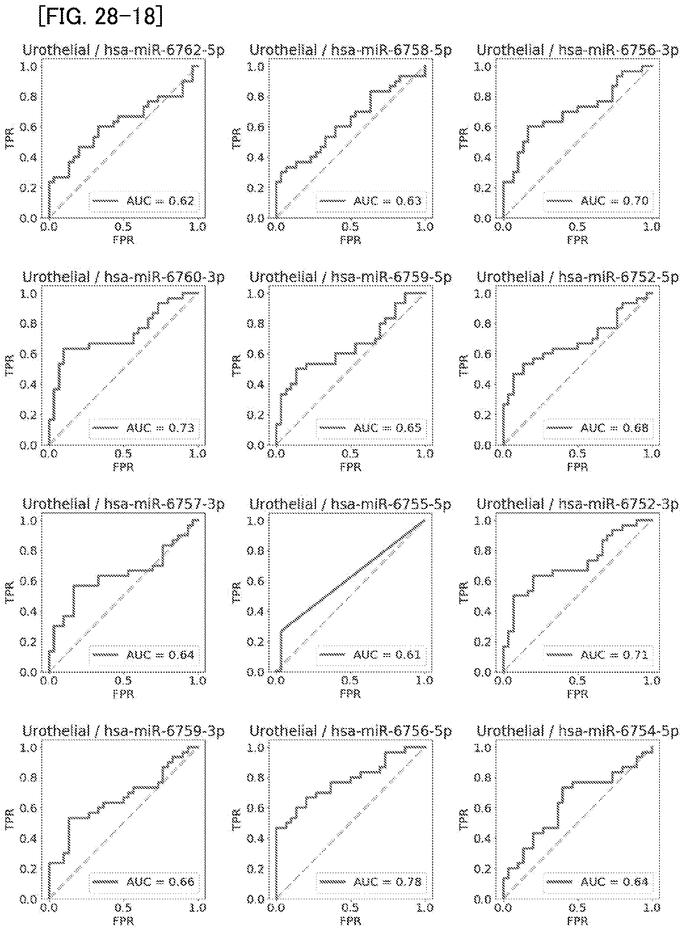

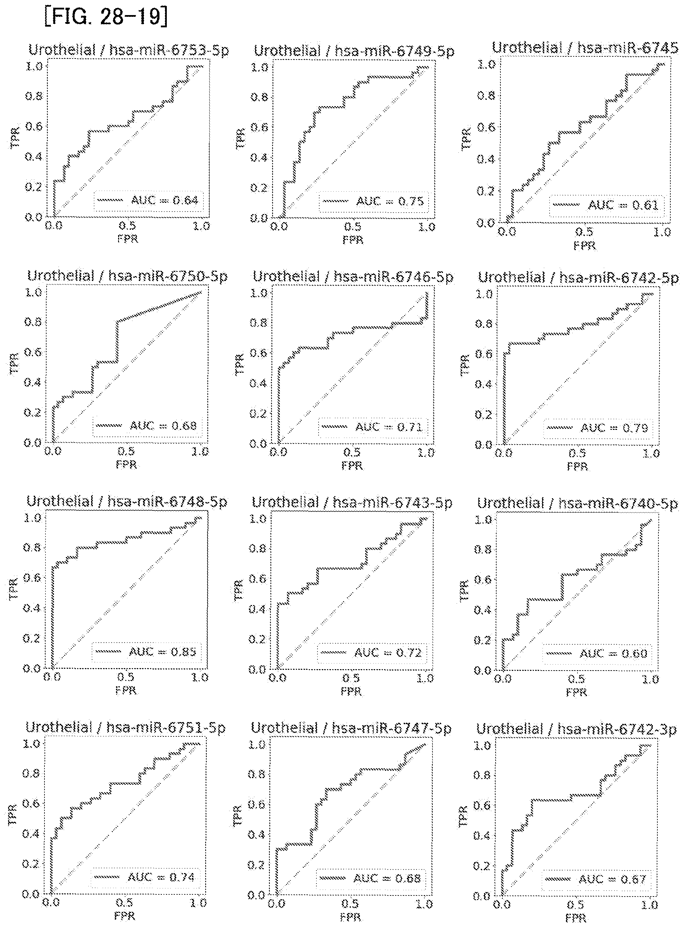

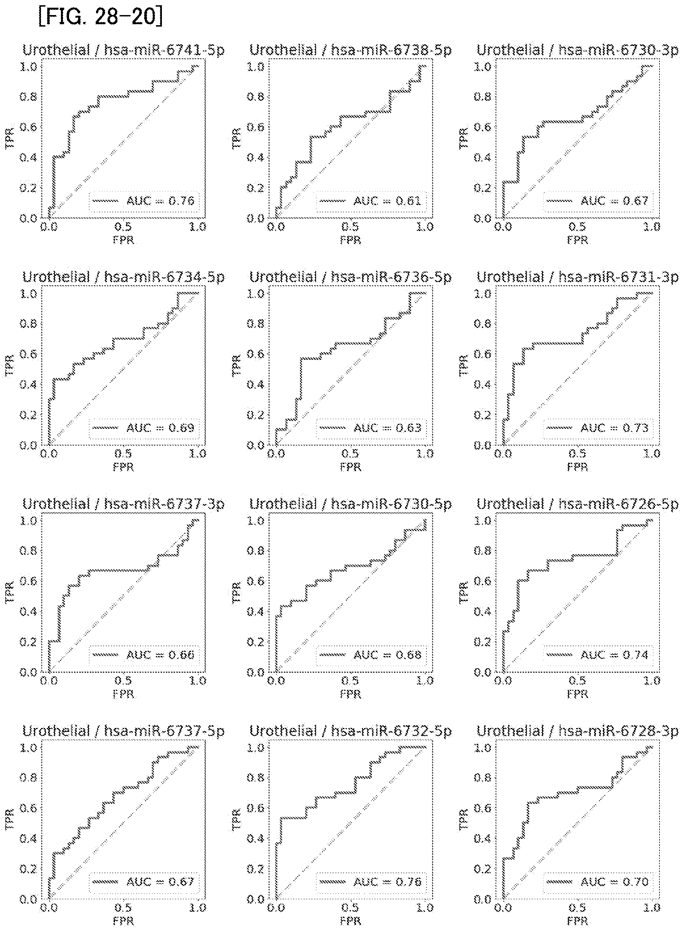

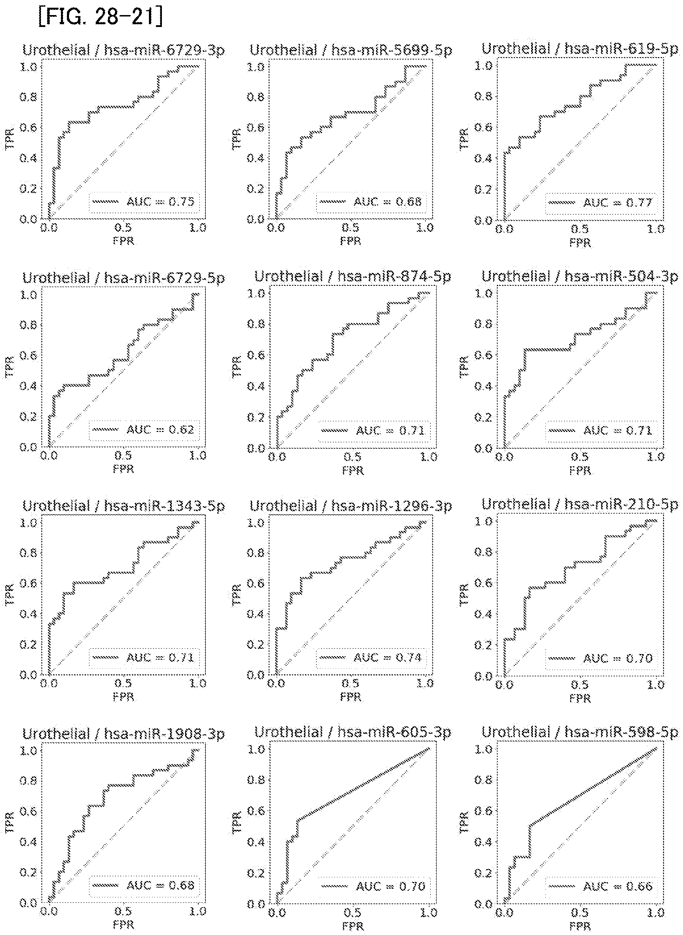

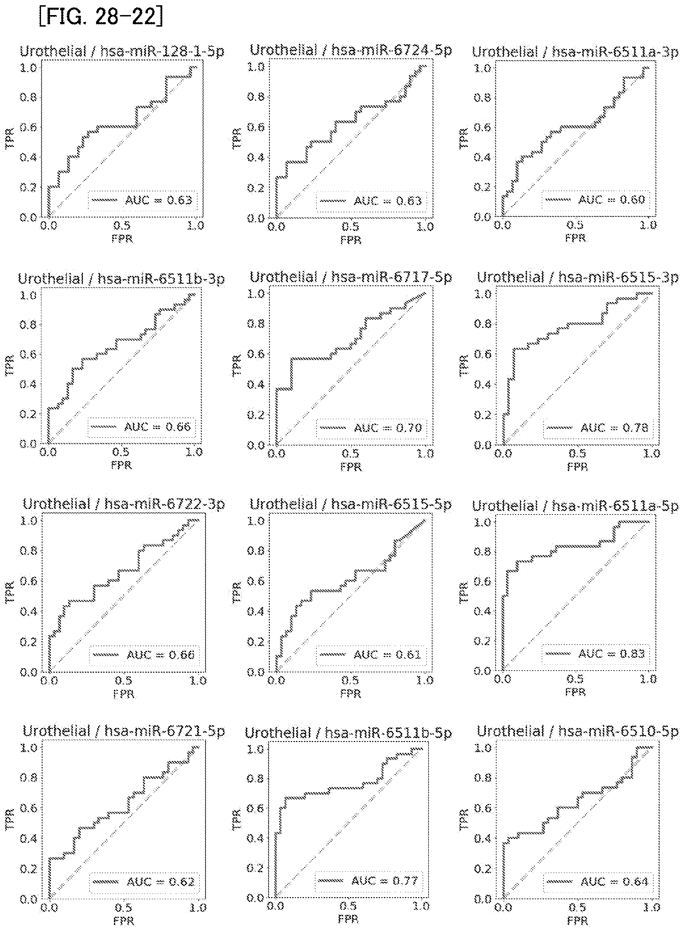

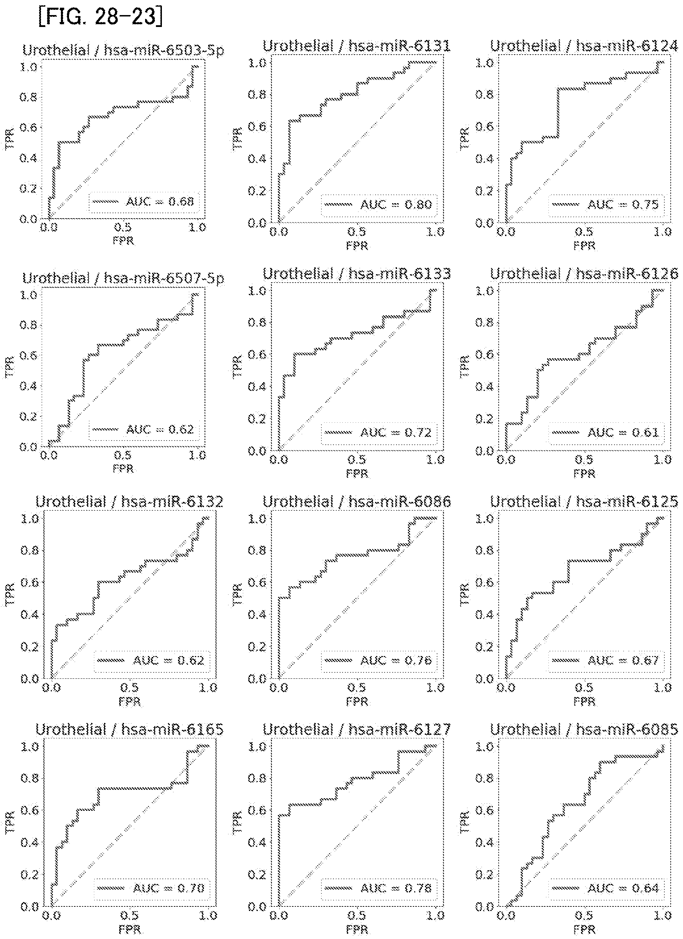

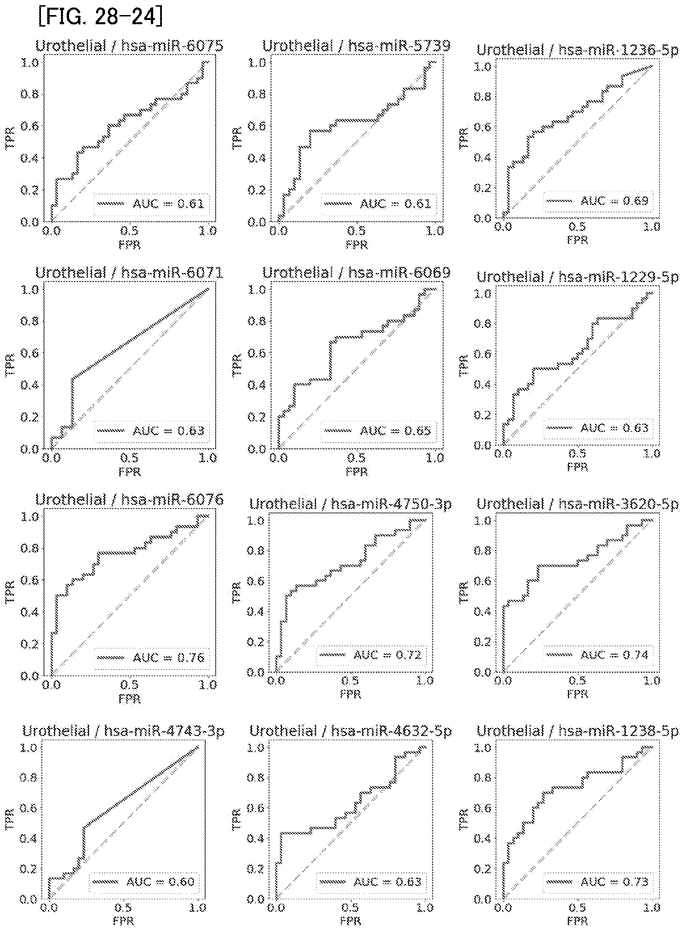

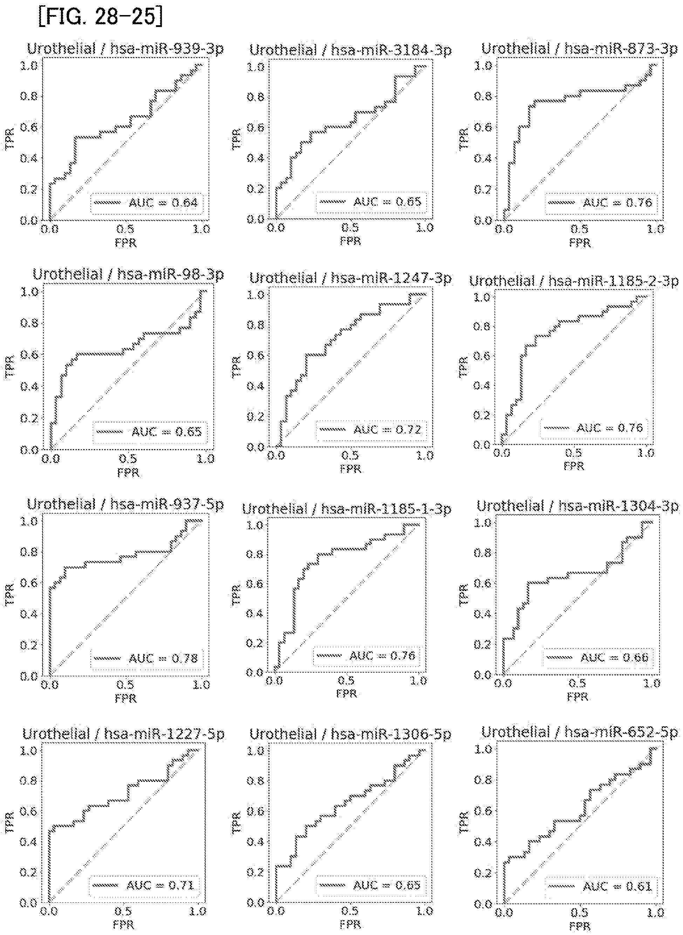

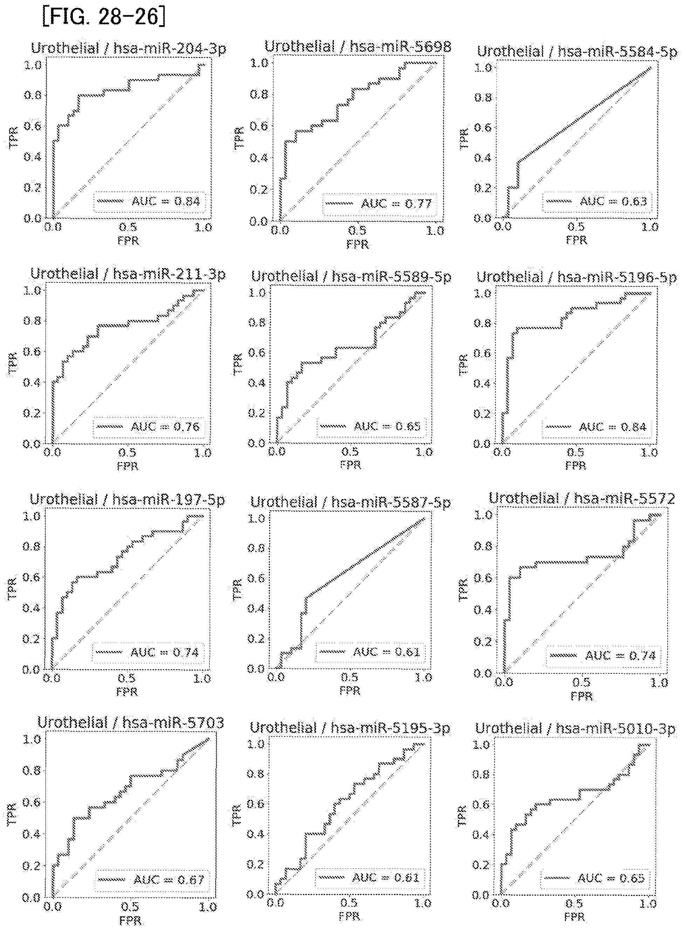

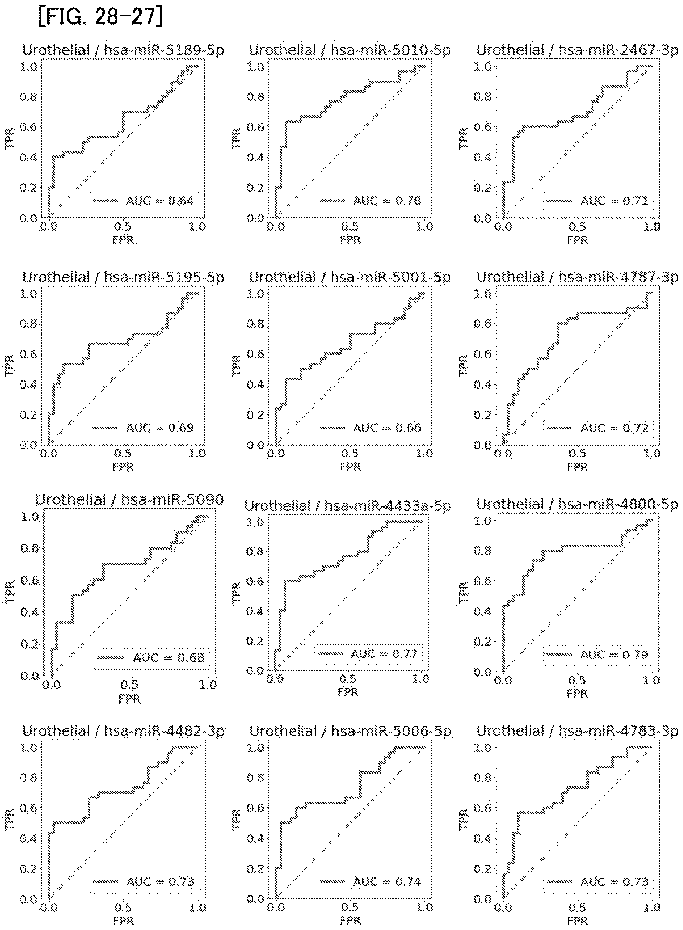

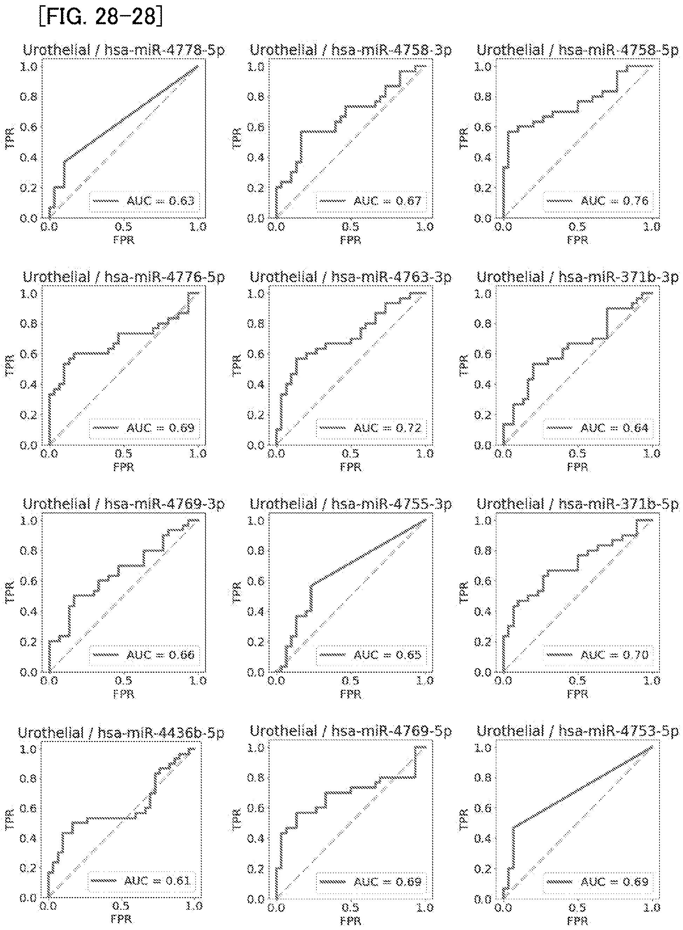

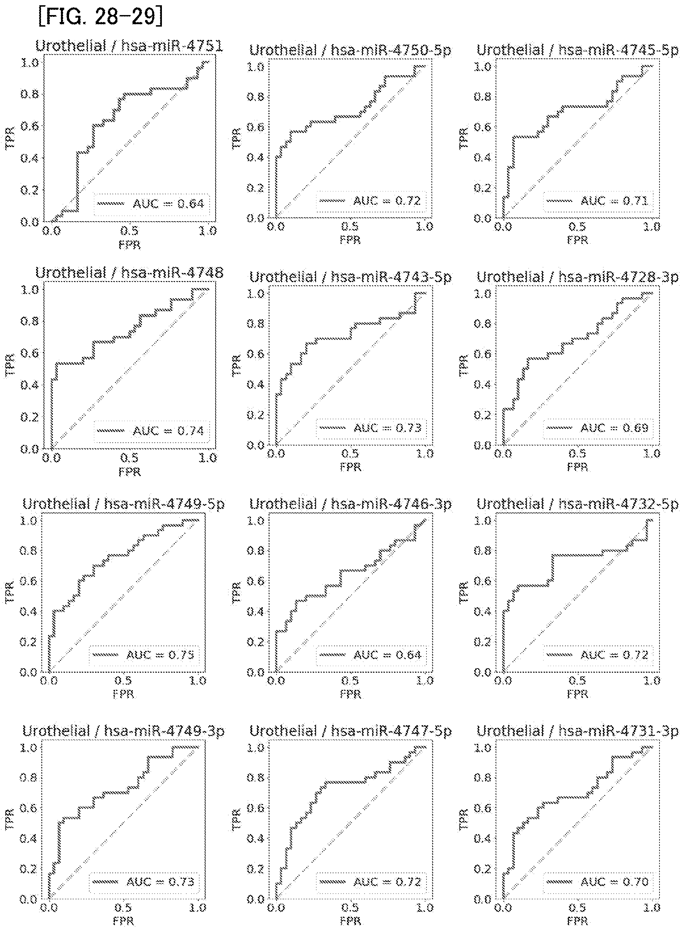

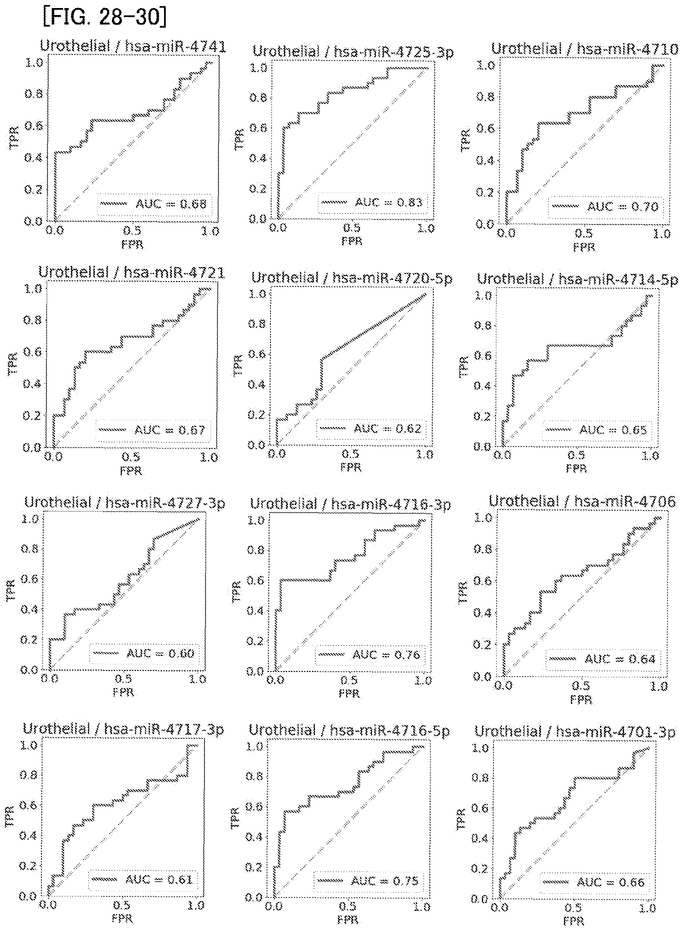

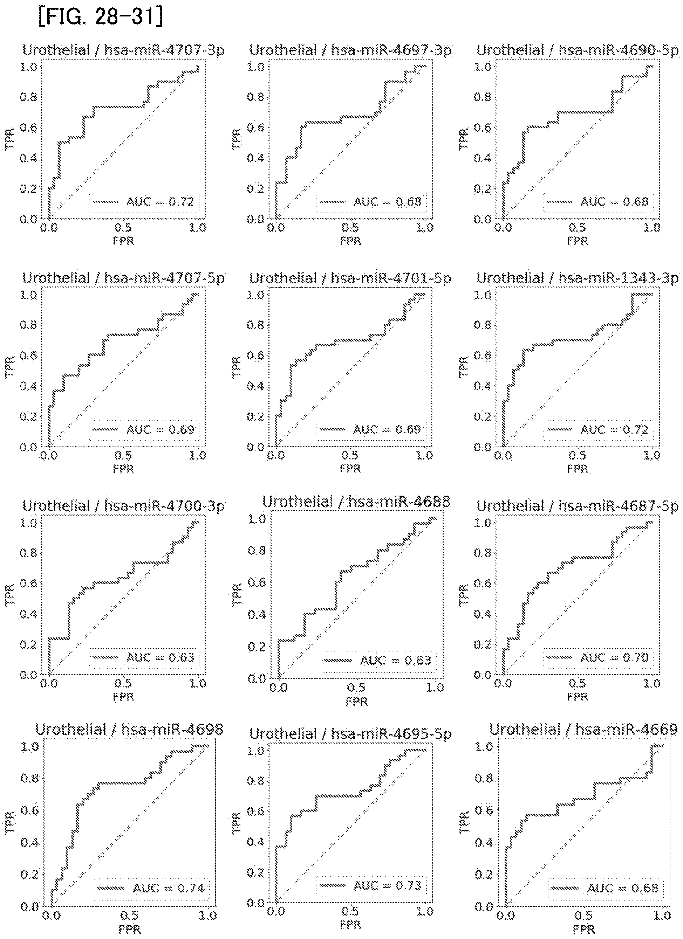

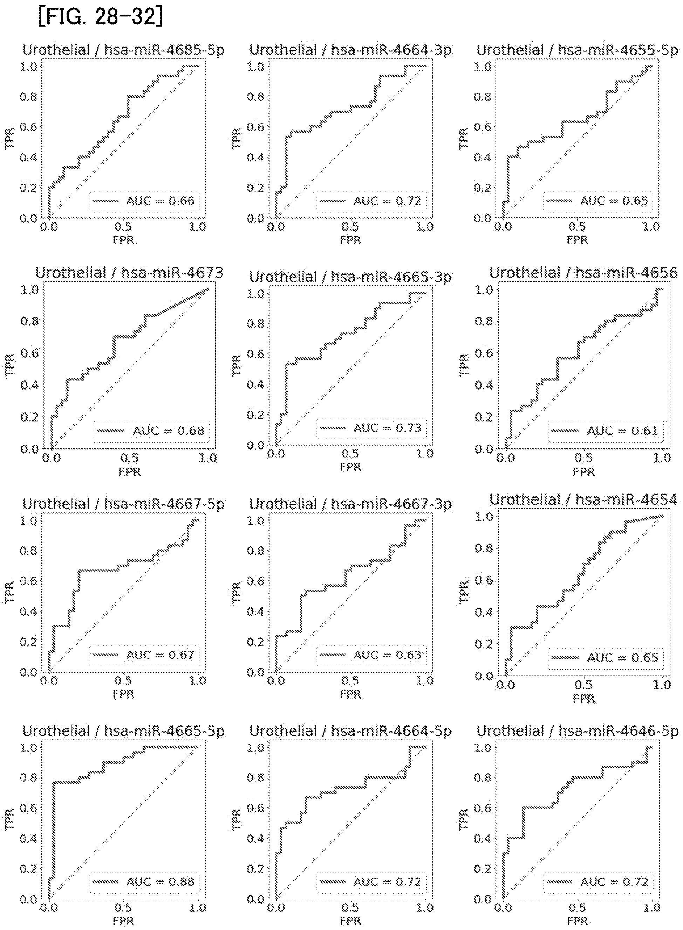

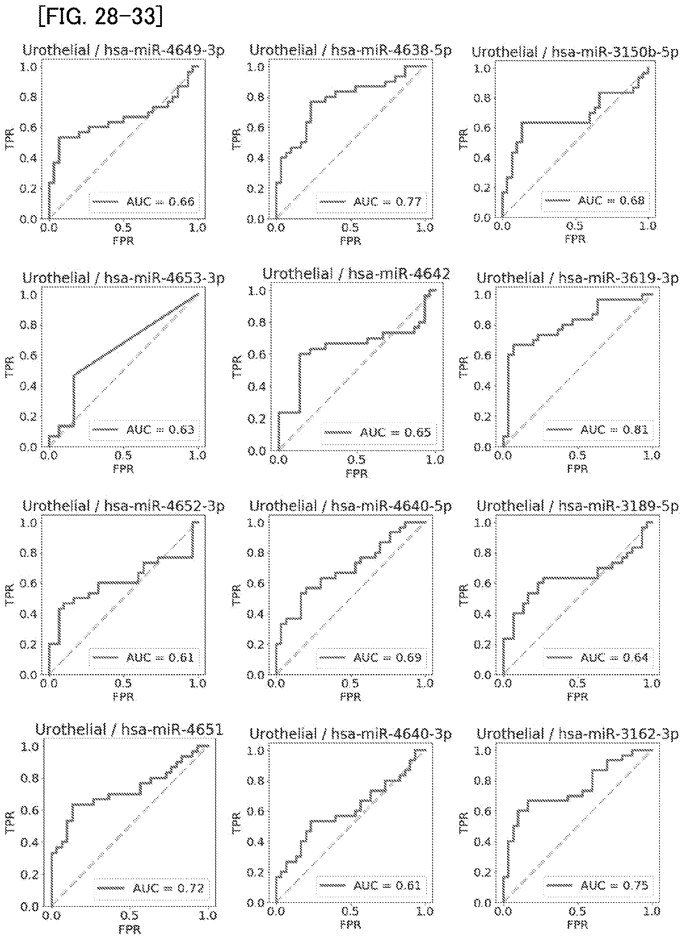

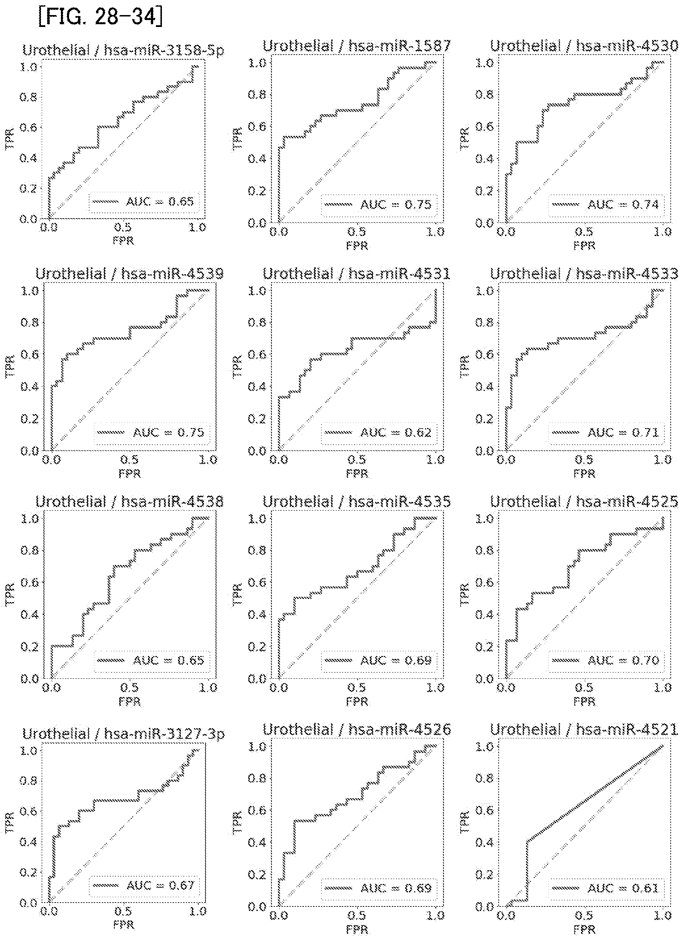

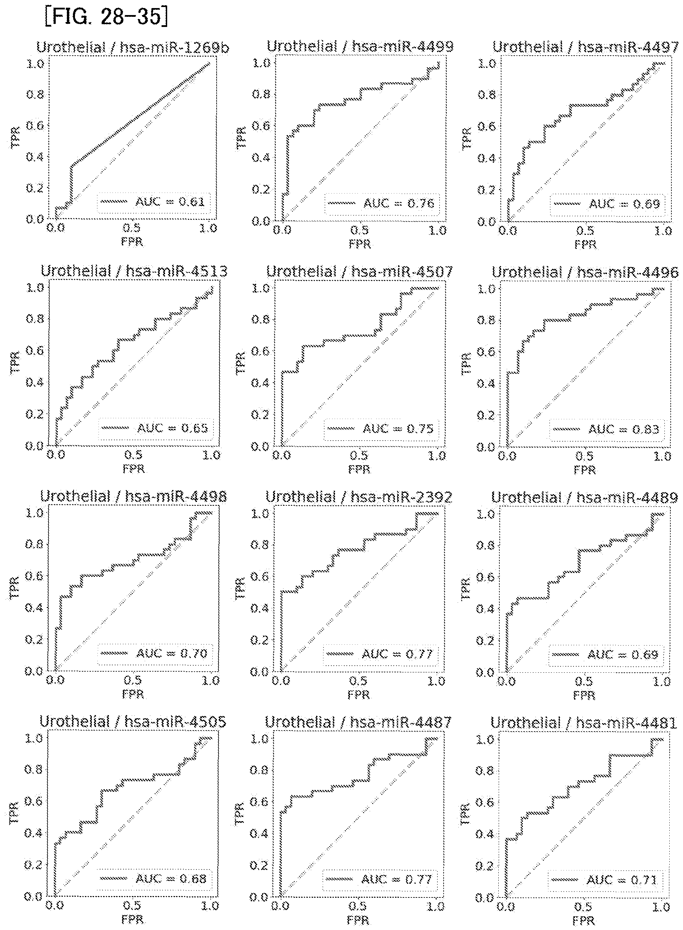

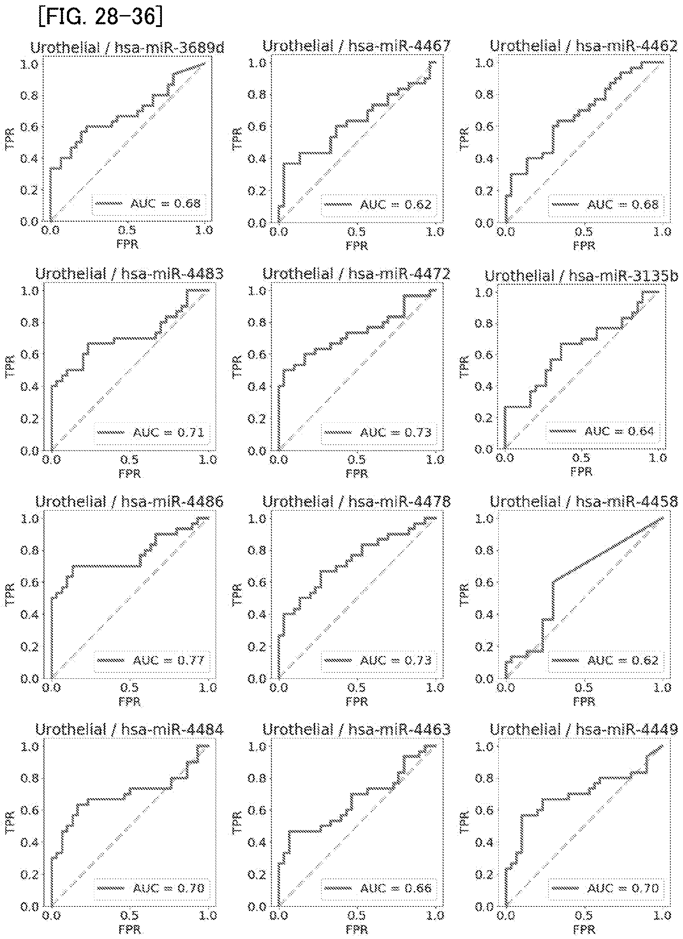

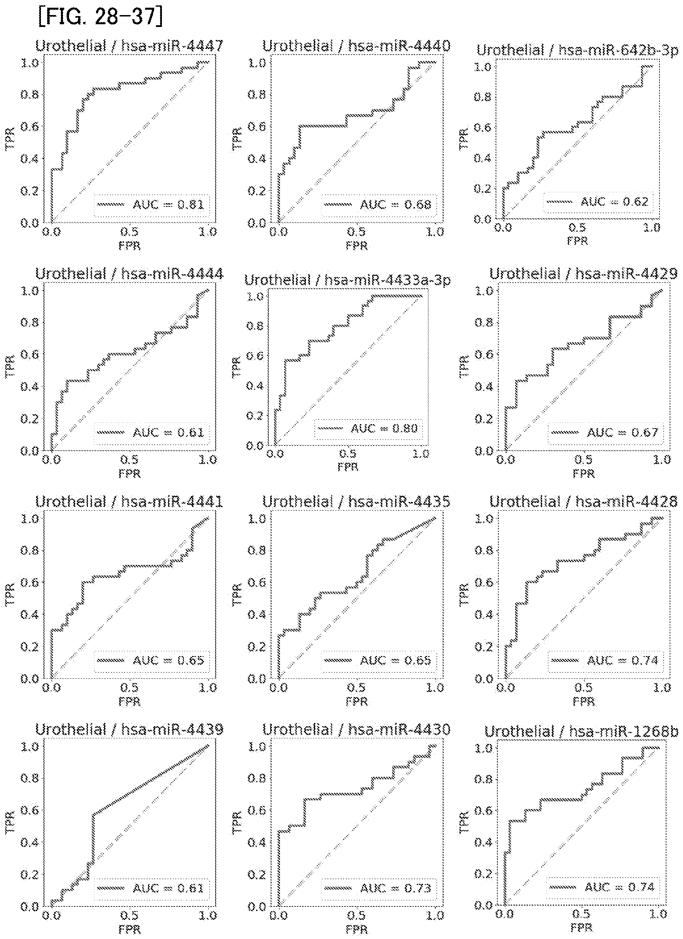

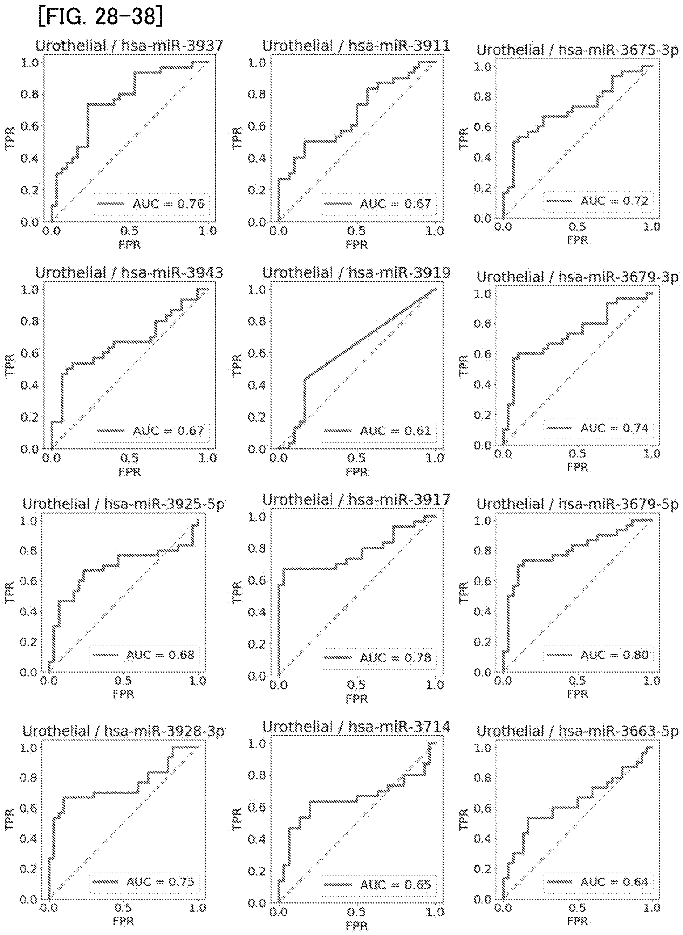

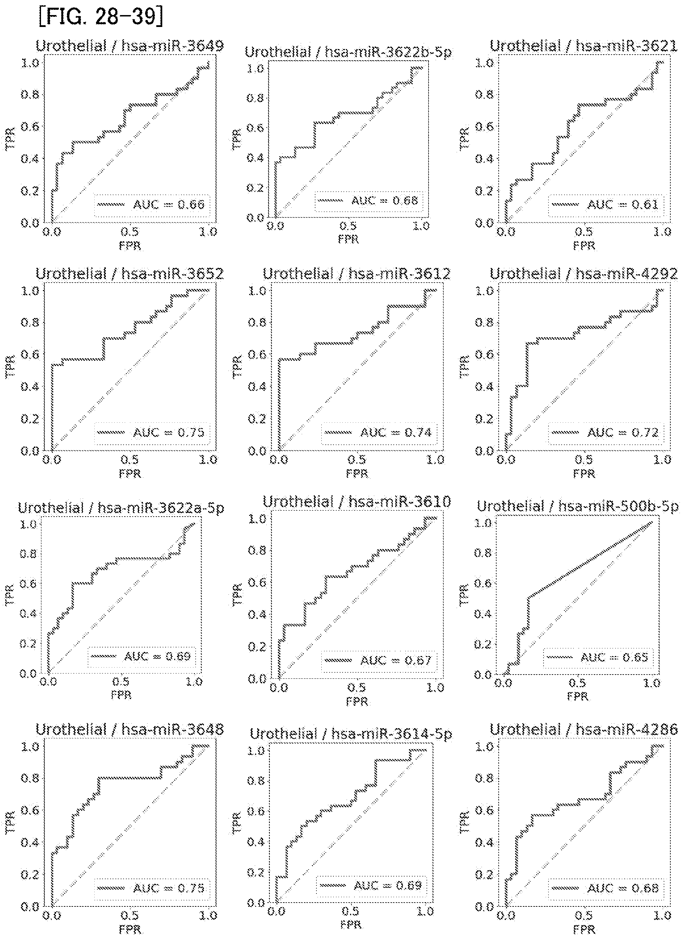

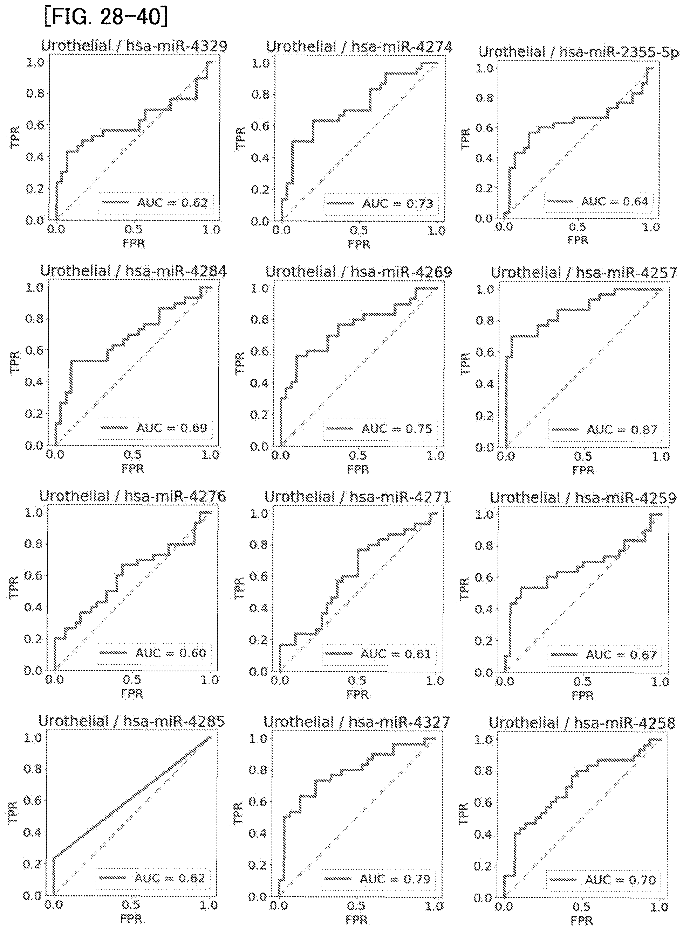

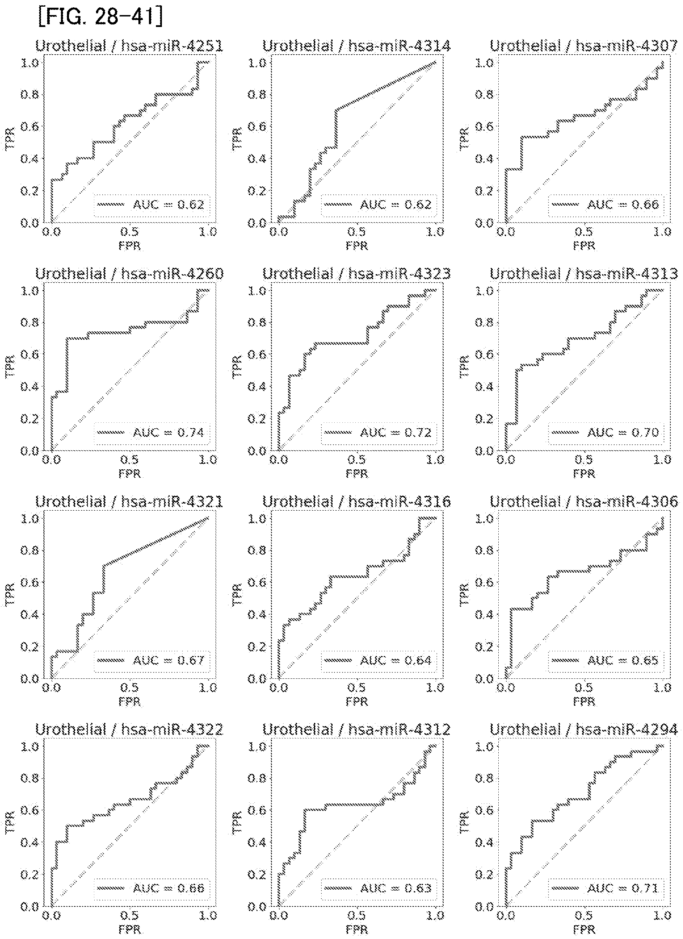

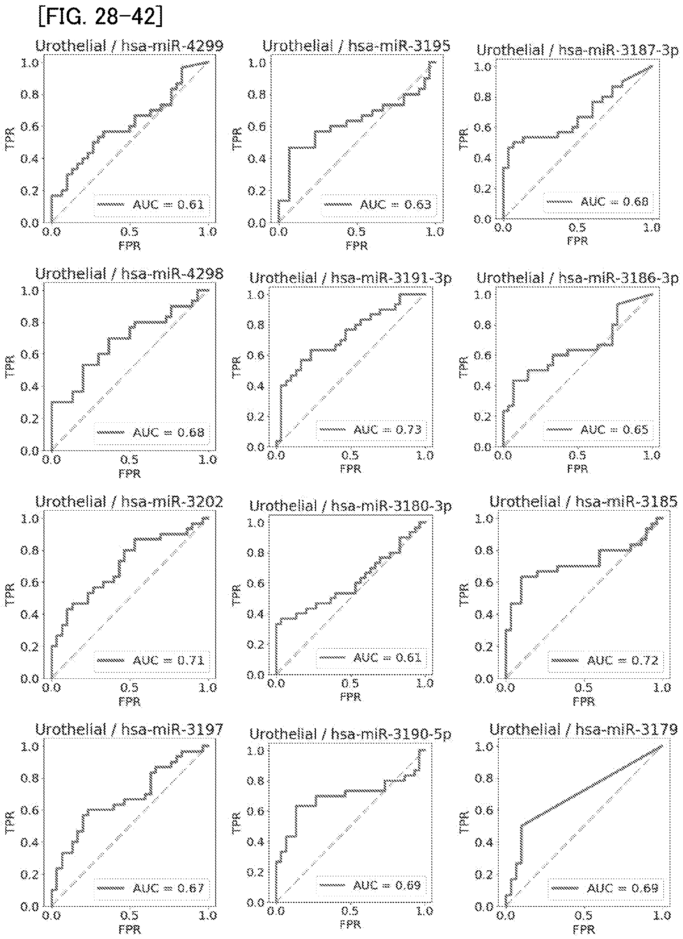

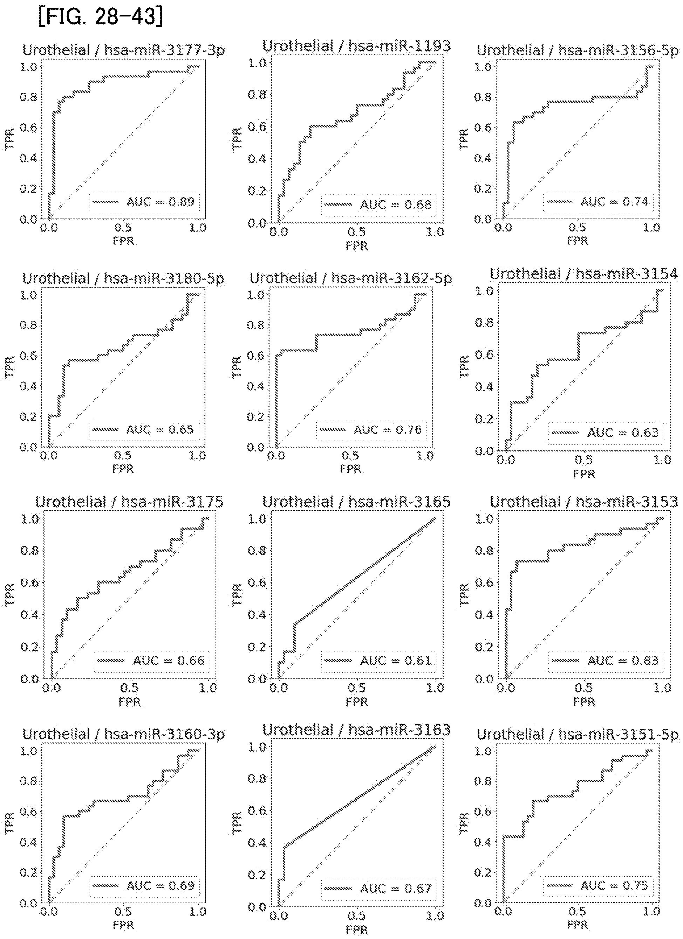

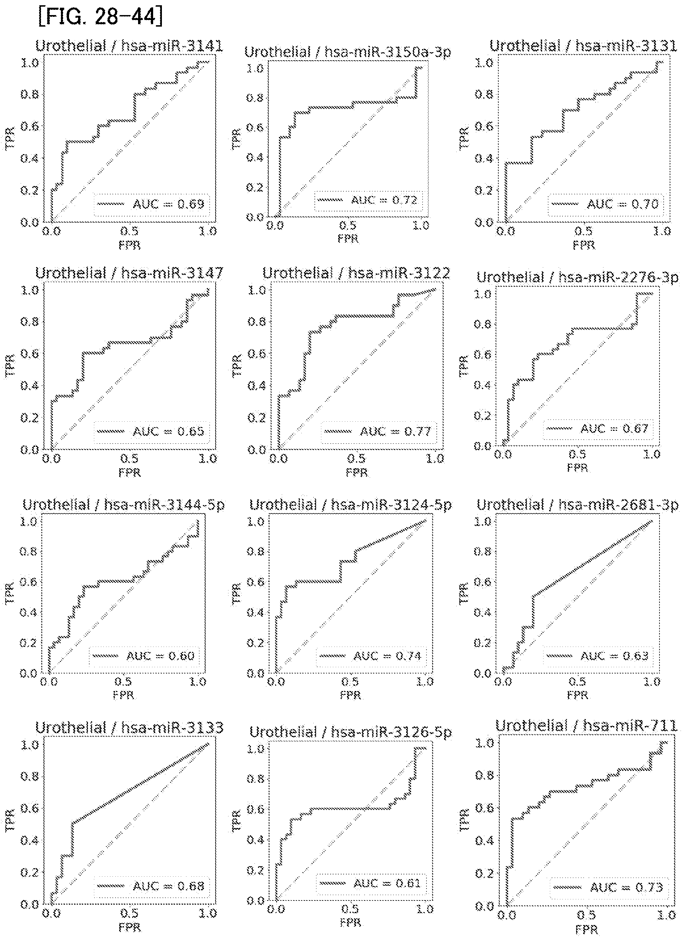

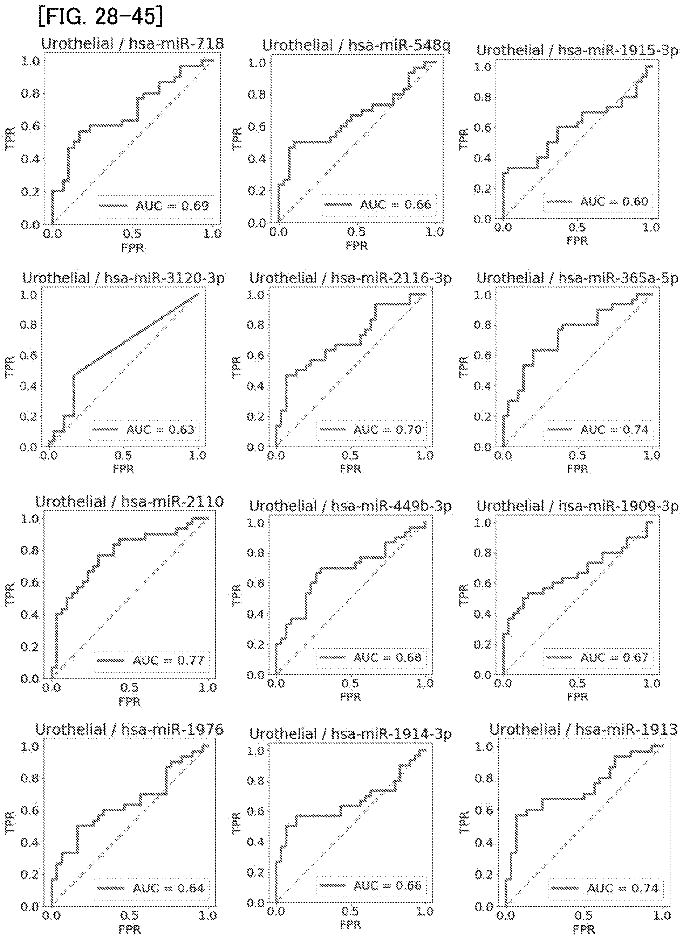

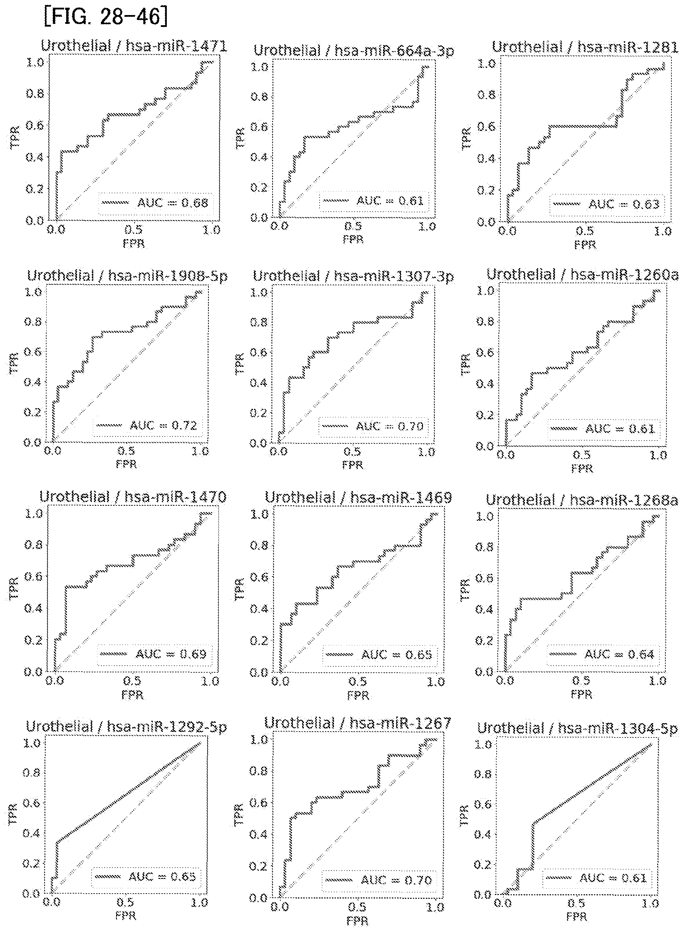

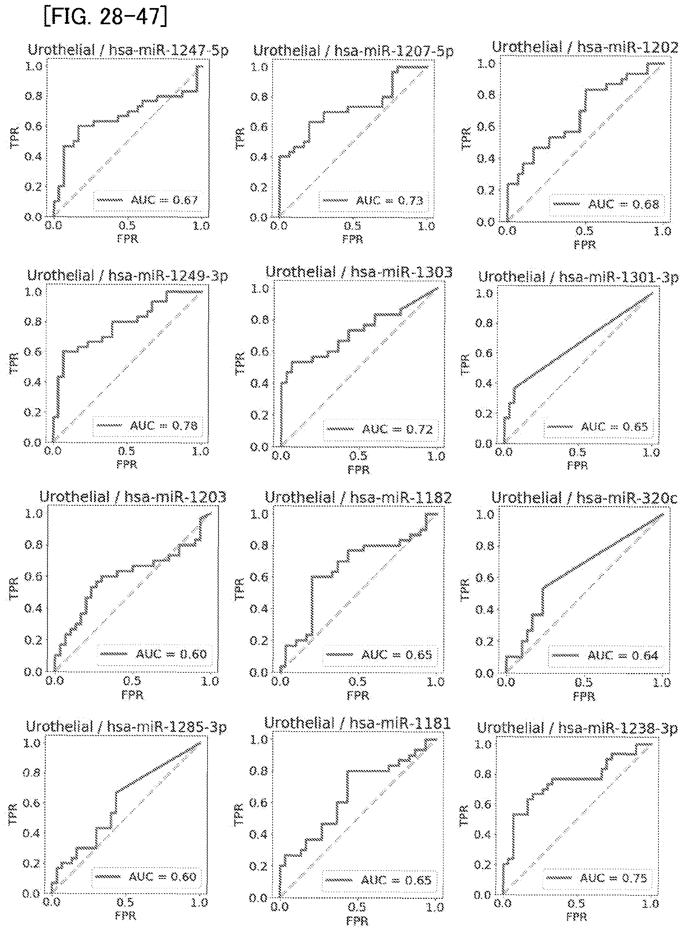

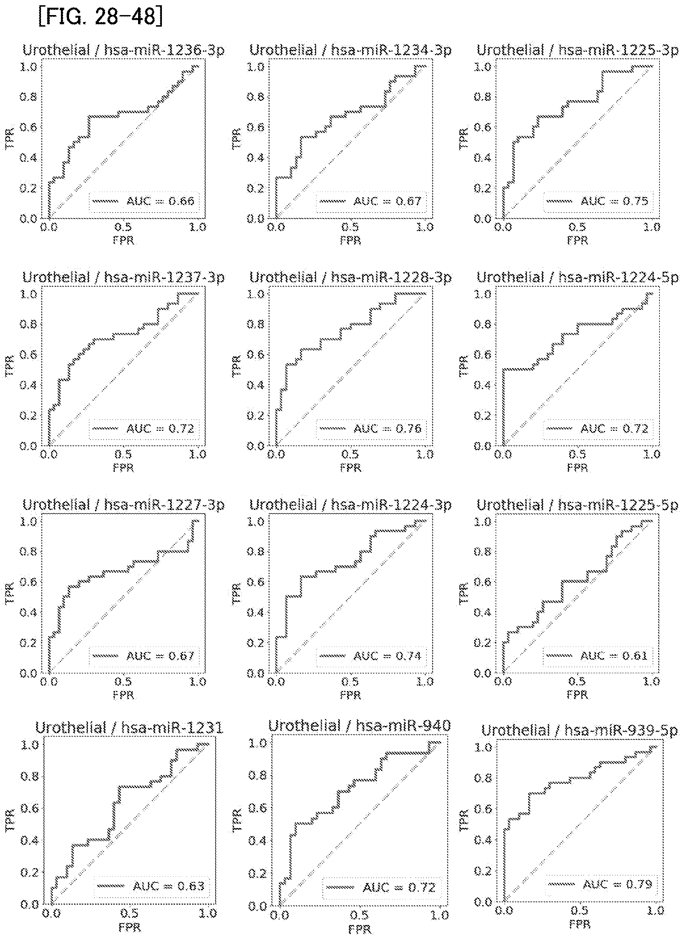

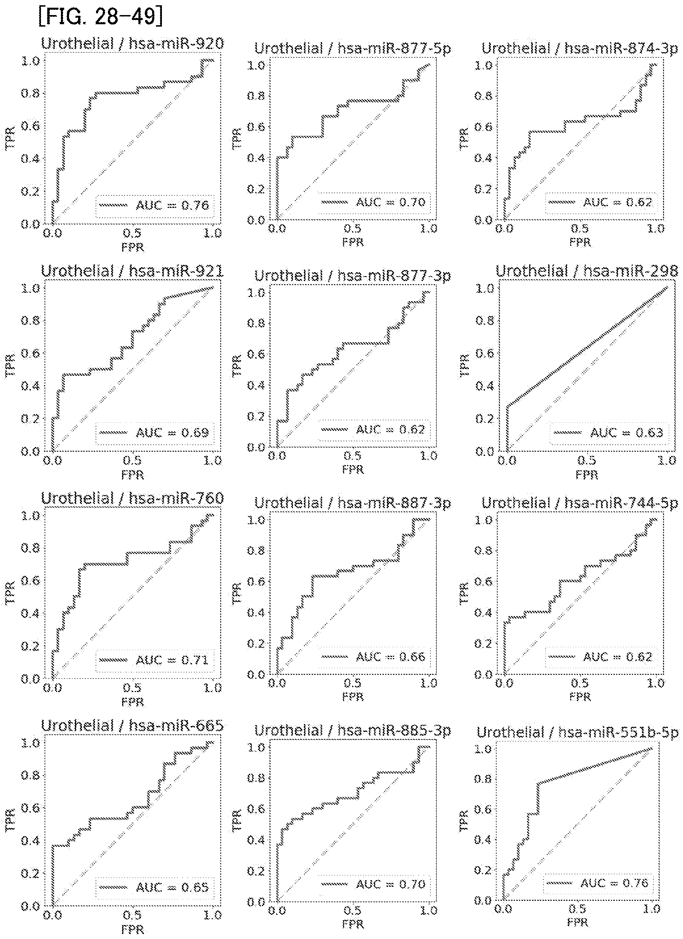

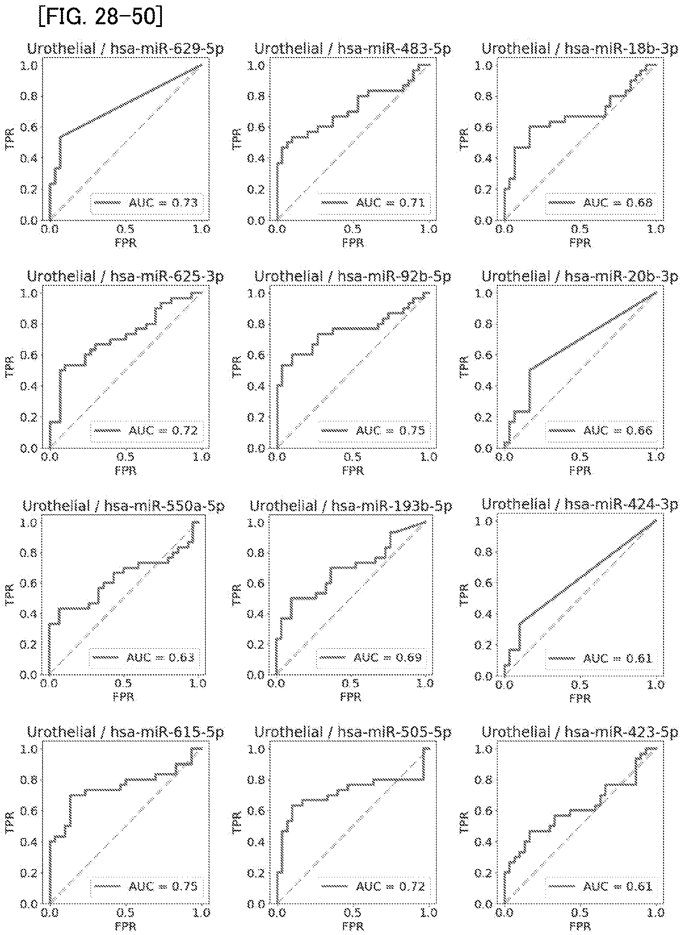

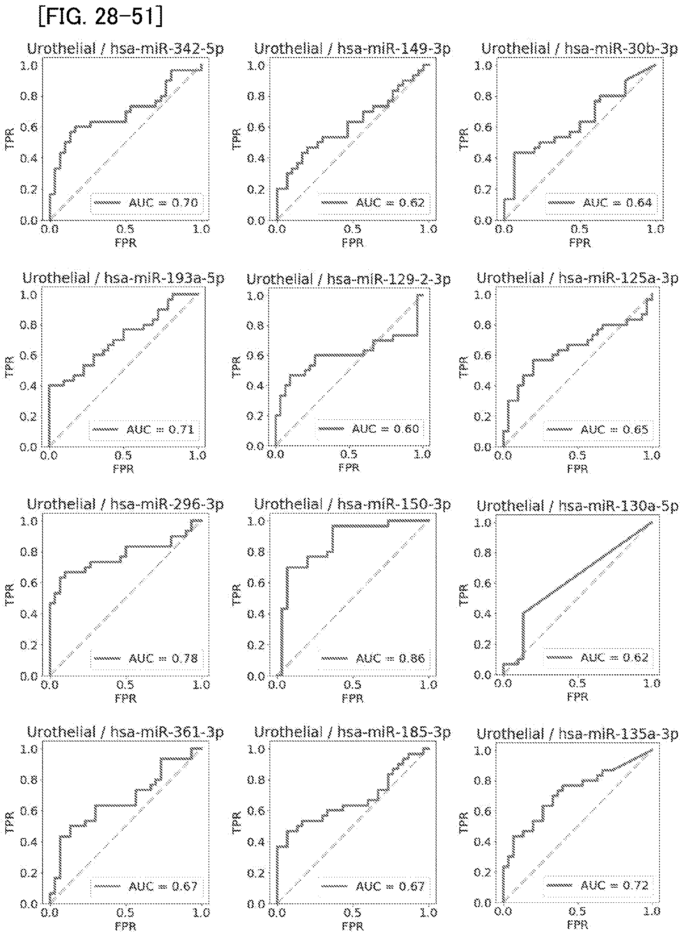

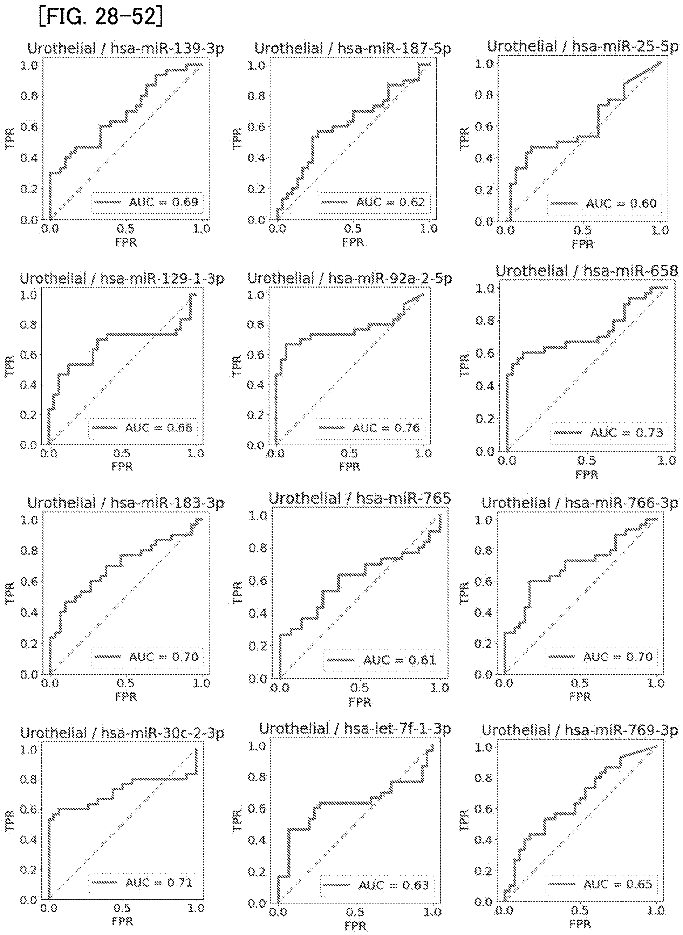

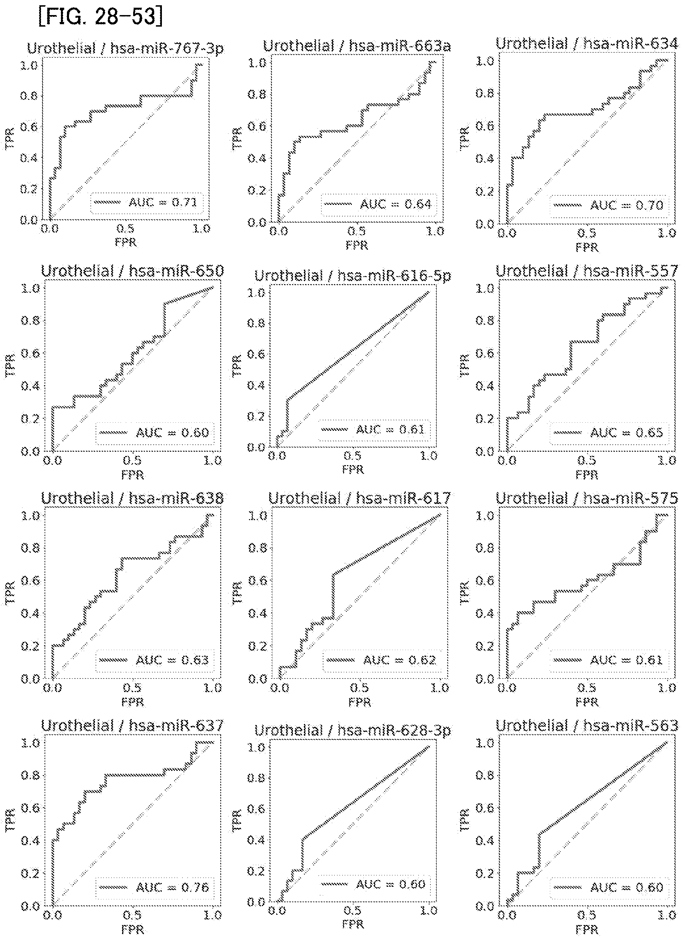

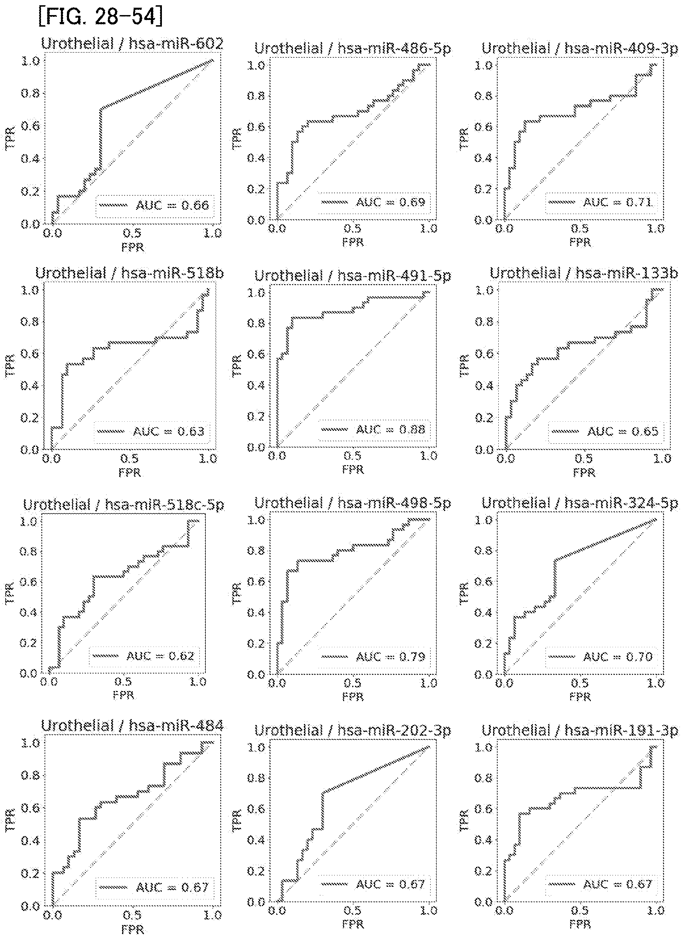

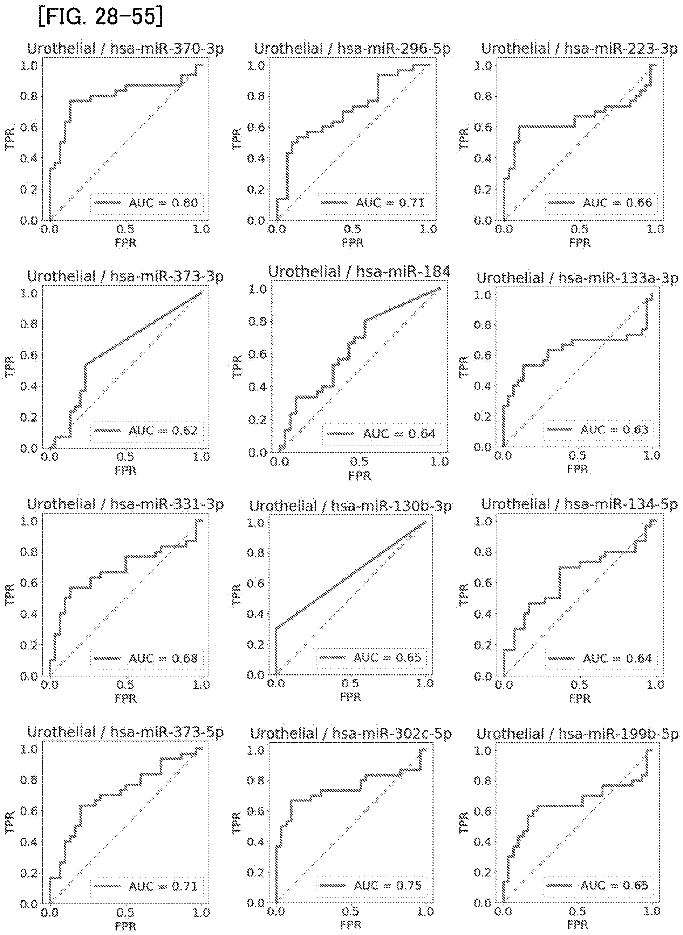

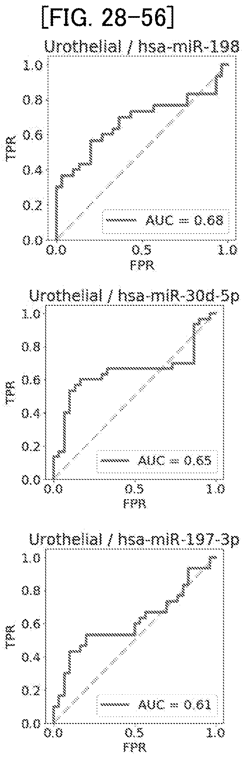

FIGS. 28-1 to 28-56 show ROC-curves for predicting urothelial carcinoma in humans using a single miRNA.

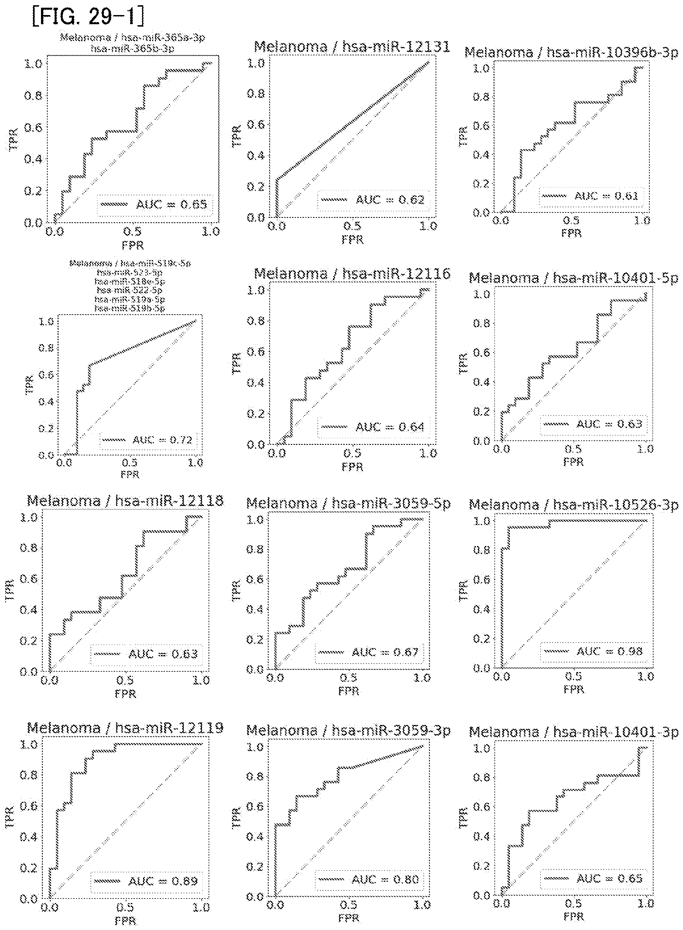

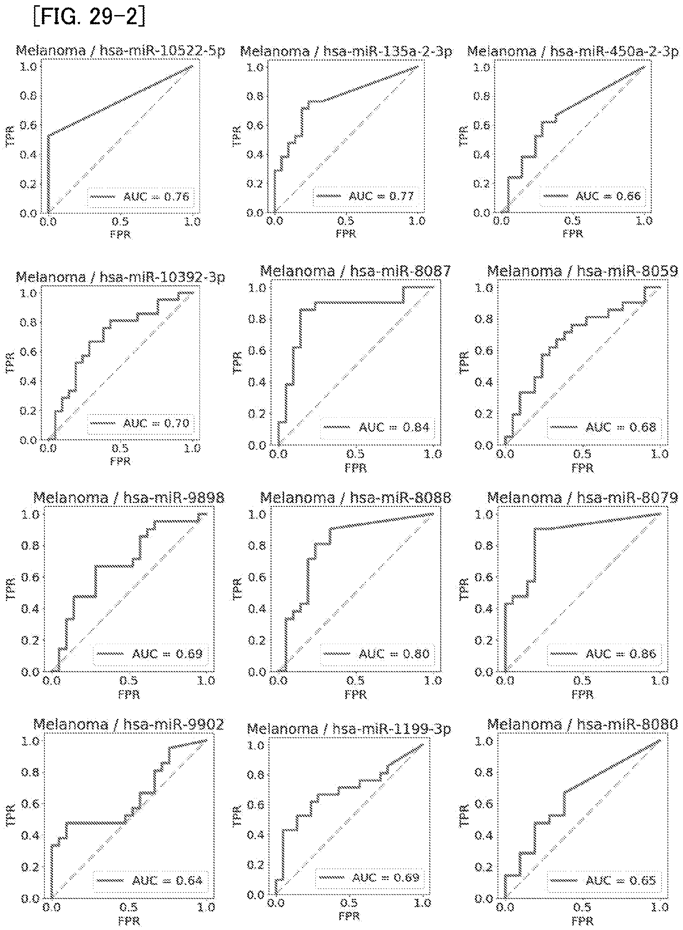

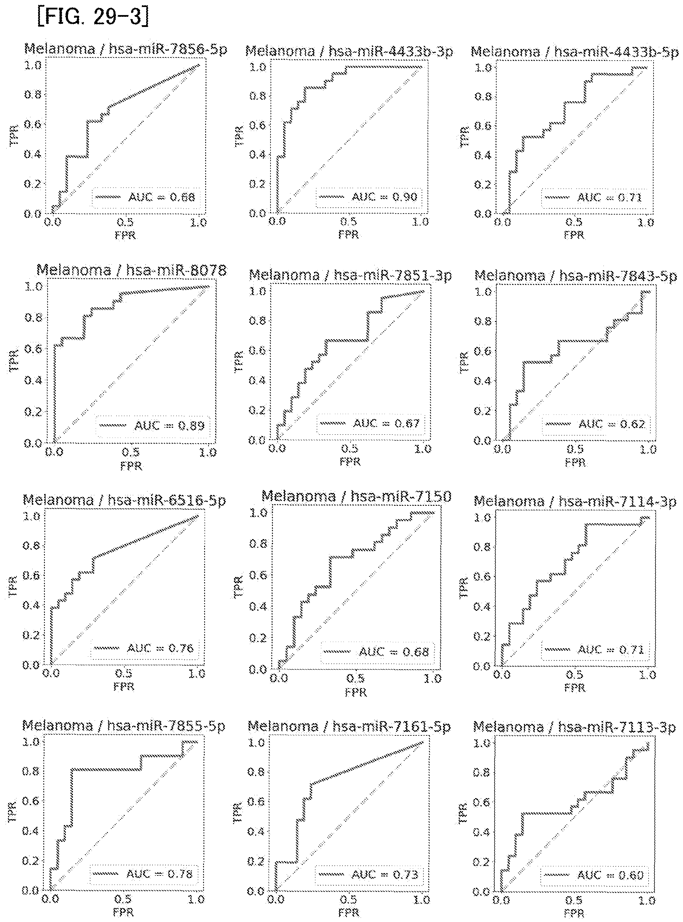

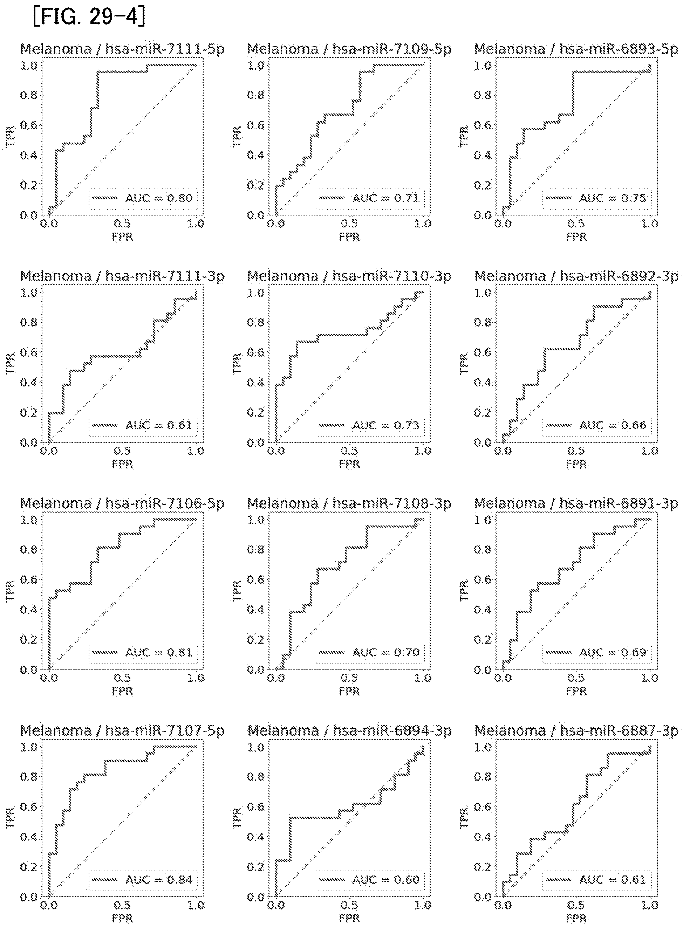

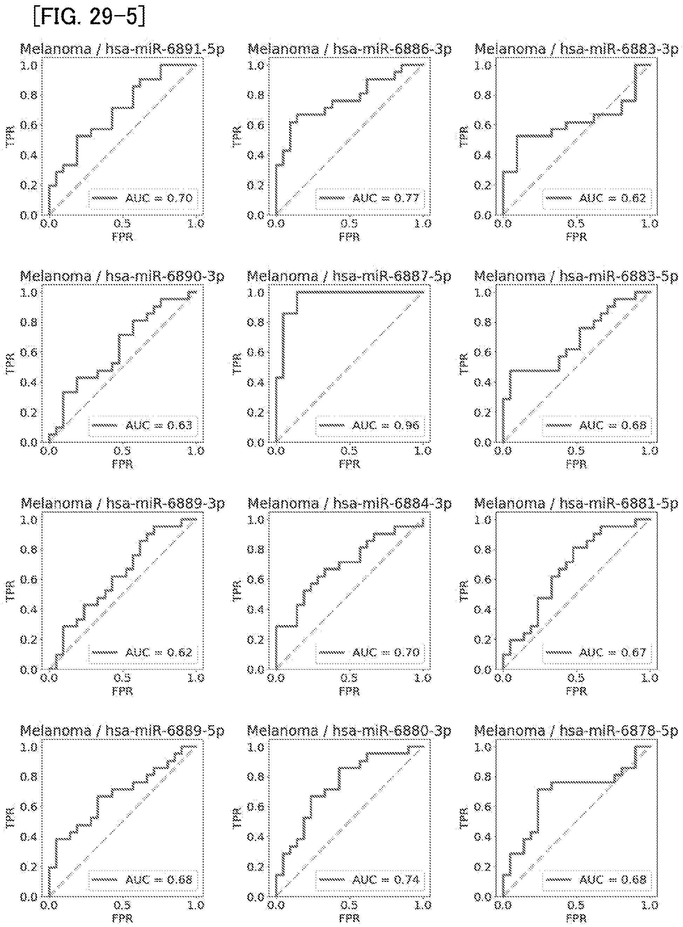

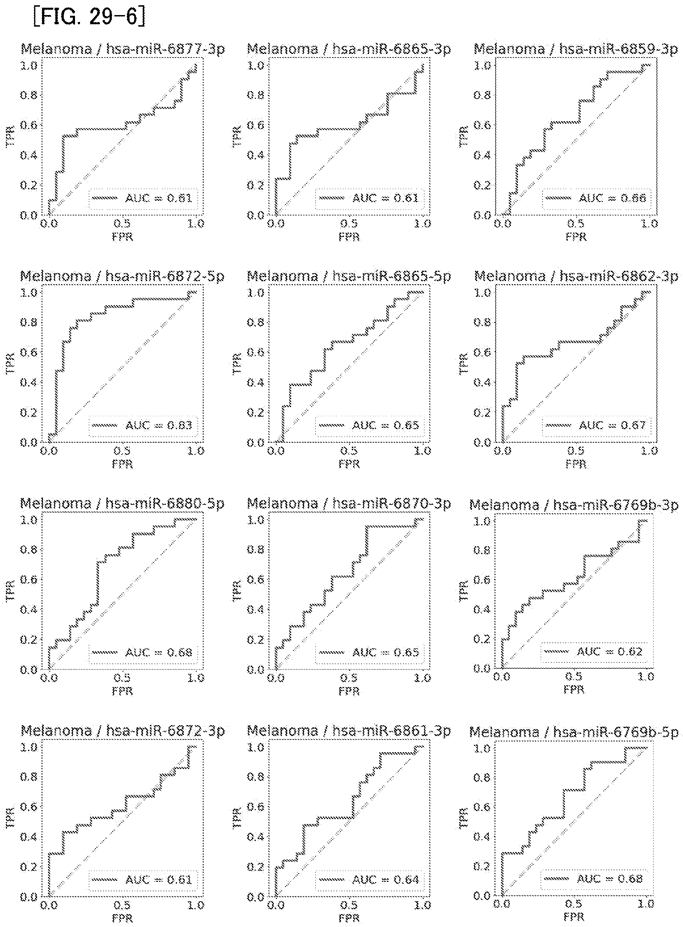

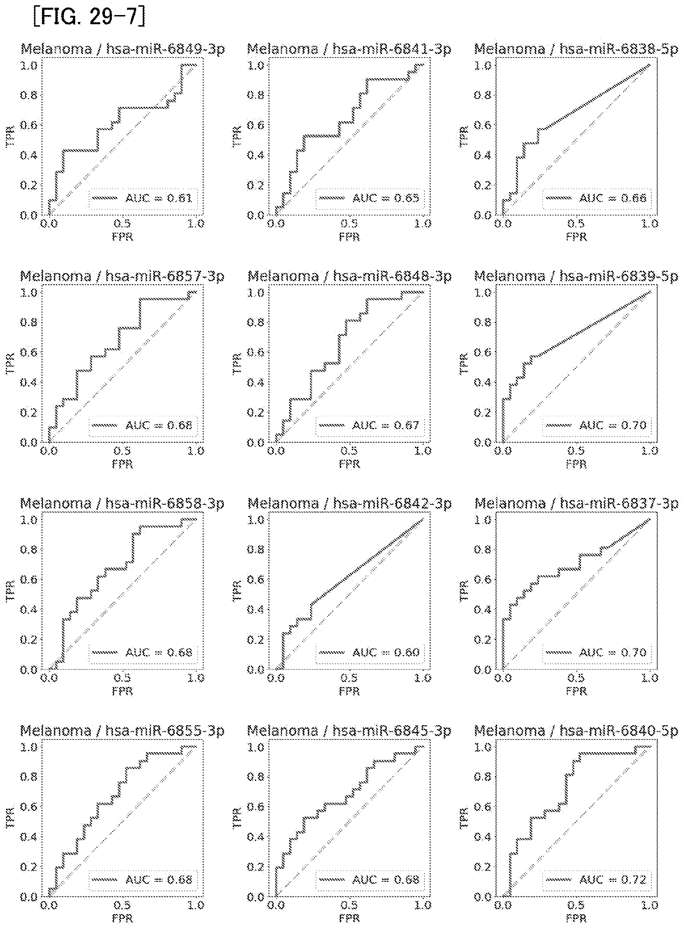

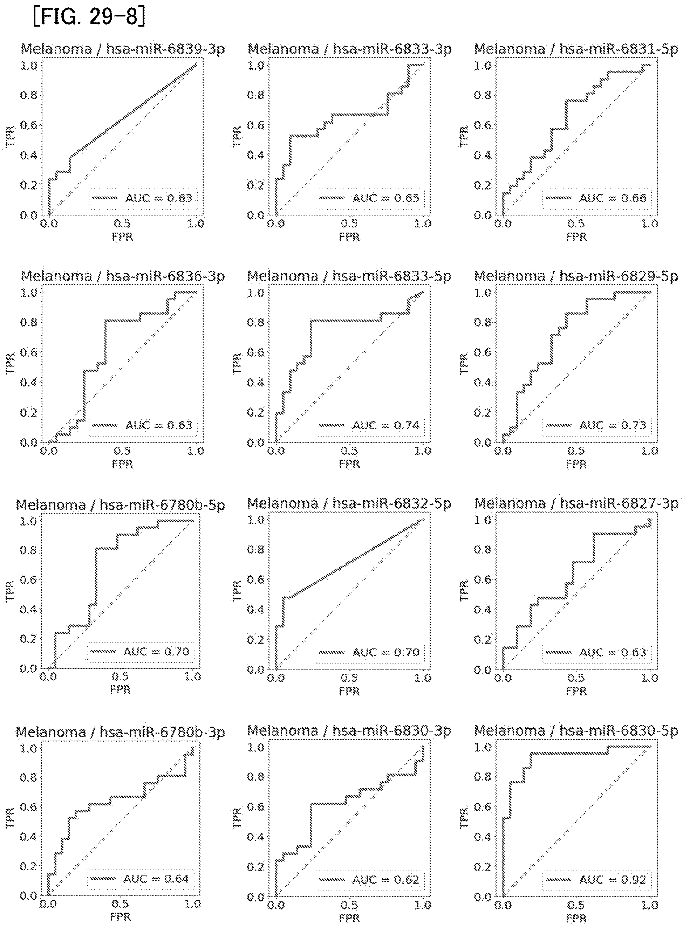

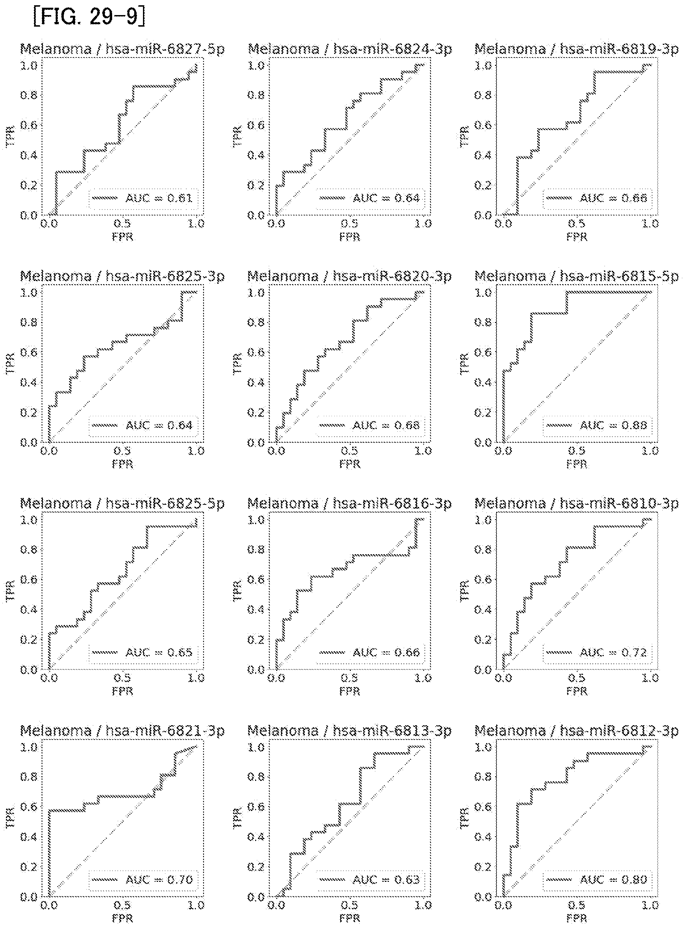

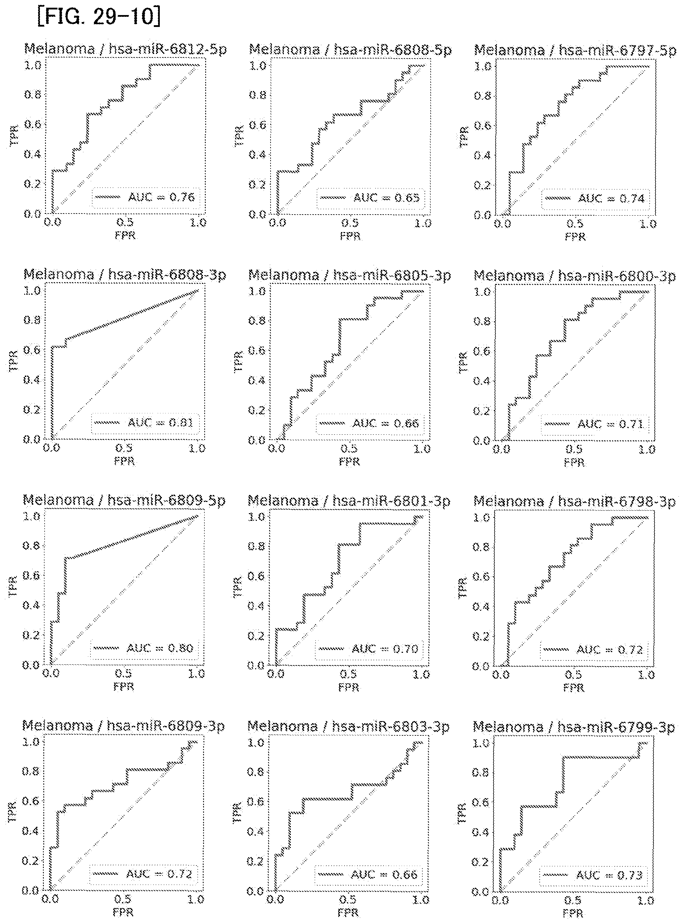

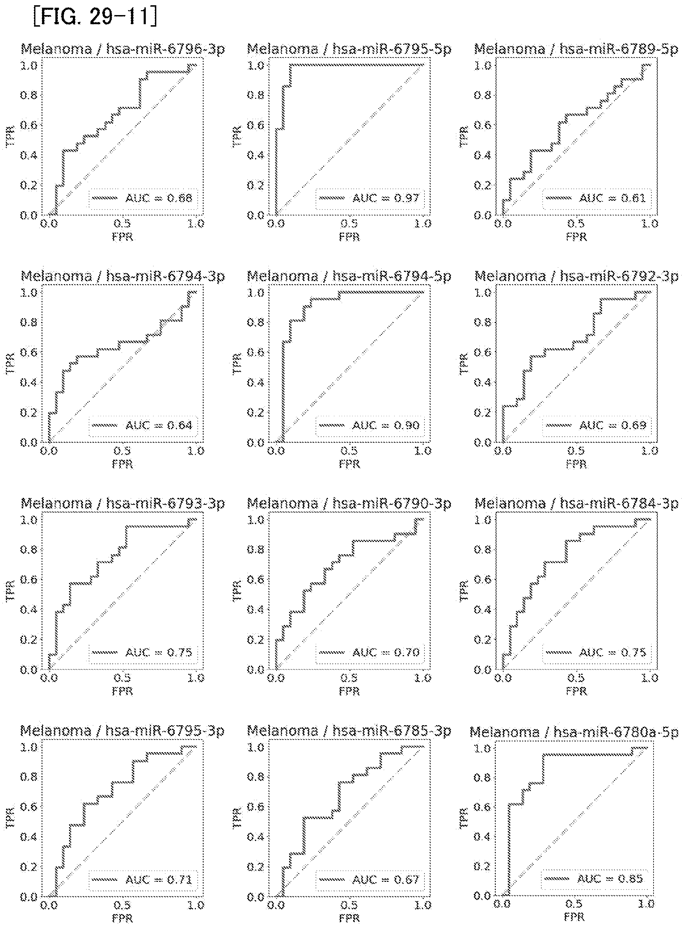

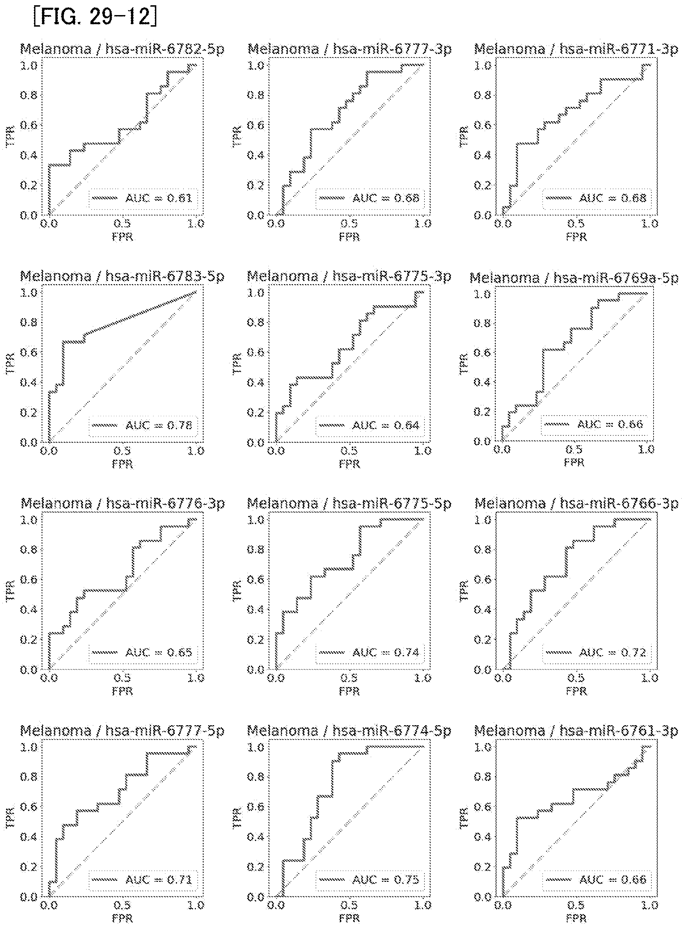

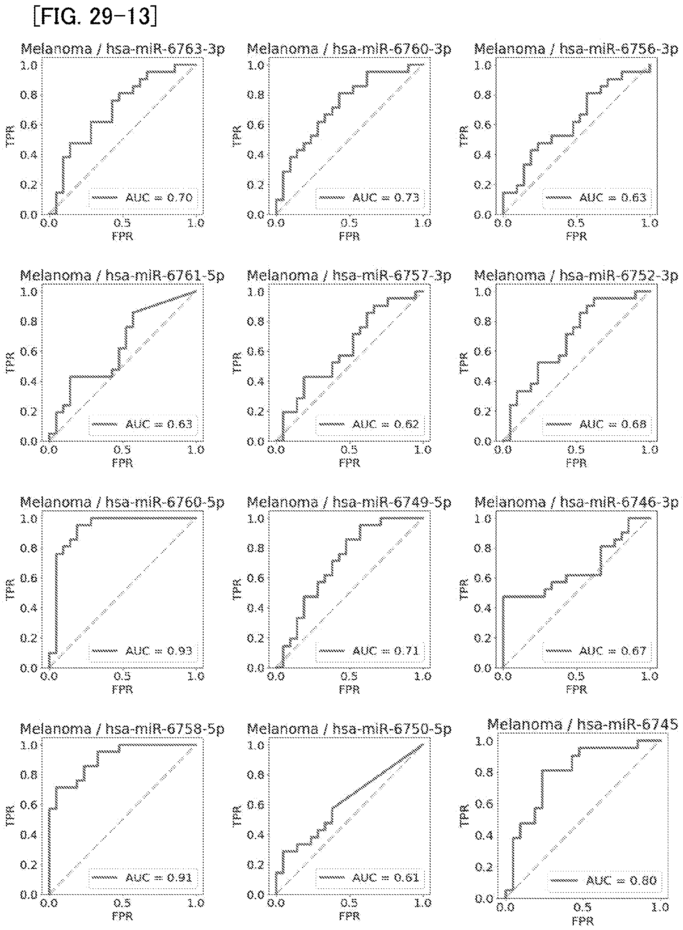

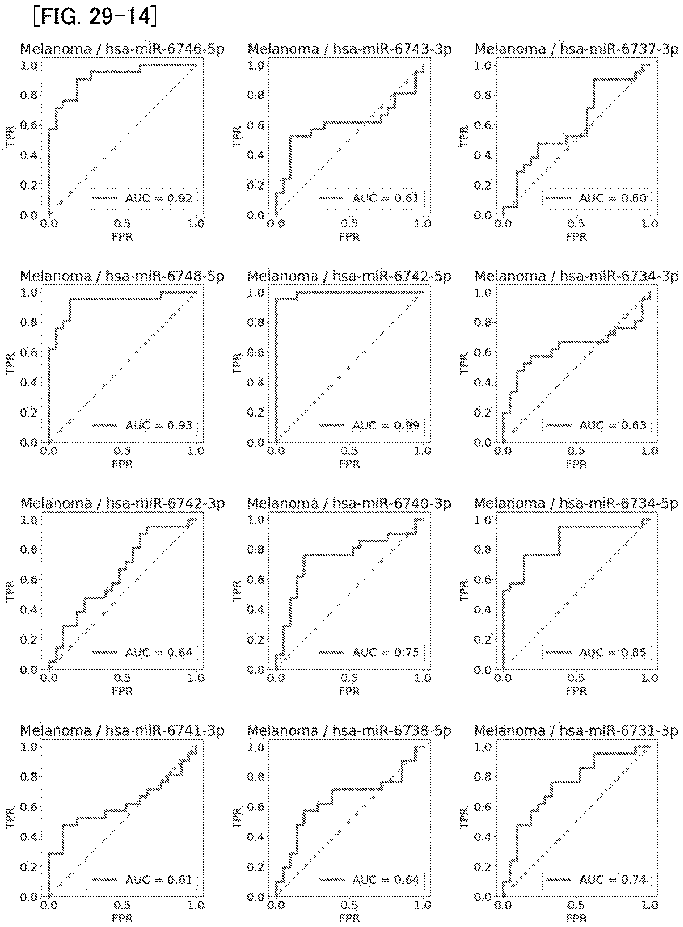

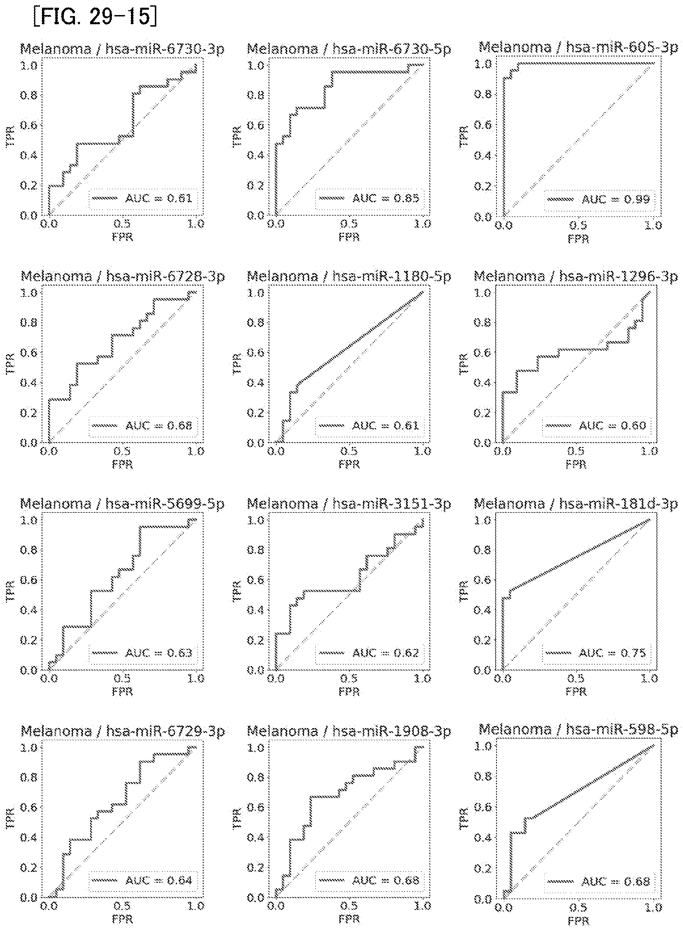

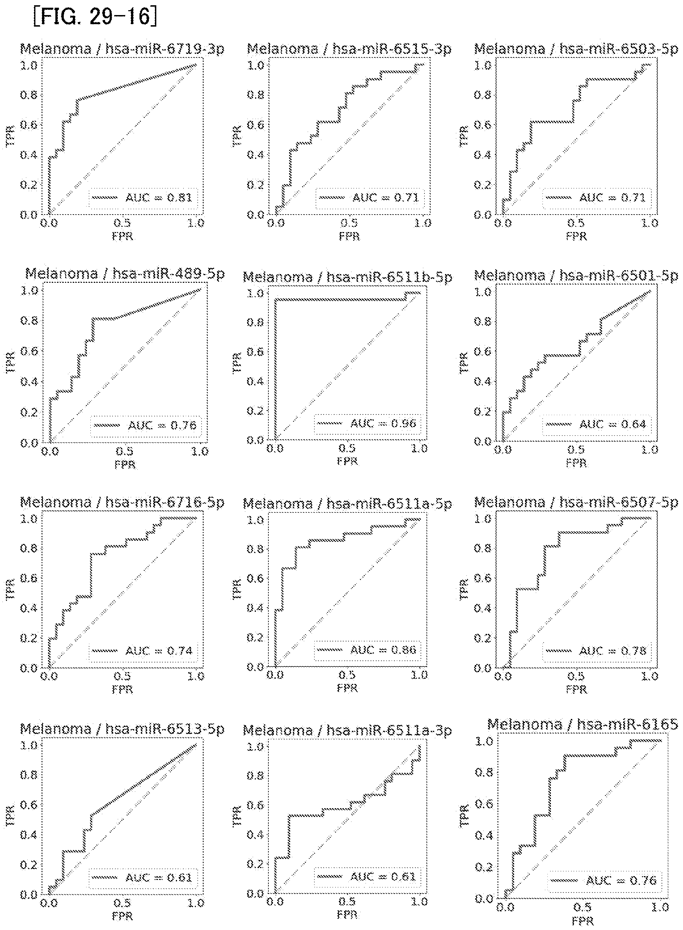

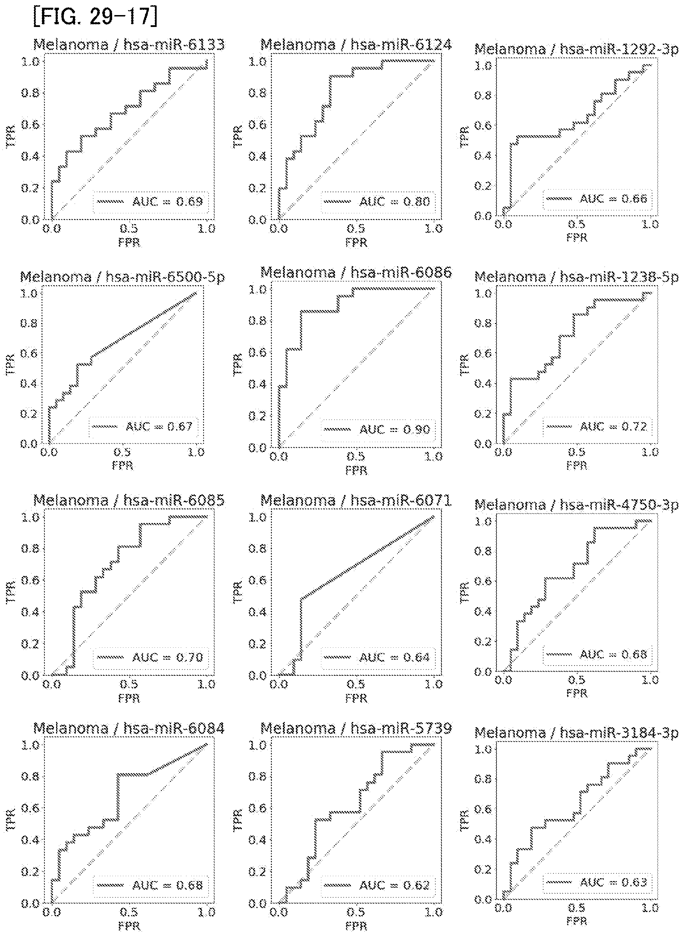

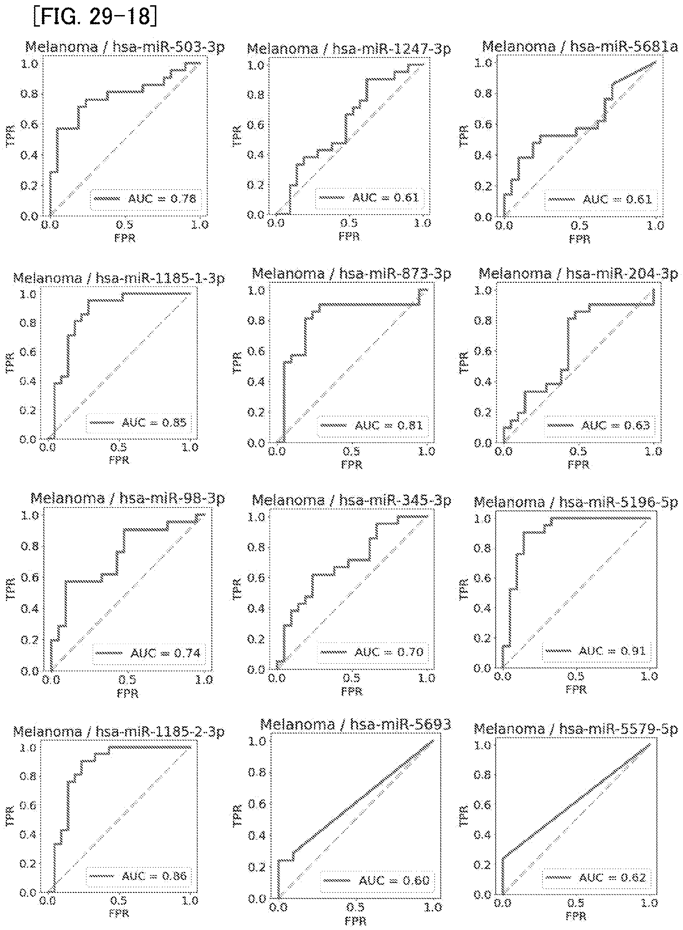

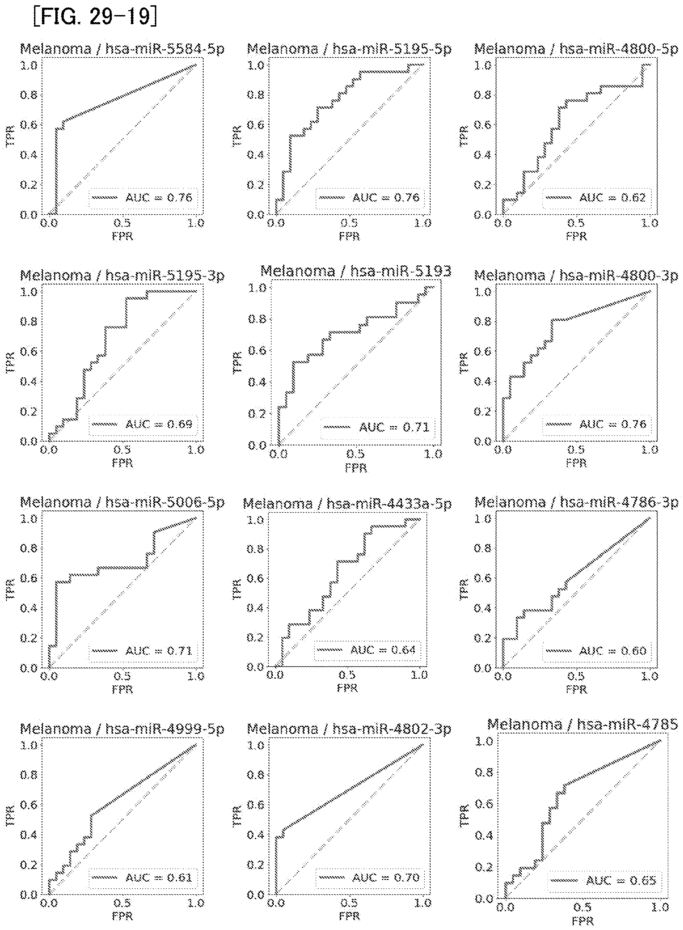

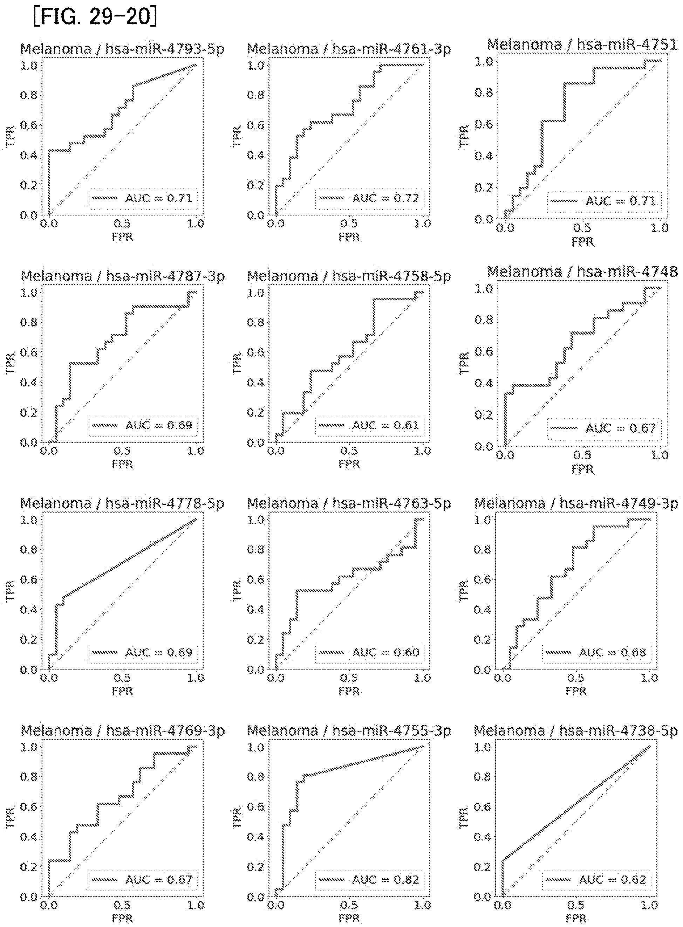

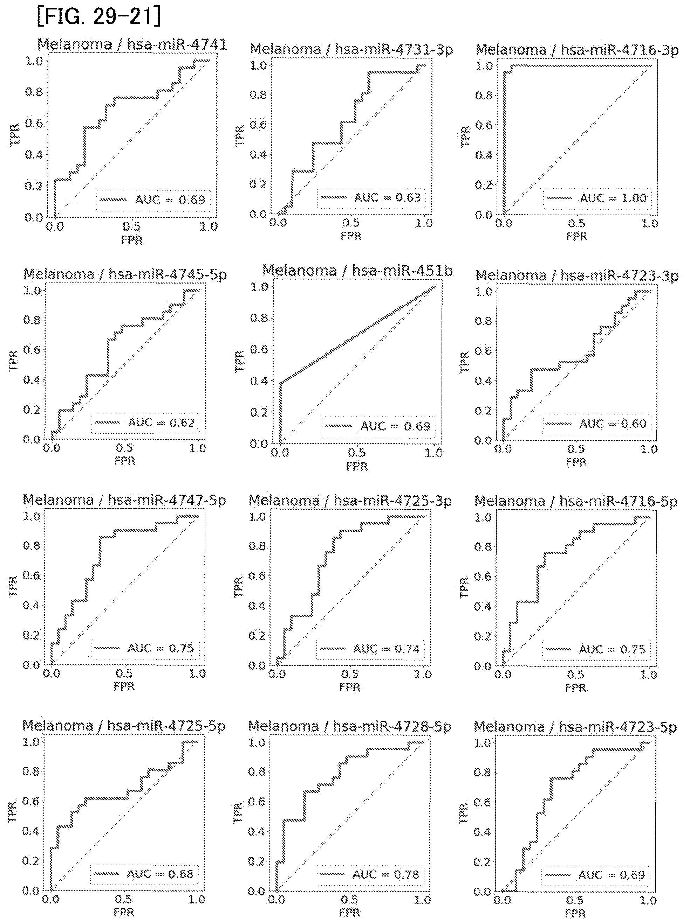

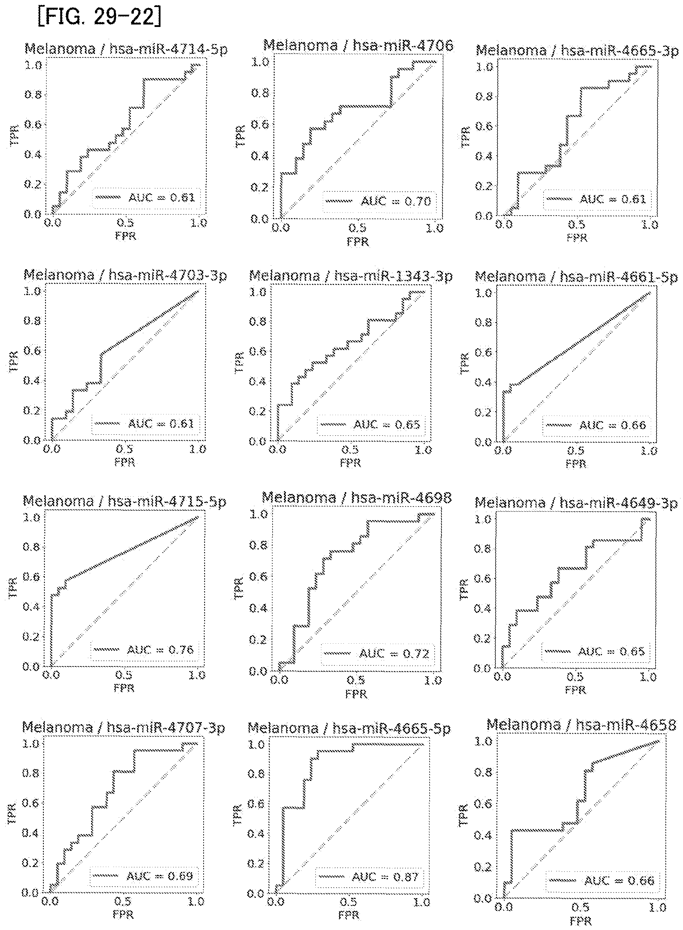

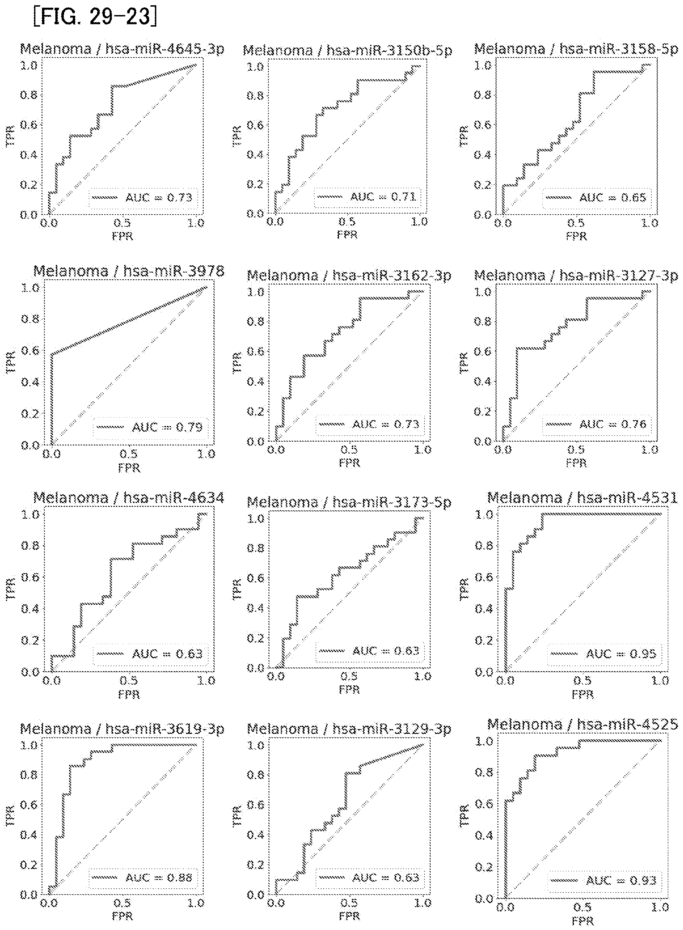

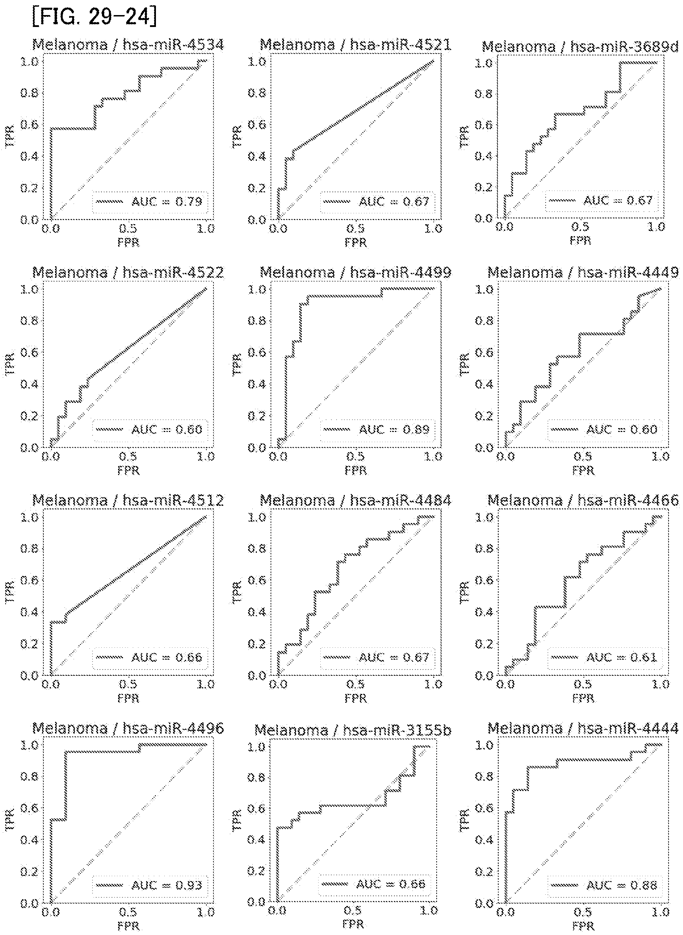

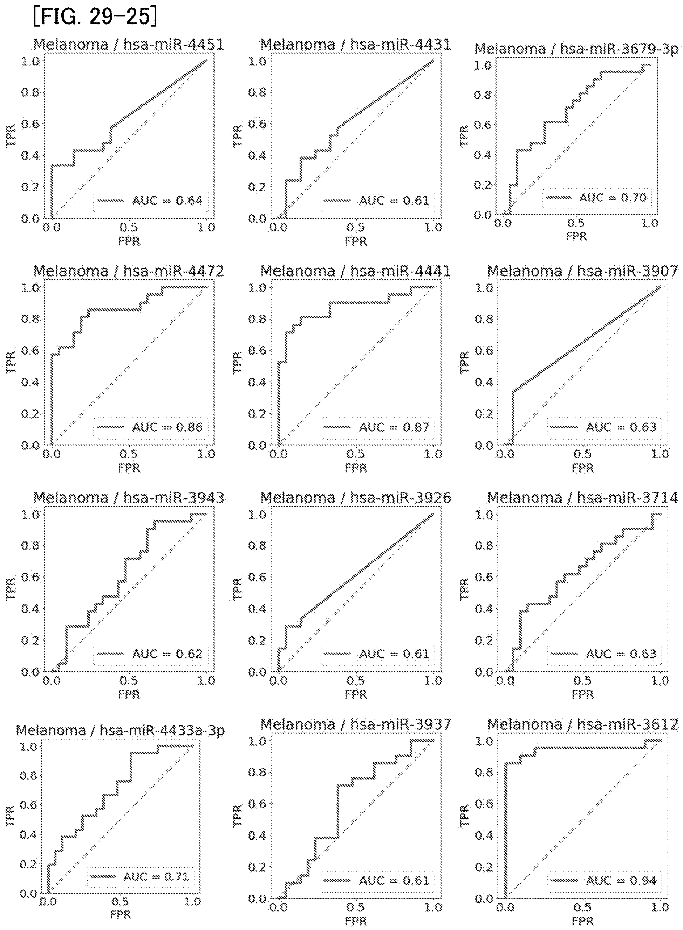

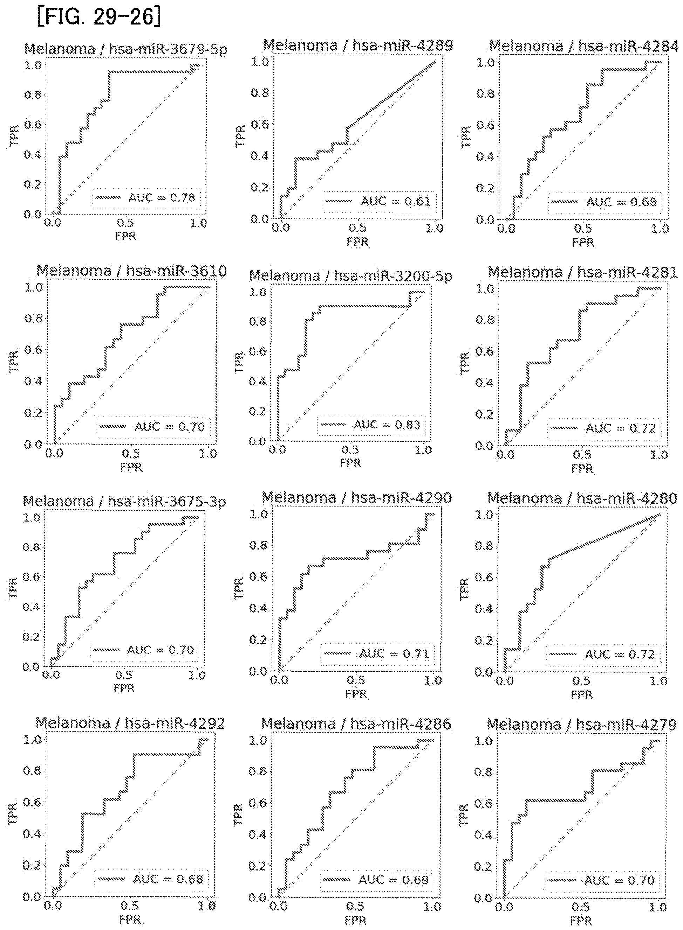

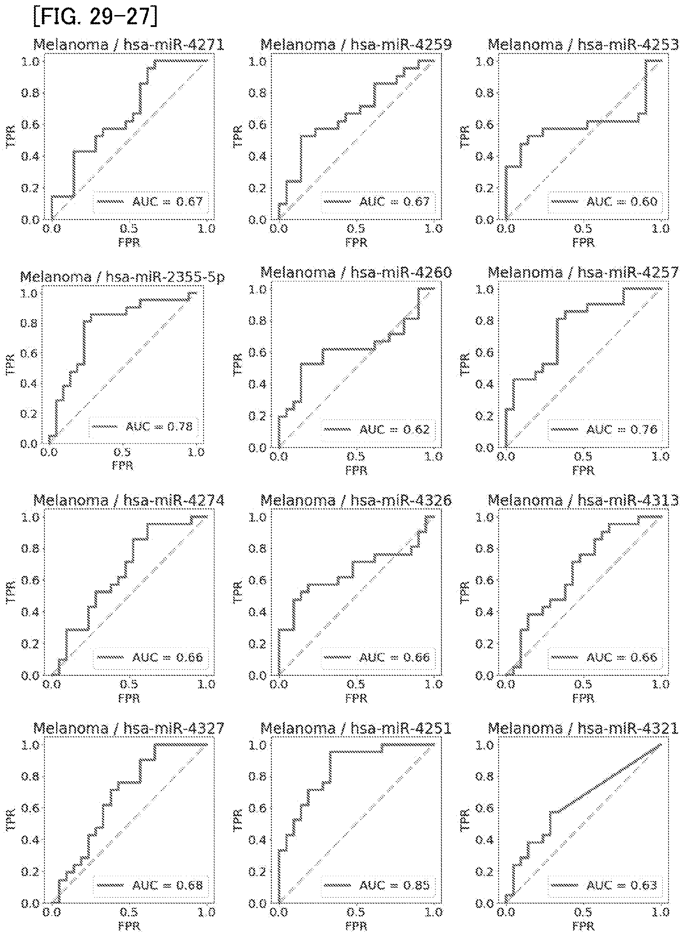

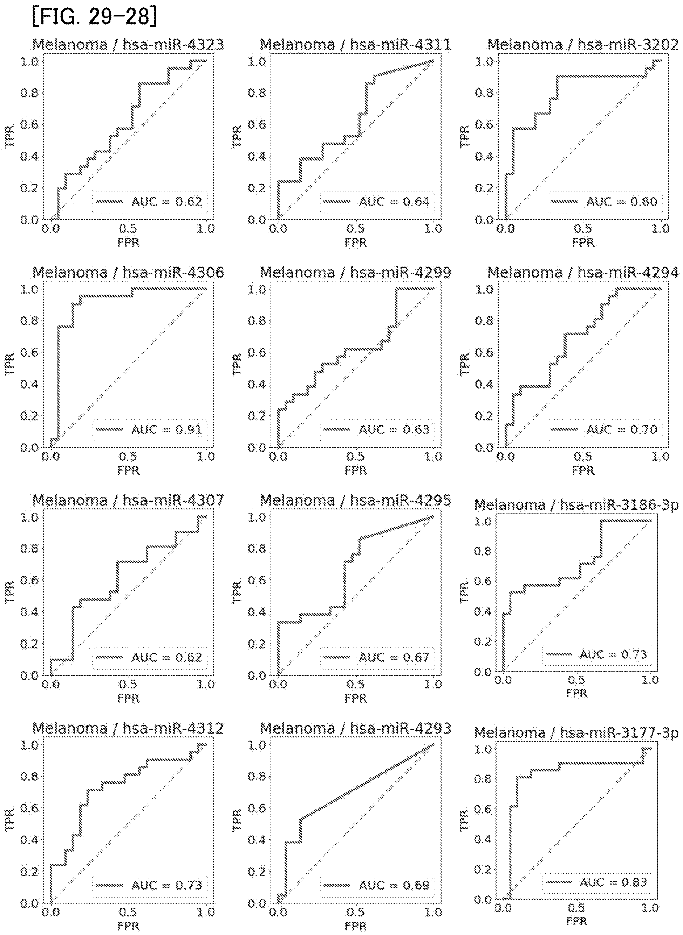

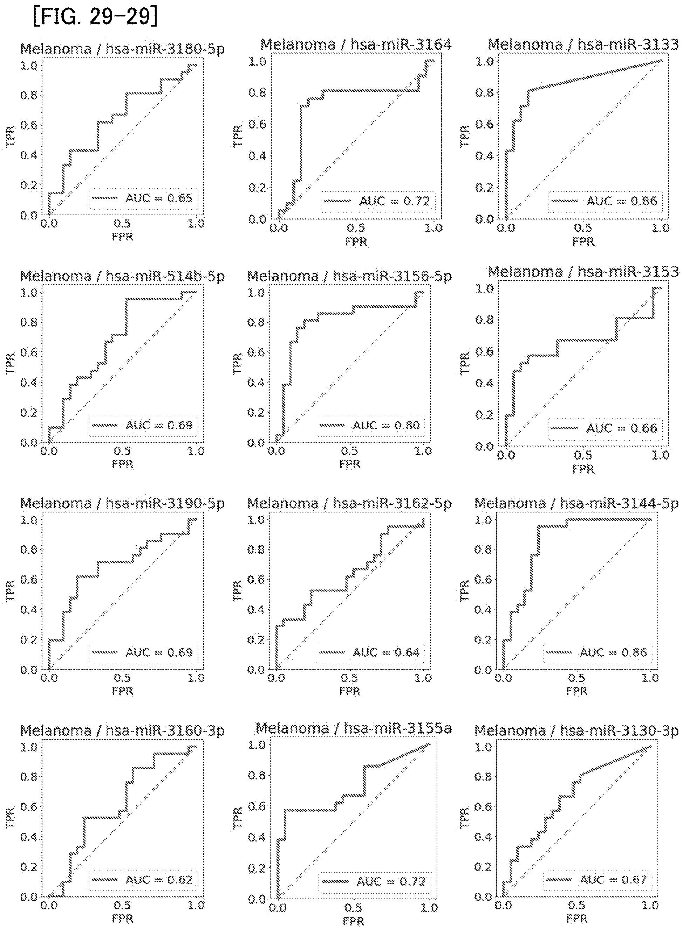

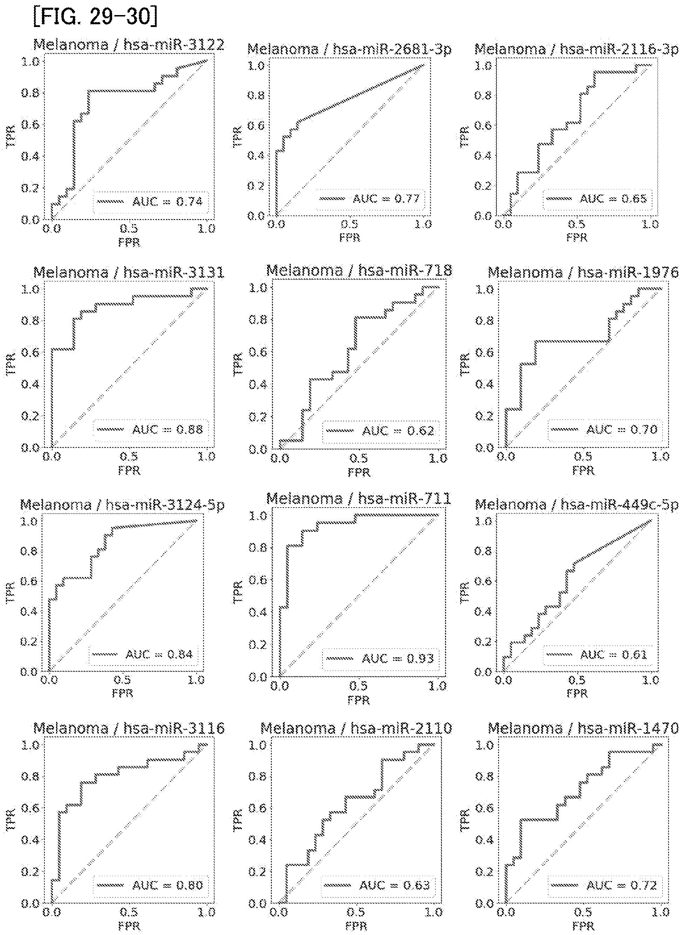

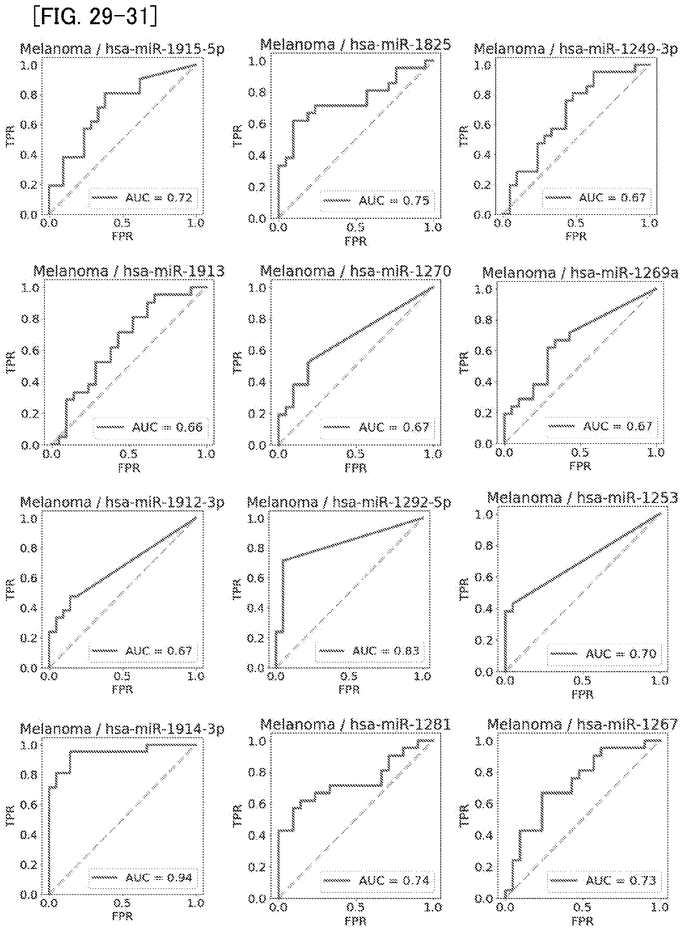

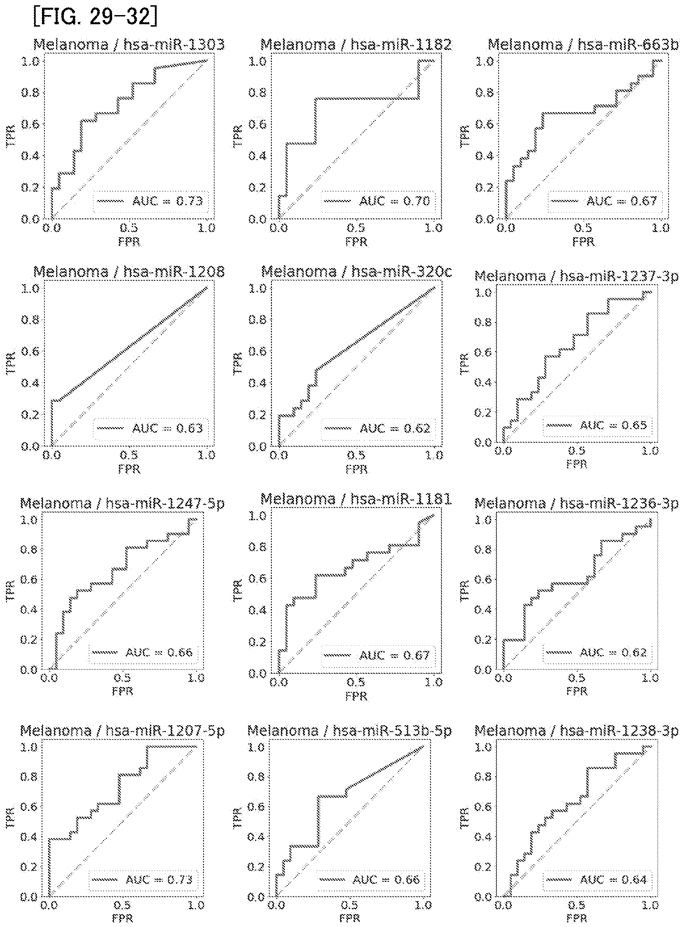

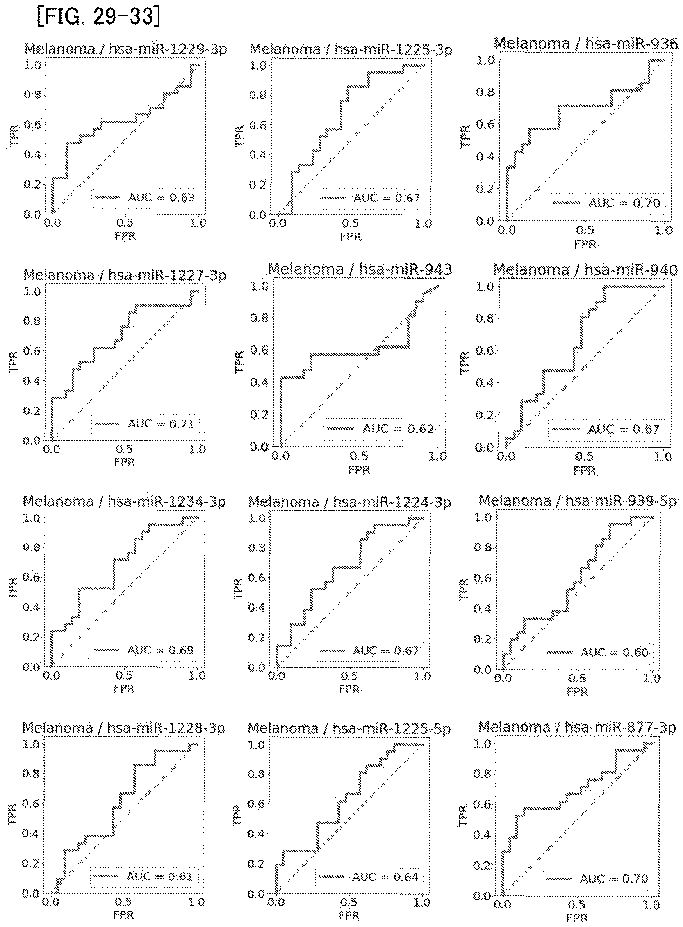

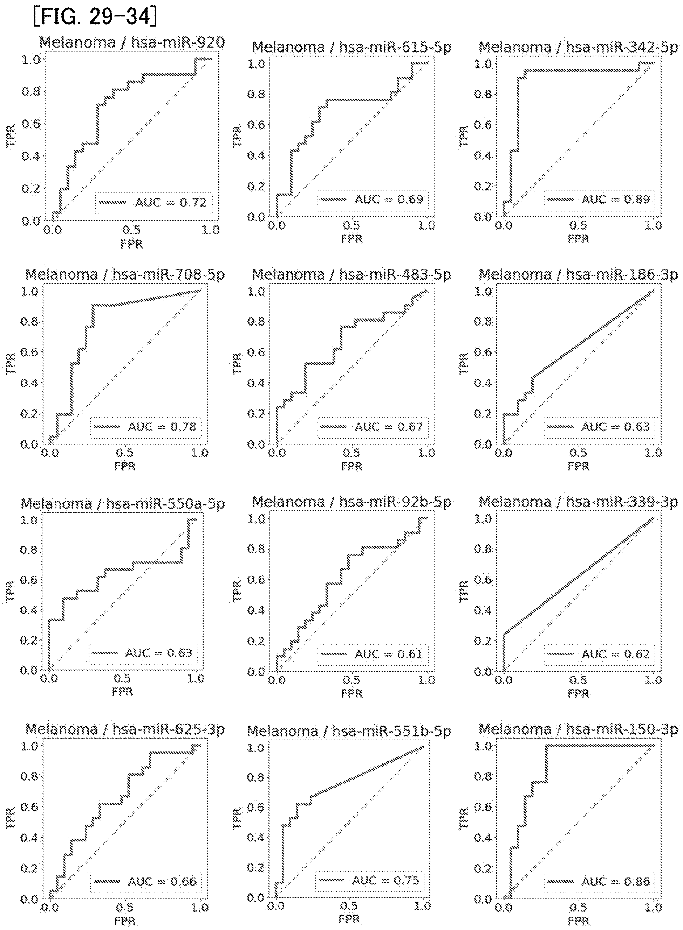

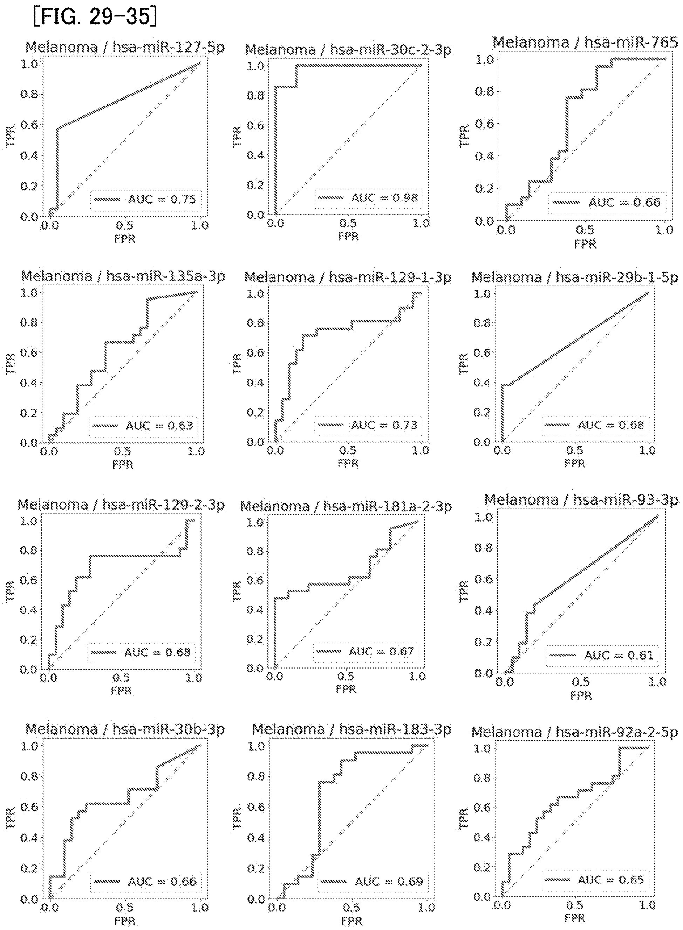

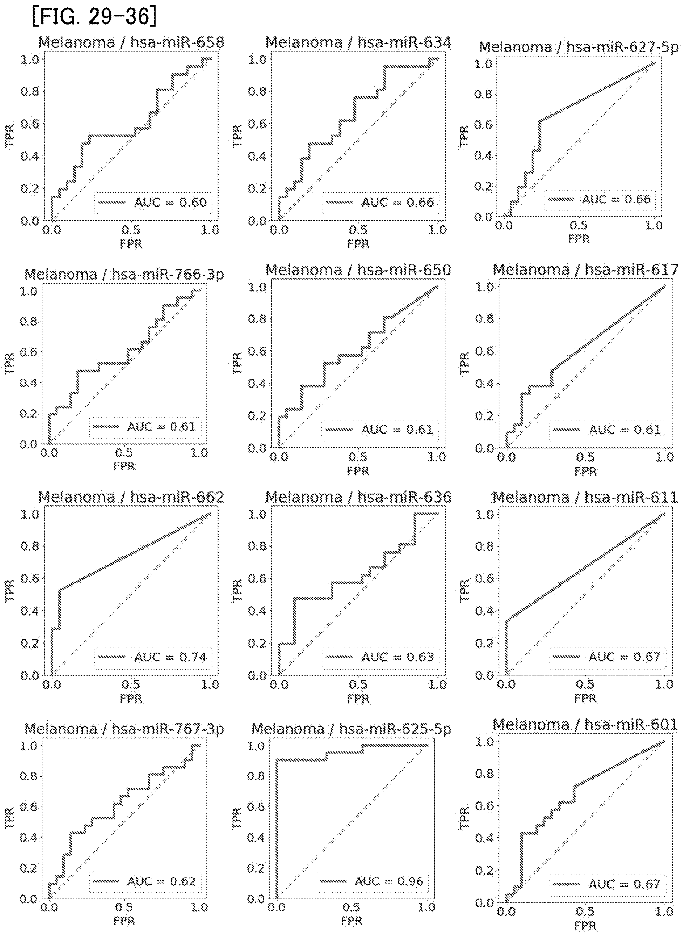

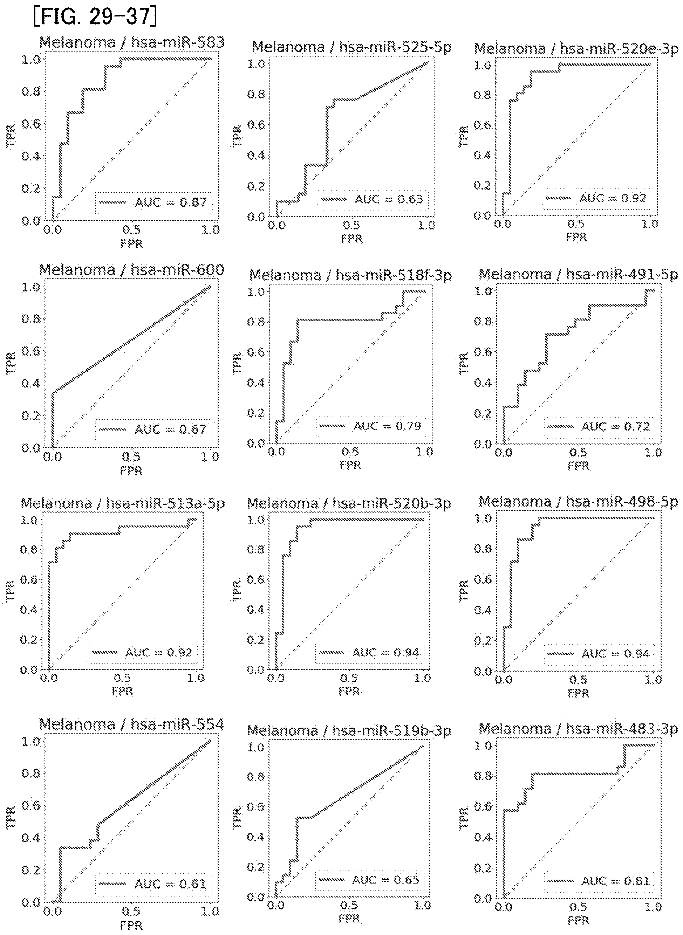

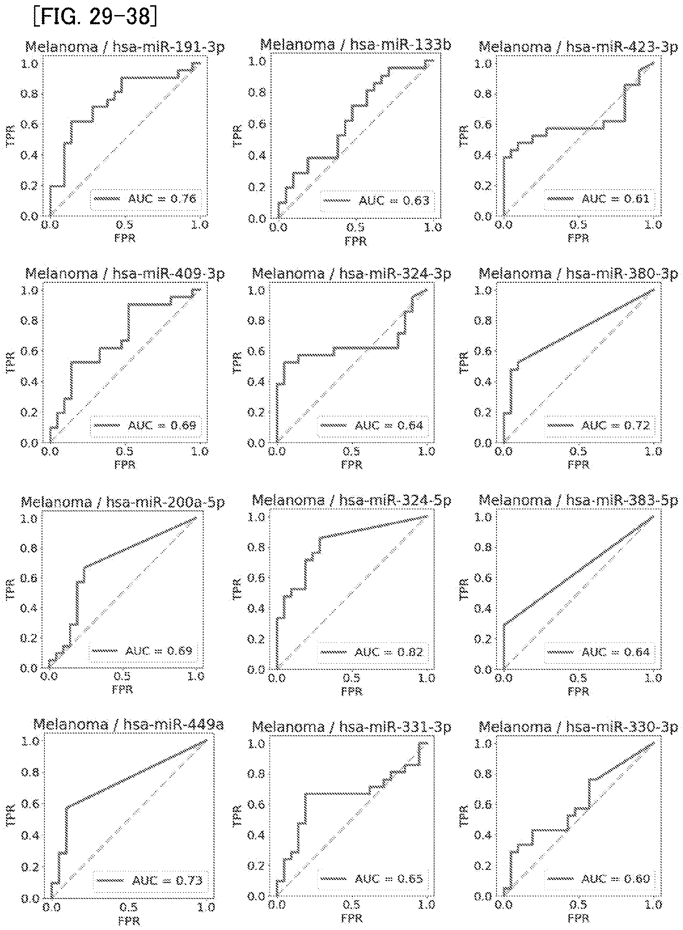

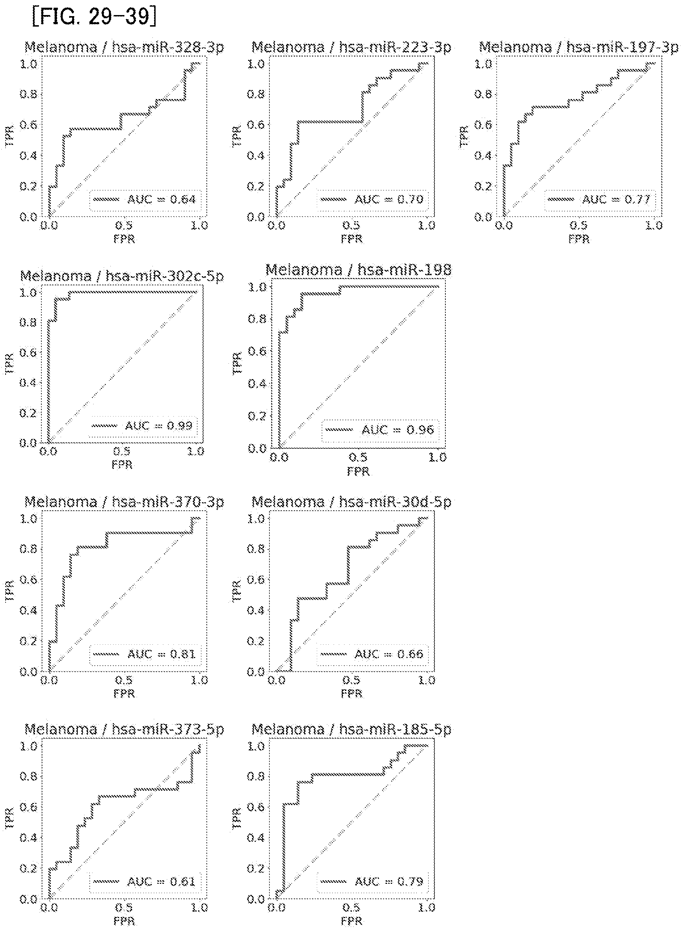

FIGS. 29-1 to 29-39 show ROCs curves for predicting melanomas in humans using a single miRNA.

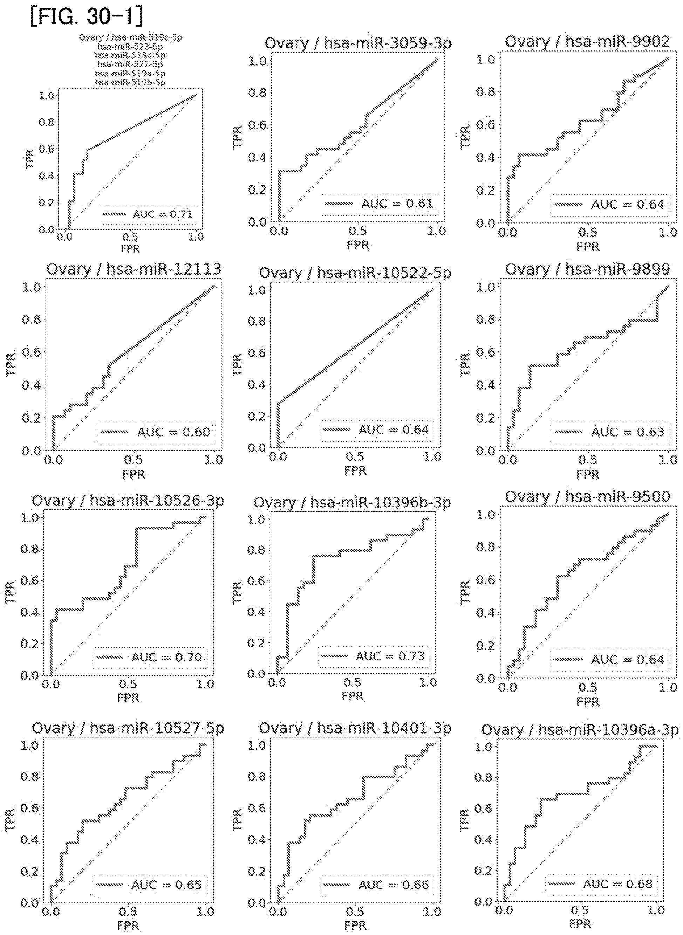

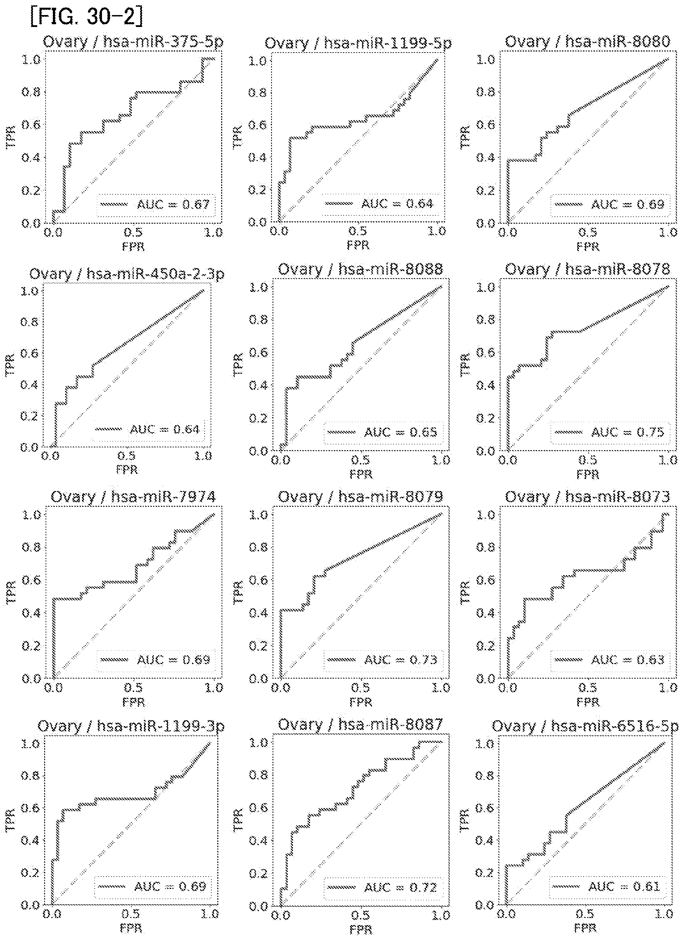

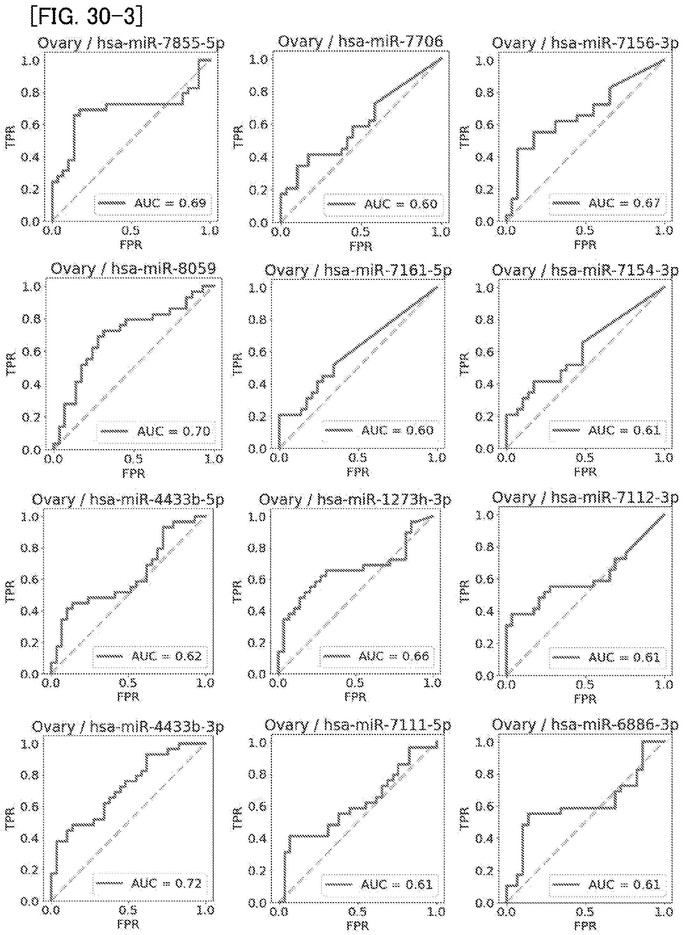

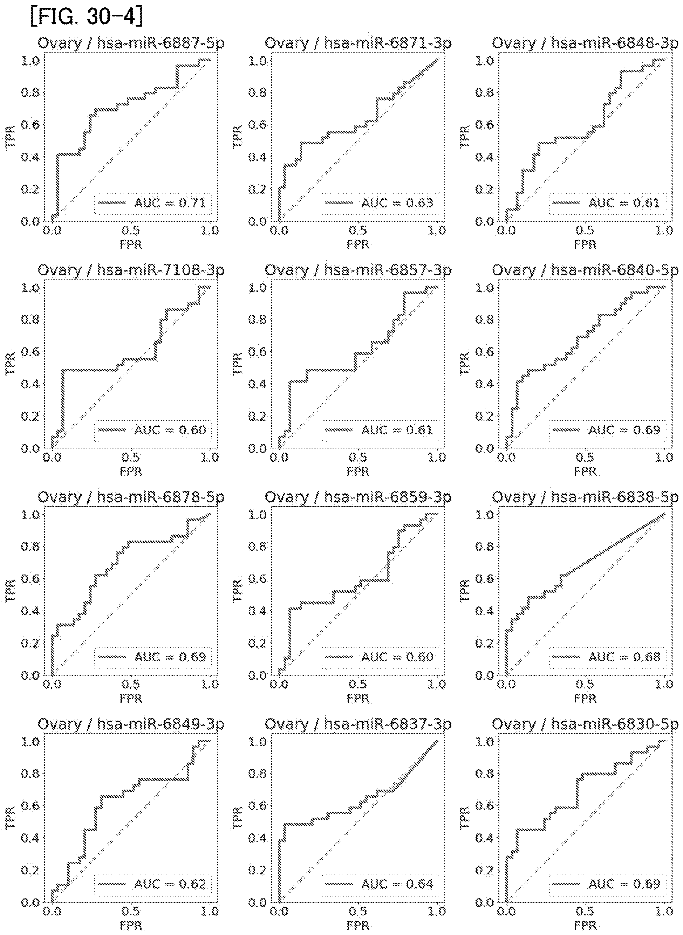

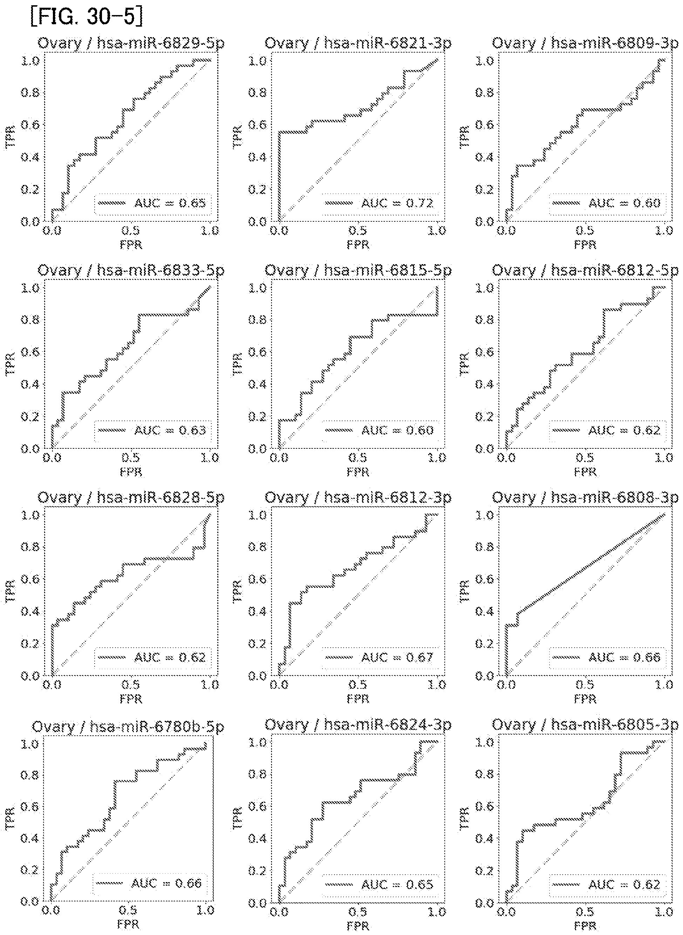

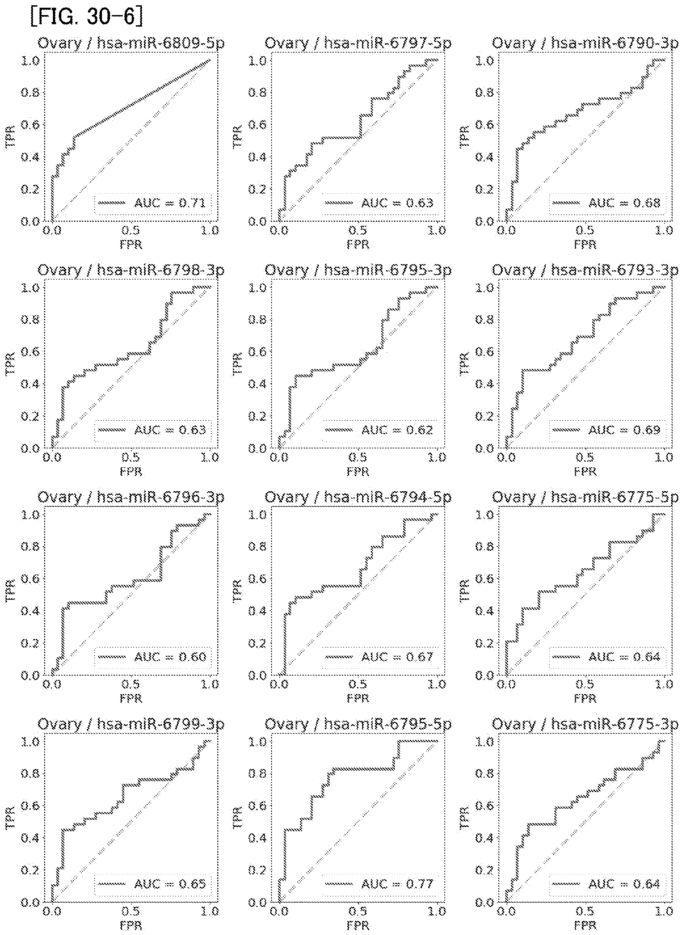

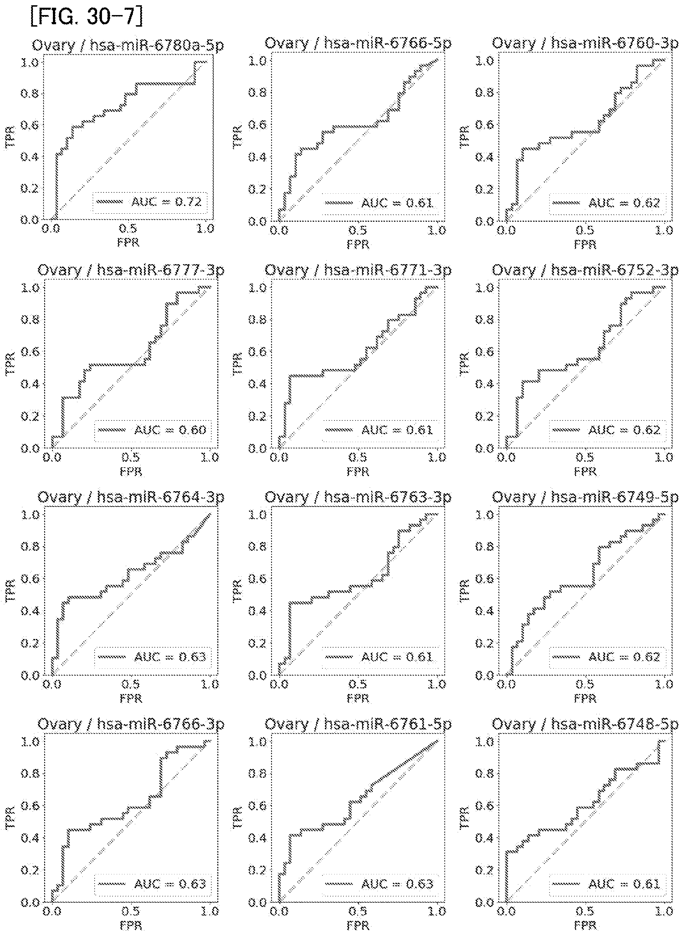

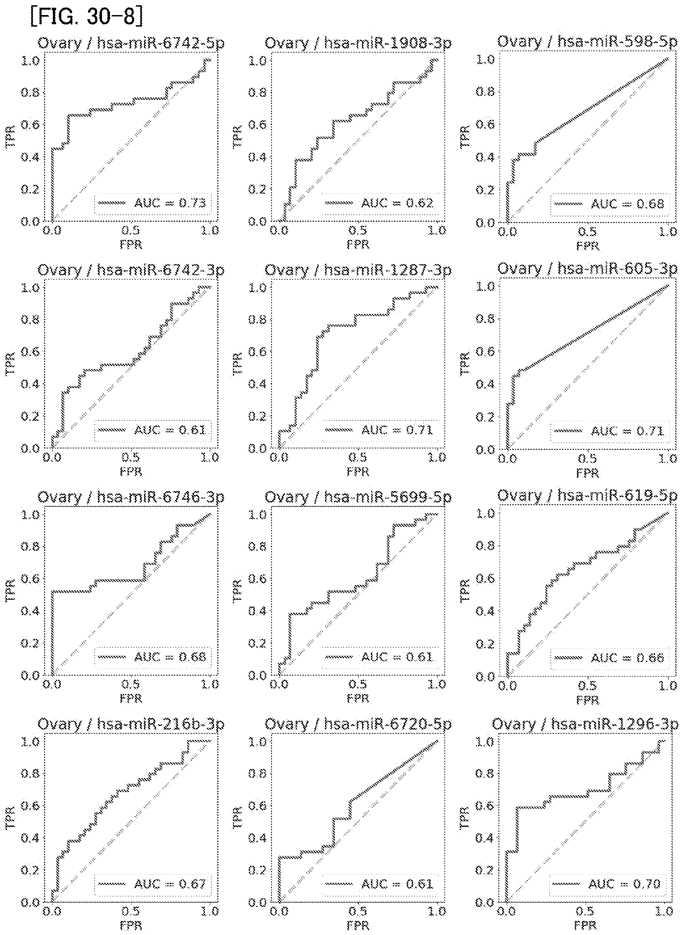

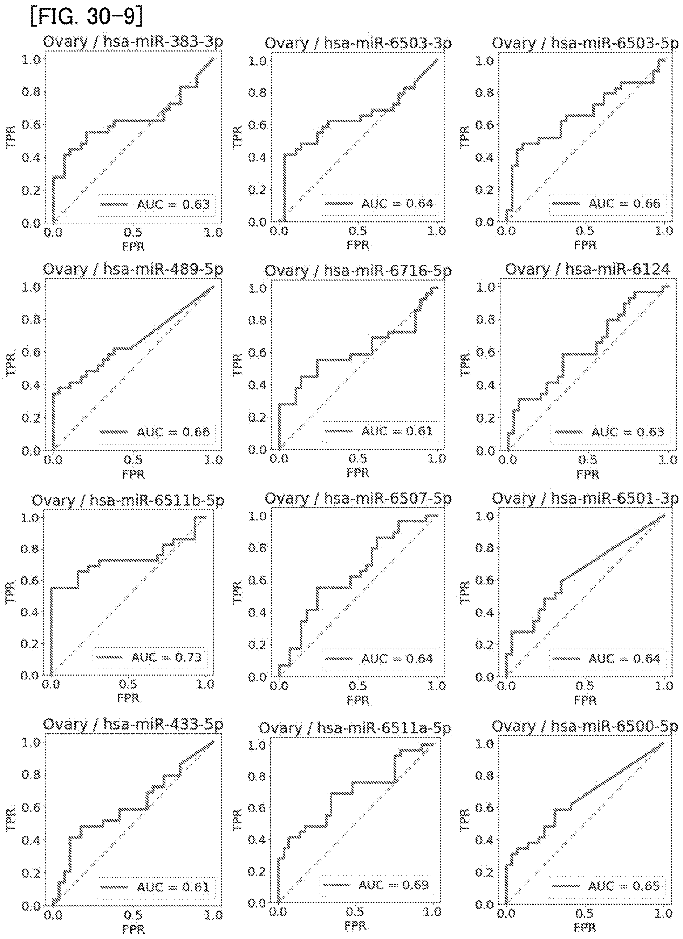

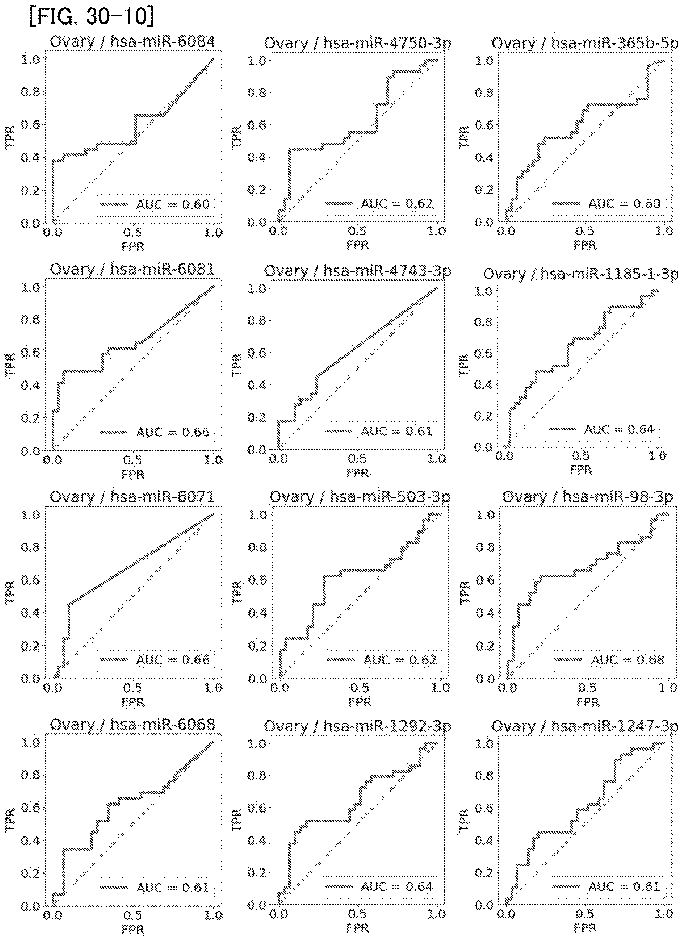

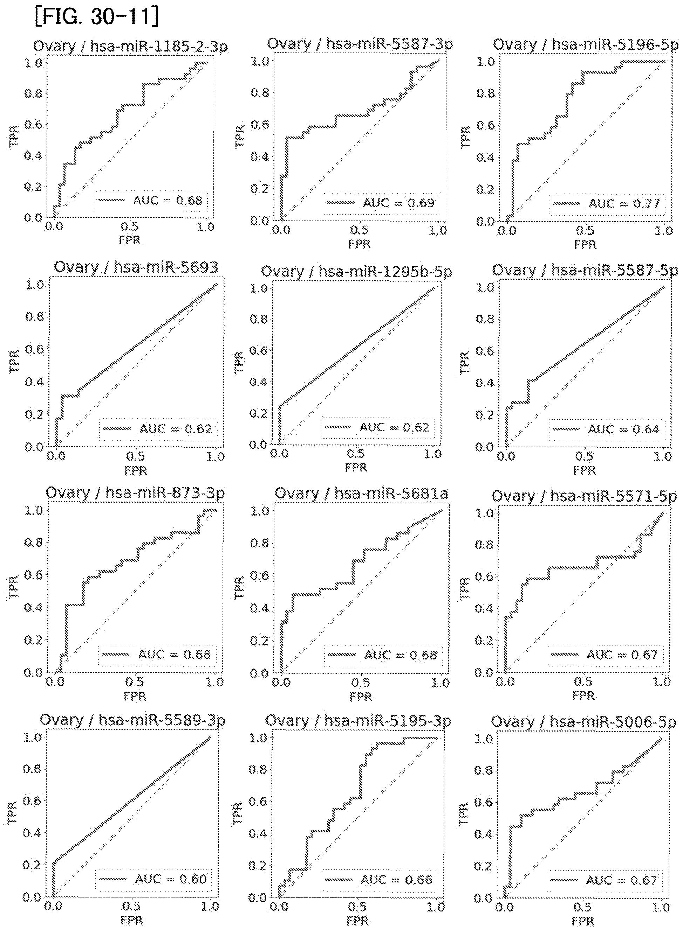

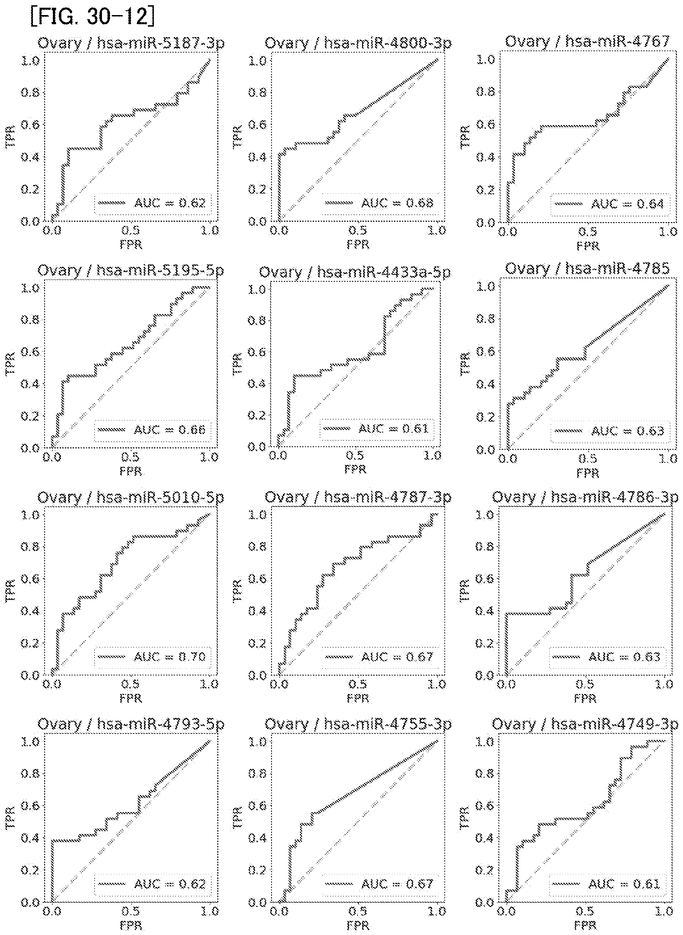

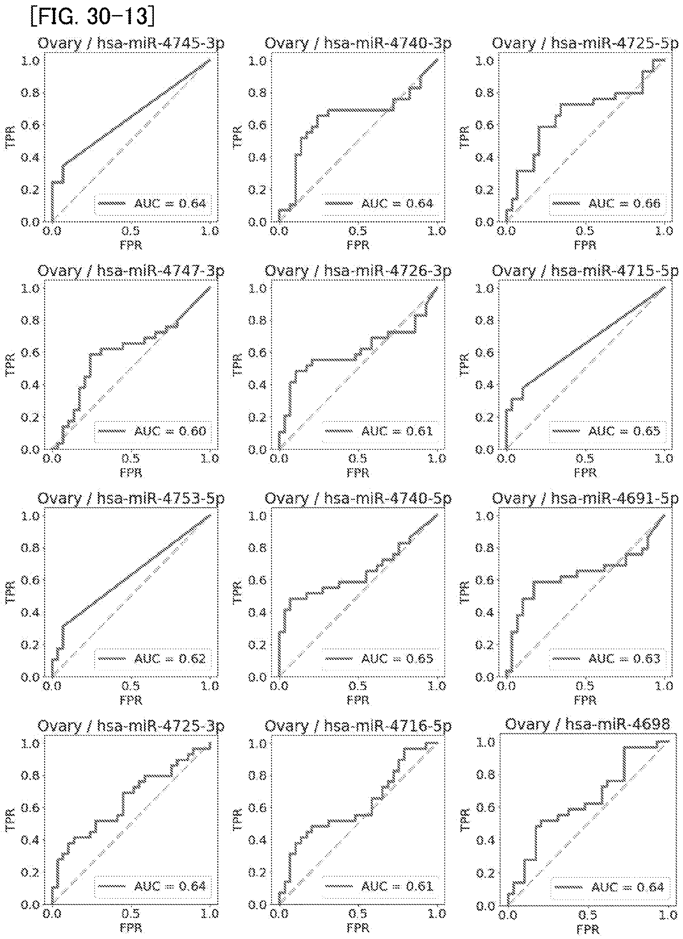

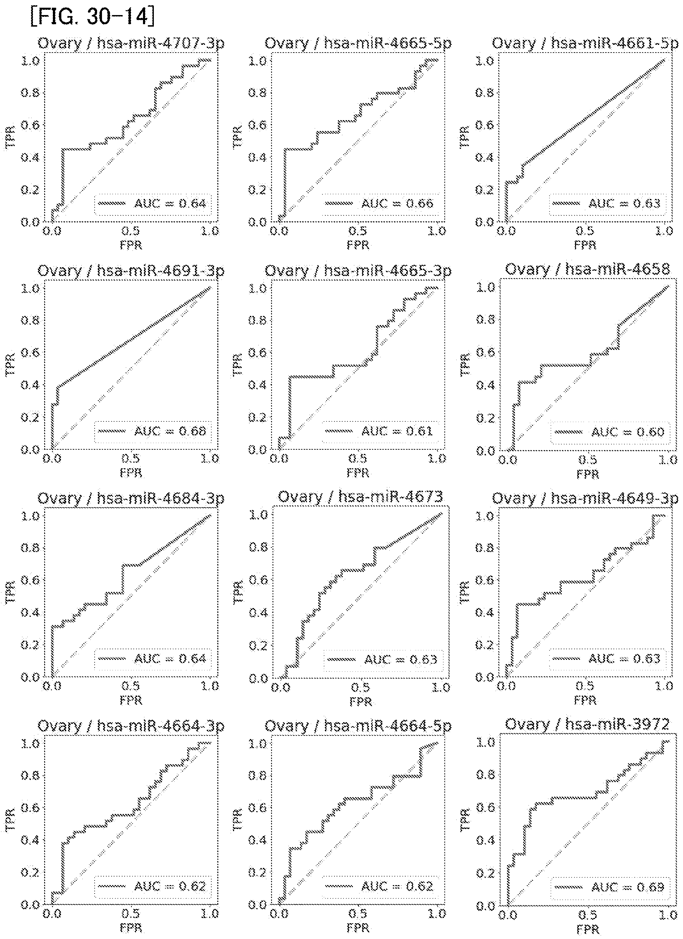

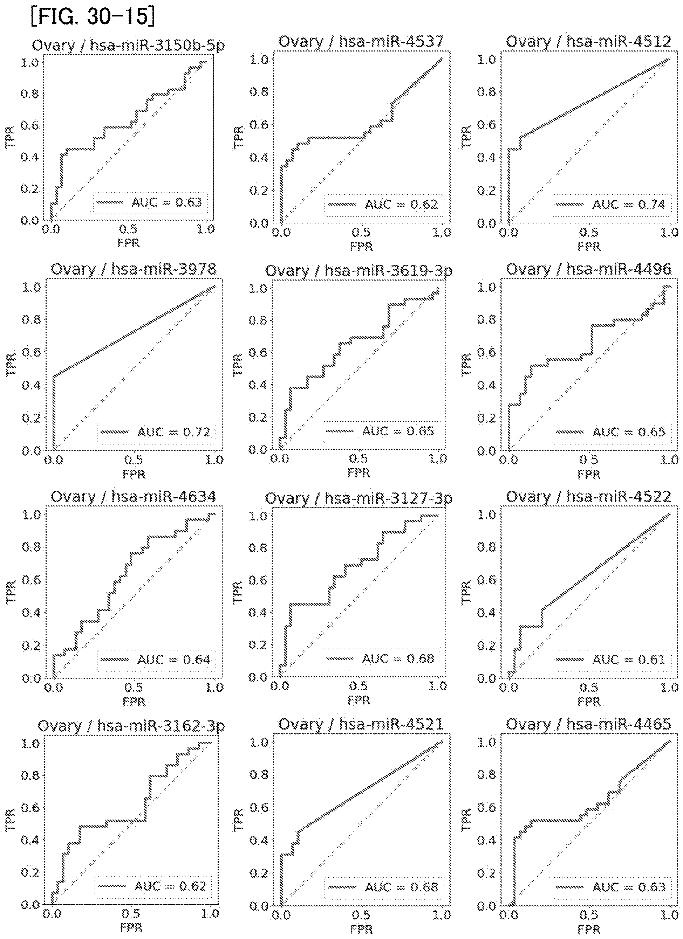

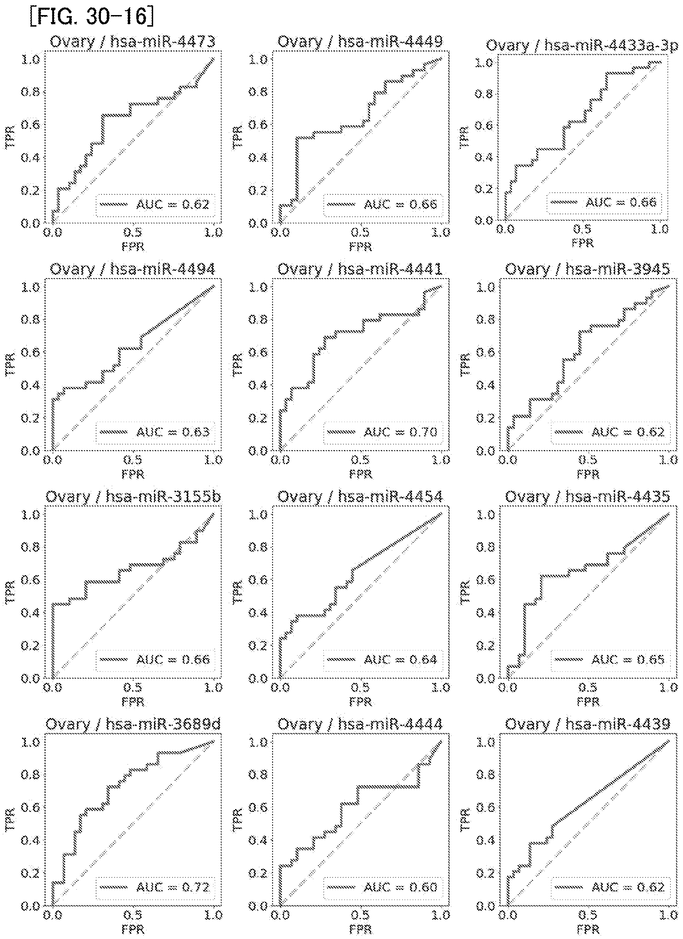

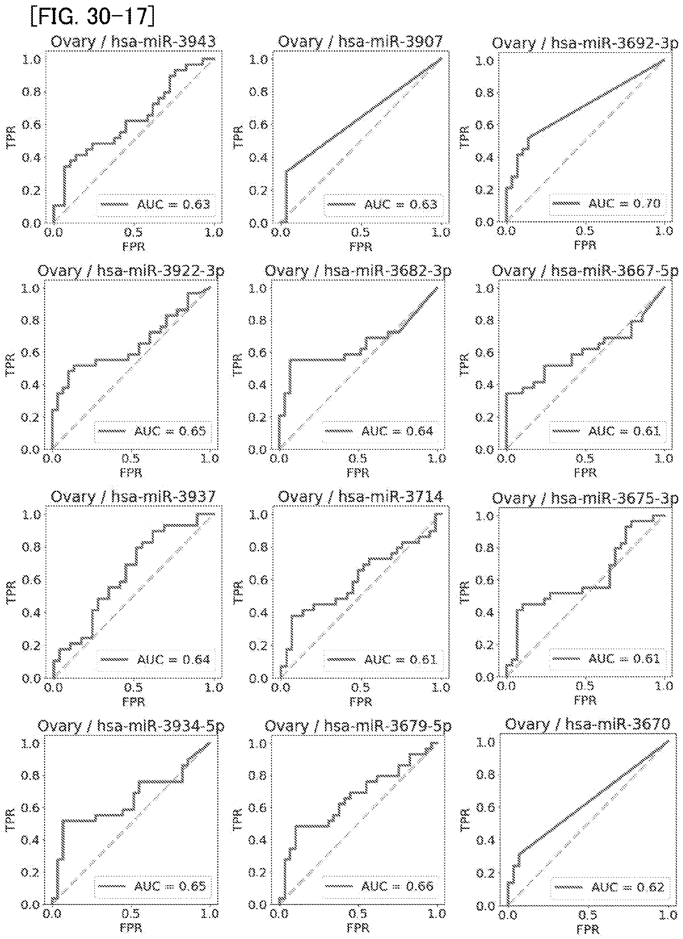

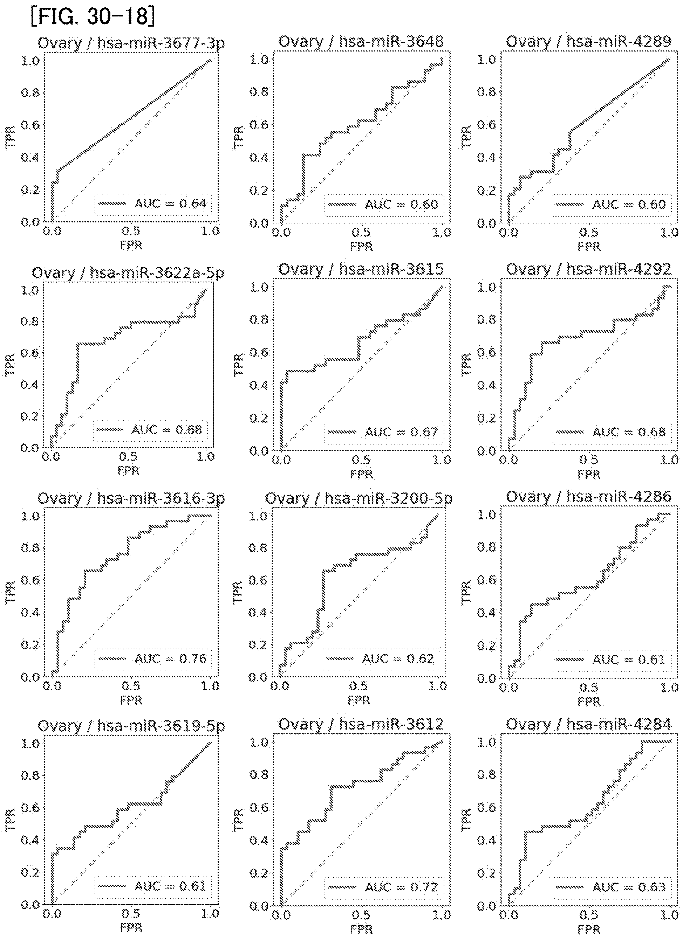

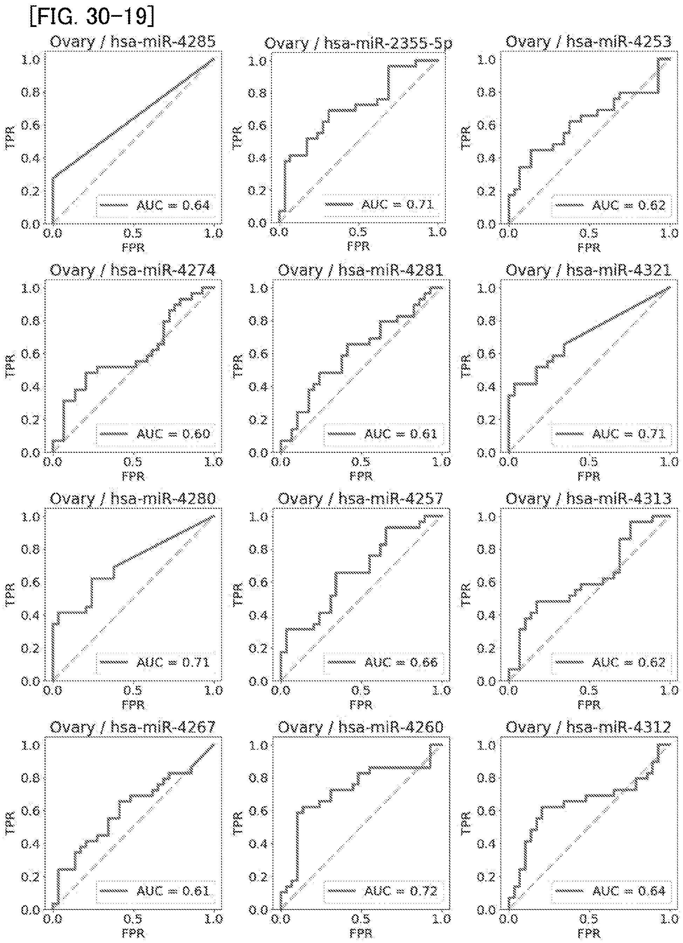

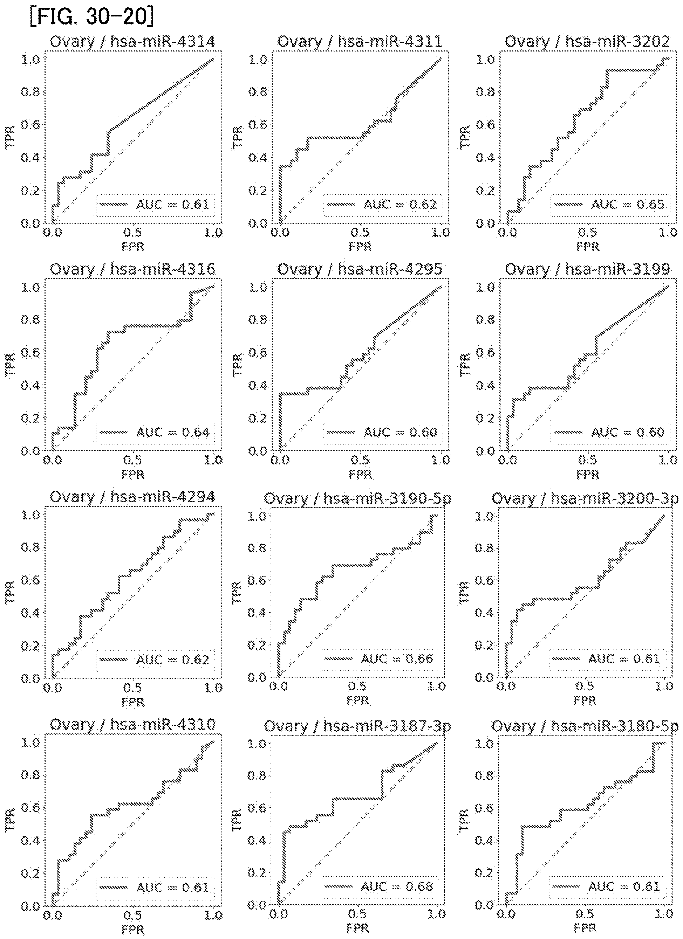

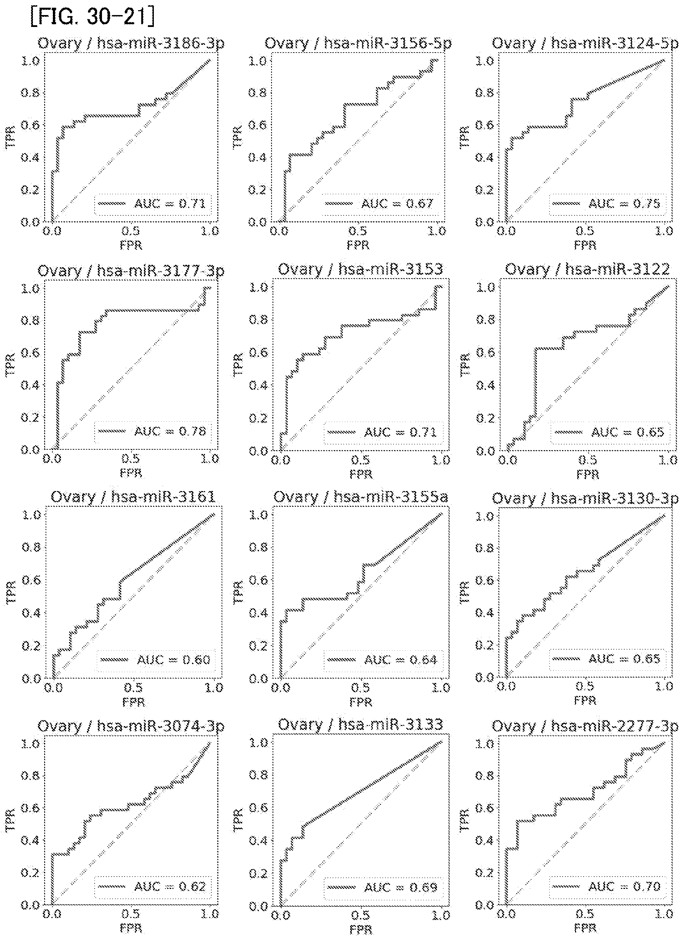

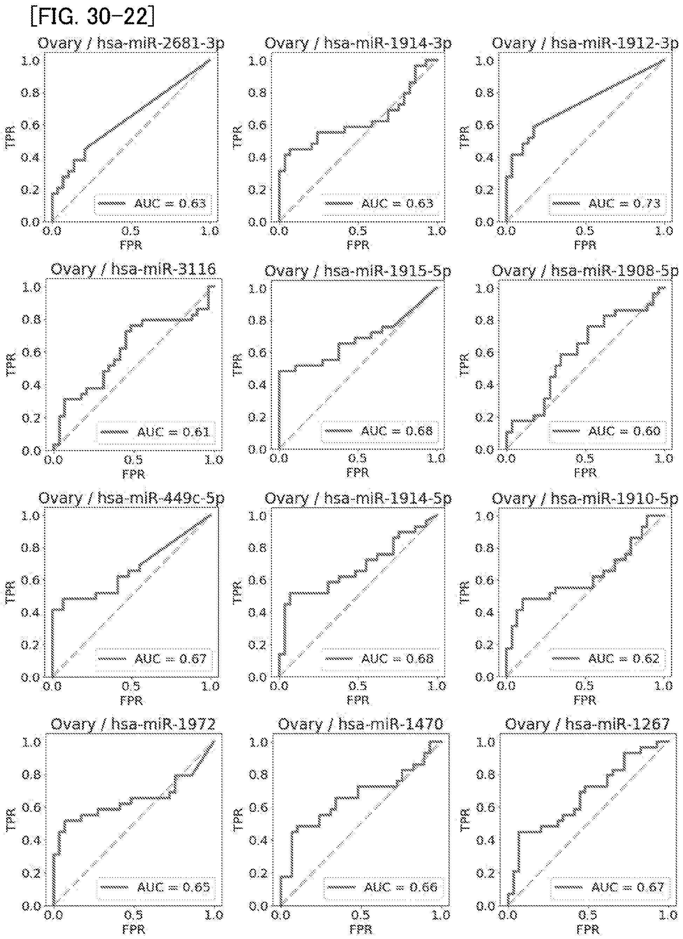

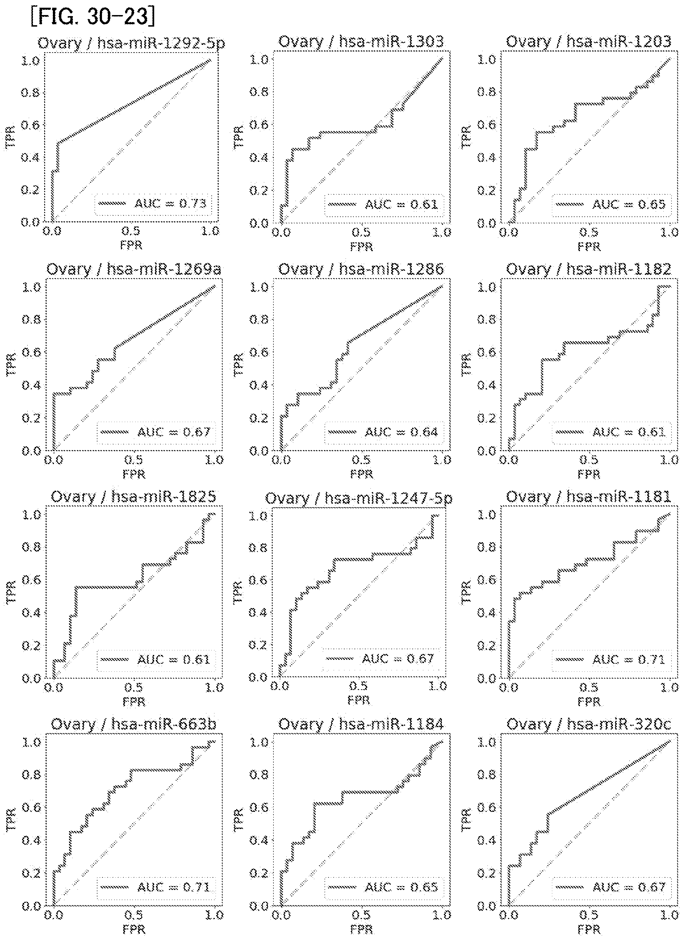

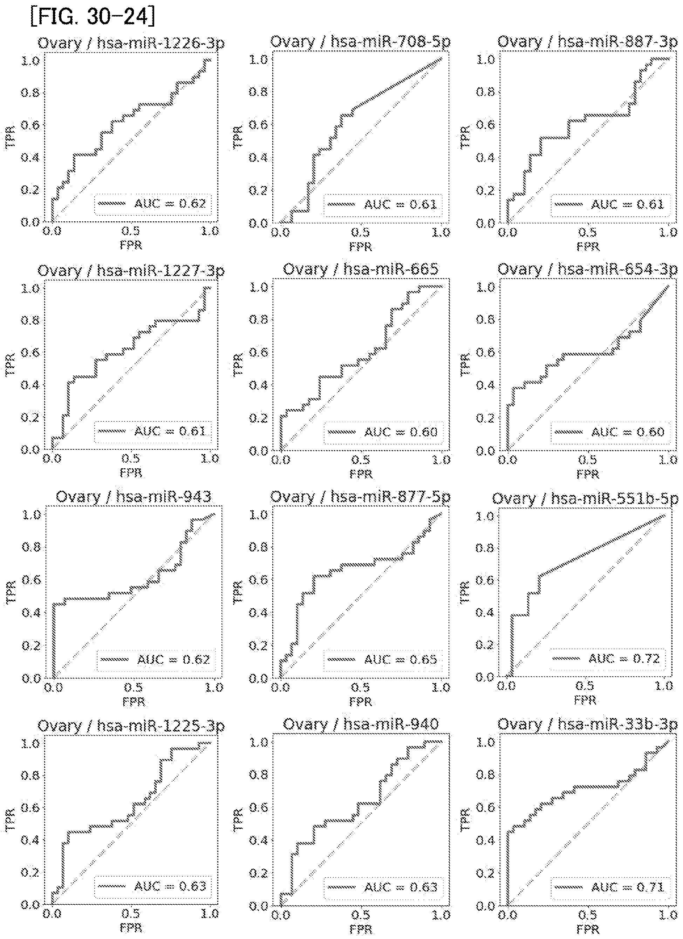

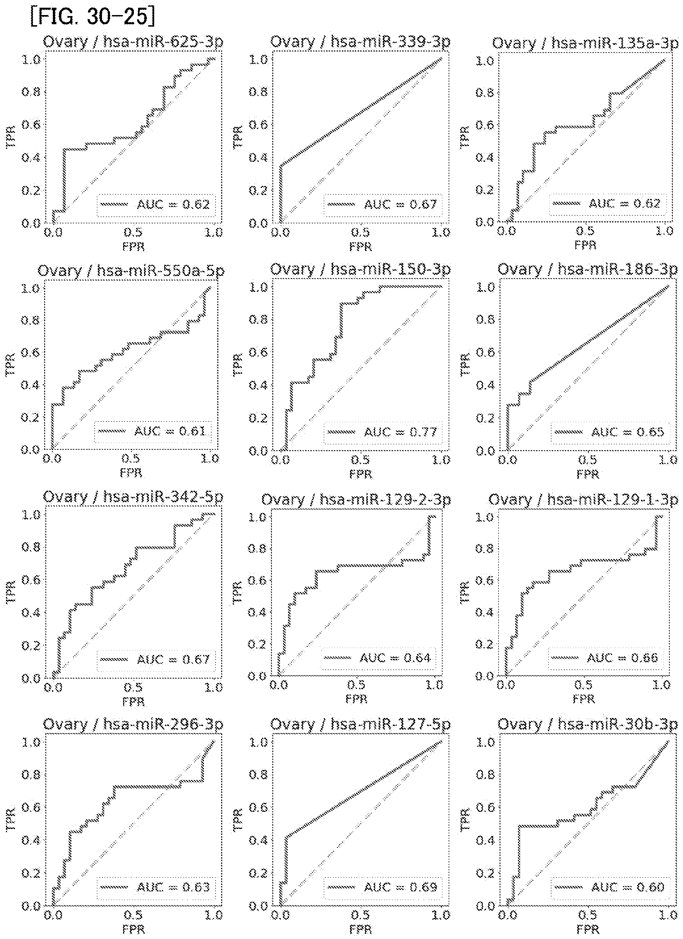

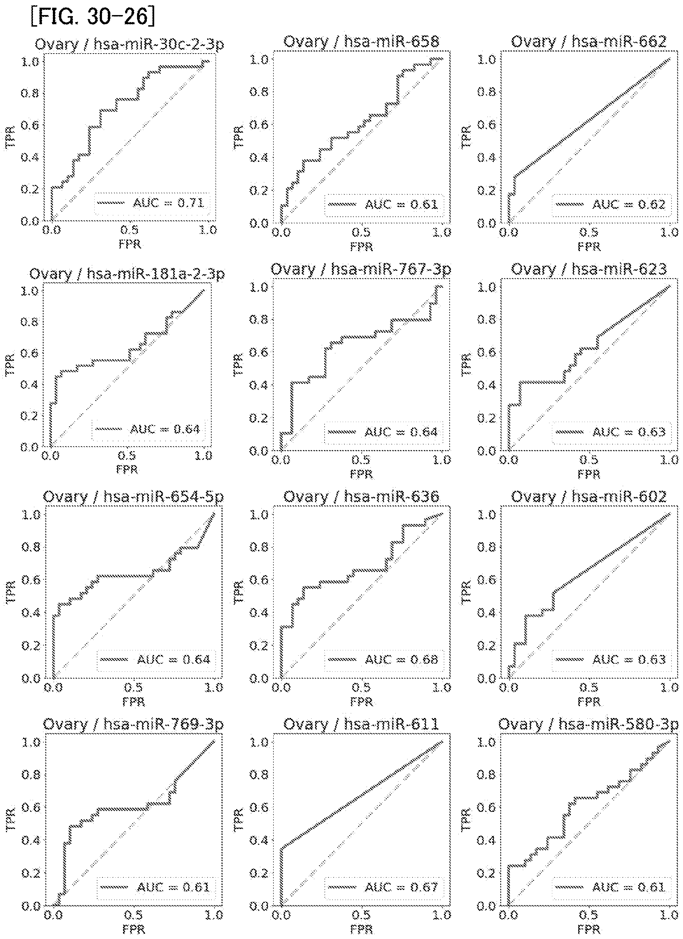

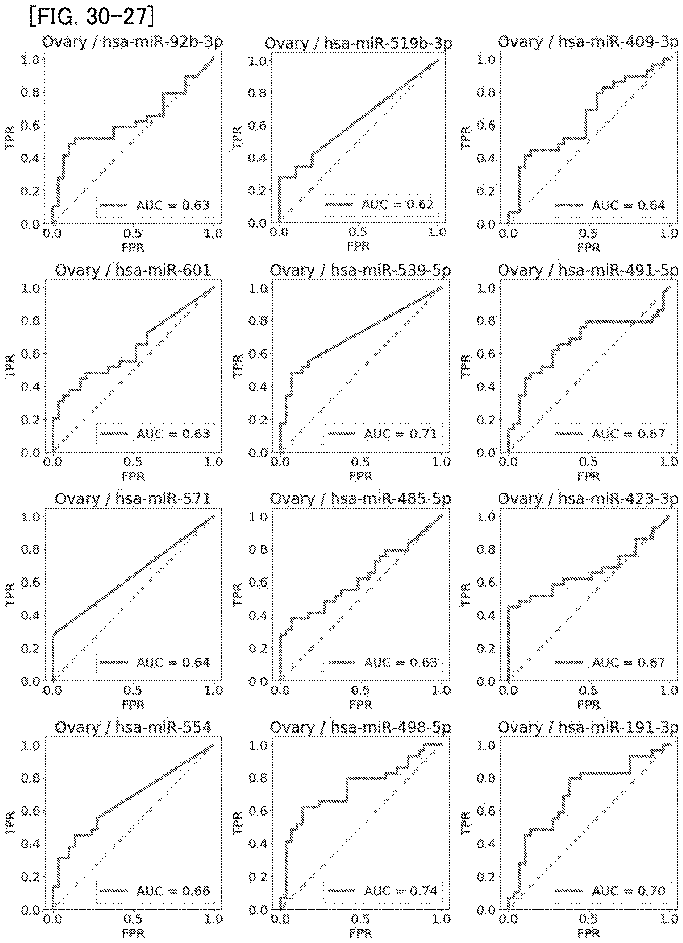

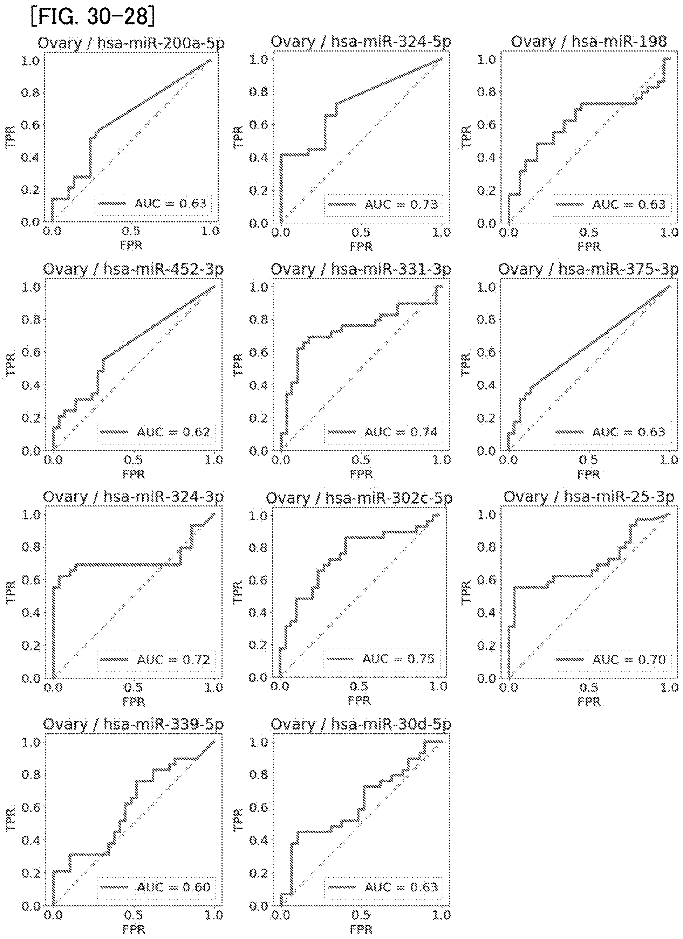

FIGS. 30-1 to 30-28 show ROCs curves for predicting ovarian cancer in humans using a single miRNA.

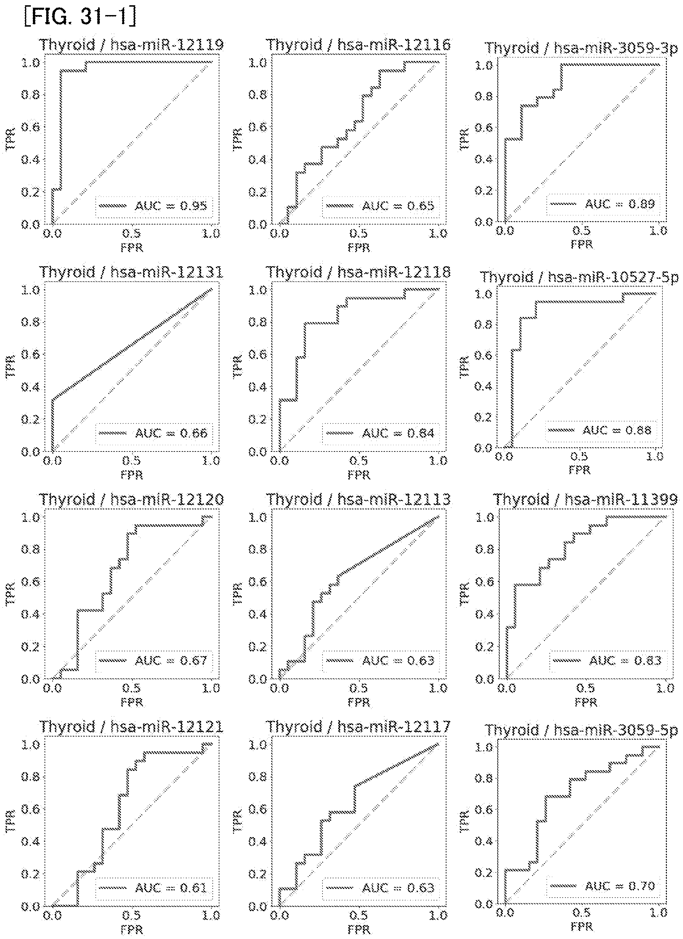

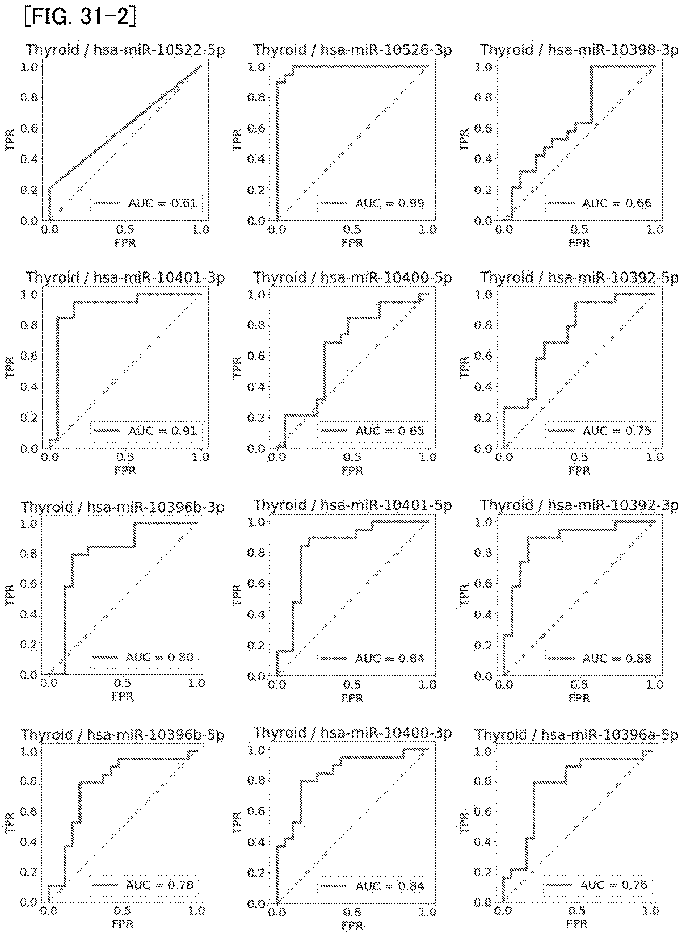

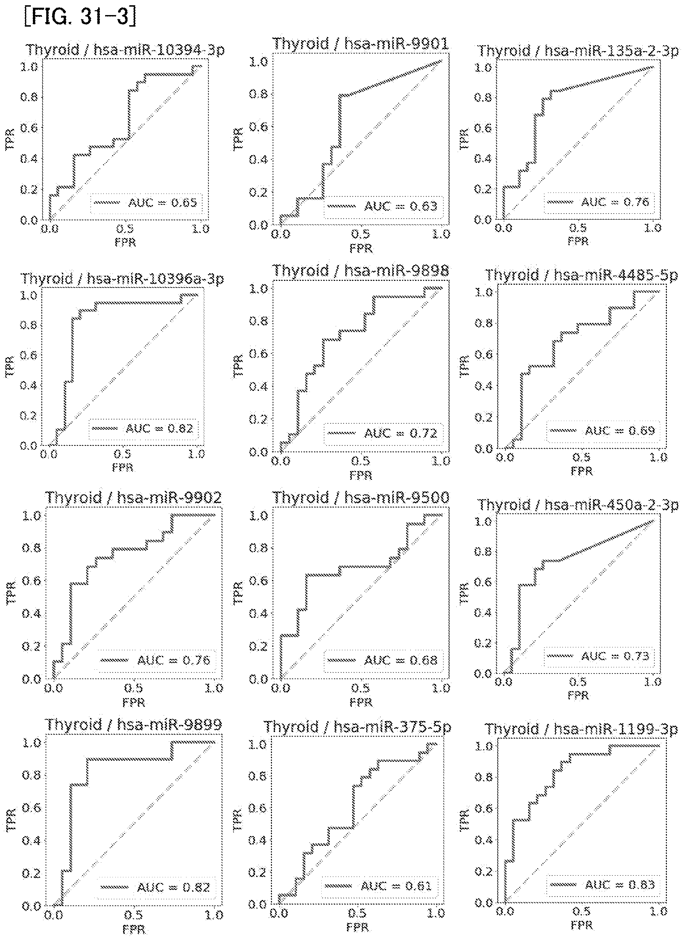

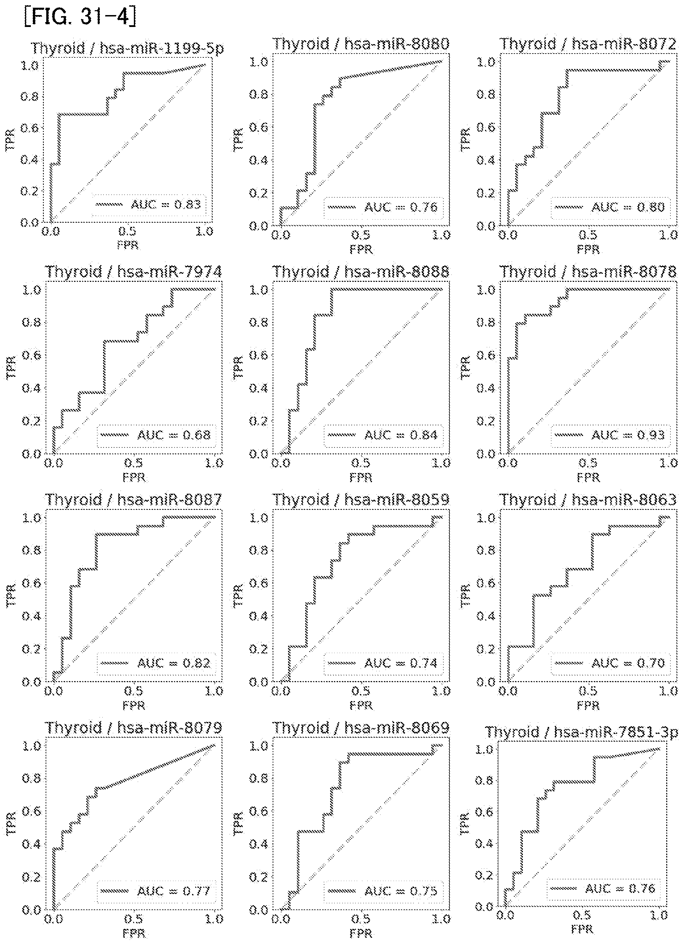

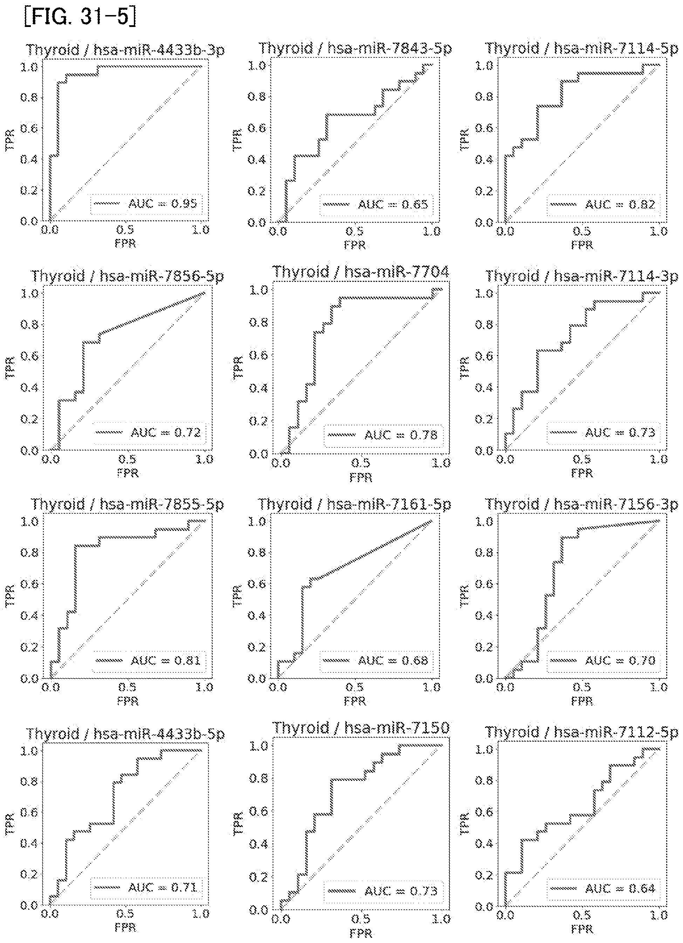

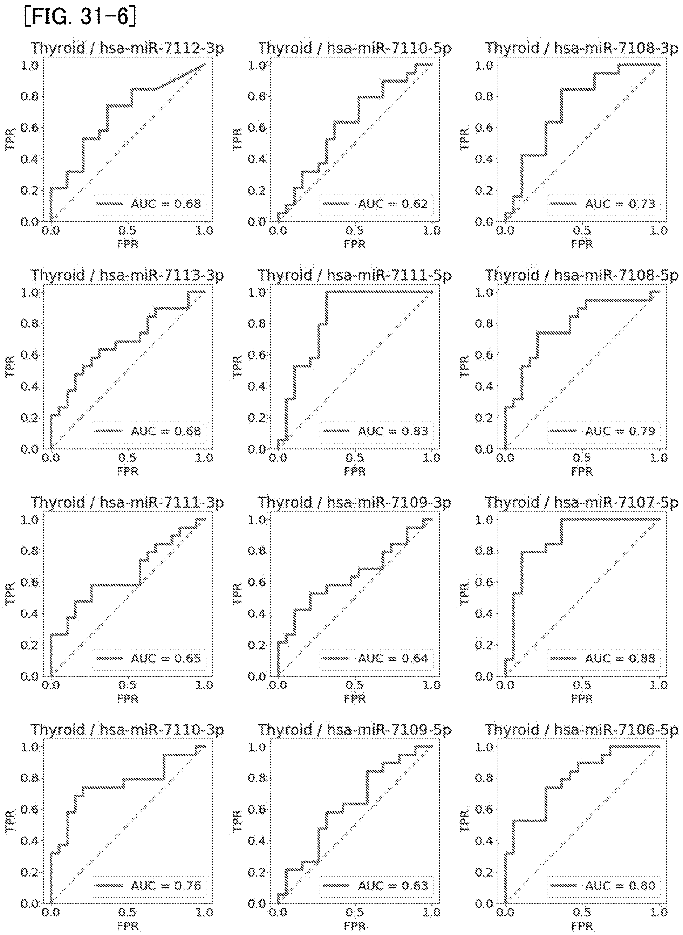

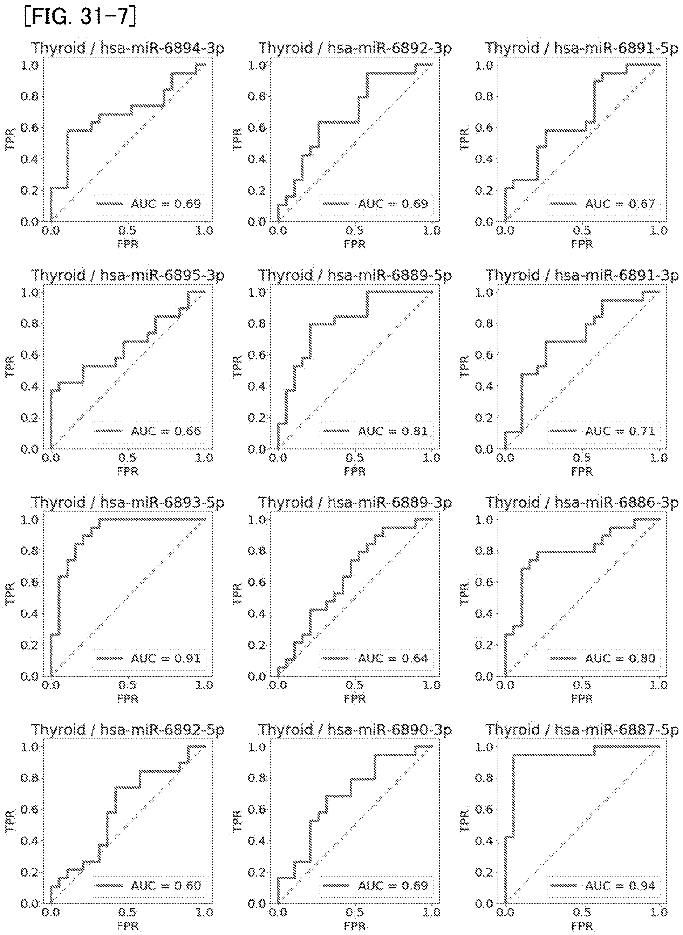

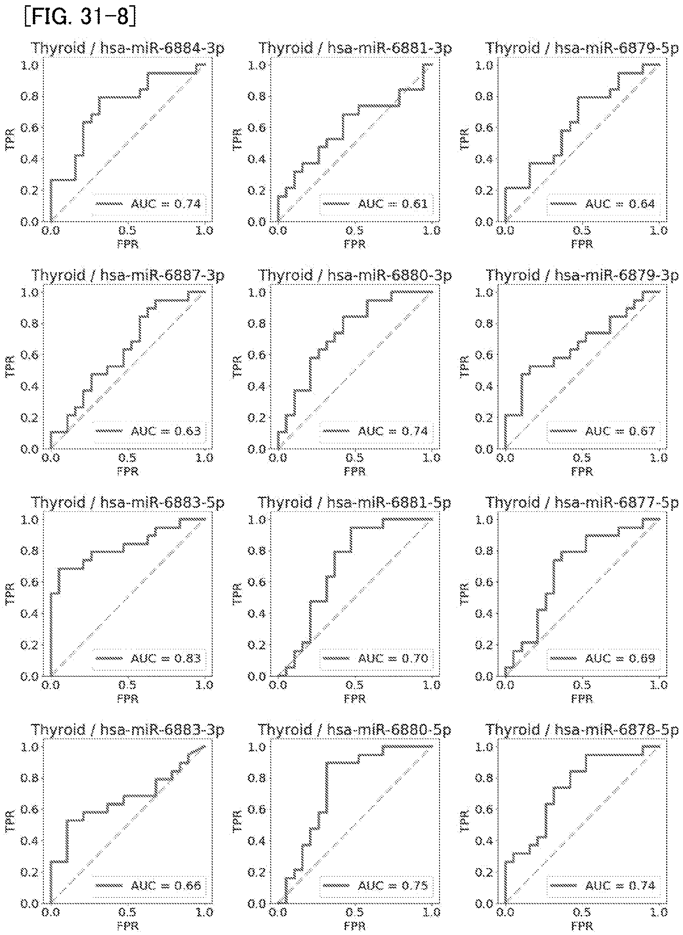

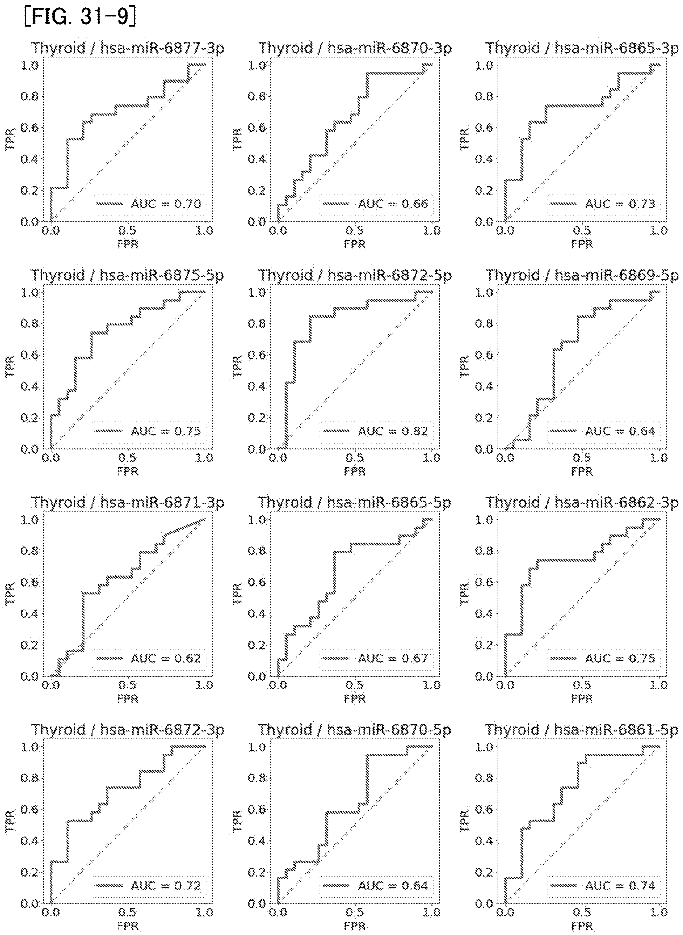

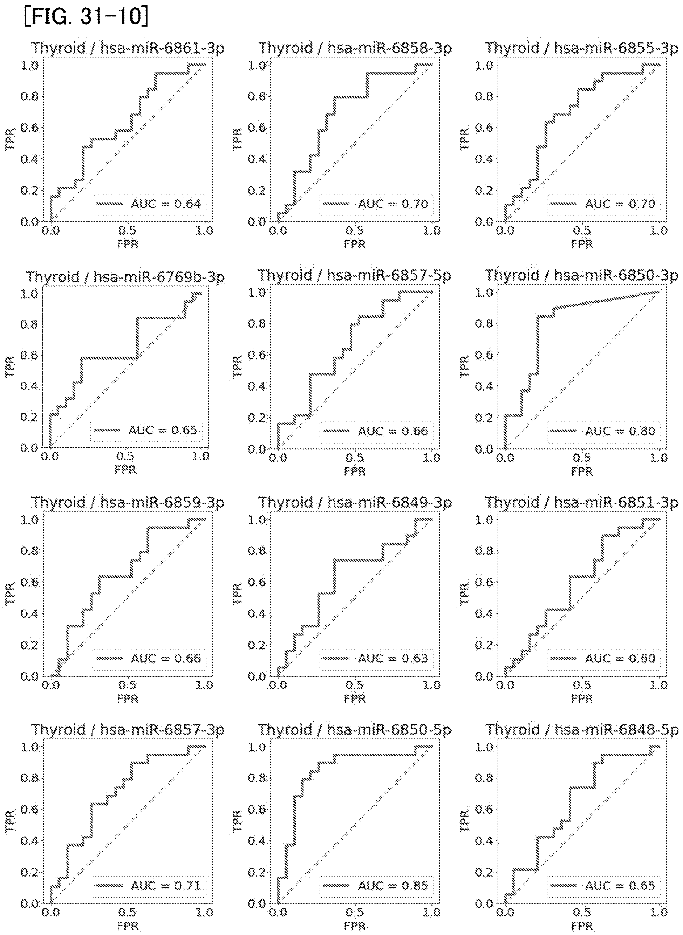

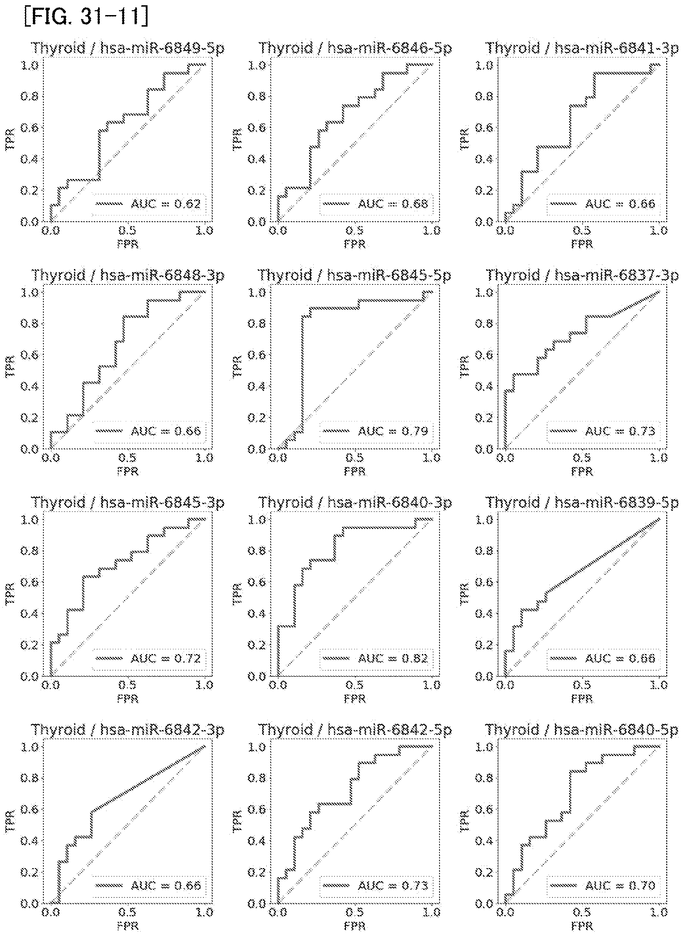

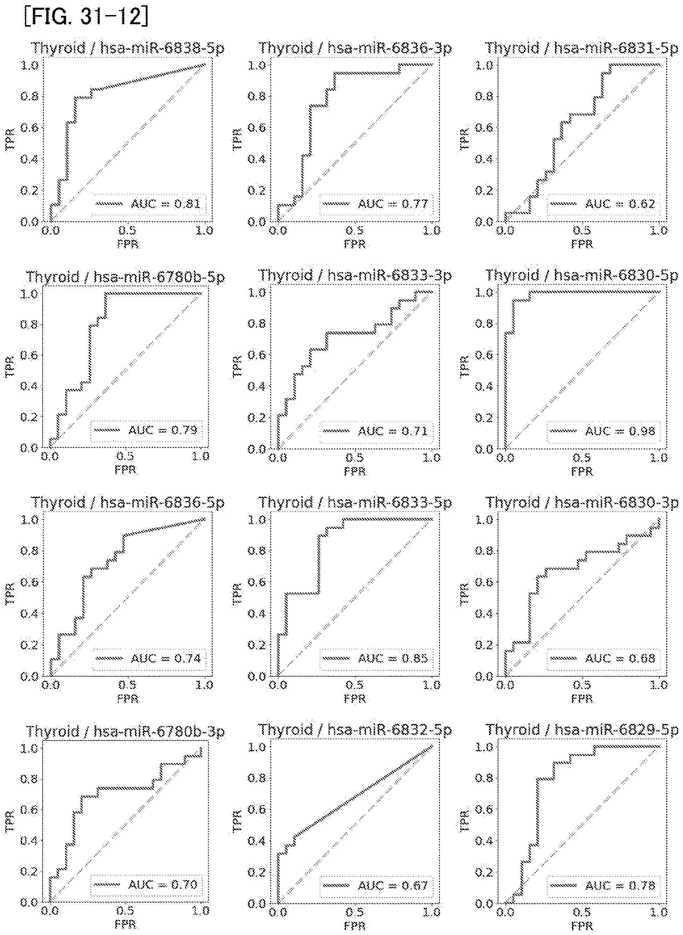

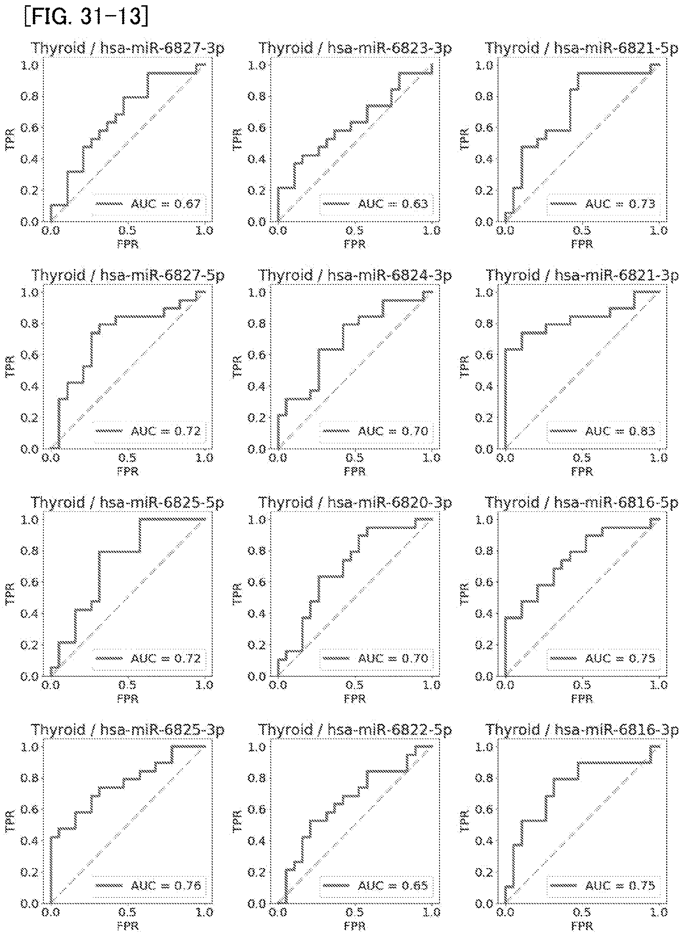

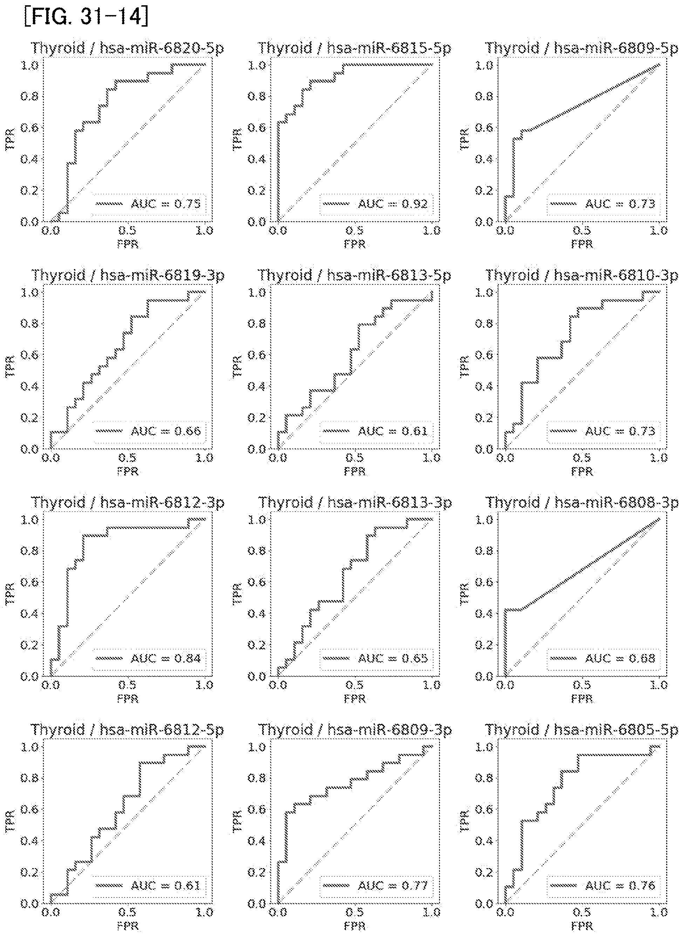

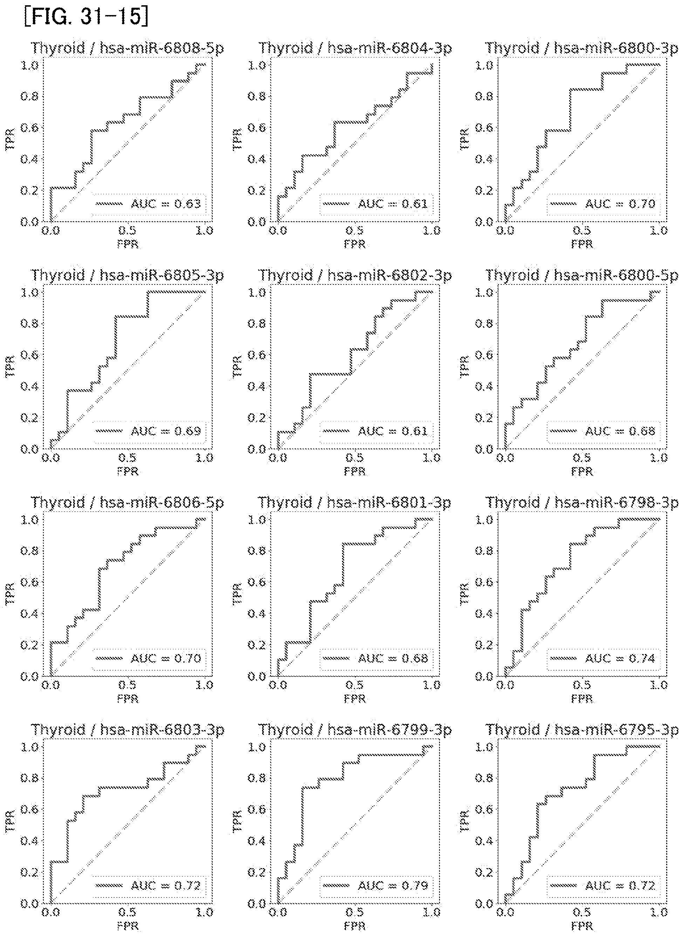

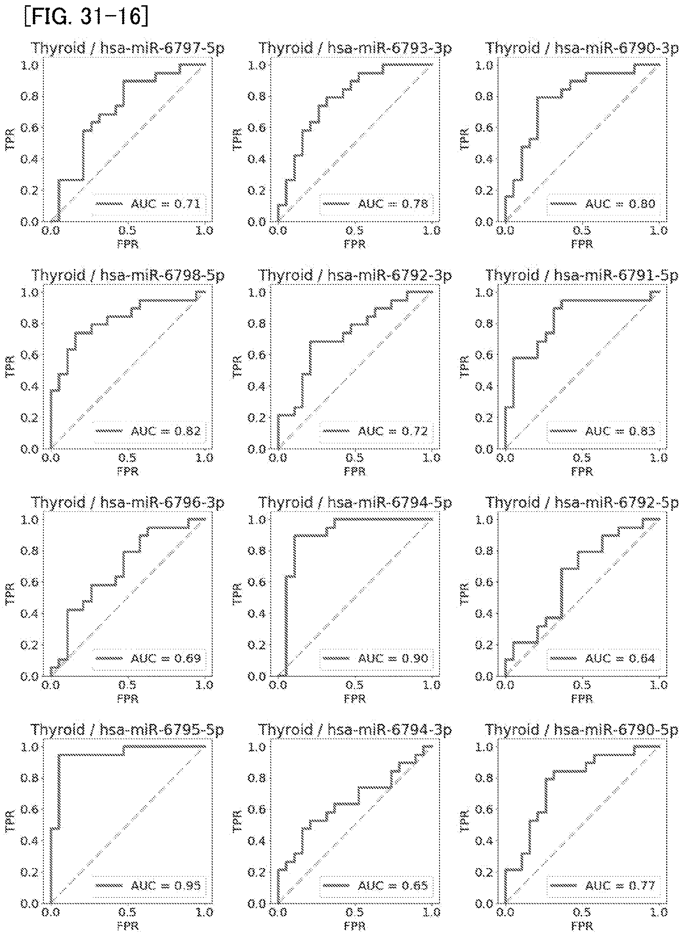

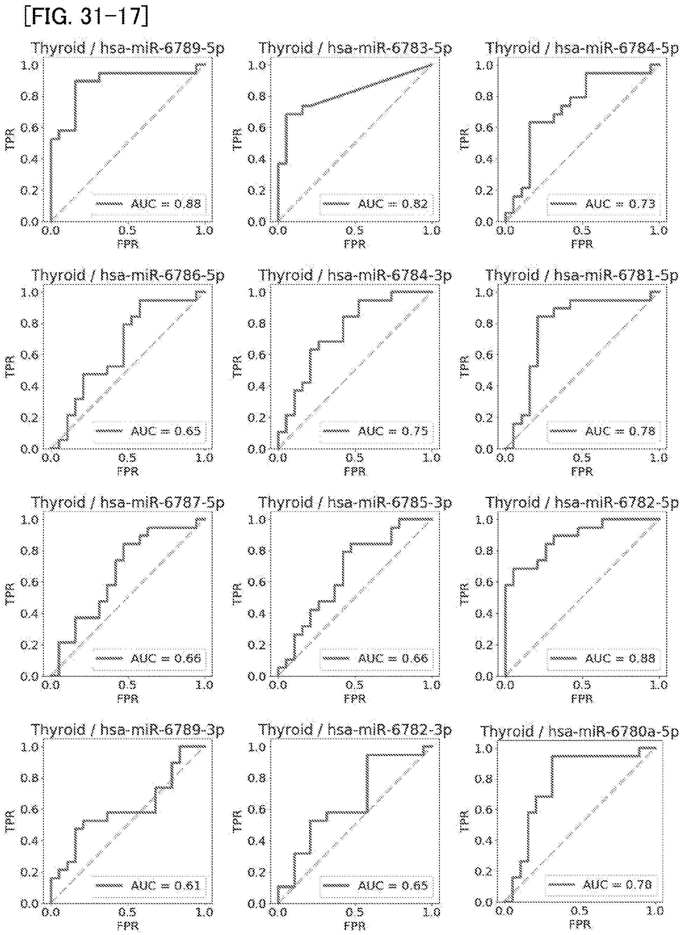

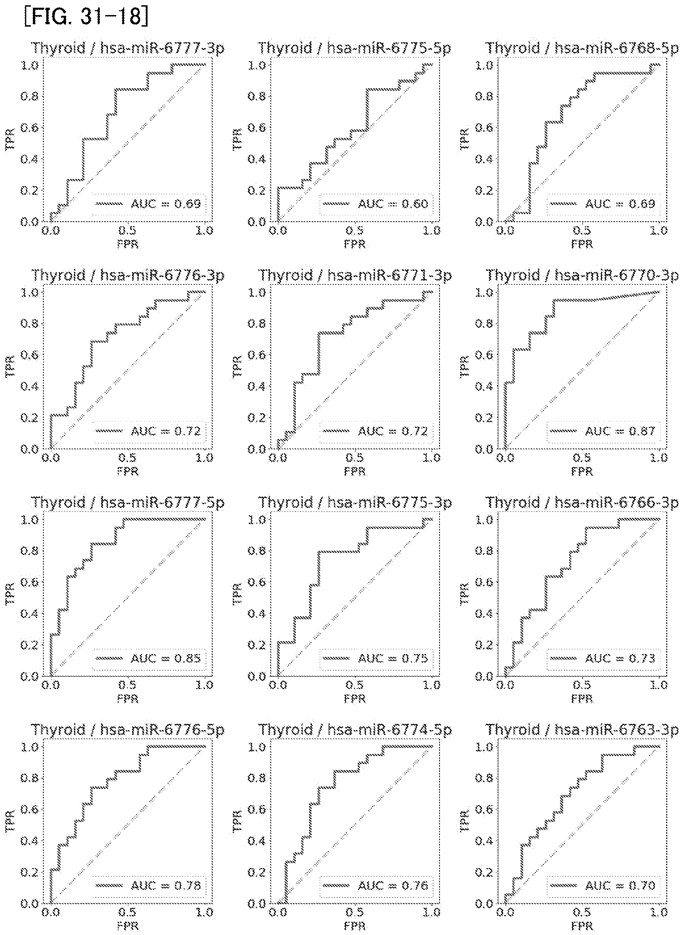

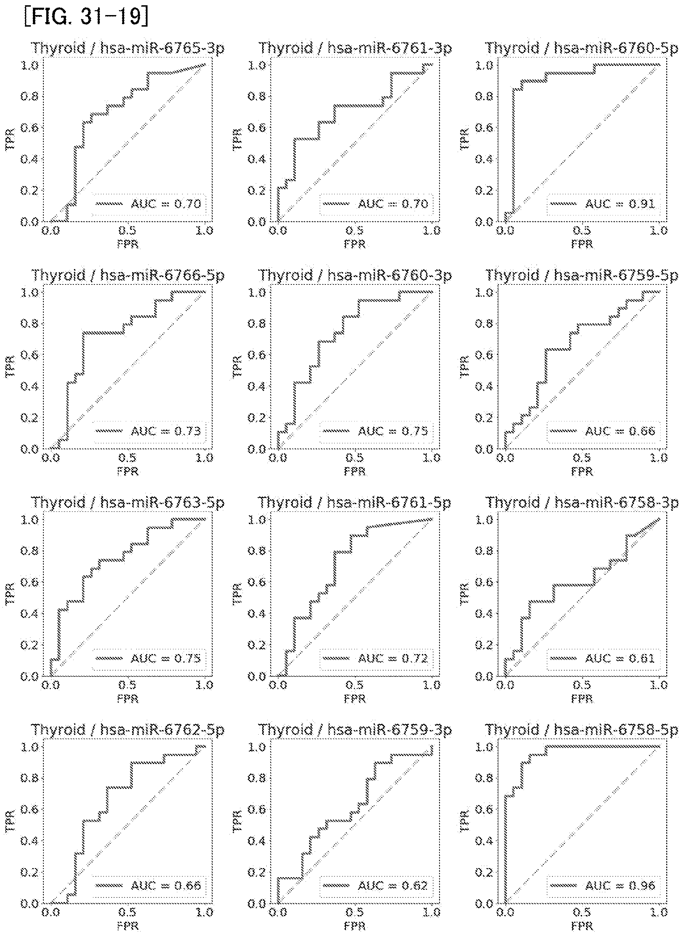

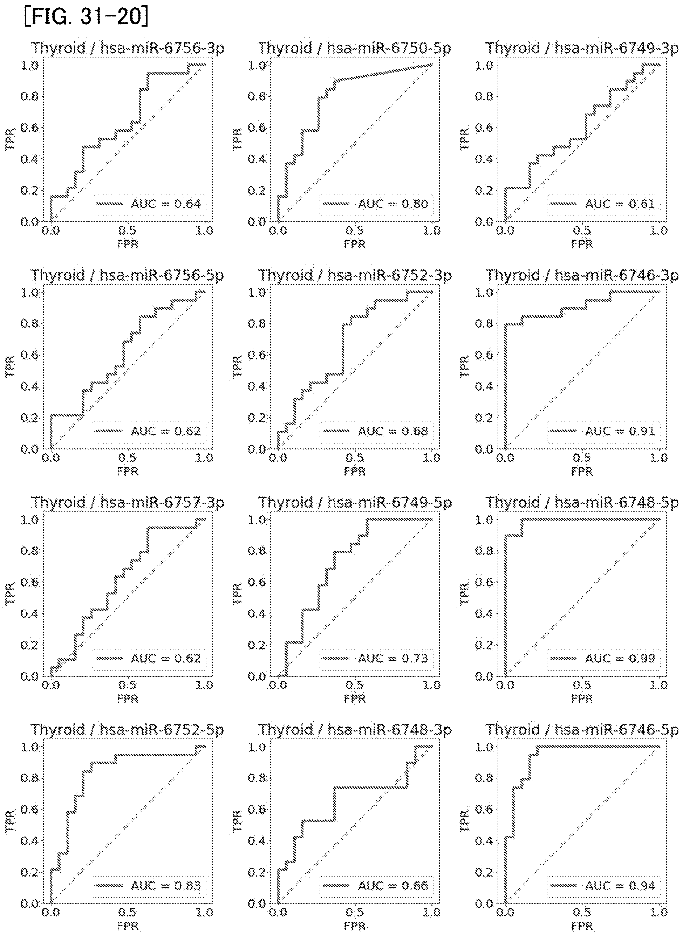

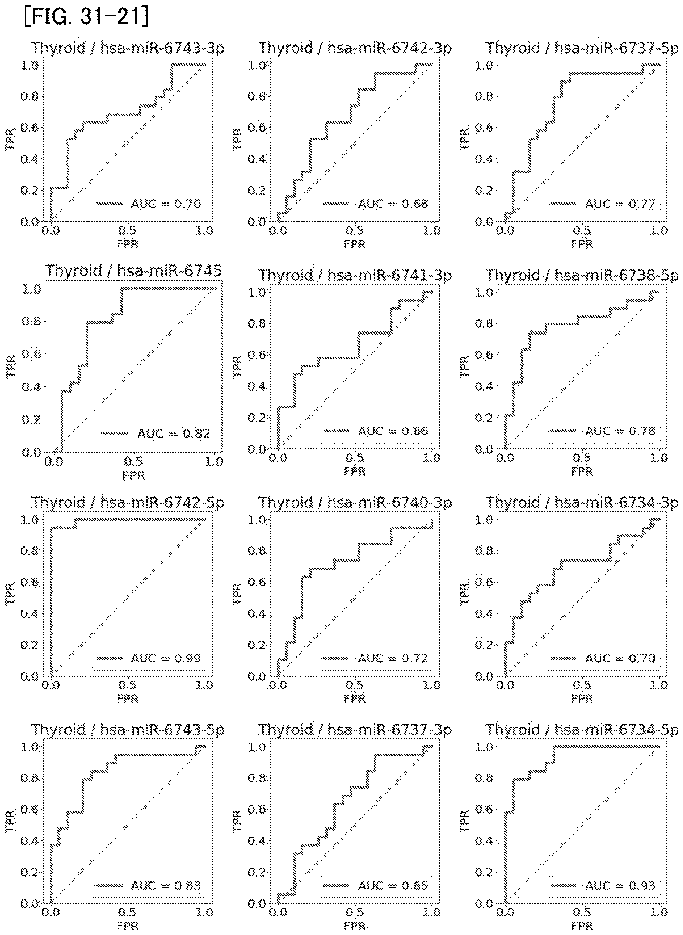

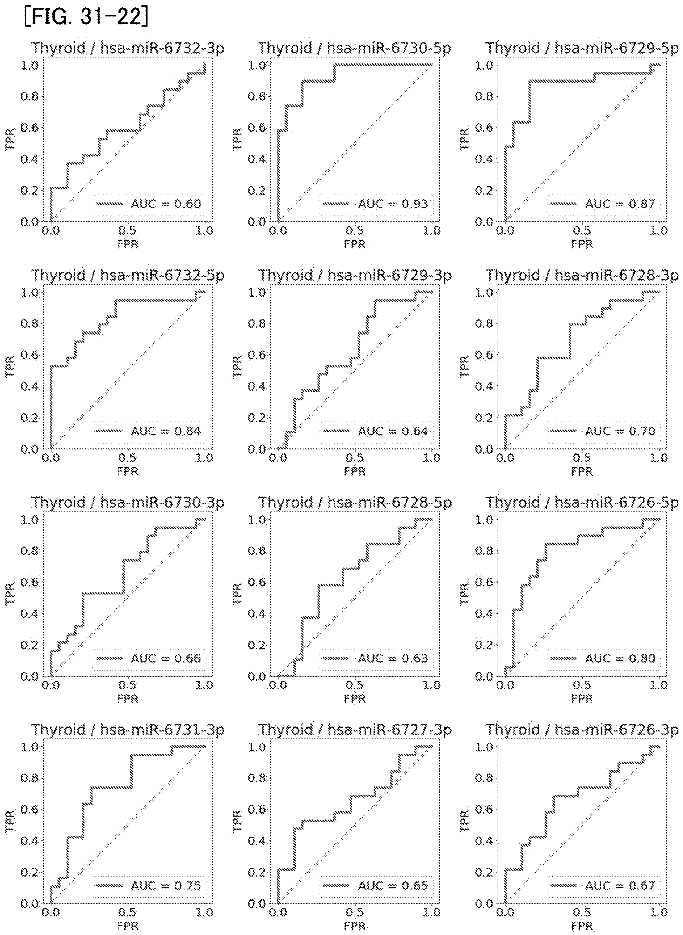

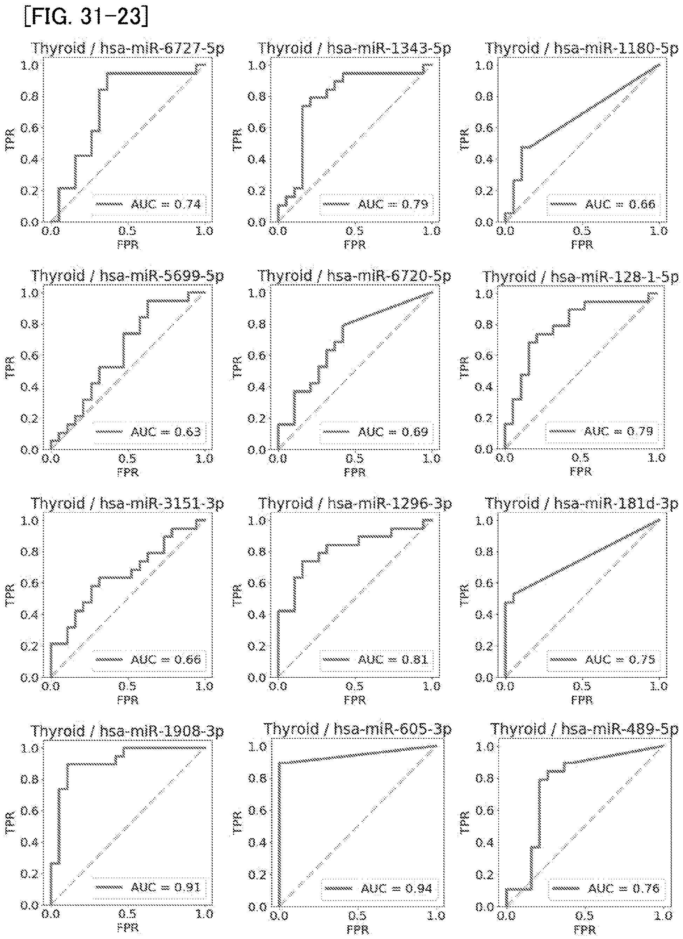

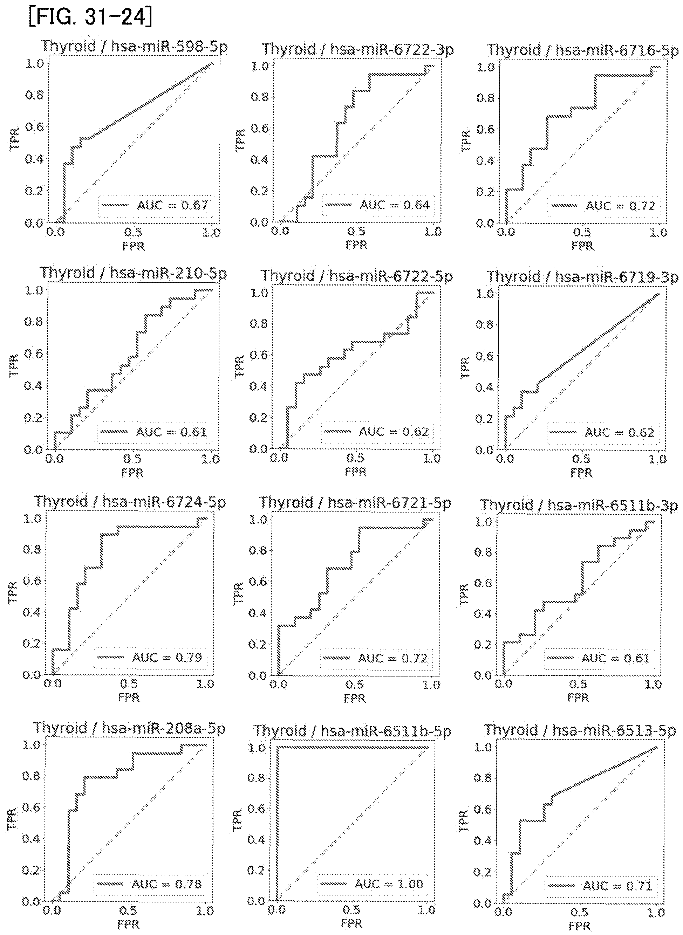

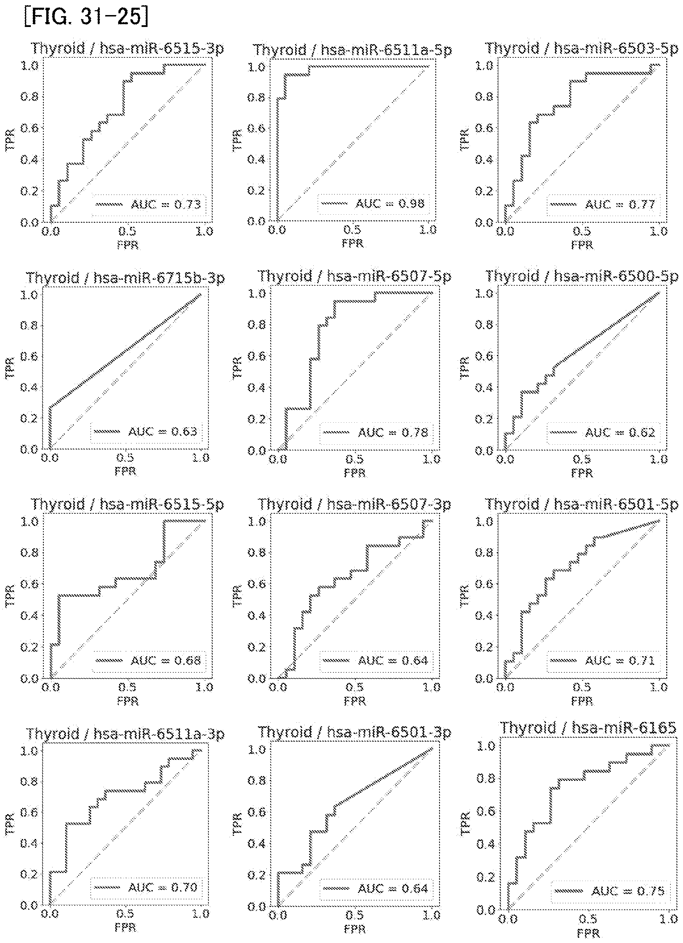

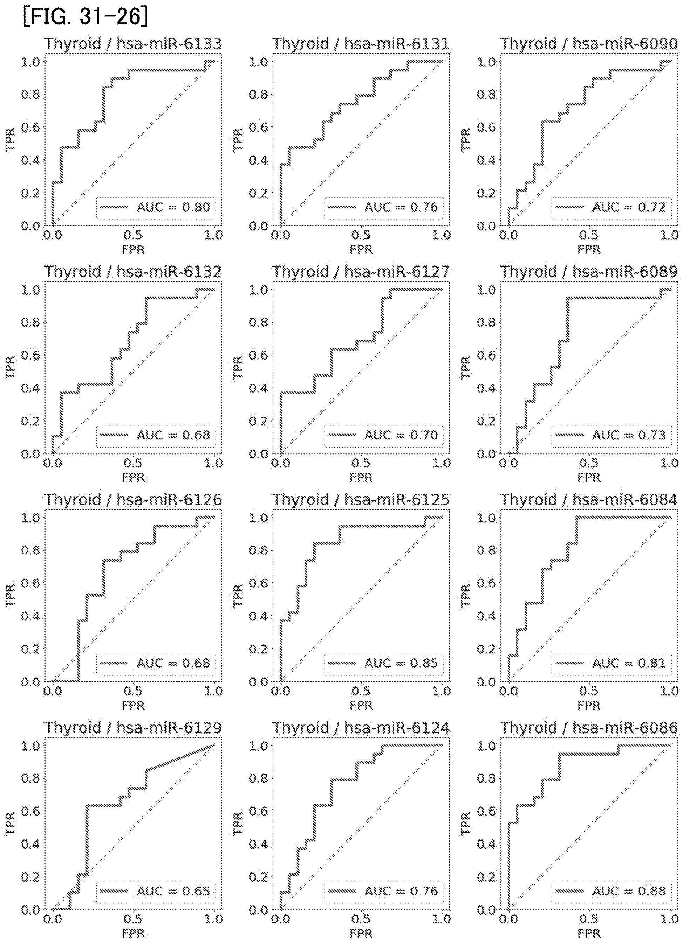

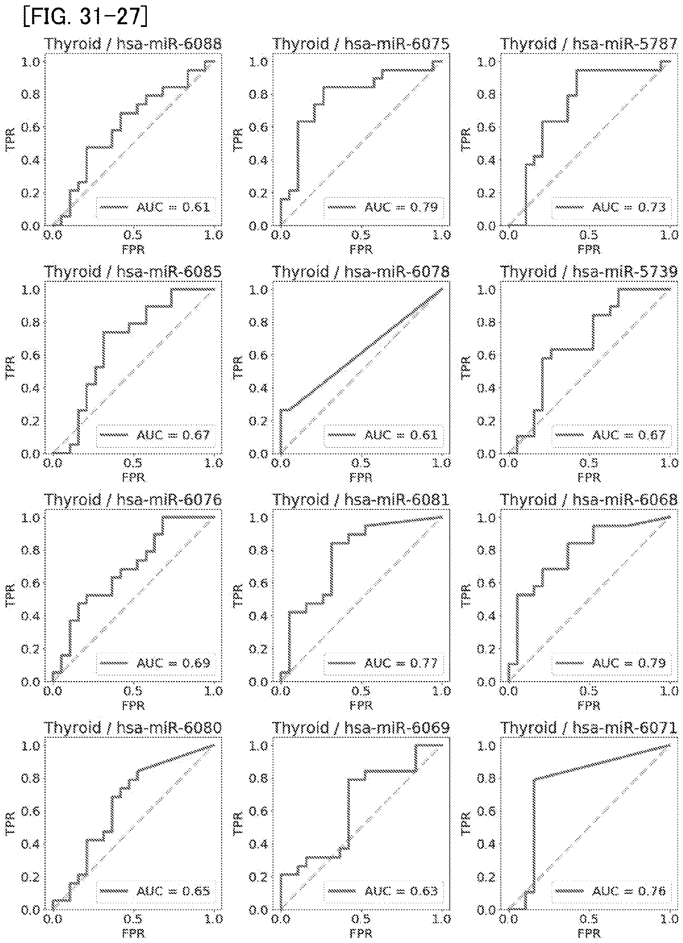

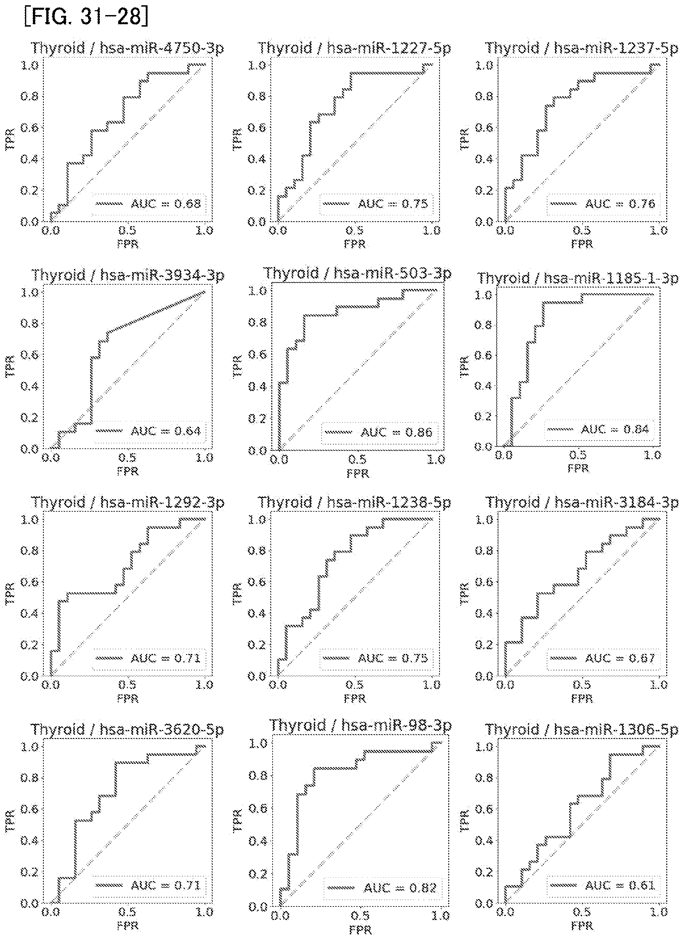

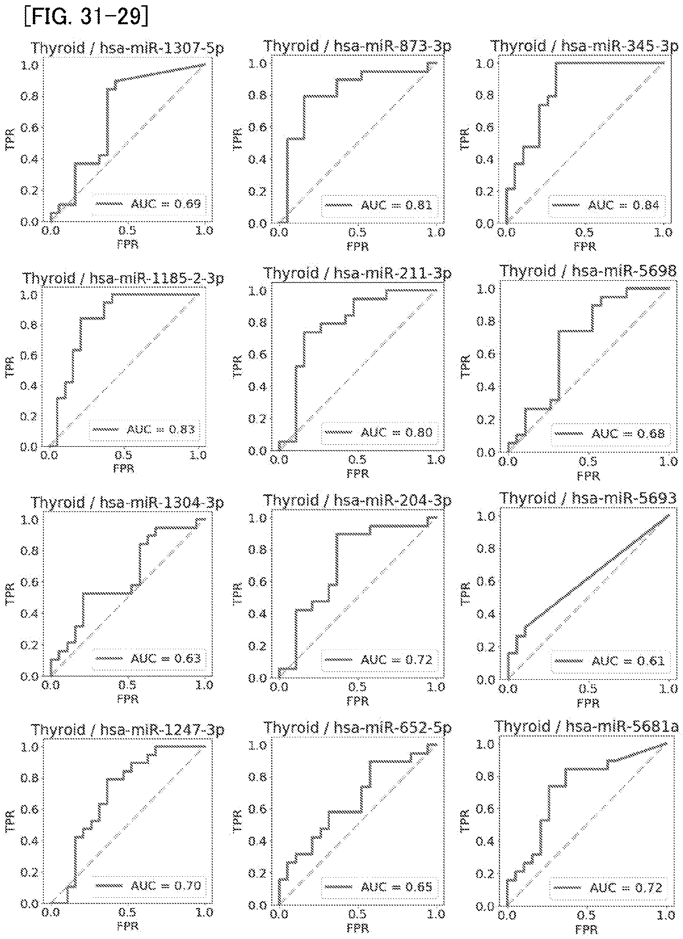

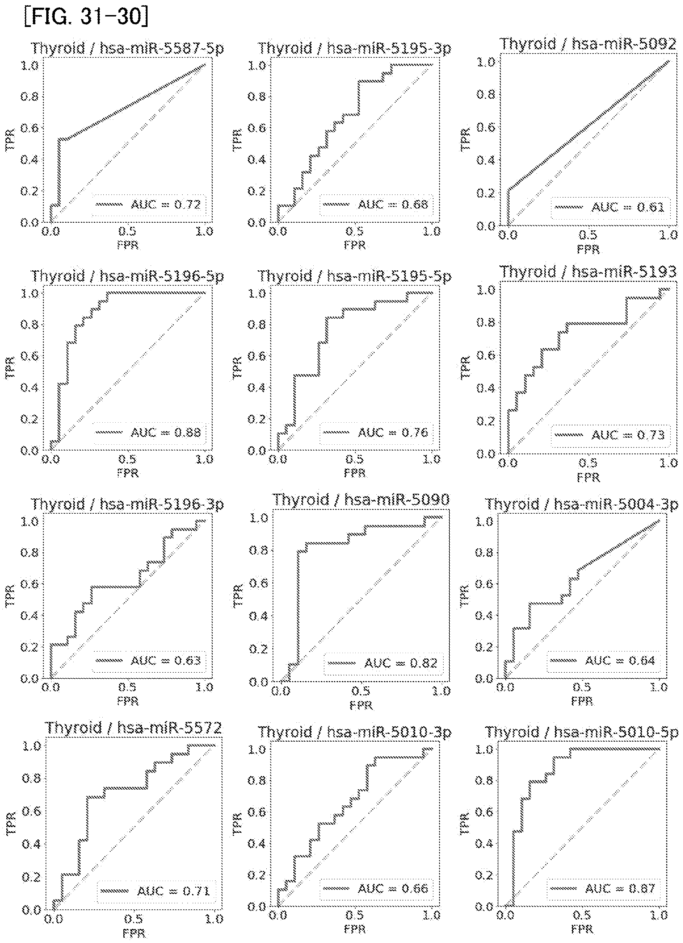

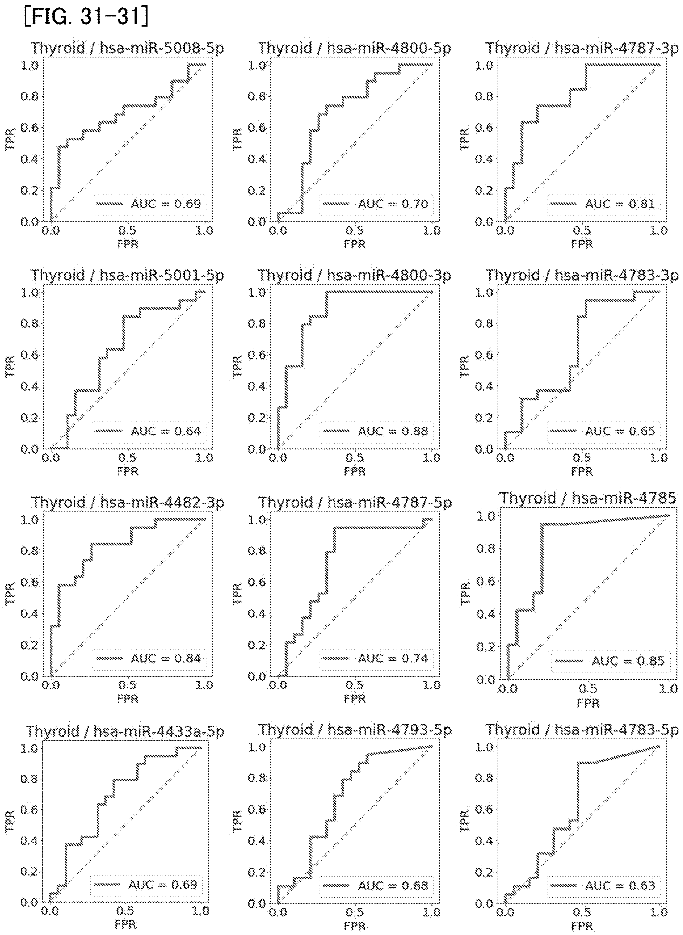

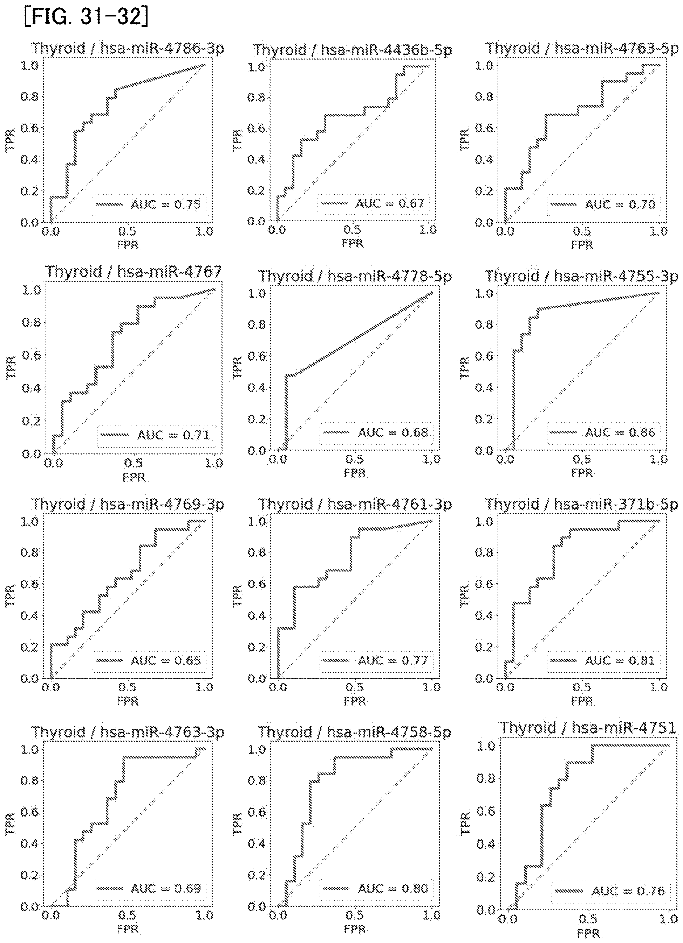

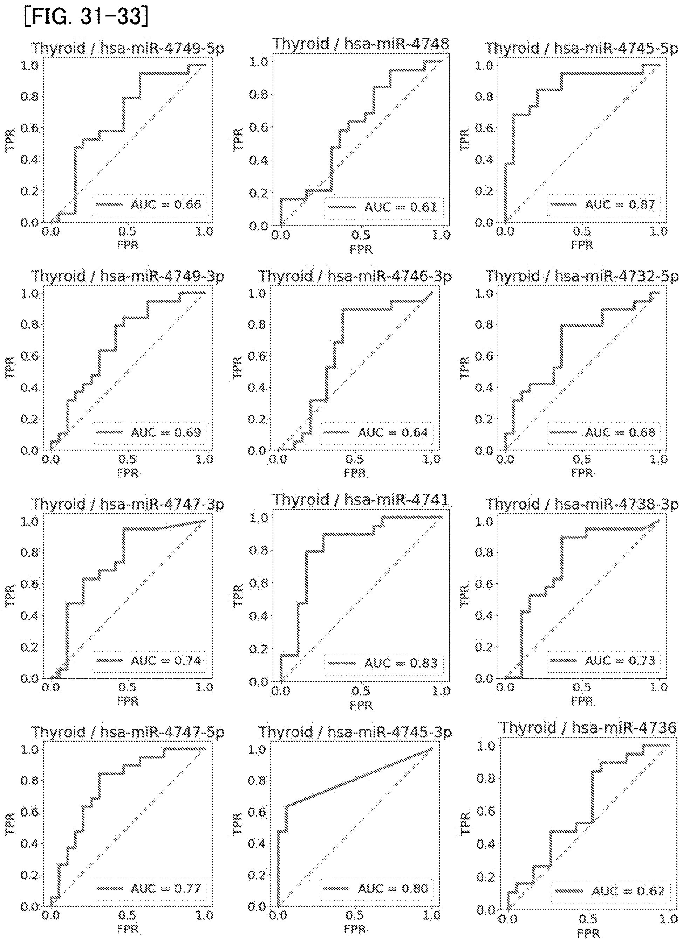

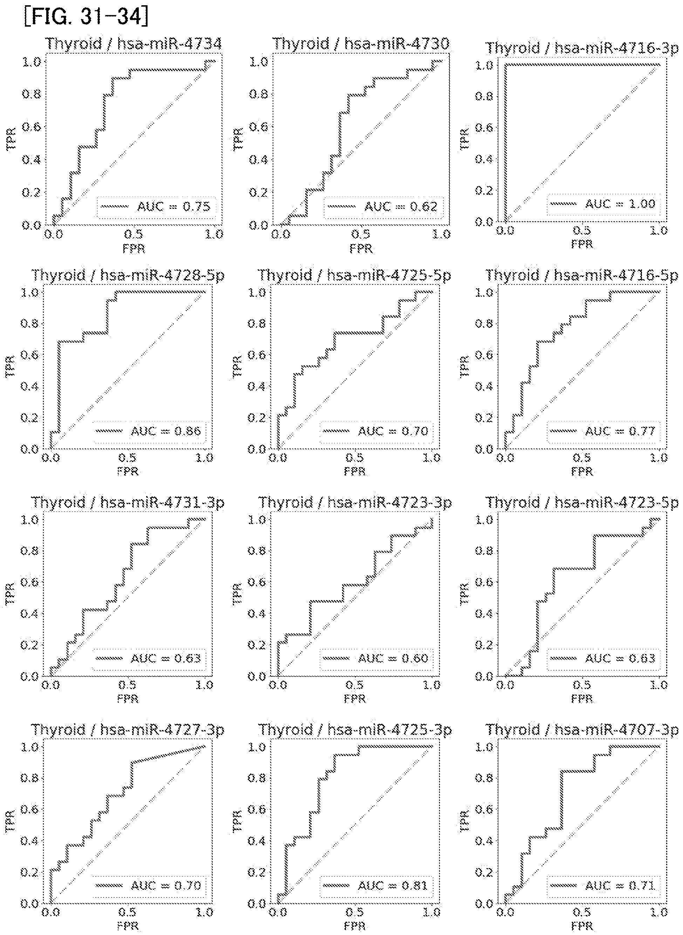

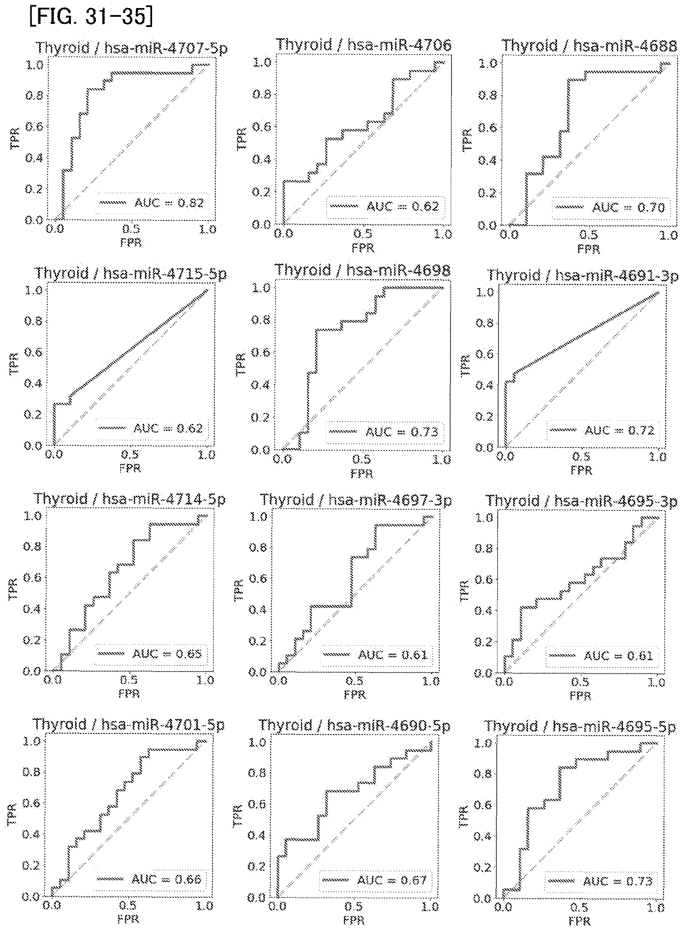

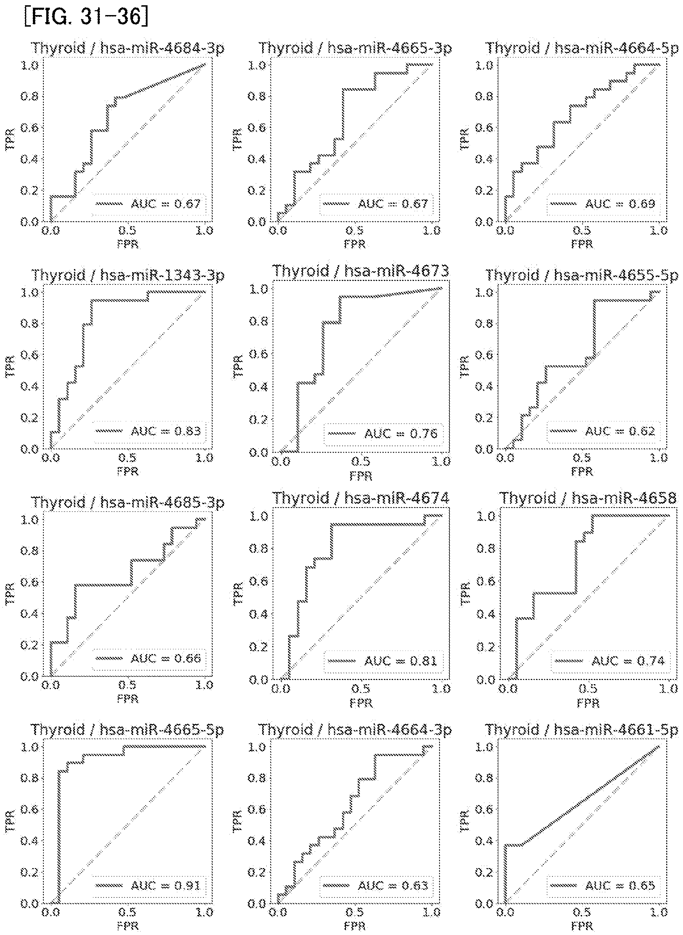

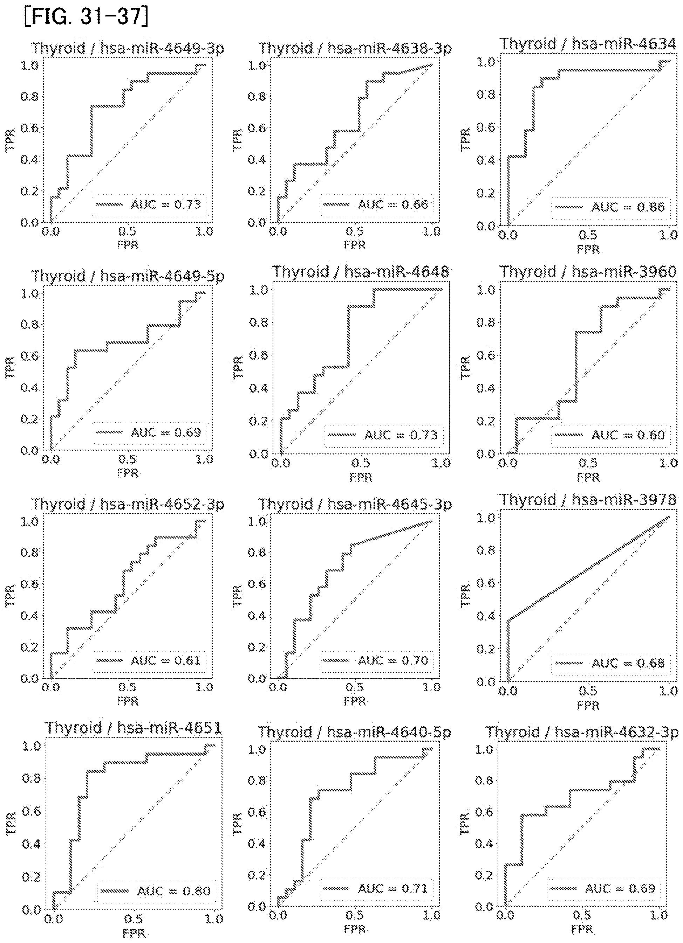

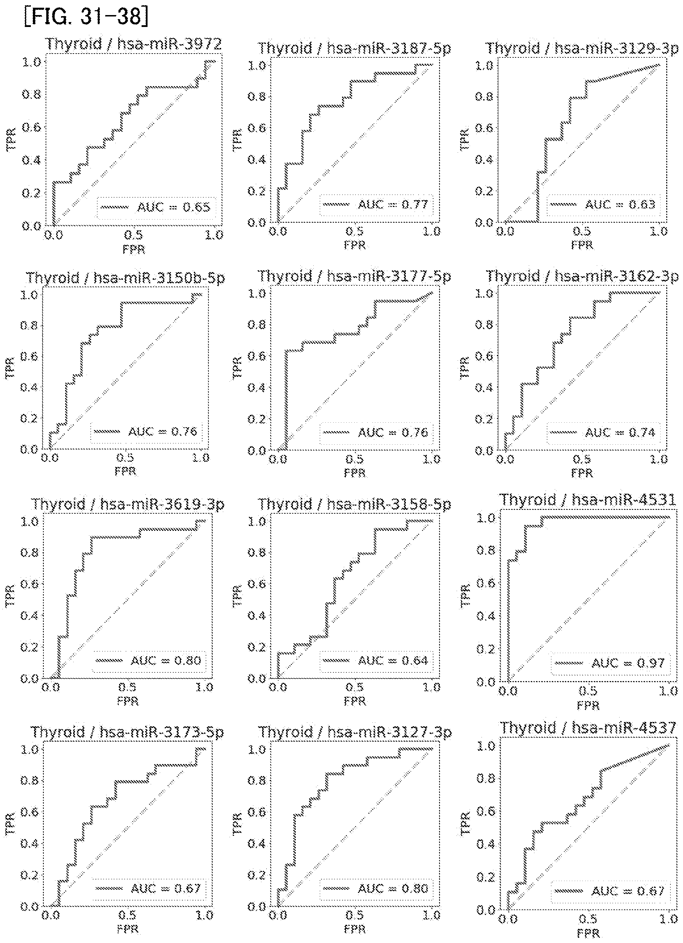

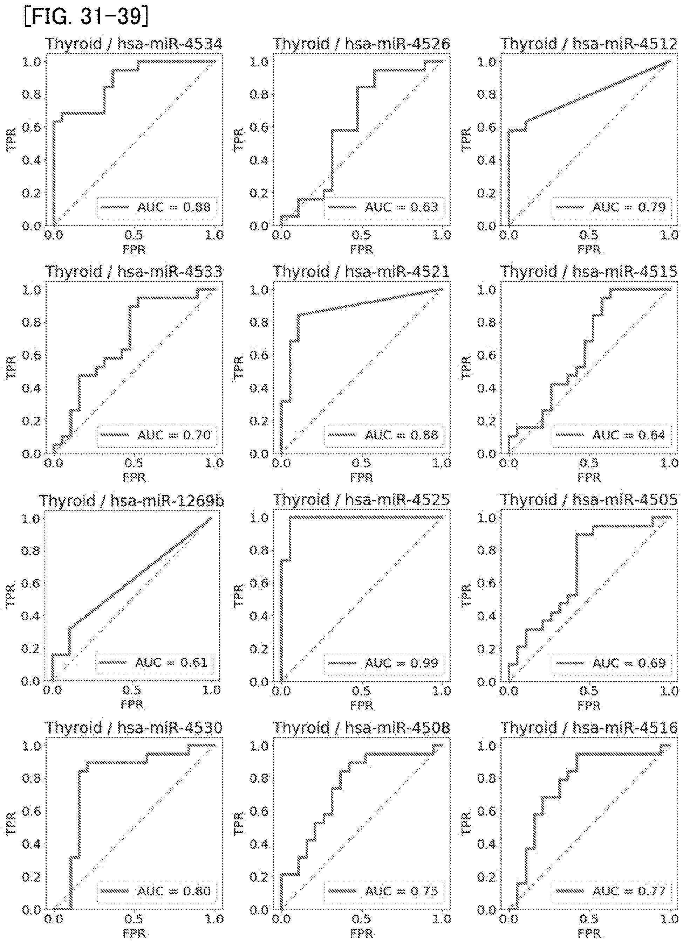

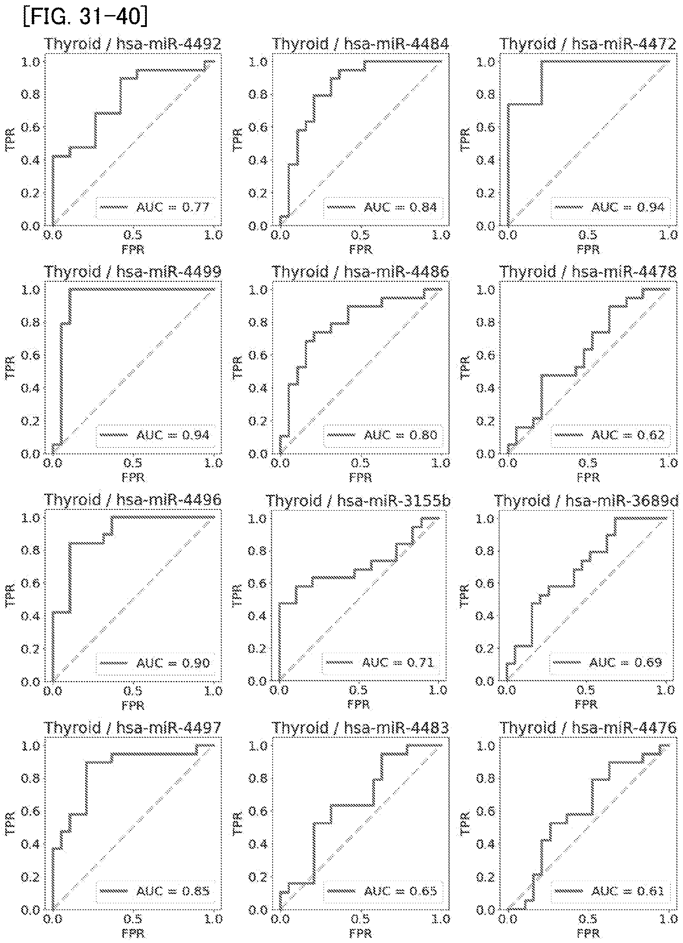

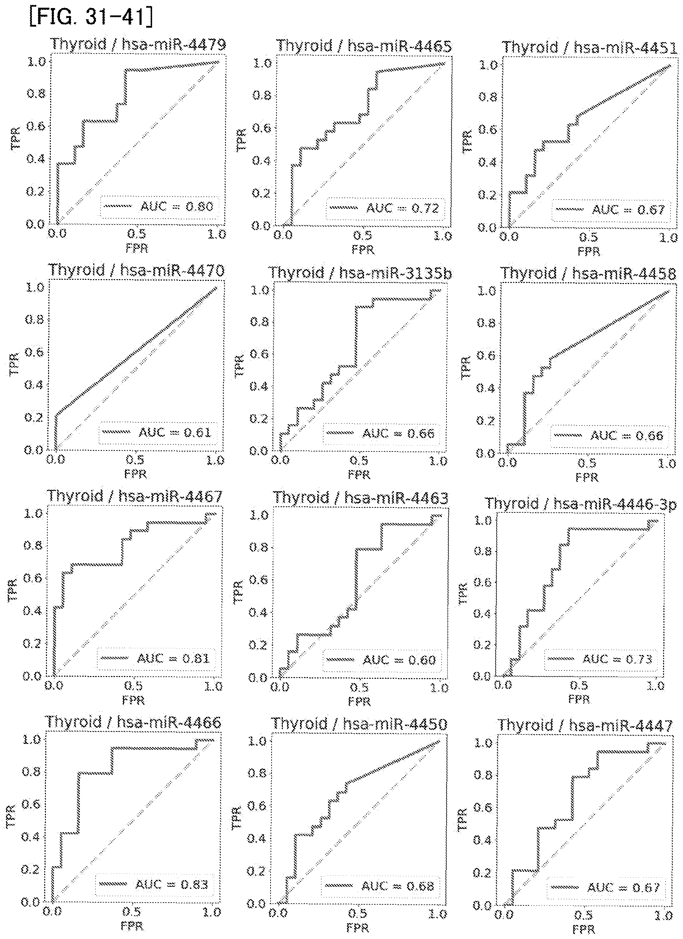

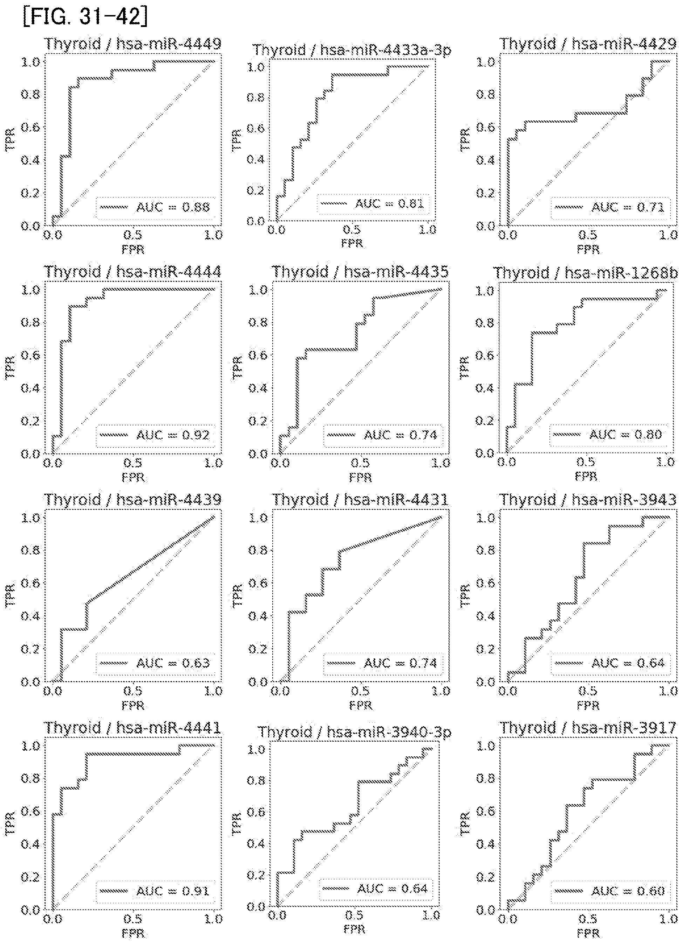

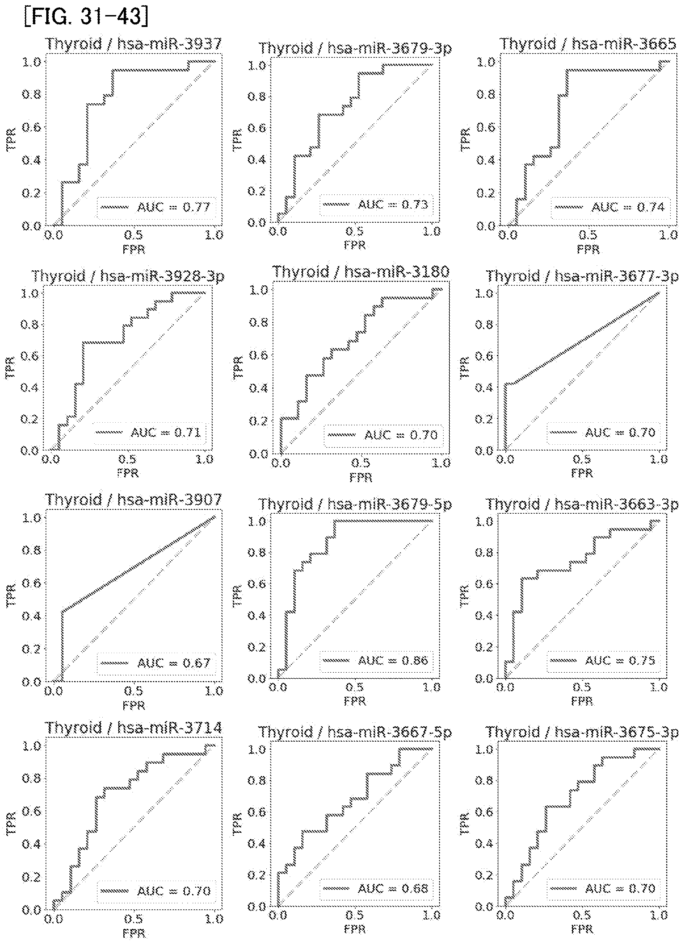

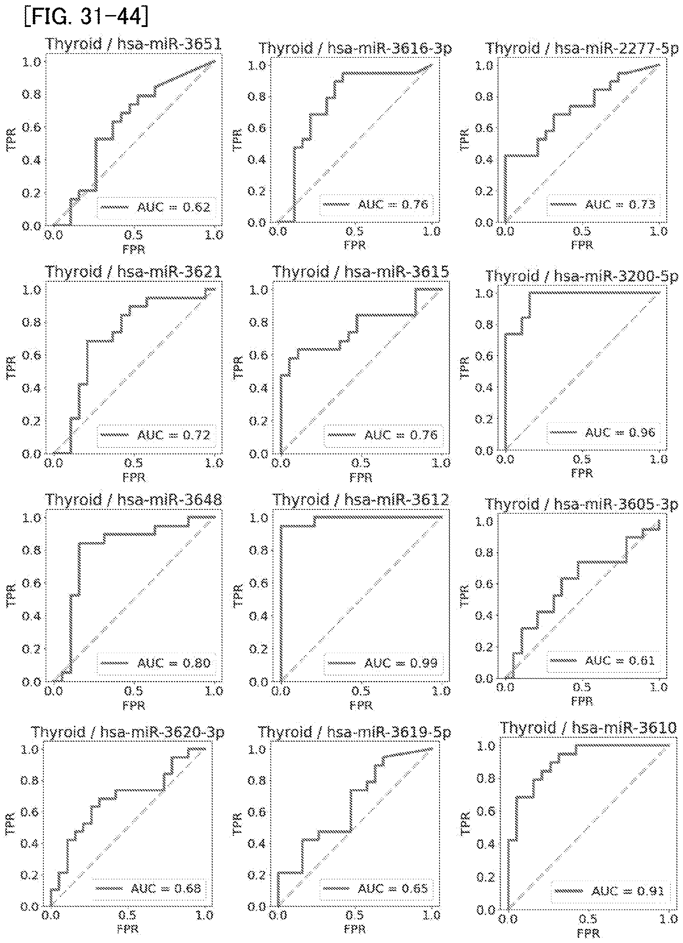

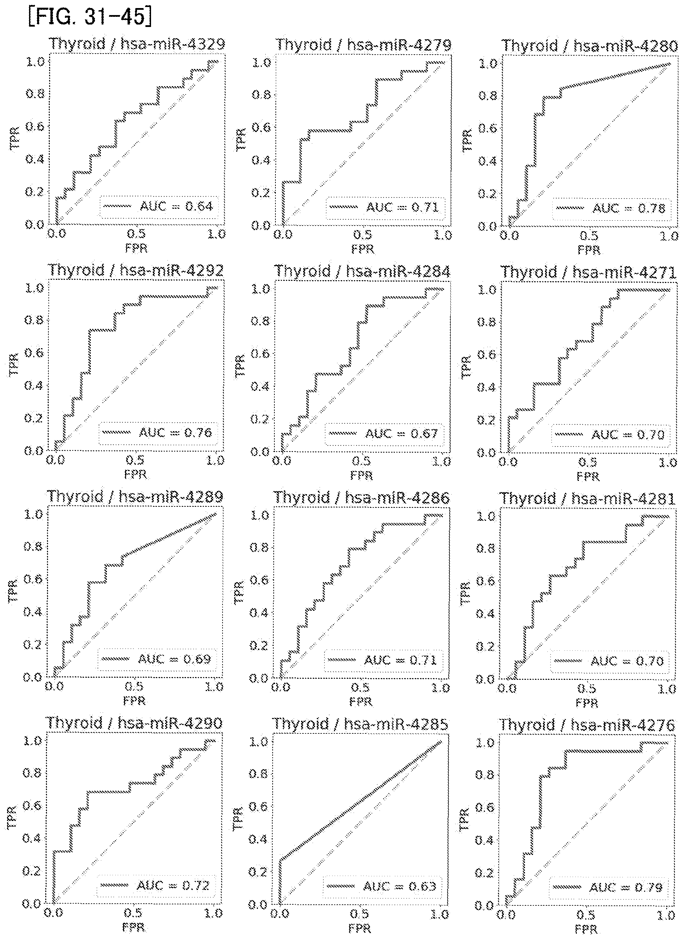

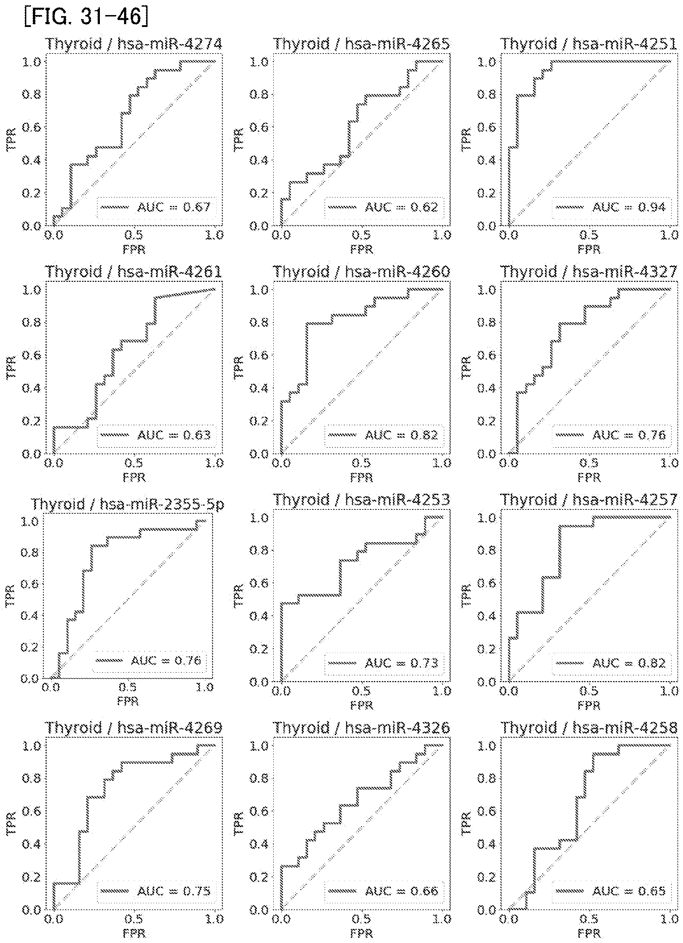

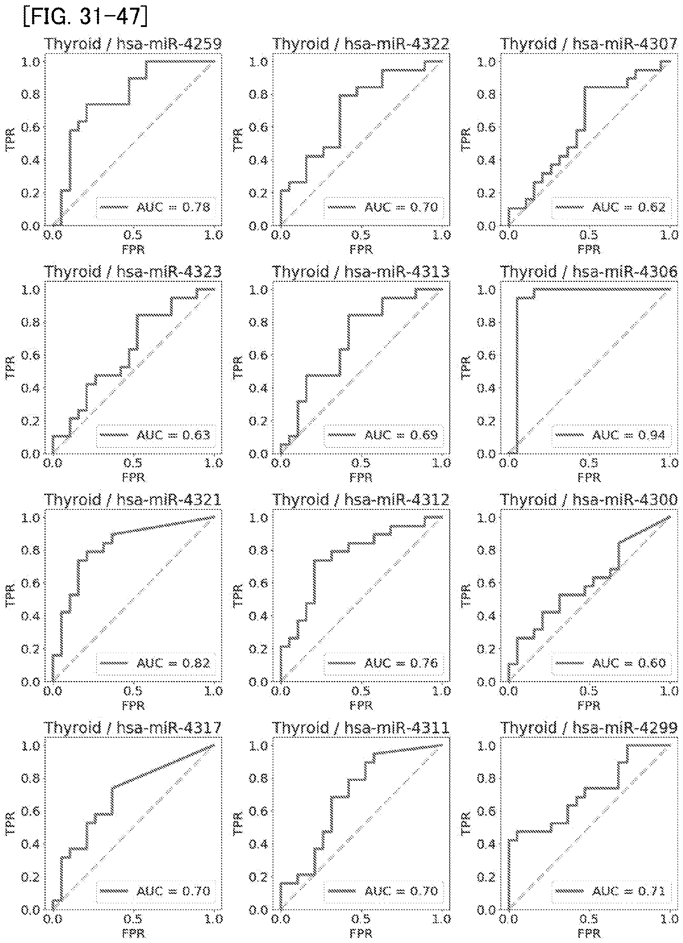

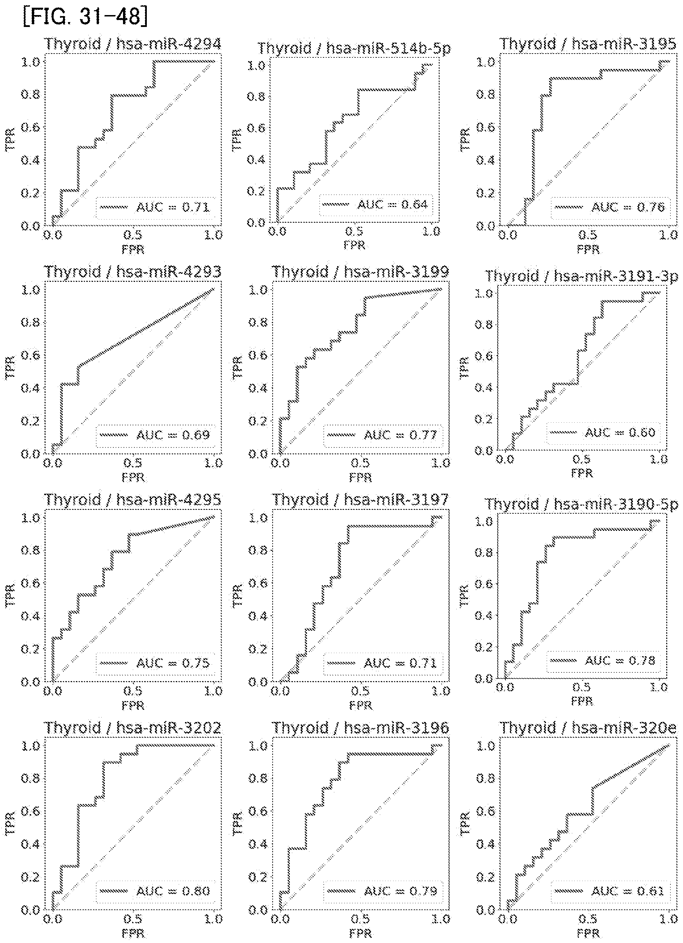

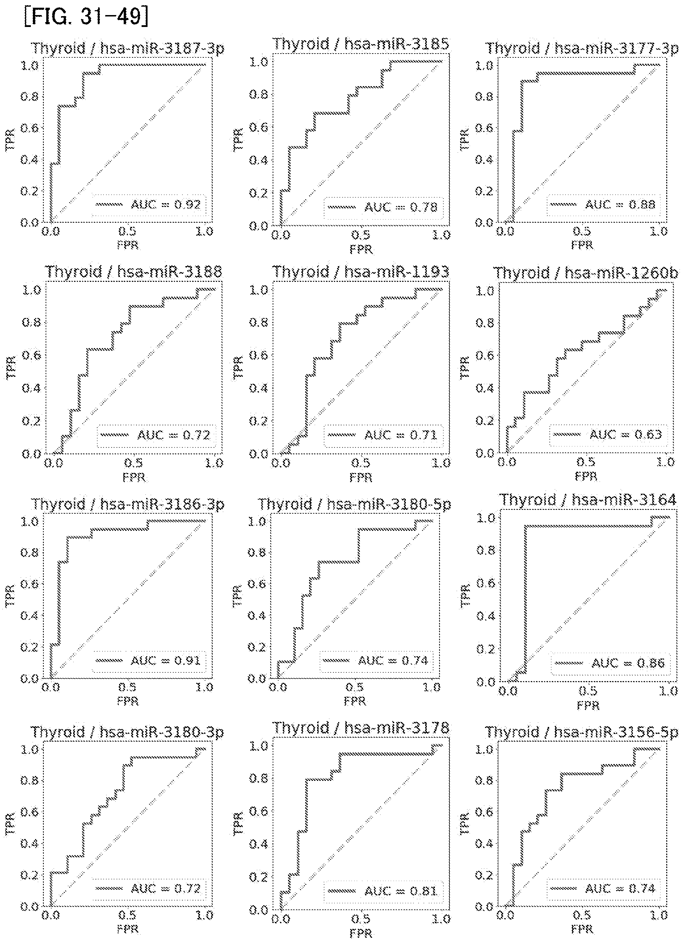

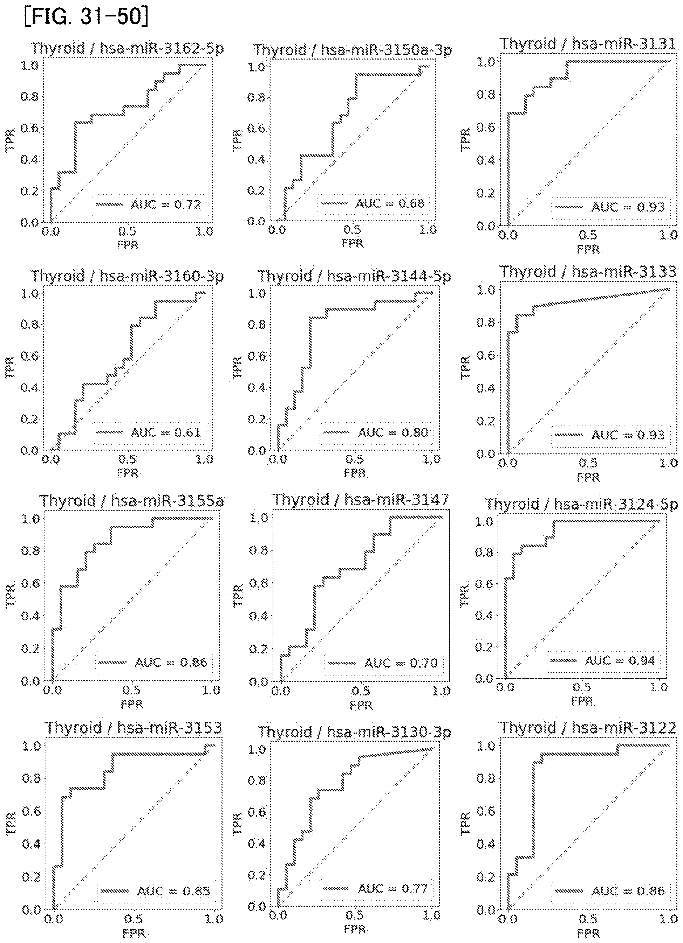

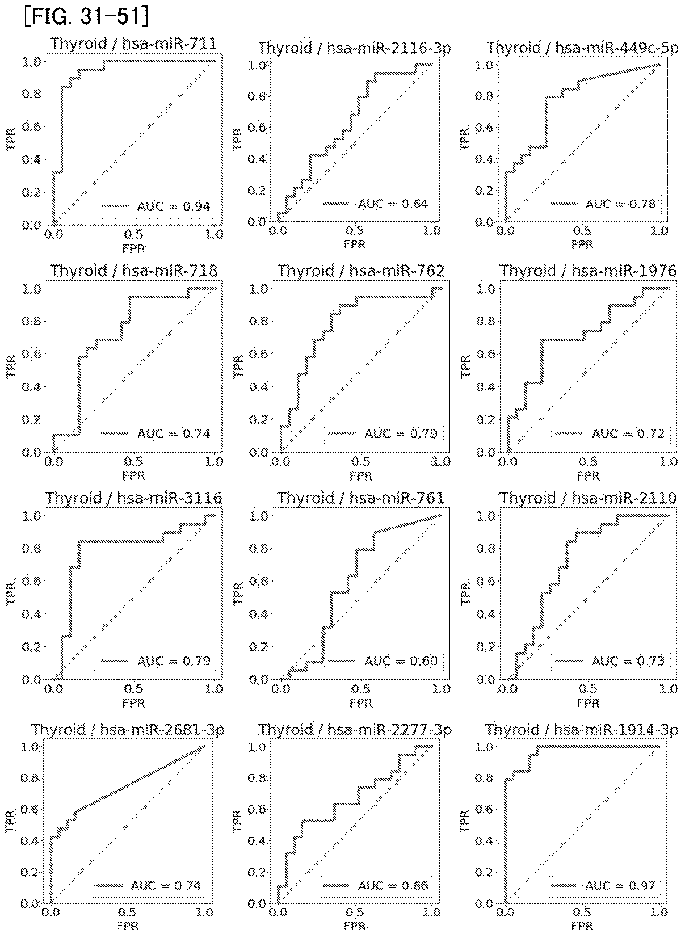

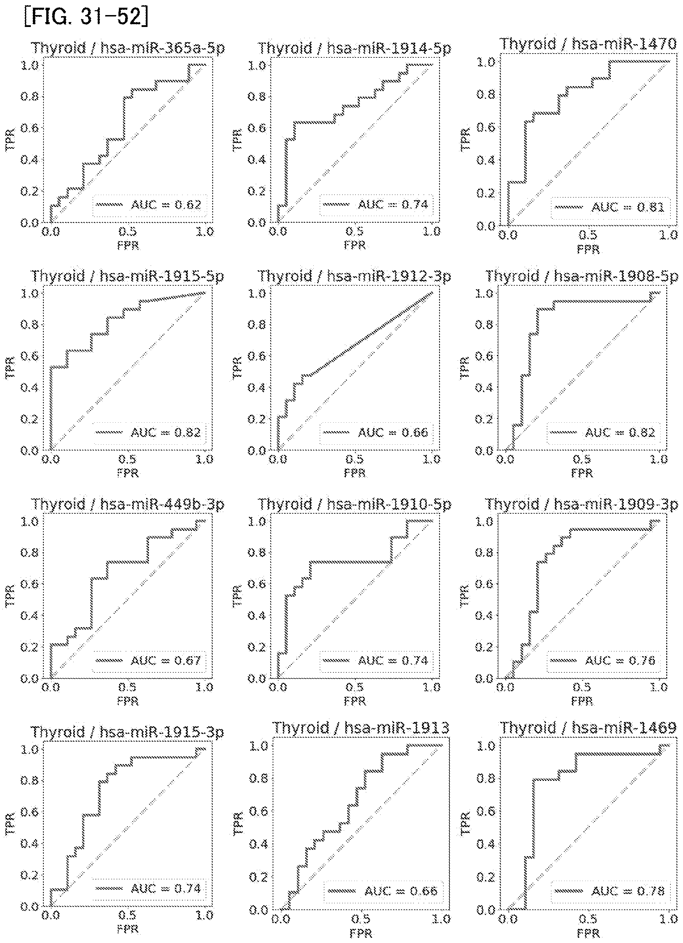

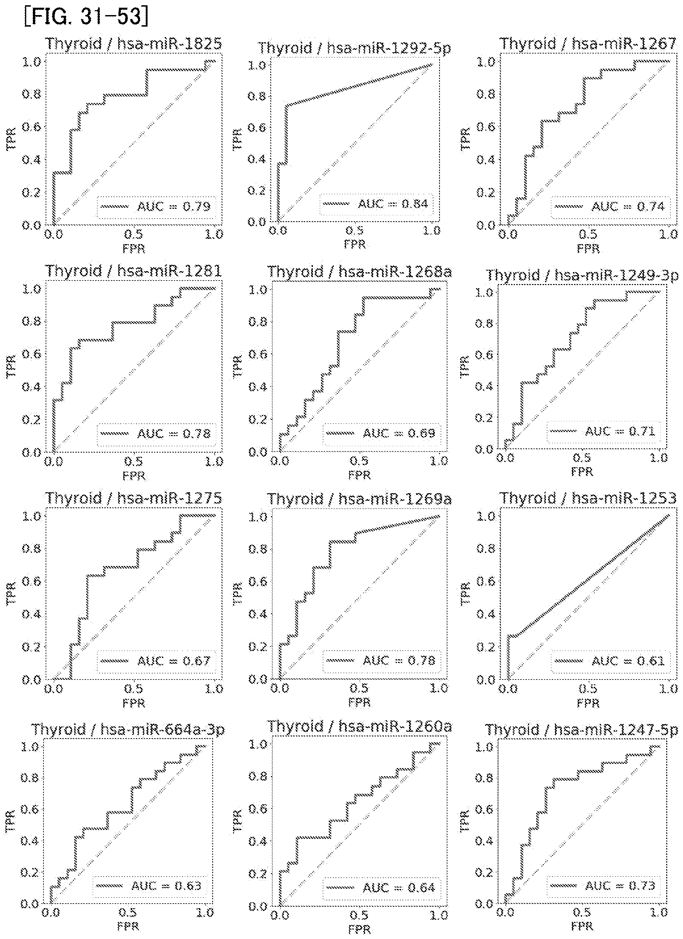

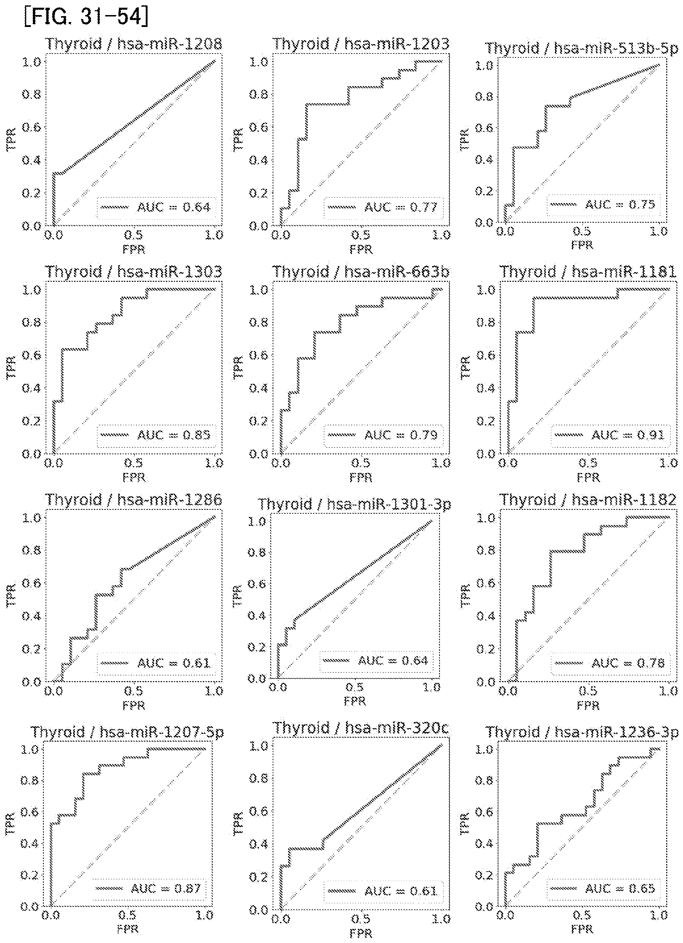

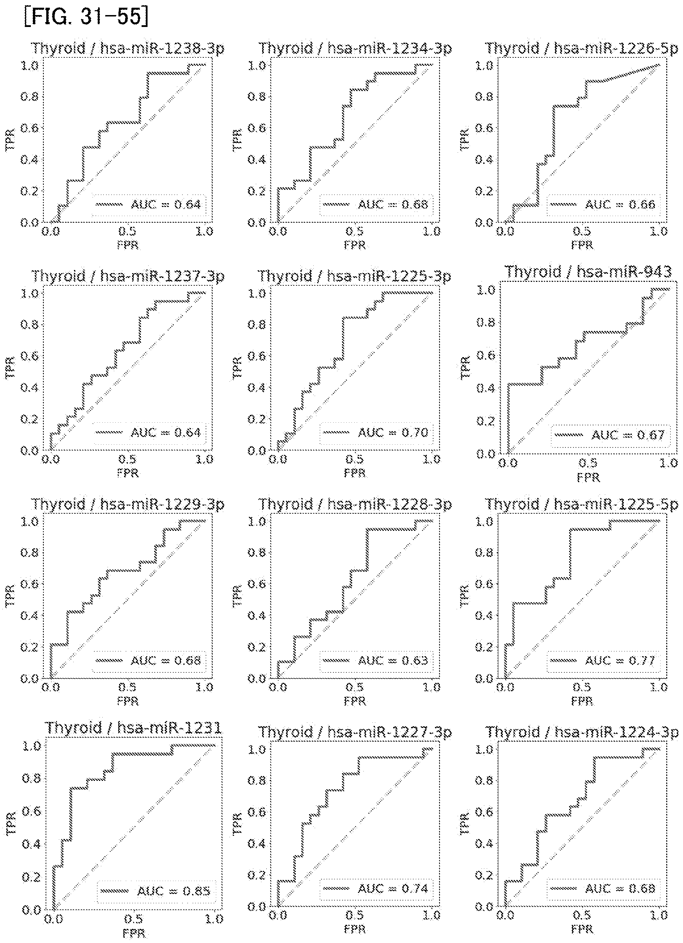

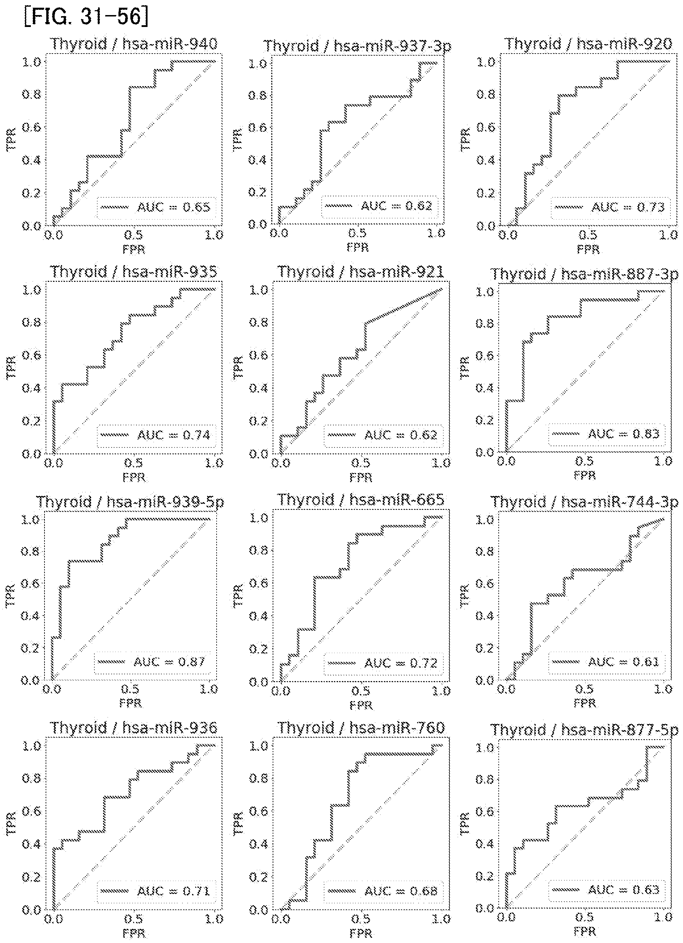

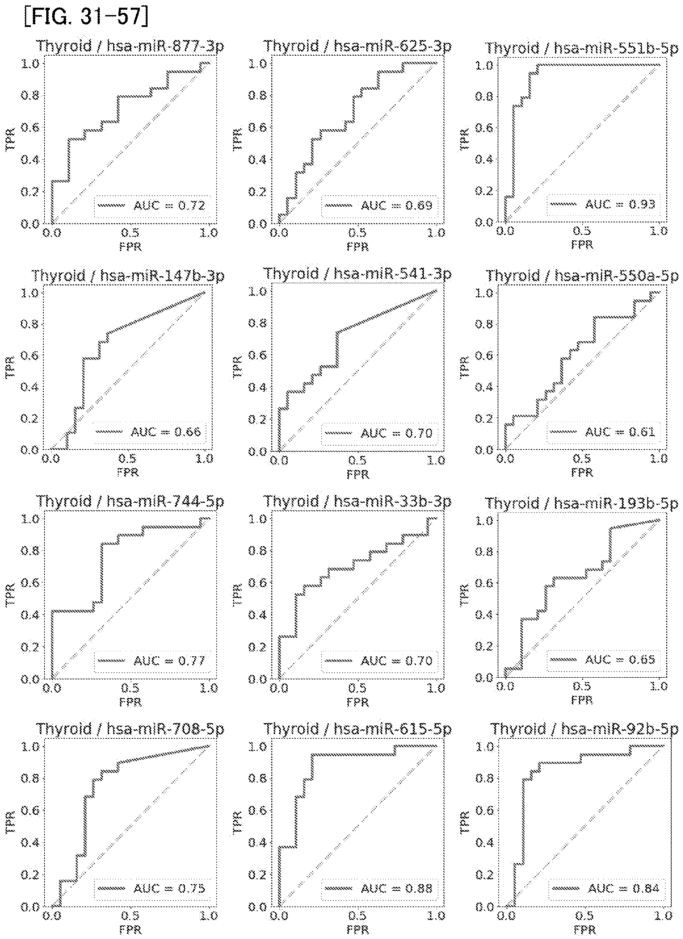

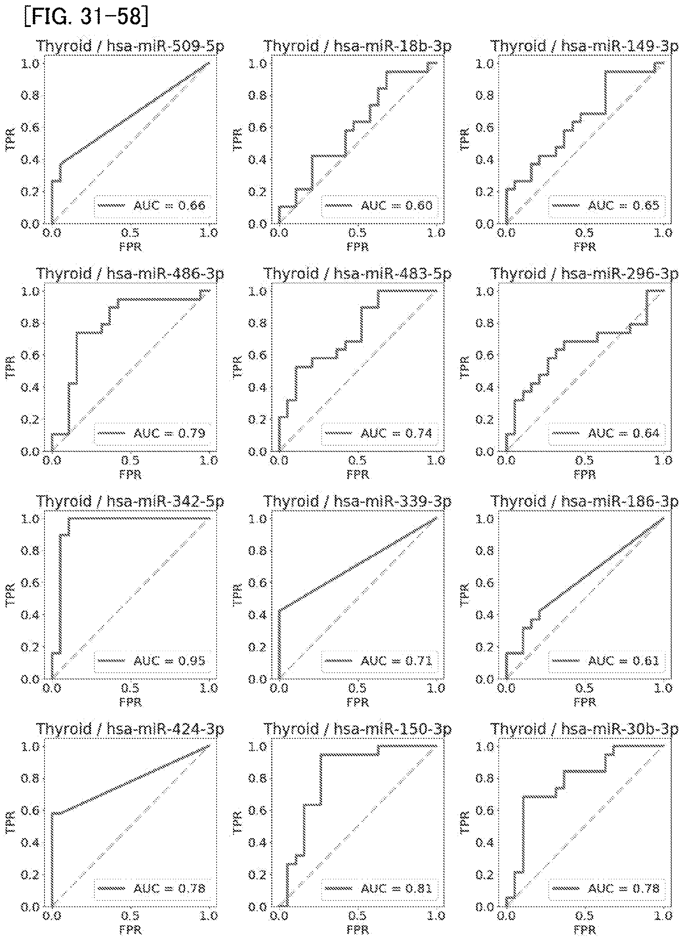

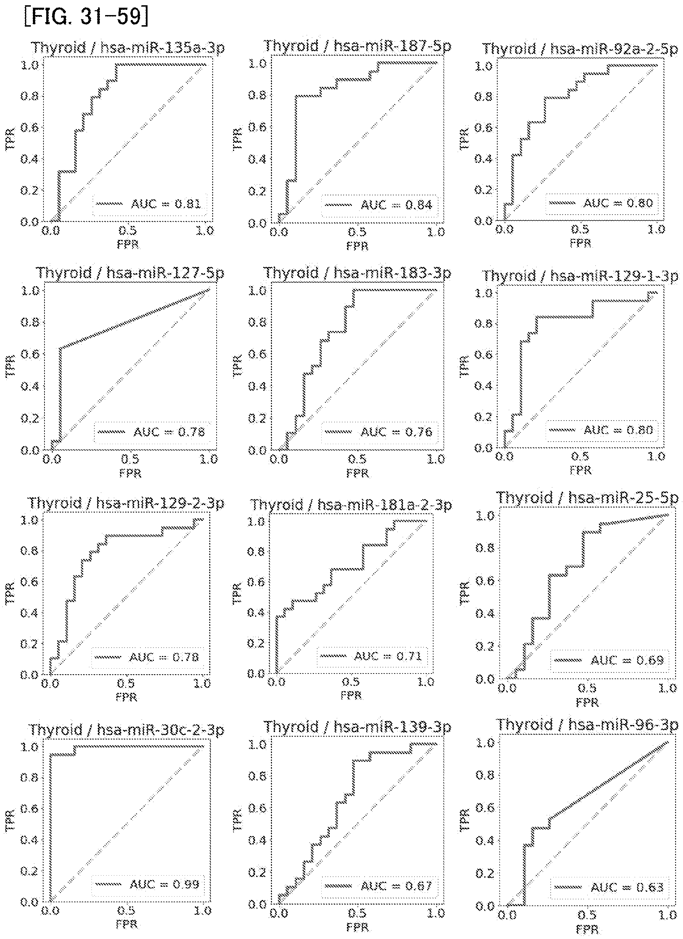

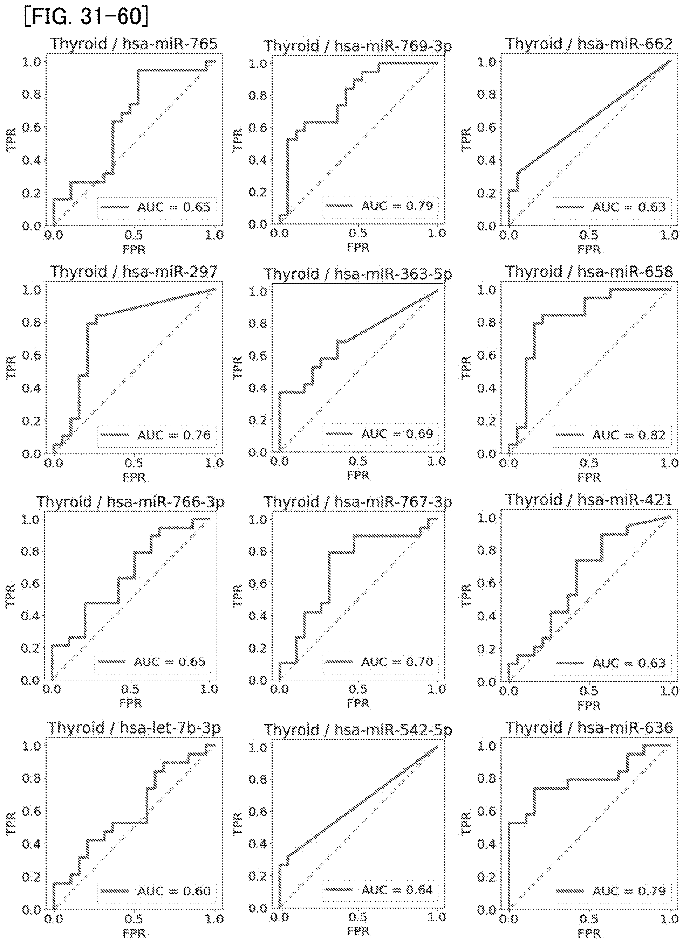

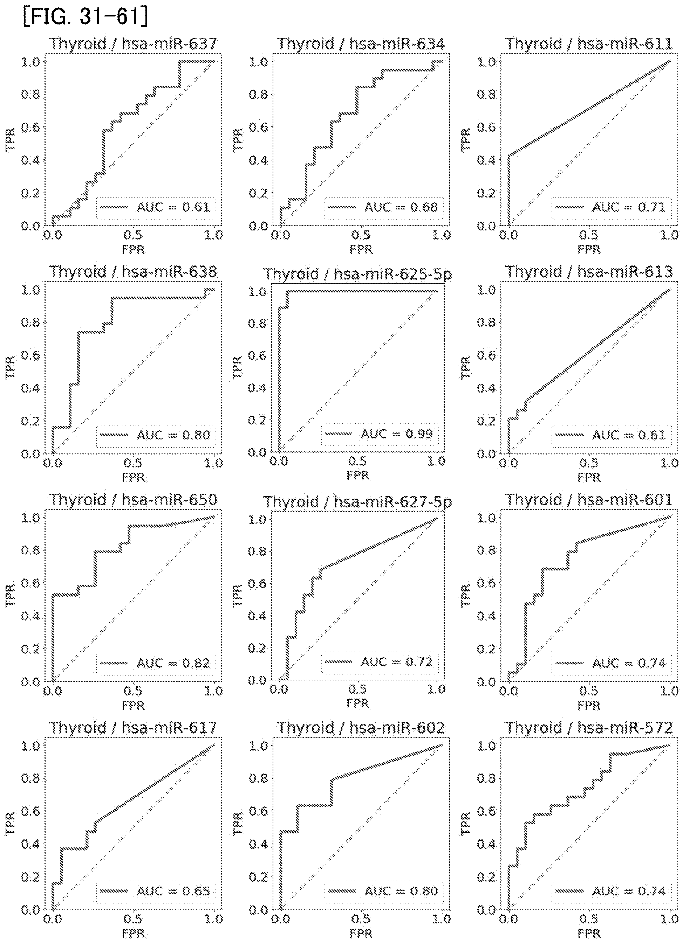

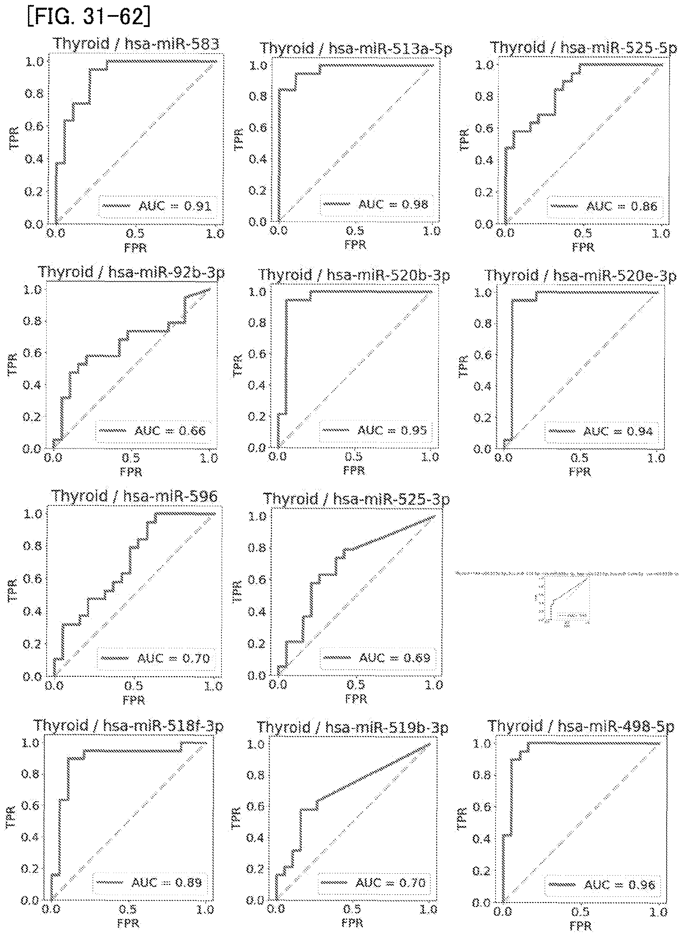

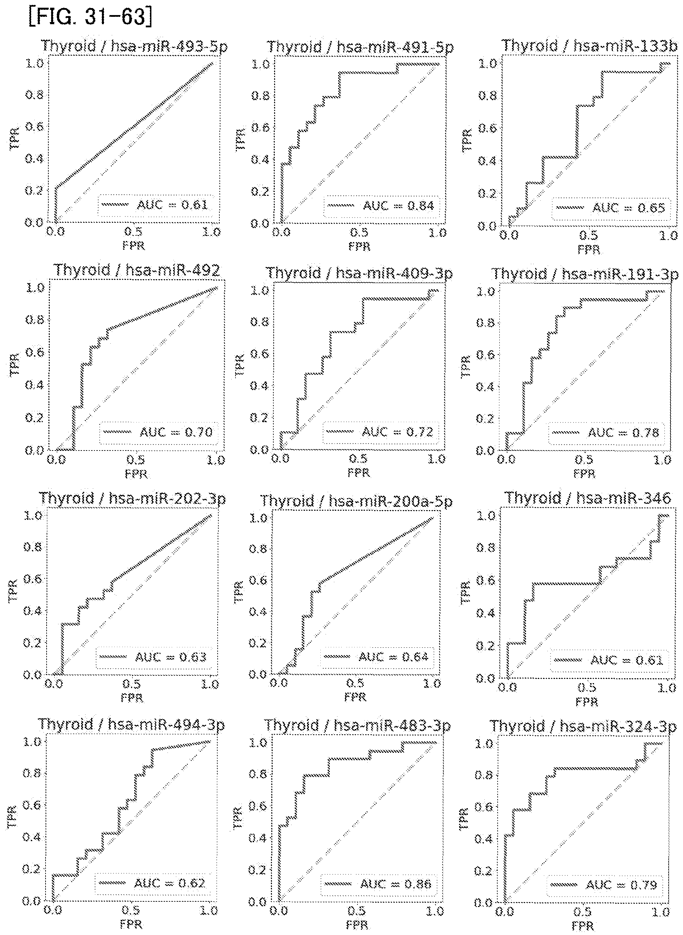

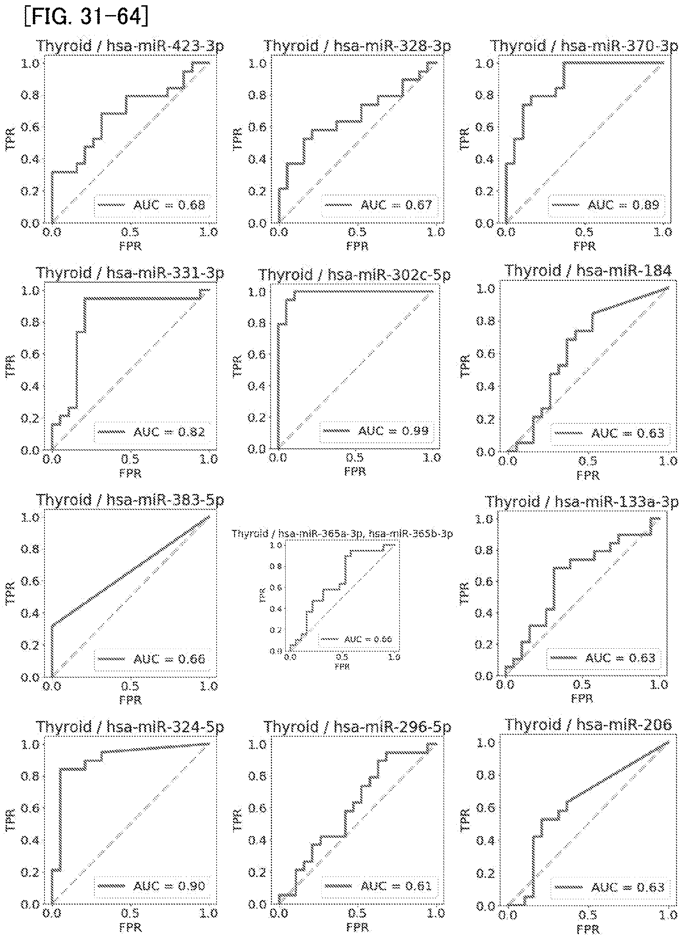

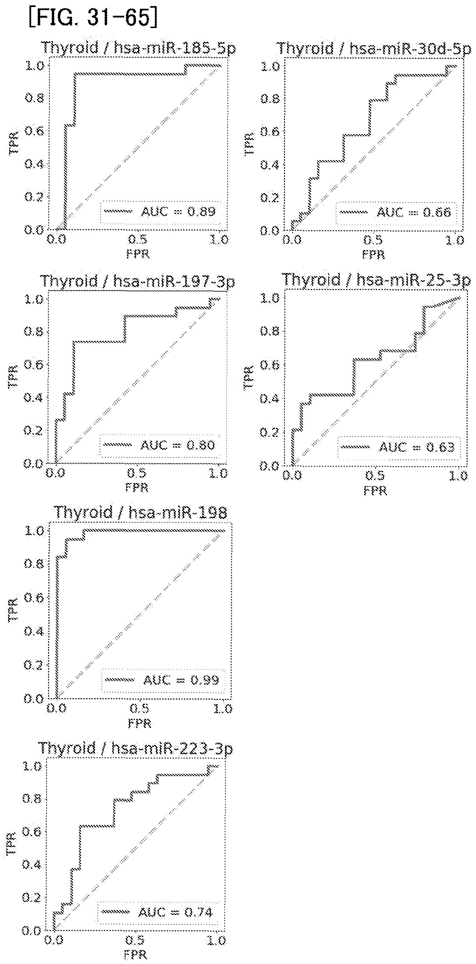

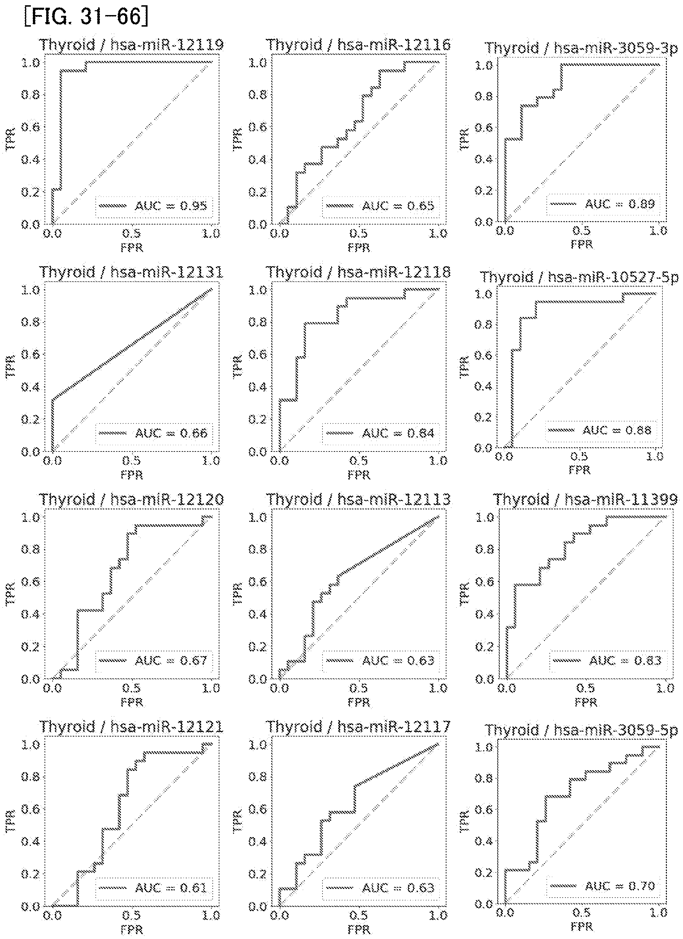

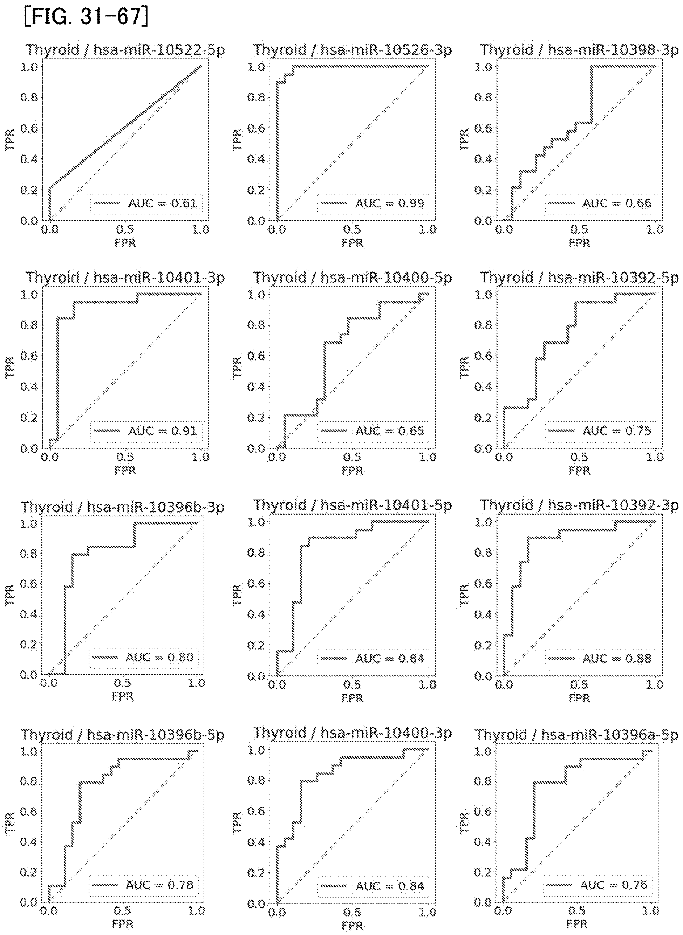

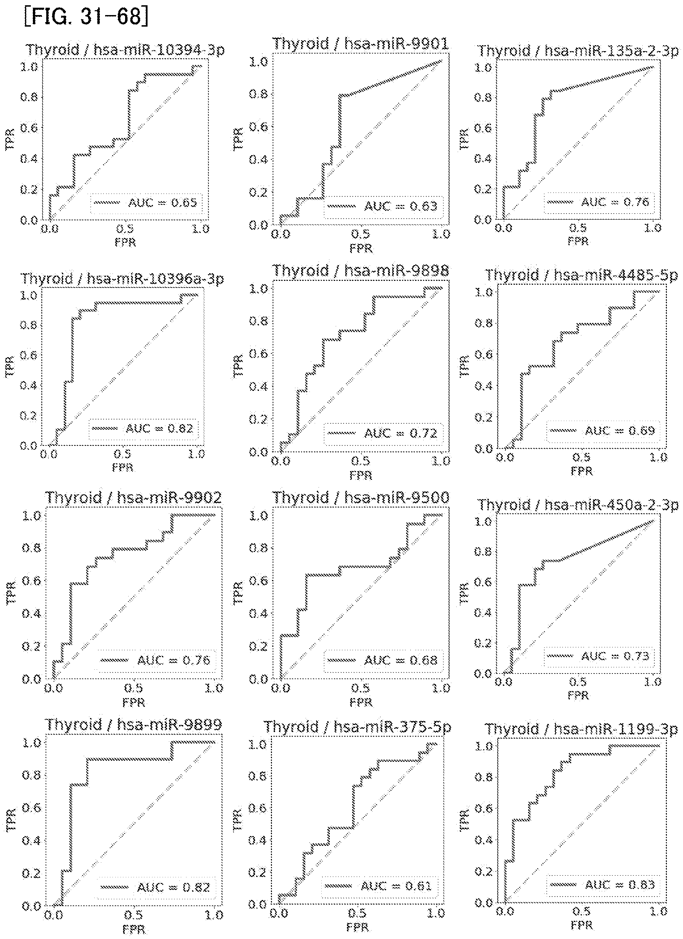

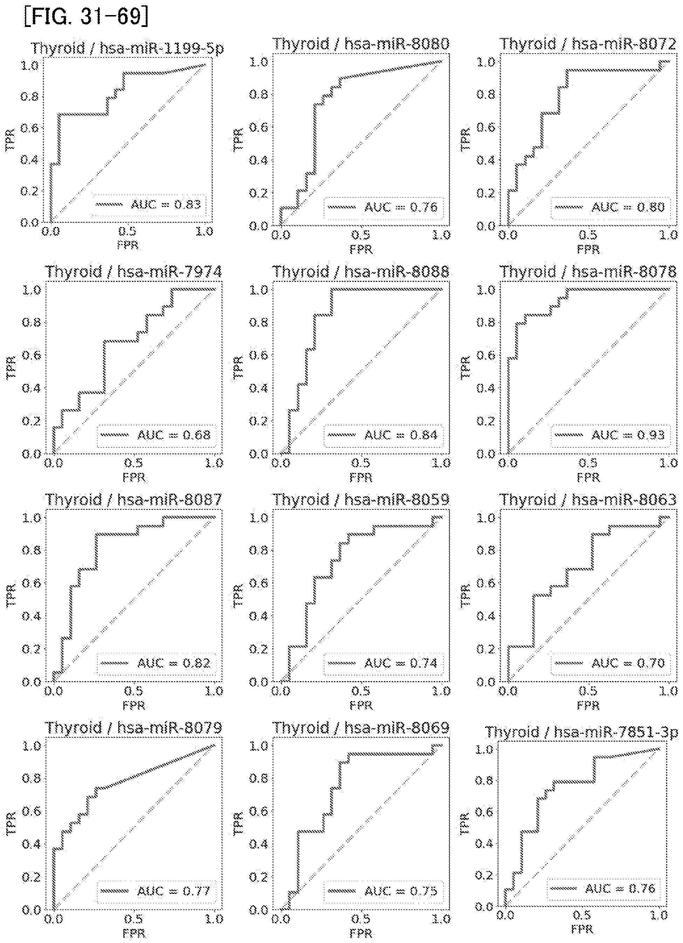

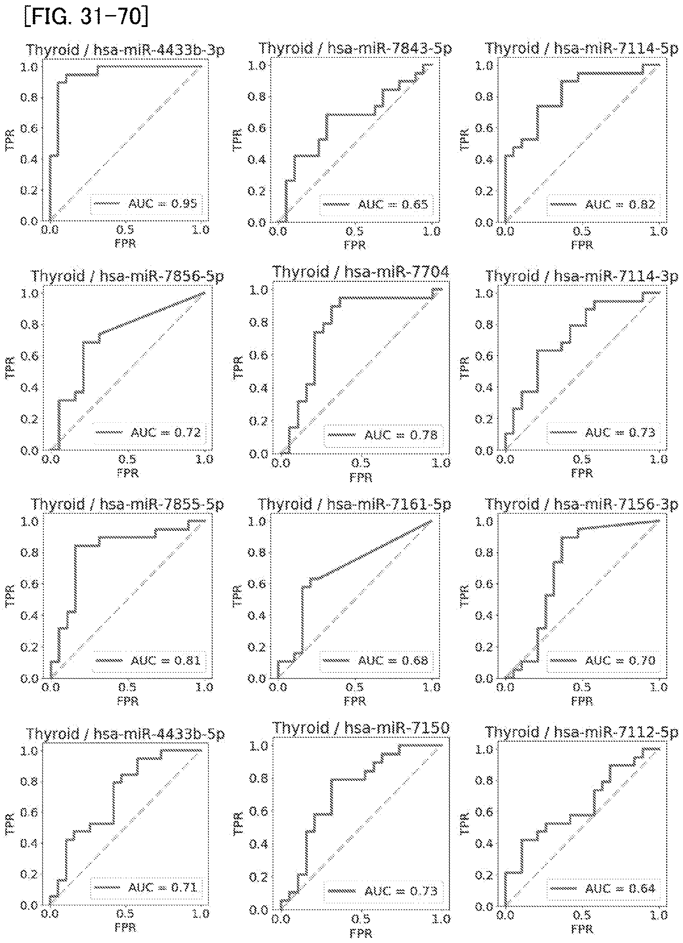

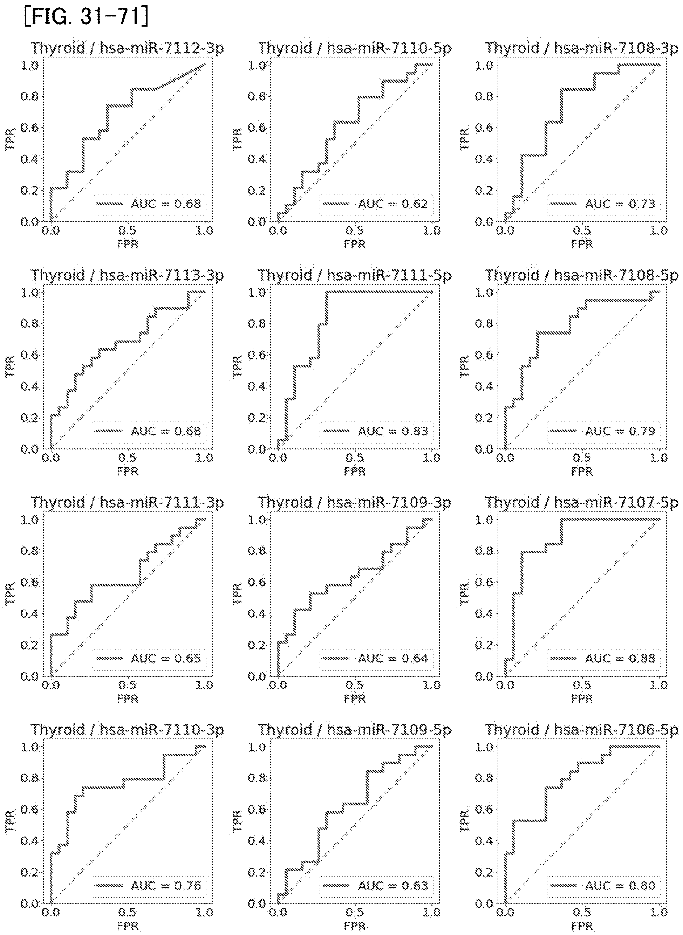

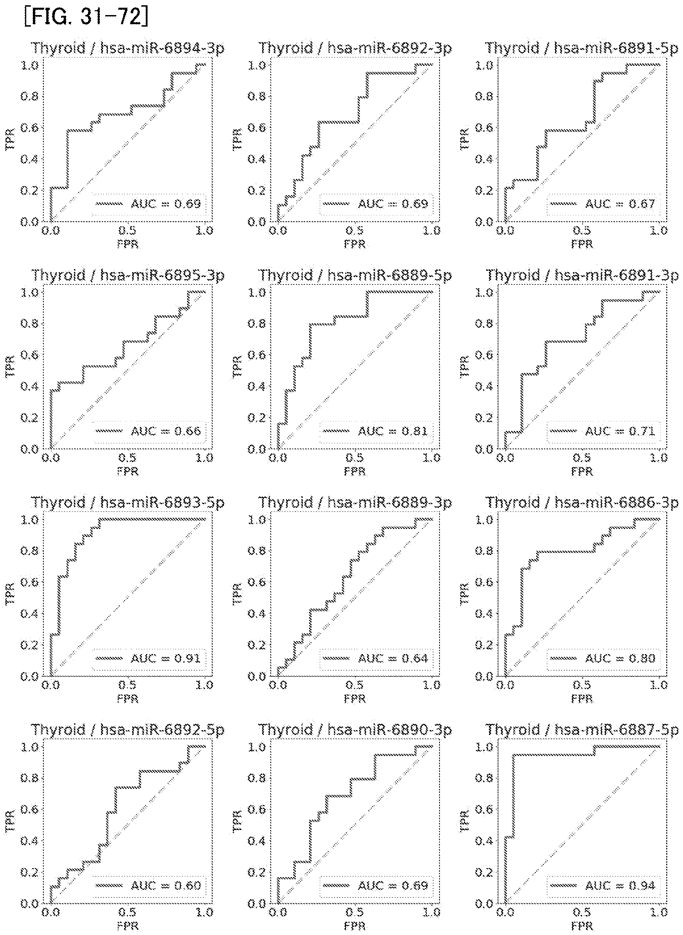

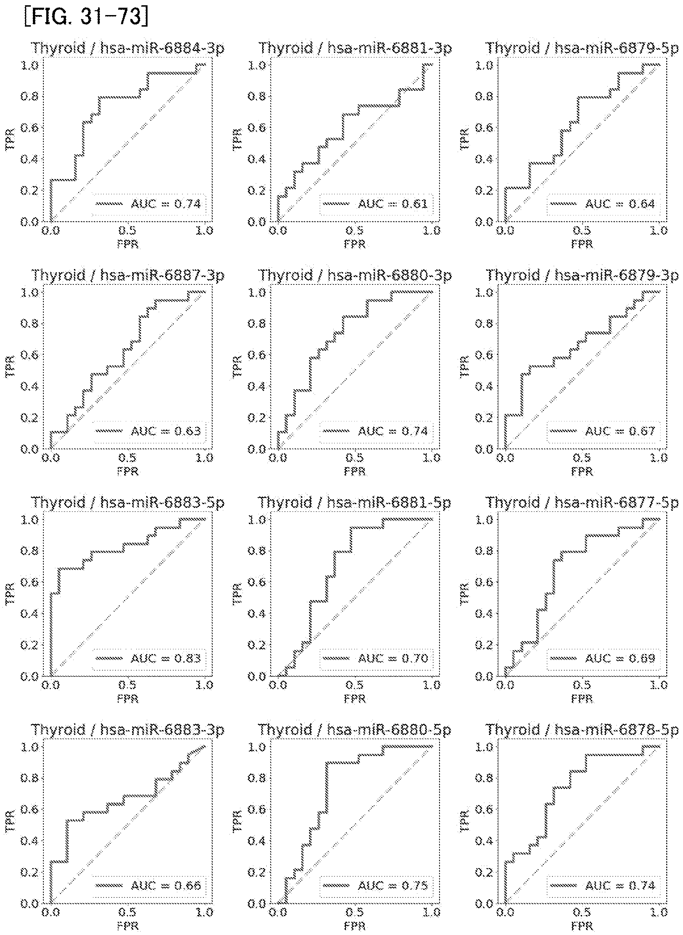

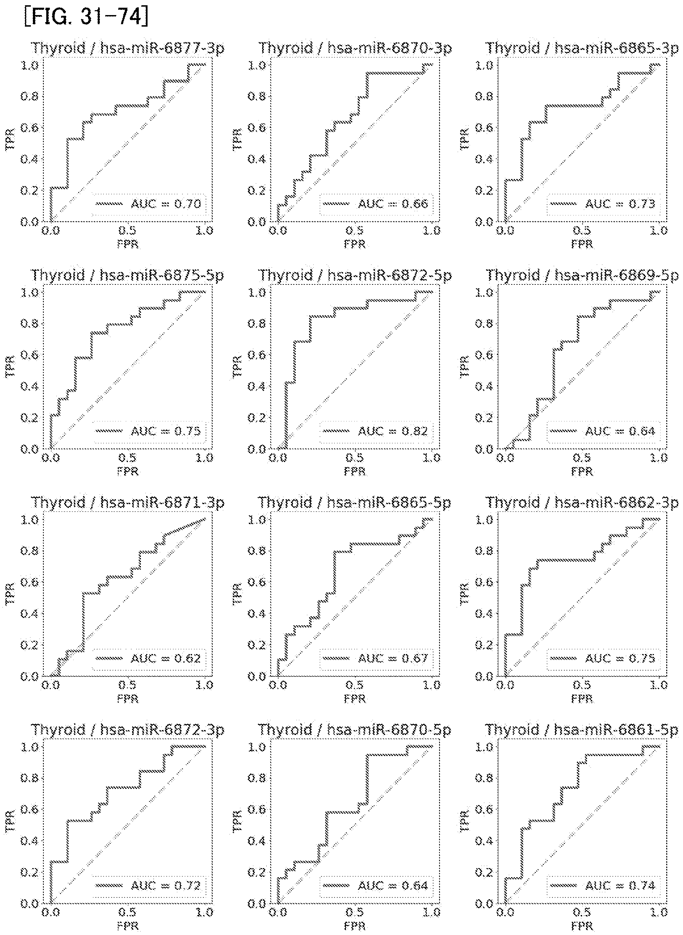

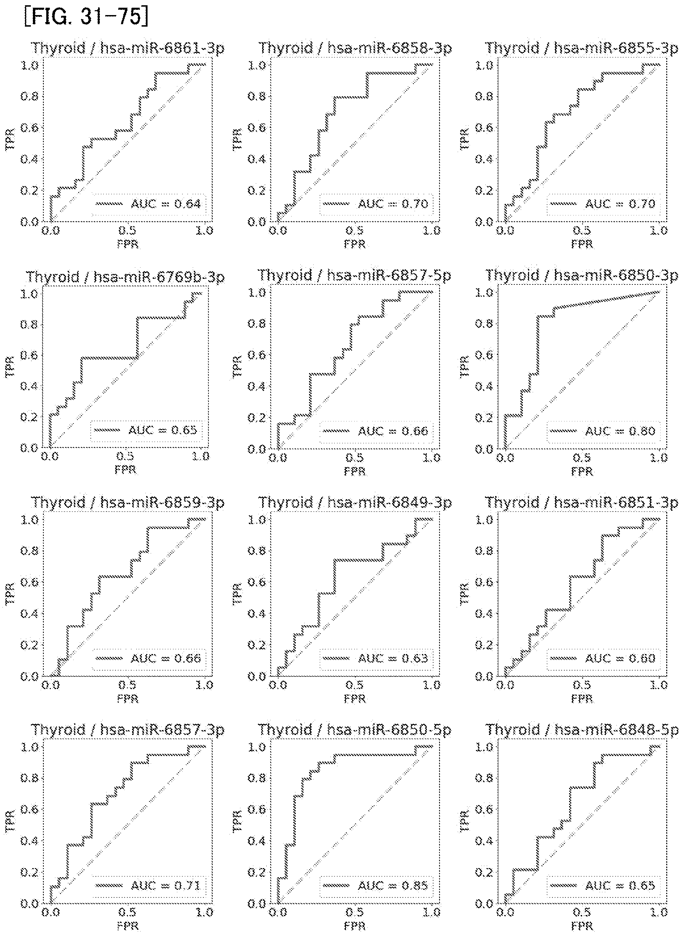

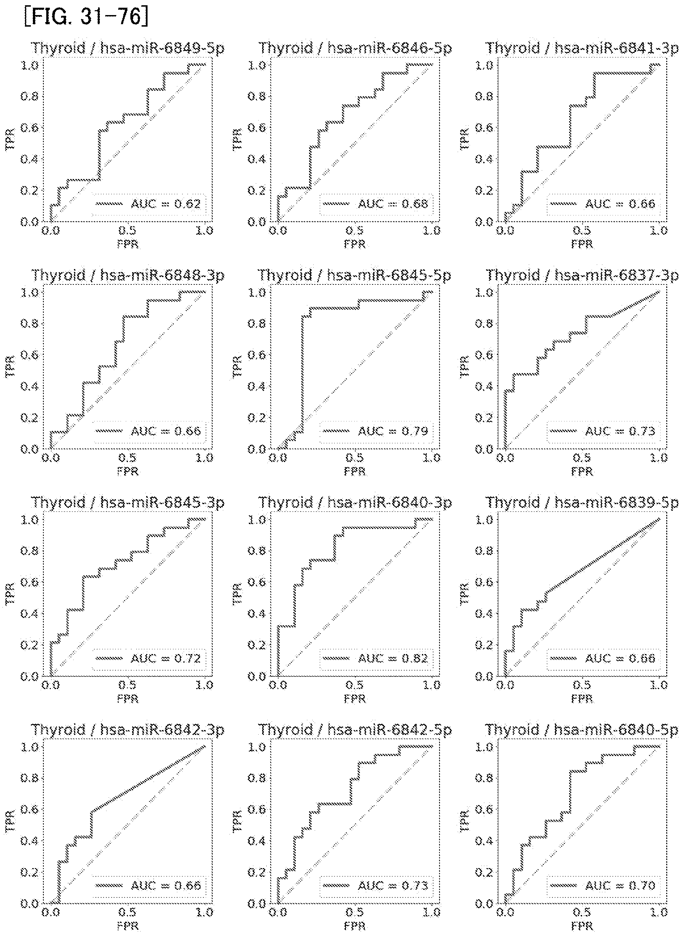

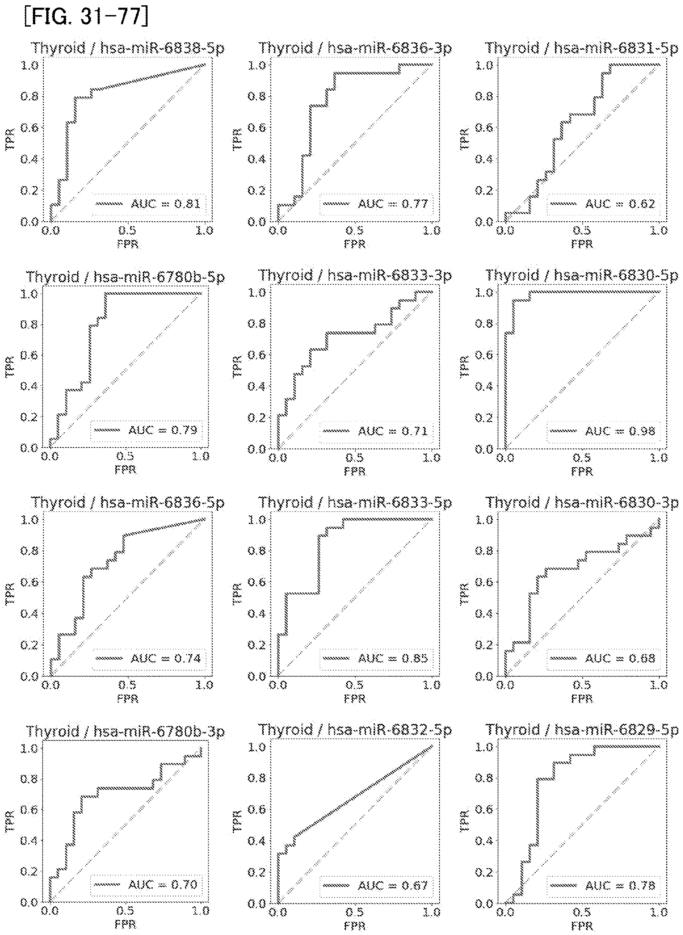

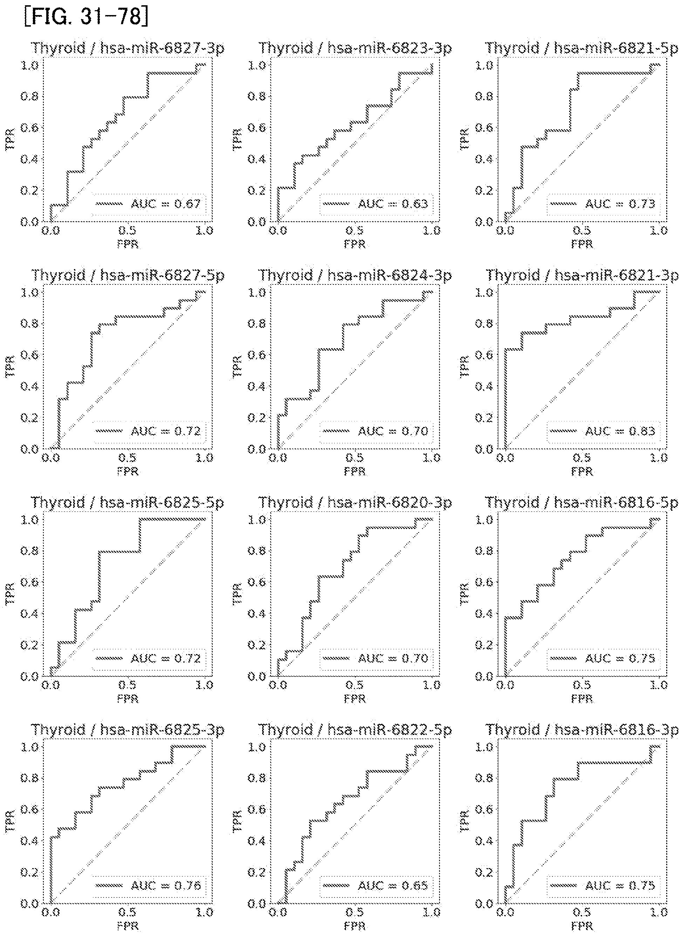

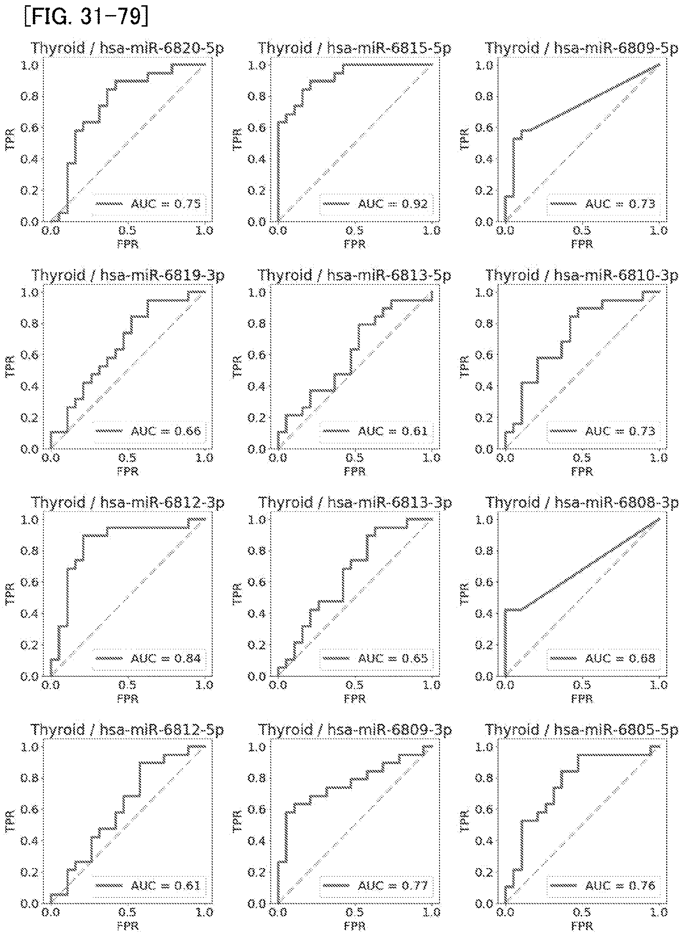

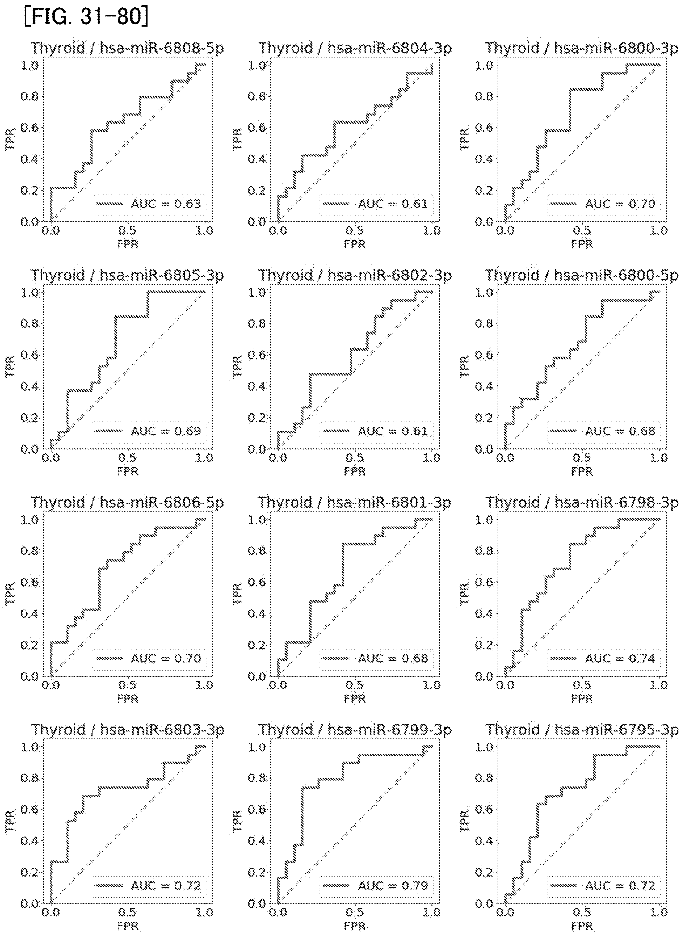

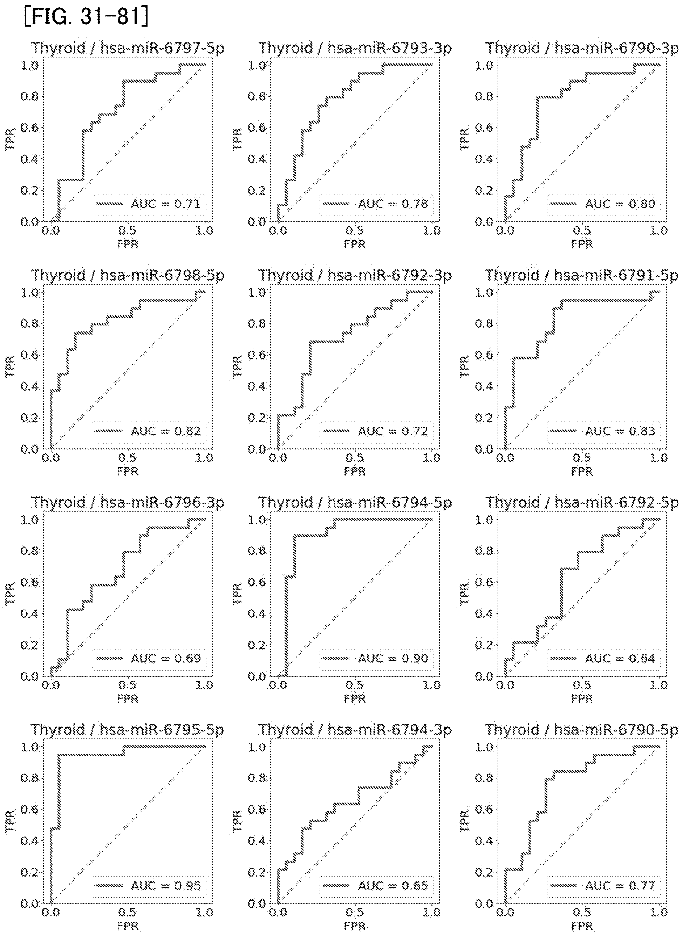

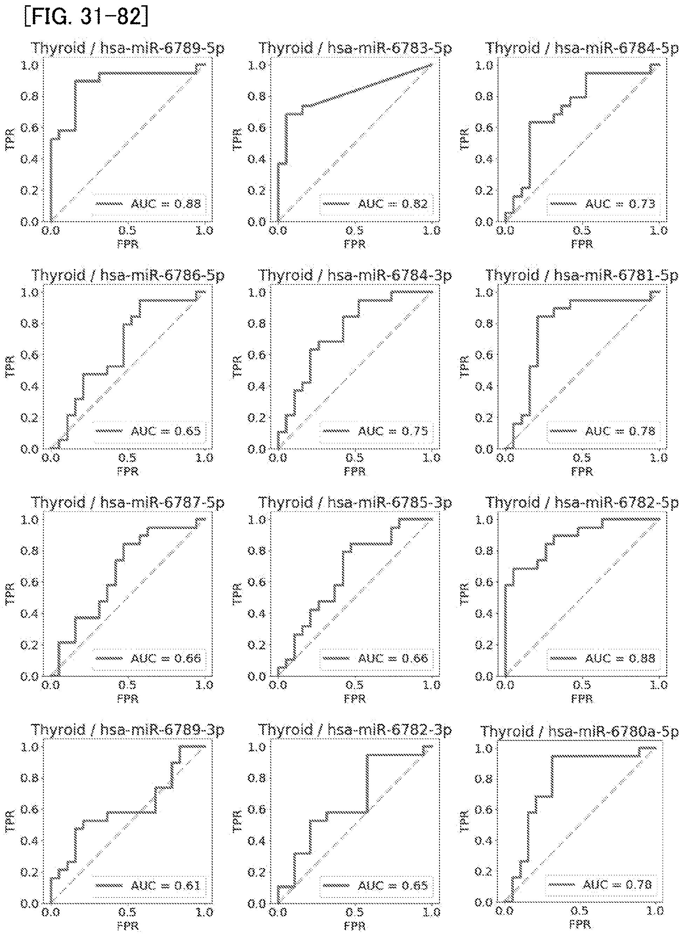

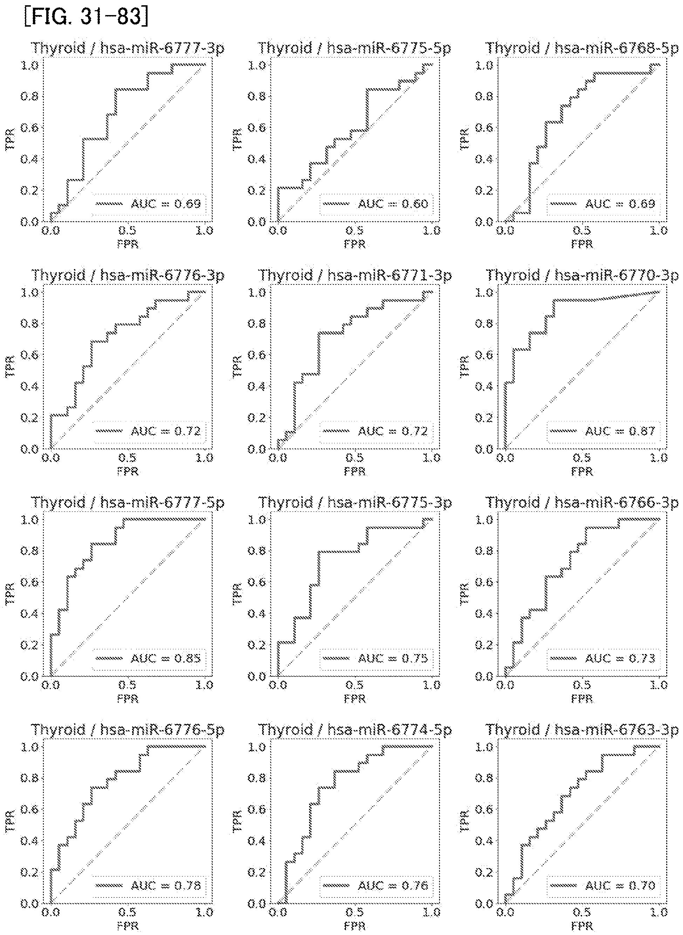

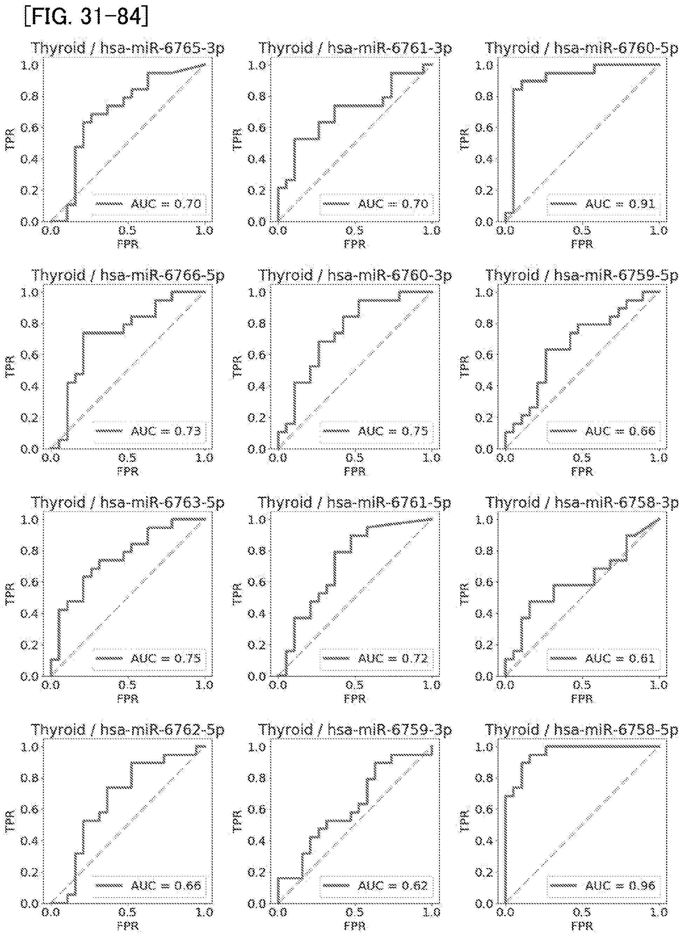

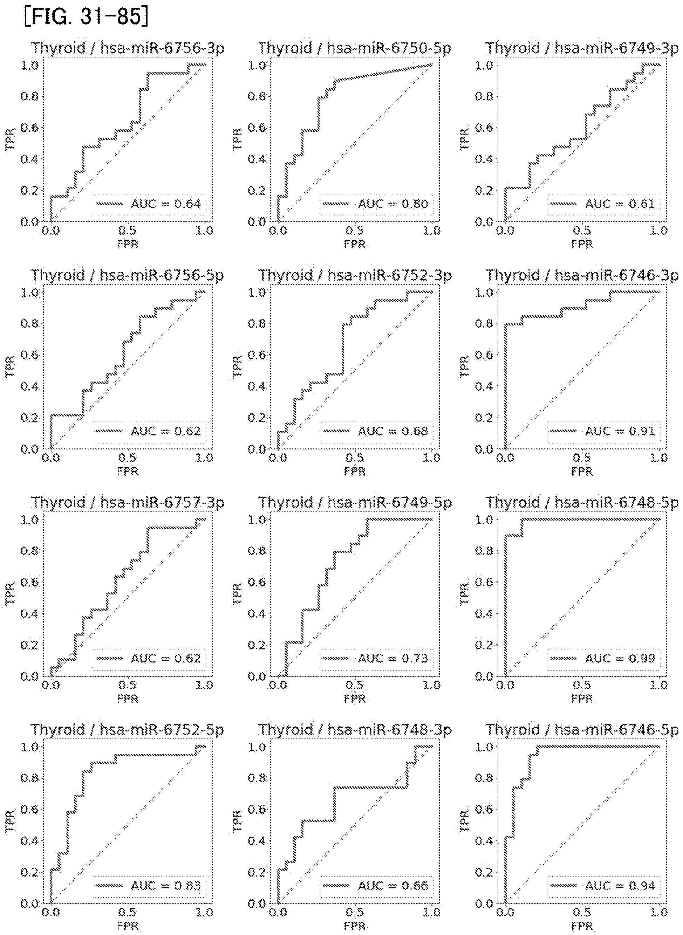

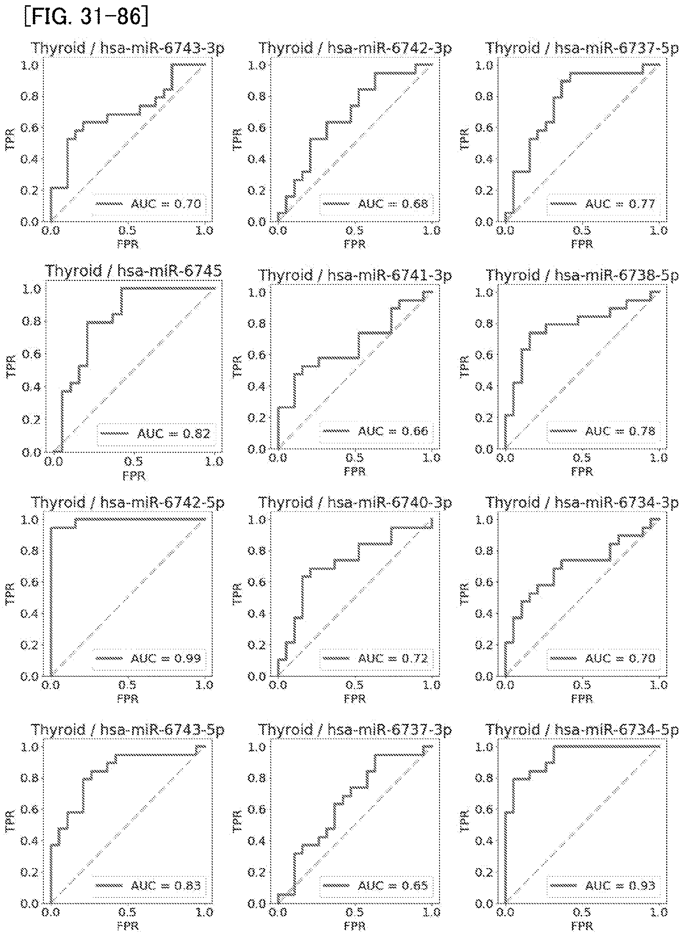

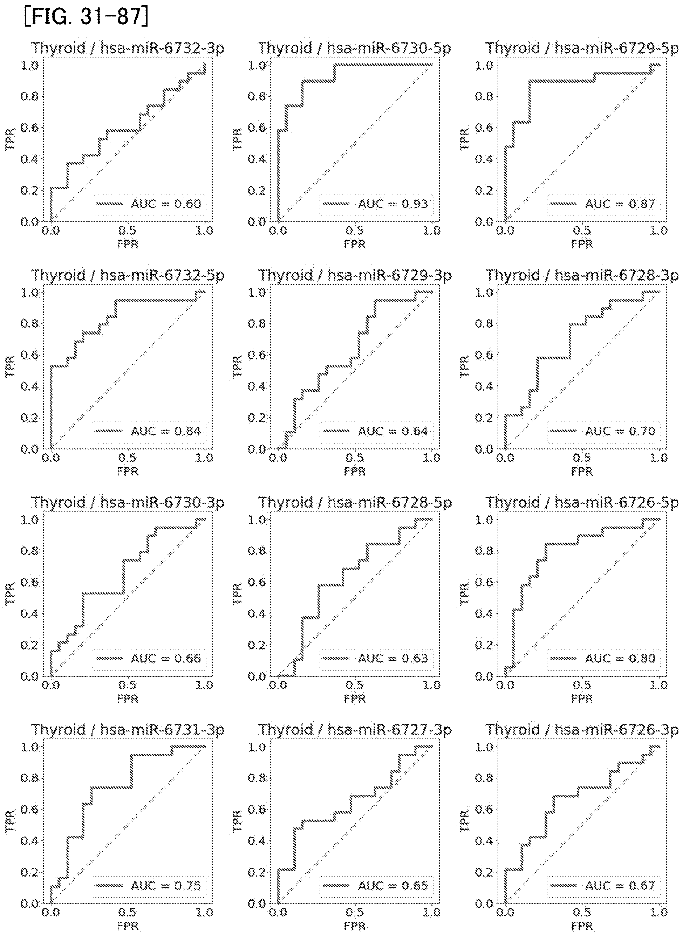

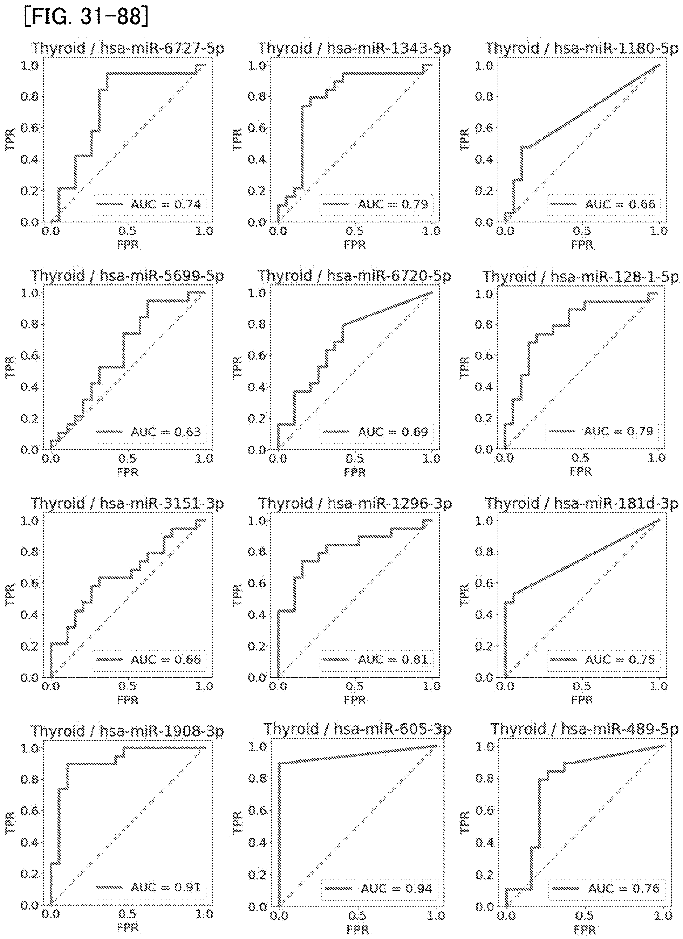

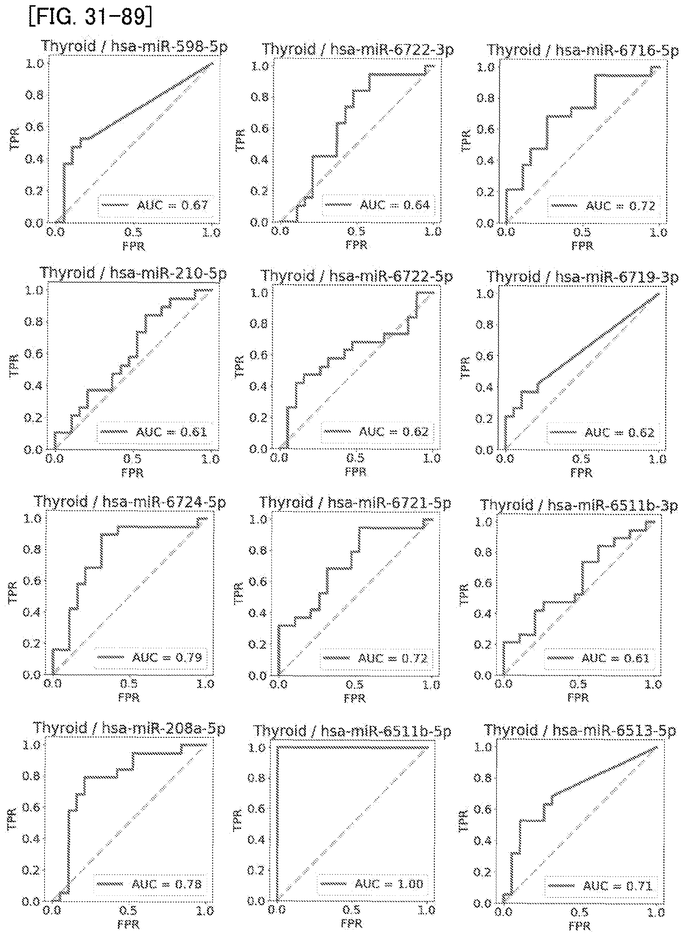

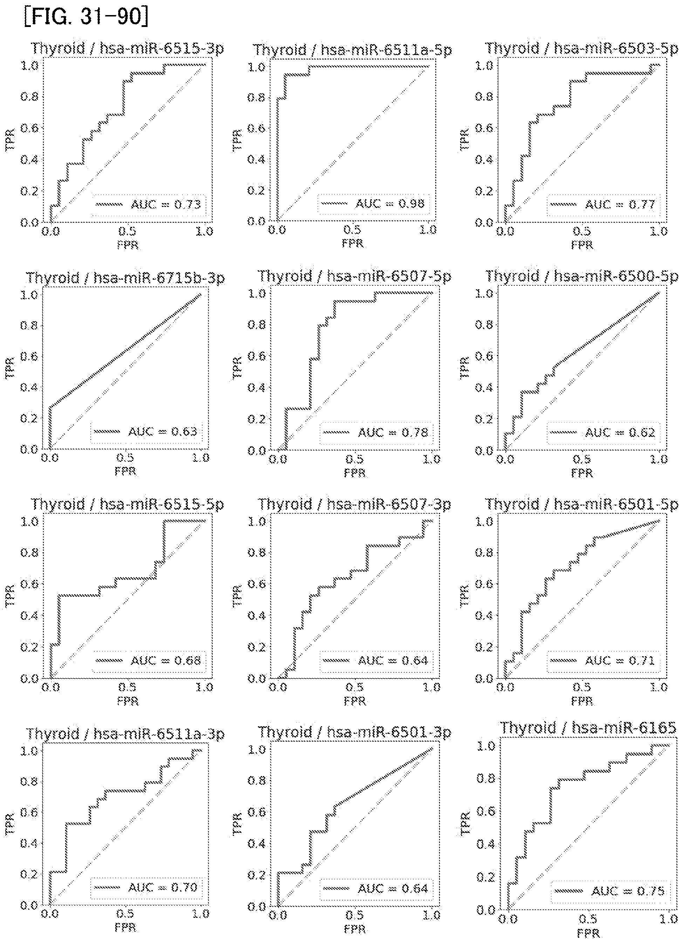

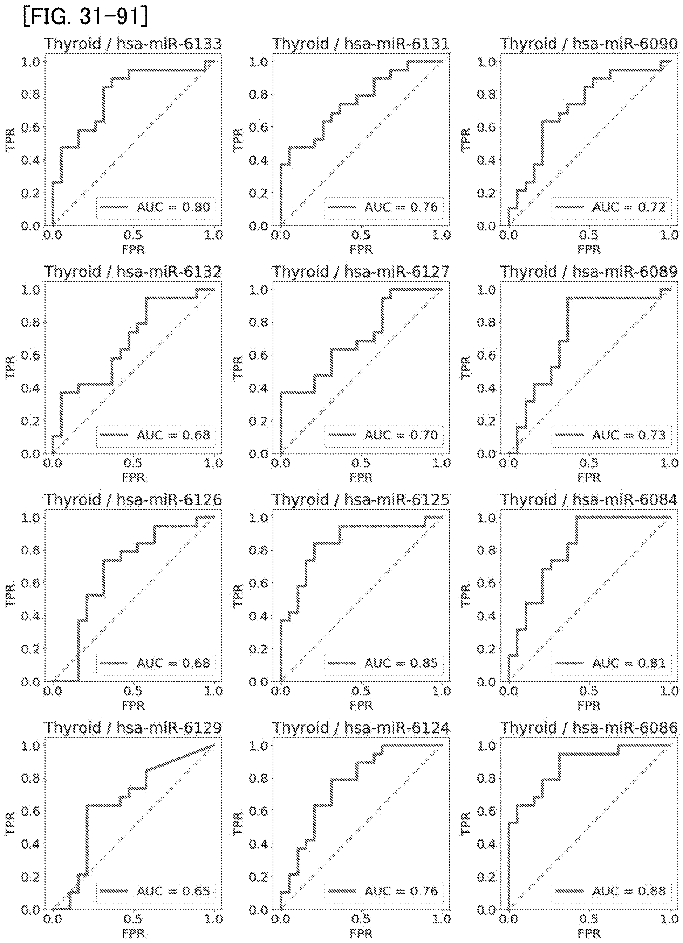

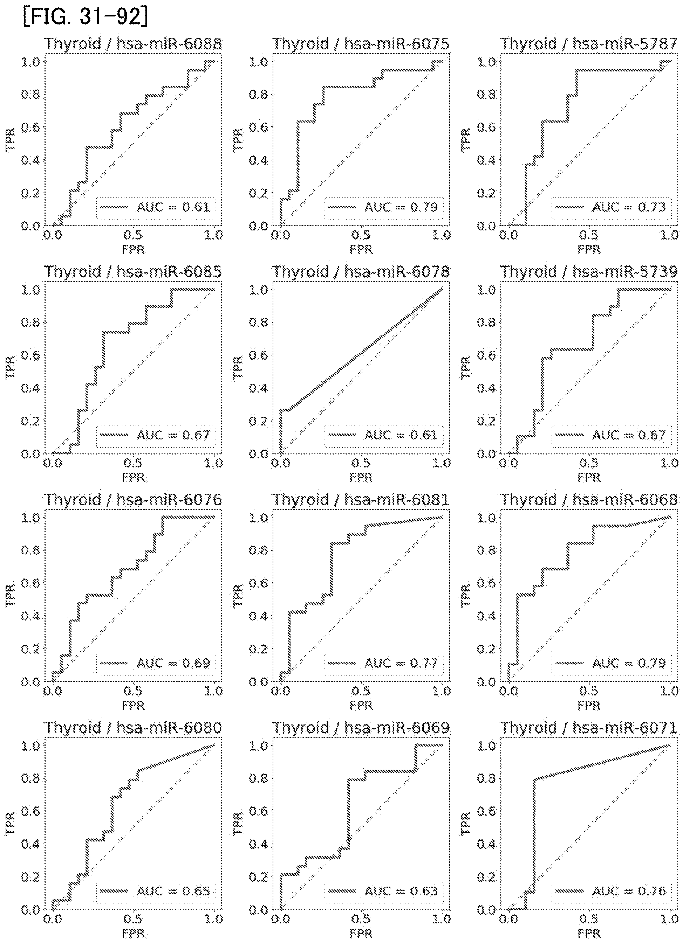

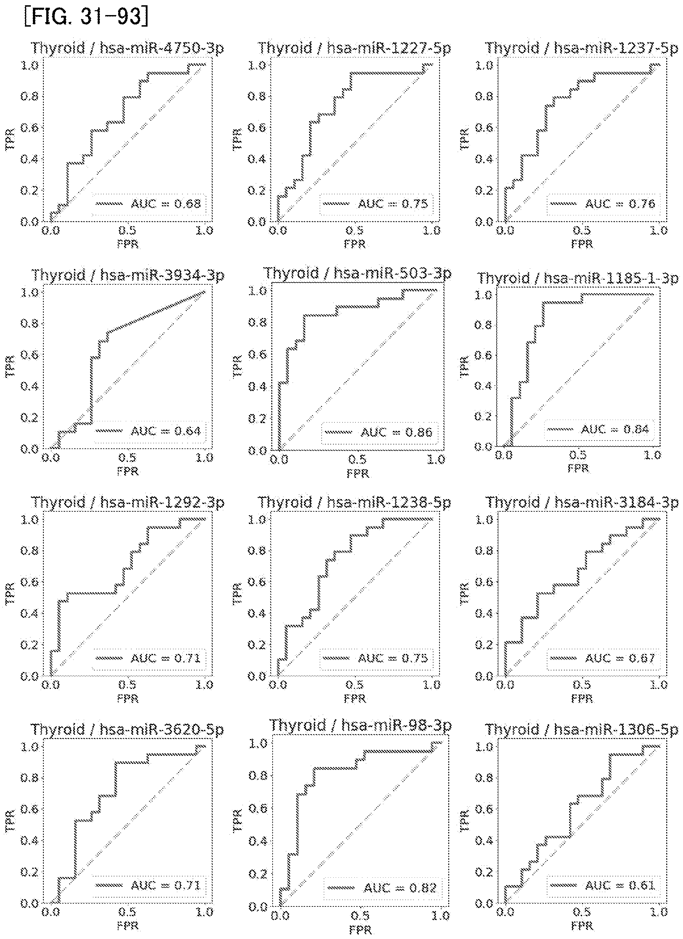

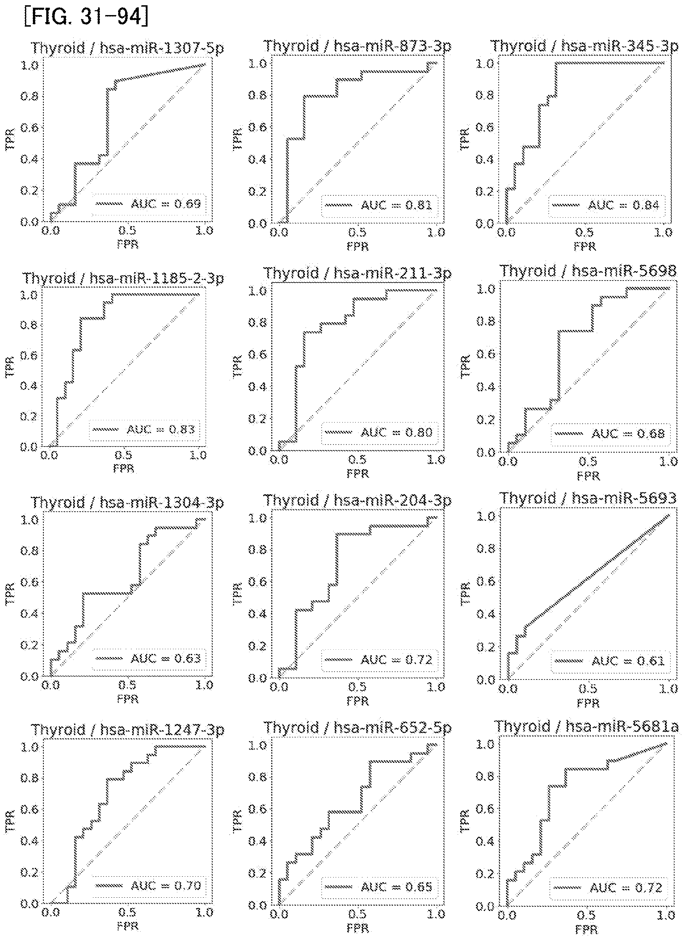

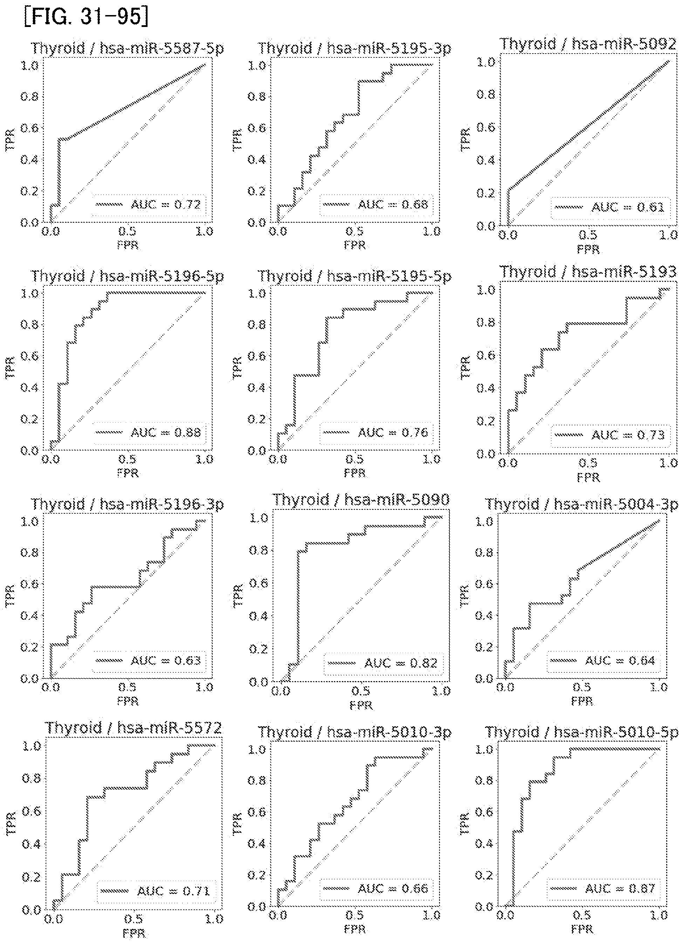

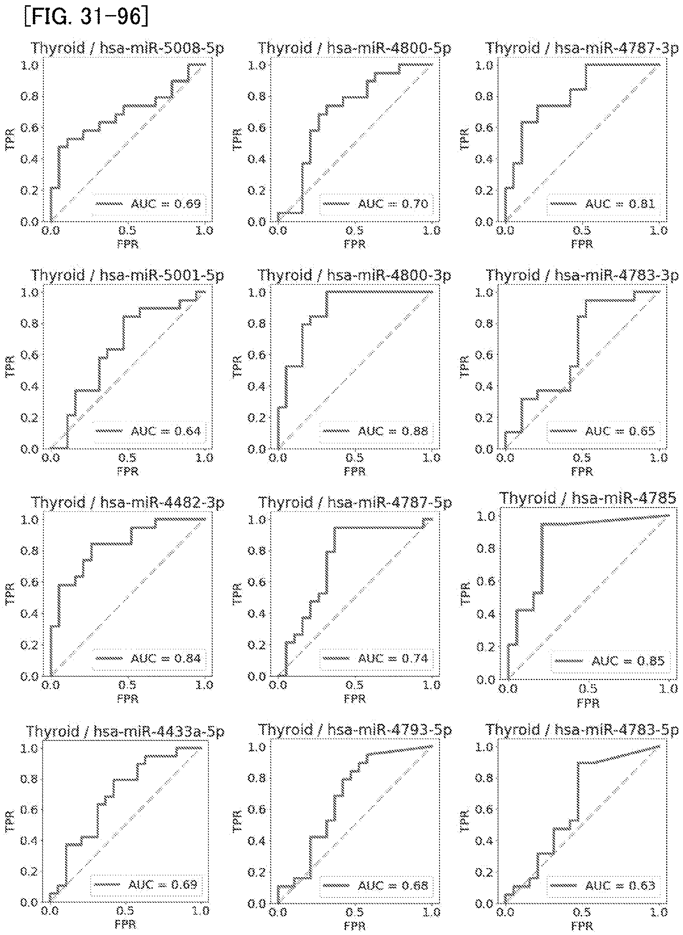

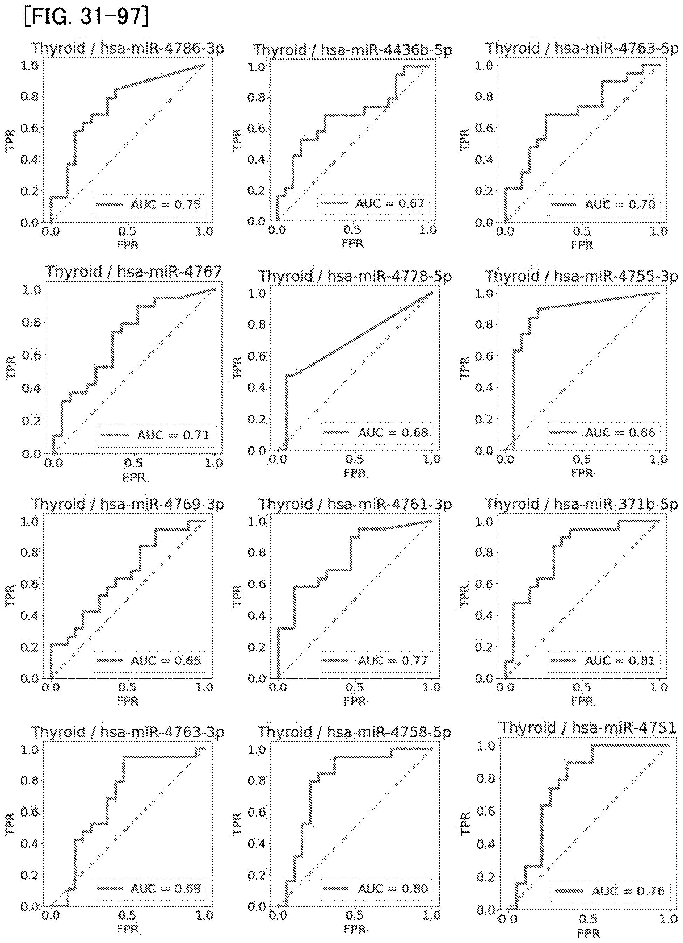

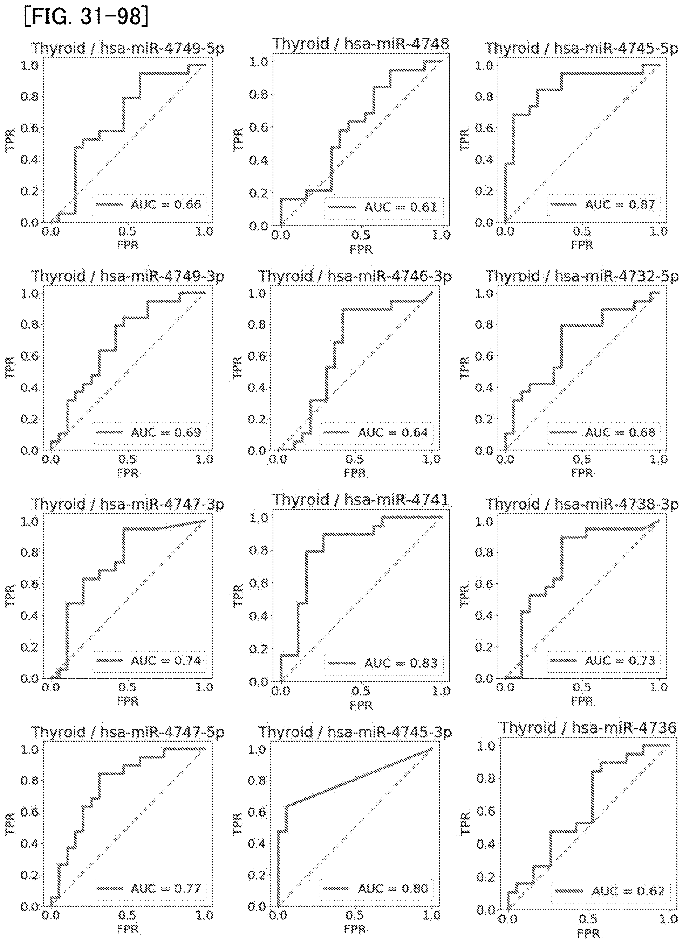

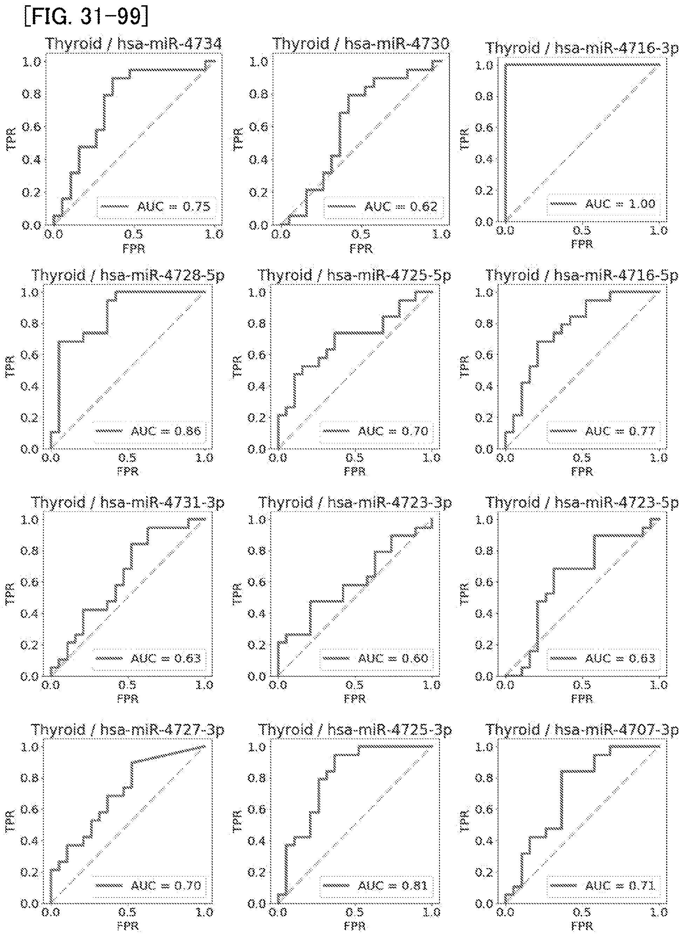

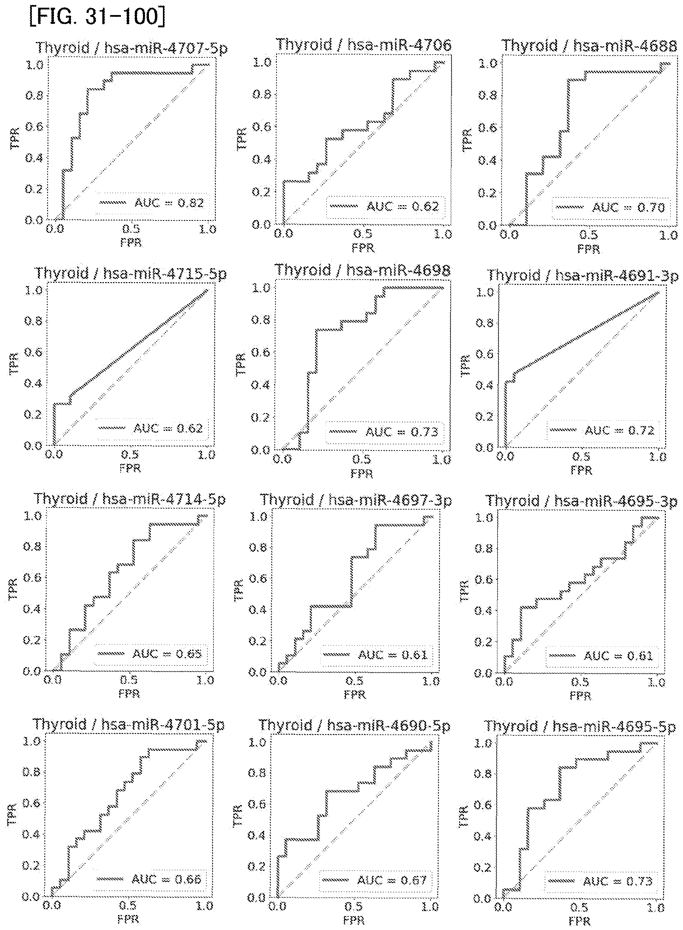

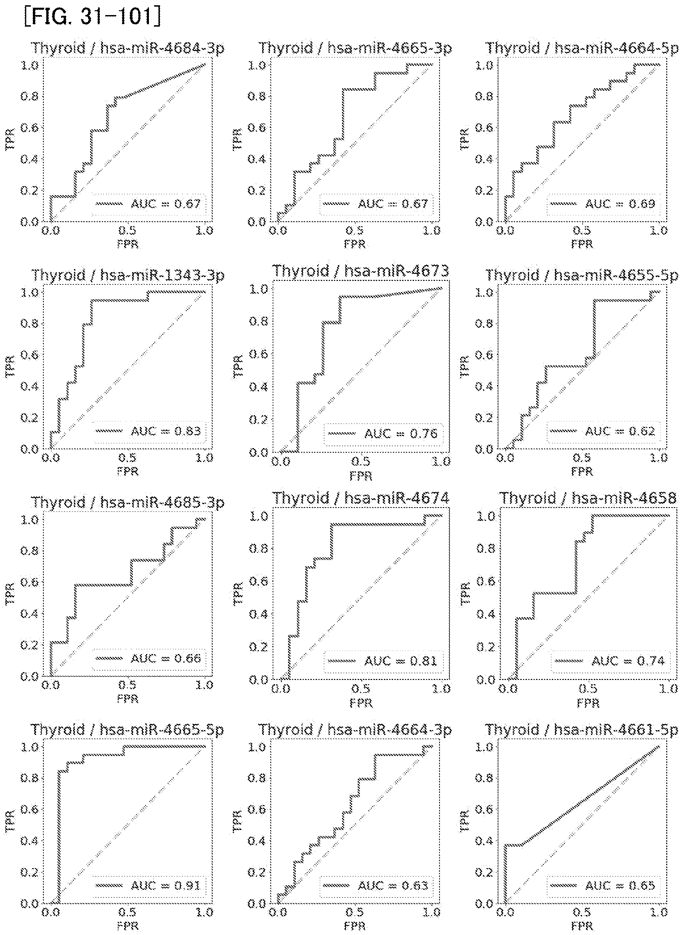

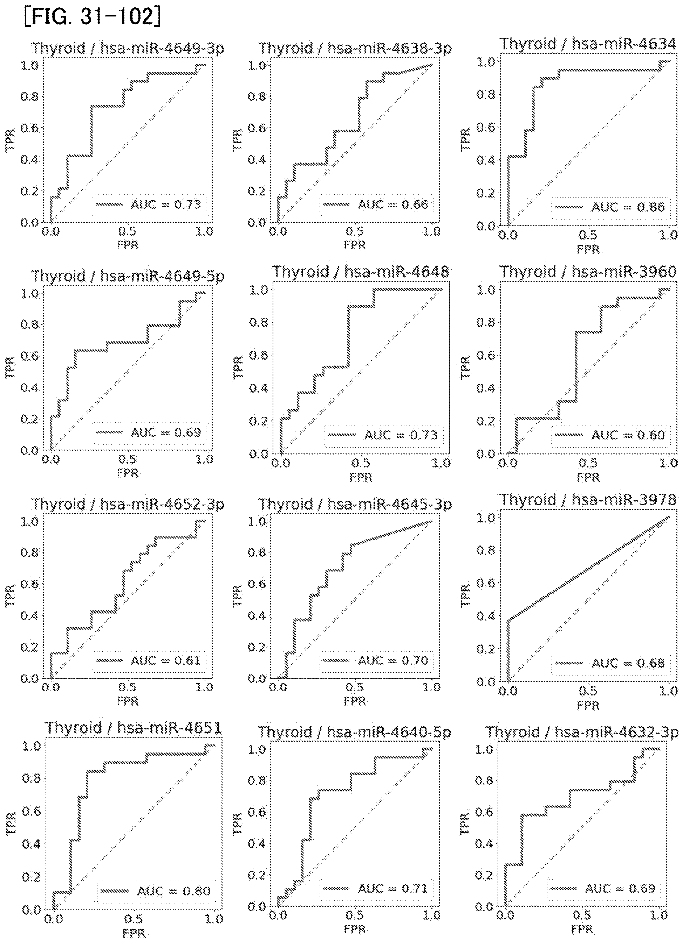

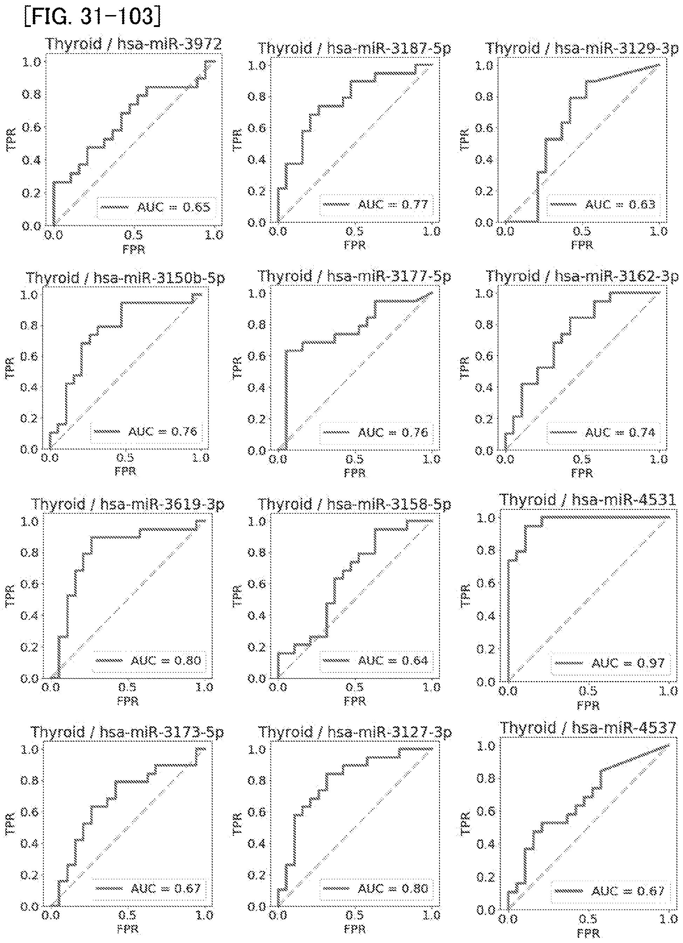

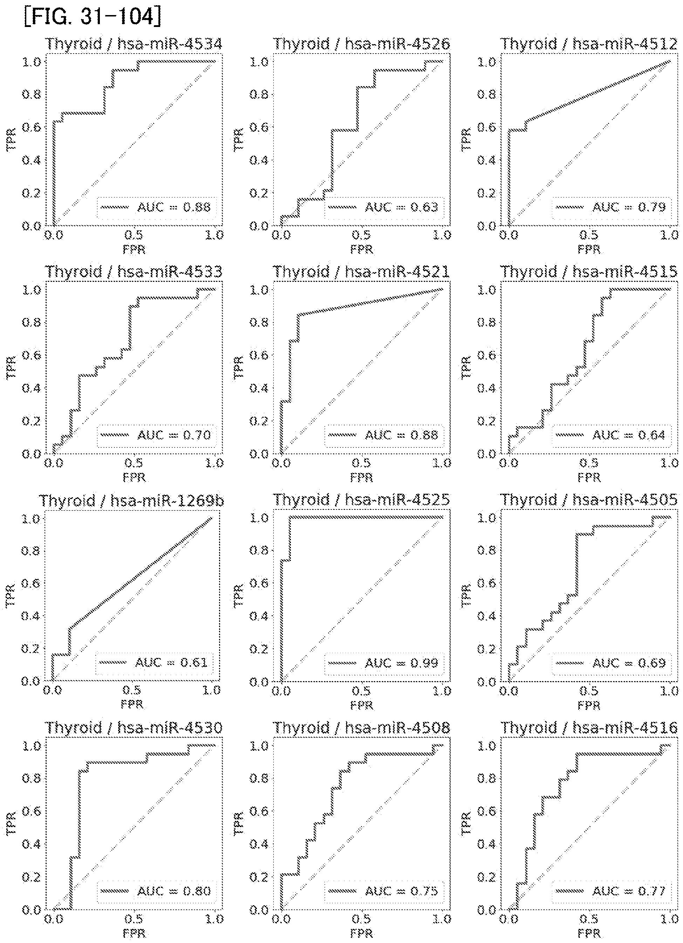

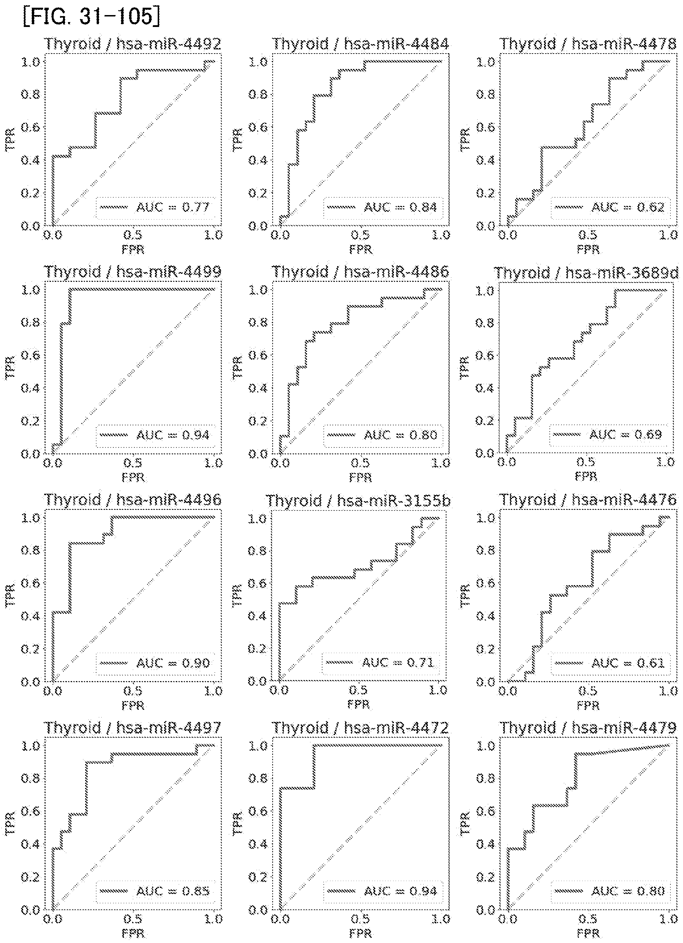

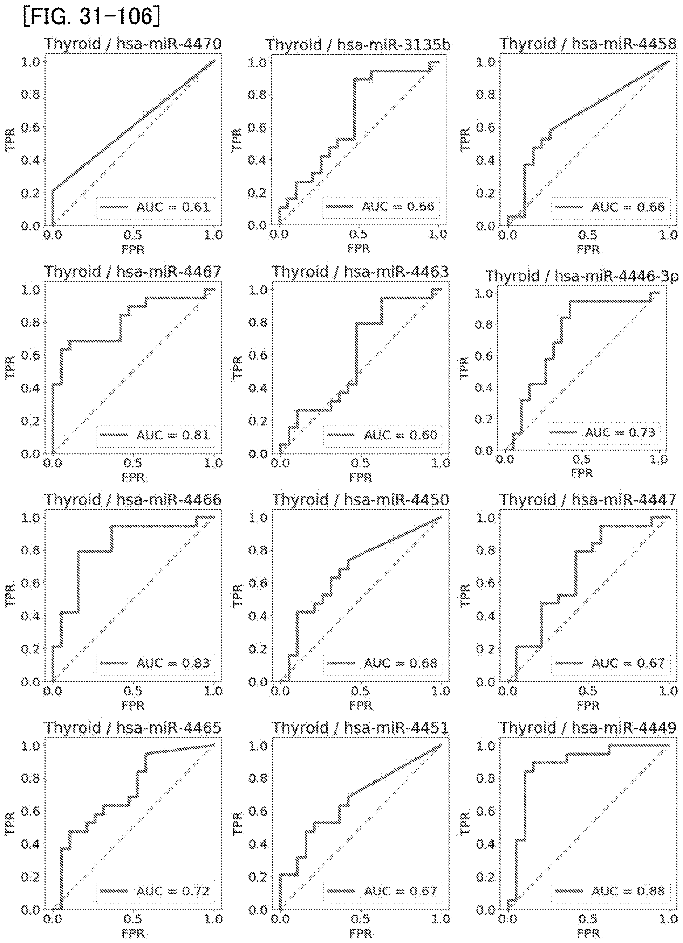

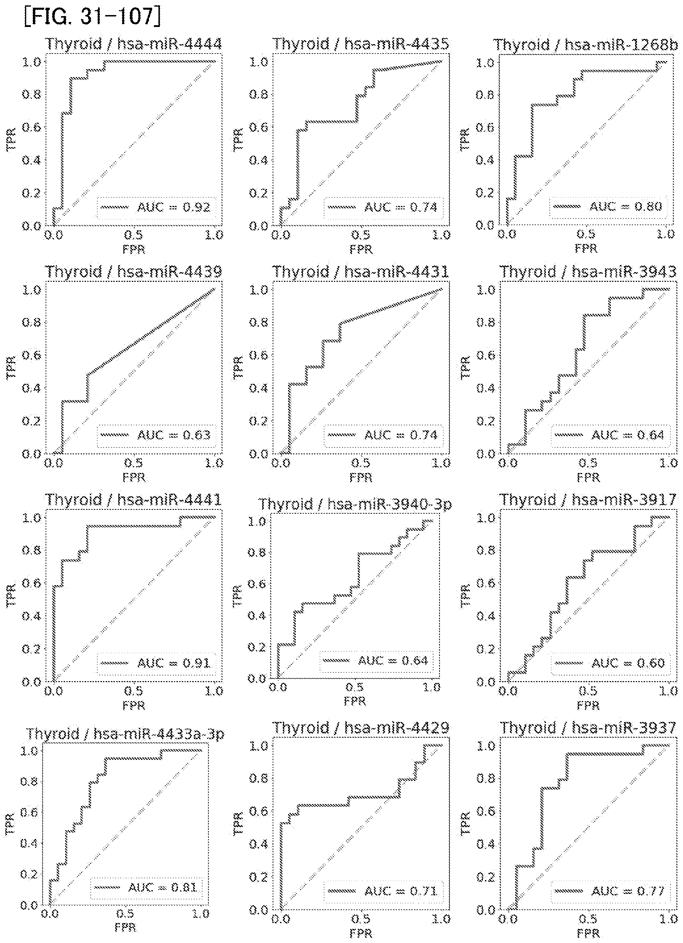

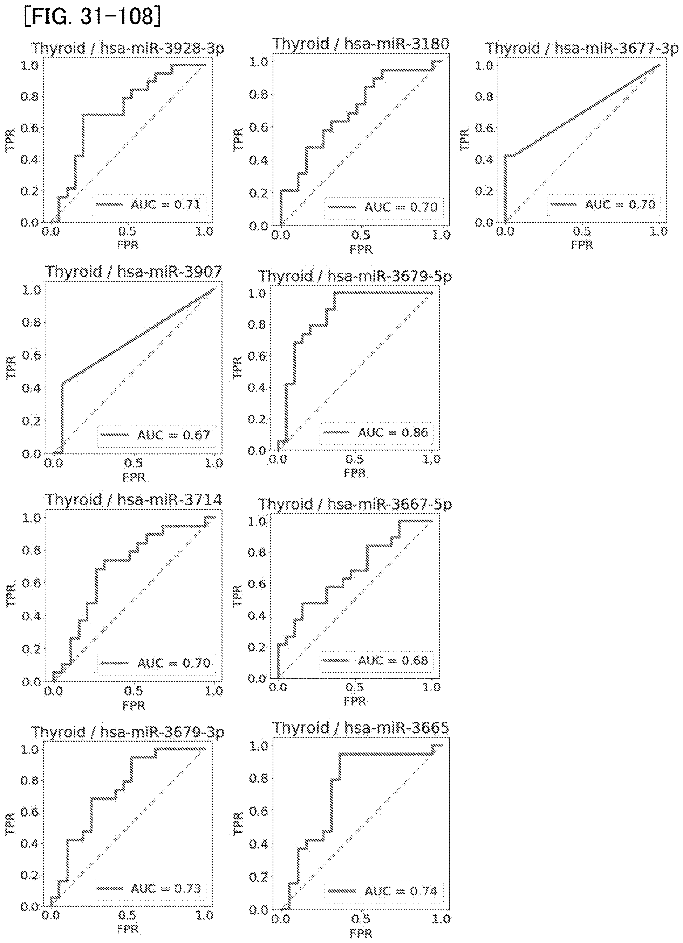

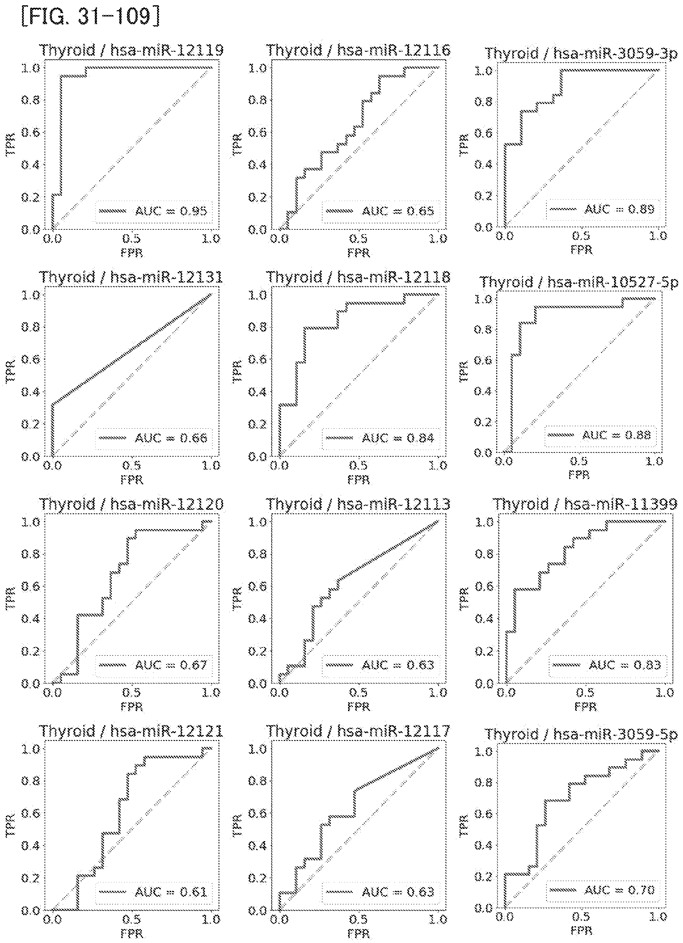

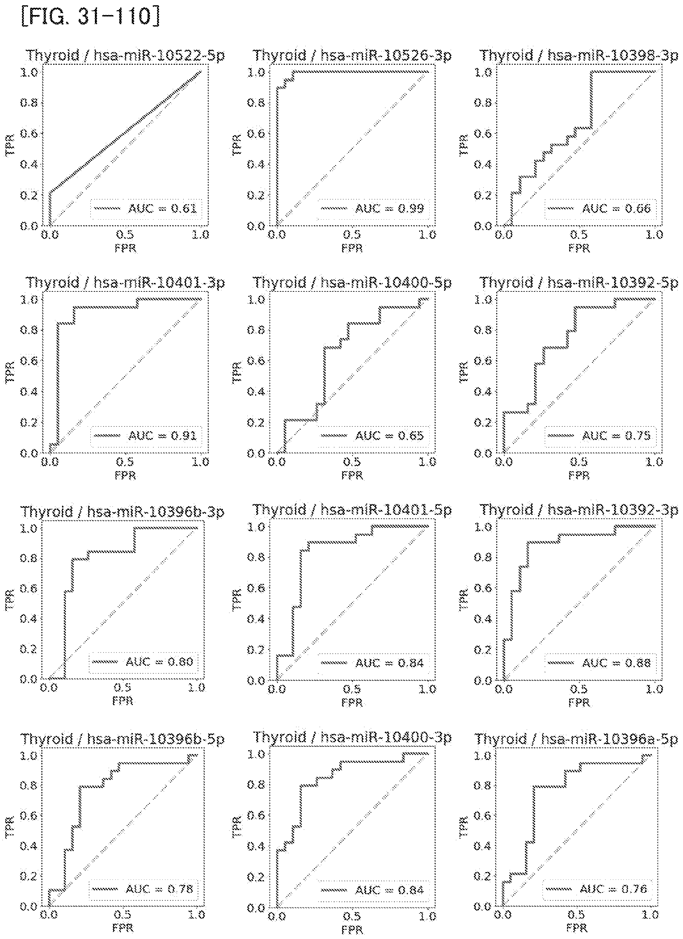

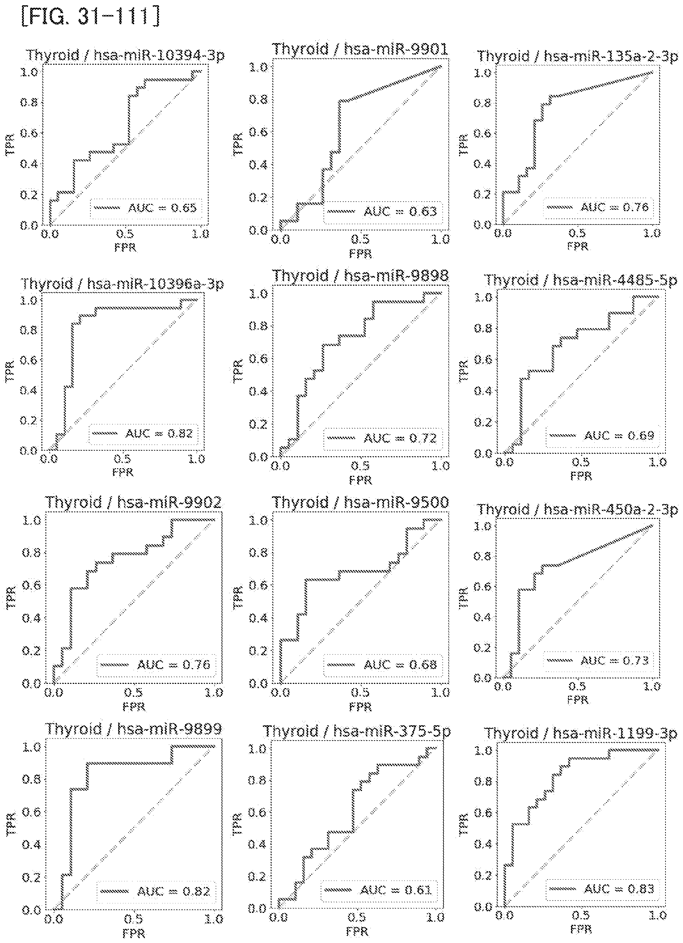

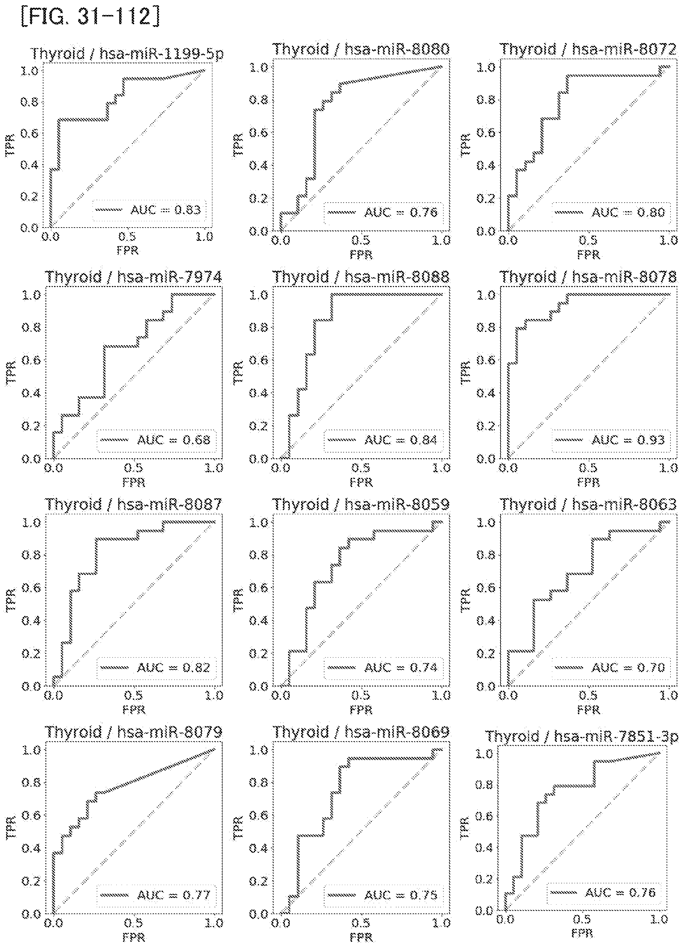

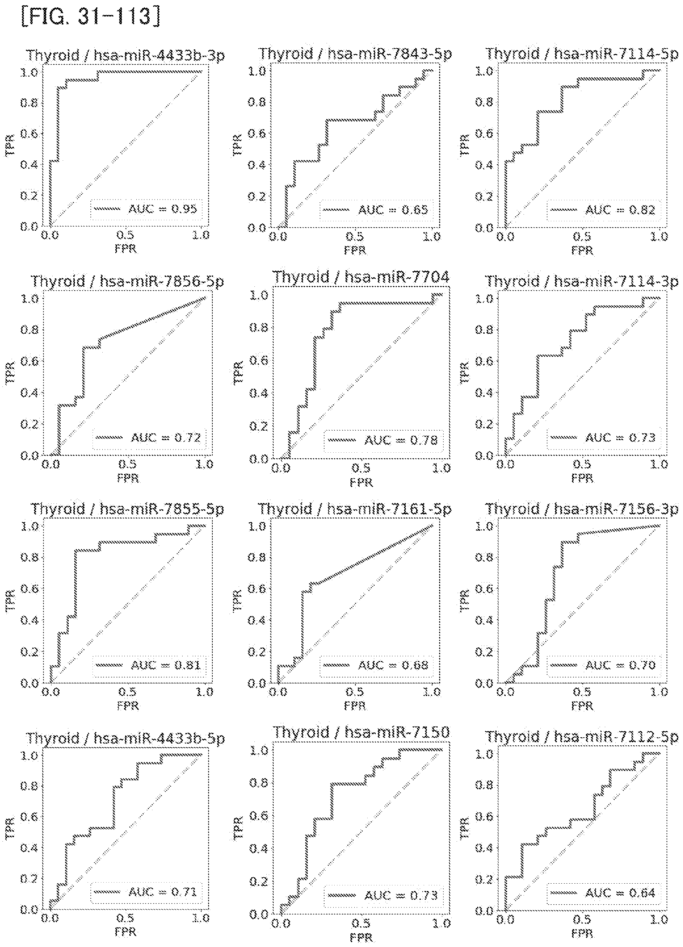

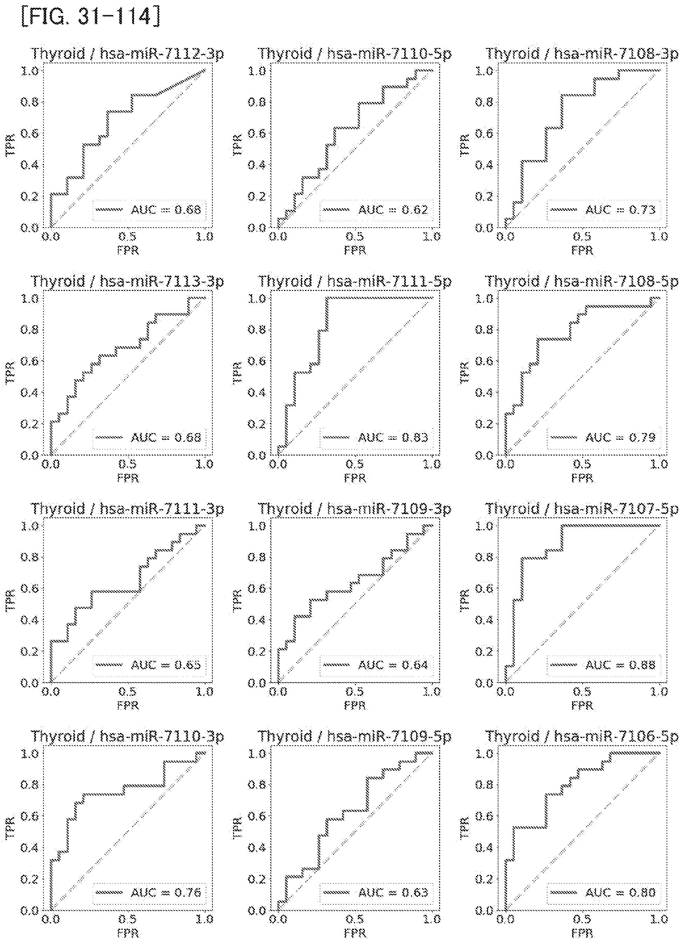

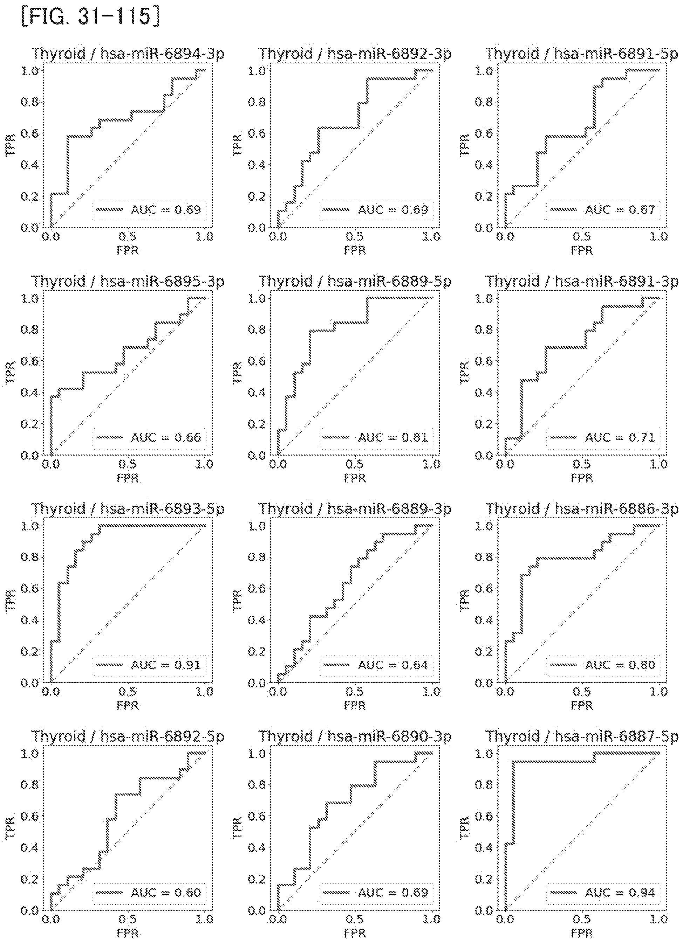

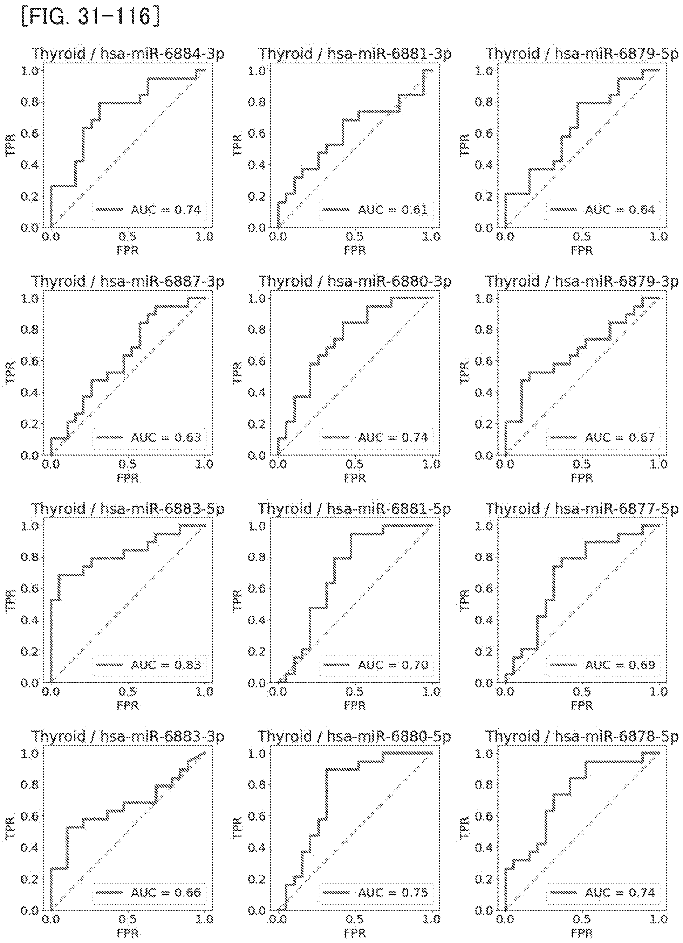

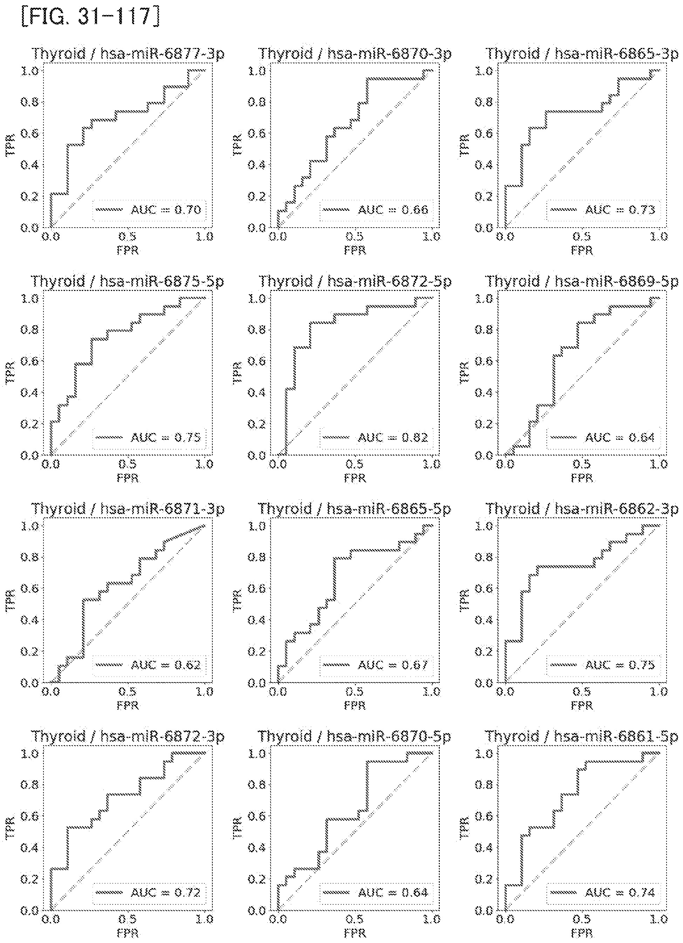

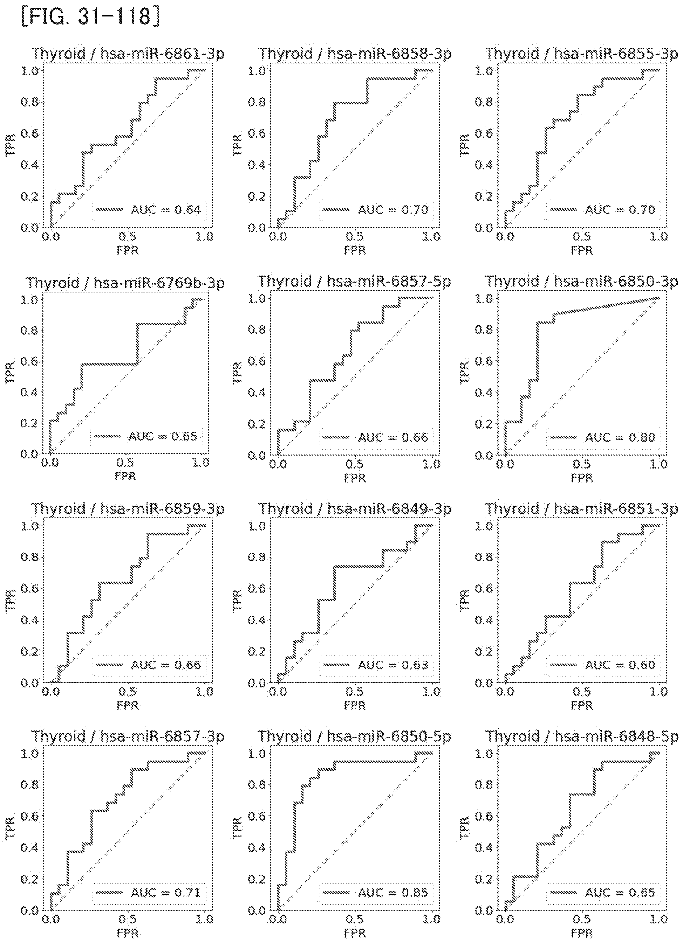

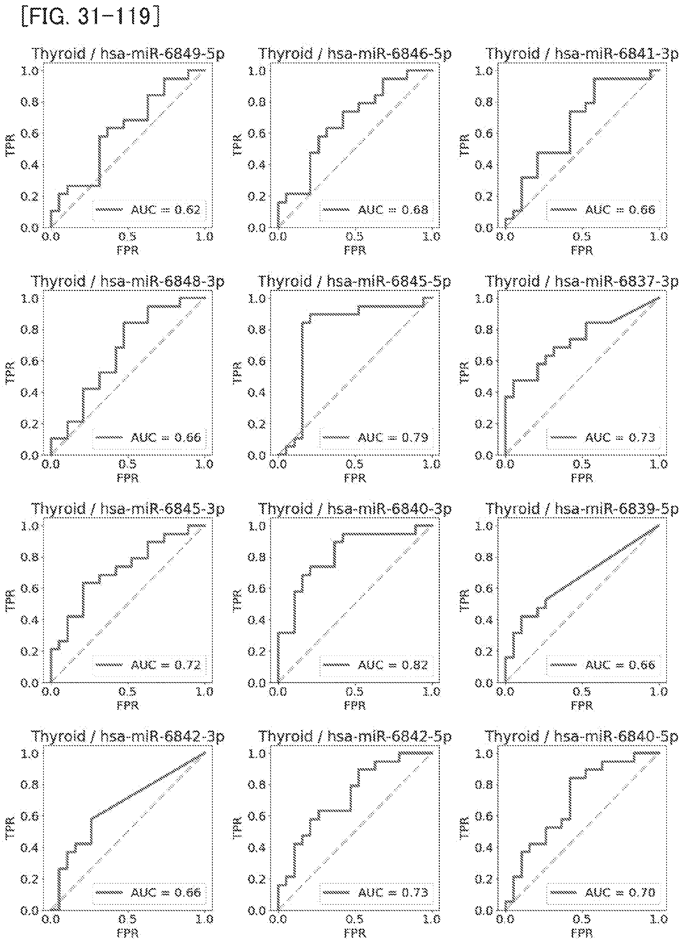

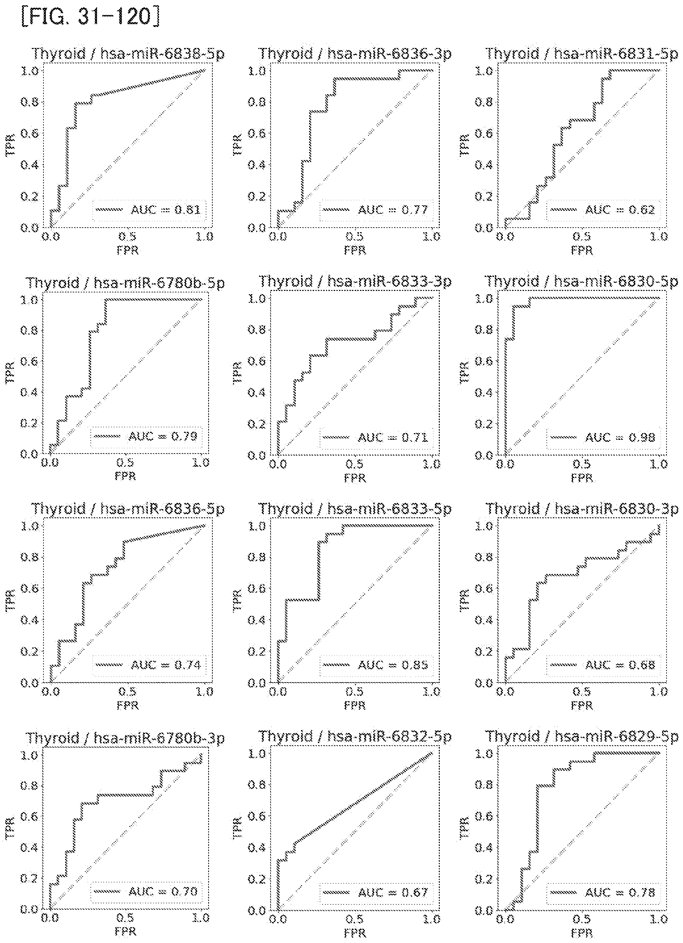

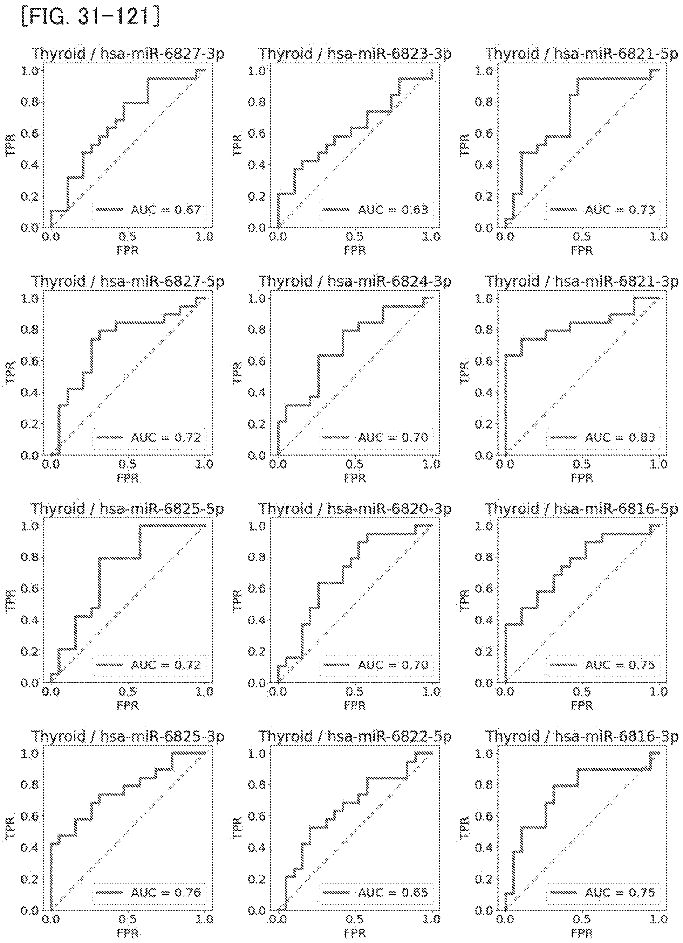

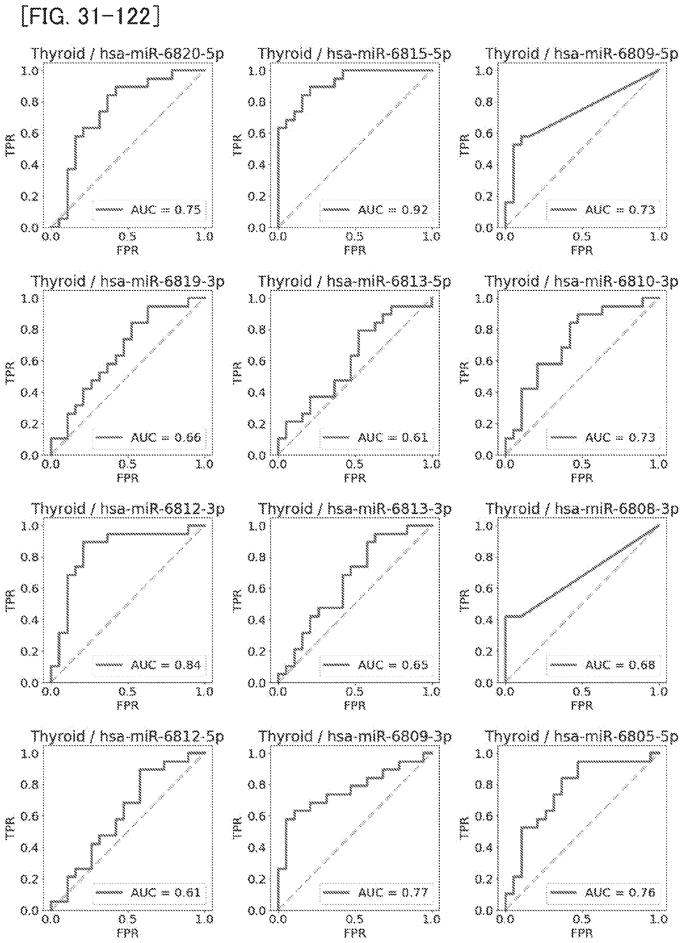

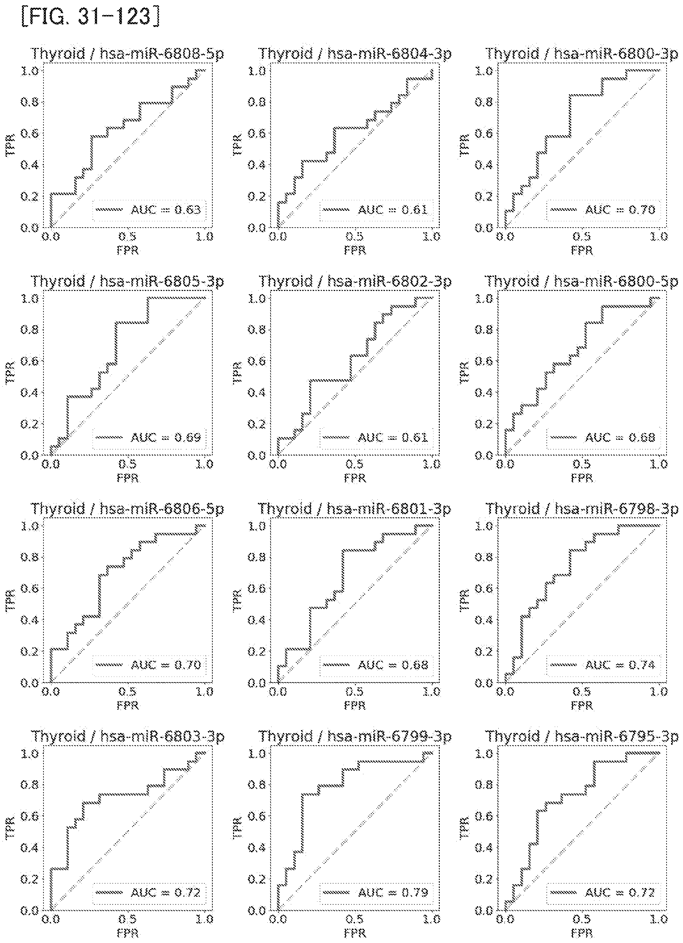

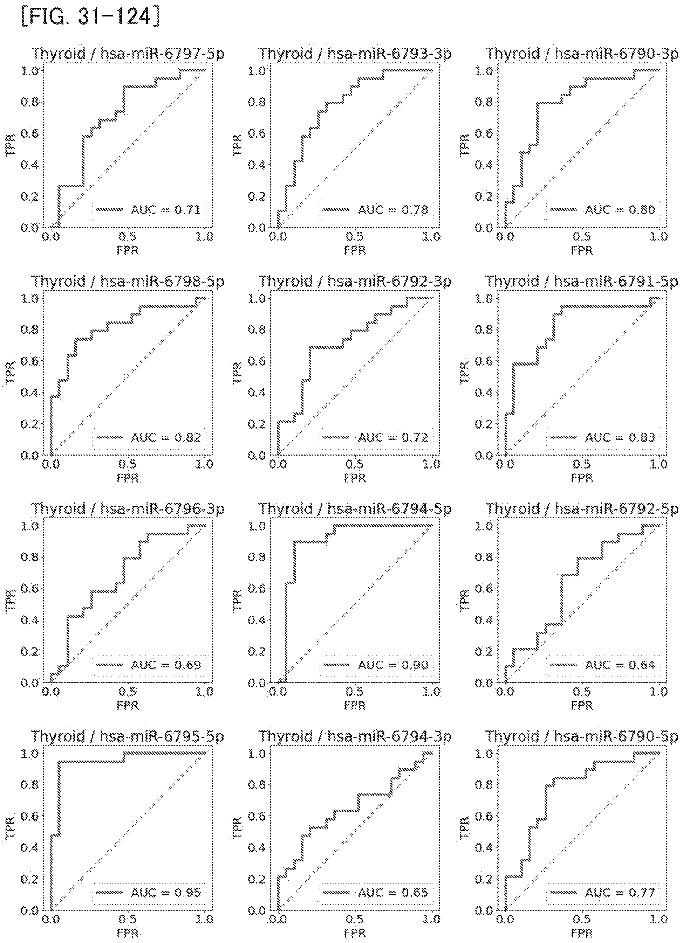

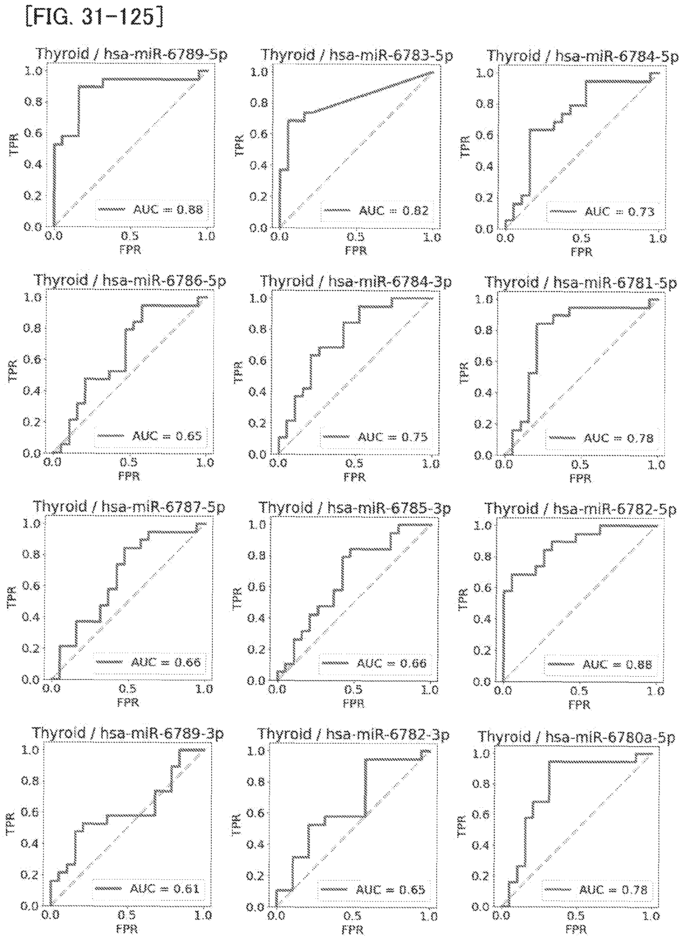

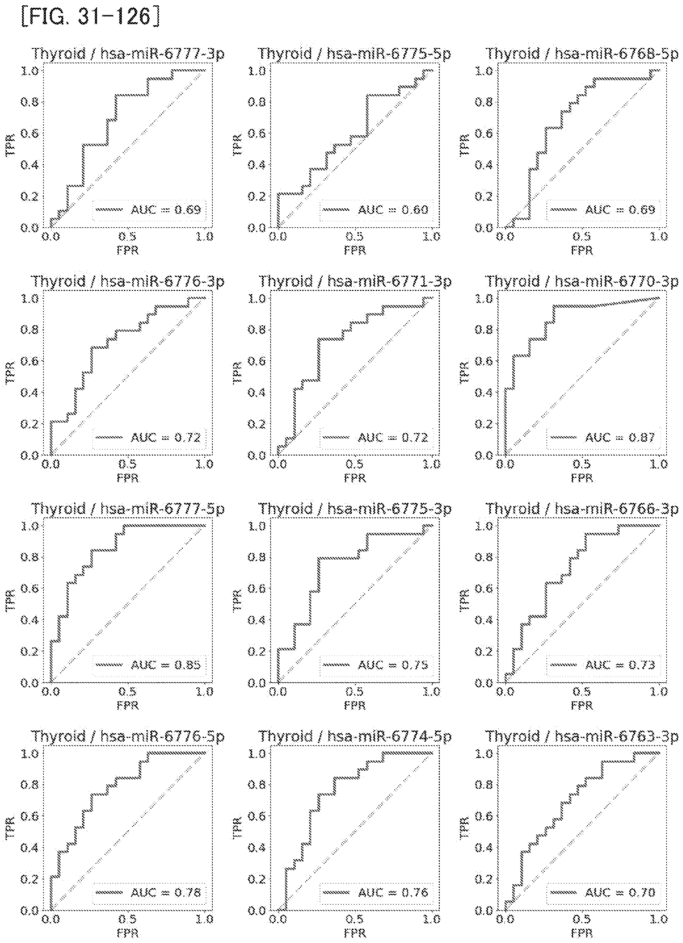

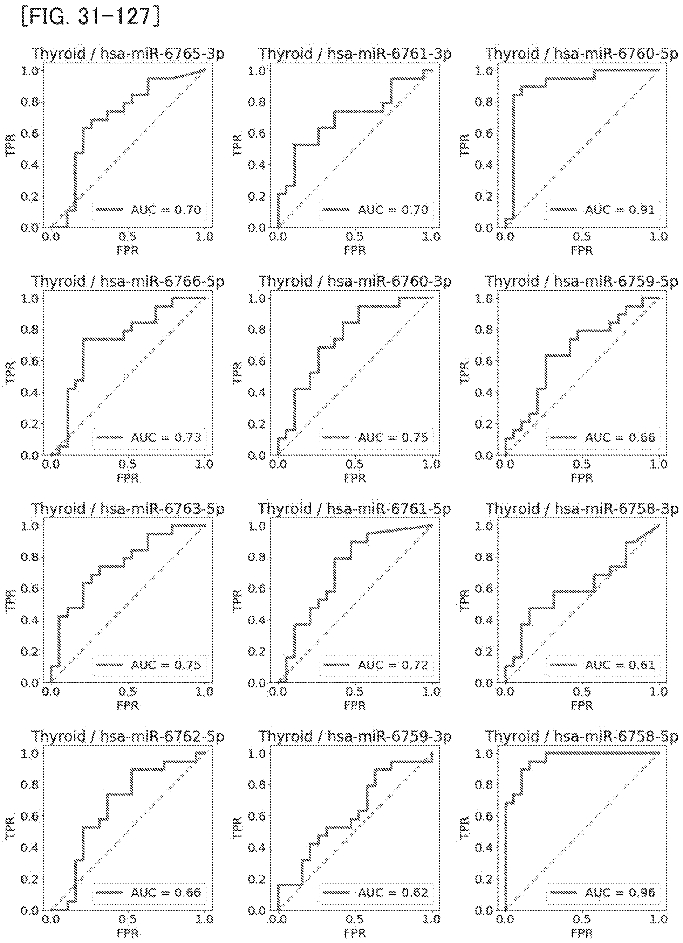

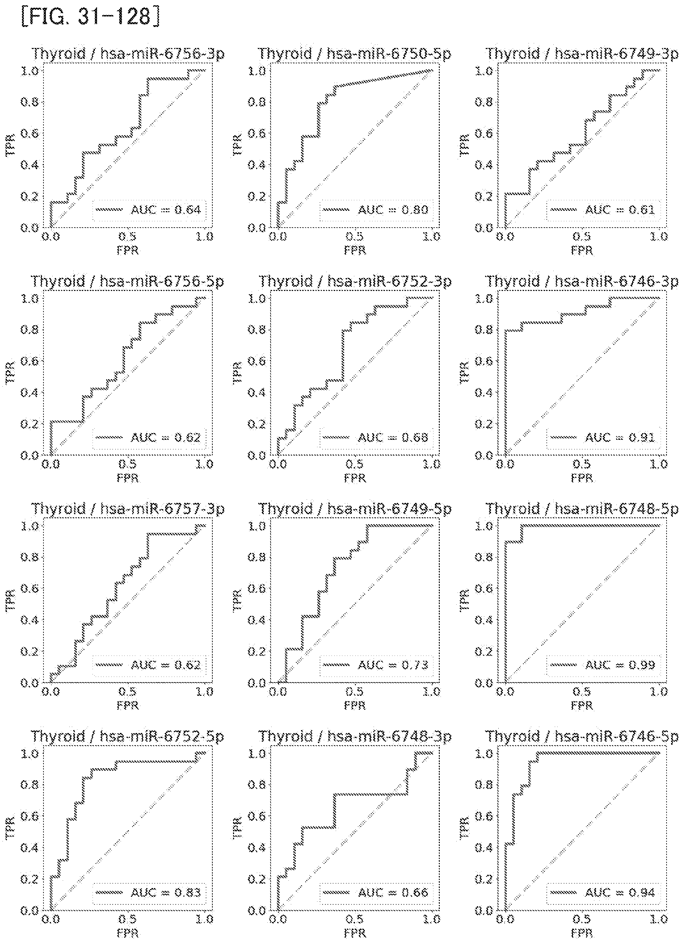

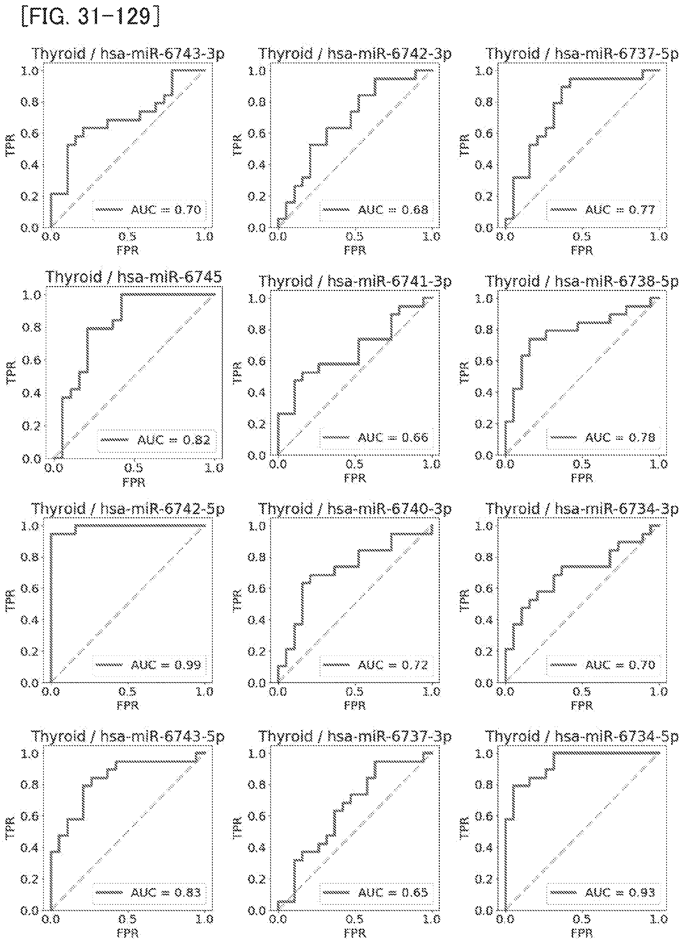

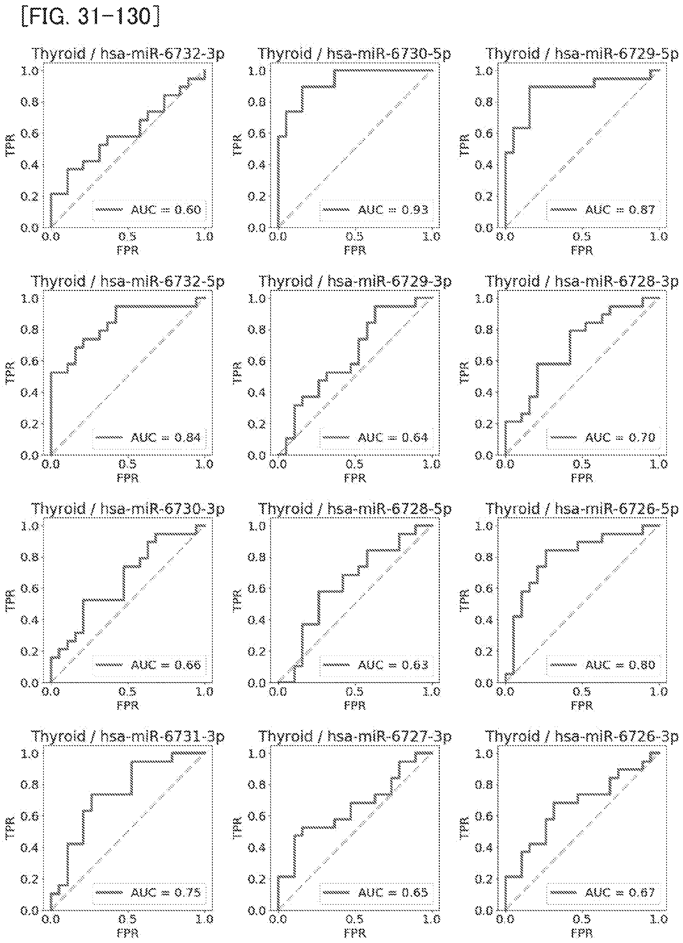

FIGS. 31-1 to 31-130 show ROCs curves for predicting thyroid cancer in humans using a single miRNA.

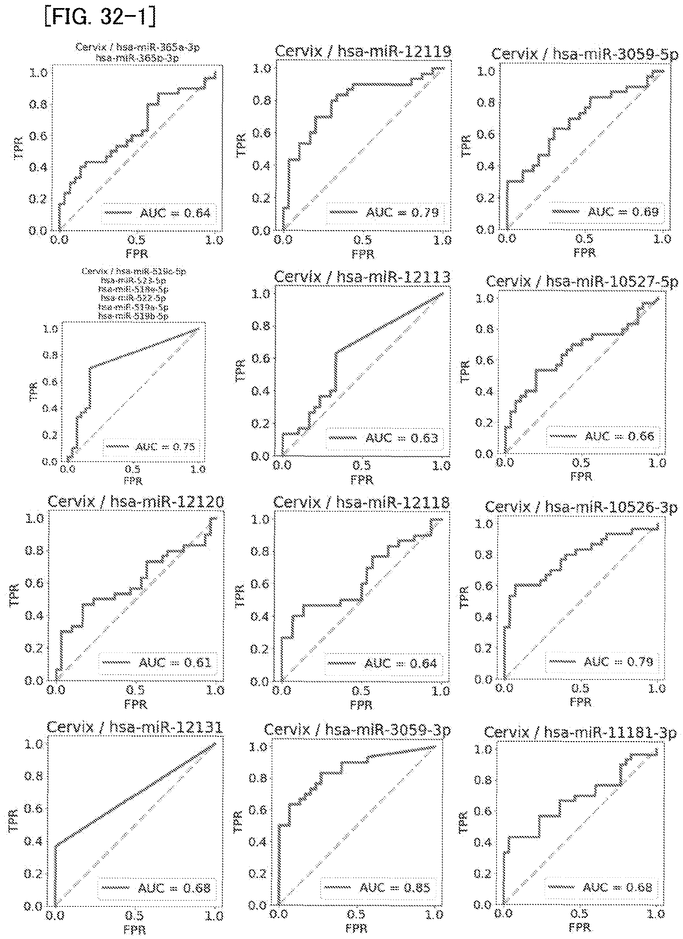

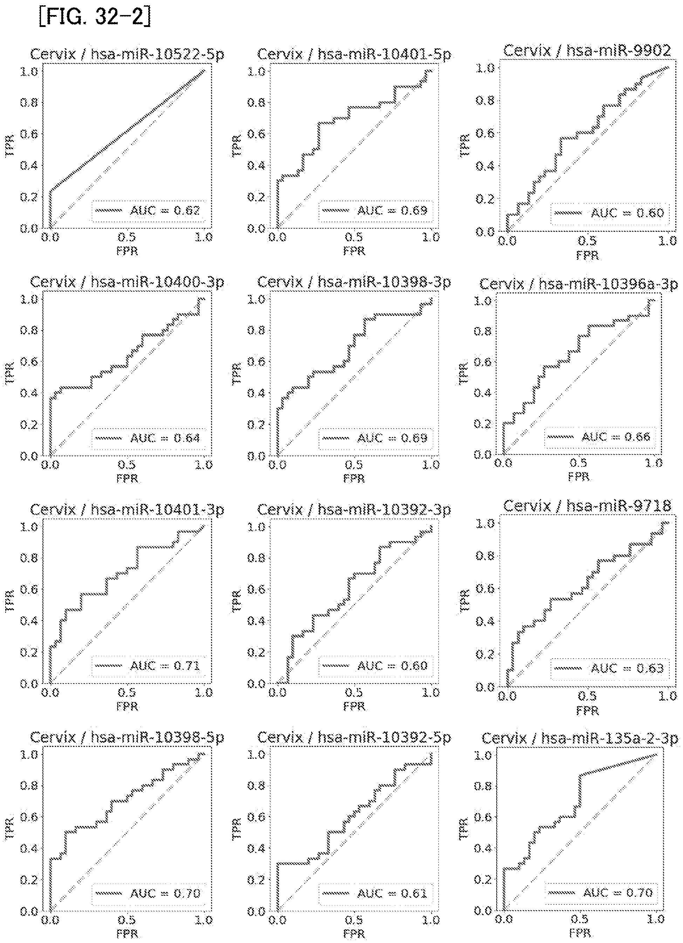

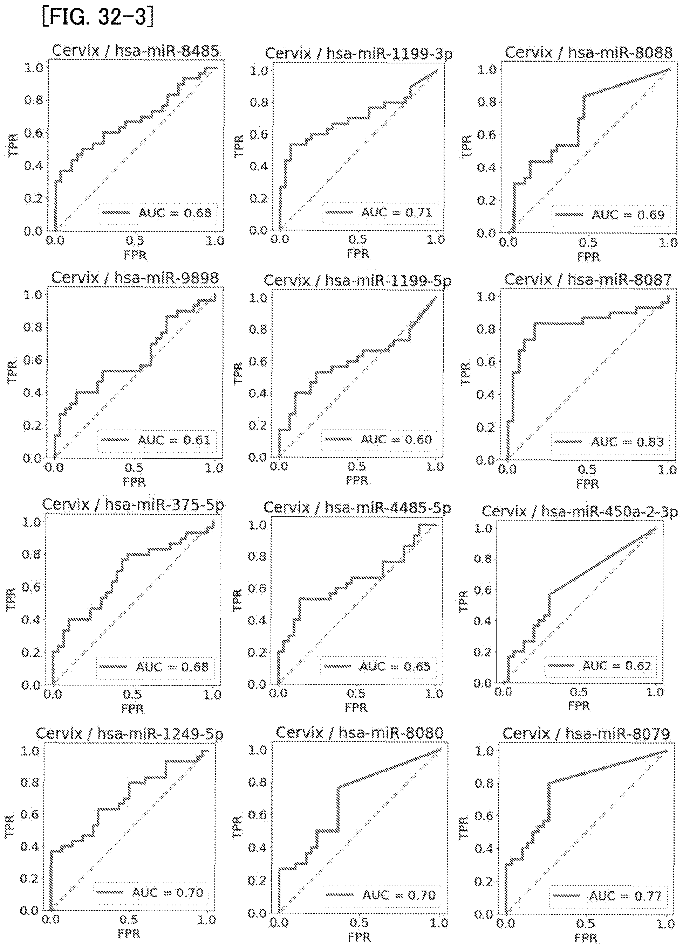

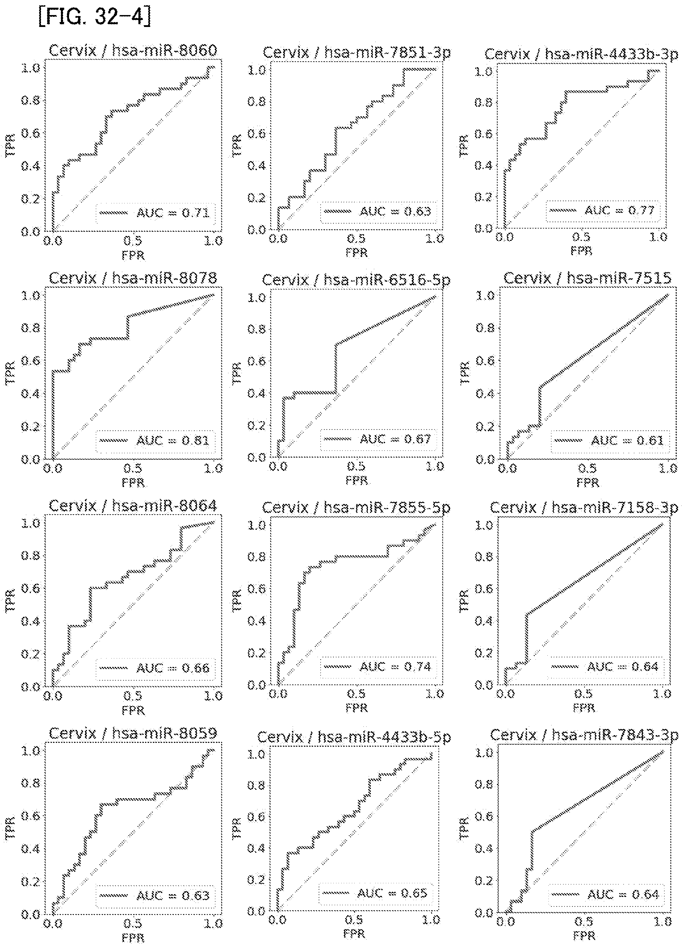

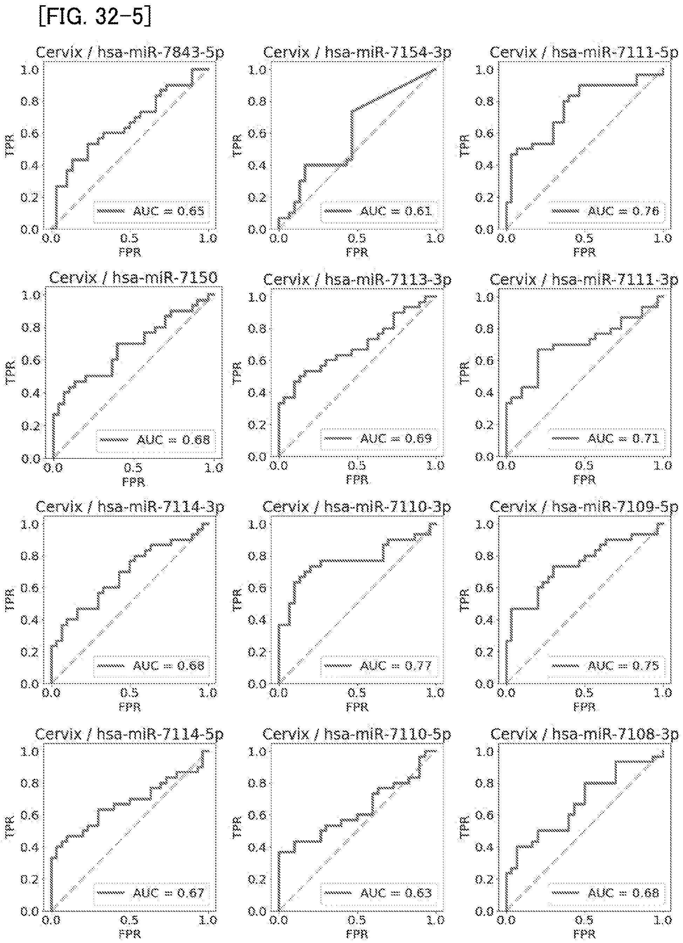

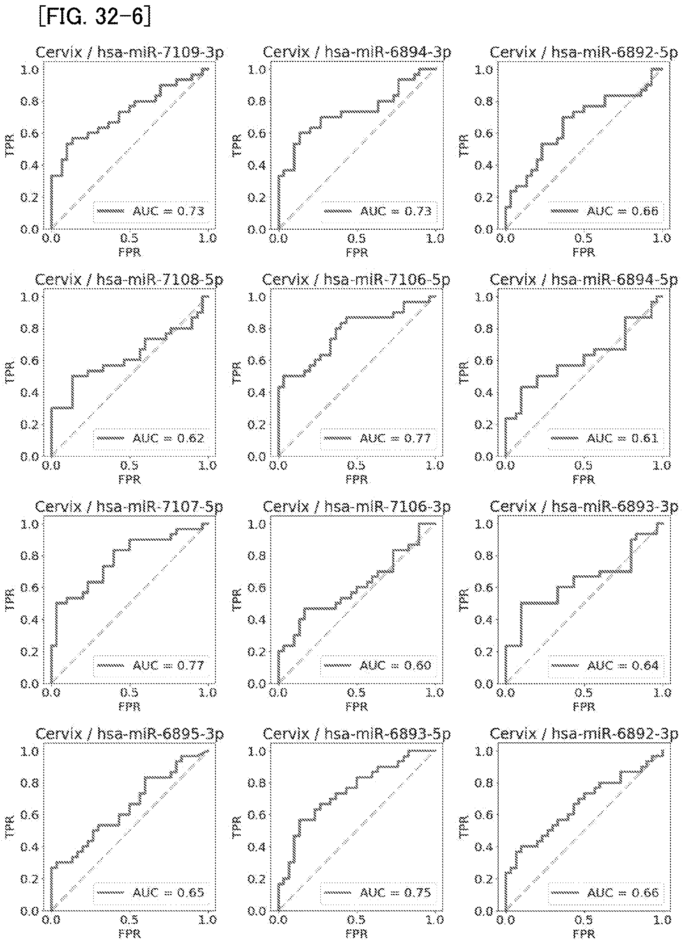

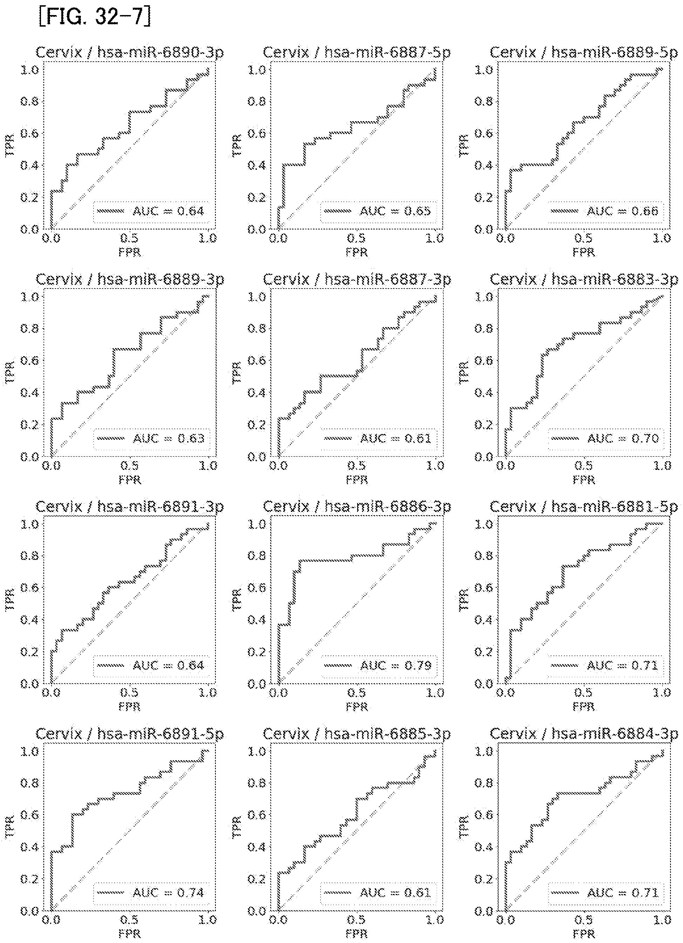

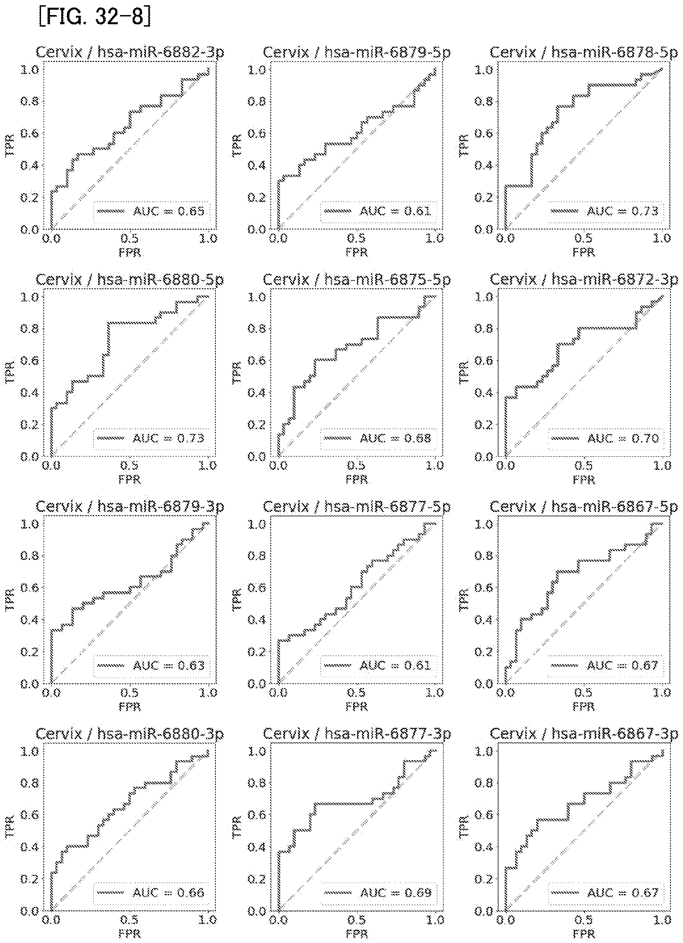

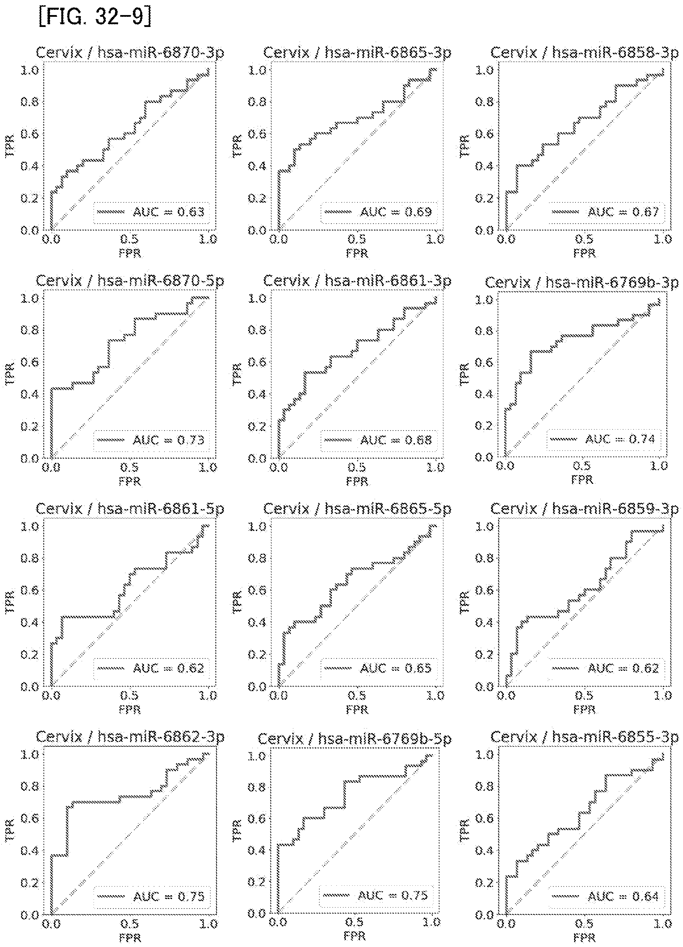

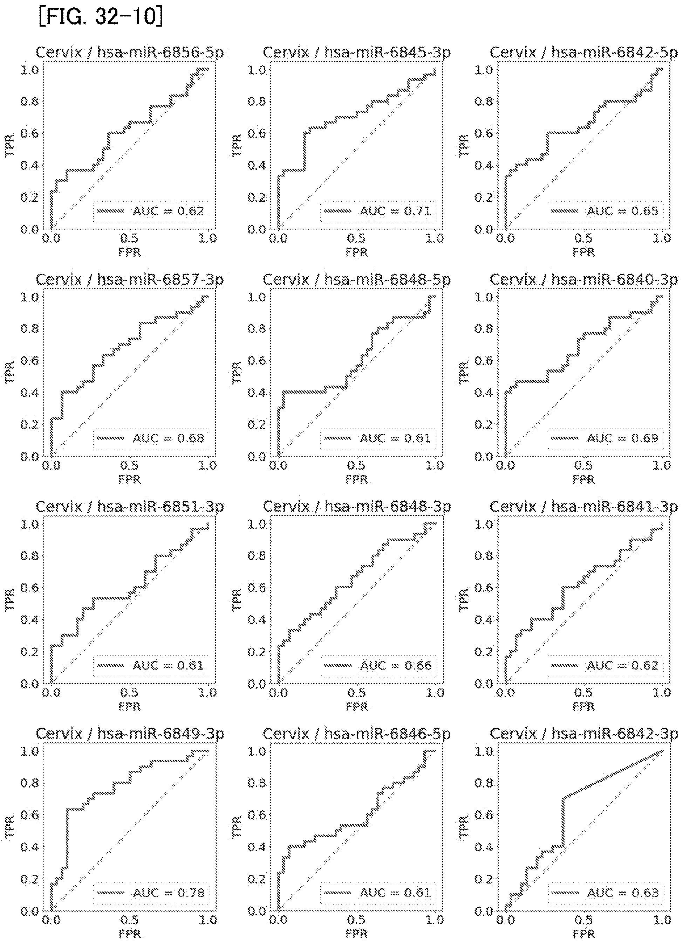

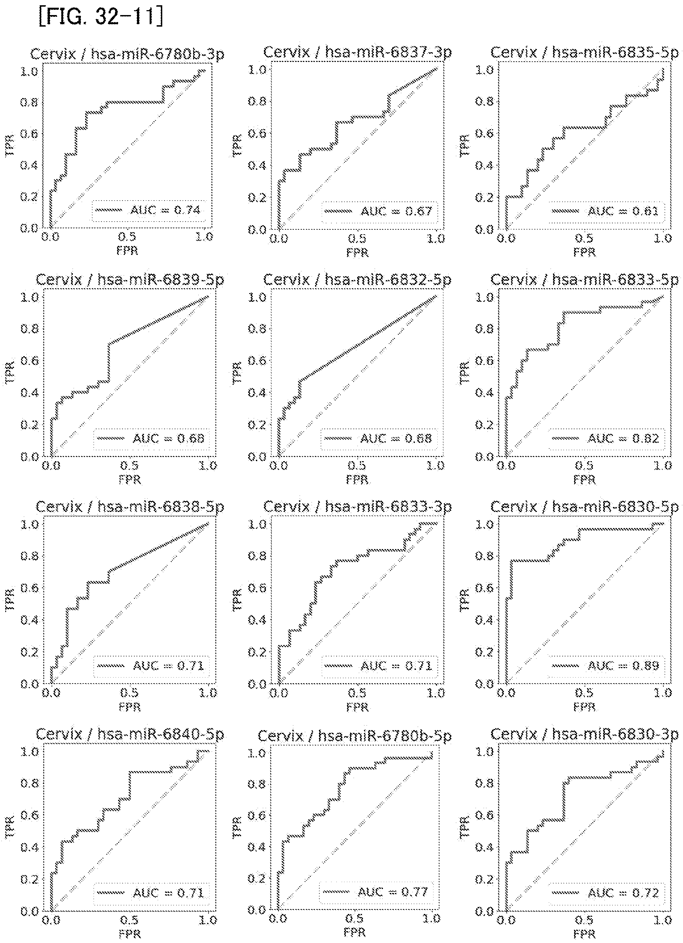

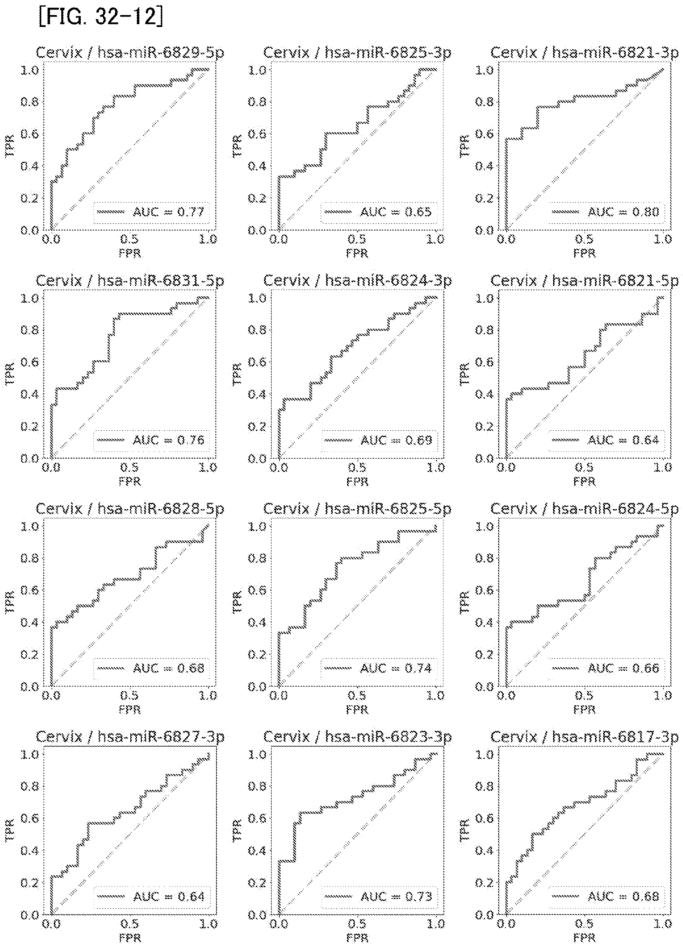

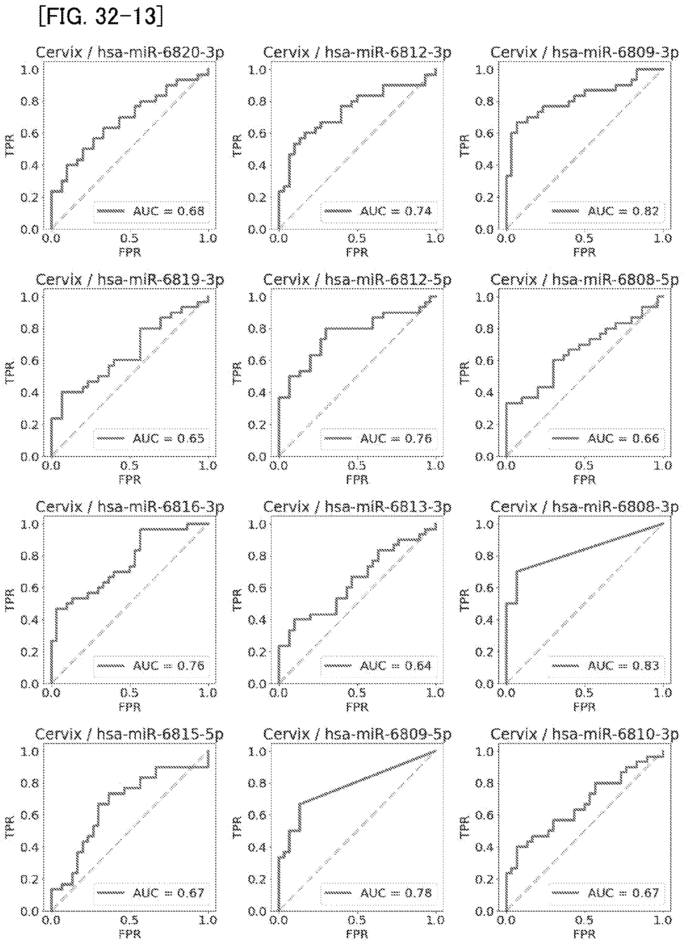

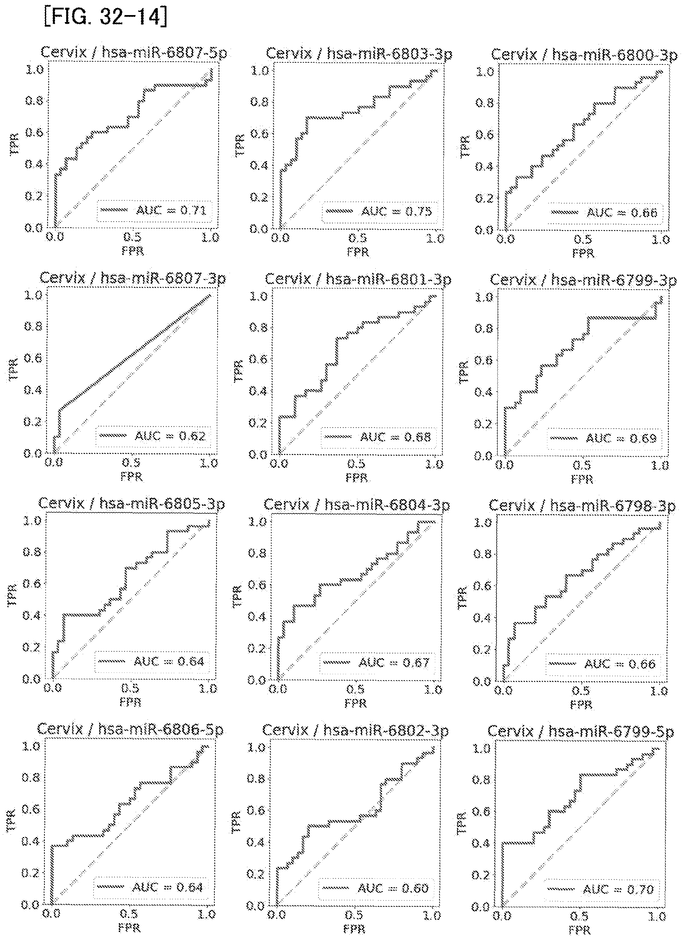

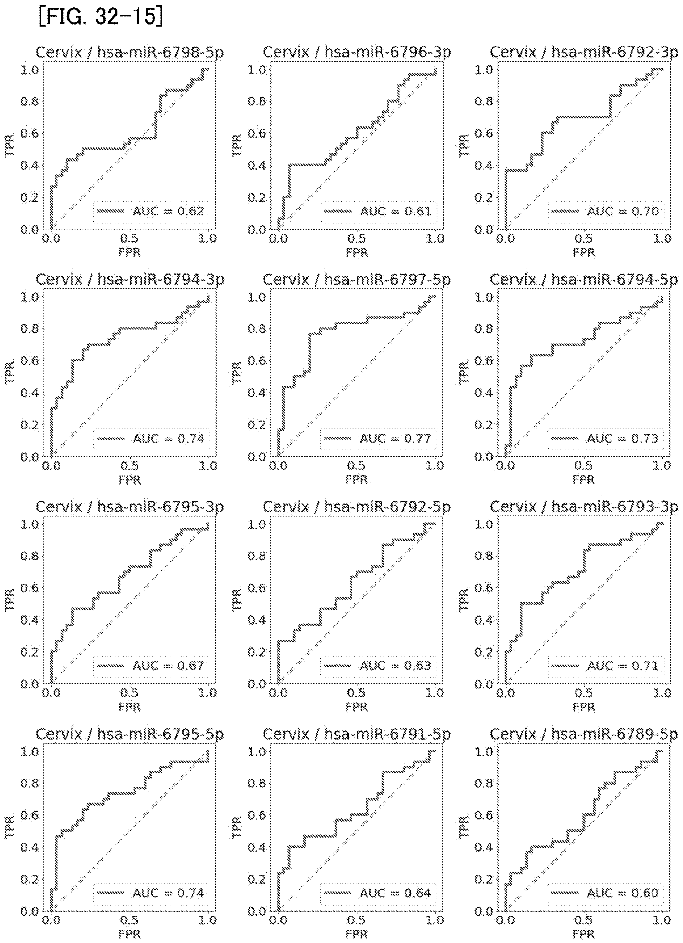

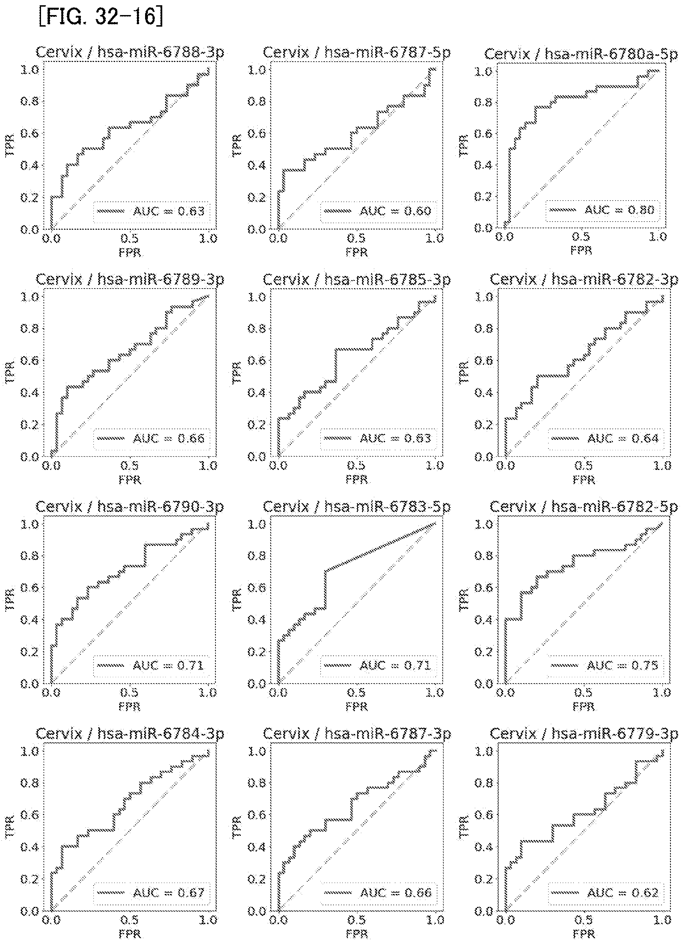

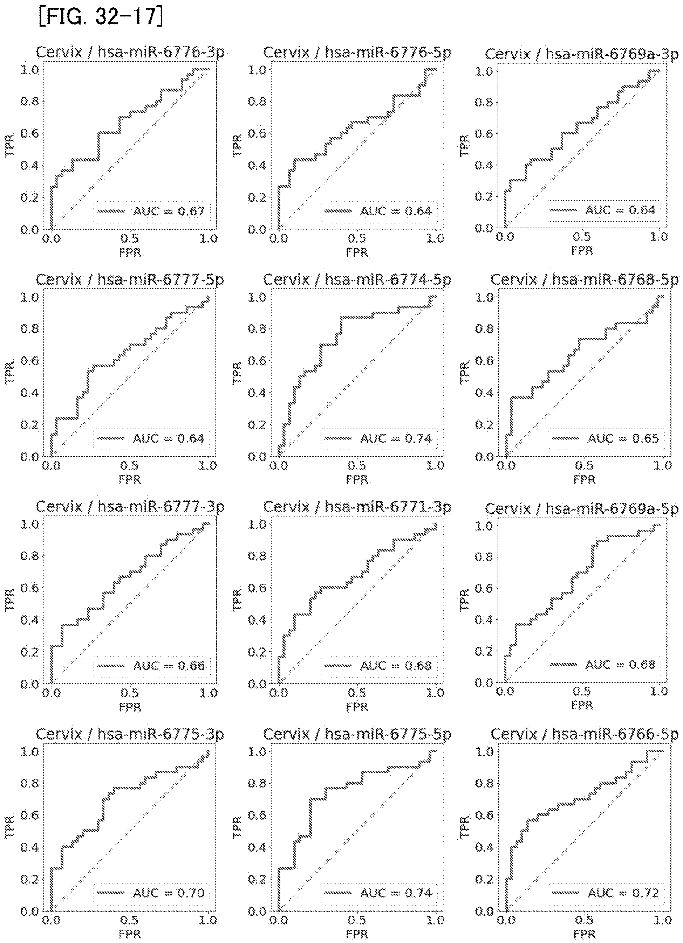

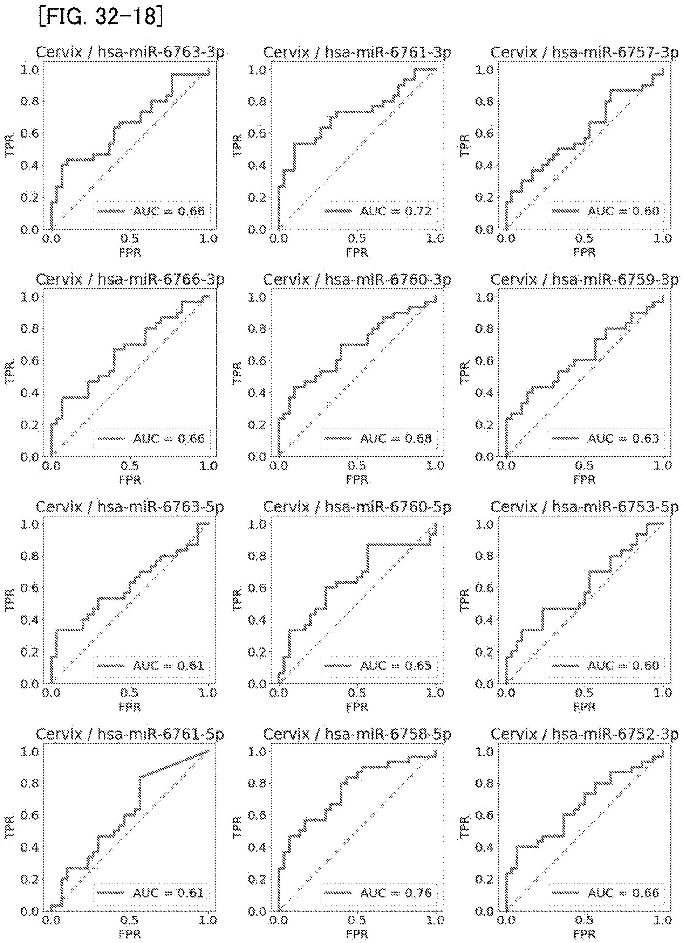

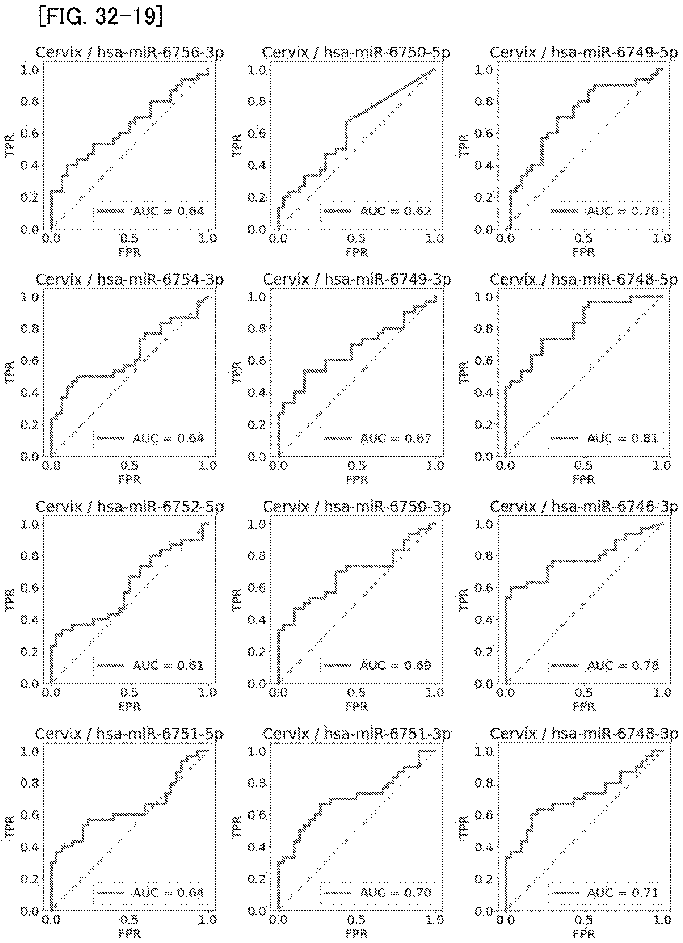

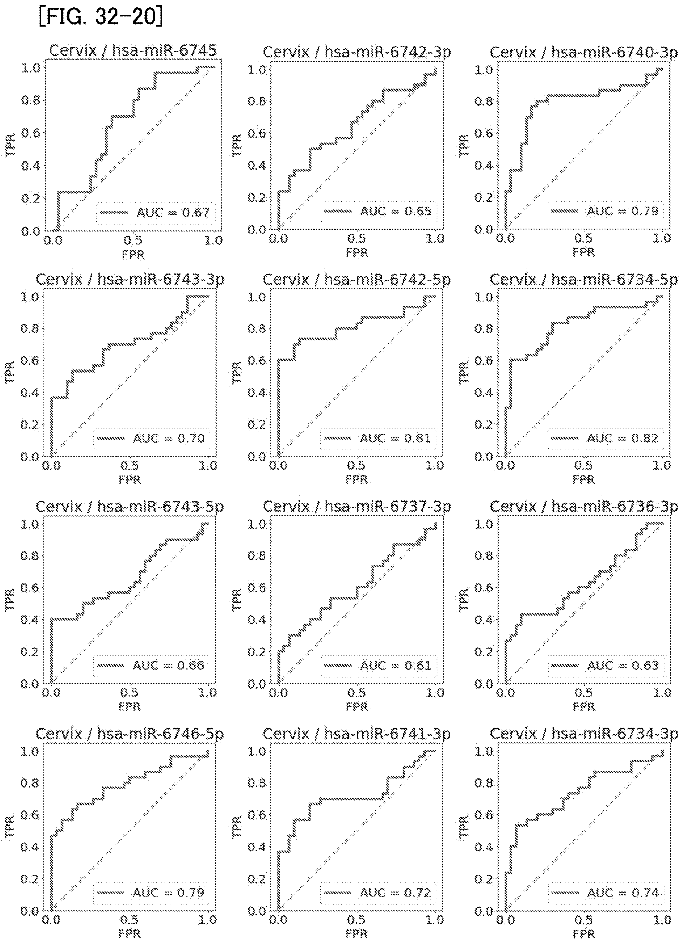

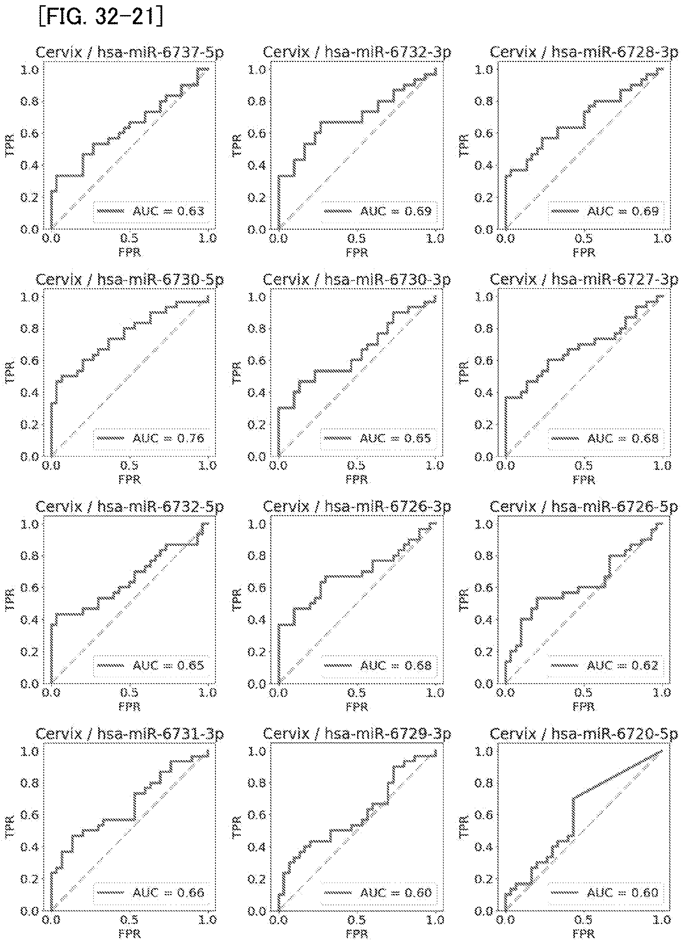

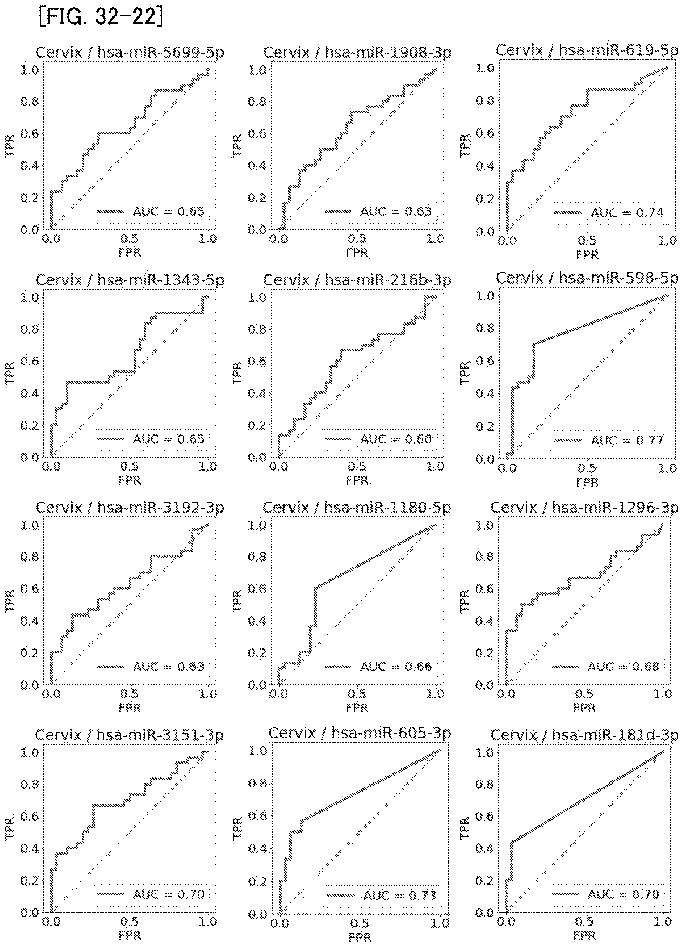

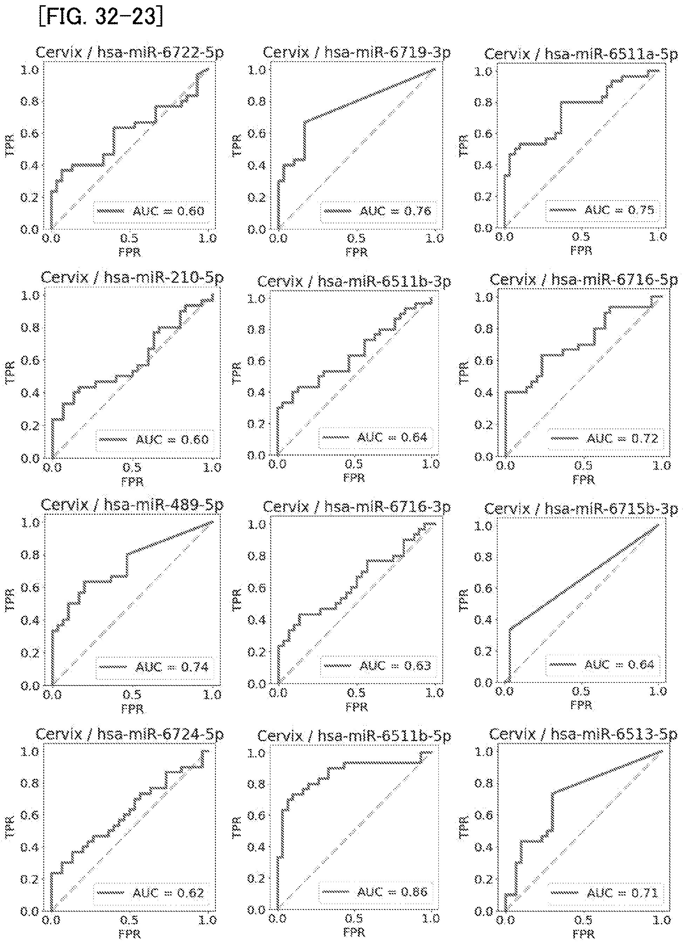

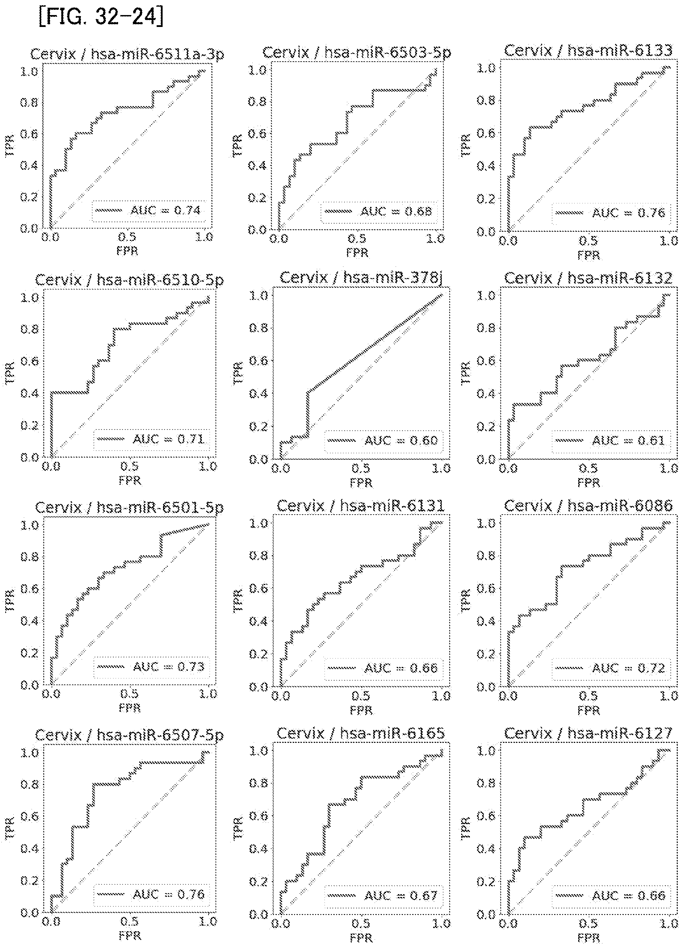

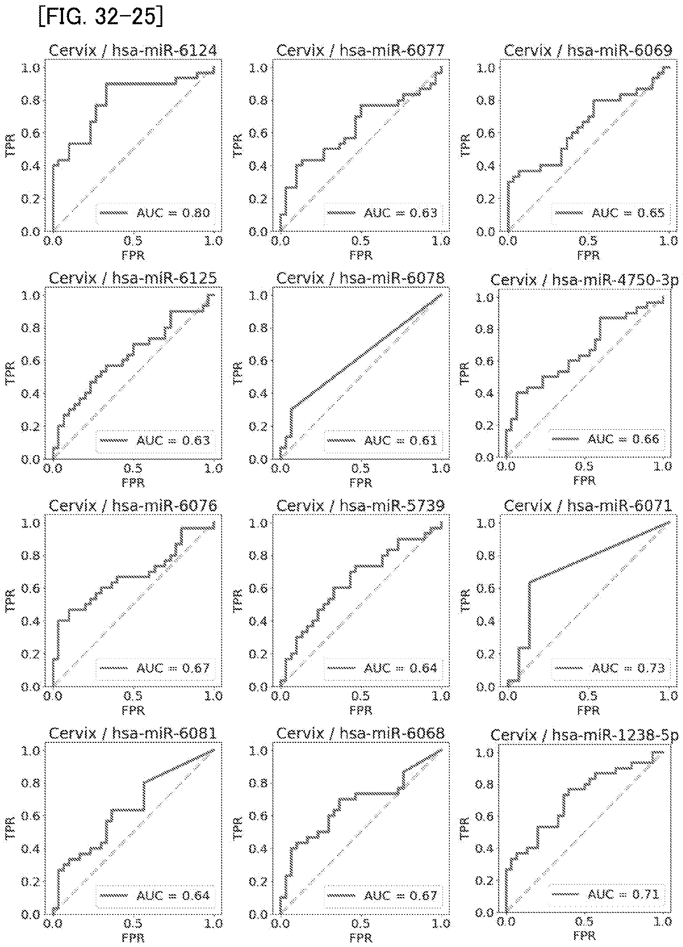

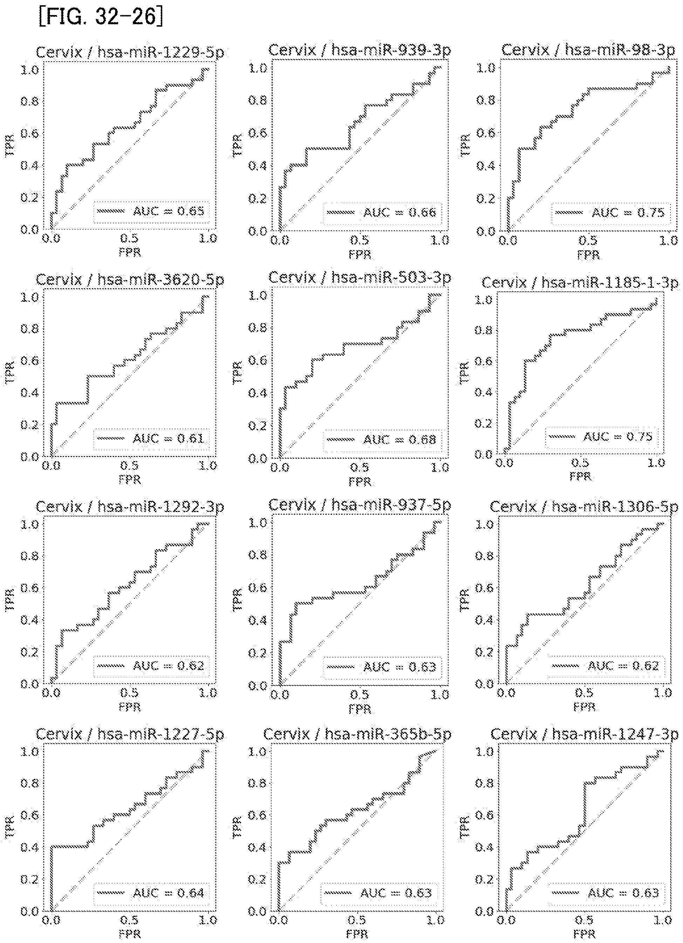

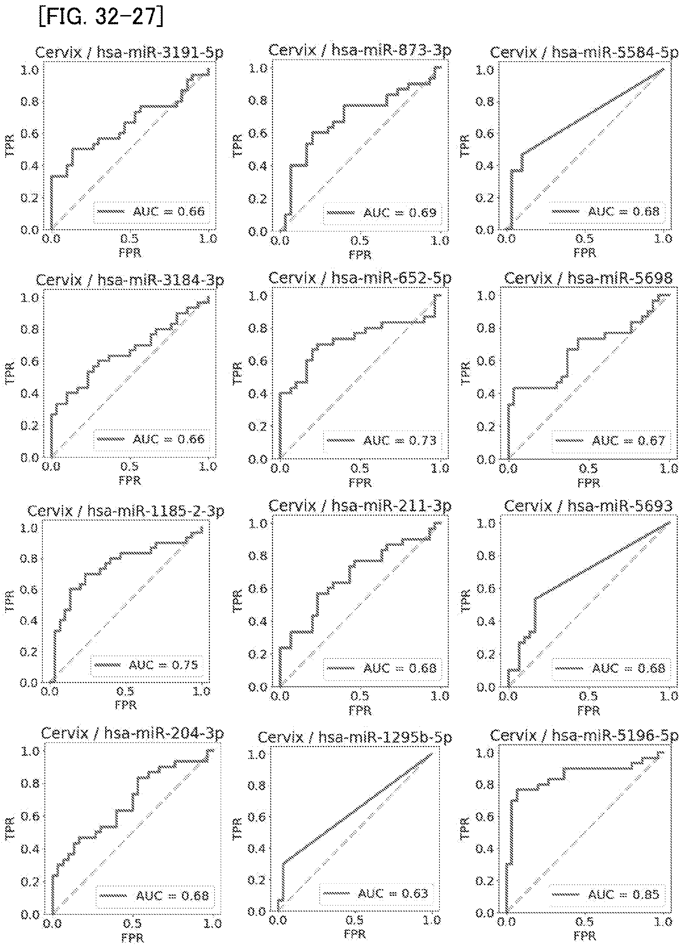

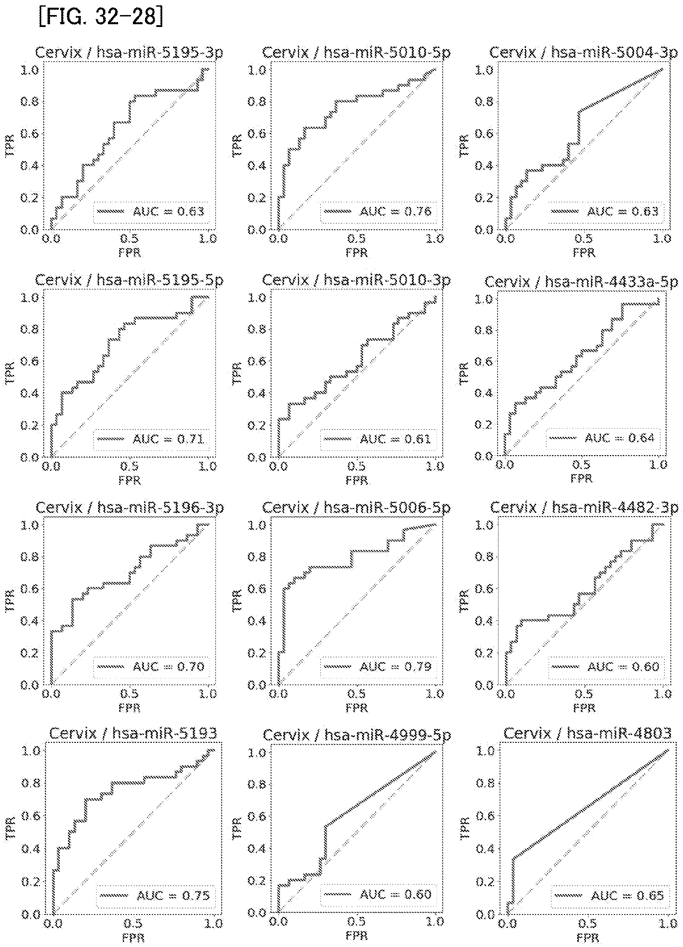

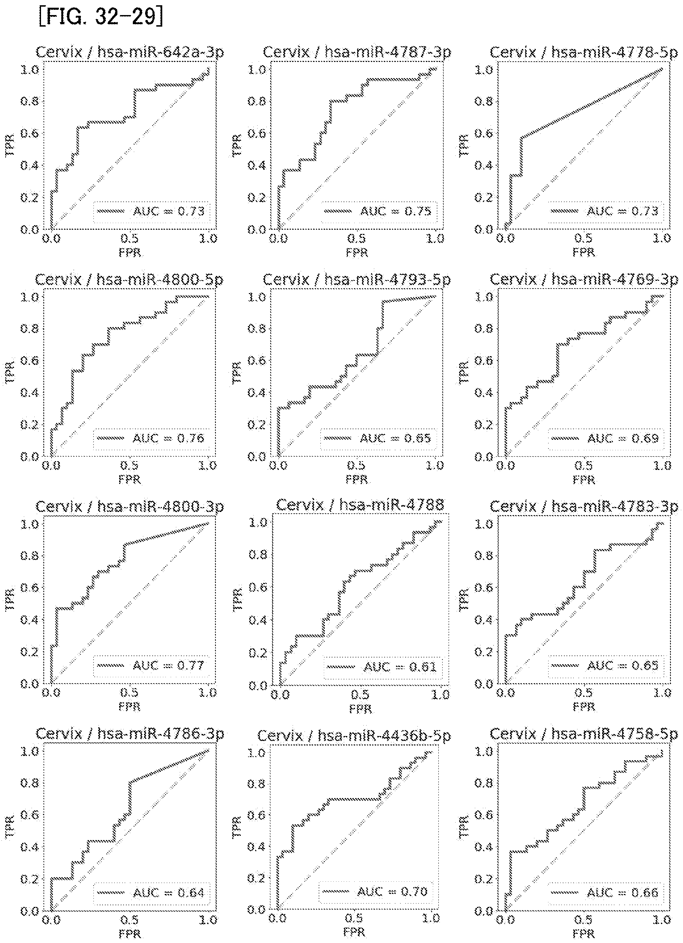

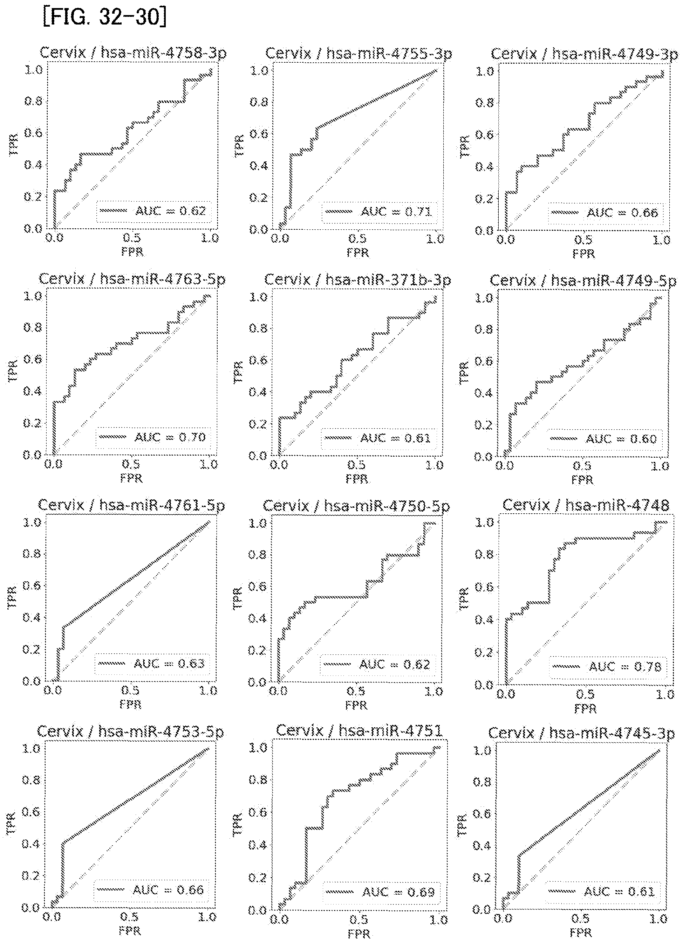

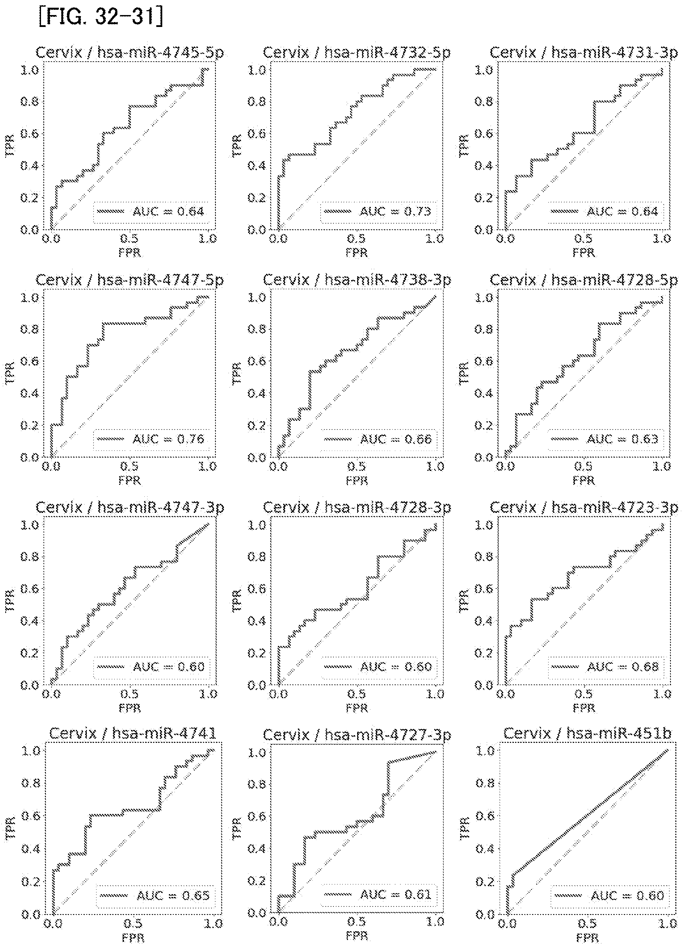

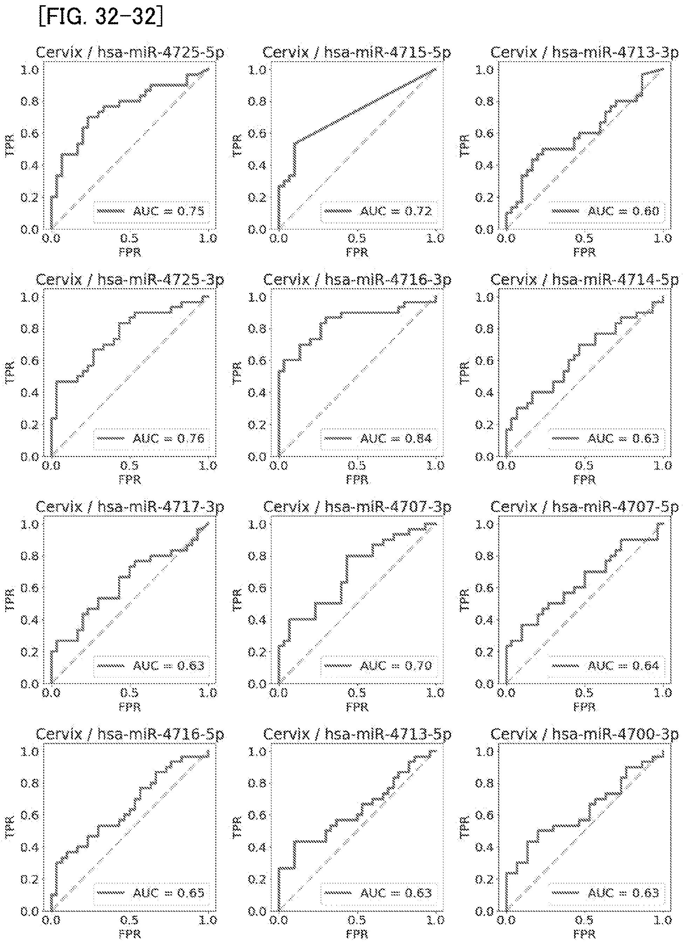

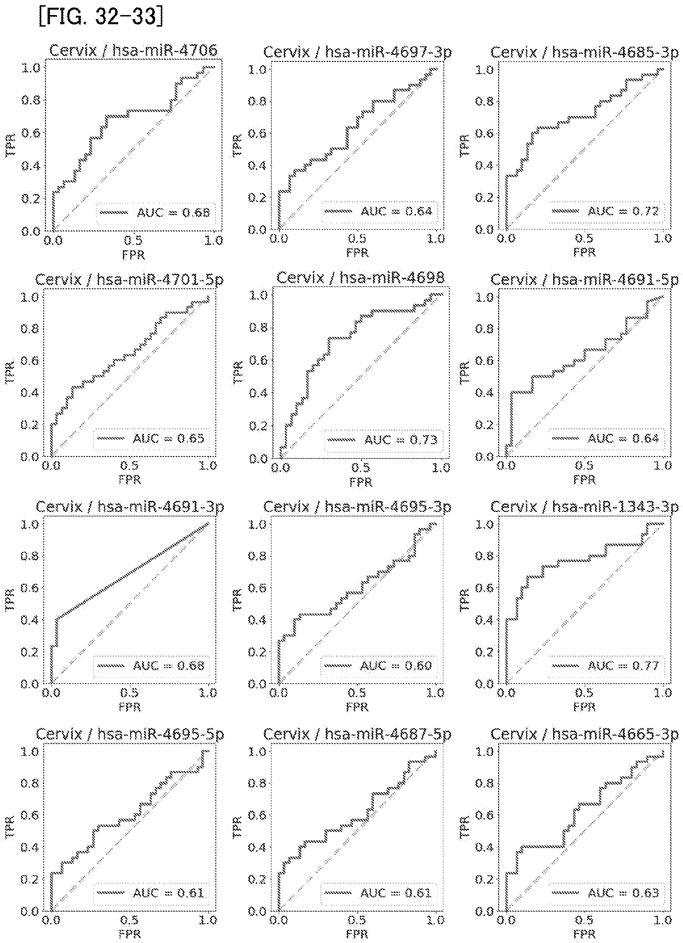

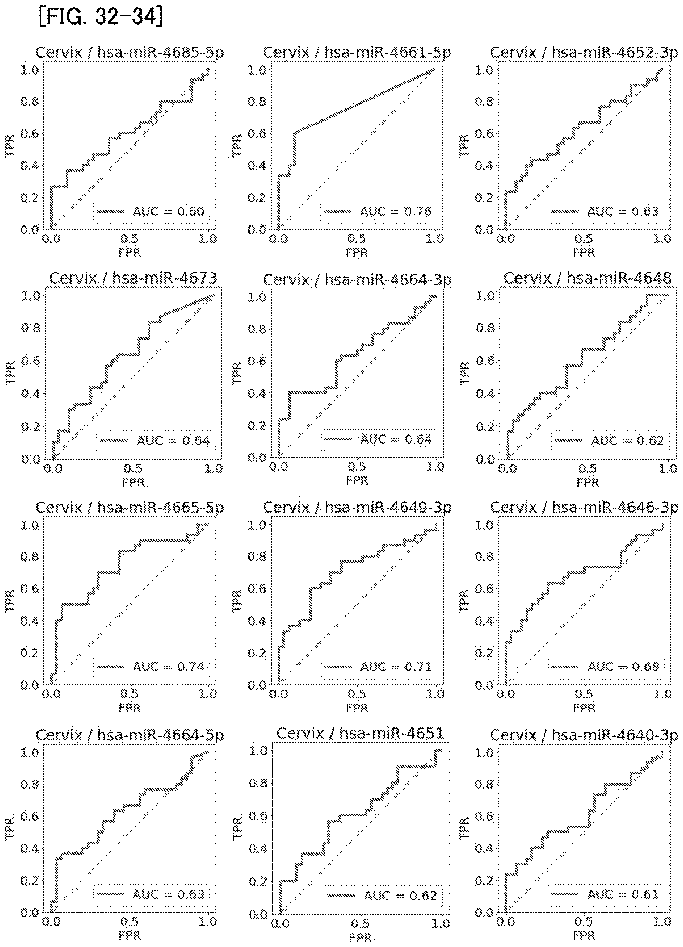

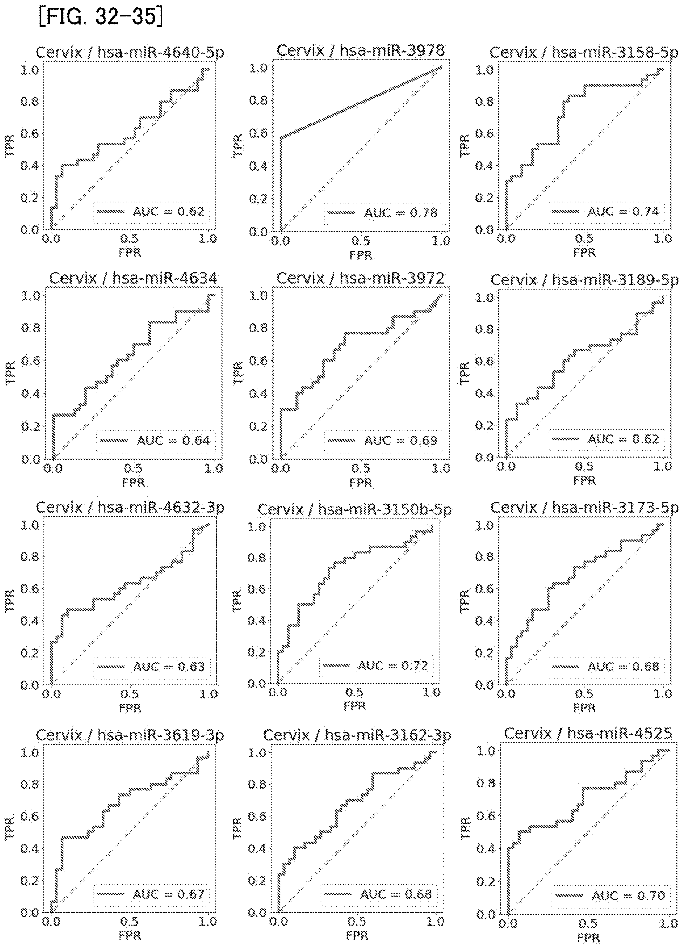

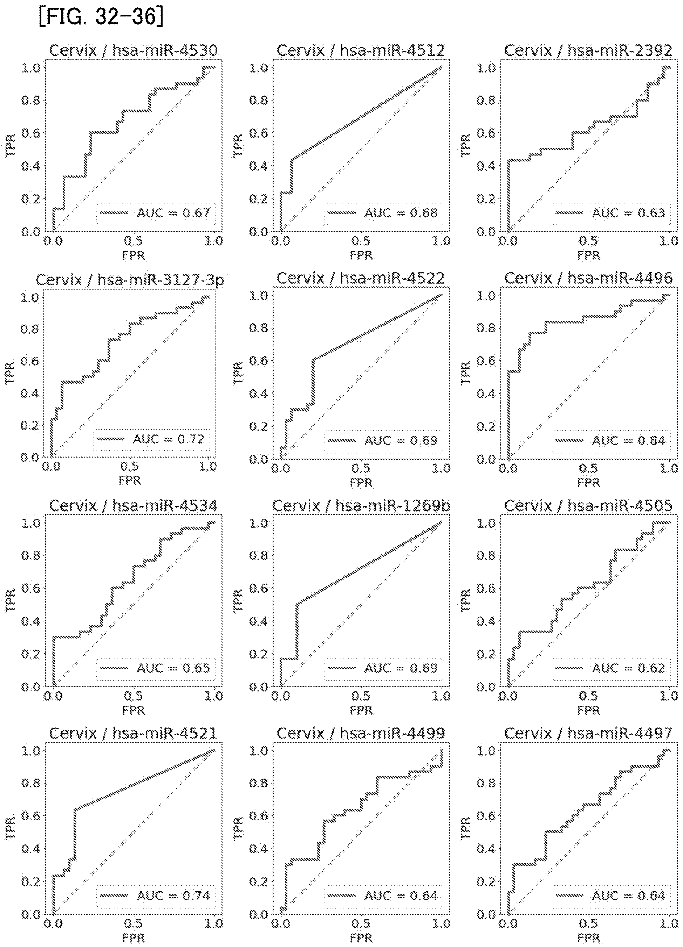

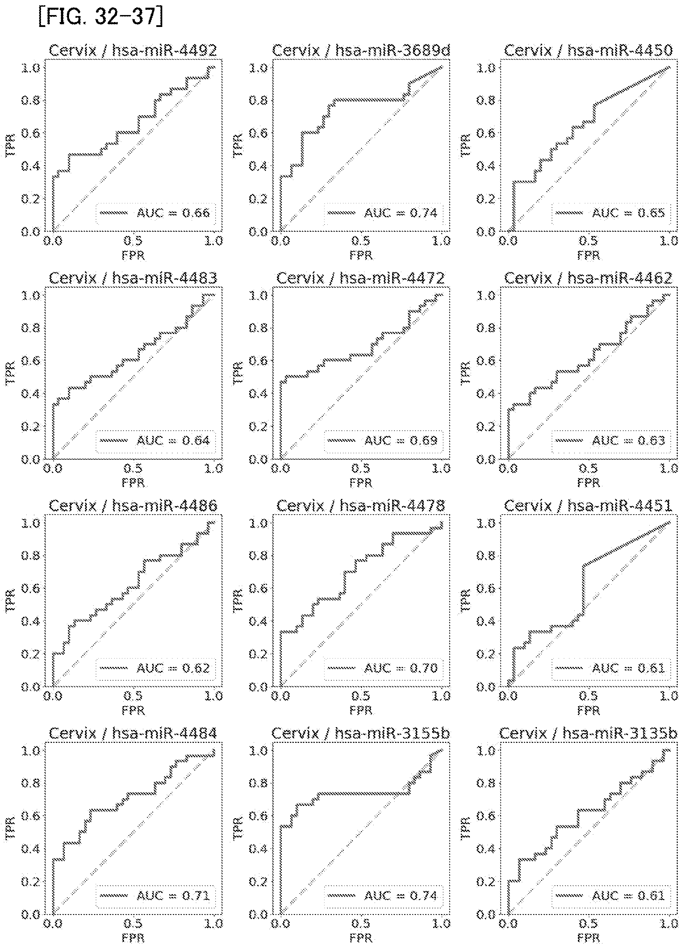

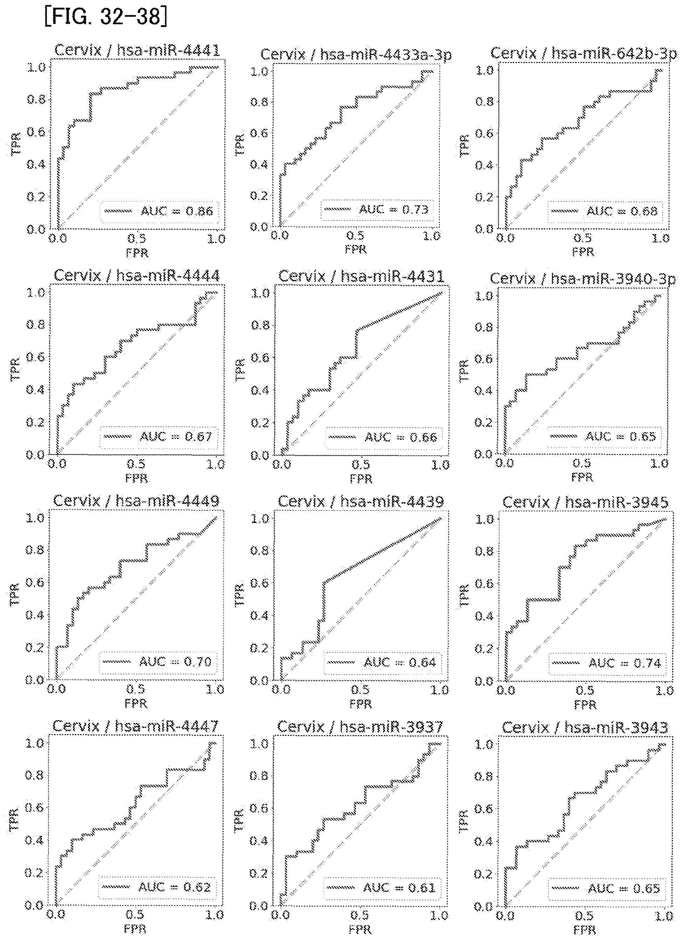

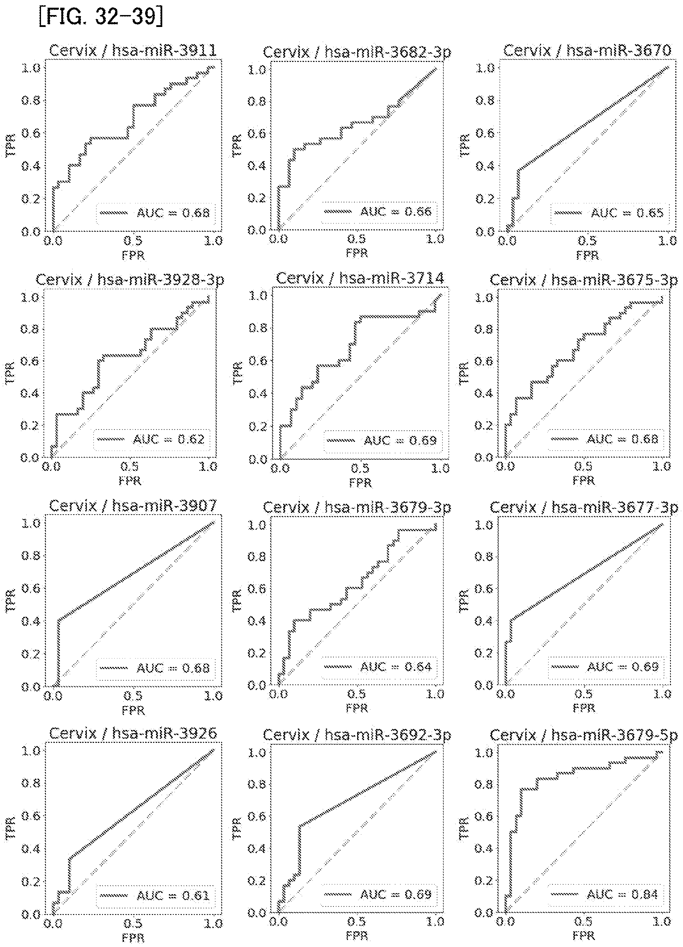

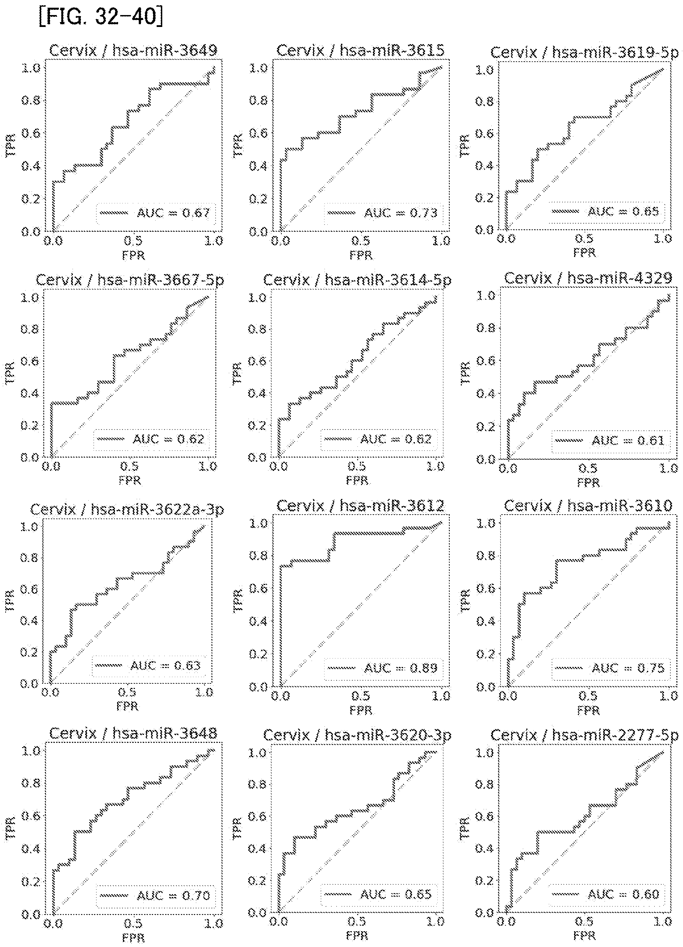

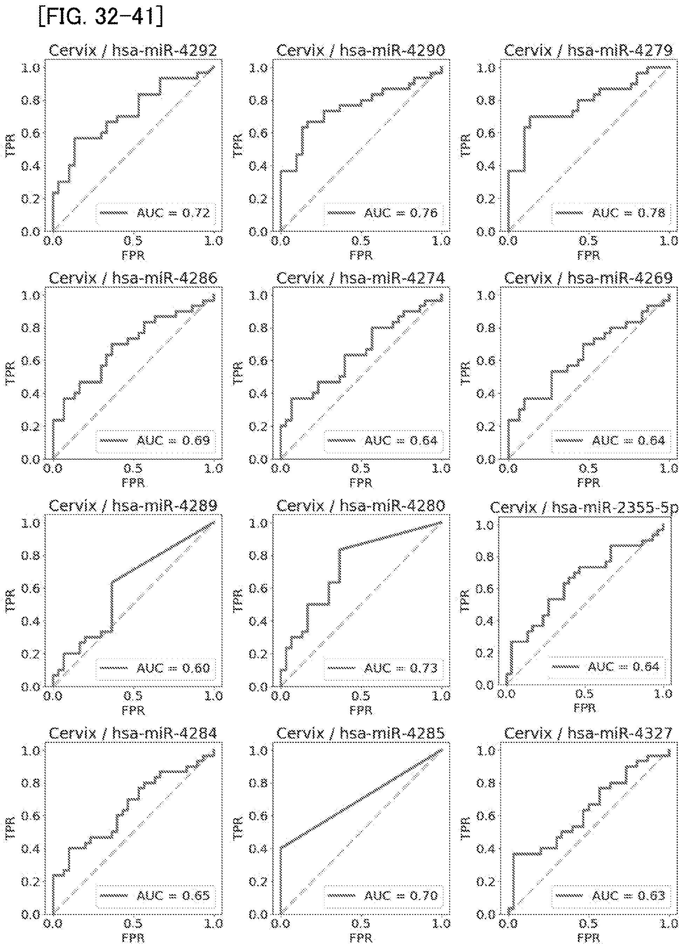

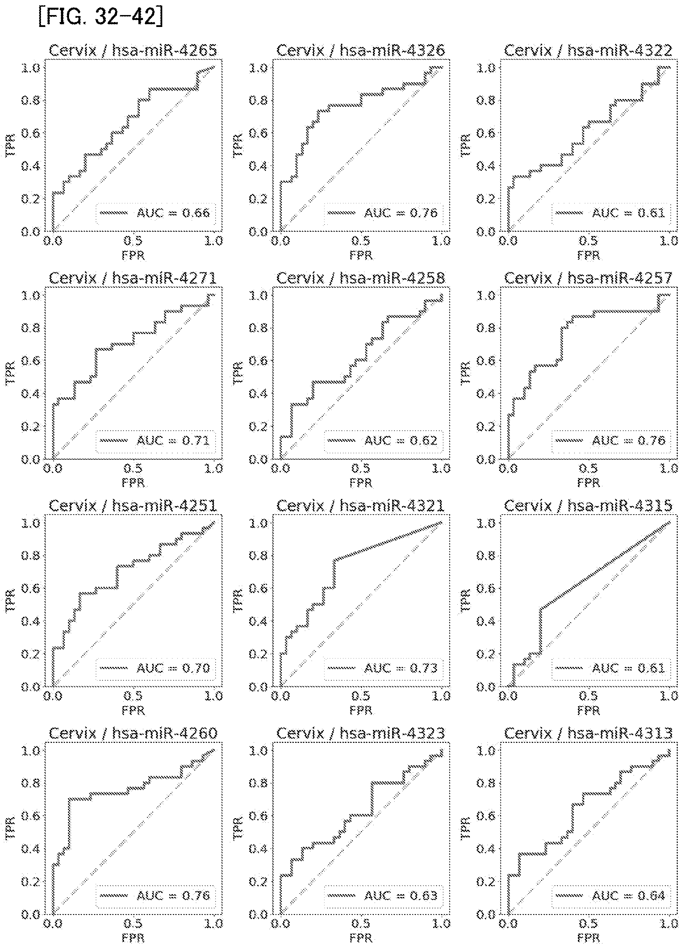

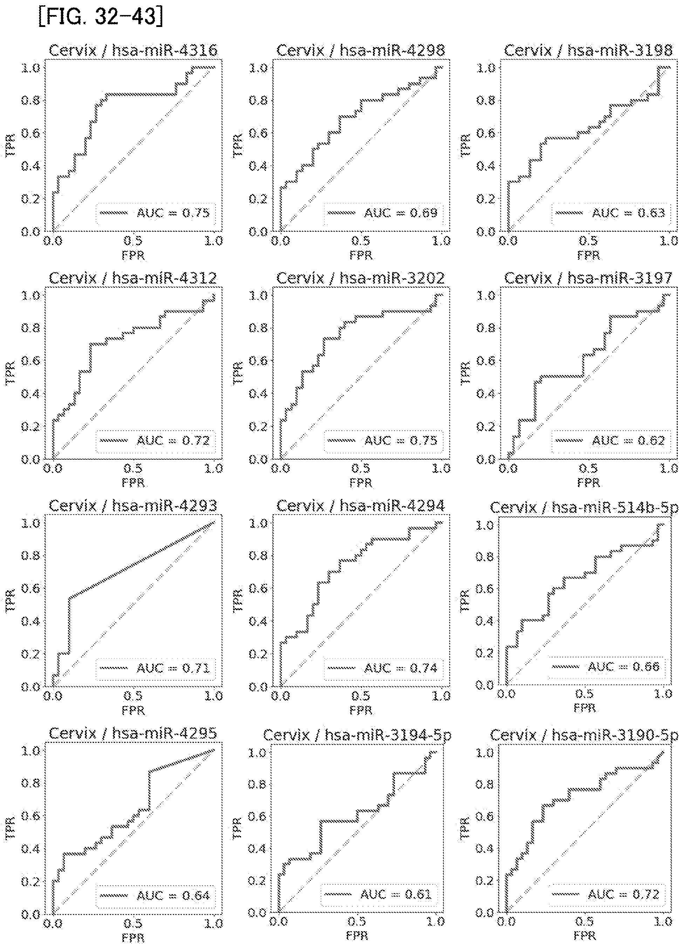

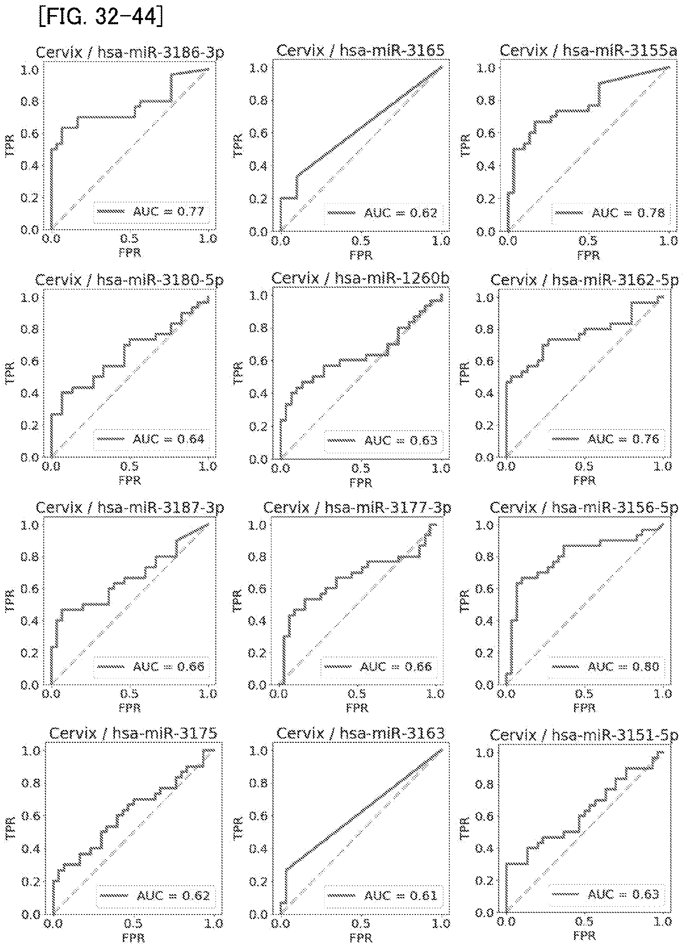

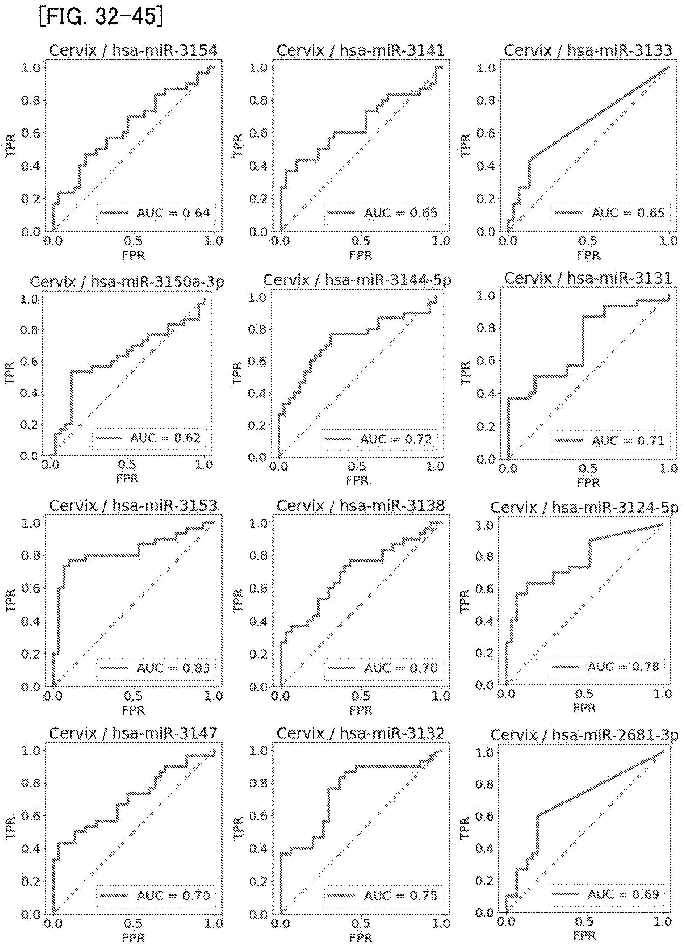

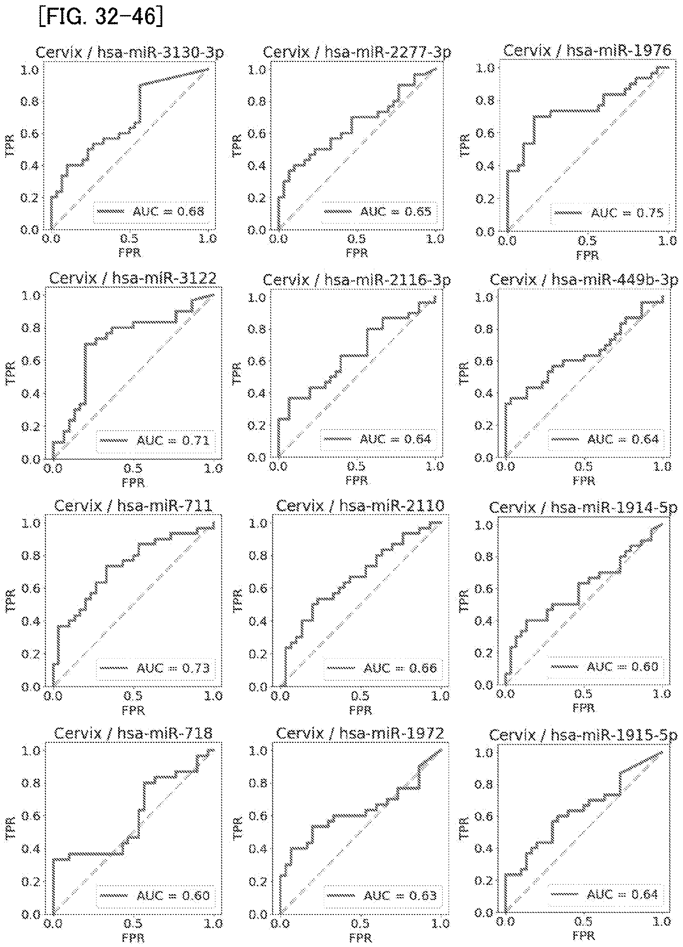

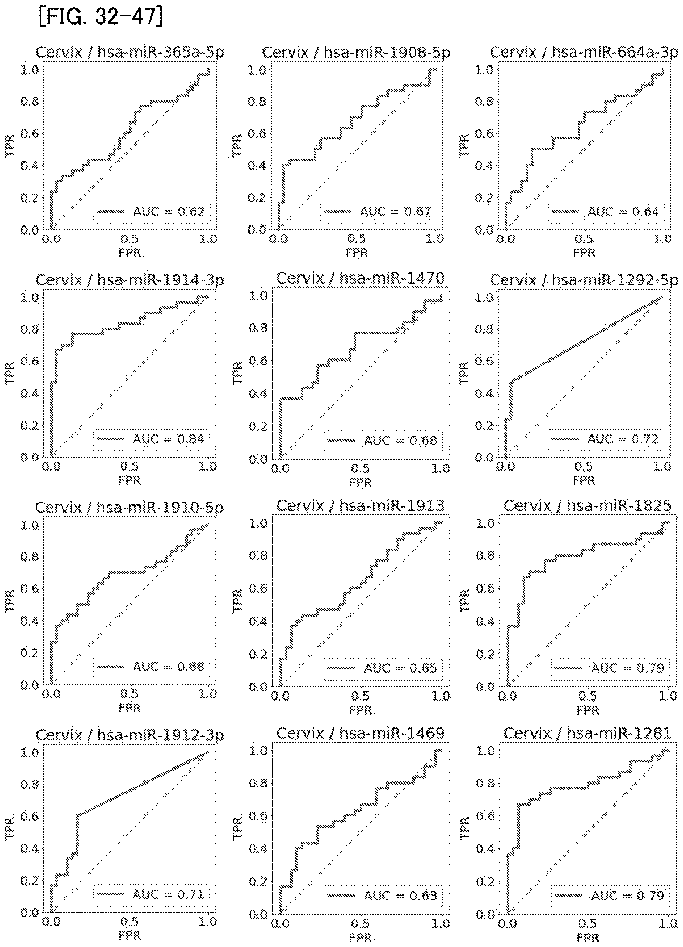

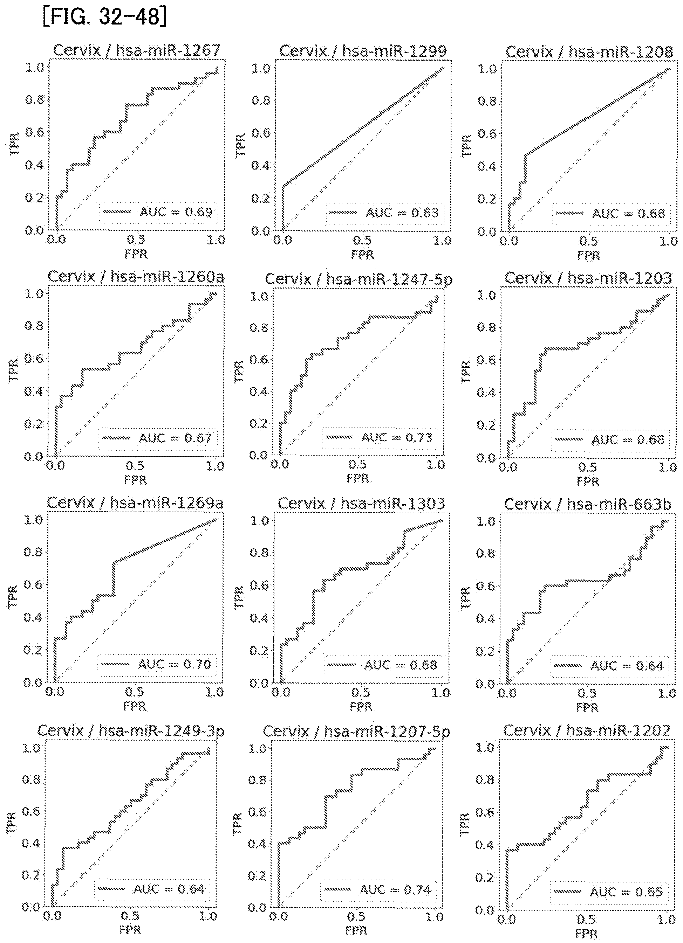

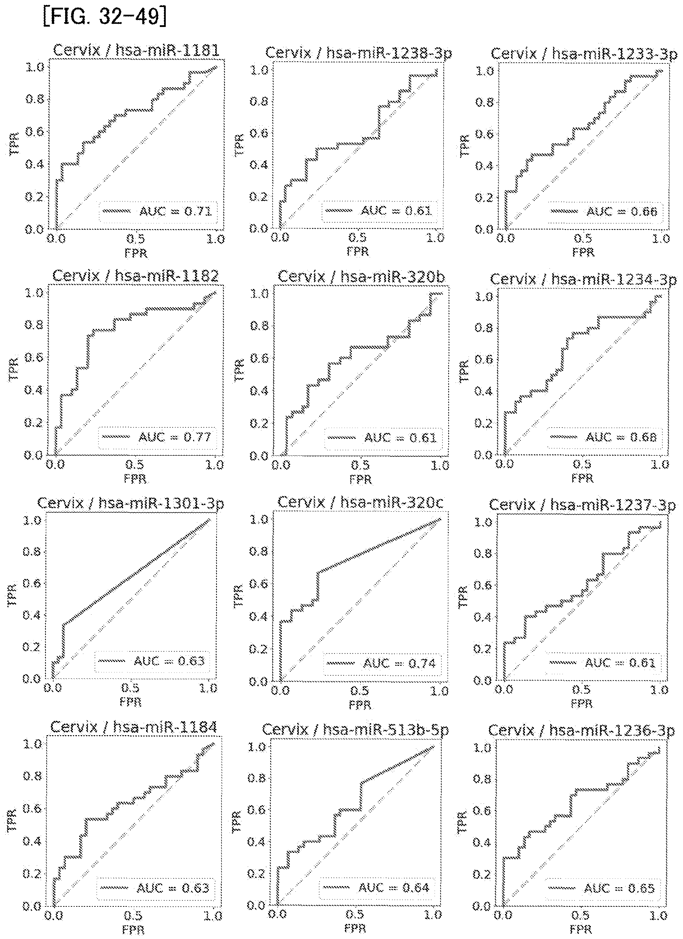

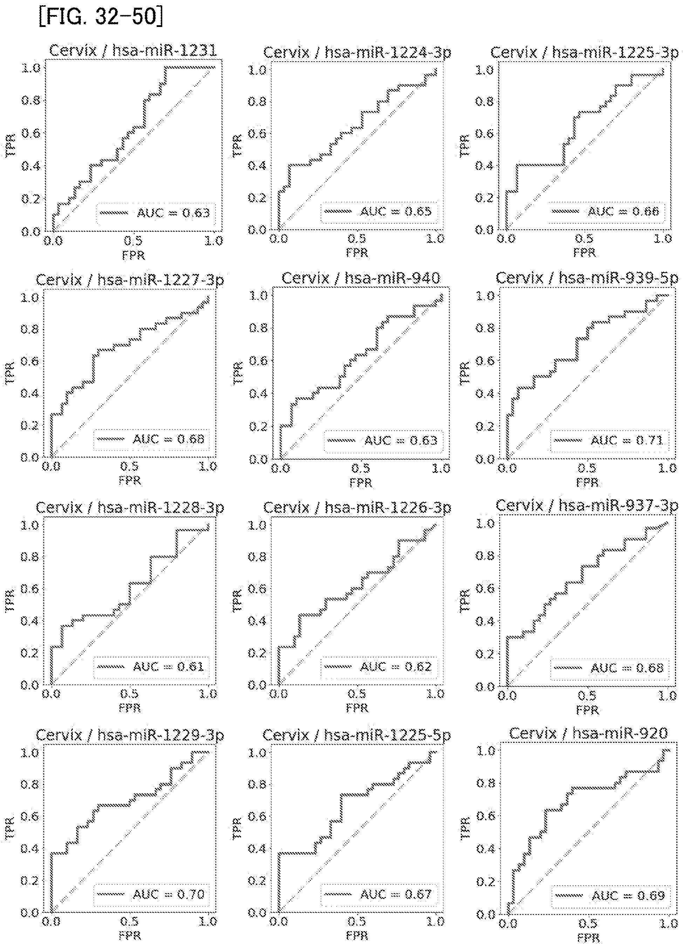

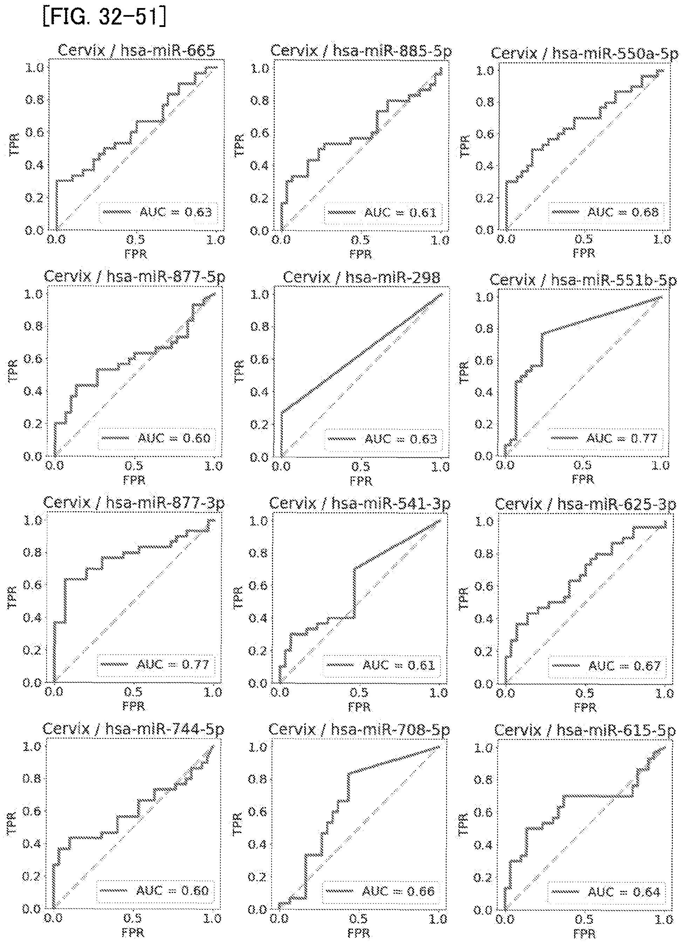

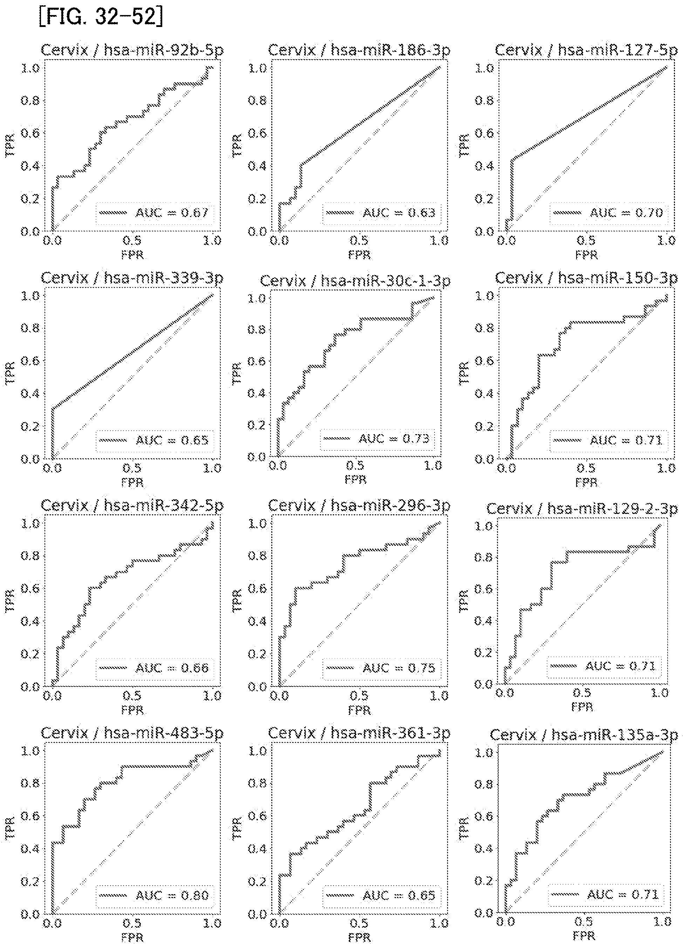

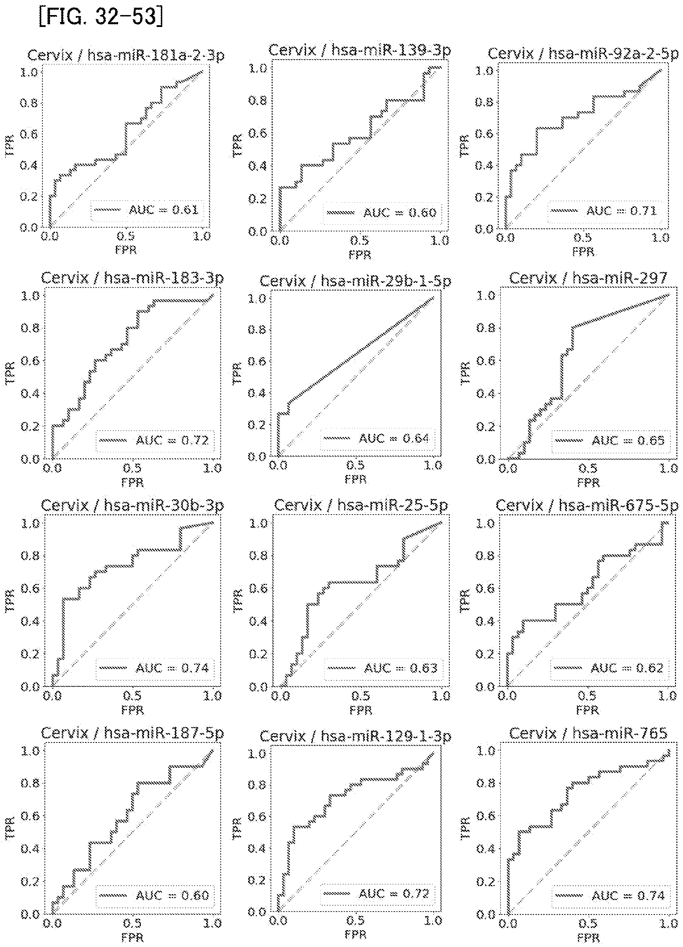

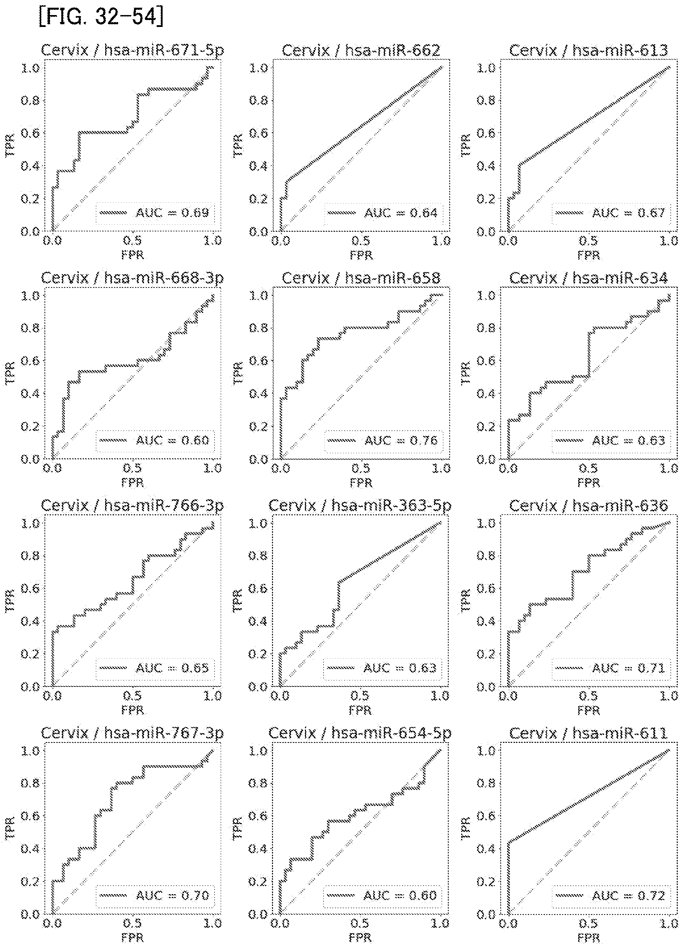

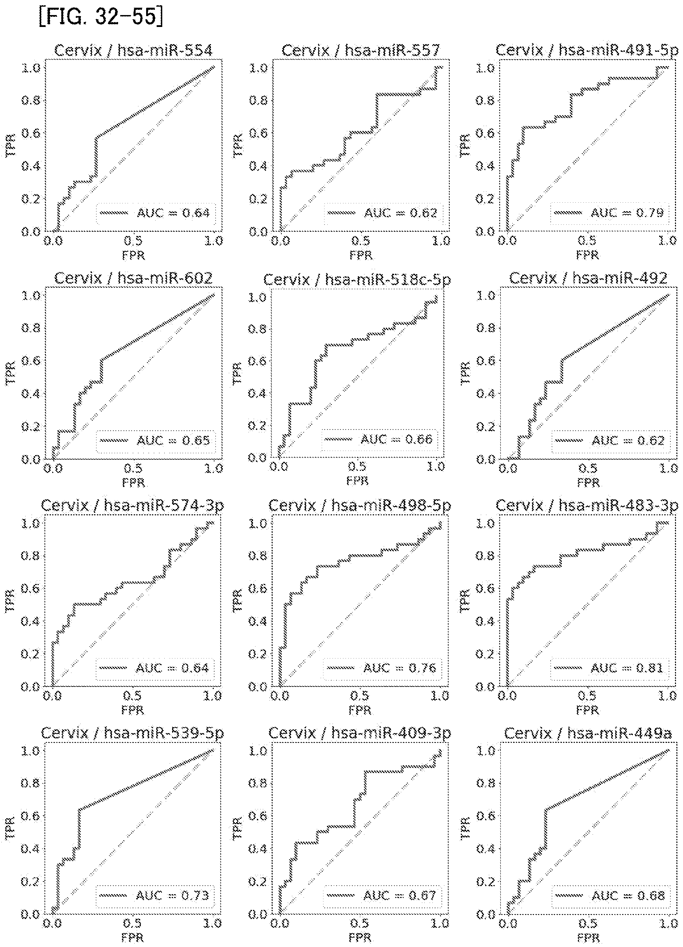

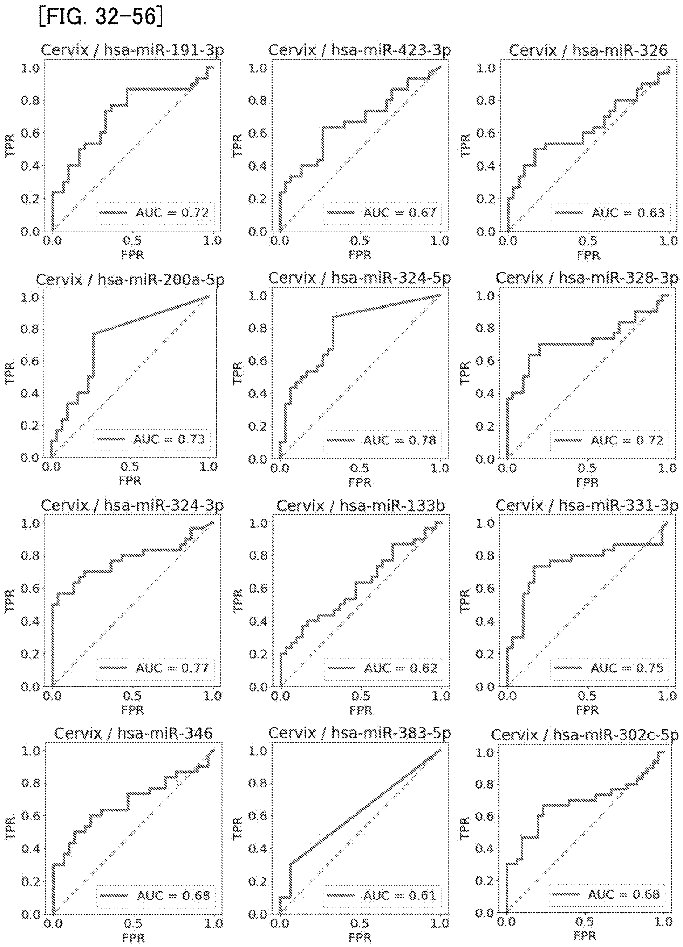

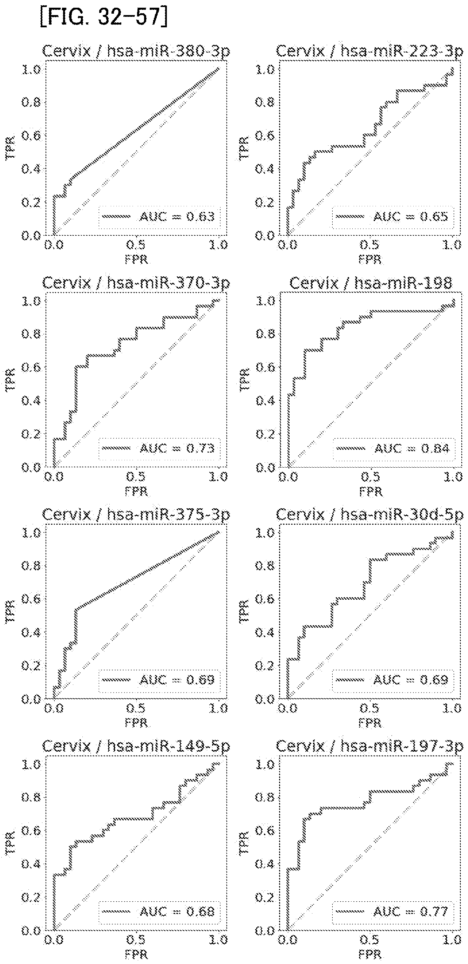

FIGS. 32-1 to 32-57 show ROC-curves for predicting cervical cancer in humans using a single miRNA.

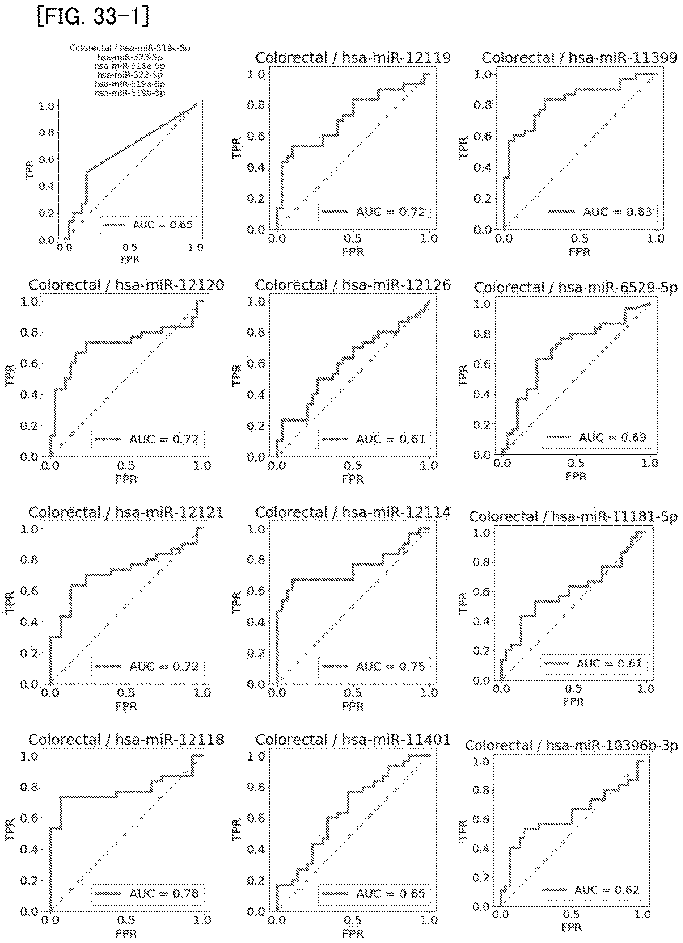

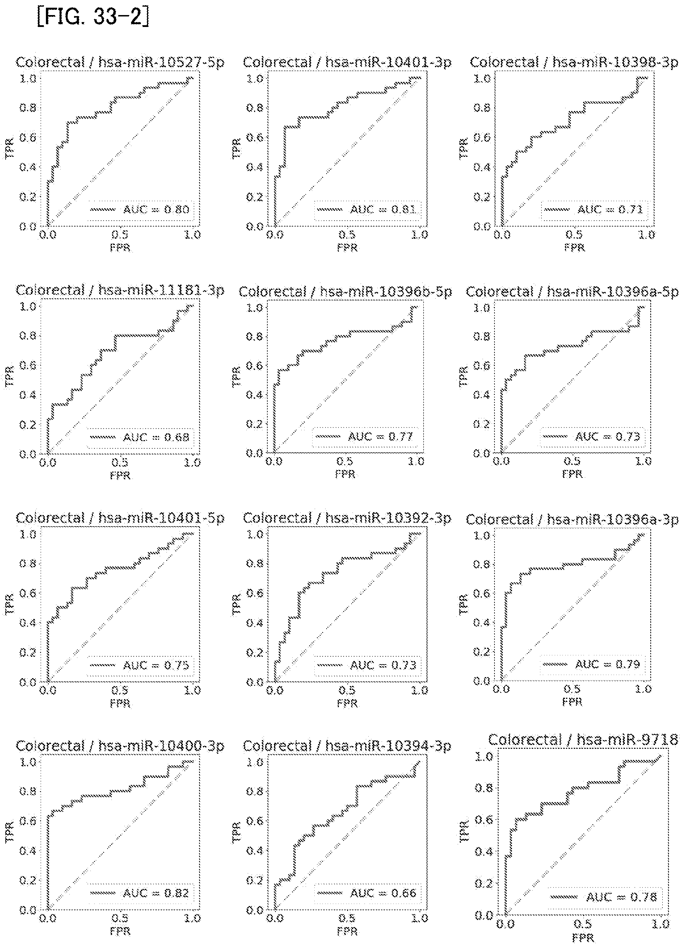

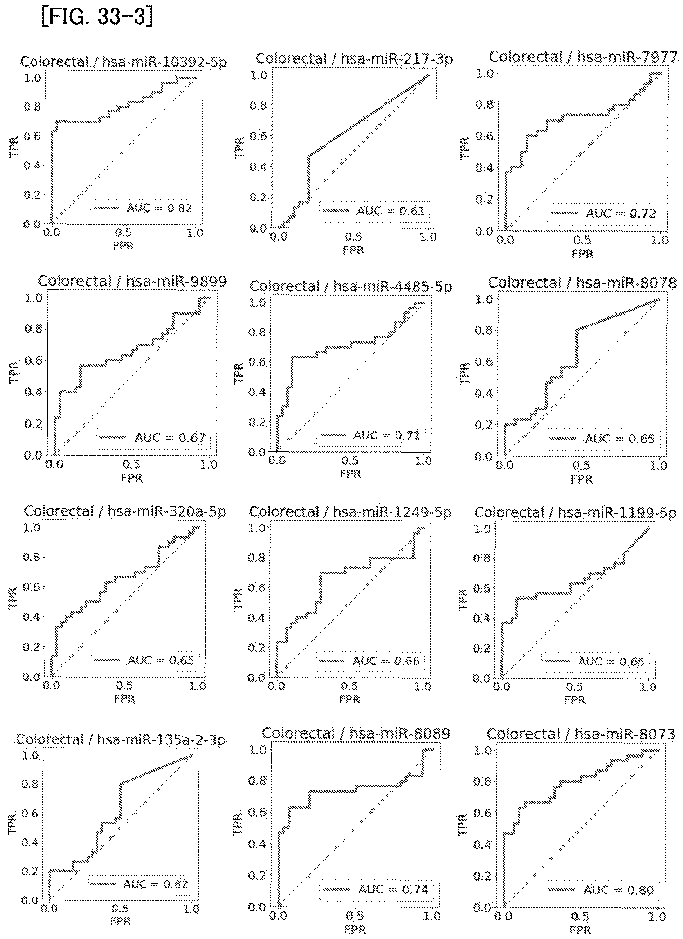

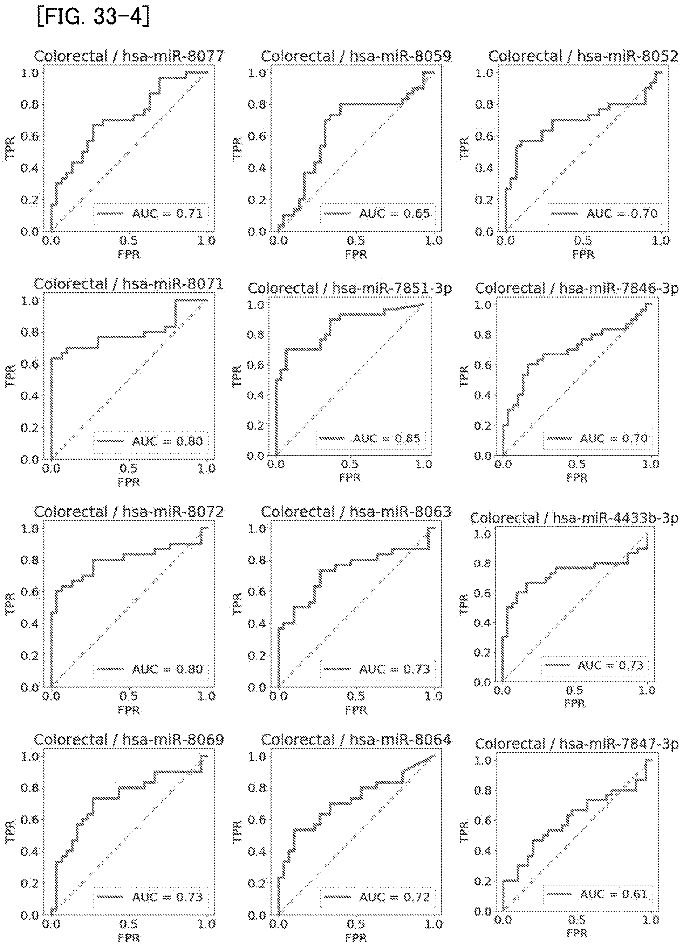

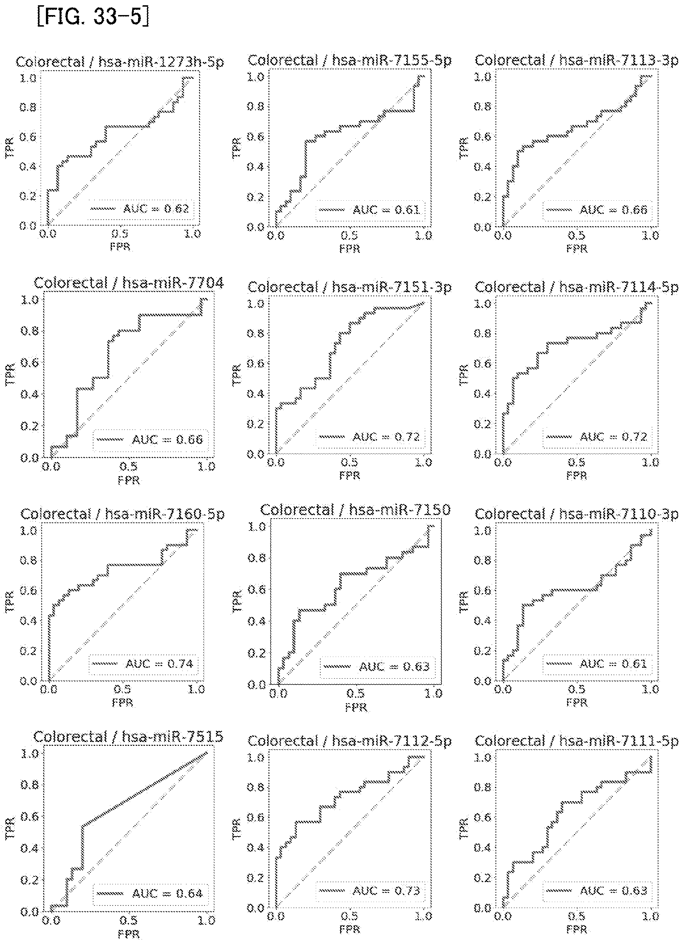

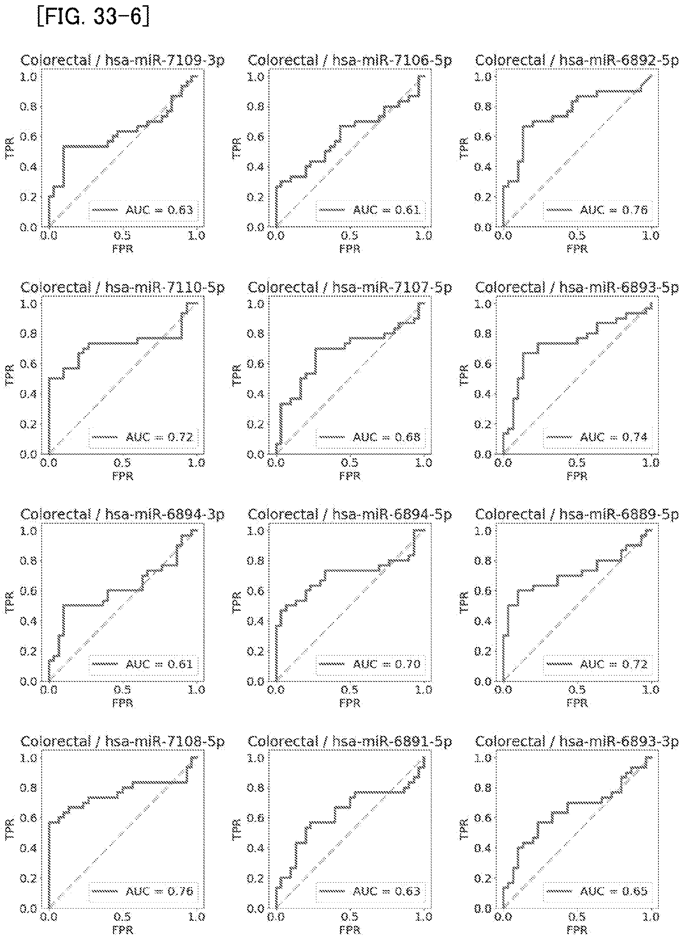

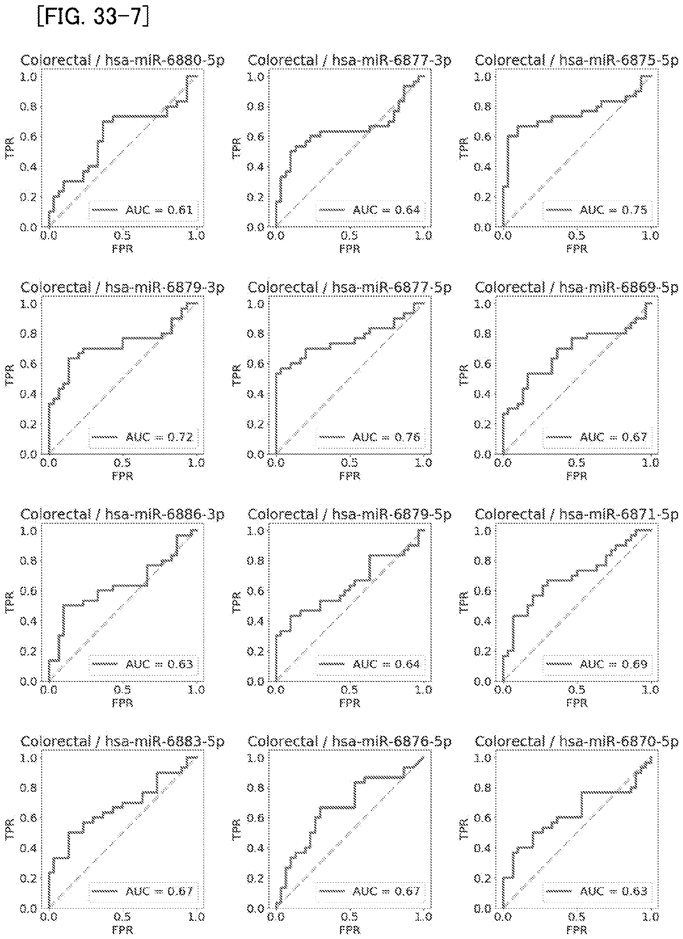

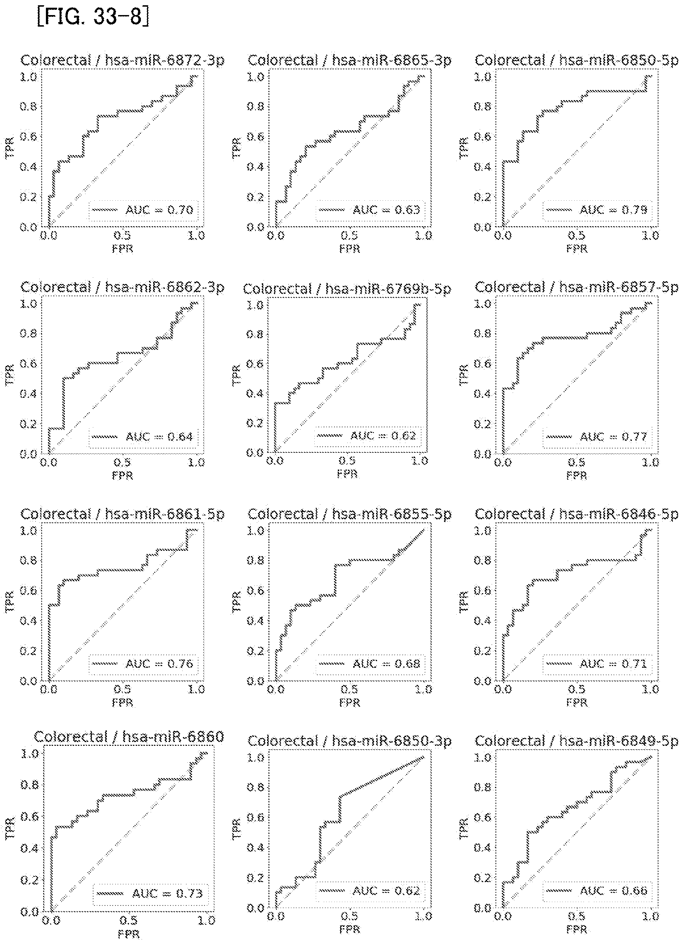

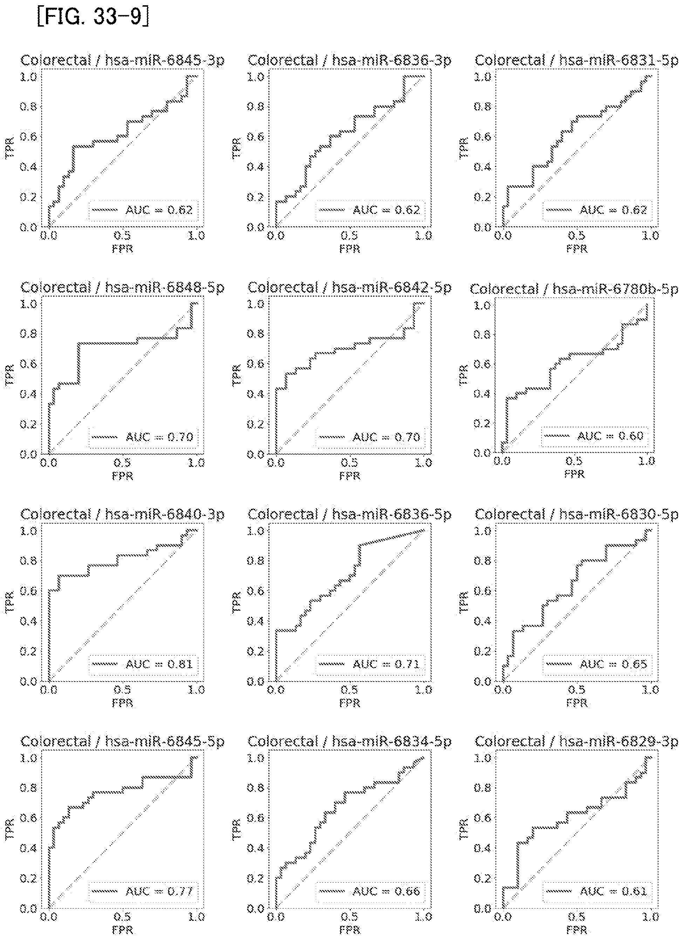

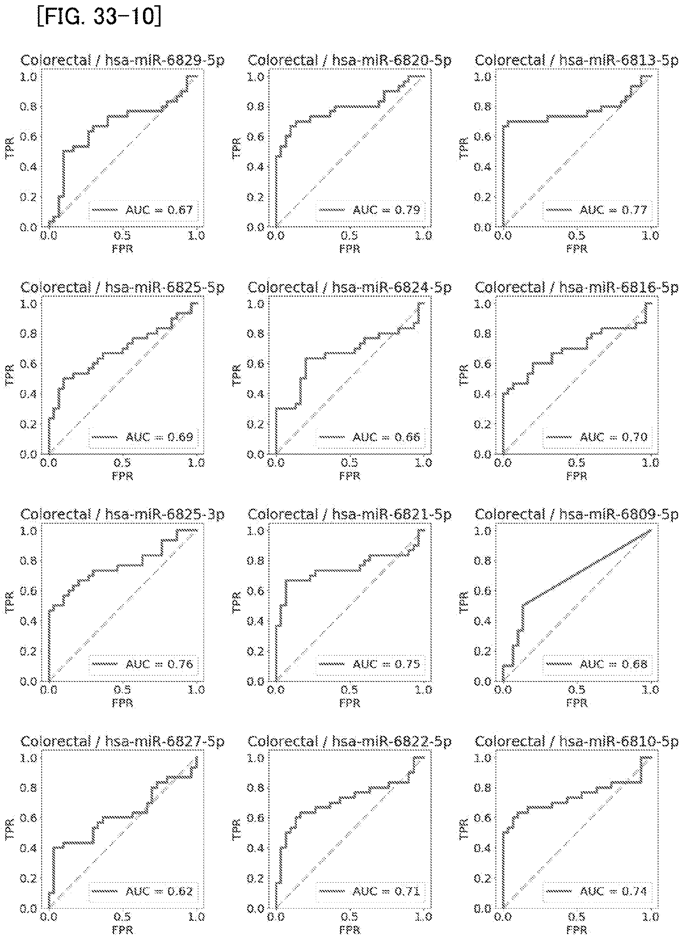

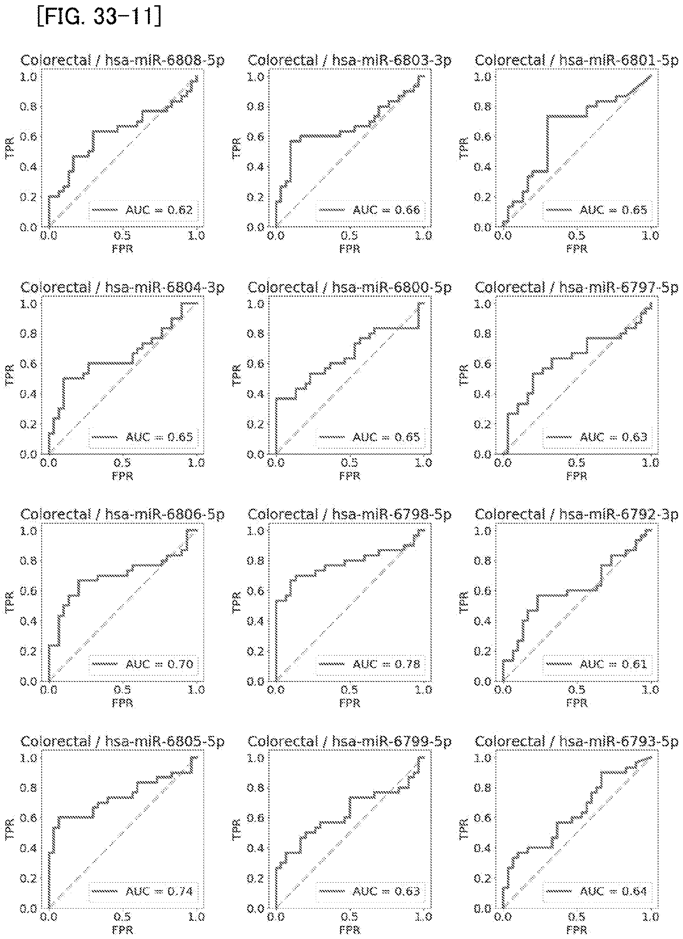

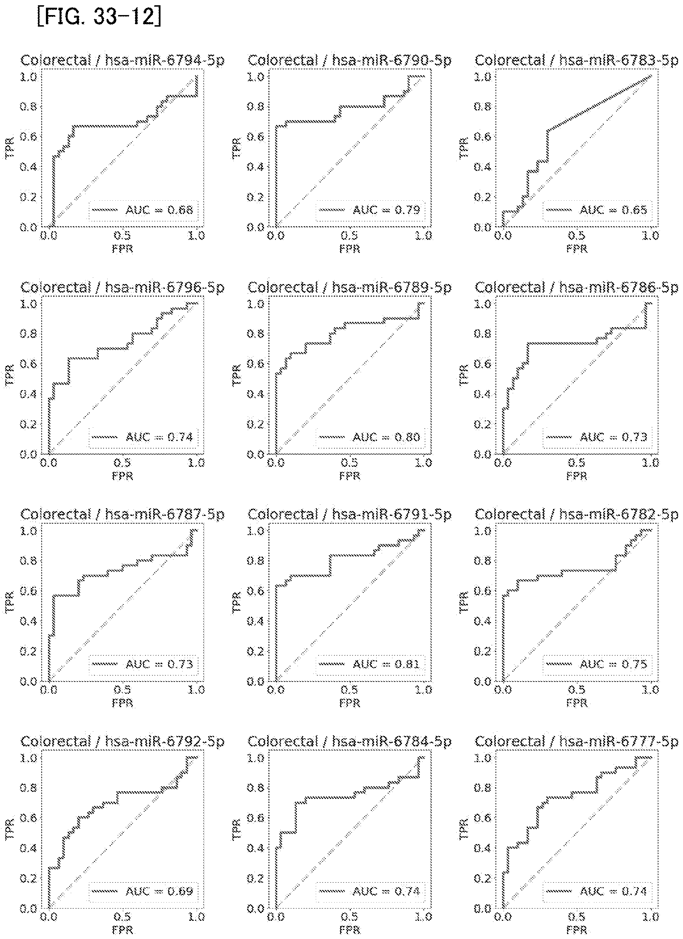

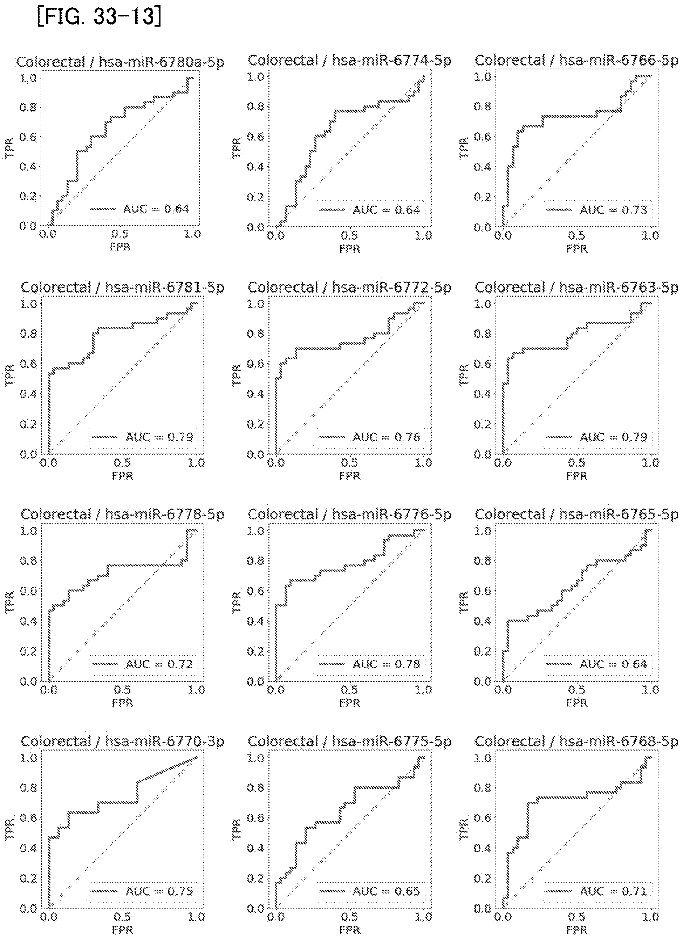

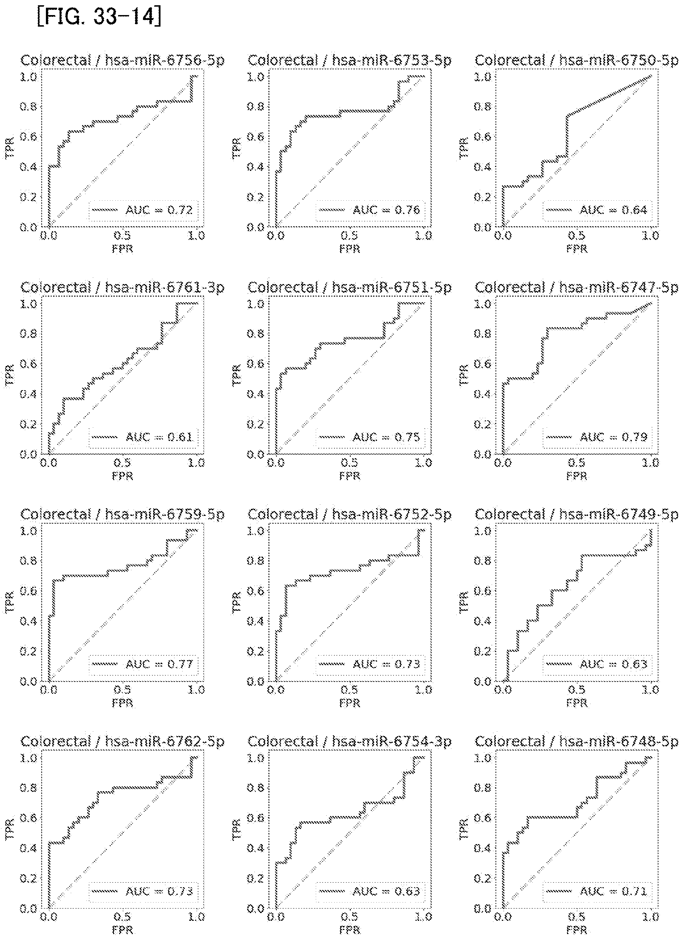

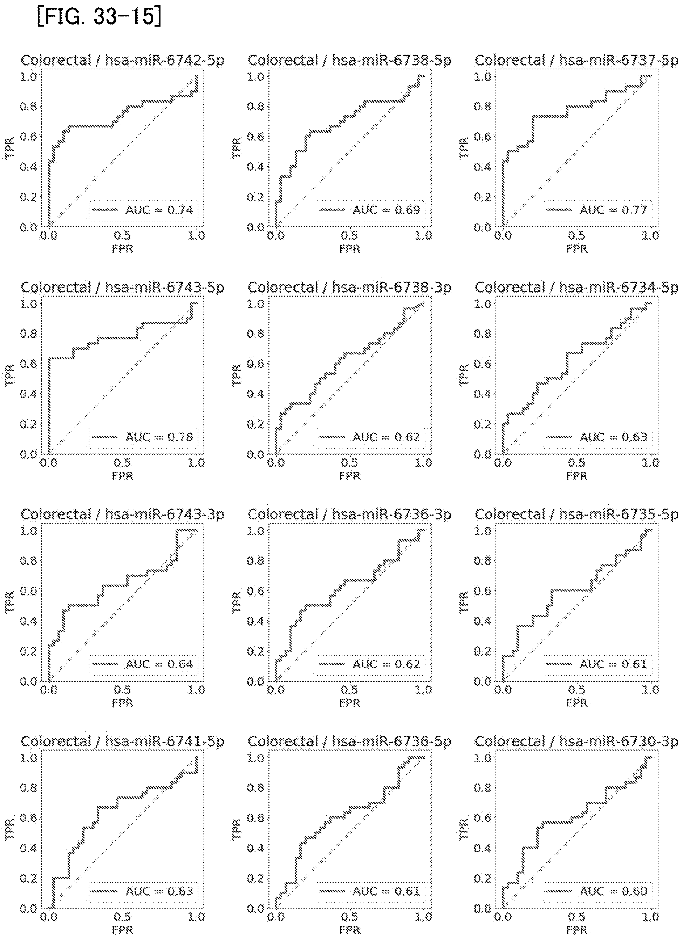

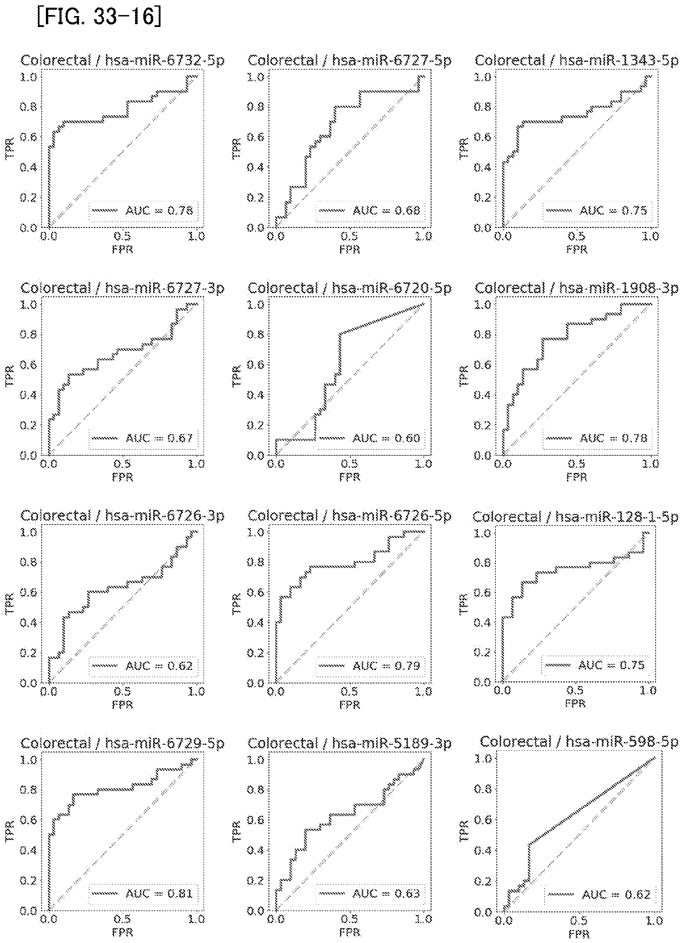

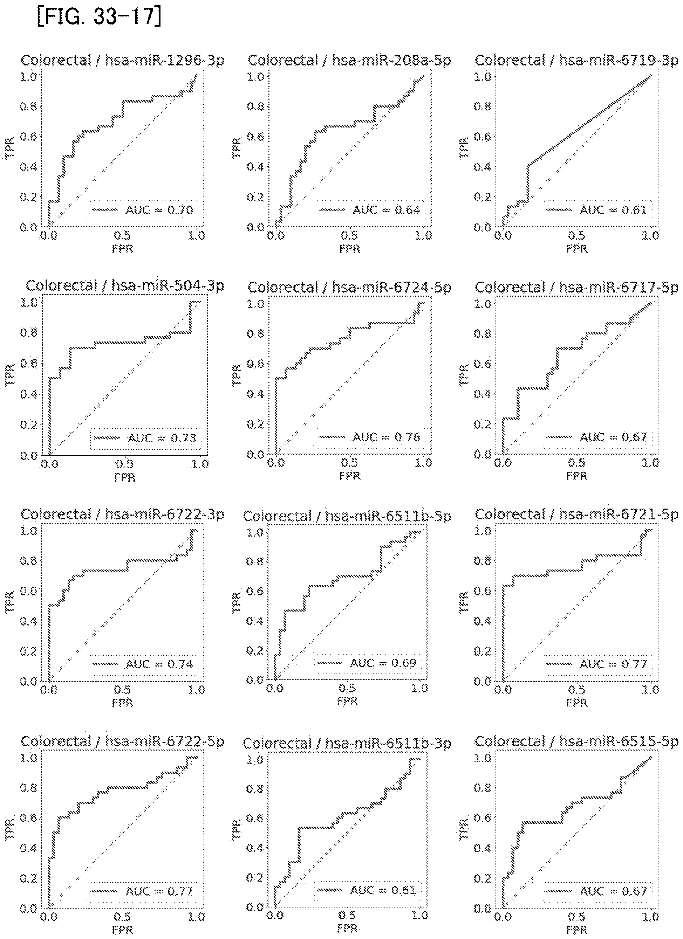

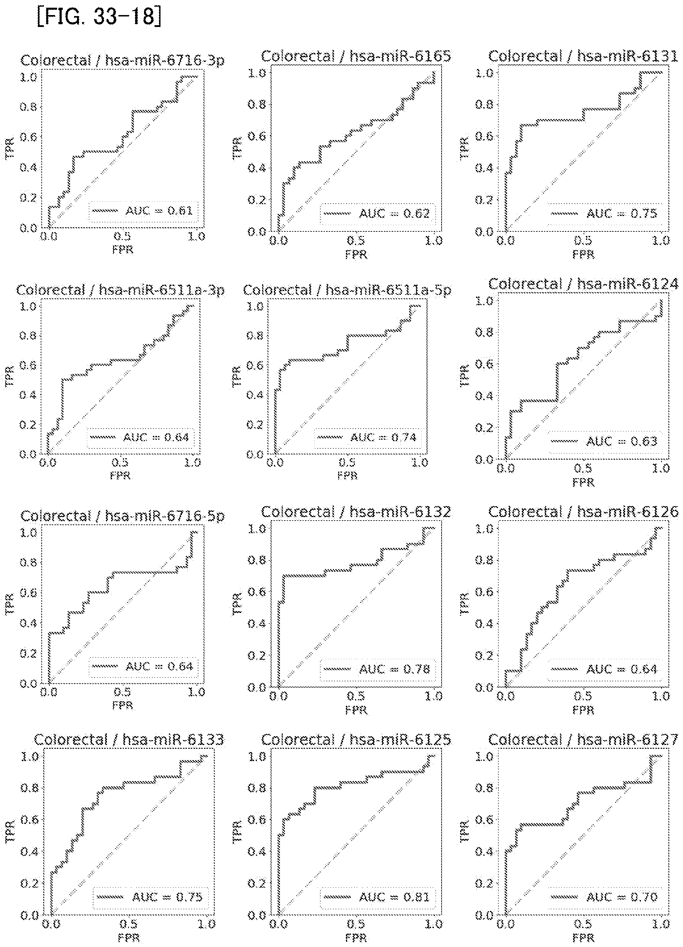

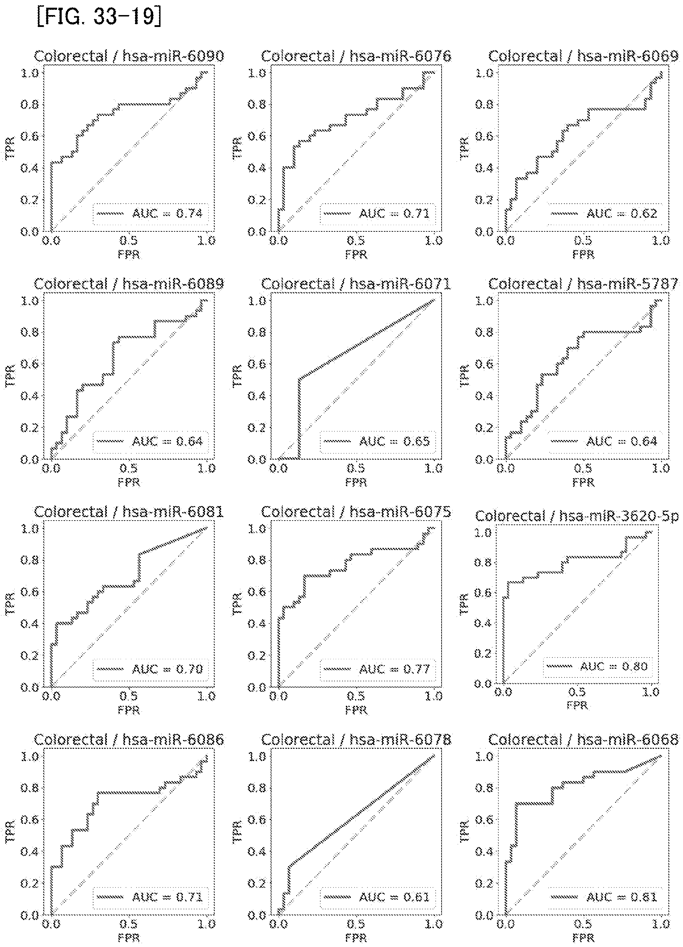

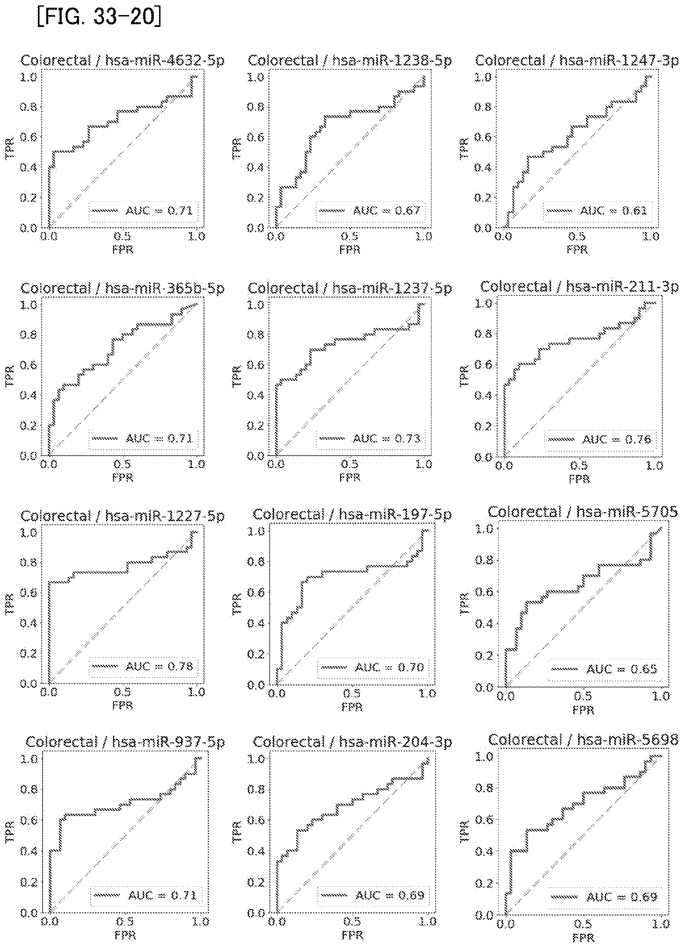

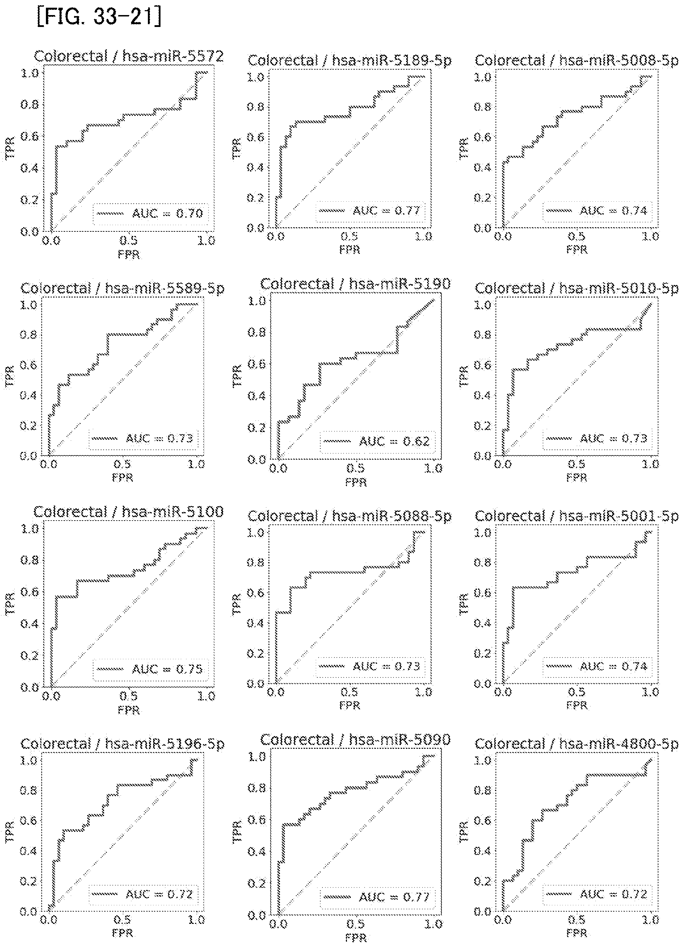

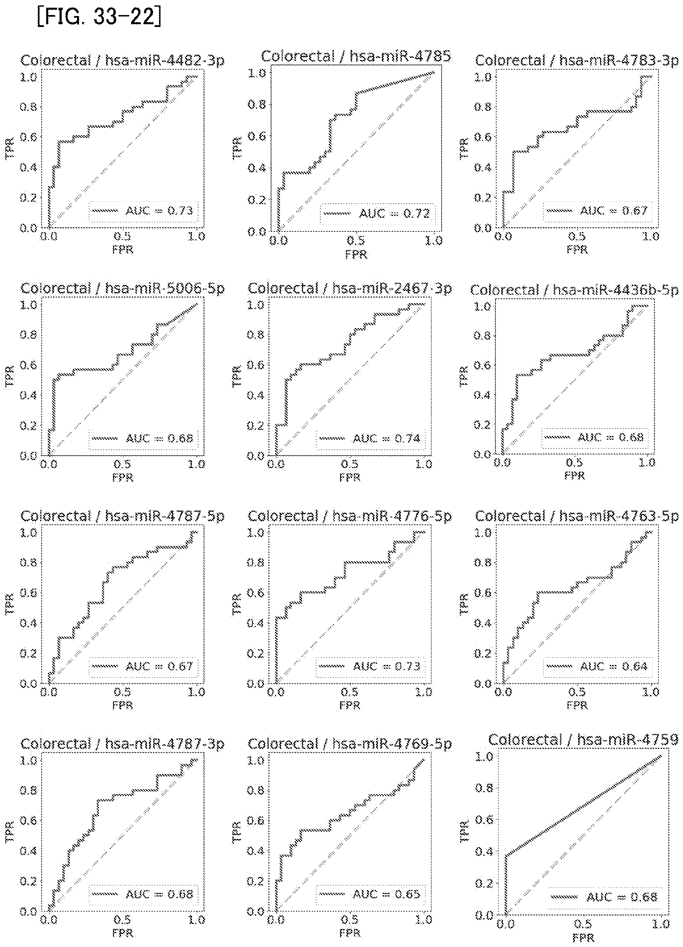

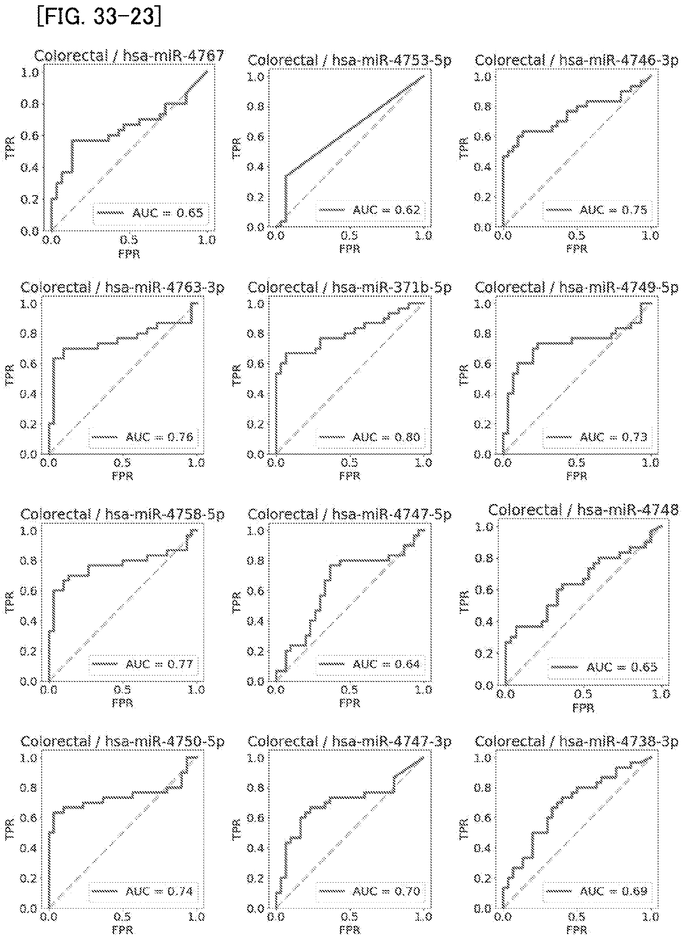

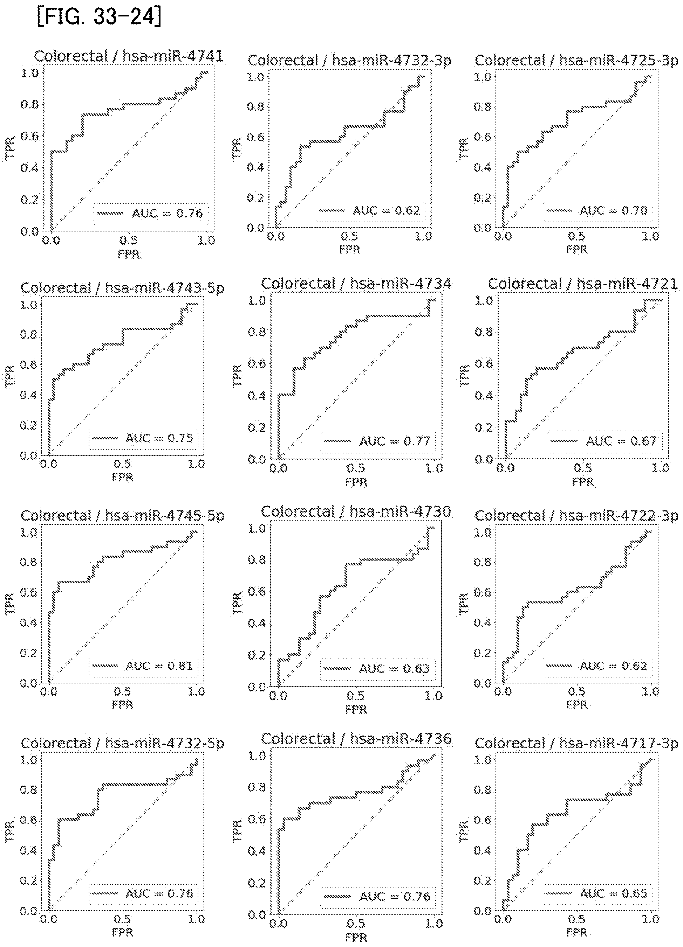

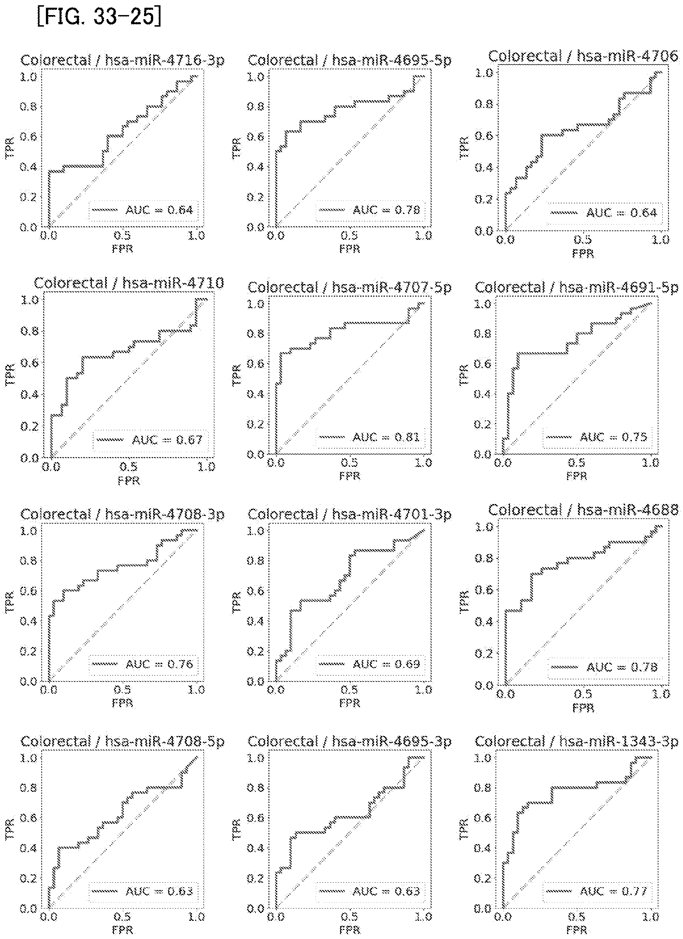

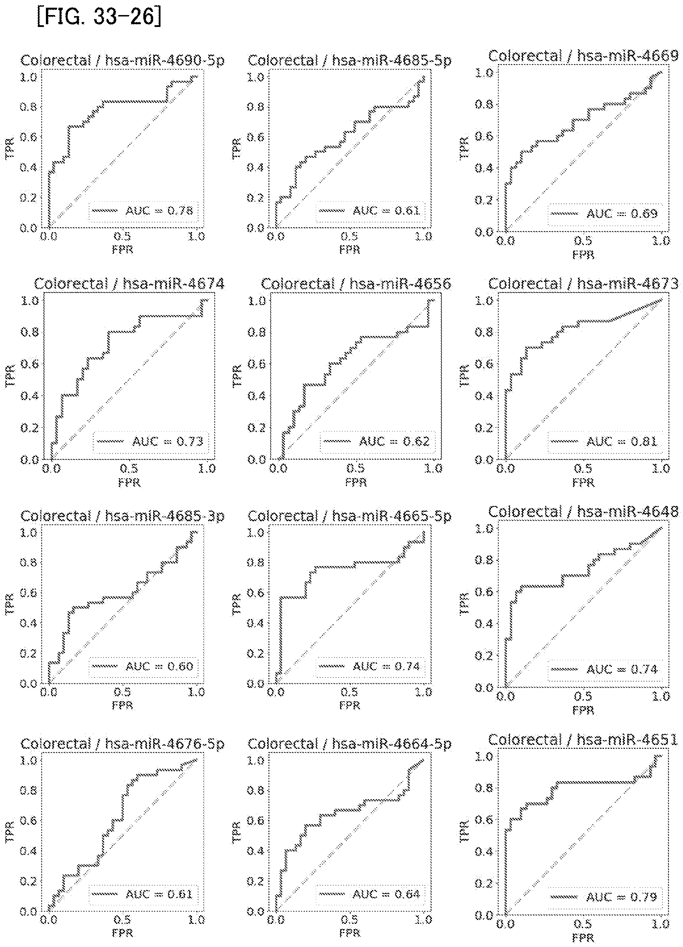

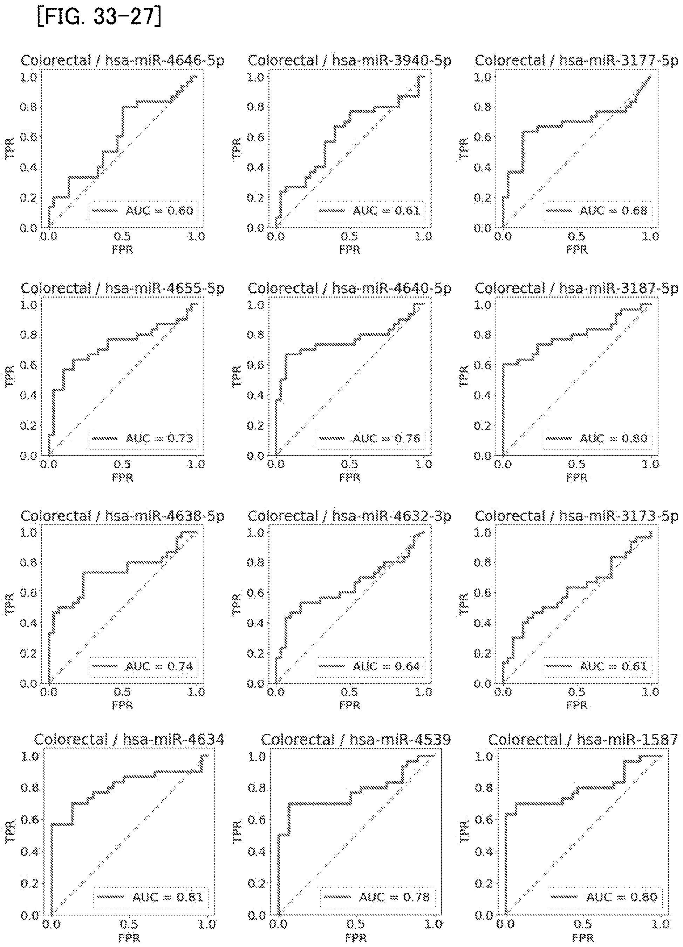

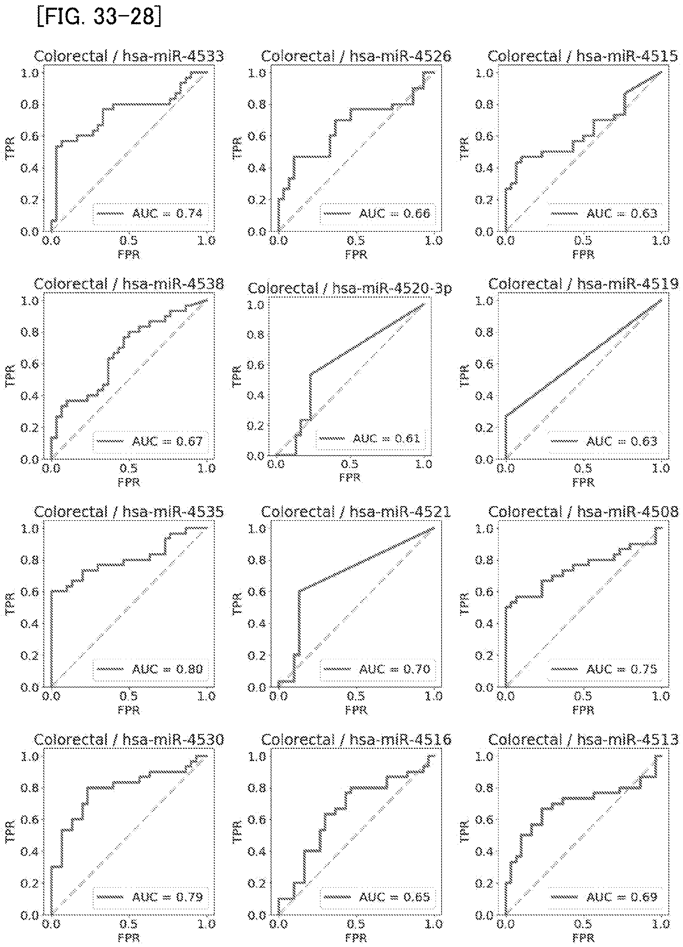

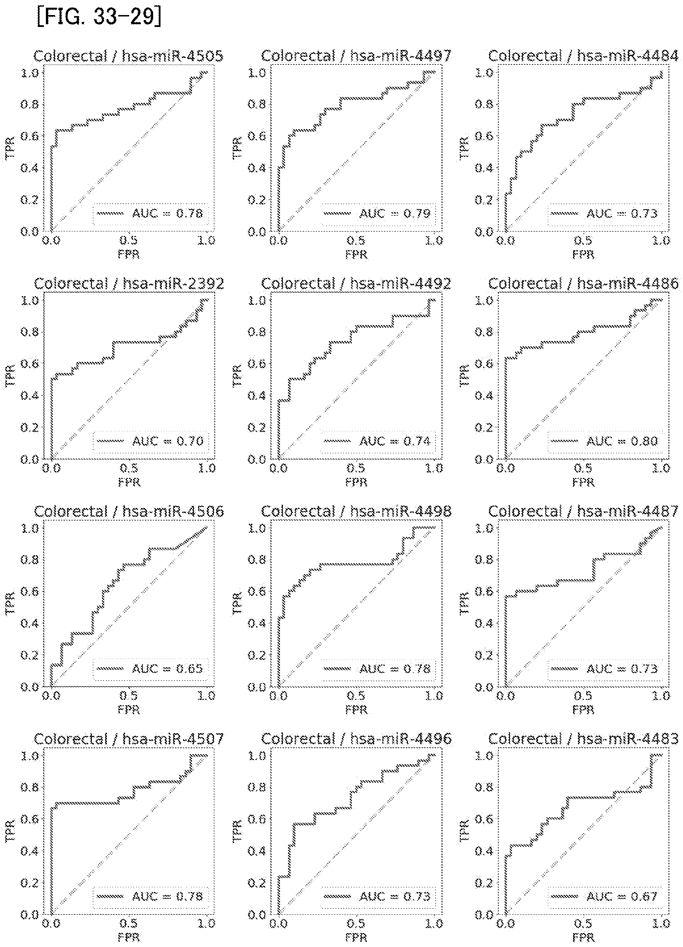

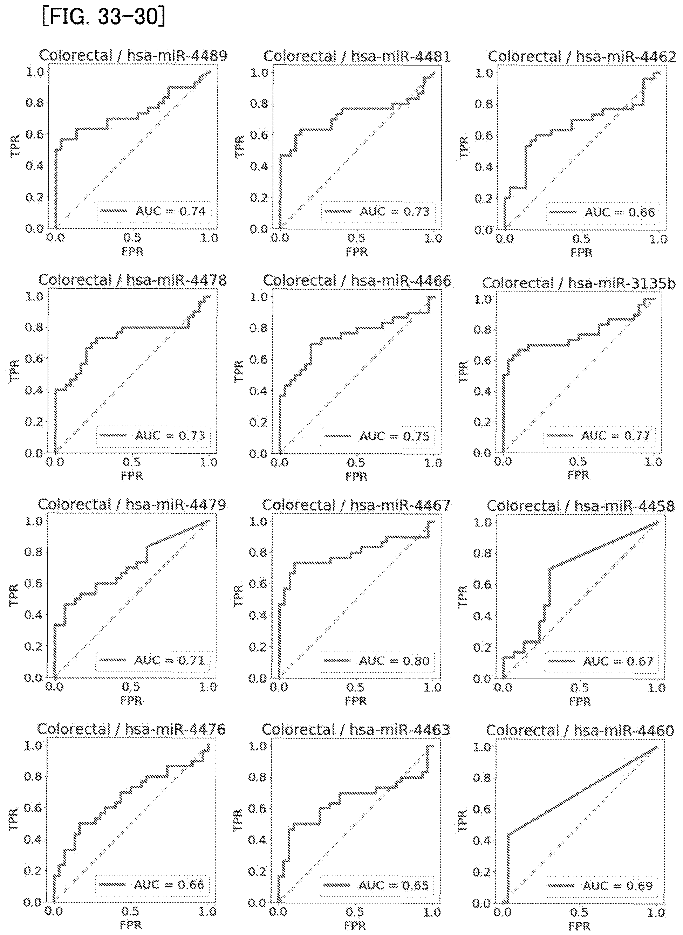

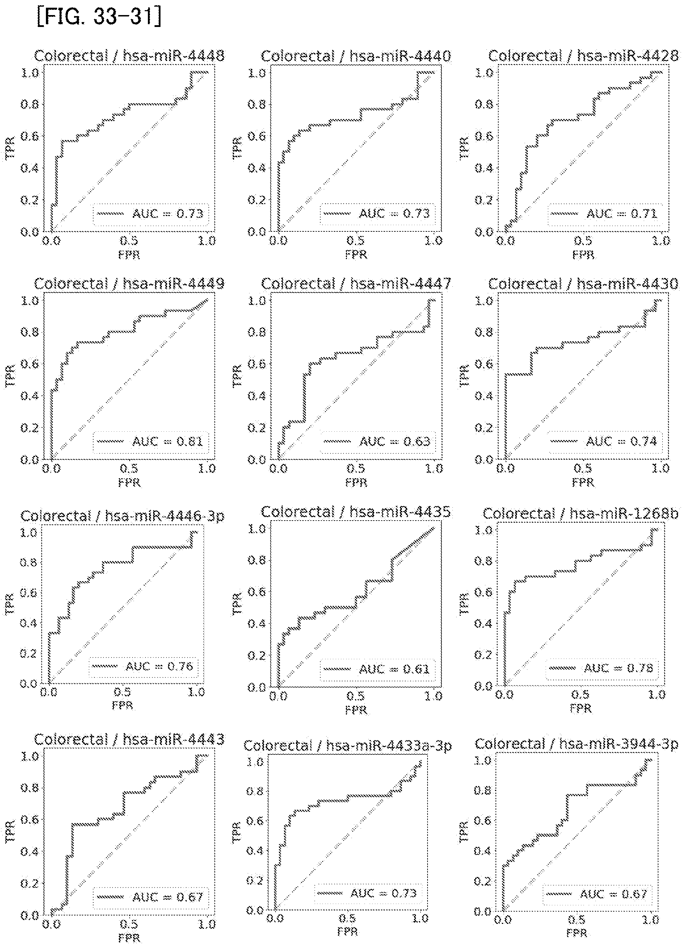

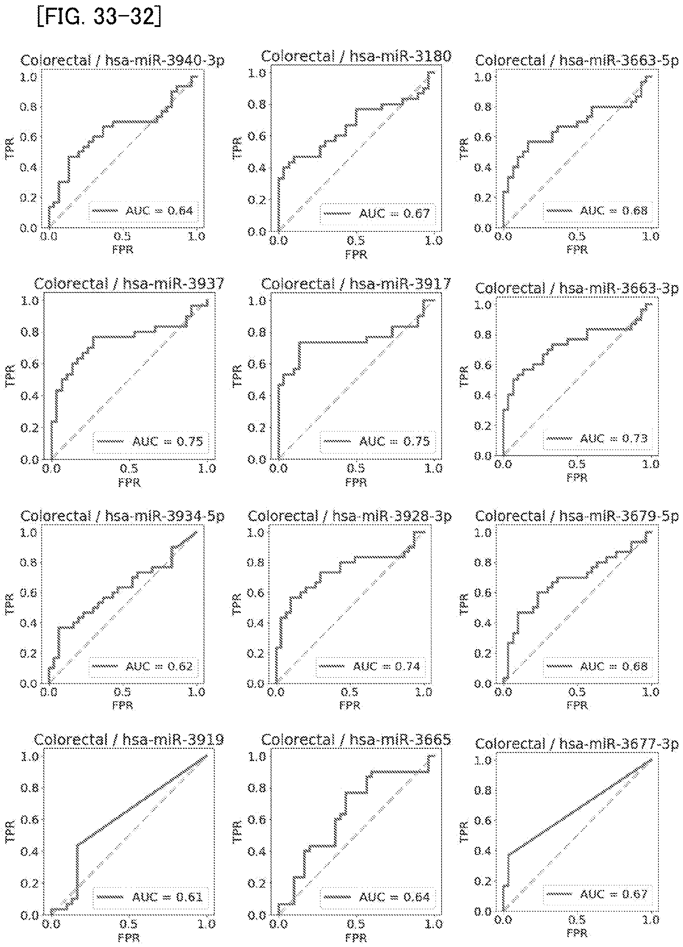

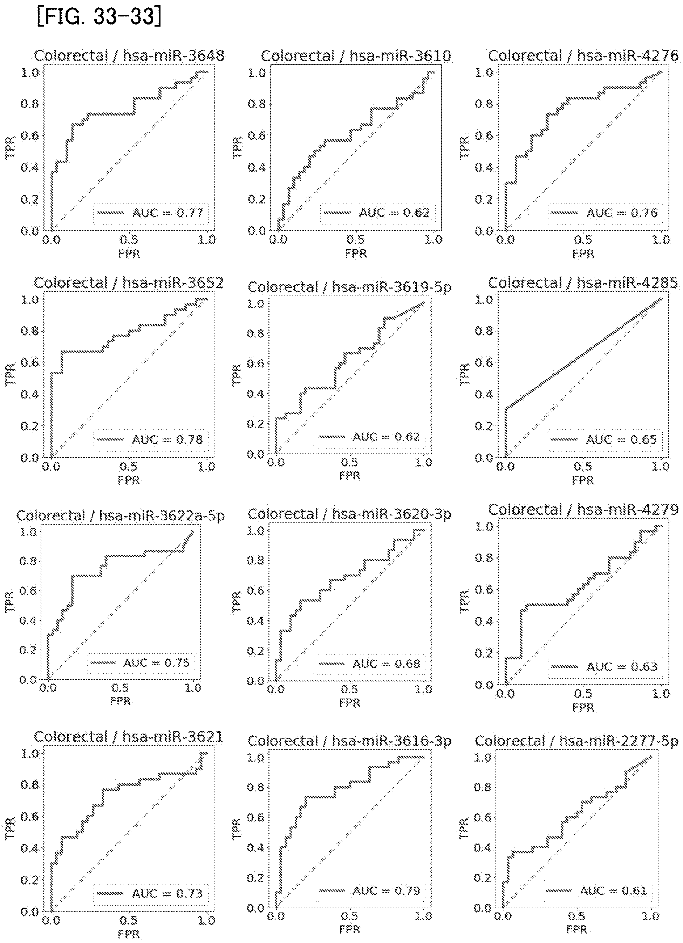

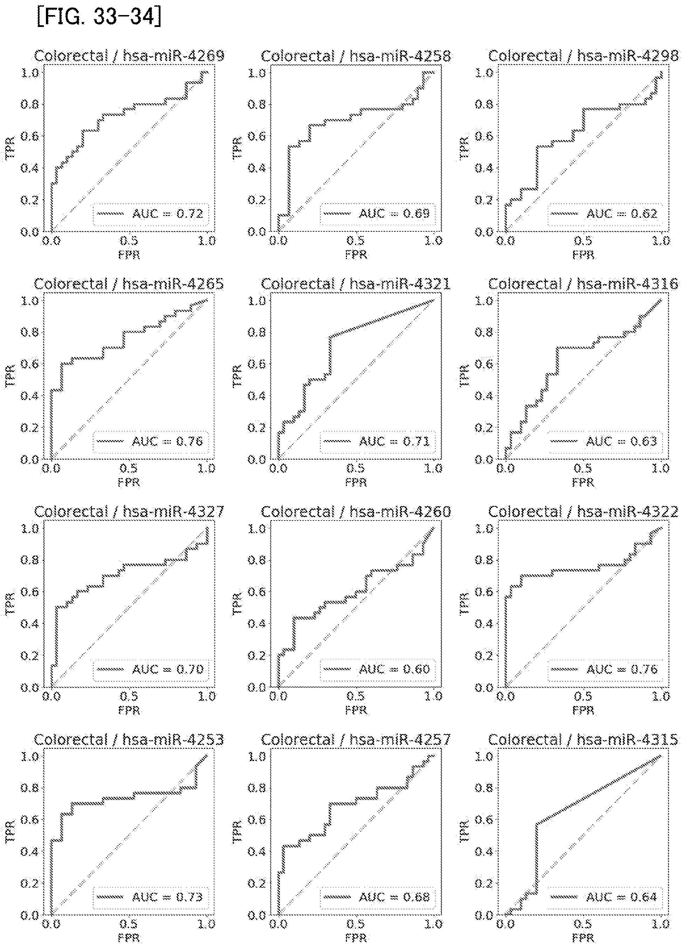

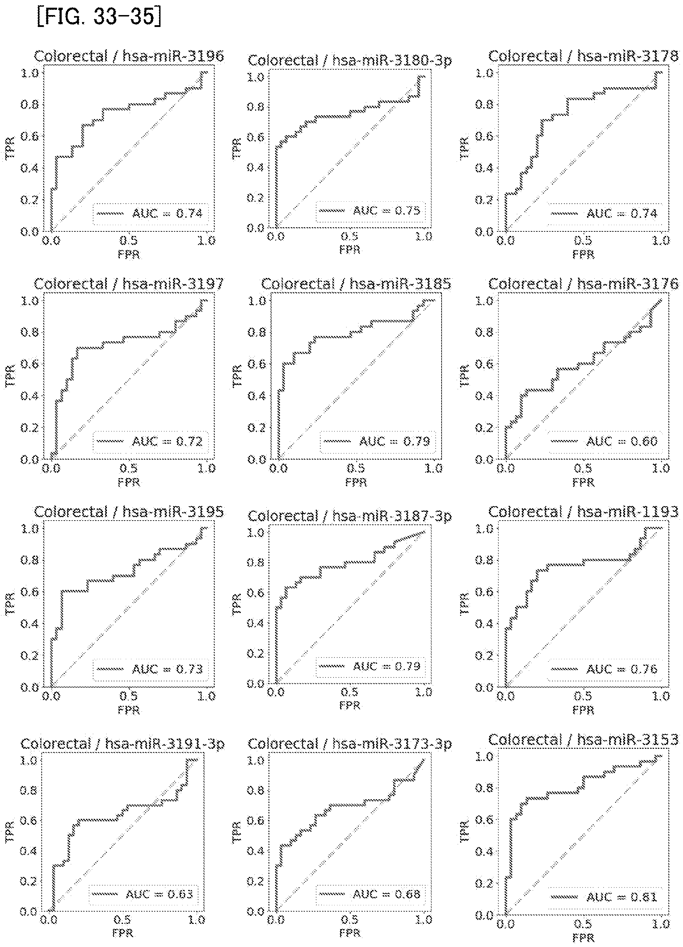

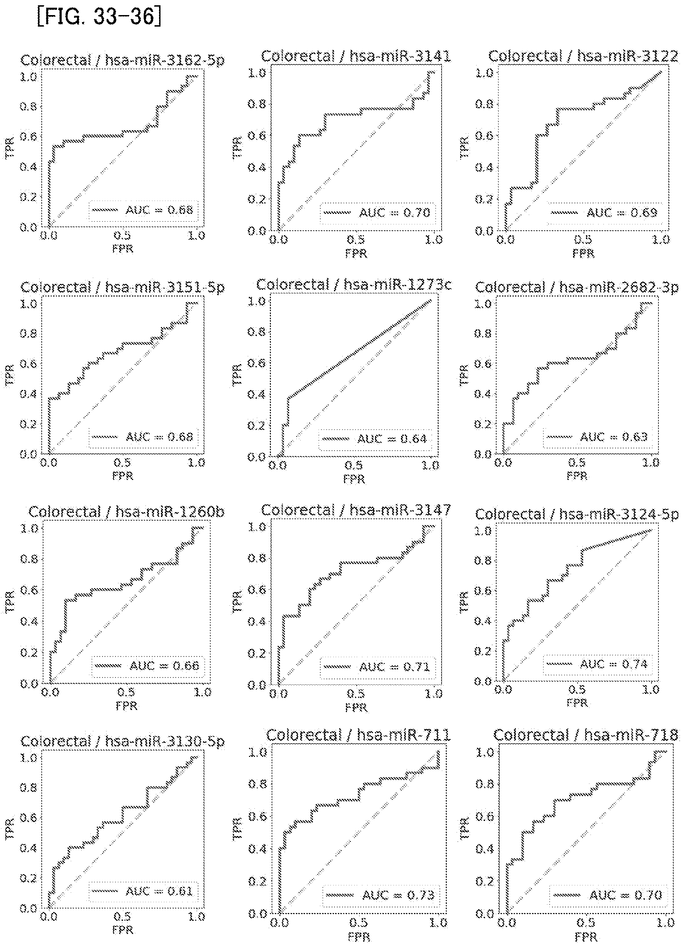

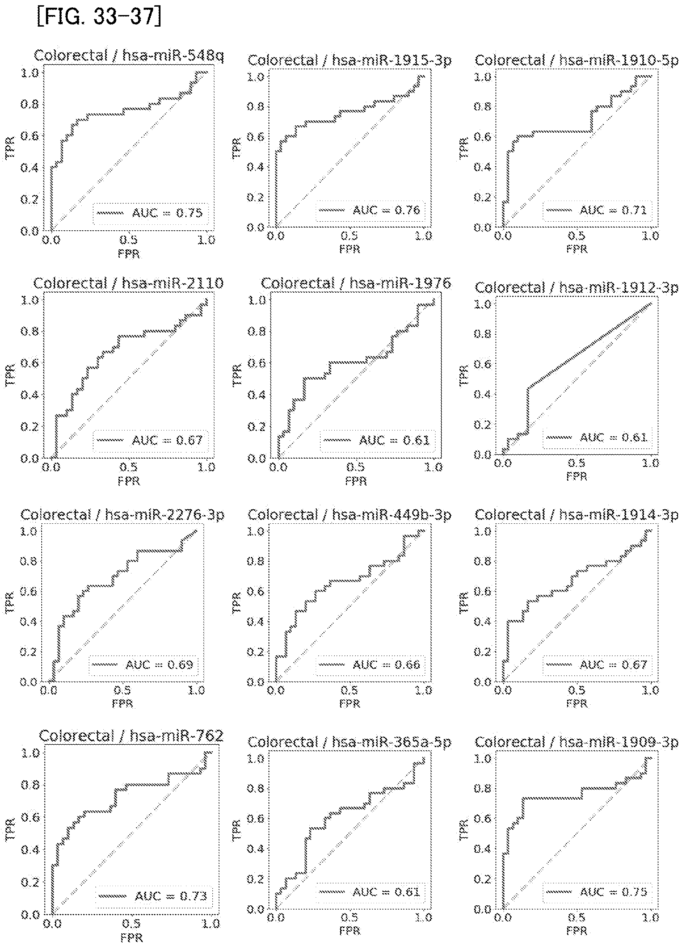

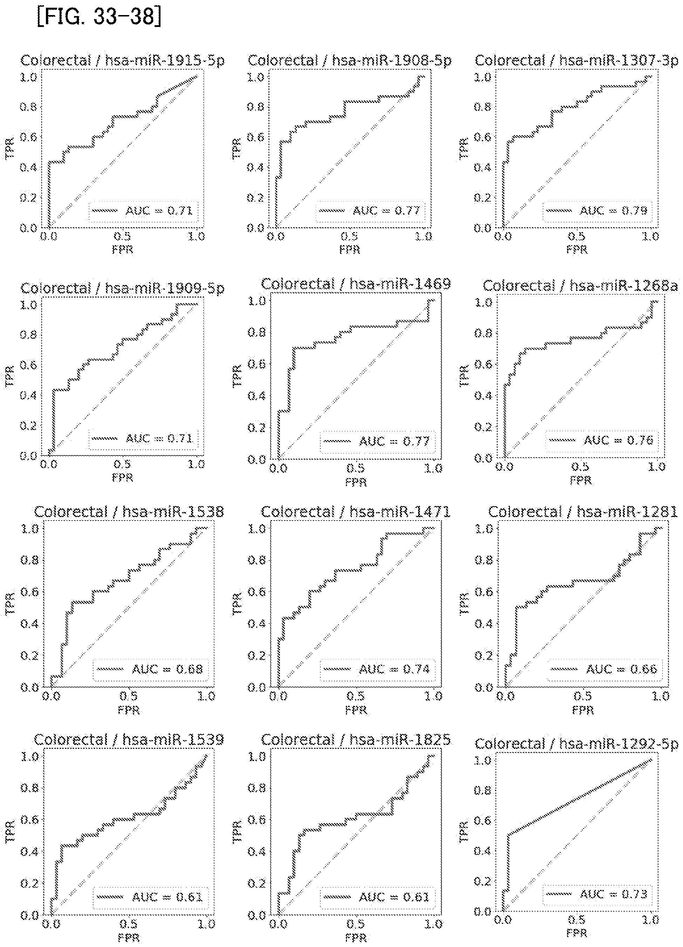

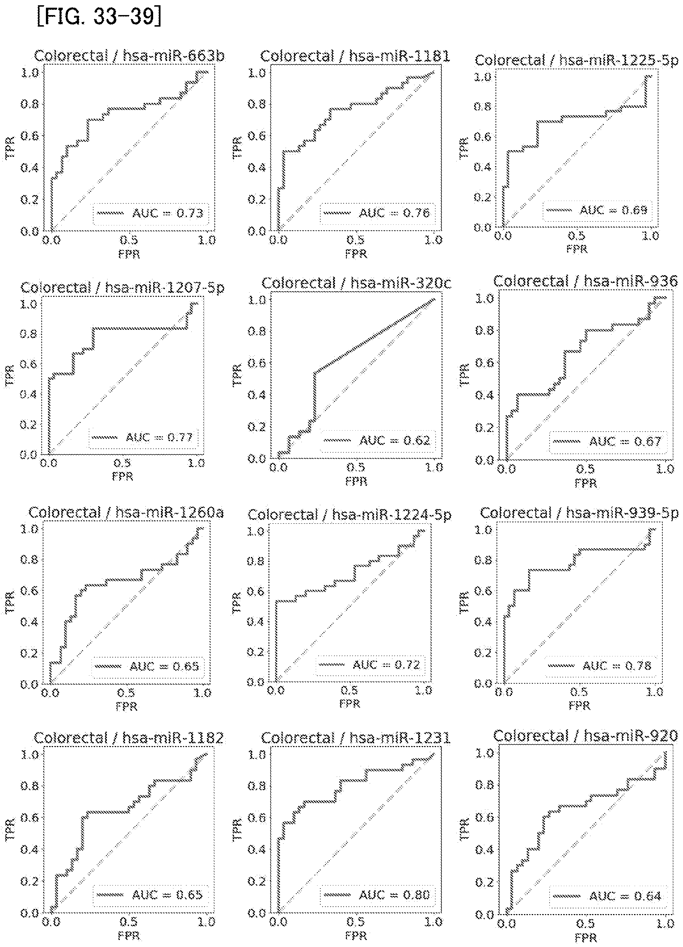

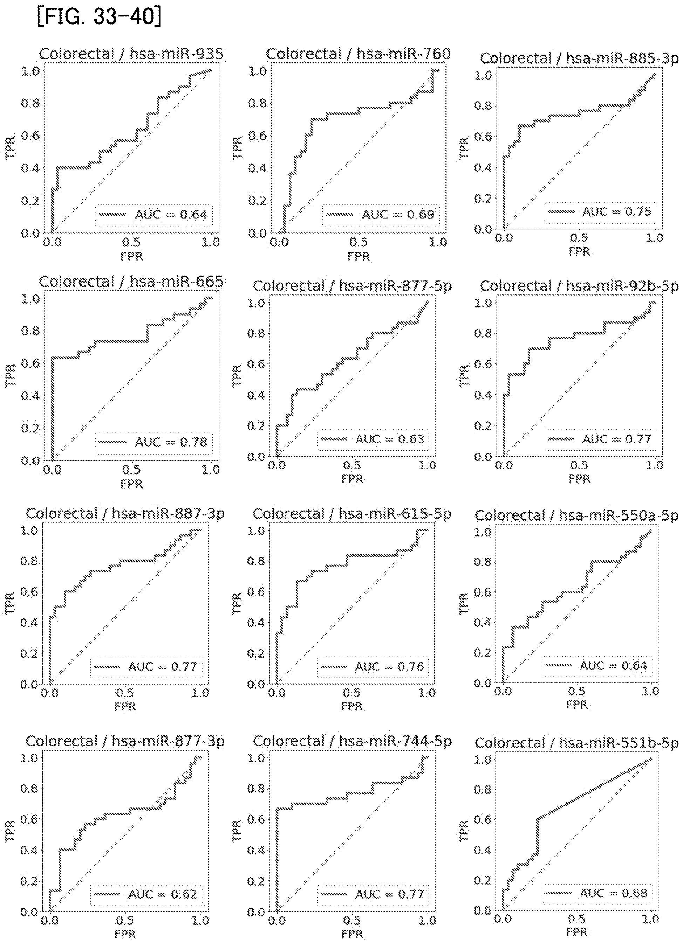

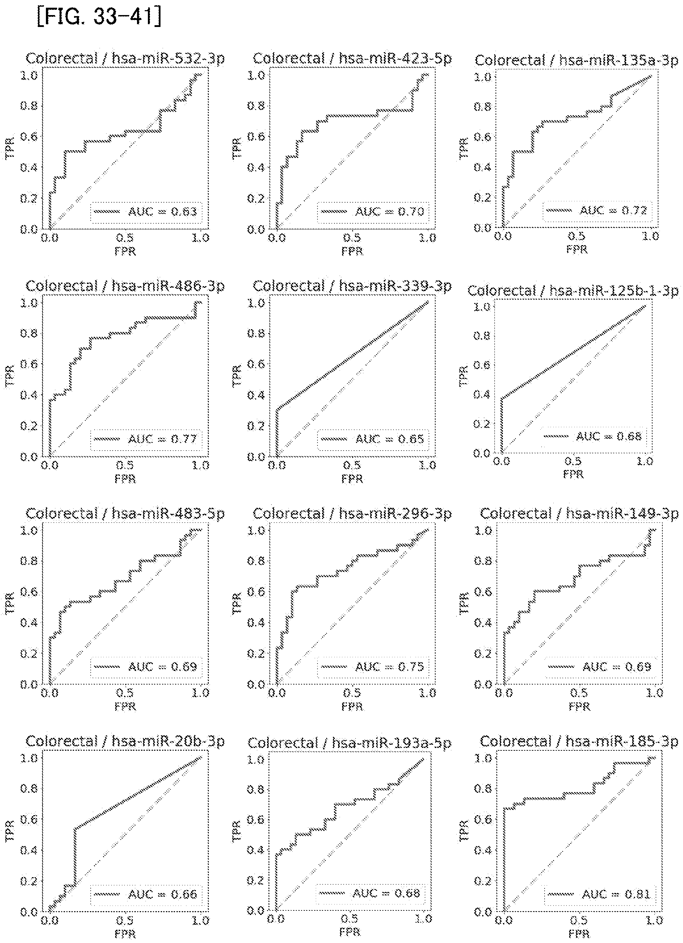

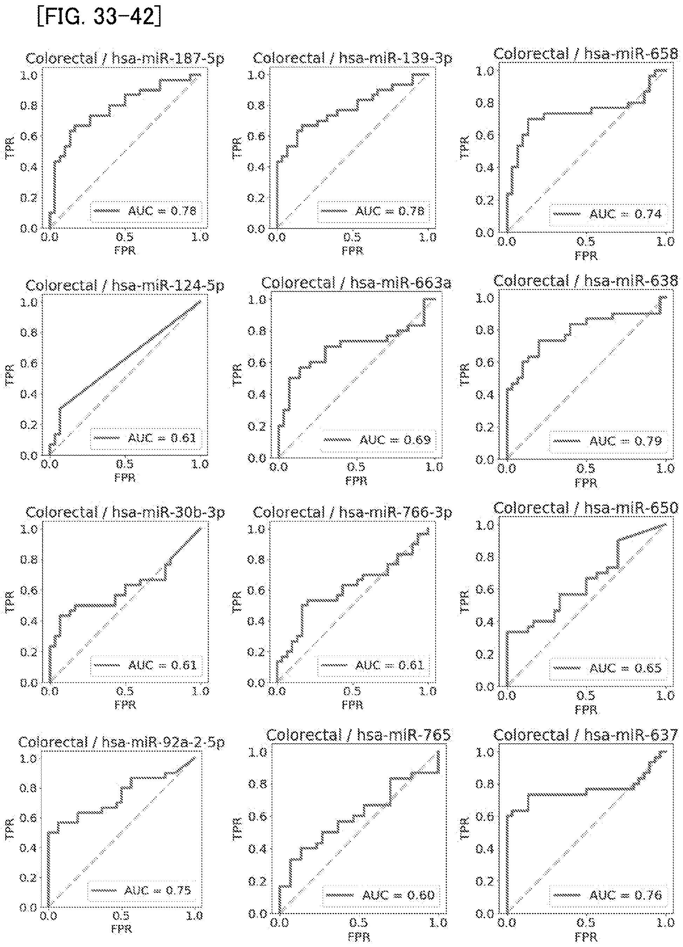

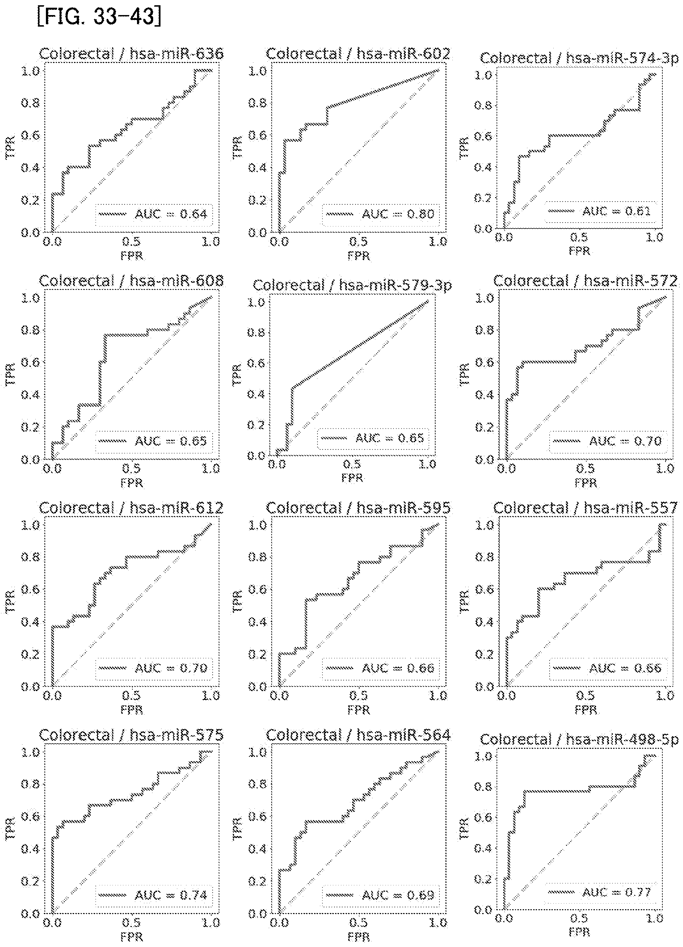

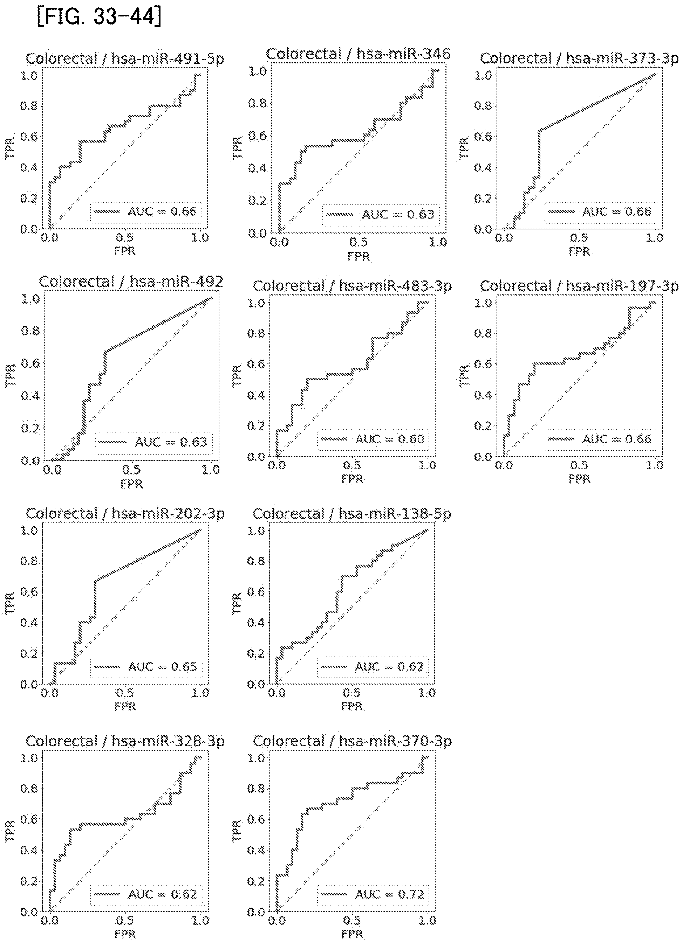

FIGS. 33-1 to 33-44 show ROCs curves for predicting colorectal cancer in humans using a single miRNA.

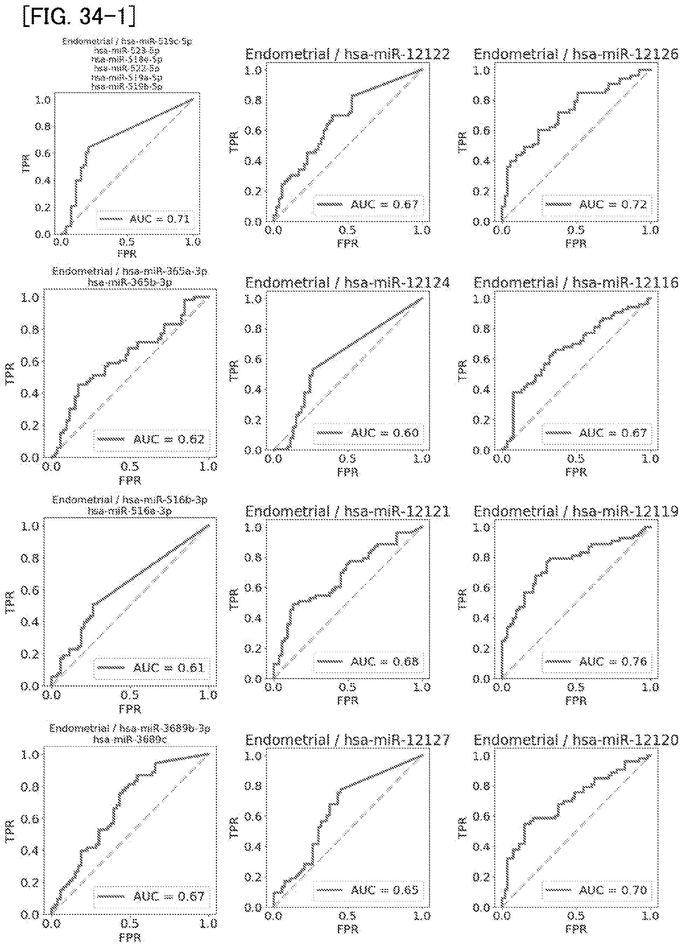

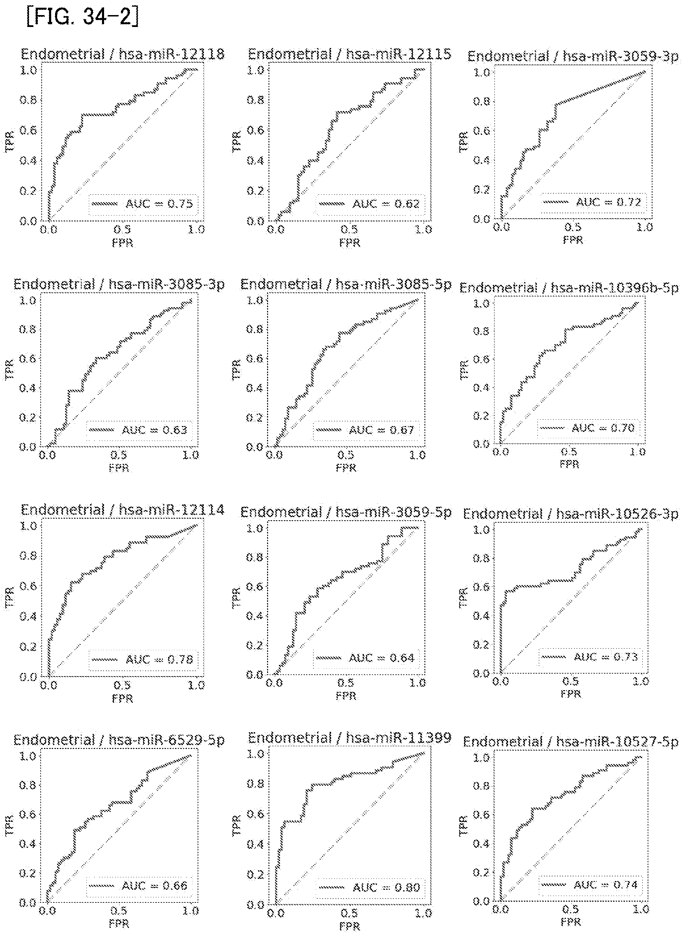

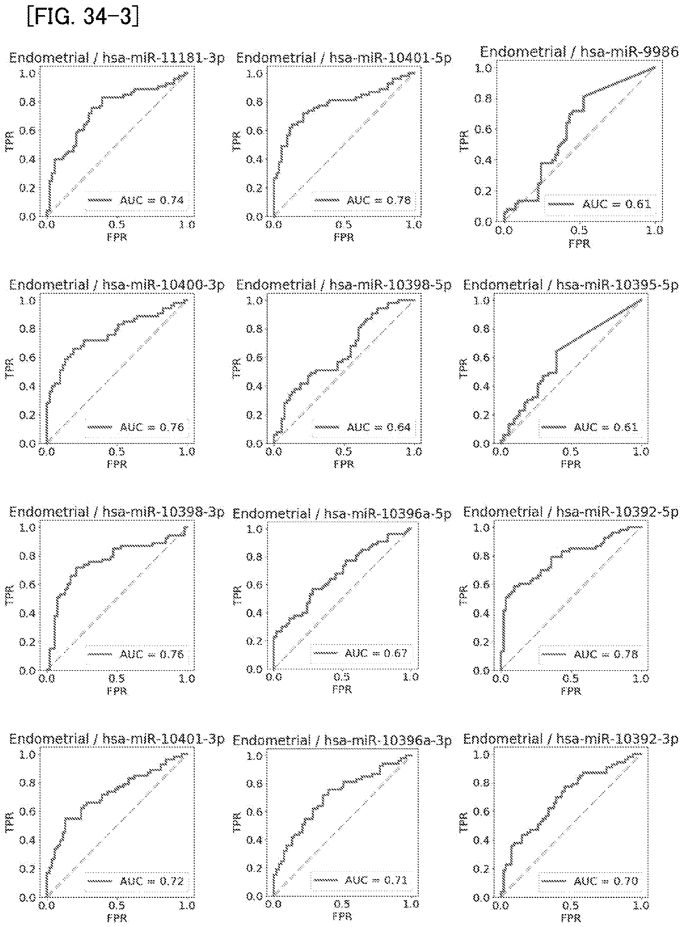

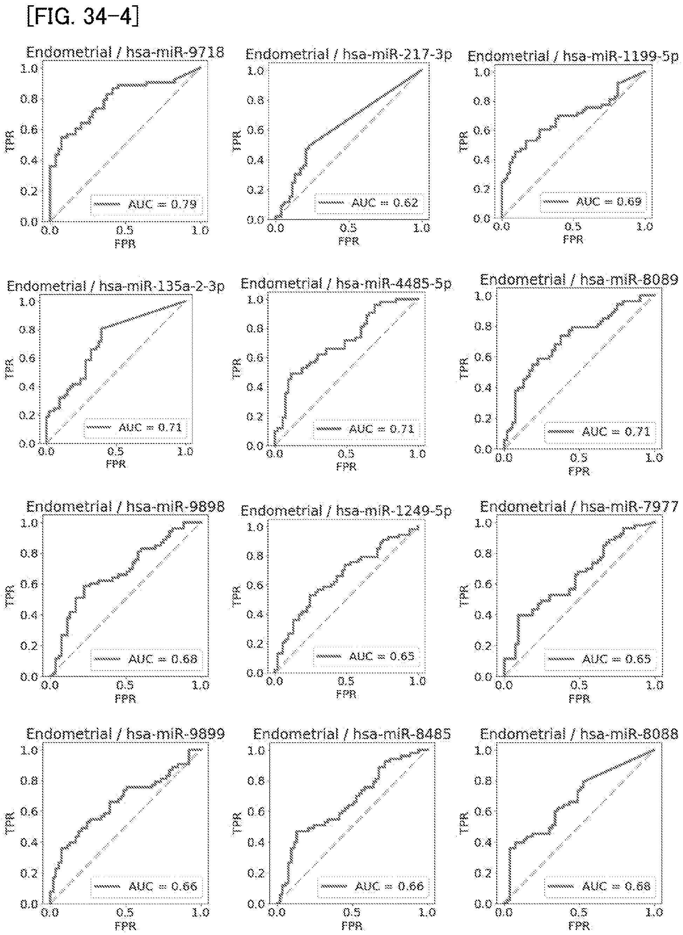

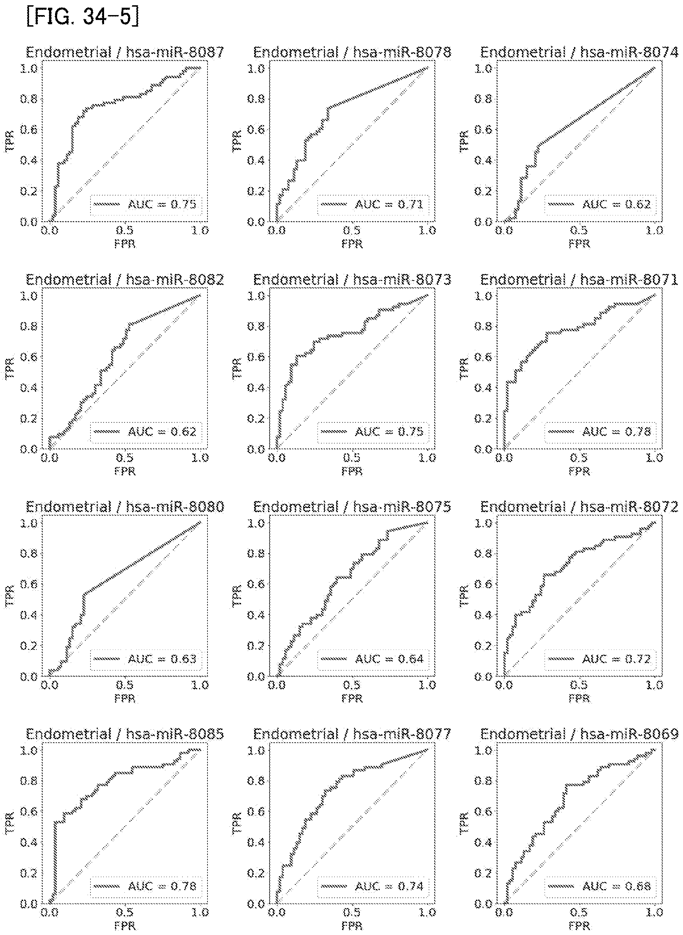

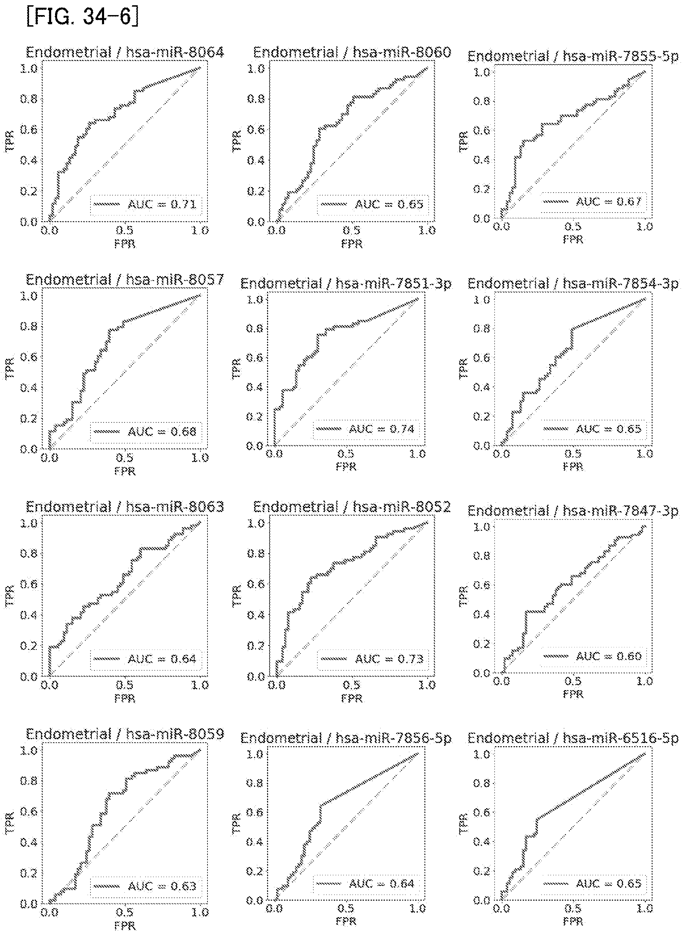

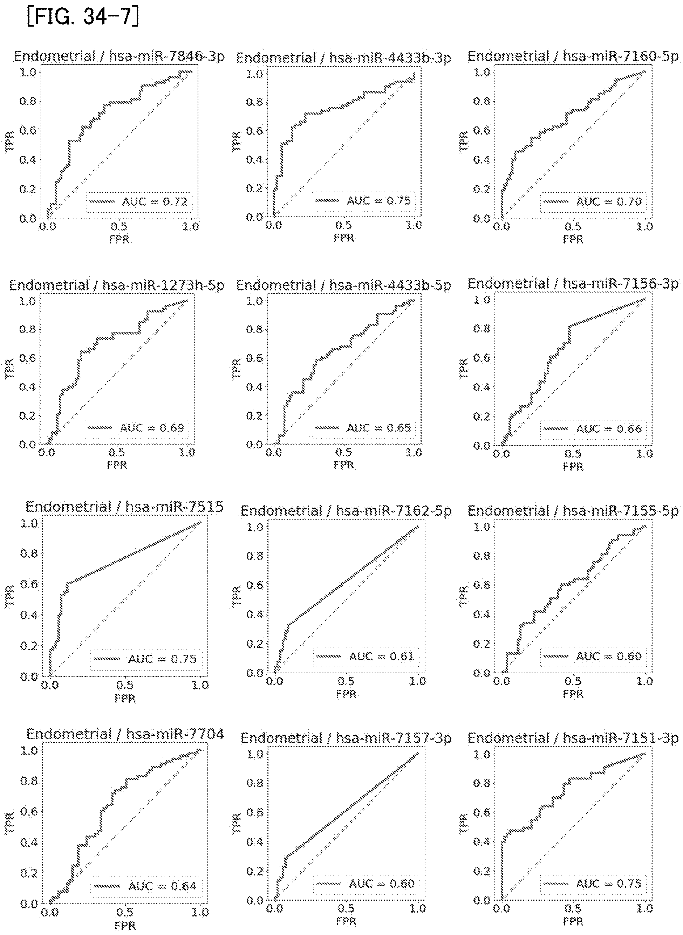

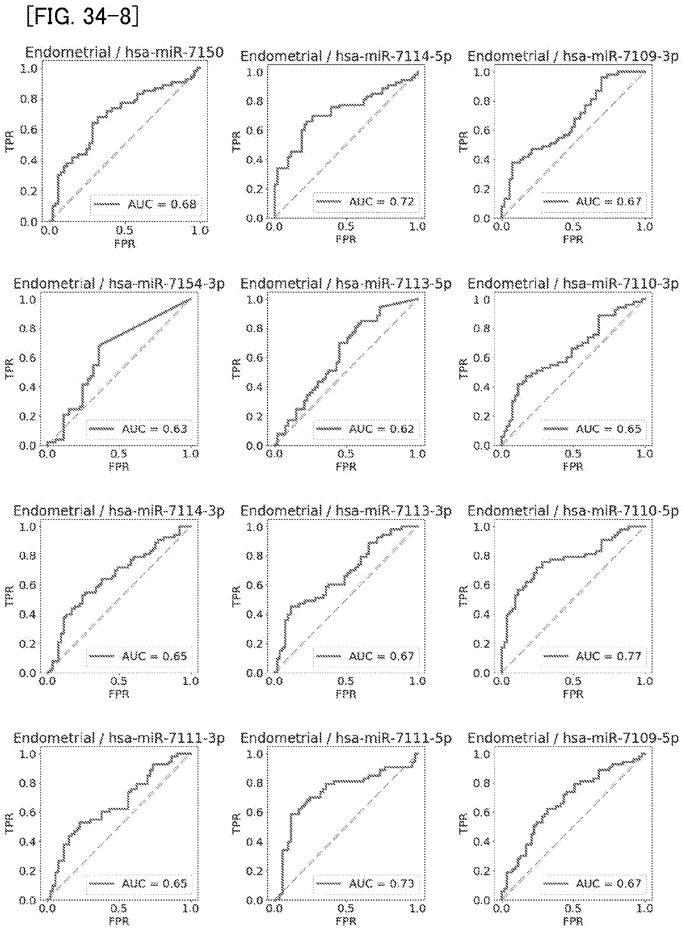

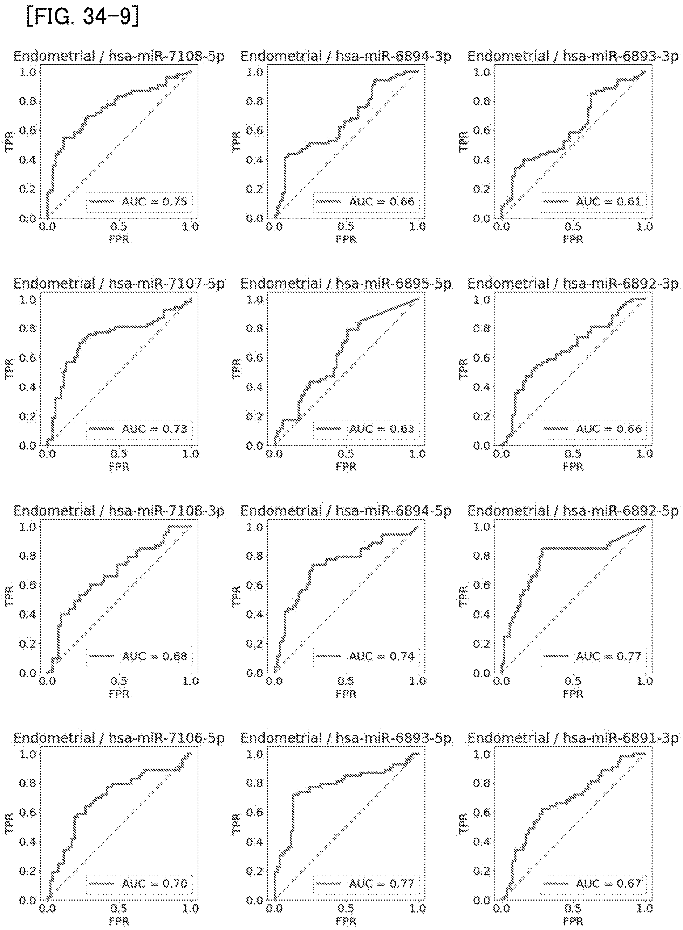

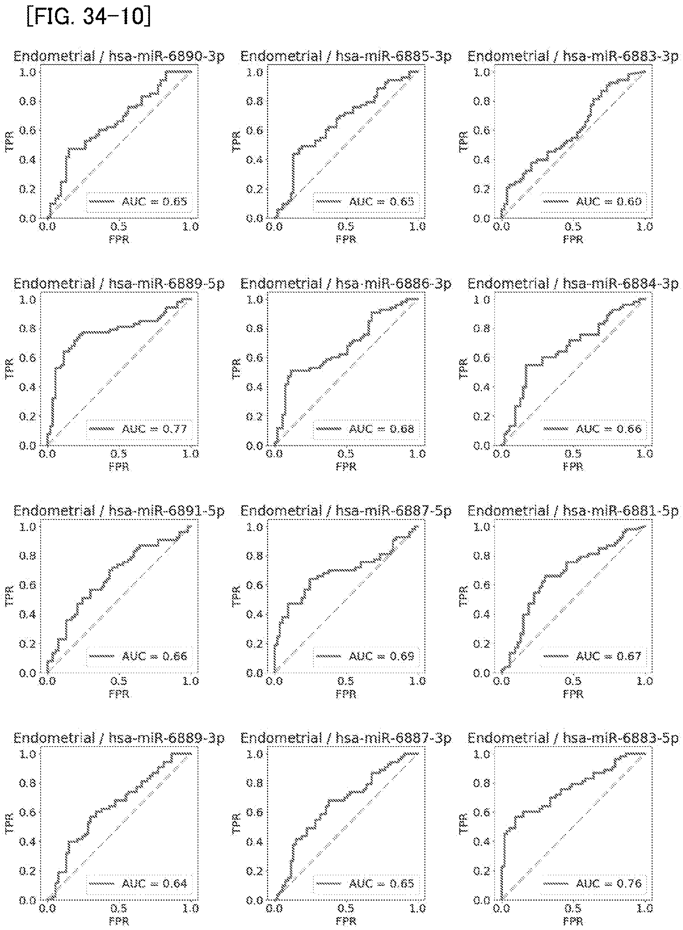

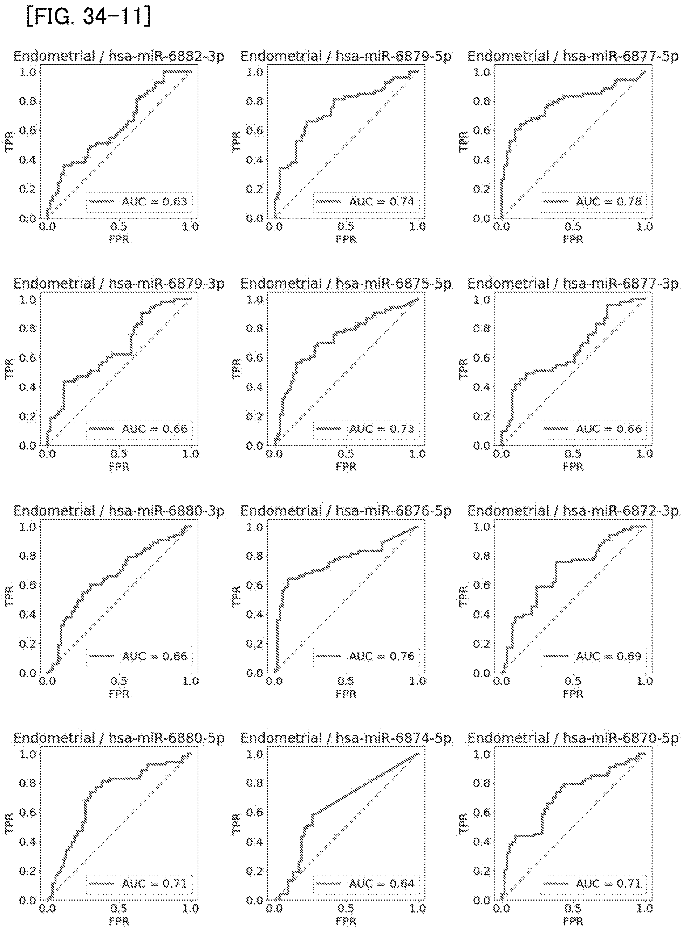

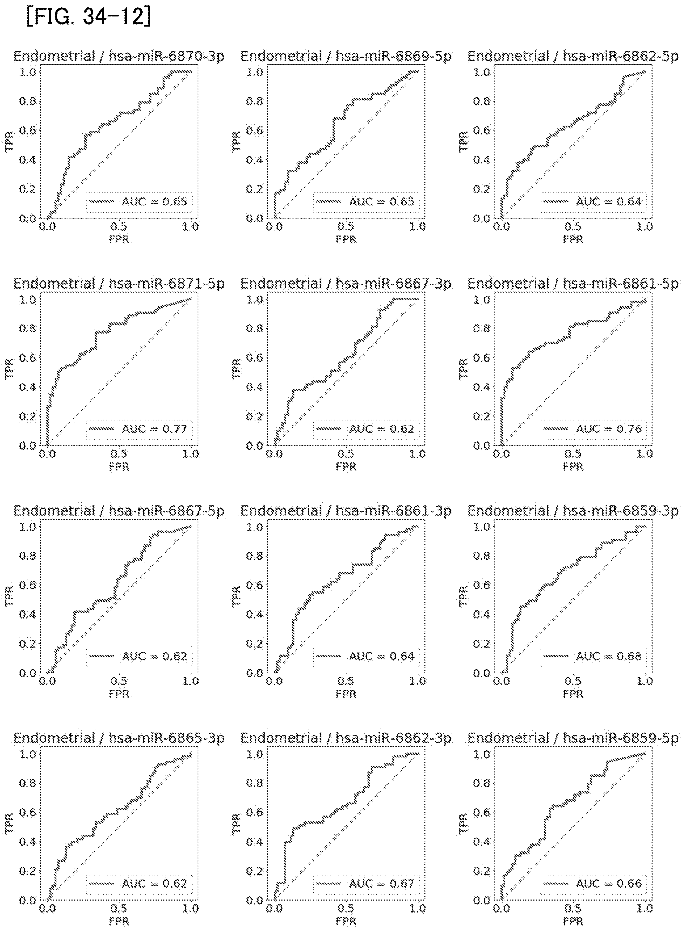

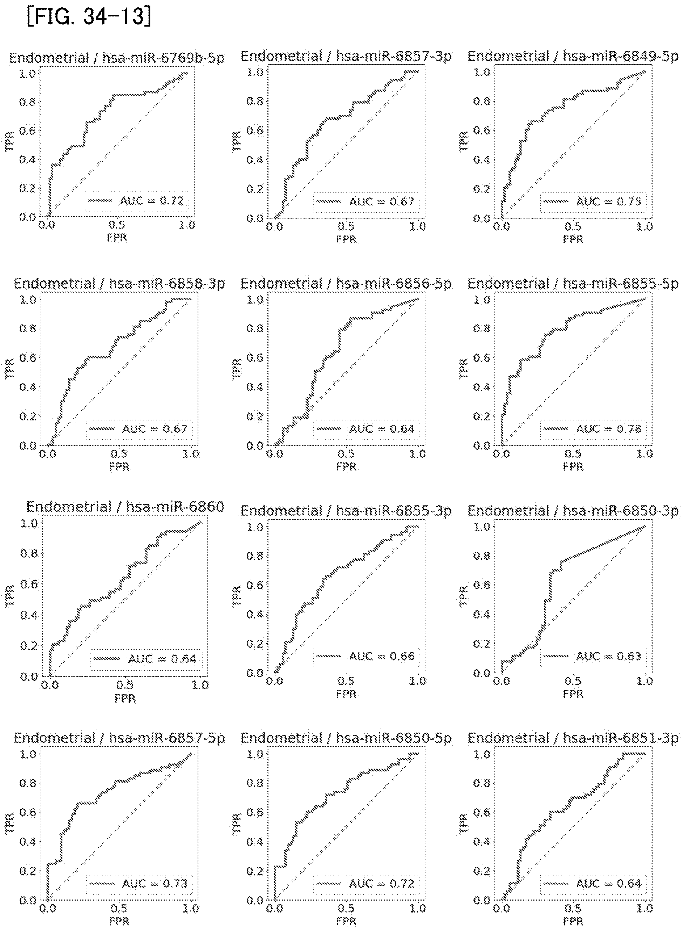

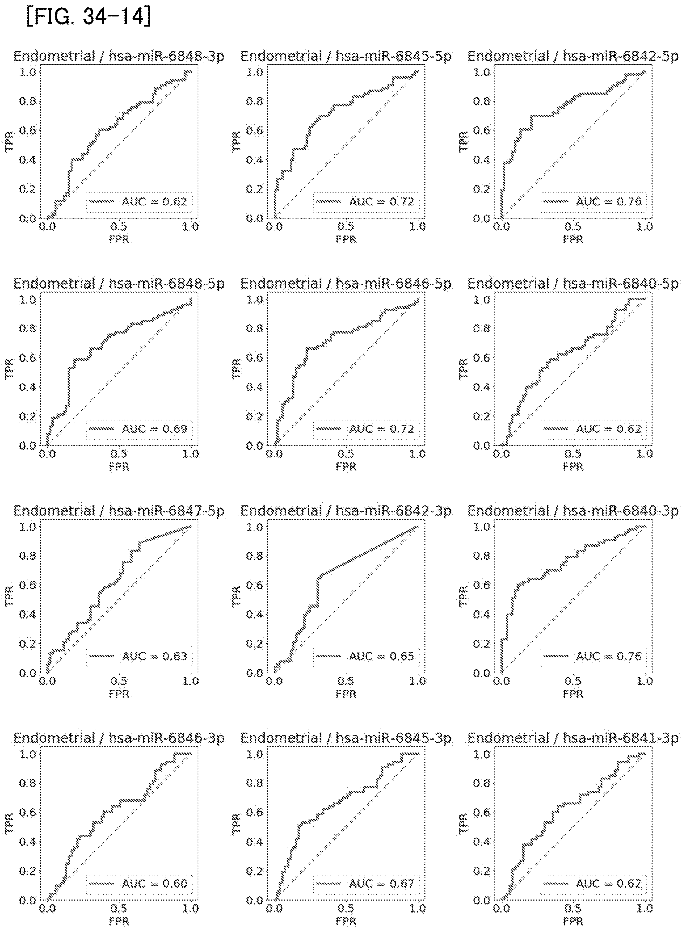

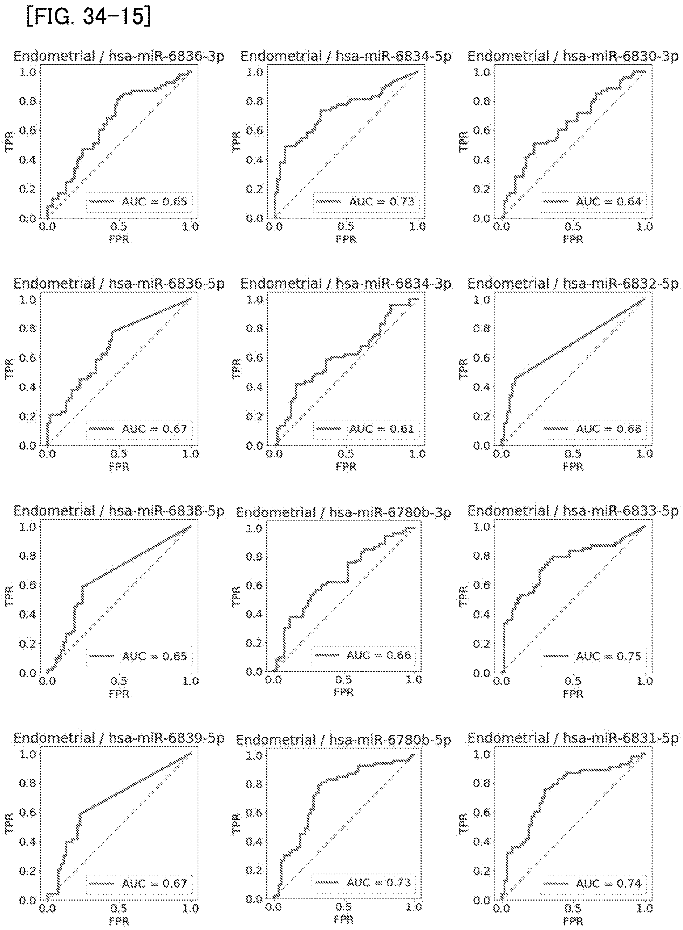

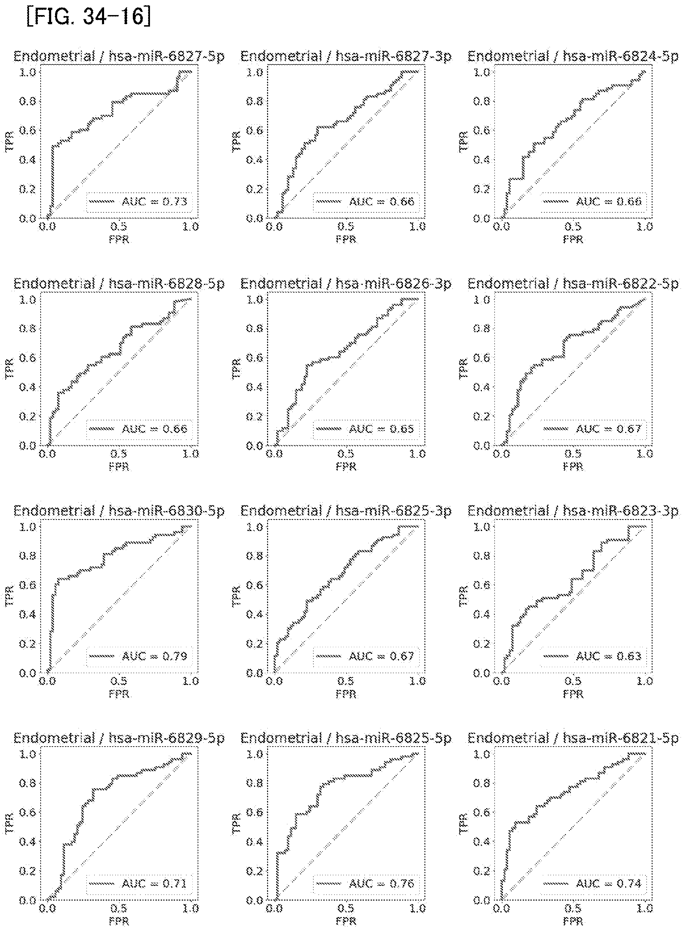

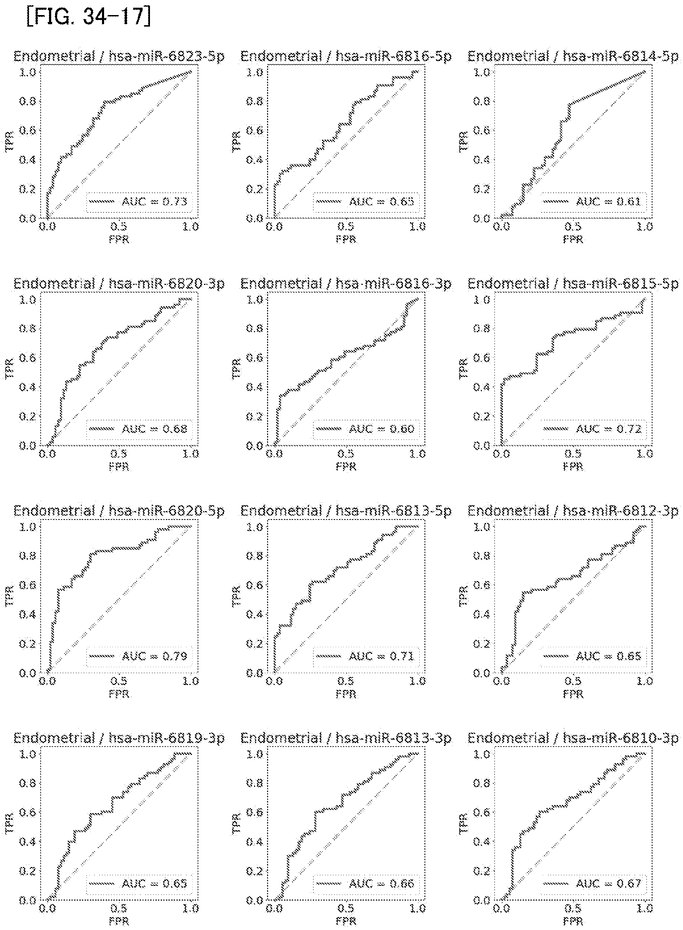

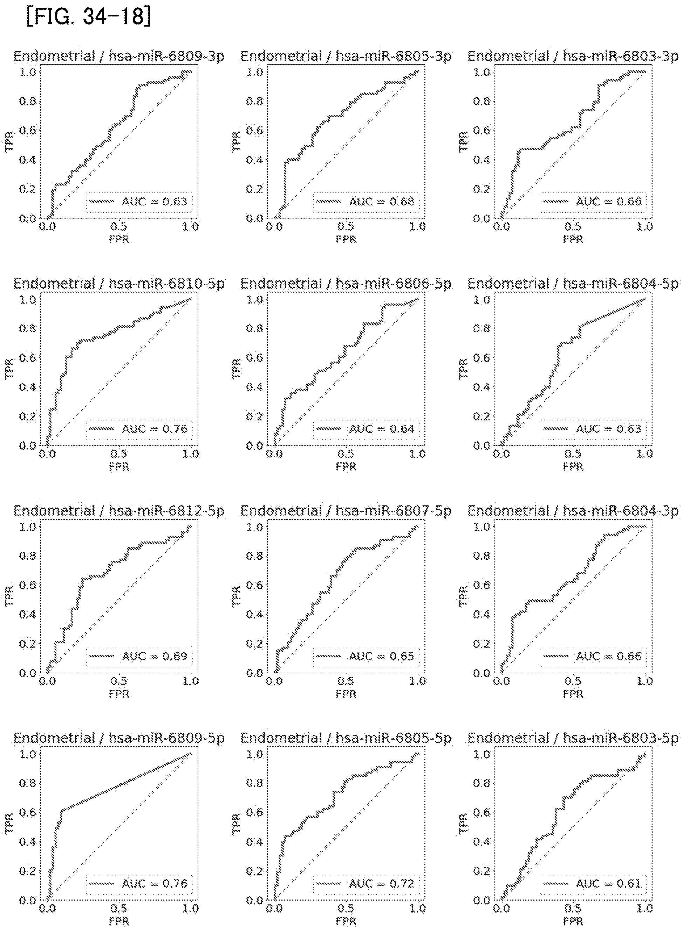

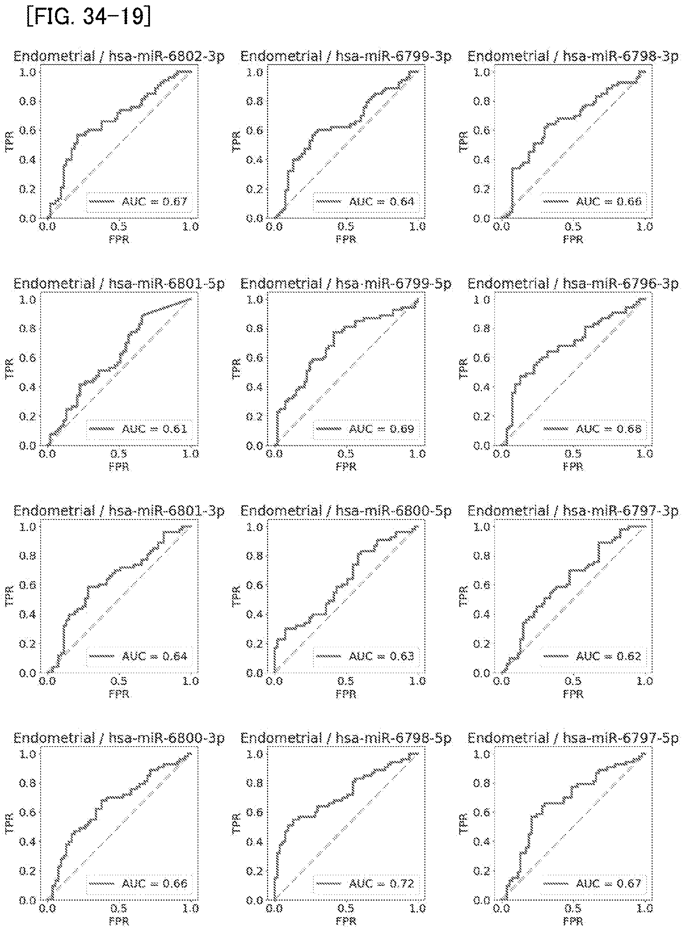

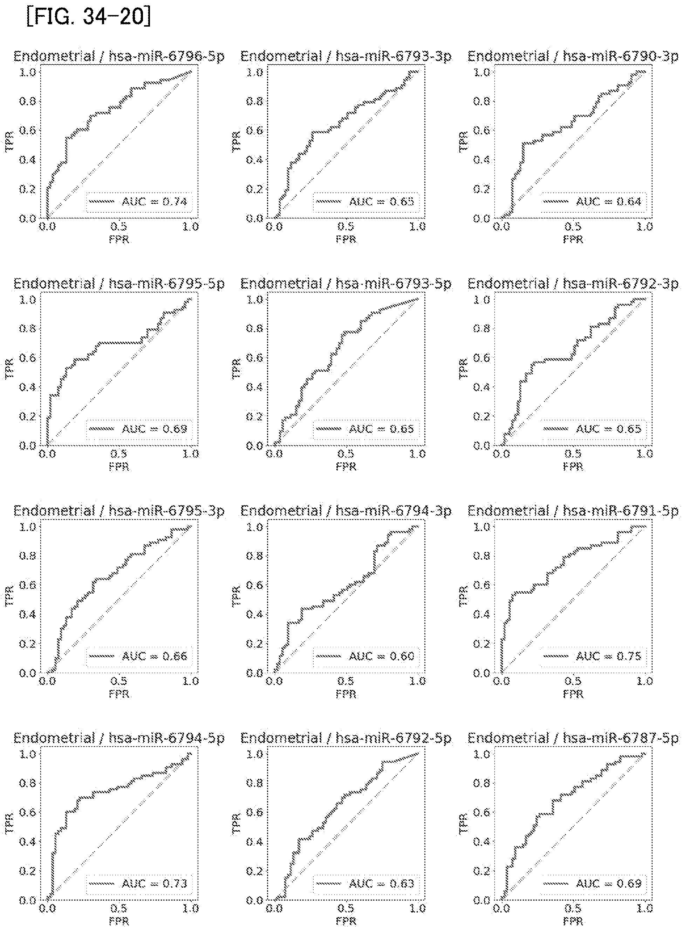

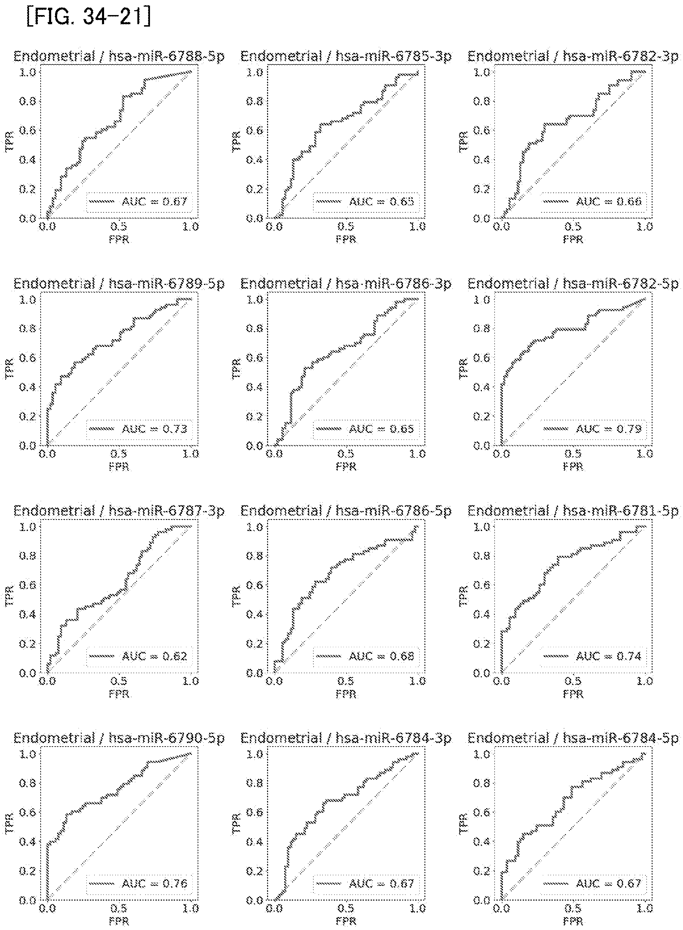

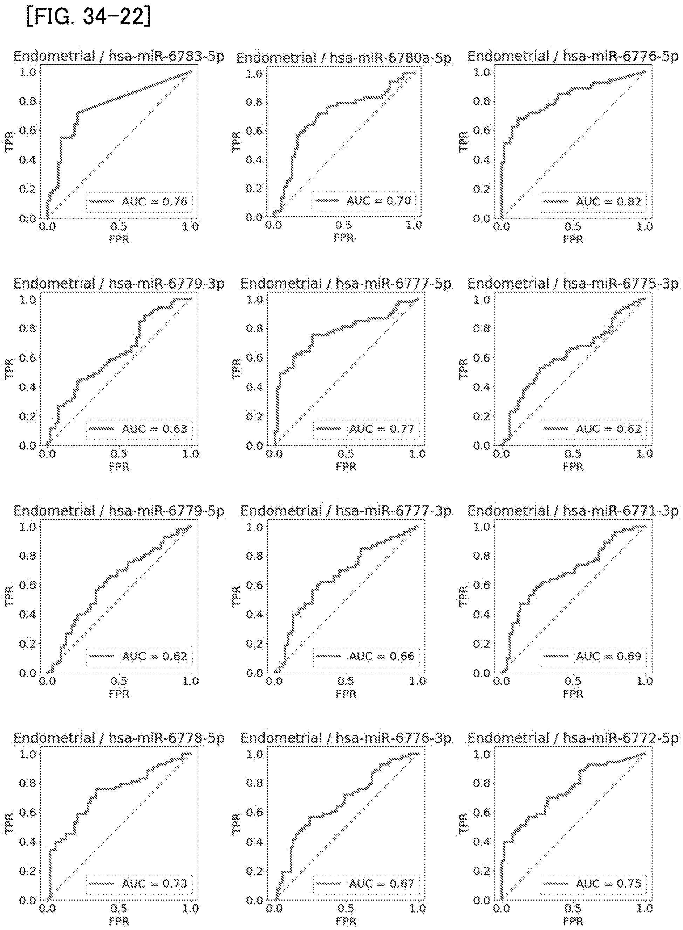

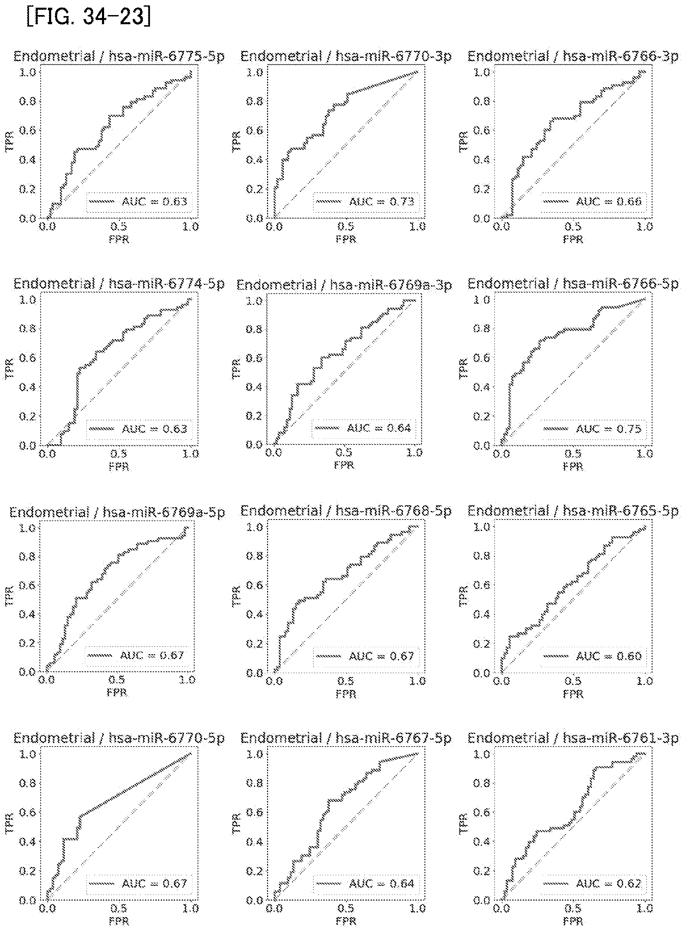

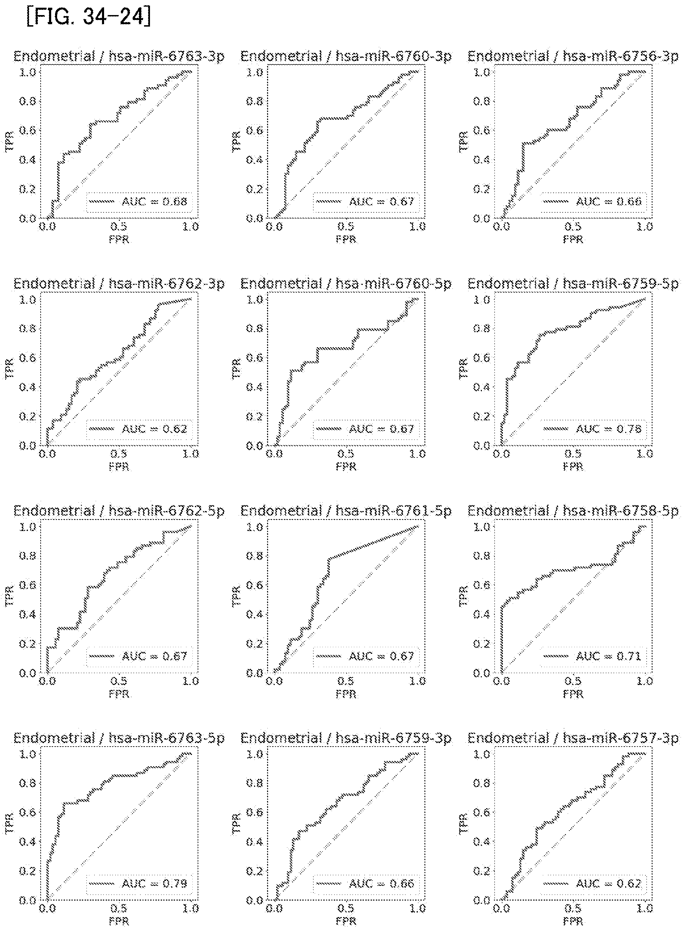

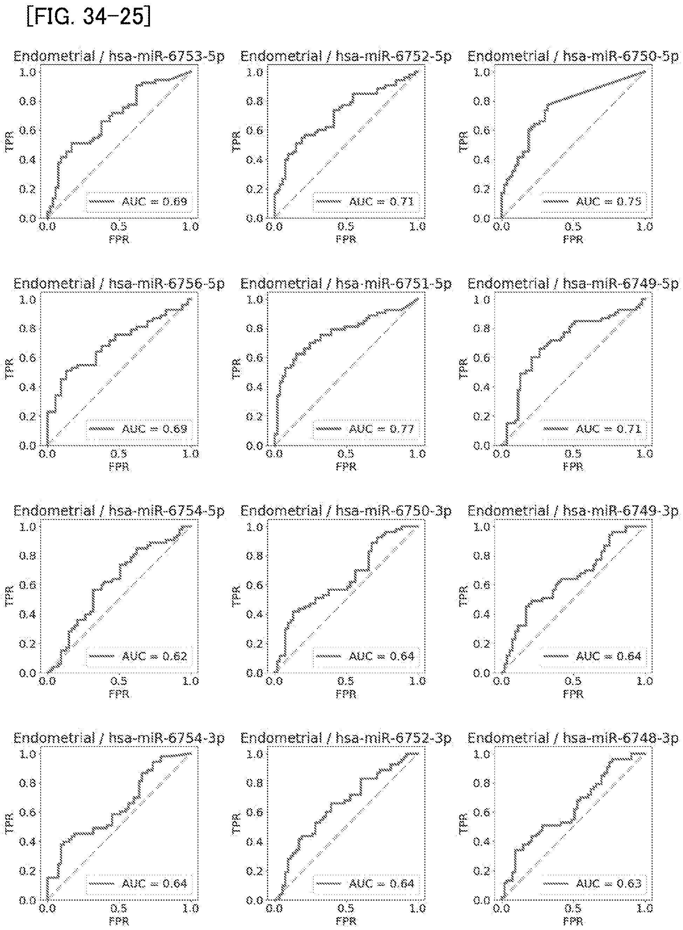

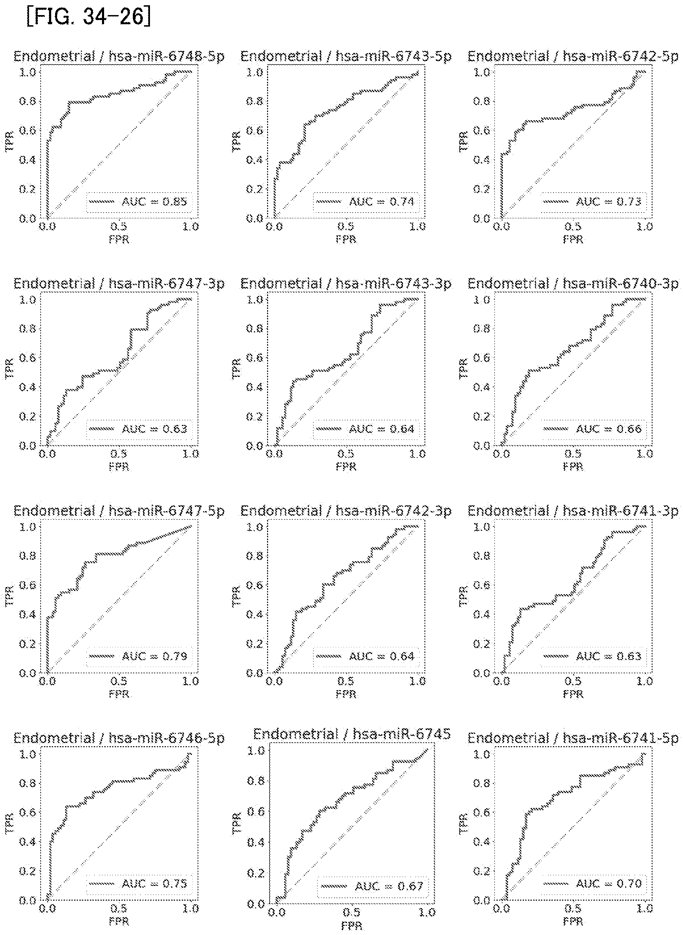

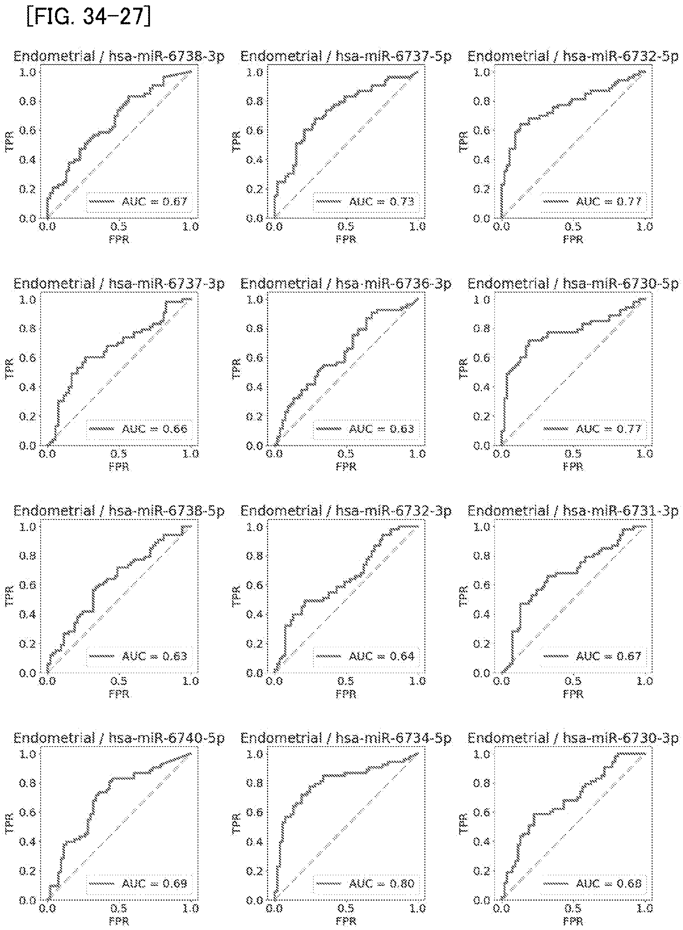

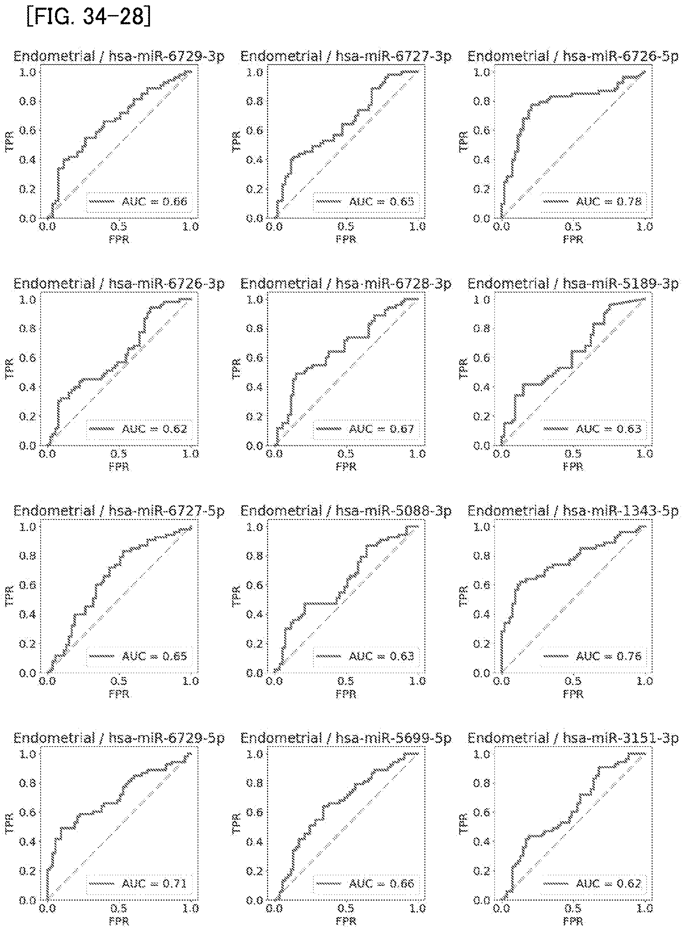

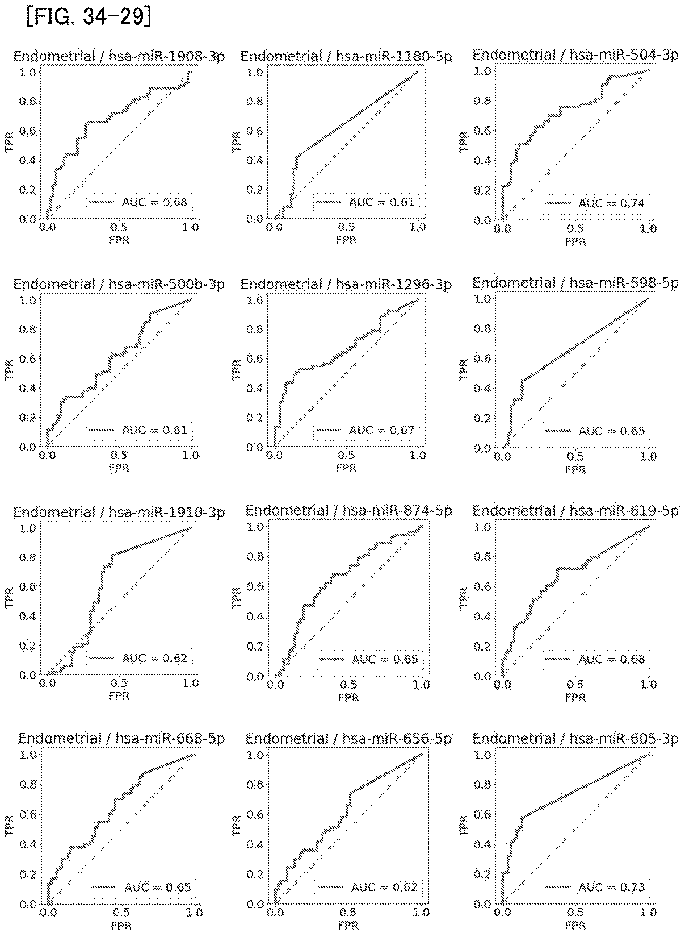

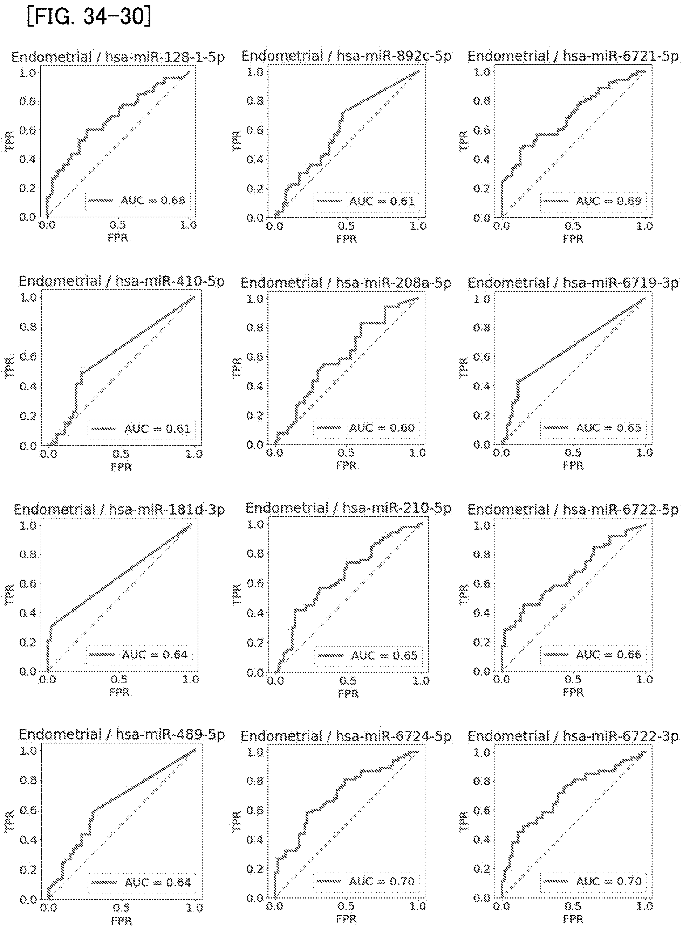

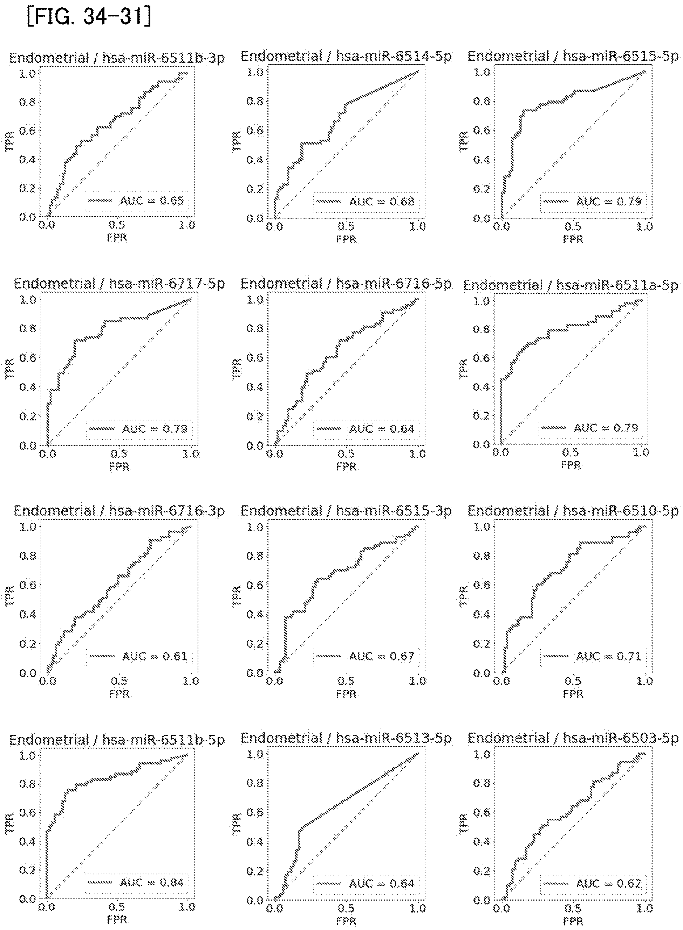

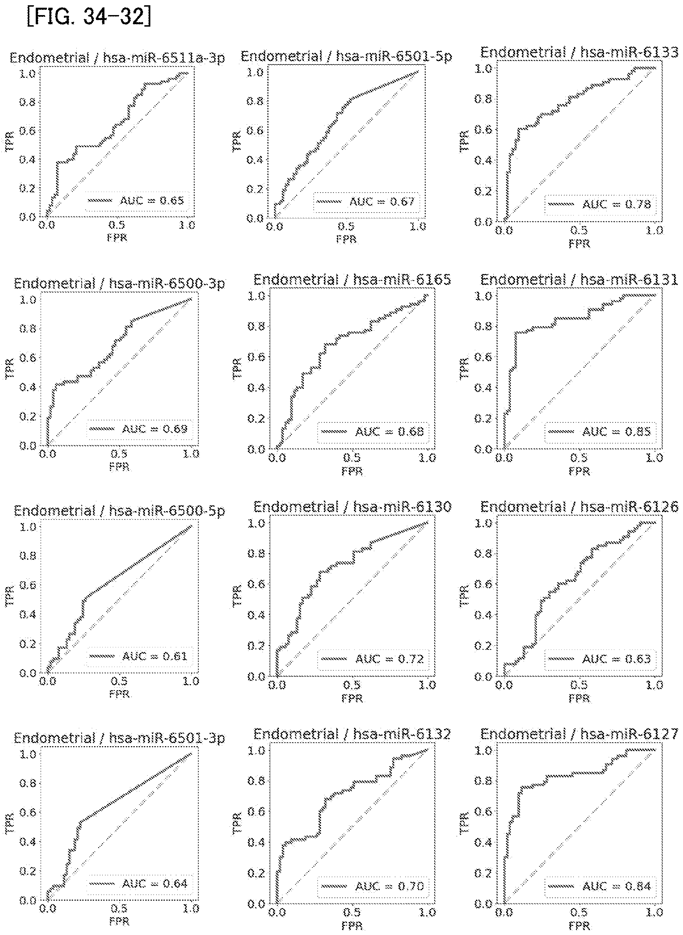

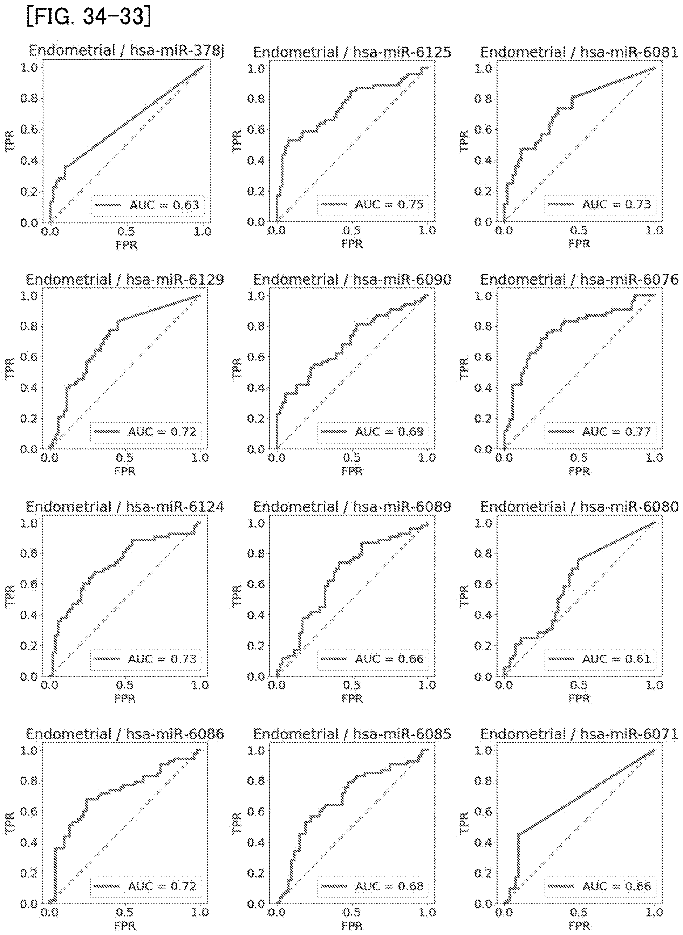

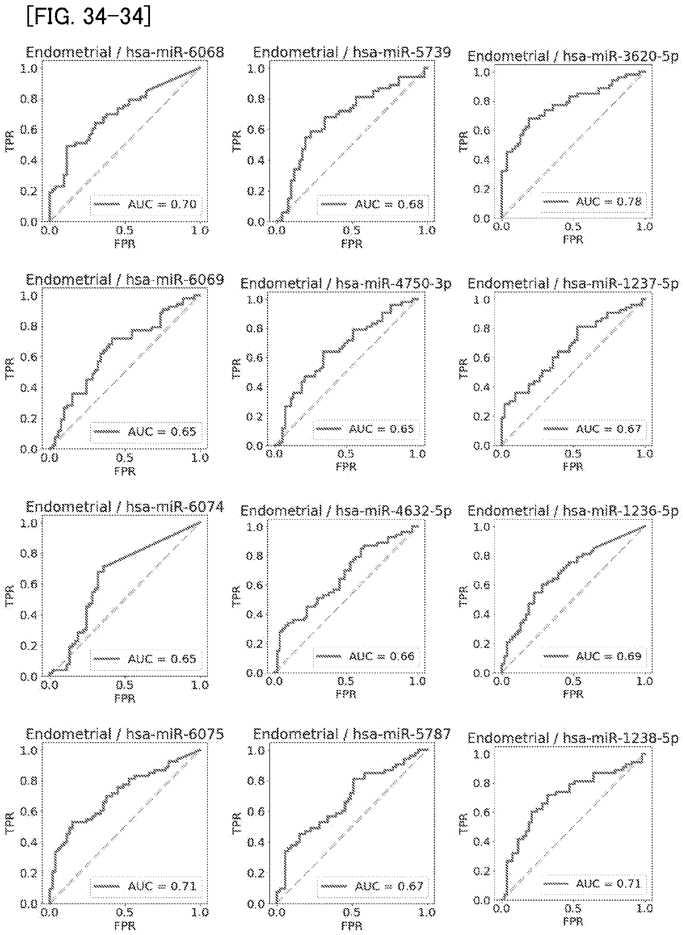

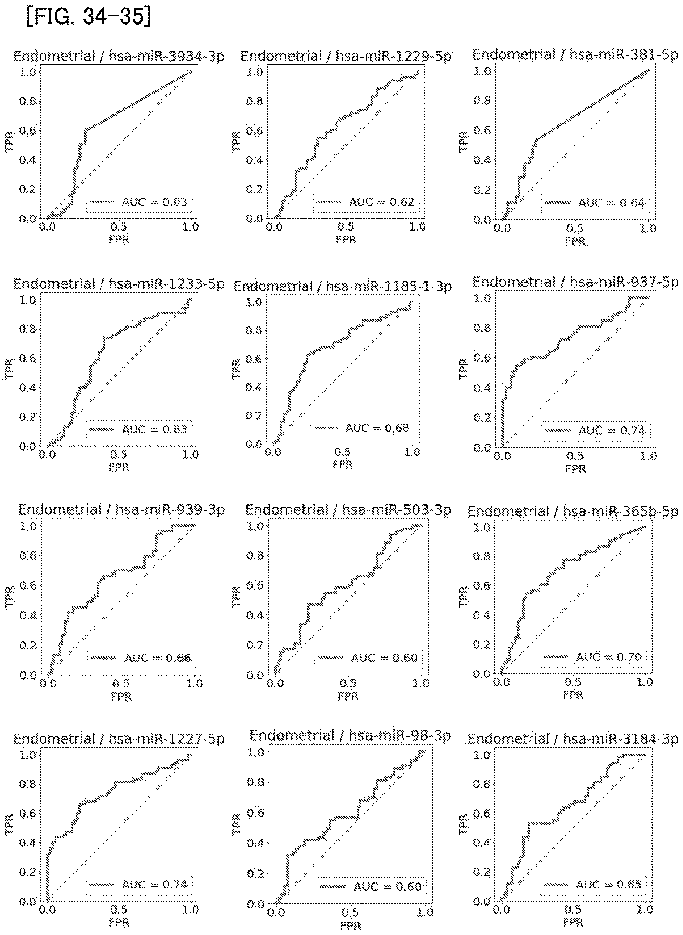

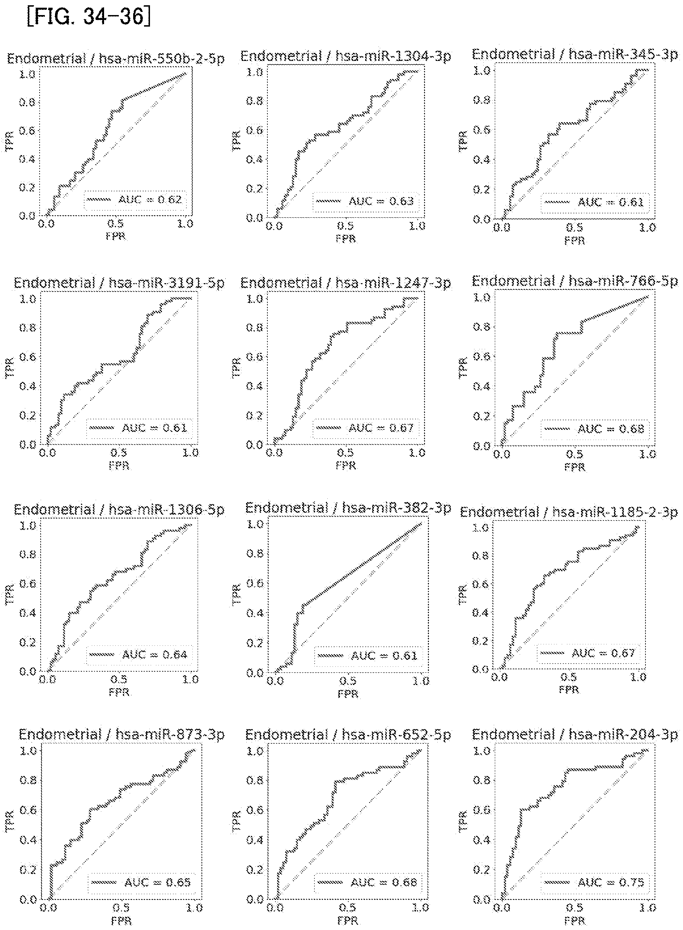

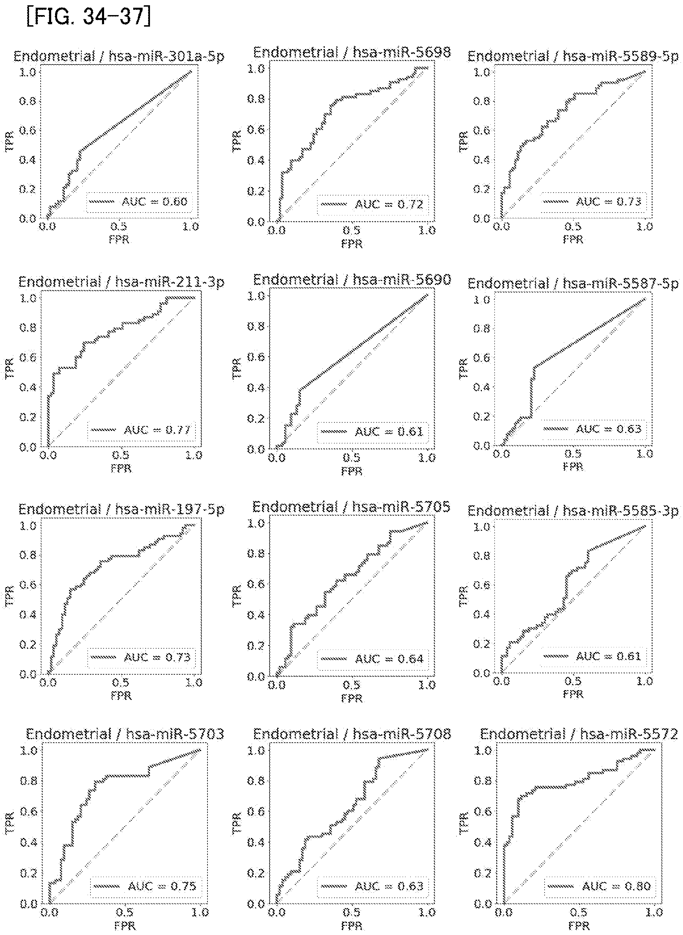

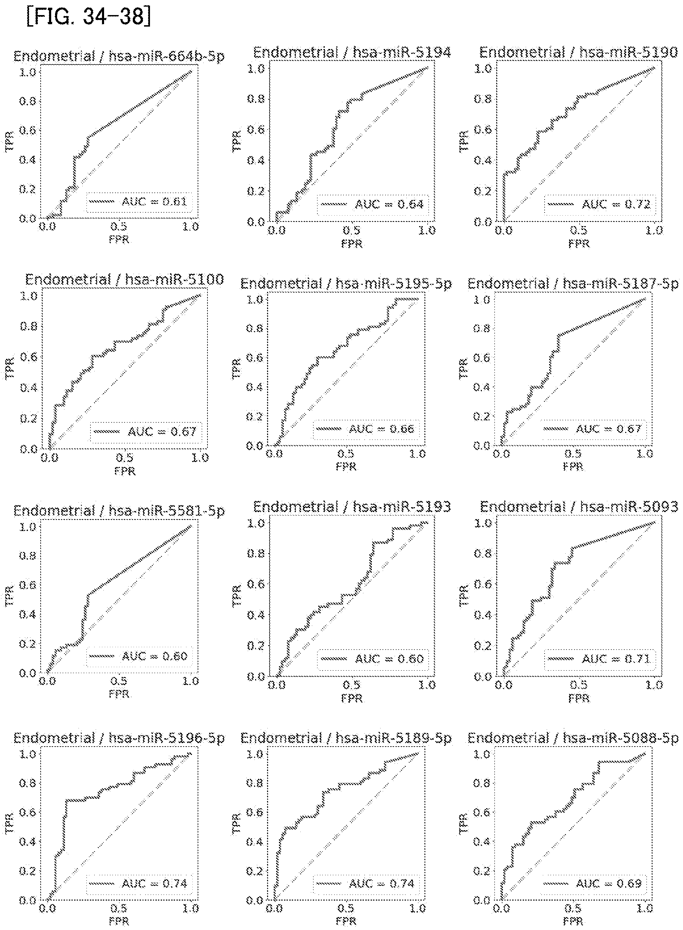

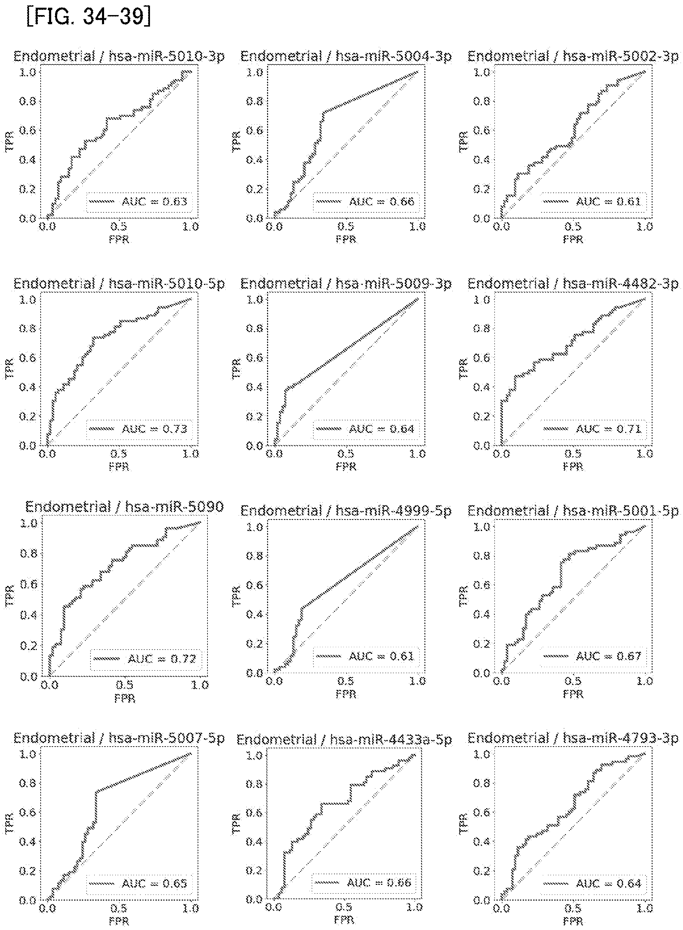

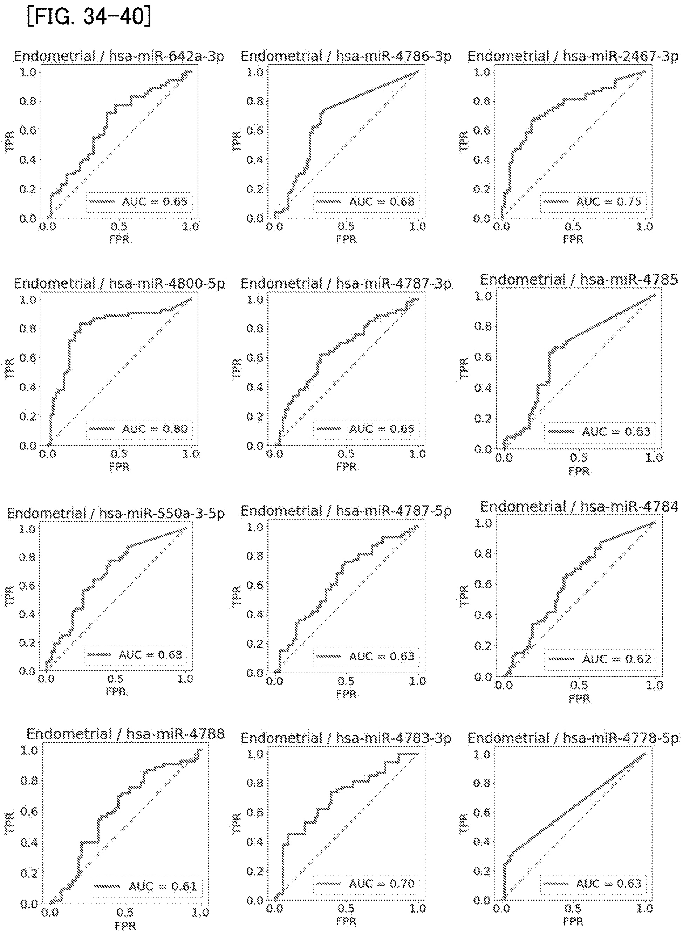

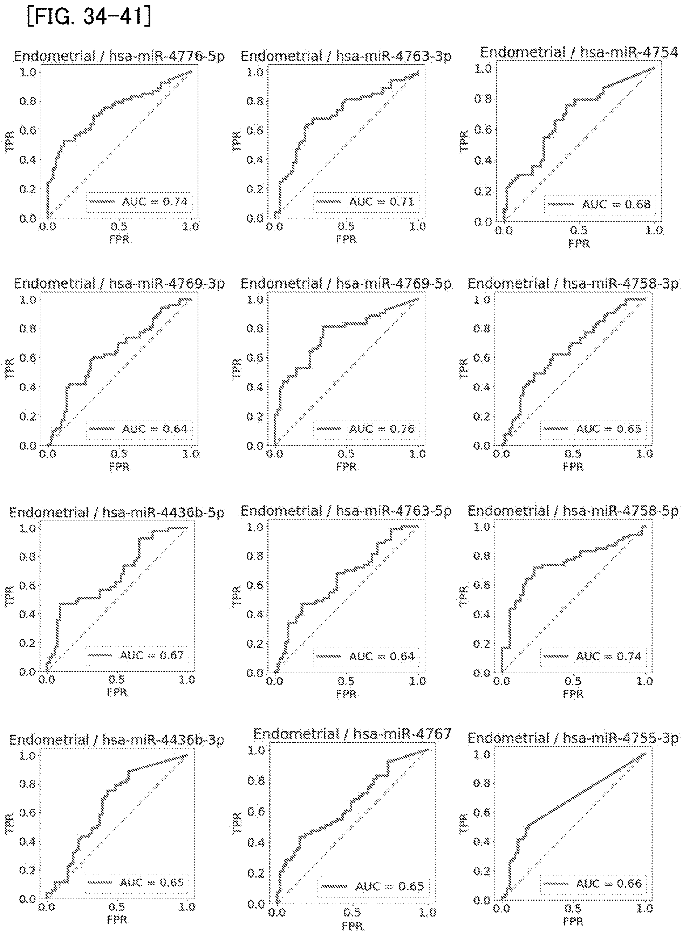

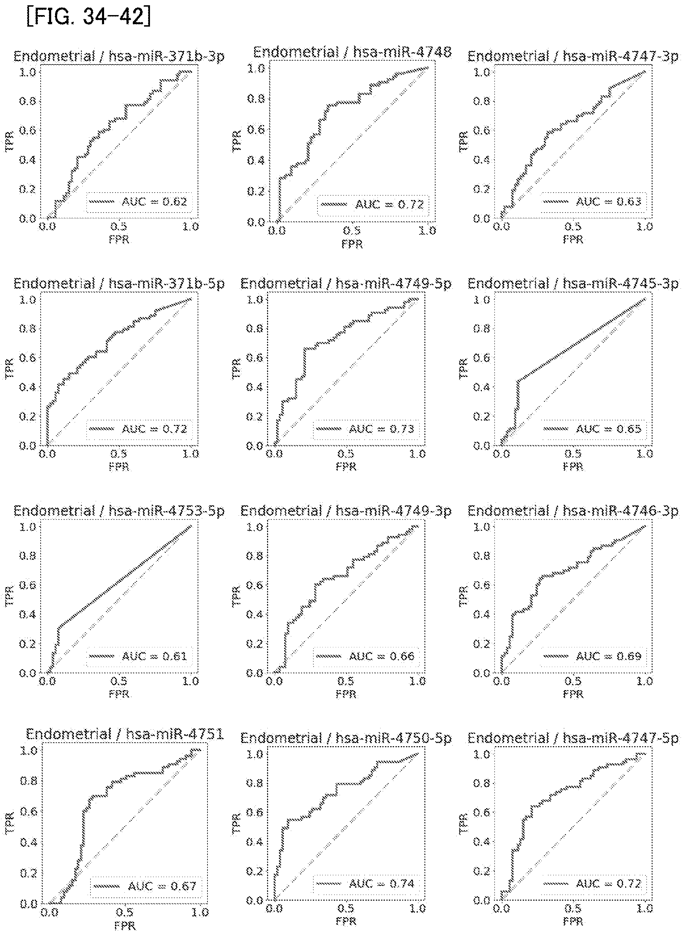

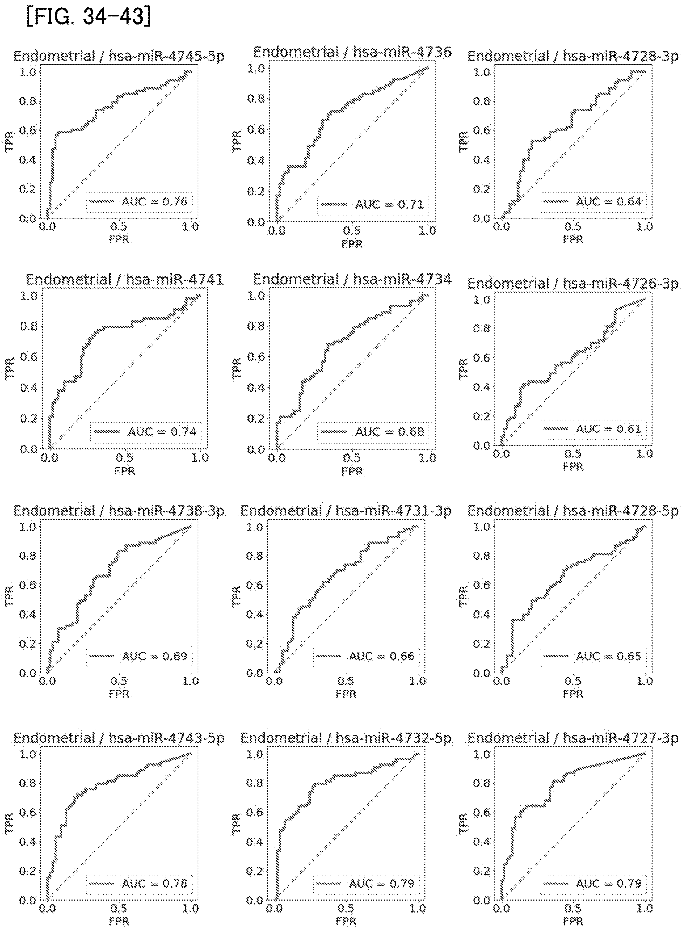

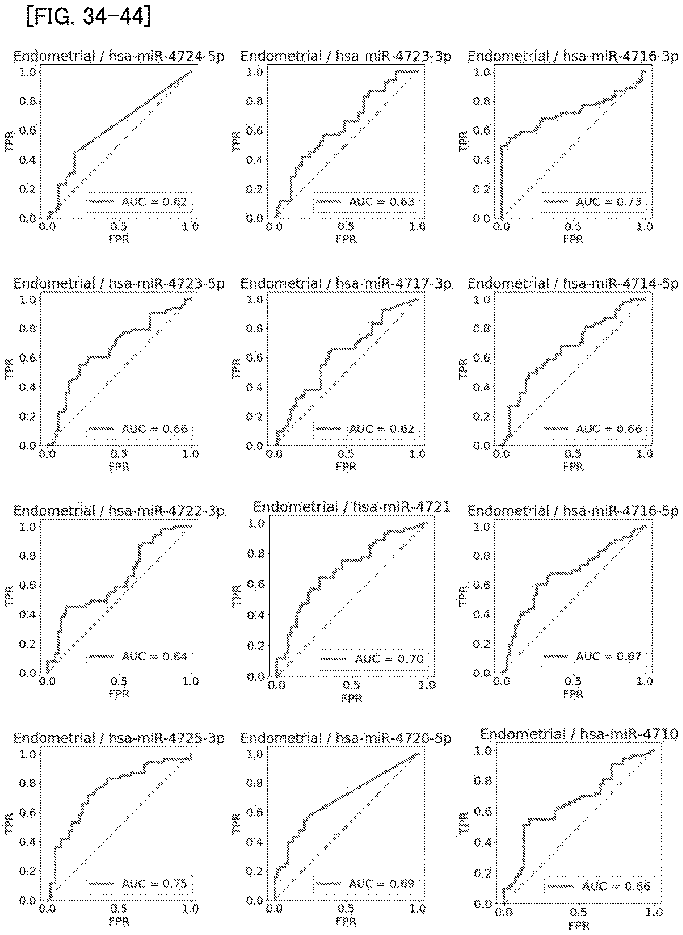

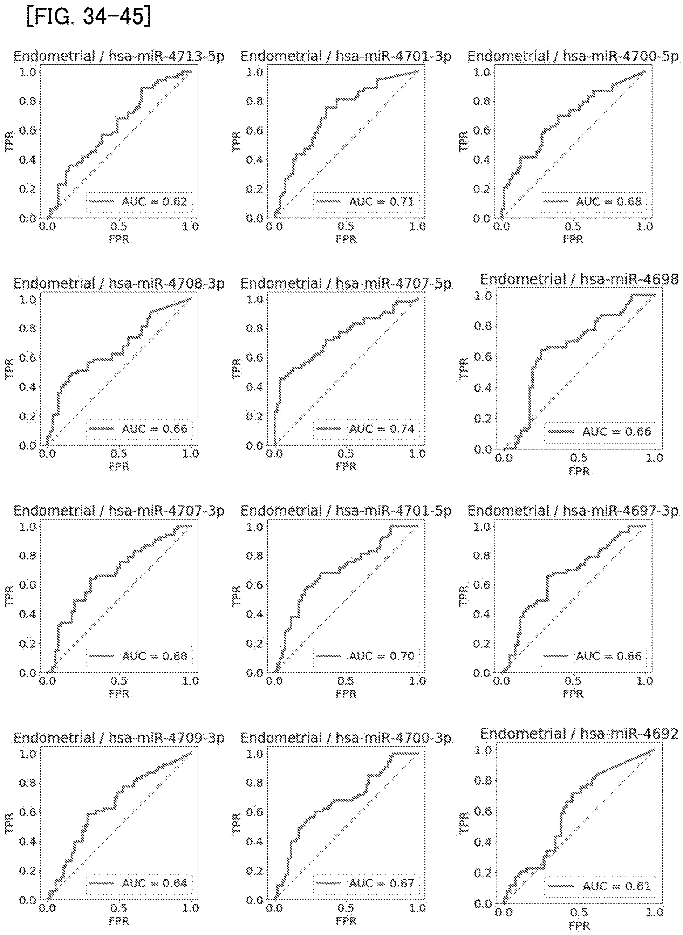

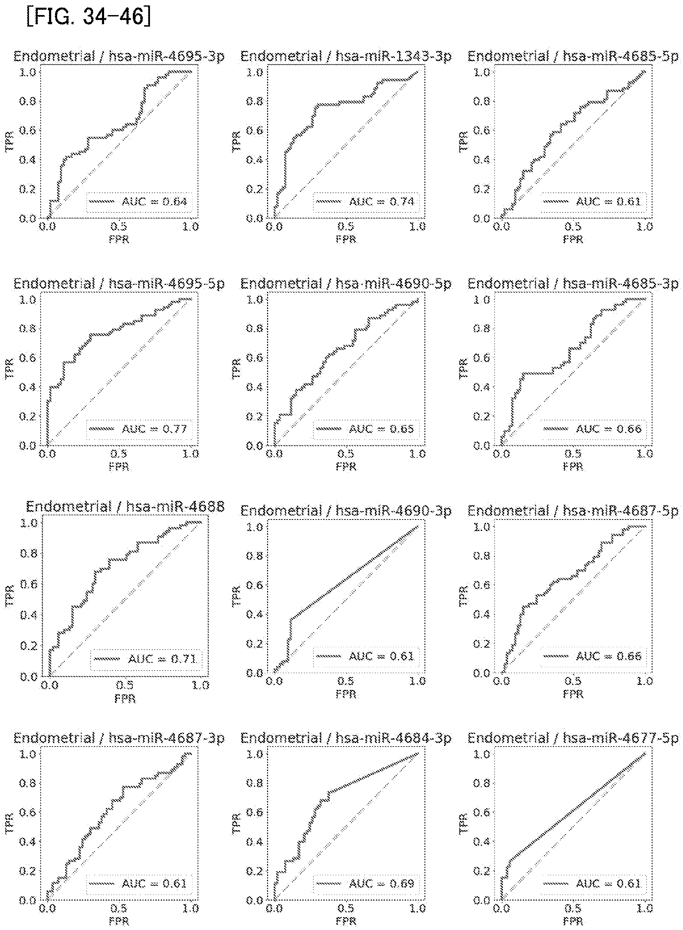

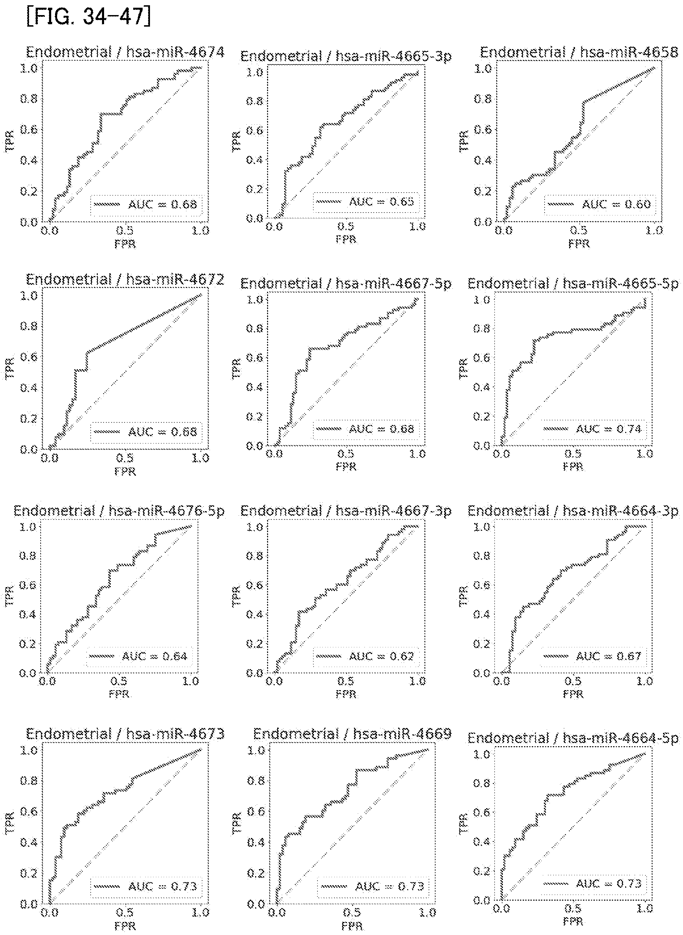

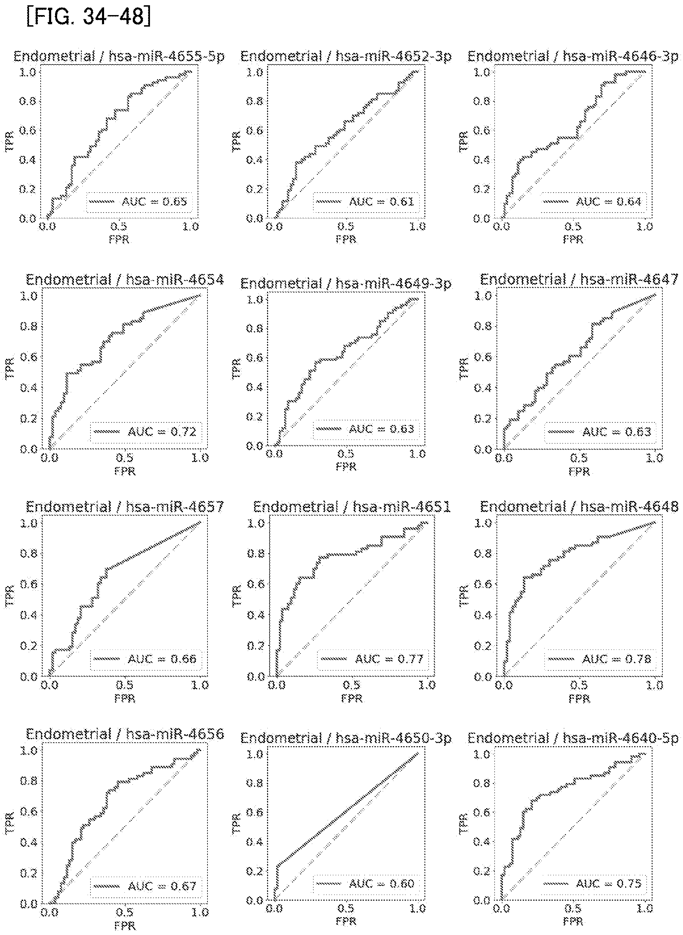

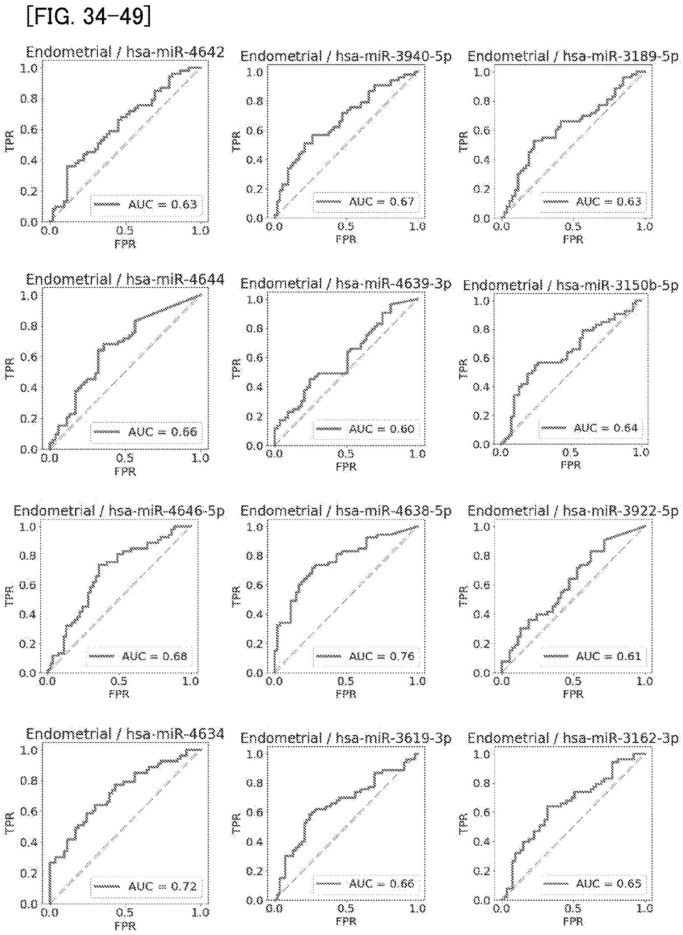

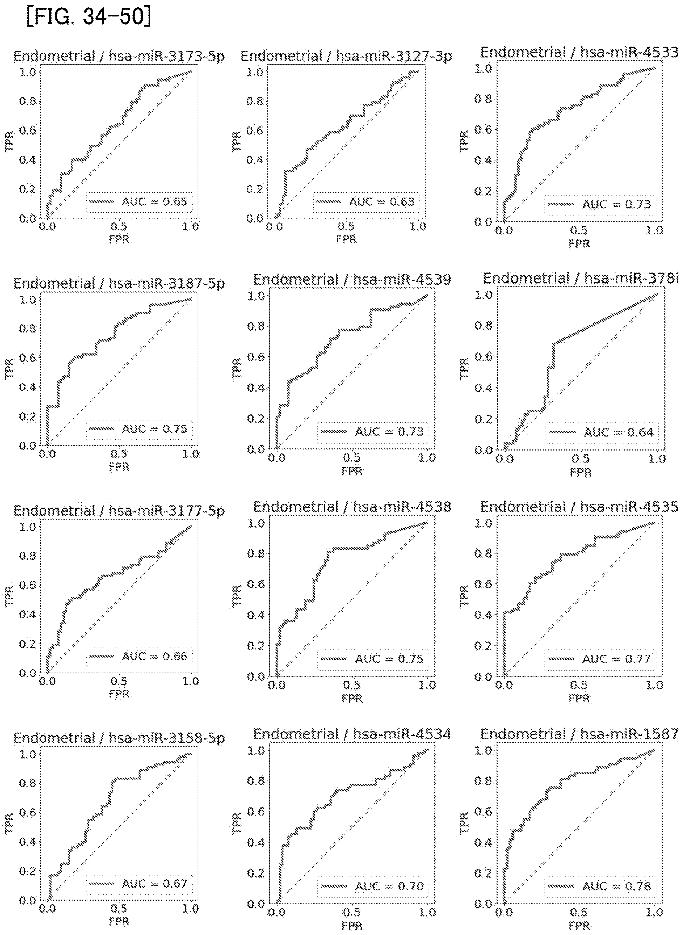

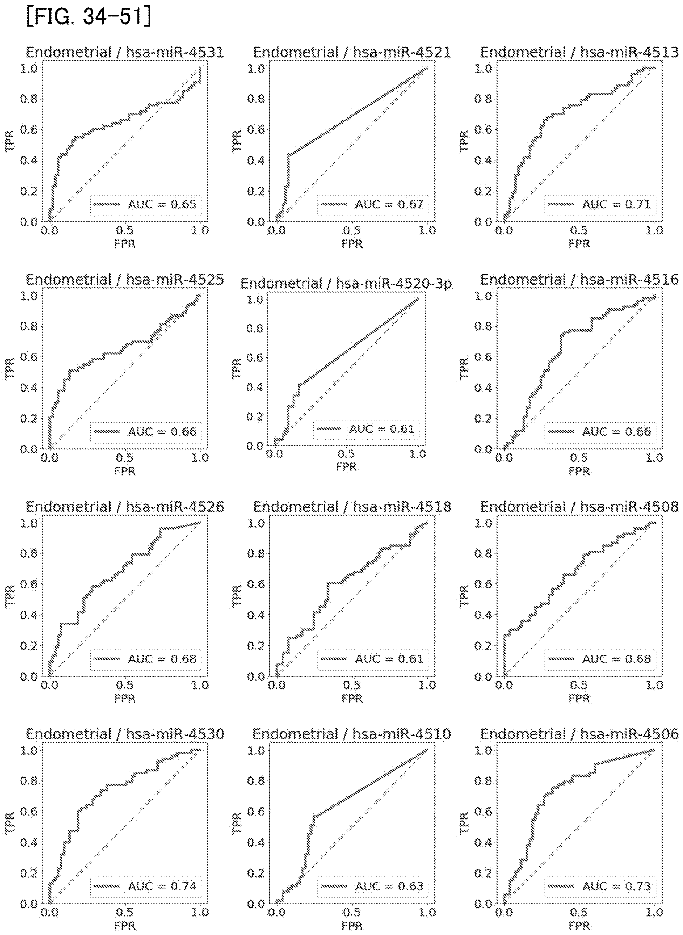

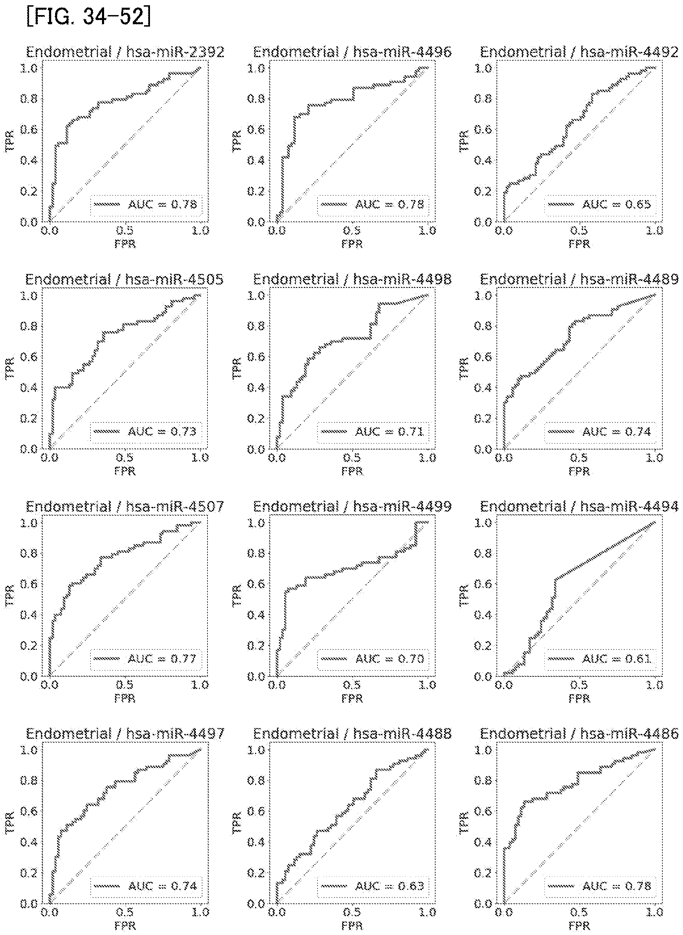

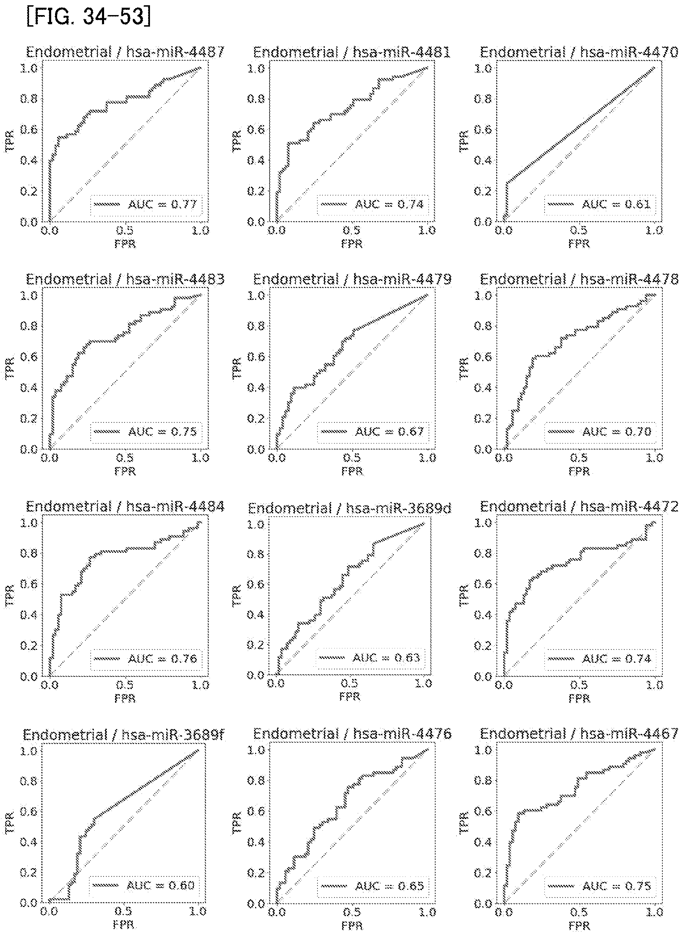

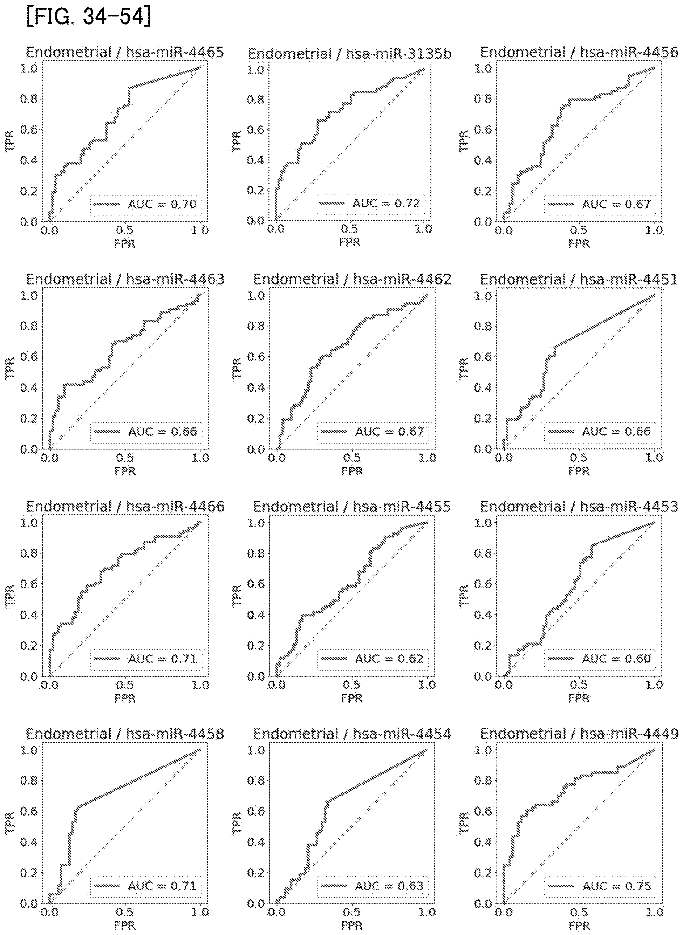

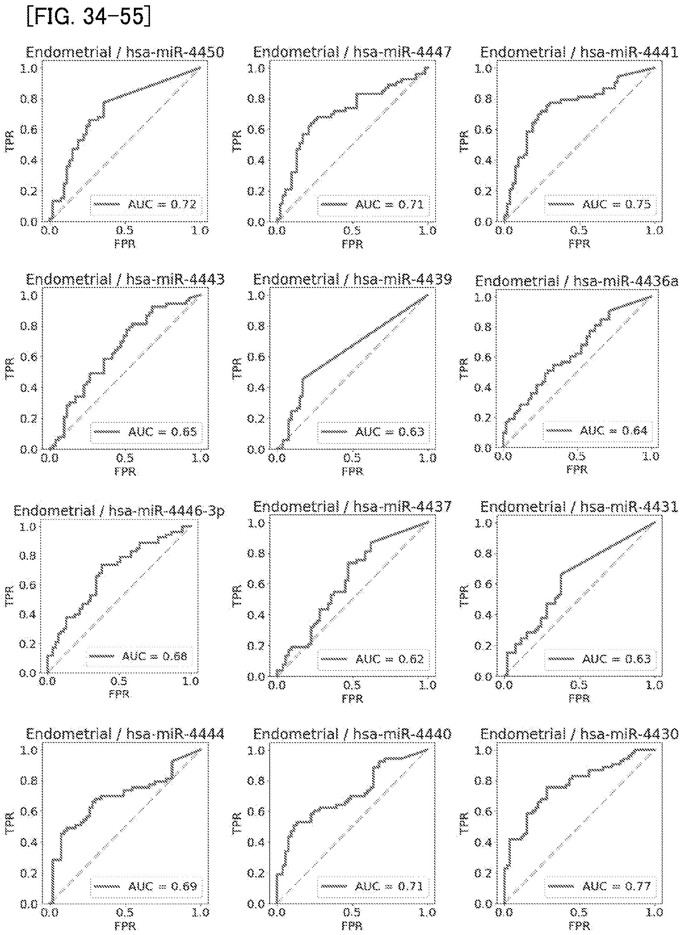

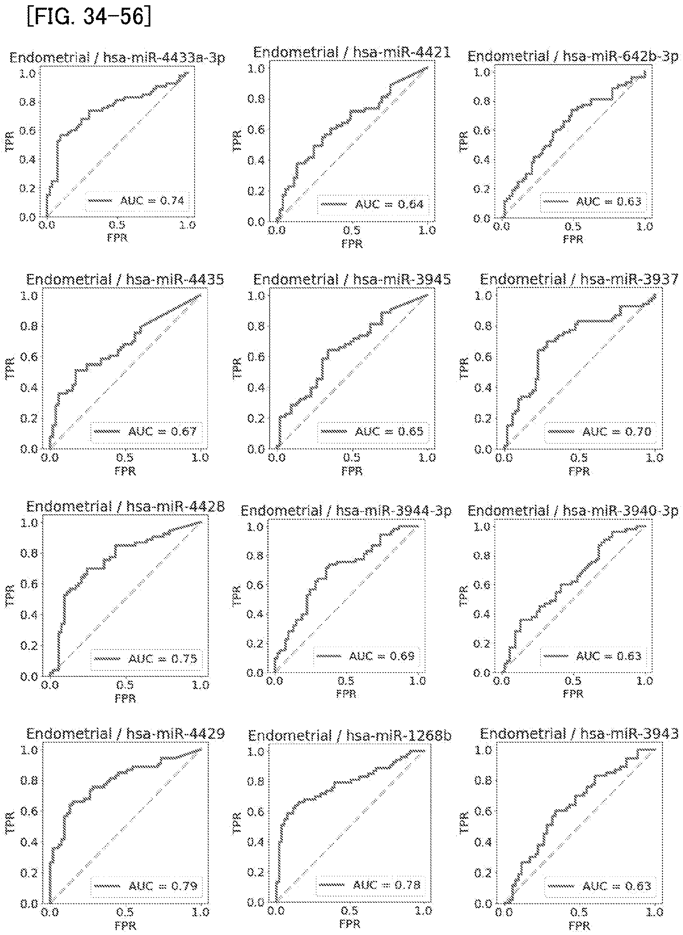

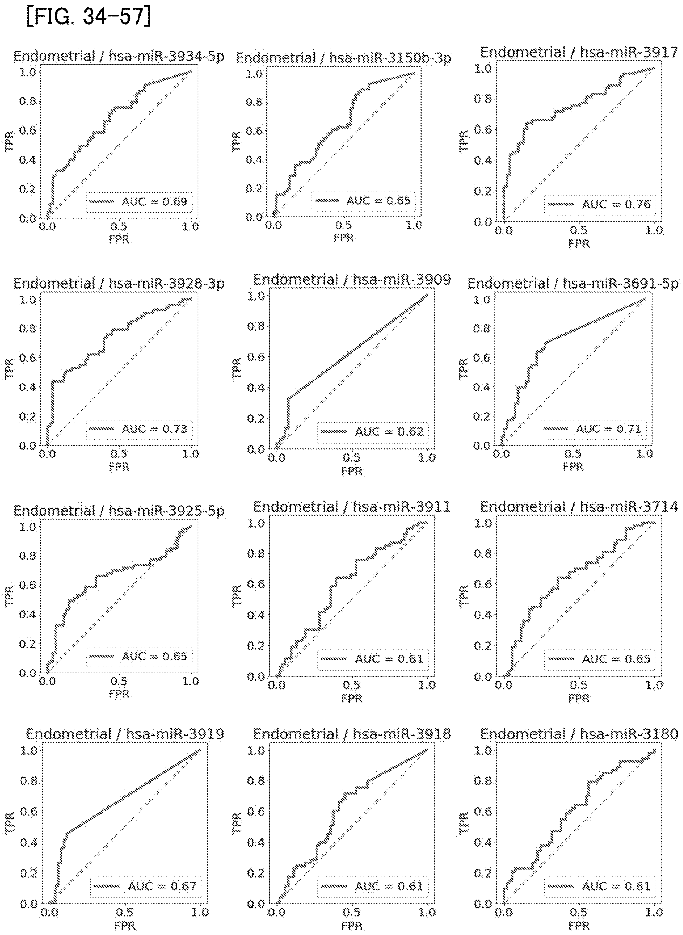

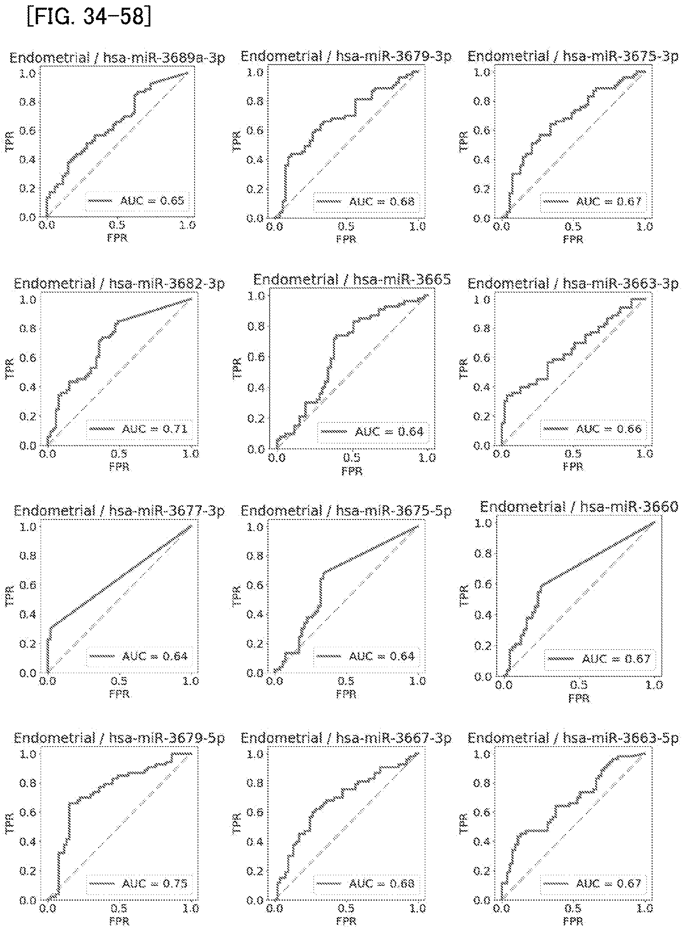

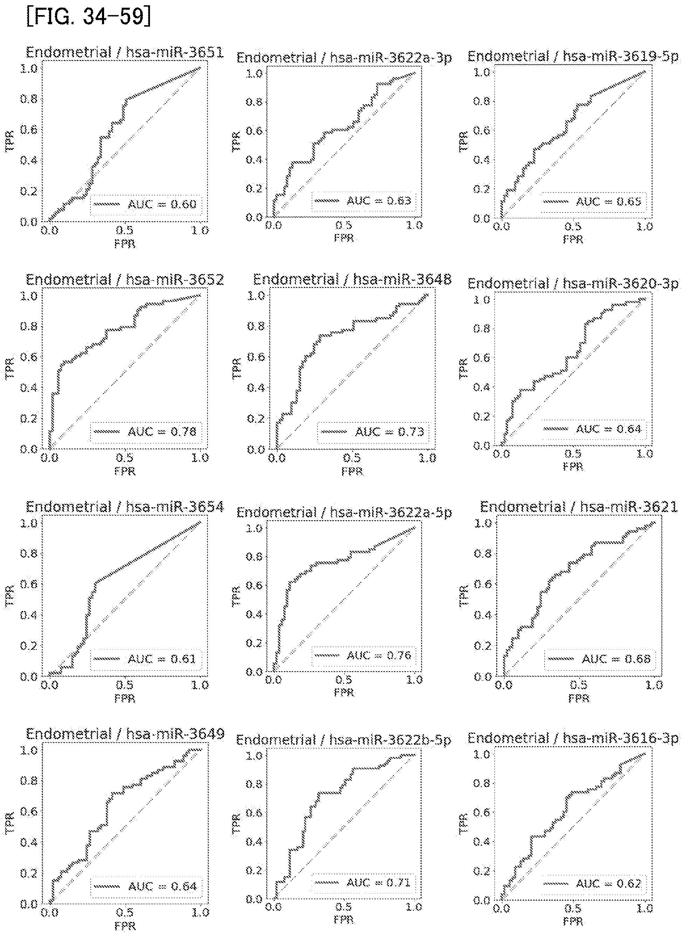

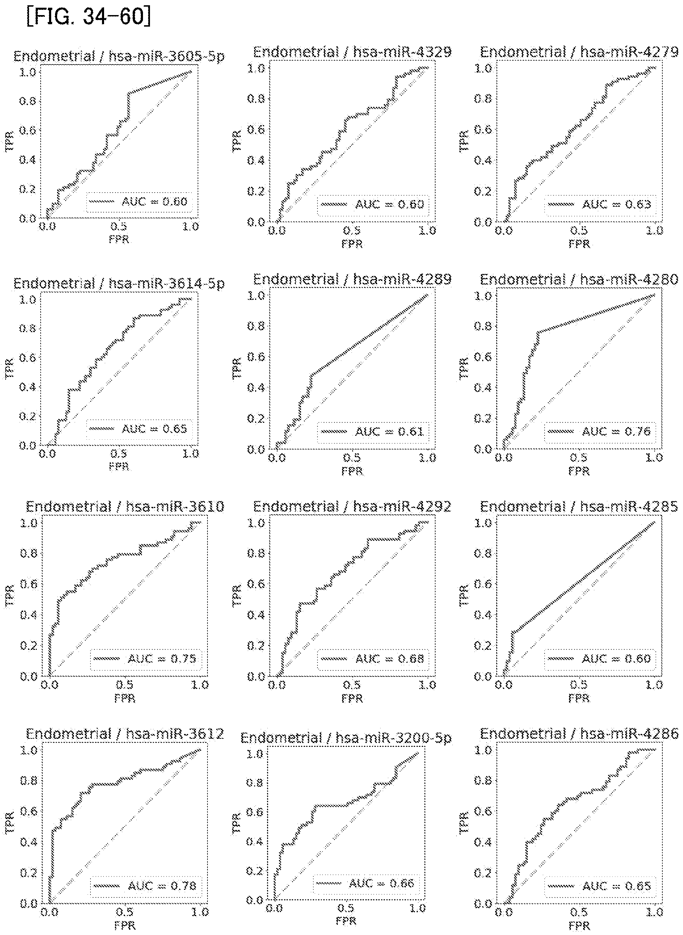

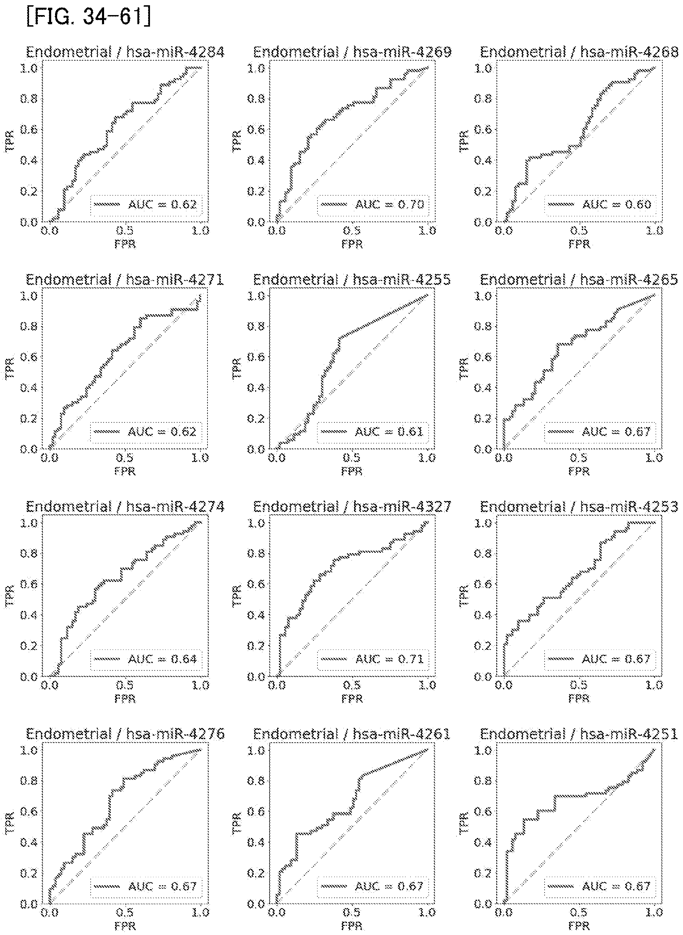

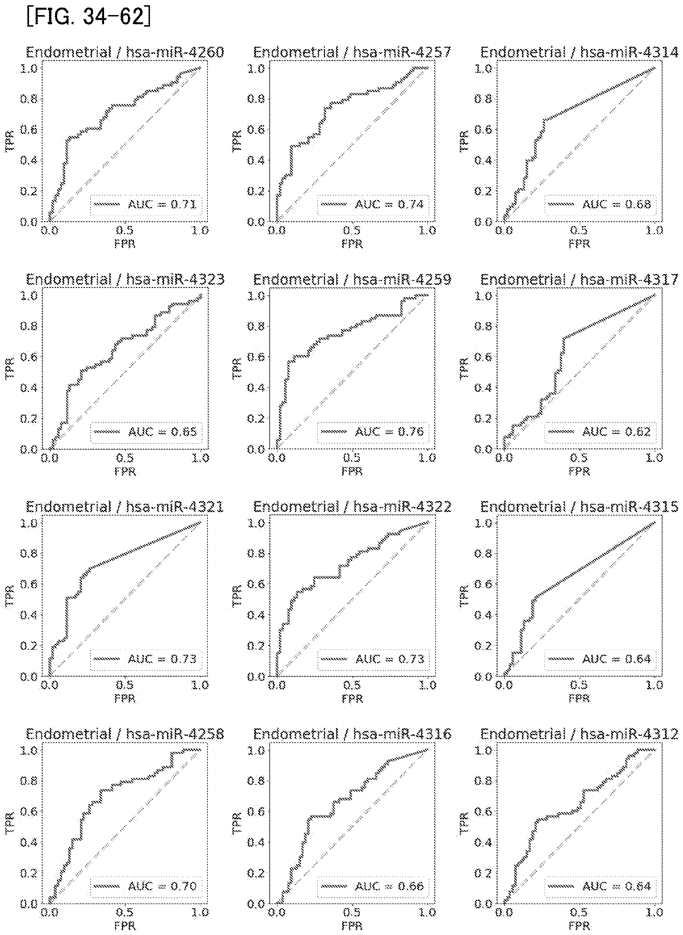

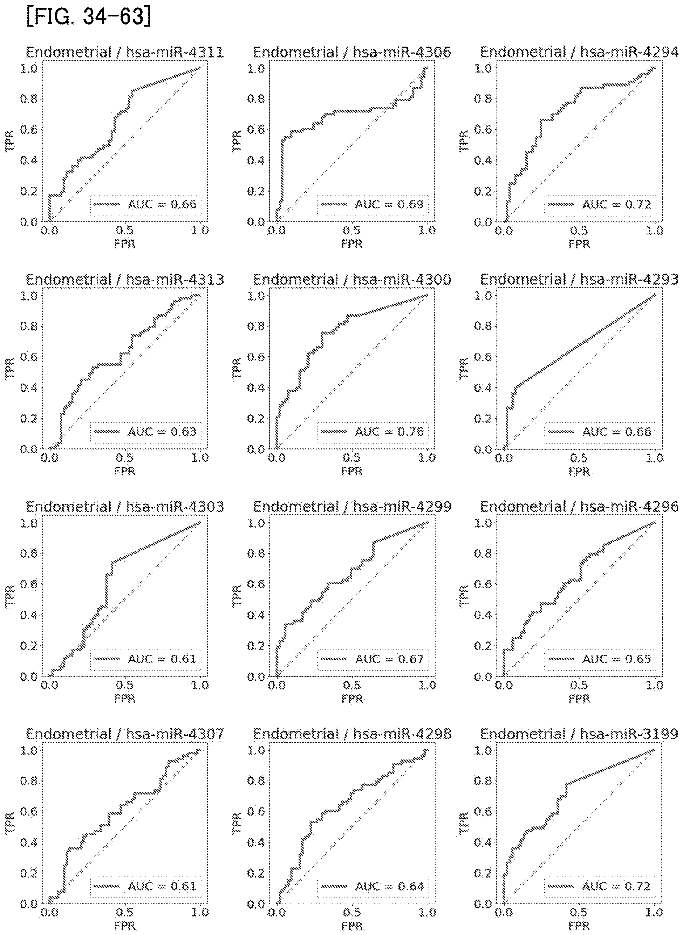

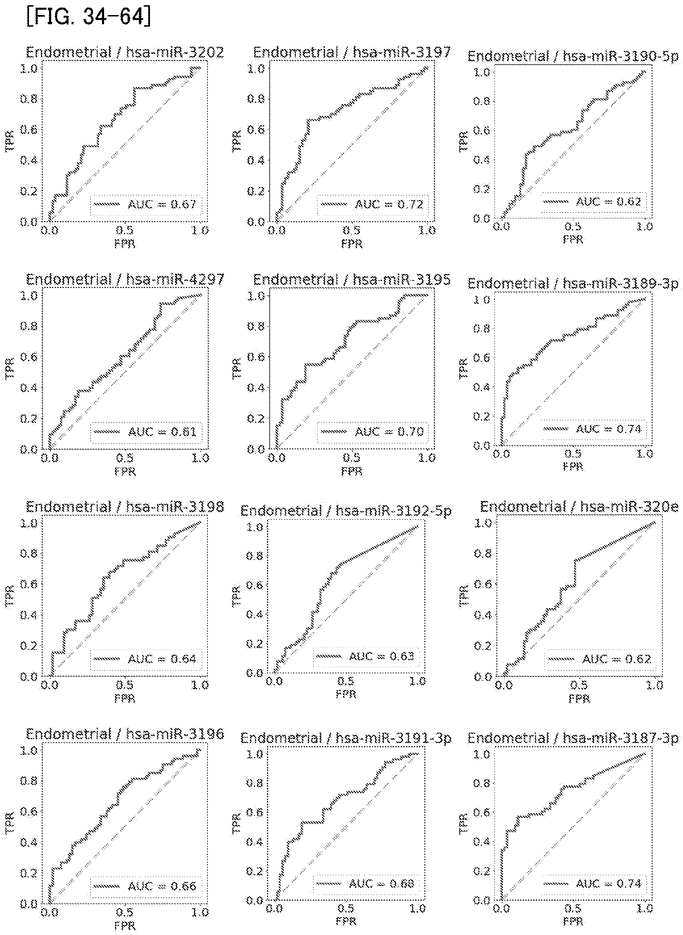

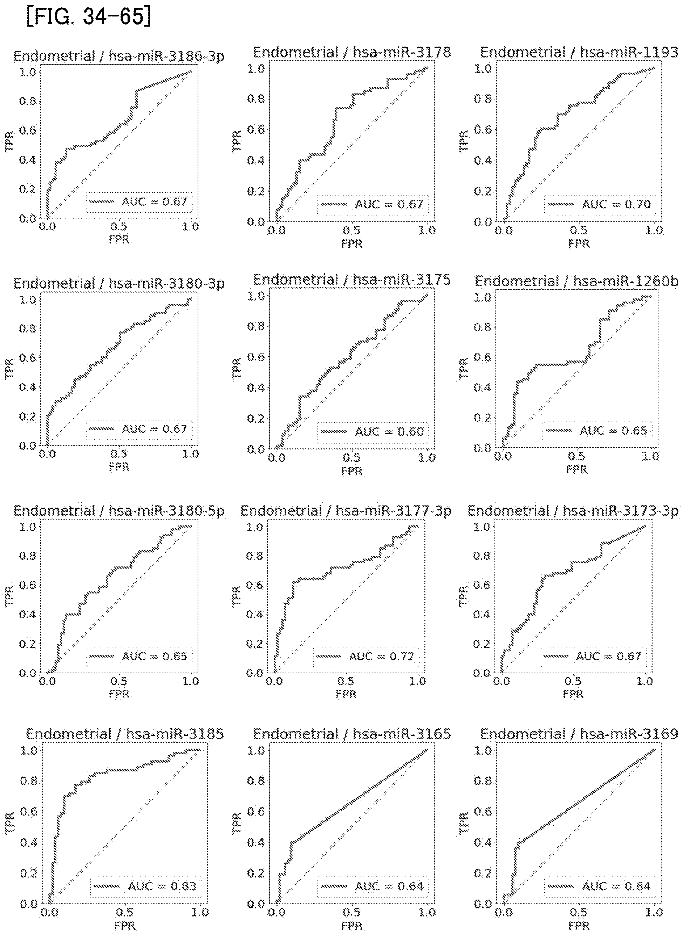

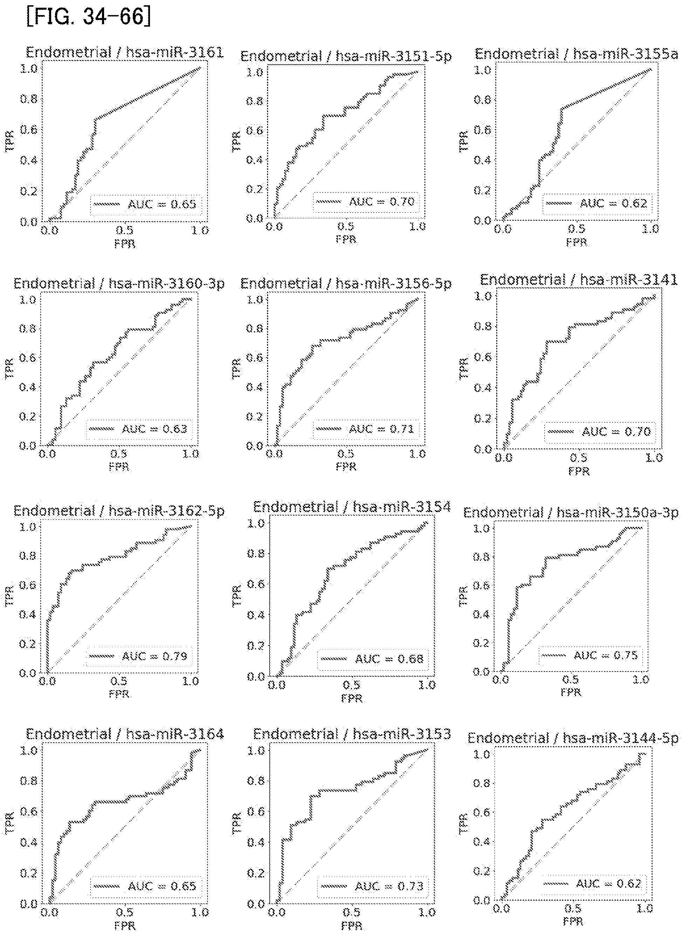

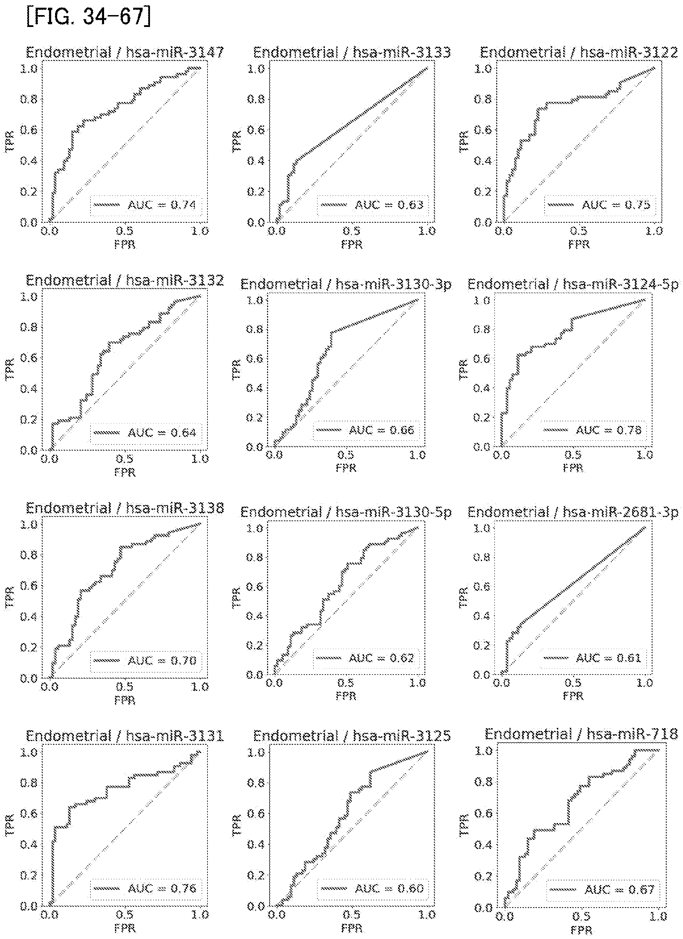

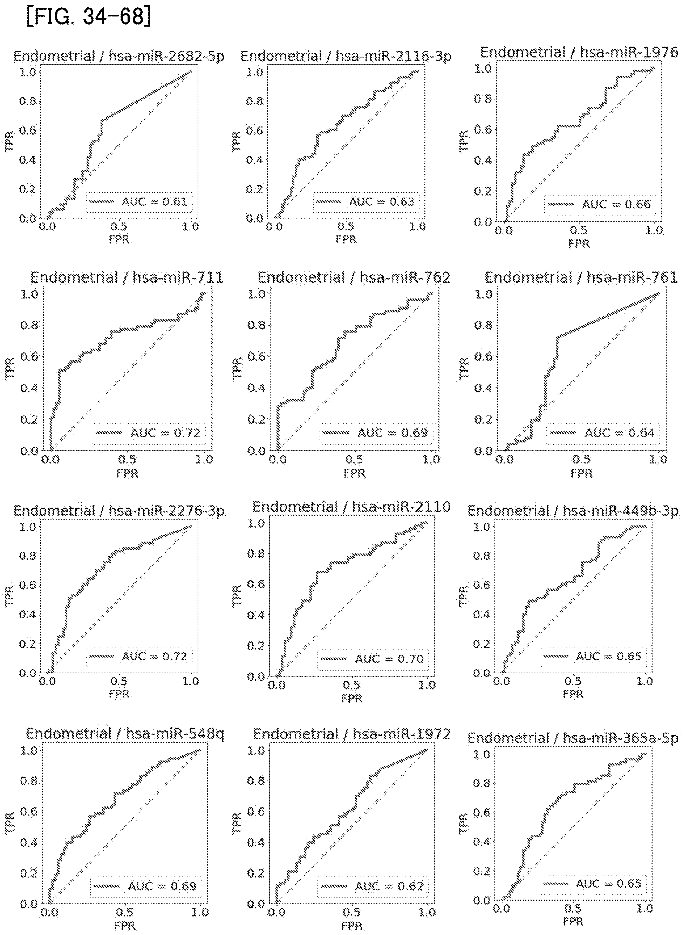

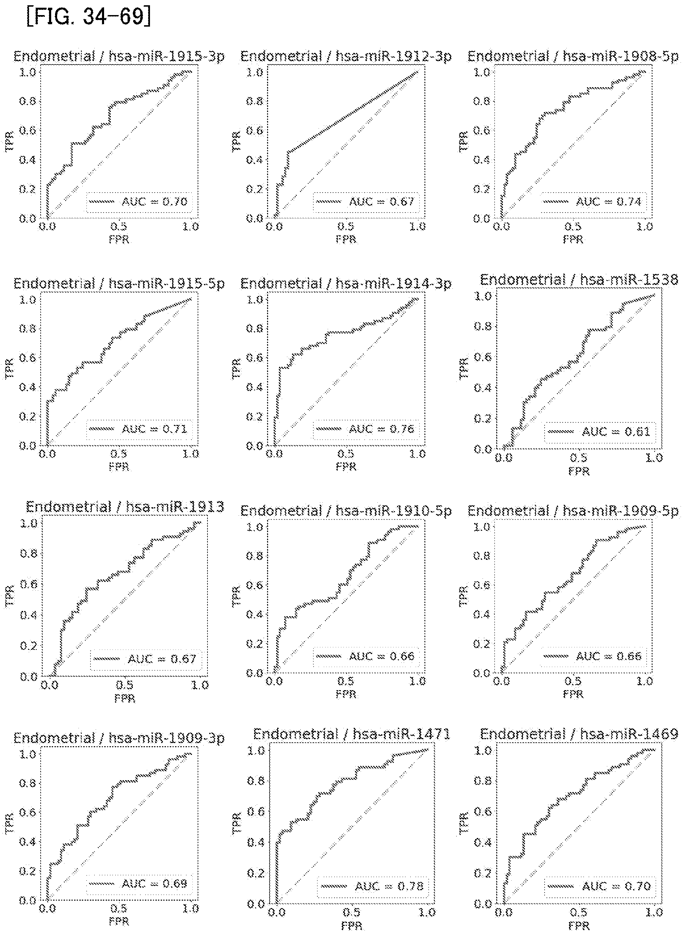

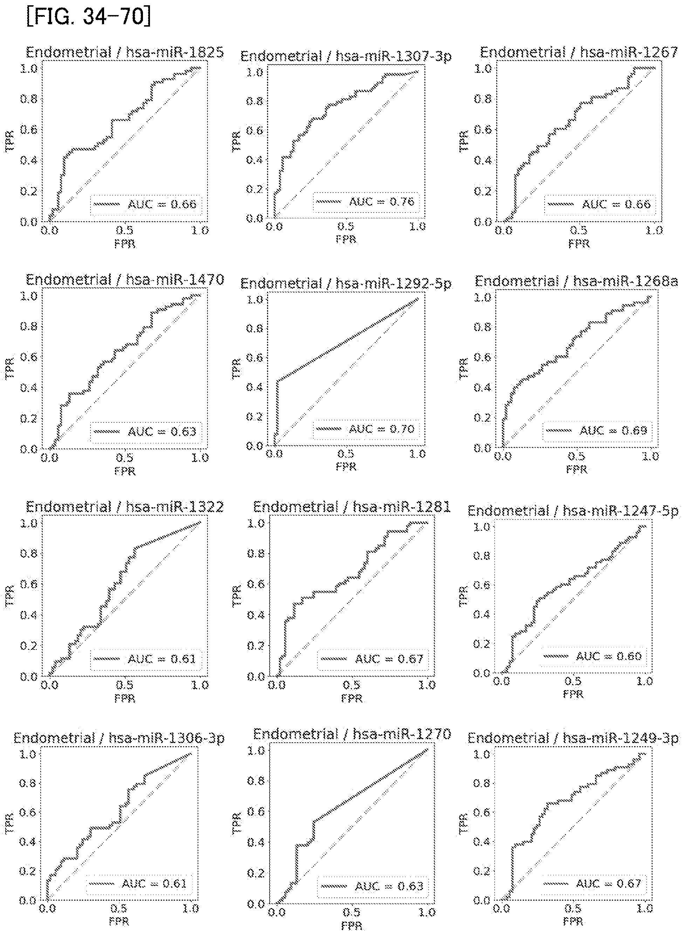

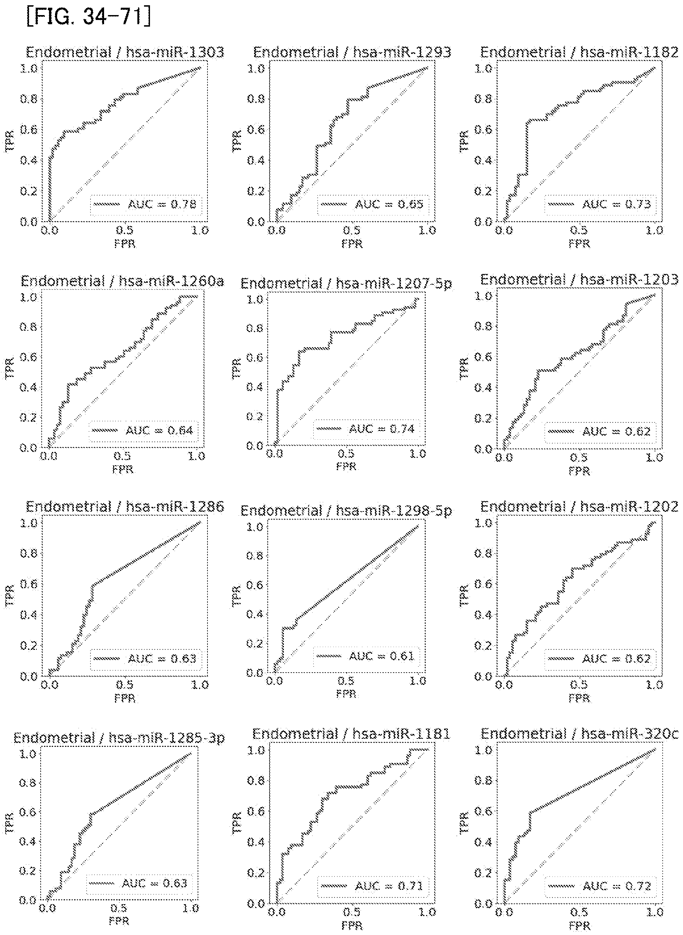

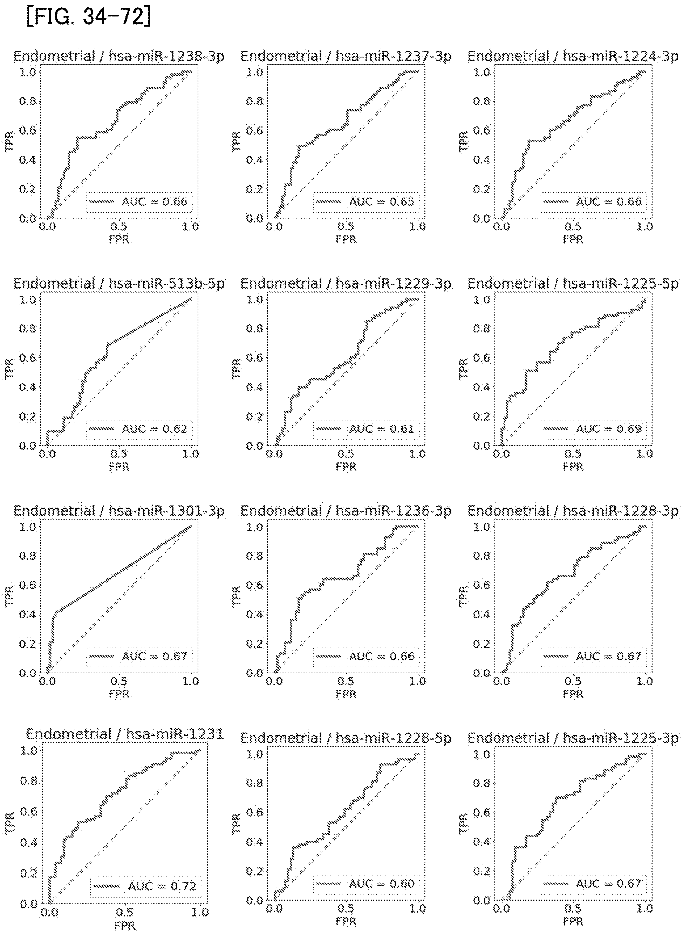

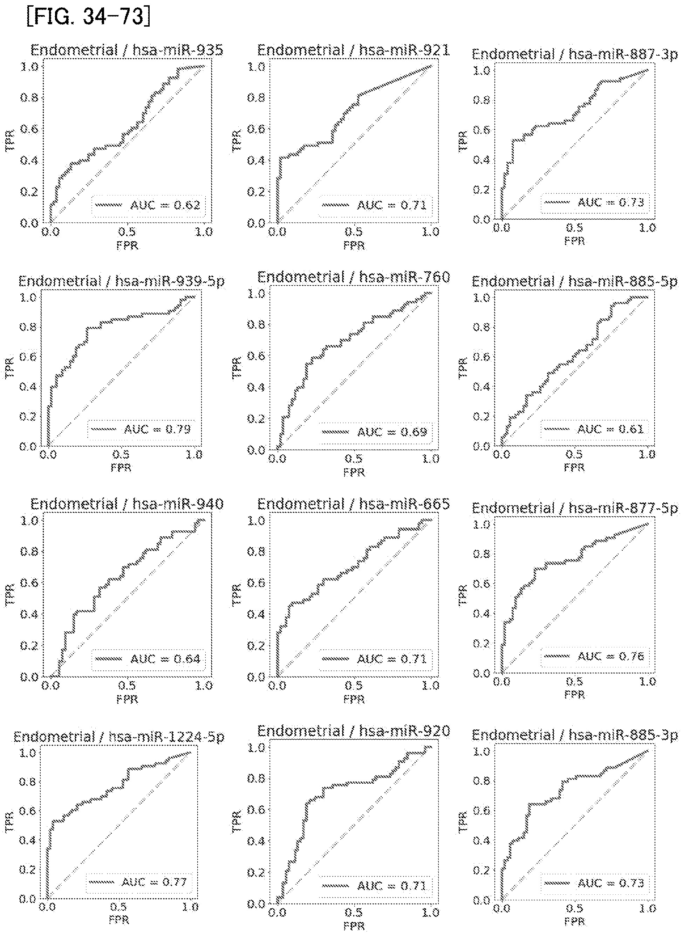

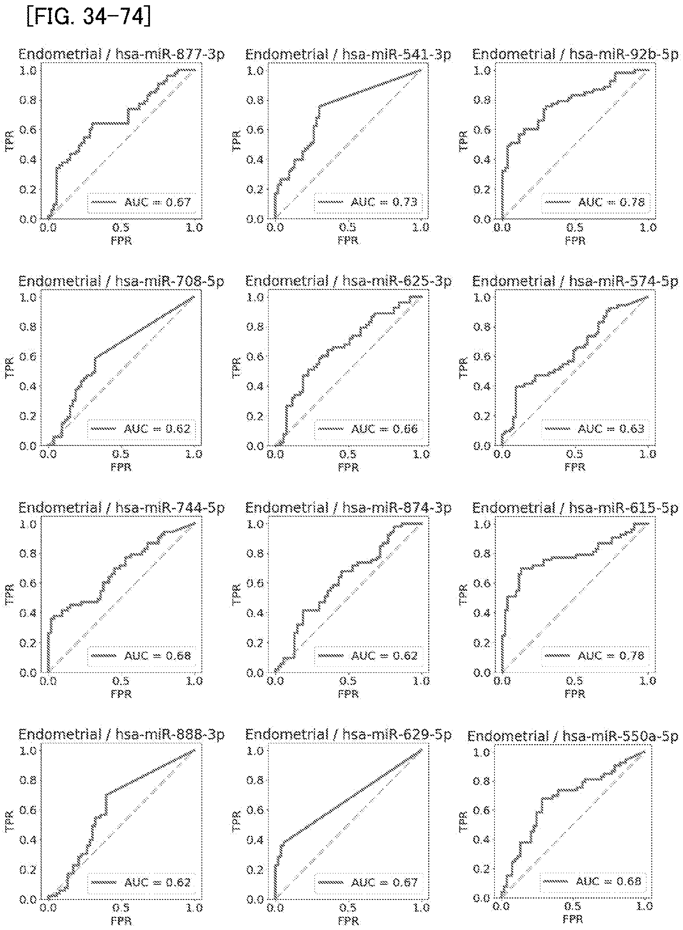

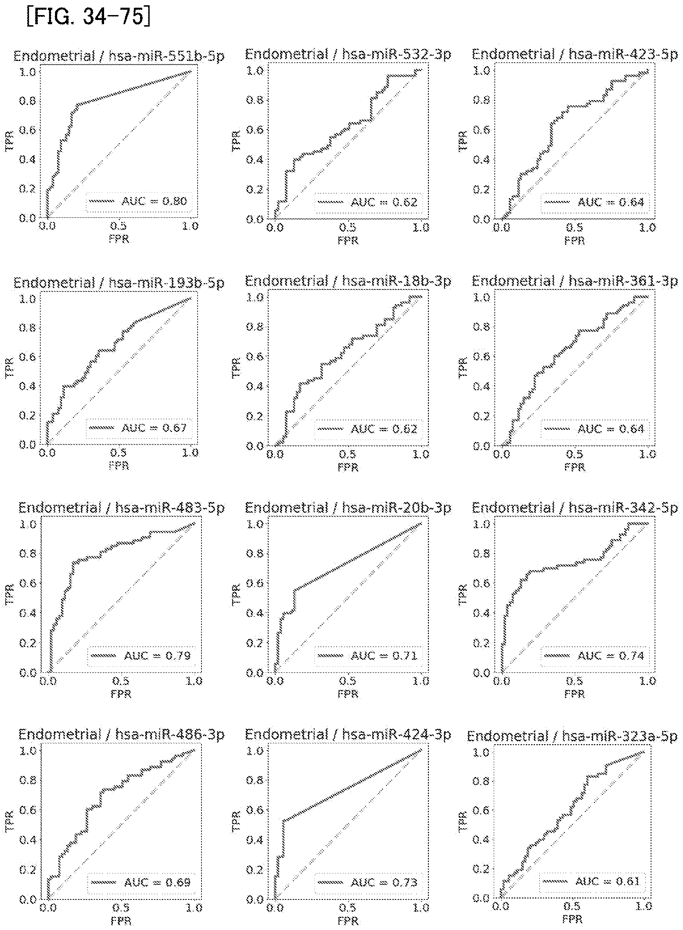

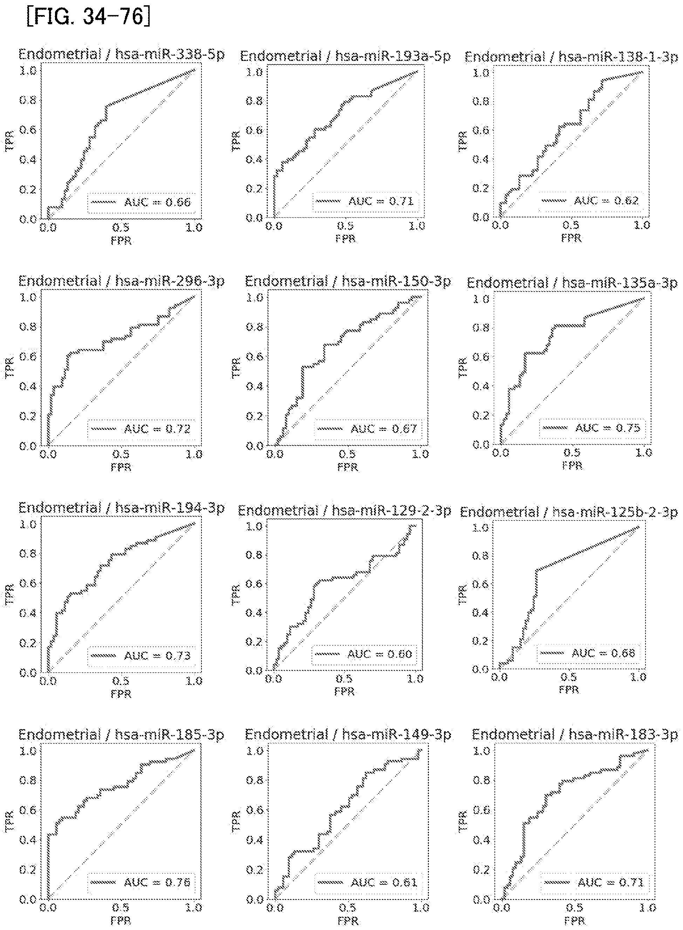

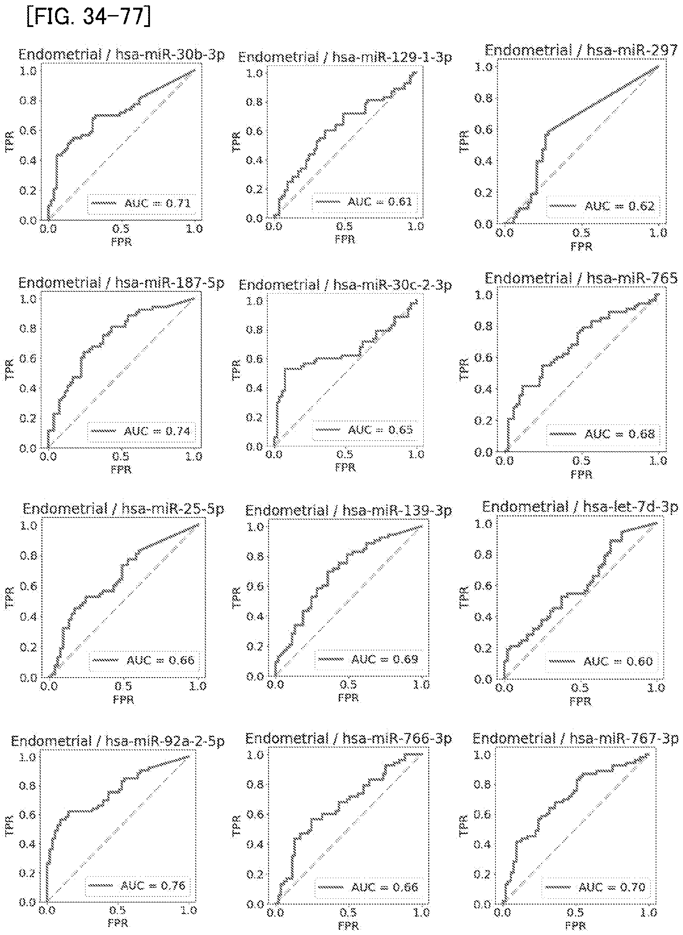

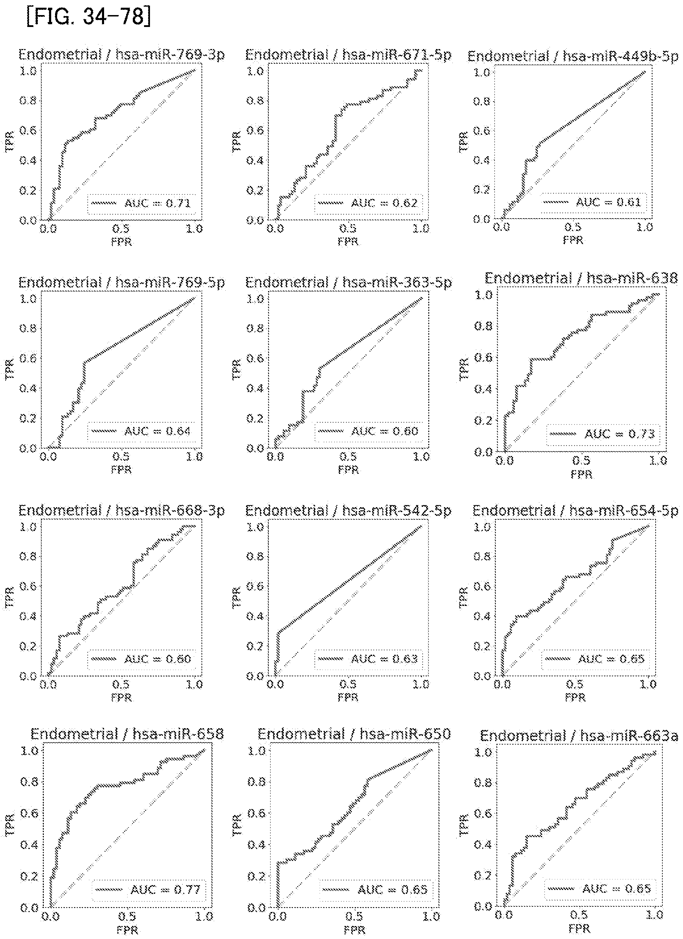

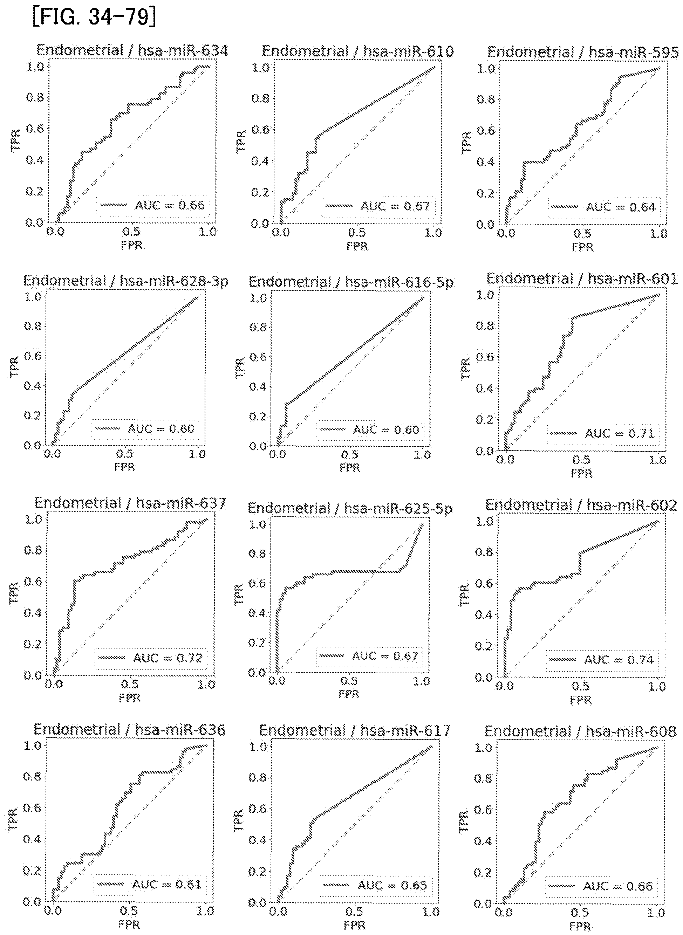

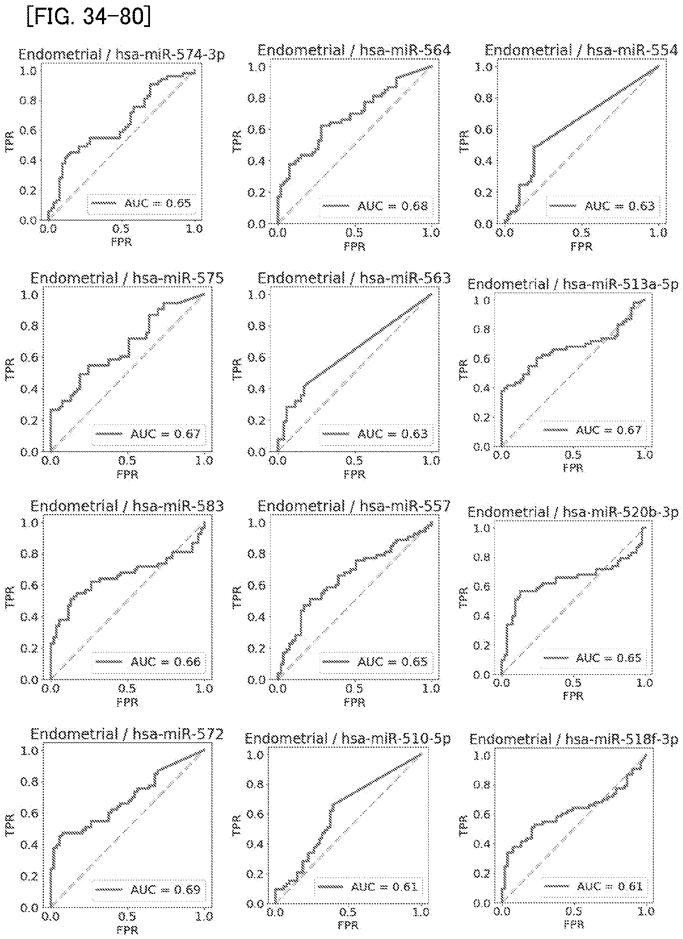

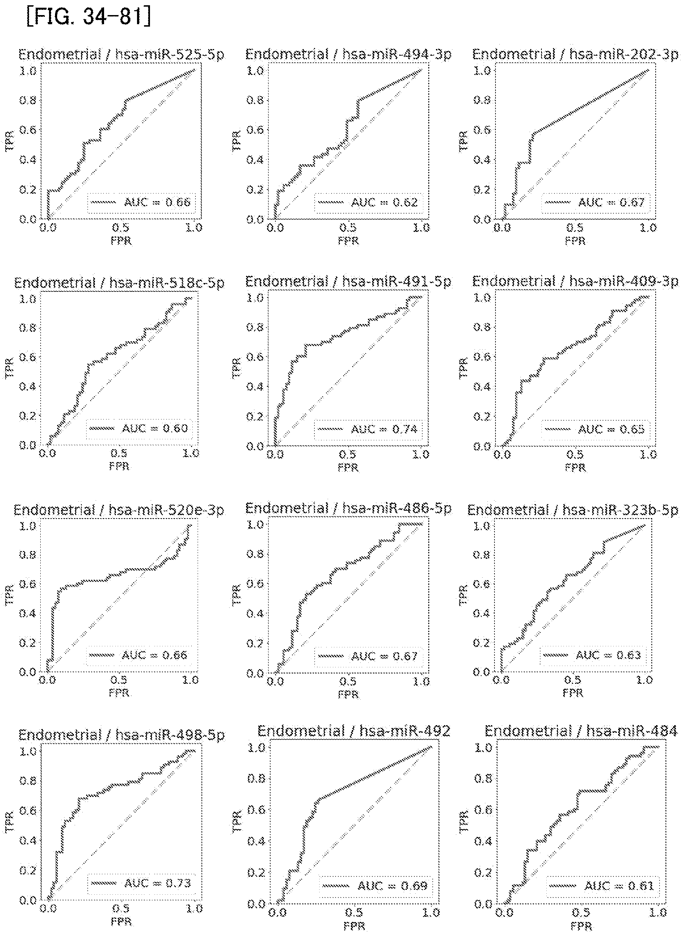

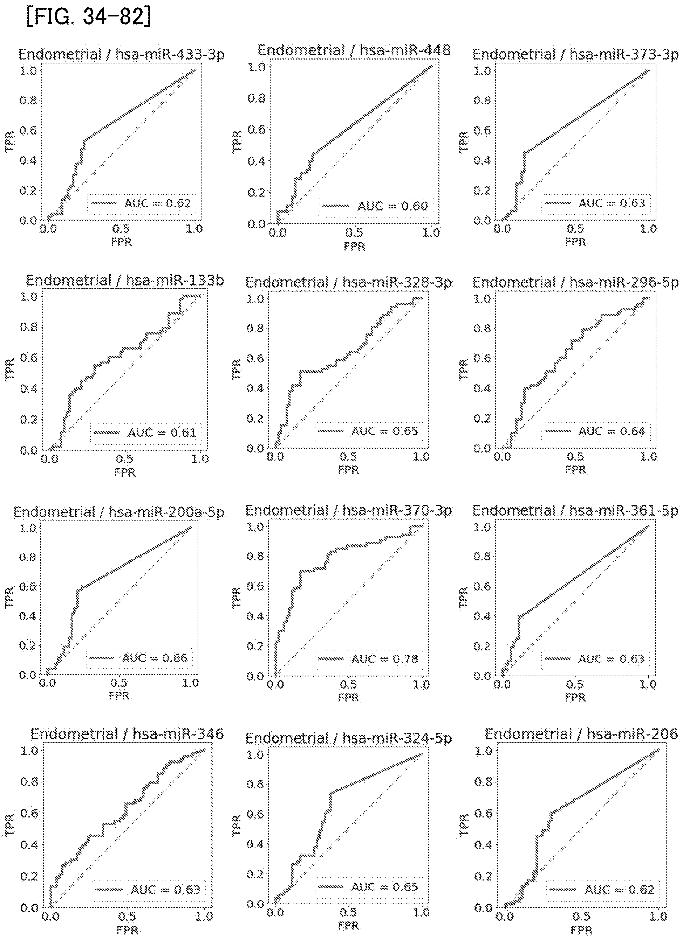

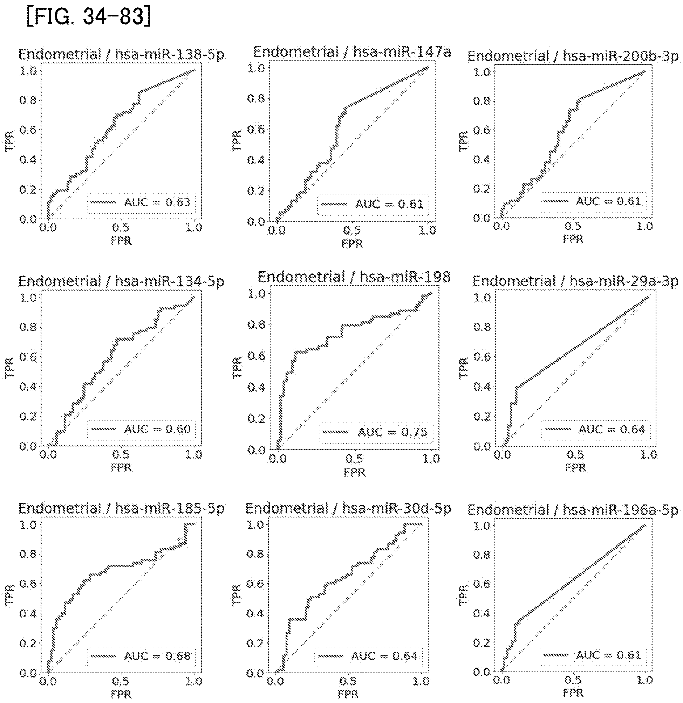

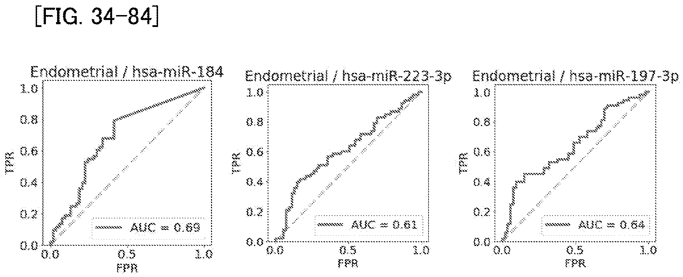

FIGS. 34-1 to 34-84 show ROC-curves for predicting human endometrial cancer using a single miRNA.

### DETAILED DESCRIPTION OF THE INVENTION

As used herein, "subject" means a subject for urinalysis.

The subject may be an animal. The subject may be a reptile, mammal, amphibian. Mammals may be dogs, cats, cows, horses, sheep, pigs, hamsters, mice, squirrels, and primates such as monkeys, gorillas, chimpanzees, bonovo, humans. The subject may in particular be a human.

As used herein, "urine" means liquid waste produced by the kidneys.

Urine can be either drained out through the urethra or accumulated in the bladder. Urine may be extracted or collected and collected from the body using an extractor such as a syringe. In this specification, the urine is not particularly limited, but may be, for example, a reptile, a mammal, an amphibian urine. Mammals may be dogs, cats, cows, horses, sheep, pigs, hamsters, mice, squirrels, and primates such as monkeys, gorillas, chimpanzees, bonovo, humans. "Urine" may be urine of a healthy subject, urine of a subject with a particular disease (e.g., cancer selected from cancers such as lung cancer, liver cancer, pancreatic cancer, bladder cancer, and prostate cancer, etc.), or urine of a subject suspected of suffering from a particular disease. "Urine" may be used as a stock solution or may be a liquid in which the stock solution is diluted or concentrated. "Urine" may be obtained by adding an additive to a urine sample. The additive may be, for example, one obtained by adding a stabilizing agent or a pH adjusting agent. "Urine" may be urine in a frozen state.

As used herein, "microRNA" (also referred to as "miRNA") is a type of non-coding RNA (ncRNA) that is believed not to encode a protein.

MicroRNAs are processed from their precursors into mature bodies.

It is known that the mature microRNA has a length of about 20 to 25 bases. Human microRNA is denoted by the name hsa {In this specification, since the examples all measure human microRNA, the notation hsa has been abbreviated, but the results of human microRNA have been disclosed} Precursors are given mir and matures are given miR. The identified sequences are numbered in the order in which they are identified, and for similar sequences, the numbers are followed by a lower case alphabet. If there is a precursor derived from the 5' and 3' ends, the microRNAs derived from the 5' end are labeled 5p, and those derived from the 3' end are labeled 3p. These symbols and numbers are connected by hyphens. The mature microRNA may be double-stranded. In this specification, a miRNA is described as explicitly including a human miRNA, whether or not has been imparted.

As used herein, "extracellular vesicles" (also referred to as "EVs") are vesicles that are released from cells, including those released from cells during apoptosis, and those released from healthy cells. Extracellular vesicles are broadly divided into exosomes (exosome), microvesicles (micro vesicle; MVs), and apoptotic bodies (apoptosis body) according to size and surface-markers. Exosomes usually have a diameter of 40 to 120 nm and may express 1 or more or all molecules selected from the group consisting of Alix, Tsg101, CD9, CD63, CD81, and flotillin.

Microvesicles usually have a diameter of 50 to 1,000 nm and may express 1 or more or all molecules selected from the group consisting of integrins, selectins, and CD40. Apoptotic bodies usually have a diameter of 500 to 2,000nm and may express 1 or more molecules selected from the group consisting of Annexin V and phosphatidylserine. Exosomes can include proteins and nucleic acids, such as mRNA, miRNA, ncRNA. Microvesicles can include proteins and nucleic acids (e.g., mRNA, miRNA, ncRNA). Apoptotic bodies are thought to contain fragmented nuclei and organelles.

As used herein, "extract" means an extracted product and wherein a particular component is more concentrated than prior to extraction.

As used herein, "extract of urine" means an extract extracted from urine in which a particular component (particularly microRNA) is more concentrated than in urine prior to extraction. Extracts of urine may be aqueous solutions (solutions or suspensions) or may be solids obtained by drying them. In urine extracts, extracts from which components other than the extracellular vesicles and nucleic acids in the urine have been substantially removed may also be referred to as urine purifications. The extract of urine may comprise a surfactant (preferably a nonionic surfactant). Urinary extracts may include detergents and debris of extracellular vesicles (e.g., exosomes and/or microvesicles). The extract of urine may be one or more free or substantially free of 1 or more selected from the group consisting of surfactants and debris (debris) of extracellular vesicles (e.g., exosomes and/or microvesicles). The extract of urine may further comprise a stabilizing agent (e.g., a stabilizing agent of a nucleic acid) and/or a pH adjusting agent (e.g., a buffering agent). The extract of urine may contain salt. The urine extract may comprise one or more urine components selected from the group consisting of urine components, e.g., urea, creatinine, uric acid, ammonia, urobilin, riboflavin, urinary protein, sugar, and urinary hormones (e.g., chorionic gonadotropin) (each independently may be 50% or less, 40% or less, 30% or less, 20% or less, 10% or less, 5% or less, 4% or less, 3% or less, 2% or less, 1×10-1% or less, 1×10⁻²% or less, or 1×10⁻³% or less of the concentration in urine. The pH of the urine extract may be greater than or greater than a value such as 2, 3, 4, or 5. The pH of the urine extract may be less than or less than a value such as 10,9,8,7,6, or 5. In the present disclosure, a urine extract comprises a microRNA. In the present disclosure, a urine extract may comprise a concentrated microRNA or a group thereof. In the present disclosure, urine extracts may comprise microRNAs extracted by the extraction methods described herein. In the present disclosure, a urine extract may comprise at least one or all of the microRNAs listed in Data S1 (or Table 3 disclosing Data S1). In the present disclosure, urine extracts can be those obtained by contacting urine with nanowires (e.g., nanowires having at least one surface selected from the group consisting of ZnO, SiO₂, Li₂O, MgO, Al₂O₃, CaO, TiO₂, Mn₂O₃, Fe₂O₃, CoO, NiO, CuO, Ga₂O₃, SrO, In₂O₃, SnO₂, Sm₂O₃, and EuO) having a positively charged surface in a urine pH environment, followed by cleaning as needed, and then extracting with a buffer containing a nonionic surfactant or the like (the urine extract thus obtained may be referred to as an "extract of urine of the present disclosure). Urine may also be adjusted in pH so that the surface charge of the nanowire is positive when contacting the nanowire with urine (before, after, or during contact). Urine extracts are suitable for nucleic acid analysis.

As used herein, "in situ extraction" means disrupting EVs captured on nanowires using nanowire-incorporated microfluidic devices to extract small molecule RNAs (e.g., microRNAs) in situ, or extracting small molecule RNAs (e.g., microRNAs) captured on nanowires into solutions from nanowires.

As used herein, "free" when used in the context of a form of microRNA present in urine means that the microRNA is not contained in an extracellular vesicle and is present in a state where the microRNA is not associated with the extracellular vesicle. As used herein, "inclusion" when used in the context of a form of presence of microRNA in urine means that the microRNA is incorporated into an extracellular vesicle (either fully or partially inclusive).

As used herein, "nanowire" generally means a rod-like structure having a size (e.g., a diameter of 1 to several hundred nanometers) such as a cross-sectional shape or diameter on the order of nanometers. The size of the nanowires is not particularly limited, for example, 1nm, 5nm, 10nm, 20nm, 20nm, 30nm, 50nm, 60nm, 70nm, 90nm, 100nm, 105nm, 110nm, 120nm, 130nm, 145nm, 150nm, 160nm, 170nm, 180nm, 185nm, 190nm, 200nm, 210nm, 220nm,Is it 230nm, 250nm, 260nm, 270nm, 280nm, 300nm, 320nm, 330nm, 340nm, 360nm, 370nm, 390nm, 410nm, 420nm, 440nm, 460nm, 470nm, 480nm, or 490nm? It may be larger than one lower limit value selected from the numerical group. The size of the nanowires is also not particularly limited, for example, 1000nm, 990nm, 980nm, 970nm, 960nm, 930nm, 920nm, 910nm, 900nm, 890nm, 880nm, 870nm, 860nm, 850nm, 840nm, 820nm, 810nm, 800nm, 790nm, 780nm, 770nm, 760nm, 750nm, 740nm, 730nm, 720nm, 710nm, 700nm, 690nm, 680nm, 670nm, 660nm, 650nm, 640nm, 560nm, 550nm, 550nm It may be 520nm, 510nm or 500nm, or it may be smaller than one lower limit value selected from the above numerical group. The size of the nanowire is not particularly limited, and may be any size between the upper limit and the lower limit, for example. In the devices of the present disclosure, the use of nanowires can increase surface area, which can increase the recovery capacity of EVs. The length of the nanowire is not particularly limited, and may be any length between two values selected from 500nm, 600nm, 700nm, 800nm, 900nm, 1µm, 2µm, 3µm, 4µm, 5µm, 6µm, 7µm, 8µm, 9µm, and 10µm, for example. The length and diameter of the nanowires can affect the physical strength and surface area of the nanowires. The length and diameter could be adjusted to suit the environment of use. The nanowires are, in some embodiments, 1µm to 10µn long (e.g., 1.5µm to 5µm long) and are made of metal oxide (e.g., zinc oxide).

As used herein, "free" means substantially free or free of the ingredients when used in combination with the ingredients described immediately prior to this term.

Here, "substantially free" does not exclude the inclusion of a level of the component that cannot be removed in the extract.

The present inventors have found that extracellular vesicles (EVs) (and free miRNAs) in urine are efficiently adsorbed on nanowires without destruction by bringing nanowires with a positively charged surface (for example, a zinc oxide (ZnO) surface) into contact with the urine of a subject at pH 6 to 8. The present inventors have also found that EVs and miRNAs adsorbed on nanowires can be effectively collected using a surfactant.

The present disclosure provides an extract of urine containing any one or more microRNAs listed in data S1 (or Table 3). The present disclosure provides an extract of urine containing all microRNAs listed in the data S1 (or Table 3). The present disclosure provides an extract of urine containing any one or more microRNAs listed in Table 2. The present disclosure provides an extract of urine containing all microRNAs listed in Table 2.

In one embodiment of the present disclosure, an extract of urine containing any one or more or all microRNAs listed in the data S1 (or Table 3) may contain 500 or more types, 600 or more types, 700 or more types, 800 or more types, 900 or more types, 1000 or more types, or 1100 or more types of microRNAs (particularly microRNAs present in urine). In one embodiment, an extract of urine containing any one or more or all microRNAs listed in the data S1 (or Table 3) may contain 749 or more types, 822 or more types, or 1111 or more types of microRNAs (particularly microRNAs present in urine).

In one embodiment of the present disclosure, 500 or more types, 600 or more types, 700 or more types, 800 or more types, 900 or more types, 1000 or more types, or 1100 or more types of microRNAs present in urine may be contained. In some embodiments, the number of types of microRNAs contained in urine may be the number of types of microRNAs actually contained. In some embodiments, the number of types of microRNAs contained in urine may be defined by a method or technique for detecting microRNAs. For example, the number of types of microRNAs contained in urine may depend on the detection limit of the method for detecting microRNAs. In one embodiment of the disclosure of the present invention, preferably, they may be prepared from urine using a nanowire-embedded device according to the present disclosure.

In one embodiment of the present disclosure, a microRNA may include at least one or all microRNAs selected from the group consisting of microRNAs that have values of 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, and 16 or more in the data S1 (or Table 3) (values log2-transformed after the background intensity is subtracted). In one embodiment of the present disclosure, a microRNA may include at least one or all microRNAs selected from the group consisting of microRNAs that have values of 1 or more and less than 2, 2 or more and less than 3, 3 or more and less than 4, 4 or more and less than 5, 5 or more and less than 6, 6 or more and less than 7, 7 or more and less than 8, 8 or more and less than 9, 9 or more and less than 10, 10 or more and less than 11, 11 or more and less than 12, 12 or more and less than 13, 13 or more and less than 14, 14 or more and less than 15, and 16 or more in the data S1 (or Table 3) (values log2-transformed after the background intensity is subtracted). In this embodiment, these values may be values of non-cancer donors (for example, healthy persons), values of lung cancer patients, values of pancreatic cancer patients, values of liver cancer patients, values of bladder cancer patients, values of prostate cancer patients, values of gastric cancer patients, values of urothelial carcinoma patients, values of melanoma patients, values of ovarian cancer patients, values of thyroid cancer patients, values of cervical cancer patients, values of colorectal cancer patients, and /or values of endometrial cancer patients. In this embodiment, the values may be, for example, values of gastrointestinal cancer patients, values of hematopoietic cancer patients, and /or values of urological cancer patients. In this embodiment, the values may be values of all the cancer patients.

The present disclosure provides an extract of urine containing at least one or all microRNAs selected from the group consisting of miR-3117-5p, miR-3118, miR-3121-3p, miR-3121-5p, miR-3126-5p, miR-3128, miR-3133, miR-3134, miR-3136-3p, miR-3136-5p, miR-3139, miR-3142, miR-3143, miR-3145-3p, miR-3163, miR-3166, miR-3167, miR-16-1-3p, miR-424-3p, miR-519c-5p, miR-525-5p, miR-551b-5p, miR-558, miR-921, miR-942-3p, miR-3126-3p, miR-3127-5p, miR-3129-5p, miR-3144-5p, miR-3150a-5p, miR-3152-5p, miR-3155a, miR-3157-3p, miR-3159, miR-3165, miR-3678-3p, miR-4321, miR-4521, miR-4800-3p, miR-4999-5p, miR-5096, miR-5187-5p, miR-6874-5p, miR-3127-3p, miR-3130-5p, miR-3131, miR-3141, miR-3150b-5p, miR-3151-3p, miR-3151-5p, miR-3154, miR-3160-3p, miR-3160-5p, miR-378a-5p, miR-520c-3p, miR-526b-3p, miR-3150a-3p, miR-3162-5p, and miR-4254. The present disclosure provides an extract of urine containing at least one microRNA or all microRNAs selected from the group consisting of miR-3163, miR-16-1-3p, miR-424-3p, miR-558, miR-3127-5p, and miR-4521. The present disclosure provides an extract of urine containing at least one microRNA or all microRNAs selected from the group consisting of miR-378a-5p, miR-520c-3p, and miR-526b-3p. These microRNAs may be detected in the urine of a lung cancer patient. Thus, according to the present disclosure, the urine may be the urine of a subject having lung cancer.

The present disclosure provides an extract of urine containing at least one or all microRNAs selected from the group consisting of let-7i-3p, miR-183-5p, miR-202-5p, miR-409-5p, miR-4661-5p, miR-4800-3p, miR-5587-5p, miR-372-3p, miR-378b, miR-520b, miR-1266-3p, miR-3605-5p, miR-3612, miR-4645-3p, miR-4694-3p, miR-4752, miR-6816-3p, miR-8087, let-7f-2-3p, miR-15a-3p, miR-20a-3p, miR-33b-3p, miR-34c-5p, miR-93-5p, miR-130a-5p, miR-135a-5p, miR-135b-5p, miR-185-5p, miR-203a-3p, miR-302d-5p, miR-337-3p, miR-378c, miR-422a, miR-449c-5p, miR-483-5p, miR-506-3p, miR-511-5p, miR-520c-3p, miR-654-3p, miR-668-5p, miR-670-5p, miR-671-3p, miR-744-3p, miR-1178-3p, miR-1254, miR-1284, miR-1323, miR-2116-5p, miR-2355-3p, miR-3132, miR-3138, miR-3164, miR-3186-3p, miR-3189-3p, miR-3198, miR-3200-5p, miR-3657, miR-3667-5p, miR-3680-5p, miR-3692-5p, miR-3713, miR-3921, miR-3936, miR-4273, miR-4299, miR-4306, miR-4316, miR-4319, miR-4421, miR-4429, miR-4435, miR-4441, miR-4473, miR-4506, miR-4633-5p, miR-4658, miR-4733-5p, miR-4733-3p, miR-5004-3p, miR-5194, miR-5197-5p, miR-5571-5p, miR-6083, miR-6717-5p, miR-6720-5p, miR-6767-3p, miR-6781-3p, miR-6811-3p, miR-6821-3p, miR-6828-5p, miR-6832-5p, miR-6837-3p, miR-6841-5p, miR-6853-5p, miR-6871-3p, miR-6875-5p, miR-6878-5p, miR-7112-3p, miR-7703, miR-7848-3p, and miR-7856-5p. The present disclosure provides an extract of urine containing at least one or all microRNAs selected from the group consisting of miR-183-5p, miR-202-5p, and miR-409-5p. The present disclosure provides an extract of urine containing at least one or all microRNAs selected from the group consisting of miR-372-3p, miR-520b, miR-15a-3p, miR-34c-5p, miR-135a-5p, miR-185-5p, miR-337-3p, miR-422a, miR-506-3p, miR-520c-3p, miR-1284, miR-1323, and miR-4273. These microRNAs may be detected in the urine of a pancreatic cancer patient. Thus, according to the present disclosure, the urine may be the urine of a subject having pancreatic cancer.

The present disclosure provides an extract of urine containing at least one or all microRNAs selected from the group consisting of miR-4521, let-7c-3p, let-7i-5p, miR-16-1-3p, miR-26a-1-3p, miR-28-5p, miR-105-5p, miR-195-3p, miR-200b-5p, miR-219a-2-3p, miR-297, miR-300, miR-330-3p, miR-374b-5p, miR-431-5p, miR-454-5p, miR-513c-5p, miR-548ax, miR-593-5p, miR-623, miR-664a-5p, miR-942-3p, miR-1205, miR-1276, miR-1288-3p, miR-1297, miR-3678-3p, miR-4283, miR-4295, miR-4439, miR-4524b-5p, miR-4703-3p, miR-4768-5p, miR-4800-3p, miR-5187-5p, miR-5696, miR-7161-5p, 1et-7i-2-3p, and miR-520c-3p.

The present disclosure provides an extract of urine containing at least one or all microRNAs selected from the group consisting of miR-16-1-3p, miR-28-5p, miR-297, miR-300, miR-330-3p, miR-454-5p, miR-1297, and miR-4295. The present disclosure provides an extract of urine containing miR-520c-3p. These microRNAs may be detected in the urine of a subject having liver cancer. Thus, the urine may be the urine of a subject having liver cancer.

The present disclosure provides an extract of urine containing at least one or all microRNAs selected from the group consisting of miR-16-1-3p, miR-23b-3p, miR-28-5p, miR-92a-2-5p, miR-142-3p, miR-195-3p, miR-196b-5p, miR-299-3p, miR-492, miR-513b-5p, miR-601, miR-619-5p, miR-1285-3p, miR-3155a, miR-3162-5p, miR-3678-3p, miR-4283, miR-4295, miR-4311, miR-4531, miR-5096, miR-5187-5p, let-7f-2-3p, miR-520c-3p, and miR-4783-5p. The present disclosure provides an extract of urine containing at least one or all microRNAs selected from the group consisting of miR-16-1-3p, miR-23b-3p, miR-28-5p, miR-142-3p, miR-195-3p, miR-299-3p, and miR-4295. The present disclosure provides an extract of urine containing miR-520c-3p. These microRNAs may be detected in an embodiment having bladder cancer. Thus, in the present disclosure, the urine may be the urine of a subject having bladder cancer.

The present disclosure provides an extract of urine containing at least one or all microRNAs selected from the group consisting of miR-4531, miR-28-5p, miR-103a-2-5p, miR-105-5p, miR-124-3p, miR-151a-5p, miR-151b, miR-200a-5p, miR-300, miR-424-3p, miR-519c-5p, miR-551b-5p, miR-617, miR-873-3p, miR-921, miR-1288-3p, miR-3124-5p, miR-3155a, miR-3917, miR-4283, miR-4727-3p, miR-5096, miR-5187-5p, miR-6074, miR-6874-5p, miR-6892-5p, miR-15a-3p, miR-135b-5p, miR-520c-3p, miR-4783-5p, and miR-7849-3p. The present disclosure provides an extract of urine containing at least one or all microRNAs selected from the group consisting of miR-28-5p, miR-105-5p, miR-124-3p, miR-151a-5p, and miR-300. The present disclosure provides an extract of urine containing at least one or all microRNAs selected from the group consisting of miR-15a-3p and miR-520c-3p. These microRNAs may be detected in an embodiment having prostate cancer. Thus, in the present disclosure, the urine may be the urine of a subject having prostate cancer.

Another aspect of the present disclosure provides a method for examining the likelihood that a subject has cancer. The method for examining the likelihood of cancer can be replaced and interchangeable with a method for diagnosing cancer, a method for acquiring preliminary information for diagnosing cancer, a method for determining whether a subject has cancer cells in the body, a method for detecting cancer cells, a method for predicting the likelihood of cancer, or a method for determining the likelihood that a subject has cancer. In the present disclosure, if it is determined that there is a likelihood that a subject has cancer by a method for examining the likelihood that a subject has cancer, then a doctor or the like can make a definitive diagnosis. In the present disclosure, there is provided a method according to the present disclosure including diagnosing cancer and performing cancer therapy (for example, radiotherapy, chemotherapy, immunological therapy, and /or surgery) on a patient diagnosed with cancer.

In the present disclosure, the likelihood that a subject has cancer can be determined using the expression level of any of the microRNAs listed in the data S1 (or Table 3) in a body fluid sample as an indicator.

The present disclosure provides an extract of urine containing any one or more microRNAs listed in any of Tables 4-1 to 4-15. The present disclosure provides an extract of urine containing all microRNAs listed in any of Tables 4-1 to 4-15. The present disclosure provides an extract of urine containing any one or more microRNAs listed in any of Tables 4-1 to 4-15. The present disclosure provides an extract of urine containing all microRNAs listed in any of Tables 4-1 to 4-15. The microRNAs may be microRNAs increased in a cancer patient. Thus, in one embodiment, a microRNA detected in an extract of urine may indicate that a subject from which the urine is derived has cancer or a likelihood of the subject having cancer.

The present disclosure provides a method of analyzing urine including obtaining an extract of urine. A method of analyzing urine according to the present disclosure may further include extracting a microRNA from an extract of urine. Whether an extract of urine contains a specific microRNA can be determined by a method of analyzing urine according to the present disclosure. A method of analyzing urine according to the present disclosure may further include measuring the amount of specific microRNA when an extracted microRNA includes the specific microRNA. In a method of analyzing urine according to the present disclosure, the specific microRNA may be any one or more microRNAs listed in any of Tables 4-1 to 4-15. In a method of analyzing urine according to the present disclosure, the specific microRNA may be any one or more microRNAs listed in any of Tables 4-1 to 4-15 and may have a p-value of less than 0.005. In a method of analyzing urine according to the present disclosure, the specific microRNA may be any one or more microRNAs listed in any of Tables 22-1 to 22-13. The specific microRNA may be any one or more microRNAs listed in any of Tables 22-1 to 22-13 having an accuracy of higher than 50%, 55% or higher, 60% or higher, 65% or higher, 70% or higher, 75% or higher, 80% or higher, 85% or higher, 90% or higher, or 95% or higher. The specific microRNA may be any one or more microRNAs listed in any of Tables 22-1 and 23-1 to 23-14 preferably having an accuracy of higher than 50%, 55% or higher, 60% or higher, 65% or higher, 70% or higher, 75% or higher, 80% or higher, 85% or higher, 90% or higher, or 95% or higher. The specific microRNA may be any one or more microRNAs listed in any of Tables 24-1 to 24-15 possibly having an accuracy of higher than 0.5, 0.55 or higher, 0.6 or higher, 0.65 or higher, 0.7 or higher, 0.75 or higher, 0.8 or higher, 0.85 or higher, or 0.9 or higher. The specific microRNA may be any one or more microRNAs listed in any of Tables 25-1 to 25-15 possibly having an accuracy of higher than 0.5, 0.55 or higher, 0.6 or higher, 0.65 or higher, 0.7 or higher, 0.75 or higher, 0.8 or higher, 0.85 or higher, or 0.9 or higher. The specific microRNA may include 5 types, 10 types, 15 types, 20 types, 30 types, 40 types, 50 types, 60 types, 70 types, 80 types, 90 types, 100 types, 200 types, 300 types, 400 types, 500 or more types, 600 or more types, 700 or more types, 800 or more types, 900 or more types, 1000 or more types, 1100 or more types, 1200 or more types, 1300 or more types, 1400 or more types, 1500 or more types, 1600 or more types, 1700 or more types, 1800 or more types, 1900 or more types, 2000 or more types, 2100 or more types, 2200 or more types, 2300 or more types, 2400 or more types, or 2500 or more types of microRNAs. The specific microRNA can be detected or quantified by various known methods. The specific microRNA can be identified by contacting the microRNA with a nucleic acid containing a sequence that can hybridize to the microRNA. A nucleic acid containing a sequence that can hybridize to a microRNA can be solid-phased. A nucleic acid containing a sequence that can hybridize to a microRNA may be solid-phased on a microarray. A nucleic acid containing a sequence that can hybridize to a microRNA may be a probe for RT-PCR.

In one embodiment of the present disclosure, an extract of urine containing any one or more or all microRNAs listed in any of Tables 4-1 to 4-15 may contain 5 types, 10 types, 15 types, 20 types, 30 types, 40 types, 50 types, 60 types, 70 types, 80 types, 90 types, 100 types, 200 types, 300 types, 400 types, 500 or more types, 600 or more types, 700 or more types, 800 or more types, 900 or more types, 1000 or more types, 1100 or more types, 1200 or more types, 1300 or more types, 1400 or more types, 1500 or more types, 1600 or more types, 1700 or more types, 1800 or more types, 1900 or more types, 2000 or more types, 2100 or more types, 2200 or more types, 2300 or more types, 2400 or more types, or 2500 or more types of microRNAs (particularly microRNAs present in urine). In one embodiment of the present disclosure, an extract of urine containing any one or more or all microRNAs listed in any of Tables 4-1 to 4-15 may contain microRNAs (particularly microRNAs present in urine) corresponding to a little more than 50%, 60% or more, 70% or more, 80% or more, 90% or more, or 100% of the number of types of microRNAs listed in the respective tables. In one embodiment, an extract of urine containing any one or more or all microRNAs listed in Table 1 may contain 749 or more types, 822 or more types, or 1111 or more types of microRNAs (particularly microRNAs present in urine). The microRNAs may be microRNAs increased in a cancer patient. Thus, in one embodiment, a microRNA detected in an extract of urine may indicate that a subject from which the urine is derived has cancer or a likelihood thereof.

In one embodiment of the present disclosure, an extract of urine containing any one or more or all microRNAs listed in any of Tables 4-1 to 4-15 may contain 500 or more types, 600 or more types, 700 or more types, 800 or more types, 900 or more types, 1000 or more types, 1100 or more types, 1200 or more types, 1300 or more types, 1400 or more types, 1500 or more types, 1600 or more types, 1700 or more types, 1800 or more types, 1900 or more types, 2000 or more types, 2100 or more types, 2200 or more types, 2300 or more types, 2400 or more types, or 2500 or more types of microRNAs present in urine. In some embodiments, the number of types of microRNAs contained in urine may be the number of types of microRNAs actually contained. In some embodiments, the number of types of microRNAs contained in urine may be defined by a method or technique for detecting microRNAs. For example, the number of types of microRNAs contained in urine may depend on the detection limit of the method for detecting microRNAs. In one embodiment of the disclosure of the present invention, preferably, they may be prepared from urine using a nanowireembedded device according to the present disclosure.

In one embodiment of the present disclosure, among the microRNAs listed in Table 4-1, a microRNA may include at least one or all microRNAs selected from the group consisting of microRNAs having a sensitivity and /or specificity value of more than 50%, more than 55%, more than 60%, more than 65%, more than 70%, more than 75%, more than 80%, more than 85%, more than 90%, or more than 95% in Table 4-1. In one embodiment of the present disclosure, among the microRNAs listed in Table 4-1, a microRNA may include at least one or all microRNAs selected from the group consisting of microRNAs having a sensitivity and /or specificity value of more than 50% and 55% or less, more than 55% and 60% or less, more than 60% and 65% or less, more than 65% and 70% or less, more than 70% and 75% or less, more than 75% and 80% or less, more than 80% and 85% or less, more than 85% and 90% or less, more than 90% and 95% or less, or more than 95% in Table 4-1. The microRNAs may be microRNAs increased in a cancer patient. Thus, in one embodiment, a microRNA detected in an extract of urine may indicate that a subject from which the urine is derived has cancer or a likelihood thereof.

Whether the specific microRNA has a higher or lower expression level in the case of a specific cancer than in a healthy person can be determined based on whether the specific microRNA has a higher or lower expression level in the case of the specific cancer than in a healthy person in Tables 4-1 to 4-15.

In the present invention, a microRNA in an extract of urine is in an amount that can be or is sufficient to be detected by a microarray.

The present disclosure provides an extract of urine containing at least one type, two types, three types, four types, five types, six types, seven types, eight types, nine types, or ten types of or all microRNAs selected from the group consisting of hsa-let-7a-3p, hsa-miR-103a-2-5p, hsa-miR-103a-3p, hsa-miR-105-3p, hsa-miR-106a-3p, hsa-miR-1180-3p, hsa-miR-1185-2-3p, hsa-miR-1193, hsa-miR-127-3p, hsa-miR-1245b-5p, hsa-miR-1247-3p, hsa-miR-125b-2-3p, hsa-miR-1263, hsa-miR-173g-3p, hsa-miR-129-2-3p, hsa-miR-1293, hsa-miR-1301-5p, hsa-miR-130a-3p, hsa-miR-130a-5p, hsa-miR-1469, hsa-miR-149-5p, hsa-miR-152-5p, hsa-miR-15b-3p, hsa-miR-16-1-3p, hsa-miR-181a-3p, hsa-miR-184, hsa-miR-186-5p, hsa-miR-18a-5p, hsa-miR-193b-3p, hsa-miR-200a-3p, hsa-miR-200c-5p, hsa-miR-208b-5p, hsa-miR-216a-3p, hsa-miR-7b-5p, hsa-miR-2861, hsa-miR-2909, hsa-miR-298, hsa-miR-29a-5p, hsa-miR-302b-3p, hsa-miR-30b-3p, hsa-miR-30d-5p, hsa-miR-3116, hsa-miR-3122, hsa-miR-3125, hsa-miR-3126-3p, hsa-miR-3129-3p, hsa-miR-3130-3p, hsa-miR-3133, hsa-miR-3136-3p, hsa-miR-3137, hsa-miR-3143, hsa-miR-3150a-3p, hsa-miR-3174, hsa-miR-3177-5p, hsa-miR-3179, hsa-miR-3180, hsa-miR-3186-3p, hsa-miR-3186-5p, hsa-miR-3187-3p, hsa-miR-3189-3p, hsa-miR-3190-3p, hsa-miR-3190-5p, hsa-miR-3194-5p, hsa-miR-3195, hsa-miR-3197, hsa-miR-3198, hsa-miR-3200-5p, hsa-miR-320a, hsa-miR-320c, hsa-miR-320d, hsa-miR-323b-5p, hsa-miR-324-3p, hsa-miR-325, hsa-miR-32-5p, hsa-miR-329-3p, hsa-miR-331-3p, hsa-miR-331-5p, hsa-miR-335-3p, hsa-miR-337-3p, hsa-miR-337-5p, hsa-miR-33a-3p, hsa-miR-33a-5p, hsa-miR-342-3p, hsa-miR-345-5p, hsa-miR-346, hsa-miR-34a-5p, hsa-miR-3606-3p, hsa-miR-3607-5p, hsa-miR-3613-3p, hsa-miR-3613-5p, hsa-miR-361-3p, hsa-miR-3616-3p, hsa-miR-3616-Sp, hsa-miR-3621, hsa-miR-3622a-5p, hsa-miR-363-3p, hsa-miR-3658, hsa-miR-3659, hsa-miR-367-3p, hsa-miR-3674, hsa-miR-3678-3p, hsa-miR-3681-5p, hsa-miR-3683, hsa-miR-3684, hsa-miR-3690, hsa-miR-374c-5p, hsa-miR-376a-3p, hsa-miR-376a-5p, hsa-miR-376b-3p, hsa-miR-384, hsa-miR-3913-3p, hsa-miR-3940-3p, hsa-miR-3960, hsa-miR-3976, hsa-miR-4269, hsa-miR-477, hsa-miR-4302, hsa-miR-4303, hsa-miR-4304, hsa-miR-4307, hsa-miR-4316, hsa-miR-4319, hsa-miR-432-3p, hsa-miR-432-5p, hsa-miR-4326, hsa-miR-4421, hsa-miR-4433b-5p, hsa-miR-4456, hsa-miR-4472, hsa-miR-4479, hsa-miR-4521, hsa-miR-4639-5p, hsa-miR-4646-5p, hsa-miR-4660, hsa-miR-4662a-3p, hsa-miR-4688, hsa-miR-4694-3p, hsa-miR-4695-3p, hsa-miR-4695-5p, hsa-miR-4699-3p, hsa-miR-4701-5p, hsa-miR-4704-3p, hsa-miR-4716-5p, hsa-miR-4719, hsa-miR-4721, hsa-miR-4724-5p, hsa-miR-4736, hsa-miR-4738-3p, hsa-miR-4740-3p, hsa-miR-4750-5p, hsa-miR-4753-5p, hsa-miR-4759, hsa-miR-4776-5p, hsa-miR-4778-3p, hsa-miR-4793-5p, hsa-miR-4796-5p, hsa-miR-4799-3p, hsa-miR-4804-5p, hsa-miR-487b-5p, hsa-miR-497-5p, hsa-miR-5008-3p, hsa-miR-5011-5p, hsa-miR-509-5p, hsa-miR-514a-3p, hsa-miR-514b-5p, hsa-miR-515-3p, hsa-miR-518f-3p, hsa-miR-5194, hsa-miR-525-3p, hsa-miR-541-3p, hsa-miR-548aa, hsa-miR-548t-3p, hsa-miR-548ae-3p, hsa-miR-548c-3p, hsa-miR-548c-5p, hsa-miR-548o-5p, hsa-miR-548am-5p, hsa-miR-548f-5p, hsa-miR-548j-3p, hsa-miR-548n, hsa-miR-548v, hsa-miR-548x-3p, hsa-miR-548z, hsa-miR-548h-3p, hsa-miR-549a, hsa-miR-5571-5p, hsa-miR-5572, hsa-miR-5580-5p, hsa-miR-5586-3p, hsa-miR-5586-5p, hsa-miR-561-3p, hsa-miR-571, hsa-miR-574-3p, hsa-miR-578, hsa-miR-582-3p, hsa-miR-593-5p, hsa-miR-598-3p, hsa-miR-601, hsa-miR-606, hsa-miR-6068, hsa-miR-6077, hsa-miR-6083, hsa-miR-6089, hsa-miR-6090, hsa-miR-6132, hsa-miR-6133, hsa-miR-616-5p, hsa-miR-617, hsa-miR-620, hsa-miR-622, hsa-miR-623, hsa-miR-625-5p, hsa-miR-626, hsa-miR-636, hsa-miR-642b-5p, hsa-miR-649, hsa-miR-6504-5p, hsa-miR-6508-3p, hsa-miR-6511b-3p, hsa-miR-6513-5p, hsa-miR-651-5p, hsa-miR-6516-5p, hsa-miR-652-3p, hsa-miR-661, hsa-miR-664a-3p, hsa-miR-664a-5p, hsa-miR-665, hsa-miR-671-3p, hsa-miR-6715b-3p, hsa-miR-6720-5p, hsa-miR-6721-5p, hsa-miR-6726-3p, hsa-miR-6734-3p, hsa-miR-6735-5p, hsa-miR-6742-3p, hsa-miR-6755-3p, hsa-miR-6760-3p, hsa-miR-6767-5p, hsa-miR-6777-5p, hsa-miR-6782-5p, hsa-miR-6890-3p, hsa-miR-8084, hsa-miR-888-3p, hsa-miR-92b-5p, hsa-miR-935, hsa-miR-93-5p, and hsa-miR-942-3p.

The present disclosure provides an extract of urine containing at least one type, two types, three types, four types, five types, six types, seven types, eight types, nine types, or ten types of or all microRNAs selected from the group consisting of hsa-miR-103a-3p, hsa-miR-1193, hsa-miR-127-3p, hsa-miR-1247-3p, hsa-miR-1263, hsa-miR-173g-3p, hsa-miR-129-2-3p, hsa-miR-1293, hsa-miR-130a-5p, hsa-miR-1469, hsa-miR-15b-3p, hsa-miR-193b-3p, hsa-miR-2861, hsa-miR-298, hsa-miR-30d-5p, hsa-miR-3122, hsa-miR-3137, hsa-miR-3174, hsa-miR-3177-5p, hsa-miR-3186-3p, hsa-miR-3187-3p, hsa-miR-3189-3p, hsa-miR-3190-5p, hsa-miR-3194-5p, hsa-miR-3198, hsa-miR-320c, hsa-miR-329-3p, hsa-miR-337-3p, hsa-miR-337-5p, hsa-miR-33a-3p, hsa-miR-345-5p, hsa-miR-346, hsa-miR-34a-5p, hsa-miR-3613-3p, hsa-miR-361-3p, hsa-miR-3616-3p, hsa-miR-3622a-5p, hsa-miR-363-3p, hsa-miR-3659, hsa-miR-3678-3p, hsa-miR-3681-5p, hsa-miR-374c-5p, hsa-miR-3960, hsa-miR-3976, hsa-miR-4269, hsa-miR-4304, hsa-miR-4307, hsa-miR-432-3p, hsa-miR-4433b-5p, hsa-miR-4456, hsa-miR-4472, hsa-miR-4479, hsa-miR-4521, hsa-miR-4646-5p, hsa-miR-4694-3p, hsa-miR-4699-3p, hsa-miR-4701-5p, hsa-miR-4719, hsa-miR-4721, hsa-miR-4724-5p, hsa-miR-4776-5p, hsa-miR-4796-5p, hsa-miR-4804-5p, hsa-miR-5011-5p, hsa-miR-509-5p, hsa-miR-515-3p, hsa-miR-5571-5p, hsa-miR-5580-5p, hsa-miR-574-3p, hsa-miR-578, hsa-miR-582-3p, hsa-miR-598-3p, hsa-miR-6068, hsa-miR-6077, hsa-miR-6089, hsa-miR-6133, hsa-miR-616-5p, hsa-miR-622, hsa-miR-626, hsa-miR-636, hsa-miR-6504-5p, hsa-miR-6516-5p, hsa-miR-661, hsa-miR-664a-3p, hsa-miR-6715b-3p, hsa-miR-6720-5p, hsa-miR-6734-3p, hsa-miR-6742-3p, hsa-miR-6760-3p, hsa-miR-6767-5p, hsa-miR-6777-5p, hsa-miR-6890-3p, and hsa-miR-93-5p.

The present disclosure provides an extract of urine containing at least one type, two types, three types, four types, five types, six types, seven types, eight types, nine types, or ten types of or all microRNAs selected from the group consisting of hsa-miR-103a-2-5p, hsa-miR-1193, hsa-miR-3622a-5p, hsa-miR-363-3p, hsa-miR-4521, hsa-miR-4796-5p, hsa-miR-518f-3p, hsa-miR-5580-5p, hsa-miR-3125, hsa-miR-3130-3p, hsa-miR-3678-3p, hsa-miR-4750-5p, hsa-miR-5194, hsa-miR-578, hsa-miR-625-5p, hsa-miR-6755-3p, hsa-miR-1293, hsa-miR-3137, hsa-miR-4646-5p, hsa-miR-514b-5p, hsa-miR-598-3p, hsa-miR-200c-5p, hsa-miR-3150a-3p, hsa-miR-3186-3p, hsa-miR-3187-3p, hsa-miR-3189-3p, hsa-miR-4303, hsa-miR-320a, hsa-miR-4421, hsa-miR-515-3p, hsa-miR-665, hsa-miR-6767-5p, hsa-miR-6782-5p, hsa-miR-3659, hsa-miR-3690, hsa-miR-4721, hsa-miR-4776-5p, hsa-miR-622, hsa-miR-2909, hsa-miR-3200-5p, hsa-miR-323b-5p, hsa-miR-34a-5p, hsa-miR-4688, hsa-miR-3190-3p, hsa-miR-3613-3p, hsa-miR-4793-5p, hsa-miR-6083, hsa-miR-4316, hsa-miR-4738-3p, hsa-miR-5572, hsa-miR-661, hsa-miR-3116, hsa-miR-3621, hsa-miR-4326, hsa-miR-623, hsa-miR-6504-5p, hsa-miR-1301-5p, hsa-miR-487b-5p, hsa-miR-3126-3p, hsa-miR-4304, hsa-miR-3186-5p, hsa-miR-342-3p, hsa-miR-4695-3p, hsa-miR-5008-3p, hsa-miR-616-5p, hsa-miR-125b-2-3p, hsa-miR-4479, hsa-miR-4740-3p, hsa-miR-103a-3p, hsa-miR-181a-3p, hsa-miR-337-5p, hsa-miR-1263, hsa-miR-374c-5p, hsa-miR-1180-3p, hsa-miR-29a-5p, hsa-miR-509-5p, hsa-miR-15b-3p, hsa-miR-32-5p, hsa-miR-376a-3p, hsa-miR-4804-5p, hsa-miR-5011-5p, hsa-miR-582-3p, hsa-miR-320d, hsa-miR-130a-3p, hsa-miR-200a-3p, hsa-miR-4639-5p, hsa-miR-571, hsa-miR-33a-5p, hsa-miR-4719, hsa-miR-3681-5p, hsa-miR-4699-3p, hsa-miR-626, hsa-miR-105-3p, hsa-miR-384, hsa-miR-3976, hsa-miR-186-5p, hsa-miR-18a-5p, hsa-miR-3616-5p, hsa-miR-4302, hsa-miR-548c-5p, hsa-miR-548o-5p, hsa-miR-548am-5p, hsa-miR-548v, hsa-let-7a-3p, hsa-miR-3143, hsa-miR-367-3p, hsa-miR-376a-5p, hsa-miR-548ae-3p, hsa-miR-5586-5p, hsa-miR-6715b-3p, hsa-miR-548aa, hsa-miR-548t-3p, hsa-miR-652-3p, hsa-miR-3607-5p, hsa-miR-3683, hsa-miR-477, hsa-miR-4704-3p, hsa-miR-4799-3p, hsa-miR-548x-3p, hsa-miR-549a, and hsa-miR-6508-3p.

The present disclosure provides an extract of urine containing at least one type, two types, three types, four types, five types, six types, seven types, eight types, nine types, or ten types of or all microRNAs selected from the group consisting of hsa-miR-103a-2-5p, hsa-miR-106a-3p, hsa-miR-1185-2-3p, hsa-miR-1193, hsa-miR-1273g-3p, hsa-miR-1293, hsa-miR-1301-5p, hsa-miR-152-5p, hsa-miR-184, hsa-miR-200c-5p, hsa-miR-2909, hsa-miR-298, hsa-miR-302b-3p, hsa-miR-3116, hsa-miR-3122, hsa-miR-3125, hsa-miR-3126-3p, hsa-miR-3129-3p, hsa-miR-3130-3p, hsa-miR-3133, hsa-miR-3137, hsa-miR-3150a-3p, hsa-miR-3174, hsa-miR-3177-5p, hsa-miR-3179, hsa-miR-3186-3p, hsa-miR-3186-5p, hsa-miR-3187-3p, hsa-miR-3189-3p, hsa-miR-3190-3p, hsa-miR-3200-5p, hsa-miR-320a, hsa-miR-320c, hsa-miR-323b-5p, hsa-miR-325, hsa-miR-331-5p, hsa-miR-335-3p, hsa-miR-33a-3p, hsa-miR-342-3p, hsa-miR-34a-5p, hsa-miR-3606-3p, hsa-miR-3616-3p, hsa-miR-3621, hsa-miR-3622a-5p, hsa-miR-3659, hsa-miR-3690, hsa-miR-376b-3p, hsa-miR-3913-3p, hsa-miR-4269, hsa-miR-4303, hsa-miR-4304, hsa-miR-4307, hsa-miR-4316, hsa-miR-4326, hsa-miR-4421, hsa-miR-4472, hsa-miR-4479, hsa-miR-4646-5p, hsa-miR-4660, hsa-miR-4688, hsa-miR-4695-3p, hsa-miR-4695-5p, hsa-miR-4721, hsa-miR-4724-5p, hsa-miR-4738-3p, hsa-miR-4740-3p, hsa-miR-4750-5p, hsa-miR-4753-5p, hsa-miR-4759, hsa-miR-4776-5p, hsa-miR-4778-3p, hsa-miR-4796-5p, hsa-miR-487b-5p, hsa-miR-5008-3p, hsa-miR-514b-5p, hsa-miR-518f-3p, hsa-miR-5194, hsa-miR-541-3p, hsa-miR-548c-3p, hsa-miR-548j-3p, hsa-miR-548n, hsa-miR-548z, hsa-miR-548h-3p, hsa-miR-5572, hsa-miR-5580-5p, hsa-miR-5586-3p, hsa-miR-601, hsa-miR-6068, hsa-miR-6083, hsa-miR-6133, hsa-miR-617, hsa-miR-622, hsa-miR-625-5p, hsa-miR-649, hsa-miR-6504-5p, hsa-miR-6513-5p, hsa-miR-651-5p, hsa-miR-6516-5p, hsa-miR-661, hsa-miR-664a-5p, hsa-miR-665, hsa-miR-6755-3p, hsa-miR-6767-5p, hsa-miR-6777-5p, hsa-miR-6782-5p, hsa-miR-8084, hsa-miR-888-3p, and hsa-miR-942-3p. In this embodiment, the extract of urine may be an extract of the urine of a patient having a stage-I cancer. In a particular embodiment, the patient having a stage-I cancer may be a patient having stage-I lung cancer. These miRNAs are detected in a larger amount in the urine of a stage-I lung cancer patient than in the urine of a healthy person. The stage may be based on TNM classification, for example. In the TNM classification, the stage is judged from three perspectives: T: tumor size, N: the presence or absence of metastasis to lymph nodes, and M: metastasis to other organs. Doctors can classify cancers in accordance with the TNM classification. The term "early-stage cancer", as used herein, refers to a cancer selected from the group consisting of stage-I and -II cancers.

Another aspect of the present disclosure provides an extract of urine containing at least one type, two types, three types, four types, five types, six types, seven types, eight types, nine types, or ten types of or all microRNAs selected from the microRNAs listed in Table 4-2. The present disclosure also provides an extract of urine containing at least one type, two types, three types, four types, five types, six types, seven types, eight types, nine types, or ten types of or all microRNAs selected from the group consisting of hsa-miR-492, hsa-miR-1292-5p, hsa-miR-6789-5p, hsa-miR-1268b, hsa-miR-3648, hsa-miR-6781-5p, hsa-miR-4745-5p, hsa-miR-4651, hsa-miR-12120, hsa-miR-3937, hsa-miR-1908-5p, hsa-miR-10400-3p, hsa-miR-6125, hsa-miR-504-5p, hsa-miR-6791-5p, hsa-miR-6743-5p, hsa-miR-4530, hsa-miR-6798-5p, hsa-miR-4458, hsa-miR-1343-5p, hsa-miR-6081, hsa-miR-6724-5p, hsa-miR-8072, hsa-miR-4763-3p, hsa-miR-320c, hsa-miR-4321, hsa-miR-4673, hsa-miR-92a-2-5p, hsa-miR-184, hsa-miR-1469, hsa-miR-193b-5p, hsa-miR-4497, hsa-miR-4749-5p, hsa-miR-6821-5p, hsa-miR-3124-5p, hsa-miR-106a-3p, hsa-miR-638, hsa-miR-6732-5p, hsa-miR-92b-5p, hsa-miR-135a-3p, hsa-miR-6845-5p, hsa-miR-1227-5p, hsa-miR-6750-5p, hsa-miR-4707-5p, hsa-miR-20b-3p, hsa-miR-10392-3p, hsa-miR-3141, hsa-miR-4467, hsa-miR-3197, hsa-miR-4285, hsa-miR-4695-5p, hsa-miR-1915-3p, hsa-miR-4720-5p, hsa-miR-10396b-5p, hsa-miR-4258, hsa-miR-10396a-3p, hsa-miR-4454, hsa-miR-4507, hsa-miR-3162-5p, hsa-miR-6850-5p, hsa-miR-1236-5p, hsa-miR-4632-5p, hsa-miR-7108-5p, hsa-miR-4435, hsa-miR-6768-5p, hsa-miR-25-5p, hsa-miR-769-3p, hsa-miR-6752-5p, hsa-miR-3620-5p, hsa-miR-6787-5p, hsa-miR-6501-5p, hsa-miR-187-5p, hsa-miR-1294, hsa-miR-4505, hsa-miR-3691-5p, hsa-miR-371b-5p, hsa-miR-6501-3p, hsa-miR-10392-5p, hsa-miR-498-5p, hsa-miR-3180-3p, hsa-miR-937-5p, hsa-miR-885-3p, hsa-miR-1285-3p, hsa-miR-10401-5p, hsa-miR-6729-5p, hsa-miR-3187-3p, hsa-miR-211-3p, hsa-miR-6721-5p, hsa-miR-4460, hsa-miR-1912-3p, hsa-miR-4665-5p, hsa-miR-4785, hsa-miR-4758-5p, hsa-miR-193a-5p, hsa-miR-29b-3p, hsa-miR-138-5p, hsa-miR-9718, hsa-miR-10396a-5p, hsa-miR-1268a, hsa-miR-5100, hsa-miR-554, hsa-miR-4657, hsa-miR-760, hsa-miR-551b-5p, hsa-miR-5588-5p, hsa-miR-4655-5p, hsa-miR-4684-3p, hsa-miR-4506, hsa-miR-6805-5p, hsa-miR-766-5p, hsa-miR-663a, hsa-miR-1304-5p, hsa-miR-4741, hsa-miR-6515-5p, hsa-miR-5090, hsa-miR-4466, hsa-miR-6076, hsa-miR-6799-5p, hsa-miR-125b-1-3p, hsa-miR-3934-3p, hsa-miR-6820-5p, hsa-miR-3621, hsa-miR-8064, hsa-miR-1587, hsa-miR-4634, hsa-miR-6068, hsa-miR-4508, hsa-miR-4439, hsa-miR-4727-3p, hsa-miR-6075, hsa-miR-7851-3p, hsa-miR-5001-5p, hsa-miR-4443, hsa-miR-4654, hsa-miR-7854-3p, hsa-miR-604, hsa-miR-4449, hsa-miR-12121, hsa-miR-575, hsa-miR-4429, hsa-miR-6871-5p, hsa-miR-1909-3p, hsa-miR-6080, hsa-miR-6126, hsa-miR-3161, hsa-miR-1303, hsa-miR-650, hsa-miR-6722-3p, hsa-miR-1237-5p, hsa-miR-4512, hsa-miR-4450, hsa-miR-4314, hsa-miR-6071, hsa-miR-6513-5p, hsa-miR-5698, hsa-miR-6790-5p, hsa-miR-598-5p, hsa-miR-4489, hsa-miR-639, hsa-miR-6839-5p, hsa-miR-1225-5p, hsa-miR-6784-5p, hsa-miR-6809-5p, hsa-miR-4750-5p, hsa-miR-4433b-3p, hsa-miR-4479, hsa-miR-3652, hsa-miR-6793-5p, hsa-miR-124-5p, hsa-miR-4688, hsa-miR-6776-5p, hsa-miR-4648, hsa-miR-3195, hsa-miR-6860, hsa-miR-6838-5p, hsa-miR-4486, hsa-miR-12114, hsa-miR-664b-5p, hsa-miR-5703, hsa-miR-4736, hsa-miR-5189-5p, hsa-miR-617, hsa-miR-519a-3p, hsa-miR-4734, hsa-miR-6861-5p, hsa-miR-762, hsa-miR-8078, hsa-miR-744-5p, hsa-miR-6726-5p, hsa-miR-887-3p, hsa-miR-4754, hsa-miR-6132, hsa-miR-4640-5p, hsa-miR-4433a-3p, hsa-miR-323a-3p, hsa-miR-2115-5p, hsa-miR-3622a-5p, hsa-miR-297, hsa-miR-1293, hsa-miR-4289, hsa-miR-10394-3p, hsa-miR-4724-5p, hsa-miR-3178, hsa-miR-8063, hsa-miR-135a-2-3p, hsa-miR-3196, hsa-miR-5010-5p, hsa-miR-1226-5p, hsa-miR-6761-5p, hsa-miR-1281, hsa-miR-147b-3p, hsa-miR-3135b, hsa-miR-6770-3p, hsa-miR-202-3p, hsa-miR-378h, hsa-miR-6090, hsa-miR-6816-5p, hsa-miR-6747-5p, hsa-miR-516b-3p, hsa-miR-516a-3p, hsa-miR-6737-5p, hsa-miR-7150, hsa-miR-6840-3p, hsa-miR-4276, hsa-miR-6847-5p, hsa-miR-4498, hsa-miR-6875-5p, hsa-miR-4535, hsa-miR-608, hsa-miR-572, hsa-miR-3619-5p, hsa-miR-3663-3p, hsa-miR-4538, hsa-miR-4322, hsa-miR-6804-5p, hsa-miR-6855-5p, hsa-miR-548g-3p, hsa-miR-4257, hsa-miR-5006-5p, hsa-miR-7515, hsa-miR-6893-5p, hsa-miR-6879-5p, hsa-miR-3187-5p, hsa-miR-3185, hsa-miR-6823-5p, hsa-miR-12127, hsa-miR-6756-5p, hsa-miR-4784, hsa-miR-4746-3p, hsa-miR-4730, hsa-miR-647, hsa-miR-6786-5p, hsa-miR-8075, hsa-miR-486-3p, hsa-miR-3919, hsa-miR-3654, hsa-miR-6763-5p, hsa-miR-6782-5p, hsa-miR-6836-5p, hsa-miR-8071, hsa-miR-11399, hsa-miR-4300, hsa-miR-4437, hsa-miR-612, hsa-miR-6868-5p, hsa-miR-6777-5p, hsa-miR-4691-3p, hsa-miR-658, hsa-miR-548q, hsa-miR-614, hsa-miR-8083, hsa-miR-4638-5p, hsa-miR-1538, hsa-miR-4786-5p, hsa-miR-12122, hsa-miR-6762-5p, hsa-miR-6783-5p, hsa-miR-4708-3p, hsa-miR-550b-2-5p, hsa-miR-6074, hsa-miR-3675-5p, hsa-miR-6514-5p, hsa-miR-4692, hsa-miR-1912-5p, hsa-miR-1269b, hsa-miR-217-3p, hsa-miR-3616-3p, hsa-miR-4533, hsa-miR-516a-5p, hsa-miR-3074-5p, hsa-miR-1471, hsa-miR-197-5p, hsa-miR-4644, hsa-miR-5088-5p, hsa-miR-151a-3p, hsa-miR-8059, hsa-miR-6127, hsa-miR-610, hsa-miR-629-5p, hsa-miR-382-5p, hsa-miR-6131, hsa-miR-4492, hsa-miR-5194, hsa-miR-6089, hsa-miR-4463, hsa-miR-4428, hsa-miR-6836-3p, hsa-miR-6130, hsa-miR-4686, hsa-miR-4451, hsa-miR-4516, hsa-miR-301a-5p, hsa-miR-3130-3p, hsa-miR-3199, hsa-miR-8069, hsa-miR-6829-5p, hsa-miR-328-3p, hsa-miR-7160-5p, hsa-miR-8077, hsa-miR-1286, hsa-miR-4431, hsa-miR-3682-3p, hsa-miR-1193, hsa-miR-5787, hsa-miR-6766-5p, hsa-miR-6842-5p, hsa-miR-6876-5p, hsa-miR-139-3p, hsa-miR-208a-5p, hsa-miR-4743-3p, hsa-miR-6759-5p, hsa-miR-877-5p, hsa-miR-12124, hsa-miR-7151-3p, hsa-miR-1231, hsa-miR-5572, hsa-miR-4674, hsa-miR-370-3p, hsa-miR-3677-3p, hsa-miR-541-3p, hsa-miR-1468-5p, hsa-miR-7112-5p, hsa-miR-12118, hsa-miR-4725-3p, hsa-miR-4446-3p, hsa-miR-4681, hsa-miR-1203, hsa-miR-378j, hsa-miR-1910-3p, hsa-miR-449b-5p, hsa-miR-6796-5p, hsa-miR-128-1-5p, hsa-miR-5189-3p, hsa-miR-4448, hsa-miR-510-5p, hsa-miR-4481, hsa-miR-9986, hsa-miR-3917, hsa-miR-149-3p, hsa-miR-7110-5p, hsa-miR-4436a, hsa-miR-410-5p, hsa-miR-2467-3p, hsa-miR-4519, hsa-miR-29a-5p, hsa-miR-6070, hsa-miR-504-3p, hsa-miR-509-3-5p, hsa-miR-4315, hsa-miR-4787-5p, hsa-miR-4327, hsa-miR-550a-3-5p, hsa-miR-668-5p, hsa-miR-6751-5p, hsa-miR-4280, hsa-miR-4759, hsa-miR-921, hsa-miR-6869-5p, hsa-miR-3918, hsa-miR-6895-5p, hsa-miR-758-5p, hsa-miR-939-5p, hsa-miR-1249-5p, hsa-miR-6825-5p, hsa-miR-4502, hsa-miR-6800-5p, hsa-miR-3940-5p, hsa-miR-8057, hsa-miR-5699-3p, hsa-miR-7114-5p, hsa-miR-3180, hsa-miR-8070, hsa-miR-3120-3p, hsa-miR-6717-5p, hsa-miR-4763-5p, hsa-miR-6877-5p, hsa-miR-10398-3p, hsa-miR-4738-3p, hsa-miR-6813-5p, hsa-miR-4712-3p, hsa-miR-5705, hsa-miR-4487, hsa-miR-6849-5p, hsa-miR-761, hsa-miR-2682-5p, hsa-miR-5585-3p, hsa-miR-6894-5p, hsa-miR-489-5p, hsa-miR-4687-5p, hsa-miR-6503-3p, hsa-miR-6083, hsa-miR-4783-3p, hsa-miR-4711-3p, hsa-miR-5685, hsa-miR-7977, hsa-miR-4483, hsa-miR-3605-5p, hsa-miR-3660, hsa-miR-487a-5p, hsa-miR-185-3p, hsa-miR-3150b-3p, hsa-miR-4690-5p, hsa-miR-4447, hsa-miR-7113-5p, hsa-miR-579-3p, hsa-miR-6500-3p, hsa-miR-204-3p, hsa-miR-4726-3p, hsa-let-7d-3p, hsa-miR-4769-5p, hsa-miR-6509-5p, hsa-miR-2276-3p, hsa-miR-6129, hsa-miR-616-5p, hsa-miR-8089, hsa-miR-3192-5p, hsa-miR-3059-3p, hsa-miR-4440, hsa-miR-3173-3p, hsa-miR-4701-3p, hsa-miR-3122, hsa-miR-325, hsa-miR-6754-3p, hsa-miR-718, hsa-miR-12119, hsa-miR-3689b-3p, hsa-miR-3689c, hsa-miR-6772-5p, hsa-miR-30b-3p, hsa-miR-6792-5p, hsa-miR-3665, hsa-miR-147a, hsa-miR-6769b-5p, hsa-miR-3148, hsa-miR-200c-5p, hsa-miR-6848-5p, hsa-miR-296-3p, hsa-miR-4526, hsa-miR-7161-5p, hsa-miR-6765-5p, hsa-miR-7107-5p, hsa-miR-542-5p, hsa-miR-122b-3p, hsa-miR-1343-3p, hsa-miR-4521, hsa-miR-564, hsa-miR-4293, hsa-miR-5093, hsa-miR-6832-5p, hsa-miR-7157-3p, hsa-miR-6511b-5p, hsa-miR-6806-5p, hsa-miR-5190, hsa-miR-4800-5p, hsa-miR-6889-5p, hsa-miR-4748, hsa-miR-520c-3p, hsa-miR-6767-5p, hsa-miR-4474-3p, hsa-miR-4294, hsa-miR-6865-3p, hsa-miR-4256, hsa-miR-4743-5p, hsa-miR-3928-3p, hsa-miR-1273c, hsa-miR-585-5p, hsa-miR-4520-3p, hsa-miR-665, hsa-miR-483-5p, hsa-miR-1307-3p, hsa-miR-4317, hsa-miR-5589-5p, hsa-miR-373-3p, hsa-miR-6738-3p, hsa-miR-4676-5p, hsa-miR-6753-5p, hsa-miR-6740-5p, hsa-miR-3646, hsa-miR-1207-5p, hsa-miR-6870-5p, hsa-miR-7704, hsa-miR-4647, hsa-miR-3666, hsa-miR-4253, hsa-miR-3926, hsa-miR-6722-5p, hsa-miR-3190-3p, hsa-miR-4436b-3p, hsa-miR-3944-5p, hsa-miR-4485-5p, hsa-miR-1909-5p, hsa-miR-6892-5p, hsa-miR-130a-5p, hsa-miR-8073, hsa-miR-342-3p, hsa-miR-4296, hsa-miR-6845-3p, hsa-miR-103a-3p, hsa-miR-5690, hsa-miR-892b, hsa-miR-4465, hsa-miR-5087, hsa-miR-34c-5p, hsa-miR-1276, hsa-miR-4456, hsa-miR-1224-5p, hsa-miR-6857-5p, hsa-miR-4261, hsa-miR-6727-5p, hsa-miR-3155a, hsa-miR-4539, hsa-miR-361-5p, hsa-miR-4709-5p, hsa-miR-619-5p, hsa-miR-4513, hsa-miR-6822-5p, hsa-miR-2392, hsa-miR-378g, hsa-miR-3142, hsa-miR-326, hsa-miR-210-3p, hsa-miR-6814-5p, hsa-miR-4260, hsa-miR-6856-5p, hsa-miR-4999-5p, hsa-miR-4669, hsa-miR-497-5p, hsa-miR-5007-5p, hsa-miR-4484, hsa-miR-493-3p, hsa-miR-4510, hsa-miR-371a-3p, hsa-miR-329-5p, hsa-miR-4304, hsa-miR-10527-5p, hsa-miR-370-5p, hsa-miR-200a-5p, hsa-miR-4767, hsa-miR-6875-3p, hsa-miR-579-5p, hsa-miR-3151-5p, hsa-miR-938, hsa-miR-1260b, hsa-miR-498-3p, hsa-miR-3945, hsa-miR-656-5p, hsa-miR-4781-5p, hsa-miR-3679-5p, hsa-miR-525-3p, hsa-miR-3622a-3p, hsa-miR-6801-5p, hsa-miR-7850-5p, hsa-miR-515-5p, hsa-miR-1247-3p, hsa-miR-892c-5p, hsa-miR-6124, hsa-miR-1301-5p, hsa-miR-550a-5p, hsa-miR-4672, hsa-miR-6824-5p, hsa-miR-6893-3p, hsa-miR-10398-5p, hsa-miR-4299, hsa-miR-5002-3p, hsa-miR-3617-3p, hsa-miR-4462, hsa-miR-4660, hsa-miR-3934-5p, hsa-miR-3137, hsa-miR-4756-3p, hsa-miR-8080, hsa-miR-6859-5p, hsa-miR-6787-3p, hsa-miR-6511a-5p, hsa-miR-5708, hsa-miR-7846-3p, hsa-miR-378i, hsa-miR-595, hsa-miR-551b-3p, hsa-miR-7109-3p, hsa-miR-6715b-5p, hsa-miR-601, hsa-miR-323b-5p, hsa-miR-4269, hsa-miR-5588-3p, hsa-miR-11400, hsa-miR-6506-5p, hsa-miR-6884-5p, hsa-miR-4800-3p, hsa-miR-4522, hsa-miR-1269a, hsa-miR-8082, hsa-miR-6504-5p, hsa-miR-11181-3p, hsa-miR-4325, hsa-miR-106b-5p, hsa-miR-4252, hsa-miR-769-5p, hsa-miR-7113-3p, hsa-miR-4708-5p, hsa-miR-194-3p, hsa-miR-654-5p, hsa-miR-644a, hsa-miR-4700-5p, hsa-miR-8062, hsa-miR-6778-5p, hsa-miR-4703-3p, hsa-miR-3622b-5p, hsa-miR-4661-5p, hsa-miR-4524b-5p, hsa-miR-1202, hsa-miR-10400-5p, hsa-miR-6846-5p, hsa-miR-4430, hsa-miR-6831-5p, hsa-miR-4737, hsa-miR-6749-3p, hsa-miR-3158-5p, hsa-miR-4511, hsa-miR-4706, hsa-miR-1273h-5p, hsa-miR-2110, hsa-miR-1322, hsa-miR-323a-5p, hsa-miR-6738-5p, hsa-miR-1265, hsa-miR-4786-3p, hsa-miR-320d, hsa-miR-6788-5p, hsa-miR-6794-5p, hsa-miR-4682, hsa-miR-1228-5p, hsa-miR-5004-5p, hsa-miR-593-3p, hsa-miR-7843-5p, hsa-miR-4722-5p, hsa-miR-515-3p, hsa-miR-5089-5p, hsa-miR-6833-3p, hsa-miR-4721, hsa-miR-7849-3p, hsa-miR-4488, hsa-miR-3168, hsa-miR-140-3p, hsa-miR-4482-3p, hsa-miR-6762-3p, hsa-miR-924, hsa-miR-5187-5p, hsa-miR-4653-3p, hsa-miR-7976, hsa-miR-3907, hsa-miR-6715b-3p, hsa-miR-6818-5p, hsa-miR-6810-5p, hsa-miR-1236-3p, hsa-miR-10401-3p, hsa-miR-4421, hsa-miR-6775-5p, hsa-miR-6736-3p, hsa-miR-4740-3p, hsa-miR-449c-5p, hsa-miR-6514-3p, hsa-miR-4316, hsa-miR-491-5p, hsa-miR-6886-5p, hsa-miR-7152-5p, hsa-miR-6789-3p, hsa-miR-382-3p, hsa-miR-4436b-5p, hsa-miR-7111-5p, hsa-miR-652-5p, hsa-miR-557, hsa-miR-365a-5p, hsa-miR-578, hsa-miR-3944-3p, hsa-miR-2277-5p, hsa-miR-6719-3p, hsa-miR-6516-5p, hsa-miR-708-5p, hsa-miR-1470, hsa-miR-4514, hsa-miR-3176, hsa-miR-4766-5p, hsa-miR-8074, hsa-miR-3655, hsa-miR-615-5p, hsa-miR-3163, hsa-miR-105-5p, hsa-miR-941, hsa-let-7b-5p, hsa-miR-187-3p, hsa-miR-4732-3p, hsa-miR-6716-3p, hsa-miR-1257, hsa-miR-4670-5p, hsa-miR-4540, hsa-miR-11401, hsa-miR-1298-3p, hsa-miR-4265, hsa-miR-338-5p, hsa-miR-124-3p, hsa-miR-6850-3p, hsa-miR-675-3p, hsa-miR-99b-3p, hsa-miR-4273, hsa-miR-6812-5p, hsa-miR-3960, hsa-miR-632, hsa-miR-3085-5p, hsa-miR-214-3p, hsa-miR-6749-5p, hsa-miR-7975, hsa-miR-4710, hsa-miR-4283, hsa-miR-29a-3p, hsa-miR-3663-5p, hsa-miR-210-5p, hsa-miR-3158-3p, hsa-miR-12128, hsa-miR-4755-3p, hsa-miR-1306-3p, hsa-miR-3179, hsa-miR-4658, hsa-miR-3147, hsa-miR-6882-3p, hsa-miR-6874-5p, hsa-miR-1205, hsa-miR-5008-5p, hsa-miR-3689a-3p, hsa-miR-4732-5p, hsa-miR-6853-5p, hsa-miR-7109-5p, hsa-miR-381-5p, hsa-miR-4740-5p, hsa-miR-4684-5p, hsa-miR-3692-5p, hsa-miR-3935, hsa-miR-3941, hsa-miR-4639-3p, hsa-miR-488-5p, hsa-miR-6833-5p, hsa-miR-378f, hsa-miR-196b-3p, hsa-miR-6755-3p, hsa-miR-34a-5p, hsa-miR-517c-3p, hsa-miR-637, hsa-miR-6778-3p, hsa-miR-4691-5p, hsa-miR-4722-3p, hsa-miR-300, hsa-miR-891a-5p, hsa-miR-103a-1-5p, hsa-miR-219a-2-3p, hsa-miR-584-5p, hsa-miR-6735-5p, hsa-miR-433-5p, hsa-miR-4455, hsa-miR-1288-3p, hsa-miR-4635, hsa-miR-122-5p, hsa-miR-4717-3p, hsa-miR-6808-5p, hsa-miR-6772-3p, hsa-miR-6837-5p, hsa-miR-4761-3p, hsa-miR-3064-5p, hsa-miR-6510-5p, and hsa-miR-5587-3p. In the present disclosure, the extract of urine may be an extract of the urine of a breast cancer patient. Only one of these microRNAs can be used to predict whether a subject from which the urine is derived is a breast cancer patient, and 5 or more types, 10 or more types, 15 or more types, or 20 or more types of these microRNAs can be used in combination to predict whether a subject from which the urine is derived is a breast cancer patient. Among these microRNAs, a microRNA that may be highly expressed in a breast cancer patient can be preferably used for prediction.

The present disclosure provides an extract of urine containing at least one type, two types, three types, four types, five types, six types, seven types, eight types, nine types, or ten types of or all microRNAs selected from the group consisting of hsa-miR-29a-3p, hsa-miR-29b-3p, hsa-miR-103a-3p, hsa-miR-107, hsa-miR-192-5p, hsa-miR-196a-5p, hsa-miR-197-3p, hsa-miR-129-5p, hsa-miR-34a-5p, hsa-miR-187-3p, hsa-miR-210-3p, hsa-miR-211-5p, hsa-miR-214-3p, hsa-miR-124-3p, hsa-miR-125b-5p, hsa-miR-138-5p, hsa-miR- 149-5p, hsa-miR-184, hsa-miR-188-5p, hsa-miR-320a-3p, hsa-miR-106b-5p, hsa-miR-29c-3p, hsa-miR-299-3p, hsa-miR-361-5p, hsa-miR-370-3p, hsa-miR-373-5p, hsa-miR-373-3p, hsa-miR-378a-3p, hsa-miR-379-5p, hsa-miR-383-5p, hsa-miR-328-3p, hsa-miR-342-3p, hsa-miR-326, hsa-miR-151a-3p, hsa-miR-325, hsa-miR-346, hsa-miR-424-5p, hsa-miR-20b-5p, hsa-miR-200a-5p, hsa-miR-323b-5p, hsa-miR-485-5p, hsa-miR-486-5p, hsa-miR-488-5p, hsa-miR-491-5p, hsa-miR-202-3p, hsa-miR-492, hsa-miR-512-5p, hsa-miR-498-5p, hsa-miR-520c-3p, hsa-miR-519d-3p, hsa-miR-516b-3p, hsa-miR-516a-3p, hsa-miR-519a-3p, hsa-miR-502-5p, hsa-miR-493-3p, hsa-miR-551a, hsa-miR-552-3p, hsa-miR-554, hsa-miR-555, hsa-miR-557, hsa-miR-564, hsa-miR-572, hsa-miR-574-3p, hsa-miR-575, hsa-miR-576-5p, hsa-miR-578, hsa-miR-579-3p, hsa-miR-584-5p, hsa-miR-588, hsa-miR-595, hsa-miR-608, hsa-miR-610, hsa-miR-612, hsa-miR-614, hsa-miR-615-3p, hsa-miR-616-5p, hsa-miR-617, hsa-miR-628-3p, hsa-miR-629-3p, hsa-miR-632, hsa-miR-636, hsa-miR-637, hsa-miR-638, hsa-miR-639, hsa-miR-663a, hsa-miR-449b-5p, hsa-miR-657, hsa-miR-658, hsa-miR-542-5p, hsa-miR-671-5p, hsa-miR-668-3p, hsa-miR-769-5p, hsa-miR-769-3p, hsa-miR-766-3p, hsa-miR-765, hsa-miR-675-5p, hsa-miR-297, hsa-let-7d-3p, hsa-miR-24-2-5p, hsa-miR-25-5p, hsa-miR-26b-3p, hsa-miR-29a-5p, hsa-miR-92a-2-5p, hsa-miR-29b-1-5p, hsa-miR-139-3p, hsa-miR-187-5p, hsa-miR-214-5p, hsa-miR-30b-3p, hsa-miR-124-5p, hsa-miR-125b-1-3p, hsa-miR-130a-5p, hsa-miR-132-5p, hsa-miR-135a-3p, hsa-miR-140-3p, hsa-miR-125a-3p, hsa-miR-138-1-3p, hsa-miR-149-3p, hsa-miR-150-3p, hsa-miR-185-3p, hsa-miR-193a-5p, hsa-miR-194-3p, hsa-miR-30c-1-3p, hsa-miR-34b-3p, hsa-miR-34c-3p, hsa-miR-99b-3p, hsa-miR-296-3p, hsa-miR-323a-5p, hsa-miR-423-5p, hsa-miR-20b-3p, hsa-miR-483-5p, hsa-miR-486-3p, hsa-miR-193b-5p, hsa-miR-508-5p, hsa-miR-532-3p, hsa-miR-92b-5p, hsa-miR-551b-5p, hsa-miR-574-5p, hsa-miR-550a-5p, hsa-miR-615-5p, hsa-miR-616-3p, hsa-miR-624-3p, hsa-miR-629-5p, hsa-miR-671-3p, hsa-miR-891a-5p, hsa-miR-874-3p, hsa-miR-891b, hsa-miR-892b, hsa-miR-744-5p, hsa-miR-885-5p, hsa-miR-885-3p, hsa-miR-877-5p, hsa-miR-877-3p, hsa-miR-887-3p, hsa-miR-665, hsa-miR-760, hsa-miR-301b-3p, hsa-miR-920, hsa-miR-924, hsa-miR-509-3-5p, hsa-miR-936, hsa-miR-939-5p, hsa-miR-943, hsa-miR-1224-5p, hsa-miR-1224-3p, hsa-miR-1225-5p, hsa-miR-1225-3p, hsa-miR-1228-5p, hsa-miR-1228-3p, hsa-miR-1229-3p, hsa-miR-1231, hsa-miR-1233-3p, hsa-miR-1236-3p, hsa-miR-1237-3p, hsa-miR-1238-3p, hsa-miR-320c, hsa-miR-1182, hsa-miR-1202, hsa-miR-1203, hsa-miR-1204, hsa-miR-1207-5p, hsa-miR-1207-3p, hsa-miR-1286, hsa-miR-1293, hsa-miR-1294, hsa-miR-1303, hsa-miR-1304-5p, hsa-miR-1250-5p, hsa-miR-1257, hsa-miR-1260a, hsa-miR-1263, hsa-miR-1266-5p, hsa-miR-1268a, hsa-miR-1281, hsa-miR-1292-5p, hsa-miR-1306-3p, hsa-miR-1307-3p, hsa-miR-320d, hsa-miR-1825, hsa-miR-1468-5p, hsa-miR-675-3p, hsa-miR-1469, hsa-miR-1470, hsa-miR-1471, hsa-miR-1538, hsa-miR-1539, hsa-miR-1908-5p, hsa-miR-1909-5p, hsa-miR-1909-3p, hsa-miR-1910-5p, hsa-miR-1912-3p, hsa-miR-1914-3p, hsa-miR-1915-3p, hsa-miR-365a-5p, hsa-miR-449b-3p, hsa-miR-2113, hsa-miR-1976, hsa-miR-2110, hsa-miR-762, hsa-miR-670-5p, hsa-miR-548q, hsa-miR-2276-3p, hsa-miR-711, hsa-miR-718, hsa-miR-2681-3p, hsa-miR-2682-3p, hsa-miR-449c-3p, hsa-miR-2861, hsa-miR-3120-3p, hsa-miR-3122, hsa-miR-3130-5p, hsa-miR-466, hsa-miR-3136-5p, hsa-miR-3137, hsa-miR-3138, hsa-miR-3141, hsa-miR-1273c, hsa-miR-3147, hsa-miR-3148, hsa-miR-3151-5p, hsa-miR-3153, hsa-miR-3154, hsa-miR-3157-5p, hsa-miR-3161, hsa-miR-3162-5p, hsa-miR-3163, hsa-miR-3165, hsa-miR-1260b, hsa-miR-3168, hsa-miR-3173-3p, hsa-miR-1193, hsa-miR-3175, hsa-miR-3176, hsa-miR-3177-3p, hsa-miR-3178, hsa-miR-3179, hsa-miR-3180-5p, hsa-miR-3180-3p, hsa-miR-3185, hsa-miR-3187-3p, hsa-miR-3191-3p, hsa-miR-3194-5p, hsa-miR-3195, hsa-miR-3196, hsa-miR-3197, hsa-miR-3198, hsa-miR-3126-3p, hsa-miR-4296, hsa-miR-4297, hsa-miR-4293, hsa-miR-4294, hsa-miR-4298, hsa-miR-4305, hsa-miR-4315, hsa-miR-4316, hsa-miR-4314, hsa-miR-4320, hsa-miR-4322, hsa-miR-4321, hsa-miR-4323, hsa-miR-4324, hsa-miR-4256, hsa-miR-4257, hsa-miR-4258, hsa-miR-4259, hsa-miR-4260, hsa-miR-4253, hsa-miR-4252, hsa-miR-4325, hsa-miR-4326, hsa-miR-4327, hsa-miR-4265, hsa-miR-4269, hsa-miR-4271, hsa-miR-4276, hsa-miR-4279, hsa-miR-4278, hsa-miR-4280, hsa-miR-4285, hsa-miR-4283, hsa-miR-4289, hsa-miR-500b-5p, hsa-miR-2277-5p, hsa-miR-3200-5p, hsa-miR-3605-3p, hsa-miR-3610, hsa-miR-3616-3p, hsa-miR-3619-5p, hsa-miR-3620-3p, hsa-miR-3621, hsa-miR-3622a-5p, hsa-miR-3622a-3p, hsa-miR-3622b-5p, hsa-miR-3646, hsa-miR-3648, hsa-miR-3649, hsa-miR-3650, hsa-miR-3652, hsa-miR-3660, hsa-miR-3663-5p, hsa-miR-3663-3p, hsa-miR-3665, hsa-miR-3677-3p, hsa-miR-3679-5p, hsa-miR-3685, hsa-miR-3689a-3p, hsa-miR-3691-5p, hsa-miR-3180, hsa-miR-3907, hsa-miR-3689b-3p, hsa-miR-3689c, hsa-miR-3908, hsa-miR-3911, hsa-miR-3917, hsa-miR-3918, hsa-miR-3919, hsa-miR-3150b-3p, hsa-miR-3926, hsa-miR-3928-3p, hsa-miR-3935, hsa-miR-3937, hsa-miR-3941, hsa-miR-3944-3p, hsa-miR-3945, hsa-miR-642b-3p, hsa-miR-550b-3p, hsa-miR-1268b, hsa-miR-378f, hsa-miR-4421, hsa-miR-4428, hsa-miR-4429, hsa-miR-4430, hsa-miR-4433a-3p, hsa-miR-4434, hsa-miR-4435, hsa-miR-4436a, hsa-miR-4437, hsa-miR-4439, hsa-miR-4440, hsa-miR-4443, hsa-miR-4446-3p, hsa-miR-4447, hsa-miR-4448, hsa-miR-4449, hsa-miR-4455, hsa-miR-4456, hsa-miR-4458, hsa-miR-4460, hsa-miR-378h, hsa-miR-3135b, hsa-miR-4462, hsa-miR-4463, hsa-miR-4466, hsa-miR-4467, hsa-miR-4472, hsa-miR-4474-3p, hsa-miR-4476, hsa-miR-4478, hsa-miR-4479, hsa-miR-4481, hsa-miR-4483, hsa-miR-4484, hsa-miR-4486, hsa-miR-4487, hsa-miR-4488, hsa-miR-4489, hsa-miR-4492, hsa-miR-4497, hsa-miR-4498, hsa-miR-4505, hsa-miR-4506, hsa-miR-2392, hsa-miR-4507, hsa-miR-4508, hsa-miR-4510, hsa-miR-4511, hsa-miR-4512, hsa-miR-4513, hsa-miR-4516, hsa-miR-4519, hsa-miR-4520-3p, hsa-miR-4521, hsa-miR-1269b, hsa-miR-4522, hsa-miR-4523, hsa-miR-4524a-3p, hsa-miR-4525, hsa-miR-4526, hsa-miR-4530, hsa-miR-4533, hsa-miR-4535, hsa-miR-1587, hsa-miR-4538, hsa-miR-4539, hsa-miR-4540, hsa-miR-3120-5p, hsa-miR-3156-3p, hsa-miR-3158-5p, hsa-miR-3173-5p, hsa-miR-3187-5p, hsa-miR-3922-5p, hsa-miR-3925-3p, hsa-miR-3940-5p, hsa-miR-4520-5p, hsa-miR-3960, hsa-miR-3973, hsa-miR-4634, hsa-miR-4638-5p, hsa-miR-4639-3p, hsa-miR-4640-5p, hsa-miR-4643, hsa-miR-4644, hsa-miR-4646-5p, hsa-miR-4646-3p, hsa-miR-4647, hsa-miR-4648, hsa-miR-4649-5p, hsa-miR-4650-3p, hsa-miR-4651, hsa-miR-4653-3p, hsa-miR-4654, hsa-miR-4655-5p, hsa-miR-4656, hsa-miR-4661-5p, hsa-miR-4664-5p, hsa-miR-4665-5p, hsa-miR-4665-3p, hsa-miR-4667-5p, hsa-miR-4669, hsa-miR-4670-5p, hsa-miR-4672, hsa-miR-4673, hsa-miR-4674, hsa-miR-4676-5p, hsa-miR-4682, hsa-miR-4685-5p, hsa-miR-4685-3p, hsa-miR-4686, hsa-miR-4687-5p, hsa-miR-1343-3p, hsa-miR-4688, hsa-miR-4690-5p, hsa-miR-4691-5p, hsa-miR-4691-3p, hsa-miR-4692, hsa-miR-4694-5p, hsa-miR-4695-5p, hsa-miR-4695-3p, hsa-miR-4697-5p, hsa-miR-4700-5p, hsa-miR-4701-3p, hsa-miR-4703-3p, hsa-miR-4704-5p, hsa-miR-4706, hsa-miR-4707-5p, hsa-miR-4707-3p, hsa-miR-4708-5p, hsa-miR-4708-3p, hsa-miR-203b-5p, hsa-miR-4710, hsa-miR-4711-3p, hsa-miR-4712-3p, hsa-miR-4713-5p, hsa-miR-4717-3p, hsa-miR-4720-5p, hsa-miR-4721, hsa-miR-4722-5p, hsa-miR-4722-3p, hsa-miR-4723-3p, hsa-miR-4724-5p, hsa-miR-4725-5p, hsa-miR-4725-3p, hsa-miR-4726-3p, hsa-miR-4728-3p, hsa-miR-4730, hsa-miR-4732-5p, hsa-miR-4732-3p, hsa-miR-4734, hsa-miR-4736, hsa-miR-3064-5p, hsa-miR-3064-3p, hsa-miR-4738-3p, hsa-miR-4739, hsa-miR-4740-3p, hsa-miR-4741, hsa-miR-4743-5p, hsa-miR-122b-3p, hsa-miR-4745-5p, hsa-miR-4746-3p, hsa-miR-4747-5p, hsa-miR-4747-3p, hsa-miR-4748, hsa-miR-4749-5p, hsa-miR-4750-5p, hsa-miR-4753-5p, hsa-miR-371b-5p, hsa-miR-4754, hsa-miR-4755-3p, hsa-miR-4758-5p, hsa-miR-4758-3p, hsa-miR-4759, hsa-miR-4761-5p, hsa-miR-4763-5p, hsa-miR-4763-3p, hsa-miR-4766-5p, hsa-miR-4767, hsa-miR-4769-5p, hsa-miR-4769-3p, hsa-miR-4772-5p, hsa-miR-4776-5p, hsa-miR-4776-3p, hsa-miR-4780, hsa-miR-4436b-5p, hsa-miR-4436b-3p, hsa-miR-4781-5p, hsa-miR-4783-3p, hsa-miR-4784, hsa-miR-2467-3p, hsa-miR-4787-5p, hsa-miR-4787-3p, hsa-miR-4793-3p, hsa-miR-4800-5p, hsa-miR-642a-3p, hsa-miR-550a-3-5p, hsa-miR-4482-3p, hsa-miR-4999-5p, hsa-miR-5000-3p, hsa-miR-5001-5p, hsa-miR-5001-3p, hsa-miR-5002-3p, hsa-miR-5003-3p, hsa-miR-5006-5p, hsa-miR-5008-5p, hsa-miR-5010-5p, hsa-miR-5087, hsa-miR-5088-5p, hsa-miR-5089-5p, hsa-miR-5090, hsa-miR-5189-5p, hsa-miR-5190, hsa-miR-5196-5p, hsa-miR-4524b-5p, hsa-miR-5100, hsa-miR-5572, hsa-miR-664b-5p, hsa-miR-664b-3p, hsa-miR-548at-5p, hsa-miR-5585-3p, hsa-miR-548au-3p, hsa-miR-5588-5p, hsa-miR-5588-3p, hsa-miR-5589-5p, hsa-miR-5685, hsa-miR-5690, hsa-miR-5698, hsa-miR-5699-3p, hsa-miR-5703, hsa-miR-5705, hsa-miR-5706, hsa-miR-5708, hsa-miR-197-5p, hsa-miR-204-3p, hsa-miR-211-3p, hsa-miR-301a-5p, hsa-miR-382-3p, hsa-miR-584-3p, hsa-miR-652-5p, hsa-miR-1185-2-3p, hsa-miR-766-5p, hsa-miR-1304-3p, hsa-miR-1247-3p, hsa-miR-1306-5p, hsa-miR-3184-3p, hsa-miR-3191-5p, hsa-miR-642b-5p, hsa-miR-3529-3p, hsa-miR- 365b-5p, hsa-miR-3190-3p, hsa-miR-381-5p, hsa-miR-503-3p, hsa-miR-758-5p, hsa-miR-937-5p, hsa-miR-939-3p, hsa-miR-1227-5p, hsa-miR-1229-5p, hsa-miR-1236-5p, hsa-miR-1237-5p, hsa-miR-1238-5p, hsa-miR-3617-3p, hsa-miR-3620-5p, hsa-miR-3927-5p, hsa-miR-3934-3p, hsa-miR-4632-5p, hsa-miR-4743-3p, hsa-miR-5739, hsa-miR-5787, hsa-miR-6068, hsa-miR-6069, hsa-miR-6071, hsa-miR-6072, hsa-miR-6073, hsa-miR-6075, hsa-miR-6076, hsa-miR-6081, hsa-miR-6083, hsa-miR-6085, hsa-miR-6088, hsa-miR-6089, hsa-miR-6090, hsa-miR-6124, hsa-miR-6125, hsa-miR-6126, hsa-miR-6127, hsa-miR-378j, hsa-miR-6131, hsa-miR-6132, hsa-miR-6133, hsa-miR-6500-3p, hsa-miR-6501-5p, hsa-miR-6501-3p, hsa-miR-6506-5p, hsa-miR-6509-3p, hsa-miR-6510-5p, hsa-miR-6511a-5p, hsa-miR-6511a-3p, hsa-miR-6512-3p, hsa-miR-6513-5p, hsa-miR-6514-3p, hsa-miR-6515-5p, hsa-miR-6515-3p, hsa-miR-6715b-3p, hsa-miR-6716-5p, hsa-miR-6716-3p, hsa-miR-6717-5p, hsa-miR-6511b-5p, hsa-miR-6511b-3p, hsa-miR-6719-3p, hsa-miR-6721-5p, hsa-miR-6722-5p, hsa-miR-6722-3p, hsa-miR-6724-5p, hsa-miR-208a-5p, hsa-miR-210-5p, hsa-miR-128-1-5p, hsa-miR-370-5p, hsa-miR-328-5p, hsa-miR-329-5p, hsa-miR-410-5p, hsa-miR-487a-5p, hsa-miR-494-5p, hsa-miR-181d-3p, hsa-miR-504-3p, hsa-miR-487b-5p, hsa-miR-585-5p, hsa-miR-598-5p, hsa-miR-605-3p, hsa-miR-619-5p, hsa-miR-655-5p, hsa-miR-1296-3p, hsa-miR-1301-5p, hsa-miR-874-5p, hsa-miR-1250-3p, hsa-miR-1266-3p, hsa-miR-1908-3p, hsa-miR-3151-3p, hsa-miR-500b-3p, hsa-miR-1343-5p, hsa-miR-5189-3p, hsa-miR-6726-5p, hsa-miR-6726-3p, hsa-miR-6727-5p, hsa-miR-6727-3p, hsa-miR-6728-5p, hsa-miR-6728-3p, hsa-miR-6729-5p, hsa-miR-6730-5p, hsa-miR-6730-3p, hsa-miR-6732-5p, hsa-miR-6732-3p, hsa-miR-6734-5p, hsa-miR-6735-5p , hsa-miR-6735-3p, hsa-miR-6736-5p, hsa-miR-6736-3p, hsa-miR-6737-5p, hsa-miR-6738-5p, hsa-miR-6738-3p, hsa-miR-6739-3p, hsa-miR-6740-5p, hsa-miR-6740-3p, hsa-miR-6741-5p, hsa-miR-6741-3p, hsa-miR-6742-5p, hsa-miR-6743-5p, hsa-miR-6743-3p, hsa-miR-6744-3p, hsa-miR-6747-5p, hsa-miR-6747-3p, hsa-miR-6748-5p, hsa-miR-6748-3p, hsa-miR-6749-5p, hsa-miR-6749-3p, hsa-miR-6750-3p, hsa-miR-6751-5p, hsa-miR-6751-3p, hsa-miR-6752-5p, hsa-miR-6753-5p, hsa-miR-6753-3p, hsa-miR-6754-5p, hsa-miR-6754-3p, hsa-miR-6756-5p, hsa-miR-6756-3p, hsa-miR-6759-5p, hsa-miR-6759-3p, hsa-miR-6761-3p, hsa-miR-6762-5p, hsa-miR-6762-3p, hsa-miR-6763-5p, hsa-miR-6763-3p, hsa-miR-6765-5p, hsa-miR-6766-5p, hsa-miR-6767-5p, hsa-miR-6768-5p, hsa-miR-6771-5p, hsa-miR-6772-5p, hsa-miR-6772-3p, hsa-miR-6773-3p, hsa-miR-6775-5p, hsa-miR-6775-3p, hsa-miR-6776-5p, hsa-miR-6776-3p, hsa-miR-6777-5p, hsa-miR-6777-3p, hsa-miR-6778-5p, hsa-miR-6778-3p, hsa-miR-6779-3p, hsa-miR-6780a-3p, hsa-miR-6781-5p, hsa-miR-6782-5p, hsa-miR-6783-5p, hsa-miR-6783-3p, hsa-miR-6784-5p, hsa-miR-6786-5p, hsa-miR-6786-3p, hsa-miR-6787-5p, hsa-miR-6787-3p, hsa-miR-6788-5p, hsa-miR-6788-3p, hsa-miR-6789-5p, hsa-miR-6789-3p, hsa-miR-6790-5p, hsa-miR-6790-3p, hsa-miR-6791-5p, hsa-miR-6792-5p, hsa-miR-6792-3p, hsa-miR-6793-5p, hsa-miR-6794-5p, hsa-miR-6794-3p, hsa-miR-6796-5p, hsa-miR-6797-5p, hsa-miR-6797-3p, hsa-miR-6798-5p, hsa-miR-6799-5p, hsa-miR-6800-5p, hsa-miR-6800-3p, hsa-miR-6801-5p, hsa-miR-6803-5p, hsa-miR-6803-3p, hsa-miR-6804-3p, hsa-miR-6805-5p, hsa-miR-6805-3p, hsa-miR-6806-5p, hsa-miR-6807-5p, hsa-miR-6808-5p, hsa-miR-6808-3p, hsa-miR-6809-5p, hsa-miR-6809-3p, hsa-miR-6810-5p, hsa-miR-6812-5p, hsa-miR-6813-5p, hsa-miR-6813-3p, hsa-miR-6815-3p, hsa-miR-6816-5p, hsa-miR-6818-5p, hsa-miR-6820-5p, hsa-miR-6821-5p, hsa-miR-6822-5p, hsa-miR-6822-3p, hsa-miR-6823-5p, hsa-miR-6823-3p, hsa-miR-6824-5p, hsa-miR-6825-5p, hsa-miR-6825-3p, hsa-miR-6827-5p, hsa-miR-6829-5p, hsa-miR-6829-3p, hsa-miR-6830-5p, hsa-miR-6830-3p, hsa-miR-6831-5p, hsa-miR-6832-5p, hsa-miR-6833-5p, hsa-miR-6833-3p, hsa-miR-6834-5p, hsa-miR-6780b-5p, hsa-miR-6780b-3p, hsa-miR-6836-3p, hsa-miR-6837-5p, hsa-miR-6838-5p, hsa-miR-6839-5p, hsa-miR-6840-3p, hsa-miR-6842-5p, hsa-miR-6845-5p, hsa-miR-6845-3p, hsa-miR-6846-5p, hsa-miR-6847-5p, hsa-miR-6847-3p, hsa-miR-6848-5p, hsa-miR-6849-5p, hsa-miR-6849-3p, hsa-miR-6850-5p, hsa-miR-6851-5p, hsa-miR-6852-3p, hsa-miR-6855-5p, hsa-miR-6856-5p, hsa-miR-6857-5p, hsa-miR-6858-5p, hsa-miR-6858-3p, hsa-miR-6859-5p, hsa-miR-6769b-5p, hsa-miR-6860, hsa-miR-6861-5p, hsa-miR-6861-3p, hsa-miR-6862-3p, hsa-miR-6865-3p, hsa-miR-6867-5p, hsa-miR-6867-3p, hsa-miR-6868-3p, hsa-miR-6869-5p, hsa-miR-6870-5p, hsa-miR-6871-5p, hsa-miR-6872-3p, hsa-miR-6875-5p, hsa-miR-6875-3p, hsa-miR-6876-5p, hsa-miR-6876-3p, hsa-miR-6877-5p, hsa-miR-6877-3p, hsa-miR-6879-5p, hsa-miR-6879-3p, hsa-miR-6880-5p, hsa-miR-6880-3p, hsa-miR-6881-5p, hsa-miR-6882-3p, hsa-miR-6883-3p, hsa-miR-6884-5p, hsa-miR-6886-3p, hsa-miR-6887-3p, hsa-miR-6889-5p, hsa-miR-6890-5p, hsa-miR-6891-5p, hsa-miR-6891-3p, hsa-miR-6892-5p, hsa-miR-6892-3p, hsa-miR-6893-5p, hsa-miR-6893-3p, hsa-miR-6894-5p, hsa-miR-6894-3p, hsa-miR-6895-5p, hsa-miR-6895-3p, hsa-miR-7106-5p, hsa-miR-7106-3p, hsa-miR-7107-5p, hsa-miR-7108-5p, hsa-miR-7108-3p, hsa-miR-7109-5p, hsa-miR-7109-3p, hsa-miR-7110-5p, hsa-miR-7110-3p, hsa-miR-7111-Sp, hsa-miR-7111-3p, hsa-miR-7112-5p, hsa-miR-7113-5p, hsa-miR-7113-3p, hsa-miR-7114-5p, hsa-miR-7150, hsa-miR-7151-3p, hsa-miR-7152-5p, hsa-miR-7155-5p, hsa-miR-7157-3p, hsa-miR-7158-3p, hsa-miR-7159-5p, hsa-miR-7160-5p, hsa-miR-7161-5p, hsa-miR-7162-5p, hsa-miR-7515, hsa-miR-7704, hsa-miR-7843-5p, hsa-miR-4433b-3p, hsa-miR-1273h-5p, hsa-miR-6516-5p, hsa-miR-7845-5p, hsa-miR-7846-3p, hsa-miR-7847-3p, hsa-miR-7850-5p, hsa-miR-7851-3p, hsa-miR-7854-3p, hsa-miR-8052, hsa-miR-8059, hsa-miR-8062, hsa-miR-8063, hsa-miR-8064, hsa-miR-8069, hsa-miR-8071, hsa-miR-8072, hsa-miR-8073, hsa-miR-8074, hsa-miR-8075, hsa-miR-8077, hsa-miR-8080, hsa-miR-8085, hsa-miR-8089, hsa-miR-7976, hsa-miR-7977, hsa-miR-203a-5p, hsa-miR-1249-5p, hsa-miR-4485-5p, hsa-miR-8485, hsa-miR-103a-1-5p, hsa-miR-196a-1-3p, hsa-miR-217-3p, hsa-miR-375-5p, hsa-miR-498-3p, hsa-miR-1912-5p, hsa-miR-9718, hsa-miR-1843, hsa-miR-10392-5p, hsa-miR-10392-3p, hsa-miR-10394-3p, hsa-miR-10395-3p, hsa-miR-10396a-5p, hsa-miR-10396a-3p, hsa-miR-10398-5p, hsa-miR-10398-3p, hsa-miR-10400-5p, hsa-miR-10400-3p, hsa-miR-10401-5p, hsa-miR-10401-3p, hsa-miR-10396b-5p, hsa-miR-10396b-3p, hsa-miR-10527-5p, hsa-miR-11181-5p, hsa-miR-11181-3p, hsa-miR-11399, hsa-miR-11400, hsa-miR-11401, hsa-miR-3085-5p, hsa-miR-3085-3p, hsa-miR-6529-5p, hsa-miR-12114, hsa-miR-12115, hsa-miR-12118, hsa-miR-12119, hsa-miR-12120, hsa-miR-12121, hsa-miR-12124, hsa-miR-12126, and hsa-miR-12128. In the present disclosure, the extract of urine may be an extract of the urine of a stage-I or -II breast cancer patient. Only one of these microRNAs can be used to predict whether a subject from which the urine is derived is a breast cancer patient (particularly stage-I or -II), and 5 or more types, 10 or more types, 15 or more types, or 20 or more types of these microRNAs can be used in combination to predict whether a subject from which the urine is derived is a breast cancer patient (particularly stage-I or -II). Among these microRNAs, a microRNA that may be highly expressed in a breast cancer patient (particularly stage-I or -II) can be preferably used for prediction.

The present disclosure provides an extract of urine containing at least one type, two types, three types, four types, five types, six types, seven types, eight types, nine types, or ten types of or all microRNAs selected from the group consisting of hsa-miR-29a-3p, hsa-miR-31-5p, hsa-miR-29b-3p, hsa-miR-124-3p, hsa-miR-184, hsa-miR-106b-5p, hsa-miR-299-3p, hsa-miR-361-5p, hsa-miR-370-3p, hsa-miR-373-3p, hsa-miR-379-5p, hsa-miR-335-5p, hsa-miR-325, hsa-miR-202-3p, hsa-miR-492, hsa-miR-498-5p, hsa-miR-555, hsa-miR-564, hsa-miR-572, hsa-miR-575, hsa-miR-578, hsa-miR-587, hsa-miR-610, hsa-miR-612, hsa-miR-617, hsa-miR-628-3p, hsa-miR-638, hsa-miR-639, hsa-miR-648, hsa-miR-663a, hsa-miR-658, hsa-miR-542-5p, hsa-miR-769-3p, hsa-miR-24-2-5p, hsa-miR-25-5p, hsa-miR-92a-2-5p, hsa-miR-139-3p, hsa-miR-187-5p, hsa-miR-30b-3p, hsa-miR-124-5p, hsa-miR-125b-1-3p, hsa-miR-130a-5p, hsa-miR-132-5p, hsa-miR-135a-3p, hsa-miR-149-3p, hsa-miR-185-3p, hsa-miR-193a-5p, hsa-miR-424-3p, hsa-miR-20b-3p, hsa-miR-486-3p, hsa-miR-193b-5p, hsa-miR-501-3p, hsa-miR-92b-5p, hsa-miR-551b-5p, hsa-miR-629-5p, hsa-miR-891b, hsa-miR-541-3p, hsa-miR-744-5p, hsa-miR-885-3p, hsa-miR-877-5p, hsa-miR-887-3p, hsa-miR-665, hsa-miR-760, hsa-miR-939-5p, hsa-miR-1224-5p, hsa-miR-1225-5p, hsa-miR-1228-5p, hsa-miR-1231, hsa-miR-320c, hsa-miR-1207-5p, hsa-miR-1285-3p, hsa-miR-1293, hsa-miR-1294, hsa-miR-1299, hsa-miR-1304-5p, hsa-miR-1257, hsa-miR-1263, hsa-miR-1268a, hsa-miR-1281, hsa-miR-1292-5p, hsa-miR-320d, hsa-miR-1469, hsa-miR-1471, hsa-miR-1538, hsa-miR-1908-5p, hsa-miR-1909-3p, hsa-miR-1912-3p, hsa-miR-1915-3p, hsa-miR-762, hsa-miR-548q, hsa-miR-718, hsa-miR-3120-3p, hsa-miR-3141, hsa-miR-1273c, hsa-miR-3161, hsa-miR-3162-5p, hsa-miR-1260b, hsa-miR-3168, hsa-miR-3173-3p, hsa-miR-1193, hsa-miR-3178, hsa-miR-3179, hsa-miR-3180-3p, hsa-miR-3185, hsa-miR-3187-3p, hsa-miR-3195, hsa-miR-3196, hsa-miR-3197, hsa-miR-4315, hsa-miR-4314, hsa-miR-4322, hsa-miR-4321, hsa-miR-4256, hsa-miR-4257, hsa-miR-4258, hsa-miR-4327, hsa-miR-4265, hsa-miR-4276, hsa-miR-4285, hsa-miR-3609, hsa-miR-3616-3p, hsa-miR-3621, hsa-miR-3648, hsa-miR-3652, hsa-miR-3660, hsa-miR-3663-3p, hsa-miR-3665, hsa-miR-3677-3p, hsa-miR-3180, hsa-miR-3907, hsa-miR-3917, hsa-miR-3919, hsa-miR-3928-3p, hsa-miR-3937, hsa-miR-1268b, hsa-miR-4428, hsa-miR-4429, hsa-miR-4430, hsa-miR-4433a-3p, hsa-miR-4434, hsa-miR-4435, hsa-miR-4439, hsa-miR-4440, hsa-miR-4443, hsa-miR-4446-3p, hsa-miR-4447, hsa-miR-4449, hsa-miR-4450, hsa-miR-4454, hsa-miR-4456, hsa-miR-4458, hsa-miR-378h, hsa-miR-3135b, hsa-miR-4466, hsa-miR-4467, hsa-miR-4468, hsa-miR-4479, hsa-miR-4481, hsa-miR-4486, hsa-miR-4487, hsa-miR-4489, hsa-miR-4492, hsa-miR-4497, hsa-miR-4498, hsa-miR-4505, hsa-miR-4506, hsa-miR-4507, hsa-miR-4508, hsa-miR-4511, hsa-miR-4512, hsa-miR-4519, hsa-miR-4521, hsa-miR-1269b, hsa-miR-4530, hsa-miR-4533, hsa-miR-4535, hsa-miR-1587, hsa-miR-4538, hsa-miR-4539, hsa-miR-3187-5p, hsa-miR-3940-5p, hsa-miR-4634, hsa-miR-4638-5p, hsa-miR-4639-5p, hsa-miR-4640-5p, hsa-miR-4648, hsa-miR-4650-3p, hsa-miR-4651, hsa-miR-4653-3p, hsa-miR-4654, hsa-miR-4655-5p, hsa-miR-4657, hsa-miR-4661-5p, hsa-miR-4665-5p, hsa-miR-4673, hsa-miR-4674, hsa-miR-4687-5p, hsa-miR-4688, hsa-miR-4690-5p, hsa-miR-4691-3p, hsa-miR-4694-5p, hsa-miR-4695-5p, hsa-miR-4704-5p, hsa-miR-4707-5p, hsa-miR-4708-5p, hsa-miR-4708-3p, hsa-miR-4720-5p, hsa-miR-4724-5p, hsa-miR-4726-3p, hsa-miR-4730, hsa-miR-4734, hsa-miR-4736, hsa-miR-4738-3p, hsa-miR-4741, hsa-miR-4743-5p, hsa-miR-122b-3p, hsa-miR-4745-5p, hsa-miR-4746-3p, hsa-miR-4749-5p, hsa-miR-4750-5p, hsa-miR-371b-5p, hsa-miR-4754, hsa-miR-4755-3p, hsa-miR-4758-5p, hsa-miR-4759, hsa-miR-4763-3p, hsa-miR-4766-5p, hsa-miR-4781-5p, hsa-miR-4784, hsa-miR-4787-5p, hsa-miR-4520-2-3p, hsa-miR-4482-3p, hsa-miR-5001-5p, hsa-miR-5010-5p, hsa-miR-5088-5p, hsa-miR-5089-5p, hsa-miR-5090, hsa-miR-5189-5p, hsa-miR-5100, hsa-miR-5572, hsa-miR-5585-3p, hsa-miR-548au-3p, hsa-miR-5690, hsa-miR-5698, hsa-miR-5699-3p, hsa-miR-5703, hsa-miR-5706, hsa-miR-197-5p, hsa-miR-211-3p, hsa-miR-766-5p, hsa-miR-3529-3p, hsa-miR-937-5p, hsa-miR-1227-5p, hsa-miR-1236-5p, hsa-miR-1237-5p, hsa-miR-3620-5p, hsa-miR-3934-3p, hsa-miR-4632-5p, hsa-miR-5787, hsa-miR-6068, hsa-miR-6071, hsa-miR-6075, hsa-miR-6076, hsa-miR-6081, hsa-miR-6089, hsa-miR-6090, hsa-miR-6125, hsa-miR-6126, hsa-miR-6127, hsa-miR-6131, hsa-miR-6132, hsa-miR-6500-3p, hsa-miR-6501-5p, hsa-miR-6501-3p, hsa-miR-6511a-5p, hsa-miR-6513-5p, hsa-miR-6719-3p, hsa-miR-6721-5p, hsa-miR-6722-3p, hsa-miR-6724-5p, hsa-miR-208a-5p, hsa-miR-128-1-5p, hsa-miR-181d-3p, hsa-miR-504-3p, hsa-miR-605-3p, hsa-miR-1343-5p, hsa-miR-5189-3p, hsa-miR-6726-5p, hsa-miR-6727-5p, hsa-miR-6729-5p, hsa-miR-6732-5p, hsa-miR-6737-5p, hsa-miR-6743-5p, hsa-miR-6750-5p, hsa-miR-6751-5p, hsa-miR-6752-5p, hsa-miR-6753-5p, hsa-miR-6756-5p, hsa-miR-6759-5p, hsa-miR-6762-5p, hsa-miR-6763-5p, hsa-miR-6766-5p, hsa-miR-6768-5p, hsa-miR-6772-5p, hsa-miR-6776-5p, hsa-miR-6777-5p, hsa-miR-6781-5p, hsa-miR-6782-5p, hsa-miR-6784-5p, hsa-miR-6786-5p, hsa-miR-6787-5p, hsa-miR-6789-5p, hsa-miR-6790-5p, hsa-miR-6791-5p, hsa-miR-6792-5p, hsa-miR-6793-5p, hsa-miR-6794-5p, hsa-miR-6796-5p, hsa-miR-6798-5p, hsa-miR-6799-5p, hsa-miR-6800-5p, hsa-miR-6805-5p, hsa-miR-6813-5p, hsa-miR-6816-5p, hsa-miR-6818-5p, hsa-miR-6820-5p, hsa-miR-6821-5p, hsa-miR-6823-5p, hsa-miR-6829-5p, hsa-miR-6832-5p, hsa-miR-6836-5p, hsa-miR-6839-5p, hsa-miR-6840-3p, hsa-miR-6842-5p, hsa-miR-6845-5p, hsa-miR-6847-5p, hsa-miR-6848-5p, hsa-miR-6849-5p, hsa-miR-6850-5p, hsa-miR-6860, hsa-miR-6861-5p, hsa-miR-6869-5p, hsa-miR-6870-5p, hsa-miR-6871-5p, hsa-miR-6875-5p, hsa-miR-6876-5p, hsa-miR-6877-5p, hsa-miR-6879-5p, hsa-miR-6893-5p, hsa-miR-6894-5p, hsa-miR-6895-5p, hsa-miR-7108-5p, hsa-miR-7110-5p, hsa-miR-7112-5p, hsa-miR-7114-5p, hsa-miR-7150, hsa-miR-7157-3p, hsa-miR-7158-3p, hsa-miR-7160-5p, hsa-miR-7515, hsa-miR-7704, hsa-miR-4433b-3p, hsa-miR-6516-5p, hsa-miR-7851-3p, hsa-miR-7854-3p, hsa-miR-8059, hsa-miR-8063, hsa-miR-8064, hsa-miR-8069, hsa-miR-8071, hsa-miR-8072, hsa-miR-8075, hsa-miR-8077, hsa-miR-8078, hsa-miR-8089, hsa-miR-7976, hsa-miR-1249-5p, hsa-miR-375-5p, hsa-miR-526a-3p, hsa-miR-9718, hsa-miR-10392-5p, hsa-miR-10392-3p, hsa-miR-10394-3p, hsa-miR-10395-3p, hsa-miR-10396a-5p, hsa-miR-10396a-3p, hsa-miR-10398-3p, hsa-miR-10400-3p, hsa-miR-10401-5p, hsa-miR-10396b-5p, hsa-miR-11399, hsa-miR-12114, hsa-miR-12118, hsa-miR-12119, hsa-miR-12120, and hsa-miR-12121. In the present disclosure, the extract of urine may be an extract of the urine of a stage-I breast cancer patient. Only one of these microRNAs can be used to predict whether a subject from which the urine is derived is a breast cancer patient (particularly stage-I), and 5 or more types, 10 or more types, 15 or more types, or 20 or more types of these microRNAs can be used in combination to predict whether a subject from which the urine is derived is a breast cancer patient (particularly stage-I). Among these microRNAs, a microRNA that may be highly expressed in a breast cancer patient (particularly stage-I) can be preferably used for prediction.

The present disclosure provides an extract of urine containing at least one type, two types, three types, four types, five types, six types, seven types, eight types, nine types, or ten types of or all microRNAs selected from the group consisting of microRNAs that have a percentage of correct answers (accuracy; multiplied by 100 when expressed as a percentage. For example, 0.5 represents 50%) higher than 0.500 among the microRNAs listed in Table 24-2. In the present disclosure, the extract of urine may be an extract of the urine of a breast cancer patient. Only one of these microRNAs can be used to predict whether a subject from which the urine is derived is a breast cancer patient, and 5 or more types, 10 or more types, 15 or more types, or 20 or more types of these microRNAs can be used in combination to predict whether a subject from which the urine is derived is a breast cancer patient. Among these microRNAs, a microRNA that may be highly expressed in a breast cancer patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

The present disclosure also provides an extract of urine containing at least one type, two types, three types, four types, five types, six types, seven types, eight types, nine types, or ten types of or all microRNAs selected from the group consisting of microRNAs having an accuracy of higher than 0.500 among the microRNAs listed in Table 25-2. In the present disclosure, the extract of urine may be an extract of the urine of a breast cancer patient. Only one of these microRNAs can be used to predict whether a subject from which the urine is derived is a breast cancer patient, and 5 or more types, 10 or more types, 15 or more types, or 20 or more types of these microRNAs can be used in combination to predict whether a subject from which the urine is derived is a breast cancer patient. Among these microRNAs, a microRNA that may be highly expressed in a breast cancer patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

Another aspect of the present disclosure provides an extract of urine containing at least one type, two types, three types, four types, five types, six types, seven types, eight types, nine types, or ten types of or all microRNAs selected from the microRNAs listed in Table 4-3. The present disclosure also provides an extract of urine containing at least one type, two types, three types, four types, five types, six types, seven types, eight types, nine types, or ten types of or all microRNAs selected from the group consisting of hsa-miR-498-Sp, hsa-miR-551b-5p, hsa-miR-10526-3p, hsa-miR-150-3p, hsa-miR-5196-5p, hsa-miR-6809-5p, hsa-miR-6783-5p, hsa-miR-3059-3p, hsa-miR-4433b-3p, hsa-miR-6794-5p, hsa-miR-3124-5p, hsa-miR-1207-5p, hsa-miR-424-3p, hsa-miR-629-5p, hsa-miR-4327, hsa-miR-4258, hsa-miR-4447, hsa-miR-6796-3p, hsa-miR-3648, hsa-miR-10392-3p, hsa-miR-7111-5p, hsa-miR-1914-3p, hsa-miR-1268b, hsa-miR-9898, hsa-miR-6805-3p, hsa-miR-4314, hsa-miR-4433b-5p, hsa-miR-6763-3p, hsa-miR-3937, hsa-miR-4433a-5p, hsa-miR-7108-3p, hsa-miR-605-3p, hsa-miR-4433a-3p, hsa-miR-7150, hsa-miR-4665-5p, hsa-miR-6746-5p, hsa-miR-4758-5p, hsa-miR-6781-5p, hsa-miR-6891-3p, hsa-miR-10396a-3p, hsa-miR-4651, hsa-miR-6859-3p, hsa-miR-12116, hsa-miR-1249-3p, hsa-miR-10400-3p, hsa-miR-202-3p, hsa-miR-6829-5p, hsa-miR-6760-3p, hsa-miR-92a-2-5p, hsa-miR-4725-3p, hsa-miR-6784-3p, hsa-miR-1224-3p, hsa-miR-6731-3p, hsa-miR-1228-3p, hsa-miR-3197, hsa-miR-7515, hsa-miR-6125, hsa-miR-6798-3p, hsa-miR-6729-3p, hsa-miR-1343-5p, hsa-miR-6749-5p, hsa-miR-6892-3p, hsa-miR-3162-3p, hsa-miR-4716-5p, hsa-miR-4257, hsa-miR-5703, hsa-miR-10522-5p, hsa-miR-1185-2-3p, hsa-miR-6810-3p, hsa-miR-6515-3p, hsa-miR-6785-3p, hsa-miR-196a-5p, hsa-miR-6832-5p, hsa-miR-4755-3p, hsa-miR-4720-5p, hsa-miR-6812-3p, hsa-miR-3679-3p, hsa-miR-6763-5p, hsa-miR-4750-3p, hsa-miR-6800-3p, hsa-miR-6780b-5p, hsa-miR-6836-3p, hsa-miR-3660, hsa-miR-1185-1-3p, hsa-miR-4429, hsa-miR-6777-3p, hsa-miR-4521, hsa-miR-519c-5p, hsa-miR-523-5p, hsa-miR-518e-5p, hsa-miR-522-5p, hsa-miR-519a-5p, hsa-miR-519b-5p, hsa-miR-6858-3p, hsa-miR-135a-2-3p, hsa-miR-1908-5p, hsa-miR-1238-3p, hsa-miR-4749-3p, hsa-miR-6789-5p, hsa-miR-135a-3p, hsa-miR-2115-5p, hsa-miR-7851-3p, hsa-miR-617, hsa-miR-1913, hsa-miR-4274, hsa-miR-1225-3p, hsa-miR-4749-5p, hsa-miR-6766-3p, hsa-miR-4665-3p, hsa-miR-6813-3p, hsa-miR-6791-5p, hsa-miR-6845-3p, hsa-miR-106a-3p, hsa-miR-6880-3p, hsa-miR-1237-3p, hsa-miR-3186-3p, hsa-miR-4261, hsa-miR-4741, hsa-miR-4745-5p, hsa-miR-7114-3p, hsa-miR-4323, hsa-miR-12120, hsa-miR-4294, hsa-miR-3675-3p, hsa-miR-6795-3p, hsa-miR-766-5p, hsa-miR-12122, hsa-miR-940, hsa-miR-20b-3p, hsa-miR-10401-5p, hsa-miR-12119, hsa-miR-6797-5p, hsa-miR-6750-5p, hsa-miR-4458, hsa-miR-4763-3p, hsa-miR-3141, hsa-miR-8072, hsa-miR-625-3p, hsa-miR-3199, hsa-miR-6819-3p, hsa-miR-6756-3p, hsa-miR-638, hsa-miR-937-5p, hsa-miR-6081, hsa-miR-4313, hsa-miR-1285-3p, hsa-miR-665, hsa-miR-1470, hsa-miR-6893-5p, hsa-miR-4484, hsa-miR-6790-3p, hsa-miR-4450, hsa-miR-4507, hsa-miR-6874-5p, hsa-miR-4505, hsa-miR-6074, hsa-miR-1227-5p, hsa-miR-1915-3p, hsa-miR-151a-5p, hsa-miR-7114-5p, hsa-miR-711, hsa-miR-494-3p, hsa-miR-4728-3p, hsa-miR-7107-5p, hsa-miR-1292-5p, hsa-miR-939-5p, hsa-miR-6884-3p, hsa-miR-1236-3p, hsa-miR-4731-3p, hsa-miR-541-3p, hsa-miR-6743-5p, hsa-miR-5585-3p, hsa-miR-6805-5p, hsa-miR-4299, hsa-miR-6732-5p, hsa-miR-193a-5p, hsa-miR-1225-5p, hsa-miR-18b-3p, hsa-miR-6752-3p, hsa-miR-6887-3p, hsa-miR-302a-3p, hsa-miR-6850-5p, hsa-miR-3150b-5p, hsa-miR-6131, hsa-miR-6870-3p, hsa-miR-6758-5p, hsa-miR-210-5p, hsa-miR-628-3p, hsa-miR-193b-5p, hsa-miR-3682-3p, hsa-miR-6124, hsa-miR-6752-5p, hsa-miR-4435, hsa-miR-4465, hsa-miR-6515-5p, hsa-miR-3614-5p, hsa-miR-4286, hsa-miR-92b-5p, hsa-miR-6516-5p, hsa-miR-6887-5p, hsa-miR-6855-5p, hsa-miR-4300, hsa-miR-1293, hsa-miR-6815-5p, hsa-miR-6165, hsa-miR-4466, hsa-miR-11399, hsa-miR-3184-3p, hsa-miR-6776-5p, hsa-miR-361-3p, hsa-miR-6840-3p, hsa-miR-6847-5p, hsa-miR-6799-5p, hsa-miR-1304-3p, hsa-miR-6845-5p, hsa-miR-3943, hsa-miR-3161, hsa-miR-6777-5p, hsa-miR-4321, hsa-miR-6795-5p, hsa-miR-3162-5p, hsa-miR-6876-5p, hsa-miR-138-5p, hsa-miR-3131, hsa-miR-6798-5p, hsa-miR-3127-3p, hsa-miR-1303, hsa-miR-4283, hsa-miR-10392-5p, hsa-miR-5003-3p, hsa-miR-5685, hsa-miR-6069, hsa-miR-4687-5p, hsa-miR-4701-3p, hsa-miR-7113-5p, hsa-miR-10396b-5p, hsa-miR-4654, hsa-miR-197-5p, hsa-miR-3620-5p, hsa-miR-7109-5p, hsa-miR-1469, hsa-miR-6821-5p, hsa-miR-4530, hsa-miR-5739, hsa-miR-3934-5p, hsa-miR-3085-3p, hsa-miR-320c, hsa-miR-3163, hsa-miR-4472, hsa-miR-6871-5p, hsa-miR-6788-5p, hsa-miR-4648, hsa-miR-601, hsa-miR-5698, hsa-miR-4284, hsa-miR-708-5p, hsa-miR-4784, hsa-miR-6729-5p, hsa-miR-296-5p, hsa-miR-4778-5p, hsa-miR-769-3p, hsa-miR-3689b-3p, hsa-miR-3689c, hsa-miR-1281, hsa-miR-7151-3p, hsa-miR-4716-3p, hsa-miR-4650-3p, hsa-miR-6086, hsa-miR-5009-3p, hsa-miR-6747-5p, hsa-miR-4298, hsa-miR-10398-3p, hsa-miR-6068, hsa-miR-4497, hsa-miR-4474-3p, hsa-miR-4727-3p, hsa-miR-6085, hsa-miR-6879-5p, hsa-miR-211-3p, hsa-miR-365a-5p, hsa-miR-4657, hsa-miR-1236-5p, hsa-miR-4525, hsa-miR-365a-3p, hsa-miR-365b-3p, hsa-miR-6756-5p, hsa-miR-3610, hsa-miR-4487, hsa-miR-6721-5p, hsa-miR-4534, hsa-miR-324-5p, hsa-miR-187-5p, hsa-miR-6127, hsa-miR-4535, hsa-miR-4673, hsa-miR-6889-5p, hsa-miR-6861-5p, hsa-miR-6812-5p, hsa-miR-7154-3p, hsa-miR-3652, hsa-miR-3187-3p, hsa-miR-8082, hsa-miR-6761-5p, hsa-miR-6717-5p, hsa-miR-6748-5p, hsa-miR-6848-3p, hsa-miR-4695-5p, hsa-miR-323b-5p, hsa-miR-3185, hsa-miR-6786-5p, hsa-miR-29a-3p, hsa-miR-6790-5p, hsa-miR-2467-3p, hsa-miR-5004-3p, hsa-miR-4649-3p, hsa-miR-1587, hsa-miR-8064, hsa-miR-6511a-5p, hsa-miR-593-3p, hsa-miR-4734, hsa-miR-8077, hsa-miR-4707-5p, hsa-miR-6797-3p, hsa-miR-4728-5p, hsa-miR-4499, hsa-miR-1471, hsa-miR-6841-3p, hsa-miR-484, hsa-miR-4647, hsa-miR-3133, hsa-miR-5010-5p, hsa-miR-4769-5p, hsa-miR-4506 , hsa-miR-12114, hsa-miR-4280, hsa-miR-4436b-3p, hsa-miR-718, hsa-miR-184, hsa-miR-6529-5p, hsa-miR-5187-5p, hsa-miR-492, hsa-miR-4467, hsa-miR-6823-5p, hsa-miR-6875-5p, hsa-miR-6083, hsa-miR-381-5p, hsa-miR-518b, hsa-miR-30b-3p, hsa-miR-6772-5p, hsa-miR-6782-5p, hsa-miR-4747-5p, hsa-miR-3135b, hsa-miR-3691-5p, hsa-miR-3622a-5p, hsa-miR-6814-5p, hsa-miR-6861-3p, hsa-miR-7156-3p, hsa-miR-1910-3p, hsa-miR-4498, hsa-miR-515-5p, hsa-miR-6892-5p, hsa-miR-8080, hsa-miR-4776-3p, hsa-miR-6859-5p, hsa-miR-4482-3p, hsa-miR-6089, hsa-miR-6514-5p, hsa-miR-6511b-5p, hsa-miR-6759-5p, hsa-miR-6724-5p, hsa-miR-516a-5p, hsa-miR-3180-5p, hsa-miR-1268a, hsa-miR-4428, hsa-miR-125b-2-3p, hsa-miR-204-3p, hsa-miR-504-5p, hsa-miR-3180-3p, hsa-miR-1909-3p, hsa-miR-3619-3p, hsa-miR-5584-5p, hsa-miR-3200-5p, hsa-miR-4638-5p, hsa-miR-6071, hsa-miR-6787-5p, hsa-miR-6839-5p, hsa-miR-130b-3p, hsa-miR-7847-3p, hsa-miR-5194, hsa-miR-5006-5p, hsa-miR-6848-5p, hsa-miR-4688, hsa-miR-6830-5p, hsa-miR-3922-5p, hsa-miR-371b-5p, hsa-miR-6770-5p, hsa-miR-575, hsa-miR-6726-5p, hsa-miR-1306-3p, hsa-miR-5190, hsa-miR-6820-5p, hsa-miR-6741-5p, hsa-miR-595, hsa-miR-6749-3p, hsa-miR-7856-5p, hsa-miR-5090, hsa-miR-4316, hsa-miR-8083, hsa-miR-4783-3p, hsa-miR-370-3p, hsa-miR-3178, hsa-miR-487a-5p, hsa-miR-3917, hsa-miR-921, hsa-miR-6836-5p, hsa-miR-6075, hsa-miR-4664-5p, hsa-miR-5588-3p, hsa-let-7d-3p, hsa-miR-762, hsa-miR-3122, hsa-miR-619-5p, hsa-miR-9718, hsa-miR-584-5p, hsa-miR-6775-3p, hsa-miR-3192-5p, hsa-miR-8071, hsa-miR-4489, hsa-miR-4644, hsa-miR-4692, hsa-miR-4508, hsa-miR-6768-5p, hsa-miR-760, hsa-miR-206, hsa-miR-516b-3p, hsa-miR-516a-3p, hsa-miR-6766-5p, hsa-miR-2681-3p, hsa-miR-6090, hsa-miR-4708-3p, hsa-miR-3196, hsa-miR-4516, hsa-miR-6852-5p, hsa-miR-4769-3p, hsa-miR-6769b-5p, hsa-miR-139-3p, hsa-miR-6796-5p, hsa-miR-7160-5p, hsa-miR-572, hsa-miR-4439, hsa-miR-6076, hsa-miR-7850-5p, hsa-miR-4682, hsa-miR-378j, hsa-miR-4481, hsa-miR-11181-3p, hsa-miR-6793-5p, hsa-miR-6834-5p, hsa-miR-4781-5p, hsa-miR-3158-3p, hsa-miR-3150b-3p, hsa-miR-4317, hsa-miR-3155b, hsa-miR-7107-3p, hsa-miR-25-5p, hsa-miR-4748, hsa-miR-4456, hsa-miR-5093, hsa-miR-5708, hsa-miR-6767-5p, hsa-miR-550a-3-5p, hsa-miR-7108-5p, hsa-miR-933, hsa-miR-4430, hsa-miR-6722-3p, hsa-miR-4478, hsa-miR-650, hsa-miR-6132, hsa-miR-378a-3p, hsa-miR-8085, hsa-miR-4754, hsa-miR-4676-5p, hsa-miR-644a, hsa-miR-1234-3p, hsa-miR-4632-5p, hsa-miR-4531, hsa-miR-7854-3p, hsa-miR-4271, hsa-miR-4640-5p, hsa-miR-6775-5p, hsa-miR-342-5p, hsa-miR-4449, hsa-miR-583, hsa-miR-30c-2-3p, hsa-miR-6504-3p, hsa-miR-5100, hsa-miR-9986, hsa-miR-449b-5p, hsa-miR-11401, hsa-miR-548g-3p, hsa-miR-1204, hsa-miR-105-5p, hsa-miR-4538, hsa-miR-4686, hsa-miR-874-5p, hsa-miR-1237-5p, hsa-miR-4513, hsa-miR-4667-5p, hsa-miR-7110-5p, hsa-miR-663a, hsa-miR-3689a-3p, hsa-miR-4743-5p, hsa-miR-3154, hsa-miR-6760-5p, hsa-miR-7162-3p, hsa-miR-3187-5p, hsa-miR-448, hsa-miR-4486, hsa-miR-525-5p, hsa-miR-4444, hsa-miR-1294, hsa-miR-7106-5p, hsa-miR-3195, hsa-miR-4522, hsa-miR-4463, hsa-miR-6730-5p, hsa-miR-185-3p, hsa-miR-4723-5p, hsa-miR-3619-5p, hsa-miR-6846-5p, hsa-miR-4510, hsa-miR-4779, hsa-miR-5572, hsa-miR-483-5p, hsa-miR-6855-3p, hsa-miR-328-3p, hsa-miR-4475, hsa-miR-4800-5p, hsa-miR-6737-5p, hsa-miR-338-5p, hsa-miR-8069, hsa-miR-1276, hsa-miR-6500-3p, hsa-miR-550b-2-5p, hsa-miR-4440, hsa-miR-3612, hsa-miR-2116-3p, hsa-miR-5189-5p, hsa-miR-183-3p, hsa-miR-373-5p, hsa-miR-4540, hsa-miR-4311, hsa-miR-1468-5p, hsa-miR-12118, hsa-miR-4750-5p, hsa-miR-1298-5p, hsa-miR-8070, hsa-miR-3909, hsa-miR-4303, hsa-miR-4251, hsa-miR-1233-5p, hsa-miR-4306, hsa-miR-6876-3p, hsa-miR-504-3p, hsa-miR-6784-5p, hsa-miR-3617-3p, hsa-miR-4786-5p, hsa-miR-610, hsa-miR-3654, hsa-miR-5189-3p, hsa-miR-200b-3p, hsa-miR-2110, hsa-miR-4685-5p, hsa-miR-6842-5p, hsa-miR-378i, hsa-miR-10396a-5p, hsa-miR-3621, hsa-miR-3064-5p, hsa-miR-1238-5p, hsa-miR-4470, hsa-miR-6837-5p, hsa-miR-486-3p, hsa-miR-4441, hsa-miR-6792-5p, hsa-miR-4421, hsa-miR-520e-3p, hsa-miR-765, hsa-miR-4674, hsa-miR-4707-3p, hsa-miR-6886-5p, hsa-miR-5589-5p, hsa-miR-622, hsa-miR-4684-3p, hsa-miR-6895-5p, hsa-miR-5007-5p, hsa-miR-4756-3p, hsa-miR-6884-5p, hsa-miR-10395-5p, hsa-miR-3928-3p, hsa-miR-658, hsa-miR-520c-3p, hsa-miR-8089, hsa-miR-761, hsa-miR-4736, hsa-miR-433-5p, hsa-miR-1301-5p, hsa-miR-4758-3p, hsa-miR-4723-3p, hsa-miR-4785, hsa-miR-3137, hsa-miR-6802-3p, hsa-miR-2276-3p, hsa-miR-4717-5p, hsa-miR-99b-3p, hsa-miR-149-3p, hsa-miR-323a-3p, hsa-miR-4794, hsa-miR-4496, hsa-miR-4437, hsa-miR-8078, hsa-miR-16-2-3p, hsa-miR-885-3p, hsa-miR-6512-3p, hsa-miR-1266-3p, hsa-miR-1843, hsa-miR-3667-5p, hsa-miR-4446-3p, hsa-miR-6825-5p, hsa-miR-6510-5p, hsa-miR-6880-5p, hsa-miR-3665, hsa-miR-6762-3p, hsa-miR-1289, hsa-miR-6831-5p, hsa-miR-4451, hsa-miR-3934-3p, hsa-miR-491-5p, hsa-miR-6833-5p, hsa-miR-6868-3p, hsa-miR-4526, hsa-miR-4786-3p, hsa-miR-34a-5p, hsa-miR-4738-3p, hsa-miR-3151-5p, hsa-miR-520b-3p, hsa-miR-4448, hsa-miR-4304, hsa-miR-5580-5p, hsa-miR-6885-3p, hsa-miR-6877-5p, hsa-miR-6740-5p, hsa-miR-373-3p, hsa-miR-3169, hsa-miR-5002-3p, hsa-miR-4669, hsa-miR-4431, hsa-miR-8075, hsa-miR-26b-3p, hsa-miR-23a-5p, hsa-miR-4656, hsa-miR-302d-5p, hsa-miR-6738-3p, hsa-miR-6793-3p, hsa-miR-564, hsa-miR-4706, hsa-miR-3155a, hsa-miR-4454, hsa-miR-887-3p, hsa-miR-1269a, hsa-miR-6770-3p, hsa-miR-200c-5p, hsa-miR-557, hsa-miR-5690, hsa-miR-6773-3p, hsa-miR-1972, hsa-miR-6843-3p, hsa-miR-652-5p, hsa-miR-34c-5p, hsa-miR-550a-5p, hsa-miR-642a-3p, hsa-miR-6822-3p, hsa-miR-5787, hsa-miR-4776-5p, hsa-miR-1301-3p, hsa-miR-3679-5p, hsa-miR-7152-3p, hsa-miR-8057, hsa-miR-296-3p, hsa-miR-6849-5p, hsa-miR-6801-5p, hsa-miR-3138, hsa-miR-1224-5p, hsa-miR-3142, hsa-miR-6869-5p, hsa-miR-500b-3p, hsa-miR-4740-3p, hsa-miR-877-5p, hsa-miR-18a-3p, hsa-miR-548q, hsa-miR-4483, hsa-miR-1270, hsa-miR-374a-3p, hsa-miR-200c-3p, hsa-miR-125b-1-3p, hsa-miR-3176, hsa-miR-122b-3p, hsa-miR-5088-5p, hsa-miR-6813-5p, hsa-miR-4533, hsa-miR-6778-5p, hsa-miR-6126, hsa-miR-3120-3p, hsa-miR-920, hsa-miR-12127, hsa-let-7f-1-3p, hsa-miR-302c-5p, hsa-miR-4658, hsa-miR-4539, hsa-miR-323a-5p, hsa-miR-6769a-5p, hsa-miR-4265, hsa-miR-1322, hsa-miR-6862-5p, hsa-miR-6727-5p, hsa-miR-342-3p, hsa-miR-4713-3p, hsa-miR-6889-3p, hsa-miR-4455, hsa-miR-214-3p, hsa-miR-6894-5p, hsa-miR-3945, hsa-miR-4726-3p, hsa-miR-6501-3p, hsa-miR-3085-5p, hsa-miR-4787-5p, hsa-miR-6856-5p, hsa-miR-501-5p, hsa-miR-6870-5p, hsa-miR-598-5p, hsa-miR-370-5p, hsa-miR-6742-5p, hsa-miR-1247-3p, hsa-miR-6129, hsa-miR-6728-5p, hsa-miR-3173-5p, hsa-miR-3649, hsa-miR-585-5p, hsa-miR-3190-3p, hsa-miR-1203, hsa-miR-466, hsa-miR-4746-3p, hsa-miR-892b, hsa-miR-632, hsa-miR-5705, hsa-miR-3646, hsa-miR-5581-5p, hsa-miR-6738-5p, hsa-miR-642b-3p, hsa-miR-133a-3p, hsa-miR-608, hsa-miR-3125, hsa-miR-3675-5p, hsa-miR-4634, hsa-miR-6792-3p, hsa-miR-138-2-3p, hsa-miR-198, hsa-miR-128-1-5p, hsa-miR-4296, hsa-miR-3177-3p, hsa-miR-6734-5p, hsa-miR-6857-5p, hsa-miR-345-3p, hsa-miR-4260, hsa-miR-4639-3p, hsa-miR-6762-5p, hsa-miR-485-5p, hsa-miR-4255, hsa-miR-1229-5p, hsa-miR-1538, hsa-miR-3940-5p, hsa-miR-493-3p, hsa-miR-4780, hsa-miR-668-5p, hsa-miR-3689d, hsa-miR-6753-5p, hsa-miR-1193, hsa-miR-4640-3p, hsa-miR-604, hsa-miR-4672, hsa-miR-664b-5p, hsa-miR-15b-3p, hsa-miR-1912-3p, hsa-miR-6883-5p, hsa-miR-3074-3p, hsa-miR-6807-5p, hsa-miR-1273h-5p, hsa-miR-5584-3p, hsa-miR-3126-5p, hsa-miR-6810-5p, hsa-miR-3153, hsa-miR-3622b-5p, hsa-miR-1343-3p, hsa-miR-513a-5p, hsa-miR-585-3p, hsa-miR-563, hsa-miR-4436a, hsa-miR-3935, hsa-miR-6772-3p, hsa-miR-615-5p, hsa-miR-6803-5p, hsa-miR-1182, hsa-miR-6838-5p, hsa-miR-4259, hsa-miR-7846-3p, hsa-miR-670-5p, hsa-miR-551a, hsa-miR-6779-5p, hsa-miR-12121, hsa-miR-371b-3p, hsa-miR-6826-3p, hsa-miR-4709-5p, hsa-miR-4293, hsa-miR-6820-3p, hsa-miR-6827-5p, hsa-miR-4700-5p, hsa-miR-3663-3p, hsa-miR-25-3p, hsa-miR-2277-5p, hsa-miR-4325, hsa-miR-185-5p, hsa-miR-1227-3p, hsa-let-7b-3p, hsa-miR-103a-1-5p, hsa-miR-6501-5p, hsa-miR-4502, hsa-miR-4322, hsa-miR-34c-3p, hsa-miR-6757-5p, hsa-miR-639, hsa-miR-421, hsa-miR-6860, hsa-miR-194-3p, hsa-miR-7704, hsa-miR-6751-5p, hsa-miR-8088, hsa-miR-769-5p, hsa-miR-3180, hsa-miR-3651, hsa-miR-3189-3p, hsa-miR-6130, hsa-miR-3132, hsa-miR-6801-3p, hsa-miR-8063, hsa-miR-4664-3p, hsa-miR-4788, hsa-miR-6719-3p, hsa-miR-4288, hsa-miR-4691-5p, hsa-miR-6133, hsa-miR-3144-5p, hsa-miR-1304-5p, hsa-miR-6824-5p, hsa-miR-6822-5p, hsa-miR-7975, hsa-miR-449c-3p, hsa-miR-574-5p, hsa-miR-3173-3p, hsa-miR-371a-5p, hsa-miR-3663-5p, hsa-miR-3685, hsa-miR-11400, hsa-miR-4753-5p, hsa-miR-4721, hsa-miR-635, hsa-miR-6780a-5p, hsa-miR-1307-3p, hsa-miR-3127-5p, hsa-miR-12124, hsa-miR-6816-3p, hsa-miR-3198, hsa-miR-134-3p, hsa-miR-6754-5p, hsa-miR-8086, hsa-miR-4520-5p, hsa-miR-4708-5p, hsa-miR-2116-5p, hsa-miR-6503-3p, hsa-miR-1226-5p, hsa-miR-6782-3p, hsa-miR-541-5p, hsa-miR-6816-5p, hsa-miR-4252, hsa-miR-4295, hsa-miR-5001-5p, hsa-miR-550b-3p, hsa-miR-6754-3p, hsa-miR-6759-3p, hsa-miR-4717-3p, hsa-miR-4297, hsa-miR-4667-3p, hsa-miR-3191-3p, hsa-miR-6895-3p, hsa-miR-660-3p, hsa-miR-4276, hsa-miR-1202, hsa-miR-2113, hsa-miR-4737, hsa-miR-4529-5p, hsa-miR-4710, hsa-miR-7111-3p, hsa-miR-4732-5p, hsa-miR-3944-3p, hsa-miR-4711-3p, hsa-miR-2392, hsa-miR-5004-5p, hsa-miR-8073, hsa-miR-3925-5p, hsa-miR-4492, hsa-miR-7152-5p, hsa-miR-125a-3p, hsa-miR-4697-5p, hsa-miR-6857-3p, hsa-miR-6873-5p, hsa-miR-8052, hsa-miR-129-5p, hsa-miR-486-5p, hsa-miR-514b-5p, hsa-miR-449a, hsa-miR-3150a-3p, hsa-miR-8074, hsa-miR-3616-3p, hsa-miR-4655-5p, hsa-miR-378g, hsa-miR-7977, hsa-miR-4476, hsa-miR-1284, hsa-miR-3944-5p, hsa-miR-3529-5p, hsa-miR-34b-3p, hsa-miR-1249-5p, hsa-miR-10527-5p, hsa-miR-766-3p, hsa-miR-6514-3p, hsa-miR-6718-5p, hsa-miR-12136, hsa-miR-3158-5p, hsa-miR-5006-3p, hsa-miR-4269, hsa-miR-3919, hsa-miR-138-1-3p, hsa-miR-200a-5p, hsa-miR-3156-5p, hsa-miR-4488, hsa-miR-4326, hsa-miR-12115, hsa-miR-4514, hsa-miR-7113-3p, hsa-miR-4253, hsa-miR-3147, hsa-miR-487b-5p, hsa-miR-410-5p, hsa-miR-6728-3p, hsa-miR-1909-5p, hsa-miR-6881-5p, hsa-miR-200b-5p, hsa-miR-1296-3p, hsa-miR-6817-5p, hsa-miR-7855-5p, hsa-miR-429, hsa-miR-637, hsa-miR-654-5p, hsa-miR-5195-3p, hsa-miR-6764-3p, hsa-let-7e-5p, hsa-miR-6789-3p, hsa-miR-4759, hsa-miR-9902, hsa-miR-3605-5p, hsa-miR-6872-5p, hsa-miR-877-3p, hsa-miR-6846-3p, hsa-miR-7845-5p, hsa-miR-3186-5p, hsa-miR-4485-5p, hsa-miR-378b, hsa-miR-938, hsa-miR-320e, hsa-miR-8087, hsa-miR-3918, hsa-miR-3960, hsa-miR-6509-5p, hsa-miR-1184, hsa-miR-6722-5p, hsa-miR-888-3p, hsa-miR-6511b-3p, hsa-miR-494-5p, hsa-miR-1267, hsa-miR-505-5p, hsa-miR-8060, hsa-miR-382-5p, hsa-miR-133b, hsa-miR-647, hsa-miR-6875-3p, hsa-miR-151b, hsa-miR-219a-2-3p, hsa-miR-7976, hsa-miR-1291, hsa-miR-3692-5p, hsa-miR-365b-5p, hsa-miR-5588-5p, hsa-miR-106b-5p, hsa-miR-3936, hsa-miR-12125, hsa-miR-4730, hsa-miR-548d-3p, hsa-miR-513b-5p, hsa-miR-612, hsa-miR-656-5p, hsa-miR-208a-5p, hsa-miR-8059, hsa-miR-6744-5p, hsa-miR-5699-5p, hsa-miR-892c-5p, hsa-miR-4660, hsa-miR-4443, hsa-miR-6858-5p, hsa-miR-1269b, hsa-miR-4689, hsa-miR-431-3p, hsa-miR-744-5p, hsa-miR-6786-3p, hsa-miR-6716-5p, hsa-miR-187-3p, hsa-miR-758-5p, hsa-miR-10401-3p, hsa-miR-221-3p, hsa-miR-186-3p, hsa-miR-4740-5p, hsa-miR-6828-5p, hsa-miR-6868-5p, hsa-miR-6800-5p, hsa-miR-642b-5p, hsa-miR-214-5p, hsa-miR-629-3p, hsa-miR-10400-5p, hsa-miR-300, hsa-miR-6731-5p, hsa-miR-1251-3p, hsa-miR-6893-3p, hsa-miR-603, hsa-miR-8062, hsa-miR-1321, hsa-miR-488-5p, hsa-miR-4524b-3p, hsa-miR-6806-5p, hsa-miR-4731-5p, hsa-miR-6854-3p, hsa-miR-3130-3p, hsa-miR-3622a-3p, hsa-miR-6506-5p, hsa-miR-1288-3p, hsa-miR-222-5p, hsa-miR-4319, hsa-miR-330-5p, hsa-miR-3150a-5p, hsa-miR-7109-3p, hsa-miR-6745, hsa-miR-196b-3p, hsa-miR-3661, hsa-miR-10398-5p, hsa-miR-634, hsa-miR-646, hsa-miR-614, hsa-miR-7157-5p, hsa-miR-6787-3p, hsa-miR-192-5p, hsa-miR-450a-2-3p, hsa-miR-4635, hsa-miR-3064-3p, hsa-miR-3157-5p, hsa-miR-4756-5p, hsa-miR-578, hsa-miR-671-5p, hsa-miR-4684-5p, hsa-miR-6808-5p, hsa-miR-3650, hsa-miR-4652-5p, hsa-miR-6821-3p, hsa-miR-7155-3p, hsa-miR-6783-3p, hsa-miR-5188, hsa-miR-6072, hsa-miR-671-3p, hsa-miR-3975, hsa-miR-4697-3p, hsa-miR-1275, hsa-miR-7151-5p, hsa-miR-515-3p, hsa-miR-412-3p, hsa-miR-1228-5p, hsa-miR-6080, hsa-miR-4793-3p, hsa-miR-297, hsa-miR-3691-3p, hsa-miR-5571-3p, hsa-miR-320d, hsa-miR-4479, hsa-miR-891a-5p, hsa-miR-589-3p, hsa-miR-4425, hsa-miR-3116, hsa-miR-4308, hsa-miR-4653-3p, hsa-miR-3120-5p, hsa-miR-661, hsa-miR-23b-3p, hsa-miR-4690-5p, hsa-miR-6851-5p, hsa-miR-6765-5p, hsa-miR-6840-5p, hsa-miR-6867-5p, hsa-miR-6715b-5p, hsa-miR-4494, hsa-miR-4462, hsa-miR-4677-5p, hsa-miR-615-3p, hsa-miR-106b-3p, hsa-miR-192-3p, hsa-miR-134-5p, hsa-miR-542-5p, hsa-miR-4681, hsa-miR-885-5p, hsa-miR-181d-3p, hsa-miR-3928-5p, hsa-miR-6736-3p, hsa-miR-520d-3p, hsa-miR-6791-3p, hsa-miR-506-3p, hsa-miR-3202, hsa-miR-6504-5p, hsa-miR-4801-3p, hsa-miR-6851-3p, hsa-miR-4767, hsa-miR-10a-5p, hsa-miR-103b, hsa-miR-4752, hsa-miR-6804-5p, hsa-miR-1250-3p, hsa-miR-6883-3p, hsa-miR-377-5p, hsa-miR-12126, hsa-miR-423-5p, hsa-miR-5681b, hsa-miR-1231, hsa-miR-5195-5p, hsa-miR-935, hsa-miR-4712-5p, hsa-miR-6830-3p, hsa-miR-6891-5p, hsa-miR-5002-5p, hsa-miR-4438, hsa-miR-3677-3p, hsa-miR-1908-3p, hsa-miR-6742-3p, hsa-miR-4266, hsa-miR-4491, hsa-miR-3911, hsa-miR-1297, hsa-miR-4727-5p, hsa-miR-4436b-5p, hsa-miR-518c-5p, hsa-miR-1255b-2-3p, hsa-miR-4687-3p, hsa-miR-6739-3p, hsa-miR-6882-3p, hsa-miR-30c-1-3p, hsa-miR-328-5p, hsa-miR-3714, hsa-miR-21-3p, hsa-miR-4537, hsa-miR-3922-3p, hsa-miR-892c-3p, hsa-miR-1976, hsa-miR-584-3p, hsa-miR-3130-5p, hsa-miR-6865-3p, hsa-miR-29c-5p, hsa-miR-6890-3p, hsa-miR-27b-3p, hsa-miR-1257, hsa-miR-518f-3p, hsa-miR-3188, hsa-miR-7843-5p, hsa-miR-4646-5p, hsa-miR-363-5p, hsa-miR-6833-3p, hsa-miR-4773, hsa-miR-3165, hsa-miR-127-5p, hsa-miR-223-3p, hsa-miR-2682-5p, hsa-miR-6509-3p, hsa-miR-369-5p, hsa-miR-6852-3p, hsa-miR-5001-3p, hsa-miR-146b-3p, hsa-miR-936, hsa-miR-6776-3p, hsa-miR-6715a-3p, hsa-miR-3160-3p, hsa-miR-6878-3p, hsa-miR-6513-5p, hsa-miR-3059-5p, hsa-miR-4709-3p, hsa-miR-4650-5p, hsa-miR-6774-5p, hsa-miR-6769a-3p, hsa-miR-770-5p, hsa-miR-5010-3p, hsa-miR-4698, hsa-miR-210-3p, hsa-miR-212-5p, hsa-miR-6819-5p, hsa-miR-675-5p, hsa-miR-1915-5p, hsa-miR-4307, hsa-miR-4763-5p, hsa-miR-6757-3p, hsa-miR-625-5p, hsa-miR-3189-5p, hsa-miR-6088, hsa-miR-4423-5p, hsa-miR-4725-5p, hsa-miR-5704, hsa-miR-6809-3p, hsa-miR-4714-5p, hsa-miR-5580-3p, hsa-miR-4999-5p, hsa-miR-558, hsa-miR-2278, hsa-miR-181a-2-3p, hsa-miR-29b-3p, hsa-miR-6499-3p, hsa-miR-512-5p, hsa-miR-3126-3p, hsa-miR-6744-3p, hsa-miR-325, hsa-miR-487a-3p, hsa-miR-4518, hsa-miR-4739, hsa-miR-6077, hsa-miR-6740-3p, hsa-miR-1250-5p, hsa-miR-301a-5p, hsa-miR-8485, hsa-miR-1263, hsa-miR-4732-3p, hsa-miR-3659, hsa-miR-147a, hsa-miR-6529-3p, hsa-miR-668-3p, hsa-miR-1266-5p, hsa-miR-3160-5p, hsa-miR-6882-5p, hsa-miR-943, hsa-miR-6850-3p, hsa-miR-30d-Sp, hsa-miR-6853-5p, hsa-miR-676-Sp, hsa-miR-6736-5p, and hsa-miR-29b-2-5p. In the present disclosure, the extract of urine may be an extract of the urine of a kidney cancer patient. Only one of these microRNAs can be used to predict whether a subject from which the urine is derived is a kidney cancer patient, and 5 or more types, 10 or more types, 15 or more types, or 20 or more types of these microRNAs can be used in combination to predict whether a subject from which the urine is derived is a kidney cancer patient. Among these microRNAs, a microRNA that may be highly expressed in a kidney cancer patient can be preferably used for prediction.

The present disclosure provides an extract of urine containing at least one type, two types, three types, four types, five types, six types, seven types, eight types, nine types, or ten types of or all microRNAs selected from the group consisting of hsa-miR-29a-3p, hsa-miR-30a-5p, hsa-miR-29b-3p, hsa-miR-196a-5p, hsa-miR-197-3p, hsa-miR-198, hsa-miR-199a-5p, hsa-miR-30d-5p, hsa-miR-10a-5p, hsa-miR-187-3p, hsa-miR-199b-5p, hsa-miR-214-3p, hsa-miR-223-3p, hsa-miR-133a-3p, hsa-miR-125a-5p, hsa-miR-134-5p, hsa-miR-149-5p, hsa-miR-184, hsa-miR-185-5p, hsa-miR-206, hsa-miR-200c-3p, hsa-miR-106b-5p, hsa-miR-29c-3p, hsa-miR-302a-3p, hsa-miR-299-3p, hsa-miR-296-5p, hsa-miR-130b-3p, hsa-miR-361-Sp, hsa-miR-365a-3p, hsa-miR-365b-3p, hsa-miR-302c-Sp, hsa-miR-370-3p, hsa-miR-373-5p, hsa-miR-373-3p, hsa-miR-378a-3p, hsa-miR-379-5p, hsa-miR-380-3p, hsa-miR-328-3p, hsa-miR-342-3p, hsa-miR-337-3p, hsa-miR-326, hsa-miR-133b, hsa-miR-325, hsa-miR-346, hsa-miR-20b-5p, hsa-miR-448, hsa-miR-429, hsa-miR-449a, hsa-miR-191-3p, hsa-miR-200a-5p, hsa-miR-409-3p, hsa-miR-483-3p, hsa-miR-484, hsa-miR-485-3p, hsa-miR-486-5p, hsa-miR-491-5p, hsa-miR-202-3p, hsa-miR-492, hsa-miR-494-3p, hsa-miR-512-5p, hsa-miR-498-5p, hsa-miR-520e-3p, hsa-miR-519c-5p, hsa-miR-523-5p, hsa-miR-518e-5p, hsa-miR-522-5p, hsa-miR-519a-5p, hsa-miR-519b-5p, hsa-miR-526b-5p, hsa-miR-525-5p, hsa-miR-518f-3p, hsa-miR-520b-3p, hsa-miR-518b, hsa-miR-526a-5p, hsa-miR-520c-5p, hsa-miR-518d-5p, hsa-miR-520c-3p, hsa-miR-518c-5p, hsa-miR-520d-5p, hsa-miR-520d-3p, hsa-miR-516b-5p, hsa-miR-527, hsa-miR-518a~5p, hsa-miR-519a-3p, hsa-miR-502-5p, hsa-miR-513a-5p, hsa-miR-557, hsa-miR-563, hsa-miR-564, hsa-miR-574-3p, hsa-miR-575, hsa-miR-578, hsa-miR-579-3p, hsa-miR-583, hsa-miR-585-3p, hsa-miR-588, hsa-miR-550a-3p, hsa-miR-595, hsa-miR-601, hsa-miR-608, hsa-miR-610, hsa-miR-612, hsa-miR-615-3p, hsa-miR-617, hsa-miR-624-5p, hsa-miR-625-5p, hsa-miR-628-3p, hsa-miR-632, hsa-miR-634, hsa-miR-636, hsa-miR-637, hsa-miR-638, hsa-miR-639, hsa-miR-641, hsa-miR-650, hsa-miR-663a, hsa-miR-449b-5p, hsa-miR-654-5p, hsa-miR-657, hsa-miR-658, hsa-miR-542-5p, hsa-miR-671-Sp, hsa-miR-668-3p, hsa-miR-767-3p, hsa-miR-769-5p, hsa-miR-769-3p, hsa-miR-766-3p, hsa-miR-765, hsa-miR-675-5p, hsa-miR-297, hsa-let-7b-3p, hsa-let-7d-3p, hsa-let-7f-1-3p, hsa-miR-25-5p, hsa-miR-26b-3p, hsa-miR-92a-2-5p, hsa-miR-29b-2-5p, hsa-miR-30c-2-3p, hsa-miR-139-3p, hsa-miR-181c-3p, hsa-miR-183-3p, hsa-miR-187-5p, hsa-miR-15b-3p, hsa-miR-124-Sp, hsa-miR-125b-1-3p, hsa-miR-130a-Sp, hsa-miR-135a-3p, hsa-miR-125a-3p, hsa-miR-127-5p, hsa-miR-149-3p, hsa-miR-150-3p, hsa-miR-185-3p, hsa-miR-186-3p, hsa-miR-193a-5p, hsa-miR-194-3p, hsa-miR-30c-1-3p, hsa-miR-296-3p, hsa-miR-361-3p, hsa-miR-302d-5p, hsa-miR-371a-5p, hsa-miR-374a-3p, hsa-miR-377-5p, hsa-miR-342-5p, hsa-miR-423-5p, hsa-miR-424-3p, hsa-miR-18b-3p, hsa-miR-20b-3p, hsa-miR-431-3p, hsa-miR-483-5p, hsa-miR-486-3p, hsa-miR-193b-5p, hsa-miR-505-5p, hsa-miR-532-3p, hsa-miR-92b-5p, hsa-miR-551b-5p, hsa-miR-574-5p, hsa-miR-550a-5p, hsa-miR-615-5p, hsa-miR-616-3p, hsa-miR-625-3p, hsa-miR-629-5p, hsa-miR-298, hsa-miR-874-3p, hsa-miR-876-3p, hsa-miR-744-5p, hsa-miR-885-5p, hsa-miR-885-3p, hsa-miR-877-5p, hsa-miR-877-3p, hsa-miR-887-3p, hsa-miR-665, hsa-miR-760, hsa-miR-920, hsa-miR-921, hsa-miR-509-3-5p, hsa-miR-933, hsa-miR-936, hsa-miR-939-5p, hsa-miR-940, hsa-miR-1224-5p, hsa-miR-1224-3p, hsa-miR-1225-5p, hsa-miR-1225-3p, hsa-miR-1227-3p, hsa-miR-1228-5p, hsa-miR-1228-3p, hsa-miR-1229-3p, hsa-miR-1231, hsa-miR-1233-3p, hsa-miR-1234-3p, hsa-miR-1236-3p, hsa-miR-1237-3p, hsa-miR-1238-3p, hsa-miR-320b, hsa-miR-320c, hsa-miR-1323, hsa-miR-1301-3p, hsa-miR-1298-5p, hsa-miR-1181, hsa-miR-1182, hsa-miR-1184, hsa-miR-1202, hsa-miR-1203, hsa-miR-663b, hsa-miR-1207-5p, hsa-miR-1207-3p, hsa-miR-1289, hsa-miR-1294, hsa-miR-1299, hsa-miR-1303, hsa-miR-1304-5p, hsa-miR-1249-3p, hsa-miR-1257, hsa-miR-1260a, hsa-miR-1263, hsa-miR-1267, hsa-miR-1268a, hsa-miR-1270, hsa-miR-1275, hsa-miR-1281, hsa-miR-1292-5p, hsa-miR-664a-3p, hsa-miR-1307-3p, hsa-miR-320d, hsa-miR-1825, hsa-miR-675-3p, hsa-miR-1469, hsa-miR-1470, hsa-miR-1471, hsa-miR-1539, hsa-miR-1908-5p, hsa-miR-1909-5p, hsa-miR-1909-3p, hsa-miR-1910-5p, hsa-miR-1912-3p, hsa-miR-1913, hsa-miR-1914-3p, hsa-miR-1915-3p, hsa-miR-365a-5p, hsa-miR-449b-3p, hsa-miR-2113, hsa-miR-1976, hsa-miR-2110, hsa-miR-151b, hsa-miR-762, hsa-miR-670-5p, hsa-miR-764, hsa-miR-2116-5p, hsa-miR-2116-3p, hsa-miR-548q, hsa-miR-2276-3p, hsa-miR-2278, hsa-miR-711, hsa-miR-718, hsa-miR-2681-3p, hsa-miR-2682-3p, hsa-miR-449c-3p, hsa-miR-2861, hsa-miR-3116, hsa-miR-3119, hsa-miR-3120-3p, hsa-miR-3122, hsa-miR-3124-5p, hsa-miR-3126-5p, hsa-miR-3130-5p, hsa-miR-3131, hsa-miR-3132, hsa-miR-3133, hsa-miR-466, hsa-miR-3136-5p, hsa-miR-3138, hsa-miR-3141, hsa-miR-3144-5p, hsa-miR-1273c, hsa-miR-3147, hsa-miR-3150a-3p, hsa-miR-3151-5p, hsa-miR-3153, hsa-miR-3154, hsa-miR-3156-5p, hsa-miR-3157-5p, hsa-miR-3160-3p, hsa-miR-3161, hsa-miR-3162-5p, hsa-miR-3163, hsa-miR-3164, hsa-miR-3165, hsa-miR-1260b, hsa-miR-3169, hsa-miR-3173-3p, hsa-miR-1193, hsa-miR-3175, hsa-miR-3177-3p, hsa-miR-3178, hsa-miR-3179, hsa-miR-3180-5p, hsa-miR-3180-3p, hsa-miR-3184-5p, hsa-miR-3185, hsa-miR-3186-3p, hsa-miR-3188, hsa-miR-3189-3p, hsa-miR-3190-5p, hsa-miR-3191-3p, hsa-miR-3194-5p, hsa-miR-3195, hsa-miR-3196, hsa-miR-3197, hsa-miR-3198, hsa-miR-3199, hsa-miR-514b-5p, hsa-miR-3202, hsa-miR-4297, hsa-miR-4293, hsa-miR-4294, hsa-miR-4299, hsa-miR-4298, hsa-miR-4305, hsa-miR-4306, hsa-miR-4307, hsa-miR-4312, hsa-miR-4313, hsa-miR-4316, hsa-miR-4314, hsa-miR-4322, hsa-miR-4321, hsa-miR-4323, hsa-miR-4256, hsa-miR-4257, hsa-miR-4258, hsa-miR-4259, hsa-miR-4260, hsa-miR-4253, hsa-miR-4251, hsa-miR-432 5, hsa-miR-4326, hsa-miR-4327, hsa-miR-4261, hsa-miR-4265, hsa-miR-4266, hsa-miR-2355-5p, hsa-miR-4268, hsa-miR-4269, hsa-miR-4271, hsa-miR-4276, hsa-miR-4274, hsa-miR-4281, hsa-miR-4279, hsa-miR-4278, hsa-miR-4285, hsa-miR-4283, hsa-miR-4284, hsa-miR-4286, hsa-miR-4287, hsa-miR-4292, hsa-miR-4290, hsa-miR-4329, hsa-miR-3200-5p, hsa-miR-3605-3p, hsa-miR-3610, hsa-miR-3612, hsa-miR-3614-5p, hsa-miR-3616-3p, hsa-miR-3620-3p, hsa-miR-3621, hsa-miR-3622a-5p, hsa-miR-3622a-3p, hsa-miR-3622b-5p, hsa-miR-3648, hsa-miR-3649, hsa-miR-3652, hsa-miR-3660, hsa-miR-3663-5p, hsa-miR-3663-3p, hsa-miR-3665, hsa-miR-3667-5p, hsa-miR-3667-3p, hsa-miR-3675-3p, hsa-miR-3677-3p, hsa-miR-3679-5p, hsa-miR-3679-3p, hsa-miR-3689a-3p, hsa-miR-3691-5p, hsa-miR-3714, hsa-miR-3180, hsa-miR-3907, hsa-miR-3689b-3p, hsa-miR-3689c, hsa-miR-3909, hsa-miR-3911, hsa-miR-3917, hsa-miR-3919, hsa-miR-3150b-3p, hsa-miR-3925-5p, hsa-miR-3928-3p, hsa-miR-3935, hsa-miR-3936, hsa-miR-3937, hsa-miR-3941, hsa-miR-3942-5p, hsa-miR-3943, hsa-miR-3944-3p, hsa-miR-3945, hsa-miR-642b-3p, hsa-miR-550b-3p, hsa-miR-1268b, hsa-miR-548ab, hsa-miR-4421, hsa-miR-4428, hsa-miR-4429, hsa-miR-4430, hsa-miR-4433a-3p, hsa-miR-4439, hsa-miR-4440, hsa-miR-4441, hsa-miR-4443, hsa-miR-4444, hsa-miR-4446-3p, hsa-miR-4447, hsa-miR-4448, hsa-miR-4449, hsa-miR-4455, hsa-miR-4456, hsa-miR-4458, hsa-miR-4460, hsa-miR-378h, hsa-miR-3135b, hsa-miR-4462, hsa-miR-4463, hsa-miR-4465, hsa-miR-4466, hsa-miR-4467, hsa-miR-4468, hsa-miR-4470, hsa-miR-4472, hsa-miR-4474-3p, hsa-miR-4475, hsa-miR-4476, hsa-miR-4478, hsa-miR-3689d, hsa-miR-4481, hsa-miR-4483, hsa-miR-4484, hsa-miR-4486, hsa-miR-4487, hsa-miR-4488, hsa-miR-4489, hsa-miR-4492, hsa-miR-4496, hsa-miR-4497, hsa-miR-4498, hsa-miR-4499, hsa-miR-4501, hsa-miR-4505, hsa-miR-4506, hsa-miR-2392, hsa-miR-4507, hsa-miR-4508, hsa-miR-4510, hsa-miR-4513, hsa-miR-4516, hsa-miR-4518, hsa-miR-4521, hsa-miR-1269b, hsa-miR-4522, hsa-miR-4523, hsa-miR-4525, hsa-miR-4526, hsa-miR-4530, hsa-miR-4531, hsa-miR-4533, hsa-miR-4534, hsa-miR-4535, hsa-miR-1587, hsa-miR-4538, hsa-miR-4539, hsa-miR-3127-3p, hsa-miR-3156-3p, hsa-miR-3158-5p, hsa-miR-3162-3p, hsa-miR-3173-5p, hsa-miR-3187-5p, hsa-miR-3189-5p, hsa-miR-3619-3p, hsa-miR-3150b-5p, hsa-miR-3940-5p, hsa-miR-3960, hsa-miR-3972, hsa-miR-3978, hsa-miR-4634, hsa-miR-4638-5p, hsa-miR-4639-3p, hsa-miR-4640-5p, hsa-miR-4640-3p, hsa-miR-4642, hsa-miR-4646-5p, hsa-miR-4646-3p, hsa-miR-4648, hsa-miR-4649-5p, hsa-miR-4649-3p, hsa-miR-4650-3p, hsa-miR-4651, hsa-miR-4652-5p, hsa-miR-4653-3p, hsa-miR-4654, hsa-miR-4655-5p, hsa-miR-4656, hsa-miR-4659b-3p, hsa-miR-4664-5p, hsa-miR-4664-3p, hsa-miR-4665-5p, hsa-miR-4665-3p, hsa-miR-4667-5p, hsa-miR-4667-3p, hsa-miR-4669, hsa-miR-4672, hsa-miR-4673, hsa-miR-4674, hsa-miR-4675, hsa-miR-4677-5p, hsa-miR-4685-5p, hsa-miR-4685-3p, hsa-miR-4687-5p, hsa-miR-4687-3p, hsa-miR-1343-3p, hsa-miR-4688, hsa-miR-4689, hsa-miR-4690-5p, hsa-miR-4691-5p, hsa-miR-4695-5p, hsa-miR-4695-3p, hsa-miR-4697-5p, hsa-miR-4697-3p, hsa-miR-4698, hsa-miR-4700-5p, hsa-miR-4700-3p, hsa-miR-4701-5p, hsa-miR-4701-3p, hsa-miR-4706, hsa-miR-4707-5p, hsa-miR-4707-3p, hsa-miR-4708-3p, hsa-miR-4709-3p, hsa-miR-4710, hsa-miR-4713-5p, hsa-miR-4714-5p, hsa-miR-4716-5p, hsa-miR-4716-3p, hsa-miR-4717-3p, hsa-miR-4720-5p, hsa-miR-4721, hsa-miR-4722-5p, hsa-miR-4722-3p, hsa-miR-4723-5p, hsa-miR-4723-3p, hsa-miR-4725-3p, hsa-miR-4726-5p, hsa-miR-4727-3p, hsa-miR-4728-5p, hsa-miR-4728-3p, hsa-miR-4729, hsa-miR-4730, hsa-miR-4731-5p, hsa-miR-4731-3p, hsa-miR-4732-5p, hsa-miR-4732-3p, hsa-miR-4734, hsa-miR-4736, hsa-miR-3064-5p, hsa-miR-3064-3p, hsa-miR-4738-3p, hsa-miR-4739, hsa-miR-4741, hsa-miR-4743-5p, hsa-miR-4745-5p, hsa-miR-4746-3p, hsa-miR-4747-5p, hsa-miR-4748, hsa-miR-4749-5p, hsa-miR-4749-3p, hsa-miR-4750-5p, hsa-miR-4751, hsa-miR-4752, hsa-miR-4753-5p, hsa-miR-371b-5p, hsa-miR-371b-3p, hsa-miR-4754, hsa-miR-4755-3p, hsa-miR-4756-5p, hsa-miR-4758-5p, hsa-miR-4758-3p, hsa-miR-4759, hsa-miR-4763-5p, hsa-miR-4763-3p, hsa-miR-4764-5p, hsa-miR-4766-5p, hsa-miR-4769-5p, hsa-miR-4769-3p, hsa-miR-4776-5p, hsa-miR-4778-5p, hsa-miR-4779, hsa-miR-4780, hsa-miR-4436b-5p, hsa-miR-4781-5p, hsa-miR-4783-3p, hsa-miR-2467-3p, hsa-miR-4787-5p, hsa-miR-4787-3p, hsa-miR-4788, hsa-miR-4793-3p, hsa-miR-4800-5p, hsa-miR-4804-3p, hsa-miR-642a-3p, hsa-miR-4433a-5p, hsa-miR-4482-3p, hsa-miR-4999-5p, hsa-miR-5001-5p, hsa-miR-5002-3p, hsa-miR-5003-3p, hsa-miR-5006-5p, hsa-miR-5008-5p, hsa-miR-5009-3p, hsa-miR-5010-5p, hsa-miR-5010-3p, hsa-miR-5088-5p, hsa-miR-5089-5p, hsa-miR-5090, hsa-miR-5188, hsa-miR-5189-5p, hsa-miR-5193, hsa-miR-5195-5p, hsa-miR-5195-3p, hsa-miR-5196-5p, hsa-miR-4524b-5p, hsa-miR-5100, hsa-miR-5572, hsa-miR-664b-5p, hsa-miR-664b-3p, hsa-miR-5581-5p, hsa-miR-5581-3p, hsa-miR-5584-5p, hsa-miR-548au-3p, hsa-miR-5588-5p, hsa-miR-5589-5p, hsa-miR-5684, hsa-miR-5690, hsa-miR-5698, hsa-miR-5703, hsa-miR-5705, hsa-miR-197-5p, hsa-miR-204-3p, hsa-miR-211-3p, hsa-miR-301a-5p, hsa-miR-345-3p, hsa-miR-652-5p, hsa-miR-1185-2-3p, hsa-miR-873-3p, hsa-miR-1304-3p, hsa-miR-1247-3p, hsa-miR-1306-5p, hsa-miR-3184-3p, hsa-miR-3191-5p, hsa-miR-642b-5p, hsa-miR-365b-5p, hsa-miR-1185-1-3p, hsa-miR-98-3p, hsa-miR-381-5p, hsa-miR-503-3p, hsa-miR-937-5p, hsa-miR-939-3p, hsa-miR-1227-5p, hsa-miR-1229-5p, hsa-miR-1233-5p, hsa-miR-1236-5p, hsa-miR-1237-5p, hsa-miR-1238-5p, hsa-miR-1292-3p, hsa-miR-3617-3p, hsa-miR-3620-5p, hsa-miR-4632-5p, hsa-miR-4750-3p, hsa-miR-5739, hsa-miR-5787, hsa-miR-6068, hsa-miR-6069, hsa-miR-6071, hsa-miR-6072, hsa-miR-6074, hsa-miR-6075, hsa-miR-6076, hsa-miR-6077, hsa-miR-6085, hsa-miR-6086, hsa-miR-6088, hsa-miR-6089, hsa-miR-6090, hsa-miR-6124, hsa-miR-6125, hsa-miR-6126, hsa-miR-6127, hsa-miR-378j, hsa-miR-6130, hsa-miR-6131, hsa-miR-6132, hsa-miR-6133, hsa-miR-6165, hsa-miR-6500-3p, hsa-miR-6501-3p, hsa-miR-6503-5p, hsa-miR-6506-5p, hsa-miR-6507-3p, hsa-miR-6508-5p, hsa-miR-6509-3p, hsa-miR-6510-5p, hsa-miR-6511a-5p, hsa-miR-6511a-3p, hsa-miR-6513-5p, hsa-miR-6514-3p, hsa-miR-6515-5p, hsa-miR-6515-3p, hsa-miR-6715b-3p, hsa-miR-6716-5p, hsa-miR-6716-3p, hsa-miR-6717-5p, hsa-miR-6511b-5p, hsa-miR-6511b-3p, hsa-miR-6719-3p, hsa-miR-6721-5p, hsa-miR-6722-5p, hsa-miR-6722-3p, hsa-miR-6724-5p, hsa-miR-208a-5p, hsa-miR-210-5p, hsa-miR-128-1-5p, hsa-miR-370-5p, hsa-miR-328-5p, hsa-miR-410-5p, hsa-miR-181d-3p, hsa-miR-520f-5p, hsa-miR-504-3p, hsa-miR-487b-5p, hsa-miR-585-5p, hsa-miR-597-3p, hsa-miR-598-5p, hsa-miR-605-3p, hsa-miR-619-5p, hsa-miR-1296-3p, hsa-miR-874-5p, hsa-miR-887-5p, hsa-miR-1250-3p, hsa-miR-1908-3p, hsa-miR-3151-3p, hsa-miR-3192-3p, hsa-miR-1343-5p, hsa-miR-5088-3p, hsa-miR-5189-3p, hsa-miR-5699-5p, hsa-miR-6726-5p, hsa-miR-6726-3p, hsa-miR-6727-5p, hsa-miR-6727-3p, hsa-miR-6728-5p, hsa-miR-6728-3p, hsa-miR-6729-5p, hsa-miR-6729-3p, hsa-miR-6730-5p, hsa-miR-6730-3p, hsa-miR-6731-5p, hsa-miR-6731-3p, hsa-miR-6732-5p, hsa-miR-6732-3p, hsa-miR-6734-5p, hsa-miR-6734-3p, hsa-miR-6735-5p, hsa-miR-6735-3p, hsa-miR-6736-5p, hsa-miR-6736-3p, hsa-miR-6737-5p, hsa-miR-6737-3p, hsa-miR-6738-5p, hsa-miR-6738-3p, hsa-miR-6740-5p, hsa-miR-6740-3p, hsa-miR-6741-5p, hsa-miR-6741-3p, hsa-miR-6742-5p, hsa-miR-6742-3p, hsa-miR-6743-5p, hsa-miR-6743-3p, hsa-miR-6744-5p, hsa-miR-6744-3p, hsa-miR-6745, hsa-miR-6746-5p, hsa-miR-6747-5p, hsa-miR-6747-3p, hsa-miR-6748-5p, hsa-miR-6748-3p, hsa-miR-6749-5p, hsa-miR-6749-3p, hsa-miR-6750-5p, hsa-miR-6750-3p, hsa-miR-6751-5p, hsa-miR-6752-5p, hsa-miR-6752-3p, hsa-miR-6753-5p, hsa-miR-6753-3p, hsa-miR-6754-5p, hsa-miR-6754-3p, hsa-miR-6755-5p, hsa-miR-6756-5p, hsa-miR-6756-3p, hsa-miR-6757-5p, hsa-miR-6757-3p, hsa-miR-6758-5p, hsa-miR-6759-5p, hsa-miR-6759-3p, hsa-miR-6760-5p, hsa-miR-6760-3p, hsa-miR-6761-3p, hsa-miR-6762-5p, hsa-miR-6762-3p, hsa-miR-6763-5p, hsa-miR-6763-3p, hsa-miR-6765-5p, hsa-miR-6766-5p, hsa-miR-6766-3p, hsa-miR-6767-5p, hsa-miR-6768-5p, hsa-miR-6769a-5p, hsa-miR-6769a-3p, hsa-miR-6770-5p, hsa-miR-6771-5p, hsa-miR-6771-3p, hsa-miR-6772-5p, hsa-miR-6772-3p, hsa-miR-6773-3p, hsa-miR-6774-5p, hsa-miR-6775-5p, hsa-miR-6775-3p, hsa-miR-6776-5p, hsa-miR-6776-3p, hsa-miR-6777-5p, hsa-miR-6777-3p, hsa-miR-6778-5p, hsa-miR-6778-3p, hsa-miR-6779-5p, hsa-miR-6779-3p, hsa-miR-6780a-5p, hsa-miR-6780a-3p, hsa-miR-6781-5p, hsa-miR-6782-5p, hsa-miR-6782-3p, hsa-miR-6783-5p, hsa-miR-6783-3p, hsa-miR-6784-5p, hsa-miR-6784-3p, hsa-miR-6785-3p, hsa-miR-6786-5p, hsa-miR-6786-3p, hsa-miR-6787-5p, hsa-miR-6787-3p, hsa-miR-6788-5p, hsa-miR-6788-3p, hsa-miR-6789-5p, hsa-miR-6789-3p, hsa-miR-6790-5p, hsa-miR-6790-3p, hsa-miR-6791-5p, hsa-miR-6792-5p, hsa-miR-6792-3p, hsa-miR-6793-5p, hsa-miR-6793-3p, hsa-miR-6794-5p, hsa-miR-6794-3p, hsa-miR-6795-5p, hsa-miR-6795-3p, hsa-miR-6796-5p, hsa-miR-6796-3p, hsa-miR-6797-5p, hsa-miR-6797-3p, hsa-miR-6798-5p, hsa-miR-6798-3p, hsa-miR-6799-5p, hsa-miR-6799-3p, hsa-miR-6800-5p, hsa-miR-6800-3p, hsa-miR-6801-3p, hsa-miR-6802-5p, hsa-miR-6802-3p, hsa-miR-6803-5p, hsa-miR-6803-3p, hsa-miR-6804-3p, hsa-miR-6805-5p, hsa-miR-6805-3p, hsa-miR-6806-5p, hsa-miR-6807-5p, hsa-miR-6808-5p, hsa-miR-6808-3p, hsa-miR-6809-5p, hsa-miR-6809-3p, hsa-miR-6810-5p, hsa-miR-6810-3p, hsa-miR-6812-5p, hsa-miR-6812-3p, hsa-miR-6813-5p, hsa-miR-6813-3p, hsa-miR-6814-3p, hsa-miR-6815-5p, hsa-miR-6816-5p, hsa-miR-6816-3p, hsa-miR-6818-5p, hsa-miR-6819-5p, hsa-miR-6819-3p, hsa-miR-6820-5p, hsa-miR-6820-3p, hsa-miR-6821-5p, hsa-miR-6822-5p, hsa-miR-6823-5p, hsa-miR-6823-3p, hsa-miR-6824-5p, hsa-miR-6824-3p, hsa-miR-6825-5p, hsa-miR-6825-3p, hsa-miR-6826-3p, hsa-miR-6827-5p, hsa-miR-6827-3p, hsa-miR-6828-5p, hsa-miR-6829-5p, hsa-miR-6829-3p, hsa-miR-6830-5p, hsa-miR-6830-3p, hsa-miR-6831-5p, hsa-miR-6831-3p, hsa-miR-6832-5p, hsa-miR-6832-3p, hsa-miR-6833-5p, hsa-miR-6833-3p, hsa-miR-6834-5p, hsa-miR-6834-3p, hsa-miR-6780b-5p, hsa-miR-6780b-3p, hsa-miR-6836-3p, hsa-miR-6838-5p, hsa-miR-6839-5p, hsa-miR-6840-5p, hsa-miR-6840-3p, hsa-miR-6841-3p, hsa-miR-6842-5p, hsa-miR-6845-5p, hsa-miR-6845-3p, hsa-miR-6846-5p, hsa-miR-6846-3p, hsa-miR-6847-3p, hsa-miR-6848-5p, hsa-miR-6848-3p, hsa-miR-6849-5p, hsa-miR-6850-5p, hsa-miR-6851-5p, hsa-miR-6851-3p, hsa-miR-6855-5p, hsa-miR-6855-3p, hsa-miR-6856-5p, hsa-miR-6857-5p, hsa-miR-6857-3p, hsa-miR-6858-5p, hsa-miR-6858-3p, hsa-miR-6859-5p, hsa-miR-6859-3p, hsa-miR-6769b-5p, hsa-miR-6769b-3p, hsa-miR-6860, hsa-miR-6861-5p, hsa-miR-6861-3p, hsa-miR-6862-5p, hsa-miR-6862-3p, hsa-miR-6865-5p, hsa-miR-6865-3p, hsa-miR-6867-5p, hsa-miR-6867-3p, hsa-miR-6868-3p, hsa-miR-6869-5p, hsa-miR-6870-5p, hsa-miR-6870-3p, hsa-miR-6871-5p, hsa-miR-6872-5p, hsa-miR-6872-3p, hsa-miR-6873-5p, hsa-miR-6874-5p, hsa-miR-6875-5p, hsa-miR-6875-3p, hsa-miR-6876-5p, hsa-miR-6877-5p, hsa-miR-6877-3p, hsa-miR-6878-5p, hsa-miR-6878-3p, hsa-miR-6879-5p, hsa-miR-6879-3p, hsa-miR-6880-5p, hsa-miR-6880-3p, hsa-miR-6881-5p, hsa-miR-6881-3p, hsa-miR-6882-5p, hsa-miR-6882-3p, hsa-miR-6883-5p, hsa-miR-6883-3p, hsa-miR-6884-3p, hsa-miR-6885-5p, hsa-miR-6885-3p, hsa-miR-6886-3p, hsa-miR-6887-5p, hsa-miR-6887-3p, hsa-miR-6889-5p, hsa-miR-6889-3p, hsa-miR-6890-5p, hsa-miR-6890-3p, hsa-miR-6891-5p, hsa-miR-6891-3p, hsa-miR-6892-5p, hsa-miR-6892-3p, hsa-miR-6893-5p, hsa-miR-6893-3p, hsa-miR-6894-5p, hsa-miR-6894-3p, hsa-miR-7106-5p, hsa-miR-7106-3p, hsa-miR-7107-5p, hsa-miR-7108-5p, hsa-miR-7108-3p, hsa-miR-7109-5p, hsa-miR-7109-3p, hsa-miR-7110-5p, hsa-miR-7110-3p, hsa-miR-7111-5p, hsa-miR-7111-3p, hsa-miR-7112-5p, hsa-miR-7113-5p, hsa-miR-7113-3p, hsa-miR-7114-5p, hsa-miR-7114-3p, hsa-miR-7150, hsa-miR-7151-3p, hsa-miR-7152-5p, hsa-miR-7152-3p, hsa-miR-7155-5p, hsa-miR-7157-3p, hsa-miR-7159-5p, hsa-miR-7160-5p, hsa-miR-7162-5p, hsa-miR-7515, hsa-miR-7702, hsa-miR-7704, hsa-miR-7843-5p, hsa-miR-4433b-5p, hsa-miR-4433b-3p, hsa-miR-1273h-5p, hsa-miR-6516-5p, hsa-miR-7845-5p, hsa-miR-7846-3p, hsa-miR-7847-3p, hsa-miR-7851-3p, hsa-miR-7855-5p, hsa-miR-7856-5p, hsa-miR-8052, hsa-miR-8054, hsa-miR-8059, hsa-miR-8060, hsa-miR-8063, hsa-miR-8064, hsa-miR-8069, hsa-miR-8071, hsa-miR-8072, hsa-miR-8073, hsa-miR-8074, hsa-miR-8075, hsa-miR-8077, hsa-miR-8080, hsa-miR-8085, hsa-miR-8087, hsa-miR-8089, hsa-miR-128-2-5p, hsa-miR-7977, hsa-miR-7978, hsa-miR-1249-5p, hsa-miR-4485-5p, hsa-miR-8485, hsa-miR-375-5p, hsa-miR-498-3p, hsa-miR-526a-3p, hsa-miR-9718, hsa-miR-9898, hsa-miR-1843, hsa-miR-10392-5p, hsa-miR-10392-3p, hsa-miR-10394-3p, hsa-miR-10395-5p, hsa-miR-10395-3p, hsa-miR-10396a-5p, hsa-miR-10396a-3p, hsa-miR-10398-5p, hsa-miR-10398-3p, hsa-miR-10400-5p, hsa-miR-10400-3p, hsa-miR-10401-5p, hsa-miR-10401-3p, hsa-miR-10396b-5p, hsa-miR-10522-5p, hsa-miR-10526-3p, hsa-miR-10527-5p, hsa-miR-11181-5p, hsa-miR-11181-3p, hsa-miR-11399, hsa-miR-11400, hsa-miR-11401, hsa-miR-3059-5p, hsa-miR-3059-3p, hsa-miR-3085-5p, hsa-miR-3085-3p, hsa-miR-6529-5p, hsa-miR-12114, hsa-miR-12115, hsa-miR-12116, hsa-miR-12118, hsa-miR-12119, hsa-miR-12120, hsa-miR-12121, hsa-miR-12122, hsa-miR-12124, hsa-miR-12126, hsa-miR-12131, and hsa-miR-12136. In the present disclosure, the extract of urine may be an extract of the urine of a stage-I or -II kidney cancer patient. Only one of these microRNAs can be used to predict whether a subject from which the urine is derived is a kidney cancer patient (particularly stage-I or -II), and 5 or more types, 10 or more types, 15 or more types, or 20 or more types of these microRNAs can be used in combination to predict whether a subject from which the urine is derived is a kidney cancer patient (particularly stage-I or -II). Among these microRNAs, a microRNA that may be highly expressed in a kidney cancer patient (particularly stage-I or -II) can be preferably used for prediction.

The present disclosure provides an extract of urine containing at least one type, two types, three types, four types, five types, six types, seven types, eight types, nine types, or ten types of or all microRNAs selected from the group consisting of hsa-miR-17-3p, hsa-miR-29a-3p, hsa-miR-30a-5p, hsa-miR-31-5p, hsa-miR-29b-3p, hsa-miR-196a-5p, hsa-miR-197-3p, hsa-miR-198, hsa-miR-30d-5p, hsa-miR-10a-5p, hsa-miR-187-3p, hsa-miR-199b-5p, hsa-miR-214-3p, hsa-miR-223-3p, hsa-miR-133a-3p, hsa-miR-125a-5p, hsa-miR-134-5p, hsa-miR-184, hsa-miR-185-5p, hsa-miR-206, hsa-miR-200c-3p, hsa-miR-106b-5p, hsa-miR-29c-3p, hsa-miR-302a-3p, hsa-miR-299-3p, hsa-miR-296-5p, hsa-miR-130b-3p, hsa-miR-30e-5p, hsa-miR-361-5p, hsa-miR-362-5p, hsa-miR-365a-3p, hsa-miR-365b-3p, hsa-miR-302c-5p, hsa-miR-370-3p, hsa-miR-373-5p, hsa-miR-373-3p, hsa-miR-378a-3p, hsa-miR-379-5p, hsa-miR-380-3p, hsa-miR-328-3p, hsa-miR-342-3p, hsa-miR-326, hsa-miR-133b, hsa-miR-325, hsa-miR-346, hsa-miR-20b-5p, hsa-miR-448, hsa-miR-429, hsa-miR-449a, hsa-miR-191-3p, hsa-miR-200a-5p, hsa-miR-409-3p, hsa-miR-483-3p, hsa-miR-484, hsa-miR-485-3p, hsa-miR-486-5p, hsa-miR-491-5p, hsa-miR-202-5p, hsa-miR-202-3p, hsa-miR-492, hsa-miR-494-3p, hsa-miR-512-5p, hsa-miR-498-5p, hsa-miR-520e-3p, hsa-miR-519c-5p, hsa-miR-523-5p, hsa-miR-518e-5p, hsa-miR-522-5p, hsa-miR-519a-5p, hsa-miR-519b-5p, hsa-miR-526b-5p, hsa-miR-518f-3p, hsa-miR-520b-3p, hsa-miR-518b, hsa-miR-520c-3p, hsa-miR-518c-5p, hsa-miR-520d-3p, hsa-miR-516b~5p, hsa-miR-516b-3p, hsa-miR-516a-3p, hsa-miR-519a-3p, hsa-miR-499a~5p, hsa-miR-502-5p, hsa-miR-513a-5p, hsa-miR-532-5p, hsa-miR-556-5p, hsa-miR-557, hsa-miR-563, hsa-miR-564, hsa-miR-571, hsa-miR-572, hsa-miR-573, hsa-miR-574-3p, hsa-miR-575, hsa-miR-578, hsa-miR-579-3p, hsa-miR-583, hsa-miR-585-3p, hsa-miR-588, hsa-miR-550a-3p, hsa-miR-590-5p, hsa-miR-608, hsa-miR-610, hsa-miR-612, hsa-miR-615-3p, hsa-miR-617, hsa-miR-619-3p, hsa-miR-624-5p, hsa-miR-625-5p, hsa-miR-628-3p, hsa-miR-634, hsa-miR-637, hsa-miR-638, hsa-miR-639, hsa-miR-641, hsa-miR-650, hsa-miR-663a, hsa-miR-449b-5p, hsa-miR-654-5p, hsa-miR-657, hsa-miR-658, hsa-miR-659-3p, hsa-miR-542-5p, hsa-miR-671-5p, hsa-miR-668-3p, hsa-miR-767-3p, hsa-miR-769-5p, hsa-miR-769-3p, hsa-miR-766-3p, hsa-miR-765, hsa-miR-675-5p, hsa-miR-297, hsa-let-7b-3p, hsa-let-7d-3p, hsa-let-7f-1-3p, hsa-miR-92a-2-5p, hsa-miR-29b-2-5p, hsa-miR-30c-2-3p, hsa-miR-139-3p, hsa-miR-10a-3p, hsa-miR-181c-3p, hsa-miR-183-3p, hsa-miR-187-5p, hsa-miR-15b-3p, hsa-miR-124-5p, hsa-miR-125b-l-3p, hsa-miR-130a-5p, hsa-miR-135a-3p, hsa-miR-125a-3p, hsa-miR-127-5p, hsa-miR-149-3p, hsa-miR-150-3p, hsa-miR-185-3p, hsa-miR-186-3p, hsa-miR-193a-5p, hsa-miR-194-3p, hsa-miR-296-3p, hsa-miR-361-3p, hsa-miR-302d-5p, hsa-miR-371a-5p, hsa-miR-374a-3p, hsa-miR-377-5p, hsa-miR-342-5p, hsa-miR-423-5p, hsa-miR-424-3p, hsa-miR-18b-3p, hsa-miR-20b-3p, hsa-miR-431-3p, hsa-miR-483-5p, hsa-miR-486-3p, hsa-miR-490-5p, hsa-miR-193b-5p, hsa-miR-505-5p, hsa-miR-513a-3p, hsa-miR-92b-5p, hsa-miR-551b-5p, hsa-miR-574-5p, hsa-miR-550a-5p, hsa-miR-615-5p, hsa-miR-616-3p, hsa-miR-625-3p, hsa-miR-629-5p, hsa-miR-298, hsa-miR-874-3p, hsa-miR-890, hsa-miR-891b, hsa-miR-876-3p, hsa-miR-744-5p, hsa-miR-885-5p, hsa-miR-885-3p, hsa-miR-877-5p, hsa-miR-877-3p, hsa-miR-887-3p, hsa-miR-665, hsa-miR-760, hsa-miR-301b-3p, hsa-miR-920, hsa-miR-509-3-5p, hsa-miR-933, hsa-miR-936, hsa-miR-939-5p, hsa-miR-940, hsa-miR-1224-5p, hsa-miR-1224-3p, hsa-miR-1225-5p, hsa-miR-1225-3p, hsa-miR-1227-3p, hsa-miR-1228-5p, hsa-miR-1228-3p, hsa-miR-1229-3p, hsa-miR-1231, hsa-miR-1233-3p, hsa-miR-1234-3p, hsa-miR-1236-3p, hsa-miR-1237-3p, hsa-miR-1238-3p, hsa-miR-320b, hsa-miR-320c, hsa-miR-1323, hsa-miR-1301-3p, hsa-miR-1298-5p, hsa-miR-1181, hsa-miR-1182, hsa-miR-1184, hsa-miR-1202, hsa-miR-1203, hsa-miR-663b, hsa-miR-1207-5p, hsa-miR-1207-3p, hsa-miR-1285-3p, hsa-miR-1289, hsa-miR-1294, hsa-miR-1299, hsa-miR-1303, hsa-miR-1304-5p, hsa-miR-1245a, hsa-miR-1246, hsa-miR-1249-3p, hsa-miR-1257, hsa-miR-1260a, hsa-miR-1263, hsa-miR-1267, hsa-miR-1268a, hsa-miR-1270, hsa-miR-1275, hsa-miR-1278, hsa-miR-1281, hsa-miR-1292-5p, hsa-miR-1255b-5p, hsa-miR-1307-3p, hsa-miR-320d, hsa-miR-1825, hsa-miR-675-3p, hsa-miR-1469, hsa-miR-1470, hsa-miR-1471, hsa-miR-1539, hsa-miR-1908-5p, hsa-miR-1909-5p, hsa-miR-1909-3p, hsa-miR-1910-5p, hsa-miR-1912-3p, hsa-miR-1913, hsa-miR-1914-3p, hsa-miR-1915-3p, hsa-miR-365a-5p, hsa-miR-449b-3p, hsa-miR-2113, hsa-miR-1976, hsa-miR-2110, hsa-miR-151b, hsa-miR-762, hsa-miR-670-5p, hsa-miR-764, hsa-miR-2116-3p, hsa-miR-548q, hsa-miR-2276-3p, hsa-miR-2278, hsa-miR-711, hsa-miR-718, hsa-miR-2681-3p, hsa-miR-2861, hsa-miR-3116, hsa-miR-3119, hsa-miR-3120-3p, hsa-miR-3122, hsa-miR-3124-5p, hsa-miR-3126-5p, hsa-miR-3128, hsa-miR-3130-5p, hsa-miR-3131, hsa-miR-3132, hsa-miR-3133, hsa-miR-466, hsa-miR-3136-5p, hsa-miR-3138, hsa-miR-3141, hsa-miR-3144-5p, hsa-miR-1273c, hsa-miR-3147, hsa-miR-3150a-3p, hsa-miR-3151-5p, hsa-miR-3153, hsa-miR-3154, hsa-miR-3156-5p, hsa-miR-3157-5p, hsa-miR-3160-3p, hsa-miR-3161, hsa-miR-3162-5p, hsa-miR-3163, hsa-miR-3165, hsa-miR-1260b, hsa-miR-3169, hsa-miR-3173-3p, hsa-miR-1193, hsa-miR-3175, hsa-miR-3177-3p, hsa-miR-3178, hsa-miR-3179, hsa-miR-3180-5p, hsa-miR-3180-3p, hsa-miR-3184-5p, hsa-miR-3185, hsa-miR-3186-3p, hsa-miR-3187-3p, hsa-miR-3188, hsa-miR-3189-3p, hsa-miR-3191-3p, hsa-miR-3194-5p, hsa-miR-3195, hsa-miR-3196, hsa-miR-3197, hsa-miR-3198, hsa-miR-3199, hsa-miR-514b-5p, hsa-miR-3202, hsa-miR-4297, hsa-miR-4293, hsa-miR-4294, hsa-miR-4299, hsa-miR-4298, hsa-miR-4300, hsa-miR-4305, hsa-miR-4306, hsa-miR-4307, hsa-miR-4312, hsa-miR-4313, hsa-miR-4316, hsa-miR-4314, hsa-miR-4322, hsa-miR-4321, hsa-miR-4323, hsa-miR-4256, hsa-miR-4257, hsa-miR-4 258, hsa-miR-4259, hsa-miR-4260, hsa-miR-4253, hsa-miR-4251, hsa-miR-4325, hsa-miR-4326, hsa-miR-4327, hsa-miR-4265, hsa-miR-4266, hsa-miR-2355-5p, hsa-miR-4268, hsa-miR-4269, hsa-miR-4270, hsa-miR-4271, hsa-miR-4276, hsa-miR-4274, hsa-miR-4281, hsa-miR-4279, hsa-miR-4278, hsa-miR-4280, hsa-miR-4285, hsa-miR-4283, hsa-miR-4284, hsa-miR-4286, hsa-miR-4287, hsa-miR-4292, hsa-miR-4290, hsa-miR-4329, hsa-miR-3200-5p, hsa-miR-3605-3p, hsa-miR-3610, hsa-miR-3612, hsa-miR-3614-5p, hsa-miR-3616-3p, hsa-miR-3620-3p, hsa-miR-3621, hsa-miR-3622a-5p, hsa-miR-3622a-3p, hsa-miR-3622b-5p, hsa-miR-3648, hsa-miR-3649, hsa-miR-3652, hsa-miR-3654, hsa-miR-3660, hsa-miR-3663-5p, hsa-miR-3663-3p, hsa-miR-3665, hsa-miR-3667-5p, hsa-miR-3667-3p, hsa-miR-3675-5p, hsa-miR-3675-3p, hsa-miR-3677-3p, hsa-miR-3679-5p, hsa-miR-3679-3p, hsa-miR-3689a-3p, hsa-miR-3691-5p, hsa-miR-3714, hsa-miR-3180, hsa-miR-3907, hsa-miR-3689b-3p, hsa-miR-3689c, hsa-miR-3909, hsa-miR-3911, hsa-miR-3917, hsa-miR-3919, hsa-miR-3925-5p, hsa-miR-3926, hsa-miR-3928-3p, hsa-miR-3935, hsa-miR-3936, hsa-miR-3937, hsa-miR-3941, hsa-miR-3943, hsa-miR-3944-3p, hsa-miR-3945, hsa-miR-642b-3p, hsa-miR-550b-3p, hsa-miR-1268b, hsa-miR-548ab, hsa-miR-4418, hsa-miR-4421, hsa-miR-4428, hsa-miR-4429, hsa-miR-4430, hsa-miR-4433a-3p, hsa-miR-4434, hsa-miR-4439, hsa-miR-4440, hsa-miR-4441, hsa-miR-4443, hsa-miR-4444, hsa-miR-4446-3p, hsa-miR-4447, hsa-miR-4448, hsa-miR-4449, hsa-miR-4451, hsa-miR-4455, hsa-miR-4456, hsa-miR-4458, hsa-miR-4460, hsa-miR-378h, hsa-miR-3135b, hsa-miR-4462, hsa-miR-4463, hsa-miR-4466, hsa-miR-4467, hsa-miR-4468, hsa-miR-4470, hsa-miR-4472, hsa-miR-4474-3p, hsa-miR-4475, hsa-miR-4476, hsa-miR-4477b, hsa-miR-4478, hsa-miR-3689d, hsa-miR-4481, hsa-miR-4483, hsa-miR-4484, hsa-miR-4486, hsa-miR-4487, hsa-miR-4488, hsa-miR-4489, hsa-miR-4492, hsa-miR-4496, hsa-miR-4497, hsa-miR-4498, hsa-miR-4499, hsa-miR-4505, hsa-miR-2392, hsa-miR-4507, hsa-miR-4508, hsa-miR-4510, hsa-miR-4513, hsa-miR-4516, hsa-miR-4518, hsa-miR-4519, hsa-miR-4521, hsa-miR-1269b, hsa-miR-4522, hsa-miR-4523, hsa-miR-4525, hsa-miR-4526, hsa-miR-4530, hsa-miR-4531, hsa-miR-4533, hsa-miR-4534, hsa-miR-4535, hsa-miR-1587, hsa-miR-4538, hsa-miR-4539, hsa-miR-3127-3p, hsa-miR-3156-3p, hsa-miR-3158-5p, hsa-miR-3162-3p, hsa-miR-3173-5p, hsa-miR-3187-5p, hsa-miR-3189-5p, hsa-miR-3619-3p, hsa-miR-3150b~5p, hsa-miR-3940-5p, hsa-miR-3960, hsa-miR-3972, hsa-miR-3978, hsa-miR-4634, hsa-miR-4638-5p, hsa-miR-4639-3p, hsa-miR-4640-5p, hsa-miR-4640-3p, hsa-miR-4642, hsa-miR-4644, hsa-miR-4646-5p, hsa-miR-4646-3p, hsa-miR-4648, hsa-miR-4649-5p, hsa-miR-4649-3p, hsa-miR-4650-3p, hsa-miR-4651, hsa-miR-4652-5p, hsa-miR-4653-3p, hsa-miR-4654, hsa-miR-4655-5p, hsa-miR-4656, hsa-miR-4659b-3p, hsa-miR-4663, hsa-miR-4664-5p, hsa-miR-4664-3p, hsa-miR-4665-5p, hsa-miR-4665-3p, hsa-miR-4667-5p, hsa-miR-4667-3p, hsa-miR-4669, hsa-miR-4670-3p, hsa-miR-4672, hsa-miR-4673, hsa-miR-4674, hsa-miR-4675, hsa-miR-4677-5p, hsa-miR-4684-3p, hsa-miR-4685-5p, hsa-miR-4685-3p, hsa-miR-4687-5p, hsa-miR-4687-3p, hsa-miR-1343-3p, hsa-miR-4688, hsa-miR-4689, hsa-miR-4690-5p, hsa-miR-4691-5p, hsa-miR-4695-5p, hsa-miR-4695-3p, hsa-miR-4697-5p, hsa-miR-4697-3p, hsa-miR-4698, hsa-miR-4700-5p, hsa-miR-4700-3p, hsa-miR-4701-5p, hsa-miR-4701-3p, hsa-miR-4706, hsa-miR-4707-5p, hsa-miR-4707-3p, hsa-miR-4708-3p, hsa-miR-4709-3p, hsa-miR-4710, hsa-miR-4713-5p, hsa-miR-4714-5p, hsa-miR-4715-5p, hsa-miR-4716-5p, hsa-miR-4716-3p, hsa-miR-4717-3p, hsa-miR-4720-5p, hsa-miR-4721, hsa-miR-4722-5p, hsa-miR-4722-3p, hsa-miR-4723-5p, hsa-miR-4723-3p, hsa-miR-4725-3p, hsa-miR-4726-5p, hsa-miR-4727-3p, hsa-miR-4728-5p, hsa-miR-4728-3p, hsa-miR-4729, hsa-miR-4730, hsa-miR-4731-5p, hsa-miR-4731-3p, hsa-miR-4732-5p, hsa-miR-4732-3p, hsa-miR-4734, hsa-miR-4736, hsa-miR-3064-5p, hsa-miR-3064-3p, hsa-miR-4738-3p, hsa-miR-4739, hsa-miR-4741, hsa-miR-4742-3p, hsa-miR-4743-5p, hsa-miR-4745-5p, hsa-miR-4746-3p, hsa-miR-4747-5p, hsa-miR-4748, hsa-miR-4749-5p, hsa-miR-4749-3p, hsa-miR-4750-5p, hsa-miR-4751, hsa-miR-4752, hsa-miR-4753-5p, hsa-miR-371b-5p, hsa-miR-371b-3p, hsa-miR-4754, hsa-miR-4755-3p, hsa-miR-4756-5p, hsa-miR-4758-5p, hsa-miR-4758-3p, hsa-miR-4759, hsa-miR-4763-5p, hsa-miR-4763-3p, hsa-miR-4764-5p, hsa-miR-4766-5p, hsa-miR-4769-5p, hsa-miR-4769-3p, hsa-miR-4776-5p, hsa-miR-4778-5p, hsa-miR-4779, hsa-miR-4780, hsa-miR-4436b-5p, hsa-miR-4781-5p, hsa-miR-4783-3p, hsa-miR-2467-3p, hsa-miR-4787-5p, hsa-miR-4787-3p, hsa-miR-4788, hsa-miR-4789-3p, hsa-miR-4790-3p, hsa-miR-4793-3p, hsa-miR-4795-3p, hsa-miR-4796-3p, hsa-miR-4798-3p, hsa-miR-4800-5p, hsa-miR-4804-5p, hsa-miR-4804-3p, hsa-miR-642a-3p, hsa-miR-550a-3-5p, hsa-miR-4433a-5p, hsa-miR-4482-3p, hsa-miR-4999-5p, hsa-miR-5001-5p, hsa-miR-5002-3p, hsa-miR-5003-3p, hsa-miR-5006-5p, hsa-miR-5008-5p, hsa-miR-5009-3p, hsa-miR-5010-5p, hsa-miR-5010-3p, hsa-miR-5088-5p, hsa-miR-5089-5p, hsa-miR-5090, hsa-miR-5093, hsa-miR-5188, hsa-miR-5189-5p, hsa-miR-5193, hsa-miR-5195-5p, hsa-miR-5195-3p, hsa-miR-5196-5p, hsa-miR-4524b-5p, hsa-miR-5100, hsa-miR-5572, hsa-miR-664b-5p, hsa-miR-664b-3p, hsa-miR-5581-5p, hsa-miR-5584-5p, hsa-miR-548au-3p, hsa-miR-5588-5p, hsa-miR-5589-5p, hsa-miR-5682, hsa-miR-5684, hsa-miR-5690, hsa-miR-5698, hsa-miR-5703, hsa-miR-5705, hsa-miR-197-5p, hsa-miR-204-3p, hsa-miR-211-3p, hsa-miR-301a-5p, hsa-miR-345-3p, hsa-miR-652-5p, hsa-miR-1271-3p, hsa-miR-1185-2-3p, hsa-miR-766-5p, hsa-miR-1304-3p, hsa-miR-1247-3p, hsa-miR-548g-5p, hsa-miR-548x-5p, hsa-miR-548aj-5p, hsa-miR-1306-5p, hsa-miR-3184-3p, hsa-miR-3191-5p, hsa-miR-642b-5p, hsa-miR-365b-5p, hsa-miR-1185-1-3p, hsa-miR-98-3p, hsa-miR-376c-5p, hsa-miR-381-5p, hsa-miR-503-3p, hsa-miR-937-5p, hsa-miR-939-3p, hsa-miR-1227-5p, hsa-miR-1229-5p, hsa-miR-1233-5p, hsa-miR-1236-5p, hsa-miR-1237-5p, hsa-miR-1238-5p, hsa-miR-3617-3p, hsa-miR-3620-5p, hsa-miR-3934-3p, hsa-miR-4632-5p, hsa-miR-4750-3p, hsa-miR-5739, hsa-miR-5787, hsa-miR-6068, hsa-miR-6069, hsa-miR-6071, hsa-miR-6072, hsa-miR-6075, hsa-miR-6076, hsa-miR-6077, hsa-miR-6081, hsa-miR-6085, hsa-miR-6086, hsa-miR-6088, hsa-miR-6089, hsa-miR-6090, hsa-miR-6124, hsa-miR-6125, hsa-miR-6126, hsa-miR-6127, hsa-miR-378j, hsa-miR-6131, hsa-miR-6132, hsa-miR-6133, hsa-miR-6165, hsa-miR-6500-3p, hsa-miR-548az-3p, hsa-miR-6501-3p, hsa-miR-6503-5p, hsa-miR-6506-5p, hsa-miR-6506-3p, hsa-miR-6508-5p, hsa-miR-6508-3p, hsa-miR-6509-3p, hsa-miR-6510-5p, hsa-miR-6511a-5p, hsa-miR-6511a-3p, hsa-miR-6513-5p, hsa-miR-6514-3p, hsa-miR-6515-5p, hsa-miR-6515-3p, hsa-miR-6715b-3p, hsa-miR-6716-5p, hsa-miR-6716-3p, hsa-miR-6717-5p, hsa-miR-6511b-Sp, hsa-miR-6511b-3p, hsa-miR-6719-3p, hsa-miR-6721-5p, hsa-miR-6722-5p, hsa-miR-6722-3p, hsa-miR-6724-5p, hsa-miR-208a-5p, hsa-miR-210-5p, hsa-miR-128-1-5p, hsa-miR-133a-5p, hsa-miR-152-5p, hsa-miR-370-5p, hsa-miR-328-5p, hsa-miR-410-5p, hsa-miR-181d-3p, hsa-miR-520g-5p, hsa-miR-504-3p, hsa-miR-487b-5p, hsa-miR-585-5p, hsa-miR-598-5p, hsa-miR-605-3p, hsa-miR-619-5p, hsa-miR-1296-3p, hsa-miR-874-5p, hsa-miR-887-5p, hsa-miR-513b-3p, hsa-miR-1908-3p, hsa-miR-3151-3p, hsa-miR-3192-3p, hsa-miR-3912-5p, hsa-miR-1343-5p, hsa-miR-5088-3p, hsa-miR-5189-3p, hsa-miR-5699-5p, hsa-miR-6726-5p, hsa-miR-6726-3p, hsa-miR-6727-5p, hsa-miR-6727-3p, hsa-miR-6728-5p, hsa-miR-6728-3p, hsa-miR-6729-5p, hsa-miR-6729-3p, hsa-miR-6730-5p, hsa-miR-6730-3p, hsa-miR-6731-5p, hsa-miR-6731-3p, hsa-miR-6732-5p, hsa-miR-6732-3p, hsa-miR-6734-5p, hsa-miR-6734-3p, hsa-miR-6735-5p, hsa-miR-6735-3p, hsa-miR-6736-5p, hsa-miR-6736-3p, hsa-miR-6737-5p, hsa-miR-6737-3p, hsa-miR-6738-5p, hsa-miR-6738-3p, hsa-miR-6740-5p, hsa-miR-6740-3p, hsa-miR-6741-5p, hsa-miR-6741-3p, hsa-miR-6742-5p, hsa-miR-6742-3p, hsa-miR-6743-5p, hsa-miR-6743-3p, hsa-miR-6744-5p, hsa-miR-6744-3p, hsa-miR-6745, hsa-miR-6746-5p, hsa-miR-6747-5p, hsa-miR-6747-3p, hsa-miR-6748-5p, hsa-miR-6749-5p, hsa-miR-6749-3p, hsa-miR-6750-5p, hsa-miR-6750-3p, hsa-miR-6751-5p, hsa-miR-6752-5p, hsa-miR-6752-3p, hsa-miR-6753-5p, hsa-miR-6753-3p, hsa-miR-6754-5p, hsa-miR-6754-3p, hsa-miR-6755-5p, hsa-miR-6756-5p, hsa-miR-6756-3p, hsa-miR-6757-5p, hsa-miR-6757-3p, hsa-miR-6758-5p, hsa-miR-6759-5p, hsa-miR-6759-3p, hsa-miR-6760-5p, hsa-miR-6760-3p, hsa-miR-6761-3p, hsa-miR-6762-5p, hsa-miR-6762-3p, hsa-miR-6763-5p, hsa-miR-6763-3p, hsa-miR-6765-5p, hsa-miR-6766-5p, hsa-miR-6766-3p, hsa-miR-6767-5p, hsa-miR-6768-5p, hsa-miR-6769a-5p, hsa-miR-6769a-3p, hsa-miR-6770-5p, hsa-miR-6771-5p, hsa-miR-6771-3p, hsa-miR-6772-5p, hsa-miR-6772-3p, hsa-miR-6773-3p, hsa-miR-6774-5p, hsa-miR-6775-5p, hsa-miR-6775-3p, hsa-miR-6776-5p, hsa-miR-6776-3p, hsa-miR-6777-5p, hsa-miR-6777-3p, hsa-miR-6778-5p, hsa-miR-6778-3p, hsa-miR-6779-5p, hsa-miR-6779-3p, hsa-miR-6780a-5p, hsa-miR-6780a-3p, hsa-miR-6781-5p, hsa-miR-6782-5p, hsa-miR-6782-3p, hsa-miR-6783-5p, hsa-miR-6783-3p, hsa-miR-6784-5p, hsa-miR-6784-3p, hsa-miR-6785-3p, hsa-miR-6786-5p, hsa-miR-6786-3p, hsa-miR-6787-5p, hsa-miR-6787-3p, hsa-miR-6788-5p, hsa-miR-6788-3p, hsa-miR-6789-5p, hsa-miR-6789-3p, hsa-miR-6790-5p, hsa-miR-6790-3p, hsa-miR-6791-5p, hsa-miR-6792-5p, hsa-miR-6792-3p, hsa-miR-6793-3p, hsa-miR-6794-5p, hsa-miR-6794-3p, hsa-miR-6795-5p, hsa-miR-6795-3p, hsa-miR-6796-5p, hsa-miR-6796-3p, hsa-miR-6797-5p, hsa-miR-6797-3p, hsa-miR-6798-5p, hsa-miR-6798-3p, hsa-miR-6799-5p, hsa-miR-6799-3p, hsa-miR-6800-5p, hsa-miR-6800-3p, hsa-miR-6801-3p, hsa-miR-6802-5p, hsa-miR-6802-3p, hsa-miR-6803-5p, hsa-miR-6803-3p, hsa-miR-6804-3p, hsa-miR-6805-5p, hsa-miR-6805-3p, hsa-miR-6806-5p, hsa-miR-6807-5p, hsa-miR-6808-5p, hsa-miR-6808-3p, hsa-miR-6809-5p, hsa-miR-6809-3p, hsa-miR-6810-5p, hsa-miR-6810-3p, hsa-miR-6812-5p, hsa-miR-6812-3p, hsa-miR-6813-5p, hsa-miR-6813-3p, hsa-miR-6815-5p, hsa-miR-6816-5p, hsa-miR-6816-3p, hsa-miR-6818-5p, hsa-miR-6819-5p, hsa-miR-6819-3p, hsa-miR-6820-5p, hsa-miR-6820-3p, hsa-miR-6821-5p, hsa-miR-6822-5p, hsa-miR-6823-5p, hsa-miR-6823-3p, hsa-miR-6824-5p, hsa-miR-6825-5p, hsa-miR-6825-3p, hsa-miR-6826-3p, hsa-miR-6827-5p, hsa-miR-6827-3p, hsa-miR-6828-5p, hsa-miR-6829-5p, hsa-miR-6829-3p, hsa-miR-6830-5p, hsa-miR-6830-3p, hsa-miR-6831-5p, hsa-miR-6831-3p, hsa-miR-6832-5p, hsa-miR-6832-3p, hsa-miR-6833-5p, hsa-miR-6833-3p, hsa-miR-6834-5p, hsa-miR-6780b-5p, hsa-miR-6780b-3p, hsa-miR-6836-3p, hsa-miR-6838-5p, hsa-miR-6839-5p, hsa-miR-6840-5p, hsa-miR-6840-3p, hsa-miR-6841-3p, hsa-miR-6842-5p, hsa-miR-6845-5p, hsa-miR-6845-3p, hsa-miR-6846-5p, hsa-miR-6846-3p, hsa-miR-6847-3p, hsa-miR-6848-5p, hsa-miR-6848-3p, hsa-miR-6849-5p, hsa-miR-6850-5p, hsa-miR-6851-5p, hsa-miR-6851-3p, hsa-miR-6855-5p, hsa-miR-6855-3p, hsa-miR-6856-5p, hsa-miR-6857-5p, hsa-miR-6857-3p, hsa-miR-6858-5p, hsa-miR-6858-3p, hsa-miR-6859-3p, hsa-miR-6769b-5p, hsa-miR-6769b-3p, hsa-miR-6860, hsa-miR-6861-5p, hsa-miR-6861-3p, hsa-miR-6862-5p, hsa-miR-6862-3p, hsa-miR-6865-5p, hsa-miR-6865-3p, hsa-miR-6867-5p, hsa-miR-6867-3p, hsa-miR-6868-3p, hsa-miR-6869-5p, hsa-miR-6870-5p, hsa-miR-6870-3p, hsa-miR-6871-5p, hsa-miR-6872-5p, hsa-miR-6873-5p, hsa-miR-6874-5p, hsa-miR-6875-5p, hsa-miR-6875-3p, hsa-miR-6876-5p, hsa-miR-6877-5p, hsa-miR-6877-3p, hsa-miR-6878-5p, hsa-miR-6878-3p, hsa-miR-6879-5p, hsa-miR-6879-3p, hsa-miR-6880-5p, hsa-miR-6880-3p, hsa-miR-6881-5p, hsa-miR-6882-5p, hsa-miR-6882-3p, hsa-miR-6883-5p, hsa-miR-6883-3p, hsa-miR-6884-3p, hsa-miR-6885-5p, hsa-miR-6885-3p, hsa-miR-6886-3p, hsa-miR-6887-5p, hsa-miR-6887-3p, hsa-miR-6889-5p, hsa-miR-6889-3p, hsa-miR-6890-5p, hsa-miR-6890-3p, hsa-miR-6891-5p, hsa-miR-6891-3p, hsa-miR-6892-5p, hsa-miR-6892-3p, hsa-miR-6893-5p, hsa-miR-6893-3p, hsa-miR-6894-5p, hsa-miR-6894-3p, hsa-miR-7106-5p, hsa-miR-7106-3p, hsa-miR-7107-5p, hsa-miR-7107-3p, hsa-miR-7108-5p, hsa-miR-7108-3p, hsa-miR-7109-5p, hsa-miR-7109-3p, hsa-miR-7110-5p, hsa-miR-7110-3p, hsa-miR-7111-Sp, hsa-miR-7111-3p, hsa-miR-7112-5p, hsa-miR-7113-3p, hsa-miR-7114-5p, hsa-miR-7114-3p, hsa-miR-7150, hsa-miR-7151-3p, hsa-miR-7152-5p, hsa-miR-7152-3p, hsa-miR-7155-5p, hsa-miR-7157-3p, hsa-miR-7159-5p, hsa-miR-7160-5p, hsa-miR-7162-5p, hsa-miR-7515, hsa-miR-7704, hsa-miR-7843-5p, hsa-miR-4433b-5p, hsa-miR-4433b-3p, hsa-miR-1273h-5p, hsa-miR-6516-5p, hsa-miR-7845-5p, hsa-miR-7846-3p, hsa-miR-7847-3p, hsa-miR-7851-3p, hsa-miR-7855-5p, hsa-miR-7856-5p, hsa-miR-8052, hsa-miR-8053, hsa-miR-8054, hsa-miR-8058, hsa-miR-8059, hsa-miR-8060, hsa-miR-8062, hsa-miR-8063, hsa-miR-8069, hsa-miR-8071, hsa-miR-8072, hsa-miR-8073, hsa-miR-8074, hsa-miR-8077, hsa-miR-8080, hsa-miR-8085, hsa-miR-8087, hsa-miR-8089, hsa-miR-128-2-5p, hsa-miR-7977, hsa-miR-7978, hsa-miR-1249-5p, hsa-miR-4485-5p, hsa-miR-8485, hsa-miR-135a-2-3p, hsa-miR-375-5p, hsa-miR-498-3p, hsa-miR-526a-3p, hsa-miR-9718, hsa-miR-9898, hsa-miR-9900, hsa-miR-10392-5p, hsa-miR-10392-3p, hsa-miR-10394-3p, hsa-miR-10395-3p, hsa-miR-10396a-5p, hsa-miR-10396a-3p, hsa-miR-10397-3p, hsa-miR-10398-5p, hsa-miR-10398-3p, hsa-miR-10399-3p, hsa-miR-10400-5p, hsa-miR-10400-3p, hsa-miR-10401-5p, hsa-miR-10401-3p, hsa-miR-10396b-5p, hsa-miR-10522-5p, hsa-miR-10526-3p, hsa-miR-10527-5p, hsa-miR-11181-3p, hsa-miR-11399, hsa-miR-11400, hsa-miR-3059-5p, hsa-miR-3059-3p, hsa-miR-3085-5p, hsa-miR-3085-3p, hsa-miR-6529-5p, hsa-miR-12114, hsa-miR-12115, hsa-miR-12116, hsa-miR-12118, hsa-miR-12119, hsa-miR-12120, hsa-miR-12121, hsa-miR-12122, hsa-miR-12124, hsa-miR-12126, hsa-miR-12131, and hsa-miR-12136. In the present disclosure, the extract of urine may be an extract of the urine of a stage-I kidney cancer patient. Only one of these microRNAs can be used to predict whether a subject from which the urine is derived is a kidney cancer patient (particularly stage-I), and 5 or more types, 10 or more types, 15 or more types, or 20 or more types of these microRNAs can be used in combination to predict whether a subject from which the urine is derived is a kidney cancer patient (particularly stage-I). Among these microRNAs, a microRNA that may be highly expressed in a kidney cancer patient (particularly stage-I) can be preferably used for prediction.

The present disclosure provides an extract of urine containing at least one type, two types, three types, four types, five types, six types, seven types, eight types, nine types, or ten types of or all microRNAs selected from the group consisting of microRNAs having an accuracy of higher than 0.500 among the microRNAs listed in Table 24-3. In the present disclosure, the extract of urine may be an extract of the urine of a kidney cancer patient. Only one of these microRNAs can be used to predict whether a subject from which the urine is derived is a kidney cancer patient, and 5 or more types, 10 or more types, 15 or more types, or 20 or more types of these microRNAs can be used in combination to predict whether a subject from which the urine is derived is a kidney cancer patient. Among these microRNAs, a microRNA that may be highly expressed in a kidney cancer patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

The present disclosure provides an extract of urine containing at least one type, two types, three types, four types, five types, six types, seven types, eight types, nine types, or ten types of or all microRNAs selected from the group consisting of microRNAs having an accuracy of higher than 0.500 among the microRNAs listed in Table 25-3. In the present disclosure, the extract of urine may be an extract of the urine of a kidney cancer patient. Only one of these microRNAs can be used to predict whether a subject from which the urine is derived is a kidney cancer patient, and 5 or more types, 10 or more types, 15 or more types, or 20 or more types of these microRNAs can be used in combination to predict whether a subject from which the urine is derived is a kidney cancer patient. A microRNA that may be highly expressed in a kidney cancer patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

The present disclosure provides an extract of urine containing at least one type, two types, three types, four types, five types, six types, seven types, eight types, nine types, or ten types of or all microRNAs selected from the microRNAs listed in Table 4-4. The present disclosure also provides an extract of urine containing at least one type, two types, three types, four types, five types, six types, seven types, eight types, nine types, or ten types of or all microRNAs selected from the group consisting of hsa-miR-1292-5p, hsa-miR-542-5p, hsa-miR-551b-5p, hsa-miR-135a-2-3p, hsa-miR-498-5p, hsa-miR-605-3p, hsa-miR-6750-5p, hsa-miR-4321, hsa-miR-4755-3p, hsa-miR-6791-5p, hsa-miR-10392-3p, hsa-miR-1268b, hsa-miR-6781-5p, hsa-miR-10400-3p, hsa-miR-3059-3p, hsa-miR-1343-5p, hsa-miR-3124-5p, hsa-miR-6081, hsa-miR-6125, hsa-miR-4745-5p, hsa-miR-4651, hsa-miR-4785, hsa-miR-6770-3p, hsa-miR-4507, hsa-miR-10392-5p, hsa-miR-6871-5p, hsa-miR-1207-5p, hsa-miR-4758-5p, hsa-miR-6743-5p, hsa-miR-6763-5p, hsa-miR-6131, hsa-miR-1908-5p, hsa-miR-3652, hsa-miR-3620-5p, hsa-miR-184, hsa-miR-6789-5p, hsa-miR-4257, hsa-miR-92a-2-5p, hsa-miR-6893-5p, hsa-miR-3648, hsa-miR-424-3p, hsa-miR-3937, hsa-miR-6836-5p, hsa-miR-939-5p, hsa-miR-1587, hsa-miR-11399, hsa-miR-6845-5p, hsa-miR-6726-5p, hsa-miR-629-5p, hsa-miR-92b-5p, hsa-miR-4665-5p, hsa-miR-6068, hsa-miR-4487, hsa-miR-135a-3p, hsa-miR-6850-5p, hsa-miR-I0401-5p, hsa-miR-601, hsa-miR-6836-3p, hsa-miR-12119, hsa-miR-3197, hsa-miR-4458, hsa-miR-638, hsa-miR-4505, hsa-miR-6777-5p, hsa-miR-6772-5p, hsa-miR-4673, hsa-miR-4648, hsa-miR-4535, hsa-miR-5196-5p, hsa-miR-4763-3p, hsa-miR-6861-5p, hsa-miR-5090, hsa-miR-3199, hsa-miR-193b-5p, hsa-miR-3135b, hsa-miR-106a-3p, hsa-miR-4498, hsa-miR-1303, hsa-miR-4258, hsa-miR-525-5p, hsa-miR-6732-5p, hsa-miR-4749-5p, hsa-miR-6515-5p, hsa-miR-8072, hsa-miR-650, hsa-miR-370-3p, hsa-miR-6820-5p, hsa-miR-6074, hsa-miR-6076, hsa-miR-6752-5p, hsa-miR-760, hsa-miR-6717-5p, hsa-miR-4695-5p, hsa-miR-8071, hsa-miR-187-5p, hsa-miR-4530, hsa-miR-1227-5p, hsa-miR-6796-5p, hsa-miR-6127, hsa-miR-6790-5p, hsa-miR-211-3p, hsa-miR-937-5p, hsa-miR-4470, hsa-miR-639, hsa-miR-4429, hsa-miR-6840-3p, hsa-miR-6798-5p, hsa-miR-5585-3p, hsa-miR-5189-5p, hsa-miR-8063, hsa-miR-8078, hsa-miR-494-3p, hsa-miR-3185, hsa-miR-4484, hsa-miR-4327, hsa-miR-2467-3p, hsa-miR-516a-5p, hsa-miR-7107-5p, hsa-miR-5010-5p, hsa-miR-130b-3p, hsa-miR-1914-3p, hsa-miR-7114-5p, hsa-miR-3917, hsa-miR-921, hsa-miR-4734, hsa-miR-4741, hsa-miR-3162-5p, hsa-miR-7515, hsa-miR-12114, hsa-miR-25-5p, hsa-miR-4440, hsa-miR-492, hsa-miR-3621, hsa-miR-4708-5p, hsa-miR-3187-3p, hsa-miR-4688, hsa-miR-12122, hsa-miR-6075, hsa-miR-6511b-5p, hsa-miR-4538, hsa-miR-6729-5p, hsa-miR-10396a-3p, hsa-miR-6766-5p, hsa-miR-4433a-3p, hsa-miR-6794-5p, hsa-miR-3165, hsa-miR-564, hsa-miR-3150b-3p, hsa-miR-7851-3p, hsa-miR-6788-5p, hsa-miR-6748-5p, hsa-miR-4486, hsa-miR-4433b-3p, hsa-miR-668-5p, hsa-miR-762, hsa-miR-617, hsa-miR-4449, hsa-miR-6783-5p, hsa-miR-4435, hsa-miR-4800-5p, hsa-miR-3200-5p, hsa-miR-4421, hsa-miR-6875-5p, hsa-miR-504-3p, hsa-miR-4727-3p, hsa-miR-4781-5p, hsa-miR-10526-3p, hsa-miR-6776-5p, hsa-miR-302a-3p, hsa-miR-3180-3p, hsa-miR-3928-3p, hsa-miR-6512-3p, hsa-miR-7111-Sp, hsa-miR-4260, hsa-miR-6751-Sp, hsa-miR-610, hsa-miR-6796-3p, hsa-miR-5190, hsa-miR-4638-5p, hsa-miR-485-5p, hsa-miR-6089, hsa-miR-4750-5p, hsa-miR-150-3p, hsa-miR-6821-5p, hsa-miR-4736, hsa-miR-10396a-5p, hsa-miR-4428, hsa-let-7d-3p, hsa-miR-6721-5p, hsa-miR-1843, hsa-miR-6805-5p, hsa-miR-6080, hsa-miR-11401, hsa-miR-6849-5p, hsa-miR-6792-5p, hsa-miR-769-3p, hsa-miR-371b-5p, hsa-miR-1915-3p, hsa-miR-4446-3p, hsa-miR-10396b-5p, hsa-miR-4300, hsa-miR-8077, hsa-miR-3178, hsa-miR-4447, hsa-miR-4467, hsa-miR-4669, hsa-miR-885-3p, hsa-miR-185-3p, hsa-miR-4516, hsa-miR-6879-5p, hsa-miR-4430, hsa-miR-138-5p, hsa-miR-145-5p, hsa-miR-4539, hsa-miR-6722-3p, hsa-miR-6784-5p, hsa-miR-6756-5p, hsa-miR-1909-3p, hsa-miR-1471, hsa-miR-5703, hsa-miR-378a-3p, hsa-miR-519c-5p, hsa-miR-523-5p, hsa-miR-518e-5p, hsa-miR-522-5p, hsa-miR-519a-5p, hsa-miR-519b-5p, hsa-miR-5100, hsa-miR-4691-5p, hsa-miR-4540, hsa-miR-7160-5p, hsa-miR-519a-3p, hsa-miR-6889-5p, hsa-miR-504-5p, hsa-miR-4439, hsa-miR-4466, hsa-miR-6090, hsa-miR-6787-5p, hsa-miR-6815-5p, hsa-miR-4499, hsa-miR-3151-5p, hsa-miR-1268a, hsa-miR-12127, hsa-miR-5685, hsa-miR-1285-3p, hsa-miR-3153, hsa-miR-6877-5p, hsa-miR-595, hsa-miR-6740-5p, hsa-miR-5572, hsa-miR-6782-5p, hsa-miR-3161, hsa-miR-6759-5p, hsa-miR-4322, hsa-miR-12118, hsa-miR-612, hsa-miR-486-3p, hsa-miR-3176, hsa-miR-8064, hsa-miR-4489, hsa-miR-4716-3p, hsa-miR-3691-5p, hsa-miR-4482-3p, hsa-miR-3689b-3p, hsa-miR-3689c, hsa-miR-6753-5p, hsa-miR-6810-5p, hsa-miR-718, hsa-miR-4481, hsa-miR-483-5p, hsa-miR-3173-5p, hsa-miR-6860, hsa-miR-4640-5p, hsa-miR-3616-3p, hsa-miR-4708-3p, hsa-miR-1307-3p, hsa-miR-6746-5p, hsa-miR-4701-3p, hsa-miR-4748, hsa-miR-4725-3p, hsa-miR-5189-3p, hsa-miR-3190-3p, hsa-miR-7156-3p, hsa-miR-6762-5p, hsa-miR-323a-5p, hsa-miR-6760-5p, hsa-miR-12116, hsa-miR-4776-5p, hsa-miR-500b-3p, hsa-miR-128-1-5p, hsa-miR-9718, hsa-miR-193a-5p, hsa-miR-5006-5p, hsa-miR-1237-5p, hsa-miR-1343-3p, hsa-miR-520b-3p, hsa-miR-320c, hsa-miR-491-5p, hsa-miR-5088-5p, hsa-miR-10398-3p, hsa-miR-2277-5p, hsa-miR-6730-5p, hsa-miR-1224-5p, hsa-miR-6758-5p, hsa-miR-6822-5p, hsa-miR-6876-5p, hsa-miR-138-1-3p, hsa-miR-6132, hsa-miR-4738-3p, hsa-miR-12131, hsa-miR-125b-1-3p, hsa-miR-4436b-3p, hsa-miR-4497, hsa-miR-6842-5p, hsa-miR-6747-5p, hsa-miR-6793-5p, hsa-miR-4444, hsa-miR-614, hsa-miR-6852-3p, hsa-miR-197-5p, hsa-miR-6855-5p, hsa-miR-1273h-5p, hsa-miR-3610, hsa-miR-887-3p, hsa-miR-3665, hsa-miR-3187-5p, hsa-miR-6745, hsa-miR-204-3p, hsa-miR-711, hsa-miR-4513, hsa-miR-6830-5p, hsa-miR-8073, hsa-miR-6805-3p, hsa-miR-7856-5p, hsa-miR-4265, hsa-miR-12120, hsa-miR-550a-5p, hsa-miR-4655-5p, hsa-miR-3196, hsa-miR-139-3p, hsa-miR-7154-3p, hsa-miR-4721, hsa-miR-4496, hsa-miR-296-3p, hsa-miR-8088, hsa-miR-8069, hsa-miR-5584-5p, hsa-miR-1296-3p, hsa-miR-6823-5p, hsa-miR-575, hsa-miR-6762-3p, hsa-miR-373-3p, hsa-miR-4707-5p, hsa-miR-214-5p, hsa-miR-3141, hsa-miR-520e-3p, hsa-miR-7854-3p, hsa-miR-449c-5p, hsa-miR-608, hsa-miR-5093, hsa-miR-4654, hsa-miR-574-5p, hsa-miR-6515-3p, hsa-miR-6894-5p, hsa-miR-65 11a-5p, hsa-miR-3922-5p, hsa-miR-6780b-5p, hsa-miR-1972, hsa-miR-4746-3p, hsa-miR-766-5p, hsa-miR-4508, hsa-miR-320d, hsa-miR-1301-5p, hsa-miR-6892-5p, hsa-miR-323a-3p, hsa-miR-4706, hsa-miR-658, hsa-miR-6773-3p, hsa-miR-4465, hsa-miR-181d-3p, hsa-miR-10527-5p, hsa-miR-8074, hsa-miR-6816-5p, hsa-miR-1469, hsa-miR-2110, hsa-miR-342-3p, hsa-miR-6754-3p, hsa-miR-6737-5p, hsa-miR-4456, hsa-miR-149-3p, hsa-miR-1538, hsa-miR-6834-5p, hsa-miR-4634, hsa-miR-3663-5p, hsa-miR-1236-5p, hsa-miR-30b-3p, hsa-miR-892b, hsa-miR-4314, hsa-miR-6514-3p, hsa-miR-20b-3p, hsa-miR-8052, hsa-miR-194-3p, hsa-miR-1249-3p, hsa-miR-585-5p, hsa-miR-4652-5p, hsa-miR-6780a-5p, hsa-miR-466, hsa-miR-1470, hsa-miR-1908-3p, hsa-miR-487b-5p, hsa-miR-3177-3p, hsa-miR-4710, hsa-miR-214-3p, hsa-miR-198, hsa-miR-4650-3p, hsa-miR-105-5p, hsa-miR-583, hsa-miR-4485-5p, hsa-miR-7151-3p, hsa-miR-10400-5p, hsa-miR-6833-5p, hsa-miR-6829-5p, hsa-miR-4269, hsa-miR-4658, hsa-miR-551a, hsa-miR-6786-5p, hsa-miR-665, hsa-miR-382-5p, hsa-miR-5787, hsa-miR-6857-5p, hsa-miR-632, hsa-miR-4443, hsa-miR-5587-5p, hsa-miR-3064-5p, hsa-miR-3646, hsa-miR-508-5p, hsa-miR-3660, hsa-miR-7977, hsa-miR-2276-3p, hsa-miR-4769-5p, hsa-miR-548g-3p, hsa-miR-6837-5p, hsa-miR-6070, hsa-miR-487a-5p, hsa-miR-6720-5p, hsa-miR-4485-3p, hsa-miR-6846-5p, hsa-miR-6086, hsa-miR-6734-5p, hsa-miR-3605-5p, hsa-miR-6813-5p, hsa-miR-4326, hsa-miR-541-3p, hsa-miR-4455, hsa-miR-4296, hsa-miR-3147, hsa-miR-4684-3p, hsa-miR-3126-5p, hsa-miR-4479, hsa-miR-4674, hsa-miR-8075, hsa-miR-12128, hsa-miR-3940-5p, hsa-miR-3960, hsa-miR-4299, hsa-miR-6859-3p, hsa-miR-4483, hsa-miR-3186-3p, hsa-miR-421, hsa-miR-6500-3p, hsa-miR-1193, hsa-miR-11181-3p, hsa-miR-3622a-5p, hsa-miR-1231, hsa-miR-5589-5p, hsa-miR-6768-5p, hsa-miR-8089, hsa-miR-6772-3p, hsa-miR-6886-5p, hsa-miR-4433a-5p, hsa-miR-3617-3p, hsa-miR-4252, hsa-miR-6071, hsa-miR-6800-5p, hsa-miR-3155a, hsa-miR-744-5p, hsa-miR-4664-5p, hsa-miR-3130-3p, hsa-miR-4441, hsa-miR-4787-5p, hsa-miR-4747-5p, hsa-miR-5009-3p, hsa-miR-4276, hsa-miR-11400, hsa-miR-10401-3p, hsa-miR-3679-3p, hsa-miR-3934-5p, hsa-miR-3085-5p, hsa-miR-6724-5p, hsa-miR-4448, hsa-miR-6727-5p, hsa-miR-6501-3p, hsa-miR-6831-5p, hsa-miR-1228-3p, hsa-miR-6848-5p, hsa-miR-6501-5p, hsa-miR-7110-5p, hsa-miR-6083, hsa-miR-1266-3p, hsa-miR-920, hsa-miR-2392, hsa-miR-3934-3p, hsa-miR-6165, hsa-miR-670-5p, hsa-miR-202-3p, hsa-miR-4463, hsa-miR-6529-5p, hsa-miR-4431, hsa-miR-6816-3p, hsa-miR-6767-5p, hsa-miR-8059, hsa-miR-6883-5p, hsa-miR-6893-3p, hsa-miR-6072, hsa-miR-7976, hsa-miR-4533, hsa-miR-6868-3p, hsa-miR-5705, hsa-miR-3131, hsa-miR-6875-3p, hsa-miR-5698, hsa-miR-3622a-3p, hsa-miR-122b-3p, hsa-miR-6869-5p, hsa-miR-6763-3p, hsa-miR-3155b, hsa-miR-449c-3p, and hsa-miR-3189-3p. In the present disclosure, the extract of urine may be an extract of the urine of a leukemia patient. Only one of these microRNAs can be used to predict whether a subject from which the urine is derived is a leukemia patient, and 5 or more types, 10 or more types, 15 or more types, or 20 or more types of these microRNAs can be used in combination to predict whether a subject from which the urine is derived is a leukemia patient. Among these microRNAs, a microRNA that may be highly expressed in a leukemia patient can be preferably used for prediction.

The present disclosure provides an extract of urine containing at least one type, two types, three types, four types, five types, six types, seven types, eight types, nine types, or ten types of or all microRNAs selected from the group consisting of microRNAs having an accuracy of higher than 0.500 among the microRNAs listed in Table 24-4. In the present disclosure, the extract of urine may be an extract of the urine of a leukemia patient. Only one of these microRNAs can be used to predict whether a subject from which the urine is derived is a leukemia patient, and 5 or more types, 10 or more types, 15 or more types, or 20 or more types of these microRNAs can be used in combination to predict whether a subject from which the urine is derived is a leukemia patient. Among these microRNAs, a microRNA that may be highly expressed in a leukemia patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

The present disclosure provides an extract of urine containing at least one type, two types, three types, four types, five types, six types, seven types, eight types, nine types, or ten types of or all microRNAs selected from the group consisting of microRNAs having an accuracy of higher than 0.500 among the microRNAs listed in Table 25-4. In the present disclosure, the extract of urine may be an extract of the urine of a leukemia patient. Only one of these microRNAs can be used to predict whether a subject from which the urine is derived is a leukemia patient, and 5 or more types, 10 or more types, 15 or more types, or 20 or more types of these microRNAs can be used in combination to predict whether a subject from which the urine is derived is a leukemia patient. Among these microRNAs, a microRNA that may be highly expressed in a leukemia patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

The present disclosure provides an extract of urine containing at least one type, two types, three types, four types, five types, six types, seven types, eight types, nine types, or ten types of or all microRNAs selected from the microRNAs listed in Table 4-5. The present disclosure also provides an extract of urine containing at least one type, two types, three types, four types, five types, six types, seven types, eight types, nine types, or ten types of or all microRNAs selected from the group consisting of hsa-miR-605-3p, hsa-miR-498-5p, hsa-miR-55 1b-5p, hsa-miR-541-3p, hsa-miR-10526-3p, hsa-miR-4321, hsa-miR-150-3p, hsa-miR-3199, hsa-miR-6770-5p, hsa-miR-1914-3p, hsa-miR-7108-3p, hsa-miR-6081, hsa-miR-1470, hsa-miR-4727-3p, hsa-miR-6074, hsa-miR-629-5p, hsa-miR-9898, hsa-miR-6809-5p, hsa-miR-4684-3p, hsa-miR-6514-5p, hsa-miR-1343-5p, hsa-miR-6794-5p, hsa-miR-6790-3p, hsa-miR-10392-3p, hsa-miR-135a-2-3p, hsa-miR-6812-3p, hsa-miR-6763-3p, hsa-miR-4433b-3p, hsa-miR-6731-3p, hsa-miR-4651, hsa-miR-4327, hsa-miR-6810-3p, hsa-miR-6836-3p, hsa-miR-6783-5p, hsa-miR-1207-5p, hsa-miR-6781-5p, hsa-miR-1268b, hsa-miR-4673, hsa-miR-6501-5p, hsa-miR-6892-3p, hsa-miR-601, hsa-miR-6796-3p, hsa-miR-6891-3p, hsa-miR-3124-5p, hsa-miR-4300, hsa-miR-4447, hsa-miR-6859-3p, hsa-miR-7111-5p, hsa-miR-4750-3p, hsa-miR-4741, hsa-miR-921, hsa-miR-8078, hsa-miR-4465, hsa-miR-12122, hsa-miR-10400-3p, hsa-miR-3150b-5p, hsa-miR-6760-3p, hsa-miR-4433a-5p, hsa-miR-6784-3p, hsa-miR-6795-3p, hsa-miR-3127-3p, hsa-miR-1303, hsa-miR-518b, hsa-miR-6750-5p, hsa-miR-12120, hsa-miR-92a-2-5p, hsa-miR-6829-5p, hsa-miR-6845-5p, hsa-miR-6819-3p, hsa-miR-4313, hsa-miR-769-3p, hsa-miR-6729-3p, hsa-miR-6805-3p, hsa-miR-3162-3p, hsa-miR-5196-5p, hsa-miR-4720-5p, hsa-miR-193b-5p, hsa-miR-10396a-3p, hsa-miR-3679-3p, hsa-miR-5190, hsa-miR-320e, hsa-miR-4433b-5p, hsa-miR-6791-5p, hsa-miR-6858-3p, hsa-miR-617, hsa-miR-3660, hsa-miR-3180-5p, hsa-miR-6789-5p, hsa-miR-550b-2-5p, hsa-miR-8063, hsa-miR-6785-3p, hsa-miR-625-3p, hsa-miR-513b-5p, hsa-miR-4433a-3p, hsa-miR-133a-3p, hsa-miR-4665-3p, hsa-miR-4258, hsa-miR-3675-3p, hsa-miR-3937, hsa-miR-6813-3p, hsa-miR-1249-3p, hsa-miR-6746-5p, hsa-miR-6080, hsa-miR-4758-5p, hsa-miR-4286, hsa-miR-665, hsa-miR-937-5p, hsa-miR-7114-3p, hsa-miR-135a-3p, hsa-miR-6500-3p, hsa-miR-6756-3p, hsa-miR-6766-3p, hsa-miR-6515-5p, hsa-miR-6125, hsa-miR-361-3p, hsa-miR-4749-5p, hsa-miR-5703, hsa-miR-7851-3p, hsa-miR-4261, hsa-miR-4257, hsa-miR-1225-3p, hsa-miR-1237-3p, hsa-miR-1228-3p, hsa-miR-3666, hsa-miR-20b-3p, hsa-miR-3648, hsa-miR-4749-3p, hsa-miR-6515-3p, hsa-miR-6798-3p, hsa-miR-1908-5p, hsa-miR-365a-3p, hsa-miR-365b-3p, hsa-miR-12116, hsa-miR-6743-5p, hsa-miR-6815-5p, hsa-miR-4274, hsa-miR-4537, hsa-miR-1238-3p, hsa-miR-519c-5p, hsa-miR-523-5p, hsa-miR-518e-5p, hsa-miR-522-5p, hsa-miR-519a-5p, hsa-miR-519b-5p, hsa-miR-6836-5p, hsa-miR-6795-5p, hsa-miR-9986, hsa-miR-4280, hsa-miR-3621, hsa-miR-6805-5p, hsa-miR-184, hsa-miR-424-3p, hsa-miR-4649-3p, hsa-miR-4716-5p, hsa-miR-1915-3p, hsa-miR-668-5p, hsa-miR-6841-3p, hsa-miR-7156-3p, hsa-miR-5093, hsa-miR-4534, hsa-miR-6832-5p, hsa-miR-650, hsa-miR-6758-5p, hsa-miR-4755-3p, hsa-miR-542-5p, hsa-miR-3918, hsa-miR-4436b-3p, hsa-miR-762, hsa-miR-4521, hsa-miR-1224-3p, hsa-miR-6752-5p, hsa-miR-1913, hsa-miR-8088, hsa-miR-6850-5p, hsa-miR-3197, hsa-miR-18b-3p, hsa-miR-187-5p, hsa-miR-1909-3p, hsa-miR-4728-5p, hsa-miR-6777-3p, hsa-miR-6763-5p, hsa-miR-6784-5p, hsa-miR-4283, hsa-miR-4435, hsa-miR-6821-5p, hsa-miR-4785, hsa-miR-6722-3p, hsa-miR-1227-5p, hsa-miR-7856-5p, hsa-miR-4728-3p, hsa-miR-4308, hsa-miR-3614-5p, hsa-miR-1226-5p, hsa-miR-6887-5p, hsa-miR-4284, hsa-miR-6884-3p, hsa-miR-6129, hsa-miR-6797-5p, hsa-miR-6797-3p, hsa-miR-628-3p, hsa-miR-6775-3p, hsa-miR-6165, hsa-miR-3187-3p, hsa-miR-6880-3p, hsa-miR-4731-3p, hsa-miR-6761-5p, hsa-miR-7107-5p, hsa-miR-6752-3p, hsa-miR-3180-3p, hsa-miR-4745-5p, hsa-miR-1469, hsa-miR-4654, hsa-miR-6756-5p, hsa-miR-4429, hsa-let-7f-1-3p, hsa-miR-25-5p, hsa-miR-30b-3p, hsa-miR-3620-5p, hsa-miR-4658, hsa-miR-1236-5p, hsa-miR-4479, hsa-miR-6887-3p, hsa-miR-4466, hsa-miR-6870-3p, hsa-miR-29a-3p, hsa-miR-6800-3p, hsa-miR-638, hsa-miR-4299, hsa-miR-8064, hsa-miR-718, hsa-miR-3133, hsa-miR-4657, hsa-miR-4665-5p, hsa-miR-4323, hsa-miR-4634, hsa-miR-6823-5p, hsa-miR-1185-2-3p, hsa-miR-6762-5p, hsa-miR-1268a, hsa-miR-4315, hsa-miR-6749-3p, hsa-miR-486-3p, hsa-miR-4507, hsa-miR-3619-5p, hsa-miR-4763-3p, hsa-miR-492, hsa-miR-449c-5p, hsa-miR-550a-3-5p, hsa-miR-3943, hsa-miR-3682-3p, hsa-miR-6747-5p, hsa-miR-1185-1-3p, hsa-miR-6850-3p, hsa-miR-4296, hsa-miR-8077, hsa-miR-10396a-5p, hsa-miR-7854-3p, hsa-miR-4756-3p, hsa-miR-6845-3p, hsa-miR-10401-5p, hsa-miR-4707-5p, hsa-miR-210-5p, hsa-miR-7151-3p, hsa-miR-4506, hsa-miR-1237-5p, hsa-miR-92b-5p, hsa-miR-711, hsa-miR-6895-5p, hsa-miR-6503-3p, hsa-miR-579-3p, hsa-miR-5739, hsa-miR-4754, hsa-miR-8057, hsa-miR-6724-5p, hsa-miR-6749-5p, hsa-miR-8072, hsa-miR-4687-5p, hsa-miR-5009-3p, hsa-miR-4294, hsa-miR-3934-5p, hsa-miR-7150, hsa-miR-6090, hsa-miR-3610, hsa-miR-4648, hsa-miR-4635, hsa-miR-4436a, hsa-miR-6721-5p, hsa-miR-3186-3p, hsa-miR-3667-5p, hsa-miR-6777-5p, hsa-miR-4484, hsa-miR-4446-3p, hsa-miR-4516, hsa-miR-3651, hsa-miR-6893-5p, hsa-miR-4783-3p, hsa-miR-6889-5p, hsa-miR-6720-5p, hsa-miR-5585-3p, hsa-miR-5006-5p, hsa-miR-4684-5p, hsa-miR-6131, hsa-miR-193a-5p, hsa-miR-6876-5p, hsa-miR-6855-5p, hsa-miR-1972, hsa-miR-6729-5p, hsa-miR-1304-3p, hsa-miR-7151-5p, hsa-miR-4505, hsa-miR-940, hsa-miR-6861-3p, hsa-miR-6859-5p, hsa-miR-494-3p, hsa-miR-1321, hsa-miR-939-5p, hsa-miR-8075, hsa-miR-8083, hsa-miR-516b-3p, hsa-miR-516a-3p, hsa-miR-3130-3p, hsa-miR-4449, hsa-miR-595, hsa-miR-7113-5p, hsa-miR-583, hsa-miR-5090, hsa-miR-501-5p, hsa-miR-3927-5p, hsa-miR-4454, hsa-miR-11399, hsa-miR-3940-5p, hsa-miR-3059-3p, hsa-miR-3689b-3p, hsa-miR-3689c, hsa-miR-4640-3p, hsa-miR-4494, hsa-miR-584-3p, hsa-miR-3131, hsa-miR-10392-5p, hsa-miR-4295, hsa-miR-200c-5p, hsa-miR-644a, hsa-miR-3122, hsa-miR-4701-3p, hsa-miR-6726-5p, hsa-miR-6770-3p, hsa-miR-3178, hsa-miR-6130, hsa-miR-371b-5p, hsa-miR-4708-3p, hsa-miR-6816-5p, hsa-miR-6854-3p, hsa-miR-105-5p, hsa-miR-485-5p, hsa-miR-584-5p, hsa-miR-4784, hsa-miR-3150b-3p, hsa-miR-149-3p, hsa-miR-1225-5p, hsa-miR-4746-3p, hsa-miR-6068, hsa-miR-4474-3p, hsa-miR-6787-5p, hsa-miR-4540, hsa-miR-3074-3p, hsa-miR-4682, hsa-miR-7976, hsa-miR-323a-5p, hsa-miR-6776-5p, hsa-miR-3173-3p, hsa-miR-6767-5p, hsa-miR-3689a-3p, hsa-miR-296-5p, hsa-miR-4740-5p, hsa-miR-1236-3p, hsa-miR-5708, hsa-miR-3184-3p, hsa-miR-6768-5p, hsa-miR-4716-3p, hsa-miR-369-5p, hsa-miR-3195, hsa-miR-3141, hsa-miR-4489, hsa-miR-4691-5p, hsa-miR-34b-3p, hsa-miR-3622a-5p, hsa-miR-4736, hsa-miR-1276, hsa-miR-4463, hsa-miR-484, hsa-miR-4783-5p, hsa-miR-4733-3p, hsa-miR-338-5p, hsa-miR-4304, hsa-miR-10523-5p, hsa-miR-12119, hsa-miR-6871-5p, hsa-miR-10396b-5p, hsa-miR-4530, hsa-miR-6732-5p, hsa-miR-4421, hsa-miR-3135b, hsa-miR-770-5p, hsa-miR-6793-5p, hsa-miR-4255, hsa-miR-4487, hsa-miR-4647, hsa-miR-4688, hsa-miR-138-5p, hsa-miR-4738-3p, hsa-miR-3085-3p, hsa-miR-196b-3p, hsa-miR-1301-5p, hsa-miR-6127, hsa-miR-2467-3p, hsa-miR-10522-5p, hsa-miR-6529-5p, hsa-let-7b-3p, hsa-miR-323b-5p, hsa-miR-2276-3p, hsa-miR-6089, hsa-miR-6075, hsa-miR-3922-5p, hsa-miR-4508, hsa-miR-365a-5p, hsa-miR-506-3p, hsa-miR-6717-5p, hsa-miR-5787, hsa-miR-4311, hsa-miR-5685, hsa-miR-593-5p, hsa-miR-6848-3p, hsa-miR-3200-5p, hsa-miR-4535, hsa-miR-4672, hsa-miR-8070, hsa-miR-12114, hsa-miR-4769-5p, hsa-miR-1286, hsa-miR-378a-3p, hsa-miR-6506-5p, hsa-miR-608, hsa-miR-4538, hsa-miR-6790-5p, hsa-miR-9851-5p, hsa-miR-30c-2-3p, hsa-miR-6071, hsa-miR-3196, hsa-miR-6884-5p, hsa-miR-6788-5p, hsa-miR-4725-3p, hsa-miR-3185, hsa-miR-6086, hsa-miR-1471, hsa-miR-5681b, hsa-miR-6822-3p, hsa-miR-6069, hsa-miR-1292-5p, hsa-miR-1587, hsa-miR-769-5p, hsa-miR-5006-3p, hsa-miR-3137, hsa-miR-4472, hsa-miR-202-3p, hsa-miR-6772-5p, hsa-miR-5004-3p, hsa-miR-1306-3p, hsa-miR-3652, hsa-miR-9718, hsa-miR-6764-3p, hsa-miR-5189-5p, hsa-miR-6820-5p, hsa-miR-8069, hsa-miR-3663-3p, hsa-miR-4498, hsa-miR-4499, hsa-miR-4441, hsa-miR-3158-3p, hsa-miR-433-5p, hsa-miR-3162-5p, hsa-miR-4695-5p, hsa-miR-3677-3p, hsa-miR-4252, hsa-miR-3155a, hsa-miR-4317, hsa-miR-4520-5p, hsa-miR-129-5p, hsa-miR-6861-5p, hsa-miR-7154-3p, hsa-miR-4439, hsa-miR-4497, hsa-miR-4451, hsa-miR-3155b, hsa-miR-4458, hsa-miR-500b-3p, hsa-miR-5000-5p, hsa-miR-2681-3p, hsa-miR-4734, hsa-miR-3180, hsa-miR-5580-5p, hsa-miR-3192-5p, hsa-miR-7114-5p, hsa-miR-4251, hsa-miR-6875-5p, hsa-miR-138-1-3p, hsa-miR-6780b-5p, hsa-miR-6124, hsa-miR-651 1b-5p, hsa-miR-7160-5p, hsa-miR-194-3p, hsa-miR-34c-5p, hsa-miR-2116-5p, hsa-miR-575, hsa-miR-5100, hsa-miR-4298, hsa-miR-6798-5p, hsa-miR-708-5p, hsa-miR-211-3p, hsa-miR-4488, hsa-miR-448, hsa-miR-1204, hsa-miR-3157-3p, hsa-miR-2277-5p, hsa-miR-3161, hsa-miR-4529-5p, hsa-miR-7850-5p, hsa-miR-10398-3p, hsa-miR-4674, hsa-miR-6801-5p, hsa-miR-197-5p, hsa-miR-5572, hsa-let-7d-3p, hsa-miR-3680-3p, hsa-miR-6800-5p, hsa-miR-1297, hsa-miR-138-2-3p, hsa-miR-7162-3p, hsa-miR-4428, hsa-miR-4314, hsa-miR-4769-3p, hsa-miR-892b, hsa-miR-345-3p, hsa-miR-6083, hsa-miR-513c-5p, hsa-miR-4686, hsa-miR-4638-5p, hsa-miR-3154, hsa-miR-12127, hsa-miR-3187-5p, hsa-miR-4444, hsa-miR-766-5p, hsa-miR-4453, hsa-miR-3190-3p, hsa-miR-1227-3p, hsa-miR-6511a-5p, hsa-miR-6782-5p, hsa-miR-6830-5p, hsa-miR-6840-3p, hsa-miR-2682-5p, hsa-miR-6513-5p, hsa-miR-4486, hsa-miR-593-3p, hsa-miR-3944-5p, hsa-miR-612, hsa-miR-433-3p, hsa-miR-6730-5p, hsa-miR-6817-5p, hsa-miR-12121, hsa-miR-378g, hsa-miR-3692-5p, hsa-miR-6874-5p, hsa-miR-887-3p, hsa-miR-604, hsa-miR-4676-5p, hsa-miR-3713, hsa-miR-3936, hsa-miR-371a-3p, hsa-miR-548q, hsa-miR-6804-5p, hsa-miR-487a-3p, hsa-miR-378b, hsa-miR-580-3p, hsa-miR-6085, hsa-miR-4288, hsa-miR-4482-3p, hsa-miR-299-3p, hsa-miR-320c, hsa-miR-4322, hsa-miR-4303, hsa-miR-2116-3p, hsa-miR-1234-3p, hsa-miR-1912-3p, hsa-miR-6786-5p, hsa-miR-622, hsa-miR-564, hsa-miR-6738-5p, hsa-miR-3665, hsa-miR-6879-5p, hsa-miR-4450, hsa-miR-3605-5p, hsa-miR-3917, hsa-miR-4531, hsa-miR-3909, hsa-miR-760, hsa-miR-221-3p, hsa-miR-4259, hsa-miR-4664-5p, hsa-miR-6132, hsa-miR-7106-5p, hsa-miR-3612, hsa-miR-6740-5p, hsa-miR-6748-5p, hsa-miR-4653-5p, hsa-miR-103a-1-5p, hsa-miR-1251-3p, hsa-miR-4456, hsa-miR-300, hsa-miR-3661, hsa-miR-6765-5p, hsa-miR-5571-3p, hsa-miR-874-5p, hsa-miR-139-3p, hsa-miR-7109-5p, hsa-miR-6812-5p, hsa-miR-4526, hsa-miR-330-5p, hsa-miR-625-5p, hsa-miR-7975, hsa-miR-4743-5p, hsa-miR-6802-3p, hsa-miR-6505-3p, hsa-miR-6499-3p, hsa-miR-6766-5p, hsa-miR-4708-5p, hsa-miR-6500-5p, hsa-miR-1284, hsa-miR-6886-5p, hsa-miR-1293, hsa-miR-6849-5p, hsa-miR-6869-5p, hsa-miR-885-3p, hsa-miR-3616-3p, hsa-miR-3655, hsa-miR-3922-3p, hsa-miR-6843-3p, hsa-miR-6833-5p, hsa-miR-4467, hsa-miR-6759-5p, hsa-miR-933, hsa-miR-6512-3p, hsa-miR-6834-5p, hsa-miR-146a-5p, hsa-miR-6818-3p, hsa-miR-12117, hsa-miR-6885-3p, hsa-miR-6837-5p, hsa-miR-4667-5p, hsa-miR-134-3p, hsa-miR-219b-5p, hsa-miR-1468-5p, hsa-miR-4513, hsa-miR-7848-3p, hsa-miR-124-5p, hsa-miR-324-5p, hsa-miR-12118, hsa-miR-340-3p, hsa-miR-552-5p, hsa-miR-6737-5p, hsa-miR-525-5p, hsa-miR-589-3p, hsa-miR-520e-3p, hsa-miR-4525, hsa-miR-4685-5p, hsa-miR-3617-3p, hsa-miR-3170, hsa-miR-6792-5p, hsa-miR-6791-3p, hsa-miR-660-3p, hsa-miR-6892-5p, hsa-miR-3928-3p, hsa-miR-5189-3p, hsa-miR-4758-3p, hsa-miR-3689d, hsa-miR-4440, hsa-miR-504-3p, hsa-miR-663a, hsa-miR-4776-3p, hsa-miR-5091, hsa-miR-1538, hsa-miR-8089, hsa-miR-4425, hsa-miR-2681-5p, hsa-miR-4726-3p, hsa-miR-5194, hsa-miR-5011-3p, hsa-miR-26b-3p, hsa-miR-4747-5p, hsa-miR-6779-5p, hsa-miR-487a-5p, hsa-miR-18a-3p, hsa-miR-7152-3p, hsa-miR-342-5p, hsa-miR-183-3p, hsa-miR-4640-5p, hsa-miR-4767, hsa-miR-6501-3p, hsa-miR-450a-2-3p, hsa-miR-3529-5p, hsa-miR-128-1-5p, hsa-miR-6762-3p, hsa-miR-7847-3p, hsa-miR-3160-5p, hsa-miR-8082, hsa-miR-432-5p, hsa-miR-185-3p, hsa-miR-5589-5p, hsa-miR-6860, hsa-miR-11181-3p, hsa-miR-99b-3p, hsa-miR-520b-3p, hsa-miR-6745, hsa-miR-6741-5p, hsa-miR-5002-3p, hsa-miR-1203, hsa-miR-4276, hsa-miR-4481, hsa-miR-3619-3p, hsa-miR-744-5p, hsa-miR-3690, hsa-miR-4656, hsa-miR-1301-3p, hsa-miR-5004-5p, hsa-miR-6813-5p, hsa-miR-4437, hsa-miR-1843, hsa-miR-8085, hsa-miR-4750-5p, hsa-miR-5588-3p, hsa-miR-4491, hsa-miR-5002-5p, hsa-miR-4692, hsa-miR-381-5p, hsa-miR-4763-5p, hsa-miR-6814-5p, hsa-miR-181a-2-3p, hsa-miR-4438, hsa-miR-1281, hsa-miR-328-3p, hsa-miR-4800-5p, hsa-miR-1322, hsa-miR-371b-3p, hsa-miR-6773-3p, hsa-miR-3646, hsa-miR-6852-5p, hsa-miR-6848-5p, hsa-miR-494-5p, hsa-miR-6774-3p, hsa-miR-6831-5p, hsa-miR-146b-3p, hsa-miR-3085-5p, hsa-miR-4316, hsa-miR-1285-3p, hsa-miR-2114-5p, hsa-miR-4780, hsa-miR-4473, hsa-miR-6889-3p, hsa-miR-3150a-5p, hsa-miR-654-5p, hsa-miR-6727-5p, hsa-miR-2110, hsa-miR-103a-2-5p, hsa-miR-888-3p, hsa-miR-125b-2-3p, hsa-miR-4670-5p, hsa-miR-6796-5p, hsa-miR-6855-3p, hsa-miR-28-5p, hsa-miR-4787-5p, hsa-miR-4319, hsa-miR-1228-5p, hsa-miR-5696, hsa-miR-6504-3p, hsa-miR-3176, hsa-miR-513a-5p, hsa-miR-135b-5p, hsa-miR-4492, hsa-miR-4430, hsa-miR-4455, hsa-miR-6718-5p, hsa-miR-15b-3p, hsa-miR-4707-3p, hsa-miR-4700-5p, hsa-miR-214-3p, hsa-miR-4723-5p, hsa-miR-6842-5p, hsa-miR-3144-3p, hsa-miR-6821-3p, hsa-miR-614, hsa-miR-658, hsa-miR-4273, hsa-miR-4265, hsa-miR-25-3p, hsa-miR-2909, hsa-miR-5187-5p, hsa-miR-4723-3p, hsa-miR-6760-5p, hsa-miR-7108-5p, hsa-miR-4522, hsa-miR-4764-3p, hsa-miR-8071, hsa-miR-3186-5p, hsa-miR-4306, hsa-miR-632, hsa-miR-4800-3p, hsa-miR-217-3p, hsa-miR-370-3p, hsa-miR-4748, hsa-miR-5584-3p, hsa-miR-483-5p, hsa-miR-6513-3p, hsa-miR-4652-5p, hsa-miR-6738-3p, hsa-miR-1273h-3p, hsa-miR-877-5p, hsa-miR-550a-5p, hsa-miR-3654, hsa-miR-4724-3p, hsa-miR-676-5p, hsa-miR-6751-5p, hsa-miR-192-5p, hsa-miR-1231, hsa-miR-493-3p, hsa-miR-4649-5p, hsa-miR-4730, hsa-miR-4262, hsa-miR-4669, hsa-miR-200b-3p, hsa-miR-4667-3p, hsa-miR-563, hsa-miR-3144-5p, hsa-miR-516b-5p, hsa-miR-488-5p, hsa-miR-4762-5p, hsa-miR-1287-3p, hsa-miR-382-5p, hsa-miR-122b-3p, hsa-miR-548ba, hsa-miR-8062, hsa-miR-616-5p, hsa-miR-5010-5p, hsa-miR-761, hsa-miR-3659, hsa-miR-4717-5p, hsa-miR-519e-3p, hsa-miR-4633-5p, hsa-miR-585-5p, hsa-miR-1178-3p, hsa-miR-6862-5p, hsa-miR-5705, hsa-miR-411-3p, hsa-miR-3138, hsa-miR-3685, hsa-miR-615-5p, hsa-miR-6820-3p, hsa-miR-4533, hsa-miR-4776-5p, hsa-miR-7158-5p, hsa-miR-551a, hsa-miR-551b-3p, hsa-miR-3691-3p, hsa-miR-11401, hsa-miR-610, hsa-miR-4524b-3p, hsa-miR-6499-5p, hsa-miR-6877-5p, hsa-miR-210-3p, hsa-miR-6895-3p, hsa-miR-449c-3p, hsa-miR-214-5p, hsa-miR-6728-5p, hsa-miR-6817-3p, hsa-miR-6076, hsa-miR-4502, hsa-miR-16-2-3p, hsa-miR-6872-5p, hsa-miR-1273h-5p, hsa-miR-1343-3p, hsa-miR-920, hsa-miR-4431, hsa-miR-187-3p, hsa-miR-6126, hsa-miR-5195-3p, hsa-miR-6874-3p, hsa-miR-34c-3p, hsa-miR-4999-3p, hsa-miR-3935, hsa-miR-5088-5p, hsa-miR-1224-5p, hsa-miR-892c-5p, hsa-miR-6734-5p, hsa-miR-4655-5p, hsa-miR-3945, hsa-miR-649, hsa-miR-892a, hsa-miR-7704, hsa-miR-3132, hsa-miR-3126-5p, hsa-miR-767-5p, hsa-miR-4697-5p, hsa-miR-510-5p, hsa-miR-1915-5p, hsa-miR-1267, hsa-miR-3928-5p, hsa-miR-491-3p, hsa-miR-5187-3p, hsa-miR-3934-3p, hsa-miR-656-5p, hsa-miR-1266-3p, hsa-miR-6772-3p, hsa-miR-3153, hsa-miR-4706, hsa-miR-6826-3p, hsa-miR-6753-5p, hsa-miR-342-3p, hsa-miR-4512, hsa-miR-6769a-5p, hsa-miR-670-5p, hsa-miR-4786-3p, hsa-miR-323a-3p, hsa-miR-6876-3p, hsa-miR-574-5p, hsa-miR-6856-3p, hsa-miR-6883-5p, hsa-miR-4448, hsa-miR-103b, hsa-miR-466, hsa-miR-1238-5p, hsa-miR-6782-3p, hsa-miR-3120-5p, hsa-miR-4639-3p, hsa-miR-500b-5p, hsa-miR-520d-3p, hsa-miR-1193, hsa-miR-6789-3p, hsa-miR-4740-3p, hsa-miR-6794-3p, hsa-miR-4483, hsa-miR-3151-5p, hsa-miR-6806-5p, hsa-miR-1288-3p, hsa-miR-422a, hsa-miR-7110-5p, hsa-miR-5694, hsa-miR-454-5p, hsa-miR-302d-5p, hsa-miR-4460, hsa-miR-198, hsa-miR-6866-3p, hsa-miR-4476, hsa-miR-3173-5p, hsa-miR-6846-5p, hsa-miR-185-5p, hsa-miR-631, hsa-miR-6775-5p, hsa-miR-875-3p, hsa-miR-6846-3p, hsa-miR-130b-3p, hsa-miR-6742-5p, hsa-miR-1247-3p, hsa-miR-12128, hsa-miR-611, hsa-miR-4664-3p, hsa-miR-204-3p, hsa-miR-6516-5p, hsa-miR-550b-3p, hsa-miR-619-5p, hsa-miR-6715b-5p, hsa-miR-891a-5p, hsa-miR-4712-5p, hsa-miR-12115, hsa-miR-6072, hsa-miR-6856-5p, hsa-miR-3152-5p, hsa-miR-4717-3p, hsa-miR-3064-5p, hsa-miR-6852-3p, hsa-miR-4539, hsa-miR-4260, hsa-miR-370-5p, hsa-miR-326, hsa-miR-508-5p, hsa-miR-744-3p, hsa-miR-1266-5p, hsa-miR-6868-3p, hsa-miR-6793-3p, hsa-miR-339-3p, hsa-miR-3150a-3p, hsa-miR-8052, hsa-miR-4676-3p, hsa-miR-3925-5p, hsa-miR-373-5p, hsa-miR-943, hsa-miR-5001-5p, hsa-miR-6816-3p, hsa-miR-4253, hsa-miR-1287-5p, hsa-miR-6827-5p, hsa-miR-6810-5p, hsa-miR-27b-3p, hsa-miR-3663-5p, hsa-miR-5587-3p, hsa-miR-6822-5p, hsa-miR-7152-5p, hsa-miR-4650-5p, hsa-miR-3191-3p, hsa-miR-2113, hsa-miR-3657, hsa-miR-1296-3p, hsa-miR-3126-3p, hsa-miR-892c-3p, hsa-miR-6754-3p, hsa-miR-3944-3p, hsa-miR-1182, hsa-miR-3177-3p, hsa-miR-6873-5p, hsa-miR-6510-5p, hsa-miR-6788-3p, hsa-miR-6764-5p, hsa-miR-517a-3p, hsa-miR-517b-3p, hsa-miR-6851-3p, hsa-miR-942-5p, hsa-miR-3189-3p, hsa-miR-9902, hsa-miR-208a-5p, hsa-miR-3164, hsa-miR-497-5p, hsa-miR-4731-5p, hsa-miR-491-5p, hsa-miR-572, hsa-miR-938, hsa-miR-1298-3p, hsa-miR-629-3p, hsa-miR-10394-3p, hsa-miR-8073, hsa-miR-6529-3p, hsa-miR-378i, hsa-miR-4485-3p, hsa-miR-1184, hsa-miR-1295b-3p, hsa-miR-1911-3p, hsa-miR-216a-3p, hsa-miR-581, hsa-miR-509-3p, hsa-miR-125a-3p, hsa-miR-1307-3p, hsa-miR-4773, hsa-miR-26a-1-3p, hsa-miR-4496, hsa-miR-5698, hsa-miR-3975, hsa-miR-3147, hsa-miR-4297, hsa-miR-11400, hsa-miR-1180-5p, hsa-miR-3622b-5p, hsa-miR-6853-5p, hsa-miR-6881-5p, hsa-miR-8059, hsa-miR-6778-5p, hsa-miR-12125, hsa-miR-5699-5p, hsa-miR-6716-5p, hsa-miR-16-1-3p, hsa-miR-6811-3p, hsa-miR-378a-5p, hsa-miR-5587-5p, hsa-miR-6894-5p, hsa-miR-4289, hsa-miR-3691-5p, hsa-miR-296-3p, hsa-miR-6134, hsa-miR-6084, hsa-miR-7155-3p, hsa-miR-6722-5p, hsa-miR-554, hsa-miR-539-5p, hsa-miR-6509-5p, hsa-miR-431-3p, hsa-miR-12136, hsa-miR-6744-5p, hsa-miR-6786-3p, hsa-miR-371a-5p, hsa-miR-4328, hsa-miR-301a-5p, hsa-miR-7845-5p, hsa-miR-557, hsa-miR-671-3p, hsa-miR-7855-5p, hsa-miR-937-3p, hsa-miR-1909-5p, hsa-miR-6514-3p, hsa-miR-8079, hsa-miR-6847-5p, hsa-miR-3681-3p, hsa-miR-4721, hsa-miR-6825-5p, hsa-miR-4485-5p, hsa-miR-4326, hsa-miR-302c-5p, hsa-miR-4781-5p, hsa-miR-1285-5p, hsa-miR-365b-5p, hsa-miR-4793-3p, hsa-miR-645, hsa-miR-3200-3p, hsa-miR-7846-3p, hsa-miR-4514, hsa-miR-4690-5p, hsa-miR-2115-5p, hsa-miR-7113-3p, hsa-miR-664b-5p, hsa-miR-6880-5p, hsa-miR-222-5p, hsa-miR-3679-5p, hsa-miR-487b-5p, hsa-miR-4529-3p, hsa-miR-4697-3p, hsa-miR-6801-3p, hsa-miR-4687-3p, hsa-miR-4710, hsa-miR-10527-5p, hsa-miR-486-5p, hsa-miR-186-3p, hsa-miR-4755-5p, hsa-miR-4797-3p, hsa-miR-5089-5p, hsa-miR-6857-3p, hsa-miR-7112-5p, hsa-miR-4324, hsa-miR-642b-5p, hsa-miR-4743-3p, hsa-miR-4709-5p, hsa-miR-4732-5p, hsa-miR-6731-5p, hsa-miR-4694-3p, hsa-miR-5188, hsa-miR-4788, hsa-miR-6851-5p, hsa-miR-518f-3p, hsa-miR-6511b-3p, hsa-miR-1250-5p, hsa-miR-7112-3p, hsa-miR-6754-5p, hsa-miR-873-3p, hsa-miR-128-2-5p, hsa-miR-515-3p, hsa-miR-10395-5p, hsa-miR-4778-5p, hsa-miR-6832-3p, hsa-miR-503-3p, hsa-miR-206, hsa-miR-4269, hsa-miR-4267, hsa-miR-6759-3p, hsa-miR-135a-5p, hsa-miR-1233-5p, hsa-miR-6875-3p, hsa-miR-3156-5p, hsa-miR-6803-5p, hsa-miR-4713-3p, hsa-miR-7155-5p, hsa-miR-500a-5p, hsa-miR-6857-5p, hsa-miR-4254, hsa-miR-7977, hsa-miR-4690-3p, hsa-miR-6799-5p, hsa-miR-431-5p, hsa-miR-7109-3p, hsa-miR-3650, hsa-miR-656-3p, hsa-miR-514b-3p, hsa-miR-6828-5p, hsa-miR-4724-5p, hsa-miR-4804-3p, hsa-miR-4478, hsa-miR-520f-5p, hsa-miR-10400-5p, hsa-miR-6863, hsa-miR-361-5p, hsa-miR-512-5p, hsa-miR-3973, hsa-miR-6769b-3p, hsa-miR-603, hsa-miR-1288-5p, hsa-miR-6787-3p, hsa-miR-4632-5p, hsa-miR-4689, hsa-miR-4266, hsa-miR-134-5p, hsa-miR-6867-5p, hsa-miR-1271-5p, hsa-miR-3678-3p, hsa-miR-589-5p, hsa-miR-615-3p, hsa-miR-133b, hsa-miR-6783-3p, hsa-miR-6133, hsa-miR-6742-3p, hsa-miR-3127-5p, hsa-miR-9500, hsa-miR-3615, hsa-miR-8087, hsa-miR-654-3p, hsa-miR-6840-5p, hsa-miR-661, hsa-miR-889-5p, hsa-miR-3188, hsa-miR-6792-3p, hsa-miR-5681a, hsa-miR-3198, hsa-miR-1270, hsa-miR-10398-5p, hsa-miR-8060, hsa-miR-3065-3p, hsa-miR-3622a-3p, hsa-miR-1275, hsa-miR-3116, hsa-miR-4312, hsa-miR-1229-5p, hsa-miR-6893-3p, hsa-miR-4470, hsa-miR-412-3p, hsa-miR-3064-3p, hsa-miR-12126, hsa-miR-155-3p, hsa-miR-34a-5p, hsa-miR-4737, hsa-miR-4725-5p, hsa-miR-3613-3p, hsa-miR-1255b-2-3p, hsa-miR-4714-5p, hsa-miR-3189-5p, hsa-miR-7843-5p, hsa-miR-29c-5p, hsa-miR-6769b-5p, hsa-miR-3929, hsa-miR-6504-5p, hsa-miR-4443, hsa-miR-3960, hsa-miR-6890-3p, hsa-miR-4786-5p, hsa-miR-2278, hsa-miR-514b~5p, hsa-miR-4644, hsa-miR-6757-3p, hsa-miR-10401-3p, hsa-miR-4318, hsa-miR-3675-5p, hsa-miR-4733-5p, hsa-miR-32-3p, hsa-miR-3714, hsa-miR-4292, hsa-miR-6771-3p, hsa-miR-5001-3p, hsa-miR-4271, hsa-miR-6739-3p, hsa-miR-3130-5p, hsa-miR-30d-5p, hsa-miR-6781-3p, hsa-miR-7702, hsa-miR-885-5p, hsa-miR-499b-3p, hsa-miR-4711-3p, hsa-miR-125b-5p, hsa-miR-5580-3p, hsa-miR-1291, hsa-miR-181a-5p, hsa-miR-4423-5p, hsa-miR-3919, hsa-miR-7974, hsa-miR-4524a-5p, hsa-miR-936, hsa-miR-6715a-3p, hsa-miR-6883-3p, hsa-miR-520g-3p, hsa-miR-5007-5p, hsa-miR-6510-3p, hsa-miR-3649, hsa-miR-3125, hsa-miR-6744-3p, hsa-miR-635, hsa-miR-8485, hsa-miR-4751, hsa-miR-3913-3p, hsa-miR-5195-5p, hsa-miR-8055, hsa-miR-10394-5p, hsa-miR-4518, hsa-miR-766-3p, hsa-miR-6878-3p, hsa-miR-4794, hsa-miR-4709-3p, hsa-let-7c-3p, hsa-miR-634, hsa-miR-1226-3p, hsa-miR-5588-5p, hsa-miR-30b-5p, hsa-miR-642b-3p, hsa-miR-3165, hsa-miR-93-5p, hsa-miR-154-3p, hsa-miR-627-5p, hsa-miR-1250-3p, hsa-miR-151b, hsa-miR-6736-3p, hsa-miR-4281, hsa-miR-29b-3p, hsa-miR-2355-5p, hsa-miR-23a-5p, hsa-miR-3158-5p, hsa-miR-526b-5p, hsa-miR-2392, hsa-miR-668-3p, hsa-miR-1292-3p, hsa-miR-519d-3p, hsa-miR-4278, hsa-miR-6815-3p, hsa-miR-657, hsa-miR-30c-1-3p, hsa-miR-4718, hsa-miR-4436b-5p, hsa-miR-7111-3p, hsa-miR-1263, hsa-miR-7161-5p, hsa-miR-6858-5p, hsa-miR-1207-3p, hsa-miR-1249-5p, hsa-miR-6827-3p, hsa-miR-6757-5p, hsa-miR-203a-3p, hsa-miR-6768-3p, hsa-let-7f-2-3p, hsa-miR-6882-3p, hsa-miR-6735-3p, hsa-miR-758-5p, hsa-miR-3202, hsa-miR-6833-3p, hsa-miR-6769a-3p, hsa-miR-6830-3p, hsa-miR-6888-5p, hsa-miR-7157-5p, hsa-let-7i-5p, hsa-miR-6765-3p, hsa-miR-591, hsa-miR-4700-3p, hsa-miR-6868-5p, hsa-miR-6509-3p, hsa-miR-223-3p, hsa-miR-4641, hsa-miR-502-5p, hsa-miR-5008-5p, hsa-miR-5010-3p, hsa-miR-605-5p, hsa-miR-489-3p, hsa-miR-3667-3p, hsa-miR-6719-3p, hsa-miR-6508-5p, hsa-miR-5702, hsa-miR-4701-5p, hsa-miR-6870-5p, hsa-miR-4756-5p, hsa-miR-6842-3p, and hsa-miR-548d-3p. In the present disclosure, the extract of urine may be an extract of the urine of a lymphoma patient. Only one of these microRNAs can be used to predict whether a subject from which the urine is derived is a lymphoma patient, and 5 or more types, 10 or more types, 15 or more types, or 20 or more types of these microRNAs can be used in combination to predict whether a subject from which the urine is derived is a lymphoma patient. Among these microRNAs, a microRNA that may be highly expressed in a lymphoma patient can be preferably used for prediction.

The present disclosure provides an extract of urine containing at least one type, two types, three types, four types, five types, six types, seven types, eight types, nine types, or ten types of or all microRNAs selected from the group consisting of hsa-miR-29a-3p, hsa-miR-29b-3p, hsa-miR-140-5p, hsa-miR-106b-5p, hsa-miR-299-3p, hsa-miR-20b-5p, hsa-miR-448, hsa-miR-492, hsa-miR-498-5p, hsa-miR-520d-3p, hsa-miR-516b-3p, hsa-miR-516a-3p, hsa-miR-502-5p, hsa-miR-628-3p, hsa-miR-639, hsa-miR-769-5p, hsa-miR-22-5p, hsa-miR-27a-5p, hsa-miR-124-5p, hsa-miR-150-3p, hsa-miR-155-3p, hsa-miR-551b-5p, hsa-miR-629-5p, hsa-miR-921, hsa-miR-1226-5p, hsa-miR-513c-5p, hsa-miR-320c, hsa-miR-1179, hsa-miR-1286, hsa-miR-1321, hsa-miR-1470, hsa-miR-1915-3p, hsa-miR-3124-5p, hsa-miR-3157-5p, hsa-miR-3166, hsa-miR-3178, hsa-miR-320e, hsa-miR-3199, hsa-miR-4295, hsa-miR-4308, hsa-miR-4315, hsa-miR-4321, hsa-miR-4255, hsa-miR-4327, hsa-miR-3651, hsa-miR-3660, hsa-miR-3665, hsa-miR-3666, hsa-miR-3677-3p, hsa-miR-3918, hsa-miR-3937, hsa-miR-4460, hsa-miR-4464, hsa-miR-4512, hsa-miR-4536-5p, hsa-miR-4635, hsa-miR-4672, hsa-miR-4673, hsa-miR-4674, hsa-miR-4724-5p, hsa-miR-4724-3p, hsa-miR-4727-3p, hsa-miR-5188, hsa-miR-5579-5p, hsa-miR-5579-3p, hsa-miR-664b-5p, hsa-miR-5588-5p, hsa-miR-381-5p, hsa-miR-6074, hsa-miR-6081, hsa-miR-6082, hsa-miR-6499-5p, hsa-miR-6501-5p, hsa-miR-605-3p, hsa-miR-6770-5p, hsa-miR-6789-5p, hsa-miR-6814-5p, hsa-miR-6836-5p, hsa-miR-6836-3p, hsa-miR-6850-5p, hsa-miR-7108-3p, hsa-miR-135a-2-3p, hsa-miR-9898, hsa-miR-9985, hsa-miR-10392-3p, hsa-miR-10522-5p, hsa-miR-12117, hsa-miR-12120, and hsa-miR-12122. In the present disclosure, the extract of urine may be an extract of the urine of a stage-I or -II lymphoma patient. Only one of these microRNAs can be used to predict whether a subject from which the urine is derived is a lymphoma patient (particularly stage-I or -II), and 5 or more types, 10 or more types, 15 or more types, or 20 or more types of these microRNAs can be used in combination to predict whether a subject from which the urine is derived is a lymphoma patient (particularly stage-I or -II). Among these microRNAs, a microRNA that may be highly expressed in a lymphoma patient (particularly stage-I or -II) can be preferably used for prediction.

The present disclosure provides an extract of urine containing at least one type, two types, three types, four types, five types, six types, seven types, eight types, nine types, or ten types of or all microRNAs selected from the group consisting of microRNAs having an accuracy of higher than 0.500 among the microRNAs listed in Table 24-5. In the present disclosure, the extract of urine may be an extract of the urine of a lymphoma patient. Only one of these microRNAs can be used to predict whether a subject from which the urine is derived is a lymphoma patient, and 5 or more types, 10 or more types, 15 or more types, or 20 or more types of these microRNAs can be used in combination to predict whether a subject from which the urine is derived is a lymphoma patient. Among these microRNAs, a microRNA that may be highly expressed in a lymphoma patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

The present disclosure provides an extract of urine containing at least one type, two types, three types, four types, five types, six types, seven types, eight types, nine types, or ten types of or all microRNAs selected from the group consisting of microRNAs having an accuracy of higher than 0.500 among the microRNAs listed in Table 25-5. In the present disclosure, the extract of urine may be an extract of the urine of a lymphoma patient. Only one of these microRNAs can be used to predict whether a subject from which the urine is derived is a lymphoma patient, and 5 or more types, 10 or more types, 15 or more types, or 20 or more types of these microRNAs can be used in combination to predict whether a subject from which the urine is derived is a lymphoma patient. Among these microRNAs, a microRNA that may be highly expressed in a lymphoma patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

The present disclosure provides an extract of urine containing at least one type, two types, three types, four types, five types, six types, seven types, eight types, nine types, or ten types of or all microRNAs selected from the microRNAs listed in Table 4-6. The present disclosure also provides an extract of urine containing at least one type, two types, three types, four types, five types, six types, seven types, eight types, nine types, or ten types of or all microRNAs selected from the group consisting of hsa-miR-4280, hsa-miR-6783-5p, hsa-miR-4755-3p, hsa-miR-6809-5p, hsa-miR-605-3p, hsa-miR-4458, hsa-miR-6074, hsa-miR-135a-2-3p, hsa-miR-601, hsa-miR-4727-3p, hsa-miR-498-5p, hsa-miR-5007-5p, hsa-miR-1207-5p, hsa-miR-338-5p, hsa-miR-6514-5p, hsa-miR-766-5p, hsa-miR-617, hsa-miR-320c, hsa-miR-921, hsa-miR-3192-5p, hsa-miR-12122, hsa-miR-4657, hsa-miR-5093, hsa-miR-9986, hsa-miR-8062, hsa-miR-6817-5p, hsa-miR-10392-3p, hsa-miR-10526-3p, hsa-miR-7150, hsa-miR-34c-5p, hsa-miR-6516-5p, hsa-miR-6501-3p, hsa-miR-4433b-3p, hsa-miR-6515-5p, hsa-miR-1914-3p, hsa-miR-6832-5p, hsa-miR-3059-3p, hsa-miR-193b-5p, hsa-miR-1289, hsa-miR-6804-5p, hsa-miR-541-3p, hsa-miR-4451, hsa-miR-4300, hsa-miR-6761-5p, hsa-miR-6794-5p, hsa-miR-202-3p, hsa-miR-6500-3p, hsa-miR-6750-5p, hsa-miR-3155a, hsa-miR-7515, hsa-miR-6836-3p, hsa-miR-20b-3p, hsa-miR-3124-5p, hsa-miR-5187-5p, hsa-miR-550b-2-5p, hsa-let-7b-5p, hsa-miR-8074, hsa-miR-320e, hsa-miR-497-5p, hsa-miR-3691-5p, hsa-miR-6845-3p, hsa-miR-3161, hsa-miR-656-5p, hsa-miR-516a-5p, hsa-miR-6129, hsa-miR-4321, hsa-miR-1288-3p, hsa-miR-6501-5p, hsa-miR-6746-5p, hsa-miR-125b-2-3p, hsa-miR-4506, hsa-miR-8082, hsa-miR-3654, hsa-miR-7854-3p, hsa-miR-4785, hsa-miR-147a, hsa-miR-1293, hsa-miR-6823-5p, hsa-miR-325, hsa-miR-6838-5p, hsa-miR-106a-3p, hsa-miR-5192, hsa-miR-4658, hsa-miR-516b-3p, hsa-miR-516a-3p, hsa-miR-8070, hsa-miR-4741, hsa-miR-1470, hsa-miR-7154-3p, hsa-miR-4317, hsa-miR-12127, hsa-miR-4784, hsa-miR-3918, hsa-miR-668-5p, hsa-miR-6130, hsa-miR-4437, hsa-miR-1224-3p, hsa-miR-4665-5p, hsa-miR-1236-3p, hsa-miR-1281, hsa-miR-4654, hsa-miR-3937, hsa-miR-4433a-3p, hsa-miR-525-5p, hsa-miR-4644, hsa-miR-138-5p, hsa-miR-6781-5p, hsa-miR-6747-5p, hsa-miR-4314, hsa-miR-5703, hsa-miR-4327, hsa-miR-4635, hsa-miR-193a-5p, hsa-miR-25-5p, hsa-miR-433-5p, hsa-miR-4754, hsa-miR-4450, hsa-miR-4758-5p, hsa-miR-708-5p, hsa-miR-494-3p, hsa-miR-5091, hsa-miR-6789-5p, hsa-miR-610, hsa-miR-579-5p, hsa-miR-4447, hsa-miR-892c-5p, hsa-miR-1292-5p, hsa-miR-7111-5p, hsa-miR-3186-3p, hsa-miR-4296, hsa-miR-650, hsa-miR-200b-5p, hsa-miR-6891-3p, hsa-miR-4529-5p, hsa-miR-7851-3p, hsa-miR-4436b-3p, hsa-miR-4299, hsa-miR-1228-3p, hsa-miR-6796-3p, hsa-miR-4257, hsa-miR-135a-3p, hsa-miR-105-5p, hsa-miR-10400-3p, hsa-miR-4431, hsa-miR-6784-3p, hsa-miR-3125, hsa-miR-550a-3-5p, hsa-miR-6506-5p, hsa-miR-206, hsa-miR-4520-3p, hsa-miR-3619-5p, hsa-miR-1321, hsa-miR-892c-3p, hsa-miR-6788-5p, hsa-miR-493-3p, hsa-miR-3934-3p, hsa-miR-6829-5p, hsa-miR-1286, hsa-miR-4773, hsa-miR-5188, hsa-miR-8057, hsa-miR-6125, hsa-miR-6855-5p, hsa-miR-10395-5p, hsa-miR-5006-5p, hsa-miR-4433b-5p, hsa-miR-3197, hsa-miR-6880-3p, hsa-miR-4439, hsa-miR-1249-3p, hsa-miR-584-5p, hsa-miR-7151-3p, hsa-miR-3934-5p, hsa-miR-34a-5p, hsa-miR-1238-3p, hsa-miR-4651, hsa-miR-1270, hsa-miR-4273, hsa-miR-4311, hsa-miR-942-5p, hsa-miR-6892-3p, hsa-miR-6810-3p, hsa-miR-6743-5p, hsa-miR-4510, hsa-miR-7107-5p, hsa-miR-4502, hsa-miR-3199, hsa-miR-3689b-3p, hsa-miR-3689c, hsa-miR-1910-3p, hsa-miR-6760-3p, hsa-miR-1237-3p, hsa-miR-6515-3p, hsa-miR-3150b-3p, hsa-miR-7114-5p, hsa-miR-7114-3p, hsa-miR-4692, hsa-miR-1268b, hsa-miR-4433a-5p, hsa-miR-5190, hsa-miR-5196-5p, hsa-miR-10522-5p, hsa-miR-410-5p, hsa-miR-512-3p, hsa-miR-4429, hsa-miR-184, hsa-miR-6715b-5p, hsa-miR-1343-5p, hsa-miR-6805-3p, hsa-miR-6798-5p, hsa-miR-3605-5p, hsa-miR-758-5p, hsa-miR-4665-3p, hsa-miR-4687-5p, hsa-miR-8083, hsa-miR-7850-5p, hsa-miR-3648, hsa-miR-4686, hsa-miR-4474-3p, hsa-miR-4716-5p, hsa-miR-323b-5p, hsa-miR-4283, hsa-miR-8072, hsa-miR-5580-5p, hsa-miR-8077, hsa-miR-299-3p, hsa-miR-7108-3p, hsa-miR-6813-3p, hsa-miR-6756-3p, hsa-miR-4706, hsa-miR-4648, hsa-miR-6086, hsa-miR-200b-3p, hsa-miR-4323, hsa-miR-7162-3p, hsa-miR-6790-3p, hsa-miR-4423-5p, hsa-miR-6717-5p, hsa-miR-4647, hsa-miR-6777-3p, hsa-miR-150-3p, hsa-miR-6791-5p, hsa-miR-6731-3p, hsa-miR-382-5p, hsa-miR-6815-5p, hsa-miR-6763-3p, hsa-miR-4538, hsa-miR-6509-5p, hsa-miR-3162-3p, hsa-miR-3184-3p, hsa-miR-767-5p, hsa-miR-4694-3p, hsa-miR-4524b-3p, hsa-miR-2115-5p, hsa-miR-6858-3p, hsa-miR-221-3p, hsa-miR-6800-3p, hsa-miR-3186-5p, hsa-miR-525-3p, hsa-miR-6884-3p, hsa-miR-6847-5p, hsa-miR-12116, hsa-miR-3141, hsa-miR-589-3p, hsa-miR-4436a, hsa-miR-4737, hsa-miR-3148, hsa-miR-4453, hsa-miR-6529-5p, hsa-miR-3130-3p, hsa-miR-8064, hsa-miR-1304-5p, hsa-miR-6749-3p, hsa-miR-6775-3p, hsa-miR-7856-5p, hsa-miR-1304-3p, hsa-miR-10401-5p, hsa-miR-1225-3p, hsa-miR-4274, hsa-miR-3200-5p, hsa-miR-34b-3p, hsa-miR-593-3p, hsa-miR-1250-5p, hsa-miR-4713-3p, hsa-miR-4763-3p, hsa-miR-6719-3p, hsa-miR-1236-5p, hsa-miR-6798-3p, hsa-miR-6850-5p, hsa-miR-4749-3p, hsa-miR-378b, hsa-miR-1322, hsa-miR-4491, hsa-miR-515-3p, hsa-miR-6785-3p, hsa-miR-548g-3p, hsa-miR-4261, hsa-miR-6764-5p, hsa-miR-4435, hsa-miR-12131, hsa-miR-3187-3p, hsa-miR-6887-3p, hsa-miR-3944-5p, hsa-miR-3679-3p, hsa-miR-6729-3p, hsa-miR-769-3p, hsa-miR-4673, hsa-miR-6884-5p, hsa-miR-6165, hsa-miR-638, hsa-miR-6871-5p, hsa-miR-29c-5p, hsa-miR-6812-3p, hsa-miR-6729-5p, hsa-miR-3922-5p, hsa-miR-222-5p, hsa-miR-7113-5p, hsa-miR-4768-3p, hsa-miR-34b-5p, hsa-miR-6763-5p, hsa-miR-6861-3p, hsa-miR-4465, hsa-miR-29a-3p, hsa-miR-6859-5p, hsa-miR-4728-3p, hsa-miR-210-5p, hsa-miR-3919, hsa-miR-498-3p, hsa-miR-487a-5p, hsa-miR-595, hsa-miR-4701-3p, hsa-miR-6853-5p, hsa-miR-8075, hsa-miR-18b-3p, hsa-miR-5685, hsa-miR-6076, hsa-miR-6134, hsa-miR-147b-3p, hsa-miR-4258, hsa-miR-297, hsa-miR-6893-5p, hsa-miR-378g, hsa-miR-30b-3p, hsa-miR-9898, hsa-miR-1306-3p, hsa-miR-519c-5p, hsa-miR-523-5p, hsa-miR-518e-5p, hsa-miR-522-5p, hsa-miR-519a-5p, hsa-miR-519b-5p, hsa-miR-449b-5p, hsa-miR-3622a-5p, hsa-miR-12117, hsa-miR-4670-5p, hsa-miR-6767-5p, hsa-miR-3137, hsa-miR-1294, hsa-miR-6797-3p, hsa-miR-4466, hsa-miR-1265, hsa-miR-5004-5p, hsa-miR-6797-5p, hsa-miR-4520-5p, hsa-miR-6859-3p, hsa-miR-604, hsa-miR-517a-3p, hsa-miR-517b-3p, hsa-miR-6776-5p, hsa-miR-6801-5p, hsa-miR-629-5p, hsa-miR-6749-5p, hsa-miR-4676-5p, hsa-miR-6821-5p, hsa-miR-513c-5p, hsa-miR-515-5p, hsa-miR-4708-3p, hsa-miR-891a-5p, hsa-miR-200a-5p, hsa-miR-5588-3p, hsa-miR-4534, hsa-miR-4445-3p, hsa-miR-328-3p, hsa-miR-4711-3p, hsa-miR-6503-3p, hsa-miR-6895-5p, hsa-miR-6081, hsa-miR-1251-3p, hsa-miR-1908-5p, hsa-miR-5708, hsa-miR-6732-5p, hsa-miR-432-5p, hsa-miR-4489, hsa-miR-6793-5p, hsa-miR-888-3p, hsa-miR-4745-5p, hsa-miR-1913, hsa-miR-506-3p, hsa-miR-134-3p, hsa-miR-4303, hsa-miR-24-2-5p, hsa-miR-11399, hsa-miR-3927-5p, hsa-miR-6777-5p, hsa-miR-6839-5p, hsa-miR-484, hsa-miR-7157-3p, hsa-miR-323a-5p, hsa-miR-4660, hsa-miR-4749-5p, hsa-miR-146b-3p, hsa-miR-6876-5p, hsa-miR-2467-3p, hsa-miR-9718, hsa-miR-7844-5p, hsa-miR-761, hsa-miR-6805-5p, hsa-miR-3652, hsa-miR-2682-5p, hsa-miR-3190-3p, hsa-miR-584-3p, hsa-miR-4682, hsa-miR-219a-2-3p, hsa-miR-10396b-5p, hsa-miR-6131, hsa-miR-4753-5p, hsa-miR-5194, hsa-miR-889-5p, hsa-miR-4499, hsa-miR-1226-5p, hsa-miR-4540, hsa-miR-608, hsa-miR-5189-5p, hsa-miR-6500-5p, hsa-miR-323a-3p, hsa-miR-551b-5p, hsa-miR-6790-5p, hsa-miR-4487, hsa-miR-3122, hsa-miR-4769-3p, hsa-miR-3651, hsa-miR-4769-5p, hsa-miR-3689a-3p, hsa-miR-6752-5p, hsa-miR-1288-5p, hsa-miR-6819-3p, hsa-miR-5585-3p, hsa-miR-885-3p, hsa-miR-4750-3p, hsa-miR-1185-2-3p, hsa-miR-7976, hsa-miR-718, hsa-miR-6738-5p, hsa-miR-6775-5p, hsa-miR-4632-5p, hsa-miR-1303, hsa-miR-6818-3p, hsa-miR-3142, hsa-miR-6892-5p, hsa-miR-1227-5p, hsa-miR-514b-3p, hsa-miR-4794, hsa-miR-92a-2-5p, hsa-miR-6766-3p, hsa-miR-214-5p, hsa-miR-138-2-3p, hsa-miR-6795-3p, hsa-miR-335-3p, hsa-miR-1471, hsa-miR-4535, hsa-miR-378c, hsa-miR-4514, hsa-miR-6083, hsa-miR-625-3p, hsa-miR-1291, hsa-miR-4507, hsa-miR-7157-5p, hsa-miR-216a-3p, hsa-miR-3675-3p, hsa-miR-6845-5p, hsa-miR-4776-3p, hsa-miR-371b-5p, hsa-miR-6089, hsa-miR-181b-5p, hsa-miR-4308, hsa-miR-6070, hsa-miR-940, hsa-miR-6795-5p, hsa-miR-4472, hsa-miR-501-5p, hsa-miR-6715a-3p, hsa-miR-5000-5p, hsa-miR-4725-3p, hsa-miR-3680-3p, hsa-miR-3085-3p, hsa-miR-645, hsa-miR-6772-5p, hsa-miR-4498, hsa-miR-4734, hsa-miR-4723-3p, hsa-miR-6870-3p, hsa-miR-6760-5p, hsa-miR-12120, hsa-miR-2276-3p, hsa-miR-99b-3p, hsa-miR-4649-3p, hsa-miR-1301-5p, hsa-miR-149-3p, hsa-miR-194-3p, hsa-miR-4700-5p, hsa-miR-575, hsa-miR-4298, hsa-miR-3150b-5p, hsa-miR-4294, hsa-miR-4731-3p, hsa-miR-4428, hsa-miR-370-3p, hsa-miR-6849-5p, hsa-miR-3713, hsa-miR-3689f, hsa-miR-6068, hsa-miR-3620-5p, hsa-miR-6740-5p, hsa-miR-12114, hsa-miR-10392-5p, hsa-miR-6887-5p, hsa-miR-3666, hsa-miR-1276, hsa-miR-6814-5p, hsa-miR-665, hsa-miR-103a-1-5p, hsa-miR-4999-5p, hsa-miR-4525, hsa-miR-4756-3p, hsa-miR-5089-3p, hsa-miR-338-3p, hsa-miR-1225-5p, hsa-miR-4691-5p, hsa-miR-585-3p, hsa-miR-489-5p, hsa-miR-5739, hsa-miR-492, hsa-miR-330-5p, hsa-miR-32-3p, hsa-miR-6848-3p, hsa-miR-4684-5p, hsa-miR-3660, hsa-miR-4505, hsa-miR-4740-5p, hsa-miR-6513-5p, hsa-miR-514b-5p, hsa-miR-4313, hsa-miR-6090, hsa-miR-329-5p, hsa-miR-6812-5p, hsa-miR-6752-3p, hsa-miR-378i, hsa-miR-3065-3p, hsa-miR-4421, hsa-miR-378a-3p, hsa-miR-564, hsa-miR-4695-5p, hsa-miR-1185-1-3p, hsa-miR-7159-5p, hsa-miR-518b, hsa-miR-3180-3p, hsa-miR-10523-5p, hsa-miR-542-5p, hsa-miR-6868-5p, hsa-miR-12119, hsa-miR-8080, hsa-let-7d-3p, hsa-miR-4255, hsa-miR-504-5p, hsa-miR-711, hsa-miR-4783-3p, hsa-miR-8071, hsa-miR-6726-5p, hsa-miR-1587, hsa-miR-2909, hsa-miR-4470, hsa-miR-4708-5p, hsa-miR-3675-5p, hsa-miR-7158-5p, hsa-miR-4289, hsa-miR-6822-3p, hsa-miR-8063, hsa-miR-6843-3p, hsa-miR-4778-5p, hsa-miR-8055, hsa-miR-7106-5p, hsa-miR-4252, hsa-miR-4672, hsa-miR-93-5p, hsa-miR-6080, hsa-miR-1287-5p, hsa-miR-4763-5p, hsa-miR-7848-3p, hsa-miR-197-5p, hsa-miR-4638-5p, hsa-miR-5006-3p, hsa-miR-4316, hsa-miR-4467, hsa-miR-92b-5p, hsa-miR-7160-5p, hsa-miR-877-5p, hsa-miR-138-1-3p, hsa-miR-4530, hsa-miR-6504-3p, hsa-miR-11401, hsa-miR-6787-5p, hsa-miR-6730-5p, hsa-miR-4456, hsa-miR-185-3p, hsa-miR-7156-3p, hsa-miR-4516, hsa-miR-6796-5p, hsa-miR-770-5p, hsa-miR-6865-3p, hsa-miR-5187-3p, hsa-miR-3187-5p, hsa-miR-1204, hsa-miR-6511a-5p, hsa-miR-6739-3p, hsa-miR-4724-3p, hsa-miR-6127, hsa-miR-340-3p, hsa-miR-939-5p, hsa-miR-500b-3p, hsa-miR-6834-5p, hsa-miR-6718-5p, hsa-miR-3913-3p, hsa-miR-4736, hsa-miR-6861-5p, hsa-miR-6756-5p, hsa-miR-7847-3p, hsa-miR-200c-5p, hsa-miR-6721-5p, hsa-miR-4684-3p, hsa-miR-504-3p, hsa-miR-5680, hsa-miR-4481, hsa-miR-6889-5p, hsa-miR-18a-3p, hsa-miR-7151-5p, hsa-miR-8078, hsa-miR-4497, hsa-miR-5010-5p, hsa-miR-6879-5p, hsa-miR-3155b, hsa-miR-3621, hsa-miR-3158-3p, hsa-miR-3170, hsa-miR-1234-3p, hsa-miR-6504-5p, hsa-miR-6837-5p, hsa-miR-421, hsa-miR-4482-3p, hsa-miR-892b, hsa-miR-3127-5p, hsa-miR-3682-3p, hsa-miR-4284, hsa-miR-6794-3p, hsa-miR-4688, hsa-miR-3150a-5p, hsa-miR-5580-3p, hsa-miR-4797-3p, hsa-miR-4420, hsa-miR-6820-5p, hsa-miR-4738-3p, hsa-miR-1178-3p, hsa-miR-6069, hsa-miR-373-3p, hsa-miR-6840-3p, hsa-miR-10396a-3p, hsa-miR-296-5p, hsa-miR-6833-5p, hsa-miR-6784-5p, hsa-miR-4786-3p, hsa-miR-762 , hsa-miR-3196, hsa-miR-4286, hsa-miR-4454, hsa-miR-4265, hsa-miR-3173-3p, hsa-miR-6734-3p, hsa-miR-3131, hsa-miR-644a, hsa-miR-181a-2-3p, hsa-miR-3064-5p, hsa-miR-10396a-5p, hsa-miR-3619-3p, hsa-miR-3936, hsa-miR-4486, hsa-miR-1237-5p, hsa-miR-365a-5p, hsa-miR-6848-5p, hsa-miR-23b-3p, hsa-miR-7155-3p, hsa-miR-196b-5p, hsa-miR-3929, hsa-miR-654-5p, hsa-miR-4709-5p, hsa-miR-3135b, hsa-miR-6745, hsa-miR-7108-5p, hsa-miR-6722-3p, hsa-miR-210-3p, hsa-miR-4295, hsa-miR-4484, hsa-miR-5087, hsa-miR-429, hsa-miR-5002-3p, hsa-miR-5584-3p, hsa-miR-3127-3p, hsa-miR-302a-3p, hsa-miR-5189-3p, hsa-miR-6788-3p, hsa-miR-211-3p, hsa-miR-942-3p, hsa-miR-6876-3p, hsa-miR-1469, hsa-miR-3149, hsa-miR-652-5p, hsa-miR-34c-3p, hsa-miR-1268a, hsa-miR-4801, hsa-miR-6842-3p, hsa-miR-6724-5p, hsa-miR-139-3p, hsa-miR-4707-5p, hsa-miR-5690, hsa-miR-1468-5p, hsa-miR-3617-3p, hsa-miR-6751-5p, hsa-miR-6764-3p, hsa-miR-3185, hsa-miR-3945, hsa-miR-361-3p, hsa-miR-1915-3p, hsa-miR-486-3p, hsa-miR-1298-3p, hsa-miR-224-3p, hsa-miR-4440, hsa-miR-5002-5p, hsa-miR-6075, hsa-miR-6854-3p, hsa-miR-5702, hsa-miR-25-3p, hsa-miR-3074-3p, hsa-miR-3917, hsa-miR-7845-5p, hsa-miR-6782-5p, hsa-miR-3681-3p, hsa-miR-3180-5p, hsa-miR-204-3p, hsa-miR-6512-3p, hsa-miR-6863, hsa-miR-4529-3p, hsa-miR-7706, hsa-miR-3685, hsa-miR-6085, hsa-miR-541-5p, hsa-miR-3178, hsa-miR-3655, hsa-miR-6759-5p, hsa-miR-6821-3p, hsa-miR-449c-5p, hsa-miR-6786-5p, hsa-miR-4444, hsa-miR-103a-2-5p, hsa-miR-326, hsa-miR-6511b-5p, hsa-miR-187-5p, hsa-miR-4746-3p, hsa-miR-3614-5p, hsa-miR-485-5p, hsa-miR-6124, hsa-miR-4720-5p, hsa-miR-5589-5p, hsa-miR-4508, hsa-miR-450a-5p, hsa-miR-6499-3p, hsa-miR-4473, hsa-miR-4747-5p, hsa-miR-141-5p, hsa-miR-1285-3p, hsa-miR-422a, hsa-miR-4446-3p, hsa-miR-6762-3p, hsa-miR-5572, hsa-miR-4800-5p, hsa-miR-509-3p, hsa-miR-4262, hsa-miR-6792-3p, hsa-miR-6868-3p, hsa-miR-4690-5p, hsa-miR-1843, hsa-miR-31-3p, hsa-miR-12118, hsa-miR-6505-3p, hsa-miR-1909-3p, hsa-miR-6792-5p, hsa-miR-510-5p, hsa-miR-6768-5p, hsa-miR-3180, hsa-miR-6738-3p, hsa-miR-6737-5p, hsa-miR-647, hsa-miR-612, hsa-miR-4722-5p, hsa-miR-622, hsa-miR-8081, hsa-let-7e-5p, hsa-miR-5581-5p, hsa-miR-300, hsa-miR-6886-5p, hsa-miR-4664-5p, hsa-miR-487a-3p, hsa-miR-203a-3p, hsa-miR-4776-5p, hsa-miR-4496, hsa-miR-3943, hsa-miR-8079, hsa-miR-1912-3p, hsa-miR-26b-3p, hsa-miR-6773-3p, hsa-miR-5009-3p, hsa-miR-8089, hsa-miR-554, hsa-miR-4669, hsa-miR-5100, hsa-miR-4743-5p, hsa-miR-3176, hsa-miR-7109-5p, hsa-miR-513b-5p, hsa-miR-3909, hsa-miR-5684, hsa-miR-129-5p, hsa-miR-6755-3p, hsa-miR-4634, hsa-miR-5090, hsa-miR-1271-5p, hsa-miR-1298-5p, hsa-miR-5698, hsa-miR-550a-5p, hsa-miR-4717-5p, hsa-miR-4728-5p, hsa-miR-342-5p, hsa-miR-585-5p, hsa-miR-4425, hsa-miR-660-3p, hsa-miR-181d-3p, hsa-miR-4430, hsa-miR-3162-5p, hsa-miR-151a-3p, hsa-miR-324-5p, hsa-miR-494-5p, hsa-miR-664b-5p, hsa-miR-3667-5p, hsa-miR-187-3p, hsa-miR-411-3p, hsa-miR-10398-3p, hsa-miR-365a-3p, hsa-miR-365b-3p, hsa-miR-4750-5p, hsa-miR-6846-5p, hsa-miR-4653-5p, hsa-miR-214-3p, hsa-miR-4455, hsa-miR-23a-5p, hsa-miR-4748, hsa-miR-7111-3p, hsa-miR-373-5p, hsa-miR-4645-3p, hsa-miR-219b-5p, hsa-miR-466, hsa-miR-1224-5p, hsa-let-7i-5p, hsa-miR-3646, hsa-miR-6753-5p, hsa-miR-3126-3p, hsa-miR-649, hsa-miR-12125, hsa-miR-936, hsa-miR-6529-3p, hsa-miR-342-3p, hsa-miR-877-3p, hsa-miR-6808-3p, hsa-miR-122b-3p, hsa-miR-943, hsa-miR-6860, hsa-miR-1266-3p, hsa-miR-4322, hsa-miR-3138, hsa-miR-4650-3p, hsa-miR-1972, hsa-miR-6842-5p, hsa-miR-769-5p, hsa-miR-4655-5p, hsa-miR-4780, hsa-miR-4463, hsa-miR-8069, hsa-miR-5088-5p, hsa-miR-5004-3p, hsa-miR-4639-3p, hsa-miR-2110, hsa-miR-3650, hsa-miR-635, hsa-miR-874-5p, hsa-miR-6885-3p, hsa-miR-4276, hsa-miR-6766-5p, hsa-miR-6791-3p, hsa-miR-11181-3p, hsa-miR-3169, hsa-miR-4674, hsa-miR-614, hsa-miR-3173-5p, hsa-miR-3928-5p, hsa-miR-6741-5p, hsa-miR-2681-3p, hsa-miR-4740-3p, hsa-miR-875-3p, hsa-miR-4449, hsa-miR-5681b, hsa-miR-1231, hsa-miR-520g-3p, hsa-miR-4787-5p, hsa-miR-9902, hsa-miR-632, hsa-miR-483-5p, hsa-miR-488-5p, hsa-miR-1193, hsa-miR-4726-3p, hsa-miR-3935, hsa-miR-8088, hsa-miR-937-5p, hsa-let-7b-3p, hsa-miR-3160-5p, hsa-miR-6778-5p, hsa-miR-6866-3p, hsa-miR-6762-5p, hsa-miR-3616-3p, hsa-miR-1238-5p, hsa-miR-6802-3p, hsa-miR-1257, hsa-miR-192-5p, hsa-miR-4752, hsa-miR-4539, hsa-miR-4448, hsa-miR-6772-3p, hsa-miR-548q, hsa-miR-4710, hsa-miR-302c-5p, hsa-miR-6855-3p, hsa-miR-6856-5p, hsa-miR-15b-5p, hsa-miR-3928-3p, hsa-miR-1249-5p, hsa-miR-6894-5p, hsa-miR-4667-5p, hsa-miR-4721, hsa-miR-887-3p, hsa-miR-6877-5p, hsa-miR-449a, hsa-miR-6880-5p, hsa-miR-11400, hsa-miR-9851-5p, hsa-miR-6132, hsa-miR-4527, hsa-miR-4319, hsa-miR-6072, hsa-miR-6816-5p, hsa-miR-4681, hsa-miR-4533, hsa-miR-28-5p, hsa-miR-3120-5p, hsa-miR-1538, hsa-miR-619-5p, hsa-miR-4438, hsa-miR-4526, hsa-miR-6852-5p, hsa-miR-3154, hsa-miR-6831-5p, hsa-miR-4729, hsa-miR-4483, hsa-miR-1202, hsa-miR-4758-3p, hsa-miR-500a-3p, hsa-miR-760, hsa-miR-4733-5p, hsa-miR-3941, hsa-miR-520e-3p, hsa-miR-4667-3p, hsa-miR-3153, hsa-miR-891a-3p, hsa-miR-449c-3p, hsa-miR-212-5p, hsa-miR-6774-3p, hsa-miR-6826-3p, hsa-miR-4796-5p, hsa-miR-7974, hsa-miR-937-3p, hsa-miR-6742-5p, hsa-miR-4441, hsa-miR-6824-5p, hsa-miR-194-5p, hsa-miR-3610, hsa-miR-6728-5p, hsa-miR-15a-5p, hsa-miR-572, hsa-miR-1182, hsa-miR-3151-5p, hsa-miR-6889-3p, hsa-miR-5705, hsa-miR-4260, hsa-miR-3665, hsa-miR-3657, hsa-miR-6810-5p, hsa-miR-6874-5p, hsa-miR-551a, hsa-miR-574-5p, hsa-miR-6801-3p, hsa-miR-744-5p, hsa-miR-4753-3p, hsa-miR-4685-5p, hsa-miR-7973, hsa-miR-2113, hsa-miR-3659, hsa-miR-520b-3p, hsa-miR-6754-3p, hsa-miR-3085-5p, hsa-miR-4492, hsa-miR-4676-3p, hsa-miR-7113-3p, hsa-miR-6830-5p, hsa-miR-4297, hsa-miR-1343-3p, hsa-miR-550b-3p, hsa-miR-938, hsa-miR-3692-5p, hsa-miR-7152-5p, hsa-miR-128-1-5p, hsa-miR-6836-5p, hsa-miR-6872-5p, hsa-miR-6852-3p, hsa-miR-3157-3p, hsa-miR-6846-3p, hsa-miR-4524a-5p, hsa-miR-4712-3p, hsa-miR-6510-5p, hsa-miR-670-5p, hsa-miR-4733-3p, hsa-miR-6511b-3p, hsa-miR-3200-3p, hsa-miR-6765-5p, hsa-miR-3193, hsa-miR-6800-5p, hsa-miR-6817-3p, hsa-miR-658, hsa-miR-8085, hsa-miR-196a-5p, hsa-miR-519e-3p, hsa-miR-6513-3p, hsa-miR-8052, hsa-miR-4640-5p, hsa-miR-1285-5p, hsa-miR-151b, hsa-miR-6873-5p, hsa-miR-12128, hsa-miR-1203, hsa-miR-6841-3p, hsa-miR-6895-3p, hsa-miR-4513, hsa-miR-3940-5p, hsa-miR-6514-3p, hsa-miR-6799-5p, hsa-miR-6716-5p, hsa-miR-4761-3p, hsa-miR-196b-3p, hsa-miR-6827-5p, hsa-miR-6825-5p, hsa-miR-3689d, hsa-miR-1233-5p, hsa-miR-3691-3p, hsa-miR-1273h-5p, hsa-miR-4306, hsa-miR-323b-3p, hsa-miR-301a-5p, hsa-miR-6771-5p, hsa-miR-1273h-3p, hsa-miR-491-5p, hsa-miR-1287-3p, hsa-miR-3663-5p, hsa-miR-1307-3p, hsa-miR-3944-3p, hsa-miR-5787, hsa-miR-4716-3p, hsa-miR-383-3p, hsa-miR-1266-5p, hsa-miR-5001-5p, hsa-miR-6813-5p, hsa-miR-7977, hsa-miR-6133, hsa-miR-3126-5p, hsa-miR-3168, hsa-miR-378e, hsa-miR-6806-5p, hsa-miR-3164, hsa-miR-487b-5p, hsa-miR-920, hsa-miR-4485-5p, hsa-miR-3679-5p, hsa-miR-3129-3p, hsa-miR-4511, hsa-miR-2116-3p, hsa-miR-3145-5p, hsa-miR-6759-3p, hsa-miR-6793-3p, hsa-miR-4999-3p, hsa-miR-4328, hsa-miR-639, hsa-miR-646, hsa-miR-520c-3p, hsa-miR-3147, hsa-miR-6782-3p, hsa-miR-6822-5p, hsa-miR-4478, hsa-miR-130a-5p, hsa-miR-4707-3p, hsa-miR-4259, hsa-miR-744-3p, hsa-miR-6770-3p, hsa-miR-4304, hsa-miR-3133, hsa-miR-589-5p, hsa-miR-30c-2-3p, hsa-miR-4288, hsa-miR-431-3p, hsa-miR-489-3p, hsa-miR-4717-3p, hsa-miR-924, hsa-miR-330-3p, hsa-miR-3132, hsa-miR-3177-3p, hsa-miR-4652-5p, hsa-miR-296-3p, hsa-miR-3198, hsa-miR-6748-5p, hsa-miR-7704, hsa-miR-4326, hsa-miR-1909-5p, hsa-miR-6727-5p, hsa-miR-12124, hsa-miR-6807-5p, hsa-miR-6875-3p, hsa-miR-6783-3p, hsa-miR-4656, hsa-miR-6780b-5p, hsa-miR-6722-5p, hsa-miR-378h, hsa-miR-4727-5p, hsa-miR-8086, hsa-miR-6736-5p, hsa-miR-7112-5p, hsa-miR-6789-3p, hsa-miR-623, hsa-miR-629-3p, hsa-miR-526b-3p, hsa-miR-6733-3p, hsa-miR-7110-5p, hsa-miR-198, hsa-miR-4324, hsa-miR-3960, hsa-miR-4653-3p, hsa-miR-4488, hsa-miR-143-3p, hsa-miR-892a, hsa-miR-2116-5p, hsa-miR-5571-3p, hsa-miR-609, hsa-miR-6808-5p, hsa-miR-339-3p, hsa-miR-146a-5p, hsa-miR-557, hsa-miR-491-3p, hsa-miR-765, hsa-miR-4479, hsa-miR-508-5p, hsa-miR-1825, hsa-miR-4271, hsa-miR-3195, hsa-miR-6881-5p, hsa-miR-4279, hsa-miR-8059, hsa-miR-6820-3p, hsa-miR-3064-3p, hsa-miR-3663-3p, hsa-miR-4443, hsa-miR-12115, hsa-miR-3191-3p, hsa-miR-3189-3p, hsa-miR-6862-5p, hsa-miR-6781-3p, hsa-miR-9500, hsa-miR-3622a-3p, hsa-miR-377-5p, hsa-miR-10394-3p, hsa-miR-766-3p, hsa-miR-10400-5p, hsa-miR-10398-5p, hsa-miR-6865-5p, hsa-miR-4253, hsa-miR-8073, hsa-miR-6883-5p, hsa-miR-6728-3p, hsa-miR-7846-3p, hsa-miR-4641, hsa-miR-6769b-5p, hsa-miR-486-5p, hsa-miR-654-3p, hsa-miR-203b-5p, hsa-miR-4269, hsa-miR-3622b-5p, hsa-miR-6088, hsa-miR-4476, hsa-miR-4697-5p, hsa-miR-7109-3p, hsa-miR-1184, hsa-miR-183-3p, hsa-miR-154-5p, hsa-miR-6734-5p, hsa-miR-128-2-5p, hsa-miR-4318, hsa-miR-603, hsa-miR-217-3p, hsa-miR-4723-5p, hsa-miR-6787-3p, hsa-miR-1227-3p, hsa-miR-6867-5p, hsa-miR-3680-5p, hsa-miR-1976, hsa-miR-7843-5p, hsa-miR-1296-3p, hsa-miR-5694, hsa-miR-6875-5p, hsa-miR-4786-5p, hsa-miR-4718, hsa-miR-424-3p, hsa-miR-6893-3p, hsa-miR-1297, hsa-miR-371a-3p, hsa-miR-363-5p, hsa-miR-642b-5p, hsa-miR-4537, hsa-miR-27b-3p, hsa-miR-6779-5p, hsa-miR-4804-3p, hsa-miR-671-3p, hsa-miR-661, hsa-miR-374a-3p, hsa-miR-509-3-5p, hsa-miR-4781-5p, hsa-miR-144-5p, hsa-miR-4519, hsa-miR-15a-3p, hsa-miR-571, hsa-miR-6883-3p, hsa-miR-558, hsa-miR-2277-5p, hsa-miR-29a-5p, hsa-miR-3165, hsa-miR-598-5p, hsa-miR-3074-5p, hsa-miR-6509-3p, hsa-miR-1247-3p, hsa-miR-885-5p, hsa-miR-563, hsa-miR-371a-5p, hsa-miR-302d-5p, hsa-miR-6736-3p, hsa-miR-3682-5p, hsa-miR-6780a-5p, hsa-miR-548d-3p, hsa-miR-30b-5p, hsa-miR-6816-3p, hsa-miR-128-3p, hsa-miR-583, hsa-miR-615-3p, hsa-miR-6869-3p, hsa-miR-5197-5p, hsa-miR-5588-5p, hsa-miR-6786-3p, hsa-miR-2355-3p, hsa-miR-412-3p, hsa-miR-4732-5p, hsa-miR-203a-5p, hsa-miR-663a, hsa-miR-6882-3p, hsa-miR-6785-5p, hsa-miR-676-3p, hsa-miR-6857-5p, hsa-miR-1228-5p, hsa-miR-4764-3p, hsa-miR-581, hsa-miR-4793-3p, hsa-miR-605-5p, hsa-miR-5704, hsa-miR-365b-5p, hsa-miR-186-3p, hsa-miR-125a-3p, hsa-miR-6809-3p, hsa-miR-6833-3p, hsa-miR-6126, hsa-miR-12121, hsa-miR-4293, hsa-miR-4788, hsa-miR-371b-3p, hsa-miR-551b-3p, hsa-miR-3156-5p, hsa-miR-6776-3p, hsa-miR-3925-5p, hsa-miR-1250-3p, hsa-miR-6878-3p, hsa-miR-933, hsa-miR-10397-5p, hsa-miR-5584-5p, hsa-miR-2392, hsa-miR-3130-5p, hsa-miR-6870-5p, hsa-miR-5001-3p, hsa-miR-6832-3p, hsa-miR-4270, hsa-miR-4640-3p, hsa-miR-6830-3p, hsa-miR-502-3p, hsa-miR-4436b-5p, hsa-miR-628-3p, hsa-miR-6720-5p, hsa-miR-4281, hsa-miR-6754-5p, hsa-miR-6769a-5p, hsa-miR-7152-3p, hsa-miR-6735-5p, hsa-miR-6716-3p, hsa-miR-4325, hsa-miR-1229-5p, hsa-miR-6731-5p, hsa-miR-522-3p, hsa-miR-4709-3p, hsa-miR-185-5p, hsa-miR-4756-5p, hsa-miR-548ba, hsa-miR-196a-1-3p, hsa-miR-3144-5p, hsa-miR-4494, hsa-miR-500a-5p, hsa-miR-3973, hsa-miR-3714, hsa-miR-4724-5p, hsa-miR-6858-5p, hsa-miR-8060, hsa-miR-320d, hsa-miR-4725-5p, hsa-miR-4691-3p, hsa-miR-6857-3p, hsa-miR-4664-3p, hsa-miR-6778-3p, hsa-miR-6815-3p, hsa-miR-4649-5p, hsa-miR-4743-3p, hsa-miR-3926, hsa-miR-505-5p, hsa-miR-7107-3p, hsa-miR-212-3p, hsa-miR-132-5p, hsa-miR-520a-3p, hsa-miR-634, hsa-miR-935, hsa-miR-6890-3p, hsa-miR-3922-3p, hsa-miR-6856-3p, hsa-miR-3116, hsa-miR-6761-3p, hsa-miR-3158-5p, hsa-miR-3059-5p, hsa-miR-4305, hsa-miR-4732-3p, hsa-miR-3160-3p, hsa-miR-5010-3p, hsa-miR-668-3p, hsa-miR-130b-3p, hsa-miR-6851-5p, hsa-miR-6715b-3p, hsa-miR-130b-5p, hsa-miR-449b-3p, hsa-miR-615-5p, hsa-miR-4712-5p, hsa-miR-4697-3p, hsa-miR-3163, hsa-miR-6769b-3p, hsa-miR-7702, hsa-miR-151a-5p, hsa-miR-3120-3p, hsa-miR-6769a-3p, hsa-miR-6774-5p, hsa-let-7g-3p, hsa-miR-378j, hsa-miR-6753-3p, hsa-miR-2681-5p, hsa-miR-134-5p, hsa-miR-6740-3p, hsa-miR-3194-5p, hsa-miR-3174, hsa-miR-4731-5p, hsa-miR-3690, hsa-miR-3150a-3p, hsa-miR-503-3p, hsa-miR-10527-5p, hsa-miR-369-5p, hsa-miR-6886-3p, hsa-miR-941, hsa-miR-6874-3p, hsa-miR-6747-3p, hsa-miR-642b-3p, hsa-miR-4685-3p, hsa-miR-6891-5p, hsa-miR-370-5p, hsa-miR-8485, hsa-miR-526b-5p, hsa-miR-4251, hsa-miR-521, hsa-miR-208a-5p, hsa-miR-5681a, hsa-miR-3925-3p, hsa-miR-7155-5p, hsa-miR-1284, hsa-miR-637, hsa-miR-1260b, hsa-miR-362-3p, hsa-miR-4518, hsa-miR-6847-3p, hsa-miR-4722-3p, hsa-miR-4268, hsa-miR-657, hsa-miR-133b, hsa-miR-381-5p, hsa-miR-320a-3p, hsa-miR-5000-3p, hsa-miR-4689, hsa-miR-4312, hsa-miR-520h, hsa-miR-574-3p, hsa-miR-6804-3p, hsa-miR-12126, hsa-miR-3605-3p, hsa-miR-6780b-3p, hsa-miR-3649, hsa-miR-6890-5p, hsa-miR-6894-3p, hsa-miR-6730-3p, hsa-miR-6829-3p, hsa-miR-6757-5p, hsa-miR-6849-3p, hsa-miR-6758-5p, hsa-miR-1908-3p, hsa-miR-4278, hsa-miR-3191-5p, hsa-miR-1226-3p, hsa-miR-664a-5p, hsa-miR-642a-3p, hsa-miR-1306-5p, hsa-miR-512-5p, hsa-let-7c-3p, hsa-miR-4307, hsa-miR-6742-3p, hsa-miR-183-5p, hsa-miR-1207-3p, hsa-miR-519d-3p, hsa-miR-3667-3p, hsa-miR-1267, hsa-miR-30a-3p, hsa-miR-6851-3p, hsa-miR-16-1-3p, hsa-miR-513a-5p, hsa-miR-676-5p, hsa-miR-6757-3p, hsa-miR-125b-5p, hsa-miR-1-5p, hsa-miR-4646-5p, hsa-miR-675-3p, hsa-miR-6779-3p, hsa-miR-7161-5p, hsa-miR-5193, hsa-miR-6803-3p, hsa-miR-5699-5p, hsa-miR-12136, hsa-miR-4462, hsa-miR-663b, hsa-miR-155-3p, hsa-miR-616-3p, hsa-miR-6879-3p, hsa-miR-1295b-3p, hsa-miR-5195-5p, hsa-miR-7975, hsa-miR-1233-3p, hsa-miR-6869-5p, hsa-miR-6819-5p, hsa-miR-7106-3p, hsa-miR-6744-5p, hsa-miR-1539, hsa-miR-550a-3p, hsa-miR-6735-3p, hsa-miR-6780a-3p, hsa-miR-887-5p, hsa-miR-6828-5p, hsa-miR-1307-5p, hsa-miR-122-5p, hsa-miR-6758-3p, hsa-miR-2355-5p, hsa-miR-6744-3p, hsa-miR-320b, and hsa-miR-485-3p. In the present disclosure, the extract of urine may be an extract of the urine of a pancreatic cancer patient. Only one of these microRNAs can be used to predict whether a subject from which the urine is derived is a pancreatic cancer patient, and 5 or more types, 10 or more types, 15 or more types, or 20 or more types of these microRNAs can be used in combination to predict whether a subject from which the urine is derived is a pancreatic cancer patient. Among these microRNAs, a microRNA that may be highly expressed in a pancreatic cancer patient can be preferably used for prediction.

The present disclosure provides an extract of urine containing at least one type, two types, three types, four types, five types, six types, seven types, eight types, nine types, or ten types of or all microRNAs selected from the group consisting of hsa-miR-15a-5p, hsa-miR-16-5p, hsa-miR-17-3p, hsa-miR-19b-3p, hsa-miR-20a-5p, hsa-miR-27a-3p, hsa-miR-28-5p, hsa-miR-29a-3p, hsa-miR-30a-5p, hsa-miR-33a-5p, hsa-miR-99a-5p, hsa-miR-29b-3p, hsa-miR-196a-5p, hsa-miR-197-3p, hsa-miR-198, hsa-miR-30d-5p, hsa-miR-139-5p, hsa-miR-10a-5p, hsa-miR-34a-5p, hsa-miR-181c-5p, hsa-miR-182-3p, hsa-miR-183-5p, hsa-miR-187-3p, hsa-miR-199b-5p, hsa-miR-210-3p, hsa-miR-211-5p, hsa-miR-214-3p, hsa-miR-218-5p, hsa-miR-223-3p, hsa-let-7g-5p, hsa-miR-125b-5p, hsa-miR-133a-3p, hsa-miR-135a-5p, hsa-miR-9-5p, hsa-miR-125a-5p, hsa-miR-134-5p, hsa-miR-149-5p, hsa-miR-154-3p, hsa-miR-184, hsa-miR-185-5p, hsa-miR-188-5p, hsa-miR-320a-3p, hsa-miR-200c-3p, hsa-miR-106b-5p, hsa-miR-29c-3p, hsa-miR-200a-3p, hsa-miR-302a-3p, hsa-miR-299-3p, hsa-miR-296-5p, hsa-miR-130b-3p, hsa-miR-361-5p, hsa-miR-365a-3p, hsa-miR-365b-3p, , hsa-miR-302c-5p, , hsa-miR-370-3p, hsa-miR-373-5p, hsa-miR-373-3p, hsa-miR-378a-3p, hsa-miR-379-5p, hsa-miR-328-3p, hsa-miR-342-3p, hsa-miR-337-3p, hsa-miR-326, hsa-miR-151a-3p, hsa-miR-133b, hsa-miR-325, hsa-miR-346, hsa-miR-422a, hsa-miR-424-5p, hsa-miR-18b-5p, hsa-miR-20b-5p, hsa-miR-429, hsa-miR-450a-5p, hsa-miR-191-3p, hsa-miR-200a-5p, hsa-miR-323b-5p, hsa-miR-409-3p, hsa-miR-412-3p, hsa-miR-410-3p, hsa-miR-483-3p, hsa-miR-484, hsa-miR-485-3p, hsa-miR-486-5p, hsa-miR-488-5p, hsa-miR-491-5p, hsa-miR-146b-5p, hsa-miR-202-3p, hsa-miR-492, hsa-miR-512-5p, hsa-miR-498-5p, hsa-miR-520e-3p, hsa-miR-515-3p, hsa-miR-519e-5p, hsa-miR-519e-3p, hsa-miR-520f-3p, hsa-miR-519c-5p, hsa-miR-523-5p, hsa-miR-518e-5p, hsa-miR-522-5p, hsa-miR-519a-5p, hsa-miR-519b-5p, hsa-miR-526b-5p, hsa-miR-526b-3p, hsa-miR-525-3p, hsa-miR-518f-3p, hsa-miR-520b-3p, hsa-miR-518b, hsa-miR-520c-3p, hsa-miR-518c-5p, hsa-miR-519d-3p, hsa-miR-516b-3p, hsa-miR-516a-3p, hsa-miR-522-3p, hsa-miR-500a-3p, hsa-miR-503-5p, hsa-miR-513a-5p, hsa-miR-532-5p, hsa-miR-455-5p, hsa-miR-539-5p, hsa-miR-551a, hsa-miR-554, hsa-miR-555, hsa-miR-557, hsa-miR-564, hsa-miR-571, hsa-miR-574-3p, hsa-miR-575, hsa-miR-579-3p, hsa-miR-580-3p, hsa-miR-583, hsa-miR-584-5p, hsa-miR-585-3p, hsa-miR-587, hsa-miR-588, hsa-miR-550a-3p, hsa-miR-595, hsa-miR-600, hsa-miR-601, hsa-miR-608, hsa-miR-610, hsa-miR-612, hsa-miR-615-3p, hsa-miR-617, hsa-miR-618, hsa-miR-619-3p, hsa-miR-622, hsa-miR-624-5p, hsa-miR-626, hsa-miR-628-3p, hsa-miR-629-3p, hsa-miR-632, hsa-miR-634, hsa-miR-637, hsa-miR-638, hsa-miR-639, hsa-miR-641, hsa-miR-642a-5p, hsa-miR-648, hsa-miR-649, hsa-miR-650, hsa-miR-661, hsa-miR-663a, hsa-miR-449b-5p, hsa-miR-654-5p, hsa-miR-657, hsa-miR-658, hsa-miR-659-3p, hsa-miR-542-5p, hsa-miR-425-5p, hsa-miR-671-5p, hsa-miR-668-3p, hsa-miR-769-3p, hsa-miR-766-3p, hsa-miR-765, hsa-miR-675-5p, hsa-miR-297, hsa-let-7b-3p, hsa-let-7d-3p, hsa-let-7f-1-3p, hsa-miR-22-5p, hsa-miR-24-2-5p, hsa-miR-25-5p, hsa-miR-26b-3p, hsa-miR-27a-5p, hsa-miR-29a-5p, hsa-miR-92a-1-5p, hsa-miR-92a-2-5p, hsa-miR-29b-1-5p, hsa-miR-30c-2-3p, hsa-miR-139-3p, hsa-miR-183-3p, hsa-miR-187-5p, hsa-miR-221-5p, hsa-miR-15b-3p, hsa-miR-124-5p, hsa-miR-125b-l-3p, hsa-miR-130a-5p, hsa-miR-132-5p, hsa-miR-135a-3p, hsa-miR-140-3p, hsa-miR-125a-3p, hsa-miR-125b-2-3p, hsa-miR-149-3p, hsa-miR-150-3p, hsa-miR-185-3p, hsa-miR-186-3p, hsa-miR-193a-5p, hsa-miR-194-3p, hsa-miR-30c-1-3p, hsa-miR-34c-3p, hsa-miR-99b-3p, hsa-miR-296-3p, hsa-miR-130b-5p, hsa-miR-361-3p, hsa-miR-302d-5p, hsa-miR-371a-5p, hsa-miR-374a-3p, hsa-miR-377-5p, hsa-miR-342-5p, hsa-miR-323a-5p, hsa-miR-338-5p, hsa-miR-339-3p, hsa-miR-423-5p, hsa-miR-424-3p, hsa-miR-18b-3p, hsa-miR-20b-3p, hsa-miR-431-3p, hsa-miR-483-5p, hsa-miR-486-3p, hsa-miR-490-5p, hsa-miR-146b-3p, hsa-miR-193b-5p, hsa-miR-502-3p, hsa-miR-505-5p, hsa-miR-509-5p, hsa-miR-532-3p, hsa-miR-92b-5p, hsa-miR-551b-5p, hsa-miR-574-5p, hsa-miR-582-3p, hsa-miR-550a-5p, hsa-miR-615-5p, hsa-miR-625-3p, hsa-miR-629-5p, hsa-miR-671-3p, hsa-miR-890, hsa-miR-891b, hsa-miR-541-3p, hsa-miR-875-5p, hsa-miR-708-3p, hsa-miR-744-5p, hsa-miR-744-3p, hsa-miR-885-5p, hsa-miR-885-3p, hsa-miR-877-5p, hsa-miR-877-3p, hsa-miR-887-3p, hsa-miR-665, hsa-miR-760, hsa-miR-920, hsa-miR-921, hsa-miR-922, hsa-miR-933, hsa-miR-936, hsa-miR-939-5p, hsa-miR-940, hsa-miR-1224-5p, hsa-miR-1224-3p, hsa-miR-1225-5p, hsa-miR-1225-3p, hsa-miR-1226-3p, hsa-miR-1227-3p, hsa-miR-1228-5p, hsa-miR-1228-3p, hsa-miR-1229-3p, hsa-miR-1231, hsa-miR-1233-3p, hsa-miR-1234-3p, hsa-miR-1236-3p, hsa-miR-1237-3p, hsa-miR-1238-3p, hsa-miR-320b, hsa-miR-320c, hsa-miR-1298-5p, hsa-miR-1180-3p, hsa-miR-1182, hsa-miR-1184, hsa-miR-1202, hsa-miR-1203, hsa-miR-1204, hsa-miR-1207-5p, hsa-miR-1207-3p, hsa-miR-1285-3p, hsa-miR-1289, hsa-miR-1290, hsa-miR-1293, hsa-miR-1294, hsa-miR-1304-5p, hsa-miR-1246, hsa-miR-1249-3p, hsa-miR-1257, hsa-miR-1260a, hsa-miR-1261, hsa-miR-1266-5p, hsa-miR-1267, hsa-miR-1268a, hsa-miR-1270, hsa-miR-1272, hsa-miR-1278, hsa-miR-1281, hsa-miR-1292-5p, hsa-miR-1255b-5p, hsa-miR-664a-3p, hsa-miR-1306-3p, hsa-miR-1307-3p, hsa-miR-320d, hsa-miR-1825, hsa-miR-1827, hsa-miR-1468-5p, hsa-miR-675-3p, hsa-miR-1469, hsa-miR-1470, hsa-miR-1471, hsa-miR-1538, hsa-miR-1539, hsa-miR-1908-5p, hsa-miR-1909-5p, hsa-miR-1909-3p, hsa-miR-1910-5p, hsa-miR-1911-5p, hsa-miR-1912-3p, hsa-miR-1913, hsa-miR-1914-3p, hsa-miR-1915-3p, hsa-miR-205-3p, hsa-miR-365a-5p, hsa-miR-449b-3p, hsa-miR-2113, hsa-miR-1972, hsa-miR-1976, hsa-miR-2110, hsa-miR-151b, hsa-miR-762, hsa-miR-670-5p, hsa-miR-764, hsa-miR-2116-3p, hsa-miR-548q, hsa-miR-2276-3p, hsa-miR-2278, hsa-miR-71 1, hsa-miR-718, hsa-miR-2681-3p, hsa-miR-449c-3p, hsa-miR-2861, hsa-miR-3115, hsa-miR-3116, hsa-miR-3117-3p, hsa-miR-3120-3p, hsa-miR-3122, hsa-miR-3125, hsa-miR-3126-5p, hsa-miR-3130-3p, hsa-miR-3130-5p, hsa-miR-3131, hsa-miR-3132, hsa-miR-3133, hsa-miR-466, hsa-miR-3136-5p, hsa-miR-3137, hsa-miR-3138, hsa-miR-3141, hsa-miR-3144-5p, hsa-miR-1273c, hsa-miR-3147, hsa-miR-548v, hsa-miR-3148, hsa-miR-3150a-3p, hsa-miR-3151-5p, hsa-miR-3153, hsa-miR-3154, hsa-miR-3156-5p, hsa-miR-3157-5p, hsa-miR-3160-3p, hsa-miR-3162-5p, hsa-miR-3163, hsa-miR-3164, hsa-miR-3165, hsa-miR-1260b, hsa-miR-3168, hsa-miR-3169, hsa-miR-3173-3p, hsa-miR-1193, hsa-miR-3176, hsa-miR-3177-3p, hsa-miR-3178, hsa-miR-3180-5p, hsa-miR-3180-3p, hsa-miR-3183, hsa-miR-3184-5p, hsa-miR-3185, hsa-miR-3186-5p, hsa-miR-3186-3p, hsa-miR-3189-3p, hsa-miR-3190-5p, hsa-miR-3191-3p, hsa-miR-3194-5p, hsa-miR-3195, hsa-miR-3196, hsa-miR-3197, hsa-miR-3198, hsa-miR-514b-5p, hsa-miR-4296, hsa-miR-4297, hsa-miR-4293, hsa-miR-4294, hsa-miR-4299, hsa-miR-4298, hsa-miR-4305, hsa-miR-4306, hsa-miR-4307, hsa-miR-4312, hsa-miR-4313, hsa-miR-4316, hsa-miR-4314, hsa-miR-4320, hsa-miR-4322, hsa-miR-4321, hsa-miR-4323, hsa-miR-4324, hsa-miR-4256, hsa-miR-4257, hsa-miR-4258, hsa-miR-4259, hsa-miR-4260, hsa-miR-4253, hsa-miR-4251, hsa-miR-4254, hsa-miR-4252, hsa-miR-4325, hsa-miR-4326, hsa-miR-4327, hsa-miR-4261, hsa-miR-4265, hsa-miR-4266, hsa-miR-2355-5p, hsa-miR-4268, hsa-miR-4269, hsa-miR-4270, hsa-miR-4271, hsa-miR-4276, hsa-miR-4274, hsa-miR-4281, hsa-miR-4279, hsa-miR-4278, hsa-miR-4280, hsa-miR-4285, hsa-miR-4283, hsa-miR-4284, hsa-miR-4286, hsa-miR-4287, hsa-miR-4289, hsa-miR-4290, hsa-miR-4329, hsa-miR-500b-5p, hsa-miR-3200-5p, hsa-miR-3605-3p, hsa-miR-3609, hsa-miR-3610, hsa-miR-3612, hsa-miR-3613-5p, hsa-miR-3614-5p, hsa-miR-3616-3p, hsa-miR-3617-5p, hsa-miR-3618, hsa-miR-3620-3p, hsa-miR-3621, hsa-miR-3622a-5p, hsa-miR-3622a-3p, hsa-miR-3622b-5p, hsa-miR-3646, hsa-miR-3648, hsa-miR-3649, hsa-miR-3650, hsa-miR-3652, hsa-miR-3660, hsa-miR-3662, hsa-miR-3663-5p, hsa-miR-3663-3p, hsa-miR-3665, hsa-miR-3667-5p, hsa-miR-3667-3p, hsa-miR-3675-3p, hsa-miR-3679-5p, hsa-miR-3679-3p, hsa-miR-3681-5p, hsa-miR-3688-3p, hsa-miR-3689a-3p, hsa-miR-3691-5p, hsa-miR-3714, hsa-miR-3180, hsa-miR-3907, hsa-miR-3689b-3p, hsa-miR-3689c, hsa-miR-3908, hsa-miR-3909, hsa-miR-3911, hsa-miR-3914, hsa-miR-3915, hsa-miR-3917, hsa-miR-3918, hsa-miR-3919, hsa-miR-3150b-3p, hsa-miR-3922-3p, hsa-miR-3924, hsa-miR-3925-5p, hsa-miR-3926, hsa-miR-676-3p, hsa-miR-3928-3p, hsa-miR-3934-5p, hsa-miR-3935, hsa-miR-3936, hsa-miR-3937, hsa-miR-3941, hsa-miR-3943, hsa-miR-3944-3p, hsa-miR-3945, hsa-miR-642b-3p, hsa-miR-550b-3p, hsa-miR-1268b, hsa-miR-4418, hsa-miR-4421, hsa-miR-4428, hsa-miR-4429, hsa-miR-4430, hsa-miR-548ad-3p, hsa-miR-4433a-3p, hsa-miR-4436a, hsa-miR-4437, hsa-miR-4439, hsa-miR-4440, hsa-miR-4441, hsa-miR-4442, hsa-miR-4443, hsa-miR-4444, hsa-miR-4446-3p, hsa-miR-4447, hsa-miR-4448, hsa-miR-4449, hsa-miR-548ah-5p, hsa-miR-4455, hsa-miR-4456, hsa-miR-4458, hsa-miR-4460, hsa-miR-378h, hsa-miR-3135b, hsa-miR-4462, hsa-miR-4463, hsa-miR-548ai, hsa-miR-570-5p, hsa-miR-4466, hsa-miR-4467, hsa-miR-4468, hsa-miR-4470, hsa-miR-4471, hsa-miR-4472, hsa-miR-4474-3p, hsa-miR-4476, hsa-miR-4478, hsa-miR-3155b, hsa-miR-4480, hsa-miR-4481, hsa-miR-4483, hsa-miR-4484, hsa-miR-4486, hsa-miR-4487, hsa-miR-4488, hsa-miR-4489, hsa-miR-4492, hsa-miR-4495, hsa-miR-4496, hsa-miR-4497, hsa-miR-4498, hsa-miR-4499, hsa-miR-4505, hsa-miR-4506, hsa-miR-2392, hsa-miR-4507, hsa-miR-4508, hsa-miR-4510, hsa-miR-4511, hsa-miR-4512, hsa-miR-4513, hsa-miR-4516, hsa-miR-4518, hsa-miR-4519, hsa-miR-4520-3p, hsa-miR-4521, hsa-miR-1269b, hsa-miR-4522, hsa-miR-4523, hsa-miR-4524a-3p, hsa-miR-4525, hsa-miR-4526, hsa-miR-4528, hsa-miR-4530, hsa-miR-4531, hsa-miR-4533, hsa-miR-4534, hsa-miR-4535, hsa-miR-1587, hsa-miR-4538, hsa-miR-4539, hsa-miR-4540, hsa-miR-3120-5p, hsa-miR-3121-5p, hsa-miR-3127-3p, hsa-miR-3140-5p, hsa-miR-3156-3p, hsa-miR-3158-5p, hsa-miR-3162-3p, hsa-miR-3173-5p, hsa-miR-3187-5p, hsa-miR-3189-5p, hsa-miR-3619-3p, hsa-miR-3691-3p, hsa-miR-3150b-5p, hsa-miR-3922-5p, hsa-miR-3925-3p, hsa-miR-3940-5p, hsa-miR-4529-5p, hsa-miR-3960, hsa-miR-3973, hsa-miR-3978, hsa-miR-4634, hsa-miR-4638-5p, hsa-miR-4639-5p, hsa-miR-4639-3p, hsa-miR-4640-5p, hsa-miR-4640-3p, hsa-miR-4642, hsa-miR-4644, hsa-miR-4646-5p, hsa-miR-4646-3p, hsa-miR-4647, hsa-miR-4648, hsa-miR-4649-5p, hsa-miR-4649-3p, hsa-miR-4650-3p, hsa-miR-4651, hsa-miR-4653-3p, hsa-miR-4654, hsa-miR-4655-5p, hsa-miR-4656, hsa-miR-4657, hsa-miR-4664-5p, hsa-miR-4664-3p, hsa-miR-4665-5p, hsa-miR-4665-3p, hsa-miR-4667-5p, hsa-miR-4667-3p, hsa-miR-4668-5p, hsa-miR-4669, hsa-miR-4670-5p, hsa-miR-4670-3p, hsa-miR-4672, hsa-miR-4673, hsa-miR-4674, hsa-miR-4676-5p, hsa-miR-4677-5p, hsa-miR-4682, hsa-miR-4684-3p, hsa-miR-4685-5p, hsa-miR-4685-3p, hsa-miR-4686, hsa-miR-4687-5p, hsa-miR-4687-3p, hsa-miR-1343-3p, hsa-miR-4688, hsa-miR-4689, hsa-miR-4690-5p, hsa-miR-4691-5p, hsa-miR-4691-3p, hsa-miR-4692, hsa-miR-4695-5p, hsa-miR-4695-3p, hsa-miR-4697-5p, hsa-miR-4697-3p, hsa-miR-4698, hsa-miR-4700-5p, hsa-miR-4700-3p, hsa-miR-4701-5p, hsa-miR-4701-3p, hsa-miR-4703-5p, hsa-miR-4704-3p, hsa-miR-4706, hsa-miR-4707-5p, hsa-miR-4707-3p, hsa-miR-4708-5p, hsa-miR-4708-3p, hsa-miR-4709-3p, hsa-miR-203b-5p, hsa-miR-203b-3p, hsa-miR-4710, hsa-miR-4712-3p, hsa-miR-4713-5p, hsa-miR-4714-5p, hsa-miR-4716-5p, hsa-miR-4716-3p, hsa-miR-4717-3p, hsa-miR-4720-5p, hsa-miR-4721, hsa-miR-4722-5p, hsa-miR-4722-3p, hsa-miR-4723-5p, hsa-miR-4723-3p, hsa-miR-4724-5p, hsa-miR-4725-5p, hsa-miR-4725-3p, hsa-miR-4726-5p, hsa-miR-4726-3p, hsa-miR-4728-5p, hsa-miR-4728-3p, hsa-miR-4729, hsa-miR-4730, hsa-miR-4731-5p, hsa-miR-4731-3p, hsa-miR-4732-5p, hsa-miR-4732-3p, hsa-miR-4734, hsa-miR-4735-3p, hsa-miR-4736, hsa-miR-4737, hsa-miR-3064-5p, hsa-miR-3064-3p, hsa-miR-4738-3p, hsa-miR-4739, hsa-miR-4741, hsa-miR-4743-5p, hsa-miR-122b-5p, hsa-miR-122b-3p, hsa-miR-4745-5p, hsa-miR-4746-5p, hsa-miR-4746-3p, hsa-miR-4747-5p, hsa-miR-4748, hsa-miR-4749-5p, hsa-miR-4749-3p, hsa-miR-4750-5p, hsa-miR-4751, hsa-miR-4752, hsa-miR-4753-5p, hsa-miR-371b-5p, hsa-miR-371b-3p, hsa-miR-4754, hsa-miR-4755-3p, hsa-miR-499b-3p, hsa-miR-4756-5p, hsa-miR-4758-5p, hsa-miR-4758-3p, hsa-miR-4759, hsa-miR-4762-3p, hsa-miR-4763-5p, hsa-miR-4763-3p, hsa-miR-4764-5p, hsa-miR-4766-5p, hsa-miR-4769-5p, hsa-miR-4769-3p, hsa-miR-4771, hsa-miR-4776-5p, hsa-miR-4776-3p, hsa-miR-4778-5p, hsa-miR-4778-3p, hsa-miR-4779, hsa-miR-4780, hsa-miR-4436b-5p, hsa-miR-4436b-3p, hsa-miR-4781-5p, hsa-miR-4781-3p, hsa-miR-4783-3p, hsa-miR-4784, hsa-miR-2467-3p, hsa-miR-4787-5p, hsa-miR-4787-3p, hsa-miR-4788, hsa-miR-4791, hsa-miR-4793-3p, hsa-miR-4795-5p, hsa-miR-4795-3p, hsa-miR-4800-5p, hsa-miR-4802-5p, hsa-miR-4802-3p, hsa-miR-4804-3p, hsa-miR-4520-2-3p, hsa-miR-642a-3p, hsa-miR-550a-3-5p, hsa-miR-4433a-5p, hsa-miR-4482-3p, hsa-miR-4999-5p, hsa-miR-5000-3p, hsa-miR-5001-5p, hsa-miR-5001-3p, hsa-miR-5002-3p, hsa-miR-5003-3p, hsa-miR-5004-5p, hsa-miR-5006-5p, hsa-miR-5006-3p, hsa-miR-5008-5p, hsa-miR-5009-5p, hsa-miR-5009-3p, hsa-miR-5010-5p, hsa-miR-5010-3p, hsa-miR-5087, hsa-miR-5088-5p, hsa-miR-5089-5p, hsa-miR-5090, hsa-miR-5093, hsa-miR-5188, hsa-miR-5189-5p, hsa-miR-5193, hsa-miR-5195-5p, hsa-miR-5195-3p, hsa-miR-5196-5p, hsa-miR-5196-3p, hsa-miR-5197-3p, hsa-miR-4524b-5p, hsa-miR-5100, hsa-miR-5572, hsa-miR-5579-5p, hsa-miR-664b-5p, hsa-miR-664b-3p, hsa-miR-548at-5p, hsa-miR-548at-3p, hsa-miR-5583-3p, hsa-miR-5586-3p, hsa-miR-5588-5p, hsa-miR-5589-5p, hsa-miR-5684, hsa-miR-5690, hsa-miR-4666b, hsa-miR-5698, hsa-miR-5703, hsa-miR-5705, hsa-miR-5708, hsa-miR-197-5p, hsa-miR-204-3p, hsa-miR-211-3p, hsa-miR-301a-5p, hsa-miR-345-3p, hsa-miR-652-5p, hsa-miR-1185-2-3p, hsa-miR-766-5p, hsa-miR-873-3p, hsa-miR-1304-3p, hsa-miR-1247-3p, hsa-miR-548g-5p, hsa-miR-548x-5p, hsa-miR-548aj-5p, hsa-miR-1306-5p, hsa-miR-3184-3p, hsa-miR-3191-5p, hsa-miR-642b-5p, hsa-miR-365b-5p, hsa-miR-1185-1-3p, hsa-miR-3190-3p, hsa-miR-381-5p, hsa-miR-503-3p, hsa-miR-758-5p, hsa-miR-937-5p, hsa-miR-939-3p, hsa-miR-1227-5p, hsa-miR-1229-5p, hsa-miR-1233-5p, hsa-miR-1237-5p, hsa-miR-1238-5p, hsa-miR-1292-3p, hsa-miR-3617-3p, hsa-miR-3620-5p, hsa-miR-4632-5p, hsa-miR-4743-3p, hsa-miR-4750-3p, hsa-miR-5739, hsa-miR-5787, hsa-miR-6068, hsa-miR-6069, hsa-miR-6071, hsa-miR-6072, hsa-miR-6074, hsa-miR-6075, hsa-miR-6076, hsa-miR-6077, hsa-miR-6079, hsa-miR-6083, hsa-miR-6085, hsa-miR-6086, hsa-miR-6088, hsa-miR-6089, hsa-miR-6090, hsa-miR-6124, hsa-miR-6125, hsa-miR-6126, hsa-miR-6127, hsa-miR-378j, hsa-miR-6130, hsa-miR-6131, hsa-miR-6132, hsa-miR-6133, hsa-miR-6134, hsa-miR-6165, hsa-miR-6500-5p, hsa-miR-6500-3p, hsa-miR-6501-3p, hsa-miR-6503-5p, hsa-miR-6506-5p, hsa-miR-6506-3p, hsa-miR-6508-5p, hsa-miR-6509-3p, hsa-miR-6510-5p, hsa-miR-651 1a-5p, hsa-miR-651 1a-3p, hsa-miR-6512-3p, hsa-miR-6513-5p, hsa-miR-6514-3p, hsa-miR-6515-5p, hsa-miR-6515-3p, hsa-miR-6715b-3p, hsa-miR-6716-5p, hsa-miR-6716-3p, hsa-miR-6717-5p, hsa-miR-6511b-Sp, hsa-miR-65 11b-3p, hsa-miR-6719-3p, hsa-miR-6721-5p, hsa-miR-6722-5p, hsa-miR-6722-3p, hsa-miR-6724-5p, hsa-miR-210-5p, hsa-miR-128-1-5p, hsa-miR-152-5p, hsa-miR-372-5p, hsa-miR-328-5p, hsa-miR-329-5p, hsa-miR-410-5p, hsa-miR-487a-5p, hsa-miR-494-5p, hsa-miR-181d-3p, hsa-miR-520f-5p, hsa-miR-519d-5p, hsa-miR-520g-5p, hsa-miR-504-3p, hsa-miR-487b-5p, hsa-miR-585-5p, hsa-miR-598-5p, hsa-miR-605-3p, hsa-miR-1296-3p, hsa-miR-891a-3p, hsa-miR-874-5p, hsa-miR-887-5p, hsa-miR-1287-3p, hsa-miR-1250-3p, hsa-miR-1537-5p, hsa-miR-1908-3p, hsa-miR-3151-3p, hsa-miR-3192-3p, hsa-miR-1343-5p, hsa-miR-5088-3p, hsa-miR-5189-3p, hsa-miR-5699-5p, hsa-miR-6726-5p, hsa-miR-6726-3p, hsa-miR-6727-5p, hsa-miR-6727-3p, hsa-miR-6728-5p, hsa-miR-6728-3p, hsa-miR-6729-5p, hsa-miR-6729-3p, hsa-miR-6730-5p, hsa-miR-6730-3p, hsa-miR-6731-5p, hsa-miR-6731-3p, hsa-miR-6732-5p, hsa-miR-6732-3p, hsa-miR-6733-5p, hsa-miR-6734-5p, hsa-miR-6734-3p, hsa-miR-6735-5p, hsa-miR-6735-3p, hsa-miR-6736-5p, hsa-miR-6736-3p, hsa-miR-6737-5p, hsa-miR-6737-3p, hsa-miR-6738-5p, hsa-miR-6738-3p, hsa-miR-6740-5p, hsa-miR-6740-3p, hsa-miR-6741-5p, hsa-miR-6741-3p, hsa-miR-6742-5p, hsa-miR-6742-3p, hsa-miR-6743-5p, hsa-miR-6743-3p, hsa-miR-6744-5p, hsa-miR-6744-3p, hsa-miR-6745, hsa-miR-6746-5p, hsa-miR-6747-5p, hsa-miR-6747-3p, hsa-miR-6748-5p, hsa-miR-6748-3p, hsa-miR-6749-5p, hsa-miR-6749-3p, hsa-miR-6750-5p, hsa-miR-6750-3p, hsa-miR-6751-5p, hsa-miR-6751-3p, hsa-miR-6752-5p, hsa-miR-6752-3p, hsa-miR-6753-5p, hsa-miR-6753-3p, hsa-miR-6754-5p, hsa-miR-6754-3p, hsa-miR-6756-5p, hsa-miR-6756-3p, hsa-miR-6757-5p, hsa-miR-6757-3p, hsa-miR-6758-5p, hsa-miR-6759-5p, hsa-miR-6759-3p, hsa-miR-6760-5p, hsa-miR-6760-3p, hsa-miR-6761-3p, hsa-miR-6762-5p, hsa-miR-6762-3p, hsa-miR-6763-5p, hsa-miR-6763-3p, hsa-miR-6765-5p, hsa-miR-6766-5p, hsa-miR-6766-3p, hsa-miR-6767-5p, hsa-miR-6768-5p, hsa-miR-6769a-5p, hsa-miR-6769a-3p, hsa-miR-677 1-5p, hsa-miR-6771-3p, hsa-miR-6772-5p, hsa-miR-6772-3p, hsa-miR-6773-3p, hsa-miR-6774-5p, hsa-miR-6774-3p, hsa-miR-6775-5p, hsa-miR-6775-3p, hsa-miR-6776-5p, hsa-miR-6776-3p, hsa-miR-6777-5p, hsa-miR-6777-3p, hsa-miR-6778-5p, hsa-miR-6778-3p, hsa-miR-6779-5p, hsa-miR-6779-3p, hsa-miR-6780a-5p, hsa-miR-6780a-3p, hsa-miR-6781-5p, hsa-miR-6782-5p, hsa-miR-6782-3p, hsa-miR-6783-5p, hsa-miR-6783-3p, hsa-miR-6784-5p, hsa-miR-6784-3p, hsa-miR-6785-5p, hsa-miR-6785-3p, hsa-miR-6786-5p, hsa-miR-6786-3p, hsa-miR-6787-5p, hsa-miR-6787-3p, hsa-miR-6788-5p, hsa-miR-6788-3p, hsa-miR-6789-5p, hsa-miR-6789-3p, hsa-miR-6790-5p, hsa-miR-6790-3p, hsa-miR-6791-5p, hsa-miR-6792-5p, hsa-miR-6792-3p, hsa-miR-6793-5p, hsa-miR-6793-3p, hsa-miR-6794-5p, hsa-miR-6794-3p, hsa-miR-6795-5p, hsa-miR-6795-3p, hsa-miR-6796-5p, hsa-miR-6796-3p, hsa-miR-6797-5p, hsa-miR-6797-3p, hsa-miR-6798-5p, hsa-miR-6798-3p, hsa-miR-6799-5p, hsa-miR-6799-3p, hsa-miR-6800-5p, hsa-miR-6800-3p, hsa-miR-6801-5p, hsa-miR-6801-3p, hsa-miR-6802-3p, hsa-miR-6803-5p, hsa-miR-6803-3p, hsa-miR-6804-3p, hsa-miR-6805-5p, hsa-miR-6805-3p, hsa-miR-6806-5p, hsa-miR-6807-5p, hsa-miR-6808-5p, hsa-miR-6808-3p, hsa-miR-6809-5p, hsa-miR-6809-3p, hsa-miR-6810-5p, hsa-miR-6810-3p, hsa-miR-6812-5p, hsa-miR-6812-3p, hsa-miR-6813-5p, hsa-miR-6813-3p, hsa-miR-6814-3p, hsa-miR-6815-5p, hsa-miR-6815-3p, hsa-miR-6816-5p, hsa-miR-6816-3p, hsa-miR-6818-5p, hsa-miR-6819-5p, hsa-miR-6819-3p, hsa-miR-6820-5p, hsa-miR-6820-3p, hsa-miR-6821-5p, hsa-miR-6822-5p, hsa-miR-6822-3p, hsa-miR-6823-5p, hsa-miR-6823-3p, hsa-miR-6824-5p, hsa-miR-6825-5p, hsa-miR-6826-3p, hsa-miR-6827-5p, hsa-miR-6827-3p, hsa-miR-6828-5p, hsa-miR-6829-5p, hsa-miR-6829-3p, hsa-miR-6830-5p, hsa-miR-6830-3p, hsa-miR-6831-5p, hsa-miR-6831-3p, hsa-miR-6832-5p, hsa-miR-6832-3p, hsa-miR-6833-5p, hsa-miR-6833-3p, hsa-miR-6834-5p, hsa-miR-6834-3p, hsa-miR-6780b-5p, hsa-miR-6780b-3p, hsa-miR-6836-3p, hsa-miR-6837-5p, hsa-miR-6838-5p, hsa-miR-6839-5p, hsa-miR-6840-5p, hsa-miR-6840-3p, hsa-miR-6841-3p, hsa-miR-6842-5p, hsa-miR-6842-3p, hsa-miR-6845-5p, hsa-miR-6845-3p, hsa-miR-6846-5p, hsa-miR-6846-3p, hsa-miR-6847-5p, hsa-miR-6847-3p, hsa-miR-6848-5p, hsa-miR-6848-3p, hsa-miR-6849-5p, hsa-miR-6849-3p, hsa-miR-6850-5p, hsa-miR-6851-5p, hsa-miR-6851-3p, hsa-miR-6852-3p, hsa-miR-6854-3p, hsa-miR-6855-5p, hsa-miR-6855-3p, hsa-miR-6856-5p, hsa-miR-6857-5p, hsa-miR-6857-3p, hsa-miR-6858-5p, hsa-miR-6858-3p, hsa-miR-6859-5p, hsa-miR-6859-3p, hsa-miR-6769b-5p, hsa-miR-6769b-3p, hsa-miR-6860, hsa-miR-6861-5p, hsa-miR-6861-3p, hsa-miR-6862-5p, hsa-miR-6862-3p, hsa-miR-6864-5p, hsa-miR-6865-5p, hsa-miR-6865-3p, hsa-miR-6866-5p, hsa-miR-6867-5p, hsa-miR-6867-3p, hsa-miR-6868-5p, hsa-miR-6868-3p, hsa-miR-6869-5p, hsa-miR-6870-5p, hsa-miR-6870-3p, hsa-miR-6871-5p, hsa-miR-6872-5p, hsa-miR-6873-5p, hsa-miR-6874-5p, hsa-miR-6875-5p, hsa-miR-6875-3p, hsa-miR-6876-5p, hsa-miR-6876-3p, hsa-miR-6877-5p, hsa-miR-6877-3p, hsa-miR-6878-5p, hsa-miR-6878-3p, hsa-miR-6879-5p, hsa-miR-6879-3p, hsa-miR-6880-5p, hsa-miR-6880-3p, hsa-miR-6881-5p, hsa-miR-6881-3p, hsa-miR-6882-3p, hsa-miR-6883-5p, hsa-miR-6883-3p, hsa-miR-6884-5p, hsa-miR-6884-3p, hsa-miR-6885-5p, hsa-miR-6885-3p, hsa-miR-6886-5p, hsa-miR-6886-3p, hsa-miR-6887-5p, hsa-miR-6887-3p, hsa-miR-6889-5p, hsa-miR-6889-3p, hsa-miR-6890-5p, hsa-miR-6890-3p, hsa-miR-6891-5p, hsa-miR-6891-3p, hsa-miR-6892-5p, hsa-miR-6892-3p, hsa-miR-6893-5p, hsa-miR-6893-3p, hsa-miR-6894-5p, hsa-miR-6894-3p, hsa-miR-6895-3p, hsa-miR-7106-5p, hsa-miR-7106-3p, hsa-miR-7107-5p, hsa-miR-7108-5p, hsa-miR-7108-3p, hsa-miR-7109-5p, hsa-miR-7109-3p, hsa-miR-7110-5p, hsa-miR-7110-3p, hsa-miR-7111-Sp, hsa-miR-7111-3p, hsa-miR-7112-5p, hsa-miR-7113-5p, hsa-miR-7113-3p, hsa-miR-7114-5p, hsa-miR-7114-3p, hsa-miR-7150, hsa-miR-7151-3p, hsa-miR-7152-5p, hsa-miR-7152-3p, hsa-miR-7153-5p, hsa-miR-7154-5p, hsa-miR-7155-5p, hsa-miR-7157-3p, hsa-miR-7159-5p, hsa-miR-7159-3p, hsa-miR-7160-5p, hsa-miR-7161-5p, hsa-miR-7162-5p, hsa-miR-7515, hsa-miR-7702, hsa-miR-7704, hsa-miR-7843-5p, hsa-miR-4433b-5p, hsa-miR-4433b-3p, hsa-miR-1273h-5p, hsa-miR-1273h-3p, hsa-miR-6516-5p, hsa-miR-7845-5p, hsa-miR-7846-3p, hsa-miR-7847-3p, hsa-miR-7851-3p, hsa-miR-7855-5p, hsa-miR-7856-5p, hsa-miR-8052, hsa-miR-8054, hsa-miR-8059, hsa-miR-8060, hsa-miR-8062, hsa-miR-8063, hsa-miR-8064, hsa-miR-8069, hsa-miR-8070, hsa-miR-8071, hsa-miR-8072, hsa-miR-8073, hsa-miR-8074, hsa-miR-8075, hsa-miR-8077, hsa-miR-8079, hsa-miR-8080, hsa-miR-8085, hsa-miR-8087, hsa-miR-8089, hsa-miR-128-2-5p, hsa-miR-548ba, hsa-miR-7976, hsa-miR-7977, hsa-miR-7978, hsa-miR-203a-5p, hsa-miR-1-5p, hsa-miR-1249-5p, hsa-miR-4485-5p, hsa-miR-8485, hsa-miR-9500, hsa-miR-135a-2-3p, hsa-miR-498-3p, hsa-miR-526a-3p, hsa-miR-9718, hsa-miR-9898, hsa-miR-9900, hsa-miR-9985, hsa-miR-1843, hsa-miR-9986, hsa-miR-10392-5p, hsa-miR-10392-3p, hsa-miR-10394-3p, hsa-miR-10396a-5p, hsa-miR-10396a-3p, hsa-miR-10398-5p, hsa-miR-10398-3p, hsa-miR-10400-5p, hsa-miR-10400-3p, hsa-miR-10401-5p, hsa-miR-10401-3p, hsa-miR-10396b-5p, hsa-miR-10522-5p, hsa-miR-9983-3p, hsa-miR-10526-3p, hsa-miR-10527-5p, hsa-miR-11181-5p, hsa-miR-11181-3p, hsa-miR-11399, hsa-miR-11400, hsa-miR-11401, hsa-miR-3059-5p, hsa-miR-3059-3p, hsa-miR-3085-5p, hsa-miR-3085-3p, hsa-miR-6529-5p, hsa-miR-12114, hsa-miR-12115, hsa-miR-12116, hsa-miR-12118, hsa-miR-12119, hsa-miR-12120, hsa-miR-12121, hsa-miR-12122, hsa-miR-12124, hsa-miR-12126, hsa-miR-12128, hsa-miR-12131, and hsa-miR-12136. In the present disclosure, the extract of urine may be an extract of the urine of a stage-I or -II pancreatic cancer patient. Only one of these microRNAs can be used to predict whether a subject from which the urine is derived is a pancreatic cancer patient (particularly stage-I or -II), and 5 or more types, 10 or more types, 15 or more types, or 20 or more types of these microRNAs can be used in combination to predict whether a subject from which the urine is derived is a pancreatic cancer patient (particularly stage-I or -II). Among these microRNAs, a microRNA that may be highly expressed in a pancreatic cancer patient (particularly stage-I or -II) can be preferably used for prediction.

The present disclosure also provides an extract of urine containing at least one type, two types, three types, four types, five types, six types, seven types, eight types, nine types, or ten types of or all microRNAs selected from the group consisting of hsa-miR-15a-5p, hsa-miR-19b-3p, hsa-miR-27a-3p, hsa-miR-28-5p, hsa-miR-29a-3p, hsa-miR-30a-5p, hsa-miR-196a-5p, hsa-miR-10a-5p, hsa-miR-34a-5p, hsa-miR-181c-5p, hsa-miR-183-5p, hsa-miR-210-3p, hsa-miR-133a-3p, hsa-miR-154-3p, hsa-miR-184, hsa-miR-206, hsa-miR-106b-5p, hsa-miR-29c-3p, hsa-miR-302a-3p, hsa-miR-34c-5p, hsa-miR-299-3p, hsa-miR-296-5p, hsa-miR-130b-3p, hsa-miR-361-5p, hsa-miR-365a-3p, hsa-miR-365b-3p, hsa-miR-302c-5p, hsa-miR-373-5p, hsa-miR-373-3p, hsa-miR-378a-3p, hsa-miR-379-5p, hsa-miR-328-3p, hsa-miR-326, hsa-miR-151a-3p, hsa-miR-325, hsa-miR-422a, hsa-miR-424-5p, hsa-miR-200a-5p, hsa-miR-323b-5p, hsa-miR-484, hsa-miR-202-3p, hsa-miR-492, hsa-miR-498-5p, hsa-miR-520e-3p, hsa-miR-515-3p, hsa-miR-519c-5p, hsa-miR-523-5p, hsa-miR-518e-5p, hsa-miR-522-5p, hsa-miR-519a-5p, hsa-miR-519b-5p, hsa-miR-520b-3p, hsa-miR-518b, hsa-miR-520c-3p, hsa-miR-516b-3p, hsa-miR-516a-3p, hsa-miR-522-3p, hsa-miR-500a-3p, hsa-miR-532-5p, hsa-miR-554, hsa-miR-555, hsa-miR-557, hsa-miR-564, hsa-miR-571, hsa-miR-575, hsa-miR-579-3p, hsa-miR-580-3p, hsa-miR-583, hsa-miR-584-5p, hsa-miR-585-3p, hsa-miR-587, hsa-miR-595, hsa-miR-601, hsa-miR-608, hsa-miR-610, hsa-miR-617, hsa-miR-619-3p, hsa-miR-622, hsa-miR-628-3p, hsa-miR-638, hsa-miR-649, hsa-miR-650, hsa-miR-663a, hsa-miR-449b-5p, hsa-miR-425-5p, hsa-miR-671-5p, hsa-miR-769-5p, hsa-miR-769-3p, hsa-miR-765, hsa-miR-675-5p, hsa-miR-297, hsa-let-7b-3p, hsa-let-7d-3p, hsa-let-7f-1-3p, hsa-miR-24-2-5p, hsa-miR-25-5p, hsa-miR-26b-3p, hsa-miR-27a-5p, hsa-miR-29a-5p, hsa-miR-92a-2-5p, hsa-miR-30c-2-3p, hsa-miR-139-3p, hsa-miR-15b-3p, hsa-miR-130a-5p, hsa-miR-132-5p, hsa-miR-135a-3p, hsa-miR-140-3p, hsa-miR-149-3p, hsa-miR-150-3p, hsa-miR-186-3p, hsa-miR-193a-5p, hsa-miR-194-3p, hsa-miR-34c-3p, hsa-miR-99b-3p, hsa-miR-361-3p, hsa-miR-371a-5p, hsa-miR-374a-3p, hsa-miR-323a-5p, hsa-miR-338-5p, hsa-miR-339-3p, hsa-miR-18b-3p, hsa-miR-20b-3p, hsa-miR-486-3p, hsa-miR-193b-5p, hsa-miR-92b-5p, hsa-miR-616-3p, hsa-miR-625-3p, hsa-miR-629-5p, hsa-miR-891b, hsa-miR-888-3p, hsa-miR-892b, hsa-miR-708-5p, hsa-miR-147b-3p, hsa-miR-885-3p, hsa-miR-877-5p, hsa-miR-877-3p, hsa-miR-665, hsa-miR-921, hsa-miR-924, hsa-miR-933, hsa-miR-936, hsa-miR-940, hsa-miR-1224-3p, hsa-miR-1225-5p, hsa-miR-1225-3p, hsa-miR-1227-3p, hsa-miR-1228-5p, hsa-miR-1228-3p, hsa-miR-1234-3p, hsa-miR-1236-3p, hsa-miR-1237-3p, hsa-miR-1238-3p, hsa-miR-320c, hsa-miR-1298-5p, hsa-miR-1202, hsa-miR-1207-5p, hsa-miR-1285-3p, hsa-miR-1289, hsa-miR-1293, hsa-miR-1294, hsa-miR-1249-3p, hsa-miR-1257, hsa-miR-1268a, hsa-miR-1270, hsa-miR-1281, hsa-miR-320d, hsa-miR-1469, hsa-miR-1470, hsa-miR-1471, hsa-miR-1908-5p, hsa-miR-1909-3p, hsa-miR-1912-3p, hsa-miR-1913, hsa-miR-1914-3p, hsa-miR-1915-3p, hsa-miR-205-3p, hsa-miR-365a-5p, hsa-miR-2110, hsa-miR-151b, hsa-miR-762, hsa-miR-2116-3p, hsa-miR-2276-3p, hsa-miR-711, hsa-miR-718, hsa-miR-2681-3p, hsa-miR-2861, hsa-miR-3120-3p, hsa-miR-3124-5p, hsa-miR-3125, hsa-miR-3131, hsa-miR-3137, hsa-miR-3141, hsa-miR-3144-5p, hsa-miR-3148, hsa-miR-3154, hsa-miR-3169, hsa-miR-3173-3p, hsa-miR-3178, hsa-miR-3180-5p, hsa-miR-3180-3p, hsa-miR-3186-3p, hsa-miR-3192-5p, hsa-miR-3196, hsa-miR-3197, hsa-miR-3199, hsa-miR-514b-5p, hsa-miR-4296, hsa-miR-4293, hsa-miR-4294, hsa-miR-4299, hsa-miR-4298, hsa-miR-4306, hsa-miR-4313, hsa-miR-4316, hsa-miR-4314, hsa-miR-4318, hsa-miR-4321, hsa-miR-4323, hsa-miR-4256, hsa-miR-4257, hsa-miR-4258, hsa-miR-4252, hsa-miR-4325, hsa-miR-4327, hsa-miR-4261, hsa-miR-4265, hsa-miR-4271, hsa-miR-4276, hsa-miR-4274, hsa-miR-4281, hsa-miR-4280, hsa-miR-4285, hsa-miR-4284, hsa-miR-4286, hsa-miR-4288, hsa-miR-4289, hsa-miR-500b-5p, hsa-miR-2277-5p, hsa-miR-3614-5p, hsa-miR-3621, hsa-miR-3622a-5p, hsa-miR-3648, hsa-miR-3652, hsa-miR-3660, hsa-miR-3663-3p, hsa-miR-3665, hsa-miR-3666, hsa-miR-3667-5p, hsa-miR-3675-3p, hsa-miR-3679-3p, hsa-miR-3689a-3p, hsa-miR-3180, hsa-miR-3689b-3p, hsa-miR-3689c, hsa-miR-3908, hsa-miR-3919, hsa-miR-3150b-3p, hsa-miR-3922-3p, hsa-miR-676-3p, hsa-miR-3936, hsa-miR-3937, hsa-miR-3943, hsa-miR-3945, hsa-miR-642b-3p, hsa-miR-1268b, hsa-miR-4418, hsa-miR-4421, hsa-miR-4428, hsa-miR-4429, hsa-miR-4431, hsa-miR-4433a-3p, hsa-miR-4436a, hsa-miR-4437, hsa-miR-4439, hsa-miR-4441, hsa-miR-4444, hsa-miR-4446-3p, hsa-miR-4447, hsa-miR-4449, hsa-miR-4451, hsa-miR-4454, hsa-miR-4456, hsa-miR-4458, hsa-miR-378h, hsa-miR-3135b, hsa-miR-4463, hsa-miR-4466, hsa-miR-4472, hsa-miR-4474-3p, hsa-miR-4478, hsa-miR-3689f, hsa-miR-3155b, hsa-miR-4480, hsa-miR-4481, hsa-miR-4487, hsa-miR-4488, hsa-miR-4489, hsa-miR-4492, hsa-miR-4498, hsa-miR-4499, hsa-miR-4505, hsa-miR-4506, hsa-miR-4507, hsa-miR-4508, hsa-miR-4516, hsa-miR-4520-3p, hsa-miR-4525, hsa-miR-4534, hsa-miR-4535, hsa-miR-4538, hsa-miR-4540, hsa-miR-3127-3p, hsa-miR-3162-3p, hsa-miR-3619-3p, hsa-miR-3691-3p, hsa-miR-3150b-5p, hsa-miR-3922-5p, hsa-miR-3925-3p, hsa-miR-3940-5p, hsa-miR-3960, hsa-miR-4634, hsa-miR-4638-5p, hsa-miR-4640-3p, hsa-miR-4644, hsa-miR-4647, hsa-miR-4648, hsa-miR-4649-5p, hsa-miR-4649-3p, hsa-miR-4650-3p, hsa-miR-4651, hsa-miR-4654, hsa-miR-4656, hsa-miR-4657, hsa-miR-4665-5p, hsa-miR-4665-3p, hsa-miR-4667-5p, hsa-miR-4667-3p, hsa-miR-4670-5p, hsa-miR-4672, hsa-miR-4674, hsa-miR-4676-5p, hsa-miR-4677-5p, hsa-miR-4681, hsa-miR-4682, hsa-miR-4685-5p, hsa-miR-4686, hsa-miR-4687-5p, hsa-miR-4687-3p, hsa-miR-4688, hsa-miR-4689, hsa-miR-4691-5p, hsa-miR-4691-3p, hsa-miR-4692, hsa-miR-4697-5p, hsa-miR-4700-5p, hsa-miR-4701-3p, hsa-miR-4703-5p, hsa-miR-4706, hsa-miR-4707-5p, hsa-miR-4708-5p, hsa-miR-4708-3p, hsa-miR-203b-5p, hsa-miR-4716-5p, hsa-miR-4720-5p, hsa-miR-4722-5p, hsa-miR-4723-5p, hsa-miR-4723-3p, hsa-miR-4724-5p, hsa-miR-4728-5p, hsa-miR-4728-3p, hsa-miR-4730, hsa-miR-4731-5p, hsa-miR-4731-3p, hsa-miR-4734, hsa-miR-4736, hsa-miR-4737, hsa-miR-3064-5p, hsa-miR-4738-3p, hsa-miR-4739, hsa-miR-4741, hsa-miR-122b-3p, hsa-miR-4747-5p, hsa-miR-4748, hsa-miR-4749-5p, hsa-miR-4749-3p, hsa-miR-371b-5p, hsa-miR-371b-3p, hsa-miR-4754, hsa-miR-4755-3p, hsa-miR-499b-3p, hsa-miR-4756-5p, hsa-miR-4758-5p, hsa-miR-4758-3p, hsa-miR-4763-5p, hsa-miR-4763-3p, hsa-miR-4769-5p, hsa-miR-4769-3p, hsa-miR-4771, hsa-miR-4776-3p, hsa-miR-4778-5p, hsa-miR-4779, hsa-miR-4436b-3p, hsa-miR-4781-5p, hsa-miR-4783-3p, hsa-miR-4784, hsa-miR-2467-3p, hsa-miR-4787-5p, hsa-miR-4788, hsa-miR-4795-3p, hsa-miR-642a-3p, hsa-miR-550a-3-5p, hsa-miR-4433a-5p, hsa-miR-4999-5p, hsa-miR-5000-3p, hsa-miR-5002-3p, hsa-miR-5003-3p, hsa-miR-5004-5p, hsa-miR-5006-5p, hsa-miR-5006-3p, hsa-miR-5009-3p, hsa-miR-5087, hsa-miR-5093, hsa-miR-5188, hsa-miR-5189-5p, hsa-miR-5194, hsa-miR-5195-3p, hsa-miR-5196-5p, hsa-miR-4524b-5p, hsa-miR-548at-5p, hsa-miR-5588-5p, hsa-miR-5684, hsa-miR-5690, hsa-miR-5703, hsa-miR-5708, hsa-miR-197-5p, hsa-miR-211-3p, hsa-miR-301a-5p, hsa-miR-1185-2-3p, hsa-miR-766-5p, hsa-miR-1304-3p, hsa-miR-3184-3p, hsa-miR-1185-1-3p, hsa-miR-381-5p, hsa-miR-758-5p, hsa-miR-1227-5p, hsa-miR-1229-5p, hsa-miR-1233-5p, hsa-miR-1237-5p, hsa-miR-1238-5p, hsa-miR-3617-3p, hsa-miR-3620-5p, hsa-miR-3934-3p, hsa-miR-4632-5p, hsa-miR-4743-3p, hsa-miR-4750-3p, hsa-miR-5739, hsa-miR-5787, hsa-miR-6068, hsa-miR-6069, hsa-miR-6076, hsa-miR-6081, hsa-miR-6083, hsa-miR-6085, hsa-miR-6086, hsa-miR-6088, hsa-miR-6089, hsa-miR-6090, hsa-miR-6124, hsa-miR-6125, hsa-miR-378j, hsa-miR-6129, hsa-miR-6130, hsa-miR-6132, hsa-miR-6165, hsa-miR-6500-5p, hsa-miR-6500-3p, hsa-miR-6501-3p, hsa-miR-6506-5p, hsa-miR-6506-3p, hsa-miR-6510-5p, hsa-miR-6512-3p, hsa-miR-6513-5p, hsa-miR-6515-5p, hsa-miR-6515-3p, hsa-miR-6715b-3p, hsa-miR-6716-5p, hsa-miR-6717-5p, hsa-miR-6511b-3p, hsa-miR-6719-3p, hsa-miR-6721-5p, hsa-miR-6722-3p, hsa-miR-6724-5p, hsa-miR-210-5p, hsa-miR-328-5p, hsa-miR-410-5p, hsa-miR-489-5p, hsa-miR-494-5p, hsa-miR-598-5p, hsa-miR-605-3p, hsa-miR-891a-3p, hsa-miR-874-5p, hsa-miR-1343-5p, hsa-miR-5189-3p, hsa-miR-6727-5p, hsa-miR-6729-5p, hsa-miR-6729-3p, hsa-miR-6730-5p, hsa-miR-6731-3p, hsa-miR-6734-3p, hsa-miR-6737-5p, hsa-miR-6738-5p, hsa-miR-6740-5p, hsa-miR-6741-5p, hsa-miR-6742-5p, hsa-miR-6743-5p, hsa-miR-6745, hsa-miR-6746-5p, hsa-miR-6747-5p, hsa-miR-6749-5p, hsa-miR-6749-3p, hsa-miR-6750-5p, hsa-miR-6752-5p, hsa-miR-6752-3p, hsa-miR-6756-5p, hsa-miR-6756-3p, hsa-miR-6757-5p, hsa-miR-6759-3p, hsa-miR-6760-5p, hsa-miR-6760-3p, hsa-miR-6762-3p, hsa-miR-6763-3p, hsa-miR-6765-5p, hsa-miR-6766-3p, hsa-miR-6767-5p, hsa-miR-6768-5p, hsa-miR-6769a-5p, hsa-miR-6771-5p, hsa-miR-6772-5p, hsa-miR-6775-5p, hsa-miR-6775-3p, hsa-miR-6776-5p, hsa-miR-6777-5p, hsa-miR-6777-3p, hsa-miR-6779-5p, hsa-miR-6781-5p, hsa-miR-6782-3p, hsa-miR-6783-5p, hsa-miR-6784-5p, hsa-miR-6784-3p, hsa-miR-6785-5p, hsa-miR-6785-3p, hsa-miR-6786-5p, hsa-miR-6787-5p, hsa-miR-6788-5p, hsa-miR-6788-3p, hsa-miR-6789-5p, hsa-miR-6790-5p, hsa-miR-6790-3p, hsa-miR-6791-5p, hsa-miR-6792-3p, hsa-miR-6793-5p, hsa-miR-6793-3p, hsa-miR-6794-5p, hsa-miR-6794-3p, hsa-miR-6795-5p, hsa-miR-6795-3p, hsa-miR-6796-3p, hsa-miR-6797-5p, hsa-miR-6797-3p, hsa-miR-6798-5p, hsa-miR-6798-3p, hsa-miR-6799-5p, hsa-miR-6800-5p, hsa-miR-6800-3p, hsa-miR-6801-5p, hsa-miR-6801-3p, hsa-miR-6802-3p, hsa-miR-6803-5p, hsa-miR-6805-5p, hsa-miR-6805-3p, hsa-miR-6807-5p, hsa-miR-6808-3p, hsa-miR-6809-5p, hsa-miR-6810-3p, hsa-miR-6812-5p, hsa-miR-6812-3p, hsa-miR-6813-3p, hsa-miR-6815-5p, hsa-miR-6816-5p, hsa-miR-6819-3p, hsa-miR-6821-5p, hsa-miR-6822-3p, hsa-miR-6823-5p, hsa-miR-6826-3p, hsa-miR-6829-5p, hsa-miR-6831-5p, hsa-miR-6832-5p, hsa-miR-6834-5p, hsa-miR-6836-3p, hsa-miR-6837-5p, hsa-miR-6838-5p, hsa-miR-6839-5p, hsa-miR-6841-3p, hsa-miR-6845-5p, hsa-miR-6845-3p, hsa-miR-6846-3p, hsa-miR-6847-5p, hsa-miR-6848-5p, hsa-miR-6848-3p, hsa-miR-6850-5p, hsa-miR-6851-5p, hsa-miR-6853-5p, hsa-miR-6855-5p, hsa-miR-6855-3p, hsa-miR-6858-5p, hsa-miR-6858-3p, hsa-miR-6859-5p, hsa-miR-6859-3p, hsa-miR-6769b-5p, hsa-miR-6769b-3p, hsa-miR-6861-3p, hsa-miR-6865-3p, hsa-miR-6868-3p, hsa-miR-6869-5p, hsa-miR-6870-3p, hsa-miR-6871-5p, hsa-miR-6875-5p, hsa-miR-6876-5p, hsa-miR-6876-3p, hsa-miR-6880-3p, hsa-miR-6884-3p, hsa-miR-6885-3p, hsa-miR-6887-5p, hsa-miR-6887-3p, hsa-miR-6889-3p, hsa-miR-6891-5p, hsa-miR-6891-3p, hsa-miR-6892-5p, hsa-miR-6892-3p, hsa-miR-7106-5p, hsa-miR-7107-5p, hsa-miR-7108-5p, hsa-miR-7108-3p, hsa-miR-7109-5p, hsa-miR-7111-Sp, hsa-miR-7111-3p, hsa-miR-7113-5p, hsa-miR-7113-3p, hsa-miR-7114-3p, hsa-miR-7150, hsa-miR-7151-3p, hsa-miR-7157-3p, hsa-miR-7159-5p, hsa-miR-7160-5p, hsa-miR-7161-5p, hsa-miR-7162-5p, hsa-miR-7515, hsa-miR-7704, hsa-miR-4433b-5p, hsa-miR-4433b-3p, hsa-miR-7845-5p, hsa-miR-7847-3p, hsa-miR-7850-5p, hsa-miR-7851-3p, hsa-miR-7856-5p, hsa-miR-8054, hsa-miR-8057, hsa-miR-8064, hsa-miR-8069, hsa-miR-8070, hsa-miR-8071, hsa-miR-8072, hsa-miR-8074, hsa-miR-8075, hsa-miR-8077, hsa-miR-8078, hsa-miR-8079, hsa-miR-8080, hsa-miR-128-2-5p, hsa-miR-7976, hsa-miR-203a-5p, hsa-miR-1-5p, hsa-miR-1249-5p, hsa-miR-135a-2-3p, hsa-miR-498-3p, hsa-miR-9718, hsa-miR-9898, hsa-miR-9986, hsa-miR-10392 -3p, hsa-miR-10396a-5p, hsa-miR-10396a-3p, hsa-miR-10400-5p, hsa-miR-10400-3p, hsa-miR-10401-5p, hsa-miR-10396b-5p, hsa-miR-10522-5p, hsa-miR-10526-3p, hsa-miR-11181-3p, hsa-miR-11399, hsa-miR-11401, hsa-miR-3059-3p, hsa-miR-3085-3p, hsa-miR-6529-5p, hsa-miR-12114, hsa-miR-12116, hsa-miR-12118, hsa-miR-12120, hsa-miR-12122, and hsa-miR-12124. In the present disclosure, the extract of urine may be an extract of the urine of a stage-I pancreatic cancer patient. Only one of these microRNAs can be used to predict whether a subject from which the urine is derived is a pancreatic cancer patient (particularly stage-I), and 5 or more types, 10 or more types, 15 or more types, or 20 or more types of these microRNAs can be used in combination to predict whether a subject from which the urine is derived is a pancreatic cancer patient (particularly stage-I). Among these microRNAs, a microRNA that may be highly expressed in a pancreatic cancer patient (particularly stage-I) can be preferably used for prediction.

The present disclosure provides an extract of urine containing at least one type, two types, three types, four types, five types, six types, seven types, eight types, nine types, or ten types of or all microRNAs selected from the group consisting of microRNAs having an accuracy of higher than 0.500 among the microRNAs listed in Table 24-6. In the present disclosure, the extract of urine may be an extract of the urine of a pancreatic cancer patient. Only one of these microRNAs can be used to predict whether a subject from which the urine is derived is a pancreatic cancer patient, and 5 or more types, 10 or more types, 15 or more types, or 20 or more types of these microRNAs can be used in combination to predict whether a subject from which the urine is derived is a pancreatic cancer patient. Among these microRNAs, a microRNA that may be highly expressed in a pancreatic cancer patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

The present disclosure provides an extract of urine containing at least one type, two types, three types, four types, five types, six types, seven types, eight types, nine types, or ten types of or all microRNAs selected from the group consisting of microRNAs having an accuracy of higher than 0.500 among the microRNAs listed in Table 25-6. In the present disclosure, the extract of urine may be an extract of the urine of a pancreatic cancer patient. Only one of these microRNAs can be used to predict whether a subject from which the urine is derived is a pancreatic cancer patient, and 5 or more types, 10 or more types, 15 or more types, or 20 or more types of these microRNAs can be used in combination to predict whether a subject from which the urine is derived is a pancreatic cancer patient. Among these microRNAs, a microRNA that may be highly expressed in a pancreatic cancer patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

The present disclosure provides an extract of urine containing at least one type, two types, three types, four types, five types, six types, seven types, eight types, nine types, or ten types of or all microRNAs selected from the microRNAs listed in Table 4-7. The present disclosure also provides an extract of urine containing at least one type, two types, three types, four types, five types, six types, seven types, eight types, nine types, or ten types of or all microRNAs selected from the group consisting of hsa-miR-629-5p, hsa-miR-7851-3p, hsa-miR-3124-5p, hsa-miR-605-3p, hsa-miR-6788-5p, hsa-miR-551b-5p, hsa-miR-8077, hsa-miR-138-5p, hsa-miR-92a-2-5p, hsa-miR-12122, hsa-miR-135a-2-3p, hsa-miR-498-5p, hsa-miR-6783-5p, hsa-miR-4755-3p, hsa-miR-6081, hsa-miR-3648, hsa-miR-4756-3p, hsa-miR-10522-5p, hsa-miR-5685, hsa-miR-4741, hsa-miR-1268b, hsa-miR-1303, hsa-miR-5196-5p, hsa-miR-6871-5p, hsa-miR-6815-5p, hsa-miR-6750-5p, hsa-miR-4429, hsa-miR-492, hsa-miR-4673, hsa-miR-769-3p, hsa-miR-150-3p, hsa-miR-193a-5p, hsa-miR-3158-3p, hsa-miR-105-5p, hsa-miR-4487, hsa-miR-1914-3p, hsa-miR-6836-3p, hsa-miR-2276-3p, hsa-miR-7113-5p, hsa-miR-6074, hsa-miR-6776-5p, hsa-miR-4651, hsa-miR-7111-5p, hsa-miR-10526-3p, hsa-miR-4321, hsa-miR-323a-5p, hsa-miR-4474-3p, hsa-miR-4506, hsa-miR-4514, hsa-miR-645, hsa-miR-6777-5p, hsa-miR-4727-3p, hsa-miR-4701-3p, hsa-miR-4648, hsa-miR-8070, hsa-miR-6781-5p, hsa-miR-12120, hsa-miR-4436b-3p, hsa-miR-5009-3p, hsa-miR-3122, hsa-miR-1293, hsa-miR-513b-5p, hsa-miR-4647, hsa-miR-541-3p, hsa-miR-601, hsa-miR-4498, hsa-miR-4540, hsa-miR-6876-5p, hsa-miR-10392-3p, hsa-miR-3187-3p, hsa-miR-3652, hsa-miR-6719-3p, hsa-miR-6843-3p, hsa-miR-12114, hsa-miR-4535, hsa-miR-575, hsa-miR-7151-3p, hsa-miR-3689d, hsa-miR-4758-5p, hsa-miR-6809-5p, hsa-miR-3200-5p, hsa-miR-6793-5p, hsa-miR-3622a-5p, hsa-miR-449c-5p, hsa-miR-4638-5p, hsa-miR-1343-5p, hsa-miR-6820-5p, hsa-miR-10401-5p, hsa-miR-3620-5p, hsa-miR-4538, hsa-miR-6855-5p, hsa-miR-4665-5p, hsa-miR-6717-5p, hsa-miR-6822-3p, hsa-miR-4784, hsa-miR-6125, hsa-miR-4435, hsa-miR-4314, hsa-miR-4421, hsa-miR-18a-3p, hsa-miR-11399, hsa-miR-6131, hsa-miR-3612, hsa-miR-2467-3p, hsa-miR-7156-3p, hsa-miR-184, hsa-miR-4261, hsa-miR-4785, hsa-miR-3940-5p, hsa-miR-3689b-3p, hsa-miR-3689c, hsa-miR-6817-5p, hsa-miR-4516, hsa-miR-7160-5p, hsa-miR-6893-5p, hsa-miR-7162-3p, hsa-miR-6859-5p, hsa-miR-211-3p, hsa-miR-6868-3p, hsa-miR-3150b-3p, hsa-miR-5703, hsa-miR-6127, hsa-miR-4534, hsa-miR-595, hsa-miR-378a-3p, hsa-miR-614, hsa-miR-762, hsa-miR-6849-5p, hsa-miR-4482-3p, hsa-miR-4800-5p, hsa-miR-6511b-5p, hsa-miR-4736, hsa-miR-5189-3p, hsa-miR-6845-5p, hsa-miR-4682, hsa-miR-4769-5p, hsa-miR-3619-5p, hsa-miR-638, hsa-miR-6791-5p, hsa-miR-6089, hsa-miR-3173-3p, hsa-miR-6796-3p, hsa-miR-3155b, hsa-miR-668-5p, hsa-miR-1250-5p, hsa-miR-10400-3p, hsa-miR-6068, hsa-miR-5708, hsa-miR-4684-3p, hsa-miR-6770-5p, hsa-miR-9718, hsa-miR-4327, hsa-miR-6743-5p, hsa-miR-92b-5p, hsa-let-7d-3p, hsa-miR-4444, hsa-miR-4708-3p, hsa-miR-6850-5p, hsa-miR-6752-5p, hsa-miR-3187-5p, hsa-miR-3621, hsa-miR-6499-5p, hsa-miR-7107-5p, hsa-miR-194-3p, hsa-miR-1843, hsa-miR-183-3p, hsa-miR-7106-5p, hsa-miR-1471, hsa-miR-3190-3p, hsa-miR-6762-3p, hsa-miR-6895-5p, hsa-miR-6767-5p, hsa-miR-3185, hsa-miR-3059-3p, hsa-miR-5571-3p, hsa-miR-6763-5p, hsa-miR-550a-5p, hsa-miR-8078, hsa-miR-5002-3p, hsa-miR-6761-5p, hsa-miR-1908-5p, hsa-miR-3937, hsa-miR-644a, hsa-miR-193b-5p, hsa-miR-135a-3p, hsa-miR-665, hsa-miR-6773-3p, hsa-miR-4481, hsa-miR-185-3p, hsa-miR-6889-5p, hsa-miR-4306, hsa-miR-1587, hsa-miR-6762-5p, hsa-miR-6789-5p, hsa-miR-939-5p, hsa-miR-3713, hsa-miR-106a-3p, hsa-miR-5589-5p, hsa-miR-3197, hsa-miR-6816-3p, hsa-miR-6794-5p, hsa-miR-6772-5p, hsa-miR-1268a, hsa-miR-494-3p, hsa-miR-214-3p, hsa-miR-6782-5p, hsa-miR-6730-5p, hsa-miR-6808-3p, hsa-miR-608, hsa-miR-3689a-3p, hsa-miR-1306-3p, hsa-miR-891a-5p, hsa-miR-4738-3p, hsa-miR-5585-3p, hsa-miR-632, hsa-miR-6831-5p, hsa-miR-4449, hsa-miR-937-5p, hsa-miR-1227-5p, hsa-miR-4529-5p, hsa-miR-4315, hsa-miR-3065-3p, hsa-miR-1207-5p, hsa-miR-3666, hsa-miR-3922-5p, hsa-miR-10396a-3p, hsa-miR-4440, hsa-miR-4776-3p, hsa-miR-4745-5p, hsa-miR-1469, hsa-miR-4743-5p, hsa-miR-4652-5p, hsa-miR-4526, hsa-miR-766-5p, hsa-miR-3120-5p, hsa-miR-8072, hsa-miR-5006-5p, hsa-miR-504-3p, hsa-miR-4695-5p, hsa-miR-6745, hsa-miR-6515-5p, hsa-miR-486-3p, hsa-miR-4428, hsa-miR-4520-5p, hsa-miR-4456, hsa-miR-4669, hsa-miR-4499, hsa-miR-4658, hsa-miR-4251, hsa-miR-6790-5p, hsa-miR-371a-3p, hsa-miR-5588-3p, hsa-miR-3178, hsa-miR-10396a-5p, hsa-miR-501-5p, hsa-miR-5580-3p, hsa-miR-3917, hsa-miR-542-5p, hsa-miR-3665, hsa-miR-99b-3p, hsa-miR-6830-5p, hsa-miR-4455, hsa-miR-4288, hsa-miR-4734, hsa-miR-6805-5p, hsa-miR-3928-3p, hsa-miR-5572, hsa-miR-3922-3p, hsa-miR-6895-3p, hsa-miR-4776-5p, hsa-miR-487a-5p, hsa-miR-6504-3p, hsa-miR-3199, hsa-miR-520b-3p, hsa-miR-3126-3p, hsa-miR-6740-5p, hsa-miR-6805-3p, hsa-miR-6837-5p, hsa-miR-6796-5p, hsa-miR-4460, hsa-miR-12116, hsa-miR-6836-5p, hsa-miR-4497, hsa-miR-3677-3p, hsa-miR-593-3p, hsa-miR-4466, hsa-miR-139-3p, hsa-miR-3131, hsa-miR-650, hsa-miR-3935, hsa-miR-6512-3p, hsa-miR-1237-5p, hsa-miR-1912-5p, hsa-miR-584-3p, hsa-miR-25-5p, hsa-miR-676-5p, hsa-miR-29a-3p, hsa-miR-6772-3p, hsa-miR-3610, hsa-miR-4707-5p, hsa-miR-3660, hsa-miR-3944-5p, hsa-miR-885-3p, hsa-miR-8075, hsa-miR-4711-3p, hsa-miR-6823-5p, hsa-miR-9902, hsa-miR-4530, hsa-miR-4259, hsa-miR-658, hsa-miR-4749-5p, hsa-miR-5090, hsa-miR-3177-3p, hsa-miR-6886-5p, hsa-miR-6792-5p, hsa-miR-6718-5p, hsa-miR-146b-3p, hsa-miR-10392-5p, hsa-miR-1292-5p, hsa-miR-520e-3p, hsa-miR-370-3p, hsa-miR-1251-3p, hsa-miR-4448, hsa-miR-11181-3p, hsa-miR-3186-5p, hsa-miR-4484, hsa-miR-4446-3p, hsa-miR-8064, hsa-miR-6834-5p, hsa-miR-3679-3p, hsa-miR-4686, hsa-miR-6076, hsa-miR-204-3p, hsa-miR-4740-5p, hsa-miR-3164, hsa-miR-6722-3p, hsa-miR-6766-5p, hsa-miR-1224-5p, hsa-miR-3135b, hsa-miR-6784-5p, hsa-miR-6852-5p, hsa-miR-4433b-3p, hsa-miR-3160-5p, hsa-miR-3918, hsa-miR-887-3p, hsa-miR-1226-5p, hsa-miR-4430, hsa-miR-4746-3p, hsa-miR-670-5p, hsa-miR-6751-5p, hsa-miR-4635, hsa-miR-6877-5p, hsa-miR-6737-5p, hsa-miR-187-5p, hsa-miR-4322, hsa-miR-3125, hsa-miR-6506-5p, hsa-miR-1915-3p, hsa-miR-3155a, hsa-miR-6872-5p, hsa-miR-3655, hsa-miR-3173-5p, hsa-miR-583, hsa-miR-3154, hsa-miR-4716-3p, hsa-miR-4433a-3p, hsa-miR-4754, hsa-miR-411-3p, hsa-miR-4531, hsa-miR-3925-5p, hsa-miR-1236-5p, hsa-miR-6072, hsa-miR-493-3p, hsa-miR-4793-3p, hsa-miR-5189-5p, hsa-miR-6499-3p, hsa-miR-518f-3p, hsa-miR-585-3p, hsa-miR-198, hsa-miR-1204, hsa-miR-7856-5p, hsa-miR-2113, hsa-miR-3196, hsa-miR-4700-5p, hsa-miR-4299, hsa-miR-6829-5p, hsa-miR-6501-5p, hsa-miR-138-1-3p, hsa-miR-589-3p, hsa-miR-6083, hsa-miR-1273h-5p, hsa-miR-615-5p, hsa-miR-6515-3p, hsa-miR-6862-5p, hsa-miR-6774-3p, hsa-miR-6080, hsa-miR-8071, hsa-miR-4507, hsa-miR-5584-3p, hsa-miR-8063, hsa-miR-5705, hsa-miR-1909-3p, hsa-miR-4441, hsa-miR-6090, hsa-miR-342-5p, hsa-miR-4726-3p, hsa-miR-7976, hsa-miR-6789-3p, hsa-miR-4476, hsa-miR-3180-3p, hsa-miR-654-5p, hsa-miR-4750-5p, hsa-miR-4479, hsa-miR-4294, hsa-miR-1285-5p, hsa-miR-222-5p, hsa-miR-6859-3p, hsa-miR-3132, hsa-miR-892b, hsa-miR-4657, hsa-miR-6821-5p, hsa-miR-1468-5p, hsa-miR-4674, hsa-miR-491-5p, hsa-miR-4521, hsa-miR-4316, hsa-miR-11400, hsa-miR-6833-5p, hsa-miR-624-5p, hsa-miR-4763-3p, hsa-miR-6753-5p, hsa-miR-4258, hsa-miR-30c-2-3p, hsa-miR-7152-5p, hsa-miR-711, hsa-miR-3934-5p, hsa-miR-6734-5p, hsa-miR-6842-5p, hsa-miR-6768-5p, hsa-miR-6749-5p, hsa-miR-11401, hsa-miR-6816-5p, hsa-miR-200c-5p, hsa-miR-422a, hsa-miR-6085, hsa-miR-4709-5p, hsa-miR-103a-1-5p, hsa-miR-3085-5p, hsa-miR-1301-5p, hsa-miR-4486, hsa-miR-2110, hsa-miR-6770-3p, hsa-miR-6856-3p, hsa-miR-12117, hsa-miR-718, hsa-miR-574-5p, hsa-miR-671-3p, hsa-miR-323a-3p, hsa-miR-488-5p, hsa-miR-4640-5p, hsa-miR-4513, hsa-miR-519c-5p, hsa-miR-523-5p, hsa-miR-518e-5p, hsa-miR-522-5p, hsa-miR-519a-5p, hsa-miR-519b-5p, hsa-miR-1972, hsa-miR-3685, hsa-miR-6873-5p, hsa-miR-4437, hsa-miR-3162-5p, hsa-miR-1296-3p, hsa-miR-4483, hsa-miR-3186-3p, hsa-miR-4691-5p, hsa-miR-564, and hsa-miR-548q. In the present disclosure, the extract of urine may be an extract of the urine of a prostate cancer patient. Only one of these microRNAs can be used to predict whether a subject from which the urine is derived is a prostate cancer patient, and 5 or more types, 10 or more types, 15 or more types, or 20 or more types of these microRNAs can be used in combination to predict whether a subject from which the urine is derived is a prostate cancer patient. Among these microRNAs, a microRNA that may be highly expressed in a prostate cancer patient can be preferably used for prediction.

The present disclosure provides an extract of urine containing at least one type, two types, three types, four types, five types, six types, seven types, eight types, nine types, or ten types of or all microRNAs selected from the group consisting of hsa-miR-28-5p, hsa-miR-29a-3p, hsa-miR-93-5p, hsa-miR-105-5p, hsa-miR-198, hsa-miR-129-5p, hsa-miR-134-5p, hsa-miR-184, hsa-miR-185-5p, hsa-miR-296-5p, hsa-miR-130b-3p, hsa-miR-302c-5p, hsa-miR-370-3p, hsa-miR-378a-3p, hsa-miR-20b-5p, hsa-miR-429, hsa-miR-485-5p, hsa-miR-491-5p, hsa-miR-492, hsa-miR-494-3p, hsa-miR-512-5p, hsa-miR-498-5p, hsa-miR-520e-3p, hsa-miR-519b-3p, hsa-miR-525-5p, hsa-miR-518f-3p, hsa-miR-520b-3p, hsa-miR-518b, hsa-miR-520c-3p, hsa-miR-524-5p, hsa-miR-520d-5p, hsa-miR-501-5p, hsa-miR-513a-5p, hsa-miR-572, hsa-miR-575, hsa-miR-580-3p, hsa-miR-583, hsa-miR-584-5p, hsa-miR-585-3p, hsa-miR-589-3p, hsa-miR-601, hsa-miR-608, hsa-miR-614, hsa-miR-615-3p, hsa-miR-622, hsa-miR-624-5p, hsa-miR-635, hsa-miR-638, hsa-miR-644a, hsa-miR-650, hsa-miR-663a, hsa-miR-654-5p, hsa-miR-658, hsa-miR-542-5p, hsa-miR-769-3p, hsa-miR-770-5p, hsa-let-7d-3p, hsa-miR-25-5p, hsa-miR-29a-5p, hsa-miR-92a-2-5p, hsa-miR-30c-2-3p, hsa-miR-139-3p, hsa-miR-181a-2-3p, hsa-miR-183-3p, hsa-miR-187-5p, hsa-miR-214-5p, hsa-miR-30b-3p, hsa-miR-135a-3p, hsa-miR-125a-3p, hsa-miR-149-3p, hsa-miR-150-3p, hsa-miR-185-3p, hsa-miR-193a-5p, hsa-miR-194-3p, hsa-miR-34c-3p, hsa-miR-296-3p, hsa-miR-361-3p, hsa-miR-302d-5p, hsa-miR-374a-3p, hsa-miR-342-5p, hsa-miR-323a-5p, hsa-miR-338-5p, hsa-miR-339-3p, hsa-miR-424-3p, hsa-miR-18b-3p, hsa-miR-20b-3p, hsa-miR-483-5p, hsa-miR-486-3p, hsa-miR-193b-5p, hsa-miR-505-5p, hsa-miR-92b-5p, hsa-miR-551b-5p, hsa-miR-574-5p, hsa-miR-550a-5p, hsa-miR-615-5p, hsa-miR-625-3p, hsa-miR-629-5p, hsa-miR-411-3p, hsa-miR-541-3p, hsa-miR-885-5p, hsa-miR-885-3p, hsa-miR-877-5p, hsa-miR-887-3p, hsa-miR-665, hsa-miR-760, hsa-miR-920, hsa-miR-933, hsa-miR-936, hsa-miR-939-5p, hsa-miR-940, hsa-miR-1224-5p, hsa-miR-1224-3p, hsa-miR-1225-5p, hsa-miR-1225-3p, hsa-miR-1226-5p, hsa-miR-1228-3p, hsa-miR-1231, hsa-miR-1237-3p, hsa-miR-1238-3p, hsa-miR-1301-3p, hsa-miR-1182, hsa-miR-1184, hsa-miR-1203, hsa-miR-1204, hsa-miR-1207-5p, hsa-miR-1289, hsa-miR-1291, hsa-miR-1303, hsa-miR-1249-3p, hsa-miR-1250-5p, hsa-miR-1263, hsa-miR-1268a, hsa-miR-1292-5p, hsa-miR-1307-3p, hsa-miR-1324, hsa-miR-1469, hsa-miR-1470, hsa-miR-1471, hsa-miR-1538, hsa-miR-1908-5p, hsa-miR-1909-5p, hsa-miR-1909-3p, hsa-miR-1913, hsa-miR-1914-3p, hsa-miR-1915-3p, hsa-miR-103a-2-5p, hsa-miR-365a-5p, hsa-miR-196b-3p, hsa-miR-1972, hsa-miR-2110, hsa-miR-449c-5p, hsa-miR-762, hsa-miR-670-5p, hsa-miR-2116-5p, hsa-miR-548q, hsa-miR-2276-3p, hsa-miR-2278, hsa-miR-711, hsa-miR-718, hsa-miR-2681-5p, hsa-miR-2909, hsa-miR-3116, hsa-miR-3122, hsa-miR-3124-5p, hsa-miR-3126-5p, hsa-miR-3130-5p, hsa-miR-3131, hsa-miR-3132, hsa-miR-3137, hsa-miR-3138, hsa-miR-3147, hsa-miR-3150a-3p, hsa-miR-3151-5p, hsa-miR-3153, hsa-miR-3154, hsa-miR-3155a, hsa-miR-3156-5p, hsa-miR-3158-3p, hsa-miR-3162-5p, hsa-miR-3164, hsa-miR-3170, hsa-miR-3173-3p, hsa-miR-1193, hsa-miR-3176, hsa-miR-3177-3p, hsa-miR-3178, hsa-miR-3180-3p, hsa-miR-3185, hsa-miR-3186-3p, hsa-miR-3187-3p, hsa-miR-3189-3p, hsa-miR-3191-3p, hsa-miR-3195, hsa-miR-3196, hsa-miR-3197, hsa-miR-3199, hsa-miR-4296, hsa-miR-4297, hsa-miR-4294, hsa-miR-4299, hsa-miR-4300, hsa-miR-4306, hsa-miR-4308, hsa-miR-4311, hsa-miR-4313, hsa-miR-4315, hsa-miR-4316, hsa-miR-4314, hsa-miR-4322, hsa-miR-4321, hsa-miR-4323, hsa-miR-4257, hsa-miR-4258, hsa-miR-4259, hsa-miR-4260, hsa-miR-4253, hsa-miR-4251, hsa-miR-4326, hsa-miR-4327, hsa-miR-4261, hsa-miR-4265, hsa-miR-4269, hsa-miR-4274, hsa-miR-4283, hsa-miR-4284, hsa-miR-4286, hsa-miR-4328, hsa-miR-3200-5p, hsa-miR-3610, hsa-miR-3612, hsa-miR-3614-5p, hsa-miR-3616-3p, hsa-miR-3619-5p, hsa-miR-3621, hsa-miR-3622a-5p, hsa-miR-3622a-3p, hsa-miR-3622b-5p, hsa-miR-3648, hsa-miR-3649, hsa-miR-3651, hsa-miR-3652, hsa-miR-3660, hsa-miR-3663-5p, hsa-miR-3665, hsa-miR-3666, hsa-miR-3667-5p, hsa-miR-3667-3p, hsa-miR-3675-3p, hsa-miR-3677-3p, hsa-miR-3679-5p, hsa-miR-3679-3p, hsa-miR-3909, hsa-miR-3917, hsa-miR-3918, hsa-miR-3925-5p, hsa-miR-3928-3p, hsa-miR-3937, hsa-miR-3943, hsa-miR-3944-3p, hsa-miR-3945, hsa-miR-1268b, hsa-miR-4421, hsa-miR-4428, hsa-miR-4429, hsa-miR-4430, hsa-miR-4433a-3p, hsa-miR-4435, hsa-miR-4439, hsa-miR-4440, hsa-miR-4441, hsa-miR-4444, hsa-miR-4446-3p, hsa-miR-4447, hsa-miR-4448, hsa-miR-4449, hsa-miR-4455, hsa-miR-4456, hsa-miR-4460, hsa-miR-3135b, hsa-miR-4463, hsa-miR-4465, hsa-miR-4466, hsa-miR-4467, hsa-miR-4470, hsa-miR-4472, hsa-miR-4476, hsa-miR-4479, hsa-miR-4481, hsa-miR-4483, hsa-miR-4484, hsa-miR-4486, hsa-miR-4487, hsa-miR-4488, hsa-miR-4489, hsa-miR-4496, hsa-miR-4497, hsa-miR-4498, hsa-miR-4499, hsa-miR-4505, hsa-miR-4506, hsa-miR-4507, hsa-miR-4508, hsa-miR-4512, hsa-miR-4513, hsa-miR-4514, hsa-miR-4516, hsa-miR-4518, hsa-miR-4519, hsa-miR-4521, hsa-miR-4525, hsa-miR-4526, hsa-miR-4530, hsa-miR-4531, hsa-miR-4533, hsa-miR-4534, hsa-miR-4535, hsa-miR-1587, hsa-miR-4538, hsa-miR-4539, hsa-miR-4540, hsa-miR-3127-3p, hsa-miR-3150a-5p, hsa-miR-3152-5p, hsa-miR-3158-5p, hsa-miR-3162-3p, hsa-miR-3173-5p, hsa-miR-3187-5p, hsa-miR-3619-3p, hsa-miR-3150b-5p, hsa-miR-3922-5p, hsa-miR-3940-5p, hsa-miR-4529-5p, hsa-miR-3960, hsa-miR-4635, hsa-miR-4638-5p, hsa-miR-4640-5p, hsa-miR-4648, hsa-miR-4651, hsa-miR-4652-5p, hsa-miR-4654, hsa-miR-4655-5p, hsa-miR-4656, hsa-miR-4658, hsa-miR-4664-5p, hsa-miR-4665-5p, hsa-miR-4665-3p, hsa-miR-4667-5p, hsa-miR-219b-5p, hsa-miR-4669, hsa-miR-4673, hsa-miR-4674, hsa-miR-4677-5p, hsa-miR-4684-3p, hsa-miR-1343-3p, hsa-miR-4688, hsa-miR-4691-5p, hsa-miR-4695-5p, hsa-miR-4701-3p, hsa-miR-4706, hsa-miR-4707-5p, hsa-miR-4707-3p, hsa-miR-4708-5p, hsa-miR-4708-3p, hsa-miR-4710, hsa-miR-4714-5p, hsa-miR-4716-5p, hsa-miR-4716-3p, hsa-miR-4717-5p, hsa-miR-4717-3p, hsa-miR-4721, hsa-miR-4723-5p, hsa-miR-4725-5p, hsa-miR-4725-3p, hsa-miR-4726-3p, hsa-miR-4727-3p, hsa-miR-4728-3p, hsa-miR-4731-3p, hsa-miR-4732-5p, hsa-miR-4733-3p, hsa-miR-4734, hsa-miR-4736, hsa-miR-4738-3p, hsa-miR-4740-5p, hsa-miR-4740-3p, hsa-miR-4741, hsa-miR-4743-5p, hsa-miR-4745-5p, hsa-miR-4746-3p, hsa-miR-4747-5p, hsa-miR-4748, hsa-miR-4749-5p, hsa-miR-4749-3p, hsa-miR-4750-5p, hsa-miR-4751, hsa-miR-371b-5p, hsa-miR-4754, hsa-miR-4755-3p, hsa-miR-4758-5p, hsa-miR-4763-3p, hsa-miR-4769-5p, hsa-miR-4776-5p, hsa-miR-4778-5p, hsa-miR-4783-3p, hsa-miR-4785, hsa-miR-2467-3p, hsa-miR-4793-3p, hsa-miR-4800-5p, hsa-miR-550a-3-5p, hsa-miR-4433a-5p, hsa-miR-4482-3p, hsa-miR-5001-3p, hsa-miR-5002-5p, hsa-miR-5002-3p, hsa-miR-5006-5p, hsa-miR-5006-3p, hsa-miR-5009-3p, hsa-miR-5010-5p, hsa-miR-5088-5p, hsa-miR-5090, hsa-miR-5093, hsa-miR-5189-5p, hsa-miR-5190, hsa-miR-5196-5p, hsa-miR-5571-3p, hsa-miR-5572, hsa-miR-5580-3p, hsa-miR-5585-3p, hsa-miR-5587-3p, hsa-miR-5589-5p, hsa-miR-5695, hsa-miR-5703, hsa-miR-5705, hsa-miR-197-5p, hsa-miR-204-3p, hsa-miR-211-3p, hsa-miR-345-3p, hsa-miR-766-5p, hsa-miR-873-3p, hsa-miR-1247-3p, hsa-miR-550b-2-5p, hsa-miR-365b-5p, hsa-miR-937-5p, hsa-miR-1227-5p, hsa-miR-1236-5p, hsa-miR-1237-5p, hsa-miR-1238-5p, hsa-miR-3620-5p, hsa-miR-4750-3p, hsa-miR-5739, hsa-miR-5787, hsa-miR-6068, hsa-miR-6074, hsa-miR-6075, hsa-miR-6076, hsa-miR-6081, hsa-miR-6084, hsa-miR-6085, hsa-miR-6086, hsa-miR-6089, hsa-miR-6090, hsa-miR-6124, hsa-miR-6125, hsa-miR-6127, hsa-miR-6130, hsa-miR-6131, hsa-miR-6133, hsa-miR-6165, hsa-miR-6500-3p, hsa-miR-6503-3p, hsa-miR-6511a-5p, hsa-miR-6515-5p, hsa-miR-6515-3p, hsa-miR-6717-5p, hsa-miR-6511b-5p, hsa-miR-6719-3p, hsa-miR-6721-5p, hsa-miR-6722-5p, hsa-miR-6722-3p, hsa-miR-6724-5p, hsa-miR-128-1-5p, hsa-miR-433-5p, hsa-miR-181d-3p, hsa-miR-504-3p, hsa-miR-487b-5p, hsa-miR-585-5p, hsa-miR-605-3p, hsa-miR-668-5p, hsa-miR-1296-3p, hsa-miR-1301-5p, hsa-miR-1251-3p, hsa-miR-1266-3p, hsa-miR-1343-5p, hsa-miR-6726-5p, hsa-miR-6727-5p, hsa-miR-6729-5p, hsa-miR-6729-3p, hsa-miR-6730-5p, hsa-miR-6731-5p, hsa-miR-6731-3p, hsa-miR-6732-5p, hsa-miR-6734-5p, hsa-miR-6737-5p, hsa-miR-6739-3p, hsa-miR-6740-5p, hsa-miR-6740-3p, hsa-miR-6741-5p, hsa-miR-6742-5p, hsa-miR-6743-5p, hsa-miR-6744-5p, hsa-miR-6745, hsa-miR-6746-5p, hsa-miR-6747-5p, hsa-miR-6748-5p, hsa-miR-6749-5p, hsa-miR-6750-5p, hsa-miR-6751-5p, hsa-miR-6752-5p, hsa-miR-6752-3p, hsa-miR-6753-5p, hsa-miR-6754-5p, hsa-miR-6756-3p, hsa-miR-6759-5p, hsa-miR-6760-5p, hsa-miR-6760-3p, hsa-miR-6762-5p, hsa-miR-6763-5p, hsa-miR-6763-3p, hsa-miR-6766-5p, hsa-miR-6766-3p, hsa-miR-6768-5p, hsa-miR-6769a-5p, hsa-miR-6770-5p, hsa-miR-6772-5p, hsa-miR-6772-3p, hsa-miR-6774-3p, hsa-miR-6776-5p, hsa-miR-6777-5p, hsa-miR-6777-3p, hsa-miR-6778-5p, hsa-miR-6781-5p, hsa-miR-6782-5p, hsa-miR-6783-5p, hsa-miR-6783-3p, hsa-miR-6784-5p, hsa-miR-6784-3p, hsa-miR-6785-3p, hsa-miR-6786-5p, hsa-miR-6787-5p, hsa-miR-6788-5p, hsa-miR-6789-5p, hsa-miR-6789-3p, hsa-miR-6790-3p, hsa-miR-6791-5p, hsa-miR-6792-5p, hsa-miR-6794-5p, hsa-miR-6795-5p, hsa-miR-6795-3p, hsa-miR-6796-5p, hsa-miR-6796-3p, hsa-miR-6797-5p, hsa-miR-6798-5p, hsa-miR-6798-3p, hsa-miR-6800-5p, hsa-miR-6800-3p, hsa-miR-6801-5p, hsa-miR-6805-5p, hsa-miR-6805-3p, hsa-miR-6806-5p, hsa-miR-6808-3p, hsa-miR-6809-5p, hsa-miR-6810-5p, hsa-miR-6810-3p, hsa-miR-6812-5p, hsa-miR-6812-3p, hsa-miR-6813-5p, hsa-miR-6813-3p, hsa-miR-6815-5p, hsa-miR-6816-5p, hsa-miR-6816-3p, hsa-miR-6819-3p, hsa-miR-6820-5p, hsa-miR-6821-5p, hsa-miR-6821-3p, hsa-miR-6822-5p, hsa-miR-6823-5p, hsa-miR-6825-5p, hsa-miR-6827-5p, hsa-miR-6828-5p, hsa-miR-6829-5p, hsa-miR-6830-5p, hsa-miR-6831-5p, hsa-miR-6832-5p, hsa-miR-6833-5p, hsa-miR-6834-5p, hsa-miR-6780b-5p, hsa-miR-6836-3p, hsa-miR-6840-3p, hsa-miR-6842-5p, hsa-miR-6845-5p, hsa-miR-6846-5p, hsa-miR-6848-5p, hsa-miR-6848-3p, hsa-miR-6849-5p, hsa-miR-6850-5p, hsa-miR-6853-5p, hsa-miR-6855-5p, hsa-miR-6856-5p, hsa-miR-6856-3p, hsa-miR-6857-5p, hsa-miR-6858-3p, hsa-miR-6859-3p, hsa-miR-6860, hsa-miR-6861-5p, hsa-miR-6862-5p, hsa-miR-6869-5p, hsa-miR-6870-3p, hsa-miR-6871-5p, hsa-miR-6872-5p, hsa-miR-6875-5p, hsa-miR-6875-3p, hsa-miR-6876-5p, hsa-miR-6877-5p, hsa-miR-6879-5p, hsa-miR-6880-3p, hsa-miR-6881-5p, hsa-miR-6882-3p, hsa-miR-6886-5p, hsa-miR-6887-5p, hsa-miR-6887-3p, hsa-miR-6889-5p, hsa-miR-6891-3p, hsa-miR-6892-5p, hsa-miR-6892-3p, hsa-miR-6893-5p, hsa-miR-6894-5p, hsa-miR-6895-5p, hsa-miR-7106-5p, hsa-miR-7107-5p, hsa-miR-7108-5p, hsa-miR-7108-3p, hsa-miR-7109-5p, hsa-miR-7111-5p, hsa-miR-7114-5p, hsa-miR-7114-3p, hsa-miR-7150, hsa-miR-7151-5p, hsa-miR-7152-5p, hsa-miR-7152-3p, hsa-miR-7156-3p, hsa-miR-7160-5p, hsa-miR-7704, hsa-miR-7843-5p, hsa-miR-4433b-5p, hsa-miR-4433b-3p, hsa-miR-1273h-5p, hsa-miR-7846-3p, hsa-miR-7848-3p, hsa-miR-7851-3p, hsa-miR-7855-5p, hsa-miR-8052, hsa-miR-8060, hsa-miR-8063, hsa-miR-8064, hsa-miR-8069, hsa-miR-8070, hsa-miR-8071, hsa-miR-8072, hsa-miR-8073, hsa-miR-8075, hsa-miR-8077, hsa-miR-8078, hsa-miR-8085, hsa-miR-8087, hsa-miR-8089, hsa-miR-7974, hsa-miR-7977, hsa-miR-8485, hsa-miR-103a-1-5p, hsa-miR-135a-2-3p, hsa-miR-526a-3p, hsa-miR-1912-5p, hsa-miR-9718, hsa-miR-9898, hsa-miR-10392-5p, hsa-miR-10392-3p, hsa-miR-10395-5p, hsa-miR-10396a-5p, hsa-miR-10396a-3p, hsa-miR-10398-3p, hsa-miR-10400-3p, hsa-miR-10401-5p, hsa-miR-10396b-5p, hsa-miR-10522-5p, hsa-miR-10526-3p, hsa-miR-11181-3p, hsa-miR-11399, hsa-miR-11400, hsa-miR-3059-3p, hsa-miR-3085-5p, hsa-miR-12114, hsa-miR-12115, hsa-miR-12116, hsa-miR-12118, hsa-miR-12119, hsa-miR-12120, hsa-miR-12122, hsa-miR-12126, and hsa-miR-12136. In the present disclosure, the extract of urine may be an extract of the urine of a stage-I or -II prostate cancer patient. Only one of these microRNAs can be used to predict whether a subject from which the urine is derived is a prostate cancer patient (particularly stage-I or -II), and 5 or more types, 10 or more types, 15 or more types, or 20 or more types of these microRNAs can be used in combination to predict whether a subject from which the urine is derived is a prostate cancer patient (particularly stage-I or -II). Among these microRNAs, a microRNA that may be highly expressed in a prostate cancer patient (particularly stage-I or -II) can be preferably used for prediction.

The present disclosure provides an extract of urine containing at least one type, two types, three types, four types, five types, six types, seven types, eight types, nine types, or ten types of or all microRNAs selected from the group consisting of hsa-miR-29a-3p, hsa-miR-105-5p, hsa-miR-129-5p, hsa-let-7i-5p, hsa-miR-133a-3p, hsa-miR-142-3p, hsa-miR-106b-5p, hsa-miR-130b-3p, hsa-miR-30e-5p, hsa-miR-365a-3p, hsa-miR-365b-3p, hsa-miR-371a-3p, hsa-miR-378a-3p, hsa-miR-135b-5p, hsa-miR-148b-3p, hsa-miR-492, hsa-miR-494-3p, hsa-miR-498-5p, hsa-miR-518b, hsa-miR-520c-3p, hsa-miR-584-5p, hsa-miR-585-3p, hsa-miR-590-5p, hsa-miR-601, hsa-miR-608, hsa-miR-614, hsa-miR-624-5p, hsa-miR-769-3p, hsa-let-7b-3p, hsa-let-7f-1-3p, hsa-miR-29a-5p, hsa-miR-92a-2-5p, hsa-miR-30c-2-3p, hsa-miR-150-3p, hsa-miR-374a-3p, hsa-miR-323a-5p, hsa-miR-339-3p, hsa-miR-18b-3p, hsa-miR-551b-5p, hsa-miR-548b-5p, hsa-miR-629-5p, hsa-miR-411-3p, hsa-miR-891b, hsa-miR-541-3p, hsa-miR-509-3-5p, hsa-miR-944, hsa-miR-1227-3p, hsa-miR-1228-3p, hsa-miR-1237-3p, hsa-miR-1238-3p, hsa-miR-513c-5p, hsa-miR-1289, hsa-miR-1303, hsa-miR-1249-3p, hsa-miR-1250-5p, hsa-miR-1324, hsa-miR-320d, hsa-miR-1470, hsa-miR-1972, hsa-miR-449c-5p, hsa-miR-2116-5p, hsa-miR-3124-5p, hsa-miR-378b, hsa-miR-3154, hsa-miR-3155a, hsa-miR-3180-5p, hsa-miR-4295, hsa-miR-4311, hsa-miR-4313, hsa-miR-4315, hsa-miR-4314, hsa-miR-4321, hsa-miR-4323, hsa-miR-4284, hsa-miR-4286, hsa-miR-3660, hsa-miR-3666, hsa-miR-3667-5p, hsa-miR-3677-3p, hsa-miR-3679-3p, hsa-miR-3713, hsa-miR-3909, hsa-miR-3918, hsa-miR-4421, hsa-miR-4441, hsa-miR-4460, hsa-miR-4514, hsa-miR-4521, hsa-miR-4538, hsa-miR-3127-3p, hsa-miR-3152-5p, hsa-miR-3162-3p, hsa-miR-3150b-5p, hsa-miR-4648, hsa-miR-4649-3p, hsa-miR-4673, hsa-miR-4684-3p, hsa-miR-4701-3p, hsa-miR-4714-3p, hsa-miR-4716-5p, hsa-miR-4729, hsa-miR-4755-3p, hsa-miR-4762-5p, hsa-miR-4436b-3p, hsa-miR-1245b-5p, hsa-miR-550a-3-5p, hsa-miR-4433a-5p, hsa-miR-5009-3p, hsa-miR-5196-5p, hsa-miR-5571-3p, hsa-miR-5580-3p, hsa-miR-5695, hsa-miR-766-5p, hsa-miR-1304-3p, hsa-miR-550b-2-5p, hsa-miR-4750-3p, hsa-miR-6074, hsa-miR-6084, hsa-miR-6506-3p, hsa-miR-6515-5p, hsa-miR-6717-5p, hsa-miR-6719-3p, hsa-miR-372-5p, hsa-miR-605-3p, hsa-miR-619-5p, hsa-miR-1301-5p, hsa-miR-1251-3p, hsa-miR-6729-3p, hsa-miR-6731-3p, hsa-miR-6747-5p, hsa-miR-6748-5p, hsa-miR-6750-5p, hsa-miR-6756-3p, hsa-miR-6760-3p, hsa-miR-6763-3p, hsa-miR-6769a-5p, hsa-miR-6770-5p, hsa-miR-6775-3p, hsa-miR-6783-5p, hsa-miR-6784-3p, hsa-miR-6785-3p, hsa-miR-6788-5p, hsa-miR-6790-3p, hsa-miR-6795-3p, hsa-miR-6796-3p, hsa-miR-6805-3p, hsa-miR-6808-3p, hsa-miR-6810-3p, hsa-miR-6812-5p, hsa-miR-6812-3p, hsa-miR-6813-3p, hsa-miR-6819-3p, hsa-miR-6832-5p, hsa-miR-6836-3p, hsa-miR-6841-3p, hsa-miR-6858-3p, hsa-miR-6859-3p, hsa-miR-6870-3p, hsa-miR-6871-5p, hsa-miR-6876-5p, hsa-miR-6891-3p, hsa-miR-6892-3p, hsa-miR-7106-5p, hsa-miR-7108-3p, hsa-miR-7111-5p, hsa-miR-7114-3p, hsa-miR-7156-3p, hsa-miR-4433b-5p, hsa-miR-7851-3p, hsa-miR-8078, hsa-miR-9898, hsa-miR-9900, hsa-miR-10392-3p, hsa-miR-10522-5p, hsa-miR-10526-3p, hsa-miR-12116, hsa-miR-12118, and hsa-miR-12122. In the present disclosure, the extract of urine may be an extract of the urine of a stage-I prostate cancer patient. Only one of these microRNAs can be used to predict whether a subject from which the urine is derived is a prostate cancer patient (particularly stage-I), and 5 or more types, 10 or more types, 15 or more types, or 20 or more types of these microRNAs can be used in combination to predict whether a subject from which the urine is derived is a prostate cancer patient (particularly stage-I). Among these microRNAs, a microRNA that may be highly expressed in a prostate cancer patient (particularly stage-I) can be preferably used for prediction.

The present disclosure provides an extract of urine containing at least one type, two types, three types, four types, five types, six types, seven types, eight types, nine types, or ten types of or all microRNAs selected from the group consisting of microRNAs having an accuracy of higher than 0.500 among the microRNAs listed in Table 24-7. In the present disclosure, the extract of urine may be an extract of the urine of a prostate cancer patient. Only one of these microRNAs can be used to predict whether a subject from which the urine is derived is a prostate cancer patient, and 5 or more types, 10 or more types, 15 or more types, or 20 or more types of these microRNAs can be used in combination to predict whether a subject from which the urine is derived is a prostate cancer patient. Among these microRNAs, a microRNA that may be highly expressed in a prostate cancer patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

The present disclosure provides an extract of urine containing at least one type, two types, three types, four types, five types, six types, seven types, eight types, nine types, or ten types of or all microRNAs selected from the group consisting of microRNAs having an accuracy of higher than 0.500 among the microRNAs listed in Table 25-7. In the present disclosure, the extract of urine may be an extract of the urine of a prostate cancer patient. Only one of these microRNAs can be used to predict whether a subject from which the urine is derived is a prostate cancer patient, and 5 or more types, 10 or more types, 15 or more types, or 20 or more types of these microRNAs can be used in combination to predict whether a subject from which the urine is derived is a prostate cancer patient. Among these microRNAs, a microRNA that may be highly expressed in a prostate cancer patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

The present disclosure provides an extract of urine containing at least one type, two types, three types, four types, five types, six types, seven types, eight types, nine types, or ten types of or all microRNAs selected from the microRNAs listed in Table 4-8. The present disclosure also provides an extract of urine containing at least one type, two types, three types, four types, five types, six types, seven types, eight types, nine types, or ten types of or all microRNAs selected from the group consisting of hsa-miR-605-3p, hsa-miR-4755-3p, hsa-miR-1292-5p, hsa-miR-202-3p, hsa-miR-492, hsa-miR-10396a-3p, hsa-miR-629-5p, hsa-miR-6809-5p, hsa-miR-6081, hsa-miR-6750-5p, hsa-miR-135a-2-3p, hsa-miR-3124-5p, hsa-miR-6789-5p, hsa-miR-10392-3p, hsa-miR-4258, hsa-miR-4479, hsa-miR-4321, hsa-miR-4521, hsa-miR-498-5p, hsa-miR-3909, hsa-miR-1268b, hsa-miR-513b-5p, hsa-miR-4741, hsa-miR-3677-3p, hsa-miR-1914-3p, hsa-miR-6125, hsa-miR-3937, hsa-miR-665, hsa-miR-519c-5p, hsa-miR-523-5p, hsa-miR-518e-5p, hsa-miR-522-5p, hsa-miR-519a-5p, hsa-miR-519b-5p, hsa-miR-6791-5p, hsa-miR-4651, hsa-miR-6080, hsa-miR-6781-5p, hsa-miR-1908-5p, hsa-miR-4433b-3p, hsa-miR-4745-5p, hsa-miR-10400-3p, hsa-miR-1343-5p, hsa-miR-3648, hsa-miR-541-3p, hsa-miR-1470, hsa-miR-12120, hsa-miR-6836-5p, hsa-miR-92a-2-5p, hsa-miR-4439, hsa-miR-4673, hsa-miR-6836-3p, hsa-miR-4634, hsa-miR-449c-5p, hsa-miR-4433a-3p, hsa-miR-1469, hsa-miR-20b-3p, hsa-miR-10526-3p, hsa-miR-4801, hsa-miR-3161, hsa-miR-424-3p, hsa-miR-6850-5p, hsa-miR-4327, hsa-miR-6783-5p, hsa-miR-4785, hsa-miR-1297, hsa-miR-6845-5p, hsa-miR-150-3p, hsa-miR-320e, hsa-miR-3660, hsa-miR-638, hsa-miR-3620-5p, hsa-miR-6743-5p, hsa-miR-421, hsa-miR-4684-3p, hsa-miR-542-5p, hsa-miR-6770-5p, hsa-miR-135a-3p, hsa-miR-4665-5p, hsa-miR-3197, hsa-miR-4507, hsa-miR-601, hsa-miR-6790-3p, hsa-miR-551b-5p, hsa-miR-4758-5p, hsa-miR-4749-5p, hsa-miR-187-5p, hsa-miR-6805-5p, hsa-miR-1915-3p, hsa-miR-7111-5p, hsa-miR-8072, hsa-miR-4648, hsa-miR-4435, hsa-miR-6729-5p, hsa-miR-3180-5p, hsa-miR-6794-5p, hsa-miR-6821-5p, hsa-miR-1225-5p, hsa-miR-12122, hsa-miR-4466, hsa-miR-1227-5p, hsa-miR-668-5p, hsa-miR-7150, hsa-miR-6814-5p, hsa-miR-4763-3p, hsa-miR-6724-5p, hsa-miR-6074, hsa-miR-4530, hsa-miR-4505, hsa-miR-3199, hsa-miR-6721-5p, hsa-miR-4688, hsa-miR-6513-5p, hsa-miR-769-3p, hsa-miR-378i, hsa-miR-1207-5p, hsa-miR-6732-5p, hsa-miR-6752-5p, hsa-miR-937-5p, hsa-miR-320c, hsa-miR-554, hsa-miR-4534, hsa-miR-663a, hsa-miR-939-5p, hsa-miR-4470, hsa-miR-718, hsa-miR-8078, hsa-miR-3178, hsa-miR-7108-3p, hsa-miR-3187-3p, hsa-miR-3130-3p, hsa-miR-7851-3p, hsa-miR-6746-5p, hsa-miR-769-5p, hsa-miR-762, hsa-miR-4454, hsa-miR-4261, hsa-miR-1268a, hsa-miR-6840-3p, hsa-miR-25-5p, hsa-miR-942-5p, hsa-miR-5196-5p, hsa-miR-8064, hsa-miR-3196, hsa-miR-6798-5p, hsa-miR-4674, hsa-miR-4734, hsa-miR-4467, hsa-miR-4295, hsa-miR-4315, hsa-miR-6763-5p, hsa-miR-449b-5p, hsa-miR-6770-3p, hsa-miR-10396b-5p, hsa-miR-7107-5p, hsa-miR-6768-5p, hsa-miR-4649-3p, hsa-miR-1303, hsa-miR-4650-5p, hsa-miR-3666, hsa-miR-92b-5p, hsa-miR-650, hsa-miR-3619-5p, hsa-miR-4720-5p, hsa-miR-3180-3p, hsa-miR-1909-3p, hsa-miR-370-5p, hsa-miR-6829-5p, hsa-miR-4280, hsa-miR-10527-5p, hsa-miR-708-5p, hsa-miR-4314, hsa-miR-3621, hsa-miR-369-5p, hsa-miR-6805-3p, hsa-miR-6784-5p, hsa-miR-6090, hsa-miR-711, hsa-miR-1237-5p, hsa-miR-5585-3p, hsa-miR-125b-2-3p, hsa-miR-10401-5p, hsa-miR-10396a-5p, hsa-miR-211-3p, hsa-miR-5587-5p, hsa-miR-4736, hsa-miR-6513-3p, hsa-miR-4783-3p, hsa-miR-338-5p, hsa-miR-6089, hsa-miR-7158-5p, hsa-miR-6722-3p, hsa-miR-6817-5p, hsa-miR-579-3p, hsa-miR-6720-5p, hsa-miR-3150b-5p, hsa-miR-6893-5p, hsa-miR-5696, hsa-miR-6889-5p, hsa-miR-4516, hsa-miR-6787-5p, hsa-miR-1912-3p, hsa-miR-8077, hsa-miR-4436b-3p, hsa-miR-4672, hsa-miR-3913-3p, hsa-miR-4707-5p, hsa-miR-619-5p, hsa-miR-4665-3p, hsa-miR-6838-5p, hsa-miR-4257, hsa-miR-10522-5p, hsa-miR-6796-3p, hsa-miR-4756-3p, hsa-miR-589-3p, hsa-miR-4484, hsa-miR-6068, hsa-miR-760, hsa-miR-4311, hsa-miR-10401-3p, hsa-miR-639, hsa-miR-124-5p, hsa-miR-3059-3p, hsa-miR-486-3p, hsa-miR-6075, hsa-miR-4465, hsa-miR-139-3p, hsa-miR-494-3p, hsa-miR-3135b, hsa-miR-525-5p, hsa-miR-2681-5p, hsa-miR-1286, hsa-miR-8069, hsa-miR-1269a, hsa-miR-4497, hsa-miR-6818-3p, hsa-miR-4449, hsa-miR-491-3p, hsa-miR-1587, hsa-miR-4508, hsa-miR-6749-5p, hsa-miR-4429, hsa-miR-4738-3p, hsa-miR-30b-3p, hsa-miR-6815-5p, hsa-miR-130b-3p, hsa-miR-3934-3p, hsa-miR-6810-3p, hsa-miR-4640-5p, hsa-miR-2277-5p, hsa-miR-6775-3p, hsa-miR-5006-3p, hsa-miR-628-3p, hsa-miR-3907, hsa-miR-3940-5p, hsa-miR-7151-5p, hsa-miR-485-5p, hsa-miR-7974, hsa-miR-518b, hsa-miR-4727-3p, hsa-miR-3665, hsa-miR-371b-5p, hsa-miR-6127, hsa-miR-6760-3p, hsa-miR-6850-3p, hsa-miR-184, hsa-miR-4446-3p, hsa-miR-4783-5p, hsa-miR-501-5p, hsa-miR-12117, hsa-miR-4750-5p, hsa-miR-1343-3p, hsa-miR-4708-3p, hsa-miR-5090, hsa-miR-6786-5p, hsa-miR-3610, hsa-miR-7108-5p, hsa-miR-27b-3p, hsa-miR-6892-3p, hsa-miR-8074, hsa-miR-3127-3p, hsa-miR-3141, hsa-miR-6816-5p, hsa-miR-921, hsa-miR-5702, hsa-miR-5584-3p, hsa-miR-6747-5p, hsa-miR-625-3p, hsa-miR-138-2-3p, hsa-miR-105-5p, hsa-miR-3663-3p, hsa-miR-1225-3p, hsa-miR-5572, hsa-miR-6730-5p, hsa-miR-371a-3p, hsa-miR-193b-5p, hsa-miR-9898, hsa-miR-1301-3p, hsa-miR-128-1-5p, hsa-miR-3928-3p, hsa-miR-887-3p, hsa-miR-6861-5p, hsa-miR-12118, hsa-miR-1228-3p, hsa-miR-6076, hsa-miR-1270, hsa-miR-4299, hsa-miR-6749-3p, hsa-miR-1298-3p, hsa-miR-4733-3p, hsa-miR-12119, hsa-miR-5703, hsa-miR-3622a-5p, hsa-miR-6731-3p, hsa-miR-1269b, hsa-miR-8089, hsa-miR-4447, hsa-miR-323a-5p, hsa-miR-6784-3p, hsa-miR-1204, hsa-miR-6776-5p, hsa-miR-8063, hsa-miR-361-3p, hsa-miR-3944-5p, hsa-miR-4716-3p, hsa-miR-4474-3p, hsa-miR-4482-3p, hsa-miR-5787, hsa-miR-3679-3p, hsa-miR-8080, hsa-miR-4286, hsa-miR-6515-5p, hsa-miR-6756-3p, hsa-miR-1298-5p, hsa-miR-6126, hsa-miR-3074-5p, hsa-miR-6777-5p, hsa-miR-4433a-5p, hsa-miR-4695-5p, hsa-miR-3934-5p, hsa-miR-4255, hsa-miR-4276, hsa-miR-6859-3p, hsa-miR-411-3p, hsa-miR-4499, hsa-miR-4635, hsa-miR-520e-3p, hsa-miR-3691-3p, hsa-miR-6812-3p, hsa-miR-6501-5p, hsa-miR-4750-3p, hsa-miR-4330, hsa-miR-6820-5p, hsa-miR-572, hsa-miR-6499-5p, hsa-miR-4526, hsa-miR-300, hsa-miR-6763-3p, hsa-miR-196b-3p, hsa-miR-1178-3p, hsa-miR-3173-3p, hsa-miR-6768-3p, hsa-miR-4787-5p, hsa-miR-4653-5p, hsa-miR-3122, hsa-miR-8070, hsa-miR-6717-5p, hsa-miR-6756-5p, hsa-miR-200b-3p, hsa-miR-2110, hsa-miR-3186-3p, hsa-miR-3651, hsa-miR-6797-5p, hsa-miR-1301-5p, hsa-miR-4293, hsa-miR-6813-3p, hsa-miR-4294, hsa-miR-8062, hsa-miR-4687-5p, hsa-miR-10392-5p, hsa-miR-6069, hsa-miR-3616-3p, hsa-miR-1237-3p, hsa-miR-7975, hsa-miR-1972, hsa-miR-6727-5p, hsa-miR-3922-5p, hsa-miR-6506-5p, hsa-miR-4492, hsa-miR-6503-3p, hsa-miR-4285, hsa-miR-4690-3p, hsa-miR-575, hsa-miR-7856-5p, hsa-miR-520b-3p, hsa-miR-3195, hsa-miR-1249-3p, hsa-miR-7106-5p, hsa-miR-5006-5p, hsa-miR-18b-3p, hsa-miR-6870-3p, hsa-miR-4694-3p, hsa-miR-6830-5p, hsa-miR-5685, hsa-miR-3120-5p, hsa-miR-4433b-5p, hsa-miR-6529-5p, hsa-miR-4749-3p, hsa-miR-6791-3p, hsa-miR-1247-3p, hsa-miR-4763-5p, hsa-miR-10395-5p, hsa-miR-1284, hsa-miR-6813-5p, hsa-miR-6790-5p, hsa-miR-1321, hsa-miR-6785-3p, hsa-miR-1288-3p, hsa-miR-6858-3p, hsa-miR-6719-3p, hsa-miR-4776-3p, hsa-miR-5004-3p, hsa-miR-340-3p, hsa-miR-6085, hsa-miR-3155a, hsa-miR-4661-3p, hsa-miR-3162-3p, hsa-miR-4740-3p, hsa-miR-6884-3p, hsa-miR-2276-3p, hsa-miR-3922-3p, hsa-miR-7114-3p, hsa-miR-6819-3p, hsa-let-7f-1-3p, hsa-miR-4716-5p, hsa-miR-4308, hsa-miR-5681b, hsa-miR-6726-5p, hsa-miR-6888-5p, hsa-miR-6841-3p, hsa-miR-5091, hsa-miR-4274, hsa-miR-1285-3p, hsa-miR-7110-5p, hsa-miR-6822-3p, hsa-miR-6500-3p, hsa-miR-181d-3p, hsa-miR-149-3p, hsa-miR-5739, hsa-miR-4313, hsa-miR-614, hsa-miR-6798-3p, hsa-miR-1913, hsa-miR-3185, hsa-miR-1306-3p, hsa-miR-4793-5p, hsa-miR-6877-5p, hsa-miR-6795-3p, hsa-miR-6766-3p, hsa-miR-6737-5p, hsa-miR-4658, hsa-miR-11181-3p, hsa-miR-12116, hsa-miR-377-5p, hsa-miR-370-3p, hsa-miR-4444, hsa-miR-6845-3p, hsa-miR-4458, hsa-miR-500a-5p, hsa-miR-9718, hsa-miR-18a-3p, hsa-miR-6866-3p, hsa-miR-4421, hsa-miR-6758-5p, hsa-miR-500b-3p, hsa-miR-6729-3p, hsa-miR-4712-5p, hsa-miR-6501-3p, hsa-miR-6875-5p, hsa-miR-4472, hsa-miR-6880-3p, hsa-miR-432-5p, hsa-miR-1908-3p, hsa-miR-4746-3p, hsa-miR-3675-3p, hsa-miR-3667-5p, hsa-miR-517a-3p, hsa-miR-517b-3p, hsa-miR-9851-5p, hsa-miR-6831-5p, hsa-miR-550a-3-5p, hsa-miR-623, hsa-miR-339-3p, hsa-miR-6869-5p, hsa-miR-6884-5p, hsa-miR-7854-3p, hsa-miR-6762-5p, hsa-miR-1236-5p, hsa-miR-1238-3p, hsa-miR-4520-5p, hsa-miR-6760-5p, hsa-miR-29a-3p, hsa-miR-6515-3p, hsa-miR-10398-3p, hsa-miR-197-5p, hsa-miR-498-3p, hsa-miR-4463, hsa-miR-1180-5p, hsa-miR-7704, hsa-miR-4769-5p, hsa-miR-1224-3p, hsa-miR-6859-5p, hsa-miR-6795-5p, hsa-miR-7114-5p, hsa-miR-487a-5p, hsa-miR-598-5p, hsa-miR-6868-3p, hsa-miR-1281, hsa-miR-654-5p, hsa-miR-617, hsa-miR-1255b-2-3p, hsa-miR-6777-3p, hsa-miR-6891-3p, hsa-miR-550a-5p, hsa-miR-513c-5p, hsa-miR-4681, hsa-miR-595, hsa-miR-3180, hsa-miR-3689b-3p, hsa-miR-3689c, hsa-miR-11399, hsa-miR-1291, hsa-miR-4488, hsa-miR-6165, hsa-miR-6742-5p, hsa-miR-5093, hsa-miR-3155b, hsa-miR-6848-5p, hsa-miR-7112-5p, hsa-miR-5002-5p, hsa-miR-378a-3p, hsa-miR-4498, hsa-miR-1295b-3p, hsa-miR-3177-3p, hsa-miR-8075, hsa-miR-4740-5p, hsa-miR-181a-2-3p, hsa-miR-4265, hsa-miR-146b-3p, hsa-miR-4731-3p, hsa-miR-4684-5p, hsa-miR-548q, hsa-miR-3150b-3p, hsa-miR-3158-3p, hsa-miR-6504-3p, hsa-miR-6124, hsa-miR-6739-3p, hsa-miR-4496, hsa-miR-3614-5p, hsa-miR-6131, hsa-miR-6083, hsa-miR-4531, hsa-miR-8071, hsa-miR-3131, hsa-miR-7113-5p, hsa-miR-6874-5p, hsa-miR-3150a-5p, hsa-miR-1228-5p, hsa-miR-937-3p, hsa-miR-200a-5p, hsa-miR-4318, hsa-miR-6811-3p, hsa-miR-155-3p, hsa-miR-216a-3p, hsa-miR-3680-3p, hsa-miR-6792-5p, hsa-miR-4800-3p, hsa-miR-491-5p, hsa-miR-940, hsa-miR-10394-5p, hsa-miR-6086, hsa-miR-342-5p, hsa-miR-4745-3p, hsa-miR-3165, hsa-miR-6514-5p, hsa-miR-6879-5p, hsa-miR-6797-3p, hsa-miR-2682-5p, hsa-miR-433-3p, hsa-miR-6511a-5p, hsa-miR-5100, hsa-miR-3663-5p, hsa-miR-6839-5p, hsa-miR-4440, hsa-miR-6895-5p, hsa-miR-3675-5p, hsa-miR-4725-3p, hsa-miR-4773, hsa-miR-365a-3p, hsa-miR-365b-3p, hsa-miR-3692-5p, hsa-miR-5001-5p, hsa-miR-6511b-5p, hsa-miR-4701-3p, hsa-miR-4645-3p, hsa-miR-4525, hsa-miR-6861-3p, hsa-miR-5190, hsa-miR-208a-5p, hsa-miR-4323, hsa-miR-6800-5p, hsa-miR-6887-5p, hsa-miR-4686, hsa-miR-3646, hsa-miR-383-3p, hsa-miR-6854-3p, hsa-miR-5708, hsa-miR-4328, hsa-miR-4267, hsa-miR-1915-5p, hsa-miR-4304, hsa-miR-3125, hsa-miR-186-3p, hsa-let-7d-3p, hsa-miR-6876-5p, hsa-miR-3654, hsa-miR-4728-3p, hsa-miR-6741-5p, hsa-miR-6765-5p, hsa-miR-5009-3p, hsa-miR-4489, hsa-miR-3187-5p, hsa-miR-576-5p, hsa-miR-4655-5p, hsa-miR-3605-5p, hsa-miR-5007-5p, hsa-miR-125b-1-3p, hsa-miR-326, hsa-miR-6887-3p, hsa-miR-4717-5p, hsa-miR-1203, hsa-miR-299-3p, hsa-miR-4428, hsa-miR-484, hsa-miR-2116-5p, hsa-miR-210-3p, hsa-miR-12121, hsa-miR-1285-5p, hsa-miR-6782-5p, hsa-miR-6800-3p, hsa-miR-7976, hsa-miR-7151-3p, hsa-miR-6832-5p, hsa-miR-3713, hsa-miR-4540, hsa-miR-612, hsa-miR-193a-5p, hsa-miR-6505-3p, hsa-miR-3943, hsa-miR-4656, hsa-miR-892a, hsa-miR-106b-3p, hsa-miR-3133, hsa-miR-3150a-3p, hsa-miR-431-3p, hsa-miR-16-2-3p, hsa-miR-4524b-5p, hsa-miR-658, hsa-miR-645, hsa-miR-210-5p, hsa-miR-6847-5p, hsa-miR-214-5p, hsa-miR-6738-3p, hsa-miR-4538, hsa-miR-551a, hsa-miR-328-3p, hsa-miR-222-5p, hsa-miR-330-5p, hsa-miR-6767-5p, hsa-miR-8088, hsa-miR-6860, hsa-miR-4647, hsa-miR-7109-5p, hsa-miR-4322, hsa-miR-4676-5p, hsa-miR-1273h-3p, hsa-miR-4486, hsa-miR-1304-3p, hsa-miR-4708-5p, hsa-miR-6752-3p, hsa-miR-4691-5p, hsa-miR-6761-5p, hsa-miR-3689a-3p, hsa-miR-4298, hsa-miR-103b, hsa-miR-4487, hsa-miR-3685, hsa-miR-7160-5p, hsa-miR-4743-3p, hsa-miR-4786-3p, hsa-miR-1236-3p, hsa-miR-30c-2-3p, hsa-miR-6871-5p, hsa-miR-3612, hsa-miR-3137, hsa-miR-7846-3p, hsa-miR-34c-3p, hsa-miR-103a-1-5p, hsa-miR-12114, hsa-miR-361-5p, hsa-miR-7-1-3p, hsa-miR-3945, hsa-miR-4632-5p, hsa-miR-5188, hsa-miR-506-3p, hsa-miR-6793-5p, hsa-miR-6817-3p, hsa-let-7i-5p, hsa-miR-608, hsa-miR-6895-3p, hsa-miR-338-3p, hsa-miR-6849-5p, hsa-miR-7706, hsa-miR-1471, hsa-miR-6828-5p, hsa-miR-3065-3p, hsa-miR-770-5p, hsa-miR-7848-3p, hsa-miR-3619-3p, hsa-miR-5194, hsa-miR-4667-5p, hsa-miR-6843-3p, hsa-miR-4513, hsa-miR-580-3p, hsa-miR-4317, hsa-miR-1304-5p, hsa-miR-2467-3p, hsa-miR-4485-5p, hsa-miR-6848-3p, hsa-miR-7154-3p, hsa-miR-4456, hsa-miR-3147, hsa-miR-4706, hsa-miR-4747-5p, hsa-miR-3652, hsa-miR-933, hsa-miR-631, hsa-miR-4784, hsa-miR-8059, hsa-miR-1193, hsa-miR-4778-5p, hsa-miR-4652-5p, hsa-miR-3184-3p, hsa-miR-4284, hsa-miR-4707-3p, hsa-miR-342-3p, hsa-miR-6788-5p, hsa-miR-5088-5p, hsa-miR-602, hsa-miR-3154, hsa-miR-3085-3p, hsa-miR-296-5p, hsa-miR-200c-5p, hsa-miR-4539, hsa-miR-6516-5p, hsa-miR-363-5p, hsa-miR-6875-3p, hsa-miR-4512, hsa-miR-6837-5p, hsa-miR-4752, hsa-miR-489-5p, hsa-miR-642b-5p, hsa-miR-583, hsa-miR-4761-3p, hsa-miR-3156-5p, hsa-miR-557, hsa-miR-2681-3p, hsa-miR-4713-3p, hsa-miR-4676-3p, hsa-miR-6778-5p, hsa-miR-6764-3p, hsa-miR-4747-3p, hsa-miR-1238-5p, hsa-miR-654-3p, hsa-miR-4430, hsa-miR-1287-3p, hsa-miR-5588-3p, hsa-miR-6769a-5p, hsa-miR-5004-5p, hsa-miR-615-3p, hsa-miR-3163, hsa-miR-4723-5p, hsa-miR-8085, hsa-miR-4535, hsa-miR-615-5p, hsa-miR-4252, hsa-miR-6827-5p, hsa-miR-1273c, hsa-miR-3917, hsa-miR-6883-5p, hsa-miR-766-5p, hsa-miR-10400-5p, hsa-miR-5189-5p, hsa-miR-6772-5p, hsa-miR-4732-5p, hsa-miR-4717-3p, hsa-miR-942-3p, hsa-miR-6857-5p, hsa-miR-5580-3p, hsa-miR-920, hsa-miR-943, hsa-miR-3960, hsa-miR-4533, hsa-miR-6892-5p, hsa-miR-449c-3p, hsa-miR-3617-3p, hsa-miR-3176, hsa-miR-7515, hsa-miR-4650-3p, hsa-miR-504-3p, hsa-miR-3193, hsa-miR-6789-3p, hsa-miR-1538, hsa-miR-29c-5p, hsa-miR-550b-2-5p, hsa-miR-4733-5p, hsa-miR-125a-3p, hsa-miR-4300, hsa-miR-519a-3p, hsa-miR-6806-5p, hsa-miR-4251, hsa-miR-151a-5p, hsa-miR-1227-3p, hsa-let-7b-3p, hsa-miR-3191-3p, hsa-miR-3173-5p, hsa-miR-5189-3p, hsa-miR-3151-5p, hsa-miR-5698, hsa-miR-593-3p, hsa-miR-4253, hsa-miR-133a-3p, hsa-miR-12115, hsa-miR-4260, hsa-miR-4754, hsa-miR-185-3p, hsa-miR-4316, hsa-miR-4506, hsa-miR-1910-3p, hsa-miR-1296-3p, hsa-miR-3152-5p, hsa-miR-1266-5p, hsa-miR-584-3p, hsa-miR-6510-3p, hsa-miR-4726-3p, hsa-let-7c-3p, hsa-miR-8073, hsa-miR-517-5p, hsa-miR-3160-5p, hsa-miR-885-3p, hsa-miR-4450, hsa-miR-1185-1-3p, hsa-miR-1234-3p, hsa-miR-217-3p, hsa-miR-6766-5p, hsa-miR-4441, hsa-miR-550b-3p, hsa-miR-6738-5p, hsa-miR-6134, hsa-miR-378a-5p, hsa-miR-26b-3p, hsa-miR-3126-3p, hsa-miR-6852-3p, hsa-miR-3064-5p, hsa-miR-3918, hsa-miR-5187-5p, hsa-miR-138-1-3p, hsa-miR-4685-5p, hsa-miR-6071, hsa-miR-4306, hsa-miR-6852-5p, hsa-miR-302c-5p, hsa-miR-6842-5p, hsa-miR-183-3p, hsa-miR-5008-5p, hsa-miR-525-3p, hsa-miR-4743-5p, hsa-miR-4767, hsa-miR-574-5p, hsa-miR-2114-5p, hsa-miR-30c-5p, hsa-miR-6812-5p, hsa-miR-4273, hsa-miR-676-5p, hsa-miR-3116, hsa-miR-6716-5p, hsa-miR-596, hsa-miR-5589-5p, hsa-miR-558, hsa-miR-3162-5p, hsa-miR-6132, hsa-miR-7109-3p, hsa-miR-3153, hsa-miR-4485-3p, hsa-miR-3944-3p, hsa-miR-4764-3p, hsa-miR-1185-2-3p, hsa-miR-4730, hsa-miR-874-5p, hsa-miR-6762-3p, hsa-miR-141-5p, hsa-miR-6748-5p, hsa-miR-5591-3p, hsa-miR-7152-5p, hsa-miR-2113, hsa-miR-6867-5p, hsa-miR-2909, hsa-miR-6775-5p, hsa-miR-25-3p, hsa-miR-761, hsa-miR-6799-5p, hsa-miR-4475, hsa-miR-6883-3p, hsa-miR-365a-5p, hsa-miR-657, hsa-miR-378b, hsa-miR-1251-3p, hsa-miR-4259, hsa-miR-204-3p, hsa-miR-4794, hsa-miR-2116-3p, hsa-miR-1307-3p, hsa-miR-203a-3p, hsa-miR-6510-5p, hsa-miR-3649, hsa-miR-10398-5p, hsa-miR-6500-5p, hsa-miR-4769-3p, hsa-miR-1843, hsa-miR-3189-3p, hsa-miR-4755-5p, hsa-miR-4473, hsa-miR-3200-5p, hsa-let-7f-2-3p, hsa-miR-494-5p, hsa-miR-4514, hsa-miR-509-3p, hsa-miR-892b, hsa-miR-1250-5p, hsa-miR-605-5p, hsa-miR-877-5p, hsa-miR-3679-5p, hsa-miR-151b, hsa-miR-4762-5p, hsa-miR-4780, hsa-miR-4638-5p, hsa-miR-6808-5p, hsa-miR-6780b-5p, hsa-miR-664b-5p, hsa-miR-629-3p, hsa-miR-4319, hsa-miR-6853-5p, hsa-miR-20a-3p, hsa-miR-892c-5p, hsa-miR-6722-5p, hsa-miR-335-3p, hsa-miR-4478, hsa-miR-6774-3p, hsa-miR-1184, hsa-miR-3659, hsa-miR-935, hsa-miR-6816-3p, hsa-miR-671-3p, hsa-miR-323b-5p, hsa-miR-6885-3p, hsa-miR-6880-5p, hsa-miR-4431, hsa-miR-3200-3p, hsa-miR-6873-5p, hsa-miR-4748, hsa-miR-936, hsa-miR-1909-5p, hsa-miR-4640-3p, hsa-miR-520d-3p, hsa-miR-198, hsa-miR-581, hsa-miR-622, hsa-miR-3928-5p, hsa-miR-4451, hsa-miR-5002-3p, hsa-miR-6796-5p, hsa-miR-4494, hsa-miR-134-3p, hsa-miR-3689d, hsa-miR-887-5p, hsa-miR-5010-5p, hsa-miR-4324, hsa-miR-5571-3p, and hsa-miR-6846-5p. In the present disclosure, the extract of urine may be an extract of the urine of a gastric cancer patient. Only one of these microRNAs can be used to predict whether a subject from which the urine is derived is a gastric cancer patient, and 5 or more types, 10 or more types, 15 or more types, or 20 or more types of these microRNAs can be used in combination to predict whether a subject from which the urine is derived is a gastric cancer patient. Among these microRNAs, a microRNA that may be highly expressed in a gastric cancer patient can be preferably used for prediction.

The present disclosure provides an extract of urine containing at least one type, two types, three types, four types, five types, six types, seven types, eight types, nine types, or ten types of or all microRNAs selected from the group consisting of hsa-miR-29a-3p, hsa-miR-29b-3p, hsa-miR-198, hsa-miR-203a-3p, hsa-miR-214-3p, hsa-miR-133a-3p, hsa-miR-134-5p, hsa-miR-184, hsa-miR-185-5p, hsa-miR-299-3p, hsa-miR-296-5p, hsa-miR-130b-3p, hsa-miR-361-5p, hsa-miR-365a-3p, hsa-miR-365b-3p, hsa-miR-302c-5p, hsa-miR-370-3p, hsa-miR-373-5p, hsa-miR-378a-3p, hsa-miR-328-3p, hsa-miR-326, hsa-miR-133b, hsa-miR-200a-5p, hsa-miR-433-3p, hsa-miR-410-3p, hsa-miR-484, hsa-miR-485-5p, hsa-miR-486-5p, hsa-miR-491-5p, hsa-miR-202-3p, hsa-miR-492, hsa-miR-494-3p, hsa-miR-512-5p, hsa-miR-498-5p, hsa-miR-520e-3p, hsa-miR-519e-3p, hsa-miR-519c-5p, hsa-miR-523-5p, hsa-miR-518e-5p, hsa-miR-522-5p, hsa-miR-519a-5p, hsa-miR-519b-5p, hsa-miR-526b-3p, hsa-miR-525-5p, hsa-miR-518f-3p, hsa-miR-520b-3p, hsa-miR-518b, hsa-miR-520d-3p, hsa-miR-516b-5p, hsa-miR-519a-3p, hsa-miR-501-5p, hsa-miR-513a-5p, hsa-miR-18a-3p, hsa-miR-551a, hsa-miR-554, hsa-miR-557, hsa-miR-564, hsa-miR-571, hsa-miR-572, hsa-miR-575, hsa-miR-576-5p, hsa-miR-579-3p, hsa-miR-580-3p, hsa-miR-583, hsa-miR-584-5p, hsa-miR-589-3p, hsa-miR-590-5p, hsa-miR-595, hsa-miR-596, hsa-miR-600, hsa-miR-601, hsa-miR-611, hsa-miR-612, hsa-miR-614, hsa-miR-615-3p, hsa-miR-617, hsa-miR-622, hsa-miR-628-3p, hsa-miR-629-3p, hsa-miR-632, hsa-miR-638, hsa-miR-639, hsa-miR-650, hsa-miR-661, hsa-miR-663a, hsa-miR-654-5p, hsa-miR-658, hsa-miR-421, hsa-miR-542-5p, hsa-miR-671-5p, hsa-miR-769-3p, hsa-miR-766-3p, hsa-miR-770-5p, hsa-miR-675-5p, hsa-let-7b-3p, hsa-let-7f-1-3p, hsa-miR-22-5p, hsa-miR-25-5p, hsa-miR-26b-3p, hsa-miR-92a-2-5p, hsa-miR-30c-2-3p, hsa-miR-139-3p, hsa-miR-181c-3p, hsa-miR-183-3p, hsa-miR-187-5p, hsa-miR-15b-3p, hsa-miR-30b-3p, hsa-miR-124-5p, hsa-miR-125b-1-3p, hsa-miR-135a-3p, hsa-miR-125a-3p, hsa-miR-125b-2-3p, hsa-miR-127-5p, hsa-miR-138-1-3p, hsa-miR-149-3p, hsa-miR-150-3p, hsa-miR-185-3p, hsa-miR-186-3p, hsa-miR-193a-5p, hsa-miR-155-3p, hsa-miR-194-3p, hsa-miR-30c-1-3p, hsa-miR-296-3p, hsa-miR-361-3p, hsa-miR-302d-5p, hsa-miR-371a-5p, hsa-miR-377-5p, hsa-miR-342-5p, hsa-miR-323a-5p, hsa-miR-339-3p, hsa-miR-423-5p, hsa-miR-424-3p, hsa-miR-18b-3p, hsa-miR-20b-3p, hsa-miR-483-5p, hsa-miR-486-3p, hsa-miR-490-5p, hsa-miR-193b-5p, hsa-miR-501-3p, hsa-miR-92b-5p, hsa-miR-551b-5p, hsa-miR-574-5p, hsa-miR-550a-5p, hsa-miR-615-5p, hsa-miR-625-3p, hsa-miR-629-5p, hsa-miR-411-3p, hsa-miR-671-3p, hsa-miR-298, hsa-miR-874-3p, hsa-miR-890, hsa-miR-892b, hsa-miR-541-3p, hsa-miR-708-5p, hsa-miR-744-5p, hsa-miR-885-3p, hsa-miR-877-5p, hsa-miR-877-3p, hsa-miR-887-3p, hsa-miR-665, hsa-miR-760, hsa-miR-920, hsa-miR-921, hsa-miR-509-3-5p, hsa-miR-933, hsa-miR-935, hsa-miR-937-3p, hsa-miR-939-5p, hsa-miR-940, hsa-miR-943, hsa-miR-1224-5p, hsa-miR-1224-3p, hsa-miR-1225-5p, hsa-miR-1225-3p, hsa-miR-1227-3p, hsa-miR-1228-5p, hsa-miR-1228-3p, hsa-miR-1231, hsa-miR-1234-3p, hsa-miR-1236-3p, hsa-miR-1237-3p, hsa-miR-1238-3p, hsa-miR-513b-5p, hsa-miR-320c, hsa-miR-1301-3p, hsa-miR-1298-5p, hsa-miR-1180-3p, hsa-miR-1182, hsa-miR-1184, hsa-miR-1202, hsa-miR-1203, hsa-miR-663b, hsa-miR-1207-5p, hsa-miR-1285-3p, hsa-miR-1297, hsa-miR-1303, hsa-miR-1304-5p, hsa-miR-1243, hsa-miR-1249-3p, hsa-miR-1250-5p, hsa-miR-1258, hsa-miR-1266-5p, hsa-miR-1267, hsa-miR-1268a, hsa-miR-1269a, hsa-miR-1270, hsa-miR-1281, hsa-miR-1292-5p, hsa-miR-1307-3p, hsa-miR-1321, hsa-miR-1469, hsa-miR-1470, hsa-miR-1471, hsa-miR-1538, hsa-miR-1908-5p, hsa-miR-1909-5p, hsa-miR-1909-3p, hsa-miR-1912-3p, hsa-miR-1913, hsa-miR-1914-3p, hsa-miR-1915-5p, hsa-miR-1915-3p, hsa-miR-365a-5p, hsa-miR-196b-3p, hsa-miR-1972, hsa-miR-2110, hsa-miR-449c-5p, hsa-miR-762, hsa-miR-670-5p, hsa-miR-2114-5p, hsa-miR-2114-3p, hsa-miR-2116-5p, hsa-miR-2116-3p, hsa-miR-548q, hsa-miR-2276-3p, hsa-miR-2278, hsa-miR-711, hsa-miR-718, hsa-miR-2681-3p, hsa-miR-449c-3p, hsa-miR-2861, hsa-miR-2909, hsa-miR-3116, hsa-miR-3122, hsa-miR-3124-5p, hsa-miR-3126-5p, hsa-miR-3130-3p, hsa-miR-3131, hsa-miR-3132, hsa-miR-3133, hsa-miR-378b, hsa-miR-3138, hsa-miR-3141, hsa-miR-3144-5p, hsa-miR-1273c, hsa-miR-3147, hsa-miR-548v, hsa-miR-3150a-3p, hsa-miR-3151-5p, hsa-miR-3153, hsa-miR-3074-3p, hsa-miR-3154, hsa-miR-3156-5p, hsa-miR-3158-3p, hsa-miR-3161, hsa-miR-3162-5p, hsa-miR-3163, hsa-miR-3164, hsa-miR-3165, hsa-miR-3173-3p, hsa-miR-1193, hsa-miR-3176, hsa-miR-3177-3p, hsa-miR-3178, hsa-miR-3180-5p, hsa-miR-3180-3p, hsa-miR-3184, 5p, hsa-miR-3185, hsa-miR-3065-5p, hsa-miR-3186-3p, hsa-miR-3187-3p, hsa-miR-3189-3p, hsa-miR-320e, hsa-miR-3191-3p, hsa-miR-3195, hsa-miR-3196, hsa-miR-3197, hsa-miR-3198, hsa-miR-3199, hsa-miR-514b-5p, hsa-miR-3202, hsa-miR-3065-3p, hsa-miR-4297, hsa-miR-4293, hsa-miR-4294, hsa-miR-4299, hsa-miR-4298, hsa-miR-4300, hsa-miR-4304, hsa-miR-4306, hsa-miR-4312, hsa-miR-4313, hsa-miR-4315, hsa-miR-4316, hsa-miR-4314, hsa-miR-4319, hsa-miR-4322, hsa-miR-4321, hsa-miR-4323, hsa-miR-4324, hsa-miR-4257, hsa-miR-4258, hsa-miR-4259, hsa-miR-4260, hsa-miR-4253, hsa-miR-4251, hsa-miR-4254, hsa-miR-4252, hsa-miR-4326, hsa-miR-4327, hsa-miR-4261, hsa-miR-4265, hsa-miR-4266, hsa-miR-4269, hsa-miR-4271, hsa-miR-4276, hsa-miR-4274, hsa-miR-4281, hsa-miR-4280, hsa-miR-4285, hsa-miR-4283, hsa-miR-4284, hsa-miR-4286, hsa-miR-4328, hsa-miR-2277-5p, hsa-miR-3200-5p, hsa-miR-3610, hsa-miR-3612, hsa-miR-3614-5p, hsa-miR-3616-3p, hsa-miR-3619-5p, hsa-miR-3621, hsa-miR-3622a-5p, hsa-miR-3622a-3p, hsa-miR-3622b-5p, hsa-miR-3646, hsa-miR-3648, hsa-miR-3649, hsa-miR-3651, hsa-miR-3652, hsa-miR-3660, hsa-miR-3661, hsa-miR-3663-5p, hsa-miR-3663-3p, hsa-miR-3665, hsa-miR-3666, h sa-miR-3667-5p, hsa-miR-3675-3p, hsa-miR-3677-3p, hsa-miR-3679-5p, hsa-miR-3679-3p, hsa-miR-3713, hsa-miR-3714, hsa-miR-3180, hsa-miR-3907, hsa-miR-3909, hsa-miR-3917, hsa-miR-3918, hsa-miR-3922-3p, hsa-miR-3925-5p, hsa-miR-3928-3p, hsa-miR-3934-5p, hsa-miR-3936, hsa-miR-3937, hsa-miR-3943, hsa-miR-3944-3p, hsa-miR-3945, hsa-miR-642b-3p, hsa-miR-550b-3p, hsa-miR-1268b, hsa-miR-4421, hsa-miR-4428, hsa-miR-4429, hsa-miR-4430, hsa-miR-4433a-3p, hsa-miR-4435, hsa-miR-4439, hsa-miR-4440, hsa-miR-4441, hsa-miR-4443, hsa-miR-4444, hsa-miR-4446-3p, hsa-miR-4447, hsa-miR-4448, hsa-miR-4449, hsa-miR-4456, hsa-miR-4458, hsa-miR-3135b, hsa-miR-4462, hsa-miR-4463, hsa-miR-4465, hsa-miR-4466, hsa-miR-4467, hsa-miR-4470, hsa-miR-4472, hsa-miR-4475, hsa-miR-4476, hsa-miR-4478, hsa-miR-3689d, hsa-miR-4479, hsa-miR-3155b, hsa-miR-4481, hsa-miR-4483, hsa-miR-4484, hsa-miR-4485-3p, hsa-miR-4486, hsa-miR-4487, hsa-miR-4488, hsa-miR-4489, hsa-miR-4492, hsa-miR-4496, hsa-miR-4497, hsa-miR-4498, hsa-miR-4499, hsa-miR-4505, hsa-miR-4506, hsa-miR-2392, hsa-miR-4507, hsa-miR-4508, hsa-miR-4512, hsa-miR-4513, hsa-miR-4516, hsa-miR-4519, hsa-miR-4521, hsa-miR-1269b, hsa-miR-4525, hsa-miR-4526, hsa-miR-4530, hsa-miR-4531, hsa-miR-4533, hsa-miR-4534, hsa-miR-378i, hsa-miR-4535, hsa-miR-1587, hsa-miR-4538, hsa-miR-4539, hsa-miR-3127-3p, hsa-miR-3150a-5p, hsa-miR-3158-5p, hsa-miR-3160-5p, hsa-miR-3162-3p, hsa-miR-3173-5p, hsa-miR-3187-5p, hsa-miR-3189-5p, hsa-miR-3619-3p, hsa-miR-3691-3p, hsa-miR-3150b-5p, hsa-miR-3922-5p, hsa-miR-3940-5p, hsa-miR-3960, hsa-miR-4634, hsa-miR-4635, hsa-miR-4638-5p, hsa-miR-4640-5p, hsa-miR-4640-3p, hsa-miR-4648, hsa-miR-4649-5p, hsa-miR-4649-3p, hsa-miR-4650-3p, hsa-miR-4651, hsa-miR-4652-5p, hsa-miR-4655-5p, hsa-miR-4656, hsa-miR-4658, hsa-miR-4661-3p, hsa-miR-4664-5p, hsa-miR-4664-3p, hsa-miR-4665-5p, hsa-miR-4665-3p, hsa-miR-4667-5p, hsa-miR-4667-3p, hsa-miR-4669, hsa-miR-4672, hsa-miR-4673, hsa-miR-4674, hsa-miR-4677-5p, hsa-miR-4684-5p, hsa-miR-4684-3p, hsa-miR-4685-5p, hsa-miR-4687-5p, hsa-miR-4687-3p, hsa-miR-1343-3p, hsa-miR-4688, hsa-miR-4689, hsa-miR-4690-5p, hsa-miR-4690-3p, hsa-miR-4691-5p, hsa-miR-4695-5p, hsa-miR-4697-5p, hsa-miR-4697-3p, hsa-miR-4706, hsa-miR-4707-5p, hsa-miR-4707-3p, hsa-miR-4708-5p, hsa-miR-4708-3p, hsa-miR-4710, hsa-miR-4716-5p, hsa-miR-4716-3p, hsa-miR-4717-3p, hsa-miR-4720-5p, hsa-miR-4721, hsa-miR-4723-5p, hsa-miR-4723-3p, hsa-miR-4725-5p, hsa-miR-4725-3p, hsa-miR-4726-5p, hsa-miR-4726-3p, hsa-miR-4727-3p, hsa-miR-4728-5p, hsa-miR-4728-3p, hsa-miR-4730, hsa-miR-4731-5p, hsa-miR-4731-3p, hsa-miR-4732-5p, hsa-miR-4734, hsa-miR-4736, hsa-miR-4738-3p, hsa-miR-4739, hsa-miR-4740-5p, hsa-miR-4740-3p, hsa-miR-4741, hsa-miR-4743-5p, hsa-miR-4745-5p, hsa-miR-4745-3p, hsa-miR-4746-3p, hsa-miR-4747-5p, hsa-miR-4748, hsa-miR-4749-5p, hsa-miR-4749-3p, hsa-miR-4750-5p, hsa-miR-371b-5p, hsa-miR-371b-3p, hsa-miR-4754, hsa-miR-4755-3p, hsa-miR-4756-5p, hsa-miR-4758-5p, hsa-miR-4758-3p, hsa-miR-4763-5p, hsa-miR-4763-3p, hsa-miR-4766-5p, hsa-miR-4767, hsa-miR-4769-5p, hsa-miR-4769-3p, hsa-miR-4776-5p, hsa-miR-4776-3p, hsa-miR-4778-5p, hsa-miR-4780, hsa-miR-4781-5p, hsa-miR-4783-5p, hsa-miR-4783-3p, hsa-miR-4785, hsa-miR-2467-3p, hsa-miR-4787-5p, hsa-miR-4788, hsa-miR-4793-3p, hsa-miR-4800-5p, hsa-miR-642a-3p, hsa-miR-4433a-5p, hsa-miR-4482-3p, hsa-miR-4999-5p, hsa-miR-5001-5p, hsa-miR-5001-3p, hsa-miR-5006-5p, hsa-miR-5006-3p, hsa-miR-5008-5p, hsa-miR-5009-3p, hsa-miR-5010-5p, hsa-miR-5088-5p, hsa-miR-5090, hsa-miR-5188, hsa-miR-5189-5p, hsa-miR-5190, hsa-miR-5195-3p, hsa-miR-5196-5p, hsa-miR-4524b-5p, hsa-miR-5100, hsa-miR-5572, hsa-miR-5584-3p, hsa-miR-5585-3p, hsa-miR-5587-5p, hsa-miR-5587-3p, hsa-miR-5589-5p, hsa-miR-5684, hsa-miR-4666b, hsa-miR-5698, hsa-miR-5703, hsa-miR-5705, hsa-miR-197-5p, hsa-miR-204-3p, hsa-miR-211-3p, hsa-miR-345-3p, hsa-miR-652-5p, hsa-miR-1185-2-3p, hsa-miR-873-3p, hsa-miR-1304-3p, hsa-miR-1247-3p, hsa-miR-3184-3p, hsa-miR-642b-5p, hsa-miR-365b-5p, hsa-miR-1185-1-3p, hsa-miR-381-5p, hsa-miR-937-5p, hsa-miR-1227-5p, hsa-miR-1229-5p, hsa-miR-1233-5p, hsa-miR-1237-5p, hsa-miR-1238-5p, hsa-miR-3620-5p, hsa-miR-4632-5p, hsa-miR-4743-3p, hsa-miR-4750-3p, hsa-miR-5739, hsa-miR-5787, hsa-miR-6068, hsa-miR-6069, hsa-miR-6071, hsa-miR-6074, hsa-miR-6075, hsa-miR-6076, hsa-miR-6080, hsa-miR-6081, hsa-miR-6083, hsa-miR-6085, hsa-miR-6086, hsa-miR-6088, hsa-miR-6089, hsa-miR-6090, hsa-miR-6124, hsa-miR-6125, hsa-miR-6126, hsa-miR-6127, hsa-miR-6131, hsa-miR-6132, hsa-miR-6133, hsa-miR-6165, hsa-miR-6499-5p, hsa-miR-6501-5p, hsa-miR-6503-3p, hsa-miR-6506-5p, hsa-miR-6510-5p, hsa-miR-6511a-5p, hsa-miR-6512-3p, hsa-miR-6513-5p, hsa-miR-6513-3p, hsa-miR-6514-5p, hsa-miR-6514-3p, hsa-miR-6515-5p, hsa-miR-6515-3p, hsa-miR-6715b-3p, hsa-miR-6716-5p, hsa-miR-6717-5p, hsa-miR-6511b-5p, hsa-miR-6511b-3p, hsa-miR-6719-3p, hsa-miR-6721-5p, hsa-miR-6722-5p, hsa-miR-6722-3p, hsa-miR-6724-5p, hsa-miR-208a-5p, hsa-miR-210-5p, hsa-miR-128-1-5p, hsa-miR-328-5p, hsa-miR-181d-3p, hsa-miR-504-3p, hsa-miR-487b-5p, hsa-miR-598-5p, hsa-miR-605-3p, hsa-miR-619-5p, hsa-miR-668-5p, hsa-miR-1296-3p, hsa-miR-1301-5p, hsa-miR-874-5p, hsa-miR-1180-5p, hsa-miR-1250-3p, hsa-miR-1266-3p, hsa-miR-1908-3p, hsa-miR-3928-5p, hsa-miR-1343-5p, hsa-miR-5189-3p, hsa-miR-5699-5p, hsa-miR-6720-5p, hsa-miR-6726-5p, hsa-miR-6727-5p, hsa-miR-6728-5p, hsa-miR-6729-5p, hsa-miR-6729-3p, hsa-miR-6730-5p, hsa-miR-6731-5p, hsa-miR-6731-3p, hsa-miR-6732-5p, hsa-miR-6734-5p, hsa-miR-6737-5p, hsa-miR-6738-5p, hsa-miR-6740-5p, hsa-miR-6741-5p, hsa-miR-6742-5p, hsa-miR-6743-5p, hsa-miR-6744-5p, hsa-miR-6745, hsa-miR-6746-5p, hsa-miR-6747-5p, hsa-miR-6748-5p, hsa-miR-6749-5p, hsa-miR-6749-3p, hsa-miR-6750-5p, hsa-miR-6751-5p, hsa-miR-6752-5p, hsa-miR-6752-3p, hsa-miR-6753-5p, hsa-miR-6754-5p, hsa-miR-6754-3p, hsa-miR-6756-5p, hsa-miR-6756-3p, hsa-miR-6757-5p, hsa-miR-6759-5p, hsa-miR-6759-3p, hsa-miR-6760-5p, hsa-miR-6760-3p, hsa-miR-6762-5p, hsa-miR-6762-3p, hsa-miR-6763-5p, hsa-miR-6763-3p, hsa-miR-6764-3p, hsa-miR-6765-5p, hsa-miR-6766-5p, hsa-miR-6766-3p, hsa-miR-6768-5p, hsa-miR-6769a-5p, hsa-miR-6770-5p, hsa-miR-6770-3p, hsa-miR-6772-5p, hsa-miR-6772-3p, hsa-miR-6774-5p, hsa-miR-6775-5p, hsa-miR-6775-3p, hsa-miR-6776-5p, hsa-miR-6777-5p, hsa-miR-6777-3p, hsa-miR-6778-5p, hsa-miR-6779-5p, hsa-miR-6781-5p, hsa-miR-6782-5p, hsa-miR-6782-3p, hsa-miR-6783-5p, hsa-miR-6784-5p, hsa-miR-6784-3p, hsa-miR-6785-3p, hsa-miR-6786-5p, hsa-miR-6786-3p, hsa-miR-6787-5p, hsa-miR-6787-3p, hsa-miR-6788-5p, hsa-miR-6788-3p, hsa-miR-6789-5p, hsa-miR-6789-3p, hsa-miR-6790-5p, hsa-miR-6790-3p, hsa-miR-6791-5p, hsa-miR-6792-5p, hsa-miR-6792-3p, hsa-miR-6793-3p, hsa-miR-6794-5p, hsa-miR-6795-5p, hsa-miR-6795-3p, hsa-miR-6796-5p, hsa-miR-6796-3p, hsa-miR-6797-5p, hsa-miR-6797-3p, hsa-miR-6798-5p, hsa-miR-6798-3p, hsa-miR-6799-5p, hsa-miR-6800-5p, hsa-miR-6800-3p, hsa-miR-6801-3p, hsa-miR-6802-5p, hsa-miR-6802-3p, hsa-miR-6803-5p, hsa-miR-6805-5p, hsa-miR-6805-3p, hsa-miR-6806-5p, hsa-miR-6808-5p, hsa-miR-6809-5p, hsa-miR-6810-5p, hsa-miR-6810-3p, hsa-miR-6811-3p, hsa-miR-6812-5p, hsa-miR-6812-3p, hsa-miR-6813-5p, hsa-miR-6813-3p, hsa-miR-6815-5p, hsa-miR-6816-5p, hsa-miR-6816-3p, hsa-miR-6819-5p, hsa-miR-6819-3p, hsa-miR-6820-5p, hsa-miR-6820-3p, hsa-miR-6821-5p, hsa-miR-6821-3p, hsa-miR-6822-5p, hsa-miR-6822-3p, hsa-miR-6823-5p, hsa-miR-6824-5p, hsa-miR-6825-5p, hsa-miR-6826-3p, hsa-miR-6827-5p, hsa-miR-6828-5p, hsa-miR-6829-5p, hsa-miR-6830-5p, hsa-miR-6831-5p, hsa-miR-6832-5p, hsa-miR-6832-3p, hsa-miR-6833-5p, hsa-miR-6834-5p, hsa-miR-6780b-5p, hsa-miR-6836-5p, hsa-miR-6836-3p, hsa-miR-6837-5p, hsa-miR-6838-5p, hsa-miR-6839-5p, hsa-miR-6840-5p, hsa-miR-6840-3p, hsa-miR-6841-3p, hsa-miR-6842-5p, hsa-miR-6845-5p, hsa-miR-6845-3p, hsa-miR-6846-5p, hsa-miR-6846-3p, hsa-miR-6848-5p, hsa-miR-6848-3p, hsa-miR-6849-5p, hsa-miR-6850-5p, hsa-miR-6851-5p, hsa-miR-6851-3p, hsa-miR-6852-5p, hsa-miR-6852-3p, hsa-miR-6855-5p, hsa-miR-6855-3p, hsa-miR-6856-5p, hsa-miR-6857-5p, hsa-miR-6857-3p, hsa-miR-6858-5p, hsa-miR-6858-3p, hsa-miR-6859-3p, hsa-miR-6769b-5p, hsa-miR-6769b-3p, hsa-miR-6860, hsa-miR-6861-5p, hsa-miR-6861-3p, hsa-miR-6862-5p, hsa-miR-6865-3p, hsa-miR-6868-3p, hsa-miR-6869-5p, hsa-miR-6870-5p, hsa-miR-6870-3p, hsa-miR-6871-5p, hsa-miR-6872-5p, hsa-miR-6873-5p, hsa-miR-6874-5p, hsa-miR-6875-5p, hsa-miR-6875-3p, hsa-miR-6876-5p, hsa-miR-6877-5p, hsa-miR-6879-5p, hsa-miR-6880-5p, hsa-miR-6880-3p, hsa-miR-6881-5p, hsa-miR-6883-5p, hsa-miR-6884-3p, hsa-miR-6885-5p, hsa-miR-6885-3p, hsa-miR-6886-5p, hsa-miR-6887-5p, hsa-miR-6887-3p, hsa-miR-6889-5p, hsa-miR-6889-3p, hsa-miR-6891-5p, hsa-miR-6891-3p, hsa-miR-6892-5p, hsa-miR-6892-3p, hsa-miR-6893-5p, hsa-miR-6894-5p, hsa-miR-6895-5p, hsa-miR-6895-3p, hsa-miR-7106-5p, hsa-miR-7107-5p, hsa-miR-7108-5p, hsa-miR-7108-3p, hsa-miR-7109-5p, hsa-miR-7109-3p, hsa-miR-7110-5p, hsa-miR-7111-Sp, hsa-miR-7112-5p, hsa-miR-7113-3p, hsa-miR-7114-5p, hsa-miR-7114-3p, hsa-miR-7150, hsa-miR-7151-3p, hsa-miR-7152-5p, hsa-miR-7152-3p, hsa-miR-7155-5p, hsa-miR-7155-3p, hsa-miR-7160-5p, hsa-miR-7515, hsa-miR-7704, hsa-miR-7843-5p, hsa-miR-4433b-5p, hsa-miR-4433b-3p, hsa-miR-1273h-5p, hsa-miR-1273h-3p, hsa-miR-6516-5p, hsa-miR-7845-5p, hsa-miR-7846-3p, hsa-miR-7847-3p, hsa-miR-7851-3p, hsa-miR-7855-5p, hsa-miR-7856-5p, hsa-miR-8052, hsa-miR-8059, hsa-miR-8060, hsa-miR-8062, hsa-miR-8063, hsa-miR-8064, hsa-miR-8069, hsa-miR-8071, hsa-miR-8072, hsa-miR-8073, hsa-miR-8074, hsa-miR-8077, hsa-miR-8078, hsa-miR-8085, hsa-miR-8089, hsa-miR-128-2-5p, hsa-miR-548ba, hsa-miR-7977, hsa-miR-1249-5p, hsa-miR-4485-5p, hsa-miR-103a-1-5p, hsa-miR-217-3p, hsa-miR-135a-2-3p, hsa-miR-498-3p, hsa-miR-526a-3p, hsa-miR-9718, hsa-miR-9898, hsa-miR-9902, hsa-miR-10392-5p, hsa-miR-10392-3p, hsa-miR-10394-3p, hsa-miR-10396a-5p, hsa-miR-10396a-3p, hsa-miR-10398-3p, hsa-miR-10400-5p, hsa-miR-10400-3p, hsa-miR-10401-5p, hsa-miR-10401-3p, hsa-miR-10396b-5p, hsa-miR-10522-5p, hsa-miR-10526-3p, hsa-miR-10527-5p, hsa-miR-11181-3p, hsa-miR-11399, hsa-miR-11401, hsa-miR-3059-3p, hsa-miR-3085-5p, hsa-miR-3085-3p, hsa-miR-12114, hsa-miR-12115, hsa-miR-12116, hsa-miR-12118, hsa-miR-12119, hsa-miR-12120, hsa-miR-12121, hsa-miR-12122, hsa-miR-12126, hsa-miR-12128, hsa-miR-12131, and hsa-miR-12136. In the present disclosure, the extract of urine may be an extract of the urine of a stage-I or -II gastric cancer patient. Only one of these microRNAs can be used to predict whether a subject from which the urine is derived is a gastric cancer patient (particularly stage-I or -II), and 5 or more types, 10 or more types, 15 or more types, or 20 or more types of these microRNAs can be used in combination to predict whether a subject from which the urine is derived is a gastric cancer patient (particularly stage-I or - II). Among these microRNAs, a microRNA that may be highly expressed in a gastric cancer patient (particularly stage-I or -II) can be preferably used for prediction.

The present disclosure provides an extract of urine containing at least one type, two types, three types, four types, five types, six types, seven types, eight types, nine types, or ten types of or all microRNAs selected from the group consisting of hsa-miR-29a-3p, hsa-miR-182-5p, hsa-miR-299-3p, hsa-miR-130b-3p, hsa-miR-361-5p, hsa-miR-200a-5p, hsa-miR-202-3p, hsa-miR-492, hsa-miR-494-3p, hsa-miR-498-5p, hsa-miR-519c-5p, hsa-miR-523-5p, hsa-miR-518e-5p, hsa-miR-522-5p, hsa-miR-519a-5p, hsa-miR-519b-5p, hsa-miR-525-5p, hsa-miR-516b-5p, hsa-miR-554, hsa-miR-576-5p, hsa-miR-601, hsa-miR-611, hsa-miR-617, hsa-miR-628-3p, hsa-miR-638, hsa-miR-639, hsa-miR-650, hsa-miR-449b-5p, hsa-miR-421, hsa-miR-542-5p, hsa-let-7f-1-3p, hsa-miR-25-5p, hsa-miR-92a-2-5p, hsa-miR-181c-3p, hsa-miR-187-5p, hsa-miR-15b-3p, hsa-miR-124-5p, hsa-miR-125b-1-3p, hsa-miR-135a-3p, hsa-miR-150-3p, hsa-miR-193a-5p, hsa-miR-339-3p, hsa-miR-424-3p, hsa-miR-20b-3p, hsa-miR-490-5p, hsa-miR-193b-5p, hsa-miR-92b-5p, hsa-miR-551b-5p, hsa-miR-629-5p, hsa-miR-541-3p, hsa-miR-665, hsa-miR-921, hsa-miR-939-5p, hsa-miR-1225-5p, hsa-miR-513b-5p, hsa-miR-320c, hsa-miR-1301-3p, hsa-miR-1298-5p, hsa-miR-1207-5p, hsa-miR-1303, hsa-miR-1304-5p, hsa-miR-1243, hsa-miR-1270, hsa-miR-1292-5p, hsa-miR-1469, hsa-miR-1908-5p, hsa-miR-1914-3p, hsa-miR-1915-3p, hsa-miR-449c-5p, hsa-miR-762, hsa-miR-2114-5p, hsa-miR-2114-3p, hsa-miR-2681-3p, hsa-miR-3122, hsa-miR-3124-5p, hsa-miR-3133, hsa-miR-3141, hsa-miR-1273c, hsa-miR-3163, hsa-miR-3165, hsa-miR-3065-5p, hsa-miR-3187-3p, hsa-miR-320e, hsa-miR-3197, hsa-miR-4295, hsa-miR-4294, hsa-miR-4315, hsa-miR-4321, hsa-miR-4257, hsa-miR-4258, hsa-miR-4327, hsa-miR-4261, hsa-miR-4280, hsa-miR-4285, hsa-miR-3200-5p, hsa-miR-3648, hsa-miR-3651, hsa-miR-3660, hsa-miR-3666, hsa-miR-3677-3p, hsa-miR-3907, hsa-miR-3909, hsa-miR-3918, hsa-miR-3937, hsa-miR-1268b, hsa-miR-4429, hsa-miR-4433a-3p, hsa-miR-4435, hsa-miR-4439, hsa-miR-4447, hsa-miR-4454, hsa-miR-4458, hsa-miR-4465, hsa-miR-4466, hsa-miR-4470, hsa-miR-4479, hsa-miR-4484, hsa-miR-4485-3p, hsa-miR-4505, hsa-miR-4507, hsa-miR-4512, hsa-miR-4516, hsa-miR-4521, hsa-miR-1269b, hsa-miR-4530, hsa-miR-3127-3p, hsa-miR-4634, hsa-miR-4635, hsa-miR-4650-3p, hsa-miR-4651, hsa-miR-4658, hsa-miR-4665-5p, hsa-miR-4672, hsa-miR-4673, hsa-miR-4677-5p, hsa-miR-4695-5p, hsa-miR-4706, hsa-miR-4707-5p, hsa-miR-4720-5p, hsa-miR-4725-3p, hsa-miR-4727-3p, hsa-miR-4741, hsa-miR-4742-3p, hsa-miR-4745-5p, hsa-miR-4745-3p, hsa-miR-4749-5p, hsa-miR-4755-3p, hsa-miR-4758-5p, hsa-miR-4763-3p, hsa-miR-4766-5p, hsa-miR-4778-5p, hsa-miR-550a-3-5p, hsa-miR-4999-5p, hsa-miR-5009-3p, hsa-miR-5190, hsa-miR-5196-5p, hsa-miR-4524b-5p, hsa-miR-5587-5p, hsa-miR-5698, hsa-miR-211-3p, hsa-miR-381-5p, hsa-miR-937-5p, hsa-miR-1227-5p, hsa-miR-3620-5p, hsa-miR-6071, hsa-miR-6080, hsa-miR-6081, hsa-miR-6089, hsa-miR-6125, hsa-miR-6131, hsa-miR-6500-3p, hsa-miR-6501-5p, hsa-miR-6501-3p, hsa-miR-6513-5p, hsa-miR-6514-5p, hsa-miR-6515-5p, hsa-miR-6715b-3p, hsa-miR-6719-3p, hsa-miR-181d-3p, hsa-miR-598-5p, hsa-miR-605-3p, hsa-miR-668-5p, hsa-miR-1343-5p, hsa-miR-6729-5p, hsa-miR-6731-3p, hsa-miR-6732-5p, hsa-miR-6743-5p, hsa-miR-6746-5p, hsa-miR-6750-5p, hsa-miR-6752-5p, hsa-miR-6756-5p, hsa-miR-6760-3p, hsa-miR-6763-5p, hsa-miR-6770-5p, hsa-miR-6770-3p, hsa-miR-6777-5p, hsa-miR-6781-5p, hsa-miR-6783-5p, hsa-miR-6784-5p, hsa-miR-6786-5p, hsa-miR-6789-5p, hsa-miR-6790-3p, hsa-miR-6791-5p, hsa-miR-6794-5p, hsa-miR-6797-5p, hsa-miR-6798-5p, hsa-miR-6799-5p, hsa-miR-6805-5p, hsa-miR-6809-5p, hsa-miR-6810-3p, hsa-miR-6814-5p, hsa-miR-6815-5p, hsa-miR-6821-5p, hsa-miR-6829-5p, hsa-miR-6780b-5p, hsa-miR-6836-5p, hsa-miR-6836-3p, hsa-miR-6838-5p, hsa-miR-6839-5p, hsa-miR-6840-3p, hsa-miR-6845-5p, hsa-miR-6848-5p, hsa-miR-6850-5p, hsa-miR-6850-3p, hsa-miR-6861-5p, hsa-miR-6875-5p, hsa-miR-6892-3p, hsa-miR-6893-5p, hsa-miR-7107-5p, hsa-miR-7111-5p, hsa-miR-7114-5p, hsa-miR-7150, hsa-miR-7515, hsa-miR-4433b-3p, hsa-miR-6516-5p, hsa-miR-8072, hsa-miR-8074, hsa-miR-1249-5p, hsa-miR-217-3p, hsa-miR-135a-2-3p, hsa-miR-498-3p, hsa-miR-526a-3p, hsa-miR-10392-5p, hsa-miR-10392-3p, hsa-miR-10396a-3p, hsa-miR-10400-3p, hsa-miR-10401-5p, hsa-miR-10396b-5p, hsa-miR-10522-5p, hsa-miR-10526-3p, hsa-miR-11399, hsa-miR-12119, hsa-miR-12120, hsa-miR-12122, and hsa-miR-121312. In the present disclosure, the extract of urine may be an extract of the urine of a stage-I gastric cancer patient. Only one of these microRNAs can be used to predict whether a subject from which the urine is derived is a gastric cancer patient (particularly stage-I), and 5 or more types, 10 or more types, 15 or more types, or 20 or more types of these microRNAs can be used in combination to predict whether a subject from which the urine is derived is a gastric cancer patient (particularly stage-I). Among these microRNAs, a microRNA that may be highly expressed in a gastric cancer patient (particularly stage-I) can be preferably used for prediction.

The present disclosure provides an extract of urine containing at least one type, two types, three types, four types, five types, six types, seven types, eight types, nine types, or ten types of or all microRNAs selected from the group consisting of microRNAs having an accuracy of higher than 0.500 among the microRNAs listed in Table 24-8. In the present disclosure, the extract of urine may be an extract of the urine of a gastric cancer patient. Only one of these microRNAs can be used to predict whether a subject from which the urine is derived is a gastric cancer patient, and 5 or more types, 10 or more types, 15 or more types, or 20 or more types of these microRNAs can be used in combination to predict whether a subject from which the urine is derived is a gastric cancer patient. Among these microRNAs, a microRNA that may be highly expressed in a gastric cancer patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

The present disclosure provides an extract of urine containing at least one type, two types, three types, four types, five types, six types, seven types, eight types, nine types, or ten types of or all microRNAs selected from the group consisting of microRNAs having an accuracy of higher than 0.500 among the microRNAs listed in Table 25-8. In the present disclosure, the extract of urine may be an extract of the urine of a gastric cancer patient. Only one of these microRNAs can be used to predict whether a subject from which the urine is derived is a gastric cancer patient, and 5 or more types, 10 or more types, 15 or more types, or 20 or more types of these microRNAs can be used in combination to predict whether a subject from which the urine is derived is a gastric cancer patient. Among these microRNAs, a microRNA that may be highly expressed in a gastric cancer patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

The present disclosure provides an extract of urine containing at least one type, two types, three types, four types, five types, six types, seven types, eight types, nine types, or ten types of or all microRNAs selected from the microRNAs listed in Table 4-9. The present disclosure also provides an extract of urine containing at least one type, two types, three types, four types, five types, six types, seven types, eight types, nine types, or ten types of or all microRNAs selected from the group consisting of hsa-miR-150-3p, hsa-miR-4447, hsa-miR-629-5p, hsa-miR-498-5p, hsa-miR-937-5p, hsa-miR-6756-5p, hsa-miR-4433b-3p, hsa-miR-4433a-3p, hsa-miR-5196-5p, hsa-miR-6763-3p, hsa-miR-3937, hsa-miR-4725-3p, hsa-miR-551b-5p, hsa-miR-6891-3p, hsa-miR-6794-5p, hsa-miR-6780b-5p, hsa-miR-4433a-5p, hsa-miR-4327, hsa-miR-7108-3p, hsa-miR-4257, hsa-miR-4665-5p, hsa-miR-6805-3p, hsa-miR-12120, hsa-miR-1224-3p, hsa-miR-1228-3p, hsa-miR-9898, hsa-miR-6785-3p, hsa-miR-4433b-5p, hsa-miR-1249-3p, hsa-miR-6796-3p, hsa-miR-6859-3p, hsa-miR-4716-5p, hsa-miR-3162-3p, hsa-miR-6731-3p, hsa-miR-6799-5p, hsa-miR-1238-3p, hsa-miR-202-3p, hsa-miR-6729-3p, hsa-miR-1225-3p, hsa-miR-6800-3p, hsa-miR-6777-3p, hsa-miR-6784-3p, hsa-miR-3648, hsa-miR-6798-3p, hsa-miR-6750-5p, hsa-miR-4749-5p, hsa-miR-4323, hsa-miR-7156-3p, hsa-miR-619-5p, hsa-miR-6760-3p, hsa-miR-4750-3p, hsa-miR-92a-2-5p, hsa-miR-7150, hsa-miR-4749-3p, hsa-miR-1237-3p, hsa-miR-10400-3p, hsa-miR-6892-3p, hsa-miR-6813-3p, hsa-miR-6880-3p, hsa-miR-4665-3p, hsa-miR-1268b, hsa-miR-12116, hsa-miR-7114-3p, hsa-miR-4274, hsa-miR-1343-5p, hsa-miR-6810-3p, hsa-miR-3141, hsa-miR-6829-5p, hsa-miR-1913, hsa-miR-193b-5p, hsa-miR-7111-5p, hsa-miR-6515-3p, hsa-miR-6861-5p, hsa-miR-6858-3p, hsa-miR-761, hsa-miR-4758-5p, hsa-miR-4258, hsa-miR-6797-5p, hsa-miR-1185-2-3p, hsa-miR-210-5p, hsa-miR-6795-3p, hsa-miR-921, hsa-miR-1207-5p, hsa-miR-6766-3p, hsa-miR-6756-3p, hsa-miR-6845-3p, hsa-miR-6812-5p, hsa-miR-6752-3p, hsa-miR-3675-3p, hsa-miR-4728-3p, hsa-miR-6812-3p, hsa-miR-940, hsa-miR-3679-3p, hsa-miR-193a-5p, hsa-miR-1227-5p, hsa-miR-7109-5p, hsa-miR-4731-3p, hsa-miR-625-3p, hsa-miR-6819-3p, hsa-miR-4507, hsa-miR-1185-1-3p, hsa-miR-6749-5p, hsa-miR-5698, hsa-miR-6821-5p, hsa-miR-3162-5p, hsa-miR-1908-5p, hsa-miR-8080, hsa-miR-4651, hsa-miR-365a-5p, hsa-miR-6887-3p, hsa-miR-4321, hsa-miR-6787-5p, hsa-miR-6763-5p, hsa-miR-4687-5p, hsa-miR-12119, hsa-miR-7851-3p, hsa-miR-106a-3p, hsa-miR-6781-5p, hsa-miR-4727-3p, hsa-miR-3614-5p, hsa-miR-4313, hsa-miR-6124, hsa-miR-4685-5p, hsa-miR-5006-5p, hsa-miR-6501-5p, hsa-miR-4294, hsa-miR-541-3p, hsa-miR-1236-3p, hsa-miR-6870-3p, hsa-miR-4465, hsa-miR-1470, hsa-miR-184, hsa-miR-4439, hsa-miR-4654, hsa-miR-6761-5p, hsa-miR-1202, hsa-miR-4284, hsa-miR-10526-3p, hsa-miR-7515, hsa-miR-18b-3p, hsa-miR-4463, hsa-miR-4648, hsa-miR-6743-5p, hsa-miR-3943, hsa-miR-4298, hsa-miR-361-3p, hsa-miR-92b-5p, hsa-miR-4783-3p, hsa-miR-3085-3p, hsa-miR-1972, hsa-miR-3917, hsa-miR-6848-5p, hsa-miR-3179, hsa-miR-8064, hsa-miR-10401-5p, hsa-miR-6809-5p, hsa-miR-4763-3p, hsa-miR-760, hsa-miR-6766-5p, hsa-miR-7151-3p, hsa-miR-4538, hsa-miR-4314, hsa-miR-9718, hsa-miR-5190, hsa-miR-6884-3p, hsa-miR-5585-3p, hsa-miR-1304-3p, hsa-miR-8077, hsa-miR-3197, hsa-miR-6791-5p, hsa-miR-3163, hsa-miR-6752-5p, hsa-miR-6848-3p, hsa-miR-6783-5p, hsa-miR-296-5p, hsa-miR-3184-3p, hsa-miR-4530, hsa-miR-6790-3p, hsa-miR-4753-5p, hsa-miR-211-3p, hsa-miR-4286, hsa-miR-6776-5p, hsa-miR-5194, hsa-miR-617, hsa-miR-484, hsa-let-7e-5p, hsa-miR-4489, hsa-miR-6131, hsa-miR-6768-5p, hsa-miR-3620-5p, hsa-miR-365a-3p, hsa-miR-365b-3p, hsa-miR-4299, hsa-miR-6782-5p, hsa-miR-885-3p, hsa-miR-197-5p, hsa-miR-7114-5p, hsa-miR-6732-5p, hsa-miR-6797-3p, hsa-miR-6069, hsa-miR-7847-3p, hsa-miR-3652, hsa-miR-1281, hsa-miR-373-3p, hsa-miR-4458, hsa-miR-6836-3p, hsa-miR-6775-5p, hsa-miR-373-5p, hsa-miR-6859-5p, hsa-miR-6840-3p, hsa-miR-769-5p, hsa-miR-6823-5p, hsa-miR-3127-3p, hsa-miR-3922-5p, hsa-miR-3150b-5p, hsa-miR-4769-3p, hsa-miR-10396a-3p, hsa-miR-20b-3p, hsa-miR-3619-5p, hsa-miR-1225-5p, hsa-miR-10392-5p, hsa-miR-6846-5p, hsa-miR-6748-5p, hsa-miR-3619-3p, hsa-miR-2681-3p, hsa-miR-515-5p, hsa-miR-1236-5p, hsa-miR-4741, hsa-miR-4535, hsa-miR-1587, hsa-miR-4748, hsa-miR-3137, hsa-miR-5010-5p, hsa-miR-6871-5p, hsa-miR-12114, hsa-miR-8059, hsa-miR-650, hsa-miR-6769b-5p, hsa-miR-6790-5p, hsa-miR-4784, hsa-miR-6747-5p, hsa-miR-6798-5p, hsa-miR-6861-3p, hsa-miR-939-5p, hsa-miR-320c, hsa-miR-130b-3p, hsa-miR-6841-3p, hsa-miR-4632-5p, hsa-miR-6879-5p, hsa-miR-7110-5p, hsa-miR-4487, hsa-miR-598-5p, hsa-miR-557, hsa-miR-6814-5p, hsa-miR-11399, hsa-miR-6893-5p, hsa-miR-6870-5p, hsa-miR-1292-5p, hsa-miR-1234-3p, hsa-miR-595, hsa-miR-4695-5p, hsa-miR-3190-3p, hsa-miR-323a-3p, hsa-miR-135a-3p, hsa-miR-4755-3p, hsa-miR-6805-5p, hsa-miR-6777-5p, hsa-miR-2467-3p, hsa-miR-4649-3p, hsa-miR-8072, hsa-miR-3199, hsa-miR-1914-3p, hsa-miR-6749-3p, hsa-miR-6832-5p, hsa-miR-3180-5p, hsa-miR-874-5p, hsa-miR-3059-3p, hsa-miR-5007-5p, hsa-miR-149-3p, hsa-miR-4708-3p, hsa-miR-6839-5p, hsa-miR-5189-5p, hsa-miR-3622a-5p, hsa-miR-575, hsa-miR-1469, hsa-miR-371b-5p, hsa-miR-6125, hsa-miR-3150b-3p, hsa-miR-6772-5p, hsa-miR-605-3p, hsa-miR-6722-3p, hsa-miR-1321, hsa-miR-4505, hsa-miR-601, hsa-miR-1303, hsa-miR-548g-3p, hsa-miR-658, hsa-miR-6845-5p, hsa-miR-6127, hsa-miR-7154-3p, hsa-miR-6086, hsa-miR-139-3p, hsa-miR-4498, hsa-miR-6803-5p, hsa-miR-6775-3p, hsa-miR-2276-3p, hsa-miR-135a-2-3p, hsa-miR-4289, hsa-miR-6796-5p, hsa-miR-4640-3p, hsa-miR-6824-5p, hsa-miR-6721-5p, hsa-miR-1229-5p, hsa-miR-1471, hsa-miR-769-3p, hsa-miR-206, hsa-miR-1268a, hsa-miR-6855-5p, hsa-miR-608, hsa-miR-500b-5p, hsa-miR-4300, hsa-miR-4769-5p, hsa-miR-6786-5p, hsa-miR-4316, hsa-miR-4428, hsa-miR-4701-3p, hsa-miR-6165, hsa-miR-4271, hsa-miR-4478, hsa-miR-642b-3p, hsa-miR-4745-5p, hsa-miR-6833-5p, hsa-miR-4436b-3p, hsa-miR-6083, hsa-miR-550a-3-5p, hsa-miR-204-3p, hsa-miR-4451, hsa-miR-4514, hsa-miR-6789-5p, hsa-miR-7108-5p, hsa-miR-4638-5p, hsa-miR-4740-5p, hsa-miR-4707-5p, hsa-miR-8071, hsa-miR-3120-3p, hsa-miR-4482-3p, hsa-miR-6847-5p, hsa-miR-1285-3p, hsa-miR-5703, hsa-miR-4673, hsa-miR-2116-3p, hsa-miR-7854-3p, hsa-miR-6515-5p, hsa-miR-6855-3p, hsa-miR-4280, hsa-miR-12122, hsa-miR-4750-5p, hsa-miR-194-3p, hsa-miR-3186-3p, hsa-miR-7107-5p, hsa-miR-663a, hsa-miR-665, hsa-miR-6849-5p, hsa-miR-8057, hsa-miR-3945, hsa-miR-3151-5p, hsa-miR-3074-3p, hsa-miR-4720-5p, hsa-miR-1226-5p, hsa-miR-8089, hsa-miR-4481, hsa-miR-6779-5p, hsa-miR-504-5p, hsa-miR-6856-5p, hsa-miR-6834-5p, hsa-miR-4475, hsa-miR-3180-3p, hsa-miR-6889-5p, hsa-miR-7113-5p, hsa-miR-6751-5p, hsa-miR-11181-3p, hsa-miR-5090, hsa-miR-138-5p, hsa-miR-1909-3p, hsa-miR-8075, hsa-miR-4486, hsa-miR-10398-3p, hsa-miR-4484, hsa-miR-6793-5p, hsa-miR-3135b, hsa-miR-4706, hsa-miR-718, hsa-miR-6825-5p, hsa-miR-2115-5p, hsa-miR-4681, hsa-miR-3660, hsa-miR-4672, hsa-miR-6778-5p, hsa-miR-6759-5p, hsa-miR-6741-5p, hsa-miR-5584-5p, hsa-miR-7160-5p, hsa-miR-6858-5p, hsa-miR-518b, hsa-miR-6895-5p, hsa-miR-6717-5p, hsa-miR-4691-5p, hsa-miR-4723-3p, hsa-miR-125b-2-3p, hsa-miR-6500-3p, hsa-miR-4521, hsa-miR-6076, hsa-miR-4758-3p, hsa-miR-4778-5p, hsa-miR-4684-3p, hsa-miR-487a-5p, hsa-miR-5572, hsa-miR-4664-5p, hsa-miR-25-5p, hsa-miR-504-3p, hsa-miR-3679-5p, hsa-miR-6081, hsa-miR-6767-5p, hsa-miR-3911, hsa-miR-187-5p, hsa-miR-6802-3p, hsa-miR-6875-5p, hsa-miR-664b-5p, hsa-miR-6724-5p, hsa-miR-3122, hsa-miR-4421, hsa-miR-3675-5p, hsa-miR-4440, hsa-miR-4456, hsa-miR-185-3p, hsa-miR-3187-3p, hsa-miR-4526, hsa-miR-5708, hsa-miR-4676-5p, hsa-miR-6842-5p, hsa-miR-8085, hsa-miR-3142, hsa-miR-4743-3p, hsa-miR-5739, hsa-miR-183-3p, hsa-miR-6506-5p, hsa-miR-6894-5p, hsa-miR-8078, hsa-miR-371a-5p, hsa-miR-4450, hsa-miR-4497, hsa-miR-6831-5p, hsa-miR-6852-5p, hsa-miR-4429, hsa-miR-6070, hsa-miR-6820-5p, hsa-miR-1304-5p, hsa-miR-711, hsa-miR-6892-5p, hsa-miR-668-5p, hsa-miR-3928-3p, hsa-miR-4430, hsa-miR-4506, hsa-miR-4743-5p, hsa-miR-6792-3p, hsa-miR-3155a, hsa-miR-4265, hsa-miR-3158-3p, hsa-miR-4640-5p, hsa-miR-4653-3p, hsa-miR-6746-5p, hsa-miR-6889-3p, hsa-miR-3649, hsa-miR-4483, hsa-miR-4435, hsa-miR-6071, hsa-miR-6511a-5p, hsa-miR-6510-5p, hsa-miR-30b-3p, hsa-miR-5093, hsa-miR-6792-5p, hsa-miR-2110, hsa-miR-5001-5p, hsa-miR-10396a-5p, hsa-miR-4800-5p, hsa-miR-2392, hsa-miR-5192, hsa-miR-3085-5p, hsa-miR-4700-5p, hsa-miR-493-3p, hsa-miR-1266-3p, hsa-miR-328-3p, hsa-miR-1293, hsa-miR-1273h-5p, hsa-miR-6726-5p, hsa-miR-4472, hsa-miR-371b-3p, hsa-miR-3155b, hsa-miR-3180, hsa-miR-3124-5p, hsa-miR-6511b-5p, hsa-miR-4779, hsa-miR-4686, hsa-miR-5702, hsa-miR-4716-3p, hsa-miR-4747-5p, hsa-miR-10392-3p, hsa-miR-5584-3p, hsa-miR-4322, hsa-miR-548q, hsa-miR-6885-3p, hsa-miR-3689a-3p, hsa-miR-3185, hsa-miR-11401, hsa-miR-105-5p, hsa-miR-5004-3p, hsa-miR-3131, hsa-miR-6793-3p, hsa-miR-5589-5p, hsa-miR-6886-5p, hsa-miR-4540, hsa-miR-4776-5p, hsa-miR-3622b-5p, hsa-miR-297, hsa-miR-4669, hsa-miR-12121, hsa-miR-1270, hsa-miR-3919, hsa-miR-10396b-5p, hsa-miR-6134, hsa-miR-6820-3p, hsa-miR-5100, hsa-miR-4697-5p, hsa-miR-6729-5p, hsa-miR-593-3p, hsa-miR-492, hsa-miR-6801-3p, hsa-miR-483-5p, hsa-miR-6802-5p, hsa-miR-424-3p, hsa-miR-6740-5p, hsa-miR-1224-5p, hsa-miR-3161, hsa-miR-6877-5p, hsa-miR-1237-5p, hsa-miR-3713, hsa-miR-4740-3p, hsa-miR-3689d, hsa-miR-4763-5p, hsa-miR-449b-5p, hsa-miR-501-5p, hsa-miR-6880-5p, hsa-miR-4655-5p, hsa-miR-938, hsa-miR-12118, hsa-miR-639, hsa-miR-584-5p, hsa-miR-638, hsa-miR-3934-5p, hsa-miR-378a-3p, hsa-miR-3186-5p, hsa-miR-4539, hsa-miR-485-5p, hsa-miR-370-3p, hsa-miR-4466, hsa-miR-4738-3p, hsa-miR-4502, hsa-miR-3691-5p, hsa-miR-1915-3p, hsa-miR-708-5p, hsa-miR-3173-3p, hsa-miR-3682-3p, hsa-miR-210-3p, hsa-miR-222-5p, hsa-miR-4462, hsa-miR-3616-3p, hsa-miR-200a-5p, hsa-miR-1468-5p, hsa-miR-3610, hsa-miR-8070, hsa-miR-4533, hsa-miR-6514-5p, hsa-miR-298, hsa-miR-3689b-3p, hsa-miR-3689c, hsa-miR-516a-5p, hsa-miR-6817-5p, hsa-miR-6874-5p, hsa-miR-4999-5p, hsa-miR-4667-3p, hsa-miR-6132, hsa-miR-6876-5p, hsa-miR-3187-5p, hsa-miR-4496, hsa-miR-1247-3p, hsa-miR-300, hsa-miR-6737-5p, hsa-miR-4707-3p, hsa-miR-4776-3p, hsa-miR-6826-3p, hsa-miR-6762-3p, hsa-miR-6830-5p, hsa-miR-6769a-5p, hsa-miR-363-5p, hsa-miR-612, hsa-miR-3138, hsa-miR-4710, hsa-miR-1285-5p, hsa-miR-196a-5p, hsa-miR-382-5p, hsa-miR-4303, hsa-miR-5088-5p, hsa-miR-7113-3p, hsa-miR-4644, hsa-miR-200b-5p, hsa-miR-4276, hsa-miR-1249-5p, hsa-miR-6075, hsa-miR-675-5p, hsa-miR-296-3p, hsa-miR-6755-5p, hsa-miR-3654, hsa-miR-6860, hsa-miR-4754, hsa-miR-7850-5p, hsa-miR-6085, hsa-miR-128-1-5p, hsa-miR-6884-5p, hsa-miR-5588-3p, hsa-miR-758-5p, hsa-miR-877-5p, hsa-miR-491-5p, hsa-miR-4513, hsa-miR-6838-5p, hsa-miR-6801-5p, hsa-miR-6788-5p, hsa-miR-4736, hsa-miR-874-3p, hsa-miR-6126, hsa-miR-323b-5p, hsa-miR-3154, hsa-miR-5705, hsa-miR-4682, hsa-miR-4711-3p, hsa-miR-6784-5p, hsa-miR-4431, hsa-miR-4449, hsa-miR-6765-5p, hsa-miR-4746-3p, hsa-miR-26b-3p, hsa-miR-564, hsa-miR-6503-3p, hsa-miR-887-3p, hsa-miR-6753-5p, hsa-miR-1301-5p, hsa-miR-4708-5p, hsa-miR-5704, hsa-miR-494-3p, hsa-miR-4520-5p, hsa-miR-6822-3p, hsa-miR-4688, hsa-miR-744-5p, hsa-miR-6795-5p, hsa-miR-4667-5p, hsa-miR-5006-3p, hsa-miR-192-3p, hsa-miR-3158-5p, hsa-miR-1343-3p, hsa-miR-4467, hsa-miR-3176, hsa-miR-6758-5p, hsa-miR-4260, hsa-miR-6887-5p, hsa-miR-3173-5p, hsa-miR-5004-5p, hsa-miR-652-5p, hsa-miR-3934-3p, hsa-miR-892b, hsa-miR-3153, hsa-miR-5580-5p, hsa-miR-4721, hsa-miR-4664-3p, hsa-miR-4474-3p, hsa-miR-8063, hsa-miR-6770-5p, hsa-miR-510-5p, hsa-miR-486-5p, hsa-miR-3130-3p, hsa-miR-5188, hsa-miR-7151-5p, hsa-miR-3191-3p, hsa-miR-1255b-5p, hsa-miR-8060, hsa-miR-4454, hsa-miR-3195, hsa-miR-125b-1-3p, hsa-miR-6808-5p, hsa-miR-6511b-3p, hsa-miR-920, hsa-miR-6851-5p, hsa-miR-500b-3p, hsa-miR-3612, hsa-miR-4781-5p, hsa-miR-151a-5p, hsa-miR-584-3p, hsa-miR-7845-5p, hsa-miR-200c-5p, hsa-miR-3177-3p, hsa-miR-12124, hsa-let-7d-3p, hsa-miR-323a-5p, hsa-miR-7152-3p, hsa-miR-4436a, hsa-miR-4786-3p, hsa-miR-6772-3p, hsa-miR-3689f, hsa-miR-6516-5p, hsa-miR-128-3p, hsa-miR-766-5p, hsa-miR-452-3p, hsa-miR-766-3p, hsa-miR-122b-3p, hsa-miR-3133, hsa-miR-6810-5p, hsa-miR-129-5p, hsa-miR-1193, hsa-miR-3944-5p, hsa-miR-10a-5p, hsa-miR-6759-3p, hsa-miR-6762-5p, hsa-miR-1538, hsa-miR-550a-5p, hsa-miR-9986, hsa-miR-933, hsa-miR-5187-5p, hsa-miR-1238-5p, hsa-miR-3132, hsa-miR-8074, hsa-miR-4731-5p, hsa-miR-3175, hsa-miR-7106-5p, hsa-miR-642a-3p, hsa-miR-423-5p, hsa-miR-6819-5p, hsa-miR-1204, hsa-miR-4453, hsa-miR-1296-3p, hsa-miR-498-3p, hsa-miR-8052, hsa-miR-5189-3p, hsa-miR-4448, hsa-miR-3156-5p, hsa-miR-18a-3p, hsa-miR-3936, hsa-miR-4529-5p, hsa-miR-1294, hsa-miR-4525, hsa-miR-516b-5p, hsa-miR-6782-3p, hsa-miR-6822-5p, hsa-miR-4283, hsa-miR-5195-3p, hsa-miR-6529-5p, hsa-miR-106b-5p, hsa-miR-6506-3p, hsa-miR-6813-5p, hsa-miR-6730-5p, hsa-miR-6857-3p, hsa-miR-3663-5p, hsa-miR-610, hsa-miR-31-3p, hsa-miR-4656, hsa-miR-6815-5p, hsa-miR-12127, hsa-miR-3617-3p, hsa-miR-6754-3p, hsa-miR-551a, hsa-miR-4657, hsa-let-7f-1-3p, hsa-miR-6499-3p, hsa-miR-670-5p, hsa-miR-3659, hsa-miR-671-5p, hsa-miR-6090, hsa-miR-6716-5p, hsa-miR-3126-5p, hsa-miR-3918, hsa-miR-6728-5p, hsa-miR-1267, hsa-miR-338-5p, hsa-miR-10522-5p, hsa-miR-3605-5p, hsa-miR-4717-3p, hsa-miR-4646-5p, hsa-miR-328-5p, hsa-miR-4709-5p, hsa-miR-6786-3p, hsa-miR-181d-3p, hsa-miR-591, hsa-miR-5685, hsa-miR-4288, hsa-miR-4441, hsa-miR-23a-5p, hsa-miR-4251, hsa-miR-1298-3p, hsa-miR-6512-3p, hsa-miR-3064-5p, hsa-miR-4522, hsa-miR-3680-3p, hsa-miR-329-5p, hsa-miR-6513-5p, hsa-miR-3685, hsa-miR-6129, hsa-miR-125a-3p, hsa-miR-320d, hsa-miR-4726-5p, hsa-miR-563, hsa-miR-99b-3p, hsa-miR-7111-3p, hsa-miR-6816-5p, hsa-miR-3120-5p, hsa-miR-5699-5p, hsa-miR-1976, hsa-miR-7155-3p, hsa-miR-6850-5p, hsa-let-7b-3p, hsa-miR-34c-3p, hsa-miR-550b-2-5p, hsa-miR-6133, hsa-miR-4470, hsa-miR-1250-5p, hsa-miR-324-5p, hsa-miR-615-5p, hsa-miR-4756-3p, hsa-miR-765, hsa-miR-4437, hsa-miR-7159-5p, hsa-miR-140-3p, hsa-miR-8073, hsa-miR-8062, hsa-miR-6505-3p, hsa-miR-6891-5p, hsa-miR-6088, hsa-miR-941, hsa-miR-1912-3p, hsa-miR-4727-5p, hsa-miR-891a-5p, hsa-miR-5002-3p, hsa-miR-6754-5p, hsa-miR-6728-3p, hsa-miR-4269, hsa-miR-3926, hsa-miR-147a, hsa-miR-656-5p, hsa-miR-3192-5p, hsa-miR-8083, hsa-miR-6862-5p, hsa-miR-6800-5p, hsa-miR-103a-1-5p, hsa-miR-4690-5p, hsa-miR-320e, hsa-miR-3940-5p, hsa-miR-1233-5p, hsa-miR-4499, hsa-miR-6130, hsa-miR-4252, hsa-miR-6846-3p, hsa-miR-5091, hsa-miR-3196, hsa-miR-3646, hsa-miR-3944-3p, hsa-miR-8087, hsa-miR-134-5p, hsa-miR-7846-3p, hsa-miR-632, hsa-miR-12115, hsa-miR-4485-5p, hsa-miR-330-5p, hsa-miR-4697-3p, hsa-miR-877-3p, hsa-miR-6807-5p, hsa-miR-3621, hsa-miR-200b-3p, hsa-miR-23b-3p, hsa-miR-516b-3p, hsa-miR-516a-3p, hsa-miR-7856-5p, hsa-miR-1843, hsa-miR-7152-5p, hsa-miR-4534, hsa-miR-6864-5p, hsa-miR-487b-5p, hsa-miR-649, hsa-miR-1182, hsa-miR-6735-5p, hsa-miR-4698, hsa-miR-103a-3p, hsa-miR-4264, hsa-miR-7977, hsa-miR-2116-5p, hsa-miR-4259, hsa-miR-6514-3p, hsa-miR-7855-5p, hsa-miR-6504-3p, hsa-miR-6789-3p, hsa-miR-4689, hsa-miR-660-3p, hsa-miR-6881-5p, hsa-miR-634, hsa-miR-6804-5p, hsa-miR-6736-5p, hsa-miR-4317, hsa-miR-585-5p, hsa-miR-1307-3p, hsa-miR-4732-5p, hsa-miR-6722-5p, hsa-miR-4508, hsa-miR-514b-5p, hsa-miR-4311, hsa-miR-5581-5p, hsa-miR-6806-5p, hsa-miR-628-3p, hsa-miR-515-3p, hsa-miR-6738-3p, hsa-miR-1909-5p, hsa-miR-4786-5p, hsa-miR-147b-3p, hsa-miR-1203, hsa-miR-1301-3p, hsa-miR-342-5p, hsa-miR-519c-5p, hsa-miR-523-5p, hsa-miR-518e-5p, hsa-miR-522-5p, hsa-miR-519a-5p, hsa-miR-519b-5p, hsa-miR-6773-3p, hsa-miR-4253, hsa-miR-214-3p, hsa-miR-4455, hsa-miR-6851-3p, hsa-miR-4658, hsa-miR-4444, hsa-miR-130a-5p, hsa-miR-5681b, hsa-miR-6895-3p, hsa-miR-1910-3p, hsa-miR-4652-5p, hsa-miR-3149, hsa-miR-6840-5p, hsa-miR-3150a-3p, hsa-miR-219b-5p, hsa-miR-3198, hsa-miR-7109-3p, hsa-miR-489-5p, hsa-miR-34c-5p, hsa-miR-12128, hsa-miR-10395-5p, hsa-miR-6890-3p, hsa-miR-466, hsa-miR-6738-5p, hsa-miR-4326, hsa-miR-3125, hsa-miR-6774-5p, hsa-miR-1266-5p, hsa-miR-671-3p, hsa-miR-154-5p, hsa-miR-526a-3p, hsa-miR-4687-3p, hsa-miR-6739-3p, hsa-miR-642b-5p, hsa-miR-3136-5p, hsa-miR-146b-3p, hsa-miR-4795-3p, hsa-miR-7162-3p, hsa-miR-4788, hsa-miR-381-5p, hsa-miR-1227-3p, hsa-miR-6869-3p, hsa-miR-6501-3p, hsa-miR-4752, hsa-miR-7155-5p, hsa-miR-6068, hsa-miR-133b, hsa-miR-7976, hsa-miR-637, hsa-miR-4634, hsa-miR-378j, hsa-miR-6734-5p, hsa-miR-4296, hsa-miR-3147, hsa-miR-1184, hsa-miR-3925-3p, hsa-miR-151b, hsa-miR-3165, hsa-miR-4722-5p, hsa-let-7b-5p, hsa-miR-448, hsa-miR-762, hsa-miR-4780, hsa-miR-770-5p, hsa-miR-6787-3p, hsa-miR-3622a-3p, hsa-miR-6764-5p, hsa-miR-497-5p, hsa-miR-6857-5p, hsa-miR-454-5p, hsa-miR-10398-5p, hsa-miR-4510, hsa-miR-6875-3p, hsa-miR-6077, hsa-miR-6865-3p, hsa-miR-1275, hsa-miR-3935, hsa-miR-4443, hsa-miR-3059-5p, hsa-miR-4262, hsa-miR-187-3p, hsa-miR-342-3p, hsa-miR-449a, hsa-miR-580-3p, hsa-miR-378i, hsa-miR-375-3p, hsa-miR-6837-5p, hsa-miR-3655, hsa-miR-7112-5p, hsa-miR-6816-3p, hsa-miR-3194-5p, hsa-miR-4733-3p, hsa-miR-410-5p, hsa-miR-4476, hsa-miR-12136, hsa-miR-5195-5p, hsa-miR-505-5p, hsa-miR-4639-3p, hsa-miR-433-3p, hsa-miR-133a-3p, hsa-miR-4730, hsa-miR-6783-3p, hsa-miR-4726-3p, hsa-miR-604, hsa-miR-4537, hsa-miR-181a-2-3p, hsa-miR-3922-3p, hsa-miR-4479, hsa-miR-943, hsa-miR-6883-5p, hsa-miR-532-5p, hsa-miR-3650, hsa-miR-6876-3p, hsa-miR-6742-3p, hsa-miR-5000-3p, hsa-miR-192-5p, hsa-miR-7974, hsa-miR-6893-3p, hsa-miR-508-5p, hsa-miR-6509-5p, hsa-miR-203a-5p, hsa-miR-6769a-3p, hsa-miR-1228-5p, hsa-miR-6776-3p, hsa-miR-6072, hsa-miR-1269b, hsa-miR-6074, hsa-miR-6827-5p, hsa-miR-6882-5p, hsa-miR-6836-5p, hsa-miR-3189-3p, hsa-miR-216a-3p, hsa-miR-6736-3p, hsa-miR-574-5p, hsa-miR-4319, hsa-miR-4739, hsa-miR-1231, hsa-miR-186-3p, hsa-miR-449b-3p, hsa-miR-488-5p, hsa-miR-4436b-5p, hsa-miR-3144-5p, hsa-miR-572, hsa-miR-6513-3p, hsa-miR-1912-5p, hsa-miR-323b-3p, hsa-miR-873-3p, hsa-miR-365b-5p, hsa-miR-198, hsa-miR-2277-5p, hsa-miR-3714, hsa-miR-876-3p, hsa-miR-888-3p, hsa-miR-4723-5p, hsa-miR-4692, hsa-miR-4801, hsa-miR-4766-5p, hsa-miR-5588-5p, hsa-miR-217-3p, hsa-miR-6755-3p, hsa-miR-8485, hsa-miR-3925-5p, hsa-miR-4261, hsa-miR-6730-3p, hsa-miR-221-3p, hsa-miR-622, hsa-miR-518c-5p, hsa-miR-4713-3p, hsa-miR-6771-3p, hsa-miR-3130-5p, hsa-miR-615-3p, hsa-miR-542-5p, hsa-miR-4256, hsa-miR-3189-5p, hsa-miR-3202, hsa-miR-7158-3p, hsa-miR-6757-3p, hsa-miR-5003-3p, hsa-miR-6089, hsa-miR-616-5p, hsa-miR-449c-3p, hsa-miR-7975, hsa-miR-3160-3p, hsa-miR-4297, hsa-miR-6821-3p, hsa-miR-8069, hsa-miR-1271-5p, hsa-miR-3691-3p, hsa-miR-11400, hsa-miR-6504-5p, hsa-miR-4728-5p, hsa-miR-495-5p, hsa-miR-6867-5p, hsa-miR-6718-5p, hsa-miR-6742-5p, hsa-miR-29b-2-5p, hsa-miR-4701-5p, hsa-miR-196a-1-3p, hsa-let-7g-3p, hsa-miR-6744-5p, hsa-miR-4425, hsa-miR-3184-5p, hsa-miR-4724-5p, hsa-miR-30c-1-3p, hsa-miR-548ay-3p, hsa-miR-661, hsa-miR-6715b-5p, hsa-miR-4520-3p, hsa-miR-3178, hsa-miR-4647, hsa-miR-196b-3p, hsa-miR-6842-3p, hsa-miR-4677-5p, hsa-miR-378h, hsa-miR-3907, hsa-miR-6827-3p, hsa-miR-887-5p, hsa-miR-7158-5p, hsa-miR-4700-3p, hsa-miR-486-3p, hsa-miR-502-5p, hsa-miR-6808-3p, hsa-miR-550b-3p, hsa-miR-146a-5p, hsa-miR-6883-3p, hsa-miR-5010-3p, hsa-miR-6719-3p, hsa-miR-589-3p, hsa-miR-3928-5p, hsa-miR-6882-3p, hsa-miR-4714-5p, hsa-miR-889-5p, hsa-miR-4305, hsa-miR-5187-3p, hsa-miR-125b-5p, hsa-miR-302c-5p, hsa-miR-25-3p, hsa-miR-1323, hsa-miR-647, hsa-miR-4732-3p, hsa-miR-6843-3p, hsa-miR-6809-3p, hsa-miR-4307, hsa-miR-574-3p, hsa-miR-6856-3p, hsa-miR-4267, hsa-miR-1273c, hsa-miR-1269a, hsa-miR-412-3p, hsa-miR-7843-5p, hsa-miR-3126-3p, hsa-miR-494-5p, hsa-miR-1306-5p, hsa-miR-370-5p, hsa-miR-3929, hsa-miR-5694, hsa-miR-1276, hsa-miR-320b, hsa-miR-6744-3p, hsa-miR-4485-3p, hsa-miR-5002-5p, hsa-miR-378g, hsa-miR-6745, hsa-miR-4709-3p, hsa-miR-12126, hsa-miR-6780a-5p, hsa-miR-2355-5p, hsa-miR-6863, hsa-miR-5001-3p, hsa-miR-6873-5p, hsa-miR-942-3p, hsa-miR-2861, hsa-miR-223-3p, hsa-miR-30d-5p, hsa-miR-6778-3p, hsa-miR-4725-5p, hsa-miR-141-5p, hsa-miR-6761-3p, hsa-miR-15b-5p, hsa-miR-555, hsa-miR-10527-5p, hsa-miR-1322, hsa-miR-4675, hsa-miR-4446-3p, hsa-miR-29a-3p, hsa-miR-6757-5p, hsa-miR-6815-3p, hsa-miR-1297, hsa-miR-6804-3p, hsa-miR-6853-5p, hsa-miR-6716-3p, hsa-miR-2682-5p, hsa-miR-409-3p, hsa-miR-513b-5p, hsa-miR-6886-3p, hsa-miR-4445-3p, hsa-miR-138-1-3p, hsa-miR-3692-5p, hsa-miR-657, hsa-miR-6737-3p, hsa-miR-1291, hsa-miR-4318, hsa-miR-6747-3p, hsa-miR-744-3p, hsa-miR-8055, hsa-miR-4722-3p, hsa-miR-654-5p, hsa-miR-936, hsa-miR-4268, hsa-miR-6770-3p, hsa-miR-6760-5p, hsa-miR-1306-3p, hsa-miR-513a-5p, hsa-miR-4685-3p, hsa-miR-6868-3p, hsa-miR-668-3p, hsa-miR-4787-3p, hsa-miR-4793-3p, hsa-miR-4794, hsa-miR-6894-3p, hsa-miR-32-3p, hsa-miR-4773, and hsa-miR-3193. In the present disclosure, the extract of urine may be an extract of the urine of an urothelial carcinoma patient. Only one of these microRNAs can be used to predict whether a subject from which the urine is derived is an urothelial carcinoma patient, and 5 or more types, 10 or more types, 15 or more types, or 20 or more types of these microRNAs can be used in combination to predict whether a subject from which the urine is derived is an urothelial carcinoma patient. Among these microRNAs, a microRNA that may be highly expressed in an urothelial carcinoma patient can be preferably used for prediction.

The present disclosure provides an extract of urine containing at least one type, two types, three types, four types, five types, six types, seven types, eight types, nine types, or ten types of or all microRNAs selected from the group consisting of miR-15a-5p, miR-17-3p, miR-29a-3p, miR-30a-5p, miR-29b-3p, miR-103a-3p, miR-107, miR-196a-5p, miR-197-3p, miR-198, miR-208a-3p, miR-30d-5p, miR-10a-5p, miR-182-5p, miR-187-3p, miR-199b-5p, miR-214-3p, miR-223-3p, miR-124-3p, miR-125b-5p, miR-133a-3p, miR-140-5p, miR-141-3p, miR-134-5p, miR-149-5p, miR-154-3p, miR-184, miR-188-5p, miR-320a-3p, miR-106b-5p, miR-302a-3p, miR-299-3p, miR-296-5p, miR-130b-3p, miR-361-5p, miR-365a-3p, miR-365b-3p, miR-302c-5p, miR-370-3p, miR-373-5p, miR-373-3p, miR-375-3p, miR-378a-3p, miR-383-5p, miR-328-3p, miR-342-3p, miR-326, miR-133b, miR-325, miR-346, miR-448, miR-449a, miR-450a-5p, miR-200a-5p, miR-433-3p, miR-452-3p, miR-409-3p, miR-484, miR-485-3p, miR-486-5p, miR-491-5p, miR-202-3p, miR-492, miR-512-5p, miR-498-5p, miR-526b-5p, miR-518b, miR-518c-5p, miR-519d-3p, miR-516b-5p, miR-518a-3p, miR-519a-3p, miR-502-5p, miR-513a-5p, miR-532-5p, miR-299-5p, miR-539-5p, miR-552-3p, miR-554, miR-555, miR-557, miR-563, miR-564, miR-571, miR-574-3p, miR-575, miR-579-3p, miR-583, miR-585-3p, miR-595, miR-608, miR-610, miR-612, miR-615-3p, miR-616-5p, miR-617, miR-624-5p, miR-628-3p, miR-632, miR-634, miR-637, miR-638, miR-639, miR-642a-5p, miR-649, miR-663a, miR-449b-5p, miR-657, miR-658, miR-659-3p, miR-542-5p, miR-671-5p, miR-668-3p, miR-767-3p, miR-769-5p, miR-766-3p, miR-765, miR-675-5p, let-7b-3p, let-7f-1-3p, miR-92a-2-5p, miR-29b-2-5p, miR-139-3p, miR-181c-3p, miR-183-3p, miR-187-5p, miR-124-5p, miR-125b-1-3p, miR-130a-5p, miR-135a-3p, miR-140-3p, miR-125a-3p, miR-127-5p, miR-149-3p, miR-150-3p, miR-185-3p, miR-186-3p, miR-193a-5p, miR-194-3p, miR-30c-1-3p, miR-99b-3p, miR-296-3p, miR-361-3p, miR-371a-5p, miR-342-5p, miR-151a-5p, miR-423-5p, miR-424-3p, miR-18b-3p, miR-20b-3p, miR-431-3p, miR-483-5p, miR-486-3p, miR-490-5p, miR-193b-5p, miR-505-5p, miR-532-3p, miR-92b-5p, miR-551b-5p, miR-574-5p, miR-550a-5p, miR-615-5p, miR-616-3p, miR-624-3p, miR-625-3p, miR-629-5p, miR-298, miR-874-3p, miR-891b, miR-876-3p, miR-744-5p, miR-885-5p, miR-885-3p, miR-877-5p, miR-877-3p, miR-887-3p, miR-665, miR-760, miR-920, miR-933, miR-936, miR-939-5p, miR-940, miR-1224-5p, miR-1224-3p, miR-1225-5p, miR-1225-3p, miR-1227-3p, miR-1228-5p, miR-1228-3p, miR-1229-3p, miR-1231, miR-1233-3p, miR-1234-3p, miR-1236-3p, miR-1237-3p, miR-1238-3p, miR-320b, miR-320c, miR-1323, miR-1301-3p, miR-1298-5p, miR-1180-3p, miR-1181, miR-1182, miR-1184, miR-1202, miR-1203, miR-1207-5p, miR-1207-3p, miR-1208, miR-1289, miR-1290, miR-1293, miR-1294, miR-1303, miR-1304-5p, miR-1246, miR-1249-3p, miR-1260a, miR-1263, miR-1266-5p, miR-1267, miR-1268a, miR-1270, miR-1275, miR-1281, miR-1292-5p, miR-1255b-5p, miR-1307-3p, miR-320d, miR-1825, miR-675-3p, miR-1469, miR-1470, miR-1471, miR-1539, miR-1908-5p, miR-1905-5p, miR-1909-3p, miR-1910-5p, miR-1912-3p, miR-1913, miR-1914-3p, miR-1915-3p, miR-365a-5p, miR-449b-3p, miR-1976, miR-2110, let-7a-2-3p, miR-151b, miR-762, miR-670-5p, miR-764, miR-2116-3p, miR-548q, miR-2276-3p, miR-2278, miR-711, miR-718, miR-2681-3p, miR-449c-3p, miR-2861, miR-3116, miR-3120-3p, miR-3122, miR-3126-5p, miR-3130-5p, miR-3131, miR-3132, miR-3133, miR-466, miR-3136-5p, miR-3137, miR-3138, miR-3141, miR-3144-5p, miR-1273c, miR-3147, miR-3148, miR-3150a-3p, miR-3151-5p, miR-3153, miR-3154, miR-3156-5p, miR-3157-5p, miR-3160-3p, miR-3161, miR-3162-5p, miR-3163, miR-3165, miR-1260b, miR-3173-3p, miR-1193, miR-3175, miR-3176, miR-3177-3p, miR-3178, miR-3179, miR-3180-5p, miR-3180-3p, miR-3184-5p, miR-3185, miR-3186-3p, miR-3188, miR-3189-3p, miR-3191-3p, miR-3194-5p, miR-3195, miR-3196, miR-3197, miR-3198, miR-514b-5p, miR-3202, miR-4297, miR-4293, miR-4294, miR-4299, miR-4298, miR-4305, miR-4307, miR-4312, miR-4313, miR-4316, miR-4314, miR-4320, miR-4322, miR-4321, miR-4323, miR-4256, miR-4257, miR-4258, miR-4259, miR-4260, miR-4253, miR-4251, miR-4326, miR-4327, miR-2355-5p, miR-4268, miR-4269, miR-4264, miR-4271, miR-4276, miR-4274, miR-4281, miR-4279, miR-4278, miR-4280, miR-4285, miR-4284, miR-4286, miR-4287, miR-4292, miR-4289, miR-4290, miR-4329, miR-500b-5p, miR-3200-5p, miR-3605-3p, miR-3609, miR-3610, miR-3612, miR-3614-5p, miR-3614-3p, miR-3616-3p, miR-3620-3p, miR-3621, miR-3622a-5p, miR-3622a-3p, miR-3622b-5p, miR-3646, miR-3648, miR-3649, miR-3652, miR-3660, miR-3663-5p, miR-3663-3p, miR-3665, miR-3667-3p, miR-3675-3p, miR-3679-5p, miR-3679-3p, miR-3689a-5p, miR-3689b-5p, miR-3689e, miR-3689a-3p, miR-3714, miR-3180, miR-3907, miR-3908, miR-3911, miR-3917, miR-3919, miR-3150b-3p, miR-3925-5p, miR-3926, miR-3928-3p, miR-3935, miR-3937, miR-3943, miR-3944-3p, miR-3945, miR-642b-3p, miR-550b-3p, miR-1268b, miR-548ab, miR-4421, miR-4428, miR-4429, miR-4430, miR-4433a-3p, miR-4436a, miR-4439, miR-4440, miR-4441, miR-4443, miR-4444, miR-4446-3p, miR-4447, miR-4448, miR-4449, miR-4455, miR-4456, miR-4458, miR-4460, miR-378h, miR-3135b, miR-4462, miR-4463, miR-4466, miR-4467, miR-4468, miR-4470, miR-4472, miR-4475, miR-4476, miR-4478, miR-3689d, miR-4481, miR-4483, miR-4484, miR-4485-3p, miR-4486, miR-4487, miR-4488, miR-4489, miR-4492, miR-4496, miR-4497, miR-4498, miR-4499, miR-4505, miR-4506, miR-2392, miR-4507, miR-4508, miR-4510, miR-4511, miR-4513, miR-4516, miR-4518, miR-4520-3p, miR-4521, miR-1269b, miR-4522, miR-4523, miR-4524a-3p, miR-4525, miR-4526, miR-4530, miR-4533, miR-4534, miR-4535, miR-1587, miR-4538, miR-4539, miR-3127-3p, miR-3156-3p, miR-3158-5p, miR-3162-3p, miR-3173-5p, miR-3187-5p, miR-3189-5p, miR-3619-3p, miR-3150b-5p, miR-3925-3p, miR-3940-5p, miR-3972, miR-3978, miR-4634, miR-4638-5p, miR-4639-3p, miR-4640-5p, miR-4640-3p, miR-4642, miR-4645-5p, miR-4646-5p, mi R-4646-3p, miR-4648, miR-4649-5p, miR-4649-3p, miR-4650-3p, miR-4651, miR-4653-3p, miR-4654, miR-4655-5p, miR-4656, miR-4661-5p, miR-4659b-3p, miR-4664-5p, miR-4664-3p, miR-4665-5p, miR-4665-3p, miR-4667-5p, miR-4667-3p, miR-4669, miR-4672, miR-4673, miR-4674, miR-4675, miR-4676-5p, miR-4677-5p, miR-4682, miR-4685-5p, miR-4685-3p, miR-4687-5p, miR-4687-3p, miR-1343-3p, miR-4688, miR-4689, miR-4690-5p, miR-4695-5p, miR-4695-3p, miR-4697-5p, miR-4697-3p, miR-4698, miR-4699-3p, miR-4700-5p, miR-4700-3p, miR-4701-5p, miR-4701-3p, miR-4704-5p, miR-4706, miR-4707-5p, miR-4707-3p, miR-4708-3p, miR-4709-3p, miR-4710, miR-4713-5p, miR-4714-5p, miR-4715-5p, miR-4716-5p, miR-4716-3p, miR-4717-3p, miR-4719, miR-4720-5p, miR-4721, miR-4722-5p, miR-4722-3p, miR-4723-5p, miR-4723-3p, miR-4724-5p, miR-4725-5p, miR-4725-3p, miR-4726-5p, miR-4728-5p, miR-4728-3p, miR-4730, miR-4731-5p, miR-4731-3p, miR-4732-5p, miR-4732-3p, miR-4734, miR-4736, miR-3064-5p, miR-3064-3p, miR-4739, miR-4741, miR-4743-5p, miR-4745-5p, miR-4746-3p, miR-4747-5p, miR-4748, miR-4749-5p, miR-4749-3p, miR-4750-5p, miR-4751, miR-4752, miR-4753-5p, miR-371b-5p, miR-371b-3p, miR-4754, miR-4755-3p, miR-499b-3p, miR-4756-5p, miR-4758-5p, miR-4758-3p, miR-4759, miR-4763-5p, miR-4763-3p, miR-4764-5p, miR-4766-5p, miR-4769-5p, miR-4769-3p, miR-4770, miR-4776-5p, miR-4778-5p, miR-4779, miR-4780, miR-4436b-5p, miR-4781-5p, miR-4783-3p, miR-4784, miR-2467-5p, miR-2467-3p, miR-4787-5p, miR-4787-3p, miR-4788, miR-4793-3p, miR-4795-3p, miR-4799-5p, miR-4800-5p, miR-4804-3p, miR-642a-3p, miR-4433a-5p, miR-4482-3p, miR-4999-5p, miR-5000-3p, miR-5001-5p, miR-5001-3p, miR-5002-3p, miR-5003-3p, miR-5006-5p, miR-5007-3p, miR-5009-3p, miR-5010-5p, miR-5010-3p, miR-5088-5p, miR-5089-5p, miR-5090, miR-5188, miR-5189-5p, miR-5193, miR-5195-5p, miR-5195-3p, miR-5196-5p, miR-5196-3p, miR-4524b-5p, miR-5100, miR-5572, miR-664b-5p, miR-664b-3p, miR-5581-3p, miR-5584-5p, miR-5586-3p, miR-548au-3p, miR-5588-5p, miR-5589-5p, miR-5690, miR-5698, miR-5699-3p, miR-5703, miR-5705, miR-5707, miR-5708, miR-197-5p, miR-181b-3p, miR-204-3p, miR-211-3p, miR-345-3p, miR-652-5p, miR-1185-2-3p, miR-873-3p, miR-1304-3p, miR-1247-3p, miR-1306-5p, miR-3184-3p, miR-3191-5p, miR-642b-5p, miR-365b-5p, miR-1185-1-3p, miR-3190-3p, miR-98-3p, miR-381-5p, miR-503-3p, miR-937-5p, miR-939-3p, miR-1227-5p, miR-1229-5p, miR-1233-5p, miR-1236-5p, miR-1237-5p, miR-1238-5p, miR-1292-3p, miR-3617-3p, miR-3620-5p, miR-4632-5p, miR-4743-3p, miR-4750-3p, miR-5739, miR-5787, miR-6069, miR-6071, miR-6072, miR-6075, miR-6076, miR-6077, miR-6082, miR-6085, miR-6086, miR-6088, miR-6089, miR-6090, miR-6124, miR-6125, miR-6126, miR-6127, miR-378j, miR-6131, miR-6132, miR-6133, miR-6165, miR-548ay-3p, miR-6501-3p, miR-6503-5p, miR-6506-5p, miR-6506-3p, miR-6508-5p, miR-6509-3p, miR-6510-5p, miR-6511a-5p, miR-6511a-3p, miR-6512-3p, miR-6513-5p, miR-6514-3p, miR-6515-5p, miR-6515-3p, miR-6716-5p, miR-6716-3p, miR-6717-5p, miR-6511b-5p, miR-6511b-3p, miR-6718-5p, miR-6719-3p, miR-6721-5p, miR-6722-5p, miR-6722-3p, miR-6724-5p, miR-208a-5p, miR-210-5p, miR-128-1-5p, miR-328-5p, miR-329-5p, miR-410-5p, miR-181d-3p, miR-504-3p, miR-487b-5p, miR-585-5p, miR-598-5p, miR-605-3p, miR-619-5p, miR-655-5p, miR-1296-3p, miR-874-5p, miR-887-5p, miR-1250-3p, miR-1266-3p, miR-3151-3p, miR-3192-3p, miR-1343-5p, miR-5088-3p, miR-5189-3p, miR-5699-5p, miR-6726-5p, miR-6726-3p, miR-6727-5p, miR-6727-3p, miR-6728-5p, miR-6728-3p, miR-6729-5p, miR-6729-3p, miR-6730-5p, miR-6730-3p, miR-6731-5p, miR-6731-3p, miR-6732-5p, miR-6732-3p, miR-6734-5p, miR-6735-5p, miR-6735-3p, miR-6736-5p, miR-6736-3p, miR-6737-5p, miR-6737-3p, miR-6738-5p, miR-6738-3p, miR-6740-5p, miR-6740-3p, miR-6741-5p, miR-6741-3p, miR-6742-5p, miR-6742-3p, miR-6743-5p, miR-6743-3p, miR-6744-5p, miR-6744-3p, miR-6745, miR-6746-5p, miR-6747-5p, miR-6747-3p, miR-6748-5p, miR-6749-5p, miR-6749-3p, miR-6750-3p, miR-6751-5p, miR-6751-3p, miR-6752-5p, miR-6752-3p, miR-6753-5p, miR-6753-3p, miR-6754-5p, miR-6754-3p, miR-6755-5p, miR-6756-5p, miR-6756-3p, miR-6757-5p, miR-6757-3p, miR-6758-5p, miR-6759-5p, miR-6759-3p, miR-6760-5p, miR-6760-3p, miR-6761-3p, miR-6762-5p, miR-6762-3p, miR-6763-5p, miR-6763-3p, miR-6765-5p, miR-6766-5p, miR-6766-3p, miR-6767-5p, miR-6768-5p, miR-6769a-5p, miR-6769a-3p, miR-6770-5p, miR-6771-5p, miR-6771-3p, miR-6772-5p, miR-6772-3p, miR-6773-3p, miR-6774-5p, miR-6775-5p, miR-6775-3p, miR-6776-5p, miR-6776-3p, miR-6777-5p, miR-6777-3p, miR-6778-5p, miR-6778-3p, miR-6779-5p, miR-6779-3p, miR-6780a-5p, miR-6780a-3p, miR-6781-5p, miR-6782-5p, miR-6782-3p, miR-6783-5p, miR-6783-3p, miR-6784-5p, miR-6784-3p, miR-6785-3p, miR-6786-5p, miR-6786-3p, miR-6787-5p, miR-6787-3p, miR-6788-5p, miR-6788-3p, miR-6789-5p, miR-6789-3p, miR-6790-5p, miR-6790-3p, miR-6791-5p, miR-6792-5p, miR-6792-3p, miR-6793-5p, miR-6793-3p, miR-6794-5p, miR-6794-3p, miR-6795-5p, miR-6795-3p, miR-6796-5p, miR-6796-3p, miR-6797-5p, miR-6797-3p, miR-6798-5p, miR-6798-3p, miR-6799-5p, miR-6799-3p, miR-6800-5p, miR-6800-3p, miR-6801-3p, miR-6802-5p, miR-6802-3p, miR-6803-5p, miR-6803-3p, miR-6804-3p, miR-6805-5p, miR-6805-3p, miR-6806-5p, miR-6807-5p, miR-6808-5p, miR-6808-3p, miR-6809-5p, miR-6809-3p, miR-6810-5p, miR-6810-3p, miR-6812-5p, miR-6812-3p, miR-6813-5p, miR-6813-3p, miR-6815-5p, miR-6815-3p, miR-6816-5p, miR-6816-3p, miR-6818-5p, miR-6819-5p, miR-6819-3p, miR-6820-5p, miR-6820-3p, miR-6821-5p, miR-6822-5p, miR-6822-3p, miR-6823-5p, miR-6823-3p, miR-6824-5p, miR-6824-3p, miR-6825-5p, miR-6825-3p, miR-6826-3p, miR-6827-5p, miR-6827-3p, miR-6828-5p, miR-6829-5p, miR-6829-3p, miR-6830-5p, miR-6830-3p, miR-6831-5p, miR-6831-3p, miR-6832-5p, miR-6832-3p, miR-6833-5p, miR-6833-3p, miR-6834-5p, miR-6834-3p, miR-6780b-5p, miR-6780b-3p, miR-6836-3p, miR-6837-5p, miR-6838-5p, miR-6839-5p, miR-6840-5p, miR-6840-3p, miR-6841-3p, miR-6842-5p, miR-6845-5p, miR-6845-3p, miR-6846-5p, miR-6846-3p, miR-6847-3p, miR-6848-5p, miR-6848-3p, miR-6849-5p, miR-6849-3p, miR-6850-5p, miR-6851-5p, miR-6851-3p, miR-6852-3p, miR-6855-5p, miR-6855-3p, miR-6856-5p, miR-6857-5p, miR-6857-3p, miR-6858-5p, miR-6858-3p, miR-6859-5p, miR-6859-3p, miR-6769b-5p, miR-6769b-3p, miR-6860, miR-6861-5p, miR-6861-3p, miR-6862-5p, miR-6862-3p, miR-6864-5p, miR-6865-5p, miR-6865-3p, miR-6867-5p, miR-6867-3p, miR-6869-5p, miR-6870-5p, miR-6870-3p, miR-6871-5p, miR-6872-3p, miR-6873-5p, miR-6874-5p, miR-6875-5p, miR-6875-3p, miR-6876-5p, miR-6876-3p, miR-6877-5p, miR-6877-3p, miR-6878-3p, miR-6879-5p, miR-6879-3p, miR-6880-5p, miR-6880-3p, miR-6881-5p, miR-6882-5p, miR-6882-3p, miR-6883-5p, miR-6883-3p, miR-6884-3p, miR-6885-3p, miR-6886-5p, miR-6886-3p, miR-6887-5p, miR-6887-3p, miR-6889-5p, miR-6889-3p, miR-6890-5p, miR-6890-3p, miR-6891-5p, miR-6891-3p, miR-6892-5p, miR-6892-3p, miR-6893-5p, miR-6893-3p, miR-6894-5p, miR-6894-3p, miR-7106-5p, miR-7106-3p, miR-7107-5p, miR-7108-5p, miR-7108-3p, miR-7109-5p, miR-7109-3p, miR-7110-5p, miR-7110-3p, miR-7111-Sp, miR-7111-3p, miR-7112-5p, miR-7113-5p, miR-7113-3p, miR-7114-5p, miR-7114-3p, miR-7150, miR-7151-3p, miR-7152-5p, miR-7152-3p, miR-7155-5p, miR-7158-3p, miR-7159-5p, miR-7160-5p, miR-7162-5p, miR-7515, miR-7702, miR-7704, miR-7843-5p, miR-4433b-5p, miR-4433b-3p, miR-1273h-5p, miR-6516-5p, miR-7845-5p, miR-7846-3p, miR-7847-3p, miR-7851-3p, miR-7855-5p, miR-7856-5p, miR-8052, miR-8054, miR-8059, miR-8060, miR-8063, miR-8064, miR-8069, miR-8071, miR-8072, miR-8073, miR-8074, miR-8075, miR-8077, miR-8080, miR-8085, miR-8087, miR-8089, miR-7977, miR-7978, miR-203a-5p, miR-1249-5p, miR-4485-5p, miR-8485, miR-196a-1-3p, miR-217-3p, miR-498-3p, miR-526a-3p, miR-1912-5p, miR-9718, miR-9898, miR-9985, miR-1843, miR-10226, miR-10392-5p, miR-10392-3p, miR-10394-3p, miR-10395-3p, miR-10396a-5p, miR-10396a-3p, miR-10398-5p, miR-10398-3p, miR-10399-3p, miR-10400-3p, miR-10401-5p, miR-10401-3p, miR-10396b-5p, miR-10522-5p, miR-10526-3p, miR-10527-5p, miR-11181-5p, miR-11181-3p, miR-11399, miR-11400, miR-3059-5p, miR-3085-5p, miR-3085-3p, miR-6529-5p, miR-12114, miR-12115, miR-12116, miR-12118, miR-12119, miR-12120, miR-12121, miR-12124, miR-12126, miR-12131, and miR-12136. In the present disclosure, the extract of urine may be an extract of the urine of a stage-I or -II urothelial carcinoma patient. Only one of these microRNAs can be used to predict whether a subject from which the urine is derived is an urothelial carcinoma patient (particularly stage-I or -II), and 5 or more types, 10 or more types, 15 or more types, or 20 or more types of these microRNAs can be used in combination to predict whether a subject from which the urine is derived is an urothelial carcinoma patient (particularly stage-I or -II). Among these microRNAs, a microRNA that may be highly expressed in an urothelial carcinoma patient (particularly stage-I or -II) can be preferably used for prediction.

The present disclosure provides an extract of urine containing at least one type, two types, three types, four types, five types, six types, seven types, eight types, nine types, or ten types of or all microRNAs selected from the group consisting of hsa-let-7a-5p, hsa-miR-15a-5p, hsa-miR-17-3p, hsa-miR-29a-3p, hsa-miR-30a-5p, hsa-miR-103a-3p, hsa-miR-196a-5p, hsa-miR-198, hsa-miR-10a-5p, hsa-miR-182-5p, hsa-miR-214-3p, hsa-miR-124-3p, hsa-miR-125b-5p, hsa-miR-140-5p, hsa-miR-141-3p, hsa-miR-134-5p, hsa-miR-206, hsa-miR-106b-5p, hsa-miR-302a-3p, hsa-miR-299-3p, hsa-miR-296-5p, hsa-miR-130b-3p, hsa-miR-365a-3p, hsa-miR-365b-3p, hsa-miR-302c-5p, hsa-miR-370-3p, hsa-miR-373-5p, hsa-miR-373-3p, hsa-miR-378a-3p, hsa-miR-380-3p, hsa-miR-383-5p, hsa-miR-328-3p, hsa-miR-342-3p, hsa-miR-133b, hsa-miR-325, hsa-miR-448, hsa-miR-429, hsa-miR-449a, hsa-miR-200a-5p, hsa-miR-452-3p, hsa-miR-484, hsa-miR-486-5p, hsa-miR-491-5p, hsa-miR-202-3p, hsa-miR-492, hsa-miR-512-5p, hsa-miR-498-5p, hsa-miR-515-3p, hsa-miR-518b, hsa-miR-518c-3p, hsa-miR-524-3p, hsa-miR-516b-5p, hsa-miR-518a-3p, hsa-miR-519a-3p, hsa-miR-499a-5p, hsa-miR-502-5p, hsa-miR-503-5p, hsa-miR-513a-5p, hsa-miR-532-5p, hsa-miR-554, hsa-miR-555, hsa-miR-556-5p, hsa-miR-557, hsa-miR-563, hsa-miR-564, hsa-miR-571, hsa-miR-574-3p, hsa-miR-575, hsa-miR-583, hsa-miR-585-3p, hsa-miR-595, hsa-miR-608, hsa-miR-610, hsa-miR-612, hsa-miR-615-3p, hsa-miR-617, hsa-miR-618, hsa-miR-628-3p, hsa-miR-632, hsa-miR-634, hsa-miR-637, hsa-miR-638, hsa-miR-639, hsa-miR-641, hsa-miR-649, hsa-miR-652-3p, hsa-miR-663a, hsa-miR-449b-5p, hsa-miR-654-5p, hsa-miR-657, hsa-miR-658, hsa-miR-542-5p, hsa-miR-671-5p, hsa-miR-668-3p, hsa-miR-769-5p, hsa-miR-766-3p, hsa-miR-765, hsa-miR-675-5p, hsa-miR-297, hsa-miR-22-5p, hsa-miR-92a-2-5p, hsa-miR-30c-2-3p, hsa-miR-139-3p, hsa-miR-181c-3p, hsa-miR-183-3p, hsa-miR-187-5p, hsa-miR-124-5p, hsa-miR-125b-1-3p, hsa-miR-132-5p, hsa-miR-135a-3p, hsa-miR-140-3p, hsa-miR-125a-3p, hsa-miR-149-3p, hsa-miR-150-3p, hsa-miR-185-3p, hsa-miR-186-3p, hsa-miR-193a-5p, hsa-miR-194-3p, hsa-miR-30c-1-3p, hsa-miR-296-3p, hsa-miR-361-3p, hsa-miR-371a-5p, hsa-miR-342-5p, hsa-miR-151a-5p, hsa-miR-423-5p, hsa-miR-424-3p, hsa-miR-18b-3p, hsa-miR-20b-3p, hsa-miR-483-5p, hsa-miR-486-3p, hsa-miR-490-5p, hsa-miR-193b-5p, hsa-miR-501-3p, hsa-miR-505-5p, hsa-miR-92b-5p, hsa-miR-551b-5p, hsa-miR-574-5p, hsa-miR-550a-5p, hsa-miR-615-5p, hsa-miR-616-3p, hsa-miR-624-3p, hsa-miR-625-3p, hsa-miR-629-5p, hsa-miR-298, hsa-miR-874-3p, hsa-miR-891b, hsa-miR-892b, hsa-miR-876-3p, hsa-miR-744-5p, hsa-miR-885-3p, hsa-miR-877-5p, hsa-miR-877-3p, hsa-miR-887-3p, hsa-miR-665, hsa-miR-760, hsa-miR-920, hsa-miR-509-3-5p, hsa-miR-936, hsa-miR-939-5p, hsa-miR-940, hsa-miR-1224-5p, hsa-miR-1224-3p, hsa-miR-1225-5p, hsa-miR-1225-3p, hsa-miR-1228-5p, hsa-miR-1228-3p, hsa-miR-1231, hsa-miR-1234-3p, hsa-miR-1236-3p, hsa-miR-1237-3p, hsa-miR-1238-3p, hsa-miR-320c, hsa-miR-1323, hsa-miR-1301-3p, hsa-miR-1180-3p, hsa-miR-1182, hsa-miR-1184, hsa-miR-1202, hsa-miR-1203, hsa-miR-1207-5p, hsa-miR-1289, hsa-miR-1290, hsa-miR-1293, hsa-miR-1294, hsa-miR-1303, hsa-miR-1304-5p, hsa-miR-1246, hsa-miR-1249-3p, hsa-miR-1266-5p, hsa-miR-1267, hsa-miR-1268a, hsa-miR-1270, hsa-miR-1281, hsa-miR-1292-5p, hsa-miR-1255b-5p, hsa-miR-1307-3p, hsa-miR-320d, hsa-miR-1469, hsa-miR-1470, hsa-miR-1471, hsa-miR-1908-5p, hsa-miR-1909-5p, hsa-miR-1909-3p, hsa-miR-1912-3p, hsa-miR-1913, hsa-miR-1914-3p, hsa-miR-1915-3p, hsa-miR-365a-5p, hsa-miR-449b-3p, hsa-miR-1976, hsa-miR-2110, hsa-miR-151b, hsa-miR-762, hsa-miR-670-5p, hsa-miR-2114-3p, hsa-miR-2116-3p, hsa-miR-548q, hsa-miR-2276-3p, hsa-miR-711, hsa-miR-718, hsa-miR-2681-3p, hsa-miR-3120-3p, hsa-miR-3122, hsa-miR-3126-5p, hsa-miR-3130-5p, hsa-miR-3131, hsa-miR-3132, hsa-miR-3133, hsa-miR-3134, hsa-miR-466, hsa-miR-3136-5p, hsa-miR-3137, hsa-miR-3138, hsa-miR-3141, hsa-miR-1273c, hsa-miR-3147, hsa-miR-3150a-3p, hsa-miR-3151-5p, hsa-miR-3153, hsa-miR-3154, hsa-miR-3156-5p, hsa-miR-3158-3p, hsa-miR-3161, hsa-miR-3162-5p, hsa-miR-3163, hsa-miR-3165, hsa-miR-3168, hsa-miR-3173-3p, hsa-miR-1193, hsa-miR-3175, hsa-miR-3176, hsa-miR-3177-3p, hsa-miR-3178, hsa-miR-3179, hsa-miR-3180-5p, hsa-miR-3180-3p, hsa-miR-3184-5p, hsa-miR-3185, hsa-miR-3186-3p, hsa-miR-3187-3p, hsa-miR-3189-3p, hsa-miR-3191-3p, hsa-miR-3195, hsa-miR-3196, hsa-miR-3197, hsa-miR-3198, hsa-miR-514b-5p, hsa-miR-3202, hsa-miR-4297, hsa-miR-4294, hsa-miR-4299, hsa-miR-4298, hsa-miR-4305, hsa-miR-4307, hsa-miR-4313, hsa-miR-4316, hsa-miR-4314, hsa-miR-4322, hsa-miR-4321, hsa-miR-4323, hsa-miR-4257, hsa-miR-4258, hsa-miR-4259, hsa-miR-4260, hsa-miR-4253, hsa-miR-4251, hsa-miR-4326, hsa-miR-4327, hsa-miR-4268, hsa-miR-4269, hsa-miR-4264, hsa-miR-4271, hsa-miR-4276, hsa-miR-4274, hsa-miR-4280, hsa-miR-4284, hsa-miR-4286, hsa-miR-500b-5p, hsa-miR-3200-5p, hsa-miR-3609, hsa-miR-3610, hsa-miR-3614-5p, hsa-miR-3616-3p, hsa-miR-3617-5p, hsa-miR-3621, hsa-miR-3622a-5p, hsa-miR-3622a-3p, hsa-miR-3622b-5p, hsa-miR-3646, hsa-miR-3648, hsa-miR-3649, hsa-miR-3652, hsa-miR-3660, hsa-miR-3663-5p, hsa-miR-3665, hsa-miR-3667-3p, hsa-miR-3675-3p, hsa-miR-3679-5p, hsa-miR-3679-3p, hsa-miR-3681-5p, hsa-miR-3689a-3p, hsa-miR-3714, hsa-miR-3180, hsa-miR-3907, hsa-miR-3908, hsa-miR-3911, hsa-miR-3915, hsa-miR-3917, hsa-miR-3919, hsa-miR-3150b-3p, hsa-miR-3925-5p, hsa-miR-3926, hsa-miR-3928-3p, hsa-miR-3935, hsa-miR-3937, hsa-miR-3943, hsa-miR-3944-3p, hsa-miR-3945, hsa-miR-642b-3p, hsa-miR-1268b, hsa-miR-548ab, hsa-miR-4422, hsa-miR-4426, hsa-miR-4428, hsa-miR-4429, hsa-miR-4430, hsa-miR-4433a-3p, hsa-miR-4436a, hsa-miR-4439, hsa-miR-4440, hsa-miR-4441, hsa-miR-4443, hsa-miR-4444, hsa-miR-4446-3p, hsa-miR-4447, hsa-miR-4448, hsa-miR-4449, hsa-miR-4455, hsa-miR-4456, hsa-miR-4458, hsa-miR-4460, hsa-miR-378h, hsa-miR-3135b, hsa-miR-4462, hsa-miR-4463, hsa-miR-4466, hsa-miR-4467, hsa-miR-4468, hsa-miR-4470, hsa-miR-4472, hsa-miR-4475, hsa-miR-4476, hsa-miR-4478, hsa-miR-3689d, hsa-miR-3155b, hsa-miR-4481, hsa-miR-4483, hsa-miR-4484, hsa-miR-4485-3p, hsa-miR-4486, hsa-miR-4487, hsa-miR-4488, hsa-miR-4489, hsa-miR-4492, hsa-miR-4496, hsa-miR-4497, hsa-miR-4498, hsa-miR-4499, hsa-miR-4505, hsa-miR-4506, hsa-miR-2392, hsa-miR-4507, hsa-miR-4508, hsa-miR-4510, hsa-miR-4511, hsa-miR-4513, hsa-miR-4516, hsa-miR-4519, hsa-miR-4520-3p, hsa-miR-4521, hsa-miR-4522, hsa-miR-4525, hsa-miR-4526, hsa-miR-4530, hsa-miR-4533, hsa-miR-4534, hsa-miR-4535, hsa-miR-1587, hsa-miR-4538, hsa-miR-4539, hsa-miR-3127-3p, hsa-miR-3158-5p, hsa-miR-3162-3p, hsa-miR-3173-5p, hsa-miR-3187-5p, hsa-miR-3619-3p, hsa-miR-3150b-5p, hsa-miR-3940-5p, hsa-miR-3978, hsa-miR-4634, hsa-miR-4638-5p, hsa-miR-4639-5p, hsa-miR-4639-3p, hsa-miR-4640-5p, hsa-miR-4640-3p, hsa-miR-4646-5p, hsa-miR-4648, hsa-miR-4649-3p, hsa-miR-4650-3p, hsa-miR-4651, hsa-miR-4653-3p, hsa-miR-4654, hsa-miR-4655-5p, hsa-miR-4656, hsa-miR-4664-5p, hsa-miR-4664-3p, hsa-miR-4665-5p, hsa-miR-4665-3p, hsa-miR-4667-5p, hsa-miR-4667-3p, hsa-miR-4669, hsa-miR-4670-3p, hsa-miR-4672, hsa-miR-4673, hsa-miR-4674, hsa-miR-4676-5p, hsa-miR-4677-5p, hsa-miR-4682, hsa-miR-4685-5p, hsa-miR-4685-3p, hsa-miR-4687-5p, hsa-miR-4687-3p, hsa-miR-1343-3p, hsa-miR-4688, hsa-miR-4689, hsa-miR-4690-5p, hsa-miR-4691-5p, hsa-miR-4695-5p, hsa-miR-4697-5p, hsa-miR-4697-3p, hsa-miR-4698, hsa-miR-4699-3p, hsa-miR-4700-5p, hsa-miR-4700-3p, hsa-miR-4701-5p, hsa-miR-4701-3p, hsa-miR-4706, hsa-miR-4707-5p, hsa-miR-4707-3p, hsa-miR-4709-3p, hsa-miR-4710, hsa-miR-4714-5p, hsa-miR-4716-5p, hsa-miR-4716-3p, hsa-miR-4717-3p, hsa-miR-4719, hsa-miR-4720-5p, hsa-miR-4721, hsa-miR-4722-5p, hsa-miR-4722-3p, hsa-miR-4723-5p, hsa-miR-4723-3p, hsa-miR-4724-5p, hsa-miR-4725-5p, hsa-miR-4725-3p, hsa-miR-4726-5p, hsa-miR-4728-5p, hsa-miR-4728-3p, hsa-miR-4730, hsa-miR-4731-5p, hsa-miR-4731-3p, hsa-miR-4732-5p, hsa-miR-4732-3p, hsa-miR-4734, hsa-miR-4736, hsa-miR-3064-5p, hsa-miR-4738-3p, hsa-miR-4739, hsa-miR-4741, hsa-miR-4742-3p, hsa-miR-4743-5p, hsa-miR-4745-5p, hsa-miR-4746-3p, hsa-miR-4747-5p, hsa-miR-4748, hsa-miR-4749-5p, hsa-miR-4749-3p, hsa-miR-4750-5p, hsa-miR-4751, hsa-miR-4752, hsa-miR-4753-5p, hsa-miR-371b-5p, hsa-miR-371b-3p, hsa-miR-4754, hsa-miR-4755-3p, hsa-miR-499b-3p, hsa-miR-4758-5p, hsa-miR-4758-3p, hsa-miR-4763-5p, hsa-miR-4763-3p, hsa-miR-4764-5p, hsa-miR-4766-5p, hsa-miR-4769-5p, hsa-miR-4769-3p, hsa-miR-4776-5p, hsa-miR-4778-5p, hsa-miR-4779, hsa-miR-4780, hsa-miR-4436b-5p, hsa-miR-4436b-3p, hsa-miR-4781-5p, hsa-miR-4783-3p, hsa-miR-2467-3p, hsa-miR-4788, hsa-miR-4793-3p, hsa-miR-4795-3p, hsa-miR-4800-5p, hsa-miR-642a-3p, hsa-miR-4433a-5p, hsa-miR-4482-3p, hsa-miR-4999-5p, hsa-miR-5000-3p, hsa-miR-5001-5p, hsa-miR-5002-3p, hsa-miR-5003-3p, hsa-miR-5006-5p, hsa-miR-5009-3p, hsa-miR-5010-5p, hsa-miR-5088-5p, hsa-miR-5090, hsa-miR-5188, hsa-miR-5189-5p, hsa-miR-5195-5p, hsa-miR-5195-3p, hsa-miR-5196-5p, hsa-miR-5100, hsa-miR-5572, hsa-miR-664b-5p, hsa-miR-5584-5p, hsa-miR-5588-5p, hsa-miR-5589-5p, hsa-miR-5698, hsa-miR-5703, hsa-miR-5705, hsa-miR-5707, hsa-miR-197-5p, hsa-miR-204-3p, hsa-miR-211-3p, hsa-miR-345-3p, hsa-miR-652-5p, hsa-miR-1185-2-3p, hsa-miR-873-3p, hsa-miR-1304-3p, hsa-miR-1247-3p, hsa-miR-3184-3p, hsa-miR-642b-5p, hsa-miR-365b-5p, hsa-miR-1185-1-3p, hsa-miR-3190-3p, hsa-miR-381-5p, hsa-miR-937-5p, hsa-miR-1227-5p, hsa-miR-1229-5p, hsa-miR-1233-5p, hsa-miR-1236-5p, hsa-miR-1237-5p, hsa-miR-1238-5p, hsa-miR-3620-5p, hsa-miR-3934-3p, hsa-miR-4632-5p, hsa-miR-4743-3p, hsa-miR-4750-3p, hsa-miR-5739, hsa-miR-5787, hsa-miR-6069, hsa-miR-6071, hsa-miR-6072, hsa-miR-6075, hsa-miR-6076, hsa-miR-6077, hsa-miR-6085, hsa-miR-6086, hsa-miR-6088, hsa-miR-6089, hsa-miR-6090, hsa-miR-6124, hsa-miR-6125, hsa-miR-6126, hsa-miR-6127, hsa-miR-378j, hsa-miR-6131, hsa-miR-6132, hsa-miR-6133, hsa-miR-6165, hsa-miR-6501-3p, hsa-miR-6506-5p, hsa-miR-6506-3p, hsa-miR-6510-5p, hsa-miR-6511a-5p, hsa-miR-6512-3p, hsa-miR-6513-5p, hsa-miR-6514-3p, hsa-miR-6515-5p, hsa-miR-6515-3p, hsa-miR-6716-5p, hsa-miR-6716-3p, hsa-miR-6717-5p, hsa-miR-6511b-5p, hsa-miR-6511b-3p, hsa-miR-6718-5p, hsa-miR-6719-3p, hsa-miR-6721-5p, hsa-miR-6722-5p, hsa-miR-6722-3p, hsa-miR-6724-5p, hsa-miR-208a-5p, hsa-miR-210-5p, hsa-miR-128-1-5p, hsa-miR-328-5p, hsa-miR-329-5p, hsa-miR-410-5p, hsa-miR-181d-3p, hsa-miR-504-3p, hsa-miR-487b-5p, hsa-miR-585-5p, hsa-miR-598-5p, hsa-miR-605-3p, hsa-miR-619-5p, hsa-miR-1296-3p, hsa-miR-874-5p, hsa-miR-887-5p, hsa-miR-1266-3p, hsa-miR-1343-5p, hsa-miR-5189-3p, hsa-miR-5699-5p, hsa-miR-6726-5p, hsa-miR-6728-5p, hsa-miR-6728-3p, hsa-miR-6729-5p, hsa-miR-6729-3p, hsa-miR-6730-5p, hsa-miR-6730-3p, hsa-miR-6731-3p, hsa-miR-6732-5p, hsa-miR-6734-5p, hsa-miR-6735-5p, hsa-miR-6736-5p, hsa-miR-6736-3p, hsa-miR-6737-5p, hsa-miR-6738-5p, hsa-miR-6738-3p, hsa-miR-6740-5p, hsa-miR-6740-3p, hsa-miR-6741-5p, hsa-miR-6742-5p, hsa-miR-6742-3p, hsa-miR-6743-5p, hsa-miR-6744-5p, hsa-miR-6745, hsa-miR-6746-5p, hsa-miR-6747-5p, hsa-miR-6747-3p, hsa-miR-6748-5p, hsa-miR-6749-5p, hsa-miR-6749-3p, hsa-miR-6751-5p, hsa-miR-6752-5p, hsa-miR-6752-3p, hsa-miR-6753-5p, hsa-miR-6754-5p, hsa-miR-6754-3p, hsa-miR-6755-5p, hsa-miR-6756-5p, hsa-miR-6756-3p, hsa-miR-6757-5p, hsa-miR-6758-5p, hsa-miR-6759-5p, hsa-miR-6759-3p, hsa-miR-6760-5p, hsa-miR-6760-3p, hsa-miR-6761-3p, hsa-miR-6762-5p, hsa-miR-6762-3p, hsa-miR-6763-5p, hsa-miR-6763-3p, hsa-miR-6765-5p, hsa-miR-6766-5p, hsa-miR-6766-3p, hsa-miR-6767-5p, hsa-miR-6768-5p, hsa-miR-6769a-5p, hsa-miR-6769a-3p, hsa-miR-6770-5p, hsa-miR-6772-5p, hsa-miR-6772-3p, hsa-miR-6773-5p, hsa-miR-6774-5p, hsa-miR-6775-5p, hsa-miR-6775-3p, hsa-miR-6776-5p, hsa-miR-6776-3p, hsa-miR-6777-5p, hsa-miR-6777-3p, hsa-miR-6778-5p, hsa-miR-6778-3p, hsa-miR-6779-5p, hsa-miR-6781-5p, hsa-miR-6782-5p, hsa-miR-6782-3p, hsa-miR-6783-5p, hsa-miR-6783-3p, hsa-miR-6784-5p, hsa-miR-6784-3p, hsa-miR-6785-3p, hsa-miR-6786-5p, hsa-miR-6786-3p, hsa-miR-6787-5p, hsa-miR-6787-3p, hsa-miR-6789-5p, hsa-miR-6789-3p, hsa-miR-6790-5p, hsa-miR-6790-3p, hsa-miR-6791-5p, hsa-miR-6792-5p, hsa-miR-6792-3p, hsa-miR-6793-5p, hsa-miR-6793-3p, hsa-miR-6794-5p, hsa-miR-6795-5p, hsa-miR-6795-3p, hsa-miR-6796-5p, hsa-miR-6796-3p, hsa-miR-6797-5p, hsa-miR-6797-3p, hsa-miR-6798-5p, hsa-miR-6798-3p, hsa-miR-6799-5p, hsa-miR-6800-5p, hsa-miR-6800-3p, hsa-miR-6801-3p, hsa-miR-6802-5p, hsa-miR-6802-3p, hsa-miR-6803-5p, hsa-miR-6804-3p, hsa-miR-6805-5p, hsa-miR-6805-3p, hsa-miR-6806-5p, hsa-miR-6806-3p, hsa-miR-6807-5p, hsa-miR-6808-5p, hsa-miR-6808-3p, hsa-miR-6809-5p, hsa-miR-6809-3p, hsa-miR-6810-5p, hsa-miR-6810-3p, hsa-miR-6812-5p, hsa-miR-6812-3p, hsa-miR-6813-5p, hsa-miR-6813-3p, hsa-miR-6815-5p, hsa-miR-6815-3p, hsa-miR-6816-5p, hsa-miR-6819-5p, hsa-miR-6819-3p, hsa-miR-6820-5p, hsa-miR-6820-3p, hsa-miR-6821-5p, hsa-miR-6822-5p, hsa-miR-6822-3p, hsa-miR-6823-5p, hsa-miR-6824-5p, hsa-miR-6825-5p, hsa-miR-6826-3p, hsa-miR-6827-5p, hsa-miR-6828-5p, hsa-miR-6829-5p, hsa-miR-6830-5p, hsa-miR-6831-5p, hsa-miR-6832-5p, hsa-miR-6833-5p, hsa-miR-6834-5p, hsa-miR-6780b-5p, hsa-miR-6836-3p, hsa-miR-6837-5p, hsa-miR-6838-5p, hsa-miR-6839-5p, hsa-miR-6840-5p, hsa-miR-6840-3p, hsa-miR-6841-3p, hsa-miR-6842-5p, hsa-miR-6845-5p, hsa-miR-6845-3p, hsa-miR-6846-5p, hsa-miR-6846-3p, hsa-miR-6848-5p, hsa-miR-6848-3p, hsa-miR-6849-5p, hsa-miR-6850-5p, hsa-miR-6851-5p, hsa-miR-6851-3p, hsa-miR-6855-5p, hsa-miR-6855-3p, hsa-miR-6856-5p, hsa-miR-6857-5p, hsa-miR-6857-3p, hsa-miR-6858-5p, hsa-miR-6858-3p, hsa-miR-6859-5p, hsa-miR-6859-3p, hsa-miR-6769b-5p, hsa-miR-6860, hsa-miR-6861-5p, hsa-miR-6861-3p, hsa-miR-6862-5p, hsa-miR-6864-5p, hsa-miR-6867-5p, hsa-miR-6869-5p, hsa-miR-6870-5p, hsa-miR-6870-3p, hsa-miR-6871-5p, hsa-miR-6874-5p, hsa-miR-6875-5p, hsa-miR-6875-3p, hsa-miR-6876-5p, hsa-miR-6877-5p, hsa-miR-6879-5p, hsa-miR-6879-3p, hsa-miR-6880-5p, hsa-miR-6880-3p, hsa-miR-6881-5p, hsa-miR-6882-5p, hsa-miR-6882-3p, hsa-miR-6883-5p, hsa-miR-6883-3p, hsa-miR-6884-3p, hsa-miR-6885-3p, hsa-miR-6886-5p, hsa-miR-6886-3p, hsa-miR-6887-5p, hsa-miR-6887-3p, hsa-miR-6888-3p, hsa-miR-6889-5p, hsa-miR-6889-3p, hsa-miR-6890-3p, hsa-miR-6891-5p, hsa-miR-6891-3p, hsa-miR-6892-5p, hsa-miR-6892-3p, hsa-miR-6893-5p, hsa-miR-6893-3p, hsa-miR-6894-5p, hsa-miR-7106-5p, hsa-miR-7107-5p, hsa-miR-7108-5p, hsa-miR-7108-3p, hsa-miR-7109-5p, hsa-miR-7109-3p, hsa-miR-7110-5p, hsa-miR-7111-Sp, hsa-miR-7111-3p, hsa-miR-7112-5p, hsa-miR-7113-3p, hsa-miR-7114-5p, hsa-miR-7114-3p, hsa-miR-7150, hsa-miR-7151-3p, hsa-miR-7152-5p, hsa-miR-7152-3p, hsa-miR-7155-5p, hsa-miR-7158-3p, hsa-miR-7159-5p, hsa-miR-7160-5p, hsa-miR-7162-5p, hsa-miR-7515, hsa-miR-7843-5p, hsa-miR-4433b-5p, hsa-miR-4433b-3p, hsa-miR-1273h-5p, hsa-miR-6516-5p, hsa-miR-7845-5p, hsa-miR-7846-3p, hsa-miR-7847-3p, hsa-miR-7851-3p, hsa-miR-7855-5p, hsa-miR-7856-5p, hsa-miR-8052, hsa-miR-8054, hsa-miR-8056, hsa-miR-8059, hsa-miR-8060, hsa-miR-8063, hsa-miR-8064, hsa-miR-8069, hsa-miR-8071, hsa-miR-8072, hsa-miR-8073, hsa-miR-8074, hsa-miR-8075, hsa-miR-8077, hsa-miR-8080, hsa-miR-8085, hsa-miR-8087, hsa-miR-8089, hsa-miR-7977, hsa-miR-7978, hsa-miR-1249-5p, hsa-miR-4485-5p, hsa-miR-8485, hsa-miR-498-3p, hsa-miR-526a-3p, hsa-miR-9718, hsa-miR-9898, hsa-miR-1843, hsa-miR-10392-5p, hsa-miR-10392-3p, hsa-miR-10394-3p, hsa-miR-10396a-5p, hsa-miR-10396a-3p, hsa-miR-10398-5p, hsa-miR-10398-3p, hsa-miR-10399-3p, hsa-miR-10400-3p, hsa-miR-10401-5p, hsa-miR-10401-3p, hsa-miR-10396b-5p, hsa-miR-10522-5p, hsa-miR-10526-3p, hsa-miR-10527-5p, hsa-miR-11181-3p, hsa-miR-11399, hsa-miR-3059-5p, hsa-miR-3059-3p, hsa-miR-3085-5p, hsa-miR-3085-3p, hsa-miR-6529-5p, hsa-miR-12114, hsa-miR-12115, hsa-miR-12116, hsa-miR-12118, hsa-miR-12119, hsa-miR-12120, hsa-miR-12121, hsa-miR-12124, hsa-miR-12126, hsa-miR-12131, and hsa-miR-12136. In the present disclosure, the extract of urine may be an extract of the urine of a stage-I urothelial carcinoma patient. Only one of these microRNAs can be used to predict whether a subject from which the urine is derived is an urothelial carcinoma patient (particularly stage-I), and 5 or more types, 10 or more types, 15 or more types, or 20 or more types of these microRNAs can be used in combination to predict whether a subject from which the urine is derived is an urothelial carcinoma patient (particularly stage-I). Among these microRNAs, a microRNA that may be highly expressed in an urothelial carcinoma patient (particularly stage-I) can be preferably used for prediction.

The present disclosure provides an extract of urine containing at least one type, two types, three types, four types, five types, six types, seven types, eight types, nine types, or ten types of or all microRNAs selected from the group consisting of microRNAs having an accuracy of higher than 0.500 among the microRNAs listed in Table 24-9. In the present disclosure, the extract of urine may be an extract of the urine of an urothelial carcinoma patient. Only one of these microRNAs can be used to predict whether a subject from which the urine is derived is an urothelial carcinoma patient, and 5 or more types, 10 or more types, 15 or more types, or 20 or more types of these microRNAs can be used in combination to predict whether a subject from which the urine is derived is an urothelial carcinoma patient. Among these microRNAs, a microRNA that may be highly expressed in an urothelial carcinoma patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

The present disclosure provides an extract of urine containing at least one type, two types, three types, four types, five types, six types, seven types, eight types, nine types, or ten types of or all microRNAs selected from the group consisting of microRNAs having an accuracy of higher than 0.500 among the microRNAs listed in Table 25-9. In the present disclosure, the extract of urine may be an extract of the urine of an urothelial carcinoma patient. Only one of these microRNAs can be used to predict whether a subject from which the urine is derived is an urothelial carcinoma patient, and 5 or more types, 10 or more types, 15 or more types, or 20 or more types of these microRNAs can be used in combination to predict whether a subject from which the urine is derived is an urothelial carcinoma patient. Among these microRNAs, a microRNA that may be highly expressed in an urothelial carcinoma patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

The present disclosure provides an extract of urine containing at least one type, two types, three types, four types, five types, six types, seven types, eight types, nine types, or ten types of or all microRNAs selected from the microRNAs listed in Table 4-10. The present disclosure also provides an extract of urine containing at least one type, two types, three types, four types, five types, six types, seven types, eight types, nine types, or ten types of or all microRNAs selected from the group consisting of hsa-miR-1292-5p, hsa-miR-3978, hsa-miR-8079, hsa-miR-6719-3p, hsa-miR-605-3p, hsa-miR-10522-5p, hsa-miR-6809-5p, hsa-miR-6808-3p, hsa-miR-5584-5p, hsa-miR-181d-3p, hsa-miR-1914-3p, hsa-miR-380-3p, hsa-miR-4715-5p, hsa-miR-10526-3p, hsa-miR-8078, hsa-miR-127-5p, hsa-miR-4755-3p, hsa-miR-145-5p, hsa-miR-3133, hsa-miR-5196-5p, hsa-miR-489-5p, hsa-miR-7161-5p, hsa-miR-498-5p, hsa-miR-1253, hsa-miR-4293, hsa-miR-2681-3p, hsa-miR-4802-3p, hsa-miR-4433b-3p, hsa-miR-449a, hsa-miR-1185-2-3p, hsa-miR-6761-5p, hsa-miR-8080, hsa-miR-6795-5p, hsa-miR-1185-1-3p, hsa-miR-4280, hsa-miR-6812-5p, hsa-miR-708-5p, hsa-miR-29b-1-5p, hsa-miR-451b, hsa-miR-6516-5p, hsa-miR-4512, hsa-miR-6794-5p, hsa-miR-3129-3p, hsa-miR-4716-3p, hsa-miR-3155a, hsa-miR-6887-5p, hsa-miR-4645-3p, hsa-miR-4778-5p, hsa-miR-5579-5p, hsa-miR-12131, hsa-miR-1912-3p, hsa-miR-6124, hsa-miR-3059-3p, hsa-miR-150-3p, hsa-miR-4433a-3p, hsa-miR-3907, hsa-miR-6784-3p, hsa-miR-6783-5p, hsa-miR-6832-5p, hsa-miR-7109-5p, hsa-miR-1207-5p, hsa-miR-6775-5p, hsa-miR-598-5p, hsa-miR-4271, hsa-miR-106a-3p, hsa-miR-3130-3p, hsa-miR-761, hsa-miR-519b-3p, hsa-miR-1269a, hsa-miR-4716-5p, hsa-miR-6880-3p, hsa-miR-6845-3p, hsa-miR-200a-5p, hsa-miR-7111-5p, hsa-miR-4761-3p, hsa-miR-7106-5p, hsa-miR-4665-5p, hsa-miR-6760-3p, hsa-miR-4793-5p, hsa-miR-3162-3p, hsa-miR-6758-5p, hsa-miR-4706, hsa-miR-324-5p, hsa-miR-4521, hsa-miR-7107-5p, hsa-miR-6797-5p, hsa-miR-4281, hsa-miR-711, hsa-miR-6071, hsa-miR-186-3p, hsa-miR-6839-5p, hsa-miR-6800-3p, hsa-miR-6746-5p, hsa-miR-4433b-5p, hsa-miR-4999-5p, hsa-miR-4668-5p, hsa-miR-4495, hsa-miR-1299, hsa-miR-652-3p, hsa-miR-4525, hsa-miR-6838-5p, hsa-miR-4691-3p, hsa-miR-7114-3p, hsa-miR-4295, hsa-miR-940, hsa-miR-4286, hsa-miR-1225-5p, hsa-miR-6795-3p, hsa-miR-382-5p, hsa-miR-4661-5p, hsa-miR-4738-5p, hsa-miR-4728-5p, hsa-miR-4299, hsa-miR-6848-3p, hsa-miR-6513-5p, hsa-miR-302c-5p, hsa-miR-662, hsa-miR-30c-2-3p, hsa-miR-4289, hsa-miR-4472, hsa-miR-6742-5p, hsa-miR-3619-3p, hsa-miR-3124-5p, hsa-miR-6810-3p, hsa-miR-6766-3p, hsa-miR-4769-3p, hsa-miR-6798-3p, hsa-miR-6785-3p, hsa-miR-1303, hsa-miR-421, hsa-miR-6777-3p, hsa-miR-4703-3p, hsa-miR-6752-3p, hsa-miR-4800-3p, hsa-miR-449c-5p, hsa-miR-6749-5p, hsa-miR-3675-3p, hsa-miR-6086, hsa-miR-3144-5p, hsa-miR-7856-5p, hsa-miR-4733-5p, hsa-miR-1225-3p, hsa-miR-525-3p, hsa-miR-6165, hsa-miR-6829-5p, hsa-miR-6501-5p, hsa-miR-6716-5p, hsa-miR-6763-3p, hsa-miR-4749-3p, hsa-miR-514b-5p, hsa-miR-4750-3p, hsa-miR-4534, hsa-miR-7706, hsa-miR-5195-3p, hsa-miR-4658, hsa-miR-125b-2-3p, hsa-miR-625-3p, hsa-miR-6500-5p, hsa-miR-4284, hsa-miR-450a-5p, hsa-miR-3186-3p, hsa-miR-3619-5p, hsa-miR-4274, hsa-miR-494-3p, hsa-miR-4294, hsa-miR-1224-3p, hsa-miR-6891-5p, hsa-miR-7150, hsa-miR-6839-3p, hsa-miR-3679-3p, hsa-miR-5091, hsa-miR-297, hsa-miR-4522, hsa-miR-6085, hsa-miR-551b-5p, hsa-miR-4775, hsa-miR-4311, hsa-miR-323a-3p, hsa-miR-1249-3p, hsa-miR-8064, hsa-miR-627-5p, hsa-miR-4441, hsa-miR-1913, hsa-miR-383-5p, hsa-miR-4313, hsa-miR-4257, hsa-miR-6892-3p, hsa-miR-223-5p, hsa-miR-3943, hsa-miR-611, hsa-miR-6819-3p, hsa-miR-4444, hsa-miR-6805-3p, hsa-miR-6796-3p, hsa-miR-6515-3p, hsa-miR-3131, hsa-miR-320c, hsa-miR-4731-3p, hsa-miR-4637, hsa-miR-5579-3p, hsa-miR-208b-5p, hsa-miR-4323, hsa-miR-5585-5p, hsa-miR-1255a, hsa-miR-548as-3p, hsa-miR-184, hsa-miR-6813-3p, hsa-miR-2115-5p, hsa-miR-9898, hsa-miR-548g-3p, hsa-miR-4321, hsa-miR-583, hsa-miR-4781-5p, hsa-miR-4665-3p, hsa-miR-3926, hsa-miR-7851-3p, hsa-miR-6859-3p, hsa-miR-1281, hsa-miR-6070, hsa-miR-516a-5p, hsa-miR-520b-3p, hsa-miR-4433a-5p, hsa-miR-6836-3p, hsa-miR-4496, hsa-miR-4785, hsa-miR-330-3p, hsa-miR-135a-2-3p, hsa-miR-515-5p, hsa-miR-4327, hsa-miR-625-5p, hsa-miR-8088, hsa-miR-5006-5p, hsa-miR-7151-5p, hsa-miR-135a-3p, hsa-miR-6769b-5p, hsa-miR-12119, hsa-miR-3689d, hsa-miR-4436b-3p, hsa-miR-1228-3p, hsa-miR-6817-5p, hsa-miR-520e-3p, hsa-miR-1270, hsa-miR-365a-3p, hsa-miR-365b-3p, hsa-miR-1238-3p, hsa-miR-6069, hsa-miR-2117, hsa-miR-198, hsa-miR-617, hsa-miR-6730-5p, hsa-miR-3199, hsa-miR-513b-5p, hsa-miR-6081, hsa-miR-6731-3p, hsa-miR-4725-3p, hsa-miR-210-5p, hsa-miR-513a-5p, hsa-miR-6729-3p, hsa-miR-601, hsa-miR-656-3p, hsa-miR-4723-5p, hsa-miR-4697-5p, hsa-miR-1825, hsa-miR-1208, hsa-miR-942-5p, hsa-miR-3155b, hsa-miR-3614-5p, hsa-miR-4531, hsa-miR-4648, hsa-miR-30b-3p, hsa-miR-6780a-5p, hsa-miR-4794, hsa-miR-92a-2-5p, hsa-miR-4684-3p, hsa-miR-6887-3p, hsa-miR-12116, hsa-miR-647, hsa-miR-6752-5p, hsa-miR-6815-5p, hsa-miR-5681a, hsa-miR-519a-3p, hsa-miR-4494, hsa-miR-361-3p, hsa-miR-4451, hsa-miR-646, hsa-miR-4682, hsa-miR-5002-5p, hsa-miR-6861-3p, hsa-miR-6774-5p, hsa-miR-25-3p, hsa-miR-2116-3p, hsa-miR-5739, hsa-miR-501-5p, hsa-miR-6798-5p, hsa-miR-6855-3p, hsa-miR-3934-3p, hsa-miR-5580-5p, hsa-miR-450a-2-3p, hsa-miR-6808-5p, hsa-miR-196b-3p, hsa-miR-485-5p, hsa-miR-3193, hsa-miR-21-3p, hsa-miR-12113, hsa-miR-4466, hsa-let-7e-5p, hsa-miR-483-3p, hsa-miR-3922-3p, hsa-miR-3667-5p, hsa-miR-6760-5p, hsa-miR-7108-3p, hsa-miR-4316, hsa-miR-7974, hsa-miR-484, hsa-miR-181a-2-3p, hsa-miR-877-3p, hsa-miR-296-5p, hsa-miR-210-3p, hsa-miR-4439, hsa-miR-152-5p, hsa-miR-3613-3p, hsa-miR-6876-3p, hsa-miR-4741, hsa-miR-3074-3p, hsa-miR-6792-3p, hsa-miR-619-5p, hsa-miR-6769a-5p, hsa-miR-6750-5p, hsa-miR-6504-5p, hsa-miR-3937, hsa-miR-3200-5p, hsa-miR-6886-3p, hsa-miR-6511a-5p, hsa-miR-9902, hsa-miR-1468-3p, hsa-miR-4701-3p, hsa-miR-4728-3p, hsa-miR-3612, hsa-miR-4707-3p, hsa-miR-744-3p, hsa-miR-554, hsa-miR-6734-5p, hsa-miR-4687-5p, hsa-miR-4768-3p, hsa-miR-6830-5p, hsa-miR-1267, hsa-let-7e-3p, hsa-miR-6088, hsa-miR-3142, hsa-miR-6504-3p, hsa-miR-4748, hsa-miR-4786-3p, hsa-miR-4279, hsa-miR-6789-5p, hsa-miR-4458, hsa-miR-4306, hsa-miR-6821-3p, hsa-miR-5010-5p, hsa-miR-6511b-5p, hsa-miR-197-3p, hsa-miR-5584-3p, hsa-miR-4670-5p, hsa-miR-5703, hsa-miR-4261, hsa-miR-371a-5p, hsa-miR-6854-3p, hsa-miR-6788-5p, hsa-miR-345-3p, hsa-miR-3173-5p, hsa-miR-3085-3p, hsa-miR-548as-5p, hsa-miR-548ah-5p, hsa-miR-224-5p, hsa-miR-511-3p, hsa-miR-4536-3p, hsa-miR-22-5p, hsa-miR-4480, hsa-miR-4492, hsa-miR-542-5p, hsa-miR-4747-5p, hsa-miR-6768-3p, hsa-miR-6872-5p, hsa-miR-1234-3p, hsa-miR-6793-3p, hsa-miR-1976, hsa-miR-3065-3p, hsa-miR-128-2-5p, hsa-miR-7854-3p, hsa-miR-1237-3p, hsa-miR-4687-3p, hsa-miR-132-3p, hsa-miR-3944-5p, hsa-miR-6840-5p, hsa-miR-6748-5p, hsa-miR-1288-3p, hsa-miR-6513-3p, hsa-miR-2355-5p, hsa-miR-6880-5p, hsa-miR-4298, hsa-miR-4484, hsa-miR-6782-5p, hsa-miR-503-3p, hsa-miR-1229-5p, hsa-miR-320e, hsa-miR-6738-5p, hsa-miR-4251, hsa-miR-4431, hsa-miR-3945, hsa-miR-8077, hsa-miR-6891-3p, hsa-miR-6745, hsa-miR-718, hsa-miR-4499, hsa-miR-7110-3p, hsa-miR-6805-5p, hsa-miR-4738-3p, hsa-miR-6801-3p, hsa-miR-6809-3p, hsa-miR-342-5p, hsa-miR-520h, hsa-miR-323b-3p, hsa-miR-1343-5p, hsa-miR-3162-5p, hsa-miR-3202, hsa-miR-6889-3p, hsa-miR-4450, hsa-miR-4449, hsa-miR-600, hsa-miR-6790-5p, hsa-miR-12118, hsa-miR-3934-5p, hsa-miR-550a-5p, hsa-miR-6857-3p, hsa-miR-325, hsa-miR-663b, hsa-miR-892a, hsa-miR-3922-5p, hsa-miR-135a-5p, hsa-miR-3713, hsa-miR-5693, hsa-miR-6869-3p, hsa-miR-936, hsa-miR-4423-3p, hsa-miR-5004-3p, hsa-miR-6503-3p, hsa-miR-6858-3p, hsa-miR-613, hsa-miR-378i, hsa-miR-6756-3p, hsa-miR-147b-3p, hsa-miR-550a-3-5p, hsa-miR-6797-3p, hsa-miR-1182, hsa-miR-6831-5p, hsa-miR-96-3p, hsa-miR-598-3p, hsa-miR-943, hsa-miR-650, hsa-miR-4317, hsa-miR-6501-3p, hsa-miR-3679-5p, hsa-miR-4685-5p, hsa-miR-3689a-3p, hsa-miR-4712-3p, hsa-miR-6728-5p, hsa-miR-4753-5p, hsa-miR-6768-5p, hsa-miR-5007-5p, hsa-miR-8087, hsa-miR-31-3p, hsa-miR-10392-3p, hsa-miR-4681, hsa-miR-3184-3p, hsa-miR-6779-5p, hsa-miR-769-3p, hsa-miR-1202, hsa-miR-7855-5p, hsa-miR-4290, hsa-miR-6895-5p, hsa-miR-4447, hsa-miR-18b-3p, hsa-miR-4502, hsa-miR-216b-3p, hsa-miR-93-5p, hsa-miR-4326, hsa-miR-6780b-5p, hsa-miR-6743-5p, hsa-miR-1343-3p, hsa-miR-1238-5p, hsa-miR-8059, hsa-miR-623, hsa-miR-10394-5p, hsa-miR-363-5p, hsa-miR-4538, hsa-miR-371b-5p, hsa-miR-4675, and hsa-miR-4776-3p. In the present disclosure, the extract of urine may be an extract of the urine of a melanoma patient. Only one of these microRNAs can be used to predict whether a subject from which the urine is derived is a melanoma patient, and 5 or more types, 10 or more types, 15 or more types, or 20 or more types of these microRNAs can be used in combination to predict whether a subject from which the urine is derived is a melanoma patient. Among these microRNAs, a microRNA that may be highly expressed in a melanoma patient can be preferably used for prediction.

The present disclosure provides an extract of urine containing at least one type, two types, three types, four types, five types, six types, seven types, eight types, nine types, or ten types of or all microRNAs selected from the group consisting of miR-25-3p, miR-197-3p, miR-198, miR-210-3p, miR-224-5p, miR-124-3p, miR-185-5p, miR-296-5p, miR-365a-3p, miR-365b-3p, miR-302c-5p, miR-370-3p, miR-373-5p, miR-380-3p, miR-383-5p, miR-330-3p, miR-328-3p, miR-449a, miR-483-3p, miR-484, miR-485-5p, miR-498-5p, miR-520e-3p, miR-519b-3p, miR-518f-3p, miR-520b-3p, miR-519a-3p, miR-513a-5p, miR-18a-3p, miR-575, miR-583, miR-611, miR-625-5p, miR-632, miR-634, miR-652-3p, miR-661, miR-658, miR-668-3p, miR-765, miR-22-5p, miR-92a-2-5p, miR-101-5p, miR-29b-1-5p, miR-30c-2-3p, miR-181a-2-3p, miR-183-3p, miR-187-5p, miR-219a-1-3p, miR-30b-3p, miR-125b-1-3p, miR-127-5p, miR-149-3p, miR-150-3p, miR-186-3p, miR-30c-1-3p, miR-361-3p, miR-371a-5p, miR-342-5p, miR-18b-3p, miR-431-3p, miR-483-5p, miR-490-5p, miR-92b-5p, miR-550a-5p, miR-615-5p, miR-625-3p, miR-654-3p, miR-541-5p, miR-708-5p, miR-744-3p, miR-885-5p, miR-877-5p, miR-877-3p, miR-887-3p, miR-665, miR-920, miR-936, miR-940, miR-943, miR-1224-3p, miR-1225-5p, miR-1225-3p, miR-1226-3p, miR-1228-3p, miR-1229-3p, miR-1231, miR-1234-3p, miR-1236-3p, miR-1237-3p, miR-1238-3p, miR-1182, miR-1184, miR-1202, miR-1203, miR-663b, miR-1204, miR-1207-5p, miR-1208, miR-1299, miR-1249-3p, miR-1253, miR-1255a, miR-1267, miR-1275, miR-1281, miR-1292-5p, miR-1252-5p, miR-1825, miR-1469, miR-1908-5p, miR-1909-5p, miR-1911-5p, miR-1912-3p, miR-1913, miR-1914-3p, miR-365a-5p, miR-196b-3p, miR-1976, miR-670-5p, miR-2116-3p, miR-711, miR-718, miR-2681-3p, miR-3116, miR-3122, miR-3130-5p, miR-3131, miR-3132, miR-3133, miR-3138, miR-3144-5p, miR-3147, miR-3153, miR-3074-3p, miR-3155a, miR-3156-5p, miR-3162-5p, miR-3173-3p, miR-1193, miR-3178, miR-3196, miR-3198, miR-3200-3p, miR-514b-5p, miR-3202, miR-3065-3p, miR-4297, miR-4293, miR-4294, miR-4299, miR-4298, miR-4306, miR-4312, miR-4313, miR-4316, miR-4322, miR-4323, miR-4257, miR-4258, miR-4259, miR-4260, miR-4253, miR-4251, miR-4326, miR-4327, miR-4265, miR-4267, miR-2355-5p, miR-4271, miR-4276, miR-4274, miR-4281, miR-4279, miR-4280, miR-4284, miR-4286, miR-4288, miR-4290, miR-3200-5p, miR-3610, miR-3612, miR-3614-5p, miR-3615, miR-3616-3p, miR-3619-5p, miR-3622a-5p, miR-3622a-3p, miR-3646, miR-3648, miR-3675-3p, miR-3679-5p, miR-3679-3p, miR-3713, miR-3714, miR-3180, miR-3907, miR-3917, miR-3922-3p, miR-3924, miR-3926, miR-3936, miR-3937, miR-3943, miR-3945, miR-550b-3p, miR-548ab, miR-4418, miR-4432, miR-4433a-3p, miR-4441, miR-4444, miR-4447, miR-4449, miR-548ah-5p, miR-4462, miR-4466, miR-4472, miR-3689d, miR-3155b, miR-4480, miR-4484, miR-4492, miR-4495, miR-4496, miR-4499, miR-4512, miR-4521, miR-4525, miR-4526, miR-4531, miR-4534, miR-3127-3p, miR-3158-5p, miR-3162-3p, miR-3173-5p, miR-3619-3p, miR-3691-3p, miR-3944-5p, miR-3978, miR-4634, miR-4637, miR-4638-5p, miR-4648, miR-4652-5p, miR-4661-5p, miR-4664-5p, miR-4665-5p, miR-4665-3p, miR-4667-3p, miR-4668-5p, miR-4674, miR-4685-5p, miR-4685-3p, miR-4687-5p, miR-4687-3p, miR-1343-3p, miR-4691-5p, miR-4691-3p, miR-4697-5p, miR-4698, miR-4706, miR-4707-5p, miR-4707-3p, miR-4712-5p, miR-4715-5p, miR-4716-5p, miR-4716-3p, miR-4717-3p, miR-4723-5p, miR-4723-3p, miR-451b, miR-4725-5p, miR-4725-3p, miR-4726-3p, miR-4728-5p, miR-4728-3p, miR-4731-5p, miR-4731-3p, miR-4733-5p, miR-4738-5p, miR-4741, miR-4745-5p, miR-4747-5p, miR-4748, miR-4749-3p, miR-4750-5p, miR-4751, miR-371b-5p, miR-4755-3p, miR-4758-5p, miR-4758-3p, miR-4761-3p, miR-4769-3p, miR-4778-5p, miR-4781-5p, miR-4783-3p, miR-4793-5p, miR-4793-3p, miR-4800-5p, miR-4802-3p, miR-642a-3p, miR-4433a-5p, miR-4482-3p, miR-4536-3p, miR-5001-3p, miR-5006-5p, miR-5010-5p, miR-5187-3p, miR-5193, miR-5195-5p, miR-5195-3p, miR-5196-5p, miR-548as-5p, miR-548as-3p, miR-5579-5p, miR-5579-3p, miR-5584-5p, miR-5584-3p, miR-5585-5p, miR-5587-3p, miR-5698, miR-211-3p, miR-345-3p, miR-652-5p, miR-1185-2-3p, miR-873-3p, miR-1247-3p, miR-3184-3p, miR-365b-5p, miR-1185-1-3p, miR-503-3p, miR-1229-5p, miR-1238-5p, miR-4750-3p, miR-5739, miR-6069, miR-6071, miR-6085, miR-6086, miR-6088, miR-6124, miR-6133, miR-6165, miR-6501-5p, miR-6503-3p, miR-6505-3p, miR-6507-5p, miR-6511a-5p, miR-6512-5p, miR-6512-3p, miR-6514-3p, miR-6515-3p, miR-6716-5p, miR-6511b-5p, miR-6719-3p, miR-6720-3p, miR-6722-5p, miR-208a-5p, miR-210-5p, miR-383-3p, miR-487a-5p, miR-489-5p, miR-511-3p, miR-181d-3p, miR-598-5p, miR-605-3p, miR-619-5p, miR-627-3p, miR-1296-3p, miR-1468-3p, miR-874-5p, miR-216b-3p, miR-208b-5p, miR-1287-3p, miR-3151-3p, miR-1343-5p, miR-5699-5p, miR-6728-5p, miR-6728-3p, miR-6729-3p, miR-6730-5p, miR-6731-3p, miR-6732-3p, miR-6734-5p, miR-6738-5p, miR-6740-3p, miR-6741-3p, miR-6742-5p, miR-6742-3p, miR-6743-5p, miR-6745, miR-6746-5p, miR-6746-3p, miR-6748-5p, miR-6749-5p, miR-6749-3p, miR-6752-5p, miR-6752-3p, miR-6753-5p, miR-6754-3p, miR-6756-3p, miR-6758-5p, miR-6758-3p, miR-6759-5p, miR-6759-3p, miR-6760-5p, miR-6760-3p, miR-6761-3p, miR-6763-3p, miR-6764-3p, miR-6766-5p, miR-6766-3p, miR-6768-5p, miR-6769a-5p, miR-6769a-3p, miR-6772-3p, miR-6774-5p, miR-6775-5p, miR-6776-3p, miR-6777-5p, miR-6777-3p, miR-6779-5p, miR-6780a-5p, miR-6782-5p, miR-6782-3p, miR-6784-3p, miR-6785-3p, miR-6787-3p, miR-6788-5p, miR-6789-5p, miR-6789-3p, miR-6790-5p, miR-6792-5p, miR-6792-3p, miR-6793-3p, miR-6794-5p, miR-6794-3p, miR-6795-5p, miR-6795-3p, miR-6796-3p, miR-6797-5p, miR-6797-3p, miR-6798-5p, miR-6798-3p, miR-6799-5p, miR-6800-3p, miR-6801-3p, miR-6802-3p, miR-6803-3p, miR-6805-5p, miR-6805-3p, miR-6806-5p, miR-6807-5p, miR-6808-5p, miR-6808-3p, miR-6809-5p, miR-6809-3p, miR-6810-5p, miR-6810-3p, miR-6812-5p, miR-6812-3p, miR-6813-3p, miR-6815-5p, miR-6816-3p, miR-6817-3p, miR-6819-3p, miR-6820-3p, miR-6821-5p, miR-6821-3p, miR-6825-5p, miR-6826-3p, miR-6828-5p, miR-6829-5p, miR-6830-5p, miR-6830-3p, miR-6831-5p, miR-6832-5p, miR-6832-3p, miR-6833-5p, miR-6833-3p, miR-6780b-5p, miR-6780b-3p, miR-6836-3p, miR-6837-3p, miR-6839-3p, miR-6840-5p, miR-6842-5p, miR-6845-3p, miR-6848-3p, miR-6849-5p, miR-6849-3p, miR-6850-5p, miR-6852-3p, miR-6854-3p, miR-6855-3p, miR-6857-3p, miR-6858-3p, miR-6859-3p, miR-6769b-5p, miR-6861-3p, miR-6862-3p, miR-6867-5p, miR-6868-3p, miR-6870-5p, miR-6870-3p, miR-6872-5p, miR-6873-5p, miR-6876-3p, miR-6877-3p, miR-6880-5p, miR-6880-3p, miR-6881-5p, miR-6883-5p, miR-6883-3p, miR-6884-3p, miR-6885-3p, miR-6886-3p, miR-6887-5p, miR-6887-3p, miR-6889-5p, miR-6889-3p, miR-6891-5p, miR-6891-3p, miR-6892-3p, miR-6893-5p, miR-6893-3p, miR-6894-3p, miR-6895-5p, miR-6895-3p, miR-7106-5p, miR-7107-5p, miR-7108-5p, miR-7108-3p, miR-7109-5p, miR-7109-3p, miR-7110-3p, miR-7111-5p, miR-7111-3p, miR-7112-5p, miR-7113-3p, miR-7114-3p, miR-7150, miR-7151-5p, miR-7152-5p, miR-7161-5p, miR-7843-5p, miR-4433b-5p, miR-4433b-3p, miR-1273h-5p, miR-7852-3p, miR-7855-5p, miR-8059, miR-8071, miR-8078, miR-8079, miR-8087, miR-128-2-5p, miR-7974, miR-1249-5p, miR-196a-1-3p, miR-137-5p, miR-190b-3p, miR-9898, miR-10392-3p, miR-10394-3p, miR-10396a-3p, miR-10398-5p, miR-10400-3p, miR-10401-5p, miR-10522-5p, miR-10526-3p, miR-11181-3p, miR-3059-5p, miR-3059-3p, miR-3085-3p, miR-12116, miR-12118, miR-12119, miR-12128, and miR-12131. In the present disclosure, the extract of urine may be an extract of the urine of a stage-I or -II melanoma patient. Only one of these microRNAs can be used to predict whether a subject from which the urine is derived is a melanoma patient (particularly stage-I or -II), and 5 or more types, 10 or more types, 15 or more types, or 20 or more types of these microRNAs can be used in combination to predict whether a subject from which the urine is derived is a melanoma patient (particularly stage-I or -II). Among these microRNAs, a microRNA that may be highly expressed in a melanoma patient (particularly stage-I or -II) can be preferably used for prediction.

The present disclosure provides an extract of urine containing at least one type, two types, three types, four types, five types, six types, seven types, eight types, nine types, or ten types of or all microRNAs selected from the group consisting of hsa-miR-25-3p, hsa-miR-197-3p, hsa-miR-198, hsa-miR-224-5p, hsa-miR-124-3p, hsa-miR-184, hsa-miR-296-5p, hsa-miR-365a-3p, hsa-miR-365b-3p, hsa-miR-302c-5p, hsa-miR-373-5p, hsa-miR-380-3p, hsa-miR-383-5p, hsa-miR-328-3p, hsa-miR-449a, hsa-miR-450a-5p, hsa-miR-483-3p, hsa-miR-484, hsa-miR-485-5p, hsa-miR-498-5p, hsa-miR-520e-3p, hsa-miR-519b-3p, hsa-miR-520b-3p, hsa-miR-513a-5p, hsa-miR-557, hsa-miR-571, hsa-miR-583, hsa-miR-611, hsa-miR-625-5p, hsa-miR-638, hsa-miR-652-3p, hsa-miR-663a, hsa-miR-658, hsa-miR-542-5p, hsa-miR-671-5p, hsa-miR-675-5p, hsa-miR-22-5p, hsa-miR-92a-2-5p, hsa-miR-29b-1-5p, hsa-miR-30c-2-3p, hsa-miR-181a-2-3p, hsa-miR-187-5p, hsa-miR-30b-3p, hsa-miR-125b-1-3p, hsa-miR-135a-3p, hsa-miR-145-3p, hsa-miR-127-5p, hsa-miR-149-3p, hsa-miR-150-3p, hsa-miR-186-3p, hsa-miR-361-3p, hsa-miR-371a-5p, hsa-miR-342-5p, hsa-miR-18b-3p, hsa-miR-490-5p, hsa-miR-92b-5p, hsa-miR-625-3p, hsa-miR-708-5p, hsa-miR-744-3p, hsa-miR-877-5p, hsa-miR-877-3p, hsa-miR-665, hsa-miR-940, hsa-miR-943, hsa-miR-1224-3p, hsa-miR-1225-5p, hsa-miR-1225-3p, hsa-miR-1228-5p, hsa-miR-1228-3p, hsa-miR-1234-3p, hsa-miR-1237-3p, hsa-miR-1238-3p, hsa-miR-1182, hsa-miR-1202, hsa-miR-663b, hsa-miR-1207-5p, hsa-miR-1208, hsa-miR-1299, hsa-miR-1249-3p, hsa-miR-1253, hsa-miR-1267, hsa-miR-1268a, hsa-miR-1281, hsa-miR-1292-5p, hsa-miR-1252-5p, hsa-miR-1825, hsa-miR-1469, hsa-miR-1908-5p, hsa-miR-1909-3p, hsa-miR-1911-5p, hsa-miR-1912-3p, hsa-miR-1913, hsa-miR-1914-3p, hsa-miR-1915-3p, hsa-miR-365a-5p, hsa-miR-196b-3p, hsa-miR-1976, hsa-miR-2053, hsa-miR-762, hsa-miR-2116-3p, hsa-miR-711, hsa-miR-718, hsa-miR-2681-3p, hsa-miR-3122, hsa-miR-3131, hsa-miR-3133, hsa-miR-3141, hsa-miR-3144-5p, hsa-miR-3147, hsa-miR-3074-3p, hsa-miR-3155a, hsa-miR-3162-5p, hsa-miR-3178, hsa-miR-3180-3p, hsa-miR-3196, hsa-miR-3197, hsa-miR-3200-3p, hsa-miR-514b-5p, hsa-miR-3202, hsa-miR-4293, hsa-miR-4294, hsa-miR-4299, hsa-miR-4298, hsa-miR-4306, hsa-miR-4313, hsa-miR-4316, hsa-miR-4323, hsa-miR-4257, hsa-miR-4258, hsa-miR-4327, hsa-miR-4265, hsa-miR-2355-5p, hsa-miR-4271, hsa-miR-4274, hsa-miR-4281, hsa-miR-4279, hsa-miR-4284, hsa-miR-4286, hsa-miR-4290, hsa-miR-3200-5p, hsa-miR-3610, hsa-miR-3612, hsa-miR-3614-5p, hsa-miR-3622a-5p, hsa-miR-3648, hsa-miR-3663-3p, hsa-miR-3665, hsa-miR-3675-3p, hsa-miR-3679-5p, hsa-miR-3679-3p, hsa-miR-3713, hsa-miR-3180, hsa-miR-3907, hsa-miR-3922-3p, hsa-miR-3924, hsa-miR-3926, hsa-miR-3936, hsa-miR-3937, hsa-miR-3943, hsa-miR-3945, hsa-miR-642b-3p, hsa-miR-1268b, hsa-miR-548ab, hsa-miR-4418, hsa-miR-4433a-3p, hsa-miR-4441, hsa-miR-4444, hsa-miR-4447, hsa-miR-4449, hsa-miR-548ah-5p, hsa-miR-4463, hsa-miR-4466, hsa-miR-4472, hsa-miR-3689d, hsa-miR-3155b, hsa-miR-4480, hsa-miR-4484, hsa-miR-4488, hsa-miR-4492, hsa-miR-4495, hsa-miR-4496, hsa-miR-4497, hsa-miR-4508, hsa-miR-4512, hsa-miR-4516, hsa-miR-4519, hsa-miR-4521, hsa-miR-4525, hsa-miR-4530, hsa-miR-4531, hsa-miR-4534, hsa-miR-3127-3p, hsa-miR-3158-5p, hsa-miR-3162-3p, hsa-miR-3173-5p, hsa-miR-3619-3p, hsa-miR-3691-3p, hsa-miR-3940-5p, hsa-miR-3978, hsa-miR-4634, hsa-miR-4637, hsa-miR-4648, hsa-miR-4649-5p, hsa-miR-4651, hsa-miR-4661-5p, hsa-miR-4665-5p, hsa-miR-4665-3p, hsa-miR-4668-5p, hsa-miR-4674, hsa-miR-4675, hsa-miR-4685-5p, hsa-miR-4687-5p, hsa-miR-4687-3p, hsa-miR-1343-3p, hsa-miR-4689, hsa-miR-4691-5p, hsa-miR-4691-3p, hsa-miR-4697-5p, hsa-miR-4706, hsa-miR-4707-5p, hsa-miR-4707-3p, hsa-miR-4715-5p, hsa-miR-4716-5p, hsa-miR-4716-3p, hsa-miR-4723-5p, hsa-miR-4723-3p, hsa-miR-451b, hsa-miR-4725-3p, hsa-miR-4726-3p, hsa-miR-4728-5p, hsa-miR-4728-3p, hsa-miR-4731-5p, hsa-miR-4731-3p, hsa-miR-4738-5p, hsa-miR-4741, hsa-miR-4745-5p, hsa-miR-4747-5p, hsa-miR-4748, hsa-miR-4749-5p, hsa-miR-4749-3p, hsa-miR-371b-5p, hsa-miR-371b-3p, hsa-miR-4755-3p, hsa-miR-4758-5p, hsa-miR-4758-3p, hsa-miR-4761-3p, hsa-miR-4763-3p, hsa-miR-4769-3p, hsa-miR-4778-5p, hsa-miR-4781-5p, hsa-miR-4783-3p, hsa-miR-4787-5p, hsa-miR-4802-3p, hsa-miR-642a-3p, hsa-miR-4433a-5p, hsa-miR-4536-3p, hsa-miR-5006-5p, hsa-miR-5010-5p, hsa-miR-5190, hsa-miR-5195-3p, hsa-miR-5196-5p, hsa-miR-548as-5p, hsa-miR-548as-3p, hsa-miR-5579-5p, hsa-miR-5579-3p, hsa-miR-5584-5p, hsa-miR-5698, hsa-miR-211-3p, hsa-miR-345-3p, hsa-miR-652-5p, hsa-miR-1185-2-3p, hsa-miR-1247-3p, hsa-miR-3184-3p, hsa-miR-1185-1-3p, hsa-miR-503-3p, hsa-miR-937-5p, hsa-miR-1227-5p, hsa-miR-1229-5p, hsa-miR-1237-5p, hsa-miR-1238-5p, hsa-miR-4750-3p, hsa-miR-5739, hsa-miR-5787, hsa-miR-6069, hsa-miR-6071, hsa-miR-6081, hsa-miR-6085, hsa-miR-6086, hsa-miR-6088, hsa-miR-6089, hsa-miR-6090, hsa-miR-6124, hsa-miR-6125, hsa-miR-6133, hsa-miR-6165, hsa-miR-6501-5p, hsa-miR-6503-3p, hsa-miR-6511a-5p, hsa-miR-6512-5p, hsa-miR-6515-3p, hsa-miR-6716-5p, hsa-miR-6511b-5p, hsa-miR-6719-3p, hsa-miR-6720-3p, hsa-miR-6721-5p, hsa-miR-6724-5p, hsa-miR-208a-5p, hsa-miR-210-5p, hsa-miR-128-1-5p, hsa-miR-152-5p, hsa-miR-328-5p, hsa-miR-489-5p, hsa-miR-511-3p, hsa-miR-181d-3p, hsa-miR-598-5p, hsa-miR-605-3p, hsa-miR-619-5p, hsa-miR-627-3p, hsa-miR-874-5p, hsa-miR-208b-5p, hsa-miR-1343-5p, hsa-miR-6727-5p, hsa-miR-6729-5p, hsa-miR-6729-3p, hsa-miR-6730-5p, hsa-miR-6731-3p, hsa-miR-6732-5p, hsa-miR-6734-5p, hsa-miR-6738-5p, hsa-miR-6742-5p, hsa-miR-6743-5p, hsa-miR-6745, hsa-miR-6746-5p, hsa-miR-6748-5p, hsa-miR-6749-5p, hsa-miR-6752-5p, hsa-miR-6752-3p, hsa-miR-6756-5p, hsa-miR-6756-3p, hsa-miR-6758-5p, hsa-miR-6759-3p, hsa-miR-6760-5p, hsa-miR-6760-3p, hsa-miR-6763-3p, hsa-miR-6765-5p, hsa-miR-6766-5p, hsa-miR-6766-3p, hsa-miR-6768-5p, hsa-miR-6769a-5p, hsa-miR-6771-5p, hsa-miR-6774-5p, hsa-miR-6775-5p, hsa-miR-6777-5p, hsa-miR-6777-3p, hsa-miR-6779-5p, hsa-miR-6780a-5p, hsa-miR-6781-5p, hsa-miR-6782-5p, hsa-miR-6784-3p, hsa-miR-6785-3p, hsa-miR-6786-5p, hsa-miR-6787-5p, hsa-miR-6789-5p, hsa-miR-6790-5p, hsa-miR-6791-5p, hsa-miR-6792-3p, hsa-miR-6793-3p, hsa-miR-6794-5p, hsa-miR-6795-5p, hsa-miR-6795-3p, hsa-miR-6796-3p, hsa-miR-6797-5p, hsa-miR-6797-3p, hsa-miR-6798-5p, hsa-miR-6798-3p, hsa-miR-6799-5p, hsa-miR-6800-5p, hsa-miR-6800-3p, hsa-miR-6801-3p, hsa-miR-6802-5p, hsa-miR-6802-3p, hsa-miR-6803-3p, hsa-miR-6805-5p, hsa-miR-6805-3p, hsa-miR-6807-5p, hsa-miR-6808-5p, hsa-miR-6808-3p, hsa-miR-6809-5p, hsa-miR-6809-3p, hsa-miR-6810-3p, hsa-miR-6812-5p, hsa-miR-6812-3p, hsa-miR-6813-3p, hsa-miR-6815-5p, hsa-miR-6816-5p, hsa-miR-6819-3p, hsa-miR-6821-5p, hsa-miR-6821-3p, hsa-miR-6824-5p, hsa-miR-6825-5p, hsa-miR-6829-5p, hsa-miR-6830-5p, hsa-miR-6831-5p, hsa-miR-6832-5p, hsa-miR-6833-5p, hsa-miR-6835-5p, hsa-miR-6780b-5p, hsa-miR-6836-3p, hsa-miR-6839-3p, hsa-miR-6840-5p, hsa-miR-6840-3p, hsa-miR-6845-3p, hsa-miR-6848-5p, hsa-miR-6848-3p, hsa-miR-6850-5p, hsa-miR-6855-3p, hsa-miR-6857-3p, hsa-miR-6858-3p, hsa-miR-6859-3p, hsa-miR-6769b-5p, hsa-miR-6861-3p, hsa-miR-6862-3p, hsa-miR-6865-5p, hsa-miR-6870-5p, hsa-miR-6870-3p, hsa-miR-6872-5p, hsa-miR-6880-5p, hsa-miR-6880-3p, hsa-miR-6881-5p, hsa-miR-6884-3p, hsa-miR-6885-3p, hsa-miR-6886-3p, hsa-miR-6887-5p, hsa-miR-6887-3p, hsa-miR-6889-5p, hsa-miR-6889-3p, hsa-miR-6891-5p, hsa-miR-6891-3p, hsa-miR-6892-3p, hsa-miR-6893-5p, hsa-miR-6895-5p, hsa-miR-7106-5p, hsa-miR-7107-5p, hsa-miR-7108-5p, hsa-miR-7108-3p, hsa-miR-7109-5p, hsa-miR-7110-3p, hsa-miR-7111-Sp, hsa-miR-7111-3p, hsa-miR-7113-3p, hsa-miR-7114-5p, hsa-miR-7114-3p, hsa-miR-7150, hsa-miR-7161-5p, hsa-miR-7704, hsa-miR-4433b-5p, hsa-miR-4433b-3p, hsa-miR-7845-5p, hsa-miR-7851-3p, hsa-miR-7852-3p, hsa-miR-8059, hsa-miR-8064, hsa-miR-8069, hsa-miR-8072, hsa-miR-8078, hsa-miR-8079, hsa-miR-128-2-5p, hsa-miR-7974, hsa-miR-1249-5p, hsa-miR-137-5p, hsa-miR-190b-3p, hsa-miR-9898, hsa-miR-9902, hsa-miR-10392-3p, hsa-miR-10394-3p, hsa-miR-10396a-5p, hsa-miR-10396a-3p, hsa-miR-10400-3p, hsa-miR-10401-5p, hsa-miR-10396b-5p, hsa-miR-10522-5p, hsa-miR-10526-3p, hsa-miR-11181-3p, hsa-miR-3059-3p, hsa-miR-3085-3p, hsa-miR-12116, hsa-miR-12118, hsa-miR-12119, hsa-miR-12120, hsa-miR-12128, and hsa-miR-12131. In the present disclosure, the extract of urine may be an extract of the urine of a stage-I melanoma patient. Only one of these microRNAs can be used to predict whether a subject from which the urine is derived is a melanoma patient (particularly stage-I), and 5 or more types, 10 or more types, 15 or more types, or 20 or more types of these microRNAs can be used in combination to predict whether a subject from which the urine is derived is a melanoma patient (particularly stage-I). Among these microRNAs, a microRNA that may be highly expressed in a melanoma patient (particularly stage-I) can be preferably used for prediction.

The present disclosure provides an extract of urine containing at least one type, two types, three types, four types, five types, six types, seven types, eight types, nine types, or ten types of or all microRNAs selected from the group consisting of microRNAs having an accuracy of higher than 0.500 among the microRNAs listed in Table 24-10. In the present disclosure, the extract of urine may be an extract of the urine of a melanoma patient. Only one of these microRNAs can be used to predict whether a subject from which the urine is derived is a melanoma patient, and 5 or more types, 10 or more types, 15 or more types, or 20 or more types of these microRNAs can be used in combination to predict whether a subject from which the urine is derived is a melanoma patient. Among these microRNAs, a microRNA that may be highly expressed in a melanoma patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

The present disclosure provides an extract of urine containing at least one type, two types, three types, four types, five types, six types, seven types, eight types, nine types, or ten types of or all microRNAs selected from the group consisting of microRNAs having an accuracy of higher than 0.500 among the microRNAs listed in Table 25-10. In the present disclosure, the extract of urine may be an extract of the urine of a melanoma patient. Only one of these microRNAs can be used to predict whether a subject from which the urine is derived is a melanoma patient, and 5 or more types, 10 or more types, 15 or more types, or 20 or more types of these microRNAs can be used in combination to predict whether a subject from which the urine is derived is a melanoma patient. Among these microRNAs, a microRNA that may be highly expressed in a melanoma patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

The present disclosure provides an extract of urine containing at least one type, two types, three types, four types, five types, six types, seven types, eight types, nine types, or ten types of or all microRNAs selected from the microRNAs listed in Table 4-11. The present disclosure also provides an extract of urine containing at least one type, two types, three types, four types, five types, six types, seven types, eight types, nine types, or ten types of or all microRNAs selected from the group consisting of hsa-miR-4512, hsa-miR-3978, hsa-miR-1912-3p, hsa-miR-1292-5p, hsa-miR-4691-3p, hsa-miR-8079, hsa-miR-8080, hsa-miR-4280, hsa-miR-539-5p, hsa-miR-150-3p, hsa-miR-106a-3p, hsa-miR-6838-5p, hsa-miR-6809-5p, hsa-miR-1286, hsa-miR-127-5p, hsa-miR-4321, hsa-miR-4521, hsa-miR-6500-5p, hsa-miR-4433a-3p, hsa-miR-320c, hsa-miR-4494, hsa-miR-8078, hsa-miR-571, hsa-miR-6761-5p, hsa-miR-598-5p, hsa-miR-3907, hsa-miR-4715-5p, hsa-miR-3692-3p, hsa-miR-6501-3p, hsa-miR-489-5p, hsa-miR-761, hsa-miR-6071, hsa-miR-5196-5p, hsa-miR-186-3p, hsa-miR-4439, hsa-miR-4786-3p, hsa-miR-7156-3p, hsa-miR-10522-5p, hsa-miR-3130-3p, hsa-miR-449c-5p, hsa-miR-4684-3p, hsa-miR-3129-3p, hsa-miR-3943, hsa-miR-5195-3p, hsa-miR-4433b-3p, hsa-miR-323a-3p, hsa-miR-145-5p, hsa-miR-940, hsa-miR-7161-5p, hsa-miR-7151-5p, hsa-miR-1269a, hsa-miR-1225-3p, hsa-miR-4454, hsa-miR-1225-5p, hsa-miR-3937, hsa-miR-6829-5p, hsa-miR-4289, hsa-miR-1185-2-3p, hsa-miR-1914-3p, hsa-miR-452-3p, hsa-miR-10396a-3p, hsa-miR-361-3p, hsa-miR-611, hsa-miR-6797-5p, hsa-miR-8059, hsa-miR-548g-3p, hsa-miR-6775-5p, hsa-miR-5681a, hsa-miR-6848-3p, hsa-miR-4743-3p, hsa-miR-4665-3p, hsa-miR-2681-3p, hsa-miR-3614-5p, hsa-miR-7706, hsa-miR-4284, hsa-miR-4750-3p, hsa-miR-552-5p, hsa-miR-6069, hsa-miR-554, hsa-miR-6795-3p, hsa-miR-3934-3p, hsa-miR-623, hsa-miR-3675-3p, hsa-miR-4755-3p, hsa-miR-382-5p, hsa-miR-375-3p, hsa-miR-625-3p, hsa-miR-4794, hsa-miR-3659, hsa-miR-665, hsa-miR-498-5p, hsa-miR-6808-3p, hsa-miR-10526-3p, hsa-miR-433-5p, hsa-miR-516a-5p, hsa-miR-4313, hsa-miR-4286, hsa-miR-6516-5p, hsa-miR-4271, hsa-miR-4731-3p, hsa-miR-3161, hsa-miR-4749-3p, hsa-miR-1185-1-3p, hsa-miR-6716-5p, hsa-miR-4661-5p, hsa-miR-25-3p, hsa-miR-6805-3p, hsa-miR-200a-5p, hsa-miR-4681, hsa-miR-4522, hsa-miR-7154-3p, hsa-miR-3155a, hsa-miR-3142, hsa-miR-4281, hsa-miR-2115-5p, hsa-miR-6876-3p, hsa-miR-6760-3p, hsa-miR-5702, hsa-miR-519b-3p, hsa-miR-6766-3p, hsa-miR-6752-3p, hsa-miR-4769-3p, hsa-miR-3660, hsa-miR-619-5p, hsa-miR-6798-3p, hsa-miR-6777-3p, hsa-miR-4433b-5p, hsa-miR-601, hsa-miR-6505-3p, hsa-miR-4465, hsa-miR-210-5p, hsa-miR-3666, hsa-miR-6763-3p, hsa-miR-630, hsa-miR-4701-3p, hsa-miR-6819-3p, hsa-miR-6859-3p, hsa-miR-541-3p, hsa-miR-370-5p, hsa-miR-7109-5p, hsa-miR-4675, hsa-miR-6794-5p, hsa-miR-4685-5p, hsa-miR-1285-3p, hsa-miR-4274, hsa-miR-4447, hsa-miR-6800-3p, hsa-miR-12113, hsa-miR-6812-5p, hsa-miR-6513-5p, hsa-miR-6813-3p, hsa-miR-4716-5p, hsa-miR-515-5p, hsa-miR-6892-3p, hsa-miR-6805-5p, hsa-miR-4793-5p, hsa-miR-7157-5p, hsa-miR-5693, hsa-miR-4433a-5p, hsa-miR-6503-3p, hsa-miR-5004-3p, hsa-miR-1913, hsa-miR-3934-5p, hsa-miR-1251-3p, hsa-miR-3162-3p, hsa-miR-6784-3p, hsa-miR-646, hsa-miR-3682-3p, hsa-miR-4451, hsa-miR-324-5p, hsa-miR-365a-3p, hsa-miR-365b-3p, hsa-miR-6845-3p, hsa-miR-663b, hsa-miR-216b-3p, hsa-miR-4708-5p, hsa-miR-4323, hsa-miR-6785-3p, hsa-miR-6802-5p, hsa-miR-378i, hsa-miR-551b-5p, hsa-miR-7114-3p, hsa-miR-371b-3p, hsa-miR-3616-3p, hsa-miR-3199, hsa-miR-4450, hsa-miR-27b-3p, hsa-miR-6836-3p, hsa-miR-3065-3p, hsa-miR-4738-3p, hsa-miR-1908-5p, hsa-miR-338-5p, hsa-miR-591, hsa-miR-339-3p, hsa-miR-9898, hsa-miR-296-5p, hsa-miR-642b-3p, hsa-miR-4294, hsa-miR-7974, hsa-miR-3074-5p, hsa-miR-4634, hsa-miR-3651, hsa-miR-6868-3p, hsa-miR-494-5p, hsa-miR-4725-3p, hsa-miR-380-3p, hsa-miR-365a-5p, hsa-miR-6880-3p, hsa-miR-12114, hsa-miR-4449, hsa-miR-1287-3p, hsa-miR-6756-5p, hsa-miR-206, hsa-miR-4713-3p, hsa-miR-5091, hsa-miR-6799-5p, hsa-miR-4665-5p, hsa-miR-4728-3p, hsa-miR-3085-3p, hsa-miR-6810-3p, hsa-miR-484, hsa-miR-6752-5p, hsa-miR-3679-3p, hsa-miR-4697-5p, hsa-miR-6780b-5p, hsa-miR-3922-3p, hsa-miR-580-3p, hsa-miR-4658, hsa-miR-1228-3p, hsa-miR-6779-5p, hsa-miR-4299, hsa-miR-12122, hsa-miR-6796-3p, hsa-miR-6081, hsa-miR-4687-3p, hsa-miR-632, hsa-miR-6808-5p, hsa-miR-34c-5p, hsa-miR-6749-5p, hsa-miR-1249-3p, hsa-miR-1224-3p, hsa-miR-6504-5p, hsa-miR-4441, hsa-miR-6790-5p, hsa-miR-374b-5p, hsa-miR-6729-3p, hsa-miR-4740-5p, hsa-miR-6124, hsa-miR-4316, hsa-miR-371b-5p, hsa-miR-4529-5p, hsa-miR-141-5p, hsa-miR-3064-5p, hsa-miR-6837-5p, hsa-miR-4687-5p, hsa-miR-6719-3p, hsa-miR-3677-3p, hsa-miR-3059-3p, hsa-miR-943, hsa-miR-2682-5p, hsa-miR-708-5p, hsa-miR-4327, hsa-miR-3133, hsa-miR-6070, hsa-miR-147b-3p, hsa-miR-4672, hsa-miR-1226-5p, hsa-miR-3187-3p, hsa-miR-4724-3p, hsa-miR-6821-5p, hsa-miR-5708, hsa-miR-3689d, hsa-miR-6821-3p, hsa-miR-622, hsa-miR-4783-3p, hsa-miR-297, hsa-miR-3617-3p, hsa-miR-6795-5p, hsa-miR-7851-3p, hsa-miR-3173-5p, hsa-miR-9986, hsa-miR-4708-3p, hsa-miR-6501-5p, hsa-miR-6855-3p, hsa-miR-6767-5p, hsa-miR-4682, hsa-miR-4463, hsa-miR-3945, hsa-miR-4314, hsa-miR-1238-3p, hsa-miR-663a, hsa-miR-2116-3p, hsa-miR-4706, hsa-miR-4649-5p, hsa-miR-718, hsa-miR-4455, hsa-miR-1296-3p, hsa-miR-330-3p, hsa-miR-1538, hsa-let-7e-5p, hsa-miR-2355-5p, hsa-miR-612, hsa-miR-154-5p, hsa-miR-513c-5p, hsa-miR-6797-3p, hsa-miR-4489, hsa-miR-4780, hsa-miR-874-5p, hsa-miR-3927-5p, hsa-miR-3654, hsa-miR-7150, hsa-miR-328-5p, hsa-miR-4257, hsa-miR-6887-3p, hsa-miR-6738-3p, hsa-miR-3646, hsa-miR-6762-3p, hsa-miR-6840-5p, hsa-miR-4295, hsa-miR-4758-3p, hsa-miR-12128, hsa-miR-557, hsa-miR-4520-5p, hsa-miR-383-3p, hsa-miR-378g, hsa-miR-5187-3p, hsa-miR-6088, hsa-miR-4707-3p, hsa-miR-1267, hsa-miR-4288, hsa-miR-6852-3p, hsa-miR-128-2-5p, hsa-miR-887-3p, hsa-miR-660-3p, hsa-miR-6803-5p, hsa-miR-5000-5p, hsa-miR-18b-3p, hsa-miR-4640-3p, hsa-miR-5006-5p, hsa-miR-4466, hsa-miR-1207-5p, hsa-miR-670-5p, hsa-miR-6768-5p, hsa-miR-4535, hsa-miR-4785, hsa-miR-6765-5p, hsa-miR-1843, hsa-miR-6889-3p, hsa-miR-2467-3p, hsa-miR-6869-3p, hsa-miR-15a-3p, hsa-miR-514b-5p, hsa-miR-7160-5p, hsa-miR-4718, hsa-miR-3180, hsa-miR-3622a-5p, hsa-miR-8064, hsa-miR-1237-5p, hsa-miR-4800-3p, hsa-miR-4524b-5p, hsa-miR-4712-5p, hsa-miR-4673, hsa-miR-1343-5p, hsa-miR-6870-5p, hsa-miR-647, hsa-miR-877-5p, hsa-miR-1273h-3p, hsa-miR-4537, hsa-miR-6793-5p, hsa-miR-6502-5p, hsa-miR-6792-5p, hsa-miR-184, hsa-miR-3190-3p, hsa-miR-3152-5p, hsa-miR-6768-3p, hsa-miR-12116, hsa-miR-3690, hsa-miR-4753-5p, hsa-miR-422a, hsa-miR-4293, hsa-miR-181d-3p, hsa-miR-4258, hsa-miR-5696, hsa-miR-6764-3p, hsa-miR-6740-5p, hsa-miR-6774-3p, hsa-miR-431-3p, hsa-miR-1322, hsa-miR-10396a-5p, hsa-miR-31-3p, hsa-miR-4487, hsa-miR-214-3p, hsa-miR-6515-3p, hsa-miR-675-5p, hsa-miR-5681b, hsa-miR-5010-5p, hsa-miR-7108-3p, hsa-miR-1202, hsa-miR-92a-2-5p, hsa-miR-4448, hsa-miR-6504-3p, hsa-miR-589-5p, hsa-miR-4769-5p, hsa-miR-6895-5p, hsa-miR-6854-3p, hsa-miR-7158-3p, hsa-miR-6816-5p, hsa-miR-4664-3p, hsa-miR-5002-5p, hsa-miR-125b-2-3p, hsa-miR-4481, hsa-miR-5587-3p, hsa-miR-4768-3p, hsa-miR-6715a-3p, hsa-miR-26b-3p, hsa-miR-1229-5p, hsa-miR-3157-3p, hsa-miR-1469, hsa-miR-1227-5p, hsa-miR-6843-3p, hsa-miR-3132, hsa-miR-6499-3p, hsa-miR-6129, hsa-miR-196b-3p, hsa-miR-1298-3p, hsa-miR-128-3p, hsa-miR-7152-5p, hsa-miR-1268b, hsa-miR-7855-5p, hsa-miR-3124-5p, hsa-miR-149-3p, hsa-miR-652-3p, hsa-miR-3074-3p, hsa-miR-4776-3p, hsa-miR-21-3p, hsa-miR-7978, hsa-miR-766-5p, hsa-miR-3619-5p, hsa-miR-4676-3p, hsa-miR-4482-3p, hsa-miR-3917, hsa-miR-3648, hsa-miR-23a-3p, hsa-miR-4648, hsa-miR-6789-5p, hsa-miR-6724-5p, hsa-miR-1972, hsa-miR-371a-5p, hsa-miR-338-3p, hsa-miR-4322, hsa-miR-6509-5p, hsa-miR-6885-5p, hsa-miR-221-3p, hsa-miR-103a-1-5p, hsa-miR-6722-5p, hsa-miR-6835-5p, hsa-miR-1247-3p, hsa-miR-4253, hsa-miR-4725-5p, hsa-miR-4768-5p, hsa-miR-487a-5p, hsa-miR-642a-3p, hsa-miR-1288-3p, hsa-miR-99b-3p, hsa-miR-6787-5p, hsa-miR-6753-5p, hsa-miR-6788-5p, hsa-miR-5589-5p, hsa-miR-6848-5p, hsa-miR-9902, hsa-miR-4311, hsa-miR-6512-3p, hsa-miR-605-3p, hsa-miR-3196, hsa-miR-5685, hsa-miR-3619-3p, hsa-miR-6731-3p, hsa-miR-6873-5p, hsa-miR-4260, hsa-miR-493-3p, hsa-miR-509-5p, hsa-miR-6895-3p, hsa-miR-6858-3p, hsa-miR-219b-5p, hsa-miR-5007-5p, hsa-miR-892a, hsa-miR-6165, hsa-miR-4804-5p, hsa-miR-575, hsa-miR-4717-3p, hsa-miR-542-5p, hsa-miR-5189-3p, hsa-miR-658, hsa-miR-6773-3p, hsa-miR-4652-5p, hsa-miR-4326, hsa-miR-6857-3p, hsa-miR-3141, hsa-miR-668-3p, hsa-miR-513b-5p, hsa-miR-8072, hsa-miR-6732-5p, hsa-miR-4645-3p, hsa-miR-3944-5p, hsa-miR-8069, hsa-miR-5699-5p, hsa-miR-3138, hsa-miR-937-5p, hsa-miR-485-5p, hsa-miR-6887-5p, hsa-miR-604, hsa-miR-1237-3p, hsa-miR-5705, hsa-miR-6083, hsa-miR-6086, hsa-miR-6772-5p, hsa-miR-6791-3p, hsa-miR-4750-5p, hsa-miR-6782-5p, hsa-miR-4700-5p, hsa-miR-4428, hsa-miR-4297, hsa-miR-6834-5p, hsa-miR-654-3p, hsa-miR-1204, hsa-miR-4298, hsa-miR-4638-5p, hsa-miR-146a-5p, hsa-miR-504-3p, hsa-let-7d-3p, hsa-miR-6817-5p, hsa-miR-135a-5p, hsa-miR-11400, hsa-miR-8062, hsa-miR-1234-3p, hsa-miR-2681-5p, hsa-miR-6772-3p, hsa-miR-4773, hsa-miR-6513-3p, hsa-miR-1182, hsa-miR-6742-5p, hsa-miR-519a-3p, hsa-miR-3622a-3p, hsa-miR-892b, hsa-miR-659-3p, hsa-miR-5187-5p, hsa-miR-4498, hsa-miR-4799-5p, hsa-miR-3652, hsa-miR-6766-5p, hsa-miR-6886-5p, hsa-miR-7111-5p, hsa-miR-6865-5p, hsa-miR-6806-5p, hsa-miR-6802-3p, hsa-miR-4731-5p, hsa-miR-3922-5p, hsa-miR-6718-5p, hsa-miR-3195, hsa-miR-6728-5p, hsa-miR-885-3p, hsa-miR-4733-5p, hsa-miR-1321, hsa-miR-5001-5p, hsa-miR-5003-3p, hsa-miR-5002-3p, hsa-miR-1226-3p, hsa-miR-3130-5p, hsa-miR-1301-5p, hsa-miR-9500, hsa-miR-6774-5p, hsa-miR-6750-5p, hsa-miR-512-5p, hsa-miR-8074, hsa-miR-4709-5p, hsa-miR-5694, hsa-miR-11401, hsa-miR-6810-5p, hsa-miR-6839-3p, hsa-miR-6867-5p, hsa-miR-5093, hsa-miR-6861-3p, hsa-miR-6130, hsa-miR-551b-3p, hsa-miR-1273h-5p, hsa-miR-3908, hsa-miR-4436a, hsa-miR-6826-5p, hsa-miR-574-5p, hsa-miR-6742-3p, hsa-miR-4435, hsa-miR-4723-3p, hsa-miR-1184, hsa-miR-3685, hsa-miR-365b-5p, hsa-miR-12120, hsa-miR-642b-5p, hsa-miR-6871-5p, hsa-miR-8485, hsa-miR-4502, hsa-miR-6756-3p, hsa-miR-23a-5p, hsa-miR-4285, hsa-miR-421, hsa-miR-4635, hsa-miR-4255, hsa-miR-499b-3p, hsa-miR-8055, hsa-miR-6852-5p, hsa-miR-6126, hsa-miR-6754-3p, hsa-miR-6514-3p, hsa-miR-12119, hsa-miR-1976, hsa-miR-6856-5p, hsa-miR-6770-5p, hsa-miR-211-3p, hsa-miR-1250-3p, hsa-miR-2467-5p, hsa-miR-585-5p, hsa-miR-432-5p, hsa-miR-6769b-5p, hsa-miR-5189-5p, hsa-miR-3151-5p, hsa-miR-885-5p, hsa-miR-6759-5p, hsa-miR-4485-5p, hsa-miR-6729-5p, hsa-miR-4526, hsa-miR-8073, hsa-miR-6798-5p, hsa-miR-3663-3p, hsa-miR-6743-5p, hsa-miR-4529-3p, hsa-miR-6793-3p, hsa-miR-4653-5p, hsa-miR-6893-3p, hsa-miR-143-3p, hsa-miR-6809-3p, hsa-miR-103a-2-5p, hsa-miR-6885-3p, hsa-miR-208a-5p, hsa-miR-942-5p, hsa-miR-7112-5p, hsa-miR-7975, hsa-miR-6720-5p, hsa-miR-320b, hsa-miR-514b-3p, hsa-miR-6722-3p, hsa-miR-4698, hsa-miR-6849-3p, hsa-miR-579-5p, hsa-miR-3194-5p, hsa-miR-1281, hsa-miR-6072, hsa-miR-6822-5p, hsa-miR-4697-3p, hsa-miR-668-5p, hsa-miR-595, hsa-miR-7109-3p, hsa-miR-4440, hsa-miR-3153, hsa-miR-4689, hsa-miR-4748, hsa-miR-6787-3p, hsa-miR-369-5p, hsa-miR-3162-5p, hsa-miR-216a-3p, hsa-miR-7113-3p, hsa-miR-6738-5p, hsa-miR-181a-2-3p, hsa-miR-3127-3p, hsa-miR-6737-5p, hsa-miR-4664-5p, hsa-miR-1266-5p, hsa-miR-6511b-5p, hsa-miR-6833-3p, hsa-miR-483-5p, hsa-miR-6842-3p, hsa-miR-8087, hsa-miR-3158-3p, hsa-miR-5088-5p, hsa-miR-10398-5p, hsa-miR-8060, hsa-miR-550a-3-5p, hsa-miR-652-5p, hsa-miR-6771-5p, hsa-miR-1910-5p, hsa-miR-1343-3p, hsa-miR-3163, hsa-miR-548q, hsa-miR-6131, hsa-miR-503-3p, hsa-miR-6801-3p, hsa-miR-1253, hsa-miR-3972, hsa-miR-3918, hsa-miR-8071, hsa-miR-3911, hsa-miR-6883-3p, hsa-miR-4640-5p, hsa-miR-3714, hsa-miR-6840-3p, hsa-miR-3155b, hsa-miR-6085, hsa-miR-5195-5p, hsa-miR-1193, hsa-miR-224-3p, hsa-miR-4639-3p, hsa-miR-4460, hsa-miR-938, hsa-miR-1307-3p, hsa-miR-921, hsa-miR-301a-5p, hsa-miR-3691-5p, hsa-miR-4727-5p, hsa-miR-4473, hsa-miR-7702, hsa-miR-10396b-5p, hsa-miR-10396b-3p, hsa-miR-671-5p, hsa-miR-769-5p, hsa-miR-6858-5p, hsa-miR-4497, hsa-miR-5698, hsa-miR-589-3p, hsa-miR-6776-5p, hsa-miR-3170, hsa-miR-4328, hsa-miR-6824-5p, hsa-miR-4462, hsa-miR-671-3p, hsa-miR-7850-5p, hsa-miR-3192-3p, hsa-miR-4763-5p, hsa-miR-196a-1-3p, hsa-miR-7977, hsa-miR-3612, hsa-miR-3144-3p, hsa-miR-550a-5p, hsa-miR-1249-5p, hsa-miR-5787, hsa-miR-187-5p, hsa-miR-550b-3p, hsa-miR-4793-3p, hsa-miR-6792-3p, hsa-miR-371a-3p, hsa-miR-6877-3p, hsa-miR-6849-5p, hsa-miR-373-5p, hsa-miR-4749-5p, hsa-miR-3160-5p, hsa-miR-10394-3p, hsa-miR-1825, hsa-miR-374c-3p, hsa-miR-139-3p, hsa-miR-4436b-3p, hsa-miR-346, hsa-miR-3175, hsa-miR-6891-3p, hsa-miR-4802-3p, hsa-miR-5584-3p, hsa-miR-1285-5p, hsa-miR-6820-3p, hsa-miR-4324, hsa-miR-6780a-5p, hsa-miR-613, hsa-miR-378a-3p, hsa-miR-6786-5p, hsa-miR-4269, hsa-miR-6880-5p, hsa-miR-25-5p, hsa-miR-6862-3p, hsa-miR-4278, hsa-miR-6886-3p, hsa-miR-3059-5p, hsa-miR-6860, hsa-miR-6870-3p, hsa-miR-4270, and hsa-miR-3187-5p. In the present disclosure, the extract of urine may be an extract of the urine of an ovarian cancer patient. Only one of these microRNAs can be used to predict whether a subject from which the urine is derived is an ovarian cancer patient, and 5 or more types, 10 or more types, 15 or more types, or 20 or more types of these microRNAs can be used in combination to predict whether a subject from which the urine is derived is an ovarian cancer patient. Among these microRNAs, a microRNA that may be highly expressed in an ovarian cancer patient can be preferably used for prediction.

The present disclosure provides an extract of urine containing at least one type, two types, three types, four types, five types, six types, seven types, eight types, nine types, or ten types of or all microRNAs selected from the group consisting of miR-25-3p, miR-129-5p, miR-221-3p, miR-27b-3p, miR-143-3p, miR-146a-5p, miR-154-5p, miR-106b-5p, miR-130b-3p, miR-371a-3p, miR-375-3p, miR-378a-5p, miR-380-3p, miR-382-5p, miR-330-3p, miR-422a, miR-431-5p, miR-432-5p, miR-519b-3p, miR-525-3p, miR-519a-3p, miR-539-5p, miR-571, miR-580-3p, miR-584-5p, miR-589-3p, miR-591, miR-608, miR-611, miR-613, miR-622, miR-623, miR-639, miR-645, miR-650, miR-421, miR-542-5p, miR-15a-3p, miR-22-5p, miR-25-5p, miR-27a-5p, miR-181a-2-3p, miR-181c-3p, miR-125b-1-3p, miR-141-5p, miR-127-5p, miR-186-3p, miR-339-3p, miR-146b-3p, miR-501-3p, miR-509-5p, miR-654-3p, miR-892a, miR-541-5p, miR-665, miR-943, miR-1226-5p, miR-513c-5p, miR-320c, miR-1286, miR-1291, miR-1250-5p, miR-1253, miR-1255a, miR-1269a, miR-1292-5p, miR-1911-3p, miR-1912-3p, miR-1914-3p, miR-103a-2-5p, miR-224-3p, miR-196b-3p, miR-1973, miR-449c-5p, miR-761, miR-2115-3p, miR-2681-5p, miR-2681-3p, miR-3130-3p, miR-548v, miR-3155a, miR-3170, miR-3187-3p, miR-3200-3p, miR-3065-3p, miR-4293, miR-4321, miR-4280, miR-4285, miR-4289, miR-3651, miR-3659, miR-3666, miR-3677-3p, miR-3688-3p, miR-3689a-3p, miR-3690, miR-3692-3p, miR-3713, miR-3907, miR-3923, miR-3934-5p, miR-3936, miR-4433a-3p, miR-4436a, miR-4439, miR-4444, miR-4449, miR-548ah-5p, miR-4460, miR-4465, miR-4473, miR-3689d, miR-3155b, miR-548al, miR-4494, miR-4502, miR-4512, miR-4521, miR-378i, miR-4537, miR-4538, miR-3129-3p, miR-3157-3p, miR-3922-5p, miR-3944-5p, miR-3978, miR-4634, miR-4637, miR-4648, miR-4653-5p, miR-4658, miR-219b-5p, miR-4682, miR-4684-3p, miR-4691-5p, miR-4691-3p, miR-4701-3p, miR-4708-5p, miR-4708-3p, miR-4711-3p, miR-4712-5p, miR-4713-3p, miR-4715-5p, miR-4724-3p, miR-4733-5p, miR-4761-5p, miR-4768-3p, miR-4772-3p, miR-4778-5p, miR-4781-5p, miR-4793-5p, miR-4797-5p, miR-4799-5p, miR-4802-3p, miR-5002-5p, miR-5006-5p, miR-5091, miR-5187-3p, miR-5571-3p, miR-5579-5p, miR-5579-3p, miR-5587-3p, miR-5685, miR-5681b, miR-5693, miR-5694, miR-660-3p, miR-1285-5p, miR-3190-3p, miR-216a-3p, miR-3927-5p, miR-6068, miR-6071, miR-6081, miR-6083, miR-6500-5p, miR-6501-5p, miR-6501-3p, miR-6502-5p, miR-6503-3p, miR-6504-5p, miR-6504-3p, miR-6505-3p, miR-6510-3p, miR-6512-5p, miR-6715a-3p, miR-6719-3p, let-7c-3p, miR-383-3p, miR-433-5p, miR-487a-5p, miR-489-5p, miR-494-5p, miR-552-5p, miR-598-5p, miR-627-3p, miR-1301-5p, miR-1298-3p, miR-1251-3p, miR-6739-3p, miR-6761-5p, miR-6764-3p, miR-6768-3p, miR-6788-5p, miR-6791-3p, miR-6795-5p, miR-6808-3p, miR-6809-5p, miR-6821-3p, miR-6838-5p, miR-6839-3p, miR-6854-3p, miR-6856-3p, miR-6876-5p, miR-6895-5p, miR-7113-5p, miR-7151-5p, miR-7155-3p, miR-7156-3p, miR-7157-5p, miR-7158-3p, miR-7161-5p, miR-7706, miR-4433b-3p, miR-7850-5p, miR-7851-3p, miR-8064, miR-8078, miR-8079, miR-8080, miR-7974, miR-103a-1-5p, miR-137-5p, miR-9902, miR-9986, miR-10396a-3p, miR-10522-5p, miR-10526-3p, miR-3059-3p, miR-6529-5p, miR-12122, and miR-12131. In the present disclosure, the extract of urine may be an extract of the urine of a stage-I or -II ovarian cancer patient. Only one of these microRNAs can be used to predict whether a subject from which the urine is derived is an ovarian cancer patient (particularly stage-I or -II), and 5 or more types, 10 or more types, 15 or more types, or 20 or more types of these microRNAs can be used in combination to predict whether a subject from which the urine is derived is an ovarian cancer patient (particularly stage-I or -II). Among these microRNAs, a microRNA that may be highly expressed in an ovarian cancer patient (particularly stage-I or -II) can be preferably used for prediction.

The present disclosure provides an extract of urine containing at least one type, two types, three types, four types, five types, six types, seven types, eight types, nine types, or ten types of or all microRNAs selected from the group consisting of hsa-miR-93-5p, hsa-miR-210-3p, hsa-miR-221-3p, hsa-miR-27b-3p, hsa-miR-143-3p, hsa-miR-34b-5p, hsa-miR-130b-3p, hsa-miR-371a-3p, hsa-miR-380-3p, hsa-miR-382-5p, hsa-miR-383-5p, hsa-miR-330-3p, hsa-miR-369-5p, hsa-miR-431-5p, hsa-miR-452-3p, hsa-miR-485-5p, hsa-miR-519b-3p, hsa-miR-525-3p, hsa-miR-519a-3p, hsa-miR-501-5p, hsa-miR-493-3p, hsa-miR-539-5p, hsa-miR-571, hsa-miR-589-3p, hsa-miR-591, hsa-miR-593-5p, hsa-miR-609, hsa-miR-611, hsa-miR-613, hsa-miR-623, hsa-miR-631, hsa-miR-639, hsa-miR-650, hsa-miR-421, hsa-miR-542-5p, hsa-miR-363-5p, hsa-miR-769-3p, hsa-miR-15a-3p, hsa-miR-22-5p, hsa-miR-25-5p, hsa-miR-29b-1-5p, hsa-miR-181c-3p, hsa-miR-30b-3p, hsa-miR-124-5p, hsa-miR-125b-1-3p, hsa-miR-135a-3p, hsa-miR-127-5p, hsa-miR-186-3p, hsa-miR-339-3p, hsa-miR-509-5p, hsa-miR-593-3p, hsa-miR-654-3p, hsa-miR-892a, hsa-miR-665, hsa-miR-942-5p, hsa-miR-1225-5p, hsa-miR-1226-5p, hsa-miR-320c, hsa-miR-1286, hsa-miR-1303, hsa-miR-1250-5p, hsa-miR-1253, hsa-miR-1255a, hsa-miR-1269a, hsa-miR-1292-5p, hsa-miR-1912-3p, hsa-miR-1914-3p, hsa-miR-103a-2-5p, hsa-miR-196b-3p, hsa-miR-1972, hsa-miR-449c-5p, hsa-miR-761, hsa-miR-2681-5p, hsa-miR-2681-3p, hsa-miR-3130-3p, hsa-miR-3144-3p, hsa-miR-548v, hsa-miR-3074-3p, hsa-miR-3155a, hsa-miR-3170, hsa-miR-3186-5p, hsa-miR-3187-3p, hsa-miR-4296, hsa-miR-4293, hsa-miR-4299, hsa-miR-4321, hsa-miR-4280, hsa-miR-4330, hsa-miR-3619-5p, hsa-miR-3651, hsa-miR-3659, hsa-miR-3661, hsa-miR-3666, hsa-miR-3677-3p, hsa-miR-3688-3p, hsa-miR-3690, hsa-miR-3692-3p, hsa-miR-3907, hsa-miR-3908, hsa-miR-3923, hsa-miR-3929, hsa-miR-3934-5p, hsa-miR-4433a-3p, hsa-miR-4435, hsa-miR-4439, hsa-miR-548ah-5p, hsa-miR-4460, hsa-miR-4465, hsa-miR-548al, hsa-miR-4494, hsa-miR-4502, hsa-miR-4512, hsa-miR-4521, hsa-miR-4522, hsa-miR-4529-3p, hsa-miR-378i, hsa-miR-4537, hsa-miR-3129-3p, hsa-miR-3157-3p, hsa-miR-3944-5p, hsa-miR-3978, hsa-miR-4634, hsa-miR-4637, hsa-miR-4648, hsa-miR-4653-5p, hsa-miR-4658, hsa-miR-4673, hsa-miR-4676-3p, hsa-miR-4684-3p, hsa-miR-4691-3p, hsa-miR-4713-3p, hsa-miR-4715-5p, hsa-miR-4733-5p, hsa-miR-4740-5p, hsa-miR-4761-5p, hsa-miR-4761-3p, hsa-miR-4768-3p, hsa-miR-4772-3p, hsa-miR-4778-5p, hsa-miR-4781-5p, hsa-miR-4786-3p, hsa-miR-4793-5p, hsa-miR-4794, hsa-miR-4797-5p, hsa-miR-4802-3p, hsa-miR-550a-3-5p, hsa-miR-5000-5p, hsa-miR-5002-5p, hsa-miR-5003-3p, hsa-miR-5004-3p, hsa-miR-5006-5p, hsa-miR-5091, hsa-miR-5190, hsa-miR-5196-5p, hsa-miR-5579-5p, hsa-miR-5579-3p, hsa-miR-5580-5p, hsa-miR-5681a, hsa-miR-5693, hsa-miR-5694, hsa-miR-5702, hsa-miR-1285-5p, hsa-miR-216a-3p, hsa-miR-4743-3p, hsa-miR-6068, hsa-miR-6071, hsa-miR-6081, hsa-miR-6083, hsa-miR-6499-5p, hsa-miR-6500-5p, hsa-miR-6501-5p, hsa-miR-6501-3p, hsa-miR-6502-5p, hsa-miR-6503-3p, hsa-miR-6504-5p, hsa-miR-6505-3p, hsa-miR-6512-5p, hsa-miR-6513-3p, hsa-miR-6715a-3p, hsa-miR-6716-5p, hsa-miR-6719-3p, hsa-miR-433-5p, hsa-miR-489-5p, hsa-miR-181d-3p, hsa-miR-598-5p, hsa-miR-627-3p, hsa-miR-668-5p, hsa-miR-1251-3p, hsa-miR-6761-5p, hsa-miR-6764-5p, hsa-miR-6768-3p, hsa-miR-6775-5p, hsa-miR-6791-3p, hsa-miR-6794-5p, hsa-miR-6795-5p, hsa-miR-6809-5p, hsa-miR-6829-5p, hsa-miR-6837-3p, hsa-miR-6838-5p, hsa-miR-6839-3p, hsa-miR-6845-3p, hsa-miR-6856-3p, hsa-miR-6888-5p, hsa-miR-6895-5p, hsa-miR-7112-3p, hsa-miR-7151-5p, hsa-miR-7156-3p, hsa-miR-7158-5p, hsa-miR-7158-3p, hsa-miR-7161-5p, hsa-miR-7706, hsa-miR-4433b-3p, hsa-miR-7850-5p, hsa-miR-7851-3p, hsa-miR-8064, hsa-miR-8078, hsa-miR-8079, hsa-miR-8080, hsa-miR-7974, hsa-miR-137-5p, hsa-miR-9986, hsa-miR-10394-5p, hsa-miR-10396a-3p, hsa-miR-10522-5p, hsa-miR-10526-3p, hsa-miR-3059-3p, hsa-miR-12122, and hsa-miR-12131. In the present disclosure, the extract of urine may be an extract of the urine of a stage-I ovarian cancer patient. Only one of these microRNAs can be used to predict whether a subject from which the urine is derived is an ovarian cancer patient (particularly stage-I), and 5 or more types, 10 or more types, 15 or more types, or 20 or more types of these microRNAs can be used in combination to predict whether a subject from which the urine is derived is an ovarian cancer patient (particularly stage-I). Among these microRNAs, a microRNA that may be highly expressed in an ovarian cancer patient (particularly stage-I) can be preferably used for prediction.

The present disclosure provides an extract of urine containing at least one type, two types, three types, four types, five types, six types, seven types, eight types, nine types, or ten types of or all microRNAs selected from the group consisting of microRNAs having an accuracy of higher than 0.500 among the microRNAs listed in Table 24-11. In the present disclosure, the extract of urine may be an extract of the urine of an ovarian cancer patient. Only one of these microRNAs can be used to predict whether a subject from which the urine is derived is an ovarian cancer patient, and 5 or more types, 10 or more types, 15 or more types, or 20 or more types of these microRNAs can be used in combination to predict whether a subject from which the urine is derived is an ovarian cancer patient. Among these microRNAs, a microRNA that may be highly expressed in an ovarian cancer patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

The present disclosure provides an extract of urine containing at least one type, two types, three types, four types, five types, six types, seven types, eight types, nine types, or ten types of or all microRNAs selected from the group consisting of microRNAs having an accuracy of higher than 0.500 among the microRNAs listed in Table 25-11. In the present disclosure, the extract of urine may be an extract of the urine of an ovarian cancer patient. Only one of these microRNAs can be used to predict whether a subject from which the urine is derived is an ovarian cancer patient, and 5 or more types, 10 or more types, 15 or more types, or 20 or more types of these microRNAs can be used in combination to predict whether a subject from which the urine is derived is an ovarian cancer patient. Among these microRNAs, a microRNA that may be highly expressed in an ovarian cancer patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

The present disclosure provides an extract of urine containing at least one type, two types, three types, four types, five types, six types, seven types, eight types, nine types, or ten types of or all microRNAs selected from the microRNAs listed in Table 4-12. The present disclosure also provides an extract of urine containing at least one type, two types, three types, four types, five types, six types, seven types, eight types, nine types, or ten types of or all microRNAs selected from the group consisting of hsa-miR-1292-5p, hsa-miR-4521, hsa-miR-4512, hsa-miR-8080, hsa-miR-6071, hsa-miR-4691-3p, hsa-miR-127-5p, hsa-miR-3133, hsa-miR-1914-3p, hsa-miR-6838-5p, hsa-miR-4755-3p, hsa-miR-10526-3p, hsa-miR-551b-5p, hsa-miR-8078, hsa-miR-181d-3p, hsa-miR-489-5p, hsa-miR-4321, hsa-miR-4280, hsa-miR-605-3p, hsa-miR-297, hsa-miR-1269a, hsa-miR-3155a, hsa-miR-498-5p, hsa-miR-6761-5p, hsa-miR-3059-3p, hsa-miR-8079, hsa-miR-12131, hsa-miR-6750-5p, hsa-miR-4433b-3p, hsa-miR-3907, hsa-miR-3130-3p, hsa-miR-761, hsa-miR-3978, hsa-miR-1207-5p, hsa-miR-449c-5p, hsa-miR-6513-5p, hsa-miR-4800-3p, hsa-miR-519b-3p, hsa-miR-4785, hsa-miR-1225-5p, hsa-miR-4293, hsa-miR-6789-5p, hsa-miR-4289, hsa-miR-525-3p, hsa-miR-3199, hsa-miR-6809-5p, hsa-miR-4433a-3p, hsa-miR-6081, hsa-miR-492, hsa-miR-424-3p, hsa-miR-3129-3p, hsa-miR-623, hsa-miR-4786-3p, hsa-miR-542-5p, hsa-miR-421, hsa-miR-5196-5p, hsa-miR-1185-2-3p, hsa-miR-324-5p, hsa-miR-6758-5p, hsa-miR-4793-5p, hsa-miR-2681-3p, hsa-miR-1185-1-3p, hsa-miR-10392-3p, hsa-miR-7706, hsa-miR-7161-5p, hsa-miR-3934-3p, hsa-miR-145-5p, hsa-miR-4658, hsa-miR-4466, hsa-miR-4716-3p, hsa-miR-184, hsa-miR-4271, hsa-miR-1343-5p, hsa-miR-6836-3p, hsa-miR-4465, hsa-miR-4778-5p, hsa-miR-6752-5p, hsa-miR-6125, hsa-miR-6805-5p, hsa-miR-6783-5p, hsa-miR-6850-5p, hsa-miR-1912-3p, hsa-miR-4665-5p, hsa-miR-7156-3p, hsa-miR-1908-5p, hsa-miR-4634, hsa-miR-6795-5p, hsa-miR-6798-5p, hsa-miR-6845-3p, hsa-miR-525-5p, hsa-miR-541-3p, hsa-miR-4525, hsa-miR-4684-3p, hsa-miR-708-5p, hsa-miR-3937, hsa-miR-1286, hsa-miR-598-5p, hsa-miR-6784-3p, hsa-miR-4645-3p, hsa-miR-6743-5p, hsa-miR-4431, hsa-miR-6794-5p, hsa-miR-4728-5p, hsa-miR-611, hsa-miR-6791-5p, hsa-miR-509-5p, hsa-miR-4494, hsa-miR-4479, hsa-miR-6501-5p, hsa-miR-6760-3p, hsa-miR-4295, hsa-miR-4716-5p, hsa-miR-147b-3p, hsa-miR-6501-3p, hsa-miR-1303, hsa-miR-4472, hsa-miR-3124-5p, hsa-miR-3162-3p, hsa-miR-3178, hsa-miR-6887-5p, hsa-miR-6721-5p, hsa-miR-6770-3p, hsa-miR-6729-5p, hsa-miR-6880-3p, hsa-miR-638, hsa-miR-7114-3p, hsa-miR-10400-3p, hsa-miR-12113, hsa-miR-3677-3p, hsa-miR-4458, hsa-miR-10396a-3p, hsa-miR-627-5p, hsa-miR-1226-5p, hsa-miR-3161, hsa-miR-601, hsa-miR-6836-5p, hsa-miR-4327, hsa-miR-6746-5p, hsa-miR-12119, hsa-miR-3187-3p, hsa-miR-202-3p, hsa-miR-3196, hsa-miR-3651, hsa-miR-4745-3p, hsa-miR-196b-3p, hsa-miR-1469, hsa-miR-382-5p, hsa-miR-30b-3p, hsa-miR-1225-3p, hsa-miR-3180-3p, hsa-miR-711, hsa-miR-6829-5p, hsa-miR-4745-5p, hsa-miR-6798-3p, hsa-miR-4674, hsa-miR-6850-3p, hsa-miR-516a-5p, hsa-miR-8072, hsa-miR-6821-5p, hsa-miR-6768-3p, hsa-miR-4492, hsa-miR-186-3p, hsa-miR-1268b, hsa-miR-6129, hsa-miR-4433b-5p, hsa-miR-510-5p, hsa-miR-892a, hsa-miR-6832-5p, hsa-miR-6808-3p, hsa-miR-6816-5p, hsa-miR-10396a-5p, hsa-miR-1915-3p, hsa-miR-4534, hsa-miR-4303, hsa-miR-5002-5p, hsa-miR-8088, hsa-miR-6795-3p, hsa-miR-150-3p, hsa-miR-4286, hsa-miR-7854-3p, hsa-miR-1321, hsa-miR-4450, hsa-miR-4299, hsa-miR-3180, hsa-miR-6781-5p, hsa-miR-7856-5p, hsa-miR-6785-3p, hsa-miR-6810-3p, hsa-miR-5004-3p, hsa-miR-106a-3p, hsa-miR-6895-5p, hsa-miR-4703-3p, hsa-miR-4775, hsa-miR-6766-3p, hsa-miR-4451, hsa-miR-6124, hsa-miR-625-3p, hsa-miR-4761-3p, hsa-miR-4749-3p, hsa-miR-1298-3p, hsa-miR-10396b-5p, hsa-miR-6800-3p, hsa-miR-383-5p, hsa-miR-7111-Sp, hsa-miR-25-5p, hsa-miR-6763-3p, hsa-miR-4311, hsa-miR-3675-3p, hsa-miR-4261, hsa-miR-339-3p, hsa-miR-5194, hsa-miR-5091, hsa-miR-4673, hsa-miR-6777-3p, hsa-miR-1237-5p, hsa-miR-4284, hsa-miR-125b-2-3p, hsa-miR-1249-3p, hsa-miR-6069, hsa-miR-4508, hsa-miR-6817-5p, hsa-miR-6892-3p, hsa-miR-4529-5p, hsa-miR-363-5p, hsa-miR-4313, hsa-miR-4665-3p, hsa-miR-4781-5p, hsa-miR-650, hsa-miR-6814-5p, hsa-miR-6752-3p, hsa-miR-7107-5p, hsa-miR-6805-3p, hsa-miR-583, hsa-miR-3679-3p, hsa-miR-3682-3p, hsa-miR-4750-3p, hsa-miR-135a-2-3p, hsa-miR-6738-5p, hsa-miR-4661-5p, hsa-miR-4281, hsa-miR-6719-3p, hsa-miR-135a-3p, hsa-miR-4433a-5p, hsa-miR-625-5p, hsa-miR-769-3p, hsa-miR-3131, hsa-miR-3675-5p, hsa-miR-4439, hsa-miR-513b-5p, hsa-miR-4506, hsa-miR-6796-3p, hsa-miR-3186-3p, hsa-miR-2115-5p, hsa-miR-6797-5p, hsa-miR-4257, hsa-miR-9898, hsa-miR-6515-3p, hsa-miR-5579-5p, hsa-miR-4495, hsa-miR-4741, hsa-miR-4274, hsa-miR-1236-5p, hsa-miR-6724-5p, hsa-miR-6080, hsa-miR-1224-3p, hsa-miR-3934-5p, hsa-miR-4447, hsa-miR-7106-5p, hsa-miR-762, hsa-miR-6511a-5p, hsa-miR-6732-5p, hsa-miR-3943, hsa-miR-1227-5p, hsa-miR-5195-3p, hsa-miR-4648, hsa-miR-940, hsa-miR-942-5p, hsa-miR-7974, hsa-miR-1288-3p, hsa-miR-193b-5p, hsa-miR-6749-5p, hsa-miR-3619-5p, hsa-miR-7975, hsa-miR-149-3p, hsa-miR-6086, hsa-miR-6515-5p, hsa-miR-4787-5p, hsa-miR-210-3p, hsa-miR-4783-5p, hsa-miR-6165, hsa-miR-6848-3p, hsa-miR-7150, hsa-miR-4769-3p, hsa-miR-617, hsa-miR-668-5p, hsa-miR-6503-3p, hsa-miR-6089, hsa-miR-6813-3p, hsa-miR-1268a, hsa-miR-494-3p, hsa-miR-1910-3p, hsa-miR-550a-3-5p, hsa-miR-4733-5p, hsa-miR-8069, hsa-miR-515-5p, hsa-miR-206, hsa-miR-551b-3p, hsa-miR-9986, hsa-miR-665, hsa-miR-6720-5p, hsa-miR-514b-5p, hsa-miR-4294, hsa-miR-718, hsa-miR-6839-5p, hsa-miR-193a-5p, hsa-miR-6819-3p, hsa-miR-6742-5p, hsa-miR-1253, hsa-miR-4497, hsa-miR-1972, hsa-miR-7114-5p, hsa-miR-3200-5p, hsa-miR-4794, hsa-miR-4435, hsa-miR-4758-5p, hsa-miR-4707-5p, hsa-miR-6727-5p, hsa-miR-4522, hsa-miR-200a-5p, hsa-miR-3197, hsa-miR-7704, hsa-miR-4715-5p, hsa-miR-4651, hsa-miR-4725-3p, hsa-miR-450a-2-3p, hsa-miR-1275, hsa-miR-539-5p, hsa-miR-7158-5p, hsa-miR-604, hsa-miR-4727-3p, hsa-miR-210-5p, hsa-miR-6815-5p, hsa-miR-593-3p, hsa-miR-4706, hsa-miR-187-5p, hsa-miR-513a-5p, hsa-miR-1208, hsa-miR-4436b-3p, hsa-miR-6787-5p, hsa-miR-5006-5p, hsa-miR-92a-2-5p, hsa-miR-302c-5p, hsa-miR-4467, hsa-miR-330-3p, hsa-miR-21-3p, hsa-miR-3665, hsa-miR-3605-5p, hsa-miR-1228-3p, hsa-miR-8064, hsa-miR-4502, hsa-miR-323a-3p, hsa-miR-4516, hsa-miR-4317, hsa-miR-656-3p, hsa-miR-5190, hsa-miR-4449, hsa-miR-4537, hsa-miR-3610, hsa-miR-10394-5p, hsa-miR-30c-2-3p, hsa-miR-622, hsa-miR-1909-3p, hsa-miR-4731-3p, hsa-miR-4251, hsa-miR-92b-5p, hsa-miR-6511b-5p, hsa-miR-613, hsa-miR-4694-3p, hsa-miR-6855-5p, hsa-miR-3663-3p, hsa-miR-1913, hsa-miR-12122, hsa-miR-3529-5p, hsa-miR-4713-3p, hsa-miR-4323, hsa-miR-4635, hsa-miR-6800-5p, hsa-miR-128-1-5p, hsa-miR-6840-3p, hsa-miR-3922-5p, hsa-miR-4441, hsa-miR-6716-5p, hsa-miR-663b, hsa-miR-6084, hsa-miR-1231, hsa-miR-1281, hsa-miR-8057, hsa-miR-4687-5p, hsa-miR-96-3p, hsa-miR-513c-5p, hsa-miR-6859-3p, hsa-miR-5681a, hsa-miR-7851-3p, hsa-miR-6068, hsa-miR-3944-5p, hsa-miR-5092, hsa-miR-345-3p, hsa-miR-10522-5p, hsa-miR-300, hsa-miR-548g-3p, hsa-miR-181a-2-3p, hsa-miR-6784-5p, hsa-miR-6845-5p, hsa-miR-4734, hsa-miR-5702, hsa-miR-3918, hsa-miR-3667-5p, hsa-miR-9902, hsa-miR-198, hsa-miR-361-3p, hsa-miR-4708-3p, hsa-miR-365a-3p, hsa-miR-365b-3p, hsa-miR-3622a-5p, hsa-miR-4538, hsa-miR-6090, hsa-miR-6821-3p, hsa-miR-485-5p, hsa-miR-493-3p, hsa-miR-6070, hsa-miR-939-5p, hsa-miR-5580-5p, hsa-miR-6786-5p, hsa-miR-6500-5p, hsa-miR-6830-5p, hsa-miR-1238-3p, hsa-miR-4697-5p, hsa-miR-486-3p, hsa-miR-6731-3p, hsa-miR-3144-5p, hsa-miR-7108-5p, hsa-miR-6780b-5p, hsa-miR-12116, hsa-miR-608, hsa-miR-4672, hsa-miR-6085, hsa-miR-520b-3p, hsa-miR-6729-3p, hsa-miR-6887-3p, hsa-miR-5787, hsa-miR-4661-3p, hsa-miR-4484, hsa-miR-4685-5p, hsa-miR-3648, hsa-miR-3689d, hsa-miR-3186-5p, hsa-miR-4701-3p, hsa-miR-6730-5p, hsa-miR-6500-3p, hsa-miR-520h, hsa-miR-103a-1-5p, hsa-miR-143-3p, hsa-miR-342-5p, hsa-miR-6823-5p, hsa-miR-4276, hsa-miR-129-5p, hsa-miR-2682-5p, hsa-miR-4773, hsa-miR-3908, hsa-miR-4470, hsa-miR-7108-3p, hsa-miR-3612, hsa-miR-4463, hsa-miR-1293, hsa-miR-6790-5p, hsa-miR-6717-5p, hsa-miR-122b-3p, hsa-miR-4654, hsa-miR-7151-5p, hsa-miR-3142, hsa-miR-6504-3p, hsa-miR-4531, hsa-miR-520e-3p, hsa-miR-4258, hsa-miR-34c-5p, hsa-miR-12118, hsa-miR-6715b-3p, hsa-miR-6854-3p, hsa-miR-6782-5p, hsa-miR-6529-5p, hsa-miR-371b-5p, hsa-miR-1825, hsa-miR-2277-5p, hsa-miR-6134, hsa-miR-3155b, hsa-miR-8077, hsa-miR-6804-5p, hsa-miR-4999-5p, hsa-miR-6499-5p, hsa-miR-1301-5p, hsa-miR-6768-5p, hsa-miR-6504-5p, hsa-miR-5010-5p, hsa-miR-4304, hsa-miR-6848-5p, hsa-miR-3621, hsa-miR-3614-5p, hsa-miR-4763-3p, hsa-miR-3619-3p, hsa-miR-20b-3p, hsa-miR-937-3p, hsa-miR-11399, hsa-miR-93-5p, hsa-miR-7109-5p, hsa-miR-12120, hsa-miR-5739, hsa-miR-6756-5p, hsa-miR-3122, hsa-miR-6737-5p, hsa-miR-5004-5p, hsa-miR-487a-5p, hsa-miR-572, hsa-miR-652-5p, hsa-miR-6776-5p, hsa-miR-5703, hsa-miR-4446-3p, hsa-miR-4300, hsa-miR-3188, hsa-miR-589-3p, hsa-miR-6855-3p, hsa-miR-654-5p, hsa-miR-224-3p, hsa-miR-4265, hsa-miR-6788-5p, hsa-miR-296-5p, hsa-miR-744-5p, hsa-miR-320c, hsa-miR-5685, hsa-miR-6088, hsa-miR-1299, hsa-miR-652-3p, hsa-miR-4480, hsa-miR-4764-5p, hsa-miR-511-3p, hsa-miR-4432, hsa-miR-133a-5p, hsa-miR-2052, hsa-miR-139-5p, hsa-miR-4477b, hsa-miR-4728-3p, hsa-miR-25-3p, hsa-miR-943, hsa-miR-639, hsa-miR-6734-5p, hsa-miR-6791-3p, hsa-miR-4505, hsa-miR-1228-5p, hsa-miR-29b-1-5p, hsa-miR-6718-5p, hsa-miR-663a, hsa-miR-6748-5p, hsa-miR-6075, hsa-miR-4429, hsa-miR-4768-3p, hsa-miR-3085-3p, hsa-miR-647, hsa-miR-1251-3p, hsa-miR-6875-5p, hsa-miR-646, hsa-miR-6876-3p, hsa-miR-194-3p, hsa-miR-4283, hsa-miR-211-3p, hsa-miR-15a-3p, hsa-miR-766-5p, hsa-miR-1468-5p, hsa-miR-6880-5p, hsa-miR-23b-3p, hsa-miR-216a-3p, hsa-miR-5090, hsa-miR-6891-5p, hsa-miR-6893-5p, hsa-miR-3125, hsa-miR-6720-3p, hsa-miR-4738-5p, hsa-miR-503-3p, hsa-miR-514b-3p, hsa-miR-6513-3p, hsa-miR-4530, hsa-miR-887-3p, hsa-miR-770-5p, hsa-miR-6130, hsa-miR-1204, hsa-miR-3074-5p, hsa-miR-921, hsa-miR-5693, hsa-miR-365a-5p, hsa-miR-4712-5p, hsa-miR-1269b, hsa-miR-138-5p, hsa-miR-4316, hsa-miR-2116-3p, hsa-miR-6891-3p, hsa-miR-4514, hsa-miR-4717-5p, hsa-miR-6889-5p, hsa-miR-6792-3p, hsa-miR-4783-3p, hsa-miR-4454, hsa-miR-6797-3p, hsa-miR-6886-3p, hsa-miR-877-5p, hsa-miR-6869-5p, hsa-miR-6871-5p, hsa-miR-4489, hsa-miR-4776-3p, hsa-miR-501-5p, hsa-miR-4482-3p, hsa-miR-3666, hsa-miR-4330, hsa-miR-6861-3p, hsa-miR-6765-5p, hsa-miR-6833-5p, hsa-miR-10394-3p, hsa-miR-18b-3p, hsa-miR-4802-3p, hsa-miR-4707-3p, hsa-miR-4515, hsa-miR-142-3p, hsa-miR-484, hsa-miR-208a-5p, hsa-miR-3691-3p, hsa-miR-3940-5p, hsa-miR-3615, hsa-miR-6858-3p, hsa-miR-6722-3p, hsa-miR-877-3p, hsa-miR-3120-5p, hsa-miR-4724-3p, hsa-miR-320e, hsa-miR-3074-3p, hsa-miR-6774-5p, hsa-miR-18a-3p, hsa-miR-3659, hsa-miR-105-5p, hsa-miR-4488, hsa-miR-1267, hsa-miR-483-3p, hsa-miR-3192-5p, hsa-miR-12114, hsa-miR-8063, hsa-miR-4444, hsa-miR-4736, hsa-miR-2276-3p, hsa-miR-3654, hsa-miR-5585-3p, hsa-miR-6775-5p, hsa-let-7e-3p, hsa-miR-6812-5p, hsa-miR-564, hsa-miR-4649-5p, hsa-miR-554, hsa-miR-6083, hsa-miR-4496, hsa-miR-6892-5p, hsa-miR-4279, hsa-miR-6895-3p, hsa-miR-6499-3p, hsa-miR-3173-5p, hsa-miR-411-3p, hsa-miR-1976, hsa-miR-3936, hsa-miR-519a-3p, hsa-miR-6883-5p, hsa-miR-3135b, hsa-miR-197-3p, hsa-miR-4676-3p, hsa-miR-6132, hsa-miR-1237-3p, hsa-miR-6842-3p, hsa-miR-4688, hsa-miR-128-2-5p, hsa-miR-4486, hsa-miR-3162-5p, hsa-miR-4664-5p, hsa-miR-3187-5p, hsa-miR-4322, hsa-miR-3195, hsa-miR-4499, hsa-miR-1234-3p, hsa-miR-557, hsa-miR-4723-5p, hsa-miR-5584-3p, hsa-miR-371a-5p, hsa-miR-4708-5p, hsa-miR-4487, hsa-miR-6764-3p, hsa-miR-6817-3p, hsa-miR-9500, hsa-miR-1296-3p, hsa-miR-4540, hsa-miR-2467-3p, hsa-miR-3945, hsa-miR-6793-3p, hsa-miR-4769-5p, hsa-miR-378g, hsa-miR-6889-3p, hsa-miR-9718, hsa-miR-3692-5p, hsa-miR-7110-3p, hsa-miR-378b, hsa-miR-3184-3p, hsa-miR-147a, hsa-miR-4747-5p, hsa-miR-139-3p, hsa-miR-3655, hsa-miR-3620-5p, hsa-miR-103a-2-5p, hsa-miR-5681b, hsa-miR-3065-3p, hsa-miR-6756-3p, hsa-miR-8059, hsa-miR-3652, hsa-miR-6847-5p, hsa-miR-6779-5p, hsa-miR-6806-5p, hsa-miR-654-3p, hsa-miR-4306, hsa-miR-6857-3p, hsa-miR-6861-5p, hsa-miR-935, hsa-miR-3141, hsa-miR-371a-3p, hsa-miR-6870-3p, hsa-miR-3689b-3p, hsa-miR-3689c, hsa-miR-6777-5p, hsa-miR-6870-5p, hsa-miR-936, hsa-miR-6747-5p, hsa-miR-493-5p, hsa-miR-4691-5p, hsa-miR-10392-5p, hsa-miR-885-3p, hsa-miR-6505-3p, hsa-miR-4687-3p, hsa-miR-6837-3p, hsa-miR-6801-3p, hsa-miR-34b-3p, hsa-miR-10401-5p, hsa-miR-760, hsa-miR-3690, hsa-miR-6728-5p, hsa-miR-6792-5p, hsa-miR-4676-5p, hsa-miR-6809-3p, hsa-miR-5006-3p, hsa-miR-3200-3p, hsa-miR-3688-3p, hsa-miR-938, hsa-miR-4453, hsa-miR-658, hsa-miR-6767-3p, hsa-miR-4686, hsa-miR-4526, hsa-miR-1203, hsa-miR-12117, hsa-miR-891a-5p, hsa-miR-6131, hsa-miR-5587-3p, hsa-miR-1471, hsa-miR-11400, hsa-miR-2276-5p, hsa-miR-6802-5p, hsa-let-7e-5p, hsa-miR-6759-5p, hsa-miR-6801-5p, hsa-miR-619-5p, hsa-miR-1301-3p, hsa-miR-1343-3p, hsa-miR-3713, hsa-miR-6862-3p, hsa-miR-661, hsa-miR-6856-3p, hsa-miR-1284, hsa-miR-4738-3p, hsa-miR-7158-3p, hsa-miR-6766-5p, hsa-miR-6760-5p, hsa-miR-431-5p, hsa-miR-6808-5p, hsa-miR-4260, hsa-miR-6840-5p, hsa-miR-6872-5p, hsa-miR-3689a-3p, hsa-miR-4262, hsa-miR-6512-3p, hsa-miR-6746-3p, hsa-miR-6812-3p, hsa-miR-3138, hsa-miR-6849-5p, hsa-miR-6837-5p, hsa-miR-4535, and hsa-miR-5002-3p. In the present disclosure, the extract of urine may be an extract of the urine of a thyroid cancer patient. Only one of these microRNAs can be used to predict whether a subject from which the urine is derived is a thyroid cancer patient, and 5 or more types, 10 or more types, 15 or more types, or 20 or more types of these microRNAs can be used in combination to predict whether a subject from which the urine is derived is a thyroid cancer patient. Among these microRNAs, a microRNA that may be highly expressed in a thyroid cancer patient can be preferably used for prediction.

The present disclosure provides an extract of urine containing at least one type, two types, three types, four types, five types, six types, seven types, eight types, nine types, or ten types of or all microRNAs selected from the group consisting of hsa-miR-17-3p, hsa-miR-25-3p, hsa-miR-197-3p, hsa-miR-198, hsa-miR-139-5p, hsa-miR-124-3p, hsa-miR-185-5p, hsa-miR-296-5p, hsa-miR-365a-3p, hsa-miR-365b-3p, hsa-miR-302c-5p, hsa-miR-370-3p, hsa-miR-373-5p, hsa-miR-383-5p, hsa-miR-330-3p, hsa-miR-328-3p, hsa-miR-324-5p, hsa-miR-324-3p, hsa-miR-346, hsa-miR-422a, hsa-miR-483-3p, hsa-miR-484, hsa-miR-485-5p, hsa-miR-491-5p, hsa-miR-493-5p, hsa-miR-498-5p, hsa-miR-520e-3p, hsa-miR-519b-3p, hsa-miR-525-3p, hsa-miR-518f-3p, hsa-miR-520b-3p, hsa-miR-513a-5p, hsa-miR-557, hsa-miR-572, hsa-miR-574-3p, hsa-miR-575, hsa-miR-583, hsa-miR-550a-3p, hsa-miR-611, hsa-miR-612, hsa-miR-613, hsa-miR-625-5p, hsa-miR-634, hsa-miR-636, hsa-miR-638, hsa-miR-639, hsa-miR-650, hsa-miR-652-3p, hsa-miR-661, hsa-miR-663a, hsa-miR-654-5p, hsa-miR-658, hsa-miR-542-5p, hsa-miR-376a-5p, hsa-miR-668-3p, hsa-miR-766-3p, hsa-miR-765, hsa-miR-297, hsa-miR-22-5p, hsa-miR-92a-2-5p, hsa-miR-29b-1-5p, hsa-miR-30c-2-3p, hsa-miR-139-3p, hsa-miR-181a-2-3p, hsa-miR-183-3p, hsa-miR-187-5p, hsa-miR-135a-3p, hsa-miR-127-5p, hsa-miR-149-3p, hsa-miR-150-3p, hsa-miR-185-3p, hsa-miR-186-3p, hsa-miR-194-3p, hsa-miR-30c-1-3p, hsa-miR-296-3p, hsa-miR-361-3p, hsa-miR-371a-5p, hsa-miR-342-5p, hsa-miR-339-3p, hsa-miR-424-3p, hsa-miR-18b-3p, hsa-miR-431-3p, hsa-miR-483-5p, hsa-miR-486-3p, hsa-miR-509-5p, hsa-miR-92b-5p, hsa-miR-550a-5p, hsa-miR-615-5p, hsa-miR-625-3p, hsa-miR-744-5p, hsa-miR-744-3p, hsa-miR-885-5p, hsa-miR-885-3p, hsa-miR-877-5p, hsa-miR-877-3p, hsa-miR-887-3p, hsa-miR-665, hsa-miR-374b-3p, hsa-miR-760, hsa-miR-920, hsa-miR-935, hsa-miR-936, hsa-miR-937-3p, hsa-miR-939-5p, hsa-miR-940, hsa-miR-943, hsa-miR-1224-3p, hsa-miR-1225-5p, hsa-miR-1225-3p, hsa-miR-1226-5p, hsa-miR-1226-3p, hsa-miR-1228-5p, hsa-miR-1228-3p, hsa-miR-1229-3p, hsa-miR-1231, hsa-miR-1234-3p, hsa-miR-1237-3p, hsa-miR-1238-3p, hsa-miR-1181, hsa-miR-1182, hsa-miR-1184, hsa-miR-1203, hsa-miR-663b, hsa-miR-1204, hsa-miR-1207-5p, hsa-miR-1207-3p, hsa-miR-1208, hsa-miR-1299, hsa-miR-1303, hsa-miR-1246, hsa-miR-1249-3p, hsa-miR-1253, hsa-miR-1255a, hsa-miR-1260a, hsa-miR-1266-5p, hsa-miR-1267, hsa-miR-1268a, hsa-miR-1269a, hsa-miR-1275, hsa-miR-1281, hsa-miR-1292-5p, hsa-miR-1307-3p, hsa-miR-1321, hsa-miR-1825, hsa-miR-1469, hsa-miR-1470, hsa-miR-1538, hsa-miR-1539, hsa-miR-1908-5p, hsa-miR-1909-5p, hsa-miR-1909-3p, hsa-miR-1910-5p, hsa-miR-1912-3p, hsa-miR-1913, hsa-miR-1914-3p, hsa-miR-1915-5p, hsa-miR-365a-5p, hsa-miR-449b-3p, hsa-miR-1972, hsa-miR-1976, hsa-miR-2052, hsa-miR-2110, hsa-miR-449c-5p, hsa-miR-762, hsa-miR-670-5p, hsa-miR-2116-3p, hsa-miR-548q, hsa-miR-2276-3p, hsa-miR-2277-3p, hsa-miR-711, hsa-miR-718, hsa-miR-2681-3p, hsa-miR-3116, hsa-miR-3122, hsa-miR-3124-5p, hsa-miR-3126-5p, hsa-miR-3130-3p, hsa-miR-3130-5p, hsa-miR-3131, hsa-miR-3132, hsa-miR-3133, hsa-miR-3138, hsa-miR-3141, hsa-miR-3144-5p, hsa-miR-3147, hsa-miR-3151-5p, hsa-miR-3153, hsa-miR-3074-3p, hsa-miR-3155a, hsa-miR-3156-5p, hsa-miR-3158-3p, hsa-miR-3162-5p, hsa-miR-3164, hsa-miR-1260b, hsa-miR-3173-3p, hsa-miR-1193, hsa-miR-3177-3p, hsa-miR-3178, hsa-miR-3180-3p, hsa-miR-3185, hsa-miR-3186-3p, hsa-miR-3187-3p, hsa-miR-3188, hsa-miR-3189-3p, hsa-miR-3195, hsa-miR-3196, hsa-miR-3197, hsa-miR-3199, hsa-miR-3200-3p, hsa-miR-514b-5p, hsa-miR-4297, hsa-miR-4293, hsa-miR-4294, hsa-miR-4299, hsa-miR-4298, hsa-miR-4306, hsa-miR-4307, hsa-miR-4312, hsa-miR-4313, hsa-miR-4316, hsa-miR-4322, hsa-miR-4321, hsa-miR-4323, hsa-miR-4257, hsa-miR-4258, hsa-miR-4259, hsa-miR-4260, hsa-miR-4253, hsa-miR-4251, hsa-miR-4254, hsa-miR-4326, hsa-miR-4327, hsa-miR-4265, hsa-miR-2355-5p, hsa-miR-4269, hsa-miR-4271, hsa-miR-4276, hsa-miR-4274, hsa-miR-4281, hsa-miR-4279, hsa-miR-4278, hsa-miR-4280, hsa-miR-4284, hsa-miR-4286, hsa-miR-4288, hsa-miR-4289, hsa-miR-4290, hsa-miR-2277-5p, hsa-miR-3200-5p, hsa-miR-3610, hsa-miR-3612, hsa-miR-3614-5p, hsa-miR-3615, hsa-miR-3616-3p, hsa-miR-3619-5p, hsa-miR-3621, hsa-miR-3622a-5p, hsa-miR-3622a-3p, hsa-miR-3646, hsa-miR-3648, hsa-miR-3651, hsa-miR-3652, hsa-miR-3663-3p, hsa-miR-3665, hsa-miR-3667-5p, hsa-miR-3675-3p, hsa-miR-3677-3p, hsa-miR-3679-5p, hsa-miR-3679-3p, hsa-miR-3688-3p, hsa-miR-3714, hsa-miR-3180, hsa-miR-3907, hsa-miR-3908, hsa-miR-3917, hsa-miR-3922-3p, hsa-miR-3928-3p, hsa-miR-3936, hsa-miR-3937, hsa-miR-3943, hsa-miR-3944-3p, hsa-miR-1268b, hsa-miR-4429, hsa-miR-4430, hsa-miR-4431, hsa-miR-4432, hsa-miR-4433a-3p, hsa-miR-4441, hsa-miR-4444, hsa-miR-4446-3p, hsa-miR-4447, hsa-miR-4448, hsa-miR-4449, hsa-miR-3135b, hsa-miR-4463, hsa-miR-4466, hsa-miR-4467, hsa-miR-4470, hsa-miR-4472, hsa-miR-4473, hsa-miR-4476, hsa-miR-4477b, hsa-miR-3689d, hsa-miR-4479, hsa-miR-3155b, hsa-miR-4480, hsa-miR-4483, hsa-miR-4484, hsa-miR-4486, hsa-miR-4488, hsa-miR-4489, hsa-miR-4492, hsa-miR-4494, hsa-miR-4495, hsa-miR-4496, hsa-miR-4497, hsa-miR-4499, hsa-miR-4505, hsa-miR-4507, hsa-miR-4508, hsa-miR-4512, hsa-miR-4513, hsa-miR-4516, hsa-miR-4521, hsa-miR-4525, hsa-miR-4526, hsa-miR-4530, hsa-miR-4531, hsa-miR-4533, hsa-miR-4534, hsa-miR-548am-3p, hsa-miR-1587, hsa-miR-4536-5p, hsa-miR-4539, hsa-miR-3120-5p, hsa-miR-3127-3p, hsa-miR-3129-3p, hsa-miR-3150a-5p, hsa-miR-3158-5p, hsa-miR-3162-3p, hsa-miR-3173-5p, hsa-miR-3187-5p, hsa-miR-3619-3p, hsa-miR-3691-3p, hsa-miR-3150b-5p, hsa-miR-3940-5p, hsa-miR-3960, hsa-miR-3972, hsa-miR-3978, hsa-miR-4634, hsa-miR-4637, hsa-miR-4639-5p, hsa-miR-4640-5p, hsa-miR-4648, hsa-miR-4649-5p, hsa-miR-4651, hsa-miR-4652-5p, hsa-miR-4655-5p, hsa-miR-4661-5p, hsa-miR-4664-5p, hsa-miR-4664-3p, hsa-miR-4665-5p, hsa-miR-4665-3p, hsa-miR-4667-3p, hsa-miR-4668-5p, hsa-miR-4673, hsa-miR-4674, hsa-miR-4684-3p, hsa-miR-4685-5p, hsa-miR-4685-3p, hsa-miR-4687-5p, hsa-miR-4687-3p, hsa-miR-1343-3p, hsa-miR-4688, hsa-miR-4690-5p, hsa-miR-4691-5p, hsa-miR-4691-3p, hsa-miR-4695-5p, hsa-miR-4697-5p, hsa-miR-4697-3p, hsa-miR-4706, hsa-miR-4707-5p, hsa-miR-4707-3p, hsa-miR-4708-5p, hsa-miR-4708-3p, hsa-miR-4714-5p, hsa-miR-4715-5p, hsa-miR-4716-5p, hsa-miR-4716-3p, hsa-miR-4717-3p, hsa-miR-4722-5p, hsa-miR-4723-5p, hsa-miR-4723-3p, hsa-miR-4725-5p, hsa-miR-4725-3p, hsa-miR-4726-3p, hsa-miR-4728-5p, hsa-miR-4728-3p, hsa-miR-4730, hsa-miR-4731-5p, hsa-miR-4731-3p, hsa-miR-4732-5p, hsa-miR-4734, hsa-miR-4736, hsa-miR-4738-5p, hsa-miR-4738-3p, hsa-miR-4741, hsa-miR-4745-5p, hsa-miR-4746-3p, hsa-miR-4747-5p, hsa-miR-4748, hsa-miR-4749-5p, hsa-miR-4749-3p, hsa-miR-4750-5p, hsa-miR-371b-5p, hsa-miR-371b-3p, hsa-miR-4755-3p, hsa-miR-4756-5p, hsa-miR-4758-5p, hsa-miR-4758-3p, hsa-miR-4761-3p, hsa-miR-4763-5p, hsa-miR-4763-3p, hsa-miR-4764-5p, hsa-miR-4767, hsa-miR-4769-3p, hsa-miR-4778-5p, hsa-miR-4780, hsa-miR-4436b-5p, hsa-miR-4781-5p, hsa-miR-4783-3p, hsa-miR-4786-3p, hsa-miR-4787-5p, hsa-miR-4787-3p, hsa-miR-4791, hsa-miR-4793-3p, hsa-miR-4798-3p, hsa-miR-4800-5p, hsa-miR-4800-3p, hsa-miR-4802-3p, hsa-miR-4433a-5p, hsa-miR-4482-3p, hsa-miR-4536-3p, hsa-miR-5001-5p, hsa-miR-5001-3p, hsa-miR-5002-5p, hsa-miR-5002-3p, hsa-miR-5008-5p, hsa-miR-5010-5p, hsa-miR-5088-5p, hsa-miR-5090, hsa-miR-5092, hsa-miR-5190, hsa-miR-5193, hsa-miR-5195-5p, hsa-miR-5195-3p, hsa-miR-5196-5p, hsa-miR-5572, hsa-miR-548as-5p, hsa-miR-548as-3p, hsa-miR-5579-5p, hsa-miR-5579-3p, hsa-miR-5587-3p, hsa-miR-5589-5p, hsa-miR-5681a, hsa-miR-5685, hsa-miR-5698, hsa-miR-5705, hsa-miR-211-3p, hsa-miR-345-3p, hsa-miR-584-3p, hsa-miR-652-5p, hsa-miR-1185-2-3p, hsa-miR-873-3p, hsa-miR-1247-3p, hsa-miR-3184-3p, hsa-miR-365b-5p, hsa-miR-1185-1-3p, hsa-miR-98-3p, hsa-miR-503-3p, hsa-miR-937-5p, hsa-miR-1227-5p, hsa-miR-1233-5p, hsa-miR-1237-5p, hsa-miR-1238-5p, hsa-miR-1292-3p, hsa-miR-3620-5p, hsa-miR-4750-3p, hsa-miR-5739, hsa-miR-5787, hsa-miR-6068, hsa-miR-6069, hsa-miR-6071, hsa-miR-6075, hsa-miR-6076, hsa-miR-6085, hsa-miR-6086, hsa-miR-6088, hsa-miR-6089, hsa-miR-6090, hsa-miR-6124, hsa-miR-6125, hsa-miR-6126, hsa-miR-6127, hsa-miR-6131, hsa-miR-6132, hsa-miR-6133, hsa-miR-6165, hsa-miR-6501-5p, hsa-miR-6503-5p, hsa-miR-6503-3p, hsa-miR-6505-5p, hsa-miR-6511a-5p, hsa-miR-6511a-3p, hsa-miR-6513-5p, hsa-miR-6514-3p, hsa-miR-6515-3p, hsa-miR-6715b-3p, hsa-miR-6716-5p, hsa-miR-6716-3p, hsa-miR-6511b-Sp, hsa-miR-6720-3p, hsa-miR-6721-5p, hsa-miR-6722-5p, hsa-miR-6722-3p, hsa-miR-6724-5p, hsa-miR-208a-5p, hsa-miR-210-5p, hsa-miR-128-1-5p, hsa-miR-133a-5p, hsa-miR-489-5p, hsa-miR-511-3p, hsa-miR-181d-3p, hsa-miR-585-5p, hsa-miR-598-5p, hsa-miR-627-3p, hsa-miR-1296-3p, hsa-miR-1301-5p, hsa-miR-874-5p, hsa-miR-216b-3p, hsa-miR-1287-3p, hsa-miR-1908-3p, hsa-miR-3151-3p, hsa-miR-3192-3p, hsa-miR-1343-5p, hsa-miR-5189-3p, hsa-miR-5699-5p, hsa-miR-6726-5p, hsa-miR-6726-3p, hsa-miR-6727-5p, hsa-miR-6727-3p, hsa-miR-6728-5p, hsa-miR-6728-3p, hsa-miR-6729-5p, hsa-miR-6729-3p, hsa-miR-6730-5p, hsa-miR-6731-3p, hsa-miR-6732-5p, hsa-miR-6732-3p, hsa-miR-6734-5p, hsa-miR-6736-3p, hsa-miR-6737-5p, hsa-miR-6738-5p, hsa-miR-6738-3p, hsa-miR-6740-3p, hsa-miR-6741-3p, hsa-miR-6742-5p, hsa-miR-6742-3p, hsa-miR-6743-5p, hsa-miR-6743-3p, hsa-miR-6745, hsa-miR-6746-5p, hsa-miR-6746-3p, hsa-miR-6748-5p, hsa-miR-6749-5p, hsa-miR-6749-3p, hsa-miR-6750-5p, hsa-miR-6751-5p, hsa-miR-6752-5p, hsa-miR-6752-3p, hsa-miR-6753-5p, hsa-miR-6754-3p, hsa-miR-6756-5p, hsa-miR-6756-3p, hsa-miR-6758-5p, hsa-miR-6759-5p, hsa-miR-6759-3p, hsa-miR-6760-5p, hsa-miR-6760-3p, hsa-miR-6761-5p, hsa-miR-6761-3p, hsa-miR-6762-5p, hsa-miR-6762-3p, hsa-miR-6763-5p, hsa-miR-6763-3p, hsa-miR-6764-3p, hsa-miR-6765-5p, hsa-miR-6766-5p, hsa-miR-6766-3p, hsa-miR-6768-5p, hsa-miR-6769a-3p, hsa-miR-6770-3p, hsa-miR-6772-5p, hsa-miR-6774-5p, hsa-miR-6775-5p, hsa-miR-6776-5p, hsa-miR-6776-3p, hsa-miR-6777-5p, hsa-miR-6777-3p, hsa-miR-6779-5p, hsa-miR-6781-5p, hsa-miR-6782-5p, hsa-miR-6782-3p, hsa-miR-6784-5p, hsa-miR-6784-3p, hsa-miR-6785-3p, hsa-miR-6786-5p, hsa-miR-6787-5p, hsa-miR-6787-3p, hsa-miR-6789-5p, hsa-miR-6789-3p, hsa-miR-6790-5p, hsa-miR-6790-3p, hsa-miR-6791-5p, hsa-miR-6792-5p, hsa-miR-6792-3p, hsa-miR-6793-3p, hsa-miR-6794-5p, hsa-miR-6795-5p, hsa-miR-6795-3p, hsa-miR-6796-3p, hsa-miR-6797-5p, hsa-miR-6797-3p, hsa-miR-6798-5p, hsa-miR-6798-3p, hsa-miR-6799-5p, hsa-miR-6799-3p, hsa-miR-6800-5p, hsa-miR-6800-3p, hsa-miR-6801-3p, hsa-miR-6802-5p, hsa-miR-6802-3p, hsa-miR-6803-5p, hsa-miR-6803-3p, hsa-miR-6804-3p, hsa-miR-6805-5p, hsa-miR-6805-3p, hsa-miR-6806-5p, hsa-miR-6808-5p, hsa-miR-6808-3p, hsa-miR-6809-5p, hsa-miR-6809-3p, hsa-miR-6810-5p, hsa-miR-6810-3p, hsa-miR-6812-5p, hsa-miR-6812-3p, hsa-miR-6813-5p, hsa-miR-6813-3p, hsa-miR-6815-5p, hsa-miR-6816-5p, hsa-miR-6816-3p, hsa-miR-6817-3p, hsa-miR-6819-3p, hsa-miR-6820-5p, hsa-miR-6820-3p, hsa-miR-6821-5p, hsa-miR-6821-3p, hsa-miR-6822-5p, hsa-miR-6823-3p, hsa-miR-6824-3p, hsa-miR-6825-5p, hsa-miR-6825-3p, hsa-miR-6827-5p, hsa-miR-6828-5p, hsa-miR-6829-5p, hsa-miR-6830-5p, hsa-miR-6830-3p, hsa-miR-6832-5p, hsa-miR-6832-3p, hsa-miR-6833-5p, hsa-miR-6833-3p, hsa-miR-6780b-5p, hsa-miR-6780b-3p, hsa-miR-6836-5p, hsa-miR-6836-3p, hsa-miR-6837-3p, hsa-miR-6838-5p, hsa-miR-6840-5p, hsa-miR-6840-3p, hsa-miR-6842-5p, hsa-miR-6845-5p, hsa-miR-6845-3p, hsa-miR-6846-5p, hsa-miR-6848-5p, hsa-miR-6848-3p, hsa-miR-6849-5p, hsa-miR-6849-3p, hsa-miR-6850-5p, hsa-miR-6855-3p, hsa-miR-6857-5p, hsa-miR-6857-3p, hsa-miR-6858-3p, hsa-miR-6859-3p, hsa-miR-6860, hsa-miR-6861-5p, hsa-miR-6861-3p, hsa-miR-6862-5p, hsa-miR-6862-3p, hsa-miR-6865-3p, hsa-miR-6867-5p, hsa-miR-6868-3p, hsa-miR-6869-5p, hsa-miR-6870-5p, hsa-miR-6870-3p, hsa-miR-6872-5p, hsa-miR-6875-5p, hsa-miR-6875-3p, hsa-miR-6876-3p, hsa-miR-6877-5p, hsa-miR-6877-3p, hsa-miR-6878-5p, hsa-miR-6878-3p, hsa-miR-6879-3p, hsa-miR-6880-5p, hsa-miR-6880-3p, hsa-miR-6881-5p, hsa-miR-6882-3p, hsa-miR-6883-5p, hsa-miR-6884-3p, hsa-miR-6885-3p, hsa-miR-6886-5p, hsa-miR-6886-3p, hsa-miR-6887-5p, hsa-miR-6887-3p, hsa-miR-6889-5p, hsa-miR-6889-3p, hsa-miR-6890-3p, hsa-miR-6891-5p, hsa-miR-6891-3p, hsa-miR-6892-3p, hsa-miR-6893-5p, hsa-miR-6893-3p, hsa-miR-6894-3p, hsa-miR-6895-3p, hsa-miR-7106-5p, hsa-miR-7106-3p, hsa-miR-7107-5p, hsa-miR-7108-5p, hsa-miR-7108-3p, hsa-miR-7109-5p, hsa-miR-7109-3p, hsa-miR-7110-3p, hsa-miR-7111-Sp, hsa-miR-7111-3p, hsa-miR-7112-5p, hsa-miR-7113-3p, hsa-miR-7114-5p, hsa-miR-7114-3p, hsa-miR-7150, hsa-miR-7151-5p, hsa-miR-7152-5p, hsa-miR-7152-3p, hsa-miR-7155-3p, hsa-miR-7704, hsa-miR-7706, hsa-miR-7843-5p, hsa-miR-4433b-5p, hsa-miR-4433b-3p, hsa-miR-1273h-5p, hsa-miR-7851-3p, hsa-miR-7855-5p, hsa-miR-8059, hsa-miR-8063, hsa-miR-8064, hsa-miR-8069, hsa-miR-8071, hsa-miR-8072, hsa-miR-8073, hsa-miR-8078, hsa-miR-8079, hsa-miR-8080, hsa-miR-8087, hsa-miR-8089, hsa-miR-128-2-5p, hsa-miR-1199-3p, hsa-miR-7974, hsa-miR-7977, hsa-miR-7978, hsa-miR-4485-5p, hsa-miR-8485, hsa-miR-9500, hsa-miR-103a-1-5p, hsa-miR-137-5p, hsa-miR-9898, hsa-miR-9902, hsa-miR-10392-5p, hsa-miR-10392-3p, hsa-miR-10394-3p, hsa-miR-10396a-5p, hsa-miR-10396a-3p, hsa-miR-10398-5p, hsa-miR-10400-5p, hsa-miR-10400-3p, hsa-miR-10401-5p, hsa-miR-10401-3p, hsa-miR-10396b-5p, hsa-miR-10396b-3p, hsa-miR-10522-5p, hsa-miR-10526-3p, hsa-miR-10527-5p, hsa-miR-11399, hsa-miR-3059-5p, hsa-miR-3059-3p, hsa-miR-3085-5p, hsa-miR-3085-3p, hsa-miR-12114, hsa-miR-12115, hsa-miR-12116, hsa-miR-12118, hsa-miR-12119, hsa-miR-12120, hsa-miR-12121, hsa-miR-12128, hsa-miR-12131, and hsa-miR-12135. In the present disclosure, the extract of urine may be an extract of the urine of a stage-I or -II thyroid cancer patient. Only one of these microRNAs can be used to predict whether a subject from which the urine is derived is a thyroid cancer patient (particularly stage-I or -II), and 5 or more types, 10 or more types, 15 or more types, or 20 or more types of these microRNAs can be used in combination to predict whether a subject from which the urine is derived is a thyroid cancer patient (particularly stage-I or -II). Among these microRNAs, a microRNA that may be highly expressed in a thyroid cancer patient (particularly stage-I or -II) can be preferably used for prediction.

The present disclosure provides an extract of urine containing at least one type, two types, three types, four types, five types, six types, seven types, eight types, nine types, or ten types of or all microRNAs selected from the group consisting of hsa-miR-17-3p, hsa-miR-25-3p, hsa-miR-197-3p, hsa-miR-198, hsa-miR-139-5p, hsa-miR-124-3p, hsa-miR-185-5p, hsa-miR-296-5p, hsa-miR-365a-3p, hsa-miR-365b-3p, hsa-miR-302c-5p, hsa-miR-370-3p, hsa-miR-373-5p, hsa-miR-383-5p, hsa-miR-330-3p, hsa-miR-328-3p, hsa-miR-324-5p, hsa-miR-324-3p, hsa-miR-346, hsa-miR-422a, hsa-miR-483-3p, hsa-miR-484, hsa-miR-485-5p, hsa-miR-491-5p, hsa-miR-498-5p, hsa-miR-520e-3p, hsa-miR-519b-3p, hsa-miR-525-3p, hsa-miR-518f-3p, hsa-miR-520b-3p, hsa-miR-513a-5p, hsa-miR-510-5p, hsa-miR-557, hsa-miR-572, hsa-miR-575, hsa-miR-583, hsa-miR-550a-3p, hsa-miR-611, hsa-miR-612, hsa-miR-613, hsa-miR-625-5p, hsa-miR-634, hsa-miR-636, hsa-miR-638, hsa-miR-639, hsa-miR-650, hsa-miR-652-3p, hsa-miR-661, hsa-miR-663a, hsa-miR-654-5p, hsa-miR-658, hsa-miR-421, hsa-miR-542-5p, hsa-miR-668-3p, hsa-miR-766-3p, hsa-miR-297, hsa-miR-15a-3p, hsa-miR-22-5p, hsa-miR-25-5p, hsa-miR-92a-2-5p, hsa-miR-29b-1-5p, hsa-miR-30c-2-3p, hsa-miR-139-3p, hsa-miR-181a-2-3p, hsa-miR-183-3p, hsa-miR-187-5p, hsa-miR-214-5p, hsa-miR-30b-3p, hsa-miR-135a-3p, hsa-miR-127-5p, hsa-miR-149-3p, hsa-miR-150-3p, hsa-miR-185-3p, hsa-miR-186-3p, hsa-miR-194-3p, hsa-miR-30c-1-3p, hsa-miR-296-3p, hsa-miR-361-3p, hsa-miR-371a-5p, hsa-miR-342-5p, hsa-miR-339-3p, hsa-miR-424-3p, hsa-miR-18b-3p, hsa-miR-431-3p, hsa-miR-483-5p, hsa-miR-486-3p, hsa-miR-509-5p, hsa-miR-92b-5p, hsa-miR-550a-5p, hsa-miR-615-5p, hsa-miR-625-3p, hsa-miR-744-5p, hsa-miR-744-3p, hsa-miR-885-5p, hsa-miR-885-3p, hsa-miR-877-5p, hsa-miR-877-3p, hsa-miR-887-3p, hsa-miR-665, hsa-miR-374b-3p, hsa-miR-760, hsa-miR-920, hsa-miR-935, hsa-miR-936, hsa-miR-937-3p, hsa-miR-939-5p, hsa-miR-940, hsa-miR-943, hsa-miR-1224-3p, hsa-miR-1225-5p, hsa-miR-1225-3p, hsa-miR-1226-3p, hsa-miR-1228-5p, hsa-miR-1228-3p, hsa-miR-1229-3p, hsa-miR-1231, hsa-miR-1234-3p, hsa-miR-1237-3p, hsa-miR-1238-3p, hsa-miR-1181, hsa-miR-1182, hsa-miR-1184, hsa-miR-1203, hsa-miR-663b, hsa-miR-1204, hsa-miR-1207-5p, hsa-miR-1208, hsa-miR-1286, hsa-miR-1299, hsa-miR-1303, hsa-miR-1246, hsa-miR-1249-3p, hsa-miR-1253, hsa-miR-1255a, hsa-miR-1260a, hsa-miR-1267, hsa-miR-1268a, hsa-miR-1269a, hsa-miR-1275, hsa-miR-1281, hsa-miR-1292-5p, hsa-miR-1307-3p, hsa-miR-1825, hsa-miR-1469, hsa-miR-1470, hsa-miR-1538, hsa-miR-1908-5p, hsa-miR-1909-5p, hsa-miR-1909-3p, hsa-miR-1910-5p, hsa-miR-1912-3p, hsa-miR-1913, hsa-miR-1914-3p, hsa-miR-1915-3p, hsa-miR-365a-5p, hsa-miR-196b-3p, hsa-miR-449b-3p, hsa-miR-1972, hsa-miR-1976, hsa-miR-2052, hsa-miR-449c-5p, hsa-miR-762, hsa-miR-670-5p, hsa-miR-761, hsa-miR-2116-3p, hsa-miR-548q, hsa-miR-2276-3p, hsa-miR-711, hsa-miR-718, hsa-miR-2681-3p, hsa-miR-3122, hsa-miR-3126-5p, hsa-miR-3130-3p, hsa-miR-3130-5p, hsa-miR-3131, hsa-miR-3132, hsa-miR-3133, hsa-miR-3138, hsa-miR-3141, hsa-miR-3144-5p, hsa-miR-3147, hsa-miR-3151-5p, hsa-miR-3153, hsa-miR-3074-3p, hsa-miR-3155a, hsa-miR-3156-5p, hsa-miR-3162-5p, hsa-miR-3164, hsa-miR-3173-3p, hsa-miR-1193, hsa-miR-3177-3p, hsa-miR-3178, hsa-miR-3180-3p, hsa-miR-3185, hsa-miR-3186-3p, hsa-miR-3187-3p, hsa-miR-3188, hsa-miR-3195, hsa-miR-3196, hsa-miR-3197, hsa-miR-3199, hsa-miR-3200-3p, hsa-miR-514b-5p, hsa-miR-4295, hsa-miR-4297, hsa-miR-4293, hsa-miR-4294, hsa-miR-4299, hsa-miR-4298, hsa-miR-4306, hsa-miR-4312, hsa-miR-4313, hsa-miR-4316, hsa-miR-4322, hsa-miR-4321, hsa-miR-4323, hsa-miR-4257, hsa-miR-4258, hsa-miR-4259, hsa-miR-4260, hsa-miR-4253, hsa-miR-4251, hsa-miR-4326, hsa-miR-4327, hsa-miR-4265, hsa-miR-2355-5p, hsa-miR-4269, hsa-miR-4271, hsa-miR-4276, hsa-miR-4274, hsa-miR-4281, hsa-miR-4279, hsa-miR-4278, hsa-miR-4280, hsa-miR-4284, hsa-miR-4286, hsa-miR-4288, hsa-miR-4289, hsa-miR-4290, hsa-miR-2277-5p, hsa-miR-3200-5p, hsa-miR-3610, hsa-miR-3612, hsa-miR-3614-5p, hsa-miR-3615, hsa-miR-3619-5p, hsa-miR-3621, hsa-miR-3622a-5p, hsa-miR-3622a-3p, hsa-miR-3646, hsa-miR-3648, hsa-miR-3652, hsa-miR-3663-3p, hsa-miR-3665, hsa-miR-3667-5p, hsa-miR-3675-3p, hsa-miR-3677-3p, hsa-miR-3679-5p, hsa-miR-3679-3p, hsa-miR-3688-3p, hsa-miR-3714, hsa-miR-3180, hsa-miR-3907, hsa-miR-3908, hsa-miR-3917, hsa-miR-3922-3p, hsa-miR-3928-3p, hsa-miR-3936, hsa-miR-3937, hsa-miR-3943, hsa-miR-3944-3p, hsa-miR-1268b, hsa-miR-4429, hsa-miR-4431, hsa-miR-4432, hsa-miR-4433a-3p, hsa-miR-4441, hsa-miR-4444, hsa-miR-4446-3p, hsa-miR-4447, hsa-miR-4449, hsa-miR-3135b, hsa-miR-4463, hsa-miR-4466, hsa-miR-4467, hsa-miR-4470, hsa-miR-4472, hsa-miR-4473, hsa-miR-4477b, hsa-miR-3689d, hsa-miR-4479, hsa-miR-3155b, hsa-miR-4480, hsa-miR-4483, hsa-miR-4484, hsa-miR-4486, hsa-miR-4488, hsa-miR-4489, hsa-miR-4492, hsa-miR-4494, hsa-miR-4495, hsa-miR-4496, hsa-miR-4497, hsa-miR-4499, hsa-miR-4505, hsa-miR-4508, hsa-miR-4512, hsa-miR-4513, hsa-miR-4516, hsa-miR-4521, hsa-miR-4525, hsa-miR-4526, hsa-miR-4530, hsa-miR-4531, hsa-miR-4533, hsa-miR-4534, hsa-miR-548am-3p, hsa-miR-1587, hsa-miR-4536-5p, hsa-miR-4539, hsa-miR-3120-5p, hsa-miR-3127-3p, hsa-miR-3150a-5p, hsa-miR-3158-5p, hsa-miR-3162-3p, hsa-miR-3173-5p, hsa-miR-3187-5p, hsa-miR-3619-3p, hsa-miR-3691-3p, hsa-miR-3150b-5p, hsa-miR-3940-5p, hsa-miR-3960, hsa-miR-3972, hsa-miR-3978, hsa-miR-4634, hsa-miR-4637, hsa-miR-4638-5p, hsa-miR-4639-5p, hsa-miR-4640-5p, hsa-miR-4648, hsa-miR-4649-5p, hsa-miR-4651, hsa-miR-4652-5p, hsa-miR-4655-5p, hsa-miR-4661-5p, hsa-miR-4664-5p, hsa-miR-4664-3p, hsa-miR-4665-5p, hsa-miR-4665-3p, hsa-miR-4667-3p, hsa-miR-4668-5p, hsa-miR-4673, hsa-miR-4674, hsa-miR-4684-3p, hsa-miR-4685-5p, hsa-miR-4685-3p, hsa-miR-4687-5p, hsa-miR-4687-3p, hsa-miR-1343-3p, hsa-miR-4688, hsa-miR-4690-5p, hsa-miR-4691-5p, hsa-miR-4691-3p, hsa-miR-4695-5p, hsa-miR-4697-5p, hsa-miR-4697-3p, hsa-miR-4706, hsa-miR-4707-5p, hsa-miR-4707-3p, hsa-miR-4708-5p, hsa-miR-4708-3p, hsa-miR-4715-5p, hsa-miR-4716-5p, hsa-miR-4716-3p, hsa-miR-4717-3p, hsa-miR-4722-5p, hsa-miR-4723-5p, hsa-miR-4723-3p, hsa-miR-4725-5p, hsa-miR-4725-3p, hsa-miR-4726-3p, hsa-miR-4728-5p, hsa-miR-4728-3p, hsa-miR-4730, hsa-miR-4731-5p, hsa-miR-4731-3p, hsa-miR-4734, hsa-miR-4736, hsa-miR-4738-5p, hsa-miR-4738-3p, hsa-miR-4741, hsa-miR-4745-5p, hsa-miR-4746-3p, hsa-miR-4747-5p, hsa-miR-4748, hsa-miR-4749-5p, hsa-miR-4749-3p, hsa-miR-4750-5p, hsa-miR-371b-5p, hsa-miR-371b-3p, hsa-miR-4755-3p, hsa-miR-4756-5p, hsa-miR-4758-5p, hsa-miR-4758-3p, hsa-miR-4761-3p, hsa-miR-4763-5p, hsa-miR-4763-3p, hsa-miR-4764-5p, hsa-miR-4767, hsa-miR-4769-3p, hsa-miR-4776-3p, hsa-miR-4778-5p, hsa-miR-4436b-5p, hsa-miR-4781-5p, hsa-miR-4783-3p, hsa-miR-4786-3p, hsa-miR-4787-5p, hsa-miR-4793-3p, hsa-miR-4798-3p, hsa-miR-4800-5p, hsa-miR-4800-3p, hsa-miR-4433a-5p, hsa-miR-4482-3p, hsa-miR-4536-3p, hsa-miR-5001-5p, hsa-miR-5001-3p, hsa-miR-5002-5p, hsa-miR-5002-3p, hsa-miR-5008-5p, hsa-miR-5010-5p, hsa-miR-5088-5p, hsa-miR-5090, hsa-miR-5092, hsa-miR-5190, hsa-miR-5193, hsa-miR-5195-5p, hsa-miR-5195-3p, hsa-miR-5196-5p, hsa-miR-5572, hsa-miR-5579-5p, hsa-miR-5579-3p, hsa-miR-5580-5p, hsa-miR-5587-3p, hsa-miR-5589-5p, hsa-miR-5681a, hsa-miR-5698, hsa-miR-5705, hsa-miR-211-3p, hsa-miR-345-3p, hsa-miR-584-3p, hsa-miR-652-5p, hsa-miR-1185-2-3p, hsa-miR-873-3p, hsa-miR-1247-3p, hsa-miR-3184-3p, hsa-miR-365b-5p, hsa-miR-1185-1-3p, hsa-miR-503-3p, hsa-miR-937-5p, hsa-miR-1227-5p, hsa-miR-1237-5p, hsa-miR-1238-5p, hsa-miR-3620-5p, hsa-miR-3934-3p, hsa-miR-4750-3p, hsa-miR-5739, hsa-miR-5787, hsa-miR-6068, hsa-miR-6069, hsa-miR-6071, hsa-miR-6075, hsa-miR-6076, hsa-miR-6085, hsa-miR-6086, hsa-miR-6088, hsa-miR-6089, hsa-miR-6090, hsa-miR-6124, hsa-miR-6125, hsa-miR-6126, hsa-miR-6127, hsa-miR-6131, hsa-miR-6132, hsa-miR-6133, hsa-miR-6165, hsa-miR-6501-5p, hsa-miR-6503-5p, hsa-miR-6503-3p, hsa-miR-6505-5p, hsa-miR-6511a-5p, hsa-miR-6511a-3p, hsa-miR-6512-3p, hsa-miR-6513-5p, hsa-miR-6514-3p, hsa-miR-6515-3p, hsa-miR-6715b-3p, hsa-miR-6716-5p, hsa-miR-6511b-5p, hsa-miR-6720-3p, hsa-miR-6721-5p, hsa-miR-6722-5p, hsa-miR-6722-3p, hsa-miR-6724-5p, hsa-let-7c-3p, hsa-miR-208a-5p, hsa-miR-210-5p, hsa-miR-128-1-5p, hsa-miR-133a-5p, hsa-miR-489-5p, hsa-miR-511-3p, hsa-miR-181d-3p, hsa-miR-585-5p, hsa-miR-598-5p, hsa-miR-627-3p, hsa-miR-1296-3p, hsa-miR-1301-5p, hsa-miR-874-5p, hsa-miR-216b-3p, hsa-miR-1266-3p, hsa-miR-1908-3p, hsa-miR-3151-3p, hsa-miR-3192-3p, hsa-miR-1343-5p, hsa-miR-5189-3p, hsa-miR-5699-5p, hsa-miR-6726-5p, hsa-miR-6726-3p, hsa-miR-6727-5p, hsa-miR-6728-5p, hsa-miR-6728-3p, hsa-miR-6729-5p, hsa-miR-6729-3p, hsa-miR-6730-5p, hsa-miR-6731-3p, hsa-miR-6732-5p, hsa-miR-6732-3p, hsa-miR-6734-5p, hsa-miR-6736-3p, hsa-miR-6737-5p, hsa-miR-6738-5p, hsa-miR-6738-3p, hsa-miR-6740-3p, hsa-miR-6741-3p, hsa-miR-6742-5p, hsa-miR-6742-3p, hsa-miR-6743-5p, hsa-miR-6743-3p, hsa-miR-6745, hsa-miR-6746-5p, hsa-miR-6746-3p, hsa-miR-6748-5p, hsa-miR-6749-5p, hsa-miR-6749-3p, hsa-miR-6750-5p, hsa-miR-6751-5p, hsa-miR-6752-5p, hsa-miR-6752-3p, hsa-miR-6753-5p, hsa-miR-6754-3p, hsa-miR-6756-5p, hsa-miR-6756-3p, hsa-miR-6758-5p, hsa-miR-6759-5p, hsa-miR-6759-3p, hsa-miR-6760-5p, hsa-miR-6760-3p, hsa-miR-6761-5p, hsa-miR-6761-3p, hsa-miR-6762-5p, hsa-miR-6762-3p, hsa-miR-6763-5p, hsa-miR-6763-3p, hsa-miR-6764-3p, hsa-miR-6765-5p, hsa-miR-6766-5p, hsa-miR-6766-3p, hsa-miR-6768-5p, hsa-miR-6769a-3p, hsa-miR-6770-3p, hsa-miR-6772-3p, hsa-miR-6773-3p, hsa-miR-6774-5p, hsa-miR-6775-5p, hsa-miR-6776-5p, hsa-miR-6776-3p, hsa-miR-6777-5p, hsa-miR-6777-3p, hsa-miR-6779-5p, hsa-miR-6781-5p, hsa-miR-6782-5p, hsa-miR-6782-3p, hsa-miR-6784-5p, hsa-miR-6784-3p, hsa-miR-6785-3p, hsa-miR-6786-5p, hsa-miR-6787-5p, hsa-miR-6787-3p, hsa-miR-6789-5p, hsa-miR-6789-3p, hsa-miR-6790-5p, hsa-miR-6790-3p, hsa-miR-6791-5p, hsa-miR-6792-5p, hsa-miR-6792-3p, hsa-miR-6793-3p, hsa-miR-6794-5p, hsa-miR-6795-5p, hsa-miR-6795-3p, hsa-miR-6796-3p, hsa-miR-6797-5p, hsa-miR-6797-3p, hsa-miR-6798-5p, hsa-miR-6798-3p, hsa-miR-6799-5p, hsa-miR-6799-3p, hsa-miR-6800-5p, hsa-miR-6800-3p, hsa-miR-6801-3p, hsa-miR-6802-5p, hsa-miR-6802-3p, hsa-miR-6803-5p, hsa-miR-6803-3p, hsa-miR-6804-3p, hsa-miR-6805-5p, hsa-miR-6805-3p, hsa-miR-6806-5p, hsa-miR-6807-5p, hsa-miR-6808-5p, hsa-miR-6808-3p, hsa-miR-6809-5p, hsa-miR-6809-3p, hsa-miR-6810-5p, hsa-miR-6810-3p, hsa-miR-6812-5p, hsa-miR-6812-3p, hsa-miR-6813-5p, hsa-miR-6813-3p, hsa-miR-6815-5p, hsa-miR-6816-5p, hsa-miR-6816-3p, hsa-miR-6817-3p, hsa-miR-6819-3p, hsa-miR-6820-5p, hsa-miR-6820-3p, hsa-miR-6821-5p, hsa-miR-6821-3p, hsa-miR-6822-5p, hsa-miR-6824-3p, hsa-miR-6825-5p, hsa-miR-6825-3p, hsa-miR-6828-5p, hsa-miR-6829-5p, hsa-miR-6830-5p, hsa-miR-6830-3p, hsa-miR-6832-5p, hsa-miR-6832-3p, hsa-miR-6833-5p, hsa-miR-6833-3p, hsa-miR-6780b-5p, hsa-miR-6780b-3p, hsa-miR-6836-5p, hsa-miR-6836-3p, hsa-miR-6837-3p, hsa-miR-6838-5p, hsa-miR-6840-5p, hsa-miR-6840-3p, hsa-miR-6842-5p, hsa-miR-6845-5p, hsa-miR-6845-3p, hsa-miR-6846-5p, hsa-miR-6848-5p, hsa-miR-6848-3p, hsa-miR-6849-5p, hsa-miR-6849-3p, hsa-miR-6850-5p, hsa-miR-6852-3p, hsa-miR-6855-3p, hsa-miR-6857-5p, hsa-miR-6857-3p, hsa-miR-6858-3p, hsa-miR-6859-3p, hsa-miR-6861-5p, hsa-miR-6861-3p, hsa-miR-6862-5p, hsa-miR-6862-3p, hsa-miR-6865-3p, hsa-miR-6867-5p, hsa-miR-6868-3p, hsa-miR-6869-5p, hsa-miR-6870-5p, hsa-miR-6870-3p, hsa-miR-6872-5p, hsa-miR-6873-5p, hsa-miR-6875-5p, hsa-miR-6875-3p, hsa-miR-6876-3p, hsa-miR-6877-5p, hsa-miR-6877-3p, hsa-miR-6878-3p, hsa-miR-6880-5p, hsa-miR-6880-3p, hsa-miR-6881-5p, hsa-miR-6882-3p, hsa-miR-6883-5p, hsa-miR-6884-3p, hsa-miR-6885-3p, hsa-miR-6886-5p, hsa-miR-6886-3p, hsa-miR-6887-5p, hsa-miR-6887-3p, hsa-miR-6889-5p, hsa-miR-6889-3p, hsa-miR-6890-3p, hsa-miR-6891-5p, hsa-miR-6891-3p, hsa-miR-6892-3p, hsa-miR-6893-5p, hsa-miR-6893-3p, hsa-miR-6894-3p, hsa-miR-6895-5p, hsa-miR-6895-3p, hsa-miR-7106-5p, hsa-miR-7107-5p, hsa-miR-7108-5p, hsa-miR-7108-3p, hsa-miR-7109-5p, hsa-miR-7109-3p, hsa-miR-7110-3p, hsa-miR-7111-5p, hsa-miR-7111-3p, hsa-miR-7112-5p, hsa-miR-7112-3p, hsa-miR-7113-3p, hsa-miR-7114-5p, hsa-miR-7114-3p, hsa-miR-7150, hsa-miR-7151-5p, hsa-miR-7152-5p, hsa-miR-7152-3p, hsa-miR-7155-3p, hsa-miR-7704, hsa-miR-7706, hsa-miR-7843-5p, hsa-miR-4433b-5p, hsa-miR-4433b-3p, hsa-miR-1273h-5p, hsa-miR-1273h-3p, hsa-miR-7851-3p, hsa-miR-7855-5p, hsa-miR-8059, hsa-miR-8063, hsa-miR-8064, hsa-miR-8069, hsa-miR-8072, hsa-miR-8073, hsa-miR-8078, hsa-miR-8079, hsa-miR-8080, hsa-miR-8087, hsa-miR-8089, hsa-miR-128-2-5p, hsa-miR-1199-3p, hsa-miR-7974, hsa-miR-7977, hsa-miR-7978, hsa-miR-4485-5p, hsa-miR-8485, hsa-miR-9500, hsa-miR-103a-1-5p, hsa-miR-9898, hsa-miR-9902, hsa-miR-10392-5p, hsa-miR-10392-3p, hsa-miR-10394-3p, hsa-miR-10396a-5p, hsa-miR-10396a-3p, hsa-miR-10398-5p, hsa-miR-10400-5p, hsa-miR-10400-3p, hsa-miR-10401-5p, hsa-miR-10401-3p, hsa-miR-10396b-5p, hsa-miR-10396b-3p, hsa-miR-10522-5p, hsa-miR-10526-3p, hsa-miR-11399, hsa-miR-3059-5p, hsa-miR-3059-3p, hsa-miR-3085-5p, hsa-miR-3085-3p, hsa-miR-12115, hsa-miR-12116, hsa-miR-12118, hsa-miR-12119, hsa-miR-12120, hsa-miR-12121, hsa-miR-12128, hsa-miR-12131, and hsa-miR-12135. In the present disclosure, the extract of urine may be an extract of the urine of a stage-I thyroid cancer patient. Only one of these microRNAs can be used to predict whether a subject from which the urine is derived is a thyroid cancer patient (particularly stage-I), and 5 or more types, 10 or more types, 15 or more types, or 20 or more types of these microRNAs can be used in combination to predict whether a subject from which the urine is derived is a thyroid cancer patient (particularly stage-I). Among these microRNAs, a microRNA that may be highly expressed in a thyroid cancer patient (particularly stage-I) can be preferably used for prediction.

The present disclosure provides an extract of urine containing at least one type, two types, three types, four types, five types, six types, seven types, eight types, nine types, or ten types of or all microRNAs selected from the group consisting of microRNAs having an accuracy of higher than 0.500 among the microRNAs listed in Table 24-12. In the present disclosure, the extract of urine may be an extract of the urine of a thyroid cancer patient. Only one of these microRNAs can be used to predict whether a subject from which the urine is derived is a thyroid cancer patient, and 5 or more types, 10 or more types, 15 or more types, or 20 or more types of these microRNAs can be used in combination to predict whether a subject from which the urine is derived is a thyroid cancer patient. Among these microRNAs, a microRNA that may be highly expressed in a thyroid cancer patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

The present disclosure provides an extract of urine containing at least one type, two types, three types, four types, five types, six types, seven types, eight types, nine types, or ten types of or all microRNAs selected from the group consisting of microRNAs having an accuracy of higher than 0.500 among the microRNAs listed in Table 25-12. In the present disclosure, the extract of urine may be an extract of the urine of a thyroid cancer patient. Only one of these microRNAs can be used to predict whether a subject from which the urine is derived is a thyroid cancer patient, and 5 or more types, 10 or more types, 15 or more types, or 20 or more types of these microRNAs can be used in combination to predict whether a subject from which the urine is derived is a thyroid cancer patient. Among these microRNAs, a microRNA that may be highly expressed in a thyroid cancer patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

The present disclosure provides an extract of urine containing at least one type, two types, three types, four types, five types, six types, seven types, eight types, nine types, or ten types of or all microRNAs selected from the microRNAs listed in Table 4-13. The present disclosure also provides an extract of urine containing at least one type, two types, three types, four types, five types, six types, seven types, eight types, nine types, or ten types of or all microRNAs selected from the group consisting of miR-3978, miR-6808-3p, miR-8079, miR-4280, miR-6719-3p, miR-6809-5p, miR-8080, miR-5196-5p, miR-4521, miR-6513-5p, miR-598-5p, miR-297, miR-4661-5p, miR-449a, miR-1912-3p, miR-6071, miR-3059-3p, miR-4715-5p, miR-12131, miR-1914-3p, miR-539-5p, miR-489-5p, miR-4691-3p, miR-1292-5p, miR-382-5p, miR-6829-5p, miR-4778-5p, miR-8078, miR-200a-5p, miR-320c, miR-4793-5p, miR-3130-3p, miR-4433a-3p, miR-6838-5p, miR-4321, miR-4293, miR-4522, miR-1225-5p, miR-3155a, miR-4512, miR-6839-5p, miR-6797-5p, miR-6124, miR-5693, miR-3907, miR-4271, miR-6845-3p, miR-4769-3p, miR-6501-5p, miR-2681-3p, miR-4439, miR-1208, miR-6799-5p, miR-6761-5p, miR-181d-3p, miR-4786-3p, miR-4433b-3p, miR-5584-5p, miR-1207-5p, miR-6812-5p, miR-3129-3p, miR-375-3p, miR-1269b, miR-4725-3p, miR-6780b-5p, miR-4645-3p, miR-7109-5p, miR-1299, miR-6870-5p, miR-1185-2-3p, miR-9986, miR-6516-5p, miR-4286, miR-6769b-5p, miR-1269a, miR-6069, miR-127-5p, miR-4494, miR-4800-3p, miR-6817-5p, miR-6716-5p, miR-210-5p, miR-6832-5p, miR-3651, miR-3141, miR-361-3p, miR-1185-1-3p, miR-7114-3p, miR-6775-5p, miR-6848-3p, miR-4794, miR-3943, miR-4687-5p, miR-4289, miR-7150, miR-324-5p, miR-4450, miR-5004-3p, miR-7111-5p, miR-4294, miR-1225-3p, miR-498-5p, miR-766-5p, miR-6760-3p, miR-6784-3p, miR-4323, miR-3675-3p, miR-6810-3p, miR-4451, miR-6880-3p, miR-625-3p, miR-4750-3p, miR-106a-3p, miR-1224-3p, miR-4431, miR-29b-1-5p, miR-1281, miR-4755-3p, miR-150-3p, miR-6892-3p, miR-7106-5p, miR-4298, miR-642a-3p, miR-7158-3p, miR-551b-5p, miR-6783-5p, miR-7851-3p, miR-6800-3p, miR-652-5p, miR-6795-3p, miR-6752-3p, miR-541-3p, miR-611, miR-4661-3p, miR-613, miR-6500-5p, miR-6819-3p, miR-1202, miR-4999-5p, miR-761, miR-4284, miR-4728-3p, miR-330-3p, miR-6777-3p, miR-1249-5p, miR-380-3p, miR-3162-3p, miR-6813-3p, miR-3648, miR-7706, miR-8064, miR-5006-5p, miR-1343-5p, miR-4295, miR-6763-3p, miR-4749-3p, miR-7151-5p, miR-4731-3p, miR-30b-3p, miR-5091, miR-4658, miR-642b-3p, miR-3655, miR-4706, miR-4502, miR-135a-2-3p, miR-6081, miR-4648, miR-4447, miR-4733-5p, miR-675-5p, miR-7156-3p, miR-184, miR-4713-3p, miR-619-5p, miR-3692-3p, miR-4327, miR-10526-3p, miR-3678-5p, miR-940, miR-3944-5p, miR-3614-5p, miR-12113, miR-708-5p, miR-4313, miR-4716-5p, miR-1288-3p, miR-6774-5p, miR-4665-3p, miR-6510-5p, miR-6785-3p, miR-4685-5p, miR-4727-3p, miR-8081, miR-206, miR-484, miR-1908-5p, miR-4433b-5p, miR-6746-5p, miR-1913, miR-7154-3p, miR-6880-5p, miR-6887-3p, miR-4274, miR-1321, miR-3934-3p, miR-6794-5p, miR-1286, miR-6718-5p, miR-6798-3p, miR-2115-5p, miR-3911, miR-5007-5p, miR-3937, miR-3682-3p, miR-5002-5p, miR-6749-5p, miR-338-5p, miR-5698, miR-6752-5p, miR-6861-3p, miR-3677-3p, miR-6505-3p, miR-6766-3p, miR-6805-3p, miR-6847-5p, miR-4480, miR-6512-5p, miR-137-5p, miR-6821-5p, miR-1255b-5p, miR-4279, miR-6515-5p, miR-6743-5p, miR-3162-5p, miR-671-5p, miR-145-5p, miR-937-5p, miR-1229-5p, miR-765, miR-6504-3p, miR-492, miR-1303, miR-6840-3p, miR-1227-5p, miR-1228-3p, miR-665, miR-4257, miR-4783-3p, miR-6504-5p, miR-6768-5p, miR-7161-5p, miR-6788-5p, miR-6895-5p, miR-5681b, miR-6807-5p, miR-6824-5p, miR-4465, miR-3085-3p, miR-514b-5p, miR-1268b, miR-942-5p, miR-4684-3p, miR-513b-5p, miR-4748, miR-7854-3p, miR-4433a-5p, miR-1825, miR-186-3p, miR-365a-3p, miR-365b-3p, miR-515-5p, miR-4758-3p, miR-3074-3p, miR-4258, miR-6721-5p, miR-6501-3p, miR-449c-5p, miR-6129, miR-3158-5p, miR-6083, miR-4701-3p, miR-1238-3p, miR-6503-3p, miR-6797-3p, miR-3186-3p, miR-365a-5p, miR-1226-5p, miR-6750-5p, miR-6859-3p, miR-7850-5p, miR-4441, miR-1976, miR-7107-5p, miR-4436b-3p, miR-6805-5p, miR-3918, miR-1234-3p, miR-4665-5p, miR-4478, miR-623, miR-6792-3p, miR-4803, miR-147a, miR-1249-3p, miR-7108-3p, miR-4647, miR-4723-3p, miR-4261, miR-3679-3p, miR-147b-3p, miR-557, miR-942-3p, miR-6842-3p, miR-6808-5p, miR-877-3p, miR-554, miR-9898, miR-4462, miR-4761-3p, miR-6891-5p, miR-4285, miR-4712-3p, miR-4299, miR-6798-5p, miR-125b-2-3p, miR-21-3p, miR-6779-5p, miR-593-3p, miR-10396a-3p, miR-25-5p, miR-2116-3p, miR-1910-3p, miR-6758-5p, miR-6789-5p, miR-6886-3p, miR-6804-5p, miR-6729-3p, miR-8077, miR-4316, miR-6513-3p, miR-12120, miR-3142, miR-7515, miR-4773, miR-3619-5p, miR-4717-5p, miR-3934-5p, miR-4463, miR-6816-5p, miR-4758-5p, miR-6848-5p, miR-6756-3p, miR-874-5p, miR-4492, miR-487a-5p, miR-7843-3p, miR-4707-3p, miR-4454, miR-6875-5p, miR-4632-5p, miR-4681, miR-3124-5p, miR-7856-5p, miR-4640-3p, miR-6858-3p, miR-7113-3p, miR-6787-5p, miR-4761-5p, miR-4753-5p, miR-6776-5p, miR-6740-5p, miR-6086, miR-6756-5p, miR-383-5p, miR-4697-5p, miR-3180, miR-208b-5p, miR-548as-5p, miR-658, miR-23b-3p, miR-4673, miR-10522-5p, miR-15a-3p, miR-371b-3p, miR-4763-5p, miR-6855-3p, miR-3144-5p, miR-3197, miR-3945, miR-210-3p, miR-10400-3p, miR-550a-3-5p, miR-3198, miR-1469, miR-5681a, miR-196b-3p, miR-3187-3p, miR-6749-3p, miR-7113-5p, miR-5194, miR-4311, miR-6766-5p, miR-363-5p, miR-4741, miR-146a-5p, miR-1972, miR-4474-3p, miR-10394-5p, miR-106b-5p, miR-6732-5p, miR-6509-5p, miR-3922-5p, miR-328-3p, miR-3199, miR-3184-3p, miR-6889-3p, miR-6840-5p, miR-4538, miR-6791-5p, miR-18b-3p, miR-6782-5p, miR-1275, miR-6862-3p, miR-6825-5p, miR-3529-5p, miR-654-3p, miR-6796-3p, miR-6814-5p, miR-12119, miR-542-5p, miR-5195-3p, miR-4731-5p, miR-6759-3p, miR-4484, miR-4472, miR-3158-3p, miR-6715b-3p, miR-4716-3p, miR-3679-5p, miR-6801-3p, miR-7847-3p, miR-718, miR-7974, miR-196a-5p, miR-4738-5p, miR-92a-2-5p, miR-3138, miR-6165, miR-485-5p, miR-6790-5p, miR-5739, miR-3165, miR-579-5p, miR-378f, miR-6854-3p, miR-139-3p, miR-493-3p, miR-4768-5p, miR-4749-5p, miR-1298-3p, miR-1270, miR-6831-5p, miR-211-3p, miR-4747-5p, miR-6836-3p, miR-6802-3p, miR-135a-3p, miR-6720-3p, miR-298, miR-3173-5p, miR-9718, miR-92b-5p, miR-5010-5p, miR-6731-3p, miR-5703, miR-646, miR-6857-3p, miR-6856-5p, miR-149-3p, miR-921, miR-525-3p, miR-4420, miR-1267, miR-4712-5p, miR-483-3p, miR-5702, miR-30c-1-3p, miR-7110-5p, miR-608, miR-10396a-5p, miR-3157-5p, miR-1237-3p, miR-4769-5p, miR-4728-5p, miR-4473, miR-7110-3p, miR-6876-5p, miR-5094, miR-4682, miR-3180-3p, miR-6769a-3p, miR-7108-5p, miR-6742-5p, miR-6823-5p, miR-4530, miR-4634, miR-3649, miR-3689b-3p, miR-3689c, miR-601, miR-4651, miR-128-1-5p, miR-6892-5p, miR-3059-5p, miR-1236-5p, miR-6764-3p, miR-3654, miR-323a-3p, miR-6817-3p, miR-6741-3p, miR-622, miR-4540, miR-4525, miR-452-3p, miR-6894-3p, miR-2276-3p, miR-18a-3p, miR-663b, miR-647, miR-3926, miR-5685, miR-4707-5p, miR-4496, miR-4466, miR-12114, miR-197-3p, miR-1298-5p, miR-4479, miR-6730-5p, miR-7975, miR-6717-5p, miR-8075, miR-4686, miR-330-5p, miR-605-3p, miR-6803-3p, miR-6809-3p, miR-6865-5p, miR-6764-5p, miR-122b-3p, miR-7111-3p, miR-3135b, miR-6724-5p, miR-6515-3p, miR-6800-5p, miR-5584-3p, miR-146b-3p, miR-4691-5p, miR-575, miR-6722-3p, miR-6833-5p, miR-4505, miR-4267, miR-663a, miR-4537, miR-6772-5p, miR-4534, miR-7109-3p, miR-372-3p, miR-4745-5p, miR-12122, miR-494-5p, miR-6845-5p, miR-7114-5p, miR-1229-3p, miR-8070, miR-378i, miR-370-5p, miR-451b, miR-6768-3p, miR-1915-3p, miR-7162-3p, miR-937-3p, miR-4529-5p, miR-103a-2-5p, miR-6759-5p, miR-7844-5p, miR-6830-5p, miR-10398-5p, miR-6740-3p, miR-483-5p, miR-8059, miR-1293, miR-4265, miR-4455, miR-711, miR-4322, miR-6891-3p, miR-7157-3p, miR-6743-3p, miR-6767-5p, miR-2052, miR-5579-3p, miR-3161, miR-8065, miR-5579-5p, miR-511-3p, miR-4708-3p, miR-3150b-3p, miR-627-3p, miR-371a-5p, miR-193a-5p, miR-5187-3p, miR-8089, miR-3908, miR-4640-5p, miR-5003-3p, miR-4740-5p, miR-6885-3p, miR-3681-5p, miR-6737-5p, miR-5189-3p, miR-3610, miR-1180-3p, miR-6751-3p, miR-6792-5p, miR-5006-3p, miR-6132, miR-3154, miR-514b-3p, miR-1251-3p, miR-10398-3p, miR-6870-3p, miR-5002-3p, miR-6806-5p, miR-432-5p, miR-6876-3p, miR-6738-5p, miR-8485, miR-6751-5p, miR-6732-3p, miR-222-5p, miR-1468-5p, miR-216b-3p, miR-373-3p, miR-4685-3p, miR-181a-2-3p, miR-1471, miR-4676-3p, miR-4252, miR-4520-5p, miR-25-3p, miR-3929, miR-3622a-5p, miR-214-5p, miR-6861-5p, miR-4489, miR-3667-5p, miR-4497, miR-4326, miR-5001-5p, miR-4768-3p, miR-3927-5p, miR-5580-5p, miR-6715a-3p, miR-3692-5p, miR-4506, miR-11400, miR-1238-5p, miR-3689a-3p, miR-3186-5p, miR-4664-3p, miR-6780a-5p, miR-3917, miR-296-5p, miR-4498, miR-501-5p, miR-6868-3p, miR-6125, miR-6750-3p, miR-6821-3p, miR-4672, miR-1180-5p, miR-659-3p, miR-3163, miR-6511b-3p, miR-6871-5p, miR-3155b, miR-760, miR-939-5p, miR-6804-3p, miR-518c-5p, miR-5190, miR-6512-3p, miR-4750-5p, miR-4483, miR-604, miR-6842-5p, miR-4288, miR-1264, miR-6865-3p, miR-202-3p, miR-4519, miR-6877-3p, miR-6769a-5p, miR-301b-5p, miR-6511a-3p, miR-1247-3p, miR-6837-5p, miR-7155-3p, miR-4644, miR-325, miR-6728-3p, miR-2392, miR-6507-5p, miR-378g, miR-10523-5p, miR-6849-5p, miR-5585-3p, miR-769-3p, miR-7160-5p, miR-6720-5p, miR-6823-3p, miR-595, miR-4776-3p, miR-4436b-5p, miR-8060, miR-525-5p, miR-2467-3p, miR-3922-3p, miR-6826-3p, miR-6820-3p, miR-5699-5p, miR-6068, miR-943, miR-4303, miR-1323, miR-1260a, miR-140-3p, miR-181c-3p, miR-6761-3p, miR-4763-3p, miR-4804-5p, miR-1343-3p, miR-197-5p, miR-6742-3p, miR-4481, miR-766-3p, let-7d-3p, miR-4535, miR-3184-5p, miR-12118, miR-4667-3p, miR-6855-5p, miR-877-5p, miR-6133, miR-29c-5p, let-7e-5p, miR-423-5p, miR-4470, miR-3151-5p, miR-9902, miR-5690, miR-3689d, miR-3194-5p, miR-3173-3p, miR-2467-5p, miR-4703-3p, miR-4698, miR-6502-5p, miR-6787-3p, miR-378b, miR-6793-3p, miR-6131, miR-6884-3p, miR-564, miR-500b-3p, miR-4675, miR-6765-5p, miR-219a-2-3p, miR-4722-5p, miR-4444, miR-4687-3p, miR-6833-3p, miR-6736-3p, miR-6747-5p, miR-3619-3p, miR-6080, miR-3151-3p, miR-433-5p, miR-3120-5p, miR-4697-3p, miR-5708, miR-574-3p, miR-6834-5p, miR-5196-3p, miR-6867-5p, miR-103a-1-5p, miR-4756-3p, miR-661, miR-6734-5p, miR-6072, miR-5585-5p, miR-11181-3p, miR-3127-3p, miR-6776-3p, miR-585-5p, miR-8072, miR-373-5p, miR-6511b-5p, miR-4482-3p, miR-3202, miR-449b-3p, miR-6753-5p, miR-632, miR-4788, miR-10399-3p, miR-6802-5p, miR-6793-5p, miR-4781-5p, miR-4485-5p, miR-3132, miR-12121, miR-3620-5p, miR-4449, miR-3200-5p, miR-3675-5p, miR-584-3p, miR-4738-3p, miR-4783-5p, miR-770-5p, miR-4695-5p, miR-378c, miR-10401-5p, miR-4726-3p, miR-5589-5p, miR-4646-3p, miR-1236-3p, miR-6773-3p, miR-194-3p, miR-503-3p, miR-8074, miR-6500-3p, miR-767-5p, miR-3074-5p, miR-5193, miR-519b-3p, miR-516b-5p, miR-3691-3p, miR-11401, miR-892b, miR-4526, miR-7159-5p, miR-449b-5p, miR-6839-3p, miR-3147, miR-378j, miR-8083, miR-3652, miR-6727-3p, miR-6511a-5p, miR-1253, miR-3615, miR-301a-5p, miR-3612, miR-550a-5p, miR-5189-5p, miR-10392-5p, miR-4732-5p, miR-4458, miR-6893-3p, miR-2277-5p, miR-24-2-5p, miR-1178-3p, miR-4700-5p, miR-5195-5p, miR-6070, miR-4676-5p, miR-5004-5p, miR-516a-5p, miR-6728-5p, miR-6890-3p, miR-5090, miR-3175, miR-4456, miR-1268a, miR-12116, miR-6858-5p, miR-744-3p, miR-1909-3p, miR-642b-5p, miR-4486, miR-6879-5p, miR-128-3p, miR-6846-5p, miR-6795-5p, miR-6499-3p, miR-6893-5p, miR-3187-5p, miR-650, miR-4802-3p, miR-4713-5p, miR-924, miR-638, miR-6778-5p, miR-320e, miR-3085-5p, miR-3131, miR-4453, miR-589-3p, miR-7845-5p, miR-4487, miR-1273h-3p, miR-6849-3p, miR-6726-5p, miR-6738-3p, miR-6895-3p, miR-3659, miR-6748-5p, miR-630, miR-6772-3p, miR-504-3p, miR-6781-5p, miR-513c-5p, miR-6726-3p, miR-3605-5p, miR-11399, miR-6791-3p, miR-4743-3p, miR-4653-5p, miR-6832-3p, miR-892a, miR-7158-5p, miR-3125, miR-4740-3p, miR-4654, miR-378a-3p, miR-4655-5p, miR-4784, miR-6716-3p, miR-3193, miR-4436a, miR-939-3p, miR-6879-3p, miR-6786-3p, miR-8088, miR-486-5p, miR-320b, miR-6859-5p, miR-3120-3p, miR-6894-5p, miR-6729-5p, miR-8071, miR-4727-5p, miR-1306-5p, miR-3190-3p, miR-4475, miR-4756-5p, miR-6780b-3p, miR-885-3p, miR-5696, miR-6889-5p, miR-365b-5p, miR-6819-5p, miR-6881-5p, miR-1306-3p, miR-3170, miR-6852-5p, miR-1288-5p, miR-487b-5p, miR-6127, miR-450a-5p, miR-6837-3p, miR-4656, miR-134-3p, miR-4724-5p, miR-668-5p, miR-4754, miR-4440, miR-4694-3p, miR-3065-3p, miR-4428, miR-3646, miR-1266-3p, miR-6820-5p, miR-4508, miR-378e, miR-3122, miR-1237-5p, miR-5093, miR-1203, miR-4755-5p, miR-7112-3p, miR-3153, miR-6762-3p, miR-617, miR-509-5p, miR-3144-3p, miR-548as-3p, miR-4495, miR-4637, miR-548ah-5p, miR-6789-3p, miR-548al, miR-204-3p, miR-4800-5p, miR-3713, miR-6763-5p, miR-300, miR-4722-3p, miR-889-5p, miR-149-5p, miR-490-5p, miR-629-5p, miR-12117, miR-6813-5p, miR-3188, miR-6782-3p, miR-631, miR-125b-1-3p, miR-574-5p, miR-4467, miR-519c-5p, miR-523-5p, miR-518e-5p, miR-522-5p, miR-519a-5p, miR-519b-5p, miR-8087, miR-4785, miR-193b-5p, miR-183-3p, miR-449c-3p, miR-3690, miR-4737, miR-6505-5p, miR-1231, miR-541-5p, miR-6850-5p, miR-8073, miR-6852-3p, miR-4523, miR-4664-5p, miR-7976, miR-7112-5p, miR-891a-5p, miR-6835-5p, miR-5572, miR-1587, miR-6851-5p, miR-4780, miR-1204, miR-634, miR-6822-3p, miR-4514, miR-4318, miR-6816-3p, miR-20b-3p, miR-7843-5p, miR-4260, miR-6741-5p, miR-12128, miR-6130, miR-2682-5p, miR-6843-3p, miR-1304-3p, miR-4448, miR-4726-5p, miR-548g-3p, miR-6786-5p, miR-320a-3p, miR-6824-3p, miR-6801-5p, miR-3149, miR-6810-5p, miR-1538, miR-6754-3p, miR-4762-3p, miR-6846-3p, miR-3064-5p, miR-4513, miR-7107-3p, miR-12124, miR-1233-3p, miR-7152-5p, miR-4730, miR-548ao-3p, miR-6886-5p, miR-6856-3p, miR-4319, miR-6748-3p, miR-4639-3p, miR-1182, miR-3160-5p, miR-3617-3p, miR-4725-5p, miR-4736, miR-2110, miR-9500, miR-3928-3p, miR-138-5p, miR-4667-5p, miR-6863, miR-1307-3p, miR-4435, miR-4268, miR-3622a-3p, miR-4724-3p, miR-4695-3p, miR-572, miR-7106-3p, miR-6796-5p, miR-4787-3p, miR-3940-3p, miR-6783-3p, miR-3196, miR-936, miR-6773-5p, miR-670-5p, miR-6860, miR-10392-3p, miR-1184, miR-675-3p, miR-551a, miR-129-5p, miR-4296, miR-875-3p, miR-6735-3p, miR-491-5p, miR-497-5p, miR-1250-5p, miR-550b-3p, miR-4281, miR-133b, miR-758-5p, miR-4751, miR-4507, miR-4297, miR-5787, miR-3185, miR-6877-5p, miR-3178, miR-4304, miR-5684, miR-885-5p, miR-571, miR-4793-3p, miR-6126, miR-4674, miR-1843, miR-345-5p, miR-4786-5p, miR-1193, miR-6134, miR-671-3p, miR-6803-5p, miR-6883-3p, miR-466, miR-5588-3p, miR-6873-5p, miR-3620-3p, miR-135a-5p, miR-2355-5p, miR-1301-5p, miR-12115, miR-6076, miR-339-3p, miR-296-3p, miR-609, miR-1266-5p, miR-4283, miR-188-5p, miR-31-3p, miR-4315, miR-1273h-5p, miR-6735-5p, miR-214-3p, miR-517a-3p, miR-517b-3p, miR-6777-5p, miR-660-3p, let-7c-3p, miR-7853-5p, miR-4710, miR-223-5p, miR-4692, miR-6746-3p, miR-920, miR-10396b-5p, miR-3936, miR-7155-5p, miR-4253, miR-7848-3p, miR-7152-3p, miR-3605-3p, miR-2116-5p, miR-5088-5p, miR-6753-3p, miR-551b-3p, miR-548q, miR-196a-1-3p, miR-7855-5p, miR-4688, miR-668-3p, miR-8057, miR-3192-5p, miR-4421, miR-4711-3p, miR-4669, miR-7151-3p, miR-34b-3p, miR-10394-3p, miR-1287-3p, miR-6757-3p, miR-4709-3p, miR-3130-5p, miR-1909-5p, miR-3127-5p, miR-23a-5p, miR-6075, miR-4324, miR-4251, miR-421, miR-941, miR-1285-5p, miR-370-3p, miR-4429, miR-6739-3p, miR-6715b-5p, miR-656-5p, miR-3137, miR-7849-3p, miR-6085, miR-4287, miR-30a-3p, miR-3666, miR-6771-5p, miR-4649-5p, miR-6887-5p, miR-1291, miR-185-3p, miR-26b-3p, miR-6825-3p, miR-3195, miR-2113, miR-6078, miR-6744-5p, miR-3678-3p, miR-379-3p, miR-6722-5p, miR-1284, miR-96-3p, miR-6784-5p, miR-6878-5p, miR-4717-3p, miR-198, miR-6830-3p, miR-6514-3p, miR-323a-5p, miR-4721, and miR-654-5p. In the present disclosure, the extract of urine may be an extract of the urine of a cervical cancer patient. Only one of these microRNAs can be used to predict whether a subject from which the urine is derived is a cervical cancer patient, and 5 or more types, 10 or more types, 15 or more types, or 20 or more types of these microRNAs can be used in combination to predict whether a subject from which the urine is derived is a cervical cancer patient. Among these microRNAs, a microRNA that may be highly expressed in a cervical cancer patient can be preferably used for prediction.

The present disclosure provides an extract of urine containing at least one type, two types, three types, four types, five types, six types, seven types, eight types, nine types, or ten types of or all microRNAs selected from the group consisting of miR-25-3p, miR-196a-5p, miR-197-3p, miR-198, miR-30d-5p, miR-139-5p, miR-210-3p, miR-214-3p, miR-124-3p, miR-125b-5p, miR-134-5p, miR-149-5p, miR-188-5p, miR-320a-3p, miR-106b-5p, miR-296-5p, miR-365a-3p, miR-365b-3p, miR-302c-5p, miR-370-3p, miR-373-5p, miR-373-3p, miR-375-3p, miR-379-5p, miR-380-3p, miR-383-5p, miR-328-3p, miR-324-3p, miR-133b, miR-346, miR-449a, miR-191-3p, miR-200a-5p, miR-483-3p, miR-484, miR-485-3p, miR-486-5p, miR-491-5p, miR-492, miR-493-5p, miR-498-5p, miR-519b-3p, miR-518c-5p, miR-519d-3p, miR-516b-5p, miR-503-5p, miR-513a-5p, miR-539-5p, miR-551a, miR-554, miR-92b-3p, miR-557, miR-564, miR-571, miR-574-3p, miR-575, miR-550a-3p, miR-611, miR-612, miR-613, miR-615-3p, miR-632, miR-634, miR-636, miR-637, miR-638, miR-652-3p, miR-661, miR-663a, miR-654-5p, miR-657, miR-658, miR-659-3p, miR-542-5p, miR-671-5p, miR-668-3p, miR-766-3p, miR-765, miR-675-5p, miR-297, miR-22-5p, miR-24-2-5p, miR-25-5p, miR-92a-2-5p, miR-29b-1-5p, miR-139-3p, miR-181a-2-3p, miR-181c-3p, miR-183-3p, miR-187-5p, miR-23b-5p, miR-30b-3p, miR-124-5p, miR-125b-1-3p, miR-135a-3p, miR-140-3p, miR-143-5p, miR-145-3p, miR-125a-3p, miR-127-5p, miR-149-3p, miR-150-3p, miR-185-3p, miR-186-3p, miR-194-3p, miR-30c-1-3p, miR-296-3p, miR-361-3p, miR-371a-5p, miR-339-3p, miR-423-5p, miR-18b-3p, miR-20b-3p, miR-431-3p, miR-483-5p, miR-486-3p, miR-490-5p, miR-505-5p, miR-509-5p, miR-92b-5p, miR-574-5p, miR-550a-5p, miR-615-5p, miR-624-3p, miR-625-3p, miR-629-5p, miR-671-3p, miR-298, miR-874-3p, miR-891b, miR-541-5p, miR-744-5p, miR-744-3p, miR-885-5p, miR-885-3p, miR-877-5p, miR-877-3p, miR-887-3p, miR-665, miR-760, miR-920, miR-936, miR-937-3p, miR-939-5p, miR-940, miR-943, miR-1224-3p, miR-1225-5p, miR-1225-3p, miR-1226-3p, miR-1228-3p, miR-1229-3p, miR-1231, miR-1233-3p, miR-1234-3p, miR-1236-3p, miR-1237-3p, miR-1238-3p, miR-1264, miR-320b, miR-320c, miR-1323, miR-1298-5p, miR-1180-3p, miR-1181, miR-1182, miR-1184, miR-1200, miR-1202, miR-1203, miR-663b, miR-1204, miR-1207-5p, miR-1207-3p, miR-1208, miR-1299, miR-1249-3p, miR-1253, miR-1255a, miR-1260a, miR-1266-5p, miR-1267, miR-1268a, miR-1272, miR-1275, miR-1281, miR-1292-5p, miR-1255b-5p, miR-1307-3p, miR-1825, miR-675-3p, miR-1469, miR-1538, miR-1539, miR-1908-5p, miR-1909-5p, miR-1909-3p, miR-1910-5p, miR-1911-5p, miR-1912-3p, miR-1913, miR-1914-3p, miR-1915-3p, miR-365a-5p, miR-196b-3p, miR-449b-3p, miR-2113, miR-1972, miR-1976, miR-2052, miR-2110, miR-762, miR-670-5p, miR-764, miR-2116-3p, miR-548q, miR-2276-3p, miR-711, miR-718, miR-2681-3p, miR-449c-3p, miR-3120-3p, miR-3122, miR-3130-5p, miR-3131, miR-3132, miR-3138, miR-3141, miR-3144-5p, miR-3147, miR-548v, miR-3151-5p, miR-3153, miR-3154, miR-3155a, miR-3156-5p, miR-3157-5p, miR-3158-3p, miR-3162-5p, miR-3163, miR-3165, miR-1260b, miR-3173-3p, miR-1193, miR-3175, miR-3177-3p, miR-3178, miR-3180-3p, miR-3182, miR-3184-5p, miR-3185, miR-3186-3p, miR-3188, miR-3191-3p, miR-3194-5p, miR-3195, miR-3196, miR-3197, miR-3198, miR-514b-5p, miR-3202, miR-3065-3p, miR-4297, miR-4293, miR-4294, miR-4299, miR-4298, miR-4305, miR-4313, miR-4316, miR-4322, miR-4321, miR-4323, miR-4324, miR-4257, miR-4258, miR-4259, miR-4260, miR-4253, miR-4251, miR-4254, miR-4325, miR-4326, miR-4327, miR-4265, miR-4266, miR-2355-5p, miR-4268, miR-4269, miR-4270, miR-4271, miR-4276, miR-4274, miR-4281, miR-4279, miR-4278, miR-4285, miR-4284, miR-4286, miR-4287, miR-4288, miR-4289, miR-4290, miR-4329, miR-2277-5p, miR-3200-5p, miR-3605-3p, miR-3610, miR-3612, miR-3614-5p, miR-3615, miR-3619-5p, miR-3620-3p, miR-3621, miR-3622a-5p, miR-3622a-3p, miR-3622b-5p, miR-3646, miR-3648, miR-3649, miR-3652, miR-3663-5p, miR-3663-3p, miR-3665, miR-3667-5p, miR-3667-3p, miR-3675-3p, miR-3677-3p, miR-3679-5p, miR-3679-3p, miR-3681-5p, miR-3682-3p, miR-3692-3p, miR-3714, miR-3180, miR-3907, miR-3908, miR-3911, miR-3917, miR-3922-3p, miR-3926, miR-3928-3p, miR-3934-5p, miR-3935, miR-3937, miR-3940-3p, miR-3943, miR-3944-3p, miR-3945, miR-642b-3p, miR-550b-3p, miR-1268b, miR-378f, miR-4428, miR-4430, miR-4433a-3p, miR-4439, miR-4440, miR-4441, miR-4444, miR-4446-3p, miR-4447, miR-4448, miR-4449, miR-548ah-5p, miR-4455, miR-3135b, miR-4462, miR-4463, miR-4466, miR-4467, miR-4470, miR-4472, miR-4473, miR-4475, miR-4476, miR-4478, miR-3689d, miR-3155b, miR-4480, miR-4481, miR-4483, miR-4484, miR-4486, miR-4489, miR-548al, miR-4492, miR-4495, miR-4496, miR-4497, miR-4498, miR-4499, miR-4505, miR-2392, miR-4507, miR-4508, miR-4512, miR-4513, miR-4516, miR-4519, miR-4521, miR-1269b, miR-4522, miR-4523, miR-4524a-3p, miR-4525, miR-4526, miR-4530, miR-4533, miR-4534, miR-1587, miR-4538, miR-4539, miR-3120-5p, miR-3127-3p, miR-3156-3p, miR-3158-5p, miR-3162-3p, miR-3173-5p, miR-3187-5p, miR-3619-3p, miR-3691-3p, miR-3922-5p, miR-3944-5p, miR-3972, miR-3977, miR-3978, miR-4634, miR-4637, miR-4638-5p, miR-4639-3p, miR-4640-5p, miR-4640-3p, miR-4645-5p, miR-4646-5p, miR-4646-3p, miR-4648, miR-4649-5p, miR-4651, miR-4655-5p, miR-4656, miR-4661-5p, miR-4661-3p, miR-4664-5p, miR-4664-3p, miR-4665-5p, miR-4665-3p, miR-4667-5p, miR-4667-3p, miR-4668-5p, miR-4669, miR-4674, miR-4675, miR-4682, miR-4685-5p, miR-4685-3p, miR-4687-5p, miR-4687-3p, miR-1343-3p, miR-4688, miR-4689, miR-4690-5p, miR-4691-5p, miR-4691-3p, miR-4695-5p, miR-4695-3p, miR-4697-5p, miR-4697-3p, miR-4698, miR-4700-3p, miR-4706, miR-4707-5p, miR-4707-3p, miR-4708-5p, miR-4708-3p, miR-4709-3p, miR-4710, miR-4712-3p, miR-4713-5p, miR-4713-3p, miR-4714-5p, miR-4715-5p, miR-4716-5p, miR-4716-3p, miR-4717-3p, miR-4721, miR-4722-5p, miR-4722-3p, miR-4723-5p, miR-4723-3p, miR-451b, miR-4725-5p, miR-4725-3p, miR-4726-5p, miR-4726-3p, miR-4728-5p, miR-4728-3p, miR-4730, miR-4731-5p, miR-4731-3p, miR-4732-5p, miR-4732-3p, miR-4733-5p, miR-4736, miR-4738-5p, miR-4738-3p, miR-4739, miR-4740-3p, miR-4741, miR-4743-5p, miR-4745-5p, miR-4746-3p, miR-4747-5p, miR-4748, miR-4749-5p, miR-4749-3p, miR-4750-5p, miR-4751, miR-4753-5p, miR-371b-5p, miR-371b-3p, miR-4755-3p, miR-4756-5p, miR-4758-5p, miR-4758-3p, miR-4761-5p, miR-4762-3p, miR-4763-5p, miR-4763-3p, miR-4769-3p, miR-4778-5p, miR-4779, miR-4780, miR-4436b-5p, miR-4781-5p, miR-4783-3p, miR-2467-5p, miR-2467-3p, miR-4787-3p, miR-4788, miR-4793-3p, miR-4800-5p, miR-4800-3p, miR-4802-3p, miR-4803, miR-4804-5p, miR-642a-3p, miR-4433a-5p, miR-4482-3p, miR-4999-5p, miR-5001-5p, miR-5001-3p, miR-5002-3p, miR-5003-3p, miR-548ao-3p, miR-5006-5p, miR-5006-3p, miR-5008-5p, miR-5010-5p, miR-5088-5p, miR-5090, miR-5094, miR-5187-3p, miR-5193, miR-5195-5p, miR-5195-3p, miR-5196-5p, miR-5196-3p, miR-5100, miR-5572, miR-548as-5p, miR-548as-3p, miR-5579-5p, miR-5579-3p, miR-5584-5p, miR-5584-3p, miR-5585-5p, miR-5586-3p, miR-5589-5p, miR-5684, miR-5685, miR-5690, miR-5693, miR-5695, miR-5698, miR-5705, miR-197-5p, miR-204-3p, miR-211-3p, miR-345-3p, miR-514a-5p, miR-584-3p, miR-652-5p, miR-1185-2-3p, miR-873-3p, miR-1304-3p, miR-1247-3p, miR-1306-5p, miR-3184-3p, miR-3191-5p, miR-642b-5p, miR-365b-5p, miR-1185-1-3p, miR-503-3p, miR-376a-2-5p, miR-937-5p, miR-939-3p, miR-1227-5p, miR-1229-5p, miR-1233-5p, miR-1237-5p, miR-1238-5p, miR-1292-3p, miR-3620-5p, miR-4632-5p, miR-4743-3p, miR-4750-3p, miR-5739, miR-5787, miR-6068, miR-6069, miR-6071, miR-6075, miR-6076, miR-6077, miR-6078, miR-6083, miR-6085, miR-6086, miR-6090, miR-6124, miR-6125, miR-6126, miR-6127, miR-378j, miR-6131, miR-6132, miR-6133, miR-6165, miR-6500-5p, miR-6501-5p, miR-6501-3p, miR-6503-5p, miR-6505-5p, miR-6505-3p, miR-6507-5p, miR-6509-3p, miR-6510-5p, miR-6511a-5p, miR-6511a-3p, miR-6512-5p, miR-6512-3p, miR-6513-5p, miR-6514-3p, miR-6515-3p, miR-6715b-3p, miR-6716-5p, miR-6716-3p, miR-6511b-5p, miR-6511b-3p, miR-6718-5p, miR-6719-3p, miR-6720-3p, miR-6721-5p, miR-6722-5p, miR-6722-3p, miR-6724-5p, miR-210-5p, miR-128-1-5p, miR-152-5p, miR-328-5p, miR-489-5p, miR-511-3p, miR-181d-3p, miR-504-3p, miR-487b-5p, miR-585-5p, miR-598-5p, miR-619-5p, miR-627-3p, miR-1296-3p, miR-874-5p, miR-887-5p, miR-216b-3p, miR-208b-5p, miR-1287-3p, miR-1250-3p, miR-1266-3p, miR-3151-3p, miR-3192-3p, miR-1343-5p, miR-5088-3p, miR-5189-3p, miR-5699-5p, miR-6726-5p, miR-6726-3p, miR-6727-5p, miR-6727-3p, miR-6728-5p, miR-6728-3p, miR-6729-5p, miR-6729-3p, miR-6730-5p, miR-6730-3p, miR-6731-3p, miR-6732-5p, miR-6732-3p, miR-6734-5p, miR-6735-5p, miR-6735-3p, miR-6736-5p, miR-6736-3p, miR-6737-5p, miR-6738-5p, miR-6738-3p, miR-6740-5p, miR-6740-3p, miR-6741-5p, miR-6741-3p, miR-6742-5p, miR-6742-3p, miR-6743-5p, miR-6743-3p, miR-6744-5p, miR-6744-3p, miR-6745, miR-6746-5p, miR-6746-3p, miR-6747-3p, miR-6748-5p, miR-6748-3p, miR-6749-5p, miR-6749-3p, miR-6750-3p, miR-6751-5p, miR-6751-3p, miR-6752-5p, miR-6752-3p, miR-6753-5p, miR-6753-3p, miR-6754-5p, miR-6754-3p, miR-6756-5p, miR-6756-3p, miR-6757-5p, miR-6757-3p, miR-6758-5p, miR-6758-3p, miR-6759-5p, miR-6759-3p, miR-6760-5p, miR-6760-3p, miR-6761-3p, miR-6762-5p, miR-6762-3p, miR-6763-5p, miR-6763-3p, miR-6764-3p, miR-6765-5p, miR-6766-5p, miR-6766-3p, miR-6768-5p, miR-6769a-5p, miR-6769a-3p, miR-6771-5p, miR-6772-5p, miR-6772-3p, miR-6773-5p, miR-6773-3p, miR-6774-5p, miR-6774-3p, miR-6775-5p, miR-6776-5p, miR-6776-3p, miR-6777-5p, miR-6777-3p, miR-6778-5p, miR-6778-3p, miR-6779-5p, miR-6779-3p, miR-6780a-5p, miR-6780a-3p, miR-6781-5p, miR-6782-5p, miR-6782-3p, miR-6783-5p, miR-6783-3p, miR-6784-5p, miR-6784-3p, miR-6785-3p, miR-6786-5p, miR-6786-3p, miR-6787-5p, miR-6787-3p, miR-6788-5p, miR-6789-5p, miR-6789-3p, miR-6790-5p, miR-6791-5p, miR-6792-5p, miR-6792-3p, miR-6793-3p, miR-6794-5p, miR-6794-3p, miR-6795-5p, miR-6795-3p, miR-6796-5p, miR-6796-3p, miR-6797-5p, miR-6797-3p, miR-6798-5p, miR-6798-3p, miR-6799-5p, miR-6799-3p, miR-6800-5p, miR-6800-3p, miR-6801-3p, miR-6802-5p, miR-6802-3p, miR-6803-5p, miR-6803-3p, miR-6804-3p, miR-6805-5p, miR-6805-3p, miR-6806-5p, miR-6807-5p, miR-6808-5p, miR-6808-3p, miR-6809-5p, miR-6809-3p, miR-6810-5p, miR-6810-3p, miR-6812-5p, miR-6813-5p, miR-6813-3p, miR-6815-5p, miR-6815-3p, miR-6816-5p, miR-6816-3p, miR-6817-3p, miR-6819-5p, miR-6819-3p, miR-6820-5p, miR-6820-3p, miR-6821-5p, miR-6821-3p, miR-6822-5p, miR-6823-3p, miR-6824-5p, miR-6824-3p, miR-6825-5p, miR-6825-3p, miR-6826-5p, miR-6826-3p, miR-6827-5p, miR-6828-5p, miR-6829-5p, miR-6829-3p, miR-6830-5p, miR-6830-3p, miR-6831-5p, miR-6832-5p, miR-6832-3p, miR-6833-5p, miR-6833-3p, miR-6835-5p, miR-6780b-5p, miR-6780b-3p, miR-6836-3p, miR-6837-5p, miR-6838-5p, miR-6839-5p, miR-6839-3p, miR-6840-5p, miR-6840-3p, miR-6842-5p, miR-6845-5p, miR-6845-3p, miR-6846-5p, miR-6846-3p, miR-6847-3p, miR-6848-5p, miR-6848-3p, miR-6849-5p, miR-6849-3p, miR-6850-5p, miR-6851-5p, miR-6851-3p, miR-6852-3p, miR-6855-5p, miR-6855-3p, miR-6856-5p, miR-6857-5p, miR-6857-3p, miR-6858-5p, miR-6858-3p, miR-6859-3p, miR-6769b-5p, miR-6769b-3p, miR-6860, miR-6861-5p, miR-6861-3p, miR-6862-3p, miR-6865-5p, miR-6865-3p, miR-6867-5p, miR-6867-3p, miR-6869-5p, miR-6870-5p, miR-6870-3p, miR-6872-5p, miR-6873-5p, miR-6873-3p, miR-6875-5p, miR-6875-3p, miR-6876-3p, miR-6877-5p, miR-6877-3p, miR-6878-5p, miR-6878-3p, miR-6879-5p, miR-6879-3p, miR-6880-5p, miR-6880-3p, miR-6881-5p, miR-6881-3p, miR-6882-3p, miR-6883-5p, miR-6883-3p, miR-6884-3p, miR-6885-3 p, miR-6886-5p, miR-6886-3p, miR-6887-5p, miR-6887-3p, miR-6889-5p, miR-6889-3p, miR-6890-5p, miR-6890-3p, miR-6891-5p, miR-6891-3p, miR-6892-5p, miR-6892-3p, miR-6893-5p, miR-6893-3p, miR-6894-5p, miR-6894-3p, miR-6895-3p, miR-7106-5p, miR-7106-3p, miR-7107-5p, miR-7108-5p, miR-7108-3p, miR-7109-5p, miR-7109-3p, miR-7110-5p, miR-7110-3p, miR-7111-5p, miR-7111-3p, miR-7112-5p, miR-7113-3p, miR-7114-5p, miR-7114-3p, miR-7150, miR-7151-5p, miR-7152-5p, miR-7152-3p, miR-7155-5p, miR-7155-3p, miR-7157-3p, miR-7158-3p, miR-7159-5p, miR-7160-5p, miR-7161-5p, miR-7515, miR-7702, miR-7704, miR-7843-5p, miR-7843-3p, miR-4433b-5p, miR-4433b-3p, miR-1273h-5p, miR-1273h-3p, miR-7845-5p, miR-7846-3p, miR-7847-3p, miR-7851-3p, miR-7852-3p, miR-7855-5p, miR-8052, miR-8059, miR-8060, miR-8063, miR-8064, miR-8065, miR-8069, miR-8071, miR-8072, miR-8073, miR-8077, miR-8078, miR-8079, miR-8085, miR-8087, miR-8089, miR-128-2-5p, miR-7974, miR-7977, miR-301b-5p, miR-1249-5p, miR-4485-5p, miR-8485, miR-9500, miR-103a-1-5p, miR-196a-1-3p, miR-137-5p, miR-190b-3p, miR-9718, miR-9898, miR-9902, miR-10226, miR-10392-5p, miR-10392-3p, miR-10394-3p, miR-10396a-5p, miR-10396a-3p, miR-10398-5p, miR-10398-3p, miR-10399-3p, miR-10400-3p, miR-10401-5p, miR-10396b-5p, miR-10522-5p, miR-10526-3p, miR-11181-5p, miR-11181-3p, miR-11399, miR-11400, miR-3059-5p, miR-3059-3p, miR-3085-5p, miR-3085-3p, miR-12113, miR-12114, miR-12115, miR-12116, miR-12118, miR-12119, miR-12120, miR-12121, miR-12128, miR-12131, and miR-12136. In the present disclosure, the extract of urine may be an extract of the urine of a stage-I or -II cervical cancer patient. Only one of these microRNAs can be used to predict whether a subject from which the urine is derived is a cervical cancer patient (particularly stage-I or -II), and 5 or more types, 10 or more types, 15 or more types, or 20 or more types of these microRNAs can be used in combination to predict whether a subject from which the urine is derived is a cervical cancer patient (particularly stage-I or -II). Among these microRNAs, a microRNA that may be highly expressed in a cervical cancer patient (particularly stage-I or -II) can be preferably used for prediction.

The present disclosure provides an extract of urine containing at least one type, two types, three types, four types, five types, six types, seven types, eight types, nine types, or ten types of or all microRNAs selected from the group consisting of miR-17-3p, miR-25-3p, miR-196a-5p, miR-197-3p, miR-139-5p, miR-210-3p, miR-214-3p, miR-224-5p, miR-124-3p, miR-140-5p, miR-149-5p, miR-188-5p, miR-320a-3p, miR-106b-5p, miR-296-5p, miR-365a-3p, miR-365b-3p, miR-373-5p, miR-373-3p, miR-375-3p, miR-380-3p, miR-383-5p, miR-328-3p, miR-133b, miR-449a, miR-191-3p, miR-200a-5p, miR-483-3p, miR-484, miR-485-5p, miR-486-5p, miR-491-5p, miR-492, miR-493-5p, miR-432-5p, miR-498-5p, miR-519b-3p, miR-518c-5p, miR-516b-5p, miR-532-5p, miR-18a-3p, miR-539-5p, miR-551a, miR-554, miR-557, miR-564, miR-571, miR-574-3p, miR-575, miR-578, miR-611, miR-613, miR-632, miR-634, miR-637, miR-638, miR-652-3p, miR-661, miR-663a, miR-658, miR-659-3p, miR-671-5p, miR-668-3p, miR-766-3p, miR-765, miR-675-5p, miR-297, miR-19b-2-5p, miR-22-5p, miR-24-2-5p, miR-26b-3p, miR-92a-2-5p, miR-29b-1-5p, miR-139-3p, miR-181a-2-3p, miR-181c-3p, miR-183-3p, miR-23b-5p, miR-124-5p, miR-125b-1-3p, miR-135a-3p, miR-140-3p, miR-143-5p, miR-145-3p, miR-127-5p, miR-149-3p, miR-150-3p, miR-186-3p, miR-194-3p, miR-30c-1-3p, miR-296-3p, miR-361-3p, miR-371a-5p, miR-339-3p, miR-423-5p, miR-18b-3p, miR-20b-3p, miR-483-5p, miR-490-5p, miR-509-5p, miR-92b-5p, miR-574-5p, miR-550a-5p, miR-624-3p, miR-625-3p, miR-629-5p, miR-671-3p, miR-298, miR-541-5p, miR-744-3p, miR-885-5p, miR-885-3p, miR-877-5p, miR-877-3p, miR-665, miR-760, miR-920, miR-933, miR-936, miR-937-3p, miR-939-5p, miR-940, miR-943, miR-1224-3p, miR-1225-5p, miR-1225-3p, miR-1228-3p, miR-1229-3p, miR-1231, miR-1233-3p, miR-1234-3p, miR-1236-3p, miR-1237-3p, miR-1238-3p, miR-1264, miR-320b, miR-320c, miR-1323, miR-1298-5p, miR-1180-3p, miR-1182, miR-1184, miR-1202, miR-1203, miR-663b, miR-1204, miR-1207-5p, miR-1207-3p, miR-1208, miR-1299, miR-1249-3p, miR-1253, miR-1255a, miR-1260a, miR-1267, miR-1268a, miR-1272, miR-1275, miR-1278, miR-1281, miR-1292-5p, miR-1255b-5p, miR-1307-3p, miR-1825, miR-675-3p, miR-1469, miR-1538, miR-1908-5p, miR-1909-3p, miR-1912-3p, miR-1913, miR-1914-3p, miR-1915-3p, miR-365a-5p, miR-196b-3p, miR-449b-3p, miR-1972, miR-1976, miR-2052, miR-2110, miR-670-5p, miR-2116-3p, miR-548q, miR-2276-3p, miR-711, miR-718, miR-2681-3p, miR-449c-3p, miR-3115, miR-3120-3p, miR-3130-5p, miR-3131, miR-3132, miR-3138, miR-3141, miR-3144-5p, miR-3147, miR-548v, miR-3151-5p, miR-3153, miR-3154, miR-3155a, miR-3157-5p, miR-3158-3p, miR-3162-5p, miR-3163, miR-3165, miR-1260b, miR-3171, miR-3173-3p, miR-1193, miR-3175, miR-3178, miR-3180-3p, miR-3182, miR-3184-5p, miR-3185, miR-3188, miR-3194-5p, miR-3195, miR-3196, miR-3197, miR-3198, miR-514b-5p, miR-3202, miR-4297, miR-4293, miR-4294, miR-4299, miR-4298, miR-4313, miR-4316, miR-4319, miR-4322, miR-4323, miR-4257, miR-4258, miR-4260, miR-4326, miR-4327, miR-4265, miR-4266, miR-2355-5p, miR-4268, miR-4270, miR-4271, miR-4276, miR-4274, miR-4281, miR-4279, miR-4280, miR-4285, miR-4284, miR-4286, miR-4287, miR-4288, miR-4290, miR-2277-5p, miR-3605-3p, miR-3610, miR-3612, miR-3613-5p, miR-3614-5p, miR-3615, miR-3617-5p, miR-3619-5p, miR-3620-3p, miR-3622a-3p, miR-3646, miR-3648, miR-3649, miR-3652, miR-3663-5p, miR-3663-3p, miR-3675-3p, miR-3677-3p, miR-3679-5p, miR-3679-3p, miR-3681-5p, miR-3682-3p, miR-3692-3p, miR-3180, miR-3907, miR-3908, miR-3911, miR-3917, miR-3922-3p, miR-3923, miR-3926, miR-3928-3p, miR-3937, miR-3940-3p, miR-3943, miR-3945, miR-642b-3p, miR-550b-3p, miR-1268b, miR-378f, miR-4424, miR-4428, miR-4430, miR-4432, miR-4433a-3p, miR-4434, miR-4439, miR-4440, miR-4441, miR-4444, miR-4447, miR-4448, miR-4449, miR-548ah-5p, miR-4455, miR-3135b, miR-4462, miR-4463, miR-4466, miR-4467, miR-4468, miR-4470, miR-4472, miR-4473, miR-4475, miR-4478, miR-3689d, miR-3155b, miR-4480, miR-4481, miR-4483, miR-4484, miR-4486, miR-4489, miR-548al, miR-4492, miR-4495, miR-4496, miR-4497, miR-4504, miR-4505, miR-2392, miR-4507, miR-4508, miR-4512, miR-4513, miR-4519, miR-4521, miR-1269b, miR-4522, miR-4523, miR-4524a-3p, miR-4525, miR-4526, miR-4530, miR-4534, miR-1587, miR-4536-5p, miR-4538, miR-3120-5p, miR-3127-3p, miR-3158-5p, miR-3162-3p, miR-3173-5p, miR-3619-3p, miR-3691-3p, miR-3922-5p, miR-3944-5p, miR-3978, miR-4634, miR-4637, miR-4638-5p, miR-4639-3p, miR-4640-5p, miR-4640-3p, miR-4645-5p, miR-4646-5p, miR-4646-3p, miR-4648, miR-4649-5p, miR-4651, miR-4655-5p, miR-4656, miR-4661-5p, miR-4661-3p, miR-4664-5p, miR-4664-3p, miR-4665-5p, miR-4665-3p, miR-4667-5p, miR-4667-3p, miR-4668-5p, miR-4669, miR-4670-3p, miR-4671-3p, miR-4674, miR-4675, miR-4685-5p, miR-4685-3p, miR-4687-5p, miR-4687-3p, miR-1343-3p, miR-4688, miR-4690-5p, miR-4691-5p, miR-4691-3p, miR-4695-5p, miR-4695-3p, miR-4697-5p, miR-4697-3p, miR-4698, miR-4704-3p, miR-4706, miR-4707-5p, miR-4707-3p, miR-4708-5p, miR-4708-3p, miR-4709-3p, miR-4710, miR-4712-3p, miR-4713-5p, miR-4713-3p, miR-4715-5p, miR-4716-5p, miR-4716-3pmiR-4721, miR-4722-5p, miR-4722-3p, miR-4723-3p, miR-451b, miR-4725-5p, miR-4725-3p, miR-4726-5p, miR-4726-3p, miR-4728-5p, miR-4728-3p, miR-4729, miR-4730, miR-4731-5p, miR-4731-3p, miR-4732-5p, miR-4733-5p, miR-4736, miR-4738-5p, miR-4738-3p, miR-4739, miR-4740-3p, miR-4741, miR-122b-5p, miR-4745-5p, miR-4746-3p, miR-4747-5p, miR-4748, miR-4749-5p, miR-4749-3p, miR-4750-5p, miR-4751, miR-4753-5p, miR-371b-3p, miR-4755-3p, miR-4756-5p, miR-4758-5p, miR-4758-3p, miR-4761-5p, miR-4762-3p, miR-4763-5p, miR-4763-3p, miR-4769-3p, miR-4778-5p, miR-4780, miR-4436b-5p, miR-4781-5p, miR-4783-3p, miR-2467-5p, miR-4787-3p, miR-4788, miR-4790-3p, miR-4793-3p, miR-4798-5p, miR-4800-5p, miR-4803, miR-4804-5p, miR-642a-3p, miR-4433a-5p, miR-4482-3p, miR-4999-5p, miR-5001-5p, miR-5002-3p, miR-5003-3p, miR-548ao-3p, miR-5006-5p, miR-5006-3p, miR-5010-5p, miR-5088-5p, miR-5090, miR-5094, miR-5187-3p, miR-5193, miR-5195-5p, miR-5195-3p, miR-5196-5p, miR-5196-3p, miR-5572, miR-548as-5p, miR-548as-3p, miR-5579-5p, miR-5579-3p, miR-5584-5p, miR-5584-3p, miR-5585-5p, miR-5586-3p, miR-5589-5p, miR-5684, miR-5685, miR-5690, miR-5693, miR-5695, miR-5698, miR-197-5p, miR-204-3p, miR-211-3p, miR-514a-5p, miR-584-3p, miR-652-5p, miR-1185-2-3p, miR-1304-3p, miR-1247-3p, miR-1306-5p, miR-3184-3p, miR-3191-5p, miR-642b-5p, miR-365b-5p, miR-1185-1-3p, miR-376c-5p, miR-503-3p, miR-376a-2-5p, miR-937-5p, miR-939-3p, miR-1227-5p, miR-1229-5p, miR-1233-5p, miR-1237-5p, miR-1238-5p, miR-1292-3p, miR-3620-5p, miR-3934-3p, miR-4632-5p, miR-4743-3p, miR-4750-3p, miR-5739, miR-5787, miR-6068, miR-6069, miR-6071, miR-6077, miR-6078, miR-6086, miR-6124, miR-6125, miR-6126, miR-6127, miR-378j, miR-6131, miR-6132, miR-6133, miR-6165, miR-6500-5p, miR-6501-5p, miR-6505-5p, miR-6507-5p, miR-6510-5p, miR-6511a-5p, miR-6511a-3p, miR-6512-5p, miR-6512-3p, miR-6513-5p, miR-6515-3p, miR-6715b-3p, miR-6716-5p, miR-6716-3p, miR-6511b-5p, miR-6511b-3p, miR-6718-5p, miR-6719-3p, miR-6720-3p, miR-6721-5p, miR-6722-3p, miR-6724-5p, miR-210-5p, miR-128-1-5p, miR-133a-5p, miR-152-5p, miR-489-5p, miR-511-3p, miR-181d-3p, miR-504-3p, miR-487b-5p, miR-585-5p, miR-598-5p, miR-619-5p, miR-627-3p, miR-1296-3p, miR-1301-5p, miR-874-5p, miR-216b-3p, miR-208b-5p, miR-1266-3p, miR-3151-3p, miR-500b-3p, miR-3912-5p, miR-1343-5p, miR-5189-3p, miR-5699-5p, miR-6726-5p, miR-6726-3p, miR-6727-3p, miR-6728-5p, miR-6728-3p, miR-6729-5p, miR-6729-3p, miR-6730-5p, miR-6730-3p, miR-6731-3p, miR-6732-5p, miR-6732-3p, miR-6734-5p, miR-6735-5p, miR-6735-3p, miR-6736-5p, miR-6736-3p, miR-6737-5p, miR-6738-5p, miR-6738-3p, miR-6740-5p, miR-6740-3p, miR-6741-5p, miR-6741-3p, miR-6742-5p, miR-6742-3p, miR-6743-5p, miR-6743-3p, miR-6744-5p, miR-6744-3p, miR-6746-5p, miR-6748-5p, miR-6748-3p, miR-6749-5p, miR-6749-3p, miR-6750-3p, miR-6751-5p, miR-6751-3p, miR-6752-5p, miR-6752-3p, miR-6753-5p, miR-6753-3p, miR-6754-5p, miR-6754-3p, miR-6756-5p, miR-6756-3p, miR-6757-3p, miR-6758-5p, miR-6759-5p, miR-6759-3p, miR-6760-3p, miR-6761-3p, miR-6762-5p, miR-6762-3p, miR-6763-5p, miR-6763-3p, miR-6764-3p, miR-6765-5p, miR-6766-5p, miR-6766-3p, miR-6768-5p, miR-6769a-5p, miR-6769a-3p, miR-6771-5p, miR-6772-5p, miR-6772-3p, miR-6773-5p, miR-6773-3p, miR-6774-5p, miR-6774-3p, miR-6775-5p, miR-6776-5p, miR-6776-3p, miR-6777-5p, miR-6777-3p, miR-6778-5p, miR-6779-5p, miR-6780a-5p, miR-6781-5p, miR-6782-5p, miR-6782-3p, miR-6783-5p, miR-6783-3p, miR-6784-5p, miR-6784-3p, miR-6785-3p, miR-6786-5p, miR-6786-3p, miR-6787-5p, miR-6787-3p, miR-6789-5p, miR-6789-3p, miR-6790-5p, miR-6791-5p, miR-6792-5p, miR-6792-3p, miR-6793-3p, miR-6794-5p, miR-6794-3p, miR-6795-5p, miR-6795-3p, miR-6796-5p, miR-6796-3p, miR-6797-5p, miR-6797-3p, miR-6798-5p, miR-6798-3p, miR-6799-5p, miR-6800-5p, miR-6800-3p, miR-6801-3p, miR-6802-5p, miR-6802-3p, miR-6803-5p, miR-6803-3p, miR-6804-3p, miR-6805-5p, miR-6805-3p, miR-6806-5p, miR-6807-5p, miR-6808-5p, miR-6808-3p, miR-6809-5p, miR-6809-3p, miR-6810-5p, miR-6810-3p, miR-6812-5p, miR-6813-5p, miR-6813-3p, miR-6816-5p, miR-6816-3p, miR-6817-3p, miR-6819-5p, miR-6819-3p, miR-6820-5p, miR-6820-3p, miR-6821-5p, miR-6821-3p, miR-6823-3p, miR-6824-5p, miR-6824-3p, miR-6825-5p, miR-6825-3p, miR-6826-5p, miR-6826-3p, miR-6828-5p, miR-6829-5p, miR-6830-5p, miR-6830-3p, miR-6831-5p, miR-6832-5p, miR-6832-3p, miR-6833-5p, miR-6833-3p, miR-6835-5p, miR-6780b-5p, miR-6780b-3p, miR-6836-3p, miR-6837-5p, miR-6837-3p, miR-6838-5p, miR-6839-5p, miR-6839-3p, miR-6840-5p, miR-6840-3p, miR-6842-5p, miR-6845-5p, miR-6845-3p, miR-6846-5p, miR-6846-3p, miR-6848-5p, miR-6848-3p, miR-6849-5p, miR-6849-3p, miR-6850-5p, miR-6851-5p, miR-6851-3p, miR-6852-3p, miR-6855-5p, miR-6855-3p, miR-6856-5p, miR-6857-3p, miR-6858-5p, miR-6858-3p, miR-6859-3p, miR-6769b-5p, miR-6860, miR-6861-5p, miR-6861-3p, miR-6862-3p, miR-6865-5p, miR-6865-3p, miR-6866-5p, miR-6867-5p, miR-6867-3p, miR-6870-5p, miR-6870-3p, miR-6873-5p, miR-6873-3p, miR-6875-5p, miR-6876-3p, miR-6877-5p, miR-6877-3p, miR-6878-5p, miR-6879-5p, miR-6879-3p, miR-6880-5p, miR-6880-3p, miR-6881-5p, miR-6881-3p, miR-6882-3p, miR-6883-3p, miR-6884-3p, miR-6885-3p, miR-6886-5p, miR-6886-3p, miR-6887-5p, miR-6887-3p, miR-6889-5p, miR-6889-3p, miR-6890-5p, miR-6890-3p, miR-6891-5p, miR-6891-3p, miR-6892-5p, miR-6892-3p, miR-6893-5p, miR-6893-3p, miR-6894-5p, miR-6894-3p, miR-6895-5p, miR-6895-3p, miR-7106-5p, miR-7106-3p, miR-7107-5p, miR-7108-5p, miR-7108-3p, miR-7109-5p, miR-7109-3p, miR-7110-5p, miR-7110-3p, miR-7111-5p, miR-7111-3p, miR-7112-5p, miR-7113-3p, miR-7114-5p, miR-7114-3p, miR-7150, miR-7151-5p, miR-7152-5p, miR-7155-5p, miR-7155-3p, miR-7157-3p, miR-7158-3p, miR-7159-5p, miR-7160-5p, miR-7161-5p, miR-7515, miR-7843-5p, miR-7843-3p, miR-4433b-5p, miR-4433b-3p, miR-1273h-5p, miR-1273h-3p, miR-7845-5p, miR-7846-3p, miR-7847-3p, miR-7852-3p, miR-7855-5p, miR-8052, miR-8053, miR-8059, miR-8060, miR-8061, miR-8063, miR-8064, miR-8065, miR-8071, miR-8072, miR-8073, miR-8077, miR-8078, miR-8079, miR-8085, miR-8087, miR-8089, miR-7974, miR-7977, miR-7978, miR-301b-5p, miR-1249-5p, miR-4485-5p, miR-8485, miR-9500, miR-103a-1-5p,miR-137-5p, miR-190b-3p, miR-9718, miR-9898, miR-9902, miR-10392-5p, miR-10392-3p, miR-10396a-5p, miR-10396a-3p, miR-10398-5p, miR-10398-3p, miR-10399-3p, miR-10400-3p, miR-10401-5p, miR-10396b-5p, miR-10522-5p, miR-10526-3p, miR-11181-3p, miR-11399, miR-11400, miR-3059-5p, miR-3059-3p, miR-3085-5p, miR-3085-3p, miR-12114, miR-12115, miR-12116, miR-12118, miR-12119, miR-12120, miR-12121, miR-12128, miR-12131, and miR-12136. In the present disclosure, the extract of urine may be an extract of the urine of a stage-I cervical cancer patient. Only one of these microRNAs can be used to predict whether a subject from which the urine is derived is a cervical cancer patient (particularly stage-I), and 5 or more types, 10 or more types, 15 or more types, or 20 or more types of these microRNAs can be used in combination to predict whether a subject from which the urine is derived is a cervical cancer patient (particularly stage-I). Among these microRNAs, a microRNA that may be highly expressed in a cervical cancer patient (particularly stage-I) can be preferably used for prediction.

The present disclosure provides an extract of urine containing at least one type, two types, three types, four types, five types, six types, seven types, eight types, nine types, or ten types of or all microRNAs selected from the group consisting of microRNAs having an accuracy of higher than 0.500 among the microRNAs listed in Table 24-13. In the present disclosure, the extract of urine may be an extract of the urine of a cervical cancer patient. Only one of these microRNAs can be used to predict whether a subject from which the urine is derived is a cervical cancer patient, and 5 or more types, 10 or more types, 15 or more types, or 20 or more types of these microRNAs can be used in combination to predict whether a subject from which the urine is derived is a cervical cancer patient. Among these microRNAs, a microRNA that may be highly expressed in a cervical cancer patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

The present disclosure provides an extract of urine containing at least one type, two types, three types, four types, five types, six types, seven types, eight types, nine types, or ten types of or all microRNAs selected from the group consisting of microRNAs having an accuracy of higher than 0.500 among the microRNAs listed in Table 25-13. In the present disclosure, the extract of urine may be an extract of the urine of a cervical cancer patient. Only one of these microRNAs can be used to predict whether a subject from which the urine is derived is a cervical cancer patient, and 5 or more types, 10 or more types, 15 or more types, or 20 or more types of these microRNAs can be used in combination to predict whether a subject from which the urine is derived is a cervical cancer patient. Among these microRNAs, a microRNA that may be highly expressed in a cervical cancer patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

The present disclosure provides an extract of urine containing at least one type, two types, three types, four types, five types, six types, seven types, eight types, nine types, or ten types of or all microRNAs selected from the microRNAs listed in Table 4-14. The present disclosure provides an extract of urine containing at least one type, two types, three types, four types, five types, six types, seven types, eight types, nine types, or ten types of or all microRNAs selected from the group consisting of miR-6791-5p, miR-10400-3p, miR-1292-5p, miR-6125, miR-6789-5p, miR-665, miR-1268b, miR-4521, miR-125b-1-3p, miR-6781-5p, miR-3937, miR-4458, miR-6729-5p, miR-10396a-3p, miR-4745-5p, miR-6721-5p, miR-4467, miR-6798-5p, miR-4321, miR-373-3p, miR-4651, miR-4634, miR-1908-5p, miR-8072, miR-4505, miR-3648, miR-6805-5p, miR-1343-5p, miR-6850-5p, miR-4759, miR-1227-5p, miR-4507, miR-6732-5p, miR-638, miR-4763-3p, miR-4707-5p, miR-6845-5p, miR-4785, miR-202-3p, miR-4433a-3p, miR-3620-5p, miR-6743-5p, miR-6875-5p, miR-1469, miR-1915-3p, miR-4758-5p, miR-6724-5p, miR-1225-5p, miR-664b-5p, miR-10392-5p, miR-4688, miR-297, miR-3197, miR-449b-5p, miR-4497, miR-3141, miR-4749-5p, miR-6756-5p, miR-4258, miR-6821-5p, miR-10396b-5p, miR-769-5p, miR-12120, miR-4460, miR-6075, miR-1207-5p, miR-8078, miR-449c-5p, miR-6836-5p, miR-1268a, miR-6787-5p, miR-6081, miR-7515, miR-20b-3p, miR-937-5p, miR-579-3p, miR-6840-3p, miR-3691-5p, miR-378h, miR-4632-5p, miR-187-5p, miR-3180-3p, miR-4466, miR-4530, miR-6132, miR-92b-5p, miR-7851-3p, miR-10392-3p, miR-3654, miR-5007-5p, miR-6750-5p, miR-492, miR-4741, miR-4734, miR-7108-5p, miR-6506-5p, miR-4640-5p, miR-1909-3p, miR-6838-5p, miR-486-3p, miR-6809-5p, miR-6722-3p, miR-4446-3p, miR-6786-5p, miR-4695-5p, miR-1587, miR-4486, miR-6737-5p, miR-644a, miR-106a-3p, miR-3161, miR-5090, miR-6752-5p, miR-8089, miR-6071, miR-510-5p, miR-371b-5p, miR-3135b, miR-1321, miR-3196, miR-4508, miR-4280, miR-1270, miR-149-3p, miR-6848-5p, miR-3185, miR-6090, miR-6501-3p, miR-6888-5p, miR-6790-5p, miR-498-5p, miR-3934-3p, miR-4519, miR-4520-3p, miR-761, miR-1237-5p, miR-5001-5p, miR-504-5p, miR-6861-5p, miR-200b-5p, miR-1265, miR-128-1-5p, miR-663a, miR-6839-5p, miR-382-5p, miR-10396a-5p, miR-4451, miR-4433b-3p, miR-6509-5p, miR-4327, miR-1273c, miR-12114, miR-6813-5p, miR-4681, miR-4708-3p, miR-4317, miR-6768-5p, miR-4727-3p, miR-4492, miR-1912-3p, miR-579-5p, miR-138-5p, miR-3652, miR-3187-3p, miR-6804-5p, miR-147a, miR-4673, miR-6759-5p, miR-3675-5p, miR-1294, miR-557, miR-3178, miR-6726-5p, miR-4535, miR-320c, miR-184, miR-3180, miR-92a-2-5p, miR-1910-3p, miR-5187-5p, miR-211-3p, miR-6784-5p, miR-3192-5p, miR-6501-5p, miR-6763-5p, miR-12127, miR-4672, miR-433-5p, miR-4674, miR-744-5p, miR-34c-5p, miR-6747-5p, miR-5100, miR-5189-5p, miR-12118, miR-6816-5p, miR-8069, miR-887-3p, miR-139-3p, miR-6820-5p, miR-6504-5p, miR-760, miR-32-3p, miR-1304-5p, miR-656-5p, miR-433-3p, miR-4479, miR-6845-3p, miR-4690-5p, miR-1269a, miR-8063, miR-4725-3p, miR-1231, miR-3651, miR-6772-5p, miR-1226-5p, miR-541-3p, miR-3917, miR-4665-5p, miR-1276, miR-325, miR-493-3p, miR-185-3p, miR-7110-5p, miR-762, miR-3663-3p, miR-8077, miR-1288-3p, miR-6720-5p, miR-718, miR-6776-5p, miR-4706, miR-4999-5p, miR-6829-5p, miR-617, miR-8074, miR-575, miR-6068, miR-650, miR-3195, miR-4684-3p, miR-3621, miR-3059-3p, miR-4498, miR-6836-3p, miR-6782-5p, miR-4506, miR-8071, miR-4691-3p, miR-11399, miR-12121, miR-3130-3p, miR-124-5p, miR-4538, miR-4687-5p, miR-12124, miR-6738-5p, miR-6762-5p, miR-939-5p, miR-4737, miR-3187-5p, miR-663b, miR-378g, miR-6871-5p, miR-4736, miR-6799-5p, miR-4660, miR-8057, miR-8064, miR-4447, miR-6134, miR-4439, miR-6513-5p, miR-4449, miR-6783-5p, miR-4276, miR-4724-5p, miR-3941, miR-2467-3p, miR-6069, miR-6765-5p, miR-7854-3p, miR-5192, miR-5196-5p, miR-548q, miR-4645-3p, miR-4750-5p, miR-516a-5p, miR-6800-5p, miR-338-5p, miR-4635, miR-410-5p, miR-7154-3p, miR-3162-5p, miR-6131, miR-3619-5p, miR-4786-3p, miR-4489, miR-4783-3p, miR-4648, miR-197-5p, miR-6770-3p, miR-3142, miR-766-5p, miR-6817-5p, miR-6868-5p, miR-5194, miR-135a-3p, miR-135a-2-3p, miR-219a-2-3p, miR-4676-5p, miR-4746-3p, miR-3919, miR-4657, miR-7151-3p, miR-885-3p, miR-5698, miR-9718, miR-4685-5p, miR-4787-5p, miR-29b-3p, miR-4465, miR-614, miR-4463, miR-323a-3p, miR-423-5p, miR-4529-5p, miR-758-5p, miR-6515-5p, miR-4484, miR-4722-5p, miR-4655-5p, miR-6080, miR-6767-5p, miR-595, miR-212-5p, miR-3677-3p, miR-10401-5p, miR-1471, miR-4638-5p, miR-6089, miR-6719-3p, miR-1972, miR-6129, miR-512-3p, miR-5684, miR-942-3p, miR-2681-5p, miR-4257, miR-196b-3p, miR-6529-5p, let-7e-5p, miR-6766-5p, miR-7160-5p, miR-4644, miR-489-5p, miR-4753-3p, miR-6715b-5p, miR-551b-3p, miR-548g-3p, miR-6857-5p, miR-610, miR-6860, miR-504-3p, miR-4478, miR-122-5p, miR-3605-5p, miR-4701-3p, miR-4682, miR-4653-3p, miR-1293, miR-3616-3p, miR-593-3p, miR-6775-5p, miR-103a-2-5p, miR-542-5p, miR-329-5p, miR-5093, miR-3934-5p, miR-106b-5p, miR-3150b-3p, miR-4450, miR-6824-5p, miR-3074-5p, miR-4510, miR-3928-3p, miR-8081, miR-2682-5p, miR-4300, miR-3124-5p, miR-4786-5p, miR-422a, miR-5006-5p, miR-4322, miR-11401, miR-4440, miR-9986, miR-147b-3p, miR-378i, miR-7114-5p, miR-892c-5p, miR-1914-3p, let-7i-5p, miR-498-3p, miR-4454, miR-639, miR-5088-5p, miR-6074, miR-1224-5p, miR-4539, miR-5787, miR-5004-3p, miR-8082, miR-4773, miR-550b-2-5p, miR-6514-5p, miR-4738-3p, miR-6863, miR-598-5p, miR-6797-5p, miR-6869-5p, miR-4470, miR-6778-5p, miR-381-5p, miR-3127-5p, miR-6761-5p, miR-4303, miR-6126, miR-1322, miR-6715a-3p, miR-7113-5p, miR-7162-3p, miR-5685, miR-7850-5p, miR-646, miR-4299, miR-4265, miR-3907, let-7b-5p, miR-125b-2-3p, miR-6847-5p, miR-2115-5p, miR-645, miR-4781-5p, miR-7847-3p, miR-6884-5p, miR-5589-5p, miR-608, miR-4437, miR-5087, miR-6832-5p, miR-3913-3p, miR-23b-3p, miR-210-5p, miR-6716-5p, miR-4428, miR-30a-3p, miR-4436b-3p, miR-4691-5p, miR-5703, miR-4255, miR-6792-5p, miR-1307-3p, miR-6793-5p, miR-6895-5p, miR-1249-5p, miR-371a-3p, miR-4776-5p, miR-5002-3p, miR-3940-5p, miR-1843, miR-938, miR-335-3p, miR-601, miR-3125, miR-4502, miR-892a, miR-572, miR-6846-5p, miR-4315, miR-4487, miR-564, miR-6796-5p, miR-1255b-2-3p, miR-181b-5p, miR-6751-5p, miR-7706, miR-5708, miR-10397-5p, miR-365a-5p, miR-1468-5p, miR-6086, miR-105-5p, miR-7150, miR-6877-5p, miR-4488, miR-5190, miR-8086, miR-4448, miR-3922-5p, miR-4516, miR-500b-5p, miR-5690, miR-12122, miR-658, miR-5585-3p, miR-6127, miR-4513, miR-10398-3p, miR-1298-3p, miR-4482-3p, miR-4481, miR-1228-5p, miR-6753-5p, miR-4733-3p, miR-6894-5p, miR-6727-5p, miR-521, miR-1538, miR-711, miR-4526, miR-4256, miR-3148, miR-373-5p, miR-1285-5p, miR-515-5p, miR-4796-5p, miR-551b-5p, miR-1236-5p, miR-892c-3p, miR-382-3p, miR-4462, miR-1287-5p, miR-487b-3p, miR-6859-5p, miR-4776-3p, miR-3689f, miR-516b-3p, miR-516a-3p, miR-12128, miR-7112-5p, miR-4261, miR-3682-3p, miR-4653-5p, miR-3689b-3p, miR-3689c, miR-1288-5p, miR-4703-3p, miR-7157-3p, miR-3973, miR-3666, miR-6772-3p, miR-4430, miR-3120-3p, miR-6814-5p, miR-6717-5p, miR-8083, miR-552-3p, miR-3944-5p, miR-3151-5p, miR-12131, miR-4730, miR-889-5p, miR-5704, miR-6740-5p, miR-6165, miR-7977, miR-4654, miR-4658, miR-6806-5p, miR-6516-5p, miR-6504-3p, miR-1228-3p, miR-4794, miR-6070, miR-4784, miR-668-5p, miR-4298, miR-449a, miR-4435, miR-6076, miR-4710, miR-487a-3p, miR-6771-5p, miR-4708-5p, miR-1343-3p, miR-1202, miR-891a-5p, miR-4743-5p, miR-8059, miR-1251-3p, miR-6499-5p, miR-3186-3p, miR-1291, miR-6810-5p, miR-6842-5p, miR-1285-3p, miR-3186-5p, miR-7704, miR-3064-5p, miR-196a-5p, miR-3660, miR-589-3p, miR-3622a-5p, miR-6083, miR-6876-5p, miR-3916, miR-3659, miR-6889-5p, miR-525-5p, miR-6825-5p, miR-6801-5p, miR-6749-5p, miR-3665, miR-6851-5p, miR-4311, miR-187-3p, miR-584-5p, miR-193a-5p, miR-6505-3p, miR-676-3p, miR-1257, miR-513c-5p, miR-1909-5p, miR-1193, miR-18a-3p, miR-591, miR-7845-5p, miR-891a-3p, miR-4692, miR-6855-5p, miR-550a-3-5p, miR-3149, miR-585-3p, miR-6858-5p, miR-6879-5p, miR-4659b-3p, miR-6870-5p, miR-217-3p, miR-3617-3p, miR-5000-5p, miR-5696, miR-584-3p, miR-3655, miR-6788-5p, miR-3137, miR-6808-5p, miR-12117, miR-4761-3p, miR-5188, miR-200c-5p, miR-5588-3p, miR-4252, miR-4283, miR-4485-5p, miR-3199, miR-619-5p, miR-6777-5p, miR-4721, miR-29c-5p, miR-148b-5p, miR-182-5p, miR-6843-3p, miR-3918, miR-4453, miR-2355-3p, miR-4717-5p, miR-4443, miR-637, miR-6722-5p, miR-8073, miR-25-5p, miR-216a-3p, miR-370-5p, miR-4324, miR-6754-3p, miR-4483, miR-506-3p, miR-1306-3p, miR-222-5p, miR-299-3p, miR-6769b-5p, miR-4511, miR-2277-5p, miR-4491, miR-6735-5p, miR-514b-5p, miR-10395-5p, miR-5091, miR-6738-3p, miR-5008-5p, miR-130a-5p, miR-8075, miR-3165, miR-4753-5p, miR-921, miR-8080, miR-4686, miR-585-5p, miR-4520-5p, miR-558, miR-1224-3p, miR-2392, miR-7113-3p, miR-99b-3p, miR-10394-3p, miR-675-5p, miR-525-3p, miR-7109-5p, miR-5591-3p, miR-210-3p, miR-3663-5p, miR-6803-5p, miR-3173-5p, miR-200a-5p, miR-3155a, miR-6837-5p, miR-3129-3p, miR-4455, miR-6892-5p, miR-6764-3p, miR-6130, let-7d-3p, miR-1204, miR-193b-5p, miR-6879-3p, miR-5572, miR-1281, miR-6511a-5p, miR-6514-3p, miR-6779-5p, miR-4711-3p, miR-7159-5p, miR-8052, miR-323b-5p, miR-4289, miR-936, miR-6805-3p, miR-615-5p, miR-6834-5p, miR-4271, miR-1301-5p, miR-214-5p, miR-3925-3p, miR-485-5p, miR-4429, miR-574-5p, miR-6718-5p, miR-12119, miR-3184-5p, miR-11181-3p, miR-34a-5p, miR-6500-3p, miR-3622a-3p, miR-6124, miR-1266-5p, miR-4314, miR-10524-5p, miR-320e, miR-450a-5p, miR-122b-3p, miR-6503-3p, miR-1273h-5p, miR-15a-5p, miR-4769-5p, miR-4323, miR-4431, miR-5580-5p, miR-30b-3p, miR-6822-5p, miR-6849-5p, miR-6780b-5p, miR-3085-5p, miR-138-2-3p, miR-4780, miR-1225-3p, miR-602, miR-4436a, miR-517a-3p, miR-517b-3p, miR-3646, miR-128-3p, miR-3681-3p, miR-3130-5p, miR-4420, miR-7109-3p, miR-3170, miR-4273, miR-501-5p, miR-483-5p, miR-6893-5p, miR-6072, miR-6762-3p, miR-204-3p, miR-4769-3p, miR-3529-5p, miR-206, miR-6792-3p, miR-2276-3p, miR-6850-3p, miR-24-2-5p, miR-146b-3p, miR-138-1-3p, miR-4748, miR-4700-5p, miR-365b-5p, miR-6512-3p, miR-3193, miR-487a-5p, miR-6499-3p, miR-515-3p, miR-10522-5p, miR-6874-5p, miR-7151-5p, miR-4436b-5p, miR-6736-5p, miR-4533, miR-6890-5p, miR-5089-3p, miR-194-3p, miR-3944-3p, miR-6893-3p, miR-3147, miR-4669, miR-8070, miR-378b, miR-221-3p, miR-4649-5p, miR-4269, miR-941, miR-143-3p, miR-604, miR-6133, miR-5681b, miR-5689, miR-4712-5p, miR-4756-3p, miR-208a-5p, miR-6875-3p, miR-7107-5p, miR-885-5p, miR-668-3p, miR-6513-3p, miR-6804-3p, miR-4474-3p, miR-4285, miR-6509-3p, miR-3174, miR-7155-5p, miR-186-3p, miR-192-5p, miR-1-5p, miR-134-3p, miR-449c-3p, miR-10401-3p, miR-4423-5p, miR-3153, miR-103a-1-5p, miR-4639-3p, miR-342-3p, miR-495-5p, miR-1250-5p, miR-5705, miR-4801, miR-10398-5p, miR-7978, miR-3685, miR-1303, miR-4514, miR-501-3p, miR-548d-3p, miR-1255b-5p, miR-6856-3p, miR-632, miR-5189-3p, miR-4665-3p, miR-6748-5p, miR-4728-3p, miR-7846-3p, miR-654-5p, miR-4456, miR-3173-3p, miR-3689a-3p, miR-3935, miR-6733-3p, miR-548au-3p, miR-10527-5p, miR-3194-5p, miR-497-5p, miR-4494, miR-6783-3p, miR-1260b, miR-6883-5p, miR-6825-3p, miR-550a-5p, miR-652-5p, miR-129-5p, miR-6794-5p, miR-432-5p, miR-6736-3p, miR-3119, miR-3150a-5p, miR-4522, miR-4326, miR-612, miR-7152-5p, miR-767-5p, miR-3138, miR-1247-3p, miR-6887-3p, miR-3680-3p, miR-103a-3p, miR-4722-3p, miR-4713-3p, miR-6852-3p, miR-214-3p, miR-7111-5p, miR-27b-3p, miR-6787-3p, miR-647, miR-3085-3p, miR-6856-5p, miR-6894-3p, miR-6803-3p, miR-6807-5p, miR-4441, miR-6877-3p, miR-4656, miR-449b-3p, miR-6088, miR-11400, miR-574-3p, miR-6822-3p, miR-8055, miR-4670-5p, miR-16-1-3p, miR-8079, miR-494-3p, miR-378c, miR-4733-5p, miR-7161-5p, miR-3667-5p, miR-514b-3p, miR-6743-3p, miR-6880-3p, miR-200b-3p, miR-6853-5p, miR-4524a-5p, miR-7155-3p, miR-320d, miR-520c-3p, miR-3120-5p, miR-4296, miR-4724-3p, miR-8485, miR-7973, miR-7106-5p, miR-4768-3p, miR-5588-5p, miR-6755-5p, miR-487b-5p, miR-4445-3p, miR-6727-3p, miR-132-5p, miR-1238-3p, miR-661, miR-4527, miR-1976, miR-6886-3p, miR-6882-3p, miR-3074-3p, miR-6854-3p, miR-5094, miR-6823-5p, miR-1910-5p, miR-18b-3p, miR-6716-3p, miR-500a-5p, miR-6880-5p, miR-6761-3p, miR-5010-5p, miR-34b-3p, miR-874-5p, miR-550b-3p, miR-935, miR-6891-5p, miR-3929, miR-6869-3p, miR-466, miR-6774-5p, miR-4732-3p, miR-6789-3p, miR-224-3p, miR-6778-3p, miR-6817-3p, miR-494-5p, miR-508-5p, miR-6842-3p, miR-6747-3p, miR-1238-5p, miR-361-5p, miR-4685-3p, miR-924, miR-3200-3p, miR-346, miR-4740-5p, miR-623, miR-615-3p, miR-548ay-3p, miR-4279, miR-411-3p, miR-6833-3p, miR-675-3p, miR-6510-5p, miR-765, miR-4697-5p, miR-3184-3p, miR-3065-3p, miR-4421, miR-7976, miR-4297, miR-3190-3p, miR-4695-3p, miR-5004-5p, miR-6818-3p, miR-4694-5p, miR-6882-5p, miR-4305, miR-6862-3p, miR-4529-3p, miR-4754, miR-4268, miR-642b-3p, miR-3622b-5p, miR-10523-5p, miR-21-3p, miR-12115, miR-328-3p, miR-154-5p, miR-106b-3p, miR-4763-5p, miR-6511b-5p, miR-3911, miR-4496, miR-6829-3p, miR-4524b-3p, miR-6773-3p, miR-500b-3p, miR-3198, miR-6741-5p, miR-4783-5p, miR-10400-5p, miR-4253, miR-1233-5p, miR-6779-3p, miR-3064-3p, miR-339-3p, miR-6785-5p, miR-370-3p, miR-6809-3p, miR-6849-3p, miR-484, miR-3175, miR-181a-5p, miR-3610, miR-6500-5p, miR-1260a, miR-5580-3p, miR-378f, miR-877-3p, miR-6511a-3p, miR-6511b-3p, miR-6739-3p, miR-3191-5p, miR-6791-3p, miR-4743-3p, miR-3191-3p, miR-6742-5p, miR-6818-5p, miR-2110, miR-1908-3p, miR-766-3p, miR-4758-3p, miR-5702, miR-6841-5p, miR-4475, miR-1298-5p, miR-6815-3p, miR-1229-3p, miR-4684-5p, miR-6813-3p, miR-3605-3p, miR-151a-5p, miR-6749-3p, miR-4444, miR-6861-3p, miR-532-3p, miR-130b-5p, miR-330-5p, miR-5694, miR-4725-5p, miR-4473, miR-6078, miR-3690, miR-6800-3p, miR-7975, miR-6515-3p, miR-5089-5p, miR-1284, miR-4661-3p, miR-517c-3p, miR-6750-3p, miR-3155b, miR-5088-3p, miR-6847-3p, miR-4316, miR-7848-3p, miR-9902, miR-330-3p, miR-5197-5p, miR-4800-5p, miR-6770-5p, miR-197-3p, miR-1825, miR-134-5p, miR-3151-3p, miR-489-3p, miR-188-5p, miR-23a-5p, miR-4727-5p, miR-769-3p, miR-15b-5p, miR-6777-3p, miR-7844-5p, miR-7106-3p, miR-1229-5p, miR-554, miR-3158-3p, miR-7111-3p, miR-654-3p, miR-6867-3p, miR-122-3p, miR-3650, miR-1249-3p, miR-3157-3p, miR-3679-5p, miR-550a-3p, miR-4755-3p, miR-6876-3p, miR-6726-3p, miR-4524b-5p, miR-6759-3p, miR-323a-5p, miR-6780b-3p, miR-491-5p, miR-4723-3p, miR-3928-5p, miR-5002-5p, miR-7158-5p, miR-1178-3p, miR-5699-3p, miR-8062, miR-3152-5p, miR-7849-3p, miR-875-3p, miR-3680-5p, miR-1237-3p, miR-6753-3p, miR-6741-3p, miR-4713-5p, and miR-6774-3p. In the present disclosure, the extract of urine may be an extract of the urine of a colorectal cancer patient. Only one of these microRNAs can be used to predict whether a subject from which the urine is derived is a colorectal cancer patient, and 5 or more types, 10 or more types, 15 or more types, or 20 or more types of these microRNAs can be used in combination to predict whether a subject from which the urine is derived is a colorectal cancer patient. Among these microRNAs, a microRNA that may be highly expressed in a colorectal cancer patient can be preferably used for prediction.

The present disclosure provides an extract of urine containing at least one type, two types, three types, four types, five types, six types, seven types, eight types, nine types, or ten types of or all microRNAs selected from the group consisting of miR-15a-5p, miR-30a-5p, miR-192-5p, miR-196a-5p, miR-139-5p, miR-187-3p, miR-210-3p, miR-122-5p, miR-124-3p, miR-138-5p, miR-200c-3p, miR-106b-5p, miR-370-3p, miR-373-5p, miR-373-3p, miR-328-3p, miR-342-3p, miR-325, miR-346, miR-422a, miR-449a, miR-450a-5p, miR-452-5p, miR-202-3p, miR-492, miR-498-5p, miR-520c-3p, miR-519a-3p, miR-503-5p, miR-552-3p, miR-557, miR-564, miR-573, miR-574-3p, miR-575, miR-579-3p, miR-585-3p, miR-588, miR-595, miR-608, miR-612, miR-615-3p, miR-617, miR-637, miR-638, miR-639, miR-663a, miR-654-5p, miR-658, miR-542-5p, miR-668-3p, miR-766-3p, miR-675-5p, miR-297, let-7d-3p, miR-22-5p, miR-24-2-5p, miR-29a-5p, miR-92a-1-Sp, miR-92a-2-5p, miR-139-3p, miR-181c-3p, miR-187-5p, miR-124-5p, miR-125b-1-3p, miR-135a-3p, miR-138-1-3p, miR-149-3p, miR-185-3p, miR-186-3p, miR-193a-5p, miR-194-3p, miR-135b-3p, miR-339-3p, miR-423-5p, miR-18b-3p, miR-20b-3p, miR-483-5p, miR-486-3p, miR-501-3p, miR-532-3p, miR-92b-5p, miR-574-5p, miR-615-5p, miR-891a-5p, miR-876-3p, miR-744-5p, miR-885-5p, miR-885-3p, miR-877-3p, miR-887-3p, miR-665, miR-760, miR-936, miR-939-5p, miR-1224-5p, miR-1224-3p, miR-1225-5p, miR-1225-3p, miR-1228-5p, miR-1228-3p, miR-1231, miR-1234-3p, miR-1238-3p, miR-320c, miR-1181, miR-1202, miR-663b, miR-1204, miR-1207-5p, miR-1208, miR-1293, miR-1294, miR-1304-5p, miR-1249-3p, miR-1260a, miR-1266-5p, miR-1268a, miR-1270, miR-1278, miR-1281, miR-1292-5p, miR-1255b-5p, miR-1307-3p, miR-320d, miR-675-3p, miR-1469, miR-1471, miR-1538, miR-1908-5p, miR-1909-5p, miR-1909-3p, miR-1910-5p, miR-1912-3p, miR-1914-3p, miR-1915-3p, miR-365a-5p, miR-449b-3p, miR-1972, miR-1976, miR-762, miR-548q, miR-2276-3p, miR-711, miR-718, miR-3119, miR-3120-3p, miR-3130-5p, miR-3137, miR-3141, miR-1273c, miR-3147, miR-3148, miR-3151-5p, miR-3153, miR-3159, miR-3162-5p, miR-3163, miR-3165, miR-1260b, miR-1193, miR-3178, miR-3180-3p, miR-3184-5p, miR-3185, miR-3187-3p, miR-3191-3p, miR-3194-5p, miR-3195, miR-3196, miR-3197, miR-514b-5p, miR-4293, miR-4298, miR-4305, miR-4322, miR-4323, miR-4324, miR-4256, miR-4257, miR-4258, miR-4326, miR-4327, miR-4265, miR-4268, miR-4269, miR-4271, miR-4276, miR-4279, miR-4280, miR-4285, miR-500b-5p, miR-2277-5p, miR-3605-3p, miR-3610, miR-3616-3p, miR-3619-5p, miR-3621, miR-3622a-3p, miR-3646, miR-3648, miR-3652, miR-3663-5p, miR-3663-3p, miR-3665, miR-3677-3p, miR-3679-5p, miR-3180, miR-3907, miR-3911, miR-3916, miR-3917, miR-3919, miR-3926, miR-3928-3p, miR-3934-5p, miR-3937, miR-3941, miR-3944-3p, miR-1268b, miR-4428, miR-4429, miR-4430, miR-548ad-3p, miR-4433a-3p, miR-4436a, miR-4439, miR-4440, miR-4443, miR-4444, miR-4446-3p, miR-4447, miR-4448, miR-4449, miR-4455, miR-4456, miR-4458, miR-4460, miR-378h, miR-3135b, miR-4462, miR-4463, miR-4466, miR-4467, miR-4470, miR-4474-3p, miR-4475, miR-4478, miR-4481, miR-4483, miR-4484, miR-4486, miR-4487, miR-4488, miR-4489, miR-4492, miR-4496, miR-4497, miR-4498, miR-4505, miR-4506, miR-2392, miR-4507, miR-4508, miR-4513, miR-4516, miR-4519, miR-4520-3p, miR-4521, miR-4524a-3p, miR-4526, miR-4530, miR-4533, miR-4535, miR-1587, miR-4538, miR-4539, miR-4540, miR-3120-5p, miR-3173-5p, miR-3187-5p, miR-3922-5p, miR-3940-5p, miR-3960, miR-4634, miR-4638-5p, miR-4640-5p, miR-4648, miR-4649-5p, miR-4650-3p, miR-4651, miR-4653-3p, miR-4654, miR-4655-5p, miR-4656, miR-4659b-3p, miR-4665-5p, miR-4665-3p, miR-4668-5p, miR-4670-3p, miR-4673, miR-4674, miR-4676-5p, miR-4682, miR-4685-5p, miR-4685-3p, miR-4687-5p, miR-1343-3p, miR-4688, miR-4690-5p, miR-4691-5p, miR-4691-3p, miR-4695-5p, miR-4695-3p, miR-4697-5p, miR-4701-3p, miR-4706, miR-4707-5p, miR-4708-5p, miR-4708-3p, miR-4710, miR-4711-3p, miR-4721, miR-4722-5p, miR-4722-3p, miR-4724-5p, miR-4725-5p, miR-4725-3p, miR-4728-3p, miR-4730, miR-4732-3p, miR-4734, miR-4736, miR-3064-5p, miR-4738-3p, miR-4741, miR-4743-5p, miR-122b-3p, miR-4745-5p, miR-4746-3p, miR-4749-5p, miR-4750-5p, miR-4753-5p, miR-371b-5p, miR-4754, miR-4758-5p, miR-4758-3p, miR-4759, miR-4763-5p, miR-4763-3p, miR-4764-5p, miR-4769-3p, miR-4770, miR-4776-5p, miR-4776-3p, miR-4780, miR-4436b-5p, miR-4781-5p, miR-4783-3p, miR-4784, miR-1245b-5p, miR-2467-3p, miR-4787-5p, miR-4800-5p, miR-4482-3p, miR-4999-5p, miR-5001-5p, miR-5002-3p, miR-5006-5p, miR-5008-5p, miR-5010-5p, miR-5087, miR-5088-5p, miR-5089-5p, miR-5090, miR-5189-5p, miR-5190, miR-5196-5p, miR-5100, miR-5572, miR-5586-3p, miR-548au-3p, miR-5589-5p, miR-5684, miR-5685, miR-5690, miR-5698, miR-5703, miR-5708, miR-197-5p, miR-204-3p, miR-211-3p, miR-382-3p, miR-652-5p, miR-1247-3p, miR-3184-3p, miR-3191-5p, miR-365b-5p, miR-376c-5p, miR-381-5p, miR-937-5p, miR-1227-5p, miR-1233-5p, miR-1237-5p, miR-1238-5p, miR-3617-3p, miR-3620-5p, miR-4632-5p, miR-5787, miR-6068, miR-6069, miR-6071, miR-6072, miR-6075, miR-6076, miR-6083, miR-6086, miR-6088, miR-6089, miR-6090, miR-6124, miR-6125, miR-6126, miR-6127, miR-6131, miR-6132, miR-6133, miR-6165, miR-548ay-3p, miR-6501-3p, miR-6506-5p, miR-6509-3p, miR-6511a-5p, miR-6511a-3p, miR-6512-3p, miR-6513-5p, miR-6514-3p, miR-6515-5p, miR-6716-5p, miR-6716-3p, miR-6717-5p, miR-6511b-5p, miR-6511b-3p, miR-6719-3p, miR-6721-5p, miR-6722-5p, miR-6722-3p, miR-6724-5p, miR-208a-5p, miR-210-5p, miR-128-1-5p, miR-329-5p, miR-410-5p, miR-487a-5p, miR-504-3p, miR-585-5p, miR-598-5p, miR-1301-5p, miR-874-5p, miR-1908-3p, miR-1343-5p, miR-5189-3p, miR-6726-5p, miR-6727-5p, miR-6727-3p, miR-6729-5p, miR-6732-5p, miR-6735-5p, miR-6736-5p, miR-6736-3p, miR-6737-5p, miR-6738-5p, miR-6738-3p, miR-6740-5p, miR-6741-5p, miR-6743-5p, miR-6743-3p, miR-6747-5p, miR-6747-3p, miR-6748-5p, miR-6749-5p, miR-6750-3p, miR-6751-5p, miR-6752-5p, miR-6753-5p, miR-6754-5p, miR-6754-3p, miR-6756-5p, miR-6759-5p, miR-6761-3p, miR-6762-5p, miR-6762-3p, miR-6763-5p, miR-6765-5p, miR-6766-5p, miR-6767-5p, miR-6768-5p, miR-6770-3p, miR-6771-5p, miR-6772-5p, miR-6772-3p, miR-6773-3p, miR-6774-5p, miR-6775-5p, miR-6776-5p, miR-6777-5p, miR-6777-3p, miR-6778-5p, miR-6778-3p, miR-6779-5p, miR-6779-3p, miR-6781-5p, miR-6782-5p, miR-6783-3p, miR-6784-5p, miR-6785-5p, miR-6786-5p, miR-6787-5p, miR-6787-3p, miR-6789-5p, miR-6790-5p, miR-6791-5p, miR-6792-5p, miR-6792-3p, miR-6793-5p, miR-6794-5p, miR-6796-5p, miR-6797-5p, miR-6798-5p, miR-6799-5p, miR-6800-5p, miR-6800-3p, miR-6801-5p, miR-6803-5p, miR-6803-3p, miR-6804-3p, miR-6805-5p, miR-6805-3p, miR-6806-5p, miR-6807-5p, miR-6808-5p, miR-6809-5p, miR-6809-3p, miR-6810-5p, miR-6813-5p, miR-6813-3p, miR-6815-3p, miR-6816-5p, miR-6818-5p, miR-6820-5p, miR-6821-5p, miR-6822-5p, miR-6824-5p, miR-6825-5p, miR-6825-3p, miR-6829-5p, miR-6829-3p, miR-6832-5p, miR-6833-3p, miR-6834-5p, miR-6780b-5p, miR-6836-5p, miR-6836-3p, miR-6837-5p, miR-6838-5p, miR-6839-5p, miR-6840-3p, miR-6842-5p, miR-6845-5p, miR-6845-3p, miR-6846-5p, miR-6847-5p, miR-6847-3p, miR-6848-5p, miR-6849-5p, miR-6849-3p, miR-6850-5p, miR-6851-5p, miR-6855-5p, miR-6857-5p, miR-6858-5p, miR-6859-5p, miR-6769b-5p, miR-6860, miR-6861-5p, miR-6861-3p, miR-6862-3p, miR-6867-3p, miR-6869-5p, miR-6870-5p, miR-6871-5p, miR-6875-5p, miR-6875-3p, miR-6877-5p, miR-6877-3p, miR-6879-5p, miR-6879-3p, miR-6880-5p, miR-6880-3p, miR-6882-5p, miR-6882-3p, miR-6883-5p, miR-6884-5p, miR-6886-3p, miR-6887-3p, miR-6888-3p, miR-6889-5p, miR-6890-5p, miR-6891-5p, miR-6892-5p, miR-6893-5p, miR-6893-3p, miR-6894-5p, miR-6894-3p, miR-6895-5p, miR-7106-5p, miR-7107-5p, miR-7108-5p, miR-7109-5p, miR-7109-3p, miR-7110-5p, miR-7111-5p, miR-7112-5p, miR-7113-3p, miR-7114-5p, miR-7114-3p, miR-7150, miR-7151-3p, miR-7155-5p, miR-7157-3p, miR-7159-5p, miR-7160-5p, miR-7515, miR-7704, miR-4433b-3p, miR-1273h-5p, miR-7845-5p, miR-7846-3p, miR-7847-3p, miR-7851-3p, miR-8052, miR-8059, miR-8063, miR-8064, miR-8069, miR-8071, miR-8072, miR-8073, miR-8074, miR-8075, miR-8077, miR-8089, miR-7977, miR-7978, miR-203a-5p, miR-1249-5p, miR-4485-5p, miR-8485, miR-137-5p, miR-498-3p, miR-9718, miR-9985, miR-1843, miR-10392-5p, miR-10392-3p, miR-10394-3p, miR-10396a-5p, miR-10396a-3p, miR-10398-5p, miR-10398-3p, miR-10400-5p, miR-10400-3p, miR-10401-5p, miR-10401-3p, miR-10396b-5p, miR-10522-5p, miR-10527-5p, miR-11399, miR-11400, miR-11401, miR-3085-5p, miR-3085-3p, miR-6529-5p, miR-12114, miR-12115, miR-12118, miR-12119, miR-12120, miR-12121, miR-12128, and miR-12131. In the present disclosure, the extract of urine may be an extract of the urine of a stage-I or -II colorectal cancer patient. Only one of these microRNAs can be used to predict whether a subject from which the urine is derived is a colorectal cancer patient (particularly stage-I or -II), and 5 or more types, 10 or more types, 15 or more types, or 20 or more types of these microRNAs can be used in combination to predict whether a subject from which the urine is derived is a colorectal cancer patient (particularly stage-I or -II). Among these microRNAs, a microRNA that may be highly expressed in a colorectal cancer patient (particularly stage-I or -II) can be preferably used for prediction.

The present disclosure provides an extract of urine containing at least one type, two types, three types, four types, five types, six types, seven types, eight types, nine types, or ten types of or all microRNAs selected from the group consisting of miR-15a-5p, miR-30a-5p, miR-124-3p, miR-373-5p, miR-373-3p, miR-325, miR-202-3p, miR-492, miR-520c-3p, miR-516b-5p, miR-552-3p, miR-557, miR-585-3p, miR-617, miR-638, miR-639, miR-663a, miR-449b-5p, miR-542-5p, miR-769-5p, miR-297, miR-92a-2-5p, miR-187-5p, miR-200b-5p, miR-124-5p, miR-130a-5p, miR-149-3p, miR-148b-5p, miR-18b-3p, miR-92b-5p, miR-624-3p, miR-629-5p, miR-876-3p, miR-665, miR-1224-3p, miR-1225-5p, miR-1225-3p, miR-1228-3p, miR-1238-3p, miR-320c, miR-1202, miR-1207-5p, miR-1208, miR-1294, miR-1304-5p, miR-1249-3p, miR-1268a, miR-1270, miR-1292-5p, miR-1469, miR-1908-5p, miR-1909-3p, miR-1912-3p, miR-1915-3p, miR-3119, miR-3120-3p, miR-3141, miR-1273c, miR-3148, miR-3163, miR-3165, miR-3178, miR-3180-3p, miR-3184-5p, miR-3196, miR-3197, miR-4293, miR-4294, miR-4298, miR-4321, miR-4323, miR-4256, miR-4258, miR-4327, miR-4271, miR-4280, miR-500b-5p, miR-3648, miR-3654, miR-3663-3p, miR-3677-3p, miR-3688-3p, miR-3916, miR-3919, miR-3928-3p, miR-3937, miR-3941, miR-642b-3p, miR-1268b, miR-548ab, miR-4422, miR-4433a-3p, miR-4439, miR-4446-3p, miR-4447, miR-4458, miR-4460, miR-378h, miR-3135b, miR-4463, miR-4466, miR-4467, miR-4470, miR-4484, miR-4492, miR-4497, miR-4505, miR-4507, miR-4508, miR-4520-3p, miR-4521, miR-4530, miR-4634, miR-4640-5p, miR-4650-3p, miR-4651, miR-4653-3p, miR-4659b-3p, miR-4665-5p, miR-4665-3p, miR-4668-5p, miR-4672, miR-4674, miR-4685-5p, miR-4687-5p, miR-4688, miR-4706, miR-4707-5p, miR-4716-5p, miR-4725-3p, miR-4728-3p, miR-4734, miR-4741, miR-4745-5p, miR-4749-5p, miR-4753-5p, miR-4758-5p, miR-4759, miR-4763-3p, miR-4770, miR-4779, miR-4781-5p, miR-4783-3p, miR-4999-5p, miR-5087, miR-5196-5p, miR-5586-3p, miR-548au-3p, miR-5684, miR-5690, miR-197-5p, miR-382-3p, miR-652-5p, miR-1185-2-3p, miR-3529-3p, miR-381-5p, miR-937-5p, miR-1227-5p, miR-1237-5p, miR-3620-5p, miR-3934-3p, miR-4632-5p, miR-6069, miR-6071, miR-6075, miR-6081, miR-6086, miR-6090, miR-6124, miR-6125, miR-6126, miR-6132, miR-548ay-3p, miR-6506-5p, miR-6510-5p, miR-6721-5p, miR-6722-3p, miR-6724-5p, miR-210-5p, miR-329-5p, miR-410-5p, miR-1343-5p, miR-6729-5p, miR-6729-3p, miR-6732-5p, miR-6743-5p, miR-6752-5p, miR-6755-5p, miR-6756-5p, miR-6763-5p, miR-6763-3p, miR-6768-5p, miR-6774-5p, miR-6775-5p, miR-6777-3p, miR-6781-5p, miR-6784-5p, miR-6784-3p, miR-6785-3p, miR-6786-5p, miR-6787-5p, miR-6789-5p, miR-6791-5p, miR-6797-5p, miR-6798-5p, miR-6799-5p, miR-6800-3p, miR-6805-5p, miR-6809-5p, miR-6810-3p, miR-6813-5p, miR-6818-5p, miR-6819-3p, miR-6821-5p, miR-6824-5p, miR-6829-5p, miR-6832-5p, miR-6780b-5p, miR-6836-3p, miR-6838-5p, miR-6839-5p, miR-6840-3p, miR-6845-5p, miR-6845-3p, miR-6848-5p, miR-6850-5p, miR-6858-5p, miR-6861-5p, miR-6875-5p, miR-6880-5p, miR-6880-3p, miR-6882-5p, miR-6887-3p, miR-6892-3p, miR-7107-5p, miR-7108-5p, miR-7111-5p, miR-7150, miR-7515, miR-4433b-3p, miR-7847-3p, miR-8072, miR-8074, miR-203a-5p, miR-1249-5p, miR-498-3p, miR-9898, miR-9985, miR-10392-5p, miR-10392-3p, miR-10396a-5p, miR-10396a-3p, miR-10398-3p, miR-10400-3p, miR-10396b-5p, miR-3085-3p, and miR-12120. In the present disclosure, the extract of urine may be an extract of the urine of a stage-I colorectal cancer patient. Only one of these microRNAs can be used to predict whether a subject from which the urine is derived is a colorectal cancer patient (particularly stage-I), and 5 or more types, 10 or more types, 15 or more types, or 20 or more types of these microRNAs can be used in combination to predict whether a subject from which the urine is derived is a colorectal cancer patient (particularly stage-I). Among these microRNAs, a microRNA that may be highly expressed in a colorectal cancer patient (particularly stage-I) can be preferably used for prediction.

The present disclosure provides an extract of urine containing at least one type, two types, three types, four types, five types, six types, seven types, eight types, nine types, or ten types of or all microRNAs selected from the group consisting of microRNAs having an accuracy of higher than 0.500 among the microRNAs listed in Table 24-14. In the present disclosure, the extract of urine may be an extract of the urine of a colorectal cancer patient. Only one of these microRNAs can be used to predict whether a subject from which the urine is derived is a colorectal cancer patient, and 5 or more types, 10 or more types, 15 or more types, or 20 or more types of these microRNAs can be used in combination to predict whether a subject from which the urine is derived is a colorectal cancer patient. Among these microRNAs, a microRNA that may be highly expressed in a colorectal cancer patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

The present disclosure provides an extract of urine containing at least one type, two types, three types, four types, five types, six types, seven types, eight types, nine types, or ten types of or all microRNAs selected from the group consisting of microRNAs having an accuracy of higher than 0.500 among the microRNAs listed in Table 25-14. In the present disclosure, the extract of urine may be an extract of the urine of a colorectal cancer patient. Only one of these microRNAs can be used to predict whether a subject from which the urine is derived is a colorectal cancer patient, and 5 or more types, 10 or more types, 15 or more types, or 20 or more types of these microRNAs can be used in combination to predict whether a subject from which the urine is derived is a colorectal cancer patient. Among these microRNAs, a microRNA that may be highly expressed in a colorectal cancer patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

The present disclosure provides an extract of urine containing at least one type, two types, three types, four types, five types, six types, seven types, eight types, nine types, or ten types of or all microRNAs selected from the microRNAs listed in Table 4-15. The present disclosure also provides an extract of urine containing at least one type, two types, three types, four types, five types, six types, seven types, eight types, nine types, or ten types of or all microRNAs selected from the group consisting of miR-4280, miR-6809-5p, miR-7515, miR-6750-5p, miR-1268b, miR-541-3p, miR-551b-5p, miR-4321, miR-601, miR-1292-5p, miR-4458, miR-184, miR-125b-2-3p, miR-20b-3p, miR-5007-5p, miR-3691-5p, miR-4450, miR-6783-5p, miR-6761-5p, miR-4433a-3p, miR-4465, miR-4651, miR-6081, miR-1343-5p, miR-6131, miR-320c, miR-4745-5p, miR-492, miR-6129, miR-3682-3p, miR-937-5p, miR-424-3p, miR-4786-3p, miR-1908-5p, miR-6791-5p, miR-10400-3p, miR-338-5p, miR-6125, miR-6789-5p, miR-5004-3p, miR-92b-5p, miR-4727-3p, miR-3130-3p, miR-4684-3p, miR-3937, miR-92a-2-5p, miR-4506, miR-6781-5p, miR-10526-3p, miR-4327, miR-12127, miR-1910-3p, miR-4672, miR-5093, miR-8078, miR-5196-5p, miR-1914-3p, miR-6732-5p, miR-761, miR-6798-5p, miR-4433b-3p, miR-6127, miR-3648, miR-1207-5p, miR-1227-5p, miR-3199, miR-1225-5p, miR-6805-5p, miR-5187-5p, miR-3620-5p, miR-6794-5p, miR-4507, miR-6743-5p, miR-6821-5p, miR-1912-3p, miR-3197, miR-6501-5p, miR-4749-5p, miR-6832-5p, miR-3059-3p, miR-4758-5p, miR-4505, miR-6829-5p, miR-4300, miR-4257, miR-6845-5p, miR-7854-3p, miR-3161, miR-6839-5p, miR-629-5p, miR-665, miR-8072, miR-6838-5p, miR-202-3p, miR-4258, miR-3651, miR-3129-3p, miR-3675-5p, miR-4447, miR-638, miR-4665-5p, miR-3141, miR-939-5p, miR-4720-5p, miR-211-3p, miR-381-5p, miR-4695-5p, miR-12120, miR-135a-2-3p, miR-4725-3p, miR-6746-5p, miR-10401-5p, miR-7156-3p, miR-12119, miR-6840-3p, miR-6514-5p, miR-8057, miR-4657, miR-4521, miR-6850-5p, miR-766-5p, miR-6763-5p, miR-200b-3p, miR-4255, miR-4451, miR-3162-5p, miR-4530, miR-12122, miR-4763-3p, miR-4314, miR-6721-5p, miR-4303, miR-10392-5p, miR-10396a-3p, miR-5194, miR-382-5p, miR-297, miR-7111-5p, miR-3689b-3p, miR-3689c, miR-4634, miR-6752-5p, miR-5708, miR-1469, miR-5703, miR-6130, miR-9986, miR-542-5p, miR-1303, miR-3934-3p, miR-888-3p, miR-4737, miR-6074, miR-6770-3p, miR-3185, miR-8080, miR-6071, miR-3165, miR-4294, miR-6724-5p, miR-1915-3p, miR-1298-3p, miR-6799-5p, miR-4293, miR-6893-5p, miR-4466, miR-4640-5p, miR-6804-5p, miR-4741, miR-378i, miR-5091, miR-6780b-5p, miR-3919, miR-6855-5p, miR-6719-3p, miR-550b-2-5p, miR-6814-5p, miR-6124, miR-4454, miR-4701-3p, miR-6788-5p, miR-4436b-3p, miR-4484, miR-6770-5p, miR-3155a, miR-7110-5p, miR-6787-5p, miR-6749-5p, miR-487a-5p, miR-6820-5p, miR-3654, miR-3124-5p, miR-4688, miR-6729-5p, miR-7154-3p, miR-921, miR-1293, miR-610, miR-4648, miR-4453, miR-3150b-3p, miR-6504-5p, miR-760, miR-3660, miR-138-5p, miR-1268a, miR-10396b-5p, miR-1286, miR-3192-5p, miR-498-5p, miR-6895-5p, miR-593-3p, miR-6513-5p, miR-2682-5p, miR-4494, miR-4429, miR-187-5p, miR-197-5p, miR-6845-3p, miR-206, miR-150-3p, miR-4510, miR-3605-5p, miR-10392-3p, miR-4311, miR-6758-5p, miR-6500-3p, miR-668-5p, miR-550a-3-5p, miR-3934-5p, miR-6715b-5p, miR-769-5p, miR-29a-3p, miR-6796-3p, miR-4439, miR-4785, miR-6836-5p, miR-200a-5p, miR-378g, miR-6823-5p, miR-373-3p, miR-6859-3p, miR-1322, miR-617, miR-487a-3p, miR-6842-3p, miR-3180-3p, miR-4654, miR-3652, miR-4687-5p, miR-4467, miR-6748-5p, miR-5572, miR-4423-5p, miR-656-5p, miR-4676-5p, miR-6805-3p, miR-6767-5p, miR-489-5p, miR-4682, miR-8083, miR-7108-5p, miR-4437, miR-6756-5p, miR-4317, miR-510-5p, miR-598-5p, miR-135a-3p, miR-5698, miR-6501-3p, miR-6836-3p, miR-4707-5p, miR-6776-5p, miR-7114-5p, miR-4632-5p, miR-6722-3p, miR-5702, miR-6763-3p, miR-6769b-5p, miR-1276, miR-4733-3p, miR-4431, miR-1321, miR-4508, miR-1228-3p, miR-6511a-5p, miR-6879-5p, miR-6870-5p, miR-1224-3p, miR-4538, miR-8075, miR-1251-3p, miR-193b-5p, miR-551b-3p, miR-3127-5p, miR-4472, miR-3655, miR-6889-5p, miR-6515-5p, miR-3944-5p, miR-8064, miR-4261, miR-12118, miR-664b-5p, miR-1271-5p, miR-892c-5p, miR-6871-5p, miR-7113-5p, miR-10396a-5p, miR-6859-5p, miR-4529-5p, miR-210-5p, miR-6825-5p, miR-1909-3p, miR-605-3p, miR-8070, miR-6729-3p, miR-6076, miR-3675-3p, miR-1972, miR-584-3p, miR-6810-3p, miR-3074-3p, miR-3610, miR-6513-3p, miR-4540, miR-558, miR-493-3p, miR-7150, miR-323a-3p, miR-1238-3p, miR-8082, miR-3195, miR-4323, miR-6848-5p, miR-608, miR-324-5p, miR-6874-5p, miR-4446-3p, miR-1270, miR-6747-5p, miR-6868-5p, miR-595, miR-4794, miR-3529-5p, miR-147a, miR-1471, miR-4731-3p, miR-9898, miR-6090, miR-3679-3p, miR-6786-5p, miR-23b-3p, miR-25-5p, miR-12114, miR-4299, miR-5189-5p, miR-6080, miR-1913, miR-4644, miR-7850-5p, miR-7107-5p, miR-370-3p, miR-3918, miR-6083, miR-8089, miR-320e, miR-6880-5p, miR-6516-5p, miR-6795-5p, miR-5787, miR-6813-5p, miR-4430, miR-625-3p, miR-6760-3p, miR-6768-5p, miR-1285-3p, miR-6813-3p, miR-4673, miR-6784-3p, miR-6797-5p, miR-762, miR-4728-3p, miR-4492, miR-217-3p, miR-6887-5p, miR-6085, miR-525-3p, miR-4433a-5p, miR-1225-3p, miR-8069, miR-4497, miR-1249-3p, miR-3178, miR-4734, miR-5681b, miR-12116, miR-4754, miR-4716-3p, miR-6892-3p, miR-4635, miR-758-5p, miR-4479, miR-4436a, miR-6816-5p, miR-4529-3p, miR-6887-3p, miR-193a-5p, miR-4750-3p, miR-181d-3p, miR-3187-3p, miR-4716-5p, miR-6505-3p, miR-7856-5p, miR-5580-5p, miR-1185-1-3p, miR-4776-3p, miR-5585-3p, miR-6847-5p, miR-6861-5p, miR-1237-5p, miR-6510-5p, miR-6500-5p, miR-6830-5p, miR-3196, miR-103a-2-5p, miR-6800-3p, miR-6875-5p, miR-3690, miR-711, miR-11181-3p, miR-3186-5p, miR-2467-3p, miR-6790-5p, miR-6801-5p, miR-6795-3p, miR-1236-5p, miR-10398-3p, miR-6515-3p, miR-7151-3p, miR-6817-5p, miR-361-3p, miR-6782-5p, miR-4502, miR-5190, miR-4999-5p, miR-4658, miR-4783-3p, miR-5704, miR-4784, miR-6880-3p, miR-4674, miR-3137, miR-6777-5p, miR-5090, miR-4681, miR-6777-3p, miR-4486, miR-3125, miR-660-3p, miR-4520-5p, miR-5685, miR-34c-5p, miR-11399, miR-6511b-5p, miR-6766-3p, miR-3922-5p, miR-4749-3p, miR-8074, miR-4463, miR-652-5p, miR-7851-3p, miR-6798-3p, miR-105-5p, miR-6850-3p, miR-449c-5p, miR-3131, miR-6134, miR-486-3p, miR-4692, miR-4665-3p, miR-6784-5p, miR-4535, miR-3170, miR-6730-5p, miR-5006-5p, miR-615-5p, miR-8085, miR-9718, miR-6499-3p, miR-3940-5p, miR-3659, miR-6165, miR-3689f, miR-513b-5p, miR-663a, miR-4283, miR-4296, miR-3621, miR-6772-5p, miR-6884-5p, miR-4274, miR-4489, miR-34b-3p, miR-1250-5p, miR-12117, miR-6504-3p, miR-3689a-3p, miR-34b-5p, miR-4717-5p, miR-7109-5p, miR-604, miR-500a-5p, miR-1185-2-3p, miR-3917, miR-7114-3p, miR-6089, miR-6086, miR-1301-5p, miR-382-3p, miR-516b-3p, miR-516a-3p, miR-4711-3p, miR-6764-5p, miR-5004-5p, miR-4470, miR-4520-3p, miR-6831-5p, miR-6069, miR-7975, miR-3614-5p, miR-563, miR-718, miR-6778-5p, miR-3619-5p, let-7d-3p, miR-449b-5p, miR-6726-5p, miR-1285-5p, miR-4474-3p, miR-4525, miR-1587, miR-3135b, miR-4441, miR-204-3p, miR-769-3p, miR-4534, miR-6509-5p, miR-1301-3p, miR-3929, miR-4755-3p, miR-6718-5p, miR-3661, miR-6819-3p, miR-433-5p, miR-642a-3p, miR-6815-5p, miR-433-3p, miR-3190-3p, miR-4728-5p, miR-4645-3p, miR-7106-5p, miR-5010-5p, miR-4513, miR-410-5p, miR-557, miR-6759-5p, miR-6842-5p, miR-4286, miR-6068, miR-4295, miR-3169, miR-4713-3p, miR-4686, miR-891a-5p, miR-4708-5p, miR-6785-3p, miR-196a-5p, miR-4516, miR-106a-3p, miR-29c-5p, miR-4786-5p, miR-149-3p, miR-3617-3p, miR-4756-3p, miR-196b-5p, miR-575, miR-6846-5p, miR-6738-5p, miR-4433b-5p, miR-1294, miR-300, miR-4456, miR-3928-3p, miR-210-3p, miR-219a-2-3p, miR-5690, miR-5739, miR-138-1-3p, miR-4491, miR-2115-5p, miR-1226-5p, miR-6800-5p, miR-554, miR-4724-5p, miR-4483, miR-4708-3p, miR-6132, miR-3665, miR-6738-3p, miR-3692-5p, miR-147b-3p, miR-5189-3p, miR-3663-3p, miR-3186-3p, miR-5188, miR-5192, miR-4773, miR-6854-3p, miR-6883-5p, miR-342-5p, miR-1231, miR-4769-3p, miR-4498, miR-4289, miR-500b-3p, miR-3927-5p, miR-323b-5p, miR-3085-3p, miR-6503-3p, miR-4514, miR-8077, miR-4647, miR-6727-5p, miR-4253, miR-4655-5p, miR-4262, miR-4435, miR-504-3p, miR-4455, miR-6133, miR-940, miR-30b-3p, miR-650, miR-4793-5p, miR-3187-5p, miR-5588-3p, miR-512-3p, miR-3666, miR-874-5p, miR-1468-5p, miR-6876-3p, miR-6126, miR-1288-3p, miR-4769-5p, miR-7151-5p, miR-4800-3p, miR-4496, miR-7976, miR-6717-5p, miR-6802-3p, miR-525-5p, miR-422a, miR-6869-5p, miR-12124, miR-6797-3p, miR-6773-3p, miR-517a-3p, miR-517b-3p, miR-6075, miR-589-3p, miR-6737-5p, miR-4778-5p, miR-3148, miR-5006-3p, miR-99b-3p, miR-5089-3p, miR-4787-5p, miR-4524b-3p, miR-5087, miR-4485-5p, miR-4315, miR-365a-5p, miR-4740-5p, miR-892a, miR-4748, miR-6779-5p, miR-1184, miR-3173-5p, miR-4304, miR-4276, miR-1306-3p, miR-3679-5p, miR-4677-5p, miR-1237-3p, miR-4478, miR-3064-5p, miR-4487, miR-185-3p, miR-6821-3p, miR-943, miR-564, miR-146b-3p, miR-4758-3p, miR-4481, miR-4271, miR-3162-3p, miR-494-5p, miR-6793-5p, miR-10395-5p, miR-6762-3p, miR-4324, miR-4723-5p, miR-134-3p, miR-3180, miR-7704, miR-7162-3p, miR-1224-5p, miR-491-5p, miR-6766-5p, miR-7109-3p, miR-885-3p, miR-885-5p, miR-7152-5p, miR-616-5p, miR-6895-3p, miR-619-5p, miR-4449, miR-296-5p, miR-3147, miR-6876-5p, miR-635, miR-4736, miR-214-5p, miR-345-5p, miR-5684, miR-7160-5p, miR-935, miR-4638-5p, miR-4428, miR-7157-3p, miR-3622b-5p, miR-6734-5p, miR-3944-3p, miR-1266-3p, miR-1307-3p, miR-431-3p, miR-6740-5p, miR-887-3p, miR-572, miR-3685, miR-10522-5p, miR-668-3p, miR-6824-5p, miR-4669, miR-361-5p, miR-6772-3p, miR-3680-5p, miR-3151-5p, miR-200b-5p, miR-222-5p, miR-301a-5p, miR-6742-5p, miR-6529-5p, miR-4537, miR-4753-3p, miR-3943, miR-6858-3p, miR-658, miR-4313, miR-1204, miR-6752-3p, miR-4308, miR-3935, miR-6822-3p, miR-4709-5p, miR-187-3p, miR-4499, miR-7108-3p, miR-194-3p, miR-3691-3p, miR-6720-5p, miR-3619-3p, miR-1249-5p, miR-323a-5p, miR-16-l-3p, miR-892c-3p, miR-1909-5p, miR-6855-3p, miR-3120-5p, miR-4488, miR-6807-5p, miR-3646, miR-4259, miR-4694-3p, miR-6756-3p, miR-330-5p, miR-6764-3p, miR-11400, miR-5002-5p, miR-6875-3p, miR-200c-5p, miR-10398-5p, miR-892b, miR-103a-1-5p, miR-4738-3p, miR-4322, miR-15a-3p, miR-18b-3p, miR-3677-3p, miR-877-5p, let-7b-5p, miR-6868-3p, miR-938, miR-5002-3p, miR-3122, miR-6731-3p, miR-4297, miR-4768-3p, miR-3622a-5p, miR-4639-3p, miR-6506-5p, miR-541-5p, miR-1202, miR-371b-5p, miR-4524a-5p, miR-3678-3p, miR-4660, miR-6787-3p, miR-4633-5p, miR-765, miR-484, miR-497-5p, miR-6812-5p, miR-1298-5p, miR-583, miR-139-3p, miR-3922-3p, miR-4653-5p, miR-6861-3p, miR-1247-3p, miR-6741-5p, miR-6891-3p, miR-1255b-2-3p, miR-4252, miR-3160-5p, miR-1228-5p, miR-494-3p, miR-16-2-3p, miR-548g-3p, miR-4526, miR-515-5p, miR-18a-3p, miR-1843, miR-8081, miR-4533, miR-4298, miR-7977, miR-10523-5p, miR-8088, miR-1269a, miR-4256, miR-6849-5p, miR-8071, miR-466, miR-6759-3p, miR-6765-5p, miR-342-3p, miR-498-3p, miR-6754-3p, miR-8485, miR-4641, miR-4284, miR-4700-5p, miR-4750-5p, miR-942-5p, miR-3649, miR-2392, miR-4676-3p, miR-3158-3p, miR-3928-5p, miR-365a-3p, miR-365b-3p, miR-5000-3p, miR-6888-5p, miR-5009-3p, miR-4316, miR-4691-5p, miR-7846-3p, miR-4707-3p, miR-4436b-5p, miR-4482-3p, miR-6715a-3p, miR-6848-3p, miR-3200-5p, miR-4475, miR-661, miR-2277-5p, miR-7112-3p, miR-432-5p, miR-363-5p, miR-3936, miR-370-5p, miR-642b-3p, miR-3142, miR-654-5p, miR-654-3p, miR-4685-5p, miR-5589-5p, miR-1273c, miR-744-3p, miR-1238-5p, miR-4730, miR-3158-5p, miR-196b-3p, miR-6760-5p, miR-3184-3p, miR-5584-3p, miR-5000-5p, miR-3138, miR-3157-5p, miR-3663-5p, miR-6512-3p, miR-6882-3p, miR-585-5p, miR-6762-5p, miR-889-5p, miR-506-3p, miR-192-5p, miR-612, miR-671-5p, miR-7113-3p, miR-5581-5p, miR-6716-5p, miR-622, miR-3177-3p, miR-183-3p, miR-198, miR-25-3p, miR-3909, miR-2116-3p, miR-485-5p, miR-2110, miR-4780, miR-3192-3p, miR-4265, miR-129-5p, miR-378a-3p, miR-4420, miR-4722-3p, miR-4685-3p, miR-5187-3p, miR-4656, miR-138-2-3p, miR-4440, miR-4753-5p, miR-3945, miR-4425, miR-4747-5p, miR-31-3p, miR-6834-5p, miR-3155b, miR-4664-5p, miR-378j, miR-449c-3p, miR-4766-5p, miR-122b-3p, miR-1538, let-7c-3p, miR-3176, miR-6747-3p, miR-5588-5p, miR-1281, miR-632, miR-2276-3p, miR-27b-3p, miR-151a-5p, miR-593-5p, miR-4733-5p, miR-4531, miR-377-5p, miR-6853-5p, miR-6894-3p, miR-513c-5p, miR-6809-3p, miR-4783-5p, miR-614, miR-6827-5p, miR-2681-3p, miR-6830-3p, miR-325, miR-6877-3p, miR-26b-3p, miR-1181, miR-4667-5p, miR-181a-2-3p, miR-345-3p, miR-6885-3p, miR-6796-5p, miR-6879-3p, miR-6883-3p, miR-6740-3p, miR-4726-3p, miR-5100, miR-128-1-5p, miR-3149, miR-4767, miR-513a-5p, miR-3907, miR-584-5p, miR-6826-3p, miR-130b-3p, miR-3667-5p, miR-6803-5p, miR-6786-3p, miR-296-3p, miR-6891-5p, miR-6804-3p, miR-1976, miR-580-3p, miR-744-5p, miR-154-5p, miR-3126-3p, miR-6789-3p, miR-3154, miR-1910-5p, miR-551a, miR-3189-3p, miR-34c-3p, miR-371b-3p, miR-516a-5p, miR-11401, miR-6755-3p, miR-6852-5p, miR-6791-3p, miR-3916, miR-6775-5p, miR-6856-5p, miR-920, miR-767-5p, miR-3150a-3p, miR-28-5p, miR-1233-5p, miR-6739-3p, miR-454-5p, miR-3065-3p, miR-521, miR-1260b, miR-6889-3p, miR-6792-3p, miR-32-3p, miR-6761-3p, miR-509-3p, miR-937-3p, miR-6870-3p, miR-548ab, miR-4743-5p, miR-12121, miR-4732-3p, miR-605-5p, miR-6886-3p, miR-5088-3p, miR-4254, miR-4776-5p, miR-602, miR-7847-3p, miR-34a-5p, miR-1273h-5p, miR-486-5p, miR-330-3p, miR-3650, miR-6751-5p, miR-6803-3p, miR-10394-3p, miR-942-3p, miR-4690-3p, miR-4793-3p, miR-4653-3p, miR-5088-5p, miR-3667-3p, miR-1539, miR-4691-3p, miR-3713, miR-4650-3p, miR-221-3p, miR-4664-3p, miR-548d-3p, miR-8086, miR-3074-5p, miR-411-3p, miR-4687-3p, miR-8087, miR-216a-3p, miR-483-5p, miR-4746-3p, miR-520b-5p, miR-519a-2-5p, miR-3064-3p, miR-6820-3p, miR-3191-5p, miR-1257, miR-185-5p, miR-6780b-3p, miR-6837-5p, miR-6769a-5p, miR-8063, miR-6852-3p, miR-3191-3p, miR-6857-3p, miR-10394-5p, miR-7706, miR-6514-3p, miR-4260, miR-675-3p, miR-365b-5p, miR-4285, miR-676-5p, miR-6886-5p, miR-1178-3p, miR-766-3p, miR-10400-5p, miR-3714, miR-6867-3p, miR-6722-5p, miR-6856-3p, miR-3913-3p, miR-4800-5p, miR-340-3p, miR-4445-3p, miR-214-3p, miR-4706, miR-4721, miR-6862-3p, miR-449b-3p, miR-4801, miR-3622a-3p, miR-3960, miR-495-5p, miR-12126, miR-3152-5p, miR-6511a-3p, miR-4797-3p, miR-3689d, miR-645, miR-548q, miR-6810-5p, miR-337-3p, miR-6779-3p, miR-6072, miR-4697-3p, miR-3924, miR-6828-5p, miR-6728-3p, miR-3157-3p, miR-6858-5p, miR-3612, miR-2909, miR-877-3p, miR-4684-5p, miR-23a-5p, miR-6730-3p, miR-4667-3p, miR-6749-3p, miR-6735-3p, miR-4269, miR-4288, miR-579-5p, miR-6863, miR-550a-5p, miR-1273h-3p, miR-500a-3p, miR-6833-5p, miR-519c-5p, miR-523-5p, miR-518e-5p, miR-522-5p, miR-519a-5p, miR-519b-5p, miR-3144-5p, miR-4724-3p, miR-4511, miR-503-3p, miR-3132, miR-4739, miR-4524b-5p, miR-3130-5p, miR-3156-3p, miR-181b-3p, miR-5699-5p, miR-1193, miR-4694-5p, miR-616-3p, miR-10397-5p, miR-550b-3p, miR-4700-3p, miR-12128, miR-4725-5p, miR-4421, miR-4796-5p, miR-4713-5p, miR-211-5p, miR-628-3p, miR-6860, miR-6776-3p, miR-1296-3p, miR-4743-3p, miR-1207-3p, miR-5591-3p, miR-450a-5p, miR-208a-5p, miR-378b, miR-6743-3p, miR-7111-3p, miR-3173-3p, miR-3657, miR-6892-5p, miR-6732-3p, miR-30c-2-3p, miR-1265, miR-4723-3p, miR-4443, miR-1236-3p, miR-4714-5p, miR-6511b-3p, miR-3975, miR-1203, miR-6750-3p, miR-378h, miR-3150a-5p, miR-10527-5p, miR-4745-3p, miR-106b-3p, miR-6843-3p, miR-375-3p, miR-6529-3p, miR-6727-3p, miR-4319, miR-125b-5p, miR-629-3p, miR-6792-5p, miR-487b-5p, miR-615-3p, miR-6769a-3p, miR-4781-5p, miR-4438, miR-1267, miR-186-3p, miR-637, miR-6878-3p, miR-328-5p, miR-4325, miR-939-3p, miR-106b-5p, miR-7155-5p, miR-500b-5p, miR-1304-5p, miR-4710, miR-6825-3p, miR-874-3p, miR-4306, miR-4539, miR-7845-5p, miR-371a-3p, miR-335-3p, miR-499b-3p, miR-30a-3p, miR-6823-3p, miR-4307, miR-1269b, miR-5580-3p, miR-1263, miR-5693, miR-6499-5p, miR-6753-5p, miR-5571-3p, miR-1260a, miR-4305, miR-642b-5p, miR-7158-5p, miR-194-5p, miR-1200, miR-8052, miR-873-3p, miR-4646-3p, miR-7110-3p, miR-6741-3p, miR-1825, miR-448, miR-9851-5p, miR-634, miR-676-3p, miR-4761-3p, miR-3193, miR-2113, miR-579-3p, miR-1266-5p, miR-5193, miR-574-5p, miR-3151-3p, miR-485-3p, miR-4326, miR-6817-3p, miR-4695-3p, miR-1284, miR-5001-5p, miR-675-5p, miR-520e-3p, miR-3059-5p, miR-3202, miR-12115, miR-1229-5p, miR-6742-3p, miR-6716-3p, miR-488-5p, miR-7974, miR-6780a-3p, miR-346, miR-6833-3p, miR-764, miR-449a, miR-520b-3p, miR-6871-3p, miR-10401-3p, miR-4328, miR-141-5p, miR-6726-3p, miR-6832-3p, miR-639, miR-1233-3p, miR-6849-3p, miR-6877-5p, miR-6890-3p, miR-6808-5p, miR-6893-3p, miR-6831-3p, miR-6780a-5p, miR-6806-5p, miR-4281, miR-3972, miR-6846-3p, miR-4722-5p, miR-644a, miR-338-3p, miR-1915-5p, miR-30b-5p, miR-4646-5p, miR-5705, miR-151a-3p, miR-6728-5p, miR-4697-5p, miR-4999-3p, miR-3174, miR-6778-3p, miR-1291, miR-212-5p, miR-151b, miR-6782-3p, miR-574-3p, miR-7155-3p, miR-7702, miR-6862-5p, miR-328-3p, miR-4640-3p, miR-6781-3p, miR-664b-3p, miR-532-3p, miR-6774-3p, miR-6894-5p, miR-6851-3p, miR-1229-3p, miR-4642, let-7e-5p, miR-196a-1-3p, miR-4330, miR-501-5p, miR-4701-5p, miR-4732-5p, miR-3680-3p, miR-5584-5p, miR-891a-3p, miR-6801-3p, miR-6851-5p, miR-4661-3p, miR-670-5p, miR-1287-5p, miR-1250-3p, miR-3620-3p, miR-4473, miR-3925-3p, miR-489-3p, miR-4650-5p, miR-5195-5p, miR-125b-1-3p, miR-8062, miR-7112-5p, miR-373-5p, miR-5694, miR-6783-3p, miR-3085-5p, miR-329-5p, miR-548ba, miR-4268, miR-603, miR-4448, miR-7162-5p, miR-6077, miR-4763-5p, miR-6881-5p, miR-141-3p, miR-6754-5p, miR-6857-5p, miR-4652-5p, miR-550a-3p, miR-3153, miR-6829-3p, miR-671-3p, miR-1304-3p, let-7a-2-3p, miR-1306-5p, miR-3616-3p, miR-514b-3p, miR-5195-3p, miR-708-5p, miR-7106-3p, miR-6755-5p, miR-29b-3p, miR-3119, miR-12125, miR-6748-3p, miR-3614-3p, miR-4690-5p, miR-1275, miR-6774-5p, miR-146a-5p, miR-92a-3p, miR-3198, miR-1343-3p, miR-4273, miR-197-3p, miR-6884-3p, miR-6867-5p, miR-502-5p, miR-130b-5p, miR-1288-5p, miR-4320, miR-519e-3p, miR-7855-5p, miR-4512, miR-6819-5p, miR-6816-3p, miR-8079, miR-4762-5p, miR-4804-3p, miR-10524-5p, miR-302d-5p, miR-219b-5p, miR-21-3p, miR-3188, miR-4266, miR-4740-3p, miR-4659b-3p, miR-421, miR-6757-5p, miR-7159-5p, miR-4279, miR-5001-3p, miR-9899, miR-7843-5p, miR-5696, miR-4515, miR-409-3p, miR-487b-3p, miR-135b-5p, miR-4764-3p, miR-941, miR-6736-3p, miR-624-3p, miR-9500, miR-3164, miR-6834-3p, miR-6733-3p, miR-6827-3p, miR-7844-5p, miR-491-3p, miR-6768-3p, miR-503-5p, miR-6746-3p, miR-2355-3p, miR-6751-3p, miR-4768-5p, miR-4698, miR-149-5p, miR-519d-3p, miR-4726-5p, miR-11181-5p, miR-8073, miR-4747-3p, miR-3911, miR-3605-3p, miR-4423-3p, miR-4718, miR-4329, miR-6890-5p, miR-4670-5p, miR-30c-1-3p, miR-423-5p, miR-4727-5p, miR-5003-5p, miR-4649-5p, miR-3160-3p, miR-3133, miR-8054, miR-7848-3p, miR-4518, miR-3179, miR-6503-5p, miR-378f, miR-4804-5p, miR-3615, miR-6771-3p, miR-7157-5p, miR-3116, miR-6757-3p, miR-378e, miR-642a-5p, miR-302c-5p, miR-589-5p, miR-6840-5p, miR-6737-3p, miR-4689, miR-4788, miR-30d-5p, miR-212-3p, miR-875-3p, miR-383-3p, miR-369-5p, miR-7107-3p, miR-6758-3p, miR-92b-3p, miR-539-5p, miR-372-3p, miR-6872-3p, miR-4717-3p, miR-6793-3p, miR-646, miR-6822-5p, miR-770-5p, miR-4522, miR-1182, miR-6799-3p, miR-371a-5p, miR-6084, miR-6731-5p, miR-6507-3p, miR-4703-3p, let-7g-3p, miR-6070, miR-4731-5p, miR-5681a, miR-2681-5p, miR-1287-3p, miR-1908-3p, miR-6818-5p, miR-767-3p, miR-3940-3p, and miR-6509-3p. In the present disclosure, the extract of urine may be an extract of the urine of an endometrial cancer patient. Only one of these microRNAs can be used to predict whether a subject from which the urine is derived is an endometrial cancer patient, and 5 or more types, 10 or more types, 15 or more types, or 20 or more types of these microRNAs can be used in combination to predict whether a subject from which the urine is derived is an endometrial cancer patient. Among these microRNAs, a microRNA that may be highly expressed in an endometrial cancer patient can be preferably used for prediction.

There is provided an extract of urine containing at least one type, two types, three types, four types, five types, six types, seven types, eight types, nine types, or ten types of or all microRNAs selected from the group consisting of let-7e-5p, miR-15a-5p, miR-17-3p, miR-29a-3p, miR-30a-5p, miR-31-5p, miR-29b-3p, miR-196a-5p, miR-197-3p, miR-198, miR-199a-5p, miR-199a-3p, miR-199b-3p, miR-30d-5p, miR-139-5p, miR-187-3p, miR-199b-5p, miR-214-3p, miR-223-3p, miR-122-5p, miR-124-3p, miR-138-5p, miR-140-5p, miR-141-3p, miR-125a-5p, miR-134-5p, miR-149-5p, miR-154-3p, miR-184, miR-185-5p, miR-188-5p, miR-206, miR-106b-5p, miR-29c-3p, miR-34c-5p, miR-299-3p, miR-296-5p, miR-130b-3p, miR-361-5p, miR-365a-3p, miR-365b-3p, miR-302c-5p, miR-370-3p, miR-371a-3p, miR-373-5p, miR-373-3p, miR-374a-5p, miR-375-3p, miR-380-3p, miR-382-5p, miR-383-5p, miR-328-3p, miR-342-3p, miR-326, miR-151a-3p, miR-133b, miR-325, miR-346, miR-20b-5p, miR-448, miR-429, miR-449a, miR-450a-5p, miR-200a-5p, miR-433-3p, miR-323b-5p, miR-452-3p, miR-409-3p, miR-483-3p, miR-484, miR-485-3p, miR-486-5p, miR-487a-3p, miR-491-5p, miR-202-3p, miR-492, miR-498-5p, miR-520e-3p, miR-519c-5p, miR-523-5p, miR-518e-5p, miR-522-5p, miR-519a-5p, miR-519b-5p, miR-519b-3p, miR-525-3p, miR-518f-3p, miR-520b-3p, miR-518b, miR-520c-3p, miR-518c-5p, miR-520d-5p, miR-520d-3p, miR-516b-5p, miR-516b-3p, miR-516a-3p, miR-518a-3p, miR-517c-3p, miR-500a-3p, miR-502-5p, miR-503-5p, miR-513a-5p, miR-510-5p, miR-299-5p, miR-539-5p, miR-487b-3p, miR-551a, miR-552-3p, miR-554, miR-555, miR-557, miR-558, miR-563, miR-564, miR-571, miR-573, miR-574-3p, miR-575, miR-578, miR-579-3p, miR-583, miR-585-3p, miR-595, miR-608, miR-610, miR-612, miR-615-3p, miR-616-5p, miR-617, miR-619-3p, miR-626, miR-628-3p, miR-629-3p, miR-632, miR-634, miR-636, miR-637, miR-638, miR-639, miR-642a-5p, miR-644a, miR-649, miR-663a, miR-449b-5p, miR-654-5p, miR-657, miR-658, miR-542-5p, miR-363-5p, miR-425-5p, miR-671-5p, miR-668-3p, miR-767-3p, miR-769-5p, miR-769-3p, miR-766-3p, miR-765, miR-675-5p, miR-297, let-7b-3p, let-7d-3p, let-7f-1-3p, miR-22-5p, miR-24-2-5p, miR-32-3p, miR-92a-2-5p, miR-29b-2-5p, miR-129-l-3p, miR-30c-2-3p, miR-139-3p, miR-181c-3p, miR-183-3p, miR-187-5p, miR-200b-5p, miR-15b-3p, miR-23b-5p, miR-30b-3p, miR-124-5p, miR-125b-1-3p, miR-130a-5p, miR-135a-3p, miR-140-3p, miR-125a-3p, miR-125b-2-3p, miR-129-2-3p, miR-138-1-3p, miR-149-3p, miR-150-3p, miR-185-3p, miR-186-3p, miR-188-3p, miR-193a-5p, miR-194-3p, miR-30c-1-3p, miR-219a-2-3p, miR-296-3p, miR-130b-5p, miR-361-3p, miR-371a-5p, miR-377-5p, miR-342-5p, miR-323a-5p, miR-151a-5p, miR-148b-5p, miR-338-5p, miR-423-5p, miR-424-3p, miR-18b-3p, miR-20b-3p, miR-431-3p, miR-483-5p, miR-486-3p, miR-490-5p, miR-146b-3p, miR-193b-5p, miR-500a-5p, miR-509-5p, miR-532-3p, miR-92b-5p, miR-551b-5p, miR-574-5p, miR-550a-5p, miR-615-5p, miR-616-3p, miR-624-3p, miR-625-3p, miR-629-5p, miR-298, miR-874-3p, miR-891b, miR-541-3p, miR-708-5p, miR-147b-3p, miR-744-5p, miR-885-5p, miR-885-3p, miR-877-5p, miR-877-3p, miR-887-3p, miR-665, miR-760, miR-920, miR-924, miR-936, miR-938, miR-939-5p, miR-940, miR-1224-5p, miR-1224-3p, miR-1225-5p, miR-1225-3p, miR-1226-3p, miR-1228-5p, miR-1228-3p, miR-1229-3p, miR-1231, miR-1233-3p, miR-1234-3p, miR-1236-3p, miR-1237-3p, miR-1238-3p, miR-1264, miR-320b, miR-320c, miR-1296-5p, miR-1301-3p, miR-1298-5p, miR-1181, miR-1182, miR-1184, miR-1202, miR-1203, miR-663b, miR-1207-5p, miR-1208, miR-1285-3p, miR-1286, miR-1289, miR-1293, miR-1294, miR-1303, miR-1304-5p, miR-1247-5p, miR-1249-3p, miR-1257, miR-1260a, miR-1263, miR-1265, miR-1267, miR-1268a, miR-1269a, miR-1270, miR-1275, miR-1276, miR-1281, miR-1292-5p, miR-1255b-5p, miR-664a-3p, miR-1307-3p, miR-320d, miR-1825, miR-675-3p, miR-1469, miR-1470, miR-1471, miR-1538, miR-1539, miR-1908-5p, miR-1909-5p, miR-1909-3p, miR-1910-5p, miR-1911-5p, miR-1912-3p, miR-1913, miR-1914-3p, miR-1915-3p, miR-365a-5p, miR-449b-3p, miR-2113, miR-1976, miR-2054, miR-2110, miR-151b, miR-449c-5p, miR-762, miR-670-5p, miR-761, miR-764, miR-2114-5p, miR-2116-3p, miR-548q, miR-2276-3p, miR-2278, miR-711, miR-718, miR-2681-3p, miR-2682-5p, miR-2682-3p, miR-449c-3p, miR-2861, miR-3116, miR-3119, miR-3120-3p, miR-3122, miR-3124-5p, miR-3126-5p, miR-3130-3p, miR-3130-5p, miR-3131, miR-3132, miR-3133, miR-466, miR-3138, miR-3141, miR-3144-5p, miR-1273c, miR-3147, miR-3148, miR-3150a-3p, miR-3151-5p, miR-3153, miR-3154, miR-3156-5p, miR-3157-5p, miR-3158-3p, miR-3160-3p, miR-3161, miR-3162-5p, miR-3163, miR-3164, miR-3165, miR-1260b, miR-3169, miR-3173-3p, miR-1193, miR-3175, miR-3176, miR-3177-3p, miR-3178, miR-3179, miR-3180-5p, miR-3180-3p, miR-3184-5p, miR-3185, miR-3187-3p, miR-3188, miR-3189-3p, miR-3190-5p, miR-3191-3p, miR-3192-5p, miR-3193, miR-3194-5p, miR-3195, miR-3196, miR-3197, miR-3198, miR-514b-5p, miR-3202, miR-4296, miR-4297, miR-4293, miR-4294, miR-4299, miR-4298, miR-4300, miR-4303, miR-4305, miR-4306, miR-4307, miR-4312, miR-4313, miR-4315, miR-4316, miR-4314, miR-4318, miR-4320, miR-4322, miR-4321, miR-4323, miR-4324, miR-4256, miR-4257, miR-4258, miR-4259, miR-4260, miR-4253, miR-4251, miR-4255, miR-4325, miR-4326, miR-4327, miR-4265, miR-2355-5p, miR-4268, miR-4269, miR-4264, miR-4271, miR-4276, miR-4274, miR-4281, miR-4279, miR-4278, miR-4280, miR-4285, miR-4284, miR-4286, miR-4287, miR-4292, miR-4289, miR-4290, miR-4329, miR-500b-5p, miR-3200-5p, miR-3605-3p, miR-3610, miR-3612, miR-3614-5p, miR-3614-3p, miR-3616-3p, miR-3619-5p, miR-3620-3p, miR-3621, miR-3622a-5p, miR-3622a-3p, miR-3622b-5p, miR-3646, miR-3648, miR-3649, miR-3652, miR-3654, miR-3660, miR-3663-5p, miR-3663-3p, miR-3665, miR-3667-3p, miR-3675-5p, miR-3675-3p, miR-3677-3p, miR-3679-5p, miR-3679-3p, miR-3682-3p, miR-3688-3p, miR-3689a-3p, miR-3691-5p, miR-3714, miR-3180, miR-3907, miR-3689b-3p, miR-3689c, miR-3909, miR-3911, miR-3913-5p, miR-3916, miR-3917, miR-3918, miR-3919, miR-3150b-3p, miR-3924, miR-3925-5p, miR-3926, miR-676-3p, miR-3928-3p, miR-3934-5p, miR-3935, miR-3937, miR-3940-3p, miR-3943, miR-3944-3p, miR-3945, miR-642b-3p, miR-550b-3p, miR-1268b, miR-378e, miR-548ab, miR-378f, miR-4421, miR-4428, miR-4429, miR-4430, miR-4431, miR-4433a-3p, miR-4434, miR-4435, miR-4436a, miR-4439, miR-4440, miR-4441, miR-4443, miR-4444, miR-4446-3p, miR-4447, miR-4448, miR-4449, miR-4450, miR-4451, miR-4454, miR-4455, miR-4456, miR-4458, miR-4460, miR-378h, miR-3135b, miR-4462, miR-4463, miR-4466, miR-4467, miR-4470, miR-4472, miR-4475, miR-4476, miR-4478, miR-3689f, miR-4481, miR-4483, miR-4484, miR-4485-3p, miR-4486, miR-4487, miR-4488, miR-4489, miR-4492, miR-4494, miR-4496, miR-4497, miR-4498, miR-4499, miR-4501, miR-4505, miR-4506, miR-2392, miR-4507, miR-4508, miR-4510, miR-4511, miR-4512, miR-4513, miR-4516, miR-4518, miR-4519, miR-4520-3p, miR-4521, miR-1269b, miR-4522, miR-4523, miR-4524a-3p, miR-4525, miR-4526, miR-4530, miR-4531, miR-4533, miR-4534, miR-378i, miR-4535, miR-1587, miR-4538, miR-4539, miR-4540, miR-3127-3p, miR-3074-5p, miR-3156-3p, miR-3158-5p, miR-3162-3p, miR-3173-5p, miR-3177-5p, miR-3187-5p, miR-3189-5p, miR-3619-3p, miR-3682-5p, miR-3913-3p, miR-3150b-5p, miR-3922-5p, miR-3925-3p, miR-3940-5p, miR-4423-5p, miR-3960, miR-3972, miR-4632-3p, miR-4634, miR-4638-5p, miR-4639-3p, miR-4640-5p, miR-4640-3p, miR-4642, miR-4643, miR-4644, miR-4646-5p, miR-4646-3p, miR-4647, miR-4648, miR-4649-5p, miR-4649-3p, miR-4650-3p, miR-4651, miR-4652-5p, miR-4652-3p, miR-4653-3p, miR-4654, miR-4655-5p, miR-4656, miR-4657, miR-4661-5p, miR-4661-3p, miR-4659b-3p, miR-4664-5p, miR-4664-3p, miR-4665-5p, miR-4665-3p, miR-4667-5p, miR-4667-3p, miR-4669, miR-4670-5p, miR-4670-3p, miR-4671-3p, miR-4672, miR-4673, miR-4674, miR-4676-5p, miR-4677-5p, miR-4681, miR-4682, miR-4684-3p, miR-4685-5p, miR-4685-3p, miR-4687-5p, miR-4687-3p, miR-1343-3p, miR-4688, miR-4689, miR-4690-5p, miR-4690-3p, miR-4691-3p, miR-4692, miR-4694-5p, miR-4695-5p, miR-4695-3p, miR-4697-5p, miR-4697-3p, miR-4698, miR-4700-5p, miR-4700-3p, miR-4701-5p, miR-4701-3p, miR-4703-3p, miR-4704-5p, miR-4706, miR-4707-5p, miR-4707-3p, miR-4708-5p, miR-4708-3p, miR-4709-3p, miR-203b-5p, miR-4710, miR-4712-3p, miR-4713-5p, miR-4714-5p, miR-4715-5p, miR-4716-5p, miR-4716-3p, miR-4717-3p, miR-4720-5p, miR-4721, miR-4722-5p, miR-4722-3p, miR-4723-5p, miR-4723-3p, miR-4724-5p, miR-4725-5p, miR-4725-3p, miR-4726-5p, miR-4726-3p, miR-4727-3p, miR-4728-5p, miR-4728-3p, miR-4730, miR-4731-5p, miR-4731-3p, miR-4732-5p, miR-4732-3p, miR-4734, miR-4736, miR-3064-5p, miR-3064-3p, miR-4738-3p, miR-4739, miR-4741, miR-4742-3p, miR-4743-5p, miR-122b-5p, miR-4745-5p, miR-4745-3p, miR-4746-3p, miR-4747-5p, miR-4747-3p, miR-4748, miR-4749-5p, miR-4749-3p, miR-4750-5p, miR-4751, miR-4753-5p, miR-371b-5p, miR-371b-3p, miR-4754, miR-4755-3p, miR-499b-3p, miR-4756-5p, miR-4758-5p, miR-4758-3p, miR-4759, miR-4762-3p, miR-4763-5p, miR-4763-3p, miR-4766-5p, miR-4769-5p, miR-4769-3p, miR-4776-5p, miR-4776-3p, miR-4778-5p, miR-4780, miR-4436b-5p, miR-4436b-3p, miR-4781-5p, miR-4783-3p, miR-4784, miR-4785, miR-2467-5p, miR-2467-3p, miR-4786-5p, miR-4786-3p, miR-4787-5p, miR-4787-3p, miR-4788, miR-4793-3p, miR-4794, miR-4797-3p, miR-4799-5p, miR-4800-5p, miR-4801, miR-4804-5p, miR-4804-3p, miR-4520-2-3p, miR-642a-3p, miR-550a-3-5p, miR-4433a-5p, miR-4482-3p, miR-4999-5p, miR-5000-3p, miR-5001-5p, miR-5002-3p, miR-5003-5p, miR-5003-3p, miR-5004-3p, miR-5006-5p, miR-5007-5p, miR-5008-5p, miR-5009-3p, miR-5010-5p, miR-5010-3p, miR-5087, miR-5088-5p, miR-5089-5p, miR-5090, miR-5092, miR-5093, miR-5187-5p, miR-5188, miR-5189-5p, miR-5190, miR-5193, miR-5194, miR-5195-5p, miR-5195-3p, miR-5196-5p, miR-5196-3p, miR-4524b-5p, miR-5100, miR-5572, miR-664b-5p, miR-664b-3p, miR-5581-5p, miR-5581-3p, miR-5582-3p, miR-5584-5p, miR-548au-3p, miR-5588-5p, miR-5589-5p, miR-5589-3p, miR-5684, miR-5689, miR-5690, miR-4666b, miR-5693, miR-5695, miR-5696, miR-5698, miR-5699-3p, miR-5702, miR-5703, miR-5704, miR-5705, miR-5708, miR-197-5p, miR-181b-3p, miR-204-3p, miR-211-3p, miR-301a-5p, miR-382-3p, miR-345-3p, miR-652-5p, miR-1185-2-3p, miR-766-5p, miR-873-3p, miR-1304-3p, miR-1247-3p, miR-1306-5p, miR-3184-3p, miR-3191-5p, miR-642b-5p, miR-365b-5p, miR-1185-1-3p, miR-98-3p, miR-381-5p, miR-495-5p, miR-503-3p, miR-937-5p, miR-939-3p, miR-1227-5p, miR-1229-5p, miR-1233-5p, miR-1236-5p, miR-1237-5p, miR-1238-5p, miR-1292-3p, miR-3617-3p, miR-3620-5p, miR-3934-3p, miR-4632-5p, miR-4743-3p, miR-4750-3p, miR-5739, miR-5787, miR-6068, miR-6069, miR-6071, miR-6072, miR-6074, miR-6075, miR-6076, miR-6077, miR-6078, miR-6081, miR-6082, miR-6083, miR-6085, miR-6086, miR-6088, miR-6089, miR-6090, miR-6124, miR-6125, miR-6126, miR-6127, miR-378j, miR-6129, miR-6130, miR-6131, miR-6132, miR-6133, miR-6165, miR-548ay-3p, miR-6500-5p, miR-6501-5p, miR-6501-3p, miR-6503-5p, miR-6506-5p, miR-6506-3p, miR-6507-3p, miR-6508-5p, miR-6509-5p, miR-6509-3p, miR-6510-5p, miR-6511a-5p, miR-6511a-3p, miR-6512-3p, miR-6513-5p, miR-6514-5p, miR-6514-3p, miR-6515-5p, miR-6515-3p, miR-6715b-3p, miR-6716-5p, miR-6716-3p, miR-6717-5p, miR-6511b-5p, miR-6511b-3p, miR-6719-3p, miR-6721-5p, miR-6722-5p, miR-6722-3p, miR-6724-5p, miR-208a-5p, miR-210-5p, miR-128-1-5p, miR-370-5p, miR-328-5p, miR-329-5p, miR-410-5p, miR-487a-5p, miR-489-5p, miR-181d-3p, miR-504-3p, miR-487b-5p, miR-579-5p, miR-585-5p, miR-597-3p, miR-598-5p, miR-605-3p, miR-619-5p, miR-1296-3p, miR-891a-3p, miR-874-5p, miR-1180-5p, miR-1250-3p, miR-1266-3p, miR-1908-3p, miR-3151-3p, miR-3192-3p, miR-500b-3p, miR-1343-5p, miR-5088-3p, miR-5189-3p, miR-5699-5p, miR-6726-5p, miR-6726-3p, miR-6727-5p, miR-6727-3p, miR-6728-5p, miR-6728-3p, miR-6729-5p, miR-6729-3p, miR-6730-5p, miR-6730-3p, miR-6731-5p, miR-6731-3p, miR-6732-5p, miR-6732-3p, miR-6734-5p, miR-6734-3p, miR-6735-5p, miR-6735-3p, miR-6736-5p, miR-6736-3p, miR-6737-5p, miR-6737-3p, miR-6738-5p, miR-6738-3p, miR-6740-5p, miR-6740-3p, miR-6741-5p, miR-6741-3p, miR-6742-5p, miR-6742-3p, miR-6743-5p, miR-6743-3p, miR-6744-5p, miR-6744-3p, miR-6745, miR-6746-5p, miR-6747-5p, miR-6747-3p, miR-6748-5p, miR-6748-3p, miR-6749-5p, miR-6749-3p, miR-6750-5p, miR-6750-3p, miR-6751-5p, miR-6751-3p, miR-6752-5p, miR-6752-3p, miR-6753-5p, miR-6753-3p, miR-6754-5p, miR-6754-3p, miR-6755-5p, miR-6755-3p, miR-6756-5p, miR-6756-3p, miR-6757-5p, miR-6757-3p, miR-6758-5p, miR-6759-5p, miR-6759-3p, miR-6760-5p, miR-6760-3p, miR-6761-5p, miR-6761-3p, miR-6762-5p, miR-6762-3p, miR-6763-5p, miR-6763-3p, miR-6765-5p, miR-6766-5p, miR-6766-3p, miR-6767-5p, miR-6768-5p, miR-6769a-5p, miR-6769a-3p, miR-6770-5p, miR-6770-3p, miR-6771-5p, miR-6771-3p, miR-6772-5p, miR-6772-3p, miR-6773-3p, miR-6774-5p, miR-6775-5p, miR-6775-3p, miR-6776-5p, miR-6776-3p, miR-6777-5p, miR-6777-3p, miR-6778-5p, miR-6778-3p, miR-6779-5p, miR-6779-3p, miR-6780a-5p, miR-6780a-3p, miR-6781-5p, miR-6782-5p, miR-6782-3p, miR-6783-3p, miR-6783-3p, miR-6784-5p, miR-6784-3p, miR-6785-3p, miR-6786-5p, miR-6786-3p, miR-6787-5p, miR-6787-3p, miR-6788-5p, miR-6788-3p, miR-6789-5p, miR-6789-3p, miR-6790-5p, miR-6790-3p, miR-6791-5p, miR-6792-5p, miR-6792-3p, miR-6793-5p, miR-6793-3p, miR-6794-5p, miR-6794-3p, miR-6795-5p, miR-6795-3p, miR-6796-5p, miR-6796-3p, miR-6797-5p, miR-6797-3p, miR-6798-5p, miR-6798-3p, miR-6799-5p, miR-6799-3p, miR-6800-5p, miR-6800-3p, miR-6801-3p, miR-6802-5p, miR-6802-3p, miR-6803-5p, miR-6803-3p, miR-6804-3p, miR-6805-5p, miR-6805-3p, miR-6806-5p, miR-6806-3p, miR-6807-5p, miR-6808-5p, miR-6808-3p, miR-6809-5p, miR-6809-3p, miR-6810-5p, miR-6810-3p, miR-6812-5p, miR-6812-3p, miR-6813-5p, miR-6813-3p, miR-6814-3p, miR-6815-5p, miR-6815-3p, miR-6816-5p, miR-6816-3p, miR-6818-5p, miR-6819-5p, miR-6819-3p, miR-6820-5p, miR-6820-3p, miR-6821-5p, miR-6822-5p, miR-6823-5p, miR-6823-3p, miR-6824-5p, miR-6824-3p, miR-6825-5p, miR-6825-3p, miR-6826-3p, miR-6827-5p, miR-6827-3p, miR-6828-5p, miR-6829-5p, miR-6829-3p, miR-6830-5p, miR-6830-3p, miR-6831-5p, miR-6831-3p, miR-6832-5p, miR-6833-5p, miR-6833-3p, miR-6834-5p, miR-6834-3p, miR-6780b-5p, miR-6780b-3p, miR-6836-5p, miR-6836-3p, miR-6837-5p, miR-6838-5p, miR-6839-5p, miR-6840-5p, miR-6840-3p, miR-6841-3p, miR-6842-5p, miR-6842-3p, miR-6845-5p, miR-6845-3p, miR-6846-5p, miR-6846-3p, miR-6847-5p, miR-6847-3p, miR-6848-5p, miR-6848-3p, miR-6849-5p, miR-6849-3p, miR-6850-5p, miR-6851-5p, miR-6851-3p, miR-6855-3p, miR-6855-3p, miR-6856-5p, miR-6857-5p, miR-6857-3p, miR-6858-5p, miR-6858-3p, miR-6859-5p, miR-6859-3p, miR-6769b-5p, miR-6769b-3p, miR-6860, miR-6861-5p, miR-6861-3p, miR-6862-5p, miR-6862-3p, miR-6867-5p, miR-6867-3p, miR-6869-5p, miR-6870-5p, miR-6870-3p, miR-6871-5p, miR-6872-5p, miR-6872-3p, miR-6874-5p, miR-6875-5p, miR-6875-3p, miR-6876-5p, miR-6877-5p, miR-6877-3p, miR-6878-3p, miR-6879-5p, miR-6879-3p, miR-6880-5p, miR-6880-3p, miR-6881-5p, miR-6881-3p, miR-6882-3p, miR-6883-5p, miR-6883-3p, miR-6884-3p, miR-6885-3p, miR-6886-5p, miR-6886-3p, miR-6887-5p, miR-6887-3p, miR-6888-5p, miR-6889-5p, miR-6889-3p, miR-6890-5p, miR-6890-3p, miR-6891-5p, miR-6891-3p, miR-6892-5p, miR-6892-3p, miR-6893-5p, miR-6893-3p, miR-6894-5p, miR-6894-3p, miR-6895-3p, miR-7106-5p, miR-7106-3p, miR-7107-5p, miR-7107-3p, miR-7108-5p, miR-7108-3p, miR-7109-5p, miR-7109-3p, miR-7110-5p, miR-7110-3p, miR-7111-5p, miR-7111-3p, miR-7112-5p, miR-7113-5p, miR-7113-3p, miR-7114-5p, miR-7114-3p, miR-7150, miR-7151-3p, miR-7152-5p, miR-7154-3p, miR-7155-5p, miR-7157-3p, miR-7159-5p, miR-7160-5p, miR-7161-5p, miR-7162-5p, miR-7515, miR-7702, miR-7704, miR-7706, miR-7843-5p, miR-4433b-5p, miR-4433b-3p, miR-1273h-5p, miR-6516-5p, miR-7845-5p, miR-7846-3p, miR-7847-3p, miR-7851-3p, miR-7855-5p, miR-7856-5p, miR-8052, miR-8054, miR-8057, miR-8059, miR-8060, miR-8062, miR-8063, miR-8064, miR-8068, miR-8069, miR-8071, miR-8072, miR-8073, miR-8074, miR-8075, miR-8077, miR-8078, miR-8079, miR-8080, miR-8085, miR-8086, miR-8087, miR-8089, miR-128-2-5p, miR-1199-5p, miR-7977, miR-7978, miR-1249-5p, miR-4485-5p, miR-8485, miR-217-3p, miR-320a-5p, miR-375-5p, miR-498-3p, miR-526a-3p, miR-9718, miR-9898, miR-9899, miR-1843, miR-10226, miR-10392-5p, miR-10392-3p, miR-10394-3p, miR-10396a-5p, miR-10396a-3p, miR-10398-5p, miR-10398-3p, miR-10400-5p, miR-10400-3p, miR-10401-5p, miR-10401-3p, miR-10396b-5p, miR-10522-5p, miR-10524-5p, miR-10526-3p, miR-10527-5p, miR-11181-5p, miR-11181-3p, miR-11399, miR-11400, miR-3059-5p, miR-3059-3p, miR-3085-5p, miR-3085-3p, miR-6529-5p, miR-12114, miR-12115, miR-12116, miR-12118, miR-12119, miR-12120, miR-12121, miR-12122, miR-12124, miR-12126, miR-12127, miR-12128, miR-12131, and miR-12136. In the present disclosure, the extract of urine may be an extract of the urine of a stage-I or -II endometrial cancer patient. Only one of these microRNAs can be used to predict whether a subject from which the urine is derived is an endometrial cancer patient (particularly stage-I or -II), and 5 or more types, 10 or more types, 15 or more types, or 20 or more types of these microRNAs can be used in combination to predict whether a subject from which the urine is derived is an endometrial cancer patient (particularly stage-I or -II). Among these microRNAs, a microRNA that may be highly expressed in an endometrial cancer patient (particularly stage-I or -II) can be preferably used for prediction.

The present disclosure provides an extract of urine containing at least one type, two types, three types, four types, five types, six types, seven types, eight types, nine types, or ten types of or all microRNAs selected from the group consisting of let-7e-5p, miR-15a-5p, miR-17-3p, miR-29a-3p, miR-30a-5p, miR-31-5p, miR-29b-3p, miR-107, miR-196a-5p, miR-197-3p, miR-198, miR-199a-5p, miR-199a-3p, miR-199b-3p, miR-30d-5p, miR-139-5p, miR-187-3p, miR-199b-5p, miR-211-5p, miR-214-3p, miR-218-5p, miR-223-3p, miR-122-5p, miR-138-5p, miR-140-5p, miR-141-3p, miR-125a-5p, miR-134-5p, miR-149-5p, miR-184, miR-185-5p, miR-188-5p, miR-206, miR-106b-5p, miR-29c-3p, miR-34c-5p, miR-299-3p, miR-296-5p, miR-130b-3p, miR-361-5p, miR-365a-3p, miR-365b-3p, miR-302c-5p, miR-370-3p, miR-371a-3p, miR-373-3p, miR-373-3p, miR-374a-5p, miR-375-3p, miR-380-3p, miR-382-5p, miR-383-5p, miR-328-3p, miR-342-3p, miR-326, miR-151a-3p, miR-133b, miR-325, miR-346, miR-20b-5p, miR-448, miR-429, miR-449a, miR-450a-5p, miR-200a-5p, miR-433-3p, miR-323b-5p, miR-452-3p, miR-409-3p, miR-483-3p, miR-484, miR-485-3p, miR-486-5p, miR-487a-3p, miR-491-5p, miR-202-3p, miR-492, miR-193b-3p, miR-498-5p, miR-520e-3p, miR-519c-5p, miR-523-5p, miR-518e-5p, miR-522-5p, miR-519a-5p, miR-519b-5p, miR-519b-3p, miR-525-3p, miR-520b-3p, miR-518b, miR-520c-3p, miR-518c-5p, miR-520d-5p, miR-520d-3p, miR-516b-5p, miR-516b-3p, miR-516a-3p, miR-518a-3p, miR-517c-3p, miR-519a-3p, miR-500a-3p, miR-502-5p, miR-503-5p, miR-513a-5p, miR-539-5p, miR-487b-3p, miR-551a, miR-552-3p, miR-554, miR-555, miR-557, miR-563, miR-564, miR-571, miR-572, miR-573, miR-574-3p, miR-575, miR-578, miR-579-3p, miR-583, miR-585-3p, miR-550a-3p, miR-595, miR-608, miR-610, miR-612, miR-615-3p, miR-616-5p, miR-617, miR-619-3p, miR-626, miR-628-3p, miR-632, miR-634, miR-636, miR-637, miR-638, miR-639, miR-642a-5p, miR-649, miR-663a, miR-449b-5p, miR-657, miR-658, miR-542-5p, miR-363-5p, miR-425-5p, miR-671-5p, miR-668-3p, miR-767-3p, miR-769-5p, miR-769-3p, miR-766-3p, miR-765, miR-675-5p, miR-297, let-7b-3p, let-7d-3p, let-7f-1-3p, miR-22-5p, miR-24-2-5p, miR-26b-3p, miR-32-3p, miR-92a-2-5p, miR-29b-2-5p, miR-129-l-3p, miR-30c-2-3p, miR-139-3p, miR-181c-3p, miR-183-3p, miR-187-5p, miR-200b-5p, miR-15b-3p, miR-23b-5p, miR-30b-3p, miR-124-5p, miR-125b-1-3p, miR-130a-5p, miR-135a-3p, miR-140-3p, miR-125a-3p, miR-125b-2-3p, miR-129-2-3p, miR-138-1-3p, miR-149-3p, miR-150-3p, miR-185-3p, miR-186-3p, miR-188-3p, miR-193a-5p, miR-194-3p, miR-30c-1-3p, miR-219a-2-3p, miR-296-3p, miR-130b-5p, miR-361-3p, miR-371a-5p, miR-377-5p, miR-342-5p, miR-337-5p, miR-323a-5p, miR-151a-5p, miR-148b-5p, miR-338-5p, miR-423-5p, miR-424-3p, miR-18b-3p, miR-20b-3p, miR-431-3p, miR-483-5p, miR-486-3p, miR-490-5p, miR-146b-3p, miR-193b-5p, miR-509-5p, miR-532-3p, miR-92b-5p, miR-551b-5p, miR-574-5p, miR-550a-5p, miR-615-5p, miR-616-3p, miR-624-3p, miR-625-3p, miR-629-5p, miR-298, miR-874-3p, miR-891b, miR-892b, miR-541-3p, miR-708-5p, miR-147b-3p, miR-744-5p, miR-885-5p, miR-885-3p, miR-877-5p, miR-877-3p, miR-887-3p, miR-665, miR-760, miR-920, miR-921, miR-924, miR-935, miR-936, miR-938, miR-939-5p, miR-940, miR-1224-5p, miR-1224-3p, miR-1225-5p, miR-1225-3p, miR-1226-3p, miR-1228-5p, miR-1228-3p, miR-1229-3p, miR-1231, miR-1233-3p, miR-1234-3p, miR-1236-3p, miR-1237-3p, miR-1238-3p, miR-1264, miR-320b, miR-320c, miR-1296-5p, miR-1301-3p, miR-1298-5p, miR-1181, miR-1182, miR-1184, miR-1202, miR-1203, miR-663b, miR-1207-5p, miR-1208, miR-1285-3p, miR-1286, miR-1289, miR-1293, miR-1291, miR-1303, miR-1304-5p, miR-1247-5p, miR-1249-3p, miR-1251-5p, miR-1257, miR-1260a, miR-1263, miR-1265, miR-1266-5p, miR-1267, miR-1268a, miR-1269a, miR-1270, miR-1275, miR-1276, miR-1281, miR-1292-5p, miR-1255b-5p, miR-664a-3p, miR-1307-3p, miR-320d, miR-1825, miR-675-3p, miR-1469, miR-1470, miR-1471, miR-1538, miR-1539, miR-1908-5p, miR-1909-5p, miR-1909-3p, miR-1910-5p, miR-1911-5p, miR-1912-3p, miR-1913, miR-1914-3p, miR-1915-3p, miR-365a-5p, miR-449b-3p, miR-2113, miR-1976, miR-2054, miR-2110, miR-151b, miR-449c-5p, miR-762, miR-670-5p, miR-761, miR-764, miR-2116-3p, miR-548q, miR-2276-3p, miR-2278, miR-711, miR-718, miR-2681-3p, miR-2682-5p, miR-2682-3p, miR-449c-3p, miR-2861, miR-3116, miR-3119, miR-3120-3p, miR-3122, miR-3124-5p, miR-3126-5p, miR-3130-3p, miR-3130-5p, miR-3131, miR-3132, miR-3133, miR-466, miR-3137, miR-3138, miR-3141, miR-3144-5p, miR-1273c, miR-3147, miR-3148, miR-3150a-3p, miR-3151-5p, miR-3153, miR-3154, miR-3156-5p, miR-3157-5p, miR-3160-3p, miR-3161, miR-3162-5p, miR-3163, miR-3164, miR-3165, miR-1260b, miR-3169, miR-3173-3p, miR-1193, miR-3176, miR-3177-3p, miR-3178, miR-3179, miR-3180-5p, miR-3180-3p, miR-3184-5p, miR-3185, miR-3187-3p, miR-3188, miR-3189-3p, miR-3190-5p, miR-3191-3p, miR-3192-5p, miR-3193, miR-3194-5p, miR-3195, miR-3196, miR-3197, miR-3198, miR-514b-5p, miR-3202, miR-4296, miR-4297, miR-4293, miR-4294, miR-4299, miR-4298, miR-4300, miR-4305, miR-4306, miR-4307, miR-4312, miR-4313, miR-4315, miR-4316, miR-4314, miR-4318, miR-4320, miR-4322, miR-4321, miR-4323, miR-4324, miR-4256, miR-4257, miR-4258, miR-4259, miR-4260, miR-4253, miR-4251, miR-4255, miR-4325, miR-4326, miR-4327, miR-4265, miR-2355-5p, miR-4268, miR-4269, miR-4264, miR-4271, miR-4276, miR-4274, miR-4281, miR-4279, miR-4280, miR-4285, miR-4284, miR-4286, miR-4287, miR-4292, miR-4289, miR-4290, miR-4329, miR-500b-5p, miR-3200-5p, miR-3605-3p, miR-3610, miR-3612, miR-3614-5p, miR-3614-3p, miR-3616-3p, miR-3619-5p, miR-3620-3p, miR-3621, miR-3622a-5p, miR-3622a-3p, miR-3622b-5p, miR-3646, miR-3648, miR-3649, miR-3652, miR-3654, miR-3660, miR-3663-5p, miR-3663-3p, miR-3665, miR-3667-3p, miR-3675-5p, miR-3675-3p, miR-3677-3p, miR-3679-5p, miR-3679-3p, miR-3682-3p, miR-3688-3p, miR-3689a-3p, miR-3691-5p, miR-3714, miR-3180, miR-3907, miR-3689b-3p, miR-3689c, miR-3909, miR-3911, miR-3913-5p, miR-3916, miR-3917, miR-3918, miR-3919, miR-3150b-3p, miR-3924, miR-3925-5p, miR-3926, miR-676-3p, miR-3928-3p, miR-3934-5p, miR-3935, miR-3937, miR-3940-3p, miR-3943, miR-3944-3p, miR-3945, miR-642b-3p, miR-550b-3p, miR-1268b, miR-378e, miR-548ab, miR-378f, miR-4421, miR-4428, miR-4429, miR-4430, miR-4431, miR-4433a-3p, miR-4434, miR-4435, miR-4436a, miR-4437, miR-4439, miR-4440, miR-4441, miR-4443, miR-4444, miR-4446-3p, miR-4447, miR-4448, miR-4449, miR-4450, miR-4451, miR-4454, miR-4455, miR-4456, miR-4458, miR-4460, miR-378h, miR-3135b, miR-4462, miR-4463, miR-4466, miR-4467, miR-4470, miR-4472, miR-4474-3p, miR-4475, miR-4476, miR-4478, miR-3689f, miR-4479, miR-4481, miR-4483, miR-4484, miR-4486, miR-4487, miR-4488, miR-4489, miR-4492, miR-4494, miR-4496, miR-4497, miR-4498, miR-4499, miR-4501, miR-4505, miR-4506, miR-2392, miR-4507, miR-4508, miR-4510, miR-4511, miR-4512, miR-4513, miR-4516, miR-4518, miR-4519, miR-4520-3p, miR-4521, miR-1269b, miR-4522, miR-4523, miR-4524a-3p, miR-4525, miR-4526, miR-4530, miR-4531, miR-4533, miR-4534, miR-378i, miR-4535, miR-1587, miR-4538, miR-4539, miR-4540, miR-3127-3p, miR-3129-3p, miR-3074-5p, miR-3156-3p, miR-3158-5p, miR-3162-3p, miR-3173-5p, miR-3177-5p, miR-3187-5p, miR-3189-5p, miR-3619-3p, miR-3682-5p, miR-3913-3p, miR-3150b-5p, miR-3922-5p, miR-3925-3p, miR-3940-5p, miR-4423-5p, miR-3960, miR-3972, miR-4632-3p, miR-4634, miR-4638-5p, miR-4639-3p, miR-4640-5p, miR-4640-3p, miR-4642, miR-4643, miR-4644, miR-4646-5p, miR-4646-3p, miR-4647, miR-4648, miR-4649-5p, miR-4649-3p, miR-4650-3p, miR-4651, miR-4652-5p, miR-4652-3p, miR-4653-3p, miR-4654, miR-4655-5p, miR-4656, miR-4657, miR-4659a-5p, miR-4661-5p, miR-4661-3p, miR-4659b-3p, miR-4664-5p, miR-4664-3p, miR-4665-5p, miR-4665-3p, miR-4667-5p, miR-4667-3p, miR-4669, miR-4670-5p, miR-4670-3p, miR-4671-3p, miR-4672, miR-4673, miR-4674, miR-4676-5p, miR-4677-5p, miR-4681, miR-4682, miR-4684-3p, miR-4685-5p, miR-4685-3p, miR-4687-5p, miR-4687-3p, miR-1343-3p, miR-4688, miR-4689, miR-4690-5p, miR-4690-3p, miR-4691-5p, miR-4691-3p, miR-4692, miR-4694-5p, miR-4695-5p, miR-4695-3p, miR-4697-5p, miR-4697-3p, miR-4698, miR-4700-5p, miR-4700-3p, miR-4701-5p, miR-4701-3p, miR-4703-3p, miR-4704-5p, miR-4706, miR-4707-5p, miR-4707-3p, miR-4708-5p, miR-4708-3p, miR-4709-3p, miR-203b-5p, miR-4710, miR-4712-3p, miR-4713-5p, miR-4714-5p, miR-4715-5p, miR-4716-5p, miR-4716-3p, miR-4717-3p, miR-4720-5p, miR-4721, miR-4722-5p, miR-4722-3p, miR-4723-5p, miR-4723-3p, miR-451b, miR-4724-5p, miR-4725-5p, miR-4725-3p, miR-4726-5p, miR-4726-3p, miR-4727-3p, miR-4728-5p, miR-4728-3p, miR-4730, miR-4731-5p, miR-4731-3p, miR-4732-5p, miR-4732-3p, miR-4734, miR-4736, miR-3064-5p, miR-3064-3p, miR-4738-3p, miR-4739, miR-4741, miR-4742-3p, miR-4743-5p, miR-122b-5p, miR-4745-5p, miR-4745-3p, miR-4746-3p, miR-4747-5p, miR-4747-3p, miR-4748, miR-4749-5p, miR-4749-3p, miR-4750-5p, miR-4751, miR-4753-5p, miR-371b-5p, miR-371b-3p, miR-4754, miR-4755-3p, miR-499b-3p, miR-4756-5p, miR-4758-5p, miR-4758-3p, miR-4759, miR-4762-3p, miR-4763-5p, miR-4763-3p, miR-4766-5p, miR-4769-5p, miR-4769-3p, miR-4776-5p, miR-4776-3p, miR-4778-5p, miR-4780, miR-4436b-5p, miR-4436b-3p, miR-4781-5p, miR-4783-3p, miR-4784, miR-4785, miR-2467-5p, miR-2467-3p, miR-4786-5p, miR-4786-3p, miR-4787-5p, miR-4787-3p, miR-4788, miR-4793-3p, miR-4794, miR-4799-5p, miR-4800-5p, miR-4801, miR-4804-5p, miR-4804-3p, miR-4520-2-3p, miR-642a-3p, miR-550a-3-5p, miR-4433a-5p, miR-4482-3p, miR-4999-5p, miR-5000-3p, miR-5001-5p, miR-5002-3p, miR-5003-5p, miR-5003-3p, miR-5004-3p, miR-5006-5p, miR-5007-5p, miR-5008-5p, miR-5009-3p, miR-5010-5p, miR-5010-3p, miR-5087, miR-5088-5p, miR-5089-5p, miR-5090, miR-5092, miR-5093, miR-5187-5p, miR-5188, miR-5189-5p, miR-5190, miR-5192, miR-5193, miR-5194, miR-5195-5p, miR-5195-3p, miR-5196-5p, miR-5196-3p, miR-4524b-5p, miR-5100, miR-5572, miR-664b-5p, miR-664b-3p, miR-5581-5p, miR-5581-3p, miR-5584-5p, miR-548au-3p, miR-5588-5p, miR-5589-5p, miR-5684, miR-5689, miR-5690, miR-4666b, miR-5693, miR-5695, miR-5696, miR-5698, miR-5699-3p, miR-5702, miR-5703, miR-5704, miR-5705, miR-5708, miR-197-5p, miR-181b-3p, miR-204-3p, miR-211-3p, miR-301a-5p, miR-382-3p, miR-345-3p, miR-652-5p, miR-1185-2-3p, miR-766-5p, miR-873-3p, miR-1304-3p, miR-1247-3p, miR-1306-5p, miR-3184-3p, miR-3191-5p, miR-642b-5p, miR-365b-5p, miR-1185-1-3p, miR-3190-3p, miR-98-3p, miR-381-5p, miR-503-3p, miR-937-5p, miR-939-3p, miR-1227-5p, miR-1229-5p, miR-1233-5p, miR-1236-5p, miR-1237-5p, miR-1238-5p, miR-1292-3p, miR-3617-3p, miR-3620-5p, miR-3934-3p, miR-4632-5p, miR-4743-3p, miR-4750-3p, miR-5739, miR-5787, miR-6068, miR-6069, miR-6071, miR-6072, miR-6074, miR-6075, miR-6076, miR-6077, miR-6078, miR-6081, miR-6082, miR-6085, miR-6086, miR-6088, miR-6089, miR-6090, miR-6124, miR-6125, miR-6126, miR-6127, miR-378j, miR-6129, miR-6130, miR-6131, miR-6132, miR-6133, miR-6165, miR-548ay-3p, miR-6500-5p, miR-6501-5p, miR-6501-3p, miR-6503-5p, miR-6506-5p, miR-6506-3p, miR-6507-3p, miR-6508-5p, miR-6509-5p, miR-6509-3p, miR-6510-5p, miR-6511a-5p, miR-6511a-3p, miR-6512-3p, miR-6513-5p, miR-6514-5p, miR-6514-3p, miR-6515-5p, miR-6515-3p, miR-6715b-3p, miR-6716-5p, miR-6716-3p, miR-6717-5p, miR-6511b-5p, miR-6511b-3p, miR-6719-3p, miR-6721-5p, miR-6722-5p, miR-6722-3p, miR-6724-5p, miR-208a-5p, miR-210-5p, miR-128-1-5p, miR-370-5p, miR-328-5p, miR-329-5p, miR-410-5p, miR-487a-5p, miR-489-5p, miR-181d-3p, miR-504-3p, miR-487b-5p, miR-585-5p, miR-597-3p, miR-598-5p, miR-605-3p, miR-619-5p, miR-1296-3p, miR-891a-3p, miR-874-5p, miR-1180-5p, miR-1250-3p, miR-1266-3p, miR-1908-3p, miR-3151-3p, miR-3192-3p, miR-500b-3p, miR-3912-5p, miR-1343-5p, miR-5088-3p, miR-5189-3p, miR-5699-5p, miR-6726-5p, miR-6726-3p, miR-6727-5p, miR-6727-3p, miR-6728-5p, miR-6728-3p, miR-6729-5p, miR-6729-3p, miR-6730-5p, miR-6730-3p, miR-6731-5p, miR-6731-3p, miR-6732-5p, miR-6732-3p, miR-6734-5p, miR-6734-3p, miR-6735-5p, miR-6735-3p, miR-6736-5p, miR-6736-3p, miR-6737-5p, miR-6737-3p, miR-6738-5p, miR-6738-3p, miR-6740-5p, miR-6740-3p, miR-6741-5p, miR-6741-3p, miR-6742-5p, miR-6742-3p, miR-6743-5p, miR-6743-3p, miR-6744-5p, miR-6744-3p, miR-6745, miR-6746-5p, miR-6747-5p, miR-6747-3p, miR-6748-5p, miR-6748-3p, miR-6749-5p, miR-6749-3p, miR-6750-5p, miR-6750-3p, miR-6751-5p, miR-6751-3p, miR-6752-5p, miR-6752-3p, miR-6753-5p, miR-6753-3p, miR-6754-5p, miR-6754-3p, miR-6755-5p, miR-6755-3p, miR-6756-5p, miR-6756-3p, miR-6757-5p, miR-6757-3p, miR-6758-5p, miR-6759-5p, miR-6759-3p, miR-6760-5p, miR-6760-3p, miR-6761-5p, miR-6761-3p, miR-6762-5p, miR-6762-3p, miR-6763-5p, miR-6763-3p, miR-6765-5p, miR-6766-5p, miR-6766-3p, miR-6767-5p, miR-6768-5p, miR-6769a-5p, miR-6769a-3p, miR-6770-5p, miR-6770-3p, miR-6771-5p, miR-6771-3p, miR-6772-5p, miR-6772-3p, miR-6773-3p, miR-6774-5p, miR-6775-5p, miR-6775-3p, miR-6776-5p, miR-6776-3p, miR-6777-5p, miR-6777-3p, miR-6778-5p, miR-6778-3p, miR-6779-5p, miR-6779-3p, miR-6780a-5p, miR-6780a-3p, miR-6781-5p, miR-6782-5p, miR-6782-3p, miR-6783-5p, miR-6783-3p, miR-6784-5p, miR-6784-3p, miR-6785-3p, miR-6786-5p, miR-6786-3p, miR-6787-5p, miR-6787-3p, miR-6788-5p, miR-6788-3p, miR-6789-5p, miR-6789-3p, miR-6790-5p, miR-6790-3p, miR-6791-5p, miR-6792-5p, miR-6792-3p, miR-6793-5p, miR-6793-3p, miR-6794-5p, miR-6794-3p, miR-6795-5p, miR-6795-3p, miR-6796-5p, miR-6796-3p, miR-6797-5p, miR-6797-3p, miR-6798-5p, miR-6798-3p, miR-6799-5p, miR-6799-3p, miR-6800-5p, miR-6800-3p, miR-6801-5p, miR-6801-3p, miR-6802-5p, miR-6802-3p, miR-6803-5p, miR-6803-3p, miR-6804-3p, miR-6805-5p, miR-6805-3p, miR-6806-5p, miR-6806-3p, miR-6807-5p, miR-6808-5p, miR-6808-3p, miR-6809-5p, miR-6809-3p, miR-6810-5p, miR-6810-3p, miR-6812-5p, miR-6812-3p, miR-6813-5p, miR-6813-3p, miR-6814-3p, miR-6815-5p, miR-6815-3p, miR-6816-5p, miR-6816-3p, miR-6818-5p, miR-6819-5p, miR-6819-3p, miR-6820-5p, miR-6820-3p, miR-6821-5p, miR-6822-5p, miR-6823-5p, miR-6823-3p, miR-6824-5p, miR-6824-3p, miR-6825-5p, miR-6825-3p, miR-6826-3p, miR-6827-5p, miR-6827-3p, miR-6828-5p, miR-6829-5p, miR-6829-3p, miR-6830-5p, miR-6830-3p, miR-6831-5p, miR-6831-3p, miR-6832-5p, miR-6833-5p, miR-6833-3p, miR-6834-5p, miR-6834-3p, miR-6780b-5p, miR-6780b-3p, miR-6836-5p, miR-6836-3p, miR-6837-5p, miR-6838-5p, miR-6839-5p, miR-6840-5p, miR-6840-3p, miR-6841-3p, miR-6842-5p, miR-6842-3p, miR-6845-5p, miR-6845-3p, miR-6846-5p, miR-6846-3p, miR-6847-5p, miR-6847-3p, miR-6848-5p, miR-6848-3p, miR-6849-5p, miR-6849-3p, miR-6850-5p, miR-6851-5p, miR-6851-3p, miR-6855-5p, miR-6855-3p, miR-6856-5p, miR-6857-5p, miR-6857-3p, miR-6858-5p, miR-6858-3p, miR-6859-5p, miR-6859-3p, miR-6769b-5p, miR-6769b-3p, miR-6860, miR-6861-5p, miR-6861-3p, miR-6862-5p, miR-6862-3p, miR-6867-5p, miR-6867-3p, miR-6869-5p, miR-6870-5p, miR-6870-3p, miR-6871-5p, miR-6872-5p, miR-6872-3p, miR-6874-5p, miR-6875-5p, miR-6875-3p, miR-6876-5p, miR-6876-3p, miR-6877-5p, miR-6877-3p, miR-6878-3p, miR-6879-5p, miR-6879-3p, miR-6880-5p, miR-6880-3p, miR-6881-5p, miR-6881-3p, miR-6882-5p, miR-6882-3p, miR-6883-5p, miR-6883-3p, miR-6884-3p, miR-6885-3p, miR-6886-5p, miR-6886-3p, miR-6887-5p, miR-6887-3p, miR-6888-5p, miR-6889-5p, miR-6889-3p, miR-6890-5p, miR-6890-3p, miR-6891-5p, miR-6891-3p, miR-6892-5p, miR-6892-3p, miR-6893-5p, miR-6893-3p, miR-6894-5p, miR-6894-3p, miR-6895-3p, miR-7106-5p, miR-7106-3p, miR-7107-5p, miR-7107-3p, miR-7108-5p, miR-7108-3p, miR-7109-5p, miR-7109-3p, miR-7110-5p, miR-7110-3p, miR-7111-5p, miR-7111-3p, miR-7112-5p, miR-7113-5p, miR-7113-3p, miR-7114-5p, miR-7114-3p, miR-7150, miR-7151-3p, miR-7152-5p, miR-7152-3p, miR-7154-3p, miR-7155-5p, miR-7157-3p, miR-7159-5p, miR-7160-5p, miR-7161-5p, miR-7162-5p, miR-7515, miR-7702, miR-7704, miR-7843-5p, miR-4433b-5p, miR-4433b-3p, miR-1273h-5p, miR-6516-5p, miR-7845-5p, miR-7846-3p, miR-7847-3p, miR-7851-3p, miR-7855-5p, miR-7856-5p, miR-8052, miR-8054, miR-8057, miR-8059, miR-8060, miR-8062, miR-8063, miR-8064, miR-8068, miR-8069, miR-8070, miR-8071, miR-8072, miR-8073, miR-8074, miR-8075, miR-8077, miR-8078, miR-8079, miR-8080, miR-8085, miR-8086, miR-8087, miR-8089, miR-128-2-5p, miR-1199-5p, miR-7977, miR-7978, miR-1249-5p, miR-4485-5p, miR-8485, miR-196a-1-3p, miR-217-3p, miR-135a-2-3p, miR-320a-5p, miR-375-5p, miR-498-3p, miR-526a-3p, miR-9718, miR-9898, miR-9899, miR-1843, miR-10392-5p, miR-10392-3p, miR-10394-3p, miR-10395-3p, miR-10396a-5p, miR-10396a-3p, miR-10398-5p, miR-10398-3p, miR-10400-5p, miR-10400-3p, miR-10401-5p, miR-10401-3p, miR-10396b-5p, miR-10522-5p, miR-10524-5p, miR-10526-3p, miR-10527-5p, miR-11181-5p, miR-11181-3p, miR-11399, miR-11400, miR-3059-5p, miR-3059-3p, miR-3085-5p, miR-3085-3p, miR-6529-5p, miR-12114, miR-12115, miR-12116, miR-12118, miR-12119, miR-12120, miR-12121, miR-12122, miR-12124, miR-12126, miR-12127, miR-12128, miR-12131, and miR-12136. In the present disclosure, the extract of urine may be an extract of the urine of a stage-I endometrial cancer patient. Only one of these microRNAs can be used to predict whether a subject from which the urine is derived is an endometrial cancer patient (particularly stage-I), and 5 or more types, 10 or more types, 15 or more types, or 20 or more types of these microRNAs can be used in combination to predict whether a subject from which the urine is derived is an endometrial cancer patient (particularly stage-I). Among these microRNAs, a microRNA that may be highly expressed in an endometrial cancer patient (particularly stage-I) can be preferably used for prediction.

The present disclosure provides an extract of urine containing at least one type, two types, three types, four types, five types, six types, seven types, eight types, nine types, or ten types of or all microRNAs selected from the group consisting of microRNAs having an accuracy of higher than 0.500 among the microRNAs listed in Table 24-15. In the present disclosure, the extract of urine may be an extract of the urine of an endometrial cancer patient. Only one of these microRNAs can be used to predict whether a subject from which the urine is derived is an endometrial cancer patient, and 5 or more types, 10 or more types, 15 or more types, or 20 or more types of these microRNAs can be used in combination to predict whether a subject from which the urine is derived is an endometrial cancer patient. Among these microRNAs, a microRNA that may be highly expressed in an endometrial cancer patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

The present disclosure provides an extract of urine containing at least one type, two types, three types, four types, five types, six types, seven types, eight types, nine types, or ten types of or all microRNAs selected from the group consisting of microRNAs having an accuracy of higher than 0.500 among the microRNAs listed in Table 25-15. In the present disclosure, the extract of urine may be an extract of the urine of an endometrial cancer patient. Only one of these microRNAs can be used to predict whether a subject from which the urine is derived is an endometrial cancer patient, and 5 or more types, 10 or more types, 15 or more types, or 20 or more types of these microRNAs can be used in combination to predict whether a subject from which the urine is derived is an endometrial cancer patient. Among these microRNAs, a microRNA that may be highly expressed in an endometrial cancer patient can be preferably used for prediction. In one embodiment, the accuracy may be higher than 0.5, 0.55 or higher, 0.6 or higher, 0.65 or higher, 0.7 or higher, 0.75 or higher, 0.8 or higher, 0.85 or higher, or 0.9 or higher.

The present disclosure provides the group (ALL) consisting of:
miR-134-5p, miR-346, miR-564, miR-574-3p, miR-575, miR-632, miR-661, miR-663a, miR-658, miR-297, miR-139-3p, miR-149-3p, miR-20b-3p, miR-431-3p, miR-550a-5p, miR-885-5p, miR-936, miR-937-3p, miR-943, miR-1228-5p, miR-1184, miR-1204, miR-1538, miR-1909-5p, miR-1910-5p, miR-1912-3p, miR-196b-3p, miR-1972, miR-3153, miR-3162-5p, miR-1193, miR-4260, miR-4253, miR-4326, miR-4269, miR-4276, miR-3622a-3p, miR-3646, miR-3652, miR-3180, miR-550b-3p, miR-4428, miR-4433a-3p, miR-4440, miR-4444, miR-4447, miR-4449, miR-4455, miR-3155b, miR-4483, miR-4498, miR-2392, miR-4535, miR-4539, miR-3173-5p, miR-4529-5p, miR-4638-5p, miR-4655-5p, miR-4685-3p, miR-1343-3p, miR-4701-3p, miR-4717-3p, miR-4725-5p, miR-4726-3p, miR-4732-5p, miR-4738-3p, miR-4748, miR-4750-5p, miR-37lb-5p, miR-4436b-5p, miR-4793-3p, miR-5001-3p, miR-5002-3p, miR-5006-5p, miR-5090, miR-5572, miR-5584-3p, miR-5705, miR-1237-5p, miR-6072, miR-6131, miR-6503-3p, miR-6511b-5p, miR-1296-3p, miR-5189-3p, miR-6728-5p, miR-6729-5p, miR-6736-3p, miR-6738-5p, miR-6738-3p, miR-6740-3p, miR-6753-5p, miR-6754-3p, miR-6756-5p, miR-6759-5p, miR-6765-5p, miR-6768-5p, miR-6772-3p, miR-6773-3p, miR-6787-3p, miR-6790-5p, miR-6792-5p, miR-6798-5p, miR-6804-3p, miR-6805-5p, miR-6809-3p, miR-6816-5p, miR-6820-5p, miR-6821-5p, miR-6821-3p, miR-6822-5p, miR-6830-3p, miR-6832-3p, miR-6833-5p, miR-6833-3p, miR-6834-5p, miR-6780b-3p, miR-6854-3p, miR-6862-5p, miR-6867-5p, miR-6868-3p, miR-6871-5p, miR-6875-3p, miR-6877-5p, miR-6878-3p, miR-6882-3p, miR-6883-5p, miR-6883-3p, miR-6893-3p, miR-6894-3p, miR-7109-3p, miR-7152-5p, miR-7160-5p, miR-7843-5p, miR-8059, miR-8077, miR-7977, miR-4485-5p, miR-8485, miR-9718, miR-10396a-5p, miR-10398-5p, miR-10396b-5p, and miR-11400. These microRNAs are microRNAs listed in the above tables and having variations observed in common to all cancer types.

The present disclosure provides an extract of urine containing at least one type, two types, three types, four types, five types, six types, seven types, eight types, nine types, or ten types of or all microRNAs selected from the group (ALL). The present disclosure also provides an extract of urine containing at least one type, two types, three types, four types, five types, six types, seven types, eight types, nine types, or ten types of or all microRNAs selected from the group consisting of microRNAs having a p-value of less than 0.005 in Tables 4-1 to 4-15 in the group (ALL). The present disclosure provides an extract of urine containing at least one type, two types, three types, four types, five types, six types, seven types, eight types, nine types, or ten types of or all microRNAs selected from the group consisting of the microRNAs listed in Tables 22-1 and 23-1 to 23-14 in the group (ALL). The present disclosure provides an extract of urine containing at least one type, two types, three types, four types, five types, six types, seven types, eight types, nine types, or ten types of or all microRNAs selected from the group consisting of the microRNAs listed in Tables 22-1 to 22-13 in the group (ALL). The present disclosure provides an extract of urine containing at least one type, two types, three types, four types, five types, six types, seven types, eight types, nine types, or ten types of or all microRNAs selected from the group consisting of the microRNAs listed in Tables 24-1 to 24-15 in the group (ALL). The present disclosure provides an extract of urine containing at least one type, two types, three types, four types, five types, six types, seven types, eight types, nine types, or ten types of or all microRNAs selected from the group consisting of the microRNAs listed in Tables 25-1 to 25-15 in the group (ALL). The microRNAs listed in Tables 24-1 to 24-15 and the microRNAs listed in Tables 25-1 to 25-15 may be those having an accuracy of higher than 0.500 in the tables. According to the present disclosure, a likelihood that a subject from which the urine is derived has cancer can be predicted using as an indicator whether the urine obtained from the subject corresponds to any of the extracts of urine described above. Alternatively, according to the present disclosure, a likelihood that a subject from which the urine is derived has cancer can be predicted when the urine obtained from the subject contains a microRNA selected from the group at a predetermined value (first threshold) or more. According to the present disclosure, a likelihood that a subject from which the urine is derived has cancer can also be predicted when the urine obtained from the subject contains a microRNA selected from the group at a predetermined value (second threshold) or less. The predetermined value (the first threshold or the second threshold) can be independently set for each microRNA.

The present disclosure provides the group (GI) consisting of:
let-7e-5p, miR-23a-3p, miR-24-3p, miR-25-3p, miR-28-5p, miR-29a-3p, miR-30a-3p, miR-92a-3p, miR-29b-3p, miR-105-5p, miR-192-5p, miR-199a-5p, miR-129-5p, miR-30c-5p, miR-181a-5p, miR-181b-5p, miR-182-5p, miR-187-3p, miR-205-5p, miR-210-3p, miR-211-5p, miR-212-3p, miR-214-3p, miR-221-3p, miR-200b-3p, let-7i-5p, miR-15b-5p, miR-23b-3p, miR-27b-3p, miR-138-5p, miR-143-3p, miR-134-5p, miR-146a-5p, miR-154-5p, miR-184, miR-194-5p, miR-34b-5p, miR-34c-5p, miR-299-3p, miR-99b-5p, miR-296-5p, miR-361-5p, miR-370-3p, miR-371a-3p, miR-373-5p, miR-378a-3p, miR-381-3p, miR-382-5p, miR-340-3p, miR-330-3p, miR-328-3p, miR-342-3p, miR-323a-3p, miR-338-3p, miR-325, miR-346, miR-196b-5p, miR-422a, miR-449a, miR-200a-5p, miR-433-3p, miR-323b-5p, miR-484, miR-485-5p, miR-485-3p, miR-486-5p, miR-487a-3p, miR-488-5p, miR-489-3p, miR-491-5p, miR-511-5p, miR-202-3p, miR-492, miR-432-5p, miR-494-3p, miR-497-5p, miR-512-5p, miR-512-3p, miR-498-5p, miR-515-5p, miR-515-3p, miR-519e-3p, miR-520a-3p, miR-526b-5p, miR-525-5p, miR-525-3p, miR-517a-3p, miR-517b-3p, miR-519d-3p, miR-521, miR-520d-3p, miR-520g-3p, miR-516b-3p, miR-516a-3p, miR-517c-3p, miR-520h, miR-519a-3p, miR-500a-3p, miR-501-5p, miR-513a-5p, miR-506-3p, miR-509-3p, miR-510-5p, miR-299-5p, miR-18a-3p, miR-493-3p, miR-487b-3p, miR-551a, miR-554, miR-92b-3p, miR-557, miR-558, miR-564, miR-551b-3p, miR-572, miR-574-3p, miR-575, miR-579-3p, miR-584-5p, miR-589-3p, miR-550a-3p, miR-591, miR-593-5p, miR-595, miR-601, miR-602, miR-603, miR-604, miR-608, miR-609, miR-610, miR-612, miR-614, miR-615-3p, miR-616-5p, miR-617, miR-622, miR-623, miR-628-3p, miR-629-3p, miR-631, miR-632, miR-634, miR-635, miR-636, miR-637, miR-638, miR-639, miR-642a-5p, miR-644a, miR-645, miR-649, miR-650, miR-661, miR-663a, miR-449b-5p, miR-654-5p, miR-657, miR-658, miR-542-5p, miR-363-5p, miR-671-5p, miR-668-3p, miR-454-5p, miR-769-5p, miR-769-3p, miR-766-3p, miR-765, miR-770-5p, miR-675-5p, miR-297, let-7d-3p, miR-15a-3p, miR-16-1-3p, miR-21-3p, miR-23a-5p, miR-25-5p, miR-26a-1-3p, miR-26b-3p, miR-92a-2-5p, miR-16-2-3p, miR-139-3p, miR-7-1-3p, miR-181a-2-3p, miR-181c-3p, miR-183-3p, miR-187-5p, miR-214-5p, miR-222-5p, miR-200b-5p, miR-15b-3p, miR-30b-3p, miR-124-5p, miR-125b-1-3p, miR-135a-3p, miR-138-2-3p, miR-141-5p, miR-125a-3p, miR-125b-2-3p, miR-138-1-3p, miR-149-3p, miR-185-3p, miR-186-3p, miR-193a-5p, miR-200c-5p, miR-194-3p, miR-106b-3p, miR-29c-5p, miR-219a-2-3p, miR-34b-3p, miR-34c-3p, miR-99b-3p, miR-296-3p, miR-130b-5p, miR-371a-5p, miR-377-5p, miR-330-5p, miR-342-5p, miR-323a-5p, miR-151a-5p, miR-338-5p, miR-339-3p, miR-335-3p, miR-423-5p, miR-424-3p, miR-18b-3p, miR-20b-3p, miR-431-3p, miR-483-5p, miR-486-3p, miR-146b-3p, miR-193b-5p, miR-500a-5p, miR-505-5p, miR-508-5p, miR-532-3p, miR-92b-5p, miR-551b-5p, miR-574-5p, miR-589-5p, miR-550a-5p, miR-593-3p, miR-615-5p, miR-625-3p, miR-629-5p, miR-411-3p, miR-654-3p, miR-671-3p, miR-891a-5p, miR-300, miR-892a, miR-874-3p, miR-888-3p, miR-892b, miR-541-5p, miR-541-3p, miR-875-3p, miR-708-5p, miR-147b-3p, miR-744-5p, miR-744-3p, miR-885-5p, miR-885-3p, miR-877-5p, miR-877-3p, miR-887-3p, miR-665, miR-760, miR-920, miR-921, miR-935, miR-936, miR-937-3p, miR-938, miR-939-5p, miR-940, miR-943, miR-1224-5p, miR-1224-3p, miR-1225-5p, miR-1225-3p, miR-1226-5p, miR-1228-5p, miR-1228-3p, miR-1229-3p, miR-1231, miR-1233-3p, miR-1234-3p, miR-1236-3p, miR-1237-3p, miR-1238-3p, miR-513b-5p, miR-513c-5p, miR-320c, miR-1271-5p, miR-1298-5p, miR-1178-3p, miR-1181, miR-1182, miR-1184, miR-1203, miR-663b, miR-1204, miR-1207-5p, miR-1207-3p, miR-1285-3p, miR-1286, miR-1287-5p, miR-1291, miR-1293, miR-1294, miR-1297, miR-1303, miR-1304-5p, miR-1249-3p, miR-1250-5p, miR-1260a, miR-1263, miR-1265, miR-1266-5p, miR-1268a, miR-1269a, miR-1270, miR-1276, miR-1281, miR-1284, miR-1288-3p, miR-1292-5p, miR-1306-3p, miR-1307-3p, miR-1321, miR-1322, miR-1825, miR-1468-5p, miR-1469, miR-1470, miR-1471, miR-1538, miR-103b, miR-1908-5p, miR-1909-5p, miR-1909-3p, miR-1910-5p, miR-1911-3p, miR-1912-3p, miR-1913, miR-1914-3p, miR-1915-5p, miR-1915-3p, miR-103a-2-5p, miR-224-3p, miR-365a-5p, miR-196b-3p, miR-449b-3p, miR-2113, miR-1972, miR-1976, miR-2110, miR-151b, miR-449c-5p, miR-762, miR-761, miR-764, miR-548q, miR-2276-3p, miR-711, miR-718, miR-2681-5p, miR-2682-5p, miR-2682-3p, miR-449c-3p, miR-2861, miR-2909, miR-3122, miR-3124-5p, miR-3125, miR-3127-5p, miR-3130-3p, miR-3130-5p, miR-378b, miR-466, miR-3137, miR-3138, miR-3141, miR-3142, miR-1273c, miR-3147, miR-3148, miR-3150a-3p, miR-3151-5p, miR-3153, miR-3074-3p, miR-3154, miR-3155a, miR-3158-3p, miR-3160-3p, miR-3161, miR-3162-5p, miR-3163, miR-3165, miR-1260b, miR-3170, miR-3173-3p, miR-1193, miR-3174, miR-3175, miR-3176, miR-3178, miR-3180-3p, miR-3185, miR-3186-5p, miR-3186-3p, miR-3187-3p, miR-3189-3p, miR-320e, miR-3191-3p, miR-3192-5p, miR-3193, miR-3194-5p, miR-3195, miR-3196, miR-3197, miR-3198, miR-3199, miR-3200-3p, miR-514b-5p, miR-514b-3p, miR-3126-3p, miR-3065-3p, miR-4296, miR-4297, miR-378c, miR-4293, miR-4294, miR-4299, miR-4298, miR-4300, miR-4304, miR-4303, miR-4305, miR-4308, miR-4311, miR-4315, miR-4316, miR-4314, miR-4318, miR-4319, miR-4317, miR-4322, miR-4321, miR-4323, miR-4324, miR-4257, miR-4258, miR-4259, miR-4260, miR-4253, miR-4254, miR-4255, miR-4252, miR-4326, miR-4327, miR-4261, miR-4265, miR-4262, miR-4268, miR-4269, miR-4264, miR-4271, miR-4273, miR-4276, miR-4274, miR-4279, miR-4278, miR-4280, miR-4285, miR-4283, miR-4287, miR-4289, miR-4329, miR-4328, miR-2277-5p, miR-3605-5p, miR-3610, miR-3614-5p, miR-3615, miR-3616-3p, miR-3619-5p, miR-3620-3p, miR-3621, miR-3622a-5p, miR-3622a-3p, miR-3622b-5p, miR-3646, miR-3648, miR-3649, miR-3650, miR-3651, miR-3652, miR-3654, miR-3655, miR-3657, miR-3659, miR-3660, miR-3661, miR-3663-5p, miR-3663-3p, miR-3665, miR-3666, miR-3667-5p, miR-3675-5p, miR-3675-3p, miR-3677-3p, miR-3678-3p, miR-3679-5p, miR-3679-3p, miR-3680-3p, miR-3681-3p, miR-3682-3p, miR-3685, miR-3689a-3p, miR-3690, miR-3691-5p, miR-3692-5p, miR-3713, miR-3180, miR-3907, miR-3689b-3p, miR-3689c, miR-3911, miR-3913-5p, miR-3917, miR-3918, miR-3919, miR-3150b-3p, miR-3925-5p, miR-676-5p, miR-3928-3p, miR-3929, miR-3934-5p, miR-3936, miR-3937, miR-3940-3p, miR-3944-3p, miR-374c-5p, miR-642b-3p, miR-550b-3p, miR-1268b, miR-378e, miR-4420, miR-4421, miR-378g, miR-4425, miR-4428, miR-4429, miR-4430, miR-4431, miR-4433a-3p, miR-4435, miR-4436a, miR-4437, miR-4438, miR-4439, miR-4440, miR-4441, miR-4444, miR-4445-3p, miR-4446-3p, miR-4447, miR-4448, miR-4449, miR-4450, miR-4451, miR-4453, miR-4454, miR-4455, miR-4456, miR-4458, miR-3135b, miR-4462, miR-4463, miR-4465, miR-4466, miR-4467, miR-4470, miR-4472, miR-4473, miR-4474-3p, miR-4475, miR-4476, miR-4478, miR-3689d, miR-3689f, miR-4479, miR-3155b, miR-4481, miR-4483, miR-4484, miR-4486, miR-4487, miR-4488, miR-4489, miR-4491, miR-4492, miR-4494, miR-4496, miR-4497, miR-4498, miR-4502, miR-4505, miR-4506, miR-2392, miR-4507, miR-4508, miR-4510, miR-4511, miR-4513, miR-4514, miR-4515, miR-4516, miR-4521, miR-4523, miR-4524a-5p, miR-4526, miR-4527, miR-4529-3p, miR-4530, miR-4533, miR-378i, miR-4535, miR-1587, miR-4537, miR-4538, miR-4539, miR-4540, miR-3120-5p, miR-3129-3p, miR-3145-5p, miR-3150a-5p, miR-3152-5p, miR-3074-5p, miR-3157-3p, miR-3158-5p, miR-3160-5p, miR-3173-5p, miR-3187-5p, miR-3691-3p, miR-3913-3p, miR-3922-5p, miR-3925-3p, miR-3940-5p, miR-3944-5p, miR-4423-5p, miR-4520-5p, miR-4529-5p, miR-3960, miR-3975, miR-4634, miR-4635, miR-4638-5p, miR-4639-3p, miR-4640-5p, miR-4641, miR-4642, miR-4644, miR-4645-3p, miR-4646-5p, miR-4646-3p, miR-4647, miR-4648, miR-4649-5p, miR-4650-3p, miR-4651, miR-4652-5p, miR-4653-5p, miR-4654, miR-4655-5p, miR-4656, miR-4657, miR-4658, miR-4661-3p, miR-4664-5p, miR-4664-3p, miR-4665-5p, miR-4665-3p, miR-4667-5p, miR-4667-3p, miR-219b-5p, miR-4669, miR-4670-5p, miR-4672, miR-4673, miR-4674, miR-4676-5p, miR-4676-3p, miR-4681, miR-4682, miR-4684-5p, miR-4684-3p, miR-4685-5p, miR-4685-3p, miR-4686, miR-4687-5p, miR-4687-3p, miR-1343-3p, miR-4688, miR-4690-5p, miR-4691-5p, miR-4691-3p, miR-4692, miR-4694-3p, miR-4695-5p, miR-4697-5p, miR-4697-3p, miR-4698, miR-4700-5p, miR-4700-3p, miR-4701-5p, miR-4701-3p, miR-4706, miR-4707-5p, miR-4707-3p, miR-4708-5p, miR-4708-3p, miR-4709-5p, miR-4709-3p, miR-4710, miR-4711-3p, miR-4712-5p, miR-4713-5p, miR-4713-3p, miR-4716-5p, miR-4716-3p, miR-3529-5p, miR-4717-5p, miR-4717-3p, miR-4718, miR-4721, miR-4722-5p, miR-4722-3p, miR-4723-3p, miR-4724-5p, miR-4724-3p, miR-4725-5p, miR-4725-3p, miR-4726-5p, miR-4726-3p, miR-4727-3p, miR-4728-3p, miR-4730, miR-4731-5p, miR-4731-3p, miR-4732-5p, miR-4732-3p, miR-4733-5p, miR-4733-3p, miR-4734, miR-4736, miR-4737, miR-3064-5p, miR-3064-3p, miR-4738-3p, miR-4739, miR-4740-5p, miR-4741, miR-4743-5p, miR-122b-3p, miR-4745-5p, miR-4746-3p, miR-4747-5p, miR-4747-3p, miR-4748, miR-4749-5p, miR-4749-3p, miR-4750-5p, miR-4753-3p, miR-371b-5p, miR-371b-3p, miR-4754, miR-4755-3p, miR-4756-5p, miR-4756-3p, miR-4758-5p, miR-4758-3p, miR-4759, miR-4761-3p, miR-4763-5p, miR-4763-3p, miR-4764-3p, miR-4766-5p, miR-4767, miR-4768-5p, miR-4768-3p, miR-4769-5p, miR-4769-3p, miR-4773, miR-4776-5p, miR-4776-3p, miR-4778-5p, miR-4780, miR-4436b-5p, miR-4436b-3p, miR-4783-5p, miR-4783-3p, miR-4784, miR-4785, miR-2467-3p, miR-4786-5p, miR-4786-3p, miR-4787-5p, miR-4787-3p, miR-4788, miR-4793-5p, miR-4793-3p, miR-4794, miR-4796-5p, miR-4797-3p, miR-4800-5p, miR-4800-3p, miR-4801, miR-4804-3p, miR-642a-3p, miR-550a-3-5p, miR-4433a-5p, miR-4482-3p, miR-4999-5p, miR-4999-3p, miR-5000-5p, miR-5001-5p, miR-5001-3p, miR-5002-5p, miR-5002-3p, miR-5004-5p, miR-5004-3p, miR-5006-5p, miR-5006-3p, miR-5007-5p, miR-5008-5p, miR-5010-5p, miR-5088-5p, miR-5090, miR-5091, miR-5093, miR-5187-5p, miR-5188, miR-5189-5p, miR-5190, miR-5192, miR-5194, miR-5196-5p, miR-5196-3p, miR-5197-5p, miR-4524b-5p, miR-4524b-3p, miR-5571-3p, miR-5100, miR-5572, miR-664b-5p, miR-664b-3p, miR-5580-5p, miR-5580-3p, miR-5581-3p, miR-5584-3p, miR-5585-3p, miR-1295b-3p, miR-5588-5p, miR-5588-3p, miR-5589-5p, miR-5591-3p, miR-5684, miR-5685, miR-5681b, miR-5690, miR-5694, miR-5696, miR-5698, miR-5702, miR-5703, miR-5704, miR-5705, miR-5708, miR-197-5p, miR-204-3p, miR-211-3p, miR-212-5p, miR-301a-5p, miR-345-3p, miR-584-3p, miR-652-5p, miR-660-3p, miR-1185-2-3p, miR-766-5p, miR-1285-5p, miR-1304-3p, miR-1247-3p, miR-1255b-2-3p, miR-1306-5p, miR-3184-3p, miR-3191-5p, miR-642b-5p, miR-550b-2-5p, miR-365b-5p, miR-1185-1-3p, miR-3190-3p, miR-216a-3p, miR-381-5p, miR-495-5p, miR-503-3p, miR-937-5p, miR-939-3p, miR-1227-5p, miR-1229-5p, miR-1233-5p, miR-1236-5p, miR-1237-5p, miR-1238-5p, miR-3617-3p, miR-3620-5p, miR-3927-5p, miR-3934-3p, miR-4632-5p, miR-4743-3p, miR-5089-3p, miR-5739, miR-5787, miR-6068, miR-6069, miR-6071, miR-6072, miR-6074, miR-6075, miR-6076, miR-6078, miR-6080, miR-6081, miR-6083, miR-6085, miR-6086, miR-6088, miR-6089, miR-6090, miR-6124, miR-6125, miR-6126, miR-6127, miR-6129, miR-6130, miR-6131, miR-6132, miR-6133, miR-6134, miR-6165, miR-6499-5p, miR-6499-3p, miR-548ay-3p, miR-6500-5p, miR-6500-3p, miR-6501-5p, miR-6501-3p, miR-6502-5p, miR-6503-3p, miR-6504-5p, miR-6504-3p, miR-6505-3p, miR-6506-5p, miR-6508-5p, miR-6509-5p, miR-6510-5p, miR-6511a-5p, miR-6512-3p, miR-6513-5p, miR-6513-3p, miR-6514-5p, miR-6514-3p, miR-6515-5p, miR-6515-3p, miR-6715a-3p, miR-6715b-5p, miR-6716-5p, miR-6716-3p, miR-6717-5p, miR-6511b-5p, miR-6511b-3p, miR-6718-5p, miR-6719-3p, miR-6721-5p, miR-6722-5p, miR-6722-3p, miR-6724-5p, miR-892c-5p, miR-892c-3p, miR-208a-5p, miR-210-5p, miR-128-1-5p, miR-134-3p, miR-370-5p, miR-328-5p, miR-433-5p, miR-487a-5p, miR-489-5p, miR-494-5p, miR-504-3p, miR-487b-5p, miR-579-5p, miR-585-5p, miR-598-5p, miR-619-5p, miR-656-5p, miR-668-5p, miR-1296-3p, miR-1301-5p, miR-1298-3p, miR-891a-3p, miR-874-5p, miR-889-5p, miR-887-5p, miR-216b-3p, miR-942-3p, miR-1180-5p, miR-1287-3p, miR-1251-3p, miR-1266-3p, miR-1288-5p, miR-1908-3p, miR-1910-3p, miR-3151-3p, miR-3192-3p, miR-500b-3p, miR-3928-5p, miR-1343-5p, miR-5088-3p, miR-5189-3p, miR-6720-5p, miR-6726-5p, miR-6726-3p, miR-6727-5p, miR-6727-3p, miR-6728-5p, miR-6728-3p, miR-6729-5p, miR-6729-3p, miR-6730-5p, miR-6730-3p, miR-6731-5p, miR-6732-5p, miR-6732-3p, miR-6733-3p, miR-6734-5p, miR-6735-5p, miR-6735-3p, miR-6736-3p, miR-6737-5p, miR-6738-5p, miR-6738-3p, miR-6739-3p, miR-6740-5p, miR-6740-3p, miR-6741-5p, miR-6741-3p, miR-6742-5p, miR-6743-5p, miR-6743-3p, miR-6744-3p, miR-6747-5p, miR-6747-3p, miR-6748-5p, miR-6748-3p, miR-6749-5p, miR-6749-3p, miR-6750-5p, miR-6750-3p, miR-6751-5p, miR-6751-3p, miR-6752-5p, miR-6752-3p, miR-6753-5p, miR-6754-5p, miR-6754-3p, miR-6755-5p, miR-6755-3p, miR-6756-5p, miR-6756-3p, miR-6758-5p, miR-6758-3p, miR-6759-5p, miR-6759-3p, miR-6760-5p, miR-6760-3p, miR-6761-5p, miR-6762-5p, miR-6762-3p, miR-6763-5p, miR-6763-3p, miR-6764-5p, miR-6764-3p, miR-6765-5p, miR-6766-5p, miR-6766-3p, miR-6767-5p, miR-6768-5p, miR-6769a-5p, miR-6769a-3p, miR-6770-5p, miR-6770-3p, miR-6771-5p, miR-6772-5p, miR-6772-3p, miR-6773-3p, miR-6774-5p, miR-6774-3p, miR-6775-5p, miR-6776-5p, miR-6776-3p, miR-6777-5p, miR-6777-3p, miR-6778-5p, miR-6778-3p, miR-6779-5p, miR-6779-3p, miR-6781-5p, miR-6781-3p, miR-6782-5p, miR-6782-3p, miR-6783-5p, miR-6783-3p, miR-6784-5p, miR-6784-3p, miR-6785-3p, miR-6786-5p, miR-6786-3p, miR-6787-5p, miR-6787-3p, miR-6788-5p, miR-6789-5p, miR-6789-3p, miR-6790-5p, miR-6791-5p, miR-6791-3p, miR-6792-5p, miR-6792-3p, miR-6793-5p, miR-6794-5p, miR-6796-5p, miR-6796-3p, miR-6797-5p, miR-6797-3p, miR-6798-5p, miR-6798-3p, miR-6799-5p, miR-6800-5p, miR-6800-3p, miR-6801-5p, miR-6801-3p, miR-6802-3p, miR-6803-5p, miR-6803-3p, miR-6804-5p, miR-6804-3p, miR-6805-5p, miR-6805-3p, miR-6806-5p, miR-6807-5p, miR-6808-5p, miR-6809-5p, miR-6809-3p, miR-6810-5p, miR-6810-3p, miR-6812-5p, miR-6813-5p, miR-6813-3p, miR-6814-5p, miR-6815-3p, miR-6816-5p, miR-6816-3p, miR-6817-5p, miR-6817-3p, miR-6818-3p, miR-6819-3p, miR-6820-5p, miR-6820-3p, miR-6821-5p, miR-6821-3p, miR-6822-5p, miR-6822-3p, miR-6823-5p, miR-6823-3p, miR-6824-5p, miR-6824-3p, miR-6825-5p, miR-6825-3p, miR-6826-3p, miR-6827-5p, miR-6829-5p, miR-6829-3p, miR-6830-5p, miR-6830-3p, miR-6831-5p, miR-6832-5p, miR-6832-3p, miR-6833-5p, miR-6833-3p, miR-6834-5p, miR-6780b-5p, miR-6780b-3p, miR-6836-5p, miR-6836-3p, miR-6837-5p, miR-6838-5p, miR-6839-5p, miR-6840-3p, miR-6842-5p, miR-6842-3p, miR-6843-3p, miR-6845-5p, miR-6845-3p, miR-6846-5p, miR-6846-3p, miR-6847-5p, miR-6847-3p, miR-6848-5p, miR-6848-3p, miR-6849-5p, miR-6849-3p, miR-6850-5p, miR-6850-3p, miR-6851-5p, miR-6852-5p, miR-6852-3p, miR-6853-5p, miR-6854-3p, miR-6855-5p, miR-6855-3p, miR-6856-5p, miR-6856-3p, miR-6857-5p, miR-6858-5p, miR-6858-3p, miR-6859-5p, miR-6859-3p, miR-6769b-5p, miR-6860, miR-6861-5p, miR-6861-3p, miR-6862-5p, miR-6862-3p, miR-6863, miR-6866-3p, miR-6867-5p, miR-6867-3p, miR-6868-5p, miR-6868-3p, miR-6869-5p, miR-6870-5p, miR-6870-3p, miR-6871-5p, miR-6873-5p, miR-6874-5p, miR-6874-3p, miR-6875-5p, miR-6875-3p, miR-6876-5p, miR-6876-3p, miR-6877-5p, miR-6877-3p, miR-6878-3p, miR-6879-5p, miR-6879-3p, miR-6880-5p, miR-6880-3p, miR-6881-5p, miR-6882-3p, miR-6883-5p, miR-6883-3p, miR-6884-5p, miR-6884-3p, miR-6885-3p, miR-6886-5p, miR-6886-3p, miR-6887-3p, miR-6888-5p, miR-6889-5p, miR-6889-3p, miR-6890-5p, miR-6890-3p, miR-6891-5p, miR-6891-3p, miR-6892-5p, miR-6892-3p, miR-6893-5p, miR-6893-3p, miR-6894-5p, miR-6894-3p, miR-6895-5p, miR-6895-3p, miR-7106-5p, miR-7106-3p, miR-7107-5p, miR-7108-5p, miR-7108-3p, miR-7109-5p, miR-7109-3p, miR-7110-5p, miR-7110-3p, miR-7111-5p, miR-7111-3p, miR-7112-5p, miR-7113-5p, miR-7113-3p, miR-7114-5p, miR-7114-3p, miR-7150, miR-7151-5p, miR-7151-3p, miR-7152-5p, miR-7154-3p, miR-7155-5p, miR-7155-3p, miR-7156-5p, miR-7156-3p, miR-7158-5p, miR-7160-5p, miR-7162-3p, miR-7515, miR-7702, miR-7704, miR-7706, miR-7843-5p, miR-4433b-5p, miR-4433b-3p, miR-1273h-5p, miR-6516-5p, miR-7844-5p, miR-7845-5p, miR-7846-3p, miR-7847-3p, miR-7848-3p, miR-7850-5p, miR-7851-3p, miR-7853-5p, miR-7854-3p, miR-7856-5p, miR-8052, miR-8055, miR-8057, miR-8059, miR-8060, miR-8062, miR-8063, miR-8064, miR-8069, miR-8070, miR-8071, miR-8072, miR-8073, miR-8074, miR-8075, miR-8077, miR-8078, miR-8079, miR-8080, miR-8081, miR-8082, miR-8083, miR-8085, miR-8089, miR-128-2-5p, miR-7973, miR-7974, miR-7975, miR-7976, miR-7977, miR-1249-5p, miR-4485-5p, miR-8485, miR-9500, miR-103a-1-5p,miR-217-3p, miR-135a-2-3p, miR-375-5p, miR-498-3p, miR-9718, miR-9899, miR-9902, miR-1843, miR-9986, miR-10392-5p, miR-10392-3p, miR-10394-5p, miR-10394-3p, miR-10395-5p, miR-10396a-5p, miR-10396a-3p, miR-10397-5p, miR-10398-5p, miR-10398-3p, miR-10400-5p, miR-10400-3p, miR-10401-5p, miR-10401-3p, miR-10396b-5p, miR-10396b-3p, miR-10522-5p, miR-10523-5p, miR-10527-5p, miR-11181-3p, miR-11399, miR-11400, miR-11401, miR-3059-5p, miR-3059-3p, miR-3085-3p, miR-6529-5p, miR-6529-3p, miR-9851-5p, miR-12114, miR-12115, miR-12116, miR-12117, miR-12118, miR-12119, miR-12120, miR-12121, miR-12122, miR-12124, miR-12125, miR-12127, miR-12128, and miR-12131.

These microRNAs are microRNAs listed in the above tables and having variations observed in common to all cancer types.

The present disclosure provides an extract of urine containing at least one type, two types, three types, four types, five types, six types, seven types, eight types, nine types, or ten types of or all microRNAs selected from the group (GI). The present disclosure also provides an extract of urine containing at least one type, two types, three types, four types, five types, six types, seven types, eight types, nine types, or ten types of or all microRNAs selected from the group consisting of microRNAs having a p-value of less than 0.005 in Tables 4-1 to 4-15 in the group (GI). The present disclosure provides an extract of urine containing at least one type, two types, three types, four types, five types, six types, seven types, eight types, nine types, or ten types of or all microRNAs selected from the group consisting of the microRNAs listed in Tables 22-1 and 23-1 to 23-14 in the group (GI). The present disclosure provides an extract of urine containing at least one type, two types, three types, four types, five types, six types, seven types, eight types, nine types, or ten types of or all microRNAs selected from the group consisting of the microRNAs listed in Tables 22-1 to 22-13 in the group (GI). The present disclosure provides an extract of urine containing at least one type, two types, three types, four types, five types, six types, seven types, eight types, nine types, or ten types of or all microRNAs selected from the group consisting of the microRNAs listed in Tables 24-1 to 24-15 in the group (GI). The present disclosure provides an extract of urine containing at least one type, two types, three types, four types, five types, six types, seven types, eight types, nine types, or ten types of or all microRNAs selected from the group consisting of the microRNAs listed in Tables 25-1 to 25-15 in the group (GI). The microRNAs listed in Tables 24-1 to 24-15 and the microRNAs listed in Tables 25-1 to 25-15 may be those having an accuracy of higher than 0.500 in the tables. According to the present disclosure, a likelihood that a subject from which the urine is derived has cancer can be predicted using as an indicator whether the urine obtained from the subject corresponds to any of the extracts of urine described above. Alternatively, according to the present disclosure, a likelihood that a subject from which the urine is derived has cancer can be predicted when the urine obtained from the subject contains a microRNA selected from the group at a predetermined value (first threshold) or more. According to the present disclosure, a likelihood that a subject from which the urine is derived has cancer can also be predicted when the urine obtained from the subject contains a microRNA selected from the group at a predetermined value (second threshold) or less. The predetermined value (the first threshold or the second threshold) can be independently set for each microRNA.

The present disclosure provides the group (Hemo) consisting of:
miR-105-5p, miR-192-5p, miR-198, miR-129-5p, miR-187-3p, miR-210-3p, miR-212-3p, miR-214-3p, miR-200b-3p, miR-125b-5p, miR-138-5p, miR-134-5p, miR-184, miR-185-5p, miR-206, miR-106b-5p, miR-34c-5p, miR-99b-5p, miR-296-5p, miR-130b-3p, miR-302c-5p, miR-370-3p, miR-373-3p, miR-378a-3p, miR-382-5p, miR-340-3p, miR-342-3p, miR-337-3p, miR-323a-3p, miR-324-5p, miR-346, miR-422a, miR-425-3p, miR-369-5p, miR-323b-5p, miR-409-3p, miR-412-3p, miR-485-5p, miR-485-3p, miR-488-5p, miR-491-5p, miR-202-3p, miR-492, miR-432-3p, miR-494-3p, miR-512-5p, miR-512-3p, miR-498-5p, miR-520e-3p, miR-515-3p, miR-519c-5p, miR-523-5p, miR-518e-5p, miR-522-5p, miR-519a-5p, miR-519b-5p, miR-526b-5p, miR-525-5p, miR-518f-3p, miR-520b-3p, miR-526a-5p, miR-520c-5p, miR-518d-5p, miR-518c-5p, miR-519d-3p, miR-520d-5p, miR-516b-3p, miR-516a-3p, miR-501-5p, miR-513a-5p, miR-510-5p, miR-18a-3p, miR-551a, miR-92b-3p, miR-558, miR-564, miR-551b-3p, miR-572, miR-574-3p, miR-575, miR-583, miR-550a-3p, miR-595, miR-596, miR-601, miR-602, miR-604, miR-608, miR-610, miR-612, miR-614, miR-615-3p, miR-616-5p, miR-617, miR-622, miR-623, miR-629-3p, miR-632, miR-636, miR-637, miR-638, miR-639, miR-646, miR-650, miR-661, miR-663a, miR-654-5p, miR-657, miR-658, miR-421, miR-542-5p, miR-363-5p, miR-668-3p, miR-769-3p, miR-766-3p, miR-770-5p, miR-297, let-7d-3p, miR-15a-3p, miR-21-3p, miR-23a-5p, miR-25-5p, miR-26b-3p, miR-92a-2-5p, miR-30c-2-3p, miR-139-3p, miR-183-3p, miR-187-5p, miR-214-5p, miR-200b-5p, miR-30b-3p, miR-130a-5p, miR-135a-3p, miR-138-2-3p, miR-125a-3p, miR-125b-2-3p, miR-138-1-3p, miR-149-3p, miR-150-3p, miR-185-3p, miR-186-3p, miR-193a-5p, miR-200c-5p, miR-155-3p, miR-194-3p, miR-34b-3p, miR-34c-3p, miR-296-3p, miR-130b-5p, miR-361-3p, miR-302d-5p, miR-377-5p, miR-342-5p, miR-323a-5p, miR-338-5p, miR-424-3p, miR-18b-3p, miR-20b-3p, miR-431-3p, miR-483-5p, miR-486-3p, miR-193b-5p, miR-505-5p, miR-508-5p, miR-92b-5p, miR-551b-5p, miR-574-5p, miR-550a-5p, miR-593-3p, miR-615-5p, miR-625-3p, miR-629-5p, miR-671-3p, miR-891a-5p, miR-300, miR-892b, miR-541-5p, miR-541-3p, miR-744-5p, miR-885-5p, miR-885-3p, miR-877-5p, miR-887-3p, miR-665, miR-760, miR-920, miR-921, miR-935, miR-936, miR-937-3p, miR-938, miR-939-5p, miR-940, miR-943, miR-1224-5p, miR-1224-3p, miR-1225-5p, miR-1225-3p, miR-1226-5p, miR-1228-5p, miR-1228-3p, miR-1231, miR-1233-3p, miR-1237-3p, miR-1238-3p, miR-513b-5p, miR-320b, miR-320c, miR-1271-3p, miR-1301-3p, miR-1298-5p, miR-1178-3p, miR-1181, miR-1182, miR-1184, miR-1203, miR-1204, miR-1207-5p, miR-1285-3p, miR-1293, miR-1303, miR-1249-3p, miR-1250-5p, miR-548g-3p, miR-1266-5p, miR-1268a, miR-1269a, miR-1270, miR-1276, miR-1292-5p, miR-1306-3p, miR-1307-3p, miR-1321, miR-1468-5p, miR-675-3p, miR-1469, miR-1470, miR-1471, miR-1538, miR-1908-5p, miR-1909-5p, miR-1909-3p, miR-1910-5p, miR-1912-3p, miR-1913, miR-1914-3p, miR-1915-3p, miR-103a-2-5p, miR-365a-5p, miR-196b-3p, miR-449b-3p, miR-1972, miR-2110, miR-449c-5p, miR-762, miR-670-5p, miR-761, miR-764, miR-2116-5p, miR-548q, miR-2276-3p, miR-2277-3p, miR-711, miR-718, miR-2682-5p, miR-449c-3p, miR-3122, miR-3124-5p, miR-3126-5p, miR-3130-3p, miR-3130-5p, miR-3131, miR-3132, miR-3133, miR-466, miR-3137, miR-3138, miR-3141, miR-3147, miR-3150a-3p, miR-3151-5p, miR-3153, miR-3074-3p, miR-3155a, miR-3158-3p, miR-3161, miR-3162-5p, miR-3163, miR-3164, miR-3165, miR-1260b, miR-3173-3p, miR-1193, miR-3176, miR-3177-3p, miR-3178, miR-3180-3p, miR-3185, miR-3186-3p, miR-3187-3p, miR-3189-3p, miR-3191-3p, miR-3192-5p, miR-3195, miR-3196, miR-3197, miR-3198, miR-3199, miR-3202, miR-3126-3p, miR-4296, miR-4297, miR-4294, miR-4299, miR-4300, miR-4304, miR-4305, miR-4306, miR-4307, miR-4308, miR-4313, miR-4316, miR-4314, miR-4319, miR-4317, miR-4322, miR-4321, miR-4323, miR-4324, miR-4257, miR-4258, miR-4259, miR-4260, miR-4253, miR-4251, miR-4255, miR-4252, miR-4326, miR-4327, miR-4261, miR-4265, miR-4266, miR-4267, miR-4262, miR-4268, miR-4269, miR-4276, miR-4274, miR-4280, miR-4285, miR-4289, miR-2277-5p, miR-3200-5p, miR-3605-5p, miR-3605-3p, miR-3610, miR-3612, miR-3614-5p, miR-3616-3p, miR-3619-5p, miR-3621, miR-3622a-5p, miR-3622a-3p, miR-3622b-5p, miR-3646, miR-3648, miR-3650, miR-3651, miR-3652, miR-3654, miR-3655, miR-3659, miR-3660, miR-3663-5p, miR-3663-3p, miR-3665, miR-3667-5p, miR-3667-3p, miR-3675-3p, miR-3677-3p, miR-3679-5p, miR-3679-3p, miR-3682-3p, miR-3685, miR-3689a-3p, miR-3691-5p, miR-3713, miR-3714, miR-3180, miR-3689b-3p, miR-3689c, miR-3917, miR-3918, miR-3150b-3p, miR-3925-5p, miR-3928-3p, miR-3934-5p, miR-3935, miR-3936, miR-3937, miR-3944-3p, miR-3945, miR-550b-3p, miR-1268b, miR-4421, miR-4428, miR-4429, miR-4430, miR-4431, miR-4433a-3p, miR-4435, miR-4436a, miR-4439, miR-4440, miR-4441, miR-4443, miR-4444, miR-4446-3p, miR-4447, miR-4448, miR-4449, miR-4450, miR-4451, miR-4453, miR-4454, miR-4455, miR-4456, miR-4458, miR-3135b, miR-4462, miR-4463, miR-4465, miR-4466, miR-4467, miR-4470, miR-4472, miR-4474-3p, miR-4475, miR-4476, miR-3689d, miR-4479, miR-3155b, miR-4481, miR-4483, miR-4484, miR-4485-3p, miR-4486, miR-4487, miR-4488, miR-4489, miR-4492, miR-4496, miR-4497, miR-4498, miR-4499, miR-4502, miR-4505, miR-4506, miR-2392, miR-4507, miR-4508, miR-4510, miR-4512, miR-4513, miR-4514, miR-4515, miR-4516, miR-4521, miR-4525, miR-4526, miR-4530, miR-4531, miR-4533, miR-4534, miR-4535, miR-1587, miR-4538, miR-4539, miR-4540, miR-3120-5p, miR-3150a-5p, miR-3074-5p, miR-3156-3p, miR-3160-5p, miR-3162-3p, miR-3173-5p, miR-3187-5p, miR-3619-3p, miR-3922-5p, miR-3940-5p, miR-3944-5p, miR-4520-5p, miR-4529-5p, miR-3960, miR-4634, miR-4635, miR-4638-5p, miR-4639-3p, miR-4640-5p, miR-4642, miR-4646-5p, miR-4647, miR-4648, miR-4650-3p, miR-4651, miR-4652-5p, miR-4653-5p, miR-4653-3p, miR-4654, miR-4655-5p, miR-4657, miR-4658, miR-4664-5p, miR-4664-3p, miR-4665-5p, miR-4665-3p, miR-4667-5p, miR-219b-5p, miR-4669, miR-4670-5p, miR-4673, miR-4674, miR-4676-5p, miR-4681, miR-4682, miR-4684-5p, miR-4684-3p, miR-4685-3p, miR-4687-5p, miR-1343-3p, miR-4688, miR-4690-5p, miR-4690-3p, miR-4691-5p, miR-4692, miR-4695-5p, miR-4700-5p, miR-4701-3p, miR-4706, miR-4707-5p, miR-4707-3p, miR-4708-5p, miR-4708-3p, miR-203b-5p, miR-4710, miR-4711-3p, miR-4714-5p, miR-4716-5p, miR-4716-3p, miR-4717-5p, miR-4717-3p, miR-4720-5p, miR-4721, miR-4722-3p, miR-4724-5p, miR-4725-5p, miR-4725-3p, miR-4726-3p, miR-4727-3p, miR-4728-5p, miR-4728-3p, miR-4730, miR-4731-3p, miR-4732-5p, miR-4732-3p, miR-4734, miR-4736, miR-3064-5p, miR-3064-3p, miR-4738-3p, miR-4740-5p, miR-4740-3p, miR-4741, miR-4743-5p, miR-122b-3p, miR-4745-5p, miR-4746-3p, miR-4747-5p, miR-4747-3p, miR-4748, miR-4749-5p, miR-4749-3p, miR-4750-5p, miR-371b-5p, miR-4754, miR-4755-3p, miR-4758-5p, miR-4763-3p, miR-4764-3p, miR-4767, miR-4769-5p, miR-4776-5p, miR-4776-3p, miR-4780, miR-4436b-5p, miR-4436b-3p, miR-4781-5p, miR-4783-5p, miR-4783-3p, miR-4785, miR-2467-3p, miR-4786-5p, miR-4786-3p, miR-4787-5p, miR-4793-3p, miR-4800-5p, miR-4800-3p, miR-4801, miR-4804-3p, miR-550a-3-5p, miR-4433a-5p, miR-4482-3p, miR-4999-5p, miR-5001-5p, miR-5001-3p, miR-5002-3p, miR-5004-5p, miR-5004-3p, miR-5006-5p, miR-5009-3p, miR-5010-5p, miR-5088-5p, miR-5090, miR-5093, miR-5187-5p, miR-5188, miR-5189-5p, miR-5190, miR-5193, miR-5195-5p, miR-5196-5p, miR-5571-3p, miR-5100, miR-5572, miR-664b-5p, miR-664b-3p, miR-5580-3p, miR-5581-3p, miR-5584-3p, miR-5585-3p, miR-5587-5p, miR-5588-5p, miR-5588-3p, miR-5589-5p, miR-5685, miR-5681b, miR-5698, miR-5703, miR-5705, miR-197-5p, miR-204-3p, miR-211-3p, miR-301a-5p, miR-345-3p, miR-584-3p, miR-659-5p, miR-1185-2-3p, miR-766-5p, miR-873-3p, miR-1285-5p, miR-1247-3p, miR-1255b-2-3p, miR-3191-5p, miR-642b-5p, miR-550b-2-5p, miR-365b-5p, miR-1185-1-3p, miR-3190-3p, miR-937-5p, miR-1227-5p, miR-1236-5p, miR-1237-5p, miR-1238-5p, miR-1292-3p, miR-3617-3p, miR-3620-5p, miR-3934-3p, miR-4632-5p, miR-4750-3p, miR-5739, miR-5787, miR-6068, miR-6070, miR-6071, miR-6072, miR-6074, miR-6075, miR-6076, miR-6080, miR-6081, miR-6083, miR-6084, miR-6085, miR-6086, miR-6089, miR-6090, miR-6124, miR-6125, miR-6126, miR-6127, miR-6129, miR-6130, miR-6131, miR-6132, miR-6133, miR-6134, miR-6165, miR-6500-5p, miR-6500-3p, miR-6501-5p, miR-6501-3p, miR-6503-3p, miR-6504-3p, miR-6508-5p, miR-6509-3p, miR-6511a-5p, miR-6512-3p, miR-6513-5p, miR-6514-5p, miR-6514-3p, miR-6515-5p, miR-6515-3p, miR-6715b-5p, miR-6716-3p, miR-6717-5p, miR-6511b-5p, miR-6718-5p, miR-6721-5p, miR-6722-5p, miR-6722-3p, miR-6724-5p, miR-210-5p, miR-128-1-5p, miR-134-3p, miR-433-5p, miR-487a-5p, miR-489-5p, miR-504-3p, miR-487b-5p, miR-585-5p, miR-605-3p, miR-619-5p, miR-656-5p, miR-668-5p, miR-1296-3p, miR-1301-5p, miR-1298-3p, miR-874-5p, miR-887-5p, miR-1180-5p, miR-548j-3p, miR-1250-3p, miR-1266-3p, miR-1288-5p, miR-1908-3p, miR-1910-3p, miR-3192-3p, miR-500b-3p, miR-3928-5p, miR-1343-5p, miR-5088-3p, miR-5189-3p, miR-6720-5p, miR-6726-5p, miR-6727-5p, miR-6728-5p, miR-6728-3p, miR-6729-5p, miR-6729-3p, miR-6730-5p, miR-6730-3p, miR-6731-5p, miR-6731-3p, miR-6732-5p, miR-6734-5p, miR-6735-3p, miR-6736-5p, miR-6736-3p, miR-6737-5p, miR-6738-5p, miR-6738-3p, miR-6740-5p, miR-6740-3p, miR-6741-5p, miR-6742-5p, miR-6743-5p, miR-6745, miR-6746-5p, miR-6747-5p, miR-6748-5p, miR-6749-5p, miR-6750-5p, miR-6751-5p, miR-6752-5p, miR-6752-3p, miR-6753-5p, miR-6753-3p, miR-6754-3p, miR-6756-5p, miR-6758-5p, miR-6759-5p, miR-6760-5p, miR-6760-3p, miR-6761-5p, miR-6761-3p, miR-6762-5p, miR-6762-3p, miR-6763-5p, miR-6763-3p, miR-6765-5p, miR-6765-3p, miR-6766-5p, miR-6766-3p, miR-6767-5p, miR-6768-5p, miR-6769a-5p, miR-6770-5p, miR-6770-3p, miR-6772-5p, miR-6772-3p, miR-6773-3p, miR-6774-3p, miR-6776-5p, miR-6776-3p, miR-6777-5p, miR-6777-3p, miR-6778-5p, miR-6778-3p, miR-6779-3p, miR-6780a-5p, miR-6780a-3p, miR-6781-5p, miR-6781-3p, miR-6782-5p, miR-6783-5p, miR-6783-3p, miR-6784-5p, miR-6784-3p, miR-6785-3p, miR-6786-5p, miR-6787-5p, miR-6787-3p, miR-6788-5p, miR-6789-5p, miR-6789-3p, miR-6790-5p, miR-6791-5p, miR-6791-3p, miR-6792-5p, miR-6792-3p, miR-6793-5p, miR-6794-5p, miR-6795-5p, miR-6795-3p, miR-6796-5p, miR-6796-3p, miR-6797-5p, miR-6798-5p, miR-6798-3p, miR-6800-5p, miR-6800-3p, miR-6803-3p, miR-6804-5p, miR-6804-3p, miR-6805-5p, miR-6805-3p, miR-6806-5p, miR-6808-5p, miR-6809-5p, miR-6809-3p, miR-6810-5p, miR-6810-3p, miR-6812-5p, miR-6813-5p, miR-6813-3p, miR-6814-5p, miR-6815-5p, miR-6816-5p, miR-6816-3p, miR-6817-5p, miR-6819-3p, miR-6820-5p, miR-6820-3p, miR-6821-5p, miR-6821-3p, miR-6822-5p, miR-6823-5p, miR-6824-5p, miR-6825-5p, miR-6827-5p, miR-6829-5p, miR-6829-3p, miR-6830-5p, miR-6830-3p, miR-6831-5p, miR-6832-5p, miR-6832-3p, miR-6833-5p, miR-6833-3p, miR-6834-5p, miR-6780b-5p, miR-6780b-3p, miR-6836-5p, miR-6836-3p, miR-6837-5p, miR-6837-3p, miR-6838-5p, miR-6839-5p, miR-6840-3p, miR-6842-5p, miR-6843-3p, miR-6845-5p, miR-6845-3p, miR-6846-5p, miR-6847-5p, miR-6847-3p, miR-6848-5p, miR-6849-5p, miR-6849-3p, miR-6850-5p, miR-6850-3p, miR-6852-5p, miR-6852-3p, miR-6854-3p, miR-6855-5p, miR-6855-3p, miR-6856-5p, miR-6856-3p, miR-6857-5p, miR-6859-5p, miR-6859-3p, miR-6769b-5p, miR-6860, miR-6861-5p, miR-6862-5p, miR-6867-5p, miR-6867-3p, miR-6868-3p, miR-6869-5p, miR-6870-5p, miR-6871-5p, miR-6872-5p, miR-6873-5p, miR-6874-5p, miR-6875-5p, miR-6875-3p, miR-6876-5p, miR-6877-5p, miR-6877-3p, miR-6878-3p, miR-6879-5p, miR-6879-3p, miR-6880-5p, miR-6880-3p, miR-6881-5p, miR-6882-3p, miR-6883-5p, miR-6883-3p, miR-6886-5p, miR-6887-5p, miR-6887-3p, miR-6889-5p, miR-6891-5p, miR-6891-3p, miR-6892-5p, miR-6892-3p, miR-6893-5p, miR-6893-3p, miR-6894-5p, miR-6894-3p, miR-6895-5p, miR-7106-5p, miR-7107-5p, miR-7108-5p, miR-7108-3p, miR-7109-3p, miR-7110-5p, miR-7111-5p, miR-7112-5p, miR-7113-5p, miR-7114-5p, miR-7114-3p, miR-7150, miR-7151-3p, miR-7152-5p, miR-7154-3p, miR-7155-5p, miR-7156-3p, miR-7160-5p, miR-7162-3p, miR-7515, miR-7702, miR-7704, miR-7843-5p, miR-4433b-5p, miR-4433b-3p, miR-1273h-5p, miR-6516-5p, miR-7845-5p, miR-7846-3p, miR-7851-3p, miR-7854-3p, miR-7855-5p, miR-7856-5p, miR-8052, miR-8057, miR-8059, miR-8060, miR-8063, miR-8064, miR-8069, miR-8070, miR-8071, miR-8072, miR-8073, miR-8074, miR-8075, miR-8077, miR-8078, miR-8079, miR-8082, miR-8085, miR-8087, miR-8088, miR-8089, miR-7974, miR-7975, miR-7976, miR-7977, miR-4485-5p, miR-8485, miR-103a-1-5p,miR-196a-1-3p, miR-217-3p, miR-135a-2-3p, miR-9718, miR-9898, miR-9902, miR-1843, miR-10226, miR-10392-5p, miR-10392-3p, miR-10394-3p, miR-10395-5p, miR-10396a-5p, miR-10396a-3p, miR-10398-5p, miR-10398-3p, miR-10400-5p, miR-10400-3p, miR-10401-5p, miR-10401-3p, miR-10396b-5p, miR-10396b-3p, miR-10522-5p, miR-10526-3p, miR-10527-5p, miR-11181-3p, miR-11399, miR-11400, miR-11401, miR-3059-3p, miR-3085-5p, miR-3085-3p, miR-6529-5p, miR-9851-5p, miR-12114, miR-12115, miR-12116, miR-12118, miR-12119, miR-12120, miR-12122, miR-12124, miR-12126, miR-12127, and miR-12128

These microRNAs are microRNAs listed in the above tables and having variations observed in common to all cancer types.

The present disclosure provides an extract of urine containing at least one type, two types, three types, four types, five types, six types, seven types, eight types, nine types, or ten types of or all microRNAs selected from the group (Hemo). The present disclosure also provides an extract of urine containing at least one type, two types, three types, four types, five types, six types, seven types, eight types, nine types, or ten types of or all microRNAs selected from the group consisting of microRNAs having a p-value of less than 0.005 in Tables 4-1 to 4-15 in the group (Hemo). The present disclosure provides an extract of urine containing at least one type, two types, three types, four types, five types, six types, seven types, eight types, nine types, or ten types of or all microRNAs selected from the group consisting of the microRNAs listed in Tables 22-1 and 23-1 to 23-14 in the group (Hemo). The present disclosure provides an extract of urine containing at least one type, two types, three types, four types, five types, six types, seven types, eight types, nine types, or ten types of or all microRNAs selected from the group consisting of the microRNAs listed in Tables 22-1 to 22-13 in the group (Hemo). The present disclosure provides an extract of urine containing at least one type, two types, three types, four types, five types, six types, seven types, eight types, nine types, or ten types of or all microRNAs selected from the group consisting of the microRNAs listed in Tables 24-1 to 24-15 in the group (Hemo). The present disclosure provides an extract of urine containing at least one type, two types, three types, four types, five types, six types, seven types, eight types, nine types, or ten types of or all microRNAs selected from the group consisting of the microRNAs listed in Tables 25-1 to 25-15 in the group (Hemo). The microRNAs listed in Tables 24-1 to 24-15 and the microRNAs listed in Tables 25-1 to 25-15 may be those having an accuracy of higher than 0.500 in the tables. According to the present disclosure, a likelihood that a subject from which the urine is derived has cancer can be predicted using as an indicator whether the urine obtained from the subject corresponds to any of the extracts of urine described above. Alternatively, according to the present disclosure, a likelihood that a subject from which the urine is derived has cancer can be predicted when the urine obtained from the subject contains a microRNA selected from the group at a predetermined value (first threshold) or more. According to the present disclosure, a likelihood that a subject from which the urine is derived has cancer can also be predicted when the urine obtained from the subject contains a microRNA selected from the group at a predetermined value (second threshold) or less. The predetermined value (the first threshold or the second threshold) can be independently set for each microRNA.

The present disclosure provides the group (Uro) consisting of:
let-7b-5p, miR-25-3p, miR-28-5p, miR-29a-3p, miR-29b-3p, miR-105-5p, miR-192-5p, miR-196a-5p, miR-197-3p, miR-198, miR-199a-5p, miR-129-5p, miR-30d-5p, miR-147a, miR-10a-5p, miR-34a-5p, miR-181a-5p, miR-181b-5p, miR-187-3p, miR-199b-5p, miR-204-5p, miR-210-3p, miR-211-5p, miR-212-3p, miR-214-3p, miR-221-3p, miR-223-3p, miR-200b-3p, miR-23b-3p, miR-30b-5p, miR-122-5p, miR-124-3p, miR-125b-5p, miR-138-5p, miR-125a-5p, miR-134-5p, miR-149-5p, miR-184, miR-185-5p, miR-194-5p, miR-206, miR-106b-5p, miR-34c-5p, miR-299-3p, miR-99b-5p, miR-296-5p, miR-130b-3p, miR-365a-3p, miR-365b-3p, miR-302c-5p, miR-370-3p, miR-373-5p, miR-373-3p, miR-378a-3p, miR-380-3p, miR-382-5p, miR-340-3p, miR-330-3p, miR-328-3p, miR-342-3p, miR-337-3p, miR-324-5p, miR-324-3p, miR-338-3p, miR-133b, miR-325, miR-346, miR-196b-5p, miR-422a, miR-425-3p, miR-448, miR-449a, miR-191-3p, miR-200a-5p, miR-369-5p, miR-433-3p, miR-323b-5p, miR-409-3p, miR-412-3p, miR-483-3p, miR-484, miR-485-5p, miR-485-3p, miR-486-5p, miR-488-5p, miR-489-3p, miR-491-5p, miR-202-3p, miR-492, miR-432-3p, miR-494-3p, miR-497-5p, miR-512-5p, miR-498-5p, miR-515-5p, miR-519c-5p, miR-523-5p, miR-518e-5p, miR-522-5p, miR-519a-5p, miR-519b-5p, miR-526b-5p, miR-525-5p, miR-525-3p, miR-518b, miR-518c-5p, miR-519d-3p, miR-520d-3p, miR-520g-3p, miR-516b-5p, miR-516b-3p, miR-516a-3p, miR-518a-3p, miR-502-5p, miR-504-5p, miR-513a-5p, miR-506-3p, miR-510-5p, miR-532-5p, miR-299-5p, miR-18a-3p, miR-493-3p, miR-487b-3p, miR-551a, miR-554, miR-92b-3p, miR-557, miR-558, miR-563, miR-564, miR-551b-3p, miR-572, miR-574-3p, miR-575, miR-579-3p, miR-580-3p, miR-583, miR-584-5p, miR-585-3p, miR-588, miR-589-3p, miR-550a-3p, miR-595, miR-601, miR-603, miR-604, miR-605-5p, miR-608, miR-610, miR-612, miR-614, miR-615-3p, miR-616-5p, miR-617, miR-622, miR-628-3p, miR-629-3p, miR-632, miR-634, miR-635, miR-636, miR-637, miR-638, miR-639, miR-642a-5p, miR-644a, miR-650, miR-548d-3p, miR-661, miR-663a, miR-449b-5p, miR-654-5p, miR-657, miR-658, miR-421, miR-542-5p, miR-671-5p, miR-668-3p, miR-767-3p, miR-454-5p, miR-769-5p, miR-769-3p, miR-766-3p, miR-765, miR-770-5p, miR-297, let-7b-3p, let-7d-3p, let-7f-1-3p, miR-21-3p, miR-23a-5p, miR-25-5p, miR-26b-3p, miR-92a-2-5p, miR-29b-2-5p, miR-106a-3p, miR-16-2-3p, miR-192-3p, miR-129-l-3p, miR-139-3p, miR-183-3p, miR-187-5p, miR-214-5p, miR-222-5p, miR-200b-5p, miR-15b-3p, miR-30b-3p, miR-124-5p, miR-125b-1-3p, miR-135a-3p, miR-138-2-3p, miR-140-3p, miR-125a-3p, miR-125b-2-3p, miR-127-5p, miR-129-2-3p, miR-138-1-3p, miR-149-3p, miR-150-3p, miR-185-3p, miR-186-3p, miR-193a-5p, miR-200c-5p, miR-194-3p, miR-30c-1-3p, miR-219a-2-3p, miR-34b-3p, miR-34c-3p, miR-99b-3p, miR-296-3p, miR-130b-5p, miR-361-3p, miR-302d-5p, miR-371a-5p, miR-330-5p, miR-342-5p, miR-323a-5p, miR-151a-5p, miR-338-5p, miR-423-5p, miR-424-3p, miR-18b-3p, miR-20b-3p, miR-431-3p, miR-483-5p, miR-486-3p, miR-490-5p, miR-146b-3p, miR-193b-5p, miR-516a-5p, miR-505-5p, miR-508-5p, miR-532-3p, miR-92b-5p, miR-551b-5p, miR-574-5p, miR-550a-5p, miR-593-3p, miR-615-5p, miR-625-3p, miR-629-5p, miR-654-3p, miR-671-3p, miR-891a-5p, miR-300, miR-874-3p, miR-888-3p, miR-892b, miR-541-5p, miR-541-3p, miR-876-3p, miR-708-5p, miR-147b-3p, miR-744-5p, miR-744-3p, miR-885-5p, miR-885-3p, miR-877-5p, miR-877-3p, miR-887-3p, miR-665, miR-760, miR-920, miR-921, miR-933, miR-935, miR-936, miR-937-3p, miR-938, miR-939-5p, miR-940, miR-943, miR-1224-5p, miR-1224-3p, miR-1225-5p, miR-1225-3p, miR-1226-5p, miR-1226-3p, miR-1227-3p, miR-1228-5p, miR-1228-3p, miR-1229-3p, miR-1231, miR-1233-3p, miR-1234-3p, miR-1236-3p, miR-1237-3p, miR-1238-3p, miR-513b-5p, miR-513c-5p, miR-320b, miR-320c, miR-1271-5p, miR-1301-3p, miR-1298-5p, miR-1178-3p, miR-1181, miR-1182, miR-1184, miR-1202, miR-1203, miR-1204, miR-1207-5p, miR-1207-3p, miR-1285-3p, miR-1286, miR-1287-5p, miR-1289, miR-1293, miR-1294, miR-1303, miR-1304-5p, miR-1249-3p, miR-1250-5p, miR-1257, miR-1260a, miR-548g-3p, miR-1263, miR-1266-5p, miR-1267, miR-1268a, miR-1269a, miR-1270, miR-1275, miR-1276, miR-1281, miR-1284, miR-1288-3p, miR-1292-5p, miR-1255b-5p, miR-664a-3p, miR-1306-3p, miR-1307-3p, miR-1321, miR-1322, miR-320d, miR-1825, miR-1468-5p, miR-675-3p, miR-1469, miR-1470, miR-1471, miR-1538, miR-1539, miR-103b, miR-1908-5p, miR-1909-5p, miR-1909-3p, miR-1910-5p, miR-1912-3p, miR-1913, miR-1914-3p, miR-1915-3p, miR-103a-2-5p, miR-365a-5p, miR-196b-3p, miR-449b-3p, miR-2113, miR-1972, miR-1976, miR-2110, let-7a-2-3p, miR-151b, miR-762, miR-670-5p, miR-761, miR-764, miR-2115-5p, miR-2116-5p, miR-2116-3p, miR-548q, miR-2276-3p, miR-2277-3p, miR-2278, miR-711, miR-718, miR-2681-3p, miR-2682-5p, miR-2682-3p, miR-449c-3p, miR-2909, miR-3116, miR-3119, miR-3120-3p, miR-3122, miR-3124-5p, miR-3125, miR-3126-5p, miR-3127-5p, miR-3130-3p, miR-3130-5p, miR-3131, miR-3132, miR-3133, miR-466, miR-3136-5p, miR-3137, miR-3138, miR-3141, miR-3144-5p, miR-1273c, miR-3147, miR-3148, miR-3149, miR-3150a-3p, miR-3151-5p, miR-3153, miR-3074-3p, miR-3154, miR-3155a, miR-3156-5p, miR-3157-5p, miR-3158-3p, miR-3160-3p, miR-3161, miR-3162-5p, miR-3163, miR-3165, miR-1260b, miR-3173-3p, miR-1193, miR-3175, miR-3176, miR-3177-3p, miR-3178, miR-3179, miR-3180-5p, miR-3180-3p, miR-3185, miR-3186-5p, miR-3186-3p, miR-3187-3p, miR-3188, miR-3189-3p, miR-320e, miR-3190-5p, miR-3191-3p, miR-3192-5p, miR-3195, miR-3196, miR-3197, miR-3198, miR-3199, miR-3200-3p, miR-514b-5p, miR-3202, miR-3126-3p, miR-4295, miR-4296, miR-4297, miR-4293, miR-4294, miR-4301, miR-4299, miR-4298, miR-4300, miR-4304, miR-4303, miR-4305, miR-4306, miR-4307, miR-4308, miR-4311, miR-4312, miR-4313, miR-4316, miR-4314, miR-4319, miR-4317, miR-4322, miR-4321, miR-4323, miR-4324, miR-4256, miR-4257, miR-4258, miR-4259, miR-4260, miR-4253, miR-4251, miR-4254, miR-4255, miR-4252, miR-4326, miR-4327, miR-4261, miR-4265, miR-4266, miR-4262, miR-2355-5p, miR-4268, miR-4269, miR-4271, miR-4276, miR-4274, miR-4279, miR-4278, miR-4280, miR-4285, miR-4283, miR-4284, miR-4286, miR-4288, miR-4292, miR-4289, miR-4290, miR-4329, miR-2277-5p, miR-3605-5p, miR-3605-3p, miR-3610, miR-3612, miR-3614-5p, miR-3615, miR-3616-3p, miR-3619-5p, miR-3620-3p, miR-3621, miR-3622a-5p, miR-3622a-3p, miR-3622b-5p, miR-3646, miR-3648, miR-3649, miR-3650, miR-3651, miR-3652, miR-3654, miR-3655, miR-3659, miR-3660, miR-3661, miR-3663-5p, miR-3663-3p, miR-3665, miR-3667-5p, miR-3667-3p, miR-3675-5p, miR-3675-3p, miR-3679-5p, miR-3679-3p, miR-3682-3p, miR-3685, miR-3689a-3p, miR-3691-5p, miR-3692-3p, miR-3713, miR-3714, miR-3180, miR-3689b-3p, miR-3689c, miR-3911, miR-3917, miR-3918, miR-3919, miR-3150b-3p, miR-3922-3p, miR-3925-5p, miR-676-3p, miR-3928-3p, miR-3929, miR-3934-5p, miR-3935, miR-3936, miR-3937, miR-3940-3p, miR-3943, miR-3944-3p, miR-3945, miR-642b-3p, miR-550b-3p, miR-1268b, miR-4420, miR-4421, miR-378g, miR-4425, miR-4428, miR-4429, miR-4430, miR-4431, miR-4433a-3p, miR-4435, miR-4436a, miR-4437, miR-4438, miR-4439, miR-4440, miR-4441, miR-4443, miR-4444, miR-4445-3p, miR-4446-3p, miR-4447, miR-4448, miR-4449, miR-4450, miR-4451, miR-4453, miR-4454, miR-4455, miR-4456, miR-4458, miR-378h, miR-3135b, miR-4462, miR-4463, miR-4465, miR-4466, miR-4467, miR-4470, miR-4472, miR-4474-3p, miR-4475, miR-4476, miR-4478, miR-3689d, miR-3689f, miR-4479, miR-3155b, miR-4481, miR-4483, miR-4484, miR-4486, miR-4487, miR-4488, miR-4489, miR-4491, miR-4492, miR-4494, miR-4496, miR-4497, miR-4498, miR-4499, miR-4502, miR-4505, miR-4506, miR-2392, miR-4507, miR-4508, miR-4510, miR-4513, miR-4514, miR-4516, miR-4518, miR-4520-3p, miR-4521, miR-1269b, miR-4522, miR-4523, miR-4524a-3p, miR-4525, miR-4526, miR-4529-3p, miR-4530, miR-4531, miR-4533, miR-4534, miR-378i, miR-4535, miR-1587, miR-4537, miR-4538, miR-4539, miR-4540, miR-3120-5p, miR-3127-3p, miR-3129-3p, miR-3150a-5p, miR-3152-5p, miR-3074-5p, miR-3156-3p, miR-3158-5p, miR-3160-5p, miR-3162-3p, miR-3173-5p, miR-3187-5p, miR-3189-5p, miR-3619-3p, miR-3691-3p, miR-3150b-5p, miR-3922-5p, miR-3940-5p, miR-3944-5p, miR-4423-5p, miR-4446-5p, miR-4520-5p, miR-4529-5p, miR-3960, miR-3972, miR-4633-3p, miR-4634, miR-4635, miR-4638-5p, miR-4639-3p, miR-4640-5p, miR-4640-3p, miR-4641, miR-4642, miR-4644, miR-4646-5p, miR-4646-3p, miR-4647, miR-4648, miR-4649-3p, miR-4650-5p, miR-4650-3p, miR-4651, miR-4652-5p, miR-4653-5p, miR-4653-3p, miR-4654, miR-4655-5p, miR-4656, miR-4657, miR-4658, miR-4660, miR-4661-3p, miR-4664-5p, miR-4664-3p, miR-4665-5p, miR-4665-3p, miR-4667-5p, miR-4667-3p, miR-219b-5p, miR-4669, miR-4672, miR-4673, miR-4674, miR-4676-5p, miR-4676-3p, miR-4677-5p, miR-4681, miR-4682, miR-4684-5p, miR-4684-3p, miR-4685-5p, miR-4685-3p, miR-4686, miR-4687-5p, miR-1343-3p, miR-4688, miR-4689, miR-4690-5p, miR-4691-5p, miR-4692, miR-4695-5p, miR-4695-3p, miR-4697-5p, miR-4697-3p, miR-4698, miR-4700-5p, miR-4700-3p, miR-4701-5p, miR-4701-3p, miR-4706, miR-4707-5p, miR-4707-3p, miR-4708-5p, miR-4708-3p, miR-4709-5p, miR-4709-3p, miR-4710, miR-4711-3p, miR-4713-5p, miR-4713-3p, miR-4714-5p, miR-4716-5p, miR-4716-3p, miR-3529-5p, miR-4717-5p, miR-4717-3p, miR-4718, miR-4720-5p, miR-4721, miR-4722-5p, miR-4722-3p, miR-4723-5p, miR-4723-3p, miR-4724-5p, miR-4725-5p, miR-4725-3p, miR-4726-5p, miR-4726-3p, miR-4727-3p, miR-4728-5p, miR-4728-3p, miR-4730, miR-4731-5p, miR-4731-3p, miR-4732-5p, miR-4732-3p, miR-4734, miR-4736, miR-4737, miR-3064-5p, miR-3064-3p, miR-4738-3p, miR-4740-5p, miR-4740-3p, miR-4741, miR-4743-5p, miR-122b-3p, miR-4745-5p, miR-4746-3p, miR-4747-5p, miR-4748, miR-4749-5p, miR-4749-3p, miR-4750-5p, miR-4751, miR-4753-5p, miR-4753-3p, miR-371b-5p, miR-371b-3p, miR-4754, miR-4755-3p, miR-4756-5p, miR-4756-3p, miR-4758-5p, miR-4758-3p, miR-4759, miR-4763-5p, miR-4763-3p, miR-4764-5p, miR-4766-5p, miR-4769-5p, miR-4769-3p, miR-4773, miR-4776-5p, miR-4776-3p, miR-4778-5p, miR-4779, miR-4780, miR-4436b-5p, miR-4436b-3p, miR-4781-5p, miR-4783-3p, miR-4784, miR-4785, miR-2467-3p, miR-4786-5p, miR-4786-3p, miR-4787-5p, miR-4787-3p, miR-4788, miR-4793-3p, miR-4794, miR-4800-5p, miR-4800-3p, miR-4801, miR-4804-3p, miR-642a-3p, miR-550a-3-5p, miR-4433a-5p, miR-4482-3p, miR-4999-5p, miR-5000-5p, miR-5000-3p, miR-5001-5p, miR-5001-3p, miR-5002-5p, miR-5002-3p, miR-5003-3p, miR-5004-5p, miR-5004-3p, miR-5006-5p, miR-5006-3p, miR-5007-5p, miR-5009-3p, miR-5010-5p, miR-5010-3p, miR-5087, miR-5088-5p, miR-5089-5p, miR-5090, miR-5093, miR-5187-5p, miR-5187-3p, miR-5188, miR-5189-5p, miR-5190, miR-5192, miR-5193, miR-5194, miR-5195-5p, miR-5196-5p, miR-5196-3p, miR-4524b-5p, miR-4524b-3p, miR-5571-5p, miR-5571-3p, miR-5100, miR-5572, miR-664b-5p, miR-664b-3p, miR-5580-5p, miR-5580-3p, miR-5581-5p, miR-5581-3p, miR-5584-5p, miR-5584-3p, miR-5585-3p, miR-5587-3p, miR-548au-3p, miR-5588-5p, miR-5588-3p, miR-5589-5p, miR-5684, miR-5685, miR-5681b, miR-5690, miR-5694, miR-5698, miR-5702, miR-5703, miR-5704, miR-5705, miR-5708, miR-197-5p, miR-204-3p, miR-211-3p, miR-345-3p, miR-584-3p, miR-652-5p, miR-660-3p, miR-1185-2-3p, miR-766-5p, miR-873-3p, miR-1285-5p, miR-1304-3p, miR-1247-3p, miR-1255b-2-3p, miR-1306-5p, miR-3184-3p, miR-3191-5p, miR-642b-5p, miR-550b-2-5p, miR-365b-5p, miR-1185-1-3p, miR-3190-3p, miR-98-3p, miR-216a-3p, miR-381-5p, miR-495-5p, miR-503-3p, miR-758-5p, miR-937-5p, miR-939-3p, miR-1227-5p, miR-1229-5p, miR-1233-5p, miR-1236-5p, miR-1237-5p, miR-1238-5p, miR-1292-3p, miR-3617-3p, miR-3620-5p, miR-3927-5p, miR-3934-3p, miR-4632-5p, miR-4750-3p, miR-5089-3p, miR-5739, miR-5787, miR-6068, miR-6069, miR-6071, miR-6072, miR-6074, miR-6075, miR-6076, miR-6077, miR-6080, miR-6081, miR-6083, miR-6084, miR-6085, miR-6086, miR-6089, miR-6090, miR-6124, miR-6125, miR-6126, miR-6127, miR-378j, miR-6129, miR-6130, miR-6131, miR-6132, miR-6133, miR-6165, miR-6499-3p, miR-6500-5p, miR-6500-3p, miR-6501-5p, miR-6501-3p, miR-6503-5p, miR-6503-3p, miR-6504-5p, miR-6504-3p, miR-6505-3p, miR-6506-5p, miR-6507-3p, miR-6508-5p, miR-6509-5p, miR-6509-3p, miR-6510-5p, miR-6511a-5p, miR-6511a-3p, miR-6512-3p, miR-6513-5p, miR-6513-3p, miR-6514-5p, miR-6514-3p, miR-6515-5p, miR-6515-3p, miR-6715a-3p, miR-6715b-5p, miR-6716-5p, miR-6716-3p, miR-6717-5p, miR-6511b-5p, miR-6511b-3p, miR-6718-5p, miR-6719-3p, miR-6721-5p, miR-6722-5p, miR-6722-3p, miR-6724-5p, miR-892c-5p, miR-892c-3p, let-7c-3p, miR-210-5p, miR-128-1-5p, miR-134-3p, miR-370-5p, miR-433-5p, miR-329-5p, miR-410-5p, miR-487a-5p, miR-489-5p, miR-494-5p, miR-504-3p, miR-487b-5p, miR-579-5p, miR-585-5p, miR-598-5p, miR-605-3p, miR-619-5p, miR-656-5p, miR-668-5p, miR-1296-3p, miR-1301-5p, miR-1298-3p, miR-874-5p, miR-889-5p, miR-887-5p, miR-1250-3p, miR-1251-3p, miR-1266-3p, miR-1288-5p, miR-1910-3p, miR-3151-3p, miR-3192-3p, miR-500b-3p, miR-3928-5p, miR-1343-5p, miR-5088-3p, miR-5189-3p, miR-5699-5p, miR-6720-5p, miR-6726-5p, miR-6726-3p, miR-6727-5p, miR-6727-3p, miR-6728-5p, miR-6728-3p, miR-6729-5p, miR-6729-3p, miR-6730-5p, miR-6730-3p, miR-6731-5p, miR-6731-3p, miR-6732-5p, miR-6732-3p, miR-6734-5p, miR-6735-5p, miR-6735-3p, miR-6736-5p, miR-6736-3p, miR-6737-5p, miR-6737-3p, miR-6738-5p, miR-6738-3p, miR-6739-3p, miR-6740-5p, miR-6740-3p, miR-6741-5p, miR-6741-3p, miR-6742-5p, miR-6742-3p, miR-6743-5p, miR-6743-3p, miR-6744-3p, miR-6744-3p, miR-6745, miR-6746-5p, miR-6746-3p, miR-6747-5p, miR-6747-3p, miR-6748-5p, miR-6748-3p, miR-6749-5p, miR-6749-3p, miR-6750-5p, miR-6750-3p, miR-6751-5p, miR-6751-3p, miR-6752-5p, miR-6752-3p, miR-6753-5p, miR-6753-3p, miR-6754-5p, miR-6754-3p, miR-6755-5p, miR-6756-5p, miR-6756-3p, miR-6757-5p, miR-6757-3p, miR-6758-5p, miR-6759-5p, miR-6759-3p, miR-6760-5p, miR-6760-3p, miR-6761-5p, miR-6761-3p, miR-6762-5p, miR-6762-3p, miR-6763-5p, miR-6763-3p, miR-6764-3p, miR-6765-5p, miR-6766-5p, miR-6766-3p, miR-6767-5p, miR-6768-5p, miR-6769a-5p, miR-6769a-3p, miR-6770-5p, miR-6770-3p, miR-6771-3p, miR-6772-5p, miR-6772-3p, miR-6773-3p, miR-6774-5p, miR-6774-3p, miR-6775-5p, miR-6775-3p, miR-6776-5p, miR-6776-3p, miR-6777-5p, miR-6777-3p, miR-6778-5p, miR-6778-3p, miR-6779-5p, miR-6779-3p, miR-6780a-5p, miR-6780a-3p, miR-6781-5p, miR-6781-3p, miR-6782-5p, miR-6782-3p, miR-6783-5p, miR-6783-3p, miR-6784-5p, miR-6784-3p, miR-6785-3p, miR-6786-5p, miR-6786-3p, miR-6787-3p, miR-6787-3p, miR-6788-5p, miR-6789-5p, miR-6789-3p, miR-6790-5p, miR-6790-3p, miR-6791-5p, miR-6791-3p, miR-6792-5p, miR-6792-3p, miR-6793-5p, miR-6793-3p, miR-6794-5p, miR-6794-3p, miR-6795-5p, miR-6795-3p, miR-6796-5p, miR-6796-3p, miR-6797-5p, miR-6797-3p, miR-6798-5p, miR-6798-3p, miR-6799-5p, miR-6799-3p, miR-6800-5p, miR-6800-3p, miR-6801-5p, miR-6801-3p, miR-6802-3p, miR-6803-5p, miR-6803-3p, miR-6804-5p, miR-6804-3p, miR-6805-5p, miR-6805-3p, miR-6806-5p, miR-6807-5p, miR-6808-5p, miR-6809-5p, miR-6809-3p, miR-6810-5p, miR-6810-3p, miR-6812-5p, miR-6812-3p, miR-6813-5p, miR-6813-3p, miR-6814-5p, miR-6814-3p, miR-6815-5p, miR-6815-3p, miR-6816-5p, miR-6816-3p, miR-6817-5p, miR-6817-3p, miR-6819-5p, miR-6819-3p, miR-6820-5p, miR-6820-3p, miR-6821-5p, miR-6821-3p, miR-6822-5p, miR-6822-3p, miR-6823-5p, miR-6823-3p, miR-6824-5p, miR-6824-3p, miR-6825-5p, miR-6825-3p, miR-6826-3p, miR-6827-5p, miR-6827-3p, miR-6828-5p, miR-6829-5p, miR-6829-3p, miR-6830-5p, miR-6830-3p, miR-6831-5p, miR-6831-3p, miR-6832-5p, miR-6832-3p, miR-6833-5p, miR-6833-3p, miR-6834-5p, miR-6834-3p, miR-6780b-5p, miR-6780b-3p, miR-6836-5p, miR-6836-3p, miR-6837-5p, miR-6838-5p, miR-6839-5p, miR-6840-5p, miR-6840-3p, miR-6841-3p, miR-6842-5p, miR-6842-3p, miR-6843-3p, miR-6845-5p, miR-6845-3p, miR-6846-5p, miR-6846-3p, miR-6847-5p, miR-6847-3p, miR-6848-5p, miR-6848-3p, miR-6849-5p, miR-6849-3p, miR-6850-5p, miR-6851-5p, miR-6851-3p, miR-6852-5p, miR-6852-3p, miR-6853-5p, miR-6854-3p, miR-6855-5p, miR-6855-3p, miR-6856-5p, miR-6857-5p, miR-6857-3p, miR-6858-5p, miR-6858-3p, miR-6859-5p, miR-6859-3p, miR-6769b-5p, miR-6860, miR-6861-5p, miR-6861-3p, miR-6862-5p, miR-6862-3p, miR-6863, miR-6865-3p, miR-6867-5p, miR-6867-3p, miR-6868-5p, miR-6868-3p, miR-6869-5p, miR-6870-5p, miR-6870-3p, miR-6871-5p, miR-6871-3p, miR-6872-3p, miR-6873-3p, miR-6874-5p, miR-6875-5p, miR-6875-3p, miR-6876-5p, miR-6876-3p, miR-6877-5p, miR-6877-3p, miR-6878-3p, miR-6879-5p, miR-6879-3p, miR-6880-5p, miR-6880-3p, miR-6881-5p, miR-6881-3p, miR-6882-5p, miR-6882-3p, miR-6883-5p, miR-6883-3p, miR-6884-5p, miR-6884-3p, miR-6885-3p, miR-6886-5p, miR-6886-3p, miR-6887-5p, miR-6887-3p, miR-6888-5p, miR-6889-5p, miR-6889-3p, miR-6890-3p, miR-6891-5p, miR-6891-3p, miR-6892-5p, miR-6892-3p, miR-6893-5p, miR-6893-3p, miR-6894-5p, miR-6894-3p, miR-6895-5p, miR-6895-3p, miR-7106-5p, miR-7106-3p, miR-7107-5p, miR-7108-5p, miR-7108-3p, miR-7109-5p, miR-7109-3p, miR-7110-5p, miR-7110-3p, miR-7111-5p, miR-7111-3p, miR-7112-5p, miR-7113-5p, miR-7113-3p, miR-7114-5p, miR-7114-3p, miR-7150, miR-7151-5p, miR-7151-3p, miR-7152-5p, miR-7152-3p, miR-7154-3p, miR-7155-5p, miR-7155-3p, miR-7156-3p, miR-7157-5p, miR-7157-3p, miR-7158-5p, miR-7160-5p, miR-7162-3p, miR-7515, miR-7702, miR-7843-5p, miR-4433b-5p, miR-4433b-3p, miR-1273h-5p, miR-1273h-3p, miR-6516-5p, miR-7845-5p, miR-7846-3p, miR-7847-3p, miR-7850-5p, miR-7851-3p, miR-7854-3p, miR-7855-5p, miR-7856-5p, miR-8052, miR-8054, miR-8057, miR-8059, miR-8060, miR-8062, miR-8063, miR-8064, miR-8069, miR-8070, miR-8071, miR-8072, miR-8073, miR-8074, miR-8075, miR-8077, miR-8078, miR-8079, miR-8080, miR-8082, miR-8083, miR-8085, miR-8086, miR-8087, miR-8088, miR-8089, miR-1199-3p, miR-7975, miR-7976, miR-7977, miR-7978, miR-1249-5p, miR-4485-5p, miR-8485, miR-103a-1-5p, miR-196a-1-3p, miR-135a-2-3p, miR-375-5p, miR-526a-3p, miR-9718, miR-9898, miR-9902, miR-1843, miR-9986, miR-10226, miR-10392-5p, miR-10392-3p, miR-10394-3p, miR-10395-5p, miR-10395-3p, miR-10396a-5p, miR-10396a-3p, miR-10398-5p, miR-10398-3p, miR-10400-3p, miR-10401-5p, miR-10401-3p, miR-10396b-5p, miR-10396b-3p, miR-10522-5p, miR-10523-5p, miR-10526-3p, miR-10527-5p, miR-11181-5p, miR-11181-3p, miR-11399, miR-11400, miR-11401, miR-3059-5p, miR-3059-3p, miR-3085-5p, miR-3085-3p, miR-6529-5p, miR-12114, miR-12115, miR-12116, miR-12117, miR-12118, miR-12119, miR-12120, miR-12121, miR-12122, miR-12124, miR-12125, miR-12126, miR-12127, miR-12128, miR-12131, and miR-12136. These microRNAs are microRNAs listed in the above tables and having variations observed in common to all cancer types.

The present disclosure provides an extract of urine containing at least one type, two types, three types, four types, five types, six types, seven types, eight types, nine types, or ten types of or all microRNAs selected from the group (Uro). The present disclosure also provides an extract of urine containing at least one type, two types, three types, four types, five types, six types, seven types, eight types, nine types, or ten types of or all microRNAs selected from the group consisting of microRNAs having a p-value of less than 0.005 in Tables 4-1 to 4-15 in the group (Uro). The present disclosure provides an extract of urine containing at least one type, two types, three types, four types, five types, six types, seven types, eight types, nine types, or ten types of or all microRNAs selected from the group consisting of the microRNAs listed in Tables 22-1 and 23-1 to 23-14 in the group (Uro). The present disclosure provides an extract of urine containing at least one type, two types, three types, four types, five types, six types, seven types, eight types, nine types, or ten types of or all microRNAs selected from the group consisting of the microRNAs listed in Tables 22-1 to 22-13 in the group (Uro). The present disclosure provides an extract of urine containing at least one type, two types, three types, four types, five types, six types, seven types, eight types, nine types, or ten types of or all microRNAs selected from the group consisting of the microRNAs listed in Tables 24-1 to 24-15 in the group (Uro). The present disclosure provides an extract of urine containing at least one type, two types, three types, four types, five types, six types, seven types, eight types, nine types, or ten types of or all microRNAs selected from the group consisting of the microRNAs listed in Tables 25-1 to 25-15 in the group (Uro). The microRNAs listed in Tables 24-1 to 24-15 and the microRNAs listed in Tables 25-1 to 25-15 may be those having an accuracy of higher than 0.500 in the tables. According to the present disclosure, a likelihood that a subject from which the urine is derived has cancer can be predicted using as an indicator whether the urine obtained from the subject corresponds to any of the extracts of urine described above. Alternatively, according to the present disclosure, a likelihood that a subject from which the urine is derived has cancer can be predicted when the urine obtained from the subject contains a microRNA selected from the group at a predetermined value (first threshold) or more. According to the present disclosure, a likelihood that a subject from which the urine is derived has cancer can also be predicted when the urine obtained from the subject contains a microRNA selected from the group at a predetermined value (second threshold) or less. The predetermined value (the first threshold or the second threshold) can be independently set for each microRNA.

In any of these embodiments, a microRNA that may be highly expressed in a cancer patient can be preferably used for prediction. In one embodiment, a microRNA that can be underexpressed in a cancer patient may be used for prediction. In one embodiment, a microRNA that may be highly expressed in a cancer patient is preferably used for prediction, and a microRNA that may be underexpressed in a cancer patient may also be used for prediction. High or low expression may be determined by comparing the expression level with the first quartile value, average value, median, or third quartile value of healthy persons.

Another aspect of the present disclosure provides a method for examining the likelihood that a subject has cancer. The method for examining the likelihood of cancer can be replaced with a method for diagnosing cancer, a method for acquiring preliminary information for diagnosing cancer, a method for determining whether a subject has cancer cells in the body, or a method for determining the likelihood that a subject has cancer. In the present disclosure, if it is determined that there is a likelihood that a subject has cancer by a method for examining the likelihood that a subject has cancer, then a doctor or the like can make a definitive diagnosis. In the present disclosure, a method according to the present disclosure includes diagnosing cancer and performing cancer therapy on a patient diagnosed with cancer. In the present invention, when a microRNA that may be highly expressed in a cancer patient is detected in urine, this may indicate that the patient from which the urine is derived has cancer or a likelihood thereof. In the present invention, when a microRNA that may be underexpressed in a cancer patient is detected in urine, this may indicate that the patient from which the urine is derived does not have cancer or a likelihood thereof.

In the present disclosure, the likelihood that a subject has cancer can be determined using the expression level of any microRNA listed in any of Tables 4-1 to 4-15 in a body fluid sample as an indicator.

In one embodiment of the present disclosure, a body fluid sample refers to a body fluid obtained from a subject or a sample derived from the body fluid. The body fluid sample may be blood, serum, plasma, or lymph, may be tissue fluid, such as intertissue fluid, intercellular fluid, or interstitial fluid, or may be coelomic fluid, serous cavity fluid, pleural effusion, ascites, pericardial fluid, cerebrospinal fluid (spinal fluid), joint fluid (synovial fluid), or aqueous humor (hydatoid). The body fluid may be a digestive juice, such as saliva, gastric juice, bile, pancreatic juice, or intestinal juice, or may be sweat, tears, runny nose, urine, semen, vaginal fluid, amniotic fluid, or milk. The body fluid may be an animal body fluid or a human body fluid. In one embodiment of the present disclosure, a body fluid sample may be provided. In the present disclosure, preferably, the body fluid sample may be urine or an extract thereof. In the present disclosure, preferably, the extract of urine may be an extract of urine according to the present disclosure. In one embodiment of the present disclosure, an extract of a body fluid sample, particularly an extract of urine, may be provided. A body fluid (for example, urine) can be derived from a patient having cancer, a patient with a likelihood of having cancer, or a healthy person.

The cancer may be, but is not limited to, one or more cancers selected from solid cancers, hematologic malignancies, and the like. The cancer is, for example, one or more selected from the group consisting of lung cancer, breast cancer, kidney cancer, leukemia, lymphoma, pancreatic cancer, gastric cancer, urothelial carcinoma, thyroid cancer, ovarian cancer, melanoma, liver cancer, bladder cancer, prostate cancer, cervical cancer, colorectal cancer, and endometrial cancer.

The likelihood that a subject has cancer can be evaluated using the microRNA level of a body fluid sample of the subject as an indicator. For example, for a microRNA with a higher expression in a subject having cancer than in a non-cancer subject in any of the data S1 (or Table 3) or Tables 4-1 to 4-15, the likelihood that a subject has cancer can be determined to be high using a microRNA level of a body fluid sample of the subject higher than a predetermined value (hereinafter sometimes referred to as a "threshold") as an indicator. In the data S1 (or Table 3), for example, for a microRNA with higher expression in three subjects having cancer than in any of three non-cancer subjects, the likelihood that a subject has cancer can be determined to be high using a microRNA level of a body fluid sample of the subject higher than a predetermined value (hereinafter sometimes referred to as a "threshold") as an indicator.

Furthermore, for example, for a microRNA with a higher (for example, twice or more, three times or more, four times or more, five times or more, six times or more, seven times or more, eight times or more, nine times or more, or ten times or more) expression in a subject having cancer than in a non-cancer subject in any of the data S1 (or Table 3) or Tables 4-1 to 4-15, the likelihood that a subject has cancer can be determined to be low using a microRNA level of a body fluid sample of the subject lower than a predetermined value (hereinafter sometimes referred to as a "threshold") as an indicator. In the data S1 (or Table 3), for example, for a microRNA with lower (for example, twice or more, three times or more, four times or more, five times or more, six times or more, seven times or more, eight times or more, nine times or more, or ten times or more) expression in three subjects having cancer than in any of three non-cancer subjects, the likelihood that a subject has cancer can be determined to be low using a microRNA level of a body fluid sample of the subject lower than a predetermined value (hereinafter sometimes referred to as a "threshold") as an indicator.

In one embodiment, a microRNA used as an indicator is any one or more in Table 3. In this embodiment, the cancer may be lung cancer. In this embodiment, the cancer may be pancreatic cancer. In this embodiment, the cancer may be liver cancer. In this embodiment, the cancer may be bladder cancer. In this embodiment, the cancer may be prostate cancer. The microRNA is a microRNA with higher expression in a subject having cancer than in a non-cancer subject.

In one embodiment, a microRNA used as an indicator is any one or more in Table 4-1. In this embodiment, the cancer may be lung cancer. The microRNA is a microRNA with higher expression in a subject having cancer than in a non-cancer subject.

In one embodiment, a microRNA used as an indicator is any one or more in Table 4-2. In this embodiment, the cancer may be breast cancer. The microRNA is a microRNA with higher expression in a subject having cancer than in a non-cancer subject.

In one embodiment, a microRNA used as an indicator is any one or more in Table 4-3. In this embodiment, the cancer may be kidney cancer. The microRNA is a microRNA with higher expression in a subject having cancer than in a non-cancer subject.

In one embodiment, a microRNA used as an indicator is any one or more in Table 4-4. In this embodiment, the cancer may be leukemia. The microRNA is a microRNA with higher expression in a subject having cancer than in a non-cancer subject.

In one embodiment, a microRNA used as an indicator is any one or more in Table 4-5. In this embodiment, the cancer may be lymphoma. The microRNA is a microRNA with higher expression in a subject having cancer than in a non-cancer subject.

In one embodiment, a microRNA used as an indicator is any one or more in Table 4-6. In this embodiment, the cancer may be pancreatic cancer. The microRNA is a microRNA with higher expression in a subject having cancer than in a non-cancer subject.

In one embodiment, a microRNA used as an indicator is any one or more in Table 4-7. In this embodiment, the cancer may be prostate cancer. The microRNA is a microRNA with higher expression in a subject having cancer than in a non-cancer subject.

In one embodiment, a microRNA used as an indicator is any one or more in Table 4-8. In this embodiment, the cancer may be gastric cancer. The microRNA is a microRNA with higher expression in a subject having cancer than in a non-cancer subject.

In one embodiment, a microRNA used as an indicator is any one or more in Table 4-9. In this embodiment, the cancer may be urothelial carcinoma. The microRNA is a microRNA with higher expression in a subject having cancer than in a non-cancer subject.

In one embodiment, a microRNA used as an indicator is any one or more in Table 4-10. In this embodiment, the cancer may be melanoma. The microRNA is a microRNA with higher expression in a subject having cancer than in a non-cancer subject.

In one embodiment, a microRNA used as an indicator is any one or more in Table 4-11. In this embodiment, the cancer may be ovarian cancer. The microRNA is a microRNA with higher expression in a subject having cancer than in a non-cancer subject.

In one embodiment, a microRNA used as an indicator is any one or more in Table 4-12. In this embodiment, the cancer may be thyroid cancer. The microRNA is a microRNA with higher expression in a subject having cancer than in a non-cancer subject.

In one embodiment, a microRNA used as an indicator is any one or more in Table 4-13. In this embodiment, the cancer may be cervical cancer. The microRNA is a microRNA with higher expression in a subject having cancer than in a non-cancer subject.

In one embodiment, a microRNA used as an indicator is any one or more in Table 4-14. In this embodiment, the cancer may be colorectal cancer. The microRNA is a microRNA with higher expression in a subject having cancer than in a non-cancer subject.

In one embodiment, a microRNA used as an indicator is any one or more in Table 4-15. In this embodiment, the cancer may be endometrial cancer. The microRNA is a microRNA with higher expression in a subject having cancer than in a non-cancer subject.

In one embodiment, a microRNA used as an indicator is a microRNA having a p-value of less than 0.005 in any of Tables 4-1 to 4-15. The microRNA is a microRNA with higher expression in a subject having cancer than in a non-cancer subject.

In one embodiment, a microRNA used as an indicator is a microRNA listed in any of Tables 22-1 and 23-1 to 23-14. The microRNA is a microRNA with higher expression in a subject having cancer than in a non-cancer subject.

In one embodiment, a microRNA used as an indicator is a microRNA listed in any of Tables 24-1 to 24-15. The microRNA is a microRNA with higher expression in a subject having cancer than in a non-cancer subject.

In one embodiment, a microRNA used as an indicator is a microRNA listed in any of Tables 25-1 to 25-15. The microRNA is a microRNA with higher expression in a subject having cancer than in a non-cancer subject.

In one embodiment, a microRNA used as an indicator is any one or more of the group (ALL). In this embodiment, the cancer may be endometrial cancer. The microRNA is a microRNA with higher expression in a subject having cancer than in a non-cancer subject.

In one embodiment, a microRNA used as an indicator is any one or more of the group (GI). In this embodiment, the cancer may be endometrial cancer. The microRNA is a microRNA with higher expression in a subject having cancer than in a non-cancer subject.

In one embodiment, a microRNA used as an indicator is any one or more of the group (Hemo). In this embodiment, the cancer may be endometrial cancer. The microRNA is a microRNA with higher expression in a subject having cancer than in a non-cancer subject.

In one embodiment, a microRNA used as an indicator is any one or more of the group (Uro). In this embodiment, the cancer may be endometrial cancer. The microRNA is a microRNA with higher expression in a subject having cancer than in a non-cancer subject.

The number of types of microRNAs used as indicators may be 0 or more types, 1 or more types, 2 or more types, 3 or more types, 4 or more types, 5 or more types, 10 or more types, 15 or more types, 20 or more types, 30 or more types, 40 or more types, 50 or more types, 60 or more types, 70 or more types, 80 or more types, 100 or more types, 200 or more types, 300 or more types, 400 or more types, 500 or more types, 600 or more types, 700 or more types, 800 or more types, 900 or more types, 1000 or more types, 2000 or more types, or 3000 or more types, or all in each table (provided that at least one microRNA in any one of the tables is used as an indicator). The number of types of microRNAs used as indicators may be more than 50%, 60% or more, 70% or more, 80% or more, 90% or more, or all of the number of types of microRNAs listed in each table.

In these cases, the predetermined value may be, but is not limited to, any value between two values (statistics or indicator values) selected from the group consisting of the average value, median, third quartile value, first quartile value, and minimum value of the microRNA level in a subject group having cancer. For example, the predetermined value may be, but is not limited to, any value between two values selected from the group consisting of the maximum value, third quartile value, average value, median, and first quartile value of the microRNA level in a non-cancer subject group. Furthermore, the predetermined value may be any value (for example, an intermediate value) between an average value in a control group having cancer and an average value in a healthy person. The predetermined value may be a first threshold. The first threshold is set for each target microRNA. The predetermined value may be a second threshold. The second threshold is set for each target microRNA.

In the present disclosure, among the microRNAs in a body fluid sample of a subject, the type of microRNA to be measured and /or the type of microRNA to be used as an indicator of the likelihood of cancer can be, for example, 1 or more, 2 or more, 3 or more, 4 or more, 5 or more, 6 or more, 7 or more, 8 or more, 9 or more, 10 or more, 20 or more, 30 or more, 40 or more, 50 or more, 60 or more, 70 or more, 80 or more, 90 or more, 100 or more, 200 or more, 300 or more, 400 or more, 500 or more, 600 or more, 700 or more, 800 or more, 900 or more, 1000 or more, 1100 or more, 1200 or more, 1300 or more, 1400 or more, 1500 or more, 1600 or more, 1700 or more, 1800 or more, 1900 or more, 2000 or more, 2100 or more, 2200 or more, 2300 or more, 2400 or more, or 2500 or more. Among the microRNAs in a body fluid sample of a subject, the type of microRNA to be measured and /or the type of microRNA to be used as an indicator of the likelihood of cancer can be, for example, 2500 or less, 2400 or less, 2300 or less, 2200 or less, 2100 or less, 2000 or less, 1900 or less, 1800 or less, 1700 or less, 1600 or less, 1500 or less, 1400 or less, 1300 or less, 1200 or less, 1100 or less, 1000 or less, 900 or less, 800 or less, 700 or less, 600 or less, 500 or less, 400 or less, 300 or less, 200 or less, 100 or less, 90 or less, 80 or less, 70 or less, 60 or less, 50 or less, 40 or less, 30 or less, 20 or less, or 10 or less. A method of using a microRNA as an indicator of the likelihood of cancer is disclosed in the present description. A microarray may be used to measure or detect a microRNA.

For example, for a microRNA with a lower expression in a subject having cancer than in a non-cancer subject in any of the data S1 (or Table 3) or Tables 4-1 to 4-15, the likelihood that a subject has cancer can be determined to be high using a microRNA level of a body fluid sample of the subject lower than a predetermined value as an indicator. In the data S1 (or Table 3), for example, for a microRNA with lower (for example, twice or more, three times or more, four times or more, five times or more, six times or more, seven times or more, eight times or more, nine times or more, or ten times or more) expression in three subjects having cancer than in any of three non-cancer subjects, the likelihood that a subject has cancer can be determined to be high using a microRNA level of a body fluid sample of the subject lower than a predetermined value (hereinafter sometimes referred to as a "threshold") as an indicator.

For example, for a microRNA with a lower expression in a subject having cancer than in a non-cancer subject in any of the data S1 (or Table 3) or Tables 4-1 to 4-15, the likelihood that a subject has cancer can be determined to be low using a microRNA level of a body fluid sample of the subject higher than a predetermined value as an indicator. In the data S1 (or Table 3), for example, for a microRNA with lower (for example, twice or more, three times or more, four times or more, five times or more, six times or more, seven times or more, eight times or more, nine times or more, or ten times or more) expression in three subjects having cancer than in any of three non-cancer subjects, the likelihood that a subject has cancer can be determined to be low using a microRNA level of a body fluid sample of the subject higher than a predetermined value (hereinafter sometimes referred to as a "threshold") as an indicator.

In one embodiment, a microRNA used as an indicator is any one or more in Table 3. In this embodiment, the cancer may be lung cancer. In this embodiment, the cancer may be pancreatic cancer. In this embodiment, the cancer may be liver cancer. In this embodiment, the cancer may be bladder cancer. In this embodiment, the cancer may be prostate cancer. The microRNA is a microRNA with lower expression in a subject having cancer than in a non-cancer subject.

In one embodiment, a microRNA used as an indicator is any one or more in Table 4-1. In this embodiment, the cancer may be lung cancer. The microRNA is a microRNA with lower expression in a subject having cancer than in a non-cancer subject.

In one embodiment, a microRNA used as an indicator is any one or more in Table 4-2. In this embodiment, the cancer may be breast cancer. The microRNA is a microRNA with lower expression in a subject having cancer than in a non-cancer subject.

In one embodiment, a microRNA used as an indicator is any one or more in Table 4-3. In this embodiment, the cancer may be kidney cancer. The microRNA is a microRNA with lower expression in a subject having cancer than in a non-cancer subject.

In one embodiment, a microRNA used as an indicator is any one or more in Table 4-4. In this embodiment, the cancer may be leukemia. The microRNA is a microRNA with lower expression in a subject having cancer than in a non-cancer subject.

In one embodiment, a microRNA used as an indicator is any one or more in Table 4-5. In this embodiment, the cancer may be lymphoma. The microRNA is a microRNA with lower expression in a subject having cancer than in a non-cancer subject.

In one embodiment, a microRNA used as an indicator is any one or more in Table 4-6. In this embodiment, the cancer may be pancreatic cancer. The microRNA is a microRNA with lower expression in a subject having cancer than in a non-cancer subject.

In one embodiment, a microRNA used as an indicator is any one or more in Table 4-7. In this embodiment, the cancer may be prostate cancer. The microRNA is a microRNA with lower expression in a subject having cancer than in a non-cancer subject.

In one embodiment, a microRNA used as an indicator is any one or more in Table 4-8. In this embodiment, the cancer may be gastric cancer. The microRNA is a microRNA with lower expression in a subject having cancer than in a non-cancer subject.

In one embodiment, a microRNA used as an indicator is any one or more in Table 4-9. In this embodiment, the cancer may be urothelial carcinoma. The microRNA is a microRNA with lower expression in a subject having cancer than in a non-cancer subject.

In one embodiment, a microRNA used as an indicator is any one or more in Table 4-10. In this embodiment, the cancer may be melanoma. The microRNA is a microRNA with lower expression in a subject having cancer than in a non-cancer subject.

In one embodiment, a microRNA used as an indicator is any one or more in Table 4-11. In this embodiment, the cancer may be ovarian cancer. The microRNA is a microRNA with lower expression in a subject having cancer than in a non-cancer subject.

In one embodiment, a microRNA used as an indicator is any one or more in Table 4-12. In this embodiment, the cancer may be thyroid cancer. The microRNA is a microRNA with lower expression in a subject having cancer than in a non-cancer subject.

In one embodiment, a microRNA used as an indicator is any one or more in Table 4-13. In this embodiment, the cancer may be cervical cancer. The microRNA is a microRNA with lower expression in a subject having cancer than in a non-cancer subject.

In one embodiment, a microRNA used as an indicator is any one or more in Table 4-14. In this embodiment, the cancer may be colorectal cancer. The microRNA is a microRNA with lower expression in a subject having cancer than in a non-cancer subject.

In one embodiment, a microRNA used as an indicator is any one or more in Table 4-15. In this embodiment, the cancer may be endometrial cancer. The microRNA is a microRNA with lower expression in a subject having cancer than in a non-cancer subject.

In one embodiment, a microRNA used as an indicator is a microRNA having a p-value of less than 0.005 in any of Tables 4-1 to 4-15. The microRNA is a microRNA with lower expression in a subject having cancer than in a non-cancer subject.

In one embodiment, a microRNA used as an indicator is a microRNA listed in any of Tables 22-1 and 23-1 to 23-14. The microRNA is a microRNA with lower expression in a subject having cancer than in a non-cancer subject.

In one embodiment, a microRNA used as an indicator is a microRNA listed in any of Tables 24-1 to 24-15. The microRNA is a microRNA with lower expression in a subject having cancer than in a non-cancer subject.

In one embodiment, a microRNA used as an indicator is a microRNA listed in any of Tables 25-1 to 25-15. The microRNA is a microRNA with lower expression in a subject having cancer than in a non-cancer subject.

In one embodiment, a microRNA used as an indicator is any one or more of the group (ALL). In this embodiment, the cancer may be endometrial cancer. The microRNA is a microRNA with lower expression in a subject having cancer than in a non-cancer subject.

In one embodiment, a microRNA used as an indicator is any one or more of the group (GI). In this embodiment, the cancer may be endometrial cancer. The microRNA is a microRNA with lower expression in a subject having cancer than in a non-cancer subject.

In one embodiment, a microRNA used as an indicator is any one or more of the group (Hemo). In this embodiment, the cancer may be endometrial cancer. The microRNA is a microRNA with lower expression in a subject having cancer than in a non-cancer subject.

In one embodiment, a microRNA used as an indicator is any one or more of the group (Uro). In this embodiment, the cancer may be endometrial cancer. The microRNA is a microRNA with lower expression in a subject having cancer than in a non-cancer subject.

The number of types of microRNAs used as indicators may be 0 or more types, 1 or more types, 2 or more types, 3 or more types, 4 or more types, 5 or more types, 10 or more types, 15 or more types, 20 or more types, 30 or more types, 40 or more types, 50 or more types, 60 or more types, 70 or more types, 80 or more types, 100 or more types, 200 or more types, 300 or more types, 400 or more types, 500 or more types, 600 or more types, 700 or more types, 800 or more types, 900 or more types, 1000 or more types, 2000 or more types, or 3000 or more types, or all in each table (provided that at least one microRNA in at least one table is used as an indicator). The number of types of microRNAs used as indicators may be more than 50%, 60% or more, 70% or more, 80% or more, 90% or more, or all of the number of types of microRNAs listed in each table.

In these cases, the predetermined value may be, but is not limited to, any value between two values selected from the group consisting of the average value, median, third quartile value, first quartile value, and minimum value of the microRNA level in a subject group having cancer. The predetermined value may be determined for each type of cancer. For example, the predetermined value may be, but is not limited to, any value between two values selected from the group consisting of the maximum value, third quartile value, average value, median, and first quartile value of the microRNA level in a non-cancer subject group.

The present disclosure provides a method for examining the likelihood that a subject has lung cancer. According to the present disclosure, a method for examining the likelihood that a subject has lung cancer can be used to examine the likelihood that the subject has lung cancer using any one or more microRNA levels selected from the data S1 (or Table 3) in a body fluid sample of the subject as an indicator. According to the present disclosure, the likelihood that a subject has lung cancer can be examined using at least one or all microRNA levels selected from the group consisting of miR-3117-5p, miR-3118, miR-3121-3p, miR-3121-5p, miR-3126-5p, miR-3128, miR-3133, miR-3134, miR-3136-3p, miR-3136-5p, miR-3139, miR-3142, miR-3143, miR-3145-3p, miR-3163, miR-3166, miR-3167, miR-16-1-3p, miR-424-3p, miR-519c-5p, miR-525-5p, miR-551b-5p, miR-558, miR-921, miR-942-3p, miR-3126-3p, miR-3127-5p, miR-3129-5p, miR-3144-5p, miR-3150a-5p, miR-3152-5p, miR-3155a, miR-3157-3p, miR-3159, miR-3165, miR-3678-3p, miR-4321, miR-4521, miR-4800-3p, miR-4999-5p, miR-5096, miR-5187-5p, miR-6874-5p, miR-3127-3p, miR-3130-5p, miR-3131, miR-3141, miR-3150b-5p, miR-3151-3p, miR-3151-5p, miR-3154, miR-3160-3p, miR-3160-5p, miR-378a-5p, miR-520c-3p, miR-526b-3p, miR-3150a-3p, miR-3162-5p, and miR-4254 in a body fluid sample of the subject as an indicator.

According to the present disclosure, the likelihood that a subject has lung cancer can also be examined using at least one or all microRNA levels selected from the group consisting of miR-3163, miR-16-1-3p, miR-424-3p, miR-558, miR-3127-5p, and miR-4521 in a body fluid sample of the subject as an indicator. If at least one or all microRNA levels selected from the group consisting of miR-3163, miR-16-1-3p, miR-424-3p, miR-558, miR-3127-5p, and miR-4521 in a body fluid sample are higher than a predetermined value, it may be determined that there is a likelihood that the subject has lung cancer ( and /or if the levels are lower than the predetermined value, it may be determined that there is a likelihood that the subject does not have lung cancer).

According to the present disclosure, the likelihood that a subject has lung cancer can also be examined using at least one or all microRNA levels selected from the group consisting of miR-378a-5p, miR-520c-3p, and miR-526b-3p in a body fluid sample of the subject as an indicator. If at least one or all microRNAs selected from the group consisting of miR-378a-5p, miR-520c-3p, and miR-526b-3p in a body fluid sample are lower than a predetermined value, it may be determined that there is a likelihood that the subject has lung cancer ( and /or if the levels are higher than the predetermined value, it may be determined that there is a likelihood that the subject does not have lung cancer).

According to the present disclosure, a microRNA in urine to be used as an indicator of lung cancer may be at least one or all selected from the group consisting of miR-3127-3p, miR-3130-5p, miR-3131, miR-3141, miR-3150b-5p, miR-3151-3p, miR-3151-5p, miR-3154, miR-3160-3p, and miR-3160-5p.

According to the present disclosure, a microRNA in urine to be used as an indicator of lung cancer may be at least one or all selected from the group consisting of miR-3117-5p, miR-3118, miR-3121-3p, miR-3121-5p, miR-3126-5p, miR-3128, miR-3133, miR-3134, miR-3136-3p, miR-3136-5p, miR-3139, miR-3142, miR-3143, miR-3145-3p, miR-3163, miR-3166, and miR-3167.

The present disclosure provides a method for examining the likelihood that a subject has pancreatic cancer. According to the present disclosure, a method for examining the likelihood that a subject has pancreatic cancer can be used to examine the likelihood that the subject has pancreatic cancer using any one or more microRNA levels selected from the data S1 (or Table 3) in a body fluid sample of the subject as an indicator. According to the present disclosure, the likelihood that a subject has pancreatic cancer can be examined using at least one or all microRNA levels selected from the group consisting of let-7i-3p, miR-183-5p, miR-202-5p, miR-409-5p, miR-4661-5p, miR-4800-3p, miR-5587-5p, miR-372-3p, miR-378b, miR-520b, miR-1266-3p, miR-3605-5p, miR-3612, miR-4645-3p, miR-4694-3p, miR-4752, miR-6816-3p, miR-8087, let-7f-2-3p, miR-15a-3p, miR-20a-3p, miR-33b-3p, miR-34c-5p, miR-93-5p, miR-130a-5p, miR-135a-5p, miR-135b-5p, miR-185-5p, miR-203a-3p, miR-302d-5p, miR-337-3p, miR-378c, miR-422a, miR-449c-5p, miR-483-5p, miR-506-3p, miR-511-5p, miR-520c-3p, miR-654-3p, miR-668-5p, miR-670-5p, miR-671-3p, miR-744-3p, miR-1178-3p, miR-1254, miR-1284, miR-1323, miR-2116-5p, miR-2355-3p, miR-3132, miR-3138, miR-3164, miR-3186-3p, miR-3189-3p, miR-3198, miR-3200-5p, miR-3657, miR-3667-5p, miR-3680-5p, miR-3692-5p, miR-3713, miR-3921, miR-3936, miR-4273, miR-4299, miR-4306, miR-4316, miR-4319, miR-4421, miR-4429, miR-4435, miR-4441, miR-4473, miR-4506, miR-4633-5p, miR-4658, miR-4733-5p, miR-4733-3p, miR-5004-3p, miR-5194, miR-5197-5p, miR-5571-5p, miR-6083, miR-6717-5p, miR-6720-5p, miR-6767-3p, miR-6781-3p, miR-6811-3p, miR-6821-3p, miR-6828-5p, miR-6832-5p, miR-6837-3p, miR-6841-5p, miR-6853-5p, miR-6871-3p, miR-6875-5p, miR-6878-5p, miR-7112-3p, miR-7703, miR-7848-3p, and miR-7856-5p in a body fluid sample of the subject as an indicator.

According to the present disclosure, the likelihood that a subject has pancreatic cancer can also be examined using at least one or all microRNA levels selected from the group consisting of miR-183-5p, miR-202-5p, and miR-409-5p in a body fluid sample of the subject as an indicator. If at least one or all microRNA levels selected from the group consisting of miR-183-5p, miR-202-5p, and miR-409-5p in a body fluid sample are higher than a predetermined value, it may be determined that there is a likelihood that the subject has pancreatic cancer ( and /or if the levels are lower than the predetermined value, it may be determined that there is a likelihood that the subject does not have pancreatic cancer).

According to the present disclosure, the likelihood that a subject has pancreatic cancer can also be examined using at least one or all microRNA levels selected from the group consisting of miR-372-3p, miR-520b, miR-15a-3p, miR-34c-5p, miR-135a-5p, miR-185-5p, miR-337-3p, miR-422a, miR-506-3p, miR-520c-3p, miR-1284, miR-1323, and miR-4273 as an indicator. If at least one or all microRNA levels selected from the group consisting of miR-372-3p, miR-520b, miR-15a-3p, miR-34c-5p, miR-135a-5p, miR-185-5p, miR-337-3p, miR-422a, miR-506-3p, miR-520c-3p, miR-1284, miR-1323, and miR-4273 in a body fluid sample are lower than a predetermined value, it may be determined that there is a likelihood that the subject has pancreatic cancer ( and /or if the levels are higher than the predetermined value, it may be determined that there is a likelihood that the subject does not have pancreatic cancer).

According to the present disclosure, a microRNA in urine to be used as an indicator of pancreatic cancer may also be at least one or all selected from the group consisting of miR-372-3p, miR-378b, miR-520b, miR-1266-3p, miR-3605-5p, miR-3612, miR-4645-3p, miR-4694-3p, miR-4752, miR-6816-3p, and miR-8087.

The present disclosure provides a method for examining the likelihood that a subject has liver cancer. According to the present disclosure, a method for examining the likelihood that a subject has liver cancer can be used to examine the likelihood that the subject has liver cancer using any one or more microRNA levels selected from the data S1 (or Table 3) in a body fluid sample of the subject as an indicator. According to the present disclosure, the likelihood that a subject has liver cancer can be examined using at least one or all microRNA levels selected from the group consisting of miR-4521, let-7c-3p, let-7i-5p, miR-16-1-3p, miR-26a-1-3p, miR-28-5p, miR-105-5p, miR-195-3p, miR-200b-5p, miR-219a-2-3p, miR-297, miR-300, miR-330-3p, miR-374b-5p, miR-431-5p, miR-454-5p, miR-513c-5p, miR-548ax, miR-593-5p, miR-623, miR-664a-5p, miR-942-3p, miR-1205, miR-1276, miR-1288-3p, miR-1297, miR-3678-3p, miR-4283, miR-4295, miR-4439, miR-4524b-5p, miR-4703-3p, miR-4768-5p, miR-4800-3p, miR-5187-5p, miR-5696, miR-7161-5p, let-7i-2-3p, and miR-520c-3p as an indicator. According to the present disclosure, the likelihood that a subject has liver cancer can be examined using at least one or all microRNA levels selected from the group consisting of miR-4521, let-7c-3p, let-7i-5p, miR-16-1-3p, miR-26a-1-3p, miR-28-5p, miR-105-5p, miR-195-3p, miR-200b-5p, miR-219a-2-3p, miR-297, miR-300, miR-330-3p, miR-374b-5p, miR-431-5p, miR-454-5p, miR-513c-5p, miR-548ax, miR-593-5p, miR-623, miR-664a-5p, miR-942-3p, miR-1205, miR-1276, miR-1288-3p, miR-1297, miR-3678-3p, miR-4283, miR-4295, miR-4439, miR-4524b-5p, miR-4703-3p, miR-4768-5p, miR-4800-3p, miR-5187-5p, miR-5696, miR-7161-5p, let-7i-2-3p, and miR-520c-3p as an indicator. If at least one or all microRNA levels selected from the group consisting of miR-16-1-3p, miR-28-5p, miR-297, miR-300, miR-330-3p, miR-454-5p, miR-1297, and miR-4295 in a body fluid sample are higher than a predetermined value, it may be determined that there is a likelihood that the subject has liver cancer ( and /or if the levels are lower than the predetermined value, it may be determined that there is a likelihood that the subject does not have liver cancer).

In the present disclosure, the likelihood that a subject has liver cancer can be examined using a miR-520c-3p level as an indicator. If the miR-520c-3p level in a body fluid sample is lower than a predetermined value, it may be determined that there is a likelihood that the subject has liver cancer ( and /or if the levels are higher than the predetermined value, it may be determined that there is a likelihood that the subject does not have liver cancer).

According to the present disclosure, a microRNA in urine to be used as an indicator of liver cancer may also be miR-4521.

The present disclosure provides a method for examining the likelihood that a subject has bladder cancer. According to the present disclosure, a method for examining the likelihood that a subject has bladder cancer can be used to examine the likelihood that the subject has bladder cancer using any one or more microRNA levels selected from the data S1 (or Table 3) in a body fluid sample of the subject as an indicator. According to the present disclosure, the likelihood that a subject has bladder cancer can be examined using at least one or all microRNA levels selected from the group consisting of miR-16-1-3p, miR-23b-3p, miR-28-5p, miR-92a-2-5p, miR-142-3p, miR-195-3p, miR-196b-5p, miR-299-3p, miR-492, miR-513b-5p, miR-601, miR-619-5p, miR-1285-3p, miR-3155a, miR-3162-5p, miR-3678-3p, miR-4283, miR-4295, miR-4311, miR-4531, miR-5096, miR-5187-5p, let-7f-2-3p, miR-520c-3p, and miR-4783-5p as an indicator.

In the present disclosure, the likelihood that a subject has bladder cancer can be examined using at least one or all microRNA levels selected from the group consisting of miR-16-1-3p, miR-23b-3p, miR-28-5p, miR-142-3p, miR-195-3p, miR-299-3p, and miR-4295 as an indicator. If at least one or all microRNA levels selected from the group consisting of miR-16-1-3p, miR-23b-3p, miR-28-5p, miR-142-3p, miR-195-3p, miR-299-3p, and miR-4295 in a body fluid sample are higher than a predetermined value, it may be determined that there is a likelihood that the subject has bladder cancer ( and /or if the levels are lower than the predetermined value, it may be determined that there is a likelihood that the subject does not have bladder cancer).

In the present disclosure, the likelihood that a subject has bladder cancer can be examined using a miR-520c-3p level as an indicator. If the miR-520c-3p level in a body fluid sample is lower than a predetermined value, it may be determined that there is a likelihood that the subject has bladder cancer ( and /or if the levels are higher than the predetermined value, it may be determined that there is a likelihood that the subject does not have bladder cancer).

The present disclosure provides a method for examining the likelihood that a subject has prostate cancer. According to the present disclosure, a method for examining the likelihood that a subject has prostate cancer can be used to examine the likelihood that the subject has prostate cancer using any one or more microRNA levels selected from the data S1 (or Table 3) in a body fluid sample of the subject as an indicator. According to the present disclosure, the likelihood that a subject has prostate cancer can be examined using at least one or all microRNA levels selected from the group consisting of miR-4531, miR-28-5p, miR-103a-2-5p, miR-105-5p, miR-124-3p, miR-151a-5p, miR-151b, miR-200a-5p, miR-300, miR-424-3p, miR-519c-5p, miR-551b-5p, miR-617, miR-873-3p, miR-921, miR-1288-3p, miR-3124-5p, miR-3155a, miR-3917, miR-4283, miR-4727-3p, miR-5096, miR-5187-5p, miR-6074, miR-6874-5p, miR-6892-5p, miR-15a-3p, miR-135b-5p, miR-520c-3p, miR-4783-5p, and miR-7849-3p as an indicator. In the present disclosure, the likelihood that a subject has prostate cancer can be examined using at least one or all microRNA levels selected from the group consisting of miR-28-5p, miR-105-5p, miR-124-3p, miR-151a-5p, and miR-300 as an indicator. If at least one or all microRNA levels selected from the group consisting of miR-28-5p, miR-105-5p, miR-124-3p, miR-151a-5p, and miR-300 in a body fluid sample are higher than a predetermined value, it may be determined that there is a likelihood that the subject has prostate cancer ( and /or if the levels are lower than the predetermined value, it may be determined that there is a likelihood that the subject does not have prostate cancer).

In the present disclosure, the likelihood that a subject has liver cancer can be examined using at least one or all microRNA levels selected from the group consisting of miR-15a-3p and miR-520c-3p as an indicator. If at least one or all microRNA levels selected from the group consisting of miR-15a-3p and miR-520c-3p in a body fluid sample are lower than a predetermined value, it may be determined that there is a likelihood that the subject has prostate cancer (and /or if the levels are higher than the predetermined value, it may be determined that there is a likelihood that the subject does not have prostate cancer).

According to the present disclosure, a microRNA in urine to be used as an indicator of prostate cancer may also be miR-4531.

For example, for a microRNA with a lower expression in a subject having cancer than in a non-cancer subject in Tables 4-1 to 4-15, the likelihood that a subject has cancer can be determined to be high using a microRNA level of a body fluid sample of the subject lower than a predetermined value (hereinafter sometimes referred to as a "threshold") as an indicator.

For example, for a microRNA with a lower expression in a subject having cancer than in a non-cancer subject in Tables 4-1 to 4-15, the likelihood that a subject has cancer can be determined to be low using a microRNA level of a body fluid sample of the subject higher than a predetermined value as an indicator.

The predetermined value may be, but is not limited to, any value between two values selected from the group consisting of the average value, median, third quartile value, first quartile value, and minimum value of the microRNA level in a subject group having cancer. For example, the predetermined value may be, but is not limited to, any value between two values selected from the group consisting of the maximum value, third quartile value, average value, median, and first quartile value of the microRNA level in a non-cancer subject group. Furthermore, the predetermined value may be any value (for example, an intermediate value) between an average value in a control group having cancer and an average value in a healthy person.

The present disclosure provides a method for examining the likelihood that a subject has lung cancer. According to the present disclosure, a method for examining the likelihood that a subject has lung cancer can be used to examine the likelihood that the subject has lung cancer using any one or more microRNA levels selected from Table 4-1 in a body fluid sample of the subject as an indicator. According to the present disclosure, the likelihood that a subject has lung cancer can be examined using at least 1, 2, 3, 4, 5, 6, 7, 8, 9, 10 or more, 15 or more, or 20 or more, or all microRNA levels selected from the group consisting of hsa-miR-4443, hsa-miR-4515, hsa-miR-4743-5p, hsa-miR-1908-3p, hsa-miR-4314, hsa-miR-296-3p, hsa-miR-6772-5p, hsa-miR-370-3p, hsa-miR-4708-3p, hsa-miR-4499, hsa-miR-6759-5p, hsa-miR-3160-3p, hsa-miR-219a-2-3p, hsa-miR-564, hsa-miR-4269, hsa-let-7b-5p, hsa-miR-4535, hsa-miR-187-3p, hsa-miR-5588-3p, hsa-miR-194-3p, hsa-miR-3153, hsa-miR-3074-5p, hsa-miR-4721, hsa-miR-173h-5p, hsa-miR-1471, hsa-miR-936, hsa-miR-622, hsa-miR-3622a-5p, hsa-miR-4252, hsa-miR-4533, hsa-miR-3917, hsa-miR-520d-5p, hsa-miR-4639-3p, hsa-miR-4496, hsa-miR-3156-5p, hsa-miR-3689b-3p, hsa-miR-3689c, hsa-miR-4669, hsa-miR-6072, hsa-miR-6751-5p, hsa-miR-378b, hsa-miR-520b, hsa-miR-1307-3p, hsa-miR-8075, hsa-miR-4448, hsa-miR-4487, hsa-miR-5002-3p, hsa-miR-4518, hsa-miR-4769-5p, hsa-miR-4724-5p, hsa-miR-397-5p, hsa-miR-494-5p, hsa-miR-6884-5p, hsa-miR-6796-5p, hsa-miR-181b-3p, hsa-miR-526b-5p, hsa-miR-1226-5p, hsa-miR-6776-5p, hsa-miR-4529-3p, hsa-miR-7851-3p, hsa-miR-3189-3p, hsa-miR-5010-5p, hsa-miR-4638-5p, hsa-miR-3177-3p, hsa-miR-6872-5p, hsa-miR-6745, hsa-miR-6871-5p, hsa-miR-138-1-3p, hsa-miR-4539, hsa-miR-3652, hsa-miR-505-5p, hsa-miR-173g-3p, hsa-miR-4701-3p, hsa-miR-3160-5p, hsa-miR-6815-5p, hsa-miR-6499-5p, hsa-miR-5589-5p, hsa-miR-4489, hsa-miR-212-5p, hsa-miR-5698, hsa-miR-3124-5p, hsa-miR-3176, hsa-miR-3612, hsa-miR-6849-5p, hsa-miR-6760-5p, hsa-miR-6833-5p, hsa-miR-8077, hsa-miR-277-3p, hsa-miR-7975, hsa-miR-6859-5p, hsa-miR-3187-5p, hsa-miR-3145-5p, hsa-miR-3689a-3p, hsa-miR-4800-5p, hsa-miR-1224-5p, hsa-miR-193a-5p, hsa-miR-6076, hsa-miR-4260, hsa-miR-298, hsa-miR-34a-5p, hsa-miR-6131, hsa-miR-5708, hsa-miR-4776-3p, hsa-miR-378e, hsa-miR-3659, hsa-miR-3120-5p, hsa-miR-3122, hsa-miR-3177-5p, hsa-miR-4420, hsa-miR-323a-5p, hsa-miR-466, hsa-miR-276-3p, hsa-miR-3138, hsa-miR-6834-5p, hsa-miR-185-5p, hsa-miR-2467-3p, hsa-miR-6847-5p, hsa-miR-608, hsa-miR-6504-5p, hsa-miR-3922-5p, hsa-miR-877-5p, hsa-miR-4307, hsa-miR-4512, hsa-miR-6892-5p, hsa-miR-4776-5p, hsa-miR-3065-5p, hsa-miR-6129, hsa-miR-4676-5p, hsa-miR-603, hsa-miR-6876-5p, hsa-miR-4635, hsa-miR-4525, hsa-miR-1468-5p, hsa-miR-519c-5p, hsa-miR-523-5p, hsa-miR-518e-5p, hsa-miR-522-5p, hsa-miR-519a-5p, hsa-miR-519b-5p, hsa-miR-4711-3p, hsa-miR-1293, hsa-miR-6068, hsa-miR-1254, hsa-miR-4306, hsa-miR-3186-3p, hsa-miR-4691-5p, hsa-miR-1321, hsa-miR-4540, hsa-miR-6823-5p, hsa-miR-3935, hsa-miR-5006-5p, hsa-miR-4756-3p, hsa-miR-4785, hsa-miR-8082, hsa-miR-6788-5p, hsa-miR-6500-3p, hsa-miR-5189-5p, hsa-miR-7151-3p, hsa-miR-891a-5p, hsa-miR-3126-5p, hsa-miR-8083, hsa-miR-4428, hsa-miR-4474-3p, hsa-miR-7850-5p, hsa-miR-4700-5p, hsa-miR-6828-5p, hsa-miR-668-5p, hsa-miR-3934-5p, hsa-miR-4654, hsa-miR-4436a, hsa-miR-173f, hsa-miR-3655, hsa-miR-650, hsa-miR-3137, hsa-miR-4285, hsa-miR-171-5p, hsa-miR-3064-3p, hsa-miR-6801-5p, hsa-miR-6817-5p, hsa-miR-4686, hsa-miR-173g-5p, hsa-miR-6807-3p, hsa-miR-6747-5p, hsa-miR-5580-5p, hsa-miR-4796-5p, hsa-miR-3174, hsa-miR-7157-5p, hsa-miR-614, hsa-miR-4502, hsa-miR-3164, hsa-miR-4647, hsa-miR-378i, hsa-miR-6515-5p, hsa-miR-656-5p, hsa-miR-449c-5p, hsa-miR-3591-3p, hsa-miR-424-3p, hsa-miR-3149, hsa-miR-6868-5p, hsa-miR-173d, hsa-miR-4644, hsa-miR-4754, hsa-miR-477-3p, hsa-miR-4445-3p, hsa-miR-4436b-3p, hsa-miR-4262, hsa-miR-1306-3p, hsa-miR-4514, hsa-miR-4296, hsa-miR-4458, hsa-miR-4520-5p, hsa-miR-766-5p, hsa-miR-548ao-3p, hsa-miR-758-5p, hsa-miR-4451, hsa-miR-4300, hsa-miR-4453, hsa-miR-4784, hsa-miR-6809-5p, hsa-miR-4692, hsa-miR-4437, hsa-miR-4681, hsa-miR-921, hsa-miR-1250-5p, hsa-miR-5093, hsa-miR-551b-5p, hsa-miR-4529-5p, hsa-miR-4538, hsa-miR-176, hsa-miR-1322, hsa-miR-473, hsa-miR-106a-5p, hsa-miR-297, hsa-miR-1910-3p, hsa-miR-4470, hsa-miR-3691-5p, hsa-miR-4999-5p, hsa-miR-3192-5p, hsa-miR-548au-3p, hsa-miR-5007-5p, hsa-miR-4657, hsa-miR-4768-3p, hsa-miR-1265, hsa-miR-4321, hsa-miR-4423-5p, hsa-miR-1294, and hsa-miR-4520-3p in a body fluid sample of the subject as an indicator.

According to the present disclosure, the likelihood that a subject has lung cancer can also be examined using at least 1, 2, 3, 4, 5, 6, 7, 8, 9, 10 or more, 15 or more, or 20 or more, or all microRNA levels selected from the group consisting of hsa-miR-6788-5p, hsa-miR-6500-3p, hsa-miR-5189-5p, hsa-miR-7151-3p, hsa-miR-891a-5p, hsa-miR-3126-5p, hsa-miR-8083, hsa-miR-4428, hsa-miR-4474-3p, hsa-miR-7850-5p, hsa-miR-4700-5p, hsa-miR-6828-5p, hsa-miR-668-5p, hsa-miR-3934-5p, hsa-miR-4654, hsa-miR-4436a, hsa-miR-173f, hsa-miR-3655, hsa-miR-650, hsa-miR-3137, hsa-miR-4285, hsa-miR-171-5p, hsa-miR-3064-3p, hsa-miR-6801-5p, hsa-miR-6817-5p, hsa-miR-4686, hsa-miR-173g-5p, hsa-miR-6807-3p, hsa-miR-6747-5p, hsa-miR-5580-5p, hsa-miR-4796-5p, hsa-miR-3174, hsa-miR-7157-5p, hsa-miR-614, hsa-miR-4502, hsa-miR-3164, hsa-miR-4647, hsa-miR-378i, hsa-miR-6515-5p, hsa-miR-656-5p, hsa-miR-449c-5p, hsa-miR-3591-3p, hsa-miR-424-3p, hsa-miR-3149, hsa-miR-6868-5p, hsa-miR-173d, hsa-miR-4644, hsa-miR-4754, hsa-miR-477-3p, hsa-miR-4445-3p, hsa-miR-4436b-3p, hsa-miR-4262, hsa-miR-1306-3p, hsa-miR-4514, hsa-miR-4296, hsa-miR-4458, hsa-miR-4520-5p, hsa-miR-766-5p, hsa-miR-548ao-3p, hsa-miR-758-5p, hsa-miR-4451, hsa-miR-4300, hsa-miR-4453, hsa-miR-4784, hsa-miR-6809-5p, hsa-miR-4692, hsa-miR-4437, hsa-miR-4681, hsa-miR-921, hsa-miR-1250-5p, hsa-miR-5093, hsa-miR-551b-5p, hsa-miR-4529-5p, hsa-miR-4538, hsa-miR-176, hsa-miR-1322, hsa-miR-473, hsa-miR-106a-5p, hsa-miR-297, hsa-miR-1910-3p, hsa-miR-4470, hsa-miR-3691-5p, hsa-miR-4999-5p, hsa-miR-3192-5p, hsa-miR-548au-3p, hsa-miR-5007-5p, hsa-miR-4657, hsa-miR-4768-3p, hsa-miR-1265, hsa-miR-4321, hsa-miR-4423-5p, hsa-miR-1294, and hsa-miR-4520-3p in a body fluid sample of the subject as an indicator.

According to the present invention, if at least one or all microRNA levels selected from the group consisting of hsa-miR-4644, hsa-miR-4754, hsa-miR-477-3p, hsa-miR-4445-3p, hsa-miR-4436b-3p, hsa-miR-4262, hsa-miR-1306-3p, hsa-miR-4514, hsa-miR-4296, hsa-miR-4458, hsa-miR-4520-5p, hsa-miR-766-5p, hsa-miR-548ao-3p, hsa-miR-758-5p, hsa-miR-4451, hsa-miR-4300, hsa-miR-4453, hsa-miR-4784, hsa-miR-6809-5p, hsa-miR-4692, hsa-miR-4437, hsa-miR-4681, hsa-miR-921, hsa-miR-1250-5p, hsa-miR-5093, hsa-miR-551b-5p, hsa-miR-4529-5p, hsa-miR-4538, hsa-miR-176, hsa-miR-1322, hsa-miR-473, hsa-miR-106a-5p, hsa-miR-297, hsa-miR-1910-3p, hsa-miR-4470, hsa-miR-3691-5p, hsa-miR-4999-5p, hsa-miR-3192-5p, hsa-miR-548au-3p, hsa-miR-5007-5p, hsa-miR-4657, hsa-miR-4768-3p, hsa-miR-1265, hsa-miR-4321, hsa-miR-4423-5p, hsa-miR-1294, and hsa-miR-4520-3p in a body fluid sample are higher than a predetermined value, it may also be determined that there is a likelihood that the subject has lung cancer ( and /or if the levels are lower than the predetermined value, it may be determined that there is a likelihood that the subject does not have lung cancer).

According to the present disclosure, the likelihood that a subject has lung cancer can also be examined using at least one, two, three, four, or five or more, or all microRNA levels selected from the group consisting of hsa-miR-4768-3p, hsa-miR-1265, hsa-miR-4321, hsa-miR-4423-5p, hsa-miR-1294, and hsa-miR-4520-3p in a body fluid sample of the subject as an indicator.

The present disclosure provides a method for examining the likelihood that a subject has lung cancer. According to the present disclosure, a method for examining the likelihood that a subject has lung cancer can be used to examine the likelihood that the subject has lung cancer using any one or more microRNA levels selected from Table 4-1 in a body fluid sample of the subject as an indicator. According to the present disclosure, the likelihood that a subject has lung cancer can be examined using at least 1, 2, 3, 4, 5, 6, 7, 8, 9, 10 or more, 15 or more, or 20 or more, or all microRNA levels selected from the group consisting of hsa-miR-147b, hsa-miR-3912-5p, hsa-miR-148a-5p, hsa-miR-6773-5p, hsa-miR-3681-5p, hsa-miR-3976, hsa-miR-3121-5p, hsa-miR-6082, hsa-miR-106b-5p, hsa-miR-758-3p, hsa-miR-4418, hsa-miR-216a-5p, hsa-miR-34a-3p, hsa-miR-516b-5p, hsa-miR-140-5p, hsa-miR-7154-5p, hsa-miR-616-5p, hsa-miR-5701, hsa-miR-582-3p, hsa-miR-626, hsa-miR-3653-3p, hsa-miR-4320, hsa-miR-3689a-5p, hsa-miR-3689b-5p, hsa-miR-3689e, hsa-miR-8058, hsa-miR-107, hsa-miR-10a-5p, hsa-miR-624-3p, hsa-miR-4683, hsa-miR-30a-5p, hsa-miR-222-3p, hsa-miR-580-5p, hsa-miR-5581-5p, hsa-miR-200c-3p, hsa-miR-2052, hsa-miR-5089-5p, hsa-miR-17-5p, hsa-miR-452-3p, hsa-miR-329-3p, hsa-miR-5092, hsa-miR-585-3p, hsa-miR-4503, hsa-miR-5011-5p, hsa-miR-372-5p, hsa-miR-5589-3p, hsa-miR-2114-5p, hsa-miR-4799-5p, hsa-miR-4422, hsa-miR-520d-3p, hsa-miR-6864-5p, hsa-miR-182-5p, hsa-miR-363-3p, hsa-miR-215-3p, hsa-miR-2681-3p, hsa-miR-922, hsa-miR-450a-2-3p, hsa-miR-520f-3p, hsa-miR-4501, hsa-miR-660-5p, hsa-miR-1911-5p, hsa-miR-20a-3p, hsa-miR-362-5p, hsa-miR-380-5p, hsa-miR-597-3p, hsa-miR-448, hsa-miR-3182, hsa-miR-99a-3p, hsa-miR-616-3p, hsa-miR-22-5p, hsa-miR-3591-5p, hsa-miR-4293, hsa-miR-433-3p, hsa-miR-1183, hsa-miR-662, hsa-miR-195-5p, hsa-miR-96-5p, hsa-miR-630, hsa-miR-7a-3p, hsa-miR-30e-3p, hsa-miR-361-5p, hsa-miR-6715b-3p, hsa-miR-563, hsa-miR-1263, hsa-miR-16-5p, hsa-miR-425-5p, hsa-miR-519a-3p, hsa-miR-655-5p, hsa-miR-429, hsa-miR-576-5p, hsa-miR-517-5p, hsa-miR-659-5p, hsa-miR-375, hsa-miR-4719, hsa-miR-891a-3p, hsa-miR-653-5p, hsa-miR-598-3p, hsa-miR-181a-5p, hsa-miR-15b-3p, hsa-miR-431-5p, hsa-miR-143-5p, hsa-miR-3157-5p, hsa-miR-181d-5p, hsa-miR-3978, hsa-miR-539-5p, hsa-let-7e-5p, hsa-miR-938, hsa-miR-369-5p, hsa-miR-656-3p, hsa-miR-132-3p, hsa-miR-23a-3p, hsa-miR-4699-3p, hsa-miR-624-5p, hsa-miR-7843-3p, hsa-miR-4524a-3p, hsa-miR-611, hsa-miR-5002-5p, hsa-miR-1286, hsa-miR-3144-3p, hsa-miR-4650-3p, hsa-miR-7162-5p, hsa-miR-548ba, hsa-miR-221-3p, hsa-miR-628-3p, hsa-miR-6715a-3p, hsa-miR-1297, hsa-miR-3907, hsa-miR-23b-5p, hsa-miR-374b-5p, hsa-miR-371a-3p, hsa-miR-372-3p, hsa-miR-7158-3p, hsa-miR-10b-5p, hsa-miR-554, hsa-miR-4325, hsa-miR-196a-5p, hsa-miR-5702, hsa-miR-519b-3p, hsa-miR-542-5p, hsa-miR-1298-5p, hsa-miR-20b-5p, hsa-miR-4661-5p, hsa-miR-172, hsa-miR-708-5p, hsa-miR-509-5p, hsa-miR-450a-5p, hsa-miR-7153-5p, hsa-miR-135a-5p, hsa-miR-4752, hsa-miR-578, hsa-miR-492, hsa-miR-93-3p, hsa-miR-1180-5p, hsa-miR-500a-5p, hsa-miR-181d-3p, hsa-miR-516b-3p, hsa-miR-516a-3p, hsa-miR-489-3p, hsa-miR-21-3p, hsa-miR-96-3p, hsa-miR-383-3p, hsa-miR-3921, hsa-miR-4762-5p, hsa-miR-1912, hsa-miR-3613-3p, hsa-miR-203b-5p, hsa-miR-135b-5p, hsa-miR-591, hsa-miR-521, hsa-miR-631, hsa-miR-4677-5p, hsa-miR-4804-5p, hsa-miR-5571-3p, hsa-miR-122-3p, hsa-miR-30c-5p, hsa-miR-6837-3p, hsa-miR-145-5p, hsa-miR-4643, hsa-miR-4522, hsa-miR-5588-5p, hsa-miR-888-5p, hsa-miR-4781-5p, hsa-miR-520a-3p, hsa-miR-28-5p, hsa-miR-580-3p, hsa-miR-4774-3p, hsa-miR-512-3p, hsa-miR-605-3p, hsa-miR-511-5p, hsa-miR-6768-3p, hsa-miR-552-5p, hsa-miR-491-3p, hsa-miR-173a, hsa-miR-635, hsa-let-7g-3p, hsa-miR-378a-5p, hsa-miR-200b-3p, hsa-miR-337-5p, hsa-miR-4694-3p, hsa-miR-3163, hsa-let-7f-2-3p, hsa-miR-4659a-3p, hsa-miR-5696, hsa-miR-103a-3p, hsa-miR-376c-3p, hsa-miR-4650-5p, hsa-miR-892c-3p, hsa-miR-203a-3p, hsa-miR-8055, hsa-miR-5004-3p, hsa-miR-339-5p, hsa-miR-7156-5p, hsa-miR-345-5p, hsa-miR-526b-3p, hsa-miR-16-2-3p, hsa-miR-552-3p, hsa-miR-340-3p, hsa-miR-3193, hsa-miR-517a-3p, hsa-miR-517b-3p, hsa-miR-1257, hsa-miR-3529-5p, hsa-miR-29a-3p, hsa-miR-566, hsa-miR-1205, hsa-miR-3622b-3p, hsa-miR-1-5p, hsa-miR-5187-3p, hsa-miR-130a-5p, hsa-miR-15a-3p, hsa-miR-146a-5p, hsa-miR-337-3p, hsa-miR-4691-3p, hsa-miR-205-5p, hsa-miR-374c-5p, hsa-miR-374c-3p, hsa-miR-551b-3p, hsa-miR-4694-5p, hsa-miR-276-5p, hsa-miR-520g-3p, hsa-miR-519e-3p, hsa-miR-299-5p, hsa-miR-25-3p, hsa-miR-8079, hsa-miR-6510-3p, hsa-miR-223-5p, hsa-miR-216b-3p, hsa-miR-99b-5p, hsa-miR-1289, hsa-miR-8086, hsa-miR-195-3p, hsa-miR-7853-5p, hsa-miR-4794, hsa-miR-1911-3p, hsa-miR-6811-3p, hsa-miR-605-5p, hsa-miR-4704-5p, hsa-miR-204-5p, hsa-miR-302d-5p, hsa-miR-4266, hsa-miR-519d-3p, hsa-miR-5684, hsa-miR-4755-5p, hsa-miR-589-5p, hsa-miR-125b-5p, hsa-miR-1291, hsa-miR-1200, hsa-miR-4724-3p, hsa-miR-93-5p, hsa-miR-942-5p, hsa-miR-432-3p, hsa-miR-7-2-3p, hsa-miR-642a-5p, hsa-miR-370-5p, hsa-miR-8074, hsa-miR-512-5p, hsa-miR-506-3p, hsa-miR-6774-3p, hsa-miR-1269b, hsa-miR-150-5p, hsa-miR-3155a, hsa-miR-208a-5p, hsa-miR-501-5p, hsa-miR-30c-1-3p, hsa-miR-5571-5p, hsa-miR-6758-3p, hsa-miR-1184, hsa-miR-454-5p, hsa-miR-34b-5p, hsa-miR-6838-5p, hsa-miR-92b-3p, hsa-miR-4652-3p, hsa-miR-4747-3p, hsa-miR-1255b-2-3p, hsa-miR-1282, hsa-miR-173h-3p, hsa-miR-7703, hsa-miR-3919, hsa-miR-324-5p, hsa-miR-1251-3p, hsa-miR-671-5p, hsa-miR-6508-5p, hsa-miR-23b-3p, hsa-miR-3184-5p, hsa-miR-6744-3p, hsa-miR-642a-3p, hsa-miR-98-3p, hsa-miR-4288, hsa-miR-1203, hsa-miR-3181, hsa-miR-6500-5p, hsa-miR-380-3p, hsa-miR-574-5p, hsa-miR-4328, hsa-miR-5584-3p, hsa-miR-6781-3p, hsa-miR-632, hsa-miR-193b-3p, and hsa-miR-6841-3p in a body fluid sample of the subject as an indicator. According to the present disclosure, the likelihood that a subject has lung cancer can be examined using at least 1, 2, 3, 4, 5, 6, 7, 8, 9, 10 or more, 15 or more, or 20 or more, or all microRNA levels selected from the group consisting of hsa-miR-147b, hsa-miR-3912-5p, hsa-miR-148a-5p, hsa-miR-6773-5p, hsa-miR-3681-5p, hsa-miR-3976, hsa-miR-3121-5p, hsa-miR-6082, hsa-miR-106b-5p, hsa-miR-758-3p, hsa-miR-4418, hsa-miR-216a-5p, hsa-miR-34a-3p, hsa-miR-516b-5p, hsa-miR-140-5p, hsa-miR-7154-5p, hsa-miR-616-5p, hsa-miR-5701, hsa-miR-582-3p, hsa-miR-626, hsa-miR-3653-3p, hsa-miR-4320, hsa-miR-3689a-5p, hsa-miR-3689b-5p, hsa-miR-3689e, hsa-miR-8058, hsa-miR-107, hsa-miR-10a-5p, hsa-miR-624-3p, hsa-miR-4683, hsa-miR-30a-5p, hsa-miR-222-3p, hsa-miR-580-5p, hsa-miR-5581-5p, hsa-miR-200c-3p, hsa-miR-2052, hsa-miR-5089-5p, hsa-miR-17-5p, hsa-miR-452-3p, hsa-miR-329-3p, hsa-miR-5092, hsa-miR-585-3p, hsa-miR-4503, hsa-miR-5011-5p, hsa-miR-372-5p, hsa-miR-5589-3p, hsa-miR-2114-5p, hsa-miR-4799-5p, hsa-miR-4422, hsa-miR-520d-3p, hsa-miR-6864-5p, hsa-miR-182-5p, hsa-miR-363-3p, hsa-miR-215-3p, hsa-miR-2681-3p, hsa-miR-922, hsa-miR-450a-2-3p, hsa-miR-520f-3p, hsa-miR-4501, hsa-miR-660-5p, hsa-miR-1911-5p, hsa-miR-20a-3p, hsa-miR-362-5p, hsa-miR-380-5p, hsa-miR-597-3p, hsa-miR-448, hsa-miR-3182, hsa-miR-99a-3p, hsa-miR-616-3p, hsa-miR-22-5p, hsa-miR-3591-5p, hsa-miR-4293, hsa-miR-433-3p, hsa-miR-1183, hsa-miR-662, hsa-miR-195-5p, hsa-miR-96-5p, hsa-miR-630, hsa-miR-7a-3p, hsa-miR-30e-3p, hsa-miR-361-5p, hsa-miR-6715b-3p, hsa-miR-563, hsa-miR-1263, hsa-miR-16-5p, hsa-miR-425-5p, hsa-miR-519a-3p, hsa-miR-655-5p, hsa-miR-429, hsa-miR-576-5p, hsa-miR-517-5p, hsa-miR-659-5p, hsa-miR-375, hsa-miR-4719, hsa-miR-891a-3p, hsa-miR-653-5p, hsa-miR-598-3p, hsa-miR-181a-5p, hsa-miR-15b-3p, hsa-miR-431-5p, hsa-miR-143-5p, hsa-miR-3157-5p, hsa-miR-181d-5p, hsa-miR-3978, hsa-miR-539-5p, hsa-let-7e-5p, hsa-miR-938, hsa-miR-369-5p, hsa-miR-656-3p, hsa-miR-132-3p, hsa-miR-23a-3p, hsa-miR-4699-3p, hsa-miR-624-5p, hsa-miR-7843-3p, hsa-miR-4524a-3p, hsa-miR-611, hsa-miR-5002-5p, hsa-miR-1286, hsa-miR-3144-3p, hsa-miR-4650-3p, hsa-miR-7162-5p, hsa-miR-548ba, hsa-miR-221-3p, hsa-miR-628-3p, hsa-miR-6715a-3p, hsa-miR-1297, hsa-miR-3907, hsa-miR-23b-5p, hsa-miR-374b-5p, hsa-miR-371a-3p, hsa-miR-372-3p, hsa-miR-7158-3p, hsa-miR-10b-5p, hsa-miR-554, hsa-miR-4325, hsa-miR-196a-5p, hsa-miR-5702, hsa-miR-519b-3p, hsa-miR-542-5p, hsa-miR-1298-5p, hsa-miR-20b-5p, hsa-miR-4661-5p, hsa-miR-172, hsa-miR-708-5p, hsa-miR-509-5p, hsa-miR-450a-5p, hsa-miR-7153-5p, hsa-miR-135a-5p, hsa-miR-4752, hsa-miR-578, hsa-miR-492, hsa-miR-93-3p, hsa-miR-1180-5p, hsa-miR-500a-5p, hsa-miR-181d-3p, hsa-miR-516b-3p, hsa-miR-516a-3p, hsa-miR-489-3p, hsa-miR-21-3p, hsa-miR-96-3p, hsa-miR-383-3p, hsa-miR-3921, hsa-miR-4762-5p, hsa-miR-1912, hsa-miR-3613-3p, hsa-miR-203b-5p, hsa-miR-135b-5p, hsa-miR-591, hsa-miR-521, hsa-miR-631, hsa-miR-4677-5p, hsa-miR-4804-5p, hsa-miR-5571-3p, hsa-miR-122-3p, and hsa-miR-30c-5p in a body fluid sample of the subject as an indicator. According to the present disclosure, the likelihood that a subject has lung cancer can be examined using at least 1, 2, 3, 4, 5, 6, 7, 8, 9, 10 or more, 15 or more, or 20 or more, or all microRNA levels selected from the group consisting of hsa-miR-147b, hsa-miR-3912-5p, hsa-miR-148a-5p, hsa-miR-6773-5p, hsa-miR-3681-5p, hsa-miR-3976, hsa-miR-3121-5p, hsa-miR-6082, hsa-miR-106b-5p, hsa-miR-758-3p, hsa-miR-4418, hsa-miR-216a-5p, hsa-miR-34a-3p, hsa-miR-516b-5p, hsa-miR-140-5p, hsa-miR-7154-5p, hsa-miR-616-5p, hsa-miR-5701, hsa-miR-582-3p, hsa-miR-626, hsa-miR-3653-3p, hsa-miR-4320, hsa-miR-3689a-5p, hsa-miR-3689b-5p, hsa-miR-3689e, hsa-miR-8058, hsa-miR-107, hsa-miR-10a-5p, hsa-miR-624-3p, hsa-miR-4683, hsa-miR-30a-5p, hsa-miR-222-3p, hsa-miR-580-5p, hsa-miR-5581-5p, hsa-miR-200c-3p, hsa-miR-2052, hsa-miR-5089-5p, hsa-miR-17-5p, hsa-miR-452-3p, hsa-miR-329-3p, hsa-miR-5092, hsa-miR-585-3p, hsa-miR-4503, hsa-miR-5011-5p, hsa-miR-372-5p, hsa-miR-5589-3p, hsa-miR-2114-5p, hsa-miR-4799-5p, hsa-miR-4422, hsa-miR-520d-3p, hsa-miR-6864-5p, hsa-miR-182-5p, hsa-miR-363-3p, hsa-miR-215-3p, hsa-miR-2681-3p, hsa-miR-922, hsa-miR-450a-2-3p, hsa-miR-520f-3p, hsa-miR-4501, hsa-miR-660-5p, hsa-miR-1911-5p, hsa-miR-20a-3p, hsa-miR-362-5p, hsa-miR-380-5p, hsa-miR-597-3p, hsa-miR-448, hsa-miR-3182, hsa-miR-99a-3p, hsa-miR-616-3p, hsa-miR-22-5p, hsa-miR-3591-5p, hsa-miR-4293, hsa-miR-433-3p, hsa-miR-1183, hsa-miR-662, hsa-miR-195-5p, hsa-miR-96-5p, hsa-miR-630, hsa-miR-7a-3p, hsa-miR-30e-3p, hsa-miR-361-5p, hsa-miR-6715b-3p, hsa-miR-563, hsa-miR-1263, hsa-miR-16-5p, hsa-miR-425-5p, hsa-miR-519a-3p, hsa-miR-655-5p, hsa-miR-429, hsa-miR-576-5p, hsa-miR-517-5p, hsa-miR-659-5p, hsa-miR-375, hsa-miR-4719, hsa-miR-891a-3p, hsa-miR-653-5p, hsa-miR-598-3p, hsa-miR-181a-5p, hsa-miR-15b-3p, hsa-miR-431-5p, hsa-miR-143-5p, hsa-miR-3157-5p, hsa-miR-181d-5p, hsa-miR-3978, hsa-miR-539-5p, and hsa-let-7e-5p in a body fluid sample of the subject as an indicator.

According to the present disclosure, the likelihood that a subject has lung cancer can be examined using at least 1, 2, 3, 4, 5, 6, 7, 8, 9, 10 or more, 15 or more, or 20 or more, or all microRNA levels selected from the group consisting of hsa-miR-147b, hsa-miR-3912-5p, hsa-miR-148a-5p, hsa-miR-6773-5p, hsa-miR-3681-5p, hsa-miR-3976, hsa-miR-3121-5p, hsa-miR-6082, hsa-miR-106b-5p, hsa-miR-758-3p, hsa-miR-4418, hsa-miR-216a-5p, hsa-miR-34a-3p, hsa-miR-516b-5p, hsa-miR-140-5p, hsa-miR-7154-5p, hsa-miR-616-5p, hsa-miR-5701, hsa-miR-582-3p, hsa-miR-626, hsa-miR-3653-3p, hsa-miR-4320, hsa-miR-3689a-5p, hsa-miR-3689b-5p, hsa-miR-3689e, hsa-miR-8058, hsa-miR-107, hsa-miR-10a-5p, hsa-miR-624-3p, hsa-miR-4683, hsa-miR-30a-5p, hsa-miR-222-3p, hsa-miR-580-5p, hsa-miR-5581-5p, hsa-miR-200c-3p, hsa-miR-2052, hsa-miR-5089-5p, hsa-miR-17-5p, and hsa-miR-452-3p in a body fluid sample of the subject as an indicator.

According to the present disclosure, the likelihood that a subject has lung cancer can be examined using at least 1, 2, 3, 4, 5, 6, 7, 8, 9, 10 or more, 15 or more, or 20 or more, or all microRNA levels selected from the group consisting of hsa-let-7a-3p, hsa-let-7g-5p, hsa-miR-100-3p, hsa-miR-101-3p, hsa-miR-105-3p, hsa-miR-10b-3p, hsa-miR-1185-5p, hsa-miR-1197, hsa-miR-1206, hsa-miR-1243, hsa-miR-1245b-3p, hsa-miR-1246, hsa-miR-1252-3p, hsa-miR-1252-5p, hsa-miR-1258, hsa-miR-126-3p, hsa-miR-126-5p, hsa-miR-1277-3p, hsa-miR-1277-5p, hsa-miR-1279, hsa-miR-1295b-5p, hsa-miR-136-5p, hsa-miR-1-3p, hsa-miR-145-3p, hsa-miR-1468-3p, hsa-miR-152-3p, hsa-miR-153-3p, hsa-miR-155-5p, hsa-miR-186-5p, hsa-miR-18a-5p, hsa-miR-190a-5p, hsa-miR-190b, hsa-miR-19a-5p, hsa-miR-19b-1-5p, hsa-miR-19b-2-5p, hsa-miR-19b-3p, hsa-miR-2053, hsa-miR-205-3p, hsa-miR-2054, hsa-miR-208a-3p, hsa-miR-20a-5p, hsa-miR-219a-1-3p, hsa-miR-219b-3p, hsa-miR-301a-3p, hsa-miR-30d-3p, hsa-miR-30e-5p, hsa-miR-3118, hsa-miR-3123, hsa-miR-3129-5p, hsa-miR-3135a, hsa-miR-3140-3p, hsa-miR-3143, hsa-miR-3152-3p, hsa-miR-3167, hsa-miR-3169, hsa-miR-3183, hsa-miR-3201, hsa-miR-335-5p, hsa-miR-339-3p, hsa-miR-3607-5p, hsa-miR-3616-5p, hsa-miR-3617-5p, hsa-miR-3618, hsa-miR-3662, hsa-miR-3668, hsa-miR-3683, hsa-miR-3686, hsa-miR-3688-3p, hsa-miR-369-3p, hsa-miR-374a-3p, hsa-miR-374a-5p, hsa-miR-376a-5p, hsa-miR-3927-3p, hsa-miR-3942-5p, hsa-miR-4277, hsa-miR-4302, hsa-miR-4432, hsa-miR-4452, hsa-miR-4457, hsa-miR-4474-5p, hsa-miR-4477b, hsa-miR-4480, hsa-miR-4495, hsa-miR-4504, hsa-miR-4509, hsa-miR-450a-1-3p, hsa-miR-450b-3p, hsa-miR-455-5p, hsa-miR-4633-3p, hsa-miR-4639-5p, hsa-miR-4668-3p, hsa-miR-4671-3p, hsa-miR-4679, hsa-miR-4693-3p, hsa-miR-4699-5p, hsa-miR-4704-3p, hsa-miR-4714-3p, hsa-miR-4720-3p, hsa-miR-4735-5p, hsa-miR-4757-3p, hsa-miR-4760-5p, hsa-miR-4772-5p, hsa-miR-4777-5p, hsa-miR-4781-3p, hsa-miR-4782-3p, hsa-miR-4782-5p, hsa-miR-4790-3p, hsa-miR-4791, hsa-miR-4795-5p, hsa-miR-4796-3p, hsa-miR-4798-5p, hsa-miR-4799-3p, hsa-miR-4802-5p, hsa-miR-499a-5p, hsa-miR-5009-3p, hsa-miR-5009-5p, hsa-miR-506-5p, hsa-miR-507, hsa-miR-510-3p, hsa-miR-513b-3p, hsa-miR-514a-5p, hsa-miR-5191, hsa-miR-544a, hsa-miR-545-5p, hsa-miR-548a-3p, hsa-miR-548a-5p, hsa-miR-548aa, hsa-miR-548t-3p, hsa-miR-548ac, hsa-miR-548ad-5p, hsa-miR-548ae-5p, hsa-miR-548ae-3p, hsa-miR-548ag, hsa-miR-548ah-5p, hsa-miR-548ak, hsa-miR-548al, hsa-miR-548am-3p, hsa-miR-548ao-5p, hsa-miR-548ap-5p, hsa-miR-548aq-5p, hsa-miR-548at-3p, hsa-miR-548au-5p, hsa-miR-548az-5p, hsa-miR-548b-5p, hsa-miR-548c-5p, hsa-miR-548o-5p, hsa-miR-548am-5p, hsa-miR-548g-5p, hsa-miR-548x-5p, hsa-miR-548aj-5p, hsa-miR-548j-5p, hsa-miR-548k, hsa-miR-548m, hsa-miR-548p, hsa-miR-548t-5p, hsa-miR-548u, hsa-miR-548v, hsa-miR-548x-3p, hsa-miR-549a, hsa-miR-553, hsa-miR-556-3p, hsa-miR-5579-5p, hsa-miR-5583-5p, hsa-miR-559, hsa-miR-5590-3p, hsa-miR-5590-5p, hsa-miR-5591-5p, hsa-miR-561-5p, hsa-miR-568, hsa-miR-5687, hsa-miR-569, hsa-miR-5692c, hsa-miR-5697, hsa-miR-5700, hsa-miR-570-3p, hsa-miR-577, hsa-miR-582-5p, hsa-miR-587, hsa-miR-590-3p, hsa-miR-6079, hsa-miR-6128, hsa-miR-618, hsa-miR-621, hsa-miR-628-5p, hsa-miR-633, hsa-miR-648, hsa-miR-6508-3p, hsa-miR-651-3p, hsa-miR-652-3p, hsa-miR-6733-5p, hsa-miR-6811-5p, hsa-miR-6844, hsa-miR-6854-5p, hsa-miR-7161-3p, hsa-miR-759, hsa-miR-7-5p, hsa-miR-7852-3p, hsa-miR-873-5p, hsa-miR-876-5p, hsa-miR-9-3p, hsa-miR-95-3p, hsa-miR-95-5p, hsa-miR-9-5p, hsa-miR-4525, hsa-miR-6760-5p, hsa-miR-4538, hsa-miR-5698, hsa-miR-5189-5p, hsa-miR-671-5p, hsa-miR-936, hsa-miR-4307, hsa-miR-6815-5p, hsa-miR-6076, hsa-miR-708-5p, hsa-miR-3187-5p, hsa-miR-3144-3p, hsa-miR-4691-5p, hsa-miR-4700-5p, hsa-miR-576-5p, hsa-miR-3652, hsa-miR-598-3p, hsa-miR-7151-3p, hsa-miR-7162-5p, hsa-miR-1229-5p, hsa-miR-6746-5p, hsa-miR-4762-5p, hsa-miR-1307-3p, hsa-miR-1273g-3p, hsa-miR-4428, hsa-miR-6515-5p, hsa-miR-1273g-5p, hsa-miR-433-3p, hsa-miR-452-3p, hsa-miR-3917, hsa-miR-1224-5p, hsa-miR-6892-5p, hsa-miR-520d-5p, hsa-miR-5092, hsa-miR-30c-1-3p, hsa-miR-1293, hsa-miR-7851-3p, hsa-miR-3934-5p, hsa-miR-3122, hsa-miR-3177-3p, hsa-miR-4800-5p, hsa-miR-4436b-3p, hsa-miR-3928-3p, hsa-miR-630, hsa-miR-628-3p, hsa-miR-6796-5p, hsa-miR-3605-5p, hsa-miR-30e-3p, hsa-miR-6801-5p, hsa-miR-6131, hsa-miR-448, hsa-miR-6834-5p, hsa-miR-3156-5p, hsa-miR-517-5p, hsa-miR-4520-5p, hsa-miR-2276-3p, hsa-miR-3591-3p, hsa-miR-4727-3p, hsa-miR-4499, hsa-miR-1471, hsa-miR-1273h-5p, hsa-miR-4539, hsa-miR-3655, hsa-miR-4690-5p, hsa-miR-6871-5p, hsa-miR-4496, hsa-miR-6772-5p, hsa-miR-4686, hsa-miR-1297, hsa-miR-450a-2-3p, hsa-miR-6745, hsa-miR-3153, hsa-miR-27a-3p, hsa-miR-106b-5p, hsa-miR-519c-5p, hsa-miR-523-5p, hsa-miR-518e-5p, hsa-miR-522-5p, hsa-miR-519a-5p, hsa-miR-519b-5p, hsa-miR-4676-5p, hsa-miR-650, hsa-miR-3922-5p, hsa-miR-4489, hsa-miR-4638-5p, hsa-miR-185-3p, hsa-miR-2467-3p, hsa-miR-4453, hsa-let-7e-5p, hsa-miR-296-3p, hsa-miR-8074, hsa-miR-6876-5p, hsa-miR-6086, hsa-miR-4474-3p, hsa-miR-4776-3p, hsa-miR-1250-5p, hsa-miR-4260, hsa-miR-6747-5p, hsa-miR-181d-3p, hsa-miR-4535, hsa-miR-3177-5p, hsa-miR-4533, hsa-miR-4635, hsa-miR-4776-5p, hsa-miR-4257, hsa-miR-3160-3p, hsa-miR-5702, hsa-miR-4306, hsa-miR-4483, hsa-miR-4472, hsa-miR-3064-3p, hsa-miR-4710, hsa-miR-4293, hsa-miR-449c-5p, hsa-miR-5589-5p, hsa-miR-4443, hsa-miR-4448, hsa-miR-3935, hsa-miR-16-5p, hsa-miR-181a-5p, hsa-miR-147b, hsa-miR-4767, hsa-miR-4677-5p, hsa-miR-6776-5p, hsa-miR-7109-5p, hsa-miR-4669, hsa-miR-4486, hsa-miR-1587, hsa-miR-363-3p, hsa-miR-5701, hsa-miR-921, hsa-miR-5581-5p, hsa-miR-4259, hsa-miR-203b-5p, hsa-miR-3653-3p, hsa-miR-4657, hsa-miR-5589-3p, hsa-miR-2681-3p, hsa-miR-6826-5p, hsa-miR-4647, hsa-miR-4540, hsa-miR-361-5p, hsa-miR-4784, hsa-miR-6861-5p, hsa-miR-6812-5p, hsa-miR-608, hsa-miR-1912, hsa-miR-554, hsa-miR-519a-3p, hsa-miR-891a-3p, hsa-miR-6872-5p, hsa-miR-4781-5p, hsa-miR-4273, hsa-miR-4769-5p, hsa-miR-505-5p, hsa-miR-7158-3p, hsa-miR-527, hsa-miR-518a-5p, hsa-miR-1273f, hsa-miR-624-5p, hsa-miR-125a-3p, hsa-miR-6830-5p, hsa-miR-520d-3p, hsa-miR-6757-5p, hsa-miR-4785, hsa-miR-6068, hsa-miR-1272, hsa-miR-4654, hsa-miR-564, hsa-miR-563, hsa-miR-4300, hsa-miR-4665-5p, hsa-miR-892c-3p, hsa-miR-6778-5p, hsa-miR-4419b, hsa-miR-329-3p, hsa-miR-212-5p, hsa-miR-616-3p, hsa-miR-4269, hsa-miR-4487, hsa-miR-1180-5p, hsa-miR-7850-5p, hsa-miR-492, hsa-miR-718, hsa-miR-1972, hsa-miR-4296, hsa-miR-516b-3p, hsa-miR-516a-3p, hsa-miR-4320, hsa-miR-4529-5p, hsa-miR-656-3p, hsa-miR-6500-3p, hsa-miR-766-5p, hsa-miR-466, hsa-miR-6777-5p, hsa-miR-1909-5p, hsa-miR-187-3p, hsa-miR-8079, hsa-miR-30a-5p, hsa-miR-5002-3p, hsa-miR-6833-5p, hsa-miR-4756-3p, hsa-let-7f-2-3p, hsa-miR-5708, hsa-miR-4721, hsa-miR-8085, hsa-miR-3591-5p, hsa-miR-6859-5p, hsa-miR-5006-5p, hsa-miR-4747-5p, hsa-miR-6827-5p, hsa-miR-3612, hsa-miR-4701-3p, hsa-miR-4418, hsa-miR-4449, hsa-miR-181d-5p, hsa-miR-3619-3p, hsa-miR-6817-5p, hsa-miR-6759-5p, hsa-miR-3654, hsa-miR-3164, hsa-miR-491-3p, hsa-miR-3176, hsa-miR-4683, hsa-miR-6807-5p, hsa-miR-5588-5p, hsa-miR-302c-5p, hsa-miR-4522, hsa-miR-1199-5p, hsa-miR-372-3p, hsa-miR-891a-5p, hsa-miR-1306-3p, hsa-miR-6894-5p, hsa-miR-3613-3p, hsa-miR-548ba, hsa-miR-6795-5p, hsa-miR-6794-5p, hsa-miR-585-3p, hsa-miR-194-3p, hsa-miR-5010-5p, hsa-miR-2052, hsa-miR-614, hsa-miR-6751-5p, hsa-miR-5089-3p, hsa-miR-1289, hsa-miR-668-5p, hsa-miR-3622b-5p, hsa-miR-6887-5p, hsa-miR-4524a-3p, hsa-miR-3689a-3p, hsa-miR-4650-5p, hsa-miR-3689b-3p, hsa-miR-3689c, hsa-miR-624-3p, hsa-miR-6129, hsa-miR-216a-5p, hsa-miR-520b, hsa-miR-4755-5p, hsa-miR-323a-5p, hsa-miR-6726-5p, hsa-miR-516b-5p, hsa-miR-7106-5p, hsa-miR-653-5p, hsa-miR-494-5p, hsa-miR-6780b-5p, hsa-miR-1910-3p, hsa-miR-3160-5p, hsa-miR-5093, hsa-miR-99a-3p, hsa-miR-8071, hsa-miR-4659a-3p, hsa-miR-597-3p, hsa-miR-4458, hsa-miR-6847-5p, hsa-miR-4436a, hsa-miR-17-5p, hsa-miR-185-5p, hsa-miR-4681, hsa-miR-8077, hsa-miR-4440, hsa-let-7g-3p, hsa-miR-375, hsa-miR-10a-5p, hsa-miR-520e, hsa-miR-6072, hsa-miR-3145-5p, hsa-miR-6723-5p, hsa-miR-4692, hsa-miR-4796-5p, hsa-miR-6849-5p, hsa-miR-4430, hsa-miR-6499-5p, hsa-miR-3151-5p, hsa-miR-182-5p, hsa-miR-195-5p, hsa-miR-3681-5p, hsa-miR-6864-5p, hsa-miR-4711-3p, hsa-miR-380-3p, hsa-miR-374c-5p, hsa-miR-758-5p, hsa-miR-6842-5p, hsa-miR-8080, hsa-miR-520f-3p, hsa-miR-4646-5p, hsa-miR-103a-3p, hsa-miR-3189-3p, hsa-miR-4691-3p, hsa-miR-877-5p, hsa-miR-6769b-5p, hsa-miR-5580-5p, hsa-miR-1911-5p, hsa-miR-519b-3p, hsa-miR-660-5p, hsa-miR-4644, hsa-miR-6133, hsa-miR-138-1-3p, hsa-miR-193a-5p, hsa-miR-222-3p, hsa-miR-603, hsa-miR-23b-3p, hsa-miR-5089-5p, hsa-miR-6799-5p, hsa-miR-6715a-3p, hsa-miR-6869-3p, hsa-miR-4445-3p, hsa-miR-542-5p, hsa-miR-1286, hsa-miR-96-3p, hsa-miR-4743-5p, hsa-miR-4724-3p, hsa-miR-4262, hsa-miR-4507, hsa-miR-7160-5p, hsa-miR-605-3p, hsa-miR-200c-3p, hsa-miR-582-3p, hsa-miR-6868-5p, hsa-miR-6773-5p, hsa-miR-1185-1-3p, hsa-miR-3182, hsa-miR-6082, hsa-miR-150-3p, hsa-miR-5002-5p, hsa-miR-487a-5p, hsa-miR-8058, hsa-miR-4529-3p, hsa-miR-3713, hsa-miR-8075, hsa-miR-6877-5p, hsa-miR-4325, hsa-miR-3130-5p, hsa-miR-616-5p, hsa-miR-1322, hsa-miR-6793-5p, hsa-miR-4694-5p, hsa-miR-29a-3p, hsa-miR-6127, hsa-miR-758-3p, hsa-miR-2114-5p, hsa-miR-515-5p, hsa-miR-196a-5p, hsa-miR-4639-3p, hsa-miR-4799-5p, hsa-miR-4498, hsa-miR-6766-5p, hsa-miR-1255b-2-3p, hsa-miR-380-5p, hsa-miR-4419a, hsa-miR-3622a-5p, hsa-let-7b-5p, hsa-miR-4650-3p, hsa-miR-29b-1-5p, hsa-miR-3137, hsa-miR-4447, hsa-miR-6500-5p, hsa-miR-3663-5p, hsa-miR-4451, hsa-miR-4422, hsa-miR-662, hsa-miR-122-3p, hsa-miR-6828-5p, hsa-miR-6884-5p, hsa-miR-4502, hsa-miR-374b-5p, hsa-miR-4462, hsa-miR-4478, hsa-miR-431-3p, hsa-miR-23b-5p, hsa-miR-7975, hsa-miR-5011-5p, hsa-miR-3691-5p, hsa-miR-6768-3p, hsa-miR-591, hsa-miR-8086, hsa-miR-372-5p, hsa-miR-5189-3p, hsa-miR-107, hsa-miR-6891-5p, hsa-miR-3138, hsa-miR-6503-3p, hsa-miR-297, hsa-miR-370-5p, hsa-miR-3944-3p, hsa-miR-425-5p, hsa-miR-3978, hsa-miR-148a-5p, hsa-miR-4684-5p, hsa-miR-34a-3p, hsa-miR-1263, hsa-miR-769-5p, hsa-miR-4437, hsa-miR-2277-3p, hsa-miR-659-5p, hsa-miR-661, hsa-miR-6788-5p, hsa-miR-15b-3p, hsa-miR-1205, hsa-miR-1468-5p, hsa-miR-34a-5p, hsa-miR-3120-5p, hsa-miR-1247-3p, hsa-miR-454-5p, hsa-miR-656-5p, hsa-miR-626, hsa-miR-4441, hsa-miR-6780a-5p, hsa-miR-6769a-5p, hsa-miR-1298-5p, hsa-miR-3157-5p, hsa-miR-6814-5p, hsa-miR-500a-5p, hsa-miR-1321, hsa-miR-4481, hsa-miR-611, hsa-miR-1273a, hsa-miR-337-5p, hsa-miR-203a-5p, hsa-miR-4479, hsa-miR-4719, hsa-miR-8083, hsa-miR-132-3p, hsa-miR-20b-5p, hsa-miR-4252, hsa-miR-429, hsa-miR-378b, hsa-miR-134-5p, hsa-miR-6741-5p, hsa-miR-1193, hsa-miR-143-5p, hsa-miR-515-3p, hsa-miR-4774-3p, hsa-miR-8064, hsa-miR-1183, hsa-miR-5684, hsa-miR-1226-5p, hsa-miR-539-5p, hsa-miR-580-5p, hsa-miR-622, hsa-miR-3907, hsa-miR-6504-5p, hsa-miR-4653-3p, hsa-miR-4706, hsa-miR-4515, hsa-miR-3919, hsa-miR-3187-3p, hsa-miR-1271-5p, hsa-miR-6767-5p, hsa-miR-4804-5p, hsa-miR-1257, hsa-miR-3126-5p, hsa-miR-6731-5p, hsa-miR-3149, hsa-miR-4717-3p, hsa-miR-509-5p, hsa-miR-215-3p, hsa-miR-1178-3p, hsa-miR-1282, hsa-miR-3174, hsa-miR-197-5p, hsa-miR-376c-3p, hsa-miR-5696, hsa-miR-3121-5p, hsa-miR-8082, hsa-miR-6823-5p, hsa-miR-4704-5p, hsa-miR-3616-3p, hsa-miR-6881-5p, hsa-miR-4661-5p, hsa-miR-22-5p, hsa-miR-6730-5p, hsa-let-7e-3p, hsa-miR-1238-5p, hsa-miR-526b-5p, hsa-miR-3163, hsa-miR-4514, hsa-miR-4699-3p, hsa-miR-7154-5p, hsa-miR-4521, hsa-miR-96-5p, hsa-miR-4754, hsa-miR-450a-5p, hsa-miR-4459, hsa-miR-922, hsa-miR-7153-5p, hsa-miR-29b-2-5p, hsa-miR-6738-3p, hsa-miR-6790-5p, hsa-miR-4470, hsa-miR-4732-3p, hsa-miR-4709-3p, hsa-miR-5588-3p, hsa-miR-93-3p, hsa-miR-6820-5p, hsa-miR-655-5p, hsa-miR-6514-3p, hsa-miR-1-5p, hsa-miR-10b-5p, hsa-miR-3193, hsa-miR-3976, hsa-miR-3678-3p, hsa-miR-3912-5p, hsa-miR-4708-3p, hsa-miR-5192, hsa-miR-140-5p, hsa-miR-4285, hsa-miR-3124-5p, hsa-miR-198, hsa-miR-3659, hsa-miR-3689a-5p, hsa-miR-3689b-5p, hsa-miR-3689e, hsa-miR-4643, hsa-miR-552-3p, hsa-miR-3186-3p, hsa-miR-424-3p, hsa-miR-578, hsa-miR-4304, hsa-miR-504-3p, hsa-miR-595, hsa-miR-4501, hsa-miR-4746-3p, hsa-miR-362-5p, hsa-miR-4297, hsa-miR-6821-3p, hsa-miR-3074-5p, hsa-miR-4503, hsa-miR-4518, hsa-miR-6715b-3p, hsa-miR-6736-5p, hsa-miR-7843-3p, hsa-miR-4420, hsa-miR-3927-5p, hsa-miR-7157-5p in a body fluid sample of the subject as an indicator.

In the present disclosure, the likelihood that a subject has lung cancer (particularly stage-I lung cancer) can be examined using at least 1, 2, 3, 4, 5, 6, 7, 8, 9, 10 or more, 15 or more, or 20 or more, or all microRNA levels selected from the group consisting of hsa-let-7e-5p, hsa-let-7f-2-3p, hsa-let-7g-3p, hsa-miR-103a-3p, hsa-miR-106b-5p, hsa-miR-107, hsa-miR-10a-5p, hsa-miR-10b-5p, hsa-miR-1180-5p, hsa-miR-1183, hsa-miR-1200, hsa-miR-1205, hsa-miR-122-3p, hsa-miR-1247-3p, hsa-miR-1257, hsa-miR-1263, hsa-miR-1272, hsa-miR-1273a, hsa-miR-1275, hsa-miR-1282, hsa-miR-128-2-5p, hsa-miR-1289, hsa-miR-1297, hsa-miR-1298-5p, hsa-miR-132-3p, hsa-miR-133a-3p, hsa-miR-134-5p, hsa-miR-135b-5p, hsa-miR-140-5p, hsa-miR-143-5p, hsa-miR-1470, hsa-miR-147b, hsa-miR-148a-5p, hsa-miR-150-5p, hsa-miR-15a-3p, hsa-miR-15b-3p, hsa-miR-1-5p, hsa-miR-16-5p, hsa-miR-17-5p, hsa-miR-181a-5p, hsa-miR-181d-3p, hsa-miR-181d-5p, hsa-miR-182-5p, hsa-miR-1911-5p, hsa-miR-1914-3p, hsa-miR-193b-3p, hsa-miR-195-3p, hsa-miR-195-5p, hsa-miR-196a-5p, hsa-miR-198, hsa-miR-200c-3p, hsa-miR-203b-5p, hsa-miR-2052, hsa-miR-205-5p, hsa-miR-208a-5p, hsa-miR-20a-3p, hsa-miR-20b-5p, hsa-miR-2114-5p, hsa-miR-215-3p, hsa-miR-216a-5p, hsa-miR-222-3p, hsa-miR-223-5p, hsa-miR-22-5p, hsa-miR-23a-3p, hsa-miR-23b-5p, hsa-miR-2681-3p, hsa-miR-27a-3p, hsa-miR-2861, hsa-miR-29a-3p, hsa-miR-29b-1-5p, hsa-miR-29b-2-5p, hsa-miR-30a-5p, hsa-miR-30e-3p, hsa-miR-3121-5p, hsa-miR-3127-3p, hsa-miR-3157-5p, hsa-miR-3162-3p, hsa-miR-3163, hsa-miR-3181, hsa-miR-3182, hsa-miR-3184-5p, hsa-miR-3188, hsa-miR-326, hsa-miR-328-5p, hsa-miR-329-3p, hsa-miR-337-5p, hsa-miR-34a-3p, hsa-miR-3591-5p, hsa-miR-3613-3p, hsa-miR-361-5p, hsa-miR-362-5p, hsa-miR-363-3p, hsa-miR-3653-3p, hsa-miR-365a-3p, hsa-miR-365b-3p, hsa-miR-3665, hsa-miR-3681-5p, hsa-miR-3689a-5p, hsa-miR-3689b-5p, hsa-miR-3689e, hsa-miR-370-5p, hsa-miR-371a-3p, hsa-miR-371a-5p, hsa-miR-372-3p, hsa-miR-372-5p, hsa-miR-374b-5p, hsa-miR-375, hsa-miR-376c-3p, hsa-miR-380-5p, hsa-miR-3907, hsa-miR-3912-5p, hsa-miR-3960, hsa-miR-3976, hsa-miR-3978, hsa-miR-425-5p, hsa-miR-4266, hsa-miR-429, hsa-miR-4293, hsa-miR-431-5p, hsa-miR-4320, hsa-miR-4325, hsa-miR-433-3p, hsa-miR-4418, hsa-miR-4422, hsa-miR-448, hsa-miR-4492, hsa-miR-4501, hsa-miR-4503, hsa-miR-450a-2-3p, hsa-miR-4519, hsa-miR-452-3p, hsa-miR-4524a-3p, hsa-miR-4643, hsa-miR-4650-3p, hsa-miR-4659a-3p, hsa-miR-4661-5p, hsa-miR-4675, hsa-miR-4677-5p, hsa-miR-4683, hsa-miR-4691-3p, hsa-miR-4694-5p, hsa-miR-4699-3p, hsa-miR-4704-5p, hsa-miR-4719, hsa-miR-4724-3p, hsa-miR-4739, hsa-miR-4747-3p, hsa-miR-4750-3p, hsa-miR-4762-5p, hsa-miR-4774-3p, hsa-miR-4781-5p, hsa-miR-4787-5p, hsa-miR-4799-5p, hsa-miR-4804-5p, hsa-miR-491-3p, hsa-miR-5002-5p, hsa-miR-500a-5p, hsa-miR-5011-5p, hsa-miR-5089-5p, hsa-miR-5092, hsa-miR-509-5p, hsa-miR-512-3p, hsa-miR-516b-3p, hsa-miR-516a-3p, hsa-miR-516b-5p, hsa-miR-517-5p, hsa-miR-518b, hsa-miR-519a-3p, hsa-miR-519b-3p, hsa-miR-520d-3p, hsa-miR-520f-3p, hsa-miR-539-5p, hsa-miR-542-5p, hsa-miR-548ba, hsa-miR-552-3p, hsa-miR-5581-5p, hsa-miR-5588-5p, hsa-miR-5589-3p, hsa-miR-563, hsa-miR-566, hsa-miR-5684, hsa-miR-5696, hsa-miR-5701, hsa-miR-5702, hsa-miR-576-5p, hsa-miR-580-5p, hsa-miR-582-3p, hsa-miR-585-3p, hsa-miR-597-3p, hsa-miR-598-3p, hsa-miR-605-3p, hsa-miR-6082, hsa-miR-611, hsa-miR-6126, hsa-miR-616-3p, hsa-miR-6165, hsa-miR-616-5p, hsa-miR-624-3p, hsa-miR-624-5p, hsa-miR-626, hsa-miR-628-3p, hsa-miR-630, hsa-miR-653-5p, hsa-miR-655-5p, hsa-miR-656-3p, hsa-miR-659-5p, hsa-miR-660-5p, hsa-miR-662, hsa-miR-6715a-3p, hsa-miR-6715b-3p, hsa-miR-671-5p, hsa-miR-6734-3p, hsa-miR-6758-5p, hsa-miR-6768-3p, hsa-miR-6769a-5p, hsa-miR-6773-5p, hsa-miR-6795-3p, hsa-miR-6798-3p, hsa-miR-6799-5p, hsa-miR-6812-5p, hsa-miR-6864-5p, hsa-miR-6869-3p, hsa-miR-6885-5p, hsa-miR-7153-5p, hsa-miR-7154-5p, hsa-miR-7158-3p, hsa-miR-7162-5p, hsa-miR-758-3p, hsa-miR-7843-3p, hsa-miR-8058, hsa-miR-8079, hsa-miR-8086, hsa-miR-891a-3p, hsa-miR-892c-3p, hsa-miR-922, hsa-miR-92b-3p, hsa-miR-933, hsa-miR-93-3p, hsa-miR-96-3p, hsa-miR-96-5p, and hsa-miR-99a-3p in a body fluid sample of the subject as an indicator.

The present disclosure provides a method for detecting a microRNA in a body fluid of a subject (or a method for determining the presence or absence of a microRNA in a body fluid of a subject), including detecting at least one microRNA or all microRNAs selected from the group consisting of hsa-miR-103a-2-5p, hsa-miR-106a-3p, hsa-miR-1185-2-3p, hsa-miR-1193, hsa-miR-1273g-3p, hsa-miR-1293, hsa-miR-1301-5p, hsa-miR-152-5p, hsa-miR-184, hsa-miR-200c-5p, hsa-miR-2909, hsa-miR-298, hsa-miR-302b-3p, hsa-miR-3116, hsa-miR-3122, hsa-miR-3125, hsa-miR-3126-3p, hsa-miR-3129-3p, hsa-miR-3130-3p, hsa-miR-3133, hsa-miR-3137, hsa-miR-3150a-3p, hsa-miR-3174, hsa-miR-3177-5p, hsa-miR-3179, hsa-miR-3186-3p, hsa-miR-3186-5p, hsa-miR-3187-3p, hsa-miR-3189-3p, hsa-miR-3190-3p, hsa-miR-3200-5p, hsa-miR-320a, hsa-miR-320c, hsa-miR-323b-5p, hsa-miR-325, hsa-miR-331-5p, hsa-miR-335-3p, hsa-miR-33a-3p, hsa-miR-342-3p, hsa-miR-34a-5p, hsa-miR-3606-3p, hsa-miR-3616-3p, hsa-miR-3621, hsa-miR-3622a-5p, hsa-miR-3659, hsa-miR-3690, hsa-miR-376b-3p, hsa-miR-3913-3p, hsa-miR-4269, hsa-miR-4303, hsa-miR-4304, hsa-miR-4307, hsa-miR-4316, hsa-miR-4326, hsa-miR-4421, hsa-miR-4472, hsa-miR-4479, hsa-miR-4646-5p, hsa-miR-4660, hsa-miR-4688, hsa-miR-4695-3p, hsa-miR-4695-5p, hsa-miR-4721, hsa-miR-4724-5p, hsa-miR-4738-3p, hsa-miR-4740-3p, hsa-miR-4750-5p, hsa-miR-4753-5p, hsa-miR-4759, hsa-miR-4776-5p, hsa-miR-4778-3p, hsa-miR-4796-5p, hsa-miR-487b-5p, hsa-miR-5008-3p, hsa-miR-514b-5p, hsa-miR-518f-3p, hsa-miR-5194, hsa-miR-541-3p, hsa-miR-548c-3p, hsa-miR-548j-3p, hsa-miR-548n, hsa-miR-548z, hsa-miR-548h-3p, hsa-miR-5572, hsa-miR-5580-5p, hsa-miR-5586-3p, hsa-miR-601, hsa-miR-6068, hsa-miR-6083, hsa-miR-6133, hsa-miR-617, hsa-miR-622, hsa-miR-625-5p, hsa-miR-649, hsa-miR-6504-5p, hsa-miR-6513-5p, hsa-miR-651-5p, hsa-miR-6516-5p, hsa-miR-661, hsa-miR-664a-5p, hsa-miR-665, hsa-miR-6755-3p, hsa-miR-6767-5p, hsa-miR-6777-5p, hsa-miR-6782-5p, hsa-miR-8084, hsa-miR-888-3p, and hsa-miR-942-3p by contacting a body fluid sample obtained from a subject with a detecting agent for a microRNA to be detected. The detecting agent for a microRNA may be a nucleic acid with a sequence complementary to the microRNA, such as a probe with a sequence complementary to the microRNA or a primer with a sequence complementary to the microRNA. If the expression level of a microRNA is high, it may be determined that there is a high likelihood of cancer, and if the expression level is low, it may be determined that there is a low likelihood of cancer. The expression level of a microRNA can be determined on the basis of a predetermined value.

In the present disclosure, when a plurality of microRNAs are used as indicators to examine the likelihood that a subject has cancer or a specific cancer, in some embodiments, each of the plurality of microRNA levels may be compared with a predetermined value, or in some embodiments, a score obtained by weighting the plurality of microRNA levels may be compared with a predetermined value weighted in the same manner. When each of a plurality of microRNA levels is compared with a predetermined value, the number of microRNAs indicating a likelihood of cancer can be compared with the number of microRNAs indicating a likelihood of non-cancer to determine whether the likelihood of cancer is high or whether the likelihood of non-cancer is high. Also in the present disclosure, when a plurality of microRNA levels are weighted to obtain a score (for example, when microRNA signatures are scored), each microRNA level can be normalized and then weighted or not weighted, and the normalized values can be added or multiplied for scoring (for example, to obtain a Z-score). The microRNA score thus obtained can be compared with a predetermined value obtained in the same manner (that is, a score obtained in the same manner from a cancer patient or a non-cancer subject, etc.) to determine whether the likelihood of cancer is high or whether the likelihood of non-cancer is high. The weighting may be positive for a higher level in a body fluid sample of a cancer subject than in a body fluid sample of a non-cancer subject and negative for a lower level in a body fluid sample of a cancer subject than in a body fluid sample of a non-cancer subject (or vice versa). Weighting can also be performed by multiplying a larger value for a larger difference or correlation between a cancer subject and a non-cancer subject. In some embodiments, when a plurality of microRNAs are used as indicators to examine the likelihood that a subject has cancer or a specific cancer, prediction may be performed using a prediction model, such as logistic regression, for the plurality of microRNA levels.

In some embodiments, a machine-learned computer or artificial intelligence may determine the presence or absence of a disease, identify a disease, or calculate the incidence rate of the disease from one or a plurality of microRNA levels. In machine learning or artificial intelligence, a machine (computer) or artificial intelligence (AI) can be trained, for example, by learning one or a plurality of microRNA levels in association with the determination of the presence or absence of a disease, the identification of a disease, and the incidence rate of the disease. Machine learning or artificial intelligence can be trained using
data relating to the relationship between cancer and the expression level(s) of at least 1, 2, 3, 4, 5, 6, 7, 8, 9, 10 or more, 15 or more, or 20 or more, or all microRNAs selected from the group consisting of (i)miR-3117-5p, miR-3118, miR-3121-3p, miR-3121-5p, miR-3126-5p, miR-3128, miR-3133, miR-3134, miR-3136-3p, miR-3136-5p, miR-3139, miR-3142, miR-3143, miR-3145-3p, miR-3163, miR-3166, miR-3167, miR-16-1-3p, miR-424-3p, miR-519c-5p, miR-525-5p, miR-551b-5p, miR-558, miR-921, miR-942-3p, miR-3126-3p, miR-3127-5p, miR-3129-5p, miR-3144-5p, miR-3150a-5p, miR-3152-5p, miR-3155a, miR-3157-3p, miR-3159, miR-3165, miR-3678-3p, miR-4321, miR-4521, miR-4800-3p, miR-4999-5p, miR-5096, miR-5187-5p, miR-6874-5p, miR-3127-3p, miR-3130-5p, miR-3131, miR-3141, miR-3150b-5p, miR-3151-3p, miR-3151-5p, miR-3154, miR-3160-3p, miR-3160-5p, miR-378a-5p, miR-520c-3p, miR-526b-3p, miR-3150a-3p, miR-3162-5p, and miR-4254;
data relating to the relationship between cancer and the expression level(s) of at least 1, 2, 3, 4, 5, 6, 7, 8, 9, 10 or more, 15 or more, or 20 or more, or all microRNAs selected from the group consisting of (ii)let-7i-3p, miR-183-5p, miR-202-5p, miR-409-5p, miR-4661-5p, miR-4800-3p, miR-5587-5p, miR-372-3p, miR-378b, miR-520b, miR-1266-3p, miR-3605-5p, miR-3612, miR-4645-3p, miR-4694-3p, miR-4752, miR-6816-3p, miR-8087, let-7f-2-3p, miR-15a-3p, miR-20a-3p, miR-33b-3p, miR-34c-5p, miR-93-5p, miR-130a-5p, miR-135a-5p, miR-135b-5p, miR-185-5p, miR-203a-3p, miR-302d-5p, miR-337-3p, miR-378c, miR-422a, miR-449c-5p, miR-483-5p, miR-506-3p, miR-511-5p, miR-520c-3p, miR-654-3p, miR-668-5p, miR-670-5p, miR-671-3p, miR-744-3p, miR-1178-3p, miR-1254, miR-1284, miR-1323, miR-2116-5p, miR-2355-3p, miR-3132, miR-3138, miR-3164, miR-3186-3p, miR-3189-3p, miR-3198, miR-3200-5p, miR-3657, miR-3667-5p, miR-3680-5p, miR-3692-5p, miR-3713, miR-3921, miR-3936, miR-4273, miR-4299, miR-4306, miR-4316, miR-4319, miR-4421, miR-4429, miR-4435, miR-4441, miR-4473, miR-4506, miR-4633-5p, miR-4658, miR-4733-5p, miR-4733-3p, miR-5004-3p, miR-5194, miR-5197-5p, miR-5571-5p, miR-6083, miR-6717-5p, miR-6720-5p, miR-6767-3p, miR-6781-3p, miR-6811-3p, miR-6821-3p, miR-6828-5p, miR-6832-5p, miR-6837-3p, miR-6841-5p, miR-6853-5p, miR-6871-3p, miR-6875-5p, miR-6878-5p, miR-7112-3p, miR-7703, miR-7848-3p, and miR-7856-5p;
data relating to the relationship between cancer and the expression level(s) of at least 1, 2, 3, 4, 5, 6, 7, 8, 9, 10 or more, 15 or more, or 20 or more, or all microRNAs selected from the group consisting of (iii)miR-4521, let-7c-3p, let-7i-5p, miR-16-1-3p, miR-26a-1-3p, miR-28-5p, miR-105-5p, miR-195-3p, miR-200b-5p, miR-219a-2-3p, miR-297, miR-300, miR-330-3p, miR-374b-5p, miR-431-5p, miR-454-5p, miR-513c-5p, miR-548ax, miR-593-5p, miR-623, miR-664a-5p, miR-942-3p, miR-1205, miR-1276, miR-1288-3p, miR-1297, miR-3678-3p, miR-4283, miR-4295, miR-4439, miR-4524b-5p, miR-4703-3p, miR-4768-5p, miR-4800-3p, miR-5187-5p, miR-5696, miR-7161-5p, let-7i-2-3p, and miR-520c-3p;
data relating to the relationship between cancer and the expression level(s) of at least 1, 2, 3, 4, 5, 6, 7, 8, 9, 10 or more, 15 or more, or 20 or more, or all microRNAs selected from the group consisting of (iv)miR-16-1-3p, miR-23b-3p, miR-28-5p, miR-92a-2-5p, miR-142-3p, miR-195-3p, miR-196b-5p, miR-299-3p, miR-492, miR-513b-5p, miR-601, miR-619-5p, miR-1285-3p, miR-3155a, miR-3162-5p, miR-3678-3p, miR-4283, miR-4295, miR-4311, miR-4531, miR-5096, miR-5187-5p, let-7f-2-3p, miR-520c-3p, and miR-4783-5p; or
data relating to the relationship between cancer and the expression level(s) of at least 1, 2, 3, 4, 5, 6, 7, 8, 9, 10 or more, 15 or more, or 20 or more, or all microRNAs selected from the group consisting of (v)miR-4531, miR-28-5p, miR-103a-2-5p, miR-105-5p, miR-124-3p, miR-151a-5p, miR-151b, miR-200a-5p, miR-300, miR-424-3p, miR-519c-5p, miR-551b-5p, miR-617, miR-873-3p, miR-921, miR-1288-3p, miR-3124-5p, miR-3155a, miR-3917, miR-4283, miR-4727-3p, miR-5096, miR-5187-5p, miR-6074, miR-6874-5p, miR-6892-5p, miR-15a-3p, miR-135b-5p, miR-520c-3p, miR-4783-5p, and miR-7849-3p;
data relating to the relationship between cancer and the expression level(s) of at least 1, 2, 3, 4, 5, 6, 7, 8, 9, 10 or more, 15 or more, or 20 or more, or all microRNAs selected from the group consisting of (vi)hsa-let-7a-3p, hsa-let-7g-5p, hsa-miR-100-3p, hsa-miR-101-3p, hsa-miR-105-3p, hsa-miR-10b-3p, hsa-miR-1185-5p, hsa-miR-1197, hsa-miR-1206, hsa-miR-1243, hsa-miR-1245b-3p, hsa-miR-1246, hsa-miR-1252-3p, hsa-miR-1252-5p, hsa-miR-1258, hsa-miR-126-3p, hsa-miR-126-5p, hsa-miR-1277-3p, hsa-miR-1277-5p, hsa-miR-1279, hsa-miR-1295b-5p, hsa-miR-136-5p, hsa-miR-1-3p, hsa-miR-145-3p, hsa-miR-1468-3p, hsa-miR-152-3p, hsa-miR-153-3p, hsa-miR-155-5p, hsa-miR-186-5p, hsa-miR-18a-5p, hsa-miR-190a-5p, hsa-miR-190b, hsa-miR-19a-5p, hsa-miR-19b-1-5p, hsa-miR-19b-2-5p, hsa-miR-19b-3p, hsa-miR-2053, hsa-miR-205-3p, hsa-miR-2054, hsa-miR-208a-3p, hsa-miR-20a-5p, hsa-miR-219a-1-3p, hsa-miR-219b-3p, hsa-miR-301a-3p, hsa-miR-30d-3p, hsa-miR-30e-5p, hsa-miR-3118, hsa-miR-3123, hsa-miR-3129-5p, hsa-miR-3135a, hsa-miR-3140-3p, hsa-miR-3143, hsa-miR-3152-3p, hsa-miR-3167, hsa-miR-3169, hsa-miR-3183, hsa-miR-3201, hsa-miR-335-5p, hsa-miR-339-3p, hsa-miR-3607-5p, hsa-miR-3616-5p, hsa-miR-3617-5p, hsa-miR-3618, hsa-miR-3662, hsa-miR-3668, hsa-miR-3683, hsa-miR-3686, hsa-miR-3688-3p, hsa-miR-369-3p, hsa-miR-374a-3p, hsa-miR-374a-5p, hsa-miR-376a-5p, hsa-miR-3927-3p, hsa-miR-3942-5p, hsa-miR-4277, hsa-miR-4302, hsa-miR-4432, hsa-miR-4452, hsa-miR-4457, hsa-miR-4474-5p, hsa-miR-4477b, hsa-miR-4480, hsa-miR-4495, hsa-miR-4504, hsa-miR-4509, hsa-miR-450a-1-3p, hsa-miR-450b-3p, hsa-miR-455-5p, hsa-miR-4633-3p, hsa-miR-4639-5p, hsa-miR-4668-3p, hsa-miR-4671-3p, hsa-miR-4679, hsa-miR-4693-3p, hsa-miR-4699-5p, hsa-miR-4704-3p, hsa-miR-4714-3p, hsa-miR-4720-3p, hsa-miR-4735-5p, hsa-miR-4757-3p, hsa-miR-4760-5p, hsa-miR-4772-5p, hsa-miR-4777-5p, hsa-miR-4781-3p, hsa-miR-4782-3p, hsa-miR-4782-5p, hsa-miR-4790-3p, hsa-miR-4791, hsa-miR-4795-5p, hsa-miR-4796-3p, hsa-miR-4798-5p, hsa-miR-4799-3p, hsa-miR-4802-5p, hsa-miR-499a-5p, hsa-miR-5009-3p, hsa-miR-5009-5p, hsa-miR-506-5p, hsa-miR-507, hsa-miR-510-3p, hsa-miR-513b-3p, hsa-miR-514a-5p, hsa-miR-5191, hsa-miR-544a, hsa-miR-545-5p, hsa-miR-548a-3p, hsa-miR-548a-5p, hsa-miR-548aa, hsa-miR-548t-3p, hsa-miR-548ac, hsa-miR-548ad-5p, hsa-miR-548ae-5p, hsa-miR-548ae-3p, hsa-miR-548ag, hsa-miR-548ah-5p, hsa-miR-548ak, hsa-miR-548al, hsa-miR-548am-3p, hsa-miR-548ao-5p, hsa-miR-548ap-5p, hsa-miR-548aq-5p, hsa-miR-548at-3p, hsa-miR-548au-5p, hsa-miR-548az-5p, hsa-miR-548b-5p, hsa-miR-548c-5p, hsa-miR-548o-5p, hsa-miR-548am-5p, hsa-miR-548g-5p, hsa-miR-548x-5p, hsa-miR-548aj-5p, hsa-miR-548j-5p, hsa-miR-548k, hsa-miR-548m, hsa-miR-548p, hsa-miR-548t-5p, hsa-miR-548u, hsa-miR-548v, hsa-miR-548x-3p, hsa-miR-549a, hsa-miR-553, hsa-miR-556-3p, hsa-miR-5579-5p, hsa-miR-5583-5p, hsa-miR-559, hsa-miR-5590-3p, hsa-miR-5590-5p, hsa-miR-5591-5p, hsa-miR-561-5p, hsa-miR-568, hsa-miR-5687, hsa-miR-569, hsa-miR-5692c, hsa-miR-5697, hsa-miR-5700, hsa-miR-570-3p, hsa-miR-577, hsa-miR-582-5p, hsa-miR-587, hsa-miR-590-3p, hsa-miR-6079, hsa-miR-6128, hsa-miR-618, hsa-miR-621, hsa-miR-628-5p, hsa-miR-633, hsa-miR-648, hsa-miR-6508-3p, hsa-miR-651-3p, hsa-miR-652-3p, hsa-miR-6733-5p, hsa-miR-6811-5p, hsa-miR-6844, hsa-miR-6854-5p, hsa-miR-7161-3p, hsa-miR-759, hsa-miR-7-5p, hsa-miR-7852-3p, hsa-miR-873-5p, hsa-miR-876-5p, hsa-miR-9-3p, hsa-miR-95-3p, hsa-miR-95-5p, hsa-miR-9-5p, hsa-miR-4525, hsa-miR-6760-5p, hsa-miR-4538, hsa-miR-5698, hsa-miR-5189-5p, hsa-miR-671-5p, hsa-miR-936, hsa-miR-4307, hsa-miR-6815-5p, hsa-miR-6076, hsa-miR-708-5p, hsa-miR-3187-5p, hsa-miR-3144-3p, hsa-miR-4691-5p, hsa-miR-4700-5p, hsa-miR-576-5p, hsa-miR-3652, hsa-miR-598-3p, hsa-miR-7151-3p, hsa-miR-7162-5p, hsa-miR-1229-5p, hsa-miR-6746-5p, hsa-miR-4762-5p, hsa-miR-1307-3p, hsa-miR-1273g-3p, hsa-miR-4428, hsa-miR-6515-5p, hsa-miR-1273g-5p, hsa-miR-433-3p, hsa-miR-452-3p, hsa-miR-3917, hsa-miR-1224-5p, hsa-miR-6892-5p, hsa-miR-520d-5p, hsa-miR-5092, hsa-miR-30c-1-3p, hsa-miR-1293, hsa-miR-7851-3p, hsa-miR-3934-5p, hsa-miR-3122, hsa-miR-3177-3p, hsa-miR-4800-5p, hsa-miR-4436b-3p, hsa-miR-3928-3p, hsa-miR-630, hsa-miR-628-3p, hsa-miR-6796-5p, hsa-miR-3605-5p, hsa-miR-30e-3p, hsa-miR-6801-5p, hsa-miR-6131, hsa-miR-448, hsa-miR-6834-5p, hsa-miR-3156-5p, hsa-miR-517-5p, hsa-miR-4520-5p, hsa-miR-2276-3p, hsa-miR-3591-3p, hsa-miR-4727-3p, hsa-miR-4499, hsa-miR-1471, hsa-miR-1273h-5p, hsa-miR-4539, hsa-miR-3655, hsa-miR-4690-5p, hsa-miR-6871-5p, hsa-miR-4496, hsa-miR-6772-5p, hsa-miR-4686, hsa-miR-1297, hsa-miR-450a-2-3p, hsa-miR-6745, hsa-miR-3153, hsa-miR-27a-3p, hsa-miR-106b-5p, hsa-miR-519c-5p, hsa-miR-523-5p, hsa-miR-518e-5p, hsa-miR-522-5p, hsa-miR-519a-5p, hsa-miR-519b-5p, hsa-miR-4676-5p, hsa-miR-650, hsa-miR-3922-5p, hsa-miR-4489, hsa-miR-4638-5p, hsa-miR-185-3p, hsa-miR-2467-3p, hsa-miR-4453, hsa-let-7e-5p, hsa-miR-296-3p, hsa-miR-8074, hsa-miR-6876-5p, hsa-miR-6086, hsa-miR-4474-3p, hsa-miR-4776-3p, hsa-miR-1250-5p, hsa-miR-4260, hsa-miR-6747-5p, hsa-miR-181d-3p, hsa-miR-4535, hsa-miR-3177-5p, hsa-miR-4533, hsa-miR-4635, hsa-miR-4776-5p, hsa-miR-4257, hsa-miR-3160-3p, hsa-miR-5702, hsa-miR-4306, hsa-miR-4483, hsa-miR-4472, hsa-miR-3064-3p, hsa-miR-4710, hsa-miR-4293, hsa-miR-449c-5p, hsa-miR-5589-5p, hsa-miR-4443, hsa-miR-4448, hsa-miR-3935, hsa-miR-16-5p, hsa-miR-181a-5p, hsa-miR-147b, hsa-miR-4767, hsa-miR-4677-5p, hsa-miR-6776-5p, hsa-miR-7109-5p, hsa-miR-4669, hsa-miR-4486, hsa-miR-1587, hsa-miR-363-3p, hsa-miR-5701, hsa-miR-921, hsa-miR-5581-5p, hsa-miR-4259, hsa-miR-203b-5p, hsa-miR-3653-3p, hsa-miR-4657, hsa-miR-5589-3p, hsa-miR-2681-3p, hsa-miR-6826-5p, hsa-miR-4647, hsa-miR-4540, hsa-miR-361-5p, hsa-miR-4784, hsa-miR-6861-5p, hsa-miR-6812-5p, hsa-miR-608, hsa-miR-1912, hsa-miR-554, hsa-miR-519a-3p, hsa-miR-891a-3p, hsa-miR-6872-5p, hsa-miR-4781-5p, hsa-miR-4273, hsa-miR-4769-5p, hsa-miR-505-5p, hsa-miR-7158-3p, hsa-miR-527, hsa-miR-518a-5p, hsa-miR-1273f, hsa-miR-624-5p, hsa-miR-125a-3p, hsa-miR-6830-5p, hsa-miR-520d-3p, hsa-miR-6757-5p, hsa-miR-4785, hsa-miR-6068, hsa-miR-1272, hsa-miR-4654, hsa-miR-564, hsa-miR-563, hsa-miR-4300, hsa-miR-4665-5p, hsa-miR-892c-3p, hsa-miR-6778-5p, hsa-miR-4419b, hsa-miR-329-3p, hsa-miR-212-5p, hsa-miR-616-3p, hsa-miR-4269, hsa-miR-4487, hsa-miR-1180-5p, hsa-miR-7850-5p, hsa-miR-492, hsa-miR-718, hsa-miR-1972, hsa-miR-4296, hsa-miR-516b-3p, hsa-miR-516a-3p, hsa-miR-4320, hsa-miR-4529-5p, hsa-miR-656-3p, hsa-miR-6500-3p, hsa-miR-766-5p, hsa-miR-466, hsa-miR-6777-5p, hsa-miR-1909-5p, hsa-miR-187-3p, hsa-miR-8079, hsa-miR-30a-5p, hsa-miR-5002-3p, hsa-miR-6833-5p, hsa-miR-4756-3p, hsa-let-7f-2-3p, hsa-miR-5708, hsa-miR-4721, hsa-miR-8085, hsa-miR-3591-5p, hsa-miR-6859-5p, hsa-miR-5006-5p, hsa-miR-4747-5p, hsa-miR-6827-5p, hsa-miR-3612, hsa-miR-4701-3p, hsa-miR-4418, hsa-miR-4449, hsa-miR-181d-5p, hsa-miR-3619-3p, hsa-miR-6817-5p, hsa-miR-6759-5p, hsa-miR-3654, hsa-miR-3164, hsa-miR-491-3p, hsa-miR-3176, hsa-miR-4683, hsa-miR-6807-5p, hsa-miR-5588-5p, hsa-miR-302c-5p, hsa-miR-4522, hsa-miR-1199-5p, hsa-miR-372-3p, hsa-miR-891a-5p, hsa-miR-1306-3p, hsa-miR-6894-5p, hsa-miR-3613-3p, hsa-miR-548ba, hsa-miR-6795-5p, hsa-miR-6794-5p, hsa-miR-585-3p, hsa-miR-194-3p, hsa-miR-5010-5p, hsa-miR-2052, hsa-miR-614, hsa-miR-6751-5p, hsa-miR-5089-3p, hsa-miR-1289, hsa-miR-668-5p, hsa-miR-3622b-5p, hsa-miR-6887-5p, hsa-miR-4524a-3p, hsa-miR-3689a-3p, hsa-miR-4650-5p, hsa-miR-3689b-3p, hsa-miR-3689c, hsa-miR-624-3p, hsa-miR-6129, hsa-miR-216a-5p, hsa-miR-520b, hsa-miR-4755-5p, hsa-miR-323a-5p, hsa-miR-6726-5p, hsa-miR-516b-5p, hsa-miR-7106-5p, hsa-miR-653-5p, hsa-miR-494-5p, hsa-miR-6780b-5p, hsa-miR-1910-3p, hsa-miR-3160-5p, hsa-miR-5093, hsa-miR-99a-3p, hsa-miR-8071, hsa-miR-4659a-3p, hsa-miR-597-3p, hsa-miR-4458, hsa-miR-6847-5p, hsa-miR-4436a, hsa-miR-17-5p, hsa-miR-185-5p, hsa-miR-4681, hsa-miR-8077, hsa-miR-4440, hsa-let-7g-3p, hsa-miR-375, hsa-miR-10a-5p, hsa-miR-520e, hsa-miR-6072, hsa-miR-3145-5p, hsa-miR-6723-5p, hsa-miR-4692, hsa-miR-4796-5p, hsa-miR-6849-5p, hsa-miR-4430, hsa-miR-6499-5p, hsa-miR-3151-5p, hsa-miR-182-5p, hsa-miR-195-5p, hsa-miR-3681-5p, hsa-miR-6864-5p, hsa-miR-4711-3p, hsa-miR-380-3p, hsa-miR-374c-5p, hsa-miR-758-5p, hsa-miR-6842-5p, hsa-miR-8080, hsa-miR-520f-3p, hsa-miR-4646-5p, hsa-miR-103a-3p, hsa-miR-3189-3p, hsa-miR-4691-3p, hsa-miR-877-5p, hsa-miR-6769b-5p, hsa-miR-5580-5p, hsa-miR-1911-5p, hsa-miR-519b-3p, hsa-miR-660-5p, hsa-miR-4644, hsa-miR-6133, hsa-miR-138-1-3p, hsa-miR-193a-5p, hsa-miR-222-3p, hsa-miR-603, hsa-miR-23b-3p, hsa-miR-5089-5p, hsa-miR-6799-5p, hsa-miR-6715a-3p, hsa-miR-6869-3p, hsa-miR-4445-3p, hsa-miR-542-5p, hsa-miR-1286, hsa-miR-96-3p, hsa-miR-4743-5p, hsa-miR-4724-3p, hsa-miR-4262, hsa-miR-4507, hsa-miR-7160-5p, hsa-miR-605-3p, hsa-miR-200c-3p, hsa-miR-582-3p, hsa-miR-6868-5p, hsa-miR-6773-5p, hsa-miR-1185-1-3p, hsa-miR-3182, hsa-miR-6082, hsa-miR-150-3p, hsa-miR-5002-5p, hsa-miR-487a-5p, hsa-miR-8058, hsa-miR-4529-3p, hsa-miR-3713, hsa-miR-8075, hsa-miR-6877-5p, hsa-miR-4325, hsa-miR-3130-5p, hsa-miR-616-5p, hsa-miR-1322, hsa-miR-6793-5p, hsa-miR-4694-5p, hsa-miR-29a-3p, hsa-miR-6127, hsa-miR-758-3p, hsa-miR-2114-5p, hsa-miR-515-5p, hsa-miR-196a-5p, hsa-miR-4639-3p, hsa-miR-4799-5p, hsa-miR-4498, hsa-miR-6766-5p, hsa-miR-1255b-2-3p, hsa-miR-380-5p, hsa-miR-4419a, hsa-miR-3622a-5p, hsa-let-7b-5p, hsa-miR-4650-3p, hsa-miR-29b-1-5p, hsa-miR-3137, hsa-miR-4447, hsa-miR-6500-5p, hsa-miR-3663-5p, hsa-miR-4451, hsa-miR-4422, hsa-miR-662, hsa-miR-122-3p, hsa-miR-6828-5p, hsa-miR-6884-5p, hsa-miR-4502, hsa-miR-374b-5p, hsa-miR-4462, hsa-miR-4478, hsa-miR-431-3p, hsa-miR-23b-5p, hsa-miR-7975, hsa-miR-5011-5p, hsa-miR-3691-5p, hsa-miR-6768-3p, hsa-miR-591, hsa-miR-8086, hsa-miR-372-5p, hsa-miR-5189-3p, hsa-miR-107, hsa-miR-6891-5p, hsa-miR-3138, hsa-miR-6503-3p, hsa-miR-297, hsa-miR-370-5p, hsa-miR-3944-3p, hsa-miR-425-5p, hsa-miR-3978, hsa-miR-148a-5p, hsa-miR-4684-5p, hsa-miR-34a-3p, hsa-miR-1263, hsa-miR-769-5p, hsa-miR-4437, hsa-miR-2277-3p, hsa-miR-659-5p, hsa-miR-661, hsa-miR-6788-5p, hsa-miR-15b-3p, hsa-miR-1205, hsa-miR-1468-5p, hsa-miR-34a-5p, hsa-miR-3120-5p, hsa-miR-1247-3p, hsa-miR-454-5p, hsa-miR-656-5p, hsa-miR-626, hsa-miR-4441, hsa-miR-6780a-5p, hsa-miR-6769a-5p, hsa-miR-1298-5p, hsa-miR-3157-5p, hsa-miR-6814-5p, hsa-miR-500a-5p, hsa-miR-1321, hsa-miR-4481, hsa-miR-611, hsa-miR-1273a, hsa-miR-337-5p, hsa-miR-203a-5p, hsa-miR-4479, hsa-miR-4719, hsa-miR-8083, hsa-miR-132-3p, hsa-miR-20b-5p, hsa-miR-4252, hsa-miR-429, hsa-miR-378b, hsa-miR-134-5p, hsa-miR-6741-5p, hsa-miR-1193, hsa-miR-143-5p, hsa-miR-515-3p, hsa-miR-4774-3p, hsa-miR-8064, hsa-miR-1183, hsa-miR-5684, hsa-miR-1226-5p, hsa-miR-539-5p, hsa-miR-580-5p, hsa-miR-622, hsa-miR-3907, hsa-miR-6504-5p, hsa-miR-4653-3p, hsa-miR-4706, hsa-miR-4515, hsa-miR-3919, hsa-miR-3187-3p, hsa-miR-1271-5p, hsa-miR-6767-5p, hsa-miR-4804-5p, hsa-miR-1257, hsa-miR-3126-5p, hsa-miR-6731-5p, hsa-miR-3149, hsa-miR-4717-3p, hsa-miR-509-5p, hsa-miR-215-3p, hsa-miR-1178-3p, hsa-miR-1282, hsa-miR-3174, hsa-miR-197-5p, hsa-miR-376c-3p, hsa-miR-5696, hsa-miR-3121-5p, hsa-miR-8082, hsa-miR-6823-5p, hsa-miR-4704-5p, hsa-miR-3616-3p, hsa-miR-6881-5p, hsa-miR-4661-5p, hsa-miR-22-5p, hsa-miR-6730-5p, hsa-let-7e-3p, hsa-miR-1238-5p, hsa-miR-526b-5p, hsa-miR-3163, hsa-miR-4514, hsa-miR-4699-3p, hsa-miR-7154-5p, hsa-miR-4521, hsa-miR-96-5p, hsa-miR-4754, hsa-miR-450a-5p, hsa-miR-4459, hsa-miR-922, hsa-miR-7153-5p, hsa-miR-29b-2-5p, hsa-miR-6738-3p, hsa-miR-6790-5p, hsa-miR-4470, hsa-miR-4732-3p, hsa-miR-4709-3p, hsa-miR-5588-3p, hsa-miR-93-3p, hsa-miR-6820-5p, hsa-miR-655-5p, hsa-miR-6514-3p, hsa-miR-1-5p, hsa-miR-10b-5p, hsa-miR-3193, hsa-miR-3976, hsa-miR-3678-3p, hsa-miR-3912-5p, hsa-miR-4708-3p, hsa-miR-5192, hsa-miR-140-5p, hsa-miR-4285, hsa-miR-3124-5p, hsa-miR-198, hsa-miR-3659, hsa-miR-3689a-5p, hsa-miR-3689b-5p, hsa-miR-3689e, hsa-miR-4643, hsa-miR-552-3p, hsa-miR-3186-3p, hsa-miR-424-3p, hsa-miR-578, hsa-miR-4304, hsa-miR-504-3p, hsa-miR-595, hsa-miR-4501, hsa-miR-4746-3p, hsa-miR-362-5p, hsa-miR-4297, hsa-miR-6821-3p, hsa-miR-3074-5p, hsa-miR-4503, hsa-miR-4518, hsa-miR-6715b-3p, hsa-miR-6736-5p, hsa-miR-7843-3p, hsa-miR-4420, hsa-miR-3927-5p, hsa-miR-7157-5p;
data relating to the relationship between cancer and the expression level(s) of at least 1, 2, 3, 4, 5, 6, 7, 8, 9, 10 or more, 15 or more, or 20 or more, or all microRNAs selected from the group consisting of (vii)hsa-let-7e-5p, hsa-let-7f-2-3p, hsa-let-7g-3p, hsa-miR-103a-3p, hsa-miR-106b-5p, hsa-miR-107, hsa-miR-10a-5p, hsa-miR-10b-5p, hsa-miR-1180-5p, hsa-miR-1183, hsa-miR-1200, hsa-miR-1205, hsa-miR-122-3p, hsa-miR-1247-3p, hsa-miR-1257, hsa-miR-1263, hsa-miR-1272, hsa-miR-1273a, hsa-miR-1275, hsa-miR-1282, hsa-miR-128-2-5p, hsa-miR-1289, hsa-miR-1297, hsa-miR-1298-5p, hsa-miR-132-3p, hsa-miR-133a-3p, hsa-miR-134-5p, hsa-miR-135b-5p, hsa-miR-140-5p, hsa-miR-143-5p, hsa-miR-1470, hsa-miR-147b, hsa-miR-148a-5p, hsa-miR-150-5p, hsa-miR-15a-3p, hsa-miR-15b-3p, hsa-miR-1-5p, hsa-miR-16-5p, hsa-miR-17-5p, hsa-miR-181a-5p, hsa-miR-181d-3p, hsa-miR-181d-5p, hsa-miR-182-5p, hsa-miR-1911-5p, hsa-miR-1914-3p, hsa-miR-193b-3p, hsa-miR-195-3p, hsa-miR-195-5p, hsa-miR-196a-5p, hsa-miR-198, hsa-miR-200c-3p, hsa-miR-203b-5p, hsa-miR-2052, hsa-miR-205-5p, hsa-miR-208a-5p, hsa-miR-20a-3p, hsa-miR-20b-5p, hsa-miR-2114-5p, hsa-miR-215-3p, hsa-miR-216a-5p, hsa-miR-222-3p, hsa-miR-223-5p, hsa-miR-22-5p, hsa-miR-23a-3p, hsa-miR-23b-5p, hsa-miR-2681-3p, hsa-miR-27a-3p, hsa-miR-2861, hsa-miR-29a-3p, hsa-miR-29b-1-5p, hsa-miR-29b-2-5p, hsa-miR-30a-5p, hsa-miR-30e-3p, hsa-miR-3121-5p, hsa-miR-3127-3p, hsa-miR-3157-5p, hsa-miR-3162-3p, hsa-miR-3163, hsa-miR-3181, hsa-miR-3182, hsa-miR-3184-5p, hsa-miR-3188, hsa-miR-326, hsa-miR-328-5p, hsa-miR-329-3p, hsa-miR-337-5p, hsa-miR-34a-3p, hsa-miR-3591-5p, hsa-miR-3613-3p, hsa-miR-361-5p, hsa-miR-362-5p, hsa-miR-363-3p, hsa-miR-3653-3p, hsa-miR-365a-3p, hsa-miR-365b-3p, hsa-miR-3665, hsa-miR-3681-5p, hsa-miR-3689a-5p, hsa-miR-3689b-5p, hsa-miR-3689e, hsa-miR-3 70-5p, hsa-miR-371a-3p, hsa-miR-371a-5p, hsa-miR-372-3p, hsa-miR-372-5p, hsa-miR-374b-5p, hsa-miR-375, hsa-miR-376c-3p, hsa-miR-380-5p, hsa-miR-3907, hsa-miR-3912-5p, hsa-miR-3960, hsa-miR-3976, hsa-miR-3978, hsa-miR-425-5p, hsa-miR-4266, hsa-miR-429, hsa-miR-4293, hsa-miR-431-5p, hsa-miR-4320, hsa-miR-4325, hsa-miR-433-3p, hsa-miR-4418, hsa-miR-4422, hsa-miR-448, hsa-miR-4492, hsa-miR-4501, hsa-miR-4503, hsa-miR-450a-2-3p, hsa-miR-4519, hsa-miR-452-3p, hsa-miR-4524a-3p, hsa-miR-4643, hsa-miR-4650-3p, hsa-miR-4659a-3p, hsa-miR-4661-5p, hsa-miR-4675, hsa-miR-4677-5p, hsa-miR-4683, hsa-miR-4691-3p, hsa-miR-4694-5p, hsa-miR-4699-3p, hsa-miR-4704-5p, hsa-miR-4719, hsa-miR-4724-3p, hsa-miR-4739, hsa-miR-4747-3p, hsa-miR-4750-3p, hsa-miR-4762-5p, hsa-miR-4774-3p, hsa-miR-4781-5p, hsa-miR-4787-5p, hsa-miR-4799-5p, hsa-miR-4804-5p, hsa-miR-491-3p, hsa-miR-5002-5p, hsa-miR-500a-5p, hsa-miR-5011-5p, hsa-miR-5089-5p, hsa-miR-5092, hsa-miR-509-5p, hsa-miR-512-3p, hsa-miR-516b-3p, hsa-miR-516a-3p, hsa-miR-516b-5p, hsa-miR-517-5p, hsa-miR-518b, hsa-miR-519a-3p, hsa-miR-519b-3p, hsa-miR-520d-3p, hsa-miR-520f-3p, hsa-miR-539-5p, hsa-miR-542-5p, hsa-miR-548ba, hsa-miR-552-3p, hsa-miR-5581-5p, hsa-miR-5588-5p, hsa-miR-5589-3p, hsa-miR-563, hsa-miR-566, hsa-miR-5684, hsa-miR-5696, hsa-miR-5701, hsa-miR-5702, hsa-miR-576-5p, hsa-miR-580-5p, hsa-miR-582-3p, hsa-miR-585-3p, hsa-miR-597-3p, hsa-miR-598-3p, hsa-miR-605-3p, hsa-miR-6082, hsa-miR-611, hsa-miR-6126, hsa-miR-616-3p, hsa-miR-6165, hsa-miR-616-5p, hsa-miR-624-3p, hsa-miR-624-5p, hsa-miR-626, hsa-miR-628-3p, hsa-miR-630, hsa-miR-653-5p, hsa-miR-655-5p, hsa-miR-656-3p, hsa-miR-659-5p, hsa-miR-660-5p, hsa-miR-662, hsa-miR-6715a-3p, hsa-miR-6715b-3p, hsa-miR-671-5p, hsa-miR-6734-3p, hsa-miR-6758-5p, hsa-miR-6768-3p, hsa-miR-6769a-5p, hsa-miR-6773-5p, hsa-miR-6795-3p, hsa-miR-6798-3p, hsa-miR-6799-5p, hsa-miR-6812-5p, hsa-miR-6864-5p, hsa-miR-6869-3p, hsa-miR-6885-5p, hsa-miR-7153-5p, hsa-miR-7154-5p, hsa-miR-7158-3p, hsa-miR-7162-5p, hsa-miR-758-3p, hsa-miR-7843-3p, hsa-miR-8058, hsa-miR-8079, hsa-miR-8086, hsa-miR-891a-3p, hsa-miR-892c-3p, hsa-miR-922, hsa-miR-92b-3p, hsa-miR-933, hsa-miR-93-3p, hsa-miR-96-3p, hsa-miR-96-5p, and hsa-miR-99a-3p;
(viii) data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 4-2; data relating to the relationship between cancer and the expression level(s) of at least 1, 2, 3, 4, 5, 6, 7, 8, 9, 10 or more, 15 or more, or 20 or more, or all microRNAs selected from the group consisting of microRNAs with p < 0.005 or less in Table 4-2; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 22-2; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 23-2; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 24-2; or data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 25-2;
(ix) data relating to the relationship between cancer and the expression level(s) of at least 1, 2, 3, 4, 5, 6, 7, 8, 9, 10 or more, 15 or more, or 20 or more, or all microRNAs selected from the group consisting of Table 4-3; data relating to the relationship between cancer and the expression level(s) of at least 1, 2, 3, 4, 5, 6, 7, 8, 9, 10 or more, 15 or more, or 20 or more, or all microRNAs selected from the group consisting of microRNAs with p < 0.005 or less in Table 4-3; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 22-3; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 23-3; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 24-3; or data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 25-3;
(x) data relating to the relationship between cancer and the expression level(s) of at least 1, 2, 3, 4, 5, 6, 7, 8, 9, 10 or more, 15 or more, or 20 or more, or all microRNAs selected from the group consisting of Table 4-4; data relating to the relationship between cancer and the expression level(s) of at least 1, 2, 3, 4, 5, 6, 7, 8, 9, 10 or more, 15 or more, or 20 or more, or all microRNAs selected from the group consisting of microRNAs with p < 0.005 or less in Table 4-4; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 24-4; or data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 25-4;
(xi) data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 4-5; data relating to the relationship between cancer and the expression level(s) of at least 1, 2, 3, 4, 5, 6, 7, 8, 9, 10 or more, 15 or more, or 20 or more, or all microRNAs selected from the group consisting of microRNAs with p < 0.005 or less in Table 4-5; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 23-4; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 24-5; or data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 25-5;
(xii) data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 4-4; data relating to the relationship between cancer and the expression level(s) of at least 1, 2, 3, 4, 5, 6, 7, 8, 9, 10 or more, 15 or more, or 20 or more, or all microRNAs selected from the group consisting of microRNAs with p < 0.005 or less in Table 4-6; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 22-4; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 23-5; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 24-6; or data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 25-6;
(xiii) data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 4-7; data relating to the relationship between cancer and the expression level(s) of at least 1, 2, 3, 4, 5, 6, 7, 8, 9, 10 or more, 15 or more, or 20 or more, or all microRNAs selected from the group consisting of microRNAs with p < 0.005 or less in Table 4-7; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 22-5; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 23-6; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 24-7; or data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 25-7;
(xiv) data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 4-8; data relating to the relationship between cancer and the expression level(s) of at least 1, 2, 3, 4, 5, 6, 7, 8, 9, 10 or more, 15 or more, or 20 or more, or all microRNAs selected from the group consisting of microRNAs with p < 0.005 or less in Table 4-8; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 22-6; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 23-7; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 24-8; or data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 25-8;
(xv) data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 4-9; data relating to the relationship between cancer and the expression level(s) of at least 1, 2, 3, 4, 5, 6, 7, 8, 9, 10 or more, 15 or more, or 20 or more, or all microRNAs selected from the group consisting of microRNAs with p < 0.005 or less in Table 4-9; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 22-7; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 23-8; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 24-9; or data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 25-9;
(xvi) data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 4-10; data relating to the relationship between cancer and the expression level(s) of at least 1, 2, 3, 4, 5, 6, 7, 8, 9, 10 or more, 15 or more, or 20 or more, or all microRNAs selected from the group consisting of microRNAs with p < 0.005 or less in Table 4-10; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 22-8; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 23-9; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 24-10; or data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 25-10;
(xvii) data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 4-11; data relating to the relationship between cancer and the expression level(s) of at least 1, 2, 3, 4, 5, 6, 7, 8, 9, 10 or more, 15 or more, or 20 or more, or all microRNAs selected from the group consisting of microRNAs with p < 0.005 or less in Table 4-11; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 22-9; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 23-10; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 24-11; or data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 25-11;
(xviii) data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 4-12; data relating to the relationship between cancer and the expression level(s) of at least 1, 2, 3, 4, 5, 6, 7, 8, 9, 10 or more, 15 or more, or 20 or more, or all microRNAs selected from the group consisting of microRNAs with p < 0.005 or less in Table 4-12; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 22-10; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 23-11; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 24-12; or data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 25-12;
(xix) data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 4-13; data relating to the relationship between cancer and the expression level(s) of at least 1, 2, 3, 4, 5, 6, 7, 8, 9, 10 or more, 15 or more, or 20 or more, or all microRNAs selected from the group consisting of microRNAs with p < 0.005 or less in Table 4-13; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 22-11; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 23-12; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 24-13; or data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 25-13;
(xx) data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 4-14; data relating to the relationship between cancer and the expression level(s) of at least 1, 2, 3, 4, 5, 6, 7, 8, 9, 10 or more, 15 or more, or 20 or more, or all microRNAs selected from the group consisting of microRNAs with p < 0.005 or less in Table 4-14; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 22-12; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 23-13; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 24-14; or data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 25-14;
(xxi) data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 4-15; data relating to the relationship between cancer and the expression level(s) of at least 1, 2, 3, 4, 5, 6, 7, 8, 9, 10 or more, 15 or more, or 20 or more, or all microRNAs selected from the group consisting of microRNAs with p < 0.005 or less in Table 4-15; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 22-13; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 23-14; data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 24-15; or data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Table 25-15;
(xxii) data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in the group (ALL); data relating to the relationship between cancer and the expression level(s) of at least 1, 2, 3, 4, 5, 6, 7, 8, 9, 10 or more, 15 or more, or 20 or more, or all microRNAs selected from the group consisting of microRNAs with p < 0.005 or less in the group (ALL); data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 22-1 to 22-13 in the group (ALL); data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 22-1 and 23-1 to 23-14 in the group (ALL); data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 24-1 to 24-15 in the group (ALL); or data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 25-1 to 25-15 in the group (ALL);
(xxiii) data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in the group (GI); data relating to the relationship between cancer and the expression level(s) of at least 1, 2, 3, 4, 5, 6, 7, 8, 9, 10 or more, 15 or more, or 20 or more, or all microRNAs selected from the group consisting of microRNAs with p < 0.005 or less in the group (GI); data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 22-1 to 22-13 in the group (GI); data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 22-1 and 23-1 to 23-14 in the group (GI); data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 24-1 to 24-15 in the group (GI); or data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 25-1 to 25-15 in the group (GI);
(xxiv) data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in the group (Hemo); data relating to the relationship between cancer and the expression level(s) of at least 1, 2, 3, 4, 5, 6, 7, 8, 9, 10 or more, 15 or more, or 20 or more, or all microRNAs selected from the group consisting of microRNAs with p < 0.005 or less in the group (Hemo); data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 22-1 to 22-13 in the group (Hemo); data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 22-1 and 23-1 to 23-14 in the group (Hemo); data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 24-1 to 24-15 in the group (Hemo); or data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 25-1 to 25-15 in the group (Hemo);
(xxv) data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in the group (Uro); data relating to the relationship between cancer and the expression level(s) of at least 1, 2, 3, 4, 5, 6, 7, 8, 9, 10 or more, 15 or more, or 20 or more, or all microRNAs selected from the group consisting of microRNAs with p < 0.005 or less in the group (Uro); data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 22-1 to 22-13 in the group (Uro); data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 22-1 and 23-1 to 23-14 in the group (Uro); data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 24-1 to 24-15 in the group (Uro); or data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 25-1 to 25-15 in the group (Uro); or
(xxvi) a combination thereof.

A trained computer or artificial intelligence can include a memory (including a magnetic recording medium, such as a ROM, a RAM, a hard disk, or an SSD, or a computer including the magnetic recording medium) in which one or more pieces of data selected from the group consisting of (i) to (v) are recorded, or can be coupled to the memory via an electronic communication circuit. A trained computer or artificial intelligence can be further trained using one or more pieces of data selected from the group consisting of (i) to (v) (in this case, the data used for learning may be further added to the memory). A trained computer or artificial intelligence can determine the likelihood that a subject has cancer on the basis of the data relating to the relationship between the expression level(s) of the at least one or all microRNAs and cancer and the expression level(s) of the at least one or all microRNAs in a body fluid sample of the subject. The learning of a computer or artificial intelligence with the relationship data can be performed by evaluating unevaluated data using one or a plurality of pieces of the relationship data as teacher data and by repeatedly learning different relationship data, for example, until the sensitivity and /or specificity for cancer detection exceeds a predetermined value. The predetermined value may depend on sensitivity and /or specificity requirements and may be, for example, 10%, 20%, 30%, 40%, 50%, 60%, 70%, 80%, or 90% or more. In general, false positives increase with increasing sensitivity, and false negatives increase with decreasing sensitivity. The sensitivity is therefore preferably set in accordance with the purpose of the examination. Furthermore, in general, false negatives increase with increasing specificity, and false positives increase with decreasing sensitivity. The specificity is therefore desirably set in accordance with the purpose of the examination.

In the present disclosure, after the likelihood of cancer is examined, data indicating a likelihood of having cancer and /or a likelihood of not having cancer can be output to a medium, such as an electronic medium or paper. The output data may be presented to the doctor and /or the patient (or the family or relatives thereof, etc.) and may be used to investigate subsequent therapeutic strategy or to investigate further work-up (for example, to select an examination). After the likelihood of cancer is examined, the patient can be treated with cancer therapy, such as chemotherapy, radiotherapy, or surgery (for example, cancer therapy for a specific cancer determined to be likely to have).

The present disclosure provides a method for detecting a microRNA in the urine or an extract of the urine of a subject,
wherein, for example, the method detects one or more selected from a microRNA group with higher expression in three subjects having cancer than in any of three non-cancer subjects in the data S1 (or Table 3). A microRNA detected in this embodiment may be, for example, one or more selected from a group of microRNAs with higher (for example, twice or more, three times or more, four times or more, five times or more, six times or more, seven times or more, eight times or more, nine times or more, or ten times or more) expression in a subject having cancer than in a non-cancer subject. Such a microRNA may be, for example, miR-3117-5p, miR-3118, miR-3121-3p, miR-3121-5p, miR-3126-5p, miR-3128, miR-3133, miR-3134, miR-3136-3p, miR-3136-5p, miR-3139, miR-3142, miR-3143, miR-3145-3p, miR-3163, miR-3166, miR-3167, miR-0558, miR-3126-3p, miR-3129-5p, miR-3144-5p, miR-3150a-5p, miR-3152-5p, miR-3157-3p, miR-3159, miR-4521, miR-0029b-3p, miR-0030b-3p, miR-0106b-3p, miR-0320c, miR-0494-3p, miR-0566, miR-0572, miR-0645, miR-0939-3p, miR-0943, miR-1972, miR-3129-3p, miR-3132, miR-3140-3p, miR-3144-3p, miR-3199, miR-3613-3p, miR-4304, miR-4454, miR-4491, miR-4506, miR-4519, miR-5006-5p, miR-6068, miR-6084, miR-6726-5p, miR-6862-5p, miR-6871-5p, miR-6877-5p, miR-4661-5p, miR-5587-5p, miR-0150-3p, miR-0718, miR-0770-5p, miR-4515, miR-4520-3p, miR-4655-3p, miR-4684-5p, miR-6723-5p, miR-6762-5p, miR-8059, miR-8063, let-7c-3p, miR-0026a-1-3p, miR-0105-5p, miR-0195-3p, miR-0219a-2-3p, miR-0431-5p, miR-0454-5p, miR-0548ax, miR-0593-5p, miR-0623, miR-0664a-5p, miR-0942-3p, miR-1205, miR-1297, miR-3678-3p, miR-4283, miR-7161-5p, let-7b-3p, let-7b-5p, miR-0018a-3p, miR-0018b-3p, miR-0021-3p, miR-0024-2-5p, miR-0025-3p, miR-0025-5p, miR-0026b-3p, miR-0030b-5p, miR-0030d-5p, miR-0030e-3p, miR-0033a-3p, miR-0033b-3p, miR-0034b-5p, miR-0092a-3p, miR-0092b-3p, miR-0093-5p, miR-0098-3p, miR-0099b-5p, miR-0125a-3p, miR-0128-2-5p, miR-0129-2-3p, miR-0130b-5p, miR-0132-3p, miR-0133a-3p, miR-0133a-5p, miR-0133b, miR-0150-5p, miR-0181a-2-3p, miR-0188-5p, miR-0191-3p, miR-0192-5p, miR-0193b-3p, miR-0194-3p, miR-0197-3p, miR-0199a-5p, miR-0199b-5p, miR-0200a-5p, miR-0202-3p, miR-0203a-3p, miR-0204-5p, miR-0205-5p, miR-0210-5p, miR-0212-3p, miR-0216b-3p, miR-0223-3p, miR-0223-5p, miR-0224-3p, miR-0296-5p, miR-0299-5p, miR-0320a, miR-0320b, miR-0320e, miR-0326, miR-0328-3p, miR-0337-3p, miR-0338-3p, miR-0339-5p, miR-0340-3p, miR-0342-5p, miR-0346, miR-0361-3p, miR-0362-3p, miR-0365a-3p, miR-365b-3p, miR-0371a-3p, miR-0371b-3p, miR-0377-5p, miR-0378d, miR-0383-3p, miR-0409-3p, miR-0411-3p, miR-0422a, miR-0423-5p, miR-0431-3p, miR-0449c-3p, miR-0483-3p, miR-0484, miR-0485-3p, miR-0485-5p, miR-0486-5p, miR-0491-3p, miR-0501-5p, miR-0503-3p, miR-0504-5p, miR-0506-3p, miR-0508-5p, miR-0509-5p, miR-0510-5p, miR-0512-3p, miR-0514b-3p, miR-0516b-3p, miR-516a-3p, miR-0518b, miR-0518c-5p, miR-0519d-3p, miR-0519e-3p, miR-0520a-3p, miR-0520g-3p, miR-0550a-3p, miR-0550a-5p, miR-0552-5p, miR-0557, miR-0574-3p, miR-0574-5p, miR-0575, miR-0580-3p, miR-0584-5p, miR-0589-3p, miR-0589-5p, miR-0601, miR-0605-5p, miR-0610, miR-0612, miR-0615-3p, miR-0625-3p, miR-0628-3p, miR-0630, miR-0634, miR-0635, miR-0636, miR-0642a-5p, miR-0650, miR-0656-5p, miR-0657, miR-0659-5p, miR-0663b, miR-0664a-3p, miR-0664b-3p, miR-0671-3p, miR-0764, miR-0766-3p, miR-0874-3p, miR-0877-3p, miR-0877-5p, miR-0888-5p, miR-0935, miR-0937-3p, miR-0938, miR-0940, miR-1181, miR-1182, miR-1200, miR-1204, miR-1207-3p, miR-1224-3p, miR-1225-3p, miR-1228-3p, miR-1234-3p, miR-1238-3p, miR-1238-5p, miR-1247-5p, miR-1249-3p, miR-1250-3p, miR-1250-5p, miR-1255b-5p, miR-1260a, miR-1260b, miR-1266-5p, miR-1273h-3p, miR-1281, miR-1286, miR-1292-3p, miR-1295b-3p, miR-1296-3p, miR-1296-5p, miR-1304-3p, miR-1306-5p, miR-1343-3p, miR-1470, miR-1538, miR-1539, miR-1825, miR-1909-5p, miR-1910-5p, miR-1911-3p, miR-1911-5p, miR-1913, miR-1914-5p, miR-1976, miR-2110, miR-2355-5p, miR-2909, miR-3064-5p, miR-3074-3p, miR-3127-3p, miR-3130-5p, miR-3141, miR-3147, miR-3150a-3p, miR-3150b-5p, miR-3151-3p, miR-3160-3p, miR-3180-5p, miR-3184-3p, miR-3186-3p, miR-3189-5p, miR-3190-5p, miR-3191-3p, miR-3192-3p, miR-3194-5p, miR-3195, miR-3200-3p, miR-3200-5p, miR-3614-5p, miR-3615, miR-3619-5p, miR-3620-3p, miR-3622a-3p, miR-3622a-5p, miR-3622b-3p, miR-3646, miR-3659, miR-3670, miR-3675-3p, miR-3679-3p, miR-3689d, miR-3690, miR-3909, miR-3918, miR-3921, miR-3922-3p, miR-3940-3p, miR-3943, miR-4253, miR-4260, miR-4267, miR-4268, miR-4269, miR-4274, miR-4278, miR-4279, miR-4280, miR-4284, miR-4286, miR-4289, miR-4290, miR-4292, miR-4310, miR-4312, miR-4313, miR-4317, miR-4318, miR-4319, miR-4323, miR-4329, miR-4433a-5p, miR-4433b-5p, miR-4436b-5p, miR-4447, miR-4455, miR-4463, miR-4494, miR-4632-3p, miR-4638-3p, miR-4640-3p, miR-4642, miR-4646-5p, miR-4649-3p, miR-4649-5p, miR-4652-3p, miR-4652-5p, miR-4653-5p, miR-4664-3p, miR-4665-3p, miR-4667-3p, miR-4675, miR-4676-3p, miR-4685-3p, miR-4687-5p, miR-4690-5p, miR-4691-5p, miR-4697-3p, miR-4697-5p, miR-4700-3p, miR-4701-5p, miR-4706, miR-4707-3p, miR-4708-3p, miR-4712-3p, miR-4713-5p, miR-4714-5p, miR-4716-5p, miR-4717-5p, miR-4718, miR-4719, miR-4722-5p, miR-4723-3p, miR-4725-5p, miR-4726-3p, miR-4727-3p, miR-4728-3p, miR-4731-3p, miR-4733-3p, miR-4740-5p, miR-4749-5p, miR-4758-3p, miR-4761-3p, miR-4763-5p, miR-4769-3p, miR-4780, miR-4783-3p, miR-4787-3p, miR-4793-5p, miR-4794, miR-4804-3p, miR-5008-3p, miR-5008-5p, miR-5091, miR-5190, miR-5196-3p, miR-5587-3p, miR-5588-5p, miR-5693, miR-5699-5p, miR-5705, miR-6086, miR-6088, miR-6124, miR-6165, miR-6501-5p, miR-6505-5p, miR-6508-5p, miR-6513-3p, miR-6515-3p, miR-6722-5p, miR-6726-3p, miR-6727-3p, miR-6728-3p, miR-6729-3p, miR-6730-3p, miR-6731-3p, miR-6732-3p, miR-6735-3p, miR-6735-5p, miR-6737-3p, miR-6738-5p, miR-6743-3p, miR-6746-3p, miR-6749-3p, miR-6752-3p, miR-6753-5p, miR-6759-3p, miR-6760-3p, miR-6763-3p, miR-6765-3p, miR-6765-5p, miR-67 68-5p, miR-6769a-3p, miR-6769b-3p, miR-6770-5p, miR-6775-3p, miR-6777-3p, miR-6784-3p, miR-6785-3p, miR-6785-5p, miR-6787-3p, miR-6788-3p, miR-6788-5p, miR-6790-3p, miR-6791-3p, miR-6792-3p, miR-6792-5p, miR-6793-3p, miR-6794-3p, miR-6795-3p, miR-6799-3p, miR-6800-3p, miR-6801-3p, miR-6802-3p, miR-6803-3p, miR-6806-5p, miR-6807-3p, miR-6808-5p, miR-6810-3p, miR-6811-3p, miR-6812-3p, miR-6813-3p, miR-6816-3p, miR-6819-3p, miR-6820-3p, miR-6823-3p, miR-6824-3p, miR-6825-3p, miR-6826-3p, miR-6828-3p, miR-6828-5p, miR-6829-3p, miR-6840-5p, miR-6845-3p, miR-6846-3p, miR-6848-3p, miR-6849-3p, miR-6851-3p, miR-6852-3p, miR-6857-3p, miR-6858-3p, miR-6859-3p, miR-6860, miR-6861-3p, miR-6865-3p, miR-6865-5p, miR-6867-3p, miR-6870-3p, miR-6871-3p, miR-6873-3p, miR-6874-3p, miR-6877-3p, miR-6879-3p, miR-6880-3p, miR-6884-3p, miR-6885-3p, miR-6887-3p, miR-6889-3p, miR-6890-3p, miR-6891-3p, miR-6895-3p, miR-7106-3p, miR-7109-3p, miR-7111-3p, miR-7113-3p, miR-7114-3p, miR-7853-5p, miR-8060, miR-8078, miR-8485, miR-0513b-5p, miR-0619-5p, miR-1285-3p, miR-3162-5p, miR-4311, miR-4531, miR-5096, miR-0016-2-3p, miR-0030c-1-3p, miR-0125a-5p, miR-0125b-5p, miR-0183-3p, miR-0184, miR-0193a-3p, miR-0211-3p, miR-0324-3p, miR-0432-5p, miR-0433-3p, miR-0483-5p, miR-0493-3p, miR-0505-5p, miR-0642a-3p, miR-0642b-3p, miR-0642b-5p, miR-0652-5p, miR-0658, miR-0663a, miR-0760, miR-0765, miR-0873-3p, miR-0885-3p, miR-0937-5p, miR-1202, miR-1224-5p, miR-1229-5p, miR-1249-5p, miR-1251-3p, miR-1273e, miR-1273g-3p, miR-1908-5p, miR-2392, miR-2467-3p, miR-3124-5p, miR-3138, miR-3156-3p, miR-3158-5p, miR-3175, miR-3190-3p, miR-3198, miR-3612, miR-3619-3p, miR-3649, miR-3653-3p, miR-3655, miR-3657, miR-3667-5p, miR-3679-5p, miR-3682-3p, miR-3917, miR-3945, miR-4255, miR-4294, miR-4307, miR-4321, miR-4419a, miR-4448, miR-4496, miR-4524a-5p, miR-4530, miR-4638-5p, miR-4725-3p, miR-4726-5p, miR-4748, miR-4754, miR-4769-5p, miR-4786-5p, miR-4800-5p, miR-5006-3p, miR-5088-3p, miR-5089-3p, miR-5093, miR-5196-5p, miR-5585-3p, miR-5698, miR-6077, miR-6716-5p, miR-6718-5p, miR-6740-5p, miR-6751-3p, miR-6756-3p, miR-6766-5p, miR-6769b-5p, miR-6778-5p, miR-6780a-5p, miR-6780b-5p, miR-6794-5p, miR-6799-5p, miR-6812-5p, miR-6824-5p, miR-6825-5p, miR-6830-3p, miR-6831-3p, miR-6831-5p, miR-6833-5p, miR-6839-5p, miR-6855-3p, miR-6861-5p, miR-6870-5p, miR-6879-5p, miR-6892-5p, miR-6894-5p, miR-7109-5p, miR-7150, miR-7154-3p, miR-8085, miR-8087, miR-0103a-2-5p, miR-0151b, miR-0519c-5p, miR-523-5p, miR-518e-5p, miR-522-5p, miR-519a-5p, miR-519b-5p, miR-0617, miR-0921, miR-6874-5p, miR-0030c-2-3p, miR-0034a-5p, miR-0092a-2-5p, miR-0129-1-3p, miR-0134-3p, miR-0181a-5p, miR-0185-5p, miR-0204-3p, miR-0302c-5p, miR-0324-5p, miR-0338-5p, miR-0370-3p, miR-0382-5p, miR-0421, miR-0450a-5p, miR-0491-5p, miR-0518f-3p, miR-0518f-5p, miR-0520b, miR-0520d-5p, miR-0520e, miR-0527, miR-518a-5p, miR-0541-3p, miR-0550b-2-5p, miR-0622, miR-0668-5p, miR-0708-5p, miR-0766-5p, miR-0767-3p, miR-0920, miR-1184, miR-1185-1-3p, miR-1185-2-3p, miR-1227-3p, miR-1237-3p, miR-1265, miR-1267, miR-1273h-5p, miR-1301-3p, miR-2116-5p, miR-3116, miR-3137, miR-3151-5p, miR-3156-5p, miR-3157-5p, miR-3164, miR-3177-3p, miR-3189-3p, miR-3202, miR-3622b-5p, miR-3651, miR-3925-5p, miR-3928-3p, miR-3975, miR-4257, miR-4261, miR-4296, miR-4300, miR-4306, miR-4316, miR-4431, miR-4443, miR-4444, miR-4453, miR-4459, miR-4465, miR-4482-3p, miR-4489, miR-4499, miR-4514, miR-4657, miR-4664-5p, miR-4669, miR-4698, miR-4749-3p, miR-4750-3p, miR-4753-5p, miR-4756-3p, miR-5000-5p, miR-5001-5p, miR-5010-5p, miR-5571-3p, miR-6076, miR-6127, miR-6500-3p, miR-6503-5p, miR-6507-3p, miR-6507-5p, miR-6511b-5p, miR-6515-5p, miR-6516-5p, miR-6717-5p, miR-6728-5p, miR-6734-5p, miR-6741-5p, miR-6742-3p, miR-6742-5p, miR-6745, miR-6746-5p, miR-6747-5p, miR-6748-5p, miR-6756-5p, miR-6760-5p, miR-6766-3p, miR-6771-3p, miR-6776-5p, miR-6782-3p, miR-6795-5p, miR-6796-3p, miR-6815-5p, miR-6823-5p, miR-6827-5p, miR-6829-5p, miR-6830-5p, miR-6834-5p, miR-6841-3p, miR-6842-5p, miR-6849-5p, miR-6853-5p, miR-6872-5p, miR-6887-5p, miR-6891-5p, miR-7106-5p, miR-7107-5p, miR-7108-3p, miR-7111-5p, miR-7846-3p, miR-7855-5p, miR-8062, miR-8071, and miR-8082, and one or more microRNAs (preferably human microRNAs) selected from the group consisting of these can be detected.

The present disclosure also provides a method for detecting a microRNA in the urine or an extract of the urine of a subject,
wherein, for example, the method detects one or more selected from a microRNA group with lower expression in three subjects having cancer than in any of three non-cancer subjects in the data S1 (or Table 3). A microRNA detected in this embodiment may be, for example, one or more selected from a group of microRNAs with lower (for example, twice or more, three times or more, four times or more, five times or more, six times or more, seven times or more, eight times or more, nine times or more, or ten times or more) expression in three subjects having cancer than in any of three non-cancer subjects. Such a microRNA may be miR-3127-3p, miR-3130-5p, miR-3131, miR-3141, miR-3150b-5p, miR-3151-3p, miR-3151-5p, miR-3154, miR-3160-3p, miR-3160-5p, miR-3162-5p, miR-0015b-5p, miR-0034c-5p, miR-0093-5p, miR-0128-2-5p, miR-0135a-5p, miR-0149-3p, miR-0214-5p, miR-0320a, miR-0339-5p, miR-0365a-5p, miR-0372-3p, miR-0378b, miR-0424-5p, miR-0488-5p, miR-0498, miR-0512-3p, miR-0512-5p, miR-0580-3p, miR-0670-5p, miR-0671-5p, miR-0758-5p, miR-0933, miR-0937-3p, miR-0942-5p, miR-1178-3p, miR-1207-3p, miR-1224-3p, miR-1233-3p, miR-1233-5p, miR-1249-5p, miR-1266-3p, miR-2277-3p, miR-2277-5p, miR-3065-5p, miR-3122, miR-3135b, miR-3153, miR-3156-3p, miR-3158-5p, miR-3162-3p, miR-3174, miR-3180, miR-3529-5p, miR-3680-3p, miR-3689f, miR-4266, miR-4273, miR-4281, miR-4327, miR-4526, miR-4643, miR-4646-3p, miR-4675, miR-4698, miR-4706, miR-4718, miR-4728-3p, miR-4752, miR-4753-3p, miR-4801, miR-5192, miR-5195-5p, miR-5704, miR-6069, miR-6088, miR-6132, miR-6502-5p, miR-6505-3p, miR-6510-5p, miR-6511b-3p, miR-6516-5p, miR-6744-5p, miR-6749-3p, miR-6754-5p, miR-6757-3p, miR-6757-5p, miR-6765-5p, miR-6771-3p, miR-6775-5p, miR-6781-3p, miR-6800-5p, miR-6807-5p, miR-6811-3p, miR-6813-5p, miR-6822-5p, miR-6829-5p, miR-6832-3p, miR-6841-3p, miR-6845-3p, miR-6864-3p, miR-6865-5p, miR-6873-5p, miR-6877-3p, miR-6878-5p, miR-6881-5p, miR-6885-5p, miR-6886-5p, miR-7106-5p, miR-7111-3p, miR-7153-3p, miR-7848-3p, miR-7978, miR-8059, miR-0520b, miR-3605-5p, miR-3612, miR-4645-3p, miR-4694-3p, miR-6816-3p, miR-8087, miR-0015a-3p, miR-0135b-5p, miR-0185-5p, miR-0302d-5p, miR-0483-5p, miR-0671-3p, miR-1254, miR-1284, miR-1323, miR-3138, miR-3164, miR-3189-3p, miR-3200-5p, miR-3657, miR-3667-5p, miR-3692-5p, miR-3713, miR-4299, miR-4306, miR-4316, miR-4319, miR-4441, miR-4658, miR-5004-3p, miR-5194, miR-6083, miR-6720-5p, miR-6821-3p, miR-6832-5p, miR-6875-5p, miR-0001-5p, miR-0007-2-3p, miR-0025-5p, miR-0030c-1-3p, miR-0030c-2-3p, miR-0125a-3p, miR-0134-5p, miR-0146a-5p, miR-0183-3p, miR-0193a-5p, miR-0193b-3p, miR-0197-5p, miR-0198, miR-0212-5p, miR-0221-3p, miR-0299-5p, miR-0326, miR-0328-5p, miR-0374c-3p, miR-0423-5p, miR-0432-5p, miR-0433-5p, miR-0483-3p, miR-0505-5p, miR-0513a-5p, miR-0521, miR-0532-3p, miR-0550a-3-5p, miR-0550a-5p, miR-0550b-3p, miR-0551b-3p, miR-0589-3p, miR-0591, miR-0615-3p, miR-0642a-3p, miR-0642b-3p, miR-0650, miR-0652-5p, miR-0664b-3p, miR-0668-3p, miR-0675-5p, miR-0711, miR-0744-5p, miR-0764, miR-0939-3p, miR-1180-3p, miR-1185-1-3p, miR-1185-2-3p, miR-1193, miR-1199-3p, miR-1202, miR-1207-5p, miR-1228-5p, miR-1229-5p, miR-1238-5p, miR-1273h-5p, miR-1275, miR-1911-3p, miR-2276-3p, miR-2278, miR-2355-5p, miR-2861, miR-3074-5p, miR-3137, miR-3144-5p, miR-3147, miR-3184-5p, miR-3188, miR-3190-3p, miR-3202, miR-3610, miR-3622b-5p, miR-3666, miR-3679-5p, miR-3689d, miR-3911, miR-3918, miR-3919, miR-3927-5p, miR-3928-3p, miR-4251, miR-4259, miR-4265, miR-4271, miR-4279, miR-4288, miR-4290, miR-4294, miR-4298, miR-4301, miR-4322, miR-4329, miR-4419a, miR-4419b, miR-4447, miR-4462, miR-4472, miR-4476, miR-4483, miR-4484, miR-4492, miR-4496, miR-4499, miR-4513, miR-4523, miR-4632-5p, miR-4646-5p, miR-4655-5p, miR-4656, miR-4667-5p, miR-4685-5p, miR-4687-3p, miR-4697-5p, miR-4709-3p, miR-4722-3p, miR-4723-5p, miR-4726-3p, miR-4726-5p, miR-4728-5p, miR-4732-5p, miR-4739, miR-4743-5p, miR-4747-5p, miR-4748, miR-4751, miR-4756-5p, miR-4783-3p, miR-4788, miR-4800-5p, miR-5088-3p, miR-5698, miR-5702, miR-5739, miR-6085, miR-6086, miR-6087, miR-6124, miR-6133, miR-6501-5p, miR-6504-5p, miR-6513-3p, miR-6716-5p, miR-6727-3p, miR-6730-5p, miR-6733-3p, miR-6734-5p, miR-6735-3p, miR-6735-5p, miR-6741-5p, miR-6744-3p, miR-6745, miR-6746-5p, miR-6749-5p, miR-6750-5p, miR-6760-5p, miR-6769a-5p, miR-6769b-5p, miR-6774-5p, miR-6776-3p, miR-6779-5p, miR-6787-5p, miR-6788-3p, miR-6790-5p, miR-6792-5p, miR-6794-5p, miR-6797-5p, miR-6799-5p, miR-6803-5p, miR-6806-5p, miR-6812-5p, miR-6814-5p, miR-6815-5p, miR-6819-5p, miR-6822-3p, miR-6823-5p, miR-6824-5p, miR-6827-5p, miR-6830-5p, miR-6831-3p, miR-6831-5p, miR-6833-5p, miR-6842-5p, miR-6851-5p, miR-6858-5p, miR-6862-5p, miR-6866-3p, miR-6870-5p, miR-6872-5p, miR-6879-5p, miR-6883-5p, miR-6884-3p, miR-6891-5p, miR-6894-5p, miR-7107-5p, miR-7109-5p, miR-7110-3p, miR-7111-5p, miR-7112-5p, miR-7150, miR-7152-3p, miR-7154-3p, miR-7155-3p, miR-7706, miR-7843-5p, miR-7845-5p, miR-7846-3p, miR-7847-3p, miR-7973, miR-8052, miR-8058, miR-8074, miR-8089, miR-0378a-5p, miR-4489, miR-6511b-5p, miR-0187-5p, miR-0208a-5p, miR-0486-3p, miR-0511-5p, miR-0585-5p, miR-0643, miR-0663b, miR-1231, miR-3187-5p, miR-3665, miR-4446-3p, miR-4466, miR-4525, miR-4634, miR-4674, miR-4734, miR-4785, miR-4787-5p, miR-4794, miR-5008-5p, miR-6499-5p, miR-6510-3p, miR-6727-5p, miR-6814-3p, miR-6836-3p, miR-7704, miR-8069, miR-7849-3p, miR-0025-3p, miR-0092a-3p, miR-0092b-3p, miR-0099b-5p, miR-0128-1-5p, miR-0139-3p, miR-0149-5p, miR-0192-5p, miR-0205-5p, miR-0216b-3p, miR-0223-5p, miR-0346, miR-0371a-3p, miR-0378c, miR-0425-3p, miR-0503-3p, miR-0520a-3p, miR-0520h, miR-0548ay-3p, miR-0548q, miR-0557, miR-0631, miR-0636, miR-0638, miR-0659-3p, miR-0762, miR-0935, miR-1203, miR-1225-5p, miR-1237-5p, miR-1268a, miR-1469, miR-1539, miR-1909-3p, miR-1910-5p, miR-1914-5p, miR-2682-3p, miR-3064-5p, miR-3065-3p, miR-3130-3p, miR-3173-3p, miR-3178, miR-3 180-3p, miR-3196, miR-3935, miR-3937, miR-3940-5p, miR-3960, miR-4270, miR-4276, miR-4442, miR-4485-5p, miR-4488, miR-4505, miR-4508, miR-4632-3p, miR-4649-5p, miR-4676-3p, miR-4688, miR-4707-5p, miR-4708-5p, miR-4722-5p, miR-4730, miR-4738-3p, miR-4747-3p, miR-4761-3p, miR-4763-3p, miR-4773, miR-5196-3p, miR-5584-3p, miR-5787, miR-6089, miR-6090, miR-6508-5p, miR-6721-5p, miR-6722-5p, miR-6726-3p, miR-6729-5p, miR-6737-5p, miR-6738-5p, miR-6746-3p, miR-6767-3p, miR-6771-5p, miR-6784-5p, miR-6785-5p, miR-6786-5p, miR-6789-3p, miR-6789-5p, miR-6805-5p, miR-6816-5p, miR-6825-3p, miR-6833-3p, miR-6837-3p, miR-6847-3p, miR-6848-5p, miR-6850-5p, miR-6869-5p, miR-6871-3p, miR-6895-3p, miR-7155-5p, miR-7844-5p, miR-8072, miR-8485, and miR-9500, for example, and one or more microRNAs (preferably human microRNAs) selected from the group consisting of these can be detected.

In these embodiments, the type of microRNA to be detected and the type of microRNA to be used as an indicator can each independently be, for example, 1 or more, 2 or more, 3 or more, 4 or more, 5 or more, 6 or more, 7 or more, 8 or more, 9 or more, 10 or more, 20 or more, 30 or more, 40 or more, 50 or more, 60 or more, 70 or more, 80 or more, 90 or more, 100 or more, 200 or more, 300 or more, 400 or more, 500 or more, 600 or more, 700 or more, 800 or more, 900 or more, 1000 or more, 1100 or more, 1200 or more, 1300 or more, 1400 or more, 1500 or more, 1600 or more, 1700 or more, 1800 or more, 1900 or more, 2000 or more, 2100 or more, 2200 or more, 2300 or more, 2400 or more, or 2500 or more. In these embodiments, the type of microRNA to be detected and the type of microRNA to be used as an indicator can each independently be, for example, 2500 or less, 2400 or less, 2300 or less, 2200 or less, 2100 or less, 2000 or less, 1900 or less, 1800 or less, 1700 or less, 1600 or less, 1500 or less, 1400 or less, 1300 or less, 1200 or less, 1100 or less, 1000 or less, 900 or less, 800 or less, 700 or less, 600 or less, 500 or less, 400 or less, 300 or less, 200 or less, 100 or less, 90 or less, 80 or less, 70 or less, 60 or less, 50 or less, 40 or less, 30 or less, 20 or less, or 10 or less. The type of microRNA to be detected and the type of microRNA to be used as an indicator may be completely identical, or microRNAs to be detected may be partly used as an indicator.

After detection, cancer can be diagnosed in accordance with the present disclosure using the expression level or the presence or absence of the microRNA as an indicator. A patient diagnosed as having a likelihood of cancer may be treated (for example, cancer therapy, such as chemotherapy, anticancer agent therapy, surgery, immunotherapy, or radiotherapy).

The present disclosure also provides a method for detecting a microRNA in the urine or an extract of the urine of a subject,
wherein the microRNA is
(i-a) at least 1, 2, 3, 4, 5, 6, 7, 8, 9, 10 or more, 15 or more, or 20 or more, or all microRNAs selected from the group consisting of the microRNAs listed in Table 4-2; at least 1, 2, 3, 4, 5, 6, 7, 8, 9, 10 or more, 15 or more, or 20 or more, or all microRNAs selected from the group consisting of microRNAs with p < 0.005 or less in Table 4-2; at least 1, 2, 3, 4, 5, 6, 7, 8, 9, 10 or more, 15 or more, or 20 or more, or all microRNAs selected from the group consisting of the microRNAs listed in Table 22-2; at least 1, 2, 3, 4, 5, 6, 7, 8, 9, 10 or more, 15 or more, or 20 or more, or all microRNAs selected from the group consisting of the microRNAs listed in Table 23-2; at least 1, 2, 3, 4, 5, 6, 7, 8, 9, 10 or more, 15 or more, or 20 or more, or all microRNAs selected from the group consisting of the microRNAs listed in Table 24-2; or at least 1, 2, 3, 4, 5, 6, 7, 8, 9, 10 or more, 15 or more, or 20 or more, or all microRNAs selected from the group consisting of the microRNAs listed in Table 25-2;
wherein the subject may be a subject having or suspected of having breast cancer;
(ii-a) at least 1, 2, 3, 4, 5, 6, 7, 8, 9, 10 or more, 15 or more, or 20 or more, or all microRNAs selected from the group consisting of Table 4-3; at least 1, 2, 3, 4, 5, 6, 7, 8, 9, 10 or more, 15 or more, or 20 or more, or all microRNAs selected from the group consisting of microRNAs with p < 0.005 or less in Table 4-3; at least 1, 2, 3, 4, 5, 6, 7, 8, 9, 10 or more, 15 or more, or 20 or more, or all microRNAs selected from the group consisting of the microRNAs listed in Table 22-3; at least 1, 2, 3, 4, 5, 6, 7, 8, 9, 10 or more, 15 or more, or 20 or more, or all microRNAs selected from the group consisting of the microRNAs listed in Table 23-3; at least 1, 2, 3, 4, 5, 6, 7, 8, 9, 10 or more, 15 or more, or 20 or more, or all microRNAs selected from the group consisting of the microRNAs listed in Table 24-3; or at least 1, 2, 3, 4, 5, 6, 7, 8, 9, 10 or more, 15 or more, or 20 or more, or all microRNAs selected from the group consisting of the microRNAs listed in Table 25-3;
wherein the subject may be a subject having or suspected of having kidney cancer;
(iii-a) at least 1, 2, 3, 4, 5, 6, 7, 8, 9, 10 or more, 15 or more, or 20 or more, or all microRNAs selected from the group consisting of Table 4-4; at least 1, 2, 3, 4, 5, 6, 7, 8, 9, 10 or more, 15 or more, or 20 or more, or all microRNAs selected from the group consisting of microRNAs with p < 0.005or less in Table 4-4; at least 1, 2, 3, 4, 5, 6, 7, 8, 9, 10 or more, 15 or more, or 20 or more, or all microRNAs selected from the group consisting of the microRNAs listed in Table 24-4; or at least 1, 2, 3, 4, 5, 6, 7, 8, 9, 10 or more, 15 or more, or 20 or more, or all microRNAs selected from the group consisting of the microRNAs listed in Table 25-4;
wherein the subject may be a subject having or suspected of having leukemia;
(iv-a) at least 1, 2, 3, 4, 5, 6, 7, 8, 9, 10 or more, 15 or more, or 20 or more, or all microRNAs selected from the group consisting of the microRNAs listed in Table 4-5; at least 1, 2, 3, 4, 5, 6, 7, 8, 9, 10 or more, 15 or more, or 20 or more, or all microRNAs selected from the group consisting of microRNAs with p < 0.005 or less in Table 4-5; at least 1, 2, 3, 4, 5, 6, 7, 8, 9, 10 or more, 15 or more, or 20 or more, or all microRNAs selected from the group consisting of the microRNAs listed in Table 23-4; at least 1, 2, 3, 4, 5, 6, 7, 8, 9, 10 or more, 15 or more, or 20 or more, or all microRNAs selected from the group consisting of the microRNAs listed in Table 24-5; or at least 1, 2, 3, 4, 5, 6, 7, 8, 9, 10 or more, 15 or more, or 20 or more, or all microRNAs selected from the group consisting of the microRNAs listed in Table 25-5;
wherein the subject may be a subject having or suspected of having lymphoma;
(v-a) at least 1, 2, 3, 4, 5, 6, 7, 8, 9, 10 or more, 15 or more, or 20 or more, or all microRNAs selected from the group consisting of the microRNAs listed in Table 4-6; at least 1, 2, 3, 4, 5, 6, 7, 8, 9, 10 or more, 15 or more, or 20 or more, or all microRNAs selected from the group consisting of microRNAs with p < 0.005 or less in Table 4-6; at least 1, 2, 3, 4, 5, 6, 7, 8, 9, 10 or more, 15 or more, or 20 or more, or all microRNAs selected from the group consisting of the microRNAs listed in Table 22-4; at least 1, 2, 3, 4, 5, 6, 7, 8, 9, 10 or more, 15 or more, or 20 or more, or all microRNAs selected from the group consisting of the microRNAs listed in Table 23-5; at least 1, 2, 3, 4, 5, 6, 7, 8, 9, 10 or more, 15 or more, or 20 or more, or all microRNAs selected from the group consisting of the microRNAs listed in Table 24-6; or at least 1, 2, 3, 4, 5, 6, 7, 8, 9, 10 or more, 15 or more, or 20 or more, or all microRNAs selected from the group consisting of the microRNAs listed in Table 25-6;
wherein the subject may be a subject having or suspected of having pancreatic cancer;
(vi-a) at least 1, 2, 3, 4, 5, 6, 7, 8, 9, 10 or more, 15 or more, or 20 or more, or all microRNAs selected from the group consisting of the microRNAs listed in Table 4-7; at least 1, 2, 3, 4, 5, 6, 7, 8, 9, 10 or more, 15 or more, or 20 or more, or all microRNAs selected from the group consisting of microRNAs with p < 0.005 or less in Table 4-7; at least 1, 2, 3, 4, 5, 6, 7, 8, 9, 10 or more, 15 or more, or 20 or more, or all microRNAs selected from the group consisting of the microRNAs listed in Table 22-5; at least 1, 2, 3, 4, 5, 6, 7, 8, 9, 10 or more, 15 or more, or 20 or more, or all microRNAs selected from the group consisting of the microRNAs listed in Table 23-6; at least 1, 2, 3, 4, 5, 6, 7, 8, 9, 10 or more, 15 or more, or 20 or more, or all microRNAs selected from the group consisting of the microRNAs listed in Table 24-7; or at least 1, 2, 3, 4, 5, 6, 7, 8, 9, 10 or more, 15 or more, or 20 or more, or all microRNAs selected from the group consisting of the microRNAs listed in Table 25-7;
wherein the subject may be a subject having or suspected of having prostate cancer;
(vii-a) at least 1, 2, 3, 4, 5, 6, 7, 8, 9, 10 or more, 15 or more, or 20 or more, or all microRNAs selected from the group consisting of the microRNAs listed in Table 4-8; at least 1, 2, 3, 4, 5, 6, 7, 8, 9, 10 or more, 15 or more, or 20 or more, or all microRNAs selected from the group consisting of microRNAs with p < 0.005 or less in Table 4-8; at least 1, 2, 3, 4, 5, 6, 7, 8, 9, 10 or more, 15 or more, or 20 or more, or all microRNAs selected from the group consisting of the microRNAs listed in Table 22-6; at least 1, 2, 3, 4, 5, 6, 7, 8, 9, 10 or more, 15 or more, or 20 or more, or all microRNAs selected from the group consisting of the microRNAs listed in Table 23-7; at least 1, 2, 3, 4, 5, 6, 7, 8, 9, 10 or more, 15 or more, or 20 or more, or all microRNAs selected from the group consisting of the microRNAs listed in Table 24-8; or at least 1, 2, 3, 4, 5, 6, 7, 8, 9, 10 or more, 15 or more, or 20 or more, or all microRNAs selected from the group consisting of the microRNAs listed in Table 25-8;
wherein the subject may be a subject having or suspected of having gastric cancer;
(viii-a) at least 1, 2, 3, 4, 5, 6, 7, 8, 9, 10 or more, 15 or more, or 20 or more, or all microRNAs selected from the group consisting of the microRNAs listed in Table 4-9; at least 1, 2, 3, 4, 5, 6, 7, 8, 9, 10 or more, 15 or more, or 20 or more, or all microRNAs selected from the group consisting of microRNAs with p < 0.005 or less in Table 4-9; at least 1, 2, 3, 4, 5, 6, 7, 8, 9, 10 or more, 15 or more, or 20 or more, or all microRNAs selected from the group consisting of the microRNAs listed in Table 22-7; at least 1, 2, 3, 4, 5, 6, 7, 8, 9, 10 or more, 15 or more, or 20 or more, or all microRNAs selected from the group consisting of the microRNAs listed in Table 23-8; at least 1, 2, 3, 4, 5, 6, 7, 8, 9, 10 or more, 15 or more, or 20 or more, or all microRNAs selected from the group consisting of the microRNAs listed in Table 24-9; or at least 1, 2, 3, 4, 5, 6, 7, 8, 9, 10 or more, 15 or more, or 20 or more, or all microRNAs selected from the group consisting of the microRNAs listed in Table 25-9;
wherein the subject may be a subject having or suspected of having urothelial carcinoma;
(ix-a) at least 1, 2, 3, 4, 5, 6, 7, 8, 9, 10 or more, 15 or more, or 20 or more, or all microRNAs selected from the group consisting of the microRNAs listed in Table 4-10; at least 1, 2, 3, 4, 5, 6, 7, 8, 9, 10 or more, 15 or more, or 20 or more, or all microRNAs selected from the group consisting of microRNAs with p < 0.005 or less in Table 4-10; at least 1, 2, 3, 4, 5, 6, 7, 8, 9, 10 or more, 15 or more, or 20 or more, or all microRNAs selected from the group consisting of the microRNAs listed in Table 22-8; at least 1, 2, 3, 4, 5, 6, 7, 8, 9, 10 or more, 15 or more, or 20 or more, or all microRNAs selected from the group consisting of the microRNAs listed in Table 23-9; at least 1, 2, 3, 4, 5, 6, 7, 8, 9, 10 or more, 15 or more, or 20 or more, or all microRNAs selected from the group consisting of the microRNAs listed in Table 24-10; or at least 1, 2, 3, 4, 5, 6, 7, 8, 9, 10 or more, 15 or more, or 20 or more, or all microRNAs selected from the group consisting of the microRNAs listed in Table 25-10;
wherein the subject may be a subject having or suspected of having melanoma;
(x-a) at least 1, 2, 3, 4, 5, 6, 7, 8, 9, 10 or more, 15 or more, or 20 or more, or all microRNAs selected from the group consisting of the microRNAs listed in Table 4-11; at least 1, 2, 3, 4, 5, 6, 7, 8, 9, 10 or more, 15 or more, or 20 or more, or all microRNAs selected from the group consisting of microRNAs with p < 0.005 or less in Table 4-11; at least 1, 2, 3, 4, 5, 6, 7, 8, 9, 10 or more, 15 or more, or 20 or more, or all microRNAs selected from the group consisting of the microRNAs listed in Table 22-9; at least 1, 2, 3, 4, 5, 6, 7, 8, 9, 10 or more, 15 or more, or 20 or more, or all microRNAs selected from the group consisting of the microRNAs listed in Table 23-10; at least 1, 2, 3, 4, 5, 6, 7, 8, 9, 10 or more, 15 or more, or 20 or more, or all microRNAs selected from the group consisting of the microRNAs listed in Table 24-11; or at least 1, 2, 3, 4, 5, 6, 7, 8, 9, 10 or more, 15 or more, or 20 or more, or all microRNAs selected from the group consisting of the microRNAs listed in Table 25-11;
wherein the subject may be a subject having or suspected of having ovarian cancer;
(xi-a) at least 1, 2, 3, 4, 5, 6, 7, 8, 9, 10 or more, 15 or more, or 20 or more, or all microRNAs selected from the group consisting of the microRNAs listed in Table 4-12; at least 1, 2, 3, 4, 5, 6, 7, 8, 9, 10 or more, 15 or more, or 20 or more, or all microRNAs selected from the group consisting of microRNAs with p < 0.005 or less in Table 4-12; at least 1, 2, 3, 4, 5, 6, 7, 8, 9, 10 or more, 15 or more, or 20 or more, or all microRNAs selected from the group consisting of the microRNAs listed in Table 22-10; at least 1, 2, 3, 4, 5, 6, 7, 8, 9, 10 or more, 15 or more, or 20 or more, or all microRNAs selected from the group consisting of the microRNAs listed in Table 23-11; at least 1, 2, 3, 4, 5, 6, 7, 8, 9, 10 or more, 15 or more, or 20 or more, or all microRNAs selected from the group consisting of the microRNAs listed in Table 24-12; or at least 1, 2, 3, 4, 5, 6, 7, 8, 9, 10 or more, 15 or more, or 20 or more, or all microRNAs selected from the group consisting of the microRNAs listed in Table 25-12;
wherein the subject may be a subject having or suspected of having thyroid cancer;
(xii-a) at least 1, 2, 3, 4, 5, 6, 7, 8, 9, 10 or more, 15 or more, or 20 or more, or all microRNAs selected from the group consisting of the microRNAs listed in Table 4-13; at least 1, 2, 3, 4, 5, 6, 7, 8, 9, 10 or more, 15 or more, or 20 or more, or all microRNAs selected from the group consisting of microRNAs with p < 0.005 or less in Table 4-13; at least 1, 2, 3, 4, 5, 6, 7, 8, 9, 10 or more, 15 or more, or 20 or more, or all microRNAs selected from the group consisting of the microRNAs listed in Table 22-11; at least 1, 2, 3, 4, 5, 6, 7, 8, 9, 10 or more, 15 or more, or 20 or more, or all microRNAs selected from the group consisting of the microRNAs listed in Table 23-12; at least 1, 2, 3, 4, 5, 6, 7, 8, 9, 10 or more, 15 or more, or 20 or more, or all microRNAs selected from the group consisting of the microRNAs listed in Table 24-13; or at least 1, 2, 3, 4, 5, 6, 7, 8, 9, 10 or more, 15 or more, or 20 or more, or all microRNAs selected from the group consisting of the microRNAs listed in Table 25-13;
wherein the subject may be a subject having or suspected of having thyroid cancer;
(xiii-a) at least 1, 2, 3, 4, 5, 6, 7, 8, 9, 10 or more, 15 or more, or 20 or more, or all microRNAs selected from the group consisting of the microRNAs listed in Table 4-14; at least 1, 2, 3, 4, 5, 6, 7, 8, 9, 10 or more, 15 or more, or 20 or more, or all microRNAs selected from the group consisting of microRNAs with p < 0.005 or less in Table 4-14; at least 1, 2, 3, 4, 5, 6, 7, 8, 9, 10 or more, 15 or more, or 20 or more, or all microRNAs selected from the group consisting of the microRNAs listed in Table 22-12; at least 1, 2, 3, 4, 5, 6, 7, 8, 9, 10 or more, 15 or more, or 20 or more, or all microRNAs selected from the group consisting of the microRNAs listed in Table 23-13; at least 1, 2, 3, 4, 5, 6, 7, 8, 9, 10 or more, 15 or more, or 20 or more, or all microRNAs selected from the group consisting of the microRNAs listed in Table 24-14; or at least 1, 2, 3, 4, 5, 6, 7, 8, 9, 10 or more, 15 or more, or 20 or more, or all microRNAs selected from the group consisting of the microRNAs listed in Table 25-14; or
(xiv-a) at least 1, 2, 3, 4, 5, 6, 7, 8, 9, 10 or more, 15 or more, or 20 or more, or all microRNAs selected from the group consisting of the microRNAs listed in Table 4-15; at least 1, 2, 3, 4, 5, 6, 7, 8, 9, 10 or more, 15 or more, or 20 or more, or all microRNAs selected from the group consisting of microRNAs with p < 0.005 or less in Table 4-15; at least 1, 2, 3, 4, 5, 6, 7, 8, 9, 10 or more, 15 or more, or 20 or more, or all microRNAs selected from the group consisting of the microRNAs listed in Table 22-13; at least 1, 2, 3, 4, 5, 6, 7, 8, 9, 10 or more, 15 or more, or 20 or more, or all microRNAs selected from the group consisting of the microRNAs listed in Table 23-14; at least 1, 2, 3, 4, 5, 6, 7, 8, 9, 10 or more, 15 or more, or 20 or more, or all microRNAs selected from the group consisting of the microRNAs listed in Table 24-15; or at least 1, 2, 3, 4, 5, 6, 7, 8, 9, 10 or more, 15 or more, or 20 or more, or all microRNAs selected from the group consisting of the microRNAs listed in Table 25-15;
(xv-a) data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in the group (ALL); data relating to the relationship between cancer and the expression level(s) of at least 1, 2, 3, 4, 5, 6, 7, 8, 9, 10 or more, 15 or more, or 20 or more, or all microRNAs selected from the group consisting of microRNAs with p < 0.005 or less in the group (ALL); data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 22-1 to 22-13 in the group (ALL); data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 22-1 and 23-1 to 23-14 in the group (ALL); data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 24-1 to 24-15 in the group (ALL); or data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 25-1 to 25-15 in the group (ALL);
(xvi-a) data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in the group (GI); data relating to the relationship between cancer and the expression level(s) of at least 1, 2, 3, 4, 5, 6, 7, 8, 9, 10 or more, 15 or more, or 20 or more, or all microRNAs selected from the group consisting of microRNAs with p < 0.005 or less in the group (GI); data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 22-1 to 22-13 in the group (GI); data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 22-1 and 23-1 to 23-14 in the group (GI); data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 24-1 to 24-15 in the group (GI); or data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 25-1 to 25-15 in the group (GI);
(xvii-a) data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in the group (Hemo); data relating to the relationship between cancer and the expression level(s) of at least 1, 2, 3, 4, 5, 6, 7, 8, 9, 10 or more, 15 or more, or 20 or more, or all microRNAs selected from the group consisting of microRNAs with p < 0.005 or less in the group (Hemo); data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 22-1 to 22-13 in the group (Hemo); data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 22-1 and 23-1 to 23-14 in the group (Hemo); data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 24-1 to 24-15 in the group (Hemo); or data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 25-1 to 25-15 in the group (Hemo);
(xviii-a) data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in the group (Uro); data relating to the relationship between cancer and the expression level(s) of at least 1, 2, 3, 4, 5, 6, 7, 8, 9, 10 or more, 15 or more, or 20 or more, or all microRNAs selected from the group consisting of microRNAs with p < 0.005 or less in the group (Uro); data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 22-1 to 22-13 in the group (Uro); data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 22-1 and 23-1 to 23-14 in the group (Uro); data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 24-1 to 24-15 in the group (Uro); or data relating to the relationship between cancer and the expression level(s) of at least 1, 2, 3, 4, 5, 6, 7, 8, 9, 10 or more, 15 or more, or 20 or more, or all microRNAs selected from the group consisting of the microRNAs listed in Tables 25-1 to 25-15 in the group (Uro); or
(xix-a) a combination thereof.

In this embodiment, the microRNA may be selected from the group consisting of microRNAs with higher expression in a subject having cancer than in a non-cancer subject. In this embodiment, the microRNA may also be selected from the group consisting of microRNAs with lower expression in a subject having cancer than in a non-cancer subject.

In these embodiments, the type of microRNA to be detected and the type of microRNA to be used as an indicator can each independently be, for example, 1 or more, 2 or more, 3 or more, 4 or more, 5 or more, 6 or more, 7 or more, 8 or more, 9 or more, 10 or more, 20 or more, 30 or more, 40 or more, 50 or more, 60 or more, 70 or more, 80 or more, 90 or more, 100 or more, 200 or more, 300 or more, 400 or more, 500 or more, 600 or more, 700 or more, 800 or more, 900 or more, 1000 or more, 1100 or more, 1200 or more, 1300 or more, 1400 or more, 1500 or more, 1600 or more, 1700 or more, 1800 or more, 1900 or more, 2000 or more, 2100 or more, 2200 or more, 2300 or more, 2400 or more, or 2500 or more. In these embodiments, the type of microRNA to be detected and the type of microRNA to be used as an indicator can each independently be, for example, 2500 or less, 2400 or less, 2300 or less, 2200 or less, 2100 or less, 2000 or less, 1900 or less, 1800 or less, 1700 or less, 1600 or less, 1500 or less, 1400 or less, 1300 or less, 1200 or less, 1100 or less, 1000 or less, 900 or less, 800 or less, 700 or less, 600 or less, 500 or less, 400 or less, 300 or less, 200 or less, 100 or less, 90 or less, 80 or less, 70 or less, 60 or less, 50 or less, 40 or less, 30 or less, 20 or less, or 10 or less. The type of microRNA to be detected and the type of microRNA to be used as an indicator may be completely identical, or microRNAs to be detected may be partly used as an indicator.

After detection, cancer can be diagnosed in accordance with the present disclosure using the expression level or the presence or absence of the microRNA as an indicator. A patient diagnosed as having a likelihood of cancer may be treated (for example, cancer therapy, such as chemotherapy, anticancer agent therapy, surgery, immunotherapy, or radiotherapy).

A microRNA may be present in the body fluid in free form and /or in EV-encapsulated form. Thus, according to the present disclosure, a microRNA may be present in urine and an extract of urine in free form and /or in EV (particularly exosome and /or microvesicle) encapsulated form.

A microRNA can be collected by contacting a body fluid with nanowires of a nanowire-embedded device according to the present disclosure. A microRNA can be collected under conditions where nanowires have a positive surface charge. For example, microRNAs in free form and EV-encapsulated form can be captured on nanowires by contacting a body fluid with the nanowires under pH conditions where the nanowires have a positive surface charge. Thus, the pH of a body fluid may be adjusted so that nanowires have a positive surface charge. Alternatively, nanowires may be made of a material having a positive surface charge in a body fluid so as to match the pH of the body fluid. One embodiment of the present disclosure includes adjusting the pH of a body fluid and contacting the body fluid with nanowires of a nanowire-embedded device according to the present disclosure. The pH of a body fluid can be adjusted before, after, or during contact with nanowires. In one embodiment of the present disclosure, the pH of a body fluid, even higher than 2, 3, 4, 5, or the like, may be adjusted to be still higher. The pH of a body fluid, even lower than 10, 9, 8, 7, 6, 5, or the like, may be adjusted to be still lower. In one embodiment of the present disclosure, the pH of urine may be adjusted in the range of 6 to 8. In one embodiment of the present invention, the body fluid is urine and is urine with a pH in the range of 6 to 8 or with a pH adjusted in the range of 6 to 8. In one embodiment of the present invention, the nanowires may be zinc oxide nanowires or nanowires coated with zinc oxide.

In one embodiment/some embodiments of the present disclosure, nanowires may substantially be directly grown on a substrate (that is, may not be embedded) and may be partially embedded in a substrate. The material of the substrate may be any material and may be selected from thermoplastic resins, such as polyethylene, polypropylene, poly(vinyl chloride), poly(vinylidene chloride), polystyrene, poly(vinyl acetate), polytetrafluoroethylene, acrylonitrile-butadiene-styrene (ABS) resins, acrylonitrile-styrene (AS) resins, and acrylic resins (PMMA); and phenolic resins, epoxy resins, melamine resins, urea resins, unsaturated polyester resins, alkyd resins, polyurethane, polyimide, silicone rubbers, poly(methyl methacrylate) (PMMA), and polycarbonate (PC).

In one embodiment/some embodiments of the present disclosure, metals and metal oxides, for example, platinum, aluminum, copper, iron, cobalt, silver, tin, indium, zinc, gallium, chromium, and oxides thereof can be used as a seed layer (catalyst layer) of nanowires. Nanowires can be embedded in a material of a substrate by growing the nanowires from a seed layer and then pouring and curing the material (in liquid form) of the substrate on the nanowires. Subsequently, nanowires exposed from the substrate can be further grown to prepare nanowires embedded in the substrate. Nanowires not exposed from the substrate can be appropriately exposed by cutting and /or polishing. A nanowire-embedded substrate thus prepared can be used in a device according to the present disclosure as a substrate on which nanowires are immobilized, has high physicochemical resistance, and can be useful.

A small RNA in EVs or in free form is captured on nanowires by contacting the nanowires with a body fluid sample. The small RNA in the captured EVs or the small RNAs in free form can be obtained by dissociation from the nanowires using a buffer solution. To obtain the small RNA from the nanowires, for example, a buffer solution containing a nonionic surfactant can be used. The buffer solution may contain an RNase inhibitor.

According to the present disclosure, an extract of urine containing any one or more or all microRNAs in the data S1 (or Table 3) and Table 2 may be substituted with a test liquid or may have the solution composition of the test liquid.

According to the present disclosure, extracts of urine containing 1, 2, 3, 4, 5, 6, 7, 8, 9, 10 or more, 15 or more, or 20 or more, or all microRNAs listed in any of Tables 4-1 to 4-15 and any of the extracts of urine may be substituted with a test liquid or may have the solution composition of the test liquid. In this embodiment, the microRNA may be selected from the group consisting of microRNAs with higher expression in a subject having cancer than in a non-cancer subject. In this embodiment, the microRNA may also be selected from the group consisting of microRNAs with lower expression in a subject having cancer than in a non-cancer subject.

The test solution for a microRNA may have a solution composition suitable for a test for examining the presence or abundance of ncRNA, such as a microRNA. The test liquid may contain, for example, one or more or all selected from the group consisting of surfactants (for example, nonionic surfactants), salts (for example, sodium, potassium, etc.), nucleic acid stabilizers (for example, ribonuclease inhibitors), pH adjusters (for example, buffering agents), and water.

A microRNA can be detected by a miRNA detection method well known to those skilled in the art, such as by a quantitative PCR method, with a microarray for miRNA detection, by an RNA-Seq method, or by a multiplex miRNA profiling method. In the present disclosure, an extract of urine obtained by extracting a microRNA with a device according to the present disclosure may contain 500 or more types of miRNAs, for example. Thus, for example, a microarray for miRNA detection, an RNA-Seq method, and a multiplex miRNA profiling method can be used to confirm the expression of all of these miRNAs. Furthermore, a quantitative PCR method, a multiplex miRNA profiling method, and the like can be used to detect one or some specific miRNAs in an extract of urine.

Detection and analysis of a miRNA with a microarray may include labeling the miRNA (for example, a fluorescent label may be used as a label), preparing a solution for bidization, hybridizing the miRNA in a sample with a miRNA detection reagent, such as a nucleic acid, on the microarray, washing the microarray, and then measuring the amount of label (for example, the amount of fluorescence). For the extracted RNA sample, for example, the quality of the RNA sample may be examined by a method well known to those skilled in the art or with a commercial apparatus and kit (for example, Agilent 2100 Bioanalyzer and RNA LabChip manufactured by Agilent Technologies, Inc.) using the appearance of a peak in the size of 20 to 30 nucleotides as an indicator. A miRNA can be labeled, for example, by a method well known to those skilled in the art or by using a commercial kit (for example, 3D-Gene (trademark) miRNA labeling kit (manufactured by Toray Industries, Inc.)). For example, the analysis of a miRNA with a microarray can be performed using 3D-Gene (trademark) Human/Mouse/Rat/4 animal miRNA Olico chip - 4 plex manufactured by Toray Industries, Inc. in accordance with the manufacturer's instruction manual. A microRNA can be detected and assayed by such a method.

A microarray for detecting a microRNA may be
a microarray with a probe for one or more selected from a microRNA group with higher expression in three subjects having cancer than in any of three non-cancer subjects in the data S1 (or Table 3); a microarray with a probe for one or more selected from a group of microRNAs with higher (for example, twice or more, three times or more, four times or more, five times or more, six times or more, seven times or more, eight times or more, nine times or more, or ten times or more) expression in a subject having cancer than in a non-cancer subject in the data S1 (or Table 3);
a microarray with a probe for one or more selected from a microRNA group with lower expression in three subjects having cancer than in any of three non-cancer subjects in the data S1 (or Table 3); or
a microarray with a probe for one or more selected from a microRNA group with lower (for example, twice or more, three times or more, four times or more, five times or more, six times or more, seven times or more, eight times or more, nine times or more, or ten times or more) expression in three subjects having cancer than any of three non-cancer subjects in the data S1 (or Table 3). In this embodiment, the type of microRNA to be detected (that is, the type of probe on the microarray) can be, for example, 1 or more, 2 or more, 3 or more, 4 or more, 5 or more, 6 or more, 7 or more, 8 or more, 9 or more, 10 or more, 20 or more, 30 or more, 40 or more, 50 or more, 60 or more, 70 or more, 80 or more, 90 or more, 100 or more, 200 or more, 300 or more, 400 or more, 500 or more, 600 or more, 700 or more, 800 or more, 900 or more, 1000 or more, or 1100 or more. In these embodiments, the type of microRNA to be detected (that is, the type of probe on the microarray) can be, for example, 2000 or less, 1900 or less, 1800 or less, 1700 or less, 1600 or less, 1500 or less, 1400 or less, 1300 or less, 1200 or less, 1100 or less, 1000 or less, 900 or less, 800 or less, 700 or less, 600 or less, 500 or less, 400 or less, 300 or less, 200 or less, 100 or less, 90 or less, 80 or less, 70 or less, 60 or less, 50 or less, 40 or less, 30 or less, 20 or less, or 10 or less. A probe for a microRNA in a microarray can be a nucleic acid that can hybridize to the microRNA or a derivative of the nucleic acid and can be appropriately designed by those skilled in the art.

A microarray may contain a microRNA listed in any one or more (or 2 or more, 3 or more, 4 or more, 5 or more, 6 or more, 7 or more, 8 or more, 9 or more, 10 or more, 15 or more, 20 or more, 25 or more, 30 or more, 35 or more, 40 or more, 45 or more, 50 or more, 60 or more, 70 or more, or all) of Tables 4-1 to 4-15, 22-1 to 22-13, 23-1 to 23-14, 24-1 to 24-15, and 25-1 to 25-15. The number of types of microRNAs to be detected with a microarray may be 0 or more types, 1 or more types, 2 or more types, 3 or more types, 4 or more types, 5 or more types, 10 or more types, 15 or more types, 20 or more types, 30 or more types, 40 or more types, 50 or more types, 60 or more types, 70 or more types, 80 or more types, 100 or more types, 200 or more types, 300 or more types, 400 or more types, 500 or more types, 600 or more types, 700 or more types, 800 or more types, 900 or more types, 1000 or more types, 2000 or more types, or 3000 or more types, or all in each table (provided that at least one microRNA in at least one table is used as an indicator). The number of types of microRNAs used as indicators may be more than 50%, 60% or more, 70% or more, 80% or more, 90% or more, or all of the number of types of microRNAs listed in each table. In these embodiments, the type of microRNA to be detected (that is, the type of probe on the microarray) can be, for example, 2500 or less, 2400 or less, 2300 or less, 2200 or less, 2100 or less, 2000 or less, 1900 or less, 1800 or less, 1700 or less, 1600 or less, 1500 or less, 1400 or less, 1300 or less, 1200 or less, 1100 or less, 1000 or less, 900 or less, 800 or less, 700 or less, 600 or less, 500 or less, 400 or less, 300 or less, 200 or less, 100 or less, 90 or less, 80 or less, 70 or less, 60 or less, 50 or less, 40 or less, 30 or less, 20 or less, or 10 or less. A probe for a microRNA in a microarray can be a nucleic acid that can hybridize to the microRNA or a derivative of the nucleic acid and can be appropriately designed by those skilled in the art.

A microarray for detecting a microRNA may also be a microarray containing a probe for one or more selected from the group consisting of 1 or more, 2 or more, 3 or more, 4 or more, 5 or more, 6 or more, 7 or more, 8 or more, 9 or more, 10 or more, 15 or more, or 20 or more) microRNAs selected from the group consisting of hsa-let-7a-3p, hsa-let-7g-5p, hsa-miR-100-3p, hsa-miR-101-3p, hsa-miR-105-3p, hsa-miR-10b-3p, hsa-miR-1185-5p, hsa-miR-1197, hsa-miR-1206, hsa-miR-1243, hsa-miR-1245b-3p, hsa-miR-1246, hsa-miR-1252-3p, hsa-miR-1252-5p, hsa-miR-1258, hsa-miR-126-3p, hsa-miR-126-5p, hsa-miR-1277-3p, hsa-miR-1277-5p, hsa-miR-1279, hsa-miR-1295b-5p, hsa-miR-136-5p, hsa-miR-1-3p, hsa-miR-145-3p, hsa-miR-1468-3p, hsa-miR-152-3p, hsa-miR-153-3p, hsa-miR-155-5p, hsa-miR-186-5p, hsa-miR-18a-5p, hsa-miR-190a-5p, hsa-miR-190b, hsa-miR-19a-5p, hsa-miR-19b-1-5p, hsa-miR-19b-2-5p, hsa-miR-19b-3p, hsa-miR-2053, hsa-miR-205-3p, hsa-miR-2054, hsa-miR-208a-3p, hsa-miR-20a-5p, hsa-miR-219a-1-3p, hsa-miR-219b-3p, hsa-miR-301a-3p, hsa-miR-30d-3p, hsa-miR-30e-5p, hsa-miR-3118, hsa-miR-3123, hsa-miR-3129-5p, hsa-miR-3135a, hsa-miR-3140-3p, hsa-miR-3143, hsa-miR-3152-3p, hsa-miR-3167, hsa-miR-3169, hsa-miR-3183, hsa-miR-3201, hsa-miR-335-5p, hsa-miR-339-3p, hsa-miR-3607-5p, hsa-miR-3616-5p, hsa-miR-3617-5p, hsa-miR-3618, hsa-miR-3662, hsa-miR-3668, hsa-miR-3683, hsa-miR-3686, hsa-miR-3688-3p, hsa-miR-369-3p, hsa-miR-374a-3p, hsa-miR-374a-5p, hsa-miR-376a-5p, hsa-miR-3927-3p, hsa-miR-3942-5p, hsa-miR-4277, hsa-miR-4302, hsa-miR-4432, hsa-miR-4452, hsa-miR-4457, hsa-miR-4474-5p, hsa-miR-4477b, hsa-miR-4480, hsa-miR-4495, hsa-miR-4504, hsa-miR-4509, hsa-miR-450a-1-3p, hsa-miR-450b-3p, hsa-miR-455-5p, hsa-miR-4633-3p, hsa-miR-4639-5p, hsa-miR-4668-3p, hsa-miR-4671-3p, hsa-miR-4679, hsa-miR-4693-3p, hsa-miR-4699-5p, hsa-miR-4704-3p, hsa-miR-4714-3p, hsa-miR-4720-3p, hsa-miR-4735-5p, hsa-miR-4757-3p, hsa-miR-4760-5p, hsa-miR-4772-5p, hsa-miR-4777-5p, hsa-miR-4781-3p, hsa-miR-4782-3p, hsa-miR-4782-5p, hsa-miR-4790-3p, hsa-miR-4791, hsa-miR-4795-5p, hsa-miR-4796-3p, hsa-miR-4798-5p, hsa-miR-4799-3p, hsa-miR-4802-5p, hsa-miR-499a-5p, hsa-miR-5009-3p, hsa-miR-5009-5p, hsa-miR-506-5p, hsa-miR-507, hsa-miR-510-3p, hsa-miR-513b-3p, hsa-miR-514a-5p, hsa-miR-5191, hsa-miR-544a, hsa-miR-545-5p, hsa-miR-548a-3p, hsa-miR-548a-5p, hsa-miR-548aa, hsa-miR-548t-3p, hsa-miR-548ac, hsa-miR-548ad-5p, hsa-miR-548ae-5p, hsa-miR-548ae-3p, hsa-miR-548ag, hsa-miR-548ah-5p, hsa-miR-548ak, hsa-miR-548al, hsa-miR-548am-3p, hsa-miR-548ao-5p, hsa-miR-548ap-5p, hsa-miR-548aq-5p, hsa-miR-548at-3p, hsa-miR-548au-5p, hsa-miR-548az-5p, hsa-miR-548b-5p, hsa-miR-548c-5p, hsa-miR-548o-5p, hsa-miR-548am-5p, hsa-miR-548g-5p, hsa-miR-548x-5p, hsa-miR-548aj-5p, hsa-miR-548j-5p, hsa-miR-548k, hsa-miR-548m, hsa-miR-548p, hsa-miR-548t-5p, hsa-miR-548u, hsa-miR-548v, hsa-miR-548x-3p, hsa-miR-549a, hsa-miR-553, hsa-miR-556-3p, hsa-miR-5579-5p, hsa-miR-5583-5p, hsa-miR-559, hsa-miR-5590-3p, hsa-miR-5590-5p, hsa-miR-5591-5p, hsa-miR-561-5p, hsa-miR-568, hsa-miR-5687, hsa-miR-569, hsa-miR-5692c, hsa-miR-5697, hsa-miR-5700, hsa-miR-570-3p, hsa-miR-577, hsa-miR-582-5p, hsa-miR-587, hsa-miR-590-3p, hsa-miR-6079, hsa-miR-6128, hsa-miR-618, hsa-miR-621, hsa-miR-628-5p, hsa-miR-633, hsa-miR-648, hsa-miR-6508-3p, hsa-miR-651-3p, hsa-miR-652-3p, hsa-miR-6733-5p, hsa-miR-6811-5p, hsa-miR-6844, hsa-miR-6854-5p, hsa-miR-7161-3p, hsa-miR-759, hsa-miR-7-5p, hsa-miR-7852-3p, hsa-miR-873-5p, hsa-miR-876-5p, hsa-miR-9-3p, hsa-miR-95-3p, hsa-miR-95-5p, hsa-miR-9-5p, hsa-miR-4525, hsa-miR-6760-5p, hsa-miR-4538, hsa-miR-5698, hsa-miR-5189-5p, hsa-miR-671-5p, hsa-miR-936, hsa-miR-4307, hsa-miR-6815-5p, hsa-miR-6076, hsa-miR-708-5p, hsa-miR-3187-5p, hsa-miR-3144-3p, hsa-miR-4691-5p, hsa-miR-4700-5p, hsa-miR-576-5p, hsa-miR-3652, hsa-miR-598-3p, hsa-miR-7151-3p, hsa-miR-7162-5p, hsa-miR-1229-5p, hsa-miR-6746-5p, hsa-miR-4762-5p, hsa-miR-1307-3p, hsa-miR-1273g-3p, hsa-miR-4428, hsa-miR-6515-5p, hsa-miR-1273g-5p, hsa-miR-433-3p, hsa-miR-452-3p, hsa-miR-3917, hsa-miR-1224-5p, hsa-miR-6892-5p, hsa-miR-520d-5p, hsa-miR-5092, hsa-miR-30c-1-3p, hsa-miR-1293, hsa-miR-7851-3p, hsa-miR-3934-5p, hsa-miR-3122, hsa-miR-3177-3p, hsa-miR-4800-5p, hsa-miR-4436b-3p, hsa-miR-3928-3p, hsa-miR-630, hsa-miR-628-3p, hsa-miR-6796-5p, hsa-miR-3605-5p, hsa-miR-30e-3p, hsa-miR-6801-5p, hsa-miR-6131, hsa-miR-448, hsa-miR-6834-5p, hsa-miR-3156-5p, hsa-miR-517-5p, hsa-miR-4520-5p, hsa-miR-2276-3p, hsa-miR-3591-3p, hsa-miR-4727-3p, hsa-miR-4499, hsa-miR-1471, hsa-miR-1273h-5p, hsa-miR-4539, hsa-miR-3655, hsa-miR-4690-5p, hsa-miR-6871-5p, hsa-miR-4496, hsa-miR-6772-5p, hsa-miR-4686, hsa-miR-1297, hsa-miR-450a-2-3p, hsa-miR-6745, hsa-miR-3153, hsa-miR-27a-3p, hsa-miR-106b-5p, hsa-miR-519c-5p, hsa-miR-523-5p, hsa-miR-518e-5p, hsa-miR-522-5p, hsa-miR-519a-5p, hsa-miR-519b-5p, hsa-miR-4676-5p, hsa-miR-650, hsa-miR-3922-5p, hsa-miR-4489, hsa-miR-4638-5p, hsa-miR-185-3p, hsa-miR-2467-3p, hsa-miR-4453, hsa-let-7e-5p, hsa-miR-296-3p, hsa-miR-8074, hsa-miR-6876-5p, hsa-miR-6086, hsa-miR-4474-3p, hsa-miR-4776-3p, hsa-miR-1250-5p, hsa-miR-4260, hsa-miR-6747-5p, hsa-miR-181d-3p, hsa-miR-4535, hsa-miR-3177-5p, hsa-miR-4533, hsa-miR-4635, hsa-miR-4776-5p, hsa-miR-4257, hsa-miR-3160-3p, hsa-miR-5702, hsa-miR-4306, hsa-miR-4483, hsa-miR-4472, hsa-miR-3064-3p, hsa-miR-4710, hsa-miR-4293, hsa-miR-449c-5p, hsa-miR-5589-5p, hsa-miR-4443, hsa-miR-4448, hsa-miR-3935, hsa-miR-16-5p, hsa-miR-181a-5p, hsa-miR-147b, hsa-miR-4767, hsa-miR-4677-5p, hsa-miR-6776-5p, hsa-miR-7109-5p, hsa-miR-4669, hsa-miR-4486, hsa-miR-1587, hsa-miR-363-3p, hsa-miR-5701, hsa-miR-921, hsa-miR-5581-5p, hsa-miR-4259, hsa-miR-203b-5p, hsa-miR-3653-3p, hsa-miR-4657, hsa-miR-5589-3p, hsa-miR-2681-3p, hsa-miR-6826-5p, hsa-miR-4647, hsa-miR-4540, hsa-miR-361-5p, hsa-miR-4784, hsa-miR-6861-5p, hsa-miR-6812-5p, hsa-miR-608, hsa-miR-1912, hsa-miR-554, hsa-miR-519a-3p, hsa-miR-891a-3p, hsa-miR-6872-5p, hsa-miR-4781-5p, hsa-miR-4273, hsa-miR-4769-5p, hsa-miR-505-5p, hsa-miR-7158-3p, hsa-miR-527, hsa-miR-518a-5p, hsa-miR-1273f, hsa-miR-624-5p, hsa-miR-125a-3p, hsa-miR-6830-5p, hsa-miR-520d-3p, hsa-miR-6757-5p, hsa-miR-4785, hsa-miR-6068, hsa-miR-1272, hsa-miR-4654, hsa-miR-564, hsa-miR-563, hsa-miR-4300, hsa-miR-4665-5p, hsa-miR-892c-3p, hsa-miR-6778-5p, hsa-miR-4419b, hsa-miR-329-3p, hsa-miR-212-5p, hsa-miR-616-3p, hsa-miR-4269, hsa-miR-4487, hsa-miR-1180-5p, hsa-miR-7850-5p, hsa-miR-492, hsa-miR-718, hsa-miR-1972, hsa-miR-4296, hsa-miR-516b-3p, hsa-miR-516a-3p, hsa-miR-4320, hsa-miR-4529-5p, hsa-miR-656-3p, hsa-miR-6500-3p, hsa-miR-766-5p, hsa-miR-466, hsa-miR-6777-5p, hsa-miR-1909-5p, hsa-miR-187-3p, hsa-miR-8079, hsa-miR-30a-5p, hsa-miR-5002-3p, hsa-miR-6833-5p, hsa-miR-4756-3p, hsa-let-7f-2-3p, hsa-miR-5708, hsa-miR-4721, hsa-miR-8085, hsa-miR-3591-5p, hsa-miR-6859-5p, hsa-miR-5006-5p, hsa-miR-4747-5p, hsa-miR-6827-5p, hsa-miR-3612, hsa-miR-4701-3p, hsa-miR-4418, hsa-miR-4449, hsa-miR-181d-5p, hsa-miR-3619-3p, hsa-miR-6817-5p, hsa-miR-6759-5p, hsa-miR-3654, hsa-miR-3164, hsa-miR-491-3p, hsa-miR-3176, hsa-miR-4683, hsa-miR-6807-5p, hsa-miR-5588-5p, hsa-miR-302c-5p, hsa-miR-4522, hsa-miR-1199-5p, hsa-miR-372-3p, hsa-miR-891a-5p, hsa-miR-1306-3p, hsa-miR-6894-5p, hsa-miR-3613-3p, hsa-miR-548ba, hsa-miR-6795-5p, hsa-miR-6794-5p, hsa-miR-585-3p, hsa-miR-194-3p, hsa-miR-5010-5p, hsa-miR-2052, hsa-miR-614, hsa-miR-6751-5p, hsa-miR-5089-3p, hsa-miR-1289, hsa-miR-668-5p, hsa-miR-3622b-5p, hsa-miR-6887-5p, hsa-miR-4524a-3p, hsa-miR-3689a-3p, hsa-miR-4650-5p, hsa-miR-3689b-3p, hsa-miR-3689c, hsa-miR-624-3p, hsa-miR-6129, hsa-miR-216a-5p, hsa-miR-520b, hsa-miR-4755-5p, hsa-miR-323a-5p, hsa-miR-6726-5p, hsa-miR-516b-5p, hsa-miR-7106-5p, hsa-miR-653-5p, hsa-miR-494-5p, hsa-miR-6780b-5p, hsa-miR-1910-3p, hsa-miR-3160-5p, hsa-miR-5093, hsa-miR-99a-3p, hsa-miR-8071, hsa-miR-4659a-3p, hsa-miR-597-3p, hsa-miR-4458, hsa-miR-6847-5p, hsa-miR-4436a, hsa-miR-17-5p, hsa-miR-185-5p, hsa-miR-4681, hsa-miR-8077, hsa-miR-4440, hsa-let-7g-3p, hsa-miR-375, hsa-miR-10a-5p, hsa-miR-520e, hsa-miR-6072, hsa-miR-3145-5p, hsa-miR-6723-5p, hsa-miR-4692, hsa-miR-4796-5p, hsa-miR-6849-5p, hsa-miR-4430, hsa-miR-6499-5p, hsa-miR-3151-5p, hsa-miR-182-5p, hsa-miR-195-5p, hsa-miR-3681-5p, hsa-miR-6864-5p, hsa-miR-4711-3p, hsa-miR-380-3p, hsa-miR-374c-5p, hsa-miR-758-5p, hsa-miR-6842-5p, hsa-miR-8080, hsa-miR-520f-3p, hsa-miR-4646-5p, hsa-miR-103a-3p, hsa-miR-3189-3p, hsa-miR-4691-3p, hsa-miR-877-5p, hsa-miR-6769b-5p, hsa-miR-5580-5p, hsa-miR-1911-5p, hsa-miR-519b-3p, hsa-miR-660-5p, hsa-miR-4644, hsa-miR-6133, hsa-miR-138-1-3p, hsa-miR-193a-5p, hsa-miR-222-3p, hsa-miR-603, hsa-miR-23b-3p, hsa-miR-5089-5p, hsa-miR-6799-5p, hsa-miR-6715a-3p, hsa-miR-6869-3p, hsa-miR-4445-3p, hsa-miR-542-5p, hsa-miR-1286, hsa-miR-96-3p, hsa-miR-4743-5p, hsa-miR-4724-3p, hsa-miR-4262, hsa-miR-4507, hsa-miR-7160-5p, hsa-miR-605-3p, hsa-miR-200c-3p, hsa-miR-582-3p, hsa-miR-6868-5p, hsa-miR-6773-5p, hsa-miR-1185-1-3p, hsa-miR-3182, hsa-miR-6082, hsa-miR-150-3p, hsa-miR-5002-5p, hsa-miR-487a-5p, hsa-miR-8058, hsa-miR-4529-3p, hsa-miR-3713, hsa-miR-8075, hsa-miR-6877-5p, hsa-miR-4325, hsa-miR-3130-5p, hsa-miR-616-5p, hsa-miR-1322, hsa-miR-6793-5p, hsa-miR-4694-5p, hsa-miR-29a-3p, hsa-miR-6127, hsa-miR-758-3p, hsa-miR-2114-5p, hsa-miR-515-5p, hsa-miR-196a-5p, hsa-miR-4639-3p, hsa-miR-4799-5p, hsa-miR-4498, hsa-miR-6766-5p, hsa-miR-1255b-2-3p, hsa-miR-380-5p, hsa-miR-4419a, hsa-miR-3622a-5p, hsa-let-7b-5p, hsa-miR-4650-3p, hsa-miR-29b-1-5p, hsa-miR-3137, hsa-miR-1447, hsa-miR-6500-5p, hsa-miR-3 663-5p, hsa-miR-4451, hsa-miR-4422, hsa-miR-662, hsa-miR-122-3p, hsa-miR-6828-5p, hsa-miR-6884-5p, hsa-miR-4502, hsa-miR-374b-5p, hsa-miR-4462, hsa-miR-4478, hsa-miR-431-3p, hsa-miR-23b-5p, hsa-miR-7975, hsa-miR-5011-5p, hsa-miR-3691-5p, hsa-miR-6768-3p, hsa-miR-591, hsa-miR-8086, hsa-miR-372-5p, hsa-miR-5189-3p, hsa-miR-107, hsa-miR-6891-5p, hsa-miR-3138, hsa-miR-6503-3p, hsa-miR-297, hsa-miR-370-5p, hsa-miR-3944-3p, hsa-miR-425-5p, hsa-miR-3978, hsa-miR-148a-5p, hsa-miR-4684-5p, hsa-miR-34a-3p, hsa-miR-1263, hsa-miR-769-5p, hsa-miR-4437, hsa-miR-2277-3p, hsa-miR-659-5p, hsa-miR-661, hsa-miR-6788-5p, hsa-miR-15b-3p, hsa-miR-1205, hsa-miR-1468-5p, hsa-miR-34a-5p, hsa-miR-3120-5p, hsa-miR-1247-3p, hsa-miR-454-5p, hsa-miR-656-5p, hsa-miR-626, hsa-miR-4441, hsa-miR-6780a-5p, hsa-miR-6769a-5p, hsa-miR-1298-5p, hsa-miR-3157-5p, hsa-miR-6814-5p, hsa-miR-500a-5p, hsa-miR-1321, hsa-miR-4481, hsa-miR-611, hsa-miR-1273a, hsa-miR-337-5p, hsa-miR-203a-5p, hsa-miR-4479, hsa-miR-4719, hsa-miR-8083, hsa-miR-132-3p, hsa-miR-20b-5p, hsa-miR-4252, hsa-miR-429, hsa-miR-378b, hsa-miR-134-5p, hsa-miR-6741-5p, hsa-miR-1193, hsa-miR-143-5p, hsa-miR-515-3p, hsa-miR-4774-3p, hsa-miR-8064, hsa-miR-1183, hsa-miR-5684, hsa-miR-1226-5p, hsa-miR-539-5p, hsa-miR-580-5p, hsa-miR-622, hsa-miR-3907, hsa-miR-6504-5p, hsa-miR-4653-3p, hsa-miR-4706, hsa-miR-4515, hsa-miR-3919, hsa-miR-3187-3p, hsa-miR-1271-5p, hsa-miR-6767-5p, hsa-miR-4804-5p, hsa-miR-1257, hsa-miR-3126-5p, hsa-miR-6731-5p, hsa-miR-3149, hsa-miR-4717-3p, hsa-miR-509-5p, hsa-miR-215-3p, hsa-miR-1178-3p, hsa-miR-1282, hsa-miR-3174, hsa-miR-197-5p, hsa-miR-376c-3p, hsa-miR-5696, hsa-miR-3121-5p, hsa-miR-8082, hsa-miR-6823-5p, hsa-miR-4704-5p, hsa-miR-3616-3p, hsa-miR-6881-5p, hsa-miR-4661-5p, hsa-miR-22-5p, hsa-miR-6730-5p, hsa-let-7e-3p, hsa-miR-1238-5p, hsa-miR-526b-5p, hsa-miR-3163, hsa-miR-4514, hsa-miR-4699-3p, hsa-miR-7154-5p, hsa-miR-4521, hsa-miR-96-5p, hsa-miR-4754, hsa-miR-450a-5p, hsa-miR-4459, hsa-miR-922, hsa-miR-7153-5p, hsa-miR-29b-2-5p, hsa-miR-6738-3p, hsa-miR-6790-5p, hsa-miR-4470, hsa-miR-4732-3p, hsa-miR-4709-3p, hsa-miR-5588-3p, hsa-miR-93-3p, hsa-miR-6820-5p, hsa-miR-655-5p, hsa-miR-6514-3p, hsa-miR-1-5p, hsa-miR-10b-5p, hsa-miR-3193, hsa-miR-3976, hsa-miR-3678-3p, hsa-miR-3912-5p, hsa-miR-4708-3p, hsa-miR-5192, hsa-miR-140-5p, hsa-miR-4285, hsa-miR-3124-5p, hsa-miR-198, hsa-miR-3659, hsa-miR-3689a-5p, hsa-miR-3689b-5p, hsa-miR-3689e, hsa-miR-4643, hsa-miR-552-3p, hsa-miR-3186-3p, hsa-miR-424-3p, hsa-miR-578, hsa-miR-4304, hsa-miR-504-3p, hsa-miR-595, hsa-miR-4501, hsa-miR-4746-3p, hsa-miR-362-5p, hsa-miR-4297, hsa-miR-6821-3p, hsa-miR-3074-5p, hsa-miR-4503, hsa-miR-4518, hsa-miR-6715b-3p, hsa-miR-6736-5p, hsa-miR-7843-3p, hsa-miR-4420, hsa-miR-3927-5p, and hsa-miR-7157-5p.

In this embodiment, the type of microRNA to be detected (that is, the type of probe on the microarray) can be, for example, 1 or more, 2 or more, 3 or more, 4 or more, 5 or more, 6 or more, 7 or more, 8 or more, 9 or more, 10 or more, 15 or more, 20 or more, 30 or more, 40 or more, 50 or more, 60 or more, 70 or more, 80 or more, 90 or more, 100 or more, 200 or more, 300 or more, 400 or more, 500 or more, 600 or more, 700 or more, 800 or more, 900 or more, 1000 or more, 1100 or more, 1200 or more, 1300 or more, 1400 or more, 1500 or more, 1600 or more, 1700 or more, 1800 or more, 1900 or more, 2000 or more, 2100 or more, 2200 or more, 2300 or more, 2400 or more, or 2500 or more. In these embodiment, the type of microRNA to be detected (that is, the type of probe on the microarray) can be, for example, 2500 or less, 2400 or less, 2300 or less, 2200 or less, 2100 or less, 2000 or less, 1900 or less, 1800 or less, 1700 or less, 1600 or less, 1500 or less, 1400 or less, 1300 or less, 1200 or less, 1100 or less, 1000 or less, 900 or less, 800 or less, 700 or less, 600 or less, 500 or less, 400 or less, 300 or less, 200 or less, 100 or less, 90 or less, 80 or less, 70 or less, 60 or less, 50 or less, 40 or less, 30 or less, 20 or less, or 10 or less. A probe for a microRNA in a microarray can be a nucleic acid that can hybridize to the microRNA or a derivative of the nucleic acid and can be appropriately designed by those skilled in the art.

### EXAMPLES

### Example 1: Preparation of Nanowires Fixed on Polydimethylsiloxane (PDMS)

A Si (100) substrate (Advantech Co., Ltd.) was washed (see Fig. 6a) and was coated with a positive photoresist (OFPR8600, Tokyo Ohka Kogyo Co., Ltd.), and a channel pattern was then formed by photolithography (see Fig. 6b). A Cr layer (catalyst layer) with a thickness of 140 nm was deposited on the substrate by sputtering (see Fig. 6b). After removal of the photoresist (see Fig. 6d), the Cr layer was thermally oxidized at 400°C for 2 hours. The Cr layer was used as a seed layer for the growth of ZnO nanowires. ZnO nanowires were grown by immersing the substrate in a mixed solution of 15 mM hexamethylenetetramine (HMTA, Wako Pure Chemical Industries Ltd.) and 15 mM zinc nitrate hexahydrate (Thermo Fisher Scientific Inc.) at 95°C for 3 hours (see Fig. 6e). The nanowires grown on the substrate were washed with Millipore water and were air-dried overnight in a vacuum desiccator. PDMS (Silpot 184, Dow Corning Corp.) was then poured onto the substrate on which the nanowires were grown, and was cured (see Fig. 6f). The PDMS was removed from the substrate to transfer the nanowires from the substrate to the PDMS. The transferred nanowires were uniformly and deeply embedded in the PDMS with their heads slightly exposed (see Fig. 6h) (this embedded state may be herein referred to as "embedded"). The heads provided growth points for secondary nanowires. The secondary nanowires were grown by immersing the PDMS in a mixed solution of 15 mM HMTA and 15 mM zinc nitrate hexahydrate at 95°C for 3 hours (see Fig. 6i). The PDMS substrate embedded with the nanowires was washed with Millipore water and was then air-dried in a vacuum desiccator. The diameter of the nanowires and the distance between the nanowires were measured with a field-emission scanning electron microscope (FESEM) (SUPRA 40VP, Carl Zeiss).

### Nanowire-embedded Microfluidic Device Capable of In Situ Extraction of Extracellular Vesicle (EV) Encapsulated miRNA in Urine

A nanowire-embedded microfluidic device for in situ extraction of an EV-encapsulated miRNA in urine was prepared by bonding a nanowire-embedded PDMS substrate to a PDMS substrate with a herringbone structure. The PDMS substrate with the herringbone structure had a microchannel (2 mm in width, 2 cm in length, and 50 µm in height) with a herringbone structure 12 µm in height. The surfaces of the nanowire-embedded PDMS substrate and the PDMS substrate with the herringbone structure were treated with a plasma etching apparatus (Meiwafosis Co., Ltd.) to bond them. The bonding device was heated at 180°C for 3 minutes to achieve strong bonding (see Fig. 6J). An inlet and an outlet of the PDMS with the herringbone structure were connected to a poly(ether ketone) (PEEK) tube [0.5 mm (outer diameter) x 0.26 mm (inner diameter), 10 cm in length, Institute of Microchemical Technology Co., Ltd.]. Thus, a nanowire-embedded microfluidic device capable of in situ extraction of the title (hereinafter also referred to simply as "a nanowire-embedded device" or "a device according to the present disclosure") was prepared. The microfluidic herringbone structure contributed to improved collection efficiency (see Fig. 15).

### EDS Elemental Mapping of Cross-Sectional FESEM Image

Elemental mappings of PDMS without nanowires, PDMS with embedded nanowires (that is, PDMS without exposed nanowires), and ZnO nanowire-embedded PDMS were obtained with a FESEM with an EDS function (JSM-7610F, Jeol). Accelerating voltages of 5 keV and 30 keV were used for top view images and cross-sectional images, respectively. Each image was 512 x 384 pixels, and the delay time for each pixel was 0.1 seconds. Images were integrated for 100 cycles. Peaks of Si Kα (1.739 keV) and Zn Lα (1.012 keV) were selected to construct an elemental mapping image. Elemental mapping of ZnO-Al₂O₃ core-shell nanowires were performed with a FESEM with an EDS function at an accelerating voltage of 30 keV. To prepare a scanning transmission electron microscopy (STEM) sample, nanowires were cut from a substrate with a conventional cutting knife, were collected, and were transferred onto a TEM grid (a Cu mesh with a carbon microgrid, JEOL) by a contact method. A STEM image was 512 x 384 pixels, and the delay time for each pixel was 0.1 ms. Images were integrated for 100 cycles. Peaks of Zn Kα(8.630 keV), O Kα(0.525 keV), and Al Kα(1.486 keV) were selected to construct an elemental mapping image.

### In Situ Extraction of EV-Encapsulated miRNA in Urine Using Nanowire-Embedded Microfluidic Device

Commercial urine (human urine of a single donor, Innovative Research Inc.) was centrifuged (15 min, 4°C, 3000g) before use to remove apoptotic bodies (see Literature 5). 1 mL of a urine sample was then introduced into the nanowire-embedded device using a syringe pump (KDS-200, KD Scientific Inc.) at a flow rate of 50 µL/min. A miRNA was extracted from EVs collected on the nanowires by introducing cell lysis buffer M [20 mM tris-HCl (pH 7.4), 200 mM sodium chloride, 2.5 mM magnesium chloride, and 0.05 w/v% NP-40; Wako Pure Chemical Industries Ltd.] into the nanowire-embedded device using a syringe pump at a flow rate of 50 µL/min. The lysis buffer was also used at a flow rate of 50 µL/min in an experiment to study separation of nanowires (Figs. 1I to K and Fig. 9).

### Extraction of miRNA in Urine Using EV Collection and Ultracentrifugation (Ultracentrifugation Method)

Commercial urine (human urine of single donor) was centrifuged (15 min, 4°C, 3000g) before use to remove apoptotic bodies (see Literature 5). The urine was then centrifuged (15 min, 4°C, 12, 000g) to remove cellular debris (see Literature 29). 20 mL of the urine sample was then ultracentrifuged (2 hours, 4°C, 110, 000g) (see Literature 29). The supernatant was removed, and 20 mL of phosphate-buffered saline (PBS, Thermo Fisher Scientific Inc.) filtered through a 0.22-µm filter was added to collected EVs, which were then further ultracentrifuged (70 minutes, 4°C, 110, 000g). The supernatant was removed, and 20 mL of phosphate-buffered saline (PBS, Thermo Fisher Scientific Inc.) filtered through a 0.22-µm filter was added to collected EVs, which were then subjected to the third ultracentrifugation (70 minutes, 4°C, 110, 000g). Finally, the supernatant was removed, and the lysis buffer was added to extract a miRNA.

### EV Collection and miRNA Extraction Using Commercial Kit (Polymer Precipitation Method)

Commercial urine (human urine of single donor) was centrifuged (15 min, 4°C, 3 000g) before use to remove apoptotic bodies. EVs were collected from 1 mL of the urine sample using a kit (ExoQuick-TC, System Biosciences Inc.) in accordance with the manufacturer's instruction manual. Finally, the lysis buffer was added to extract a miRNA.

### Microarray Analysis of miRNA Expression

A 3D-Gene (Toray Industries) human miRNA chip was used for the expression profile of a miRNA. The miRNA extracted with the lysis buffer was purified using SeraMir Exosome RNA Purification Column Kit (System Biosciences Inc.) in accordance with the manufacturer's instruction manual. 15 □L of the purified miRNA was analyzed for miRNA profiling using 3D-Gene Human miRNA Oligo chip ver. 21 (Toray Industries). In the microarray analysis of miRNA expression, each signal intensity corresponds to one type of miRNA. The expression level of each miRNA is expressed as the signal intensity (after the background is subtracted) of all miRNAs in each microarray. To compare the miRNA expression level between extraction and centrifugation with the nanowire-embedded device and extraction with the commercial kit in the same urine miRNA sample, the signal intensity was normalized throughout. A scatter diagram was drawn for intensities of 10 or more (intensity in extraction with the nanowire-embedded device/intensity in extraction by ultracentrifugation or with the commercial kit) normalized throughout. Thus, each point on the scatter diagram is a normalized intensity. To compare the miRNA expression level between extraction and centrifugation with the nanowire-embedded device and extraction with the commercial kit in the same urine miRNA sample, the signal intensity was log2-transformed. To compare the miRNA between a cancer donor urine sample and a non-cancer donor urine sample, the intensity in each sample normalized throughout was log2-transformed. The normalized intensity was shown in black (intensity = 5), blue (intensity ≦2), and yellow (intensity ≧8) on a heat map.

### Zeta Potential of EVs

### After the ultracentrifugation step, the zeta potential of EVs was measured with a dynamic light scattering apparatus (Zetasizer Nano ZS, Malvern Instruments Ltd.).

### Size Distribution and Concentration of Free Suspended Matter in Urine

The size distribution and concentration of free suspended matter in urine were measured with a nanoparticle analyzing system (NanoSight, Malvern Instruments Ltd.). The concentrations of free suspended matter in untreated urine, in a flow-through fraction of the urine treated with the nanowire-embedded device, and in the urine after ultracentrifugation were 2.6 x 10¹² ml-1, 5.8 x 10⁹ ml-1, and 3.5 x 10⁹ ml-1, respectively. The size distribution and concentration of free suspended matter in urine were measured with a nanoparticle detector (qNano, Meiwafosis Co., Ltd.) equipped with a 100-nm nanopore membrane (NP100, Meiwafosis Co., Ltd.). The concentrations of free suspended matter in untreated urine, in a flow-through fraction of the urine treated with the nanowire-embedded device, and in the urine after ultracentrifugation were 1.4 x 10¹² ml-1, 2.4 x 10¹⁰ ml-1, and 2.5 x 10¹⁰ ml-1, respectively.

### Fluorescent Molecular Label of EVs

EVs were labeled with PKH26 (excitation light/fluorescence = 551/567 nm, Sigma-Aldrich Co. LLC), which is a fluorescent molecule capable of passing through the lipid bilayer of EVs. 5 mg of PKH26 was added to 1.5 x 10⁸ ml-1 of EVs in Millipore water (240 µL) filtered through a 0.22-µm filter. The EVs labeled with PKH26 were introduced into the nanowire-embedded device using a syringe pump at a flow rate of 10 µl/min, and Millipore water was then introduced into the nanowire-embedded device at the same flow rate to remove uncollected EVs (EVs not bonded to the nanowires). Finally, fluorescence of EVs was observed with a fluorescence microscope (AZ100, Nikon Corp.). The nanowire-embedded device was then separated, and the nanowires with the collected EVs were observed with FESEM (SUPRA 40VP, Carl Zeiss).

### Detection of Membrane Protein

After EVs were introduced into the nanowire-embedded device, PBS was introduced to remove uncollected EVs. Subsequently, a 1% bovine serum albumin (BSA) solution (Kirkegaard & Perry Laboratories Inc.) was introduced into the device and was allowed to stand for 15 minutes. The device was washed with PBS, and a mouse anti-human CD63 monoclonal antibody (10 µg/ml, Santa Cruz Biotechnology Inc.) or mouse anti-human CD81 monoclonal antibody (10 µg/ml, Abcam PLC) labeled with Alexa Fluor 488 was introduced into the device and was allowed to stand for 15 minutes. To detect CD81, the device was washed, and a goat anti-mouse IgG polyclonal antibody labeled with Alexa Fluor 488 was introduced into the device as a secondary antibody and was then allowed to stand for 15 minutes. Finally, the device was washed with PBS, and the fluorescence intensity was observed with a fluorescence microscope (Olympus Co., Ltd.). PBS instead of the EV sample was used to obtain the background value. For detection in a 96-well plate (Nunc Co., Ltd.), an EV sample was injected into the wells of the plate and was allowed to stand for 6 hours, after which the wells were washed with PBS. A 1% BSA solution was introduced into the wells of the plate and was allowed to stand for 90 minutes. The wells were then washed with PBS, and a mouse anti-human CD63 monoclonal antibody (10 µg/ml) or mouse anti-human CD81 antibody (10 µg/ml) labeled with Alexa Fluor 488 was introduced into the wells of the plate and was allowed to stand for 45 minutes. To detect CD81, in addition to the above, the wells of the plate were washed with PBS, and a goat anti-mouse IgG polyclonal antibody (5 µg/ml) labeled with Alexa Fluor 488 was introduced into the wells of the plate as a secondary antibody and was allowed to stand for 45 minutes. Finally, the wells of the plate were washed with PBS, and the fluorescence intensity was observed with a plate reader (POLARstar OPTIMA, BMG Labtech Japan Ltd.). PBS instead of the EV sample was used to obtain the background value.

### Preparation of ZnO-Al₂O₃ Core-Shell Nanowires

The ZnO nanowires thus prepared were coated using an atomic layer deposition apparatus (Savannah G2, Ultratech Inc.). Al₂O₃ layers were stacked by repeating 100 cycles of flowing trimethylaluminum and a H₂O precursor into a chamber containing the nanowires in the atomic layer deposition apparatus and reacting them at 150_{°}C

### Urine Sample Information and In Situ Extraction of EV-Encapsulated miRNA Using Nanowire-Embedded Device

The following urine samples (BioreclamationIVT) were used: urine samples from non-cancer patients (53, 60, and 50 years of age), lung cancer patients (68 years of age, stage 2b; 54 years of age, stage 3a; 50 years of age, stage 3b), pancreatic cancer patients (56 years of age, stage 2a; 61 years of age, stage 2a; 74 years of age, stage 3), liver cancer patients (49 years of age, stage 3; 64 years of age, stage 3a; 18 years of age, stage 3c), bladder cancer patients (63 years of age, stage 1; 65 years of age, stage 1; 67 years of age, stage 0a), and prostate cancer (58 years of age, stage 4; 57 years of age, stage 2a; 54 years of age, stage 2b). These urine samples were centrifuged (15 min, 4_{°} C, 3000g) to remove apoptotic bodies before use. 1 ml of each urine sample was introduced into the nanowire-embedded device using a syringe pump at a flow rate of 50 µl/min. A miRNA was in situ extracted from EVs collected on the nanowires by introducing the lysis buffer into the device using a syringe pump at a flow rate of 50 ml/min.

### Identification of miRNA in Urine as Cancer Biomarker

The 95% confidence interval was calculated using (average) ± 1.96 x (average x CV/100) from a Z score of 1.96 (95% confidence level and 5% significance level) and the relationship of the variation variable (CV) (without a specific value) to log2 (intensity) provided by Toray Industries, Inc. Using X% for CV when log2 (intensity) = 3, the upper limit of the confidence interval was 8 + 0.16X. The CV values for log2 (intensity) = 5 or 6 were 0.7X% and 0.5X% from the relationship. Considering the 5% significance level, the CV for each example was 40 and less than 71%. In Fig. 5, CV values of more than 70% were not reasonable, and three logarithmic intensity differences were determined against a statistically significant threshold.

### Results

To establish a methodology of collecting EV-encapsulated miRNAs, nanowires embedded in a microfluidic substrate have been developed. These nanowires have an important function as a solid phase for electrostatic collection of EVs and subsequently in in situ extraction of EV-encapsulated miRNAs (see Fig. 1A).

### Nanowire-Embedded PDMS Substrate

A nanowire-embedded PDMS substrate was prepared in four steps (see Literature 40 and Fig. 6). In a first step, nanowires were grown from a thermally oxidized chromium layer on a Si substrate. In a second step, uncured PDMS was poured on the growing nanowires. In a third step, the PDMS with the nanowires was cured and separated to form nanowire-embedded PDMS (see Fig. 1B). In a fourth step, nanowires were grown from the embedded nanowires (see Fig. C). The nanowire-embedded PDMS substrate was prepared in this way. A field-emission scanning electron microscope (FESEM) image of a vertical cross section of the embedded nanowires showed that the nanowires were uniformly and deeply embedded in the PDMS with their heads slightly exposed (see Fig. 1B), and the heads provided growth starting points for secondary nanowires (see Figs. 1C and 7). Elemental mapping in the energy dispersive X-ray analysis (EDS) of a cross-sectional FESEM image of PDMS without nanowires was compared with that of the nanowire-embedded PDMS (see Fig. 8) to confirm that ZnO nanowires were embedded in the nanowire-embedded PDMS. Furthermore, ESD elemental mapping of vertical cross-sectional and overall FESEM images and cross-sectional FESEM images showed that secondary nanowires were grown on the embedded nanowires. Thus, the nanowire-embedded PDMS substrate was successfully prepared. To promote contact between the nanowires and EVs and prevent pressure drop, a PDMS substrate with a microfluidic herringbone structure with a channel height (50 µm) larger than the nanowire length (2 µm) was used as a cover of the nanowire-embedded PDMS substrate (the herringbone structure ensures good convection and dispersion of the solution) (see Literature 41) (see Fig. 1D). A nanowire-embedded device for extracting EV-encapsulated miRNAs in urine was completed by attaching the nanowire-embedded PDMS substrate to the PDMS substrate with the microfluidic herringbone structure and coupling it to a poly(ether ketone) (PEEK) tube for introducing and collecting a urine sample (see Fig. 1D). The nanowires embedded in the PDMS substrate had mechanical stability against exposure to the lysis buffer and could be prevented from being separated from the substrate, which occurs in nanowires not embedded in PDMS (see Figs. IE and F and Fig. 9).

### Microarray Analysis of miRNA Expression

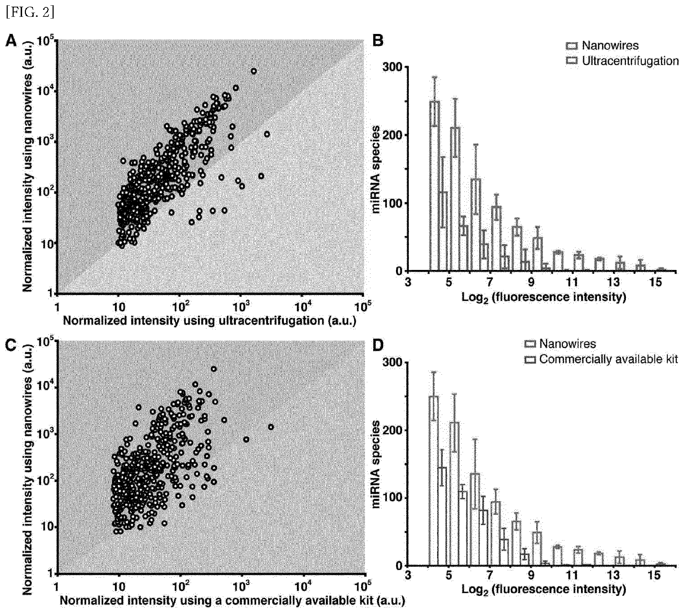

Microarray analysis of miRNA expression (2565 types) showed that the nanowire-embedded device can extract more types of miRNA (approximately 1,000 types) than a known ultracentrifugation method or a known polymer precipitation method using a commercial kit (see Figs. 2 and 10A). Completion of EV-encapsulated miRNAs in urine within 40 minutes (20 minutes for adsorption and 20 minutes for extraction) was demonstrated by introducing a urine sample (1 ml) into the device and then introducing the lysis buffer (1 ml) into the device. In contrast, extraction of a miRNA from EVs by an ultracentrifugation method required 20 ml of urine and required 5 hours or more for adsorption and extraction. It has been reported that the commercial kit used in this study is superior to other EV isolation methods in terms of the yield of a small RNA (see B and C in Fig. 10) (see Literature 22). Thus, the kit was used to extract a miRNA by suspending EVs collected from 1 ml of urine in 1 ml of the lysis buffer. This operation took 14 hours or more for collection and extraction. A scatter diagram and a histogram showed that the expression level of a miRNA extracted with a nanowire-embedded device according to the present disclosure was much higher than a miRNA obtained by the ultracentrifugation method, despite urine consumption 20 times or less the urine consumption in the extraction of the miRNA by the ultracentrifugation method (see Figs. 2A and 2B). Normally, the use of 20 times or more of urine should result in the amount of miRNA obtained by the ultracentrifugation method higher than the amount of miRNA obtained with a device according to the present disclosure, but the results were reversed. A device according to the present disclosure yielded five times the amount of miRNA obtained by the ultracentrifugation method (see Fig. 2A) and could extract more types of miRNA (the amount of miRNA extracted with a device according to the present disclosure: 749, 822, 1111 types; the amount of miRNA extracted by the centrifugation method: 171 261, 352 types) (see Fig. 2B). Furthermore, a scatter diagram and a histogram showed that the amount of miRNA extracted with a device according to the present disclosure was much higher than the amount of miRNA obtained with the kit, despite the same volume of urine sample consumed (see Figs. 2C and 2D). A device according to the present disclosure yielded four times the amount of miRNA obtained with the kit (see Fig. 2C) and could extract more types of miRNA (the amount of miRNA extracted with a device according to the present disclosure: 749, 822, 1111 types; the amount of miRNA extracted by the centrifugation method: 337, 355, 491 types) (see Fig. 2D). It was therefore concluded that a device according to the present disclosure is superior to the known methods (the ultracentrifugation method or the polymer precipitation method) in terms of miRNA extraction efficiency (see Fig. 2), extraction time (see Figs. 10A and 10 b), and small RNA extraction efficiency (see Fig. 10C).

### EV Collection Capacity

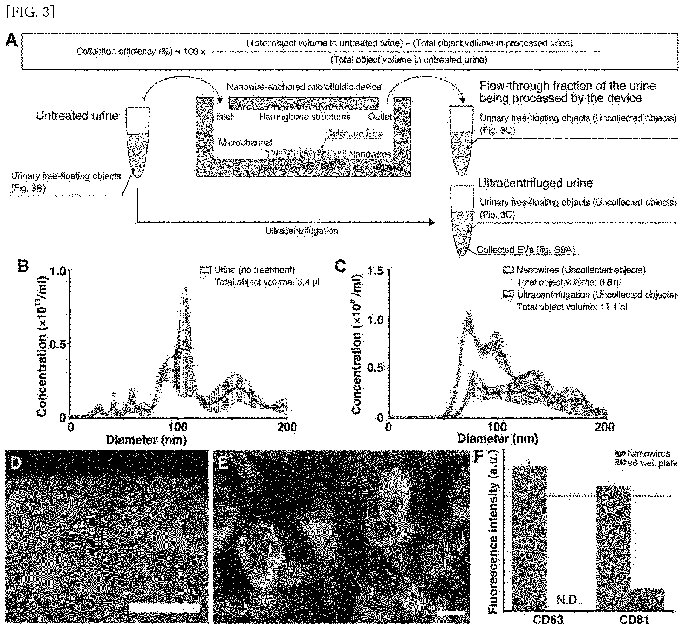

Examining the ability of a device according to the present disclosure to collect EVs showed that a device according to the present disclosure can efficiently collect EVs (see Fig. 3). More specifically, considering that the total amount of free suspended matter in untreated urine, in a flow-through fraction of urine treated with a device according to the present disclosure (input: 1 ml), and in urine treated with the ultracentrifugation method (input: 20 ml) were 3.4 µl, 8.8 nl, and 11.1 nl, respectively, the collection efficiency of suspended matter with a device according to the present disclosure was estimated to be 99% or more, and a device according to the present disclosure could collect a larger volume than the ultracentrifugation method (see Figs. 3A to 3C) (the volume of collected EVs was calculated by subtracting the volume of treated urine from the volume of untreated urine). After fluorescently labeled EVs were observed, the nanowire-embedded PDMS was separated from the PDMS substrate with the microfluidic herringbone structure to obtain its FESEM image. This showed that the free suspended matter collected by the nanowires contained EVs (see Figs. 3C and 3D). To further confirm that the free suspended matter collected by the nanowires contained EVs, CD63 and CD81, which are membrane proteins expressed on EVs and are known to be expressed on exosome membranes, were detected (see Literature 2 and 42 to 44). The fluorescence intensity (concentration: 1.4 x 10⁸ ml-1) of EVs in the urine collected on the nanowires (or the wells of the 96-well plate) showed that only the nanowires could collect these membrane proteins (see Fig. 3E). This indicates that a device according to the present disclosure can efficiently collect EVs on the nanowires. Next, instead of the ZnO nanowires, ZnO-Al₂O₃ core-shell nanowires were prepared in which a ZnO core was completely covered with an Al₂O₃ layer with a thickness of 10 nm (see Fig. 11). Because Al₂O₃ has an isoelectric point of approximately 7.5, the ZnO-Al₂O₃ core-shell nanowires have almost electrically neutral (possibly slightly positive or slightly negative) surface at pH 6 to 8 (see Literature 45 and Literature 46). Thus, the ZnO-Al₂O₃ core-shell nanowires were used to examine whether the surface charge state of the nanowires had a dominant effect on the collection of EVs (see Fig. 12). The ZnO nanowires have a positively charged surface at pH 6 to 8 because ZnO has an isoelectric point of approximately 9.5, whereas EVs in urine have a negatively charged surface at pH 6 to 8. Thus, it can be understood that the ZnO nanowires can efficiently collect EVs (see Literature 47 and Literature 48). These results further showed that the ZnO nanowires can collect free suspended matter with a diameter of up to 200 nm, such as EVs, in urine at a collection efficiency of 99% or more.

### Merits of EV Collection with Nanowires

A value obtained by adding the amount of material collected by the ultracentrifugation method (that is, exosomes (9.0 nl; see Fig. 14A)) to the amount of material not collected (11.1 nl; Fig. 3C) is not equal to the volume of suspended matter contained in untreated urine (3.4 µl; Fig. B). Although it is not yet easy to completely distinguish between exosomes and microvesicles, this suggests that exosomes are mainly collected by the ultracentrifugation method (see Literature 4, Literature 5, and Literature 29). Furthermore, many of the EVs collected by the ultracentrifugation method are pressed against the inner wall of the ultracentrifugation tube and may be fused together and collapse. In this process, miRNAs encapsulated in microvesicles may be released to the surroundings. The mechanism of EV collection by ultracentrifugation is based on the balance between the force applied to the material to be collected and the density of the material. Thus, the experimental conditions for ultracentrifugation may not be suitable for collection of released miRNAs.

In contrast, the mechanism of EV collection by nanowires is based on electrostatic interaction between positively charged nanowires and a negatively charged material, which enables the nanowires to collect exosomes and microvesicles. Furthermore, a nucleic acid, such as a miRNA, has a surface property of being negatively charged at pH 6 to 8, and nanowires can therefore collect EV-free miRNAs (free miRNAs). However, free miRNAs cannot be collected by the ultracentrifugation method or the polymer precipitation method. Furthermore, the collection efficiency of miRNAs from the nanowires by introducing the lysis buffer was almost 100% (see Fig. 14B). Nanowires with a positively charged surface will have a significant advantage in collecting negatively charged materials in urine, such as exosomes, microvesicles, and free miRNAs. Considering characteristics such as collection efficiency, selectivity of the collected material, and ability to collect miRNAs in urine (see Table 1), the ultracentrifugation method has high collection efficiency, high selectivity of the collected material (can collect only exosomes), and low collection ability, whereas a device according to the present disclosure has high collection efficiency, low selectivity (can widely collect exosomes, microvesicles, and free miRNAs), and high collection ability. Furthermore, a kit using the polymer precipitation method cannot completely satisfy these characteristics. Although a protamine precipitation method for EVs is performed by overnight incubation with protamine/poly(ethylene glycol) at 4□C (similar to ExoQuick), comparing with such an isolation technique based on the electric charge of EVs (see Literature 49), the extraction of an EV-encapsulated miRNA with a device according to the present disclosure has an advantage in requiring only 40 minutes (20 minutes for collection or adsorption and 20 minutes for extraction) at room temperature. Furthermore, comparing with a charge-based isolation technique for a free nucleic acid using a chitosan polymer with an amine group that produces a positively charged surface at pH 6.3 or less (see Literature 50), the extraction of an EV-encapsulated miRNA with a device according to the present disclosure has the advantage that nanowires are certainly positively charged in urine at pH 6 to 8. From these findings, collection by electrostatic interaction of EVs ( and free miRNAs) with ZnO nanowires and the mechanical stability of nanowires embedded in PDMS during the application of the lysis buffer are important mechanisms, and early diagnosis and timely medical examination of diseases are expected on the basis of the analysis of a miRNA in urine.

**[Table 1] Comparisons of Three miRNA Extraction Methods**

| | Device of present disclosure | ExoQuick^{™} | Ultracentrifugation method |
|---|---|---|---|
| Objects to be collected | Exosomes microvesicles EV-free miRNAEV | Exosomes microvesicles | Exosomes |
| Collection mechanism | Electrostatic interaction between nanowire surface charge and collected objects | According to the kit manufacturer's instructions, capture of objects with a diameter of 60-180 nm by polymer | Balance between the applied force and the density of collected objects matrial |
| Necessary volume of sample | 1 ml | 1 ml | 1ml for quantification of small molecule RNA; 20 ml for filing of urinary miRNA |
| Process time | 40 min | 870 min | 300 min |
| Collection efficiency ( yield of small molecule RNA ) | 0.194 ± 0.028 ng/µl | 0.120 ± 0.015 ng/µl | 0.159 ± 0.077 ng/µl |
| Types of identified miRNA extracted from urine | 749 types, 822 types, 1111 types (n = 3) | 337 types, 355 types, 491 types (n = 3) | 171 types, 261 types, 352 types (n = 3) 200 types ~ 300 types^{∗} |

| | | | |
|---|---|---|---|
| ^{∗} According to Chang et al. ( 31) | | | |

Identification of miRNAs from Urine Samples of Various Cancer Patient Donors Next, whether an undiscovered miRNA could be identified in the urine of various cancer patient donors was examined (see Fig. 4). In this examination, miRNAs were extracted from urine samples of cancer patient donors and healthy persons (non-cancer donors) using a device according to the present disclosure and were compared on heat maps. As shown in Fig. 4, various miRNAs with different expression levels for different cancers could be observed. A comparison between the cancer patient donors and the non-cancer donors showed miRNAs decreased or increased in the urine in relation to the cancers (see Fig. 5 and Table 2). It was suggested from the heat maps that each of the decreased and increased miRNAs is a new indicator for cancer, and a combination thereof can be a new indicator for cancer.

**[Table 2] Cancer-Related Human miRNA Shown in FIG. 5**

| Cancer types (downregulation /overexpression) | miRNA name | Physiological function |
|---|---|---|
| Lung cancer | miR-3127-3p^{∗} | |
| (downregulation | miR-3130-5p^{∗} | |
| ) | miR-3131^{∗} | |
| | miR-3141 ^{∗} | |
| | miR-3150b-5p^{∗} | |
| | miR-3151-3p^{∗} | |
| | miR-3151-5p^{∗} | |
| | miR-3154^{∗} | |
| | miR-3160-3p^{∗} | |
| | miR-3160-5p^{∗} | |
| | miR-378a-5p | Cell proliferation suppression and induction of apoptosis (Lit. 52) |
| | miR-520c-3p | Tumor suppressor (Lit. 53-57) |
| | miR-526b-3p | Tumor suppressor (Lit. 58) |
| | miR-3150a-3p | |
| | miR-3162-5p | |
| | miR-4254 | |
| Lung cancer (overexpression) | miR-3117-5p^{∗} | |
| | miR-3118^{∗} | |
| | miR-3121-3p^{∗} | |
| | miR-3121-5p^{∗} | |
| | miR-3126-5p^{∗} | |
| | miR-3128^{∗} | |
| | miR-3133^{∗} | |
| | miR-3134^{∗} | |
| | miR-3136-3p^{∗} | |
| | miR-3136-5p^{∗} | |
| | miR-3139^{∗} | |
| | miR-3142^{∗} | |
| | miR-3143^{∗} | |
| | miR-3145-3p^{∗} | |
| | miR-3163^{∗} | Suppression of growth of non-small cell lung cancer cells (Lit. 59) |
| | miR-3166^{∗} | |
| | miR-3167^{∗} | |
| | miR-16-1-3p | Suppression of gastric cancer cell infiltration and metastasis (Lit. 60) |
| | miR-424-3p | miRNAs associated with metastasis (Lit. 61) |
| | miR-519c-5p | |
| | miR-525-5p | |
| | miR-55lb-5p | |
| | miR-558 | Increased tumorigenicity (Lit. 62) |
| | miR-921 | |
| | miR-942-3p | |
| | miR-3126-3p | |
| | miR-3127-5p | Suppression of growth of non-small cell lung cancer cells (Lit. 63) |
| | miR-3129-5p | |
| | miR-3144-5p | |
| | miR-3150a-5p | |
| | miR-3152-5p | |
| | miR-3155a | |
| | miR-3157-3p | |
| | miR-3159 | |
| | miR-3165 | |
| | miR-3678-3p | |
| | miR-4321 | |
| | miR-4521 | Significantly increased miRNAs in cancer stem cells (Lit. 64) |
| | miR-4800-3p | |
| | miR-4999-5p | |
| | miR-5096 | |
| | miR-5187-5p | |
| | miR-6874-5p | |
| Pancreatic cancer (downregulation ) | miR-372-3p^{∗} | Tumor suppressor (Lit. 65) |
| | miR-378b^{∗} | |
| | miR-520b^{∗} | Suppression of cell migration and infiltration (Lit. 66) |
| | miR-1266-3p^{∗} | |
| | miR-3605-5p^{∗} | |
| | miR-3612^{∗} | |
| | miR-4645-3p^{∗} | |
| | miR-4694-3p^{∗} | |
| | miR-4752^{∗} | |
| | miR-6816-3p^{∗} | |
| | miR-8087^{∗} | |
| | let-7f-2-3p | |
| | miR-15a-3p | Induction of apoptosis in human cancer cell lines (Lit. 67) |
| | miR-20a-3p | |
| | miR-33b-3p | |
| | miR-34c-5p | Tumor suppressor (Lit. 68) |
| | miR-93-5p | |
| | miR-130a-5p | |
| | miR-135a-5p | Suppression of cancer metastasis (Lit. 69) |
| | miR-135b-5p | |
| | miR-185-5p | Tumor suppressor (Lit. 70) |
| | miR-203a-3p | |
| | miR-302d-5p | |
| | miR-337-3p | Tumor suppressor (Lit. 71) |
| | miR-378c | |
| | miR-422a | Downregulated in colorectal cancer (Lit. 72) |
| | miR-449c-5p | |
| | miR-483-5p | |
| | miR-506-3p | Induces differentiation (Lit. 73) |
| | miR-511-5p | |
| | miR-520c-3p | Tumor suppressor (Lit. 53-57) |
| | miR-654-3p | |
| | miR-668-5p | |
| | miR-670-5p | |
| | miR-671-3p | |
| | miR-744-3p | |
| | miR-1178-3p | |
| | miR-1254 | |
| | miR-1284 | Downregulated at the site of lymph node metastasis (Lit. 74) |
| | miR-1323 | Adjusts radiation system (Lit. 75) |
| | miR-2116-5p | |
| | miR-2355-3p | |
| | miR-3132 | |
| | miR-3138 | |
| | miR-3164 | |
| | miR-3186-3p | |
| | miR-3189-3p | |
| | miR-3198 | |
| | miR-3200-5p | |
| | miR-3657 | |
| | miR-3667-5p | |
| | miR-3680-5p | |
| | miR-3692-5p | |
| | miR-37 13 | |
| | miR-3921 | |
| | miR-3936 | |
| | miR-4273 | Increases risk of colorectal cancer (Lit. 76) |
| | miR-4299 | |
| | miR-43 06 | |
| | miR-43 16 | |
| | miR-43 19 | |
| | miR-442 1 | |
| | miR-4429 | |
| | miR-4435 | |
| | miR-444 1 | |
| | miR-4473 | |
| | miR-45 06 | |
| | miR-4633-5p | |
| | miR-4658 | |
| | miR-4733-5p | |
| | miR-4733-3p | |
| | miR-5004-3p | |
| | miR-5194 | |
| | miR-5197-5p | |
| | miR-5571-5p | |
| | miR-6083 | |
| | miR-6717-5p | |
| | miR-6720-5p | |
| | miR-6767-3p | |
| | miR-6781-3p | |
| | miR-6811-3p | |
| | miR-6821-3p | |
| | miR-6828-5p | |
| | miR-6832-5p | |
| | miR-6837-3p | |
| | miR-6841-5p | |
| | miR-6853-5p | |
| | miR-6871-3p | |
| | miR-6875-5p | |
| | miR-6878-5p | |
| | miR-7112-3p | |
| | miR-7703 | |
| | miR-7848-3p | |
| | miR-7856-5p | |
| Pancreatic | let-7i-3p | |
| cancer | miR-183-5p | Promotes cancer growth, infiltration and metastasis (Lit. 77) |
| (overexpression) | miR-202-5p | Increases protein expression of TGFBR1 and TGFBR2 (Lit. 78) |
| | miR-409-5p | Promotes cancer formation (Lit. 79) |
| | miR-4661-5p | |
| | miR-4800-3p | |
| | miR-5587-5p | |
| Liver cancer (downregulation ) | let-7i-2-3p miR-520c-3p | Tumor suppressor (Lit. 53-57) |
| Liver cancer | miR-4521^{∗} | |
| (overexpression) | let-7c-3p | |
| | let-7i-5p | |
| | miR-16-1-3p | Suppresses gastric cancer cell infiltration and metastasis (Lit. 60) |
| | miR-26a-1-3p | |
| | miR-28-5p | Suppresses the expression of insulin-like growth factor (Lit. 80) |
| | miR-105-5p | |
| | miR-195-3p | |
| | miR-200b-5p | |
| | miR-219a-2-3p | |
| | miR-297 | Promotes cell proliferation and infiltration (Lit. 81) |
| | miR-300 | Suppression of transforming gene expression in pituitary gland (Lit. 82) |
| | miR-330-3p | Promotion of infiltration and metastasis (Lit. 83) |
| | miR-374b-5p | |
| | miR-431-5p | |
| | miR-454-5p | Promotes cancer formation (84) |
| | miR-513c-5p | |
| | miR-548ax | |
| | miR-593-5p | |
| | miR-623 | |
| | miR-664a-5p | |
| | miR-942-3p | |
| | miR-1205 | |
| | miR-1276 | |
| | miR-1288-3p | |
| | miR-1297 | Promotion of cell proliferation (Lit. 85) |
| | miR-3678-3p | |
| | miR-4283 | |
| | miR-4295 | Promotion of cell proliferation and infiltration (Lit. 86) |
| | miR-4439 | |
| | miR-4524b-5p | |
| | miR-4703-3p | |
| | miR-4768-5p | |
| | miR-4800-3p | |
| | miR-5187-5p | |
| | miR-5696 | |
| | miR-7161-5p | |
| Bladder cancer | let-7f-2-3p | |
| (downregulation | miR-520c-3p | Tumor suppressor (Lit. 53-57) |
| ) | miR-4783-5p | |
| Bladder cancer (overexpression) | miR-16-1-3p | Suppresses gastric cancer cell infiltration and metastasis (Lit. 60) |
| | mR-23b-3p | Controls chemical resistance of gastric cancer cells (Lit. 87) |
| | miR-28-5p | Suppresses the expression of insulin-like growth factor (Lit. 80) |
| | imR-92a-2-5p | |
| | miR-142-3p | Promotion of malignant phenotype (Lit. 88) |
| | miR-195-3p | Promotes tumorigenesis and suppresses apoptosis (Lit. 89) |
| | imR-196b-5p | |
| | imR-299-3p | Reduces Oct4 gene expression (Lit. 90) |
| | miR-492 | |
| | imR-513b-5p | |
| | miR-601 | |
| | imR-619-5p | |
| | imR-1285-3p | |
| | nuR-3155a | |
| | imR-3162-5p | |
| | imR-3678-3p | |
| | miR-4283 | |
| | miR-4295 | Promotion of proliferation of bladder cancer cells (Lit. 91) |
| | miR-4311 | |
| | miR-4531 | |
| | miR-5096 | |
| | miR-5187-5p | |
| Prostate cancer (downregulation ) | miR-15a-3p | Induction of apoptosis in human cancer cell lines (Lit. 67) |
| | miR-135b-5p | |
| | miR-520c-3p | Tumor suppressor (Lit. 53-57) |
| | miR-4783-5p | |
| | miR-7849-3p | |
| Prostate cancer (overexpression) | miR-4531^{∗} | |
| | miR-28-5p | Suppresses the expression of insulin-like growth factor (Lit. 80) |
| | miR-103a-2-5p | |
| | miR-105-5p | Suppression of expression of tumor suppressor gene (Lit. 92) |
| | miR-124-3p | Control of cell proliferation, infiltration and apoptosis (Lit. 93) |
| | miR-151a-5p | Migration and infiltration of cancer cells (Lit. 94) |
| | miR-151b | |
| | miR-200a-5p | |
| | miR-300 | Promotion of cancer growth and infiltration (Lit. 95) |
| | miR-424-3p | |
| | miR-519c-5p | |
| | miR-551b-5p | |
| | miR-617 | |
| | miR-873-3p | |
| | miR-921 | |
| | miR-1288-3p | |
| | miR-3124-5p | |
| | miR-3155a | |
| | miR-3917 | |
| | miR-4283 | |
| | miR-4727-3p | |
| | miR-5096 | |
| | miR-5187-5p | |
| | miR-6074 | |
| | miR-6874-5p | |
| | miR-6892-5p | |

On the basis of a comparison with published results (see Literature 52 to Literature 95), an attempt was made to identify statistically significantly decreased miRNAs and increased miRNAs. As a result, miRNAs extracted with a device according to the present disclosure included two miRNA groups: a miRNA group with an unknown physiological function and a miRNA group with a reported physiological function. The miRNA group with an unknown physiological function can further be broadly divided into two subgroups: a cancer-related miRNA group and an artifact group derived from free miRNAs. The miRNA group with a reported physiological function was also broadly divided into two subgroups: a miRNA group positively correlated with disease outcome, such as miR-520c-3p (miRNA that is a tumor suppressor and was decreased in the urine from all the cancer patients) (see Literature 53 to Literature 57) and a miRNA group with a counter-intuitive correlation between function and disease, such as miR-16-1-3p (miRNA that suppresses the invasion and metastasis of gastric cancer cells and was overexpressed in the urine from liver cancer patients and bladder cancer patients) (see Literature 60). In counter-intuitive cases, the following three possibilities were considered: a first possibility is that the patient has a corresponding risk; a second possibility is that the present study cannot cover the relationship between a miRNA and the cancer type; and a third possibility is that a miRNA is attributed to an artifact. Comparing previously reported miRNAs in urine extracted with commercial kits from 4 ml of the urine from bladder cancer patients (shown as top 25 cell-free and exosome-derived increased miRNAs) (see Literature 96) and overexpressed miRNAs obtained with a device according to the present disclosure, the present study has found 20 types of miRNAs, including miR-4454 (see the data S1 or Table 3). However, the miRNAs disclosed in Literature 96 are not identified as candidates for an indicator for cancer in the present disclosure. This is because, in the present disclosure, the miRNAs disclosed in Literature 96 were also found in the urine of the non-cancer donors, and no significant difference was observed in logarithmic fluorescence intensity between the urine of the non-cancer donors and the urine of the bladder cancer patients.

### Example 2: Further Analysis

Urine was collected from 100 patients including stage-I to -IV human cancer patients and 100 healthy persons, and microRNAs were extracted from the urine in the same manner as in Example 1. The human cancer patients included 100 each of lung cancer, breast cancer, kidney cancer, leukemia, lymphoma, pancreatic cancer, prostate cancer, gastric cancer, urothelial carcinoma, melanoma, ovarian cancer, and thyroid cancer patients. Urine was collected from each patient, and microRNAs were extracted from the urine in the same manner as in Example 1.

The stages of lung cancer patients were as follows:
Number of lung cancer patients of each stage
Stage-IA: 10
Stage-IB: 14
Stage-II: 18
Stage-III: 41
Stage-IV: 15
Unknown: 2
log2 (fluorescence intensity) was calculated from the fluorescence intensity of each miRNA, and a histogram was created. Fig. 16D shows the results. The histogram was created for miRNAs detected in all the 200 samples and for miRNAs detected in any one or more samples.

A pie chart was then created on the basis of the p-value in the Student t-test for the urine of the lung cancer patients and for the urine of healthy persons. Fig. 16E shows the results. Furthermore, patients and healthy persons were randomly divided into 20 groups. A criterion formula was derived by logistic regression from the results of 19 groups randomly selected from them, and the remaining group was predicted. This procedure was performed 20 cycles. An ROC curve was plotted on the basis of the resulting data. Fig. 16F shows the ROC curve. Furthermore, the value derived using the criterion formula (cancer risk) was taken as the vertical axis, and the cancer risk of each subject was plotted for each stage. The cancer risk indicates the likelihood of cancer. Fig. 16G shows the resulting plot. Fig. 16G shows that any of stage-I to -IV can be effectively predicted using miRNAs. Next, among the miRNAs used in the criterion formula, miRNAs with an absolute weighting factor of more than 0.01 were extracted, and the relationship between the weighting factor and miRNAs was illustrated. Fig. 17A shows the results. miR-4520-3p, miR-1250-5p, miR-6070, miR-4453, miR-449c-5p, miR-4307, miR-106a-3p, miR-4311, miR-1265, and miR-6828-5p was identified as a factor that has a weighting factor of more than 0.5 and that is particularly strongly related to lung cancer. miR-630, miR-6837-3p, miR624-3p, miR-642a-5p, miR339-5p, miR452-3p, miR-30e-3p, miR-938, and miR-20a-3p was also identified as a factor that has a weighting factor of less than -1 and that is strongly related to healthy persons rather than lung cancer.

Furthermore, a heat map was created from the relationship between log2 (fluorescence intensity) and miRNAs shown in Fig. 17A. Fig. 17B shows the heat map.

Patients and healthy persons were randomly divided into 20 groups. A criterion formula was derived by logistic regression (using the expression levels of all the miRNAs) from the results of 19 groups randomly selected from them, and the remaining group was predicted. This procedure was performed for 50 cycles. The accuracy (percentage of correct answers), sensitivity, and specificity are shown. Furthermore, the procedure was performed 50 cycles using 19 miRNAs with an absolute weighting factor of more than 0.5 in the criterion formula at this time (that is, miRNAs composed ofmiR-4520-3p, miR-1250-5p, miR-6070, miR-4453, miR-449c-5p, miR-4307, miR-106a-3p, miR-4311, miR-1265, miR-6828-5p, miR-630, miR-6837-3p, miR-624-3p, miR-642a-5p, miR-339-5p, miR-452-3p, miR-30e-3p, miR-938, and miR-20a-3p and considered to be of high importance), and the accuracy (percentage of correct answers), sensitivity, and specificity were shown. As a result, lung cancer could be predicted with very high accuracy (percentage of correct answers), sensitivity, and specificity.

The prediction was performed for each stage. All the miRNAs detected in the urine were used as miRNAs. A criterion formula was derived as described above using patients other than stage-I and healthy persons as teacher data to predict stage-I patients and derive the cancer risk value (an indicator of the likelihood of cancer) and an ROC curve based on the cancer risk value. Figs. 18B and 18A show the results. Next, a criterion formula was derived as described above using patients other than stage-II and healthy persons as teacher data to predict stage-II patients and derive the cancer risk value and an ROC curve based on the cancer risk value. Fig. 18D and 18C show the results. Next, a criterion formula was derived as described above using patients other than stage-III and healthy persons as teacher data to predict stage-III patients and derive the cancer risk value and an ROC curve based on the cancer risk value. Fig. 18F and 18E show the results. Furthermore, a criterion formula was derived as described above using patients other than stage-IV and healthy persons as teacher data to predict stage-IV patients and derive the cancer risk value and an ROC curve based on the cancer risk value. Fig. 18H and 18G show the results. Thus, it was clear that lung cancer could be predicted using miRNAs in any of stage-I to -IV.

Fig. 19A shows the number of miRNAs detected in each sample of lung cancer tissues and the urine of the lung cancer patients. Fig. 19A also shows the number of miRNAs detected in both the lung cancer tissues and the urine of the lung cancer patients.

Fig. 19B shows the data of the log2 (fluorescence intensity) of 19 miRNAs with an absolute weighting factor of more than 0.5 compared between the tissues and the urine.

Among the microRNAs detected in the above experiments, Student t-test was performed on the lung cancer patients and healthy persons, and a microRNA group with p < 0.05 is listed in Table 4-1. Table 4-1 shows that the expression level of each microRNA is statistically significantly different between the urine of the lung cancer patients and the urine of the healthy persons.

The expression level of each microRNA in Table 4-1 was used as an indicator to predict whether a subject from which the urine was derived was a lung cancer patient or a healthy person. An intermediate value between the average mRNA level of the urine of the lung cancer patients and the average mRNA level of the urine of the healthy persons was used as a threshold for prediction. As a result, as shown in Table 4-1, even a single miRNA could be used to predict a lung cancer patient with high sensitivity or specificity.

Likewise, Student t-test was performed on breast cancer patients and healthy persons, and a microRNA group with p < 0.05 is listed in Table 4-2. Student t-test was performed on kidney patients and healthy persons, and a microRNA group with p < 0.05 is listed in Table 4-3. Student t-test was performed on leukemia patients and healthy persons, and a microRNA group with p < 0.05 is listed in Table 4-4. Student t-test was performed on lymphoma patients and healthy persons, and a microRNA group with p < 0.05 is listed in Table 4-5. Student t-test was performed on pancreatic cancer patients and healthy persons, and a microRNA group with p < 0.05 is listed in Table 4-6. Student t-test was performed on prostate cancer patients and healthy persons, and a microRNA group with p < 0.05 is listed in Table 4-7. Student t-test was performed on gastric cancer patients and healthy persons, and a microRNA group with p < 0.05 is listed in Table 4-8. Student t-test was performed on urothelial carcinoma patients and healthy persons, and a microRNA group with p < 0.05 is listed in Table 4-9. Student t-test was performed on melanoma patients and healthy persons, and a microRNA group with p < 0.05 is listed in Table 4-10. Student t-test was performed on ovarian cancer patients and healthy persons, and a microRNA group with p < 0.05 is listed in Table 4-11. Student t-test was performed on thyroid cancer patients and healthy persons, and a microRNA group with p < 0.05 is listed in Table 4-12. Student t-test was performed on cervical cancer patients and healthy persons, and a microRNA group with p < 0.05 is listed in Table 4-13. Student t-test was performed on colorectal cancer patients and healthy persons, and a microRNA group with p < 0.05 is listed in Table 4-14. Student t-test was performed on endometrial cancer patients and healthy persons, and a microRNA group with p < 0.05 is listed in Table 4-15.

Next, 5, 10, 15, or 20 miRNAs were randomly selected from the miRNA group listed in Table 4-1, and the expression levels of the selected miRNAs were used as indicators to predict whether the subject was a lung cancer patient or not by logistic regression. The prediction was performed 20 times. Tables 5-1 to 19-2 show the results. Tables 5-1, 6-1, 7-1, 8-1, 9-1, 10-1, 11-1, 12-1, 13-1, 14-1, 15-1, 16-1, 17-1, 18-1, and 19-1 list the accuracy (percentage of correct answers or accuracy), recall (sensitivity or reproducibility), precision, and F-values for each of the 20 predictions. The accuracy represents (TP + TN)/(TP + FP + TN + TP), the precision represents TP/(TP + FP), the recall represents TP/(TP + FN), TP denotes true positive, TN denotes true negative, FP denotes false positive, and FN denotes false negative. Each F-value is a harmonic mean of precision and recall.

Tables 5-2, 6-2, 7-2, 8-2, 9-2, 10-2, 11-2, 12-2, 13-2, 14-2, 15-2, 16-2, 17-2, 18-2, and 19-2 show the analysis results of the results shown in Tables 5-1, 6-1, 7-1, 8-1, 9-1, 10-1, 11-1, 12-1, 13-1, 14-1, 15-1, 16-1, 17-1, 18-1, and 19-1, respectively. As shown in Tables 5-2, 6-2, 7-2, 8-2, 9-2, 10-2, 11-2, 12-2, 13-2, 14-2, 15-2, and 16-2, the analyses with 5 or more miRNAs showed high accuracy, recall, and precision in all cases except in exceptional cases. Also, as shown in Tables 5-2, 6-2, 7-2, 8-2, 9-2, 10-2, 11-2, 12-2, 13-2, 14-2, 15-2, 16-2, 17-2, 18-2, and 19-2, the analyses with 10, 15, and 20 randomly selected miRNAs showed further improved accuracy, recall, and precision. Thus, various cancer patients could also be predicted by randomly selecting and combining 5 or more, preferably 10 or more, miRNAs listed in any of Tables 4-1 to 4-15.

Furthermore, combinations of n miRNAs selected from 9 randomly selected miRNAs (see Table 20) (that is, 9Cn combinations) were prepared, and the expression levels of the n miRNAs in all the combinations were used as indicators to predict a lung cancer patient by logistic regression. As a result, as shown in Table 21, the accuracy increased with n, though the prediction was possible even when n was 1.

miRNAs that could predict stage-I and -II patients are also individually identified. Among the miRNAs with p < 0.05 in the t-test for all the lung cancer patients and all the healthy persons, miRNAs with an accuracy of 50% or higher and the accuracy of the miRNAs were determined in the prediction of stage-I and -II patients using the expression level with a single miRNA as an indicator. Tables 22-1 to 22-13 show the prediction of various stage-I cancer patients. Table 23-1 shows the prediction of stage-II lung cancer patients. For other cancers, Table 23-2 to 23-14 show the predicted results for stage-I and -II cancer patients.

ROC curves of the expression level of a single miRNA were plotted by simple cut-off to determine true positive rates, false positive rates, cut-off values therefore, and AUC values. Tables 25-1 to 25-15 show the results. ROC curves of the expression level of a single miRNA were also plotted by logistic regression to determine true positive rates, false positive rates, cut-off values therefore, and AUC values. Tables 24-1 to 24-15 show the results. These results show that the miRNAs disclosed in these tables could be used alone for evaluation to predict cancer patients.

It was also shown that an increased number of miRNAs used for prediction resulted in at least one of improved prediction accuracy, sensitivity, and specificity.

### List of related references (listed below with reference numbers)

1. G. Raposo, W. Stoorvogel, Extracellular vesicles: Exosomes, microvesicles, and friends. J. Cell Biol. 200, 373-383(2013).
2. I. Evans-Osses, L. H. Reichembach, M. I. Ramirez, Exosomes or microvesicles? Two kinds of extracellular vesicles with different routes to modify protozoan-host cell interaction. Parasitol. Res.114, 3567-3575(2015).
3. P. Ma, Y. Pan, W. Li, C. Sun, J. Liu, T. Xu, Y. Shu, Extracellular vesicles-mediated noncoding RNAs transfer in cancer. J. Hematol. Oncol. 10, 57(2017).
4. R. Szatanek, J. Baran, M. Siedlar, M. Baj-Krzyworzeka, Isolation of extracellular vesicles:Determining the correct approach(Review). Int. J. Mol. Med. 36, 11-17(2015).
5. K. Jeppesen, M. L. Hvam, B. Primdahl-Bengtson, A. T. Boysen, B. Whitehead, L. Dyrskjot, T. F. Orntoft, K. A. Howard, M. S. Ostenfeld, Comparative analysis of discrete exosome fractions obtained by differential centrifugation. J. Extracell. Vesicles 3, 25011(2014).
6. A. Weber, D. H. Baxter, S. Zhang, D. Y. Huang, K. H. Huang, M. J. Lee, D. J. Galas, K. Wang, The microRNA spectrum in 12 body fluids. Clin. Chem. 56, 1733-1741(2010).
7. L. -L. Lv, Y. Cao, D. Liu, M. Xu, H. Liu, R. -N. Tang, K. -L. Ma, B. -C. Liu, Isolation and quantification of microRNAs from urinary exosomes/microvesicles for biomarker discovery. Int. J. Biol. Sci. 9, 1021-1031(2013).
8. M. L. Alvarez, M. Khosroheidari, R. K. Ravi, J. K. DiStefano, Comparison of protein, microRNA, and mRNA yields using different methods of urinary exosome isolation for the discovery of kidney disease biomarkers. Kidney Int. 82, 1024-1032(2012).
9. J. Zhang, S. Li, L. Li, M. Li, C. Guo, J. Yao, S. Mi, Exosome and exosomal microRNA: Trafficking, sorting, and function. Genomics Proteomics Bioinformatics 13, 17-24(2015).
10. N. Kosaka, H. Iguchi, T. Ochiya, Circulating microRNA in body fluid: A new potential biomarker for cancer diagnosis and prognosis. Cancer Sci. 101, 2087-2092(2010).
11. M. Iero, R. Valenti, V. Huber, P. Filipazzi, G. Parmiani, S. Fais, L. Rivoltini, Tumour-released exosomes and their implications in cancer immunity. Cell Death Differ. 15, 80-88(2008).
12. D. D. Taylor, C. Gercel-Taylor, MicroRNA signatures of tumor-derived exosomes as diagnostic biomarkers of ovarian cancer. Gynecol. Oncol. 110, 13-21(2008).
13. K. Al-Nedawi, B. Meehan, J. Micallef, V. Lhotak, L. May, A. Guha, J. Rak, Intercellular transfer of the oncogenic receptor EGFRvIII by microvesicles derived from tumour cells. Nat. Cell Biol. 10, 619-624(2008).
14. Y. Yoshioka, N. Kosaka, Y. Konishi, H. Ohta, H. Okamoto, H. Sonoda, R. Nonaka, H. Yamamoto, H. Ishii, M. Mori, K. Furuta, T. Nakajima, H. Hayashi, H. Sugisaki, H. Higashimoto, T. Kato, F. Takeshita, T. Ochiya, Ultra-sensitive liquid biopsy of circulating extracellular vesicles using ExoScreen. Nat. Commun. 5, 3591(2014).
15. H. Peinado, M. S. Lavotshkin, I. Matei, B. Costa-Silva, G. Moreno-Bueno, M. Hergueta-Redondo, C. Williams, G. Garcia-Santos, C. M. Ghajar, A. Nitadori-Hoshino, C. Hoffman, K. Badal, B. A. Garcia, M. K. Callahan, J. Yuan, V. R. Martins, J. Skog, R. N. Kaplan, M. S. Brady, J. D. Wolchok, P. B. Chapman, Y. Kang, J. Bromberg, D. Lyden, Melanoma exosomes educate bone marrow progenitor cells toward a pro-metastatic phenotype through MET. Nat. Med. 18, 883-891(2012).
16. C. Y. Chen, M. C. Hogan, C. J. Ward, Purification of exosome-like vesicles from urine. Methods Enzymol. 524, 225-241(2013).
17. J. Webber, R. Steadman, M. D. Mason, Z. Tabi, A. Clayton, Cancer exosomes trigger fibroblast to myofibroblast differentiation. Cancer Res. 70, 9621-9630(2010).
18. J. Skog, T. Wurdinger, S. van Rijn, D. H. Meijer, L. Gainche, W. T. Curry Jr. , B. S. Carter, A. M. Krichevsky, X. O. Breakefield, Glioblastoma microvesicles transport RNA and proteins that promote tumour growth and provide diagnostic biomarkers. Nat. Cell Biol. 10, 1470-1476(2008).
19. A. V. Vlassov, S. Magdaleno, R. Setterquist, R. Conrad, Exosomes:Current knowledge of their composition, biological functions, and diagnostic and therapeutic potentials. Biochim. Biophys. Acta 1820, 940-948(2012).
20. C. H. Arnaud, Seeking tiny vesicles for diagnostics. Chem. Eng. News 93, 30-32(2015).
21. M. B. Kirschner, J. J. B. Edelman, S. C. -H. Kao, M. P. Vallely, N. van Zandwijk, G. Reid, The impact of hemolysis on cell-free microRNA biomarkers. Front. Genet. 4, 94(2013).
22. D. D. Taylor, W. Zacharias, C. Gercel-Taylor, Exosome isolation for proteomic analyses and RNA profiling. Methods Mol. Biol. 728, 235-246(2011).
23. S. Jeong, J. Park, D. Pathania, C. M. Castro, R. Weissleder, H. Lee, Integrated magneto-electrochemical sensor for exosome analysis. ACS Nano 10, 1802-1809(2016).
24. V. Sunkara, H. -K. Woo, Y. -K. Cho, Emerging techniques in the isolation and characterization of extracellular vesicles and their roles in cancer diagnostics and prognostics. Analyst 141, 371-381(2016).
25. P. Zhang, M. He, Y. Zeng, Ultrasensitive microfluidic analysis of circulating exosomes using a nanostructured graphene oxide/polydopamine coating. Lab Chip 16, 3033-3042(2016).
26. B. H. Wunsch, J. T. Smith, S. M. Gifford, C. Wang, M. Brink, R. L. Bruce, R. H. Austin, G. Stolovitzky, Y. Astier, Nanoscale lateral displacement arrays for the separation of exosomes and colloids down to 20nm. Nat. Nanotechnol. 11, 936-940(2016).
27. H. -K. Woo, V. Sunkara, J. Park, T. -H. Kim, J. -R. Han, C. -J. Kim, H. -I. Choi, Y. -K. Kim, Y. -K. Cho, Exodisc for rapid, size-selective, and efficient isolation and analysis of nanoscale extracellular vesicles from biological samples. ACS Nano 11, 1360-1370(2017).
28. F. Barutta, M. Tricarico, A. Corbelli, L. Annaratone, S. Pinach, S. Grimaldi, G. Bruno, D. Cimino, D. Taverna, M. C. Deregibus, M. P. Rastaldi, P. C. Perin, G. Gruden, Urinary exosomal microRNAs in incipient diabetic nephropathy. PLOS ONE 8, e73798(2013).
29. C. Thery, S. Amigorena, G. Raposo, A. Clayton, Isolation and characterization of exosomes from cell culture supernatants and biological fluids. Curr. Protoc. Cell Biol. Chapter 3, Unit 3. 22(2006).
30. K. E. Petersen, E. Manangon, J. L. Hood, S. A. Wickline, D. P. Fernandez, W. P. Johnson, B. K. Gale, A review of exosome separation techniques and characterization of B16-F10 mouse melanoma exosomes with AF4-UV-MALS-DLS-TEM. Anal. Bioanal. Chem. 406, 7855-7866(2014).
31. L. Cheng, X. Sun, B. J. Scicluna, B. M. Coleman, A. F. Hill, Characterization and deep sequencing analysis of exosomal and non-exosomal miRNA in human urine. Kidney Int. 86, 433-444(2014).
32. X. Duan, R. Gao, P. Xie, T. Cohen-Karni, Q. Qing, H. S. Choe, B. Tian, X. Jiang, C. M. Lieber, Intracellular recordings of action potentials by an extracellular nanoscale field-effect transistor. Nat. Nanotechnol. 7, 174-179(2012).
33. R. Yan, J. -H. Park, Y. Choi, C. -J. Heo, S. -M. Yang, L. P. Lee, P. Yang, Nanowire-based single-cell endoscopy. Nat. Nanotechnol. 7, 191-196(2012).
34. T. Yasui, S. Rahong, K. Motoyama, T. Yanagida, Q. Wu, N. Kaji, M. Kanai, K. Doi, K. Nagashima, M. Tokeshi, M. Taniguchi, S. Kawano, T. Kawai, Y. Baba, DNA manipulation and separation in sublithographic-scale nanowire array. ACS Nano 7, 3029-3035(2013).
35. H. So, K. Lee, N. Murthy, A. P. Pisano, All-in-one nanowire-decorated multifunctional membrane for rapid cell lysis and direct DNA isolation. ACS Appl. Mater. Interfaces 6, 20693-20699(2014).
36. J. Liu, T. -M. Fu, Z. Cheng, G. Hong, T. Zhou, L. Jin, M. Duvvuri, Z. Jiang, P. Kruskal, C. Xie, Z. Suo, Y. Fang, C. M. Lieber, Syringe-injectable electronics. Nat. Nanotechnol. 10, 629-636(2015).
37. Q. Shen, L. Xu, L. Zhao, D. Wu, Y. Fan, Y. Zhou, W. -H. OuYang, X. Xu, Z. Zhang, M. Song, T. Lee, M. A. Garcia, B. Xiong, S. Hou, H. -R. Tseng, X. Fang, Specific capture and release of circulating tumor cells using aptamer-modified nanosubstrates. Adv. Mater. 25, 2368-2373(2013).
38. J. Kim, J. W. Hong, D. P. Kim, J. H. Shin, I. Park, Nanowire-integrated microfluidic devices for facile and reagent-free mechanical cell lysis. Lab Chip 12, 2914-2921(2012).
39. S. Rahong, T. Yasui, N. Kaji, Y. Baba, Recent developments in nanowires for bio-applications from molecular to cellular levels. Lab Chip 16, 1126-1138(2016).
40. S. Zhang, Y. Shen, H. Fang, S. Xu, J. Song, Z. L. Wang, Growth and replication of ordered ZnO nanowire arrays on general flexible substrates. J. Mater. Chem. 20, 10606-10610(2010).
41. A. D. Stroock, S. K. W. Dertinger, A. Ajdari, I. H. A. Stone, G. M. Whitesides, Chaotic mixer for microchannels. Science 295, 647-651(2002).
42. D. Xiao, J. Ohlendorf, Y. Chen, D. D. Taylor, S. N. Rai, S. Waigel, W. Zacharias, H. Hao, K. M. McMasters, Identifying mRNA, microRNA and protein profiles of melanoma exosomes. PLOS ONE 7, e46874(2012).
43. D. -S. Choi, D. -Y. Choi, B. S. Hong, S. C. Jang, D. -K. Kim, J. Lee, Y. -K. Kim, K. P. Kim, Y. S. Gho, Quantitative proteomics of extracellular vesicles derived from human primary and metastatic colorectal cancer cells. J. Extracell. Vesicles 1, 18704(2012).
44. J. R. Edgar, Q&A:What are exosomes, exactly? BMC Biol. 14, 46(2016).
45. J. W. Elam, S. M. George, Growth of ZnO/A12O3 alloy films using atomic layer deposition techniques. Chem. Mater. 15, 1020-1028(2003).
46. B. Liu, R. Hu, J. Deng, Studies on a potentiometric urea biosensor based on an ammonia electrode and urease: Immobilized on a g-aluminum oxide matrix. Anal. Chim. Acta 341, 161-169(1997).
47. S. Xu, Z. L. Wang, One-dimensional ZnO nanostructures: Solution growth and functional properties. Nano Res. 4, 1013-1098(2011).
48. J. Zang, C. M. Li, X. Cui, J. Wang, X. Sun, H. Dong, C. Q. Sun, Tailoring zinc oxide nanowires for high performance amperometric glucose sensor. Electroanalysis 19, 1008-1014(2007).
49. M. C. Deregibus, F. Figliolini, S. D'Antico, P. M. Manzini, C. Pasquino, M. De Lena, C. Tetta, M. F. Brizzi, G. Camussi, Charge-based precipitation of extracellular vesicles. Int. J. Mol. Med. 38, 1359-1366(2016).
50. T. S. Schlappi, S. E. McCalla, N. G. Schoepp, R. F. Ismagilov, Flow-through capture and in situ amplification can enable rapid detection of a few single molecules of nucleic acids from several milliliters of solution. Anal. Chem. 88, 7647-7653(2016).
51. J. A. Hanley, B. J. McNeil, The meaning and use of the area under a receiver operating characteristic(ROC)curve. Radiology 143, 29-36(1982).
52. Z. Wang, B. Ma, X. Ji, Y. Deng, T. Zhang, X. Zhang, H. Gao, H. Sun, H. Wu, X. Chen, R. Zhao, MicroRNA-378-5p suppresses cell proliferation and induces apoptosis in colorectal cancer cells by targeting BRAF. Cancer Cell Int. 15, 40(2015).
53. H. -L. Miao, C. -J. Lei, Z. -D. Qiu, Z. -K. Liu, R. Li, S. -T. Bao, M. -Y. Li, MicroRNA-520c-3p inhibits hepatocellular carcinoma cell proliferation and invasion through induction of cell apoptosis by targeting glypican-3. Hepatol. Res. 44, 338-348(2014).
54. S. Lu, Q. Zhu, Y. Zhang, W. Song, M. J. Wilson, P. Liu, Dual-functions of miR-373 and miR-520c by differently regulating the activities of MMP2 and MMP9. J. Cell. Physiol. 230, 1862-1870(2015).
55. K. Mazan-Mamczarz, X. F. Zhao, B. Dai, J. J. Steinhardt, R. J. Peroutka, K. L. Berk, A. L. Landon, M. Sadowska, Y. Zhang, E. Lehrmann, K. G. Becker, R. Shaknovich, Z. Liu, R. B. Gartenhaus, Down-regulation of eIF4GII by miR-520c-3p represses diffuse large B cell lymphoma development. PLOS Genet. 10, e1004105(2014).
56. C. -J. Lei, C. Yao, D. -K. Li, Z. -X. Long, Y. Li, D. Tao, Y. -P. Liou, J. -Z. Zhang, N. Liu, Effect of co-transfection of miR-520c-3p and miR-132 on proliferation and apoptosis of hepatocellular carcinoma Huh7. Asian Pac. J. Trop. Med. 9, 898-902(2016).
57. G. Mudduluru, K. Ilm, S. Fuchs, U. Stein, Epigenetic silencing of miR-520c leads to induced S100A4 expression and its mediated colorectal cancer progression. Oncotarget 8, 21081-21094(2017).
58. R. Zhang, J. Zhao, J. Xu, J. Wang, J. Jia, miR-526b-3p functions as a tumor suppressor in colon cancer by regulating HIF-1a. Am. J. Transl. Res. 8, 2783-2789(2016).
59. L. Su, D. Han, J. Wu, X. Huo, Skp2 regulates non-small cell lung cancer cell growth by Meg3 and miR-3163. Tumour Biol. 37, 3925-3931(2016).
60. T. Wang, J. Hou, Z. Li, Z. Zheng, J. Wei, D. Song, T. Hu, Q. Wu, J. Y. Yang, J. -c. Cai, miR-15a-3p and miR-16-1-3p negatively regulate Twist1 to repress gastric cancer cell invasion and metastasis. Int. J. Biol. Sci. 13, 122-134(2017).
61. Q. -y. Chen, D. -m. Jiao, L. Yan, Y. -q. Wu, H. -z. Hu, J. Song, J. Yan, L. -j. Wu, L. -q. Xu, J. -g. Shi, Comprehensive gene and microRNA expression profiling reveals miR-206 inhibits MET in lung cancer metastasis. Mol. Biosyst. 11, 2290-2302(2015).
62. L. Zheng, W. Jiao, H. Song, H. Qu, D. Li, H. Mei, Y. Chen, F. Yang, H. Li, K. Huang, Q. Tong, miRNA-558 promotes gastric cancer progression through attenuating Smad4-mediated repression of heparanase expression. Cell Death Dis. 7, e2382(2016).
63. Y. Sun, C. Chen, P. Zhang, H. Xie, L. Hou, Z. Hui, Y. Xu, Q. Du, X. Zhou, B. Su, W. Gao, Reduced miR-3127-5p expression promotes NSCLC proliferation/invasion and contributes to dasatinib sensitivity via the c-Abl/Ras/ERK pathway. Sci. Rep. 4, 6527(2014).
64. Y. Fang, J. Xiang, Z. Chen, X. Gu, Z. Li, F. Tang, Z. Zhou, miRNA expression profile of colon cancer stem cells compared to non-stem cells using the SW1116 cell line. Oncol. Rep. 28, 2115-2124(2012).
65. X. Huang, M. Huang, L. Kong, Y. Li, miR-372 suppresses tumour proliferation and invasion by targeting IGF2BP1 in renal cell carcinoma. Cell Prolif. 48, 593-599(2015).
66. Y. -C. Lu, A. -J. Cheng, L. -Y. Lee, G. -R. You, Y. -L. Li, H. -Y. Chen, J. T. Chang, MiR-520b as a novel molecular target for suppressing stemness phenotype of head-neck cancer by inhibiting CD44. Sci. Rep. 7, 2042(2017).
67. A. Druz, Y. -C. Chen, R. Guha, M. Betenbaugh, S. E. Martin, J. Shiloach, Large-scale screening identifies a novel microRNA, miR-15a-3p, which induces apoptosis in human cancer cell lines. RNA Biol. 10, 287-300(2013).
68. Z. Wu, Y. Wu, Y. Tian, X. Sun, J. Liu, H. Ren, C. Liang, L. Song, H. Hu, L. Wang, B. Jiao, Differential effects of miR-34c-3p and miR-34c-5p on the proliferation, apoptosis and invasion of glioma cells. Oncol. Lett. 6, 1447-1452(2013).
69. Z. Cheng, F. Liu, H. Zhang, X. Li, Y. Li, J. Li, F. Liu, Y. Cao, L. Cao, F. Li, miR-135a inhibits tumor metastasis and angiogenesis by targeting FAK pathway. Oncotarget 8, 31153-31168(2017).
70. C. Liu, G. Li, S. Ren, Z. Su, Y. Wang, Y. Tian, Y. Liu, Y. Qiu, miR-185-3p regulates the invasion and metastasis of nasopharyngeal carcinoma by targeting WNT2B in vitro. Oncol. Lett. 13, 2631-2636(2017).
71. R. Zhang, H. Leng, J. Huang, Y. Du, Y. Wang, W. Zang, X. Chen, G. Zhao, miR-337 regulates the proliferation and invasion in pancreatic ductal adenocarcinoma by targeting HOXB7. Diagn. Pathol. 9, 171(2014).
72. M. Lu, X. Zhou, C. -G. Zheng, F. -J. Liu, Expression profiling of miR-96, miR-584 and miR-422a in colon cancer and their potential involvement in colon cancer pathogenesis. Trop. J. Pharm. Res. 15, 2535-2542(2016).
73. Z. Zhao, X. Ma, T. -H. Hsiao, G. Lin, A. Kosti, X. Yu, U. Suresh, Y. Chen, G. E. Tomlinson, A. Pertsemlidis, L. Du, A high-content morphological screen identifies novel microRNAs that regulate neuroblastoma cell differentiation. Oncotarget 5, 2499-2512(2014).
74. W. Chen, Z. Tang, Y. Sun, Y. Zhang, X. Wang, Z. Shen, F. Liu, X. Qin, miRNA expression profile in primary gastric cancers and paired lymph node metastases indicates that miR-10a plays a role in metastasis from primary gastric cancer to lymph nodes. Exp. Ther. Med. 3, 351-356(2012).
75. Y. Li, W. Han, T. -T. Ni, L. Lu, M. Huang, Y. Zhang, H. Cao, H. -Q. Zhang, W. Luo, H. Li, Knockdown of microRNA-1323 restores sensitivity to radiation by suppression of PRKDC activity in radiation-resistant lung cancer cells. Oncol. Rep. 33, 2821-2828(2015).
76. A. R. Lee, J. Park, K. J. Jung, S. H. Jee, S. J. Kim-Yoon, Genetic variation rs7930 in the miR-4273-5p target site is associated with a risk of colorectal cancer. Oncotargets Ther. 9, 6885-6895(2016).
77. F. Miao, J. Zhu, Y. Chen, N. Tang, X. Wang, X. Li, MicroRNA-183-5p promotes the proliferation, invasion and metastasis of human pancreatic adenocarcinoma cells. Oncol. Lett. 11, 134-140(2016).
78. H. R. Mody, S. W. Hung, R. K. Pathak, J. Griffin, Z. Cruz-Monserrate, R. Govindarajan, miR-202 diminishes TGFb receptors and attenuates TGFb1-induced EMT in pancreatic cancer. Mol. Cancer Res. 15, 1029-1039(2017).
79. S. Josson, M. Gururajan, P. Hu, C. Shao, G. C. -Y. Chu, H. E. Zhau, C. Liu, K. Lao, C. -L. Lu, Y. -T. Lu, J. Lichterman, S. Nandana, Q. Li, A. Rogatko, D. Berel, E. M. Posadas, L. Fazli, D. Sareen, L. W. K. Chung, miR-409-3p/-5p promotes tumorigenesis, epithelial-to-mesenchymal transition, and bone metastasis of human prostate cancer. Clin. Cancer Res. 20, 4636-4646(2014).
80. X. Shi, F. Teng, Down-regulated miR-28-5p in human hepatocellular carcinoma correlated with tumor proliferation and migration by targeting insulin-like growth factor-1(IGF-1). Mol. Cell. Biochem. 408, 283-293(2015).
81. Y. Sun, J. Zhao, X. Yin, X. Yuan, J. Guo, J. Bi, miR-297 acts as an oncogene by targeting GPC5 in lung adenocarcinoma. Cell Prolif. 49, 636-643(2016).
82. H. -q. Liang, R. -j. Wang, C. -f. Diao, J. -w. Li, J. -1. Su, S. Zhang, The PTTG1-targeting miRNAs miR-329, miR-300, miR-381, and miR-655 inhibit pituitary tumor cell tumorigenesis and are involved in a p53/PTTG1 regulation feedback loop. Oncotarget 6, 29413-29427(2015).
83. A. Mesci, X. Huang, S. Taeb, S. Jahangiri, Y. Kim, E. Fokas, J. Bruce, H. S. Leong, S. K. Liu, Targeting of CCBE1 by miR-330-3p in human breast cancer promotes metastasis. Br. J. Cancer 116, 1350-1357(2017).
84. L. Yu, X. Gong, L. Sun, H. Yao, B. Lu, L. Zhu, miR-454 functions as an oncogene by inhibiting CHD5 in hepatocellular carcinoma. Oncotarget 6, 39225-39234(2015).
85. C. Liu, C. Wang, J. Wang, H. Huang, miR-1297 promotes cell proliferation by inhibiting RB1 in liver cancer. Oncol. Lett. 12, 5177-5182(2016).
86. M. Shao, Y. Geng, P. Lu, Y. Xi, S. Wei, L. Wang, Q. Fan, W. Ma, miR-4295 promotes cell proliferation and invasion in anaplastic thyroid carcinoma via CDKN1A. Biochem. Biophys. Res. Commun. 464, 1309-1313(2015).
87. Y. An, Z. Zhang, Y. Shang, X. Jiang, J. Dong, P. Yu, Y. Nie, Q. Zhao, miR-23b-3p regulates the chemoresistance of gastric cancer cells by targeting ATG12 and HMGB2. Cell Death Dis. 6, e1766(2015).
88. C. T. D. Dickman, J. Lawson, J. Jabalee, S. A. MacLellan, N. E. LePard, K. L. Bennewith, C. Garnis, Selective extracellular vesicle exclusion of miR-142-3p by oral cancer cells promotes both internal and extracellular malignant phenotypes. Oncotarget 8, 15252-15266(2017).
89. L. Jin, X. Li, Y. Li, Z. Zhang, T. He, J. Hu, J. Liu, M. Chen, M. Shi, Z. Jiang, Y. Gui, S. Yang, X. Mao, Y. Lai, Identification of miR-195-3p as an oncogene in ROC. Mol. Med. Rep. 15, 1916-1924(2017).
90. A. R. Gohring, S. Reuter, J. H. Clement, X. Cheng, J. Theobald, S. Wolfl, R. Mrowka, Human microRNA-299-3p decreases invasive behavior of cancer cells by downregulation of Oct4 expression and causes apoptosis. PLOS ONE 12, e0174912(2017).
91. Y. -H. Nan, J. Wang, Y. Wang, P. -H. Sun, Y. -P. Han, L. Fan, K. -C. Wang, F. -J. Shen, W. -H. Wang, MiR-4295 promotes cell growth in bladder cancer by targeting BTG1. Am. J. Transl. Res. 9, 1960(2017).
92. N. Nabavi, N. R. N. Saidy, E. Venalainen, A. Haegert, A. Parolia, H. Xue, Y. Wang, R. Wu, X. Dong, C. Collins, F. Crea, Y. Wang, miR-100-5p inhibition induces apoptosis in dormant prostate cancer cells and prevents the emergence of castration-resistant prostate cancer. Sci. Rep. 7, 4079(2017).
93. D. Deng, L. Wang, Y. Chen, B. Li, L. Xue, N. Shao, Q. Wang, X. Xia, Y. Yang, F. Zhi, MicroRNA-124-3p regulates cell proliferation, invasion, apoptosis, and bioenergetics by targeting PIM1 in astrocytoma. Cancer Sci. 107, 899-907(2016).
94. T. Chiyomaru, S. Yamamura, M. S. Zaman, S. Majid, G. Deng, V. Shahryari, S. Saini, H. Hirata, K. Ueno, I. Chang, Y. Tanaka, Z. L. Tabatabai, H. Enokida, M. Nakagawa, R. Dahiya, Genistein suppresses prostate cancer growth through inhibition of oncogenic microRNA-151. PLOS ONE 7, e43812(2012).
95. Z. Xue, J. Zhao, L. Niu, G. An, Y. Guo, L. Ni, Up-regulation of MiR-300 promotes proliferation and invasion of osteosarcoma by targeting BRD7. PLOS ONE 10, e0127682(2015).
96. D. A. Armstrong, B. B. Green, J. D. Seigne, A. R. Schned, C. J. Marsit, MicroRNA molecular profiling from matched tumor and bio-fluids in bladder cancer. Mol. Cancer 14, 194(2015).

Table 3-1
Table 3-2
Table 4-1~15
Table 5-1~2
Table 6-1~2
Table 7-1~2
Table 8-1~2
Table 9-1~2
Table 10-1~2
Table 11-1~2
Table 12-1~2
Table 13-1~2
Table 14-1~2
Table 15-1~2
Table 16-1~2
Table 17-1~2
Table 18-1~2
Table 19-1~2
Table 20
Table 21
Table 22-1~13
Table 23-1~14
Table 24-1~15
Table 25-1~15

### [Table 3-1]

**Table 3-1: Data S1 (noncancer, lung cancer, pancreatic cancer)**

| miRNA name | Noncancer | | | Lung cancer | | | Pancreatic cancer | | |
|---|---|---|---|---|---|---|---|---|---|
| hsa-let-7a-5p | 0.0 | 0.0 | 0.0 | 0.0 | 0.0 | 0.0 | 0.0 | 2.4 | 0.0 |
| hsa-let-7c-5p | 0.0 | 2.0 | 0.0 | 0.0 | 0.0 | 0.0 | 0.0 | 0.0 | 0.0 |
| hsa-let-7d-5p | 0.0 | 0.0 | 0.0 | 0.0 | 0.0 | 0.0 | 0.0 | 0.0 | 0.0 |
| hsa-let-7e-3p | 0.0 | 0.0 | 2.5 | 0.0 | 0.0 | 0.0 | 0.0 | 0.0 | 0.0 |
| hsa-let-7f-2-3p | 0.0 | 3.5 | 3.8 | 2.5 | 0.0 | 1.8 | 0.0 | 0.0 | 0.0 |
| hsa-let-7f-5p | 0.0 | 0.0 | 0.0 | 0.0 | 0.0 | 0.0 | 0.0 | 0.0 | 0.0 |
| hsa-let-7g-3p | 0.0 | 0.0 | 2.1 | 2.4 | 0.0 | 0.0 | 4.0 | 0.0 | 0.0 |
| hsa-let-7i-3p | 0.0 | 1.9 | 0.0 | 2.8 | 0.0 | 0.0 | 3.3 | 0.0 | 3.0 |
| hsa-miR-0001-3p | 0.0 | 0.0 | 0.0 | 6.2 | 0.0 | 0.0 | 0.0 | 0.0 | 0.0 |
| hsa-miR-0001-5p | 2.6 | 3.4 | 2.1 | 0.0 | 0.0 | 2.2 | 0.0 | 0.0 | 0.0 |
| hsa-miR-0009-5p | 0.0 | 1.9 | 0.0 | 0.0 | 0.0 | 0.0 | 0.0 | 0.0 | 0.0 |
| hsa-miR-0015a-3p | 2.6 | 4.0 | 3.6 | 2.7 | 0.0 | 2.3 | 0.0 | 0.0 | 0.0 |
| hsa-miR-0015a-5p | 0.0 | 0.0 | 0.0 | 0.0 | 0.0 | 2.0 | 3.7 | 0.0 | 0.0 |
| hsa-miR-0015b-3p | 0.0 | 0.0 | 0.0 | 0.0 | 0.0 | 0.0 | 0.0 | 0.0 | 0.0 |
| hsa-miR-0016-2-3p | 2.4 | 1.9 | 0.0 | 0.0 | 2.1 | 2.2 | 0.0 | 0.0 | 0.0 |
| hsa-miR-0016-5p | 0.0 | 0.0 | 0.0 | 0.0 | 0.0 | 0.0 | 3.4 | 0.0 | 0.0 |
| hsa-miR-0017-5p | 0.0 | 0.0 | 0.0 | 0.0 | 0.0 | 0.0 | 0.0 | 0.0 | 0.0 |
| hsa-miR-0018b-5p | 0.0 | 0.0 | 3.4 | 3.9 | 1.6 | 0.0 | 0.0 | 0.0 | 0.0 |
| hsa-miR-0019a-3p | 0.0 | 0.0 | 0.0 | 0.0 | 0.0 | 0.0 | 0.0 | 0.0 | 2.9 |
| hsa-miR-0019a-5p | 0.0 | 0.0 | 0.0 | 0.0 | 0.0 | 0.0 | 0.0 | 0.0 | 0.0 |
| hsa-miR-0019b-1-5p | 0.0 | 0.0 | 0.0 | 2.4 | 0.0 | 0.0 | 0.0 | 0.0 | 0.0 |
| hsa-miR-0019b-2-5p | 0.0 | 0.0 | 0.0 | 0.0 | 0.0 | 0.0 | 3.1 | 0.0 | 0.0 |
| hsa-miR-0020b-5p | 0.0 | 2.0 | 0.0 | 0.0 | 1.8 | 0.0 | 0.0 | 3.1 | 0.0 |
| hsa-miR-0021-5p | 0.0 | 0.0 | 0.0 | 0.0 | 0.0 | 0.0 | 0.0 | 0.0 | 0.0 |
| hsa-miR-0022-5p | 0.0 | 0.0 | 0.0 | 0.0 | 0.0 | 0.0 | 3.0 | 0.0 | 0.0 |
| hsa-miR-0023b-5p | 0.0 | 0.0 | 0.0 | 0.0 | 0.0 | 0.0 | 0.0 | 0.0 | 0.0 |
| hsa -miR -0023c | 0.0 | 0.0 | 0.0 | 0.0 | 0.0 | 0.0 | 0.0 | 0.0 | 0.0 |
| hsa-miR-0024-1-5p | 0.0 | 0.0 | 2.6 | 0.0 | 0.0 | 0.0 | 2.7 | 3.1 | 0.0 |
| hsa-miR-0024-2-5p | 0.0 | 0.0 | 0.0 | 0.0 | 2.3 | 2.0 | 0.0 | 0.0 | 0.0 |
| hsa-miR-0024-3p | 2.1 | 2.6 | 2.6 | 0.0 | 2.9 | 2.9 | 0.0 | 2.8 | 0.0 |
| hsa-miR-0026a-2-3p | 0.0 | 0.0 | 0.0 | 0.0 | 0.0 | 0.0 | 3.4 | 0.0 | 0.0 |
| hsa-miR-0026a-5p | 0.0 | 0.0 | 2.5 | 2.5 | 0.0 | 0.0 | 3.1 | 0.0 | 0.0 |
| hsa-miR-0027a-5p | 0.0 | 0.0 | 3.4 | 0.0 | 0.0 | 0.0 | 0.0 | 0.0 | 0.0 |
| hsa-miR-0028-3p | 0.0 | 2.1 | 3.9 | 0.0 | 2.2 | 0.0 | 0.0 | 0.0 | 3.7 |
| hsa-miR-0029a-5p | 0.0 | 2.2 | 3.7 | 5.1 | 0.0 | 0.0 | 2.6 | 0.0 | 0.0 |
| hsa-miR-0029b-1-5p | 0.0 | 0.0 | 0.0 | 0.0 | 1.7 | 0.0 | 2.6 | 0.0 | 0.0 |
| hsa-miR-0029c-3p | 0.0 | 0.0 | 2.2 | 0.0 | 0.0 | 0.0 | 0.0 | 0.0 | 0.0 |
| hsa-miR-0030a-5p | 0.0 | 0.0 | 0.0 | 0.0 | 0.0 | 0.0 | 0.0 | 0.0 | 0.0 |
| hsa-miR-0031-3p | 0.0 | 0.0 | 3.3 | 5.5 | 0.0 | 0.0 | 4.0 | 0.0 | 0.0 |
| hsa-miR-0031-5p | 0.0 | 0.0 | 0.0 | 0.0 | 0.0 | 0.0 | 3.8 | 0.0 | 0.0 |
| hsa-miR-0032-3p | 1.7 | 2.6 | 3.2 | 0.0 | 1.9 | 2.3 | 0.0 | 2.9 | 0.0 |
| hsa-miR-0032-5p | 0.0 | 2.6 | 0.0 | 0.0 | 2.5 | 0.0 | 0.0 | 0.0 | 0.0 |
| hsa-miR-0033a-3p | 0.0 | 0.0 | 0.0 | 0.0 | 0.0 | 1.5 | 0.0 | 0.0 | 0.0 |
| hsa-miR-0033a-5p | 0.0 | 0.0 | 0.0 | 0.0 | 0.0 | 0.0 | 0.0 | 0.0 | 3.6 |
| hsa-miR-0033b-5p | 0.0 | 0.0 | 0.0 | 0.0 | 0.0 | 0.0 | 0.0 | 0.0 | 0.0 |
| hsa-miR-0034a-3p | 0.0 | 0.0 | 0.0 | 2.4 | 0.0 | 0.0 | 0.0 | 0.0 | 0.0 |
| hsa-miR-0095-5p | 0.0 | 0.0 | 4.1 | 3.8 | 0.0 | 0.0 | 0.0 | 0.0 | 0.0 |
| hsa-miR-0096-5p | 0.0 | 0.0 | 0.0 | 0.0 | 0.0 | 0.0 | 0.0 | 0.0 | 3.0 |
| hsa-miR-0099a-5p | 0.0 | 0.0 | 0.0 | 0.0 | 0.0 | 0.0 | 3.3 | 2.7 | 0.0 |
| hsa-miR-0100-3p | 0.0 | 0.0 | 0.0 | 2.5 | 0.0 | 0.0 | 0.0 | 0.0 | 0.0 |
| hsa-miR-0100-5p | 0.0 | 2.1 | 0.0 | 2.8 | 0.0 | 0.0 | 0.0 | 2.7 | 0.0 |
| hsa-miR-0101-3p | 0.0 | 0.0 | 0.0 | 2.4 | 0.0 | 0.0 | 0.0 | 0.0 | 0.0 |
| hsa-miR-0101-5p | 0.0 | 0.0 | 0.0 | 0.0 | 0.0 | 0.0 | 0.0 | 0.0 | 0.0 |
| hsa-miR-0103a-3p | 0.0 | 2.4 | 0.0 | 0.0 | 0.0 | 2.4 | 0.0 | 0.0 | 2.5 |
| hsa-miR-0103b | 0.0 | 0.0 | 0.0 | 0.0 | 0.0 | 0.0 | 3.5 | 0.0 | 0.0 |
| hsa-miR-0105-3p | 0.0 | 0.0 | 0.0 | 2.5 | 0.0 | 0.0 | 0.0 | 0.0 | 0.0 |
| hsa-miR-0106a-3p | 0.0 | 0.0 | 0.0 | 0.0 | 0.0 | 0.0 | 0.0 | 0.0 | 2.6 |
| hsa-miR-0106a-5p | 0.0 | 0.0 | 0.0 | 0.0 | 1.4 | 0.0 | 0.0 | 0.0 | 0.0 |
| hsa-miR-0106b-5p | 0.0 | 0.0 | 0.0 | 6.3 | 1.7 | 0.0 | 0.0 | 0.0 | 0.0 |
| hsa-miR-0107 | 0.0 | 2.0 | 0.0 | 0.0 | 0.0 | 0.0 | 0.0 | 0.0 | 0.0 |
| hsa-miR-0122-3p | 0.0 | 2.4 | 1.9 | 0.0 | 2.5 | 1.4 | 0.0 | 0.0 | 0.0 |
| hsa-miR-0124-3p | 0.0 | 0.0 | 0.0 | 0.0 | 0.0 | 0.0 | 3.3 | 0.0 | 0.0 |
| hsa-miR-0125b-1-3p | 0.0 | 2.1 | 4.4 | 3.1 | 0.0 | 2.0 | 2.7 | 0.0 | 0.0 |
| hsa-miR-0126-5p | 0.0 | 0.0 | 1.7 | 0.0 | 0.0 | 0.0 | 2.6 | 0.0 | 0.0 |
| hsa-miR-0127-3p | 0.0 | 0.0 | 1.6 | 0.0 | 0.0 | 0.0 | 0.0 | 0.0 | 0.0 |
| hsa-miR-0130a-3p | 0.0 | 0.0 | 0.0 | 0.0 | 1.3 | 0.0 | 0.0 | 0.0 | 0.0 |
| hsa-miR-0130b-3p | 0.0 | 0.0 | 0.0 | 0.0 | 2.8 | 0.0 | 0.0 | 0.0 | 3.1 |
| hsa-miR-0132-3p | 0.0 | 0.0 | 0.0 | 0.0 | 2.0 | 1.6 | 0.0 | 0.0 | 0.0 |
| hsa-miR-0132-5p | 0.0 | 2.2 | 0.0 | 0.0 | 0.0 | 0.0 | 0.0 | 0.0 | 0.0 |
| hsa-miR-0133a-5p | 0.0 | 0.0 | 0.0 | 0.0 | 1.7 | 0.0 | 2.5 | 0.0 | 3.1 |
| hsa-miR-0135b-3p | 0.0 | 0.0 | 0.0 | 2.5 | 0.0 | 0.0 | 0.0 | 0.0 | 0.0 |
| hsa-miR-0136-5p | 0.0 | 0.0 | 0.0 | 6.0 | 0.0 | 0.0 | 0.0 | 0.0 | 0.0 |
| hsa-miR-0139-5p | 0.0 | 0.0 | 0.0 | 5.0 | 0.0 | 0.0 | 0.0 | 0.0 | 0.0 |
| hsa-miR-0140-3p | 0.0 | 0.0 | 0.0 | 0.0 | 0.0 | 0.0 | 0.0 | 0.0 | 0.0 |
| hsa-miR-0140-5p | 0.0 | 0.0 | 0.0 | 6.3 | 0.0 | 0.0 | 0.0 | 0.0 | 0.0 |
| hsa-miR-0141-3p | 0.0 | 0.0 | 0.0 | 0.0 | 0.0 | 0.0 | 0.0 | 0.0 | 0.0 |
| hsa-miR-0142-5p | 0.0 | 0.0 | 0.0 | 6.4 | 0.0 | 0.0 | 0.0 | 0.0 | 0.0 |
| hsa-miR-0144-3p | 0.0 | 0.0 | 0.0 | 0.0 | 0.0 | 0.0 | 0.0 | 0.0 | 2.9 |
| hsa-miR-0145-3p | 0.0 | 0.0 | 0.0 | 2.9 | 0.0 | 0.0 | 0.0 | 0.0 | 2.7 |
| hsa-miR-0146a-3p | 0.0 | 0.0 | 0.0 | 0.0 | 0.0 | 0.0 | 3.2 | 0.0 | 0.0 |
| hsa-miR-0146b-5p | 0.0 | 0.0 | 3.8 | 4.1 | 0.0 | 0.0 | 3.7 | 0.0 | 0.0 |
| hsa-miR-0148a-5p | 0.0 | 0.0 | 0.0 | 0.0 | 0.0 | 0.0 | 0.0 | 0.0 | 0.0 |
| hsa-miR-0148b-3p | 2.1 | 0.0 | 0.0 | 4.7 | 0.0 | 0.0 | 0.0 | 0.0 | 0.0 |
| hsa-miR-0148b-5p | 0.0 | 2.8 | 0.0 | 0.0 | 2.2 | 0.0 | 3.3 | 0.0 | 0.0 |
| hsa-miR-0151a-3p | 0.0 | 0.0 | 3.8 | 0.0 | 0.0 | 2.2 | 3.6 | 0.0 | 0.0 |
| hsa-miR-0151a-5p | 0.0 | 2.1 | 0.0 | 0.0 | 0.0 | 1.7 | 0.0 | 0.0 | 0.0 |
| hsa-miR-0152-3p | 0.0 | 0.0 | 0.0 | 0.0 | 0.0 | 0.0 | 0.0 | 0.0 | 0.0 |
| hsa-miR-0153-3p | 0.0 | 0.0 | 0.0 | 0.0 | 0.0 | 0.0 | 2.7 | 0.0 | 0.0 |
| hsa-miR-0154-3p | 0.0 | 2.6 | 0.0 | 0.0 | 0.0 | 1.3 | 0.0 | 0.0 | 0.0 |
| hsa-miR-0155-3p | 0.0 | 2.5 | 0.0 | 2.5 | 2.7 | 1.4 | 0.0 | 0.0 | 3.3 |
| hsa-miR-0155-5p | 0.0 | 0.0 | 0.0 | 5.8 | 0.0 | 0.0 | 0.0 | 0.0 | 0.0 |
| hsa-miR-0181a-3p | 0.0 | 0.0 | 4.4 | 0.0 | 0.0 | 0.0 | 0.0 | 0.0 | 0.0 |
| hsa-miR-0181b-2-3p | 0.0 | 0.0 | 0.0 | 3.0 | 0.0 | 0.0 | 3.0 | 0.0 | 0.0 |
| hsa-miR-0181c-3p | 0.0 | 0.0 | 0.0 | 0.0 | 0.0 | 0.0 | 0.0 | 0.0 | 0.0 |
| hsa-miR-0181c-5p | 0.0 | 0.0 | 0.0 | 0.0 | 0.0 | 0.0 | 2.7 | 0.0 | 0.0 |
| hsa-miR-0181d-5p | 0.0 | 0.0 | 0.0 | 2.8 | 0.0 | 0.0 | 0.0 | 0.0 | 0.0 |
| hsa-miR-0182-3p | 0.0 | 0.0 | 0.0 | 0.0 | 1.3 | 0.0 | 3.1 | 0.0 | 0.0 |
| hsa-miR-0182-5p | 0.0 | 0.0 | 2.8 | 2.4 | 1.8 | 0.0 | 3.3 | 0.0 | 0.0 |
| hsa-miR-0186-3p | 0.0 | 0.0 | 0.0 | 0.0 | 0.0 | 0.0 | 2.9 | 0.0 | 0.0 |
| hsa-miR-0186-5p | 0.0 | 0.0 | 0.0 | 0.0 | 1.9 | 0.0 | 0.0 | 0.0 | 0.0 |
| hsa-miR-0188-3p | 0.0 | 0.0 | 1.8 | 0.0 | 2.2 | 2.1 | 2.9 | 2.3 | 0.0 |
| hsa-miR-0190a-3p | 0.0 | 2.7 | 0.0 | 2.3 | 0.0 | 0.0 | 4.0 | 0.0 | 0.0 |
| hsa-miR-0190b | 0.0 | 2.5 | 0.0 | 4.6 | 0.0 | 0.0 | 0.0 | 0.0 | 0.0 |
| hsa-miR-0191-5p | 0.0 | 0.0 | 0.0 | 3.0 | 0.0 | 0.0 | 2.6 | 0.0 | 0.0 |
| hsa-miR-0192-3p | 0.0 | 0.0 | 3.9 | 4.1 | 1.3 | 0.0 | 0.0 | 0.0 | 2.6 |
| hsa-miR-0193a-3p | 0.0 | 0.0 | 0.0 | 0.0 | 1.7 | 0.0 | 0.0 | 0.0 | 0.0 |
| hsa-miR-0200a-3p | 0.0 | 0.0 | 0.0 | 0.0 | 0.0 | 0.0 | 0.0 | 0.0 | 0.0 |
| hsa-miR-0200c-3p | 0.0 | 0.0 | 0.0 | 0.0 | 0.0 | 0.0 | 0.0 | 0.0 | 0.0 |
| hsa-miR-0202-5p | 0.0 | 0.0 | 0.0 | 0.0 | 0.0 | 0.0 | 3.2 | 3.4 | 0.0 |
| hsa-miR-0203a-5p | 0.0 | 3.4 | 1.7 | 4.2 | 0.0 | 1.3 | 0.0 | 0.0 | 0.0 |
| hsa-miR-0208a-3p | 0.0 | 0.0 | 0.0 | 6.3 | 0.0 | 0.0 | 0.0 | 0.0 | 0.0 |
| hsa-miR-0208b-3p | 0.0 | 2.5 | 2.4 | 3.2 | 0.0 | 0.0 | 0.0 | 0.0 | 0.0 |
| hsa-miR-0216a-5p | 0.0 | 0.0 | 1.9 | 3.0 | 1.7 | 1.4 | 0.0 | 0.0 | 0.0 |
| hsa-miR-0216b-5p | 0.0 | 2.6 | 4.2 | 0.0 | 0.0 | 0.0 | 0.0 | 0.0 | 0.0 |
| hsa-miR-0218-1-3p | 0.0 | 0.0 | 3.8 | 4.0 | 0.0 | 0.0 | 3.2 | 0.0 | 0.0 |
| hsa-miR-0218-2-3p | 0.0 | 2.1 | 0.0 | 0.0 | 0.0 | 0.0 | 0.0 | 0.0 | 0.0 |
| hsa-miR-0218-5p | 0.0 | 3.0 | 0.0 | 0.0 | 0.0 | 0.0 | 2.7 | 0.0 | 0.0 |
| hsa-miR-0219a-2-3p | 0.0 | 0.0 | 0.0 | 0.0 | 0.0 | 0.0 | 0.0 | 0.0 | 0.0 |
| hsa-miR-0219a-5p | 0.0 | 0.0 | 0.0 | 4.3 | 0.0 | 0.0 | 0.0 | 0.0 | 0.0 |
| hsa-miR-0224-5p | 0.0 | 2.5 | 0.0 | 0.0 | 0.0 | 0.0 | 0.0 | 0.0 | 0.0 |
| hsa-miR-0298 | 0.0 | 0.0 | 0.0 | 0.0 | 2.9 | 0.0 | 3.2 | 0.0 | 0.0 |
| hsa-miR-0299-3p | 0.0 | 2.5 | 0.0 | 0.0 | 2.1 | 2.2 | 0.0 | 0.0 | 0.0 |
| hsa-miR-0301a-3p | 0.0 | 2.3 | 0.0 | 0.0 | 0.0 | 0.0 | 0.0 | 0.0 | 0.0 |
| hsa-miR-0301a-5p | 0.0 | 3.2 | 0.0 | 0.0 | 2.0 | 1.5 | 0.0 | 0.0 | 3.0 |
| hsa-miR-0301b-3p | 0.0 | 0.0 | 3.3 | 0.0 | 0.0 | 0.0 | 0.0 | 0.0 | 0.0 |
| hsa-miR-0301b-5p | 0.0 | 0.0 | 0.0 | 0.0 | 0.0 | 3.3 | 0.0 | 0.0 | 0.0 |
| hsa-miR-0302a-5p | 0.0 | 0.0 | 2.2 | 0.0 | 0.0 | 0.0 | 3.1 | 0.0 | 0.0 |
| hsa-miR-0302b-3p | 0.0 | 0.0 | 0.0 | 0.0 | 0.0 | 0.0 | 3.2 | 0.0 | 0.0 |
| hsa-miR-0302b-5p | 0.0 | 0.0 | 0.0 | 4.8 | 1.9 | 0.0 | 3.9 | 0.0 | 0.0 |
| hsa-miR-0302c-3p | 0.0 | 0.0 | 0.0 | 0.0 | 2.4 | 0.0 | 0.0 | 0.0 | 0.0 |
| hsa-miR-0302d-3p | 0.0 | 1.9 | 2.9 | 0.0 | 2.0 | 1.6 | 0.0 | 0.0 | 0.0 |
| hsa-miR-0302f | 0.0 | 0.0 | 0.0 | 0.0 | 1.9 | 0.0 | 3.2 | 0.0 | 0.0 |
| hsa -miR -0320d | 0.0 | 0.0 | 0.0 | 0.0 | 0.0 | 0.0 | 0.0 | 0.0 | 0.0 |
| hsa -miR -0320e | 0.0 | 0.0 | 0.0 | 0.0 | 0.0 | 0.0 | 0.0 | 0.0 | 0.0 |
| hsa-miR-0323b-3p | 0.0 | 0.0 | 0.0 | 5.5 | 0.0 | 1.3 | 0.0 | 0.0 | 0.0 |
| hsa-miR-0329-3p | 0.0 | 0.0 | 0.0 | 0.0 | 0.0 | 0.0 | 3.0 | 0.0 | 0.0 |
| hsa-miR-0331-5p | 0.0 | 0.0 | 0.0 | 0.0 | 0.0 | 0.0 | 3.1 | 0.0 | 0.0 |
| hsa-miR-0335-3p | 0.0 | 3.0 | 0.0 | 0.0 | 2.8 | 2.1 | 0.0 | 0.0 | 0.0 |
| hsa-miR-0337-5p | 0.0 | 0.0 | 0.0 | 0.0 | 0.0 | 0.0 | 0.0 | 0.0 | 0.0 |
| hsa-miR-0340-5p | 0.0 | 0.0 | 0.0 | 2.5 | 0.0 | 0.0 | 0.0 | 0.0 | 0.0 |
| hsa-miR-0361-5p | 0.0 | 0.0 | 0.0 | 6.2 | 0.0 | 0.0 | 2.7 | 0.0 | 0.0 |
| hsa-miR-0362-5p | 0.0 | 0.0 | 2.4 | 0.0 | 0.0 | 0.0 | 0.0 | 0.0 | 0.0 |
| hsa-miR-0363-3p | 0.0 | 0.0 | 2.5 | 0.0 | 0.0 | 0.0 | 0.0 | 0.0 | 0.0 |
| hsa-miR-0367-5p | 0.0 | 0.0 | 0.0 | 0.0 | 0.0 | 0.0 | 2.5 | 0.0 | 0.0 |
| hsa-miR-0372-5p | 1.6 | 0.0 | 0.0 | 0.0 | 0.0 | 0.0 | 0.0 | 0.0 | 0.0 |
| hsa-miR-0374a-3p | 0.0 | 0.0 | 0.0 | 0.0 | 0.0 | 0.0 | 0.0 | 0.0 | 3.3 |
| hsa-miR-0374a-5p | 0.0 | 0.0 | 0.0 | 6.4 | 0.0 | 0.0 | 0.0 | 0.0 | 0.0 |
| hsa-miR-0374c-5p | 1.7 | 2.0 | 0.0 | 0.0 | 0.0 | 0.0 | 0.0 | 0.0 | 0.0 |
| hsa-miR-0375 | 0.0 | 0.0 | 4.1 | 0.0 | 0.0 | 2.6 | 0.0 | 0.0 | 0.0 |
| hsa-miR-0376a-2-5p | 0.0 | 2.0 | 0.0 | 0.0 | 0.0 | 0.0 | 0.0 | 0.0 | 0.0 |
| hsa-miR-0376a-3p | 0.0 | 0.0 | 2.9 | 0.0 | 0.0 | 0.0 | 0.0 | 0.0 | 0.0 |
| hsa-miR-0376a-5p | 0.0 | 0.0 | 0.0 | 0.0 | 0.0 | 0.0 | 2.8 | 0.0 | 0.0 |
| hsa-miR-0376b-3p | 0.0 | 0.0 | 0.0 | 3.4 | 0.0 | 0.0 | 0.0 | 0.0 | 0.0 |
| hsa-miR-0376b-5p | 0.0 | 0.0 | 0.0 | 0.0 | 0.0 | 0.0 | 2.8 | 0.0 | 0.0 |
| hsa-miR-0376c-5p | 0.0 | 0.0 | 0.0 | 0.0 | 0.0 | 0.0 | 3.6 | 0.0 | 0.0 |
| hsa-miR-0377-3p | 3.3 | 0.0 | 0.0 | 5.5 | 0.0 | 0.0 | 3.7 | 0.0 | 0.0 |
| hsa-miR-0377-5p | 0.0 | 0.0 | 2.1 | 0.0 | 1.2 | 2.0 | 0.0 | 0.0 | 0.0 |
| hsa-miR-0378j | 0.0 | 0.0 | 0.0 | 6.4 | 0.0 | 0.0 | 0.0 | 0.0 | 0.0 |
| hsa-miR-0379-5p | 0.0 | 0.0 | 0.0 | 0.0 | 0.0 | 0.0 | 2.9 | 0.0 | 0.0 |
| hsa-miR-0380-3p | 0.0 | 0.0 | 3.8 | 0.0 | 2.7 | 2.7 | 0.0 | 0.0 | 0.0 |
| hsa-miR-0381-3p | 0.0 | 0.0 | 0.0 | 0.0 | 0.0 | 0.0 | 0.0 | 0.0 | 3.0 |
| hsa-miR-0384 | 0.0 | 0.0 | 6.2 | 0.0 | 0.0 | 0.0 | 0.0 | 0.0 | 0.0 |
| hsa-miR-0409-5p | 0.0 | 0.0 | 3.6 | 3.8 | 0.0 | 0.0 | 3.9 | 0.0 | 3.3 |
| hsa-miR-0410-3p | 0.0 | 0.0 | 0.0 | 0.0 | 2.0 | 0.0 | 2.7 | 0.0 | 0.0 |
| hsa-miR-0411-5p | 0.0 | 0.0 | 3.5 | 0.0 | 0.0 | 0.0 | 0.0 | 0.0 | 0.0 |
| hsa-miR-0412-5p | 0.0 | 0.0 | 3.1 | 5.1 | 0.0 | 0.0 | 0.0 | 0.0 | 0.0 |
| hsa-miR-0424-3p | 0.0 | 0.0 | 1.6 | 0.0 | 5.2 | 5.1 | 0.0 | 3.1 | 0.0 |
| hsa-miR-0424-5p | 1.9 | 2.7 | 3.2 | 0.0 | 0.0 | 0.0 | 0.0 | 0.0 | 0.0 |
| hsa-miR-0429 | 0.0 | 0.0 | 0.0 | 0.0 | 0.0 | 0.0 | 0.0 | 0.0 | 0.0 |
| hsa-miR-0433-3p | 0.0 | 0.0 | 0.0 | 0.0 | 0.0 | 0.0 | 0.0 | 0.0 | 0.0 |
| hsa-miR-0433-5p | 2.7 | 3.6 | 2.4 | 0.0 | 2.4 | 3.6 | 0.0 | 0.0 | 0.0 |
| hsa-miR-0448 | 0.0 | 2.2 | 0.0 | 0.0 | 0.0 | 2.3 | 2.6 | 3.3 | 0.0 |
| hsa-miR-0450a-2-3p | 2.0 | 0.0 | 2.4 | 4.5 | 0.0 | 0.0 | 0.0 | 0.0 | 0.0 |
| hsa-miR-0451a | 0.0 | 0.0 | 3.6 | 0.0 | 0.0 | 0.0 | 0.0 | 0.0 | 0.0 |
| hsa-miR-0451b | 0.0 | 0.0 | 2.5 | 4.7 | 0.0 | 0.0 | 3.5 | 0.0 | 0.0 |
| hsa-miR-0454-3p | 0.0 | 0.0 | 0.0 | 0.0 | 0.0 | 0.0 | 3.6 | 0.0 | 0.0 |
| hsa-miR-0455-3p | 0.0 | 2.4 | 0.0 | 6.0 | 2.2 | 0.0 | 0.0 | 0.0 | 0.0 |
| hsa-miR-0487a-3p | 0.0 | 0.0 | 0.0 | 0.0 | 2.1 | 0.0 | 0.0 | 0.0 | 0.0 |
| hsa-miR-0489-3p | 2.3 | 3.0 | 0.0 | 0.0 | 0.0 | 0.0 | 0.0 | 0.0 | 0.0 |
| hsa-miR-0490-3p | 1.9 | 0.0 | 0.0 | 0.0 | 1.8 | 0.0 | 0.0 | 0.0 | 0.0 |
| hsa-miR-0490-5p | 0.0 | 1.9 | 0.0 | 0.0 | 0.0 | 0.0 | 0.0 | 0.0 | 0.0 |
| hsa-miR-0492 | 0.0 | 2.5 | 0.0 | 0.0 | 2.0 | 2.1 | 0.0 | 0.0 | 0.0 |
| hsa-miR-0493-5p | 3.3 | 0.0 | 0.0 | 4.3 | 0.0 | 0.0 | 0.0 | 0.0 | 0.0 |
| hsa-miR-0495-3p | 0.0 | 2.0 | 0.0 | 2.4 | 1.9 | 0.0 | 3.7 | 0.0 | 2.9 |
| hsa-miR-0496 | 0.0 | 0.0 | 2.5 | 0.0 | 2.3 | 0.0 | 0.0 | 0.0 | 0.0 |
| hsa-miR-0497-5p | 1.9 | 0.0 | 0.0 | 0.0 | 0.0 | 0.0 | 3.1 | 0.0 | 0.0 |
| hsa-miR-0499a-3p | 0.0 | 0.0 | 0.0 | 0.0 | 0.0 | 0.0 | 3.7 | 0.0 | 0.0 |
| hsa-miR-0499a-5p | 0.0 | 0.0 | 0.0 | 0.0 | 0.0 | 0.0 | 3.0 | 0.0 | 0.0 |
| hsa-miR-0500a-3p | 0.0 | 1.8 | 0.0 | 0.0 | 2.3 | 1.7 | 0.0 | 0.0 | 0.0 |
| hsa-miR-0501-3p | 0.0 | 0.0 | 0.0 | 0.0 | 1.2 | 0.0 | 0.0 | 0.0 | 0.0 |
| hsa-miR-0503-5p | 0.0 | 0.0 | 0.0 | 2.5 | 1.8 | 0.0 | 0.0 | 0.0 | 0.0 |
| hsa-miR-0504-5p | 0.0 | 0.0 | 0.0 | 3.2 | 0.0 | 0.0 | 3.6 | 0.0 | 0.0 |
| hsa-miR-0506-5p | 0.0 | 0.0 | 2.2 | 0.0 | 0.0 | 0.0 | 0.0 | 0.0 | 0.0 |
| hsa-miR-0509-5p | 0.0 | 0.0 | 0.0 | 0.0 | 0.0 | 0.0 | 4.1 | 0.0 | 0.0 |
| hsa-miR-0513b-3p | 0.0 | 0.0 | 0.0 | 4.8 | 1.2 | 0.0 | 0.0 | 0.0 | 0.0 |
| hsa-miR-0513c-5p | 0.0 | 2.3 | 0.0 | 0.0 | 0.0 | 0.0 | 0.0 | 0.0 | 0.0 |
| hsa-miR-0514a-5p | 0.0 | 0.0 | 0.0 | 2.6 | 0.0 | 0.0 | 0.0 | 0.0 | 0.0 |
| hsa-miR-0515-5p | 1.6 | 0.0 | 0.0 | 5.3 | 2.0 | 0.0 | 0.0 | 0.0 | 0.0 |
| hsa-miR-0516a-5p | 1.5 | 0.0 | 0.0 | 3.1 | 0.0 | 0.0 | 3.9 | 0.0 | 0.0 |
| hsa-miR-0516b-3p, hsa-miR-516a-3p | 0.0 | 0.0 | 0.0 | 0.0 | 0.0 | 0.0 | 0.0 | 0.0 | 0.0 |
| hsa-miR-0517c-3p | 0.0 | 2.1 | 0.0 | 0.0 | 1.7 | 2.3 | 0.0 | 0.0 | 0.0 |
| hsa-miR-0518a-3p | 0.0 | 0.0 | 0.0 | 0.0 | 1.6 | 0.0 | 0.0 | 0.0 | 0.0 |
| hsa-miR-0518c-3p | 0.0 | 0.0 | 0.0 | 0.0 | 0.0 | 0.0 | 0.0 | 3.3 | 0.0 |
| hsa-miR-0518d-3p | 1.9 | 0.0 | 2.1 | 0.0 | 0.0 | 0.0 | 2.6 | 4.9 | 0.0 |
| hsa-miR-0519c-3p | 0.0 | 0.0 | 0.0 | 2.6 | 0.0 | 0.0 | 0.0 | 0.0 | 0.0 |
| hsa-miR-0520a-5p | 0.0 | 0.0 | 2.5 | 0.0 | 2.9 | 0.0 | 0.0 | 0.0 | 0.0 |
| hsa-miR-0520c-3p | 0.0 | 3.5 | 3.7 | 5.2 | 0.0 | 0.0 | 0.0 | 0.0 | 0.0 |
| hsa-miR-0520d-3p | 0.0 | 0.0 | 0.0 | 0.0 | 2.6 | 0.0 | 0.0 | 0.0 | 3.2 |
| hsa-miR-0520f-3p | 0.0 | 0.0 | 0.0 | 0.0 | 1.8 | 1.6 | 0.0 | 0.0 | 0.0 |
| hsa-miR-0520f-5p | 0.0 | 0.0 | 0.0 | 0.0 | 0.0 | 2.3 | 2.5 | 0.0 | 0.0 |
| hsa-miR-0520g-5p | 0.0 | 0.0 | 0.0 | 0.0 | 1.7 | 0.0 | 2.8 | 0.0 | 0.0 |
| hsa-miR-0521 | 2.2 | 2.3 | 2.3 | 0.0 | 3.1 | 2.6 | 0.0 | 0.0 | 0.0 |
| hsa-miR-0522-3p | 0.0 | 0.0 | 0.0 | 0.0 | 0.0 | 0.0 | 0.0 | 0.0 | 0.0 |
| hsa-miR-0523-3p | 0.0 | 0.0 | 0.0 | 0.0 | 1.9 | 0.0 | 0.0 | 0.0 | 0.0 |
| hsa-miR-0524-3p | 0.0 | 0.0 | 0.0 | 0.0 | 0.0 | 0.0 | 3.1 | 0.0 | 0.0 |
| hsa-miR-0539-5p | 0.0 | 0.0 | 0.0 | 0.0 | 0.0 | 1.6 | 0.0 | 0.0 | 0.0 |
| hsa-miR-0542-5p | 0.0 | 2.6 | 0.0 | 0.0 | 2.3 | 0.0 | 2.5 | 0.0 | 0.0 |
| hsa-miR-0544b | 0.0 | 0.0 | 0.0 | 0.0 | 1.8 | 0.0 | 0.0 | 0.0 | 0.0 |
| hsa-miR-0545-3p | 0.0 | 0.0 | 0.0 | 0.0 | 0.0 | 0.0 | 2.5 | 0.0 | 0.0 |
| hsa-miR-0545-5p | 0.0 | 0.0 | 0.0 | 5.5 | 0.0 | 0.0 | 0.0 | 0.0 | 0.0 |
| hsa-miR-0548a-3p | 0.0 | 0.0 | 0.0 | 6.3 | 0.0 | 0.0 | 0.0 | 0.0 | 0.0 |
| hsa-miR-0548a-5p | 0.0 | 0.0 | 0.0 | 3.8 | 0.0 | 0.0 | 0.0 | 0.0 | 0.0 |
| hsa-miR-0548aa, hsa-miR-548t-3p | 0.0 | 0.0 | 0.0 | 0.0 | 0.0 | 0.0 | 0.0 | 0.0 | 2.7 |
| hsa-miR-0548ab | 0.0 | 0.0 | 0.0 | 0.0 | 0.0 | 0.0 | 0.0 | 0.0 | 0.0 |
| hsa-miR-0548ac | 0.0 | 0.0 | 2.1 | 0.0 | 0.0 | 0.0 | 0.0 | 0.0 | 0.0 |
| hsa-miR-0548ag | 0.0 | 0.0 | 0.0 | 6.1 | 0.0 | 0.0 | 0.0 | 0.0 | 0.0 |
| hsa-miR-0548ah-3p | 0.0 | 0.0 | 0.0 | 4.5 | 0.0 | 0.0 | 3.2 | 0.0 | 0.0 |
| hsa-miR-0548ai, hsa-miR-570-5p | 0.0 | 0.0 | 0.0 | 6.1 | 0.0 | 0.0 | 0.0 | 0.0 | 0.0 |
| hsa-miR-0548al | 0.0 | 0.0 | 0.0 | 0.0 | 0.0 | 0.0 | 3.7 | 0.0 | 0.0 |
| hsa-miR-0548am-3p | 0.0 | 0.0 | 0.0 | 0.0 | 0.0 | 0.0 | 3.3 | 0.0 | 0.0 |
| hsa-miR-0548an | 0.0 | 3.1 | 0.0 | 3.2 | 0.0 | 0.0 | 2.6 | 0.0 | 0.0 |
| hsa-miR-0548ao-3p | 0.0 | 0.0 | 0.0 | 5.1 | 0.0 | 0.0 | 3.9 | 0.0 | 0.0 |
| hsa-miR-0548ao-5p | 0.0 | 0.0 | 2.3 | 0.0 | 0.0 | 0.0 | 0.0 | 0.0 | 0.0 |
| hsa-miR-0548ap-3p | 0.0 | 0.0 | 0.0 | 0.0 | 3.5 | 0.0 | 0.0 | 0.0 | 0.0 |
| hsa-miR-0548aq-5p | 0.0 | 2.1 | 0.0 | 0.0 | 0.0 | 0.0 | 0.0 | 0.0 | 0.0 |
| hsa-miR-0548as-3p | 0.0 | 0.0 | 3.6 | 4.8 | 0.0 | 0.0 | 0.0 | 0.0 | 0.0 |
| hsa-miR-0548as-5p | 1.7 | 0.0 | 2.8 | 5.0 | 0.0 | 0.0 | 0.0 | 0.0 | 0.0 |
| hsa-miR-0548at-5p | 0.0 | 0.0 | 0.0 | 0.0 | 0.0 | 0.0 | 2.7 | 0.0 | 3.3 |
| hsa-miR-0548au-3p | 0.0 | 2.1 | 0.0 | 0.0 | 0.0 | 0.0 | 3.1 | 0.0 | 0.0 |
| hsa-miR-0548av-3p | 0.0 | 0.0 | 0.0 | 0.0 | 0.0 | 0.0 | 3.7 | 0.0 | 2.6 |
| hsa-miR-0548ay-5p | 0.0 | 0.0 | 0.0 | 0.0 | 0.0 | 0.0 | 0.0 | 0.0 | 0.0 |
| hsa-miR-0548az-3p | 0.0 | 0.0 | 0.0 | 0.0 | 0.0 | 0.0 | 3.3 | 0.0 | 0.0 |
| hsa-miR-0548b-3p | 0.0 | 0.0 | 2.8 | 0.0 | 0.0 | 0.0 | 3.5 | 0.0 | 0.0 |
| hsa-miR-0548c-3p | 0.0 | 0.0 | 0.0 | 0.0 | 0.0 | 0.0 | 3.4 | 0.0 | 0.0 |
| hsa-miR-0548c-5p, hsa-miR-548o-5p, hsa-miR-548am-5p | 0.0 | 0.0 | 0.0 | 5.8 | 0.0 | 0.0 | 0.0 | 0.0 | 0.0 |
| hsa-miR-0548e-3p | 0.0 | 0.0 | 0.0 | 4.9 | 0.0 | 0.0 | 0.0 | 0.0 | 0.0 |
| hsa-miR-0548e-5p | 0.0 | 0.0 | 0.0 | 0.0 | 0.0 | 0.0 | 3.9 | 0.0 | 0.0 |
| hsa-miR-0548f-3p | 0.0 | 0.0 | 0.0 | 0.0 | 0.0 | 0.0 | 3.3 | 0.0 | 0.0 |
| hsa-miR-0548h-5p | 0.0 | 0.0 | 0.0 | 0.0 | 0.0 | 0.0 | 0.0 | 0.0 | 0.0 |
| hsa-miR-0548j-5p | 0.0 | 2.4 | 4.4 | 5.2 | 0.0 | 0.0 | 0.0 | 0.0 | 0.0 |
| hsa -miR -0548k | 0.0 | 0.0 | 0.0 | 0.0 | 2.1 | 0.0 | 0.0 | 0.0 | 0.0 |
| hsa -miR -05481 | 0.0 | 0.0 | 1.8 | 0.0 | 0.0 | 0.0 | 0.0 | 0.0 | 0.0 |
| hsa -miR -0548m | 0.0 | 0.0 | 0.0 | 0.0 | 0.0 | 0.0 | 0.0 | 0.0 | 3.3 |
| hsa -miR -0548p | 0.0 | 0.0 | 0.0 | 0.0 | 0.0 | 0.0 | 3.2 | 0.0 | 0.0 |
| hsa -miR -0548s | 0.0 | 0.0 | 0.0 | 0.0 | 0.0 | 0.0 | 3.8 | 0.0 | 0.0 |
| hsa-miR-0548t-5p | 0.0 | 0.0 | 0.0 | 0.0 | 0.0 | 0.0 | 3.0 | 0.0 | 0.0 |
| hsa -miR -0548w | 0.0 | 0.0 | 0.0 | 0.0 | 0.0 | 0.0 | 0.0 | 0.0 | 0.0 |
| hsa-miR-0551b-3p | 2.1 | 3.7 | 2.8 | 0.0 | 3.2 | 2.8 | 0.0 | 0.0 | 0.0 |
| hsa-miR-0551b-5p | 0.0 | 0.0 | 8.1 | 0.0 | 5.2 | 5.6 | 0.0 | 0.0 | 0.0 |
| hsa-miR-0552-3p | 0.0 | 1.8 | 0.0 | 0.0 | 0.0 | 0.0 | 0.0 | 0.0 | 0.0 |
| hsa-miR-0554 | 0.0 | 0.0 | 2.5 | 0.0 | 0.0 | 0.0 | 2.7 | 0.0 | 0.0 |
| hsa-miR-0555 | 0.0 | 0.0 | 0.0 | 0.0 | 0.0 | 0.0 | 0.0 | 0.0 | 0.0 |
| hsa-miR-0556-5p | 0.0 | 0.0 | 0.0 | 0.0 | 0.0 | 0.0 | 2.6 | 0.0 | 0.0 |
| hsa-miR-0559 | 0.0 | 0.0 | 0.0 | 0.0 | 0.0 | 0.0 | 2.9 | 0.0 | 0.0 |
| hsa-miR-0563 | 0.0 | 0.0 | 0.0 | 0.0 | 0.0 | 2.1 | 0.0 | 0.0 | 0.0 |
| hsa-miR-0567 | 0.0 | 0.0 | 0.0 | 2.9 | 0.0 | 0.0 | 0.0 | 0.0 | 0.0 |
| hsa-miR-0570-3p | 0.0 | 0.0 | 0.0 | 0.0 | 0.0 | 2.1 | 0.0 | 0.0 | 0.0 |
| hsa-miR-0571 | 0.0 | 0.0 | 0.0 | 0.0 | 0.0 | 0.0 | 0.0 | 0.0 | 0.0 |
| hsa-miR-0573 | 0.0 | 2.0 | 0.0 | 0.0 | 0.0 | 0.0 | 0.0 | 0.0 | 0.0 |
| hsa-miR-0582-3p | 0.0 | 0.0 | 0.0 | 0.0 | 2.0 | 0.0 | 0.0 | 0.0 | 0.0 |
| hsa-miR-0582-5p | 0.0 | 1.8 | 0.0 | 0.0 | 0.0 | 0.0 | 2.9 | 0.0 | 0.0 |
| hsa-miR-0586 | 0.0 | 0.0 | 0.0 | 0.0 | 0.0 | 0.0 | 0.0 | 3.2 | 0.0 |
| hsa-miR-0587 | 0.0 | 0.0 | 2.9 | 0.0 | 0.0 | 0.0 | 2.7 | 0.0 | 0.0 |
| hsa-miR-0588 | 0.0 | 0.0 | 0.0 | 0.0 | 0.0 | 0.0 | 2.7 | 0.0 | 0.0 |
| hsa-miR-0592 | 0.0 | 0.0 | 0.0 | 0.0 | 0.0 | 0.0 | 0.0 | 0.0 | 0.0 |
| hsa-miR-0597-3p | 0.0 | 2.1 | 2.6 | 4.2 | 0.0 | 0.0 | 0.0 | 0.0 | 0.0 |
| hsa-miR-0597-5p | 0.0 | 0.0 | 0.0 | 0.0 | 0.0 | 0.0 | 0.0 | 0.0 | 3.1 |
| hsa-miR-0598-3p | 2.0 | 2.9 | 0.0 | 0.0 | 0.0 | 0.0 | 0.0 | 0.0 | 0.0 |
| hsa-miR-0599 | 0.0 | 0.0 | 0.0 | 6.3 | 0.0 | 0.0 | 0.0 | 0.0 | 0.0 |
| hsa-miR-0600 | 0.0 | 0.0 | 0.0 | 6.4 | 0.0 | 0.0 | 2.5 | 0.0 | 0.0 |
| hsa-miR-0601 | 0.0 | 0.0 | 0.0 | 0.0 | 2.9 | 2.5 | 0.0 | 0.0 | 0.0 |
| hsa-miR-0616-3p | 0.0 | 0.0 | 0.0 | 0.0 | 0.0 | 0.0 | 0.0 | 0.0 | 0.0 |
| hsa-miR-0616-5p | 0.0 | 2.0 | 0.0 | 5.8 | 0.0 | 0.0 | 0.0 | 0.0 | 0.0 |
| hsa-miR-0619-3p | 0.0 | 0.0 | 0.0 | 0.0 | 0.0 | 0.0 | 3.4 | 0.0 | 0.0 |
| hsa-miR-0623 | 2.6 | 0.0 | 0.0 | 0.0 | 0.0 | 2.2 | 0.0 | 0.0 | 0.0 |
| hsa-miR-0624-3p | 0.0 | 2.3 | 2.5 | 6.1 | 0.0 | 0.0 | 4.1 | 0.0 | 0.0 |
| hsa-miR-0627-3p | 0.0 | 0.0 | 0.0 | 0.0 | 0.0 | 0.0 | 3.3 | 0.0 | 0.0 |
| hsa-miR-0629-5p | 0.0 | 0.0 | 0.0 | 0.0 | 1.3 | 0.0 | 3.0 | 0.0 | 0.0 |
| hsa-miR-0631 | 2.4 | 3.4 | 2.9 | 6.0 | 0.0 | 2.9 | 2.8 | 0.0 | 0.0 |
| hsa-miR-0633 | 0.0 | 0.0 | 0.0 | 0.0 | 0.0 | 0.0 | 3.4 | 0.0 | 0.0 |
| hsa-miR-0639 | 0.0 | 0.0 | 0.0 | 0.0 | 0.0 | 0.0 | 0.0 | 0.0 | 0.0 |
| hsa-miR-0640 | 0.0 | 0.0 | 0.0 | 0.0 | 1.6 | 0.0 | 0.0 | 0.0 | 0.0 |
| hsa-miR-0641 | 0.0 | 0.0 | 0.0 | 6.8 | 0.0 | 0.0 | 0.0 | 0.0 | 0.0 |
| hsa-miR-0643 | 1.7 | 1.9 | 3.6 | 2.6 | 2.6 | 0.0 | 2.9 | 0.0 | 0.0 |
| hsa -miR -0644a | 2.8 | 0.0 | 4.4 | 3.1 | 0.0 | 2.4 | 0.0 | 0.0 | 3.5 |
| hsa-miR-0646 | 0.0 | 0.0 | 3.3 | 5.4 | 0.0 | 0.0 | 2.6 | 0.0 | 0.0 |
| hsa-miR-0647 | 0.0 | 0.0 | 0.0 | 0.0 | 0.0 | 0.0 | 3.5 | 0.0 | 0.0 |
| hsa-miR-0653-3p | 0.0 | 0.0 | 0.0 | 0.0 | 0.0 | 0.0 | 2.8 | 0.0 | 0.0 |
| hsa-miR-0653-5p | 0.0 | 0.0 | 0.0 | 6.6 | 0.0 | 0.0 | 0.0 | 0.0 | 0.0 |
| hsa-miR-0655-3p | 2.6 | 0.0 | 0.0 | 5.1 | 0.0 | 0.0 | 0.0 | 0.0 | 0.0 |
| hsa-miR-0655-5p | 0.0 | 2.2 | 0.0 | 0.0 | 0.0 | 0.0 | 2.5 | 0.0 | 0.0 |
| hsa-miR-0656-3p | 2.0 | 0.0 | 1.9 | 4.9 | 1.2 | 0.0 | 3.7 | 0.0 | 0.0 |
| hsa-miR-0659-5p | 2.5 | 2.1 | 0.0 | 0.0 | 1.2 | 0.0 | 3.5 | 0.0 | 0.0 |
| hsa-miR-0660-5p | 0.0 | 0.0 | 0.0 | 0.0 | 1.3 | 1.3 | 0.0 | 0.0 | 0.0 |
| hsa-miR-0758-3p | 0.0 | 0.0 | 0.0 | 6.2 | 0.0 | 0.0 | 0.0 | 0.0 | 0.0 |
| hsa-miR-0759 | 0.0 | 0.0 | 0.0 | 0.0 | 0.0 | 0.0 | 3.2 | 0.0 | 0.0 |
| hsa-miR-0761 | 0.0 | 2.1 | 0.0 | 3.8 | 0.0 | 2.1 | 0.0 | 0.0 | 0.0 |
| hsa-miR-0769-5p | 2.1 | 3.0 | 0.0 | 2.4 | 0.0 | 1.2 | 3.0 | 0.0 | 0.0 |
| hsa-miR-0802 | 0.0 | 0.0 | 0.0 | 2.6 | 2.0 | 0.0 | 3.8 | 0.0 | 0.0 |
| hsa-miR-0875-5p | 0.0 | 0.0 | 0.0 | 2.4 | 0.0 | 0.0 | 0.0 | 0.0 | 0.0 |
| hsa-miR-0876-3p | 0.0 | 0.0 | 0.0 | 0.0 | 0.0 | 0.0 | 0.0 | 0.0 | 0.0 |
| hsa-miR-0890 | 0.0 | 0.0 | 0.0 | 5.8 | 0.0 | 0.0 | 0.0 | 0.0 | 0.0 |
| hsa-miR-0892c-3p | 0.0 | 2.7 | 0.0 | 0.0 | 0.0 | 1.9 | 0.0 | 0.0 | 0.0 |
| hsa-miR-0924 | 0.0 | 2.9 | 0.0 | 0.0 | 0.0 | 0.0 | 0.0 | 0.0 | 0.0 |
| hsa-miR-0934 | 0.0 | 0.0 | 0.0 | 0.0 | 1.2 | 0.0 | 0.0 | 0.0 | 3.5 |
| hsa-miR-0941 | 2.5 | 0.0 | 3.4 | 0.0 | 0.0 | 0.0 | 0.0 | 0.0 | 0.0 |
| hsa-miR-0944 | 0.0 | 0.0 | 0.0 | 3.5 | 1.5 | 0.0 | 0.0 | 0.0 | 0.0 |
| hsa-miR-1178-5p | 0.0 | 0.0 | 1.6 | 8.0 | 0.0 | 0.0 | 0.0 | 0.0 | 3.8 |
| hsa-miR-1179 | 0.0 | 0.0 | 0.0 | 4.7 | 0.0 | 0.0 | 3.9 | 0.0 | 0.0 |
| hsa-miR-1180-3p | 1.9 | 2.8 | 2.3 | 4.5 | 1.2 | 0.0 | 0.0 | 0.0 | 0.0 |
| hsa-miR-1180-5p | 0.0 | 0.0 | 0.0 | 0.0 | 0.0 | 0.0 | 4.2 | 0.0 | 0.0 |
| hsa-miR-1183 | 0.0 | 0.0 | 0.0 | 0.0 | 0.0 | 0.0 | 0.0 | 0.0 | 0.0 |
| hsa-miR-1185-5p | 0.0 | 0.0 | 0.0 | 6.6 | 0.0 | 0.0 | 2.5 | 0.0 | 0.0 |
| hsa-miR-1208 | 0.0 | 0.0 | 0.0 | 0.0 | 0.0 | 0.0 | 0.0 | 0.0 | 0.0 |
| hsa-miR-1226-5p | 0.0 | 3.3 | 1.9 | 0.0 | 3.7 | 3.9 | 2.6 | 0.0 | 0.0 |
| hsa-miR-1245b-3p | 0.0 | 0.0 | 0.0 | 0.0 | 0.0 | 0.0 | 3.6 | 0.0 | 0.0 |
| hsa-miR-1245b-5p | 0.0 | 0.0 | 0.0 | 0.0 | 0.0 | 0.0 | 3.7 | 0.0 | 0.0 |
| hsa-miR-1248 | 0.0 | 0.0 | 0.0 | 2.6 | 0.0 | 0.0 | 2.7 | 0.0 | 0.0 |
| hsa-miR-1251-5p | 0.0 | 0.0 | 0.0 | 0.0 | 1.6 | 0.0 | 0.0 | 0.0 | 0.0 |
| hsa-miR-1252-3p | 0.0 | 0.0 | 0.0 | 4.8 | 0.0 | 0.0 | 2.5 | 0.0 | 0.0 |
| hsa-miR-1253 | 0.0 | 0.0 | 0.0 | 0.0 | 0.0 | 0.0 | 0.0 | 0.0 | 0.0 |
| hsa-miR-1255b-5p | 0.0 | 0.0 | 0.0 | 0.0 | 0.0 | 0.0 | 0.0 | 0.0 | 0.0 |
| hsa-miR-1256 | 0.0 | 0.0 | 3.8 | 4.5 | 2.3 | 0.0 | 0.0 | 0.0 | 0.0 |
| hsa-miR-1257 | 0.0 | 0.0 | 2.7 | 3.4 | 0.0 | 0.0 | 0.0 | 0.0 | 0.0 |
| hsa-miR-1263 | 0.0 | 2.0 | 0.0 | 0.0 | 2.4 | 0.0 | 0.0 | 0.0 | 0.0 |
| hsa-miR-1264 | 0.0 | 1.8 | 0.0 | 0.0 | 0.0 | 0.0 | 0.0 | 0.0 | 0.0 |
| hsa-miR-1265 | 0.0 | 2.8 | 0.0 | 0.0 | 2.1 | 2.5 | 3.8 | 2.6 | 0.0 |
| hsa-miR-1269a | 0.0 | 0.0 | 0.0 | 0.0 | 0.0 | 1.5 | 0.0 | 0.0 | 0.0 |
| hsa-miR-1270 | 0.0 | 0.0 | 0.0 | 0.0 | 0.0 | 0.0 | 0.0 | 0.0 | 0.0 |
| hsa-miR-1272 | 0.0 | 0.0 | 0.0 | 3.6 | 0.0 | 0.0 | 0.0 | 0.0 | 0.0 |
| hsa-miR-1273a | 0.0 | 0.0 | 3.9 | 4.2 | 0.0 | 2.5 | 4.3 | 2.3 | 0.0 |
| hsa-miR-1273c | 0.0 | 0.0 | 2.1 | 0.0 | 2.4 | 1.5 | 0.0 | 0.0 | 0.0 |
| hsa-miR-1273d | 0.0 | 0.0 | 0.0 | 2.6 | 0.0 | 0.0 | 0.0 | 0.0 | 0.0 |
| hsa-miR-1277-5p | 0.0 | 0.0 | 0.0 | 5.6 | 0.0 | 0.0 | 2.6 | 0.0 | 0.0 |
| hsa-miR-1279 | 0.0 | 0.0 | 0.0 | 3.1 | 0.0 | 0.0 | 0.0 | 0.0 | 0.0 |
| hsa-miR-1282 | 0.0 | 0.0 | 0.0 | 0.0 | 2.8 | 0.0 | 0.0 | 0.0 | 0.0 |
| hsa-miR-1283 | 0.0 | 0.0 | 0.0 | 6.6 | 0.0 | 0.0 | 0.0 | 0.0 | 0.0 |
| hsa-miR-1285-3p | 0.0 | 0.0 | 0.0 | 0.0 | 2.4 | 3.1 | 0.0 | 2.7 | 0.0 |
| hsa-miR-1289 | 0.0 | 1.9 | 0.0 | 2.6 | 0.0 | 0.0 | 0.0 | 0.0 | 0.0 |
| hsa-miR-1290 | 0.0 | 0.0 | 0.0 | 0.0 | 0.0 | 0.0 | 0.0 | 0.0 | 0.0 |
| hsa-miR-1292-5p | 0.0 | 0.0 | 2.2 | 0.0 | 0.0 | 2.0 | 0.0 | 0.0 | 0.0 |
| hsa-miR-1294 | 0.0 | 0.0 | 0.0 | 0.0 | 2.4 | 0.0 | 0.0 | 0.0 | 0.0 |
| hsa-miR-1295a | 0.0 | 0.0 | 0.0 | 0.0 | 0.0 | 0.0 | 0.0 | 0.0 | 0.0 |
| hsa-miR-1295b-5p | 0.0 | 0.0 | 0.0 | 0.0 | 0.0 | 0.0 | 2.6 | 0.0 | 0.0 |
| hsa-miR-1302 | 0.0 | 0.0 | 2.1 | 0.0 | 0.0 | 0.0 | 3.0 | 0.0 | 0.0 |
| hsa-miR-1304-5p | 0.0 | 0.0 | 4.7 | 5.0 | 0.0 | 0.0 | 0.0 | 0.0 | 3.7 |
| hsa-miR-1305 | 0.0 | 0.0 | 0.0 | 0.0 | 0.0 | 0.0 | 0.0 | 2.7 | 0.0 |
| hsa-miR-1307-5p | 0.0 | 1.8 | 0.0 | 0.0 | 1.6 | 2.1 | 3.4 | 0.0 | 0.0 |
| hsa-miR-1321 | 2.4 | 0.0 | 0.0 | 0.0 | 1.9 | 2.5 | 0.0 | 0.0 | 0.0 |
| hsa-miR-1468-3p | 0.0 | 0.0 | 0.0 | 0.0 | 0.0 | 0.0 | 0.0 | 0.0 | 2.7 |
| hsa-miR-1537-3p | 0.0 | 0.0 | 0.0 | 2.8 | 0.0 | 0.0 | 2.8 | 0.0 | 2.7 |
| hsa-miR-1827 | 0.0 | 0.0 | 0.0 | 4.7 | 0.0 | 0.0 | 0.0 | 0.0 | 0.0 |
| hsa-miR-1911-5p | 0.0 | 0.0 | 0.0 | 2.6 | 0.0 | 0.0 | 0.0 | 0.0 | 0.0 |
| hsa-miR-1973 | 0.0 | 0.0 | 0.0 | 0.0 | 0.0 | 0.0 | 0.0 | 0.0 | 0.0 |
| hsa-miR-2052 | 0.0 | 0.0 | 0.0 | 3.8 | 0.0 | 0.0 | 0.0 | 0.0 | 0.0 |
| hsa-miR-2053 | 0.0 | 0.0 | 0.0 | 6.4 | 0.0 | 0.0 | 0.0 | 0.0 | 0.0 |
| hsa-miR-2054 | 0.0 | 0.0 | 0.0 | 6.3 | 0.0 | 0.0 | 0.0 | 0.0 | 0.0 |
| hsa-miR-2114-3p | 0.0 | 0.0 | 0.0 | 0.0 | 1.2 | 0.0 | 3.2 | 0.0 | 0.0 |
| hsa-miR-2114-5p | 0.0 | 0.0 | 0.0 | 0.0 | 0.0 | 0.0 | 0.0 | 0.0 | 0.0 |
| hsa-miR-2115-3p | 0.0 | 0.0 | 0.0 | 0.0 | 0.0 | 0.0 | 3.4 | 0.0 | 0.0 |
| hsa-miR-2115-5p | 0.0 | 2.5 | 0.0 | 0.0 | 0.0 | 0.0 | 3.0 | 0.0 | 0.0 |
| hsa-miR-2681-3p | 0.0 | 0.0 | 0.0 | 0.0 | 0.0 | 0.0 | 0.0 | 0.0 | 0.0 |
| hsa-miR-3065-5p | 2.6 | 2.3 | 3.2 | 0.0 | 0.0 | 0.0 | 0.0 | 0.0 | 0.0 |
| hsa-miR-3119 | 0.0 | 0.0 | 0.0 | 5.0 | 0.0 | 0.0 | 2.8 | 0.0 | 0.0 |
| hsa-miR-3120-3p | 0.0 | 2.9 | 0.0 | 0.0 | 0.0 | 0.0 | 3.4 | 0.0 | 0.0 |
| hsa-miR-3122 | 4.0 | 4.4 | 5.4 | 0.0 | 1.6 | 2.1 | 0.0 | 4.4 | 3.4 |
| hsa-miR-3123 | 0.0 | 0.0 | 0.0 | 0.0 | 1.9 | 2.5 | 0.0 | 0.0 | 0.0 |
| hsa-miR-3125 | 2.9 | 3.4 | 0.0 | 0.0 | 0.0 | 0.0 | 2.6 | 0.0 | 0.0 |
| hsa-miR-3127-3p | 5.4 | 5.0 | 5.4 | 0.0 | 0.0 | 0.0 | 5.9 | 3.6 | 4.1 |
| hsa-miR-3130-3p | 2.1 | 2.8 | 2.0 | 2.8 | 0.0 | 0.0 | 3.3 | 0.0 | 0.0 |
| hsa-miR-3130-5p | 4.4 | 4.8 | 4.2 | 0.0 | 0.0 | 0.0 | 4.4 | 4.8 | 4.0 |
| hsa-miR-3135a | 0.0 | 0.0 | 0.0 | 4.7 | 0.0 | 0.0 | 0.0 | 0.0 | 0.0 |
| hsa-miR-3140-5p | 0.0 | 0.0 | 3.0 | 2.6 | 0.0 | 0.0 | 0.0 | 0.0 | 0.0 |
| hsa-miR-3148 | 0.0 | 2.8 | 1.8 | 3.8 | 0.0 | 0.0 | 0.0 | 0.0 | 3.4 |
| hsa-miR-3149 | 2.5 | 4.1 | 0.0 | 6.4 | 0.0 | 0.0 | 4.1 | 0.0 | 2.8 |
| hsa-miR-3150a-3p | 5.2 | 4.3 | 5.5 | 6.3 | 0.0 | 0.0 | 5.0 | 3.8 | 4.3 |
| hsa-miR-3150b-5p | 4.9 | 5.1 | 5.1 | 0.0 | 1.2 | 0.0 | 6.0 | 4.4 | 4.6 |
| hsa-miR-3151-3p | 5.7 | 5.9 | 5.3 | 0.0 | 0.0 | 0.0 | 5.9 | 5.5 | 5.0 |
| hsa-miR-3151-5p | 6.2 | 5.9 | 6.1 | 0.0 | 0.0 | 0.0 | 6.0 | 5.7 | 5.4 |
| hsa-miR-3160-5p | 3.2 | 4.2 | 3.4 | 0.0 | 0.0 | 0.0 | 3.9 | 3.7 | 0.0 |
| hsa-miR-3181 | 0.0 | 0.0 | 3.0 | 6.0 | 0.0 | 1.2 | 4.6 | 0.0 | 0.0 |
| hsa-miR-3182 | 0.0 | 0.0 | 4.5 | 4.9 | 0.0 | 0.0 | 0.0 | 0.0 | 0.0 |
| hsa-miR-3183 | 0.0 | 0.0 | 0.0 | 0.0 | 2.1 | 0.0 | 3.2 | 0.0 | 0.0 |
| hsa-miR-3192-5p | 0.0 | 3.6 | 0.0 | 0.0 | 2.8 | 2.7 | 3.7 | 0.0 | 2.5 |
| hsa-miR-3194-3p | 0.0 | 0.0 | 0.0 | 0.0 | 0.0 | 0.0 | 0.0 | 0.0 | 0.0 |
| hsa-miR-3199 | 0.0 | 2.3 | 2.5 | 5.7 | 2.8 | 4.8 | 3.7 | 0.0 | 0.0 |
| hsa-miR-3591-5p | 0.0 | 2.2 | 0.0 | 4.3 | 0.0 | 0.0 | 0.0 | 0.0 | 0.0 |
| hsa-miR-3606-3p | 0.0 | 0.0 | 0.0 | 0.0 | 0.0 | 0.0 | 3.2 | 0.0 | 0.0 |
| hsa-miR-3607-3p | 0.0 | 0.0 | 0.0 | 0.0 | 0.0 | 0.0 | 3.2 | 0.0 | 0.0 |
| hsa-miR-3611 | 0.0 | 0.0 | 0.0 | 0.0 | 0.0 | 0.0 | 4.0 | 0.0 | 0.0 |
| hsa-miR-3613-3p | 0.0 | 0.0 | 0.0 | 2.4 | 1.7 | 2.4 | 3.2 | 0.0 | 0.0 |
| hsa-miR-3614-3p | 0.0 | 0.0 | 2.5 | 0.0 | 0.0 | 0.0 | 3.2 | 0.0 | 2.6 |
| hsa-miR-3617-5p | 0.0 | 0.0 | 0.0 | 0.0 | 0.0 | 0.0 | 2.7 | 0.0 | 0.0 |
| hsa-miR-3653-3p | 0.0 | 0.0 | 0.0 | 0.0 | 0.0 | 0.0 | 0.0 | 0.0 | 0.0 |
| hsa-miR-3660 | 2.3 | 0.0 | 0.0 | 0.0 | 0.0 | 2.0 | 2.9 | 0.0 | 0.0 |
| hsa-miR-3661 | 0.0 | 3.6 | 0.0 | 0.0 | 2.6 | 3.4 | 3.4 | 0.0 | 0.0 |
| hsa-miR-3664-5p | 0.0 | 0.0 | 0.0 | 0.0 | 0.0 | 0.0 | 0.0 | 0.0 | 3.3 |
| hsa-miR-3670 | 0.0 | 0.0 | 0.0 | 0.0 | 0.0 | 0.0 | 0.0 | 0.0 | 3.0 |
| hsa-miR-3671 | 0.0 | 0.0 | 0.0 | 0.0 | 0.0 | 0.0 | 2.9 | 0.0 | 0.0 |
| hsa-miR-3672 | 0.0 | 0.0 | 0.0 | 0.0 | 0.0 | 0.0 | 2.6 | 0.0 | 0.0 |
| hsa-miR-3674 | 0.0 | 0.0 | 0.0 | 0.0 | 0.0 | 0.0 | 2.6 | 0.0 | 0.0 |
| hsa-miR-3675-5p | 0.0 | 2.5 | 0.0 | 0.0 | 0.0 | 1.3 | 0.0 | 2.3 | 0.0 |
| hsa-miR-3680-5p | 3.1 | 3.9 | 0.0 | 0.0 | 2.9 | 2.9 | 3.3 | 0.0 | 0.0 |
| hsa-miR-3681-5p | 0.0 | 0.0 | 0.0 | 5.3 | 0.0 | 0.0 | 0.0 | 0.0 | 0.0 |
| hsa-miR-3683 | 0.0 | 0.0 | 0.0 | 0.0 | 1.4 | 0.0 | 0.0 | 0.0 | 0.0 |
| hsa-miR-3689a-5p, hsa-miR-3689b-5p, hsa-miR-3689e | 0.0 | 2.1 | 0.0 | 0.0 | 0.0 | 0.0 | 3.1 | 0.0 | 0.0 |
| hsa-miR-3907 | 0.0 | 0.0 | 0.0 | 0.0 | 0.0 | 0.0 | 3.0 | 0.0 | 0.0 |
| hsa-miR-3909 | 0.0 | 0.0 | 2.0 | 2.4 | 0.0 | 0.0 | 3.2 | 0.0 | 0.0 |
| hsa-miR-3910 | 2.3 | 0.0 | 0.0 | 0.0 | 1.6 | 1.9 | 2.9 | 0.0 | 0.0 |
| hsa-miR-3912-5p | 0.0 | 0.0 | 0.0 | 0.0 | 0.0 | 0.0 | 0.0 | 0.0 | 0.0 |
| hsa-miR-3913-5p | 2.2 | 2.3 | 0.0 | 0.0 | 0.0 | 1.6 | 3.5 | 0.0 | 0.0 |
| hsa-miR-3915 | 0.0 | 0.0 | 0.0 | 0.0 | 2.2 | 0.0 | 3.4 | 0.0 | 0.0 |
| hsa-miR-3919 | 2.8 | 1.9 | 3.2 | 0.0 | 2.4 | 0.0 | 0.0 | 0.0 | 0.0 |
| hsa-miR-3920 | 0.0 | 0.0 | 0.0 | 5.7 | 0.0 | 0.0 | 4.3 | 0.0 | 2.6 |
| hsa-miR-3924 | 0.0 | 2.9 | 0.0 | 0.0 | 1.3 | 0.0 | 0.0 | 0.0 | 0.0 |
| hsa-miR-3925-3p | 0.0 | 0.0 | 0.0 | 0.0 | 0.0 | 0.0 | 0.0 | 0.0 | 0.0 |
| hsa-miR-3926 | 0.0 | 1.8 | 0.0 | 0.0 | 2.7 | 1.3 | 3.4 | 0.0 | 0.0 |
| hsa-miR-3938 | 0.0 | 0.0 | 1.8 | 4.1 | 0.0 | 0.0 | 2.9 | 0.0 | 0.0 |
| hsa-miR-3939 | 0.0 | 0.0 | 0.0 | 0.0 | 0.0 | 0.0 | 0.0 | 0.0 | 0.0 |
| hsa-miR-3941 | 0.0 | 2.8 | 0.0 | 0.0 | 0.0 | 0.0 | 0.0 | 0.0 | 0.0 |
| hsa -miR -3942-3p | 0.0 | 0.0 | 0.0 | 0.0 | 0.0 | 0.0 | 0.0 | 0.0 | 0.0 |
| hsa -miR -3942-5p | 0.0 | 0.0 | 0.0 | 0.0 | 0.0 | 0.0 | 0.0 | 0.0 | 0.0 |
| hsa-miR-3974 | 0.0 | 0.0 | 3.7 | 0.0 | 0.0 | 0.0 | 0.0 | 0.0 | 0.0 |
| hsa-miR-3977 | 3.2 | 0.0 | 2.3 | 4.9 | 2.2 | 2.5 | 0.0 | 0.0 | 0.0 |
| hsa-miR-3978 | 0.0 | 0.0 | 3.4 | 4.8 | 0.0 | 0.0 | 0.0 | 0.0 | 0.0 |
| hsa-miR-4263 | 0.0 | 0.0 | 0.0 | 0.0 | 0.0 | 0.0 | 4.0 | 0.0 | 0.0 |
| hsa-miR-4267 | 0.0 | 2.7 | 1.8 | 0.0 | 2.1 | 3.6 | 4.1 | 0.0 | 0.0 |
| hsa-miR-4285 | 0.0 | 0.0 | 3.3 | 0.0 | 0.0 | 0.0 | 0.0 | 0.0 | 0.0 |
| hsa-miR-4289 | 0.0 | 0.0 | 0.0 | 0.0 | 2.1 | 0.0 | 0.0 | 0.0 | 0.0 |
| hsa-miR-4293 | 0.0 | 0.0 | 1.9 | 5.6 | 0.0 | 0.0 | 3.1 | 0.0 | 0.0 |
| hsa-miR-4309 | 0.0 | 0.0 | 0.0 | 3.0 | 0.0 | 0.0 | 2.9 | 0.0 | 0.0 |
| hsa-miR-4311 | 2.3 | 0.0 | 0.0 | 0.0 | 0.0 | 2.1 | 0.0 | 0.0 | 0.0 |
| hsa-miR-4315 | 0.0 | 2.0 | 0.0 | 0.0 | 1.2 | 1.9 | 3.5 | 0.0 | 0.0 |
| hsa-miR-4321 | 0.0 | 2.6 | 0.0 | 2.4 | 3.7 | 3.0 | 0.0 | 3.0 | 0.0 |
| hsa-miR-4418 | 0.0 | 0.0 | 0.0 | 0.0 | 0.0 | 0.0 | 0.0 | 0.0 | 0.0 |
| hsa-miR-4422 | 0.0 | 0.0 | 2.4 | 0.0 | 0.0 | 0.0 | 0.0 | 0.0 | 0.0 |
| hsa-miR-4423-3p | 0.0 | 2.3 | 0.0 | 0.0 | 0.0 | 0.0 | 2.7 | 0.0 | 0.0 |
| hsa-miR-4424 | 0.0 | 0.0 | 2.2 | 0.0 | 0.0 | 0.0 | 0.0 | 0.0 | 0.0 |
| hsa-miR-4427 | 0.0 | 0.0 | 0.0 | 0.0 | 0.0 | 2.2 | 0.0 | 0.0 | 0.0 |
| hsa-miR-4431 | 2.2 | 2.5 | 0.0 | 0.0 | 2.3 | 1.7 | 0.0 | 0.0 | 0.0 |
| hsa-miR-4432 | 0.0 | 0.0 | 1.7 | 2.5 | 0.0 | 0.0 | 0.0 | 0.0 | 0.0 |
| hsa-miR-4435 | 2.3 | 6.4 | 3.6 | 5.8 | 6.2 | 5.9 | 0.0 | 5.5 | 0.0 |
| hsa-miR-4439 | 0.0 | 3.3 | 0.0 | 0.0 | 3.3 | 2.0 | 2.7 | 0.0 | 0.0 |
| hsa-miR-4445-5p | 2.2 | 0.0 | 2.7 | 5.1 | 0.0 | 0.0 | 3.5 | 3.4 | 0.0 |
| hsa-miR-4452 | 0.0 | 0.0 | 0.0 | 0.0 | 0.0 | 0.0 | 2.7 | 0.0 | 0.0 |
| hsa-miR-4457 | 0.0 | 2.1 | 0.0 | 0.0 | 0.0 | 0.0 | 0.0 | 0.0 | 0.0 |
| hsa-miR-4458 | 0.0 | 2.6 | 0.0 | 0.0 | 2.5 | 2.2 | 3.3 | 0.0 | 0.0 |
| hsa-miR-4461 | 0.0 | 0.0 | 0.0 | 6.8 | 0.0 | 0.0 | 0.0 | 0.0 | 0.0 |
| hsa-miR-4464 | 0.0 | 0.0 | 0.0 | 0.0 | 0.0 | 0.0 | 0.0 | 0.0 | 0.0 |
| hsa-miR-4468 | 0.0 | 0.0 | 0.0 | 0.0 | 0.0 | 0.0 | 0.0 | 0.0 | 0.0 |
| hsa-miR-4469 | 0.0 | 0.0 | 0.0 | 2.4 | 0.0 | 0.0 | 0.0 | 0.0 | 0.0 |
| hsa-miR-4470 | 0.0 | 2.8 | 3.1 | 2.6 | 4.7 | 4.3 | 4.0 | 0.0 | 0.0 |
| hsa-miR-4471 | 0.0 | 3.3 | 0.0 | 0.0 | 0.0 | 2.5 | 0.0 | 0.0 | 0.0 |
| hsa-miR-4475 | 0.0 | 0.0 | 0.0 | 0.0 | 1.5 | 0.0 | 0.0 | 0.0 | 0.0 |
| hsa-miR-4480 | 0.0 | 0.0 | 0.0 | 0.0 | 0.0 | 0.0 | 2.7 | 0.0 | 0.0 |
| hsa-miR-4485-3p | 0.0 | 0.0 | 0.0 | 0.0 | 0.0 | 0.0 | 0.0 | 0.0 | 0.0 |
| hsa-miR-4490 | 0.0 | 0.0 | 2.1 | 0.0 | 0.0 | 0.0 | 0.0 | 0.0 | 0.0 |
| hsa-miR-4493 | 0.0 | 0.0 | 3.4 | 4.9 | 0.0 | 0.0 | 0.0 | 0.0 | 3.1 |
| hsa-miR-4500 | 0.0 | 0.0 | 0.0 | 0.0 | 2.5 | 0.0 | 0.0 | 0.0 | 0.0 |
| hsa-miR-4503 | 0.0 | 0.0 | 0.0 | 0.0 | 0.0 | 0.0 | 3.0 | 0.0 | 0.0 |
| hsa-miR-4504 | 0.0 | 0.0 | 0.0 | 6.3 | 0.0 | 0.0 | 0.0 | 0.0 | 0.0 |
| hsa-miR-4509 | 0.0 | 0.0 | 3.7 | 3.2 | 0.0 | 0.0 | 0.0 | 0.0 | 0.0 |
| hsa-miR-4510 | 0.0 | 2.2 | 0.0 | 0.0 | 2.4 | 2.3 | 0.0 | 0.0 | 0.0 |
| hsa-miR-4511 | 0.0 | 2.4 | 0.0 | 0.0 | 0.0 | 0.0 | 0.0 | 0.0 | 0.0 |
| hsa-miR-4512 | 0.0 | 0.0 | 0.0 | 0.0 | 0.0 | 0.0 | 3.1 | 0.0 | 0.0 |
| hsa-miR-4515 | 0.0 | 3.5 | 3.2 | 3.5 | 3.0 | 3.7 | 3.8 | 3.9 | 3.9 |
| hsa-miR-4517 | 0.0 | 3.7 | 0.0 | 2.6 | 0.0 | 0.0 | 0.0 | 0.0 | 0.0 |
| hsa-miR-4519 | 0.0 | 0.0 | 0.0 | 6.0 | 1.5 | 1.5 | 3.7 | 0.0 | 0.0 |
| hsa-miR-4520-2-3p | 0.0 | 0.0 | 0.0 | 0.0 | 2.3 | 0.0 | 0.0 | 0.0 | 0.0 |
| hsa-miR-4522 | 0.0 | 0.0 | 2.9 | 0.0 | 2.4 | 1.8 | 3.7 | 0.0 | 0.0 |
| hsa-miR-4524a-3p | 0.0 | 2.4 | 2.1 | 0.0 | 0.0 | 0.0 | 0.0 | 0.0 | 0.0 |
| hsa -miR -4536-3p | 0.0 | 0.0 | 0.0 | 0.0 | 0.0 | 0.0 | 3.0 | 0.0 | 0.0 |
| hsa-miR-4537 | 0.0 | 0.0 | 0.0 | 0.0 | 0.0 | 1.9 | 0.0 | 0.0 | 0.0 |
| hsa-miR-4633-3p | 0.0 | 0.0 | 2.2 | 4.1 | 0.0 | 0.0 | 0.0 | 0.0 | 0.0 |
| hsa-miR-4637 | 0.0 | 0.0 | 3.3 | 4.6 | 0.0 | 0.0 | 2.9 | 0.0 | 0.0 |
| hsa-miR-4643 | 2.5 | 2.3 | 3.8 | 0.0 | 2.1 | 1.8 | 0.0 | 0.0 | 0.0 |
| hsa-miR-4650-3p | 0.0 | 0.0 | 0.0 | 0.0 | 0.0 | 0.0 | 0.0 | 0.0 | 0.0 |
| hsa-miR-4653-3p | 0.0 | 2.6 | 1.7 | 5.1 | 0.0 | 0.0 | 0.0 | 0.0 | 0.0 |
| hsa-miR-4659a-3p | 0.0 | 0.0 | 1.8 | 0.0 | 0.0 | 0.0 | 0.0 | 0.0 | 0.0 |
| hsa-miR-4659a-5p | 0.0 | 0.0 | 4.9 | 0.0 | 2.0 | 0.0 | 2.6 | 0.0 | 0.0 |
| hsa-miR-4659b-5p | 0.0 | 2.3 | 2.4 | 0.0 | 0.0 | 0.0 | 0.0 | 0.0 | 0.0 |
| hsa-miR-4661-3p | 2.7 | 3.0 | 0.0 | 0.0 | 1.8 | 1.7 | 0.0 | 0.0 | 0.0 |
| hsa-miR-4662a-3p | 0.0 | 2.8 | 0.0 | 0.0 | 0.0 | 0.0 | 0.0 | 0.0 | 0.0 |
| hsa-miR-4662a-5p | 0.0 | 0.0 | 0.0 | 6.3 | 0.0 | 0.0 | 3.3 | 0.0 | 0.0 |
| hsa-miR-4666a-3p | 0.0 | 0.0 | 0.0 | 5.0 | 0.0 | 0.0 | 0.0 | 0.0 | 0.0 |
| hsa -miR -4666b | 0.0 | 0.0 | 0.0 | 0.0 | 0.0 | 1.4 | 0.0 | 0.0 | 0.0 |
| hsa-miR-4668-3p | 0.0 | 0.0 | 0.0 | 2.9 | 0.0 | 0.0 | 0.0 | 0.0 | 0.0 |
| hsa-miR-4670-3p | 0.0 | 0.0 | 0.0 | 0.0 | 0.0 | 0.0 | 0.0 | 0.0 | 0.0 |
| hsa-miR-4670-5p | 0.0 | 0.0 | 3.2 | 0.0 | 0.0 | 0.0 | 3.1 | 0.0 | 0.0 |
| hsa-miR-4671-3p | 0.0 | 0.0 | 0.0 | 0.0 | 0.0 | 0.0 | 0.0 | 0.0 | 0.0 |
| hsa-miR-4679 | 0.0 | 0.0 | 0.0 | 0.0 | 1.6 | 0.0 | 0.0 | 0.0 | 0.0 |
| hsa-miR-4683 | 0.0 | 0.0 | 0.0 | 0.0 | 0.0 | 0.0 | 0.0 | 0.0 | 0.0 |
| hsa-miR-4699-3p | 0.0 | 0.0 | 0.0 | 0.0 | 0.0 | 0.0 | 0.0 | 0.0 | 0.0 |
| hsa-miR-4699-5p | 0.0 | 0.0 | 4.5 | 4.8 | 0.0 | 0.0 | 0.0 | 0.0 | 0.0 |
| hsa-miR-4704-5p | 0.0 | 0.0 | 2.6 | 0.0 | 0.0 | 0.0 | 0.0 | 0.0 | 0.0 |
| hsa-miR-4705 | 0.0 | 0.0 | 0.0 | 0.0 | 0.0 | 0.0 | 0.0 | 0.0 | 0.0 |
| hsa-miR-4714-3p | 0.0 | 0.0 | 0.0 | 6.1 | 0.0 | 0.0 | 0.0 | 0.0 | 0.0 |
| hsa-miR-4715-3p | 0.0 | 0.0 | 3.1 | 0.0 | 0.0 | 0.0 | 0.0 | 0.0 | 0.0 |
| hsa-miR-4715-5p | 0.0 | 2.6 | 0.0 | 0.0 | 1.9 | 1.3 | 0.0 | 0.0 | 0.0 |
| hsa-miR-4719 | 0.0 | 0.0 | 0.0 | 0.0 | 0.0 | 0.0 | 3.3 | 0.0 | 0.0 |
| hsa-miR-4720-3p | 0.0 | 0.0 | 0.0 | 6.1 | 0.0 | 0.0 | 0.0 | 0.0 | 0.0 |
| hsa-miR-4724-5p | 1.7 | 2.9 | 0.0 | 6.9 | 0.0 | 2.0 | 0.0 | 0.0 | 0.0 |
| hsa-miR-4727-5p | 0.0 | 0.0 | 2.7 | 3.3 | 0.0 | 0.0 | 4.1 | 0.0 | 0.0 |
| hsa-miR-4735-3p | 0.0 | 0.0 | 2.4 | 0.0 | 0.0 | 0.0 | 0.0 | 0.0 | 0.0 |
| hsa-miR-4737 | 2.1 | 3.4 | 0.0 | 0.0 | 2.6 | 2.7 | 0.0 | 0.0 | 0.0 |
| hsa-miR-4743-3p | 0.0 | 3.1 | 0.0 | 0.0 | 1.6 | 0.0 | 0.0 | 0.0 | 0.0 |
| hsa-miR-4744 | 0.0 | 0.0 | 0.0 | 0.0 | 0.0 | 0.0 | 3.8 | 0.0 | 0.0 |
| hsa-miR-4753-3p | 3.2 | 3.3 | 2.3 | 0.0 | 1.8 | 2.0 | 0.0 | 0.0 | 0.0 |
| hsa-miR-4753-5p | 0.0 | 2.4 | 1.9 | 0.0 | 0.0 | 0.0 | 3.6 | 0.0 | 0.0 |
| hsa-miR-4757-5p | 0.0 | 0.0 | 2.2 | 0.0 | 0.0 | 0.0 | 3.2 | 0.0 | 2.7 |
| hsa-miR-4759 | 0.0 | 0.0 | 0.0 | 0.0 | 0.0 | 1.6 | 0.0 | 0.0 | 0.0 |
| hsa-miR-4760-5p | 0.0 | 0.0 | 0.0 | 3.8 | 0.0 | 0.0 | 0.0 | 0.0 | 0.0 |
| hsa-miR-4761-5p | 0.0 | 0.0 | 3.7 | 4.8 | 0.0 | 1.3 | 3.2 | 0.0 | 0.0 |
| hsa-miR-4762-5p | 0.0 | 2.0 | 0.0 | 6.4 | 0.0 | 0.0 | 2.7 | 0.0 | 0.0 |
| hsa-miR-4764-5p | 0.0 | 0.0 | 0.0 | 0.0 | 1.2 | 0.0 | 0.0 | 0.0 | 0.0 |
| hsa-miR-4768-5p | 2.5 | 0.0 | 0.0 | 0.0 | 0.0 | 2.1 | 0.0 | 0.0 | 0.0 |
| hsa-miR-4770 | 2.5 | 2.9 | 0.0 | 0.0 | 0.0 | 0.0 | 3.1 | 0.0 | 0.0 |
| hsa-miR-4772-3p | 0.0 | 0.0 | 0.0 | 5.9 | 0.0 | 0.0 | 0.0 | 0.0 | 0.0 |
| hsa-miR-4774-3p | 0.0 | 2.0 | 0.0 | 0.0 | 1.8 | 2.2 | 0.0 | 0.0 | 0.0 |
| hsa-miR-4774-5p | 0.0 | 0.0 | 0.0 | 0.0 | 0.0 | 0.0 | 2.8 | 0.0 | 0.0 |
| hsa-miR-4777-5p | 0.0 | 0.0 | 0.0 | 5.5 | 0.0 | 0.0 | 0.0 | 0.0 | 0.0 |
| hsa-miR-4778-3p | 0.0 | 1.8 | 0.0 | 0.0 | 0.0 | 0.0 | 0.0 | 0.0 | 0.0 |
| hsa-miR-4778-5p | 0.0 | 0.0 | 0.0 | 0.0 | 0.0 | 0.0 | 0.0 | 0.0 | 0.0 |
| hsa-miR-4779 | 0.0 | 0.0 | 0.0 | 0.0 | 0.0 | 0.0 | 0.0 | 0.0 | 0.0 |
| hsa-miR-4781-5p | 0.0 | 0.0 | 0.0 | 0.0 | 2.3 | 0.0 | 0.0 | 0.0 | 0.0 |
| hsa-miR-4782-5p | 5.7 | 0.0 | 0.0 | 0.0 | 0.0 | 0.0 | 0.0 | 0.0 | 0.0 |
| hsa-miR-4785 | 2.7 | 3.4 | 4.3 | 2.9 | 3.9 | 3.3 | 3.5 | 3.7 | 0.0 |
| hsa-miR-4789-3p | 0.0 | 0.0 | 0.0 | 0.0 | 0.0 | 0.0 | 2.7 | 0.0 | 0.0 |
| hsa-miR-4789-5p | 0.0 | 2.1 | 0.0 | 0.0 | 0.0 | 0.0 | 0.0 | 0.0 | 0.0 |
| hsa-miR-4791 | 0.0 | 0.0 | 0.0 | 5.0 | 0.0 | 0.0 | 0.0 | 0.0 | 0.0 |
| hsa-miR-4792 | 0.0 | 2.6 | 3.3 | 5.4 | 1.7 | 4.3 | 3.2 | 4.0 | 0.0 |
| hsa-miR-4795-3p | 0.0 | 0.0 | 0.0 | 0.0 | 0.0 | 0.0 | 0.0 | 0.0 | 0.0 |
| hsa-miR-4797-5p | 0.0 | 0.0 | 0.0 | 4.9 | 0.0 | 0.0 | 3.6 | 0.0 | 0.0 |
| hsa-miR-4798-5p | 0.0 | 0.0 | 0.0 | 5.4 | 0.0 | 0.0 | 0.0 | 0.0 | 0.0 |
| hsa-miR-4801 | 2.4 | 2.7 | 2.4 | 0.0 | 0.0 | 2.1 | 0.0 | 0.0 | 0.0 |
| hsa-miR-4803 | 0.0 | 2.0 | 0.0 | 0.0 | 0.0 | 2.3 | 2.8 | 0.0 | 0.0 |
| hsa-miR-4804-5p | 0.0 | 3.4 | 0.0 | 0.0 | 0.0 | 0.0 | 0.0 | 0.0 | 0.0 |
| hsa-miR-4999-3p | 2.0 | 2.7 | 0.0 | 0.0 | 2.6 | 2.1 | 2.6 | 0.0 | 0.0 |
| hsa-miR-5000-3p | 0.0 | 2.8 | 0.0 | 0.0 | 2.0 | 0.0 | 0.0 | 2.4 | 0.0 |
| hsa -miR -5004-3p | 3.1 | 2.2 | 3.4 | 0.0 | 2.2 | 2.5 | 0.0 | 0.0 | 0.0 |
| hsa-miR-5007-3p | 0.0 | 0.0 | 0.0 | 0.0 | 0.0 | 0.0 | 0.0 | 0.0 | 0.0 |
| hsa-miR-5009-5p | 0.0 | 0.0 | 0.0 | 0.0 | 0.0 | 0.0 | 0.0 | 0.0 | 2.6 |
| hsa-miR-5047 | 0.0 | 0.0 | 1.7 | 5.9 | 0.0 | 0.0 | 3.1 | 0.0 | 0.0 |
| hsa-miR-5089-5p | 0.0 | 2.4 | 3.4 | 3.6 | 0.0 | 0.0 | 0.0 | 0.0 | 0.0 |
| hsa-miR-5092 | 0.0 | 0.0 | 2.6 | 0.0 | 2.7 | 2.1 | 2.7 | 0.0 | 0.0 |
| hsa-miR-5579-5p | 0.0 | 0.0 | 0.0 | 0.0 | 0.0 | 0.0 | 0.0 | 0.0 | 0.0 |
| hsa -miR -5582-5p | 0.0 | 0.0 | 0.0 | 0.0 | 0.0 | 0.0 | 2.9 | 0.0 | 0.0 |
| hsa -miR -5584-5p | 0.0 | 0.0 | 0.0 | 3.2 | 0.0 | 0.0 | 0.0 | 0.0 | 0.0 |
| hsa-miR-5585-5p | 0.0 | 1.8 | 0.0 | 0.0 | 1.4 | 0.0 | 0.0 | 0.0 | 0.0 |
| hsa -miR -5586-3p | 0.0 | 2.2 | 0.0 | 0.0 | 0.0 | 0.0 | 0.0 | 0.0 | 0.0 |
| hsa -miR -5586-5p | 0.0 | 0.0 | 0.0 | 0.0 | 0.0 | 0.0 | 0.0 | 0.0 | 0.0 |
| hsa-miR-5587-5p | 0.0 | 0.0 | 0.0 | 0.0 | 2.8 | 2.9 | 4.2 | 3.8 | 2.8 |
| hsa -miR -5588-5p | 0.0 | 0.0 | 0.0 | 0.0 | 0.0 | 1.4 | 0.0 | 0.0 | 0.0 |
| hsa-miR-5590-3p | 0.0 | 0.0 | 0.0 | 2.7 | 0.0 | 0.0 | 0.0 | 0.0 | 0.0 |
| hsa-miR-5590-5p | 0.0 | 0.0 | 0.0 | 0.0 | 0.0 | 0.0 | 0.0 | 0.0 | 2.6 |
| hsa-miR-5684 | 0.0 | 1.8 | 0.0 | 0.0 | 0.0 | 0.0 | 0.0 | 0.0 | 0.0 |
| hsa-miR-5691 | 0.0 | 0.0 | 0.0 | 0.0 | 0.0 | 0.0 | 3.4 | 0.0 | 0.0 |
| hsa -miR -5692b | 0.0 | 0.0 | 0.0 | 0.0 | 0.0 | 0.0 | 0.0 | 3.8 | 2.6 |
| hsa-miR-5697 | 0.0 | 0.0 | 0.0 | 0.0 | 0.0 | 0.0 | 0.0 | 0.0 | 2.8 |
| hsa-miR-5707 | 0.0 | 0.0 | 0.0 | 0.0 | 0.0 | 1.6 | 0.0 | 0.0 | 3.0 |
| hsa-miR-6070 | 0.0 | 0.0 | 0.0 | 3.1 | 0.0 | 0.0 | 4.5 | 0.0 | 0.0 |
| hsa-miR-6071 | 0.0 | 2.2 | 0.0 | 0.0 | 0.0 | 1.2 | 0.0 | 0.0 | 0.0 |
| hsa-miR-6074 | 0.0 | 2.7 | 0.0 | 0.0 | 3.2 | 3.0 | 0.0 | 0.0 | 0.0 |
| hsa-miR-6078 | 0.0 | 0.0 | 0.0 | 0.0 | 0.0 | 0.0 | 3.0 | 0.0 | 0.0 |
| hsa-miR-6080 | 0.0 | 2.3 | 2.8 | 6.0 | 1.7 | 2.9 | 0.0 | 0.0 | 0.0 |
| hsa-miR-6084 | 0.0 | 0.0 | 0.0 | 5.3 | 2.3 | 1.9 | 3.6 | 0.0 | 0.0 |
| hsa-miR-6129 | 2.0 | 3.2 | 0.0 | 5.8 | 2.2 | 3.5 | 2.8 | 0.0 | 0.0 |
| hsa-miR-6130 | 0.0 | 2.9 | 2.0 | 0.0 | 2.6 | 3.0 | 0.0 | 0.0 | 0.0 |
| hsa-miR-6501-3p | 2.4 | 0.0 | 2.1 | 7.2 | 0.0 | 1.9 | 3.6 | 0.0 | 0.0 |
| hsa -miR -6502-3p | 0.0 | 0.0 | 3.8 | 2.8 | 0.0 | 0.0 | 0.0 | 0.0 | 0.0 |
| hsa -miR -6502-5p | 2.8 | 3.3 | 2.0 | 0.0 | 1.8 | 1.7 | 3.1 | 0.0 | 0.0 |
| hsa -miR -6506-3p | 0.0 | 0.0 | 0.0 | 0.0 | 0.0 | 0.0 | 3.5 | 0.0 | 0.0 |
| hsa-miR-6516-3p | 0.0 | 0.0 | 0.0 | 6.5 | 0.0 | 0.0 | 0.0 | 0.0 | 0.0 |
| hsa-miR-6715a-3p | 1.6 | 2.0 | 0.0 | 0.0 | 0.0 | 0.0 | 3.5 | 0.0 | 0.0 |
| hsa-miR-6715b-3p | 0.0 | 0.0 | 0.0 | 0.0 | 0.0 | 0.0 | 3.2 | 0.0 | 0.0 |
| hsa-miR-6719-3p | 0.0 | 0.0 | 0.0 | 0.0 | 0.0 | 0.0 | 0.0 | 0.0 | 0.0 |
| hsa-miR-6750-5p | 1.9 | 2.9 | 2.6 | 0.0 | 2.6 | 3.5 | 0.0 | 0.0 | 0.0 |
| hsa-miR-6755-5p | 0.0 | 0.0 | 0.0 | 0.0 | 0.0 | 1.5 | 0.0 | 0.0 | 0.0 |
| hsa-miR-6767-3p | 3.2 | 1.9 | 3.3 | 0.0 | 2.1 | 2.7 | 3.0 | 0.0 | 0.0 |
| hsa-miR-6773-5p | 0.0 | 2.3 | 0.0 | 6.4 | 0.0 | 0.0 | 0.0 | 0.0 | 0.0 |
| hsa -miR -6806-3p | 0.0 | 0.0 | 0.0 | 5.0 | 0.0 | 0.0 | 3.6 | 0.0 | 0.0 |
| hsa -miR -6808-3p | 0.0 | 2.2 | 0.0 | 0.0 | 0.0 | 1.4 | 0.0 | 0.0 | 0.0 |
| hsa-miR-6811-5p | 0.0 | 2.2 | 0.0 | 0.0 | 2.6 | 0.0 | 3.1 | 0.0 | 0.0 |
| hsa-miR-6814-5p | 2.7 | 3.7 | 2.7 | 0.0 | 2.2 | 3.4 | 0.0 | 0.0 | 0.0 |
| hsa -miR -6828-3p | 0.0 | 0.0 | 0.0 | 0.0 | 0.0 | 1.4 | 2.8 | 0.0 | 0.0 |
| hsa-miR-6835-3p | 0.0 | 0.0 | 0.0 | 2.9 | 1.9 | 0.0 | 0.0 | 0.0 | 0.0 |
| hsa -miR -6838-3p | 0.0 | 1.9 | 0.0 | 0.0 | 0.0 | 0.0 | 0.0 | 0.0 | 0.0 |
| hsa -miR -6842-3p | 0.0 | 2.2 | 0.0 | 2.7 | 0.0 | 1.9 | 0.0 | 0.0 | 0.0 |
| hsa-miR-6853-3p | 0.0 | 0.0 | 1.8 | 0.0 | 0.0 | 0.0 | 0.0 | 0.0 | 0.0 |
| hsa -miR -6854-5p | 0.0 | 2.1 | 0.0 | 0.0 | 0.0 | 0.0 | 0.0 | 0.0 | 0.0 |
| hsa -miR -6864-5p | 0.0 | 0.0 | 2.4 | 0.0 | 0.0 | 1.4 | 0.0 | 0.0 | 0.0 |
| hsa -miR -6866-5p | 0.0 | 0.0 | 0.0 | 0.0 | 0.0 | 0.0 | 0.0 | 0.0 | 0.0 |
| hsa-miR-6869-3p | 0.0 | 2.1 | 0.0 | 2.6 | 0.0 | 0.0 | 4.0 | 0.0 | 0.0 |
| hsa -miR -6882-5p | 0.0 | 2.0 | 0.0 | 0.0 | 2.3 | 1.5 | 0.0 | 0.0 | 0.0 |
| hsa -miR -6888-3p | 0.0 | 2.1 | 0.0 | 0.0 | 0.0 | 0.0 | 0.0 | 0.0 | 0.0 |
| hsa-miR-7153-5p | 0.0 | 2.1 | 0.0 | 0.0 | 0.0 | 0.0 | 0.0 | 0.0 | 0.0 |
| hsa-miR-7154-5p | 0.0 | 0.0 | 0.0 | 6.6 | 0.0 | 0.0 | 0.0 | 0.0 | 0.0 |
| hsa-miR-7156-3p | 0.0 | 3.3 | 2.8 | 5.1 | 0.0 | 1.4 | 4.7 | 0.0 | 0.0 |
| hsa-miR-7156-5p | 2.0 | 3.2 | 0.0 | 4.9 | 2.4 | 2.4 | 0.0 | 0.0 | 0.0 |
| hsa-miR-7158-3p | 0.0 | 2.2 | 0.0 | 0.0 | 0.0 | 0.0 | 0.0 | 0.0 | 0.0 |
| hsa-miR-7159-3p | 0.0 | 0.0 | 0.0 | 0.0 | 0.0 | 0.0 | 3.1 | 0.0 | 0.0 |
| hsa-miR-7160-3p | 0.0 | 0.0 | 0.0 | 2.4 | 0.0 | 2.5 | 0.0 | 0.0 | 0.0 |
| hsa-miR-7515 | 0.0 | 0.0 | 3.8 | 0.0 | 0.0 | 2.9 | 0.0 | 0.0 | 0.0 |
| hsa-miR-7705 | 0.0 | 0.0 | 1.6 | 0.0 | 0.0 | 0.0 | 2.9 | 0.0 | 0.0 |
| hsa-miR-7706 | 3.1 | 2.0 | 2.6 | 0.0 | 1.2 | 2.1 | 0.0 | 0.0 | 0.0 |
| hsa-miR-7978 | 2.2 | 2.0 | 1.9 | 0.0 | 0.0 | 1.8 | 0.0 | 0.0 | 0.0 |
| hsa-miR-8053 | 0.0 | 0.0 | 0.0 | 0.0 | 0.0 | 0.0 | 0.0 | 0.0 | 0.0 |
| hsa-miR-8054 | 0.0 | 2.3 | 0.0 | 0.0 | 0.0 | 0.0 | 0.0 | 0.0 | 0.0 |
| hsa-miR-8056 | 0.0 | 0.0 | 2.0 | 0.0 | 0.0 | 0.0 | 0.0 | 0.0 | 0.0 |
| hsa-miR-8058 | 3.3 | 1.8 | 2.1 | 4.8 | 0.0 | 0.0 | 0.0 | 0.0 | 0.0 |
| hsa-miR-8067 | 0.0 | 0.0 | 0.0 | 0.0 | 0.0 | 0.0 | 3.6 | 0.0 | 0.0 |
| hsa-miR-8068 | 0.0 | 0.0 | 0.0 | 0.0 | 0.0 | 1.4 | 0.0 | 0.0 | 0.0 |
| hsa-miR-8074 | 3.0 | 2.0 | 2.4 | 4.6 | 2.1 | 2.4 | 0.0 | 0.0 | 0.0 |
| hsa-miR-8079 | 0.0 | 2.6 | 0.0 | 0.0 | 0.0 | 1.8 | 0.0 | 2.4 | 0.0 |
| hsa-miR-8086 | 0.0 | 2.4 | 0.0 | 0.0 | 2.5 | 1.2 | 0.0 | 0.0 | 0.0 |
| hsa-miR-8088 | 0.0 | 0.0 | 3.2 | 0.0 | 1.3 | 0.0 | 0.0 | 0.0 | 0.0 |
| hsa -miR -4694-5p | 0.0 | 1.8 | 0.0 | 0.0 | 0.0 | 1.5 | 2.9 | 0.0 | 0.0 |
| hsa-miR-4746-5p | 0.0 | 0.0 | 0.0 | 0.0 | 0.0 | 0.0 | 0.0 | 0.0 | 0.0 |
| hsa-miR-0517-5p | 0.0 | 2.1 | 2.0 | 0.0 | 0.0 | 2.2 | 3.3 | 0.0 | 0.0 |
| hsa-miR-4302 | 0.0 | 0.0 | 0.0 | 0.0 | 0.0 | 0.0 | 0.0 | 0.0 | 0.0 |
| hsa-miR-3908 | 0.0 | 2.2 | 0.0 | 0.0 | 2.2 | 0.0 | 0.0 | 2.4 | 0.0 |
| hsa-miR-0611 | 0.0 | 1.9 | 0.0 | 0.0 | 1.7 | 0.0 | 3.3 | 0.0 | 0.0 |
| hsa-miR-0873-5p | 0.0 | 0.0 | 0.0 | 2.9 | 0.0 | 0.0 | 0.0 | 0.0 | 0.0 |
| hsa-miR-4423-5p | 0.0 | 3.2 | 1.6 | 0.0 | 2.7 | 3.2 | 0.0 | 0.0 | 0.0 |
| hsa-miR-0383-5p | 0.0 | 1.8 | 0.0 | 0.0 | 0.0 | 0.0 | 3.3 | 0.0 | 0.0 |
| hsa-miR-6500-5p | 1.7 | 3.1 | 0.0 | 0.0 | 2.2 | 2.1 | 3.3 | 2.4 | 0.0 |
| hsa-miR-4454 | 1.8 | 2.1 | 2.2 | 3.3 | 3.3 | 2.6 | 3.6 | 0.0 | 0.0 |
| hsa-miR-0203b-3p | 1.8 | 0.0 | 0.0 | 3.2 | 0.0 | 0.0 | 0.0 | 0.0 | 0.0 |
| hsa-miR-4711-5p | 0.0 | 0.0 | 0.0 | 0.0 | 0.0 | 0.0 | 0.0 | 0.0 | 0.0 |
| hsa-miR-0664a-5p | 0.0 | 2.2 | 0.0 | 0.0 | 2.0 | 1.2 | 2.8 | 0.0 | 0.0 |
| hsa -miR -0548v | 0.0 | 2.3 | 0.0 | 0.0 | 0.0 | 0.0 | 0.0 | 0.0 | 2.8 |
| hsa-miR-3916 | 0.0 | 0.0 | 0.0 | 5.6 | 0.0 | 0.0 | 0.0 | 0.0 | 0.0 |
| hsa-miR-4691-3p | 0.0 | 2.6 | 0.0 | 0.0 | 3.2 | 1.2 | 0.0 | 0.0 | 0.0 |
| hsa-miR-0548z, hsa-miR-548h-3p | 0.0 | 0.0 | 0.0 | 6.5 | 0.0 | 0.0 | 3.3 | 0.0 | 0.0 |
| hsa-miR-0027b-5p | 0.0 | 0.0 | 0.0 | 0.0 | 0.0 | 0.0 | 0.0 | 0.0 | 0.0 |
| hsa-miR-2681-5p | 2.4 | 2.2 | 2.8 | 0.0 | 2.3 | 1.7 | 2.6 | 0.0 | 0.0 |
| hsa-miR-3161 | 2.4 | 1.9 | 3.0 | 0.0 | 2.3 | 1.7 | 3.2 | 3.1 | 0.0 |
| hsa-miR-0604 | 1.6 | 3.3 | 0.0 | 0.0 | 2.8 | 2.4 | 0.0 | 2.5 | 0.0 |
| hsa-miR-1269b | 0.0 | 2.1 | 0.0 | 4.5 | 0.0 | 0.0 | 0.0 | 0.0 | 0.0 |
| hsa-miR-4659b-3p | 0.0 | 2.4 | 0.0 | 0.0 | 0.0 | 0.0 | 0.0 | 0.0 | 0.0 |
| hsa-miR-0938 | 2.2 | 2.1 | 0.0 | 0.0 | 2.5 | 1.7 | 3.5 | 0.0 | 0.0 |
| hsa-miR-4733-3p | 2.3 | 2.3 | 0.0 | 0.0 | 2.0 | 3.1 | 3.7 | 2.7 | 0.0 |
| hsa-miR-0491-3p | 0.0 | 0.0 | 0.0 | 0.0 | 0.0 | 1.5 | 3.2 | 0.0 | 0.0 |
| hsa-miR-6505-5p | 0.0 | 0.0 | 1.8 | 0.0 | 1.3 | 0.0 | 3.0 | 0.0 | 0.0 |
| hsa-miR-0630 | 0.0 | 0.0 | 0.0 | 5.5 | 0.0 | 1.6 | 0.0 | 0.0 | 0.0 |
| hsa -miR -3688-3p | 0.0 | 0.0 | 0.0 | 0.0 | 0.0 | 0.0 | 0.0 | 0.0 | 0.0 |
| hsa-miR-3179 | 0.0 | 0.0 | 0.0 | 0.0 | 0.0 | 0.0 | 0.0 | 0.0 | 3.0 |
| hsa-miR-4755-3p | 0.0 | 0.0 | 0.0 | 0.0 | 1.6 | 0.0 | 0.0 | 0.0 | 0.0 |
| hsa-miR-6720-5p | 1.7 | 3.3 | 3.2 | 2.9 | 0.0 | 2.7 | 0.0 | 0.0 | 0.0 |
| hsa -miR -0452-3p | 3.0 | 0.0 | 4.0 | 3.5 | 0.0 | 0.0 | 0.0 | 0.0 | 0.0 |
| hsa-miR-0374b-3p | 0.0 | 0.0 | 0.0 | 6.0 | 0.0 | 0.0 | 0.0 | 0.0 | 0.0 |
| hsa -miR -0022-3p | 0.0 | 0.0 | 0.0 | 0.0 | 0.0 | 0.0 | 0.0 | 0.0 | 0.0 |
| hsa -miR -0222-3p | 1.6 | 0.0 | 0.0 | 0.0 | 1.9 | 0.0 | 0.0 | 0.0 | 0.0 |
| hsa-miR-0093-3p | 0.0 | 0.0 | 3.1 | 0.0 | 2.6 | 0.0 | 0.0 | 0.0 | 0.0 |
| hsa-miR-0200a-5p | 0.0 | 0.0 | 0.0 | 0.0 | 1.2 | 2.5 | 0.0 | 0.0 | 0.0 |
| hsa -miR -0362-3p | 0.0 | 0.0 | 0.0 | 0.0 | 2.1 | 1.9 | 0.0 | 2.3 | 0.0 |
| hsa-miR-0144-5p | 0.0 | 0.0 | 0.0 | 0.0 | 0.0 | 0.0 | 0.0 | 0.0 | 0.0 |
| hsa -miR -0628-3p | 0.0 | 0.0 | 0.0 | 0.0 | 1.6 | 1.7 | 0.0 | 0.0 | 0.0 |
| hsa-miR-0378c | 3.0 | 2.0 | 3.8 | 0.0 | 2.5 | 2.9 | 2.9 | 0.0 | 0.0 |
| hsa-miR-0335-5p | 0.0 | 0.0 | 0.0 | 0.0 | 0.0 | 0.0 | 0.0 | 0.0 | 0.0 |
| hsa-miR-7112-3p | 2.2 | 3.7 | 4.0 | 5.9 | 2.1 | 2.9 | 4.0 | 0.0 | 0.0 |
| hsa-miR-5009-3p | 0.0 | 0.0 | 0.0 | 0.0 | 0.0 | 0.0 | 0.0 | 0.0 | 0.0 |
| hsa-let-7g-5p | 0.0 | 0.0 | 0.0 | 0.0 | 0.0 | 0.0 | 0.0 | 0.0 | 0.0 |
| hsa-miR-0130a-5p | 2.2 | 3.1 | 4.2 | 3.3 | 0.0 | 2.2 | 0.0 | 0.0 | 3.2 |
| hsa-miR-0215-5p | 0.0 | 0.0 | 0.0 | 0.0 | 0.0 | 0.0 | 0.0 | 0.0 | 0.0 |
| hsa -miR -3186-3p | 3.9 | 3.1 | 3.1 | 0.0 | 4.5 | 5.5 | 3.6 | 0.0 | 0.0 |
| hsa-miR-4745-3p | 0.0 | 3.6 | 2.6 | 3.7 | 2.2 | 2.6 | 4.8 | 2.8 | 3.7 |
| hsa-miR-0449a | 0.0 | 3.0 | 0.0 | 2.5 | 2.2 | 3.0 | 0.0 | 0.0 | 0.0 |
| hsa-miR-0017-3p | 0.0 | 0.0 | 0.0 | 2.6 | 0.0 | 0.0 | 0.0 | 0.0 | 0.0 |
| hsa-miR-4786-5p | 2.7 | 3.5 | 3.0 | 0.0 | 3.0 | 3.6 | 3.6 | 3.3 | 0.0 |
| hsa-miR-5695 | 0.0 | 2.6 | 0.0 | 0.0 | 1.9 | 0.0 | 2.8 | 0.0 | 0.0 |
| hsa-miR-3927-3p | 0.0 | 0.0 | 0.0 | 0.0 | 0.0 | 0.0 | 2.7 | 0.0 | 0.0 |
| hsa-miR-0200b-5p | 0.0 | 3.1 | 0.0 | 0.0 | 3.3 | 2.6 | 0.0 | 0.0 | 0.0 |
| hsa-miR-0010a-5p | 0.0 | 0.0 | 0.0 | 5.7 | 2.5 | 0.0 | 3.5 | 0.0 | 0.0 |
| hsa-miR-0181d-3p | 0.0 | 2.3 | 3.3 | 0.0 | 2.6 | 2.4 | 0.0 | 0.0 | 0.0 |
| hsa-miR-0369-3p | 0.0 | 2.9 | 0.0 | 0.0 | 3.1 | 0.0 | 0.0 | 0.0 | 0.0 |
| hsa-miR-0578 | 0.0 | 3.1 | 0.0 | 0.0 | 2.9 | 3.7 | 0.0 | 0.0 | 2.8 |
| hsa-miR-3193 | 1.7 | 0.0 | 0.0 | 0.0 | 2.1 | 0.0 | 0.0 | 0.0 | 0.0 |
| hsa-miR-0302a-3p | 0.0 | 0.0 | 0.0 | 0.0 | 0.0 | 0.0 | 0.0 | 0.0 | 0.0 |
| hsa-miR-0549a | 0.0 | 0.0 | 0.0 | 0.0 | 0.0 | 0.0 | 0.0 | 0.0 | 0.0 |
| hsa-miR-1255b-2-3p | 0.0 | 3.3 | 0.0 | 4.9 | 0.0 | 1.4 | 0.0 | 0.0 | 0.0 |
| hsa-miR-6510-3p | 2.9 | 2.9 | 4.0 | 0.0 | 2.0 | 2.9 | 3.6 | 0.0 | 0.0 |
| hsa-miR-0363-5p | 0.0 | 2.4 | 0.0 | 0.0 | 1.6 | 1.9 | 0.0 | 0.0 | 3.4 |
| hsa -miR -0028-5p | 3.8 | 0.0 | 0.0 | 0.0 | 1.8 | 2.0 | 0.0 | 0.0 | 0.0 |
| hsa-miR-4720-5p | 0.0 | 1.8 | 0.0 | 0.0 | 0.0 | 0.0 | 3.2 | 0.0 | 0.0 |
| hsa -miR -0202-3p | 0.0 | 0.0 | 0.0 | 0.0 | 0.0 | 1.6 | 0.0 | 3.6 | 0.0 |
| hsa-miR-4255 | 1.8 | 2.3 | 0.0 | 0.0 | 2.1 | 2.8 | 0.0 | 0.0 | 0.0 |
| hsa -miR -0449c-5p | 1.9 | 3.3 | 3.6 | 0.0 | 2.9 | 2.8 | 0.0 | 2.8 | 0.0 |
| hsa-miR-0181b-3p | 0.0 | 2.4 | 0.0 | 0.0 | 0.0 | 0.0 | 0.0 | 0.0 | 0.0 |
| hsa-miR-4775 | 2.4 | 2.6 | 0.0 | 0.0 | 0.0 | 2.6 | 3.5 | 0.0 | 0.0 |
| hsa-miR-0378h | 0.0 | 3.4 | 2.5 | 0.0 | 2.3 | 1.8 | 3.1 | 2.5 | 2.9 |
| hsa-miR-0152-5p | 0.0 | 2.4 | 0.0 | 0.0 | 2.7 | 1.9 | 3.1 | 0.0 | 0.0 |
| hsa-miR-0513b-5p | 0.0 | 0.0 | 1.9 | 0.0 | 3.4 | 0.0 | 0.0 | 0.0 | 0.0 |
| hsa-miR-0029b-3p | 0.0 | 0.0 | 0.0 | 3.8 | 1.9 | 1.9 | 2.8 | 3.1 | 0.0 |
| hsa-miR-1297 | 0.0 | 0.0 | 1.9 | 0.0 | 0.0 | 2.2 | 0.0 | 0.0 | 2.7 |
| hsa -miR -0020b-3p | 0.0 | 1.8 | 1.8 | 2.8 | 1.3 | 1.8 | 0.0 | 0.0 | 0.0 |
| hsa-miR-0541-3p | 1.7 | 2.4 | 0.0 | 0.0 | 2.1 | 2.4 | 0.0 | 0.0 | 0.0 |
| hsa-miR-1301-3p | 2.5 | 0.0 | 0.0 | 0.0 | 2.8 | 2.2 | 3.1 | 0.0 | 0.0 |
| hsa-miR-3115 | 0.0 | 0.0 | 0.0 | 0.0 | 2.8 | 2.2 | 0.0 | 0.0 | 0.0 |
| hsa-miR-6807-3p | 0.0 | 0.0 | 0.0 | 0.0 | 0.0 | 0.0 | 3.1 | 0.0 | 0.0 |
| hsa -miR -0532-5p | 0.0 | 0.0 | 0.0 | 0.0 | 0.0 | 0.0 | 3.1 | 0.0 | 0.0 |
| hsa -miR -6864-3p | 3.0 | 2.8 | 3.1 | 2.3 | 2.1 | 0.0 | 0.0 | 0.0 | 2.6 |
| hsa-miR-0196a-5p | 0.0 | 0.0 | 2.5 | 0.0 | 3.1 | 1.8 | 0.0 | 0.0 | 0.0 |
| hsa-miR-4676-3p | 2.9 | 2.2 | 2.4 | 0.0 | 2.4 | 2.0 | 0.0 | 2.4 | 0.0 |
| hsa -miR -0096-3p | 0.0 | 0.0 | 0.0 | 0.0 | 0.0 | 0.0 | 0.0 | 0.0 | 0.0 |
| hsa-miR-0126-3p | 0.0 | 1.8 | 0.0 | 0.0 | 1.2 | 0.0 | 0.0 | 0.0 | 0.0 |
| hsa-miR-0550a-3-5p | 3.9 | 3.8 | 4.8 | 0.0 | 4.2 | 4.3 | 2.8 | 2.8 | 0.0 |
| hsa-miR-0378f | 0.0 | 2.2 | 0.0 | 0.0 | 2.4 | 2.4 | 0.0 | 0.0 | 0.0 |
| hsa-miR-5581-5p | 3.1 | 0.0 | 2.7 | 0.0 | 2.1 | 2.5 | 3.8 | 0.0 | 0.0 |
| hsa-miR-0627-5p | 0.0 | 0.0 | 4.1 | 0.0 | 4.7 | 0.0 | 0.0 | 0.0 | 0.0 |
| hsa-miR-0649 | 0.0 | 0.0 | 0.0 | 0.0 | 2.7 | 0.0 | 3.9 | 0.0 | 0.0 |
| hsa-miR-0626 | 0.0 | 0.0 | 0.0 | 0.0 | 0.0 | 0.0 | 3.9 | 0.0 | 0.0 |
| hsa-miR-5007-5p | 1.9 | 3.6 | 0.0 | 0.0 | 2.9 | 3.2 | 4.0 | 2.4 | 0.0 |
| hsa-miR-7107-3p | 2.0 | 2.7 | 0.0 | 3.0 | 2.5 | 3.2 | 2.5 | 0.0 | 0.0 |
| hsa -miR -0030e-3p | 2.0 | 0.0 | 2.1 | 5.8 | 1.5 | 0.0 | 0.0 | 0.0 | 0.0 |
| hsa-miR-5589-3p | 1.6 | 2.1 | 0.0 | 0.0 | 0.0 | 0.0 | 3.1 | 0.0 | 0.0 |
| hsa-miR-0128-3p | 0.0 | 0.0 | 0.0 | 0.0 | 0.0 | 0.0 | 0.0 | 0.0 | 0.0 |
| hsa-miR-0421 | 0.0 | 0.0 | 0.0 | 0.0 | 1.5 | 0.0 | 2.9 | 0.0 | 0.0 |
| hsa-miR-7856-5p | 1.8 | 3.4 | 3.1 | 0.0 | 3.1 | 1.7 | 3.2 | 0.0 | 0.0 |
| hsa-miR-0378e | 2.4 | 2.8 | 0.0 | 0.0 | 1.6 | 2.6 | 0.0 | 0.0 | 3.0 |
| hsa-miR-1205 | 0.0 | 0.0 | 0.0 | 0.0 | 1.6 | 2.3 | 0.0 | 0.0 | 0.0 |
| hsa-miR-0579-3p | 0.0 | 1.8 | 2.8 | 0.0 | 0.0 | 1.4 | 0.0 | 2.3 | 2.7 |
| hsa-miR-6839-3p | 0.0 | 1.8 | 0.0 | 0.0 | 0.0 | 0.0 | 0.0 | 0.0 | 0.0 |
| hsa-miR-0219b-3p | 0.0 | 0.0 | 0.0 | 0.0 | 0.0 | 0.0 | 0.0 | 0.0 | 0.0 |
| hsa-miR-0662 | 0.0 | 0.0 | 0.0 | 0.0 | 0.0 | 0.0 | 0.0 | 0.0 | 0.0 |
| hsa-miR-7153-3p | 2.1 | 2.4 | 2.6 | 0.0 | 0.0 | 0.0 | 0.0 | 0.0 | 0.0 |
| hsa-miR-4742-3p | 0.0 | 2.5 | 0.0 | 2.7 | 0.0 | 2.3 | 0.0 | 0.0 | 0.0 |
| hsa-miR-1303 | 1.8 | 3.9 | 5.1 | 5.4 | 4.5 | 4.4 | 0.0 | 3.8 | 2.7 |
| hsa-miR-3117-5p | 0.0 | 0.0 | 0.0 | 5.4 | 4.5 | 4.4 | 0.0 | 0.0 | 0.0 |
| hsa-miR-0576-5p | 0.0 | 2.5 | 0.0 | 2.4 | 3.2 | 1.9 | 0.0 | 0.0 | 0.0 |
| hsa -miR -0372-3p | 3.4 | 3.7 | 3.6 | 0.0 | 2.4 | 2.7 | 0.0 | 0.0 | 0.0 |
| hsa-let-7e-5p | 2.8 | 0.0 | 3.4 | 0.0 | 0.0 | 2.2 | 0.0 | 0.0 | 0.0 |
| hsa-miR-3677-5p | 0.0 | 0.0 | 0.0 | 2.4 | 0.0 | 0.0 | 2.7 | 0.0 | 0.0 |
| hsa-miR-0092a-1-5p | 0.0 | 0.0 | 3.6 | 0.0 | 0.0 | 0.0 | 0.0 | 0.0 | 0.0 |
| hsa-miR-0029a-3p | 0.0 | 0.0 | 0.0 | 0.0 | 2.1 | 0.0 | 0.0 | 0.0 | 0.0 |
| hsa-miR-0007-1-3p | 1.7 | 2.7 | 0.0 | 0.0 | 0.0 | 2.5 | 2.9 | 0.0 | 0.0 |
| hsa-miR-0450a-5p | 0.0 | 2.3 | 0.0 | 0.0 | 3.1 | 0.0 | 0.0 | 0.0 | 0.0 |
| hsa-miR-4330 | 2.2 | 3.4 | 0.0 | 0.0 | 2.4 | 2.7 | 3.6 | 3.0 | 0.0 |
| hsa-miR-0515-3p | 0.0 | 3.0 | 3.3 | 0.0 | 2.8 | 2.8 | 3.0 | 2.8 | 0.0 |
| hsa-miR-0323a-3p | 0.0 | 0.0 | 0.0 | 7.1 | 0.0 | 0.0 | 3.4 | 0.0 | 0.0 |
| hsa-miR-7159-5p | 0.0 | 3.3 | 0.0 | 2.6 | 1.8 | 3.0 | 3.4 | 0.0 | 2.8 |
| hsa-miR-4766-5p | 0.0 | 2.4 | 0.0 | 0.0 | 0.0 | 0.0 | 0.0 | 0.0 | 0.0 |
| hsa-miR-4524a-5p | 0.0 | 2.1 | 0.0 | 0.0 | 2.1 | 2.5 | 0.0 | 0.0 | 0.0 |
| hsa-miR-7843-3p | 0.0 | 2.3 | 0.0 | 2.7 | 0.0 | 2.3 | 3.0 | 0.0 | 0.0 |
| hsa-miR-6809-5p | 0.0 | 2.4 | 0.0 | 0.0 | 1.7 | 0.0 | 0.0 | 0.0 | 0.0 |
| hsa -miR -6888-5p | 2.4 | 3.1 | 0.0 | 0.0 | 2.2 | 2.9 | 2.6 | 2.9 | 0.0 |
| hsa-miR-2909 | 3.5 | 2.5 | 3.3 | 0.0 | 1.6 | 3.0 | 4.0 | 0.0 | 3.1 |
| hsa-miR-3164 | 3.5 | 3.0 | 2.9 | 0.0 | 1.6 | 3.0 | 0.0 | 0.0 | 2.6 |
| hsa-miR-4520-3p | 0.0 | 1.9 | 0.0 | 0.0 | 6.1 | 0.0 | 2.6 | 2.3 | 3.3 |
| hsa-miR-0510-5p | 0.0 | 2.6 | 2.6 | 0.0 | 2.8 | 5.2 | 3.7 | 0.0 | 3.5 |
| hsa-miR-4690-3p | 0.0 | 2.5 | 3.3 | 0.0 | 0.0 | 1.8 | 0.0 | 0.0 | 0.0 |
| hsa-miR-0026a-1-3p | 2.0 | 0.0 | 0.0 | 0.0 | 1.7 | 0.0 | 2.9 | 0.0 | 0.0 |
| hsa-miR-4793-5p | 0.0 | 0.0 | 0.0 | 0.0 | 3.8 | 1.8 | 3.1 | 0.0 | 0.0 |
| hsa -miR -0624-5p | 0.0 | 0.0 | 0.0 | 0.0 | 0.0 | 2.3 | 0.0 | 0.0 | 0.0 |
| hsa-miR-0617 | 0.0 | 0.0 | 0.0 | 0.0 | 0.0 | 2.7 | 0.0 | 0.0 | 0.0 |
| hsa-miR-0371a-3p | 2.4 | 2.8 | 2.2 | 0.0 | 3.2 | 2.6 | 0.0 | 2.8 | 3.2 |
| hsa-miR-0500a-5p | 2.5 | 2.6 | 0.0 | 0.0 | 0.0 | 3.1 | 0.0 | 0.0 | 0.0 |
| hsa-miR-3687 | 0.0 | 3.0 | 0.0 | 0.0 | 1.8 | 2.5 | 2.7 | 0.0 | 0.0 |
| hsa-miR-0378i | 3.0 | 1.8 | 0.0 | 0.0 | 0.0 | 2.7 | 0.0 | 0.0 | 3.1 |
| hsa -miR -3934-3p | 1.9 | 3.0 | 0.0 | 0.0 | 2.9 | 2.8 | 0.0 | 0.0 | 0.0 |
| hsa-miR-5688 | 1.7 | 3.2 | 0.0 | 4.3 | 0.0 | 0.0 | 0.0 | 0.0 | 0.0 |
| hsa-miR-6768-3p | 2.8 | 3.4 | 0.0 | 0.0 | 1.4 | 2.2 | 3.0 | 2.2 | 0.0 |
| hsa-miR-0010b-5p | 0.0 | 2.4 | 0.0 | 0.0 | 0.0 | 1.3 | 2.8 | 0.0 | 0.0 |
| hsa-miR-0023a-3p | 0.0 | 2.1 | 0.0 | 0.0 | 0.0 | 0.0 | 0.0 | 0.0 | 0.0 |
| hsa-miR-0373-3p | 0.0 | 0.0 | 2.2 | 6.1 | 0.0 | 0.0 | 0.0 | 0.0 | 0.0 |
| hsa-miR-0487b-3p | 0.0 | 2.8 | 0.0 | 0.0 | 1.4 | 1.4 | 0.0 | 0.0 | 2.6 |
| hsa-miR-4661-5p | 0.0 | 0.0 | 0.0 | 0.0 | 3.0 | 1.9 | 3.7 | 2.7 | 3.3 |
| hsa-miR-0767-5p | 2.8 | 3.4 | 0.0 | 4.4 | 0.0 | 2.4 | 0.0 | 0.0 | 0.0 |
| hsa-miR-0922 | 0.0 | 0.0 | 0.0 | 0.0 | 0.0 | 0.0 | 0.0 | 0.0 | 0.0 |
| hsa-miR-4677-5p | 0.0 | 2.2 | 0.0 | 0.0 | 0.0 | 0.0 | 3.5 | 0.0 | 0.0 |
| hsa -miR -0502-3p | 0.0 | 0.0 | 0.0 | 0.0 | 2.6 | 0.0 | 0.0 | 0.0 | 0.0 |
| hsa -miR -0598-5p | 2.3 | 0.0 | 2.8 | 0.0 | 2.4 | 3.4 | 3.6 | 2.2 | 0.0 |
| hsa-miR-4521 | 0.0 | 0.0 | 0.0 | 3.0 | 4.3 | 3.4 | 0.0 | 2.7 | 3.1 |
| hsa-miR-0215-3p | 0.0 | 1.8 | 0.0 | 0.0 | 0.0 | 0.0 | 2.7 | 0.0 | 0.0 |
| hsa-miR-4261 | 2.9 | 0.0 | 3.0 | 0.0 | 2.0 | 1.9 | 0.0 | 0.0 | 0.0 |
| hsa-miR-4280 | 0.0 | 1.9 | 0.0 | 0.0 | 3.0 | 1.3 | 3.2 | 0.0 | 0.0 |
| hsa-miR-0027a-3p | 0.0 | 0.0 | 0.0 | 2.4 | 0.0 | 0.0 | 0.0 | 0.0 | 0.0 |
| hsa-miR-6073 | 0.0 | 0.0 | 0.0 | 0.0 | 1.3 | 0.0 | 2.7 | 0.0 | 0.0 |
| hsa-miR-0029b-2-5p | 0.0 | 0.0 | 0.0 | 0.0 | 2.3 | 0.0 | 0.0 | 0.0 | 0.0 |
| hsa-miR-4794 | 3.3 | 3.5 | 3.3 | 5.6 | 2.6 | 3.3 | 3.2 | 0.0 | 3.5 |
| hsa-miR-4645-5p | 0.0 | 0.0 | 0.0 | 0.0 | 0.0 | 0.0 | 2.7 | 0.0 | 0.0 |
| hsa-miR-3529-3p | 0.0 | 0.0 | 0.0 | 0.0 | 0.0 | 0.0 | 3.3 | 0.0 | 0.0 |
| hsa-miR-4325 | 0.0 | 0.0 | 0.0 | 6.4 | 0.0 | 0.0 | 0.0 | 0.0 | 0.0 |
| hsa-miR-5702 | 3.0 | 2.5 | 1.7 | 5.7 | 2.9 | 2.0 | 0.0 | 0.0 | 0.0 |
| hsa-miR-4262 | 2.0 | 3.1 | 3.0 | 0.0 | 3.1 | 2.9 | 0.0 | 2.8 | 3.6 |
| hsa-miR-0891a-3p | 1.6 | 2.7 | 0.0 | 3.3 | 0.0 | 2.0 | 3.4 | 0.0 | 0.0 |
| hsa-miR-0205-5p | 2.0 | 3.1 | 2.1 | 3.1 | 2.7 | 2.5 | 0.0 | 2.6 | 0.0 |
| hsa-miR-8078 | 2.7 | 3.3 | 2.7 | 0.0 | 3.9 | 3.9 | 2.8 | 3.0 | 3.1 |
| hsa-miR-0181a-5p | 0.0 | 2.0 | 0.0 | 0.0 | 0.0 | 2.1 | 2.6 | 0.0 | 0.0 |
| hsa-miR-4314 | 0.0 | 2.5 | 1.7 | 0.0 | 2.4 | 2.1 | 2.9 | 0.0 | 3.1 |
| hsa-miR-4633-5p | 2.6 | 3.5 | 3.1 | 0.0 | 2.8 | 3.2 | 2.9 | 0.0 | 0.0 |
| hsa -miR -0502-5p | 0.0 | 0.0 | 0.0 | 0.0 | 2.0 | 0.0 | 0.0 | 0.0 | 0.0 |
| hsa -miR -0494-3p | 2.0 | 2.2 | 2.5 | 2.8 | 3.8 | 3.0 | 3.0 | 0.0 | 3.7 |
| hsa-miR-4317 | 0.0 | 0.0 | 0.0 | 0.0 | 0.0 | 1.4 | 0.0 | 0.0 | 0.0 |
| hsa-miR-5003-3p | 0.0 | 0.0 | 0.0 | 6.0 | 0.0 | 0.0 | 0.0 | 0.0 | 0.0 |
| hsa-miR-0203a-3p | 3.3 | 2.3 | 3.2 | 0.0 | 2.9 | 2.8 | 0.0 | 0.0 | 3.2 |
| hsa-miR-5693 | 2.3 | 0.0 | 0.0 | 0.0 | 2.6 | 2.6 | 3.0 | 0.0 | 0.0 |
| hsa-miR-0151b | 0.0 | 0.0 | 1.8 | 0.0 | 0.0 | 2.2 | 0.0 | 0.0 | 0.0 |
| hsa-miR-0135b-5p | 3.1 | 2.9 | 3.7 | 0.0 | 3.3 | 2.1 | 2.5 | 0.0 | 0.0 |
| hsa-miR-0614 | 0.0 | 3.7 | 4.1 | 3.7 | 3.5 | 3.3 | 4.2 | 3.1 | 0.0 |
| hsa-miR-5690 | 0.0 | 2.4 | 0.0 | 0.0 | 0.0 | 2.3 | 2.9 | 0.0 | 4.0 |
| hsa-miR-2467-5p | 0.0 | 0.0 | 0.0 | 0.0 | 0.0 | 0.0 | 0.0 | 0.0 | 0.0 |
| hsa-miR-3160-3p | 5.3 | 5.1 | 5.4 | 0.0 | 0.0 | 0.0 | 5.3 | 3.9 | 4.7 |
| hsa-miR-5011-3p | 0.0 | 0.0 | 0.0 | 0.0 | 0.0 | 0.0 | 0.0 | 0.0 | 0.0 |
| hsa-miR-0219b-5p | 0.0 | 2.7 | 5.0 | 4.8 | 1.4 | 2.2 | 3.0 | 2.6 | 0.0 |
| hsa-miR-4318 | 0.0 | 2.1 | 0.0 | 2.6 | 2.3 | 0.0 | 2.9 | 0.0 | 3.0 |
| hsa-miR-0593-5p | 0.0 | 2.1 | 0.0 | 0.0 | 3.1 | 2.2 | 3.2 | 0.0 | 0.0 |
| hsa-miR-0181b-5p | 0.0 | 3.2 | 0.0 | 0.0 | 2.2 | 3.0 | 0.0 | 0.0 | 3.0 |
| hsa-miR-0195-5p | 0.0 | 0.0 | 0.0 | 0.0 | 3.0 | 0.0 | 4.1 | 0.0 | 0.0 |
| hsa-miR-5003-5p | 0.0 | 0.0 | 0.0 | 0.0 | 0.0 | 0.0 | 0.0 | 0.0 | 0.0 |
| hsa-miR-6068 | 3.1 | 4.0 | 3.7 | 5.2 | 4.7 | 5.1 | 3.3 | 3.5 | 0.0 |
| hsa-miR-2682-5p | 0.0 | 3.0 | 2.0 | 2.4 | 3.3 | 1.8 | 2.8 | 0.0 | 0.0 |
| hsa-miR-3162-5p | 5.5 | 6.3 | 5.5 | 2.4 | 3.3 | 1.8 | 5.4 | 5.9 | 5.5 |
| hsa-miR-4494 | 1.8 | 2.3 | 0.0 | 0.0 | 1.9 | 3.2 | 0.0 | 0.0 | 3.5 |
| hsa-miR-0635 | 2.3 | 2.6 | 0.0 | 0.0 | 3.1 | 3.5 | 3.8 | 0.0 | 3.0 |
| hsa-miR-3677-3p | 0.0 | 2.1 | 3.1 | 0.0 | 2.2 | 2.5 | 0.0 | 2.5 | 0.0 |
| hsa -miR -4694-3p | 3.4 | 3.0 | 3.0 | 3.6 | 1.2 | 3.3 | 0.0 | 0.0 | 0.0 |
| hsa-miR-0676-3p | 0.0 | 0.0 | 0.0 | 0.0 | 0.0 | 1.6 | 0.0 | 0.0 | 0.0 |
| hsa-miR-0509-3-5p | 0.0 | 0.0 | 0.0 | 0.0 | 1.6 | 0.0 | 2.5 | 0.0 | 0.0 |
| hsa -miR -6832-5p | 3.3 | 3.2 | 2.9 | 0.0 | 4.0 | 3.3 | 0.0 | 0.0 | 2.8 |
| hsa -miR -3692-3p | 0.0 | 0.0 | 0.0 | 0.0 | 1.7 | 0.0 | 0.0 | 0.0 | 0.0 |
| hsa-miR-4672 | 2.3 | 2.8 | 0.0 | 0.0 | 2.5 | 2.8 | 2.7 | 0.0 | 0.0 |
| hsa-miR-0449b-5p | 0.0 | 3.4 | 2.1 | 6.8 | 0.0 | 2.2 | 0.0 | 0.0 | 0.0 |
| hsa-miR-4712-3p | 0.0 | 0.0 | 0.0 | 0.0 | 2.1 | 3.1 | 0.0 | 0.0 | 0.0 |
| hsa-miR-3622b-3p | 3.3 | 3.1 | 0.0 | 0.0 | 3.3 | 2.2 | 3.6 | 0.0 | 3.5 |
| hsa-miR-0489-5p | 2.5 | 3.3 | 0.0 | 0.0 | 3.3 | 2.8 | 0.0 | 0.0 | 0.0 |
| hsa-miR-0374b-5p | 2.8 | 0.0 | 0.0 | 0.0 | 0.0 | 0.0 | 0.0 | 0.0 | 0.0 |
| hsa-miR-4256 | 0.0 | 0.0 | 0.0 | 0.0 | 0.0 | 0.0 | 3.5 | 0.0 | 0.0 |
| hsa-miR-0411-3p | 2.0 | 2.2 | 0.0 | 0.0 | 2.9 | 3.0 | 2.5 | 0.0 | 0.0 |
| hsa-miR-2277-3p | 3.7 | 3.6 | 4.2 | 0.0 | 3.6 | 3.6 | 4.6 | 3.2 | 2.7 |
| hsa -miR -3156-3p | 5.1 | 5.6 | 4.5 | 0.0 | 3.6 | 3.6 | 4.9 | 4.2 | 4.5 |
| hsa-miR-6513-5p | 0.0 | 2.5 | 0.0 | 0.0 | 2.5 | 1.8 | 0.0 | 2.3 | 0.0 |
| hsa-miR-0023b-3p | 0.0 | 3.0 | 0.0 | 0.0 | 1.7 | 2.1 | 4.3 | 0.0 | 0.0 |
| hsa-miR-0194-5p | 2.0 | 2.5 | 0.0 | 0.0 | 1.3 | 1.5 | 0.0 | 2.2 | 3.3 |
| hsa-miR-0206 | 0.0 | 3.1 | 2.1 | 0.0 | 0.0 | 1.9 | 2.8 | 0.0 | 0.0 |
| hsa-miR-7157-3p | 1.9 | 2.3 | 0.0 | 0.0 | 2.0 | 2.0 | 0.0 | 0.0 | 0.0 |
| hsa-miR-6717-5p | 3.2 | 3.7 | 2.0 | 0.0 | 3.1 | 4.0 | 0.0 | 2.2 | 0.0 |
| hsa-miR-4295 | 0.0 | 0.0 | 3.1 | 3.6 | 1.2 | 1.9 | 3.2 | 0.0 | 0.0 |
| hsa-miR-1912 | 1.8 | 2.7 | 0.0 | 0.0 | 2.9 | 0.0 | 3.7 | 0.0 | 3.1 |
| hsa-miR-3141 | 8.1 | 8.1 | 7.9 | 0.0 | 2.9 | 0.0 | 7.0 | 7.6 | 7.0 |
| hsa -miR -0382-3p | 0.0 | 2.3 | 0.0 | 0.0 | 0.0 | 1.5 | 0.0 | 0.0 | 0.0 |
| hsa -miR -0499b-3p | 2.2 | 2.5 | 0.0 | 0.0 | 0.0 | 0.0 | 0.0 | 0.0 | 0.0 |
| hsa -miR -0098-5p | 0.0 | 0.0 | 0.0 | 0.0 | 0.0 | 0.0 | 0.0 | 3.1 | 0.0 |
| hsa-miR-0378a-5p | 4.2 | 3.3 | 3.5 | 4.4 | 0.0 | 0.0 | 4.4 | 0.0 | 2.6 |
| hsa-miR-4253 | 4.0 | 5.1 | 5.1 | 2.5 | 5.2 | 5.8 | 3.3 | 5.1 | 4.0 |
| hsa-miR-0550b-2-5p | 2.4 | 2.9 | 0.0 | 2.4 | 3.8 | 2.7 | 0.0 | 0.0 | 0.0 |
| hsa-miR-0025-5p | 2.4 | 3.0 | 3.3 | 3.4 | 3.4 | 2.5 | 0.0 | 2.3 | 0.0 |
| hsa-miR-4796-5p | 3.1 | 4.3 | 0.0 | 6.4 | 2.6 | 3.5 | 3.7 | 2.9 | 3.2 |
| hsa-miR-0015b-5p | 2.6 | 3.8 | 2.7 | 0.0 | 1.7 | 2.5 | 3.2 | 2.7 | 0.0 |
| hsa-miR-0378g | 2.1 | 3.2 | 2.8 | 0.0 | 0.0 | 2.3 | 3.2 | 0.0 | 0.0 |
| hsa-miR-5704 | 2.5 | 2.7 | 2.8 | 0.0 | 0.0 | 1.9 | 3.4 | 2.7 | 0.0 |
| hsa-miR-0512-3p | 2.5 | 2.9 | 3.3 | 0.0 | 1.3 | 1.4 | 3.3 | 0.0 | 0.0 |
| hsa-miR-0027b-3p | 0.0 | 2.6 | 3.1 | 2.5 | 2.7 | 2.8 | 3.0 | 3.4 | 0.0 |
| hsa-miR-0122-5p | 1.6 | 2.6 | 0.0 | 0.0 | 1.8 | 1.9 | 0.0 | 0.0 | 0.0 |
| hsa-miR-4729 | 0.0 | 0.0 | 0.0 | 0.0 | 0.0 | 0.0 | 0.0 | 0.0 | 3.0 |
| hsa-miR-0324-5p | 0.0 | 1.8 | 2.8 | 0.0 | 3.0 | 3.0 | 3.3 | 0.0 | 0.0 |
| hsa-miR-5681a | 3.6 | 2.3 | 0.0 | 0.0 | 2.8 | 3.0 | 3.5 | 0.0 | 0.0 |
| hsa-miR-4421 | 3.5 | 3.4 | 3.7 | 3.9 | 3.2 | 2.9 | 3.6 | 0.0 | 0.0 |
| hsa-miR-1299 | 0.0 | 0.0 | 0.0 | 2.4 | 0.0 | 0.0 | 0.0 | 0.0 | 0.0 |
| hsa-miR-0196b-5p | 0.0 | 3.7 | 0.0 | 6.7 | 2.8 | 1.3 | 2.9 | 0.0 | 0.0 |
| hsa-let-7c-3p | 0.0 | 0.0 | 0.0 | 0.0 | 1.5 | 2.4 | 0.0 | 0.0 | 0.0 |
| hsa-miR-0200b-3p | 0.0 | 2.9 | 0.0 | 0.0 | 1.7 | 0.0 | 0.0 | 0.0 | 0.0 |
| hsa-miR-0519c-5p, hsa-miR-523-5p, hsa-miR-518e-5p, hsa-miR-522-5p, hsa-miR-519a-5p, hsa-miR-519b-5p | 0.0 | 0.0 | 3.1 | 0.0 | 3.8 | 3.1 | 2.8 | 0.0 | 0.0 |
| hsa-miR-6839-5p | 2.9 | 3.2 | 2.8 | 0.0 | 2.5 | 4.0 | 3.3 | 2.5 | 2.8 |
| hsa-miR-6818-5p | 1.6 | 2.0 | 2.8 | 0.0 | 2.4 | 2.4 | 0.0 | 0.0 | 3.2 |
| hsa-miR-6837-3p | 4.4 | 3.2 | 4.2 | 4.6 | 1.5 | 2.0 | 4.0 | 0.0 | 0.0 |
| hsa-miR-3936 | 3.3 | 2.0 | 4.3 | 0.0 | 2.6 | 3.7 | 2.6 | 0.0 | 0.0 |
| hsa-miR-6783-5p | 0.0 | 3.1 | 0.0 | 3.9 | 1.8 | 0.0 | 2.8 | 0.0 | 0.0 |
| hsa-miR-1911-3p | 3.4 | 2.4 | 2.5 | 2.7 | 3.1 | 1.9 | 0.0 | 0.0 | 0.0 |
| hsa-miR-3140-3p | 0.0 | 0.0 | 0.0 | 2.7 | 3.1 | 1.9 | 0.0 | 0.0 | 0.0 |
| hsa-miR-6770-5p | 1.8 | 2.6 | 0.0 | 3.3 | 0.0 | 2.6 | 3.4 | 2.4 | 0.0 |
| hsa-miR-4527 | 3.2 | 2.6 | 1.7 | 0.0 | 0.0 | 2.4 | 2.7 | 0.0 | 3.0 |
| hsa-miR-8055 | 3.5 | 3.4 | 1.9 | 2.8 | 3.2 | 3.2 | 3.3 | 0.0 | 3.3 |
| hsa -miR -3678-5p | 1.6 | 3.0 | 0.0 | 0.0 | 1.5 | 2.1 | 0.0 | 0.0 | 0.0 |
| hsa-miR-4451 | 2.2 | 3.7 | 0.0 | 3.3 | 3.8 | 3.1 | 3.3 | 0.0 | 0.0 |
| hsa-miR-4783-5p | 0.0 | 3.6 | 3.3 | 3.7 | 2.9 | 3.1 | 3.5 | 4.2 | 3.1 |
| hsa-miR-0380-5p | 0.0 | 0.0 | 0.0 | 0.0 | 1.4 | 0.0 | 3.7 | 0.0 | 0.0 |
| hsa-miR-0610 | 0.0 | 0.0 | 0.0 | 0.0 | 2.5 | 2.4 | 2.5 | 0.0 | 0.0 |
| hsa-miR-4660 | 2.6 | 3.2 | 0.0 | 0.0 | 3.9 | 3.1 | 0.0 | 2.4 | 0.0 |
| hsa-miR-6715b-5p | 0.0 | 3.0 | 2.2 | 0.0 | 2.3 | 2.0 | 0.0 | 0.0 | 0.0 |
| hsa-let-7i-5p | 0.0 | 3.3 | 0.0 | 6.2 | 2.8 | 2.2 | 0.0 | 0.0 | 0.0 |
| hsa-miR-0153-5p | 1.5 | 2.5 | 2.1 | 0.0 | 1.5 | 2.9 | 3.6 | 0.0 | 0.0 |
| hsa-miR-1245a | 0.0 | 0.0 | 0.0 | 0.0 | 0.0 | 0.0 | 0.0 | 0.0 | 0.0 |
| hsa-miR-2276-5p | 2.6 | 3.4 | 0.0 | 0.0 | 3.0 | 3.5 | 3.6 | 3.1 | 3.4 |
| hsa-miR-3155b | 3.3 | 4.2 | 3.1 | 0.0 | 3.0 | 3.5 | 4.2 | 2.8 | 0.0 |
| hsa-miR-0425-5p | 0.0 | 2.3 | 0.0 | 6.9 | 1.3 | 0.0 | 0.0 | 0.0 | 0.0 |
| hsa-miR-0888-3p | 0.0 | 2.4 | 0.0 | 0.0 | 1.7 | 0.0 | 0.0 | 0.0 | 0.0 |
| hsa-miR-0369-5p | 1.9 | 2.3 | 0.0 | 0.0 | 1.6 | 2.2 | 3.6 | 0.0 | 0.0 |
| hsa-miR-0034c-5p | 4.8 | 3.7 | 4.4 | 0.0 | 2.8 | 2.4 | 2.6 | 0.0 | 0.0 |
| hsa-miR-0585-3p | 0.0 | 0.0 | 3.1 | 6.1 | 0.0 | 0.0 | 0.0 | 0.0 | 0.0 |
| hsa-miR-0378d | 0.0 | 0.0 | 0.0 | 0.0 | 0.0 | 2.4 | 0.0 | 0.0 | 0.0 |
| hsa-miR-0605-3p | 0.0 | 0.0 | 0.0 | 0.0 | 0.0 | 0.0 | 0.0 | 0.0 | 0.0 |
| hsa-miR-4491 | 0.0 | 0.0 | 0.0 | 2.9 | 1.5 | 2.1 | 0.0 | 0.0 | 0.0 |
| hsa-miR-0525-3p | 0.0 | 2.0 | 2.8 | 0.0 | 2.4 | 2.0 | 0.0 | 2.5 | 0.0 |
| hsa -miR -0548ay-3p | 2.0 | 2.6 | 2.6 | 2.9 | 0.0 | 0.0 | 4.1 | 2.3 | 3.4 |
| hsa-miR-7162-5p | 0.0 | 1.8 | 0.0 | 6.6 | 0.0 | 0.0 | 3.1 | 0.0 | 0.0 |
| hsa-miR-4800-3p | 0.0 | 3.2 | 0.0 | 5.1 | 2.5 | 3.1 | 3.3 | 2.5 | 3.3 |
| hsa-miR-1286 | 2.3 | 2.1 | 2.1 | 0.0 | 2.1 | 2.0 | 2.9 | 2.4 | 0.0 |
| hsa -miR -0942-3p | 3.3 | 0.0 | 0.0 | 0.0 | 3.6 | 3.3 | 3.9 | 0.0 | 0.0 |
| hsa-miR-4771 | 0.0 | 3.1 | 0.0 | 3.5 | 0.0 | 1.8 | 3.1 | 0.0 | 2.8 |
| hsa -miR -3682-3p | 2.7 | 3.5 | 0.0 | 0.0 | 3.3 | 4.5 | 3.5 | 3.1 | 0.0 |
| hsa-miR-8083 | 3.1 | 4.0 | 1.8 | 0.0 | 4.5 | 4.4 | 4.6 | 3.5 | 2.9 |
| hsa-miR-6513-3p | 2.2 | 3.2 | 2.6 | 0.0 | 2.9 | 2.7 | 0.0 | 0.0 | 0.0 |
| hsa-miR-3929 | 2.1 | 2.8 | 0.0 | 6.8 | 2.4 | 2.8 | 0.0 | 0.0 | 0.0 |
| hsa-miR-3200-3p | 2.8 | 2.3 | 0.0 | 3.1 | 0.0 | 2.2 | 0.0 | 2.2 | 2.8 |
| hsa-miR-4703-3p | 0.0 | 3.5 | 0.0 | 0.0 | 2.3 | 2.8 | 0.0 | 2.5 | 0.0 |
| hsa-miR-3918 | 3.6 | 3.3 | 3.5 | 0.0 | 2.9 | 3.7 | 2.6 | 2.5 | 0.0 |
| hsa-miR-0650 | 2.0 | 3.3 | 3.0 | 0.0 | 5.0 | 6.1 | 0.0 | 0.0 | 0.0 |
| hsa-miR-0034b-5p | 2.2 | 2.1 | 0.0 | 0.0 | 2.6 | 2.0 | 3.3 | 0.0 | 2.7 |
| hsa-miR-4524b-5p | 0.0 | 2.6 | 0.0 | 0.0 | 2.5 | 0.0 | 0.0 | 0.0 | 0.0 |
| hsa-miR-4644 | 3.4 | 4.1 | 0.0 | 0.0 | 2.7 | 3.0 | 3.1 | 2.4 | 0.0 |
| hsa-miR-4283 | 0.0 | 3.4 | 0.0 | 0.0 | 3.0 | 3.8 | 3.1 | 2.3 | 0.0 |
| hsa-miR-5696 | 0.0 | 2.7 | 0.0 | 0.0 | 0.0 | 2.9 | 0.0 | 0.0 | 0.0 |
| hsa-miR-0127-5p | 0.0 | 0.0 | 0.0 | 2.6 | 3.2 | 0.0 | 2.6 | 0.0 | 0.0 |
| hsa-miR-3666 | 3.9 | 4.8 | 4.8 | 0.0 | 5.0 | 4.1 | 2.6 | 3.4 | 3.2 |
| hsa-miR-4786-3p | 0.0 | 3.7 | 3.2 | 0.0 | 3.1 | 3.0 | 4.0 | 3.0 | 0.0 |
| hsa-miR-0138-2-3p | 0.0 | 4.0 | 0.0 | 0.0 | 3.2 | 2.2 | 2.7 | 2.2 | 0.0 |
| hsa-miR-1276 | 0.0 | 2.9 | 0.0 | 0.0 | 2.9 | 3.2 | 0.0 | 2.3 | 0.0 |
| hsa-miR-4709-5p | 1.7 | 3.6 | 2.3 | 0.0 | 0.0 | 2.2 | 3.5 | 0.0 | 0.0 |
| hsa-miR-4681 | 0.0 | 3.7 | 1.9 | 0.0 | 3.1 | 3.4 | 3.8 | 2.2 | 0.0 |
| hsa-miR-5094 | 1.9 | 3.2 | 2.2 | 0.0 | 3.2 | 3.3 | 0.0 | 2.4 | 0.0 |
| hsa-miR-0431-5p | 0.0 | 2.1 | 0.0 | 0.0 | 0.0 | 0.0 | 3.4 | 0.0 | 2.8 |
| hsa-miR-0383-3p | 3.4 | 3.5 | 3.0 | 3.1 | 2.6 | 2.7 | 3.2 | 0.0 | 3.1 |
| hsa-miR-0183-5p | 0.0 | 0.0 | 2.1 | 0.0 | 0.0 | 0.0 | 3.5 | 0.0 | 3.2 |
| hsa -miR -3678-3p | 2.2 | 0.0 | 0.0 | 0.0 | 3.6 | 3.3 | 0.0 | 2.4 | 3.3 |
| hsa-miR-0370-5p | 0.0 | 2.0 | 1.8 | 0.0 | 0.0 | 1.9 | 0.0 | 0.0 | 0.0 |
| hsa-miR-4755-5p | 3.1 | 2.2 | 0.0 | 0.0 | 2.1 | 2.6 | 3.9 | 0.0 | 0.0 |
| hsa-miR-3605-5p | 3.0 | 3.3 | 4.1 | 0.0 | 3.5 | 3.7 | 0.0 | 0.0 | 0.0 |
| hsa -miR -0654-5p | 3.3 | 3.1 | 4.2 | 0.0 | 4.1 | 4.4 | 3.2 | 3.5 | 0.0 |
| hsa-miR-0579-5p | 2.7 | 3.3 | 2.8 | 0.0 | 2.1 | 3.6 | 2.7 | 0.0 | 3.1 |
| hsa-miR-8080 | 1.7 | 2.7 | 0.0 | 0.0 | 3.1 | 3.3 | 0.0 | 2.4 | 2.7 |
| hsa-miR-4437 | 2.6 | 4.3 | 1.7 | 0.0 | 3.3 | 3.0 | 0.0 | 2.6 | 0.0 |
| hsa-miR-7853-5p | 3.7 | 3.0 | 2.6 | 0.0 | 2.4 | 2.7 | 3.3 | 2.3 | 0.0 |
| hsa-miR-6718-5p | 3.0 | 3.4 | 0.0 | 0.0 | 3.1 | 3.6 | 0.0 | 3.0 | 0.0 |
| hsa-miR-6501-5p | 2.6 | 2.1 | 1.7 | 6.6 | 0.0 | 2.9 | 0.0 | 0.0 | 0.0 |
| hsa-miR-7641 | 0.0 | 3.9 | 4.4 | 3.5 | 3.8 | 3.7 | 3.8 | 4.5 | 4.1 |
| hsa-miR-0495-5p | 3.7 | 4.1 | 0.0 | 2.7 | 3.7 | 3.0 | 3.9 | 2.2 | 3.4 |
| hsa-miR-0888-5p | 3.4 | 2.4 | 0.0 | 0.0 | 2.4 | 3.2 | 4.6 | 0.0 | 0.0 |
| hsa-miR-5188 | 3.1 | 3.5 | 2.2 | 0.0 | 1.9 | 2.5 | 4.2 | 2.9 | 2.6 |
| hsa-miR-3174 | 3.3 | 3.4 | 3.2 | 0.0 | 3.0 | 2.4 | 0.0 | 2.3 | 2.8 |
| hsa -miR -0548ax | 0.0 | 2.3 | 0.0 | 2.5 | 0.0 | 1.6 | 0.0 | 0.0 | 3.4 |
| hsa-miR-1284 | 3.0 | 3.6 | 1.7 | 0.0 | 2.3 | 3.4 | 0.0 | 0.0 | 0.0 |
| hsa-miR-4724-3p | 0.0 | 2.2 | 5.5 | 0.0 | 2.2 | 2.0 | 0.0 | 0.0 | 0.0 |
| hsa-miR-4275 | 1.7 | 0.0 | 1.6 | 0.0 | 0.0 | 0.0 | 0.0 | 0.0 | 0.0 |
| hsa-miR-0193b-5p | 2.4 | 3.8 | 1.9 | 0.0 | 3.4 | 3.6 | 0.0 | 2.6 | 0.0 |
| hsa -miR -5006-5p | 3.4 | 3.7 | 3.3 | 4.5 | 4.9 | 5.1 | 4.0 | 3.6 | 0.0 |
| hsa-miR-4650-5p | 2.0 | 2.7 | 0.0 | 0.0 | 1.5 | 2.7 | 0.0 | 0.0 | 0.0 |
| hsa-miR-4465 | 2.7 | 3.0 | 2.0 | 0.0 | 4.0 | 3.2 | 2.7 | 0.0 | 2.6 |
| hsa -miR -0320c | 0.0 | 0.0 | 0.0 | 2.9 | 1.7 | 2.0 | 0.0 | 0.0 | 0.0 |
| hsa-miR-0030a-3p | 2.1 | 3.7 | 0.0 | 0.0 | 3.5 | 2.1 | 2.6 | 2.3 | 0.0 |
| hsa-miR-0892a | 0.0 | 2.9 | 0.0 | 2.8 | 2.7 | 2.2 | 0.0 | 2.5 | 2.7 |
| hsa -miR -0668-5p | 2.8 | 3.5 | 3.8 | 2.4 | 4.2 | 4.3 | 0.0 | 3.6 | 0.0 |
| hsa-miR-8057 | 3.3 | 4.3 | 3.4 | 0.0 | 3.6 | 4.9 | 0.0 | 2.2 | 3.4 |
| hsa-miR-3200-5p | 2.9 | 3.2 | 3.1 | 0.0 | 2.3 | 3.0 | 0.0 | 0.0 | 0.0 |
| hsa-miR-6874-5p | 0.0 | 2.8 | 0.0 | 0.0 | 3.3 | 3.2 | 3.2 | 0.0 | 0.0 |
| hsa -miR -0338-3p | 3.2 | 3.3 | 0.0 | 2.5 | 4.3 | 1.8 | 3.4 | 2.8 | 3.3 |
| hsa-miR-0184 | 0.0 | 2.8 | 2.3 | 0.0 | 0.0 | 1.5 | 3.4 | 2.3 | 2.7 |
| hsa-miR-5096 | 0.0 | 2.7 | 0.0 | 0.0 | 3.6 | 3.6 | 0.0 | 2.4 | 0.0 |
| hsa-miR-7848-3p | 4.0 | 4.2 | 3.9 | 3.3 | 3.4 | 2.9 | 3.3 | 0.0 | 0.0 |
| hsa-miR-6081 | 0.0 | 4.2 | 3.9 | 6.1 | 3.2 | 4.3 | 4.2 | 4.3 | 0.0 |
| hsa-miR-0196b-3p | 2.9 | 4.0 | 3.8 | 0.0 | 3.4 | 3.6 | 3.2 | 2.9 | 0.0 |
| hsa-miR-0147a | 3.1 | 3.0 | 2.7 | 0.0 | 3.5 | 3.5 | 0.0 | 3.8 | 2.9 |
| hsa-let-7b-5p | 3.0 | 2.9 | 2.8 | 0.0 | 3.3 | 3.7 | 0.0 | 3.4 | 3.0 |
| hsa-miR-0143-3p | 2.0 | 2.6 | 0.0 | 0.0 | 0.0 | 1.7 | 3.1 | 2.5 | 0.0 |
| hsa-miR-1273e | 2.8 | 3.3 | 0.0 | 0.0 | 3.8 | 4.1 | 0.0 | 3.8 | 0.0 |
| hsa-miR-4712-5p | 3.3 | 4.0 | 0.0 | 0.0 | 3.0 | 3.4 | 2.9 | 0.0 | 3.2 |
| hsa-miR-5011-5p | 0.0 | 0.0 | 0.0 | 0.0 | 0.0 | 0.0 | 3.0 | 0.0 | 0.0 |
| hsa-miR-0514b-3p | 2.0 | 2.0 | 0.0 | 0.0 | 0.0 | 2.5 | 2.7 | 0.0 | 0.0 |
| hsa-miR-0325 | 2.1 | 3.8 | 2.8 | 0.0 | 1.9 | 2.3 | 3.7 | 2.2 | 3.2 |
| hsa-miR-4482-5p | 2.5 | 3.0 | 0.0 | 2.4 | 1.7 | 2.6 | 3.6 | 0.0 | 0.0 |
| hsa-miR-4303 | 2.2 | 3.9 | 0.0 | 3.8 | 3.7 | 3.9 | 2.9 | 2.8 | 0.0 |
| hsa-miR-5680 | 2.3 | 3.3 | 0.0 | 0.0 | 1.8 | 2.7 | 4.5 | 0.0 | 0.0 |
| hsa-miR-0664b-5p | 0.0 | 3.4 | 0.0 | 0.0 | 2.3 | 2.7 | 0.0 | 0.0 | 0.0 |
| hsa-miR-6853-5p | 3.2 | 3.5 | 3.5 | 0.0 | 2.9 | 3.9 | 3.7 | 0.0 | 0.0 |
| hsa-miR-4752 | 4.3 | 3.1 | 3.3 | 0.0 | 2.8 | 3.0 | 0.0 | 0.0 | 0.0 |
| hsa -miR -5002-5p | 2.2 | 2.4 | 0.0 | 0.0 | 0.0 | 0.0 | 0.0 | 0.0 | 0.0 |
| hsa-miR-4524b-3p | 2.8 | 4.0 | 0.0 | 0.0 | 1.7 | 4.0 | 2.6 | 0.0 | 2.7 |
| hsa-miR-0589-3p | 2.8 | 3.0 | 2.3 | 0.0 | 0.0 | 3.0 | 0.0 | 0.0 | 0.0 |
| hsa-miR-1271-5p | 1.6 | 3.3 | 0.0 | 0.0 | 2.8 | 3.2 | 2.9 | 2.2 | 3.2 |
| hsa-miR-6509-5p | 2.3 | 3.3 | 0.0 | 7.8 | 3.2 | 2.6 | 0.0 | 2.3 | 0.0 |
| hsa -miR -4638-3p | 0.0 | 3.2 | 3.0 | 3.0 | 2.4 | 3.3 | 4.2 | 3.9 | 2.6 |
| hsa-miR-5689 | 0.0 | 1.9 | 0.0 | 0.0 | 1.8 | 2.7 | 3.3 | 0.0 | 0.0 |
| hsa-miR-8084 | 0.0 | 2.7 | 0.0 | 0.0 | 0.0 | 3.4 | 0.0 | 0.0 | 0.0 |
| hsa-miR-1295b-3p | 2.9 | 2.9 | 2.5 | 0.0 | 1.6 | 3.1 | 4.2 | 2.7 | 3.2 |
| hsa-miR-7154-3p | 1.7 | 2.1 | 1.8 | 6.4 | 0.0 | 2.4 | 0.0 | 0.0 | 0.0 |
| hsa-miR-5095 | 2.5 | 3.6 | 2.2 | 0.0 | 3.6 | 3.6 | 0.0 | 2.5 | 0.0 |
| hsa-miR-4273 | 2.9 | 3.7 | 3.7 | 0.0 | 2.7 | 2.8 | 0.0 | 2.4 | 0.0 |
| hsa-miR-0145-5p | 1.7 | 0.0 | 2.5 | 0.0 | 2.3 | 1.3 | 3.5 | 0.0 | 3.0 |
| hsa-miR-0142-3p | 0.0 | 3.1 | 0.0 | 0.0 | 2.4 | 1.7 | 0.0 | 0.0 | 0.0 |
| hsa-miR-4657 | 0.0 | 3.9 | 1.6 | 0.0 | 4.0 | 4.3 | 2.7 | 3.5 | 0.0 |
| hsa-miR-0519a-3p | 0.0 | 1.9 | 3.0 | 0.0 | 0.0 | 0.0 | 3.1 | 2.4 | 0.0 |
| hsa-miR-3689f | 2.2 | 3.1 | 1.8 | 0.0 | 1.2 | 1.8 | 3.4 | 0.0 | 0.0 |
| hsa-miR-5571-3p | 2.8 | 3.2 | 2.1 | 0.0 | 2.9 | 3.0 | 2.7 | 0.0 | 0.0 |
| hsa-miR-0566 | 0.0 | 2.4 | 3.0 | 3.9 | 3.9 | 3.3 | 4.4 | 2.8 | 4.4 |
| hsa-miR-0138-5p | 2.2 | 3.3 | 3.5 | 0.0 | 4.3 | 3.9 | 3.3 | 0.0 | 3.0 |
| hsa-miR-0526b-3p | 4.3 | 3.6 | 1.7 | 0.0 | 0.0 | 2.6 | 3.6 | 0.0 | 2.9 |
| hsa-miR-4717-5p | 2.8 | 3.1 | 0.0 | 0.0 | 3.4 | 3.2 | 3.1 | 2.3 | 0.0 |
| hsa-miR-5591-3p | 0.0 | 3.4 | 0.0 | 0.0 | 2.6 | 3.0 | 0.0 | 0.0 | 0.0 |
| hsa-miR-4254 | 3.8 | 3.7 | 0.0 | 0.0 | 0.0 | 2.9 | 3.8 | 2.3 | 4.0 |
| hsa -miR -3682-5p | 0.0 | 2.7 | 2.7 | 2.5 | 2.8 | 2.4 | 0.0 | 0.0 | 0.0 |
| hsa -miR -6506-5p | 1.8 | 3.8 | 0.0 | 0.0 | 3.1 | 2.8 | 3.2 | 3.0 | 0.0 |
| hsa-miR-8082 | 2.5 | 3.5 | 0.0 | 0.0 | 3.4 | 3.5 | 0.0 | 3.0 | 0.0 |
| hsa-miR-7974 | 2.9 | 3.8 | 3.0 | 0.0 | 3.6 | 3.2 | 0.0 | 3.1 | 3.0 |
| hsa-miR-4999-5p | 0.0 | 3.3 | 0.0 | 0.0 | 3.8 | 3.2 | 3.2 | 0.0 | 0.0 |
| hsa-miR-4797-3p | 1.8 | 2.6 | 2.0 | 0.0 | 2.0 | 2.7 | 3.5 | 0.0 | 2.9 |
| hsa-miR-4264 | 0.0 | 2.5 | 2.1 | 0.0 | 0.0 | 0.0 | 2.9 | 0.0 | 0.0 |
| hsa-miR-0378b | 4.2 | 4.1 | 3.5 | 0.0 | 2.0 | 3.2 | 0.0 | 0.0 | 0.0 |
| hsa-miR-0323a-5p | 3.4 | 4.4 | 3.3 | 0.0 | 2.8 | 3.9 | 4.3 | 3.5 | 0.0 |
| hsa-miR-3713 | 3.9 | 3.4 | 3.8 | 0.0 | 2.0 | 3.8 | 2.7 | 0.0 | 0.0 |
| hsa-miR-4308 | 2.1 | 2.9 | 2.4 | 0.0 | 2.3 | 1.4 | 3.3 | 0.0 | 0.0 |
| hsa -miR -0942-5p | 2.4 | 2.6 | 3.5 | 0.0 | 1.9 | 1.6 | 0.0 | 0.0 | 0.0 |
| hsa-miR-4450 | 3.3 | 3.9 | 2.5 | 2.4 | 2.9 | 4.1 | 4.8 | 4.0 | 3.9 |
| hsa-miR-0146a-5p | 2.8 | 4.5 | 2.4 | 6.2 | 1.6 | 2.4 | 0.0 | 0.0 | 0.0 |
| hsa-miR-0329-5p | 2.8 | 4.0 | 2.0 | 0.0 | 3.8 | 3.0 | 3.6 | 0.0 | 0.0 |
| hsa-miR-1178-3p | 2.9 | 3.7 | 3.6 | 0.0 | 2.2 | 2.7 | 2.5 | 0.0 | 0.0 |
| hsa -miR -3922-5p | 3.8 | 4.1 | 2.7 | 3.6 | 3.1 | 3.2 | 3.6 | 2.6 | 3.0 |
| hsa-miR-6811-3p | 3.4 | 2.7 | 3.3 | 2.7 | 0.0 | 2.1 | 4.0 | 0.0 | 0.0 |
| hsa-miR-6781-3p | 3.5 | 4.1 | 4.1 | 0.0 | 3.4 | 3.3 | 3.0 | 0.0 | 0.0 |
| hsa-miR-3651 | 2.5 | 3.6 | 3.4 | 0.0 | 2.9 | 3.4 | 3.1 | 3.1 | 2.7 |
| hsa-miR-0021-3p | 2.4 | 2.8 | 2.5 | 0.0 | 3.6 | 3.3 | 3.5 | 0.0 | 2.6 |
| hsa-miR-6817-5p | 2.2 | 4.1 | 2.6 | 0.0 | 3.5 | 3.3 | 0.0 | 3.3 | 0.0 |
| hsa-miR-0195-3p | 2.5 | 0.0 | 0.0 | 0.0 | 3.0 | 2.5 | 4.1 | 2.5 | 0.0 |
| hsa-miR-3913-3p | 0.0 | 2.4 | 2.7 | 2.9 | 2.0 | 2.6 | 0.0 | 0.0 | 0.0 |
| hsa-miR-0339-5p | 3.1 | 3.1 | 3.5 | 0.0 | 2.8 | 2.9 | 3.6 | 3.3 | 3.2 |
| hsa-miR-4718 | 3.4 | 3.3 | 2.8 | 0.0 | 2.4 | 2.1 | 3.5 | 0.0 | 3.7 |
| hsa-miR-0584-5p | 3.8 | 3.5 | 1.9 | 0.0 | 4.0 | 3.3 | 3.3 | 2.7 | 0.0 |
| hsa-miR-0345-5p | 1.9 | 3.7 | 2.8 | 0.0 | 3.1 | 2.3 | 0.0 | 2.3 | 2.7 |
| hsa-miR-7155-3p | 3.9 | 4.3 | 4.6 | 0.0 | 3.7 | 4.0 | 3.3 | 2.8 | 0.0 |
| hsa-miR-6505-3p | 4.0 | 4.3 | 3.7 | 0.0 | 3.5 | 3.2 | 3.5 | 3.0 | 3.0 |
| hsa-miR-1251-3p | 2.5 | 3.2 | 2.6 | 0.0 | 1.8 | 3.0 | 0.0 | 2.4 | 3.1 |
| hsa-miR-3690 | 2.6 | 3.1 | 0.0 | 0.0 | 4.0 | 3.1 | 3.4 | 0.0 | 2.8 |
| hsa-miR-5000-5p | 2.5 | 2.5 | 0.0 | 0.0 | 1.4 | 2.8 | 0.0 | 0.0 | 2.9 |
| hsa-miR-0596 | 0.0 | 3.4 | 1.9 | 0.0 | 3.3 | 3.2 | 3.4 | 4.1 | 0.0 |
| hsa-miR-0591 | 2.6 | 3.8 | 2.0 | 0.0 | 3.0 | 3.4 | 0.0 | 0.0 | 0.0 |
| hsa-miR-4658 | 2.8 | 3.2 | 3.9 | 0.0 | 2.2 | 3.2 | 0.0 | 0.0 | 0.0 |
| hsa-miR-3680-3p | 3.8 | 3.8 | 3.9 | 0.0 | 3.5 | 3.4 | 2.5 | 2.6 | 0.0 |
| hsa-miR-0379-3p | 2.1 | 3.6 | 0.0 | 0.0 | 3.8 | 2.3 | 3.1 | 0.0 | 3.2 |
| hsa-miR-0020a-3p | 3.1 | 0.0 | 3.7 | 0.0 | 4.2 | 1.6 | 0.0 | 0.0 | 0.0 |
| hsa-miR-5093 | 2.5 | 3.4 | 0.0 | 0.0 | 3.9 | 3.7 | 4.1 | 3.2 | 3.4 |
| hsa -miR -0524-5p | 2.6 | 2.8 | 1.7 | 0.0 | 2.1 | 2.3 | 0.0 | 0.0 | 3.6 |
| hsa-miR-4733-5p | 3.1 | 3.5 | 0.0 | 3.2 | 3.2 | 3.4 | 0.0 | 0.0 | 0.0 |
| hsa-miR-0619-5p | 3.4 | 3.6 | 1.7 | 3.1 | 3.7 | 3.9 | 2.6 | 4.6 | 0.0 |
| hsa-miR-0214-5p | 6.9 | 4.4 | 4.3 | 3.5 | 3.5 | 2.7 | 0.0 | 2.7 | 3.2 |
| hsa-miR-0423-3p | 3.9 | 3.1 | 3.9 | 2.5 | 3.4 | 2.3 | 3.9 | 0.0 | 2.9 |
| hsa -miR -6838-5p | 2.3 | 3.3 | 2.5 | 4.1 | 0.0 | 2.2 | 5.0 | 2.5 | 0.0 |
| hsa -miR -3186-5p | 3.0 | 4.2 | 2.8 | 5.0 | 3.4 | 3.0 | 4.7 | 2.6 | 0.0 |
| hsa-miR-6083 | 3.9 | 4.0 | 3.4 | 0.0 | 3.1 | 3.5 | 0.0 | 2.7 | 0.0 |
| hsa-miR-6761-5p | 3.4 | 4.1 | 2.7 | 0.0 | 3.6 | 2.8 | 3.7 | 3.1 | 0.0 |
| hsa-miR-0103a-2-5p | 0.0 | 2.9 | 0.0 | 0.0 | 0.0 | 2.8 | 2.5 | 0.0 | 0.0 |
| hsa-miR-6895-5p | 3.5 | 4.3 | 3.6 | 6.1 | 3.7 | 4.2 | 4.4 | 4.0 | 0.0 |
| hsa-miR-4645-3p | 3.2 | 3.0 | 4.2 | 0.0 | 3.6 | 3.5 | 0.0 | 0.0 | 0.0 |
| hsa-miR-0300 | 0.0 | 3.9 | 0.0 | 0.0 | 3.7 | 2.7 | 3.8 | 0.0 | 2.6 |
| hsa-miR-5089-3p | 2.8 | 3.1 | 0.0 | 7.5 | 2.8 | 1.9 | 0.0 | 0.0 | 0.0 |
| hsa-miR-4740-3p | 3.7 | 4.1 | 3.4 | 4.7 | 3.5 | 3.7 | 4.9 | 3.6 | 3.0 |
| hsa-miR-0511-5p | 3.4 | 2.7 | 3.4 | 4.0 | 2.4 | 1.6 | 4.0 | 0.0 | 0.0 |
| hsa-miR-6514-5p | 2.3 | 3.2 | 3.7 | 2.4 | 4.0 | 4.1 | 3.2 | 2.4 | 0.0 |
| hsa-miR-1288-5p | 2.0 | 3.7 | 0.0 | 0.0 | 0.0 | 3.0 | 3.1 | 0.0 | 3.0 |
| hsa -miR -0454-5p | 0.0 | 2.7 | 0.0 | 0.0 | 1.6 | 1.9 | 3.2 | 0.0 | 0.0 |
| hsa-miR-0645 | 2.8 | 2.7 | 0.0 | 3.9 | 2.9 | 2.8 | 0.0 | 0.0 | 0.0 |
| hsa-miR-3190-3p | 3.8 | 3.8 | 3.5 | 0.0 | 4.2 | 4.1 | 3.0 | 3.1 | 0.0 |
| hsa-miR-4438 | 2.8 | 3.0 | 0.0 | 0.0 | 0.0 | 1.7 | 0.0 | 2.4 | 0.0 |
| hsa-miR-0519d-5p | 0.0 | 2.4 | 0.0 | 0.0 | 0.0 | 0.0 | 3.0 | 0.0 | 2.7 |
| hsa-miR-1199-3p | 3.3 | 3.6 | 3.7 | 0.0 | 4.0 | 4.2 | 2.9 | 3.2 | 0.0 |
| hsa-miR-0330-3p | 0.0 | 2.5 | 0.0 | 0.0 | 3.3 | 1.9 | 4.3 | 0.0 | 2.9 |
| hsa -miR -0030c-5p | 2.6 | 3.4 | 0.0 | 2.6 | 2.4 | 2.5 | 0.0 | 0.0 | 3.3 |
| hsa-miR-7973 | 2.3 | 3.5 | 2.3 | 0.0 | 2.5 | 3.2 | 0.0 | 0.0 | 0.0 |
| hsa-miR-0509-3p | 3.0 | 3.2 | 4.1 | 0.0 | 5.0 | 4.7 | 0.0 | 3.4 | 2.8 |
| hsa-miR-1298-3p | 3.5 | 4.3 | 3.2 | 0.0 | 2.9 | 3.8 | 4.5 | 2.9 | 3.4 |
| hsa-miR-3667-5p | 3.3 | 2.7 | 3.6 | 5.6 | 1.3 | 2.8 | 0.0 | 0.0 | 0.0 |
| hsa-miR-0519b-3p | 0.0 | 3.1 | 0.0 | 0.0 | 0.0 | 0.0 | 0.0 | 0.0 | 3.0 |
| hsa-miR-6499-5p | 3.7 | 4.3 | 4.3 | 3.4 | 2.9 | 3.8 | 4.4 | 3.4 | 0.0 |
| hsa-miR-6850-3p | 0.0 | 3.4 | 3.5 | 2.9 | 2.4 | 3.6 | 3.5 | 4.4 | 2.9 |
| hsa-miR-5194 | 2.5 | 4.0 | 3.1 | 0.0 | 3.0 | 3.2 | 0.0 | 0.0 | 0.0 |
| hsa-miR-7849-3p | 3.6 | 2.1 | 3.8 | 0.0 | 3.4 | 2.7 | 2.9 | 3.1 | 2.7 |
| hsa-miR-0410-5p | 1.6 | 3.1 | 2.8 | 5.0 | 3.0 | 3.0 | 3.5 | 0.0 | 0.0 |
| hsa-miR-3654 | 2.5 | 3.1 | 2.9 | 3.6 | 2.7 | 2.3 | 2.8 | 0.0 | 3.0 |
| hsa-miR-4446-5p | 2.1 | 3.6 | 2.1 | 2.5 | 2.8 | 2.4 | 3.0 | 0.0 | 3.2 |
| hsa-miR-4740-5p | 3.1 | 3.4 | 2.6 | 2.8 | 3.3 | 3.1 | 3.4 | 2.6 | 3.2 |
| hsa-miR-6503-3p | 4.2 | 4.5 | 3.7 | 4.2 | 4.4 | 4.1 | 4.4 | 3.3 | 0.0 |
| hsa-miR-0106b-3p | 2.8 | 3.0 | 2.7 | 3.2 | 3.2 | 3.4 | 0.0 | 3.9 | 0.0 |
| hsa-miR-3657 | 3.0 | 3.3 | 2.5 | 0.0 | 2.4 | 3.2 | 0.0 | 0.0 | 0.0 |
| hsa-miR-5192 | 2.2 | 2.8 | 2.7 | 0.0 | 2.1 | 2.1 | 3.5 | 2.3 | 3.0 |
| hsa-miR-6818-3p | 3.4 | 3.9 | 3.5 | 0.0 | 3.3 | 3.8 | 4.5 | 2.8 | 2.7 |
| hsa-miR-1915-5p | 3.7 | 4.3 | 4.8 | 4.4 | 4.3 | 4.3 | 4.3 | 4.9 | 4.6 |
| hsa-miR-3145-3p | 0.0 | 0.0 | 0.0 | 4.4 | 4.3 | 4.3 | 0.0 | 0.0 | 0.0 |
| hsa-miR-4684-3p | 3.6 | 3.5 | 3.7 | 0.0 | 3.1 | 3.7 | 3.6 | 3.3 | 0.0 |
| hsa-miR-5694 | 2.7 | 3.8 | 2.3 | 0.0 | 3.1 | 3.8 | 3.8 | 2.7 | 0.0 |
| hsa -miR -0654-3p | 3.3 | 3.3 | 2.4 | 0.0 | 3.1 | 3.6 | 2.9 | 0.0 | 0.0 |
| hsa-miR-4773 | 3.0 | 3.4 | 2.6 | 5.5 | 3.0 | 2.5 | 3.1 | 0.0 | 0.0 |
| hsa-miR-7161-5p | 0.0 | 2.6 | 0.0 | 0.0 | 0.0 | 2.4 | 0.0 | 0.0 | 0.0 |
| hsa-miR-5197-5p | 3.3 | 3.7 | 1.9 | 0.0 | 1.6 | 2.9 | 0.0 | 2.5 | 0.0 |
| hsa-miR-5703 | 3.0 | 4.1 | 3.5 | 5.5 | 3.7 | 3.5 | 3.8 | 2.6 | 3.7 |
| hsa-miR-0141-5p | 4.2 | 4.0 | 0.0 | 0.0 | 1.2 | 3.6 | 3.6 | 2.4 | 0.0 |
| hsa -miR -5008-3p | 2.7 | 3.3 | 2.1 | 3.3 | 3.8 | 3.2 | 4.3 | 3.5 | 3.1 |
| hsa-miR-5681b | 3.9 | 4.1 | 3.0 | 5.1 | 3.9 | 3.7 | 2.7 | 3.2 | 3.6 |
| hsa -miR -0200c-5p | 1.9 | 3.9 | 0.0 | 0.0 | 4.1 | 2.7 | 3.6 | 0.0 | 3.3 |
| hsa-miR-0125b-2-3p | 0.0 | 2.1 | 2.8 | 0.0 | 1.8 | 0.0 | 0.0 | 0.0 | 0.0 |
| hsa-miR-4754 | 3.3 | 4.0 | 3.2 | 0.0 | 3.9 | 4.6 | 4.0 | 3.5 | 2.9 |
| hsa-miR-1322 | 0.0 | 3.8 | 1.6 | 0.0 | 2.9 | 3.3 | 2.8 | 3.2 | 2.7 |
| hsa -miR -3124-3p | 0.0 | 0.0 | 1.8 | 0.0 | 2.9 | 3.3 | 0.0 | 0.0 | 0.0 |
| hsa-miR-0381-5p | 2.7 | 3.4 | 0.0 | 2.4 | 2.4 | 2.8 | 2.9 | 0.0 | 0.0 |
| hsa-miR-5699-3p | 0.0 | 3.3 | 2.3 | 0.0 | 0.0 | 1.7 | 2.7 | 0.0 | 0.0 |
| hsa -miR -5584-3p | 4.0 | 3.8 | 4.1 | 4.7 | 3.0 | 3.1 | 4.2 | 0.0 | 2.8 |
| hsa-miR-5190 | 3.5 | 3.8 | 3.6 | 3.1 | 4.1 | 4.1 | 3.9 | 2.6 | 2.6 |
| hsa -miR -0892c-5p | 2.3 | 0.0 | 0.0 | 0.0 | 1.9 | 0.0 | 0.0 | 0.0 | 0.0 |
| hsa-miR-4445-3p | 2.5 | 3.8 | 0.0 | 7.5 | 1.9 | 2.7 | 0.0 | 0.0 | 0.0 |
| hsa -miR -5004-5p | 2.8 | 3.8 | 2.8 | 6.5 | 2.3 | 3.6 | 0.0 | 3.6 | 3.0 |
| hsa-miR-0889-5p | 3.3 | 4.2 | 2.9 | 0.0 | 3.9 | 4.0 | 4.0 | 3.7 | 3.3 |
| hsa -miR -0338-5p | 0.0 | 0.0 | 0.0 | 0.0 | 2.9 | 2.6 | 3.3 | 0.0 | 0.0 |
| hsa-miR-0488-5p | 3.5 | 3.9 | 3.5 | 0.0 | 3.4 | 2.8 | 4.9 | 2.8 | 4.0 |
| hsa-miR-4653-5p | 3.0 | 3.5 | 2.3 | 0.0 | 3.4 | 3.5 | 5.0 | 2.9 | 2.7 |
| hsa-miR-0016-1-3p | 0.0 | 0.0 | 3.2 | 3.2 | 2.8 | 3.3 | 2.7 | 2.8 | 0.0 |
| hsa-miR-4310 | 3.4 | 2.6 | 2.1 | 0.0 | 2.3 | 2.8 | 4.5 | 0.0 | 2.7 |
| hsa-miR-4316 | 4.3 | 3.4 | 4.4 | 5.1 | 3.9 | 4.1 | 3.1 | 0.0 | 0.0 |
| hsa-miR-1288-3p | 0.0 | 3.6 | 0.0 | 0.0 | 2.7 | 2.7 | 3.8 | 0.0 | 0.0 |
| hsa -miR -6854-3p | 2.7 | 4.6 | 3.2 | 5.8 | 1.6 | 3.5 | 4.2 | 3.0 | 3.0 |
| hsa-miR-0221-3p | 2.3 | 3.6 | 1.9 | 0.0 | 3.7 | 2.3 | 0.0 | 0.0 | 0.0 |
| hsa-miR-4299 | 3.1 | 3.1 | 4.3 | 3.8 | 3.6 | 3.5 | 2.8 | 0.0 | 0.0 |
| hsa-miR-7703 | 3.6 | 4.1 | 3.1 | 4.2 | 3.4 | 3.0 | 4.8 | 0.0 | 0.0 |
| hsa-miR-4520-5p | 3.5 | 4.6 | 3.0 | 0.0 | 4.1 | 4.2 | 4.4 | 3.4 | 3.9 |
| hsa-miR-0212-5p | 3.5 | 4.4 | 3.3 | 0.0 | 3.5 | 3.3 | 3.0 | 3.2 | 3.0 |
| hsa-miR-0025-3p | 3.5 | 3.6 | 3.3 | 2.4 | 3.5 | 3.3 | 3.5 | 2.9 | 3.3 |
| hsa -miR -0708-5p | 2.3 | 2.2 | 0.0 | 0.0 | 3.1 | 1.2 | 0.0 | 0.0 | 0.0 |
| hsa-miR-6821-3p | 3.9 | 4.1 | 4.2 | 0.0 | 4.1 | 3.6 | 0.0 | 2.5 | 0.0 |
| hsa-miR-0146b-3p | 3.3 | 4.0 | 0.0 | 0.0 | 3.7 | 3.9 | 3.2 | 3.6 | 0.0 |
| hsa-miR-4514 | 2.9 | 3.2 | 0.0 | 0.0 | 3.6 | 3.0 | 2.8 | 3.1 | 3.8 |
| hsa-miR-4429 | 2.3 | 3.9 | 4.3 | 3.4 | 3.6 | 4.5 | 0.0 | 2.6 | 0.0 |
| hsa-miR-0216a-3p | 3.4 | 4.6 | 4.2 | 0.0 | 4.0 | 4.5 | 4.0 | 3.9 | 4.5 |
| hsa -miR -0432-5p | 3.7 | 3.9 | 3.7 | 3.6 | 3.8 | 4.0 | 0.0 | 2.3 | 3.0 |
| hsa-miR-0525-5p | 1.7 | 3.2 | 5.6 | 2.6 | 7.3 | 6.7 | 0.0 | 2.4 | 3.0 |
| hsa-miR-1250-5p | 2.4 | 3.0 | 2.1 | 0.0 | 3.9 | 3.4 | 0.0 | 2.4 | 0.0 |
| hsa-miR-5580-3p | 2.8 | 3.6 | 3.0 | 0.0 | 3.8 | 3.3 | 4.1 | 3.1 | 3.0 |
| hsa-miR-0622 | 3.4 | 4.0 | 2.3 | 0.0 | 3.5 | 4.2 | 4.0 | 2.5 | 0.0 |
| hsa-miR-0875-3p | 3.2 | 2.9 | 0.0 | 0.0 | 3.2 | 3.2 | 0.0 | 2.5 | 0.0 |
| hsa-miR-0099b-5p | 3.9 | 4.0 | 2.9 | 0.0 | 4.0 | 3.2 | 3.9 | 2.6 | 3.5 |
| hsa-miR-0660-3p | 3.4 | 3.7 | 3.0 | 0.0 | 3.4 | 3.6 | 3.7 | 0.0 | 3.3 |
| hsa -miR -0520h | 2.8 | 3.1 | 3.6 | 3.4 | 3.2 | 3.1 | 4.2 | 2.3 | 3.7 |
| hsa-miR-8062 | 1.9 | 3.1 | 2.3 | 0.0 | 2.2 | 2.3 | 2.5 | 0.0 | 0.0 |
| hsa-miR-6765-3p | 3.1 | 2.5 | 3.4 | 3.4 | 3.8 | 3.5 | 3.8 | 3.0 | 0.0 |
| hsa -miR -0508-5p | 2.8 | 3.6 | 0.0 | 0.0 | 3.6 | 2.8 | 3.7 | 2.8 | 3.4 |
| hsa-miR-0501-5p | 2.0 | 3.4 | 0.0 | 0.0 | 2.5 | 3.2 | 2.6 | 0.0 | 0.0 |
| hsa -miR -6868-5p | 3.1 | 4.7 | 3.2 | 0.0 | 4.0 | 4.2 | 3.5 | 3.9 | 0.0 |
| hsa-miR-5187-3p | 3.8 | 4.1 | 3.0 | 0.0 | 2.6 | 3.6 | 3.8 | 2.7 | 2.7 |
| hsa-miR-3074-5p | 3.7 | 3.9 | 3.5 | 2.8 | 3.3 | 3.9 | 2.5 | 3.2 | 3.3 |
| hsa-miR-3170 | 2.3 | 3.3 | 0.0 | 2.8 | 3.3 | 3.9 | 4.0 | 2.8 | 2.6 |
| hsa -miR -0224-3p | 2.2 | 3.3 | 2.1 | 0.0 | 3.0 | 2.8 | 3.6 | 0.0 | 0.0 |
| hsa-miR-2116-5p | 0.0 | 3.4 | 3.6 | 0.0 | 4.1 | 3.1 | 2.9 | 0.0 | 0.0 |
| hsa-miR-3153 | 5.0 | 4.2 | 5.3 | 0.0 | 4.1 | 3.1 | 4.8 | 3.8 | 3.5 |
| hsa-miR-6728-5p | 3.9 | 4.1 | 3.7 | 0.0 | 4.2 | 4.0 | 3.8 | 3.2 | 0.0 |
| hsa-miR-4328 | 2.9 | 4.0 | 3.4 | 6.5 | 1.9 | 2.7 | 3.2 | 2.3 | 0.0 |
| hsa -miR -6804-5p | 3.1 | 4.0 | 3.1 | 0.0 | 3.3 | 4.2 | 3.2 | 0.0 | 2.6 |
| hsa -miR -0204-5p | 3.4 | 3.9 | 2.5 | 2.7 | 3.8 | 3.7 | 4.8 | 0.0 | 4.3 |
| hsa-miR-4764-3p | 2.6 | 3.3 | 0.0 | 2.7 | 2.7 | 3.0 | 3.5 | 2.2 | 0.0 |
| hsa-miR-7854-3p | 2.7 | 3.9 | 2.6 | 0.0 | 4.0 | 4.4 | 3.7 | 3.8 | 0.0 |
| hsa -miR -0382-5p | 2.0 | 2.9 | 0.0 | 0.0 | 2.1 | 1.6 | 2.7 | 0.0 | 0.0 |
| hsa-miR-3615 | 3.4 | 3.3 | 2.9 | 0.0 | 4.7 | 3.0 | 4.5 | 0.0 | 3.7 |
| hsa-miR-5585-3p | 3.8 | 4.1 | 3.9 | 0.0 | 3.9 | 4.8 | 3.4 | 4.3 | 3.7 |
| hsa -miR -0422a | 3.1 | 3.8 | 1.8 | 7.1 | 1.9 | 2.9 | 3.1 | 0.0 | 0.0 |
| hsa-miR-6871-3p | 3.3 | 3.5 | 3.6 | 0.0 | 3.8 | 3.1 | 3.8 | 0.0 | 0.0 |
| hsa-miR-4756-3p | 3.9 | 3.7 | 2.3 | 0.0 | 3.2 | 3.4 | 3.4 | 2.3 | 2.6 |
| hsa-miR-0485-5p | 3.7 | 3.9 | 3.4 | 2.8 | 3.9 | 4.1 | 3.8 | 3.7 | 3.8 |
| hsa-miR-0921 | 0.0 | 3.1 | 0.0 | 0.0 | 3.5 | 3.2 | 2.8 | 0.0 | 0.0 |
| hsa-miR-0340-3p | 2.9 | 2.9 | 0.0 | 0.0 | 2.4 | 3.3 | 3.0 | 2.8 | 3.3 |
| hsa-miR-3529-5p | 3.5 | 3.8 | 3.5 | 0.0 | 3.2 | 2.9 | 2.8 | 2.4 | 0.0 |
| hsa-miR-0758-5p | 3.4 | 3.5 | 3.5 | 0.0 | 3.1 | 2.8 | 3.7 | 2.6 | 0.0 |
| hsa-miR-3681-3p | 2.4 | 3.3 | 0.0 | 5.4 | 1.2 | 2.3 | 3.3 | 0.0 | 0.0 |
| hsa-miR-3074-3p | 3.1 | 2.5 | 2.1 | 0.0 | 2.6 | 2.5 | 2.7 | 2.4 | 0.0 |
| hsa-miR-3169 | 0.0 | 0.0 | 0.0 | 0.0 | 2.6 | 2.5 | 0.0 | 0.0 | 0.0 |
| hsa-miR-0030b-5p | 2.3 | 3.5 | 3.3 | 0.0 | 3.6 | 3.7 | 4.1 | 2.5 | 3.2 |
| hsa -miR -0506-3p | 3.4 | 3.9 | 2.8 | 0.0 | 4.0 | 3.5 | 3.7 | 0.0 | 0.0 |
| hsa-miR-2117 | 2.2 | 3.5 | 0.0 | 0.0 | 2.2 | 1.6 | 4.4 | 0.0 | 2.9 |
| hsa-miR-3154 | 9.2 | 7.3 | 8.7 | 0.0 | 2.2 | 1.6 | 7.1 | 6.0 | 5.8 |
| hsa-miR-0943 | 3.0 | 3.5 | 2.5 | 3.7 | 3.6 | 3.6 | 3.7 | 3.1 | 0.0 |
| hsa-miR-0603 | 2.9 | 3.8 | 2.4 | 0.0 | 3.2 | 2.8 | 2.8 | 2.8 | 3.7 |
| hsa-miR-4784 | 3.0 | 4.2 | 0.0 | 0.0 | 3.8 | 4.5 | 2.6 | 3.2 | 3.2 |
| hsa -miR -0296-3p | 4.3 | 5.5 | 4.5 | 3.8 | 4.9 | 5.6 | 4.8 | 5.5 | 3.5 |
| hsa-miR-4529-5p | 3.3 | 3.9 | 1.6 | 0.0 | 3.0 | 3.8 | 2.6 | 3.0 | 0.0 |
| hsa-miR-8081 | 2.1 | 4.2 | 0.0 | 0.0 | 3.6 | 4.0 | 4.5 | 3.4 | 3.2 |
| hsa-miR-0034b-3p | 1.8 | 3.9 | 2.0 | 0.0 | 3.0 | 3.9 | 3.2 | 0.0 | 0.0 |
| hsa-miR-4768-3p | 0.0 | 2.4 | 0.0 | 0.0 | 1.4 | 2.2 | 0.0 | 0.0 | 0.0 |
| hsa-miR-6134 | 3.6 | 4.6 | 3.2 | 0.0 | 3.5 | 4.2 | 4.0 | 3.4 | 3.1 |
| hsa-miR-0558 | 0.0 | 2.7 | 0.0 | 3.8 | 3.1 | 2.8 | 0.0 | 0.0 | 0.0 |
| hsa-miR-4635 | 3.2 | 4.2 | 2.1 | 3.1 | 4.2 | 3.6 | 3.9 | 3.2 | 0.0 |
| hsa-miR-5091 | 2.4 | 3.3 | 0.0 | 0.0 | 2.9 | 3.5 | 0.0 | 0.0 | 0.0 |
| hsa-miR-1254 | 3.4 | 3.5 | 4.5 | 0.0 | 6.4 | 7.0 | 0.0 | 3.2 | 0.0 |
| hsa-miR-4296 | 2.9 | 3.8 | 0.0 | 3.0 | 4.5 | 3.4 | 4.0 | 4.1 | 0.0 |
| hsa -miR -6504-3p | 4.3 | 4.6 | 3.3 | 0.0 | 4.0 | 4.3 | 4.2 | 3.5 | 2.9 |
| hsa -miR -3944-5p | 4.1 | 4.7 | 3.3 | 2.4 | 3.0 | 3.9 | 4.0 | 2.9 | 3.8 |
| hsa-miR-0541-5p | 2.8 | 3.5 | 0.0 | 0.0 | 3.1 | 3.4 | 3.3 | 2.8 | 2.8 |
| hsa-miR-5580-5p | 3.0 | 4.2 | 2.6 | 0.0 | 3.7 | 4.0 | 0.0 | 2.5 | 2.9 |
| hsa-miR-3655 | 3.5 | 4.2 | 2.3 | 0.0 | 3.6 | 4.3 | 4.8 | 3.0 | 2.9 |
| hsa -miR -0029c-5p | 2.6 | 3.4 | 2.4 | 2.9 | 2.8 | 2.9 | 0.0 | 0.0 | 3.2 |
| hsa-miR-1184 | 4.0 | 3.9 | 4.2 | 0.0 | 4.3 | 4.0 | 4.5 | 3.3 | 3.6 |
| hsa-miR-5571-5p | 3.7 | 4.1 | 3.0 | 2.6 | 3.3 | 3.5 | 4.4 | 0.0 | 0.0 |
| hsa-miR-0092b-3p | 3.9 | 4.1 | 4.2 | 4.0 | 3.8 | 4.3 | 3.8 | 3.9 | 4.0 |
| hsa-miR-5587-3p | 2.4 | 3.7 | 3.2 | 2.7 | 3.1 | 4.7 | 4.5 | 4.1 | 3.7 |
| hsa-miR-0769-3p | 2.4 | 4.0 | 5.0 | 0.0 | 4.0 | 4.3 | 3.4 | 4.8 | 3.0 |
| hsa-miR-0935 | 3.5 | 4.1 | 4.0 | 0.0 | 4.1 | 4.1 | 4.6 | 4.1 | 0.0 |
| hsa-miR-1199-5p | 2.0 | 5.0 | 4.7 | 3.9 | 5.0 | 5.0 | 4.0 | 5.8 | 3.5 |
| hsa-miR-0520a-3p | 3.4 | 2.8 | 2.9 | 0.0 | 3.3 | 3.1 | 3.6 | 2.3 | 3.1 |
| hsa-miR-0297 | 0.0 | 3.7 | 0.0 | 0.0 | 1.3 | 2.8 | 0.0 | 0.0 | 0.0 |
| hsa-miR-6841-5p | 4.1 | 4.3 | 2.3 | 0.0 | 2.9 | 4.5 | 0.0 | 3.0 | 0.0 |
| hsa-miR-7151-5p | 3.2 | 4.2 | 2.0 | 0.0 | 3.1 | 3.9 | 3.5 | 3.2 | 0.0 |
| hsa-miR-1277-3p | 0.0 | 0.0 | 0.0 | 0.0 | 0.0 | 0.0 | 3.4 | 0.0 | 0.0 |
| hsa-miR-0033b-3p | 3.1 | 3.2 | 2.9 | 3.0 | 3.5 | 3.2 | 3.7 | 0.0 | 0.0 |
| hsa-miR-4304 | 1.8 | 2.8 | 2.9 | 3.3 | 3.7 | 3.9 | 4.1 | 3.2 | 2.9 |
| hsa-miR-0873-3p | 2.8 | 3.2 | 0.0 | 2.7 | 2.4 | 3.3 | 2.9 | 0.0 | 0.0 |
| hsa-miR-4420 | 2.7 | 4.0 | 2.0 | 0.0 | 4.4 | 4.3 | 4.0 | 3.0 | 0.0 |
| hsa-miR-4479 | 2.5 | 5.2 | 4.7 | 4.4 | 5.1 | 5.1 | 4.2 | 4.7 | 4.7 |
| hsa-miR-0105-5p | 0.0 | 3.0 | 0.0 | 0.0 | 4.2 | 2.9 | 4.4 | 2.8 | 0.0 |
| hsa-miR-6855-5p | 2.3 | 4.5 | 3.1 | 4.9 | 4.2 | 4.6 | 3.5 | 3.6 | 2.5 |
| hsa-miR-3975 | 2.9 | 2.6 | 2.1 | 0.0 | 2.8 | 3.2 | 3.0 | 0.0 | 0.0 |
| hsa-miR-4453 | 2.5 | 3.7 | 0.0 | 0.0 | 4.6 | 4.2 | 3.4 | 3.4 | 2.9 |
| hsa-miR-1236-5p | 4.1 | 4.3 | 3.8 | 5.5 | 3.9 | 3.6 | 3.9 | 2.3 | 0.0 |
| hsa-miR-7162-3p | 2.9 | 4.2 | 1.7 | 6.6 | 3.3 | 3.8 | 3.5 | 0.0 | 0.0 |
| hsa-miR-0580-3p | 2.2 | 2.5 | 1.8 | 0.0 | 1.4 | 0.0 | 0.0 | 0.0 | 0.0 |
| hsa -miR -6852-5p | 2.9 | 3.9 | 0.0 | 0.0 | 3.4 | 3.9 | 4.7 | 2.7 | 2.7 |
| hsa-miR-0154-5p | 3.9 | 4.5 | 2.8 | 0.0 | 4.2 | 4.5 | 4.3 | 2.3 | 3.4 |
| hsa-miR-0412-3p | 3.4 | 3.8 | 3.6 | 0.0 | 4.2 | 4.4 | 4.5 | 3.8 | 3.2 |
| hsa-miR-6863 | 3.4 | 3.7 | 4.1 | 0.0 | 4.5 | 3.9 | 4.4 | 3.5 | 0.0 |
| hsa-miR-0676-5p | 3.0 | 4.1 | 2.0 | 0.0 | 3.4 | 3.1 | 3.9 | 2.2 | 0.0 |
| hsa-miR-6733-3p | 3.9 | 4.2 | 3.7 | 0.0 | 2.0 | 3.9 | 0.0 | 2.4 | 2.5 |
| hsa-miR-3972 | 4.3 | 3.9 | 5.1 | 0.0 | 3.0 | 4.0 | 4.7 | 2.6 | 3.0 |
| hsa-miR-0007-2-3p | 3.8 | 4.0 | 3.1 | 0.0 | 3.6 | 3.8 | 0.0 | 2.5 | 3.1 |
| hsa-miR-4441 | 4.4 | 2.0 | 5.0 | 0.0 | 5.3 | 4.3 | 0.0 | 0.0 | 0.0 |
| hsa-miR-4300 | 2.6 | 3.8 | 1.8 | 0.0 | 4.4 | 4.2 | 3.3 | 2.5 | 0.0 |
| hsa-miR-6874-3p | 3.7 | 4.1 | 2.2 | 0.0 | 4.2 | 4.0 | 4.1 | 3.2 | 0.0 |
| hsa-miR-3691-3p | 3.9 | 3.7 | 2.7 | 3.0 | 3.3 | 3.6 | 3.5 | 2.9 | 0.0 |
| hsa-miR-0034c-3p | 3.2 | 4.8 | 3.6 | 0.0 | 3.6 | 4.0 | 4.2 | 2.7 | 0.0 |
| hsa-miR-0519e-3p | 4.2 | 4.1 | 2.5 | 2.5 | 5.2 | 4.1 | 3.2 | 0.0 | 3.0 |
| hsa-miR-6774-3p | 3.9 | 3.8 | 3.4 | 5.2 | 3.0 | 3.9 | 4.1 | 2.7 | 0.0 |
| hsa-miR-1285-5p | 2.8 | 4.2 | 3.7 | 6.6 | 3.5 | 3.4 | 4.6 | 3.3 | 3.6 |
| hsa-miR-0518f-3p | 2.4 | 0.0 | 3.5 | 0.0 | 4.7 | 2.7 | 0.0 | 0.0 | 0.0 |
| hsa-miR-6791-3p | 3.9 | 4.3 | 3.1 | 2.5 | 3.6 | 4.1 | 4.1 | 3.5 | 2.8 |
| hsa-miR-4761-3p | 3.7 | 3.4 | 4.0 | 3.5 | 3.3 | 3.1 | 3.9 | 3.0 | 4.1 |
| hsa-miR-0548d-3p | 0.0 | 0.0 | 0.0 | 0.0 | 2.4 | 0.0 | 3.3 | 0.0 | 0.0 |
| hsa-miR-1266-3p | 4.5 | 4.2 | 4.3 | 2.5 | 3.3 | 4.0 | 0.0 | 0.0 | 0.0 |
| hsa-miR-0212-3p | 3.1 | 3.9 | 3.7 | 3.2 | 4.1 | 4.1 | 0.0 | 3.1 | 4.0 |
| hsa-miR-0744-3p | 3.3 | 4.2 | 2.1 | 2.5 | 3.8 | 2.7 | 3.0 | 0.0 | 0.0 |
| hsa-miR-9500 | 5.2 | 4.3 | 4.5 | 0.0 | 3.5 | 4.3 | 5.2 | 2.9 | 3.8 |
| hsa-miR-0670-5p | 4.4 | 4.1 | 4.2 | 0.0 | 3.8 | 4.1 | 4.3 | 0.0 | 0.0 |
| hsa-miR-2276-3p | 4.3 | 4.8 | 4.9 | 0.0 | 4.9 | 4.5 | 3.5 | 3.2 | 0.0 |
| hsa-miR-3155a | 3.3 | 0.0 | 0.0 | 0.0 | 4.9 | 4.5 | 0.0 | 0.0 | 0.0 |
| hsa-miR-0517a-3p, hsa-miR-517b-3p | 2.8 | 3.3 | 0.0 | 0.0 | 3.5 | 3.3 | 2.5 | 0.0 | 0.0 |
| hsa-miR-0493-3p | 2.1 | 3.5 | 3.0 | 0.0 | 3.6 | 3.9 | 3.7 | 2.4 | 0.0 |
| hsa-miR-0302d-5p | 4.9 | 3.9 | 3.7 | 2.6 | 3.4 | 3.9 | 3.6 | 0.0 | 0.0 |
| hsa-miR-4727-3p | 1.7 | 3.5 | 0.0 | 0.0 | 4.5 | 4.9 | 0.0 | 2.2 | 3.8 |
| hsa-miR-2355-3p | 0.0 | 3.9 | 3.8 | 0.0 | 2.6 | 2.3 | 0.0 | 0.0 | 0.0 |
| hsa-miR-3157-5p | 0.0 | 0.0 | 0.0 | 0.0 | 2.6 | 2.3 | 2.7 | 0.0 | 0.0 |
| hsa-miR-0766-5p | 3.0 | 4.0 | 0.0 | 0.0 | 4.0 | 4.1 | 4.3 | 2.9 | 3.0 |
| hsa-miR-0605-5p | 2.1 | 3.0 | 0.0 | 0.0 | 3.1 | 2.6 | 3.2 | 2.5 | 3.1 |
| hsa-miR-6758-3p | 3.7 | 4.1 | 3.1 | 5.7 | 3.3 | 3.9 | 4.6 | 2.7 | 3.0 |
| hsa-miR-6515-5p | 2.9 | 4.1 | 4.8 | 0.0 | 7.0 | 6.9 | 3.5 | 4.3 | 0.0 |
| hsa -miR -3692-5p | 3.7 | 3.8 | 3.1 | 4.1 | 3.4 | 3.7 | 0.0 | 2.5 | 0.0 |
| hsa-miR-5187-5p | 0.0 | 3.8 | 0.0 | 2.6 | 3.3 | 3.8 | 0.0 | 2.8 | 0.0 |
| hsa-miR-1287-5p | 3.1 | 4.4 | 1.8 | 0.0 | 3.6 | 4.2 | 3.4 | 3.0 | 3.3 |
| hsa-miR-0518f-5p | 2.1 | 0.0 | 2.0 | 0.0 | 0.0 | 0.0 | 4.1 | 0.0 | 0.0 |
| hsa-miR-4425 | 2.6 | 3.2 | 2.2 | 0.0 | 3.1 | 3.2 | 3.5 | 2.6 | 0.0 |
| hsa-miR-0181a-2-3p | 2.9 | 3.3 | 0.0 | 0.0 | 3.0 | 3.2 | 0.0 | 2.3 | 3.1 |
| hsa -miR -0222-5p | 3.8 | 4.1 | 2.4 | 0.0 | 3.7 | 4.1 | 3.8 | 3.4 | 3.4 |
| hsa-miR-0129-5p | 3.9 | 4.5 | 2.8 | 0.0 | 4.3 | 3.9 | 4.0 | 3.6 | 3.5 |
| hsa-miR-3659 | 4.0 | 3.8 | 3.1 | 2.4 | 4.2 | 3.8 | 4.3 | 0.0 | 3.3 |
| hsa-miR-7158-5p | 3.7 | 3.7 | 2.1 | 0.0 | 3.4 | 3.6 | 0.0 | 2.8 | 3.4 |
| hsa-miR-6516-5p | 2.8 | 2.5 | 3.4 | 0.0 | 1.8 | 1.6 | 3.7 | 0.0 | 0.0 |
| hsa-miR-0216b-3p | 4.6 | 4.7 | 4.2 | 0.0 | 3.6 | 4.6 | 4.5 | 3.4 | 3.8 |
| hsa-miR-6739-3p | 2.9 | 4.1 | 0.0 | 3.1 | 4.2 | 3.8 | 4.4 | 3.8 | 0.0 |
| hsa-miR-0609 | 2.7 | 3.2 | 0.0 | 0.0 | 3.5 | 3.7 | 3.4 | 0.0 | 2.6 |
| hsa-miR-0210-3p | 4.1 | 4.7 | 4.3 | 2.4 | 4.2 | 4.5 | 4.4 | 4.2 | 4.2 |
| hsa-miR-0323b-5p | 2.2 | 4.0 | 2.9 | 0.0 | 4.3 | 3.7 | 3.8 | 3.7 | 3.2 |
| hsa-miR-7844-5p | 4.2 | 3.6 | 3.4 | 6.9 | 2.6 | 3.0 | 4.1 | 2.5 | 3.1 |
| hsa-miR-4301 | 3.2 | 3.8 | 3.3 | 0.0 | 3.7 | 3.9 | 0.0 | 2.6 | 2.8 |
| hsa-miR-0134-3p | 3.8 | 4.4 | 3.9 | 2.5 | 4.0 | 4.4 | 4.5 | 3.6 | 3.1 |
| hsa-miR-3691-5p | 2.1 | 3.6 | 0.0 | 3.5 | 3.0 | 3.5 | 0.0 | 2.3 | 0.0 |
| hsa-miR-0425-3p | 3.8 | 4.6 | 3.4 | 4.1 | 3.5 | 3.9 | 4.0 | 3.0 | 0.0 |
| hsa-miR-0135a-3p | 3.8 | 5.0 | 4.3 | 2.7 | 4.9 | 6.5 | 0.0 | 6.1 | 3.0 |
| hsa-miR-1207-3p | 4.5 | 4.6 | 4.6 | 0.0 | 4.1 | 4.4 | 4.9 | 3.1 | 3.2 |
| hsa -miR -6856-3p | 4.0 | 4.2 | 2.2 | 0.0 | 3.8 | 3.7 | 3.4 | 3.6 | 3.4 |
| hsa-miR-4641 | 3.5 | 4.4 | 2.0 | 0.0 | 3.8 | 3.7 | 4.3 | 3.3 | 3.5 |
| hsa -miR -0520g-3p | 4.1 | 3.4 | 2.3 | 0.0 | 3.3 | 2.9 | 4.5 | 2.2 | 2.8 |
| hsa-miR-6843-3p | 3.5 | 4.2 | 0.0 | 0.0 | 3.6 | 4.3 | 3.0 | 2.7 | 2.5 |
| hsa-miR-0877-5p | 3.5 | 3.9 | 2.9 | 0.0 | 3.6 | 3.6 | 3.1 | 2.6 | 3.2 |
| hsa-miR-0584-3p | 4.0 | 4.6 | 3.2 | 2.7 | 4.6 | 3.9 | 4.9 | 3.8 | 3.7 |
| hsa-miR-4260 | 4.1 | 3.9 | 3.9 | 0.0 | 5.1 | 5.3 | 4.2 | 3.9 | 3.6 |
| hsa-miR-3619-5p | 3.6 | 4.5 | 3.8 | 3.6 | 4.2 | 4.7 | 4.5 | 4.4 | 3.7 |
| hsa-miR-7975 | 3.5 | 4.6 | 3.1 | 0.0 | 4.0 | 3.8 | 4.5 | 3.6 | 3.1 |
| hsa-miR-0512-5p | 4.4 | 4.1 | 4.4 | 3.5 | 3.6 | 3.8 | 4.3 | 3.2 | 4.0 |
| hsa-miR-4691-5p | 3.7 | 3.5 | 3.1 | 2.5 | 4.1 | 4.5 | 3.0 | 3.3 | 3.3 |
| hsa-miR-4713-3p | 4.2 | 4.1 | 0.0 | 2.7 | 3.6 | 3.8 | 3.1 | 2.3 | 3.2 |
| hsa-miR-6755-3p | 3.2 | 3.1 | 0.0 | 0.0 | 2.4 | 2.9 | 2.7 | 0.0 | 2.7 |
| hsa-miR-3945 | 4.2 | 5.0 | 2.4 | 2.9 | 3.5 | 4.0 | 4.5 | 2.4 | 0.0 |
| hsa-miR-0030b-3p | 2.6 | 2.8 | 2.9 | 3.2 | 3.9 | 4.1 | 3.6 | 2.7 | 3.3 |
| hsa-miR-0185-5p | 3.3 | 2.7 | 3.5 | 0.0 | 4.9 | 3.3 | 0.0 | 0.0 | 0.0 |
| hsa -miR -6878-5p | 4.5 | 4.4 | 4.4 | 0.0 | 3.8 | 3.8 | 0.0 | 2.6 | 0.0 |
| hsa-miR-0129-1-3p | 4.6 | 3.6 | 4.6 | 4.8 | 4.2 | 3.9 | 4.9 | 2.9 | 2.6 |
| hsa -miR -0548ba | 3.9 | 2.8 | 0.0 | 0.0 | 0.0 | 0.0 | 0.0 | 0.0 | 0.0 |
| hsa-miR-0150-5p | 3.7 | 4.0 | 3.7 | 3.2 | 4.0 | 3.8 | 4.3 | 3.5 | 3.7 |
| hsa-miR-1910-3p | 3.1 | 4.4 | 2.8 | 0.0 | 3.8 | 3.9 | 4.2 | 3.5 | 3.4 |
| hsa-miR-3138 | 4.3 | 3.8 | 3.8 | 0.0 | 3.8 | 3.9 | 0.0 | 2.7 | 0.0 |
| hsa-miR-6500-3p | 3.6 | 4.2 | 3.2 | 2.9 | 4.4 | 4.7 | 3.2 | 3.2 | 0.0 |
| hsa-miR-7157-5p | 3.8 | 4.3 | 2.8 | 0.0 | 3.9 | 4.1 | 3.3 | 3.1 | 3.0 |
| hsa-miR-0593-3p | 2.7 | 4.3 | 0.0 | 0.0 | 3.9 | 3.8 | 2.9 | 3.4 | 0.0 |
| hsa-miR-0018a-3p | 2.9 | 4.0 | 2.5 | 2.9 | 3.7 | 4.0 | 0.0 | 3.2 | 2.7 |
| hsa-miR-1972 | 3.6 | 4.0 | 2.7 | 7.5 | 4.0 | 4.0 | 4.7 | 3.5 | 3.6 |
| hsa-miR-3145-5p | 0.0 | 4.2 | 2.4 | 7.5 | 4.0 | 4.0 | 4.6 | 2.8 | 0.0 |
| hsa -miR -3922-3p | 3.9 | 3.9 | 2.4 | 0.0 | 3.8 | 3.8 | 3.4 | 3.5 | 2.9 |
| hsa-miR-6746-3p | 3.8 | 4.4 | 3.9 | 0.0 | 4.3 | 4.4 | 4.8 | 4.5 | 3.8 |
| hsa-miR-0093-5p | 3.2 | 3.1 | 2.5 | 0.0 | 2.3 | 1.8 | 2.8 | 0.0 | 0.0 |
| hsa-miR-1301-5p | 3.7 | 4.8 | 3.9 | 0.0 | 3.9 | 4.1 | 3.2 | 3.9 | 3.6 |
| hsa-miR-3116 | 4.4 | 3.7 | 4.1 | 0.0 | 3.9 | 4.1 | 3.9 | 0.0 | 2.9 |
| hsa-miR-0937-3p | 4.0 | 3.7 | 3.8 | 2.5 | 3.7 | 3.7 | 4.1 | 3.3 | 3.7 |
| hsa-miR-3187-3p | 4.3 | 5.5 | 5.5 | 7.2 | 4.6 | 5.9 | 4.7 | 5.3 | 2.7 |
| hsa-miR-0581 | 3.7 | 4.3 | 1.7 | 0.0 | 3.0 | 3.7 | 4.2 | 3.2 | 4.2 |
| hsa-miR-6816-3p | 4.0 | 3.2 | 3.6 | 0.0 | 4.1 | 3.1 | 0.0 | 0.0 | 0.0 |
| hsa-miR-1296-3p | 4.3 | 4.5 | 4.5 | 0.0 | 4.1 | 4.3 | 3.9 | 4.5 | 4.2 |
| hsa-miR-3921 | 4.4 | 3.1 | 0.0 | 0.0 | 1.4 | 3.7 | 0.0 | 0.0 | 3.4 |
| hsa-miR-1273g-5p | 3.0 | 4.2 | 1.9 | 0.0 | 4.3 | 4.3 | 2.6 | 2.7 | 3.2 |
| hsa -miR -0432-3p | 4.5 | 4.1 | 2.3 | 2.9 | 4.4 | 4.0 | 3.1 | 3.3 | 3.3 |
| hsa -miR -0656-5p | 3.1 | 4.0 | 0.0 | 0.0 | 4.1 | 3.9 | 4.7 | 3.9 | 0.0 |
| hsa-miR-4726-3p | 3.8 | 3.9 | 3.7 | 2.5 | 3.8 | 3.7 | 3.3 | 3.0 | 3.4 |
| hsa-miR-0092a-3p | 3.7 | 3.9 | 3.3 | 3.7 | 4.0 | 4.4 | 2.7 | 2.8 | 4.7 |
| hsa-miR-4265 | 4.9 | 4.8 | 5.6 | 5.1 | 4.4 | 4.6 | 4.8 | 3.9 | 2.8 |
| hsa-miR-0608 | 2.8 | 4.5 | 0.0 | 0.0 | 4.6 | 4.2 | 4.2 | 4.2 | 3.0 |
| hsa-miR-0135a-5p | 3.5 | 3.0 | 1.7 | 0.0 | 1.4 | 1.3 | 3.3 | 0.0 | 0.0 |
| hsa-miR-0500b-3p | 3.9 | 5.0 | 2.9 | 2.6 | 3.6 | 4.6 | 3.6 | 4.0 | 3.1 |
| hsa-miR-6823-5p | 2.6 | 3.3 | 2.7 | 3.5 | 3.2 | 3.8 | 0.0 | 0.0 | 0.0 |
| hsa-miR-4767 | 4.1 | 4.6 | 4.8 | 4.5 | 4.5 | 4.7 | 5.6 | 4.8 | 4.8 |
| hsa-miR-6499-3p | 3.8 | 4.7 | 3.5 | 0.0 | 4.3 | 3.3 | 4.4 | 3.8 | 0.0 |
| hsa-miR-6788-5p | 3.9 | 3.4 | 0.0 | 0.0 | 4.0 | 4.2 | 3.8 | 2.9 | 0.0 |
| hsa-miR-1200 | 4.0 | 4.0 | 0.0 | 0.0 | 4.0 | 4.2 | 0.0 | 3.7 | 3.4 |
| hsa -miR -4436a | 3.7 | 4.9 | 3.6 | 0.0 | 4.4 | 4.9 | 3.8 | 2.5 | 3.1 |
| hsa-miR-4700-5p | 4.4 | 4.8 | 4.1 | 0.0 | 4.9 | 5.0 | 4.8 | 3.8 | 3.3 |
| hsa -miR -3928-5p | 3.5 | 4.7 | 4.0 | 3.5 | 4.1 | 4.6 | 4.8 | 4.4 | 3.3 |
| hsa -miR -5006-3p | 3.4 | 3.6 | 2.8 | 0.0 | 3.8 | 3.8 | 0.0 | 2.9 | 3.2 |
| hsa-miR-7152-3p | 5.8 | 4.6 | 4.6 | 3.7 | 4.3 | 4.9 | 4.4 | 3.4 | 0.0 |
| hsa -miR -6504-5p | 4.1 | 4.7 | 3.9 | 2.7 | 4.6 | 4.3 | 3.8 | 3.1 | 3.3 |
| hsa-miR-0602 | 0.0 | 7.1 | 6.2 | 5.8 | 6.9 | 6.8 | 4.3 | 7.1 | 4.8 |
| hsa-miR-0330-5p | 2.9 | 4.1 | 2.8 | 0.0 | 2.9 | 3.5 | 2.9 | 2.4 | 0.0 |
| hsa-miR-0491-5p | 4.2 | 4.5 | 5.1 | 2.9 | 6.0 | 6.1 | 4.8 | 4.4 | 3.2 |
| hsa-miR-6747-5p | 3.7 | 4.3 | 4.0 | 0.0 | 4.8 | 5.0 | 3.4 | 4.4 | 0.0 |
| hsa -miR -6822-3p | 4.1 | 4.5 | 4.4 | 4.1 | 3.5 | 3.9 | 3.4 | 2.8 | 0.0 |
| hsa-miR-7850-5p | 3.7 | 4.8 | 3.2 | 0.0 | 4.2 | 4.3 | 4.7 | 4.3 | 3.8 |
| hsa-miR-0572 | 3.8 | 4.6 | 4.1 | 4.6 | 5.8 | 5.6 | 4.7 | 4.6 | 4.6 |
| hsa-miR-3065-3p | 3.6 | 4.3 | 4.9 | 3.4 | 3.5 | 3.7 | 0.0 | 3.0 | 3.8 |
| hsa-miR-3167 | 0.0 | 0.0 | 0.0 | 3.4 | 3.5 | 3.7 | 0.0 | 0.0 | 0.0 |
| hsa-miR-1273h-3p | 4.1 | 3.6 | 3.7 | 3.5 | 3.8 | 3.6 | 4.3 | 3.2 | 3.0 |
| hsa-miR-3198 | 4.9 | 4.2 | 2.2 | 0.0 | 4.1 | 4.5 | 0.0 | 3.4 | 0.0 |
| hsa-miR-6764-3p | 5.0 | 4.1 | 4.6 | 7.0 | 4.6 | 4.5 | 4.4 | 3.1 | 2.7 |
| hsa-miR-4538 | 3.1 | 5.1 | 2.9 | 0.0 | 4.1 | 3.2 | 4.5 | 4.0 | 2.7 |
| hsa-miR-0378a-3p | 4.5 | 3.9 | 4.9 | 0.0 | 4.1 | 4.3 | 4.5 | 3.1 | 3.1 |
| hsa-miR-3612 | 4.3 | 3.9 | 4.0 | 0.0 | 4.6 | 4.2 | 0.0 | 0.0 | 0.0 |
| hsa -miR -0552-5p | 4.3 | 3.7 | 3.3 | 0.0 | 3.4 | 3.7 | 4.1 | 0.0 | 2.8 |
| hsa-miR-8070 | 3.3 | 4.0 | 2.5 | 0.0 | 2.6 | 3.7 | 4.3 | 3.1 | 0.0 |
| hsa-miR-3689b-3p, hsa-miR-3689c | 3.9 | 4.8 | 4.0 | 3.4 | 4.3 | 4.9 | 4.1 | 4.3 | 3.9 |
| hsa-let-7a-2-3p | 4.3 | 4.4 | 3.5 | 6.3 | 2.3 | 3.9 | 3.9 | 3.3 | 3.2 |
| hsa -miR -0668-3p | 4.5 | 4.8 | 4.9 | 2.4 | 4.7 | 4.4 | 4.3 | 4.2 | 4.3 |
| hsa-miR-0023a-5p | 3.9 | 4.5 | 2.5 | 3.4 | 4.3 | 4.3 | 4.4 | 3.2 | 3.4 |
| hsa-miR-4288 | 5.1 | 5.1 | 4.2 | 0.0 | 3.8 | 4.8 | 3.8 | 2.9 | 0.0 |
| hsa-miR-6077 | 4.9 | 4.5 | 4.2 | 0.0 | 3.9 | 4.5 | 4.7 | 2.7 | 2.8 |
| hsa-miR-0770-5p | 3.7 | 4.0 | 0.0 | 2.6 | 3.6 | 3.5 | 4.1 | 4.1 | 4.2 |
| hsa-miR-1306-3p | 3.1 | 4.3 | 0.0 | 2.9 | 4.0 | 3.4 | 4.2 | 4.0 | 3.5 |
| hsa-miR-3120-5p | 3.5 | 4.4 | 0.0 | 2.9 | 4.0 | 3.4 | 4.0 | 3.9 | 2.7 |
| hsa-miR-0337-3p | 3.2 | 3.3 | 0.0 | 2.6 | 3.8 | 2.6 | 0.0 | 0.0 | 3.1 |
| hsa-miR-6512-3p | 4.3 | 5.1 | 3.8 | 0.0 | 4.6 | 4.7 | 3.7 | 4.0 | 4.3 |
| hsa-miR-1914-5p | 4.7 | 4.8 | 4.8 | 4.2 | 4.7 | 4.5 | 5.6 | 4.8 | 5.1 |
| hsa-miR-3144-3p | 2.6 | 0.0 | 2.0 | 4.2 | 4.7 | 4.5 | 3.0 | 0.0 | 0.0 |
| hsa-miR-0887-5p | 4.7 | 4.0 | 5.3 | 0.0 | 5.1 | 5.3 | 4.1 | 4.2 | 3.6 |
| hsa-miR-3927-5p | 3.7 | 3.9 | 2.9 | 0.0 | 3.9 | 3.7 | 2.9 | 2.7 | 0.0 |
| hsa-miR-3177-5p | 3.2 | 5.3 | 4.6 | 4.7 | 5.5 | 5.6 | 4.9 | 5.7 | 5.1 |
| hsa-miR-3176 | 4.1 | 4.4 | 4.1 | 0.0 | 3.2 | 4.2 | 4.6 | 3.7 | 2.7 |
| hsa-miR-1291 | 3.4 | 3.9 | 2.9 | 2.9 | 4.2 | 3.9 | 3.9 | 3.2 | 3.7 |
| hsa-miR-0891a-5p | 3.0 | 4.2 | 0.0 | 0.0 | 3.7 | 4.4 | 4.5 | 3.3 | 3.5 |
| hsa-miR-4708-3p | 3.7 | 5.0 | 4.6 | 0.0 | 4.7 | 5.3 | 4.2 | 4.4 | 4.2 |
| hsa-miR-5010-5p | 4.2 | 5.3 | 5.0 | 3.7 | 4.7 | 5.9 | 4.3 | 5.1 | 2.9 |
| hsa-miR-6773-3p | 4.4 | 5.4 | 4.5 | 0.0 | 4.7 | 4.4 | 4.9 | 4.1 | 3.8 |
| hsa-miR-5581-3p | 4.4 | 4.8 | 4.2 | 0.0 | 4.7 | 4.9 | 4.8 | 4.1 | 4.0 |
| hsa-miR-0365b-5p | 4.2 | 4.5 | 4.4 | 6.6 | 4.3 | 4.7 | 4.6 | 3.6 | 3.9 |
| hsa -miR -4652-5p | 4.3 | 4.1 | 4.1 | 2.7 | 4.6 | 4.7 | 5.3 | 3.0 | 4.2 |
| hsa-miR-0125b-5p | 5.0 | 4.6 | 4.3 | 0.0 | 4.3 | 4.3 | 4.7 | 3.0 | 0.0 |
| hsa-miR-4502 | 2.6 | 3.9 | 2.9 | 6.1 | 3.5 | 4.0 | 3.2 | 2.7 | 0.0 |
| hsa-miR-4673 | 4.6 | 5.3 | 4.9 | 2.9 | 4.2 | 5.5 | 4.0 | 5.5 | 4.2 |
| hsa -miR -5588-3p | 3.5 | 4.5 | 3.6 | 0.0 | 3.7 | 3.9 | 3.3 | 3.6 | 3.5 |
| hsa-miR-0671-3p | 4.0 | 3.8 | 3.4 | 0.0 | 3.5 | 4.1 | 3.1 | 0.0 | 0.0 |
| hsa-miR-5001-3p | 3.8 | 4.7 | 0.0 | 0.0 | 4.2 | 4.4 | 3.2 | 3.1 | 0.0 |
| hsa -miR -6828-5p | 3.2 | 3.2 | 2.8 | 0.0 | 4.2 | 3.4 | 2.8 | 0.0 | 0.0 |
| hsa-miR-4319 | 3.9 | 3.9 | 3.6 | 2.9 | 3.7 | 4.4 | 0.0 | 3.5 | 0.0 |
| hsa-miR-6814-3p | 4.6 | 5.2 | 4.2 | 4.8 | 4.1 | 4.2 | 4.6 | 3.0 | 4.4 |
| hsa-miR-1273f | 3.6 | 5.2 | 3.9 | 2.9 | 4.4 | 5.1 | 3.4 | 4.2 | 0.0 |
| hsa-miR-0099b-3p | 3.6 | 4.7 | 3.9 | 3.5 | 4.1 | 4.1 | 4.6 | 3.1 | 3.8 |
| hsa-miR-3622a-5p | 3.8 | 4.5 | 4.2 | 3.6 | 4.5 | 5.0 | 3.3 | 4.5 | 3.0 |
| hsa-miR-6833-5p | 4.7 | 4.6 | 4.3 | 0.0 | 5.1 | 4.1 | 3.8 | 3.7 | 3.0 |
| hsa-miR-2277-5p | 4.0 | 4.7 | 4.6 | 3.0 | 4.0 | 3.7 | 3.8 | 2.6 | 3.6 |
| hsa -miR -3156-5p | 5.3 | 3.9 | 5.0 | 3.0 | 4.0 | 3.7 | 5.1 | 3.0 | 3.9 |
| hsa-miR-3685 | 4.2 | 4.4 | 3.4 | 0.0 | 4.3 | 3.8 | 4.1 | 4.0 | 3.6 |
| hsa-miR-7976 | 4.1 | 4.8 | 4.2 | 0.0 | 4.4 | 4.3 | 4.8 | 4.0 | 3.7 |
| hsa-miR-3189-3p | 4.6 | 3.8 | 4.1 | 0.0 | 4.7 | 6.0 | 0.0 | 2.4 | 0.0 |
| hsa-miR-0138-1-3p | 3.3 | 4.6 | 3.0 | 0.0 | 3.7 | 4.4 | 4.3 | 3.9 | 2.8 |
| hsa-miR-6789-3p | 4.5 | 4.8 | 5.0 | 0.0 | 4.2 | 4.8 | 4.7 | 3.9 | 2.9 |
| hsa-miR-0661 | 3.9 | 4.7 | 3.6 | 0.0 | 4.4 | 4.4 | 4.6 | 4.0 | 3.7 |
| hsa-miR-4654 | 2.6 | 5.2 | 3.4 | 6.0 | 4.4 | 4.7 | 5.0 | 4.0 | 0.0 |
| hsa-miR-4692 | 2.4 | 4.5 | 0.0 | 0.0 | 4.0 | 3.6 | 4.1 | 3.4 | 2.9 |
| hsa-miR-1471 | 4.7 | 5.4 | 4.9 | 3.0 | 5.5 | 5.9 | 4.8 | 4.7 | 4.2 |
| hsa-miR-3129-5p | 0.0 | 0.0 | 0.0 | 3.0 | 5.5 | 5.9 | 0.0 | 0.0 | 0.0 |
| hsa-miR-6764-5p | 3.9 | 4.8 | 3.0 | 3.0 | 4.5 | 4.2 | 4.4 | 3.7 | 4.7 |
| hsa-miR-4529-3p | 3.8 | 4.2 | 3.1 | 0.0 | 3.9 | 4.3 | 4.1 | 3.7 | 3.2 |
| hsa-miR-0505-5p | 4.6 | 4.2 | 4.1 | 0.0 | 4.8 | 4.6 | 3.7 | 2.5 | 4.0 |
| hsa-miR-6511b-5p | 4.7 | 5.1 | 5.3 | 4.4 | 5.4 | 5.5 | 4.8 | 3.9 | 3.7 |
| hsa-miR-4686 | 3.2 | 4.7 | 3.3 | 0.0 | 3.5 | 3.3 | 4.3 | 3.8 | 2.8 |
| hsa-miR-0551a | 3.1 | 4.6 | 3.1 | 0.0 | 3.9 | 3.9 | 3.0 | 2.9 | 3.6 |
| hsa -miR -6892-5p | 4.0 | 4.3 | 4.4 | 3.6 | 3.9 | 4.2 | 4.5 | 3.9 | 0.0 |
| hsa-miR-3935 | 4.4 | 5.2 | 4.5 | 0.0 | 4.0 | 4.9 | 4.8 | 4.4 | 2.7 |
| hsa-miR-0193a-5p | 3.6 | 4.4 | 3.9 | 2.8 | 4.5 | 4.6 | 3.2 | 3.6 | 2.8 |
| hsa-miR-1293 | 1.7 | 4.6 | 2.2 | 2.4 | 4.1 | 4.1 | 4.7 | 3.8 | 3.6 |
| hsa-miR-4540 | 4.0 | 4.7 | 2.7 | 6.2 | 4.4 | 4.6 | 3.0 | 3.8 | 3.8 |
| hsa-miR-0331-3p | 4.9 | 4.2 | 4.5 | 5.2 | 4.0 | 4.3 | 4.7 | 3.1 | 3.6 |
| hsa-miR-6817-3p | 4.0 | 4.5 | 2.9 | 0.0 | 4.3 | 4.5 | 4.1 | 3.2 | 3.9 |
| hsa -miR -6884-5p | 3.8 | 4.6 | 3.1 | 0.0 | 4.7 | 4.4 | 4.1 | 3.9 | 3.6 |
| hsa -miR -6862-5p | 5.0 | 4.2 | 5.6 | 6.1 | 6.1 | 6.4 | 3.6 | 3.8 | 3.4 |
| hsa-miR-1266-5p | 3.9 | 4.0 | 3.5 | 2.8 | 3.8 | 3.7 | 3.4 | 2.6 | 3.6 |
| hsa-miR-1204 | 3.6 | 3.9 | 0.0 | 0.0 | 4.6 | 4.3 | 3.9 | 3.7 | 4.2 |
| hsa -miR -0030c-1-3p | 4.4 | 5.8 | 4.4 | 3.6 | 4.9 | 5.3 | 4.2 | 3.2 | 3.3 |
| hsa-miR-1468-5p | 2.5 | 4.5 | 3.3 | 0.0 | 3.3 | 3.5 | 3.7 | 2.2 | 0.0 |
| hsa-miR-3127-5p | 0.0 | 3.1 | 0.0 | 0.0 | 3.3 | 3.5 | 0.0 | 2.6 | 0.0 |
| hsa-miR-0527, hsa-miR-518a-5p | 4.4 | 3.5 | 4.6 | 0.0 | 5.3 | 4.0 | 3.3 | 0.0 | 4.0 |
| hsa-miR-4307 | 4.3 | 4.2 | 3.9 | 2.9 | 4.0 | 4.0 | 4.2 | 2.8 | 3.7 |
| hsa-miR-6722-5p | 4.3 | 4.5 | 4.5 | 3.5 | 4.6 | 4.7 | 4.7 | 4.5 | 4.3 |
| hsa-miR-0657 | 4.2 | 4.2 | 4.0 | 0.0 | 4.2 | 4.2 | 4.1 | 3.3 | 4.3 |
| hsa-miR-4684-5p | 3.4 | 4.6 | 3.8 | 7.0 | 4.0 | 3.4 | 5.2 | 4.6 | 5.1 |
| hsa-miR-6780a-3p | 4.4 | 5.0 | 4.3 | 5.8 | 3.9 | 4.6 | 4.5 | 3.9 | 0.0 |
| hsa-miR-0885-3p | 3.9 | 4.8 | 5.5 | 3.4 | 5.4 | 5.5 | 4.1 | 4.4 | 3.1 |
| hsa-miR-0034a-5p | 3.1 | 3.6 | 1.6 | 2.6 | 3.5 | 4.2 | 3.1 | 3.3 | 2.8 |
| hsa-miR-0194-3p | 3.7 | 4.1 | 3.7 | 2.9 | 5.3 | 5.1 | 4.0 | 4.0 | 4.2 |
| hsa-miR-4776-3p | 3.2 | 4.4 | 3.8 | 0.0 | 3.9 | 4.1 | 4.2 | 3.5 | 3.6 |
| hsa -miR -3064-3p | 3.5 | 5.3 | 3.7 | 0.0 | 3.3 | 4.2 | 4.3 | 3.0 | 0.0 |
| hsa-miR-3165 | 0.0 | 2.2 | 0.0 | 0.0 | 3.3 | 4.2 | 3.7 | 0.0 | 0.0 |
| hsa-miR-4473 | 3.5 | 3.2 | 0.0 | 0.0 | 3.4 | 3.7 | 0.0 | 0.0 | 2.9 |
| hsa-miR-0625-5p | 3.9 | 0.0 | 5.0 | 0.0 | 6.4 | 4.8 | 2.8 | 0.0 | 2.9 |
| hsa-miR-4252 | 3.6 | 4.5 | 1.9 | 3.2 | 4.3 | 4.5 | 4.3 | 4.0 | 3.3 |
| hsa -miR -6866-3p | 4.1 | 4.7 | 3.9 | 0.0 | 3.4 | 4.3 | 3.5 | 2.9 | 0.0 |
| hsa-miR-1203 | 4.7 | 5.2 | 4.9 | 3.4 | 5.0 | 4.7 | 4.8 | 3.8 | 3.9 |
| hsa -miR -3934-5p | 3.3 | 4.1 | 3.0 | 6.7 | 3.6 | 4.2 | 3.2 | 3.2 | 0.0 |
| hsa-miR-4647 | 2.9 | 4.4 | 0.0 | 6.6 | 3.2 | 4.3 | 3.8 | 3.0 | 0.0 |
| hsa-miR-4711-3p | 3.3 | 4.6 | 0.0 | 0.0 | 4.7 | 4.5 | 4.5 | 3.9 | 3.9 |
| hsa-miR-4436b-3p | 3.2 | 4.7 | 3.3 | 0.0 | 4.1 | 3.8 | 2.8 | 3.5 | 3.5 |
| hsa-miR-0299-5p | 3.8 | 3.8 | 3.9 | 0.0 | 4.1 | 4.2 | 3.6 | 3.6 | 3.8 |
| hsa-miR-0193b-3p | 4.6 | 4.4 | 4.3 | 3.4 | 4.0 | 4.3 | 3.9 | 3.7 | 4.2 |
| hsa-miR-3689d | 4.3 | 4.4 | 4.4 | 4.2 | 4.1 | 4.4 | 4.2 | 4.0 | 3.5 |
| hsa-miR-3617-3p | 4.6 | 5.3 | 3.2 | 3.1 | 5.0 | 4.7 | 5.3 | 4.3 | 4.1 |
| hsa-miR-6876-5p | 3.7 | 5.1 | 3.8 | 2.6 | 4.8 | 4.7 | 3.5 | 4.1 | 4.0 |
| hsa-miR-6847-5p | 3.6 | 4.9 | 3.6 | 5.2 | 3.8 | 3.7 | 4.0 | 3.6 | 2.9 |
| hsa-miR-0767-3p | 4.6 | 4.7 | 4.3 | 3.9 | 3.6 | 4.4 | 5.1 | 3.3 | 3.4 |
| hsa-miR-4748 | 5.0 | 5.3 | 4.5 | 0.0 | 5.0 | 4.9 | 4.5 | 4.2 | 2.6 |
| hsa-miR-3177-3p | 4.0 | 3.8 | 4.4 | 2.6 | 4.7 | 4.8 | 4.1 | 3.8 | 3.2 |
| hsa -miR -6886-5p | 4.8 | 4.8 | 4.8 | 0.0 | 4.1 | 4.6 | 4.0 | 3.7 | 3.2 |
| hsa-miR-4717-3p | 5.5 | 4.7 | 5.1 | 4.5 | 4.4 | 4.9 | 5.5 | 4.4 | 4.4 |
| hsa-miR-2113 | 4.1 | 4.3 | 3.0 | 0.0 | 4.1 | 4.3 | 4.3 | 3.4 | 3.1 |
| hsa-miR-3150b-3p | 3.8 | 4.6 | 3.4 | 0.0 | 4.1 | 4.3 | 4.1 | 3.2 | 2.6 |
| hsa-miR-4326 | 4.5 | 5.3 | 4.7 | 0.0 | 4.4 | 4.6 | 4.5 | 3.5 | 4.6 |
| hsa -miR -0892b | 4.0 | 4.8 | 3.7 | 0.0 | 4.3 | 4.1 | 3.6 | 3.8 | 3.9 |
| hsa-miR-6895-3p | 4.9 | 4.9 | 4.5 | 3.6 | 4.5 | 4.9 | 5.0 | 4.1 | 2.6 |
| hsa-miR-4708-5p | 4.5 | 5.2 | 4.9 | 3.8 | 4.8 | 4.9 | 4.9 | 4.7 | 4.9 |
| hsa -miR -6856-5p | 4.5 | 5.1 | 4.0 | 3.0 | 5.0 | 5.3 | 4.8 | 4.7 | 4.0 |
| hsa-miR-0550a-5p | 4.1 | 4.4 | 4.1 | 2.9 | 4.3 | 4.5 | 3.9 | 3.7 | 2.7 |
| hsa-miR-4324 | 4.7 | 4.8 | 3.6 | 2.8 | 4.6 | 4.0 | 4.5 | 3.8 | 3.3 |
| hsa-miR-4776-5p | 4.2 | 4.8 | 3.8 | 3.6 | 5.0 | 5.4 | 4.7 | 4.4 | 3.7 |
| hsa-miR-0629-3p | 4.7 | 4.7 | 3.8 | 0.0 | 3.6 | 4.2 | 4.7 | 3.6 | 3.9 |
| hsa-miR-1273h-5p | 5.0 | 4.8 | 5.0 | 3.9 | 4.9 | 5.1 | 4.0 | 3.6 | 3.5 |
| hsa-miR-1909-5p | 4.3 | 4.6 | 4.3 | 3.3 | 5.2 | 5.6 | 5.0 | 4.2 | 3.8 |
| hsa-miR-3137 | 3.5 | 3.9 | 3.3 | 3.3 | 5.2 | 5.6 | 2.5 | 3.1 | 2.7 |
| hsa-miR-4780 | 4.7 | 4.9 | 4.3 | 3.4 | 4.0 | 4.5 | 4.9 | 4.5 | 4.2 |
| hsa-miR-8064 | 4.6 | 5.2 | 4.5 | 3.3 | 4.8 | 5.2 | 4.4 | 4.9 | 4.2 |
| hsa-miR-7702 | 4.8 | 4.9 | 4.2 | 4.7 | 4.6 | 4.5 | 4.5 | 3.9 | 3.9 |
| hsa-miR-1181 | 4.5 | 4.9 | 5.3 | 4.4 | 5.2 | 5.5 | 5.1 | 5.4 | 4.5 |
| hsa-miR-4448 | 4.2 | 4.4 | 3.5 | 3.6 | 4.6 | 5.0 | 4.0 | 3.5 | 4.1 |
| hsa-miR-4800-5p | 4.1 | 4.5 | 4.5 | 0.0 | 4.8 | 5.0 | 3.6 | 2.5 | 0.0 |
| hsa-miR-8075 | 3.8 | 5.3 | 3.6 | 4.4 | 5.1 | 3.9 | 4.7 | 4.3 | 4.3 |
| hsa -miR -0494-5p | 3.8 | 5.0 | 3.1 | 0.0 | 4.8 | 4.2 | 5.0 | 4.3 | 4.5 |
| hsa-miR-4506 | 2.5 | 3.0 | 3.3 | 3.3 | 4.2 | 4.3 | 2.9 | 0.0 | 0.0 |
| hsa-miR-3591-3p | 3.4 | 4.9 | 2.9 | 3.1 | 4.3 | 4.7 | 4.7 | 3.8 | 2.6 |
| hsa-miR-0612 | 4.1 | 4.9 | 4.2 | 4.4 | 4.6 | 4.8 | 4.9 | 4.9 | 4.8 |
| hsa-miR-3714 | 4.9 | 4.4 | 4.8 | 5.5 | 4.1 | 4.4 | 5.1 | 2.9 | 3.7 |
| hsa-miR-3616-3p | 4.4 | 5.6 | 5.3 | 4.7 | 4.8 | 5.5 | 5.2 | 5.4 | 5.4 |
| hsa-miR-0211-5p | 4.3 | 4.9 | 4.2 | 3.9 | 4.9 | 5.1 | 5.2 | 4.3 | 4.5 |
| hsa-miR-1287-3p | 5.0 | 4.6 | 3.9 | 2.5 | 4.3 | 4.3 | 5.0 | 3.7 | 4.4 |
| hsa-miR-1538 | 4.4 | 5.3 | 5.1 | 3.6 | 5.4 | 5.1 | 5.1 | 5.0 | 4.7 |
| hsa-miR-3131 | 11.1 | 9.7 | 11.8 | 3.6 | 5.4 | 5.1 | 9.6 | 8.7 | 9.3 |
| hsa-miR-0526a, hsa-miR-520c-5p, hsa-miR-518d-5p | 3.8 | 3.4 | 4.9 | 4.2 | 2.6 | 2.6 | 3.6 | 0.0 | 2.7 |
| hsa -miR -0449c-3p | 4.1 | 4.9 | 4.0 | 3.0 | 4.4 | 4.4 | 4.1 | 3.8 | 4.8 |
| hsa-miR-0636 | 4.6 | 5.0 | 5.0 | 4.2 | 5.2 | 5.3 | 5.6 | 4.9 | 4.8 |
| hsa-miR-0589-5p | 4.5 | 4.9 | 3.8 | 2.8 | 4.3 | 4.4 | 4.8 | 3.9 | 3.9 |
| hsa-miR-6815-3p | 4.7 | 4.9 | 3.7 | 2.6 | 4.3 | 4.5 | 4.7 | 3.8 | 4.5 |
| hsa-miR-6507-3p | 4.8 | 4.5 | 3.8 | 3.4 | 4.2 | 4.4 | 4.9 | 3.6 | 2.8 |
| hsa -miR -6822-5p | 5.3 | 5.3 | 5.5 | 2.5 | 4.9 | 5.0 | 4.9 | 4.3 | 4.1 |
| hsa-miR-7855-5p | 4.5 | 4.5 | 4.3 | 3.2 | 4.3 | 4.3 | 4.5 | 3.7 | 4.1 |
| hsa-miR-4428 | 4.0 | 7.6 | 3.3 | 2.9 | 4.9 | 4.4 | 4.3 | 3.9 | 2.9 |
| hsa-miR-4476 | 4.9 | 4.3 | 5.7 | 0.0 | 5.9 | 5.9 | 3.7 | 3.9 | 2.7 |
| hsa-miR-0129-2-3p | 4.4 | 4.2 | 4.3 | 3.3 | 4.5 | 3.9 | 5.2 | 3.3 | 4.4 |
| hsa-miR-4278 | 5.3 | 4.8 | 5.1 | 3.5 | 5.0 | 5.1 | 5.8 | 4.3 | 4.8 |
| hsa-miR-5685 | 4.4 | 4.6 | 3.9 | 0.0 | 4.4 | 4.6 | 4.2 | 4.3 | 4.1 |
| hsa-miR-0192-5p | 4.3 | 4.4 | 4.2 | 0.0 | 4.7 | 4.6 | 4.0 | 4.2 | 4.3 |
| hsa-miR-4482-3p | 5.4 | 5.3 | 5.9 | 5.1 | 5.6 | 5.7 | 5.4 | 5.1 | 4.5 |
| hsa-miR-1296-5p | 4.1 | 4.7 | 4.2 | 3.6 | 4.4 | 4.5 | 4.1 | 4.0 | 3.8 |
| hsa-miR-0324-3p | 4.5 | 4.8 | 4.5 | 3.7 | 4.8 | 4.7 | 4.6 | 3.5 | 3.8 |
| hsa-miR-6873-5p | 5.2 | 4.8 | 5.0 | 2.6 | 4.3 | 4.5 | 4.8 | 3.1 | 4.6 |
| hsa-miR-6847-3p | 4.7 | 4.9 | 4.6 | 2.4 | 5.2 | 5.3 | 4.9 | 4.6 | 4.2 |
| hsa-miR-5708 | 4.0 | 4.9 | 3.1 | 2.6 | 4.5 | 5.2 | 3.6 | 4.5 | 3.7 |
| hsa-miR-4747-3p | 4.8 | 4.8 | 6.1 | 4.4 | 5.6 | 5.8 | 4.9 | 5.6 | 3.3 |
| hsa-miR-6801-5p | 3.9 | 5.0 | 4.0 | 2.6 | 4.8 | 3.9 | 4.6 | 4.6 | 4.2 |
| hsa-miR-0125a-5p | 4.8 | 4.8 | 4.0 | 3.5 | 4.9 | 4.7 | 4.5 | 4.2 | 4.1 |
| hsa-miR-4269 | 4.3 | 4.9 | 5.1 | 3.5 | 5.0 | 4.9 | 4.8 | 5.1 | 4.0 |
| hsa-miR-6881-3p | 4.7 | 5.1 | 3.9 | 2.4 | 4.8 | 5.2 | 4.8 | 4.5 | 5.0 |
| hsa-miR-4664-5p | 5.5 | 4.7 | 5.6 | 4.0 | 6.0 | 5.8 | 5.0 | 3.8 | 3.9 |
| hsa-miR-0431-3p | 4.0 | 4.7 | 4.3 | 2.9 | 4.2 | 4.8 | 5.3 | 3.5 | 4.4 |
| hsa-miR-6503-5p | 5.1 | 4.6 | 4.6 | 4.6 | 4.9 | 4.5 | 5.4 | 3.5 | 3.2 |
| hsa-miR-6723-5p | 4.4 | 5.0 | 5.3 | 4.2 | 4.7 | 4.4 | 5.7 | 5.9 | 5.6 |
| hsa-miR-7113-5p | 4.0 | 4.9 | 2.9 | 5.0 | 4.6 | 3.9 | 3.9 | 3.9 | 4.7 |
| hsa-miR-2467-3p | 4.2 | 5.1 | 4.2 | 2.8 | 5.2 | 4.4 | 4.8 | 4.5 | 4.3 |
| hsa-miR-3159 | 0.0 | 0.0 | 0.0 | 2.8 | 5.2 | 4.4 | 0.0 | 0.0 | 0.0 |
| hsa-miR-8077 | 4.2 | 4.8 | 3.9 | 2.7 | 4.6 | 5.1 | 4.3 | 4.7 | 0.0 |
| hsa-miR-2682-3p | 4.6 | 5.2 | 4.8 | 2.7 | 4.9 | 5.0 | 5.3 | 4.4 | 3.9 |
| hsa-miR-3162-3p | 6.6 | 6.2 | 6.5 | 2.7 | 4.9 | 5.0 | 6.7 | 5.2 | 5.7 |
| hsa-miR-4639-3p | 4.0 | 5.3 | 2.8 | 0.0 | 4.9 | 4.2 | 4.9 | 4.4 | 4.8 |
| hsa-miR-4725-5p | 4.8 | 4.6 | 4.3 | 3.7 | 5.1 | 4.8 | 4.9 | 4.2 | 4.6 |
| hsa-miR-7151-3p | 3.3 | 5.3 | 3.2 | 3.0 | 4.7 | 5.2 | 4.4 | 4.7 | 3.7 |
| hsa-miR-4306 | 4.3 | 3.3 | 5.0 | 0.0 | 5.6 | 4.6 | 0.0 | 0.0 | 3.3 |
| hsa-miR-0585-5p | 5.2 | 5.7 | 5.1 | 5.3 | 4.6 | 4.8 | 5.6 | 4.3 | 4.5 |
| hsa-miR-4804-3p | 4.6 | 5.0 | 4.2 | 3.2 | 4.6 | 4.9 | 4.3 | 4.3 | 4.1 |
| hsa-miR-4474-3p | 3.5 | 4.4 | 2.4 | 0.0 | 4.4 | 3.7 | 4.2 | 2.4 | 3.9 |
| hsa-miR-6876-3p | 4.3 | 5.1 | 4.1 | 7.3 | 4.6 | 5.0 | 4.5 | 4.5 | 4.0 |
| hsa-miR-1224-5p | 4.1 | 5.0 | 4.5 | 2.8 | 5.0 | 5.3 | 4.3 | 4.6 | 4.1 |
| hsa -miR -6832-3p | 5.3 | 5.2 | 5.1 | 2.8 | 4.7 | 4.9 | 4.4 | 3.6 | 4.5 |
| hsa-miR-0191-3p | 4.8 | 4.6 | 4.7 | 2.7 | 5.2 | 4.7 | 5.3 | 4.0 | 4.5 |
| hsa -miR -4638-5p | 3.8 | 5.3 | 4.4 | 3.1 | 5.2 | 5.0 | 5.0 | 5.5 | 4.6 |
| hsa-miR-4518 | 4.2 | 4.7 | 3.1 | 0.0 | 3.9 | 4.4 | 4.7 | 3.0 | 2.7 |
| hsa-miR-4487 | 4.2 | 5.0 | 4.8 | 3.4 | 5.1 | 5.2 | 4.7 | 4.7 | 3.3 |
| hsa-miR-0764 | 4.9 | 4.8 | 4.7 | 3.1 | 4.9 | 4.8 | 4.4 | 4.1 | 4.4 |
| hsa-miR-6767-5p | 4.0 | 5.4 | 3.6 | 6.1 | 4.9 | 4.2 | 5.4 | 4.1 | 4.6 |
| hsa -miR -3944-3p | 4.7 | 5.4 | 4.3 | 3.4 | 5.2 | 4.9 | 5.2 | 5.0 | 5.0 |
| hsa-miR-6829-3p | 5.2 | 5.1 | 5.3 | 3.1 | 4.9 | 5.2 | 5.1 | 4.4 | 4.0 |
| hsa-miR-4292 | 4.7 | 4.6 | 4.8 | 3.6 | 4.9 | 4.4 | 4.9 | 4.7 | 4.4 |
| hsa-miR-4525 | 5.3 | 6.4 | 5.8 | 5.0 | 5.6 | 7.1 | 3.2 | 5.8 | 3.3 |
| hsa-miR-4676-5p | 3.6 | 5.0 | 3.2 | 3.0 | 4.9 | 3.4 | 4.5 | 4.4 | 4.1 |
| hsa-miR-4489 | 4.9 | 5.4 | 5.3 | 3.3 | 5.4 | 5.7 | 4.9 | 5.0 | 4.8 |
| hsa-miR-0615-5p | 4.8 | 5.1 | 6.4 | 4.4 | 6.9 | 6.4 | 5.1 | 5.3 | 4.2 |
| hsa -miR -0342-3p | 4.1 | 4.6 | 3.2 | 3.2 | 4.5 | 4.3 | 4.6 | 4.1 | 3.9 |
| hsa-miR-0550a-3p | 4.6 | 4.2 | 3.8 | 2.7 | 4.8 | 4.8 | 3.6 | 4.1 | 3.7 |
| hsa-miR-4456 | 2.9 | 4.9 | 2.5 | 0.0 | 4.8 | 4.8 | 4.3 | 3.5 | 3.7 |
| hsa-miR-1908-3p | 4.1 | 5.6 | 5.9 | 5.5 | 6.7 | 6.6 | 5.6 | 7.5 | 5.2 |
| hsa-miR-3135b | 7.3 | 8.0 | 8.7 | 5.5 | 6.7 | 6.6 | 6.8 | 7.4 | 6.3 |
| hsa-miR-1539 | 5.3 | 5.3 | 5.0 | 4.1 | 5.1 | 5.3 | 5.3 | 4.9 | 4.9 |
| hsa-miR-3132 | 3.7 | 3.9 | 0.0 | 4.1 | 5.1 | 5.3 | 3.2 | 0.0 | 0.0 |
| hsa-miR-4709-3p | 5.2 | 4.7 | 4.9 | 0.0 | 5.1 | 4.8 | 4.6 | 3.6 | 3.2 |
| hsa-let-7f-1-3p | 5.4 | 4.6 | 5.3 | 0.0 | 5.1 | 4.9 | 5.4 | 3.6 | 4.0 |
| hsa-miR-4305 | 4.4 | 5.2 | 3.5 | 2.7 | 4.7 | 4.8 | 4.5 | 4.1 | 3.2 |
| hsa-miR-6770-3p | 4.1 | 5.3 | 5.6 | 3.4 | 5.1 | 5.4 | 4.3 | 5.4 | 3.2 |
| hsa-miR-4732-5p | 5.0 | 5.6 | 5.5 | 3.3 | 5.2 | 5.4 | 4.4 | 5.0 | 3.6 |
| hsa-miR-6716-3p | 4.1 | 5.5 | 4.1 | 0.0 | 4.1 | 5.2 | 5.2 | 3.2 | 4.1 |
| hsa-miR-0483-5p | 5.1 | 5.1 | 4.4 | 0.0 | 3.9 | 4.6 | 0.0 | 2.7 | 0.0 |
| hsa-miR-1226-3p | 4.8 | 5.1 | 4.4 | 2.6 | 4.9 | 5.1 | 5.1 | 4.3 | 4.3 |
| hsa-miR-6883-5p | 5.4 | 5.2 | 5.9 | 0.0 | 7.1 | 6.4 | 4.9 | 4.6 | 4.5 |
| hsa -miR -6834-5p | 3.9 | 5.0 | 3.8 | 0.0 | 4.9 | 4.8 | 3.9 | 4.1 | 3.4 |
| hsa-miR-6849-3p | 4.5 | 5.0 | 4.1 | 2.6 | 4.9 | 5.1 | 4.4 | 4.7 | 3.8 |
| hsa-miR-3667-3p | 4.3 | 4.8 | 3.7 | 4.9 | 4.7 | 4.5 | 4.9 | 3.3 | 0.0 |
| hsa-miR-4669 | 4.5 | 5.0 | 4.1 | 3.3 | 5.2 | 4.4 | 3.7 | 4.7 | 4.0 |
| hsa -miR -4646-5p | 5.3 | 4.6 | 4.7 | 3.0 | 5.2 | 5.0 | 4.2 | 3.9 | 3.8 |
| hsa-miR-4743-5p | 5.0 | 5.3 | 4.9 | 3.5 | 5.4 | 5.5 | 4.3 | 4.6 | 3.9 |
| hsa-let-7d-3p | 3.7 | 5.0 | 3.8 | 5.9 | 4.2 | 4.5 | 5.0 | 4.1 | 3.3 |
| hsa -miR -6872-3p | 5.3 | 5.4 | 5.8 | 3.2 | 5.3 | 5.4 | 5.6 | 5.0 | 4.9 |
| hsa-miR-0526b-5p | 4.8 | 4.6 | 3.6 | 2.5 | 4.5 | 4.5 | 5.2 | 2.8 | 3.0 |
| hsa-miR-0550b-3p | 4.6 | 4.9 | 4.7 | 6.3 | 4.1 | 4.5 | 4.6 | 3.7 | 3.0 |
| hsa -miR -4652-3p | 4.5 | 4.4 | 4.1 | 2.5 | 4.9 | 4.9 | 4.8 | 3.8 | 3.5 |
| hsa-miR-4682 | 3.7 | 5.0 | 3.5 | 2.4 | 4.1 | 5.2 | 3.5 | 4.2 | 2.9 |
| hsa-miR-7851-3p | 4.5 | 5.6 | 5.3 | 3.9 | 5.7 | 5.8 | 5.1 | 5.3 | 4.6 |
| hsa-miR-1323 | 6.9 | 6.0 | 5.5 | 0.0 | 3.8 | 5.7 | 2.6 | 3.8 | 2.8 |
| hsa -miR -3124-5p | 2.2 | 2.8 | 2.9 | 0.0 | 3.8 | 5.7 | 0.0 | 3.1 | 0.0 |
| hsa -miR -6852-3p | 4.7 | 4.7 | 4.3 | 2.5 | 4.4 | 4.9 | 5.2 | 3.7 | 4.5 |
| hsa-miR-4266 | 6.8 | 5.3 | 5.5 | 0.0 | 4.7 | 4.6 | 4.3 | 3.1 | 0.0 |
| hsa-miR-1247-5p | 4.7 | 4.9 | 4.2 | 3.3 | 4.8 | 4.8 | 5.4 | 4.2 | 4.5 |
| hsa -miR -6836-5p | 3.5 | 5.7 | 5.8 | 4.1 | 5.8 | 4.8 | 5.1 | 6.0 | 4.3 |
| hsa -miR -0520b | 3.8 | 3.4 | 5.6 | 4.4 | 5.7 | 3.9 | 0.0 | 0.0 | 0.0 |
| hsa-miR-3649 | 5.7 | 5.9 | 4.1 | 2.7 | 4.3 | 5.2 | 4.4 | 3.9 | 2.9 |
| hsa-miR-6782-5p | 5.1 | 5.9 | 5.6 | 3.5 | 5.3 | 5.5 | 4.9 | 5.4 | 3.2 |
| hsa -miR -6878-3p | 4.8 | 5.6 | 4.3 | 5.7 | 4.7 | 5.0 | 5.4 | 4.4 | 3.9 |
| hsa-miR-3646 | 5.0 | 5.1 | 4.7 | 3.7 | 5.3 | 5.2 | 4.7 | 4.7 | 3.8 |
| hsa-miR-0520d-5p | 4.4 | 3.7 | 5.0 | 0.0 | 5.5 | 4.9 | 4.0 | 2.5 | 3.9 |
| hsa-miR-1182 | 3.9 | 3.3 | 4.3 | 0.0 | 5.8 | 5.8 | 3.5 | 3.0 | 3.4 |
| hsa-miR-6837-5p | 4.9 | 5.1 | 4.6 | 0.0 | 4.6 | 5.0 | 4.9 | 4.6 | 4.4 |
| hsa -miR -0098-3p | 4.7 | 4.7 | 4.7 | 2.5 | 5.0 | 4.7 | 5.2 | 3.5 | 4.5 |
| hsa-miR-3650 | 4.7 | 5.4 | 3.5 | 3.4 | 5.0 | 4.3 | 5.4 | 4.5 | 4.7 |
| hsa-miR-0130b-5p | 4.2 | 5.0 | 4.8 | 3.7 | 5.3 | 5.5 | 5.7 | 4.6 | 5.4 |
| hsa-miR-4701-3p | 4.0 | 4.9 | 0.0 | 0.0 | 5.1 | 5.5 | 4.9 | 4.8 | 4.2 |
| hsa-miR-1273g-3p | 4.4 | 5.1 | 3.8 | 6.8 | 4.7 | 5.8 | 5.4 | 6.2 | 3.7 |
| hsa-miR-3689a-3p | 4.1 | 4.7 | 4.0 | 0.0 | 4.9 | 4.9 | 0.0 | 4.1 | 4.2 |
| hsa-miR-0187-3p | 3.7 | 5.4 | 3.4 | 3.4 | 4.9 | 3.9 | 4.7 | 4.3 | 4.9 |
| hsa-miR-5100 | 4.5 | 7.0 | 6.0 | 4.7 | 5.3 | 4.9 | 4.8 | 6.3 | 4.6 |
| hsa-miR-6514-3p | 3.8 | 4.9 | 3.4 | 3.0 | 4.8 | 4.9 | 4.8 | 4.2 | 4.4 |
| hsa-miR-0133a-3p | 4.4 | 4.7 | 4.6 | 3.4 | 4.9 | 4.2 | 5.5 | 3.0 | 4.4 |
| hsa-miR-0519d-3p | 5.2 | 5.0 | 4.5 | 4.0 | 5.2 | 5.1 | 4.9 | 4.1 | 4.9 |
| hsa-miR-0326 | 5.0 | 5.1 | 5.1 | 7.0 | 4.8 | 4.7 | 4.1 | 4.2 | 3.7 |
| hsa-miR-6766-5p | 5.0 | 5.7 | 5.2 | 3.9 | 5.3 | 5.6 | 5.6 | 6.1 | 5.5 |
| hsa-miR-4732-3p | 4.4 | 5.2 | 4.0 | 3.7 | 5.5 | 5.3 | 4.8 | 4.6 | 4.7 |
| hsa-miR-3189-5p | 5.0 | 4.9 | 4.1 | 3.3 | 5.0 | 4.8 | 5.2 | 3.8 | 3.9 |
| hsa -miR -5088-3p | 5.1 | 5.2 | 5.0 | 3.7 | 4.9 | 5.3 | 4.3 | 4.7 | 4.8 |
| hsa-miR-4648 | 4.9 | 5.5 | 6.3 | 6.7 | 5.9 | 6.0 | 4.6 | 6.4 | 3.5 |
| hsa-miR-0183-3p | 4.9 | 3.9 | 5.3 | 0.0 | 5.4 | 4.3 | 3.6 | 3.2 | 3.7 |
| hsa-miR-8073 | 5.1 | 5.7 | 6.3 | 4.0 | 5.7 | 6.1 | 5.4 | 5.6 | 4.8 |
| hsa -miR -6508-5p | 5.4 | 5.2 | 5.1 | 3.6 | 5.0 | 5.3 | 5.3 | 4.2 | 4.6 |
| hsa-miR-6830-3p | 4.9 | 4.6 | 4.7 | 5.2 | 4.4 | 4.7 | 5.1 | 4.0 | 4.4 |
| hsa-miR-0409-3p | 4.8 | 4.6 | 4.9 | 2.8 | 5.4 | 5.1 | 4.6 | 4.4 | 4.3 |
| hsa-miR-0564 | 3.6 | 5.1 | 3.0 | 3.7 | 4.9 | 5.5 | 4.5 | 4.4 | 4.5 |
| hsa-miR-4533 | 4.4 | 5.5 | 4.9 | 7.0 | 5.3 | 5.6 | 5.5 | 5.5 | 5.3 |
| hsa-miR-0134-5p | 6.2 | 5.3 | 5.7 | 3.6 | 5.4 | 5.8 | 5.1 | 3.8 | 5.0 |
| hsa-miR-4312 | 5.4 | 5.2 | 5.1 | 3.8 | 5.4 | 5.1 | 5.4 | 4.6 | 4.6 |
| hsa-miR-6509-3p | 4.5 | 5.2 | 3.7 | 6.6 | 4.4 | 4.9 | 5.0 | 4.4 | 4.0 |
| hsa-miR-7152-5p | 4.9 | 5.6 | 3.9 | 3.5 | 5.0 | 5.4 | 5.2 | 5.0 | 4.5 |
| hsa-miR-0346 | 4.8 | 5.2 | 4.6 | 4.3 | 5.3 | 5.4 | 4.5 | 4.6 | 4.7 |
| hsa-miR-3653-5p | 4.9 | 5.2 | 3.8 | 0.0 | 4.9 | 4.6 | 4.7 | 3.9 | 3.8 |
| hsa-miR-6775-3p | 5.3 | 5.1 | 5.1 | 3.8 | 5.3 | 5.1 | 5.4 | 4.6 | 4.1 |
| hsa-miR-0664a-3p | 5.3 | 4.9 | 4.8 | 2.5 | 4.9 | 5.0 | 5.5 | 4.4 | 4.7 |
| hsa-miR-6859-5p | 4.1 | 5.0 | 3.5 | 3.2 | 4.8 | 4.5 | 5.0 | 4.3 | 4.0 |
| hsa-miR-0632 | 5.1 | 5.4 | 3.7 | 3.9 | 5.1 | 5.1 | 4.8 | 3.8 | 4.3 |
| hsa-miR-6740-5p | 4.4 | 4.9 | 4.1 | 6.8 | 4.1 | 5.0 | 4.2 | 4.0 | 3.4 |
| hsa-miR-1227-3p | 4.9 | 4.4 | 4.4 | 4.1 | 4.6 | 4.7 | 5.5 | 4.3 | 3.3 |
| hsa-miR-5193 | 5.0 | 5.6 | 4.3 | 2.4 | 4.9 | 5.3 | 5.4 | 4.3 | 4.5 |
| hsa-miR-0214-3p | 4.7 | 5.1 | 4.8 | 3.6 | 4.9 | 4.6 | 5.0 | 4.1 | 4.9 |
| hsa-miR-6072 | 3.9 | 5.4 | 0.0 | 2.6 | 5.2 | 3.5 | 4.6 | 4.7 | 4.6 |
| hsa-miR-6133 | 5.7 | 4.9 | 6.0 | 3.3 | 6.5 | 5.7 | 4.1 | 3.7 | 0.0 |
| hsa-miR-4722-3p | 5.3 | 5.8 | 5.3 | 2.8 | 5.3 | 5.7 | 5.2 | 4.7 | 4.9 |
| hsa-miR-2392 | 4.9 | 5.4 | 5.3 | 4.0 | 5.8 | 6.0 | 4.6 | 5.2 | 4.0 |
| hsa -miR -3158-5p | 7.1 | 6.9 | 6.3 | 4.0 | 5.8 | 6.0 | 5.4 | 4.4 | 3.4 |
| hsa-miR-6788-3p | 5.1 | 5.1 | 5.2 | 6.3 | 4.5 | 5.3 | 4.7 | 4.4 | 4.0 |
| hsa-miR-4769-5p | 4.3 | 5.1 | 4.3 | 0.0 | 5.4 | 5.3 | 4.4 | 4.3 | 4.6 |
| hsa-miR-3190-5p | 5.1 | 4.4 | 4.7 | 3.4 | 4.6 | 4.5 | 5.1 | 4.0 | 4.1 |
| hsa-miR-6734-3p | 4.8 | 5.5 | 4.6 | 6.0 | 5.1 | 4.9 | 5.3 | 4.3 | 4.5 |
| hsa-miR-0466 | 3.8 | 5.4 | 2.1 | 3.2 | 4.9 | 5.3 | 4.9 | 4.5 | 4.0 |
| hsa-miR-3187-5p | 4.9 | 5.9 | 5.7 | 4.5 | 6.3 | 6.4 | 4.6 | 5.6 | 4.9 |
| hsa-miR-4297 | 4.6 | 5.4 | 4.8 | 0.0 | 5.6 | 4.6 | 5.5 | 4.7 | 4.2 |
| hsa -miR -6824-3p | 5.3 | 5.5 | 4.9 | 3.0 | 5.2 | 5.4 | 5.4 | 4.8 | 4.7 |
| hsa-miR-6734-5p | 4.9 | 4.8 | 5.8 | 5.5 | 6.4 | 5.4 | 4.6 | 3.7 | 0.0 |
| hsa-miR-0139-3p | 5.5 | 5.5 | 6.3 | 5.9 | 6.1 | 6.5 | 4.8 | 6.3 | 4.5 |
| hsa-miR-3192-3p | 4.8 | 5.2 | 4.4 | 3.8 | 4.8 | 5.3 | 4.8 | 4.7 | 4.3 |
| hsa-miR-6799-3p | 5.1 | 4.6 | 5.0 | 3.0 | 5.3 | 5.2 | 4.9 | 4.4 | 4.4 |
| hsa-miR-4329 | 5.5 | 5.4 | 5.0 | 3.9 | 5.1 | 5.5 | 4.9 | 4.6 | 4.0 |
| hsa-miR-0199b-5p | 4.4 | 4.6 | 4.5 | 2.8 | 5.0 | 4.3 | 4.5 | 3.0 | 4.2 |
| hsa-miR-0885-5p | 5.1 | 5.4 | 4.5 | 3.7 | 5.5 | 5.0 | 4.9 | 4.6 | 4.5 |
| hsa-miR-5589-5p | 4.1 | 5.6 | 4.5 | 3.3 | 5.7 | 5.7 | 5.2 | 4.7 | 4.4 |
| hsa-miR-4539 | 4.9 | 5.7 | 5.1 | 3.9 | 5.6 | 5.2 | 5.1 | 5.4 | 4.3 |
| hsa-miR-0223-3p | 5.0 | 5.2 | 4.6 | 3.2 | 5.1 | 4.8 | 5.4 | 3.9 | 4.4 |
| hsa-miR-6772-3p | 4.4 | 5.5 | 4.1 | 6.5 | 4.7 | 5.2 | 5.0 | 4.5 | 3.9 |
| hsa-miR-6841-3p | 5.3 | 5.1 | 5.1 | 0.0 | 5.0 | 5.0 | 5.4 | 4.3 | 3.9 |
| hsa -miR -4632-3p | 5.3 | 5.4 | 5.5 | 7.4 | 4.8 | 5.1 | 5.6 | 4.8 | 4.7 |
| hsa-miR-4523 | 6.5 | 5.6 | 6.3 | 5.4 | 4.6 | 5.9 | 5.2 | 3.6 | 4.5 |
| hsa -miR -6868-3p | 4.4 | 5.3 | 4.3 | 8.1 | 5.1 | 5.1 | 5.2 | 4.7 | 4.4 |
| hsa-miR-6810-5p | 4.7 | 5.6 | 4.6 | 3.4 | 5.2 | 5.5 | 5.0 | 5.2 | 4.2 |
| hsa-miR-3620-3p | 5.1 | 5.4 | 4.8 | 2.6 | 5.3 | 5.1 | 5.4 | 4.7 | 4.6 |
| hsa-miR-1250-3p | 5.4 | 5.3 | 4.7 | 0.0 | 5.1 | 5.0 | 4.6 | 4.9 | 4.9 |
| hsa-miR-0595 | 3.8 | 5.5 | 3.4 | 3.0 | 5.0 | 4.1 | 5.0 | 4.6 | 4.7 |
| hsa-miR-0513a-5p | 5.4 | 3.7 | 6.5 | 2.7 | 7.5 | 4.9 | 3.6 | 0.0 | 3.6 |
| hsa-miR-4787-3p | 5.1 | 5.3 | 5.3 | 7.0 | 4.7 | 5.4 | 5.8 | 4.9 | 4.2 |
| hsa-miR-0518b | 5.2 | 4.8 | 5.0 | 3.0 | 5.1 | 4.8 | 5.0 | 3.2 | 5.0 |
| hsa-miR-6849-5p | 3.9 | 5.1 | 5.1 | 3.7 | 5.8 | 6.0 | 5.2 | 4.4 | 3.7 |
| hsa -miR -0320b | 5.1 | 5.0 | 2.6 | 3.0 | 3.2 | 4.6 | 4.6 | 3.2 | 3.5 |
| hsa -miR -3064-5p | 5.5 | 5.3 | 5.3 | 3.4 | 5.5 | 5.8 | 6.0 | 5.1 | 5.1 |
| hsa-miR-3166 | 0.0 | 0.0 | 0.0 | 3.4 | 5.5 | 5.8 | 0.0 | 0.0 | 0.0 |
| hsa-miR-4481 | 4.9 | 5.6 | 4.8 | 3.6 | 5.8 | 5.3 | 4.8 | 5.4 | 4.3 |
| hsa-miR-3175 | 6.1 | 6.1 | 5.4 | 4.3 | 4.8 | 6.7 | 6.2 | 5.8 | 5.5 |
| hsa-miR-3180-5p | 5.3 | 5.2 | 5.5 | 4.4 | 5.5 | 5.6 | 5.6 | 5.1 | 5.0 |
| hsa-miR-0615-3p | 4.8 | 5.2 | 4.8 | 5.9 | 3.7 | 4.8 | 4.6 | 4.4 | 3.4 |
| hsa-miR-4449 | 4.9 | 6.3 | 6.5 | 6.3 | 6.4 | 7.4 | 5.7 | 6.6 | 4.4 |
| hsa-miR-4496 | 4.9 | 4.7 | 5.1 | 2.6 | 5.7 | 5.3 | 4.3 | 3.3 | 3.8 |
| hsa-miR-0198 | 4.7 | 4.3 | 5.7 | 0.0 | 6.7 | 4.5 | 0.0 | 2.5 | 3.5 |
| hsa-miR-0026b-3p | 4.7 | 5.0 | 4.4 | 3.6 | 4.7 | 4.7 | 5.2 | 4.3 | 4.7 |
| hsa-miR-0675-3p | 5.0 | 5.9 | 5.1 | 3.3 | 4.9 | 5.5 | 5.1 | 5.0 | 5.1 |
| hsa-miR-0765 | 5.5 | 5.2 | 4.7 | 2.4 | 5.5 | 5.1 | 5.0 | 3.8 | 3.1 |
| hsa-miR-0487a-5p | 4.5 | 5.1 | 4.4 | 3.0 | 5.2 | 5.2 | 4.9 | 4.6 | 4.3 |
| hsa-miR-5189-5p | 3.7 | 5.3 | 4.7 | 4.0 | 5.2 | 5.4 | 4.3 | 4.5 | 3.3 |
| hsa-miR-6871-5p | 4.2 | 5.5 | 4.7 | 6.0 | 5.5 | 6.3 | 4.8 | 4.8 | 4.2 |
| hsa-miR-0575 | 5.3 | 5.4 | 4.9 | 4.0 | 5.5 | 5.8 | 5.2 | 5.1 | 4.7 |
| hsa-miR-1910-5p | 5.0 | 5.3 | 5.4 | 4.3 | 5.4 | 5.6 | 5.6 | 4.8 | 4.7 |
| hsa-miR-3139 | 0.0 | 0.0 | 0.0 | 4.3 | 5.4 | 5.6 | 0.0 | 0.0 | 0.0 |
| hsa-miR-6762-3p | 4.8 | 5.8 | 4.6 | 3.9 | 5.7 | 5.0 | 5.2 | 5.1 | 4.5 |
| hsa-miR-4251 | 5.3 | 3.1 | 6.4 | 0.0 | 7.6 | 5.8 | 3.0 | 0.0 | 2.9 |
| hsa-miR-6790-3p | 5.5 | 5.6 | 5.4 | 4.7 | 5.6 | 5.4 | 6.2 | 5.0 | 4.8 |
| hsa-miR-6751-5p | 4.8 | 5.7 | 4.5 | 5.2 | 5.4 | 5.9 | 4.9 | 5.3 | 4.4 |
| hsa-miR-3173-3p | 5.3 | 3.8 | 5.6 | 3.7 | 4.5 | 4.3 | 3.4 | 4.1 | 4.0 |
| hsa-miR-6783-3p | 4.8 | 5.5 | 4.5 | 3.2 | 5.3 | 5.3 | 5.5 | 4.8 | 4.5 |
| hsa-miR-6793-5p | 4.1 | 5.4 | 3.4 | 3.2 | 5.5 | 4.6 | 4.9 | 4.7 | 4.8 |
| hsa-miR-1193 | 5.5 | 5.5 | 6.0 | 4.4 | 5.6 | 6.1 | 5.2 | 5.1 | 5.4 |
| hsa-miR-0637 | 5.0 | 5.8 | 5.3 | 3.7 | 5.5 | 5.7 | 5.5 | 5.2 | 4.7 |
| hsa-miR-6738-3p | 4.5 | 5.4 | 3.7 | 3.5 | 5.1 | 4.7 | 5.2 | 5.1 | 4.6 |
| hsa-miR-0223-5p | 5.6 | 5.1 | 4.5 | 3.1 | 4.8 | 5.0 | 5.9 | 3.9 | 5.4 |
| hsa-miR-3940-3p | 5.0 | 5.7 | 4.9 | 3.8 | 5.3 | 5.2 | 5.0 | 5.1 | 5.0 |
| hsa-miR-6893-3p | 5.1 | 5.6 | 5.2 | 4.2 | 5.5 | 5.1 | 5.4 | 5.1 | 4.5 |
| hsa -miR -0342-5p | 5.1 | 4.3 | 5.2 | 3.3 | 6.3 | 5.9 | 5.3 | 2.7 | 4.2 |
| hsa-miR-1307-3p | 4.4 | 5.7 | 5.6 | 4.8 | 6.3 | 6.5 | 5.5 | 5.5 | 4.8 |
| hsa-miR-3121-5p | 0.0 | 0.0 | 0.0 | 4.8 | 6.3 | 6.5 | 0.0 | 0.0 | 0.0 |
| hsa-miR-6131 | 5.0 | 6.2 | 6.3 | 4.9 | 6.2 | 7.2 | 4.7 | 6.4 | 4.3 |
| hsa-miR-0485-3p | 4.6 | 5.3 | 3.9 | 3.0 | 5.5 | 5.2 | 5.7 | 4.5 | 4.9 |
| hsa -miR -5088-5p | 5.4 | 5.8 | 5.0 | 4.4 | 5.4 | 5.5 | 5.6 | 5.3 | 4.5 |
| hsa-miR-0642a-5p | 5.5 | 5.3 | 4.9 | 6.6 | 4.9 | 5.1 | 5.1 | 4.3 | 3.7 |
| hsa-miR-1470 | 5.4 | 5.4 | 5.5 | 4.6 | 5.5 | 5.6 | 6.0 | 5.6 | 5.5 |
| hsa-miR-3129-3p | 2.7 | 3.3 | 0.0 | 4.6 | 5.5 | 5.6 | 3.8 | 2.3 | 0.0 |
| hsa -miR -5002-3p | 4.0 | 5.6 | 3.8 | 3.3 | 5.2 | 4.5 | 5.1 | 4.9 | 4.8 |
| hsa-miR-0664b-3p | 5.1 | 5.2 | 5.1 | 3.4 | 5.2 | 5.3 | 4.9 | 4.6 | 4.2 |
| hsa-miR-4322 | 6.0 | 6.1 | 6.6 | 4.7 | 5.9 | 6.0 | 5.8 | 5.9 | 5.3 |
| hsa-miR-4535 | 4.3 | 6.0 | 5.1 | 4.3 | 5.9 | 6.0 | 5.7 | 5.7 | 5.4 |
| hsa-miR-1233-3p | 5.6 | 5.6 | 6.1 | 3.8 | 5.6 | 5.6 | 5.6 | 4.9 | 5.1 |
| hsa-miR-6825-3p | 5.2 | 5.6 | 5.5 | 4.6 | 5.7 | 5.9 | 5.3 | 6.2 | 5.0 |
| hsa-miR-6769b-3p | 5.4 | 5.4 | 4.9 | 3.4 | 5.0 | 5.3 | 5.5 | 4.5 | 4.8 |
| hsa-miR-4290 | 5.1 | 5.4 | 5.2 | 2.4 | 5.3 | 5.5 | 4.6 | 4.3 | 4.5 |
| hsa-miR-0874-3p | 5.6 | 5.9 | 5.4 | 6.1 | 5.7 | 5.7 | 5.6 | 5.3 | 5.2 |
| hsa -miR -0532-3p | 5.6 | 5.8 | 5.2 | 3.5 | 5.5 | 5.5 | 5.1 | 5.1 | 4.7 |
| hsa-miR-6744-3p | 5.9 | 5.3 | 5.3 | 2.9 | 5.0 | 5.4 | 5.2 | 4.5 | 4.8 |
| hsa-miR-0030d-5p | 5.2 | 5.0 | 5.0 | 4.0 | 5.7 | 5.2 | 5.6 | 4.9 | 5.1 |
| hsa-miR-3652 | 4.6 | 5.8 | 5.5 | 4.1 | 7.4 | 7.2 | 5.3 | 5.6 | 4.4 |
| hsa-miR-4710 | 5.6 | 5.7 | 5.0 | 5.0 | 5.6 | 6.0 | 5.7 | 5.1 | 4.9 |
| hsa-miR-5195-5p | 5.5 | 5.5 | 5.8 | 5.2 | 5.3 | 5.1 | 6.0 | 3.9 | 4.5 |
| hsa-miR-6812-3p | 5.6 | 5.3 | 5.3 | 4.1 | 5.2 | 5.5 | 5.6 | 4.4 | 4.2 |
| hsa-let-7b-3p | 5.3 | 5.4 | 5.3 | 6.4 | 5.0 | 5.3 | 5.4 | 4.4 | 4.5 |
| hsa-miR-0483-3p | 5.3 | 5.5 | 5.2 | 4.5 | 5.7 | 5.8 | 5.2 | 5.0 | 4.9 |
| hsa-miR-7106-3p | 5.5 | 5.5 | 5.2 | 3.8 | 5.3 | 5.3 | 5.5 | 5.0 | 4.7 |
| hsa-miR-0199a-5p | 4.9 | 5.3 | 4.4 | 2.7 | 4.8 | 4.8 | 5.0 | 4.0 | 4.6 |
| hsa-miR-0188-5p | 5.8 | 5.8 | 5.7 | 3.6 | 5.7 | 5.8 | 5.9 | 4.6 | 5.6 |
| hsa-miR-6737-3p | 5.3 | 5.3 | 4.9 | 3.7 | 5.5 | 5.1 | 5.7 | 4.6 | 4.1 |
| hsa-miR-6796-5p | 4.9 | 6.0 | 5.4 | 3.7 | 5.7 | 5.8 | 5.0 | 5.5 | 4.9 |
| hsa-miR-7843-5p | 5.9 | 6.1 | 5.3 | 2.5 | 5.7 | 5.4 | 4.8 | 4.2 | 3.8 |
| hsa-miR-4498 | 4.8 | 6.2 | 5.2 | 5.6 | 5.7 | 5.7 | 5.1 | 5.6 | 4.6 |
| hsa-miR-5010-3p | 5.2 | 5.4 | 5.0 | 3.3 | 5.1 | 5.2 | 5.4 | 4.3 | 4.6 |
| hsa-miR-8060 | 4.8 | 4.7 | 4.1 | 0.0 | 4.2 | 4.9 | 4.3 | 4.0 | 3.6 |
| hsa-miR-0487b-5p | 4.9 | 5.3 | 4.8 | 3.7 | 5.1 | 5.1 | 4.9 | 4.6 | 4.4 |
| hsa-miR-6831-3p | 5.7 | 5.6 | 5.5 | 0.0 | 5.4 | 5.6 | 4.9 | 4.7 | 4.4 |
| hsa -miR -0320a | 5.9 | 5.2 | 5.3 | 3.2 | 5.0 | 5.2 | 5.6 | 3.9 | 5.1 |
| hsa-miR-6771-3p | 5.5 | 5.3 | 5.5 | 4.2 | 5.1 | 4.9 | 5.9 | 4.3 | 4.4 |
| hsa-miR-6511a-3p | 5.1 | 5.8 | 5.1 | 5.5 | 5.8 | 5.6 | 5.5 | 5.4 | 5.4 |
| hsa-miR-0374c-3p | 6.6 | 5.3 | 5.3 | 0.0 | 4.6 | 5.7 | 5.3 | 4.2 | 5.0 |
| hsa-miR-8052 | 6.0 | 6.0 | 5.9 | 3.9 | 5.8 | 6.1 | 5.7 | 5.6 | 5.0 |
| hsa-miR-4531 | 0.0 | 2.4 | 0.0 | 0.0 | 3.7 | 2.6 | 0.0 | 0.0 | 3.2 |
| hsa-miR-6790-5p | 5.8 | 5.9 | 6.2 | 4.1 | 5.7 | 6.5 | 5.0 | 5.8 | 4.5 |
| hsa-miR-6883-3p | 4.4 | 5.4 | 4.3 | 3.5 | 5.1 | 5.2 | 5.0 | 4.8 | 4.7 |
| hsa-miR-4655-3p | 4.8 | 5.9 | 5.9 | 5.1 | 6.0 | 4.7 | 7.9 | 5.9 | 7.2 |
| hsa-miR-5189-3p | 5.1 | 5.7 | 4.9 | 3.7 | 5.6 | 5.0 | 5.6 | 5.2 | 5.1 |
| hsa-miR-6753-3p | 4.7 | 5.8 | 4.4 | 3.8 | 5.4 | 5.8 | 5.5 | 5.1 | 4.4 |
| hsa-miR-4455 | 5.1 | 6.0 | 5.0 | 3.7 | 5.9 | 5.5 | 5.4 | 5.3 | 4.8 |
| hsa-miR-3605-3p | 5.2 | 5.9 | 5.0 | 3.3 | 5.4 | 5.2 | 5.8 | 5.0 | 4.6 |
| hsa-miR-4642 | 5.3 | 5.6 | 5.0 | 2.6 | 5.4 | 4.9 | 5.7 | 4.7 | 4.6 |
| hsa-miR-7160-5p | 4.9 | 6.0 | 5.1 | 4.5 | 5.9 | 6.2 | 6.0 | 5.8 | 5.3 |
| hsa -miR -5008-5p | 6.2 | 6.0 | 6.2 | 4.8 | 6.1 | 6.0 | 6.2 | 5.8 | 5.8 |
| hsa-miR-1292-3p | 5.4 | 5.8 | 5.7 | 4.8 | 5.9 | 5.7 | 6.3 | 5.6 | 6.1 |
| hsa-miR-6753-5p | 5.3 | 6.1 | 5.7 | 4.6 | 6.1 | 5.7 | 5.6 | 5.6 | 5.2 |
| hsa-miR-4649-3p | 5.6 | 5.6 | 5.2 | 3.8 | 5.5 | 5.4 | 5.7 | 4.7 | 5.0 |
| hsa-miR-7846-3p | 6.6 | 6.1 | 6.3 | 3.3 | 5.9 | 6.2 | 5.5 | 4.9 | 4.3 |
| hsa-miR-0936 | 5.9 | 5.8 | 6.8 | 3.6 | 7.0 | 4.9 | 6.0 | 3.3 | 5.1 |
| hsa -miR -0504-3p | 5.5 | 5.9 | 5.5 | 3.8 | 6.1 | 6.2 | 5.0 | 6.0 | 5.4 |
| hsa-miR-4746-3p | 5.7 | 6.5 | 6.3 | 5.6 | 6.3 | 7.2 | 6.0 | 6.8 | 5.5 |
| hsa-miR-6833-3p | 5.5 | 5.7 | 5.4 | 3.7 | 5.3 | 5.6 | 5.7 | 5.2 | 4.9 |
| hsa-miR-4279 | 5.8 | 6.1 | 6.0 | 3.7 | 6.1 | 6.4 | 5.7 | 5.5 | 4.8 |
| hsa-miR-6776-5p | 4.8 | 5.4 | 5.0 | 3.0 | 6.0 | 6.0 | 4.3 | 5.6 | 4.3 |
| hsa-miR-6748-3p | 5.0 | 5.8 | 4.8 | 7.0 | 5.3 | 5.6 | 5.5 | 4.8 | 4.9 |
| hsa-miR-4721 | 5.2 | 5.7 | 4.7 | 4.2 | 5.9 | 6.2 | 5.7 | 5.1 | 5.2 |
| hsa-miR-0185-3p | 4.9 | 5.8 | 5.7 | 4.7 | 6.1 | 6.7 | 5.5 | 5.8 | 5.5 |
| hsa-miR-6735-3p | 5.8 | 5.5 | 5.5 | 4.2 | 5.8 | 5.9 | 5.3 | 4.8 | 5.0 |
| hsa-miR-4287 | 6.5 | 6.0 | 5.3 | 3.5 | 5.1 | 5.9 | 5.3 | 3.9 | 3.4 |
| hsa-miR-3173-5p | 4.6 | 5.8 | 4.0 | 3.9 | 5.7 | 5.0 | 5.3 | 5.3 | 5.0 |
| hsa-miR-6782-3p | 5.8 | 5.7 | 5.6 | 5.1 | 5.8 | 5.6 | 6.0 | 5.0 | 4.9 |
| hsa-miR-7112-5p | 5.9 | 6.1 | 6.4 | 5.4 | 5.9 | 6.4 | 5.7 | 5.8 | 5.5 |
| hsa-miR-6794-3p | 5.8 | 5.8 | 5.4 | 4.0 | 5.6 | 5.6 | 5.8 | 5.0 | 5.2 |
| hsa-miR-6757-3p | 5.9 | 5.6 | 5.6 | 3.2 | 5.5 | 5.3 | 6.0 | 4.7 | 5.1 |
| hsa-miR-3622a-3p | 5.0 | 5.7 | 4.6 | 4.2 | 5.9 | 5.8 | 5.5 | 4.9 | 5.1 |
| hsa-miR-6726-3p | 5.7 | 6.1 | 5.8 | 6.0 | 5.7 | 5.8 | 5.9 | 5.3 | 5.1 |
| hsa-miR-0642b-5p | 5.5 | 5.7 | 4.5 | 0.0 | 5.1 | 5.1 | 5.2 | 4.6 | 4.3 |
| hsa-miR-6809-3p | 5.0 | 5.4 | 3.9 | 4.0 | 5.6 | 5.8 | 5.2 | 4.9 | 5.2 |
| hsa-miR-6857-5p | 5.5 | 7.1 | 6.9 | 5.7 | 6.6 | 7.1 | 5.4 | 7.6 | 4.6 |
| hsa-miR-0197-3p | 5.4 | 5.9 | 5.2 | 3.8 | 6.0 | 5.4 | 5.5 | 5.2 | 5.5 |
| hsa-miR-0149-5p | 5.4 | 5.8 | 5.5 | 4.2 | 5.8 | 5.7 | 5.5 | 5.0 | 5.2 |
| hsa-miR-4485-5p | 5.6 | 6.2 | 5.6 | 4.3 | 5.7 | 6.3 | 6.0 | 5.5 | 5.2 |
| hsa-miR-0939-3p | 5.6 | 5.7 | 5.5 | 7.4 | 5.7 | 5.7 | 5.4 | 5.1 | 4.9 |
| hsa-miR-6780b-3p | 5.3 | 6.0 | 4.9 | 3.4 | 5.6 | 5.3 | 5.8 | 5.4 | 4.3 |
| hsa-miR-6748-5p | 5.1 | 4.7 | 5.8 | 3.5 | 6.6 | 6.1 | 5.2 | 4.8 | 4.9 |
| hsa-miR-6751-3p | 5.7 | 5.9 | 5.1 | 4.0 | 5.4 | 5.8 | 5.7 | 5.0 | 5.2 |
| hsa-miR-4736 | 5.7 | 6.8 | 7.1 | 5.2 | 6.6 | 6.6 | 5.6 | 6.4 | 5.6 |
| hsa-miR-6778-3p | 5.1 | 5.9 | 4.5 | 4.7 | 5.5 | 6.0 | 5.7 | 5.4 | 4.9 |
| hsa-miR-6827-3p | 5.7 | 5.1 | 5.1 | 3.7 | 5.4 | 5.3 | 5.3 | 4.5 | 4.1 |
| hsa-miR-5698 | 6.3 | 6.5 | 6.3 | 4.0 | 6.7 | 6.7 | 5.4 | 4.9 | 5.0 |
| hsa-miR-0371b-5p | 5.4 | 5.9 | 6.4 | 4.6 | 6.4 | 6.9 | 5.2 | 6.0 | 5.4 |
| hsa-miR-4714-5p | 5.5 | 5.2 | 5.3 | 4.1 | 5.4 | 5.0 | 5.8 | 4.4 | 4.7 |
| hsa-miR-6772-5p | 5.0 | 6.0 | 5.4 | 4.3 | 5.6 | 5.3 | 5.9 | 5.4 | 4.9 |
| hsa-miR-4462 | 7.1 | 6.2 | 5.9 | 3.9 | 5.3 | 6.4 | 5.6 | 4.7 | 4.7 |
| hsa-miR-6793-3p | 5.8 | 5.5 | 5.9 | 4.0 | 5.9 | 5.3 | 6.3 | 5.0 | 5.3 |
| hsa-miR-0328-3p | 5.6 | 6.0 | 5.4 | 4.5 | 5.7 | 5.7 | 5.8 | 5.2 | 5.2 |
| hsa-miR-3202 | 5.3 | 5.6 | 4.7 | 0.0 | 4.8 | 5.0 | 3.4 | 4.1 | 0.0 |
| hsa-miR-6867-5p | 6.2 | 6.0 | 5.3 | 3.5 | 5.7 | 5.3 | 6.1 | 4.9 | 4.8 |
| hsa -miR -0302c-5p | 5.0 | 4.6 | 5.5 | 3.8 | 6.0 | 5.4 | 4.9 | 3.6 | 4.3 |
| hsa-miR-6881-5p | 5.7 | 6.1 | 5.6 | 0.0 | 5.6 | 5.5 | 4.8 | 3.5 | 4.7 |
| hsa-miR-0574-5p | 5.2 | 5.9 | 4.9 | 4.6 | 6.0 | 5.5 | 5.8 | 5.4 | 5.2 |
| hsa-miR-1260b | 5.4 | 5.9 | 5.2 | 4.4 | 5.9 | 6.0 | 5.4 | 5.4 | 5.3 |
| hsa -miR -6834-3p | 5.6 | 5.8 | 5.2 | 2.8 | 5.7 | 5.8 | 5.4 | 5.0 | 5.0 |
| hsa-miR-4763-5p | 5.5 | 5.9 | 5.6 | 4.6 | 5.6 | 5.8 | 5.8 | 5.6 | 5.1 |
| hsa-miR-7110-3p | 5.7 | 6.0 | 5.3 | 7.5 | 5.6 | 5.9 | 5.0 | 5.1 | 4.7 |
| hsa-miR-1825 | 5.4 | 5.8 | 5.7 | 4.1 | 6.0 | 5.5 | 5.7 | 5.0 | 5.2 |
| hsa-miR-3134 | 0.0 | 0.0 | 0.0 | 4.1 | 6.0 | 5.5 | 0.0 | 0.0 | 0.0 |
| hsa-miR-3679-5p | 5.6 | 5.2 | 6.2 | 4.1 | 6.3 | 6.0 | 4.6 | 4.5 | 4.5 |
| hsa-miR-0370-3p | 5.2 | 5.7 | 6.1 | 3.8 | 7.4 | 8.3 | 5.4 | 5.4 | 4.6 |
| hsa-miR-0092a-2-5p | 5.3 | 7.5 | 6.6 | 5.6 | 7.9 | 7.6 | 5.8 | 8.6 | 6.6 |
| hsa-miR-7111-3p | 6.2 | 6.1 | 6.0 | 3.7 | 6.0 | 5.8 | 6.0 | 5.2 | 5.2 |
| hsa-miR-3663-5p | 4.8 | 6.0 | 4.8 | 4.9 | 6.1 | 6.1 | 5.8 | 5.6 | 5.7 |
| hsa-miR-0503-3p | 6.0 | 6.1 | 5.6 | 4.2 | 5.5 | 5.8 | 6.3 | 5.2 | 5.1 |
| hsa-miR-6736-5p | 5.7 | 6.1 | 4.8 | 4.8 | 6.1 | 5.9 | 5.7 | 5.4 | 5.6 |
| hsa-miR-3191-5p | 4.9 | 5.8 | 4.7 | 3.4 | 5.4 | 5.7 | 5.5 | 4.9 | 4.9 |
| hsa-miR-5705 | 6.0 | 5.6 | 5.9 | 4.0 | 5.7 | 5.9 | 5.6 | 5.3 | 5.4 |
| hsa-miR-7977 | 5.4 | 6.0 | 5.1 | 4.4 | 5.8 | 5.2 | 5.6 | 5.6 | 5.5 |
| hsa-miR-6840-5p | 5.6 | 5.6 | 5.8 | 4.0 | 5.7 | 5.7 | 5.8 | 4.8 | 5.1 |
| hsa-miR-6865-3p | 5.7 | 5.9 | 5.5 | 4.4 | 5.8 | 5.9 | 5.7 | 5.3 | 5.1 |
| hsa-miR-6754-3p | 5.3 | 6.0 | 4.7 | 4.0 | 5.9 | 5.5 | 5.5 | 5.6 | 5.4 |
| hsa-miR-6830-5p | 5.7 | 5.5 | 5.4 | 0.0 | 6.6 | 6.2 | 4.7 | 4.4 | 3.2 |
| hsa-miR-6736-3p | 5.1 | 5.9 | 4.6 | 4.1 | 5.9 | 5.3 | 5.6 | 5.3 | 4.8 |
| hsa -miR -6842-5p | 6.4 | 6.2 | 6.1 | 4.4 | 6.5 | 7.1 | 5.4 | 5.9 | 4.3 |
| hsa-miR-6761-3p | 5.4 | 6.2 | 5.0 | 4.1 | 6.0 | 6.1 | 5.7 | 5.4 | 5.2 |
| hsa-miR-1236-3p | 6.1 | 6.0 | 5.9 | 4.2 | 6.1 | 5.8 | 6.0 | 5.4 | 4.8 |
| hsa-miR-5196-3p | 5.7 | 5.9 | 5.6 | 4.5 | 5.8 | 6.0 | 5.9 | 5.4 | 5.4 |
| hsa-miR-1234-3p | 6.3 | 5.7 | 6.3 | 4.2 | 5.5 | 6.0 | 6.2 | 5.3 | 4.9 |
| hsa-miR-3925-5p | 5.9 | 4.3 | 6.1 | 3.8 | 6.4 | 5.6 | 5.0 | 3.7 | 4.4 |
| hsa -miR -6872-5p | 4.4 | 4.8 | 5.6 | 2.5 | 6.2 | 4.9 | 4.3 | 3.3 | 4.2 |
| hsa-miR-4483 | 6.1 | 6.5 | 5.6 | 3.8 | 5.8 | 6.4 | 5.6 | 5.0 | 4.8 |
| hsa-miR-3917 | 5.6 | 5.9 | 5.5 | 6.6 | 5.8 | 6.2 | 5.9 | 6.1 | 5.6 |
| hsa-miR-6877-3p | 5.7 | 6.2 | 5.7 | 4.3 | 5.7 | 5.6 | 5.7 | 5.4 | 5.1 |
| hsa -miR -0520e | 4.2 | 2.0 | 5.9 | 4.7 | 5.9 | 4.2 | 4.1 | 0.0 | 3.6 |
| hsa-miR-4436b-5p | 5.7 | 6.1 | 5.5 | 5.1 | 6.2 | 6.2 | 5.8 | 5.6 | 5.4 |
| hsa-miR-4444 | 4.2 | 3.2 | 4.8 | 0.0 | 5.5 | 4.8 | 3.8 | 0.0 | 3.6 |
| hsa-miR-6759-5p | 5.1 | 6.6 | 6.2 | 5.9 | 6.4 | 6.6 | 5.1 | 7.5 | 5.2 |
| hsa-miR-6511a-5p | 6.0 | 6.5 | 6.4 | 5.2 | 6.6 | 6.6 | 6.3 | 5.9 | 5.3 |
| hsa-miR-4430 | 5.6 | 6.0 | 6.6 | 4.0 | 6.8 | 6.6 | 5.5 | 5.7 | 5.0 |
| hsa-miR-6127 | 5.1 | 6.0 | 5.6 | 4.7 | 6.1 | 6.2 | 5.4 | 5.7 | 4.6 |
| hsa-miR-6740-3p | 5.2 | 6.1 | 5.0 | 6.8 | 5.8 | 5.6 | 5.8 | 5.0 | 4.8 |
| hsa-miR-6747-3p | 5.4 | 6.0 | 5.1 | 4.3 | 5.7 | 5.4 | 5.7 | 5.4 | 5.4 |
| hsa-miR-0133b | 5.7 | 5.4 | 5.5 | 4.0 | 6.1 | 5.6 | 6.0 | 4.6 | 5.5 |
| hsa-miR-4440 | 4.8 | 6.1 | 4.8 | 4.7 | 6.2 | 6.1 | 5.8 | 5.8 | 5.4 |
| hsa -miR -0548q | 5.9 | 6.1 | 6.8 | 4.4 | 5.8 | 6.1 | 5.9 | 6.1 | 5.9 |
| hsa-miR-6741-3p | 5.7 | 6.2 | 5.5 | 4.2 | 5.7 | 5.5 | 6.0 | 5.2 | 5.1 |
| hsa-miR-6779-3p | 5.7 | 6.1 | 5.2 | 4.0 | 5.8 | 5.6 | 5.8 | 5.1 | 5.3 |
| hsa-miR-3622b-5p | 6.1 | 5.6 | 5.8 | 2.5 | 6.4 | 5.6 | 4.9 | 4.4 | 4.1 |
| hsa-miR-6787-3p | 5.5 | 5.9 | 5.1 | 4.3 | 5.4 | 5.4 | 5.7 | 5.2 | 5.1 |
| hsa-miR-6873-3p | 5.4 | 6.1 | 4.7 | 4.1 | 5.7 | 6.1 | 6.3 | 5.4 | 5.4 |
| hsa-miR-4713-5p | 5.8 | 6.2 | 5.3 | 4.3 | 6.0 | 6.2 | 5.9 | 5.3 | 5.3 |
| hsa-miR-6742-3p | 6.0 | 6.0 | 5.7 | 6.0 | 5.6 | 5.7 | 6.0 | 4.8 | 5.3 |
| hsa-miR-0423-5p | 6.8 | 6.9 | 6.9 | 5.4 | 6.6 | 7.3 | 5.5 | 6.5 | 5.0 |
| hsa -miR -0204-3p | 4.5 | 6.1 | 4.8 | 4.6 | 5.5 | 6.5 | 4.2 | 4.6 | 4.0 |
| hsa-miR-6823-3p | 5.7 | 5.8 | 5.4 | 3.1 | 5.8 | 5.9 | 5.6 | 5.5 | 5.2 |
| hsa-miR-6877-5p | 5.7 | 6.4 | 6.2 | 7.8 | 6.5 | 6.9 | 5.7 | 6.4 | 5.1 |
| hsa-miR-4695-3p | 5.3 | 6.4 | 5.0 | 4.5 | 6.0 | 5.5 | 6.1 | 5.7 | 5.4 |
| hsa-miR-0018b-3p | 5.7 | 5.6 | 5.8 | 4.5 | 5.7 | 5.7 | 6.2 | 5.1 | 5.5 |
| hsa-miR-1260a | 5.6 | 5.8 | 5.3 | 4.1 | 5.8 | 5.6 | 5.6 | 5.4 | 5.5 |
| hsa-miR-6803-3p | 5.7 | 6.0 | 5.6 | 4.0 | 5.9 | 6.0 | 5.8 | 5.6 | 5.7 |
| hsa-miR-0211-3p | 6.2 | 6.4 | 7.1 | 6.0 | 7.4 | 7.5 | 5.3 | 7.1 | 5.1 |
| hsa-miR-6875-3p | 5.4 | 6.1 | 5.4 | 4.4 | 5.9 | 5.6 | 5.9 | 5.6 | 5.4 |
| hsa -miR -6882-3p | 5.4 | 6.2 | 5.0 | 3.4 | 6.1 | 5.5 | 5.7 | 5.6 | 5.3 |
| hsa-miR-8085 | 5.8 | 5.8 | 5.3 | 2.8 | 6.1 | 6.3 | 5.6 | 5.3 | 5.1 |
| hsa-miR-6750-3p | 5.6 | 6.1 | 5.1 | 5.9 | 6.2 | 6.0 | 5.8 | 5.6 | 5.2 |
| hsa-miR-6879-3p | 5.8 | 6.1 | 6.2 | 5.0 | 6.5 | 6.7 | 6.3 | 6.1 | 5.6 |
| hsa-miR-6867-3p | 5.4 | 6.0 | 5.2 | 4.0 | 5.9 | 5.8 | 5.9 | 5.4 | 5.3 |
| hsa-miR-6890-5p | 6.4 | 6.6 | 5.6 | 4.4 | 5.8 | 6.5 | 6.2 | 6.1 | 5.7 |
| hsa -miR -6894-5p | 6.6 | 6.2 | 6.1 | 4.9 | 6.0 | 6.7 | 5.8 | 5.7 | 4.8 |
| hsa-miR-1306-5p | 6.1 | 6.2 | 5.9 | 4.5 | 6.2 | 6.4 | 6.2 | 5.6 | 5.6 |
| hsa-miR-3121-3p | 0.0 | 0.0 | 0.0 | 4.5 | 6.2 | 6.4 | 0.0 | 0.0 | 2.7 |
| hsa -miR -6886-3p | 5.5 | 6.2 | 5.5 | 6.2 | 5.9 | 6.3 | 5.7 | 5.6 | 5.3 |
| hsa-miR-1267 | 5.8 | 5.5 | 5.6 | 4.2 | 5.7 | 5.5 | 6.2 | 4.5 | 5.3 |
| hsa -miR -6862-3p | 5.8 | 6.3 | 5.3 | 6.4 | 5.9 | 5.8 | 5.5 | 5.5 | 5.3 |
| hsa-miR-4701-5p | 5.7 | 5.7 | 5.8 | 4.4 | 5.9 | 5.8 | 6.2 | 5.1 | 5.1 |
| hsa-miR-6792-5p | 6.3 | 6.2 | 6.3 | 5.3 | 6.3 | 6.4 | 6.2 | 6.0 | 5.8 |
| hsa-miR-4698 | 6.0 | 5.6 | 5.9 | 5.0 | 5.6 | 5.5 | 6.0 | 4.6 | 4.7 |
| hsa-miR-4738-3p | 5.4 | 6.2 | 6.0 | 5.4 | 5.8 | 5.7 | 6.0 | 6.2 | 6.1 |
| hsa-miR-4268 | 5.6 | 5.9 | 5.2 | 4.3 | 5.9 | 5.9 | 5.5 | 4.9 | 5.0 |
| hsa-miR-4664-3p | 5.8 | 6.0 | 5.7 | 4.3 | 6.1 | 6.0 | 6.3 | 5.4 | 5.3 |
| hsa-miR-0877-3p | 5.8 | 5.8 | 5.6 | 4.9 | 6.0 | 6.0 | 6.2 | 5.5 | 5.4 |
| hsa-miR-1304-3p | 6.0 | 5.9 | 5.5 | 6.7 | 5.9 | 5.9 | 6.2 | 5.2 | 5.2 |
| hsa-miR-3118 | 0.0 | 0.0 | 0.0 | 6.7 | 5.9 | 5.9 | 0.0 | 0.0 | 0.0 |
| hsa-miR-6870-3p | 6.0 | 6.0 | 5.7 | 6.8 | 6.0 | 5.8 | 6.2 | 5.0 | 5.1 |
| hsa-miR-0574-3p | 5.9 | 6.3 | 5.6 | 4.7 | 6.2 | 6.0 | 6.1 | 5.8 | 5.7 |
| hsa -miR -4646-3p | 6.0 | 6.2 | 5.9 | 3.8 | 5.8 | 5.7 | 5.9 | 5.5 | 5.4 |
| hsa -miR -6884-3p | 6.1 | 6.1 | 6.0 | 7.4 | 5.9 | 6.0 | 6.0 | 5.4 | 5.1 |
| hsa-miR-0658 | 5.8 | 6.4 | 6.3 | 4.6 | 6.5 | 6.5 | 5.3 | 6.0 | 4.8 |
| hsa-miR-3911 | 7.8 | 6.9 | 6.2 | 3.6 | 5.8 | 6.6 | 5.2 | 4.8 | 4.8 |
| hsa-miR-6743-3p | 5.9 | 6.4 | 5.9 | 4.4 | 6.1 | 6.4 | 6.3 | 5.8 | 5.6 |
| hsa-miR-6870-5p | 7.0 | 7.3 | 6.8 | 4.4 | 6.5 | 7.3 | 5.4 | 6.7 | 4.7 |
| hsa-miR-4685-3p | 5.5 | 6.1 | 5.0 | 4.0 | 6.2 | 5.7 | 6.1 | 5.8 | 5.2 |
| hsa-miR-3191-3p | 6.5 | 6.0 | 6.5 | 4.7 | 6.4 | 6.5 | 6.3 | 5.6 | 5.4 |
| hsa-miR-1281 | 6.2 | 6.1 | 6.0 | 4.7 | 6.2 | 6.1 | 6.3 | 5.6 | 5.8 |
| hsa-miR-4747-5p | 6.5 | 6.1 | 6.2 | 0.0 | 6.6 | 6.5 | 5.4 | 5.1 | 4.6 |
| hsa-miR-0887-3p | 5.4 | 7.6 | 7.8 | 6.7 | 7.7 | 7.8 | 6.3 | 7.9 | 6.2 |
| hsa-miR-4700-3p | 5.8 | 6.2 | 5.4 | 3.6 | 6.2 | 5.7 | 6.0 | 5.5 | 5.2 |
| hsa-miR-6727-3p | 6.0 | 6.3 | 6.1 | 4.6 | 6.2 | 6.2 | 5.8 | 5.9 | 5.6 |
| hsa-miR-5699-5p | 6.3 | 5.9 | 6.0 | 4.0 | 5.9 | 6.0 | 6.4 | 4.8 | 5.3 |
| hsa-miR-0498 | 6.9 | 6.8 | 7.2 | 5.3 | 6.8 | 6.6 | 6.8 | 5.9 | 6.4 |
| hsa-miR-0449b-3p | 5.7 | 6.3 | 5.3 | 4.3 | 5.9 | 6.2 | 6.0 | 5.7 | 5.6 |
| hsa-miR-6735-5p | 7.0 | 6.9 | 6.8 | 5.1 | 6.8 | 7.0 | 6.7 | 6.1 | 6.4 |
| hsa-miR-1343-3p | 5.8 | 6.2 | 6.3 | 4.6 | 6.8 | 6.9 | 6.1 | 5.8 | 6.4 |
| hsa -miR -3126-3p | 3.6 | 4.0 | 3.3 | 4.6 | 6.8 | 6.9 | 3.5 | 3.0 | 2.9 |
| hsa-miR-8071 | 4.8 | 6.4 | 5.4 | 4.9 | 6.2 | 6.4 | 5.9 | 6.3 | 4.9 |
| hsa-miR-1587 | 5.4 | 7.1 | 6.5 | 5.6 | 6.7 | 7.3 | 6.1 | 7.6 | 5.6 |
| hsa-miR-3133 | 0.0 | 0.0 | 0.0 | 5.6 | 6.7 | 7.3 | 0.0 | 0.0 | 0.0 |
| hsa-miR-4793-3p | 5.5 | 5.6 | 4.9 | 0.0 | 5.0 | 5.6 | 5.4 | 4.5 | 4.8 |
| hsa-miR-6730-5p | 6.2 | 5.6 | 6.8 | 3.3 | 7.2 | 6.2 | 5.4 | 4.5 | 4.1 |
| hsa-miR-6076 | 5.1 | 6.2 | 5.3 | 4.6 | 6.5 | 7.7 | 4.9 | 5.7 | 4.8 |
| hsa-miR-8089 | 7.0 | 7.0 | 7.0 | 5.0 | 6.7 | 7.1 | 6.5 | 6.4 | 5.7 |
| hsa-miR-6857-3p | 5.8 | 5.7 | 5.5 | 3.9 | 6.0 | 6.1 | 6.1 | 5.1 | 5.1 |
| hsa-miR-6749-3p | 6.5 | 6.2 | 6.3 | 4.4 | 5.9 | 6.1 | 6.1 | 5.5 | 5.2 |
| hsa-miR-6890-3p | 6.3 | 6.1 | 5.9 | 4.0 | 6.0 | 6.0 | 6.2 | 5.5 | 5.6 |
| hsa-miR-4751 | 6.4 | 5.2 | 6.9 | 3.0 | 6.7 | 6.1 | 4.5 | 4.2 | 3.6 |
| hsa-miR-6825-5p | 5.7 | 6.4 | 6.2 | 4.2 | 6.8 | 6.4 | 5.9 | 6.7 | 5.1 |
| hsa-miR-6810-3p | 6.3 | 6.1 | 6.0 | 4.1 | 6.1 | 6.0 | 6.7 | 5.5 | 5.1 |
| hsa-miR-0365a-3p, hsa-miR-365b-3p | 6.5 | 5.9 | 6.0 | 6.1 | 6.3 | 5.8 | 6.6 | 5.1 | 5.6 |
| hsa-miR-3943 | 6.2 | 6.0 | 6.0 | 3.7 | 6.0 | 5.9 | 6.4 | 5.2 | 5.3 |
| hsa-miR-6728-3p | 6.0 | 6.4 | 5.8 | 4.7 | 6.4 | 6.1 | 6.2 | 5.8 | 5.8 |
| hsa-miR-3185 | 5.9 | 8.1 | 7.7 | 6.5 | 8.3 | 8.5 | 5.8 | 7.8 | 5.5 |
| hsa -miR -6894-3p | 5.7 | 6.4 | 5.5 | 6.1 | 6.3 | 6.3 | 6.0 | 5.9 | 5.7 |
| hsa-miR-8485 | 6.1 | 6.4 | 6.0 | 4.5 | 6.2 | 6.6 | 6.1 | 6.0 | 5.9 |
| hsa -miR -6846-5p | 6.7 | 6.6 | 6.4 | 5.0 | 6.5 | 6.9 | 6.7 | 6.3 | 5.9 |
| hsa-miR-4286 | 6.3 | 6.0 | 6.1 | 4.1 | 6.1 | 5.9 | 6.5 | 5.3 | 5.3 |
| hsa-miR-4259 | 6.0 | 5.8 | 6.4 | 3.7 | 6.9 | 6.3 | 5.5 | 4.7 | 5.4 |
| hsa-miR-0208a-5p | 6.4 | 6.7 | 7.0 | 6.8 | 6.7 | 5.9 | 5.8 | 6.4 | 5.0 |
| hsa-miR-6851-3p | 6.3 | 5.9 | 5.9 | 4.4 | 6.3 | 6.1 | 6.2 | 5.3 | 5.7 |
| hsa-miR-4472 | 7.1 | 7.2 | 6.9 | 4.5 | 6.7 | 7.1 | 5.9 | 6.7 | 5.2 |
| hsa-miR-6820-5p | 5.8 | 6.7 | 7.3 | 5.6 | 7.1 | 7.0 | 5.9 | 6.6 | 5.6 |
| hsa-miR-6855-3p | 6.3 | 6.2 | 6.1 | 4.5 | 6.5 | 6.4 | 6.7 | 6.0 | 5.9 |
| hsa-miR-6801-3p | 6.3 | 6.2 | 6.2 | 4.6 | 6.4 | 6.3 | 6.4 | 5.6 | 5.8 |
| hsa-miR-0634 | 5.9 | 5.9 | 5.9 | 4.6 | 6.3 | 5.8 | 6.5 | 5.5 | 5.7 |
| hsa-miR-1229-3p | 6.0 | 6.3 | 5.6 | 7.7 | 6.1 | 6.4 | 5.9 | 5.7 | 5.8 |
| hsa-miR-7109-3p | 5.7 | 6.4 | 5.6 | 4.5 | 6.3 | 5.9 | 6.1 | 5.7 | 5.7 |
| hsa -miR -6804-3p | 6.1 | 6.3 | 5.8 | 5.4 | 6.0 | 5.8 | 6.1 | 5.8 | 5.6 |
| hsa-miR-4723-3p | 6.4 | 6.3 | 6.2 | 6.0 | 6.1 | 6.4 | 6.4 | 5.6 | 5.2 |
| hsa-miR-6742-5p | 5.9 | 6.5 | 6.3 | 6.4 | 6.3 | 6.8 | 5.5 | 6.6 | 5.4 |
| hsa-miR-6511b-3p | 6.2 | 6.4 | 6.1 | 5.1 | 5.7 | 5.9 | 6.2 | 5.5 | 5.5 |
| hsa-miR-6820-3p | 6.2 | 6.1 | 5.9 | 4.2 | 6.0 | 6.2 | 6.7 | 5.3 | 5.2 |
| hsa-miR-2278 | 7.6 | 6.1 | 8.0 | 4.9 | 7.9 | 6.9 | 5.7 | 4.1 | 5.3 |
| hsa-miR-3157-3p | 0.0 | 2.4 | 2.4 | 4.9 | 7.9 | 6.9 | 2.5 | 0.0 | 0.0 |
| hsa-miR-6776-3p | 5.9 | 6.4 | 5.9 | 4.6 | 6.5 | 6.0 | 5.9 | 5.8 | 5.4 |
| hsa-miR-6730-3p | 5.9 | 6.3 | 5.6 | 4.5 | 6.2 | 5.9 | 6.4 | 5.7 | 5.8 |
| hsa-miR-6797-3p | 6.9 | 6.3 | 6.8 | 4.7 | 6.5 | 6.4 | 6.5 | 5.6 | 5.8 |
| hsa-miR-0920 | 6.8 | 5.3 | 6.5 | 4.5 | 6.2 | 6.3 | 6.1 | 4.4 | 4.2 |
| hsa-miR-4443 | 5.3 | 6.5 | 5.9 | 6.4 | 7.2 | 6.6 | 6.0 | 6.3 | 5.5 |
| hsa-miR-6732-3p | 6.3 | 6.5 | 5.9 | 4.6 | 6.3 | 6.0 | 6.2 | 5.6 | 6.0 |
| hsa-miR-0365a-5p | 7.1 | 6.4 | 6.4 | 5.2 | 5.9 | 6.2 | 6.7 | 5.6 | 5.8 |
| hsa-miR-6780a-5p | 5.4 | 5.1 | 4.4 | 3.2 | 5.0 | 4.6 | 5.5 | 3.8 | 3.1 |
| hsa-miR-6510-5p | 7.1 | 7.2 | 7.0 | 4.3 | 6.6 | 7.0 | 6.1 | 5.5 | 5.0 |
| hsa-miR-6745 | 4.0 | 5.0 | 6.0 | 0.0 | 6.5 | 5.2 | 3.9 | 3.2 | 3.0 |
| hsa-miR-7114-3p | 6.5 | 6.2 | 6.1 | 4.4 | 6.5 | 6.2 | 6.5 | 5.5 | 5.6 |
| hsa-miR-7113-3p | 6.5 | 6.6 | 6.5 | 5.4 | 6.7 | 6.9 | 6.5 | 6.3 | 6.2 |
| hsa-miR-4276 | 6.1 | 6.7 | 6.9 | 7.7 | 6.5 | 7.1 | 6.4 | 7.1 | 5.2 |
| hsa-miR-6726-5p | 5.7 | 7.4 | 7.2 | 7.5 | 7.6 | 8.0 | 7.2 | 8.4 | 6.7 |
| hsa-miR-0125a-3p | 6.6 | 5.8 | 6.3 | 4.5 | 6.6 | 6.4 | 5.7 | 4.8 | 5.6 |
| hsa -miR -0486-5p | 6.0 | 6.2 | 6.0 | 4.7 | 6.6 | 6.3 | 6.5 | 5.6 | 5.9 |
| hsa-miR-4499 | 5.6 | 4.4 | 6.1 | 7.0 | 5.9 | 5.3 | 3.9 | 2.9 | 3.8 |
| hsa-miR-4697-3p | 6.0 | 6.3 | 5.6 | 4.6 | 6.2 | 6.0 | 6.3 | 5.5 | 5.5 |
| hsa-miR-6507-5p | 5.8 | 5.3 | 5.0 | 2.9 | 5.3 | 5.1 | 5.6 | 3.7 | 4.3 |
| hsa-miR-1976 | 6.1 | 6.5 | 6.1 | 4.8 | 6.2 | 6.6 | 6.5 | 5.9 | 5.9 |
| hsa-miR-3147 | 6.3 | 6.2 | 6.4 | 4.8 | 6.2 | 6.6 | 5.6 | 5.7 | 5.8 |
| hsa-miR-0519e-5p | 5.5 | 3.4 | 7.3 | 5.6 | 6.5 | 4.8 | 7.3 | 4.4 | 6.7 |
| hsa-miR-6815-5p | 4.9 | 4.9 | 6.9 | 5.4 | 7.0 | 6.9 | 4.5 | 4.3 | 4.3 |
| hsa-miR-4750-5p | 5.9 | 6.8 | 6.4 | 6.4 | 6.3 | 7.3 | 6.0 | 6.4 | 5.8 |
| hsa-miR-4687-5p | 6.6 | 6.6 | 6.6 | 5.3 | 6.6 | 6.8 | 6.7 | 6.3 | 6.2 |
| hsa -miR -6806-5p | 6.9 | 7.0 | 7.1 | 5.8 | 7.0 | 7.1 | 6.8 | 6.7 | 6.3 |
| hsa-miR-4783-3p | 6.9 | 6.7 | 7.3 | 6.5 | 6.5 | 7.1 | 6.6 | 6.3 | 6.1 |
| hsa-miR-6754-5p | 7.9 | 6.4 | 6.6 | 3.7 | 6.4 | 6.3 | 5.7 | 5.3 | 5.1 |
| hsa-miR-4419a | 6.8 | 6.2 | 6.4 | 3.6 | 7.0 | 6.8 | 5.7 | 5.0 | 5.2 |
| hsa-miR-6769a-3p | 6.3 | 6.4 | 6.1 | 4.7 | 6.4 | 6.4 | 6.5 | 5.8 | 5.7 |
| hsa -miR -6846-3p | 6.3 | 6.4 | 5.9 | 5.2 | 6.0 | 6.1 | 6.6 | 5.5 | 5.8 |
| hsa-miR-4769-3p | 6.5 | 6.5 | 6.3 | 5.3 | 6.3 | 6.4 | 6.5 | 5.5 | 5.8 |
| hsa-miR-0744-5p | 6.9 | 7.4 | 7.5 | 7.2 | 6.8 | 7.1 | 6.3 | 6.9 | 5.8 |
| hsa -miR -3184-3p | 6.4 | 6.6 | 6.4 | 4.8 | 6.8 | 6.2 | 6.8 | 6.1 | 6.0 |
| hsa-miR-6792-3p | 6.3 | 6.6 | 6.0 | 4.7 | 6.7 | 6.2 | 6.6 | 5.9 | 5.9 |
| hsa-miR-0150-3p | 5.9 | 6.1 | 6.1 | 4.7 | 6.5 | 6.1 | 6.9 | 6.5 | 6.6 |
| hsa-miR-7155-5p | 7.6 | 7.0 | 7.2 | 5.2 | 6.7 | 7.1 | 7.1 | 6.7 | 6.6 |
| hsa -miR -6858-3p | 6.6 | 6.4 | 6.3 | 4.7 | 6.4 | 6.4 | 6.9 | 5.8 | 5.5 |
| hsa-miR-4690-5p | 6.6 | 7.2 | 6.7 | 6.1 | 7.1 | 6.9 | 7.7 | 6.6 | 7.0 |
| hsa-miR-6861-3p | 6.6 | 6.4 | 6.2 | 3.8 | 6.2 | 6.4 | 6.6 | 5.5 | 5.4 |
| hsa-miR-6069 | 7.1 | 6.8 | 7.0 | 6.8 | 6.4 | 6.5 | 6.7 | 6.0 | 5.9 |
| hsa-miR-0766-3p | 5.9 | 6.3 | 5.8 | 4.8 | 6.4 | 6.4 | 6.2 | 6.0 | 5.8 |
| hsa-miR-4478 | 6.4 | 6.9 | 6.0 | 4.4 | 7.1 | 7.5 | 5.7 | 6.1 | 4.7 |
| hsa-miR-6827-5p | 6.2 | 6.1 | 6.3 | 3.9 | 6.5 | 6.0 | 5.9 | 5.3 | 5.3 |
| hsa-miR-4284 | 6.2 | 6.0 | 6.2 | 4.1 | 6.6 | 6.3 | 6.6 | 5.5 | 6.0 |
| hsa-miR-7108-3p | 6.7 | 6.4 | 6.8 | 5.3 | 6.6 | 6.5 | 6.8 | 5.9 | 5.6 |
| hsa-miR-6889-3p | 6.7 | 6.5 | 6.3 | 4.4 | 6.4 | 6.4 | 6.7 | 5.7 | 6.0 |
| hsa-miR-6860 | 6.6 | 6.9 | 6.5 | 5.4 | 6.6 | 6.7 | 6.7 | 6.9 | 6.4 |
| hsa -miR -3928-3p | 6.6 | 6.5 | 7.4 | 5.6 | 8.1 | 7.6 | 5.5 | 6.1 | 5.6 |
| hsa-miR-6760-5p | 5.3 | 5.7 | 6.9 | 3.6 | 7.5 | 6.4 | 4.7 | 4.2 | 4.6 |
| hsa-miR-4667-3p | 6.7 | 6.6 | 6.5 | 4.6 | 6.6 | 6.7 | 6.7 | 6.0 | 6.3 |
| hsa-miR-1247-3p | 6.6 | 6.3 | 6.9 | 6.1 | 6.8 | 6.9 | 6.8 | 6.1 | 5.9 |
| hsa-miR-6845-3p | 6.8 | 6.8 | 6.7 | 4.9 | 6.5 | 6.6 | 6.7 | 6.2 | 6.1 |
| hsa-miR-4486 | 7.0 | 8.1 | 8.1 | 6.7 | 7.9 | 8.4 | 7.2 | 7.9 | 6.7 |
| hsa-miR-5572 | 6.6 | 6.4 | 7.0 | 5.1 | 7.3 | 6.9 | 6.1 | 6.7 | 5.7 |
| hsa-miR-0484 | 6.7 | 6.5 | 6.4 | 4.9 | 6.7 | 6.5 | 6.7 | 5.7 | 6.2 |
| hsa-miR-6760-3p | 6.6 | 6.3 | 6.5 | 4.1 | 6.7 | 6.4 | 6.7 | 5.5 | 5.8 |
| hsa-miR-6885-3p | 6.6 | 6.3 | 6.3 | 4.6 | 6.4 | 6.6 | 6.4 | 5.6 | 5.8 |
| hsa -miR -6892-3p | 6.9 | 6.5 | 6.8 | 7.7 | 6.3 | 6.3 | 6.8 | 5.6 | 5.9 |
| hsa-miR-2355-5p | 6.3 | 6.0 | 6.1 | 4.4 | 6.1 | 6.0 | 6.0 | 5.3 | 5.3 |
| hsa -miR -3158-3p | 4.0 | 4.6 | 3.6 | 4.4 | 6.1 | 6.0 | 3.8 | 3.4 | 2.7 |
| hsa-miR-6759-3p | 6.4 | 6.5 | 6.2 | 4.8 | 6.8 | 6.7 | 6.6 | 6.0 | 6.2 |
| hsa-miR-0210-5p | 6.7 | 6.7 | 6.6 | 4.8 | 6.8 | 6.9 | 6.9 | 6.3 | 6.2 |
| hsa-miR-6778-5p | 6.4 | 6.8 | 6.3 | 5.5 | 6.7 | 7.4 | 6.4 | 6.6 | 5.9 |
| hsa-miR-2110 | 7.2 | 6.2 | 7.2 | 5.7 | 7.0 | 6.9 | 6.8 | 6.3 | 5.7 |
| hsa-miR-3150a-5p | 2.2 | 3.7 | 0.0 | 5.7 | 7.0 | 6.9 | 3.6 | 2.3 | 3.6 |
| hsa-miR-6731-5p | 7.3 | 5.7 | 7.9 | 4.1 | 8.0 | 7.5 | 5.8 | 5.1 | 5.1 |
| hsa-miR-6756-3p | 6.8 | 6.5 | 6.7 | 7.3 | 6.5 | 6.5 | 6.8 | 5.9 | 6.2 |
| hsa-miR-6786-3p | 6.5 | 6.8 | 6.4 | 5.4 | 6.7 | 6.6 | 7.0 | 6.2 | 6.2 |
| hsa-miR-6831-5p | 7.0 | 6.5 | 7.3 | 5.2 | 7.7 | 7.3 | 5.8 | 5.3 | 5.2 |
| hsa -miR -0652-5p | 8.3 | 7.6 | 7.5 | 5.4 | 8.4 | 8.8 | 6.4 | 5.9 | 5.5 |
| hsa -miR -4419b | 8.7 | 7.1 | 7.5 | 4.9 | 6.7 | 7.2 | 5.6 | 6.1 | 5.0 |
| hsa-miR-4685-5p | 7.5 | 7.0 | 6.7 | 4.9 | 6.4 | 7.1 | 6.6 | 6.5 | 6.5 |
| hsa-miR-0939-5p | 6.9 | 7.0 | 8.2 | 7.3 | 8.4 | 8.9 | 6.2 | 8.0 | 4.5 |
| hsa-miR-6819-3p | 6.7 | 6.1 | 6.4 | 4.2 | 6.3 | 6.1 | 6.8 | 5.4 | 5.8 |
| hsa -miR -6826-3p | 6.7 | 6.7 | 6.4 | 4.7 | 6.6 | 6.5 | 6.7 | 6.0 | 6.0 |
| hsa-miR-0030c-2-3p | 5.3 | 5.6 | 6.2 | 2.8 | 6.5 | 5.6 | 4.2 | 4.1 | 5.1 |
| hsa-miR-6132 | 7.6 | 8.1 | 8.7 | 6.6 | 7.1 | 7.3 | 6.2 | 6.9 | 5.0 |
| hsa-miR-6769b-5p | 7.3 | 7.3 | 6.7 | 4.7 | 6.7 | 7.2 | 6.5 | 6.6 | 5.7 |
| hsa -miR -6802-3p | 6.5 | 6.5 | 6.1 | 4.2 | 6.6 | 6.4 | 6.9 | 6.0 | 6.0 |
| hsa-miR-3194-5p | 7.0 | 6.7 | 6.6 | 5.4 | 6.6 | 6.6 | 6.8 | 5.9 | 6.2 |
| hsa-miR-0718 | 6.2 | 7.1 | 7.1 | 6.4 | 7.5 | 7.4 | 7.7 | 7.7 | 7.3 |
| hsa-miR-6861-5p | 7.1 | 7.2 | 7.3 | 6.3 | 7.4 | 7.8 | 7.3 | 7.5 | 6.7 |
| hsa-miR-4313 | 6.6 | 6.5 | 6.4 | 5.5 | 6.6 | 6.0 | 7.0 | 6.1 | 6.0 |
| hsa-miR-6777-5p | 5.9 | 6.7 | 7.7 | 5.2 | 8.4 | 7.4 | 5.8 | 6.7 | 5.6 |
| hsa-miR-6813-5p | 6.9 | 7.6 | 7.7 | 5.6 | 6.4 | 6.6 | 6.7 | 6.8 | 5.9 |
| hsa-miR-4707-3p | 6.6 | 6.5 | 7.0 | 5.4 | 6.8 | 6.8 | 6.9 | 6.5 | 6.0 |
| hsa-miR-7110-5p | 7.0 | 7.2 | 7.9 | 6.4 | 7.7 | 7.9 | 6.5 | 8.3 | 5.8 |
| hsa-miR-4258 | 7.1 | 7.0 | 7.9 | 7.1 | 7.7 | 7.8 | 7.0 | 7.9 | 6.2 |
| hsa-miR-4526 | 6.8 | 6.6 | 6.9 | 5.5 | 6.4 | 6.2 | 6.3 | 6.8 | 6.7 |
| hsa-miR-4758-3p | 6.6 | 6.9 | 6.3 | 4.8 | 6.6 | 6.7 | 6.8 | 6.2 | 6.0 |
| hsa-miR-6889-5p | 6.7 | 6.7 | 7.7 | 5.5 | 8.2 | 7.3 | 6.3 | 6.8 | 5.8 |
| hsa-miR-6752-3p | 6.9 | 6.7 | 6.7 | 4.8 | 6.9 | 6.7 | 7.1 | 6.0 | 6.3 |
| hsa-miR-0711 | 7.4 | 6.9 | 8.6 | 6.0 | 8.2 | 7.5 | 6.3 | 6.4 | 5.7 |
| hsa-miR-6879-5p | 6.9 | 7.0 | 7.4 | 6.6 | 7.6 | 7.9 | 6.5 | 6.3 | 5.4 |
| hsa-miR-8087 | 5.1 | 4.1 | 4.4 | 0.0 | 4.1 | 4.5 | 0.0 | 0.0 | 0.0 |
| hsa-miR-0933 | 8.2 | 6.3 | 6.7 | 3.6 | 5.9 | 5.1 | 5.8 | 3.7 | 4.6 |
| hsa-miR-4507 | 6.6 | 8.0 | 7.8 | 6.9 | 7.9 | 8.1 | 6.8 | 8.6 | 6.7 |
| hsa-miR-4756-5p | 8.2 | 6.8 | 7.3 | 4.9 | 7.3 | 7.2 | 6.4 | 5.7 | 5.7 |
| hsa-miR-1249-5p | 7.8 | 7.5 | 7.5 | 5.9 | 6.7 | 7.2 | 7.0 | 5.9 | 5.9 |
| hsa-miR-0197-5p | 8.2 | 7.8 | 7.7 | 6.1 | 7.1 | 8.0 | 6.6 | 7.4 | 5.9 |
| hsa-miR-4323 | 7.0 | 6.9 | 6.7 | 5.3 | 7.0 | 6.9 | 7.2 | 6.3 | 6.2 |
| hsa-miR-0092b-5p | 6.9 | 8.1 | 8.5 | 7.3 | 8.7 | 8.8 | 6.7 | 8.3 | 6.5 |
| hsa-miR-6887-3p | 7.0 | 6.8 | 6.9 | 4.9 | 7.0 | 7.0 | 7.2 | 6.1 | 6.2 |
| hsa-miR-6893-5p | 6.3 | 6.8 | 8.2 | 7.3 | 8.8 | 10.0 | 6.4 | 6.9 | 5.9 |
| hsa-miR-6813-3p | 7.3 | 6.8 | 7.2 | 4.7 | 7.1 | 6.8 | 7.5 | 6.2 | 6.4 |
| hsa-miR-6891-3p | 7.0 | 6.6 | 6.8 | 5.2 | 6.9 | 6.5 | 7.3 | 5.8 | 6.1 |
| hsa-miR-6731-3p | 6.6 | 6.4 | 6.8 | 4.9 | 6.8 | 6.6 | 7.2 | 5.8 | 6.1 |
| hsa-miR-0345-3p | 7.8 | 6.3 | 10.0 | 4.8 | 10.1 | 8.0 | 6.9 | 5.5 | 5.7 |
| hsa-miR-4640-3p | 7.2 | 6.8 | 6.9 | 5.4 | 7.0 | 6.7 | 7.2 | 5.8 | 6.5 |
| hsa-miR-4716-3p | 6.9 | 5.2 | 7.8 | 4.9 | 8.4 | 6.9 | 5.7 | 4.0 | 4.6 |
| hsa-miR-0371b-3p | 7.0 | 6.9 | 6.8 | 5.1 | 6.9 | 7.0 | 7.0 | 6.3 | 6.6 |
| hsa-miR-6812-5p | 8.2 | 7.2 | 7.0 | 4.8 | 7.1 | 7.8 | 6.7 | 6.2 | 6.5 |
| hsa-miR-6784-3p | 6.9 | 6.6 | 6.8 | 4.9 | 7.1 | 6.7 | 7.3 | 6.1 | 6.4 |
| hsa-miR-6716-5p | 8.1 | 7.6 | 7.8 | 5.5 | 7.2 | 8.6 | 6.9 | 6.8 | 6.4 |
| hsa-miR-4513 | 7.9 | 7.5 | 8.4 | 5.3 | 7.1 | 7.9 | 6.5 | 6.9 | 6.1 |
| hsa -miR -6848-3p | 7.1 | 7.1 | 6.9 | 5.4 | 7.1 | 7.1 | 7.3 | 6.3 | 6.7 |
| hsa-miR-1238-5p | 7.2 | 7.1 | 7.3 | 5.2 | 7.5 | 7.4 | 6.7 | 6.0 | 6.0 |
| hsa-miR-4530 | 7.0 | 8.2 | 8.3 | 6.9 | 8.2 | 8.9 | 6.9 | 8.2 | 6.5 |
| hsa-miR-4655-5p | 7.1 | 7.6 | 7.7 | 7.2 | 7.3 | 7.8 | 6.8 | 7.0 | 6.0 |
| hsa-miR-6785-3p | 7.2 | 6.8 | 7.1 | 5.4 | 7.1 | 6.9 | 7.5 | 6.2 | 6.4 |
| hsa-miR-4728-3p | 7.3 | 7.1 | 7.0 | 5.4 | 6.8 | 7.0 | 7.4 | 6.3 | 6.3 |
| hsa-miR-5090 | 7.2 | 7.9 | 8.6 | 7.3 | 7.8 | 7.6 | 7.5 | 8.4 | 7.9 |
| hsa -miR -6824-5p | 8.0 | 7.5 | 7.1 | 5.6 | 7.2 | 7.9 | 6.9 | 6.7 | 6.4 |
| hsa-miR-4667-5p | 7.8 | 7.2 | 7.7 | 5.0 | 7.3 | 7.0 | 6.7 | 6.1 | 5.3 |
| hsa-miR-0874-5p | 7.4 | 7.5 | 7.1 | 5.7 | 7.2 | 7.1 | 7.5 | 6.7 | 6.5 |
| hsa-miR-4749-5p | 7.7 | 7.6 | 7.7 | 6.3 | 7.8 | 8.2 | 7.3 | 7.9 | 6.7 |
| hsa-miR-6086 | 7.5 | 6.8 | 7.9 | 4.7 | 8.6 | 8.0 | 6.7 | 6.2 | 6.4 |
| hsa-miR-4257 | 7.1 | 6.8 | 6.4 | 5.9 | 6.3 | 7.1 | 7.8 | 6.9 | 6.4 |
| hsa-miR-1224-3p | 7.6 | 7.1 | 7.3 | 5.3 | 7.1 | 6.9 | 7.4 | 6.3 | 6.5 |
| hsa-miR-3610 | 7.6 | 6.4 | 9.2 | 5.4 | 10.2 | 9.1 | 6.0 | 5.7 | 5.5 |
| hsa-miR-6880-5p | 7.8 | 8.1 | 7.6 | 5.2 | 7.8 | 8.8 | 6.9 | 7.6 | 6.3 |
| hsa-miR-0663b | 7.5 | 8.0 | 8.0 | 6.5 | 8.1 | 8.0 | 7.9 | 7.7 | 7.4 |
| hsa-miR-1237-3p | 7.4 | 7.0 | 7.4 | 6.0 | 7.1 | 7.1 | 7.5 | 6.5 | 6.4 |
| hsa-miR-1225-3p | 7.3 | 7.2 | 7.3 | 6.2 | 7.4 | 7.4 | 7.5 | 6.8 | 6.5 |
| hsa-miR-6763-5p | 7.0 | 7.7 | 7.9 | 6.5 | 8.8 | 10.5 | 7.2 | 7.7 | 6.5 |
| hsa-miR-4707-5p | 7.5 | 8.4 | 8.6 | 7.6 | 8.0 | 8.8 | 7.4 | 8.4 | 6.8 |
| hsa-miR-3195 | 7.0 | 8.1 | 8.2 | 7.7 | 8.4 | 8.6 | 8.1 | 8.9 | 7.9 |
| hsa-miR-1231 | 6.9 | 8.2 | 8.1 | 8.2 | 7.4 | 8.1 | 7.3 | 8.2 | 6.1 |
| hsa-miR-4695-5p | 6.9 | 8.7 | 8.5 | 7.7 | 8.6 | 9.1 | 7.0 | 8.9 | 7.1 |
| hsa-miR-1225-5p | 7.6 | 8.5 | 8.2 | 7.1 | 8.3 | 8.7 | 6.8 | 8.9 | 6.7 |
| hsa-miR-6891-5p | 8.8 | 7.9 | 7.5 | 4.8 | 7.2 | 7.8 | 6.7 | 6.8 | 6.3 |
| hsa-miR-3188 | 8.8 | 8.0 | 9.6 | 5.3 | 10.1 | 9.5 | 6.9 | 6.8 | 6.3 |
| hsa-miR-1202 | 8.9 | 7.8 | 7.6 | 5.6 | 7.6 | 8.5 | 7.4 | 6.7 | 7.0 |
| hsa-miR-6807-5p | 9.2 | 7.9 | 7.5 | 5.1 | 6.7 | 7.3 | 6.8 | 5.9 | 5.6 |
| hsa-miR-6738-5p | 7.7 | 8.0 | 7.9 | 6.6 | 7.9 | 7.8 | 8.6 | 7.6 | 7.8 |
| hsa-miR-1238-3p | 7.2 | 7.3 | 7.2 | 5.7 | 7.7 | 7.3 | 7.7 | 6.6 | 7.1 |
| hsa-miR-6840-3p | 7.7 | 8.2 | 8.5 | 6.9 | 9.4 | 10.0 | 7.5 | 8.6 | 7.2 |
| hsa-miR-0583 | 6.4 | 4.2 | 8.5 | 0.0 | 9.6 | 7.3 | 4.0 | 0.0 | 4.4 |
| hsa-miR-4640-5p | 7.2 | 8.6 | 8.5 | 8.0 | 8.2 | 9.0 | 7.1 | 9.1 | 6.9 |
| hsa-miR-0518c-5p | 6.5 | 6.3 | 6.2 | 5.0 | 6.8 | 6.6 | 6.0 | 5.7 | 6.4 |
| hsa-miR-7108-5p | 7.7 | 9.6 | 9.4 | 8.4 | 9.3 | 9.9 | 7.6 | 9.7 | 7.1 |
| hsa-miR-4725-3p | 7.5 | 8.3 | 7.8 | 6.9 | 8.4 | 8.6 | 7.6 | 8.1 | 6.1 |
| hsa-miR-4497 | 7.5 | 9.2 | 8.9 | 8.1 | 9.1 | 10.6 | 7.9 | 9.2 | 7.1 |
| hsa-miR-0361-3p | 7.1 | 7.2 | 7.2 | 5.7 | 7.6 | 7.3 | 7.8 | 6.5 | 6.9 |
| hsa-miR-5001-5p | 7.2 | 8.3 | 7.9 | 6.7 | 7.5 | 8.3 | 7.4 | 7.9 | 6.4 |
| hsa-miR-6880-3p | 7.5 | 7.3 | 7.5 | 5.7 | 7.7 | 7.5 | 7.7 | 6.9 | 7.1 |
| hsa-miR-0659-3p | 8.7 | 7.8 | 7.4 | 4.9 | 7.3 | 8.3 | 7.8 | 6.4 | 7.3 |
| hsa -miR -6848-5p | 8.4 | 8.8 | 8.8 | 7.1 | 8.6 | 9.0 | 8.0 | 9.0 | 7.5 |
| hsa-miR-6124 | 8.1 | 8.0 | 7.6 | 6.0 | 7.9 | 8.1 | 6.9 | 6.5 | 6.4 |
| hsa-miR-4750-3p | 7.4 | 7.1 | 7.3 | 8.3 | 7.4 | 7.0 | 8.0 | 6.4 | 6.9 |
| hsa-miR-4665-3p | 7.4 | 7.3 | 7.4 | 6.2 | 7.5 | 7.2 | 7.9 | 7.0 | 7.2 |
| hsa-miR-0373-5p | 8.9 | 7.5 | 8.0 | 5.7 | 7.5 | 8.1 | 7.6 | 6.5 | 7.5 |
| hsa-miR-0128-1-5p | 8.0 | 8.1 | 8.6 | 7.3 | 8.2 | 8.4 | 7.6 | 8.2 | 7.1 |
| hsa-miR-4433b-5p | 7.5 | 7.2 | 7.4 | 5.7 | 7.5 | 7.2 | 8.0 | 6.6 | 6.7 |
| hsa-miR-4433a-3p | 7.8 | 8.4 | 8.3 | 6.8 | 8.3 | 8.7 | 7.4 | 8.3 | 7.5 |
| hsa -miR -6808-5p | 8.5 | 8.3 | 7.8 | 6.3 | 7.8 | 8.5 | 8.5 | 7.7 | 7.7 |
| hsa-miR-6835-5p | 8.9 | 9.4 | 7.1 | 3.7 | 6.7 | 8.1 | 8.0 | 7.6 | 5.8 |
| hsa-miR-5196-5p | 7.2 | 7.0 | 7.4 | 5.6 | 7.9 | 7.9 | 7.4 | 6.4 | 6.7 |
| hsa-miR-6075 | 6.9 | 8.6 | 8.7 | 7.6 | 8.5 | 8.5 | 7.3 | 8.5 | 6.7 |
| hsa-miR-6737-5p | 7.4 | 8.6 | 8.3 | 7.2 | 8.3 | 8.4 | 7.7 | 8.8 | 7.1 |
| hsa-miR-6780b-5p | 7.6 | 7.2 | 7.7 | 5.6 | 8.5 | 8.6 | 7.6 | 6.2 | 6.4 |
| hsa-miR-6732-5p | 7.7 | 9.3 | 9.4 | 7.9 | 9.0 | 9.2 | 7.7 | 8.9 | 7.4 |
| hsa-miR-6865-5p | 7.9 | 8.0 | 7.5 | 6.2 | 7.5 | 7.4 | 8.4 | 7.1 | 7.7 |
| hsa-miR-4665-5p | 7.9 | 8.5 | 9.0 | 7.3 | 8.3 | 8.4 | 8.2 | 8.3 | 8.4 |
| hsa-miR-2116-3p | 7.7 | 7.3 | 7.5 | 5.7 | 7.3 | 7.1 | 7.9 | 6.4 | 6.9 |
| hsa -miR -3152-5p | 2.9 | 3.5 | 2.4 | 5.7 | 7.3 | 7.1 | 4.3 | 2.2 | 2.6 |
| hsa-miR-8059 | 7.3 | 7.3 | 7.5 | 7.2 | 7.0 | 7.1 | 9.3 | 7.8 | 7.6 |
| hsa-miR-6851-5p | 8.9 | 8.4 | 8.2 | 6.4 | 7.5 | 8.3 | 8.0 | 7.7 | 7.1 |
| hsa-miR-4788 | 9.9 | 7.8 | 8.6 | 5.0 | 7.9 | 7.5 | 7.2 | 6.1 | 6.3 |
| hsa-miR-4447 | 8.4 | 8.5 | 8.0 | 6.9 | 7.9 | 8.5 | 7.9 | 7.8 | 7.1 |
| hsa-miR-6744-5p | 9.8 | 8.6 | 8.7 | 4.9 | 7.7 | 7.9 | 6.7 | 6.4 | 5.8 |
| hsa-miR-0187-5p | 7.4 | 9.1 | 9.6 | 7.9 | 9.0 | 9.0 | 7.3 | 9.8 | 7.2 |
| hsa-miR-4467 | 7.7 | 10.2 | 9.8 | 9.1 | 9.8 | 10.6 | 7.7 | 10.0 | 7.5 |
| hsa-miR-0671-5p | 10.5 | 9.0 | 8.9 | 5.5 | 7.7 | 8.6 | 7.7 | 6.6 | 6.4 |
| hsa-miR-6799-5p | 9.3 | 9.1 | 9.0 | 7.3 | 8.5 | 9.3 | 8.0 | 8.4 | 7.2 |
| hsa-miR-4298 | 8.9 | 8.3 | 8.1 | 5.9 | 8.2 | 8.3 | 7.7 | 6.8 | 7.2 |
| hsa-miR-6797-5p | 8.6 | 8.2 | 7.9 | 7.3 | 7.8 | 8.2 | 7.9 | 7.0 | 7.3 |
| hsa-miR-6829-5p | 7.5 | 7.6 | 7.7 | 6.1 | 7.3 | 7.4 | 7.6 | 7.2 | 6.8 |
| hsa-miR-1275 | 9.3 | 8.6 | 10.1 | 6.0 | 10.7 | 10.0 | 7.6 | 7.1 | 7.7 |
| hsa-miR-3614-5p | 7.9 | 7.6 | 7.5 | 5.6 | 8.0 | 7.8 | 8.2 | 6.9 | 7.4 |
| hsa-miR-4688 | 7.7 | 8.7 | 8.5 | 7.3 | 8.2 | 8.6 | 8.1 | 8.6 | 7.0 |
| hsa-miR-1207-5p | 8.6 | 8.1 | 8.8 | 6.7 | 9.6 | 9.5 | 7.7 | 7.4 | 7.0 |
| hsa-miR-6794-5p | 8.4 | 8.6 | 8.6 | 6.9 | 8.2 | 8.8 | 8.0 | 8.4 | 7.4 |
| hsa-miR-6721-5p | 8.3 | 9.0 | 9.2 | 7.5 | 8.6 | 8.8 | 7.8 | 9.5 | 7.7 |
| hsa-miR-0665 | 7.7 | 8.8 | 8.1 | 7.3 | 8.6 | 8.9 | 7.9 | 8.4 | 8.0 |
| hsa-miR-7845-5p | 8.8 | 8.7 | 8.3 | 6.3 | 8.0 | 8.4 | 8.0 | 7.0 | 7.2 |
| hsa-miR-7150 | 8.6 | 8.3 | 8.9 | 6.1 | 8.9 | 8.8 | 7.7 | 7.2 | 7.5 |
| hsa-miR-4433b-3p | 8.2 | 9.0 | 8.8 | 7.3 | 8.7 | 9.3 | 7.6 | 9.1 | 7.4 |
| hsa-miR-7114-5p | 8.9 | 8.5 | 9.2 | 7.3 | 9.8 | 10.0 | 8.6 | 8.1 | 7.8 |
| hsa-miR-4716-5p | 7.9 | 7.5 | 7.9 | 6.0 | 8.0 | 7.6 | 8.3 | 6.7 | 7.2 |
| hsa-miR-6795-3p | 8.0 | 7.6 | 7.8 | 5.9 | 7.9 | 7.6 | 8.3 | 6.9 | 7.2 |
| hsa-miR-4433a-5p | 8.1 | 7.7 | 8.2 | 6.2 | 7.9 | 7.8 | 8.5 | 7.0 | 7.3 |
| hsa-miR-6777-3p | 7.8 | 7.7 | 7.8 | 6.1 | 8.0 | 7.8 | 8.4 | 7.1 | 7.3 |
| hsa-miR-0557 | 8.9 | 8.9 | 8.9 | 7.3 | 8.6 | 9.1 | 8.6 | 9.8 | 8.3 |
| hsa-miR-1185-2-3p | 8.8 | 7.8 | 7.9 | 4.7 | 8.0 | 8.9 | 7.1 | 6.4 | 6.6 |
| hsa-miR-4731-3p | 7.8 | 7.7 | 7.7 | 6.1 | 8.1 | 7.8 | 8.3 | 7.0 | 7.6 |
| hsa-miR-4745-5p | 8.0 | 9.6 | 9.6 | 8.5 | 9.6 | 10.6 | 8.3 | 9.6 | 8.1 |
| hsa-miR-6763-3p | 8.1 | 7.6 | 8.0 | 6.1 | 7.8 | 7.6 | 8.6 | 7.1 | 7.4 |
| hsa-miR-3937 | 8.5 | 8.9 | 9.4 | 8.0 | 8.5 | 9.2 | 8.2 | 9.2 | 7.5 |
| hsa-miR-0625-3p | 8.1 | 7.9 | 8.1 | 6.3 | 8.3 | 7.9 | 8.6 | 7.3 | 7.7 |
| hsa-miR-6800-3p | 8.2 | 7.9 | 7.9 | 6.1 | 8.1 | 7.9 | 8.6 | 7.4 | 7.5 |
| hsa-miR-0940 | 8.4 | 7.8 | 8.0 | 8.0 | 7.8 | 7.9 | 8.4 | 7.2 | 7.3 |
| hsa-miR-6758-5p | 7.8 | 4.6 | 9.7 | 4.1 | 10.1 | 8.6 | 6.1 | 4.3 | 5.2 |
| hsa-miR-4274 | 8.4 | 7.7 | 8.2 | 6.2 | 8.1 | 7.9 | 8.4 | 7.1 | 7.5 |
| hsa-miR-4749-3p | 8.3 | 7.9 | 8.2 | 7.8 | 7.9 | 8.0 | 8.6 | 7.3 | 7.3 |
| hsa-miR-3675-3p | 8.2 | 7.8 | 7.9 | 6.2 | 8.3 | 8.1 | 8.5 | 7.3 | 7.5 |
| hsa-miR-0760 | 8.2 | 8.7 | 8.7 | 7.5 | 8.4 | 9.0 | 8.7 | 9.0 | 7.9 |
| hsa-miR-3620-5p | 8.0 | 9.4 | 9.2 | 8.1 | 9.2 | 9.5 | 8.2 | 9.8 | 8.2 |
| hsa-miR-4484 | 8.9 | 9.1 | 10.2 | 7.3 | 10.4 | 10.7 | 7.5 | 8.5 | 7.1 |
| hsa-miR-6774-5p | 9.0 | 8.3 | 8.8 | 6.5 | 8.3 | 9.4 | 7.4 | 7.4 | 6.9 |
| hsa-miR-0675-5p | 9.7 | 8.8 | 8.7 | 6.8 | 8.3 | 9.5 | 8.0 | 7.9 | 7.8 |
| hsa-miR-1229-5p | 10.1 | 8.6 | 9.6 | 6.5 | 9.4 | 10.0 | 8.0 | 7.7 | 8.3 |
| hsa-miR-4459 | 7.6 | 8.3 | 8.1 | 7.3 | 8.7 | 9.3 | 7.7 | 7.9 | 7.4 |
| hsa-miR-6787-5p | 9.1 | 9.0 | 9.1 | 7.4 | 8.8 | 9.5 | 8.2 | 8.9 | 7.7 |
| hsa-miR-4271 | 10.0 | 9.4 | 8.9 | 5.7 | 7.9 | 9.0 | 7.1 | 7.5 | 6.5 |
| hsa-miR-4417 | 8.7 | 9.4 | 9.2 | 8.1 | 9.3 | 9.8 | 8.5 | 9.6 | 8.4 |
| hsa-miR-4505 | 8.3 | 9.2 | 9.6 | 7.8 | 8.6 | 8.7 | 7.3 | 8.4 | 6.7 |
| hsa-miR-6875-5p | 9.0 | 8.2 | 10.5 | 8.1 | 9.7 | 10.1 | 5.5 | 8.0 | 5.5 |
| hsa-miR-4758-5p | 8.5 | 9.2 | 9.0 | 7.8 | 8.5 | 8.7 | 8.5 | 8.9 | 8.3 |
| hsa-miR-6741-5p | 9.2 | 8.3 | 8.7 | 6.3 | 8.3 | 8.5 | 8.0 | 7.3 | 7.0 |
| hsa-miR-0514b-5p | 10.1 | 8.2 | 9.4 | 6.2 | 9.2 | 8.5 | 8.7 | 7.1 | 8.0 |
| hsa-miR-4726-5p | 9.6 | 9.0 | 8.8 | 6.2 | 9.0 | 9.4 | 8.6 | 7.7 | 8.0 |
| hsa-miR-6791-5p | 8.8 | 10.3 | 10.2 | 8.9 | 10.0 | 10.4 | 8.6 | 10.5 | 8.2 |
| hsa-miR-6789-5p | 8.6 | 10.1 | 9.9 | 9.3 | 9.5 | 10.2 | 8.6 | 10.3 | 8.4 |
| hsa-miR-6762-5p | 8.4 | 8.8 | 8.9 | 8.0 | 9.0 | 8.5 | 9.4 | 9.3 | 9.0 |
| hsa-miR-6859-3p | 8.1 | 7.8 | 8.0 | 6.3 | 8.4 | 8.0 | 8.8 | 7.4 | 7.8 |
| hsa-miR-7109-5p | 9.7 | 9.0 | 8.9 | 6.9 | 8.7 | 10.0 | 8.1 | 8.2 | 7.8 |
| hsa-miR-0937-5p | 8.5 | 9.4 | 8.7 | 7.5 | 9.0 | 8.9 | 9.2 | 10.1 | 9.7 |
| hsa-miR-4722-5p | 8.4 | 8.4 | 8.2 | 7.4 | 8.6 | 8.7 | 8.5 | 7.6 | 8.4 |
| hsa -miR -6826-5p | 9.3 | 9.9 | 7.9 | 5.8 | 7.9 | 8.7 | 9.4 | 8.5 | 7.8 |
| hsa-miR-1228-3p | 8.5 | 8.3 | 8.5 | 6.9 | 8.7 | 8.5 | 8.9 | 7.9 | 8.1 |
| hsa-miR-6798-3p | 9.1 | 8.5 | 9.0 | 7.0 | 9.0 | 8.5 | 9.4 | 8.0 | 8.2 |
| hsa-miR-1268b | 8.7 | 10.3 | 10.7 | 9.3 | 10.3 | 10.8 | 8.3 | 10.5 | 8.1 |
| hsa-miR-1909-3p | 8.8 | 9.2 | 9.5 | 8.5 | 9.4 | 9.2 | 9.1 | 9.6 | 9.3 |
| hsa -miR -3136-5p | 0.0 | 0.0 | 0.0 | 8.5 | 9.4 | 9.2 | 0.0 | 0.0 | 0.0 |
| hsa-miR-7847-3p | 10.3 | 9.5 | 9.1 | 6.8 | 9.1 | 9.3 | 9.0 | 8.0 | 8.2 |
| hsa-miR-7107-5p | 7.8 | 8.1 | 8.3 | 5.9 | 8.6 | 8.2 | 6.9 | 7.2 | 6.7 |
| hsa-miR-6729-3p | 8.6 | 8.2 | 8.4 | 6.8 | 8.8 | 8.3 | 9.0 | 7.7 | 8.0 |
| hsa-miR-3648 | 8.7 | 9.6 | 9.8 | 8.7 | 9.9 | 10.7 | 8.7 | 9.1 | 8.7 |
| hsa-miR-6746-5p | 8.6 | 7.8 | 7.7 | 5.7 | 8.1 | 8.9 | 6.4 | 6.6 | 6.2 |
| hsa-miR-6821-5p | 9.4 | 9.4 | 9.6 | 8.0 | 9.0 | 9.9 | 8.7 | 9.4 | 8.1 |
| hsa-miR-6768-5p | 8.9 | 9.2 | 9.4 | 8.3 | 9.4 | 9.5 | 9.1 | 9.1 | 8.9 |
| hsa-miR-1185-1-3p | 9.8 | 8.8 | 8.7 | 5.4 | 8.6 | 9.3 | 7.9 | 6.8 | 7.4 |
| hsa-miR-6798-5p | 8.9 | 10.7 | 10.9 | 9.5 | 10.3 | 10.8 | 8.8 | 10.7 | 8.7 |
| hsa-miR-6743-5p | 8.8 | 9.7 | 9.7 | 8.4 | 9.8 | 10.0 | 8.9 | 9.4 | 8.4 |
| hsa-miR-7111-5p | 9.0 | 8.8 | 9.1 | 7.3 | 8.7 | 9.0 | 7.6 | 7.8 | 6.8 |
| hsa-miR-6769a-5p | 9.8 | 8.5 | 8.9 | 5.8 | 9.0 | 9.2 | 7.6 | 6.9 | 7.3 |
| hsa-miR-6845-5p | 9.0 | 9.7 | 10.2 | 8.9 | 9.8 | 10.1 | 9.4 | 10.2 | 8.9 |
| hsa -miR -3184-5p | 10.6 | 9.9 | 9.9 | 6.9 | 8.5 | 9.9 | 7.0 | 7.8 | 6.2 |
| hsa-miR-4656 | 10.3 | 9.2 | 10.0 | 7.0 | 9.2 | 9.2 | 8.1 | 8.3 | 7.5 |
| hsa -miR -4632-5p | 9.3 | 9.7 | 9.1 | 8.5 | 9.0 | 9.9 | 8.5 | 9.0 | 7.9 |
| hsa-miR-0642b-3p | 10.6 | 9.8 | 9.3 | 6.9 | 8.8 | 9.5 | 9.0 | 8.3 | 8.4 |
| hsa-miR-6724-5p | 8.4 | 10.4 | 10.0 | 9.5 | 10.1 | 10.7 | 9.1 | 10.5 | 8.6 |
| hsa-miR-4651 | 8.9 | 10.5 | 10.7 | 9.4 | 10.2 | 10.3 | 9.3 | 10.6 | 9.1 |
| hsa-miR-6805-3p | 9.1 | 8.7 | 9.3 | 7.7 | 9.3 | 9.1 | 9.4 | 8.5 | 8.3 |
| hsa-miR-4763-3p | 9.4 | 9.8 | 10.6 | 8.4 | 9.5 | 10.3 | 8.6 | 9.7 | 8.2 |
| hsa-miR-3619-3p | 8.8 | 8.1 | 7.7 | 5.8 | 7.7 | 7.9 | 8.2 | 7.2 | 7.7 |
| hsa-miR-6126 | 9.2 | 10.7 | 9.7 | 7.8 | 9.5 | 10.9 | 8.9 | 10.4 | 8.2 |
| hsa-miR-4706 | 9.2 | 8.8 | 8.8 | 7.3 | 8.5 | 8.6 | 8.8 | 8.0 | 8.2 |
| hsa-miR-4634 | 8.8 | 10.3 | 10.0 | 9.3 | 10.1 | 10.4 | 9.4 | 10.4 | 8.8 |
| hsa-miR-6722-3p | 9.1 | 9.7 | 9.6 | 8.6 | 9.8 | 9.9 | 9.6 | 10.3 | 9.3 |
| hsa-miR-6796-3p | 9.0 | 8.5 | 9.0 | 8.0 | 9.0 | 8.6 | 9.7 | 8.2 | 8.4 |
| hsa-miR-6165 | 8.6 | 7.8 | 8.6 | 6.4 | 9.0 | 8.4 | 8.5 | 7.4 | 7.7 |
| hsa-miR-4327 | 9.1 | 9.7 | 10.0 | 7.8 | 8.9 | 8.7 | 8.6 | 8.8 | 7.8 |
| hsa-miR-6781-5p | 9.2 | 10.5 | 10.5 | 9.5 | 10.0 | 10.6 | 9.2 | 10.7 | 8.5 |
| hsa-miR-1913 | 9.0 | 8.8 | 9.0 | 7.3 | 9.3 | 8.9 | 9.5 | 8.1 | 8.7 |
| hsa-miR-3142 | 0.0 | 0.0 | 0.0 | 7.3 | 9.3 | 8.9 | 3.2 | 0.0 | 0.0 |
| hsa-miR-0642a-3p | 11.0 | 10.0 | 9.3 | 6.3 | 8.5 | 9.5 | 8.7 | 7.7 | 7.5 |
| hsa-miR-6756-5p | 9.7 | 9.9 | 9.6 | 8.5 | 9.4 | 9.7 | 10.1 | 10.0 | 9.7 |
| hsa-miR-4731-5p | 10.2 | 9.3 | 9.2 | 7.1 | 8.8 | 9.4 | 9.4 | 7.9 | 8.6 |
| hsa-miR-5739 | 10.3 | 8.7 | 9.9 | 6.9 | 9.4 | 9.6 | 8.6 | 7.8 | 7.4 |
| hsa-miR-6766-3p | 9.4 | 9.0 | 9.2 | 7.6 | 9.2 | 8.9 | 9.7 | 8.3 | 8.5 |
| hsa-miR-4741 | 9.7 | 9.8 | 11.1 | 8.6 | 10.6 | 10.0 | 9.2 | 9.8 | 8.6 |
| hsa-miR-8063 | 9.8 | 10.2 | 10.1 | 9.3 | 10.3 | 9.6 | 10.9 | 11.0 | 10.8 |
| hsa-miR-1249-3p | 9.2 | 9.0 | 9.2 | 7.5 | 9.4 | 8.8 | 9.8 | 8.1 | 8.8 |
| hsa-miR-1914-3p | 9.7 | 8.6 | 10.4 | 7.4 | 11.2 | 10.2 | 9.0 | 7.9 | 8.4 |
| hsa-miR-3143 | 0.0 | 0.0 | 0.0 | 7.4 | 11.2 | 10.2 | 0.0 | 0.0 | 0.0 |
| hsa -miR -6858-5p | 10.9 | 10.1 | 9.9 | 8.1 | 9.3 | 10.4 | 9.8 | 9.3 | 8.9 |
| hsa-miR-3679-3p | 9.2 | 8.9 | 9.2 | 7.4 | 9.4 | 8.9 | 9.7 | 8.3 | 8.6 |
| hsa-miR-1227-5p | 9.8 | 10.7 | 10.7 | 9.4 | 10.3 | 10.4 | 10.0 | 10.7 | 9.5 |
| hsa-miR-6779-5p | 11.1 | 10.1 | 9.9 | 7.8 | 9.9 | 10.7 | 9.8 | 9.1 | 8.9 |
| hsa -miR -6836-3p | 9.0 | 9.6 | 9.9 | 9.1 | 10.3 | 9.6 | 10.1 | 9.9 | 9.5 |
| hsa-miR-5195-3p | 11.4 | 11.0 | 9.9 | 7.8 | 9.4 | 10.3 | 10.3 | 8.9 | 9.4 |
| hsa-miR-3180-3p | 9.9 | 10.3 | 10.4 | 9.2 | 10.2 | 10.3 | 9.6 | 10.1 | 9.5 |
| hsa-miR-4446-3p | 9.5 | 10.4 | 10.4 | 9.1 | 10.3 | 10.1 | 10.1 | 10.4 | 9.6 |
| hsa-miR-1915-3p | 9.2 | 11.5 | 11.1 | 10.3 | 11.4 | 11.5 | 10.1 | 11.5 | 9.7 |
| hsa-miR-3144-5p | 8.2 | 7.5 | 7.3 | 10.3 | 11.4 | 11.5 | 6.2 | 5.2 | 5.7 |
| hsa-miR-1908-5p | 10.0 | 11.1 | 11.3 | 9.9 | 11.1 | 11.8 | 9.7 | 11.2 | 9.3 |
| hsa -miR -3136-3p | 0.0 | 0.0 | 0.0 | 9.9 | 11.1 | 11.8 | 0.0 | 0.0 | 0.0 |
| hsa-miR-6800-5p | 10.6 | 10.6 | 10.9 | 9.3 | 10.4 | 10.3 | 10.2 | 10.4 | 10.1 |
| hsa -miR -0486-3p | 9.7 | 10.8 | 10.8 | 9.5 | 10.7 | 10.4 | 10.6 | 10.6 | 10.2 |
| hsa-miR-4294 | 10.4 | 10.3 | 9.9 | 8.0 | 10.1 | 10.0 | 9.5 | 8.8 | 8.5 |
| hsa-miR-4697-5p | 11.0 | 10.7 | 10.0 | 8.5 | 9.9 | 10.9 | 9.9 | 9.4 | 9.8 |
| hsa-miR-6515-3p | 10.0 | 9.7 | 10.0 | 7.9 | 10.0 | 9.8 | 10.6 | 9.2 | 9.3 |
| hsa-miR-3197 | 10.5 | 10.2 | 11.9 | 8.9 | 10.6 | 11.7 | 9.3 | 10.7 | 8.9 |
| hsa-miR-3180 | 10.5 | 10.6 | 10.7 | 9.6 | 10.1 | 10.4 | 10.3 | 10.4 | 9.8 |
| hsa-miR-6795-5p | 9.6 | 8.6 | 9.5 | 6.3 | 9.6 | 8.5 | 8.8 | 7.8 | 8.5 |
| hsa-miR-6819-5p | 11.5 | 10.9 | 10.5 | 8.1 | 10.6 | 11.2 | 10.2 | 9.7 | 9.5 |
| hsa-miR-1469 | 10.5 | 11.2 | 11.8 | 10.2 | 11.1 | 11.6 | 10.2 | 11.2 | 10.0 |
| hsa-miR-3128 | 0.0 | 0.0 | 0.0 | 10.2 | 11.1 | 11.6 | 0.0 | 0.0 | 0.0 |
| hsa-miR-7106-5p | 8.8 | 8.4 | 8.4 | 5.4 | 8.1 | 7.9 | 7.5 | 6.5 | 6.5 |
| hsa-miR-4728-5p | 10.0 | 9.2 | 11.0 | 7.1 | 11.3 | 10.4 | 8.9 | 7.7 | 8.3 |
| hsa -miR -6802-5p | 12.0 | 10.8 | 10.5 | 8.6 | 9.9 | 10.8 | 10.6 | 9.7 | 10.0 |
| hsa-miR-6749-5p | 11.4 | 10.5 | 10.5 | 8.6 | 10.2 | 10.7 | 9.9 | 9.7 | 8.9 |
| hsa-miR-0371a-5p | 11.2 | 10.4 | 10.1 | 7.9 | 9.6 | 10.6 | 10.2 | 9.1 | 9.4 |
| hsa-miR-4675 | 11.4 | 10.8 | 10.7 | 8.9 | 10.1 | 10.5 | 11.3 | 9.7 | 10.3 |
| hsa-miR-1343-5p | 10.2 | 10.5 | 11.1 | 9.4 | 10.9 | 11.4 | 9.9 | 10.9 | 9.5 |
| hsa -miR -3126-5p | 3.8 | 3.7 | 2.3 | 9.4 | 10.9 | 11.4 | 0.0 | 2.4 | 4.3 |
| hsa-miR-0149-3p | 11.1 | 10.9 | 10.9 | 9.5 | 10.4 | 10.8 | 10.6 | 10.6 | 10.2 |
| hsa-miR-1233-5p | 12.7 | 11.1 | 11.6 | 8.4 | 10.5 | 10.7 | 9.6 | 10.3 | 8.9 |
| hsa-miR-6125 | 10.6 | 12.8 | 12.5 | 11.6 | 12.5 | 13.0 | 10.5 | 13.0 | 10.3 |
| hsa-miR-6887-5p | 10.1 | 9.1 | 9.9 | 7.3 | 10.2 | 9.4 | 9.1 | 8.6 | 9.0 |
| hsa-miR-6775-5p | 11.3 | 10.3 | 10.4 | 8.3 | 9.7 | 10.1 | 10.3 | 9.3 | 9.3 |
| hsa-miR-6850-5p | 10.6 | 12.2 | 12.0 | 11.1 | 12.2 | 12.1 | 11.1 | 12.3 | 10.8 |
| hsa-miR-4534 | 11.1 | 9.7 | 11.9 | 7.4 | 12.2 | 10.3 | 10.0 | 8.2 | 9.5 |
| hsa -miR -0296-5p | 11.0 | 10.4 | 10.8 | 8.8 | 10.8 | 10.6 | 11.3 | 9.9 | 10.3 |
| hsa-miR-6803-5p | 12.2 | 11.9 | 11.9 | 10.3 | 11.3 | 12.1 | 11.3 | 11.8 | 10.5 |
| hsa-miR-0663a | 11.0 | 11.5 | 11.5 | 10.4 | 11.4 | 11.3 | 11.2 | 11.6 | 10.7 |
| hsa-miR-6752-5p | 11.2 | 12.0 | 12.3 | 10.7 | 11.9 | 12.1 | 11.1 | 11.8 | 10.9 |
| hsa-miR-4492 | 10.8 | 11.5 | 11.8 | 10.4 | 11.1 | 10.9 | 10.7 | 10.7 | 10.5 |
| hsa-miR-4270 | 12.5 | 11.5 | 11.2 | 9.4 | 10.4 | 11.6 | 11.6 | 10.0 | 10.8 |
| hsa-miR-6816-5p | 11.1 | 11.7 | 11.7 | 10.6 | 11.5 | 11.5 | 11.3 | 11.6 | 11.3 |
| hsa-miR-4687-3p | 13.0 | 12.2 | 11.5 | 9.1 | 10.8 | 12.2 | 10.7 | 10.8 | 10.2 |
| hsa-miR-6771-5p | 11.8 | 11.4 | 11.3 | 10.4 | 11.1 | 12.0 | 11.6 | 10.6 | 11.0 |
| hsa-miR-3656 | 11.2 | 12.4 | 12.3 | 11.3 | 12.4 | 12.4 | 11.4 | 12.5 | 11.3 |
| hsa-miR-4730 | 10.9 | 12.5 | 11.8 | 9.6 | 11.0 | 10.5 | 12.2 | 11.7 | 11.4 |
| hsa-miR-4463 | 11.8 | 12.0 | 11.8 | 10.6 | 11.8 | 11.8 | 11.7 | 11.9 | 11.6 |
| hsa-miR-6805-5p | 11.9 | 12.5 | 12.6 | 10.9 | 11.8 | 12.7 | 11.6 | 12.5 | 11.4 |
| hsa-miR-1268a | 11.8 | 12.3 | 12.6 | 11.0 | 11.9 | 12.3 | 11.4 | 12.3 | 11.4 |
| hsa-miR-8072 | 11.6 | 13.5 | 13.2 | 12.2 | 13.1 | 13.3 | 11.6 | 13.2 | 11.2 |
| hsa-miR-6757-5p | 13.7 | 11.5 | 12.4 | 8.1 | 11.1 | 11.0 | 10.0 | 9.7 | 8.9 |
| hsa-miR-4281 | 13.4 | 12.5 | 12.5 | 10.1 | 11.7 | 12.2 | 12.2 | 11.1 | 11.1 |
| hsa-miR-3663-3p | 11.0 | 12.8 | 12.3 | 11.4 | 12.5 | 12.3 | 12.6 | 12.8 | 12.3 |
| hsa-miR-6786-5p | 12.7 | 13.2 | 13.2 | 11.8 | 12.8 | 13.3 | 11.6 | 13.0 | 11.3 |
| hsa-miR-6765-5p | 12.1 | 12.2 | 12.3 | 11.2 | 11.9 | 12.1 | 12.3 | 12.0 | 11.9 |
| hsa-miR-6729-5p | 11.5 | 13.3 | 13.0 | 12.1 | 13.1 | 13.3 | 12.1 | 13.4 | 11.6 |
| hsa-miR-6087 | 13.3 | 13.0 | 13.2 | 11.3 | 11.9 | 13.2 | 13.0 | 12.7 | 11.8 |
| hsa-miR-6088 | 13.2 | 12.8 | 12.5 | 10.9 | 12.3 | 12.4 | 13.2 | 12.1 | 12.7 |
| hsa-miR-0638 | 11.9 | 13.2 | 13.0 | 12.1 | 13.2 | 13.3 | 11.9 | 13.3 | 11.6 |
| hsa-miR-4674 | 11.6 | 12.9 | 12.6 | 11.5 | 12.7 | 12.2 | 12.5 | 12.5 | 11.5 |
| hsa-miR-4734 | 11.9 | 13.4 | 13.2 | 12.2 | 12.9 | 13.1 | 12.6 | 13.3 | 11.6 |
| hsa-miR-4689 | 13.3 | 12.5 | 12.2 | 9.9 | 11.4 | 12.5 | 12.2 | 11.1 | 11.2 |
| hsa-miR-6784-5p | 12.7 | 12.9 | 13.5 | 12.1 | 13.1 | 13.1 | 12.6 | 13.0 | 12.4 |
| hsa-miR-6785-5p | 12.9 | 12.6 | 12.3 | 11.2 | 12.3 | 12.5 | 13.0 | 11.5 | 12.7 |
| hsa-miR-3621 | 11.9 | 13.1 | 12.8 | 12.2 | 13.1 | 12.8 | 13.1 | 13.4 | 12.8 |
| hsa-miR-0328-5p | 13.7 | 13.0 | 12.8 | 10.8 | 11.9 | 13.1 | 12.2 | 12.2 | 11.6 |
| hsa-miR-4739 | 14.5 | 13.3 | 13.3 | 10.7 | 12.2 | 13.4 | 12.1 | 11.9 | 11.3 |
| hsa-miR-6085 | 13.7 | 12.0 | 12.8 | 10.0 | 12.0 | 12.4 | 11.4 | 10.8 | 10.4 |
| hsa-miR-4442 | 14.0 | 12.9 | 12.6 | 10.4 | 12.2 | 13.4 | 13.2 | 11.6 | 12.6 |
| hsa-miR-4649-5p | 12.4 | 13.2 | 12.6 | 11.9 | 12.6 | 12.5 | 13.9 | 12.7 | 13.1 |
| hsa-miR-3196 | 12.8 | 13.4 | 13.4 | 12.2 | 13.3 | 13.1 | 12.6 | 13.0 | 12.1 |
| hsa-miR-4466 | 12.6 | 13.7 | 13.6 | 12.4 | 13.4 | 13.5 | 12.8 | 13.5 | 12.2 |
| hsa-miR-4508 | 12.5 | 13.7 | 13.7 | 12.4 | 13.4 | 13.4 | 12.5 | 13.4 | 12.2 |
| hsa-miR-1237-5p | 12.8 | 13.6 | 13.6 | 12.5 | 13.5 | 13.4 | 13.1 | 13.5 | 13.1 |
| hsa-miR-4532 | 12.2 | 13.6 | 13.5 | 11.9 | 13.5 | 12.9 | 12.6 | 13.2 | 10.9 |
| hsa-miR-6090 | 12.8 | 13.8 | 13.7 | 12.7 | 13.5 | 13.8 | 13.4 | 13.8 | 13.0 |
| hsa-miR-2861 | 14.2 | 13.5 | 13.6 | 12.0 | 13.2 | 13.9 | 13.1 | 13.3 | 12.7 |
| hsa-miR-3163 | 0.0 | 0.0 | 0.0 | 12.0 | 13.2 | 13.9 | 0.0 | 0.0 | 0.0 |
| hsa-miR-4723-5p | 13.6 | 11.9 | 12.7 | 9.7 | 12.9 | 12.7 | 11.7 | 10.1 | 10.9 |
| hsa-miR-3178 | 12.9 | 13.7 | 13.7 | 12.5 | 13.5 | 13.5 | 13.3 | 13.6 | 12.4 |
| hsa-miR-0128-2-5p | 13.9 | 13.9 | 13.3 | 12.0 | 12.9 | 13.3 | 14.1 | 12.7 | 13.3 |
| hsa-miR-1228-5p | 13.9 | 13.4 | 13.6 | 12.3 | 13.1 | 13.5 | 12.7 | 13.2 | 12.6 |
| hsa-miR-0762 | 13.3 | 14.1 | 14.4 | 13.2 | 14.2 | 13.8 | 14.0 | 14.2 | 13.7 |
| hsa-miR-4488 | 13.6 | 14.3 | 14.2 | 12.9 | 13.9 | 14.0 | 14.5 | 14.0 | 13.9 |
| hsa-miR-8069 | 13.7 | 14.5 | 14.3 | 13.1 | 14.1 | 14.2 | 13.7 | 14.3 | 12.9 |
| hsa-miR-3940-5p | 13.6 | 13.9 | 14.1 | 12.6 | 13.7 | 13.7 | 13.5 | 14.2 | 13.6 |
| hsa-miR-6869-5p | 13.0 | 14.5 | 14.3 | 13.0 | 14.1 | 13.8 | 13.8 | 14.1 | 13.2 |
| hsa-miR-6089 | 13.7 | 14.6 | 14.5 | 13.2 | 14.5 | 14.3 | 13.9 | 14.5 | 13.5 |
| hsa-miR-6727-5p | 13.8 | 14.7 | 14.4 | 13.2 | 14.5 | 14.2 | 14.3 | 14.5 | 13.5 |
| hsa-miR-6885-5p | 14.6 | 14.0 | 13.9 | 12.7 | 13.7 | 13.8 | 14.6 | 13.3 | 13.9 |
| hsa-miR-5787 | 13.5 | 14.8 | 14.8 | 13.5 | 14.4 | 13.7 | 14.7 | 14.4 | 14.2 |
| hsa-miR-4787-5p | 14.1 | 14.9 | 14.8 | 13.6 | 14.6 | 14.5 | 14.3 | 14.6 | 13.6 |
| hsa-miR-7704 | 14.2 | 15.2 | 15.0 | 13.8 | 14.9 | 14.7 | 14.8 | 14.9 | 13.9 |
| hsa-miR-4516 | 14.2 | 15.3 | 15.1 | 14.1 | 15.2 | 14.6 | 15.4 | 15.2 | 14.8 |
| hsa-miR-3665 | 14.7 | 15.8 | 15.5 | 14.3 | 15.6 | 15.1 | 15.4 | 15.4 | 14.6 |
| hsa-miR-3960 | 15.4 | 15.9 | 16.4 | 15.4 | 16.2 | 15.1 | 16.4 | 15.7 | 15.6 |

### [Table 3-2]

**Table 3-2: Data S1 (Liver cancer, Bladder cancer, Prostate cancer)**

| miRNA name | Liver cancer | | | Bladder cancer | | | Prostate cancer | | |
|---|---|---|---|---|---|---|---|---|---|
| hsa-let-7a-5p | 0.0 | 0.0 | 0.0 | 0.0 | 2.2 | 0.0 | 0.0 | 0.0 | 0.0 |
| hsa-let-7c-5p | 1.5 | 0.0 | 0.0 | 1.8 | 0.6 | 0.0 | 0.0 | 0.0 | 0.0 |
| hsa-let-7d-5p | 0.0 | 0.0 | 0.0 | 0.0 | 1.4 | 0.0 | 0.0 | 0.0 | 1.1 |
| hsa-let-7e-3p | 0.0 | 2.2 | 0.0 | 0.0 | 2.2 | 1.7 | 1.4 | 0.0 | 0.0 |
| hsa-let-7f-2-3p | 0.0 | 2.2 | 0.0 | 0.0 | 0.0 | 0.0 | 2.5 | 0.2 | 2.4 |
| hsa-let-7f-5p | 1.1 | 0.0 | 0.0 | 0.0 | 0.0 | 0.0 | 0.0 | 0.0 | 0.0 |
| hsa-let-7g-3p | 0.0 | 0.0 | 0.0 | 0.0 | 0.0 | 0.0 | 0.0 | 0.0 | 0.0 |
| hsa-let-7i-3p | 0.0 | 0.0 | 0.0 | 0.0 | 0.0 | 0.0 | 0.0 | 0.0 | 0.0 |
| hsa-miR-0001-3p | 0.0 | 0.0 | 0.0 | 0.0 | 0.0 | 0.0 | 0.0 | 0.0 | 0.0 |
| hsa-miR-0001-5p | 1.9 | 2.5 | 1.8 | 1.7 | 2.5 | 1.9 | 0.0 | 0.0 | 0.0 |
| hsa-miR-0009-5p | 1.1 | 0.0 | 0.0 | 0.0 | 0.0 | 0.0 | 0.0 | 0.0 | 0.0 |
| hsa-miR-0015a-3p | 0.0 | 3.0 | 3.0 | 1.7 | 0.0 | 1.8 | 2.5 | 0.0 | 0.0 |
| hsa-miR-0015a-5p | 0.0 | 0.0 | 1.3 | 0.0 | 0.0 | 0.0 | 0.0 | 0.0 | 0.0 |
| hsa-miR-0015b-3p | 0.0 | 1.2 | 0.0 | 0.0 | 0.0 | 0.0 | 0.0 | 0.0 | 0.0 |
| hsa-miR-0016-2-3p | 0.0 | 2.6 | 2.8 | 3.4 | 2.6 | 3.4 | 2.9 | 0.0 | 2.9 |
| hsa-miR-0016-5p | 0.0 | 0.0 | 0.0 | 2.0 | 0.0 | 0.0 | 0.0 | 0.0 | 0.0 |
| hsa-miR-0017-5p | 0.0 | 0.0 | 1.9 | 0.0 | 1.8 | 0.0 | 0.0 | 0.0 | 0.0 |
| hsa-miR-0018b-5p | 0.0 | 0.0 | 0.0 | 0.0 | 0.0 | 0.0 | 0.0 | 0.0 | 0.0 |
| hsa-miR-0019a-3p | 0.0 | 0.0 | 0.0 | 0.0 | 0.0 | 0.0 | 0.0 | 0.0 | 0.0 |
| hsa-miR-0019a-5p | 2.2 | 0.0 | 0.0 | 0.0 | 0.0 | 0.0 | 0.0 | 0.0 | 0.0 |
| hsa-miR-0019b-1-5p | 0.0 | 0.0 | 0.0 | 0.0 | 0.0 | 0.0 | 0.0 | 0.0 | 0.0 |
| hsa-miR-0019b-2-5p | 0.0 | 0.0 | 0.0 | 0.0 | 0.0 | 0.0 | 0.0 | 0.0 | 0.0 |
| hsa -miR -0020b-5p | 1.3 | 0.0 | 2.8 | 2.7 | 2.2 | 0.0 | 0.0 | 0.0 | 0.0 |
| hsa-miR-0021-5p | 0.0 | 0.0 | 0.0 | 0.0 | 1.1 | 0.0 | 0.0 | 0.0 | 0.0 |
| hsa -miR -0022-5p | 0.0 | 1.5 | 1.7 | 2.8 | 0.7 | 0.0 | 0.0 | 0.5 | 0.0 |
| hsa-miR-0023b-5p | 0.0 | 1.8 | 1.1 | 0.0 | 0.0 | 0.0 | 0.0 | 0.0 | 0.0 |
| hsa -miR -0023c | 0.0 | 0.0 | 2.1 | 0.0 | 1.6 | 0.0 | 0.0 | 0.0 | 1.8 |
| hsa-miR-0024-1-5p | 0.0 | 0.0 | 0.0 | 0.0 | 3.2 | 0.0 | 0.0 | 0.0 | 0.0 |
| hsa-miR-0024-2-5p | 2.2 | 1.5 | 3.2 | 2.1 | 0.0 | 1.1 | 0.0 | 0.0 | 0.0 |
| hsa -miR -0024-3p | 3.2 | 1.8 | 3.5 | 3.1 | 3.7 | 1.6 | 2.2 | 2.5 | 2.2 |
| hsa-miR-0026a-2-3p | 2.0 | 0.0 | 2.6 | 0.0 | 1.5 | 0.0 | 0.0 | 0.0 | 0.0 |
| hsa-miR-0026a-5p | 0.0 | 0.0 | 0.0 | 0.0 | 0.0 | 0.0 | 0.0 | 0.0 | 0.0 |
| hsa-miR-0027a-5p | 0.0 | 0.0 | 0.0 | 0.0 | 0.7 | 0.0 | 0.0 | 0.0 | 0.0 |
| hsa -miR -0028-3p | 0.0 | 2.3 | 0.0 | 0.0 | 2.0 | 0.0 | 0.0 | 0.0 | 0.0 |
| hsa-miR-0029a-5p | 0.0 | 0.0 | 0.0 | 0.0 | 2.2 | 0.0 | 0.0 | 0.0 | 0.0 |
| hsa-miR-0029b-1-5p | 0.0 | 0.0 | 0.0 | 0.0 | 0.0 | 0.0 | 0.0 | 0.0 | 0.0 |
| hsa -miR -0029c-3p | 0.0 | 0.0 | 0.0 | 0.0 | 0.0 | 0.0 | 0.0 | 1.1 | 0.0 |
| hsa-miR-0030a-5p | 0.0 | 0.0 | 0.0 | 0.0 | 1.5 | 0.0 | 0.0 | 0.0 | 0.0 |
| hsa-miR-0031-3p | 0.0 | 0.0 | 2.0 | 0.0 | 2.3 | 0.0 | 0.0 | 0.0 | 0.0 |
| hsa-miR-0031-5p | 0.0 | 0.0 | 2.1 | 0.0 | 0.0 | 0.0 | 0.0 | 0.0 | 0.0 |
| hsa -miR -0032-3p | 0.0 | 2.3 | 3.5 | 2.9 | 2.2 | 1.9 | 2.5 | 2.5 | 2.7 |
| hsa -miR -0032-5p | 0.0 | 0.0 | 0.0 | 0.0 | 0.0 | 0.0 | 0.0 | 0.0 | 0.0 |
| hsa-miR-0033a-3p | 3.3 | 1.8 | 2.7 | 3.2 | 0.0 | 2.0 | 0.0 | 1.9 | 1.9 |
| hsa-miR-0033a-5p | 0.0 | 1.0 | 0.0 | 0.0 | 0.0 | 0.0 | 0.0 | 0.0 | 0.0 |
| hsa-miR-0033b-5p | 2.4 | 0.0 | 0.0 | 0.0 | 0.0 | 0.0 | 0.0 | 0.0 | 0.0 |
| hsa-miR-0034a-3p | 0.0 | 0.0 | 0.0 | 0.0 | 0.0 | 0.0 | 0.0 | 0.0 | 0.0 |
| hsa-miR-0095-5p | 0.0 | 0.0 | 0.0 | 0.0 | 0.0 | 0.0 | 0.0 | 0.0 | 0.0 |
| hsa -miR -0096-5p | 1.7 | 0.0 | 2.9 | 0.0 | 0.0 | 0.0 | 0.0 | 0.0 | 0.0 |
| hsa-miR-0099a-5p | 0.0 | 0.0 | 0.0 | 0.0 | 0.0 | 0.0 | 0.0 | 0.0 | 0.0 |
| hsa-miR-0100-3p | 0.0 | 0.0 | 0.0 | 0.0 | 0.0 | 0.0 | 0.0 | 0.0 | 0.0 |
| hsa-miR-0100-5p | 0.0 | 0.0 | 0.0 | 0.0 | 0.6 | 0.0 | 0.0 | 0.0 | 0.0 |
| hsa-miR-0101-3p | 0.0 | 0.0 | 0.0 | 0.0 | 0.0 | 0.0 | 0.0 | 0.0 | 0.0 |
| hsa-miR-0101-5p | 0.0 | 0.0 | 1.6 | 0.0 | 0.0 | 0.0 | 0.0 | 0.0 | 0.0 |
| hsa-miR-0103a-3p | 2.5 | 0.0 | 3.3 | 3.2 | 0.5 | 1.2 | 2.0 | 1.3 | 1.9 |
| hsa-miR-0103b | 2.1 | 1.7 | 0.0 | 0.0 | 0.0 | 0.0 | 0.0 | 0.0 | 0.0 |
| hsa-miR-0105-3p | 0.0 | 0.0 | 0.0 | 0.0 | 0.0 | 0.0 | 0.0 | 0.0 | 0.0 |
| hsa-miR-0106a-3p | 0.0 | 0.0 | 1.2 | 0.0 | 0.0 | 0.0 | 0.0 | 0.0 | 0.0 |
| hsa-miR-0106a-5p | 0.0 | 0.0 | 0.0 | 0.0 | 0.0 | 0.0 | 0.0 | 0.0 | 0.0 |
| hsa-miR-0106b-5p | 0.0 | 1.8 | 0.0 | 0.0 | 1.3 | 0.0 | 0.0 | 0.0 | 0.0 |
| hsa-miR-0107 | 0.0 | 0.0 | 3.0 | 0.0 | 0.6 | 0.0 | 2.3 | 0.3 | 1.4 |
| hsa-miR-0122-3p | 0.0 | 2.1 | 2.3 | 1.9 | 0.0 | 0.0 | 0.0 | 0.0 | 0.0 |
| hsa-miR-0124-3p | 2.2 | 0.0 | 0.0 | 1.6 | 2.1 | 0.0 | 0.0 | 3.5 | 5.7 |
| hsa-miR-0125b-1-3p | 0.0 | 2.9 | 0.0 | 0.0 | 0.0 | 0.0 | 0.0 | 0.0 | 1.1 |
| hsa-miR-0126-5p | 0.0 | 0.0 | 0.0 | 0.0 | 0.0 | 0.0 | 0.0 | 0.0 | 0.0 |
| hsa-miR-0127-3p | 0.0 | 0.0 | 0.0 | 1.9 | 0.0 | 0.0 | 0.0 | 0.0 | 0.0 |
| hsa-miR-0130a-3p | 0.0 | 0.0 | 0.0 | 1.8 | 0.0 | 0.0 | 0.0 | 0.0 | 0.0 |
| hsa-miR-0130b-3p | 0.0 | 0.0 | 0.0 | 0.0 | 0.5 | 0.0 | 0.0 | 0.0 | 1.2 |
| hsa-miR-0132-3p | 2.5 | 1.5 | 2.2 | 0.0 | 2.6 | 0.0 | 1.4 | 0.0 | 0.0 |
| hsa-miR-0132-5p | 0.0 | 0.0 | 0.0 | 0.0 | 0.0 | 0.0 | 0.0 | 0.0 | 0.0 |
| hsa-miR-0133a-5p | 1.8 | 1.7 | 1.4 | 0.0 | 0.0 | 0.0 | 1.8 | 0.0 | 0.0 |
| hsa-miR-0135b-3p | 0.0 | 0.0 | 0.0 | 0.0 | 0.0 | 0.0 | 0.0 | 0.0 | 0.0 |
| hsa-miR-0136-5p | 0.0 | 0.0 | 0.0 | 0.0 | 0.0 | 0.0 | 0.0 | 0.0 | 0.0 |
| hsa-miR-0139-5p | 0.0 | 0.0 | 0.0 | 0.0 | 0.0 | 0.0 | 0.0 | 0.0 | 0.0 |
| hsa-miR-0140-3p | 0.0 | 0.0 | 0.0 | 1.5 | 0.0 | 0.0 | 0.0 | 0.0 | 0.0 |
| hsa-miR-0140-5p | 0.0 | 0.0 | 0.0 | 0.0 | 2.1 | 0.0 | 0.0 | 0.0 | 0.0 |
| hsa-miR-0141-3p | 0.0 | 0.0 | 0.0 | 0.0 | 0.0 | 0.0 | 0.0 | 1.0 | 0.0 |
| hsa-miR-0142-5p | 0.0 | 0.0 | 0.0 | 0.0 | 1.4 | 0.0 | 0.0 | 0.0 | 0.0 |
| hsa-miR-0144-3p | 0.0 | 0.0 | 0.0 | 0.0 | 0.0 | 0.0 | 0.0 | 0.0 | 0.0 |
| hsa-miR-0145-3p | 0.0 | 0.0 | 0.0 | 0.0 | 0.0 | 0.0 | 0.0 | 0.0 | 0.0 |
| hsa-miR-0146a-3p | 0.0 | 0.0 | 0.0 | 0.0 | 0.0 | 0.0 | 0.0 | 0.0 | 0.0 |
| hsa-miR-0146b-5p | 0.0 | 0.0 | 0.0 | 0.0 | 0.0 | 0.0 | 0.0 | 0.0 | 0.0 |
| hsa-miR-0148a-5p | 0.0 | 0.0 | 1.1 | 0.0 | 0.0 | 0.0 | 0.0 | 0.0 | 0.0 |
| hsa-miR-0148b-3p | 0.0 | 0.0 | 0.0 | 0.0 | 1.1 | 0.0 | 0.0 | 0.0 | 0.0 |
| hsa-miR-0148b-5p | 2.0 | 1.0 | 0.0 | 0.0 | 0.0 | 0.0 | 0.0 | 0.0 | 0.0 |
| hsa-miR-0151a-3p | 2.4 | 1.9 | 2.4 | 2.2 | 0.0 | 1.0 | 0.0 | 0.0 | 0.0 |
| hsa-miR-0151a-5p | 0.0 | 0.0 | 1.9 | 3.5 | 2.4 | 0.0 | 3.8 | 1.8 | 3.3 |
| hsa-miR-0152-3p | 1.6 | 0.0 | 1.1 | 0.0 | 1.7 | 0.0 | 0.0 | 0.0 | 0.0 |
| hsa-miR-0153-3p | 0.0 | 0.0 | 0.0 | 0.0 | 0.0 | 0.0 | 0.0 | 0.0 | 0.0 |
| hsa-miR-0154-3p | 0.0 | 3.0 | 2.6 | 0.0 | 0.0 | 1.6 | 2.2 | 0.0 | 1.9 |
| hsa-miR-0155-3p | 0.0 | 2.6 | 1.1 | 0.0 | 0.0 | 0.0 | 2.2 | 0.0 | 0.0 |
| hsa-miR-0155-5p | 0.0 | 0.0 | 0.0 | 0.0 | 0.0 | 0.0 | 0.0 | 0.0 | 0.0 |
| hsa-miR-0181a-3p | 2.2 | 0.0 | 0.0 | 0.0 | 0.0 | 0.0 | 0.0 | 0.0 | 0.0 |
| hsa-miR-0181b-2-3p | 0.0 | 0.0 | 0.0 | 0.0 | 0.0 | 0.0 | 0.0 | 0.0 | 0.0 |
| hsa-miR-0181c-3p | 0.0 | 1.4 | 0.0 | 0.0 | 0.0 | 0.0 | 0.0 | 1.5 | 0.0 |
| hsa-miR-0181c-5p | 0.0 | 0.0 | 0.0 | 0.0 | 0.0 | 0.0 | 0.0 | 0.0 | 0.0 |
| hsa-miR-0181d-5p | 0.0 | 2.6 | 1.9 | 1.5 | 0.0 | 1.6 | 0.0 | 0.0 | 0.0 |
| hsa-miR-0182-3p | 0.0 | 0.0 | 0.0 | 0.0 | 0.0 | 0.0 | 0.0 | 0.0 | 0.0 |
| hsa-miR-0182-5p | 0.0 | 0.0 | 2.3 | 0.0 | 0.0 | 0.0 | 0.0 | 0.0 | 0.0 |
| hsa-miR-0186-3p | 0.0 | 0.0 | 0.0 | 0.0 | 0.0 | 0.0 | 0.0 | 0.1 | 1.4 |
| hsa-miR-0186-5p | 0.0 | 0.0 | 0.0 | 0.0 | 0.0 | 0.0 | 0.0 | 0.0 | 0.0 |
| hsa-miR-0188-3p | 1.7 | 0.0 | 2.2 | 2.2 | 1.4 | 0.0 | 0.0 | 1.5 | 1.7 |
| hsa-miR-0190a-3p | 0.0 | 0.0 | 0.0 | 0.0 | 3.4 | 0.0 | 0.0 | 0.0 | 0.0 |
| hsa-miR-0190b | 0.0 | 0.0 | 0.0 | 0.0 | 0.0 | 0.0 | 0.0 | 0.0 | 0.0 |
| hsa-miR-0191-5p | 0.0 | 0.0 | 0.0 | 0.0 | 1.1 | 0.0 | 0.0 | 0.0 | 0.0 |
| hsa-miR-0192-3p | 0.0 | 0.0 | 1.1 | 0.0 | 2.9 | 0.0 | 0.0 | 0.0 | 0.0 |
| hsa-miR-0193a-3p | 1.9 | 0.0 | 2.9 | 2.2 | 1.2 | 1.2 | 0.0 | 0.0 | 0.0 |
| hsa-miR-0200a-3p | 2.7 | 0.0 | 0.0 | 0.0 | 0.0 | 0.0 | 0.0 | 0.0 | 0.0 |
| hsa -miR -0200c-3p | 2.7 | 0.0 | 0.0 | 2.2 | 0.0 | 1.8 | 0.0 | 0.0 | 0.0 |
| hsa -miR -0202-5p | 0.0 | 0.0 | 1.2 | 0.0 | 0.0 | 0.0 | 0.0 | 0.0 | 0.0 |
| hsa-miR-0203a-5p | 0.0 | 2.2 | 3.6 | 0.0 | 0.0 | 1.5 | 0.0 | 0.0 | 0.0 |
| hsa-miR-0208a-3p | 0.0 | 0.0 | 0.0 | 0.0 | 0.0 | 0.0 | 0.0 | 0.0 | 0.0 |
| hsa-miR-0208b-3p | 0.0 | 0.0 | 0.0 | 0.0 | 0.0 | 0.0 | 0.0 | 0.0 | 0.0 |
| hsa-miR-0216a-5p | 0.0 | 0.0 | 2.6 | 1.9 | 0.7 | 0.0 | 0.0 | 0.0 | 0.0 |
| hsa-miR-0216b-5p | 0.0 | 0.0 | 0.0 | 0.0 | 0.0 | 0.0 | 0.0 | 0.0 | 0.0 |
| hsa-miR-0218-1-3p | 0.0 | 0.0 | 0.0 | 0.0 | 0.0 | 0.0 | 0.0 | 0.0 | 3.4 |
| hsa-miR-0218-2-3p | 0.0 | 0.0 | 1.3 | 0.0 | 0.0 | 0.0 | 0.0 | 0.0 | 0.0 |
| hsa-miR-0218-5p | 0.0 | 0.0 | 0.0 | 0.0 | 1.5 | 0.0 | 0.0 | 0.0 | 0.0 |
| hsa-miR-0219a-2-3p | 3.5 | 2.4 | 3.1 | 1.8 | 0.0 | 0.0 | 0.0 | 0.6 | 1.2 |
| hsa-miR-0219a-5p | 0.0 | 0.0 | 0.0 | 0.0 | 0.0 | 0.0 | 0.0 | 0.0 | 0.0 |
| hsa -miR -0224-5p | 0.0 | 0.0 | 0.0 | 0.0 | 1.0 | 0.0 | 0.0 | 0.0 | 0.0 |
| hsa-miR-0298 | 0.0 | 1.1 | 0.0 | 1.7 | 0.0 | 0.0 | 0.0 | 0.0 | 0.0 |
| hsa-miR-0299-3p | 3.0 | 1.5 | 1.2 | 3.1 | 3.1 | 0.0 | 0.0 | 1.7 | 2.0 |
| hsa-miR-0301a-3p | 0.0 | 0.0 | 0.0 | 0.0 | 0.0 | 0.0 | 0.0 | 0.0 | 0.0 |
| hsa-miR-0301a-5p | 1.8 | 3.2 | 2.8 | 1.5 | 0.0 | 1.8 | 2.8 | 1.8 | 2.1 |
| hsa-miR-0301b-3p | 0.0 | 0.0 | 0.0 | 0.0 | 0.0 | 0.0 | 0.0 | 0.0 | 0.0 |
| hsa-miR-0301b-5p | 0.0 | 0.0 | 3.0 | 0.0 | 0.0 | 0.0 | 0.0 | 0.4 | 0.0 |
| hsa-miR-0302a-5p | 0.0 | 0.0 | 0.0 | 0.0 | 0.4 | 0.0 | 0.0 | 0.0 | 0.0 |
| hsa-miR-0302b-3p | 1.3 | 0.0 | 0.0 | 0.0 | 0.0 | 0.0 | 0.0 | 0.0 | 0.0 |
| hsa-miR-0302b-5p | 0.0 | 0.0 | 0.0 | 0.0 | 2.8 | 0.0 | 0.0 | 0.0 | 0.0 |
| hsa -miR -0302c-3p | 0.0 | 0.0 | 0.0 | 2.1 | 2.0 | 0.0 | 2.2 | 0.0 | 0.0 |
| hsa-miR-0302d-3p | 0.0 | 0.0 | 2.6 | 2.5 | 0.0 | 0.0 | 0.0 | 0.0 | 0.0 |
| hsa-miR-0302f | 0.0 | 0.0 | 2.5 | 0.0 | 0.0 | 0.0 | 3.0 | 0.0 | 0.0 |
| hsa -miR -0320d | 0.0 | 1.7 | 0.0 | 0.0 | 0.0 | 0.0 | 0.0 | 0.0 | 0.0 |
| hsa -miR -0320e | 1.8 | 1.7 | 1.5 | 1.9 | 0.0 | 0.0 | 0.0 | 1.0 | 1.2 |
| hsa-miR-0323b-3p | 0.0 | 1.5 | 0.0 | 0.0 | 0.0 | 0.0 | 0.0 | 0.0 | 0.0 |
| hsa-miR-0329-3p | 0.0 | 1.0 | 1.8 | 0.0 | 0.8 | 0.0 | 2.1 | 0.0 | 0.0 |
| hsa-miR-0331-5p | 0.0 | 0.0 | 0.0 | 0.0 | 0.0 | 0.0 | 0.0 | 0.0 | 0.0 |
| hsa-miR-0335-3p | 2.2 | 2.4 | 2.8 | 2.4 | 0.9 | 2.3 | 2.2 | 0.0 | 1.2 |
| hsa-miR-0337-5p | 0.0 | 1.1 | 0.0 | 0.0 | 0.0 | 2.2 | 1.5 | 0.0 | 0.0 |
| hsa-miR-0340-5p | 0.0 | 0.0 | 0.0 | 0.0 | 0.0 | 0.0 | 0.0 | 0.0 | 0.0 |
| hsa-miR-0361-5p | 0.0 | 0.0 | 2.2 | 2.0 | 0.0 | 1.2 | 2.4 | 0.0 | 0.0 |
| hsa -miR -0362-5p | 0.0 | 0.0 | 0.0 | 0.0 | 1.8 | 0.0 | 0.0 | 0.0 | 0.0 |
| hsa-miR-0363-3p | 0.0 | 0.0 | 1.8 | 2.8 | 1.1 | 2.0 | 1.5 | 0.0 | 0.0 |
| hsa-miR-0367-5p | 1.0 | 0.0 | 0.0 | 0.0 | 0.0 | 0.0 | 0.0 | 0.0 | 0.0 |
| hsa -miR -0372-5p | 0.0 | 0.0 | 1.9 | 0.0 | 0.0 | 0.0 | 0.0 | 0.0 | 1.2 |
| hsa-miR-0374a-3p | 0.0 | 0.0 | 0.0 | 0.0 | 0.0 | 0.0 | 0.0 | 0.0 | 0.0 |
| hsa-miR-0374a-5p | 0.0 | 0.0 | 2.0 | 0.0 | 0.0 | 0.0 | 0.0 | 0.0 | 0.0 |
| hsa-miR-0374c-5p | 3.4 | 2.5 | 1.4 | 2.6 | 1.2 | 1.4 | 2.0 | 0.0 | 0.0 |
| hsa-miR-0375 | 0.0 | 1.4 | 0.0 | 1.9 | 2.8 | 1.7 | 0.0 | 0.0 | 1.6 |
| hsa-miR-0376a-2-5p | 0.0 | 0.0 | 1.9 | 0.0 | 0.0 | 0.0 | 0.0 | 0.0 | 0.0 |
| hsa-miR-0376a-3p | 0.0 | 0.0 | 0.0 | 0.0 | 0.0 | 0.0 | 0.0 | 0.0 | 0.0 |
| hsa-miR-0376a-5p | 0.0 | 0.0 | 0.0 | 0.0 | 0.0 | 0.0 | 0.0 | 0.0 | 0.0 |
| hsa-miR-0376b-3p | 0.0 | 0.0 | 0.0 | 0.0 | 0.0 | 0.0 | 0.0 | 0.0 | 0.0 |
| hsa-miR-0376b-5p | 0.0 | 0.0 | 0.0 | 0.0 | 0.0 | 0.0 | 0.0 | 0.0 | 0.0 |
| hsa-miR-0376c-5p | 0.0 | 0.0 | 2.0 | 0.0 | 0.0 | 0.0 | 0.0 | 0.0 | 0.0 |
| hsa-miR-0377-3p | 0.0 | 0.0 | 0.0 | 2.4 | 2.9 | 0.0 | 0.0 | 0.0 | 0.0 |
| hsa-miR-0377-5p | 2.4 | 2.3 | 2.8 | 2.0 | 0.0 | 0.0 | 0.0 | 0.8 | 0.0 |
| hsa-miR-0378j | 0.0 | 0.0 | 1.5 | 0.0 | 0.0 | 0.0 | 0.0 | 0.0 | 0.0 |
| hsa-miR-0379-5p | 0.0 | 0.0 | 1.5 | 0.0 | 1.0 | 0.0 | 0.0 | 0.2 | 0.0 |
| hsa-miR-0380-3p | 2.2 | 1.9 | 1.3 | 0.0 | 1.1 | 1.9 | 1.7 | 1.6 | 0.0 |
| hsa-miR-0381-3p | 2.1 | 0.0 | 0.0 | 2.1 | 0.0 | 0.0 | 2.3 | 1.2 | 0.0 |
| hsa-miR-0384 | 0.0 | 0.0 | 0.0 | 0.0 | 0.0 | 0.0 | 0.0 | 0.0 | 0.0 |
| hsa-miR-0409-5p | 0.0 | 0.0 | 1.6 | 0.0 | 1.4 | 0.0 | 0.0 | 0.0 | 0.0 |
| hsa-miR-0410-3p | 1.7 | 0.0 | 0.0 | 0.0 | 0.0 | 0.0 | 0.0 | 0.0 | 0.0 |
| hsa-miR-0411-5p | 0.0 | 0.0 | 0.0 | 0.0 | 0.0 | 0.0 | 0.0 | 0.0 | 0.0 |
| hsa-miR-0412-5p | 0.0 | 0.0 | 0.0 | 0.0 | 0.0 | 0.0 | 0.0 | 0.0 | 0.0 |
| hsa-miR-0424-3p | 0.0 | 2.3 | 1.9 | 0.0 | 0.0 | 0.0 | 0.0 | 5.4 | 3.6 |
| hsa-miR-0424-5p | 0.0 | 0.0 | 2.1 | 0.0 | 0.0 | 0.0 | 0.0 | 0.0 | 0.0 |
| hsa-miR-0429 | 0.0 | 0.0 | 0.0 | 0.0 | 0.0 | 0.0 | 1.4 | 0.0 | 0.0 |
| hsa-miR-0433-3p | 0.0 | 1.6 | 2.4 | 2.5 | 2.2 | 1.2 | 0.0 | 0.0 | 0.0 |
| hsa-miR-0433-5p | 1.1 | 2.4 | 2.8 | 2.0 | 0.5 | 3.6 | 3.1 | 2.4 | 2.9 |
| hsa-miR-0448 | 2.6 | 1.9 | 3.6 | 2.8 | 2.8 | 0.0 | 2.6 | 1.9 | 0.0 |
| hsa-miR-0450a-2-3p | 0.0 | 0.0 | 0.0 | 0.0 | 0.0 | 0.0 | 0.0 | 0.0 | 1.8 |
| hsa-miR-0451a | 0.0 | 0.0 | 0.0 | 0.0 | 0.4 | 0.0 | 0.0 | 0.0 | 0.0 |
| hsa-miR-0451b | 0.0 | 0.0 | 0.0 | 0.0 | 0.0 | 0.0 | 0.0 | 0.0 | 0.0 |
| hsa -miR -0454-3p | 0.0 | 0.0 | 0.0 | 0.0 | 0.0 | 0.0 | 0.0 | 0.0 | 0.0 |
| hsa-miR-0455-3p | 0.0 | 1.6 | 1.8 | 0.0 | 0.0 | 1.2 | 0.0 | 0.0 | 0.0 |
| hsa-miR-0487a-3p | 0.0 | 0.0 | 2.7 | 2.4 | 1.5 | 0.0 | 0.0 | 0.9 | 0.0 |
| hsa-miR-0489-3p | 2.6 | 2.2 | 2.6 | 2.4 | 2.2 | 2.1 | 1.5 | 0.0 | 1.9 |
| hsa-miR-0490-3p | 0.0 | 0.0 | 2.2 | 0.0 | 1.8 | 0.0 | 0.0 | 0.0 | 0.0 |
| hsa-miR-0490-5p | 0.0 | 0.0 | 0.0 | 0.0 | 0.0 | 0.0 | 0.0 | 2.0 | 0.0 |
| hsa-miR-0492 | 2.0 | 2.2 | 2.7 | 3.0 | 2.5 | 3.1 | 2.2 | 1.4 | 2.2 |
| hsa-miR-0493-5p | 0.0 | 0.0 | 1.2 | 0.0 | 0.0 | 2.1 | 0.0 | 0.0 | 0.0 |
| hsa-miR-0495-3p | 0.0 | 0.0 | 0.0 | 0.0 | 0.0 | 0.0 | 0.0 | 0.0 | 0.0 |
| hsa-miR-0496 | 1.6 | 1.7 | 0.0 | 0.0 | 0.0 | 0.0 | 0.0 | 0.0 | 0.0 |
| hsa-miR-0497-5p | 0.0 | 0.0 | 3.4 | 2.1 | 1.3 | 2.3 | 2.1 | 1.5 | 0.0 |
| hsa-miR-0499a-3p | 2.6 | 2.2 | 0.0 | 0.0 | 0.0 | 0.0 | 0.0 | 0.0 | 0.0 |
| hsa-miR-0499a-5p | 0.0 | 0.0 | 0.0 | 0.0 | 0.0 | 0.0 | 0.0 | 0.0 | 0.0 |
| hsa-miR-0500a-3p | 2.3 | 0.0 | 0.0 | 2.1 | 0.0 | 0.0 | 0.0 | 1.4 | 0.0 |
| hsa-miR-0501-3p | 0.0 | 0.0 | 0.0 | 0.0 | 0.0 | 0.0 | 0.0 | 0.0 | 0.0 |
| hsa-miR-0503-5p | 0.0 | 0.0 | 1.5 | 2.4 | 0.0 | 0.0 | 0.0 | 0.9 | 0.0 |
| hsa -miR -0504-5p | 1.6 | 2.8 | 2.4 | 2.3 | 0.0 | 0.0 | 0.0 | 0.0 | 0.0 |
| hsa -miR -0506-5p | 1.2 | 0.0 | 1.8 | 0.0 | 0.0 | 0.0 | 0.0 | 0.0 | 0.0 |
| hsa-miR-0509-5p | 2.7 | 1.0 | 1.6 | 0.0 | 0.0 | 0.0 | 0.0 | 0.0 | 0.0 |
| hsa-miR-0513b-3p | 0.0 | 0.0 | 0.0 | 0.0 | 0.0 | 0.0 | 0.0 | 0.0 | 0.0 |
| hsa-miR-0513c-5p | 2.0 | 3.2 | 3.1 | 1.9 | 0.0 | 1.2 | 0.0 | 0.0 | 0.0 |
| hsa-miR-0514a-5p | 0.0 | 0.0 | 0.0 | 0.0 | 0.0 | 0.0 | 0.0 | 0.0 | 0.0 |
| hsa-miR-0515-5p | 0.0 | 0.0 | 2.5 | 0.0 | 2.5 | 0.0 | 0.0 | 0.0 | 0.0 |
| hsa-miR-0516a-5p | 0.0 | 0.0 | 0.0 | 0.0 | 0.0 | 0.0 | 0.0 | 0.0 | 0.0 |
| hsa-miR-0516b-3p, hsa-miR-516a-3p | 2.4 | 1.8 | 3.1 | 0.0 | 0.0 | 0.0 | 0.0 | 1.7 | 0.0 |
| hsa-miR-0517c-3p | 0.0 | 2.3 | 2.0 | 2.7 | 1.2 | 1.2 | 0.0 | 1.9 | 0.0 |
| hsa-miR-0518a-3p | 0.0 | 0.0 | 0.0 | 0.0 | 0.0 | 0.0 | 0.0 | 0.0 | 0.0 |
| hsa-miR-0518c-3p | 0.0 | 0.0 | 0.0 | 0.0 | 0.0 | 3.5 | 0.0 | 0.0 | 0.0 |
| hsa-miR-0518d-3p | 0.0 | 0.0 | 1.4 | 0.0 | 2.2 | 0.0 | 0.0 | 0.0 | 0.0 |
| hsa -miR -0519c-3p | 0.0 | 0.0 | 0.0 | 0.0 | 0.0 | 0.0 | 0.0 | 0.0 | 0.0 |
| hsa-miR-0520a-5p | 0.0 | 0.0 | 2.0 | 0.0 | 0.0 | 0.0 | 0.0 | 0.0 | 0.0 |
| hsa -miR -0520c-3p | 0.0 | 0.0 | 0.0 | 0.0 | 0.0 | 0.0 | 0.0 | 0.0 | 0.0 |
| hsa-miR-0520d-3p | 0.0 | 2.9 | 2.4 | 0.0 | 0.0 | 0.0 | 0.0 | 0.5 | 0.0 |
| hsa -miR -0520f-3p | 0.0 | 0.0 | 2.4 | 0.0 | 0.0 | 0.0 | 0.0 | 0.0 | 0.0 |
| hsa -miR -0520f-5p | 0.0 | 0.0 | 0.0 | 0.0 | 0.0 | 0.0 | 0.0 | 3.5 | 2.2 |
| hsa -miR -0520g-5p | 0.0 | 0.0 | 0.0 | 0.0 | 0.0 | 0.0 | 1.6 | 0.0 | 2.5 |
| hsa-miR-0521 | 3.0 | 1.8 | 3.6 | 2.8 | 2.8 | 2.1 | 2.7 | 1.6 | 2.6 |
| hsa -miR -0522-3p | 0.0 | 0.0 | 1.7 | 0.0 | 0.0 | 0.0 | 0.0 | 0.0 | 0.0 |
| hsa-miR-0523-3p | 1.2 | 1.6 | 0.0 | 0.0 | 0.0 | 0.0 | 0.0 | 0.0 | 0.0 |
| hsa -miR -0524-3p | 0.0 | 0.0 | 0.0 | 0.0 | 0.0 | 0.0 | 0.0 | 0.0 | 0.0 |
| hsa-miR-0539-5p | 0.0 | 0.0 | 2.5 | 1.9 | 0.0 | 2.1 | 0.0 | 0.0 | 1.2 |
| hsa -miR -0542-5p | 0.0 | 0.0 | 0.0 | 0.0 | 0.0 | 2.2 | 0.0 | 2.5 | 2.8 |
| hsa-miR-0544b | 0.0 | 0.0 | 0.0 | 0.0 | 0.0 | 0.0 | 0.0 | 0.0 | 0.0 |
| hsa-miR-0545-3p | 0.0 | 0.0 | 0.0 | 0.0 | 0.0 | 0.0 | 0.0 | 0.0 | 0.0 |
| hsa-miR-0545-5p | 0.0 | 0.0 | 0.0 | 0.0 | 0.0 | 0.0 | 0.0 | 0.0 | 0.0 |
| hsa-miR-0548a-3p | 0.0 | 0.0 | 0.0 | 0.0 | 1.2 | 0.0 | 0.0 | 0.0 | 0.0 |
| hsa-miR-0548a-5p | 0.0 | 0.0 | 0.0 | 0.0 | 0.0 | 0.0 | 0.0 | 0.0 | 0.0 |
| hsa-miR-0548aa, hsa-miR-548t-3p | 0.0 | 0.0 | 0.0 | 0.0 | 0.0 | 0.0 | 0.0 | 0.0 | 0.0 |
| hsa -miR -0548ab | 0.0 | 0.0 | 0.0 | 0.0 | 1.2 | 2.2 | 0.0 | 0.0 | 0.0 |
| hsa -miR -0548ac | 0.0 | 0.0 | 0.0 | 0.0 | 0.0 | 0.0 | 0.0 | 0.0 | 0.0 |
| hsa -miR -0548ag | 0.0 | 0.0 | 0.0 | 0.0 | 0.0 | 0.0 | 0.0 | 0.0 | 0.0 |
| hsa-miR-0548ah-3p | 0.0 | 0.0 | 0.0 | 0.0 | 2.6 | 0.0 | 0.0 | 0.0 | 0.0 |
| hsa-miR-0548ai, hsa-miR-570-5p | 0.0 | 0.0 | 0.0 | 0.0 | 0.0 | 0.0 | 0.0 | 0.0 | 0.0 |
| hsa -miR -0548al | 0.0 | 0.0 | 2.4 | 0.0 | 0.0 | 0.0 | 0.0 | 0.0 | 0.0 |
| hsa-miR-0548am-3p | 0.0 | 0.0 | 0.0 | 0.0 | 0.0 | 0.0 | 0.0 | 0.0 | 0.0 |
| hsa -miR -0548an | 0.0 | 0.0 | 0.0 | 0.0 | 0.0 | 0.0 | 0.0 | 0.0 | 0.0 |
| hsa-miR-0548ao-3p | 0.0 | 0.0 | 0.0 | 0.0 | 0.0 | 0.0 | 0.0 | 0.0 | 0.0 |
| hsa-miR-0548ao-5p | 0.0 | 0.0 | 0.0 | 0.0 | 0.0 | 0.0 | 0.0 | 0.0 | 0.0 |
| hsa -miR -0548ap-3p | 0.0 | 0.0 | 0.0 | 0.0 | 0.0 | 0.0 | 0.0 | 0.0 | 0.0 |
| hsa-miR-0548aq-5p | 0.0 | 0.0 | 0.0 | 0.0 | 0.0 | 0.0 | 0.0 | 0.0 | 0.0 |
| hsa -miR -0548as-3p | 0.0 | 0.0 | 0.0 | 0.0 | 0.0 | 0.0 | 0.0 | 0.0 | 0.0 |
| hsa -miR -0548as-5p | 0.0 | 0.0 | 2.6 | 0.0 | 0.0 | 1.8 | 0.0 | 0.0 | 0.0 |
| hsa -miR -0548at-5p | 0.0 | 2.8 | 1.9 | 0.0 | 0.0 | 0.0 | 0.0 | 0.0 | 0.0 |
| hsa-miR-0548au-3p | 0.0 | 1.8 | 1.2 | 0.0 | 0.0 | 0.0 | 0.0 | 0.6 | 0.0 |
| hsa -miR -0548av-3p | 0.0 | 0.0 | 0.0 | 0.0 | 0.0 | 0.0 | 0.0 | 0.0 | 0.0 |
| hsa -miR -0548ay-5p | 0.0 | 1.5 | 0.0 | 0.0 | 0.0 | 0.0 | 0.0 | 0.0 | 0.0 |
| hsa -miR -0548az-3p | 0.0 | 0.0 | 0.0 | 0.0 | 0.0 | 0.0 | 0.0 | 0.0 | 0.0 |
| hsa-miR-0548b-3p | 1.0 | 0.0 | 2.0 | 0.0 | 1.2 | 0.0 | 0.0 | 0.0 | 0.0 |
| hsa -miR -0548c-3p | 0.0 | 0.0 | 0.0 | 0.0 | 0.0 | 0.0 | 0.0 | 0.0 | 0.0 |
| hsa-miR-0548c-5p, hsa-miR-548o-5p, hsa-miR-548am-5p | 0.0 | 0.0 | 0.0 | 0.0 | 0.0 | 0.0 | 0.0 | 0.0 | 0.0 |
| hsa-miR-0548e-3p | 0.0 | 0.0 | 0.0 | 0.0 | 0.0 | 0.0 | 0.0 | 0.0 | 0.0 |
| hsa-miR-0548e-5p | 0.0 | 0.0 | 0.0 | 0.0 | 0.0 | 0.0 | 0.0 | 0.0 | 0.0 |
| hsa-miR-0548f-3p | 0.0 | 0.0 | 0.0 | 0.0 | 0.0 | 0.0 | 0.0 | 0.0 | 0.0 |
| hsa-miR-0548h-5p | 1.9 | 0.0 | 0.0 | 0.0 | 0.0 | 0.0 | 0.0 | 0.0 | 0.0 |
| hsa-miR-0548j-5p | 0.0 | 0.0 | 0.0 | 0.0 | 0.0 | 0.0 | 0.0 | 0.0 | 0.0 |
| hsa -miR -0548k | 0.0 | 0.0 | 0.0 | 0.0 | 0.0 | 0.0 | 0.0 | 0.0 | 0.0 |
| hsa -miR -05481 | 0.0 | 1.8 | 2.1 | 0.0 | 0.0 | 0.0 | 0.0 | 0.0 | 0.0 |
| hsa -miR -0548m | 0.0 | 0.0 | 0.0 | 0.0 | 0.0 | 0.0 | 0.0 | 0.0 | 0.0 |
| hsa -miR -0548p | 0.0 | 0.0 | 0.0 | 0.0 | 0.0 | 0.0 | 0.0 | 0.0 | 0.0 |
| hsa -miR -0548s | 3.1 | 0.0 | 0.0 | 0.0 | 0.0 | 0.0 | 0.0 | 0.0 | 0.0 |
| hsa -miR -0548t-5p | 0.0 | 0.0 | 0.0 | 0.0 | 0.0 | 0.0 | 0.0 | 0.0 | 0.0 |
| hsa -miR -0548w | 0.0 | 2.2 | 0.0 | 0.0 | 0.0 | 0.0 | 0.0 | 0.0 | 0.0 |
| hsa-miR-0551b-3p | 0.0 | 3.3 | 2.6 | 3.0 | 0.6 | 3.7 | 1.6 | 2.4 | 1.9 |
| hsa-miR-0551b-5p | 1.9 | 2.9 | 2.9 | 2.3 | 2.0 | 1.8 | 3.8 | 8.7 | 7.3 |
| hsa -miR -0552-3p | 0.0 | 1.2 | 2.6 | 0.0 | 0.0 | 0.0 | 0.0 | 0.7 | 1.9 |
| hsa-miR-0554 | 0.0 | 2.8 | 2.3 | 0.0 | 2.1 | 1.7 | 0.0 | 0.0 | 1.8 |
| hsa-miR-0555 | 1.2 | 0.0 | 3.4 | 2.8 | 0.0 | 0.0 | 0.0 | 1.3 | 0.0 |
| hsa -miR -0556-5p | 0.0 | 0.0 | 0.0 | 0.0 | 0.0 | 0.0 | 0.0 | 0.0 | 0.0 |
| hsa-miR-0559 | 0.0 | 0.0 | 0.0 | 0.0 | 0.0 | 0.0 | 0.0 | 0.0 | 0.0 |
| hsa-miR-0563 | 0.0 | 1.8 | 2.5 | 1.9 | 0.0 | 0.0 | 1.6 | 2.0 | 0.0 |
| hsa-miR-0567 | 0.0 | 0.0 | 0.0 | 0.0 | 0.0 | 0.0 | 0.0 | 0.0 | 0.0 |
| hsa-miR-0570-3p | 0.0 | 0.0 | 0.0 | 0.0 | 0.0 | 0.0 | 0.0 | 0.0 | 0.0 |
| hsa-miR-0571 | 3.0 | 0.0 | 2.4 | 0.0 | 2.7 | 0.0 | 0.0 | 0.0 | 1.3 |
| hsa-miR-0573 | 0.0 | 0.0 | 1.3 | 0.0 | 0.0 | 0.0 | 0.0 | 0.0 | 0.0 |
| hsa -miR -0582-3p | 2.7 | 0.0 | 0.0 | 0.0 | 0.0 | 0.0 | 0.0 | 0.0 | 0.0 |
| hsa -miR -0582-5p | 0.0 | 0.0 | 0.0 | 0.0 | 0.0 | 0.0 | 0.0 | 0.0 | 0.0 |
| hsa-miR-0586 | 0.0 | 0.0 | 0.0 | 0.0 | 2.6 | 0.0 | 0.0 | 0.0 | 0.0 |
| hsa-miR-0587 | 0.0 | 0.0 | 0.0 | 0.0 | 0.8 | 0.0 | 0.0 | 0.0 | 0.0 |
| hsa-miR-0588 | 0.0 | 1.8 | 2.5 | 0.0 | 0.0 | 0.0 | 0.0 | 0.0 | 0.0 |
| hsa-miR-0592 | 0.0 | 0.0 | 0.0 | 1.8 | 0.0 | 0.0 | 0.0 | 0.0 | 0.0 |
| hsa-miR-0597-3p | 0.0 | 0.0 | 0.0 | 0.0 | 0.0 | 0.0 | 0.0 | 0.0 | 0.0 |
| hsa-miR-0597-5p | 0.0 | 0.0 | 0.0 | 0.0 | 0.0 | 0.0 | 0.0 | 0.0 | 0.0 |
| hsa -miR -0598-3p | 2.3 | 1.2 | 1.5 | 1.7 | 0.0 | 1.1 | 0.0 | 0.0 | 1.3 |
| hsa-miR-0599 | 0.0 | 0.0 | 0.0 | 0.0 | 0.0 | 0.0 | 0.0 | 0.0 | 0.0 |
| hsa-miR-0600 | 0.0 | 0.0 | 0.0 | 0.0 | 0.0 | 0.0 | 0.0 | 0.0 | 0.0 |
| hsa-miR-0601 | 2.3 | 2.2 | 2.9 | 3.1 | 3.3 | 2.5 | 2.4 | 2.7 | 4.1 |
| hsa-miR-0616-3p | 0.0 | 0.0 | 0.0 | 0.0 | 2.0 | 1.1 | 1.4 | 1.2 | 0.0 |
| hsa-miR-0616-5p | 2.1 | 1.1 | 0.0 | 0.0 | 1.8 | 0.0 | 1.7 | 0.0 | 0.0 |
| hsa-miR-0619-3p | 0.0 | 1.0 | 0.0 | 0.0 | 0.9 | 0.0 | 0.0 | 0.0 | 0.0 |
| hsa-miR-0623 | 3.0 | 3.0 | 3.4 | 3.2 | 0.6 | 1.8 | 0.0 | 0.0 | 0.0 |
| hsa -miR -0624-3p | 0.0 | 0.0 | 0.0 | 0.0 | 2.9 | 2.8 | 0.0 | 0.0 | 0.0 |
| hsa-miR-0627-3p | 0.0 | 0.0 | 0.0 | 0.0 | 0.0 | 0.0 | 0.0 | 0.0 | 0.0 |
| hsa-miR-0629-5p | 0.0 | 0.0 | 0.0 | 2.5 | 2.3 | 0.0 | 2.6 | 2.1 | 0.0 |
| hsa-miR-0631 | 3.3 | 3.5 | 3.9 | 2.9 | 3.0 | 3.1 | 1.8 | 1.6 | 1.3 |
| hsa-miR-0633 | 0.0 | 0.0 | 1.7 | 0.0 | 0.0 | 0.0 | 0.0 | 0.0 | 0.0 |
| hsa-miR-0639 | 1.9 | 0.0 | 0.0 | 0.0 | 1.0 | 0.0 | 0.0 | 0.0 | 0.0 |
| hsa-miR-0640 | 1.8 | 1.3 | 0.0 | 0.0 | 0.0 | 0.0 | 0.0 | 0.0 | 0.0 |
| hsa-miR-0641 | 0.0 | 0.0 | 1.6 | 0.0 | 0.0 | 0.0 | 0.0 | 0.0 | 0.0 |
| hsa-miR-0643 | 0.0 | 1.8 | 2.3 | 0.0 | 0.6 | 0.0 | 0.0 | 0.0 | 0.0 |
| hsa -miR -0644a | 2.9 | 2.1 | 2.1 | 2.8 | 0.6 | 1.2 | 2.0 | 0.0 | 0.0 |
| hsa-miR-0646 | 0.0 | 0.0 | 0.0 | 0.0 | 3.2 | 0.0 | 0.0 | 0.0 | 0.0 |
| hsa-miR-0647 | 2.5 | 0.0 | 1.1 | 0.0 | 0.0 | 0.0 | 0.0 | 0.0 | 0.0 |
| hsa-miR-0653-3p | 0.0 | 0.0 | 0.0 | 0.0 | 0.0 | 0.0 | 0.0 | 0.0 | 0.0 |
| hsa-miR-0653-5p | 0.0 | 1.3 | 1.7 | 0.0 | 0.9 | 0.0 | 2.1 | 0.0 | 0.0 |
| hsa-miR-0655-3p | 0.0 | 0.0 | 0.0 | 0.0 | 1.4 | 0.0 | 0.0 | 0.0 | 0.0 |
| hsa-miR-0655-5p | 0.0 | 0.0 | 0.0 | 0.0 | 0.0 | 1.2 | 0.0 | 0.0 | 0.0 |
| hsa -miR -0656-3p | 0.0 | 1.5 | 1.3 | 0.0 | 0.0 | 0.0 | 0.0 | 0.0 | 1.8 |
| hsa-miR-0659-5p | 2.8 | 3.1 | 3.9 | 2.0 | 0.9 | 1.5 | 1.7 | 0.0 | 0.0 |
| hsa-miR-0660-5p | 1.5 | 0.0 | 1.8 | 0.0 | 0.0 | 0.0 | 0.0 | 0.0 | 0.0 |
| hsa-miR-0758-3p | 0.0 | 0.0 | 2.0 | 0.0 | 0.0 | 0.0 | 0.0 | 0.0 | 0.0 |
| hsa-miR-0759 | 0.0 | 0.0 | 0.0 | 0.0 | 0.0 | 0.0 | 0.0 | 0.0 | 0.0 |
| hsa-miR-0761 | 0.0 | 2.9 | 3.3 | 2.1 | 0.0 | 2.5 | 2.7 | 2.8 | 1.2 |
| hsa-miR-0769-5p | 1.2 | 2.0 | 2.9 | 2.9 | 1.7 | 2.3 | 2.5 | 1.8 | 2.8 |
| hsa-miR-0802 | 0.0 | 0.0 | 0.0 | 0.0 | 0.0 | 0.0 | 0.0 | 0.0 | 0.0 |
| hsa-miR-0875-5p | 0.0 | 0.0 | 0.0 | 0.0 | 0.0 | 0.0 | 0.0 | 0.0 | 0.0 |
| hsa-miR-0876-3p | 0.0 | 0.0 | 0.0 | 0.0 | 1.7 | 0.0 | 0.0 | 0.0 | 0.0 |
| hsa-miR-0890 | 0.0 | 0.0 | 0.0 | 0.0 | 0.0 | 0.0 | 0.0 | 1.7 | 0.0 |
| hsa -miR -0892c-3p | 1.9 | 2.5 | 3.5 | 2.2 | 0.0 | 2.7 | 1.9 | 1.0 | 0.0 |
| hsa-miR-0924 | 1.9 | 1.7 | 2.4 | 2.3 | 0.5 | 0.0 | 1.9 | 0.0 | 0.0 |
| hsa-miR-0934 | 0.0 | 0.0 | 0.0 | 0.0 | 0.0 | 0.0 | 0.0 | 0.0 | 0.0 |
| hsa-miR-0941 | 0.0 | 0.0 | 0.0 | 0.0 | 1.1 | 0.0 | 0.0 | 0.0 | 0.0 |
| hsa-miR-0944 | 0.0 | 0.0 | 0.0 | 0.0 | 2.6 | 0.0 | 0.0 | 0.0 | 2.1 |
| hsa-miR-1178-5p | 0.0 | 0.0 | 2.1 | 0.0 | 1.3 | 0.0 | 0.0 | 0.0 | 0.0 |
| hsa-miR-1179 | 0.0 | 0.0 | 0.0 | 0.0 | 0.0 | 0.0 | 0.0 | 0.0 | 0.0 |
| hsa-miR-1180-3p | 0.0 | 0.0 | 0.0 | 0.0 | 0.0 | 0.0 | 0.0 | 0.0 | 0.0 |
| hsa-miR-1180-5p | 2.8 | 1.9 | 0.0 | 0.0 | 0.0 | 0.0 | 0.0 | 0.2 | 1.3 |
| hsa-miR-1183 | 0.0 | 0.0 | 2.1 | 0.0 | 0.0 | 0.0 | 0.0 | 3.2 | 0.0 |
| hsa-miR-1185-5p | 0.0 | 0.0 | 1.9 | 0.0 | 0.0 | 0.0 | 0.0 | 0.0 | 0.0 |
| hsa-miR-1208 | 0.0 | 0.0 | 1.2 | 0.0 | 0.0 | 0.0 | 0.0 | 0.0 | 0.0 |
| hsa-miR-1226-5p | 3.7 | 3.5 | 2.4 | 2.6 | 0.0 | 2.6 | 0.0 | 3.9 | 2.7 |
| hsa-miR-1245b-3p | 0.0 | 0.0 | 0.0 | 0.0 | 0.0 | 0.0 | 0.0 | 0.0 | 0.0 |
| hsa-miR-1245b-5p | 0.0 | 0.0 | 1.4 | 0.0 | 0.0 | 0.0 | 0.0 | 0.0 | 0.0 |
| hsa-miR-1248 | 0.0 | 0.0 | 0.0 | 0.0 | 0.0 | 0.0 | 0.0 | 0.0 | 0.0 |
| hsa-miR-1251-5p | 0.0 | 0.0 | 0.0 | 0.0 | 0.0 | 0.0 | 0.0 | 0.0 | 0.0 |
| hsa-miR-1252-3p | 0.0 | 0.0 | 0.0 | 0.0 | 0.0 | 0.0 | 0.0 | 0.0 | 0.0 |
| hsa-miR-1253 | 0.0 | 0.0 | 0.0 | 0.0 | 0.4 | 0.0 | 0.0 | 0.0 | 0.0 |
| hsa-miR-1255b-5p | 1.7 | 1.8 | 2.3 | 0.0 | 0.0 | 0.0 | 0.0 | 0.0 | 0.0 |
| hsa-miR-1256 | 0.0 | 0.0 | 0.0 | 0.0 | 0.0 | 0.0 | 0.0 | 0.0 | 0.0 |
| hsa-miR-1257 | 0.0 | 0.0 | 0.0 | 0.0 | 0.0 | 0.0 | 0.0 | 0.0 | 2.3 |
| hsa-miR-1263 | 1.6 | 1.2 | 1.4 | 1.6 | 0.0 | 0.0 | 0.0 | 0.0 | 0.0 |
| hsa-miR-1264 | 1.4 | 0.0 | 1.5 | 0.0 | 0.0 | 0.0 | 0.0 | 0.0 | 0.0 |
| hsa-miR-1265 | 2.8 | 2.2 | 2.6 | 3.0 | 0.8 | 1.0 | 3.0 | 2.9 | 2.9 |
| hsa-miR-1269a | 0.0 | 0.0 | 0.0 | 0.0 | 0.0 | 1.0 | 0.0 | 0.0 | 0.0 |
| hsa-miR-1270 | 1.7 | 0.0 | 0.0 | 1.5 | 0.0 | 1.2 | 2.1 | 0.0 | 1.4 |
| hsa-miR-1272 | 0.0 | 0.0 | 0.0 | 0.0 | 0.0 | 0.0 | 0.0 | 0.0 | 0.0 |
| hsa-miR-1273a | 0.0 | 0.0 | 0.0 | 0.0 | 0.0 | 0.0 | 0.0 | 0.0 | 2.7 |
| hsa-miR-1273c | 2.8 | 1.5 | 2.6 | 0.0 | 0.0 | 0.0 | 0.0 | 0.0 | 1.3 |
| hsa-miR-1273d | 1.6 | 0.0 | 2.0 | 0.0 | 2.5 | 2.2 | 0.0 | 3.0 | 2.2 |
| hsa-miR-1277-5p | 0.0 | 0.0 | 0.0 | 0.0 | 0.0 | 0.0 | 0.0 | 0.0 | 0.0 |
| hsa-miR-1279 | 0.0 | 0.0 | 0.0 | 0.0 | 0.0 | 0.0 | 0.0 | 0.0 | 0.0 |
| hsa-miR-1282 | 1.8 | 1.3 | 0.0 | 0.0 | 0.0 | 0.0 | 0.0 | 0.0 | 0.0 |
| hsa-miR-1283 | 0.0 | 0.0 | 0.0 | 0.0 | 0.0 | 0.0 | 0.0 | 0.0 | 0.0 |
| hsa-miR-1285-3p | 2.1 | 0.0 | 1.9 | 2.8 | 3.2 | 3.1 | 1.6 | 2.7 | 3.9 |
| hsa-miR-1289 | 0.0 | 0.0 | 1.8 | 0.0 | 0.0 | 0.0 | 1.9 | 0.7 | 2.3 |
| hsa-miR-1290 | 0.0 | 1.8 | 0.0 | 0.0 | 0.0 | 0.0 | 0.0 | 0.0 | 0.0 |
| hsa-miR-1292-5p | 1.7 | 1.8 | 1.8 | 0.0 | 0.0 | 1.9 | 0.0 | 1.6 | 0.0 |
| hsa-miR-1294 | 0.0 | 0.0 | 2.0 | 2.1 | 0.0 | 0.0 | 0.0 | 0.0 | 0.0 |
| hsa-miR-1295a | 2.6 | 0.0 | 0.0 | 0.0 | 0.0 | 0.0 | 0.0 | 0.0 | 0.0 |
| hsa-miR-1295b-5p | 0.0 | 0.0 | 1.7 | 0.0 | 0.0 | 0.0 | 0.0 | 0.0 | 0.0 |
| hsa-miR-1302 | 0.0 | 0.0 | 0.0 | 0.0 | 0.0 | 0.0 | 0.0 | 0.0 | 0.0 |
| hsa-miR-1304-5p | 0.0 | 1.1 | 0.0 | 1.5 | 0.0 | 0.0 | 0.0 | 0.0 | 0.0 |
| hsa-miR-1305 | 0.0 | 0.0 | 0.0 | 0.0 | 0.0 | 0.0 | 0.0 | 0.0 | 0.0 |
| hsa-miR-1307-5p | 3.0 | 1.0 | 3.1 | 0.0 | 1.6 | 0.0 | 0.0 | 0.0 | 0.0 |
| hsa-miR-1321 | 1.7 | 1.7 | 2.8 | 3.2 | 0.0 | 2.4 | 0.0 | 3.3 | 1.4 |
| hsa-miR-1468-3p | 0.0 | 0.0 | 0.0 | 0.0 | 0.0 | 0.0 | 0.0 | 0.0 | 0.0 |
| hsa-miR-1537-3p | 0.0 | 0.0 | 1.3 | 0.0 | 0.0 | 0.0 | 0.0 | 0.0 | 0.0 |
| hsa-miR-1827 | 0.0 | 0.0 | 0.0 | 0.0 | 0.0 | 0.0 | 0.0 | 0.0 | 0.0 |
| hsa-miR-1911-5p | 1.3 | 2.1 | 1.9 | 0.0 | 0.0 | 0.0 | 0.0 | 1.9 | 1.9 |
| hsa-miR-1973 | 2.6 | 0.0 | 0.0 | 0.0 | 0.0 | 0.0 | 0.0 | 0.0 | 0.0 |
| hsa-miR-2052 | 0.0 | 0.0 | 0.0 | 0.0 | 0.0 | 0.0 | 0.0 | 0.0 | 0.0 |
| hsa-miR-2053 | 0.0 | 0.0 | 1.4 | 0.0 | 0.0 | 0.0 | 0.0 | 0.0 | 0.0 |
| hsa-miR-2054 | 0.0 | 0.0 | 0.0 | 0.0 | 0.0 | 0.0 | 0.0 | 0.0 | 0.0 |
| hsa-miR-2114-3p | 0.0 | 0.0 | 0.0 | 0.0 | 0.0 | 0.0 | 0.0 | 0.0 | 0.0 |
| hsa-miR-2114-5p | 0.0 | 0.0 | 2.6 | 0.0 | 1.1 | 0.0 | 0.0 | 0.0 | 0.0 |
| hsa-miR-2115-3p | 0.0 | 0.0 | 0.0 | 0.0 | 0.0 | 0.0 | 0.0 | 0.0 | 0.0 |
| hsa-miR-2115-5p | 0.0 | 0.0 | 1.4 | 0.0 | 0.0 | 0.0 | 0.0 | 0.0 | 0.0 |
| hsa-miR-2681-3p | 0.0 | 0.0 | 2.4 | 0.0 | 0.0 | 1.5 | 0.0 | 0.0 | 0.0 |
| hsa-miR-3065-5p | 2.6 | 3.6 | 2.1 | 1.8 | 1.0 | 2.0 | 0.0 | 0.0 | 0.0 |
| hsa-miR-3119 | 0.0 | 0.0 | 2.0 | 2.4 | 0.0 | 0.0 | 0.0 | 1.3 | 2.4 |
| hsa-miR-3120-3p | 1.0 | 2.6 | 2.9 | 2.6 | 0.0 | 1.7 | 2.9 | 1.7 | 1.9 |
| hsa-miR-3122 | 5.1 | 4.5 | 4.0 | 4.7 | 4.7 | 5.3 | 4.7 | 6.0 | 5.7 |
| hsa-miR-3123 | 0.0 | 0.0 | 1.9 | 0.0 | 0.0 | 0.0 | 0.0 | 0.0 | 0.0 |
| hsa-miR-3125 | 2.5 | 3.3 | 3.7 | 3.7 | 3.3 | 3.6 | 4.1 | 3.3 | 3.9 |
| hsa-miR-3127-3p | 5.6 | 5.6 | 6.0 | 5.4 | 5.1 | 5.0 | 6.3 | 5.8 | 6.1 |
| hsa-miR-3130-3p | 1.4 | 3.0 | 3.0 | 3.6 | 2.5 | 2.7 | 1.7 | 1.4 | 1.8 |
| hsa-miR-3130-5p | 5.3 | 5.0 | 5.4 | 5.1 | 4.7 | 4.9 | 4.6 | 5.0 | 4.8 |
| hsa-miR-3135a | 0.0 | 0.0 | 0.0 | 0.0 | 0.0 | 0.0 | 0.0 | 0.0 | 0.0 |
| hsa-miR-3140-5p | 0.0 | 0.0 | 0.0 | 0.0 | 0.0 | 0.0 | 0.0 | 0.0 | 0.0 |
| hsa-miR-3148 | 0.0 | 2.6 | 3.0 | 1.8 | 0.0 | 2.4 | 2.8 | 2.3 | 1.9 |
| hsa-miR-3149 | 3.5 | 3.9 | 4.1 | 4.0 | 3.2 | 2.7 | 3.8 | 3.1 | 3.6 |
| hsa-miR-3150a-3p | 5.9 | 5.7 | 5.6 | 5.4 | 5.6 | 5.0 | 5.5 | 5.7 | 5.7 |
| hsa-miR-3150b-5p | 5.6 | 5.5 | 5.9 | 5.3 | 5.2 | 4.9 | 6.0 | 5.6 | 5.8 |
| hsa-miR-3151-3p | 6.3 | 6.3 | 6.5 | 6.1 | 6.0 | 5.9 | 5.6 | 5.6 | 5.5 |
| hsa-miR-3151-5p | 6.0 | 6.1 | 6.2 | 6.5 | 5.9 | 6.1 | 6.9 | 7.7 | 7.3 |
| hsa-miR-3160-5p | 4.4 | 4.2 | 4.5 | 4.1 | 3.5 | 3.8 | 4.6 | 3.7 | 3.9 |
| hsa-miR-3181 | 0.0 | 2.7 | 0.0 | 0.0 | 3.0 | 0.0 | 0.0 | 0.0 | 0.0 |
| hsa-miR-3182 | 0.0 | 0.0 | 0.0 | 0.0 | 0.0 | 0.0 | 0.0 | 0.0 | 0.0 |
| hsa-miR-3183 | 0.0 | 1.1 | 0.0 | 0.0 | 0.0 | 0.0 | 0.0 | 0.0 | 0.0 |
| hsa-miR-3192-5p | 3.1 | 1.9 | 2.7 | 3.4 | 2.7 | 2.6 | 2.3 | 2.7 | 3.2 |
| hsa-miR-3194-3p | 0.0 | 0.0 | 2.2 | 0.0 | 0.0 | 0.0 | 0.0 | 0.0 | 0.0 |
| hsa-miR-3199 | 0.0 | 2.7 | 2.5 | 2.6 | 0.0 | 2.2 | 2.2 | 4.4 | 3.2 |
| hsa-miR-3591-5p | 0.0 | 1.4 | 1.8 | 1.9 | 0.5 | 0.0 | 2.0 | 0.3 | 0.0 |
| hsa -miR -3606-3p | 0.0 | 0.0 | 0.0 | 0.0 | 1.2 | 0.0 | 0.0 | 0.0 | 0.0 |
| hsa-miR-3607-3p | 0.0 | 0.0 | 0.0 | 0.0 | 0.0 | 0.0 | 0.0 | 0.0 | 0.0 |
| hsa-miR-3611 | 0.0 | 0.0 | 0.0 | 0.0 | 0.0 | 0.0 | 0.0 | 0.0 | 0.0 |
| hsa-miR-3613-3p | 2.9 | 3.0 | 2.4 | 1.6 | 0.0 | 0.0 | 0.0 | 0.0 | 2.2 |
| hsa-miR-3614-3p | 0.0 | 2.1 | 0.0 | 0.0 | 0.0 | 1.0 | 0.0 | 0.0 | 1.1 |
| hsa-miR-3617-5p | 0.0 | 0.0 | 0.0 | 0.0 | 0.0 | 0.0 | 0.0 | 0.0 | 0.0 |
| hsa-miR-3653-3p | 0.0 | 1.3 | 2.5 | 1.9 | 0.7 | 3.0 | 2.6 | 0.0 | 2.8 |
| hsa-miR-3660 | 1.8 | 2.3 | 2.8 | 2.2 | 2.6 | 2.6 | 2.7 | 3.0 | 1.8 |
| hsa-miR-3661 | 2.5 | 2.3 | 3.1 | 2.5 | 2.4 | 2.7 | 2.9 | 3.9 | 2.4 |
| hsa -miR -3664-5p | 0.0 | 0.0 | 2.3 | 0.0 | 0.0 | 0.0 | 0.0 | 0.0 | 0.0 |
| hsa-miR-3670 | 1.7 | 2.2 | 1.3 | 0.0 | 0.0 | 0.0 | 0.0 | 0.0 | 0.0 |
| hsa-miR-3671 | 0.0 | 0.0 | 0.0 | 0.0 | 0.0 | 0.0 | 0.0 | 0.0 | 0.0 |
| hsa-miR-3672 | 0.0 | 0.0 | 0.0 | 0.0 | 0.0 | 0.0 | 0.0 | 0.0 | 0.0 |
| hsa-miR-3674 | 0.0 | 0.0 | 0.0 | 0.0 | 0.0 | 0.0 | 0.0 | 0.0 | 0.0 |
| hsa-miR-3675-5p | 3.0 | 0.0 | 1.9 | 2.9 | 0.0 | 1.6 | 0.0 | 1.4 | 1.6 |
| hsa-miR-3680-5p | 3.0 | 2.8 | 0.0 | 2.4 | 0.0 | 2.2 | 2.6 | 1.8 | 2.2 |
| hsa-miR-3681-5p | 0.0 | 0.0 | 0.0 | 0.0 | 0.0 | 0.0 | 0.0 | 0.0 | 0.0 |
| hsa-miR-3683 | 0.0 | 0.0 | 0.0 | 0.0 | 0.0 | 0.0 | 0.0 | 0.0 | 0.0 |
| hsa-miR-3689a-5p, hsa-miR-3689b-5p, hsa-miR-3689e | 0.0 | 1.5 | 0.0 | 0.0 | 0.0 | 0.0 | 0.0 | 0.0 | 0.0 |
| hsa-miR-3907 | 2.3 | 0.0 | 0.0 | 0.0 | 0.0 | 0.0 | 0.0 | 0.7 | 0.0 |
| hsa-miR-3909 | 2.5 | 3.0 | 2.6 | 0.0 | 0.0 | 0.0 | 0.0 | 0.0 | 1.4 |
| hsa-miR-3910 | 3.2 | 2.2 | 2.4 | 2.2 | 0.0 | 0.0 | 1.6 | 0.0 | 0.0 |
| hsa-miR-3912-5p | 0.0 | 1.2 | 1.4 | 0.0 | 0.0 | 0.0 | 0.0 | 0.0 | 0.0 |
| hsa-miR-3913-5p | 0.0 | 2.6 | 3.9 | 2.9 | 0.0 | 2.1 | 2.4 | 0.0 | 1.1 |
| hsa-miR-3915 | 0.0 | 0.0 | 0.0 | 1.6 | 0.0 | 0.0 | 0.0 | 0.8 | 0.0 |
| hsa-miR-3919 | 1.1 | 2.1 | 2.7 | 2.7 | 0.0 | 2.1 | 0.0 | 0.0 | 1.9 |
| hsa-miR-3920 | 0.0 | 0.0 | 2.1 | 0.0 | 0.7 | 0.0 | 0.0 | 0.0 | 0.0 |
| hsa-miR-3924 | 0.0 | 0.0 | 0.0 | 0.0 | 0.0 | 0.0 | 0.0 | 0.0 | 0.0 |
| hsa-miR-3925-3p | 1.8 | 0.0 | 0.0 | 1.6 | 0.0 | 0.0 | 0.0 | 0.5 | 0.0 |
| hsa-miR-3926 | 1.2 | 0.0 | 1.5 | 2.0 | 0.0 | 0.0 | 0.0 | 0.7 | 2.0 |
| hsa-miR-3938 | 0.0 | 0.0 | 0.0 | 0.0 | 0.0 | 0.0 | 0.0 | 0.0 | 0.0 |
| hsa-miR-3939 | 1.7 | 0.0 | 0.0 | 0.0 | 0.0 | 0.0 | 0.0 | 0.0 | 0.0 |
| hsa-miR-3941 | 0.0 | 0.0 | 0.0 | 0.0 | 0.0 | 0.0 | 0.0 | 0.0 | 2.3 |
| hsa -miR -3942-3p | 0.0 | 0.0 | 1.2 | 0.0 | 0.0 | 0.0 | 0.0 | 0.0 | 0.0 |
| hsa -miR -3942-5p | 2.1 | 0.0 | 0.0 | 0.0 | 0.0 | 0.0 | 0.0 | 0.0 | 0.0 |
| hsa-miR-3974 | 0.0 | 0.0 | 0.0 | 0.0 | 0.0 | 0.0 | 0.0 | 0.0 | 0.0 |
| hsa-miR-3977 | 0.0 | 2.2 | 3.6 | 0.0 | 2.7 | 2.0 | 1.5 | 0.5 | 0.0 |
| hsa-miR-3978 | 0.0 | 1.2 | 2.7 | 0.0 | 0.0 | 0.0 | 1.6 | 3.7 | 1.7 |
| hsa-miR-4263 | 0.0 | 0.0 | 1.1 | 0.0 | 0.0 | 0.0 | 0.0 | 0.0 | 0.0 |
| hsa-miR-4267 | 3.8 | 3.6 | 3.4 | 3.3 | 3.1 | 2.6 | 2.9 | 3.3 | 3.2 |
| hsa-miR-4285 | 0.0 | 1.5 | 0.0 | 0.0 | 0.0 | 1.4 | 0.0 | 0.6 | 0.0 |
| hsa-miR-4289 | 2.2 | 2.2 | 2.7 | 2.3 | 2.4 | 0.0 | 0.0 | 0.0 | 2.1 |
| hsa-miR-4293 | 0.0 | 1.6 | 1.9 | 0.0 | 0.0 | 1.4 | 0.0 | 0.0 | 0.0 |
| hsa-miR-4309 | 1.5 | 0.0 | 0.0 | 0.0 | 0.0 | 0.0 | 0.0 | 0.0 | 0.0 |
| hsa-miR-4311 | 2.2 | 2.0 | 2.3 | 3.1 | 3.4 | 3.1 | 2.2 | 3.0 | 2.6 |
| hsa-miR-4315 | 0.0 | 0.0 | 1.9 | 1.5 | 0.0 | 0.0 | 0.0 | 0.1 | 0.0 |
| hsa-miR-4321 | 2.5 | 3.0 | 1.1 | 2.6 | 2.8 | 2.9 | 2.0 | 4.2 | 2.7 |
| hsa-miR-4418 | 0.0 | 0.0 | 1.4 | 0.0 | 0.0 | 0.0 | 0.0 | 0.0 | 0.0 |
| hsa-miR-4422 | 0.0 | 0.0 | 0.0 | 0.0 | 0.0 | 0.0 | 0.0 | 0.0 | 0.0 |
| hsa-miR-4423-3p | 1.3 | 1.5 | 2.0 | 2.0 | 2.6 | 0.0 | 2.3 | 0.1 | 1.6 |
| hsa-miR-4424 | 0.0 | 0.0 | 0.0 | 0.0 | 0.0 | 0.0 | 0.0 | 0.0 | 0.0 |
| hsa-miR-4427 | 0.0 | 0.0 | 0.0 | 0.0 | 0.0 | 0.0 | 0.0 | 0.0 | 0.0 |
| hsa-miR-4431 | 2.9 | 0.0 | 1.3 | 2.9 | 2.1 | 0.0 | 2.5 | 2.9 | 3.4 |
| hsa-miR-4432 | 0.0 | 0.0 | 0.0 | 0.0 | 0.0 | 0.0 | 0.0 | 0.0 | 0.0 |
| hsa-miR-4435 | 3.7 | 2.2 | 2.8 | 4.8 | 3.8 | 5.6 | 3.8 | 4.8 | 5.8 |
| hsa-miR-4439 | 1.8 | 3.4 | 3.0 | 2.9 | 2.0 | 3.3 | 3.4 | 1.5 | 2.8 |
| hsa-miR-4445-5p | 0.0 | 0.0 | 0.0 | 0.0 | 0.0 | 0.0 | 0.0 | 0.0 | 0.0 |
| hsa-miR-4452 | 0.0 | 0.0 | 0.0 | 0.0 | 0.0 | 0.0 | 0.0 | 0.0 | 0.0 |
| hsa-miR-4457 | 0.0 | 0.0 | 0.0 | 0.0 | 0.0 | 0.0 | 0.0 | 0.0 | 0.0 |
| hsa-miR-4458 | 2.8 | 2.0 | 3.8 | 2.5 | 1.3 | 2.6 | 2.1 | 3.6 | 2.1 |
| hsa-miR-4461 | 0.0 | 0.0 | 2.5 | 2.5 | 0.0 | 2.0 | 0.0 | 2.2 | 0.0 |
| hsa-miR-4464 | 0.0 | 0.0 | 1.3 | 0.0 | 0.0 | 0.0 | 0.0 | 0.0 | 0.0 |
| hsa-miR-4468 | 0.0 | 0.0 | 1.3 | 0.0 | 0.0 | 0.0 | 0.0 | 0.0 | 0.0 |
| hsa-miR-4469 | 0.0 | 0.0 | 1.3 | 0.0 | 0.0 | 0.0 | 0.0 | 0.0 | 0.0 |
| hsa-miR-4470 | 1.9 | 0.0 | 1.3 | 0.0 | 0.7 | 1.3 | 2.8 | 6.0 | 4.2 |
| hsa-miR-4471 | 0.0 | 1.8 | 2.7 | 0.0 | 1.6 | 1.4 | 3.0 | 0.0 | 2.0 |
| hsa-miR-4475 | 2.8 | 0.0 | 0.0 | 0.0 | 0.0 | 0.0 | 0.0 | 0.0 | 1.8 |
| hsa-miR-4480 | 0.0 | 0.0 | 0.0 | 0.0 | 0.0 | 0.0 | 0.0 | 0.0 | 0.0 |
| hsa-miR-4485-3p | 0.0 | 0.0 | 0.0 | 0.0 | 0.0 | 0.0 | 0.0 | 3.8 | 0.0 |
| hsa-miR-4490 | 0.0 | 0.0 | 2.3 | 0.0 | 0.0 | 0.0 | 0.0 | 0.0 | 0.0 |
| hsa-miR-4493 | 0.0 | 0.0 | 0.0 | 0.0 | 0.0 | 0.0 | 0.0 | 0.0 | 0.0 |
| hsa-miR-4500 | 0.0 | 0.0 | 0.0 | 0.0 | 0.0 | 0.0 | 0.0 | 0.0 | 0.0 |
| hsa-miR-4503 | 0.0 | 0.0 | 0.0 | 0.0 | 0.0 | 0.0 | 0.0 | 0.0 | 0.0 |
| hsa-miR-4504 | 0.0 | 0.0 | 0.0 | 0.0 | 0.0 | 0.0 | 0.0 | 0.0 | 0.0 |
| hsa-miR-4509 | 0.0 | 0.0 | 0.0 | 0.0 | 0.0 | 0.0 | 0.0 | 0.0 | 0.0 |
| hsa-miR-4510 | 1.8 | 0.0 | 0.0 | 0.0 | 0.0 | 1.4 | 1.8 | 0.2 | 2.1 |
| hsa-miR-4511 | 1.3 | 2.2 | 0.0 | 2.5 | 0.0 | 1.6 | 1.8 | 1.4 | 1.2 |
| hsa-miR-4512 | 0.0 | 1.2 | 3.1 | 0.0 | 0.8 | 0.0 | 0.0 | 0.0 | 0.0 |
| hsa-miR-4515 | 3.7 | 4.2 | 2.5 | 3.8 | 1.4 | 3.9 | 2.2 | 2.5 | 2.4 |
| hsa-miR-4517 | 0.0 | 0.0 | 0.0 | 0.0 | 0.0 | 0.0 | 0.0 | 0.0 | 0.0 |
| hsa-miR-4519 | 0.0 | 0.0 | 1.9 | 0.0 | 0.7 | 1.9 | 1.4 | 1.6 | 0.0 |
| hsa-miR-4520-2-3p | 0.0 | 0.0 | 2.4 | 0.0 | 0.0 | 0.0 | 0.0 | 0.0 | 0.0 |
| hsa-miR-4522 | 0.0 | 1.8 | 2.8 | 0.0 | 1.8 | 1.2 | 0.0 | 1.3 | 0.0 |
| hsa-miR-4524a-3p | 0.0 | 1.3 | 0.0 | 0.0 | 0.0 | 0.0 | 1.6 | 0.0 | 0.0 |
| hsa -miR -4536-3p | 0.0 | 0.0 | 0.0 | 0.0 | 0.0 | 0.0 | 0.0 | 0.0 | 0.0 |
| hsa-miR-4537 | 0.0 | 0.0 | 1.4 | 0.0 | 2.8 | 1.6 | 0.0 | 0.0 | 0.0 |
| hsa-miR-4633-3p | 0.0 | 0.0 | 0.0 | 0.0 | 0.0 | 0.0 | 0.0 | 0.0 | 0.0 |
| hsa-miR-4637 | 0.0 | 0.0 | 0.0 | 0.0 | 0.0 | 0.0 | 0.0 | 0.0 | 0.0 |
| hsa-miR-4643 | 0.0 | 1.8 | 0.0 | 0.0 | 3.6 | 1.1 | 0.0 | 0.0 | 0.0 |
| hsa-miR-4650-3p | 3.1 | 0.0 | 0.0 | 0.0 | 1.8 | 1.2 | 2.2 | 0.0 | 1.9 |
| hsa-miR-4653-3p | 0.0 | 0.0 | 0.0 | 0.0 | 0.0 | 2.0 | 2.0 | 0.0 | 2.1 |
| hsa-miR-4659a-3p | 0.0 | 1.3 | 2.5 | 0.0 | 0.0 | 0.0 | 0.0 | 0.0 | 0.0 |
| hsa-miR-4659a-5p | 2.6 | 0.0 | 1.5 | 0.0 | 0.7 | 1.0 | 2.0 | 0.3 | 0.0 |
| hsa-miR-4659b-5p | 0.0 | 0.0 | 0.0 | 0.0 | 0.0 | 0.0 | 0.0 | 0.0 | 0.0 |
| hsa-miR-4661-3p | 0.0 | 2.3 | 2.4 | 2.8 | 1.6 | 0.0 | 2.2 | 0.0 | 2.0 |
| hsa-miR-4662a-3p | 0.0 | 0.0 | 0.0 | 0.0 | 0.0 | 0.0 | 0.0 | 0.0 | 0.0 |
| hsa-miR-4662a-5p | 0.0 | 0.0 | 0.0 | 0.0 | 0.0 | 0.0 | 0.0 | 0.0 | 0.0 |
| hsa-miR-4666a-3p | 0.0 | 0.0 | 0.0 | 0.0 | 0.0 | 0.0 | 0.0 | 0.0 | 0.0 |
| hsa -miR -4666b | 0.0 | 0.0 | 1.7 | 0.0 | 0.0 | 0.0 | 0.0 | 0.0 | 0.0 |
| hsa-miR-4668-3p | 0.0 | 0.0 | 0.0 | 0.0 | 0.0 | 0.0 | 0.0 | 0.0 | 0.0 |
| hsa-miR-4670-3p | 0.0 | 0.0 | 1.8 | 0.0 | 0.0 | 0.0 | 0.0 | 0.0 | 1.2 |
| hsa-miR-4670-5p | 0.0 | 2.0 | 0.0 | 0.0 | 0.0 | 0.0 | 0.0 | 0.0 | 0.0 |
| hsa-miR-4671-3p | 0.0 | 0.0 | 0.0 | 0.0 | 0.0 | 0.0 | 0.0 | 1.1 | 0.0 |
| hsa-miR-4679 | 0.0 | 0.0 | 0.0 | 0.0 | 0.0 | 0.0 | 0.0 | 0.0 | 0.0 |
| hsa-miR-4683 | 0.0 | 0.0 | 2.1 | 0.0 | 0.0 | 0.0 | 0.0 | 0.0 | 0.0 |
| hsa-miR-4699-3p | 1.4 | 0.0 | 0.0 | 0.0 | 0.0 | 0.0 | 0.0 | 0.0 | 0.0 |
| hsa-miR-4699-5p | 0.0 | 0.0 | 0.0 | 0.0 | 0.0 | 0.0 | 0.0 | 0.0 | 0.0 |
| hsa-miR-4704-5p | 0.0 | 0.0 | 0.0 | 0.0 | 0.0 | 1.3 | 0.0 | 0.0 | 0.0 |
| hsa-miR-4705 | 0.0 | 0.0 | 1.1 | 0.0 | 0.0 | 0.0 | 0.0 | 0.0 | 0.0 |
| hsa-miR-4714-3p | 0.0 | 0.0 | 0.0 | 0.0 | 0.0 | 0.0 | 0.0 | 0.0 | 0.0 |
| hsa-miR-4715-3p | 0.0 | 0.0 | 0.0 | 0.0 | 0.0 | 0.0 | 0.0 | 0.0 | 0.0 |
| hsa-miR-4715-5p | 3.0 | 1.9 | 2.7 | 1.5 | 0.0 | 0.0 | 0.0 | 0.7 | 0.0 |
| hsa-miR-4719 | 1.7 | 1.3 | 2.6 | 0.0 | 0.0 | 0.0 | 0.0 | 0.0 | 0.0 |
| hsa-miR-4720-3p | 0.0 | 0.0 | 0.0 | 0.0 | 0.0 | 0.0 | 0.0 | 0.0 | 0.0 |
| hsa-miR-4724-5p | 1.9 | 1.5 | 3.0 | 2.5 | 0.7 | 2.5 | 0.0 | 0.7 | 1.1 |
| hsa-miR-4727-5p | 0.0 | 0.0 | 0.0 | 0.0 | 0.9 | 0.0 | 0.0 | 0.0 | 0.0 |
| hsa-miR-4735-3p | 0.0 | 0.0 | 0.0 | 0.0 | 0.0 | 0.0 | 0.0 | 0.0 | 0.0 |
| hsa-miR-4737 | 0.0 | 2.7 | 3.3 | 3.2 | 0.8 | 2.5 | 2.7 | 3.5 | 3.3 |
| hsa-miR-4743-3p | 0.0 | 2.0 | 2.7 | 3.2 | 0.0 | 2.3 | 2.1 | 0.9 | 2.7 |
| hsa-miR-4744 | 0.0 | 0.0 | 0.0 | 0.0 | 0.0 | 0.0 | 0.0 | 0.0 | 0.0 |
| hsa-miR-4753-3p | 2.5 | 3.1 | 2.9 | 2.7 | 0.5 | 2.8 | 2.6 | 2.1 | 2.6 |
| hsa-miR-4753-5p | 0.0 | 2.1 | 2.0 | 2.3 | 2.5 | 1.9 | 3.8 | 3.3 | 3.7 |
| hsa-miR-4757-5p | 0.0 | 0.0 | 3.2 | 0.0 | 0.0 | 0.0 | 0.0 | 0.0 | 0.0 |
| hsa-miR-4759 | 2.5 | 0.0 | 1.4 | 0.0 | 0.0 | 0.0 | 0.0 | 0.0 | 0.0 |
| hsa-miR-4760-5p | 0.0 | 0.0 | 0.0 | 0.0 | 0.0 | 0.0 | 0.0 | 0.0 | 0.0 |
| hsa-miR-4761-5p | 0.0 | 2.5 | 3.7 | 0.0 | 0.0 | 0.0 | 0.0 | 0.0 | 0.0 |
| hsa-miR-4762-5p | 2.2 | 2.7 | 0.0 | 2.5 | 2.2 | 2.0 | 1.9 | 0.0 | 1.1 |
| hsa-miR-4764-5p | 0.0 | 0.0 | 1.5 | 0.0 | 0.0 | 0.0 | 0.0 | 0.0 | 0.0 |
| hsa-miR-4768-5p | 2.2 | 3.4 | 3.5 | 2.8 | 1.5 | 3.1 | 2.0 | 0.0 | 1.1 |
| hsa-miR-4770 | 0.0 | 1.4 | 2.5 | 0.0 | 0.7 | 0.0 | 0.0 | 0.0 | 1.5 |
| hsa-miR-4772-3p | 0.0 | 0.0 | 0.0 | 0.0 | 0.0 | 0.0 | 0.0 | 0.0 | 0.0 |
| hsa-miR-4774-3p | 0.0 | 1.6 | 3.4 | 0.0 | 0.0 | 0.0 | 0.0 | 0.0 | 0.0 |
| hsa-miR-4774-5p | 0.0 | 0.0 | 0.0 | 0.0 | 0.0 | 0.0 | 0.0 | 0.0 | 0.0 |
| hsa-miR-4777-5p | 0.0 | 0.0 | 0.0 | 0.0 | 0.0 | 0.0 | 0.0 | 0.0 | 0.0 |
| hsa-miR-4778-3p | 0.0 | 0.0 | 0.0 | 0.0 | 0.0 | 0.0 | 0.0 | 0.0 | 0.0 |
| hsa-miR-4778-5p | 0.0 | 0.0 | 0.0 | 0.0 | 0.6 | 2.4 | 0.0 | 0.0 | 0.0 |
| hsa-miR-4779 | 1.3 | 0.0 | 2.0 | 0.0 | 0.0 | 0.0 | 0.0 | 0.0 | 0.0 |
| hsa-miR-4781-5p | 2.1 | 0.0 | 0.0 | 0.0 | 0.0 | 0.0 | 0.0 | 0.0 | 0.0 |
| hsa-miR-4782-5p | 0.0 | 0.0 | 0.0 | 0.0 | 0.0 | 0.0 | 0.0 | 0.0 | 0.0 |
| hsa-miR-4785 | 4.4 | 4.1 | 3.0 | 1.9 | 0.0 | 1.1 | 2.2 | 2.1 | 1.3 |
| hsa-miR-4789-3p | 0.0 | 0.0 | 0.0 | 0.0 | 0.0 | 0.0 | 0.0 | 0.0 | 0.0 |
| hsa-miR-4789-5p | 0.0 | 0.0 | 0.0 | 0.0 | 0.0 | 0.0 | 0.0 | 0.0 | 0.0 |
| hsa-miR-4791 | 0.0 | 0.0 | 0.0 | 0.0 | 0.0 | 0.0 | 0.0 | 0.0 | 0.0 |
| hsa-miR-4792 | 0.0 | 0.0 | 2.0 | 0.0 | 0.0 | 0.0 | 0.0 | 2.1 | 3.4 |
| hsa-miR-4795-3p | 0.0 | 0.0 | 2.8 | 0.0 | 0.0 | 0.0 | 0.0 | 0.0 | 0.0 |
| hsa-miR-4797-5p | 0.0 | 1.1 | 0.0 | 0.0 | 0.0 | 0.0 | 0.0 | 0.0 | 0.0 |
| hsa-miR-4798-5p | 0.0 | 0.0 | 0.0 | 0.0 | 0.0 | 0.0 | 0.0 | 0.0 | 0.0 |
| hsa-miR-4801 | 0.0 | 0.0 | 2.1 | 2.0 | 0.0 | 2.3 | 3.1 | 0.1 | 3.7 |
| hsa-miR-4803 | 0.0 | 0.0 | 2.7 | 0.0 | 0.8 | 0.0 | 0.0 | 0.0 | 0.0 |
| hsa-miR-4804-5p | 1.1 | 1.5 | 4.0 | 0.0 | 0.0 | 0.0 | 1.7 | 0.9 | 1.4 |
| hsa-miR-4999-3p | 2.0 | 1.5 | 3.3 | 2.3 | 2.7 | 1.7 | 2.6 | 2.3 | 2.9 |
| hsa-miR-5000-3p | 0.0 | 1.7 | 2.1 | 1.9 | 0.0 | 1.5 | 0.0 | 2.0 | 2.3 |
| hsa -miR -5004-3p | 2.9 | 2.9 | 2.7 | 2.8 | 2.3 | 2.4 | 2.1 | 3.1 | 2.3 |
| hsa-miR-5007-3p | 0.0 | 1.0 | 0.0 | 0.0 | 0.0 | 1.8 | 0.0 | 0.0 | 0.0 |
| hsa-miR-5009-5p | 0.0 | 0.0 | 0.0 | 0.0 | 0.0 | 0.0 | 0.0 | 0.0 | 0.0 |
| hsa-miR-5047 | 0.0 | 0.0 | 2.3 | 0.0 | 0.0 | 0.0 | 0.0 | 0.0 | 0.0 |
| hsa-miR-5089-5p | 0.0 | 0.0 | 2.1 | 0.0 | 0.0 | 0.0 | 0.0 | 0.0 | 0.0 |
| hsa-miR-5092 | 0.0 | 2.3 | 2.9 | 0.0 | 0.0 | 0.0 | 0.0 | 0.0 | 0.0 |
| hsa-miR-5579-5p | 0.0 | 0.0 | 1.7 | 0.0 | 0.0 | 0.0 | 0.0 | 0.0 | 0.0 |
| hsa -miR -5582-5p | 0.0 | 0.0 | 1.7 | 0.0 | 0.0 | 0.0 | 0.0 | 0.0 | 0.0 |
| hsa -miR -5584-5p | 0.0 | 0.0 | 0.0 | 0.0 | 0.0 | 0.0 | 0.0 | 1.6 | 1.7 |
| hsa-miR-5585-5p | 0.0 | 0.0 | 1.2 | 0.0 | 0.0 | 0.0 | 0.0 | 0.0 | 0.0 |
| hsa -miR -5586-3p | 0.0 | 0.0 | 0.0 | 0.0 | 0.0 | 0.0 | 0.0 | 0.4 | 0.0 |
| hsa -miR -5586-5p | 0.0 | 0.0 | 1.6 | 0.0 | 0.0 | 0.0 | 0.0 | 0.0 | 0.0 |
| hsa-miR-5587-5p | 3.9 | 1.9 | 0.0 | 1.5 | 0.0 | 0.0 | 0.0 | 1.8 | 2.6 |
| hsa -miR -5588-5p | 1.8 | 2.8 | 2.3 | 0.0 | 0.0 | 1.8 | 1.9 | 1.6 | 1.9 |
| hsa-miR-5590-3p | 0.0 | 0.0 | 0.0 | 0.0 | 0.0 | 0.0 | 0.0 | 0.0 | 0.0 |
| hsa-miR-5590-5p | 0.0 | 0.0 | 0.0 | 0.0 | 0.0 | 0.0 | 0.0 | 0.0 | 0.0 |
| hsa-miR-5684 | 0.0 | 1.8 | 2.3 | 1.9 | 0.0 | 2.3 | 1.8 | 0.3 | 1.9 |
| hsa-miR-5691 | 0.0 | 0.0 | 0.0 | 0.0 | 0.0 | 0.0 | 0.0 | 0.0 | 0.0 |
| hsa -miR -5692b | 0.0 | 0.0 | 0.0 | 0.0 | 0.0 | 0.0 | 0.0 | 0.0 | 0.0 |
| hsa-miR-5697 | 0.0 | 0.0 | 0.0 | 0.0 | 0.0 | 0.0 | 0.0 | 0.0 | 0.0 |
| hsa-miR-5707 | 0.0 | 0.0 | 2.4 | 2.3 | 0.0 | 0.0 | 0.0 | 0.0 | 1.3 |
| hsa-miR-6070 | 2.0 | 1.1 | 0.0 | 0.0 | 0.7 | 0.0 | 0.0 | 0.0 | 0.0 |
| hsa-miR-6071 | 3.6 | 2.2 | 1.9 | 0.0 | 4.2 | 2.6 | 0.0 | 1.5 | 0.0 |
| hsa-miR-6074 | 0.0 | 1.5 | 1.6 | 1.7 | 0.0 | 0.0 | 3.3 | 2.4 | 3.9 |
| hsa-miR-6078 | 0.0 | 0.0 | 1.4 | 0.0 | 0.0 | 0.0 | 0.0 | 0.0 | 0.0 |
| hsa-miR-6080 | 0.0 | 2.8 | 1.2 | 0.0 | 0.0 | 3.1 | 2.1 | 4.4 | 3.4 |
| hsa-miR-6084 | 1.0 | 2.8 | 1.8 | 0.0 | 2.7 | 2.4 | 0.0 | 2.5 | 1.5 |
| hsa-miR-6129 | 0.0 | 3.1 | 3.5 | 1.7 | 0.0 | 3.0 | 2.5 | 3.0 | 2.4 |
| hsa-miR-6130 | 0.0 | 2.4 | 0.0 | 0.0 | 0.0 | 2.3 | 2.3 | 3.3 | 1.2 |
| hsa-miR-6501-3p | 0.0 | 3.1 | 3.2 | 0.0 | 0.0 | 1.9 | 1.8 | 4.0 | 1.1 |
| hsa -miR -6502-3p | 0.0 | 0.0 | 0.0 | 0.0 | 0.0 | 0.0 | 0.0 | 0.0 | 0.0 |
| hsa -miR -6502-5p | 1.1 | 2.7 | 3.4 | 1.9 | 0.0 | 0.0 | 1.4 | 0.2 | 0.0 |
| hsa -miR -6506-3p | 0.0 | 0.0 | 2.8 | 0.0 | 0.0 | 0.0 | 0.0 | 0.0 | 0.0 |
| hsa-miR-6516-3p | 0.0 | 0.0 | 0.0 | 0.0 | 0.0 | 0.0 | 0.0 | 0.0 | 0.0 |
| hsa-miR-6715a-3p | 1.2 | 2.2 | 3.2 | 2.7 | 0.5 | 2.2 | 2.1 | 0.2 | 0.0 |
| hsa-miR-6715b-3p | 0.0 | 0.0 | 1.7 | 0.0 | 0.0 | 0.0 | 0.0 | 0.0 | 0.0 |
| hsa-miR-6719-3p | 0.0 | 0.0 | 1.7 | 0.0 | 0.0 | 0.0 | 0.0 | 0.0 | 0.0 |
| hsa-miR-6750-5p | 0.0 | 2.3 | 3.0 | 2.2 | 2.2 | 3.2 | 2.4 | 5.3 | 3.9 |
| hsa-miR-6755-5p | 0.0 | 0.0 | 0.0 | 0.0 | 0.0 | 0.0 | 0.0 | 0.0 | 0.0 |
| hsa-miR-6767-3p | 2.2 | 2.8 | 2.9 | 2.7 | 0.0 | 2.9 | 1.7 | 0.3 | 0.0 |
| hsa-miR-6773-5p | 0.0 | 0.0 | 2.4 | 0.0 | 0.0 | 1.8 | 0.0 | 0.0 | 0.0 |
| hsa -miR -6806-3p | 0.0 | 0.0 | 0.0 | 0.0 | 0.0 | 0.0 | 0.0 | 0.0 | 0.0 |
| hsa -miR -6808-3p | 2.0 | 0.0 | 2.8 | 0.0 | 0.0 | 2.2 | 1.8 | 0.0 | 0.0 |
| hsa-miR-6811-5p | 0.0 | 0.0 | 0.0 | 0.0 | 0.0 | 0.0 | 0.0 | 0.0 | 0.0 |
| hsa-miR-6814-5p | 2.3 | 3.2 | 3.2 | 3.6 | 0.0 | 3.4 | 2.4 | 3.2 | 2.7 |
| hsa -miR -6828-3p | 2.4 | 2.4 | 2.8 | 0.0 | 0.0 | 0.0 | 0.0 | 0.0 | 0.0 |
| hsa-miR-6835-3p | 1.6 | 0.0 | 1.4 | 0.0 | 0.0 | 0.0 | 0.0 | 0.0 | 0.0 |
| hsa -miR -6838-3p | 0.0 | 0.0 | 0.0 | 0.0 | 0.0 | 0.0 | 0.0 | 0.0 | 1.3 |
| hsa -miR -6842-3p | 0.0 | 2.1 | 1.5 | 0.0 | 0.0 | 1.8 | 0.0 | 2.9 | 2.6 |
| hsa-miR-6853-3p | 0.0 | 0.0 | 0.0 | 0.0 | 0.0 | 0.0 | 0.0 | 0.0 | 0.0 |
| hsa -miR -6854-5p | 1.7 | 0.0 | 2.1 | 0.0 | 0.0 | 0.0 | 0.0 | 0.0 | 0.0 |
| hsa -miR -6864-5p | 0.0 | 1.9 | 2.6 | 1.6 | 2.3 | 1.7 | 0.0 | 0.0 | 0.0 |
| hsa -miR -6866-5p | 2.0 | 0.0 | 2.8 | 0.0 | 0.0 | 0.0 | 1.7 | 0.0 | 0.0 |
| hsa-miR-6869-3p | 0.0 | 0.0 | 0.0 | 0.0 | 2.7 | 0.0 | 0.0 | 0.0 | 0.0 |
| hsa -miR -6882-5p | 2.4 | 1.2 | 0.0 | 1.6 | 2.2 | 0.0 | 1.5 | 1.7 | 2.5 |
| hsa -miR -6888-3p | 1.1 | 0.0 | 0.0 | 0.0 | 0.0 | 0.0 | 0.0 | 0.0 | 0.0 |
| hsa-miR-7153-5p | 0.0 | 0.0 | 0.0 | 1.6 | 0.0 | 0.0 | 0.0 | 0.0 | 1.4 |
| hsa-miR-7154-5p | 0.0 | 0.0 | 0.0 | 0.0 | 0.0 | 0.0 | 0.0 | 0.9 | 0.0 |
| hsa-miR-7156-3p | 0.0 | 1.6 | 2.2 | 2.5 | 1.4 | 3.1 | 3.0 | 3.4 | 3.3 |
| hsa-miR-7156-5p | 2.2 | 3.3 | 3.7 | 2.7 | 3.4 | 3.6 | 2.7 | 1.2 | 2.0 |
| hsa-miR-7158-3p | 2.3 | 0.0 | 3.6 | 1.7 | 3.3 | 2.1 | 2.2 | 0.0 | 0.0 |
| hsa-miR-7159-3p | 0.0 | 0.0 | 0.0 | 0.0 | 0.0 | 0.0 | 0.0 | 0.0 | 0.0 |
| hsa-miR-7160-3p | 1.8 | 0.0 | 0.0 | 0.0 | 0.0 | 0.0 | 0.0 | 0.0 | 0.0 |
| hsa-miR-7515 | 0.0 | 0.0 | 0.0 | 0.0 | 1.5 | 2.3 | 2.2 | 3.5 | 1.2 |
| hsa-miR-7705 | 2.7 | 0.0 | 0.0 | 0.0 | 0.0 | 0.0 | 0.0 | 0.0 | 0.0 |
| hsa-miR-7706 | 2.3 | 2.5 | 3.4 | 2.0 | 2.5 | 2.3 | 2.0 | 2.2 | 0.0 |
| hsa-miR-7978 | 2.3 | 2.1 | 2.9 | 0.0 | 0.0 | 0.0 | 0.0 | 0.0 | 0.0 |
| hsa-miR-8053 | 1.3 | 0.0 | 0.0 | 0.0 | 0.0 | 0.0 | 0.0 | 0.0 | 0.0 |
| hsa-miR-8054 | 0.0 | 0.0 | 1.6 | 0.0 | 0.0 | 0.0 | 0.0 | 0.0 | 0.0 |
| hsa-miR-8056 | 0.0 | 1.4 | 0.0 | 0.0 | 0.0 | 0.0 | 0.0 | 0.0 | 0.0 |
| hsa-miR-8058 | 0.0 | 0.0 | 2.1 | 0.0 | 1.5 | 1.8 | 0.0 | 0.0 | 0.0 |
| hsa-miR-8067 | 0.0 | 0.0 | 1.8 | 0.0 | 0.0 | 0.0 | 0.0 | 0.0 | 0.0 |
| hsa-miR-8068 | 0.0 | 0.0 | 0.0 | 0.0 | 2.2 | 1.6 | 1.5 | 0.0 | 0.0 |
| hsa-miR-8074 | 0.0 | 2.6 | 2.5 | 2.1 | 0.0 | 2.7 | 2.6 | 3.7 | 2.4 |
| hsa-miR-8079 | 2.3 | 2.0 | 3.6 | 0.0 | 2.6 | 1.7 | 1.4 | 0.0 | 1.7 |
| hsa-miR-8086 | 0.0 | 1.6 | 3.1 | 0.0 | 0.0 | 1.7 | 1.6 | 1.7 | 1.4 |
| hsa-miR-8088 | 0.0 | 0.0 | 0.0 | 0.0 | 0.0 | 0.0 | 0.0 | 0.0 | 0.0 |
| hsa -miR -4694-5p | 0.0 | 2.1 | 0.0 | 1.6 | 0.0 | 1.2 | 0.0 | 0.0 | 0.0 |
| hsa-miR-4746-5p | 0.0 | 0.0 | 0.0 | 0.0 | 0.0 | 0.0 | 0.0 | 0.0 | 0.0 |
| hsa-miR-0517-5p | 2.6 | 0.0 | 2.7 | 2.9 | 2.6 | 0.0 | 1.8 | 1.1 | 2.0 |
| hsa-miR-4302 | 0.0 | 0.0 | 0.0 | 0.0 | 0.0 | 0.0 | 0.0 | 0.0 | 0.0 |
| hsa-miR-3908 | 1.8 | 1.6 | 2.5 | 0.0 | 0.0 | 2.3 | 0.0 | 0.3 | 0.0 |
| hsa-miR-0611 | 1.9 | 2.0 | 0.0 | 0.0 | 2.3 | 0.0 | 0.0 | 0.0 | 1.3 |
| hsa-miR-0873-5p | 0.0 | 0.0 | 0.0 | 0.0 | 0.0 | 0.0 | 0.0 | 0.0 | 0.0 |
| hsa-miR-4423-5p | 0.0 | 1.4 | 3.1 | 2.8 | 0.8 | 2.4 | 2.7 | 2.7 | 2.3 |
| hsa-miR-0383-5p | 0.0 | 0.0 | 1.4 | 1.7 | 1.3 | 0.0 | 0.0 | 0.0 | 0.0 |
| hsa-miR-6500-5p | 1.6 | 1.9 | 3.0 | 2.9 | 1.9 | 2.0 | 1.9 | 1.4 | 2.1 |
| hsa-miR-4454 | 0.0 | 3.5 | 4.0 | 2.8 | 3.5 | 2.8 | 2.5 | 3.1 | 2.4 |
| hsa-miR-0203b-3p | 0.0 | 2.2 | 2.2 | 0.0 | 0.0 | 0.0 | 0.0 | 0.0 | 1.3 |
| hsa-miR-4711-5p | 0.0 | 0.0 | 0.0 | 0.0 | 0.0 | 0.0 | 0.0 | 0.0 | 0.0 |
| hsa-miR-0664a-5p | 3.5 | 2.7 | 3.7 | 2.8 | 0.9 | 0.0 | 0.0 | 0.0 | 0.0 |
| hsa -miR -0548v | 0.0 | 0.0 | 0.0 | 0.0 | 0.0 | 0.0 | 0.0 | 0.0 | 0.0 |
| hsa-miR-3916 | 0.0 | 1.8 | 2.6 | 0.0 | 0.0 | 2.0 | 1.8 | 0.0 | 0.0 |
| hsa-miR-4691-3p | 1.5 | 1.9 | 2.0 | 0.0 | 1.4 | 0.0 | 0.0 | 0.8 | 1.2 |
| hsa-miR-0548z, hsa-miR-548h-3p | 0.0 | 0.0 | 0.0 | 0.0 | 0.0 | 0.0 | 0.0 | 0.0 | 0.0 |
| hsa-miR-0027b-5p | 0.0 | 0.0 | 0.0 | 0.0 | 1.7 | 0.0 | 0.0 | 0.0 | 0.0 |
| hsa-miR-2681-5p | 2.7 | 3.5 | 3.4 | 2.2 | 2.7 | 2.6 | 2.5 | 2.8 | 1.6 |
| hsa-miR-3161 | 3.0 | 3.2 | 3.6 | 2.7 | 0.0 | 2.1 | 3.5 | 2.6 | 2.2 |
| hsa-miR-0604 | 2.7 | 2.9 | 3.3 | 3.4 | 3.0 | 2.6 | 0.0 | 2.3 | 2.7 |
| hsa-miR-1269b | 0.0 | 4.6 | 2.2 | 0.0 | 2.5 | 2.1 | 2.1 | 0.0 | 1.4 |
| hsa-miR-4659b-3p | 0.0 | 0.0 | 2.2 | 0.0 | 1.2 | 0.0 | 1.8 | 0.0 | 1.4 |
| hsa-miR-0938 | 3.6 | 2.3 | 2.8 | 3.5 | 1.7 | 2.4 | 1.8 | 0.5 | 0.0 |
| hsa-miR-4733-3p | 3.3 | 2.9 | 2.9 | 3.7 | 2.3 | 3.0 | 3.0 | 2.4 | 3.7 |
| hsa-miR-0491-3p | 3.2 | 2.7 | 2.6 | 2.4 | 1.9 | 1.5 | 2.5 | 1.0 | 2.4 |
| hsa-miR-6505-5p | 1.9 | 2.7 | 2.1 | 0.0 | 0.0 | 0.0 | 0.0 | 0.0 | 0.0 |
| hsa-miR-0630 | 1.7 | 3.2 | 2.7 | 1.9 | 0.4 | 2.6 | 0.0 | 0.0 | 0.0 |
| hsa -miR -3688-3p | 0.0 | 0.0 | 0.0 | 0.0 | 0.0 | 0.0 | 0.0 | 0.0 | 1.7 |
| hsa-miR-3179 | 0.0 | 1.5 | 1.3 | 0.0 | 1.5 | 1.0 | 0.0 | 2.9 | 0.0 |
| hsa-miR-4755-3p | 0.0 | 0.0 | 2.0 | 1.6 | 0.0 | 0.0 | 0.0 | 1.4 | 2.3 |
| hsa-miR-6720-5p | 2.1 | 3.9 | 2.1 | 2.7 | 0.0 | 3.0 | 0.0 | 1.4 | 2.0 |
| hsa -miR -0452-3p | 1.7 | 1.9 | 2.6 | 2.1 | 3.1 | 2.2 | 0.0 | 0.0 | 0.0 |
| hsa-miR-0374b-3p | 0.0 | 0.0 | 0.0 | 0.0 | 0.0 | 0.0 | 0.0 | 0.0 | 0.0 |
| hsa -miR -0022-3p | 0.0 | 0.0 | 0.0 | 0.0 | 0.9 | 0.0 | 0.0 | 0.0 | 0.0 |
| hsa -miR -0222-3p | 1.8 | 0.0 | 3.0 | 2.0 | 2.2 | 0.0 | 2.3 | 0.0 | 0.0 |
| hsa-miR-0093-3p | 1.3 | 2.0 | 3.0 | 2.1 | 0.0 | 0.0 | 0.0 | 0.0 | 0.0 |
| hsa-miR-0200a-5p | 2.1 | 2.5 | 3.5 | 3.7 | 2.9 | 2.0 | 3.5 | 2.5 | 3.2 |
| hsa -miR -0362-3p | 2.0 | 1.6 | 1.6 | 2.6 | 1.1 | 0.0 | 1.5 | 0.0 | 0.0 |
| hsa-miR-0144-5p | 0.0 | 1.3 | 2.2 | 0.0 | 0.0 | 0.0 | 0.0 | 0.0 | 0.0 |
| hsa -miR -0628-3p | 1.9 | 1.1 | 1.4 | 1.7 | 0.0 | 1.8 | 0.0 | 0.0 | 0.0 |
| hsa-miR-0378c | 0.0 | 3.2 | 3.8 | 2.7 | 1.9 | 2.8 | 1.8 | 0.4 | 1.5 |
| hsa-miR-0335-5p | 0.0 | 0.0 | 0.0 | 0.0 | 0.0 | 0.0 | 0.0 | 0.0 | 0.0 |
| hsa-miR-7112-3p | 0.0 | 3.6 | 4.1 | 1.5 | 3.4 | 4.3 | 2.0 | 1.7 | 3.0 |
| hsa-miR-5009-3p | 0.0 | 0.0 | 0.0 | 0.0 | 0.0 | 0.0 | 0.0 | 0.0 | 0.0 |
| hsa-let-7g-5p | 2.0 | 0.0 | 0.0 | 0.0 | 0.0 | 0.0 | 0.0 | 0.0 | 0.0 |
| hsa-miR-0130a-5p | 0.0 | 3.0 | 3.6 | 2.9 | 0.0 | 2.1 | 2.4 | 1.2 | 2.0 |
| hsa-miR-0215-5p | 1.5 | 0.0 | 1.9 | 2.9 | 1.1 | 0.0 | 0.0 | 0.0 | 0.0 |
| hsa -miR -3186-3p | 4.0 | 4.2 | 4.1 | 3.7 | 2.7 | 4.3 | 3.4 | 4.4 | 4.0 |
| hsa-miR-4745-3p | 3.9 | 4.2 | 3.3 | 3.4 | 2.0 | 1.7 | 0.0 | 0.0 | 0.0 |
| hsa-miR-0449a | 2.0 | 1.4 | 1.9 | 3.0 | 2.1 | 2.0 | 2.1 | 1.3 | 2.0 |
| hsa-miR-0017-3p | 1.1 | 0.0 | 2.7 | 1.5 | 2.2 | 0.0 | 0.0 | 0.0 | 0.0 |
| hsa-miR-4786-5p | 1.9 | 2.8 | 3.7 | 3.6 | 3.6 | 3.7 | 2.9 | 3.2 | 3.0 |
| hsa-miR-5695 | 0.0 | 1.7 | 2.4 | 0.0 | 0.0 | 0.0 | 0.0 | 0.0 | 0.0 |
| hsa-miR-3927-3p | 0.0 | 0.0 | 1.6 | 0.0 | 0.0 | 0.0 | 0.0 | 0.0 | 0.0 |
| hsa-miR-0200b-5p | 3.1 | 3.0 | 3.6 | 3.0 | 1.2 | 1.6 | 2.9 | 2.8 | 2.0 |
| hsa-miR-0010a-5p | 0.0 | 1.5 | 2.6 | 0.0 | 0.0 | 1.0 | 0.0 | 0.0 | 0.0 |
| hsa-miR-0181d-3p | 2.3 | 1.9 | 3.2 | 3.6 | 3.6 | 3.0 | 0.0 | 3.8 | 2.9 |
| hsa-miR-0369-3p | 0.0 | 0.0 | 0.0 | 0.0 | 0.0 | 0.0 | 0.0 | 0.0 | 0.0 |
| hsa-miR-0578 | 0.0 | 2.5 | 2.5 | 2.3 | 0.0 | 1.9 | 2.1 | 1.3 | 2.1 |
| hsa-miR-3193 | 2.0 | 3.0 | 0.0 | 2.8 | 0.0 | 1.1 | 0.0 | 1.0 | 2.2 |
| hsa-miR-0302a-3p | 1.8 | 0.0 | 1.4 | 2.4 | 2.3 | 0.0 | 0.0 | 0.0 | 1.1 |
| hsa-miR-0549a | 0.0 | 0.0 | 0.0 | 0.0 | 0.0 | 0.0 | 0.0 | 0.0 | 0.0 |
| hsa-miR-1255b-2-3p | 0.0 | 1.5 | 2.6 | 0.0 | 0.0 | 1.9 | 0.0 | 2.0 | 3.2 |
| hsa-miR-6510-3p | 3.0 | 3.6 | 3.1 | 2.3 | 0.8 | 2.5 | 1.5 | 0.7 | 1.6 |
| hsa-miR-0363-5p | 0.0 | 1.3 | 1.4 | 1.6 | 0.0 | 0.0 | 0.0 | 1.9 | 0.0 |
| hsa -miR -0028-5p | 3.1 | 0.0 | 4.2 | 4.1 | 4.3 | 3.1 | 4.3 | 2.6 | 3.5 |
| hsa-miR-4720-5p | 0.0 | 2.9 | 3.3 | 2.3 | 0.0 | 2.8 | 0.0 | 1.9 | 1.9 |
| hsa -miR -0202-3p | 3.1 | 1.6 | 2.9 | 2.5 | 3.1 | 1.9 | 0.0 | 0.0 | 0.0 |
| hsa-miR-4255 | 2.4 | 0.0 | 0.0 | 2.9 | 2.8 | 2.5 | 1.6 | 2.4 | 1.6 |
| hsa -miR -0449c-5p | 3.8 | 4.3 | 3.0 | 3.3 | 0.0 | 2.8 | 2.5 | 2.0 | 2.2 |
| hsa-miR-0181b-3p | 0.0 | 0.0 | 1.7 | 0.0 | 0.0 | 0.0 | 0.0 | 0.4 | 0.0 |
| hsa-miR-4775 | 1.8 | 2.9 | 3.4 | 2.3 | 0.5 | 0.0 | 2.0 | 0.0 | 0.0 |
| hsa-miR-0378h | 1.7 | 2.4 | 2.8 | 2.7 | 1.6 | 1.7 | 2.5 | 2.2 | 2.4 |
| hsa-miR-0152-5p | 1.2 | 2.6 | 1.8 | 2.4 | 0.0 | 0.0 | 2.8 | 1.4 | 2.6 |
| hsa-miR-0513b-5p | 3.2 | 1.6 | 0.0 | 2.2 | 3.2 | 3.8 | 2.0 | 0.8 | 3.3 |
| hsa-miR-0029b-3p | 3.1 | 1.3 | 1.1 | 2.2 | 2.4 | 0.0 | 0.0 | 0.8 | 0.0 |
| hsa-miR-1297 | 3.3 | 3.6 | 2.7 | 3.4 | 0.0 | 1.8 | 2.5 | 0.0 | 1.9 |
| hsa -miR -0020b-3p | 0.0 | 1.4 | 2.6 | 0.0 | 0.0 | 0.0 | 2.1 | 2.1 | 0.0 |
| hsa-miR-0541-3p | 1.4 | 2.2 | 2.7 | 2.8 | 0.4 | 2.0 | 3.4 | 4.0 | 3.3 |
| hsa-miR-1301-3p | 2.4 | 1.6 | 0.0 | 1.6 | 0.0 | 0.0 | 2.7 | 4.3 | 2.6 |
| hsa-miR-3115 | 0.0 | 0.0 | 0.0 | 0.0 | 0.0 | 0.0 | 0.0 | 0.0 | 0.0 |
| hsa-miR-6807-3p | 2.2 | 1.4 | 2.7 | 0.0 | 0.0 | 0.0 | 0.0 | 0.0 | 0.0 |
| hsa -miR -0532-5p | 1.9 | 0.0 | 0.0 | 0.0 | 0.7 | 0.0 | 0.0 | 0.0 | 2.0 |
| hsa -miR -6864-3p | 1.5 | 2.5 | 2.9 | 2.0 | 2.6 | 1.7 | 1.5 | 1.5 | 1.3 |
| hsa-miR-0196a-5p | 1.8 | 1.5 | 2.5 | 2.3 | 0.0 | 2.7 | 0.0 | 0.0 | 2.1 |
| hsa-miR-4676-3p | 3.4 | 3.4 | 3.4 | 2.6 | 2.9 | 2.7 | 2.0 | 2.1 | 1.8 |
| hsa -miR -0096-3p | 0.0 | 1.9 | 2.0 | 1.6 | 0.0 | 0.0 | 0.0 | 0.0 | 0.0 |
| hsa-miR-0126-3p | 0.0 | 0.0 | 2.8 | 0.0 | 0.0 | 0.0 | 0.0 | 0.0 | 0.0 |
| hsa-miR-0550a-3-5p | 3.0 | 3.7 | 3.7 | 3.0 | 3.0 | 4.1 | 3.6 | 4.3 | 4.9 |
| hsa-miR-0378f | 1.7 | 0.0 | 1.4 | 0.0 | 0.0 | 2.3 | 1.7 | 0.3 | 0.0 |
| hsa-miR-5581-5p | 0.0 | 1.3 | 2.8 | 0.0 | 0.6 | 4.4 | 1.9 | 0.0 | 0.0 |
| hsa-miR-0627-5p | 1.7 | 2.5 | 2.8 | 2.9 | 3.2 | 1.3 | 0.0 | 0.0 | 3.4 |
| hsa-miR-0649 | 1.6 | 0.0 | 1.8 | 2.3 | 0.0 | 1.2 | 0.0 | 0.0 | 2.2 |
| hsa-miR-0626 | 0.0 | 0.0 | 2.1 | 0.0 | 0.0 | 0.0 | 0.0 | 0.0 | 2.9 |
| hsa-miR-5007-5p | 1.5 | 2.4 | 4.0 | 3.5 | 2.9 | 2.5 | 3.1 | 3.0 | 2.8 |
| hsa-miR-7107-3p | 2.9 | 1.8 | 2.7 | 2.6 | 2.5 | 2.5 | 3.9 | 1.9 | 2.6 |
| hsa -miR -0030e-3p | 2.7 | 3.8 | 3.2 | 2.9 | 0.0 | 1.6 | 2.2 | 0.6 | 1.4 |
| hsa-miR-5589-3p | 1.1 | 2.5 | 0.0 | 0.0 | 0.0 | 0.0 | 0.0 | 0.0 | 0.0 |
| hsa-miR-0128-3p | 0.0 | 0.0 | 2.1 | 0.0 | 0.0 | 0.0 | 0.0 | 0.0 | 0.0 |
| hsa-miR-0421 | 1.2 | 1.6 | 0.0 | 0.0 | 0.0 | 0.0 | 1.5 | 0.5 | 1.7 |
| hsa-miR-7856-5p | 1.6 | 2.7 | 3.3 | 2.4 | 3.6 | 3.2 | 3.9 | 2.7 | 4.0 |
| hsa-miR-0378e | 2.2 | 2.5 | 2.5 | 0.0 | 1.6 | 2.9 | 1.6 | 1.9 | 1.6 |
| hsa-miR-1205 | 2.5 | 3.2 | 3.9 | 3.3 | 0.0 | 2.9 | 1.5 | 0.0 | 0.0 |
| hsa-miR-0579-3p | 1.4 | 3.4 | 3.1 | 3.0 | 3.2 | 2.6 | 0.0 | 0.8 | 0.0 |
| hsa-miR-6839-3p | 0.0 | 2.7 | 2.1 | 0.0 | 0.0 | 0.0 | 0.0 | 0.3 | 0.0 |
| hsa-miR-0219b-3p | 0.0 | 0.0 | 1.5 | 0.0 | 0.0 | 0.0 | 0.0 | 0.0 | 0.0 |
| hsa-miR-0662 | 1.4 | 0.0 | 0.0 | 0.0 | 1.8 | 0.0 | 0.0 | 0.0 | 0.0 |
| hsa-miR-7153-3p | 1.6 | 0.0 | 3.3 | 1.6 | 0.0 | 2.6 | 0.0 | 0.0 | 0.0 |
| hsa-miR-4742-3p | 2.0 | 0.0 | 1.7 | 0.0 | 0.0 | 0.0 | 2.2 | 1.9 | 1.1 |
| hsa-miR-1303 | 4.0 | 3.8 | 2.9 | 3.7 | 4.7 | 3.7 | 3.7 | 4.4 | 4.7 |
| hsa-miR-3117-5p | 0.0 | 0.0 | 0.0 | 0.0 | 0.0 | 0.0 | 0.0 | 0.0 | 0.0 |
| hsa-miR-0576-5p | 2.2 | 2.4 | 2.5 | 2.1 | 2.2 | 0.0 | 1.7 | 0.0 | 2.1 |
| hsa -miR -0372-3p | 2.4 | 2.9 | 4.4 | 3.4 | 0.0 | 2.0 | 3.3 | 0.0 | 2.2 |
| hsa-let-7e-5p | 1.6 | 2.1 | 3.6 | 3.4 | 3.2 | 1.8 | 2.3 | 1.3 | 0.0 |
| hsa-miR-3677-5p | 0.0 | 1.7 | 0.0 | 0.0 | 0.0 | 0.0 | 0.0 | 0.0 | 0.0 |
| hsa-miR-0092a-1-5p | 0.0 | 0.0 | 0.0 | 0.0 | 0.0 | 0.0 | 0.0 | 0.0 | 0.0 |
| hsa-miR-0029a-3p | 1.7 | 0.0 | 1.7 | 2.3 | 0.0 | 0.0 | 0.0 | 0.7 | 0.0 |
| hsa-miR-0007-1-3p | 1.5 | 1.2 | 2.0 | 2.6 | 0.0 | 2.1 | 2.9 | 2.5 | 2.0 |
| hsa-miR-0450a-5p | 0.0 | 3.0 | 2.5 | 3.0 | 2.3 | 2.1 | 2.9 | 2.8 | 2.6 |
| hsa-miR-4330 | 2.1 | 3.1 | 2.8 | 2.4 | 1.8 | 1.7 | 2.1 | 2.0 | 2.5 |
| hsa-miR-0515-3p | 1.3 | 1.9 | 3.0 | 3.1 | 2.9 | 1.0 | 1.5 | 2.8 | 1.8 |
| hsa-miR-0323a-3p | 0.0 | 0.0 | 0.0 | 1.8 | 0.8 | 0.0 | 0.0 | 0.0 | 0.0 |
| hsa-miR-7159-5p | 1.2 | 1.8 | 1.8 | 1.9 | 2.2 | 2.0 | 1.8 | 2.1 | 2.2 |
| hsa-miR-4766-5p | 0.0 | 0.0 | 2.8 | 1.9 | 0.0 | 1.5 | 0.0 | 1.6 | 1.3 |
| hsa-miR-4524a-5p | 1.9 | 0.0 | 3.1 | 2.9 | 2.4 | 2.3 | 2.8 | 1.8 | 2.0 |
| hsa-miR-7843-3p | 2.8 | 1.8 | 3.4 | 0.0 | 1.7 | 1.2 | 0.0 | 0.0 | 2.1 |
| hsa-miR-6809-5p | 1.9 | 1.5 | 2.1 | 0.0 | 2.6 | 3.4 | 0.0 | 0.2 | 0.0 |
| hsa -miR -6888-5p | 2.3 | 2.9 | 3.0 | 3.1 | 3.4 | 3.3 | 3.2 | 2.5 | 2.7 |
| hsa-miR-2909 | 4.0 | 3.7 | 4.6 | 3.5 | 2.3 | 3.1 | 2.8 | 2.5 | 2.3 |
| hsa-miR-3164 | 3.5 | 3.1 | 2.6 | 3.7 | 2.8 | 3.0 | 4.5 | 4.8 | 5.0 |
| hsa-miR-4520-3p | 0.0 | 0.0 | 2.5 | 2.7 | 0.0 | 1.2 | 2.8 | 2.0 | 2.3 |
| hsa-miR-0510-5p | 3.5 | 3.3 | 3.5 | 2.8 | 1.6 | 2.0 | 2.5 | 4.1 | 0.0 |
| hsa-miR-4690-3p | 1.1 | 0.0 | 0.0 | 0.0 | 0.0 | 0.0 | 0.0 | 0.0 | 0.0 |
| hsa-miR-0026a-1-3p | 2.8 | 3.6 | 3.7 | 2.6 | 0.0 | 0.0 | 0.0 | 0.0 | 0.0 |
| hsa-miR-4793-5p | 2.7 | 2.8 | 2.1 | 3.2 | 0.8 | 0.0 | 0.0 | 0.1 | 1.3 |
| hsa -miR -0624-5p | 1.9 | 1.1 | 0.0 | 2.1 | 0.0 | 0.0 | 0.0 | 0.0 | 0.0 |
| hsa-miR-0617 | 0.0 | 0.0 | 0.0 | 0.0 | 2.1 | 0.0 | 2.4 | 4.7 | 3.1 |
| hsa-miR-0371a-3p | 3.9 | 3.8 | 3.5 | 3.1 | 2.8 | 2.6 | 1.6 | 0.9 | 0.0 |
| hsa-miR-0500a-5p | 2.4 | 2.6 | 3.5 | 2.9 | 1.2 | 3.1 | 2.4 | 1.3 | 0.0 |
| hsa-miR-3687 | 2.5 | 1.9 | 0.0 | 1.7 | 0.0 | 2.1 | 2.5 | 1.3 | 1.7 |
| hsa-miR-0378i | 2.0 | 2.1 | 3.8 | 2.1 | 0.5 | 2.1 | 3.0 | 1.8 | 2.1 |
| hsa -miR -3934-3p | 0.0 | 2.0 | 2.8 | 2.5 | 2.8 | 2.5 | 2.8 | 2.1 | 1.6 |
| hsa-miR-5688 | 0.0 | 1.3 | 3.0 | 0.0 | 0.0 | 0.0 | 0.0 | 0.0 | 0.0 |
| hsa-miR-6768-3p | 2.5 | 3.2 | 3.6 | 2.3 | 1.3 | 2.4 | 2.2 | 2.2 | 1.5 |
| hsa-miR-0010b-5p | 0.0 | 1.2 | 2.3 | 2.3 | 1.7 | 0.0 | 0.0 | 0.0 | 1.1 |
| hsa-miR-0023a-3p | 1.9 | 1.8 | 1.8 | 2.8 | 0.8 | 2.6 | 0.0 | 1.5 | 1.5 |
| hsa-miR-0373-3p | 2.7 | 2.2 | 2.5 | 0.0 | 0.0 | 2.7 | 1.9 | 0.0 | 0.0 |
| hsa-miR-0487b-3p | 0.0 | 2.1 | 2.9 | 2.3 | 0.0 | 1.6 | 2.2 | 2.3 | 2.1 |
| hsa-miR-4661-5p | 2.8 | 2.6 | 2.4 | 2.5 | 2.4 | 1.7 | 2.2 | 1.3 | 2.9 |
| hsa-miR-0767-5p | 1.4 | 2.4 | 3.6 | 2.6 | 0.0 | 2.8 | 3.2 | 1.9 | 2.6 |
| hsa-miR-0922 | 0.0 | 0.0 | 0.0 | 0.0 | 0.8 | 0.0 | 0.0 | 0.0 | 0.0 |
| hsa-miR-4677-5p | 0.0 | 2.4 | 2.7 | 0.0 | 1.3 | 1.5 | 1.6 | 0.0 | 1.3 |
| hsa -miR -0502-3p | 0.0 | 0.0 | 0.0 | 0.0 | 0.0 | 0.0 | 0.0 | 0.0 | 0.0 |
| hsa -miR -0598-5p | 3.2 | 2.7 | 3.3 | 3.8 | 2.5 | 3.4 | 3.6 | 3.0 | 2.3 |
| hsa-miR-4521 | 3.6 | 4.1 | 3.8 | 0.0 | 1.4 | 1.9 | 2.1 | 0.0 | 1.6 |
| hsa-miR-0215-3p | 1.2 | 0.0 | 3.1 | 0.0 | 1.9 | 0.0 | 0.0 | 0.0 | 0.0 |
| hsa-miR-4261 | 3.1 | 2.8 | 2.4 | 2.7 | 2.7 | 4.1 | 3.9 | 4.9 | 4.3 |
| hsa-miR-4280 | 3.0 | 2.3 | 2.5 | 3.0 | 0.0 | 2.9 | 2.7 | 2.5 | 3.2 |
| hsa-miR-0027a-3p | 3.2 | 0.0 | 1.9 | 2.0 | 1.2 | 0.0 | 0.0 | 0.0 | 0.0 |
| hsa-miR-6073 | 0.0 | 1.8 | 2.7 | 0.0 | 0.0 | 0.0 | 0.0 | 0.0 | 0.0 |
| hsa-miR-0029b-2-5p | 0.0 | 0.0 | 0.0 | 0.0 | 0.0 | 1.7 | 2.3 | 0.0 | 0.0 |
| hsa-miR-4794 | 3.7 | 3.7 | 3.6 | 3.2 | 2.3 | 3.3 | 3.0 | 2.6 | 2.3 |
| hsa-miR-4645-5p | 0.0 | 0.0 | 1.6 | 0.0 | 0.0 | 1.2 | 0.0 | 0.0 | 1.2 |
| hsa-miR-3529-3p | 0.0 | 0.0 | 0.0 | 0.0 | 0.0 | 0.0 | 0.0 | 0.0 | 0.0 |
| hsa-miR-4325 | 0.0 | 0.0 | 1.1 | 0.0 | 0.0 | 0.0 | 0.0 | 0.3 | 0.0 |
| hsa-miR-5702 | 0.0 | 2.9 | 3.2 | 3.2 | 3.1 | 2.4 | 0.0 | 2.1 | 0.0 |
| hsa-miR-4262 | 3.4 | 2.1 | 3.0 | 3.5 | 1.1 | 1.6 | 3.0 | 2.5 | 3.2 |
| hsa-miR-0891a-3p | 1.8 | 2.3 | 4.2 | 2.3 | 0.0 | 2.5 | 2.0 | 0.5 | 0.0 |
| hsa-miR-0205-5p | 3.2 | 3.2 | 3.3 | 3.0 | 2.5 | 2.9 | 1.7 | 1.7 | 1.9 |
| hsa-miR-8078 | 4.1 | 3.9 | 3.5 | 4.2 | 4.1 | 4.8 | 3.5 | 4.7 | 4.1 |
| hsa-miR-0181a-5p | 2.0 | 0.0 | 4.3 | 3.1 | 2.3 | 0.0 | 2.2 | 2.0 | 2.2 |
| hsa-miR-4314 | 2.9 | 3.4 | 1.9 | 2.0 | 0.0 | 2.0 | 2.5 | 2.2 | 2.5 |
| hsa-miR-4633-5p | 3.5 | 3.2 | 4.8 | 3.4 | 2.8 | 2.6 | 3.6 | 2.3 | 3.1 |
| hsa -miR -0502-5p | 2.1 | 0.0 | 2.9 | 2.7 | 0.0 | 0.0 | 1.5 | 1.0 | 0.0 |
| hsa -miR -0494-3p | 1.8 | 1.3 | 2.7 | 3.3 | 2.7 | 3.1 | 4.0 | 4.4 | 4.1 |
| hsa-miR-4317 | 2.7 | 2.6 | 2.1 | 2.8 | 0.0 | 2.2 | 2.6 | 1.2 | 1.6 |
| hsa-miR-5003-3p | 0.0 | 0.0 | 2.5 | 0.0 | 0.0 | 0.0 | 2.2 | 0.0 | 0.0 |
| hsa-miR-0203a-3p | 3.9 | 4.1 | 4.3 | 4.3 | 3.1 | 3.2 | 3.1 | 0.0 | 2.0 |
| hsa-miR-5693 | 2.9 | 2.4 | 3.6 | 2.8 | 0.0 | 2.0 | 0.0 | 1.0 | 2.3 |
| hsa-miR-0151b | 0.0 | 0.0 | 2.7 | 0.0 | 1.5 | 1.7 | 3.7 | 2.6 | 4.1 |
| hsa-miR-0135b-5p | 1.5 | 2.0 | 3.5 | 2.5 | 1.9 | 1.9 | 0.0 | 0.0 | 0.0 |
| hsa-miR-0614 | 4.2 | 4.2 | 2.6 | 2.5 | 2.3 | 2.9 | 2.9 | 4.6 | 4.0 |
| hsa-miR-5690 | 0.0 | 1.5 | 3.4 | 2.6 | 0.0 | 0.0 | 0.0 | 1.6 | 0.0 |
| hsa-miR-2467-5p | 0.0 | 0.0 | 2.1 | 0.0 | 0.0 | 1.0 | 1.8 | 0.0 | 1.2 |
| hsa-miR-3160-3p | 5.5 | 5.7 | 6.3 | 5.9 | 5.6 | 5.2 | 6.0 | 5.6 | 5.9 |
| hsa-miR-5011-3p | 0.0 | 1.1 | 2.5 | 0.0 | 0.0 | 0.0 | 1.7 | 0.0 | 0.0 |
| hsa-miR-0219b-5p | 1.1 | 3.7 | 3.1 | 2.4 | 2.1 | 2.2 | 2.6 | 2.4 | 0.0 |
| hsa-miR-4318 | 3.4 | 2.5 | 3.0 | 2.7 | 0.0 | 1.6 | 0.0 | 0.4 | 0.0 |
| hsa-miR-0593-5p | 3.6 | 3.1 | 3.3 | 3.3 | 1.7 | 2.3 | 2.5 | 2.2 | 2.5 |
| hsa-miR-0181b-5p | 2.9 | 2.0 | 2.4 | 3.2 | 2.3 | 2.3 | 3.1 | 2.2 | 2.8 |
| hsa-miR-0195-5p | 1.2 | 0.0 | 2.2 | 1.6 | 0.7 | 0.0 | 1.5 | 0.0 | 0.0 |
| hsa-miR-5003-5p | 0.0 | 0.0 | 0.0 | 0.0 | 0.0 | 0.0 | 0.0 | 0.0 | 0.0 |
| hsa-miR-6068 | 3.5 | 3.7 | 3.7 | 1.7 | 2.7 | 5.4 | 3.8 | 6.2 | 5.5 |
| hsa-miR-2682-5p | 3.3 | 2.9 | 3.1 | 3.2 | 2.5 | 2.0 | 3.4 | 1.6 | 3.0 |
| hsa-miR-3162-5p | 6.3 | 6.2 | 5.6 | 6.9 | 8.7 | 8.6 | 7.2 | 7.8 | 7.6 |
| hsa-miR-4494 | 3.0 | 3.0 | 3.5 | 2.8 | 2.8 | 2.6 | 0.0 | 1.7 | 2.3 |
| hsa-miR-0635 | 3.1 | 3.1 | 3.3 | 2.7 | 3.4 | 1.1 | 2.0 | 2.3 | 2.4 |
| hsa-miR-3677-3p | 2.8 | 1.7 | 2.4 | 0.0 | 0.0 | 0.0 | 0.0 | 0.0 | 0.0 |
| hsa -miR -4694-3p | 0.0 | 2.4 | 3.5 | 2.6 | 3.2 | 2.9 | 3.3 | 1.6 | 2.4 |
| hsa-miR-0676-3p | 0.0 | 1.4 | 0.0 | 0.0 | 0.0 | 0.0 | 0.0 | 0.0 | 0.0 |
| hsa-miR-0509-3-5p | 0.0 | 1.3 | 1.1 | 0.0 | 0.0 | 0.0 | 0.0 | 1.6 | 0.0 |
| hsa -miR -6832-5p | 2.9 | 3.0 | 3.0 | 3.6 | 3.5 | 3.1 | 3.3 | 2.6 | 3.5 |
| hsa -miR -3692-3p | 0.0 | 2.2 | 0.0 | 0.0 | 0.0 | 1.5 | 0.0 | 0.2 | 0.0 |
| hsa-miR-4672 | 0.0 | 2.4 | 2.5 | 2.7 | 2.1 | 2.5 | 3.0 | 2.7 | 2.7 |
| hsa-miR-0449b-5p | 1.7 | 2.8 | 0.0 | 2.1 | 1.4 | 2.1 | 3.0 | 1.5 | 0.0 |
| hsa-miR-4712-3p | 2.2 | 1.9 | 2.6 | 2.1 | 0.0 | 1.7 | 2.4 | 1.8 | 1.9 |
| hsa-miR-3622b-3p | 3.7 | 3.6 | 3.7 | 2.9 | 3.7 | 2.5 | 2.3 | 0.9 | 1.7 |
| hsa-miR-0489-5p | 3.3 | 3.1 | 4.3 | 3.9 | 2.3 | 3.2 | 3.1 | 4.6 | 2.9 |
| hsa-miR-0374b-5p | 2.8 | 3.5 | 3.6 | 2.3 | 0.0 | 0.0 | 0.0 | 0.0 | 0.0 |
| hsa-miR-4256 | 2.8 | 0.0 | 2.1 | 0.0 | 2.2 | 1.5 | 0.0 | 0.0 | 0.0 |
| hsa-miR-0411-3p | 3.4 | 2.8 | 3.1 | 2.9 | 1.0 | 2.1 | 3.2 | 2.4 | 2.2 |
| hsa-miR-2277-3p | 4.2 | 4.4 | 4.5 | 3.7 | 3.5 | 3.8 | 3.5 | 2.6 | 3.2 |
| hsa -miR -3156-3p | 5.4 | 5.8 | 5.7 | 5.9 | 5.7 | 5.7 | 5.5 | 4.9 | 5.1 |
| hsa-miR-6513-5p | 1.1 | 1.7 | 2.5 | 0.0 | 0.0 | 2.0 | 1.6 | 1.1 | 1.4 |
| hsa-miR-0023b-3p | 2.9 | 2.5 | 2.9 | 3.2 | 3.6 | 3.0 | 2.4 | 1.9 | 2.4 |
| hsa-miR-0194-5p | 2.7 | 2.4 | 2.1 | 3.1 | 2.4 | 2.5 | 2.7 | 1.4 | 0.0 |
| hsa-miR-0206 | 2.7 | 3.3 | 3.9 | 3.4 | 3.1 | 2.7 | 3.0 | 2.5 | 1.7 |
| hsa-miR-7157-3p | 0.0 | 0.0 | 3.0 | 2.7 | 2.8 | 1.5 | 2.7 | 1.8 | 2.9 |
| hsa-miR-6717-5p | 2.2 | 3.4 | 3.2 | 2.8 | 3.2 | 4.4 | 5.0 | 7.5 | 6.0 |
| hsa-miR-4295 | 3.4 | 2.7 | 3.1 | 3.2 | 3.4 | 2.2 | 2.9 | 3.0 | 2.7 |
| hsa-miR-1912 | 1.9 | 3.5 | 2.1 | 2.9 | 1.3 | 2.7 | 2.3 | 0.5 | 1.8 |
| hsa-miR-3141 | 8.5 | 8.6 | 8.5 | 8.9 | 9.1 | 9.2 | 7.6 | 7.6 | 7.6 |
| hsa -miR -0382-3p | 2.5 | 1.7 | 0.0 | 1.7 | 1.2 | 2.4 | 0.0 | 0.0 | 1.8 |
| hsa -miR -0499b-3p | 2.6 | 0.0 | 1.8 | 1.9 | 0.0 | 2.2 | 3.3 | 0.0 | 1.3 |
| hsa -miR -0098-5p | 0.0 | 0.0 | 0.0 | 0.0 | 0.0 | 0.0 | 0.0 | 0.0 | 0.0 |
| hsa-miR-0378a-5p | 2.5 | 3.0 | 3.3 | 2.9 | 3.7 | 3.0 | 2.1 | 1.9 | 2.1 |
| hsa-miR-4253 | 5.7 | 5.8 | 5.3 | 4.9 | 4.9 | 4.7 | 3.7 | 4.2 | 4.0 |
| hsa-miR-0550b-2-5p | 0.0 | 3.1 | 3.9 | 2.6 | 1.4 | 3.3 | 3.1 | 3.8 | 3.5 |
| hsa-miR-0025-5p | 4.4 | 5.2 | 4.3 | 4.2 | 4.0 | 4.3 | 1.9 | 3.1 | 4.8 |
| hsa-miR-4796-5p | 0.0 | 3.5 | 3.6 | 2.6 | 0.0 | 2.5 | 3.9 | 2.9 | 3.1 |
| hsa-miR-0015b-5p | 3.6 | 3.5 | 3.4 | 3.7 | 2.9 | 2.1 | 3.7 | 3.0 | 3.0 |
| hsa-miR-0378g | 1.6 | 3.2 | 3.8 | 2.7 | 0.0 | 3.0 | 3.2 | 3.0 | 1.9 |
| hsa-miR-5704 | 2.9 | 3.1 | 1.7 | 3.4 | 1.9 | 2.8 | 2.9 | 2.0 | 2.0 |
| hsa-miR-0512-3p | 3.2 | 3.3 | 3.2 | 3.0 | 2.4 | 3.3 | 2.1 | 0.0 | 1.7 |
| hsa-miR-0027b-3p | 2.7 | 3.0 | 3.1 | 2.8 | 3.2 | 0.0 | 2.2 | 1.3 | 2.0 |
| hsa-miR-0122-5p | 3.1 | 1.6 | 1.7 | 2.2 | 2.6 | 0.0 | 2.4 | 1.8 | 2.3 |
| hsa-miR-4729 | 0.0 | 0.0 | 0.0 | 0.0 | 0.0 | 0.0 | 0.0 | 0.8 | 0.0 |
| hsa-miR-0324-5p | 4.0 | 2.5 | 4.0 | 3.1 | 3.8 | 2.4 | 3.8 | 3.3 | 3.4 |
| hsa-miR-5681a | 3.8 | 3.4 | 4.3 | 3.3 | 0.0 | 1.8 | 3.6 | 3.0 | 2.4 |
| hsa-miR-4421 | 2.4 | 3.8 | 3.7 | 3.5 | 3.7 | 3.1 | 3.9 | 3.4 | 3.9 |
| hsa-miR-1299 | 0.0 | 1.4 | 0.0 | 0.0 | 0.0 | 0.0 | 0.0 | 0.0 | 0.0 |
| hsa-miR-0196b-5p | 2.1 | 2.6 | 3.9 | 3.2 | 2.3 | 3.6 | 2.8 | 2.2 | 1.2 |
| hsa-let-7c-3p | 2.6 | 3.0 | 3.1 | 2.3 | 2.0 | 2.2 | 2.7 | 1.0 | 2.1 |
| hsa-miR-0200b-3p | 0.0 | 1.6 | 2.3 | 2.2 | 2.2 | 2.6 | 1.9 | 1.1 | 1.9 |
| hsa-miR-0519c-5p, hsa-miR-523-5p, hsa-miR-518e-5p, hsa-miR-522-5p, hsa-miR-519a-5p, hsa-miR-519b-5p | 2.9 | 2.6 | 1.2 | 0.0 | 0.0 | 0.0 | 3.7 | 5.6 | 4.5 |
| hsa-miR-6839-5p | 3.0 | 2.4 | 3.1 | 3.8 | 3.4 | 3.7 | 3.6 | 4.0 | 3.2 |
| hsa-miR-6818-5p | 0.0 | 1.6 | 0.0 | 2.2 | 0.0 | 1.2 | 2.8 | 1.3 | 2.5 |
| hsa-miR-6837-3p | 3.5 | 3.9 | 4.4 | 4.3 | 3.7 | 3.7 | 2.8 | 0.0 | 2.4 |
| hsa-miR-3936 | 2.6 | 4.1 | 3.5 | 3.0 | 1.3 | 2.9 | 3.7 | 4.5 | 3.5 |
| hsa-miR-6783-5p | 0.0 | 1.6 | 2.2 | 0.0 | 0.0 | 1.7 | 2.6 | 3.0 | 2.2 |
| hsa-miR-1911-3p | 4.3 | 3.9 | 4.3 | 3.8 | 2.1 | 3.2 | 2.8 | 2.1 | 1.9 |
| hsa-miR-3140-3p | 0.0 | 0.0 | 0.0 | 0.0 | 0.0 | 0.0 | 0.0 | 0.0 | 0.0 |
| hsa-miR-6770-5p | 2.8 | 3.3 | 3.4 | 1.8 | 0.6 | 2.1 | 0.0 | 0.5 | 1.8 |
| hsa-miR-4527 | 3.4 | 2.3 | 3.6 | 3.4 | 3.2 | 2.4 | 2.1 | 1.6 | 0.0 |
| hsa-miR-8055 | 1.8 | 3.9 | 4.6 | 3.8 | 2.8 | 3.5 | 3.9 | 2.8 | 2.8 |
| hsa -miR -3678-5p | 2.2 | 2.4 | 3.3 | 1.9 | 0.0 | 1.2 | 2.2 | 0.4 | 1.8 |
| hsa-miR-4451 | 3.3 | 2.8 | 3.4 | 4.1 | 3.6 | 2.7 | 3.7 | 3.4 | 3.6 |
| hsa-miR-4783-5p | 2.6 | 2.7 | 2.3 | 0.0 | 0.0 | 1.8 | 0.0 | 2.9 | 0.0 |
| hsa-miR-0380-5p | 2.7 | 0.0 | 0.0 | 0.0 | 0.0 | 0.0 | 1.4 | 0.0 | 0.0 |
| hsa-miR-0610 | 2.1 | 2.4 | 1.8 | 3.0 | 0.6 | 2.5 | 1.6 | 3.4 | 2.3 |
| hsa-miR-4660 | 2.3 | 2.5 | 3.7 | 2.6 | 2.2 | 2.6 | 2.6 | 3.1 | 3.4 |
| hsa-miR-6715b-5p | 2.9 | 2.8 | 1.3 | 2.4 | 3.2 | 3.1 | 1.9 | 0.8 | 1.8 |
| hsa-let-7i-5p | 2.8 | 3.6 | 3.1 | 2.8 | 2.5 | 3.4 | 1.4 | 0.0 | 2.0 |
| hsa-miR-0153-5p | 2.2 | 2.5 | 3.0 | 2.4 | 0.5 | 1.6 | 2.8 | 0.9 | 2.8 |
| hsa-miR-1245a | 0.0 | 0.0 | 0.0 | 0.0 | 0.0 | 0.0 | 0.0 | 0.0 | 0.0 |
| hsa-miR-2276-5p | 2.7 | 3.8 | 4.1 | 3.3 | 3.7 | 3.2 | 2.9 | 2.8 | 3.3 |
| hsa-miR-3155b | 3.9 | 3.5 | 4.7 | 4.5 | 4.2 | 4.4 | 4.5 | 4.2 | 3.7 |
| hsa-miR-0425-5p | 0.0 | 2.8 | 3.3 | 0.0 | 0.0 | 1.5 | 1.9 | 0.0 | 0.0 |
| hsa-miR-0888-3p | 0.0 | 1.3 | 1.3 | 0.0 | 2.0 | 0.0 | 2.7 | 1.9 | 2.6 |
| hsa-miR-0369-5p | 2.3 | 2.6 | 3.2 | 3.2 | 2.3 | 2.1 | 2.1 | 1.5 | 2.9 |
| hsa-miR-0034c-5p | 2.3 | 2.1 | 2.7 | 2.7 | 0.8 | 4.5 | 2.3 | 2.0 | 2.2 |
| hsa-miR-0585-3p | 0.0 | 1.9 | 2.1 | 0.0 | 0.0 | 1.5 | 0.0 | 0.0 | 0.0 |
| hsa-miR-0378d | 2.8 | 2.9 | 2.7 | 0.0 | 0.0 | 1.2 | 1.6 | 0.0 | 2.3 |
| hsa-miR-0605-3p | 0.0 | 0.0 | 0.0 | 0.0 | 0.0 | 0.0 | 0.0 | 0.0 | 3.2 |
| hsa-miR-4491 | 0.0 | 2.4 | 3.4 | 2.0 | 0.0 | 2.8 | 1.9 | 2.0 | 0.0 |
| hsa-miR-0525-3p | 3.5 | 3.1 | 2.6 | 0.0 | 2.1 | 0.0 | 0.0 | 1.7 | 1.7 |
| hsa -miR -0548ay-3p | 2.8 | 2.5 | 2.2 | 0.0 | 2.9 | 1.6 | 0.0 | 1.2 | 0.0 |
| hsa-miR-7162-5p | 0.0 | 1.7 | 2.2 | 0.0 | 1.5 | 0.0 | 1.5 | 0.0 | 0.0 |
| hsa-miR-4800-3p | 2.3 | 3.1 | 3.7 | 2.3 | 2.9 | 2.2 | 1.6 | 0.9 | 1.6 |
| hsa-miR-1286 | 3.5 | 2.9 | 4.4 | 3.2 | 2.8 | 3.6 | 2.1 | 1.5 | 1.9 |
| hsa -miR -0942-3p | 3.9 | 3.6 | 3.9 | 3.5 | 2.5 | 2.4 | 3.1 | 2.0 | 1.2 |
| hsa-miR-4771 | 0.0 | 1.6 | 2.5 | 2.7 | 0.9 | 0.0 | 0.0 | 0.0 | 0.0 |
| hsa -miR -3682-3p | 3.7 | 3.2 | 3.4 | 4.0 | 4.5 | 5.5 | 4.1 | 4.0 | 3.5 |
| hsa-miR-8083 | 3.4 | 3.4 | 4.2 | 3.8 | 3.3 | 3.5 | 4.4 | 3.9 | 3.5 |
| hsa-miR-6513-3p | 3.6 | 3.8 | 3.5 | 3.0 | 2.3 | 2.9 | 3.1 | 2.0 | 2.9 |
| hsa-miR-3929 | 0.0 | 2.7 | 3.4 | 3.4 | 2.5 | 2.9 | 3.4 | 2.2 | 2.8 |
| hsa-miR-3200-3p | 3.6 | 3.2 | 3.5 | 3.1 | 2.9 | 3.1 | 2.8 | 1.4 | 1.9 |
| hsa-miR-4703-3p | 3.6 | 3.2 | 3.1 | 3.2 | 0.0 | 0.0 | 2.2 | 2.4 | 1.1 |
| hsa-miR-3918 | 4.3 | 4.4 | 3.8 | 3.7 | 3.3 | 4.4 | 3.4 | 3.4 | 3.5 |
| hsa-miR-0650 | 3.6 | 3.5 | 4.0 | 3.3 | 3.0 | 3.3 | 2.5 | 6.0 | 4.6 |
| hsa-miR-0034b-5p | 3.7 | 2.8 | 3.0 | 3.7 | 3.6 | 2.7 | 3.1 | 2.4 | 2.3 |
| hsa-miR-4524b-5p | 3.6 | 2.6 | 3.3 | 2.4 | 0.0 | 0.0 | 1.5 | 0.3 | 0.0 |
| hsa-miR-4644 | 2.3 | 3.7 | 3.0 | 3.7 | 3.8 | 4.4 | 3.0 | 2.9 | 3.4 |
| hsa-miR-4283 | 4.0 | 3.6 | 3.8 | 4.1 | 3.6 | 4.5 | 3.4 | 3.4 | 3.0 |
| hsa-miR-5696 | 2.0 | 3.0 | 3.2 | 2.9 | 2.0 | 3.1 | 2.2 | 1.7 | 1.7 |
| hsa-miR-0127-5p | 1.9 | 1.9 | 0.0 | 0.0 | 1.6 | 0.0 | 0.0 | 1.9 | 1.1 |
| hsa-miR-3666 | 4.7 | 4.6 | 4.7 | 3.0 | 2.9 | 3.1 | 5.0 | 2.2 | 1.3 |
| hsa-miR-4786-3p | 3.9 | 3.3 | 3.7 | 3.3 | 2.6 | 3.0 | 3.7 | 2.8 | 2.8 |
| hsa-miR-0138-2-3p | 2.7 | 2.8 | 1.6 | 3.1 | 2.9 | 1.5 | 2.2 | 2.9 | 2.8 |
| hsa-miR-1276 | 3.3 | 2.9 | 3.6 | 3.2 | 2.5 | 3.0 | 1.7 | 2.2 | 2.9 |
| hsa-miR-4709-5p | 0.0 | 3.0 | 3.4 | 2.4 | 0.7 | 2.6 | 3.0 | 2.0 | 2.9 |
| hsa-miR-4681 | 2.9 | 2.9 | 2.5 | 3.1 | 0.0 | 2.3 | 2.7 | 3.2 | 3.1 |
| hsa-miR-5094 | 2.8 | 3.4 | 3.3 | 3.7 | 2.2 | 2.9 | 2.9 | 2.8 | 1.9 |
| hsa-miR-0431-5p | 3.0 | 3.7 | 3.6 | 2.7 | 2.7 | 0.0 | 1.6 | 0.0 | 0.0 |
| hsa-miR-0383-3p | 3.9 | 4.5 | 4.5 | 3.9 | 3.2 | 3.2 | 3.4 | 1.9 | 1.9 |
| hsa-miR-0183-5p | 2.4 | 2.2 | 1.5 | 2.1 | 0.0 | 0.0 | 0.0 | 0.0 | 0.0 |
| hsa -miR -3678-3p | 3.6 | 3.6 | 3.9 | 4.0 | 3.2 | 3.6 | 2.2 | 2.2 | 2.2 |
| hsa-miR-0370-5p | 0.0 | 3.1 | 2.6 | 2.5 | 0.0 | 2.7 | 2.5 | 0.1 | 1.2 |
| hsa-miR-4755-5p | 3.3 | 2.8 | 3.4 | 2.8 | 3.1 | 3.2 | 2.1 | 1.2 | 1.8 |
| hsa-miR-3605-5p | 0.0 | 3.1 | 3.1 | 2.9 | 3.5 | 3.0 | 3.2 | 3.2 | 3.2 |
| hsa -miR -0654-5p | 3.9 | 4.0 | 3.0 | 3.4 | 2.4 | 3.5 | 3.7 | 4.7 | 4.2 |
| hsa-miR-0579-5p | 3.2 | 3.1 | 3.7 | 3.2 | 1.4 | 2.4 | 3.8 | 2.1 | 3.5 |
| hsa-miR-8080 | 2.3 | 2.7 | 3.3 | 3.1 | 2.5 | 2.9 | 3.1 | 2.5 | 2.5 |
| hsa-miR-4437 | 1.5 | 2.1 | 1.8 | 3.0 | 0.0 | 3.1 | 2.7 | 3.5 | 3.3 |
| hsa-miR-7853-5p | 3.8 | 3.8 | 4.0 | 4.0 | 3.4 | 3.4 | 2.9 | 1.5 | 3.1 |
| hsa-miR-6718-5p | 3.2 | 3.7 | 4.1 | 4.2 | 3.9 | 3.6 | 3.6 | 3.5 | 3.6 |
| hsa-miR-6501-5p | 3.5 | 3.4 | 3.6 | 3.2 | 1.9 | 4.1 | 3.2 | 4.3 | 3.7 |
| hsa-miR-7641 | 4.1 | 4.8 | 3.6 | 3.5 | 3.3 | 2.6 | 2.7 | 3.3 | 1.6 |
| hsa-miR-0495-5p | 3.4 | 3.8 | 3.9 | 4.2 | 3.8 | 3.4 | 3.7 | 3.2 | 2.7 |
| hsa-miR-0888-5p | 3.4 | 3.8 | 4.7 | 4.0 | 3.6 | 3.9 | 3.1 | 1.8 | 2.1 |
| hsa-miR-5188 | 2.6 | 3.2 | 3.3 | 2.8 | 2.2 | 2.6 | 3.3 | 2.6 | 3.2 |
| hsa-miR-3174 | 2.8 | 4.4 | 3.8 | 2.6 | 2.6 | 3.4 | 3.3 | 1.3 | 2.3 |
| hsa -miR -0548ax | 2.9 | 3.3 | 3.6 | 0.0 | 0.0 | 0.0 | 0.0 | 0.0 | 0.0 |
| hsa-miR-1284 | 3.6 | 4.0 | 3.6 | 3.4 | 0.8 | 3.5 | 3.4 | 2.4 | 3.6 |
| hsa-miR-4724-3p | 2.1 | 2.1 | 3.1 | 2.3 | 0.0 | 0.0 | 2.1 | 0.7 | 1.8 |
| hsa-miR-4275 | 0.0 | 0.0 | 2.9 | 0.0 | 0.0 | 0.0 | 0.0 | 0.0 | 1.6 |
| hsa-miR-0193b-5p | 3.9 | 3.1 | 3.6 | 5.0 | 3.1 | 4.5 | 2.7 | 4.9 | 4.1 |
| hsa -miR -5006-5p | 2.8 | 4.2 | 4.5 | 3.8 | 3.8 | 5.1 | 4.1 | 6.6 | 4.4 |
| hsa-miR-4650-5p | 1.9 | 2.8 | 3.2 | 1.5 | 2.6 | 2.4 | 2.7 | 1.0 | 2.8 |
| hsa-miR-4465 | 3.6 | 3.1 | 2.9 | 4.0 | 0.0 | 3.1 | 3.2 | 3.9 | 4.1 |
| hsa -miR -0320c | 2.2 | 1.7 | 0.0 | 2.5 | 0.0 | 2.9 | 2.8 | 2.6 | 2.6 |
| hsa-miR-0030a-3p | 3.0 | 3.6 | 3.8 | 3.3 | 0.0 | 2.3 | 2.1 | 1.9 | 2.4 |
| hsa-miR-0892a | 2.2 | 3.4 | 2.8 | 2.2 | 0.0 | 2.0 | 2.2 | 2.1 | 2.3 |
| hsa -miR -0668-5p | 3.5 | 3.4 | 3.8 | 3.6 | 4.0 | 4.2 | 4.9 | 6.1 | 5.9 |
| hsa-miR-8057 | 3.8 | 3.5 | 4.3 | 2.7 | 0.6 | 3.8 | 2.9 | 4.3 | 3.3 |
| hsa-miR-3200-5p | 4.3 | 4.7 | 4.1 | 2.3 | 0.8 | 1.8 | 0.0 | 2.1 | 2.4 |
| hsa-miR-6874-5p | 2.4 | 2.4 | 2.3 | 2.7 | 2.8 | 2.0 | 3.4 | 3.5 | 3.4 |
| hsa -miR -0338-3p | 3.8 | 3.4 | 4.4 | 3.5 | 2.9 | 2.1 | 3.2 | 2.5 | 2.4 |
| hsa-miR-0184 | 1.6 | 2.7 | 2.5 | 4.8 | 3.6 | 4.0 | 2.9 | 5.9 | 3.7 |
| hsa-miR-5096 | 2.5 | 3.7 | 0.0 | 4.2 | 6.2 | 5.2 | 4.0 | 3.6 | 5.3 |
| hsa-miR-7848-3p | 3.1 | 3.8 | 4.8 | 3.3 | 4.7 | 3.9 | 3.7 | 2.3 | 2.6 |
| hsa-miR-6081 | 4.3 | 4.7 | 3.1 | 3.3 | 2.8 | 3.4 | 2.4 | 2.9 | 2.3 |
| hsa-miR-0196b-3p | 5.4 | 4.8 | 3.5 | 3.3 | 1.6 | 2.5 | 3.2 | 2.4 | 2.2 |
| hsa-miR-0147a | 3.4 | 2.6 | 3.8 | 3.5 | 3.0 | 2.5 | 2.8 | 3.4 | 2.1 |
| hsa-let-7b-5p | 3.6 | 3.6 | 3.7 | 4.1 | 3.8 | 3.1 | 3.2 | 3.1 | 0.0 |
| hsa-miR-0143-3p | 3.3 | 2.9 | 2.3 | 2.7 | 3.6 | 0.0 | 1.6 | 0.0 | 0.0 |
| hsa-miR-1273e | 3.6 | 3.1 | 1.2 | 4.2 | 5.0 | 3.9 | 3.1 | 3.1 | 4.7 |
| hsa-miR-4712-5p | 3.6 | 4.0 | 3.8 | 3.5 | 2.9 | 4.0 | 3.7 | 2.8 | 3.5 |
| hsa-miR-5011-5p | 0.0 | 0.0 | 1.6 | 0.0 | 0.0 | 0.0 | 0.0 | 0.0 | 0.0 |
| hsa-miR-0514b-3p | 4.0 | 3.2 | 3.2 | 2.4 | 0.0 | 2.0 | 1.6 | 1.4 | 1.1 |
| hsa-miR-0325 | 0.0 | 3.2 | 2.9 | 3.0 | 1.4 | 1.9 | 2.9 | 2.4 | 3.0 |
| hsa-miR-4482-5p | 0.0 | 3.2 | 3.4 | 2.8 | 0.5 | 1.6 | 3.5 | 1.8 | 2.1 |
| hsa-miR-4303 | 2.9 | 3.1 | 3.7 | 3.8 | 3.7 | 2.7 | 3.5 | 3.6 | 3.5 |
| hsa-miR-5680 | 3.5 | 2.8 | 3.4 | 2.5 | 0.5 | 0.0 | 2.1 | 1.7 | 0.0 |
| hsa-miR-0664b-5p | 0.0 | 0.0 | 3.0 | 2.5 | 1.9 | 2.0 | 2.8 | 1.1 | 0.0 |
| hsa-miR-6853-5p | 3.0 | 3.7 | 4.4 | 3.3 | 3.3 | 3.5 | 4.1 | 3.7 | 4.0 |
| hsa-miR-4752 | 0.0 | 2.8 | 4.3 | 2.2 | 2.4 | 2.7 | 3.4 | 2.2 | 3.2 |
| hsa -miR -5002-5p | 0.0 | 1.5 | 2.5 | 1.8 | 0.0 | 1.5 | 2.8 | 0.9 | 2.2 |
| hsa-miR-4524b-3p | 3.2 | 3.2 | 4.1 | 4.4 | 0.0 | 2.9 | 3.6 | 3.0 | 3.1 |
| hsa-miR-0589-3p | 3.2 | 3.4 | 3.4 | 3.2 | 1.2 | 2.9 | 3.3 | 2.6 | 3.1 |
| hsa-miR-1271-5p | 3.1 | 3.5 | 3.6 | 3.3 | 1.6 | 3.5 | 1.9 | 2.5 | 3.0 |
| hsa-miR-6509-5p | 1.1 | 3.4 | 3.6 | 2.1 | 2.9 | 2.8 | 1.9 | 2.4 | 2.8 |
| hsa -miR -4638-3p | 4.4 | 4.0 | 3.5 | 2.3 | 2.8 | 0.0 | 1.4 | 2.3 | 1.1 |
| hsa-miR-5689 | 0.0 | 1.8 | 1.9 | 2.4 | 0.0 | 2.2 | 3.1 | 0.9 | 2.7 |
| hsa-miR-8084 | 2.2 | 4.8 | 0.0 | 0.0 | 0.0 | 0.0 | 0.0 | 0.0 | 0.0 |
| hsa-miR-1295b-3p | 3.3 | 3.5 | 4.0 | 3.4 | 0.0 | 3.5 | 3.1 | 2.2 | 1.9 |
| hsa-miR-7154-3p | 0.0 | 2.4 | 3.2 | 2.2 | 2.9 | 2.8 | 2.8 | 2.2 | 3.0 |
| hsa-miR-5095 | 2.5 | 2.5 | 3.1 | 2.6 | 2.2 | 3.3 | 2.6 | 2.7 | 4.0 |
| hsa-miR-4273 | 3.5 | 2.8 | 3.6 | 3.4 | 2.5 | 3.3 | 3.1 | 2.7 | 2.4 |
| hsa-miR-0145-5p | 2.3 | 3.2 | 2.7 | 2.9 | 3.6 | 1.3 | 0.0 | 0.9 | 4.0 |
| hsa-miR-0142-3p | 1.8 | 2.7 | 2.6 | 3.4 | 3.2 | 1.4 | 2.7 | 1.0 | 2.9 |
| hsa-miR-4657 | 1.1 | 2.8 | 4.1 | 2.7 | 1.7 | 2.7 | 3.9 | 5.1 | 3.9 |
| hsa-miR-0519a-3p | 2.4 | 2.6 | 1.4 | 2.9 | 0.0 | 0.0 | 0.0 | 0.0 | 0.0 |
| hsa-miR-3689f | 0.0 | 2.4 | 0.0 | 1.8 | 0.0 | 1.4 | 2.1 | 2.0 | 0.0 |
| hsa-miR-5571-3p | 0.0 | 2.9 | 3.9 | 3.1 | 2.5 | 2.8 | 3.9 | 4.2 | 4.2 |
| hsa-miR-0566 | 4.3 | 4.1 | 3.5 | 4.8 | 2.7 | 3.9 | 2.4 | 2.5 | 3.3 |
| hsa-miR-0138-5p | 3.9 | 3.2 | 3.2 | 4.0 | 3.1 | 4.8 | 3.0 | 2.8 | 3.3 |
| hsa-miR-0526b-3p | 4.2 | 4.3 | 4.9 | 4.1 | 3.2 | 2.0 | 2.6 | 1.3 | 0.0 |
| hsa-miR-4717-5p | 3.1 | 3.8 | 3.9 | 3.6 | 2.5 | 3.2 | 3.0 | 2.5 | 2.3 |
| hsa-miR-5591-3p | 2.4 | 3.3 | 2.8 | 3.2 | 1.6 | 2.4 | 3.4 | 2.1 | 2.6 |
| hsa-miR-4254 | 3.4 | 4.1 | 3.9 | 3.7 | 2.9 | 3.8 | 2.5 | 2.1 | 2.9 |
| hsa -miR -3682-5p | 2.3 | 1.4 | 2.9 | 2.0 | 2.3 | 0.0 | 3.1 | 1.1 | 1.7 |
| hsa -miR -6506-5p | 2.3 | 3.6 | 4.0 | 3.2 | 2.0 | 2.3 | 2.8 | 2.6 | 2.1 |
| hsa-miR-8082 | 3.6 | 3.1 | 3.6 | 2.8 | 3.1 | 3.1 | 3.9 | 4.0 | 3.9 |
| hsa-miR-7974 | 3.6 | 3.7 | 4.0 | 3.4 | 1.9 | 3.4 | 3.3 | 2.4 | 3.8 |
| hsa-miR-4999-5p | 1.4 | 1.8 | 2.3 | 2.9 | 1.8 | 2.5 | 3.3 | 3.0 | 3.0 |
| hsa-miR-4797-3p | 1.4 | 2.5 | 4.1 | 2.7 | 0.0 | 1.4 | 2.9 | 1.8 | 1.7 |
| hsa-miR-4264 | 0.0 | 1.6 | 0.0 | 0.0 | 1.3 | 2.0 | 2.1 | 0.0 | 2.8 |
| hsa-miR-0378b | 2.7 | 3.5 | 3.8 | 3.5 | 3.1 | 3.9 | 2.9 | 2.1 | 3.0 |
| hsa-miR-0323a-5p | 4.2 | 4.2 | 3.6 | 3.7 | 3.1 | 3.7 | 3.1 | 3.9 | 3.0 |
| hsa-miR-3713 | 3.2 | 4.4 | 3.5 | 3.8 | 3.4 | 4.8 | 3.5 | 3.3 | 3.3 |
| hsa-miR-4308 | 3.0 | 3.2 | 2.6 | 3.0 | 4.2 | 2.9 | 2.2 | 2.6 | 1.5 |
| hsa -miR -0942-5p | 0.0 | 1.7 | 2.3 | 2.3 | 0.0 | 1.6 | 2.0 | 0.0 | 1.7 |
| hsa-miR-4450 | 2.5 | 3.3 | 3.5 | 4.0 | 3.8 | 4.5 | 3.6 | 3.4 | 3.9 |
| hsa-miR-0146a-5p | 3.5 | 3.2 | 3.7 | 3.1 | 0.0 | 3.1 | 2.7 | 1.1 | 2.2 |
| hsa-miR-0329-5p | 0.0 | 0.0 | 2.6 | 0.0 | 0.4 | 3.0 | 3.2 | 2.5 | 2.7 |
| hsa-miR-1178-3p | 3.4 | 4.2 | 3.3 | 3.1 | 2.0 | 2.7 | 3.1 | 2.6 | 2.9 |
| hsa -miR -3922-5p | 3.6 | 4.0 | 4.2 | 3.6 | 3.6 | 3.9 | 4.1 | 4.2 | 3.9 |
| hsa-miR-6811-3p | 3.5 | 4.1 | 4.2 | 3.3 | 1.7 | 3.4 | 3.4 | 2.0 | 2.7 |
| hsa-miR-6781-3p | 3.5 | 4.1 | 4.2 | 3.5 | 3.3 | 3.6 | 2.5 | 2.2 | 2.8 |
| hsa-miR-3651 | 3.6 | 4.3 | 3.2 | 3.2 | 2.8 | 2.8 | 3.7 | 4.4 | 4.0 |
| hsa-miR-0021-3p | 3.1 | 3.4 | 3.9 | 3.2 | 2.7 | 2.0 | 2.4 | 2.4 | 2.9 |
| hsa-miR-6817-5p | 2.9 | 3.2 | 2.5 | 3.9 | 3.4 | 3.3 | 3.7 | 3.6 | 3.4 |
| hsa-miR-0195-3p | 3.9 | 3.1 | 3.6 | 3.2 | 3.6 | 3.2 | 2.8 | 1.7 | 1.1 |
| hsa-miR-3913-3p | 3.0 | 2.2 | 2.7 | 2.8 | 0.0 | 2.3 | 2.8 | 0.9 | 2.1 |
| hsa-miR-0339-5p | 4.3 | 4.2 | 4.7 | 4.1 | 3.6 | 3.3 | 3.1 | 2.2 | 1.7 |
| hsa-miR-4718 | 3.7 | 4.4 | 4.2 | 3.4 | 2.9 | 3.1 | 3.0 | 2.1 | 2.1 |
| hsa-miR-0584-5p | 4.4 | 4.0 | 4.0 | 4.2 | 2.9 | 3.8 | 3.3 | 4.5 | 3.6 |
| hsa-miR-0345-5p | 3.1 | 3.2 | 3.3 | 3.8 | 3.2 | 3.8 | 3.2 | 1.9 | 2.8 |
| hsa-miR-7155-3p | 4.5 | 4.7 | 4.6 | 4.1 | 3.5 | 4.2 | 3.5 | 3.2 | 3.4 |
| hsa-miR-6505-3p | 4.1 | 4.5 | 4.6 | 4.4 | 4.5 | 4.0 | 4.0 | 3.6 | 3.8 |
| hsa-miR-1251-3p | 3.0 | 3.8 | 3.9 | 3.2 | 3.2 | 3.6 | 3.3 | 2.7 | 3.1 |
| hsa-miR-3690 | 3.7 | 3.7 | 3.7 | 3.5 | 1.1 | 3.0 | 3.2 | 1.8 | 2.0 |
| hsa-miR-5000-5p | 2.1 | 2.2 | 3.2 | 2.9 | 1.2 | 2.5 | 3.4 | 2.6 | 2.9 |
| hsa-miR-0596 | 4.0 | 3.7 | 3.4 | 1.7 | 0.0 | 0.0 | 3.0 | 3.5 | 3.3 |
| hsa-miR-0591 | 2.3 | 3.6 | 4.3 | 4.1 | 3.5 | 4.0 | 4.0 | 2.8 | 2.6 |
| hsa-miR-4658 | 3.4 | 3.4 | 4.2 | 2.9 | 3.3 | 3.6 | 2.9 | 3.8 | 3.5 |
| hsa-miR-3680-3p | 3.1 | 3.9 | 0.0 | 3.1 | 1.5 | 3.3 | 3.1 | 2.8 | 2.9 |
| hsa-miR-0379-3p | 2.9 | 3.7 | 2.9 | 2.9 | 0.0 | 2.3 | 2.9 | 1.4 | 1.9 |
| hsa-miR-0020a-3p | 3.2 | 3.1 | 4.2 | 3.1 | 2.9 | 0.0 | 1.8 | 0.0 | 3.0 |
| hsa-miR-5093 | 3.6 | 2.5 | 3.6 | 3.9 | 3.8 | 3.6 | 4.2 | 4.1 | 4.0 |
| hsa -miR -0524-5p | 0.0 | 2.3 | 3.4 | 2.4 | 2.8 | 0.0 | 1.9 | 2.0 | 3.2 |
| hsa-miR-4733-5p | 3.7 | 3.6 | 3.3 | 3.6 | 2.2 | 2.7 | 2.8 | 1.2 | 2.6 |
| hsa-miR-0619-5p | 4.3 | 4.0 | 3.0 | 5.4 | 8.0 | 7.4 | 4.7 | 4.5 | 6.3 |
| hsa-miR-0214-5p | 2.0 | 3.8 | 2.7 | 3.8 | 2.3 | 3.0 | 3.2 | 2.9 | 3.7 |
| hsa-miR-0423-3p | 3.3 | 3.3 | 3.5 | 3.3 | 2.6 | 2.8 | 3.6 | 2.3 | 2.8 |
| hsa -miR -6838-5p | 2.6 | 3.1 | 3.4 | 2.7 | 1.7 | 3.0 | 2.6 | 2.8 | 2.4 |
| hsa -miR -3186-5p | 2.5 | 3.9 | 4.0 | 3.4 | 3.2 | 3.3 | 3.8 | 3.3 | 3.7 |
| hsa-miR-6083 | 3.4 | 4.2 | 4.1 | 3.8 | 1.4 | 4.1 | 3.2 | 2.9 | 3.1 |
| hsa-miR-6761-5p | 3.8 | 3.7 | 4.1 | 4.0 | 4.0 | 3.3 | 3.8 | 3.7 | 3.4 |
| hsa-miR-0103a-2-5p | 1.9 | 3.1 | 2.6 | 3.3 | 2.8 | 2.8 | 3.5 | 2.9 | 3.2 |
| hsa-miR-6895-5p | 3.5 | 4.4 | 4.9 | 3.8 | 3.7 | 4.3 | 3.7 | 4.0 | 4.2 |
| hsa-miR-4645-3p | 3.7 | 3.6 | 3.8 | 3.5 | 3.5 | 3.2 | 3.6 | 2.7 | 2.5 |
| hsa-miR-0300 | 2.6 | 3.9 | 3.7 | 2.8 | 2.5 | 4.5 | 3.5 | 2.7 | 3.1 |
| hsa-miR-5089-3p | 0.0 | 2.6 | 3.9 | 3.2 | 3.4 | 3.5 | 3.3 | 2.7 | 2.9 |
| hsa-miR-4740-3p | 3.5 | 4.6 | 4.5 | 3.8 | 4.5 | 4.6 | 3.7 | 3.6 | 3.7 |
| hsa-miR-0511-5p | 2.9 | 3.4 | 3.3 | 2.5 | 2.2 | 2.1 | 2.5 | 0.5 | 2.3 |
| hsa-miR-6514-5p | 3.0 | 2.6 | 3.4 | 2.7 | 2.6 | 3.2 | 3.4 | 4.6 | 3.2 |
| hsa-miR-1288-5p | 3.1 | 3.4 | 2.6 | 2.3 | 0.0 | 2.8 | 2.8 | 1.4 | 1.5 |
| hsa -miR -0454-5p | 3.0 | 3.3 | 3.3 | 3.0 | 2.1 | 2.6 | 3.2 | 2.7 | 2.6 |
| hsa-miR-0645 | 3.1 | 3.5 | 3.5 | 3.2 | 3.2 | 3.4 | 3.3 | 1.5 | 2.6 |
| hsa-miR-3190-3p | 3.7 | 4.6 | 4.4 | 4.7 | 4.0 | 5.0 | 3.2 | 3.6 | 3.8 |
| hsa-miR-4438 | 0.0 | 2.7 | 3.3 | 3.4 | 2.3 | 2.7 | 2.6 | 2.0 | 2.1 |
| hsa-miR-0519d-5p | 1.7 | 0.0 | 2.3 | 0.0 | 0.0 | 0.0 | 0.0 | 2.1 | 1.8 |
| hsa-miR-1199-3p | 4.5 | 3.5 | 3.9 | 3.4 | 3.8 | 3.6 | 3.2 | 3.7 | 3.5 |
| hsa-miR-0330-3p | 1.9 | 3.1 | 3.6 | 3.8 | 2.4 | 2.8 | 2.8 | 2.1 | 2.6 |
| hsa -miR -0030c-5p | 3.4 | 2.9 | 3.9 | 3.3 | 2.6 | 2.0 | 2.6 | 1.3 | 1.6 |
| hsa-miR-7973 | 3.1 | 3.1 | 3.7 | 2.2 | 3.2 | 3.0 | 3.1 | 2.6 | 2.2 |
| hsa-miR-0509-3p | 3.7 | 3.8 | 4.5 | 4.7 | 4.3 | 3.6 | 3.4 | 3.5 | 3.1 |
| hsa-miR-1298-3p | 3.5 | 3.9 | 4.0 | 3.9 | 3.0 | 3.3 | 2.9 | 3.5 | 3.4 |
| hsa-miR-3667-5p | 3.0 | 3.7 | 4.1 | 3.7 | 4.8 | 4.4 | 3.9 | 3.4 | 4.1 |
| hsa-miR-0519b-3p | 3.6 | 2.9 | 2.7 | 3.0 | 0.0 | 1.2 | 0.0 | 0.0 | 0.0 |
| hsa-miR-6499-5p | 4.4 | 5.9 | 3.9 | 3.6 | 3.1 | 3.4 | 3.3 | 2.1 | 2.6 |
| hsa-miR-6850-3p | 4.4 | 4.2 | 3.3 | 2.7 | 2.9 | 3.1 | 3.2 | 2.0 | 1.5 |
| hsa-miR-5194 | 3.0 | 2.5 | 3.2 | 2.6 | 1.9 | 3.5 | 4.0 | 2.0 | 3.4 |
| hsa-miR-7849-3p | 3.4 | 3.8 | 4.1 | 3.6 | 2.6 | 2.7 | 0.0 | 1.1 | 0.0 |
| hsa-miR-0410-5p | 2.3 | 3.0 | 3.2 | 1.9 | 2.3 | 3.1 | 3.2 | 2.6 | 2.7 |
| hsa-miR-3654 | 3.2 | 3.2 | 2.9 | 2.3 | 0.0 | 2.7 | 1.4 | 3.9 | 2.7 |
| hsa-miR-4446-5p | 2.4 | 2.9 | 3.6 | 2.5 | 2.9 | 2.5 | 3.0 | 2.1 | 2.9 |
| hsa-miR-4740-5p | 3.8 | 4.4 | 4.3 | 4.3 | 2.8 | 4.0 | 2.7 | 2.5 | 3.9 |
| hsa-miR-6503-3p | 3.7 | 3.9 | 4.6 | 3.6 | 4.1 | 4.1 | 4.9 | 3.9 | 4.8 |
| hsa-miR-0106b-3p | 4.2 | 4.5 | 4.3 | 4.2 | 2.9 | 3.5 | 2.9 | 2.6 | 1.9 |
| hsa-miR-3657 | 3.2 | 4.0 | 4.4 | 3.5 | 3.7 | 3.4 | 3.3 | 2.9 | 2.7 |
| hsa-miR-5192 | 2.5 | 2.8 | 3.9 | 3.2 | 2.5 | 3.7 | 3.6 | 2.4 | 2.5 |
| hsa-miR-6818-3p | 3.3 | 3.9 | 4.0 | 3.8 | 3.3 | 3.7 | 4.0 | 3.1 | 3.5 |
| hsa-miR-1915-5p | 4.7 | 5.1 | 4.3 | 3.4 | 3.4 | 3.8 | 3.0 | 3.8 | 2.0 |
| hsa-miR-3145-3p | 0.0 | 0.0 | 0.0 | 0.0 | 0.0 | 0.0 | 0.0 | 0.0 | 0.0 |
| hsa-miR-4684-3p | 3.4 | 4.6 | 4.7 | 4.2 | 3.8 | 3.6 | 3.7 | 4.6 | 3.8 |
| hsa-miR-5694 | 2.5 | 3.4 | 4.0 | 3.4 | 2.2 | 3.4 | 4.2 | 3.4 | 3.8 |
| hsa -miR -0654-3p | 2.0 | 3.1 | 4.6 | 3.6 | 3.0 | 3.4 | 4.0 | 2.8 | 3.5 |
| hsa-miR-4773 | 3.0 | 4.0 | 3.8 | 3.1 | 2.5 | 3.2 | 2.1 | 1.1 | 0.0 |
| hsa-miR-7161-5p | 3.2 | 3.6 | 3.3 | 2.6 | 0.0 | 2.0 | 1.9 | 1.7 | 0.0 |
| hsa-miR-5197-5p | 3.6 | 3.5 | 3.7 | 3.9 | 3.2 | 4.1 | 2.5 | 2.7 | 2.4 |
| hsa-miR-5703 | 3.6 | 3.8 | 4.0 | 3.9 | 3.8 | 4.4 | 4.0 | 4.1 | 3.9 |
| hsa-miR-0141-5p | 4.3 | 3.7 | 5.0 | 4.3 | 2.4 | 3.6 | 3.1 | 2.2 | 2.4 |
| hsa -miR -5008-3p | 3.6 | 4.2 | 3.6 | 2.4 | 1.9 | 2.3 | 2.2 | 1.9 | 2.9 |
| hsa-miR-5681b | 3.8 | 4.4 | 4.1 | 3.9 | 3.7 | 3.3 | 1.9 | 3.6 | 2.7 |
| hsa -miR -0200c-5p | 0.0 | 3.1 | 3.2 | 3.2 | 1.2 | 2.2 | 2.6 | 2.6 | 3.2 |
| hsa-miR-0125b-2-3p | 2.1 | 0.0 | 2.3 | 2.7 | 0.0 | 1.9 | 2.3 | 2.2 | 2.0 |
| hsa-miR-4754 | 2.6 | 3.3 | 3.6 | 4.3 | 4.0 | 4.1 | 3.6 | 4.3 | 4.4 |
| hsa-miR-1322 | 3.2 | 2.9 | 2.6 | 4.1 | 1.5 | 3.0 | 3.1 | 2.3 | 3.5 |
| hsa -miR -3124-3p | 0.0 | 0.0 | 0.0 | 0.0 | 0.0 | 0.0 | 0.0 | 0.0 | 0.0 |
| hsa-miR-0381-5p | 1.8 | 3.4 | 3.4 | 3.1 | 2.8 | 2.7 | 2.6 | 3.2 | 2.9 |
| hsa-miR-5699-3p | 0.0 | 3.3 | 3.3 | 0.0 | 0.0 | 2.5 | 2.6 | 1.8 | 0.0 |
| hsa -miR -5584-3p | 3.6 | 4.4 | 3.7 | 3.8 | 3.9 | 4.0 | 3.4 | 3.5 | 3.4 |
| hsa-miR-5190 | 4.5 | 4.3 | 4.4 | 3.9 | 4.0 | 4.2 | 3.4 | 4.7 | 3.9 |
| hsa -miR -0892c-5p | 2.0 | 2.4 | 2.6 | 2.5 | 1.5 | 2.7 | 1.5 | 2.0 | 2.1 |
| hsa-miR-4445-3p | 1.9 | 2.8 | 3.6 | 3.1 | 3.4 | 2.8 | 2.7 | 1.5 | 2.1 |
| hsa -miR -5004-5p | 2.9 | 4.2 | 4.3 | 3.8 | 3.5 | 3.8 | 3.7 | 3.9 | 3.7 |
| hsa-miR-0889-5p | 3.1 | 3.7 | 3.9 | 4.1 | 4.2 | 3.7 | 3.3 | 3.9 | 3.6 |
| hsa -miR -0338-5p | 0.0 | 2.3 | 3.6 | 3.1 | 0.0 | 2.1 | 2.9 | 2.5 | 3.1 |
| hsa-miR-0488-5p | 3.6 | 4.2 | 4.3 | 4.3 | 3.7 | 4.1 | 3.8 | 2.4 | 3.4 |
| hsa-miR-4653-5p | 3.7 | 3.8 | 4.1 | 3.4 | 3.5 | 3.3 | 2.7 | 2.9 | 3.0 |
| hsa-miR-0016-1-3p | 4.2 | 3.0 | 3.6 | 3.1 | 3.6 | 2.6 | 2.9 | 2.2 | 2.9 |
| hsa-miR-4310 | 4.3 | 4.0 | 4.1 | 4.2 | 2.9 | 3.3 | 3.9 | 3.1 | 3.4 |
| hsa-miR-4316 | 1.7 | 4.2 | 4.5 | 4.5 | 3.8 | 4.4 | 4.5 | 4.9 | 4.5 |
| hsa-miR-1288-3p | 3.5 | 3.5 | 3.3 | 2.7 | 0.0 | 3.5 | 3.1 | 2.8 | 3.3 |
| hsa -miR -6854-3p | 3.8 | 4.4 | 4.5 | 3.6 | 3.7 | 3.3 | 3.7 | 3.4 | 3.3 |
| hsa-miR-0221-3p | 2.9 | 2.7 | 4.1 | 2.9 | 1.0 | 2.3 | 2.3 | 2.6 | 2.1 |
| hsa-miR-4299 | 3.1 | 3.6 | 3.6 | 3.6 | 2.5 | 3.6 | 3.1 | 2.9 | 2.8 |
| hsa-miR-7703 | 0.0 | 3.7 | 4.2 | 3.2 | 4.3 | 3.9 | 4.0 | 2.8 | 3.6 |
| hsa-miR-4520-5p | 2.8 | 3.8 | 4.0 | 4.5 | 3.6 | 3.9 | 3.7 | 4.0 | 4.1 |
| hsa-miR-0212-5p | 3.7 | 3.4 | 4.2 | 3.7 | 3.5 | 3.4 | 3.9 | 3.4 | 3.4 |
| hsa-miR-0025-3p | 4.0 | 4.6 | 4.3 | 3.8 | 3.1 | 3.3 | 3.2 | 2.6 | 3.0 |
| hsa -miR -0708-5p | 0.0 | 1.6 | 2.3 | 0.0 | 0.8 | 0.0 | 5.0 | 2.8 | 4.4 |
| hsa-miR-6821-3p | 3.8 | 4.6 | 4.4 | 4.0 | 3.4 | 4.2 | 3.3 | 2.1 | 2.9 |
| hsa-miR-0146b-3p | 4.3 | 3.7 | 4.2 | 4.0 | 3.6 | 3.9 | 4.0 | 3.2 | 3.6 |
| hsa-miR-4514 | 3.1 | 3.3 | 3.3 | 3.9 | 3.3 | 2.7 | 3.9 | 3.8 | 3.2 |
| hsa-miR-4429 | 2.3 | 3.8 | 3.6 | 3.0 | 2.3 | 3.5 | 4.1 | 4.2 | 4.3 |
| hsa-miR-0216a-3p | 4.7 | 4.6 | 4.5 | 4.4 | 4.1 | 3.5 | 3.4 | 4.0 | 3.8 |
| hsa -miR -0432-5p | 3.4 | 3.7 | 4.5 | 3.9 | 4.0 | 4.2 | 3.8 | 3.6 | 3.4 |
| hsa-miR-0525-5p | 4.3 | 3.6 | 3.3 | 3.3 | 3.9 | 3.3 | 2.8 | 4.4 | 3.8 |
| hsa-miR-1250-5p | 3.8 | 3.6 | 3.6 | 3.7 | 2.4 | 3.7 | 3.1 | 4.2 | 3.3 |
| hsa-miR-5580-3p | 3.9 | 3.9 | 3.6 | 3.7 | 2.1 | 3.0 | 3.4 | 3.0 | 3.5 |
| hsa-miR-0622 | 3.5 | 3.4 | 4.6 | 4.3 | 3.5 | 3.6 | 4.6 | 4.4 | 4.1 |
| hsa-miR-0875-3p | 2.8 | 3.1 | 3.8 | 3.2 | 1.5 | 3.6 | 3.7 | 2.5 | 2.0 |
| hsa-miR-0099b-5p | 4.5 | 4.6 | 4.7 | 4.3 | 4.0 | 3.6 | 2.8 | 2.4 | 2.4 |
| hsa-miR-0660-3p | 3.9 | 3.4 | 4.5 | 4.2 | 3.6 | 3.5 | 3.9 | 3.1 | 3.7 |
| hsa -miR -0520h | 3.4 | 4.2 | 4.4 | 3.0 | 3.3 | 2.2 | 2.3 | 2.8 | 2.4 |
| hsa-miR-8062 | 2.3 | 3.0 | 2.8 | 2.9 | 3.5 | 2.7 | 4.7 | 3.6 | 4.1 |
| hsa-miR-6765-3p | 3.9 | 4.1 | 3.9 | 2.6 | 3.5 | 3.4 | 2.3 | 2.8 | 3.1 |
| hsa -miR -0508-5p | 4.3 | 4.5 | 3.8 | 3.6 | 2.7 | 4.3 | 2.4 | 3.1 | 3.0 |
| hsa-miR-0501-5p | 3.4 | 4.0 | 3.5 | 4.0 | 1.8 | 3.1 | 3.3 | 3.4 | 3.3 |
| hsa -miR -6868-5p | 3.3 | 2.9 | 3.8 | 3.6 | 2.8 | 3.0 | 4.3 | 3.6 | 3.9 |
| hsa-miR-5187-3p | 3.4 | 4.0 | 4.3 | 3.9 | 3.3 | 3.9 | 4.5 | 3.0 | 4.2 |
| hsa-miR-3074-5p | 4.8 | 5.6 | 3.8 | 3.7 | 3.6 | 3.4 | 3.4 | 2.4 | 2.1 |
| hsa-miR-3170 | 3.0 | 3.3 | 4.2 | 3.7 | 3.4 | 2.8 | 3.1 | 2.8 | 3.2 |
| hsa -miR -0224-3p | 4.4 | 4.2 | 4.0 | 2.9 | 2.6 | 2.7 | 2.6 | 0.6 | 1.7 |
| hsa-miR-2116-5p | 3.5 | 3.0 | 3.5 | 2.9 | 4.6 | 3.6 | 3.8 | 3.8 | 4.4 |
| hsa-miR-3153 | 4.7 | 5.5 | 5.3 | 5.1 | 5.2 | 5.3 | 4.8 | 5.8 | 5.1 |
| hsa-miR-6728-5p | 3.7 | 4.1 | 4.4 | 4.0 | 4.0 | 3.8 | 4.5 | 4.2 | 4.2 |
| hsa-miR-4328 | 2.9 | 4.1 | 3.9 | 4.3 | 2.5 | 3.2 | 3.2 | 2.0 | 3.4 |
| hsa -miR -6804-5p | 3.0 | 3.3 | 3.4 | 3.5 | 3.2 | 4.2 | 3.9 | 4.8 | 4.0 |
| hsa -miR -0204-5p | 3.9 | 4.1 | 4.1 | 3.9 | 4.3 | 3.8 | 3.5 | 3.1 | 3.8 |
| hsa-miR-4764-3p | 3.3 | 3.5 | 3.2 | 3.1 | 2.8 | 3.0 | 3.0 | 2.7 | 2.4 |
| hsa-miR-7854-3p | 3.0 | 3.1 | 3.5 | 4.4 | 2.5 | 4.9 | 2.8 | 4.8 | 4.3 |
| hsa -miR -0382-5p | 2.7 | 2.5 | 3.7 | 3.7 | 3.1 | 2.4 | 3.2 | 3.5 | 3.1 |
| hsa-miR-3615 | 4.2 | 4.8 | 4.3 | 3.1 | 3.2 | 4.1 | 3.4 | 2.4 | 3.1 |
| hsa-miR-5585-3p | 4.0 | 4.5 | 4.5 | 4.6 | 4.4 | 4.2 | 3.8 | 3.9 | 4.3 |
| hsa -miR -0422a | 4.0 | 4.3 | 4.4 | 3.7 | 2.3 | 2.9 | 1.8 | 2.3 | 2.0 |
| hsa-miR-6871-3p | 3.7 | 4.2 | 4.1 | 3.6 | 4.0 | 3.6 | 3.0 | 2.7 | 3.2 |
| hsa-miR-4756-3p | 3.3 | 3.8 | 4.2 | 3.9 | 4.1 | 3.4 | 4.0 | 3.9 | 4.0 |
| hsa-miR-0485-5p | 5.0 | 4.6 | 4.7 | 4.5 | 4.2 | 3.8 | 3.6 | 3.9 | 3.7 |
| hsa-miR-0921 | 3.5 | 1.7 | 2.6 | 3.5 | 2.3 | 2.9 | 3.5 | 5.7 | 4.4 |
| hsa-miR-0340-3p | 3.7 | 3.4 | 3.7 | 3.5 | 3.9 | 3.3 | 2.7 | 2.9 | 2.9 |
| hsa-miR-3529-5p | 4.3 | 3.4 | 3.7 | 2.5 | 1.7 | 3.2 | 2.6 | 2.8 | 1.9 |
| hsa-miR-0758-5p | 2.0 | 3.1 | 3.9 | 3.7 | 2.9 | 3.2 | 3.4 | 3.4 | 3.5 |
| hsa-miR-3681-3p | 0.0 | 3.6 | 3.8 | 2.4 | 2.0 | 2.9 | 3.0 | 2.4 | 3.5 |
| hsa-miR-3074-3p | 3.9 | 3.9 | 3.6 | 3.5 | 1.1 | 3.4 | 3.2 | 2.5 | 2.3 |
| hsa-miR-3169 | 0.0 | 1.8 | 2.6 | 0.0 | 0.0 | 0.0 | 0.0 | 0.0 | 0.0 |
| hsa-miR-0030b-5p | 3.5 | 4.0 | 4.2 | 4.2 | 4.0 | 2.9 | 3.1 | 2.8 | 3.3 |
| hsa -miR -0506-3p | 4.6 | 4.3 | 4.4 | 3.9 | 3.9 | 3.7 | 3.3 | 3.7 | 3.1 |
| hsa-miR-2117 | 3.6 | 3.4 | 2.3 | 2.1 | 0.0 | 0.0 | 0.0 | 1.3 | 1.3 |
| hsa-miR-3154 | 8.1 | 8.1 | 8.8 | 8.6 | 8.9 | 8.6 | 9.0 | 8.6 | 8.8 |
| hsa-miR-0943 | 3.3 | 4.5 | 3.9 | 3.8 | 3.6 | 3.7 | 3.2 | 3.1 | 2.9 |
| hsa-miR-0603 | 3.8 | 3.3 | 3.7 | 4.0 | 4.1 | 3.4 | 3.6 | 3.5 | 3.4 |
| hsa-miR-4784 | 3.8 | 3.3 | 3.6 | 4.2 | 4.1 | 3.4 | 3.5 | 4.8 | 4.4 |
| hsa -miR -0296-3p | 5.1 | 4.4 | 4.5 | 5.4 | 5.8 | 5.2 | 5.3 | 6.1 | 5.9 |
| hsa-miR-4529-5p | 2.6 | 2.6 | 3.8 | 3.9 | 2.8 | 3.4 | 3.9 | 3.5 | 3.9 |
| hsa-miR-8081 | 3.8 | 3.6 | 3.4 | 3.7 | 3.8 | 3.3 | 2.7 | 3.5 | 3.4 |
| hsa-miR-0034b-3p | 3.1 | 3.5 | 3.5 | 2.9 | 3.0 | 3.3 | 2.4 | 2.6 | 2.7 |
| hsa-miR-4768-3p | 0.0 | 2.4 | 2.2 | 3.1 | 2.5 | 2.3 | 3.2 | 1.6 | 2.9 |
| hsa-miR-6134 | 4.1 | 5.3 | 3.9 | 4.1 | 3.5 | 4.3 | 4.0 | 2.9 | 3.5 |
| hsa-miR-0558 | 2.8 | 2.9 | 3.0 | 2.9 | 0.0 | 1.3 | 2.0 | 2.4 | 2.6 |
| hsa-miR-4635 | 4.0 | 3.8 | 3.9 | 4.0 | 3.7 | 3.6 | 3.3 | 2.9 | 2.7 |
| hsa-miR-5091 | 3.5 | 3.4 | 3.4 | 3.9 | 2.9 | 3.3 | 3.0 | 3.8 | 3.0 |
| hsa-miR-1254 | 3.8 | 3.7 | 3.5 | 3.1 | 3.8 | 4.3 | 3.9 | 7.5 | 4.9 |
| hsa-miR-4296 | 3.9 | 3.9 | 3.8 | 4.0 | 3.7 | 3.3 | 4.1 | 4.3 | 4.0 |
| hsa -miR -6504-3p | 3.8 | 4.0 | 4.3 | 4.5 | 4.3 | 4.0 | 4.2 | 3.9 | 4.3 |
| hsa -miR -3944-5p | 3.9 | 4.1 | 4.4 | 4.3 | 3.2 | 4.2 | 3.4 | 3.9 | 3.8 |
| hsa-miR-0541-5p | 3.5 | 3.6 | 3.7 | 3.8 | 3.4 | 2.8 | 3.8 | 3.7 | 3.0 |
| hsa-miR-5580-5p | 3.3 | 3.3 | 4.1 | 4.2 | 3.0 | 3.6 | 3.8 | 3.8 | 3.1 |
| hsa-miR-3655 | 4.0 | 3.9 | 4.3 | 4.4 | 4.3 | 4.2 | 3.3 | 4.1 | 4.0 |
| hsa -miR -0029c-5p | 2.9 | 3.5 | 3.7 | 3.5 | 1.8 | 3.5 | 3.3 | 2.7 | 3.2 |
| hsa-miR-1184 | 4.1 | 4.7 | 5.2 | 4.4 | 4.1 | 4.2 | 4.6 | 4.4 | 4.2 |
| hsa-miR-5571-5p | 3.9 | 4.1 | 4.2 | 3.5 | 2.8 | 3.5 | 4.0 | 3.5 | 3.7 |
| hsa-miR-0092b-3p | 4.9 | 5.2 | 5.0 | 4.4 | 4.0 | 4.0 | 3.3 | 3.2 | 3.2 |
| hsa-miR-5587-3p | 4.5 | 4.6 | 3.8 | 3.8 | 1.3 | 3.4 | 3.2 | 3.0 | 3.2 |
| hsa-miR-0769-3p | 4.7 | 3.4 | 3.4 | 3.7 | 5.0 | 4.4 | 4.0 | 4.9 | 4.9 |
| hsa-miR-0935 | 4.8 | 4.6 | 4.2 | 4.2 | 3.9 | 3.9 | 3.4 | 3.4 | 2.8 |
| hsa-miR-1199-5p | 5.4 | 5.2 | 4.2 | 3.6 | 3.5 | 3.3 | 3.3 | 4.7 | 2.9 |
| hsa-miR-0520a-3p | 3.9 | 3.9 | 4.4 | 3.2 | 1.7 | 2.2 | 2.7 | 2.2 | 2.5 |
| hsa-miR-0297 | 3.2 | 3.2 | 2.3 | 3.3 | 1.1 | 2.4 | 0.0 | 2.2 | 2.8 |
| hsa-miR-6841-5p | 4.3 | 4.1 | 5.0 | 5.0 | 4.1 | 4.7 | 4.0 | 2.7 | 2.8 |
| hsa-miR-7151-5p | 3.1 | 3.5 | 4.1 | 4.0 | 3.7 | 3.6 | 3.9 | 3.4 | 4.2 |
| hsa-miR-1277-3p | 0.0 | 0.0 | 0.0 | 0.0 | 0.0 | 0.0 | 0.0 | 0.0 | 0.0 |
| hsa-miR-0033b-3p | 4.6 | 4.6 | 4.6 | 3.4 | 4.1 | 2.8 | 3.7 | 3.1 | 3.8 |
| hsa-miR-4304 | 4.2 | 4.5 | 3.2 | 4.0 | 2.7 | 4.5 | 3.1 | 3.6 | 3.7 |
| hsa-miR-0873-3p | 2.1 | 3.2 | 3.9 | 4.5 | 3.8 | 3.5 | 6.2 | 5.6 | 6.3 |
| hsa-miR-4420 | 3.1 | 3.6 | 4.8 | 4.2 | 3.8 | 4.1 | 4.6 | 3.8 | 4.7 |
| hsa-miR-4479 | 5.0 | 5.2 | 4.4 | 3.4 | 2.8 | 3.9 | 2.7 | 2.7 | 2.8 |
| hsa-miR-0105-5p | 3.4 | 3.1 | 3.3 | 4.0 | 2.8 | 2.7 | 3.9 | 3.1 | 3.6 |
| hsa-miR-6855-5p | 1.4 | 3.0 | 3.6 | 2.9 | 3.8 | 4.4 | 3.8 | 4.8 | 4.2 |
| hsa-miR-3975 | 3.8 | 2.3 | 3.5 | 3.3 | 0.0 | 2.5 | 4.6 | 3.7 | 4.5 |
| hsa-miR-4453 | 2.4 | 3.6 | 3.2 | 3.8 | 3.3 | 2.8 | 4.1 | 4.0 | 3.9 |
| hsa-miR-1236-5p | 3.2 | 3.4 | 4.0 | 4.7 | 4.1 | 5.5 | 3.8 | 4.5 | 4.0 |
| hsa-miR-7162-3p | 2.7 | 3.7 | 4.4 | 3.9 | 3.0 | 3.4 | 3.5 | 4.6 | 3.7 |
| hsa-miR-0580-3p | 3.6 | 3.4 | 3.5 | 4.8 | 2.4 | 3.2 | 3.4 | 1.6 | 3.4 |
| hsa -miR -6852-5p | 3.6 | 3.3 | 3.7 | 4.1 | 3.5 | 4.2 | 3.4 | 3.4 | 3.7 |
| hsa-miR-0154-5p | 4.3 | 4.7 | 5.0 | 4.8 | 4.5 | 4.1 | 3.7 | 4.1 | 3.7 |
| hsa-miR-0412-3p | 3.4 | 4.0 | 4.4 | 4.4 | 4.1 | 3.4 | 3.8 | 3.7 | 3.8 |
| hsa-miR-6863 | 3.7 | 3.1 | 3.9 | 3.5 | 4.2 | 3.9 | 3.8 | 3.1 | 3.5 |
| hsa-miR-0676-5p | 2.4 | 3.5 | 3.3 | 2.4 | 1.4 | 2.8 | 3.9 | 2.5 | 3.4 |
| hsa-miR-6733-3p | 4.1 | 4.3 | 4.5 | 3.2 | 2.8 | 5.9 | 3.0 | 2.1 | 3.6 |
| hsa-miR-3972 | 4.0 | 4.8 | 5.0 | 4.4 | 3.5 | 4.1 | 4.4 | 3.3 | 3.7 |
| hsa-miR-0007-2-3p | 3.8 | 4.1 | 4.6 | 4.1 | 1.8 | 3.8 | 2.9 | 3.8 | 2.2 |
| hsa-miR-4441 | 5.2 | 4.5 | 5.1 | 5.3 | 5.2 | 4.5 | 3.4 | 3.7 | 3.1 |
| hsa-miR-4300 | 3.0 | 3.2 | 3.4 | 3.4 | 1.7 | 3.3 | 4.1 | 7.9 | 5.4 |
| hsa-miR-6874-3p | 4.1 | 4.5 | 4.8 | 4.2 | 3.5 | 4.0 | 3.6 | 3.2 | 3.3 |
| hsa-miR-3691-3p | 3.9 | 3.8 | 4.5 | 3.8 | 3.5 | 3.4 | 3.8 | 3.1 | 3.5 |
| hsa-miR-0034c-3p | 3.9 | 4.5 | 3.9 | 4.0 | 3.4 | 3.9 | 3.3 | 3.0 | 2.9 |
| hsa-miR-0519e-3p | 4.7 | 4.5 | 5.0 | 4.3 | 3.6 | 3.9 | 3.1 | 2.9 | 2.6 |
| hsa-miR-6774-3p | 3.5 | 4.7 | 5.1 | 3.9 | 3.8 | 4.0 | 4.0 | 3.4 | 3.4 |
| hsa-miR-1285-5p | 4.0 | 4.5 | 4.5 | 4.0 | 4.6 | 3.7 | 3.9 | 3.5 | 3.9 |
| hsa-miR-0518f-3p | 3.8 | 3.1 | 2.6 | 3.1 | 3.5 | 0.0 | 4.8 | 4.9 | 4.6 |
| hsa-miR-6791-3p | 4.7 | 4.9 | 4.6 | 3.7 | 3.7 | 3.9 | 4.0 | 3.5 | 3.0 |
| hsa-miR-4761-3p | 5.1 | 5.2 | 4.1 | 3.8 | 0.0 | 2.4 | 1.8 | 3.1 | 0.0 |
| hsa-miR-0548d-3p | 1.8 | 1.3 | 0.0 | 0.0 | 0.0 | 0.0 | 0.0 | 0.0 | 0.0 |
| hsa-miR-1266-3p | 4.5 | 4.7 | 4.1 | 4.1 | 4.8 | 5.3 | 4.1 | 3.7 | 4.5 |
| hsa-miR-0212-3p | 4.0 | 4.4 | 4.6 | 4.2 | 4.6 | 3.6 | 3.9 | 3.4 | 3.4 |
| hsa-miR-0744-3p | 4.3 | 4.2 | 4.0 | 4.0 | 3.2 | 3.5 | 3.9 | 3.6 | 3.4 |
| hsa-miR-9500 | 5.4 | 4.7 | 5.7 | 4.6 | 4.6 | 4.1 | 4.1 | 3.4 | 2.6 |
| hsa-miR-0670-5p | 3.5 | 4.5 | 5.2 | 4.4 | 3.8 | 4.7 | 4.9 | 4.1 | 4.6 |
| hsa-miR-2276-3p | 4.2 | 4.7 | 4.4 | 4.4 | 4.8 | 5.2 | 4.3 | 4.8 | 4.5 |
| hsa-miR-3155a | 2.8 | 3.0 | 3.2 | 3.5 | 2.5 | 3.0 | 3.1 | 3.5 | 2.7 |
| hsa-miR-0517a-3p, hsa-miR-517b-3p | 3.3 | 3.2 | 4.0 | 3.7 | 3.0 | 3.2 | 3.1 | 2.7 | 2.9 |
| hsa-miR-0493-3p | 3.0 | 3.8 | 3.5 | 4.5 | 4.4 | 4.9 | 3.8 | 3.7 | 3.7 |
| hsa-miR-0302d-5p | 4.8 | 3.6 | 5.3 | 3.7 | 2.8 | 2.9 | 4.3 | 3.8 | 4.2 |
| hsa-miR-4727-3p | 3.6 | 4.0 | 4.3 | 3.1 | 2.6 | 3.3 | 4.2 | 8.0 | 5.9 |
| hsa-miR-2355-3p | 3.5 | 4.0 | 3.6 | 2.8 | 0.0 | 2.9 | 1.8 | 1.9 | 1.6 |
| hsa-miR-3157-5p | 0.0 | 0.0 | 2.8 | 0.0 | 0.0 | 0.0 | 2.2 | 0.1 | 1.4 |
| hsa-miR-0766-5p | 0.0 | 3.3 | 3.9 | 3.1 | 1.5 | 3.4 | 4.4 | 4.4 | 4.1 |
| hsa-miR-0605-5p | 4.1 | 3.7 | 3.6 | 3.5 | 2.5 | 3.7 | 3.5 | 3.0 | 1.7 |
| hsa-miR-6758-3p | 3.5 | 4.9 | 4.8 | 3.6 | 4.0 | 3.8 | 3.3 | 3.4 | 3.5 |
| hsa-miR-6515-5p | 3.4 | 4.0 | 3.9 | 3.6 | 4.0 | 4.6 | 6.4 | 9.9 | 7.0 |
| hsa -miR -3692-5p | 3.1 | 3.8 | 4.4 | 2.9 | 3.2 | 3.3 | 3.9 | 3.1 | 3.4 |
| hsa-miR-5187-5p | 2.6 | 3.2 | 3.2 | 3.6 | 3.7 | 2.9 | 3.3 | 3.6 | 3.5 |
| hsa-miR-1287-5p | 3.6 | 3.5 | 4.8 | 4.2 | 3.2 | 3.0 | 4.4 | 3.8 | 3.6 |
| hsa-miR-0518f-5p | 1.8 | 2.2 | 2.4 | 2.1 | 3.0 | 0.0 | 3.4 | 2.9 | 3.1 |
| hsa-miR-4425 | 3.5 | 3.2 | 3.4 | 4.1 | 3.6 | 3.0 | 4.0 | 3.0 | 3.4 |
| hsa-miR-0181a-2-3p | 3.4 | 3.8 | 3.9 | 4.0 | 3.2 | 3.4 | 3.0 | 2.5 | 1.8 |
| hsa -miR -0222-5p | 3.9 | 4.1 | 4.6 | 4.6 | 3.9 | 3.8 | 3.8 | 3.8 | 3.5 |
| hsa-miR-0129-5p | 4.7 | 4.5 | 4.3 | 3.9 | 3.6 | 3.5 | 3.8 | 3.4 | 3.9 |
| hsa-miR-3659 | 4.5 | 4.1 | 4.6 | 4.8 | 4.1 | 3.6 | 4.0 | 4.2 | 3.9 |
| hsa-miR-7158-5p | 3.6 | 3.8 | 4.5 | 3.8 | 3.1 | 3.7 | 3.5 | 3.0 | 2.9 |
| hsa-miR-6516-5p | 1.7 | 2.9 | 3.5 | 3.6 | 1.7 | 3.5 | 4.6 | 3.4 | 4.4 |
| hsa-miR-0216b-3p | 5.2 | 5.5 | 5.6 | 5.2 | 5.0 | 5.0 | 3.4 | 3.0 | 2.9 |
| hsa-miR-6739-3p | 3.5 | 4.1 | 4.3 | 3.9 | 3.9 | 4.3 | 4.5 | 3.5 | 4.0 |
| hsa-miR-0609 | 2.7 | 3.5 | 3.0 | 3.1 | 2.2 | 2.9 | 3.6 | 2.9 | 3.1 |
| hsa-miR-0210-3p | 4.2 | 4.4 | 4.4 | 4.4 | 4.9 | 4.2 | 4.3 | 4.2 | 4.1 |
| hsa-miR-0323b-5p | 3.6 | 4.3 | 4.0 | 4.1 | 3.7 | 3.7 | 4.1 | 3.2 | 3.4 |
| hsa-miR-7844-5p | 4.2 | 4.0 | 5.1 | 3.7 | 3.8 | 3.8 | 3.3 | 2.8 | 2.4 |
| hsa-miR-4301 | 4.0 | 3.6 | 4.4 | 4.4 | 3.0 | 3.2 | 4.0 | 3.0 | 3.5 |
| hsa-miR-0134-3p | 3.7 | 4.2 | 4.5 | 4.0 | 4.4 | 4.3 | 4.8 | 5.0 | 4.6 |
| hsa-miR-3691-5p | 1.2 | 3.3 | 3.3 | 2.1 | 0.0 | 2.8 | 3.3 | 3.6 | 3.7 |
| hsa-miR-0425-3p | 3.7 | 4.5 | 3.8 | 3.6 | 4.1 | 3.9 | 3.2 | 2.2 | 2.2 |
| hsa-miR-0135a-3p | 6.0 | 4.4 | 4.8 | 4.7 | 5.7 | 5.6 | 4.9 | 5.5 | 5.4 |
| hsa-miR-1207-3p | 5.0 | 5.5 | 5.4 | 4.9 | 4.5 | 4.7 | 4.4 | 3.0 | 3.5 |
| hsa -miR -6856-3p | 4.1 | 4.3 | 4.6 | 4.2 | 4.0 | 3.7 | 3.6 | 3.3 | 3.7 |
| hsa-miR-4641 | 4.1 | 4.1 | 4.7 | 4.3 | 4.0 | 3.8 | 3.8 | 3.6 | 3.6 |
| hsa -miR -0520g-3p | 4.4 | 5.0 | 5.1 | 4.2 | 3.9 | 3.6 | 4.1 | 2.9 | 3.6 |
| hsa-miR-6843-3p | 4.2 | 4.5 | 4.4 | 3.6 | 2.9 | 3.9 | 4.0 | 3.0 | 3.9 |
| hsa-miR-0877-5p | 4.7 | 4.3 | 4.0 | 4.6 | 4.1 | 4.8 | 3.2 | 4.4 | 3.8 |
| hsa-miR-0584-3p | 3.7 | 4.3 | 4.6 | 4.7 | 4.5 | 3.7 | 4.1 | 4.1 | 4.1 |
| hsa-miR-4260 | 4.9 | 4.6 | 5.3 | 4.6 | 4.7 | 4.6 | 5.1 | 8.4 | 5.8 |
| hsa-miR-3619-5p | 4.8 | 4.6 | 4.7 | 4.3 | 4.5 | 4.3 | 3.9 | 4.1 | 3.6 |
| hsa-miR-7975 | 4.2 | 4.0 | 4.1 | 4.0 | 3.9 | 3.8 | 3.5 | 3.3 | 3.8 |
| hsa-miR-0512-5p | 4.9 | 4.9 | 5.1 | 4.9 | 4.4 | 4.1 | 4.1 | 3.8 | 3.5 |
| hsa-miR-4691-5p | 3.9 | 4.7 | 4.1 | 4.3 | 3.9 | 4.5 | 4.1 | 4.7 | 3.9 |
| hsa-miR-4713-3p | 4.0 | 4.4 | 5.2 | 4.0 | 4.5 | 4.5 | 4.3 | 3.8 | 5.4 |
| hsa-miR-6755-3p | 0.0 | 3.7 | 4.0 | 3.5 | 1.8 | 3.1 | 3.2 | 3.0 | 2.7 |
| hsa-miR-3945 | 4.6 | 4.4 | 5.0 | 5.4 | 5.6 | 6.3 | 4.5 | 3.5 | 4.1 |
| hsa-miR-0030b-3p | 4.2 | 4.3 | 3.7 | 3.9 | 3.7 | 5.2 | 3.7 | 6.3 | 4.3 |
| hsa-miR-0185-5p | 4.2 | 3.0 | 3.3 | 3.7 | 4.4 | 2.9 | 5.0 | 5.2 | 5.3 |
| hsa -miR -6878-5p | 3.8 | 4.4 | 4.9 | 3.5 | 4.5 | 4.0 | 3.8 | 4.7 | 4.3 |
| hsa-miR-0129-1-3p | 4.3 | 4.7 | 4.4 | 4.4 | 4.1 | 4.0 | 5.3 | 4.6 | 4.9 |
| hsa -miR -0548ba | 0.0 | 2.1 | 4.3 | 0.0 | 2.7 | 2.4 | 2.1 | 0.0 | 2.0 |
| hsa-miR-0150-5p | 4.6 | 4.7 | 4.5 | 4.5 | 4.3 | 3.8 | 3.4 | 3.2 | 3.8 |
| hsa-miR-1910-3p | 3.4 | 3.2 | 3.4 | 4.0 | 4.5 | 3.7 | 4.3 | 4.0 | 4.4 |
| hsa-miR-3138 | 3.6 | 4.0 | 4.9 | 4.6 | 4.8 | 5.0 | 4.2 | 5.2 | 3.8 |
| hsa-miR-6500-3p | 2.3 | 3.4 | 4.1 | 3.4 | 3.0 | 3.5 | 4.4 | 5.5 | 4.6 |
| hsa-miR-7157-5p | 4.2 | 3.5 | 4.7 | 4.1 | 4.4 | 3.3 | 4.4 | 4.3 | 4.1 |
| hsa-miR-0593-3p | 3.7 | 3.8 | 3.8 | 3.7 | 3.2 | 3.6 | 4.3 | 3.5 | 3.8 |
| hsa-miR-0018a-3p | 4.3 | 4.1 | 4.6 | 4.3 | 3.1 | 3.8 | 4.2 | 3.7 | 3.7 |
| hsa-miR-1972 | 4.9 | 4.7 | 4.9 | 4.1 | 5.2 | 4.7 | 3.7 | 4.0 | 4.6 |
| hsa-miR-3145-5p | 3.2 | 4.5 | 4.0 | 3.7 | 1.8 | 2.8 | 4.3 | 3.2 | 3.6 |
| hsa -miR -3922-3p | 4.6 | 4.6 | 4.6 | 4.5 | 4.0 | 3.9 | 3.3 | 3.0 | 3.2 |
| hsa-miR-6746-3p | 5.0 | 5.1 | 5.2 | 4.3 | 4.6 | 4.2 | 3.5 | 3.5 | 2.6 |
| hsa-miR-0093-5p | 3.3 | 3.6 | 3.9 | 3.4 | 3.4 | 2.6 | 4.5 | 2.8 | 3.9 |
| hsa-miR-1301-5p | 4.4 | 4.3 | 4.7 | 4.4 | 4.3 | 4.4 | 3.8 | 3.8 | 3.8 |
| hsa-miR-3116 | 4.1 | 4.8 | 4.8 | 5.0 | 4.3 | 4.2 | 4.8 | 4.7 | 4.9 |
| hsa-miR-0937-3p | 4.9 | 5.0 | 4.7 | 4.6 | 4.0 | 4.4 | 3.6 | 3.2 | 2.7 |
| hsa-miR-3187-3p | 4.7 | 4.8 | 4.7 | 4.5 | 4.7 | 5.0 | 3.9 | 5.9 | 5.2 |
| hsa-miR-0581 | 4.3 | 4.8 | 4.7 | 4.2 | 3.7 | 2.5 | 3.6 | 3.0 | 2.6 |
| hsa-miR-6816-3p | 4.1 | 4.2 | 4.3 | 4.0 | 5.1 | 4.8 | 4.1 | 3.7 | 4.7 |
| hsa-miR-1296-3p | 5.1 | 4.8 | 4.8 | 4.8 | 4.5 | 4.6 | 4.4 | 4.7 | 5.0 |
| hsa-miR-3921 | 4.6 | 5.3 | 5.5 | 4.3 | 3.5 | 3.7 | 3.5 | 2.2 | 2.0 |
| hsa-miR-1273g-5p | 3.5 | 3.9 | 3.9 | 4.0 | 4.0 | 3.6 | 3.8 | 4.3 | 3.8 |
| hsa -miR -0432-3p | 4.5 | 4.2 | 5.1 | 5.0 | 4.7 | 4.5 | 4.6 | 3.8 | 4.2 |
| hsa -miR -0656-5p | 4.2 | 4.1 | 4.0 | 4.3 | 3.1 | 3.7 | 4.3 | 4.2 | 3.6 |
| hsa-miR-4726-3p | 4.7 | 4.7 | 4.6 | 4.2 | 3.4 | 4.8 | 3.8 | 3.4 | 3.3 |
| hsa-miR-0092a-3p | 4.6 | 4.8 | 4.8 | 4.2 | 4.6 | 3.6 | 3.1 | 2.6 | 2.8 |
| hsa-miR-4265 | 4.4 | 5.1 | 5.0 | 4.0 | 4.3 | 5.1 | 4.2 | 4.0 | 3.7 |
| hsa-miR-0608 | 3.1 | 3.8 | 4.5 | 4.6 | 4.3 | 4.0 | 4.7 | 4.6 | 4.2 |
| hsa-miR-0135a-5p | 3.0 | 2.8 | 4.3 | 2.9 | 2.6 | 1.7 | 1.9 | 0.0 | 1.7 |
| hsa-miR-0500b-3p | 4.1 | 4.4 | 4.8 | 4.5 | 4.1 | 4.2 | 4.8 | 4.5 | 4.6 |
| hsa-miR-6823-5p | 1.8 | 3.6 | 3.1 | 2.8 | 2.9 | 3.3 | 3.7 | 4.8 | 3.8 |
| hsa-miR-4767 | 5.5 | 5.3 | 4.7 | 4.5 | 4.2 | 4.2 | 4.0 | 4.3 | 3.7 |
| hsa-miR-6499-3p | 3.6 | 4.7 | 4.4 | 4.4 | 4.4 | 3.9 | 4.3 | 3.2 | 3.8 |
| hsa-miR-6788-5p | 4.0 | 4.2 | 4.4 | 4.3 | 4.0 | 4.6 | 3.8 | 4.1 | 4.1 |
| hsa-miR-1200 | 4.9 | 4.8 | 4.9 | 4.4 | 4.0 | 4.2 | 4.0 | 2.9 | 3.4 |
| hsa -miR -4436a | 3.2 | 4.2 | 4.2 | 3.8 | 3.7 | 3.9 | 3.8 | 3.6 | 4.4 |
| hsa-miR-4700-5p | 3.7 | 4.4 | 4.5 | 5.1 | 4.5 | 4.9 | 3.9 | 5.8 | 4.7 |
| hsa -miR -3928-5p | 4.9 | 5.0 | 4.6 | 4.0 | 3.5 | 4.5 | 3.7 | 3.4 | 3.6 |
| hsa -miR -5006-3p | 3.9 | 3.5 | 3.9 | 4.4 | 4.0 | 4.3 | 4.2 | 4.1 | 4.2 |
| hsa-miR-7152-3p | 4.4 | 4.2 | 5.7 | 4.1 | 4.9 | 4.6 | 5.1 | 5.0 | 5.3 |
| hsa -miR -6504-5p | 4.3 | 3.9 | 4.2 | 4.5 | 4.1 | 4.1 | 4.4 | 4.0 | 4.0 |
| hsa-miR-0602 | 5.4 | 4.3 | 1.8 | 0.0 | 5.6 | 5.0 | 4.2 | 4.8 | 4.7 |
| hsa-miR-0330-5p | 4.4 | 4.1 | 3.8 | 3.5 | 3.6 | 3.8 | 3.0 | 3.3 | 2.9 |
| hsa-miR-0491-5p | 4.7 | 4.6 | 5.0 | 5.1 | 4.9 | 5.8 | 5.6 | 7.4 | 6.2 |
| hsa-miR-6747-5p | 2.8 | 4.1 | 3.7 | 4.3 | 4.8 | 4.7 | 4.8 | 6.8 | 4.9 |
| hsa -miR -6822-3p | 4.2 | 4.5 | 4.6 | 4.4 | 4.2 | 4.5 | 4.7 | 4.0 | 4.6 |
| hsa-miR-7850-5p | 4.0 | 4.1 | 4.1 | 4.7 | 4.1 | 4.2 | 4.0 | 4.6 | 4.6 |
| hsa-miR-0572 | 5.4 | 5.2 | 4.7 | 4.3 | 5.0 | 6.0 | 4.1 | 5.7 | 4.8 |
| hsa-miR-3065-3p | 3.3 | 4.6 | 3.9 | 3.7 | 2.6 | 3.4 | 3.3 | 3.1 | 3.3 |
| hsa-miR-3167 | 0.0 | 0.0 | 0.0 | 0.0 | 0.0 | 0.0 | 0.0 | 0.0 | 0.0 |
| hsa-miR-1273h-3p | 4.6 | 4.7 | 4.9 | 4.1 | 4.6 | 4.4 | 4.2 | 3.7 | 3.7 |
| hsa-miR-3198 | 4.6 | 4.0 | 5.3 | 5.6 | 5.8 | 6.0 | 3.9 | 4.5 | 3.7 |
| hsa-miR-6764-3p | 4.5 | 4.7 | 5.8 | 4.4 | 4.4 | 4.3 | 4.6 | 4.2 | 3.7 |
| hsa-miR-4538 | 3.2 | 3.5 | 4.3 | 4.4 | 4.2 | 3.8 | 4.3 | 5.8 | 4.5 |
| hsa-miR-0378a-3p | 4.4 | 4.9 | 5.4 | 4.1 | 4.6 | 4.2 | 4.5 | 3.8 | 4.2 |
| hsa-miR-3612 | 4.0 | 4.6 | 4.6 | 4.5 | 5.2 | 4.3 | 4.0 | 3.8 | 3.5 |
| hsa -miR -0552-5p | 4.8 | 4.7 | 4.9 | 4.0 | 3.8 | 3.7 | 3.9 | 2.2 | 2.7 |
| hsa-miR-8070 | 3.2 | 3.6 | 4.0 | 3.7 | 3.8 | 4.0 | 4.1 | 3.4 | 3.3 |
| hsa-miR-3689b-3p, hsa-miR-3689c | 4.4 | 4.3 | 4.9 | 4.6 | 4.7 | 4.3 | 4.2 | 4.8 | 4.8 |
| hsa-let-7a-2-3p | 4.2 | 4.8 | 5.0 | 4.4 | 3.5 | 4.0 | 4.0 | 3.6 | 3.4 |
| hsa -miR -0668-3p | 4.8 | 5.2 | 5.0 | 4.8 | 4.3 | 4.7 | 3.9 | 3.7 | 4.0 |
| hsa-miR-0023a-5p | 4.4 | 5.0 | 5.0 | 4.6 | 4.8 | 4.2 | 4.5 | 4.4 | 4.6 |
| hsa-miR-4288 | 4.4 | 4.7 | 6.0 | 4.7 | 3.9 | 4.5 | 5.1 | 3.8 | 4.1 |
| hsa-miR-6077 | 4.5 | 4.9 | 5.7 | 5.7 | 6.6 | 7.4 | 4.7 | 4.1 | 4.3 |
| hsa-miR-0770-5p | 4.9 | 4.9 | 4.4 | 4.3 | 3.5 | 3.4 | 3.9 | 3.7 | 3.3 |
| hsa-miR-1306-3p | 3.9 | 4.0 | 4.2 | 4.5 | 4.3 | 4.0 | 4.5 | 4.0 | 4.2 |
| hsa-miR-3120-5p | 4.2 | 4.2 | 4.6 | 4.1 | 4.3 | 3.9 | 3.9 | 3.8 | 4.1 |
| hsa-miR-0337-3p | 3.8 | 3.9 | 4.0 | 3.5 | 4.0 | 3.9 | 4.0 | 3.3 | 3.5 |
| hsa-miR-6512-3p | 4.6 | 4.7 | 4.9 | 5.0 | 4.6 | 4.5 | 4.4 | 4.7 | 4.3 |
| hsa-miR-1914-5p | 6.1 | 6.1 | 5.7 | 5.0 | 5.0 | 4.6 | 4.4 | 3.9 | 3.6 |
| hsa-miR-3144-3p | 0.0 | 3.5 | 3.9 | 2.8 | 2.1 | 2.9 | 2.9 | 2.5 | 2.8 |
| hsa-miR-0887-5p | 4.9 | 4.3 | 5.3 | 4.7 | 4.8 | 5.2 | 5.3 | 5.2 | 5.2 |
| hsa-miR-3927-5p | 2.5 | 3.5 | 4.3 | 3.8 | 3.9 | 3.7 | 4.0 | 3.8 | 3.7 |
| hsa-miR-3177-5p | 6.1 | 4.9 | 4.1 | 2.9 | 3.8 | 2.8 | 3.8 | 3.6 | 3.6 |
| hsa-miR-3176 | 4.3 | 4.4 | 4.1 | 4.4 | 4.1 | 4.3 | 4.0 | 4.8 | 4.0 |
| hsa-miR-1291 | 3.8 | 4.6 | 4.6 | 4.2 | 3.7 | 3.9 | 3.8 | 3.1 | 3.8 |
| hsa-miR-0891a-5p | 3.5 | 4.3 | 4.0 | 4.2 | 3.6 | 3.6 | 3.6 | 3.9 | 3.5 |
| hsa-miR-4708-3p | 5.2 | 5.0 | 5.1 | 4.8 | 4.6 | 4.7 | 4.2 | 4.4 | 4.3 |
| hsa-miR-5010-5p | 5.0 | 4.8 | 4.9 | 6.0 | 5.0 | 6.3 | 6.5 | 9.5 | 8.0 |
| hsa-miR-6773-3p | 4.5 | 5.0 | 4.9 | 4.6 | 4.0 | 4.8 | 4.6 | 4.2 | 4.9 |
| hsa-miR-5581-3p | 4.7 | 5.3 | 5.6 | 5.0 | 4.7 | 4.8 | 4.7 | 4.4 | 4.4 |
| hsa-miR-0365b-5p | 4.4 | 5.1 | 4.9 | 4.2 | 4.1 | 4.4 | 4.3 | 4.8 | 4.4 |
| hsa -miR -4652-5p | 5.2 | 5.1 | 4.9 | 4.6 | 3.6 | 3.3 | 3.7 | 4.1 | 3.4 |
| hsa-miR-0125b-5p | 4.9 | 4.6 | 5.7 | 5.4 | 5.3 | 5.3 | 4.7 | 3.9 | 3.6 |
| hsa-miR-4502 | 1.8 | 3.2 | 4.4 | 3.8 | 1.9 | 3.8 | 3.1 | 4.1 | 3.9 |
| hsa-miR-4673 | 5.2 | 5.4 | 4.2 | 4.7 | 4.8 | 5.8 | 4.3 | 4.6 | 4.3 |
| hsa -miR -5588-3p | 3.2 | 4.1 | 4.4 | 4.1 | 4.2 | 4.1 | 4.2 | 4.2 | 4.2 |
| hsa-miR-0671-3p | 4.4 | 4.6 | 4.9 | 4.1 | 4.1 | 4.9 | 4.0 | 3.9 | 3.8 |
| hsa-miR-5001-3p | 4.1 | 4.2 | 5.0 | 4.3 | 4.2 | 4.1 | 4.3 | 3.8 | 3.8 |
| hsa -miR -6828-5p | 3.3 | 3.6 | 3.9 | 4.0 | 4.2 | 4.8 | 5.2 | 5.2 | 5.1 |
| hsa-miR-4319 | 4.3 | 4.8 | 4.8 | 4.4 | 4.0 | 4.1 | 4.2 | 4.1 | 3.9 |
| hsa-miR-6814-3p | 4.3 | 5.0 | 5.2 | 4.1 | 3.7 | 4.2 | 4.1 | 4.1 | 4.3 |
| hsa-miR-1273f | 3.7 | 4.6 | 4.2 | 4.6 | 5.6 | 4.9 | 4.8 | 4.3 | 5.5 |
| hsa-miR-0099b-3p | 3.9 | 4.2 | 4.6 | 4.3 | 3.5 | 4.0 | 4.2 | 3.6 | 3.7 |
| hsa-miR-3622a-5p | 5.1 | 4.7 | 4.7 | 5.1 | 4.4 | 5.7 | 4.0 | 4.5 | 4.4 |
| hsa-miR-6833-5p | 4.5 | 4.5 | 4.7 | 5.3 | 5.6 | 5.7 | 4.4 | 6.3 | 4.8 |
| hsa-miR-2277-5p | 4.4 | 4.5 | 4.0 | 3.5 | 3.9 | 4.3 | 2.7 | 3.2 | 3.1 |
| hsa -miR -3156-5p | 4.3 | 4.8 | 5.6 | 5.3 | 5.3 | 4.6 | 5.9 | 7.2 | 6.8 |
| hsa-miR-3685 | 4.3 | 4.6 | 5.0 | 4.7 | 4.5 | 4.3 | 4.2 | 3.7 | 3.9 |
| hsa-miR-7976 | 4.1 | 4.7 | 4.7 | 4.3 | 4.4 | 4.4 | 4.3 | 4.2 | 4.1 |
| hsa-miR-3189-3p | 4.4 | 4.3 | 4.5 | 4.5 | 3.6 | 4.1 | 4.6 | 6.6 | 4.8 |
| hsa-miR-0138-1-3p | 4.6 | 4.7 | 4.3 | 4.0 | 3.4 | 3.8 | 3.2 | 3.5 | 3.7 |
| hsa-miR-6789-3p | 4.8 | 5.4 | 5.0 | 4.8 | 4.7 | 5.0 | 4.3 | 4.0 | 4.0 |
| hsa-miR-0661 | 4.6 | 4.8 | 4.8 | 4.5 | 4.5 | 4.0 | 4.5 | 4.1 | 4.3 |
| hsa-miR-4654 | 3.8 | 3.8 | 4.1 | 3.6 | 3.9 | 4.6 | 3.3 | 4.6 | 4.5 |
| hsa-miR-4692 | 2.9 | 3.5 | 4.3 | 3.5 | 2.3 | 3.6 | 4.3 | 4.1 | 4.1 |
| hsa-miR-1471 | 5.1 | 5.0 | 5.3 | 5.2 | 5.3 | 5.4 | 4.8 | 6.5 | 5.4 |
| hsa-miR-3129-5p | 0.0 | 0.0 | 0.0 | 0.0 | 0.0 | 0.0 | 0.0 | 0.0 | 0.0 |
| hsa-miR-6764-5p | 4.3 | 4.5 | 5.0 | 4.2 | 4.0 | 4.2 | 4.7 | 4.0 | 4.1 |
| hsa-miR-4529-3p | 3.9 | 4.5 | 4.5 | 3.8 | 3.0 | 4.0 | 3.0 | 3.4 | 3.7 |
| hsa-miR-0505-5p | 4.8 | 4.5 | 4.8 | 5.3 | 4.8 | 4.7 | 5.3 | 4.9 | 5.5 |
| hsa-miR-6511b-5p | 4.5 | 4.6 | 4.6 | 4.5 | 4.0 | 4.1 | 5.3 | 7.1 | 5.9 |
| hsa-miR-4686 | 3.3 | 4.1 | 4.2 | 3.9 | 2.9 | 3.7 | 4.6 | 4.1 | 4.4 |
| hsa-miR-0551a | 4.7 | 4.7 | 4.1 | 4.4 | 3.7 | 4.0 | 3.5 | 3.2 | 3.9 |
| hsa -miR -6892-5p | 3.7 | 3.9 | 4.5 | 4.7 | 5.3 | 5.7 | 6.5 | 7.6 | 7.3 |
| hsa-miR-3935 | 4.5 | 4.6 | 4.3 | 4.5 | 4.1 | 4.1 | 3.7 | 3.7 | 4.3 |
| hsa-miR-0193a-5p | 4.2 | 3.8 | 4.5 | 5.0 | 4.7 | 4.4 | 4.1 | 5.0 | 4.5 |
| hsa-miR-1293 | 3.5 | 3.8 | 4.3 | 4.0 | 2.8 | 4.0 | 5.0 | 4.2 | 4.8 |
| hsa-miR-4540 | 3.1 | 4.3 | 4.7 | 4.1 | 3.4 | 4.2 | 4.7 | 4.1 | 4.4 |
| hsa-miR-0331-3p | 4.6 | 4.8 | 5.2 | 4.5 | 4.1 | 4.1 | 5.0 | 4.2 | 4.8 |
| hsa-miR-6817-3p | 4.4 | 4.8 | 5.0 | 4.6 | 4.4 | 4.3 | 4.4 | 3.8 | 4.0 |
| hsa -miR -6884-5p | 3.9 | 4.6 | 4.7 | 4.1 | 3.6 | 3.8 | 4.6 | 4.1 | 4.2 |
| hsa -miR -6862-5p | 4.7 | 5.1 | 5.2 | 5.4 | 5.1 | 4.7 | 4.7 | 5.5 | 5.2 |
| hsa-miR-1266-5p | 4.6 | 4.8 | 4.4 | 4.2 | 3.9 | 4.7 | 4.0 | 3.2 | 3.9 |
| hsa-miR-1204 | 4.2 | 4.4 | 4.8 | 4.2 | 4.4 | 4.2 | 4.0 | 4.1 | 3.6 |
| hsa -miR -0030c-1-3p | 6.1 | 5.4 | 6.3 | 6.1 | 6.8 | 7.5 | 5.4 | 4.4 | 4.3 |
| hsa-miR-1468-5p | 3.4 | 3.5 | 3.4 | 3.8 | 3.1 | 2.7 | 2.9 | 2.7 | 3.8 |
| hsa-miR-3127-5p | 1.9 | 2.5 | 2.4 | 2.9 | 2.4 | 4.3 | 2.8 | 2.6 | 2.3 |
| hsa-miR-0527, hsa-miR-518a-5p | 4.4 | 4.0 | 4.2 | 3.5 | 4.4 | 2.3 | 4.7 | 5.1 | 4.9 |
| hsa-miR-4307 | 4.1 | 4.0 | 4.5 | 4.6 | 4.4 | 4.5 | 5.4 | 4.4 | 5.1 |
| hsa-miR-6722-5p | 5.1 | 5.3 | 4.8 | 4.6 | 4.6 | 4.6 | 4.2 | 4.2 | 4.1 |
| hsa-miR-0657 | 5.0 | 4.9 | 5.0 | 5.1 | 4.8 | 5.1 | 4.7 | 4.5 | 4.5 |
| hsa-miR-4684-5p | 4.7 | 5.4 | 4.7 | 4.0 | 4.1 | 3.0 | 3.6 | 4.4 | 2.5 |
| hsa-miR-6780a-3p | 3.3 | 4.5 | 4.9 | 4.8 | 4.6 | 4.9 | 4.6 | 4.4 | 4.6 |
| hsa-miR-0885-3p | 5.2 | 5.6 | 4.6 | 6.2 | 5.7 | 6.9 | 4.5 | 5.0 | 5.2 |
| hsa-miR-0034a-5p | 4.0 | 2.7 | 4.2 | 4.3 | 3.8 | 3.1 | 3.7 | 3.7 | 3.7 |
| hsa-miR-0194-3p | 4.5 | 4.7 | 4.6 | 4.8 | 4.2 | 4.5 | 4.3 | 5.7 | 5.2 |
| hsa-miR-4776-3p | 2.8 | 4.0 | 4.6 | 3.8 | 4.1 | 3.7 | 4.5 | 4.0 | 4.0 |
| hsa -miR -3064-3p | 3.9 | 4.4 | 4.3 | 3.9 | 3.3 | 3.8 | 4.4 | 3.3 | 3.9 |
| hsa-miR-3165 | 0.0 | 2.0 | 2.1 | 1.8 | 0.0 | 0.0 | 1.5 | 3.1 | 2.7 |
| hsa-miR-4473 | 3.4 | 4.1 | 4.9 | 3.3 | 2.4 | 3.1 | 4.1 | 3.3 | 3.9 |
| hsa-miR-0625-5p | 4.8 | 4.3 | 4.1 | 4.2 | 4.2 | 1.7 | 3.1 | 5.5 | 2.8 |
| hsa-miR-4252 | 3.9 | 4.4 | 4.5 | 4.5 | 4.5 | 4.2 | 3.7 | 4.0 | 4.2 |
| hsa -miR -6866-3p | 4.1 | 4.1 | 4.7 | 4.1 | 4.2 | 4.3 | 4.3 | 3.8 | 3.5 |
| hsa-miR-1203 | 5.7 | 5.4 | 5.1 | 5.0 | 5.8 | 6.2 | 4.6 | 3.8 | 3.8 |
| hsa -miR -3934-5p | 2.4 | 4.0 | 4.1 | 3.9 | 3.7 | 4.0 | 3.8 | 4.3 | 4.4 |
| hsa-miR-4647 | 1.8 | 4.0 | 4.7 | 4.4 | 3.9 | 4.1 | 4.1 | 4.4 | 4.2 |
| hsa-miR-4711-3p | 4.0 | 4.1 | 4.7 | 4.1 | 3.8 | 3.3 | 3.6 | 4.2 | 4.6 |
| hsa-miR-4436b-3p | 3.3 | 3.7 | 4.4 | 4.6 | 4.5 | 4.0 | 4.3 | 4.4 | 4.2 |
| hsa-miR-0299-5p | 5.0 | 4.8 | 5.2 | 5.1 | 4.4 | 4.3 | 3.8 | 4.0 | 3.6 |
| hsa-miR-0193b-3p | 5.1 | 5.3 | 5.6 | 5.2 | 5.1 | 4.8 | 4.2 | 3.5 | 3.4 |
| hsa-miR-3689d | 6.6 | 6.5 | 5.9 | 4.4 | 5.3 | 5.9 | 4.3 | 3.4 | 3.4 |
| hsa-miR-3617-3p | 5.0 | 4.7 | 5.3 | 5.1 | 4.9 | 4.6 | 5.1 | 4.6 | 4.6 |
| hsa-miR-6876-5p | 4.3 | 4.2 | 4.7 | 4.3 | 4.6 | 5.5 | 5.0 | 4.5 | 4.8 |
| hsa-miR-6847-5p | 3.3 | 3.9 | 4.7 | 4.2 | 3.9 | 4.1 | 4.7 | 4.5 | 4.2 |
| hsa-miR-0767-3p | 4.4 | 4.8 | 4.9 | 4.4 | 4.5 | 4.7 | 5.4 | 5.1 | 5.0 |
| hsa-miR-4748 | 4.8 | 5.0 | 5.1 | 6.0 | 6.4 | 6.9 | 5.1 | 4.1 | 5.4 |
| hsa-miR-3177-3p | 4.6 | 5.1 | 3.9 | 5.2 | 4.3 | 4.6 | 5.2 | 6.4 | 6.5 |
| hsa -miR -6886-5p | 4.8 | 4.8 | 5.1 | 4.5 | 4.6 | 4.7 | 4.5 | 5.0 | 4.7 |
| hsa-miR-4717-3p | 5.4 | 5.7 | 5.5 | 5.4 | 5.0 | 5.0 | 4.8 | 5.3 | 4.7 |
| hsa-miR-2113 | 3.9 | 4.3 | 5.0 | 4.3 | 4.6 | 4.0 | 4.7 | 3.3 | 4.3 |
| hsa-miR-3150b-3p | 3.6 | 4.5 | 4.5 | 4.1 | 3.5 | 4.2 | 4.0 | 3.9 | 4.2 |
| hsa-miR-4326 | 4.6 | 5.4 | 4.9 | 5.0 | 4.3 | 4.7 | 5.2 | 4.3 | 4.7 |
| hsa -miR -0892b | 4.3 | 4.6 | 5.0 | 4.8 | 4.8 | 4.6 | 4.7 | 4.4 | 4.8 |
| hsa-miR-6895-3p | 5.5 | 5.5 | 5.4 | 5.0 | 4.8 | 5.1 | 4.2 | 3.6 | 3.8 |
| hsa-miR-4708-5p | 5.4 | 5.5 | 4.9 | 4.8 | 4.8 | 4.9 | 4.2 | 3.7 | 4.2 |
| hsa -miR -6856-5p | 4.3 | 4.3 | 4.8 | 5.0 | 6.3 | 5.9 | 4.3 | 4.7 | 5.2 |
| hsa-miR-0550a-5p | 4.9 | 5.0 | 4.8 | 4.7 | 4.6 | 5.0 | 4.1 | 4.5 | 4.3 |
| hsa-miR-4324 | 5.1 | 4.5 | 5.2 | 5.1 | 4.7 | 4.6 | 4.4 | 3.8 | 4.0 |
| hsa-miR-4776-5p | 4.6 | 4.5 | 5.0 | 5.1 | 5.0 | 4.6 | 4.2 | 5.7 | 4.7 |
| hsa-miR-0629-3p | 4.7 | 5.1 | 4.9 | 4.7 | 3.6 | 4.6 | 3.9 | 3.5 | 3.8 |
| hsa-miR-1273h-5p | 4.5 | 4.9 | 5.3 | 4.9 | 6.2 | 6.1 | 5.1 | 6.1 | 5.8 |
| hsa-miR-1909-5p | 4.8 | 5.2 | 5.1 | 4.9 | 4.8 | 4.7 | 4.5 | 4.6 | 4.5 |
| hsa-miR-3137 | 3.6 | 3.3 | 4.0 | 4.0 | 3.2 | 4.1 | 4.0 | 5.1 | 4.6 |
| hsa-miR-4780 | 5.1 | 5.4 | 5.2 | 5.0 | 4.7 | 5.0 | 4.7 | 4.0 | 3.4 |
| hsa-miR-8064 | 5.0 | 5.1 | 4.7 | 5.2 | 5.2 | 5.5 | 4.2 | 5.2 | 4.9 |
| hsa-miR-7702 | 4.5 | 5.1 | 5.7 | 4.8 | 4.8 | 4.7 | 4.9 | 4.2 | 4.0 |
| hsa-miR-1181 | 5.8 | 5.7 | 5.4 | 4.9 | 5.2 | 5.3 | 4.4 | 4.2 | 4.6 |
| hsa-miR-4448 | 4.7 | 4.3 | 4.8 | 5.1 | 4.6 | 4.7 | 4.6 | 4.5 | 4.9 |
| hsa-miR-4800-5p | 4.4 | 4.3 | 4.5 | 4.7 | 5.6 | 5.8 | 5.0 | 5.9 | 5.3 |
| hsa-miR-8075 | 3.7 | 4.5 | 4.7 | 4.2 | 4.1 | 4.4 | 4.3 | 4.7 | 5.1 |
| hsa -miR -0494-5p | 4.6 | 4.9 | 5.2 | 4.9 | 4.7 | 4.6 | 4.9 | 4.5 | 4.5 |
| hsa-miR-4506 | 2.1 | 5.1 | 3.9 | 3.0 | 2.6 | 3.6 | 3.8 | 3.7 | 3.7 |
| hsa-miR-3591-3p | 3.9 | 3.8 | 4.3 | 3.6 | 2.4 | 3.7 | 3.7 | 4.3 | 4.8 |
| hsa-miR-0612 | 5.7 | 5.6 | 5.3 | 5.3 | 4.7 | 5.5 | 4.2 | 4.8 | 5.3 |
| hsa-miR-3714 | 3.8 | 5.4 | 5.0 | 4.7 | 4.3 | 4.3 | 5.3 | 4.5 | 5.0 |
| hsa-miR-3616-3p | 6.8 | 7.2 | 5.3 | 6.9 | 5.5 | 6.0 | 4.3 | 4.0 | 4.8 |
| hsa-miR-0211-5p | 4.5 | 4.5 | 4.5 | 4.6 | 5.1 | 4.4 | 4.8 | 4.6 | 4.3 |
| hsa-miR-1287-3p | 4.9 | 5.2 | 5.5 | 4.7 | 4.1 | 4.3 | 4.0 | 3.2 | 3.5 |
| hsa-miR-1538 | 5.6 | 5.6 | 5.3 | 5.3 | 4.5 | 5.0 | 4.8 | 4.7 | 4.6 |
| hsa-miR-3131 | 11.3 | 11.2 | 11.7 | 11.7 | 12.4 | 12.1 | 11.7 | 11.8 | 12.5 |
| hsa-miR-0526a, hsa-miR-520c-5p, hsa-miR-518d-5p | 3.9 | 2.7 | 3.7 | 3.0 | 4.3 | 2.4 | 3.9 | 4.0 | 3.9 |
| hsa -miR -0449c-3p | 5.2 | 5.2 | 5.2 | 4.8 | 4.7 | 4.5 | 4.4 | 4.6 | 4.1 |
| hsa-miR-0636 | 5.6 | 6.0 | 5.4 | 5.1 | 5.2 | 4.9 | 4.5 | 3.9 | 4.0 |
| hsa-miR-0589-5p | 5.1 | 5.4 | 5.5 | 4.8 | 4.4 | 3.6 | 4.4 | 4.0 | 3.4 |
| hsa-miR-6815-3p | 4.8 | 4.6 | 5.3 | 5.1 | 5.3 | 4.9 | 4.8 | 4.6 | 4.8 |
| hsa-miR-6507-3p | 4.5 | 4.6 | 5.3 | 4.6 | 4.6 | 4.5 | 5.5 | 5.1 | 5.4 |
| hsa -miR -6822-5p | 5.3 | 5.4 | 5.4 | 5.2 | 4.7 | 5.3 | 5.2 | 4.7 | 4.6 |
| hsa-miR-7855-5p | 4.5 | 4.2 | 4.7 | 4.4 | 3.9 | 4.6 | 5.7 | 5.3 | 5.5 |
| hsa-miR-4428 | 4.4 | 4.8 | 4.5 | 5.3 | 5.0 | 5.0 | 4.7 | 5.1 | 5.0 |
| hsa-miR-4476 | 5.2 | 5.8 | 5.0 | 5.9 | 5.3 | 7.1 | 4.7 | 4.9 | 5.1 |
| hsa-miR-0129-2-3p | 4.9 | 4.9 | 5.0 | 4.8 | 4.5 | 4.3 | 5.5 | 4.7 | 5.2 |
| hsa-miR-4278 | 5.7 | 5.5 | 5.4 | 5.2 | 5.2 | 4.8 | 5.1 | 4.8 | 4.6 |
| hsa-miR-5685 | 3.9 | 4.4 | 4.8 | 4.1 | 4.0 | 4.5 | 4.4 | 4.5 | 4.4 |
| hsa-miR-0192-5p | 4.6 | 4.5 | 5.1 | 4.5 | 4.4 | 4.4 | 4.0 | 4.1 | 4.1 |
| hsa-miR-4482-3p | 5.1 | 5.5 | 5.4 | 5.1 | 5.4 | 5.5 | 6.3 | 8.1 | 7.3 |
| hsa-miR-1296-5p | 5.4 | 5.3 | 5.2 | 4.5 | 4.7 | 4.5 | 4.5 | 4.0 | 4.3 |
| hsa-miR-0324-3p | 5.2 | 4.6 | 5.6 | 5.1 | 4.9 | 4.8 | 5.1 | 5.3 | 5.2 |
| hsa-miR-6873-5p | 4.6 | 5.3 | 5.8 | 5.2 | 5.4 | 4.6 | 4.4 | 4.4 | 4.5 |
| hsa-miR-6847-3p | 4.1 | 4.7 | 4.8 | 4.7 | 5.0 | 4.7 | 3.9 | 4.5 | 4.6 |
| hsa-miR-5708 | 4.7 | 4.2 | 4.6 | 4.7 | 4.4 | 4.2 | 3.9 | 4.6 | 4.4 |
| hsa-miR-4747-3p | 5.2 | 4.7 | 4.8 | 4.5 | 3.5 | 4.9 | 4.2 | 3.5 | 4.1 |
| hsa-miR-6801-5p | 4.6 | 4.5 | 4.8 | 4.4 | 4.0 | 4.0 | 5.1 | 4.6 | 4.5 |
| hsa-miR-0125a-5p | 4.8 | 5.1 | 5.4 | 5.3 | 5.0 | 4.9 | 4.6 | 3.9 | 3.9 |
| hsa-miR-4269 | 5.5 | 5.8 | 5.4 | 5.2 | 5.4 | 4.5 | 5.2 | 4.2 | 5.0 |
| hsa-miR-6881-3p | 5.1 | 5.4 | 5.6 | 5.4 | 5.3 | 4.8 | 5.3 | 4.7 | 4.9 |
| hsa-miR-4664-5p | 5.5 | 5.4 | 6.6 | 5.2 | 5.8 | 5.4 | 6.2 | 7.3 | 6.8 |
| hsa-miR-0431-3p | 5.1 | 5.4 | 5.3 | 4.5 | 3.8 | 3.8 | 4.6 | 3.9 | 3.7 |
| hsa-miR-6503-5p | 3.3 | 4.6 | 5.6 | 4.5 | 4.4 | 4.9 | 6.1 | 5.2 | 5.6 |
| hsa-miR-6723-5p | 6.1 | 6.4 | 5.4 | 4.8 | 5.0 | 3.9 | 4.5 | 5.5 | 3.5 |
| hsa-miR-7113-5p | 2.9 | 4.4 | 4.8 | 4.5 | 4.1 | 4.4 | 5.2 | 4.4 | 4.6 |
| hsa-miR-2467-3p | 5.0 | 5.1 | 5.0 | 5.2 | 5.2 | 5.2 | 5.0 | 4.8 | 4.9 |
| hsa-miR-3159 | 0.0 | 0.0 | 0.0 | 0.0 | 0.0 | 0.0 | 0.0 | 1.0 | 0.0 |
| hsa-miR-8077 | 4.4 | 4.2 | 4.7 | 4.8 | 5.3 | 4.9 | 4.0 | 5.5 | 5.0 |
| hsa-miR-2682-3p | 5.3 | 5.6 | 5.1 | 4.8 | 5.1 | 4.1 | 4.6 | 4.3 | 4.2 |
| hsa-miR-3162-3p | 6.6 | 6.7 | 6.9 | 6.6 | 6.4 | 6.2 | 7.2 | 6.5 | 7.0 |
| hsa-miR-4639-3p | 4.6 | 4.6 | 4.9 | 4.8 | 5.0 | 4.4 | 5.2 | 4.7 | 4.8 |
| hsa-miR-4725-5p | 5.4 | 5.5 | 5.4 | 5.2 | 5.2 | 5.2 | 4.7 | 4.2 | 4.4 |
| hsa-miR-7151-3p | 4.0 | 4.3 | 4.1 | 4.1 | 3.8 | 4.6 | 4.9 | 5.6 | 4.8 |
| hsa-miR-4306 | 4.4 | 5.0 | 5.2 | 4.6 | 5.2 | 4.0 | 5.8 | 6.4 | 6.4 |
| hsa-miR-0585-5p | 5.1 | 5.7 | 5.4 | 5.1 | 4.7 | 4.9 | 5.5 | 4.2 | 4.7 |
| hsa-miR-4804-3p | 5.0 | 5.2 | 5.5 | 4.6 | 4.6 | 4.5 | 4.5 | 4.2 | 4.1 |
| hsa-miR-4474-3p | 4.0 | 4.5 | 4.4 | 3.8 | 3.7 | 3.8 | 4.3 | 4.3 | 4.6 |
| hsa-miR-6876-3p | 3.7 | 4.8 | 5.5 | 4.6 | 4.2 | 4.4 | 5.0 | 4.5 | 4.5 |
| hsa-miR-1224-5p | 5.1 | 4.8 | 4.9 | 5.3 | 5.1 | 5.1 | 4.7 | 6.7 | 5.5 |
| hsa -miR -6832-3p | 4.9 | 5.4 | 5.6 | 5.2 | 5.5 | 5.4 | 5.1 | 4.4 | 5.0 |
| hsa-miR-0191-3p | 5.5 | 5.0 | 5.6 | 5.2 | 5.1 | 4.5 | 5.6 | 5.2 | 5.5 |
| hsa -miR -4638-5p | 5.4 | 5.4 | 5.2 | 5.8 | 5.7 | 5.7 | 4.9 | 6.2 | 5.3 |
| hsa-miR-4518 | 3.6 | 4.1 | 5.1 | 4.0 | 4.1 | 4.2 | 4.3 | 4.0 | 4.3 |
| hsa-miR-4487 | 5.3 | 5.2 | 4.7 | 4.9 | 5.0 | 5.1 | 4.4 | 4.9 | 4.8 |
| hsa-miR-0764 | 5.5 | 5.2 | 5.7 | 5.4 | 4.9 | 5.2 | 5.5 | 4.9 | 5.2 |
| hsa-miR-6767-5p | 4.0 | 4.8 | 5.0 | 4.5 | 5.0 | 4.5 | 4.8 | 4.7 | 4.9 |
| hsa -miR -3944-3p | 5.0 | 5.4 | 5.3 | 4.9 | 5.0 | 5.0 | 5.2 | 5.0 | 4.8 |
| hsa-miR-6829-3p | 5.5 | 5.5 | 5.8 | 5.2 | 5.2 | 5.4 | 5.2 | 4.4 | 4.7 |
| hsa-miR-4292 | 5.9 | 5.4 | 5.2 | 5.0 | 5.0 | 4.9 | 4.9 | 4.8 | 4.7 |
| hsa-miR-4525 | 5.4 | 4.8 | 6.3 | 4.5 | 5.0 | 4.9 | 6.0 | 5.3 | 5.3 |
| hsa-miR-4676-5p | 4.2 | 4.3 | 4.7 | 4.9 | 3.5 | 4.4 | 5.0 | 4.8 | 4.7 |
| hsa-miR-4489 | 4.8 | 4.7 | 4.9 | 5.7 | 5.4 | 5.5 | 5.8 | 7.3 | 6.2 |
| hsa-miR-0615-5p | 6.0 | 5.5 | 4.5 | 5.0 | 4.8 | 5.7 | 5.6 | 6.3 | 6.1 |
| hsa -miR -0342-3p | 4.9 | 4.6 | 4.2 | 4.7 | 4.5 | 4.3 | 3.8 | 3.7 | 4.2 |
| hsa-miR-0550a-3p | 5.4 | 5.2 | 5.2 | 5.2 | 4.6 | 4.4 | 4.7 | 4.6 | 4.7 |
| hsa-miR-4456 | 4.4 | 3.9 | 4.5 | 4.4 | 3.7 | 4.4 | 5.0 | 4.4 | 4.7 |
| hsa-miR-1908-3p | 6.2 | 5.6 | 5.1 | 4.8 | 4.9 | 4.9 | 5.2 | 5.7 | 5.3 |
| hsa-miR-3135b | 7.8 | 7.4 | 7.6 | 6.1 | 7.4 | 7.7 | 7.0 | 7.7 | 6.9 |
| hsa-miR-1539 | 5.9 | 6.0 | 6.0 | 5.6 | 5.3 | 5.3 | 4.9 | 4.8 | 4.6 |
| hsa-miR-3132 | 3.5 | 4.2 | 4.8 | 4.5 | 4.7 | 5.2 | 4.3 | 3.9 | 4.2 |
| hsa-miR-4709-3p | 5.1 | 5.7 | 5.8 | 5.0 | 5.0 | 5.3 | 4.9 | 5.1 | 5.3 |
| hsa-let-7f-1-3p | 5.2 | 5.5 | 5.4 | 5.1 | 4.8 | 5.1 | 5.9 | 5.1 | 5.5 |
| hsa-miR-4305 | 4.5 | 4.6 | 4.9 | 5.0 | 4.9 | 4.8 | 5.1 | 4.8 | 4.6 |
| hsa-miR-6770-3p | 5.3 | 5.0 | 4.9 | 2.7 | 4.6 | 4.7 | 3.6 | 5.5 | 4.1 |
| hsa-miR-4732-5p | 5.2 | 5.1 | 5.9 | 5.3 | 5.7 | 6.0 | 5.4 | 5.0 | 5.1 |
| hsa-miR-6716-3p | 4.6 | 4.8 | 4.9 | 4.4 | 4.0 | 4.7 | 4.9 | 3.6 | 5.1 |
| hsa-miR-0483-5p | 4.6 | 4.8 | 4.8 | 5.2 | 5.9 | 6.5 | 4.8 | 4.4 | 4.6 |
| hsa-miR-1226-3p | 5.1 | 5.4 | 5.7 | 5.1 | 4.9 | 4.9 | 4.9 | 4.3 | 4.7 |
| hsa-miR-6883-5p | 5.3 | 5.4 | 6.0 | 5.4 | 6.1 | 5.8 | 5.9 | 6.0 | 5.5 |
| hsa -miR -6834-5p | 4.2 | 4.4 | 4.4 | 5.0 | 4.9 | 4.6 | 5.1 | 6.3 | 5.9 |
| hsa-miR-6849-3p | 5.1 | 5.0 | 5.1 | 5.3 | 5.1 | 4.8 | 4.5 | 4.9 | 4.5 |
| hsa-miR-3667-3p | 4.1 | 5.1 | 5.3 | 4.7 | 4.8 | 5.0 | 4.7 | 4.7 | 4.9 |
| hsa-miR-4669 | 4.2 | 5.0 | 5.2 | 5.0 | 5.4 | 4.8 | 5.1 | 5.0 | 5.3 |
| hsa -miR -4646-5p | 5.3 | 5.5 | 5.5 | 5.7 | 6.0 | 5.3 | 5.8 | 5.4 | 5.9 |
| hsa-miR-4743-5p | 5.2 | 4.6 | 5.2 | 5.3 | 5.3 | 5.1 | 4.8 | 5.6 | 5.9 |
| hsa-let-7d-3p | 4.4 | 4.4 | 4.6 | 4.3 | 3.5 | 3.9 | 4.6 | 4.2 | 4.5 |
| hsa -miR -6872-3p | 5.5 | 6.2 | 6.0 | 5.5 | 6.0 | 5.6 | 5.3 | 5.7 | 5.7 |
| hsa-miR-0526b-5p | 4.5 | 4.6 | 5.3 | 4.5 | 4.7 | 4.4 | 4.7 | 4.5 | 4.5 |
| hsa-miR-0550b-3p | 4.4 | 5.2 | 5.2 | 4.5 | 4.3 | 4.7 | 4.8 | 4.2 | 4.0 |
| hsa -miR -4652-3p | 5.0 | 5.1 | 5.4 | 5.0 | 4.5 | 4.7 | 5.4 | 5.1 | 5.3 |
| hsa-miR-4682 | 3.5 | 4.5 | 4.4 | 4.0 | 3.1 | 4.5 | 4.0 | 4.5 | 4.8 |
| hsa-miR-7851-3p | 5.6 | 5.4 | 4.6 | 4.7 | 5.6 | 4.8 | 5.4 | 5.2 | 6.1 |
| hsa-miR-1323 | 5.7 | 5.5 | 7.0 | 5.6 | 5.6 | 6.4 | 5.9 | 6.4 | 5.9 |
| hsa -miR -3124-5p | 1.9 | 3.7 | 2.0 | 3.0 | 4.1 | 5.1 | 4.4 | 6.4 | 5.9 |
| hsa -miR -6852-3p | 5.1 | 5.1 | 5.8 | 4.8 | 4.5 | 4.6 | 4.8 | 4.4 | 4.5 |
| hsa-miR-4266 | 5.4 | 4.7 | 6.9 | 3.9 | 5.3 | 4.5 | 5.9 | 4.1 | 5.1 |
| hsa-miR-1247-5p | 5.2 | 5.4 | 5.3 | 5.1 | 4.9 | 4.9 | 5.4 | 4.9 | 5.2 |
| hsa -miR -6836-5p | 6.5 | 5.9 | 4.5 | 3.2 | 4.3 | 3.3 | 4.1 | 3.8 | 4.5 |
| hsa -miR -0520b | 3.7 | 3.0 | 4.5 | 3.4 | 4.3 | 3.2 | 6.0 | 5.7 | 6.2 |
| hsa-miR-3649 | 6.1 | 5.4 | 6.3 | 7.0 | 8.5 | 9.0 | 6.4 | 5.2 | 6.1 |
| hsa-miR-6782-5p | 5.2 | 5.9 | 4.7 | 5.4 | 6.5 | 6.8 | 5.3 | 6.2 | 5.8 |
| hsa -miR -6878-3p | 4.5 | 5.3 | 5.4 | 4.7 | 5.2 | 4.9 | 4.8 | 4.9 | 5.0 |
| hsa-miR-3646 | 5.4 | 5.8 | 5.8 | 5.6 | 4.8 | 5.4 | 4.8 | 4.8 | 4.6 |
| hsa-miR-0520d-5p | 4.7 | 4.3 | 4.6 | 4.2 | 4.8 | 2.9 | 5.2 | 6.0 | 5.7 |
| hsa-miR-1182 | 4.8 | 4.5 | 4.9 | 4.4 | 4.1 | 3.8 | 5.6 | 5.4 | 5.3 |
| hsa-miR-6837-5p | 5.1 | 5.2 | 4.9 | 4.9 | 4.8 | 5.6 | 4.7 | 4.5 | 4.8 |
| hsa -miR -0098-3p | 5.4 | 5.0 | 5.8 | 5.1 | 4.7 | 4.9 | 5.9 | 5.4 | 5.8 |
| hsa-miR-3650 | 5.3 | 5.5 | 5.4 | 4.9 | 3.8 | 4.7 | 5.2 | 4.6 | 4.5 |
| hsa-miR-0130b-5p | 5.7 | 6.0 | 5.8 | 5.1 | 4.9 | 4.5 | 4.7 | 5.0 | 4.5 |
| hsa-miR-4701-3p | 4.9 | 4.4 | 5.0 | 4.5 | 3.9 | 4.6 | 4.4 | 5.0 | 4.7 |
| hsa-miR-1273g-3p | 5.0 | 5.2 | 5.3 | 5.2 | 6.8 | 6.1 | 5.3 | 6.2 | 6.7 |
| hsa-miR-3689a-3p | 4.0 | 4.4 | 4.6 | 4.4 | 3.8 | 4.2 | 4.3 | 4.8 | 4.5 |
| hsa-miR-0187-3p | 4.7 | 4.8 | 4.8 | 4.3 | 3.9 | 4.2 | 5.1 | 4.4 | 4.7 |
| hsa-miR-5100 | 5.6 | 5.3 | 5.2 | 4.7 | 6.4 | 5.5 | 4.7 | 5.5 | 5.1 |
| hsa-miR-6514-3p | 4.4 | 4.8 | 4.8 | 4.7 | 4.3 | 4.6 | 4.4 | 4.5 | 4.5 |
| hsa-miR-0133a-3p | 5.3 | 5.2 | 5.6 | 5.2 | 4.9 | 4.3 | 5.6 | 4.7 | 5.0 |
| hsa-miR-0519d-3p | 5.4 | 5.6 | 5.9 | 5.2 | 5.0 | 5.1 | 5.2 | 4.4 | 4.7 |
| hsa-miR-0326 | 5.7 | 5.8 | 5.8 | 5.4 | 5.0 | 5.3 | 4.8 | 4.2 | 4.4 |
| hsa-miR-6766-5p | 6.2 | 6.4 | 5.1 | 7.2 | 6.6 | 6.6 | 5.9 | 6.7 | 7.1 |
| hsa-miR-4732-3p | 5.4 | 5.0 | 5.6 | 5.2 | 5.2 | 4.7 | 4.3 | 4.8 | 4.8 |
| hsa-miR-3189-5p | 5.6 | 5.3 | 5.8 | 5.4 | 5.0 | 5.0 | 5.6 | 5.1 | 5.4 |
| hsa -miR -5088-3p | 5.1 | 5.6 | 5.7 | 5.3 | 5.4 | 5.6 | 5.2 | 5.2 | 5.2 |
| hsa-miR-4648 | 5.6 | 5.8 | 5.1 | 4.1 | 5.2 | 6.0 | 4.6 | 5.3 | 5.1 |
| hsa-miR-0183-3p | 5.1 | 5.3 | 5.2 | 6.5 | 7.4 | 7.8 | 5.6 | 4.9 | 6.4 |
| hsa-miR-8073 | 5.7 | 6.2 | 5.7 | 5.4 | 5.6 | 6.2 | 5.4 | 6.8 | 6.0 |
| hsa -miR -6508-5p | 5.6 | 6.1 | 6.2 | 5.5 | 5.2 | 5.2 | 5.0 | 4.6 | 4.6 |
| hsa-miR-6830-3p | 4.6 | 5.2 | 5.5 | 5.1 | 5.2 | 5.3 | 5.4 | 4.6 | 5.2 |
| hsa-miR-0409-3p | 5.3 | 5.0 | 5.9 | 5.4 | 5.4 | 4.8 | 6.1 | 5.6 | 5.9 |
| hsa-miR-0564 | 4.6 | 4.7 | 4.8 | 4.2 | 3.7 | 4.6 | 3.9 | 4.6 | 4.7 |
| hsa-miR-4533 | 5.9 | 5.5 | 5.3 | 5.5 | 5.0 | 5.1 | 4.8 | 5.6 | 5.3 |
| hsa-miR-0134-5p | 6.1 | 6.1 | 5.6 | 6.4 | 5.9 | 5.9 | 5.5 | 5.2 | 5.2 |
| hsa-miR-4312 | 5.9 | 5.8 | 6.1 | 5.5 | 5.6 | 5.4 | 5.7 | 5.5 | 5.5 |
| hsa-miR-6509-3p | 4.1 | 5.1 | 5.0 | 4.7 | 4.7 | 4.8 | 4.3 | 4.6 | 4.5 |
| hsa-miR-7152-5p | 4.7 | 5.2 | 5.8 | 5.4 | 5.0 | 5.1 | 5.4 | 5.2 | 5.3 |
| hsa-miR-0346 | 5.3 | 5.7 | 5.8 | 5.3 | 5.4 | 5.1 | 4.4 | 4.6 | 4.4 |
| hsa-miR-3653-5p | 5.0 | 5.2 | 5.4 | 5.1 | 5.3 | 5.2 | 5.6 | 5.1 | 5.5 |
| hsa-miR-6775-3p | 5.9 | 5.8 | 5.9 | 5.3 | 5.2 | 5.1 | 5.5 | 5.2 | 5.2 |
| hsa-miR-0664a-3p | 5.6 | 5.9 | 6.3 | 5.4 | 5.2 | 5.0 | 5.7 | 5.3 | 5.6 |
| hsa-miR-6859-5p | 4.5 | 4.4 | 5.1 | 4.6 | 4.3 | 4.7 | 5.3 | 5.5 | 5.0 |
| hsa-miR-0632 | 5.2 | 4.8 | 5.9 | 5.8 | 5.0 | 4.8 | 5.2 | 5.0 | 4.9 |
| hsa-miR-6740-5p | 3.8 | 4.8 | 5.0 | 5.0 | 5.1 | 5.3 | 5.2 | 5.0 | 5.2 |
| hsa-miR-1227-3p | 4.6 | 5.3 | 5.6 | 5.0 | 5.2 | 4.9 | 5.6 | 5.0 | 5.4 |
| hsa-miR-5193 | 4.9 | 5.6 | 5.4 | 5.1 | 4.8 | 5.1 | 5.4 | 4.4 | 5.1 |
| hsa-miR-0214-3p | 4.6 | 5.1 | 5.1 | 5.5 | 5.4 | 4.9 | 5.0 | 4.3 | 4.6 |
| hsa-miR-6072 | 4.4 | 4.5 | 4.6 | 4.9 | 3.8 | 4.2 | 5.0 | 5.0 | 4.9 |
| hsa-miR-6133 | 5.2 | 5.8 | 5.9 | 5.7 | 6.0 | 6.1 | 5.7 | 5.7 | 5.5 |
| hsa-miR-4722-3p | 5.8 | 6.0 | 5.7 | 5.4 | 5.5 | 5.6 | 5.3 | 4.7 | 4.8 |
| hsa-miR-2392 | 5.4 | 5.3 | 5.1 | 5.8 | 6.0 | 6.7 | 5.3 | 6.2 | 5.6 |
| hsa -miR -3158-5p | 6.6 | 6.3 | 8.0 | 7.4 | 8.3 | 8.3 | 7.6 | 6.9 | 7.5 |
| hsa-miR-6788-3p | 5.2 | 5.8 | 6.0 | 5.2 | 5.3 | 5.4 | 5.2 | 4.9 | 4.8 |
| hsa-miR-4769-5p | 4.5 | 5.2 | 4.9 | 5.3 | 5.2 | 5.3 | 5.1 | 5.4 | 5.2 |
| hsa-miR-3190-5p | 5.3 | 5.3 | 5.5 | 5.3 | 4.6 | 4.7 | 6.2 | 5.4 | 5.6 |
| hsa-miR-6734-3p | 5.5 | 5.8 | 5.7 | 5.1 | 4.9 | 5.4 | 5.0 | 4.5 | 4.9 |
| hsa-miR-0466 | 4.9 | 4.1 | 4.7 | 4.0 | 3.0 | 4.1 | 4.2 | 4.8 | 4.7 |
| hsa-miR-3187-5p | 6.0 | 5.7 | 5.8 | 4.8 | 4.8 | 4.8 | 4.7 | 5.7 | 5.2 |
| hsa-miR-4297 | 5.0 | 5.5 | 5.7 | 5.5 | 5.5 | 4.7 | 5.3 | 5.0 | 5.1 |
| hsa -miR -6824-3p | 5.7 | 6.2 | 6.2 | 5.4 | 5.3 | 5.3 | 5.5 | 5.3 | 5.0 |
| hsa-miR-6734-5p | 4.9 | 5.1 | 5.2 | 5.2 | 5.4 | 5.8 | 6.0 | 5.9 | 6.2 |
| hsa-miR-0139-3p | 6.5 | 6.5 | 6.2 | 6.1 | 5.8 | 6.4 | 4.9 | 5.1 | 5.4 |
| hsa-miR-3192-3p | 5.6 | 5.7 | 5.9 | 5.4 | 5.1 | 5.4 | 4.9 | 4.6 | 4.7 |
| hsa-miR-6799-3p | 5.7 | 5.6 | 5.8 | 5.3 | 4.9 | 5.3 | 5.7 | 5.4 | 5.5 |
| hsa-miR-4329 | 5.5 | 5.8 | 6.0 | 5.7 | 5.5 | 5.5 | 5.7 | 5.4 | 5.1 |
| hsa-miR-0199b-5p | 5.1 | 5.2 | 5.5 | 5.4 | 4.9 | 4.6 | 5.7 | 4.9 | 5.3 |
| hsa-miR-0885-5p | 5.1 | 5.3 | 5.1 | 5.5 | 5.2 | 5.1 | 4.7 | 4.9 | 5.1 |
| hsa-miR-5589-5p | 4.9 | 5.0 | 4.5 | 4.1 | 4.2 | 4.6 | 4.6 | 6.0 | 5.5 |
| hsa-miR-4539 | 5.3 | 5.6 | 4.9 | 5.0 | 4.9 | 5.4 | 4.8 | 5.9 | 5.3 |
| hsa-miR-0223-3p | 5.2 | 5.5 | 5.8 | 5.3 | 4.9 | 5.2 | 6.3 | 5.4 | 5.8 |
| hsa-miR-6772-3p | 4.7 | 5.4 | 5.7 | 5.1 | 4.9 | 5.0 | 4.7 | 4.9 | 4.6 |
| hsa-miR-6841-3p | 5.5 | 5.2 | 6.1 | 5.3 | 5.4 | 5.3 | 6.1 | 5.7 | 5.7 |
| hsa -miR -4632-3p | 5.8 | 6.4 | 6.0 | 5.1 | 5.2 | 5.4 | 4.5 | 4.0 | 3.9 |
| hsa-miR-4523 | 5.4 | 6.6 | 7.1 | 5.4 | 5.6 | 6.1 | 5.5 | 4.8 | 4.9 |
| hsa -miR -6868-3p | 4.7 | 5.0 | 5.2 | 4.5 | 4.4 | 4.3 | 5.3 | 4.8 | 5.1 |
| hsa-miR-6810-5p | 4.7 | 5.5 | 4.9 | 5.0 | 5.0 | 4.6 | 4.7 | 5.2 | 5.4 |
| hsa-miR-3620-3p | 5.7 | 5.8 | 5.7 | 5.7 | 5.7 | 5.4 | 5.5 | 5.0 | 5.1 |
| hsa-miR-1250-3p | 5.5 | 5.5 | 6.0 | 5.4 | 5.0 | 5.4 | 5.4 | 5.2 | 4.7 |
| hsa-miR-0595 | 4.7 | 5.1 | 5.2 | 4.6 | 4.0 | 4.6 | 5.2 | 5.1 | 5.0 |
| hsa-miR-0513a-5p | 6.6 | 5.7 | 6.1 | 5.6 | 6.2 | 5.3 | 4.8 | 5.0 | 4.6 |
| hsa-miR-4787-3p | 5.6 | 5.8 | 5.9 | 5.3 | 5.4 | 5.6 | 5.2 | 4.8 | 5.1 |
| hsa-miR-0518b | 5.5 | 5.2 | 5.9 | 5.3 | 5.4 | 4.9 | 5.9 | 4.9 | 5.5 |
| hsa-miR-6849-5p | 4.6 | 4.6 | 4.9 | 4.9 | 5.4 | 5.3 | 5.7 | 6.8 | 5.7 |
| hsa -miR -0320b | 5.6 | 6.2 | 5.9 | 6.0 | 5.1 | 5.5 | 6.1 | 5.5 | 5.8 |
| hsa -miR -3064-5p | 5.7 | 5.8 | 5.7 | 5.7 | 5.8 | 5.4 | 4.7 | 5.0 | 4.9 |
| hsa-miR-3166 | 2.2 | 1.3 | 1.3 | 0.0 | 2.7 | 0.0 | 0.0 | 0.0 | 0.0 |
| hsa-miR-4481 | 4.7 | 5.5 | 5.8 | 4.8 | 5.0 | 5.9 | 5.6 | 5.8 | 5.3 |
| hsa-miR-3175 | 5.7 | 7.3 | 6.0 | 7.0 | 6.7 | 7.4 | 5.5 | 5.4 | 6.1 |
| hsa-miR-3180-5p | 6.0 | 6.2 | 6.1 | 5.7 | 5.6 | 5.3 | 5.8 | 5.4 | 5.5 |
| hsa-miR-0615-3p | 5.3 | 5.5 | 5.5 | 5.1 | 4.8 | 4.9 | 4.7 | 5.0 | 4.8 |
| hsa-miR-4449 | 6.3 | 6.5 | 5.6 | 5.2 | 5.9 | 6.5 | 4.6 | 5.6 | 5.4 |
| hsa-miR-4496 | 4.9 | 4.9 | 4.8 | 5.7 | 5.8 | 5.5 | 6.0 | 6.2 | 6.4 |
| hsa-miR-0198 | 5.1 | 4.9 | 5.2 | 5.1 | 5.5 | 5.2 | 6.0 | 5.1 | 5.9 |
| hsa-miR-0026b-3p | 5.5 | 5.3 | 5.4 | 5.3 | 4.8 | 4.9 | 5.3 | 4.5 | 4.9 |
| hsa-miR-0675-3p | 5.7 | 6.0 | 5.8 | 5.6 | 5.3 | 5.5 | 4.7 | 4.7 | 5.2 |
| hsa-miR-0765 | 5.5 | 5.3 | 5.3 | 7.2 | 8.2 | 9.0 | 5.5 | 5.1 | 5.2 |
| hsa-miR-0487a-5p | 4.8 | 4.8 | 5.0 | 5.3 | 5.0 | 4.9 | 4.5 | 4.8 | 5.0 |
| hsa-miR-5189-5p | 4.5 | 4.8 | 4.5 | 4.4 | 3.9 | 4.7 | 4.2 | 5.5 | 5.3 |
| hsa-miR-6871-5p | 4.2 | 4.9 | 5.3 | 4.8 | 4.8 | 4.8 | 4.6 | 6.9 | 5.5 |
| hsa-miR-0575 | 6.2 | 5.8 | 5.6 | 6.3 | 6.8 | 8.5 | 5.3 | 4.9 | 5.9 |
| hsa-miR-1910-5p | 6.0 | 6.1 | 5.8 | 5.6 | 5.2 | 5.4 | 4.8 | 4.6 | 4.6 |
| hsa-miR-3139 | 0.0 | 0.0 | 0.0 | 0.0 | 0.0 | 0.0 | 0.0 | 0.0 | 0.0 |
| hsa-miR-6762-3p | 5.2 | 5.4 | 5.9 | 4.9 | 5.2 | 5.3 | 5.6 | 5.2 | 5.2 |
| hsa-miR-4251 | 6.1 | 5.4 | 5.5 | 5.9 | 6.1 | 6.2 | 4.6 | 4.9 | 4.7 |
| hsa-miR-6790-3p | 5.8 | 6.0 | 6.3 | 5.2 | 5.5 | 5.5 | 6.0 | 5.7 | 5.8 |
| hsa-miR-6751-5p | 4.2 | 5.3 | 5.1 | 4.8 | 5.2 | 5.9 | 5.0 | 6.0 | 5.6 |
| hsa-miR-3173-3p | 5.5 | 5.9 | 5.4 | 5.4 | 5.5 | 5.6 | 3.4 | 3.4 | 3.5 |
| hsa-miR-6783-3p | 5.1 | 5.2 | 5.6 | 5.3 | 5.2 | 5.3 | 5.7 | 5.2 | 5.0 |
| hsa-miR-6793-5p | 5.0 | 5.1 | 5.6 | 4.6 | 4.9 | 5.0 | 5.4 | 5.2 | 5.1 |
| hsa-miR-1193 | 6.4 | 6.0 | 6.0 | 6.2 | 5.9 | 6.0 | 5.1 | 5.7 | 6.2 |
| hsa-miR-0637 | 5.4 | 5.8 | 5.4 | 5.8 | 5.3 | 5.8 | 5.5 | 5.5 | 5.2 |
| hsa-miR-6738-3p | 4.7 | 5.0 | 5.4 | 4.6 | 4.2 | 4.8 | 6.1 | 5.3 | 5.0 |
| hsa-miR-0223-5p | 5.8 | 6.3 | 6.3 | 5.2 | 4.5 | 3.8 | 4.2 | 3.6 | 3.1 |
| hsa-miR-3940-3p | 5.7 | 5.8 | 5.8 | 5.6 | 5.5 | 5.4 | 5.1 | 5.0 | 5.0 |
| hsa-miR-6893-3p | 5.4 | 5.7 | 5.6 | 5.2 | 5.3 | 5.5 | 5.0 | 5.2 | 5.3 |
| hsa -miR -0342-5p | 5.3 | 5.4 | 5.2 | 5.1 | 4.4 | 4.5 | 5.9 | 6.1 | 6.4 |
| hsa-miR-1307-3p | 5.9 | 5.4 | 5.5 | 5.2 | 5.5 | 5.4 | 5.2 | 5.6 | 5.6 |
| hsa-miR-3121-5p | 0.0 | 0.0 | 1.8 | 0.0 | 0.0 | 0.0 | 0.0 | 0.0 | 0.0 |
| hsa-miR-6131 | 5.4 | 5.9 | 4.9 | 4.9 | 5.5 | 5.9 | 6.1 | 9.4 | 6.5 |
| hsa-miR-0485-3p | 5.4 | 5.3 | 5.6 | 5.5 | 5.4 | 5.2 | 5.4 | 4.8 | 5.1 |
| hsa -miR -5088-5p | 5.6 | 5.8 | 5.7 | 5.9 | 5.7 | 6.0 | 5.4 | 4.9 | 5.0 |
| hsa-miR-0642a-5p | 5.7 | 6.0 | 6.1 | 5.7 | 5.6 | 6.1 | 5.3 | 4.5 | 4.9 |
| hsa-miR-1470 | 5.9 | 6.4 | 6.0 | 5.6 | 5.6 | 5.4 | 5.8 | 5.4 | 5.8 |
| hsa-miR-3129-3p | 3.4 | 2.9 | 2.5 | 2.8 | 1.8 | 2.5 | 2.0 | 2.1 | 2.9 |
| hsa -miR -5002-3p | 4.7 | 5.2 | 4.9 | 4.5 | 4.0 | 4.5 | 4.5 | 5.0 | 4.8 |
| hsa-miR-0664b-3p | 6.0 | 5.6 | 6.1 | 5.7 | 5.4 | 5.4 | 5.4 | 5.0 | 5.3 |
| hsa-miR-4322 | 6.3 | 6.0 | 6.0 | 5.6 | 5.7 | 6.0 | 5.2 | 6.1 | 5.4 |
| hsa-miR-4535 | 5.9 | 5.9 | 5.4 | 5.3 | 5.5 | 5.3 | 4.6 | 6.2 | 5.4 |
| hsa-miR-1233-3p | 6.1 | 6.4 | 6.2 | 5.7 | 5.6 | 5.5 | 4.8 | 4.5 | 4.8 |
| hsa-miR-6825-3p | 6.0 | 6.1 | 5.8 | 5.6 | 5.7 | 5.8 | 4.9 | 4.9 | 4.8 |
| hsa-miR-6769b-3p | 5.6 | 5.9 | 6.1 | 5.5 | 5.5 | 5.7 | 5.5 | 4.9 | 5.1 |
| hsa-miR-4290 | 5.5 | 5.9 | 5.6 | 5.5 | 5.4 | 5.6 | 5.0 | 4.9 | 5.1 |
| hsa-miR-0874-3p | 6.5 | 6.1 | 5.9 | 8.0 | 7.3 | 7.1 | 5.4 | 5.4 | 7.0 |
| hsa -miR -0532-3p | 5.8 | 6.2 | 5.7 | 6.0 | 5.4 | 5.8 | 5.1 | 5.0 | 5.0 |
| hsa-miR-6744-3p | 5.7 | 6.0 | 6.5 | 5.8 | 6.0 | 5.7 | 5.5 | 5.1 | 5.7 |
| hsa-miR-0030d-5p | 5.9 | 5.7 | 6.3 | 5.4 | 5.4 | 4.9 | 6.2 | 5.8 | 6.0 |
| hsa-miR-3652 | 5.9 | 5.7 | 5.3 | 5.4 | 5.9 | 6.4 | 5.0 | 6.6 | 6.2 |
| hsa-miR-4710 | 5.5 | 6.1 | 6.0 | 5.7 | 5.2 | 5.5 | 5.6 | 6.2 | 5.4 |
| hsa-miR-5195-5p | 5.0 | 5.7 | 5.9 | 5.4 | 5.3 | 5.2 | 6.7 | 5.7 | 6.2 |
| hsa-miR-6812-3p | 6.0 | 6.5 | 6.4 | 5.7 | 5.5 | 5.4 | 6.3 | 5.7 | 5.9 |
| hsa-let-7b-3p | 5.6 | 5.7 | 5.9 | 5.5 | 5.2 | 5.4 | 6.1 | 5.3 | 5.6 |
| hsa-miR-0483-3p | 6.0 | 5.8 | 6.1 | 5.8 | 5.8 | 5.7 | 5.4 | 5.0 | 5.2 |
| hsa-miR-7106-3p | 5.6 | 6.0 | 6.2 | 5.6 | 5.5 | 5.4 | 5.6 | 5.2 | 5.1 |
| hsa-miR-0199a-5p | 5.6 | 5.6 | 5.7 | 5.4 | 5.1 | 4.8 | 5.5 | 4.9 | 5.0 |
| hsa-miR-0188-5p | 6.6 | 6.9 | 7.0 | 6.1 | 6.1 | 6.0 | 5.9 | 6.8 | 6.3 |
| hsa-miR-6737-3p | 5.7 | 5.6 | 6.0 | 5.7 | 5.4 | 5.4 | 6.1 | 5.6 | 5.9 |
| hsa-miR-6796-5p | 5.3 | 5.6 | 5.5 | 5.4 | 5.1 | 5.5 | 5.3 | 8.4 | 6.6 |
| hsa-miR-7843-5p | 5.8 | 5.8 | 6.6 | 5.7 | 6.0 | 6.2 | 6.0 | 6.2 | 5.9 |
| hsa-miR-4498 | 4.9 | 5.7 | 5.8 | 5.3 | 5.1 | 5.6 | 5.0 | 5.5 | 5.7 |
| hsa-miR-5010-3p | 5.3 | 5.7 | 5.7 | 5.4 | 5.3 | 5.3 | 6.2 | 5.3 | 5.7 |
| hsa-miR-8060 | 5.2 | 5.3 | 5.9 | 5.7 | 5.9 | 5.6 | 5.7 | 6.2 | 6.2 |
| hsa-miR-0487b-5p | 5.0 | 5.9 | 5.4 | 5.3 | 5.0 | 5.2 | 5.5 | 4.9 | 5.1 |
| hsa-miR-6831-3p | 5.2 | 5.7 | 6.0 | 6.4 | 5.8 | 5.8 | 5.6 | 5.0 | 5.5 |
| hsa -miR -0320a | 6.5 | 6.7 | 6.7 | 6.5 | 5.9 | 6.0 | 6.3 | 5.6 | 6.0 |
| hsa-miR-6771-3p | 5.5 | 5.4 | 5.7 | 5.5 | 5.1 | 5.5 | 6.4 | 5.7 | 6.1 |
| hsa-miR-6511a-3p | 5.1 | 5.9 | 6.0 | 5.5 | 5.8 | 5.8 | 5.9 | 5.4 | 5.4 |
| hsa-miR-0374c-3p | 6.4 | 6.6 | 7.1 | 6.2 | 6.0 | 6.0 | 5.2 | 4.0 | 3.8 |
| hsa-miR-8052 | 6.0 | 6.2 | 5.9 | 6.0 | 6.0 | 6.1 | 5.6 | 5.9 | 5.6 |
| hsa-miR-4531 | 2.2 | 2.6 | 3.9 | 2.4 | 3.9 | 3.2 | 5.7 | 6.0 | 6.3 |
| hsa-miR-6790-5p | 5.6 | 6.4 | 5.8 | 6.0 | 5.7 | 6.3 | 5.6 | 6.5 | 6.0 |
| hsa-miR-6883-3p | 4.9 | 5.4 | 5.6 | 5.3 | 5.1 | 5.0 | 5.6 | 5.0 | 5.2 |
| hsa-miR-4655-3p | 6.6 | 6.2 | 5.7 | 6.2 | 5.6 | 4.3 | 5.0 | 5.0 | 5.3 |
| hsa-miR-5189-3p | 5.4 | 5.6 | 5.9 | 5.1 | 4.8 | 5.8 | 5.5 | 5.1 | 5.2 |
| hsa-miR-6753-3p | 5.7 | 5.5 | 5.8 | 5.0 | 4.7 | 5.4 | 5.6 | 5.2 | 5.2 |
| hsa-miR-4455 | 7.0 | 6.5 | 6.1 | 5.1 | 5.1 | 5.2 | 6.1 | 5.4 | 5.5 |
| hsa-miR-3605-3p | 5.6 | 5.7 | 5.5 | 5.3 | 4.9 | 5.0 | 5.4 | 5.1 | 5.1 |
| hsa-miR-4642 | 5.6 | 5.8 | 5.9 | 5.6 | 5.3 | 5.2 | 6.1 | 5.6 | 5.7 |
| hsa-miR-7160-5p | 5.4 | 5.6 | 6.1 | 5.0 | 5.6 | 5.5 | 5.8 | 6.0 | 5.8 |
| hsa -miR -5008-5p | 6.8 | 6.4 | 6.3 | 5.8 | 5.6 | 5.7 | 5.2 | 4.8 | 4.1 |
| hsa-miR-1292-3p | 6.4 | 7.1 | 6.6 | 6.1 | 6.1 | 5.7 | 6.1 | 5.4 | 6.0 |
| hsa-miR-6753-5p | 6.1 | 6.6 | 6.6 | 5.9 | 6.4 | 6.1 | 5.5 | 5.4 | 5.6 |
| hsa-miR-4649-3p | 5.7 | 6.0 | 6.1 | 5.7 | 5.6 | 5.7 | 6.3 | 5.6 | 6.0 |
| hsa-miR-7846-3p | 5.6 | 6.1 | 5.9 | 6.1 | 6.6 | 6.8 | 6.7 | 7.1 | 6.7 |
| hsa-miR-0936 | 7.0 | 6.9 | 6.6 | 6.1 | 6.3 | 5.6 | 5.9 | 5.9 | 5.9 |
| hsa -miR -0504-3p | 5.6 | 6.1 | 5.7 | 5.3 | 5.4 | 6.1 | 5.5 | 5.6 | 5.9 |
| hsa-miR-4746-3p | 6.7 | 6.8 | 6.3 | 6.1 | 6.6 | 6.8 | 5.6 | 6.0 | 6.1 |
| hsa-miR-6833-3p | 5.9 | 5.7 | 5.8 | 5.7 | 5.6 | 5.7 | 5.2 | 5.4 | 5.3 |
| hsa-miR-4279 | 6.1 | 6.3 | 6.1 | 6.1 | 5.9 | 6.4 | 5.7 | 5.4 | 5.9 |
| hsa-miR-6776-5p | 5.3 | 5.3 | 5.1 | 5.2 | 6.0 | 6.3 | 5.6 | 7.1 | 6.1 |
| hsa-miR-6748-3p | 5.3 | 5.7 | 6.0 | 5.4 | 5.3 | 5.5 | 5.6 | 5.2 | 5.3 |
| hsa-miR-4721 | 5.5 | 5.8 | 5.7 | 5.4 | 4.9 | 5.5 | 5.4 | 6.5 | 5.8 |
| hsa-miR-0185-3p | 6.2 | 5.6 | 5.5 | 5.2 | 5.4 | 5.3 | 5.3 | 7.9 | 6.6 |
| hsa-miR-6735-3p | 5.9 | 6.3 | 6.4 | 6.1 | 6.0 | 6.0 | 6.0 | 5.4 | 5.6 |
| hsa-miR-4287 | 6.5 | 6.2 | 7.3 | 6.2 | 5.9 | 6.3 | 5.8 | 5.1 | 5.3 |
| hsa-miR-3173-5p | 4.8 | 5.2 | 5.5 | 4.8 | 4.6 | 4.9 | 5.7 | 5.4 | 5.3 |
| hsa-miR-6782-3p | 5.7 | 6.1 | 6.7 | 5.8 | 5.6 | 5.9 | 6.7 | 5.9 | 6.3 |
| hsa-miR-7112-5p | 6.5 | 6.9 | 6.2 | 5.7 | 5.6 | 6.3 | 4.7 | 5.1 | 5.3 |
| hsa-miR-6794-3p | 6.1 | 6.2 | 6.5 | 5.8 | 5.9 | 5.9 | 5.9 | 5.1 | 5.5 |
| hsa-miR-6757-3p | 5.7 | 6.0 | 6.2 | 5.8 | 5.3 | 5.7 | 6.6 | 5.9 | 6.2 |
| hsa-miR-3622a-3p | 6.1 | 5.8 | 5.9 | 5.4 | 5.3 | 5.2 | 5.0 | 5.4 | 5.1 |
| hsa-miR-6726-3p | 6.2 | 6.5 | 6.3 | 5.9 | 6.1 | 6.0 | 5.5 | 5.5 | 5.1 |
| hsa-miR-0642b-5p | 5.6 | 5.7 | 6.3 | 5.8 | 5.7 | 5.9 | 6.0 | 5.3 | 5.3 |
| hsa-miR-6809-3p | 5.2 | 5.6 | 5.7 | 5.4 | 5.3 | 5.5 | 5.3 | 5.4 | 5.4 |
| hsa-miR-6857-5p | 6.3 | 6.4 | 6.0 | 5.5 | 6.4 | 6.1 | 5.5 | 5.7 | 5.2 |
| hsa-miR-0197-3p | 5.9 | 6.0 | 6.0 | 5.8 | 5.7 | 5.4 | 5.5 | 5.3 | 5.2 |
| hsa-miR-0149-5p | 5.8 | 5.8 | 5.9 | 5.7 | 5.9 | 5.6 | 5.2 | 5.3 | 5.3 |
| hsa-miR-4485-5p | 6.1 | 6.2 | 6.2 | 5.8 | 5.8 | 5.9 | 5.5 | 5.5 | 5.4 |
| hsa-miR-0939-3p | 5.9 | 6.4 | 6.4 | 5.9 | 6.1 | 6.0 | 6.1 | 5.5 | 5.7 |
| hsa-miR-6780b-3p | 5.5 | 5.8 | 6.0 | 5.6 | 5.5 | 5.8 | 6.1 | 5.5 | 5.6 |
| hsa-miR-6748-5p | 5.1 | 5.6 | 5.3 | 5.9 | 6.1 | 5.6 | 6.5 | 8.2 | 7.0 |
| hsa-miR-6751-3p | 5.9 | 6.3 | 6.2 | 6.0 | 6.0 | 6.0 | 5.7 | 5.2 | 5.4 |
| hsa-miR-4736 | 6.1 | 5.5 | 6.1 | 5.3 | 6.2 | 6.1 | 5.4 | 5.7 | 6.2 |
| hsa-miR-6778-3p | 5.2 | 5.6 | 6.1 | 5.6 | 5.2 | 5.2 | 5.8 | 5.4 | 5.6 |
| hsa-miR-6827-3p | 5.6 | 5.9 | 6.1 | 5.8 | 5.4 | 5.7 | 6.1 | 5.6 | 5.9 |
| hsa-miR-5698 | 5.6 | 5.9 | 6.2 | 7.1 | 7.0 | 8.1 | 6.8 | 6.9 | 7.4 |
| hsa-miR-0371b-5p | 6.9 | 6.8 | 5.9 | 5.8 | 5.8 | 7.1 | 4.6 | 5.9 | 5.4 |
| hsa-miR-4714-5p | 5.9 | 5.9 | 5.9 | 5.7 | 5.5 | 5.6 | 6.4 | 5.6 | 6.0 |
| hsa-miR-6772-5p | 5.3 | 5.5 | 5.3 | 4.8 | 5.2 | 5.4 | 5.2 | 6.3 | 5.9 |
| hsa-miR-4462 | 6.1 | 6.2 | 6.5 | 7.2 | 6.0 | 7.0 | 5.8 | 5.7 | 5.6 |
| hsa-miR-6793-3p | 5.9 | 6.1 | 6.4 | 5.8 | 5.7 | 5.8 | 6.9 | 6.2 | 6.6 |
| hsa-miR-0328-3p | 6.2 | 6.3 | 6.3 | 6.2 | 5.9 | 6.1 | 5.9 | 5.2 | 5.5 |
| hsa-miR-3202 | 6.2 | 5.4 | 6.3 | 5.5 | 6.9 | 5.6 | 6.3 | 5.8 | 6.0 |
| hsa-miR-6867-5p | 5.8 | 6.1 | 6.7 | 5.9 | 6.0 | 6.0 | 5.7 | 5.2 | 5.4 |
| hsa -miR -0302c-5p | 5.9 | 5.3 | 5.8 | 5.8 | 5.5 | 4.8 | 6.2 | 6.5 | 6.6 |
| hsa-miR-6881-5p | 5.1 | 6.3 | 6.8 | 5.4 | 7.4 | 6.3 | 5.8 | 5.6 | 6.2 |
| hsa-miR-0574-5p | 6.2 | 6.2 | 6.3 | 5.9 | 5.8 | 5.6 | 5.9 | 5.5 | 5.8 |
| hsa-miR-1260b | 6.1 | 6.0 | 6.2 | 5.8 | 5.7 | 5.7 | 5.2 | 5.5 | 5.4 |
| hsa -miR -6834-3p | 5.7 | 6.1 | 6.3 | 5.8 | 5.8 | 5.8 | 6.1 | 5.5 | 5.8 |
| hsa-miR-4763-5p | 6.1 | 6.4 | 6.5 | 6.0 | 6.1 | 5.8 | 5.9 | 5.3 | 5.4 |
| hsa-miR-7110-3p | 5.7 | 6.2 | 6.3 | 5.8 | 5.9 | 6.0 | 5.3 | 5.6 | 5.5 |
| hsa-miR-1825 | 6.1 | 6.3 | 6.1 | 6.1 | 5.7 | 6.1 | 5.8 | 5.4 | 5.5 |
| hsa-miR-3134 | 0.0 | 0.0 | 0.0 | 0.0 | 0.0 | 0.0 | 0.0 | 0.0 | 0.0 |
| hsa-miR-3679-5p | 5.5 | 5.8 | 6.0 | 6.3 | 7.8 | 8.5 | 6.4 | 7.8 | 7.7 |
| hsa-miR-0370-3p | 5.7 | 5.4 | 5.6 | 5.8 | 5.7 | 6.2 | 6.3 | 9.1 | 6.8 |
| hsa-miR-0092a-2-5p | 7.9 | 6.3 | 5.2 | 7.4 | 11.3 | 10.8 | 7.7 | 7.8 | 8.5 |
| hsa-miR-7111-3p | 6.3 | 6.6 | 6.3 | 6.0 | 6.2 | 6.4 | 6.0 | 5.6 | 6.1 |
| hsa-miR-3663-5p | 6.0 | 5.9 | 5.8 | 5.4 | 5.3 | 5.4 | 5.8 | 5.9 | 5.7 |
| hsa-miR-0503-3p | 6.5 | 6.6 | 7.1 | 5.6 | 5.8 | 5.6 | 5.5 | 5.1 | 4.6 |
| hsa-miR-6736-5p | 6.0 | 6.7 | 6.9 | 6.0 | 6.0 | 5.1 | 6.0 | 6.4 | 6.1 |
| hsa-miR-3191-5p | 5.6 | 5.6 | 5.9 | 5.4 | 5.2 | 5.5 | 5.7 | 5.1 | 5.2 |
| hsa-miR-5705 | 6.1 | 6.3 | 6.3 | 5.9 | 6.0 | 6.0 | 5.3 | 5.9 | 5.4 |
| hsa-miR-7977 | 5.8 | 5.9 | 6.0 | 5.4 | 5.7 | 5.7 | 5.9 | 5.6 | 5.5 |
| hsa-miR-6840-5p | 6.3 | 6.4 | 6.8 | 5.7 | 6.0 | 5.5 | 6.5 | 6.3 | 6.4 |
| hsa-miR-6865-3p | 6.1 | 6.3 | 6.2 | 6.0 | 6.0 | 6.0 | 5.8 | 5.3 | 5.4 |
| hsa-miR-6754-3p | 5.8 | 6.0 | 6.2 | 5.4 | 5.5 | 5.7 | 6.1 | 5.7 | 5.7 |
| hsa-miR-6830-5p | 5.7 | 5.5 | 5.8 | 5.4 | 6.4 | 5.7 | 5.9 | 6.7 | 5.8 |
| hsa-miR-6736-3p | 5.4 | 5.9 | 6.4 | 5.9 | 6.0 | 5.7 | 5.9 | 5.7 | 5.7 |
| hsa -miR -6842-5p | 6.2 | 6.2 | 6.7 | 6.3 | 6.8 | 7.3 | 6.4 | 8.3 | 6.6 |
| hsa-miR-6761-3p | 6.2 | 6.0 | 6.2 | 5.6 | 5.3 | 5.6 | 6.0 | 5.5 | 5.7 |
| hsa-miR-1236-3p | 6.0 | 6.4 | 6.4 | 6.0 | 5.9 | 6.3 | 6.6 | 5.8 | 6.2 |
| hsa-miR-5196-3p | 6.3 | 6.2 | 6.4 | 6.2 | 5.8 | 6.0 | 5.6 | 5.5 | 5.5 |
| hsa-miR-1234-3p | 6.3 | 6.7 | 6.5 | 6.2 | 6.0 | 6.2 | 6.4 | 5.7 | 6.0 |
| hsa-miR-3925-5p | 5.5 | 5.4 | 5.8 | 5.5 | 5.9 | 5.6 | 6.2 | 6.4 | 6.5 |
| hsa -miR -6872-5p | 4.7 | 4.3 | 4.7 | 4.6 | 5.6 | 4.7 | 6.6 | 6.1 | 6.9 |
| hsa-miR-4483 | 5.9 | 6.1 | 6.4 | 5.8 | 5.9 | 6.6 | 6.0 | 6.8 | 6.2 |
| hsa-miR-3917 | 5.8 | 6.1 | 5.9 | 6.7 | 6.3 | 6.1 | 8.3 | 10.5 | 9.1 |
| hsa-miR-6877-3p | 6.5 | 6.3 | 6.2 | 6.0 | 6.1 | 6.3 | 5.5 | 5.4 | 5.4 |
| hsa -miR -0520e | 4.7 | 3.7 | 4.5 | 3.8 | 4.4 | 2.4 | 6.3 | 6.3 | 6.5 |
| hsa-miR-4436b-5p | 6.5 | 6.5 | 6.4 | 5.8 | 6.0 | 6.3 | 5.6 | 5.8 | 5.7 |
| hsa-miR-4444 | 4.1 | 4.6 | 3.9 | 4.2 | 4.3 | 3.8 | 8.2 | 6.0 | 7.1 |
| hsa-miR-6759-5p | 5.7 | 6.4 | 6.4 | 4.9 | 5.6 | 5.7 | 5.6 | 6.4 | 5.8 |
| hsa-miR-6511a-5p | 5.9 | 6.3 | 6.4 | 5.8 | 6.0 | 6.1 | 6.4 | 7.2 | 6.6 |
| hsa-miR-4430 | 6.4 | 6.4 | 6.2 | 6.2 | 6.6 | 7.3 | 5.9 | 6.1 | 6.1 |
| hsa-miR-6127 | 5.3 | 5.8 | 5.6 | 5.4 | 5.7 | 6.0 | 6.4 | 8.0 | 6.6 |
| hsa-miR-6740-3p | 5.2 | 5.8 | 6.1 | 5.6 | 5.5 | 5.8 | 6.2 | 5.6 | 5.8 |
| hsa-miR-6747-3p | 5.6 | 6.1 | 6.2 | 5.5 | 5.6 | 5.7 | 5.9 | 5.4 | 5.6 |
| hsa-miR-0133b | 6.2 | 6.1 | 6.3 | 6.0 | 5.6 | 5.5 | 6.5 | 5.9 | 6.2 |
| hsa-miR-4440 | 5.9 | 5.6 | 5.8 | 5.2 | 5.0 | 5.2 | 6.0 | 6.8 | 6.0 |
| hsa -miR -0548q | 6.8 | 7.1 | 6.2 | 6.4 | 6.5 | 6.3 | 5.1 | 5.5 | 5.0 |
| hsa-miR-6741-3p | 5.9 | 6.2 | 6.2 | 6.0 | 5.7 | 6.2 | 5.6 | 5.5 | 5.7 |
| hsa-miR-6779-3p | 6.1 | 6.3 | 6.4 | 5.9 | 5.7 | 5.9 | 5.4 | 5.4 | 5.6 |
| hsa-miR-3622b-5p | 5.5 | 5.6 | 6.1 | 6.0 | 7.0 | 6.9 | 6.1 | 6.2 | 6.8 |
| hsa-miR-6787-3p | 6.0 | 6.2 | 6.2 | 5.7 | 5.5 | 5.6 | 5.3 | 5.3 | 5.4 |
| hsa-miR-6873-3p | 6.1 | 6.2 | 6.2 | 5.8 | 5.6 | 5.5 | 5.7 | 5.2 | 5.1 |
| hsa-miR-4713-5p | 6.3 | 6.4 | 6.4 | 6.2 | 6.1 | 6.0 | 5.8 | 6.0 | 5.9 |
| hsa-miR-6742-3p | 5.8 | 6.5 | 6.9 | 6.1 | 5.8 | 6.0 | 6.7 | 6.2 | 6.4 |
| hsa-miR-0423-5p | 7.1 | 7.0 | 7.4 | 6.7 | 6.7 | 7.3 | 6.2 | 5.6 | 5.7 |
| hsa -miR -0204-3p | 5.4 | 4.9 | 4.7 | 6.1 | 6.3 | 9.3 | 6.2 | 6.6 | 6.9 |
| hsa-miR-6823-3p | 6.0 | 6.2 | 6.4 | 6.2 | 6.2 | 5.9 | 5.7 | 5.8 | 5.7 |
| hsa-miR-6877-5p | 5.8 | 6.2 | 6.4 | 5.8 | 6.7 | 7.0 | 6.4 | 7.0 | 6.1 |
| hsa-miR-4695-3p | 5.7 | 6.0 | 6.3 | 5.5 | 5.4 | 5.7 | 5.8 | 5.7 | 5.7 |
| hsa-miR-0018b-3p | 6.4 | 6.4 | 6.4 | 6.2 | 5.6 | 5.8 | 6.4 | 5.8 | 6.1 |
| hsa-miR-1260a | 6.3 | 6.2 | 6.5 | 6.3 | 6.0 | 5.9 | 6.0 | 5.8 | 5.7 |
| hsa-miR-6803-3p | 6.1 | 6.4 | 6.1 | 6.0 | 5.8 | 6.3 | 5.6 | 5.4 | 5.7 |
| hsa-miR-0211-3p | 7.3 | 6.2 | 6.0 | 7.9 | 7.5 | 7.8 | 6.7 | 7.8 | 7.2 |
| hsa-miR-6875-3p | 6.0 | 5.9 | 6.2 | 5.5 | 5.3 | 5.7 | 6.0 | 5.6 | 5.6 |
| hsa -miR -6882-3p | 5.9 | 5.9 | 6.5 | 5.6 | 5.7 | 5.8 | 6.5 | 5.8 | 5.8 |
| hsa-miR-8085 | 5.4 | 6.0 | 5.9 | 6.7 | 6.3 | 6.5 | 6.0 | 5.9 | 6.2 |
| hsa-miR-6750-3p | 5.5 | 6.2 | 6.5 | 5.8 | 5.8 | 5.8 | 5.5 | 5.5 | 5.7 |
| hsa-miR-6879-3p | 6.2 | 6.4 | 6.4 | 6.1 | 6.5 | 6.0 | 5.9 | 5.5 | 5.7 |
| hsa-miR-6867-3p | 6.2 | 6.4 | 6.3 | 5.9 | 5.9 | 6.1 | 6.2 | 5.7 | 5.8 |
| hsa-miR-6890-5p | 6.5 | 6.9 | 7.1 | 6.4 | 6.2 | 5.9 | 6.1 | 5.7 | 5.7 |
| hsa -miR -6894-5p | 6.6 | 6.4 | 6.8 | 6.7 | 6.8 | 7.2 | 6.0 | 7.9 | 6.4 |
| hsa-miR-1306-5p | 6.5 | 6.6 | 6.7 | 6.4 | 6.6 | 6.3 | 6.2 | 6.2 | 6.2 |
| hsa-miR-3121-3p | 0.0 | 0.0 | 0.0 | 0.0 | 0.0 | 0.0 | 0.0 | 0.0 | 0.0 |
| hsa -miR -6886-3p | 5.7 | 6.3 | 6.5 | 5.8 | 6.1 | 6.1 | 5.4 | 5.5 | 6.0 |
| hsa-miR-1267 | 5.7 | 6.1 | 6.3 | 5.9 | 5.7 | 5.8 | 6.9 | 6.4 | 6.5 |
| hsa -miR -6862-3p | 6.1 | 6.4 | 6.6 | 5.9 | 6.1 | 6.2 | 6.0 | 5.6 | 5.8 |
| hsa-miR-4701-5p | 6.1 | 6.2 | 6.4 | 6.1 | 5.8 | 5.8 | 6.6 | 5.8 | 6.3 |
| hsa-miR-6792-5p | 6.9 | 6.8 | 6.9 | 6.7 | 6.4 | 6.4 | 6.0 | 6.2 | 5.8 |
| hsa-miR-4698 | 5.8 | 5.9 | 6.3 | 5.9 | 6.1 | 6.6 | 7.4 | 6.3 | 7.1 |
| hsa-miR-4738-3p | 6.6 | 6.6 | 5.7 | 5.9 | 6.2 | 6.3 | 4.9 | 5.0 | 5.1 |
| hsa-miR-4268 | 6.0 | 6.2 | 6.1 | 5.7 | 5.9 | 6.0 | 5.8 | 5.4 | 5.6 |
| hsa-miR-4664-3p | 6.5 | 6.7 | 6.6 | 6.1 | 5.8 | 6.0 | 6.7 | 6.3 | 6.3 |
| hsa-miR-0877-3p | 6.4 | 6.4 | 6.5 | 6.4 | 6.2 | 6.2 | 5.9 | 5.5 | 6.0 |
| hsa-miR-1304-3p | 6.1 | 6.1 | 6.6 | 6.0 | 5.9 | 5.8 | 6.7 | 6.1 | 6.3 |
| hsa-miR-3118 | 0.0 | 0.0 | 0.0 | 0.0 | 0.0 | 0.0 | 0.0 | 0.0 | 0.0 |
| hsa-miR-6870-3p | 6.1 | 6.5 | 6.8 | 5.9 | 5.8 | 6.1 | 6.7 | 6.3 | 6.5 |
| hsa-miR-0574-3p | 6.6 | 6.8 | 6.7 | 6.7 | 6.4 | 6.1 | 6.1 | 5.7 | 5.8 |
| hsa -miR -4646-3p | 6.0 | 6.4 | 6.6 | 6.2 | 6.1 | 6.4 | 6.4 | 5.7 | 6.0 |
| hsa -miR -6884-3p | 6.2 | 6.3 | 6.9 | 6.1 | 6.5 | 6.3 | 6.7 | 6.1 | 6.4 |
| hsa-miR-0658 | 7.4 | 7.4 | 6.2 | 8.0 | 8.1 | 7.4 | 5.7 | 5.4 | 6.9 |
| hsa-miR-3911 | 6.8 | 6.8 | 7.5 | 7.9 | 9.4 | 7.6 | 6.4 | 5.8 | 6.3 |
| hsa-miR-6743-3p | 6.5 | 6.6 | 6.7 | 6.3 | 6.5 | 6.1 | 6.1 | 5.7 | 5.8 |
| hsa-miR-6870-5p | 6.9 | 6.9 | 6.9 | 7.7 | 7.9 | 8.8 | 7.0 | 6.6 | 7.0 |
| hsa-miR-4685-3p | 6.2 | 6.3 | 6.4 | 6.0 | 5.8 | 6.0 | 6.2 | 5.7 | 6.0 |
| hsa-miR-3191-3p | 7.0 | 7.2 | 7.3 | 6.9 | 6.4 | 6.5 | 6.3 | 7.7 | 6.8 |
| hsa-miR-1281 | 6.8 | 6.8 | 6.7 | 6.6 | 6.6 | 6.5 | 6.3 | 5.6 | 5.8 |
| hsa-miR-4747-5p | 6.4 | 6.4 | 6.8 | 6.5 | 7.2 | 6.6 | 6.4 | 6.3 | 6.4 |
| hsa-miR-0887-3p | 7.5 | 7.3 | 6.1 | 6.7 | 7.1 | 6.9 | 5.7 | 6.1 | 6.7 |
| hsa-miR-4700-3p | 6.2 | 6.3 | 6.6 | 6.1 | 5.9 | 5.9 | 6.8 | 6.4 | 6.4 |
| hsa-miR-6727-3p | 6.7 | 7.4 | 6.7 | 6.4 | 6.4 | 6.3 | 5.5 | 5.8 | 5.8 |
| hsa-miR-5699-5p | 6.3 | 6.5 | 6.6 | 6.4 | 5.9 | 6.5 | 7.1 | 6.2 | 6.7 |
| hsa-miR-0498 | 7.1 | 6.9 | 7.2 | 7.2 | 6.8 | 6.1 | 6.4 | 6.0 | 6.5 |
| hsa-miR-0449b-3p | 6.3 | 6.5 | 6.3 | 6.1 | 5.7 | 6.0 | 5.9 | 5.7 | 6.0 |
| hsa-miR-6735-5p | 7.2 | 7.4 | 7.6 | 7.7 | 7.0 | 7.0 | 6.3 | 6.0 | 6.4 |
| hsa-miR-1343-3p | 6.6 | 6.8 | 6.5 | 6.1 | 6.2 | 6.4 | 8.1 | 8.9 | 8.7 |
| hsa -miR -3126-3p | 3.6 | 3.6 | 4.7 | 4.2 | 4.1 | 3.5 | 3.8 | 3.5 | 3.6 |
| hsa-miR-8071 | 5.6 | 5.7 | 5.8 | 6.0 | 6.0 | 6.9 | 7.2 | 7.5 | 7.0 |
| hsa-miR-1587 | 6.2 | 6.4 | 6.2 | 6.4 | 5.8 | 6.3 | 6.4 | 7.5 | 6.3 |
| hsa-miR-3133 | 0.0 | 0.0 | 0.0 | 0.0 | 0.0 | 0.0 | 0.0 | 0.0 | 1.9 |
| hsa-miR-4793-3p | 5.4 | 5.7 | 6.0 | 4.7 | 5.3 | 5.1 | 4.8 | 4.9 | 5.3 |
| hsa-miR-6730-5p | 6.2 | 5.7 | 6.6 | 6.3 | 6.7 | 6.6 | 7.1 | 6.8 | 7.2 |
| hsa-miR-6076 | 5.6 | 5.8 | 5.4 | 5.9 | 6.6 | 7.8 | 6.7 | 9.2 | 7.8 |
| hsa-miR-8089 | 6.8 | 7.0 | 6.6 | 6.8 | 6.5 | 7.1 | 6.3 | 6.8 | 6.7 |
| hsa-miR-6857-3p | 6.1 | 6.0 | 6.5 | 6.3 | 6.1 | 5.9 | 6.8 | 6.5 | 6.7 |
| hsa-miR-6749-3p | 6.6 | 7.0 | 6.8 | 6.6 | 6.4 | 6.6 | 6.5 | 5.9 | 6.4 |
| hsa-miR-6890-3p | 6.3 | 6.7 | 7.1 | 6.4 | 6.3 | 6.2 | 6.9 | 6.3 | 6.4 |
| hsa-miR-4751 | 6.4 | 6.2 | 6.4 | 6.4 | 6.9 | 6.3 | 6.8 | 7.0 | 6.9 |
| hsa-miR-6825-5p | 6.7 | 6.3 | 6.1 | 6.5 | 8.2 | 8.8 | 7.4 | 6.4 | 7.6 |
| hsa-miR-6810-3p | 6.4 | 6.6 | 6.8 | 6.3 | 6.2 | 6.3 | 7.1 | 6.6 | 6.9 |
| hsa-miR-0365a-3p, hsa-miR-365b-3p | 6.5 | 6.6 | 7.0 | 6.5 | 6.5 | 6.3 | 7.2 | 6.4 | 6.7 |
| hsa-miR-3943 | 6.5 | 6.7 | 7.0 | 6.5 | 5.9 | 6.2 | 6.9 | 6.2 | 6.7 |
| hsa-miR-6728-3p | 6.5 | 6.7 | 6.9 | 6.5 | 6.4 | 6.2 | 6.8 | 6.4 | 6.6 |
| hsa-miR-3185 | 6.9 | 6.6 | 6.3 | 6.1 | 7.4 | 6.8 | 6.0 | 7.0 | 6.9 |
| hsa -miR -6894-3p | 5.8 | 6.6 | 6.8 | 5.9 | 6.0 | 6.3 | 5.8 | 6.0 | 6.0 |
| hsa-miR-8485 | 6.5 | 6.8 | 6.8 | 6.7 | 6.4 | 6.5 | 5.9 | 5.9 | 5.8 |
| hsa -miR -6846-5p | 6.5 | 6.6 | 6.2 | 6.6 | 6.2 | 6.9 | 6.3 | 6.8 | 6.4 |
| hsa-miR-4286 | 6.4 | 6.5 | 6.8 | 6.3 | 6.1 | 6.5 | 7.2 | 6.4 | 6.8 |
| hsa-miR-4259 | 6.1 | 6.5 | 7.1 | 6.2 | 6.4 | 5.7 | 6.4 | 6.8 | 6.5 |
| hsa-miR-0208a-5p | 7.1 | 6.2 | 6.9 | 6.1 | 5.7 | 6.0 | 5.9 | 5.3 | 6.1 |
| hsa-miR-6851-3p | 6.4 | 6.7 | 6.8 | 6.4 | 6.2 | 6.3 | 6.8 | 6.2 | 6.5 |
| hsa-miR-4472 | 7.5 | 6.8 | 7.7 | 6.7 | 7.1 | 7.6 | 6.7 | 7.9 | 6.6 |
| hsa-miR-6820-5p | 6.5 | 6.5 | 5.9 | 6.0 | 6.7 | 7.0 | 6.3 | 6.5 | 6.7 |
| hsa-miR-6855-3p | 6.3 | 6.2 | 6.8 | 6.6 | 6.5 | 6.3 | 7.2 | 6.9 | 7.0 |
| hsa-miR-6801-3p | 6.5 | 6.8 | 6.8 | 6.7 | 6.3 | 6.5 | 6.9 | 6.5 | 6.7 |
| hsa-miR-0634 | 6.3 | 6.2 | 6.6 | 6.3 | 6.0 | 5.8 | 6.9 | 6.5 | 6.8 |
| hsa-miR-1229-3p | 6.2 | 6.8 | 6.9 | 6.2 | 6.0 | 6.3 | 6.0 | 6.0 | 5.9 |
| hsa-miR-7109-3p | 6.5 | 6.5 | 6.6 | 5.9 | 6.2 | 6.3 | 6.3 | 5.9 | 6.0 |
| hsa -miR -6804-3p | 6.3 | 6.5 | 6.7 | 6.2 | 6.1 | 6.3 | 6.4 | 5.8 | 5.9 |
| hsa-miR-4723-3p | 6.5 | 6.7 | 7.2 | 6.7 | 6.7 | 6.7 | 6.8 | 6.1 | 6.3 |
| hsa-miR-6742-5p | 6.3 | 7.2 | 6.3 | 6.5 | 6.2 | 6.4 | 6.9 | 7.2 | 7.1 |
| hsa-miR-6511b-3p | 6.3 | 6.6 | 6.7 | 6.2 | 6.3 | 6.4 | 6.5 | 5.8 | 6.2 |
| hsa-miR-6820-3p | 6.6 | 6.5 | 6.9 | 6.4 | 6.0 | 6.3 | 7.1 | 6.5 | 6.8 |
| hsa-miR-2278 | 6.7 | 7.2 | 7.1 | 7.1 | 6.8 | 6.4 | 6.4 | 6.1 | 6.9 |
| hsa-miR-3157-3p | 0.0 | 3.6 | 3.1 | 2.4 | 0.0 | 2.8 | 2.7 | 0.0 | 2.3 |
| hsa-miR-6776-3p | 6.3 | 6.4 | 6.8 | 6.4 | 6.2 | 6.0 | 6.7 | 6.3 | 6.5 |
| hsa-miR-6730-3p | 6.3 | 6.5 | 6.8 | 6.4 | 6.2 | 6.3 | 6.8 | 6.2 | 6.5 |
| hsa-miR-6797-3p | 6.7 | 7.0 | 7.4 | 6.7 | 6.6 | 6.6 | 7.3 | 6.4 | 6.9 |
| hsa-miR-0920 | 5.5 | 6.2 | 6.5 | 6.1 | 6.1 | 6.2 | 7.9 | 7.9 | 8.0 |
| hsa-miR-4443 | 7.5 | 7.0 | 6.0 | 6.9 | 6.3 | 7.8 | 7.0 | 6.6 | 6.9 |
| hsa-miR-6732-3p | 6.6 | 6.9 | 6.9 | 6.4 | 6.4 | 6.6 | 6.5 | 6.0 | 6.2 |
| hsa-miR-0365a-5p | 7.0 | 7.0 | 6.7 | 7.2 | 6.2 | 7.4 | 6.8 | 6.8 | 6.5 |
| hsa-miR-6780a-5p | 5.2 | 5.5 | 5.7 | 5.6 | 6.0 | 5.7 | 7.5 | 6.0 | 6.9 |
| hsa-miR-6510-5p | 6.7 | 6.9 | 7.2 | 7.1 | 7.9 | 8.7 | 7.4 | 6.4 | 7.6 |
| hsa-miR-6745 | 5.2 | 4.5 | 4.9 | 5.3 | 6.3 | 5.2 | 7.4 | 6.8 | 7.5 |
| hsa-miR-7114-3p | 6.5 | 6.6 | 7.2 | 6.5 | 6.7 | 6.4 | 7.0 | 6.6 | 6.9 |
| hsa-miR-7113-3p | 7.2 | 7.2 | 7.3 | 6.9 | 7.1 | 6.9 | 6.6 | 6.2 | 6.3 |
| hsa-miR-4276 | 7.5 | 7.5 | 6.6 | 6.6 | 7.0 | 7.2 | 5.8 | 5.0 | 5.5 |
| hsa-miR-6726-5p | 7.1 | 6.6 | 6.1 | 5.7 | 7.0 | 6.8 | 6.9 | 7.9 | 7.3 |
| hsa-miR-0125a-3p | 6.9 | 7.1 | 6.9 | 6.9 | 6.7 | 6.7 | 7.4 | 6.2 | 7.3 |
| hsa -miR -0486-5p | 6.5 | 6.5 | 6.9 | 6.6 | 6.4 | 6.1 | 6.8 | 6.5 | 6.7 |
| hsa-miR-4499 | 5.1 | 5.1 | 5.8 | 5.3 | 6.0 | 5.3 | 6.8 | 6.8 | 7.6 |
| hsa-miR-4697-3p | 6.5 | 6.6 | 6.9 | 6.3 | 6.1 | 6.0 | 7.0 | 6.5 | 6.5 |
| hsa-miR-6507-5p | 4.7 | 5.4 | 6.1 | 5.5 | 6.5 | 6.0 | 7.6 | 7.1 | 8.0 |
| hsa-miR-1976 | 6.8 | 6.9 | 7.0 | 6.5 | 6.5 | 6.5 | 6.3 | 6.1 | 6.2 |
| hsa-miR-3147 | 6.6 | 6.6 | 6.6 | 6.5 | 7.0 | 6.9 | 5.9 | 6.5 | 7.5 |
| hsa-miR-0519e-5p | 6.7 | 5.1 | 6.0 | 6.0 | 7.2 | 5.6 | 6.9 | 5.6 | 7.4 |
| hsa-miR-6815-5p | 5.7 | 5.2 | 4.9 | 5.5 | 6.4 | 6.2 | 7.8 | 7.6 | 8.4 |
| hsa-miR-4750-5p | 6.9 | 7.2 | 6.4 | 7.3 | 6.7 | 7.4 | 6.3 | 7.2 | 7.2 |
| hsa-miR-4687-5p | 7.0 | 7.2 | 7.2 | 6.8 | 7.0 | 6.9 | 6.7 | 6.3 | 6.5 |
| hsa -miR -6806-5p | 7.6 | 7.6 | 7.4 | 7.2 | 6.9 | 6.9 | 6.1 | 6.1 | 6.3 |
| hsa-miR-4783-3p | 7.3 | 7.5 | 7.4 | 7.3 | 6.7 | 7.2 | 6.8 | 8.0 | 6.9 |
| hsa-miR-6754-5p | 6.3 | 6.3 | 7.3 | 6.5 | 6.7 | 6.7 | 7.1 | 6.7 | 7.0 |
| hsa-miR-4419a | 6.5 | 6.6 | 7.0 | 7.4 | 7.4 | 7.6 | 7.6 | 7.4 | 7.6 |
| hsa-miR-6769a-3p | 6.7 | 6.9 | 6.9 | 6.6 | 6.5 | 6.7 | 6.8 | 6.3 | 6.5 |
| hsa -miR -6846-3p | 6.5 | 6.8 | 6.9 | 6.4 | 6.2 | 6.1 | 7.1 | 6.4 | 6.5 |
| hsa-miR-4769-3p | 6.7 | 6.9 | 7.2 | 6.6 | 6.6 | 6.6 | 7.0 | 6.2 | 6.6 |
| hsa-miR-0744-5p | 7.0 | 7.3 | 6.9 | 6.6 | 6.6 | 6.7 | 5.8 | 6.0 | 6.4 |
| hsa -miR -3184-3p | 6.9 | 7.0 | 7.3 | 6.7 | 6.7 | 6.3 | 6.9 | 6.6 | 6.7 |
| hsa-miR-6792-3p | 6.8 | 7.0 | 7.3 | 6.7 | 6.6 | 6.6 | 7.0 | 6.6 | 6.6 |
| hsa-miR-0150-3p | 7.6 | 7.5 | 6.9 | 8.3 | 7.0 | 7.3 | 7.3 | 7.8 | 7.9 |
| hsa-miR-7155-5p | 7.5 | 7.8 | 7.6 | 7.4 | 7.5 | 7.5 | 6.6 | 6.4 | 6.0 |
| hsa -miR -6858-3p | 6.6 | 6.8 | 7.1 | 6.7 | 6.7 | 6.5 | 7.3 | 6.8 | 7.1 |
| hsa-miR-4690-5p | 8.0 | 7.8 | 7.5 | 6.9 | 7.1 | 6.6 | 5.8 | 7.0 | 6.4 |
| hsa-miR-6861-3p | 6.8 | 6.8 | 6.9 | 6.6 | 6.7 | 6.7 | 6.8 | 6.4 | 6.8 |
| hsa-miR-6069 | 6.8 | 7.2 | 7.6 | 6.8 | 6.7 | 6.8 | 7.3 | 6.5 | 6.8 |
| hsa-miR-0766-3p | 6.7 | 6.8 | 6.7 | 6.6 | 6.2 | 6.1 | 6.3 | 6.1 | 6.1 |
| hsa-miR-4478 | 6.4 | 6.8 | 7.2 | 7.3 | 8.7 | 6.8 | 6.6 | 6.4 | 7.3 |
| hsa-miR-6827-5p | 5.9 | 6.0 | 6.5 | 6.2 | 6.6 | 6.4 | 7.2 | 6.6 | 7.1 |
| hsa-miR-4284 | 6.9 | 6.7 | 7.4 | 6.6 | 6.4 | 6.5 | 7.5 | 6.9 | 7.2 |
| hsa-miR-7108-3p | 6.5 | 6.7 | 7.2 | 6.5 | 6.7 | 6.7 | 7.4 | 6.8 | 7.0 |
| hsa-miR-6889-3p | 6.8 | 6.9 | 7.4 | 7.0 | 6.6 | 6.6 | 7.3 | 6.8 | 7.1 |
| hsa-miR-6860 | 7.0 | 7.2 | 7.2 | 6.9 | 6.6 | 7.1 | 6.5 | 6.7 | 6.7 |
| hsa -miR -3928-3p | 7.2 | 7.8 | 6.6 | 7.5 | 7.0 | 7.6 | 8.1 | 10.4 | 9.4 |
| hsa-miR-6760-5p | 6.1 | 5.4 | 6.0 | 6.0 | 7.3 | 5.9 | 8.0 | 8.3 | 8.7 |
| hsa-miR-4667-3p | 7.1 | 7.3 | 7.5 | 6.9 | 6.7 | 6.7 | 7.0 | 6.6 | 6.7 |
| hsa-miR-1247-3p | 7.6 | 7.1 | 6.3 | 7.2 | 6.2 | 7.7 | 6.9 | 6.1 | 6.8 |
| hsa-miR-6845-3p | 7.1 | 7.2 | 7.1 | 7.0 | 6.8 | 7.0 | 7.0 | 6.5 | 6.9 |
| hsa-miR-4486 | 8.0 | 7.9 | 7.1 | 7.5 | 7.3 | 7.7 | 6.5 | 7.9 | 7.2 |
| hsa-miR-5572 | 7.2 | 7.0 | 6.6 | 6.9 | 7.5 | 8.1 | 6.3 | 6.8 | 6.8 |
| hsa-miR-0484 | 7.1 | 7.0 | 7.5 | 7.1 | 6.7 | 6.5 | 7.1 | 6.5 | 6.7 |
| hsa-miR-6760-3p | 6.9 | 7.0 | 7.6 | 6.6 | 6.8 | 6.5 | 7.5 | 7.1 | 7.3 |
| hsa-miR-6885-3p | 6.6 | 6.9 | 7.0 | 6.5 | 6.4 | 6.5 | 7.3 | 6.8 | 7.1 |
| hsa -miR -6892-3p | 6.8 | 6.8 | 7.4 | 6.6 | 6.7 | 6.7 | 7.4 | 6.7 | 7.0 |
| hsa-miR-2355-5p | 6.5 | 6.5 | 6.9 | 7.1 | 6.2 | 6.2 | 7.3 | 6.5 | 7.1 |
| hsa -miR -3158-3p | 3.7 | 4.2 | 4.4 | 4.5 | 3.9 | 4.6 | 4.2 | 3.6 | 4.0 |
| hsa-miR-6759-3p | 6.8 | 7.1 | 7.2 | 6.7 | 6.7 | 6.6 | 7.1 | 6.8 | 6.9 |
| hsa-miR-0210-5p | 7.1 | 7.0 | 7.5 | 7.2 | 7.0 | 6.9 | 7.0 | 6.8 | 6.7 |
| hsa-miR-6778-5p | 6.7 | 6.9 | 6.8 | 6.9 | 7.4 | 7.0 | 6.8 | 7.5 | 7.5 |
| hsa-miR-2110 | 7.3 | 7.3 | 7.3 | 8.2 | 7.5 | 8.0 | 7.1 | 7.7 | 7.9 |
| hsa-miR-3150a-5p | 3.5 | 3.7 | 3.5 | 3.0 | 1.9 | 2.5 | 2.9 | 2.6 | 3.1 |
| hsa-miR-6731-5p | 6.9 | 7.1 | 7.0 | 7.2 | 7.0 | 6.8 | 6.5 | 6.6 | 7.0 |
| hsa-miR-6756-3p | 6.7 | 7.0 | 7.5 | 6.8 | 7.0 | 6.9 | 7.4 | 6.8 | 7.1 |
| hsa-miR-6786-3p | 6.7 | 7.1 | 7.3 | 6.7 | 6.7 | 6.5 | 7.4 | 6.7 | 6.9 |
| hsa-miR-6831-5p | 6.8 | 7.0 | 7.1 | 7.7 | 8.7 | 9.0 | 7.3 | 6.3 | 9.2 |
| hsa -miR -0652-5p | 7.7 | 7.6 | 9.0 | 8.6 | 9.5 | 9.6 | 7.7 | 9.0 | 7.9 |
| hsa -miR -4419b | 6.9 | 6.5 | 8.2 | 6.7 | 7.3 | 6.9 | 7.9 | 9.0 | 7.9 |
| hsa-miR-4685-5p | 7.2 | 7.6 | 7.8 | 8.1 | 7.2 | 6.9 | 7.3 | 7.3 | 7.5 |
| hsa-miR-0939-5p | 7.1 | 7.3 | 6.5 | 6.3 | 7.6 | 8.3 | 7.7 | 9.4 | 8.5 |
| hsa-miR-6819-3p | 6.8 | 6.8 | 6.9 | 6.8 | 6.3 | 6.5 | 7.4 | 6.5 | 6.8 |
| hsa -miR -6826-3p | 6.9 | 7.1 | 7.5 | 6.8 | 6.7 | 6.8 | 7.5 | 6.9 | 7.0 |
| hsa-miR-0030c-2-3p | 6.1 | 5.7 | 7.1 | 5.3 | 6.3 | 4.4 | 8.4 | 8.4 | 8.4 |
| hsa-miR-6132 | 6.5 | 7.0 | 7.5 | 5.8 | 7.0 | 7.6 | 6.6 | 5.7 | 6.1 |
| hsa-miR-6769b-5p | 6.9 | 7.5 | 7.4 | 8.2 | 9.2 | 9.5 | 7.3 | 7.8 | 7.4 |
| hsa -miR -6802-3p | 6.7 | 6.8 | 7.2 | 6.7 | 6.6 | 6.7 | 7.4 | 6.9 | 7.1 |
| hsa-miR-3194-5p | 7.4 | 7.5 | 7.7 | 6.8 | 6.9 | 7.1 | 7.1 | 6.5 | 7.0 |
| hsa-miR-0718 | 8.0 | 7.6 | 7.3 | 6.7 | 6.8 | 6.6 | 7.1 | 6.8 | 6.8 |
| hsa-miR-6861-5p | 7.5 | 7.3 | 6.7 | 7.8 | 7.4 | 7.6 | 9.3 | 11.3 | 10.1 |
| hsa-miR-4313 | 7.0 | 7.0 | 7.2 | 6.5 | 6.2 | 6.2 | 7.5 | 6.6 | 6.9 |
| hsa-miR-6777-5p | 6.9 | 6.4 | 6.7 | 6.5 | 7.4 | 6.8 | 7.6 | 9.1 | 8.0 |
| hsa-miR-6813-5p | 7.1 | 6.7 | 7.1 | 6.5 | 6.8 | 7.1 | 6.6 | 6.3 | 5.9 |
| hsa-miR-4707-3p | 7.4 | 7.2 | 7.3 | 7.0 | 6.9 | 7.0 | 7.2 | 6.6 | 6.8 |
| hsa-miR-7110-5p | 7.5 | 7.6 | 6.7 | 7.0 | 8.2 | 9.4 | 7.4 | 8.1 | 7.9 |
| hsa-miR-4258 | 7.8 | 7.2 | 7.3 | 7.7 | 8.0 | 7.6 | 7.4 | 6.6 | 7.2 |
| hsa-miR-4526 | 7.4 | 7.7 | 6.8 | 7.1 | 6.7 | 6.7 | 5.9 | 6.5 | 5.6 |
| hsa-miR-4758-3p | 7.1 | 7.1 | 7.5 | 7.0 | 6.9 | 6.8 | 7.2 | 6.7 | 6.9 |
| hsa-miR-6889-5p | 7.1 | 7.2 | 7.5 | 6.9 | 8.2 | 7.9 | 7.5 | 8.1 | 8.0 |
| hsa-miR-6752-3p | 7.0 | 7.4 | 7.8 | 6.9 | 7.0 | 6.8 | 7.7 | 7.2 | 7.4 |
| hsa-miR-0711 | 7.3 | 7.6 | 7.0 | 7.6 | 7.3 | 7.9 | 7.1 | 7.1 | 7.0 |
| hsa-miR-6879-5p | 6.4 | 7.0 | 7.2 | 7.4 | 7.8 | 9.4 | 8.2 | 10.1 | 8.8 |
| hsa-miR-8087 | 3.9 | 4.3 | 5.8 | 6.0 | 5.3 | 5.2 | 6.4 | 7.3 | 6.9 |
| hsa-miR-0933 | 5.9 | 5.7 | 7.3 | 6.6 | 6.7 | 6.3 | 7.1 | 5.4 | 6.8 |
| hsa-miR-4507 | 7.8 | 7.6 | 7.2 | 7.5 | 7.2 | 7.1 | 6.9 | 8.3 | 6.9 |
| hsa-miR-4756-5p | 7.2 | 7.3 | 8.1 | 6.7 | 6.9 | 6.4 | 7.1 | 7.0 | 7.1 |
| hsa-miR-1249-5p | 7.2 | 8.4 | 8.5 | 7.9 | 8.3 | 8.8 | 7.1 | 6.4 | 6.9 |
| hsa-miR-0197-5p | 7.2 | 7.5 | 7.9 | 7.6 | 7.6 | 8.1 | 7.7 | 8.8 | 7.9 |
| hsa-miR-4323 | 7.5 | 7.4 | 7.4 | 7.2 | 6.8 | 6.9 | 7.7 | 7.0 | 7.5 |
| hsa-miR-0092b-5p | 8.5 | 8.0 | 7.1 | 8.3 | 9.1 | 9.8 | 7.3 | 8.7 | 7.7 |
| hsa-miR-6887-3p | 7.2 | 7.4 | 7.7 | 7.2 | 7.2 | 7.1 | 7.6 | 7.3 | 7.5 |
| hsa-miR-6893-5p | 6.6 | 6.4 | 6.5 | 7.2 | 7.2 | 8.3 | 8.0 | 10.7 | 8.8 |
| hsa-miR-6813-3p | 7.4 | 7.5 | 7.7 | 7.3 | 7.0 | 7.3 | 7.9 | 7.0 | 7.5 |
| hsa-miR-6891-3p | 7.0 | 7.2 | 7.6 | 7.0 | 7.0 | 7.0 | 7.8 | 7.3 | 7.6 |
| hsa-miR-6731-3p | 7.1 | 7.2 | 7.4 | 7.0 | 6.7 | 6.7 | 7.8 | 7.2 | 7.6 |
| hsa-miR-0345-3p | 9.2 | 8.5 | 7.5 | 8.7 | 8.7 | 8.1 | 7.8 | 7.8 | 7.8 |
| hsa-miR-4640-3p | 7.4 | 7.5 | 7.9 | 7.4 | 7.0 | 7.0 | 7.7 | 6.9 | 7.2 |
| hsa-miR-4716-3p | 7.3 | 7.0 | 7.0 | 6.9 | 7.5 | 7.2 | 7.1 | 7.5 | 6.7 |
| hsa-miR-0371b-3p | 7.5 | 7.6 | 7.9 | 7.3 | 7.1 | 7.1 | 7.6 | 7.3 | 7.3 |
| hsa-miR-6812-5p | 7.5 | 8.1 | 8.0 | 9.0 | 8.6 | 8.6 | 7.7 | 7.6 | 7.8 |
| hsa-miR-6784-3p | 7.2 | 7.2 | 7.8 | 7.0 | 7.0 | 6.9 | 7.9 | 7.5 | 7.6 |
| hsa-miR-6716-5p | 8.0 | 8.7 | 8.5 | 8.3 | 8.1 | 8.4 | 7.2 | 6.5 | 6.9 |
| hsa-miR-4513 | 7.6 | 7.6 | 7.9 | 6.8 | 7.8 | 7.7 | 7.7 | 8.1 | 7.5 |
| hsa -miR -6848-3p | 7.5 | 7.8 | 8.1 | 7.2 | 7.4 | 7.2 | 7.8 | 7.3 | 7.5 |
| hsa-miR-1238-5p | 7.3 | 7.4 | 7.5 | 7.4 | 7.2 | 7.9 | 7.9 | 7.8 | 7.9 |
| hsa-miR-4530 | 8.2 | 8.4 | 7.6 | 8.4 | 8.7 | 10.4 | 7.6 | 8.4 | 8.5 |
| hsa-miR-4655-5p | 7.6 | 7.7 | 7.3 | 8.1 | 7.6 | 8.6 | 6.8 | 8.0 | 7.4 |
| hsa-miR-6785-3p | 7.4 | 7.5 | 7.9 | 7.4 | 7.1 | 7.2 | 8.2 | 7.6 | 8.0 |
| hsa-miR-4728-3p | 7.5 | 7.4 | 7.5 | 7.4 | 6.9 | 7.3 | 7.9 | 7.2 | 7.5 |
| hsa-miR-5090 | 8.0 | 8.0 | 6.8 | 7.7 | 7.8 | 7.8 | 7.0 | 7.4 | 7.2 |
| hsa -miR -6824-5p | 7.7 | 7.6 | 8.4 | 8.2 | 8.4 | 8.8 | 7.5 | 7.4 | 8.3 |
| hsa-miR-4667-5p | 7.1 | 7.6 | 7.7 | 7.2 | 7.6 | 7.6 | 7.8 | 8.0 | 7.8 |
| hsa-miR-0874-5p | 7.3 | 7.5 | 7.9 | 7.4 | 7.1 | 7.2 | 7.9 | 7.3 | 7.5 |
| hsa-miR-4749-5p | 8.1 | 8.2 | 7.8 | 8.8 | 8.7 | 9.5 | 7.1 | 7.4 | 7.9 |
| hsa-miR-6086 | 8.1 | 8.1 | 8.0 | 8.3 | 8.5 | 7.9 | 7.7 | 8.3 | 8.2 |
| hsa-miR-4257 | 6.7 | 6.8 | 7.1 | 7.4 | 6.9 | 7.4 | 9.5 | 10.8 | 9.8 |
| hsa-miR-1224-3p | 7.8 | 7.6 | 7.9 | 7.7 | 7.4 | 7.5 | 8.0 | 7.2 | 7.6 |
| hsa-miR-3610 | 8.2 | 8.0 | 8.0 | 7.6 | 8.3 | 7.9 | 8.0 | 9.6 | 9.7 |
| hsa-miR-6880-5p | 7.8 | 8.0 | 8.1 | 7.8 | 9.0 | 9.0 | 7.9 | 7.7 | 7.5 |
| hsa-miR-0663b | 8.2 | 8.5 | 8.2 | 7.1 | 6.9 | 6.7 | 6.8 | 7.4 | 5.6 |
| hsa-miR-1237-3p | 7.3 | 7.7 | 7.7 | 7.3 | 7.2 | 7.3 | 8.1 | 7.4 | 7.8 |
| hsa-miR-1225-3p | 7.8 | 8.0 | 7.9 | 7.6 | 7.2 | 7.5 | 8.2 | 7.3 | 7.8 |
| hsa-miR-6763-5p | 7.3 | 7.5 | 7.5 | 7.0 | 7.6 | 7.7 | 7.7 | 10.1 | 8.2 |
| hsa-miR-4707-5p | 8.2 | 8.9 | 7.6 | 7.8 | 8.5 | 8.7 | 6.9 | 6.9 | 7.2 |
| hsa-miR-3195 | 8.9 | 8.8 | 8.4 | 8.1 | 8.4 | 8.4 | 7.5 | 7.9 | 8.0 |
| hsa-miR-1231 | 8.1 | 8.0 | 7.2 | 6.2 | 6.3 | 6.3 | 6.4 | 6.3 | 5.6 |
| hsa-miR-4695-5p | 8.1 | 8.4 | 7.6 | 7.7 | 8.2 | 8.4 | 7.2 | 7.7 | 7.7 |
| hsa-miR-1225-5p | 8.6 | 8.5 | 8.0 | 8.0 | 7.9 | 8.4 | 6.9 | 7.3 | 6.9 |
| hsa-miR-6891-5p | 7.4 | 7.8 | 8.8 | 8.6 | 9.0 | 9.1 | 9.5 | 9.4 | 9.2 |
| hsa-miR-3188 | 8.8 | 9.1 | 9.5 | 9.5 | 9.0 | 9.1 | 7.3 | 7.2 | 6.9 |
| hsa-miR-1202 | 8.7 | 9.1 | 9.3 | 9.4 | 9.8 | 10.4 | 8.2 | 8.3 | 8.0 |
| hsa-miR-6807-5p | 7.3 | 7.2 | 8.5 | 8.7 | 9.4 | 9.7 | 8.4 | 8.4 | 8.0 |
| hsa-miR-6738-5p | 8.3 | 8.2 | 8.4 | 8.1 | 8.2 | 7.7 | 7.2 | 7.4 | 7.0 |
| hsa-miR-1238-3p | 8.0 | 7.9 | 8.0 | 7.7 | 7.3 | 7.2 | 8.2 | 7.7 | 7.9 |
| hsa-miR-6840-3p | 8.1 | 7.9 | 7.6 | 7.8 | 8.0 | 8.0 | 7.8 | 10.3 | 7.9 |
| hsa-miR-0583 | 7.6 | 6.8 | 6.3 | 6.8 | 7.4 | 6.4 | 8.1 | 7.6 | 8.3 |
| hsa-miR-4640-5p | 7.9 | 8.3 | 7.8 | 7.3 | 7.7 | 8.4 | 7.5 | 7.9 | 7.3 |
| hsa-miR-0518c-5p | 6.9 | 6.7 | 7.2 | 6.9 | 7.2 | 6.0 | 7.3 | 6.7 | 7.3 |
| hsa-miR-7108-5p | 8.9 | 8.6 | 8.0 | 8.6 | 8.8 | 8.8 | 7.8 | 8.0 | 8.1 |
| hsa-miR-4725-3p | 7.5 | 7.3 | 7.8 | 8.7 | 10.3 | 10.6 | 7.9 | 8.7 | 9.5 |
| hsa-miR-4497 | 8.7 | 8.7 | 7.8 | 7.7 | 8.1 | 9.6 | 7.8 | 8.8 | 8.6 |
| hsa-miR-0361-3p | 7.7 | 7.8 | 8.3 | 7.4 | 7.3 | 7.1 | 8.3 | 7.9 | 8.1 |
| hsa-miR-5001-5p | 8.2 | 8.6 | 7.6 | 8.3 | 7.8 | 7.6 | 9.6 | 10.8 | 10.0 |
| hsa-miR-6880-3p | 7.7 | 8.1 | 8.5 | 7.7 | 7.8 | 7.5 | 8.3 | 7.9 | 8.2 |
| hsa-miR-0659-3p | 8.7 | 9.5 | 9.8 | 8.3 | 8.1 | 7.7 | 7.4 | 6.8 | 6.3 |
| hsa -miR -6848-5p | 8.5 | 8.8 | 8.9 | 8.4 | 8.5 | 8.5 | 7.6 | 8.0 | 7.3 |
| hsa-miR-6124 | 8.3 | 8.4 | 8.3 | 9.2 | 9.4 | 10.2 | 8.1 | 7.3 | 8.0 |
| hsa-miR-4750-3p | 7.4 | 7.5 | 8.1 | 7.3 | 7.4 | 7.2 | 8.5 | 7.6 | 8.1 |
| hsa-miR-4665-3p | 8.1 | 8.0 | 7.9 | 7.5 | 7.3 | 7.2 | 8.3 | 7.4 | 7.8 |
| hsa-miR-0373-5p | 8.6 | 9.0 | 9.0 | 9.2 | 8.2 | 8.6 | 8.2 | 7.5 | 7.5 |
| hsa-miR-0128-1-5p | 8.9 | 8.3 | 8.4 | 8.3 | 8.5 | 8.3 | 7.2 | 7.5 | 6.9 |
| hsa-miR-4433b-5p | 7.8 | 7.7 | 8.2 | 7.6 | 7.4 | 7.4 | 8.7 | 7.9 | 8.3 |
| hsa-miR-4433a-3p | 8.6 | 8.8 | 8.3 | 9.1 | 8.2 | 8.6 | 7.6 | 9.4 | 8.0 |
| hsa -miR -6808-5p | 8.8 | 8.9 | 8.9 | 8.9 | 8.6 | 8.9 | 8.1 | 9.6 | 7.9 |
| hsa-miR-6835-5p | 8.8 | 9.7 | 10.4 | 7.0 | 8.3 | 7.6 | 8.9 | 9.0 | 7.3 |
| hsa-miR-5196-5p | 7.8 | 7.4 | 7.4 | 7.9 | 9.0 | 7.8 | 8.8 | 9.3 | 9.4 |
| hsa-miR-6075 | 9.0 | 8.4 | 7.2 | 7.0 | 7.2 | 7.6 | 7.0 | 6.8 | 7.1 |
| hsa-miR-6737-5p | 8.6 | 8.7 | 7.8 | 7.3 | 8.0 | 7.9 | 7.1 | 7.3 | 6.5 |
| hsa-miR-6780b-5p | 7.4 | 7.4 | 7.1 | 9.6 | 10.2 | 11.1 | 7.9 | 9.1 | 9.7 |
| hsa-miR-6732-5p | 8.5 | 8.9 | 8.0 | 8.0 | 8.6 | 8.9 | 7.8 | 8.8 | 8.4 |
| hsa-miR-6865-5p | 8.2 | 8.6 | 8.9 | 7.9 | 8.8 | 7.3 | 8.3 | 8.2 | 9.1 |
| hsa-miR-4665-5p | 8.7 | 10.0 | 8.2 | 8.5 | 8.3 | 8.2 | 7.7 | 8.1 | 8.3 |
| hsa-miR-2116-3p | 7.7 | 8.0 | 8.2 | 7.7 | 7.5 | 7.7 | 8.6 | 7.6 | 8.2 |
| hsa -miR -3152-5p | 3.8 | 4.4 | 4.7 | 3.2 | 1.8 | 3.7 | 4.0 | 2.9 | 3.3 |
| hsa-miR-8059 | 9.0 | 10.0 | 7.2 | 10.7 | 8.9 | 9.4 | 6.5 | 7.0 | 7.4 |
| hsa-miR-6851-5p | 8.6 | 8.3 | 9.2 | 8.5 | 8.7 | 8.8 | 8.2 | 7.5 | 7.3 |
| hsa-miR-4788 | 7.6 | 7.8 | 9.2 | 7.9 | 8.2 | 8.2 | 8.7 | 8.2 | 8.5 |
| hsa-miR-4447 | 8.8 | 9.2 | 8.8 | 8.7 | 7.9 | 8.6 | 8.3 | 9.8 | 8.6 |
| hsa-miR-6744-5p | 8.2 | 7.8 | 9.9 | 7.4 | 8.5 | 8.1 | 9.3 | 8.4 | 9.2 |
| hsa-miR-0187-5p | 8.5 | 7.9 | 7.0 | 7.4 | 7.4 | 7.1 | 7.5 | 8.2 | 7.0 |
| hsa-miR-4467 | 9.6 | 8.9 | 8.0 | 8.0 | 8.8 | 9.6 | 7.7 | 7.9 | 8.4 |
| hsa-miR-0671-5p | 8.8 | 8.7 | 10.1 | 10.1 | 10.5 | 11.0 | 9.0 | 8.1 | 7.9 |
| hsa-miR-6799-5p | 8.2 | 8.7 | 9.2 | 10.0 | 10.7 | 11.5 | 8.9 | 7.7 | 8.4 |
| hsa-miR-4298 | 8.6 | 8.6 | 8.7 | 8.8 | 9.3 | 8.4 | 8.3 | 7.3 | 8.5 |
| hsa-miR-6797-5p | 7.9 | 8.4 | 8.8 | 8.2 | 9.1 | 8.4 | 8.6 | 8.3 | 9.2 |
| hsa-miR-6829-5p | 8.1 | 7.7 | 7.9 | 7.7 | 9.2 | 8.0 | 9.2 | 8.5 | 10.2 |
| hsa-miR-1275 | 9.5 | 9.8 | 10.1 | 10.8 | 9.6 | 9.5 | 8.0 | 8.1 | 7.9 |
| hsa-miR-3614-5p | 8.2 | 8.3 | 8.6 | 8.3 | 7.6 | 7.8 | 8.8 | 8.0 | 8.5 |
| hsa-miR-4688 | 8.6 | 8.4 | 7.8 | 7.7 | 7.7 | 8.0 | 7.6 | 7.1 | 6.9 |
| hsa-miR-1207-5p | 8.8 | 8.3 | 8.2 | 8.4 | 8.1 | 8.1 | 8.5 | 8.9 | 8.4 |
| hsa-miR-6794-5p | 8.6 | 9.1 | 8.4 | 9.4 | 8.9 | 9.1 | 8.9 | 9.3 | 9.1 |
| hsa-miR-6721-5p | 9.0 | 9.0 | 8.7 | 8.2 | 8.6 | 8.6 | 7.7 | 7.9 | 6.9 |
| hsa-miR-0665 | 9.4 | 9.5 | 8.5 | 8.1 | 8.2 | 6.7 | 7.8 | 7.8 | 8.2 |
| hsa-miR-7845-5p | 8.0 | 8.7 | 8.6 | 7.8 | 9.4 | 8.5 | 7.9 | 7.6 | 7.9 |
| hsa-miR-7150 | 8.6 | 9.1 | 9.4 | 9.8 | 9.8 | 10.1 | 8.4 | 7.9 | 8.2 |
| hsa-miR-4433b-3p | 8.5 | 9.1 | 8.5 | 9.1 | 8.6 | 9.1 | 8.3 | 9.3 | 8.5 |
| hsa-miR-7114-5p | 9.2 | 8.7 | 9.2 | 8.7 | 9.3 | 9.1 | 9.0 | 11.3 | 9.9 |
| hsa-miR-4716-5p | 8.1 | 8.0 | 8.2 | 8.0 | 7.7 | 7.9 | 9.0 | 8.1 | 8.8 |
| hsa-miR-6795-3p | 8.2 | 8.3 | 8.6 | 8.1 | 7.8 | 7.7 | 9.0 | 8.3 | 8.7 |
| hsa-miR-4433a-5p | 8.2 | 8.3 | 8.4 | 8.0 | 7.7 | 7.9 | 9.0 | 8.2 | 8.8 |
| hsa-miR-6777-3p | 8.2 | 8.5 | 8.7 | 8.2 | 7.9 | 7.9 | 8.9 | 8.2 | 8.6 |
| hsa-miR-0557 | 9.5 | 9.6 | 9.7 | 9.1 | 9.3 | 9.3 | 8.4 | 8.5 | 8.1 |
| hsa-miR-1185-2-3p | 8.1 | 9.0 | 9.9 | 9.9 | 8.7 | 8.7 | 9.6 | 10.0 | 9.9 |
| hsa-miR-4731-3p | 8.5 | 8.2 | 8.6 | 8.1 | 7.8 | 7.7 | 8.9 | 8.2 | 8.6 |
| hsa-miR-4745-5p | 9.3 | 8.8 | 7.9 | 8.0 | 8.2 | 9.4 | 8.2 | 8.9 | 8.8 |
| hsa-miR-6763-3p | 8.1 | 8.2 | 8.5 | 8.1 | 7.8 | 7.9 | 9.0 | 8.3 | 8.8 |
| hsa-miR-3937 | 9.3 | 9.6 | 8.6 | 9.6 | 8.6 | 9.7 | 7.3 | 8.1 | 8.3 |
| hsa-miR-0625-3p | 8.5 | 8.3 | 8.8 | 8.3 | 8.3 | 7.9 | 9.1 | 8.5 | 8.9 |
| hsa-miR-6800-3p | 8.4 | 8.4 | 8.9 | 8.3 | 8.1 | 8.0 | 9.1 | 8.5 | 8.8 |
| hsa-miR-0940 | 8.5 | 8.7 | 8.9 | 8.6 | 8.1 | 8.2 | 9.0 | 8.1 | 8.6 |
| hsa-miR-6758-5p | 8.7 | 8.2 | 7.9 | 8.2 | 8.6 | 8.3 | 6.9 | 8.1 | 7.4 |
| hsa-miR-4274 | 8.6 | 8.5 | 8.7 | 8.5 | 8.0 | 8.1 | 9.1 | 8.1 | 8.7 |
| hsa-miR-4749-3p | 8.2 | 8.6 | 8.9 | 8.3 | 8.1 | 8.1 | 9.1 | 8.4 | 8.7 |
| hsa-miR-3675-3p | 8.4 | 8.4 | 8.9 | 8.3 | 8.2 | 8.0 | 9.2 | 8.8 | 8.9 |
| hsa-miR-0760 | 9.6 | 9.2 | 8.5 | 10.6 | 9.4 | 10.4 | 8.0 | 9.9 | 9.8 |
| hsa-miR-3620-5p | 9.1 | 9.2 | 8.2 | 8.9 | 8.6 | 8.7 | 7.9 | 9.2 | 7.9 |
| hsa-miR-4484 | 9.3 | 9.3 | 9.1 | 9.1 | 9.8 | 10.6 | 9.0 | 9.6 | 9.5 |
| hsa-miR-6774-5p | 8.9 | 9.4 | 8.9 | 9.0 | 9.5 | 10.3 | 8.7 | 8.5 | 9.6 |
| hsa-miR-0675-5p | 9.5 | 9.9 | 10.2 | 9.9 | 9.5 | 10.3 | 8.2 | 7.4 | 7.4 |
| hsa-miR-1229-5p | 9.4 | 10.2 | 10.1 | 11.6 | 10.2 | 11.0 | 8.7 | 8.5 | 8.8 |
| hsa-miR-4459 | 8.6 | 8.4 | 7.8 | 8.3 | 8.7 | 9.8 | 8.7 | 10.3 | 9.6 |
| hsa-miR-6787-5p | 8.9 | 9.6 | 9.4 | 9.9 | 9.1 | 9.4 | 8.3 | 9.5 | 8.4 |
| hsa-miR-4271 | 9.2 | 9.0 | 9.6 | 9.6 | 9.0 | 9.4 | 8.5 | 7.6 | 7.9 |
| hsa-miR-4417 | 9.5 | 9.6 | 9.2 | 8.9 | 9.0 | 9.1 | 8.3 | 9.4 | 8.2 |
| hsa-miR-4505 | 8.5 | 8.2 | 8.2 | 7.4 | 8.4 | 8.7 | 7.8 | 8.0 | 7.5 |
| hsa-miR-6875-5p | 8.7 | 7.0 | 7.8 | 6.9 | 9.3 | 9.6 | 7.9 | 7.5 | 7.5 |
| hsa-miR-4758-5p | 9.1 | 8.5 | 8.6 | 9.1 | 9.4 | 8.9 | 8.7 | 9.5 | 9.3 |
| hsa-miR-6741-5p | 8.3 | 8.0 | 8.6 | 8.9 | 8.8 | 9.1 | 9.7 | 9.2 | 9.8 |
| hsa-miR-0514b-5p | 9.5 | 9.4 | 10.0 | 9.0 | 9.0 | 8.5 | 8.7 | 9.7 | 8.4 |
| hsa-miR-4726-5p | 9.5 | 9.8 | 10.6 | 9.7 | 9.8 | 10.2 | 9.1 | 8.7 | 8.5 |
| hsa-miR-6791-5p | 9.3 | 9.6 | 8.8 | 8.4 | 9.2 | 9.3 | 8.2 | 9.3 | 8.3 |
| hsa-miR-6789-5p | 9.4 | 9.2 | 8.9 | 8.0 | 8.8 | 8.9 | 8.3 | 8.6 | 7.8 |
| hsa-miR-6762-5p | 10.0 | 9.8 | 8.9 | 8.5 | 8.8 | 8.3 | 8.1 | 8.8 | 7.0 |
| hsa-miR-6859-3p | 8.4 | 8.5 | 8.7 | 8.4 | 8.2 | 8.0 | 9.3 | 8.6 | 9.0 |
| hsa-miR-7109-5p | 9.0 | 9.8 | 10.0 | 10.5 | 10.3 | 10.3 | 9.3 | 8.7 | 9.1 |
| hsa-miR-0937-5p | 9.5 | 9.3 | 8.6 | 11.0 | 10.8 | 10.1 | 10.4 | 10.6 | 11.5 |
| hsa-miR-4722-5p | 9.4 | 9.4 | 9.4 | 8.7 | 8.5 | 8.9 | 8.2 | 8.1 | 8.2 |
| hsa -miR -6826-5p | 9.9 | 10.8 | 10.7 | 8.8 | 9.4 | 8.8 | 8.9 | 9.8 | 7.8 |
| hsa-miR-1228-3p | 8.9 | 9.0 | 9.0 | 8.7 | 8.4 | 8.5 | 9.6 | 8.7 | 9.2 |
| hsa-miR-6798-3p | 8.7 | 8.9 | 9.5 | 8.8 | 8.8 | 8.8 | 9.9 | 9.1 | 9.5 |
| hsa-miR-1268b | 9.5 | 10.0 | 8.8 | 9.2 | 10.0 | 9.9 | 9.0 | 9.7 | 10.0 |
| hsa-miR-1909-3p | 10.1 | 9.7 | 9.0 | 9.3 | 10.0 | 9.4 | 8.2 | 8.8 | 8.5 |
| hsa -miR -3136-5p | 0.0 | 0.0 | 0.0 | 0.0 | 0.0 | 0.0 | 0.0 | 0.0 | 1.5 |
| hsa-miR-7847-3p | 8.6 | 9.1 | 10.0 | 9.5 | 11.5 | 9.5 | 9.2 | 8.8 | 9.3 |
| hsa-miR-7107-5p | 8.0 | 7.9 | 8.4 | 8.0 | 9.2 | 9.2 | 8.8 | 9.1 | 9.4 |
| hsa-miR-6729-3p | 8.9 | 8.7 | 9.1 | 8.8 | 8.5 | 8.5 | 9.6 | 8.9 | 9.3 |
| hsa-miR-3648 | 10.1 | 9.4 | 8.6 | 9.4 | 11.1 | 13.1 | 10.1 | 9.4 | 9.6 |
| hsa-miR-6746-5p | 7.7 | 7.6 | 8.8 | 8.5 | 9.1 | 9.1 | 9.3 | 9.5 | 10.1 |
| hsa-miR-6821-5p | 9.8 | 10.2 | 9.5 | 10.4 | 9.3 | 10.1 | 8.8 | 9.8 | 9.6 |
| hsa-miR-6768-5p | 10.3 | 10.3 | 9.6 | 10.5 | 10.0 | 10.7 | 9.4 | 10.0 | 9.9 |
| hsa-miR-1185-1-3p | 8.7 | 9.4 | 10.2 | 10.8 | 9.2 | 9.5 | 10.3 | 10.2 | 10.7 |
| hsa-miR-6798-5p | 9.9 | 10.1 | 9.1 | 8.7 | 9.8 | 9.9 | 8.6 | 9.3 | 9.5 |
| hsa-miR-6743-5p | 9.8 | 9.9 | 9.2 | 9.3 | 9.2 | 9.6 | 9.4 | 9.9 | 9.5 |
| hsa-miR-7111-5p | 8.5 | 8.7 | 9.3 | 9.1 | 9.9 | 10.1 | 9.7 | 9.4 | 9.9 |
| hsa-miR-6769a-5p | 8.7 | 8.8 | 10.2 | 9.5 | 10.1 | 10.1 | 9.6 | 10.0 | 9.7 |
| hsa-miR-6845-5p | 10.1 | 9.8 | 9.3 | 9.3 | 10.0 | 10.4 | 8.9 | 9.4 | 8.6 |
| hsa -miR -3184-5p | 9.7 | 9.1 | 11.1 | 9.4 | 9.8 | 10.1 | 9.4 | 7.8 | 8.1 |
| hsa-miR-4656 | 9.3 | 9.0 | 9.8 | 8.8 | 9.5 | 8.9 | 9.6 | 8.6 | 9.3 |
| hsa -miR -4632-5p | 9.9 | 9.9 | 9.5 | 9.7 | 9.9 | 10.9 | 8.5 | 8.3 | 8.1 |
| hsa-miR-0642b-3p | 10.1 | 10.1 | 10.6 | 11.7 | 11.5 | 11.6 | 9.7 | 8.9 | 9.5 |
| hsa-miR-6724-5p | 9.8 | 10.1 | 8.9 | 9.7 | 9.1 | 10.0 | 8.5 | 8.8 | 8.8 |
| hsa-miR-4651 | 9.9 | 9.7 | 7.8 | 9.1 | 10.0 | 10.0 | 8.9 | 9.0 | 9.3 |
| hsa-miR-6805-3p | 9.3 | 9.3 | 9.2 | 9.1 | 8.8 | 9.2 | 9.8 | 8.9 | 9.6 |
| hsa-miR-4763-3p | 9.6 | 9.9 | 9.1 | 9.2 | 9.7 | 9.3 | 9.0 | 9.0 | 9.2 |
| hsa-miR-3619-3p | 8.6 | 8.9 | 9.0 | 9.7 | 9.1 | 9.1 | 9.8 | 10.4 | 9.9 |
| hsa-miR-6126 | 10.9 | 10.7 | 10.2 | 11.8 | 10.1 | 11.8 | 9.2 | 8.7 | 8.9 |
| hsa-miR-4706 | 9.3 | 9.5 | 9.5 | 9.1 | 9.5 | 8.9 | 9.1 | 9.8 | 9.6 |
| hsa-miR-4634 | 10.2 | 10.6 | 9.4 | 8.5 | 8.6 | 8.5 | 8.5 | 8.4 | 7.7 |
| hsa-miR-6722-3p | 10.3 | 10.3 | 9.5 | 9.7 | 9.6 | 9.6 | 8.8 | 9.2 | 8.4 |
| hsa-miR-6796-3p | 8.8 | 8.8 | 9.4 | 8.7 | 9.0 | 8.5 | 9.8 | 9.2 | 9.6 |
| hsa-miR-6165 | 8.8 | 8.7 | 9.0 | 8.7 | 8.2 | 7.8 | 9.4 | 9.0 | 9.5 |
| hsa-miR-4327 | 9.4 | 9.4 | 9.2 | 9.4 | 9.1 | 9.5 | 9.4 | 9.3 | 9.1 |
| hsa-miR-6781-5p | 9.9 | 10.0 | 9.4 | 9.5 | 10.4 | 10.6 | 9.1 | 9.6 | 9.2 |
| hsa-miR-1913 | 9.4 | 9.3 | 9.7 | 9.4 | 8.9 | 8.8 | 10.1 | 9.2 | 9.9 |
| hsa-miR-3142 | 0.0 | 0.0 | 0.0 | 0.0 | 0.0 | 0.0 | 0.0 | 0.0 | 0.0 |
| hsa-miR-0642a-3p | 9.7 | 9.4 | 11.1 | 11.4 | 12.0 | 12.1 | 10.5 | 8.9 | 9.5 |
| hsa-miR-6756-5p | 9.9 | 10.1 | 9.9 | 10.6 | 10.1 | 9.7 | 10.3 | 11.5 | 11.2 |
| hsa-miR-4731-5p | 9.9 | 9.9 | 10.6 | 9.9 | 9.4 | 9.8 | 10.2 | 9.1 | 9.5 |
| hsa-miR-5739 | 9.5 | 9.6 | 9.6 | 9.5 | 9.1 | 9.3 | 10.4 | 9.9 | 10.2 |
| hsa-miR-6766-3p | 9.1 | 9.3 | 10.0 | 9.2 | 9.2 | 9.1 | 10.3 | 9.6 | 10.0 |
| hsa-miR-4741 | 10.7 | 10.0 | 8.5 | 9.9 | 11.0 | 10.8 | 9.5 | 9.1 | 9.8 |
| hsa-miR-8063 | 11.3 | 11.2 | 10.7 | 10.2 | 10.5 | 9.3 | 9.2 | 10.6 | 8.1 |
| hsa-miR-1249-3p | 9.6 | 9.6 | 10.0 | 9.4 | 9.3 | 9.1 | 10.3 | 9.4 | 10.1 |
| hsa-miR-1914-3p | 10.2 | 10.2 | 9.8 | 9.6 | 10.1 | 9.4 | 10.3 | 9.7 | 10.2 |
| hsa-miR-3143 | 0.0 | 0.0 | 0.0 | 0.0 | 0.0 | 0.0 | 0.0 | 0.0 | 0.0 |
| hsa -miR -6858-5p | 10.4 | 10.0 | 10.9 | 10.6 | 10.7 | 11.2 | 9.7 | 9.7 | 10.6 |
| hsa-miR-3679-3p | 9.4 | 9.4 | 9.9 | 9.3 | 9.2 | 9.1 | 10.4 | 9.6 | 10.1 |
| hsa-miR-1227-5p | 10.5 | 10.4 | 9.7 | 10.2 | 10.5 | 10.6 | 10.9 | 10.6 | 11.1 |
| hsa-miR-6779-5p | 10.1 | 10.4 | 11.5 | 10.5 | 10.6 | 11.3 | 10.9 | 10.0 | 10.2 |
| hsa -miR -6836-3p | 10.8 | 10.3 | 9.7 | 8.7 | 8.6 | 8.5 | 9.6 | 9.0 | 9.1 |
| hsa-miR-5195-3p | 11.0 | 10.9 | 12.1 | 10.7 | 9.8 | 9.7 | 11.4 | 10.1 | 10.5 |
| hsa-miR-3180-3p | 10.9 | 10.5 | 10.1 | 10.3 | 9.9 | 9.6 | 9.0 | 9.5 | 8.7 |
| hsa-miR-4446-3p | 10.8 | 10.3 | 9.9 | 8.3 | 8.3 | 7.9 | 9.0 | 8.9 | 6.8 |
| hsa-miR-1915-3p | 11.0 | 10.5 | 10.1 | 9.6 | 10.6 | 10.1 | 9.3 | 9.4 | 9.3 |
| hsa-miR-3144-5p | 8.0 | 7.4 | 9.0 | 8.1 | 7.9 | 7.6 | 8.0 | 7.4 | 7.6 |
| hsa-miR-1908-5p | 11.2 | 11.0 | 10.1 | 11.7 | 11.5 | 12.2 | 9.6 | 10.1 | 10.2 |
| hsa -miR -3136-3p | 0.0 | 0.0 | 0.0 | 0.0 | 0.0 | 0.0 | 0.0 | 0.0 | 0.0 |
| hsa-miR-6800-5p | 11.2 | 11.1 | 10.8 | 11.0 | 10.6 | 10.0 | 9.3 | 9.8 | 8.6 |
| hsa -miR -0486-3p | 11.3 | 10.9 | 10.0 | 8.8 | 8.6 | 8.1 | 9.2 | 9.1 | 7.4 |
| hsa-miR-4294 | 10.3 | 10.2 | 10.7 | 11.6 | 12.0 | 12.1 | 11.0 | 9.8 | 11.0 |
| hsa-miR-4697-5p | 11.1 | 11.2 | 11.5 | 11.1 | 10.1 | 10.3 | 10.9 | 9.8 | 9.9 |
| hsa-miR-6515-3p | 10.0 | 10.0 | 10.4 | 9.9 | 9.8 | 9.8 | 11.1 | 10.4 | 10.8 |
| hsa-miR-3197 | 10.9 | 10.5 | 10.5 | 10.1 | 12.0 | 11.9 | 10.3 | 10.2 | 10.0 |
| hsa-miR-3180 | 10.7 | 11.2 | 10.8 | 10.6 | 10.4 | 10.0 | 9.3 | 9.5 | 8.7 |
| hsa-miR-6795-5p | 9.1 | 9.1 | 9.8 | 10.5 | 9.4 | 9.5 | 10.8 | 10.7 | 10.9 |
| hsa-miR-6819-5p | 10.8 | 11.5 | 12.2 | 11.2 | 11.4 | 11.9 | 11.1 | 10.5 | 10.3 |
| hsa-miR-1469 | 11.5 | 11.9 | 10.5 | 11.9 | 11.4 | 11.7 | 10.1 | 9.5 | 10.0 |
| hsa-miR-3128 | 0.0 | 0.0 | 0.0 | 0.0 | 0.0 | 0.0 | 0.0 | 0.0 | 0.0 |
| hsa-miR-7106-5p | 8.4 | 7.7 | 8.6 | 8.2 | 9.0 | 8.7 | 10.5 | 10.6 | 11.3 |
| hsa-miR-4728-5p | 10.3 | 10.5 | 10.4 | 10.6 | 10.0 | 9.6 | 11.3 | 10.2 | 11.3 |
| hsa -miR -6802-5p | 11.2 | 11.4 | 12.0 | 11.7 | 10.8 | 11.5 | 10.9 | 10.6 | 10.0 |
| hsa-miR-6749-5p | 10.0 | 10.3 | 11.3 | 11.3 | 11.5 | 11.1 | 11.8 | 11.3 | 12.0 |
| hsa-miR-0371a-5p | 11.2 | 11.0 | 11.8 | 11.4 | 10.7 | 11.0 | 11.0 | 10.4 | 10.2 |
| hsa-miR-4675 | 11.6 | 11.7 | 12.0 | 11.0 | 10.5 | 10.5 | 10.7 | 9.7 | 9.6 |
| hsa-miR-1343-5p | 11.1 | 11.7 | 10.4 | 10.6 | 11.2 | 11.8 | 9.9 | 11.1 | 10.3 |
| hsa -miR -3126-5p | 4.3 | 3.9 | 4.3 | 3.6 | 3.8 | 4.5 | 4.0 | 4.5 | 4.0 |
| hsa-miR-0149-3p | 11.1 | 11.6 | 10.8 | 11.2 | 10.6 | 10.4 | 10.6 | 10.7 | 10.3 |
| hsa-miR-1233-5p | 10.1 | 10.4 | 12.0 | 10.1 | 10.9 | 10.9 | 11.6 | 11.2 | 11.2 |
| hsa-miR-6125 | 11.8 | 11.7 | 10.9 | 10.1 | 11.7 | 11.8 | 10.9 | 11.2 | 10.9 |
| hsa-miR-6887-5p | 9.4 | 10.0 | 10.4 | 11.0 | 10.1 | 9.8 | 11.2 | 11.8 | 11.4 |
| hsa-miR-6775-5p | 10.0 | 10.5 | 11.1 | 10.7 | 10.8 | 11.2 | 12.3 | 11.2 | 11.6 |
| hsa-miR-6850-5p | 12.1 | 11.4 | 10.7 | 10.1 | 10.9 | 10.4 | 10.4 | 10.4 | 10.0 |
| hsa-miR-4534 | 11.6 | 10.7 | 11.4 | 11.2 | 12.2 | 11.9 | 10.9 | 10.4 | 12.0 |
| hsa -miR -0296-5p | 11.0 | 11.2 | 11.7 | 11.1 | 10.9 | 10.7 | 11.8 | 10.9 | 11.3 |
| hsa-miR-6803-5p | 11.6 | 11.9 | 12.4 | 12.2 | 11.8 | 12.2 | 11.4 | 11.4 | 11.0 |
| hsa-miR-0663a | 12.1 | 12.2 | 11.2 | 12.6 | 12.1 | 12.4 | 10.7 | 10.0 | 11.1 |
| hsa-miR-6752-5p | 11.9 | 12.1 | 11.6 | 11.9 | 11.9 | 11.5 | 10.6 | 11.3 | 10.9 |
| hsa-miR-4492 | 11.8 | 11.7 | 11.6 | 10.1 | 10.8 | 11.0 | 10.0 | 9.1 | 8.7 |
| hsa-miR-4270 | 11.8 | 12.7 | 12.6 | 11.6 | 11.1 | 11.1 | 11.1 | 10.0 | 9.9 |
| hsa-miR-6816-5p | 12.1 | 12.1 | 11.5 | 11.7 | 11.6 | 11.1 | 10.3 | 10.7 | 9.7 |
| hsa-miR-4687-3p | 11.8 | 12.2 | 13.4 | 12.9 | 12.0 | 12.0 | 12.1 | 11.0 | 11.0 |
| hsa-miR-6771-5p | 11.8 | 12.2 | 12.6 | 11.3 | 11.2 | 11.5 | 10.7 | 10.0 | 9.5 |
| hsa-miR-3656 | 12.3 | 12.3 | 11.6 | 11.9 | 12.0 | 11.6 | 10.9 | 11.4 | 11.1 |
| hsa-miR-4730 | 12.2 | 11.8 | 11.3 | 11.9 | 11.3 | 9.7 | 10.2 | 10.2 | 10.4 |
| hsa-miR-4463 | 12.6 | 12.3 | 12.1 | 12.5 | 12.9 | 12.3 | 11.9 | 12.2 | 12.0 |
| hsa-miR-6805-5p | 12.5 | 13.2 | 12.6 | 11.9 | 12.1 | 12.5 | 11.0 | 11.6 | 10.5 |
| hsa-miR-1268a | 12.2 | 12.7 | 11.8 | 11.8 | 11.9 | 11.8 | 10.9 | 11.1 | 10.7 |
| hsa-miR-8072 | 12.5 | 12.5 | 11.2 | 11.9 | 12.3 | 13.2 | 11.4 | 10.8 | 11.3 |
| hsa-miR-6757-5p | 10.8 | 11.0 | 13.1 | 11.0 | 11.8 | 11.6 | 12.6 | 11.7 | 12.0 |
| hsa-miR-4281 | 12.4 | 11.8 | 13.3 | 12.4 | 12.2 | 12.4 | 11.8 | 10.5 | 11.0 |
| hsa-miR-3663-3p | 13.6 | 12.8 | 12.4 | 12.2 | 12.3 | 11.2 | 11.4 | 11.2 | 11.0 |
| hsa-miR-6786-5p | 12.7 | 12.6 | 12.7 | 12.3 | 13.0 | 13.5 | 12.0 | 11.6 | 11.9 |
| hsa-miR-6765-5p | 12.6 | 12.7 | 12.5 | 12.5 | 12.3 | 11.9 | 11.2 | 11.3 | 10.7 |
| hsa-miR-6729-5p | 12.9 | 12.6 | 11.8 | 11.3 | 12.3 | 12.4 | 11.3 | 11.3 | 10.8 |
| hsa-miR-6087 | 13.4 | 13.1 | 13.1 | 13.2 | 13.1 | 13.5 | 12.0 | 11.8 | 11.2 |
| hsa-miR-6088 | 13.5 | 13.6 | 13.8 | 12.9 | 12.9 | 12.9 | 11.9 | 11.7 | 10.8 |
| hsa-miR-0638 | 13.0 | 12.8 | 11.9 | 11.5 | 12.5 | 12.7 | 11.5 | 11.6 | 11.0 |
| hsa-miR-4674 | 13.4 | 12.8 | 12.1 | 10.7 | 10.6 | 10.5 | 11.0 | 10.7 | 8.9 |
| hsa-miR-4734 | 12.6 | 12.4 | 12.0 | 10.7 | 11.4 | 11.2 | 11.6 | 11.2 | 10.5 |
| hsa-miR-4689 | 12.5 | 12.8 | 13.8 | 13.0 | 12.5 | 12.8 | 13.1 | 12.0 | 12.3 |
| hsa-miR-6784-5p | 13.5 | 13.0 | 12.8 | 12.5 | 13.0 | 12.8 | 11.9 | 12.2 | 11.0 |
| hsa-miR-6785-5p | 13.2 | 13.6 | 13.9 | 12.4 | 12.2 | 11.8 | 11.6 | 11.4 | 10.2 |
| hsa-miR-3621 | 13.8 | 13.6 | 12.8 | 12.1 | 12.3 | 11.3 | 11.6 | 12.5 | 10.9 |
| hsa-miR-0328-5p | 13.4 | 13.5 | 13.6 | 13.6 | 13.3 | 13.9 | 12.4 | 11.5 | 11.8 |
| hsa-miR-4739 | 13.1 | 13.4 | 14.4 | 13.8 | 13.9 | 13.9 | 12.9 | 11.2 | 11.6 |
| hsa-miR-6085 | 11.7 | 12.3 | 13.2 | 13.1 | 13.5 | 13.3 | 13.5 | 12.5 | 13.5 |
| hsa-miR-4442 | 13.8 | 14.5 | 14.7 | 13.4 | 12.6 | 12.8 | 12.4 | 11.6 | 10.6 |
| hsa-miR-4649-5p | 13.5 | 13.7 | 13.3 | 12.3 | 12.6 | 11.3 | 11.7 | 11.7 | 9.8 |
| hsa-miR-3196 | 13.9 | 13.6 | 13.0 | 12.6 | 12.8 | 13.2 | 11.8 | 11.3 | 10.9 |
| hsa-miR-4466 | 13.8 | 13.6 | 12.9 | 12.4 | 12.4 | 12.6 | 12.0 | 11.7 | 11.5 |
| hsa-miR-4508 | 13.5 | 13.3 | 13.1 | 12.1 | 13.2 | 12.9 | 11.9 | 11.6 | 11.5 |
| hsa-miR-1237-5p | 13.7 | 13.9 | 13.1 | 13.3 | 13.3 | 13.1 | 12.1 | 12.6 | 12.1 |
| hsa-miR-4532 | 13.5 | 12.6 | 12.9 | 10.1 | 12.5 | 11.9 | 12.3 | 12.1 | 9.8 |
| hsa-miR-6090 | 13.8 | 13.9 | 13.2 | 13.2 | 13.3 | 13.1 | 12.4 | 12.4 | 12.2 |
| hsa-miR-2861 | 14.0 | 14.0 | 14.4 | 14.2 | 14.0 | 13.9 | 12.9 | 12.4 | 12.1 |
| hsa-miR-3163 | 0.0 | 0.0 | 0.0 | 0.0 | 0.0 | 1.6 | 0.0 | 0.0 | 2.2 |
| hsa-miR-4723-5p | 12.3 | 12.5 | 13.8 | 13.5 | 14.0 | 13.0 | 14.0 | 13.2 | 14.2 |
| hsa-miR-3178 | 14.5 | 14.3 | 13.1 | 12.8 | 13.0 | 13.1 | 12.2 | 11.7 | 11.3 |
| hsa-miR-0128-2-5p | 14.0 | 14.1 | 14.5 | 13.5 | 13.4 | 12.9 | 13.3 | 12.3 | 11.9 |
| hsa-miR-1228-5p | 13.8 | 14.2 | 13.6 | 13.4 | 13.2 | 13.4 | 12.4 | 12.0 | 11.5 |
| hsa-miR-0762 | 14.4 | 14.4 | 13.5 | 13.2 | 13.6 | 13.3 | 12.9 | 13.1 | 11.9 |
| hsa-miR-4488 | 14.4 | 14.7 | 14.1 | 13.9 | 13.9 | 13.3 | 13.0 | 12.9 | 12.4 |
| hsa-miR-8069 | 14.4 | 14.8 | 13.6 | 13.2 | 12.9 | 13.3 | 12.6 | 12.8 | 11.9 |
| hsa-miR-3940-5p | 14.3 | 14.4 | 13.8 | 13.9 | 13.9 | 13.4 | 12.6 | 13.1 | 11.8 |
| hsa-miR-6869-5p | 14.5 | 14.2 | 13.8 | 12.7 | 14.3 | 13.3 | 12.7 | 12.3 | 12.5 |
| hsa-miR-6089 | 14.6 | 14.2 | 14.0 | 13.6 | 14.0 | 13.5 | 13.2 | 13.1 | 12.8 |
| hsa-miR-6727-5p | 14.9 | 14.9 | 14.1 | 13.2 | 13.1 | 13.0 | 12.9 | 12.6 | 11.1 |
| hsa-miR-6885-5p | 14.5 | 14.8 | 15.1 | 13.8 | 13.9 | 13.4 | 13.6 | 12.7 | 12.1 |
| hsa-miR-5787 | 14.7 | 14.8 | 14.0 | 13.6 | 14.2 | 13.9 | 13.2 | 12.8 | 12.7 |
| hsa-miR-4787-5p | 15.1 | 15.1 | 14.3 | 13.5 | 13.8 | 13.9 | 13.2 | 12.6 | 12.1 |
| hsa-miR-7704 | 15.4 | 15.1 | 14.6 | 13.5 | 13.8 | 13.8 | 13.4 | 13.1 | 11.9 |
| hsa-miR-4516 | 16.0 | 15.1 | 14.6 | 14.2 | 14.4 | 13.9 | 14.1 | 14.2 | 12.8 |
| hsa-miR-3665 | 15.9 | 15.1 | 14.7 | 14.1 | 13.8 | 13.9 | 14.0 | 13.9 | 12.5 |
| hsa-miR-3960 | 16.0 | 15.1 | 15.7 | 14.8 | 15.6 | 13.9 | 15.1 | 14.4 | 13.8 |

### [Table 4-1]

**Table 4-1: Relationship between miRNA expression and the sensitivity and specificity of lung cancer detection**

| miRNA name | P value (*t*-test) | Mean of lung cancer patients | Mean of healthy persons | Sensitivity | Specificity |
|---|---|---|---|---|---|
| hsa-let-7a-3p | 0 | 0.017 | 0.061 | 99% | 97% |
| hsa-let-7b-5p | 0.01047985 | 1.975 | 1.773 | 60% | 58% |
| hsa-let-7e-3p | 0.03175525 | 1.677 | 1.658 | 53% | 60% |
| hsa-let-7e-5p | 2.76E-05 | 0.715 | 1.437 | 69% | 46% |
| hsa-let-7f-2-3p | 0.00095546 | 1.045 | 1.414 | 61% | 47% |
| hsa-let-7g-3p | 0.00342142 | 0.407 | 0.555 | 76% | 75% |
| hsa-let-7g-5p | 0 | 0.000 | 0.180 | 100% | 93% |
| hsa-miR-100-3p | 0 | 0.019 | 0.025 | 99% | 99% |
| hsa-miR-101-3p | 0 | 0.034 | 0.095 | 99% | 97% |
| hsa-miR-103a-3p | 0.00468298 | 0.514 | 0.691 | 72% | 69% |
| hsa-miR-105-3p | 0 | 0.000 | 0.110 | 100% | 94% |
| hsa-miR-106b-5p | 1.39E-05 | 0.065 | 0.499 | 94% | 79% |
| hsa-miR-107 | 0.01285019 | 0.133 | 0.654 | 90% | 69% |
| hsa-miR-10a-5p | 0.00357768 | 0.152 | 0.744 | 89% | 68% |
| hsa-miR-10b-3p | 0 | 0.000 | 0.028 | 100% | 99% |
| hsa-miR-10b-5p | 0.040208 | 0.267 | 0.449 | 82% | 77% |
| hsa-miR-1178-3p | 0.02844284 | 2.537 | 2.501 | 66% | 66% |
| hsa-miR-1180-5p | 0.00068534 | 0.247 | 0.386 | 83% | 84% |
| hsa-miR-1183 | 0.0222621 | 0.069 | 0.183 | 93% | 90% |
| hsa-miR-1185-1-3p | 0.0075871 | 9.064 | 8.791 | 64% | 52% |
| hsa-miR-1185-5p | 0 | 0.000 | 0.018 | 100% | 99% |
| hsa-miR-1193 | 0.02122042 | 5.677 | 5.494 | 65% | 51% |
| hsa-miR-1197 | 0 | 0.000 | -0.004 | 100% | 99% |
| hsa-miR-1199-5p | 0.00152727 | 3.340 | 3.105 | 68% | 50% |
| hsa-miR-1205 | 0.01502456 | 0.823 | 1.060 | 62% | 58% |
| hsa-miR-1206 | 0 | 0.009 | 0.086 | 99% | 97% |
| hsa-miR-122-3p | 0.01119994 | 0.645 | 0.932 | 69% | 62% |
| hsa-miR-1224-5p | 1.13E-06 | 4.602 | 3.845 | 75% | 54% |
| hsa-miR-1226-5p | 0.0225801 | 2.258 | 1.949 | 69% | 52% |
| hsa-miR-1229-5p | 2.05E-07 | 8.900 | 9.516 | 63% | 39% |
| hsa-miR-1238-5p | 0.03222639 | 7.012 | 6.791 | 67% | 56% |
| hsa-miR-1243 | 0 | 0.000 | 0.027 | 100% | 99% |
| hsa-miR-1245b-3p | 0 | 0.000 | 0.017 | 100% | 99% |
| hsa-miR-1246 | 0 | 0.038 | 0.046 | 99% | 98% |
| hsa-miR-1247-3p | 0.01517924 | 6.030 | 6.342 | 65% | 41% |
| hsa-miR-1250-5p | 4.18E-05 | 2.723 | 1.643 | 79% | 46% |
| hsa-miR-1252-3p | 0 | 0.023 | 0.113 | 99% | 96% |
| hsa-miR-1252-5p | 0 | 0.000 | 0.025 | 100% | 99% |
| hsa-miR-1255b-2-3p | 0.00995524 | 0.763 | 0.865 | 63% | 65% |
| hsa-miR-1257 | 0.02736289 | 0.345 | 0.450 | 80% | 83% |
| hsa-miR-1258 | 0 | 0.003 | 0.028 | 99% | 99% |
| hsa-miR-125a-3p | 0.00043968 | 6.224 | 5.896 | 64% | 53% |
| hsa-miR-1263 | 0.01450131 | 0.255 | 0.597 | 82% | 71% |
| hsa-miR-126-3p | 0 | 0.000 | 0.167 | 100% | 93% |
| hsa-miR-126-5p | 0 | 0.000 | 0.156 | 100% | 92% |
| hsa-miR-1271-5p | 0.0258972 | 2.271 | 1.678 | 71% | 54% |
| hsa-miR-1272 | 0.0005132 | 0.280 | 0.459 | 82% | 83% |
| hsa-miR-1273a | 0.01784548 | 0.462 | 0.635 | 78% | 75% |
| hsa-miR-1273f | 0.00041619 | 4.027 | 2.997 | 80% | 53% |
| hsa-miR-1273g-3p | 4.54E-07 | 4.456 | 3.802 | 68% | 46% |
| hsa-miR-1273g-5p | 5.54E-07 | 2.814 | 2.042 | 75% | 52% |
| hsa-miR-1273h-5p | 8.47E-06 | 5.220 | 4.653 | 69% | 48% |
| hsa-miR-1277-3p | 0 | 0.000 | 0.024 | 100% | 99% |
| hsa-miR-1277-5p | 0 | 0.031 | 0.137 | 99% | 96% |
| hsa-miR-1279 | 0 | 0.016 | 0.050 | 99% | 98% |
| hsa-miR-1282 | 0.02860194 | 0.052 | 0.059 | 95% | 97% |
| hsa-miR-1286 | 0.00633393 | 0.831 | 1.534 | 64% | 41% |
| hsa-miR-1289 | 0.00208456 | 0.197 | 0.241 | 84% | 88% |
| hsa-miR-1293 | 1.86E-06 | 3.485 | 2.734 | 76% | 53% |
| hsa-miR-1295b-5p | 0 | 0.022 | 0.179 | 99% | 93% |
| hsa-miR-1297 | 1.05E-05 | 0.763 | 1.346 | 64% | 48% |
| hsa-miR-1298-5p | 0.01610711 | 0.312 | 0.514 | 80% | 77% |
| hsa-miR-1306-3p | 0.00162408 | 2.887 | 1.905 | 72% | 51% |
| hsa-miR-1307-3p | 3.49E-07 | 5.273 | 4.649 | 73% | 42% |
| hsa-miR-1321 | 0.01671212 | 1.574 | 1.226 | 59% | 42% |
| hsa-miR-1322 | 0.0086827 | 1.823 | 1.070 | 61% | 41% |
| hsa-miR-132-3p | 0.01846603 | 0.539 | 1.051 | 72% | 51% |
| hsa-miR-134-5p | 0.02022658 | 5.406 | 5.667 | 61% | 39% |
| hsa-miR-136-5p | 0 | 0.000 | 0.266 | 100% | 88% |
| hsa-miR-138-1-3p | 0.0056765 | 3.685 | 3.153 | 74% | 54% |
| hsa-miR-1-3p | 0 | 0.000 | 0.020 | 100% | 99% |
| hsa-miR-140-5p | 0.0428156 | 0.080 | 0.512 | 95% | 75% |
| hsa-miR-143-5p | 0.02123662 | 0.038 | 0.080 | 95% | 94% |
| hsa-miR-145-3p | 0 | 0.000 | 0.059 | 100% | 97% |
| hsa-miR-1468-3p | 0 | 0.029 | 0.059 | 99% | 98% |
| hsa-miR-1468-5p | 0.01505323 | 3.297 | 2.604 | 77% | 59% |
| hsa-miR-1471 | 8.14E-06 | 4.761 | 4.243 | 76% | 53% |
| hsa-miR-147b | 0.00013086 | -0.013 | 0.144 | 100% | 95% |
| hsa-miR-148a-5p | 0.01415458 | 0.002 | 0.162 | 98% | 92% |
| hsa-miR-150-3p | 0.00788376 | 6.522 | 6.276 | 60% | 47% |
| hsa-miR-152-3p | 0 | 0.036 | 0.169 | 99% | 92% |
| hsa-miR-153-3p | 0 | 0.000 | 0.141 | 100% | 93% |
| hsa-miR-155-5p | 0 | 0.022 | 0.174 | 99% | 93% |
| hsa-miR-1587 | 0.00019331 | 6.330 | 5.778 | 70% | 44% |
| hsa-miR-15b-3p | 0.01494351 | 0.143 | 0.302 | 88% | 84% |
| hsa-miR-1-5p | 0.04008832 | 0.920 | 1.182 | 60% | 53% |
| hsa-miR-16-5p | 0.00011636 | 0.189 | 0.443 | 86% | 77% |
| hsa-miR-17-5p | 0.00303782 | 0.075 | 0.303 | 92% | 85% |
| hsa-miR-181a-5p | 0.00011924 | 0.650 | 1.396 | 71% | 46% |
| hsa-miR-181d-3p | 5.08E-05 | 0.775 | 1.193 | 67% | 59% |
| hsa-miR-181d-5p | 0.00125209 | 0.381 | 0.797 | 76% | 63% |
| hsa-miR-182-5p | 0.00407475 | 0.108 | 0.346 | 90% | 83% |
| hsa-miR-185-3p | 2.48E-05 | 5.544 | 5.115 | 66% | 50% |
| hsa-miR-185-5p | 0.00306169 | 4.688 | 3.827 | 67% | 45% |
| hsa-miR-186-5p | 0 | 0.029 | 0.137 | 99% | 95% |
| hsa-miR-187-3p | 0.00081766 | 3.872 | 3.470 | 72% | 60% |
| hsa-miR-18a-5p | 0 | 0.000 | 0.122 | 100% | 95% |
| hsa-miR-1909-5p | 0.00078548 | 4.216 | 3.952 | 69% | 59% |
| hsa-miR-190a-5p | 0 | 0.000 | 0.024 | 100% | 99% |
| hsa-miR-190b | 0 | 0.000 | 0.032 | 100% | 99% |
| hsa-miR-1910-3p | 0.00261709 | 2.767 | 1.559 | 73% | 42% |
| hsa-miR-1911-5p | 0.00547949 | 0.180 | 0.551 | 86% | 70% |
| hsa-miR-1912 | 0.00034036 | 0.852 | 1.270 | 62% | 51% |
| hsa-miR-193a-5p | 0.00567942 | 3.623 | 3.021 | 75% | 57% |
| hsa-miR-194-3p | 0.00175762 | 4.156 | 3.724 | 75% | 52% |
| hsa-miR-195-5p | 0.00408192 | 0.084 | 0.213 | 92% | 90% |
| hsa-miR-196a-5p | 0.00923371 | 0.371 | 0.620 | 78% | 71% |
| hsa-miR-1972 | 0.0007111 | 3.560 | 3.244 | 73% | 57% |
| hsa-miR-197-5p | 0.0289932 | 7.227 | 7.085 | 65% | 50% |
| hsa-miR-198 | 0.04318343 | 5.538 | 5.884 | 59% | 44% |
| hsa-miR-19a-5p | 0 | 0.032 | 0.366 | 99% | 85% |
| hsa-miR-19b-1 -5p | 0 | 0.033 | 0.043 | 99% | 99% |
| hsa-miR-19b-2-5p | 0 | 0.000 | 0.103 | 100% | 95% |
| hsa-miR-19b-3p | 0 | 0.022 | 0.175 | 99% | 92% |
| hsa-miR-200c-3p | 0.00703711 | 0.116 | 0.523 | 92% | 79% |
| hsa-miR-203 a-5p | 0.01796095 | 0.936 | 1.004 | 59% | 62% |
| hsa-miR-203b-5p | 0.0002285 | 0.047 | 0.070 | 94% | 97% |
| hsa-miR-2052 | 0.00177066 | 0.041 | 0.178 | 98% | 94% |
| hsa-miR-2053 | 0 | 0.000 | 0.056 | 100% | 97% |
| hsa-miR-205-3p | 0 | 0.000 | 0.053 | 100% | 98% |
| hsa-miR-2054 | 0 | 0.000 | 0.038 | 100% | 99% |
| hsa-miR-208a-3p | 0 | -0.006 | 0.040 | 100% | 98% |
| hsa-miR-20a-5p | 0 | 0.000 | 0.132 | 100% | 94% |
| hsa-miR-20b-5p | 0.01852504 | 0.274 | 0.452 | 83% | 79% |
| hsa-miR-2114-5p | 0.00897927 | 0.046 | 0.159 | 96% | 92% |
| hsa-miR-212-5p | 0.00065937 | 2.453 | 2.069 | 69% | 58% |
| hsa-miR-215-3p | 0.02821865 | 0.042 | 0.133 | 96% | 93% |
| hsa-miR-216a-5p | 0.00233015 | 0.081 | 0.567 | 95% | 72% |
| hsa-miR-219a-1-3p | 0 | 0.031 | 0.109 | 99% | 95% |
| hsa-miR-219b-3p | 0 | 0.000 | 0.041 | 100% | 98% |
| hsa-miR-222-3p | 0.00572668 | 0.099 | 0.457 | 91% | 78% |
| hsa-miR-22-5p | 0.0314819 | 0.184 | 0.503 | 88% | 78% |
| hsa-miR-2276-3p | 6.46E-06 | 4.807 | 3.944 | 84% | 51% |
| hsa-miR-2277-3p | 0.01464309 | 3.747 | 3.144 | 75% | 55% |
| hsa-miR-23b-3p | 0.00583149 | 1.123 | 1.263 | 54% | 52% |
| hsa-miR-23b-5p | 0.01203739 | 0.178 | 0.309 | 86% | 85% |
| hsa-miR-2467-3p | 2.49E-05 | 4.369 | 3.543 | 78% | 60% |
| hsa-miR-2681-3p | 0.0002473 | 0.089 | 0.284 | 93% | 87% |
| hsa-miR-27a-3p | 1.37E-05 | 0.375 | 0.922 | 77% | 62% |
| hsa-miR-296-3p | 3.15E-05 | 4.786 | 4.319 | 65% | 45% |
| hsa-miR-297 | 0.01314551 | 2.119 | 1.226 | 67% | 42% |
| hsa-miR-29a-3p | 0.00887703 | 0.247 | 0.321 | 83% | 84% |
| hsa-miR-29b-1 -5p | 0.01067588 | 0.312 | 0.329 | 78% | 84% |
| hsa-miR-29b-2-5p | 0.03683633 | 0.357 | 0.393 | 76% | 79% |
| hsa-miR-301a-3p | 0 | 0.013 | 0.113 | 99% | 94% |
| hsa-miR-3 02c-5p | 0.00149779 | 5.758 | 5.412 | 69% | 45% |
| hsa-miR-3064-3p | 9.28E-05 | 3.815 | 2.818 | 78% | 56% |
| hsa-miR-3 074-5p | 0.04678983 | 2.091 | 1.869 | 57% | 52% |
| hsa-miR-3 0a-5p | 0.00084183 | 0.142 | 0.663 | 90% | 71% |
| hsa-miR-30c-1-3p | 1.84E-06 | 4.714 | 5.426 | 63% | 37% |
| hsa-miR-30d-3p | 0 | 0.000 | 0.045 | 100% | 98% |
| hsa-miR-30e-3p | 3.89E-06 | 0.629 | 1.539 | 71% | 39% |
| hsa-miR-30e-5p | 0 | 0.000 | 0.231 | 100% | 91% |
| hsa-miR-3118 | 0 | 0.016 | 0.018 | 99% | 99% |
| hsa-miR-3l20-5p | 0.015145 | 3.215 | 2.654 | 74% | 57% |
| hsa-miR-3121-5p | 0.02995321 | 0.013 | 0.108 | 98% | 94% |
| hsa-miR-3122 | 2.43E-06 | 4.801 | 3.963 | 75% | 42% |
| hsa-miR-3123 | 0 | 0.000 | 0.021 | 100% | 99% |
| hsa-miR-3124-5p | 0.04305765 | 4.475 | 3.768 | 67% | 49% |
| hsa-miR-3126-5p | 0.0274292 | 3.600 | 2.731 | 79% | 56% |
| hsa-miR-3129-5p | 0 | 0.002 | 0.021 | 99% | 99% |
| hsa-miR-3130-5p | 0.00835479 | 4.259 | 4.019 | 67% | 58% |
| hsa-miR-3135a | 0 | 0.000 | 0.166 | 100% | 93% |
| hsa-miR-3137 | 0.01069797 | 2.989 | 2.217 | 77% | 53% |
| hsa-miR-3138 | 0.01299281 | 4.240 | 3.476 | 75% | 50% |
| hsa-miR-3140-3p | 0 | 0.000 | 0.096 | 100% | 97% |
| hsa-miR-3143 | 0 | 0.030 | 0.078 | 99% | 97% |
| hsa-miR-3144-3p | 1.80E-08 | 0.853 | 1.574 | 60% | 41% |
| hsa-miR-3145-5p | 0.00362573 | 2.728 | 2.286 | 71% | 58% |
| hsa-miR-3149 | 0.02776021 | 2.678 | 1.813 | 68% | 51% |
| hsa-miR-3151-5p | 0.00406222 | 6.132 | 5.756 | 69% | 52% |
| hsa-miR-3152-3p | 0 | 0.000 | 0.031 | 100% | 98% |
| hsa-miR-3153 | 1.15E-05 | 5.669 | 5.074 | 72% | 52% |
| hsa-miR-3156-5p | 5.14E-06 | 5.027 | 4.452 | 72% | 48% |
| hsa-miR-3157-5p | 0.01627428 | 0.078 | 0.162 | 91% | 91% |
| hsa-miR-3160-3p | 7.54E-05 | 5.179 | 4.663 | 70% | 51% |
| hsa-miR-3160-5p | 0.00273037 | 3.204 | 2.730 | 75% | 58% |
| hsa-miR-3163 | 0.03309939 | 0.322 | 0.435 | 78% | 78% |
| hsa-miR-3164 | 0.00141326 | 4.206 | 2.944 | 74% | 51% |
| hsa-miR-3167 | 0 | 0.000 | 0.036 | 100% | 99% |
| hsa-miR-3169 | 0 | 0.003 | 0.188 | 99% | 92% |
| hsa-miR-3174 | 0.02882934 | 2.234 | 1.596 | 68% | 48% |
| hsa-miR-3176 | 0.00144698 | 3.915 | 3.292 | 79% | 61% |
| hsa-miR-3177-3p | 2.67E-06 | 4.974 | 4.269 | 70% | 46% |
| hsa-miR-3177-5p | 6.79E-05 | 4.095 | 3.372 | 74% | 43% |
| hsa-miR-3182 | 0.00765237 | 0.122 | 0.340 | 92% | 87% |
| hsa-miR-3183 | 0 | 0.000 | 0.133 | 100% | 94% |
| hsa-miR-3186-3p | 0.04483078 | 4.126 | 3.216 | 75% | 54% |
| hsa-miR-3187-3p | 0.02489021 | 4.522 | 4.156 | 74% | 54% |
| hsa-miR-3187-5p | 1.62E-08 | 5.278 | 4.424 | 77% | 50% |
| hsa-miR-3189-3p | 0.00473949 | 4.370 | 3.753 | 65% | 54% |
| hsa-miR-3193 | 0.0403854 | 0.745 | 0.978 | 64% | 60% |
| hsa-miR-3201 | 0 | 0.030 | 0.031 | 99% | 99% |
| hsa-miR-323a-5p | 0.00236446 | 3.468 | 2.847 | 76% | 59% |
| hsa-miR-329-3p | 0.00062724 | 0.283 | 1.100 | 83% | 50% |
| hsa-miR-335-5p | 0 | 0.038 | 0.066 | 99% | 98% |
| hsa-miR-337-5p | 0.0179591 | 0.476 | 0.647 | 74% | 70% |
| hsa-miR-339-3p | 0 | 0.073 | 0.423 | 99% | 87% |
| hsa-miR-34a-3p | 0.01432864 | 0.059 | 0.412 | 96% | 81% |
| hsa-miR-34a-5p | 0.0151047 | 2.782 | 2.311 | 69% | 57% |
| hsa-miR-3591-3p | 6.48E-06 | 3.735 | 2.539 | 82% | 52% |
| hsa-miR-3591-5p | 0.00103621 | 0.300 | 0.804 | 82% | 66% |
| hsa-miR-3 605 -5p | 3.74E-06 | 2.230 | 2.176 | 67% | 59% |
| hsa-miR-3607-5p | 0 | 0.000 | 0.019 | 100% | 99% |
| hsa-miR-3612 | 0.00114695 | 4.718 | 3.964 | 71% | 50% |
| hsa-miR-3613-3p | 0.00165296 | 0.626 | 0.930 | 65% | 58% |
| hsa-miR-361-5p | 0.0002826 | 0.201 | 0.488 | 85% | 78% |
| hsa-miR-3616-3p | 0.03065773 | 5.024 | 4.912 | 61% | 48% |
| hsa-miR-3616-5p | 0 | 0.023 | 0.136 | 99% | 95% |
| hsa-miR-3617-5p | 0 | 0.000 | 0.161 | 100% | 93% |
| hsa-miR-3618 | 0 | 0.025 | 0.214 | 99% | 91% |
| hsa-miR-3619-3p | 0.00129529 | 8.764 | 8.420 | 69% | 47% |
| hsa-miR-3622a-5p | 0.01038175 | 4.265 | 3.793 | 69% | 51% |
| hsa-miR-3622b-5p | 0.00213122 | 6.071 | 5.799 | 63% | 49% |
| hsa-miR-362-5p | 0.04550376 | 0.109 | 0.328 | 95% | 89% |
| hsa-miR-363-3p | 0.00020027 | 0.406 | 1.300 | 81% | 51% |
| hsa-miR-3652 | 6.69E-08 | 6.052 | 5.168 | 70% | 37% |
| hsa-miR-3653-3p | 0.00023717 | 0.090 | 0.479 | 92% | 79% |
| hsa-miR-3654 | 0.00141001 | 1.819 | 1.766 | 58% | 56% |
| hsa-miR-3655 | 8.86E-06 | 2.798 | 2.081 | 74% | 51% |
| hsa-miR-3659 | 0.04376822 | 3.018 | 2.491 | 72% | 56% |
| hsa-miR-3662 | 0 | 0.008 | 0.025 | 99% | 99% |
| hsa-miR-3663-5p | 0.01102841 | 5.140 | 4.811 | 74% | 53% |
| hsa-miR-3668 | 0 | 0.000 | 0.074 | 100% | 97% |
| hsa-miR-3678-3p | 0.0421118 | 1.320 | 1.347 | 51% | 52% |
| hsa-miR-3681-5p | 0.00415788 | 0.010 | 0.164 | 98% | 93% |
| hsa-miR-3683 | 0 | 0.000 | 0.038 | 100% | 99% |
| hsa-miR-3686 | 0 | 0.000 | 0.018 | 100% | 99% |
| hsa-miR-3688-3p | 0 | 0.000 | 0.126 | 100% | 94% |
| hsa-miR-3689a-3p | 0.00219707 | 3.784 | 3.168 | 75% | 60% |
| hsa-miR-3689a-5p, hsa-miR-3689b-5p, hsa-miR-3689e | 0.04428878 | 0.025 | 0.129 | 97% | 93% |
| hsa-miR-3689b-3p, hsa-miR-3689c | 0.00226354 | 3.658 | 3.237 | 75% | 59% |
| hsa-miR-3 69 1-5p | 0.01244876 | 2.315 | 1.278 | 74% | 44% |
| hsa-miR-369-3p | 0 | 0.000 | 0.141 | 100% | 94% |
| hsa-miR-370-5p | 0.01322067 | 0.860 | 1.011 | 62% | 60% |
| hsa-miR-3713 | 0.00820287 | 3.011 | 3.003 | 65% | 64% |
| hsa-miR-372-3p | 0.00158925 | 1.062 | 1.801 | 61% | 43% |
| hsa-miR-372-5p | 0.01252586 | 0.129 | 0.463 | 90% | 77% |
| hsa-miR-374a-3p | 0 | 0.000 | 0.027 | 100% | 99% |
| hsa-miR-374a-5p | 0 | 0.000 | 0.033 | 100% | 98% |
| hsa-miR-374b-5p | 0.01180602 | 0.574 | 0.989 | 72% | 59% |
| hsa-miR-3 74c-5p | 0.00454819 | 0.538 | 0.679 | 71% | 72% |
| hsa-miR-375 | 0.00345643 | 0.574 | 1.294 | 75% | 50% |
| hsa-miR-376a-5p | 0 | 0.005 | 0.036 | 99% | 97% |
| hsa-miR-376c-3p | 0.02907226 | 0.067 | 0.091 | 98% | 96% |
| hsa-miR-378b | 0.01993256 | 2.729 | 2.410 | 69% | 61% |
| hsa-miR-380-3p | 0.00445659 | 1.119 | 1.251 | 52% | 51% |
| hsa-miR-380-5p | 0.01009763 | 0.083 | 0.245 | 94% | 88% |
| hsa-miR-3907 | 0.0234229 | 0.076 | 0.132 | 93% | 93% |
| hsa-miR-3912-5p | 0.04227772 | -0.001 | 0.078 | 99% | 96% |
| hsa-miR-3917 | 1.05E-06 | 6.129 | 5.440 | 74% | 44% |
| hsa-miR-3919 | 0.02477154 | 1.146 | 1.294 | 50% | 52% |
| hsa-miR-3922-5p | 1. 77E-05 | 3.440 | 2.769 | 80% | 55% |
| hsa-miR-3927-3p | 0 | 0.000 | 0.023 | 100% | 99% |
| hsa-miR-3927-5p | 0.04974602 | 2.476 | 2.150 | 68% | 58% |
| hsa-miR-3928-3p | 2.95E-06 | 7.859 | 7.187 | 60% | 46% |
| hsa-miR-3934-5p | 2.43E-06 | 3.494 | 2.612 | 77% | 50% |
| hsa-miR-3935 | 0.00011155 | 4.136 | 3.212 | 79% | 55% |
| hsa-miR-3942-5p | 0 | 0.034 | 0.053 | 99% | 98% |
| hsa-miR-3944-3p | 0.01329274 | 4.463 | 4.230 | 75% | 55% |
| hsa-miR-3976 | 0.04075733 | 0.011 | 0.121 | 98% | 94% |
| hsa-miR-3978 | 0.01389531 | 0.303 | 0.621 | 85% | 74% |
| hsa-miR-424-3p | 0.04490195 | 3.191 | 2.162 | 63% | 46% |
| hsa-miR-4252 | 0.01886895 | 3.486 | 3.097 | 69% | 57% |
| hsa-miR-425-5p | 0.01357536 | 0.276 | 0.645 | 82% | 68% |
| hsa-miR-4257 | 7.43E-05 | 7.235 | 6.792 | 58% | 46% |
| hsa-miR-4259 | 0.00022613 | 6.173 | 5.851 | 67% | 42% |
| hsa-miR-4260 | 4.52E-05 | 4.679 | 3.896 | 74% | 42% |
| hsa-miR-4262 | 0.00664949 | 1.865 | 1.233 | 61% | 45% |
| hsa-miR-4269 | 0.00066323 | 4.781 | 4.291 | 71% | 49% |
| hsa-miR-4273 | 0.00036042 | 2.143 | 1.253 | 68% | 43% |
| hsa-miR-4277 | 0 | 0.010 | 0.031 | 99% | 99% |
| hsa-miR-4285 | 0.04294135 | 1.728 | 1.279 | 57% | 45% |
| hsa-miR-429 | 0.01912748 | 0.103 | 0.234 | 92% | 89% |
| hsa-miR-4293 | 9.45E-05 | 0.388 | 1.030 | 78% | 59% |
| hsa-miR-4296 | 0.00071279 | 2.891 | 1.882 | 73% | 52% |
| hsa-miR-4297 | 0.04573006 | 4.139 | 4.087 | 63% | 64% |
| hsa-miR-4300 | 0.00054404 | 3.429 | 2.130 | 69% | 43% |
| hsa-miR-4302 | 0 | 0.024 | 0.099 | 99% | 95% |
| hsa-miR-4304 | 0.04519453 | 2.340 | 2.302 | 64% | 62% |
| hsa-miR-4306 | 8.82E-05 | 5.571 | 4.343 | 75% | 46% |
| hsa-miR-4307 | 1.83E-09 | 4.313 | 3.451 | 77% | 45% |
| hsa-miR-431-3p | 0.01202945 | 3.893 | 3.758 | 68% | 58% |
| hsa-miR-4320 | 0.00073285 | 0.049 | 0.257 | 94% | 85% |
| hsa-miR-4325 | 0.00835072 | 0.252 | 0.422 | 84% | 80% |
| hsa-miR-433-3p | 6.26E-07 | 0.567 | 1.503 | 71% | 44% |
| hsa-miR-4418 | 0.0012129 | 0.030 | 0.213 | 97% | 93% |
| hsa-miR-4419a | 0.01014614 | 7.237 | 6.994 | 63% | 46% |
| hsa-miR-4419b | 0.00057147 | 7.442 | 6.961 | 65% | 48% |
| hsa-miR-4420 | 0.04973188 | 2.455 | 2.020 | 68% | 53% |
| hsa-miR-4422 | 0.01117026 | 0.104 | 0.352 | 94% | 87% |
| hsa-miR-4428 | 4.80E-07 | 4.460 | 3.364 | 80% | 48% |
| hsa-miR-4430 | 0.00394051 | 5.970 | 5.606 | 70% | 53% |
| hsa-miR-4432 | 0 | -0.003 | 0.078 | 100% | 95% |
| hsa-miR-4436a | 0.00303329 | 3.490 | 2.597 | 77% | 56% |
| hsa-miR-4436b-3p | 2.80E-06 | 3.868 | 2.562 | 83% | 50% |
| hsa-miR-4437 | 0.01459363 | 2.440 | 1.497 | 74% | 48% |
| hsa-miR-4440 | 0.00331705 | 5.457 | 5.004 | 80% | 54% |
| hsa-miR-4441 | 0.01566187 | 5.487 | 5.068 | 59% | 45% |
| hsa-miR-4443 | 0.00010436 | 6.175 | 5.609 | 72% | 42% |
| hsa-miR-4445-3p | 0.00607861 | 2.418 | 1.605 | 71% | 48% |
| hsa-miR-4447 | 0.01094891 | 8.267 | 8.080 | 70% | 50% |
| hsa-miR-4448 | 0.00010756 | 4.271 | 3.757 | 75% | 50% |
| hsa-miR-4449 | 0.00122524 | 5.859 | 5.487 | 74% | 46% |
| hsa-miR-4451 | 0.01111579 | 2.391 | 1.514 | 73% | 50% |
| hsa-miR-4452 | 0 | 0.009 | 0.063 | 99% | 98% |
| hsa-miR-4453 | 2.61E-05 | 3.085 | 1.904 | 77% | 47% |
| hsa-miR-4457 | 0 | 0.000 | 0.019 | 100% | 99% |
| hsa-miR-445 8 | 0.00301993 | 2.392 | 1.553 | 75% | 47% |
| hsa-miR-4459 | 0.03623045 | 8.124 | 7.943 | 61% | 41% |
| hsa-miR-4462 | 0.01194283 | 6.441 | 6.002 | 58% | 48% |
| hsa-miR-4470 | 0.03781258 | 2.638 | 1.481 | 64% | 42% |
| hsa-miR-4472 | 8.89E-05 | 7.139 | 6.666 | 66% | 44% |
| hsa-miR-4474-3p | 3.37E-05 | 3.576 | 2.694 | 79% | 53% |
| hsa-miR-4474-5p | 0 | 0.030 | 0.100 | 99% | 97% |
| hsa-miR-4477b | 0 | 0.000 | 0.035 | 100% | 99% |
| hsa-miR-4478 | 0.01196084 | 6.689 | 6.401 | 63% | 51% |
| hsa-miR-4479 | 0.01804988 | 3.185 | 3.071 | 70% | 65% |
| hsa-miR-448 | 4.59E-06 | 0.389 | 1.095 | 76% | 54% |
| hsa-miR-4480 | 0 | 0.035 | 0.095 | 99% | 96% |
| hsa-miR-4481 | 0.0173652 | 4.872 | 4.437 | 75% | 57% |
| hsa-miR-4483 | 8.84E-05 | 6.389 | 5.830 | 68% | 47% |
| hsa-miR-4486 | 0.00018648 | 7.515 | 7.041 | 71% | 52% |
| hsa-miR-4487 | 0.00066402 | 4.877 | 4.276 | 76% | 57% |
| hsa-miR-4489 | 1.94E-05 | 4.651 | 3.926 | 80% | 60% |
| hsa-miR-4495 | 0 | 0.000 | 0.049 | 100% | 97% |
| hsa-miR-4496 | 9.87E-06 | 6.015 | 5.328 | 66% | 39% |
| hsa-miR-4498 | 0.00947555 | 5.058 | 4.635 | 75% | 54% |
| hsa-miR-4499 | 7.92E-06 | 6.928 | 6.239 | 61% | 40% |
| hsa-miR-449c-5p | 9.66E-05 | 2.685 | 1.827 | 73% | 49% |
| hsa-miR-4501 | 0.04522928 | 0.083 | 0.254 | 95% | 92% |
| hsa-miR-4502 | 0.01179768 | 2.728 | 1.913 | 69% | 51% |
| hsa-miR-4503 | 0.04732249 | 0.046 | 0.167 | 98% | 95% |
| hsa-miR-4504 | 0 | 0.000 | 0.052 | 100% | 98% |
| hsa-miR-4507 | 0.00678417 | 7.017 | 6.649 | 67% | 54% |
| hsa-miR-4509 | 0 | 0.000 | 0.174 | 100% | 95% |
| hsa-miR-450a-1-3p | 0 | 0.031 | 0.204 | 99% | 94% |
| hsa-miR-450a-2-3p | 1.10E-05 | 0.446 | 1.399 | 78% | 50% |
| hsa-miR-450a-5p | 0.03609498 | 0.852 | 1.371 | 63% | 46% |
| hsa-miR-450b-3p | 0 | 0.024 | 0.271 | 99% | 91% |
| hsa-miR-4514 | 0.0333188 | 2.327 | 1.515 | 67% | 47% |
| hsa-miR-4515 | 0.02441736 | 3.054 | 2.774 | 72% | 54% |
| hsa-miR-4518 | 0.04737878 | 3.264 | 2.856 | 69% | 54% |
| hsa-miR-4520-5p | 5.38E-06 | 3.105 | 2.001 | 73% | 50% |
| hsa-miR-4521 | 0.03449917 | 1.255 | 1.379 | 54% | 51% |
| hsa-miR-4522 | 0.00151918 | 0.850 | 1.193 | 58% | 51% |
| hsa-miR-452-3p | 7.96E-07 | 0.381 | 1.540 | 80% | 40% |
| hsa-miR-4524a-3p | 0.00217262 | 0.247 | 0.467 | 83% | 79% |
| hsa-miR-4525 | 1.67E-19 | 6.509 | 5.146 | 88% | 36% |
| hsa-miR-4529-3p | 0.00815565 | 2.807 | 2.416 | 70% | 58% |
| hsa-miR-4529-5p | 0.00074518 | 2.192 | 1.305 | 67% | 44% |
| hsa-miR-4533 | 6.90E-05 | 4.830 | 4.290 | 81% | 49% |
| hsa-miR-4535 | 6.33E-05 | 5.044 | 4.521 | 74% | 60% |
| hsa-miR-4538 | 3.20E-12 | 3.964 | 2.354 | 81% | 41% |
| hsa-miR-4539 | 8.52E-06 | 5.105 | 4.365 | 79% | 55% |
| hsa-miR-4540 | 0.000281 | 3.458 | 2.690 | 74% | 56% |
| hsa-miR-454-5p | 0.01526153 | 0.929 | 1.061 | 60% | 57% |
| hsa-miR-455-5p | 0 | 0.000 | 0.051 | 100% | 98% |
| hsa-miR-4633-3p | 0 | 0.027 | 0.196 | 99% | 92% |
| hsa-miR-4635 | 7.29E-05 | 2.847 | 2.257 | 70% | 57% |
| hsa-miR-4638-5p | 1.96E-05 | 4.819 | 4.136 | 77% | 55% |
| hsa-miR-4639-3p | 0.00923773 | 3.640 | 3.228 | 73% | 60% |
| hsa-miR-4639-5p | 0 | 0.019 | 0.238 | 99% | 90% |
| hsa-miR-4643 | 0.04448326 | 0.661 | 0.930 | 67% | 61% |
| hsa-miR-4644 | 0.00559404 | 3.271 | 2.176 | 79% | 51% |
| hsa-miR-4646-5p | 0.00464534 | 5.451 | 5.140 | 75% | 53% |
| hsa-miR-4647 | 0.00026679 | 3.421 | 2.386 | 79% | 52% |
| hsa-miR-4650-3p | 0.01059244 | 0.221 | 0.406 | 86% | 83% |
| hsa-miR-4650-5p | 0.00221428 | 0.651 | 0.872 | 67% | 64% |
| hsa-miR-4653-3p | 0.02388503 | 0.807 | 0.897 | 64% | 67% |
| hsa-miR-4654 | 0.00052163 | 3.557 | 2.653 | 80% | 53% |
| hsa-miR-4657 | 0.00024172 | 2.709 | 1.394 | 76% | 39% |
| hsa-miR-4659a-3p | 0.00293562 | 0.134 | 0.181 | 91% | 92% |
| hsa-miR-466 | 0.00077285 | 3.988 | 3.274 | 72% | 57% |
| hsa-miR-4661-5p | 0.03100559 | 0.759 | 1.250 | 63% | 44% |
| hsa-miR-4665-5p | 0.00054954 | 8.031 | 7.707 | 65% | 46% |
| hsa-miR-4668-3p | 0 | 0.000 | 0.032 | 100% | 99% |
| hsa-miR-4669 | 0.00018363 | 4.324 | 3.826 | 73% | 54% |
| hsa-miR-4671-3p | 0 | 0.000 | 0.137 | 100% | 96% |
| hsa-miR-4676-5p | 1.72E-05 | 3.774 | 3.001 | 75% | 53% |
| hsa-miR-4677-5p | 0.00015205 | 0.497 | 0.722 | 74% | 68% |
| hsa-miR-4679 | 0 | 0.000 | 0.035 | 100% | 99% |
| hsa-miR-4681 | 0.0032243 | 1.756 | 1.067 | 61% | 42% |
| hsa-miR-4683 | 0.00147498 | 0.051 | 0.246 | 94% | 90% |
| hsa-miR-4684-5p | 0.01429916 | 3.018 | 2.883 | 68% | 63% |
| hsa-miR-4686 | 1.04E-05 | 3.361 | 2.448 | 76% | 48% |
| hsa-miR-4690-5p | 9.00E-06 | 6.879 | 6.389 | 75% | 45% |
| hsa-miR-4691-3p | 0.00481546 | 0.188 | 0.238 | 85% | 89% |
| hsa-miR-4691-5p | 2.27E-08 | 3.958 | 3.083 | 81% | 49% |
| hsa-miR-4692 | 0.00382063 | 2.906 | 1.784 | 73% | 47% |
| hsa-miR-4693-3p | 0 | 0.000 | 0.020 | 100% | 99% |
| hsa-miR-4694-5p | 0.0088459 | 0.522 | 0.656 | 70% | 70% |
| hsa-miR-4699-3p | 0.03361995 | 0.054 | 0.104 | 94% | 93% |
| hsa-miR-4699-5p | 0 | 0.000 | 0.151 | 100% | 96% |
| hsa-miR-4700-5p | 2.61E-08 | 3.907 | 2.924 | 76% | 51% |
| hsa-miR-4701-3p | 0.00117201 | 4.068 | 3.469 | 72% | 56% |
| hsa-miR-4704-3p | 0 | 0.015 | 0.018 | 99% | 99% |
| hsa-miR-4704-5p | 0.03036051 | 0.554 | 0.670 | 69% | 72% |
| hsa-miR-4706 | 0.0240398 | 9.015 | 8.775 | 69% | 48% |
| hsa-miR-4708-3p | 0.04254016 | 3.946 | 3.555 | 74% | 59% |
| hsa-miR-4709-3p | 0.03885052 | 5.067 | 4.675 | 75% | 55% |
| hsa-miR-4710 | 9.37E-05 | 5.817 | 5.351 | 76% | 46% |
| hsa-miR-4711-3p | 0.00423159 | 3.084 | 2.428 | 73% | 53% |
| hsa-miR-4714-3p | 0 | 0.000 | 0.064 | 100% | 98% |
| hsa-miR-4717-3p | 0.02807998 | 4.985 | 4.756 | 68% | 54% |
| hsa-miR-4719 | 0.01815202 | 0.081 | 0.182 | 94% | 92% |
| hsa-miR-4720-3p | 0 | 0.008 | 0.010 | 99% | 99% |
| hsa-miR-4721 | 0.00101792 | 5.344 | 4.764 | 78% | 56% |
| hsa-miR-4724-3p | 0.00652906 | 0.558 | 0.663 | 68% | 71% |
| hsa-miR-4727-3p | 6.82E-06 | 4.305 | 2.859 | 72% | 41% |
| hsa-miR-4732-3p | 0.03820788 | 4.211 | 3.927 | 70% | 60% |
| hsa-miR-4735-5p | 0 | 0.000 | 0.068 | 100% | 98% |
| hsa-miR-4743-5p | 0.00651794 | 4.820 | 4.376 | 75% | 60% |
| hsa-miR-4746-3p | 0.04526768 | 5.756 | 5.643 | 66% | 52% |
| hsa-miR-4747-5p | 0.00106533 | 6.775 | 6.460 | 64% | 48% |
| hsa-miR-4754 | 0.03583102 | 2.787 | 1.852 | 70% | 51% |
| hsa-miR-4755-5p | 0.00235492 | 0.971 | 1.169 | 57% | 55% |
| hsa-miR-4756-3p | 0.0009249 | 2.659 | 2.058 | 73% | 53% |
| hsa-miR-4757-3p | 0 | 0.037 | 0.169 | 99% | 91% |
| hsa-miR-4760-5p | 0 | 0.000 | 0.096 | 100% | 97% |
| hsa-miR-4762-5p | 3.16E-07 | 0.578 | 0.868 | 68% | 66% |
| hsa-miR-4767 | 0.00014316 | 4.077 | 3.761 | 71% | 46% |
| hsa-miR-4769-5p | 0.00036279 | 4.460 | 3.898 | 73% | 52% |
| hsa-miR-4772-5p | 0 | 0.000 | 0.150 | 100% | 95% |
| hsa-miR-4774-3p | 0.02218661 | 0.179 | 0.249 | 87% | 89% |
| hsa-miR-4776-3p | 4.09E-05 | 3.545 | 2.933 | 69% | 50% |
| hsa-miR-4776-5p | 7.40E-05 | 4.410 | 3.518 | 79% | 56% |
| hsa-miR-4777-5p | 0 | 0.017 | 0.070 | 99% | 98% |
| hsa-miR-4781-3p | 0 | 0.000 | 0.049 | 100% | 98% |
| hsa-miR-4781-5p | 0.00035955 | 0.247 | 0.345 | 83% | 86% |
| hsa-miR-4782-3p | 0 | 0.000 | 0.137 | 100% | 94% |
| hsa-miR-4782-5p | 0 | 0.000 | 0.070 | 100% | 98% |
| hsa-miR-4784 | 0.00030077 | 3.259 | 2.011 | 75% | 50% |
| hsa-miR-4785 | 0.00050976 | 3.010 | 2.327 | 78% | 53% |
| hsa-miR-4790-3p | 0 | 0.000 | 0.152 | 100% | 94% |
| hsa-miR-4791 | 0 | 0.008 | 0.194 | 99% | 93% |
| hsa-miR-4795-5p | 0 | 0.007 | 0.046 | 99% | 98% |
| hsa-miR-4796-3p | 0 | 0.027 | 0.033 | 99% | 98% |
| hsa-miR-4796-5p | 0.00389725 | 2.596 | 1.861 | 69% | 51% |
| hsa-miR-4798-5p | 0 | 0.019 | 0.100 | 99% | 97% |
| hsa-miR-4799-3p | 0 | 0.000 | 0.018 | 100% | 99% |
| hsa-miR-4799-5p | 0.00934704 | 0.053 | 0.181 | 95% | 92% |
| hsa-miR-4800-5p | 2.69E-06 | 5.426 | 4.538 | 75% | 46% |
| hsa-miR-4802-5p | 0 | 0.015 | 0.109 | 99% | 97% |
| hsa-miR-4804-5p | 0.02730143 | 0.213 | 0.309 | 87% | 85% |
| hsa-miR-487a-5p | 0.00813458 | 3.824 | 3.679 | 65% | 64% |
| hsa-miR-491-3p | 0.00144427 | 0.570 | 0.785 | 68% | 66% |
| hsa-miR-492 | 0.00069228 | 0.952 | 1.500 | 58% | 44% |
| hsa-miR-494-5p | 0.00258085 | 3.532 | 3.066 | 69% | 59% |
| hsa-miR-499a-5p | 0 | 0.000 | 0.031 | 100% | 98% |
| hsa-miR-5002-3p | 0.00085337 | 4.099 | 3.593 | 73% | 60% |
| hsa-miR-5002-5p | 0.00794951 | 0.215 | 0.403 | 87% | 83% |
| hsa-miR-5006-5p | 0.00105343 | 4.094 | 3.178 | 75% | 52% |
| hsa-miR-5009-3p | 0 | 0.017 | 0.040 | 99% | 97% |
| hsa-miR-5009-5p | 0 | 0.000 | 0.064 | 100% | 98% |
| hsa-miR-500a-5p | 0.0167035 | 0.605 | 0.944 | 73% | 63% |
| hsa-miR-5010-Sp | 0.00175832 | 5.431 | 4.664 | 61% | 46% |
| hsa-miR-5011-5p | 0.01212642 | 0.076 | 0.276 | 93% | 87% |
| hsa-miR-504-3p | 0.0452157 | 5.401 | 5.175 | 66% | 56% |
| hsa-miR-505-5p | 0.0003644 | 4.787 | 4.085 | 80% | 56% |
| hsa-miR-506-5p | 0 | 0.000 | 0.078 | 100% | 97% |
| hsa-miR-507 | 0 | 0.015 | 0.152 | 99% | 94% |
| hsa-miR-5089-3p | 0.00206563 | 1.508 | 1.464 | 58% | 50% |
| hsa-miR-5089-5p | 0.00591895 | 0.084 | 0.358 | 94% | 88% |
| hsa-miR-5092 | 1.50E-06 | 0.130 | 0.497 | 88% | 78% |
| hsa-miR-5093 | 0.00273831 | 2.725 | 1.631 | 74% | 46% |
| hsa-miR-509-5p | 0.02808621 | 0.257 | 0.416 | 84% | 82% |
| hsa-miR-510-3p | 0 | 0.015 | 0.017 | 99% | 99% |
| hsa-miR-513b-3p | 0 | 0.000 | 0.174 | 100% | 94% |
| hsa-miR-514a-5p | 0 | 0.000 | 0.096 | 100% | 97% |
| hsa-miR-515-3p | 0.02144541 | 1.107 | 1.151 | 52% | 55% |
| hsa-miR-515-5p | 0.00903905 | 1.637 | 1.520 | 52% | 54% |
| hsa-miR-516b-3p, hsa-miR-516a-3p | 0.00071511 | 0.791 | 1.214 | 61% | 50% |
| hsa-miR-516b-5p | 0.0025262 | 0.060 | 0.415 | 95% | 78% |
| hsa-miR-517-5p | 5.24E-06 | 0.513 | 1.168 | 71% | 49% |
| hsa-miR-5189-3p | 0.01282767 | 4.494 | 4.198 | 68% | 58% |
| hsa-miR-5189-5p | 2.58E-10 | 4.735 | 3.615 | 81% | 47% |
| hsa-miR-5191 | 0 | 0.007 | 0.042 | 99% | 99% |
| hsa-miR-5192 | 0.04258541 | 1.880 | 1.805 | 60% | 56% |
| hsa-miR-519a-3p | 0.00034884 | 0.350 | 0.809 | 79% | 65% |
| hsa-miR-519b-3p | 0.00548804 | 0.543 | 0.905 | 73% | 60% |
| hsa-miR-519c-5p, hsa-miR-523-5p, hsa-miR-518e-5p, hsa-miR-522-5p, hsa-miR-519a-5p, hsa-miR-519b-5p | 1.42E-05 | 2.984 | 2.353 | 65% | 44% |
| hsa-miR-520b | 0.00233977 | 5.747 | 5.070 | 64% | 48% |
| hsa-miR-520d-3p | 0.0004582 | 0.274 | 0.925 | 82% | 59% |
| hsa-miR-520d-5p | 1.34E-06 | 5.328 | 4.726 | 67% | 38% |
| hsa-miR-520e | 0.00357817 | 5.972 | 5.483 | 63% | 46% |
| hsa-miR-520f-3p | 0.00464205 | 0.036 | 0.111 | 95% | 95% |
| hsa-miR-526b-5p | 0.03257759 | 4.279 | 3.696 | 73% | 51% |
| hsa-miR-527, hsa-miR-518a-5p | 0.00041504 | 4.933 | 4.516 | 63% | 42% |
| hsa-miR-539-5p | 0.02269097 | 0.220 | 0.442 | 85% | 78% |
| hsa-miR-542-5p | 0.00610662 | 0.154 | 0.255 | 89% | 89% |
| hsa-miR-544a | 0 | 0.000 | 0.022 | 100% | 99% |
| hsa-miR-545-5p | 0 | 0.000 | 0.040 | 100% | 99% |
| hsa-miR-548a-3p | 0 | 0.029 | 0.259 | 99% | 89% |
| hsa-miR-548a-5p | 0 | 0.000 | 0.070 | 100% | 98% |
| hsa-miR-548aa, hsa-miR-548t-3p | 0 | 0.022 | 0.051 | 99% | 98% |
| hsa-miR-548ac | 0 | 0.000 | 0.116 | 100% | 96% |
| hsa-miR-548ad-5p, hsa-miR-548ae-5p | 0 | 0.000 | 0.026 | 100% | 99% |
| hsa-miR-548ae-3p | 0 | 0.000 | 0.098 | 100% | 97% |
| hsa-miR-548ag | 0 | 0.000 | 0.073 | 100% | 98% |
| hsa-miR-548ah-5p | 0 | 0.000 | 0.033 | 100% | 99% |
| hsa-miR-548ak | 0 | 0.000 | 0.012 | 100% | 99% |
| hsa-miR-548al | 0 | 0.000 | 0.017 | 100% | 99% |
| hsa-miR-548am-3p | 0 | 0.000 | 0.089 | 100% | 97% |
| hsa-miR-548ao-5p | 0 | 0.000 | 0.206 | 100% | 93% |
| hsa-miR-548ap-5p | 0 | 0.019 | 0.050 | 99% | 99% |
| hsa-miR-548aq-5p | 0 | 0.000 | 0.045 | 100% | 98% |
| hsa-miR-548at-3p | 0 | 0.000 | 0.036 | 100% | 98% |
| hsa-miR-548au-5p | 0 | 0.000 | 0.025 | 100% | 99% |
| hsa-miR-548az-5p | 0 | 0.000 | 0.053 | 100% | 98% |
| hsa-miR-548b-5p | 0 | 0.000 | 0.088 | 100% | 97% |
| hsa-miR-548ba | 0.00165638 | 0.545 | 0.991 | 75% | 66% |
| hsa-miR-548c-5p, hsa-miR-548o-5p, hsa-miR-548am-5p | 0 | 0.029 | 0.125 | 99% | 96% |
| hsa-miR-548g-5p, hsa-miR-548x-5p, hsa-miR-548aj-5p | 0 | 0.022 | 0.085 | 99% | 96% |
| hsa-miR-548j-5p | 0 | 0.000 | 0.236 | 100% | 92% |
| hsa-miR-548k | 0 | 0.000 | 0.024 | 100% | 99% |
| hsa-miR-548m | 0 | 0.000 | 0.042 | 100% | 99% |
| hsa-miR-548p | 0 | 0.050 | 0.079 | 98% | 97% |
| hsa-miR-548t-5p | 0 | 0.017 | 0.036 | 99% | 98% |
| hsa-miR-548u | 0 | 0.000 | 0.131 | 100% | 95% |
| hsa-miR-548v | 0 | 0.033 | 0.077 | 99% | 96% |
| hsa-miR-548x-3p | 0 | 0.000 | 0.049 | 100% | 99% |
| hsa-miR-549a | 0 | 0.000 | 0.024 | 100% | 99% |
| hsa-miR-552-3p | 0.0446735 | 0.333 | 0.437 | 80% | 82% |
| hsa-miR-553 | 0 | 0.000 | 0.095 | 100% | 97% |
| hsa-miR-554 | 0.00034125 | 0.778 | 1.307 | 63% | 47% |
| hsa-miR-556-3p | 0 | 0.055 | 0.200 | 97% | 90% |
| hsa-miR-5579-5p | 0 | 0.000 | 0.085 | 100% | 96% |
| hsa-miR-5580-5p | 0.00546904 | 2.456 | 1.763 | 68% | 51% |
| hsa-miR-5581-5p | 0.00022192 | 0.221 | 1.009 | 88% | 65% |
| hsa-miR-5583-5p | 0 | 0.020 | 0.148 | 99% | 96% |
| hsa-miR-5588-3p | 0.03919453 | 2.833 | 2.539 | 68% | 59% |
| hsa-miR-5588-5p | 0.00148361 | 0.178 | 0.249 | 86% | 89% |
| hsa-miR-5589-3p | 0.00024323 | 0.159 | 0.563 | 88% | 76% |
| hsa-miR-5589-5p | 0.00010002 | 4.863 | 4.111 | 79% | 51% |
| hsa-miR-559 | 0 | 0.000 | 0.026 | 100% | 99% |
| hsa-miR-5590-3p | 0 | 0.019 | 0.105 | 99% | 96% |
| hsa-miR-5590-5p | 0 | 0.000 | 0.086 | 100% | 97% |
| hsa-miR-5591-5p | 0 | 0.000 | 0.015 | 100% | 98% |
| hsa-miR-561-5p | 0 | 0.016 | 0.024 | 99% | 99% |
| hsa-miR-563 | 0.00053723 | 0.203 | 0.481 | 84% | 76% |
| hsa-miR-564 | 0.00052246 | 3.969 | 3.565 | 73% | 52% |
| hsa-miR-568 | 0 | 0.025 | 0.132 | 99% | 95% |
| hsa-miR-5684 | 0.02257736 | 0.291 | 0.351 | 82% | 82% |
| hsa-miR-5687 | 0 | 0.000 | 0.126 | 100% | 96% |
| hsa-miR-569 | 0 | 0.000 | 0.086 | 100% | 96% |
| hsa-miR-5692c | 0 | 0.000 | 0.089 | 100% | 97% |
| hsa-miR-5696 | 0.0294399 | 0.606 | 0.817 | 70% | 65% |
| hsa-miR-5697 | 0 | 0.023 | 0.067 | 99% | 97% |
| hsa-miR-5698 | 4.51E-12 | 6.547 | 5.515 | 76% | 37% |
| hsa-miR-5700 | 0 | 0.000 | 0.130 | 100% | 95% |
| hsa-miR-5701 | 0.00021222 | 0.049 | 0.282 | 95% | 90% |
| hsa-miR-5702 | 8.29E-05 | 0.822 | 1.372 | 64% | 51% |
| hsa-miR-570-3p | 0 | 0.022 | 0.133 | 99% | 94% |
| hsa-miR-5708 | 0.00096822 | 3.511 | 2.912 | 70% | 55% |
| hsa-miR-576-5p | 5.57E-08 | 0.670 | 1.528 | 66% | 40% |
| hsa-miR-577 | 0 | 0.010 | 0.049 | 99% | 98% |
| hsa-miR-578 | 0.04517329 | 0.686 | 1.082 | 66% | 52% |
| hsa-miR-580-5p | 0.02283023 | 0.045 | 0.205 | 98% | 94% |
| hsa-miR-582-3p | 0.00708344 | 0.047 | 0.266 | 95% | 87% |
| hsa-miR-582-5p | 0 | 0.037 | 0.148 | 99% | 93% |
| hsa-miR-585-3p | 0.00174237 | 0.226 | 0.827 | 86% | 64% |
| hsa-miR-587 | 0 | 0.019 | 0.120 | 99% | 94% |
| hsa-miR-590-3p | 0 | 0.014 | 0.020 | 99% | 99% |
| hsa-miR-591 | 0.01247906 | 1.188 | 1.735 | 61% | 42% |
| hsa-miR-595 | 0.04521686 | 4.264 | 4.074 | 63% | 57% |
| hsa-miR-597-3p | 0.0029378 | 0.193 | 0.568 | 86% | 73% |
| hsa-miR-598-3p | 6.99E-08 | 0.492 | 1.058 | 70% | 53% |
| hsa-miR-603 | 0.00579367 | 2.480 | 1.967 | 68% | 56% |
| hsa-miR-605-3p | 0.00697943 | 0.295 | 0.408 | 85% | 89% |
| hsa-miR-6068 | 0.00051191 | 4.345 | 3.405 | 73% | 41% |
| hsa-miR-6072 | 0.00361969 | 3.711 | 3.283 | 71% | 61% |
| hsa-miR-6076 | 1.04E-08 | 6.626 | 5.518 | 73% | 36% |
| hsa-miR-6079 | 0 | 0.025 | 0.059 | 99% | 98% |
| hsa-miR-608 | 0.00031597 | 3.315 | 2.679 | 76% | 52% |
| hsa-miR-6082 | 0.0077416 | 0.043 | 0.357 | 96% | 87% |
| hsa-miR-6086 | 3.22E-05 | 8.335 | 7.811 | 64% | 43% |
| hsa-miR-611 | 0.01752937 | 0.334 | 0.627 | 80% | 70% |
| hsa-miR-6127 | 0.00893287 | 6.063 | 5.653 | 64% | 49% |
| hsa-miR-6128 | 0 | 0.000 | 0.040 | 100% | 98% |
| hsa-miR-6129 | 0.00229821 | 2.119 | 1.685 | 66% | 51% |
| hsa-miR-6131 | 4.30E-06 | 6.273 | 5.210 | 67% | 43% |
| hsa-miR-6133 | 0.00563157 | 6.403 | 5.944 | 61% | 44% |
| hsa-miR-614 | 0.00181466 | 3.984 | 2.813 | 69% | 44% |
| hsa-miR-616-3p | 0.00066174 | 0.327 | 0.897 | 82% | 62% |
| hsa-miR-616-5p | 0.00844363 | 0.123 | 0.755 | 90% | 64% |
| hsa-miR-618 | 0 | 0.051 | 0.084 | 99% | 96% |
| hsa-miR-621 | 0 | 0.000 | 0.047 | 100% | 98% |
| hsa-miR-622 | 0.02329794 | 2.659 | 2.365 | 73% | 56% |
| hsa-miR-624-3p | 0.00226389 | 0.216 | 1.045 | 89% | 61% |
| hsa-miR-624-5p | 0.00043226 | 0.178 | 0.343 | 87% | 85% |
| hsa-miR-626 | 0.01549569 | 0.026 | 0.138 | 94% | 93% |
| hsa-miR-628-3p | 3.50E-06 | 0.596 | 1.072 | 71% | 59% |
| hsa-miR-628-5p | 0 | 0.000 | 0.035 | 100% | 98% |
| hsa-miR-630 | 3.34E-06 | 0.555 | 1.367 | 75% | 50% |
| hsa-miR-633 | 0 | 0.000 | 0.028 | 100% | 99% |
| hsa-miR-648 | 0 | 0.000 | 0.031 | 100% | 99% |
| hsa-miR-6499-5p | 0.00405351 | 3.086 | 2.616 | 68% | 61% |
| hsa-miR-650 | 1.75E-05 | 4.109 | 3.056 | 64% | 39% |
| hsa-miR-6500-3p | 0.00076437 | 3.454 | 2.649 | 76% | 54% |
| hsa-miR-6500-5p | 0.01101021 | 0.733 | 0.820 | 59% | 62% |
| hsa-miR-6503-3p | 0.01311065 | 3.354 | 3.102 | 65% | 65% |
| hsa-miR-6504-5p | 0.02360248 | 3.140 | 2.536 | 71% | 60% |
| hsa-miR-6508-3p | 0 | 0.000 | 0.022 | 100% | 99% |
| hsa-miR-651-3p | 0 | 0.000 | 0.272 | 100% | 93% |
| hsa-miR-6514-3p | 0.03946356 | 3.821 | 3.574 | 70% | 63% |
| hsa-miR-6515-5p | 4.87E-07 | 5.610 | 3.885 | 68% | 37% |
| hsa-miR-652-3p | 0 | 0.000 | 0.057 | 100% | 98% |
| hsa-miR-653-5p | 0.00253977 | 0.136 | 0.303 | 88% | 85% |
| hsa-miR-655-5p | 0.03940835 | 0.066 | 0.152 | 93% | 93% |
| hsa-miR-656-3p | 0.00075332 | 0.596 | 1.170 | 72% | 56% |
| hsa-miR-656-5p | 0.01528641 | 2.726 | 1.887 | 74% | 48% |
| hsa-miR-659-5p | 0.01484615 | 0.200 | 0.450 | 87% | 80% |
| hsa-miR-660-5p | 0.00552938 | 0.069 | 0.210 | 93% | 90% |
| hsa-miR-661 | 0.01487103 | 3.726 | 3.458 | 68% | 59% |
| hsa-miR-662 | 0.01119764 | 0.290 | 0.739 | 84% | 69% |
| hsa-miR-668-5p | 0.0021166 | 3.002 | 2.244 | 72% | 52% |
| hsa-miR-6715 a-3p | 0.00596449 | 0.582 | 1.036 | 70% | 53% |
| hsa-miR-6715b-3p | 0.04781373 | 0.022 | 0.052 | 98% | 97% |
| hsa-miR-671-5p | 5.51E-10 | 8.176 | 9.210 | 75% | 32% |
| hsa-miR-6723-5p | 0.00372187 | 4.011 | 3.721 | 67% | 55% |
| hsa-miR-6726-5p | 0.00240814 | 6.865 | 6.432 | 66% | 46% |
| hsa-miR-6730-5p | 0.03153554 | 6.661 | 6.422 | 58% | 52% |
| hsa-miR-6731-5p | 0.02748201 | 7.121 | 7.376 | 56% | 44% |
| hsa-miR-6733-5p | 0 | 0.021 | 0.164 | 99% | 94% |
| hsa-miR-6736-5p | 0.0478419 | 5.551 | 5.315 | 68% | 55% |
| hsa-miR-6738-3p | 0.0369154 | 4.089 | 3.845 | 65% | 61% |
| hsa-miR-6741-5p | 0.02102993 | 8.616 | 8.437 | 63% | 53% |
| hsa-miR-6745 | 1.14E-05 | 6.445 | 5.522 | 64% | 41% |
| hsa-miR-6746-5p | 2.29E-07 | 8.572 | 7.995 | 64% | 39% |
| hsa-miR-6747-5p | 4.87E-05 | 4.296 | 3.100 | 76% | 56% |
| hsa-miR-6751-5p | 0.00191564 | 4.969 | 4.391 | 76% | 55% |
| hsa-miR-6757-5p | 0.00047134 | 11.132 | 11.623 | 63% | 40% |
| hsa-miR-6759-5p | 0.00137306 | 5.667 | 5.102 | 74% | 52% |
| hsa-miR-6760-5p | 2.38E-14 | 7.544 | 6.336 | 75% | 32% |
| hsa-miR-6766-5p | 0.00978604 | 5.727 | 5.380 | 70% | 52% |
| hsa-miR-6767-5p | 0.02637095 | 4.144 | 3.779 | 72% | 55% |
| hsa-miR-6768-3p | 0.0124595 | 0.670 | 0.925 | 66% | 60% |
| hsa-miR-6769a-5p | 0.01601823 | 8.738 | 9.132 | 63% | 45% |
| hsa-miR-6769b-5p | 0.0050574 | 7.271 | 7.670 | 62% | 47% |
| hsa-miR-6772-5p | 1.04E-05 | 5.134 | 4.630 | 77% | 54% |
| hsa-miR-6773-5p | 0.00755862 | 0.015 | 0.277 | 98% | 87% |
| hsa-miR-6776-5p | 0.00015948 | 4.895 | 4.223 | 74% | 59% |
| hsa-miR-6777-5p | 0.00077923 | 7.640 | 7.133 | 67% | 49% |
| hsa-miR-6778-5p | 0.00056848 | 6.796 | 6.474 | 72% | 44% |
| hsa-miR-6780a-5p | 0.0159536 | 5.718 | 5.380 | 64% | 46% |
| hsa-miR-6780b-5p | 0.00258787 | 8.262 | 8.748 | 56% | 45% |
| hsa-miR-6788-5p | 0.01488743 | 3.784 | 2.910 | 81% | 57% |
| hsa-miR-6790-5p | 0.03714632 | 5.745 | 5.484 | 72% | 56% |
| hsa-miR-6793-5p | 0.00875247 | 4.386 | 4.053 | 67% | 55% |
| hsa-miR-6794-5p | 0.00171987 | 8.710 | 8.432 | 71% | 49% |
| hsa-miR-6795-5p | 0.00169703 | 10.229 | 9.869 | 62% | 41% |
| hsa-miR-6796-5p | 3.59E-06 | 5.689 | 4.926 | 65% | 41% |
| hsa-miR-6799-5p | 0.00595169 | 8.768 | 9.254 | 64% | 41% |
| hsa-miR-6801-5p | 4.27E-06 | 3.828 | 2.824 | 77% | 52% |
| hsa-miR-6807-5p | 0.00147904 | 7.632 | 8.133 | 66% | 40% |
| hsa-miR-6811-5p | 0 | 0.000 | 0.013 | 100% | 99% |
| hsa-miR-6812-5p | 0.00030729 | 7.890 | 8.525 | 74% | 48% |
| hsa-miR-6814-5p | 0.01649901 | 1.651 | 1.566 | 61% | 54% |
| hsa-miR-6815-5p | 2.92E-09 | 7.046 | 5.996 | 72% | 41% |
| hsa-miR-6817-5p | 0.00129735 | 2.049 | 1.494 | 64% | 49% |
| hsa-miR-6820-5p | 0.03930169 | 6.363 | 6.062 | 64% | 50% |
| hsa-miR-6821-3p | 0.04582833 | 3.000 | 2.995 | 62% | 64% |
| hsa-miR-6823-5p | 0.03012751 | 3.448 | 2.679 | 79% | 55% |
| hsa-miR-6826-5p | 0.00025996 | 8.467 | 9.222 | 63% | 38% |
| hsa-miR-6827-5p | 0.00111717 | 6.273 | 6.003 | 66% | 50% |
| hsa-miR-6828-5p | 0.01122887 | 4.229 | 3.162 | 81% | 52% |
| hsa-miR-6830-5p | 0.0004425 | 6.519 | 6.083 | 66% | 42% |
| hsa-miR-6833-5p | 0.00087036 | 4.744 | 3.984 | 75% | 46% |
| hsa-miR-6834-5p | 5.08E-06 | 4.303 | 3.522 | 79% | 55% |
| hsa-miR-6842-5p | 0.00462775 | 6.324 | 5.987 | 65% | 50% |
| hsa-miR-6844 | 0 | 0.000 | 0.023 | 100% | 99% |
| hsa-miR-6847-5p | 0.00302839 | 3.455 | 2.795 | 67% | 53% |
| hsa-miR-6849-5p | 0.00392107 | 5.307 | 4.458 | 78% | 51% |
| hsa-miR-6854-5p | 0 | 0.000 | 0.168 | 100% | 94% |
| hsa-miR-6859-5p | 0.00104518 | 4.126 | 3.460 | 76% | 56% |
| hsa-miR-6861-5p | 0.00030233 | 7.400 | 6.992 | 64% | 48% |
| hsa-miR-6864-5p | 0.00417994 | 0.117 | 0.381 | 92% | 86% |
| hsa-miR-6868-5p | 0.00719921 | 2.439 | 1.648 | 71% | 48% |
| hsa-miR-6869-3p | 0.00603758 | 1.177 | 1.250 | 51% | 52% |
| hsa-miR-6871-5p | 9.28E-06 | 4.991 | 4.275 | 76% | 52% |
| hsa-miR-6872-5p | 0.00035614 | 5.186 | 4.449 | 66% | 47% |
| hsa-miR-6876-5p | 3.22E-05 | 4.347 | 3.446 | 81% | 51% |
| hsa-miR-6877-5p | 0.008257 | 6.060 | 5.673 | 72% | 53% |
| hsa-miR-6881-5p | 0.03066244 | 5.686 | 5.247 | 66% | 52% |
| hsa-miR-6884-5p | 0.01155357 | 3.280 | 2.846 | 75% | 62% |
| hsa-miR-6887-5p | 0.00215258 | 10.964 | 10.557 | 62% | 42% |
| hsa-miR-6891-5p | 0.01298897 | 8.155 | 8.614 | 58% | 45% |
| hsa-miR-6892-5p | 1.30E-06 | 4.514 | 3.603 | 78% | 49% |
| hsa-miR-6894-5p | 0.00162823 | 6.212 | 5.910 | 70% | 48% |
| hsa-miR-708-5p | 1.61E-08 | 1.835 | 2.973 | 56% | 45% |
| hsa-miR-7106-5p | 0.00252993 | 8.673 | 8.381 | 64% | 45% |
| hsa-miR-7109-5p | 0.00017292 | 9.028 | 9.606 | 63% | 42% |
| hsa-miR-7151-3p | 1.08E-07 | 4.228 | 3.222 | 83% | 54% |
| hsa-miR-7153-5p | 0.03654835 | 0.138 | 0.222 | 89% | 90% |
| hsa-miR-7154-5p | 0.03374816 | 0.037 | 0.229 | 97% | 91% |
| hsa-miR-7157-5p | 0.049898 | 2.740 | 1.942 | 70% | 52% |
| hsa-miR-7158-3p | 0.00037589 | 0.208 | 0.352 | 84% | 84% |
| hsa-miR-7160-5p | 0.00685358 | 4.948 | 4.657 | 75% | 59% |
| hsa-miR-7161-3p | 0 | 0.000 | 0.113 | 100% | 93% |
| hsa-miR-7162-5p | 1.12E-07 | 0.321 | 0.588 | 78% | 76% |
| hsa-miR-718 | 0.00070095 | 6.748 | 6.424 | 69% | 45% |
| hsa-miR-758-3p | 0.00896657 | 0.037 | 0.269 | 97% | 89% |
| hsa-miR-758-5p | 0.00455092 | 2.192 | 1.399 | 70% | 49% |
| hsa-miR-759 | 0 | 0.012 | 0.066 | 99% | 97% |
| hsa-miR-7-5p | 0 | 0.000 | 0.019 | 100% | 99% |
| hsa-miR-766-5p | 0.00076503 | 2.796 | 1.793 | 75% | 53% |
| hsa-miR-769-5p | 0.01455232 | 0.585 | 0.607 | 71% | 74% |
| hsa-miR-7843-3p | 0.04809694 | 0.195 | 0.374 | 87% | 82% |
| hsa-miR-7850-5p | 0.00068839 | 3.179 | 2.380 | 70% | 56% |
| hsa-miR-7851-3p | 2.16E-06 | 4.621 | 3.971 | 70% | 50% |
| hsa-miR-7852-3p | 0 | 0.000 | 0.028 | 100% | 99% |
| hsa-miR-7975 | 0.0120438 | 2.820 | 2.366 | 71% | 57% |
| hsa-miR-8058 | 0.00815544 | 0.104 | 0.527 | 92% | 78% |
| hsa-miR-8064 | 0.02219846 | 4.070 | 3.717 | 75% | 62% |
| hsa-miR-8071 | 0.00292833 | 5.763 | 5.256 | 72% | 49% |
| hsa-miR-8074 | 3.21E-05 | 1.154 | 1.355 | 53% | 52% |
| hsa-miR-8075 | 0.00820756 | 3.731 | 3.283 | 73% | 58% |
| hsa-miR-8077 | 0.00328947 | 3.794 | 3.185 | 75% | 57% |
| hsa-miR-8079 | 0.00083095 | 0.350 | 0.433 | 76% | 79% |
| hsa-miR-8080 | 0.00463184 | 1.233 | 1.242 | 49% | 55% |
| hsa-miR-8082 | 0.02997473 | 1.997 | 1.538 | 65% | 54% |
| hsa-miR-8083 | 0.01829213 | 2.511 | 1.905 | 71% | 52% |
| hsa-miR-8085 | 0.0010324 | 5.890 | 5.513 | 75% | 51% |
| hsa-miR-8086 | 0.01251524 | 0.562 | 0.689 | 70% | 70% |
| hsa-miR-873-5p | 0 | 0.021 | 0.065 | 99% | 98% |
| hsa-miR-876-5p | 0 | 0.000 | 0.041 | 100% | 99% |
| hsa-miR-877-5p | 0.00484109 | 3.912 | 3.140 | 75% | 51% |
| hsa-miR-891a-3p | 0.00034888 | 0.406 | 0.909 | 76% | 63% |
| hsa-miR-891a-5p | 0.00161552 | 3.133 | 2.385 | 70% | 58% |
| hsa-miR-892c-3p | 0.00055305 | 0.663 | 0.885 | 68% | 66% |
| hsa-miR-921 | 0.00021736 | 2.737 | 1.657 | 77% | 48% |
| hsa-miR-922 | 0.03625765 | 0.044 | 0.138 | 96% | 93% |
| hsa-miR-93-3p | 0.03928919 | 0.620 | 0.971 | 68% | 56% |
| hsa-miR-936 | 6.79E-10 | 6.928 | 6.165 | 68% | 35% |
| hsa-miR-9-3p | 0 | 0.020 | 0.096 | 99% | 96% |
| hsa-miR-95-3p | 0 | 0.033 | 0.156 | 99% | 94% |
| hsa-miR-95-5p | 0 | 0.000 | 0.202 | 100% | 94% |
| hsa-miR-9-5p | 0 | 0.010 | 0.019 | 99% | 99% |
| hsa-miR-96-3p | 0.00645151 | 0.248 | 0.378 | 82% | 83% |
| hsa-miR-96-5p | 0.03456922 | 0.259 | 0.643 | 85% | 73% |
| hsa-miR-99a-3p | 0.00292302 | 0.157 | 0.434 | 90% | 82% |

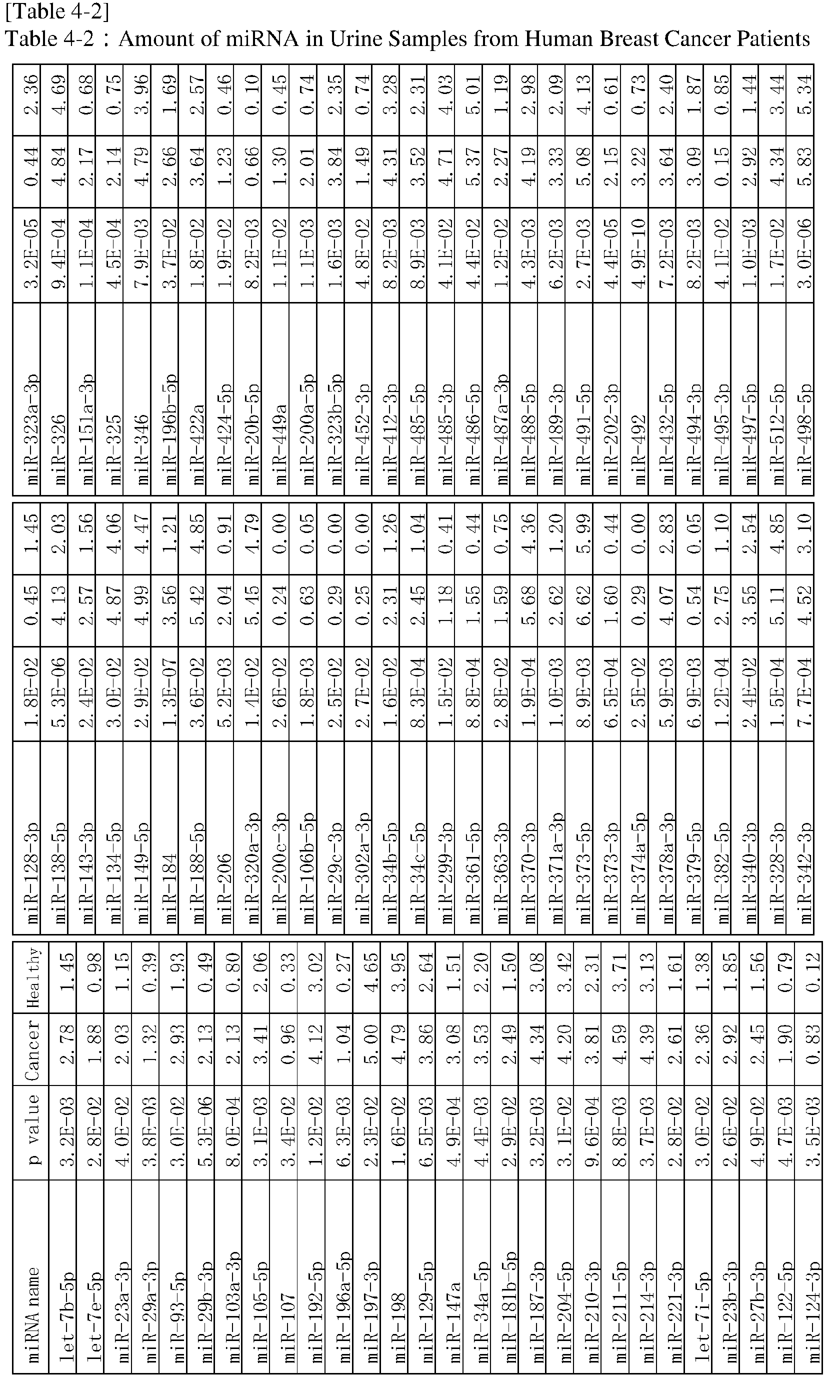

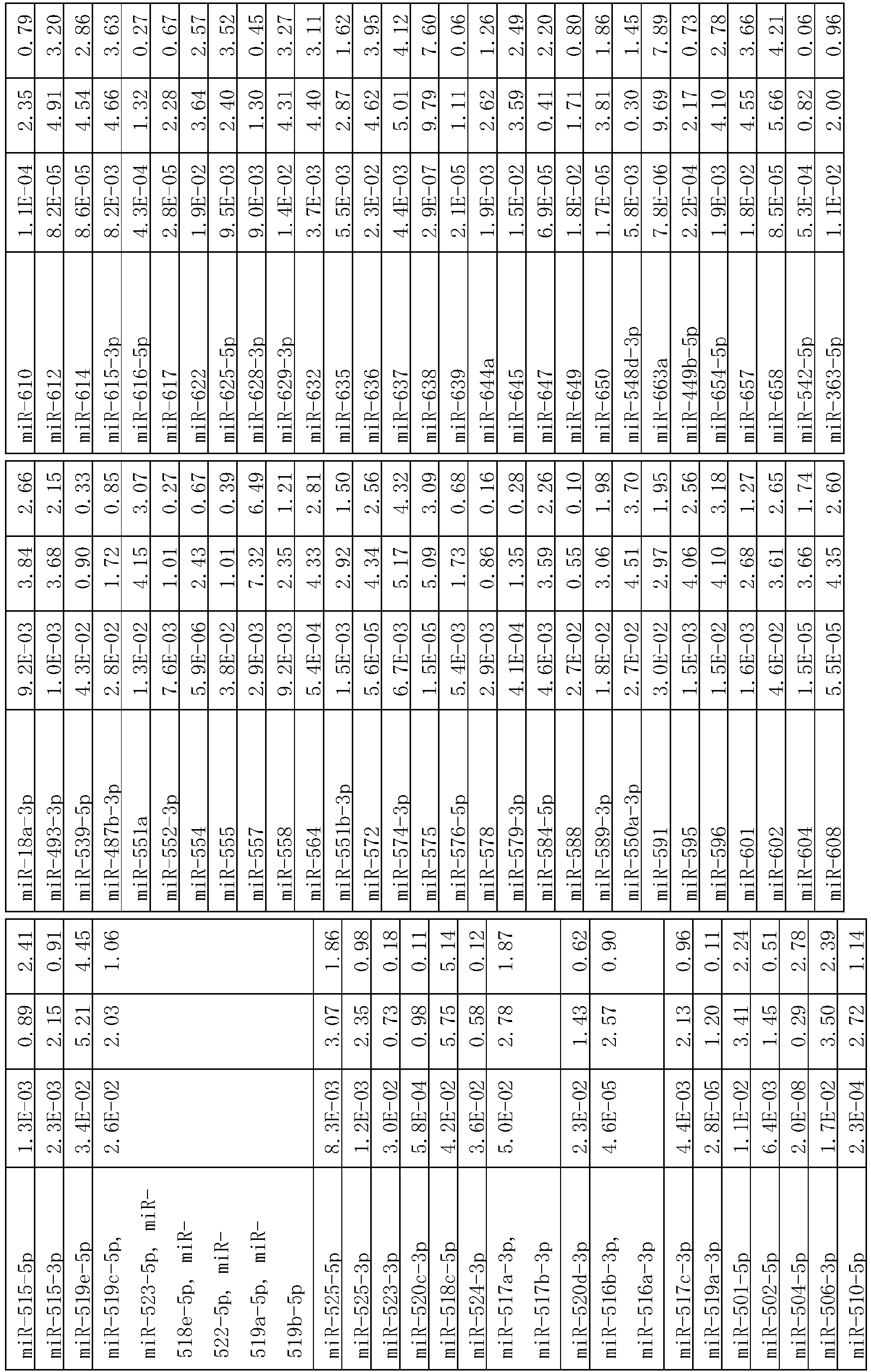

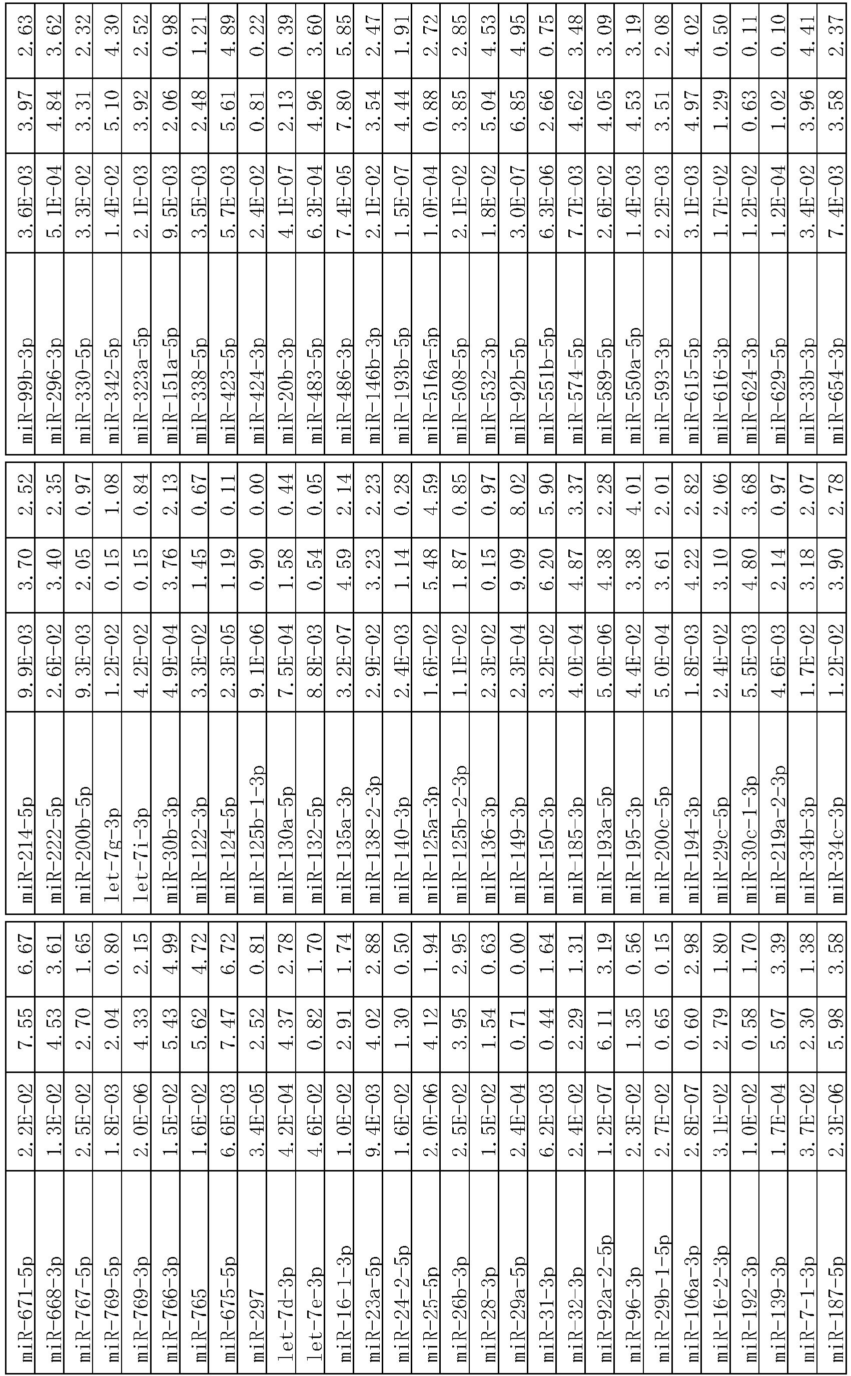

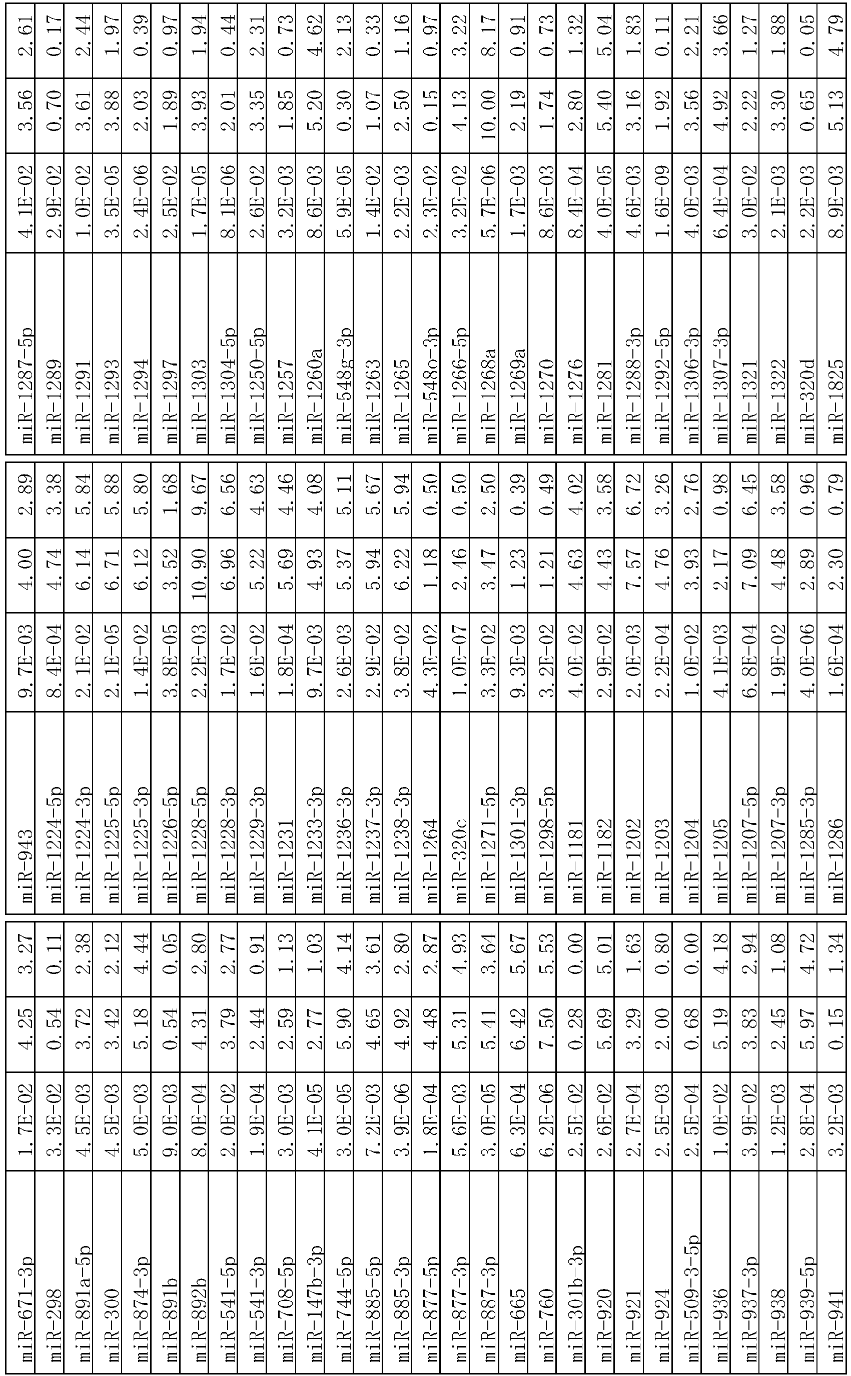

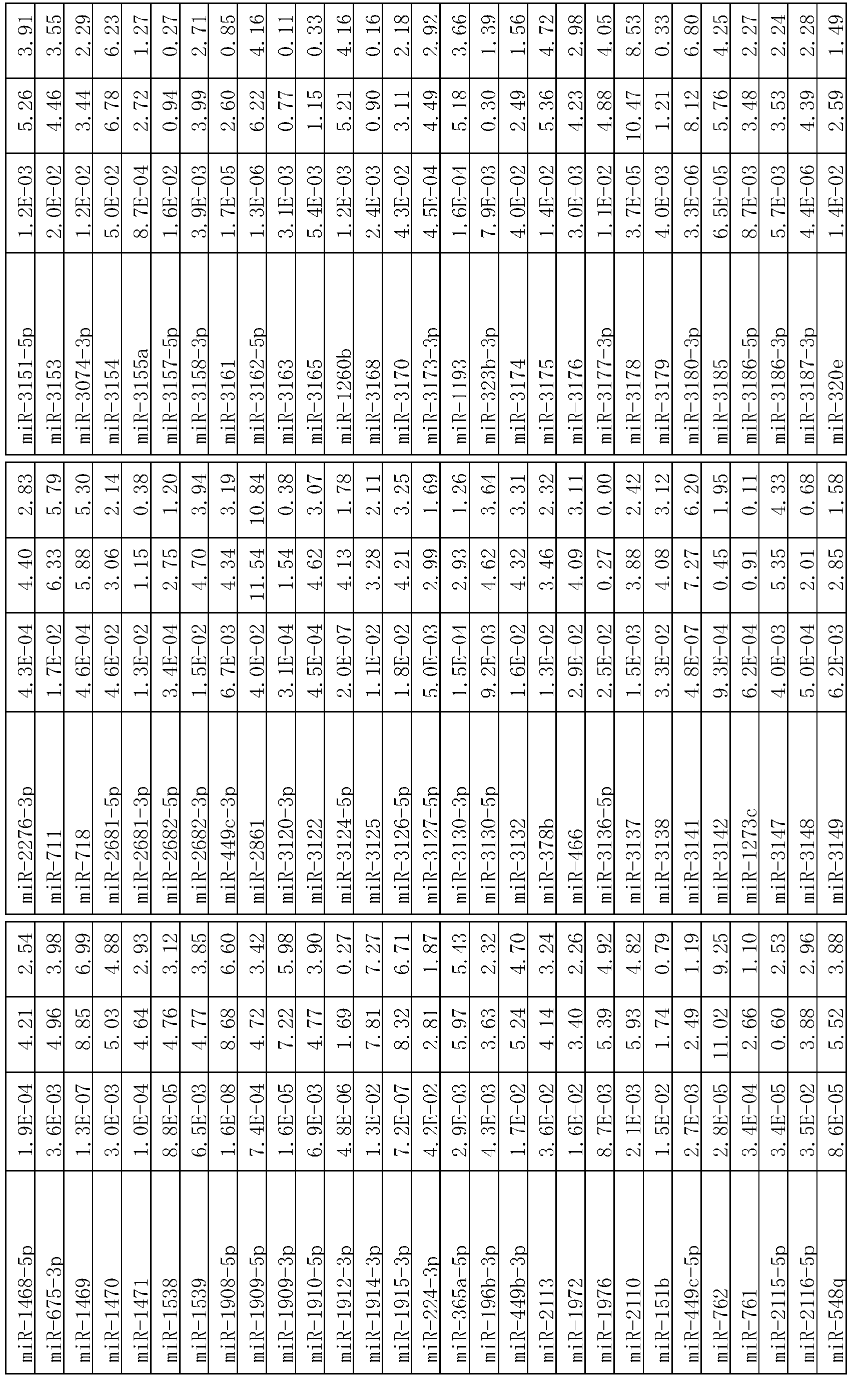

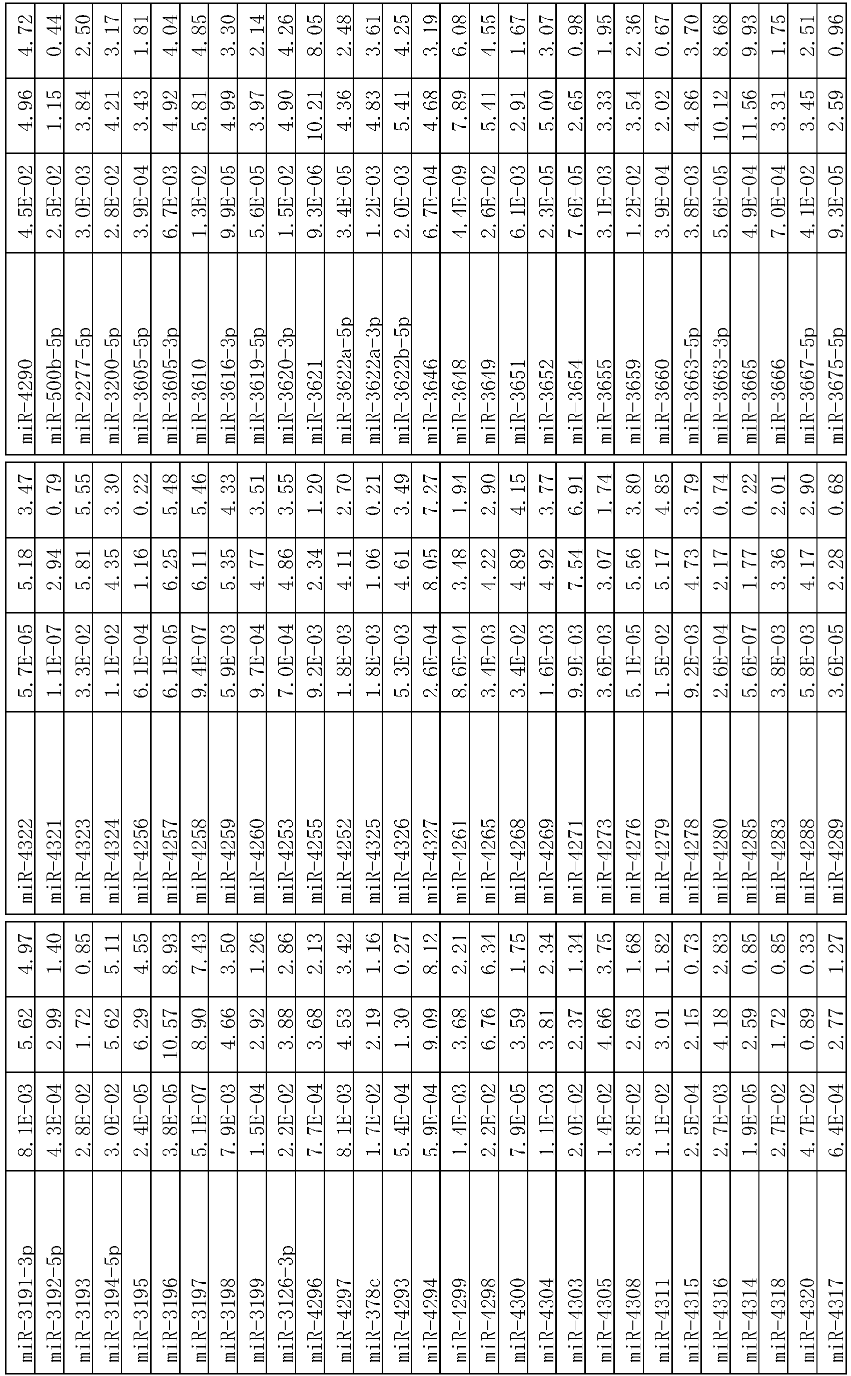

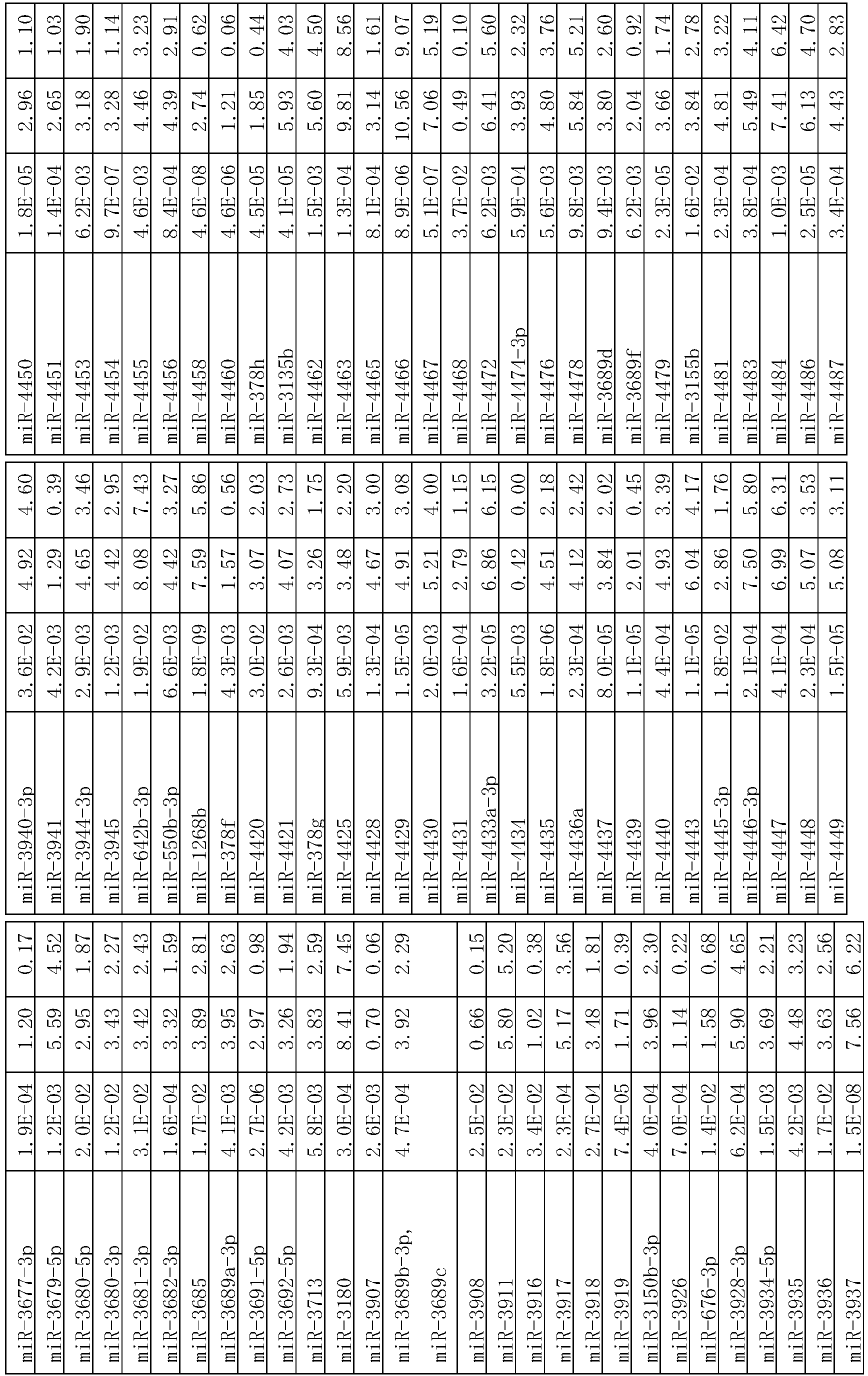

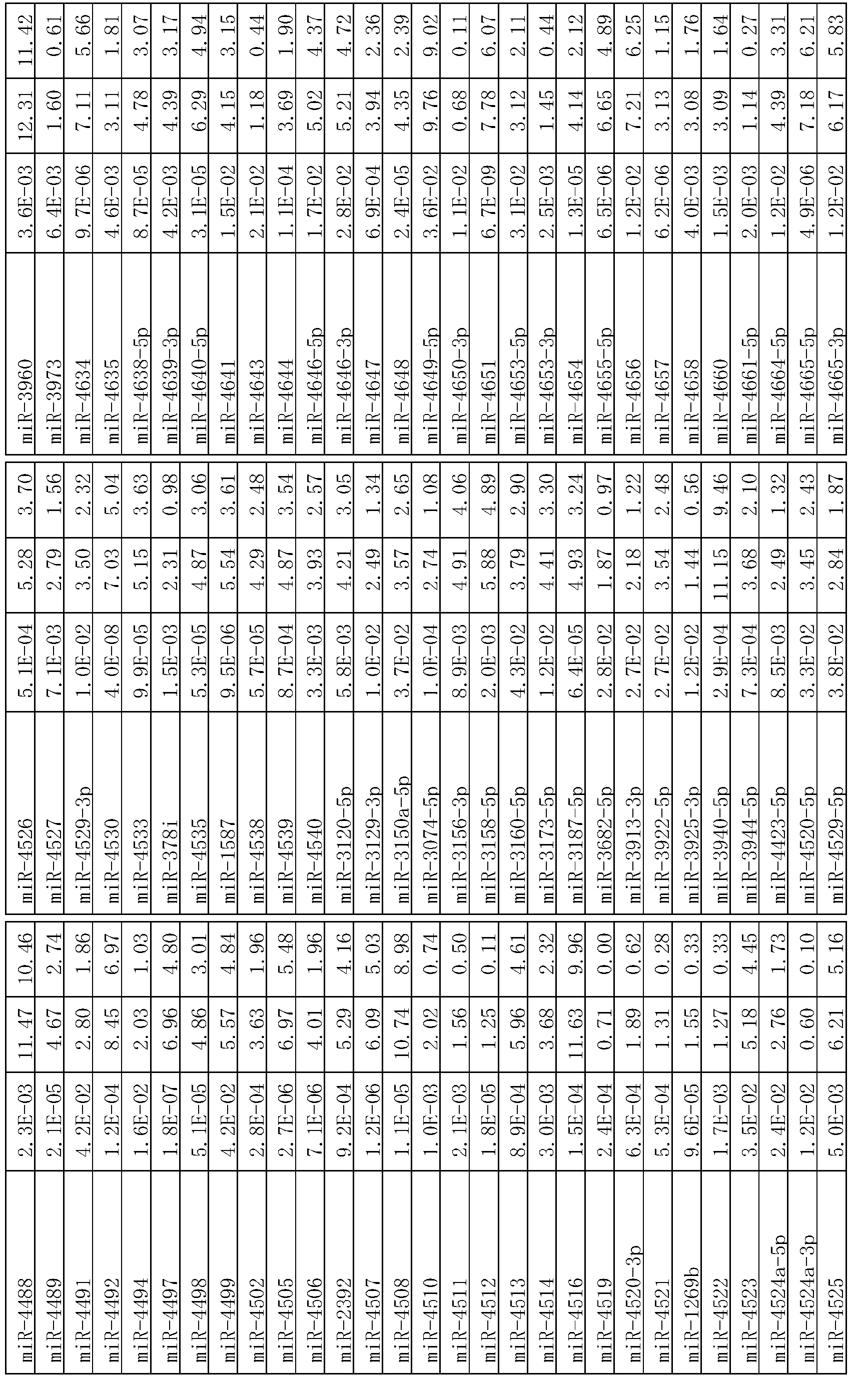

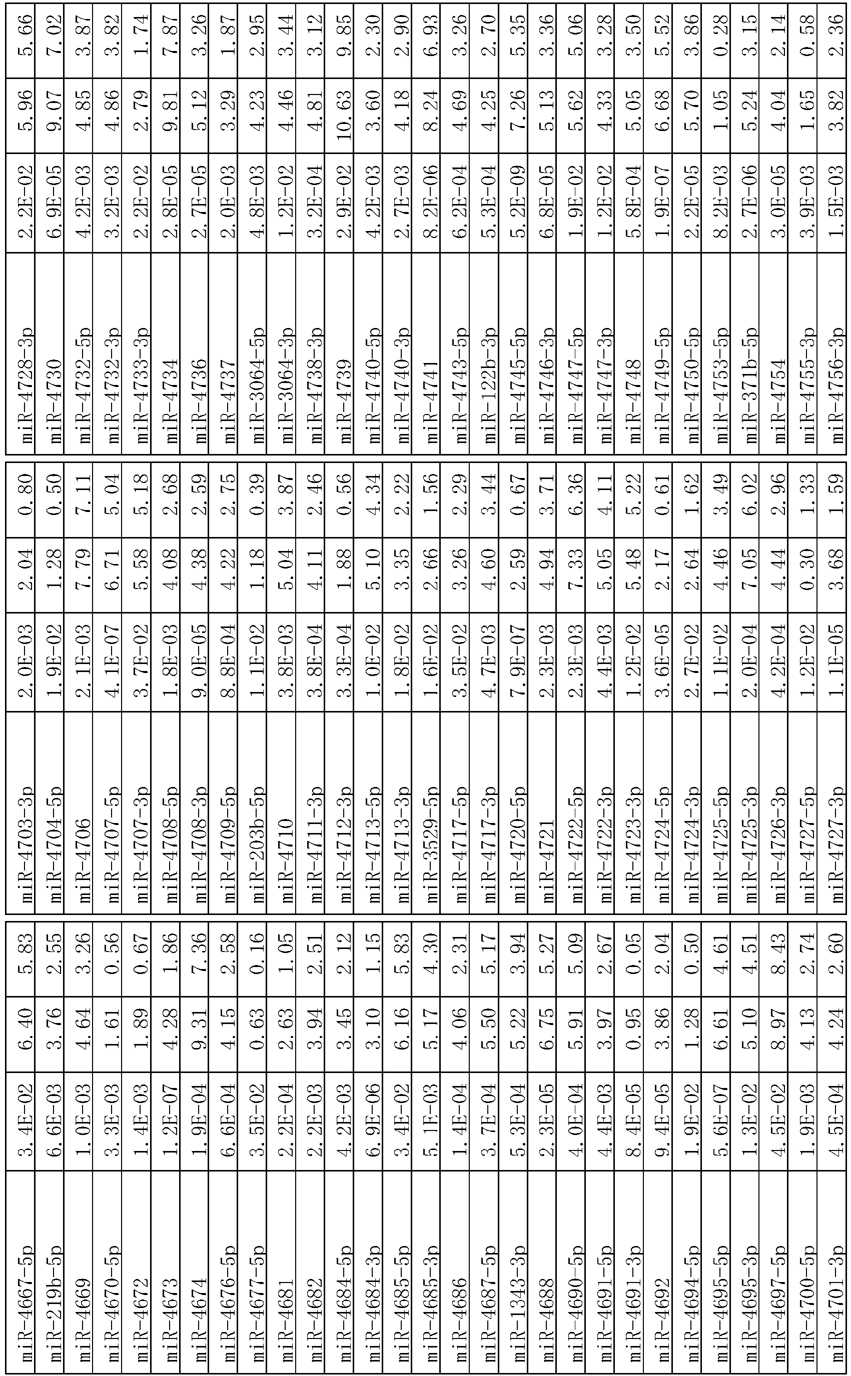

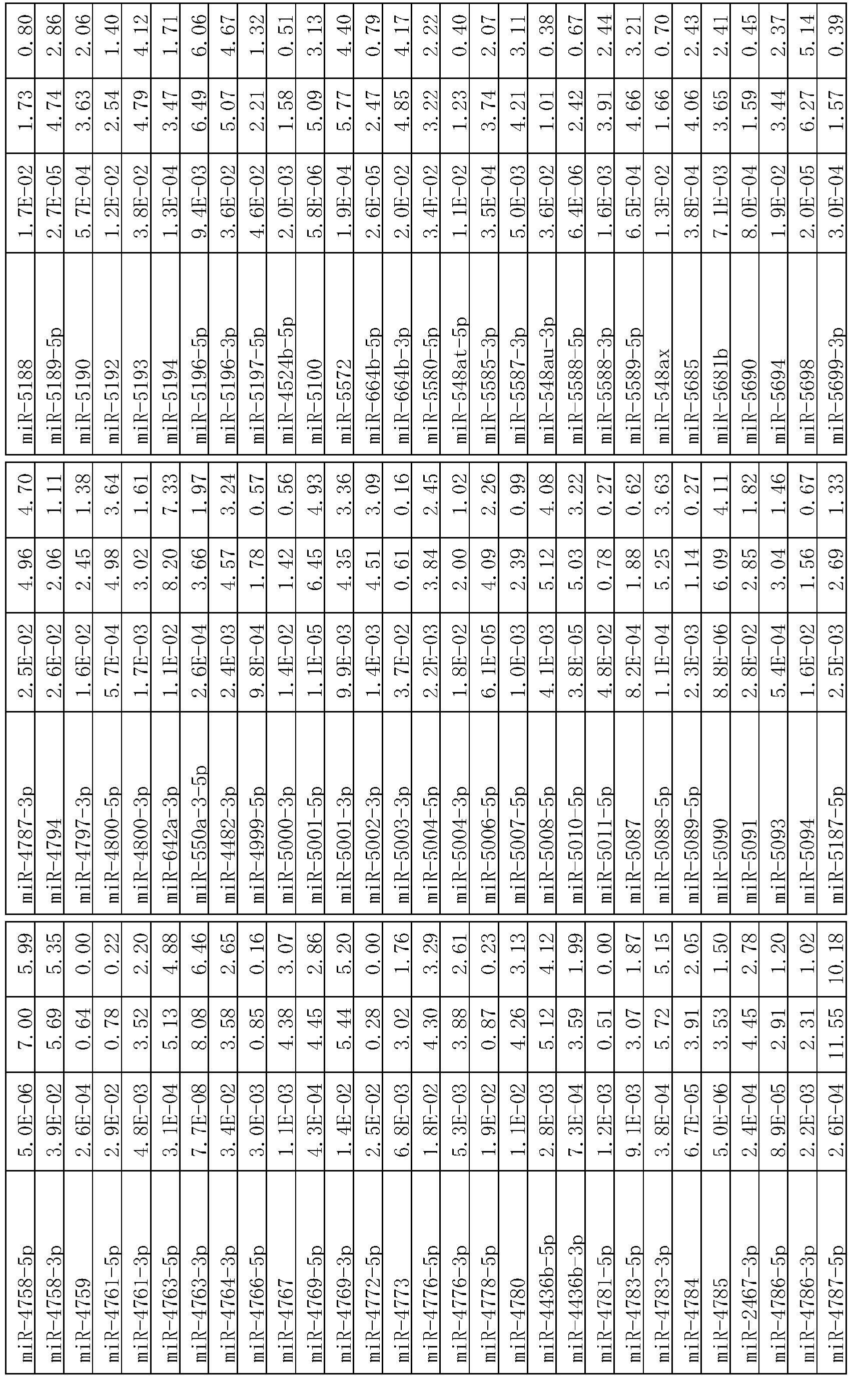

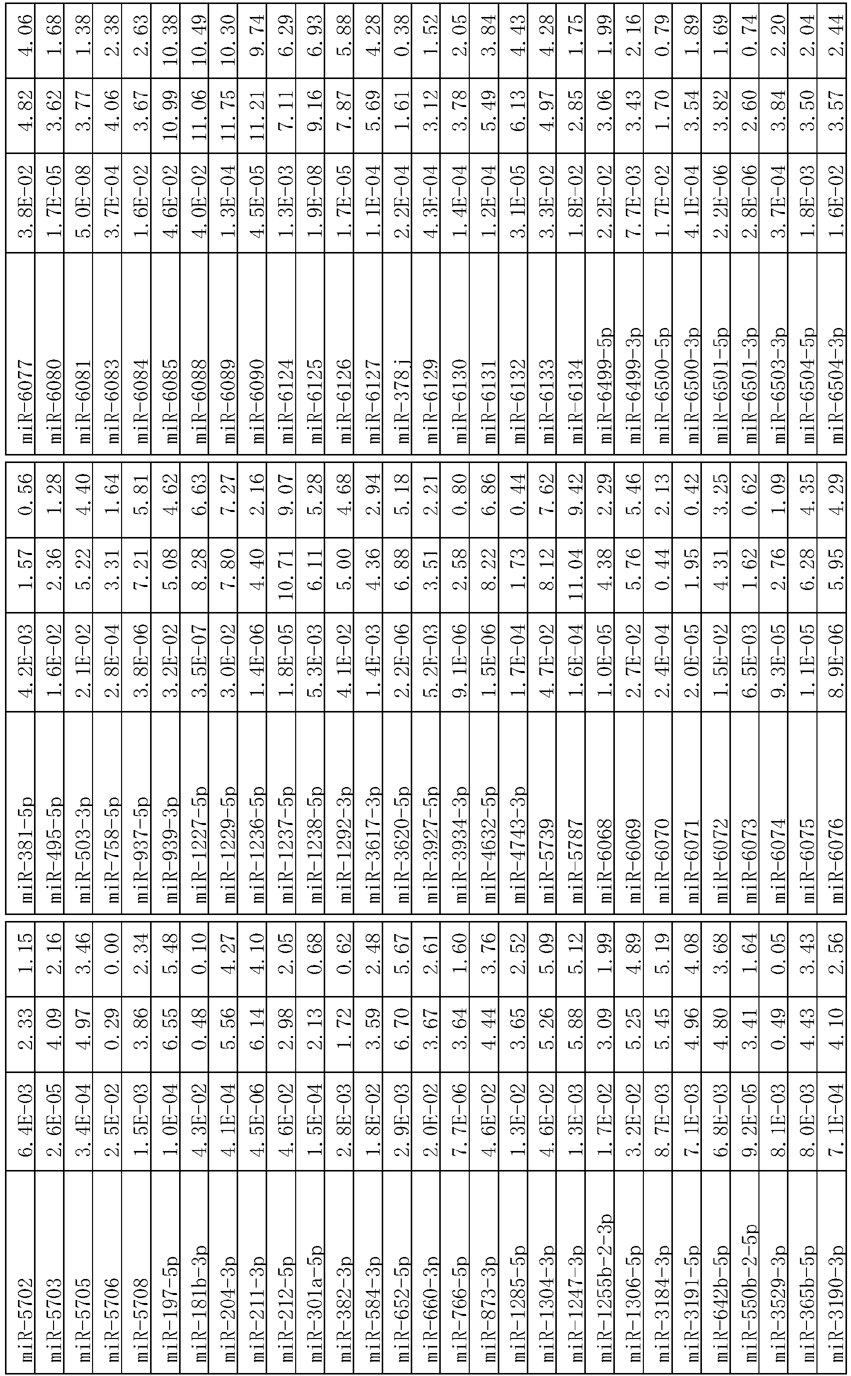

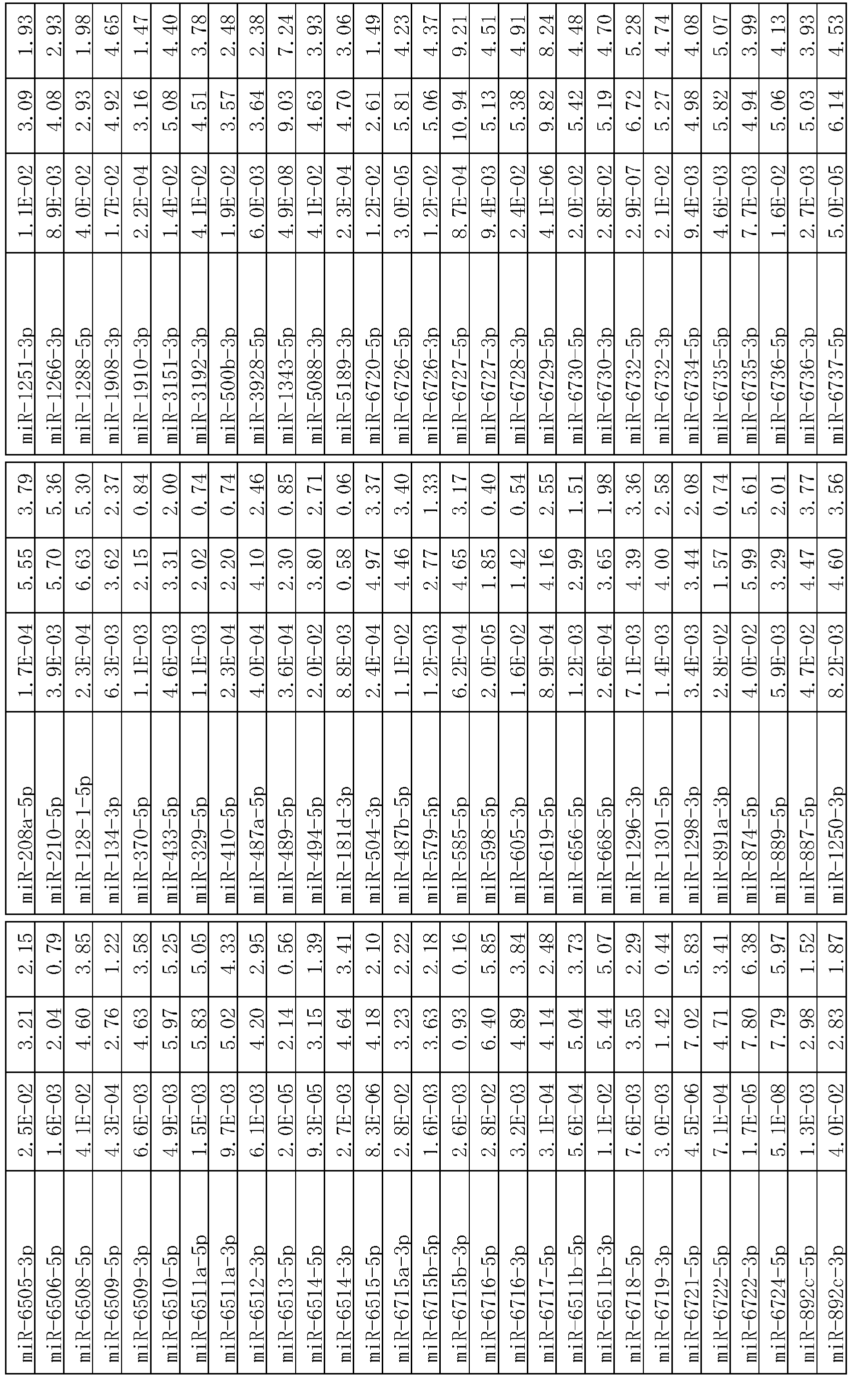

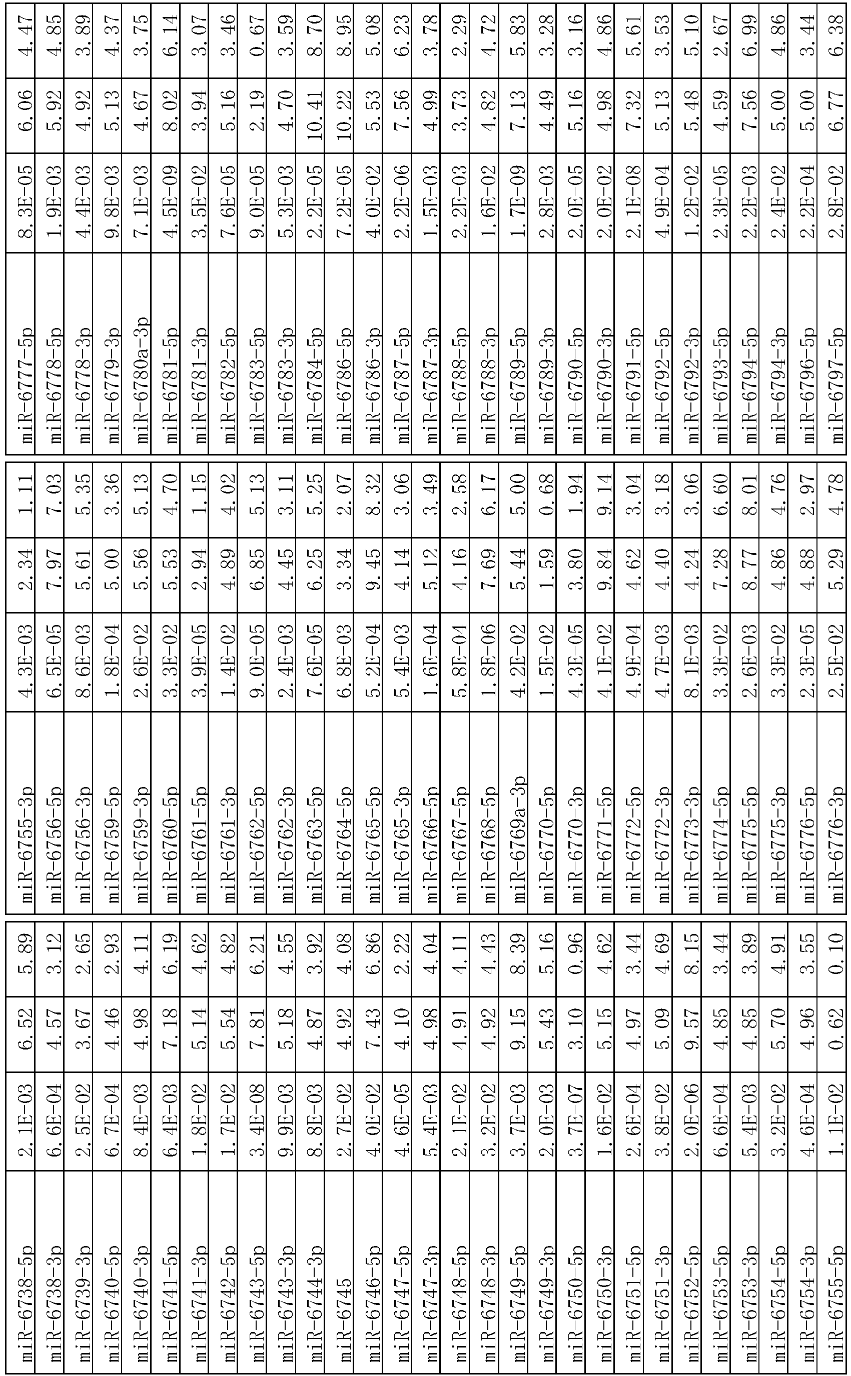

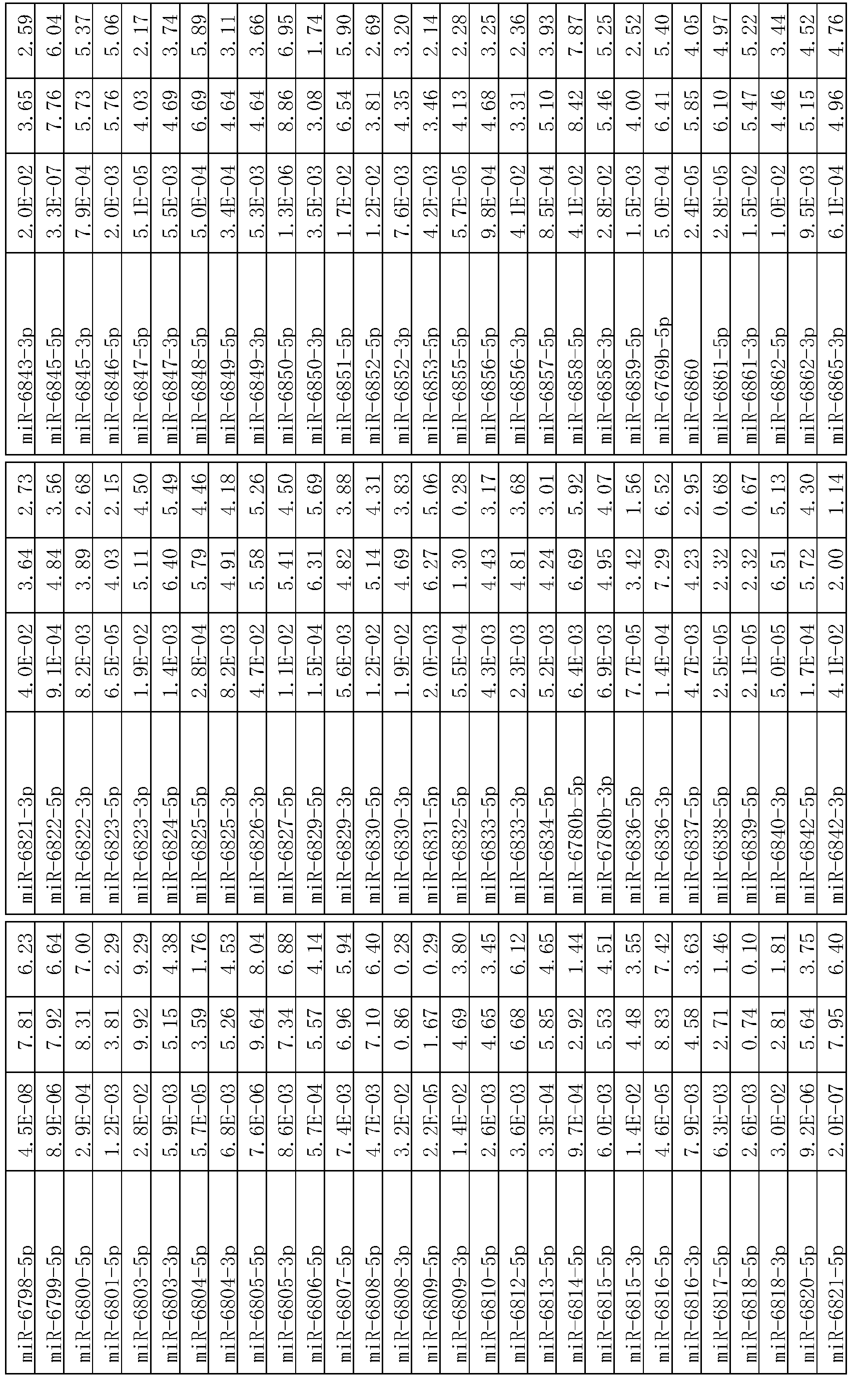

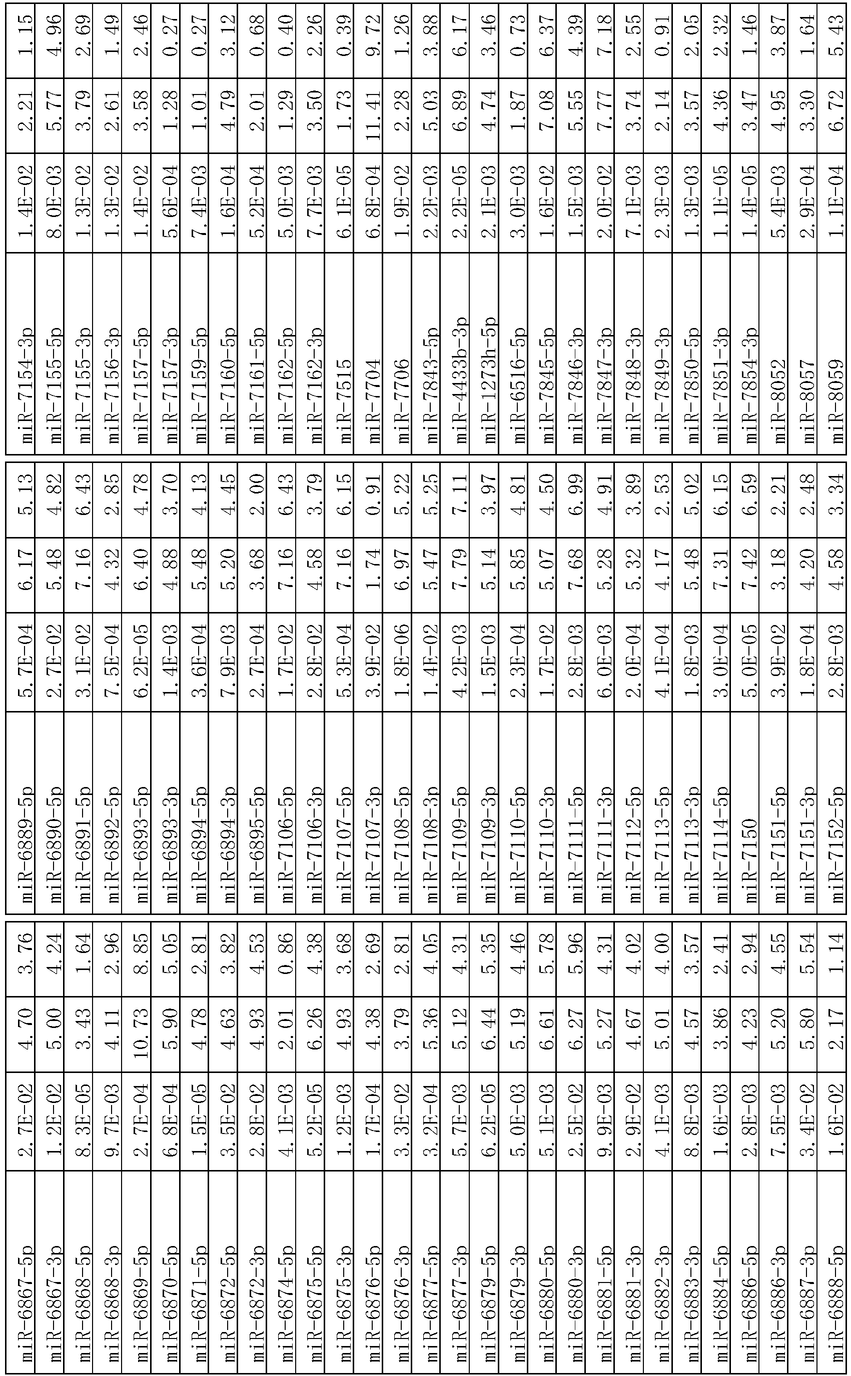

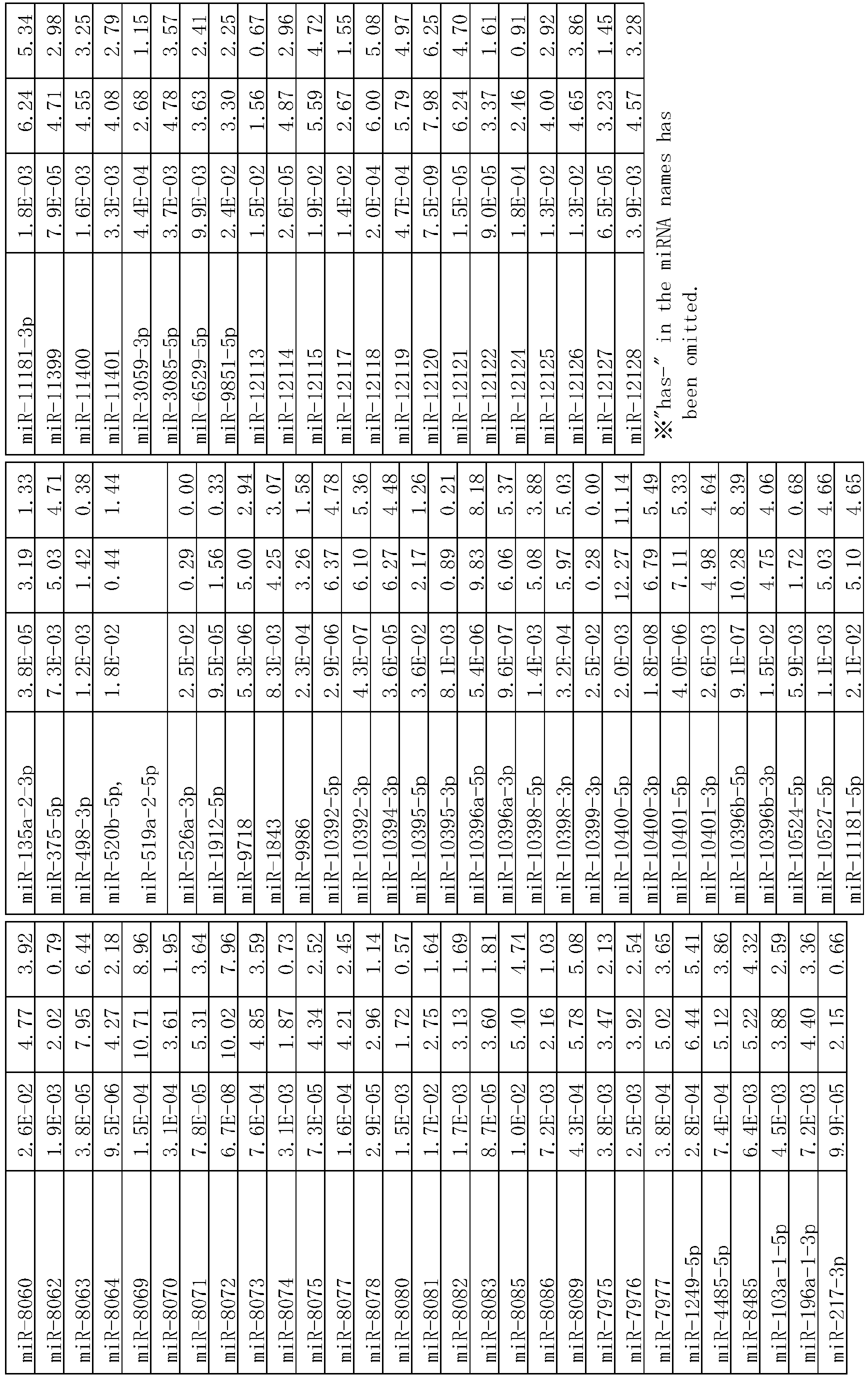

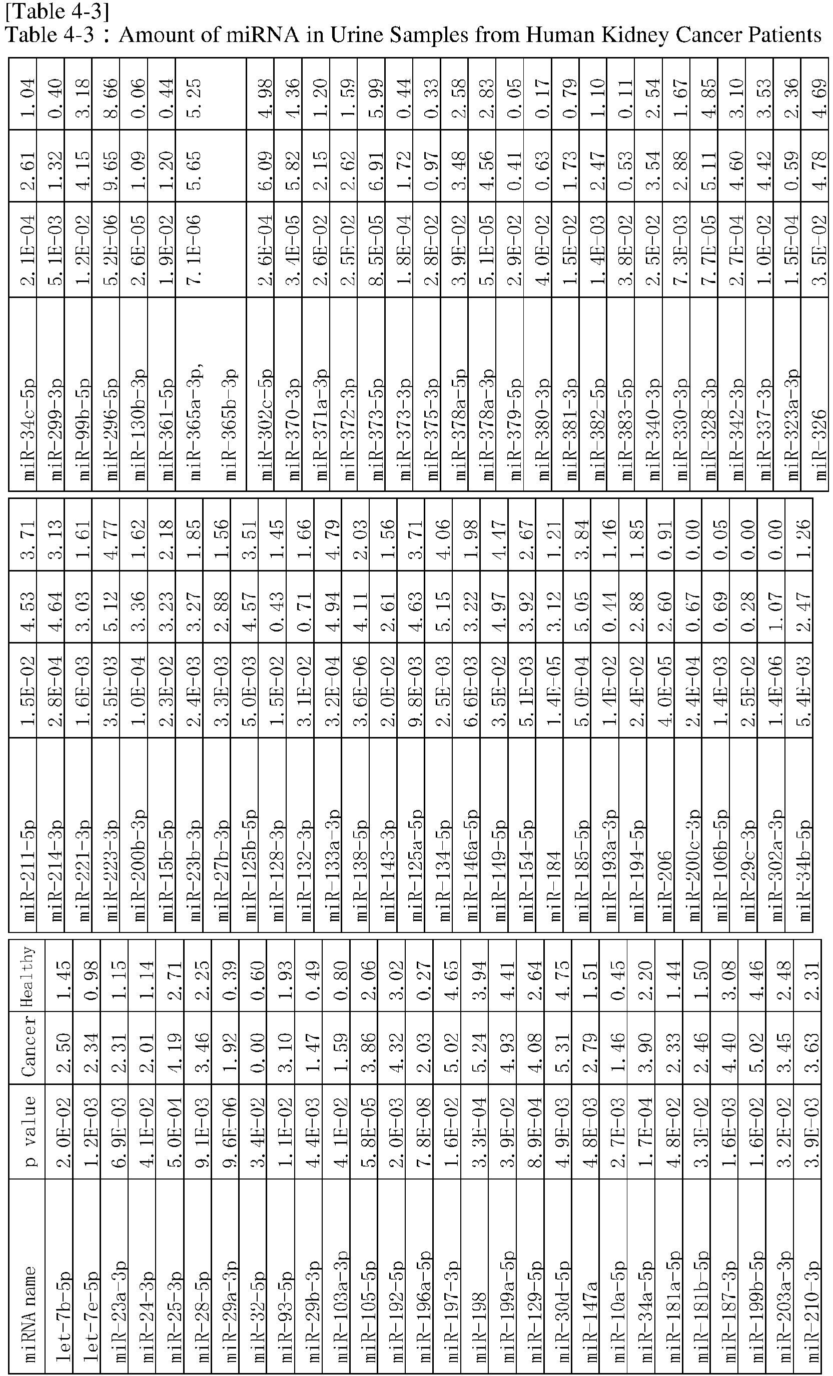

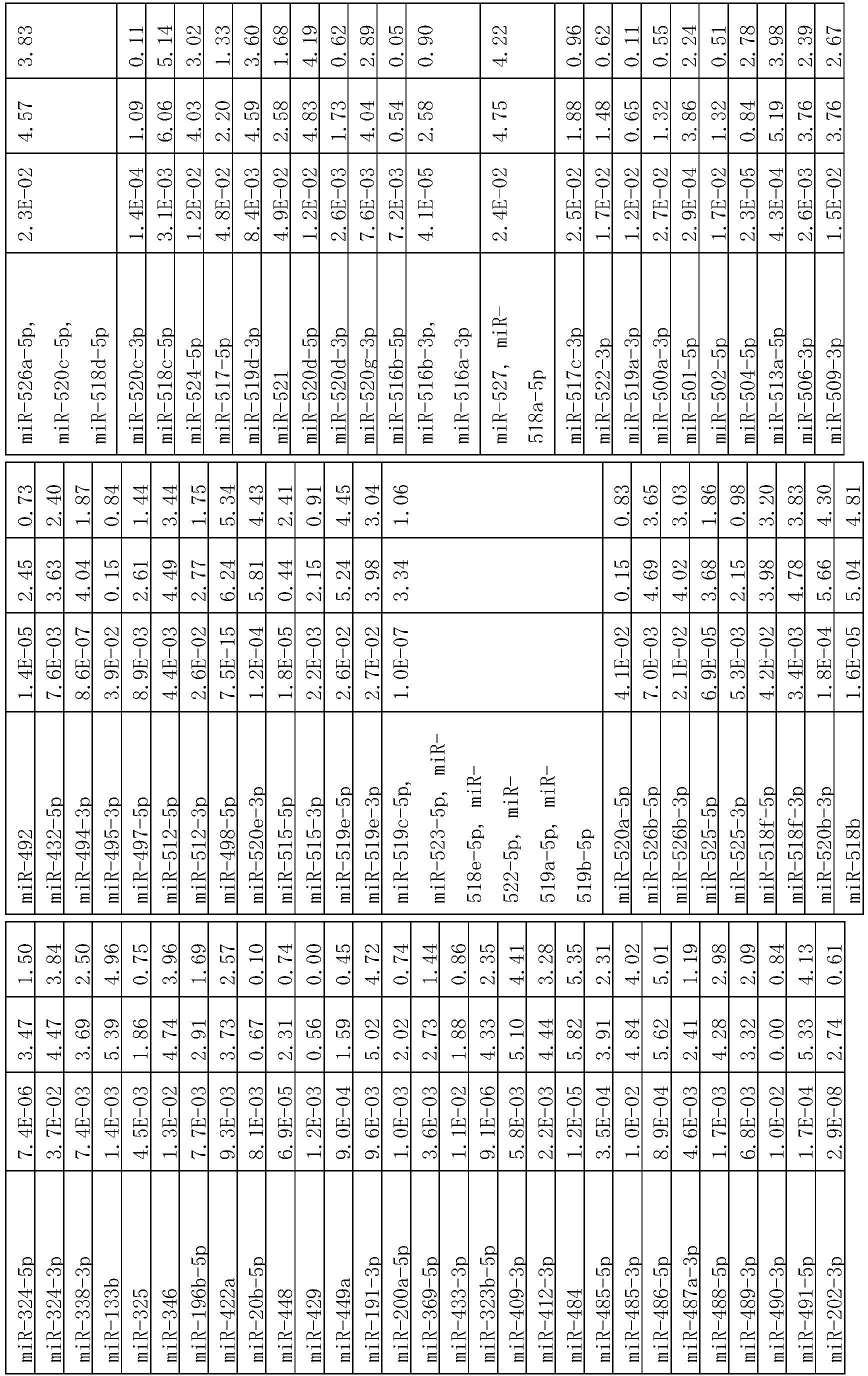

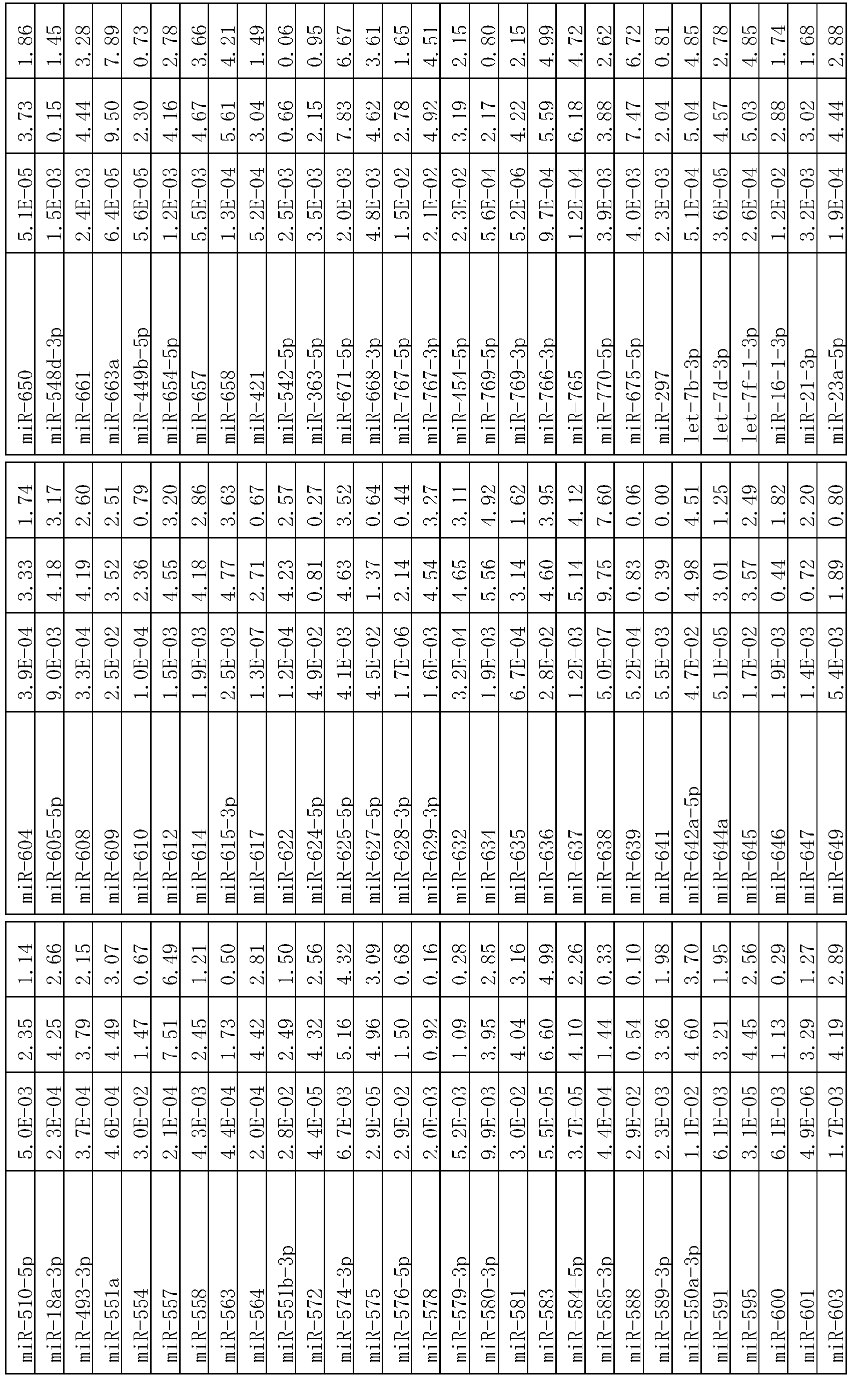

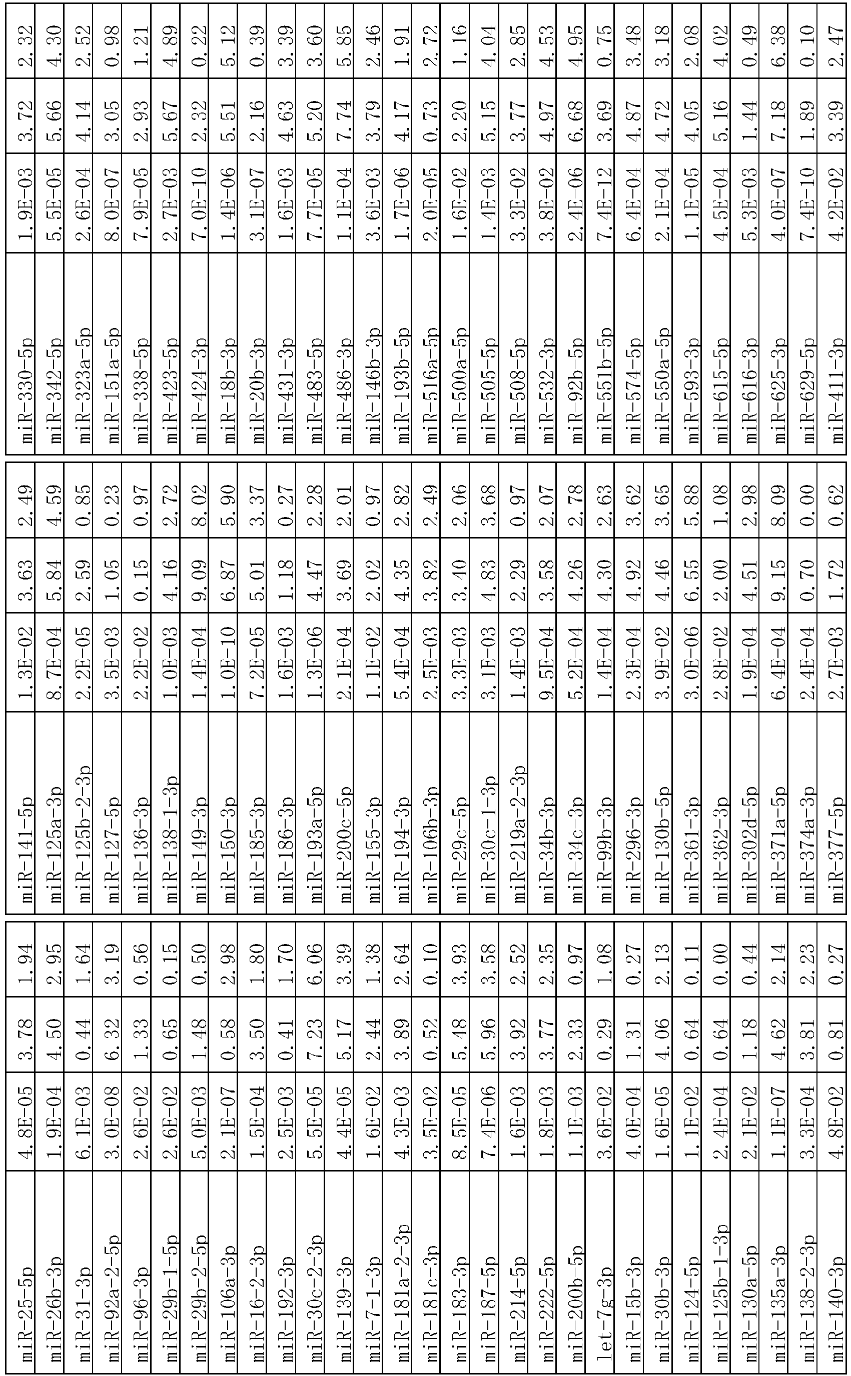

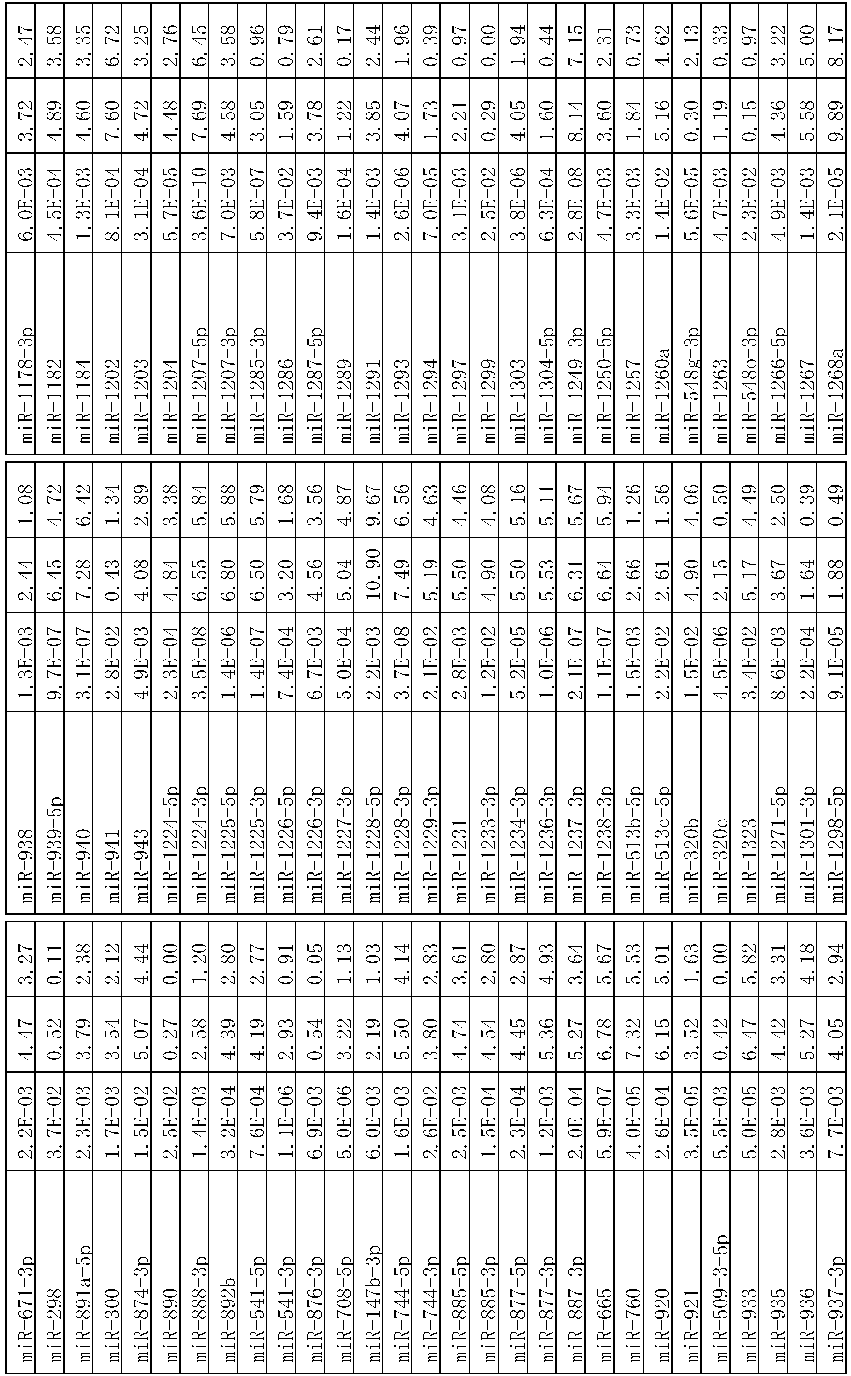

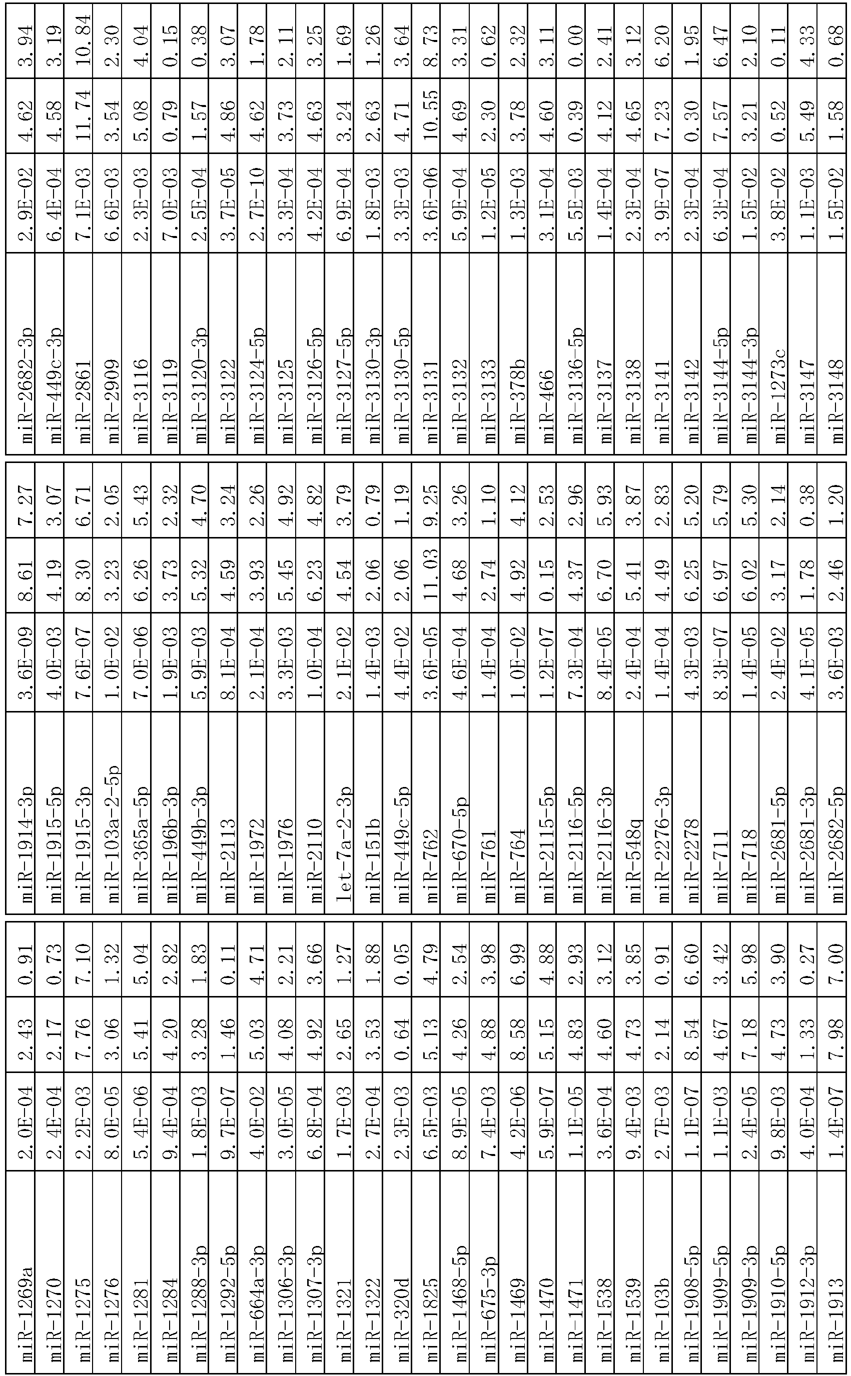

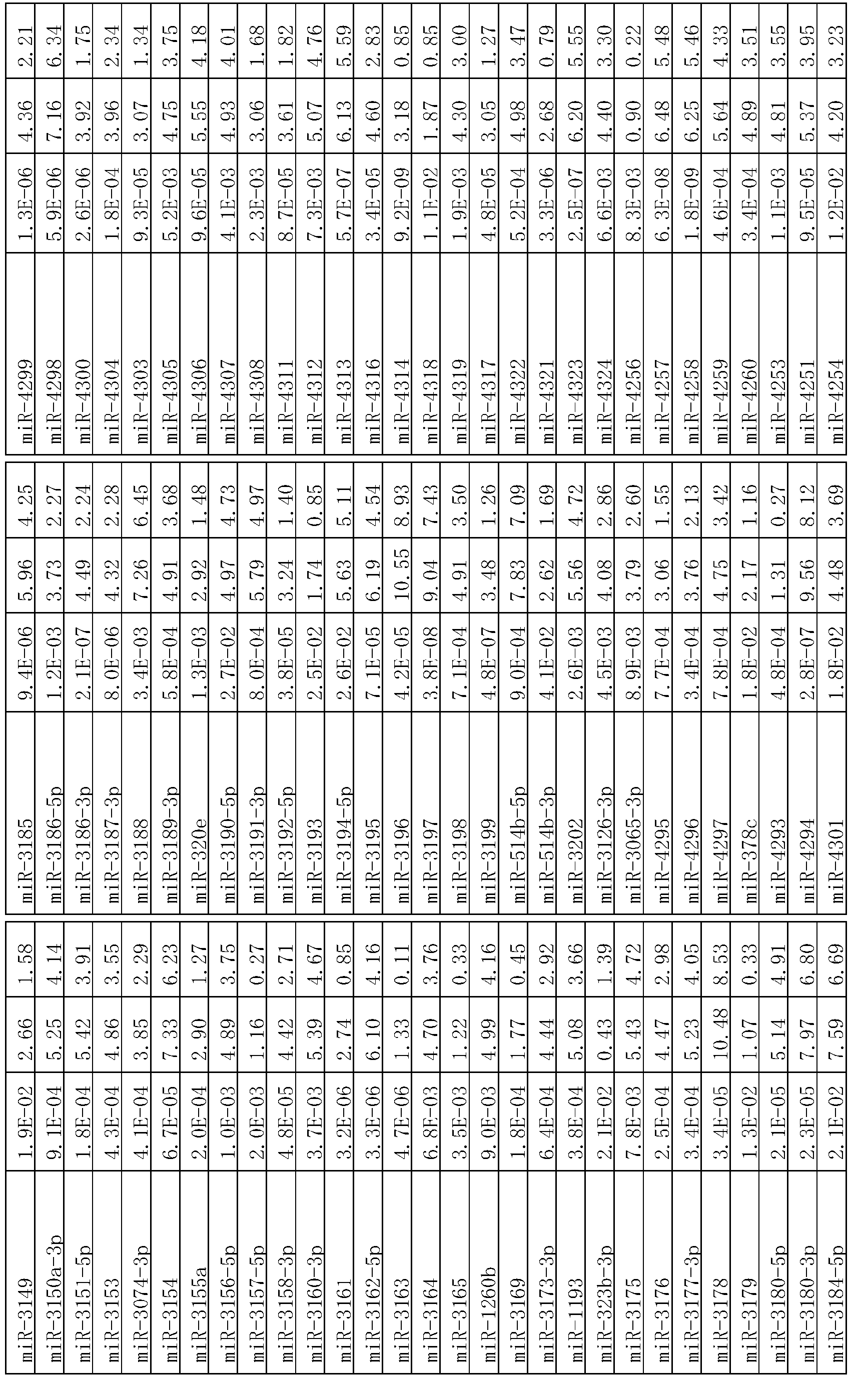

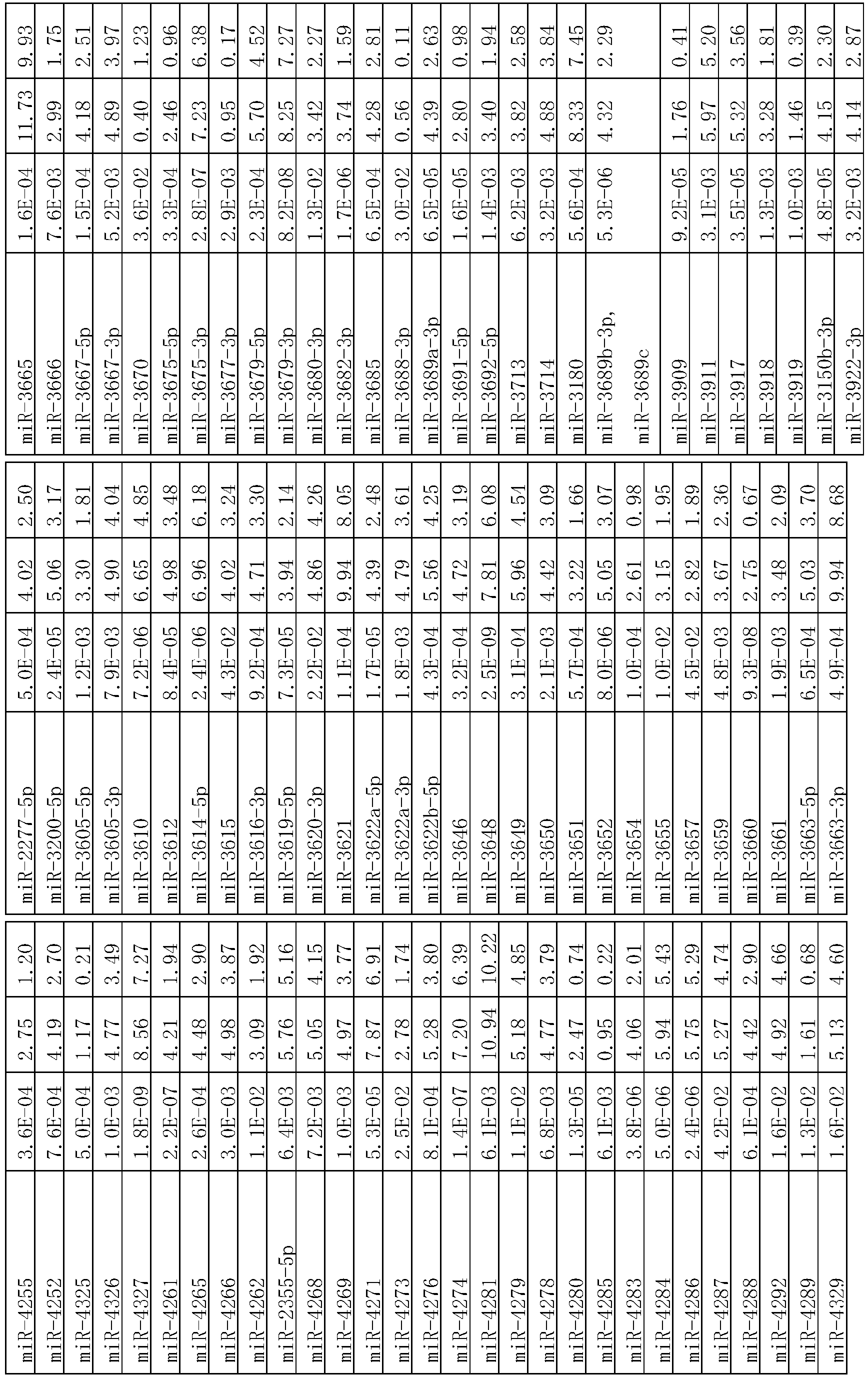

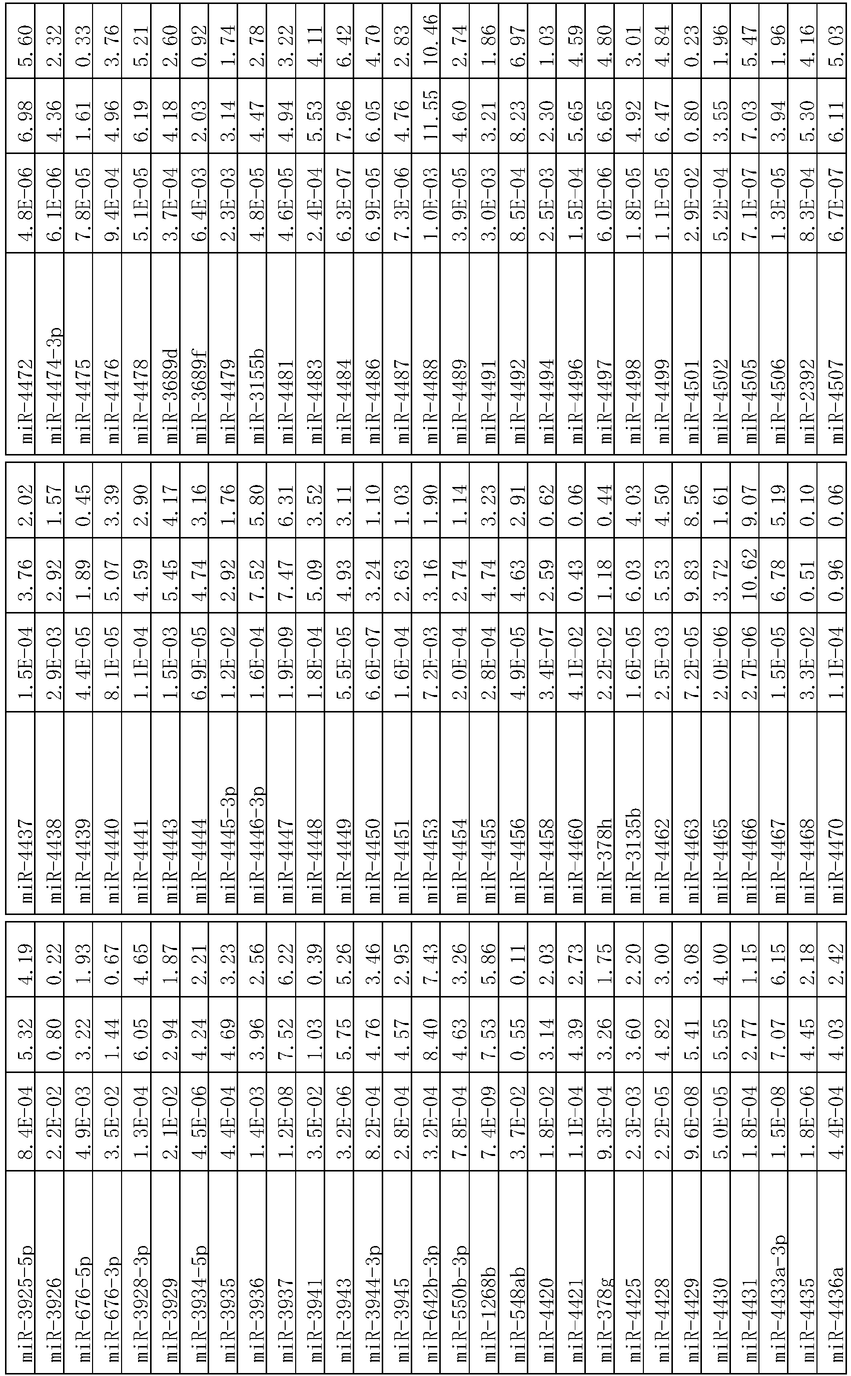

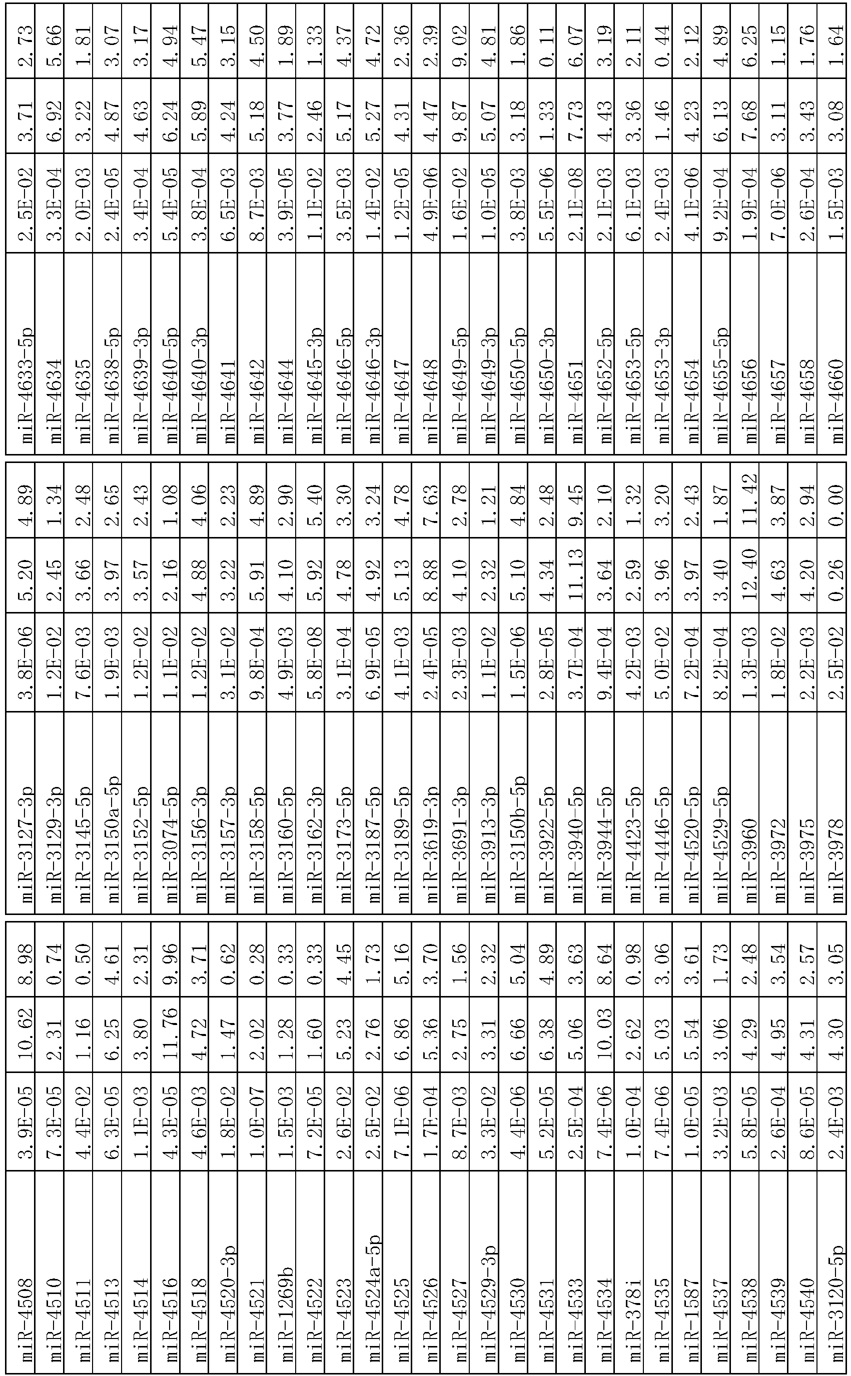

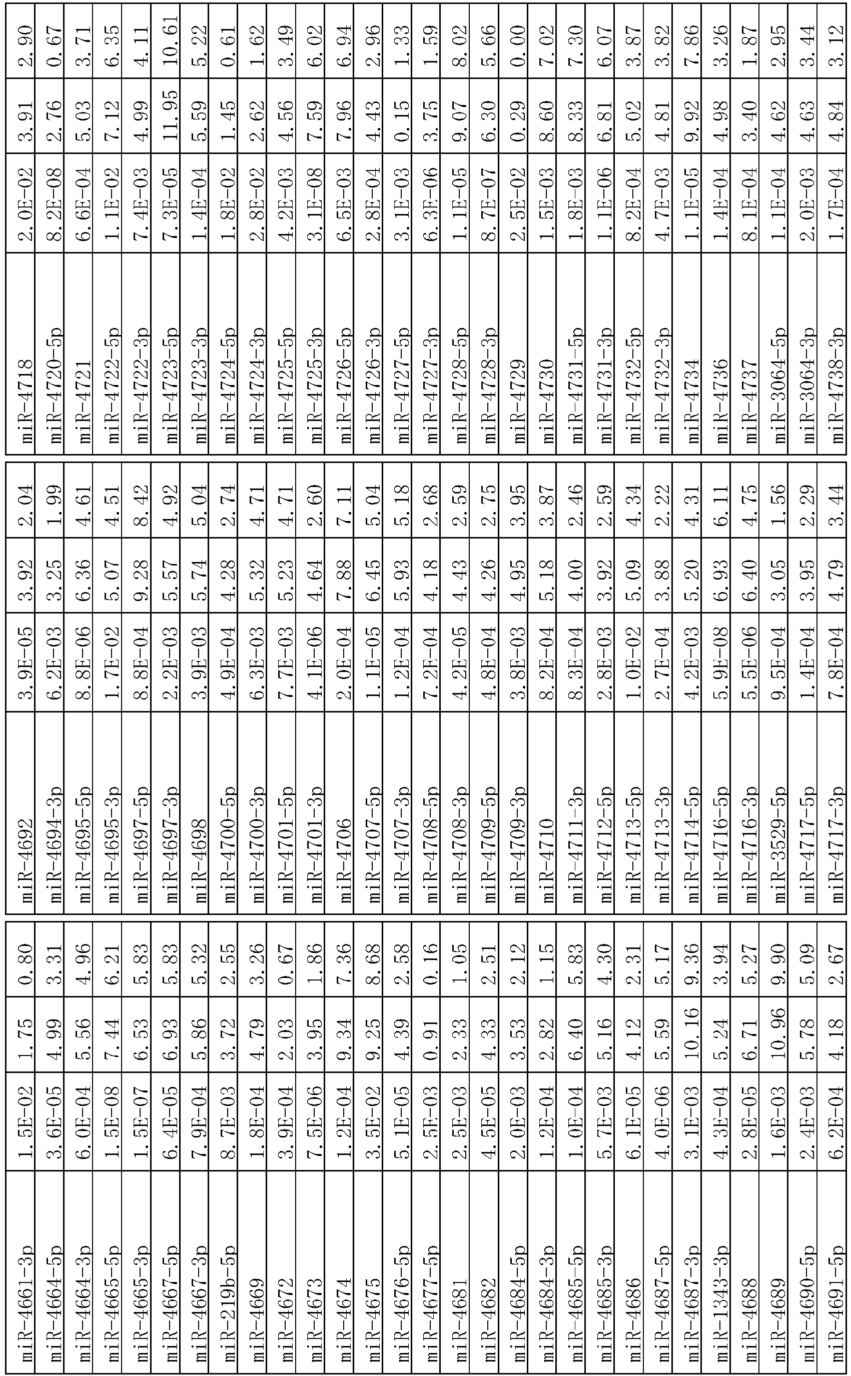

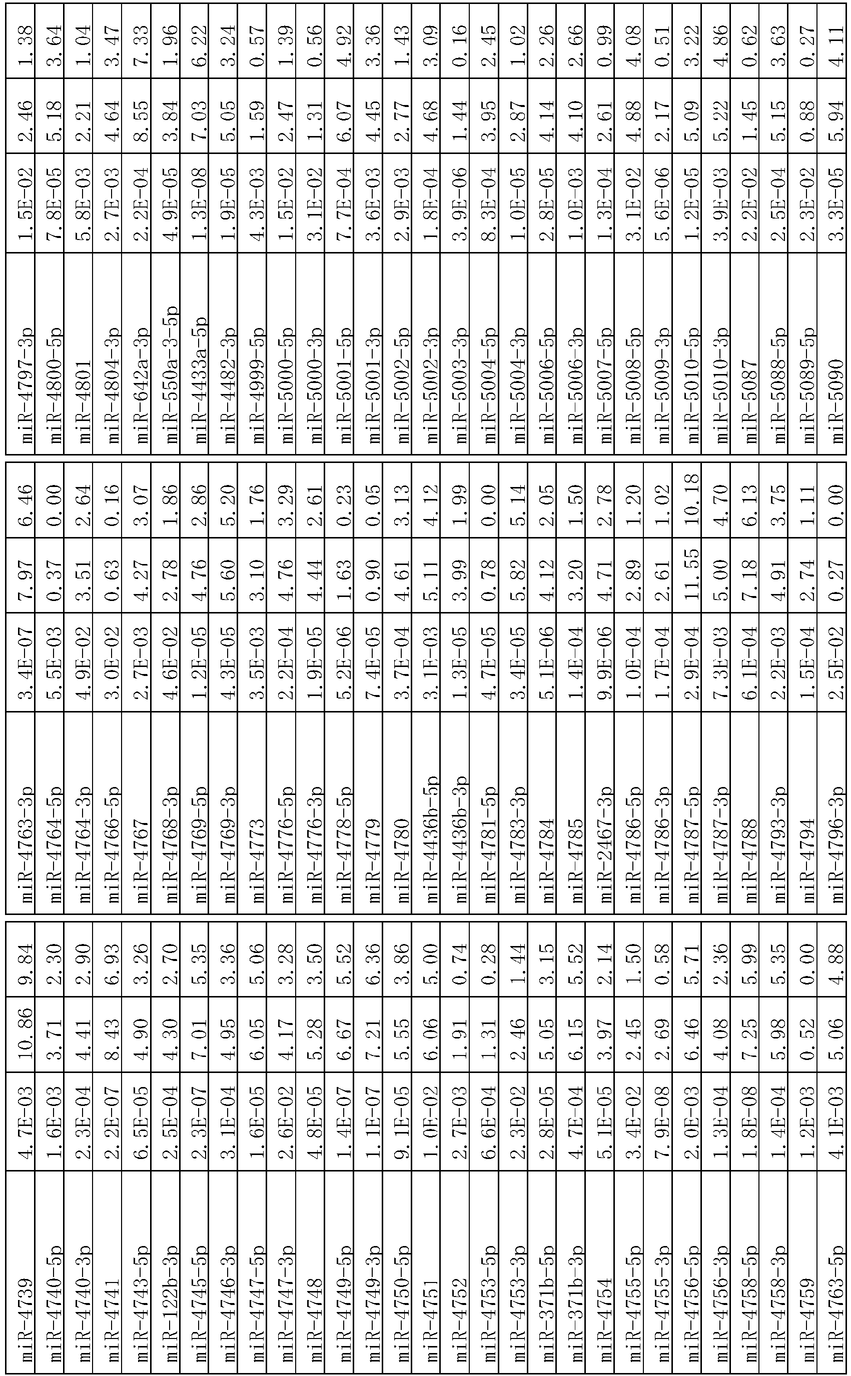

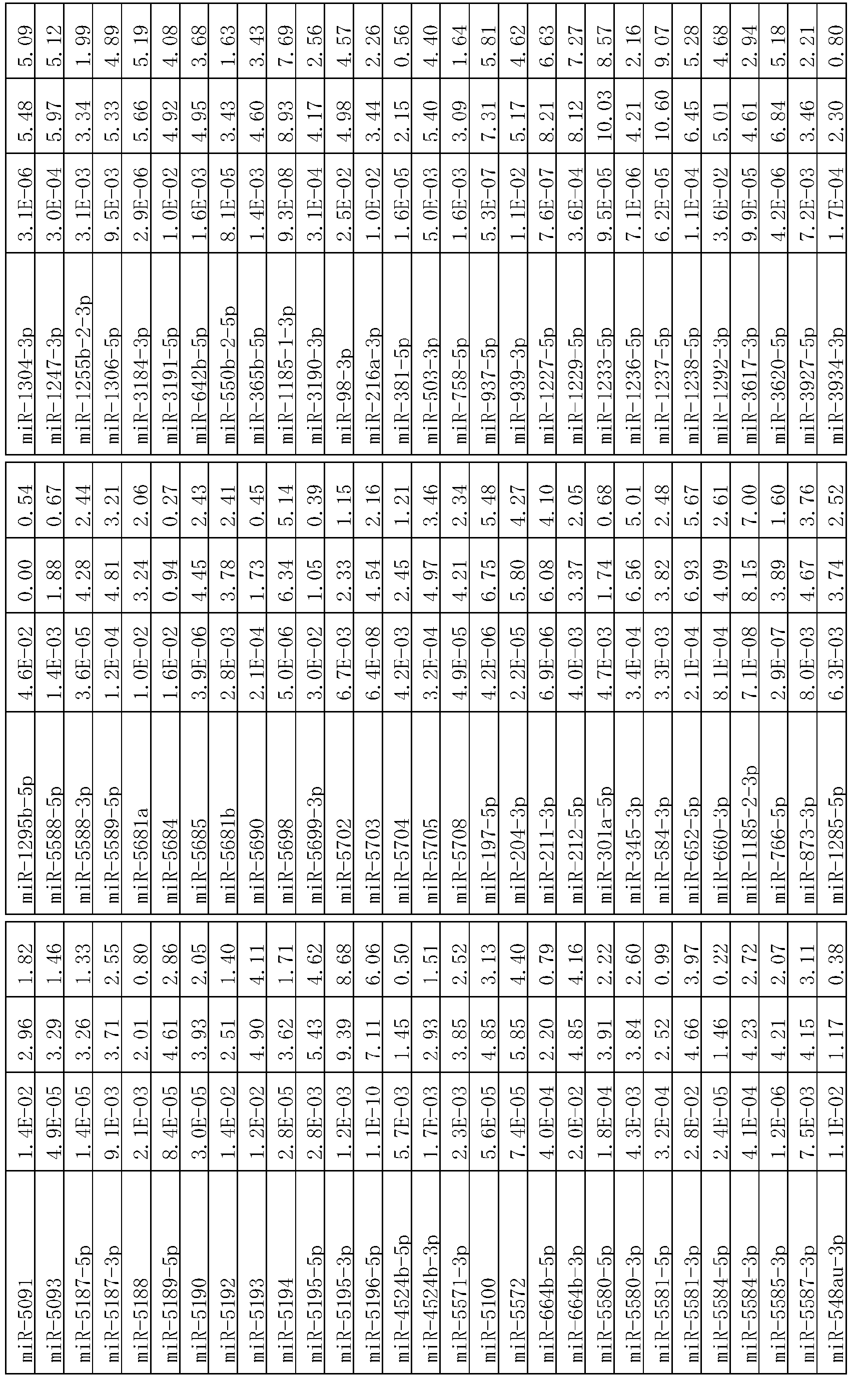

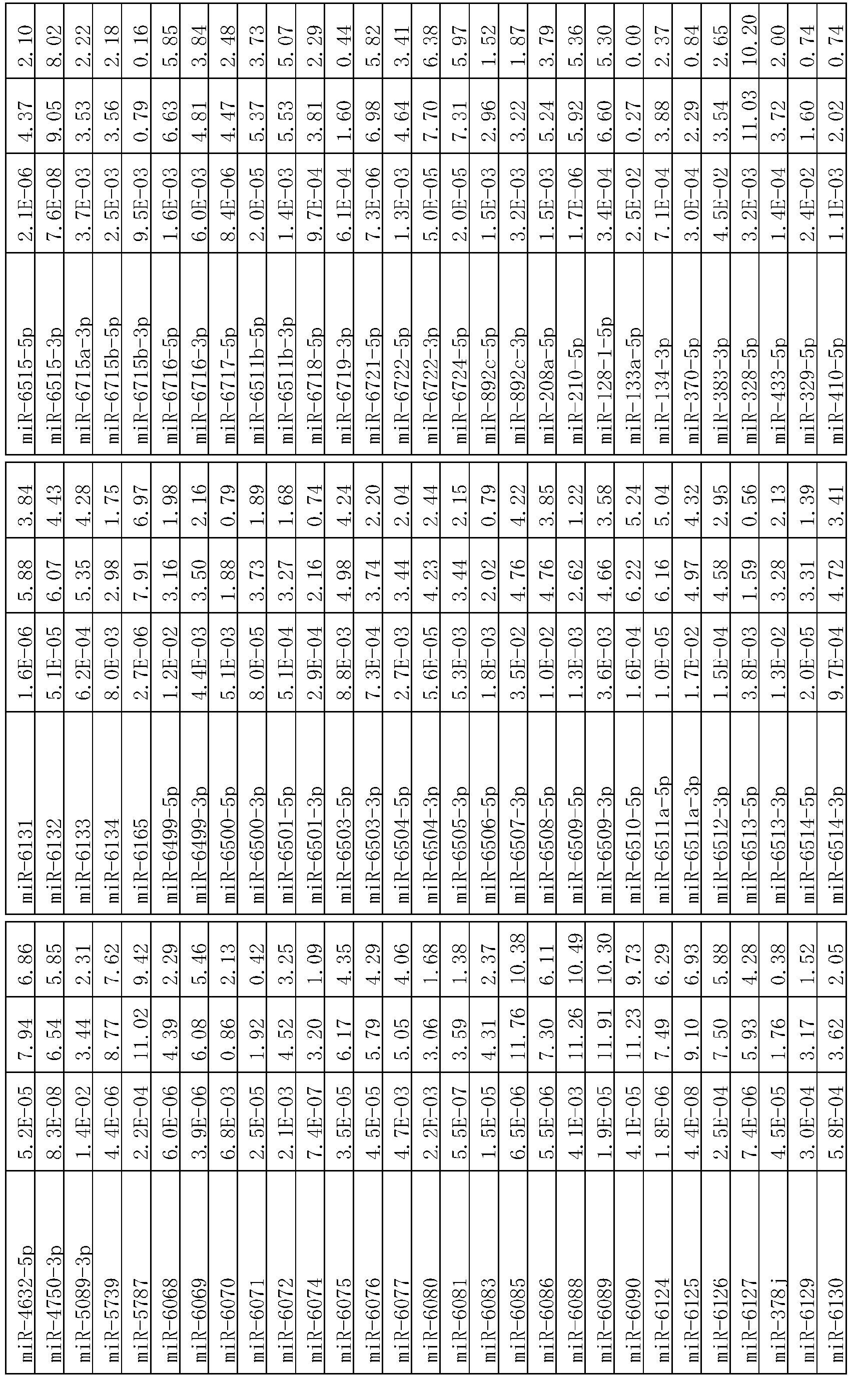

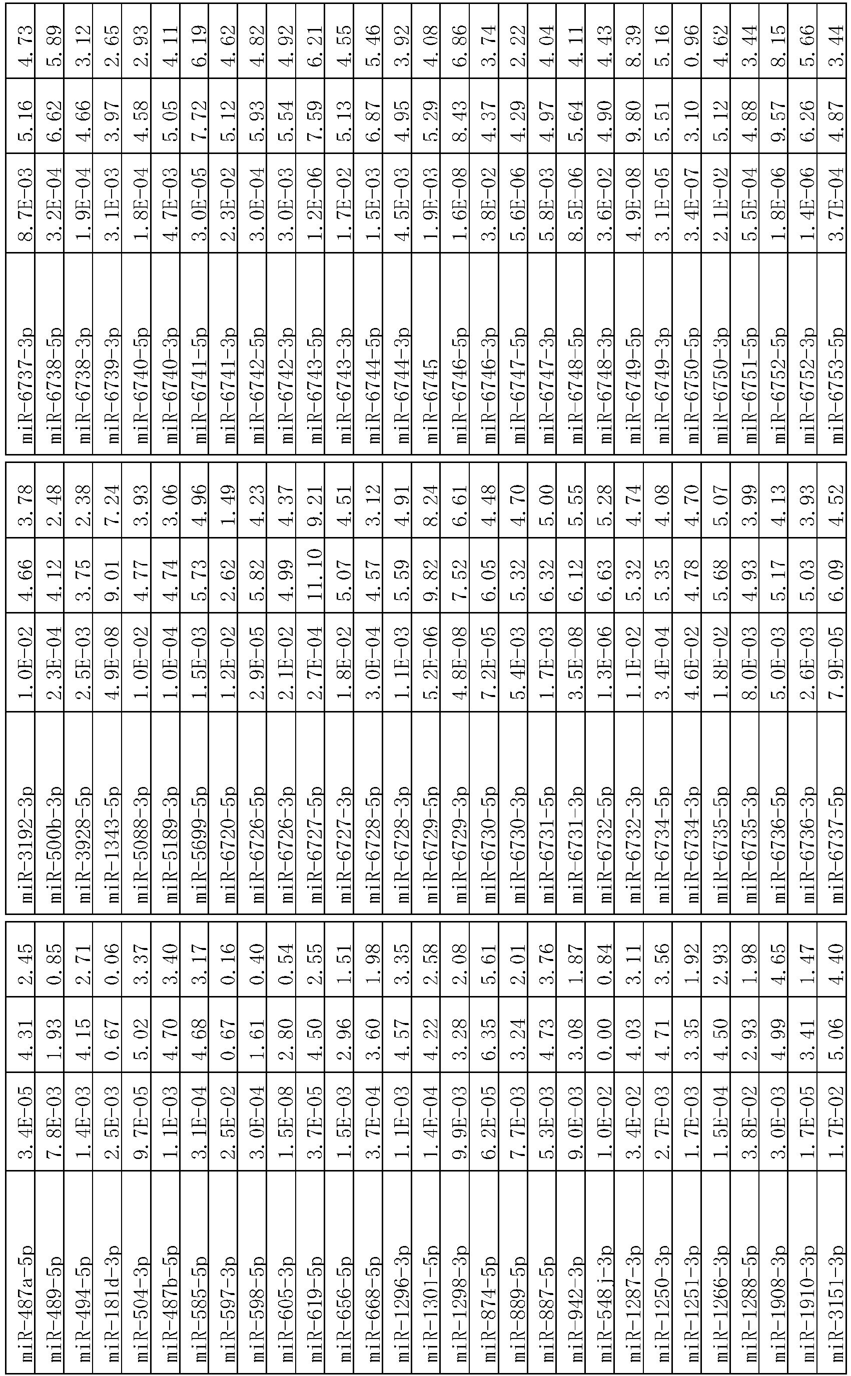

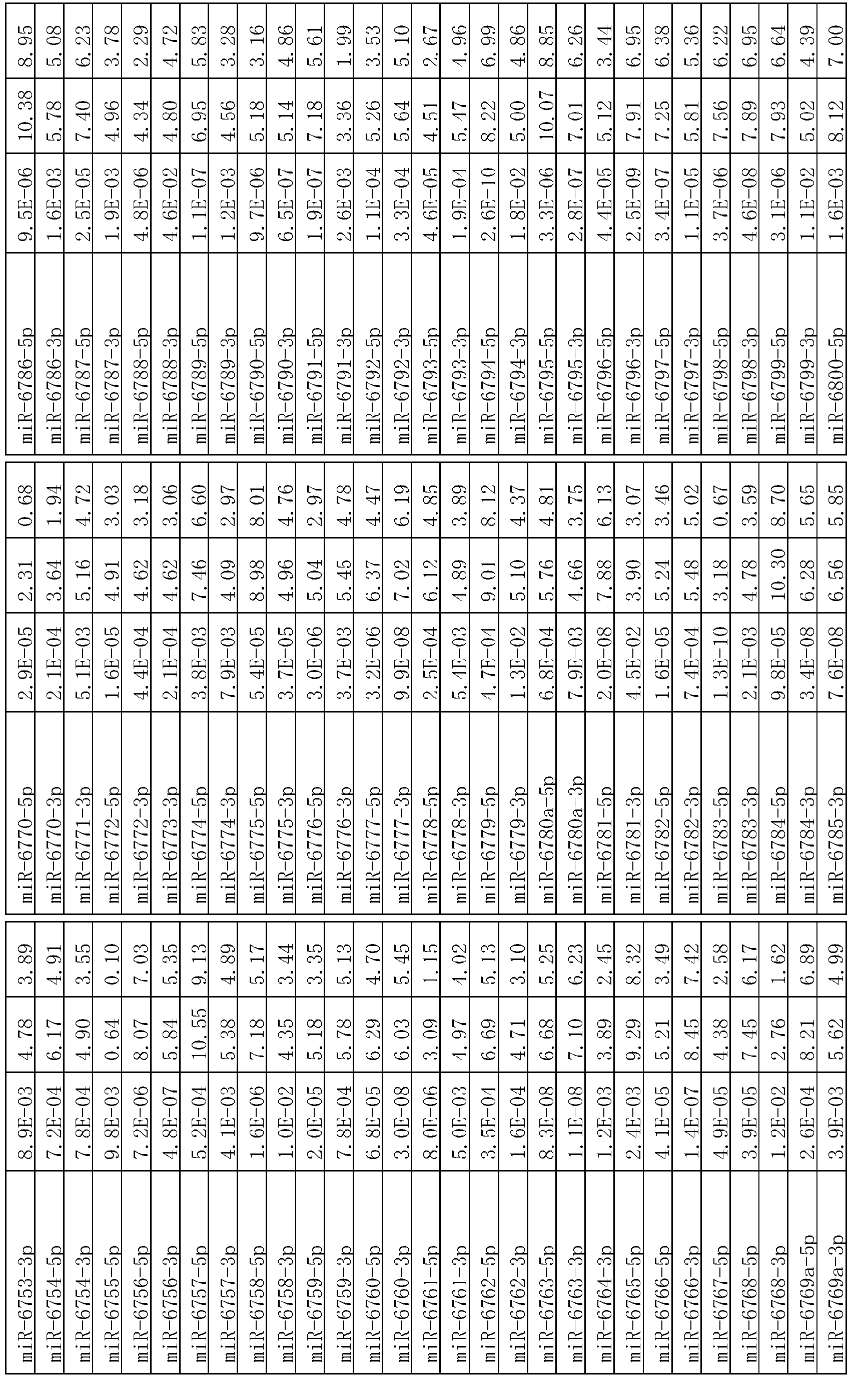

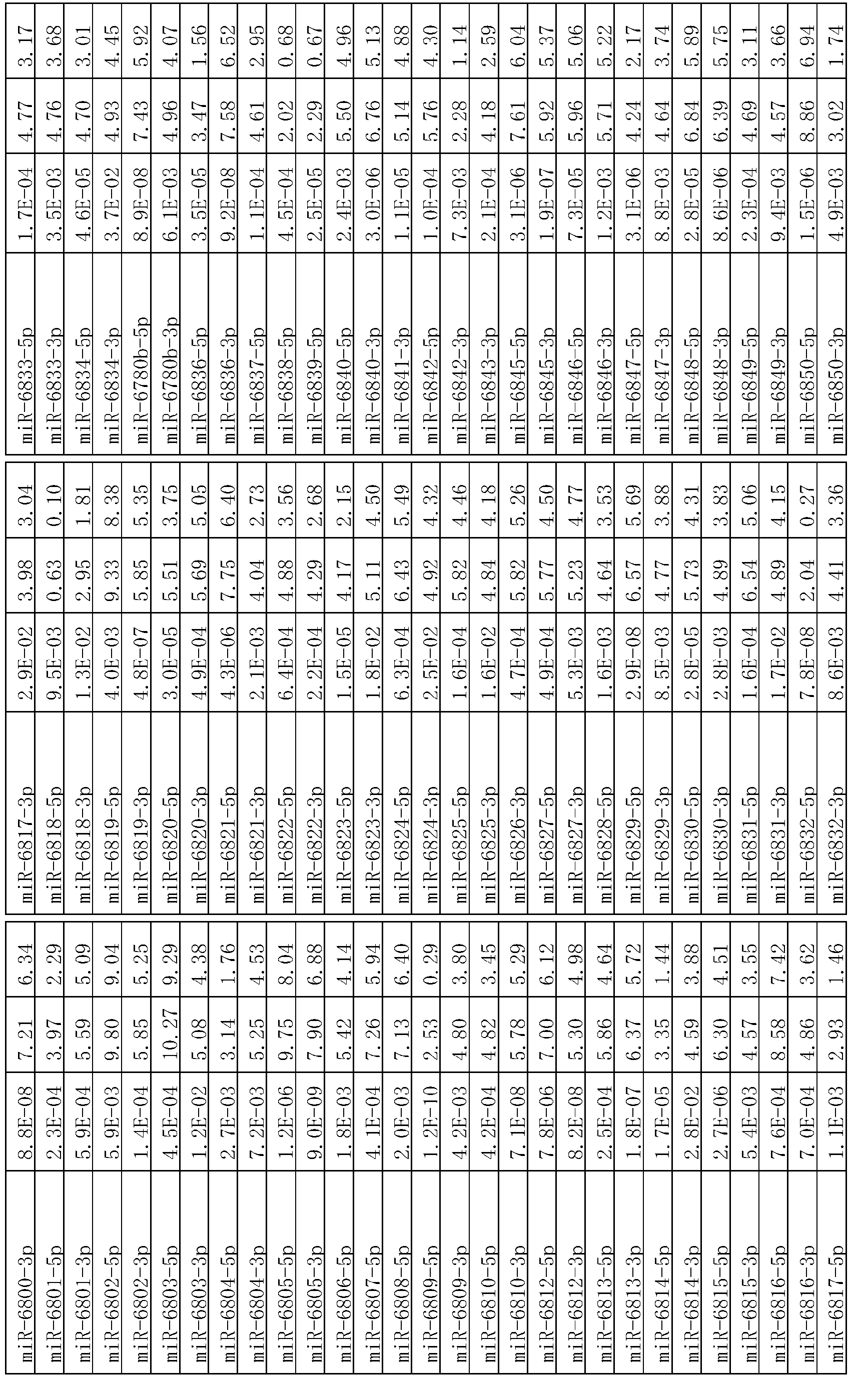

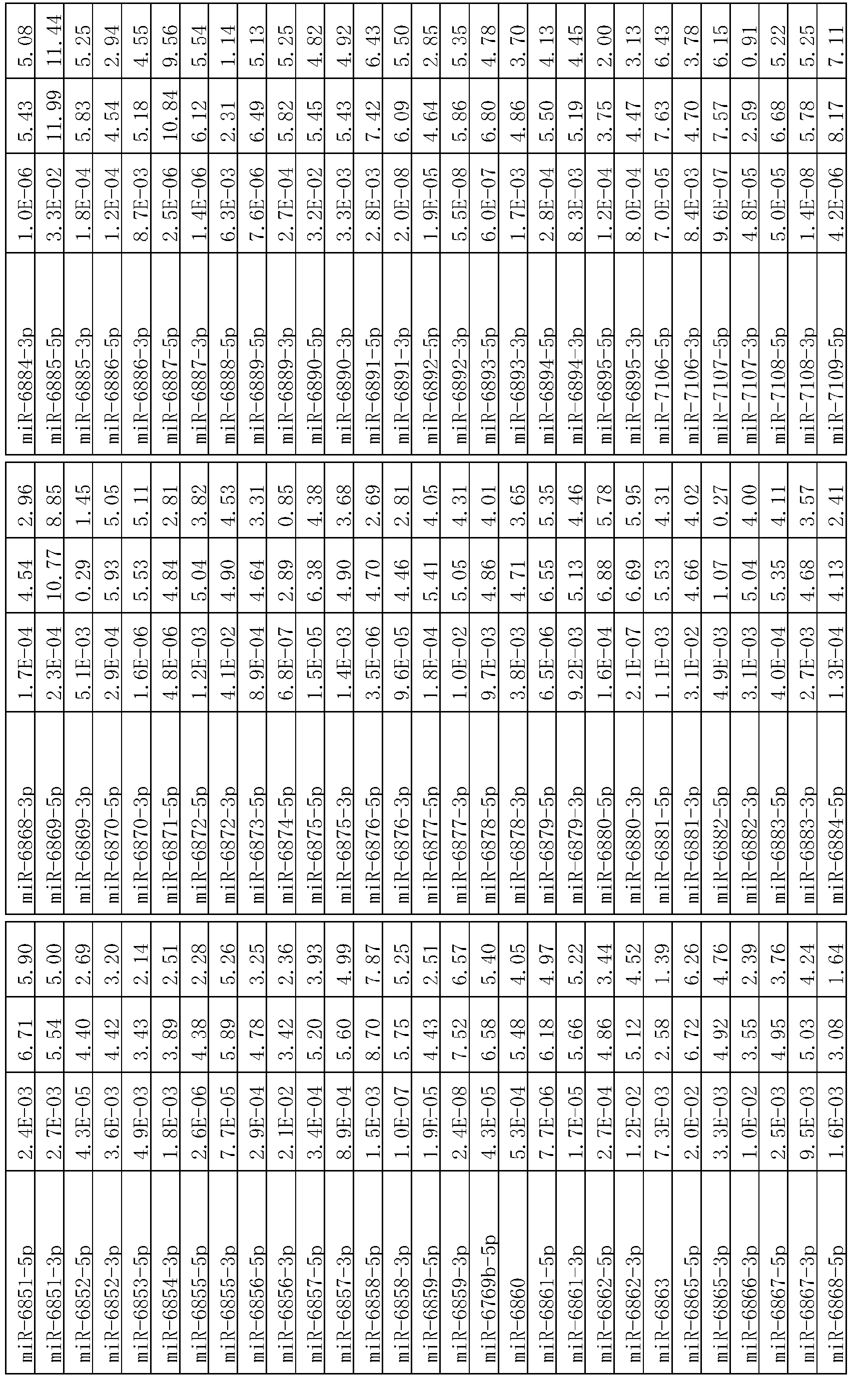

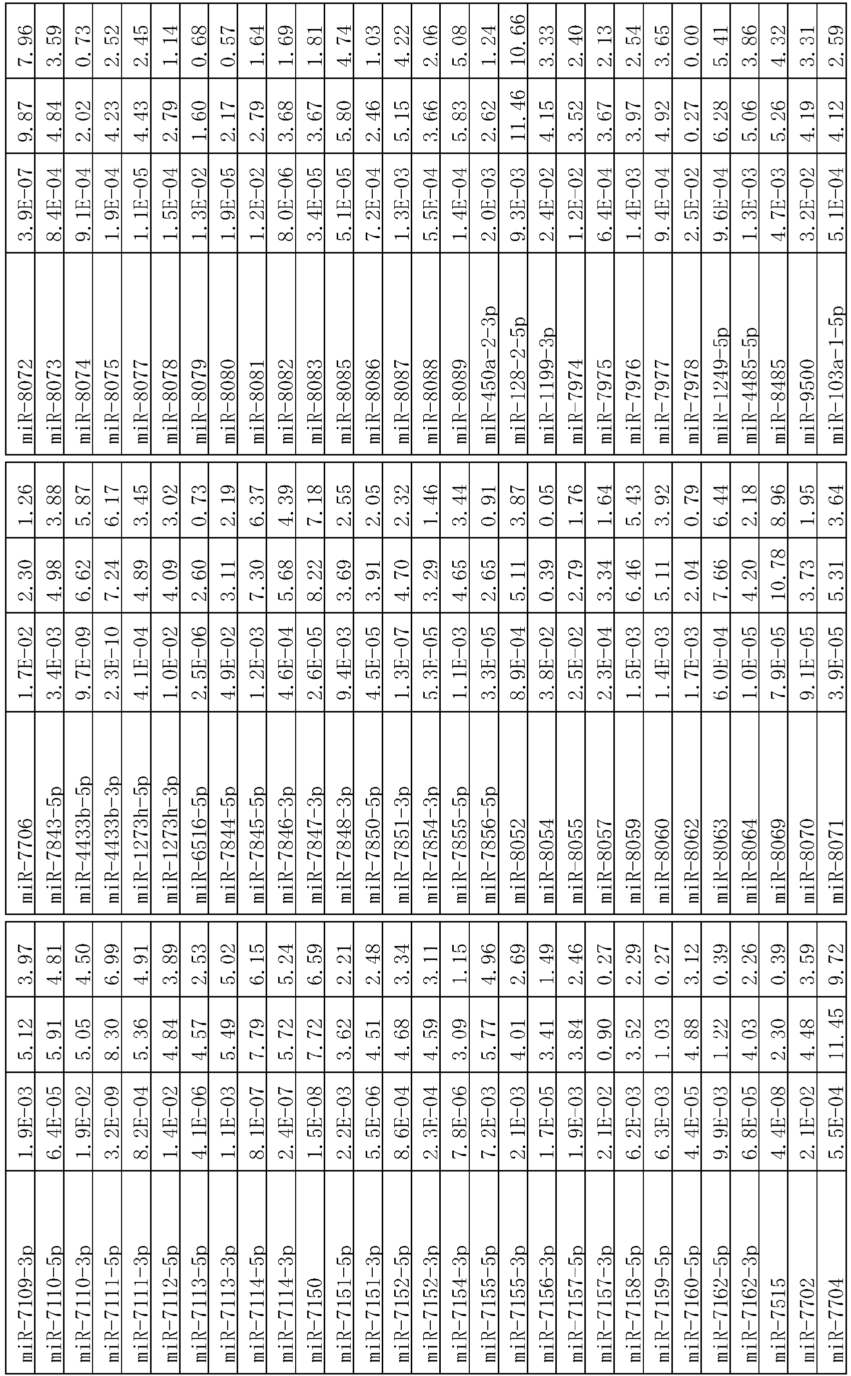

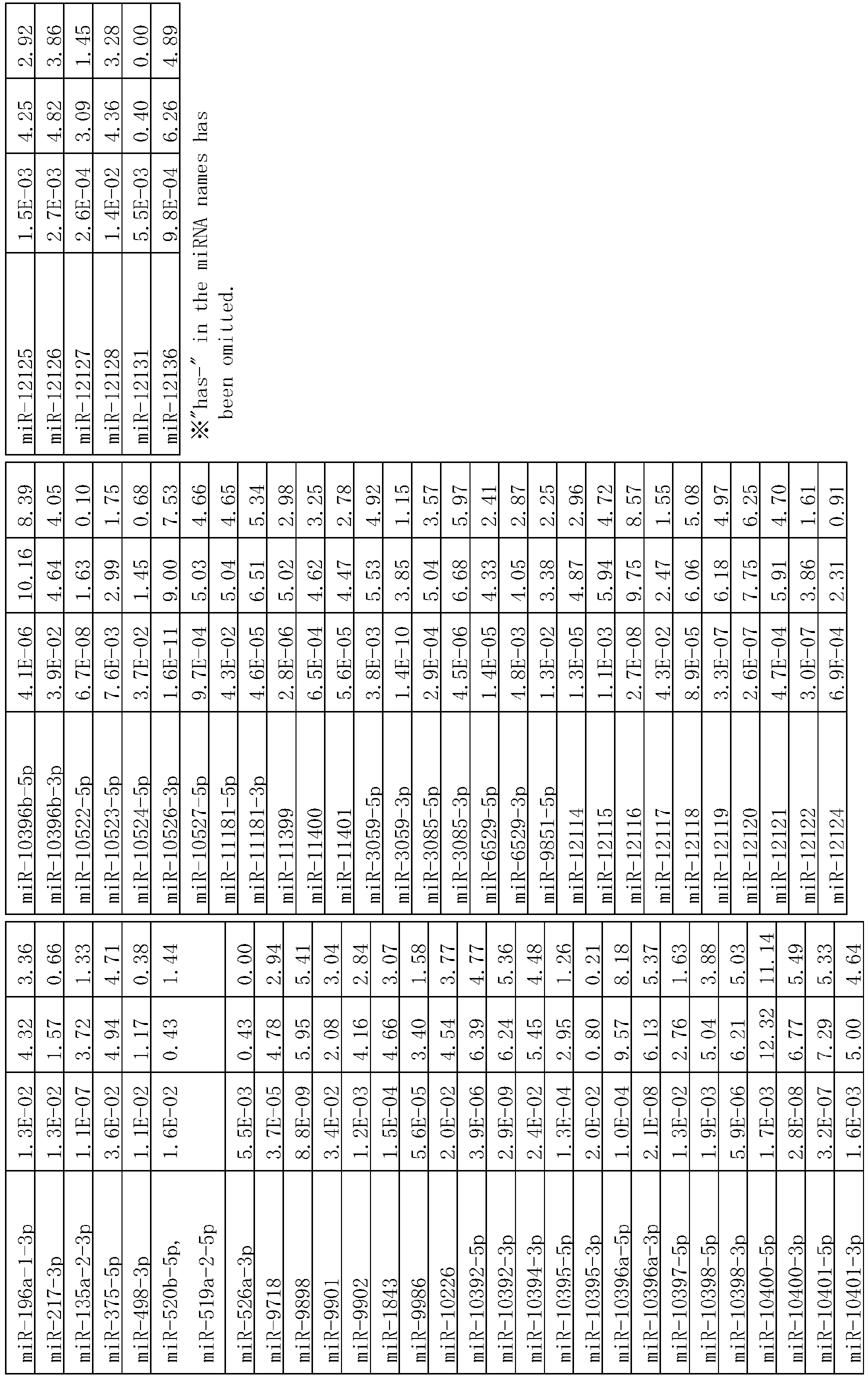

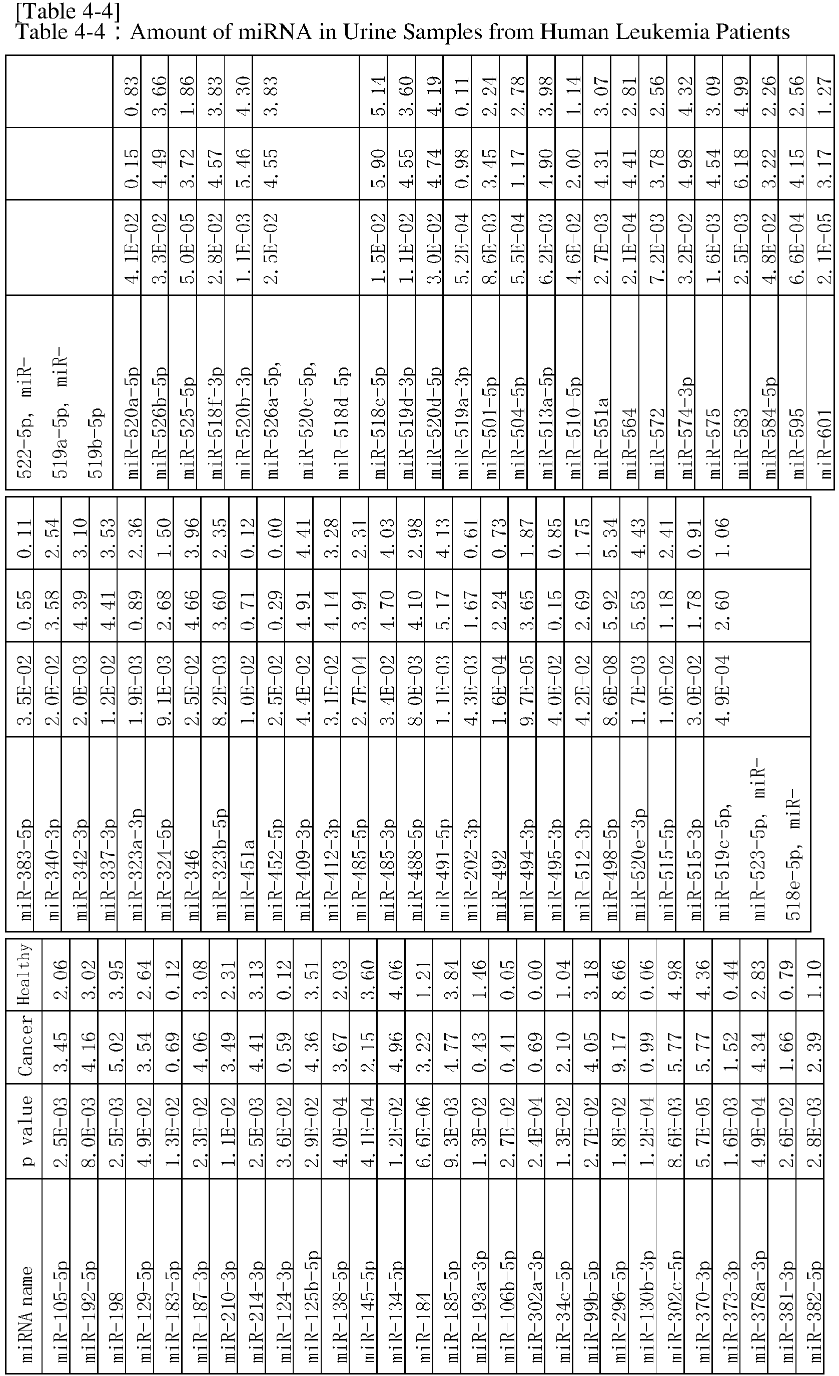

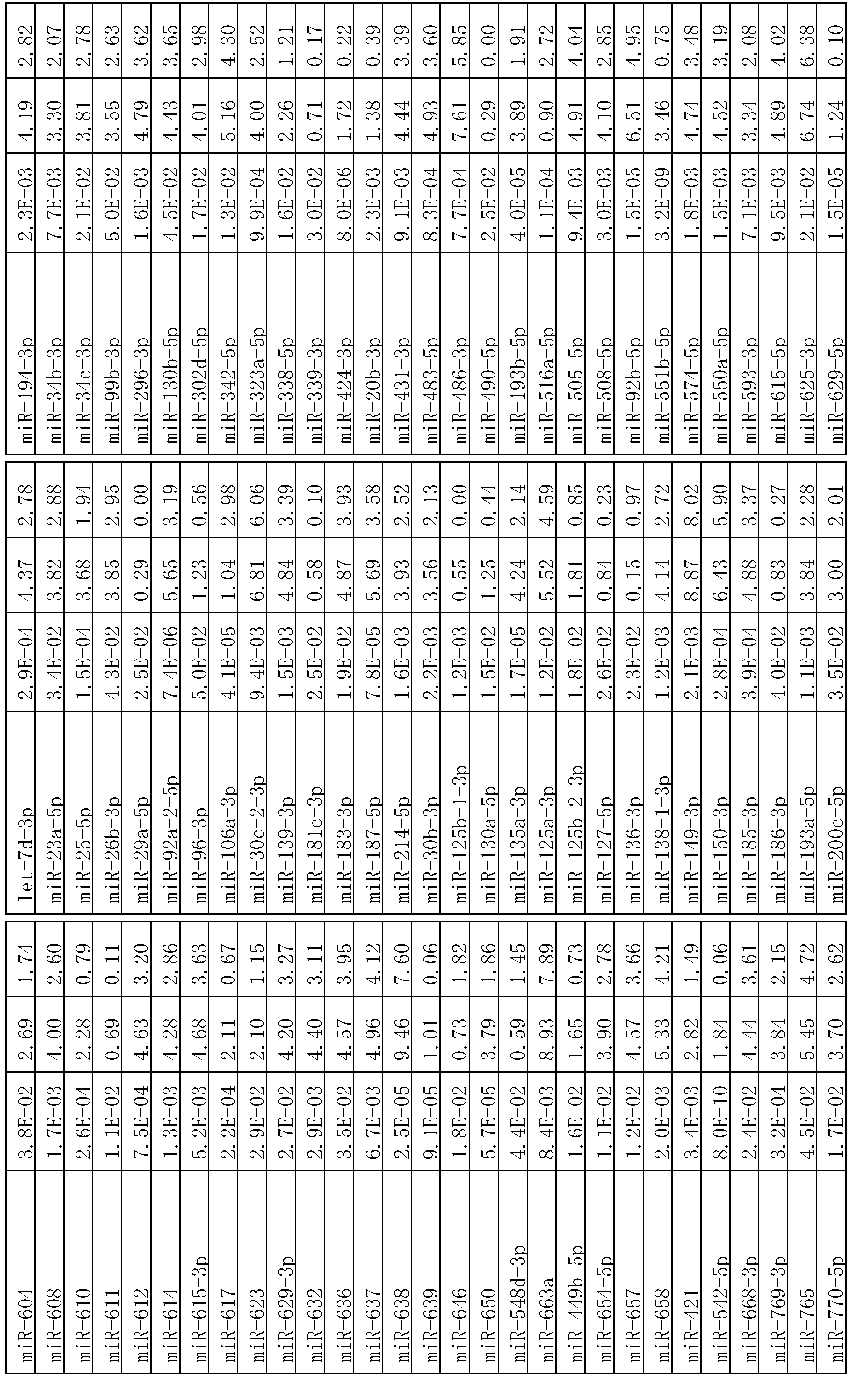

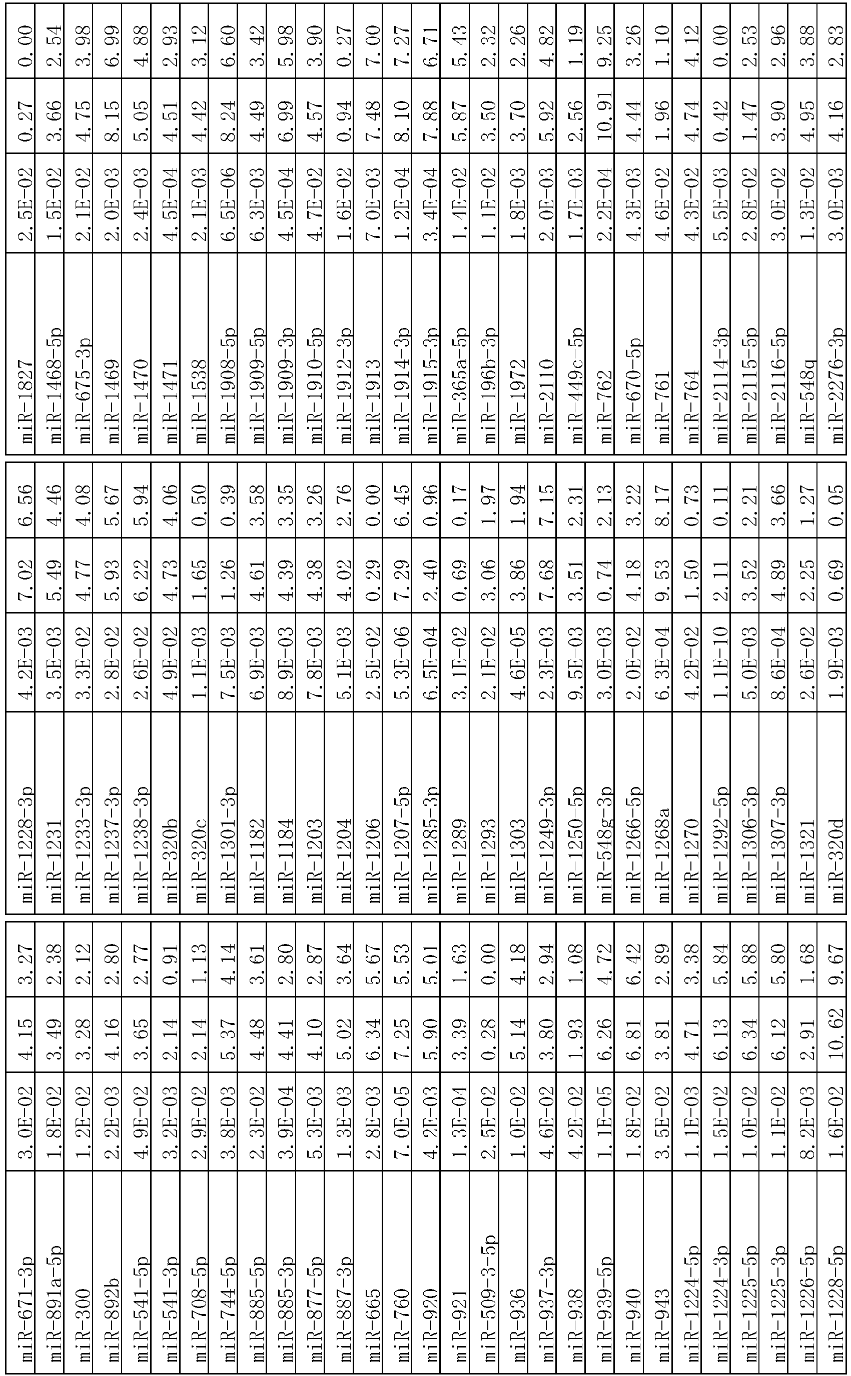

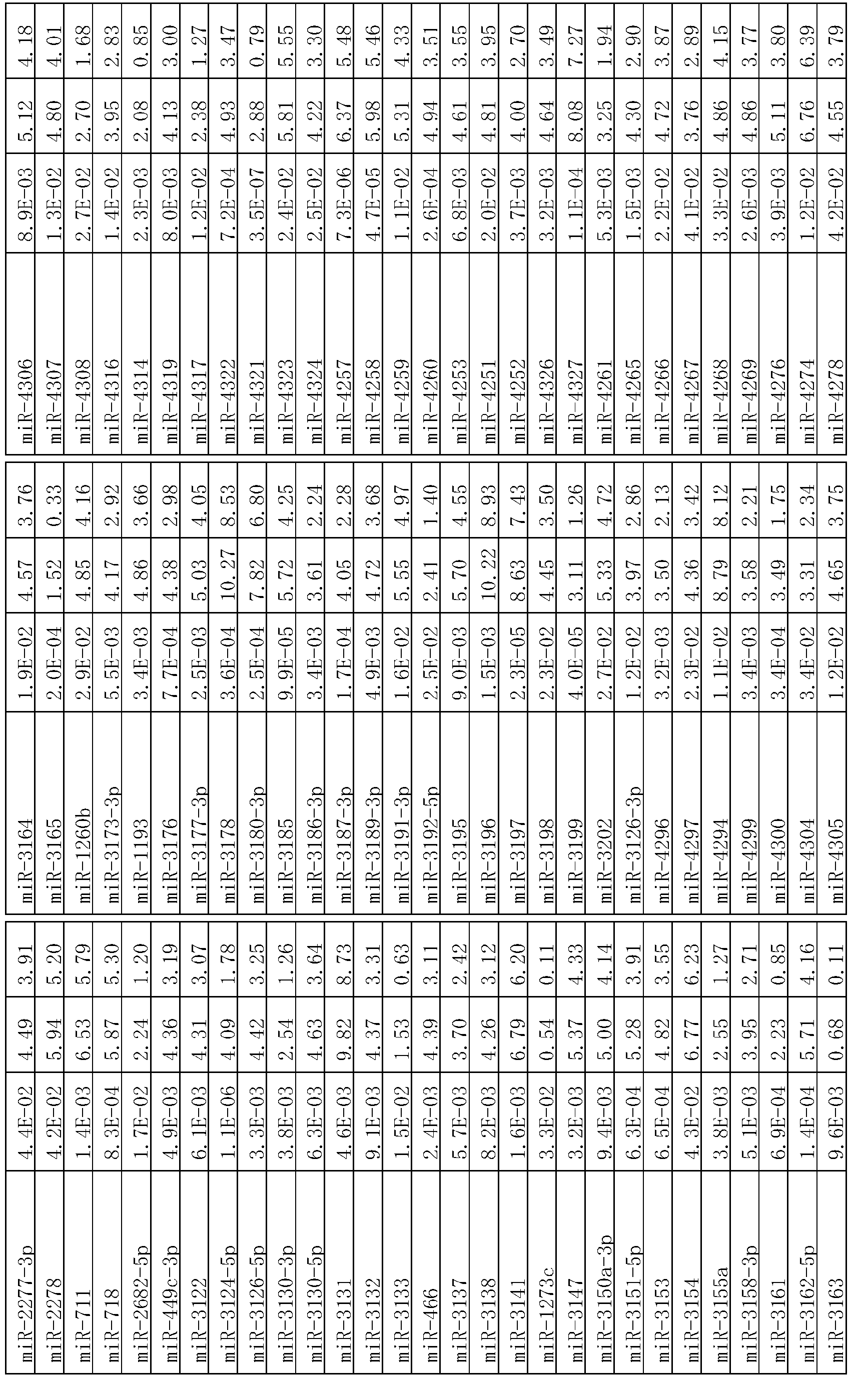

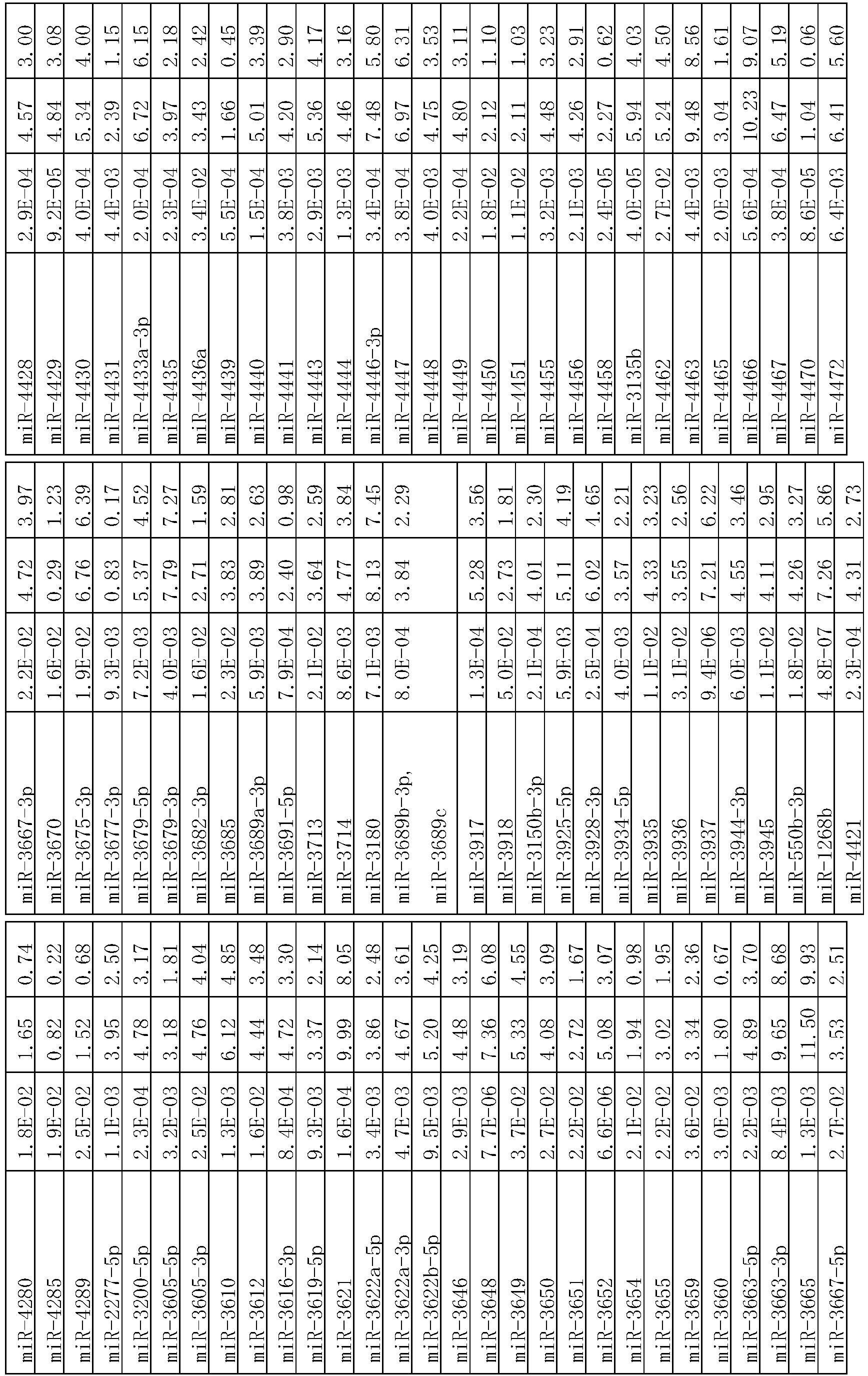

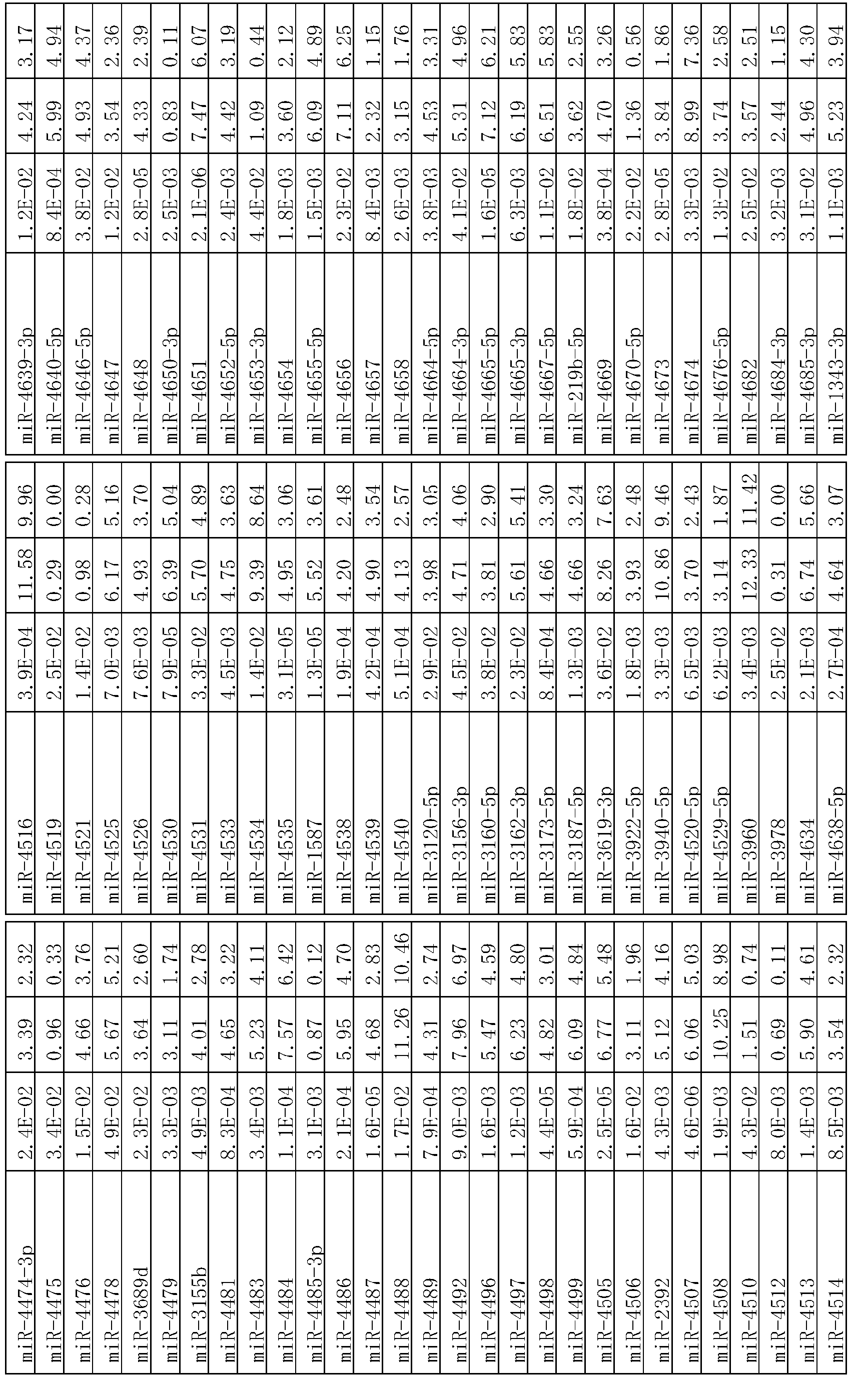

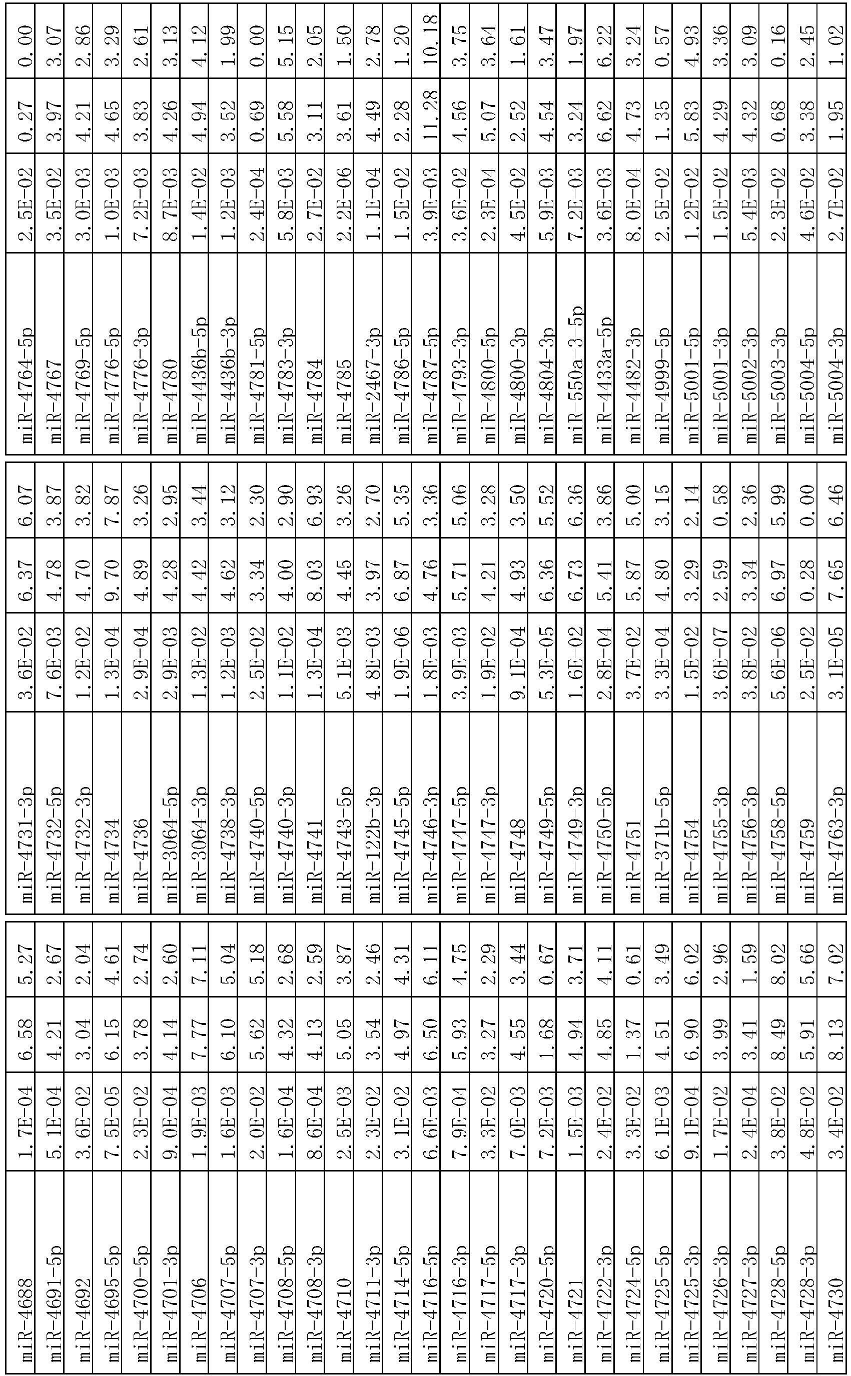

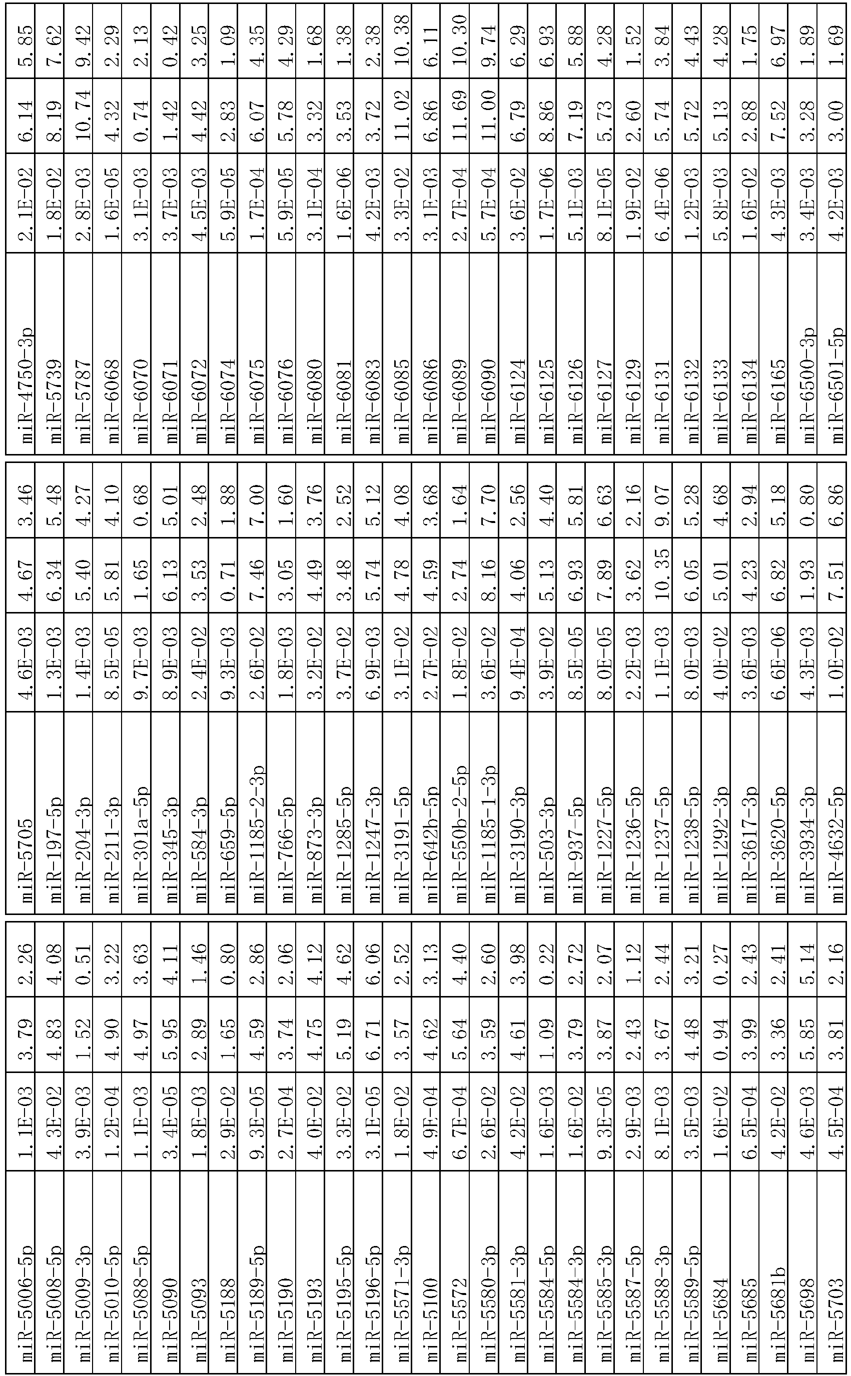

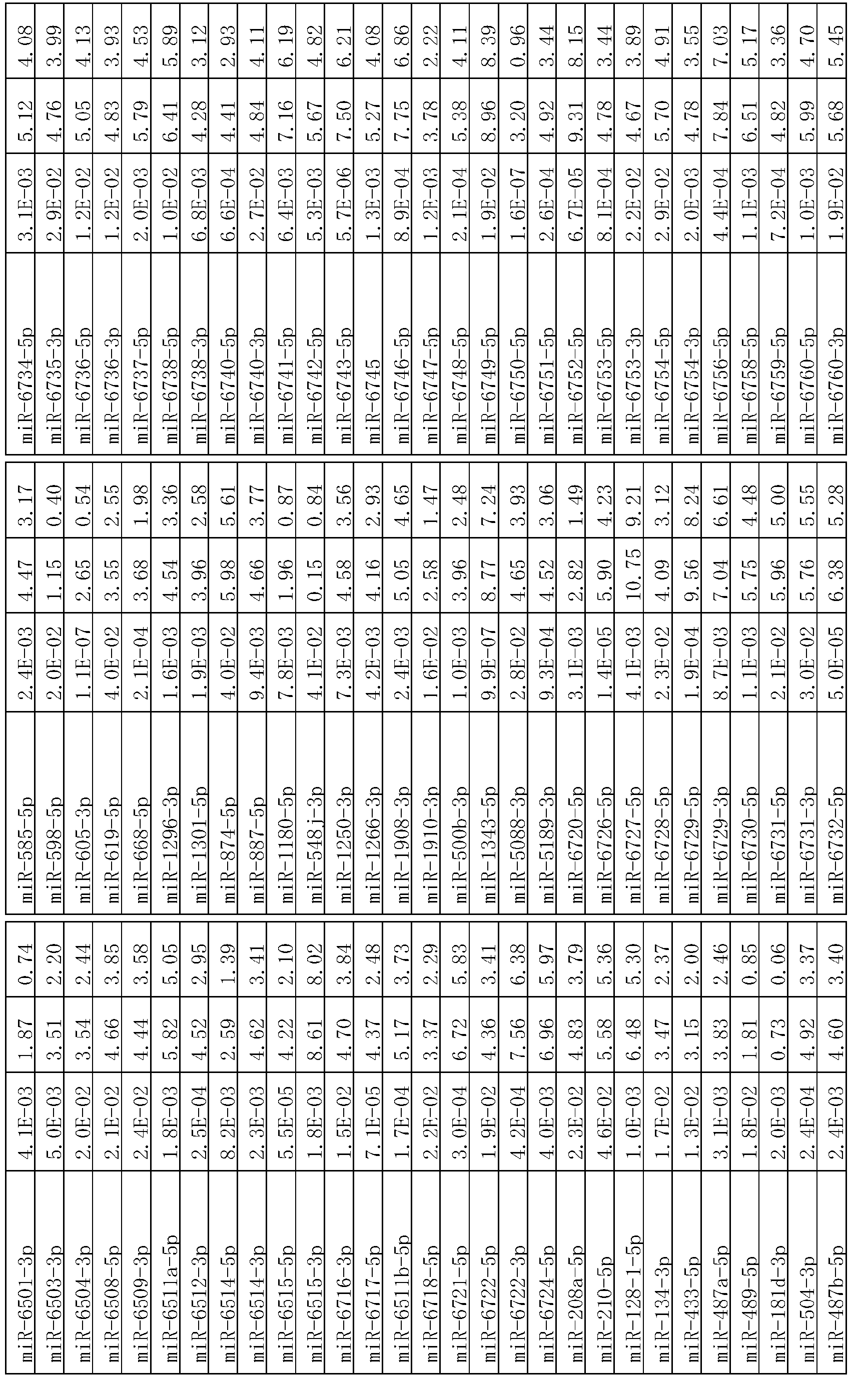

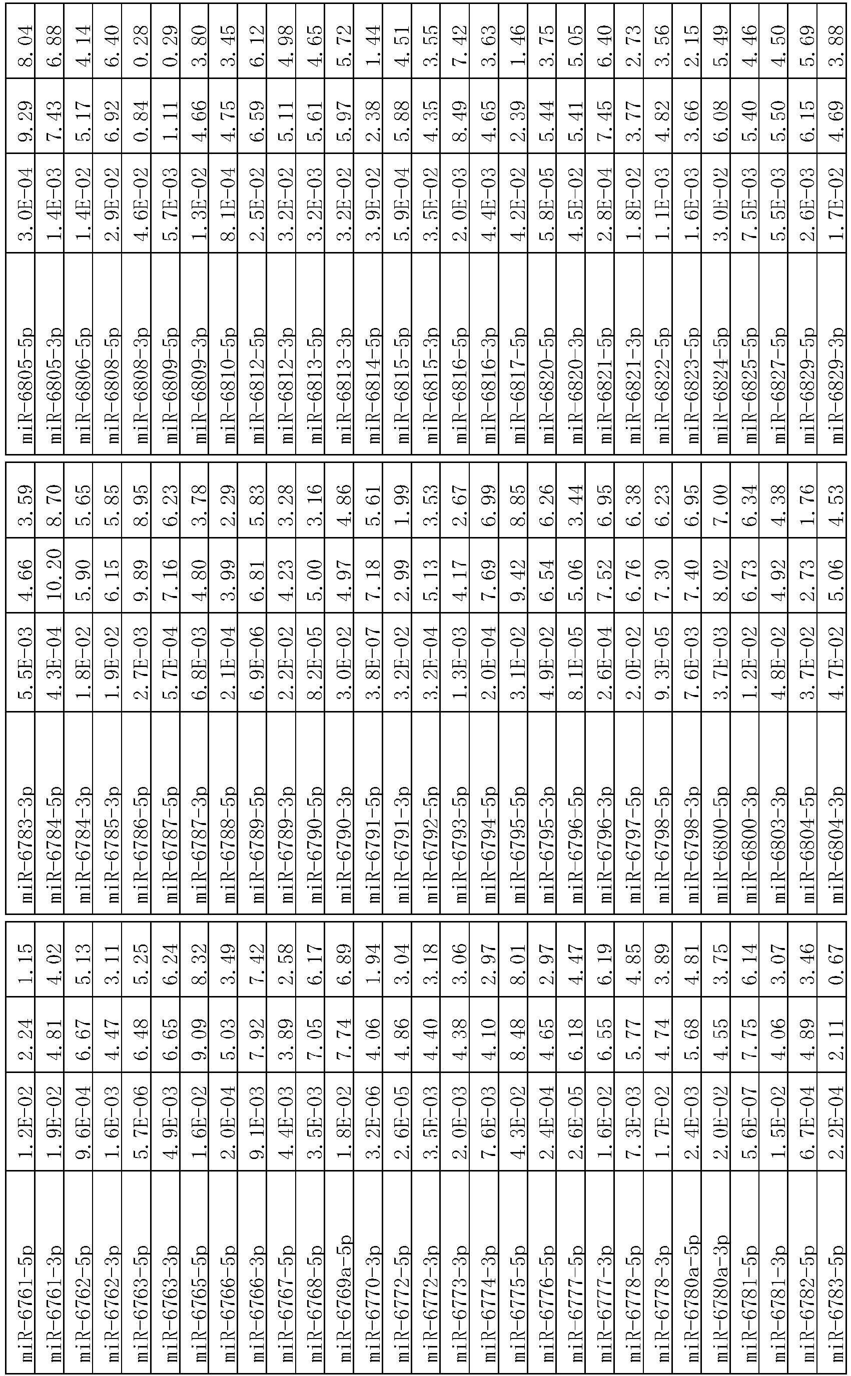

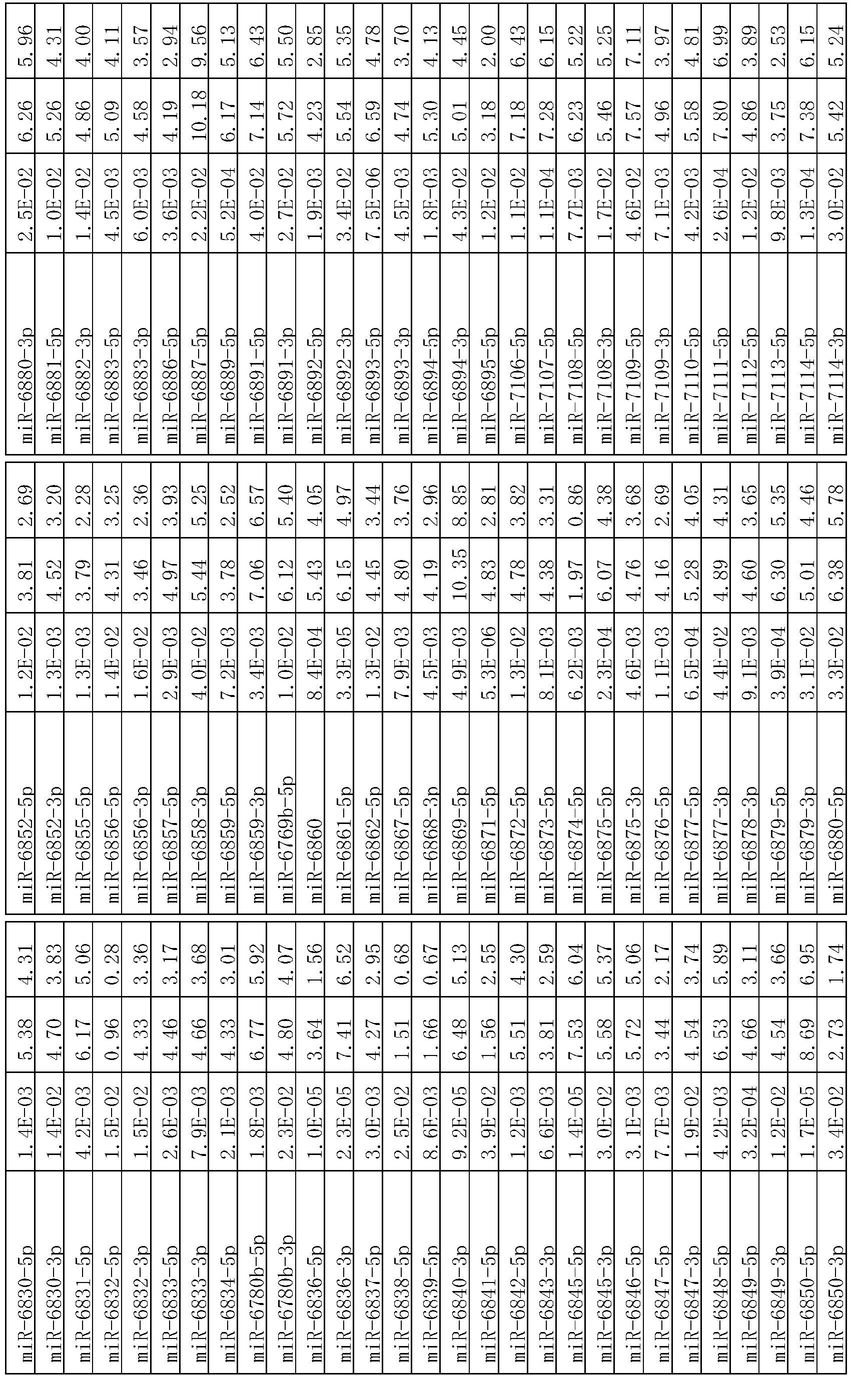

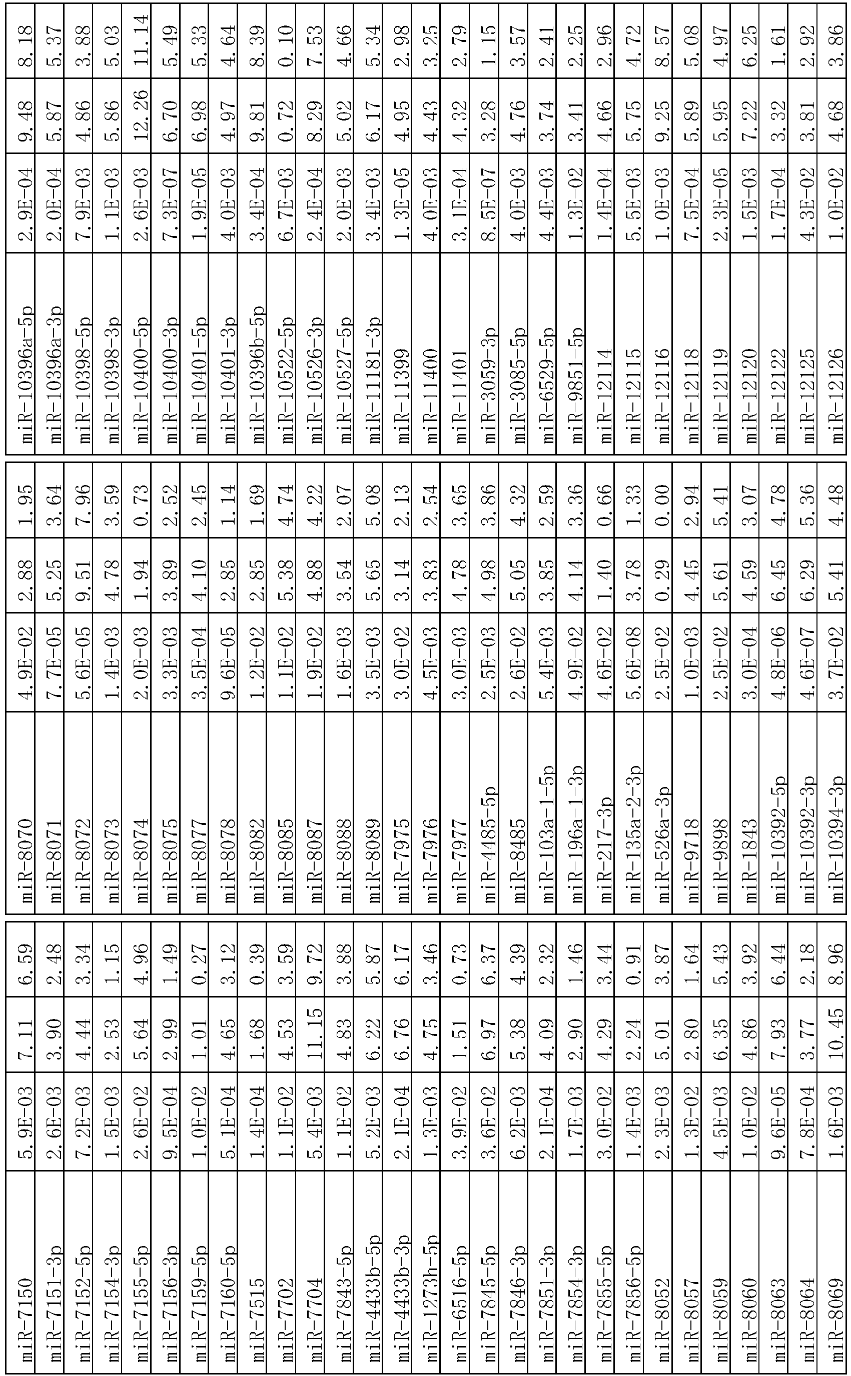

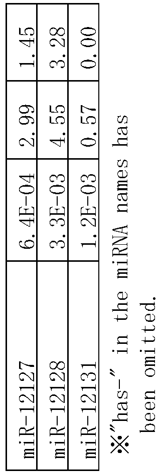

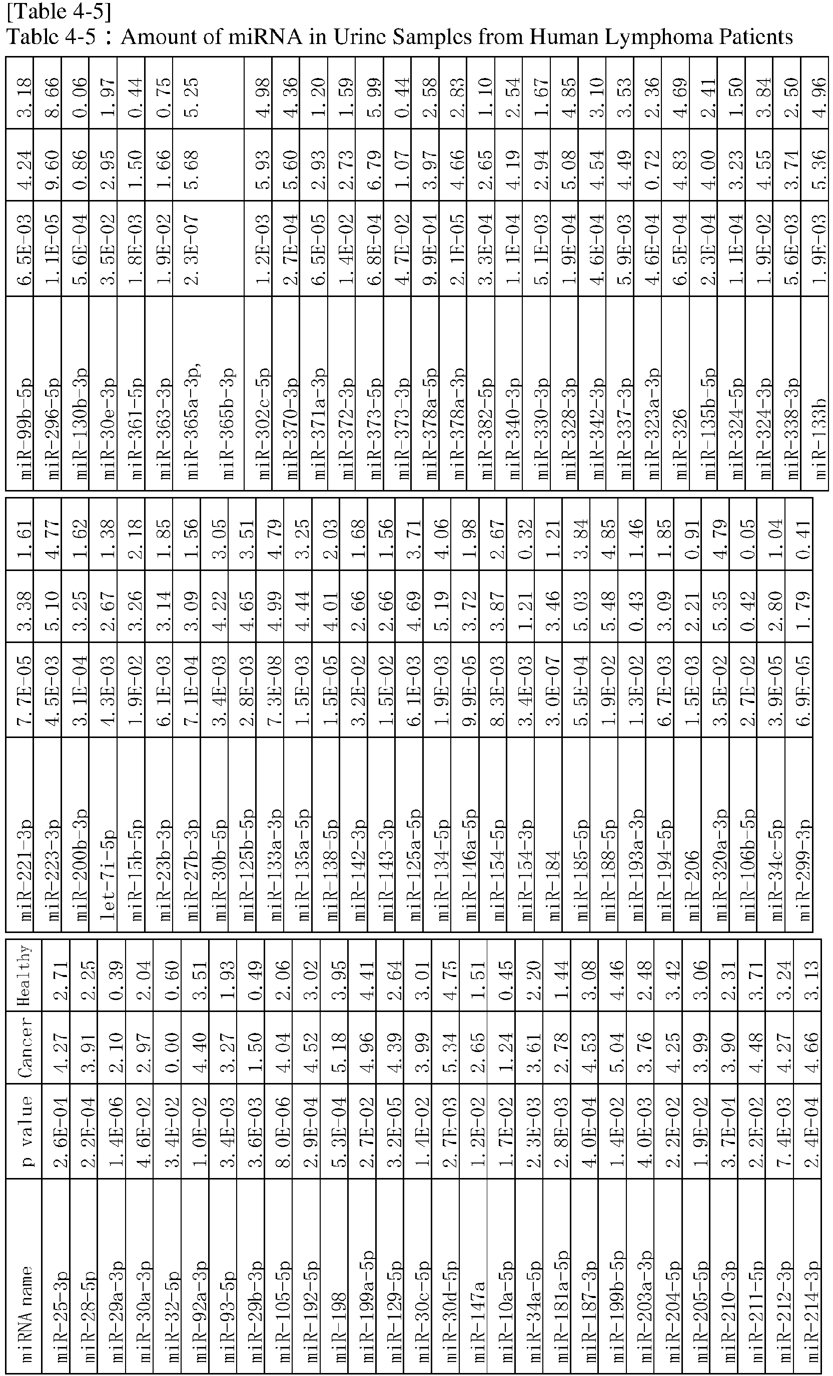

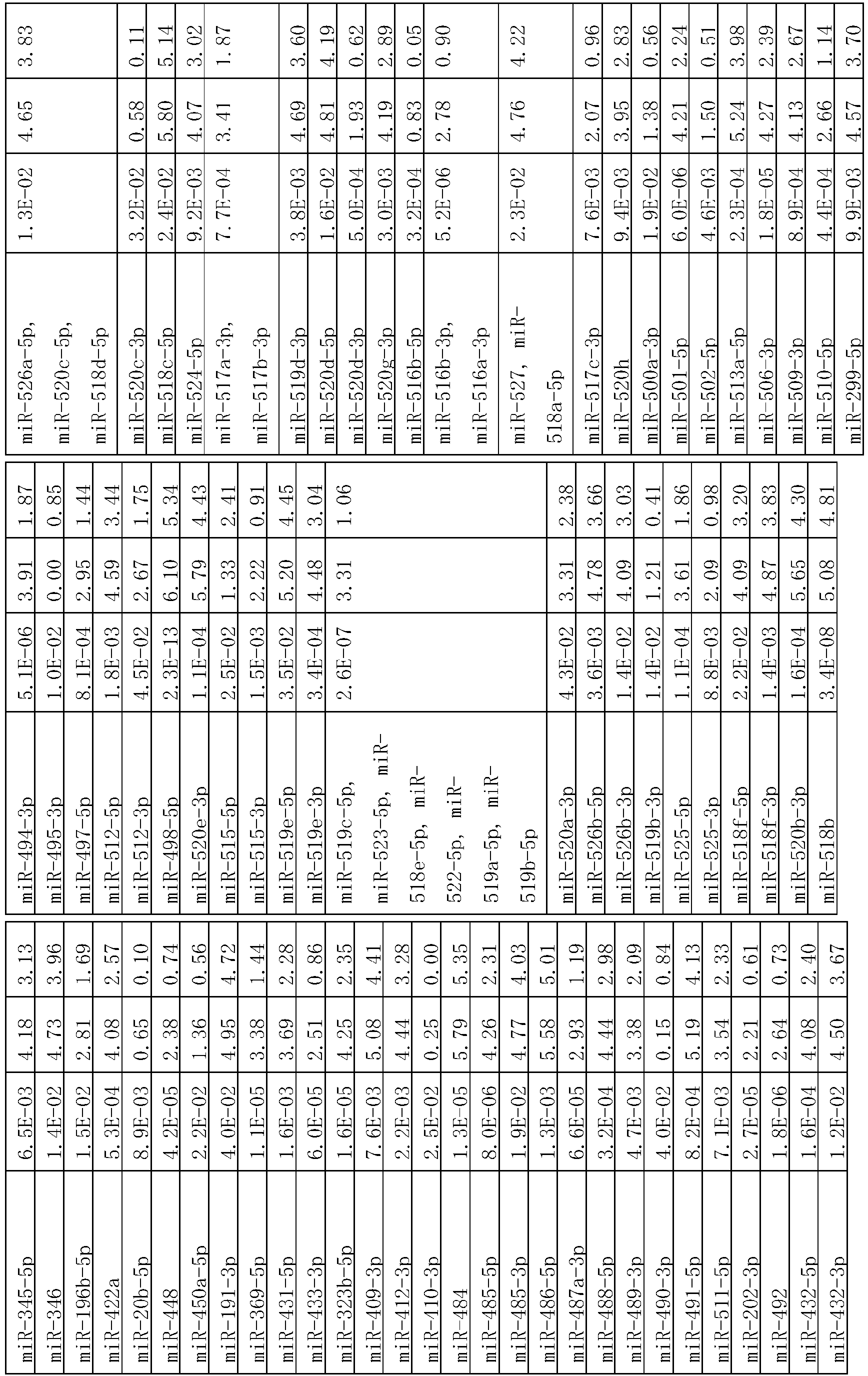

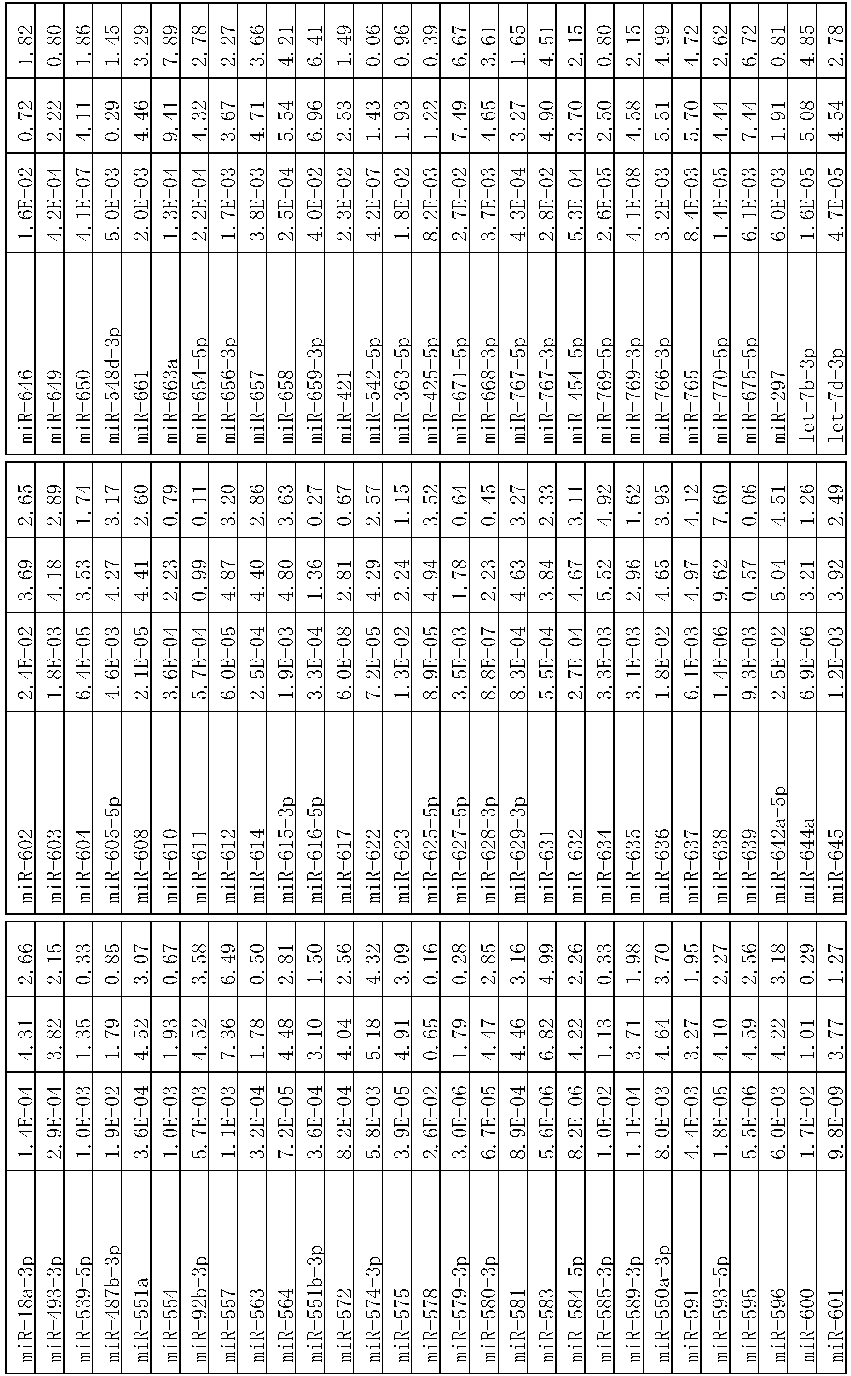

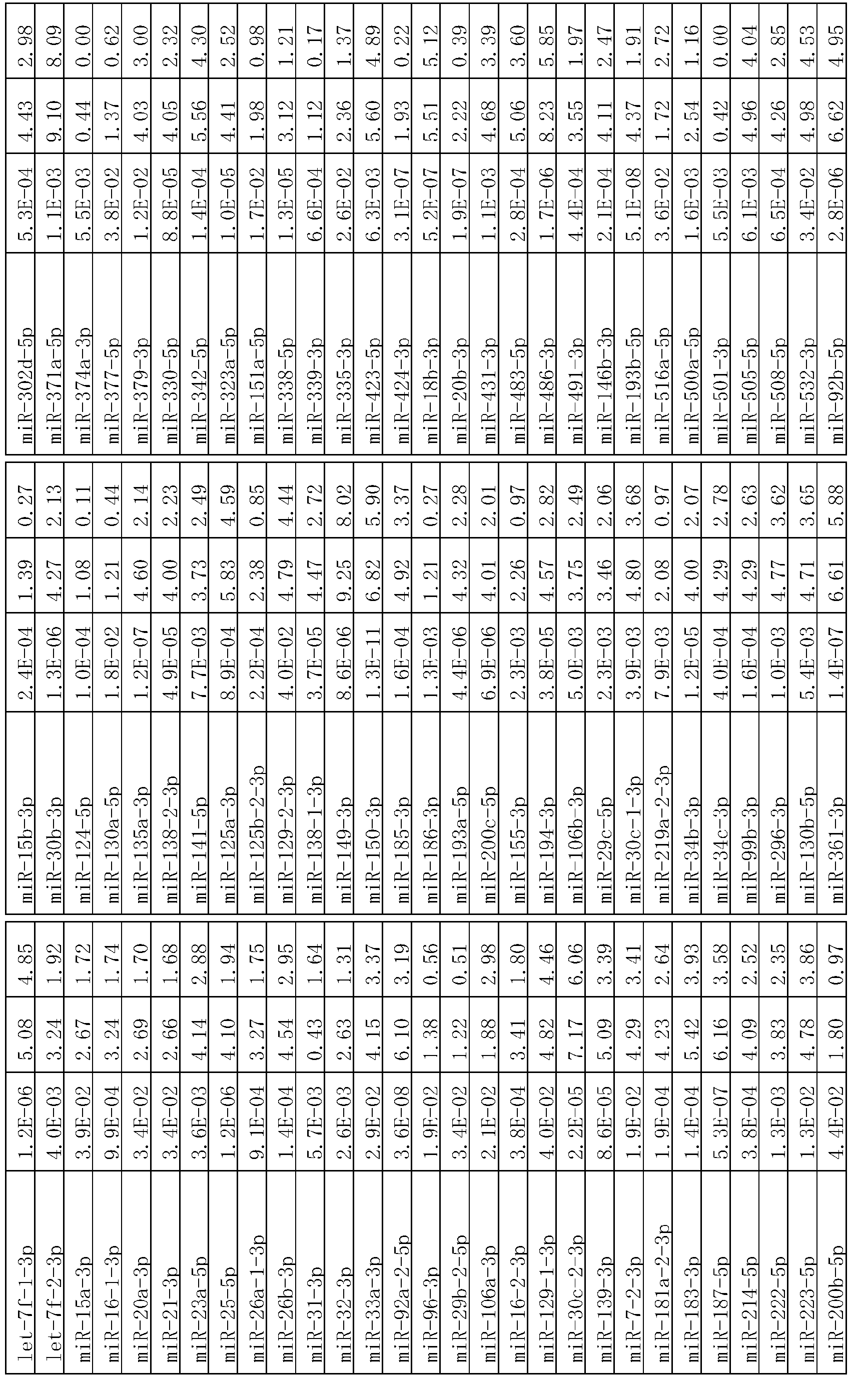

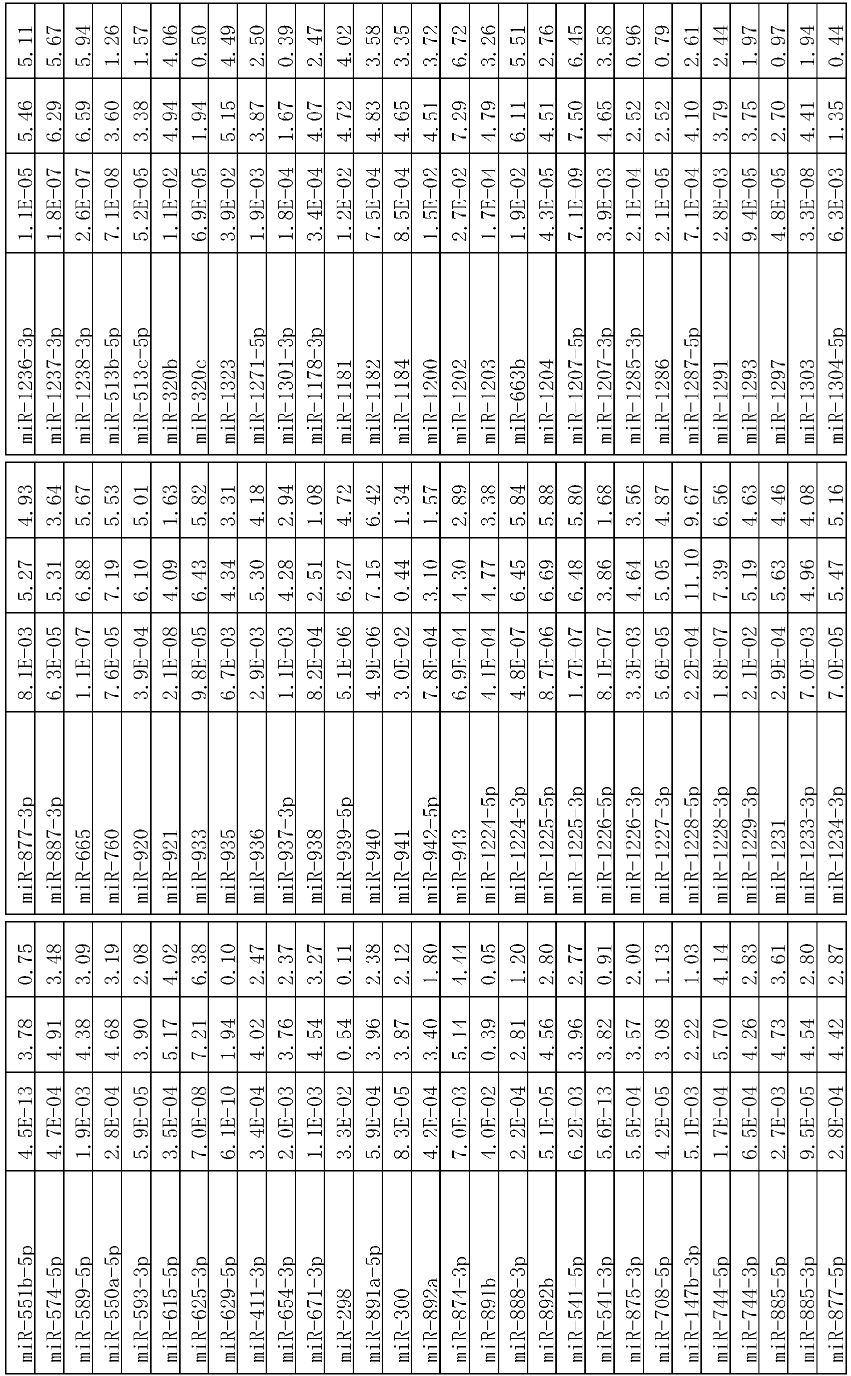

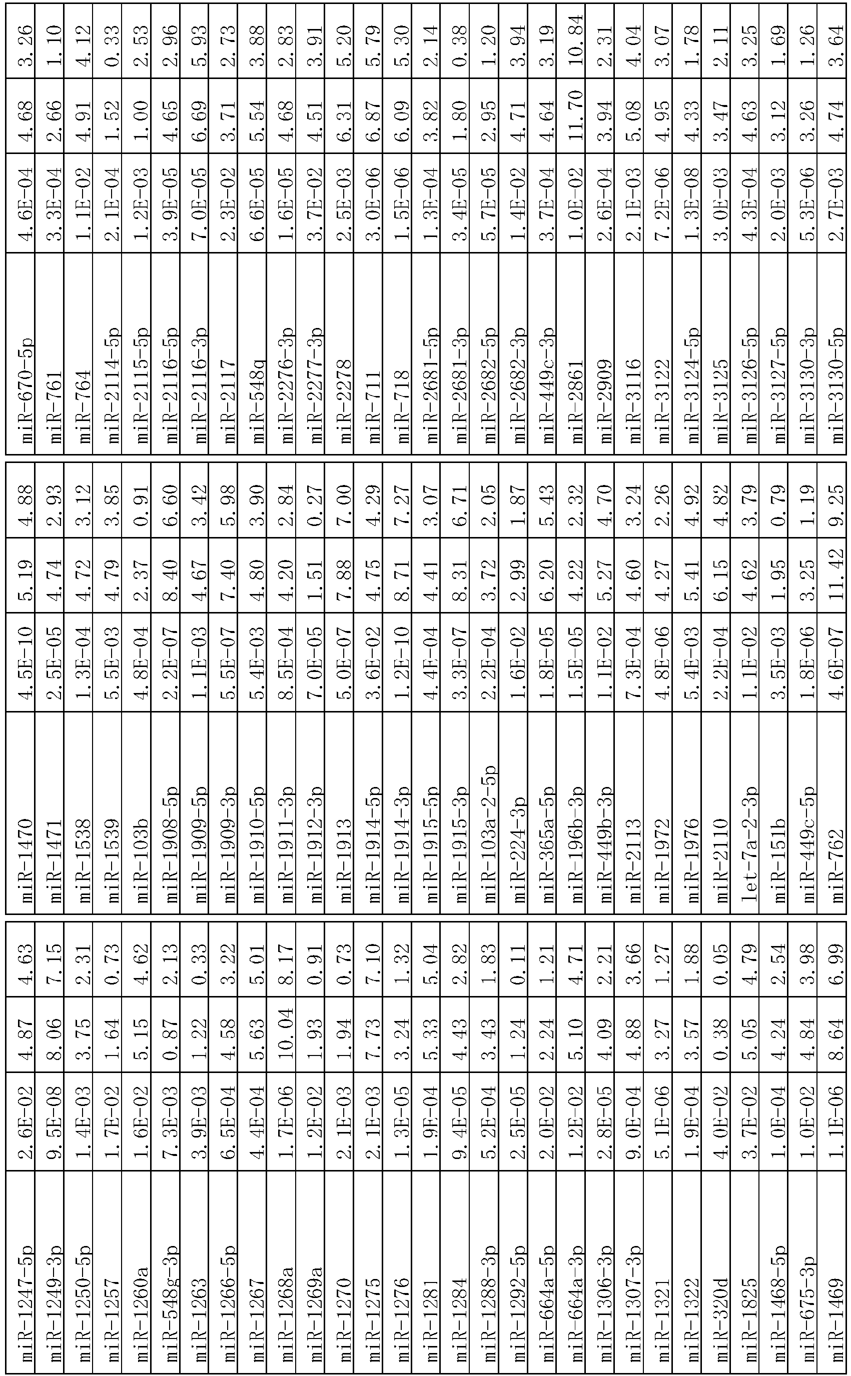

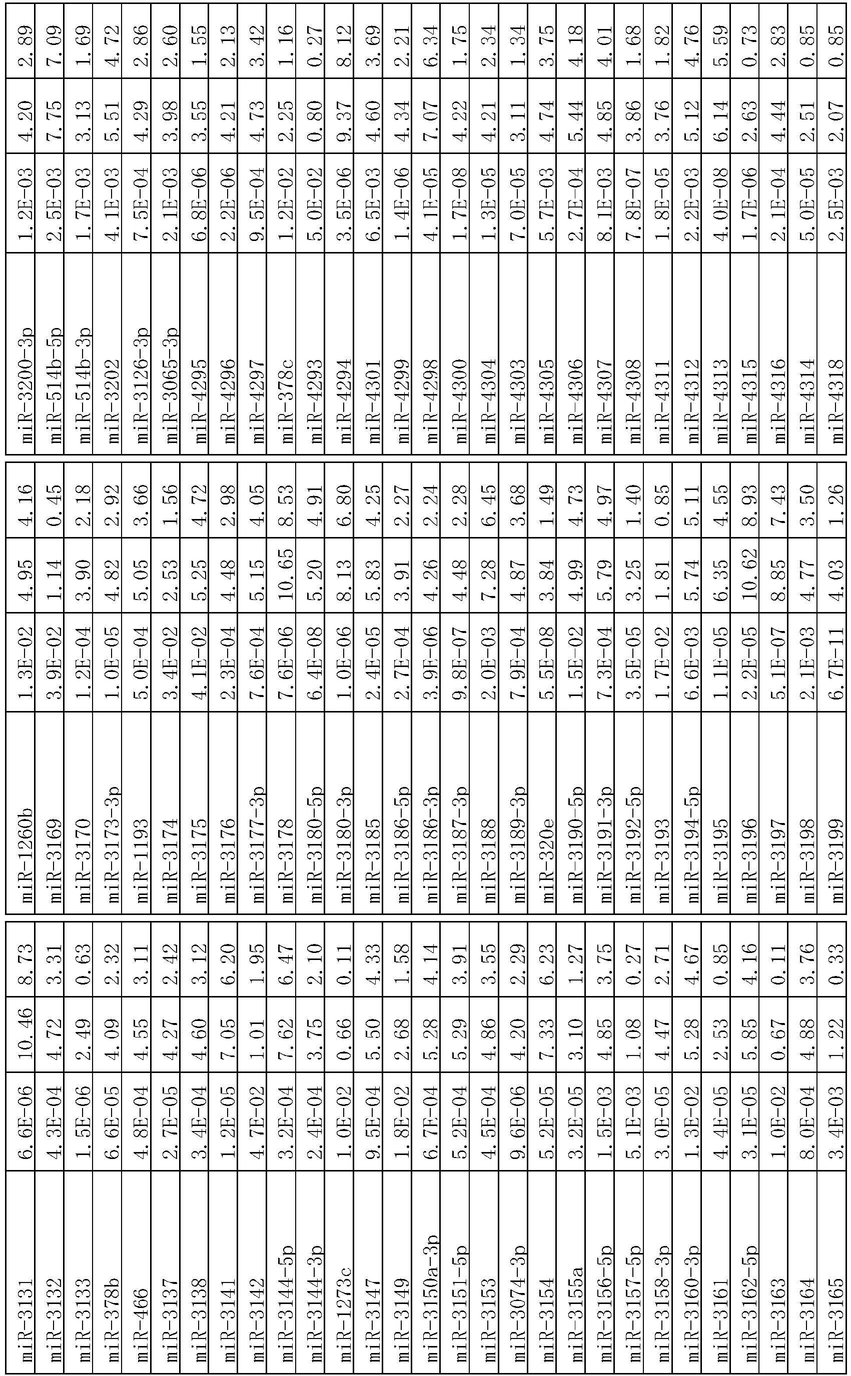

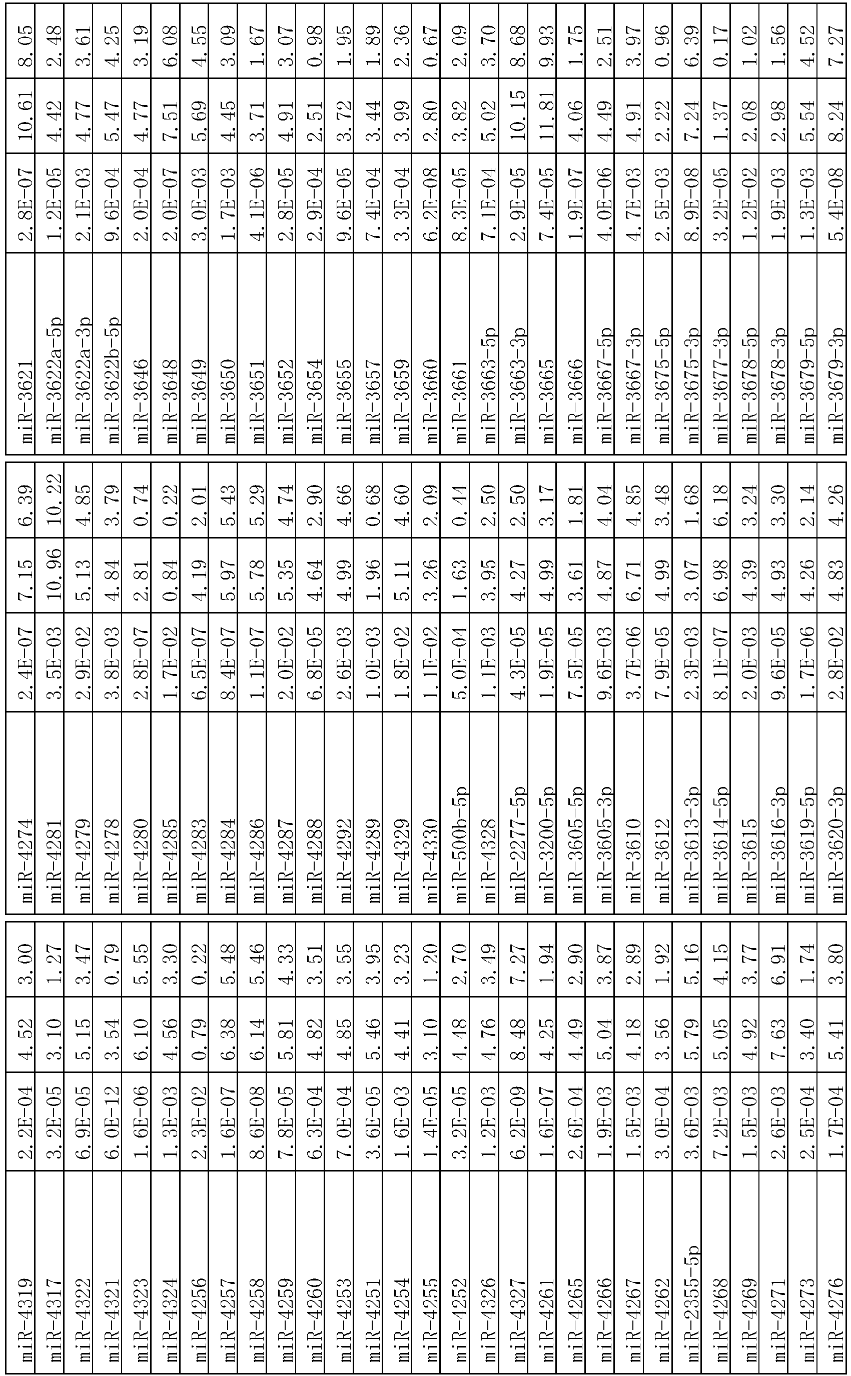

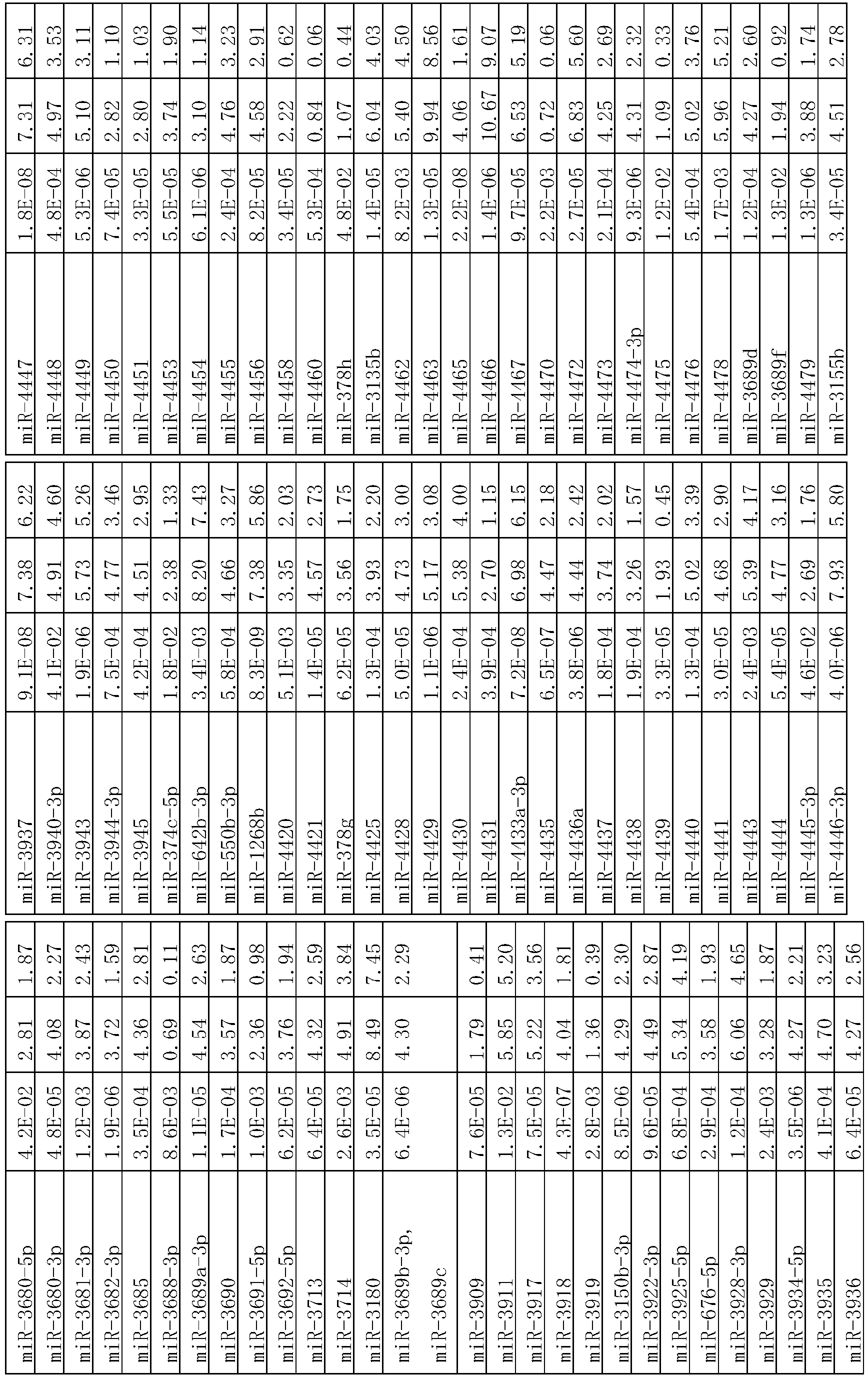

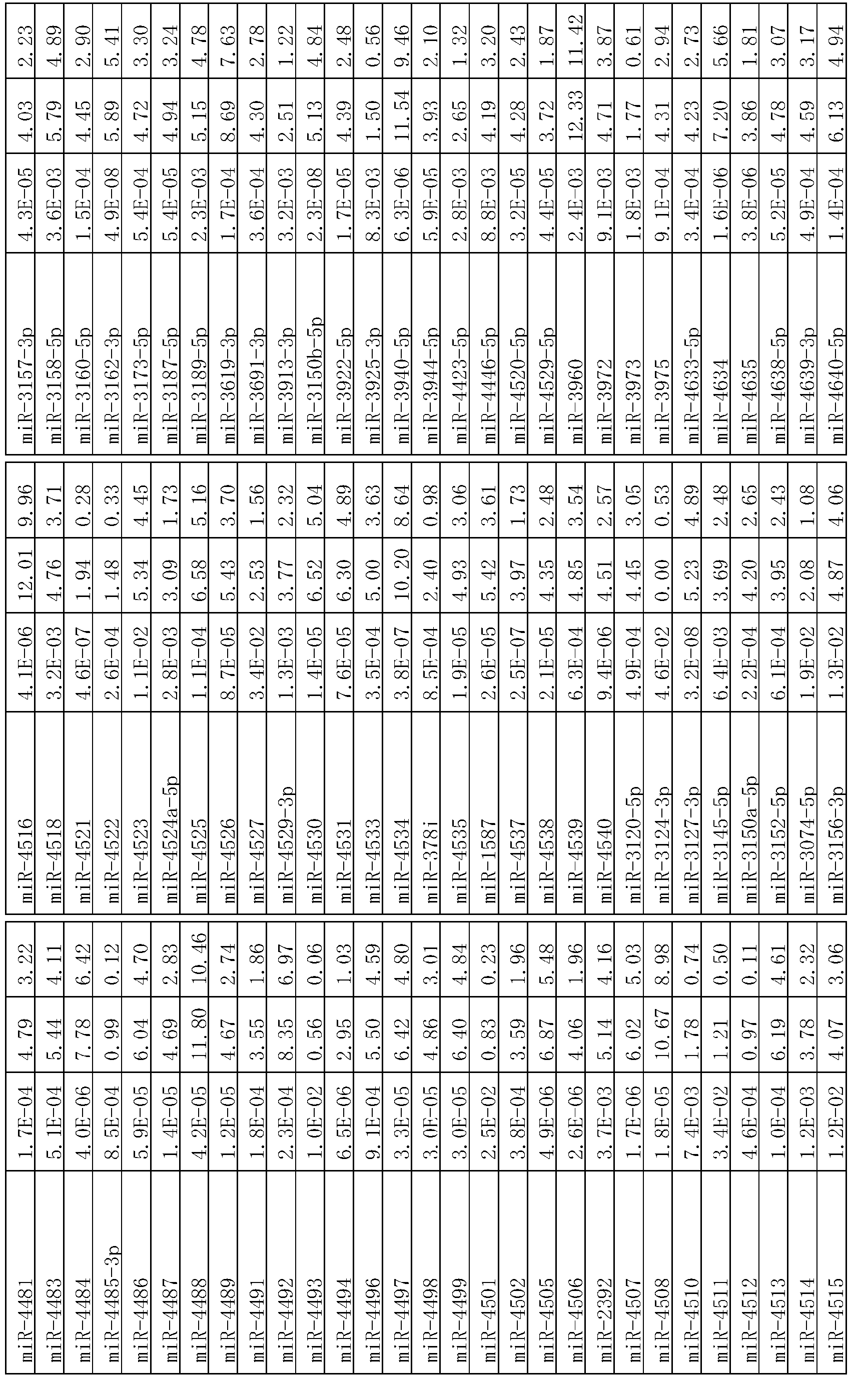

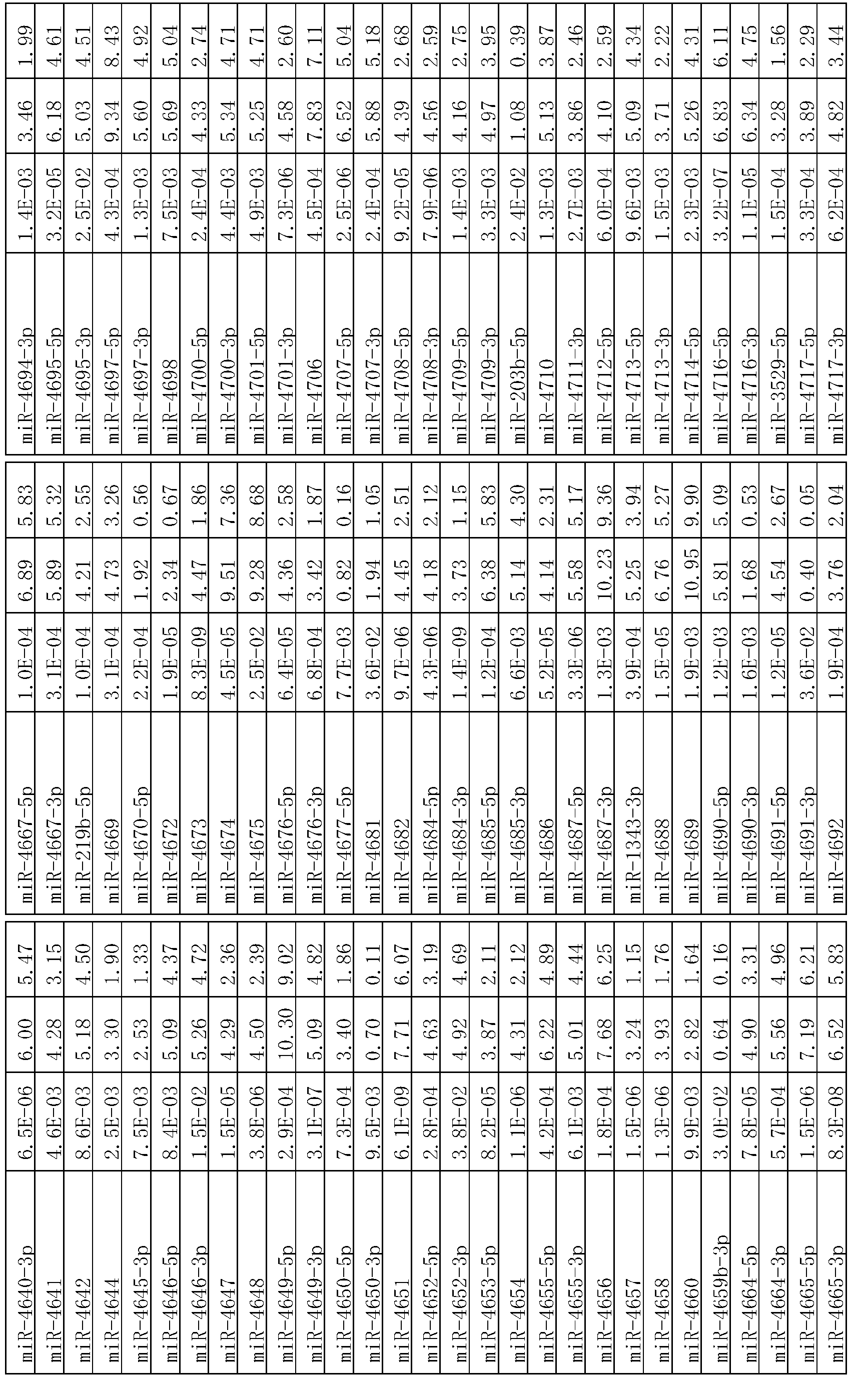

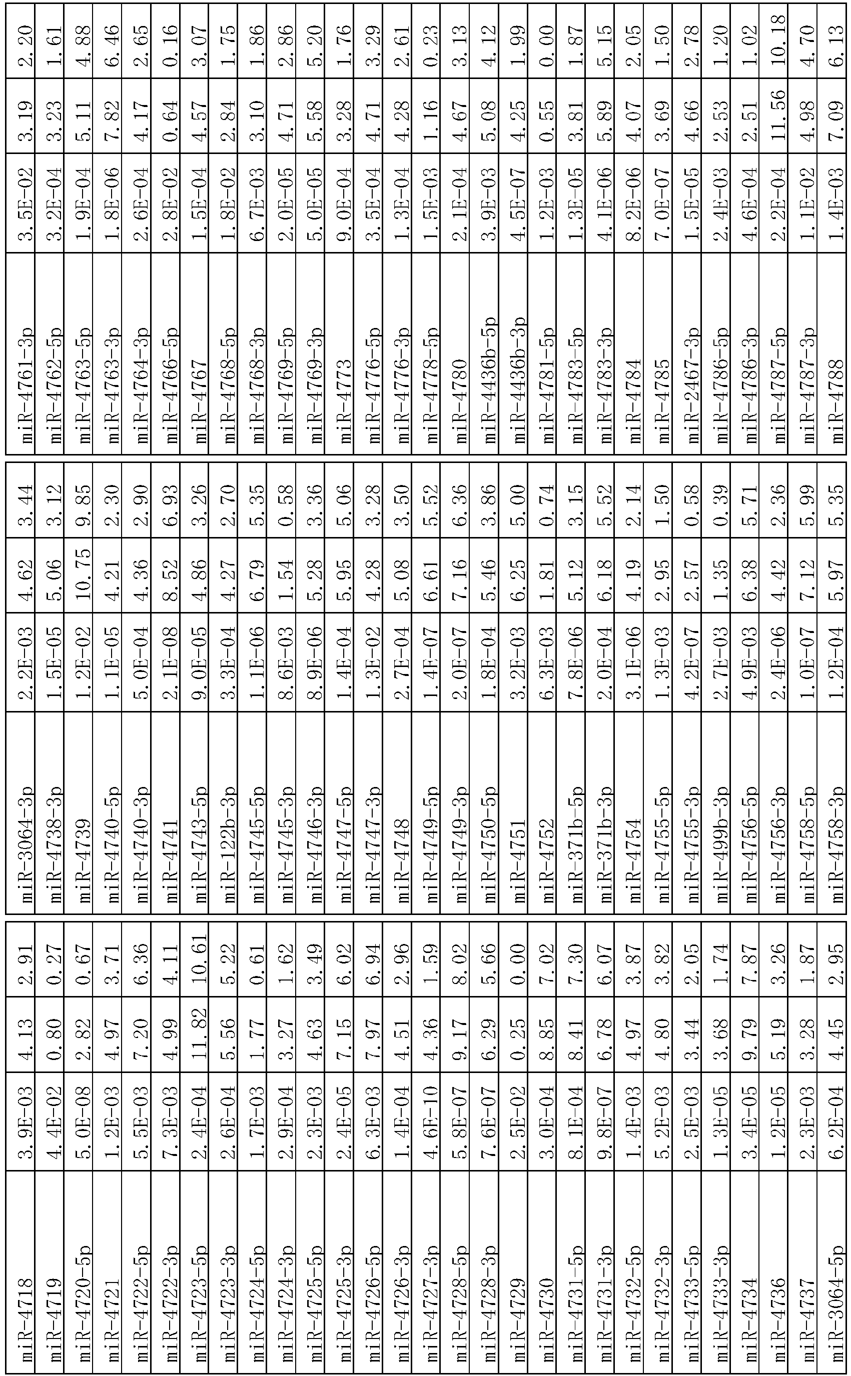

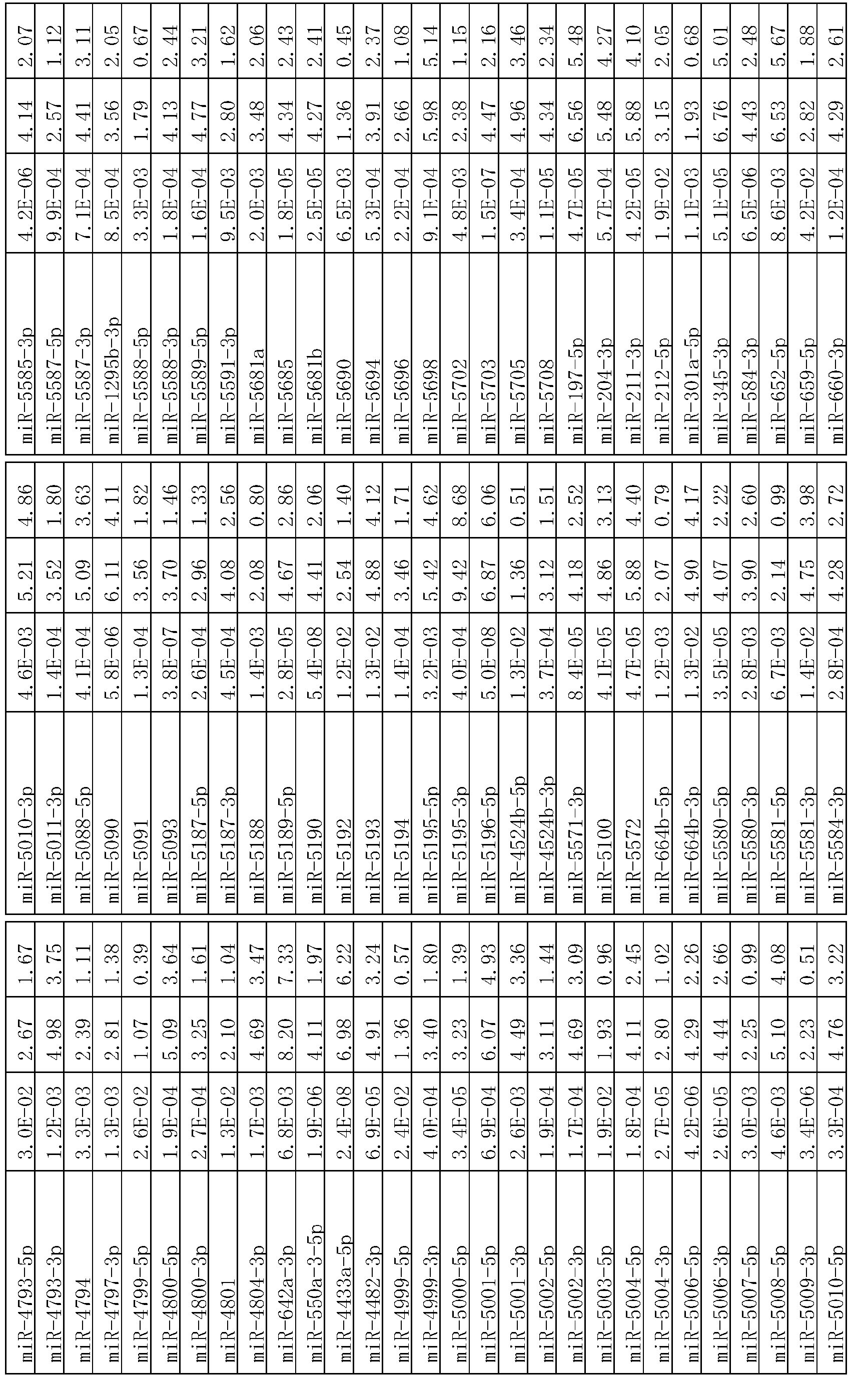

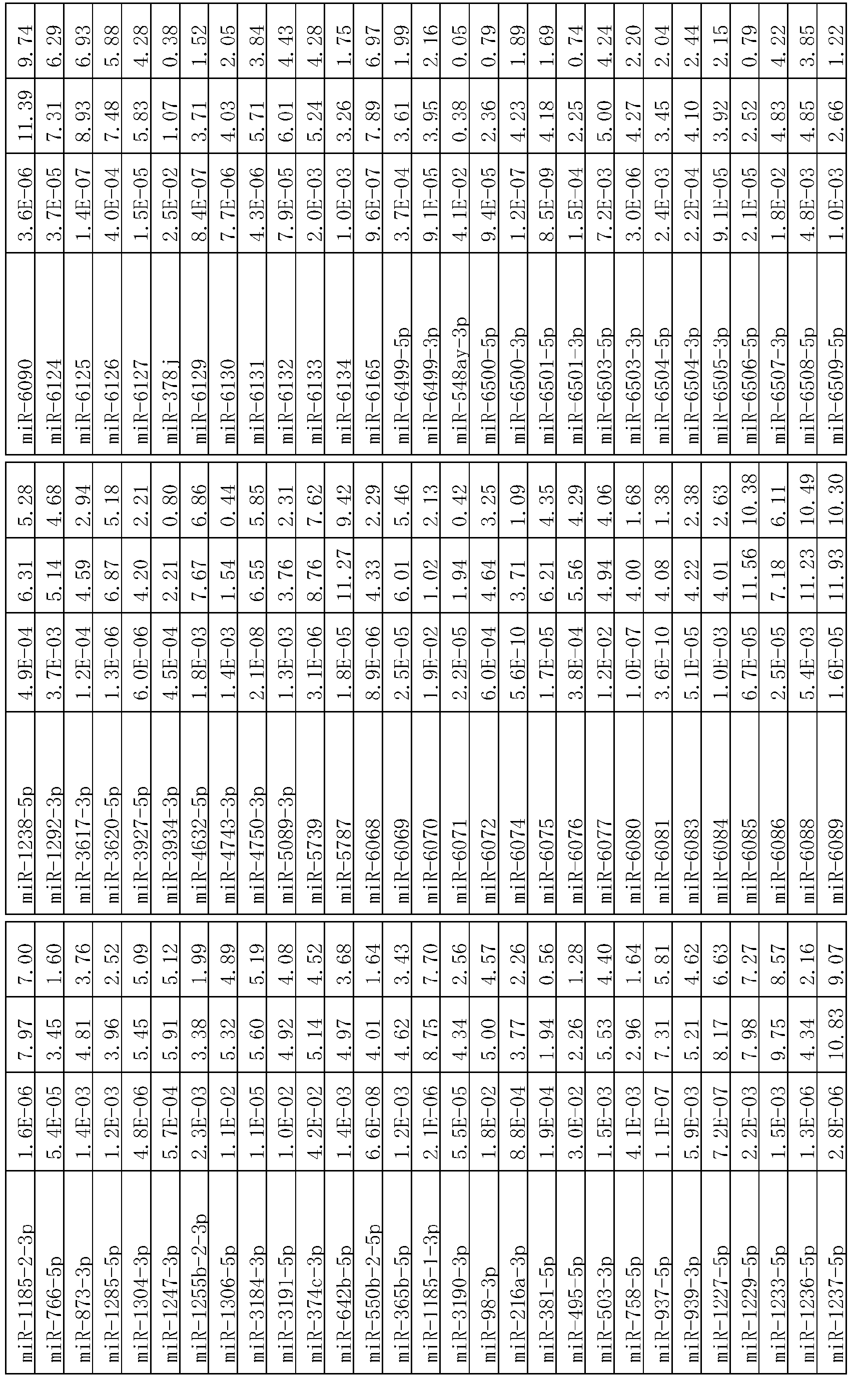

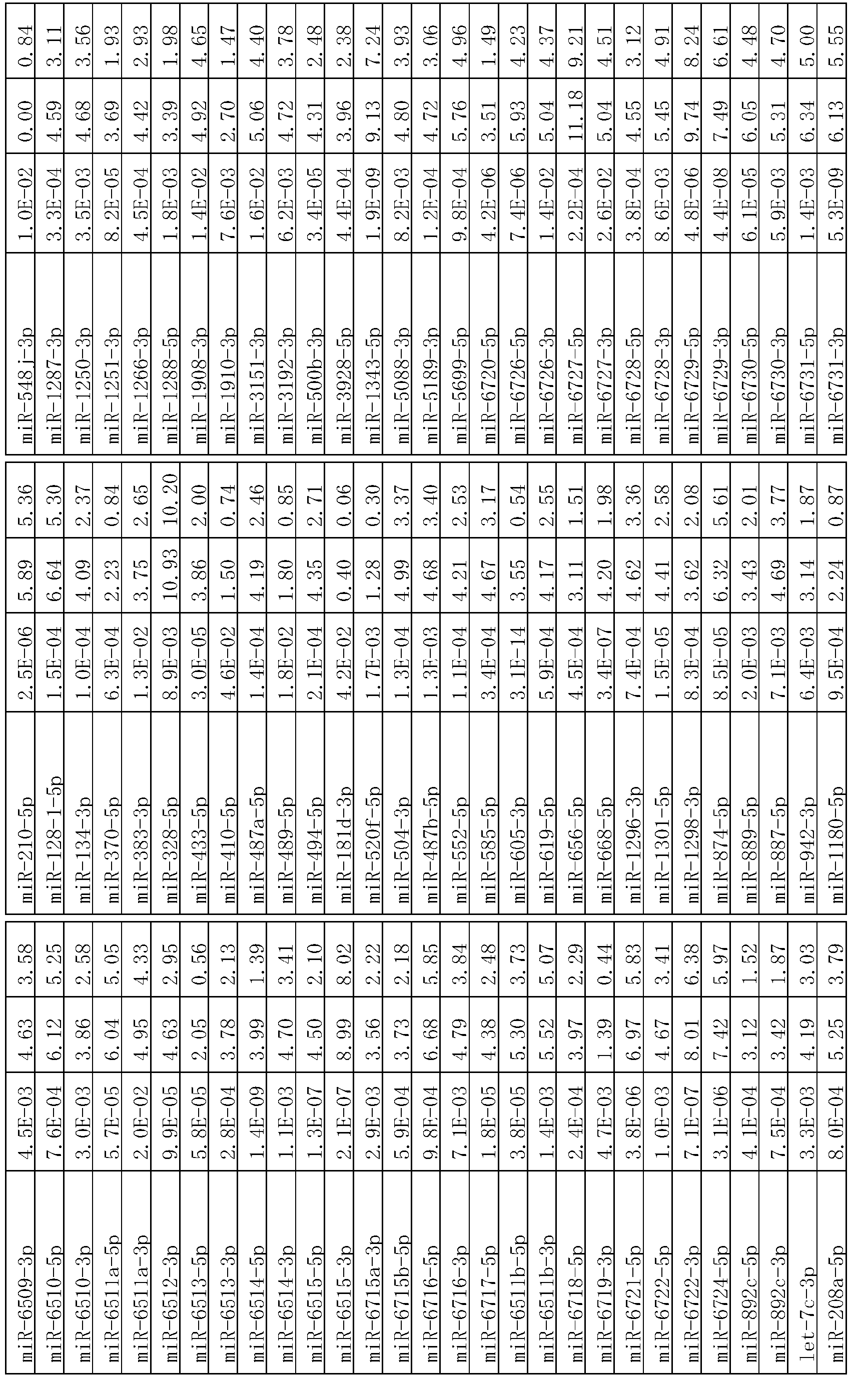

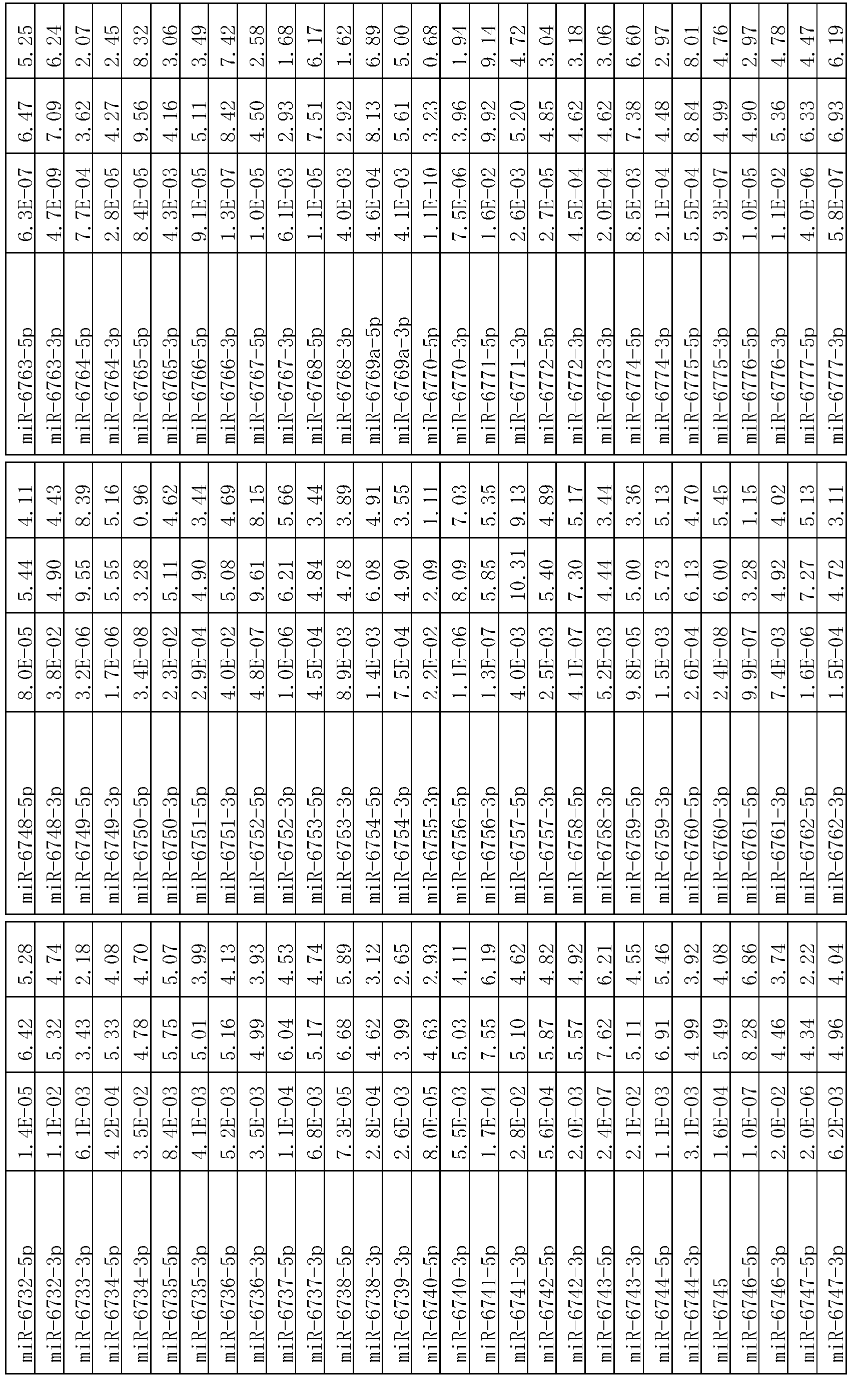

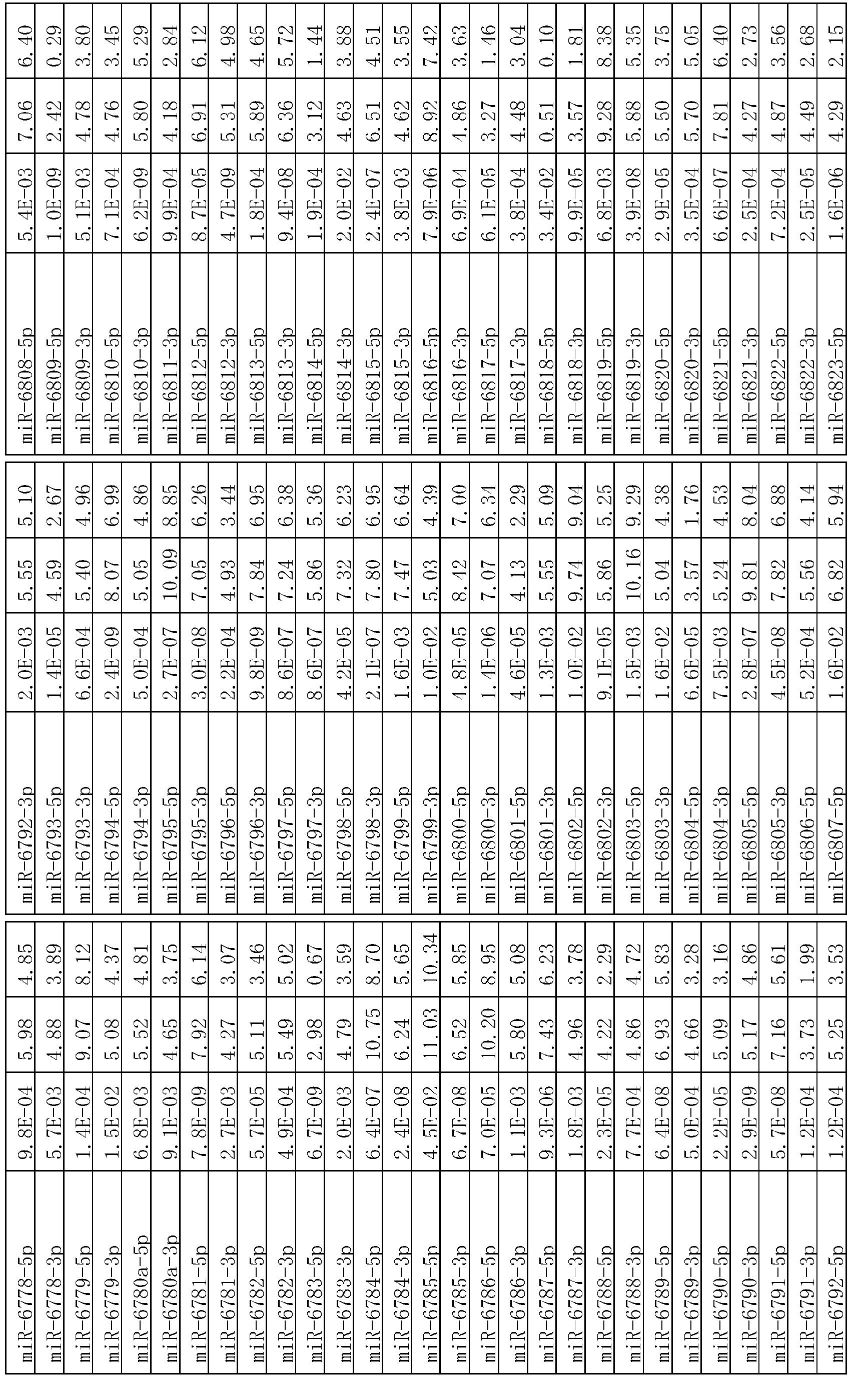

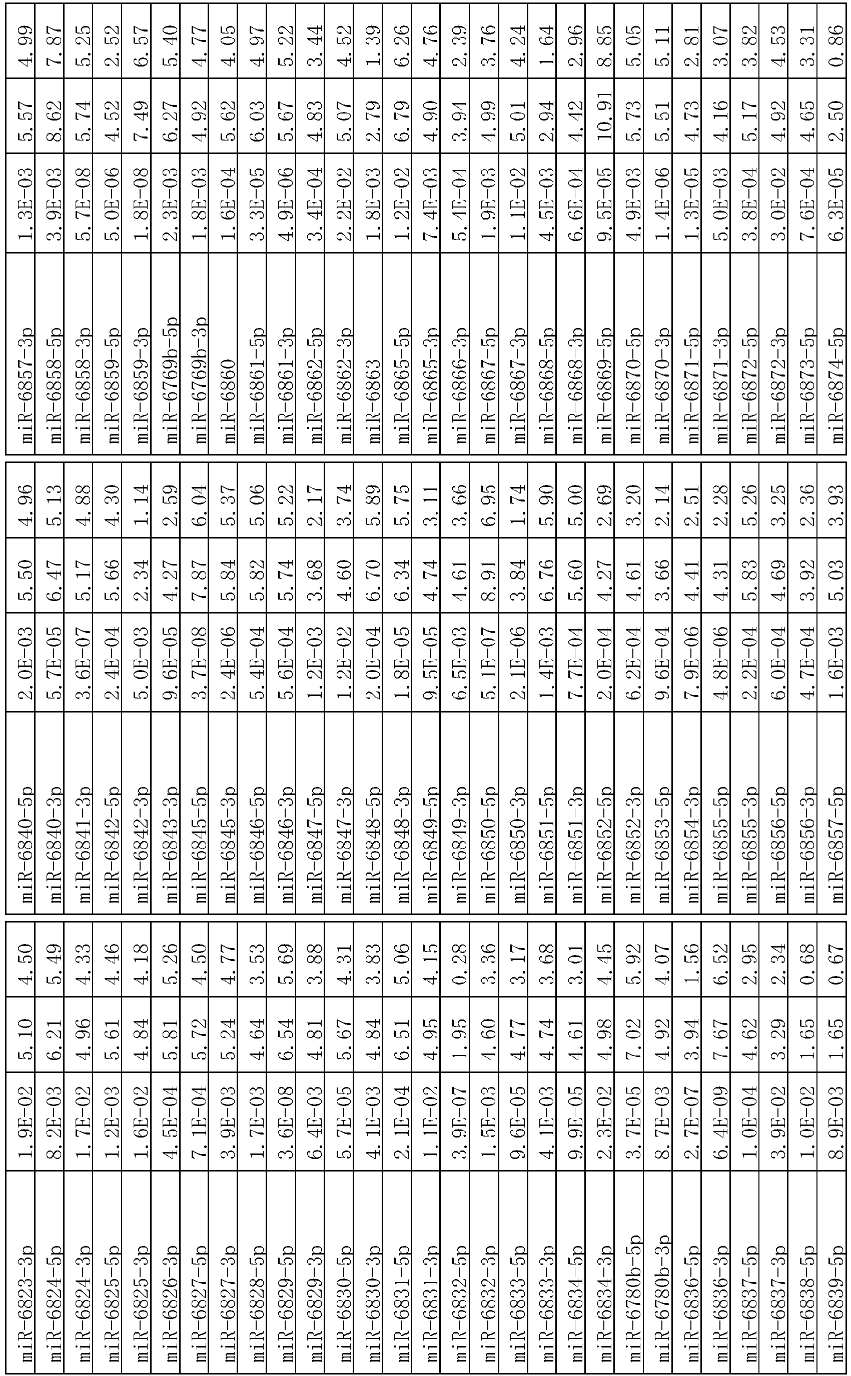

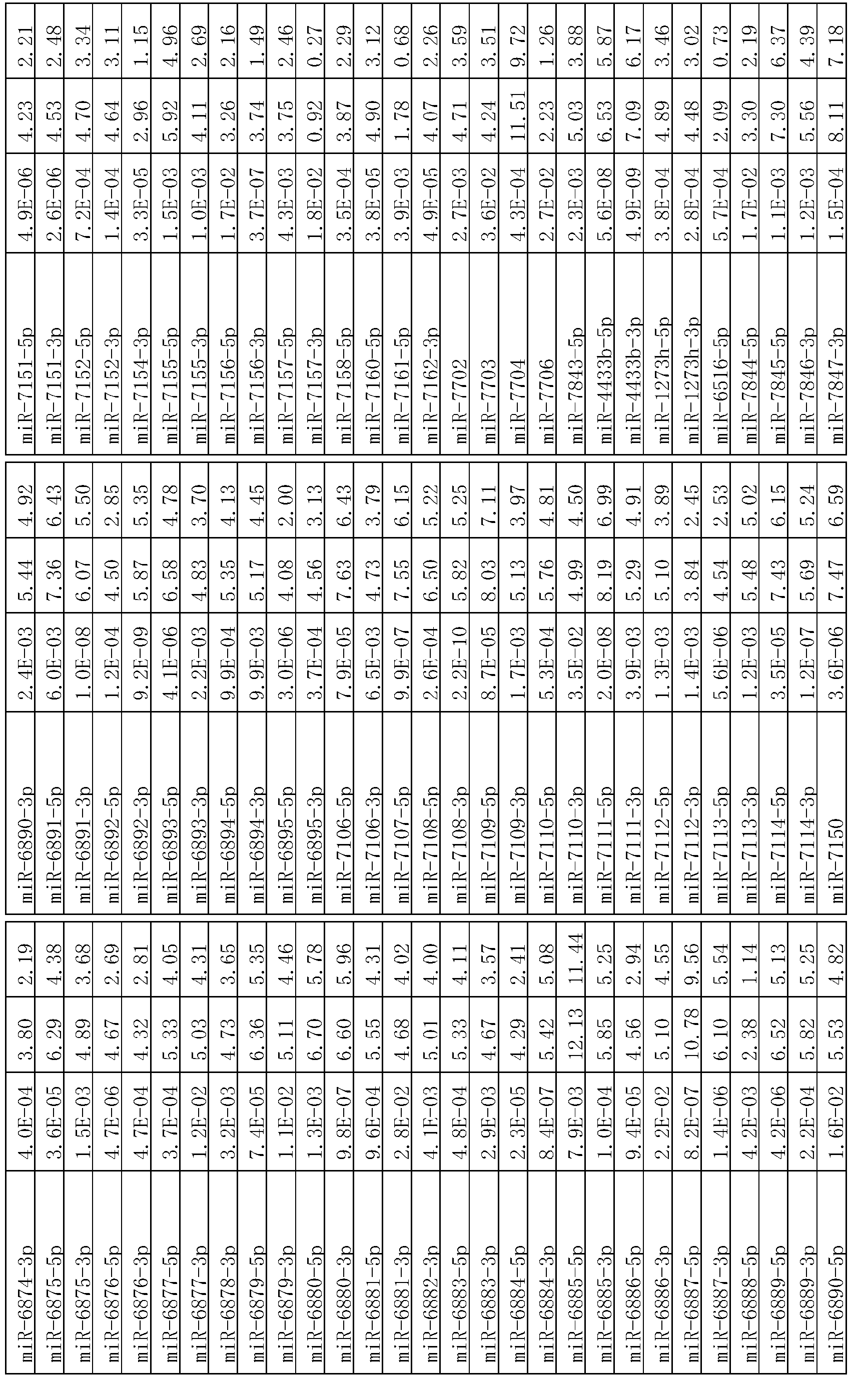

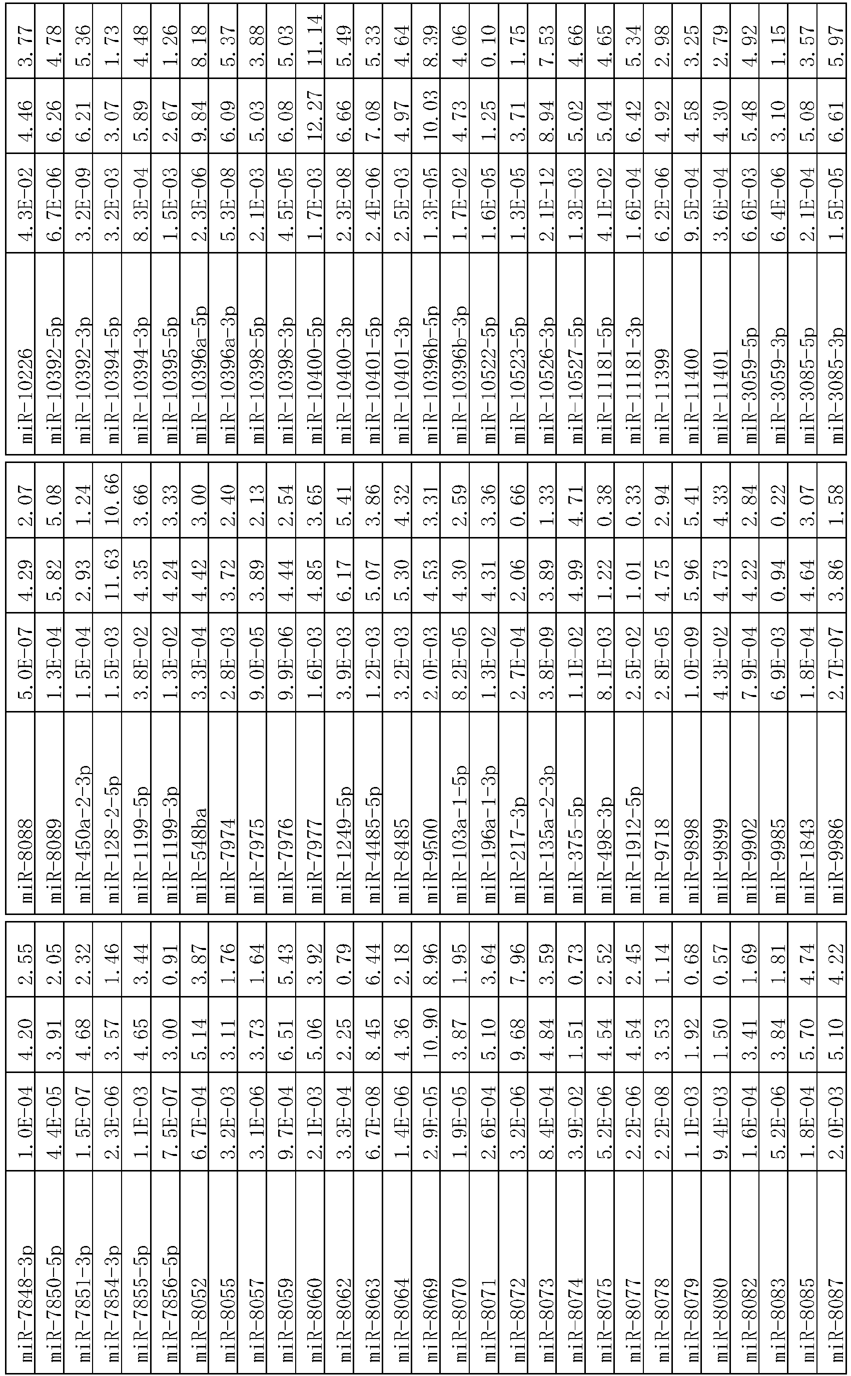

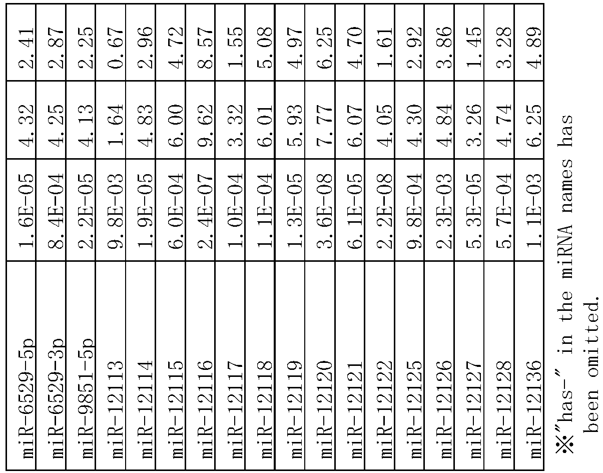

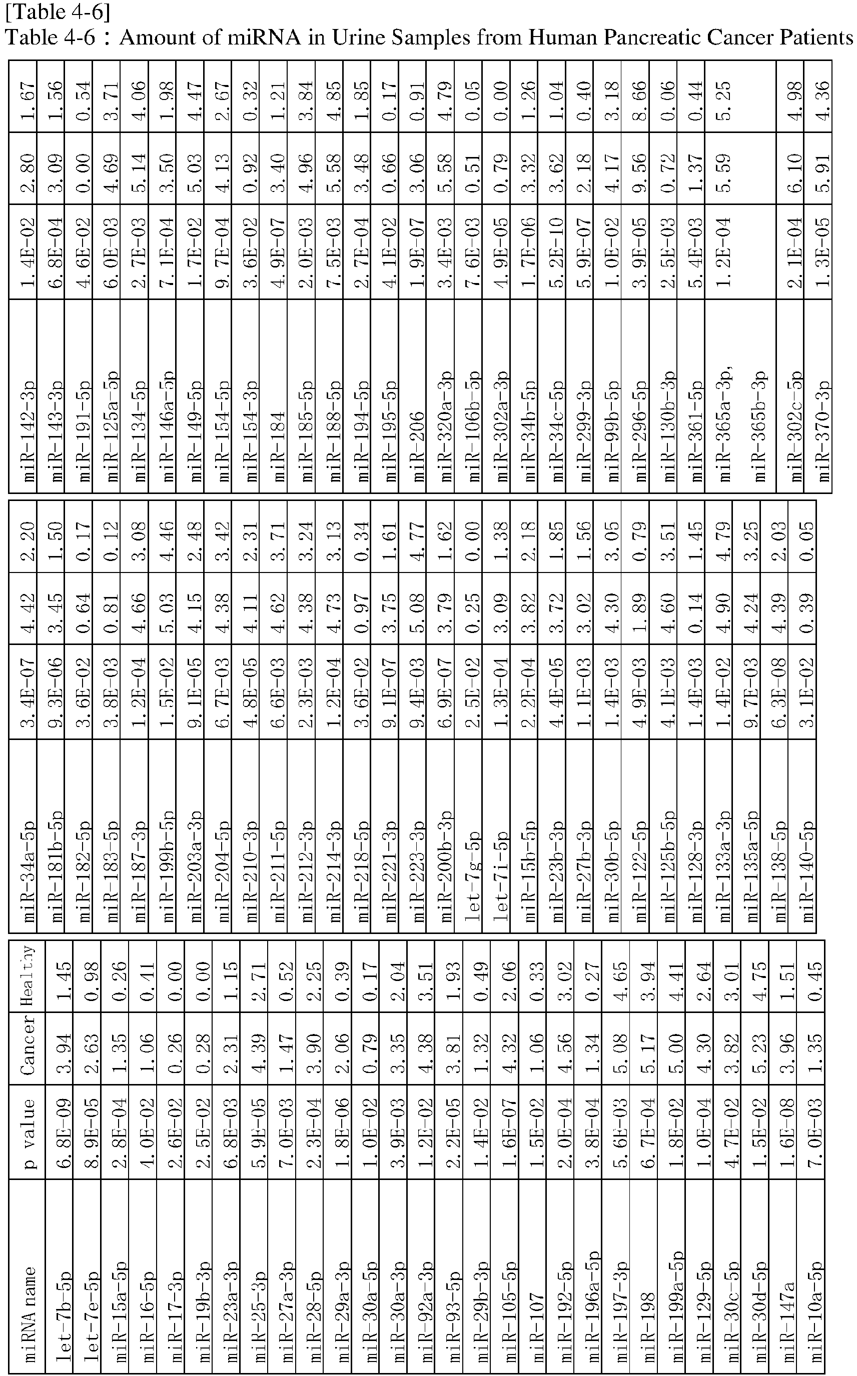

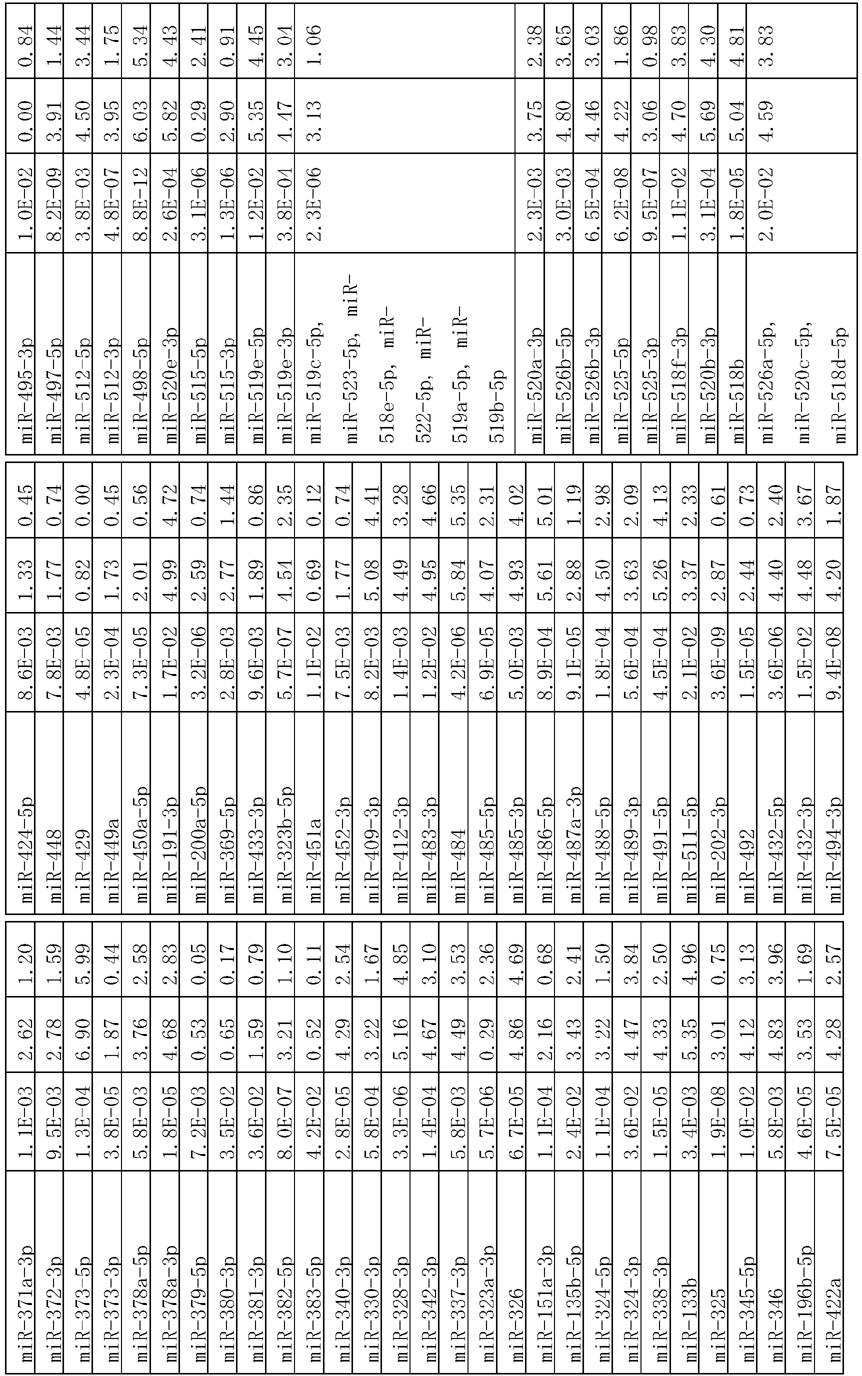

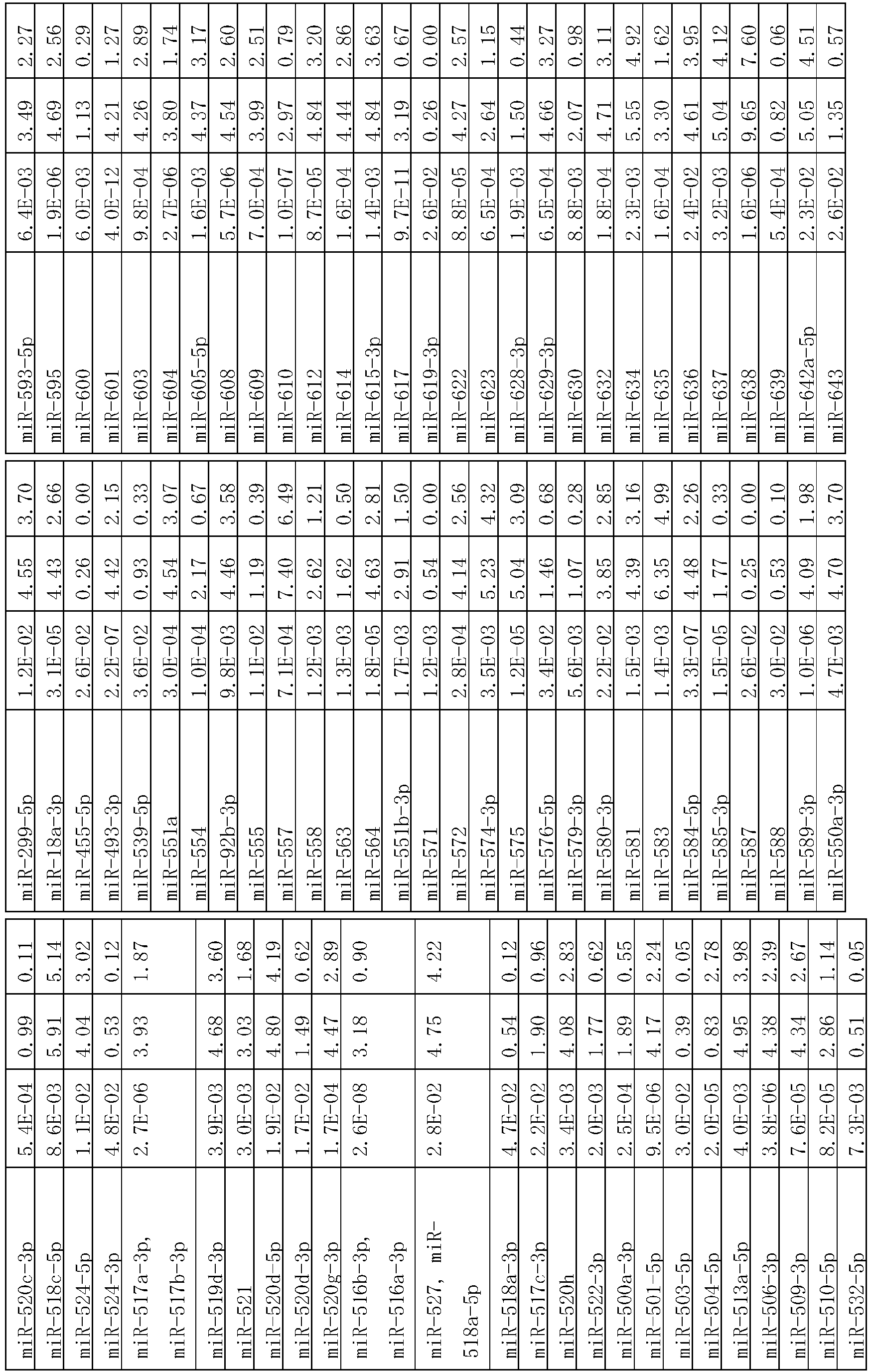

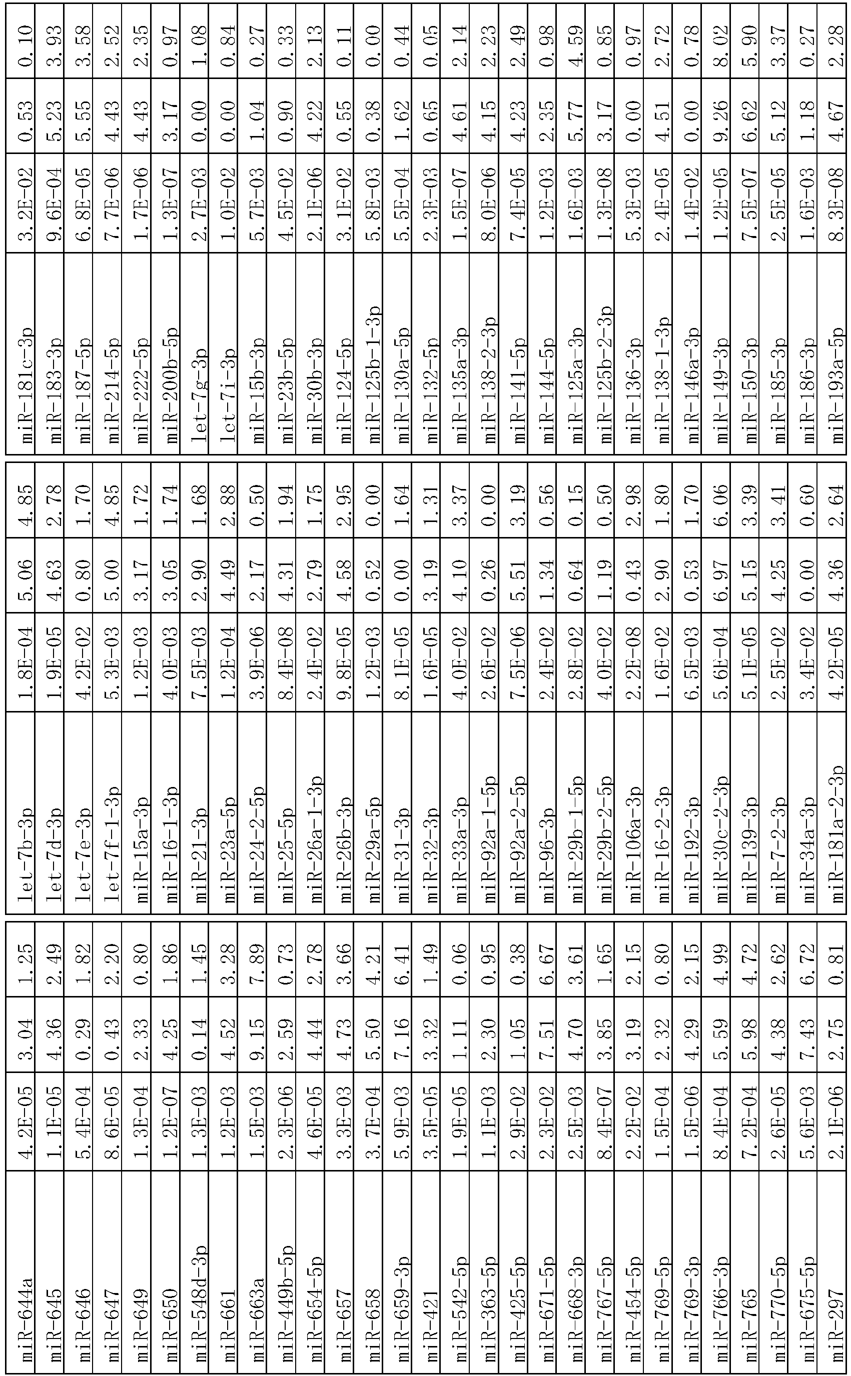

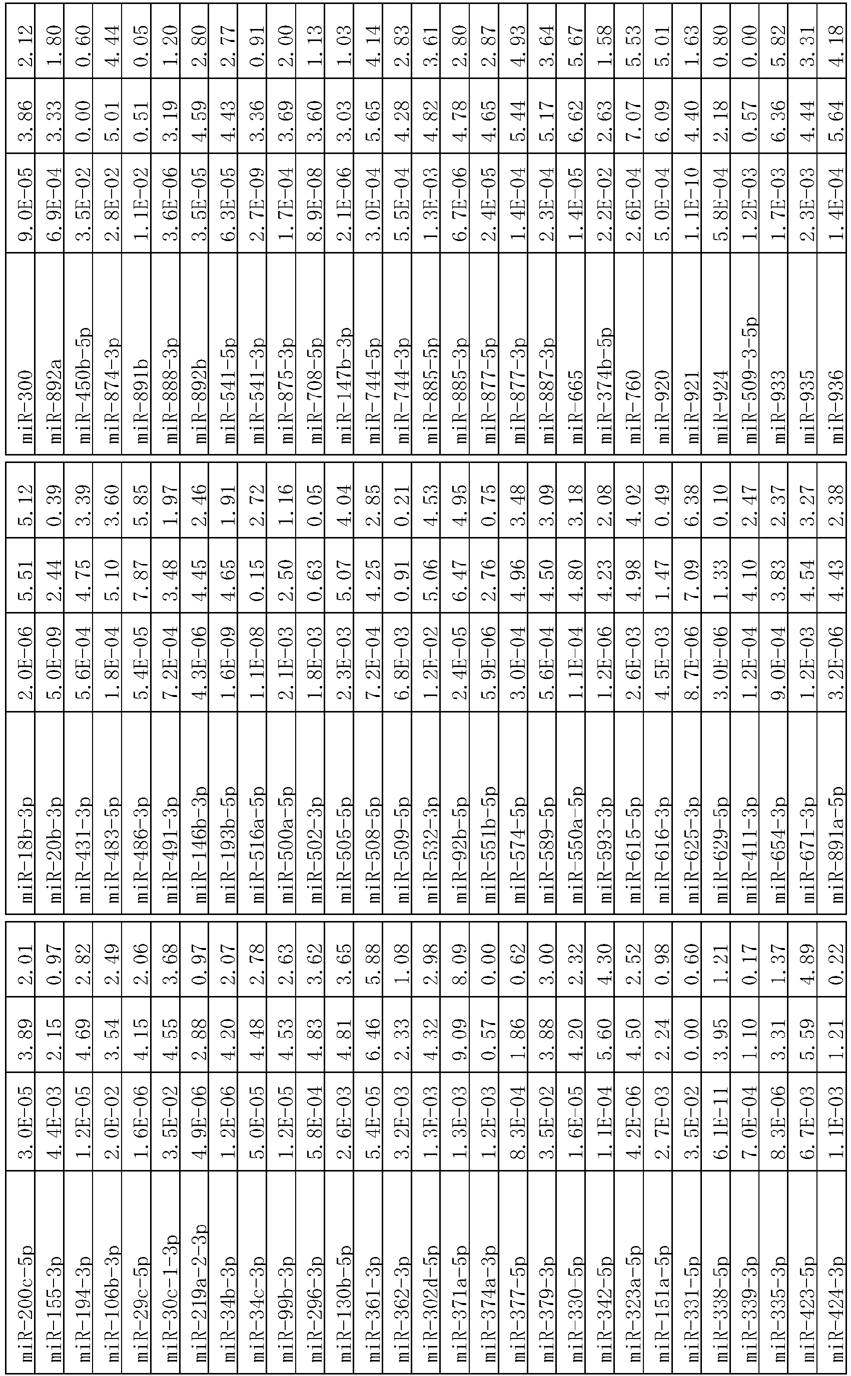

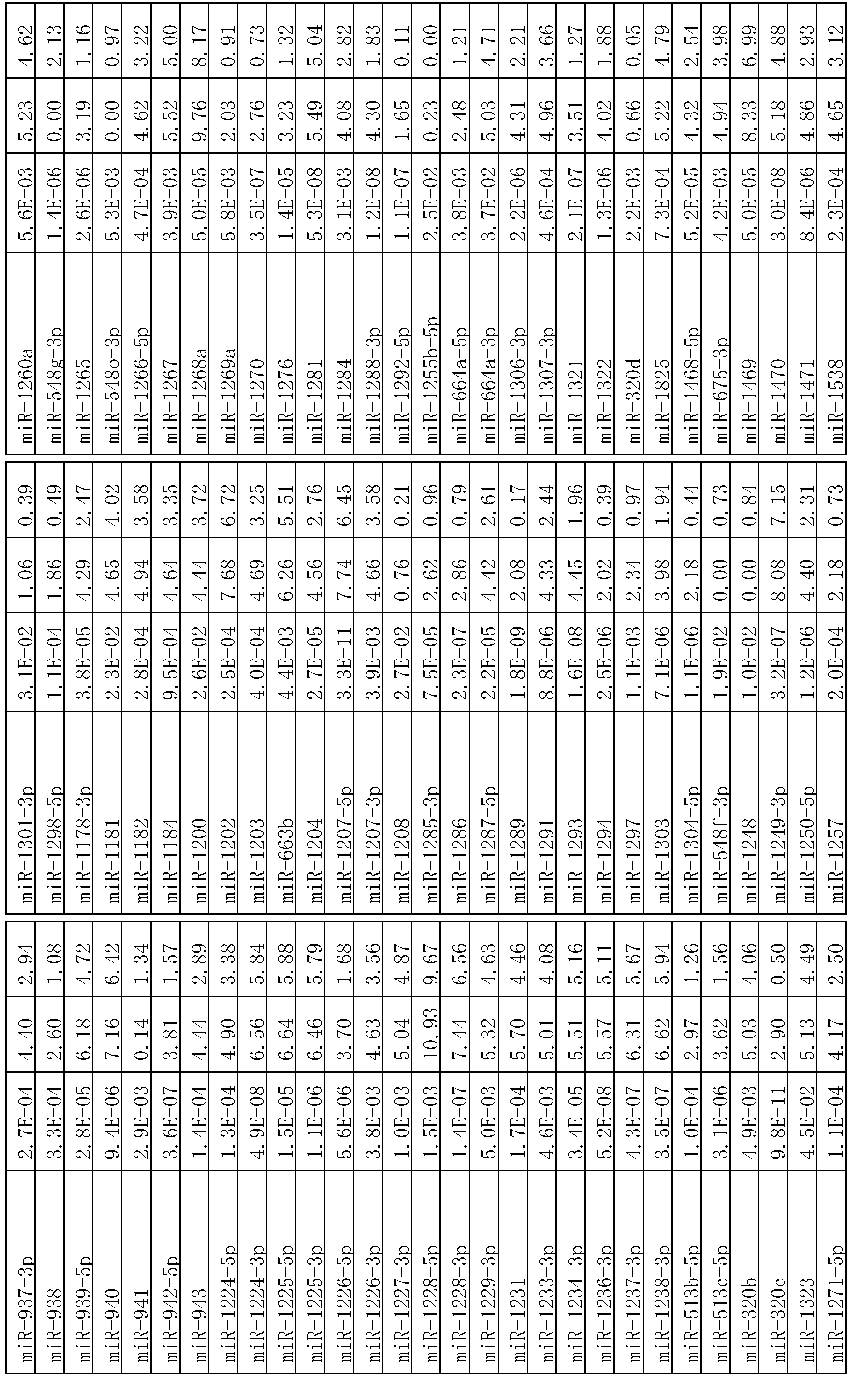

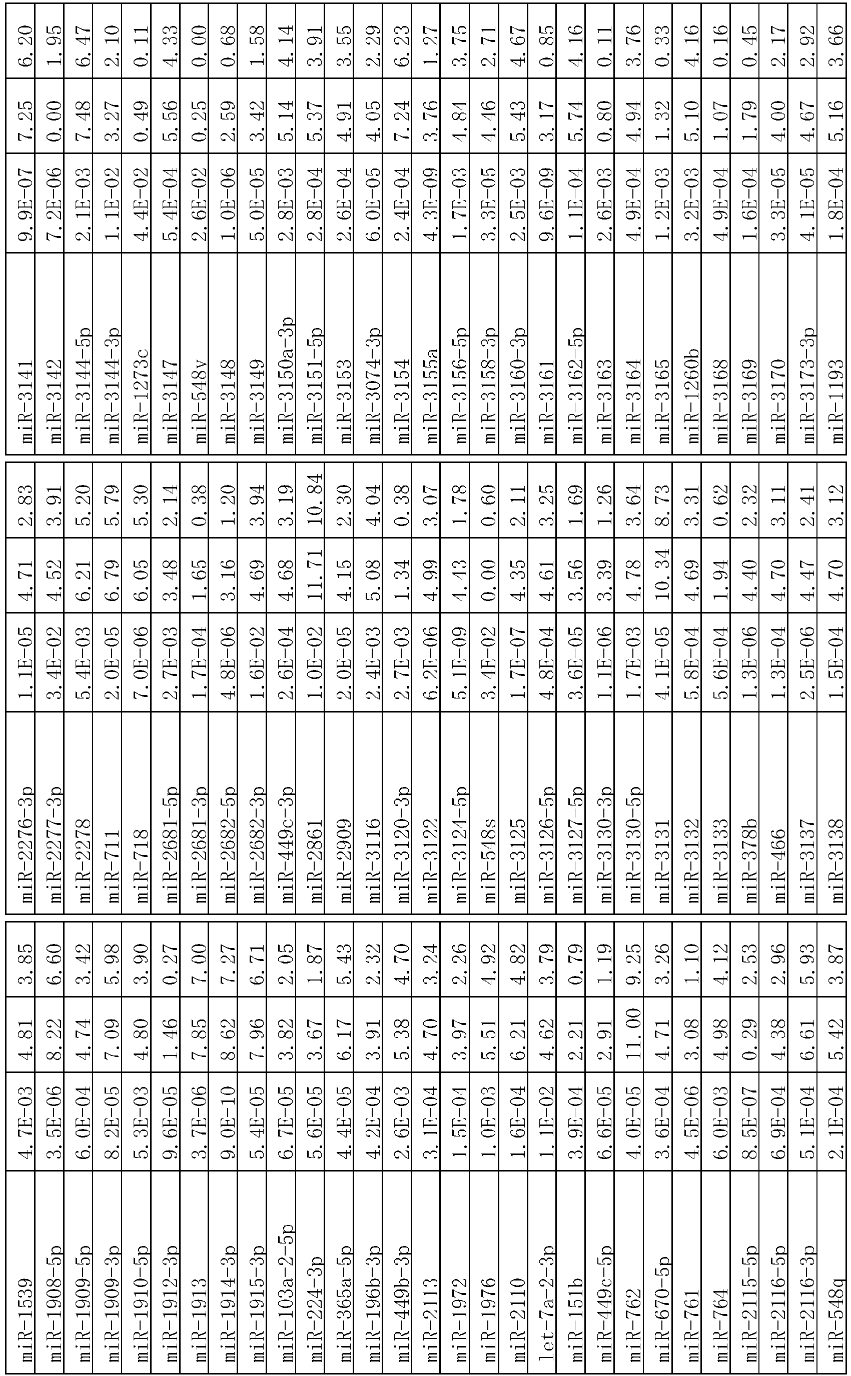

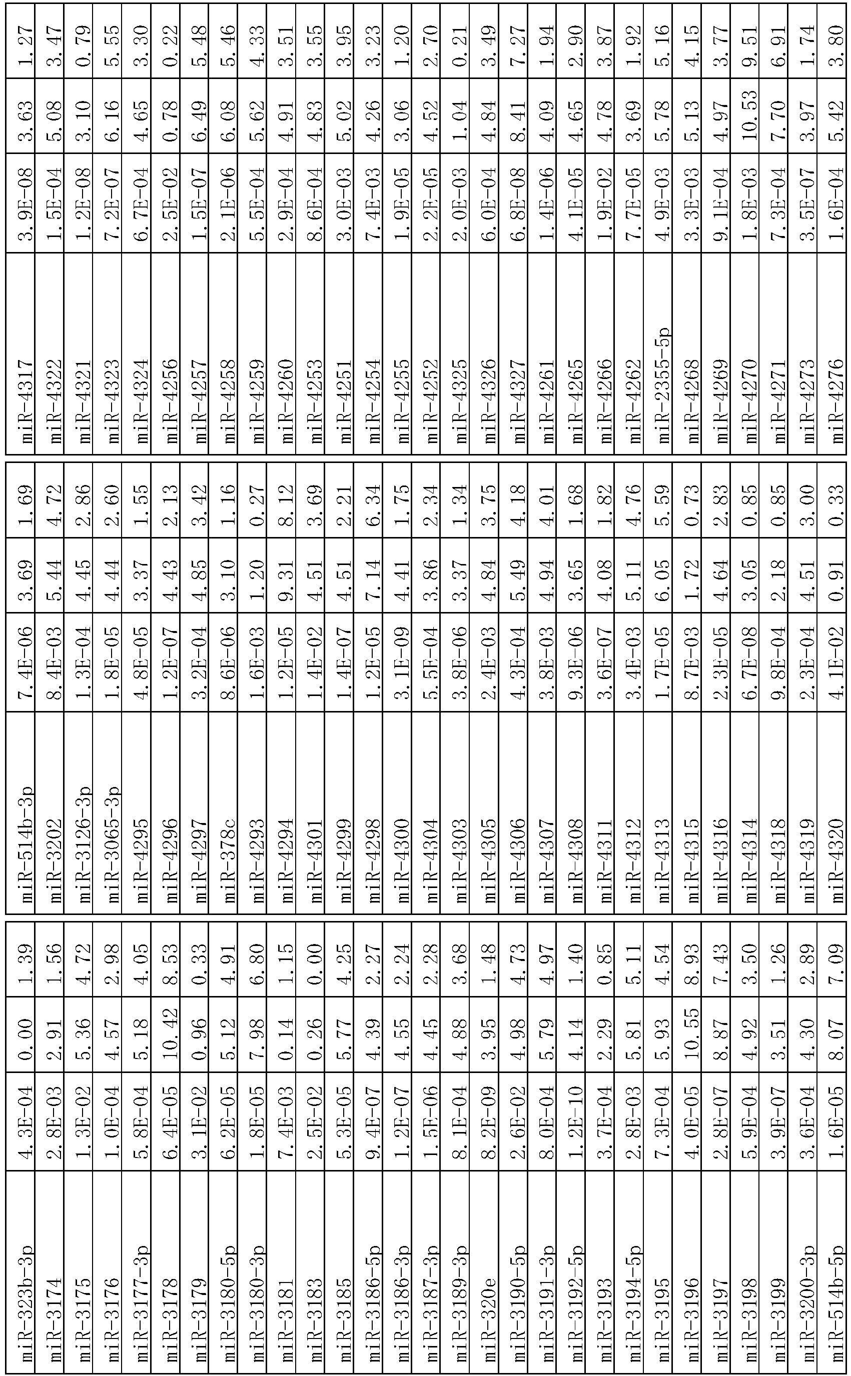

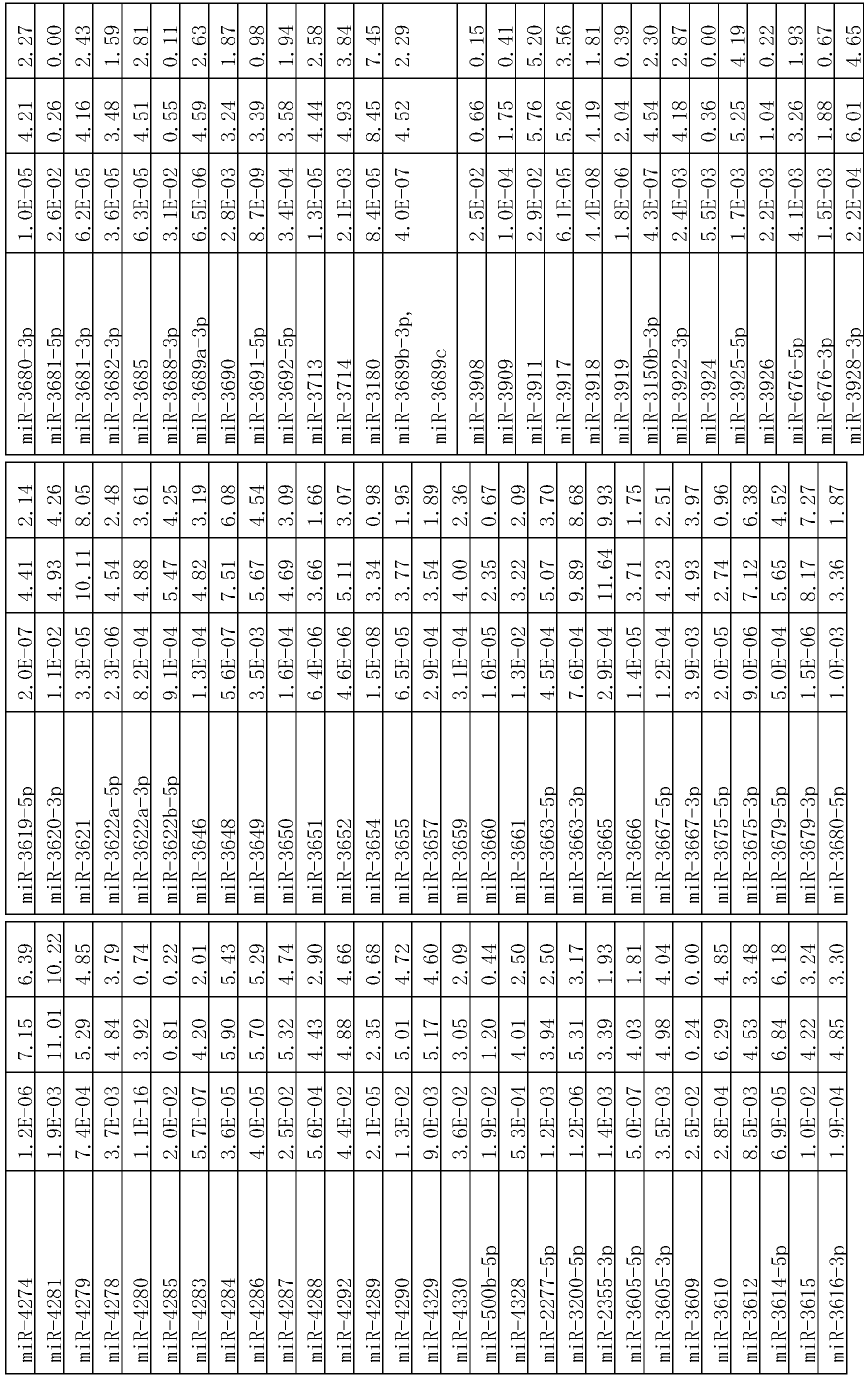

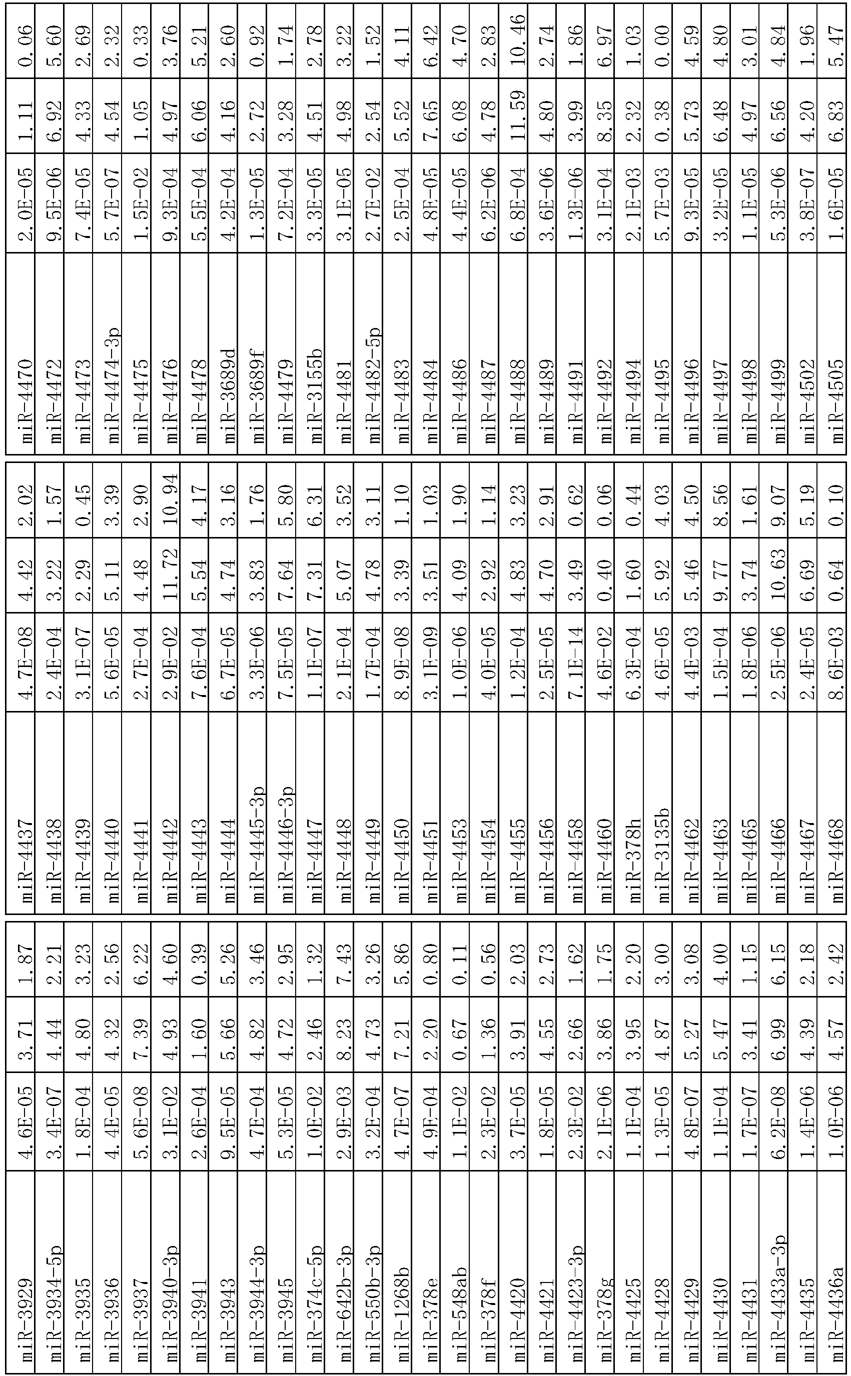

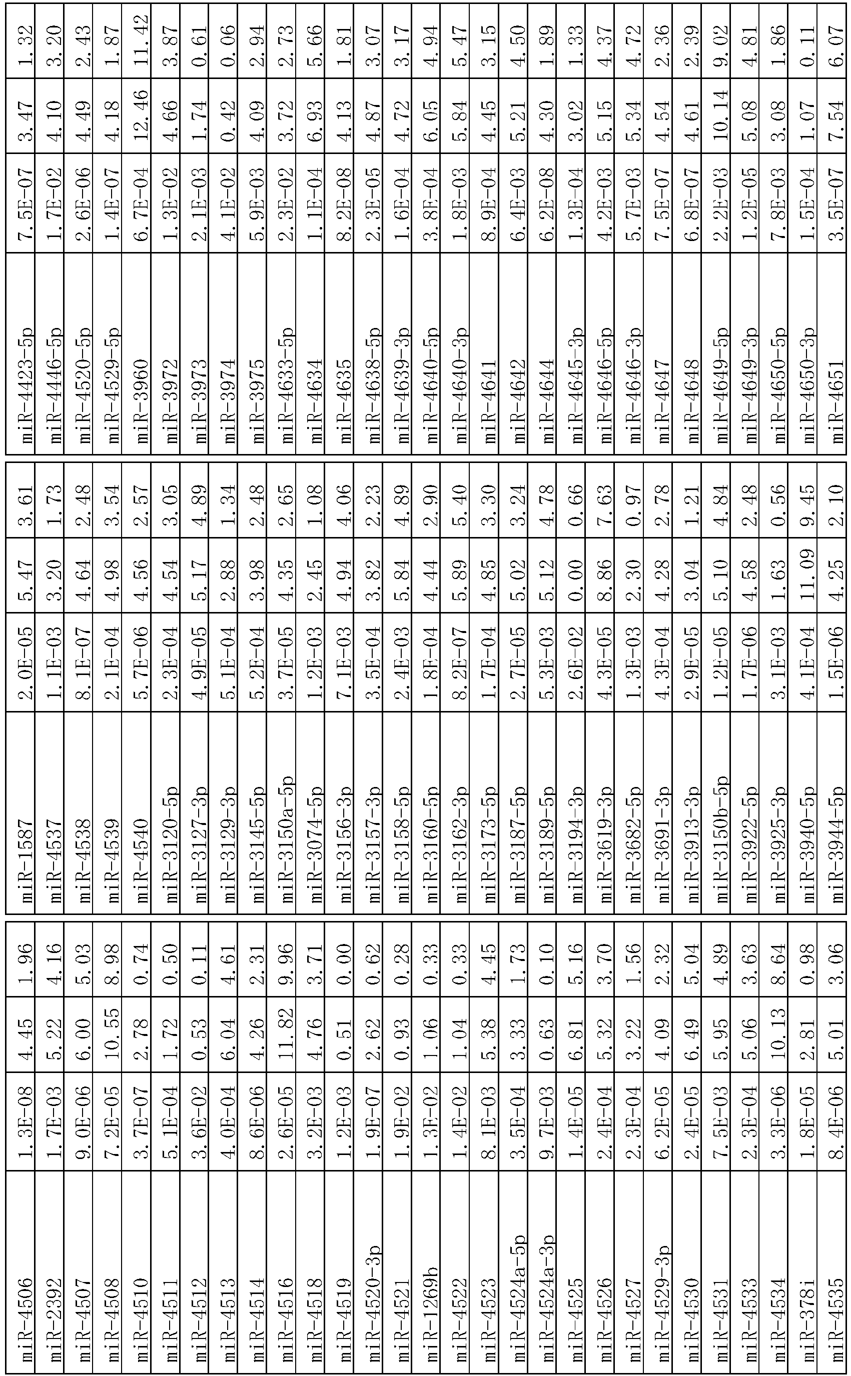

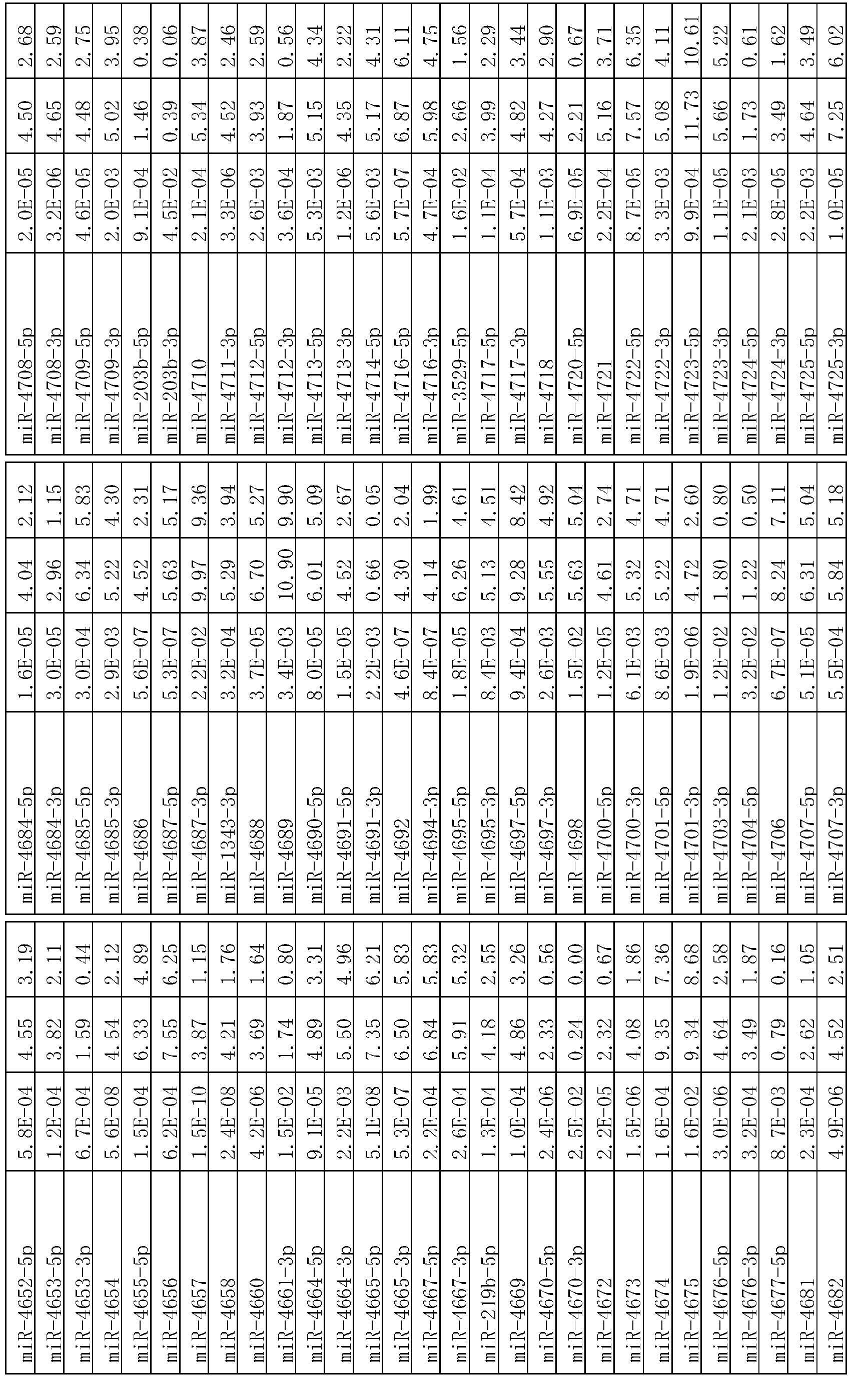

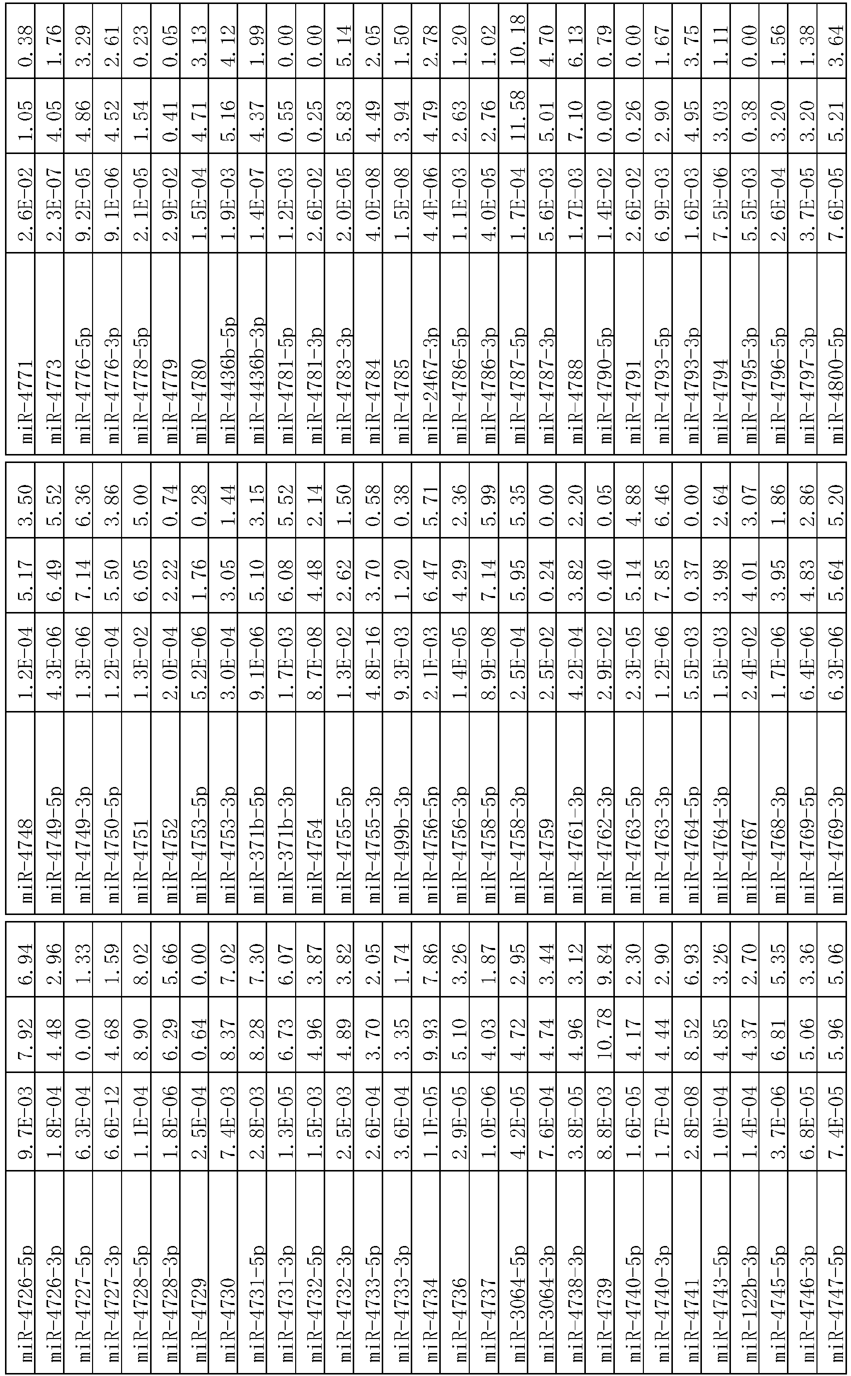

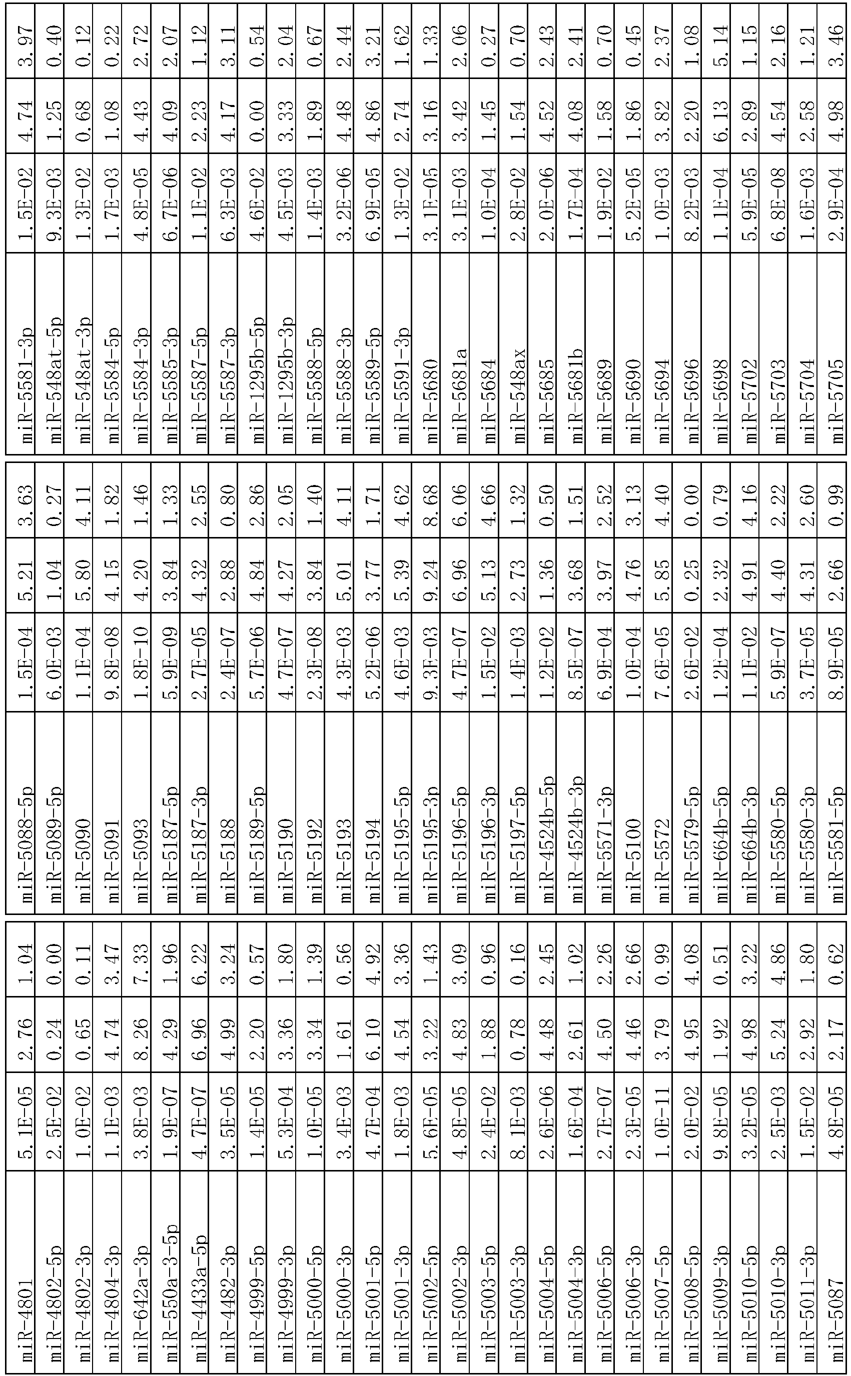

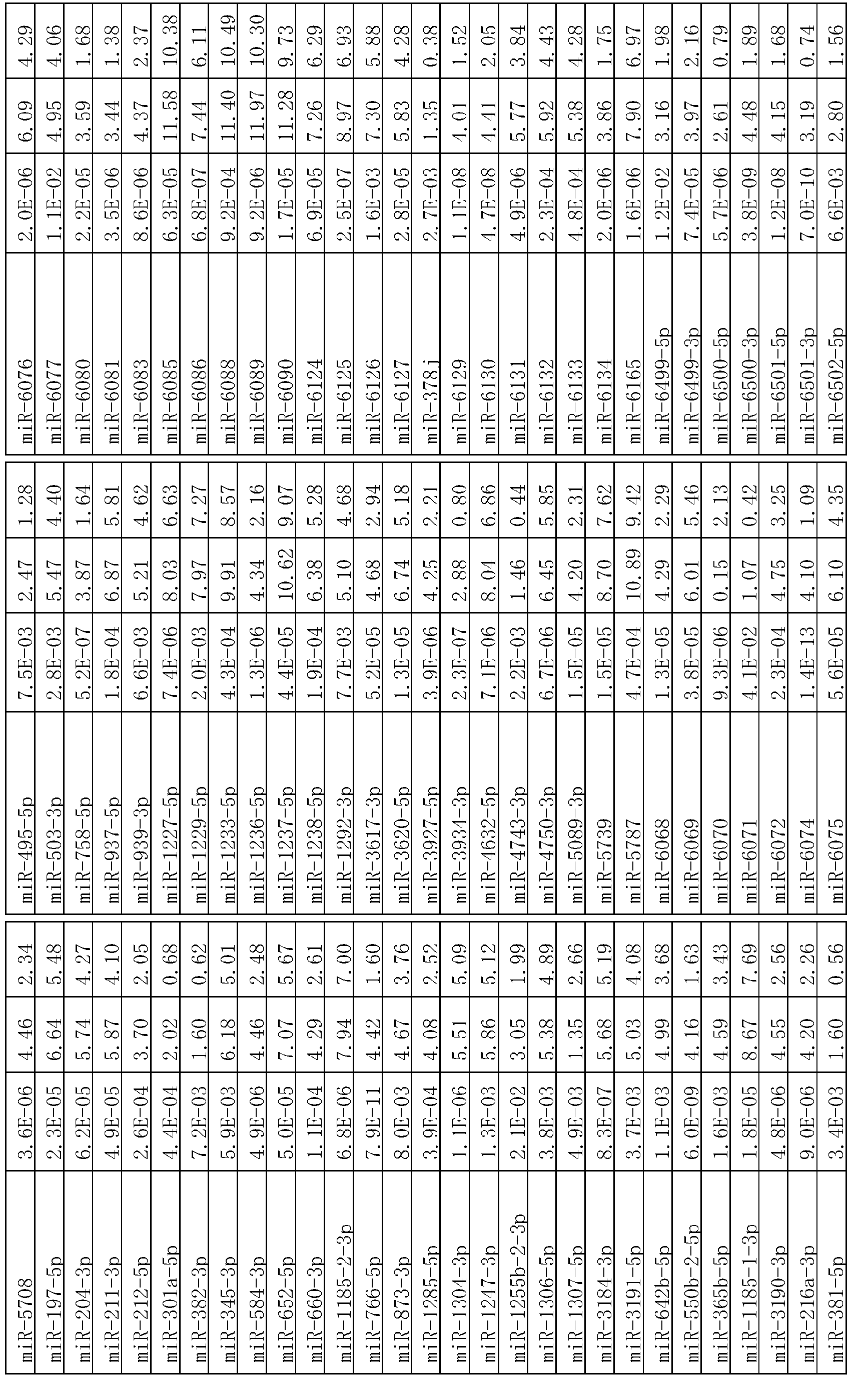

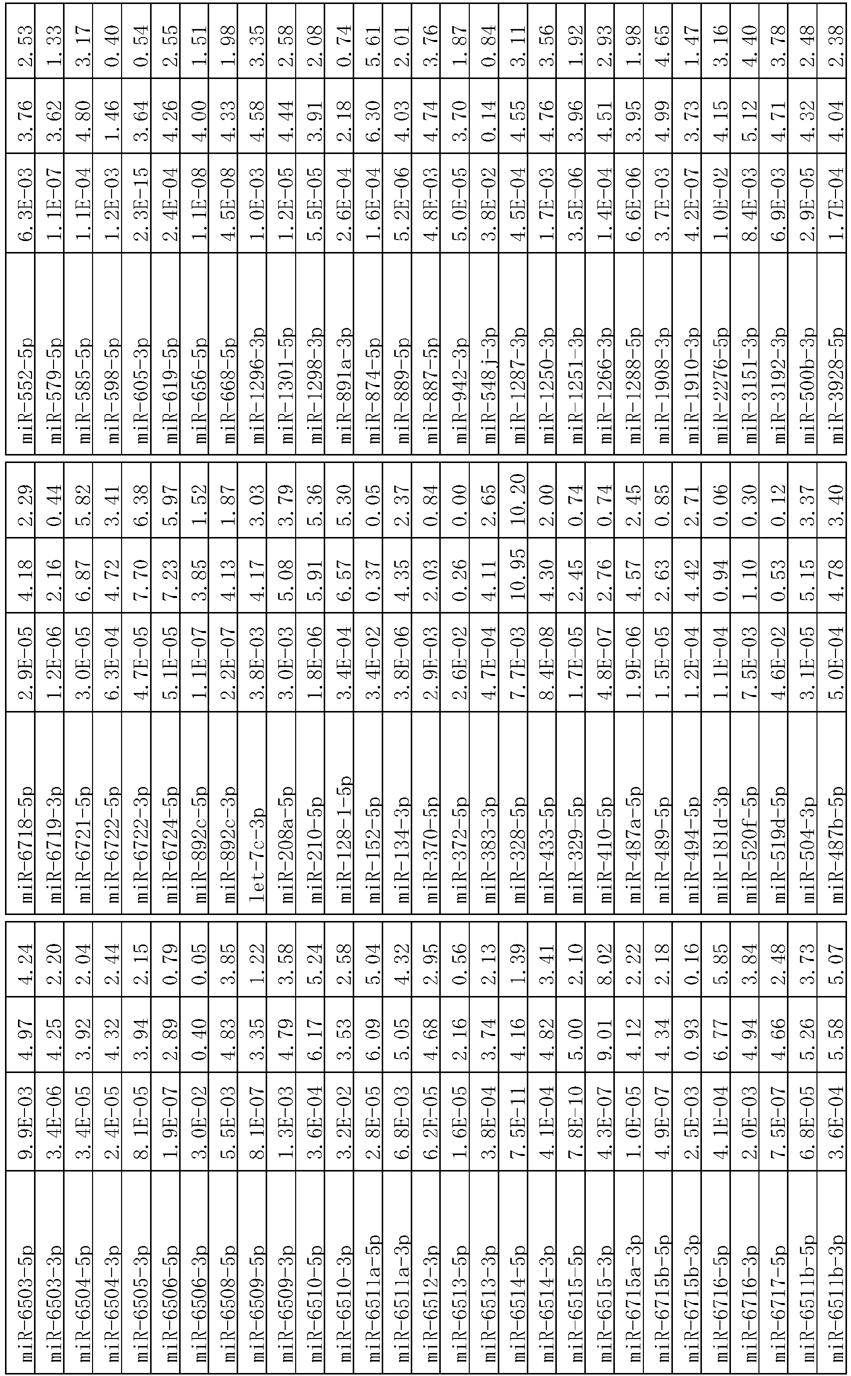

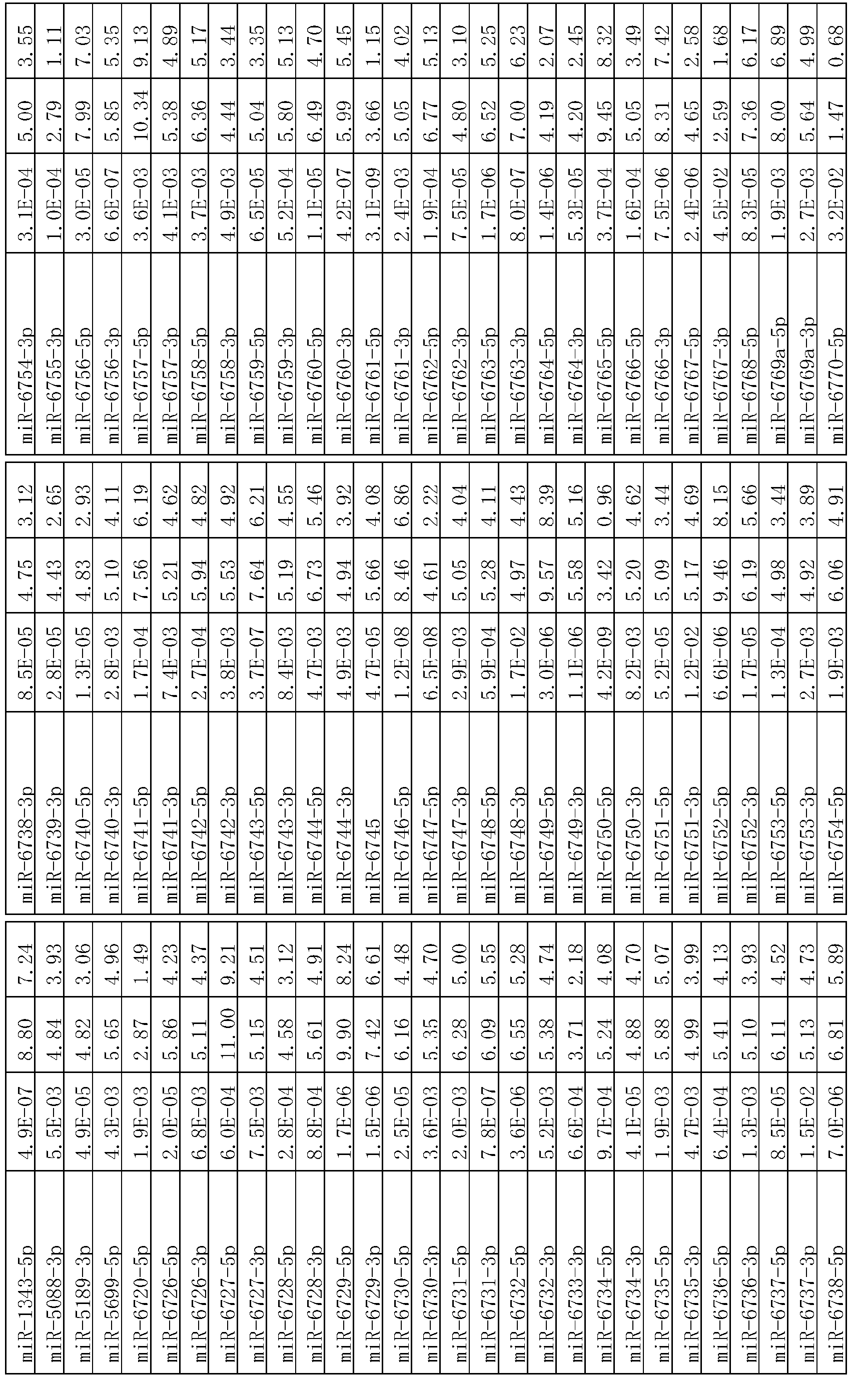

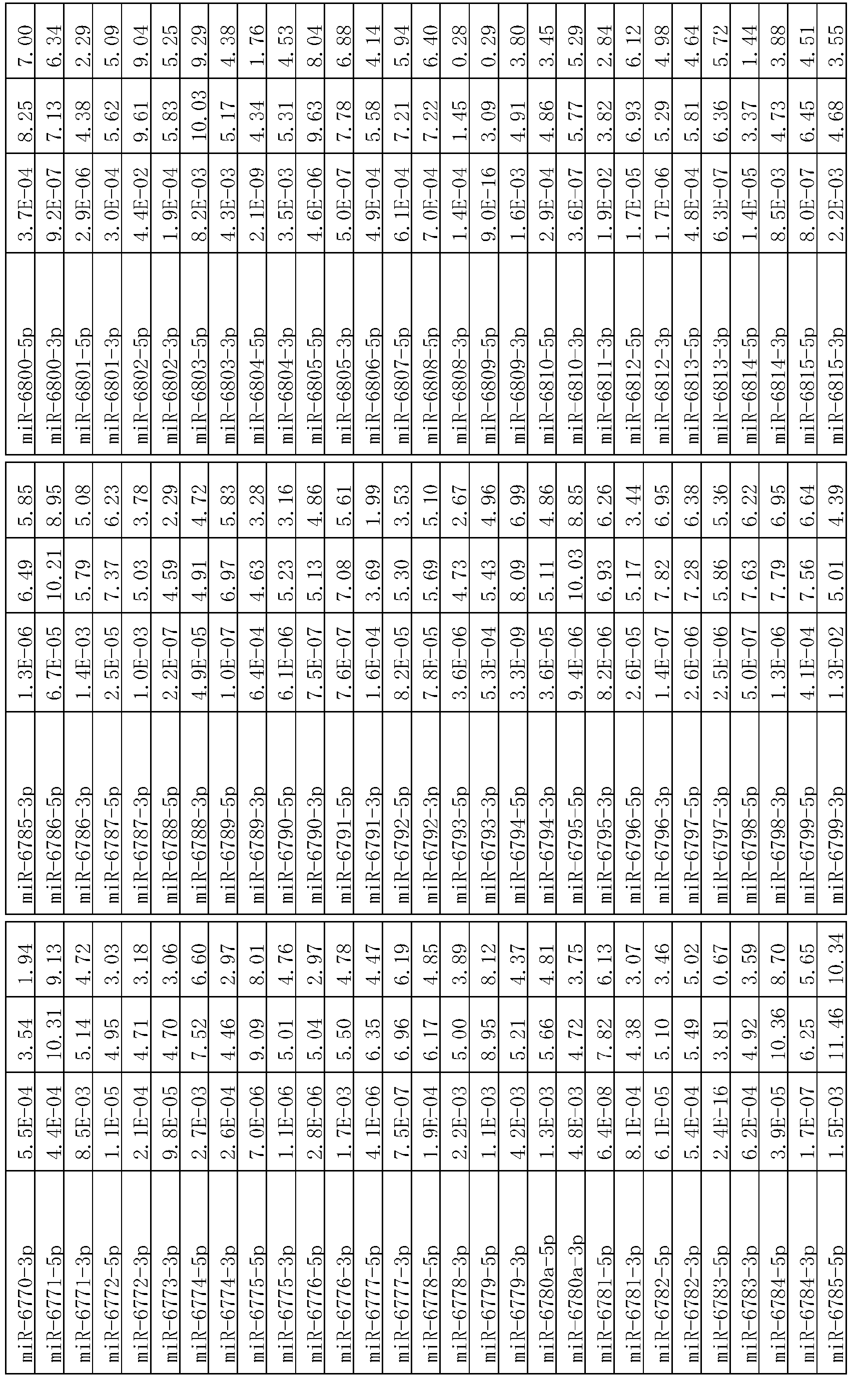

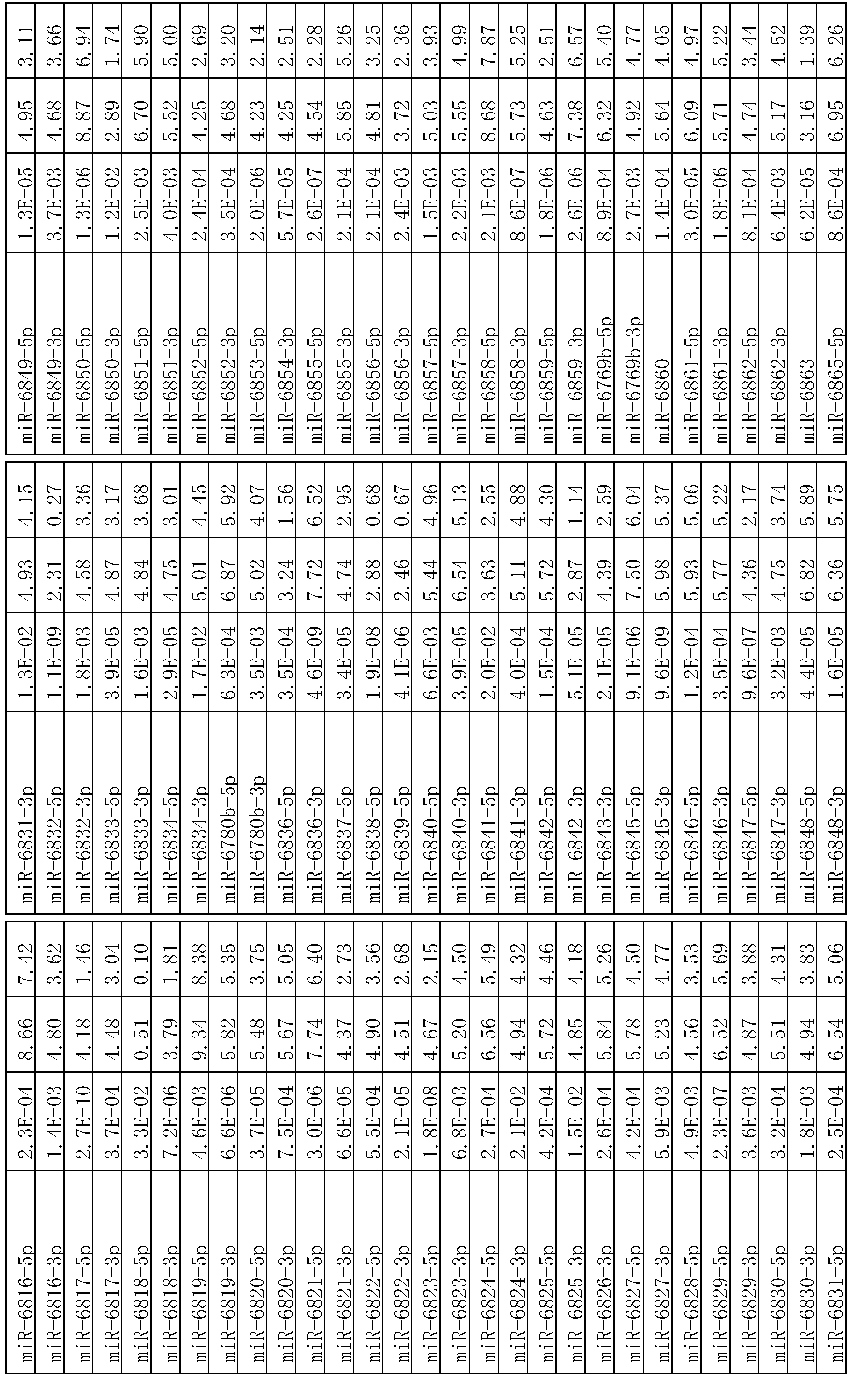

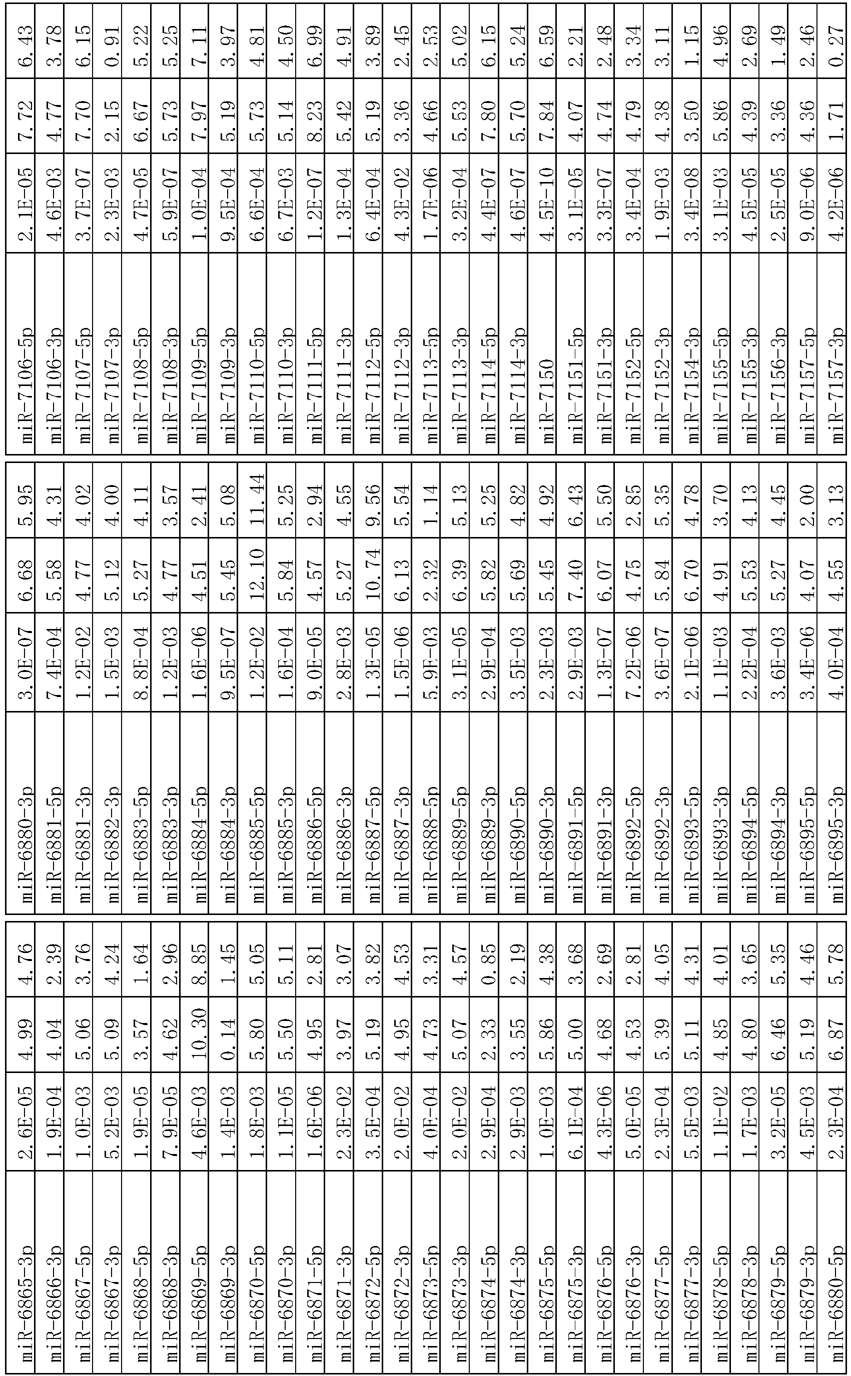

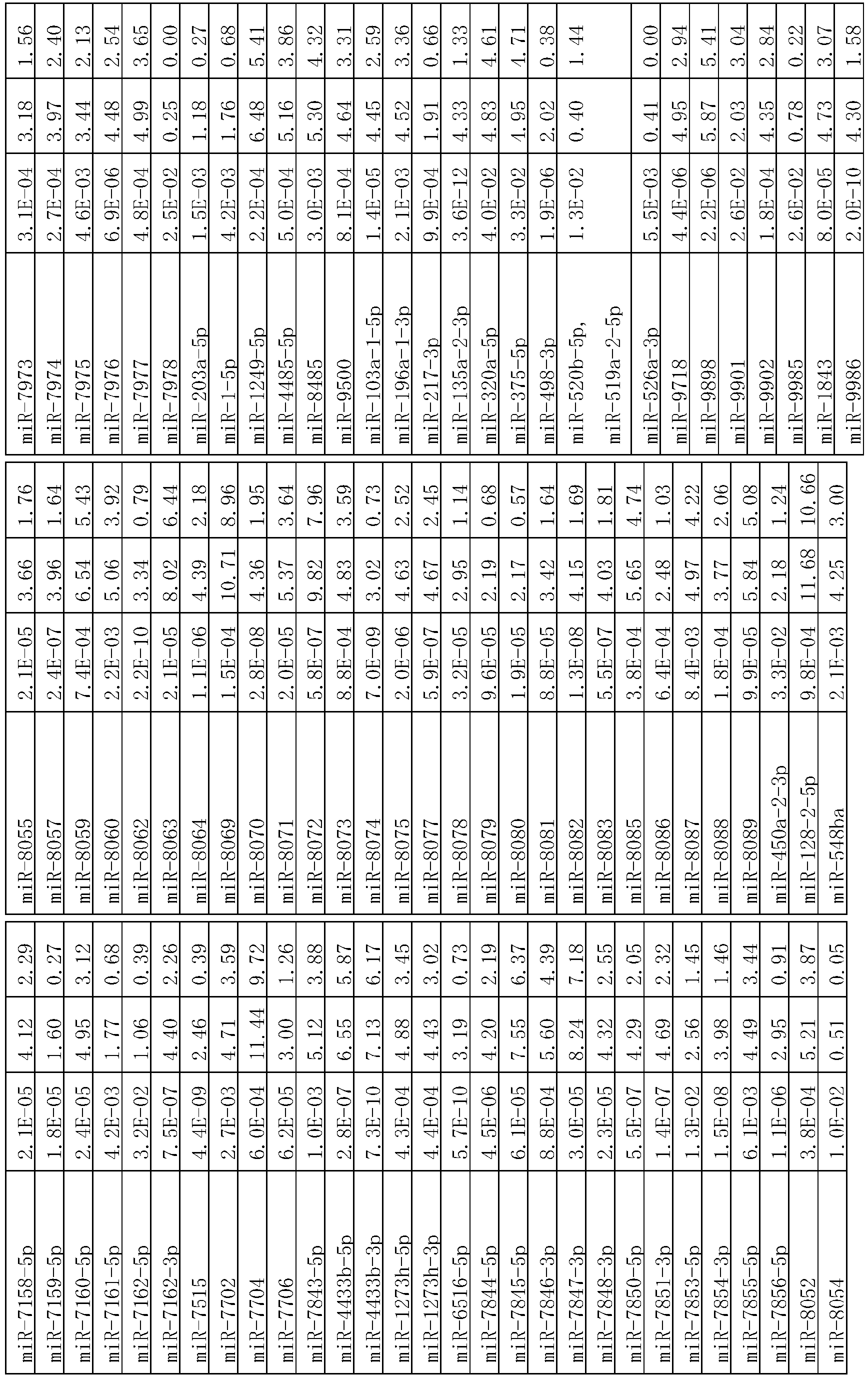

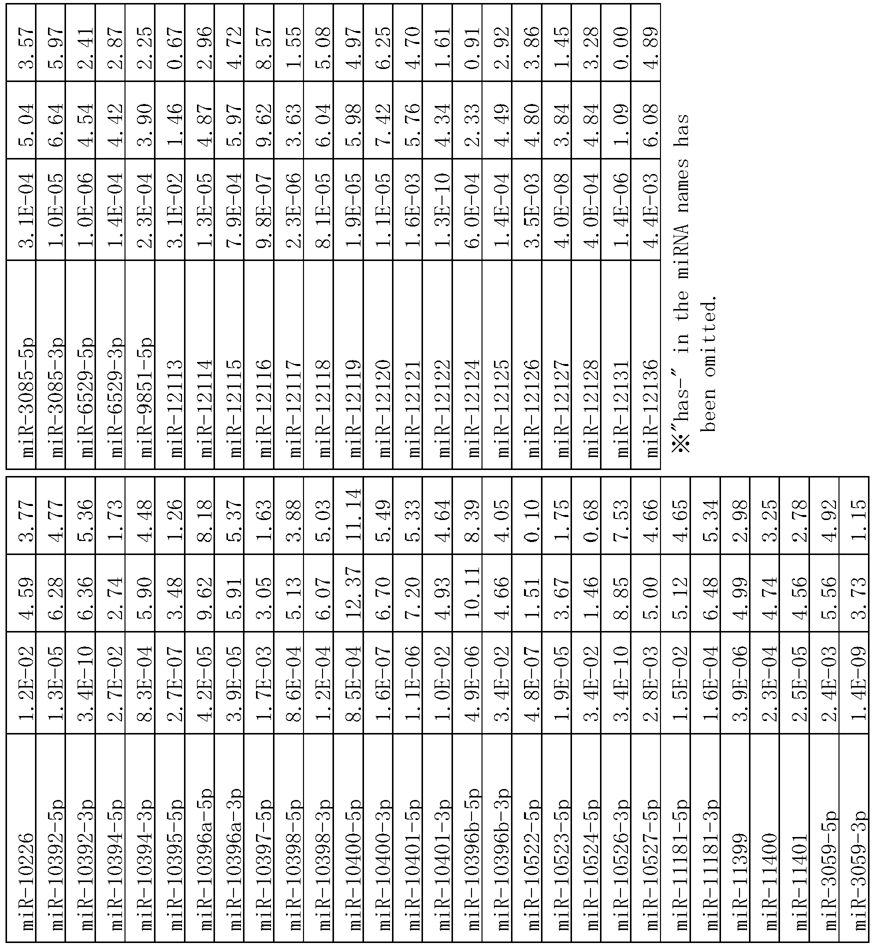

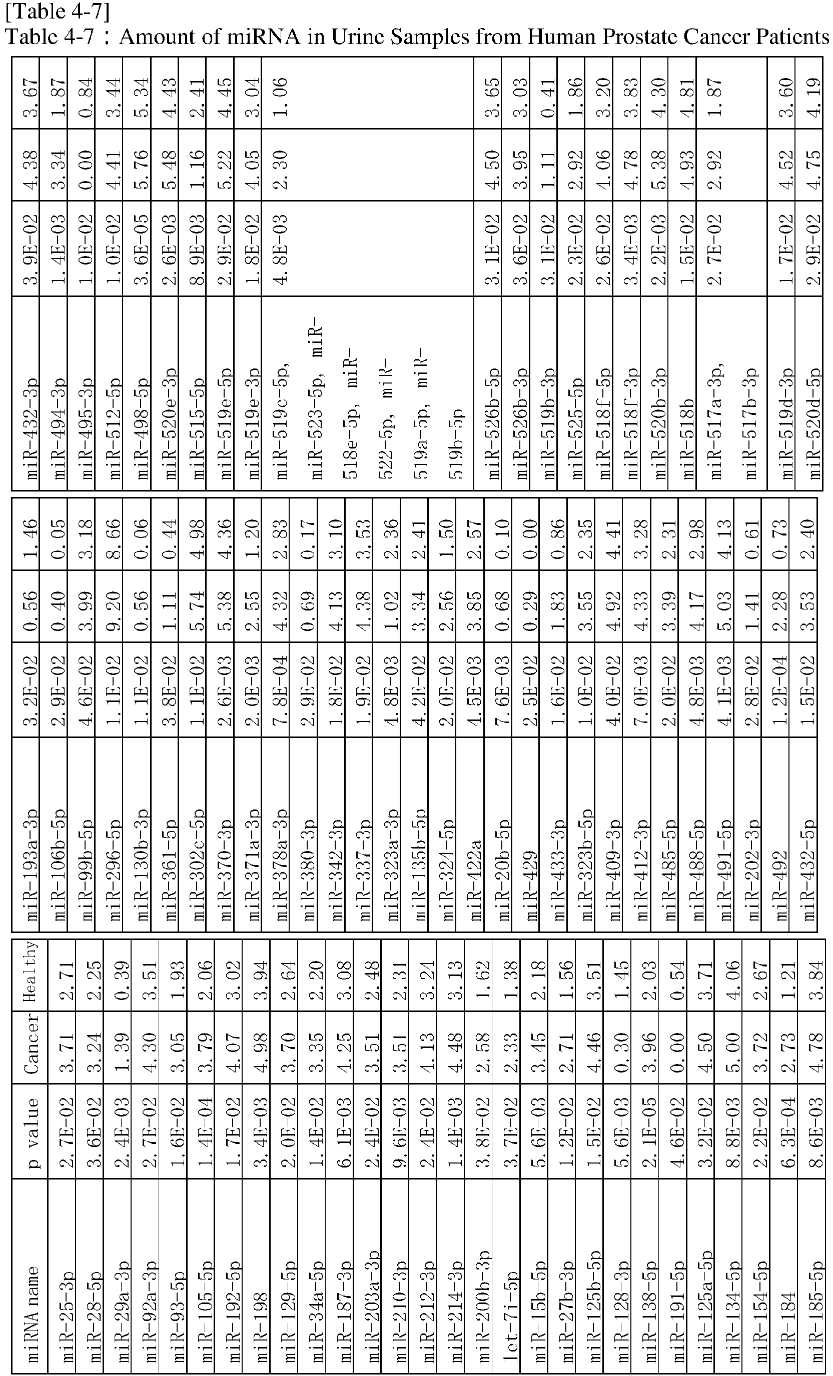

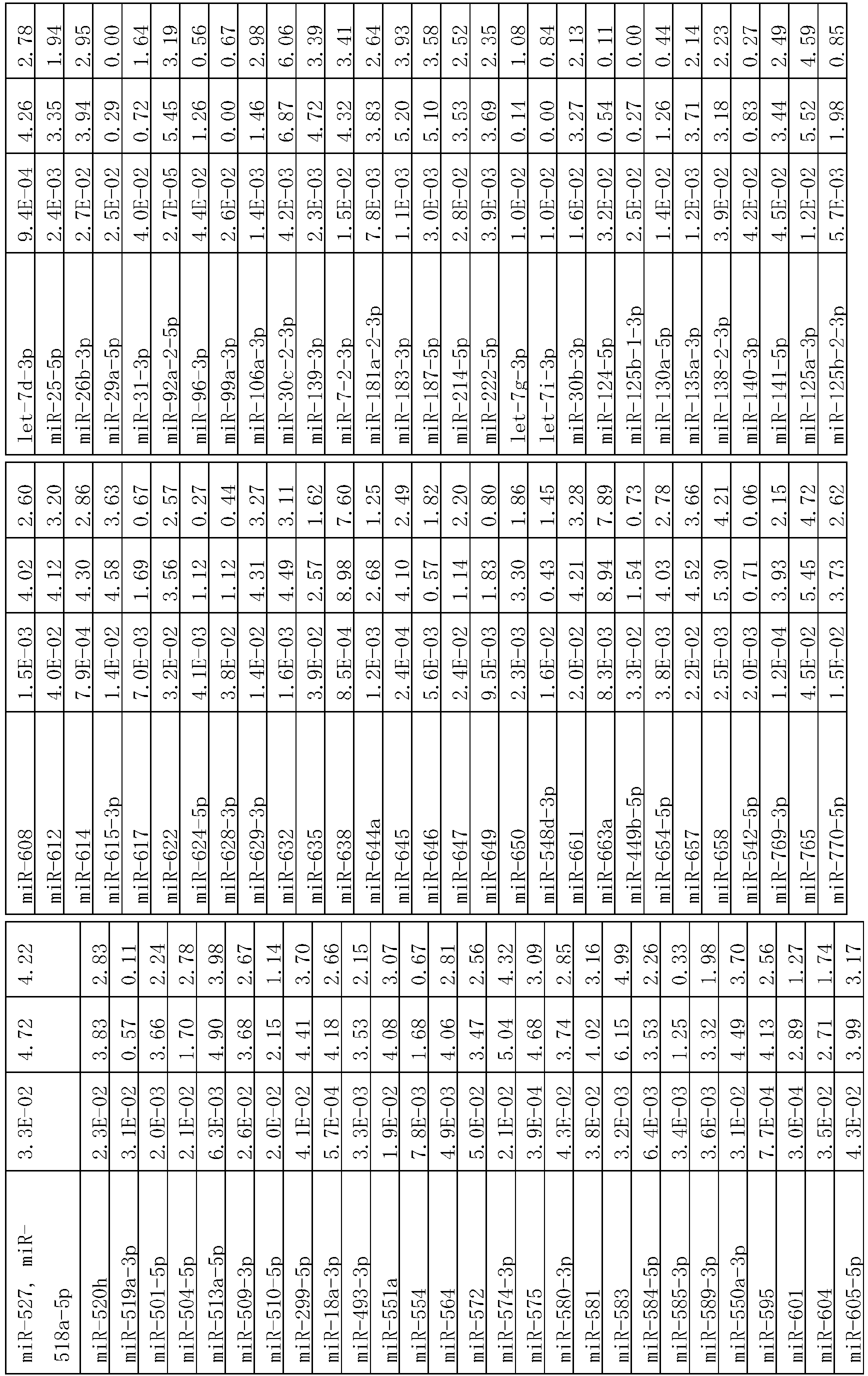

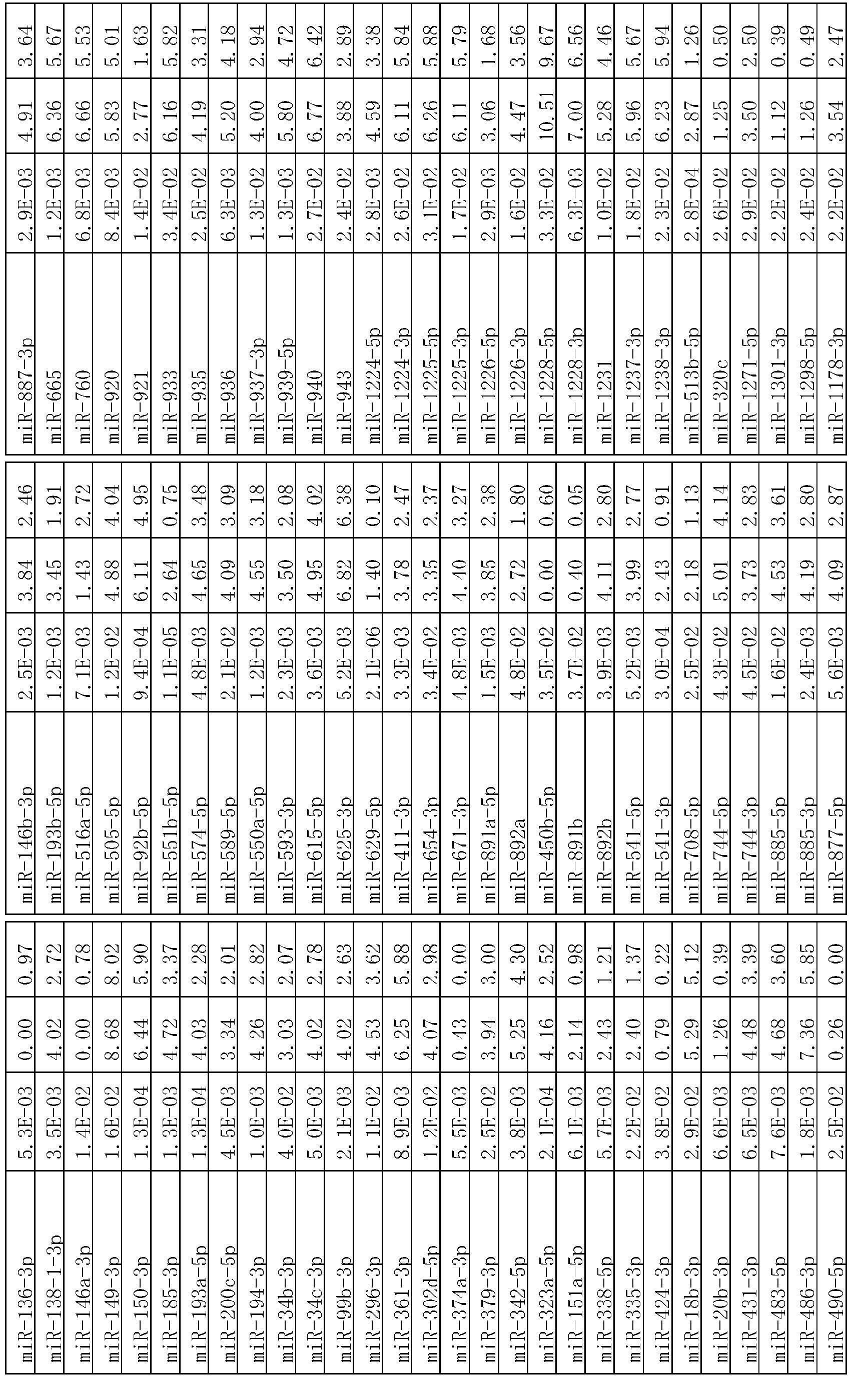

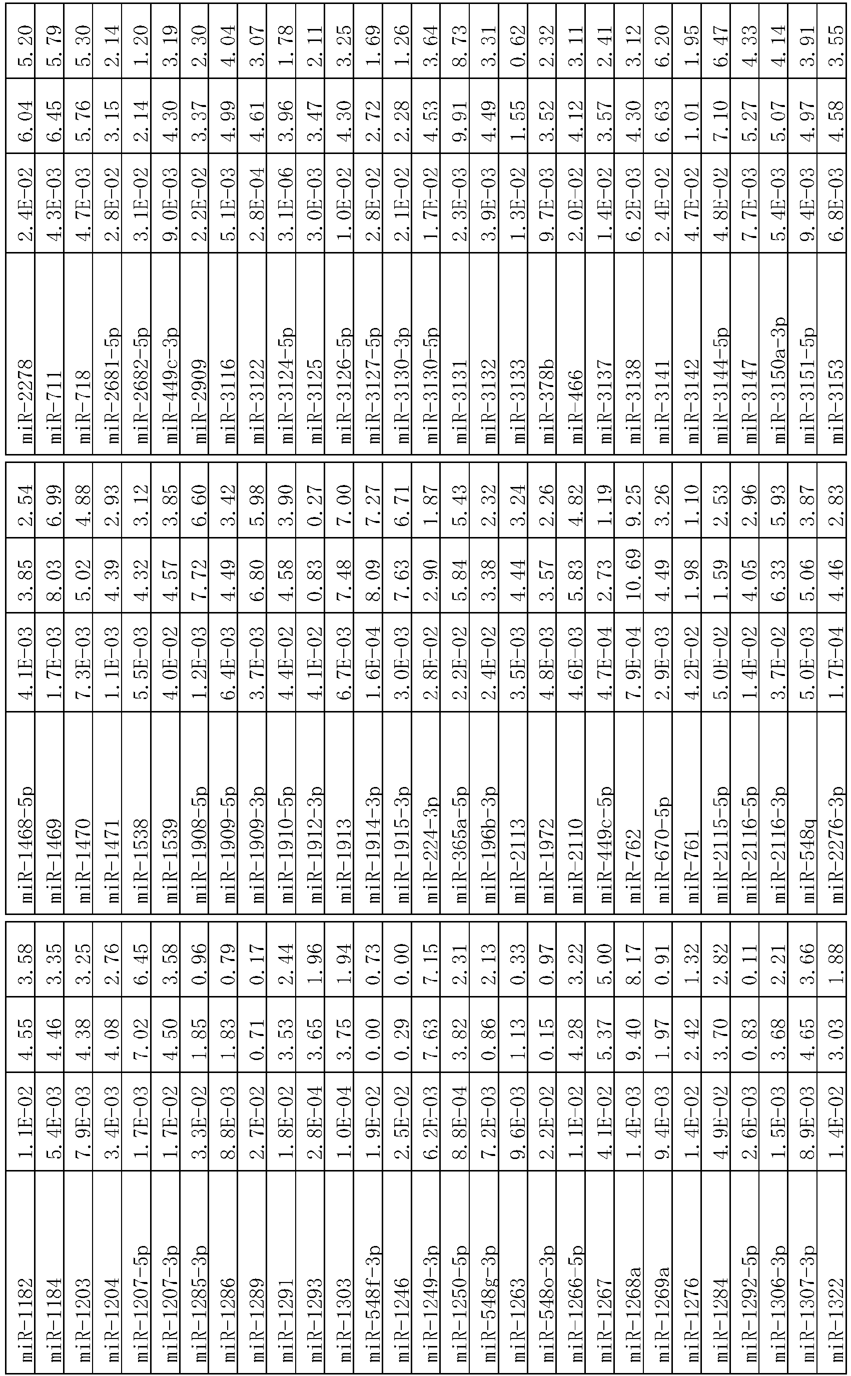

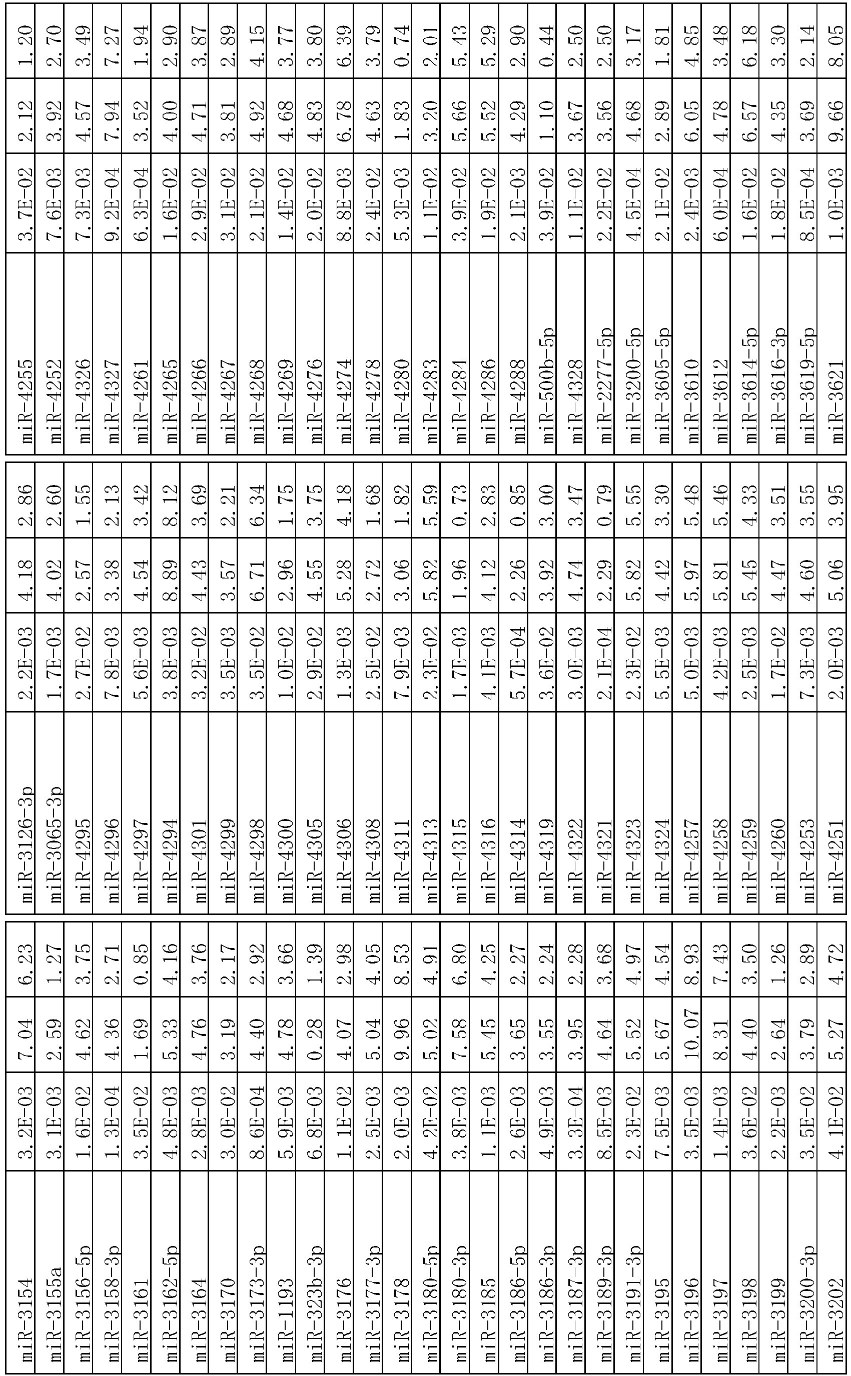

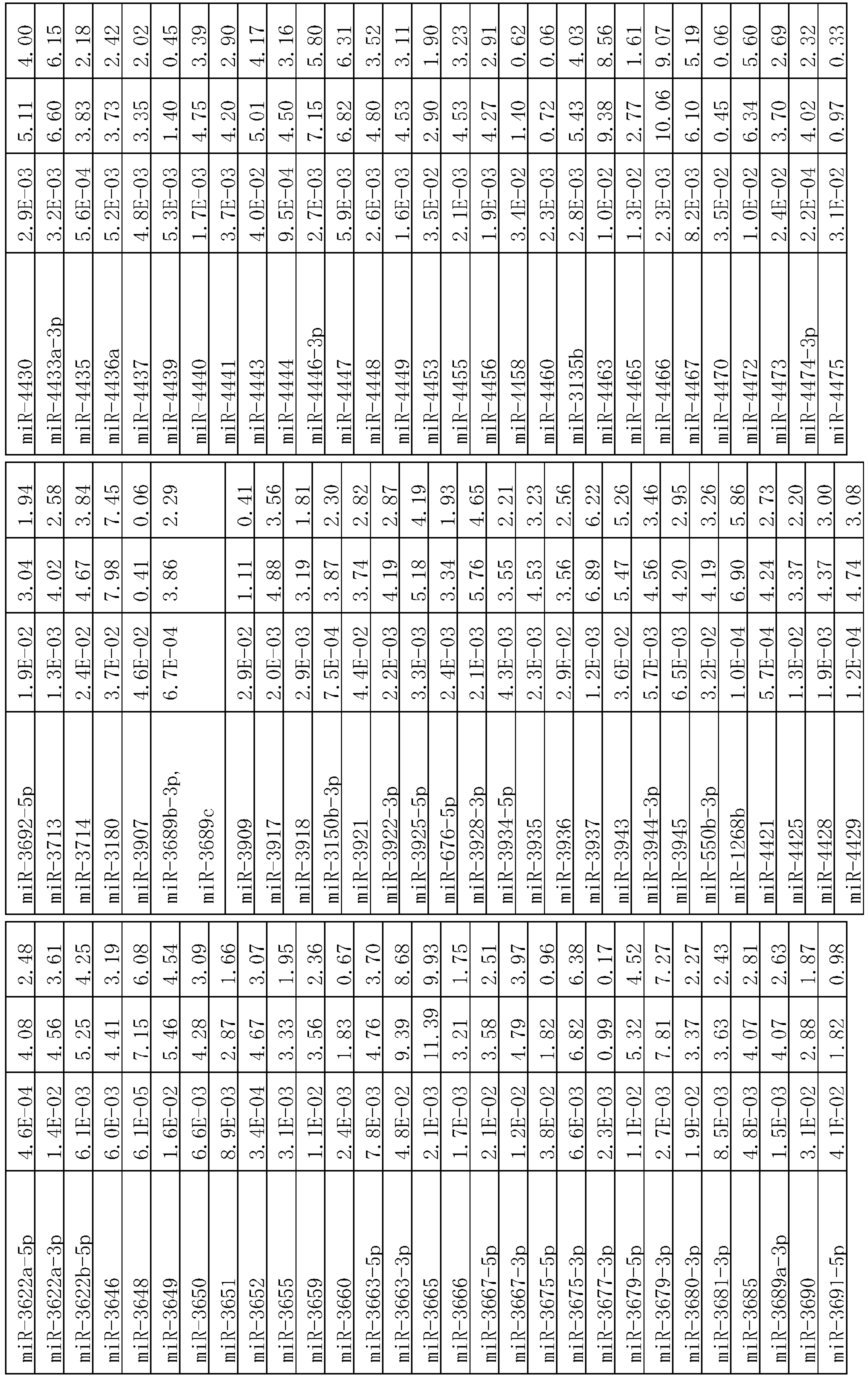

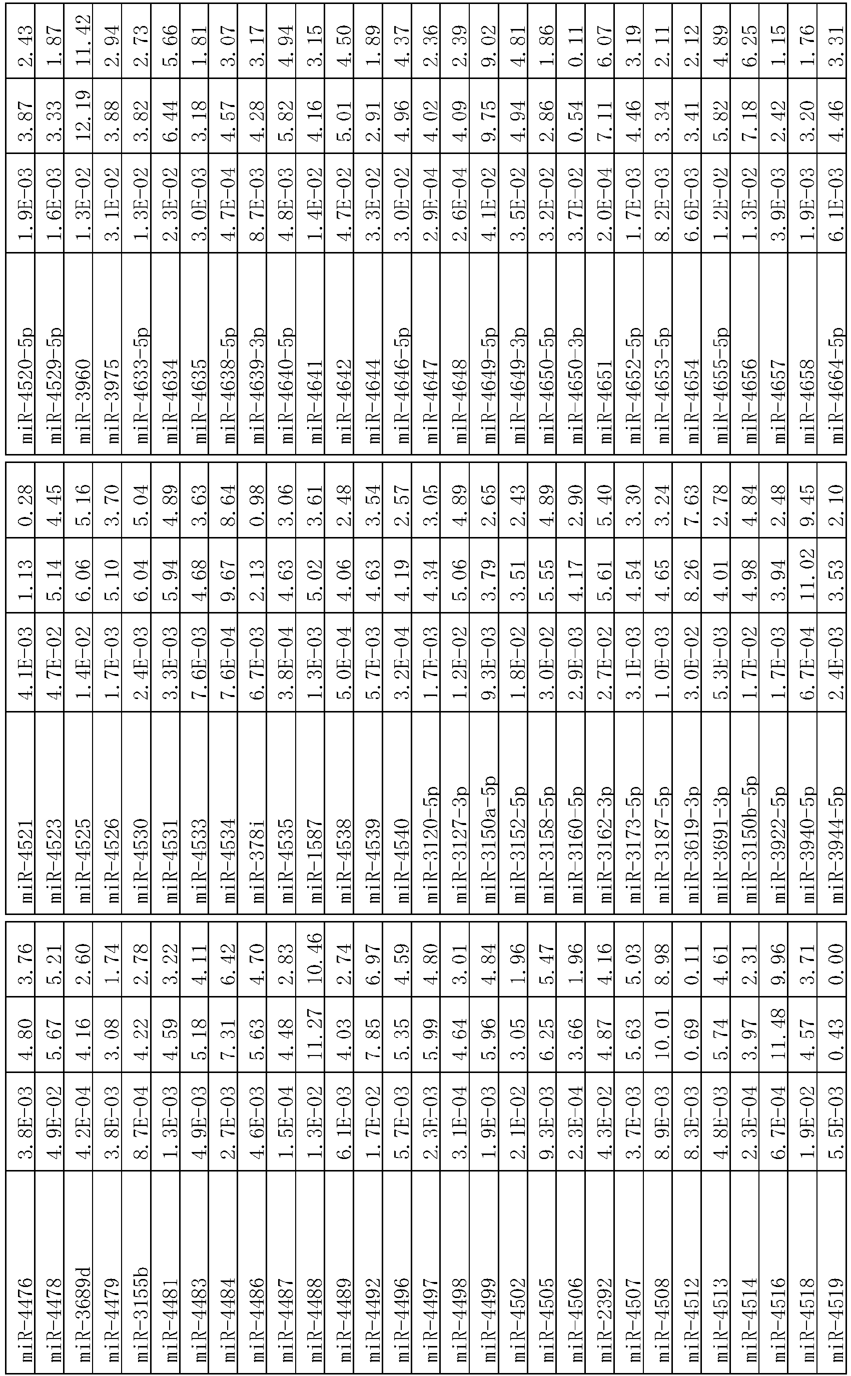

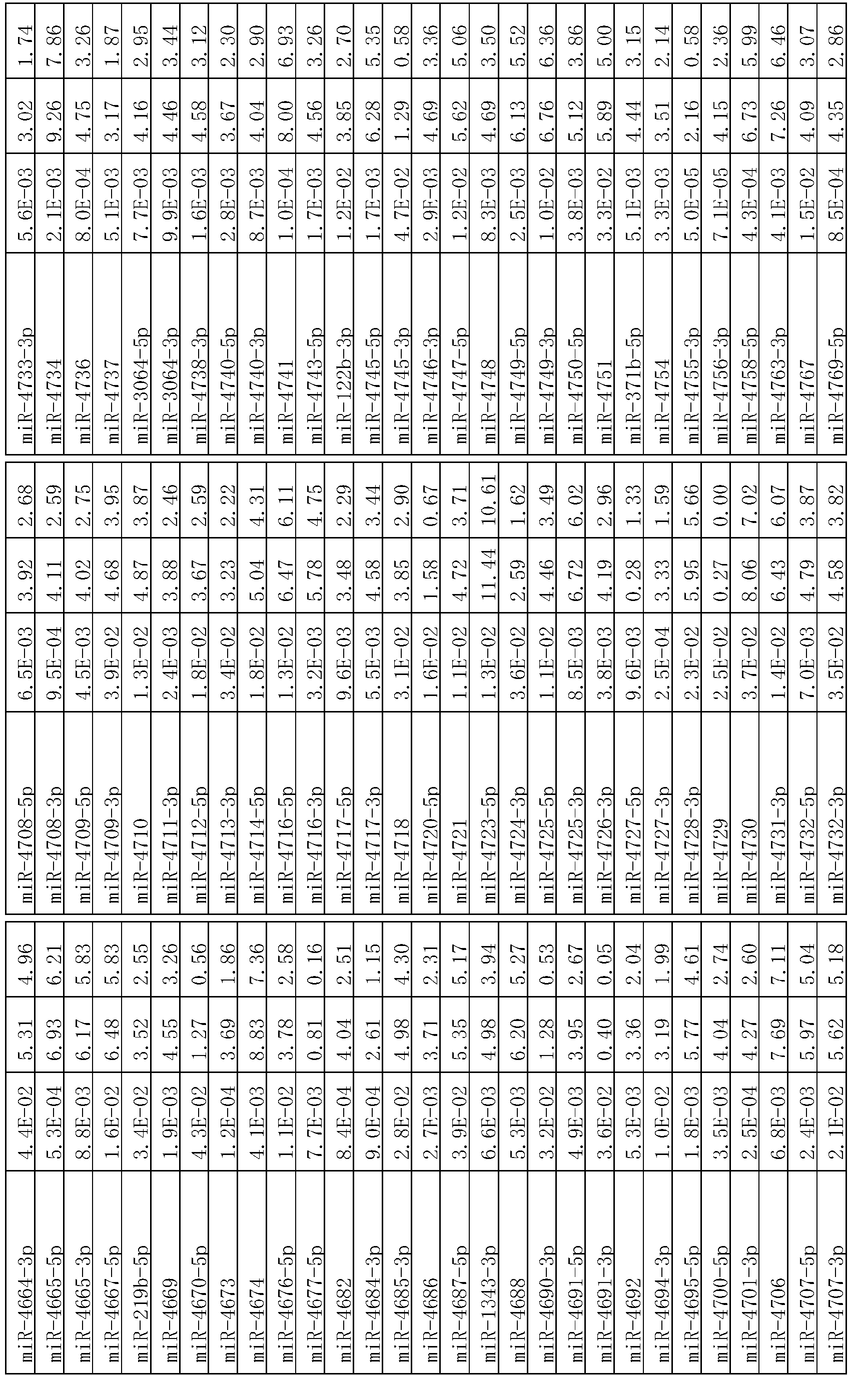

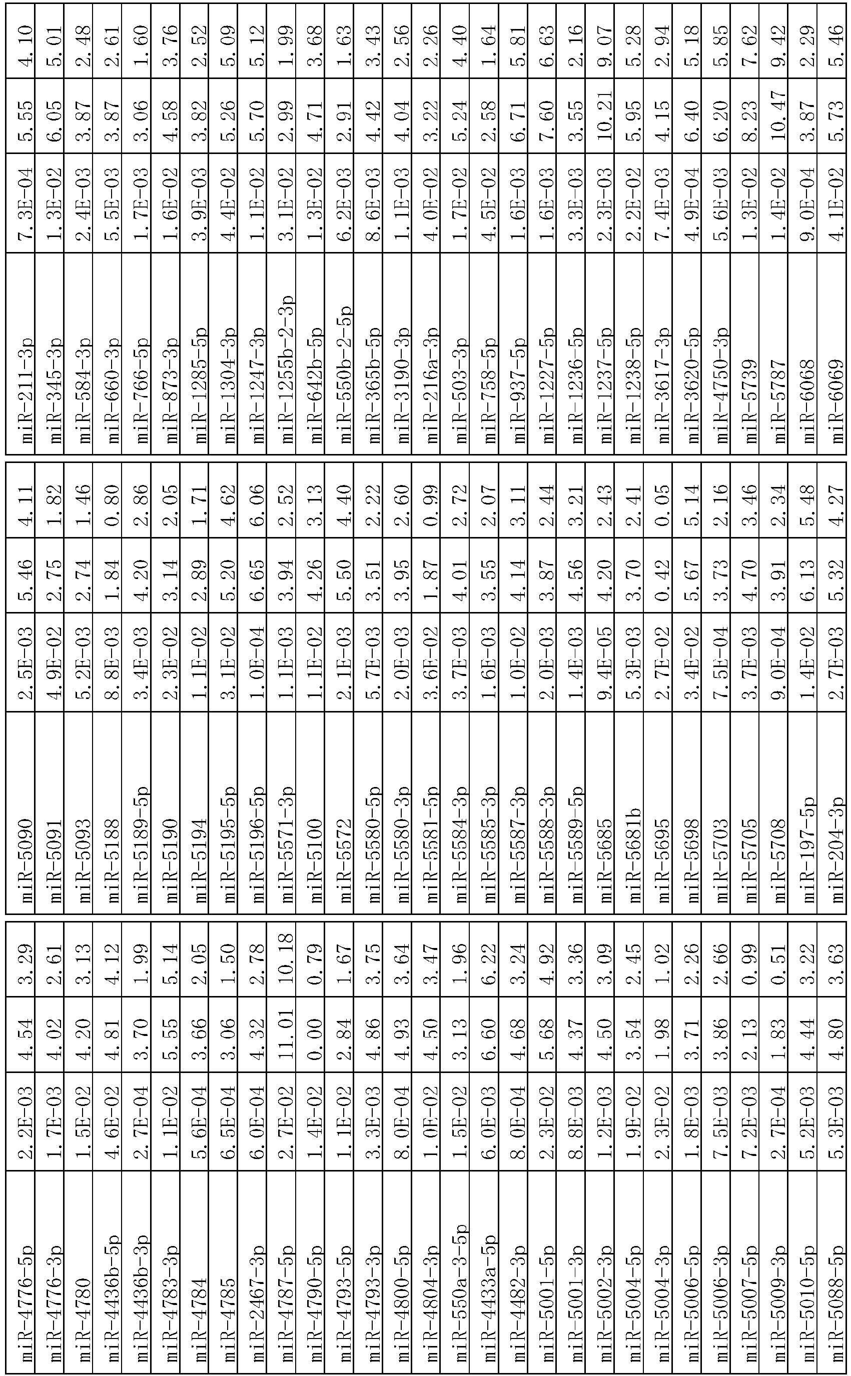

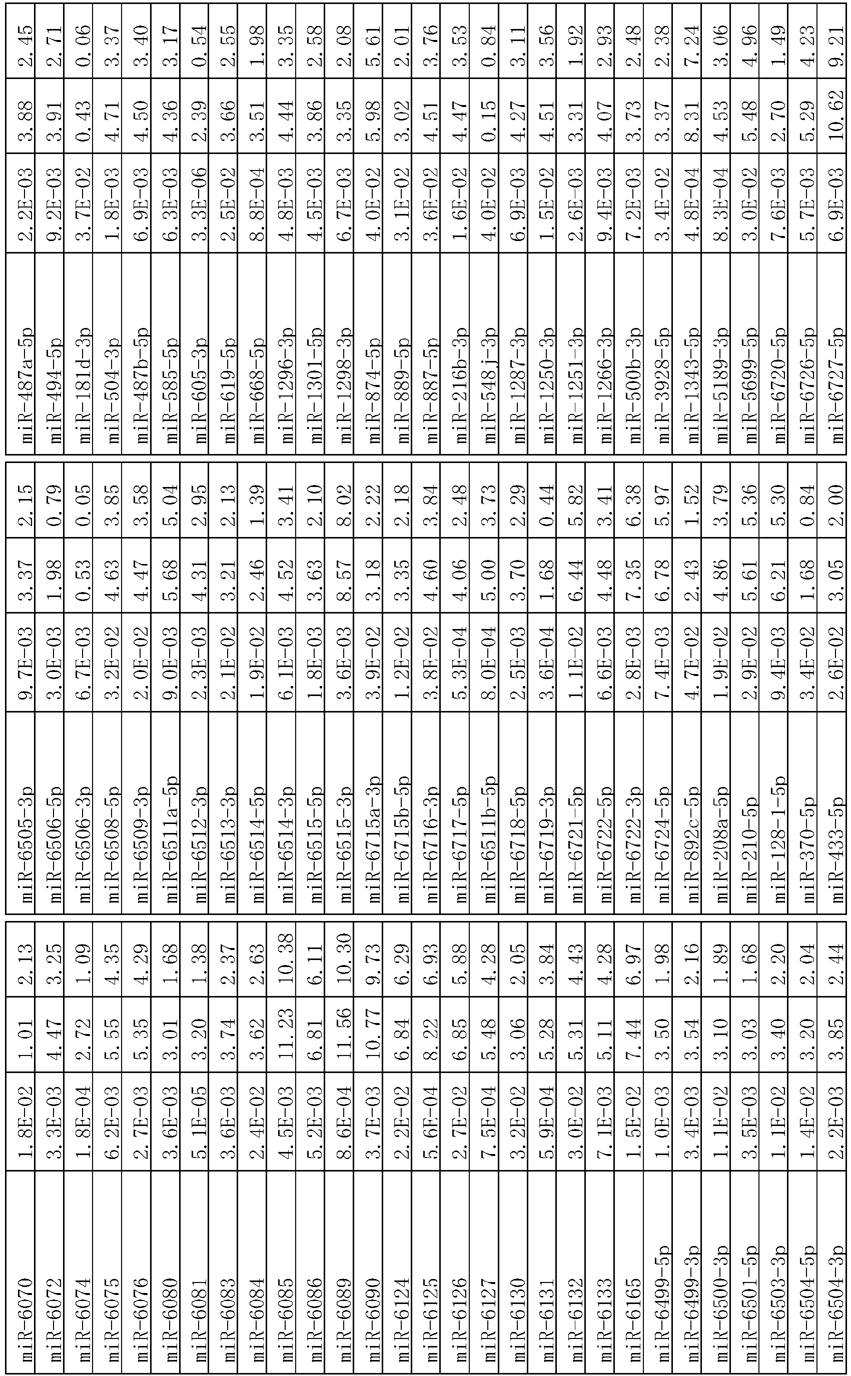

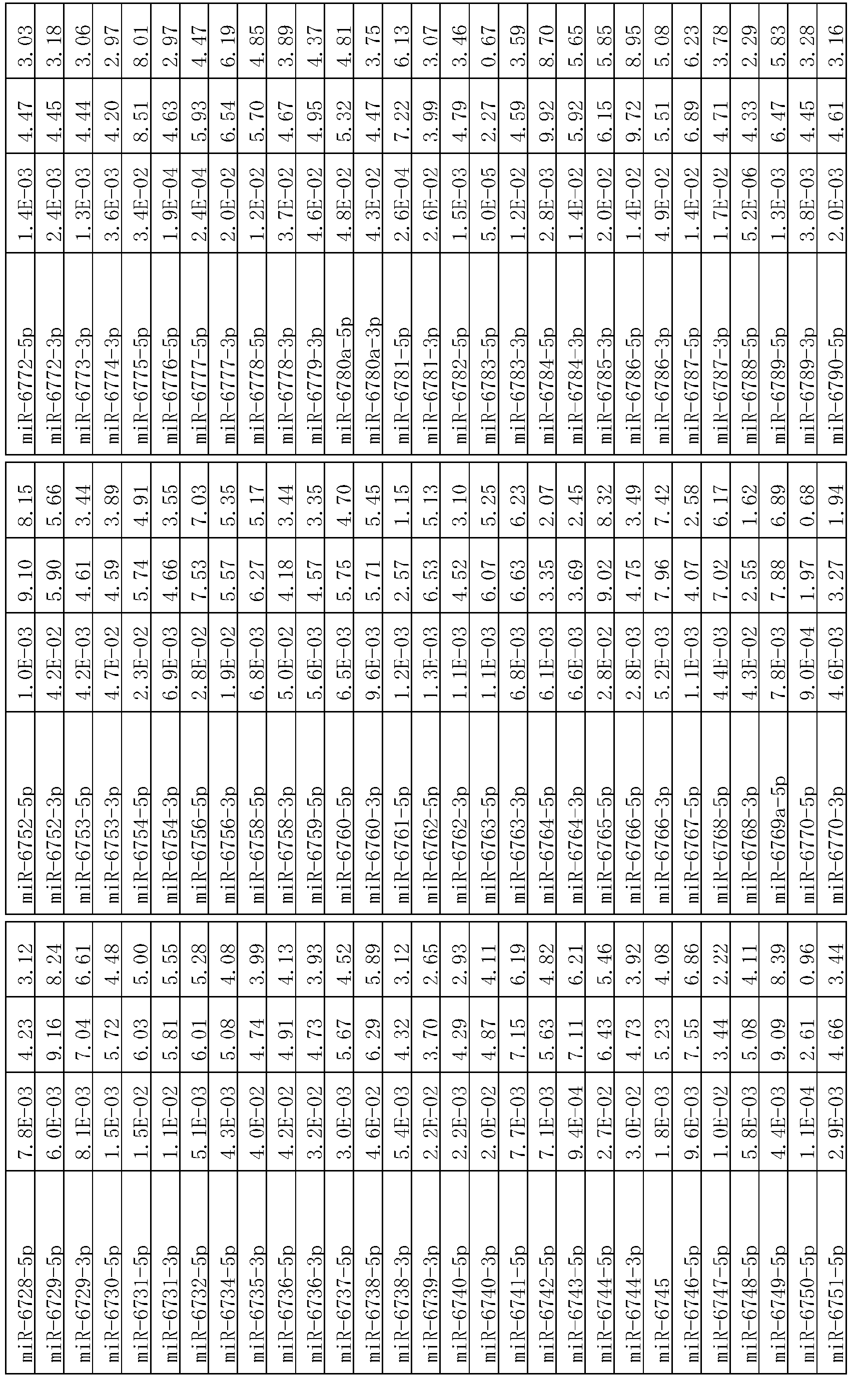

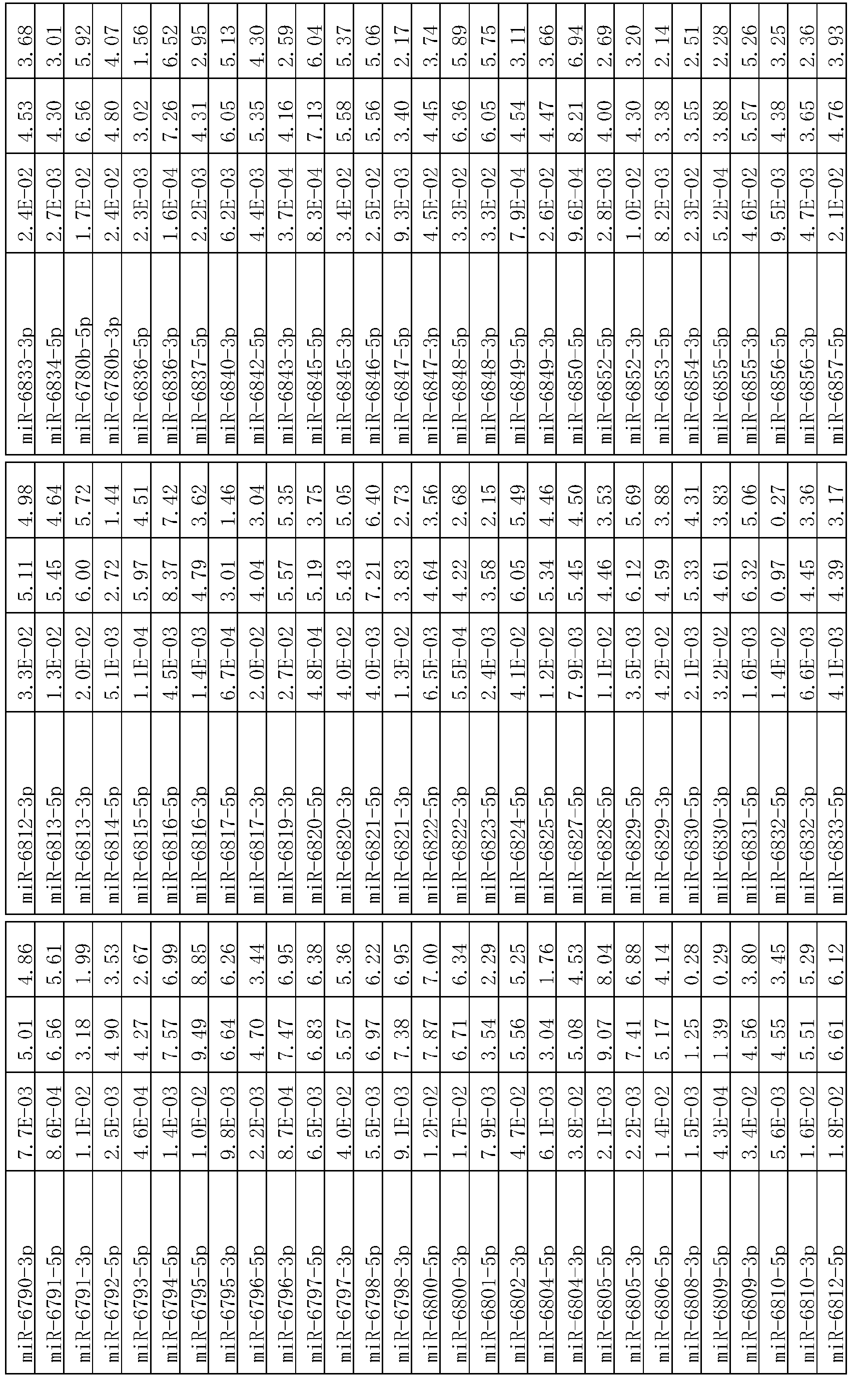

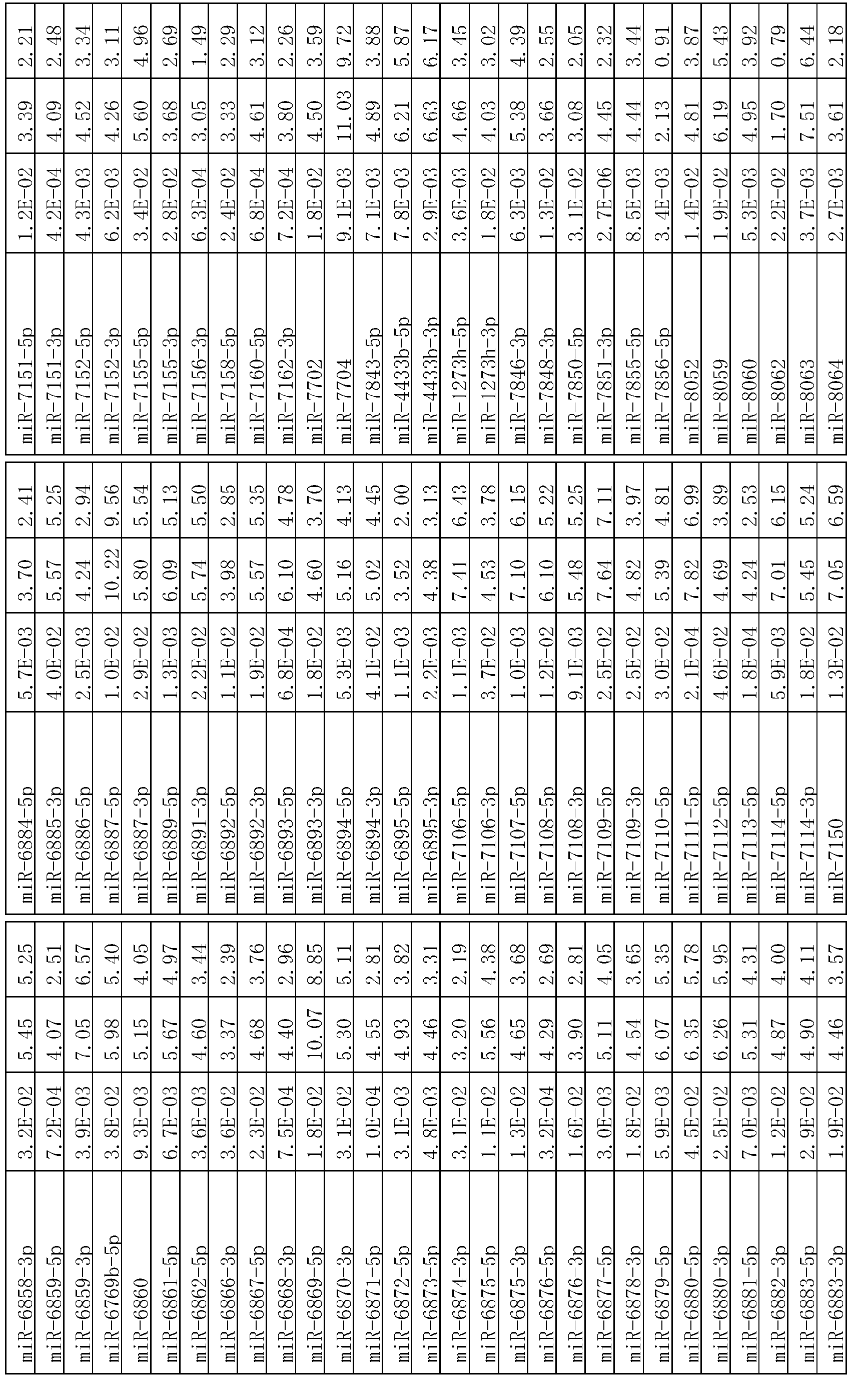

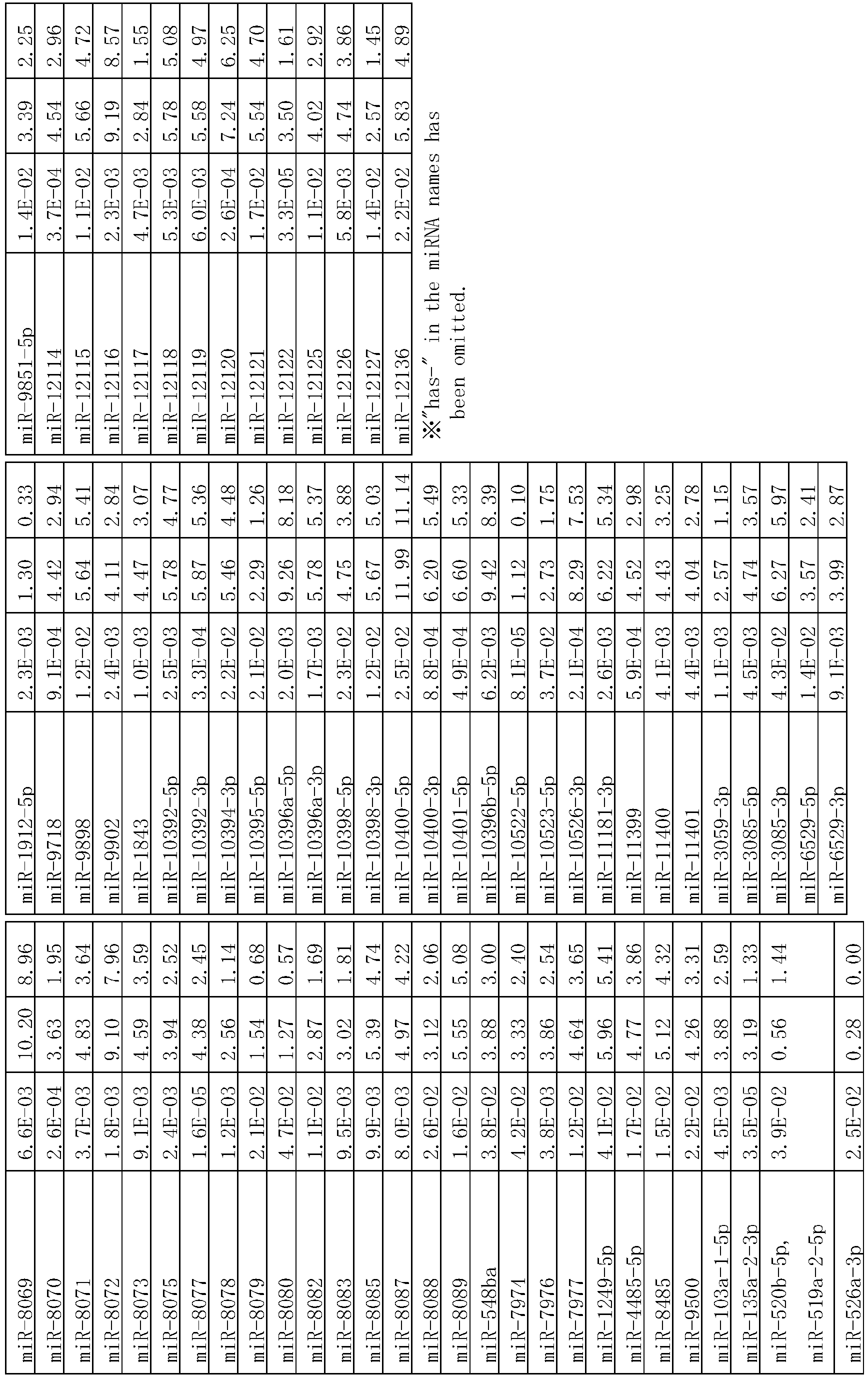

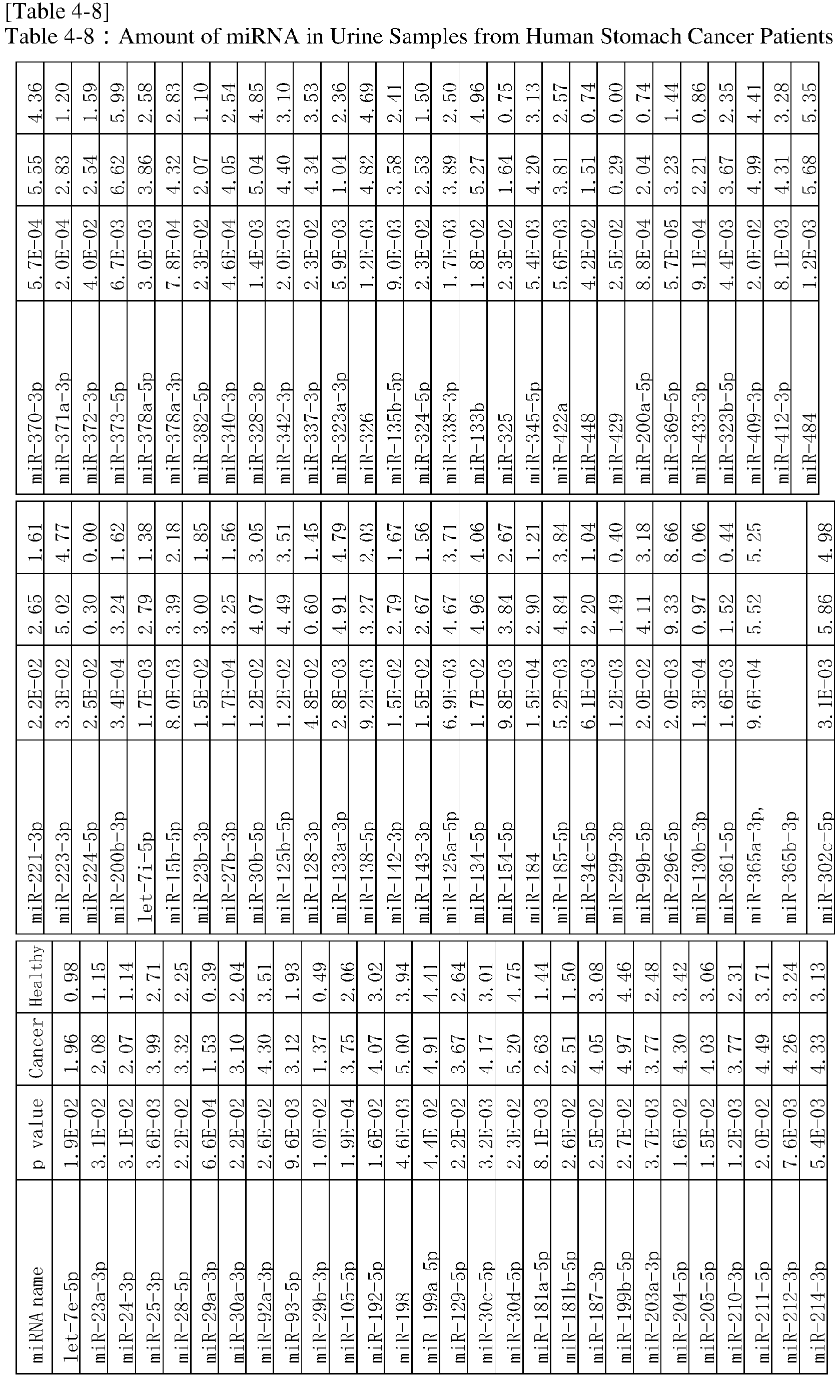

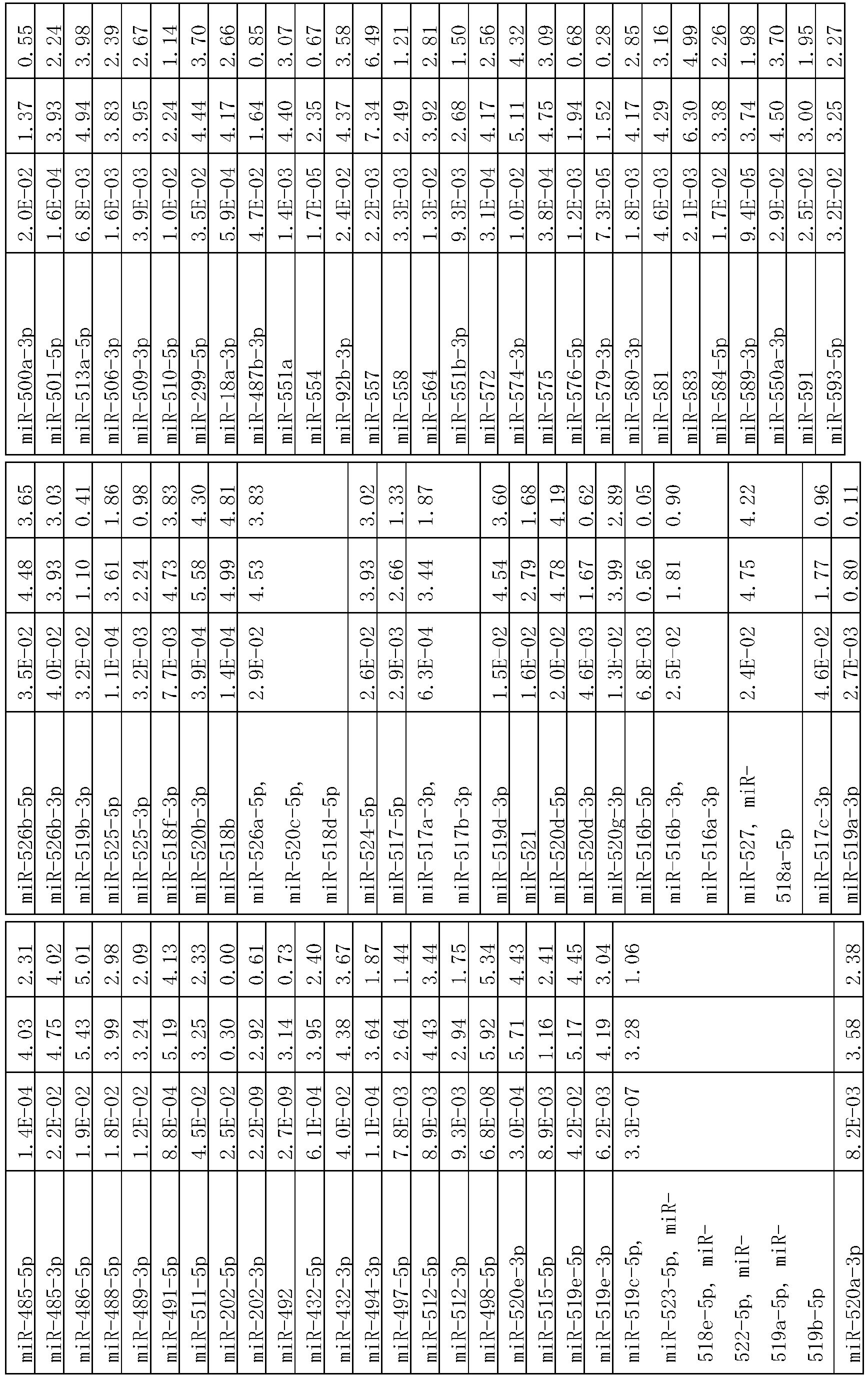

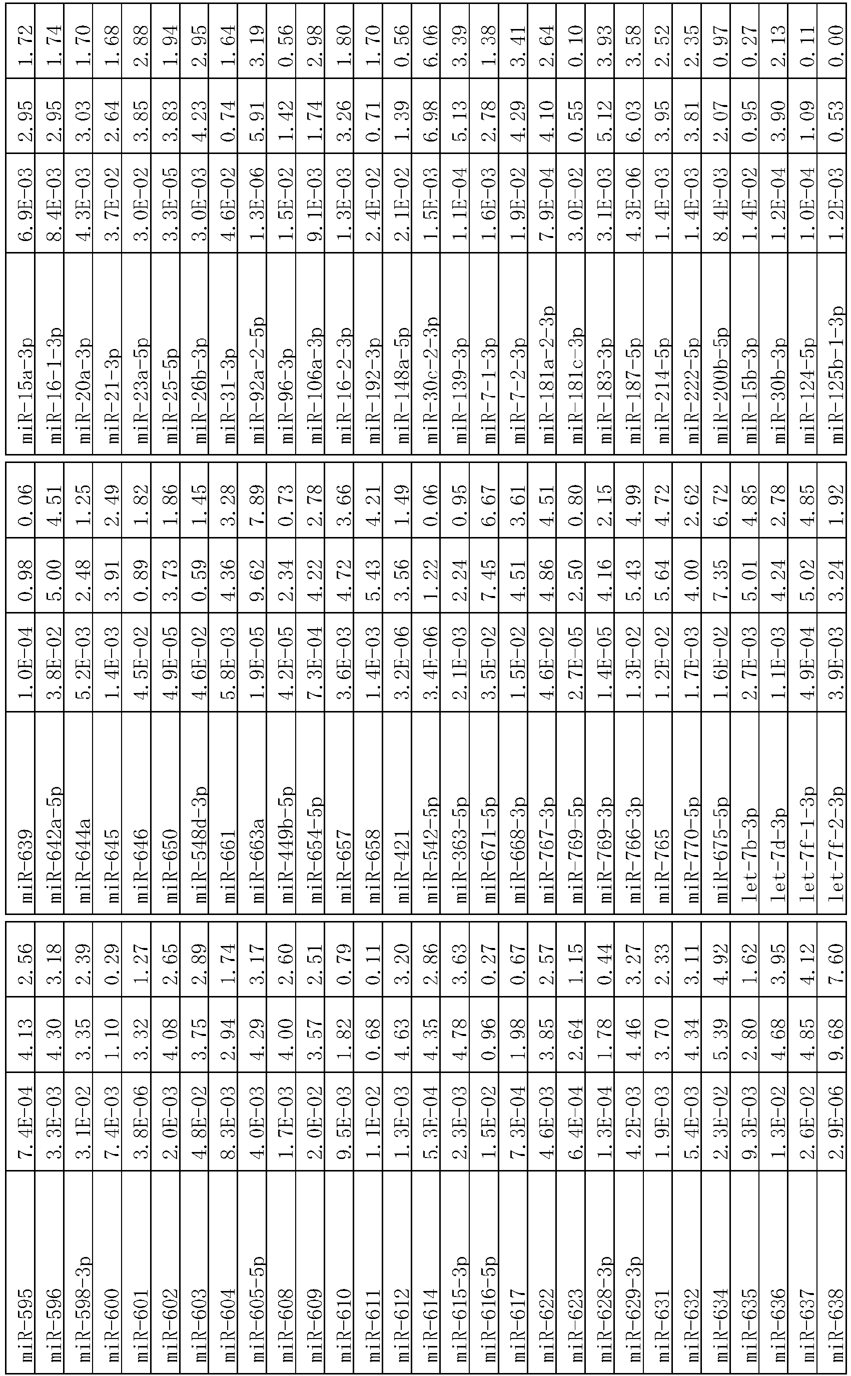

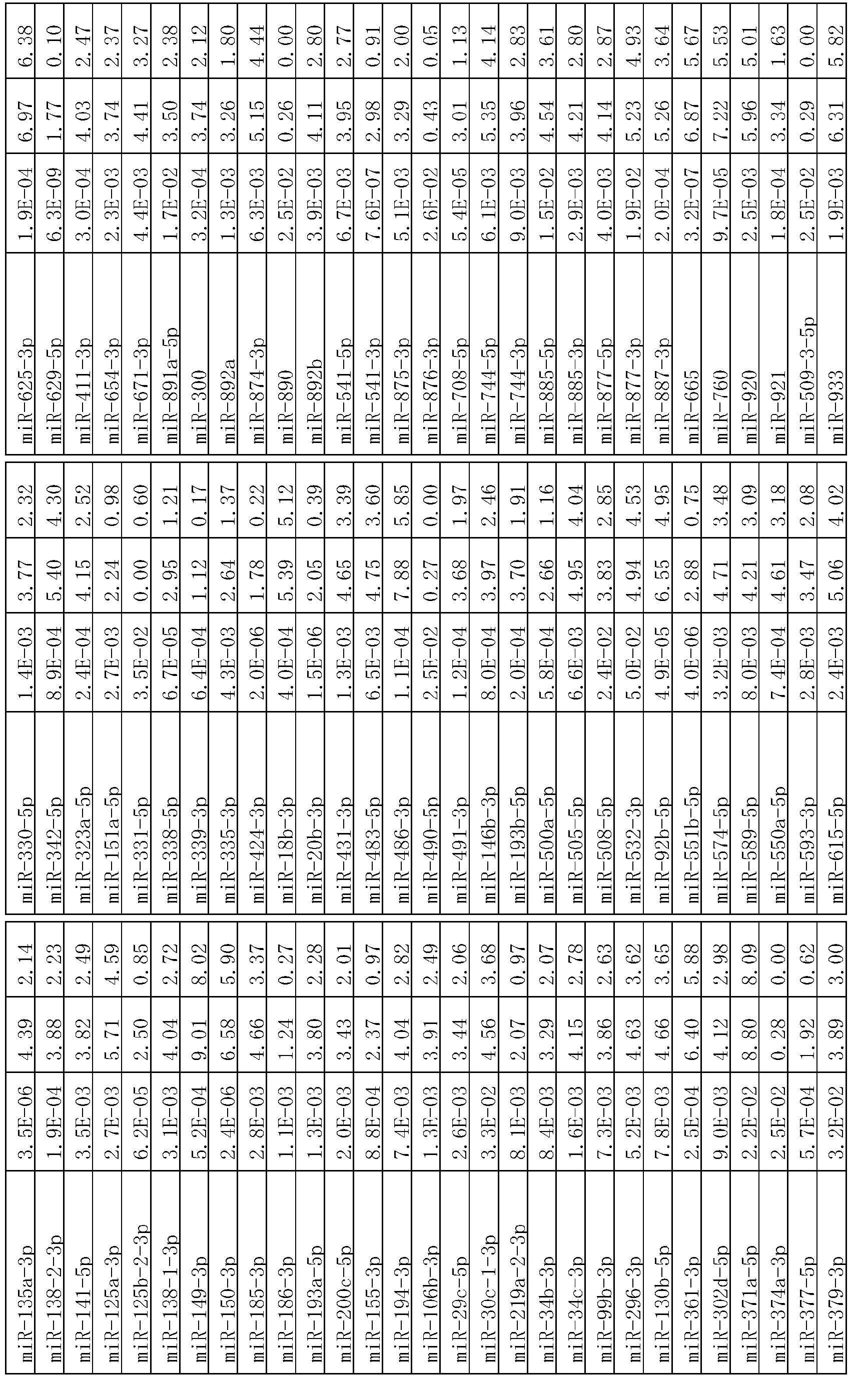

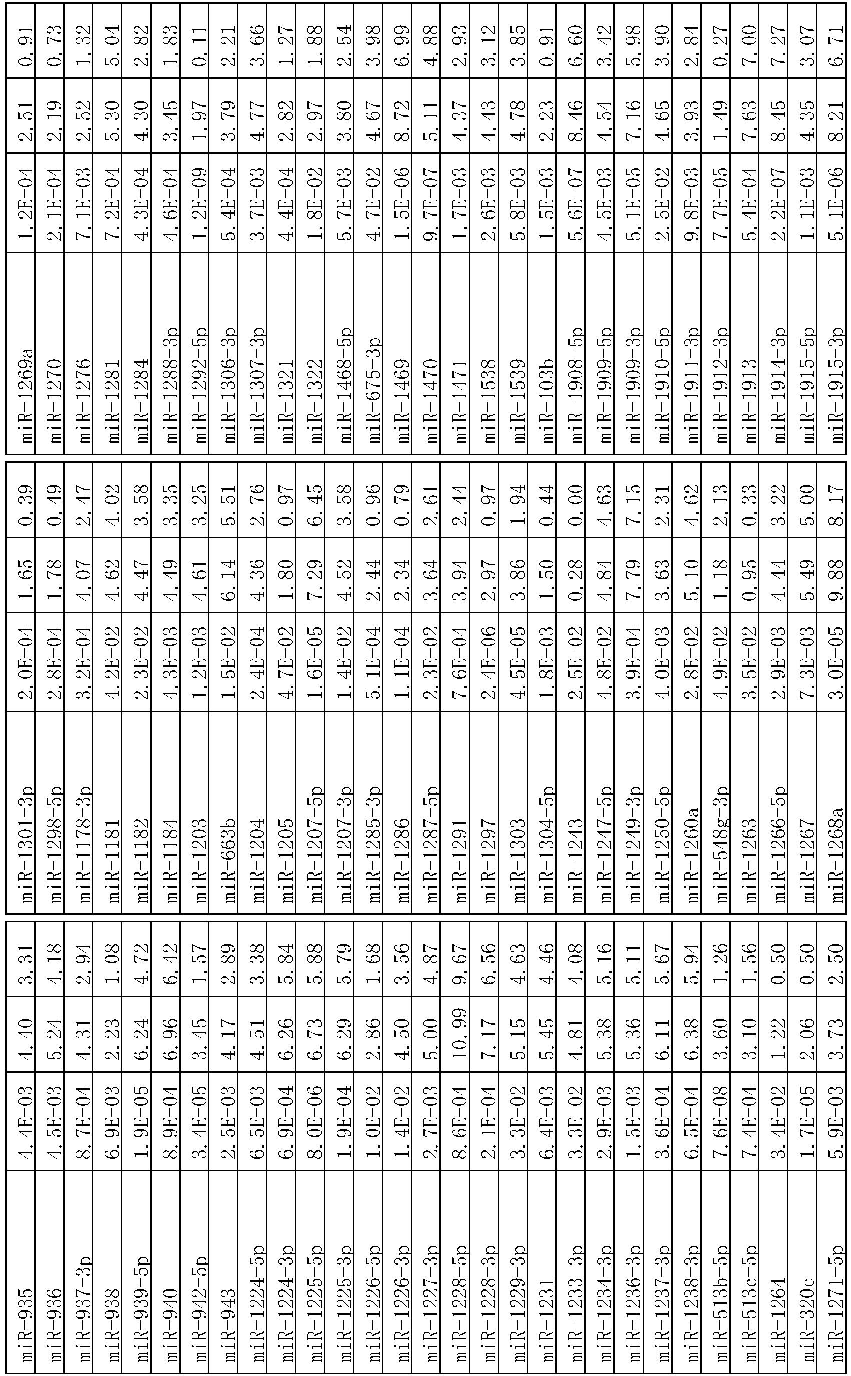

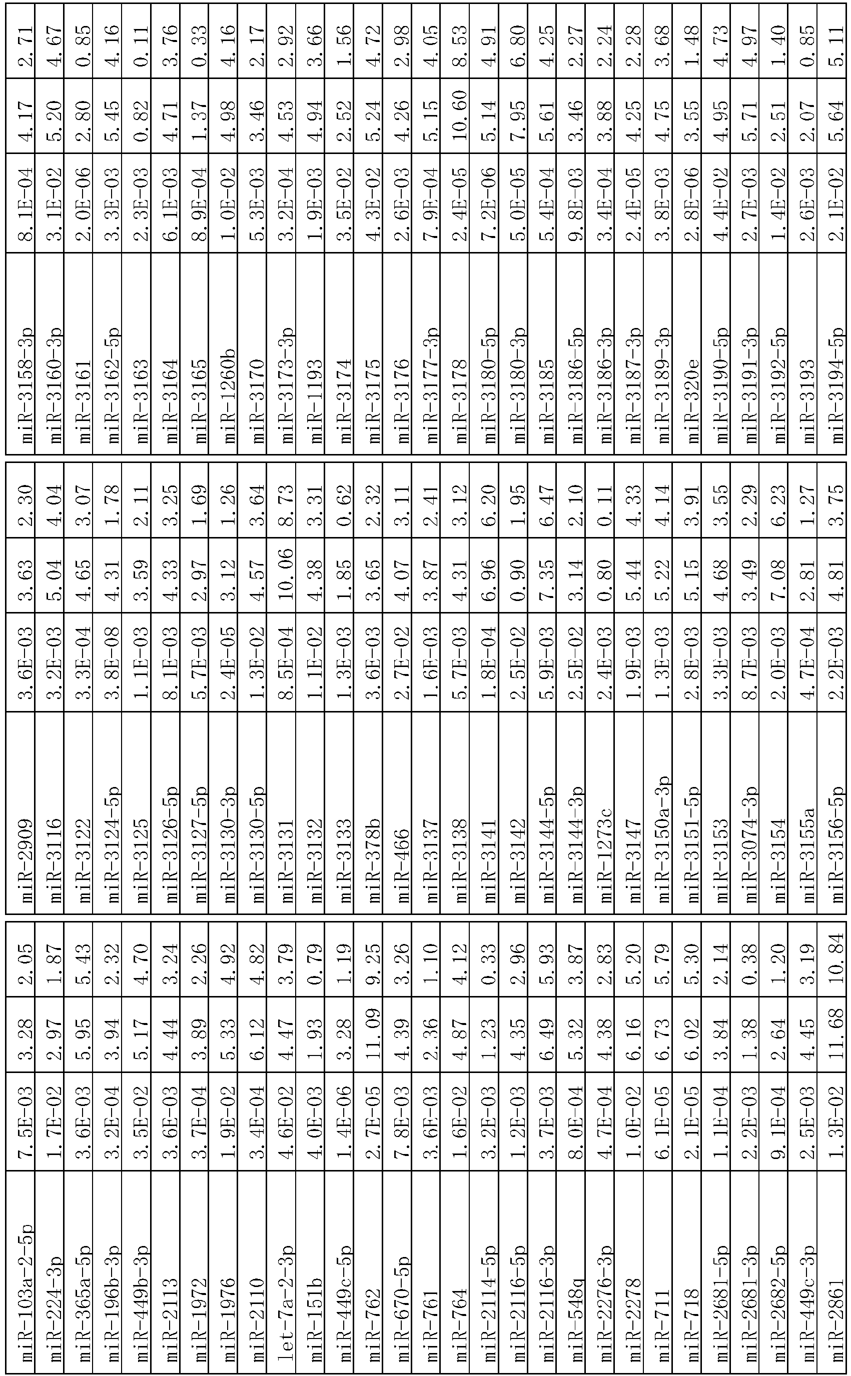

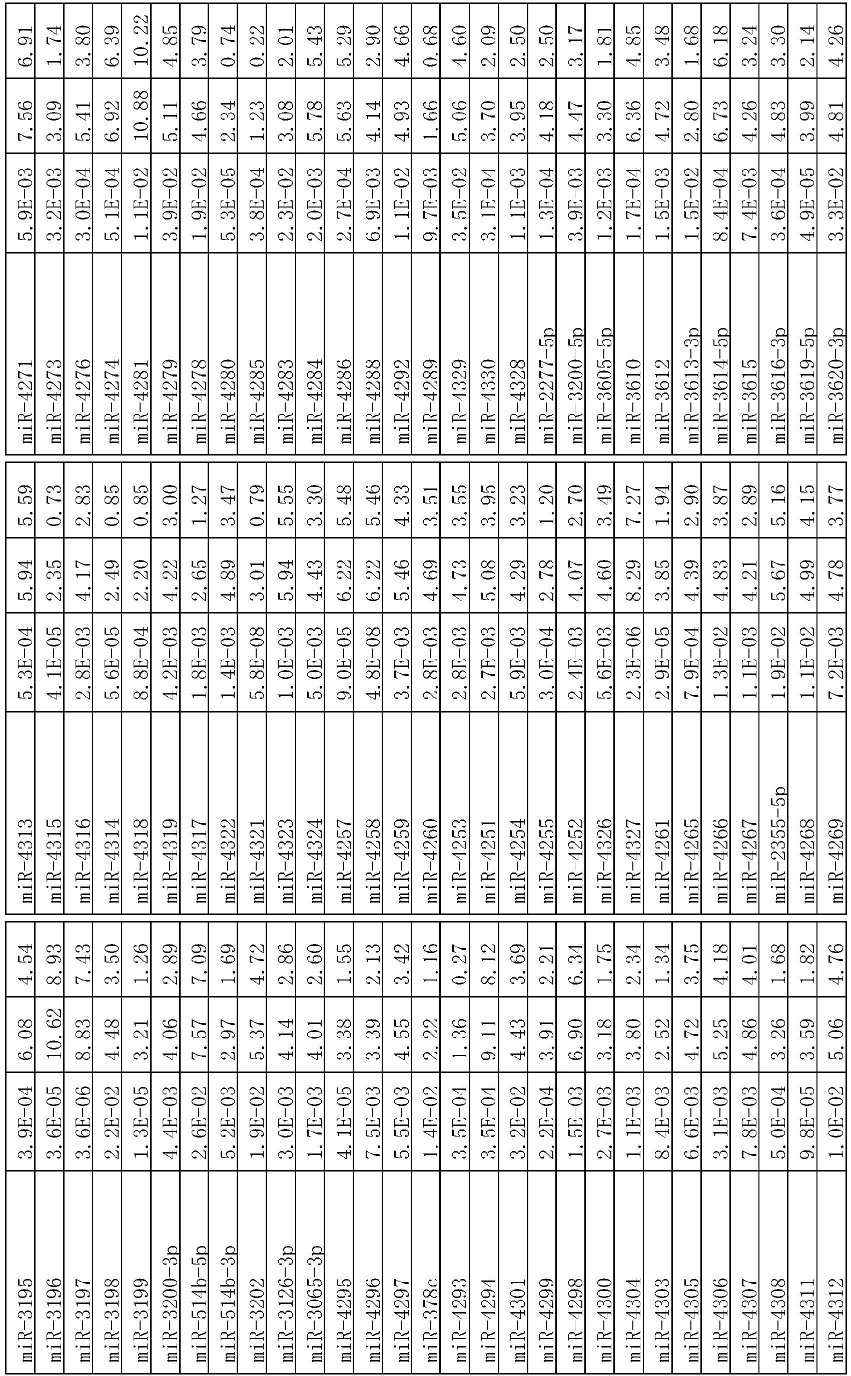

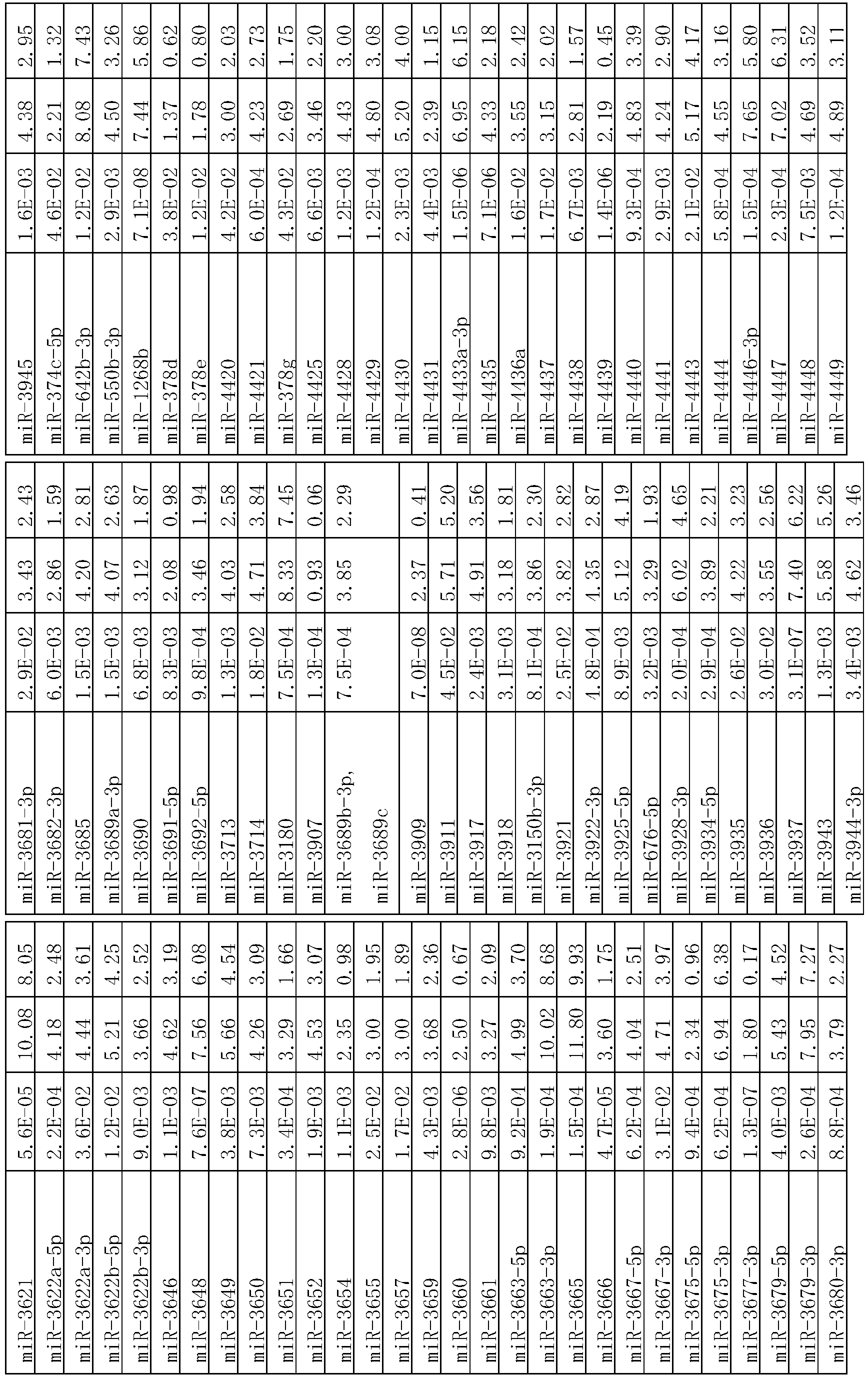

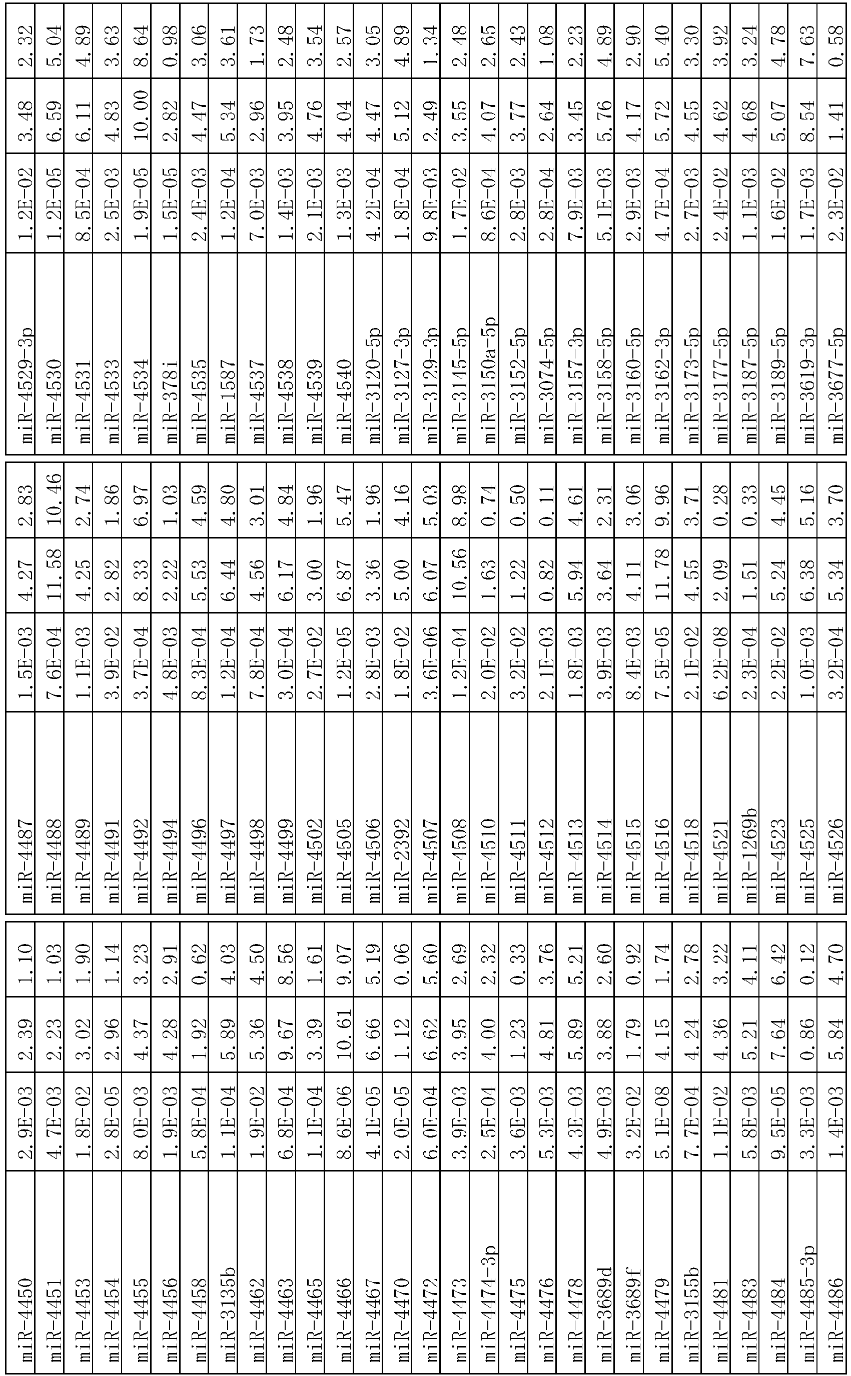

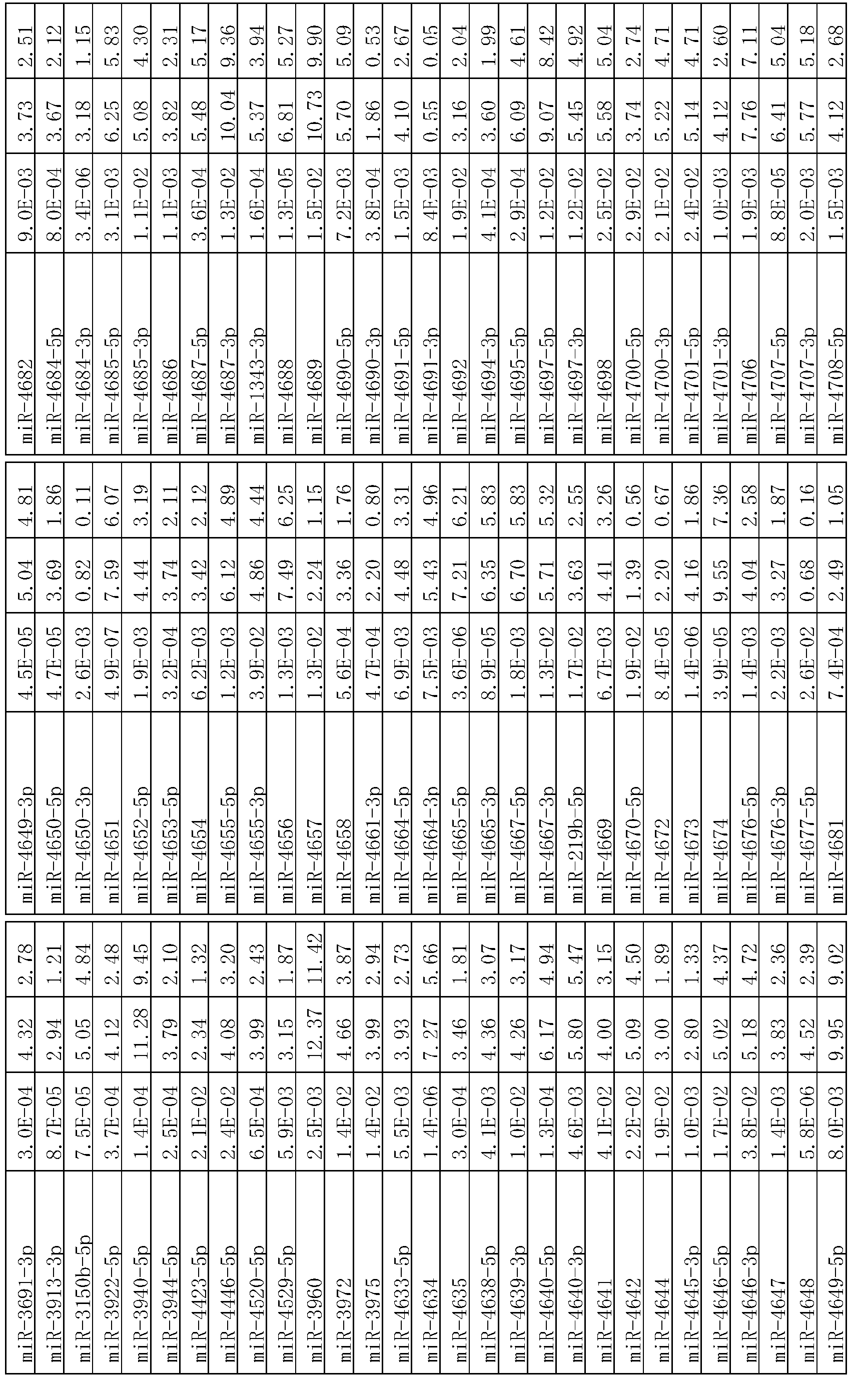

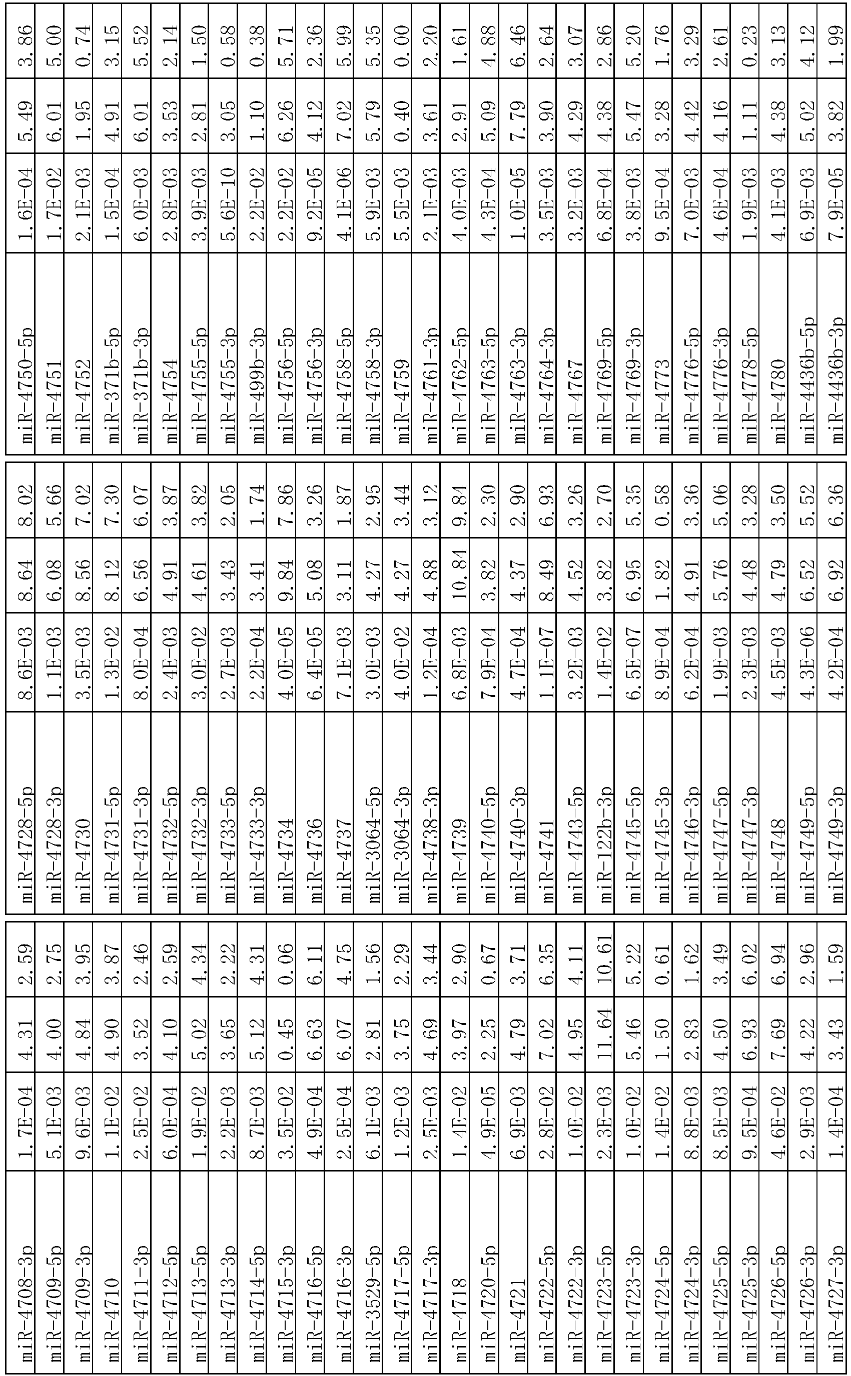

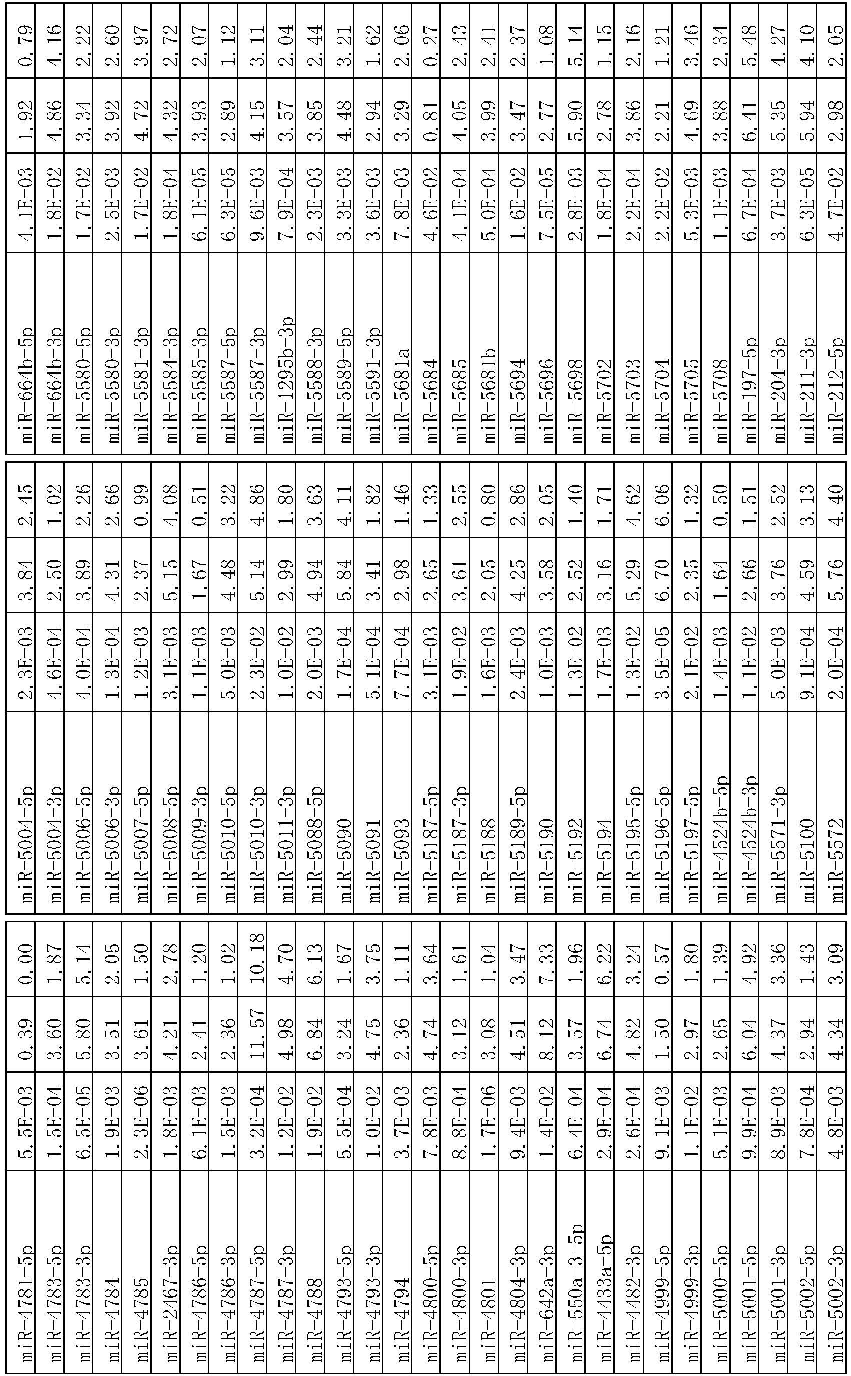

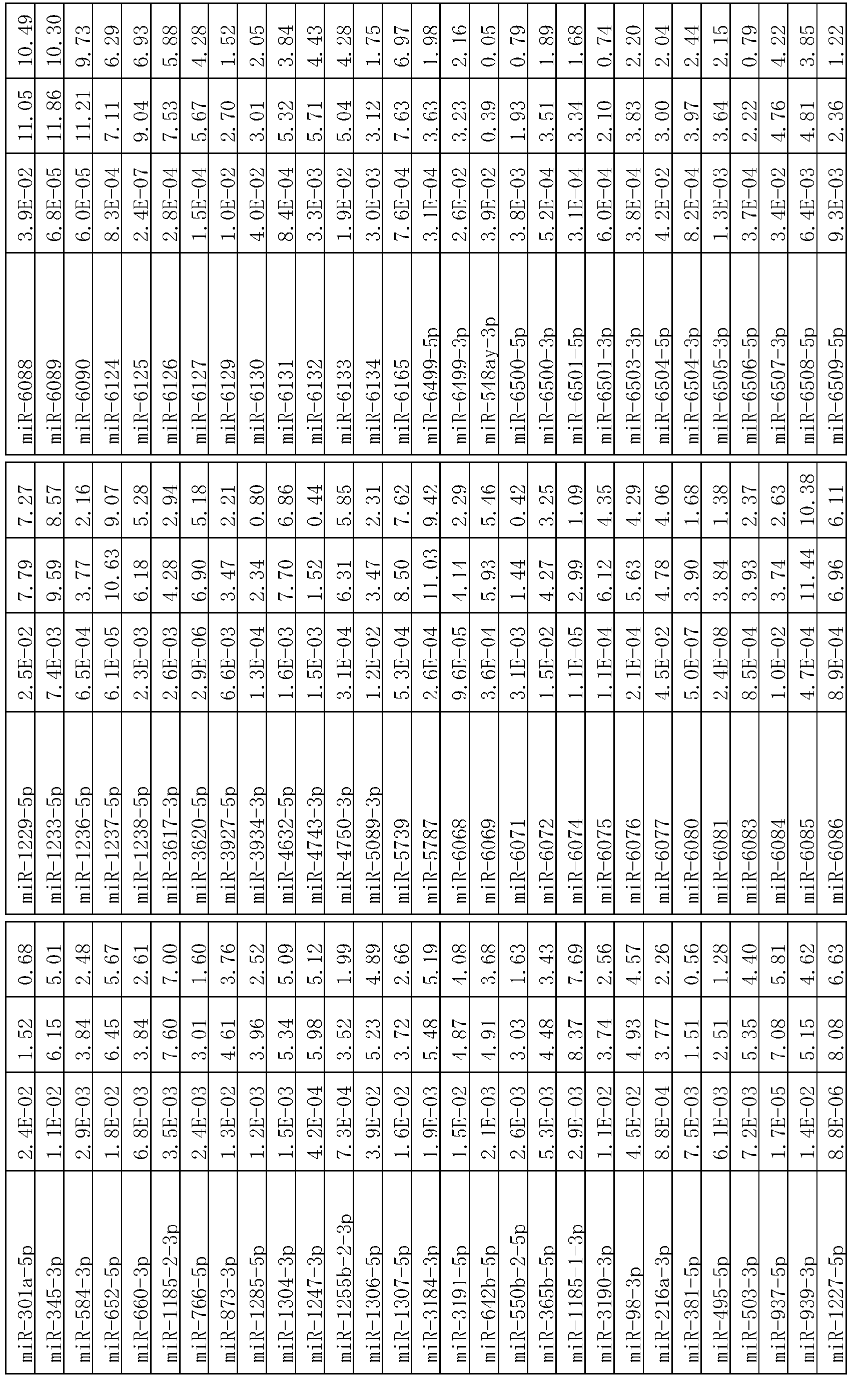

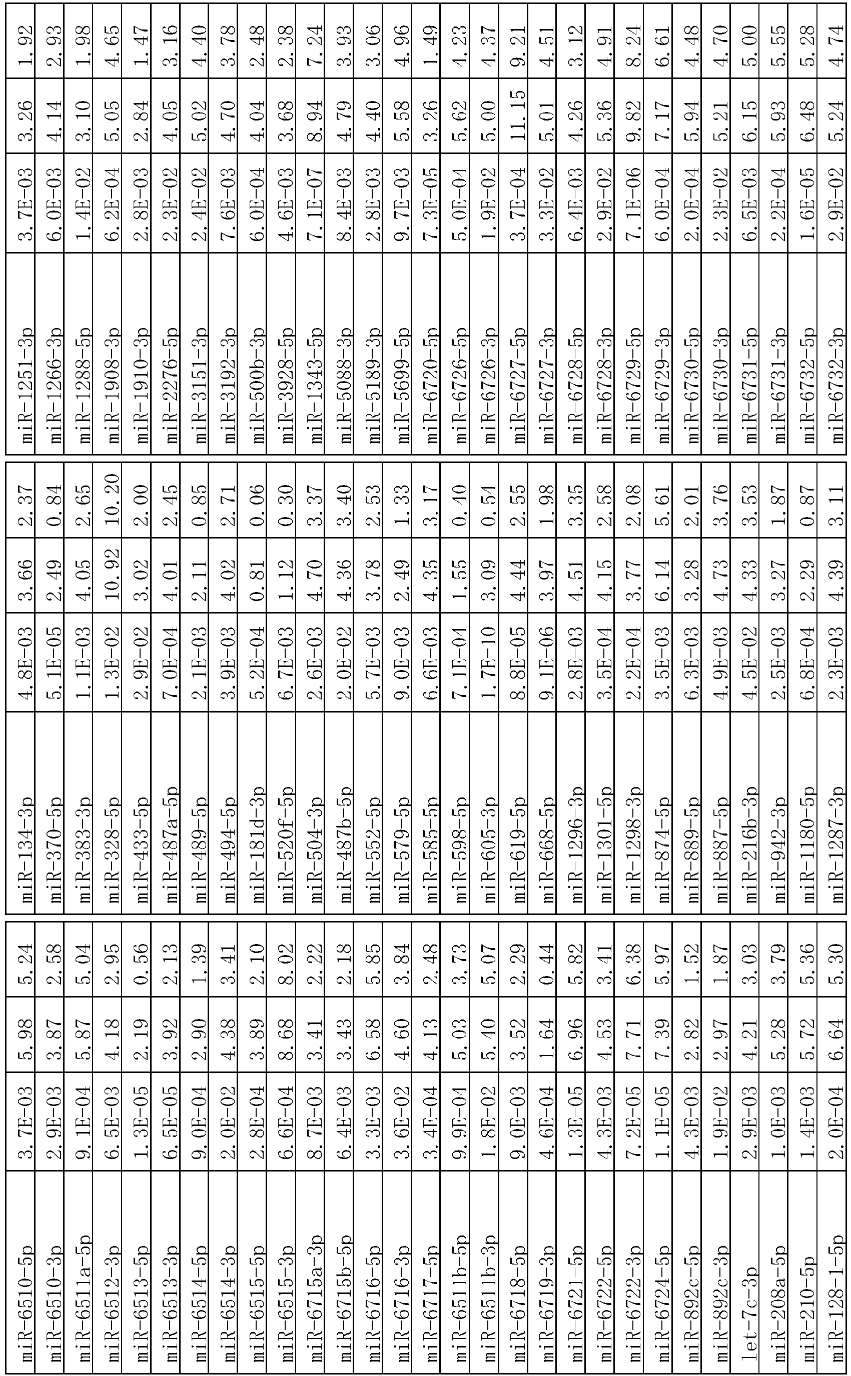

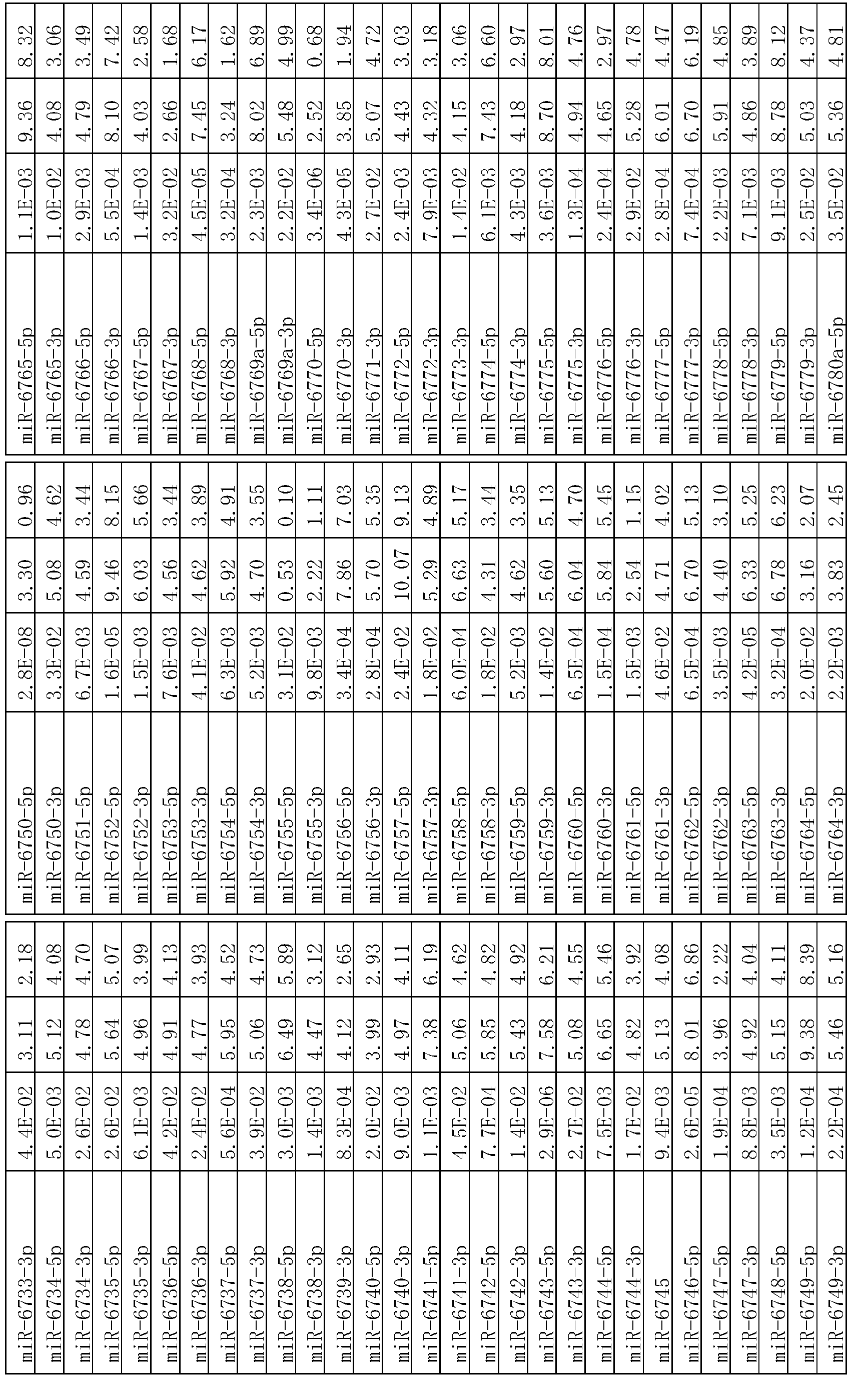

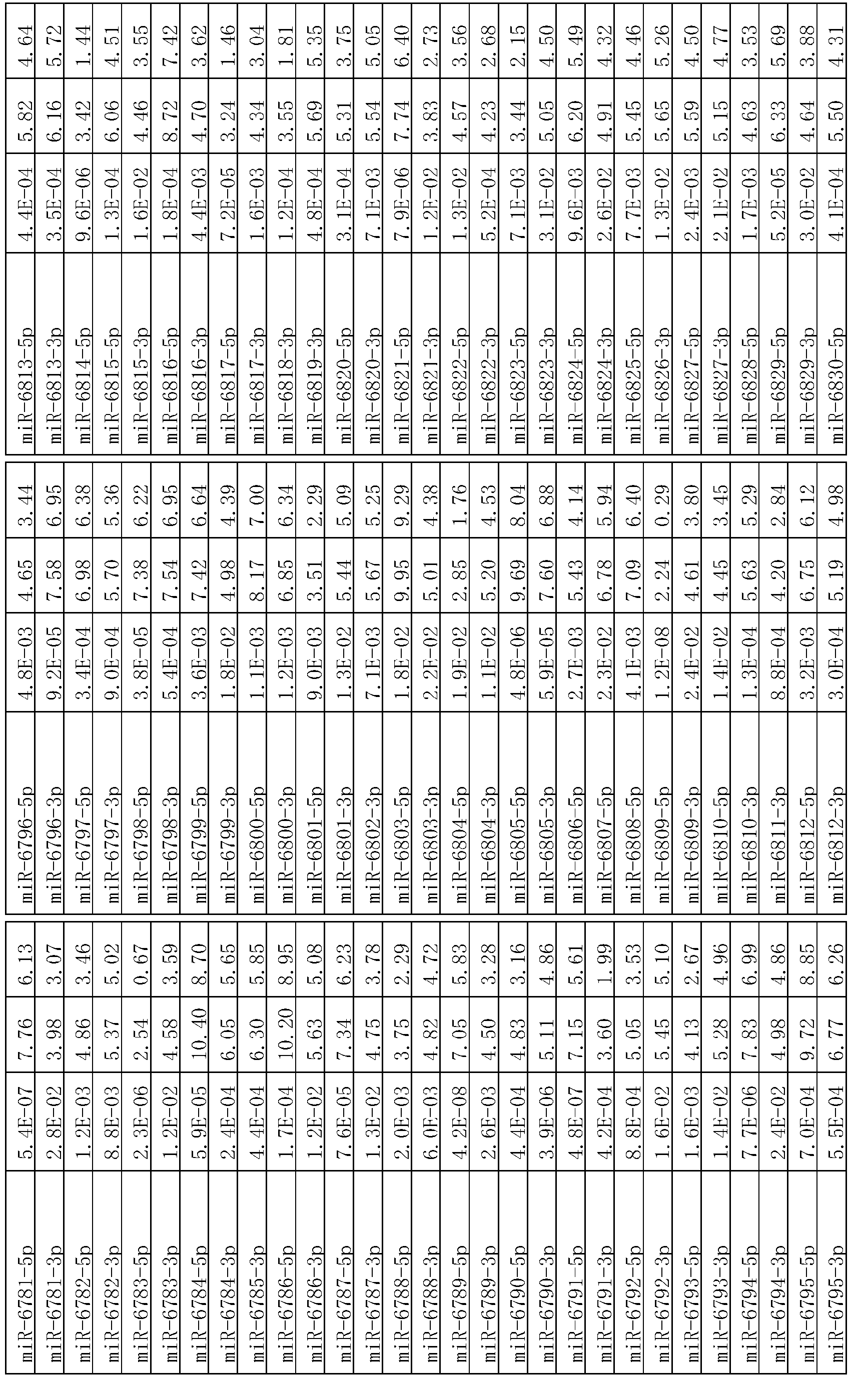

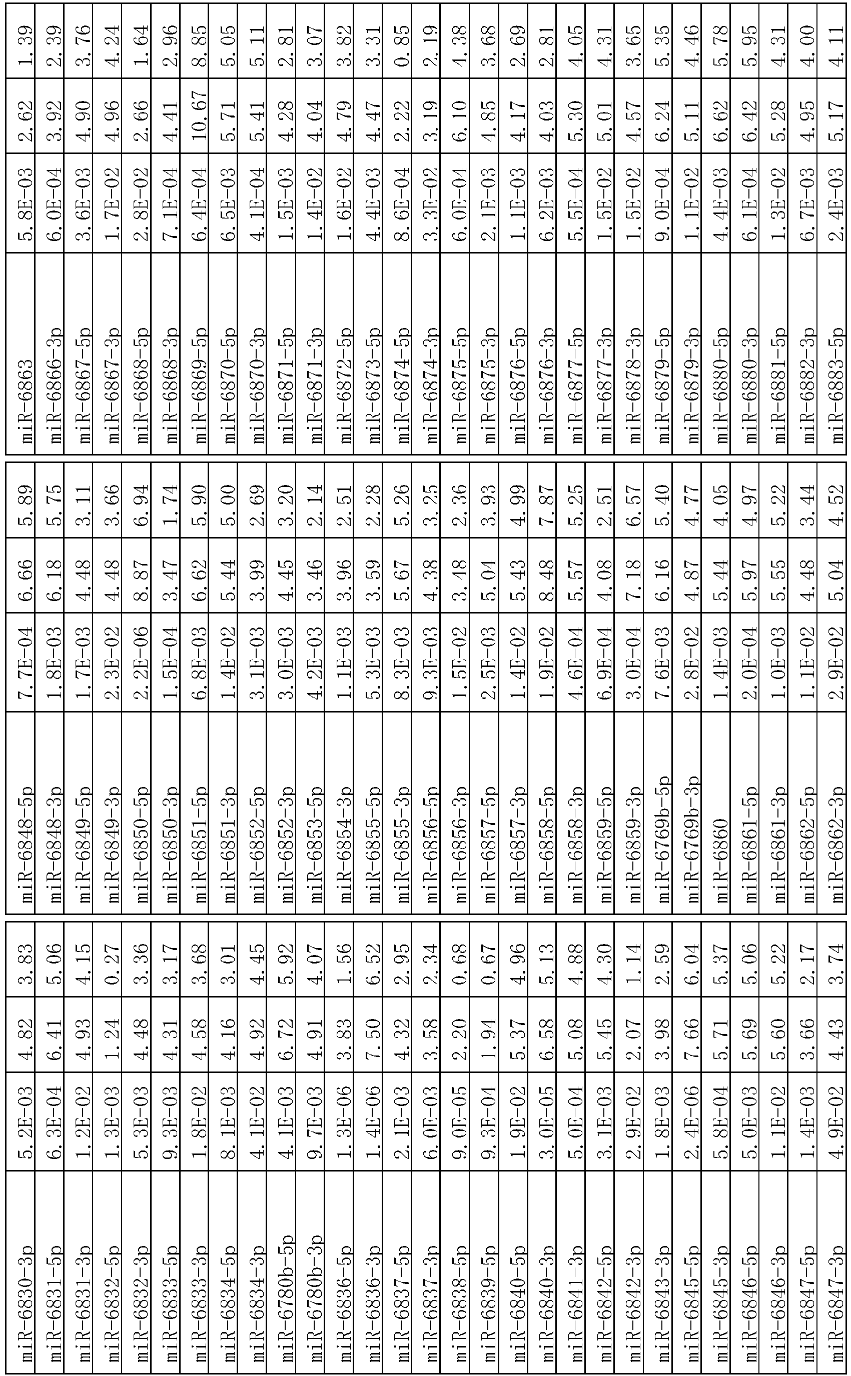

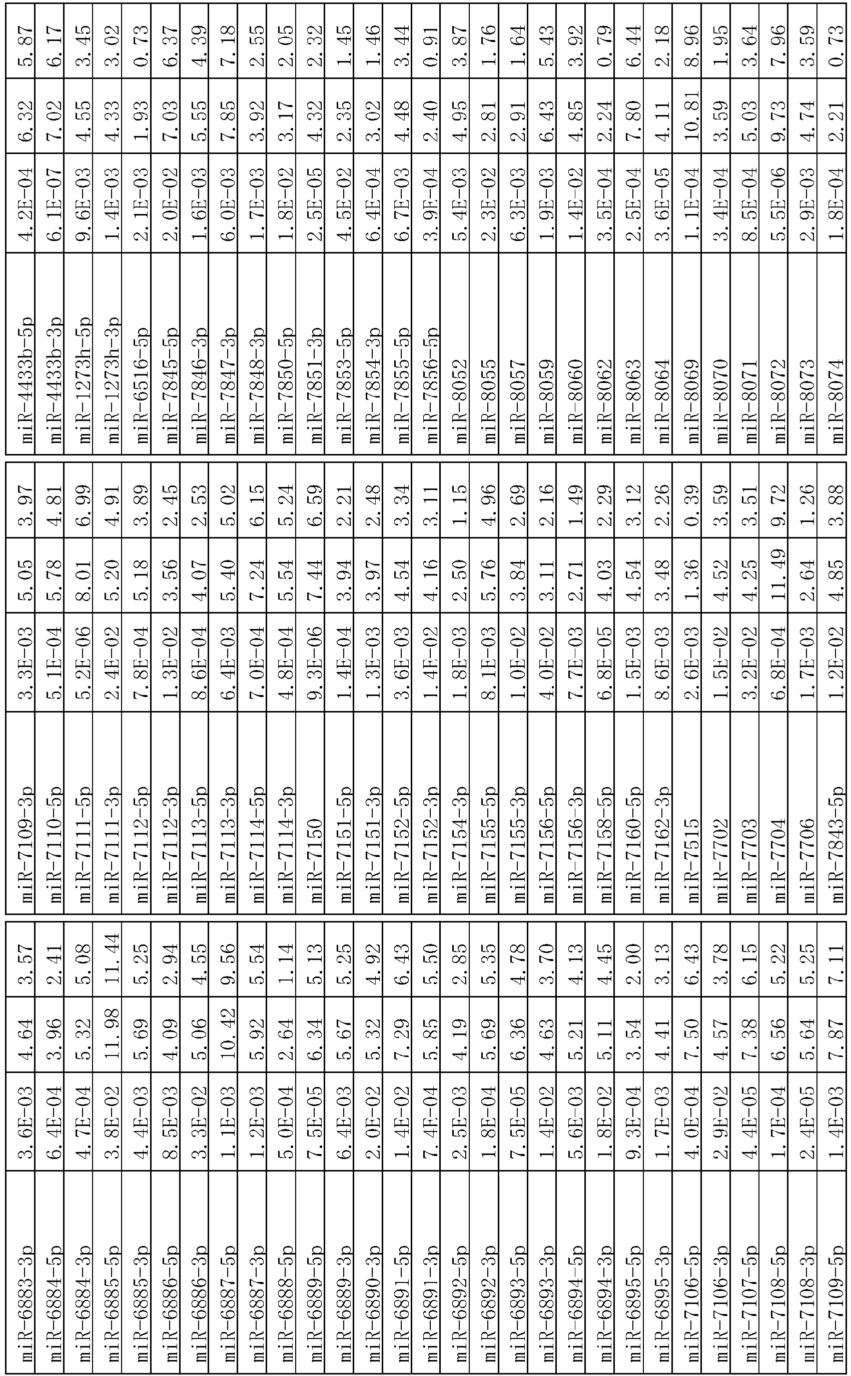

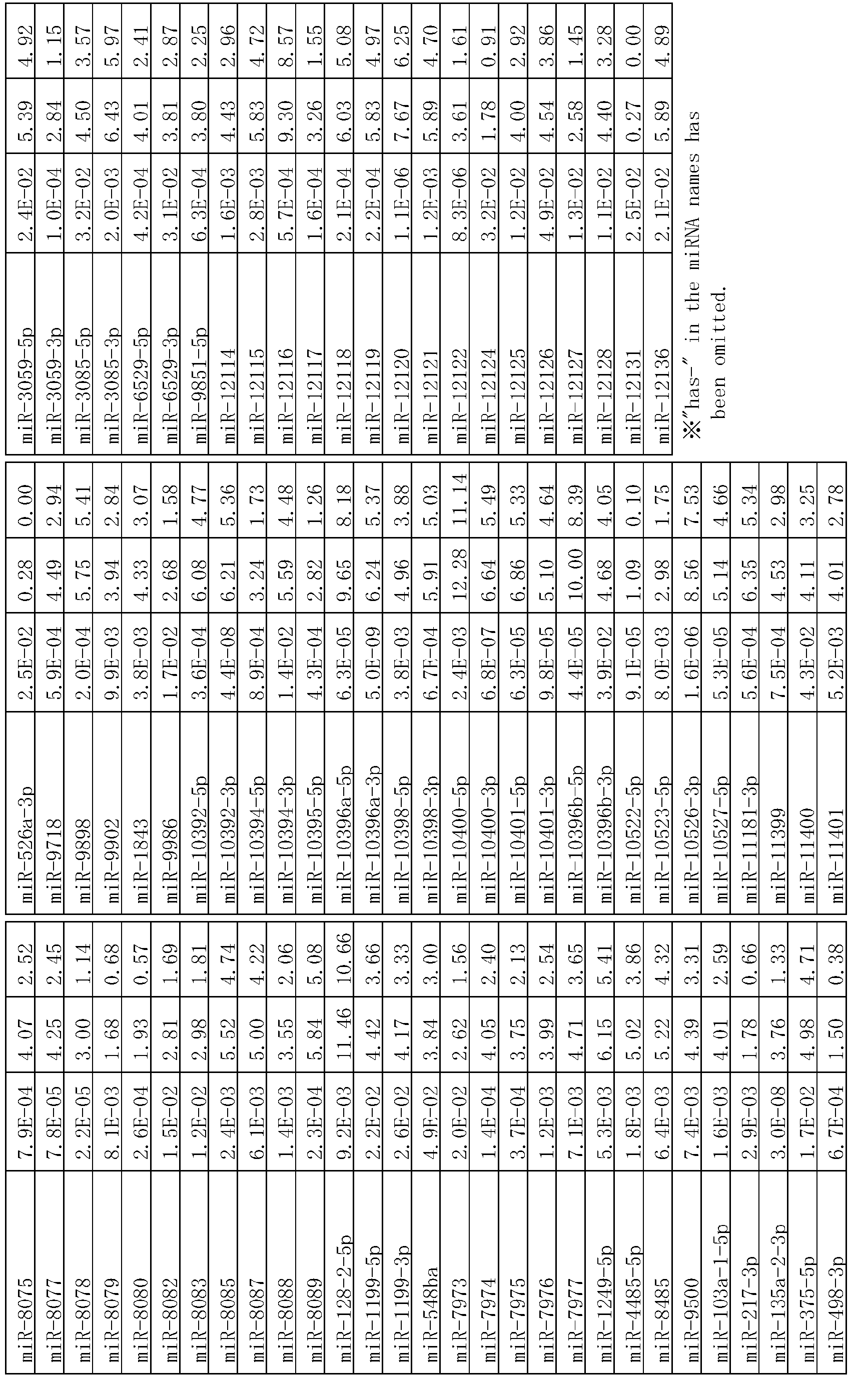

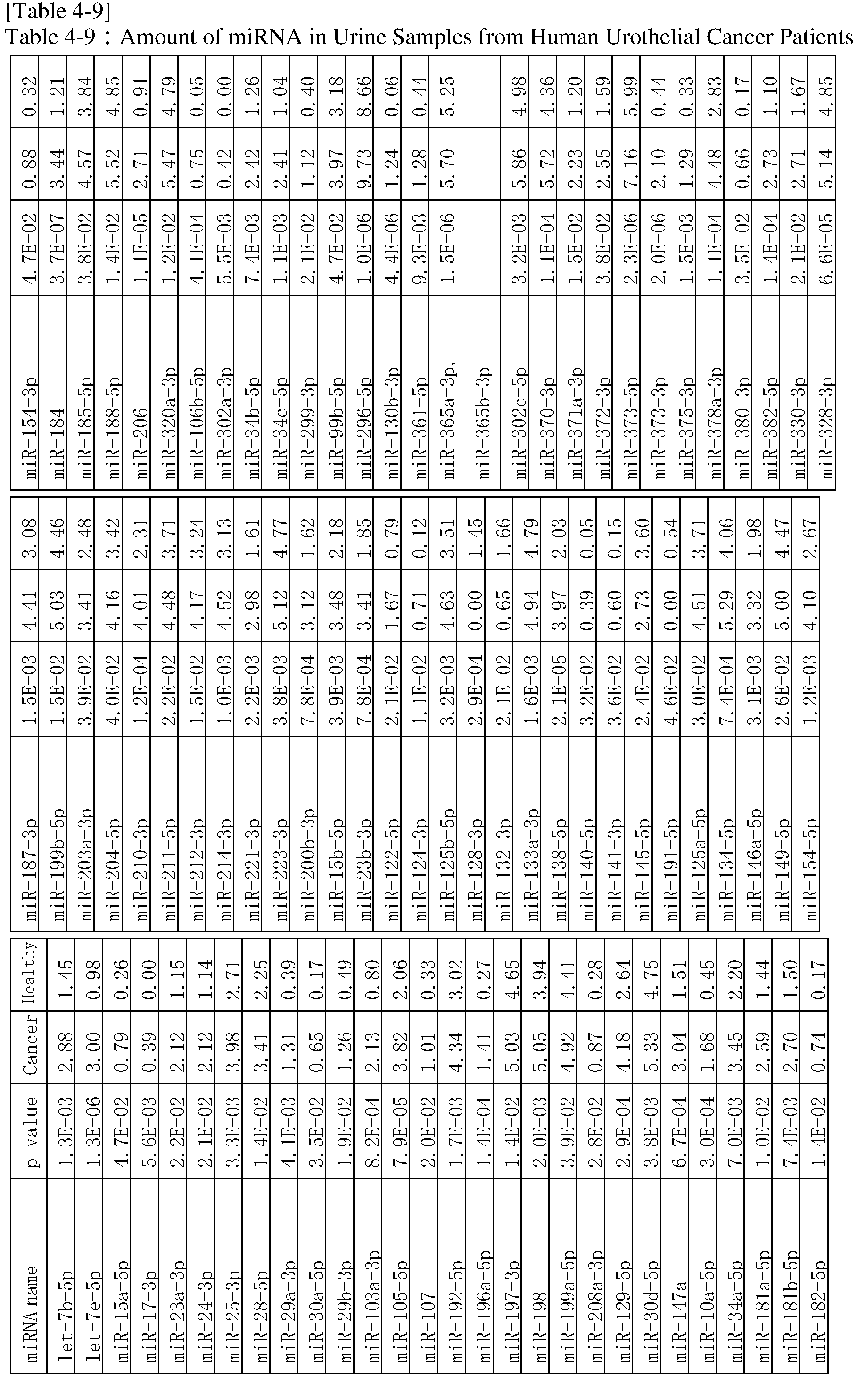

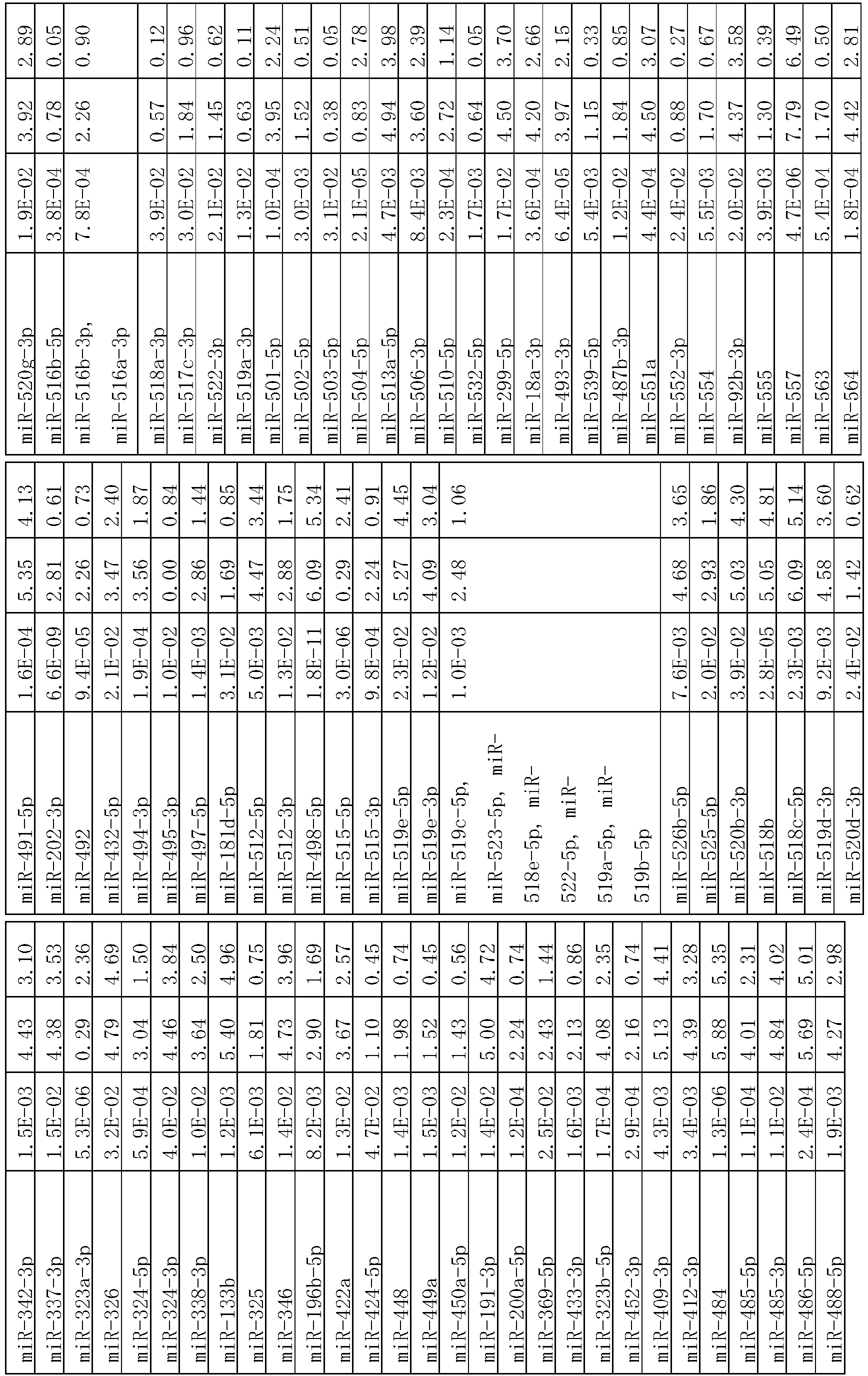

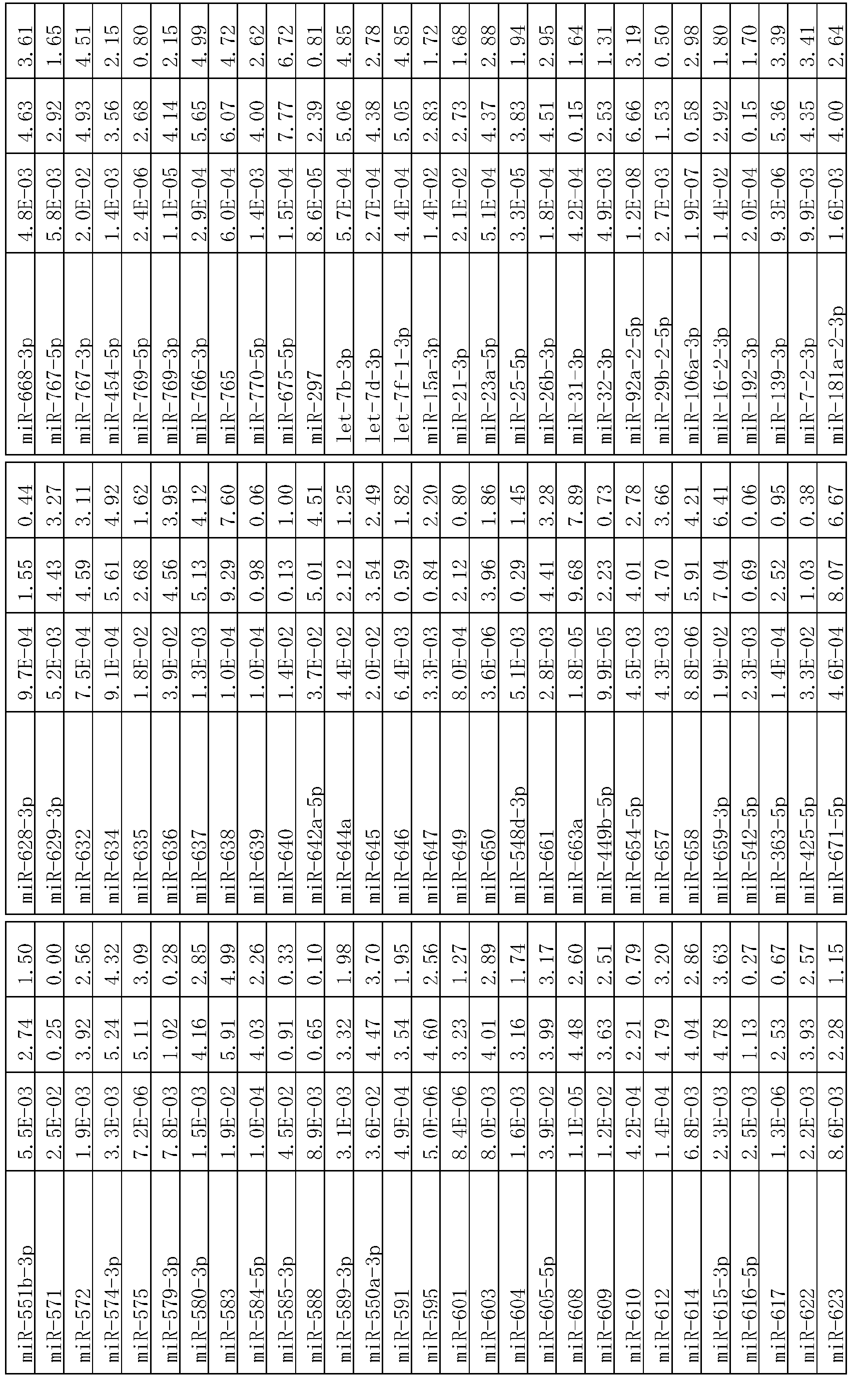

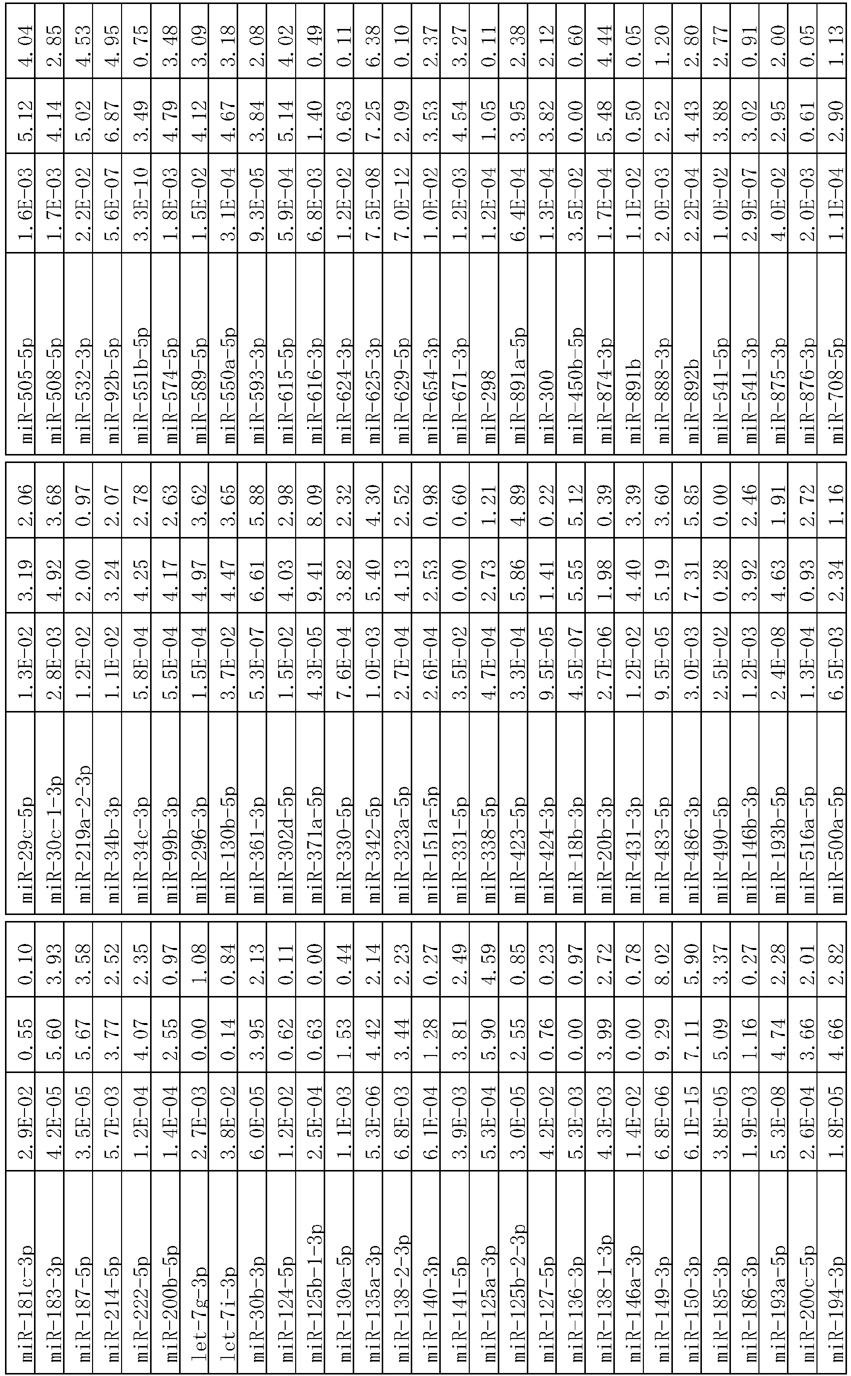

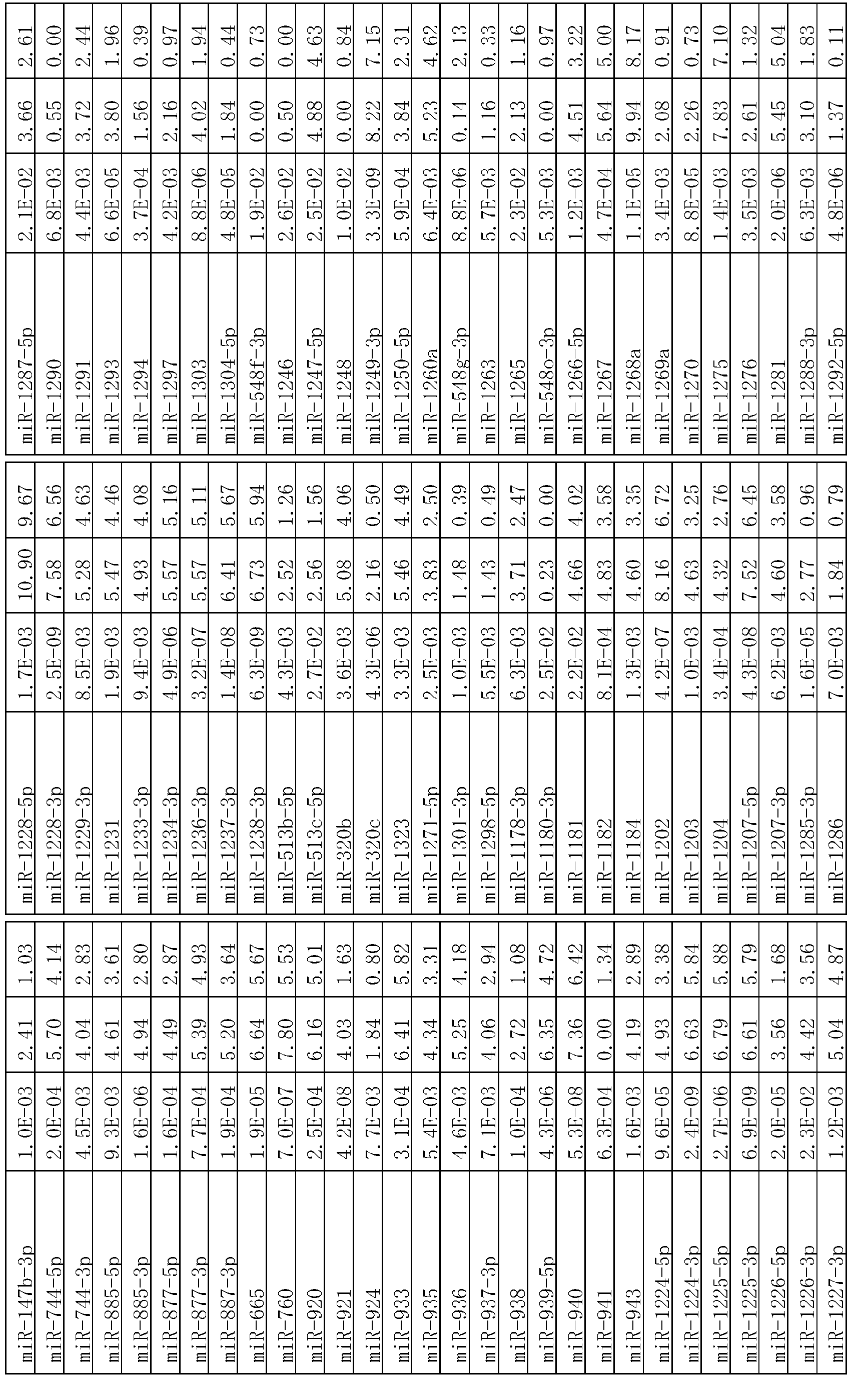

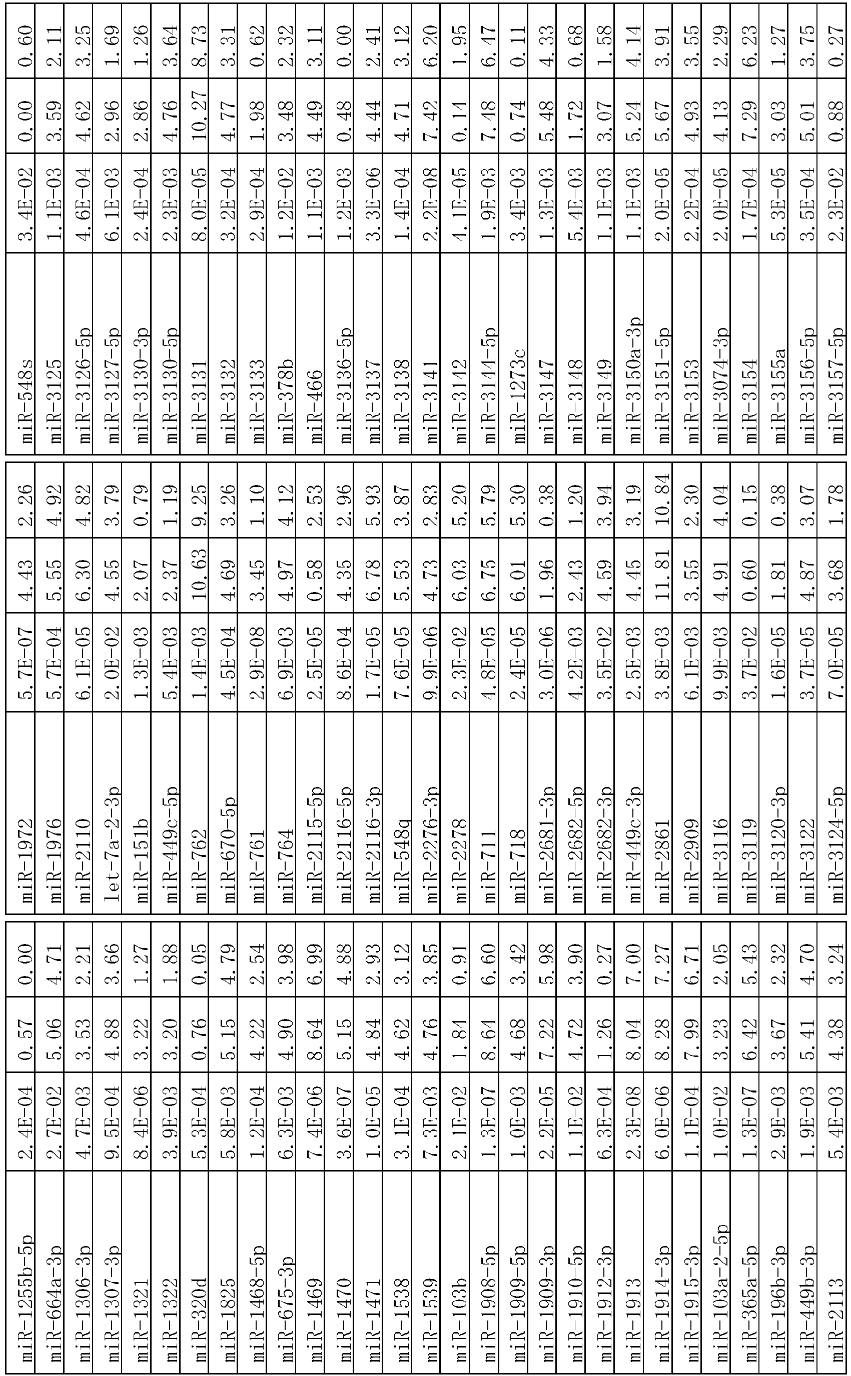

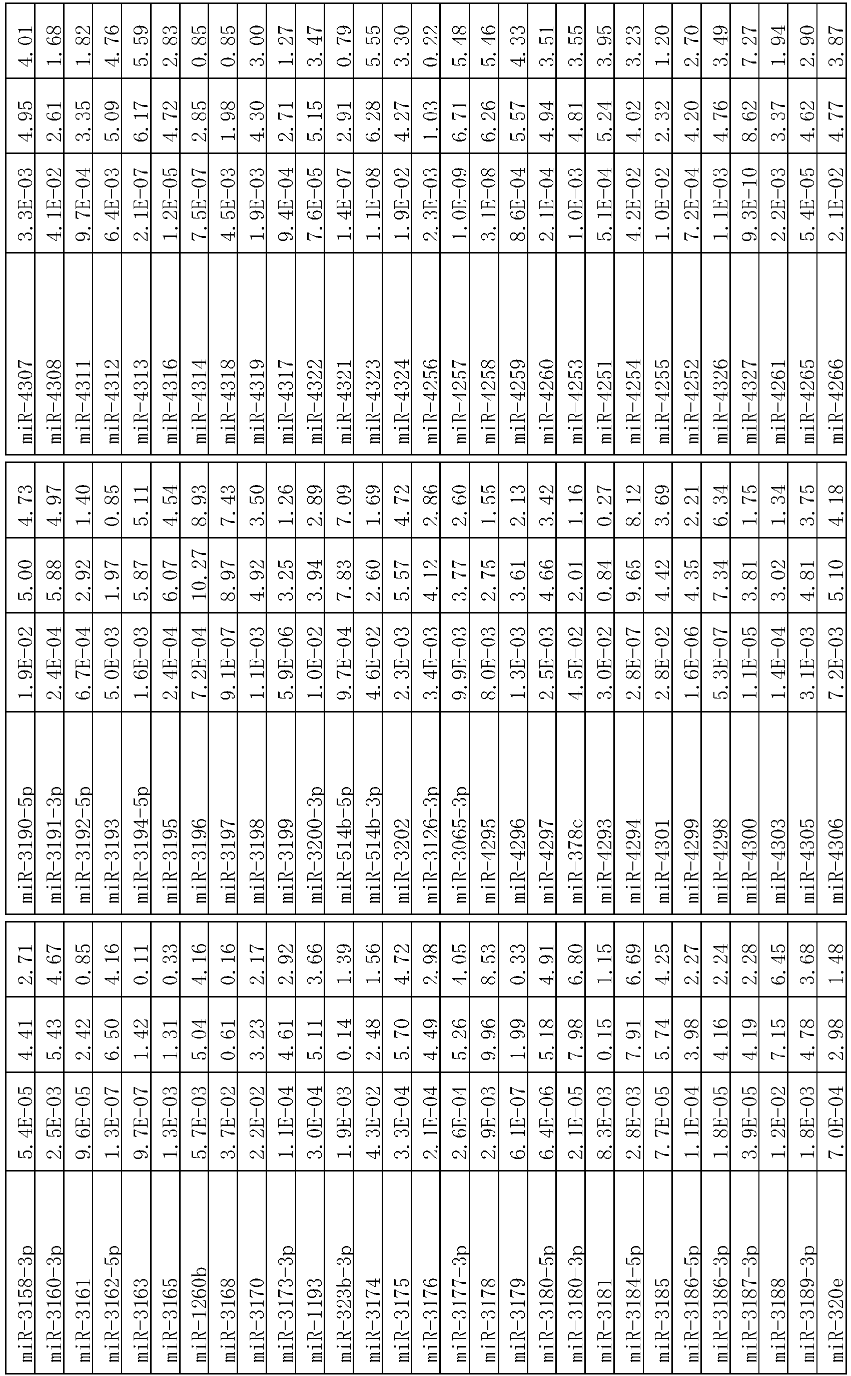

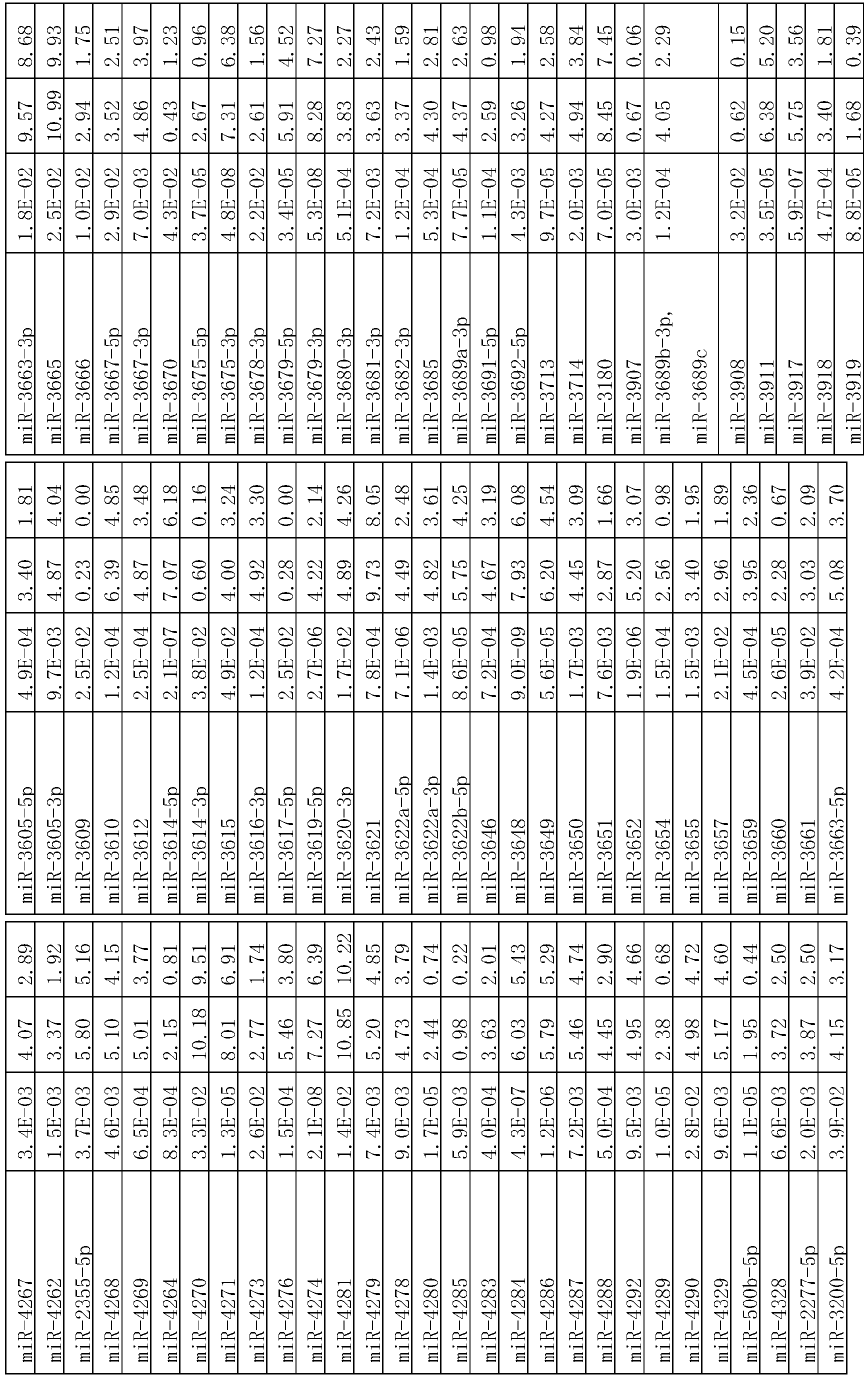

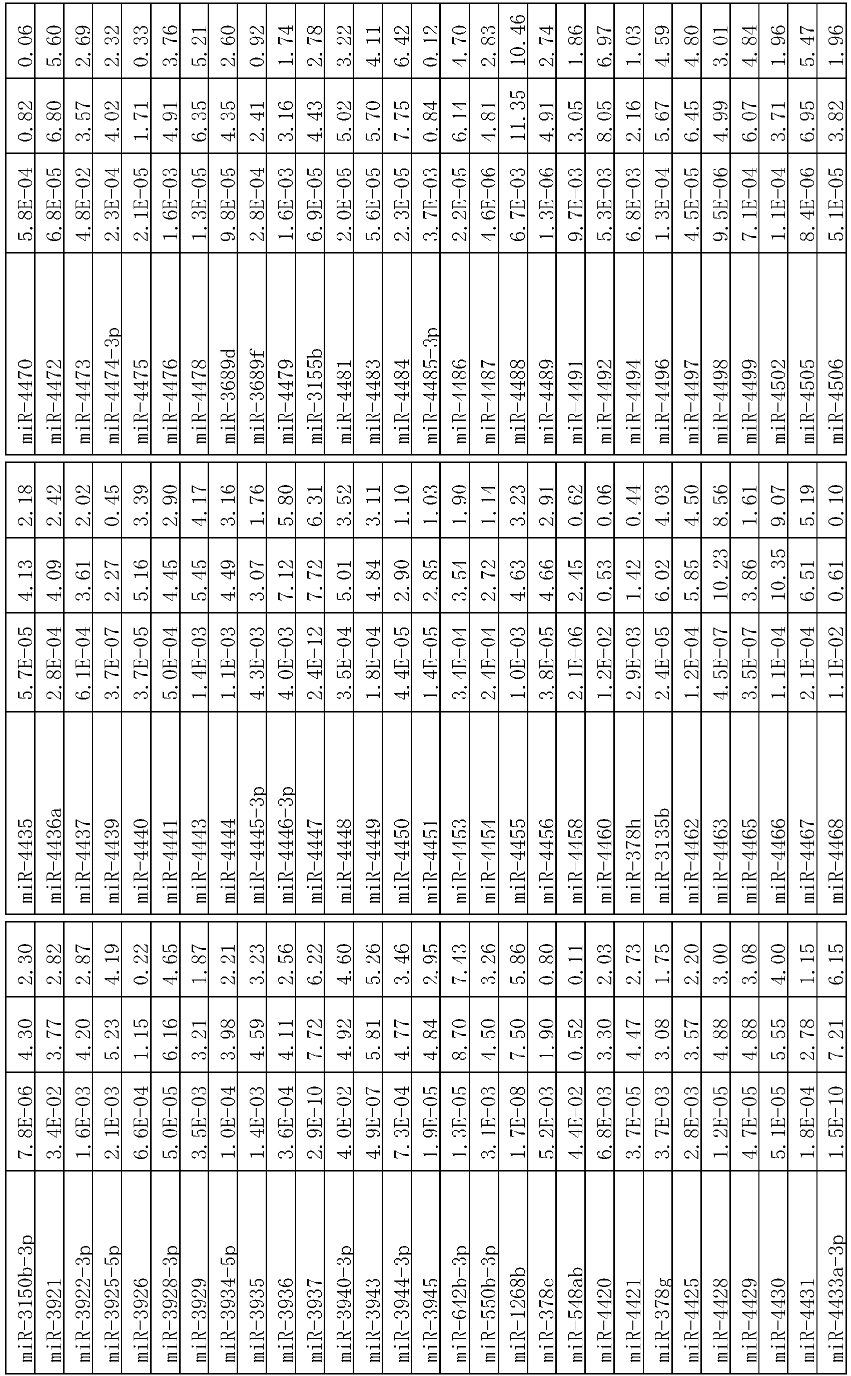

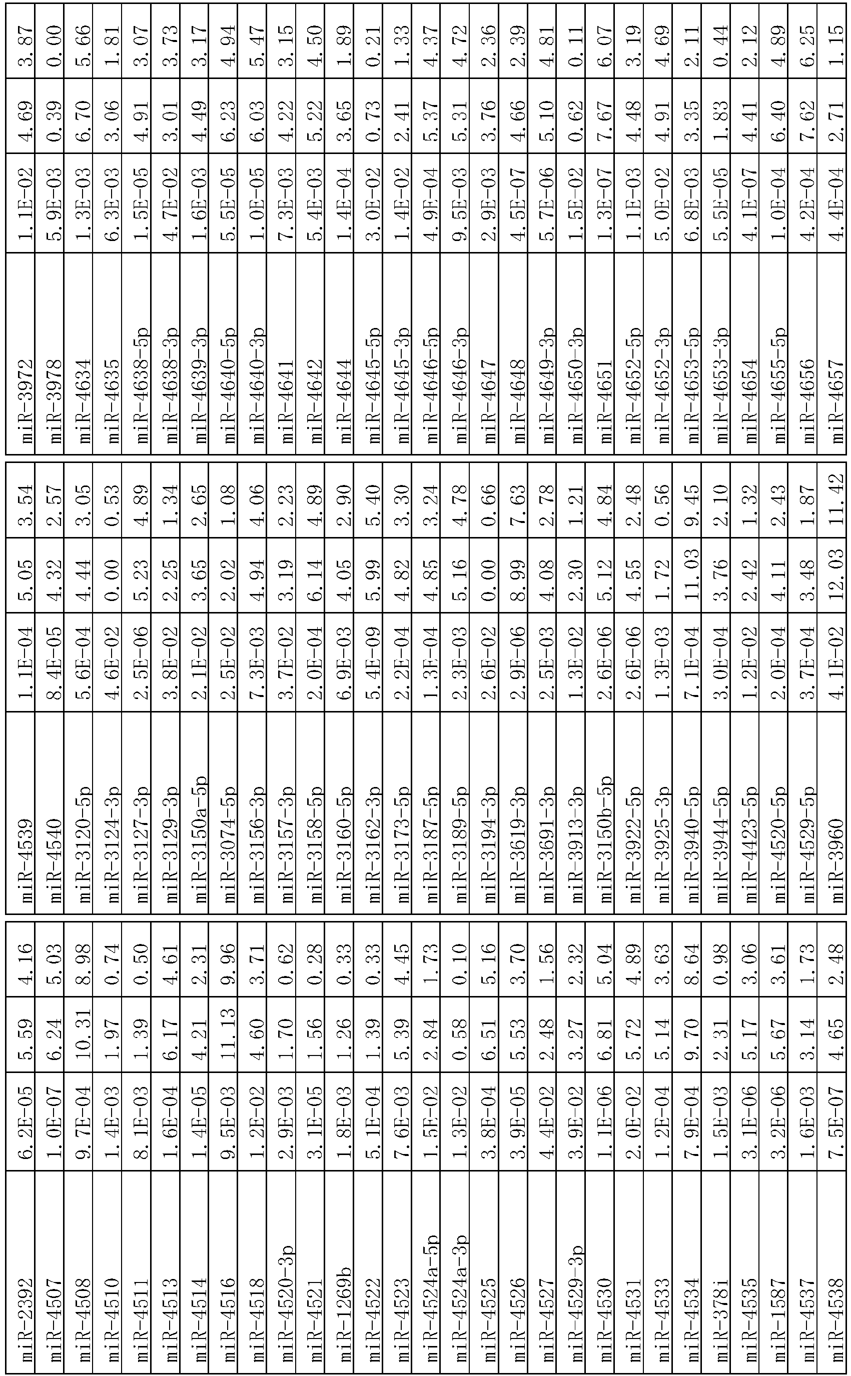

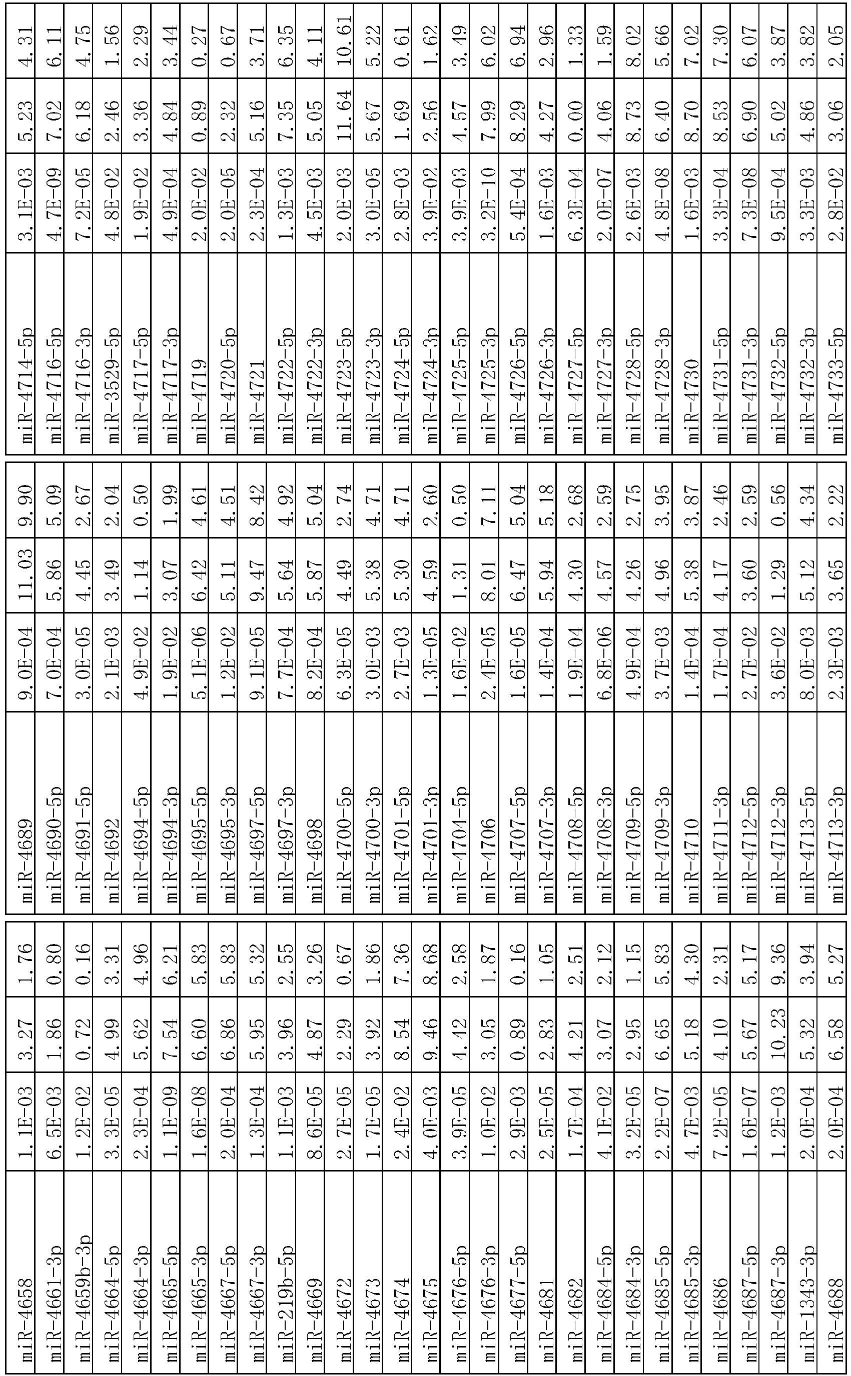

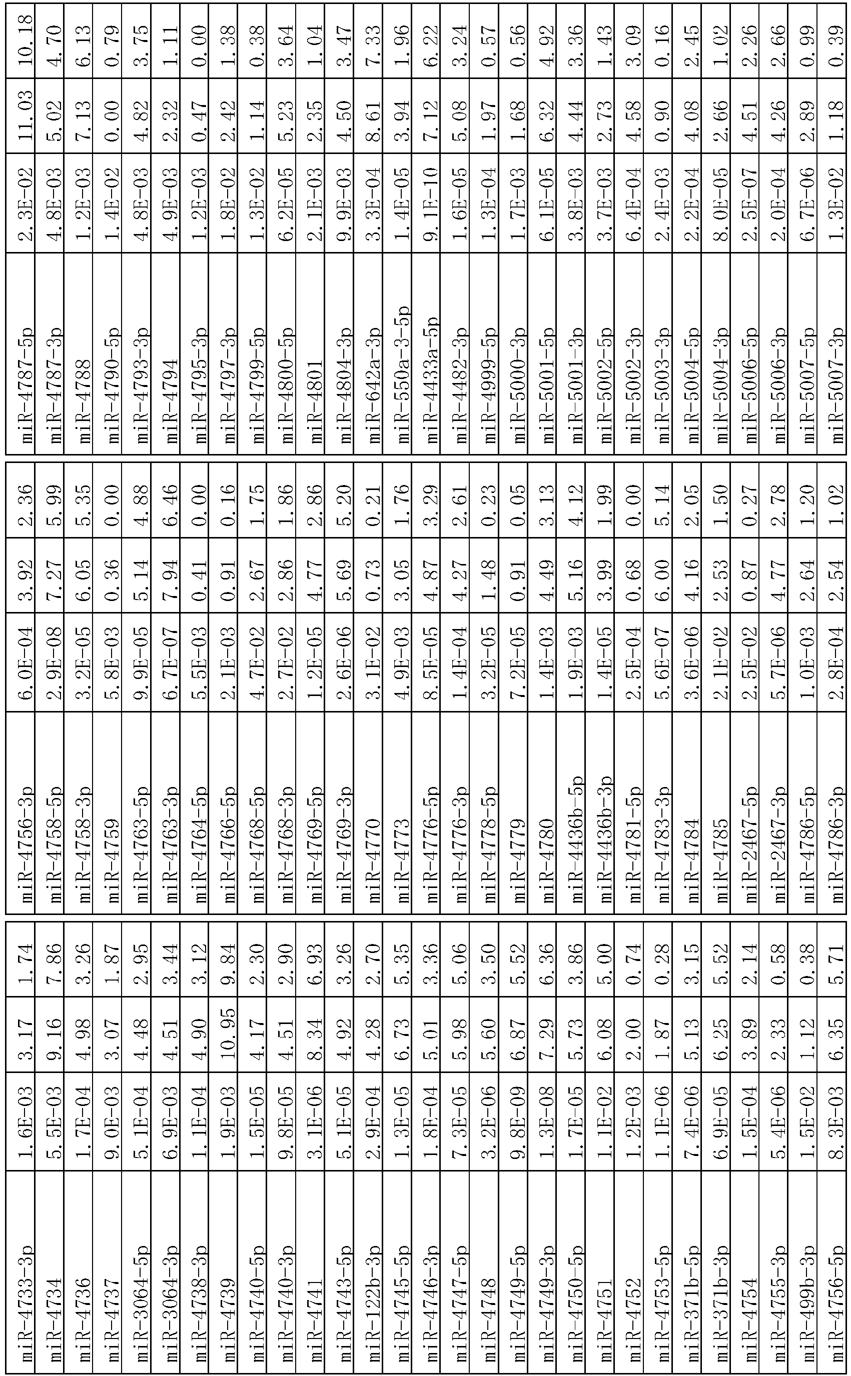

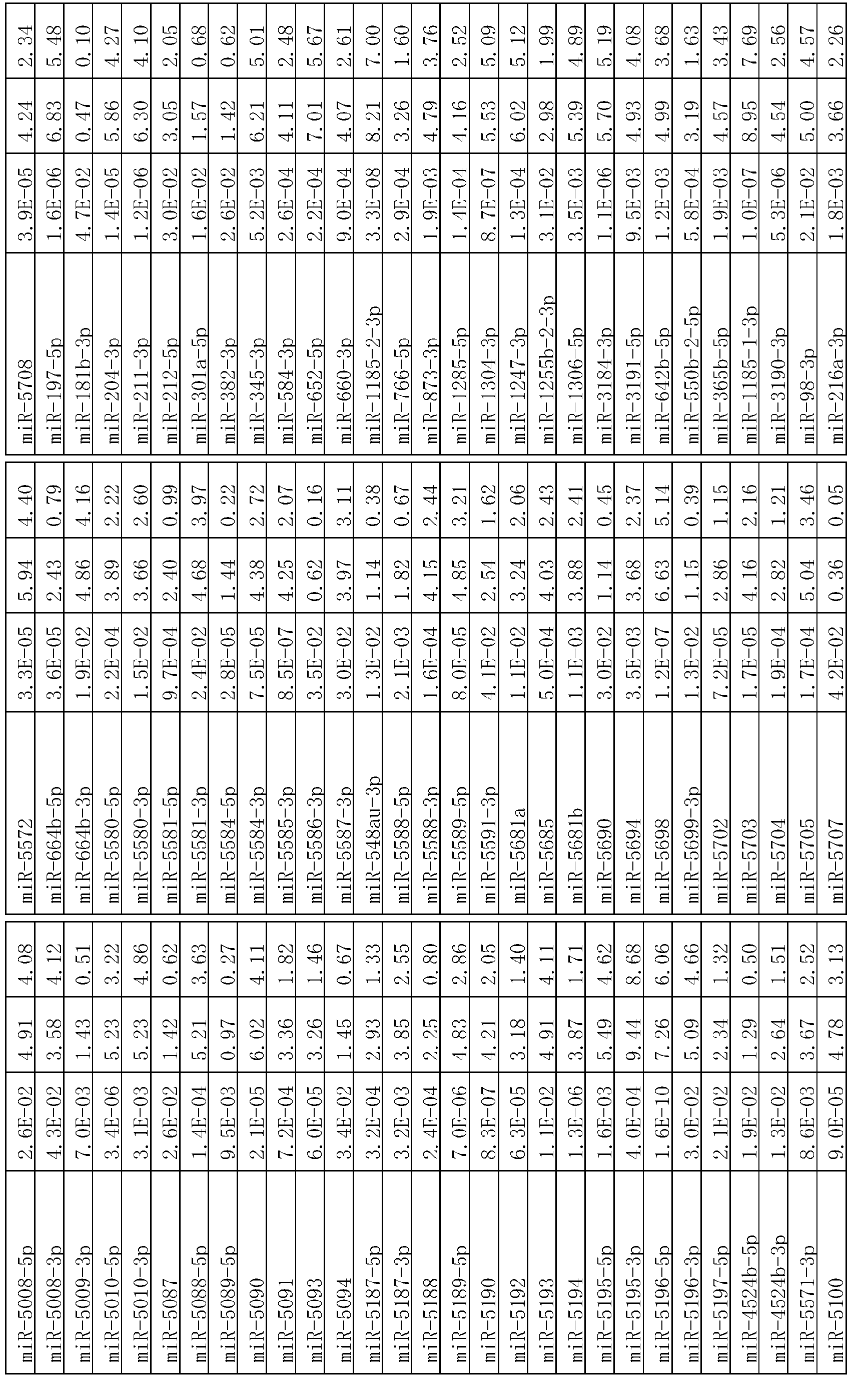

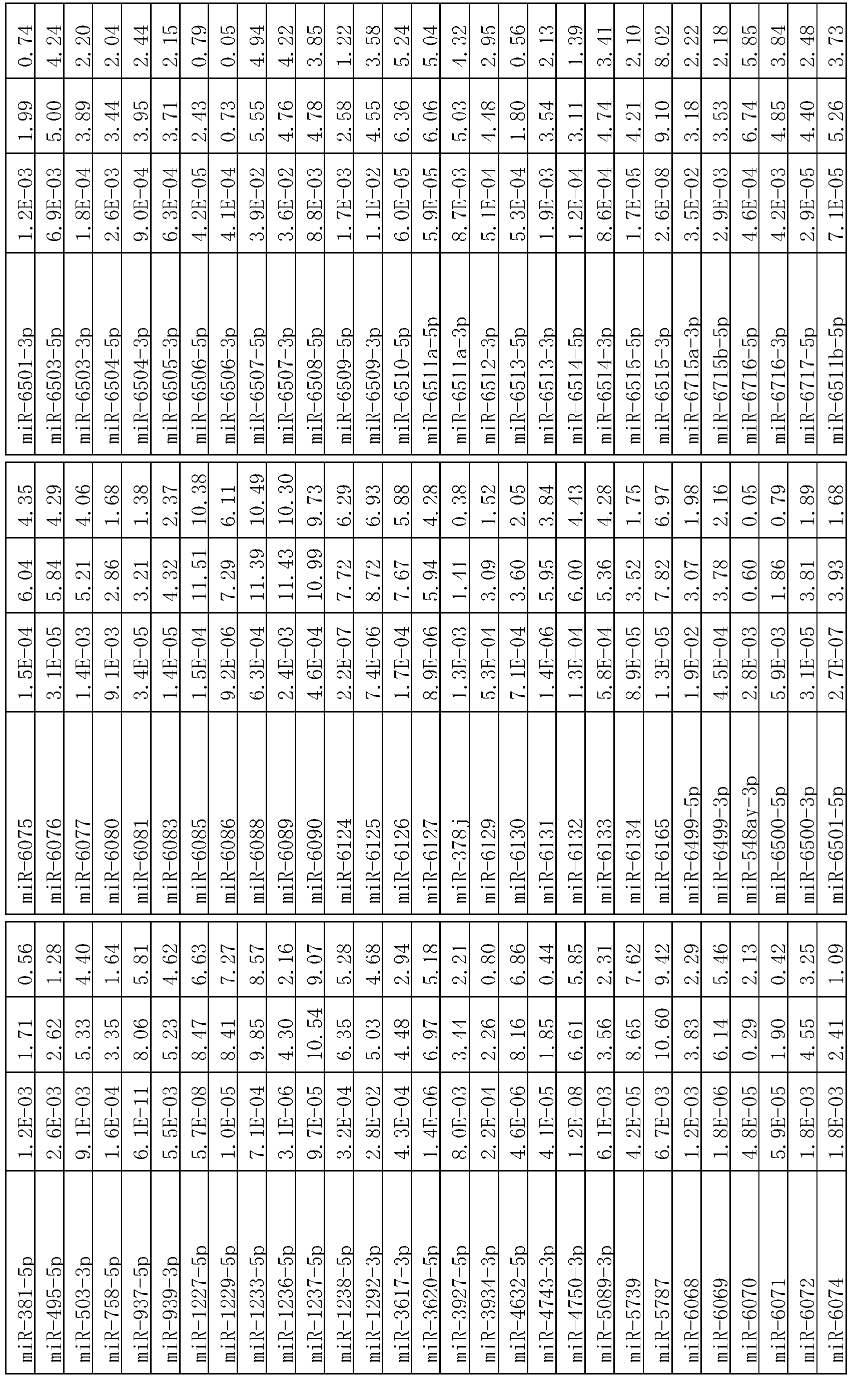

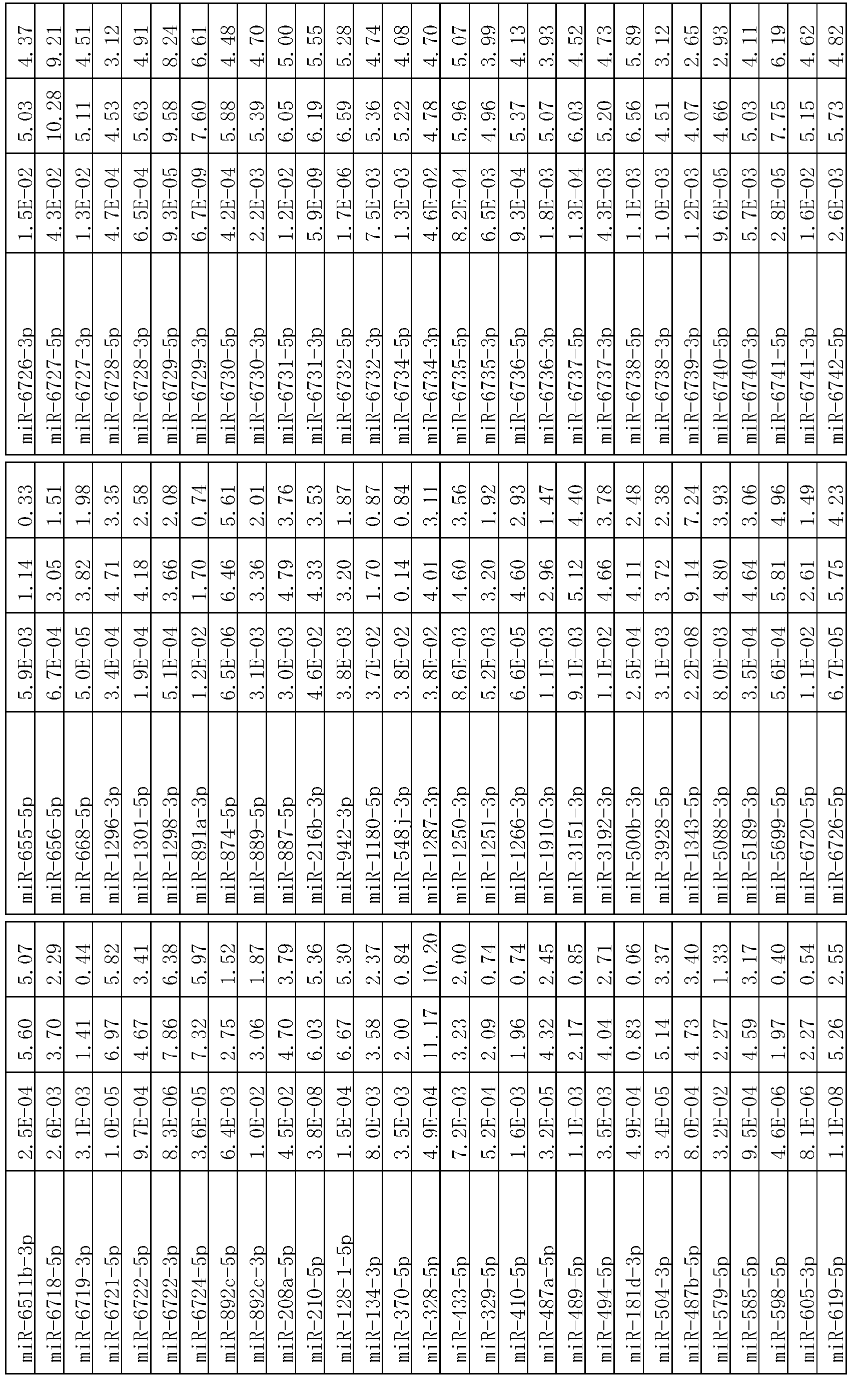

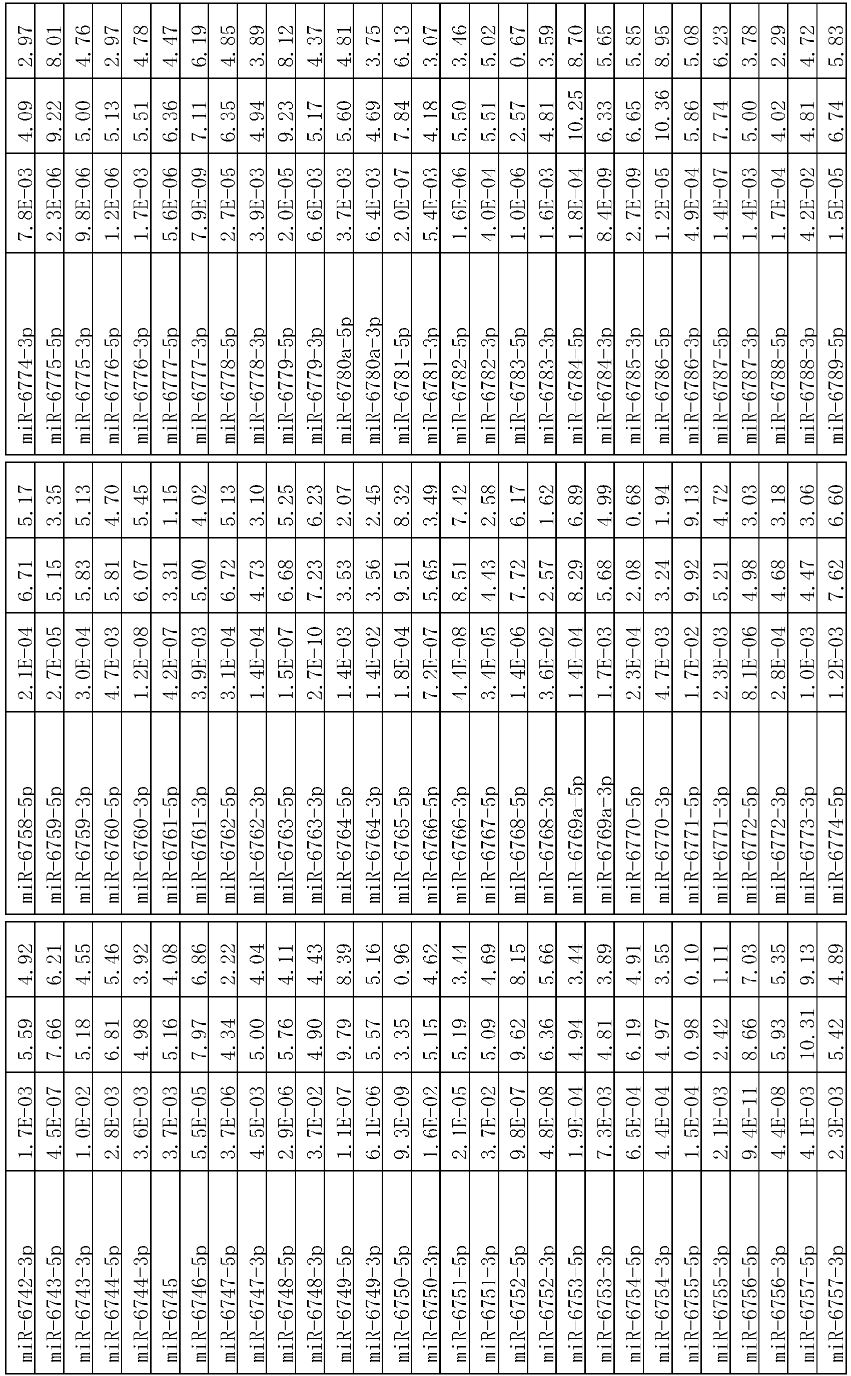

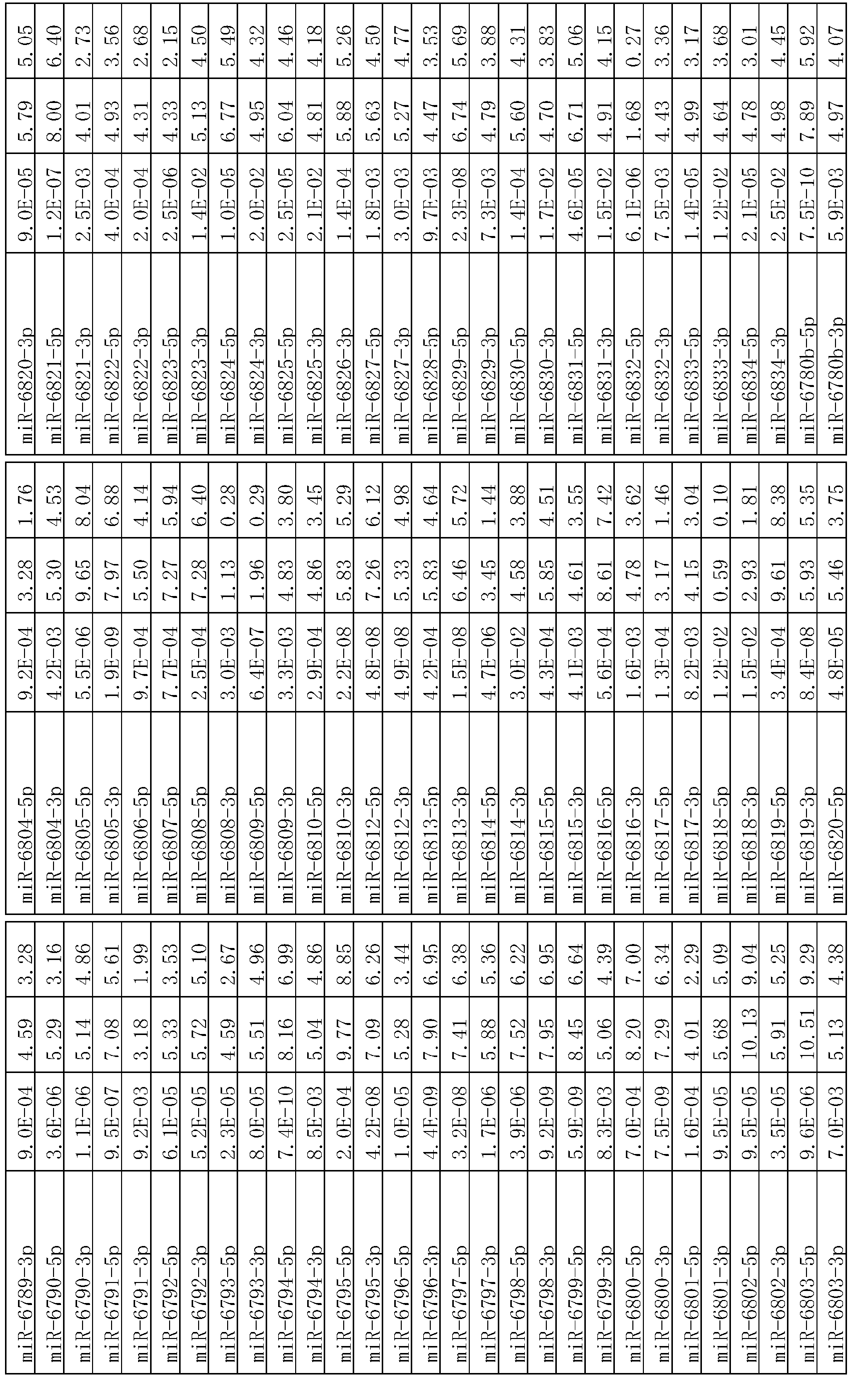

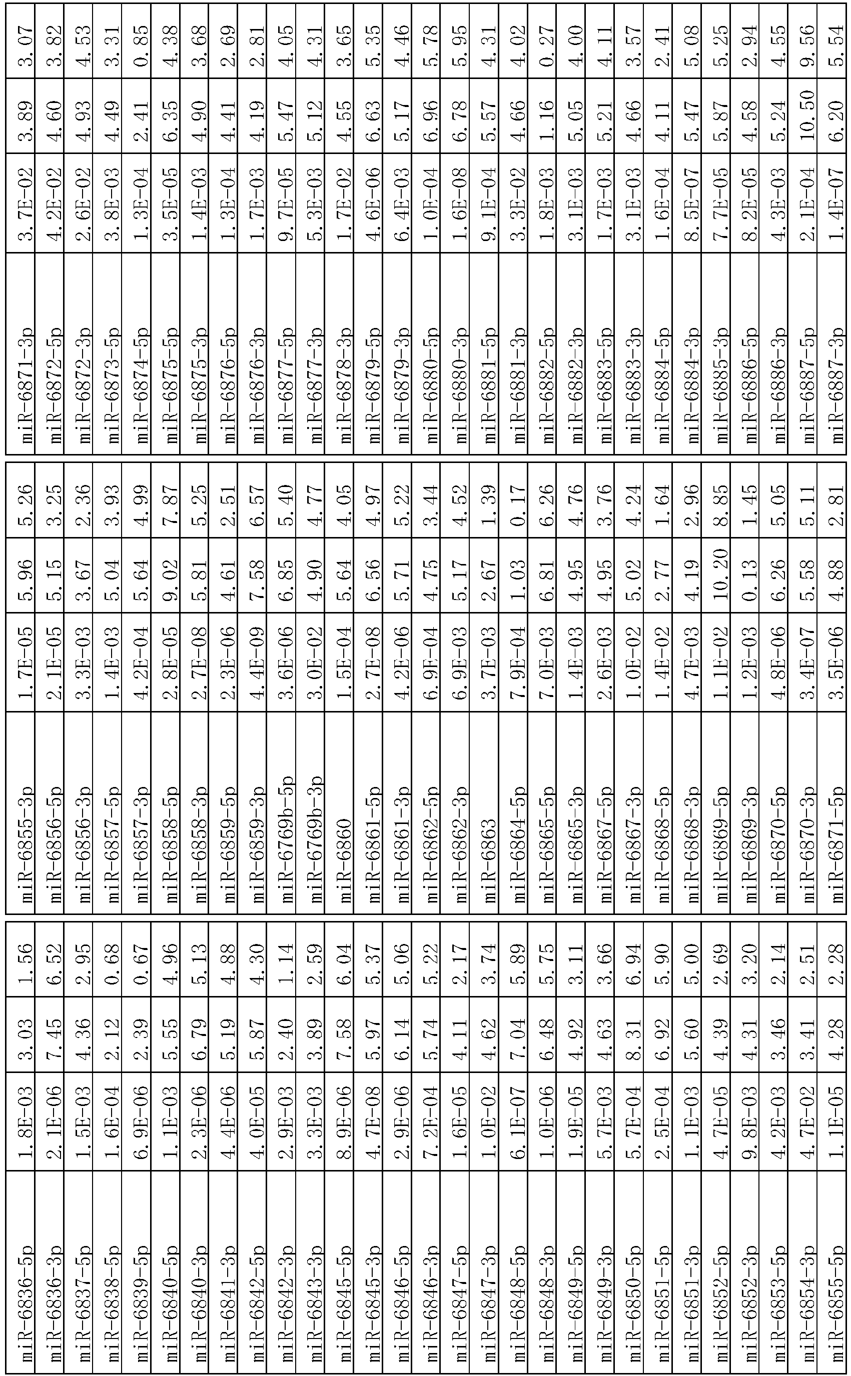

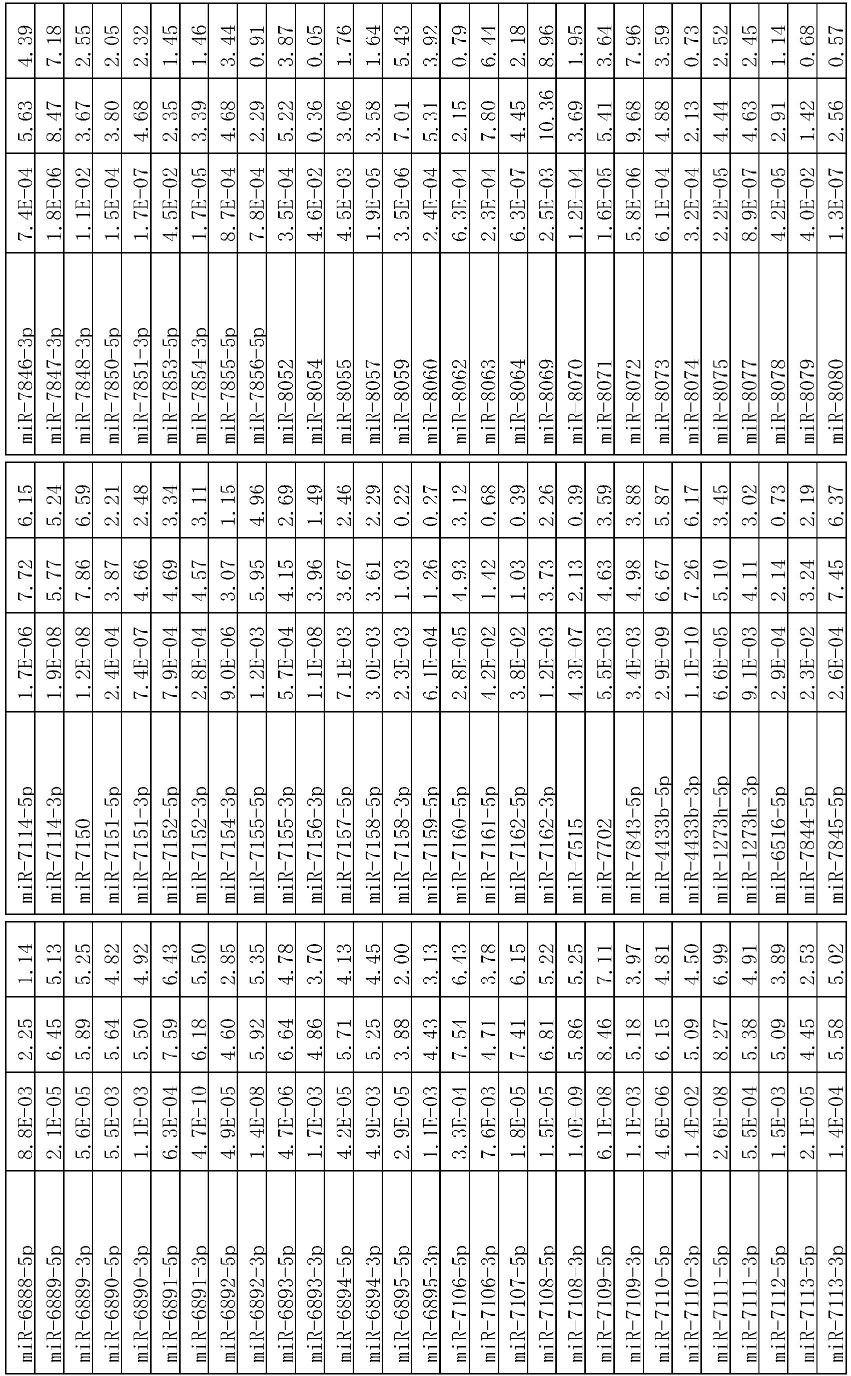

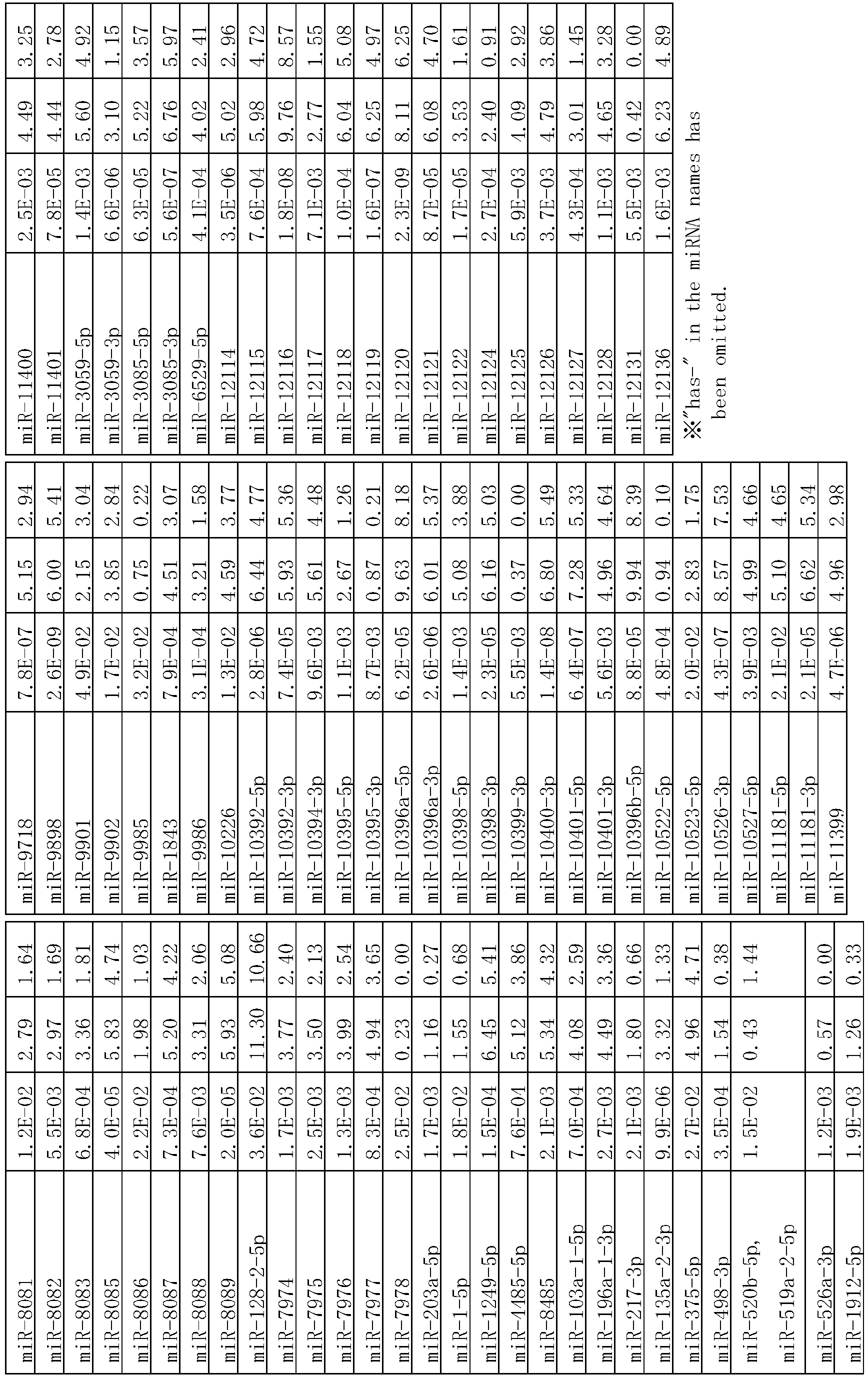

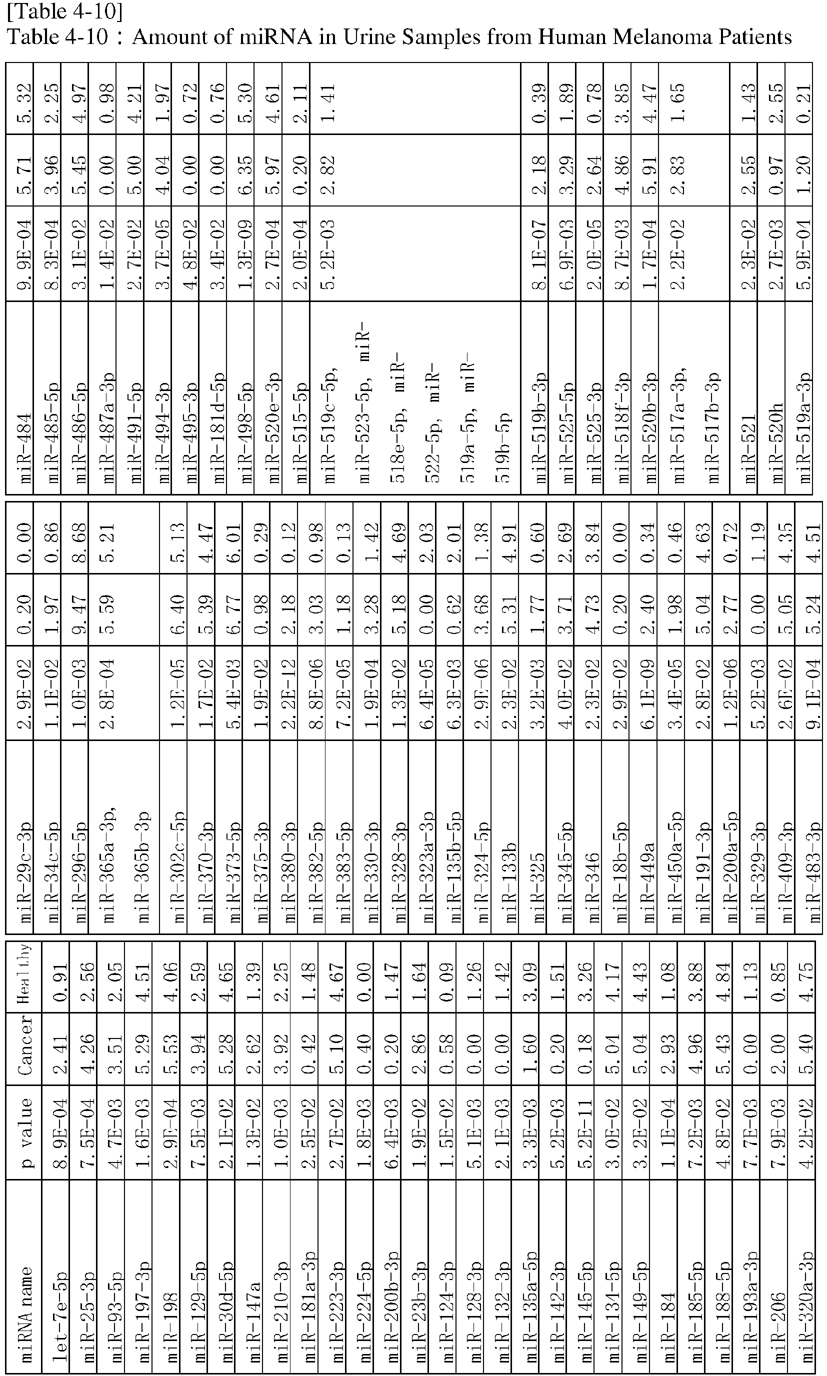

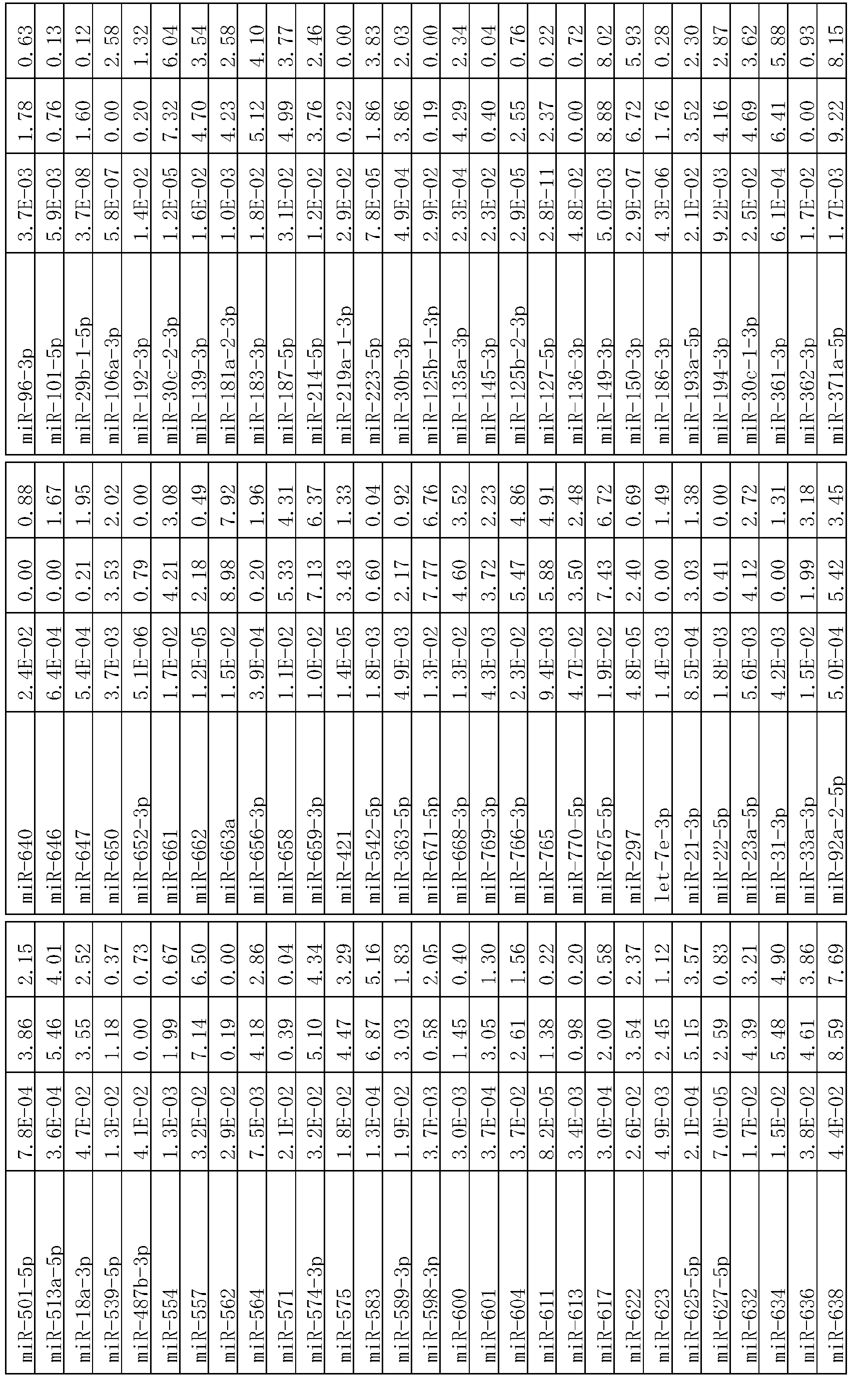

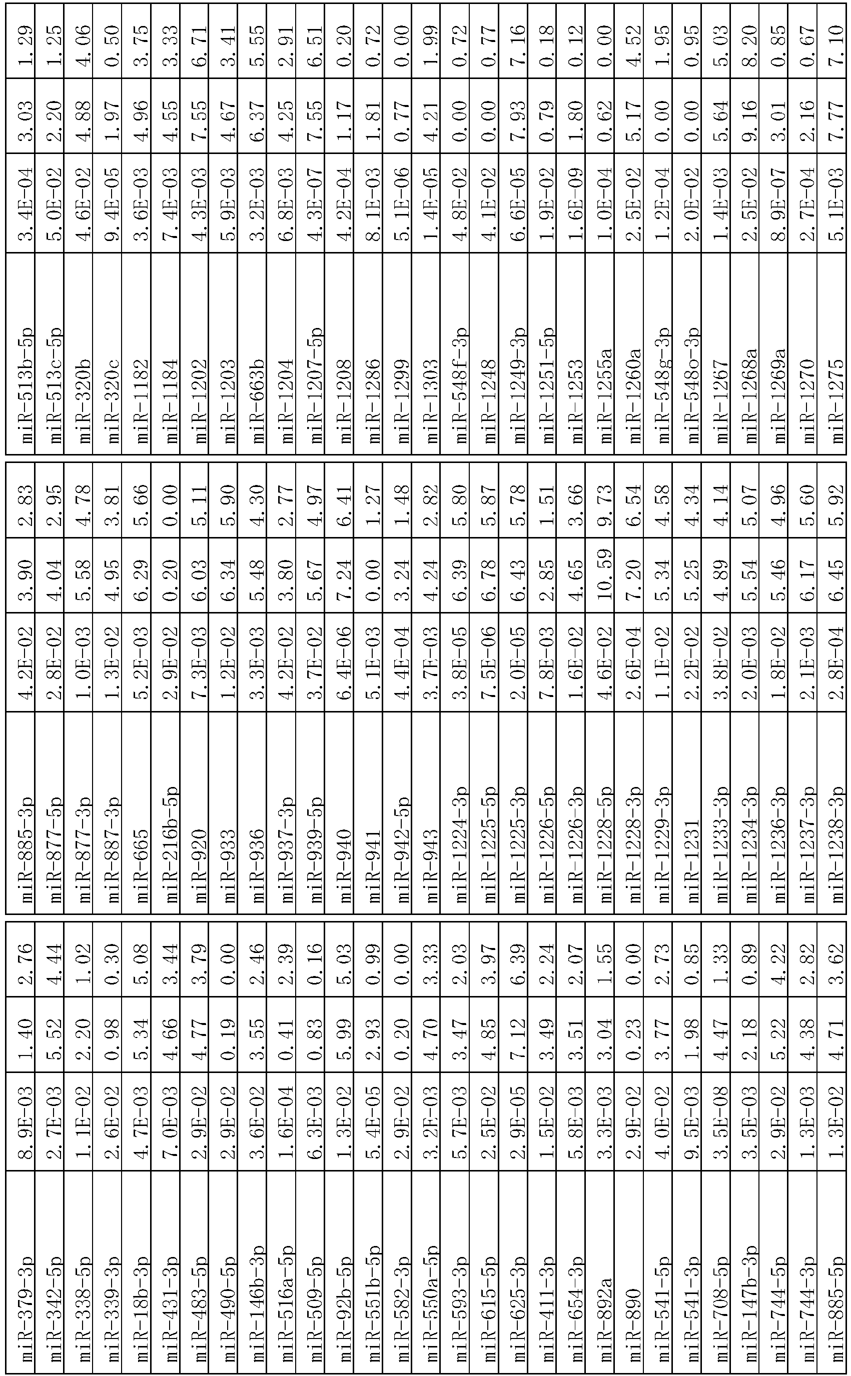

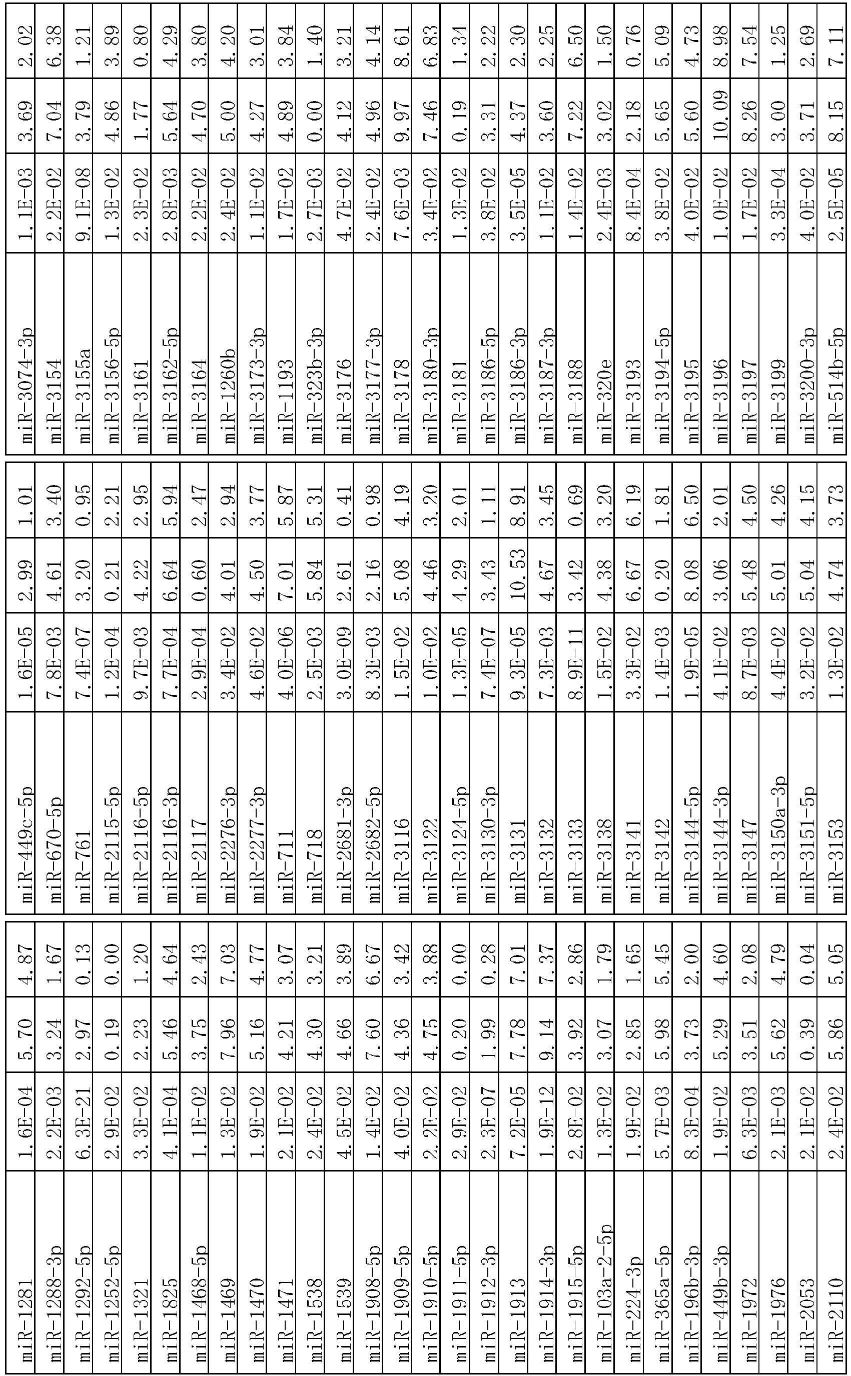

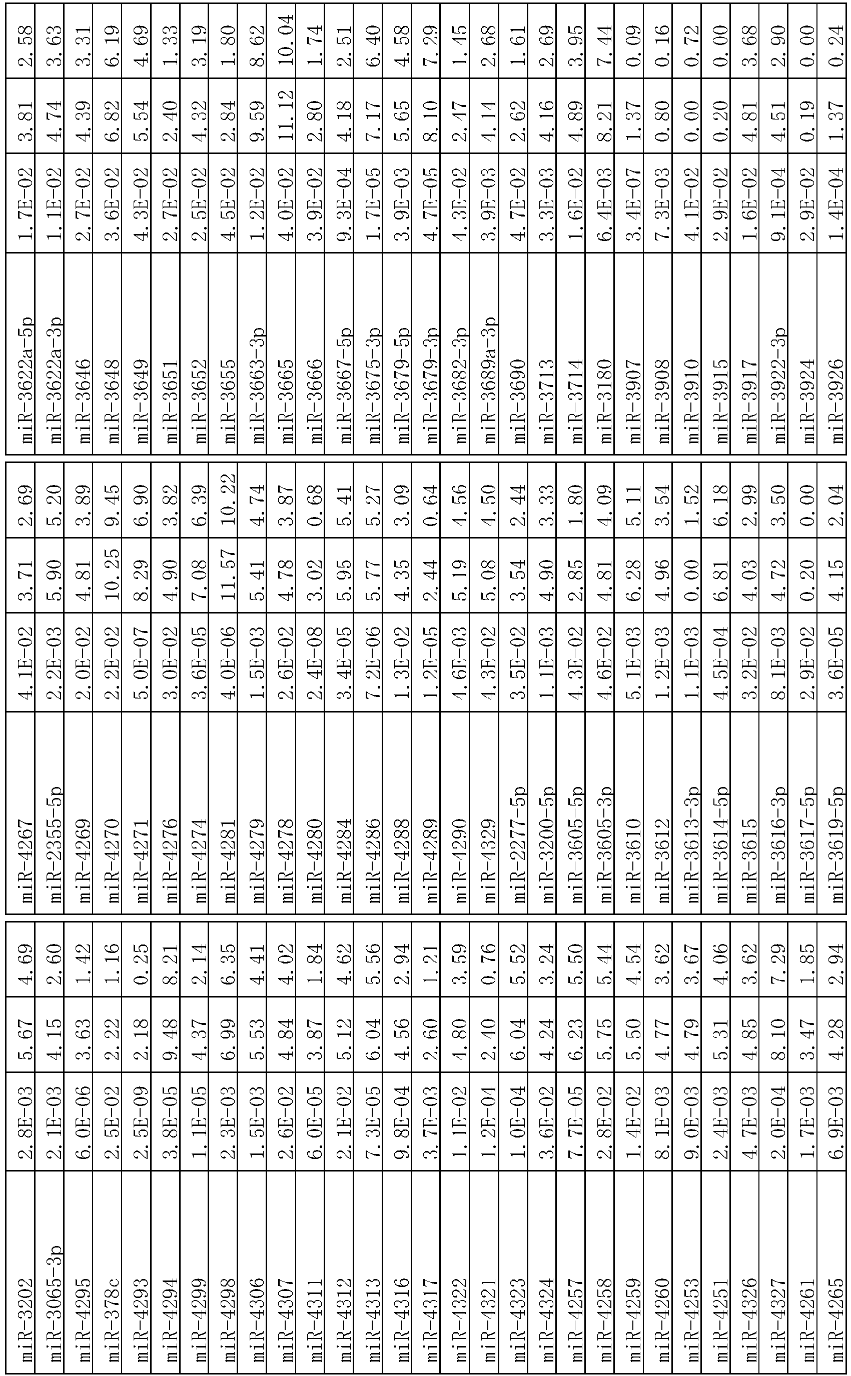

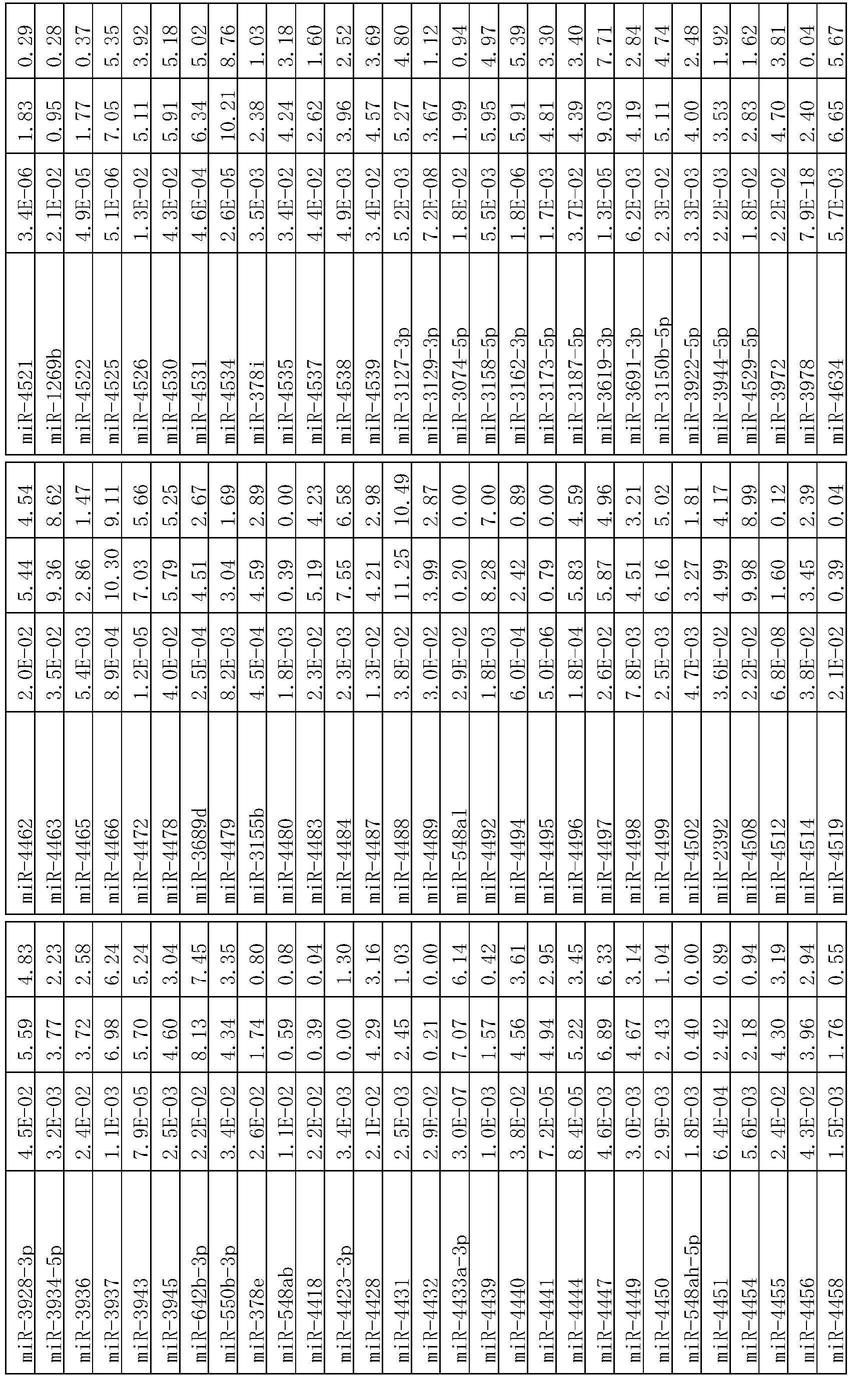

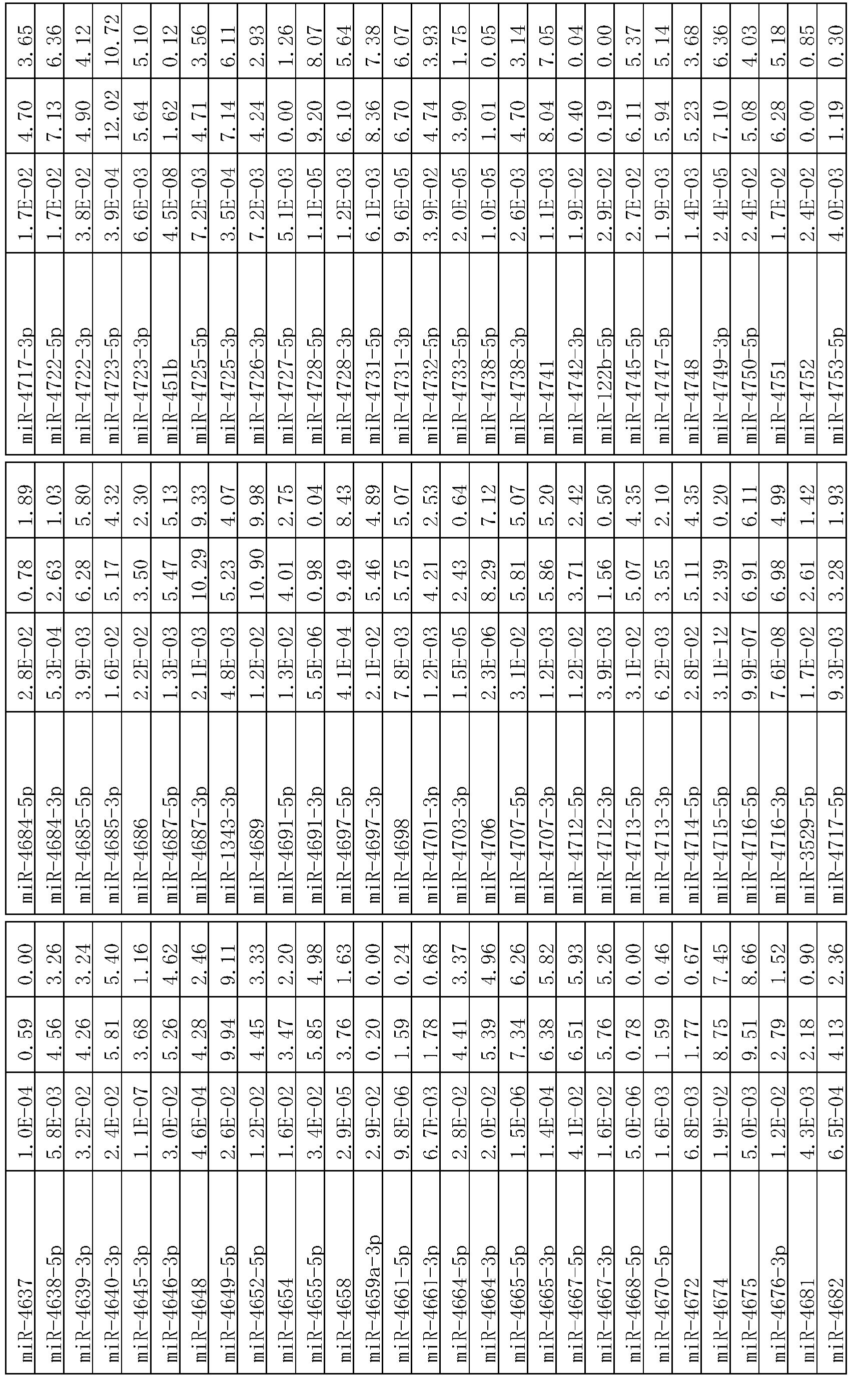

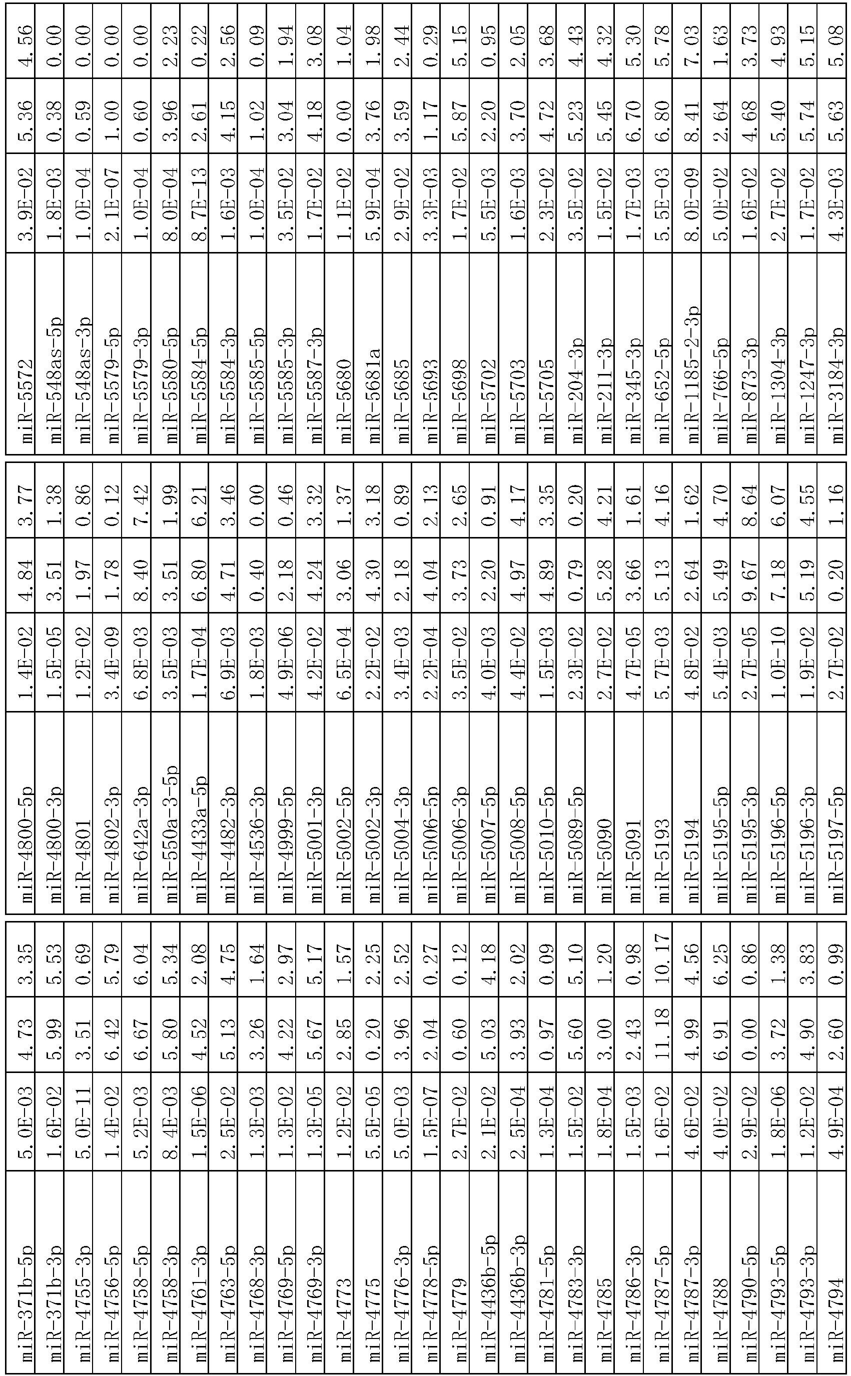

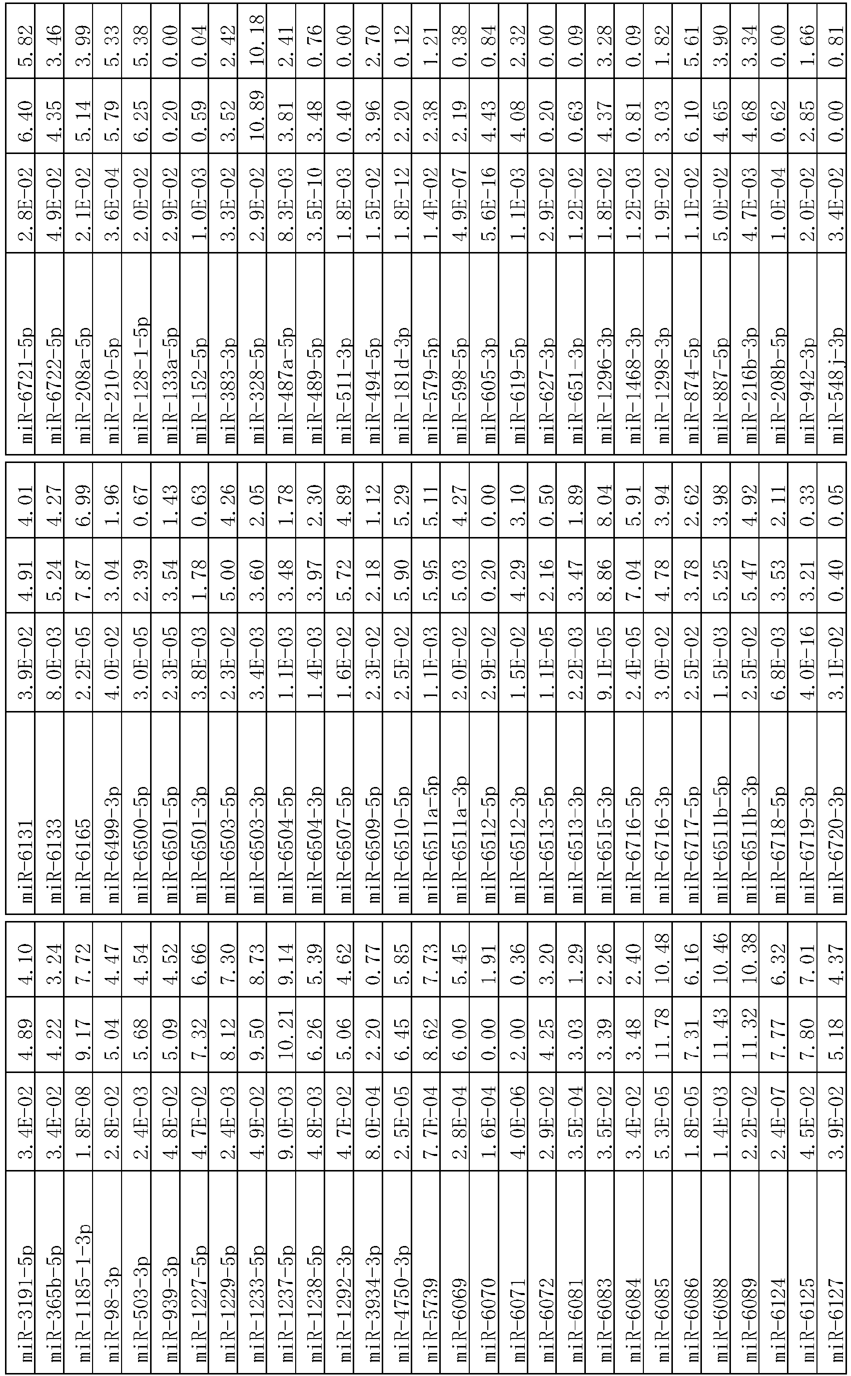

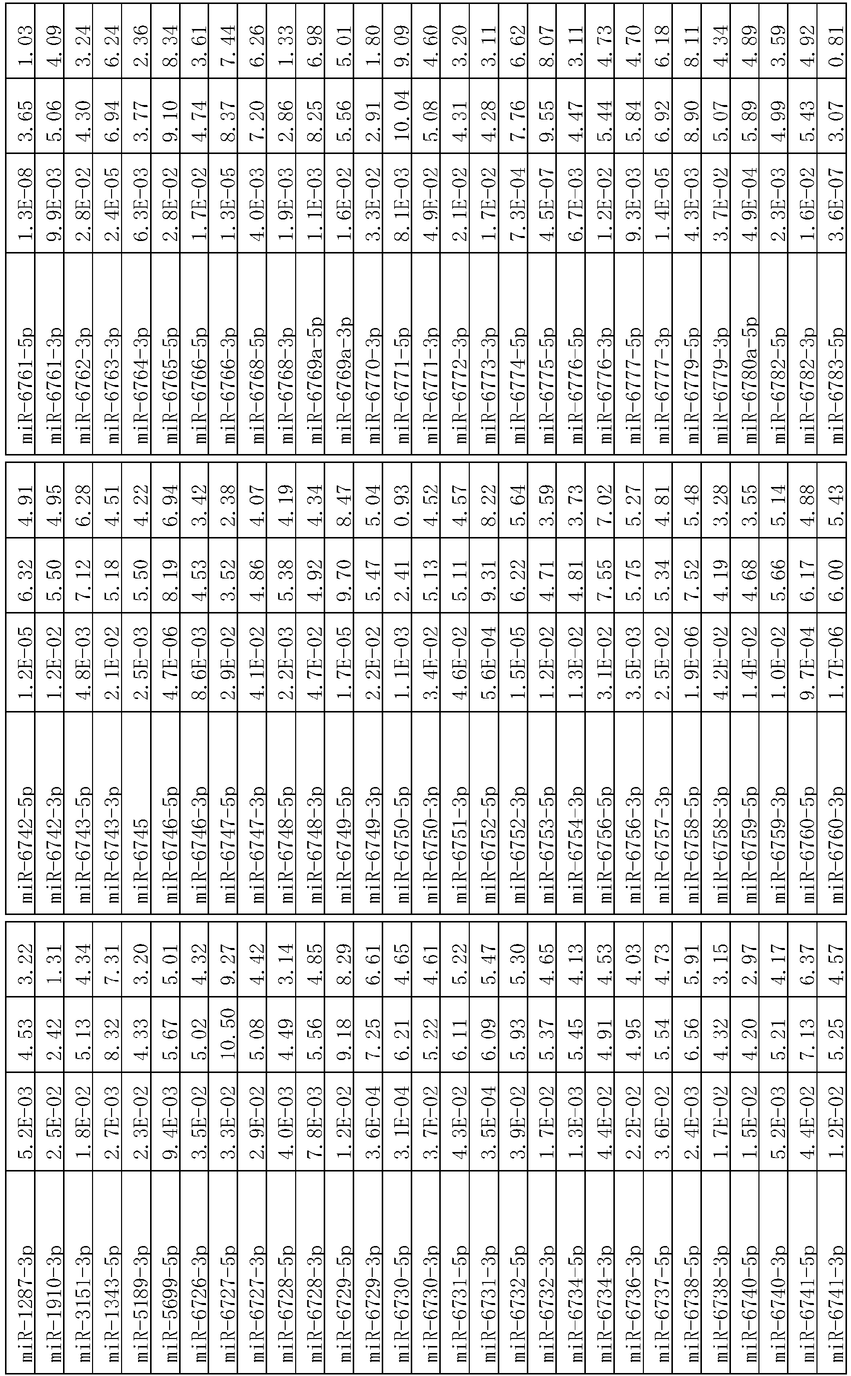

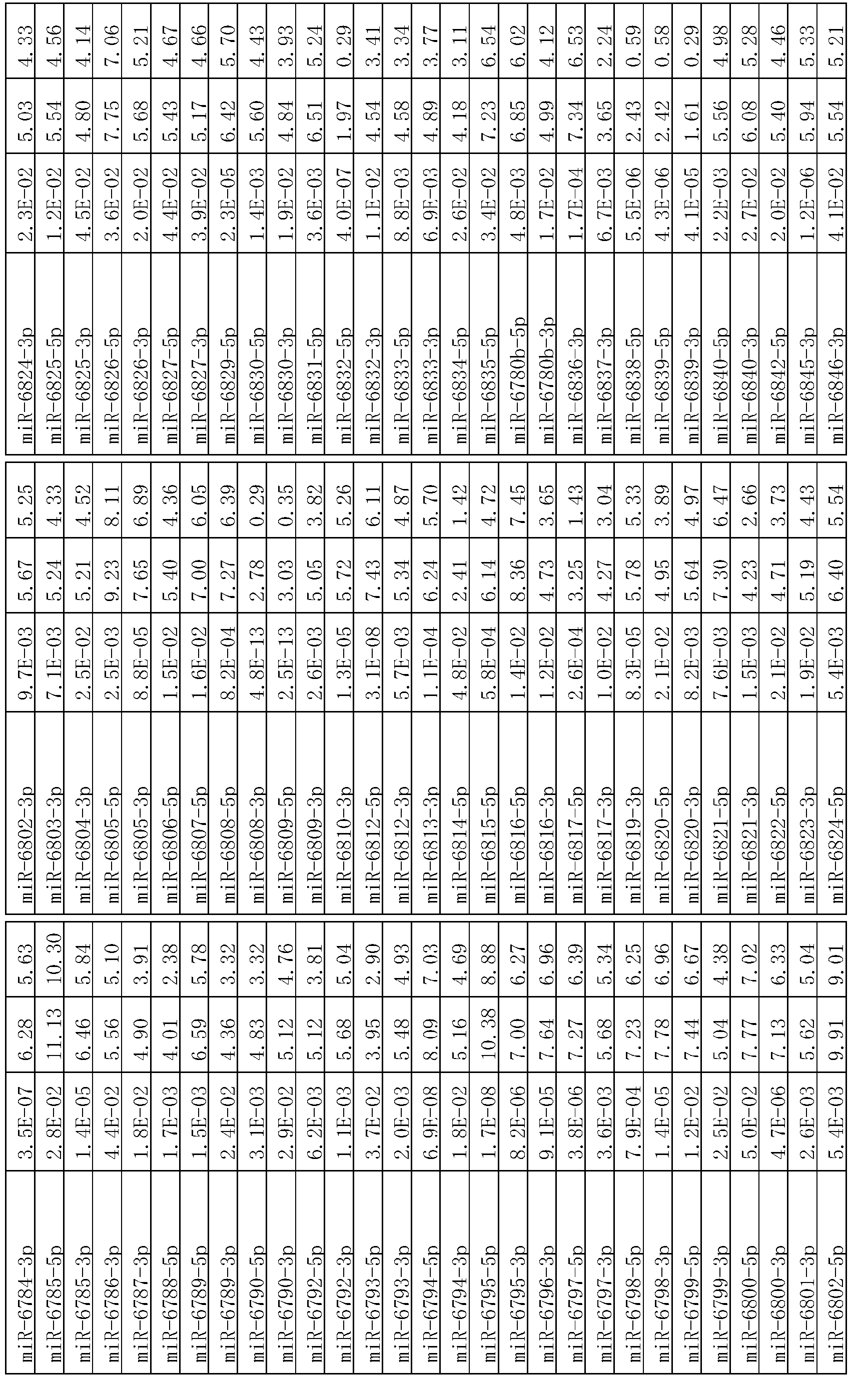

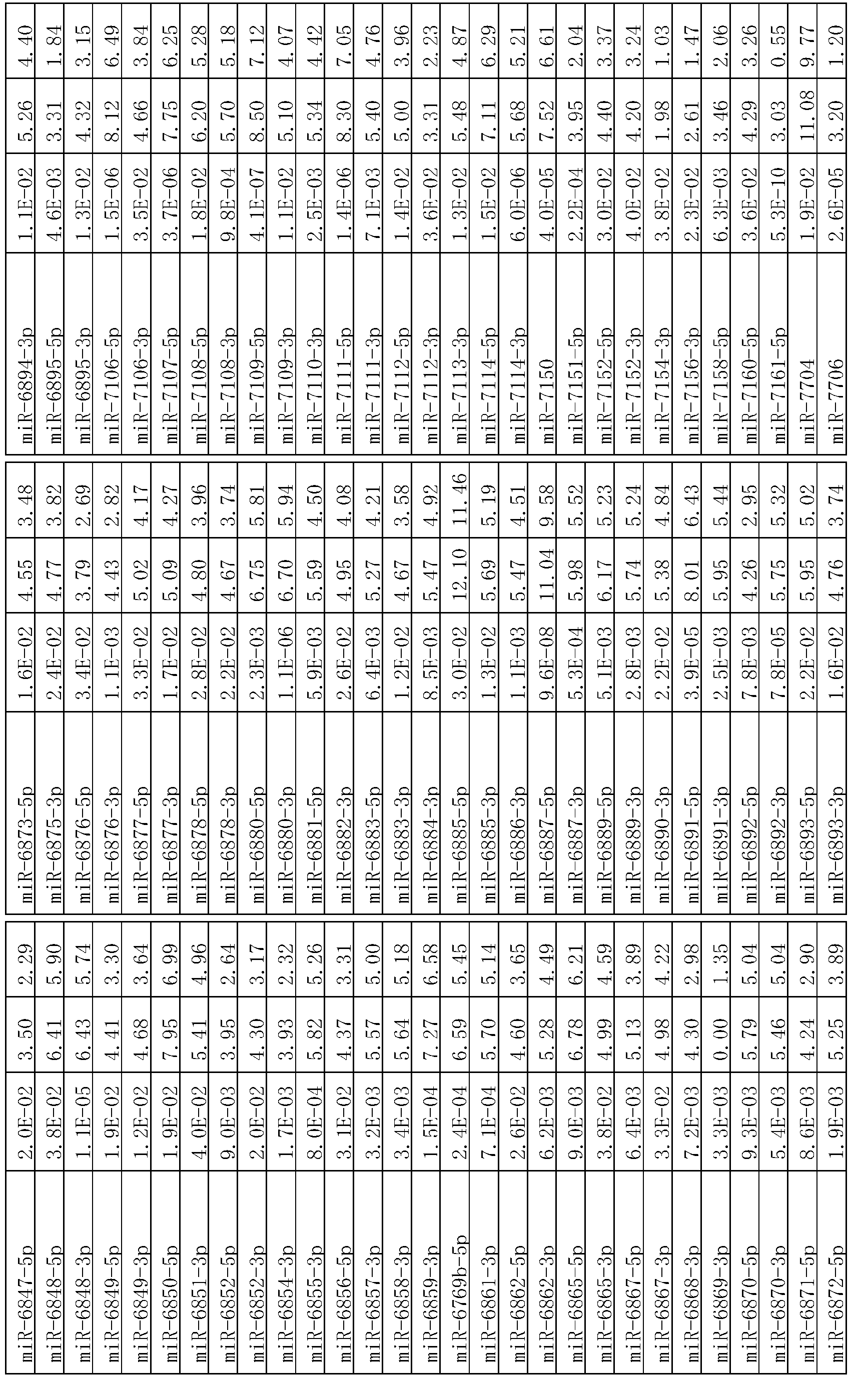

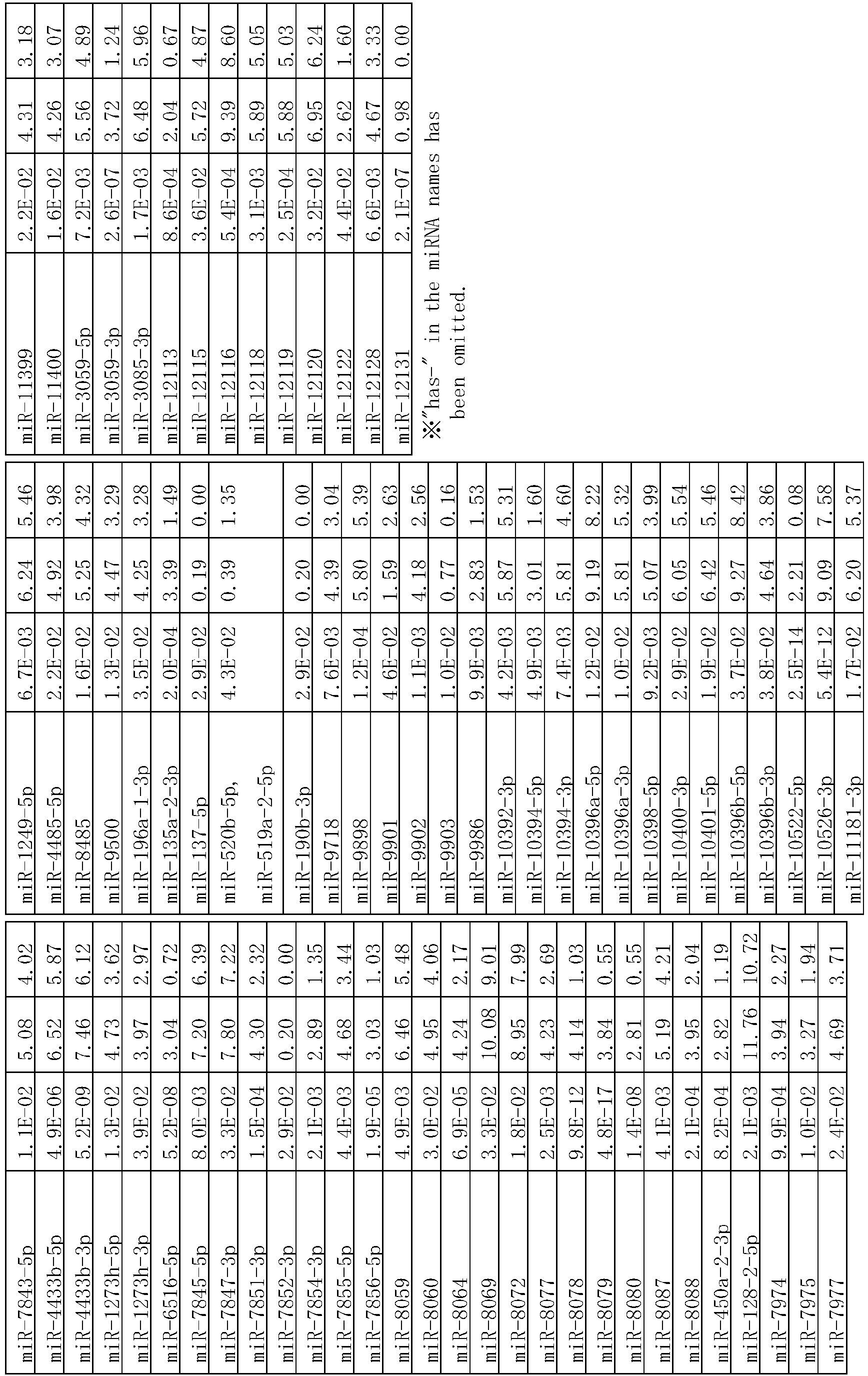

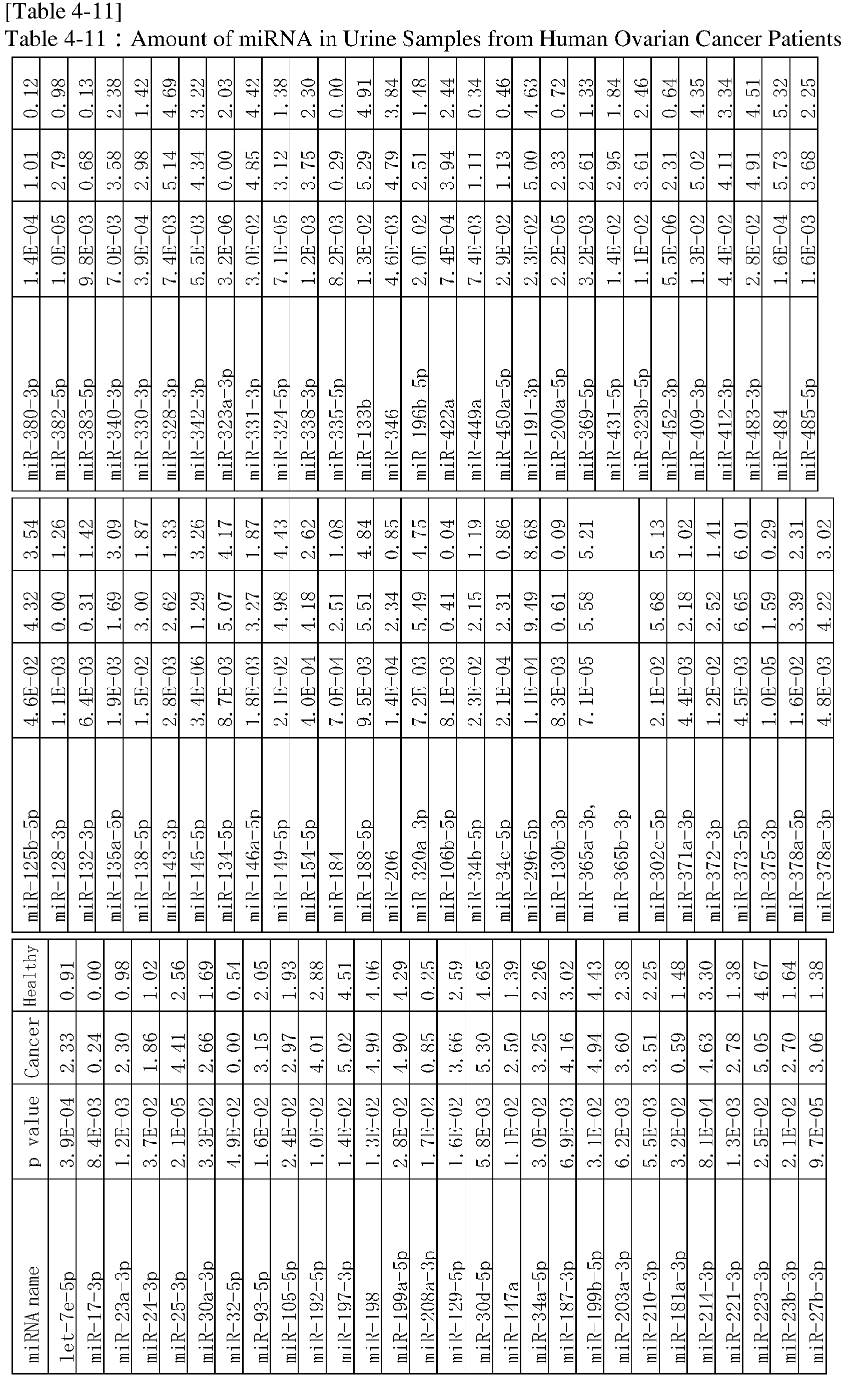

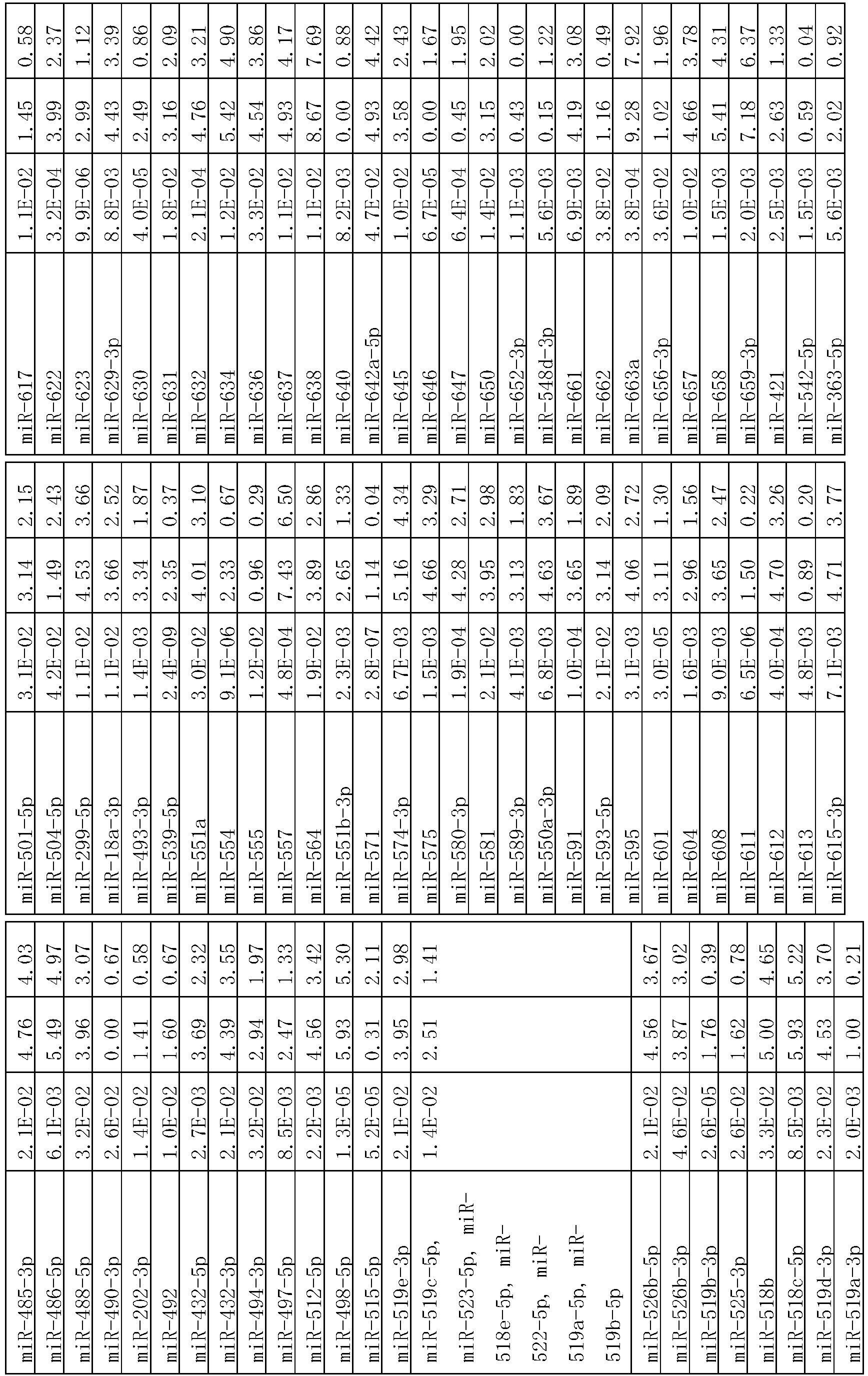

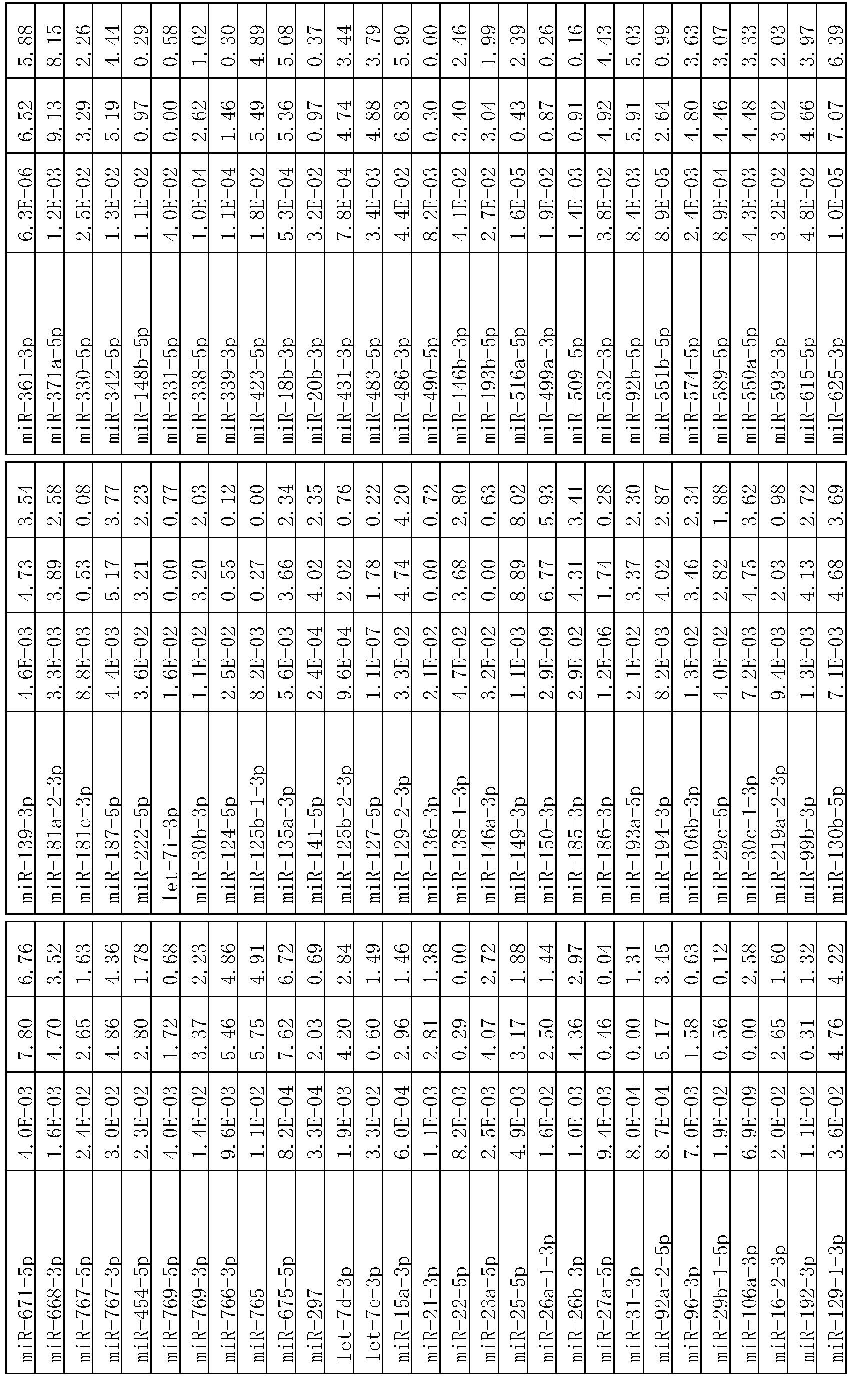

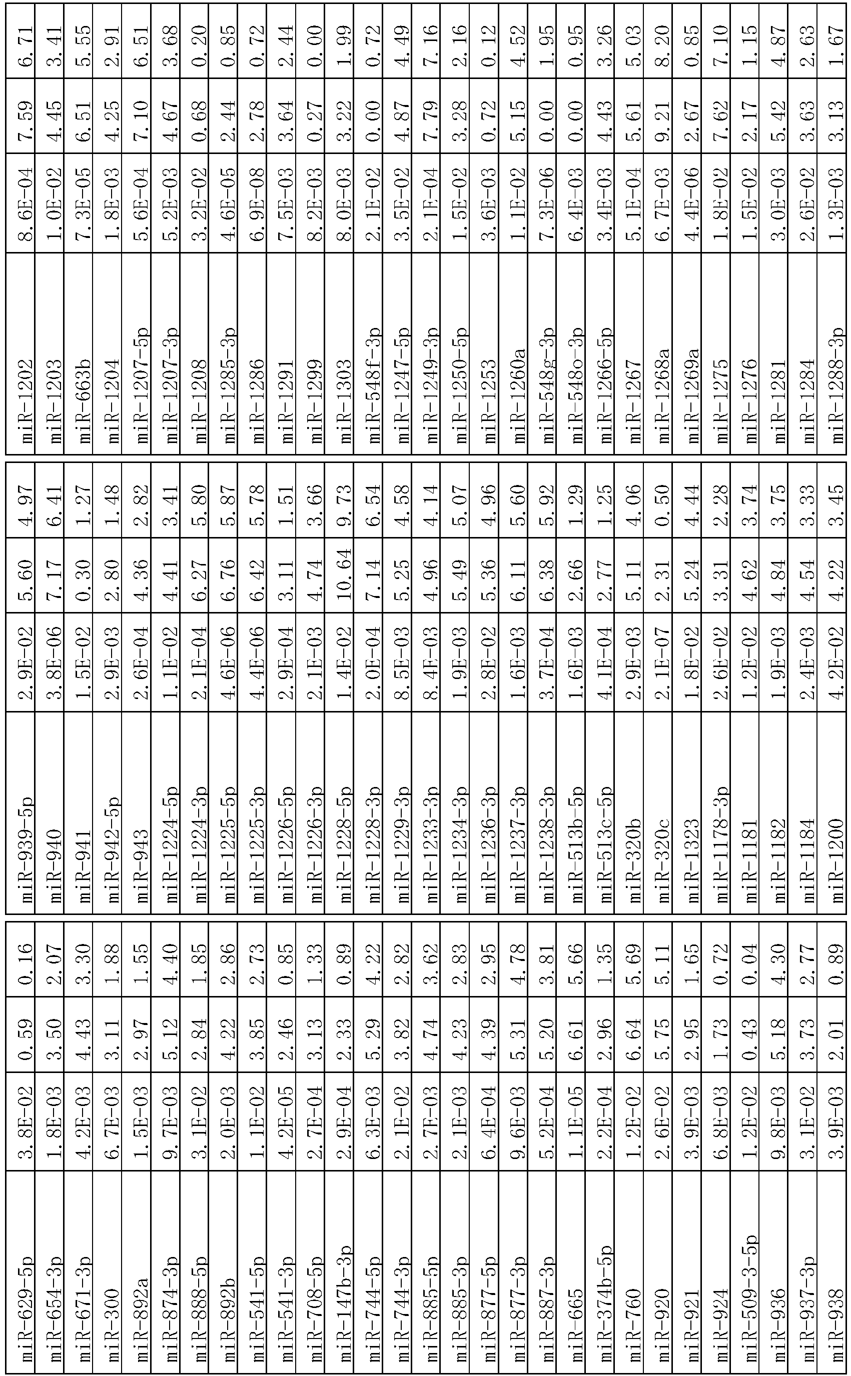

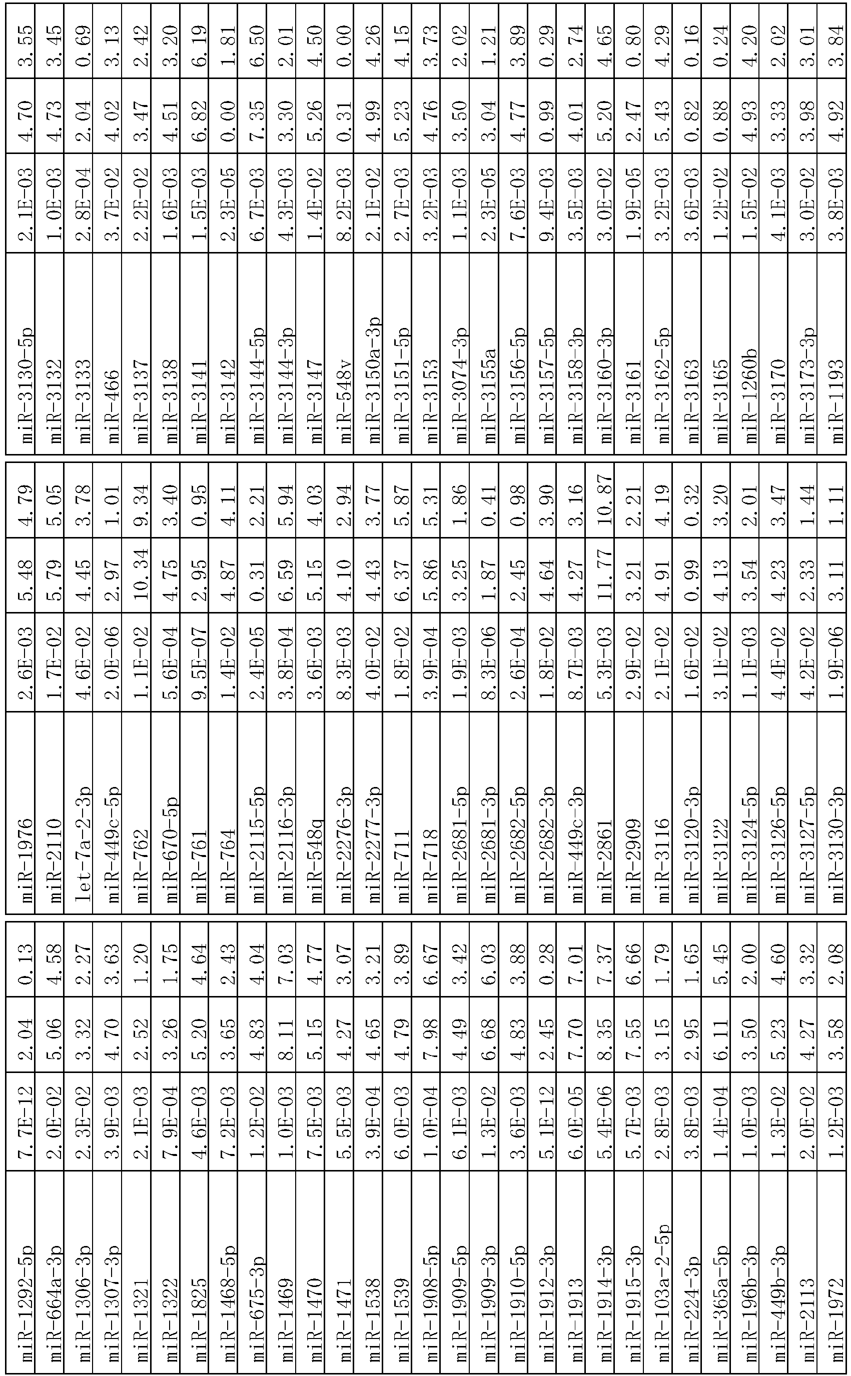

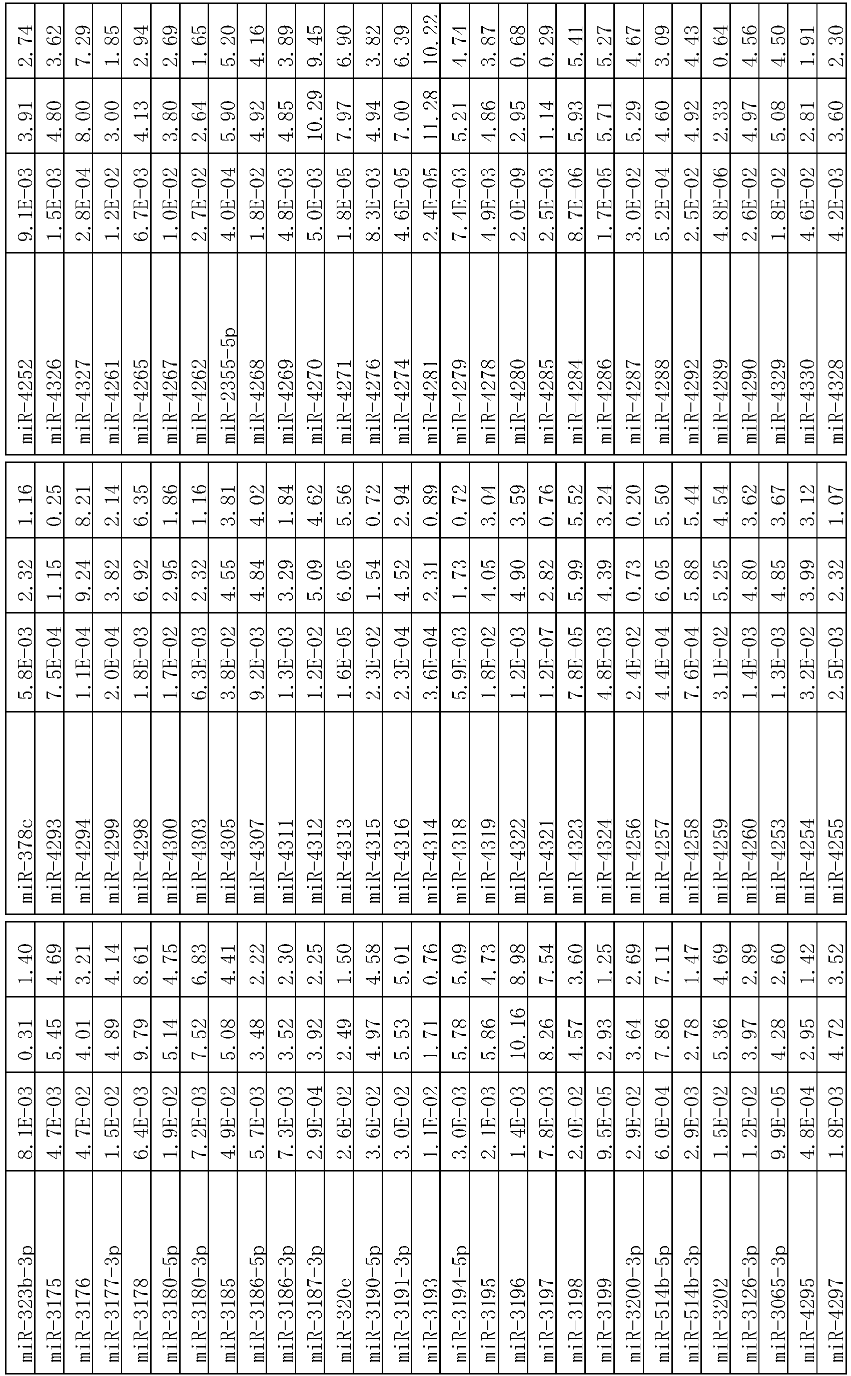

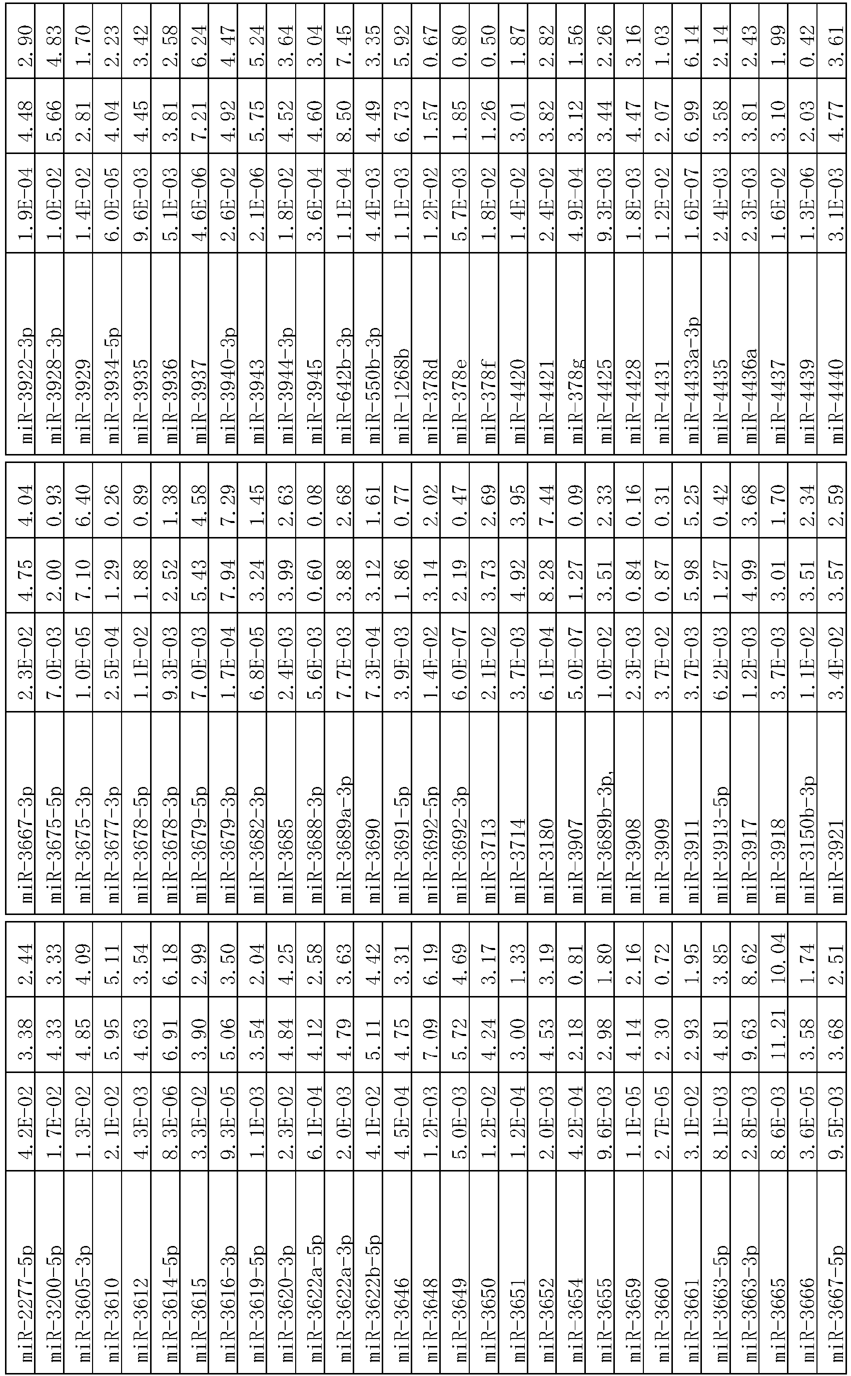

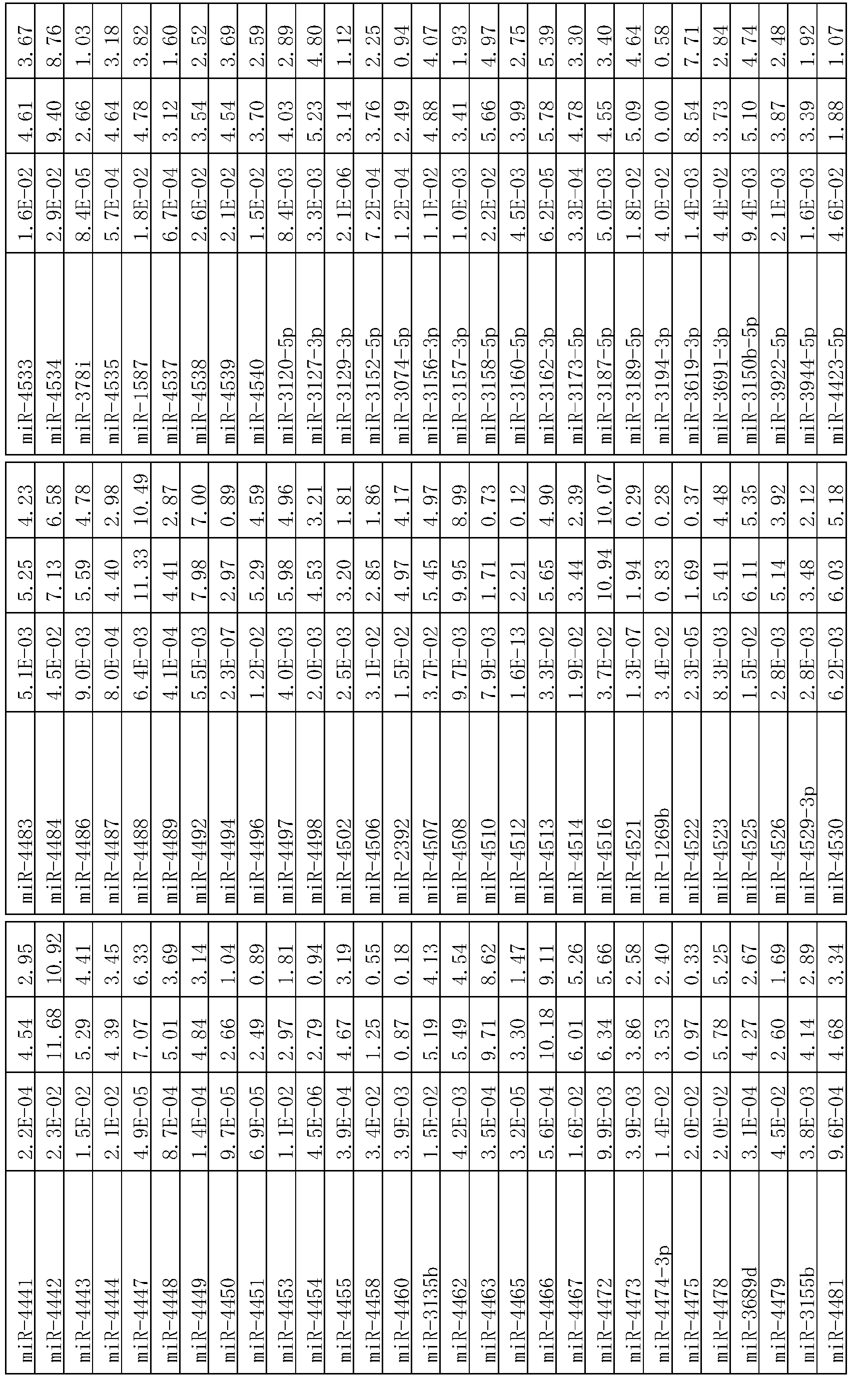

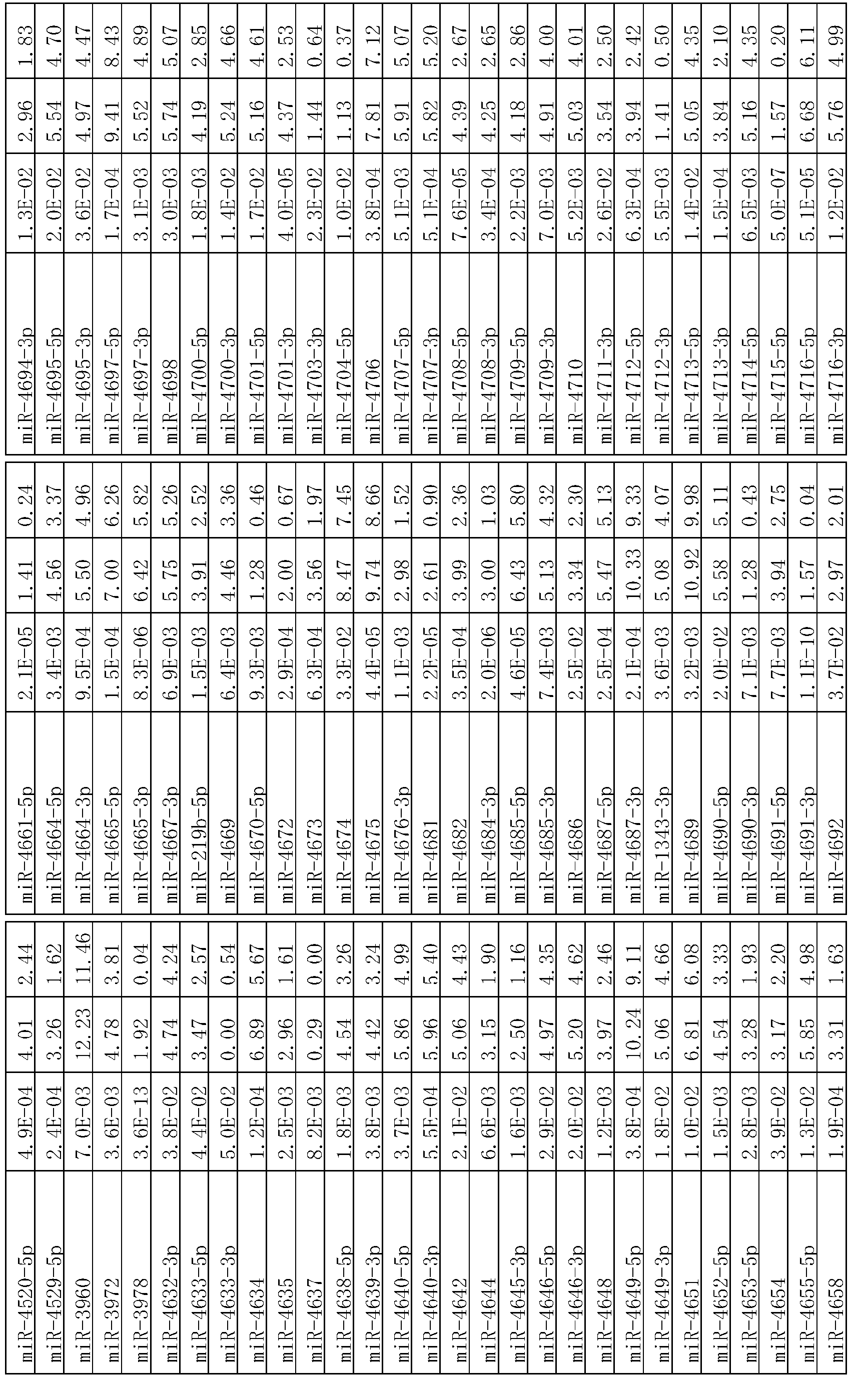

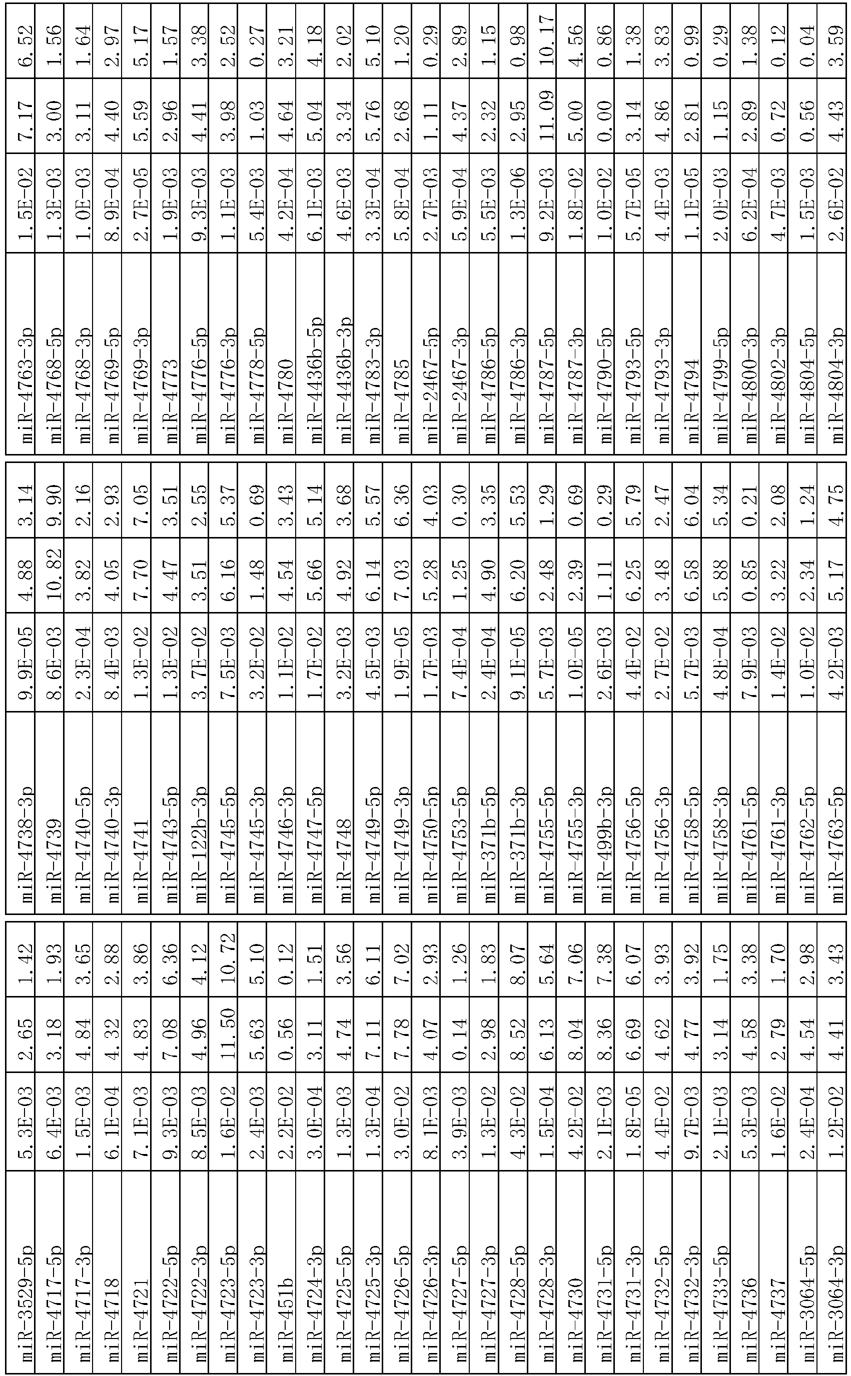

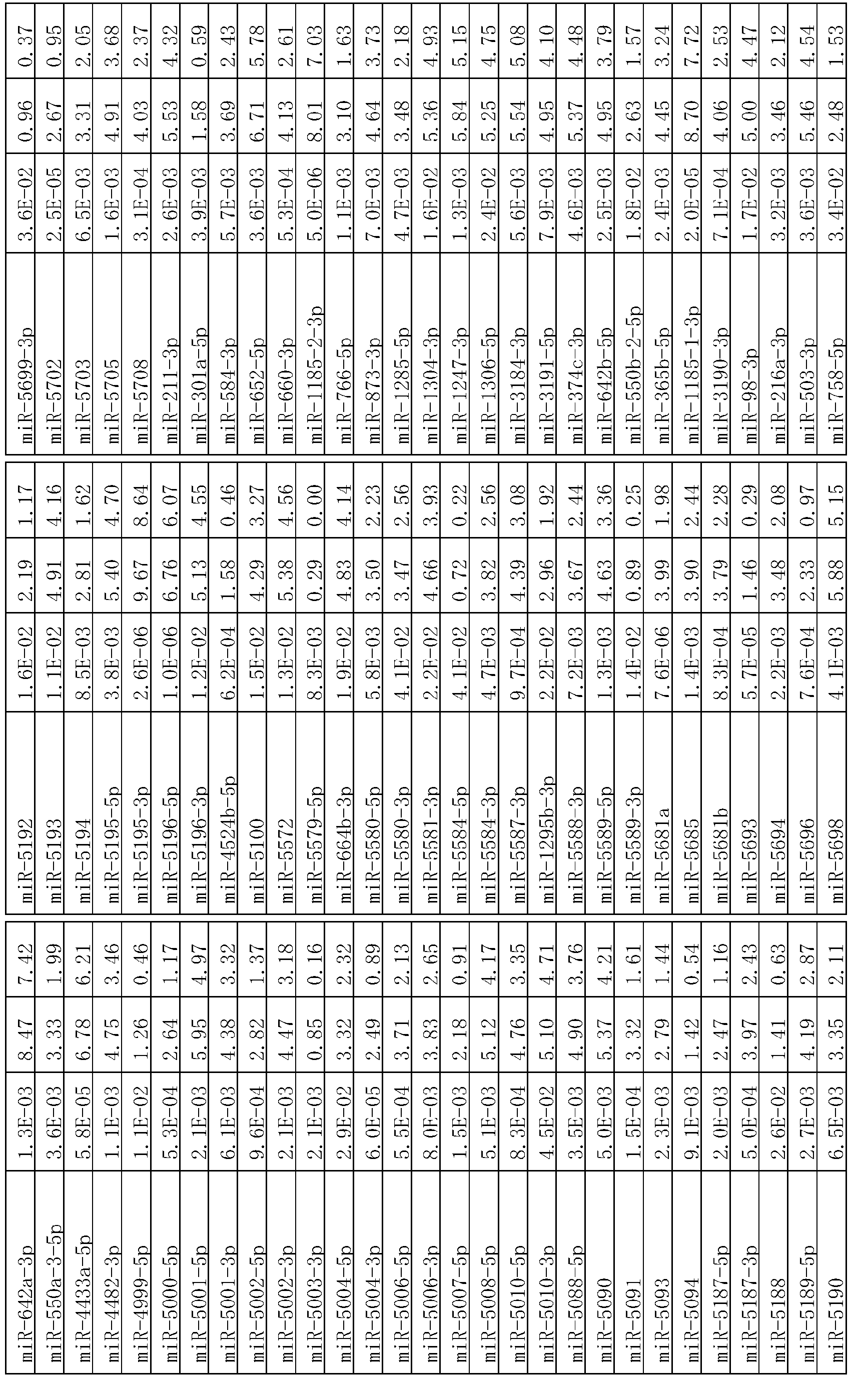

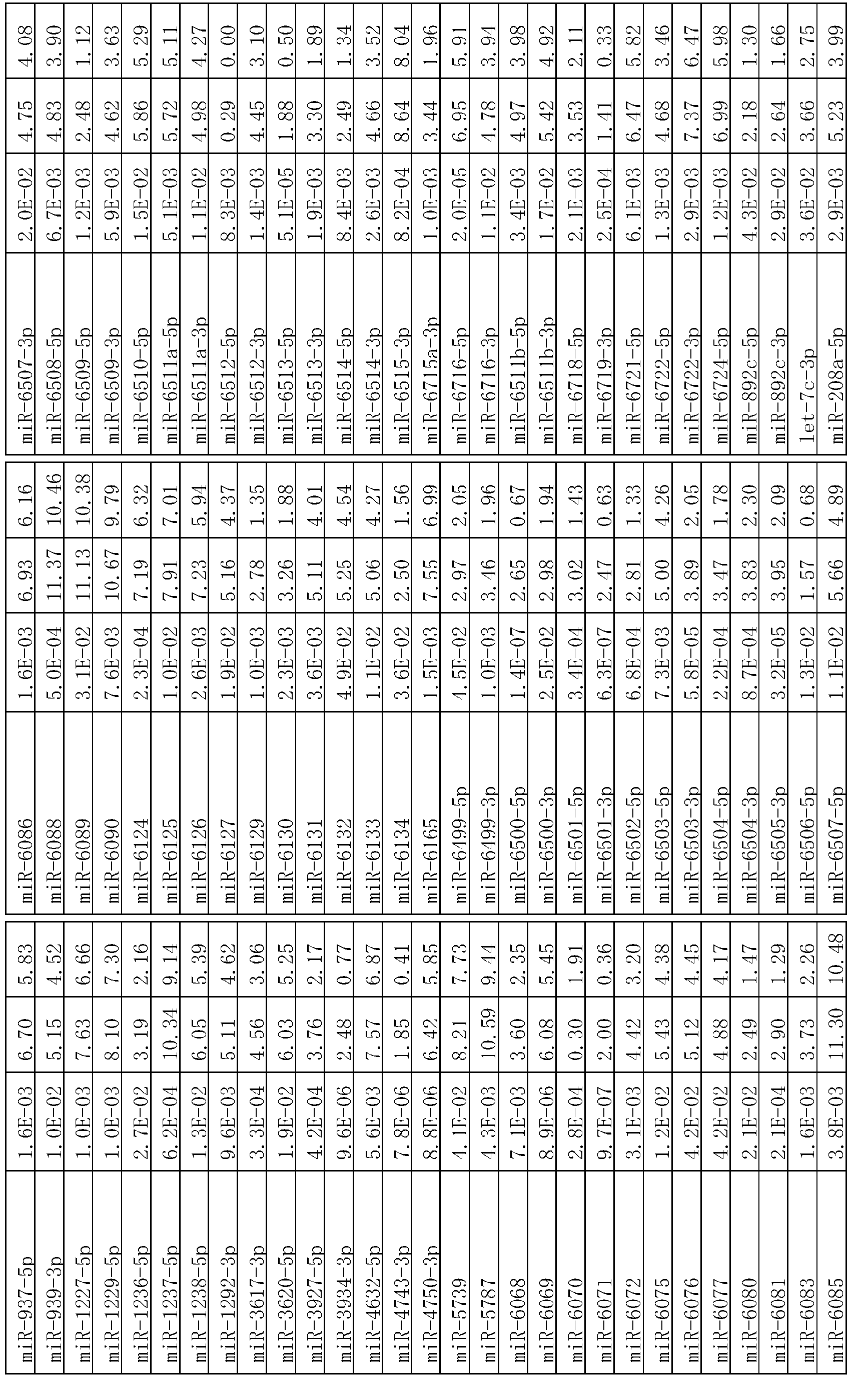

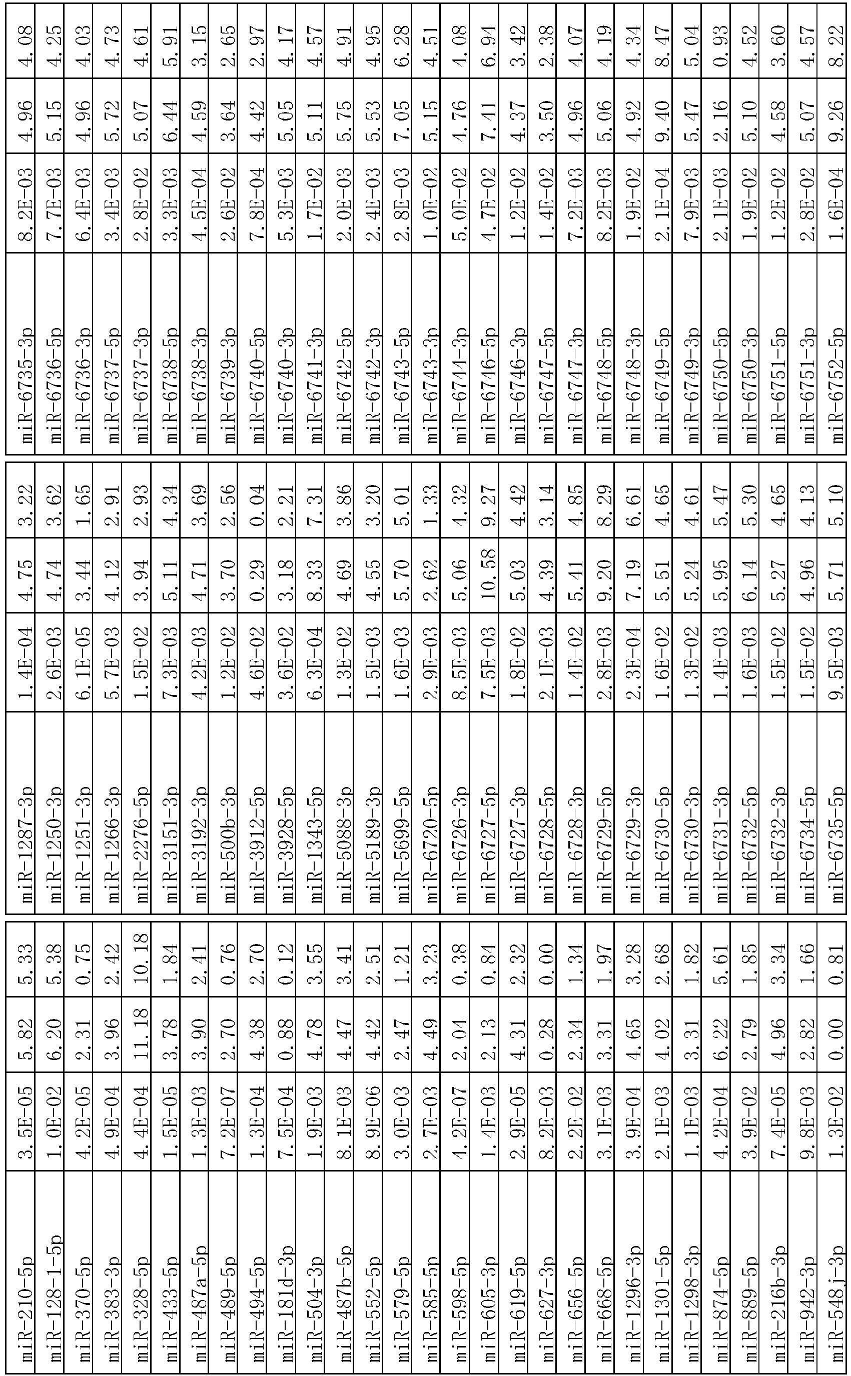

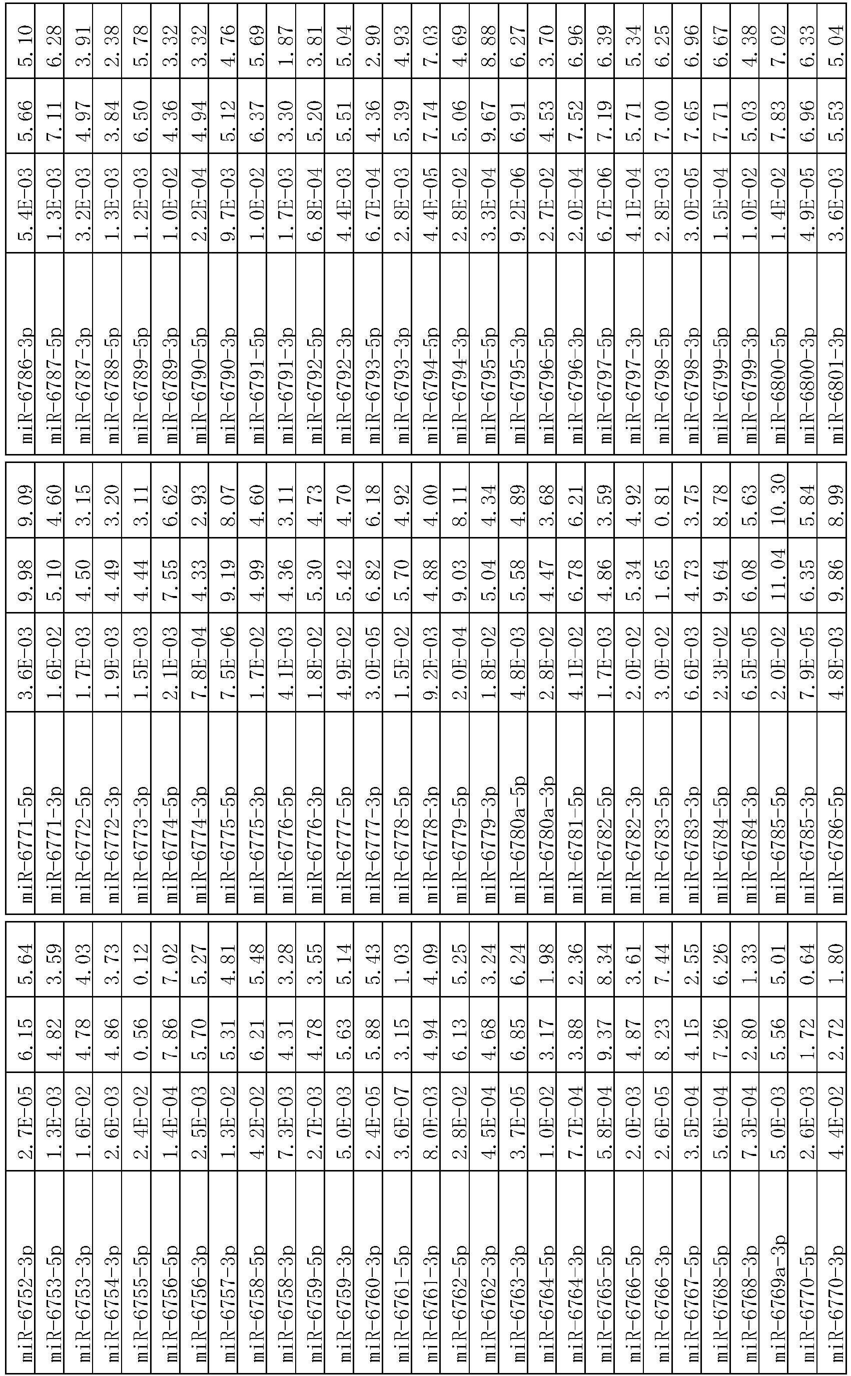

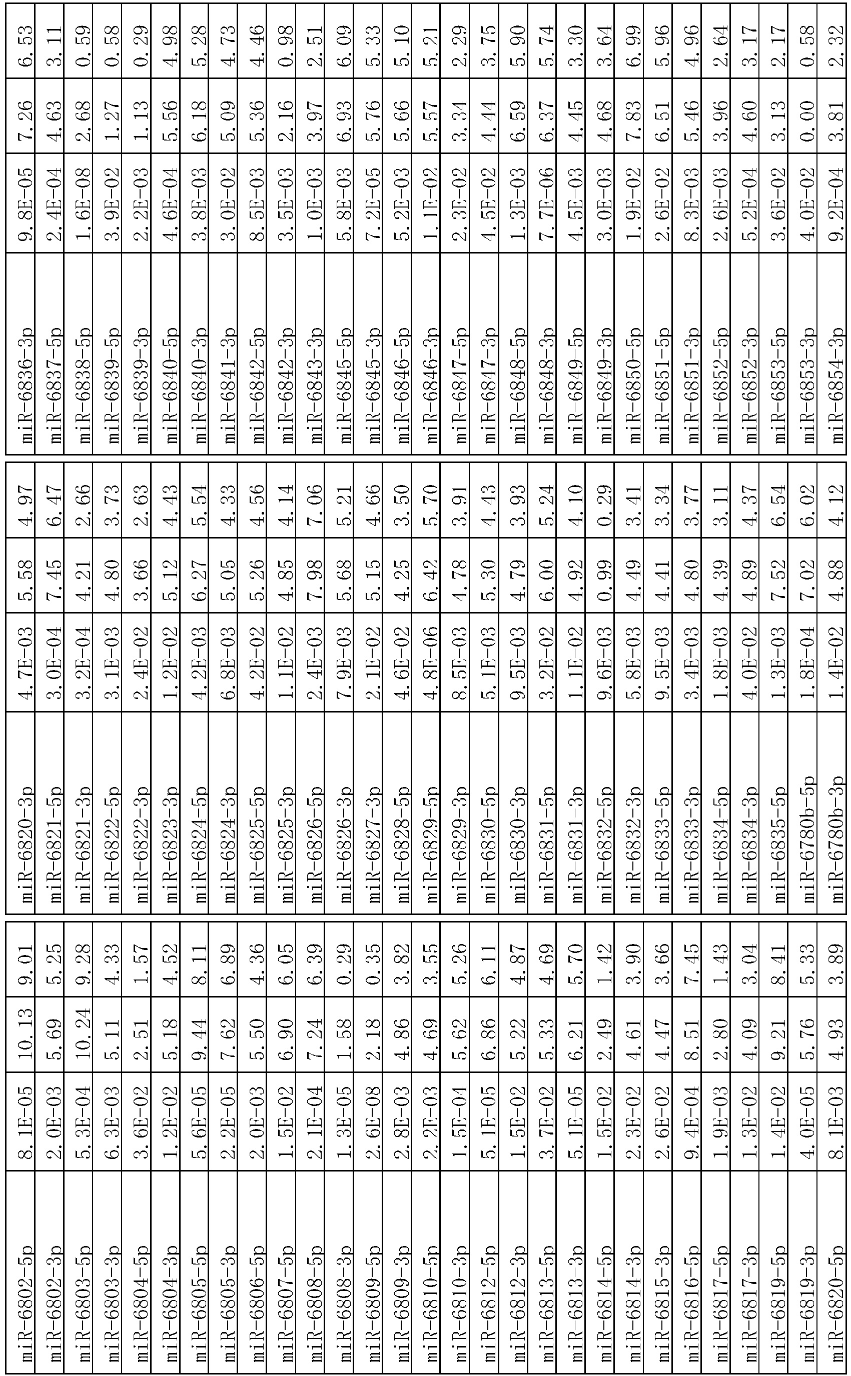

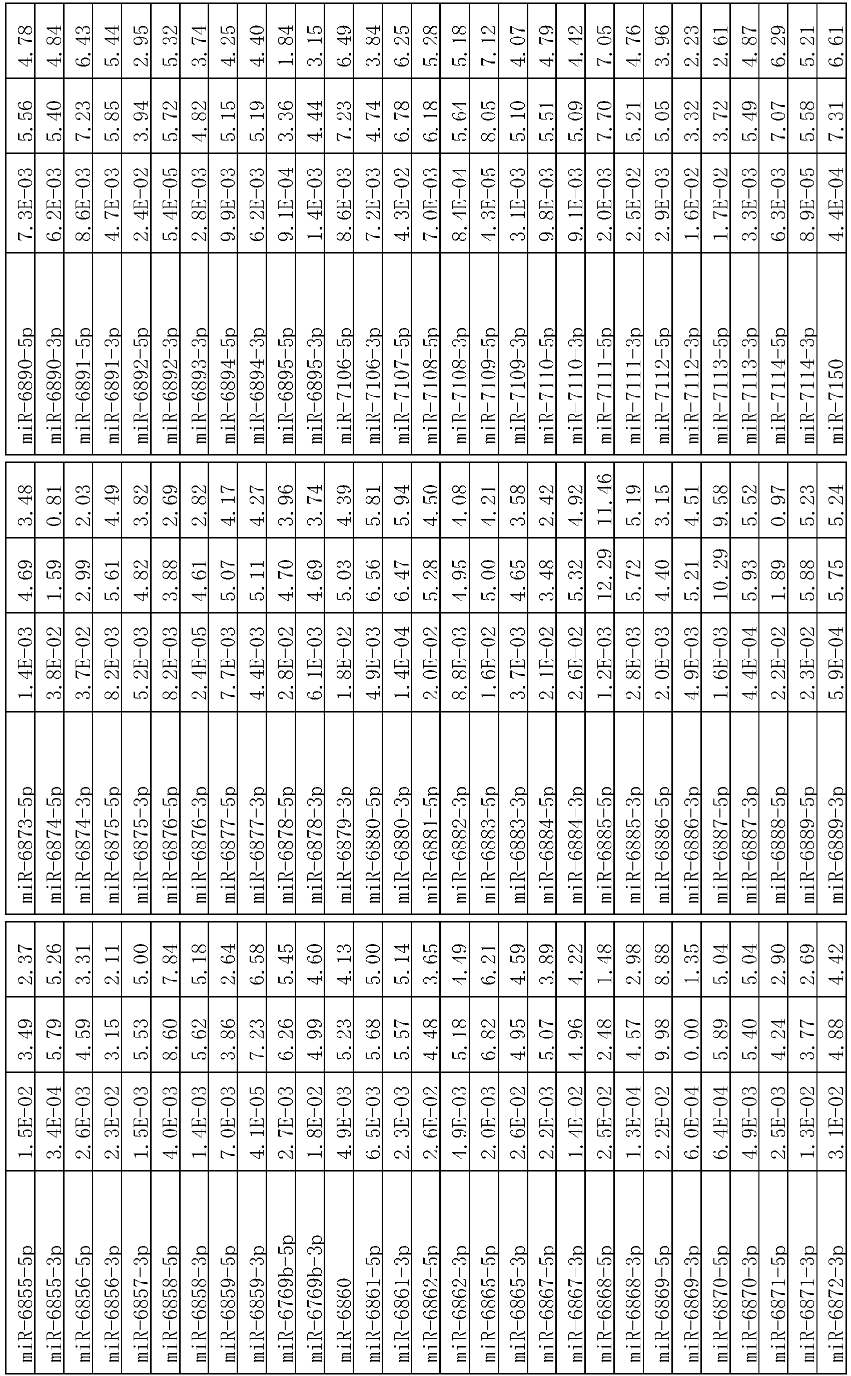

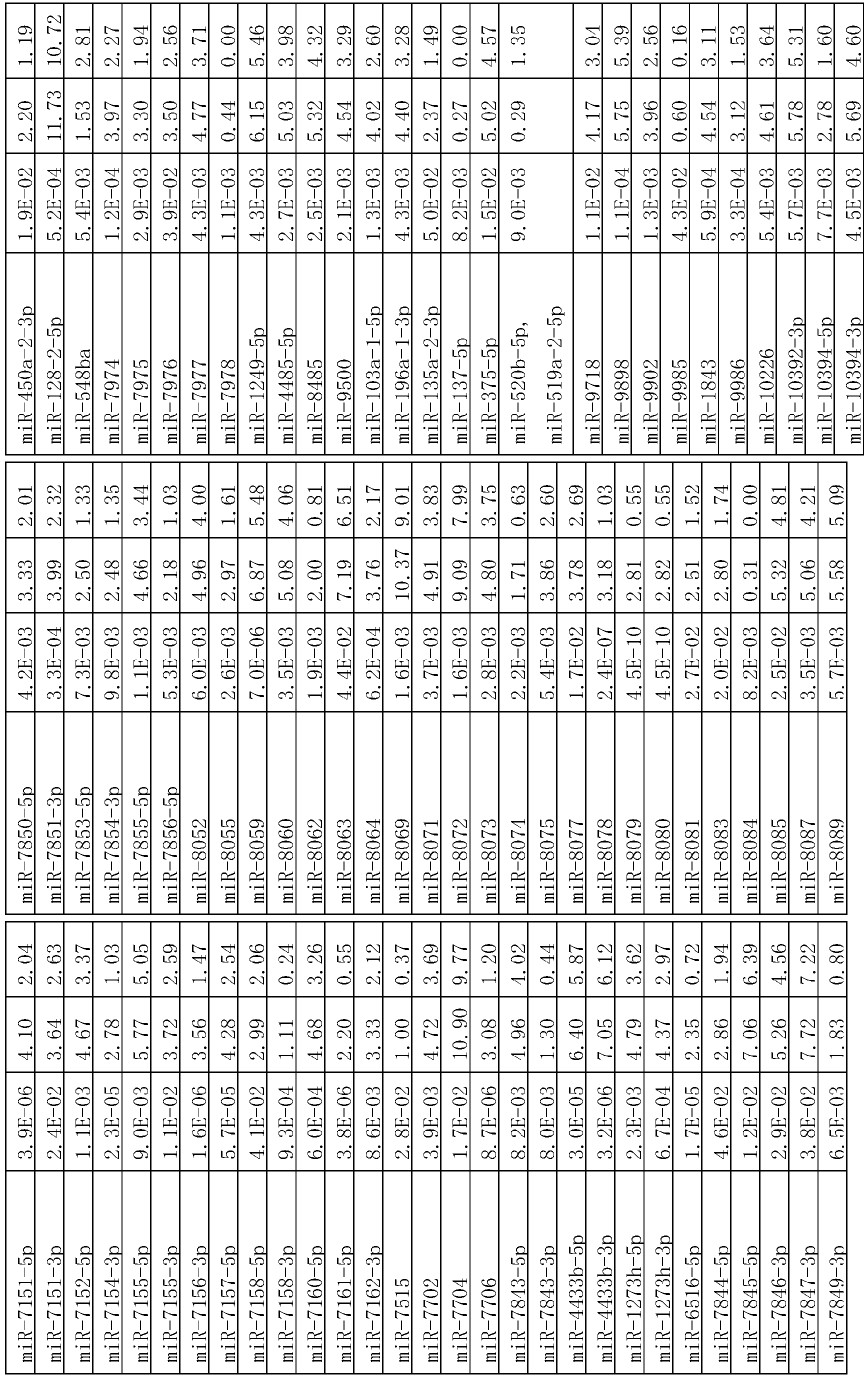

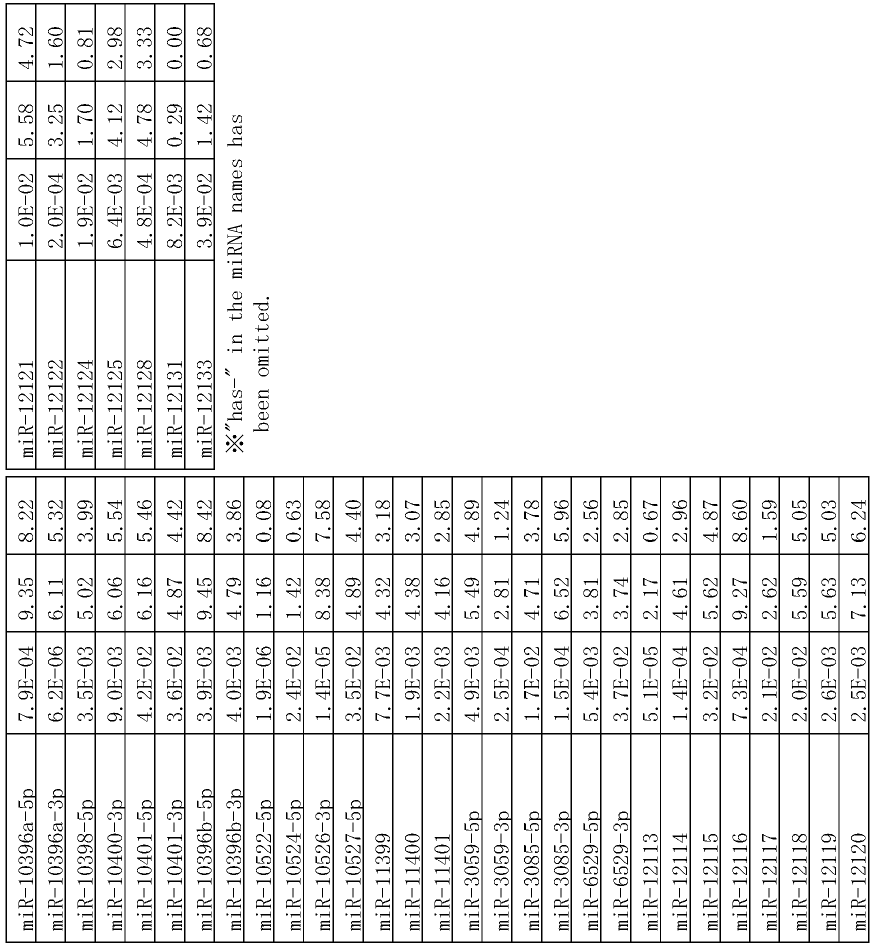

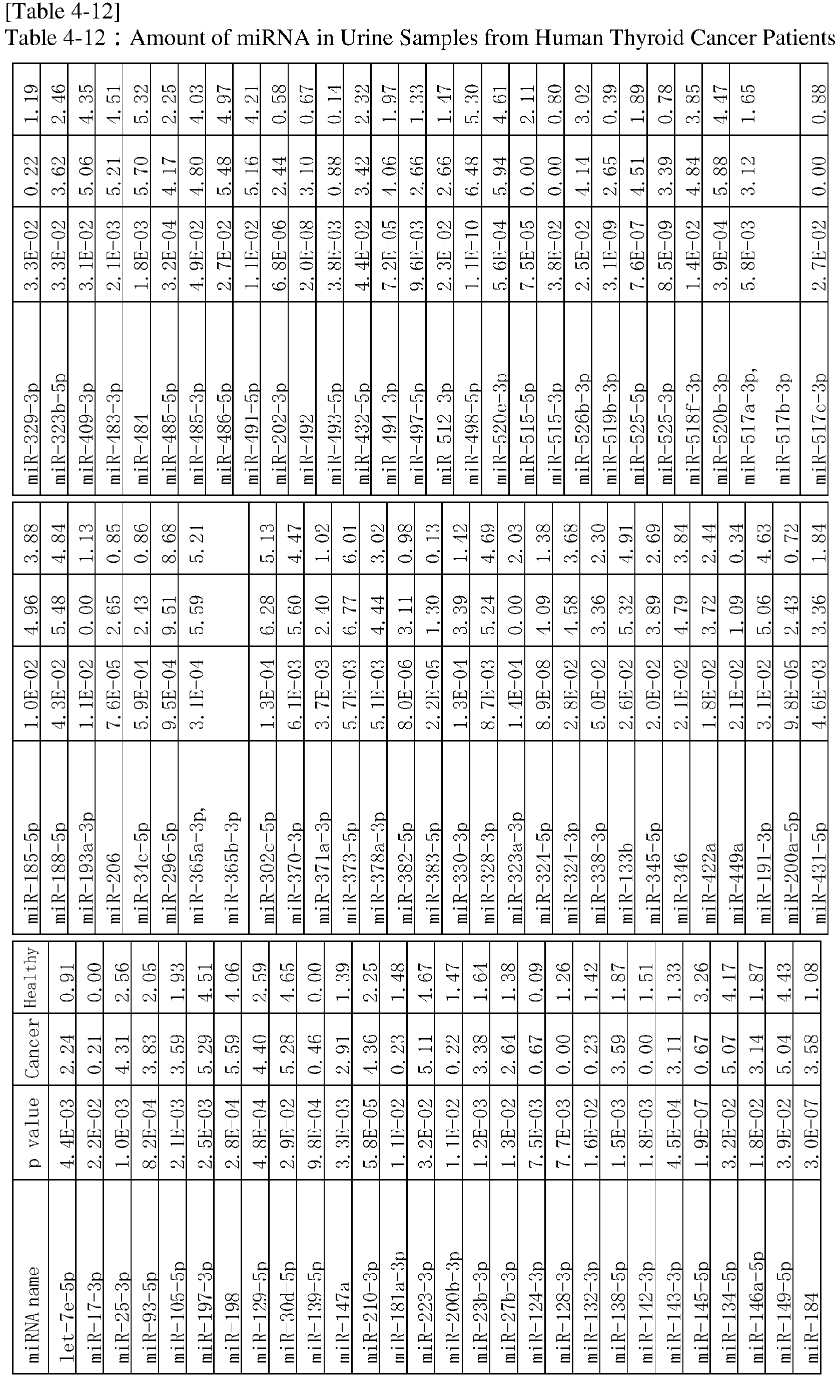

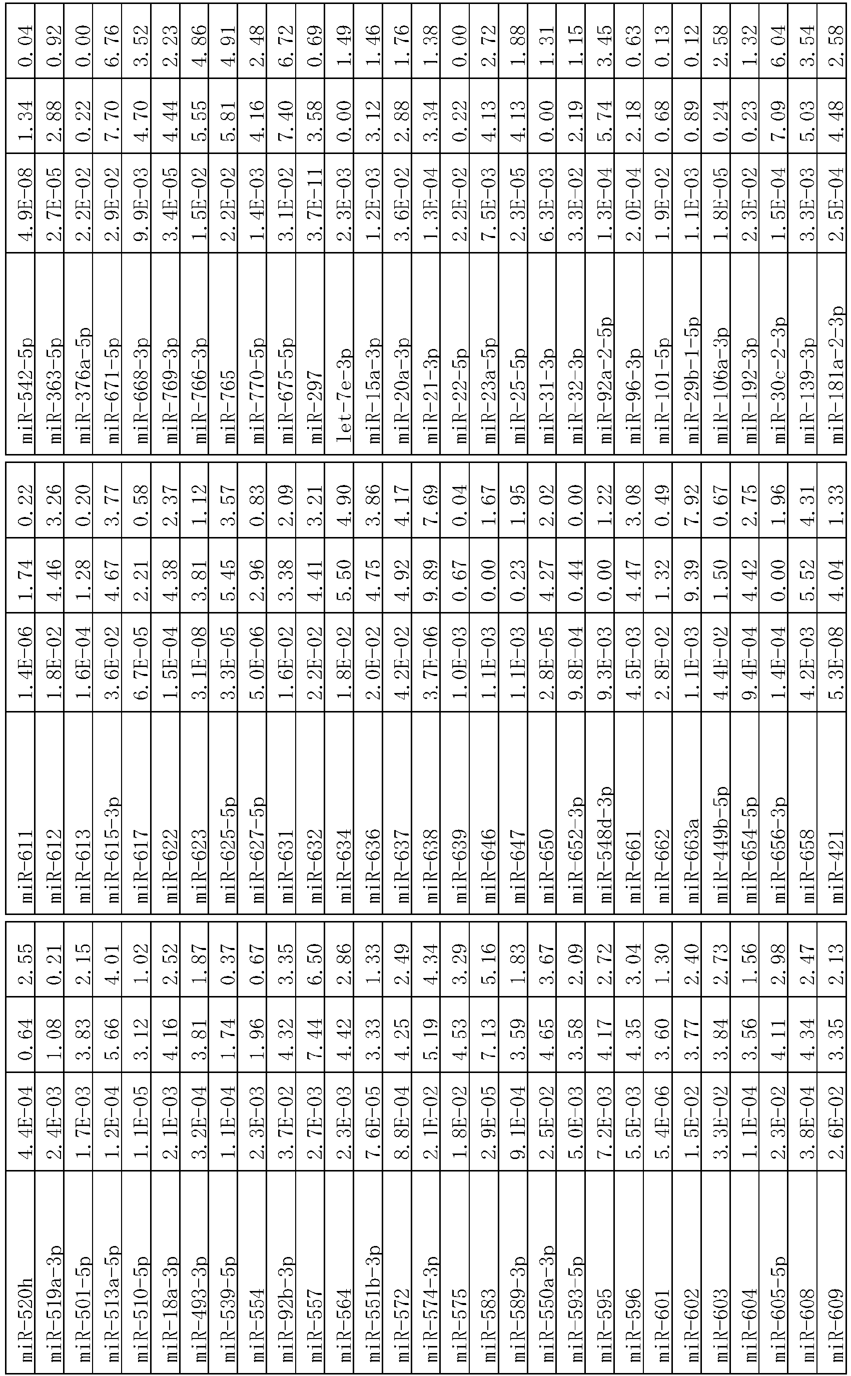

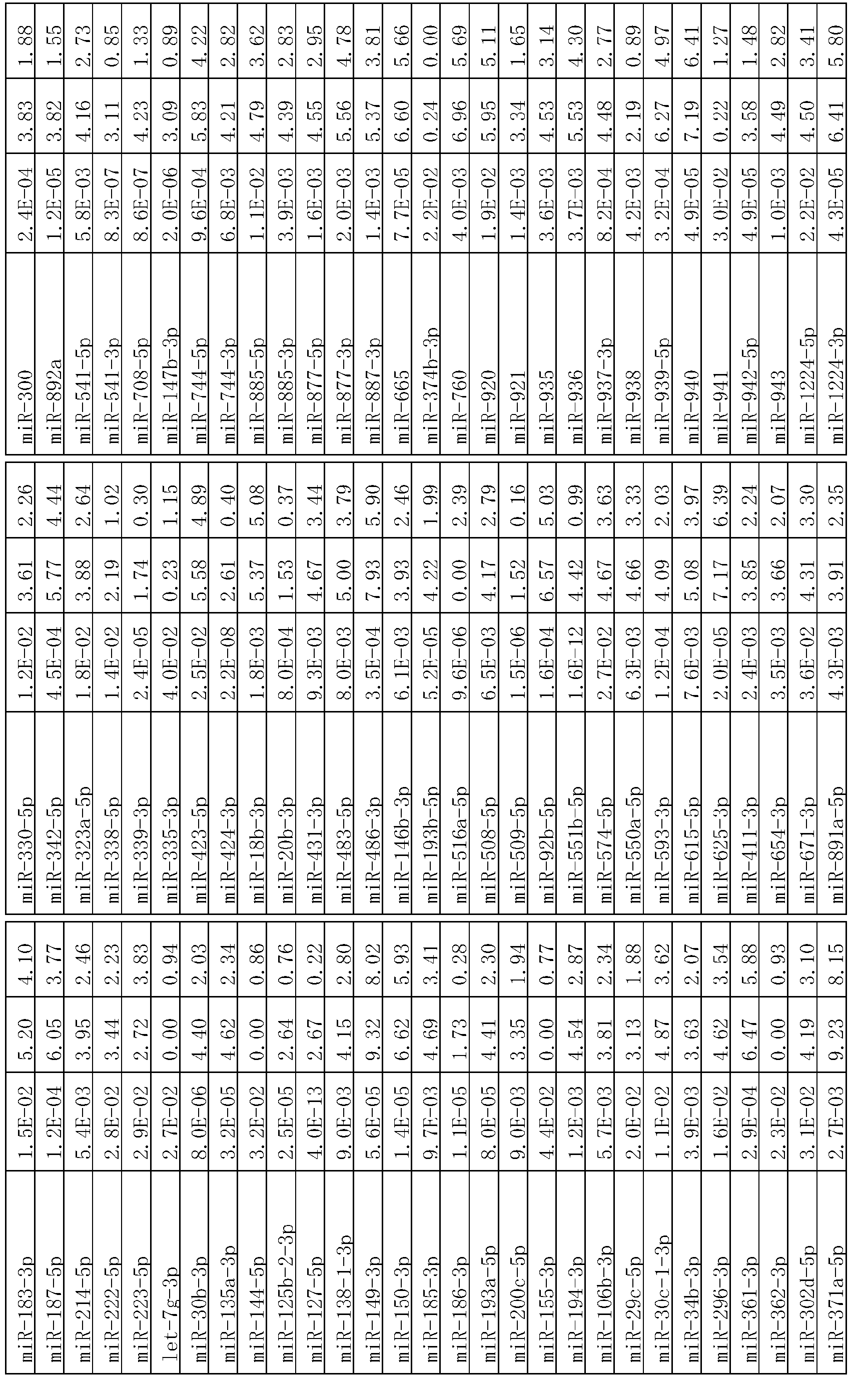

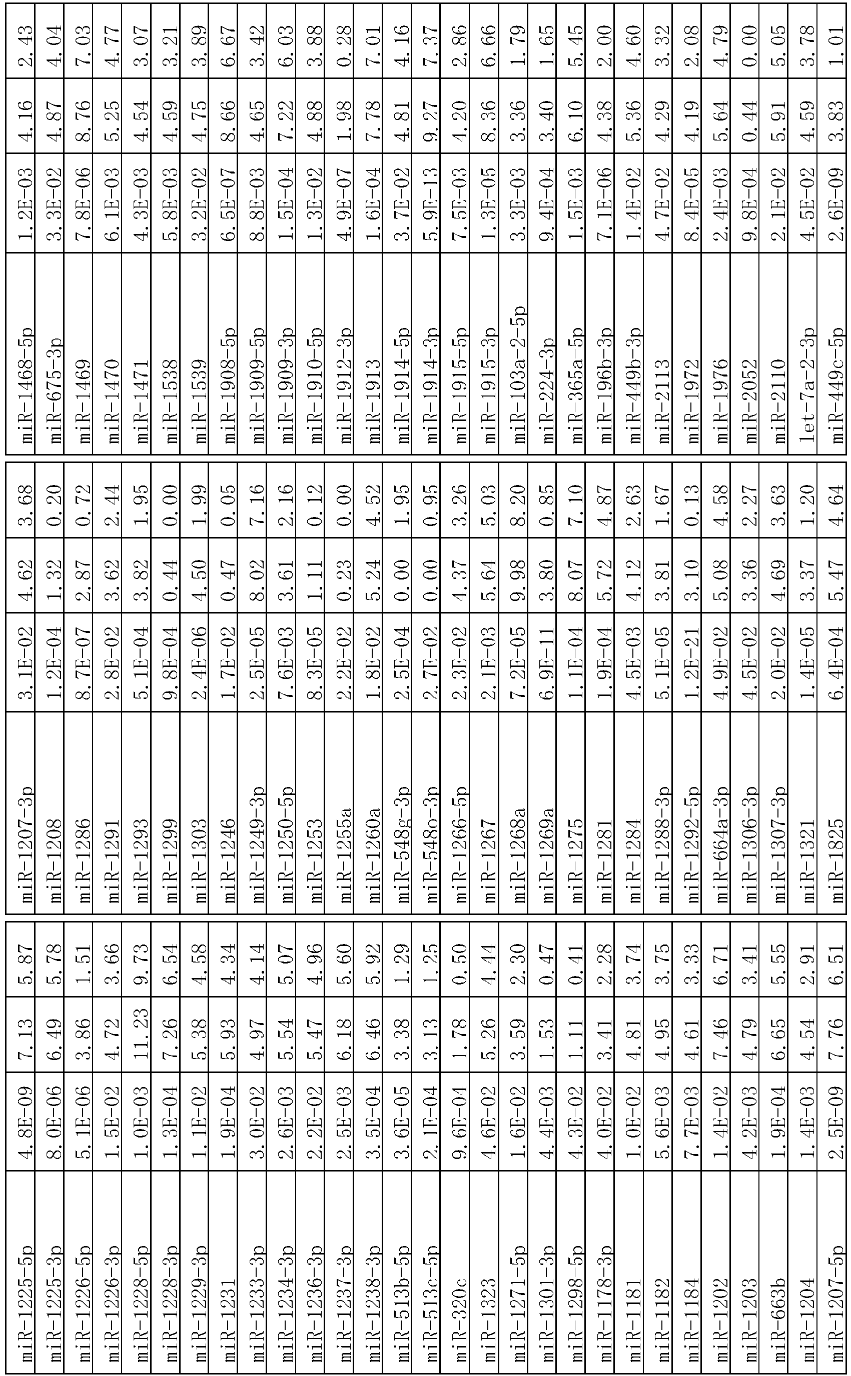

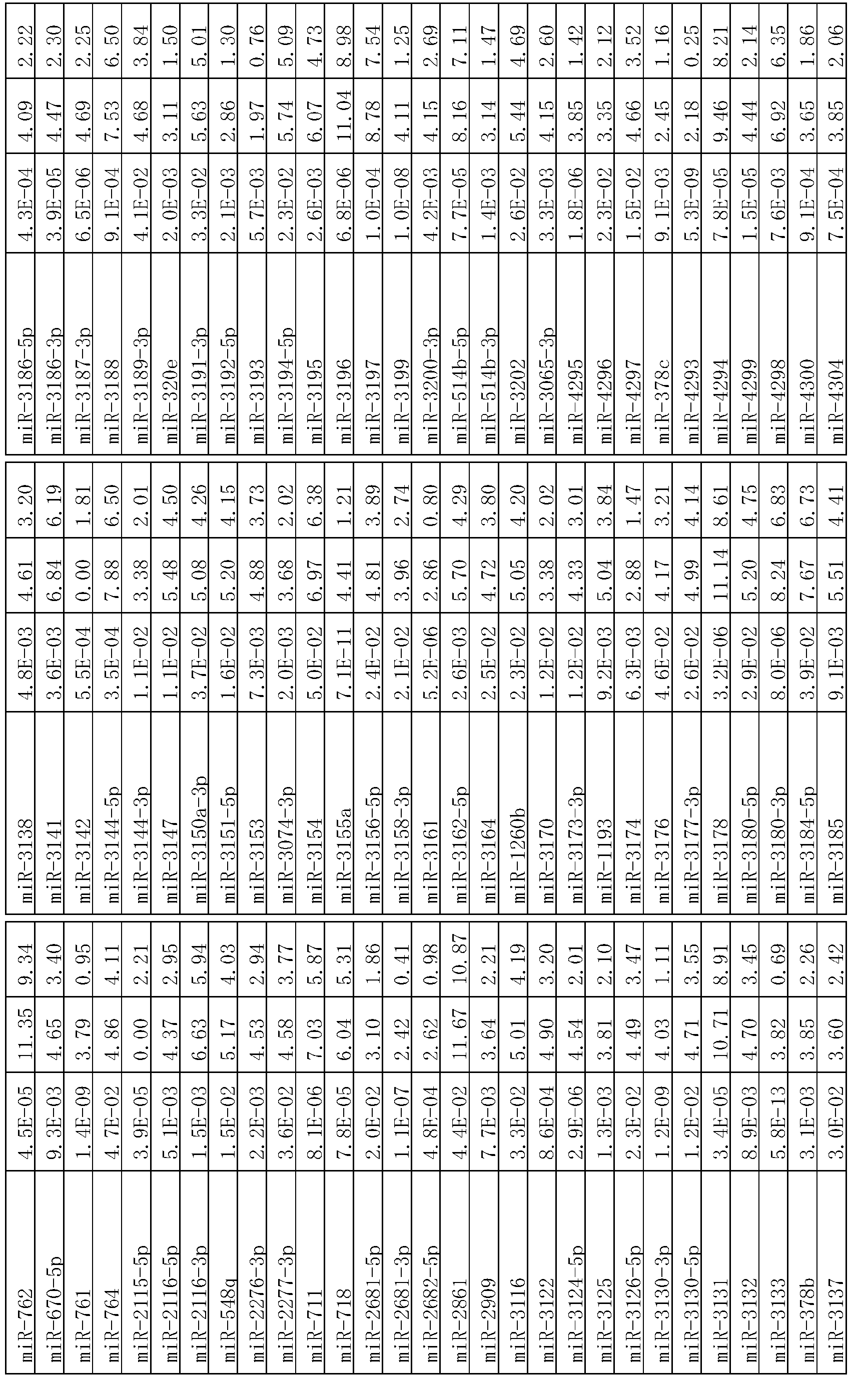

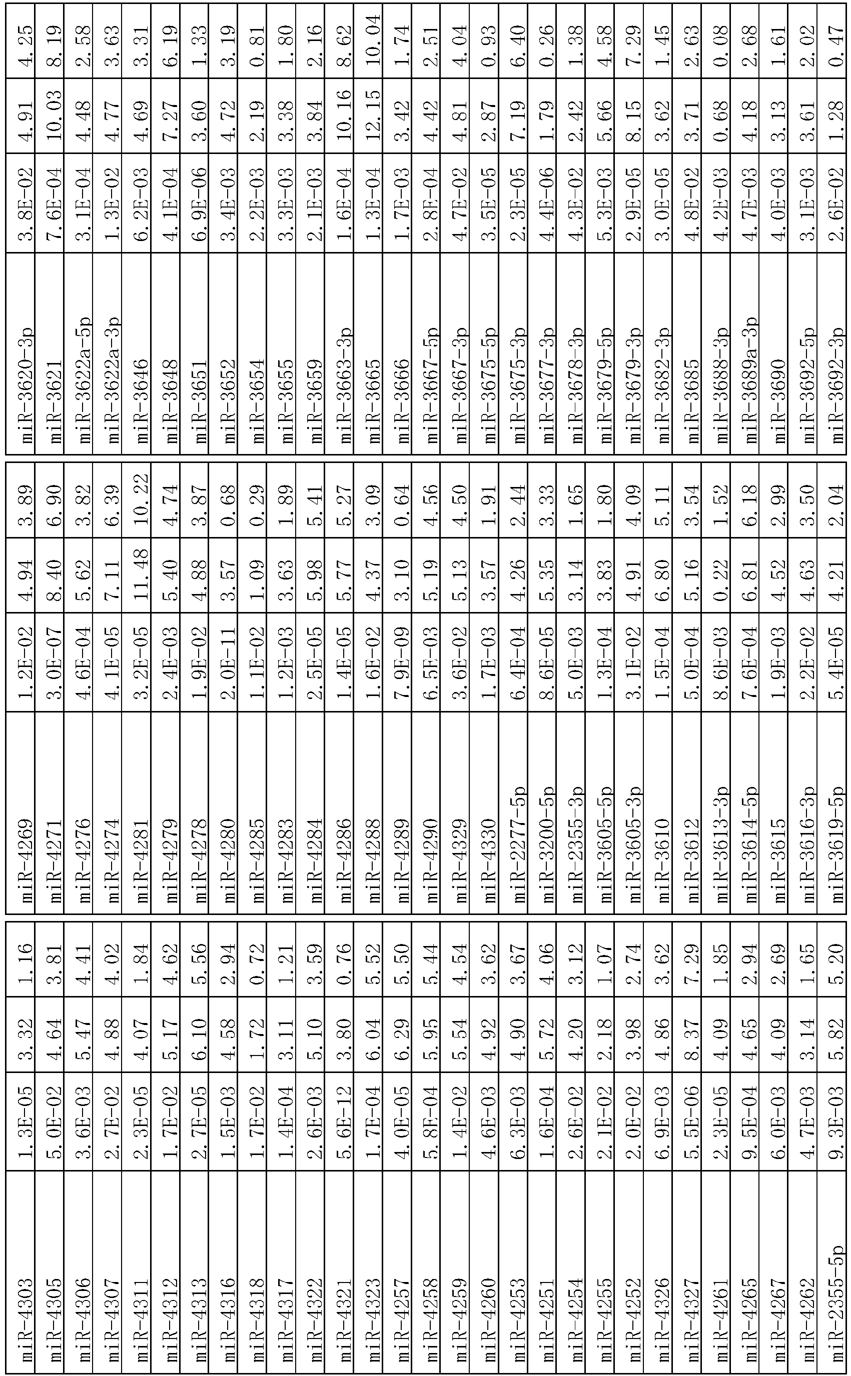

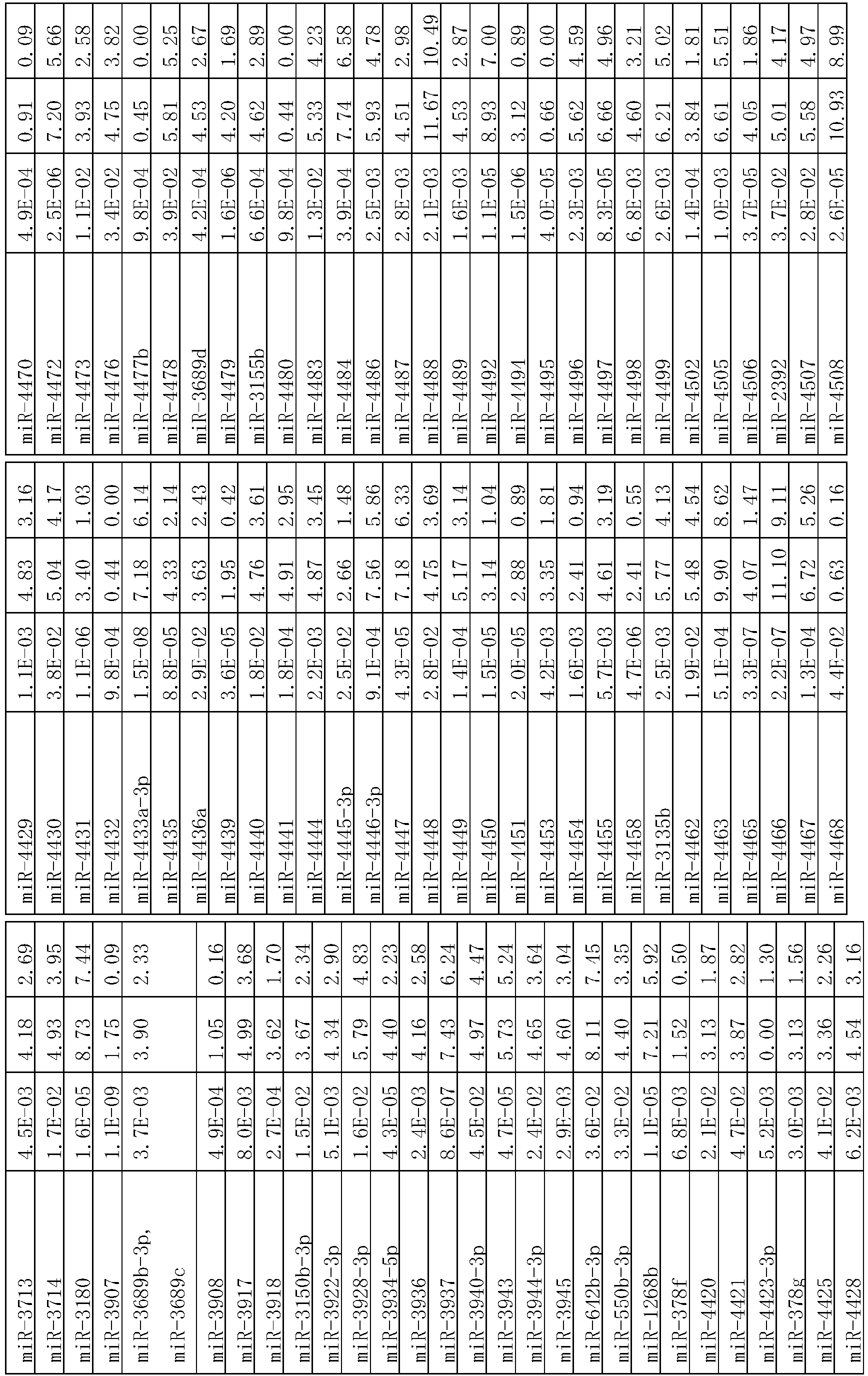

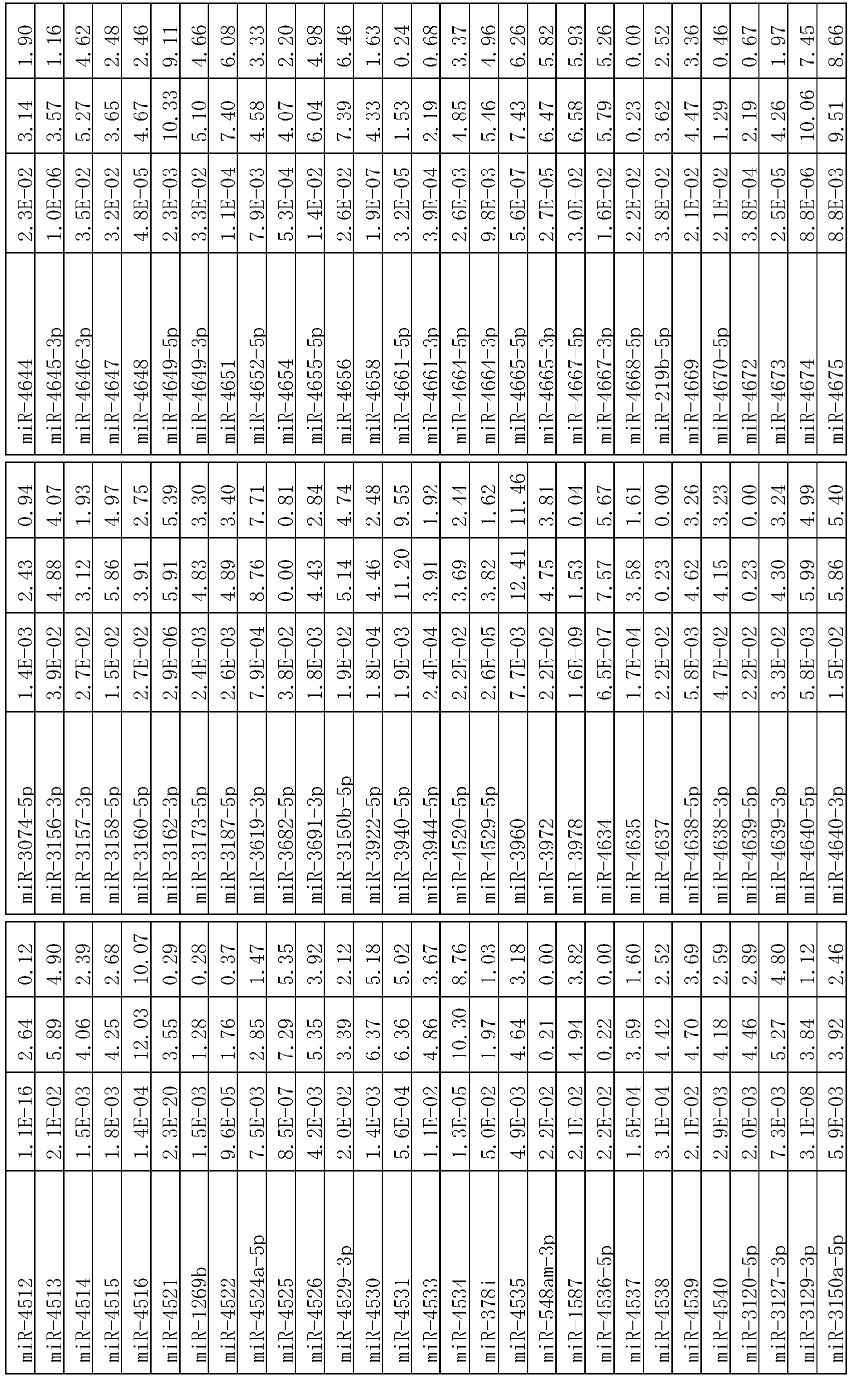

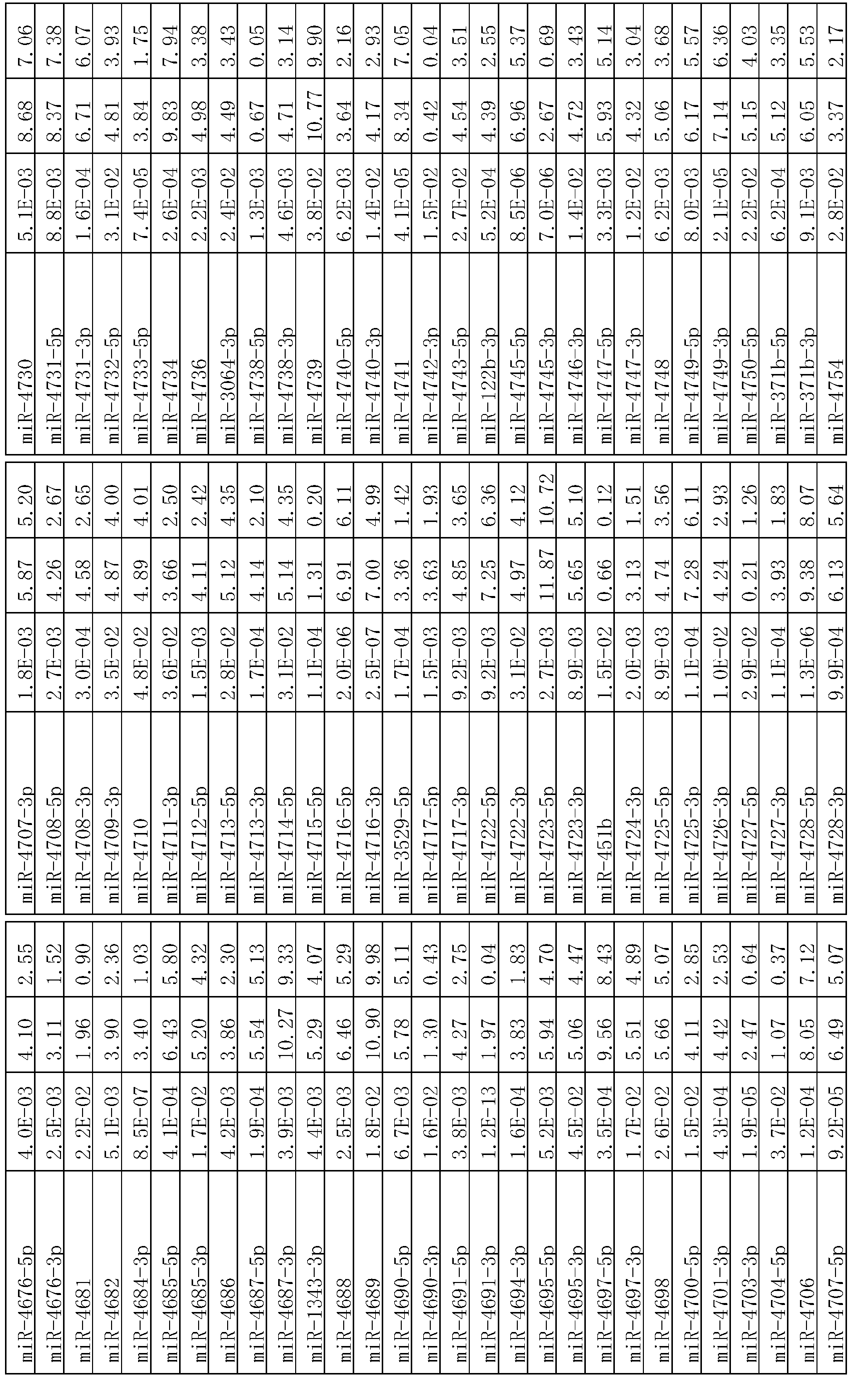

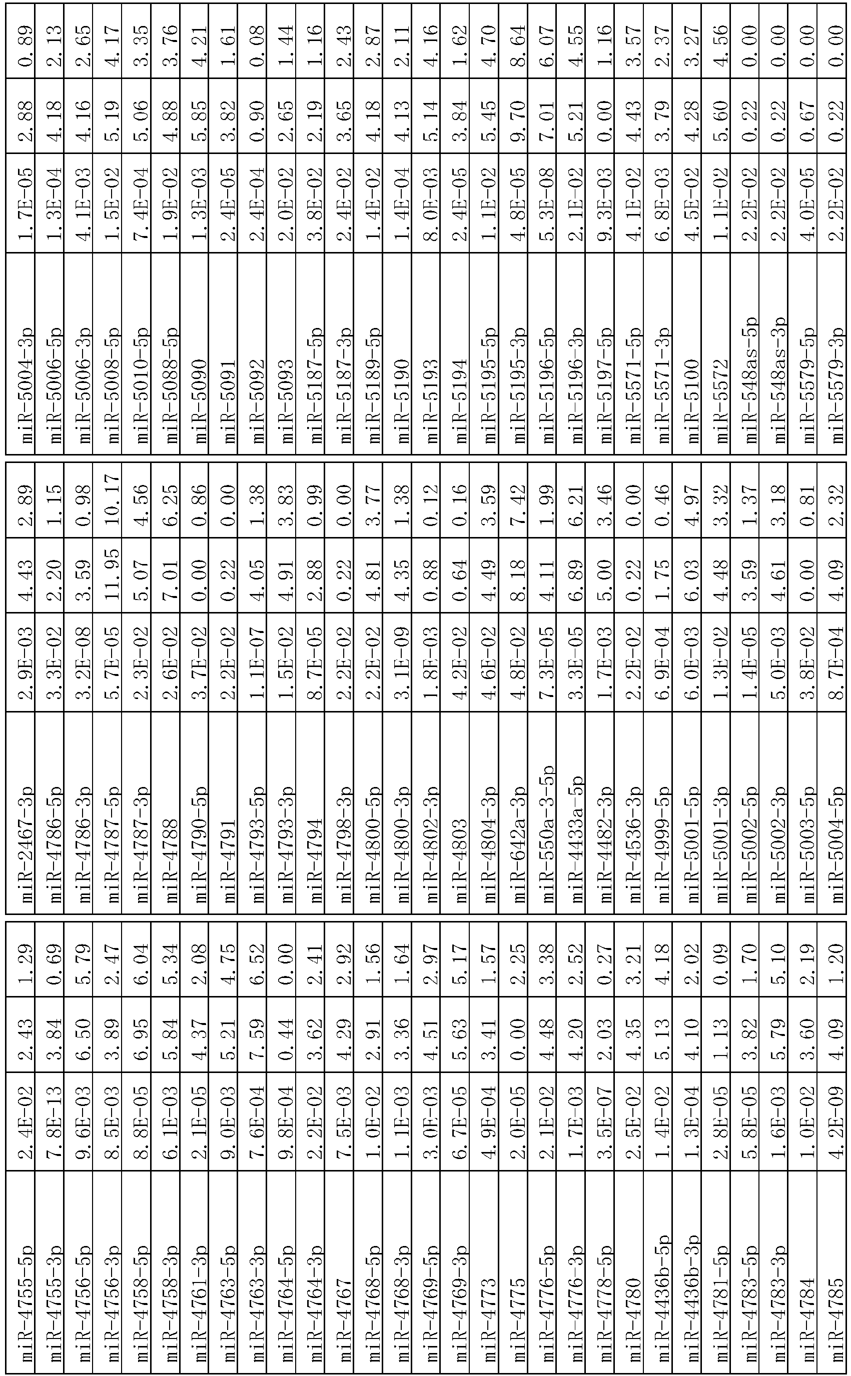

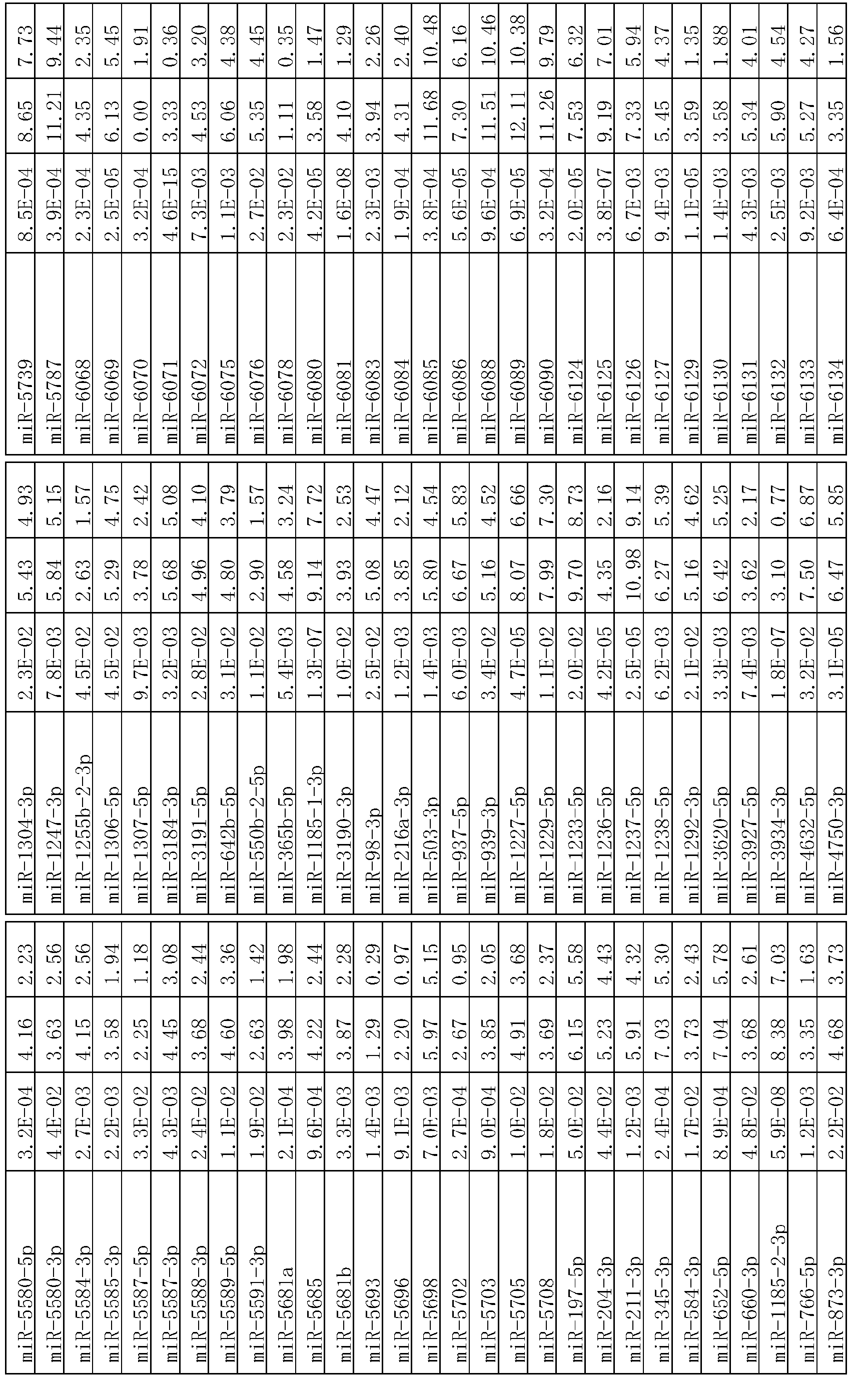

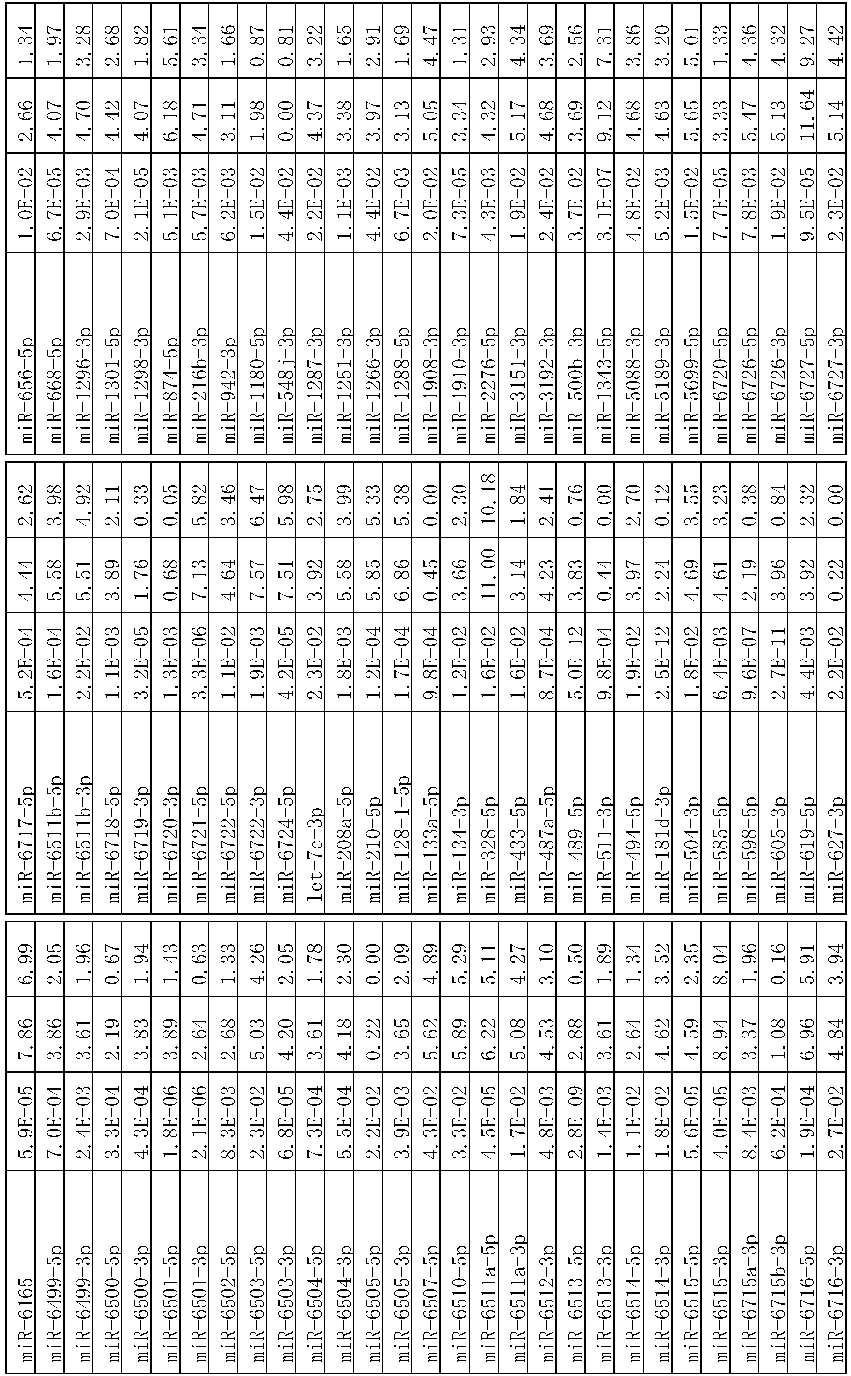

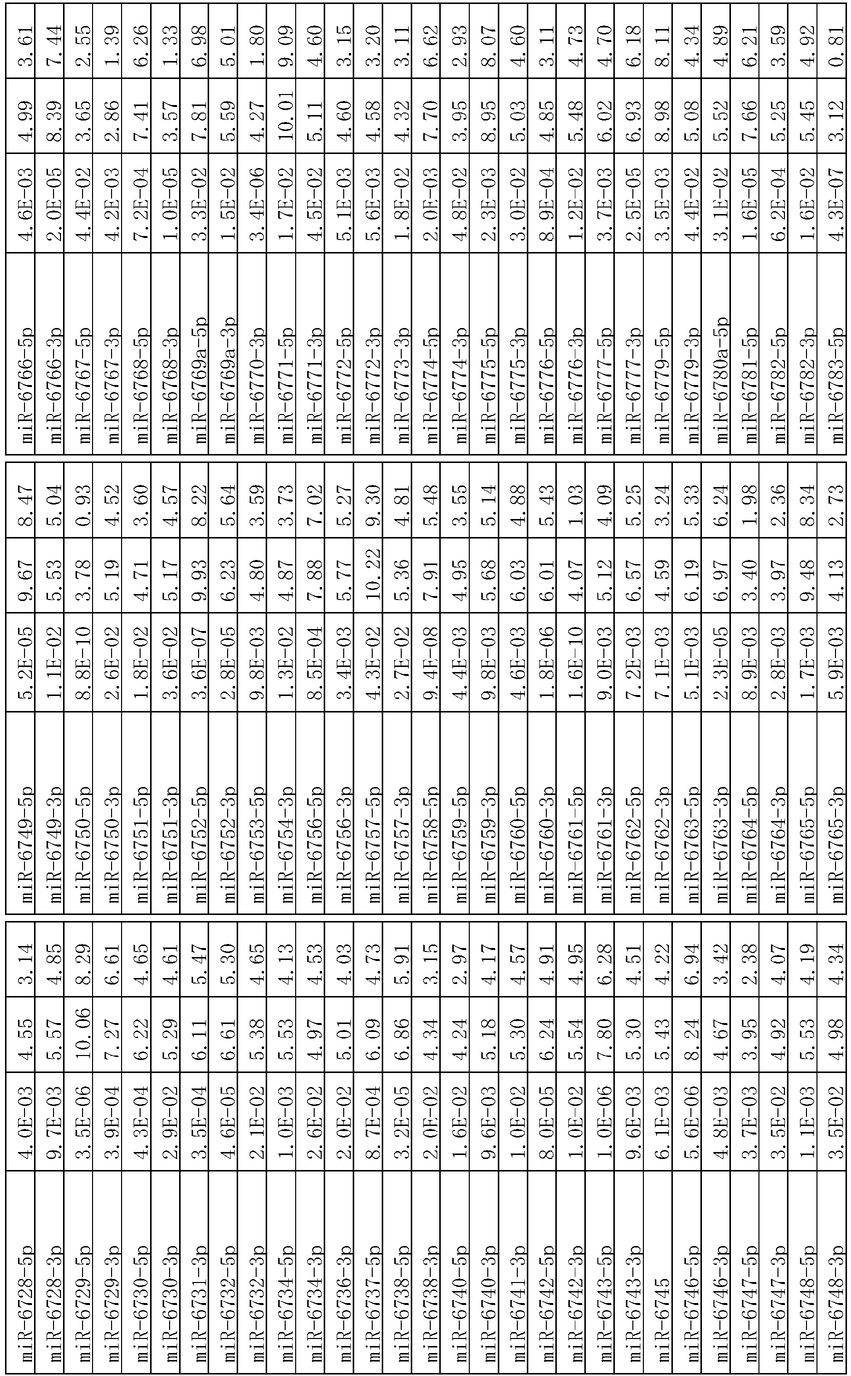

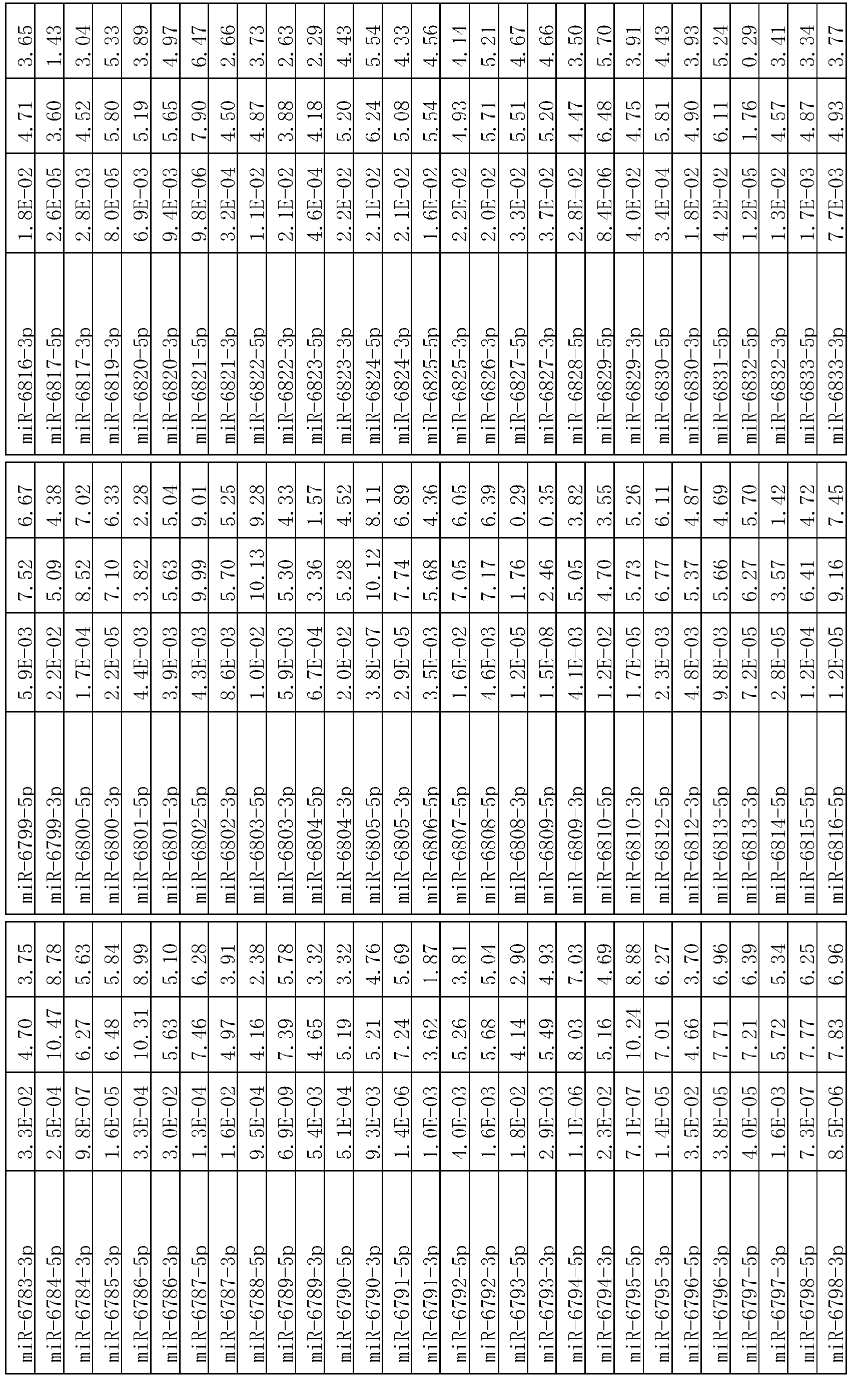

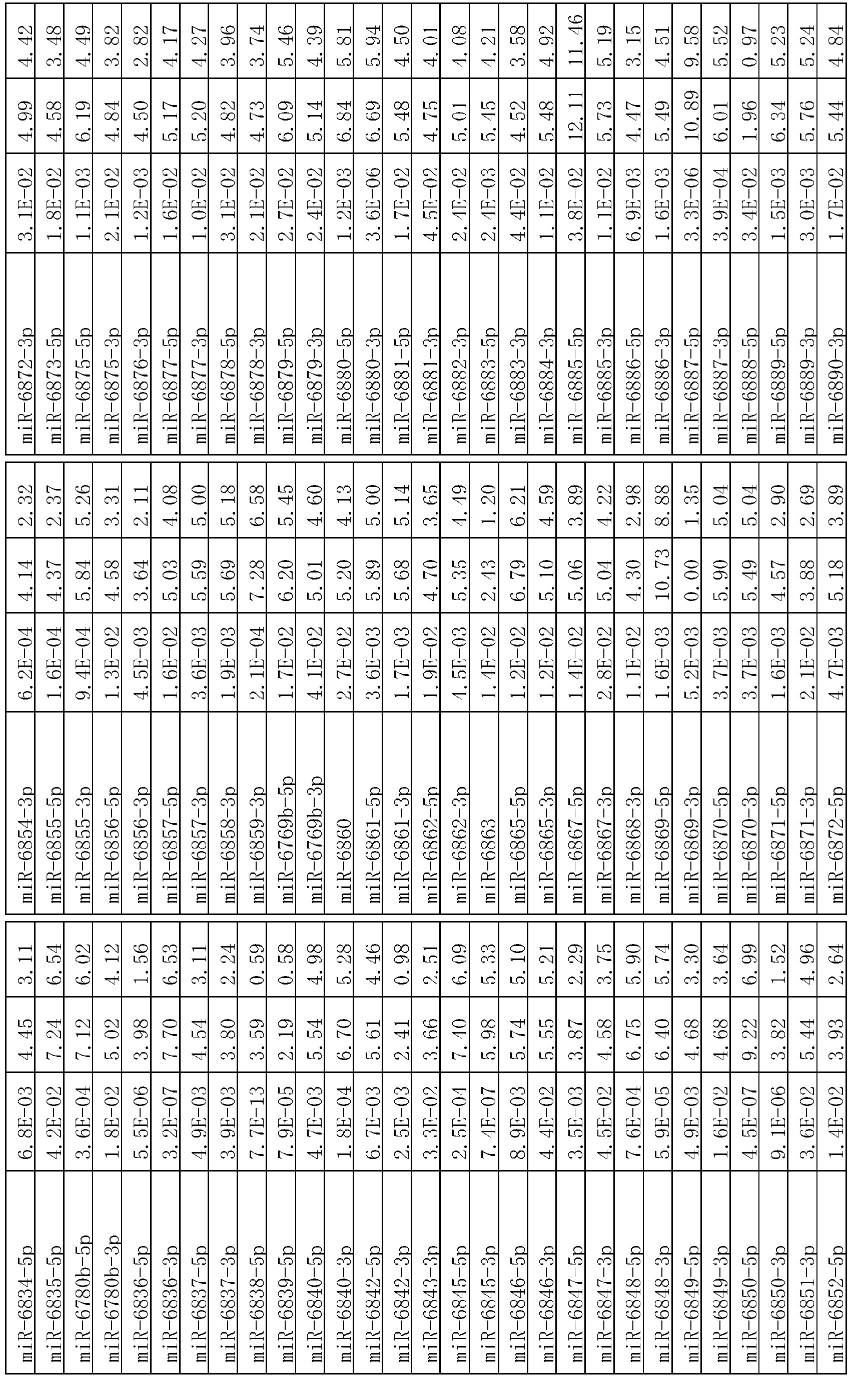

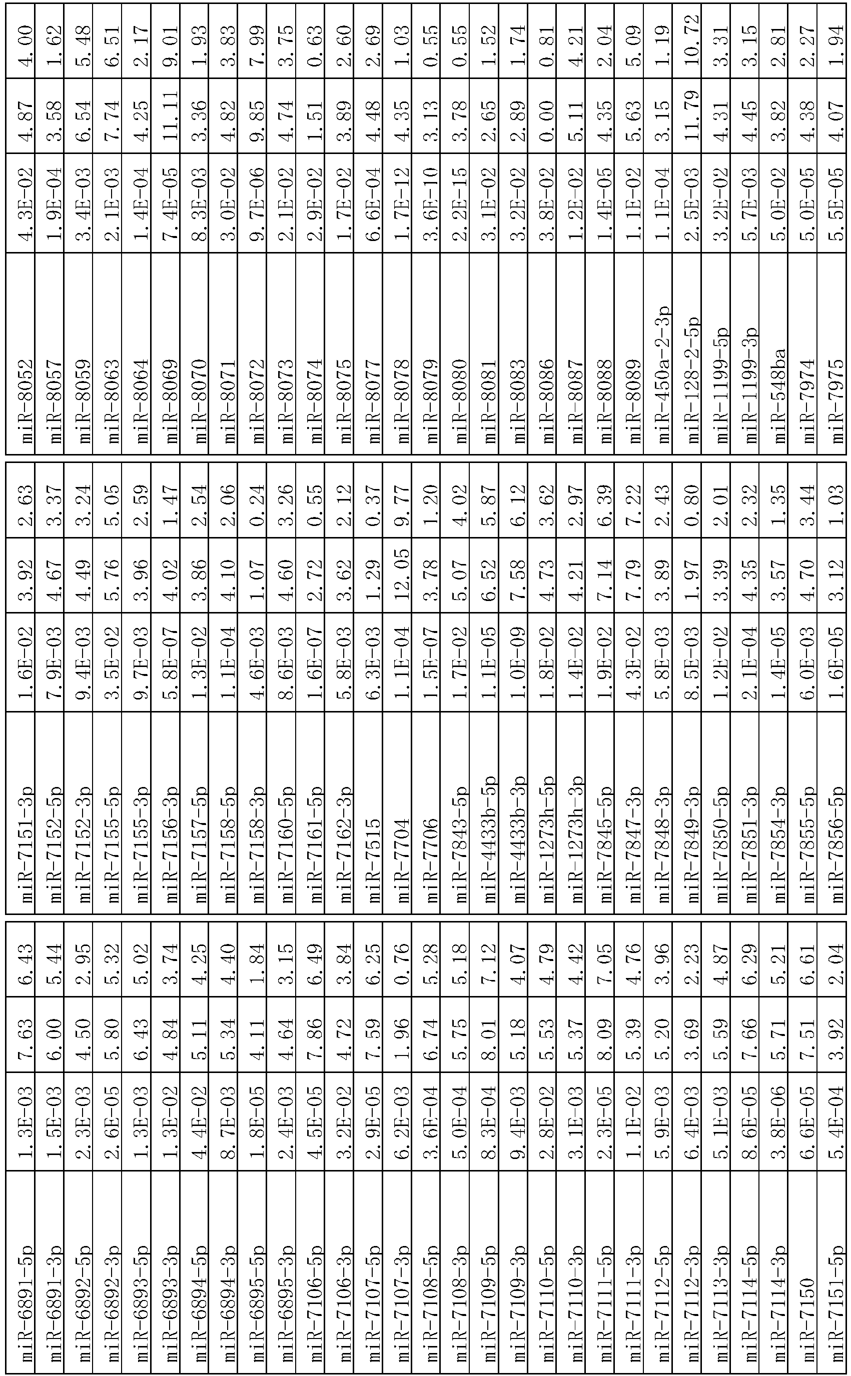

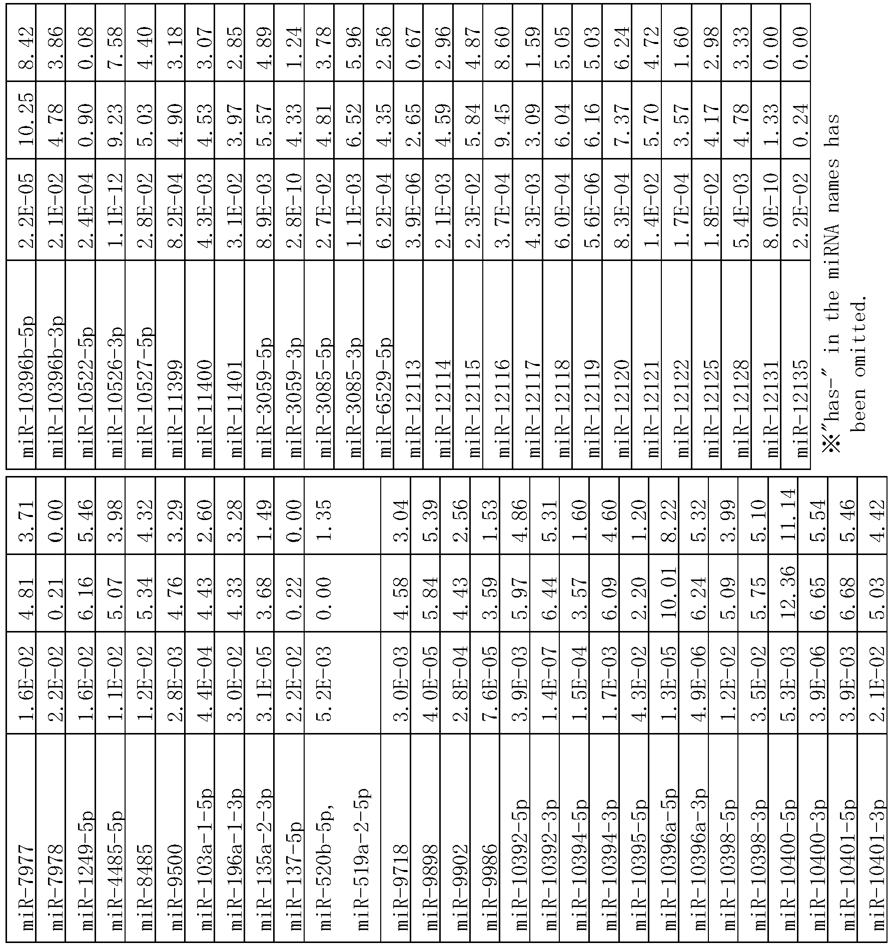

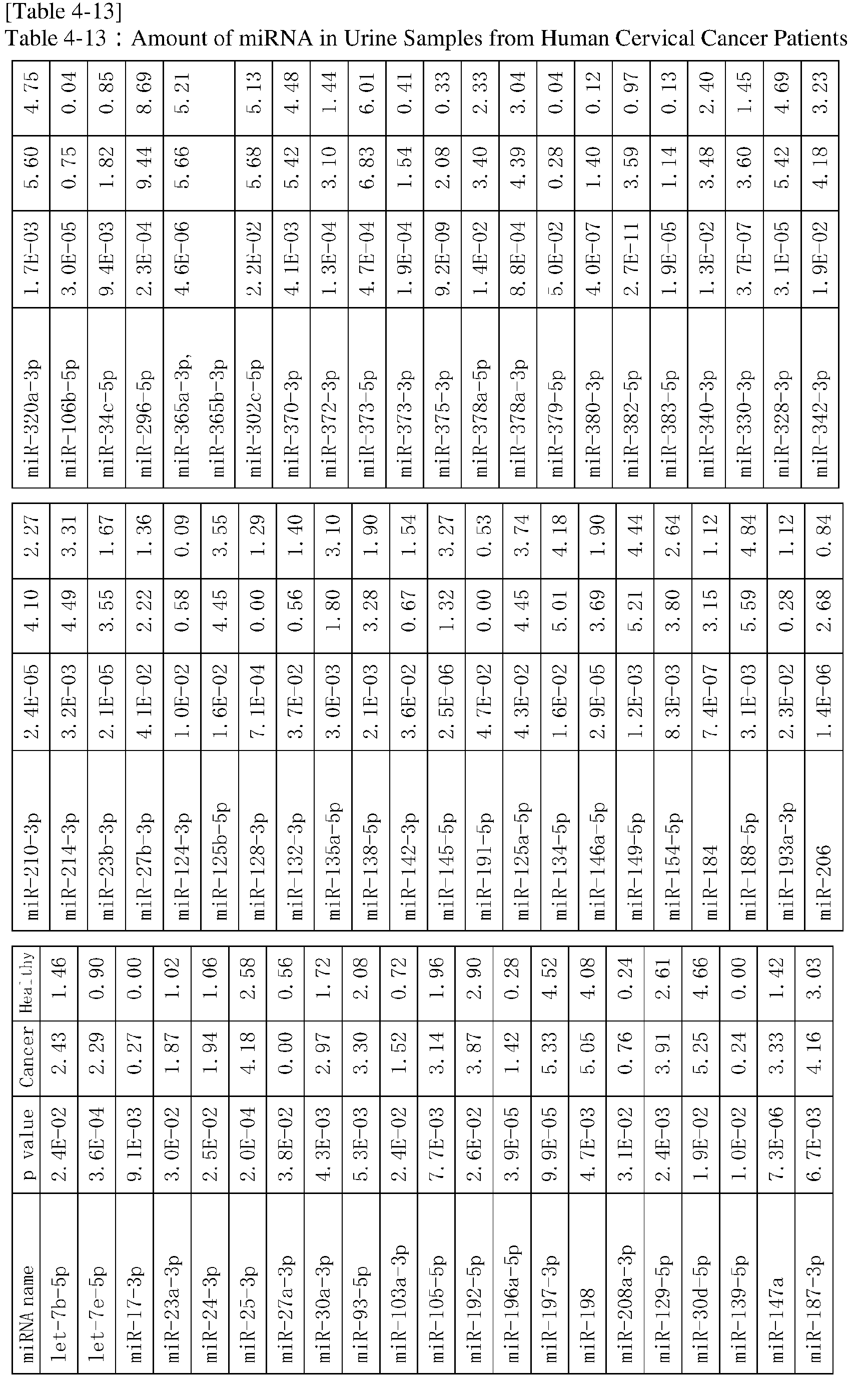

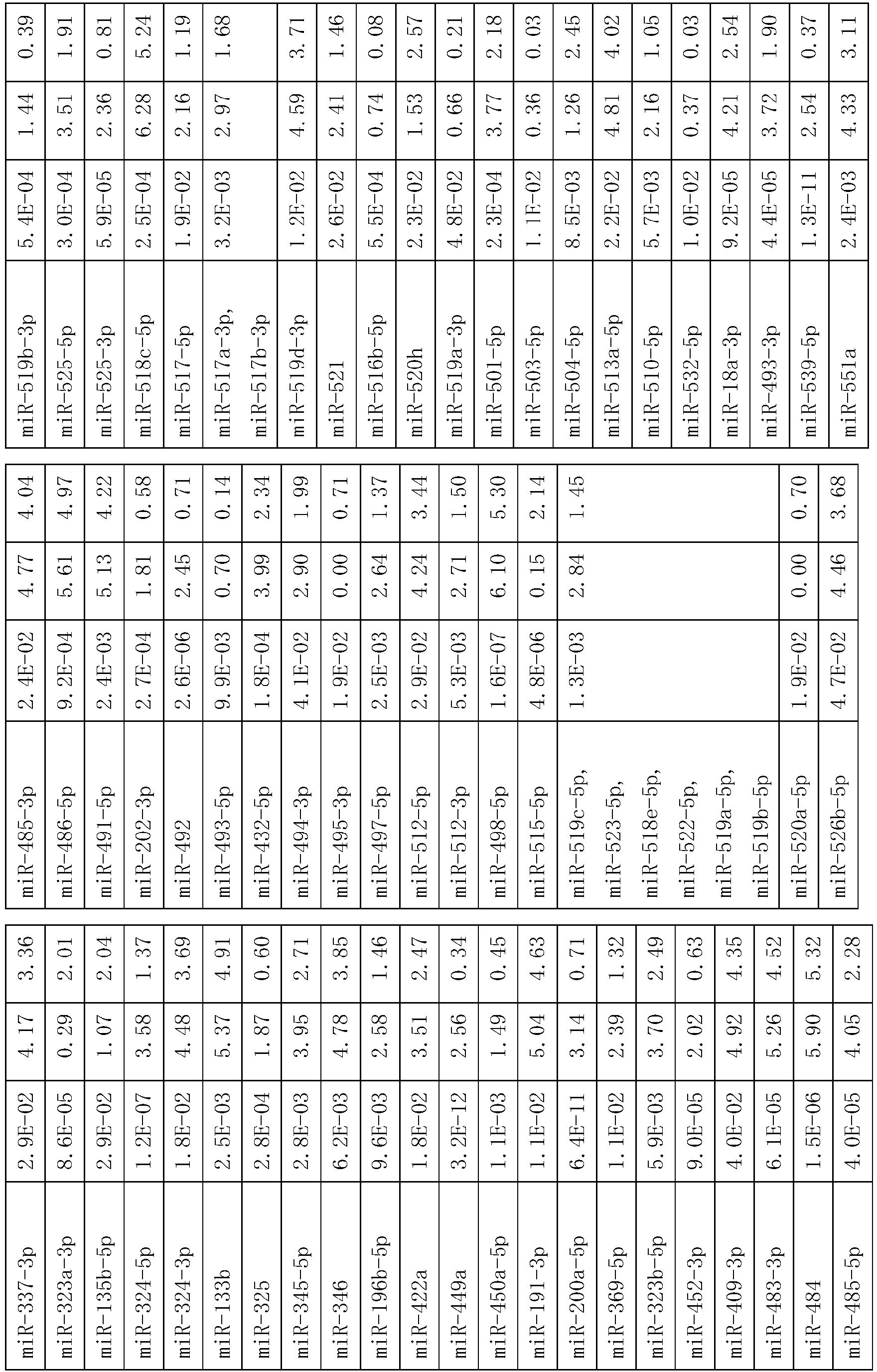

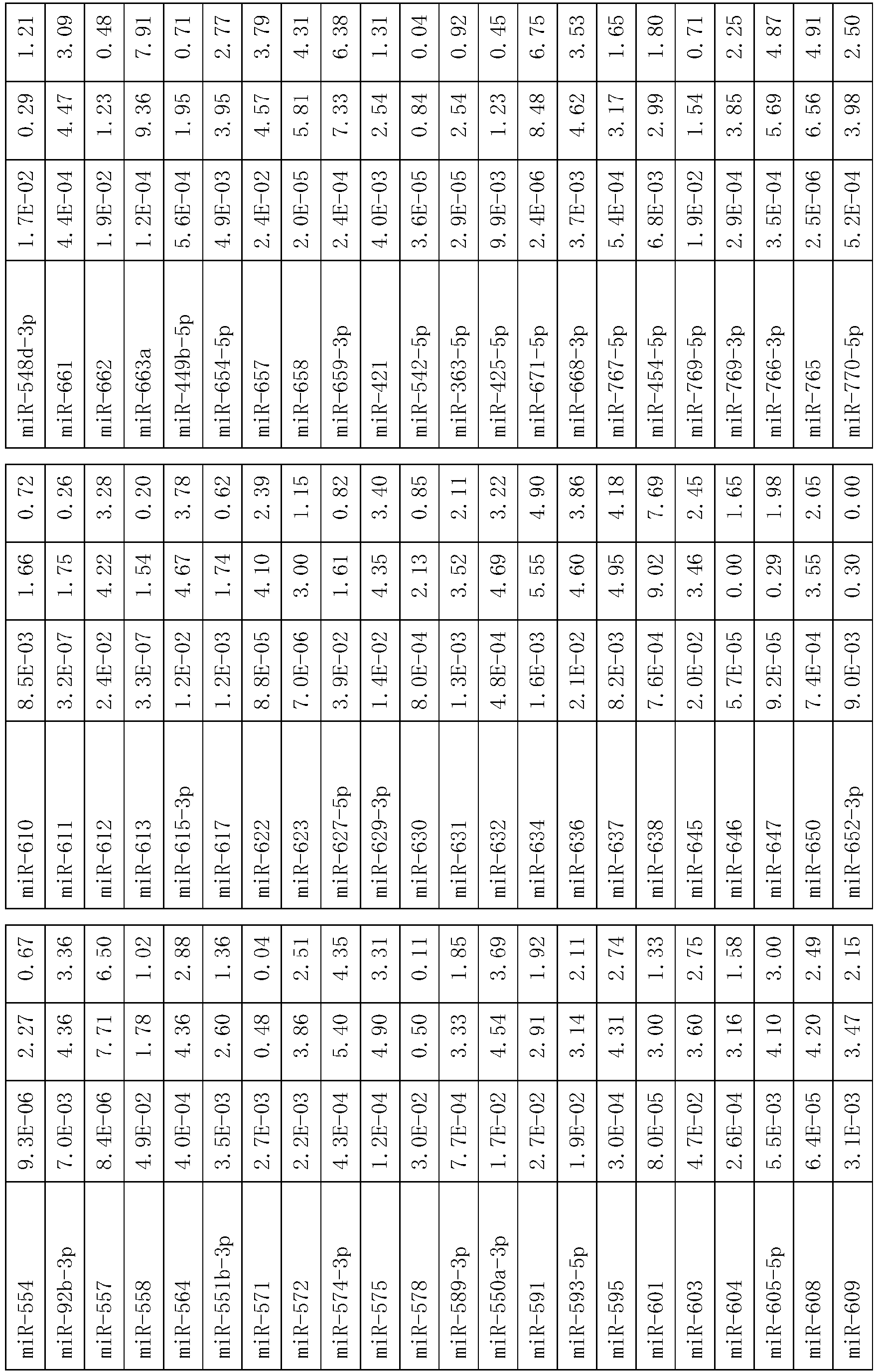

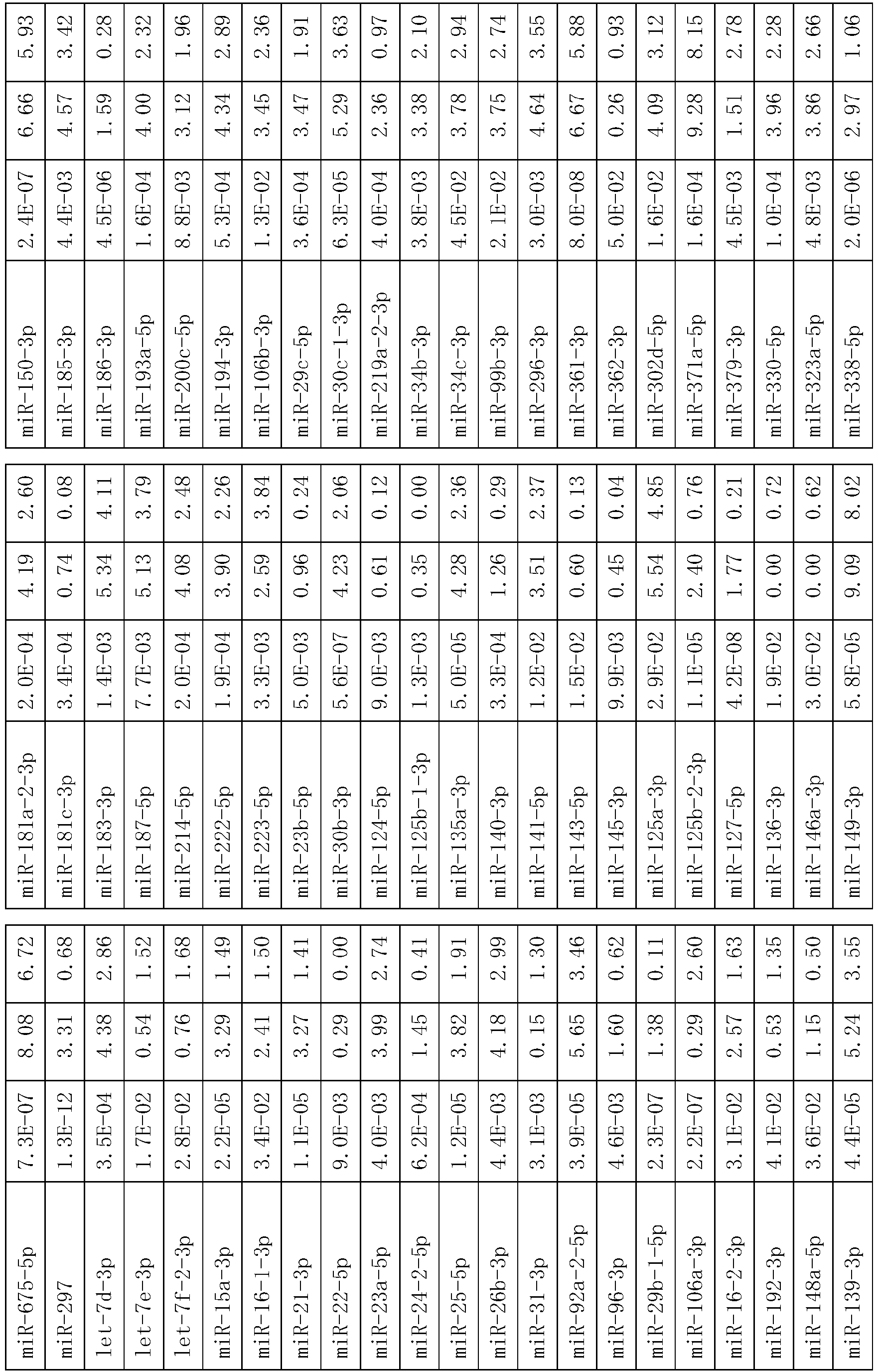

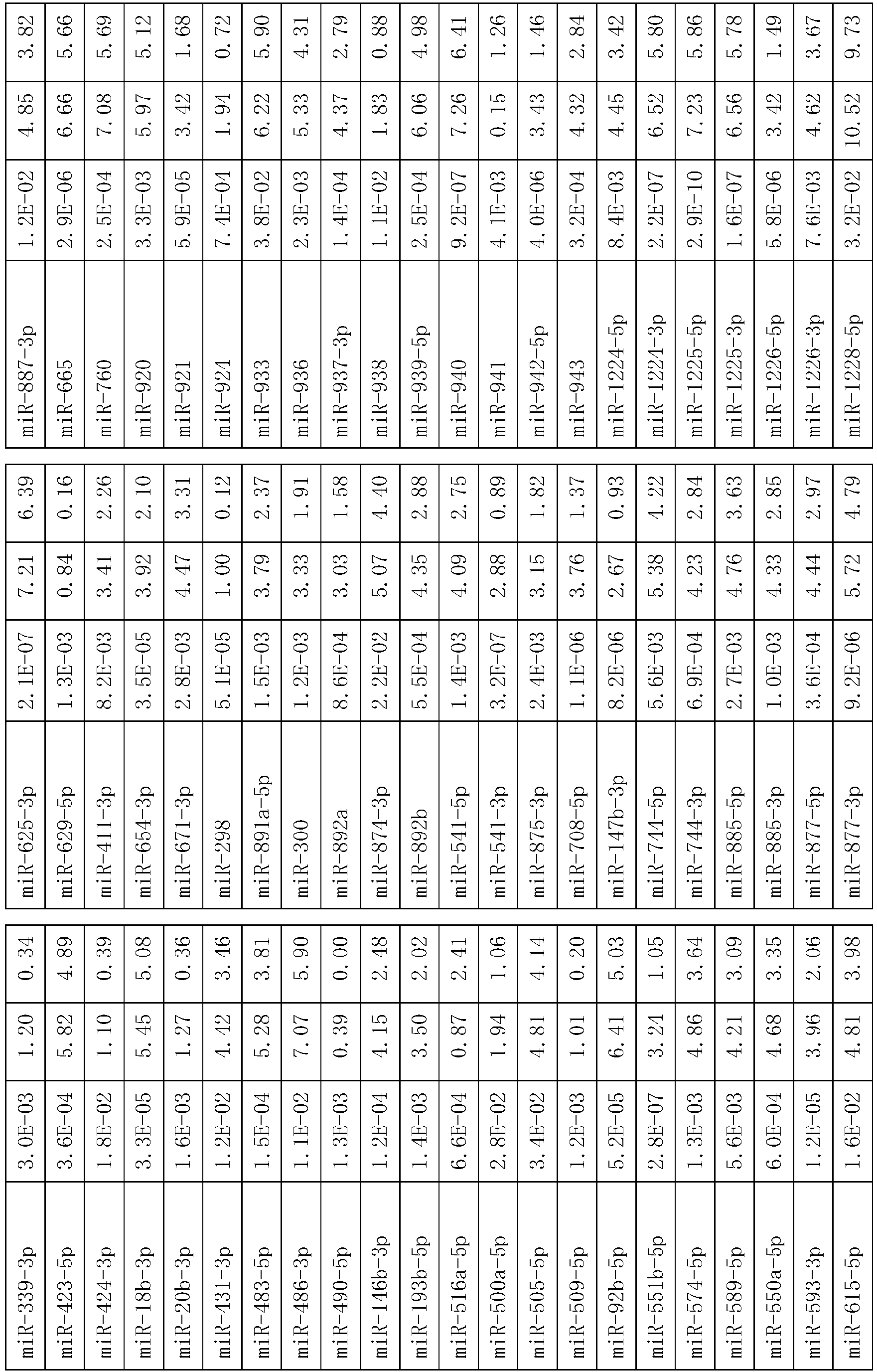

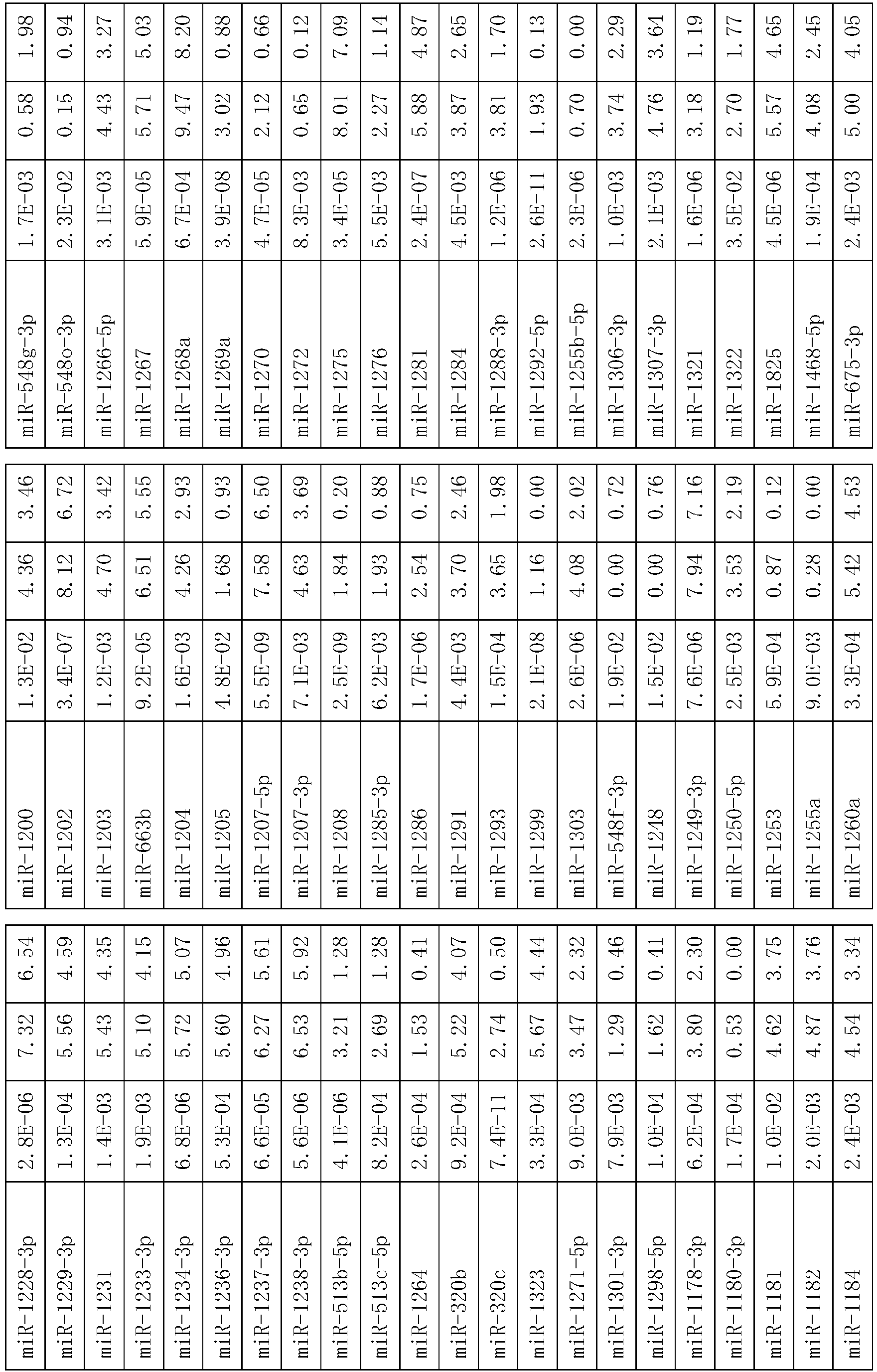

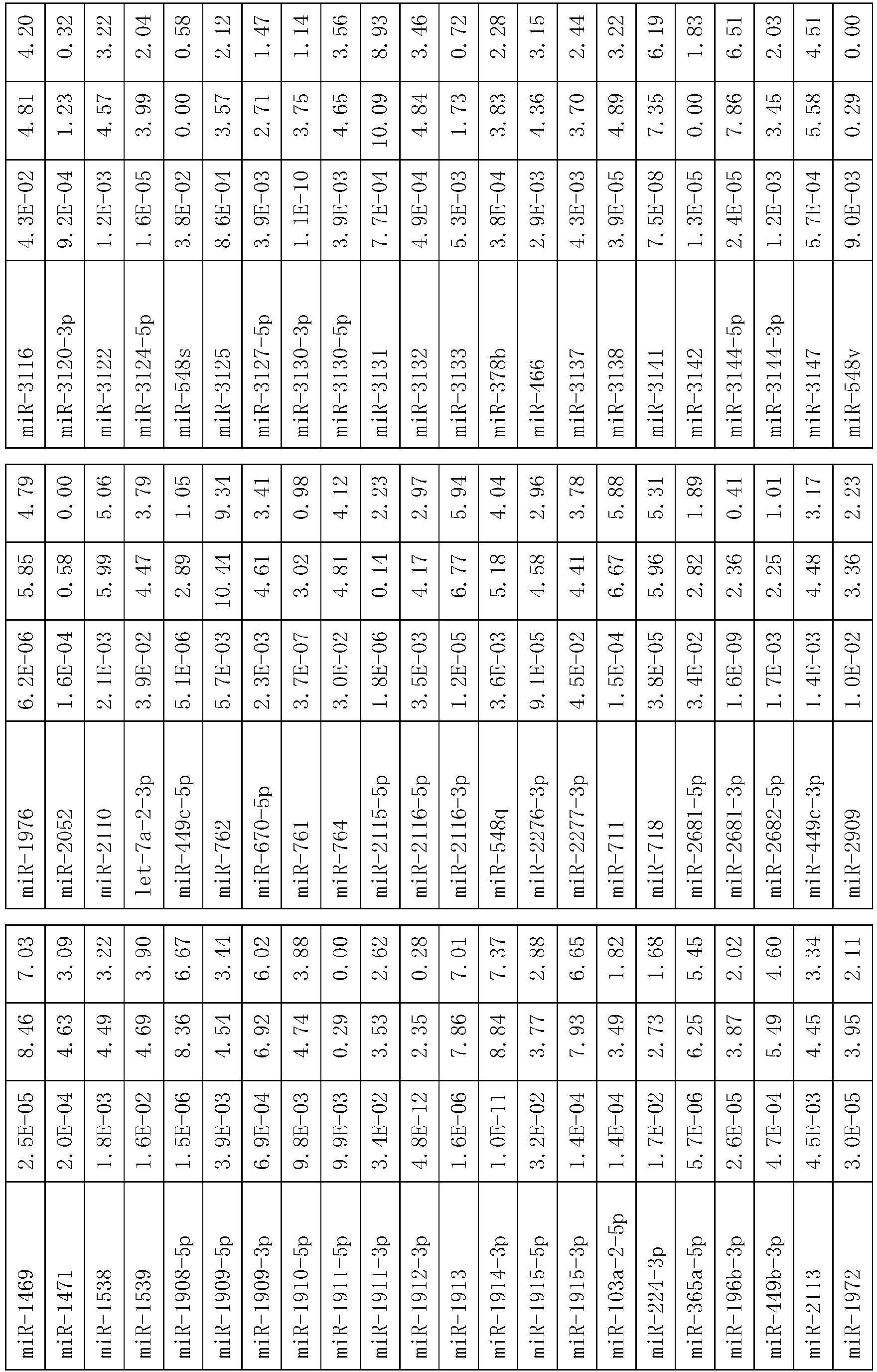

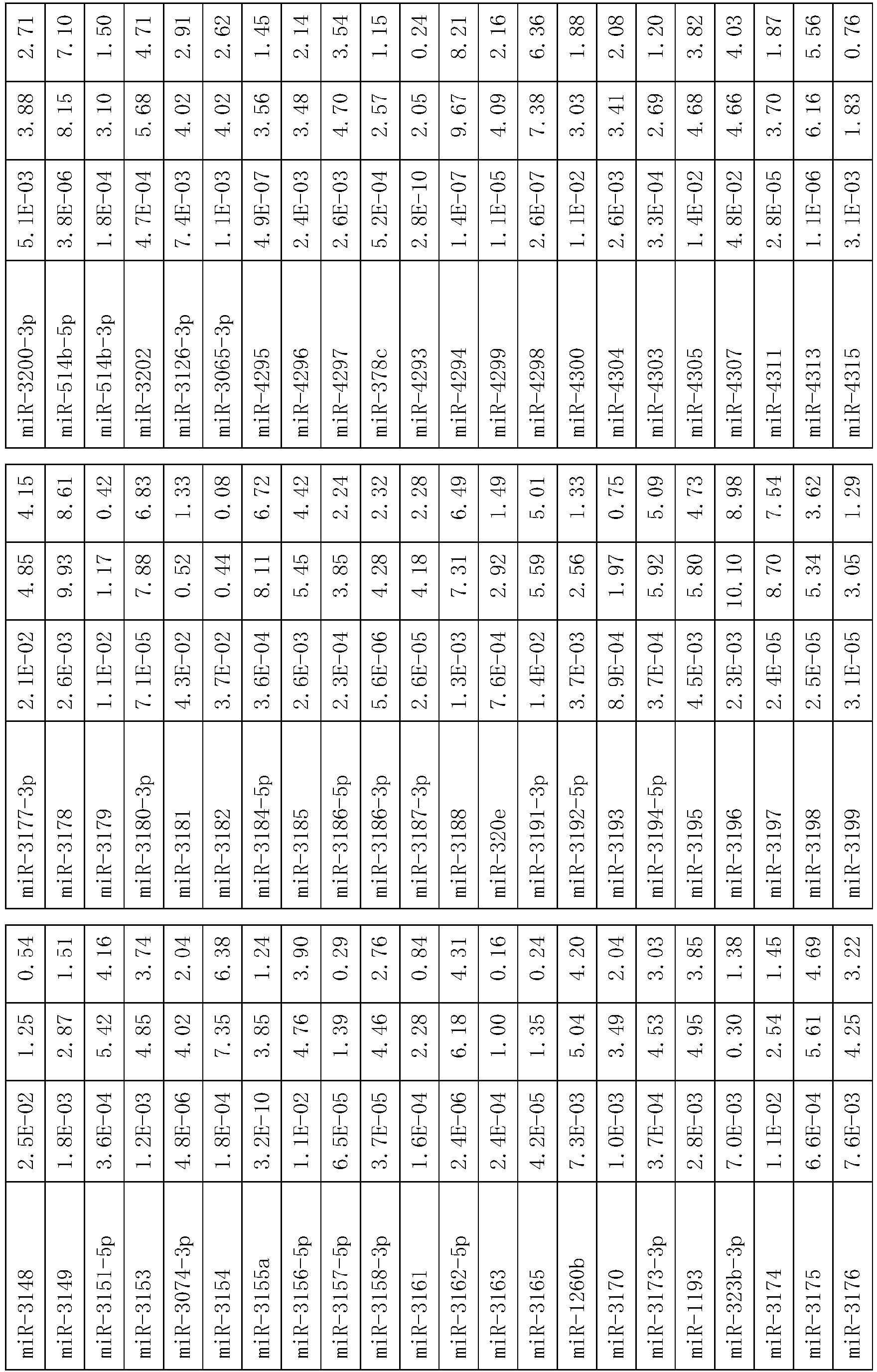

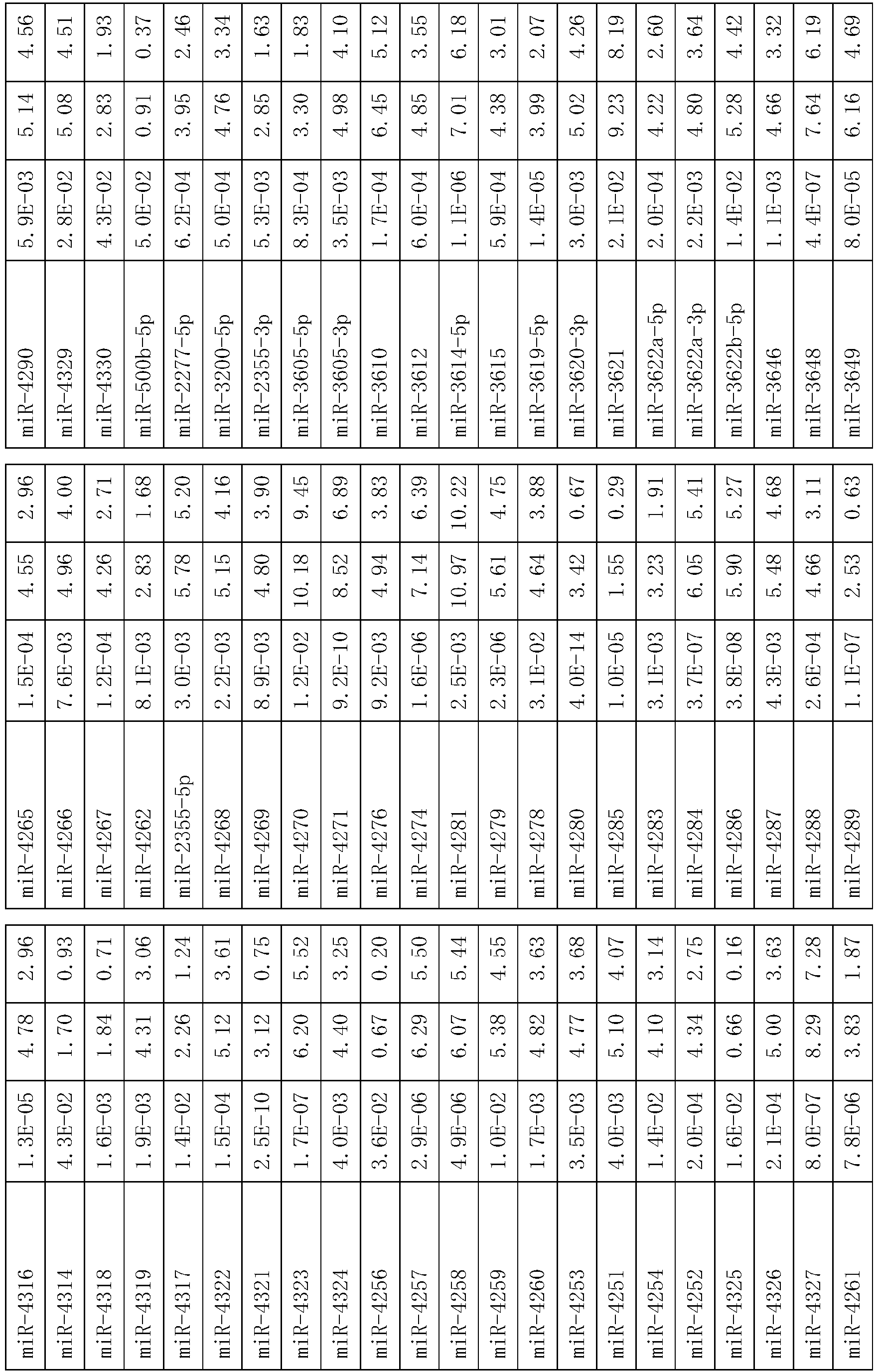

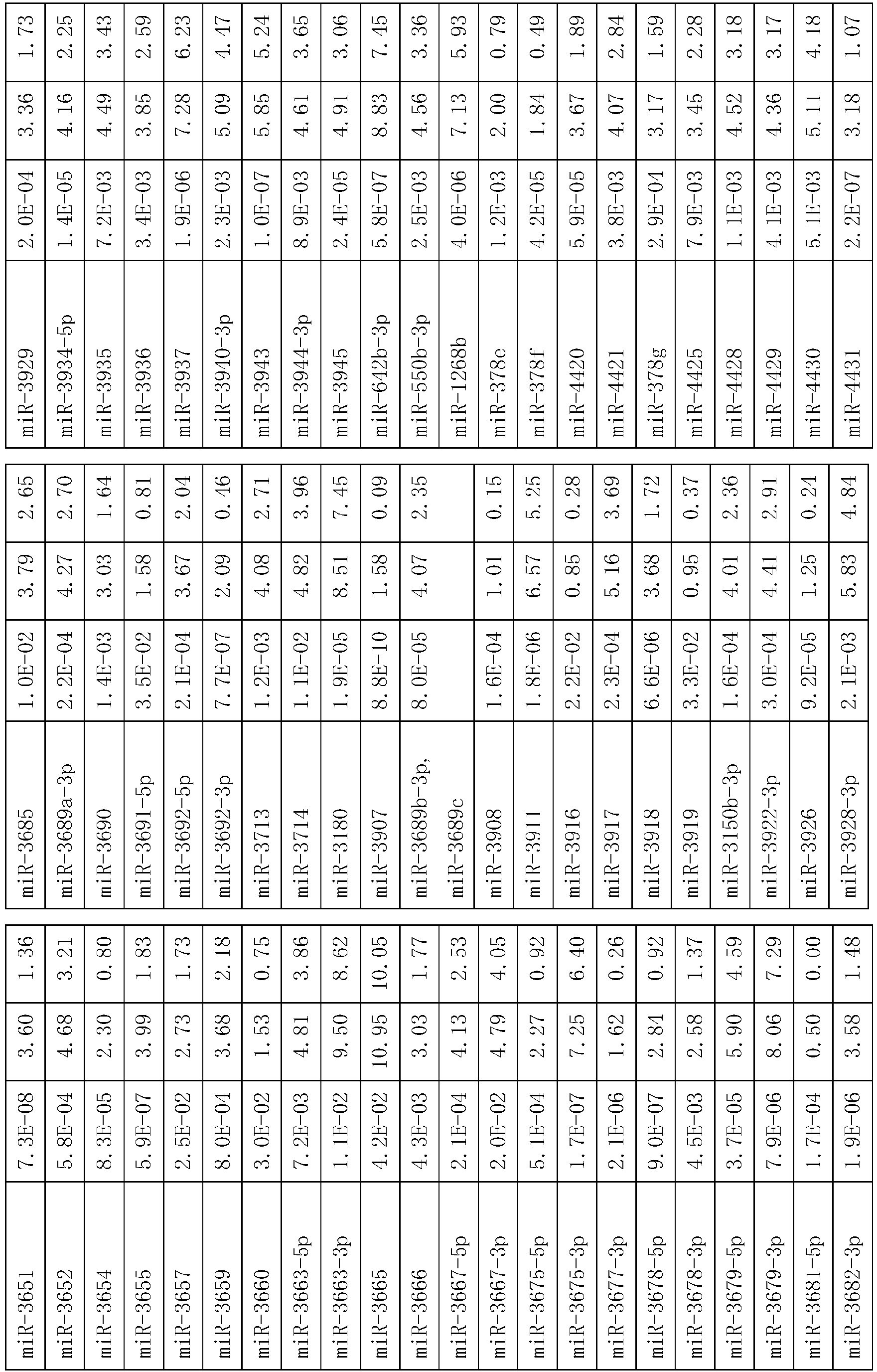

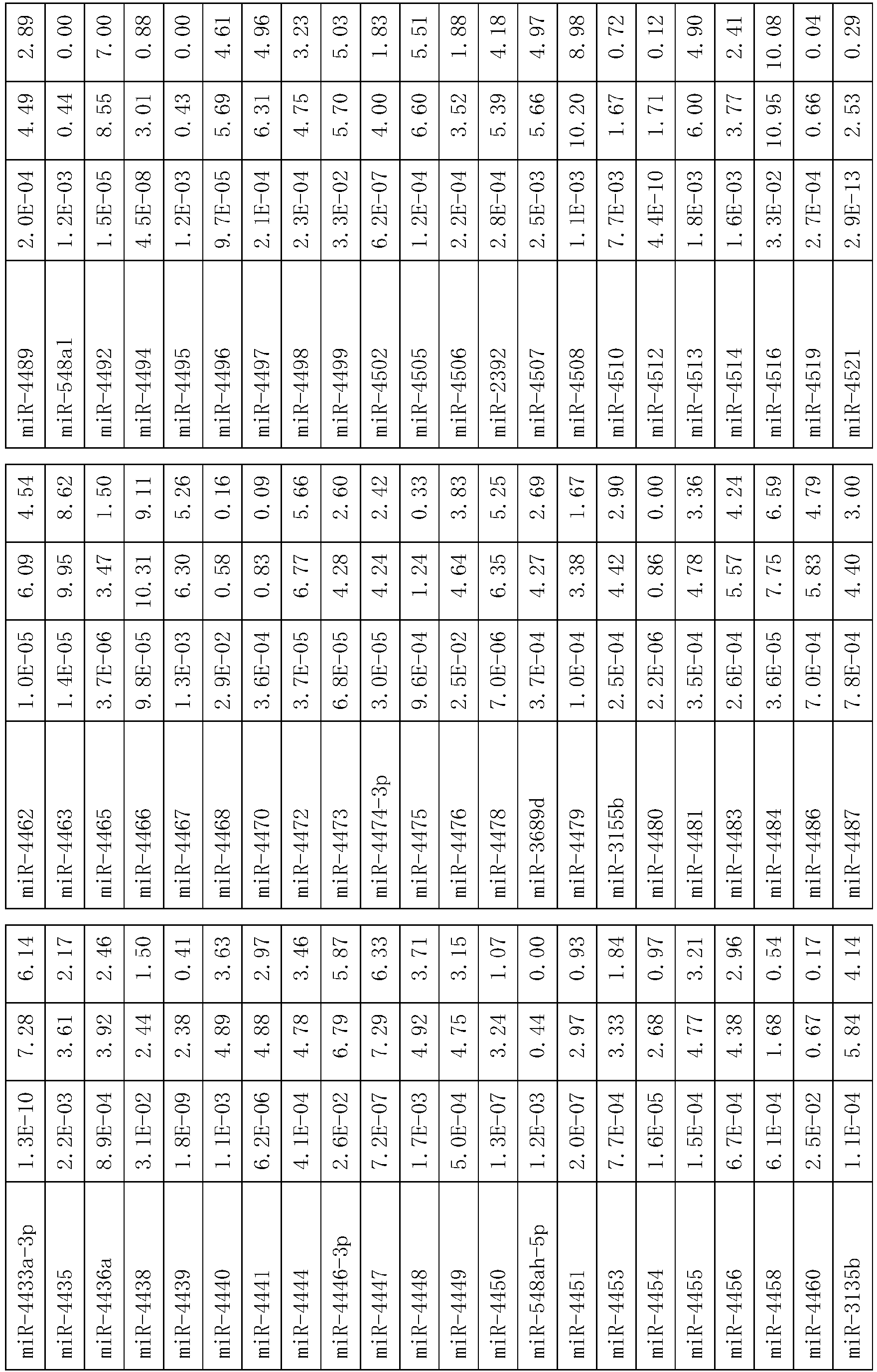

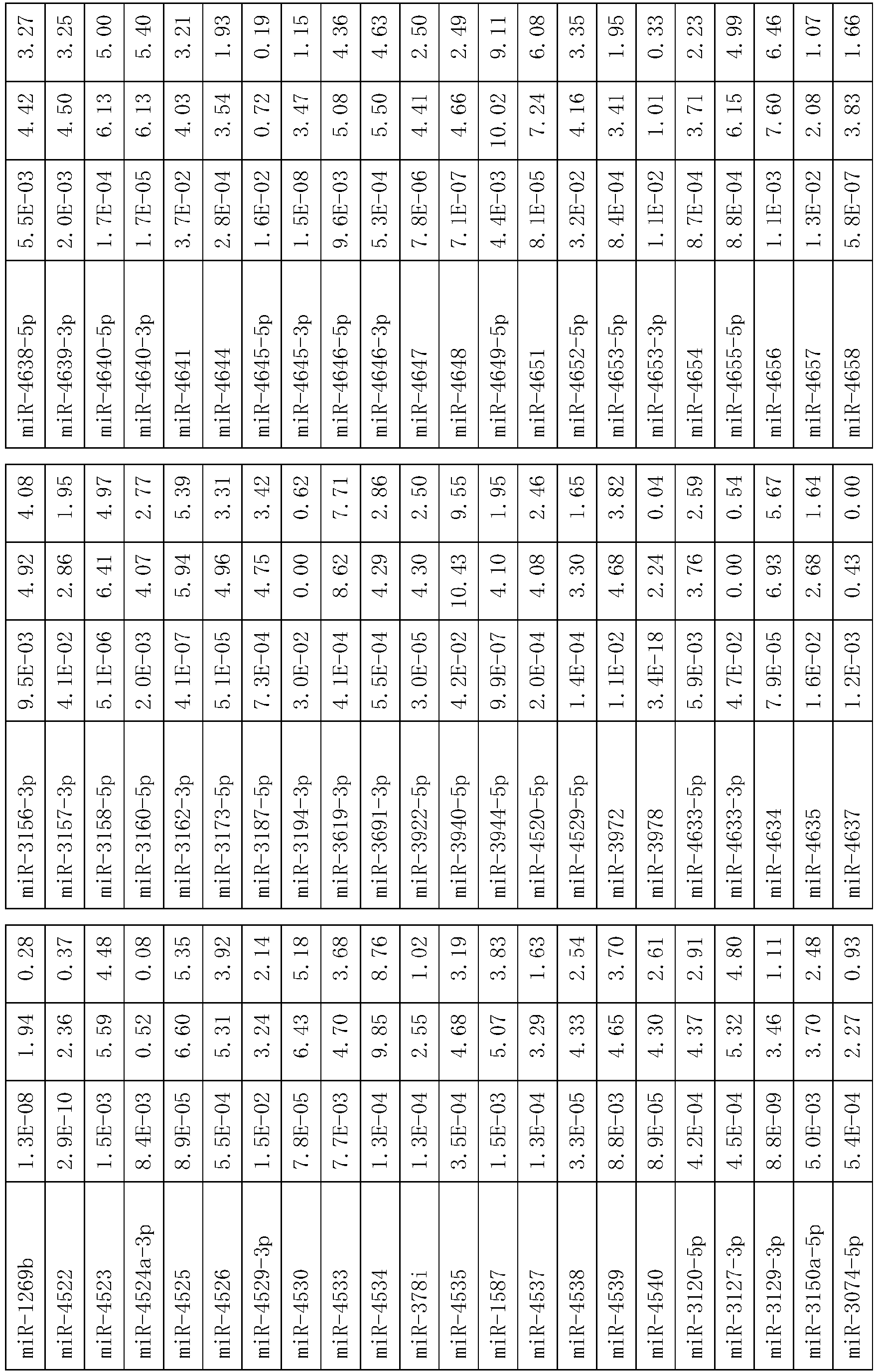

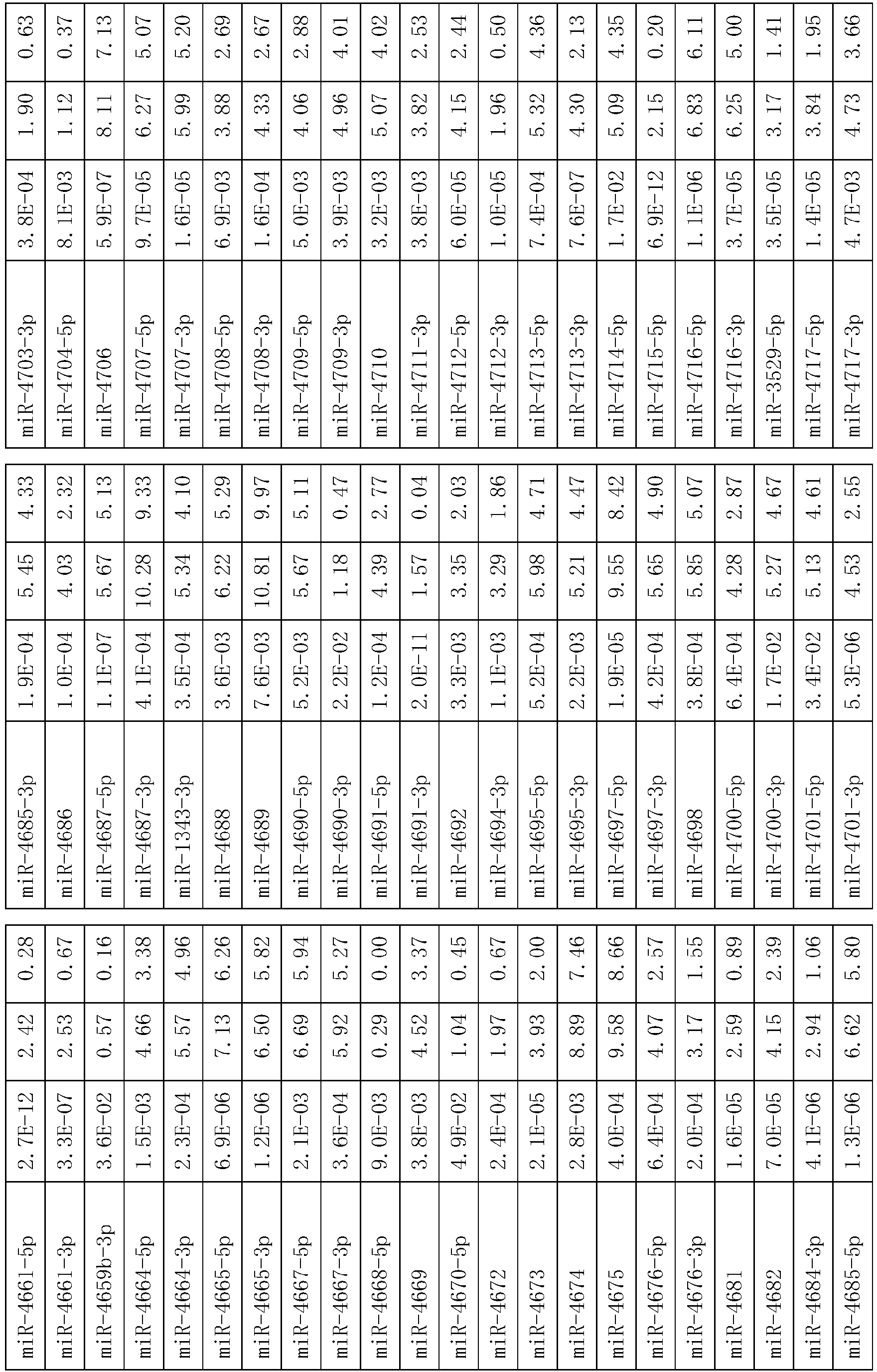

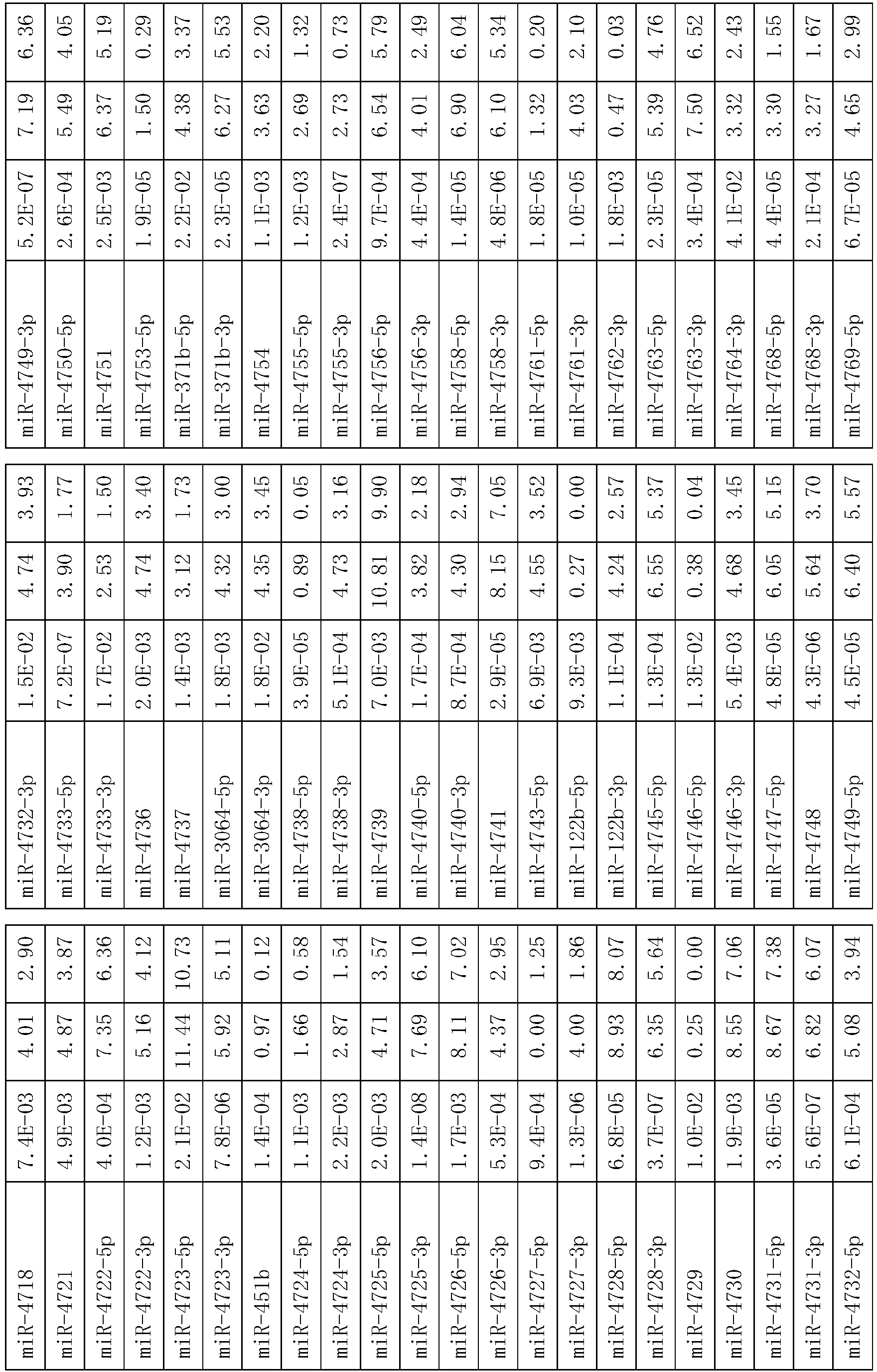

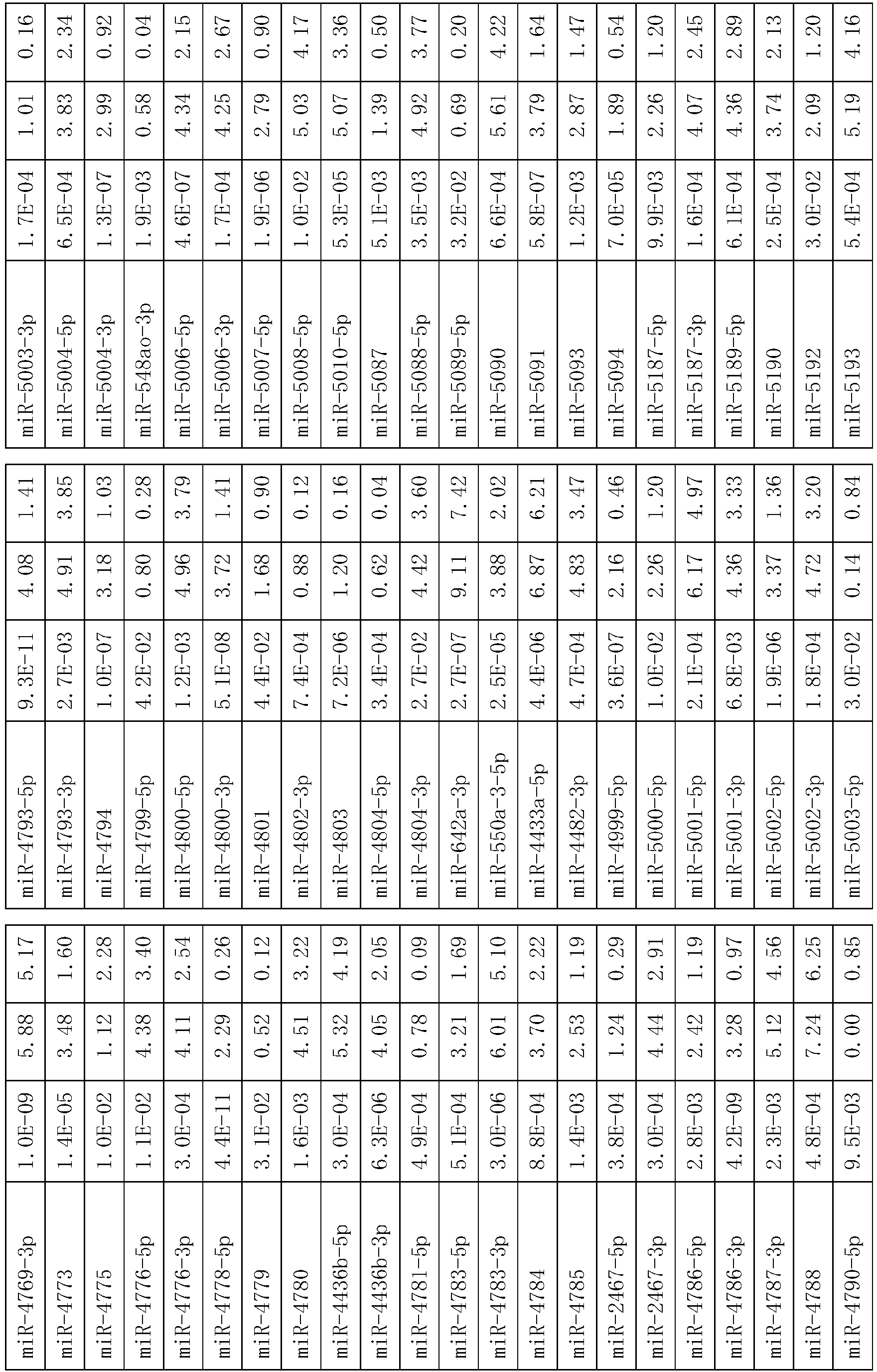

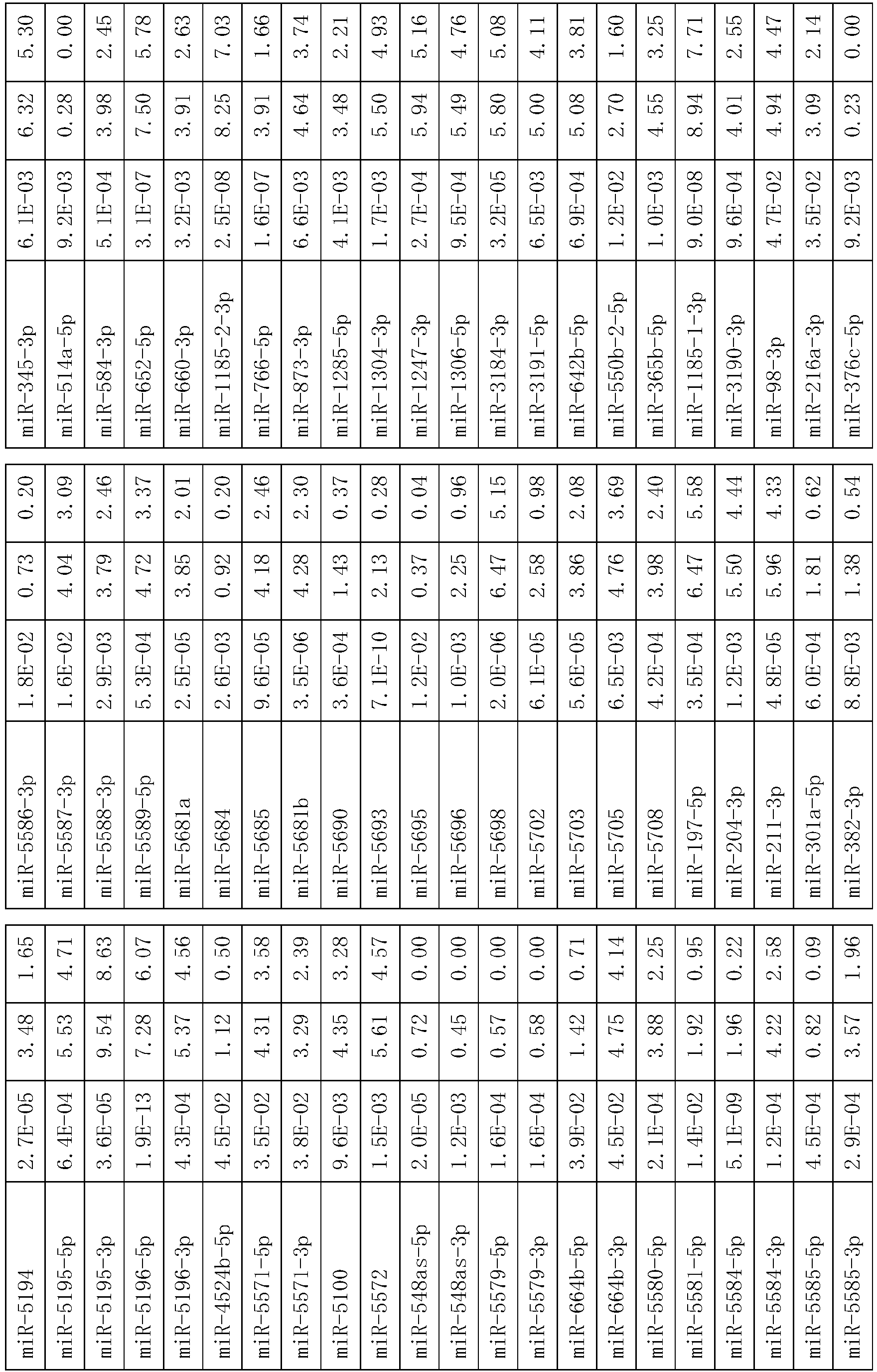

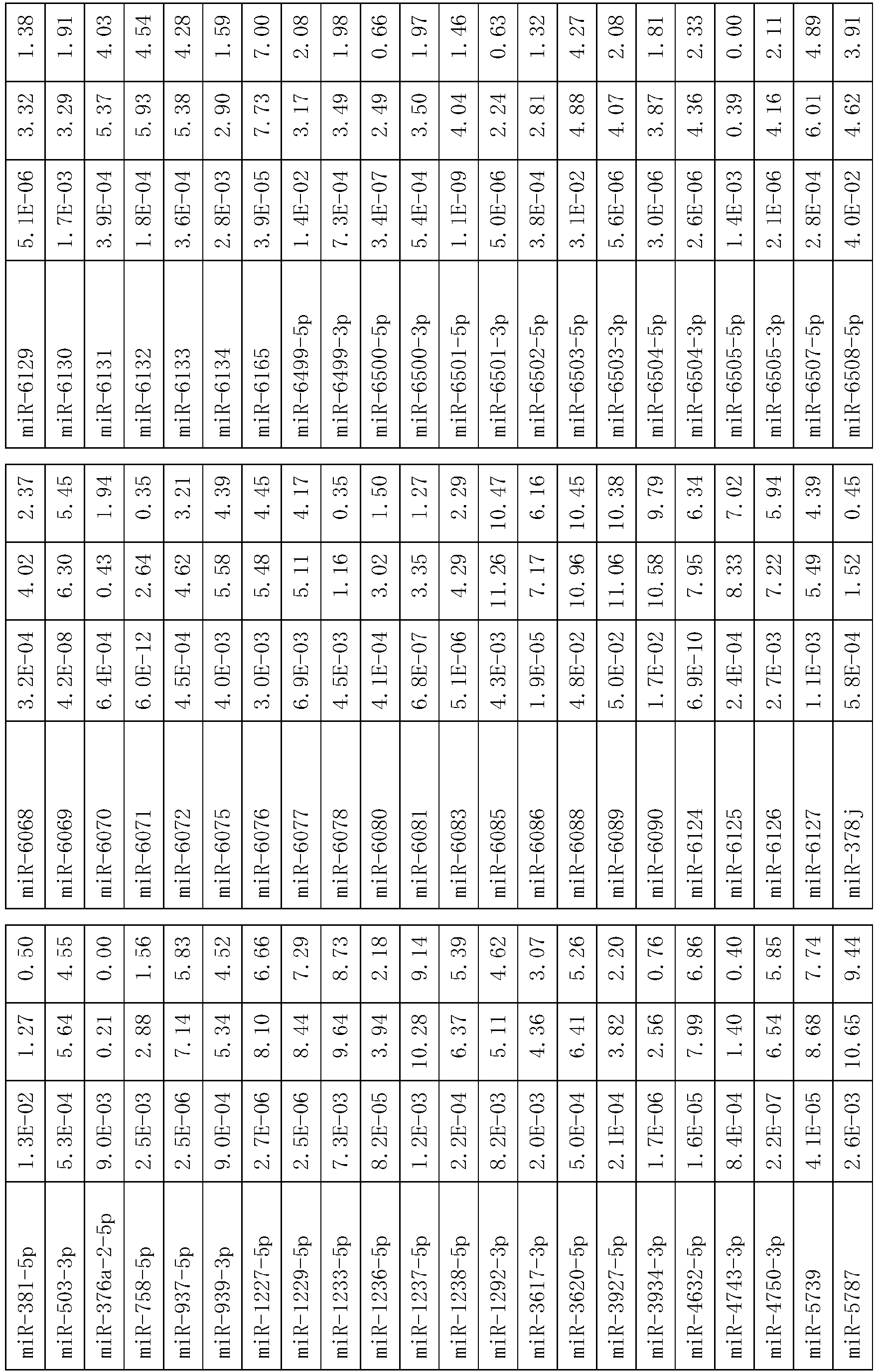

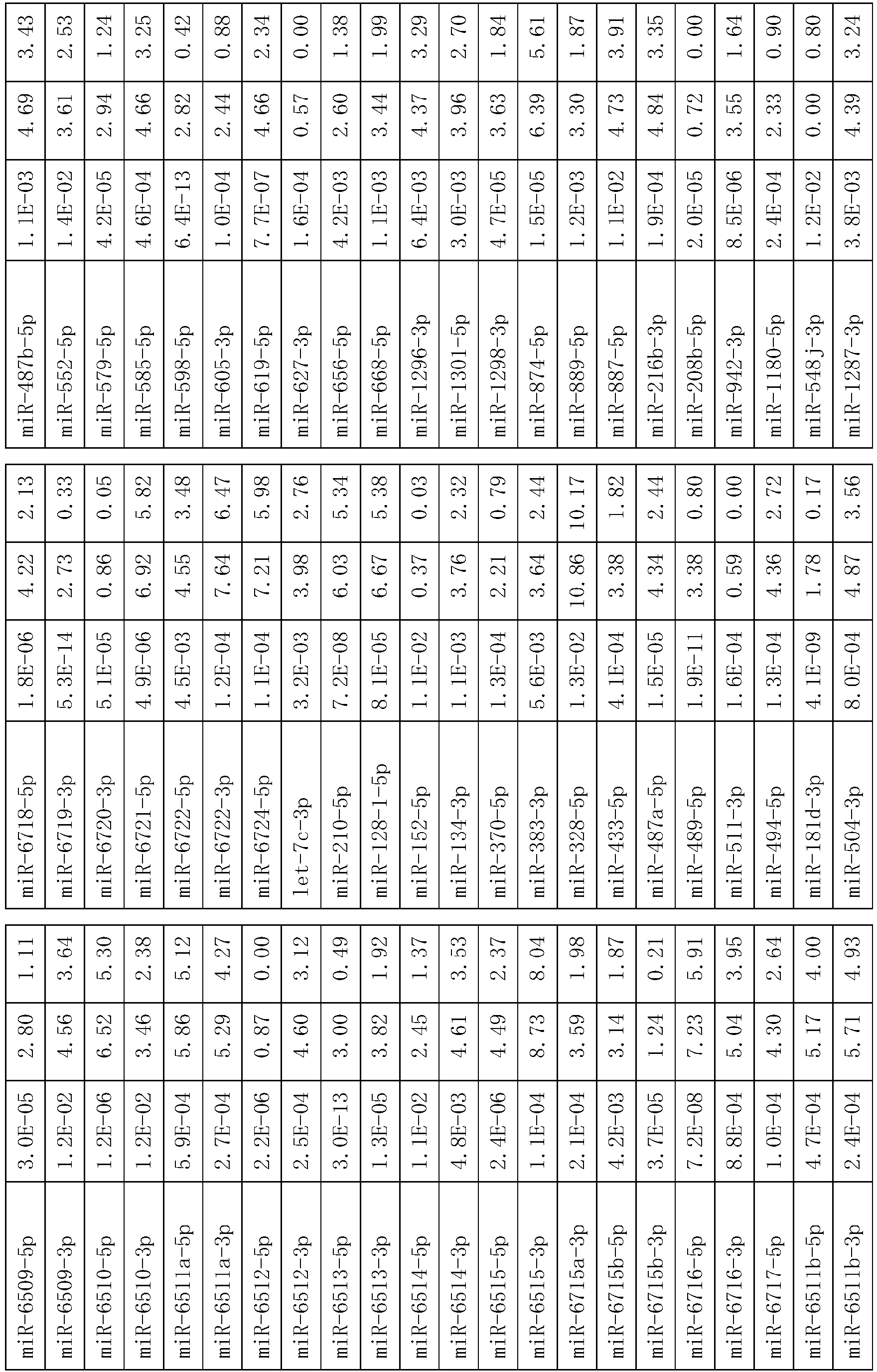

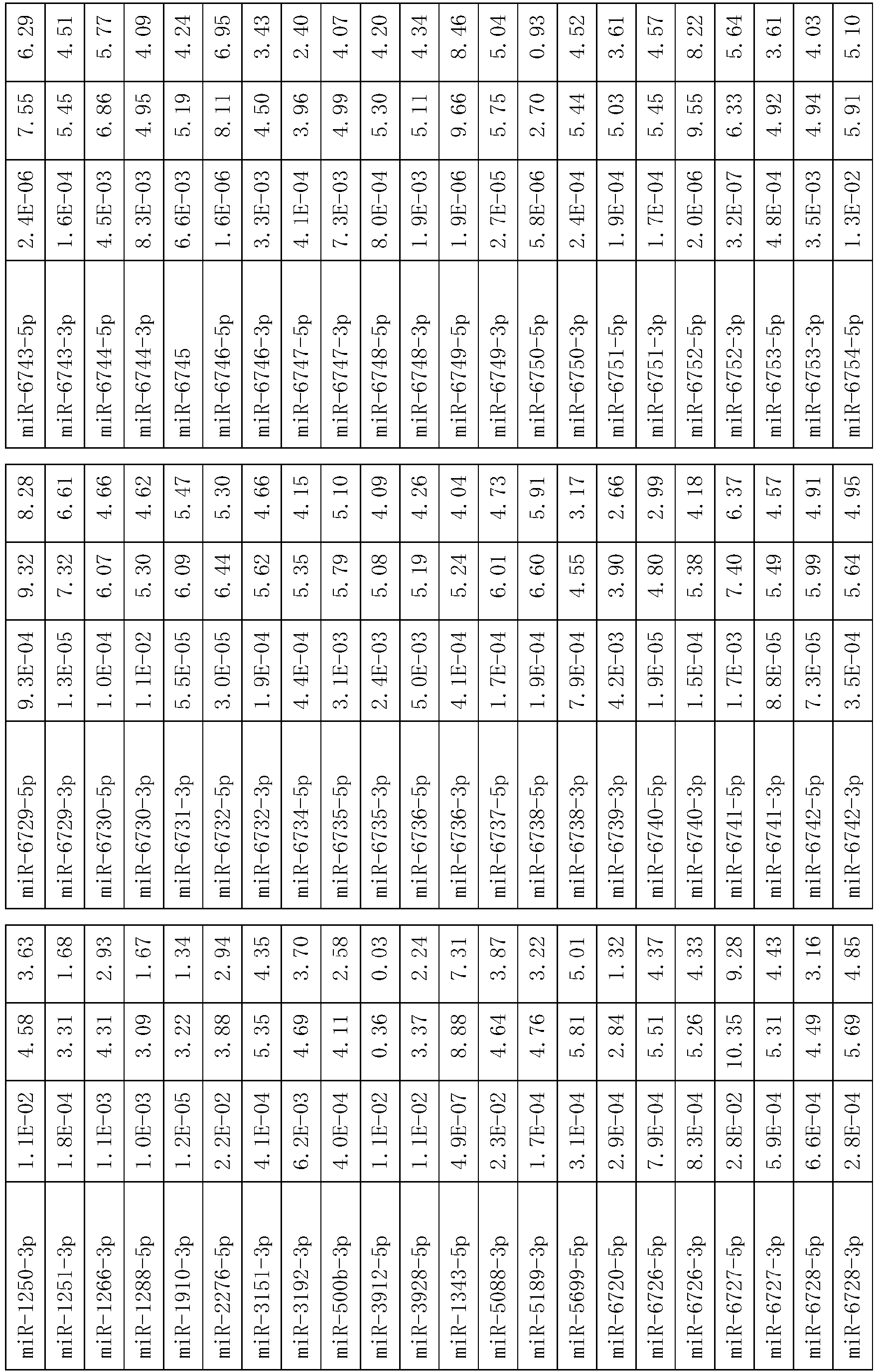

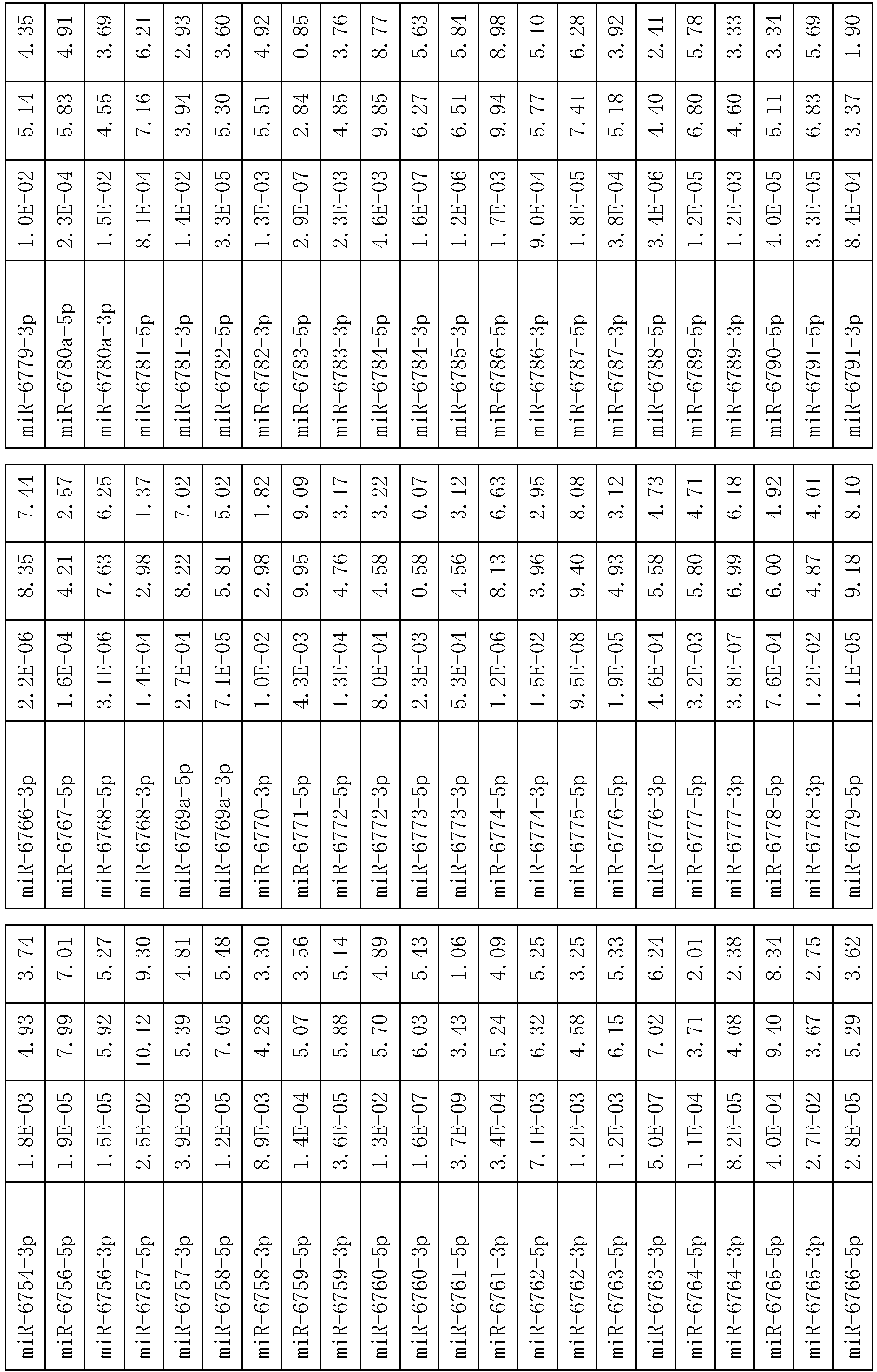

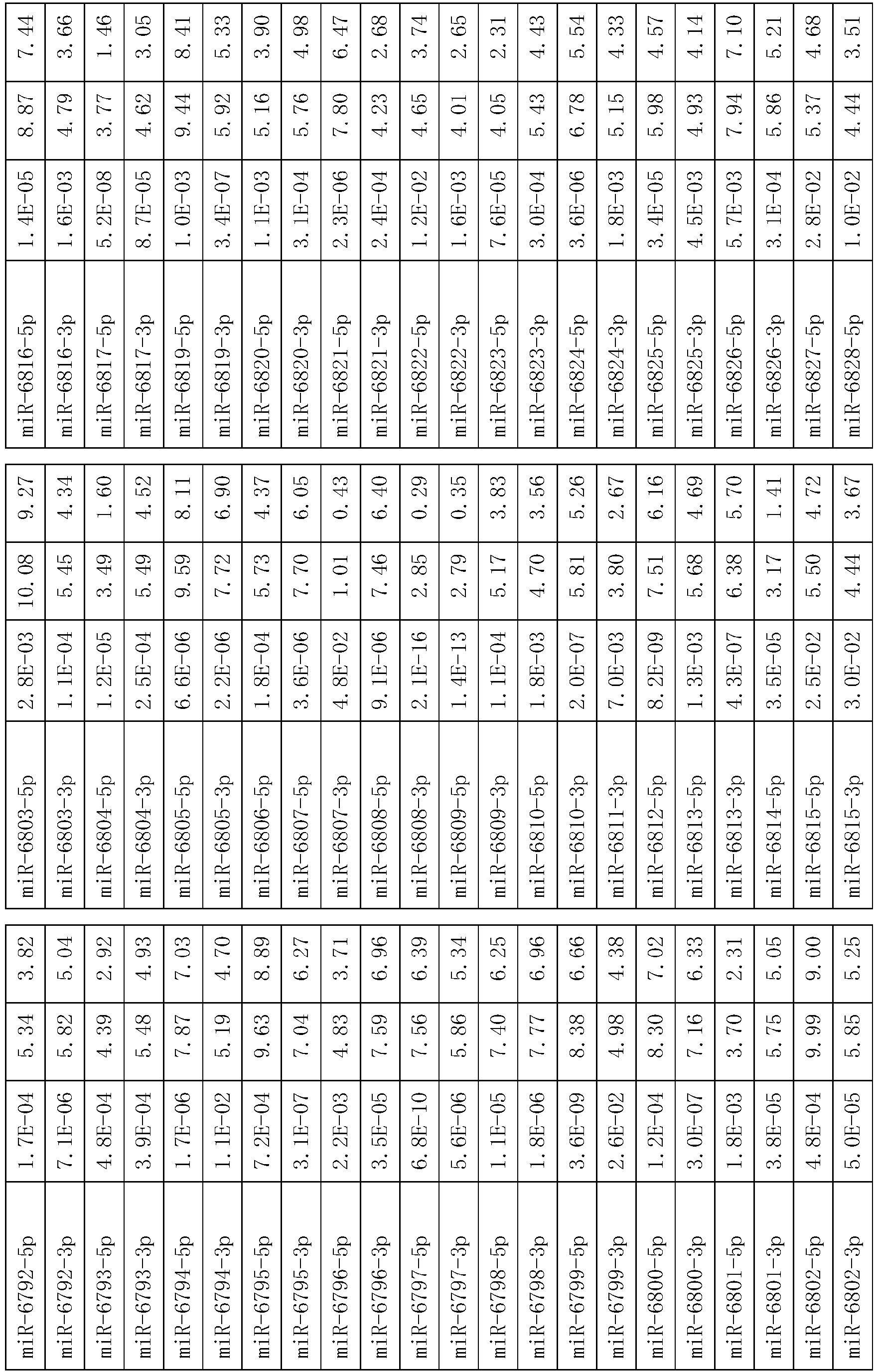

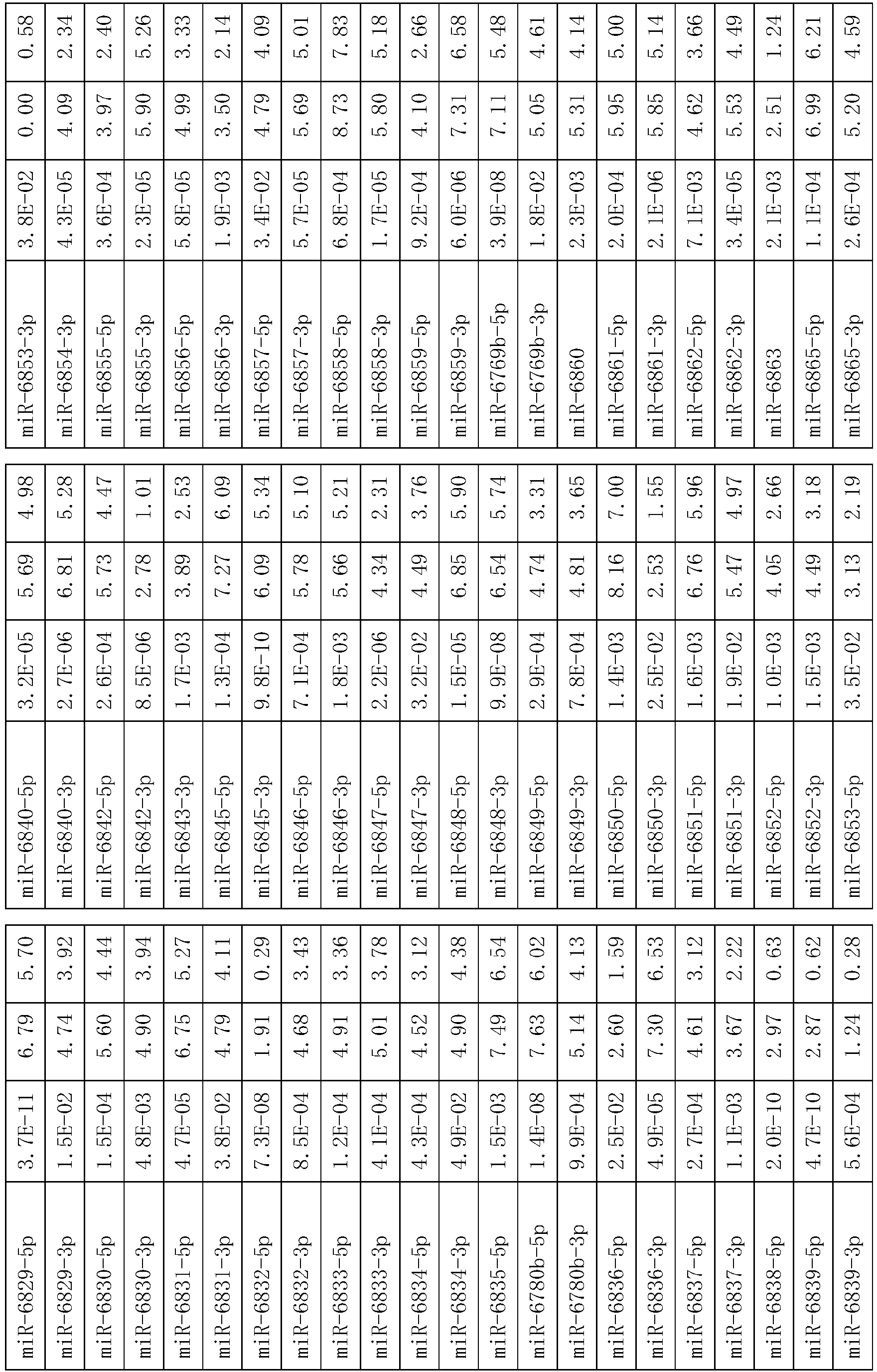

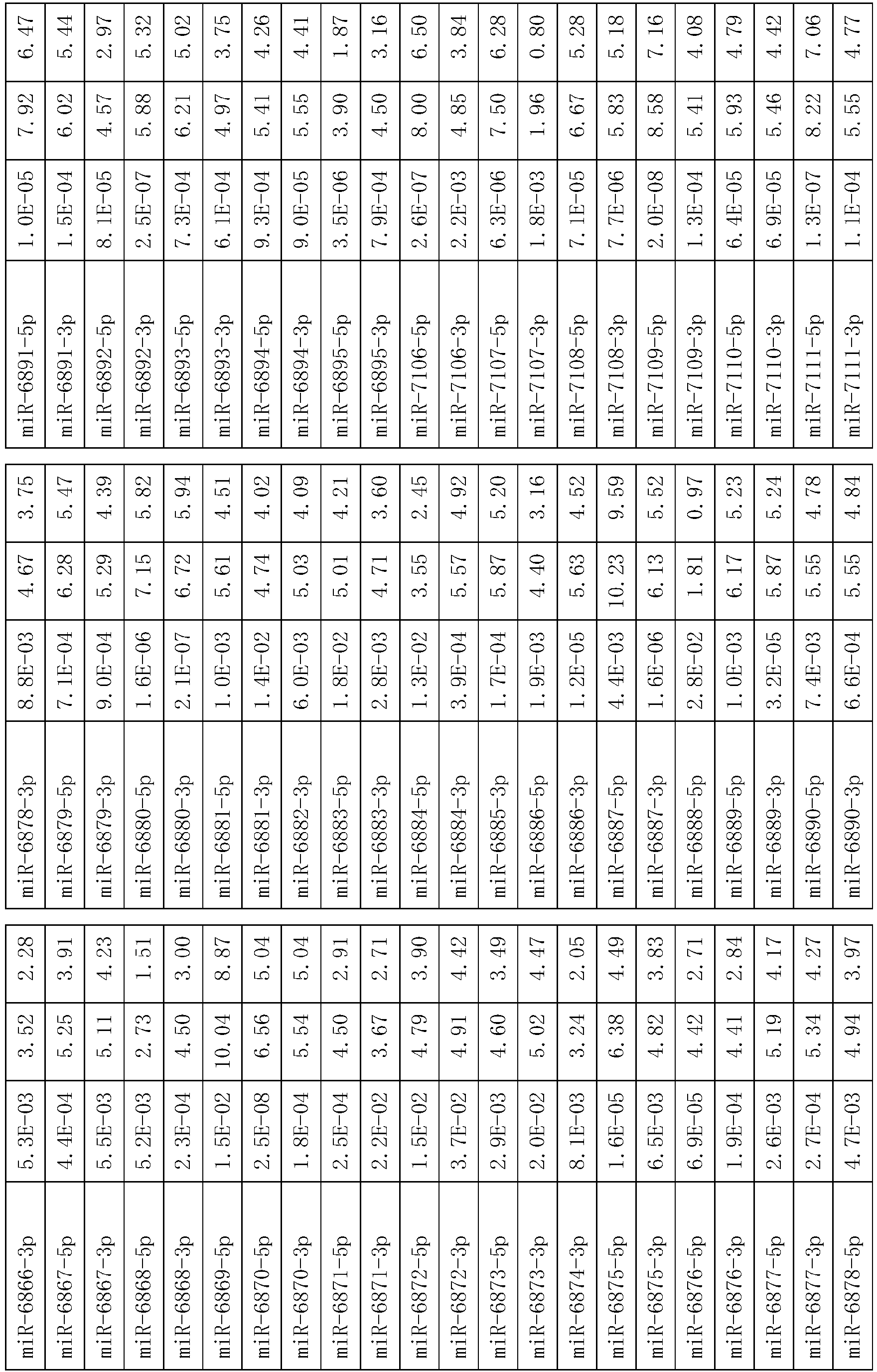

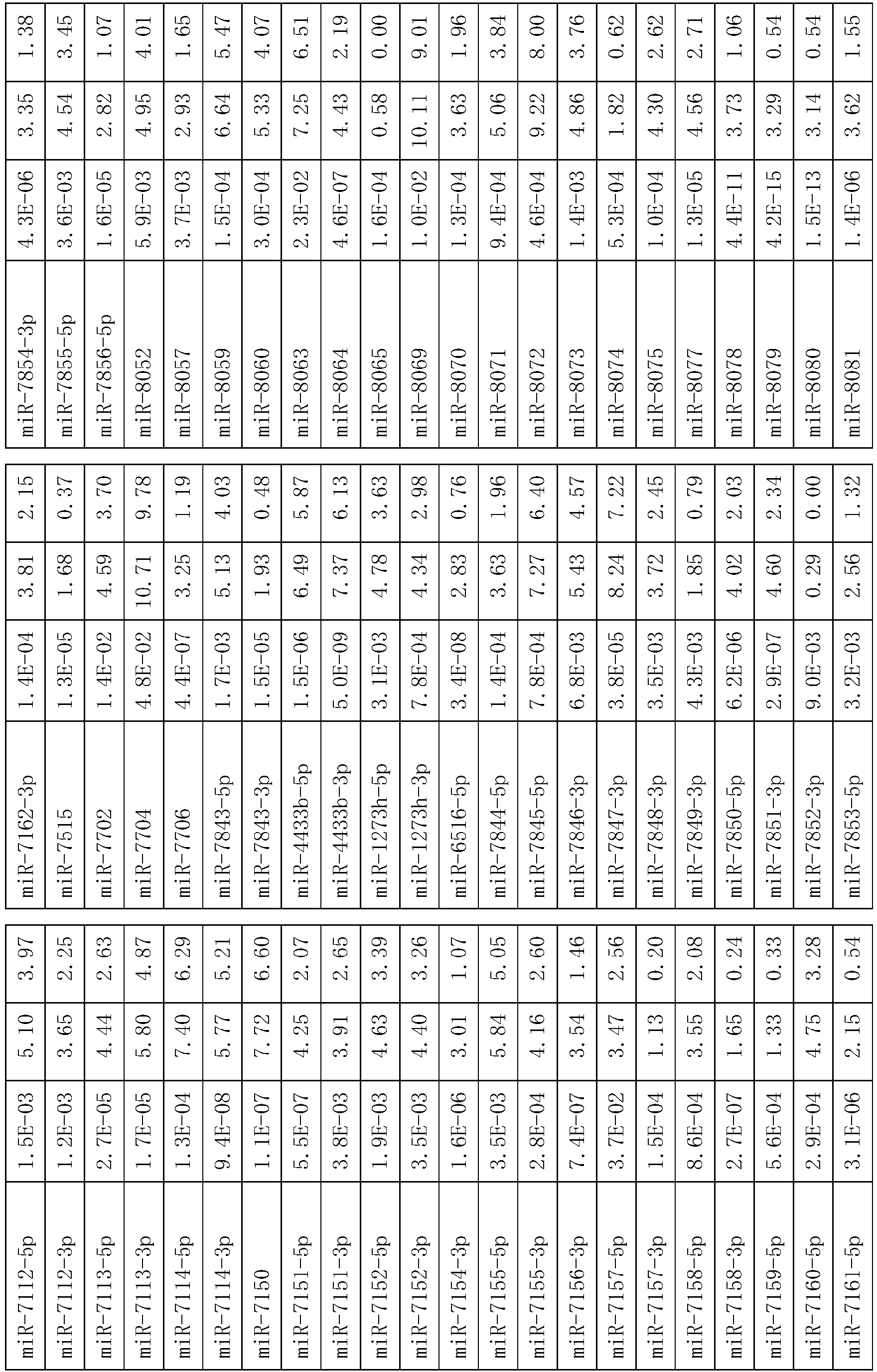

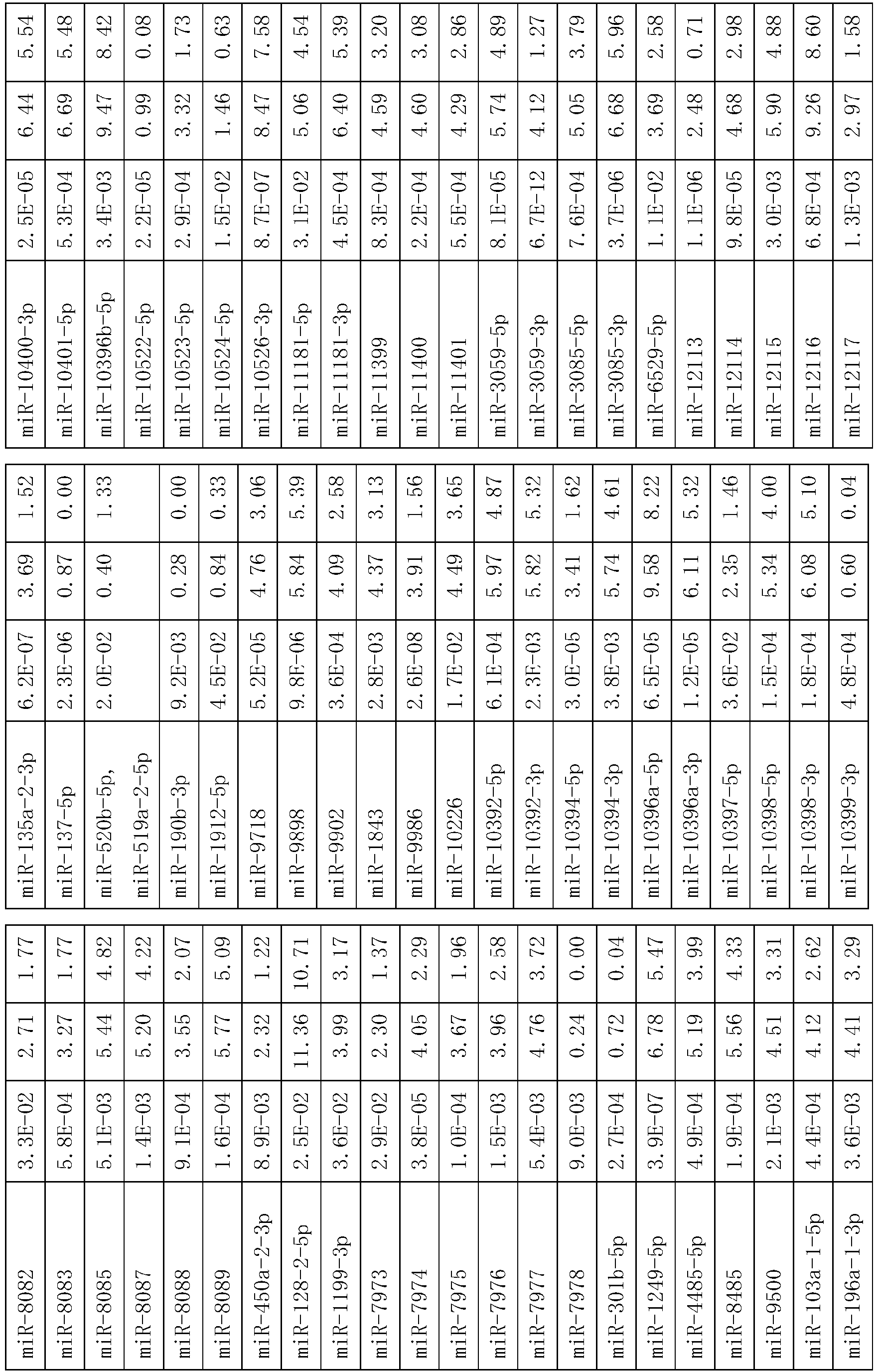

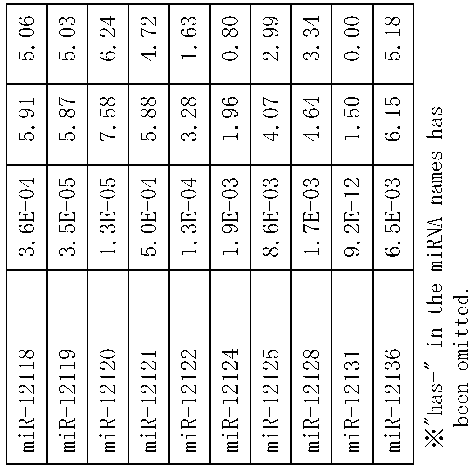

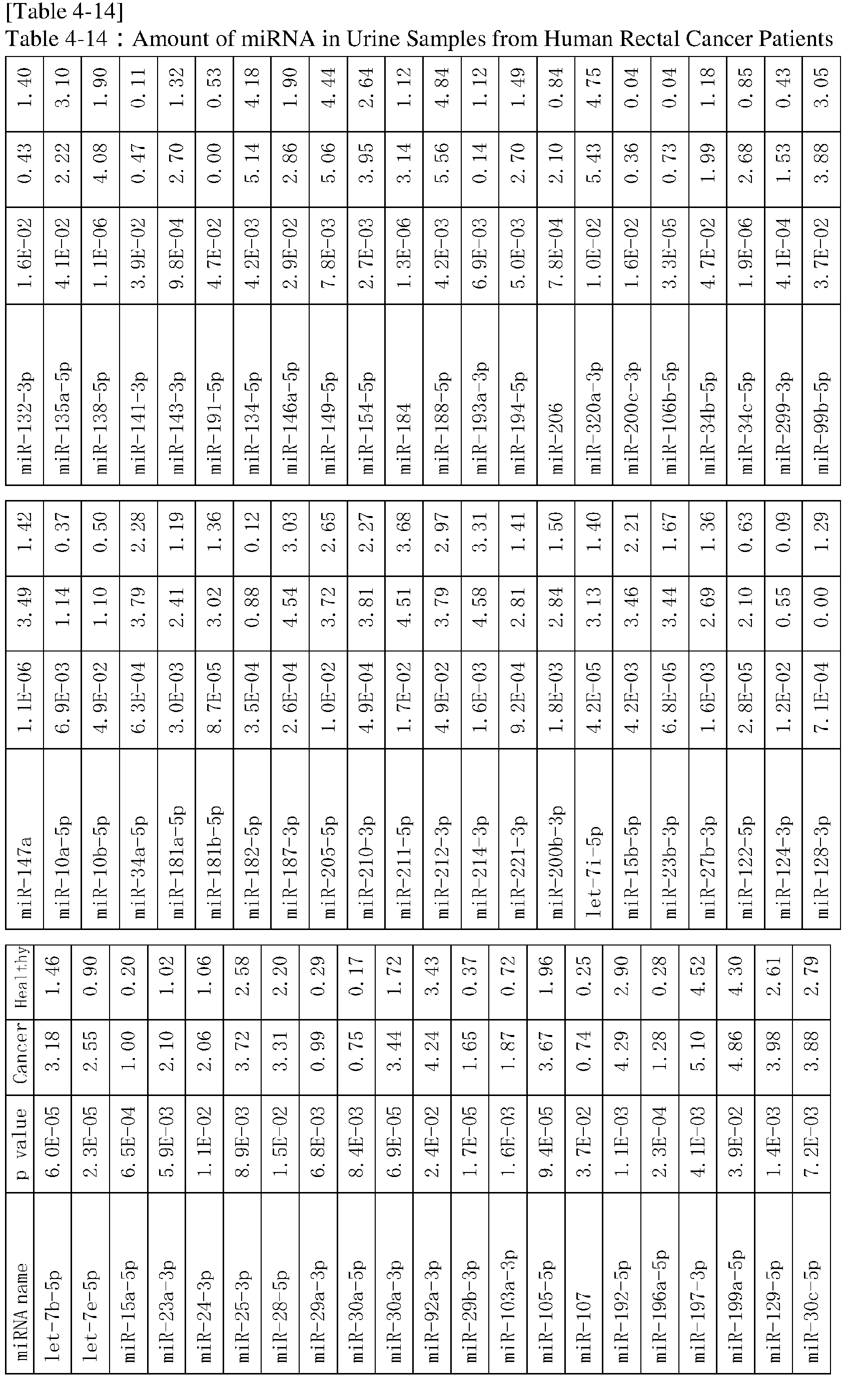

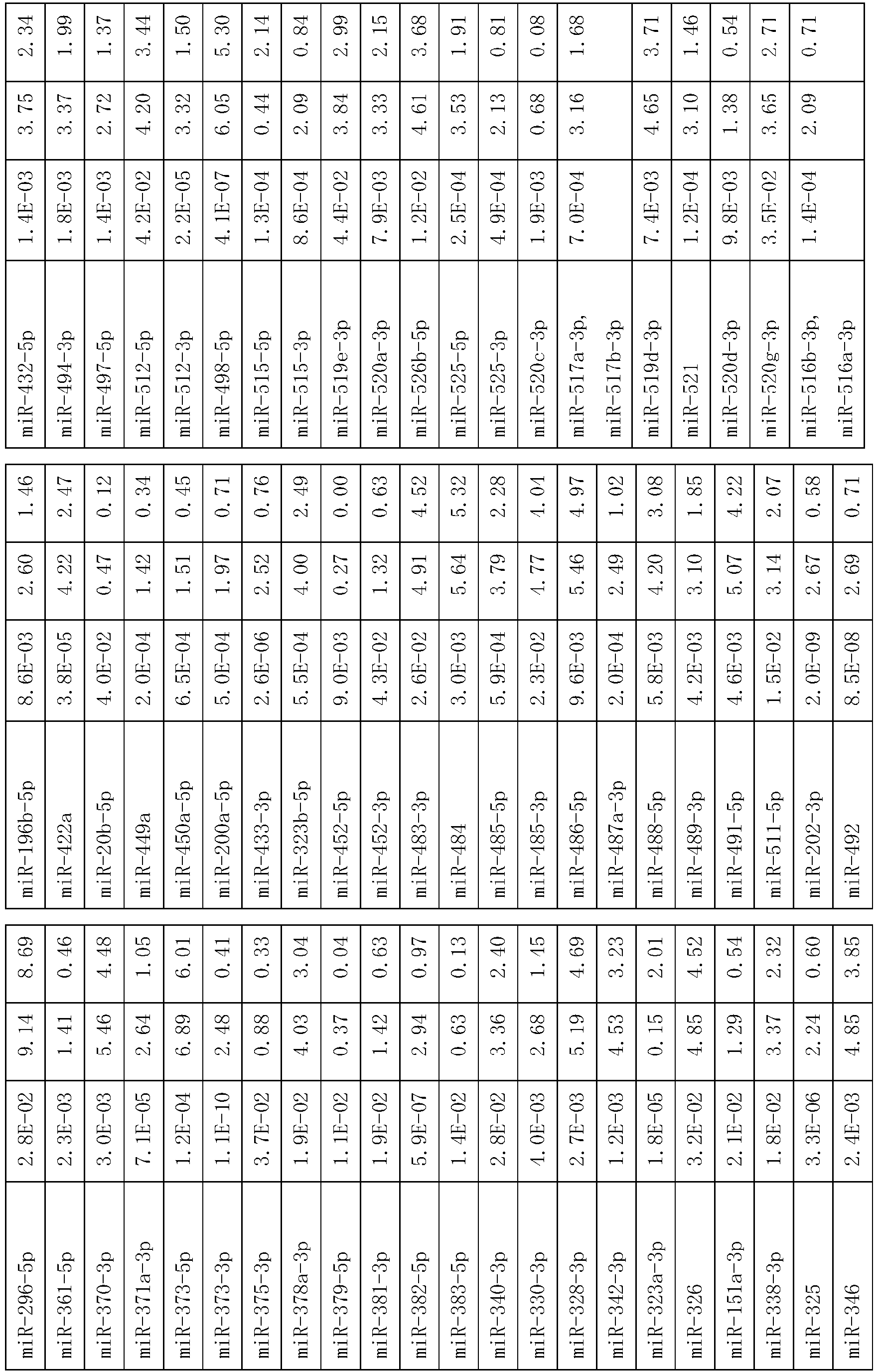

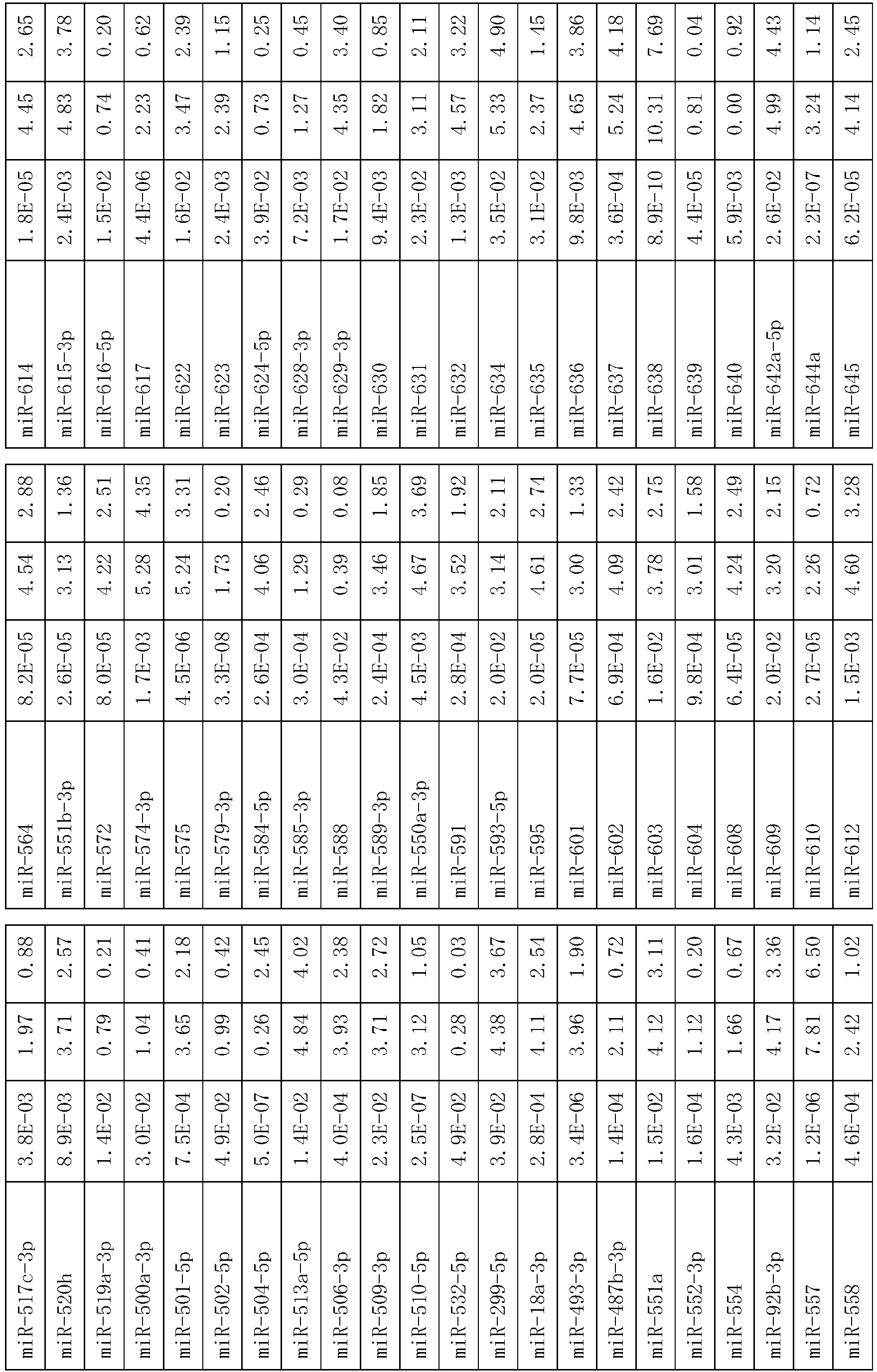

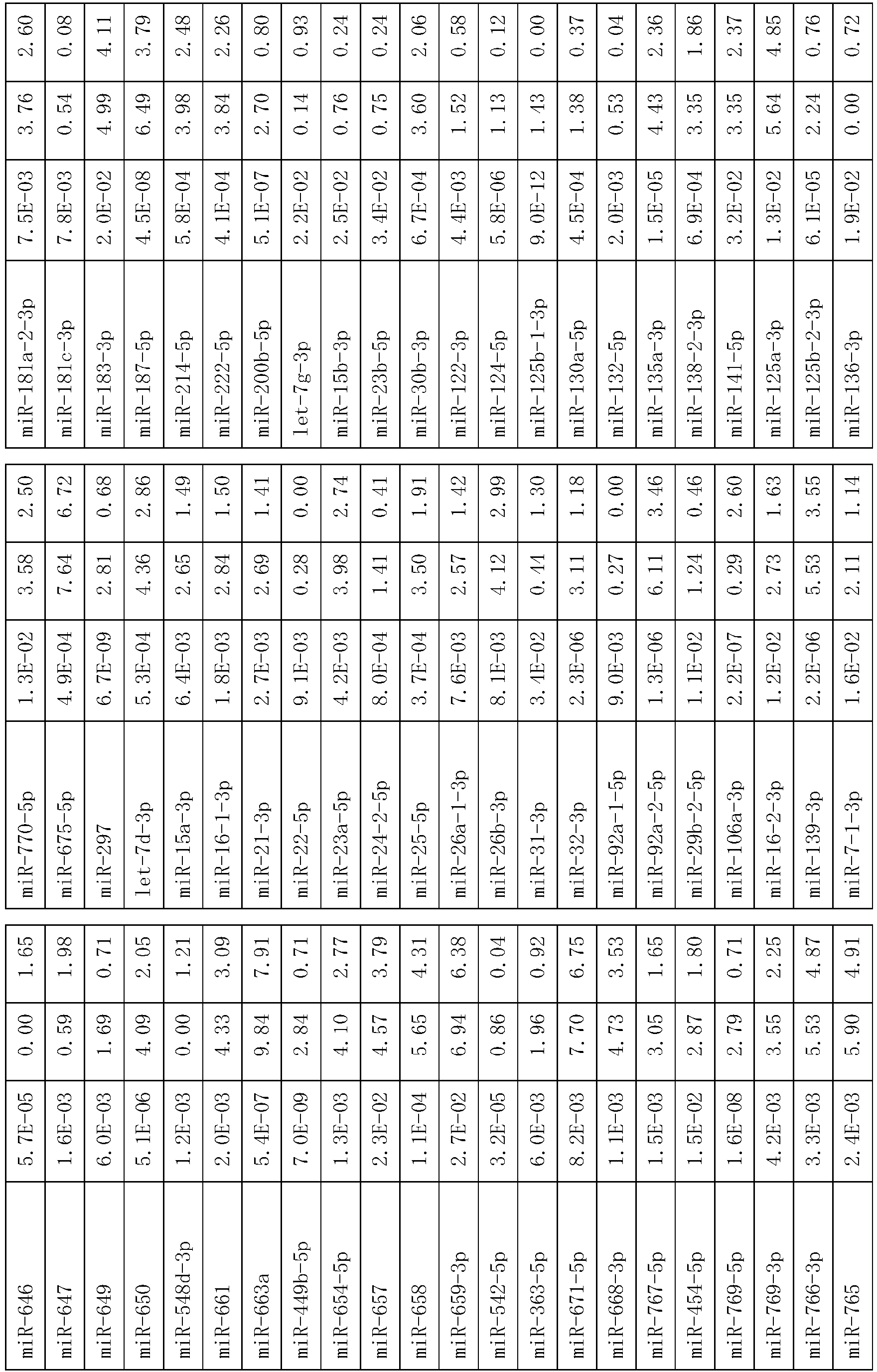

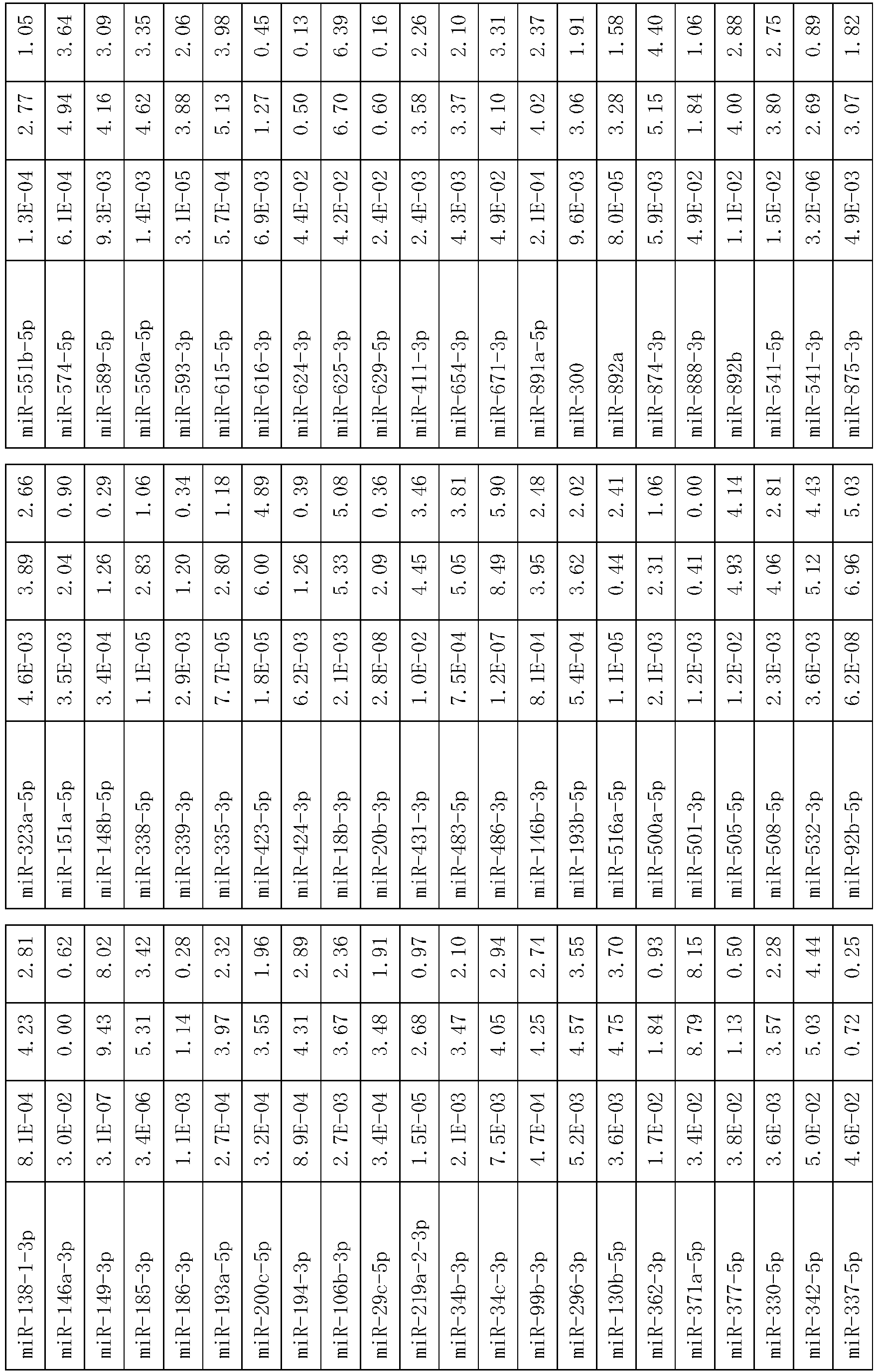

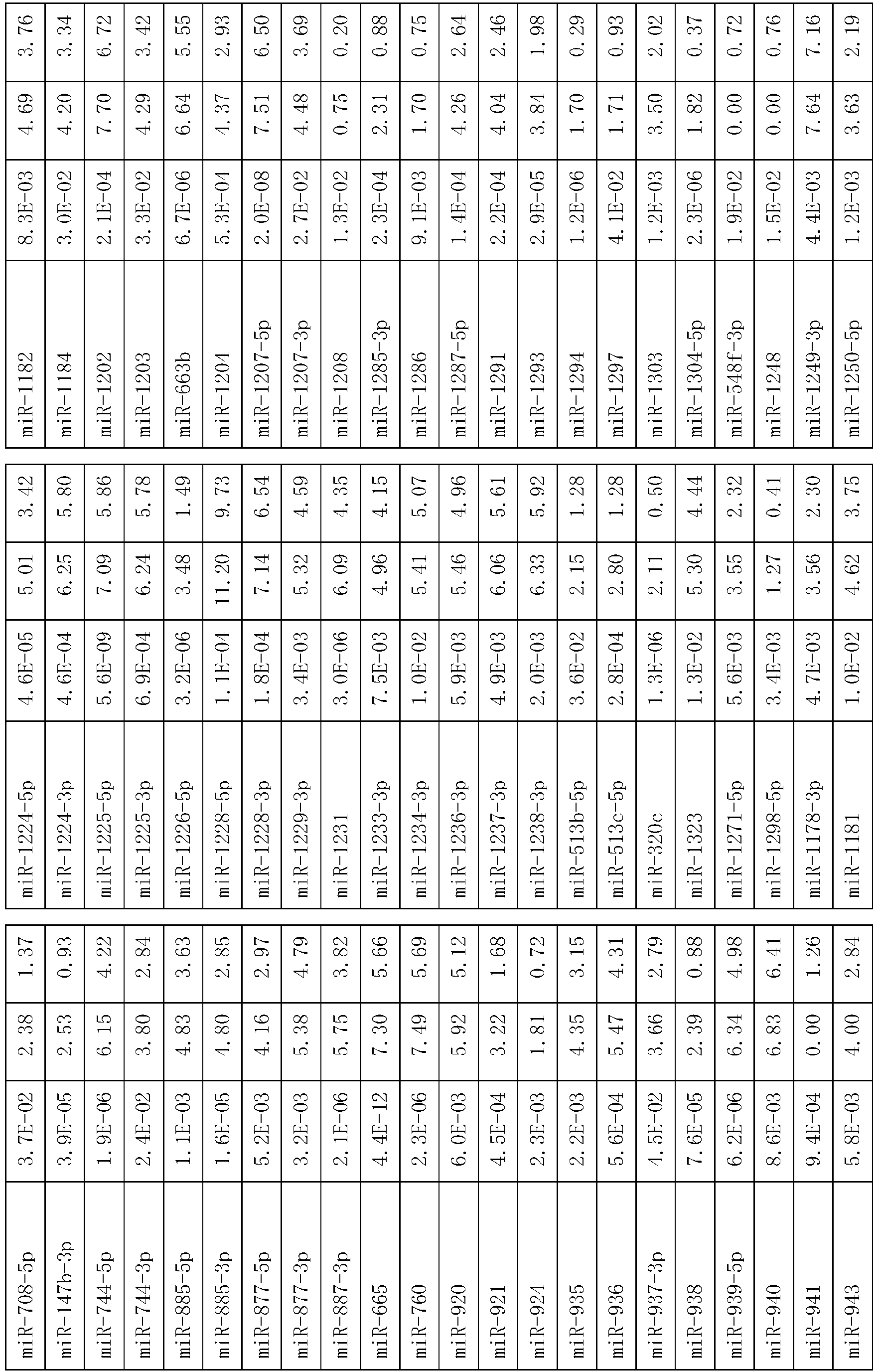

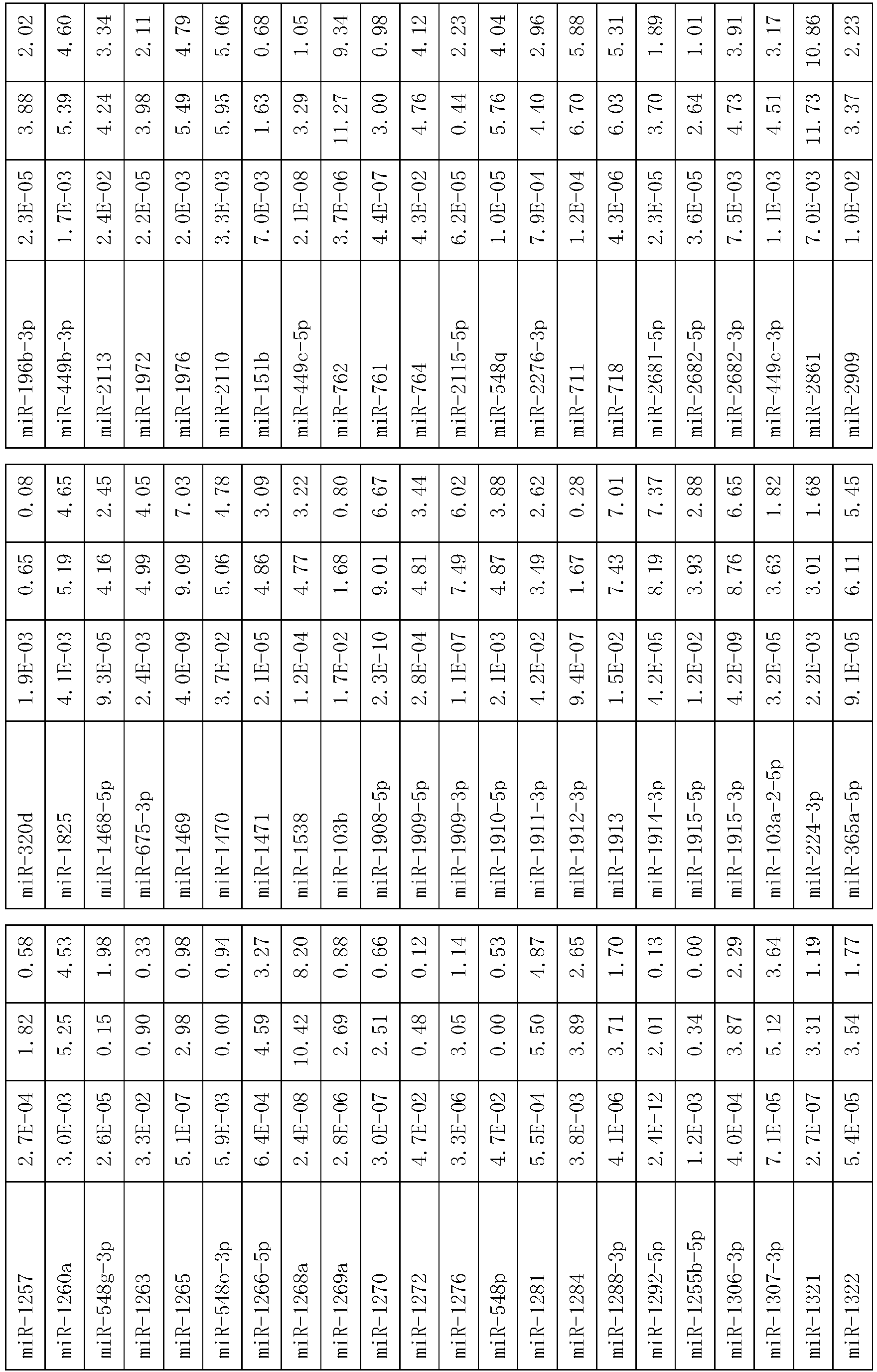

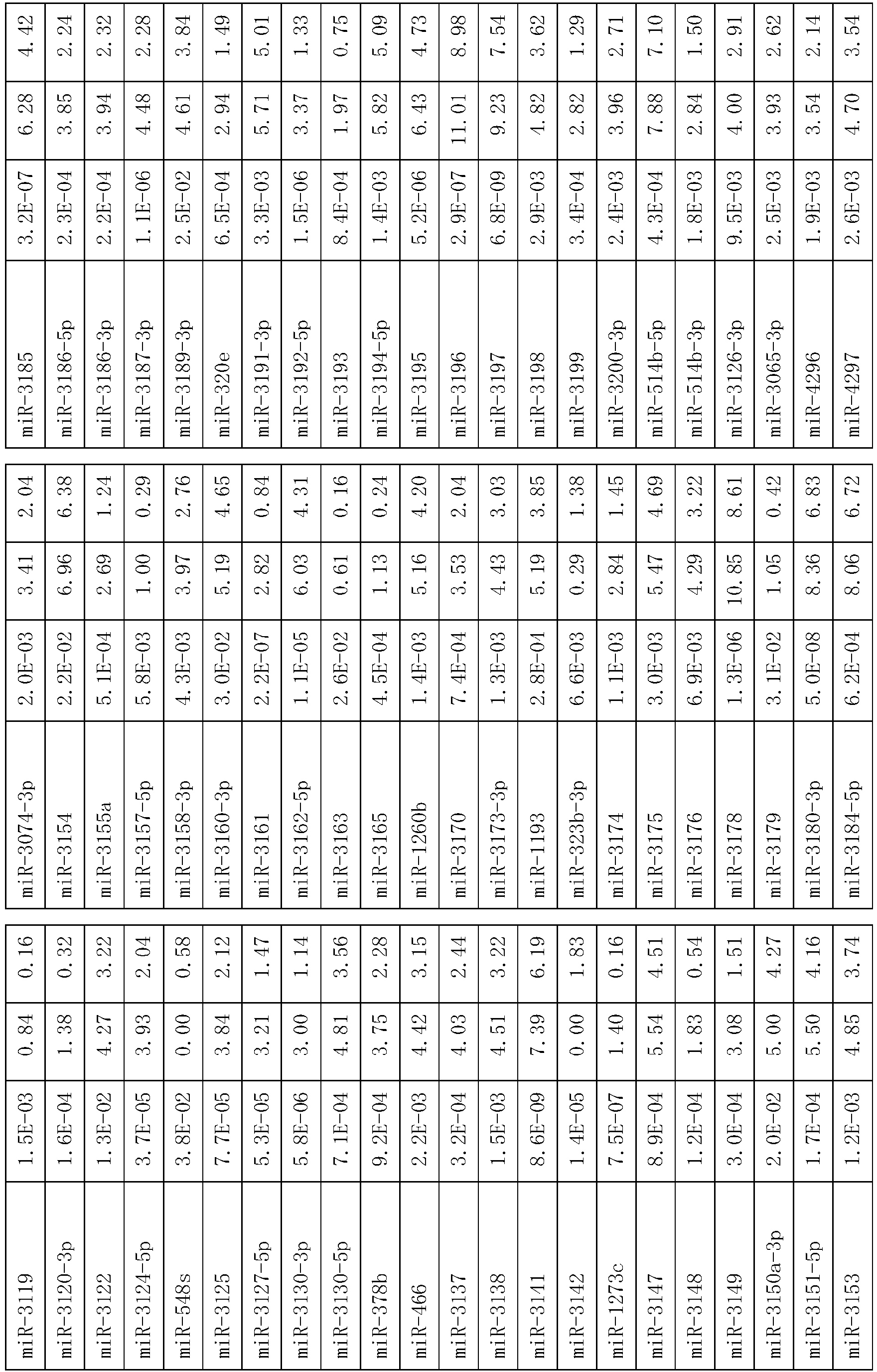

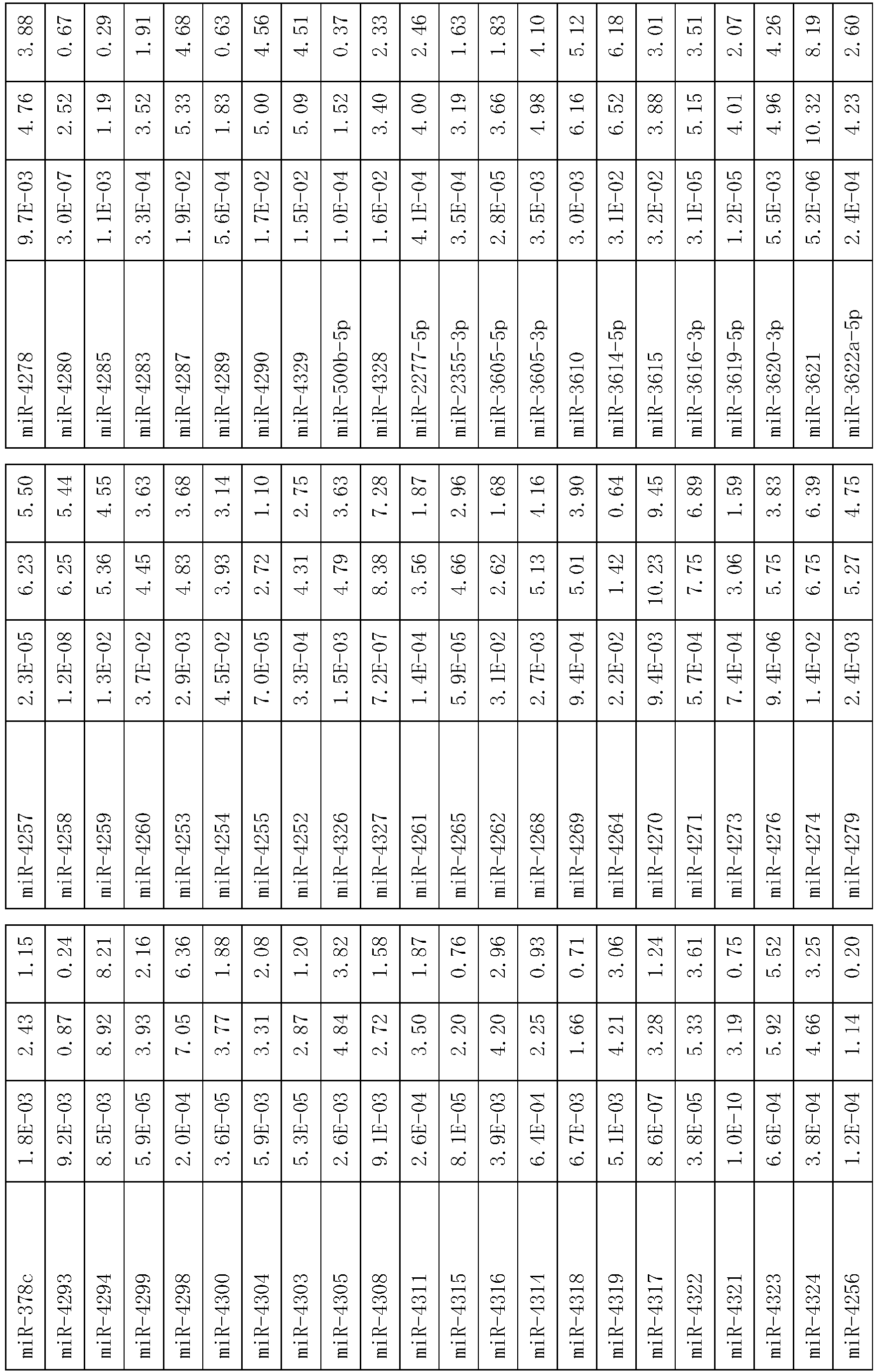

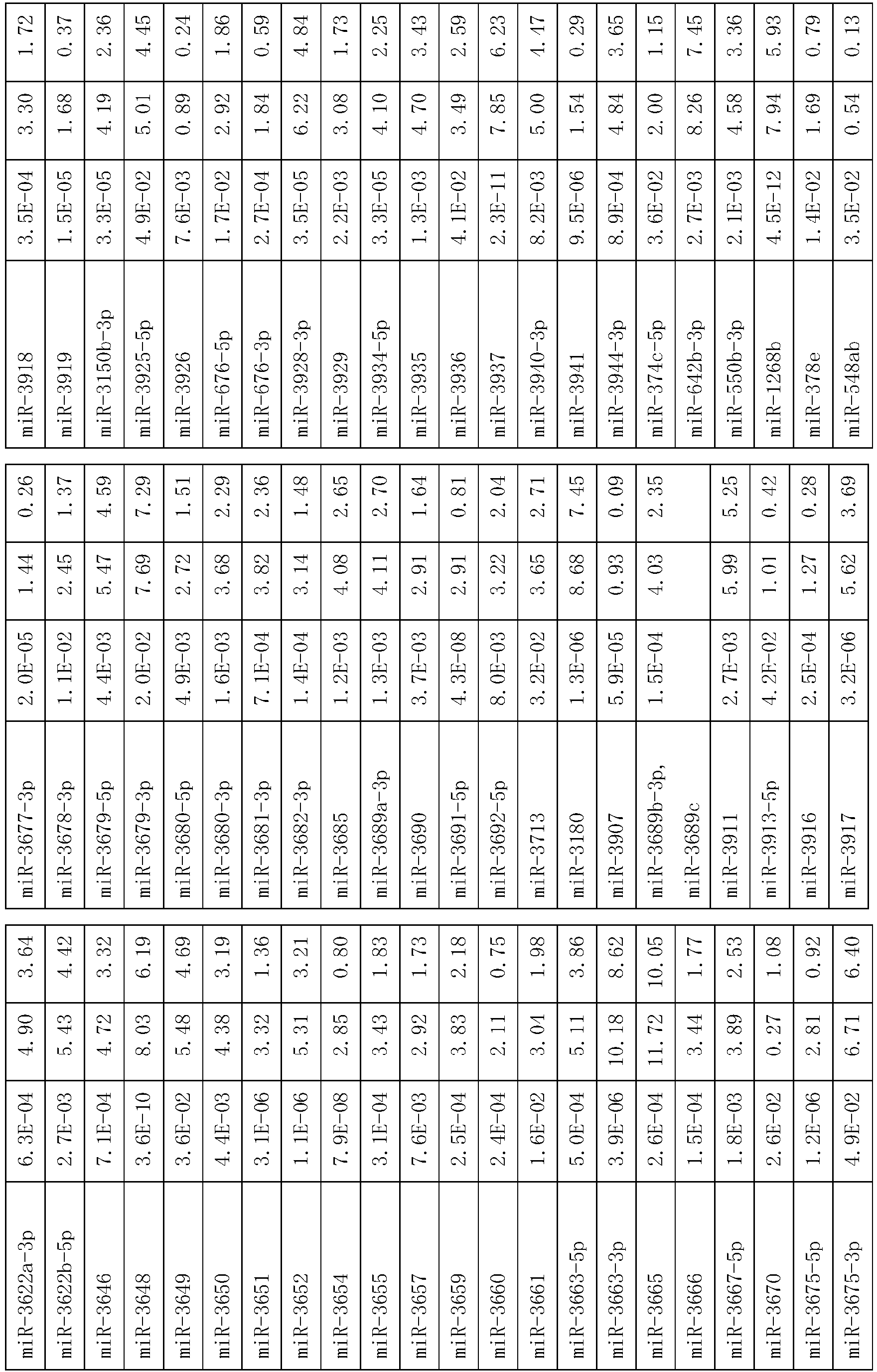

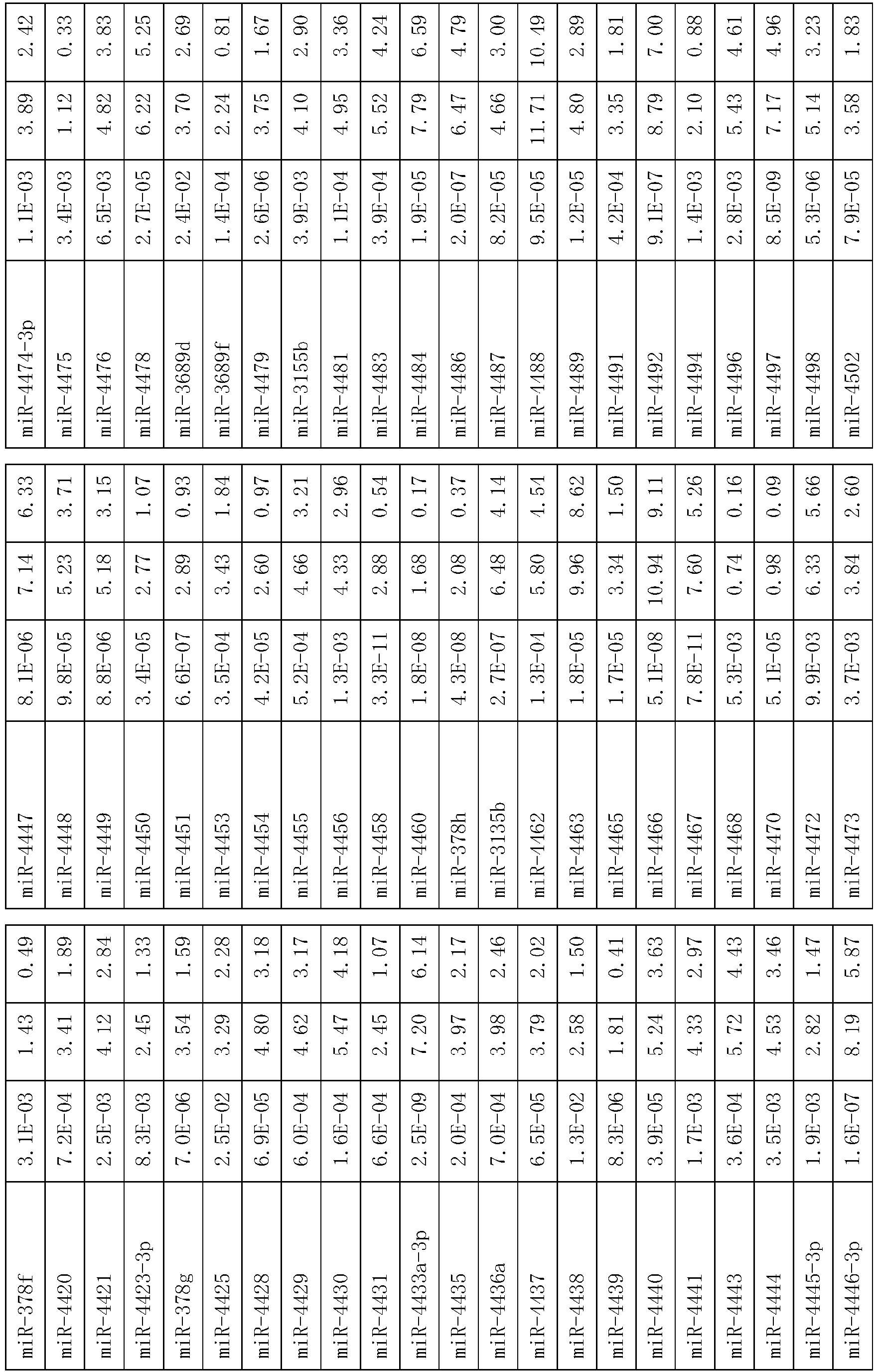

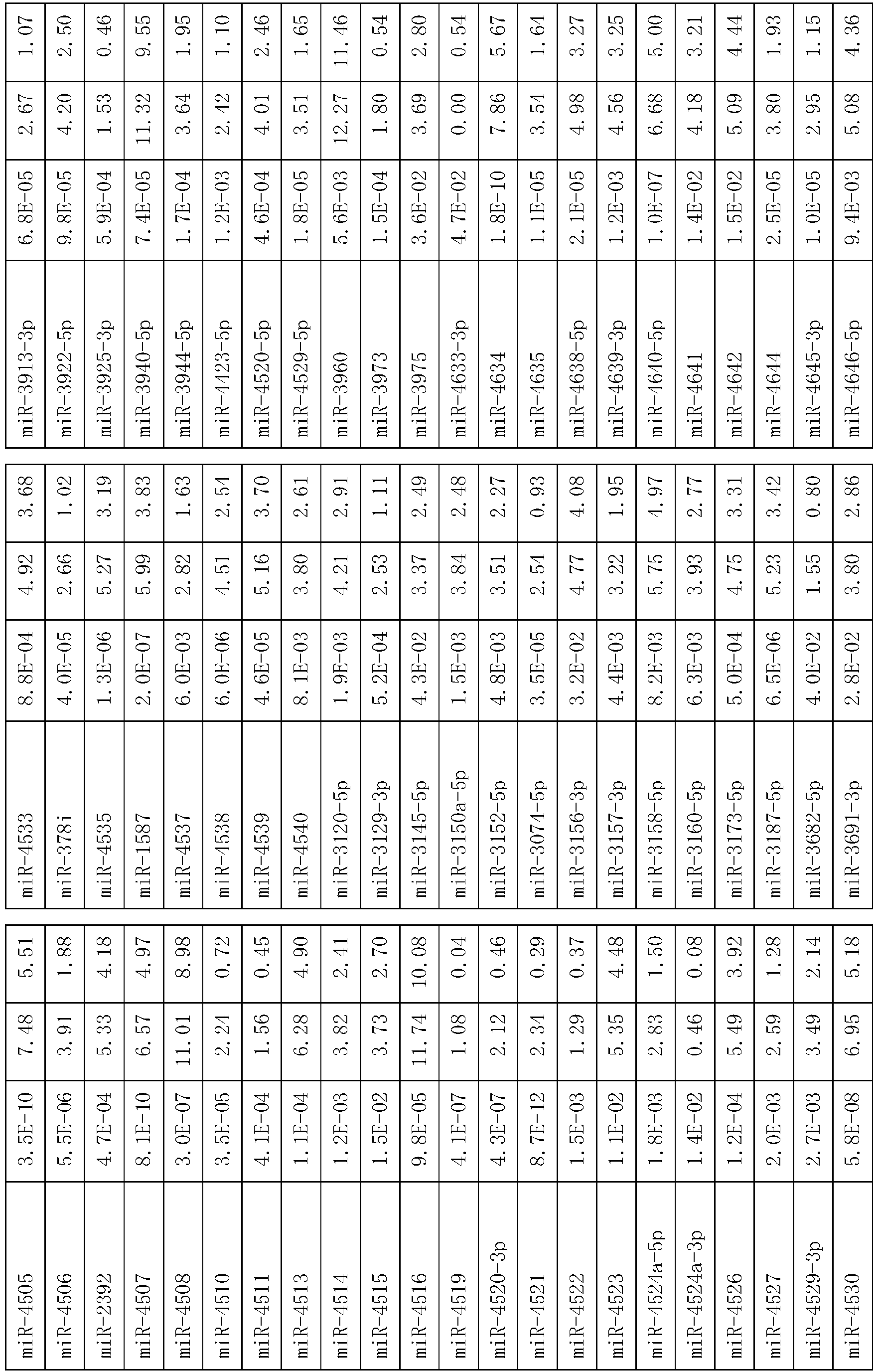

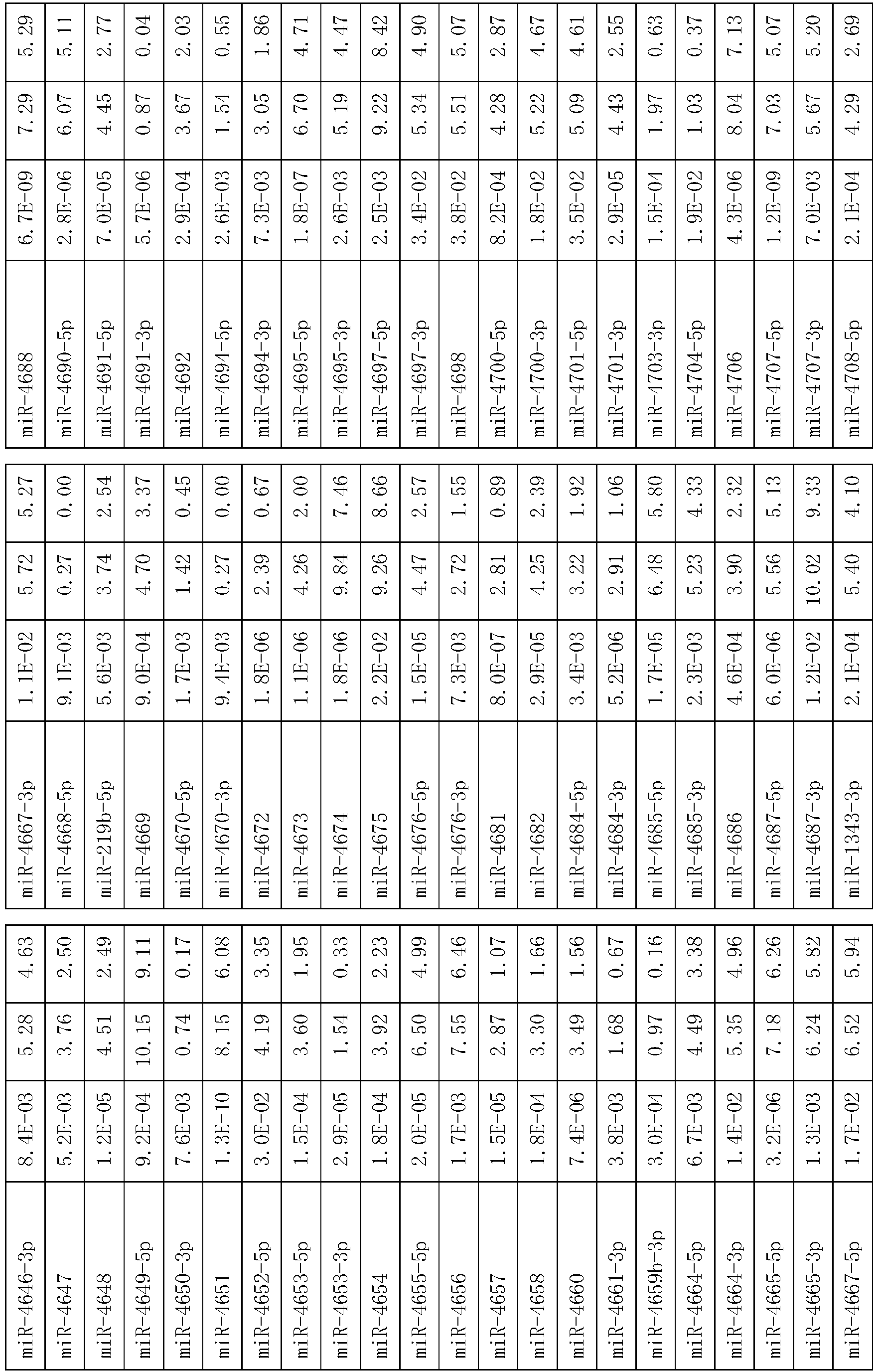

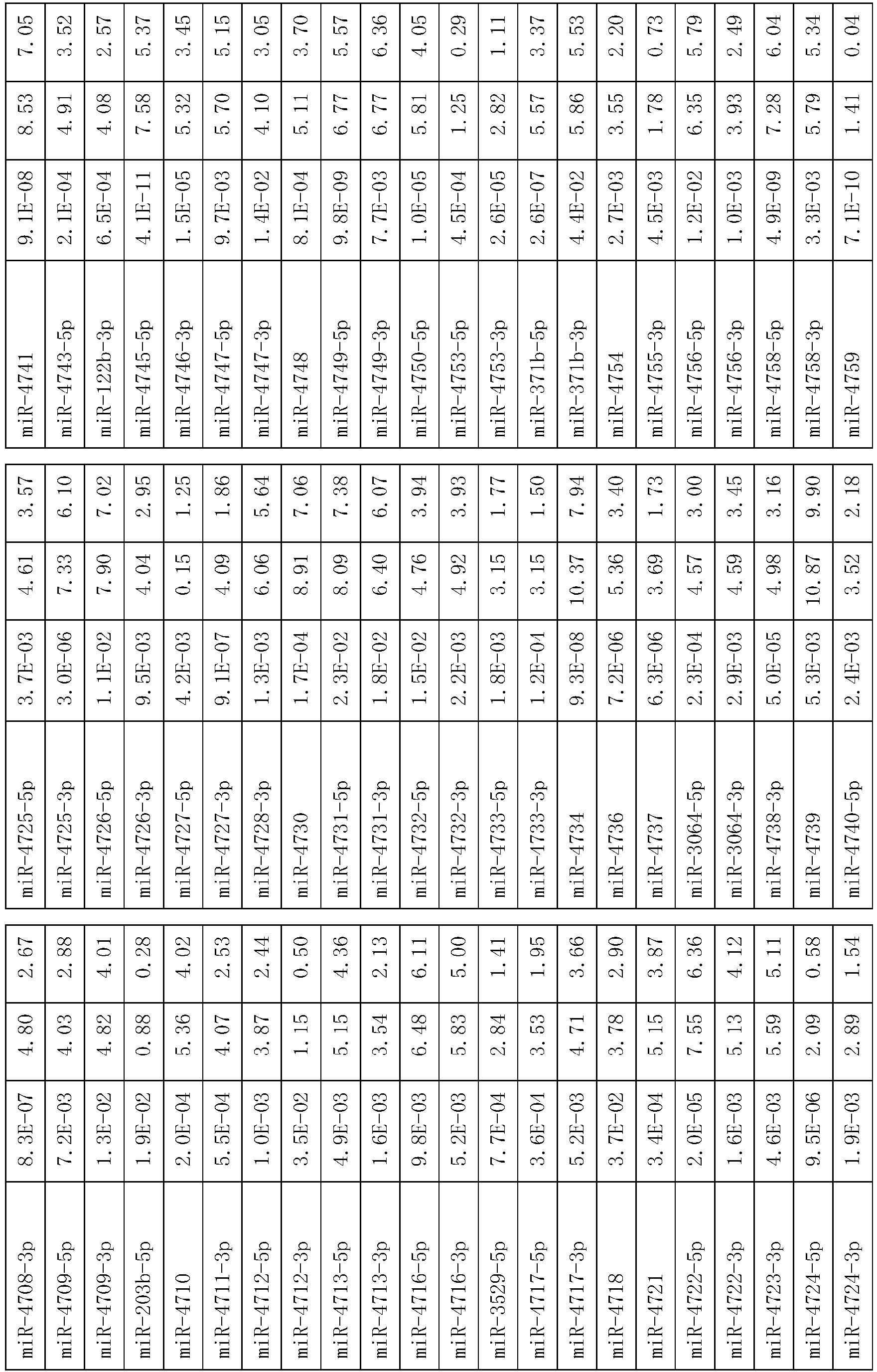

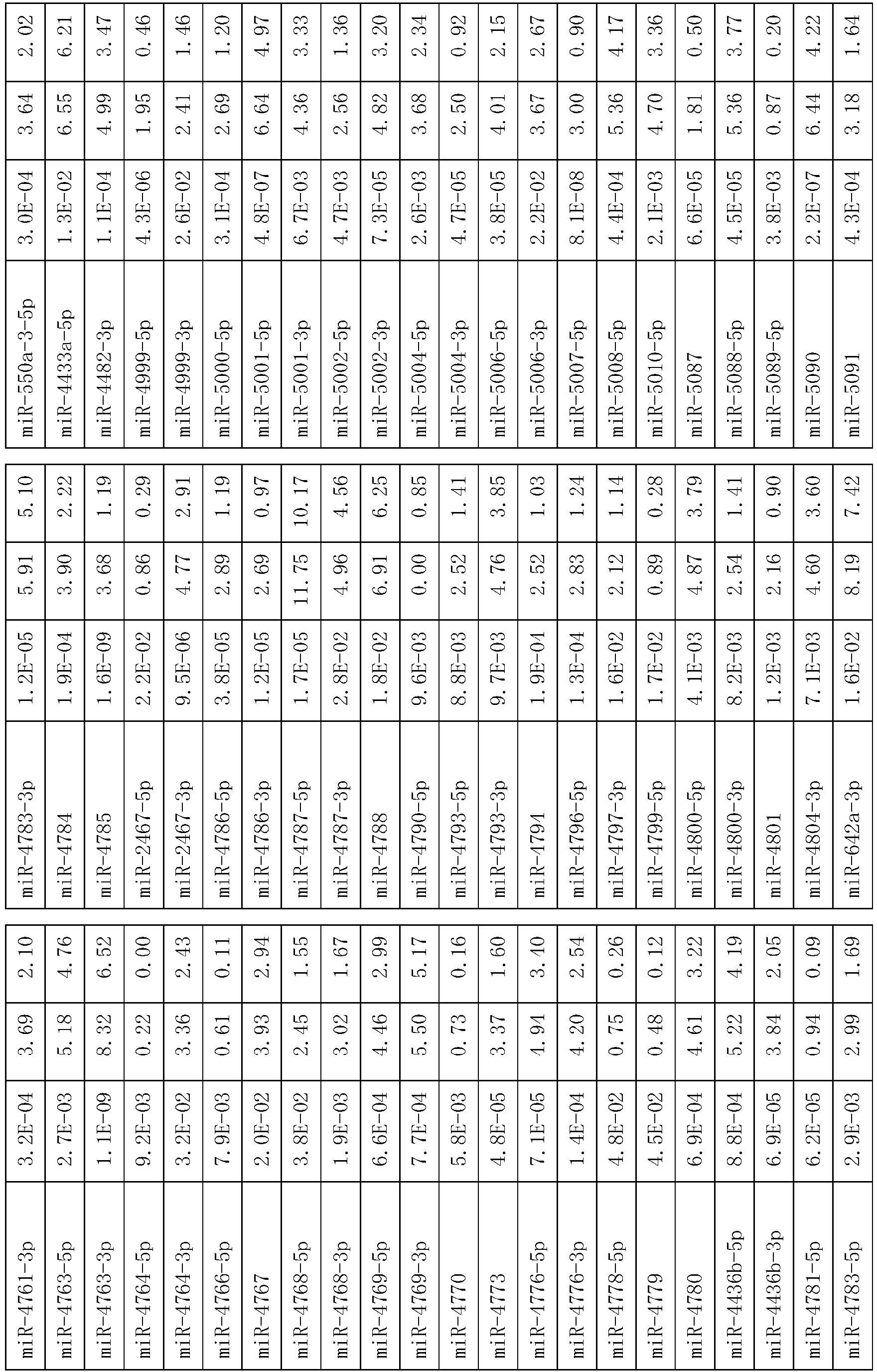

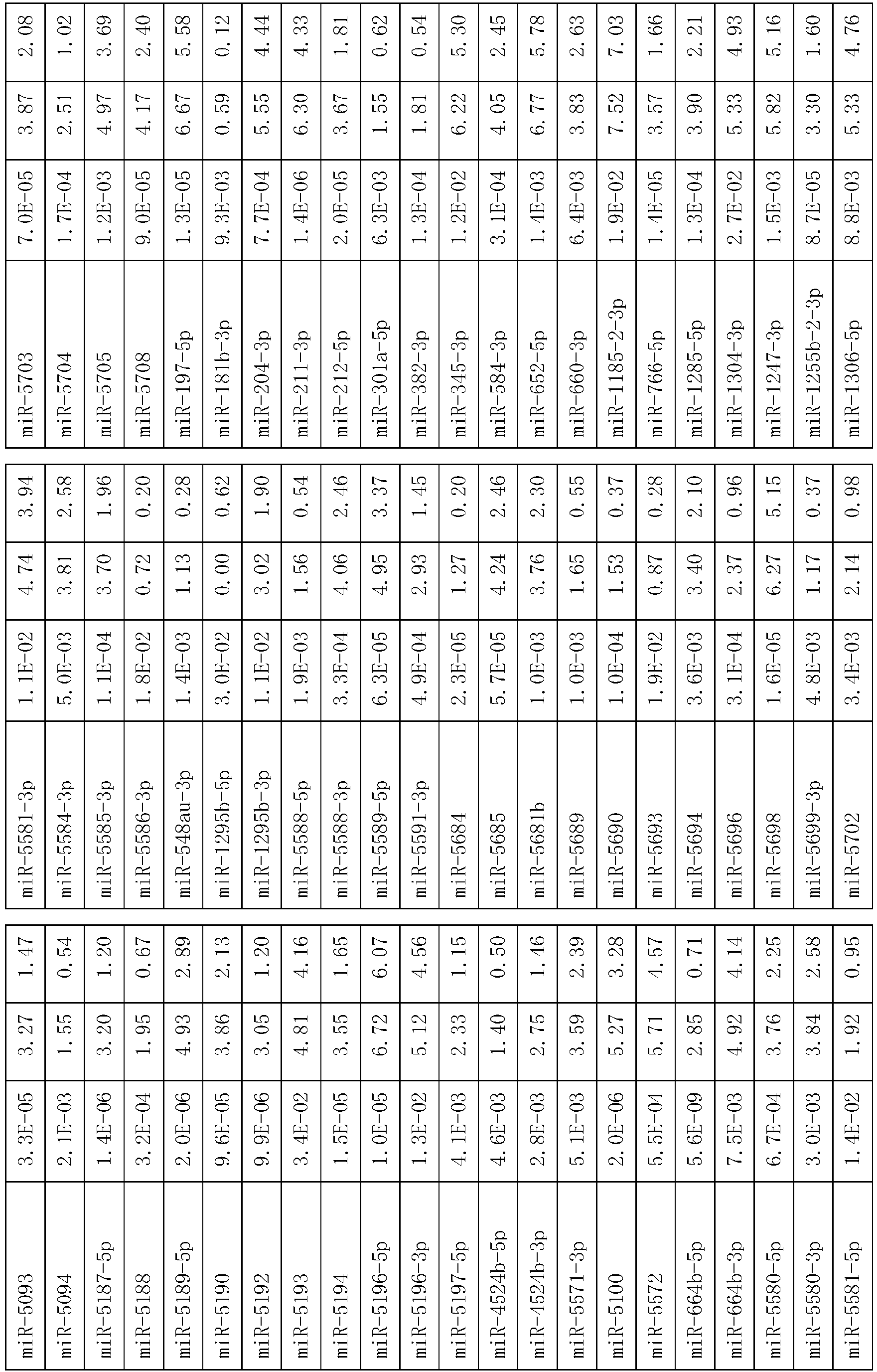

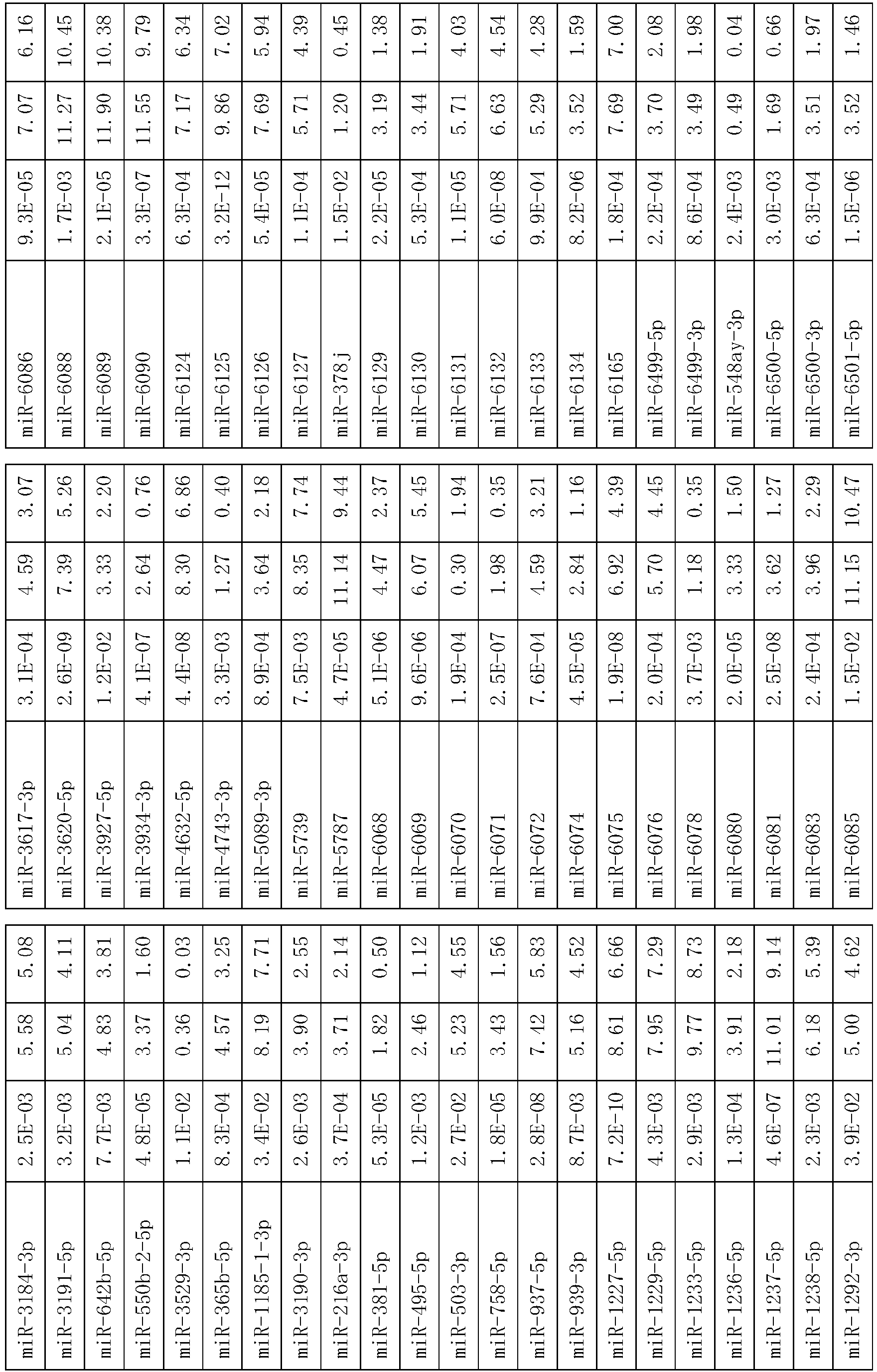

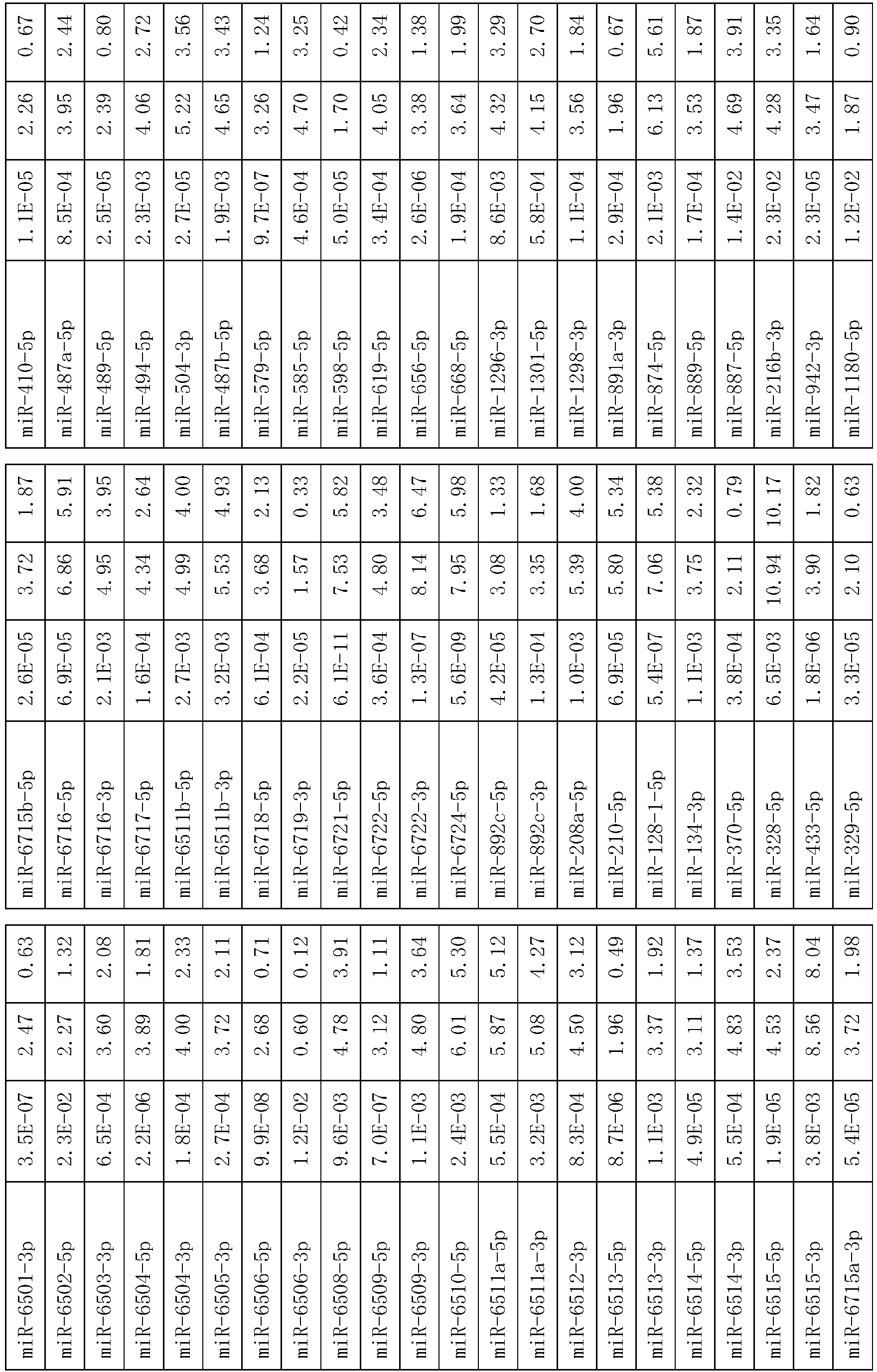

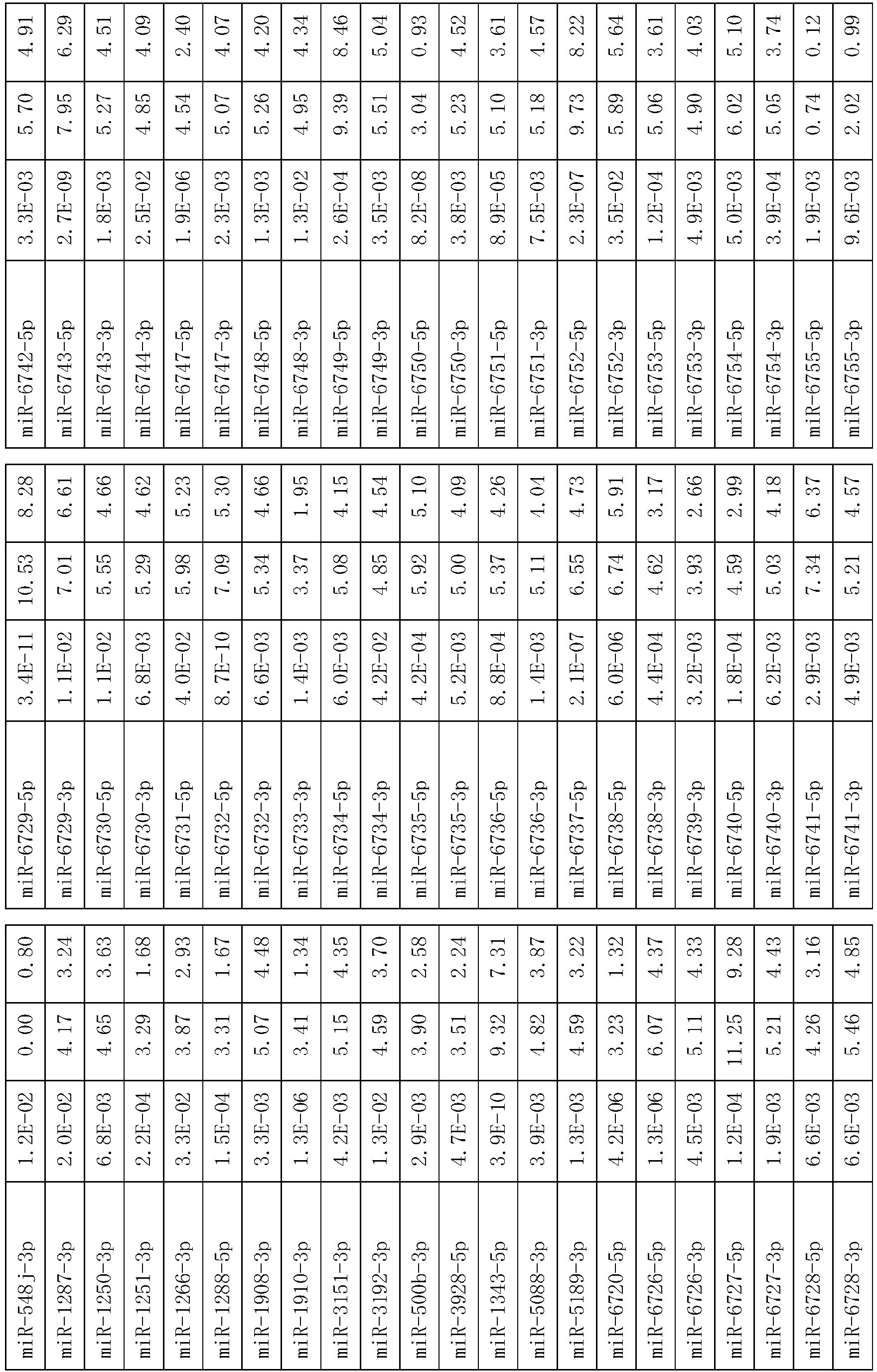

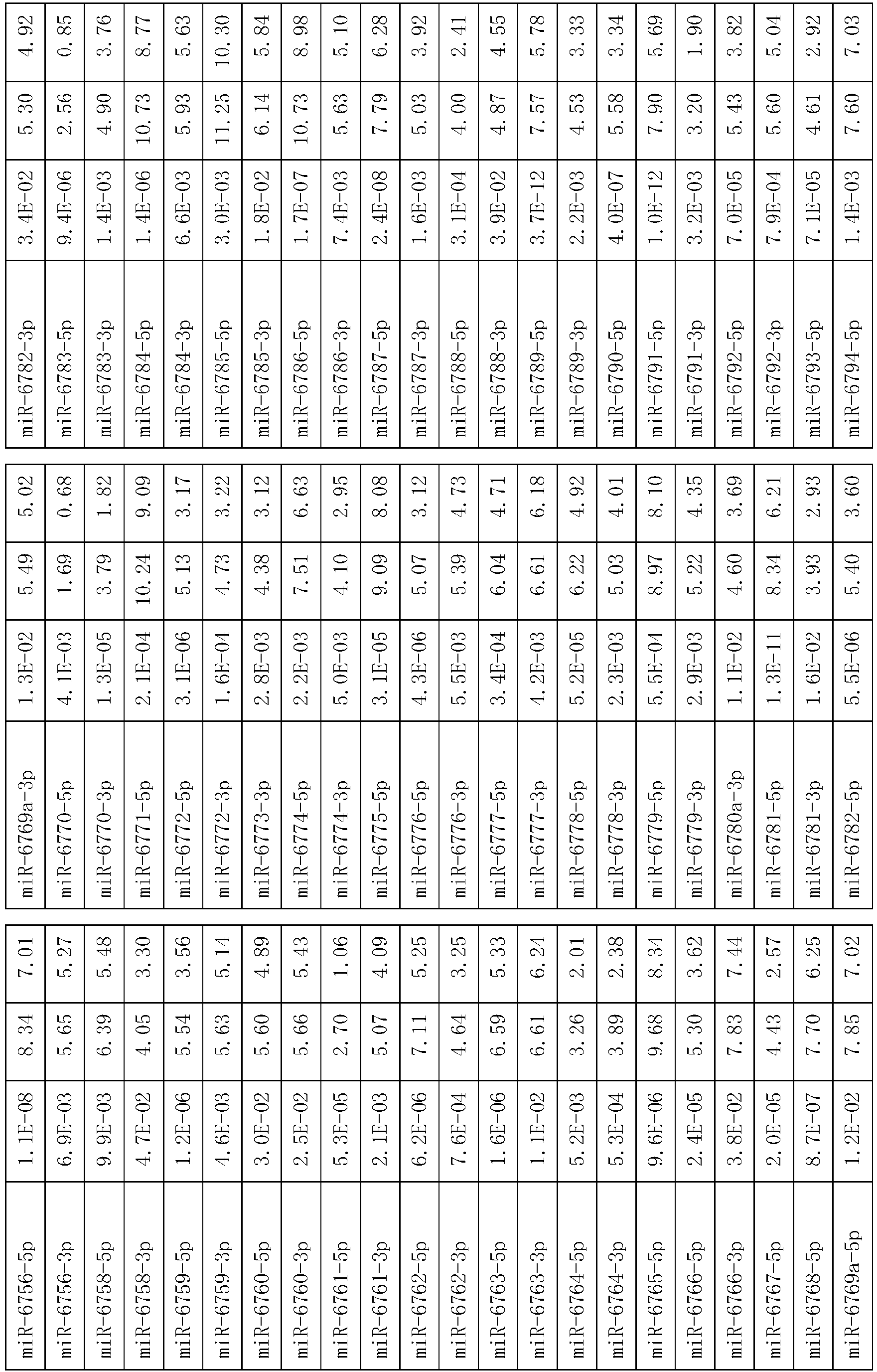

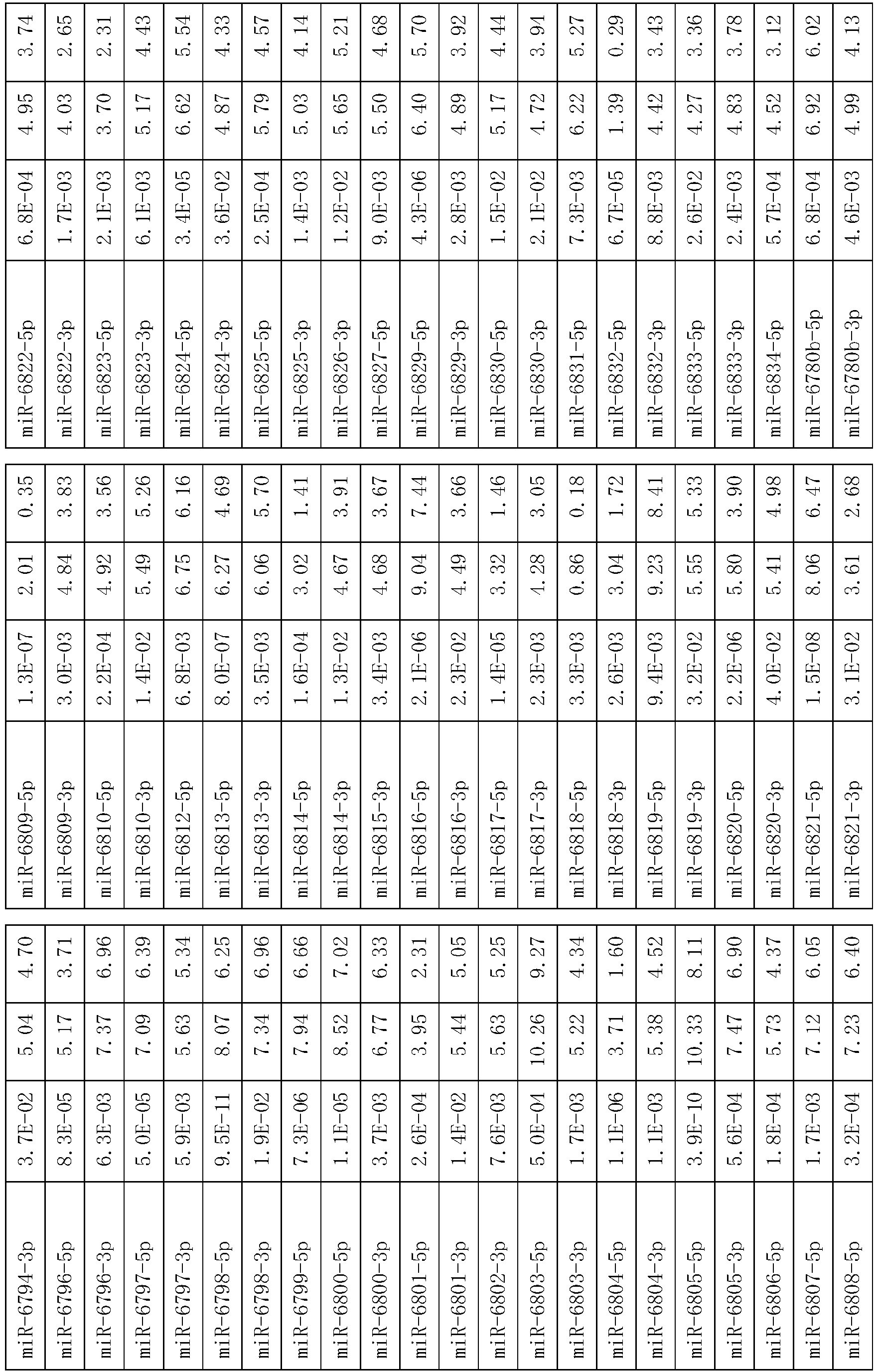

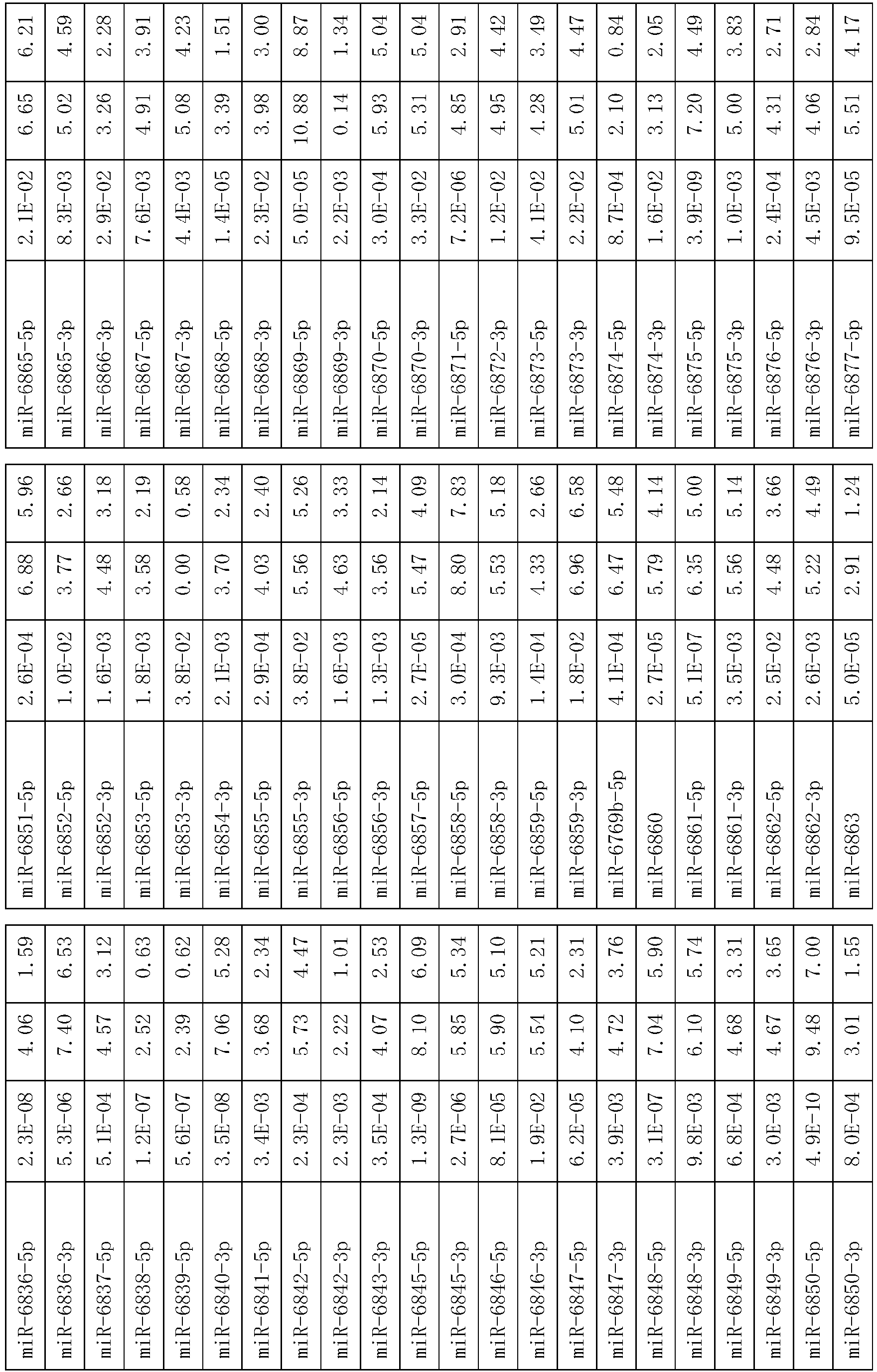

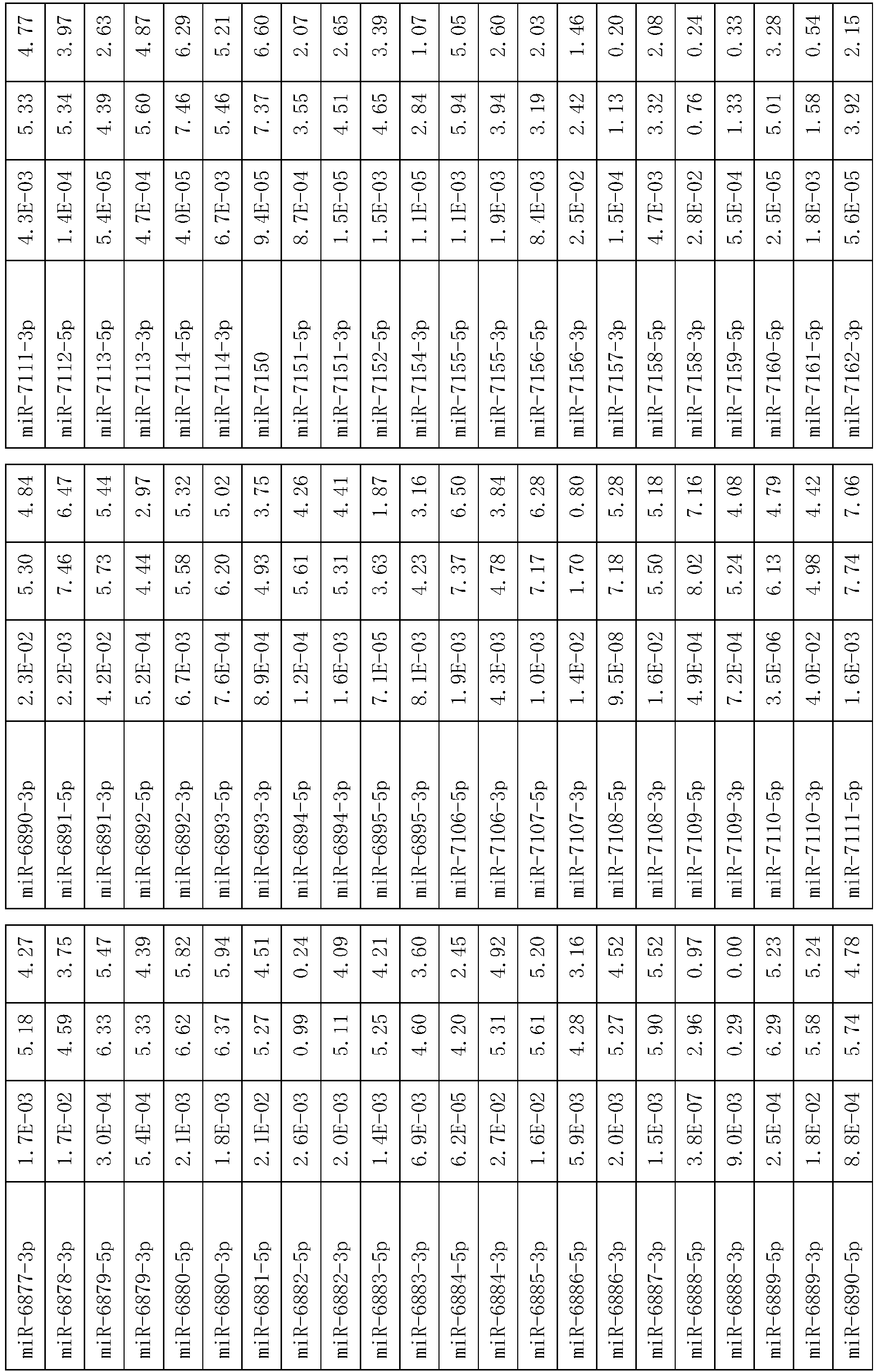

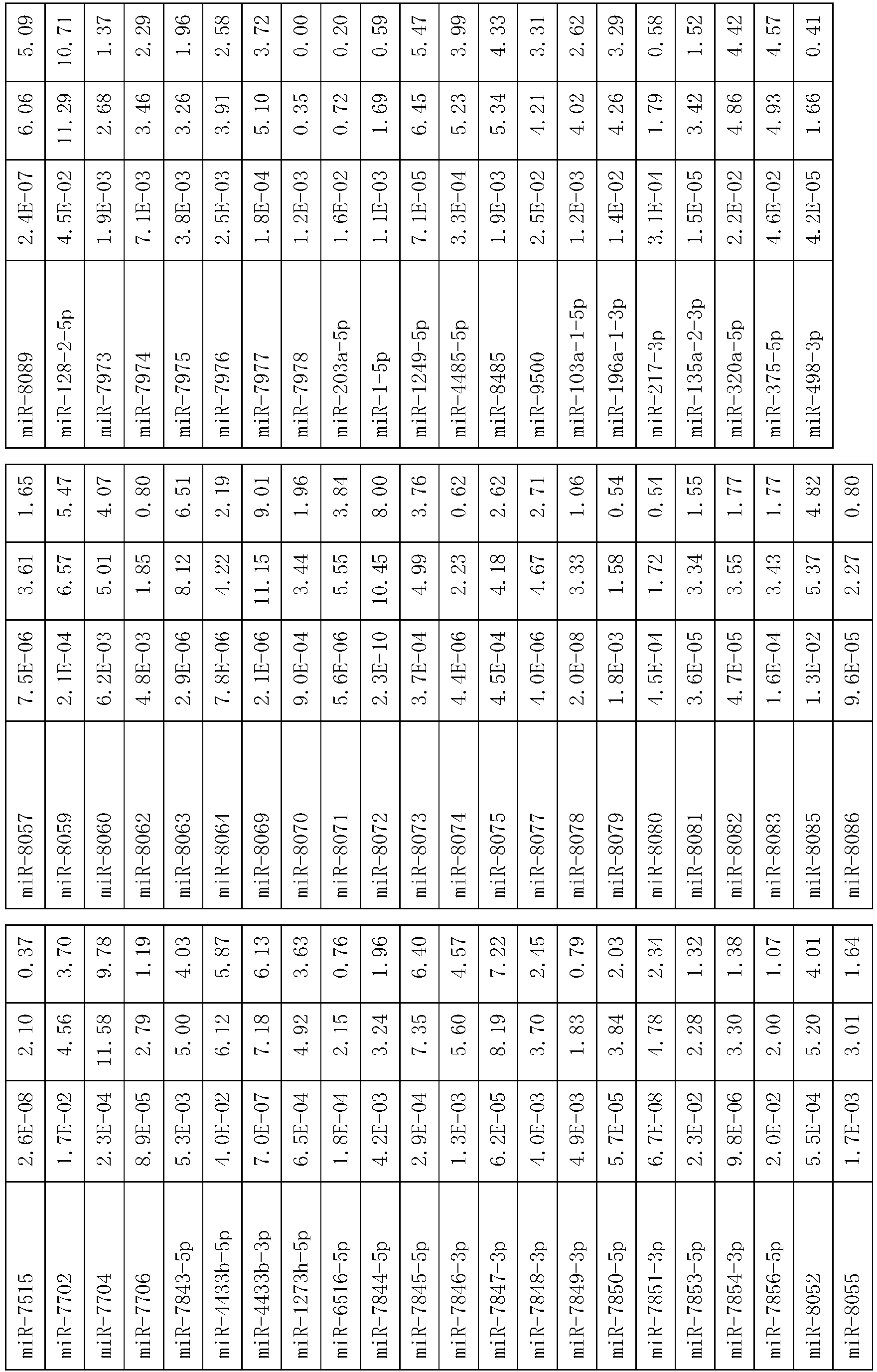

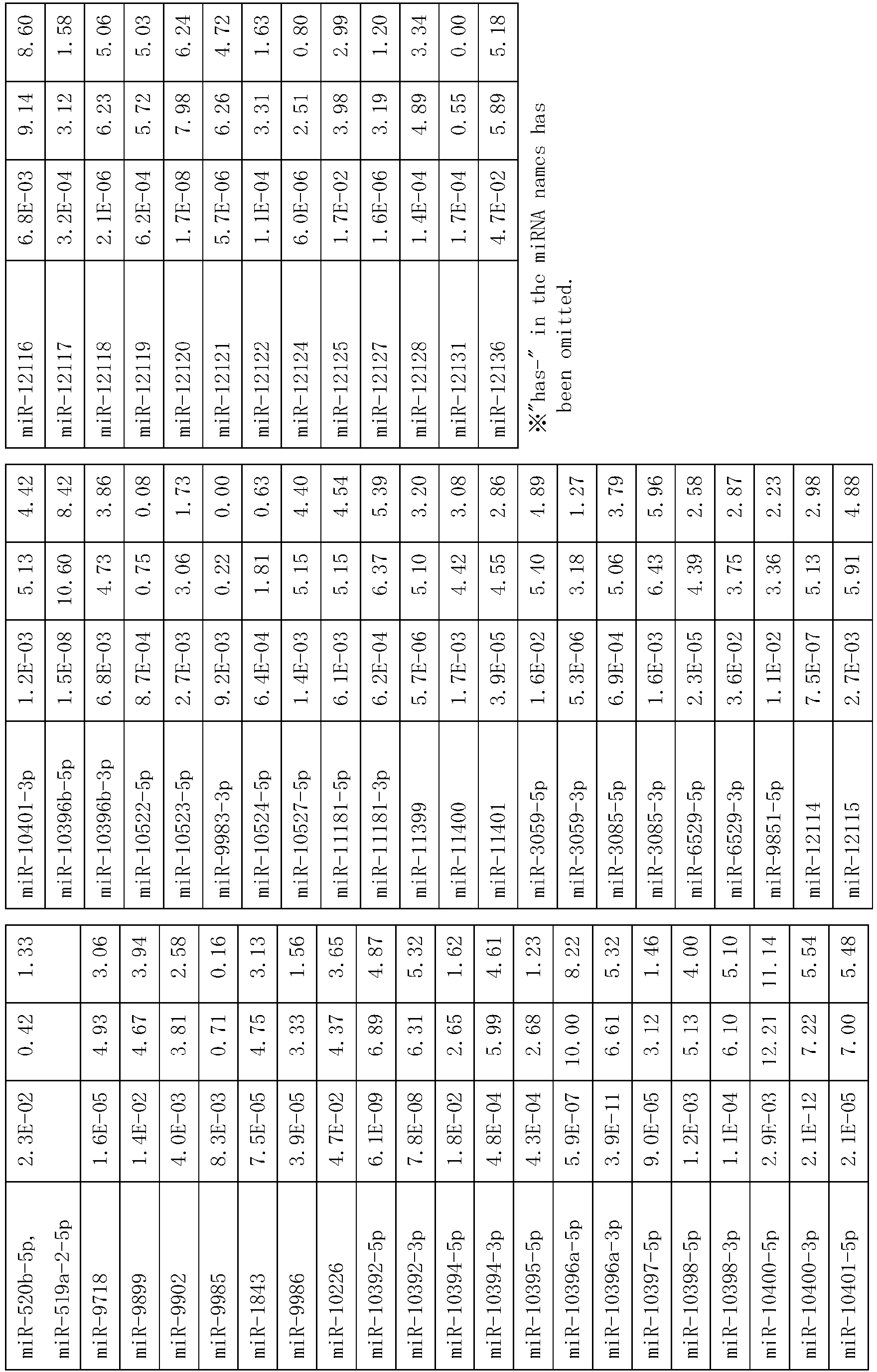

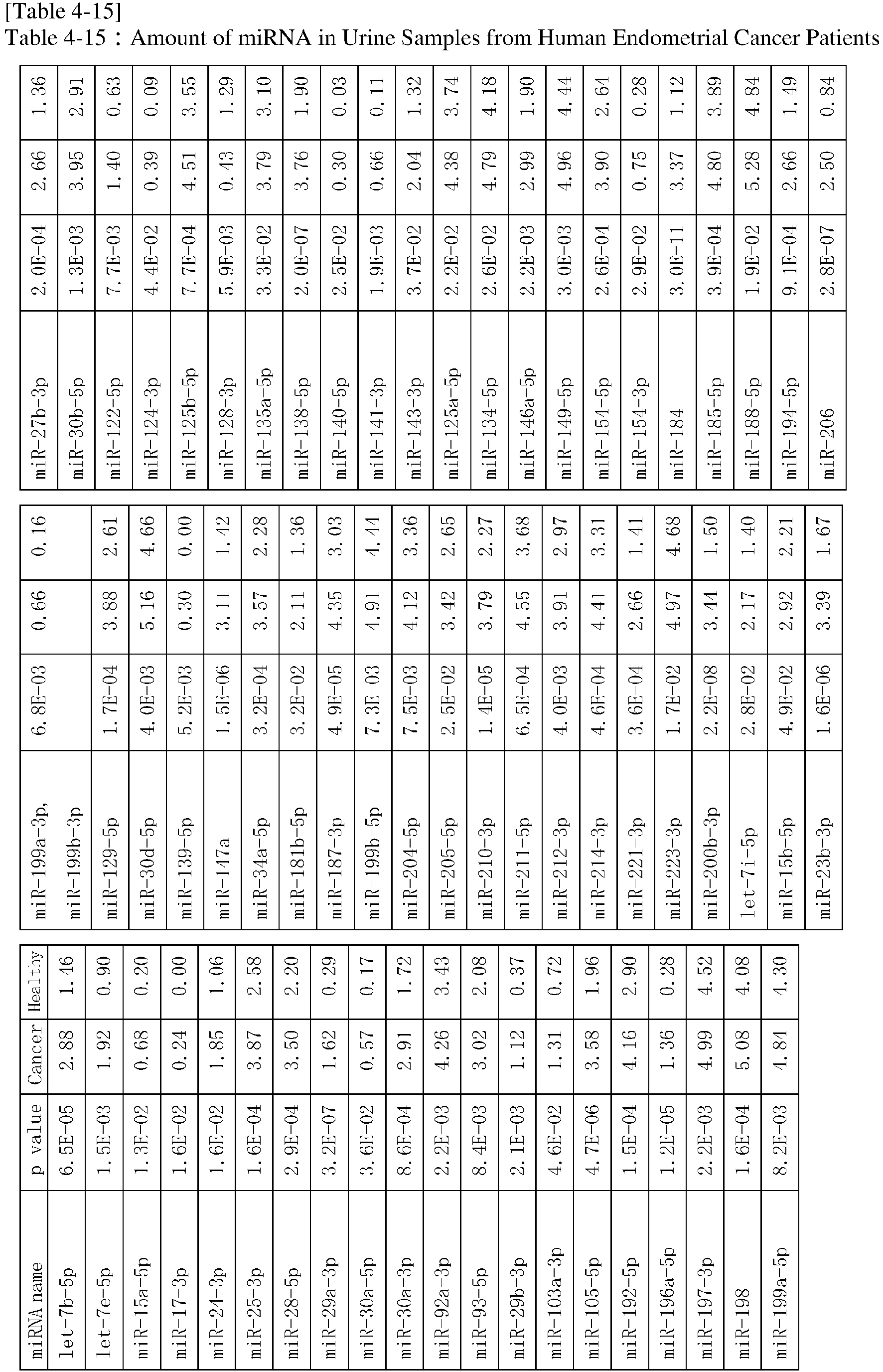

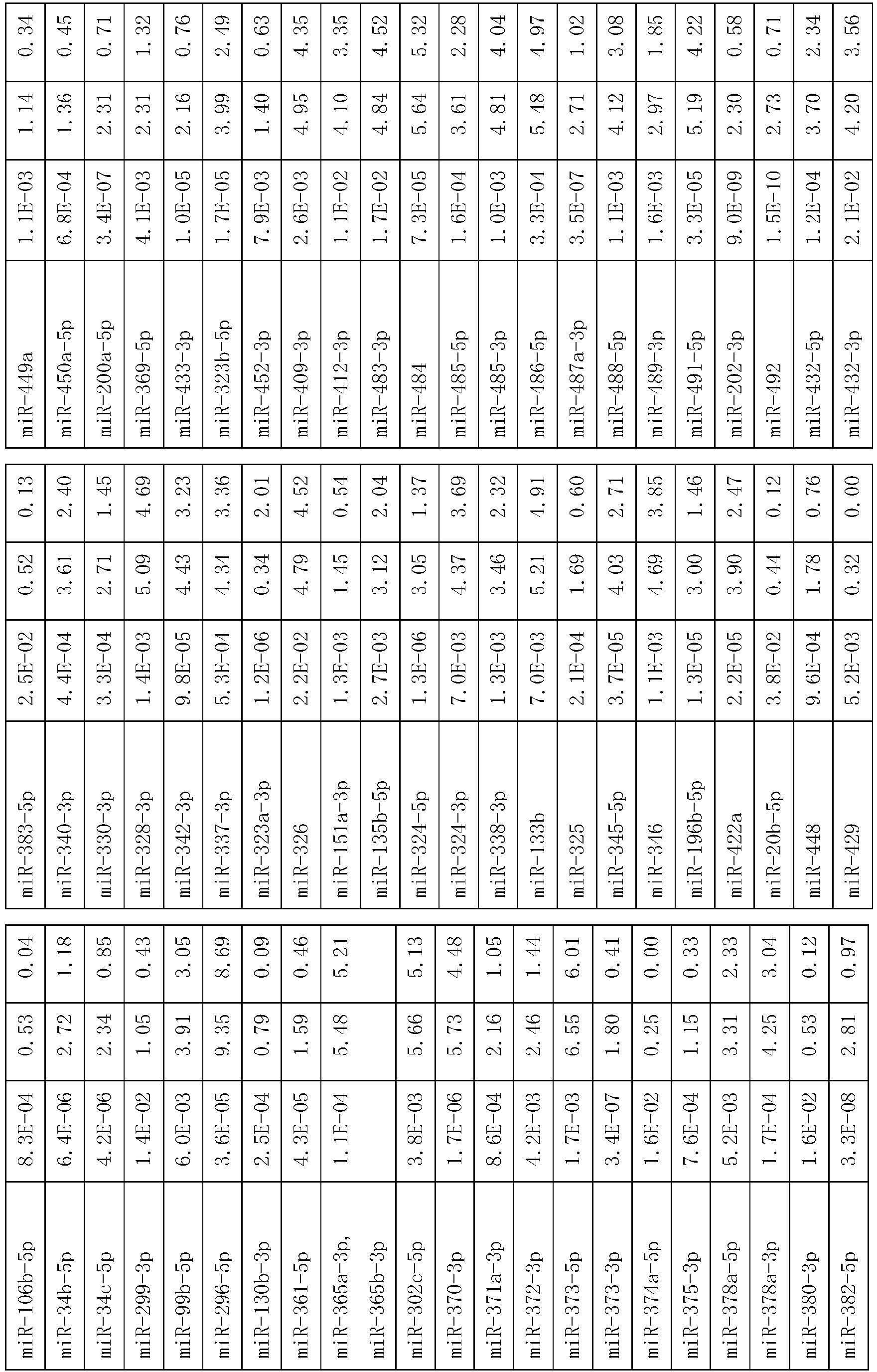

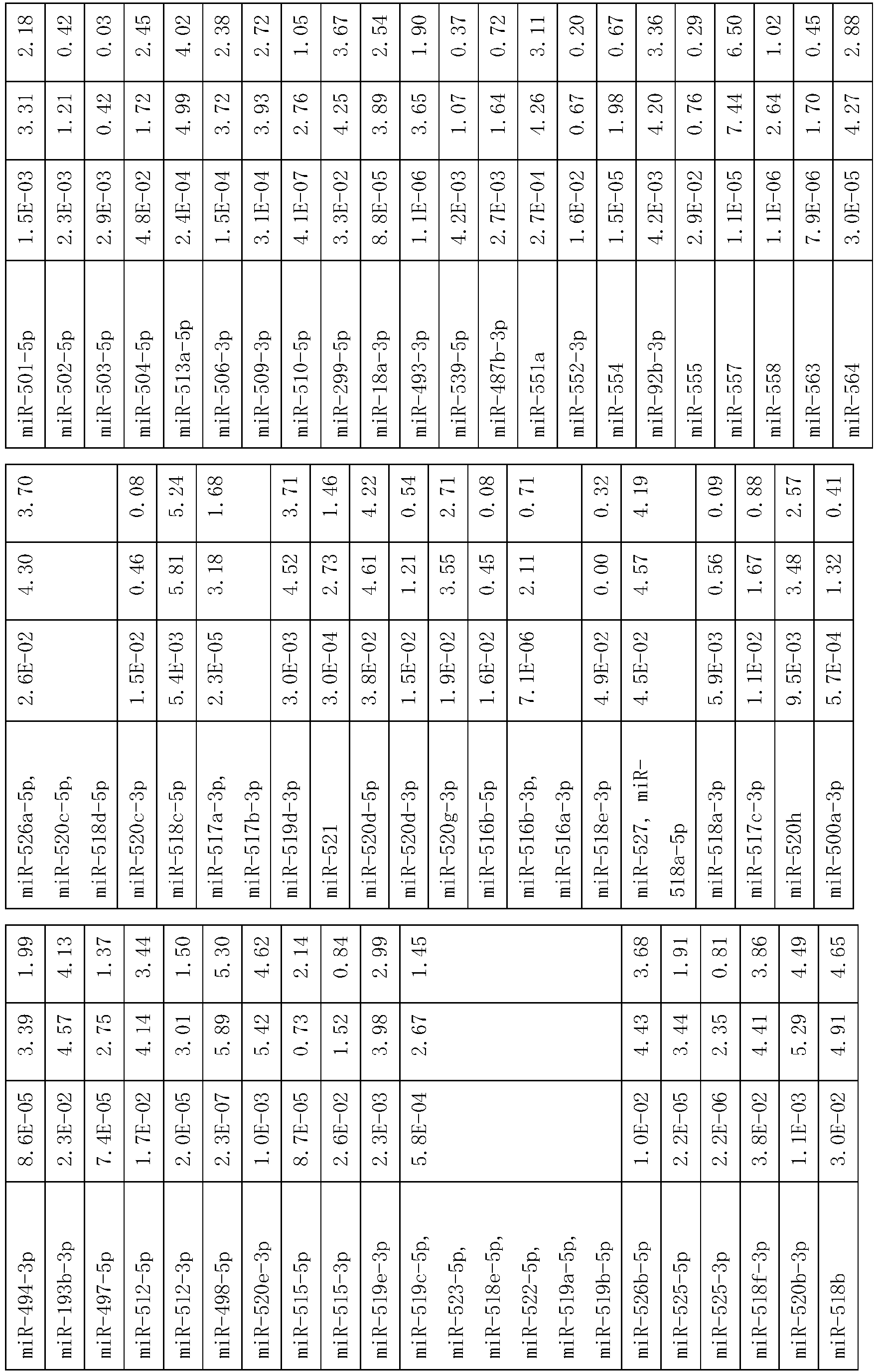

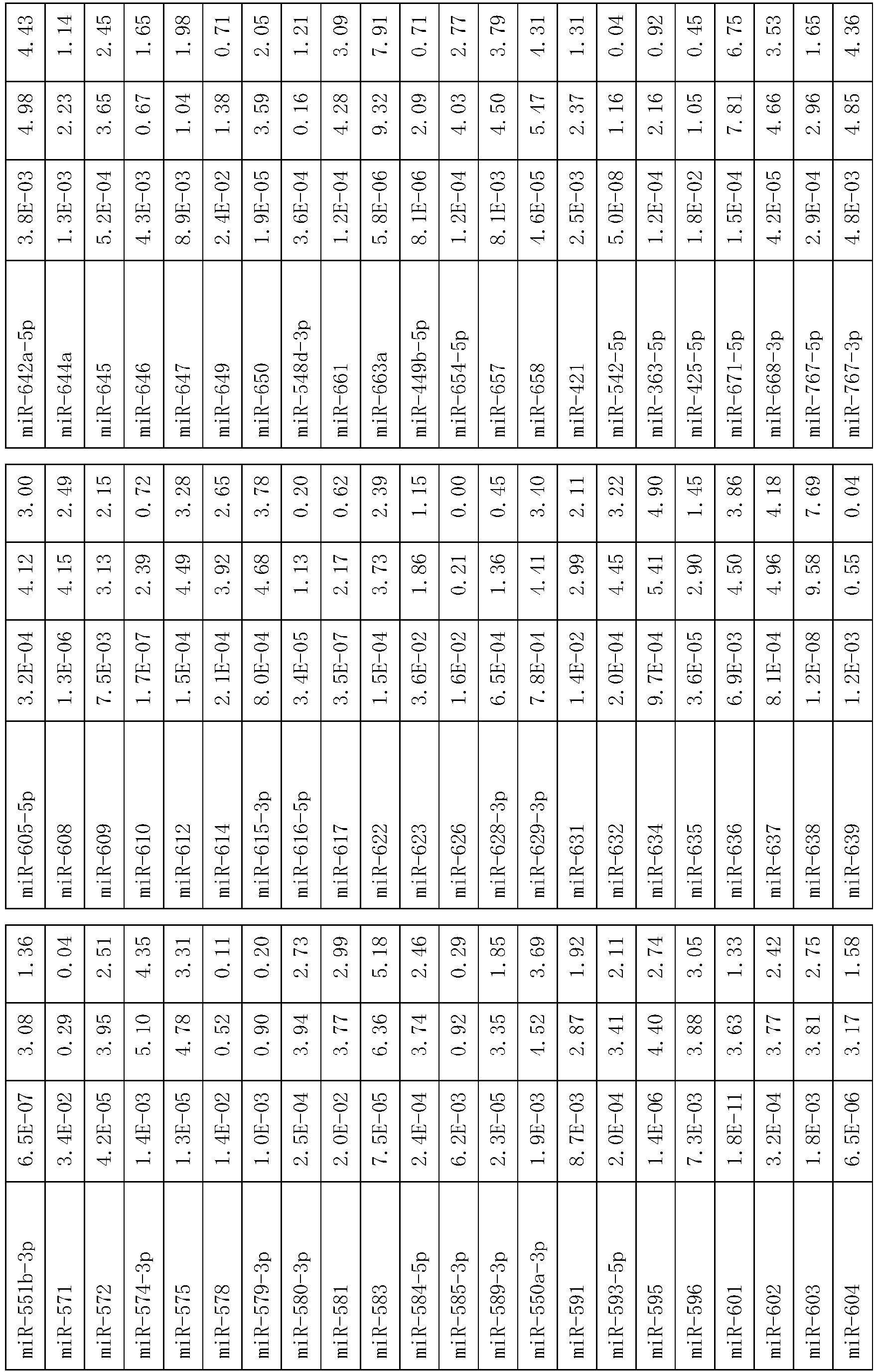

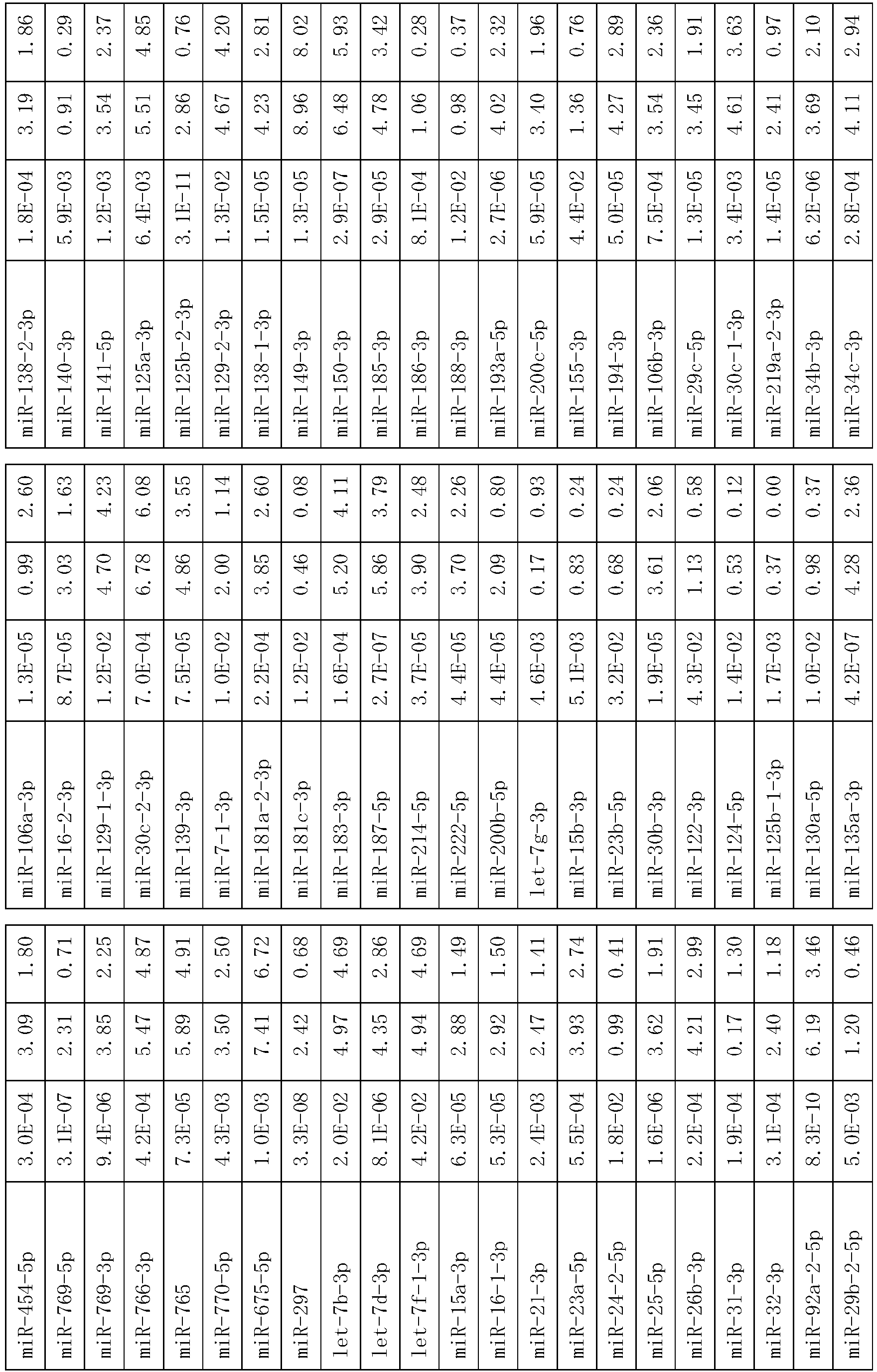

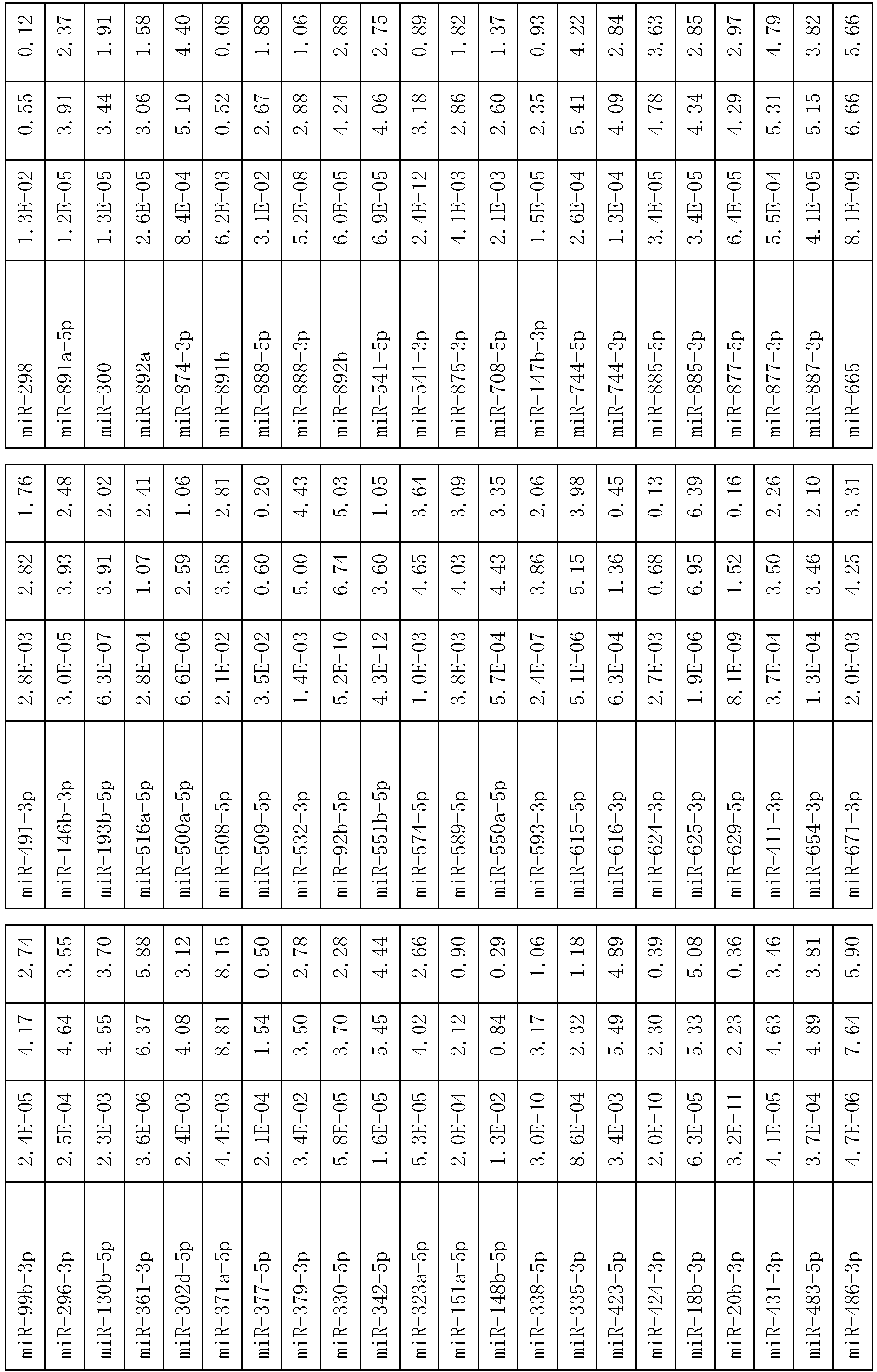

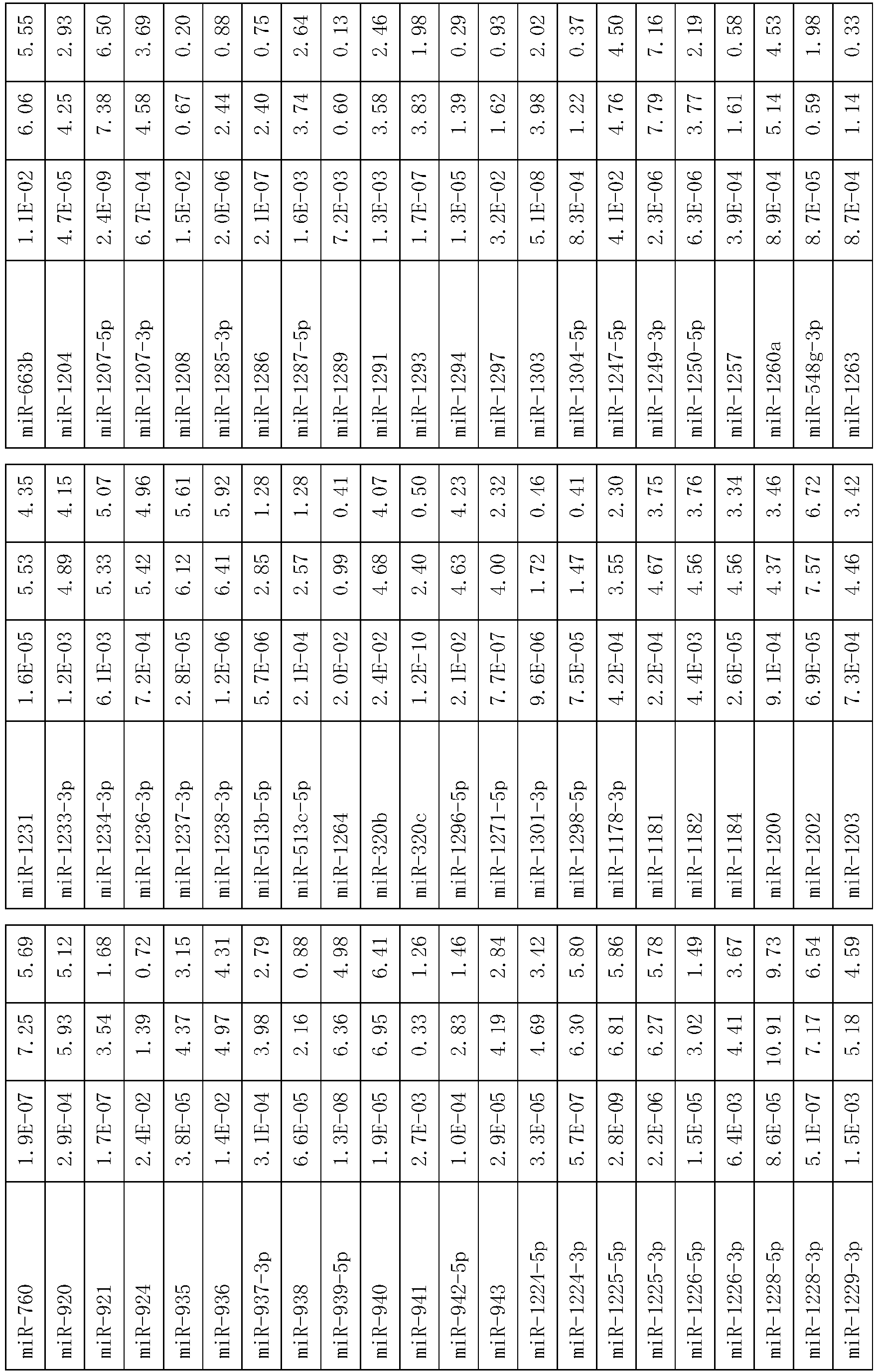

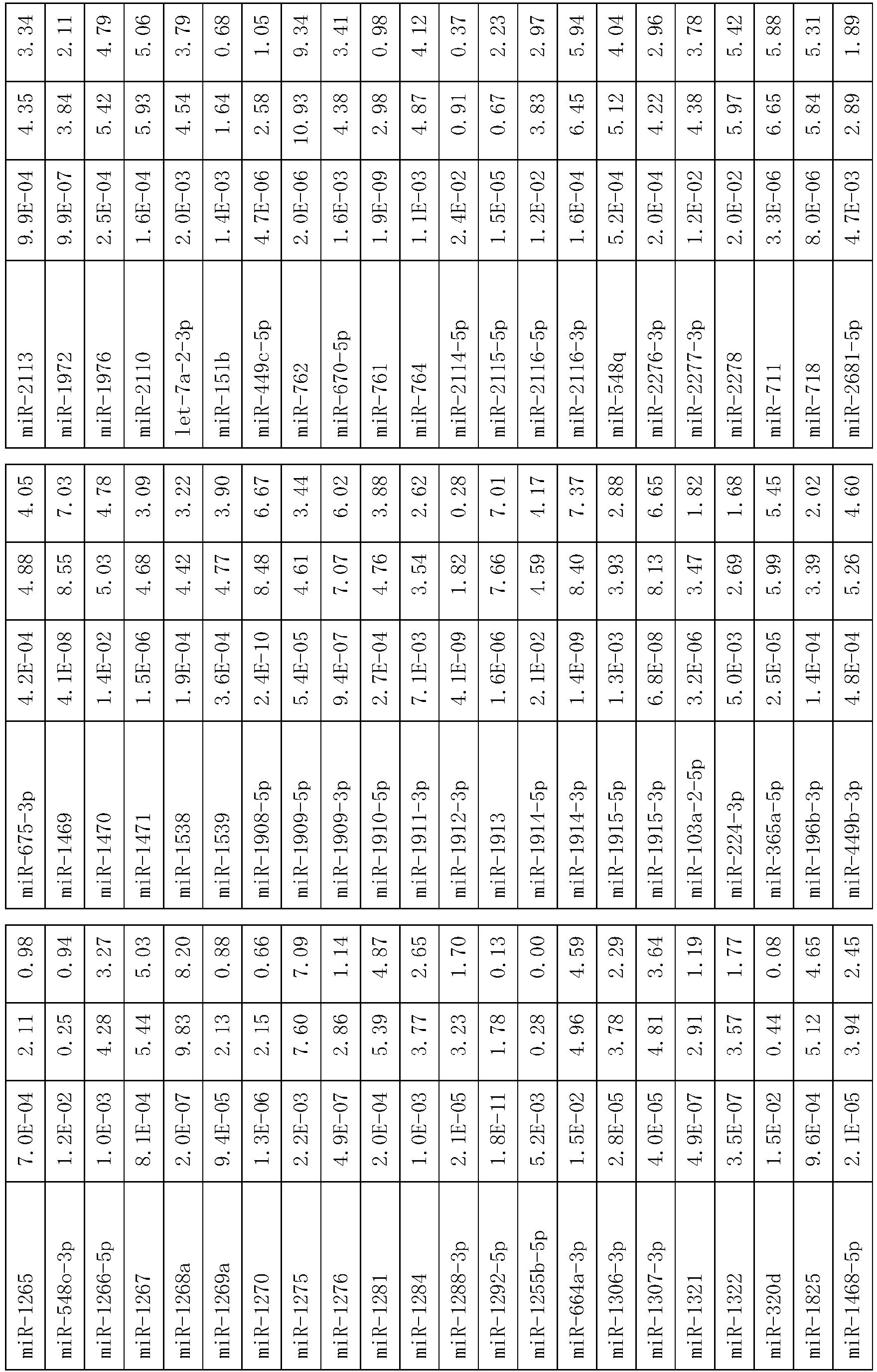

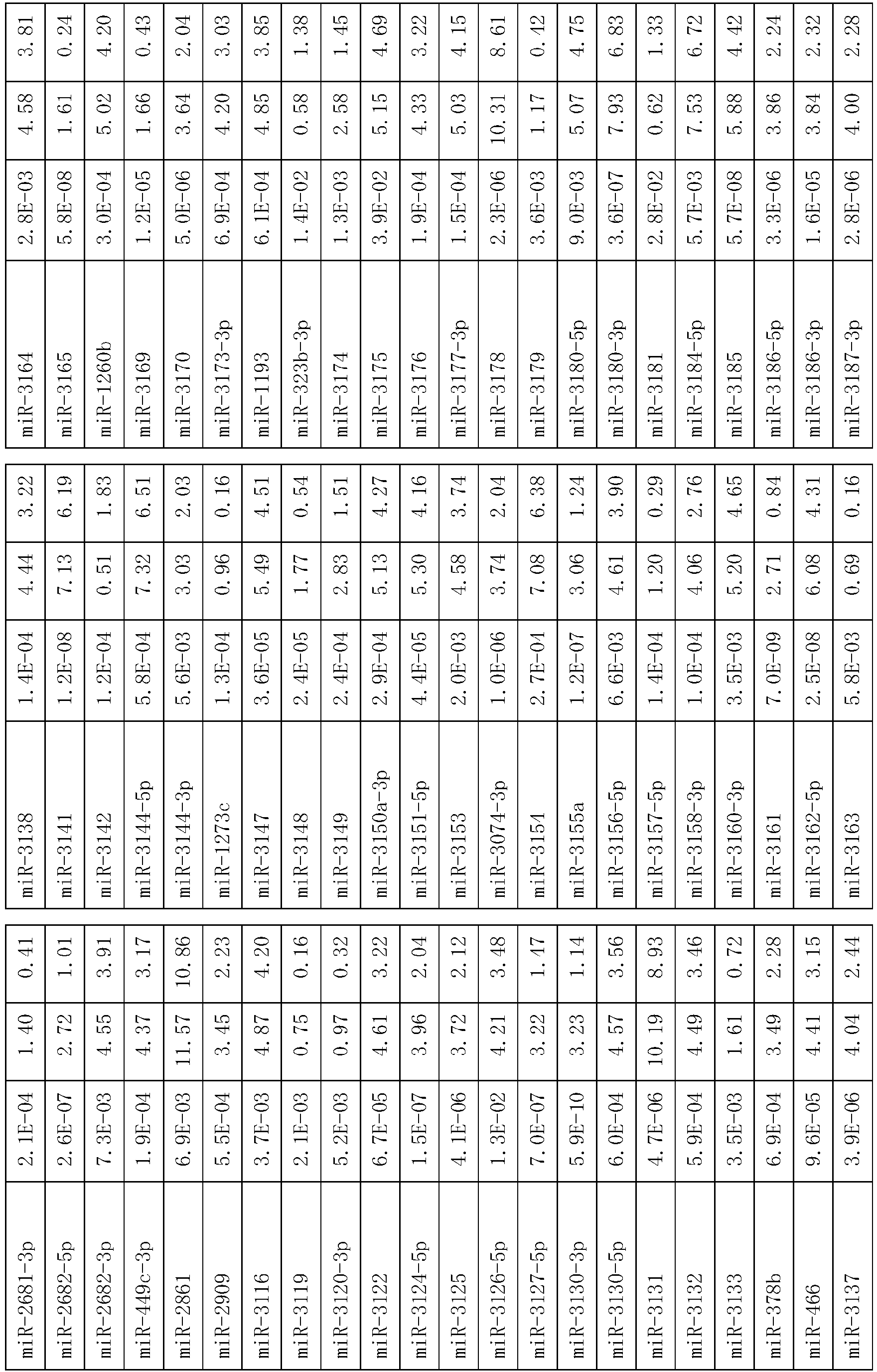

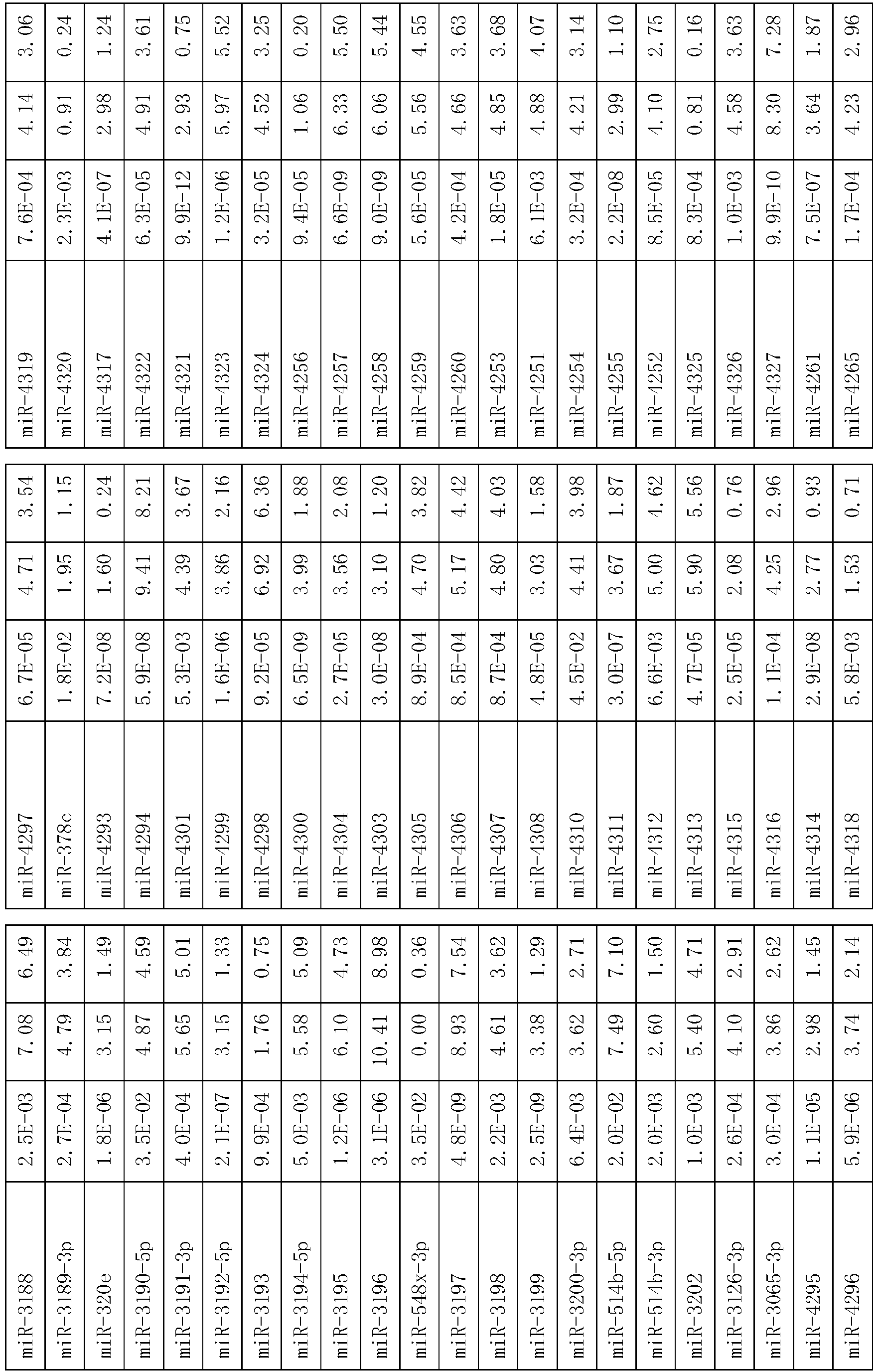

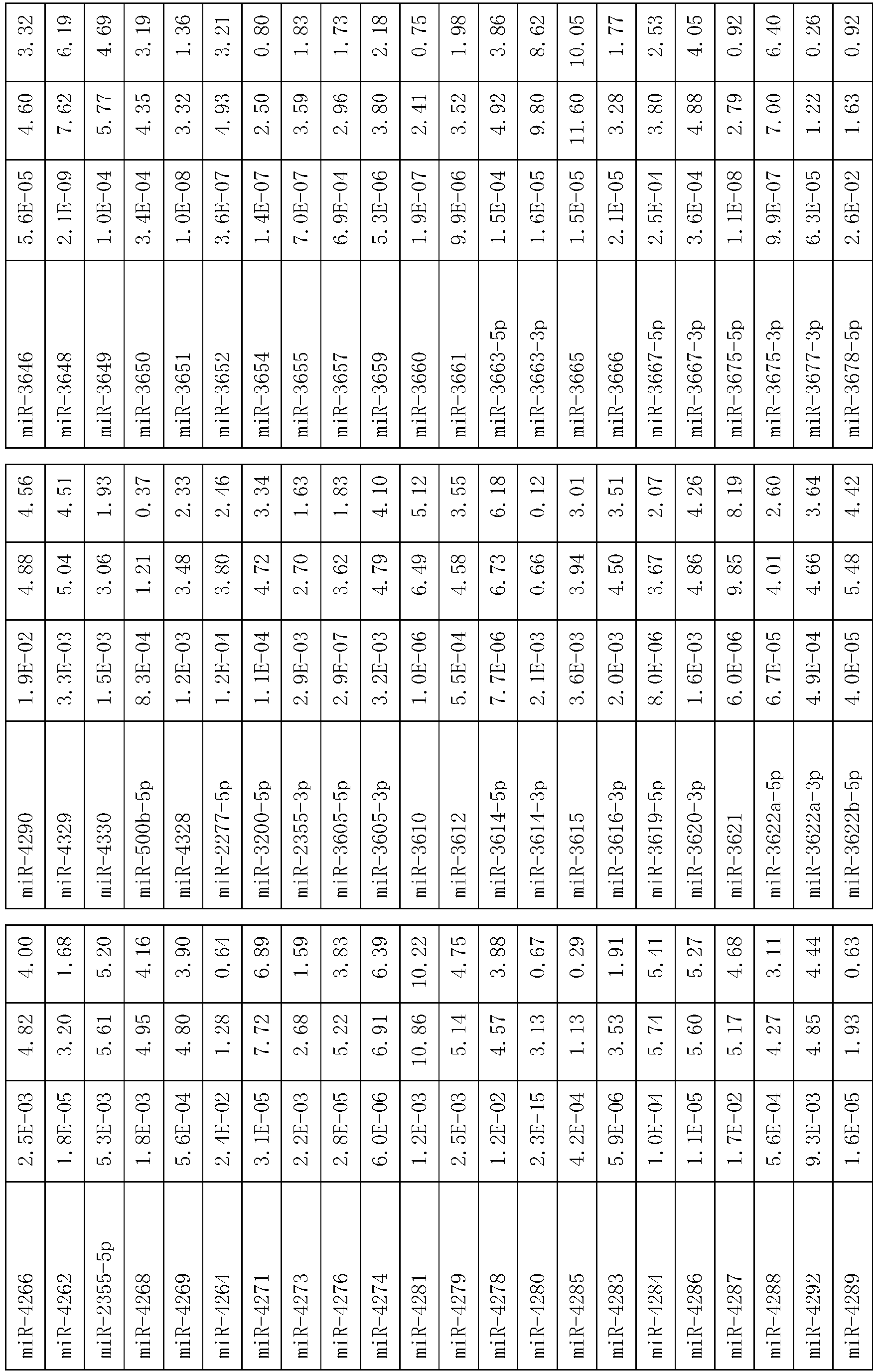

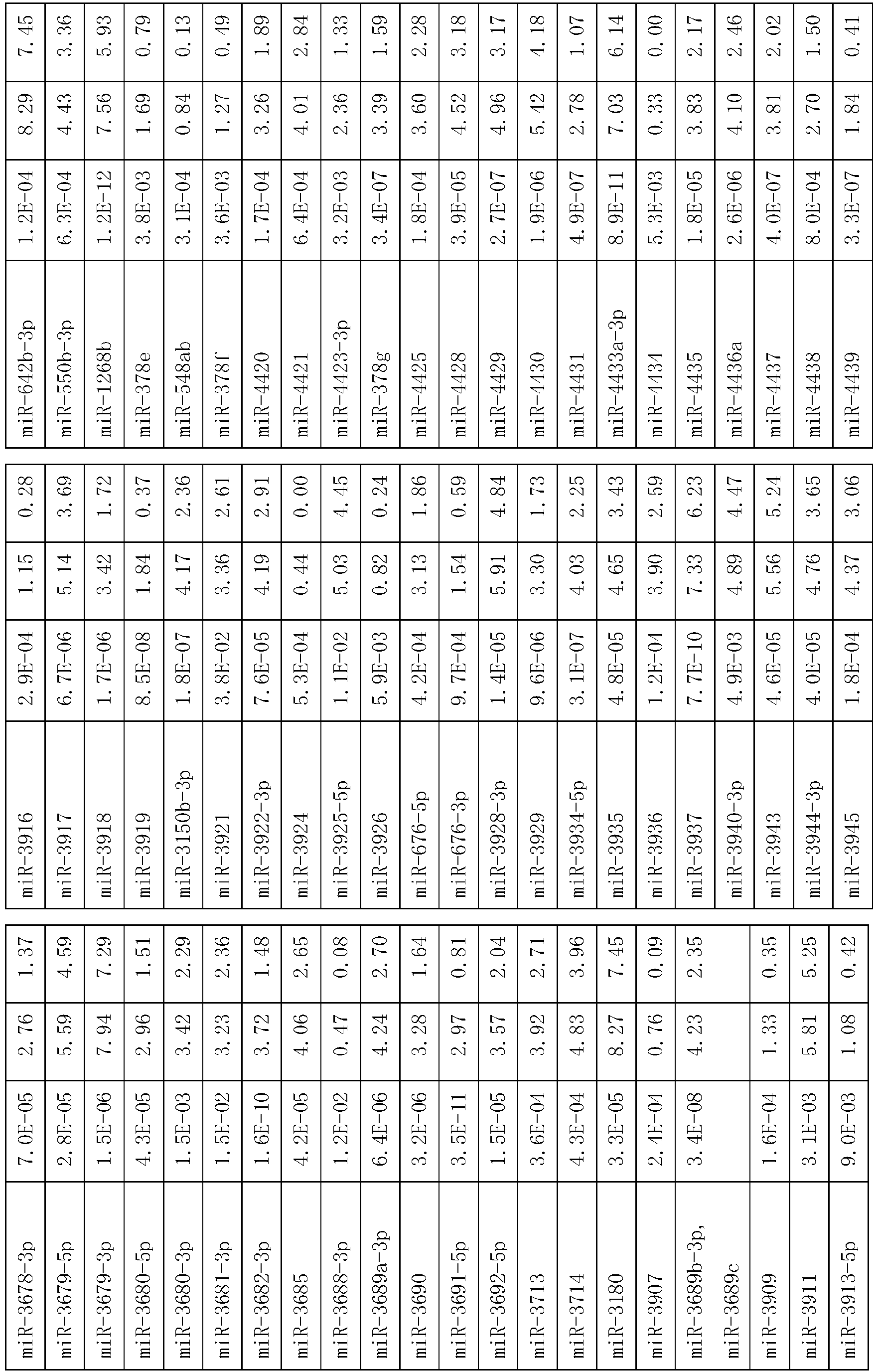

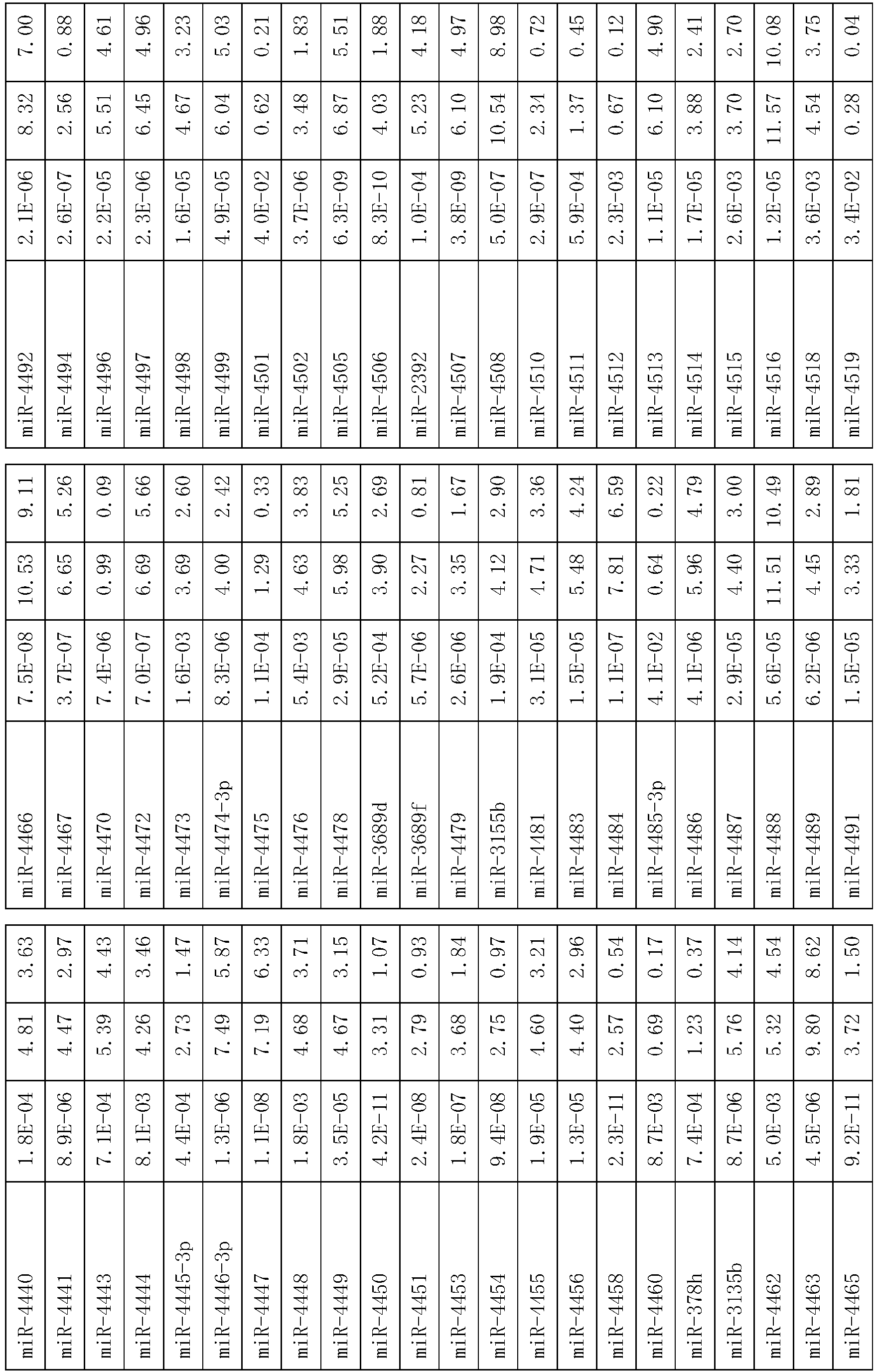

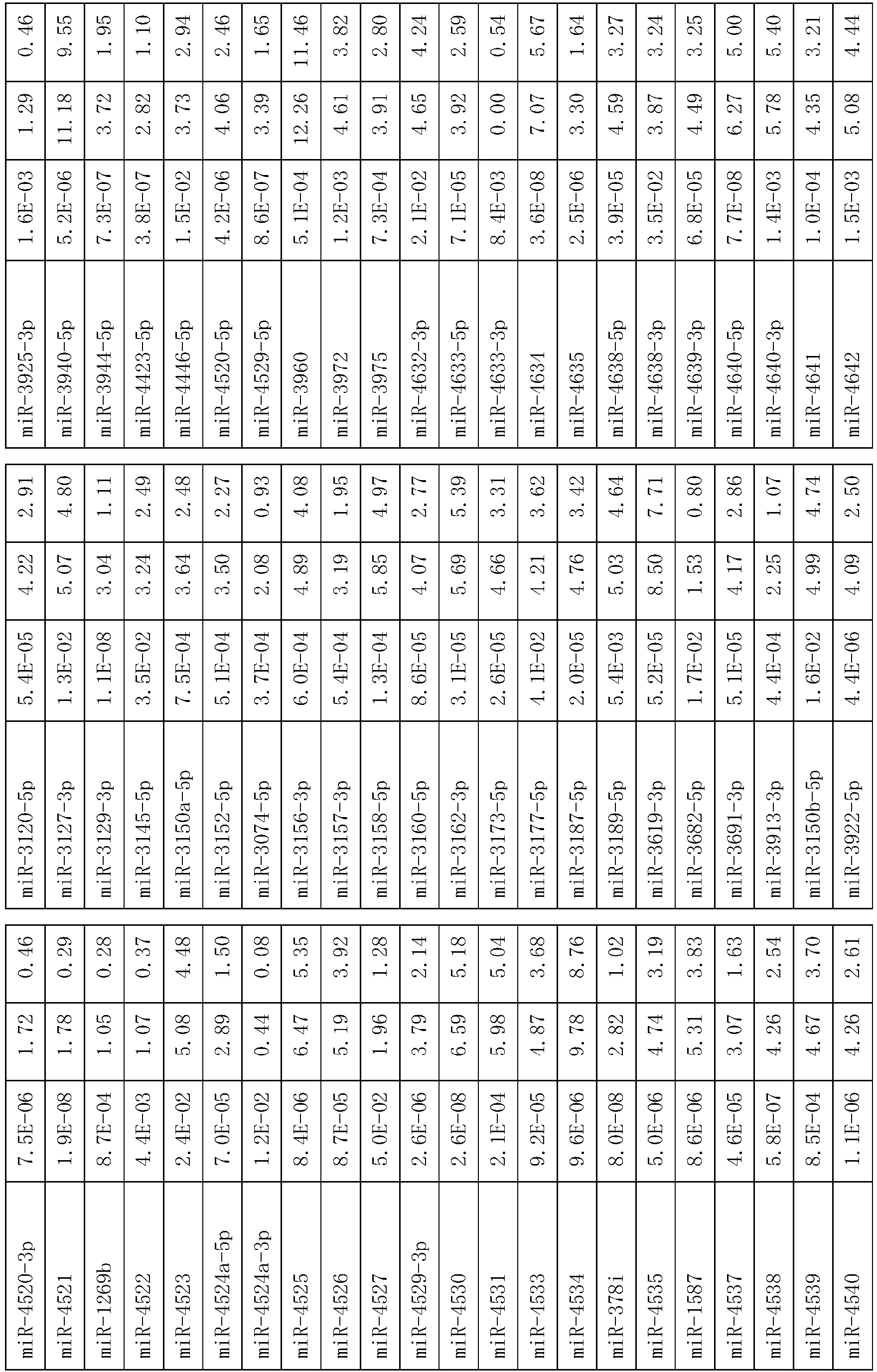

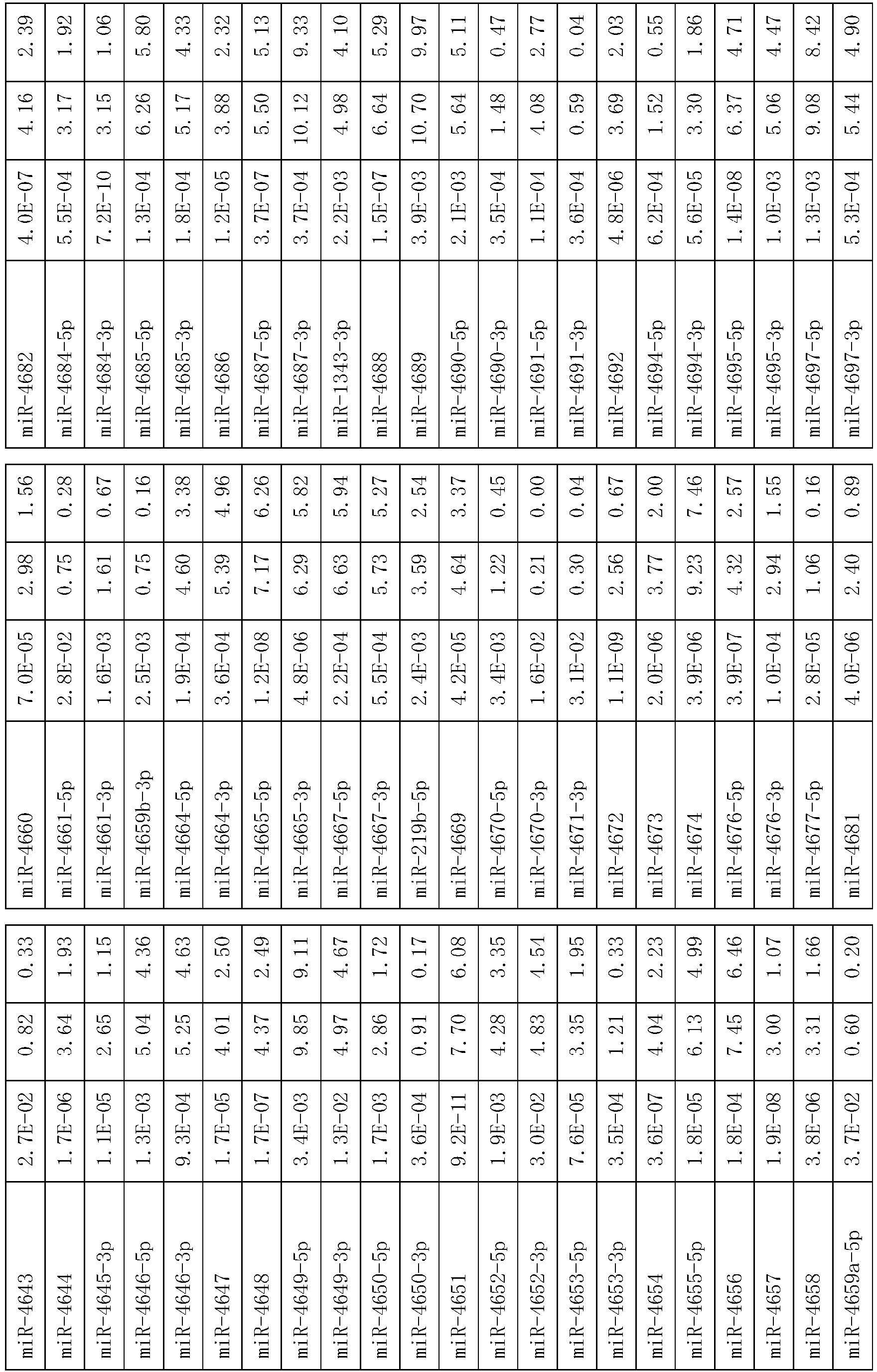

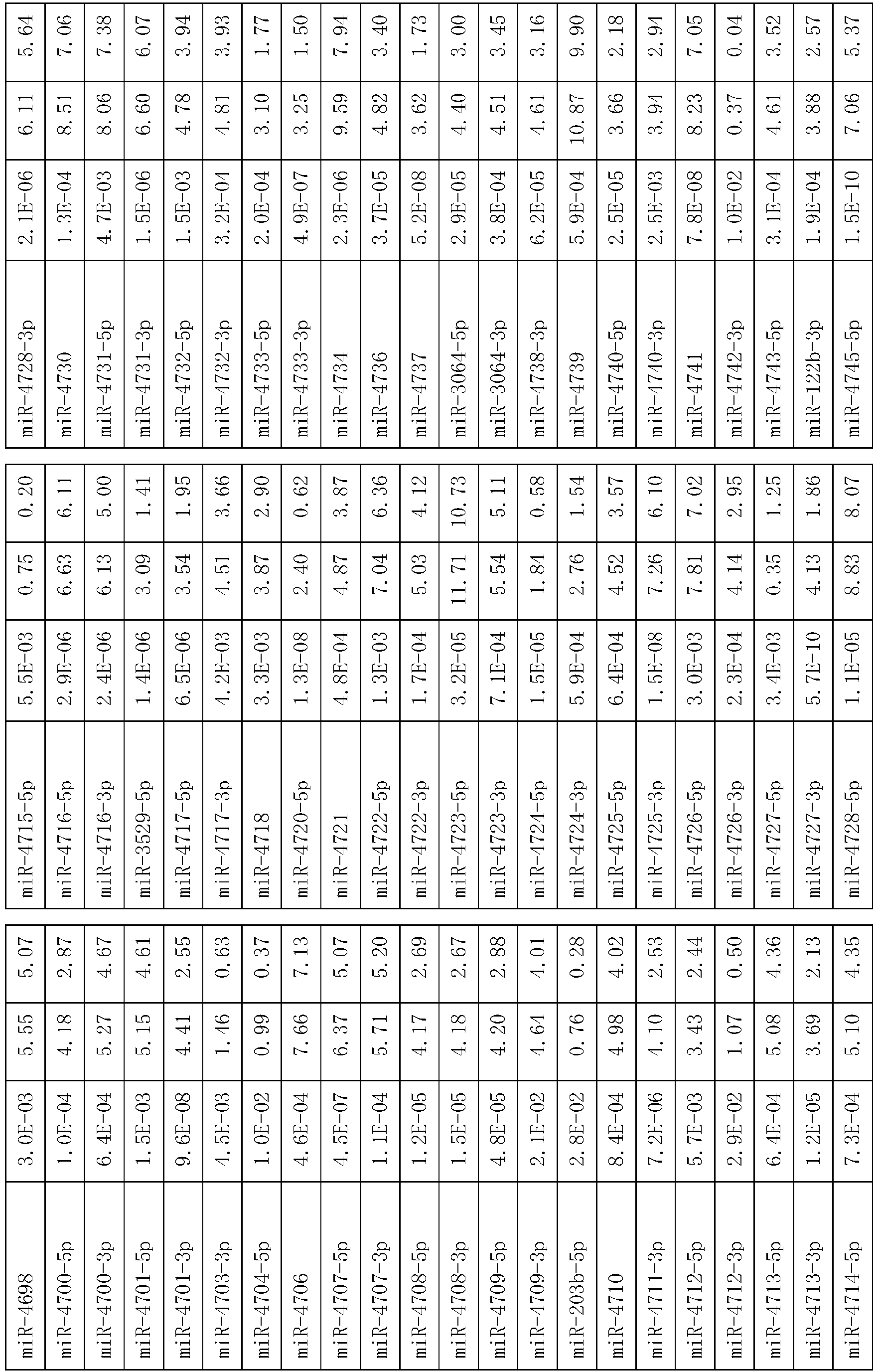

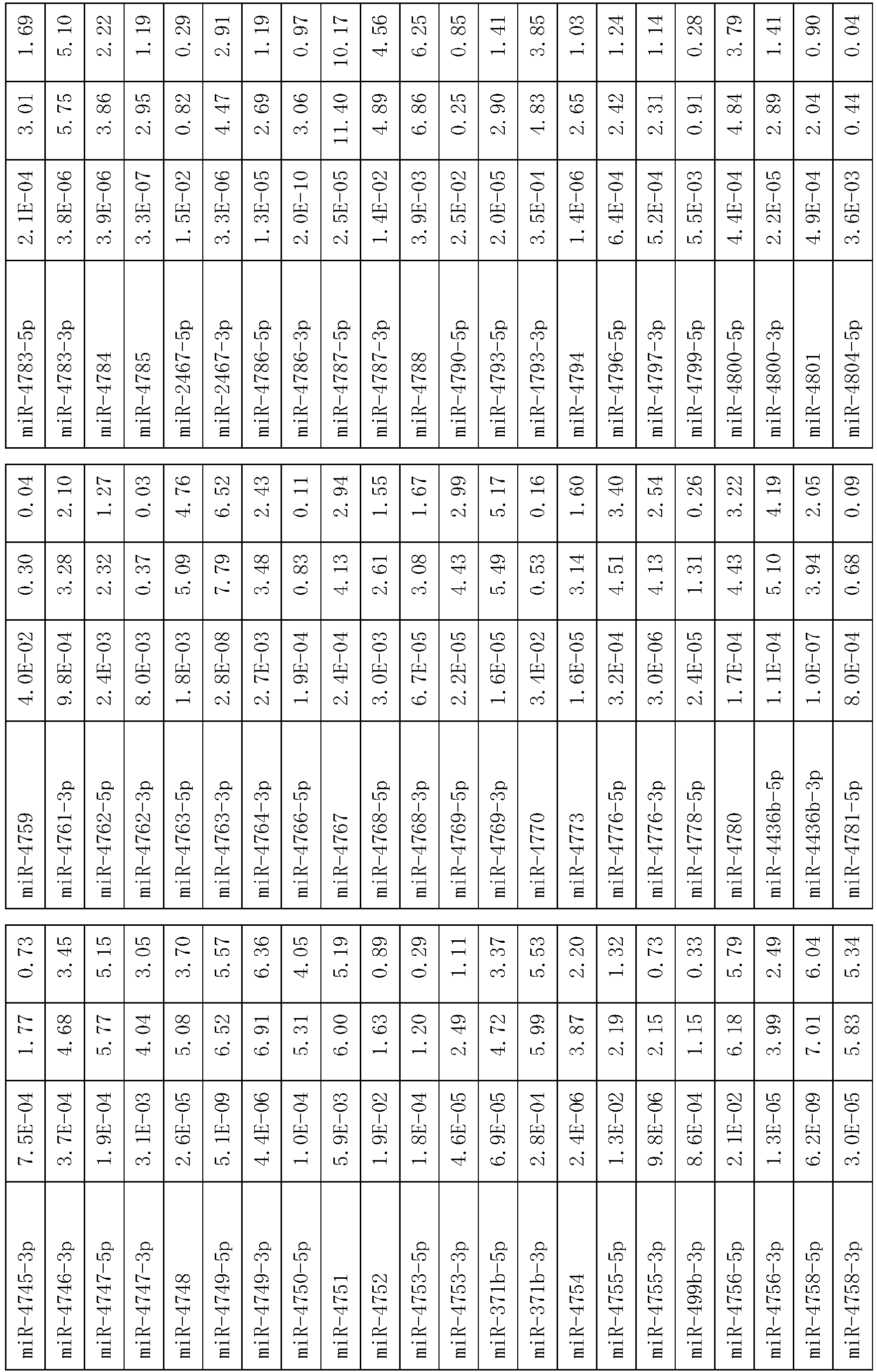

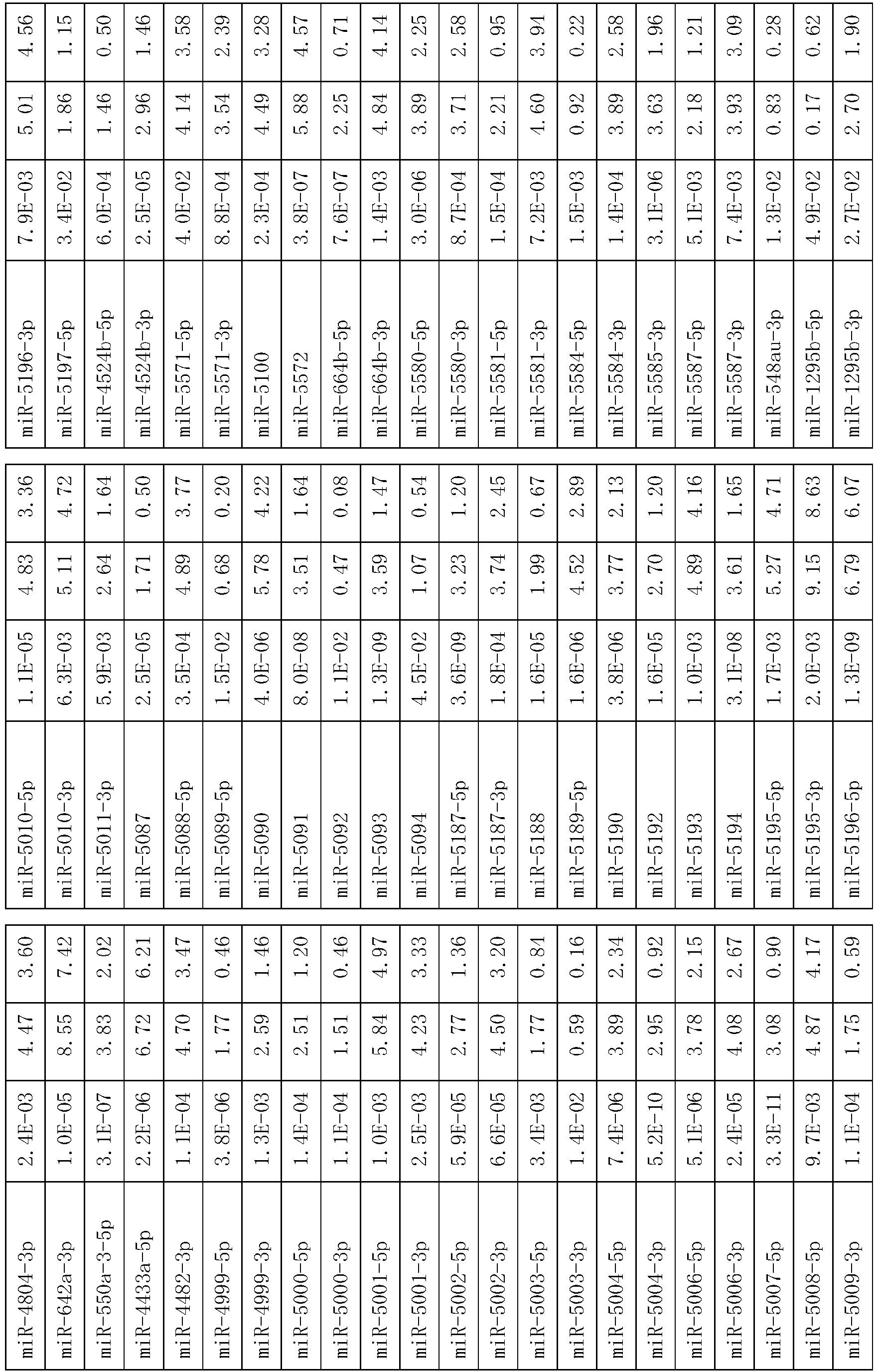

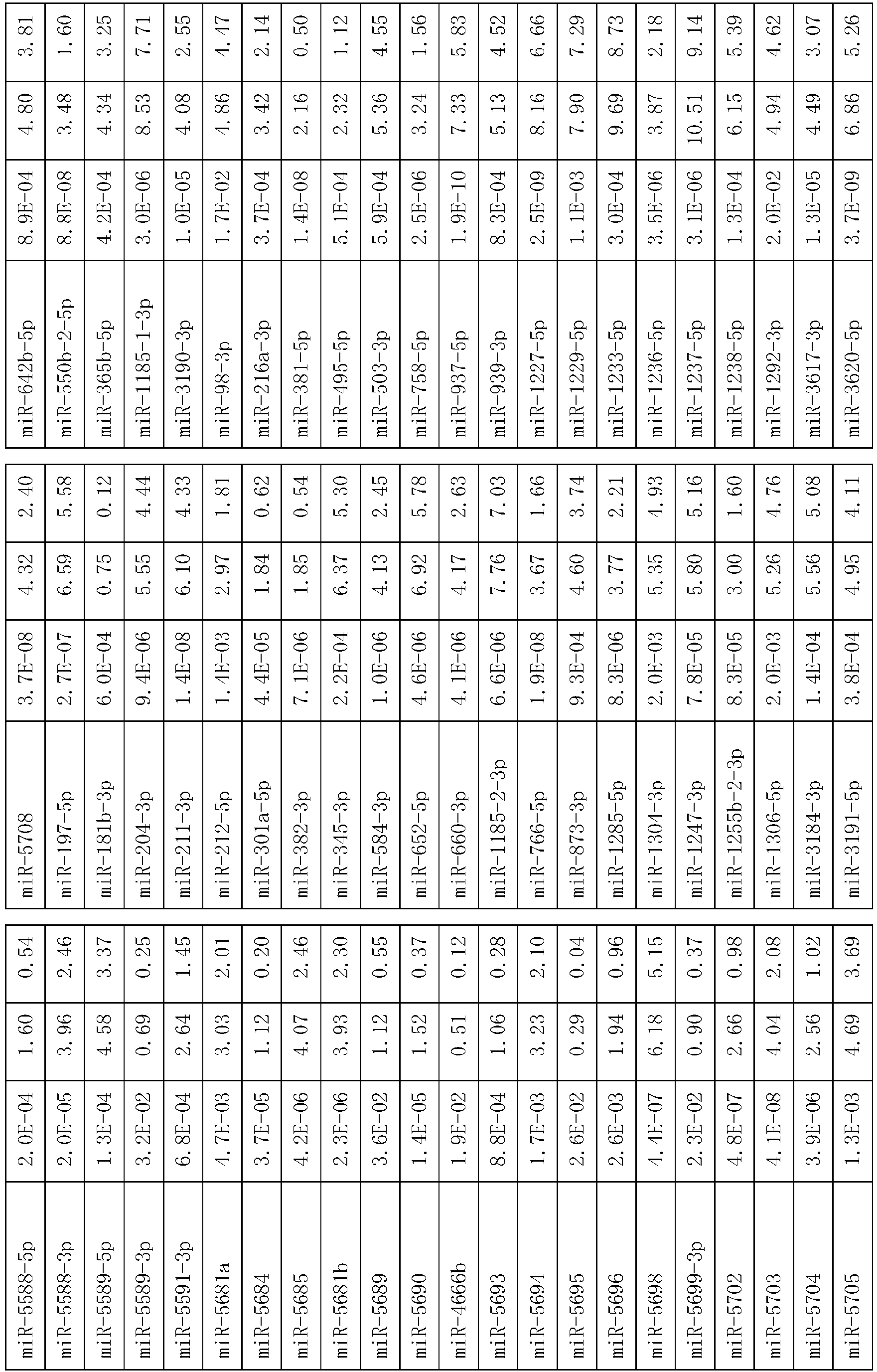

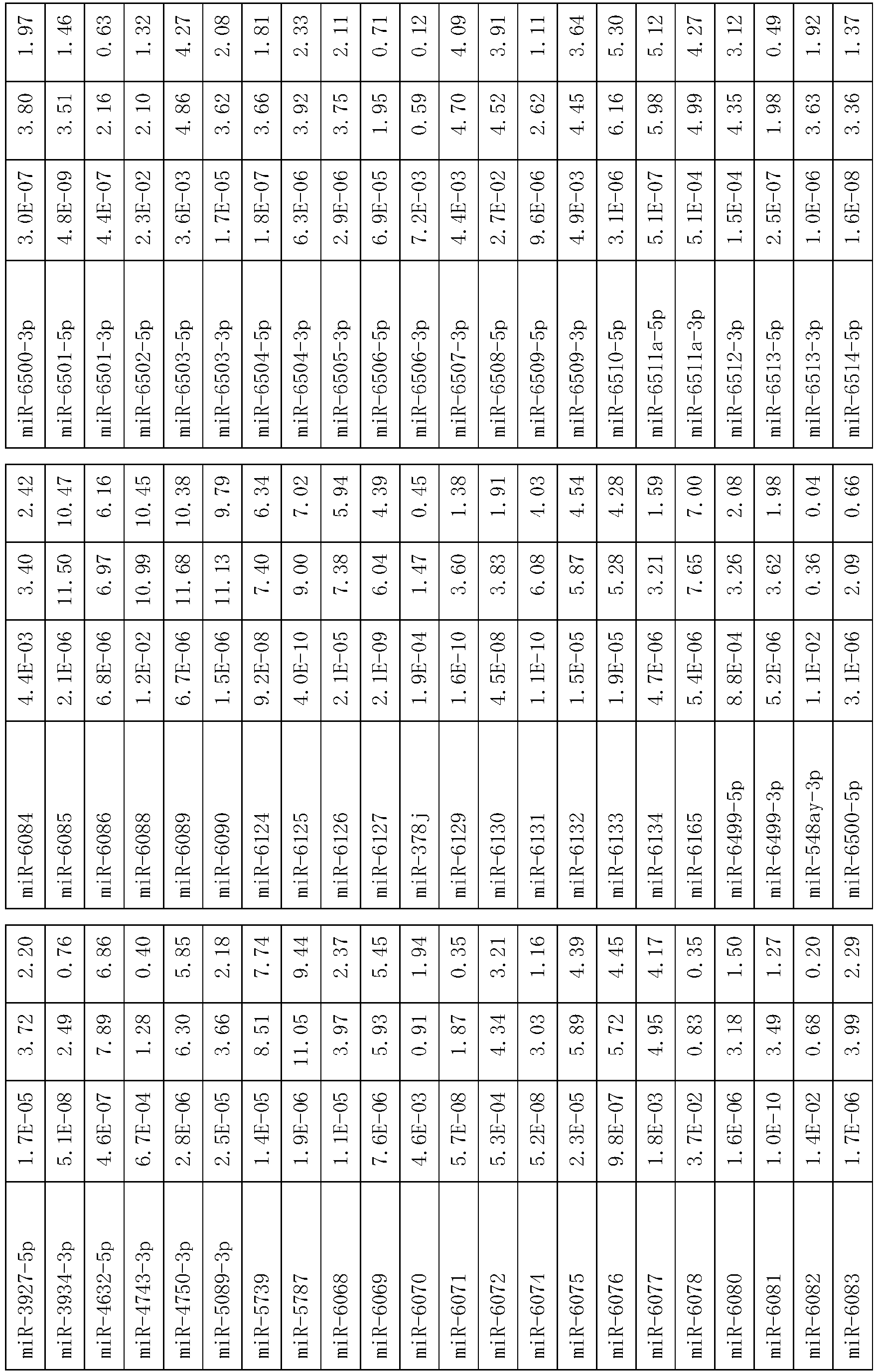

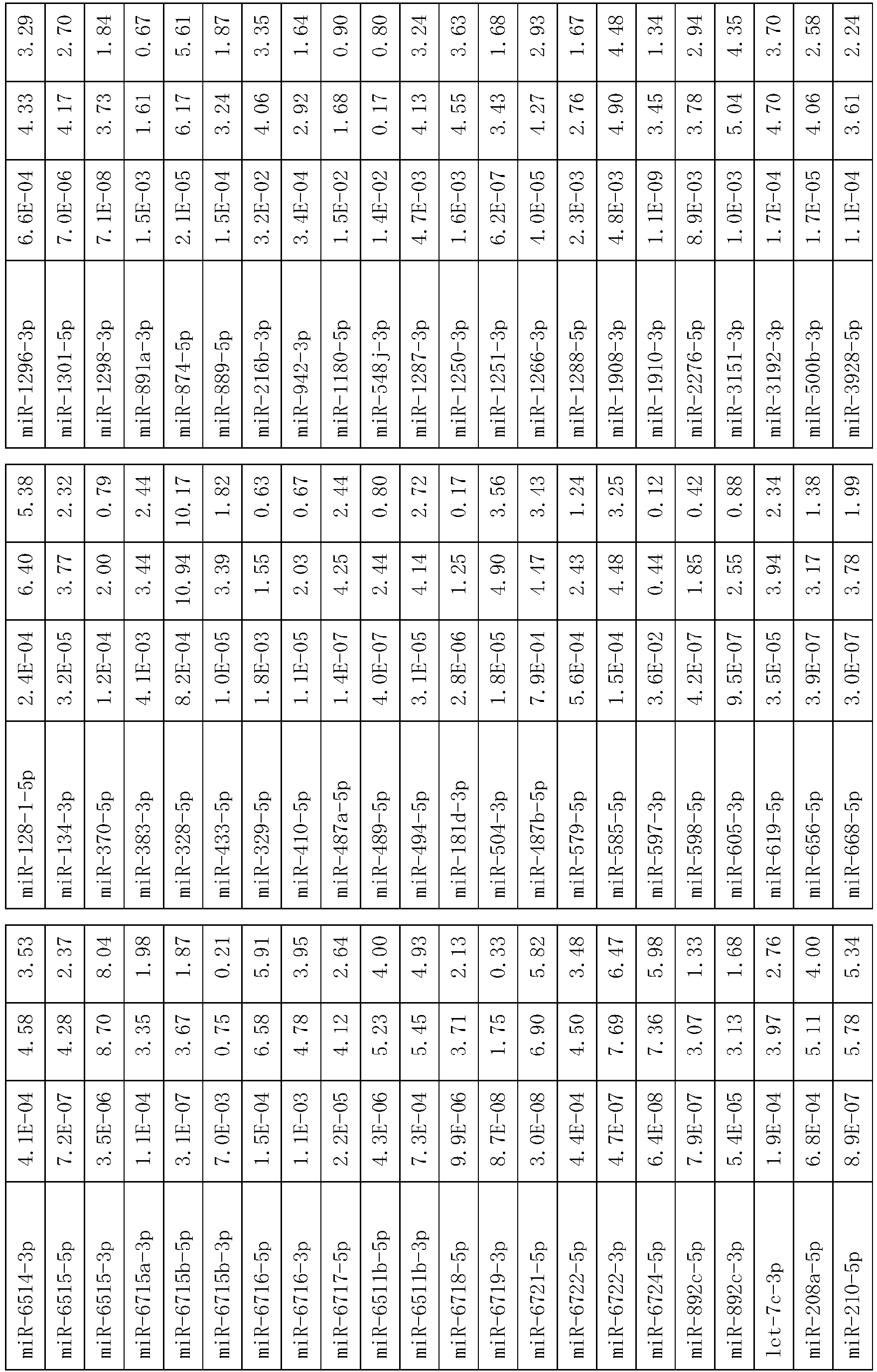

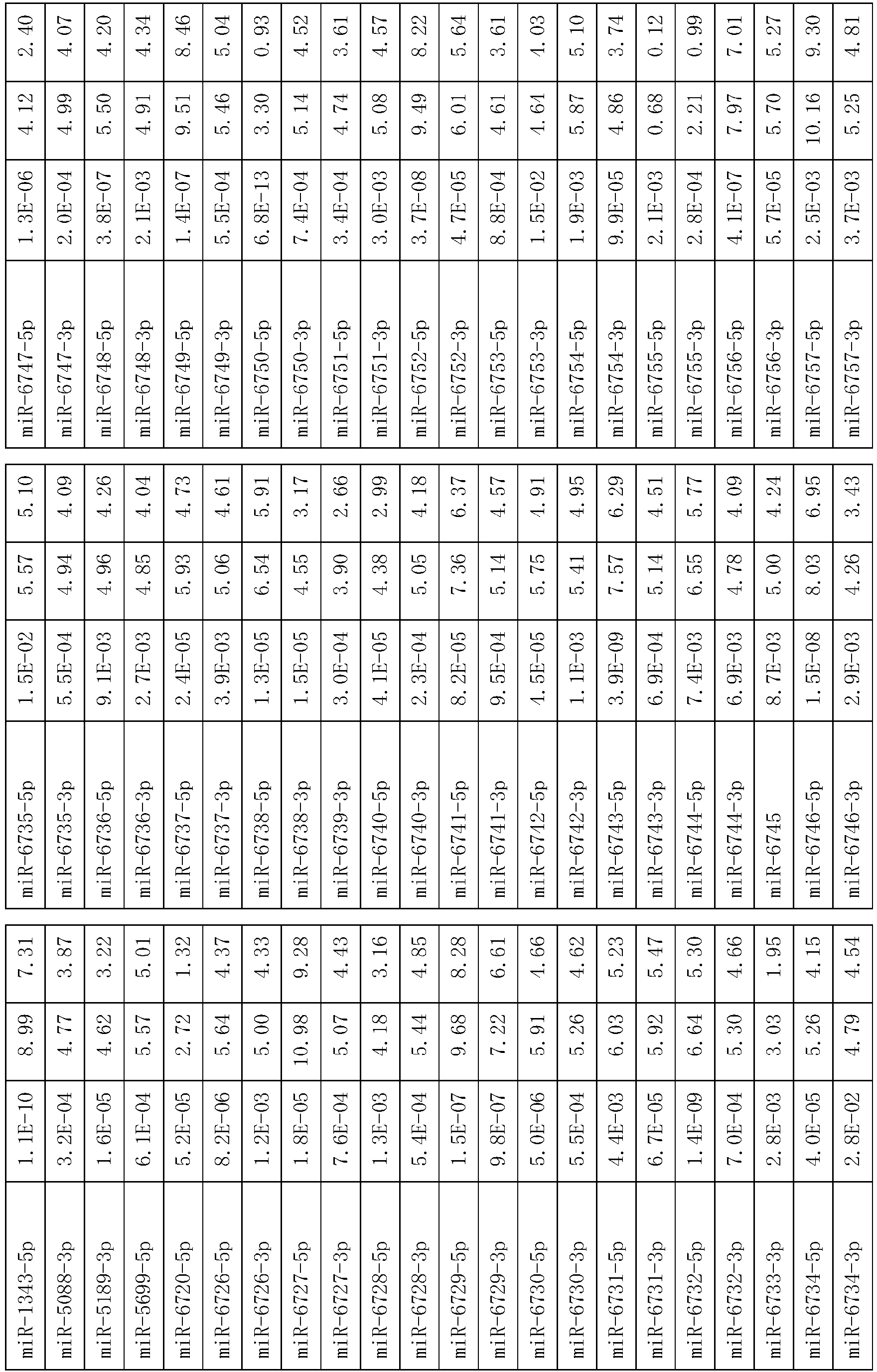

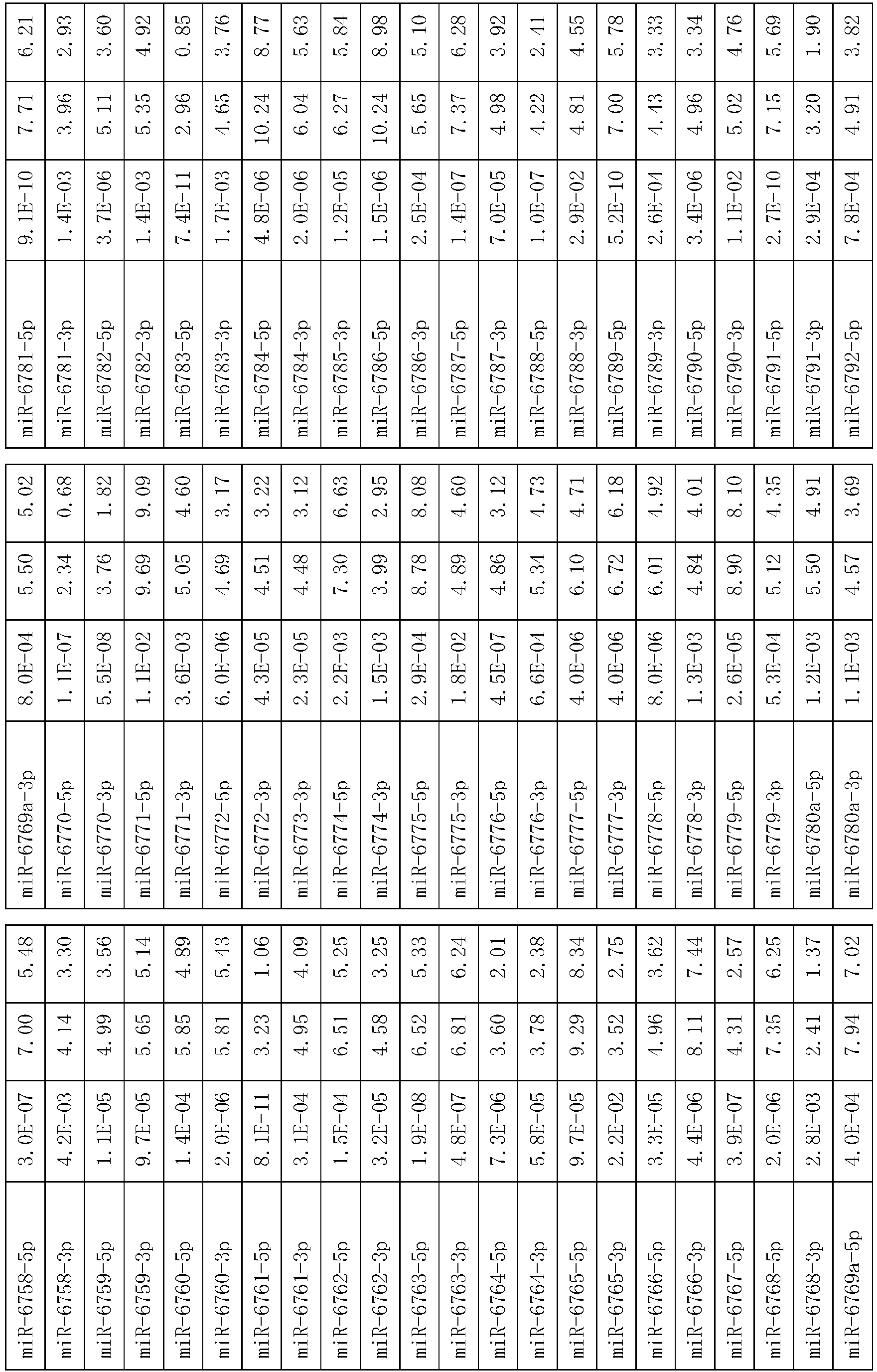

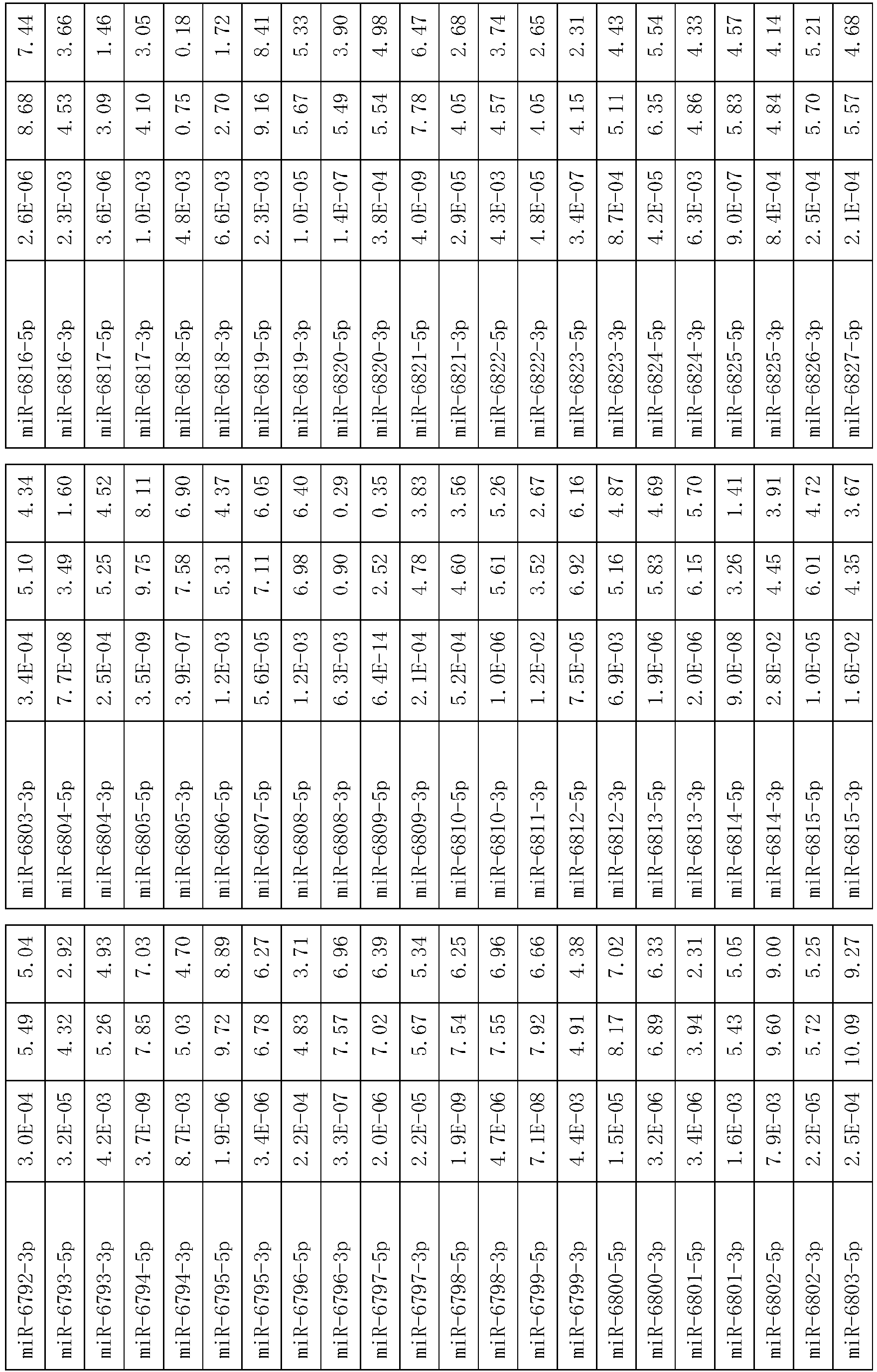

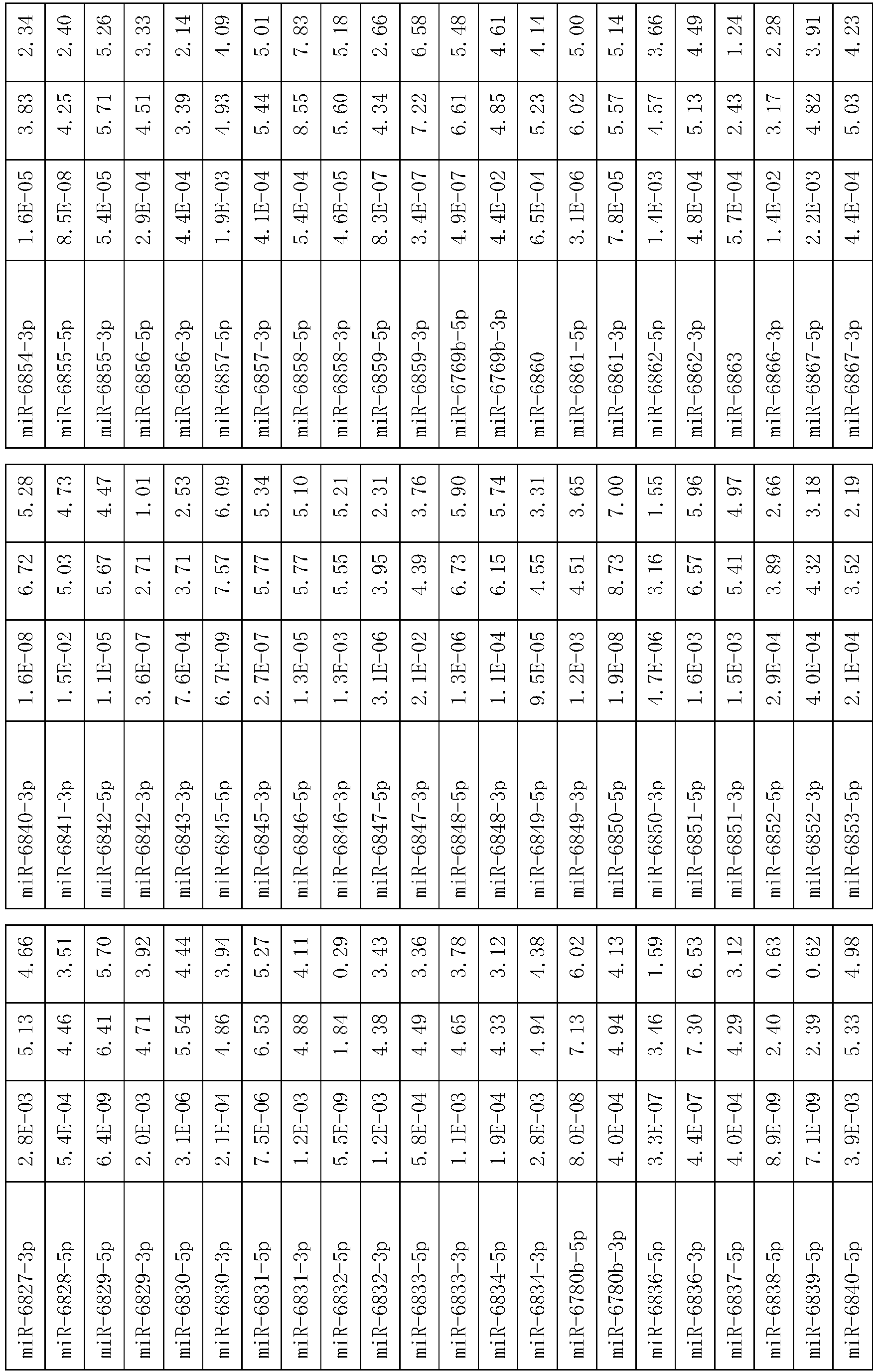

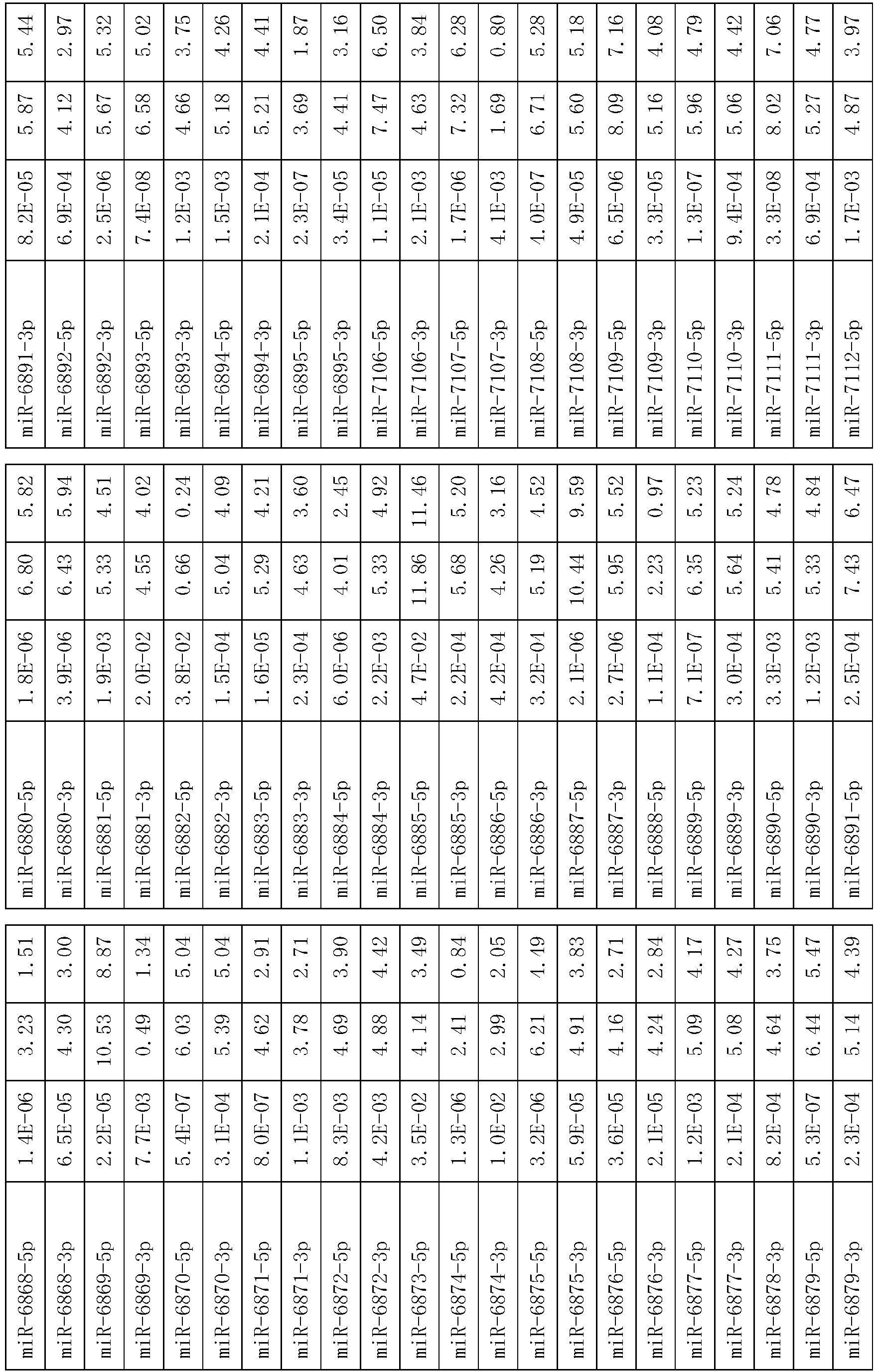

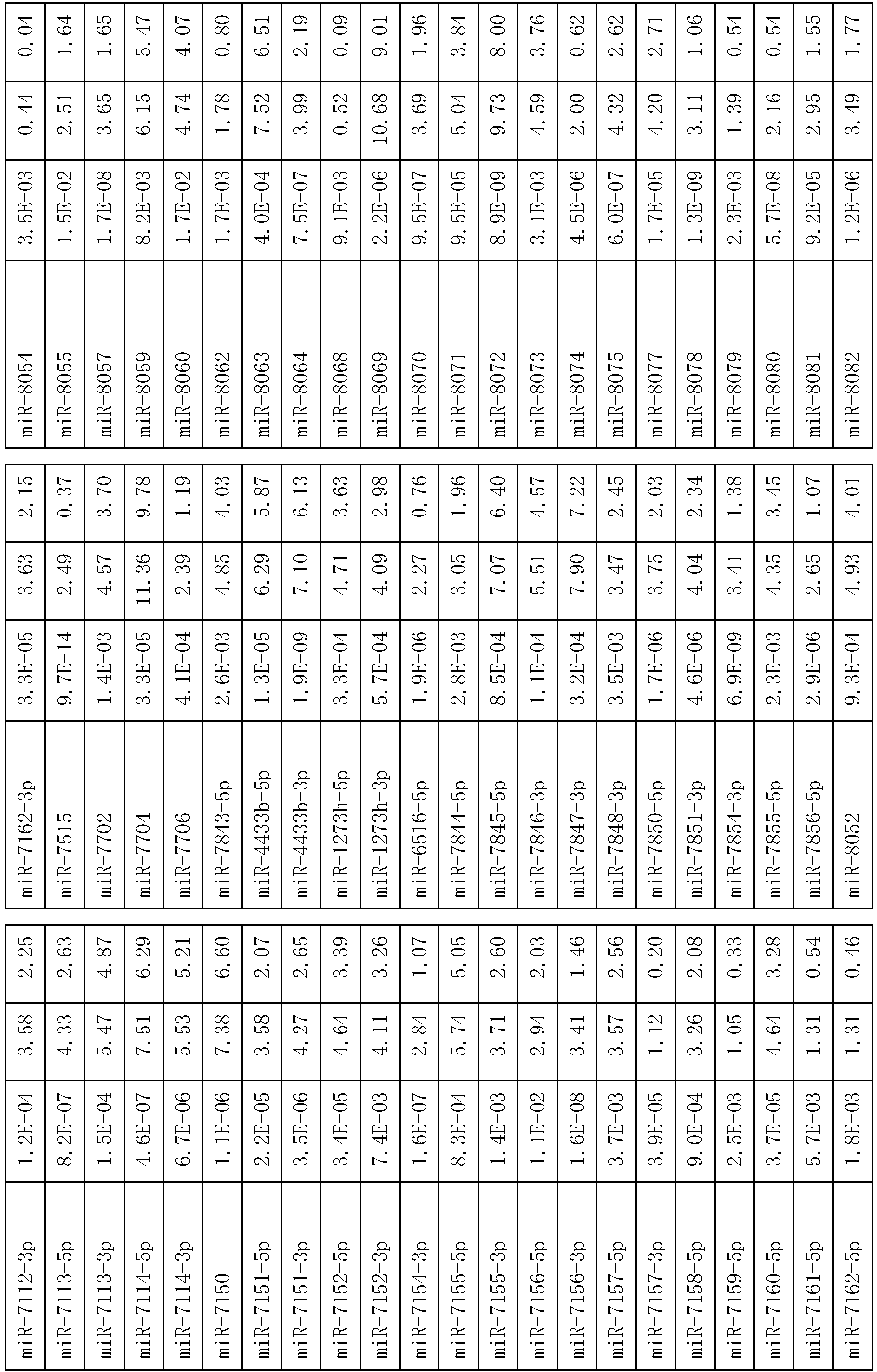

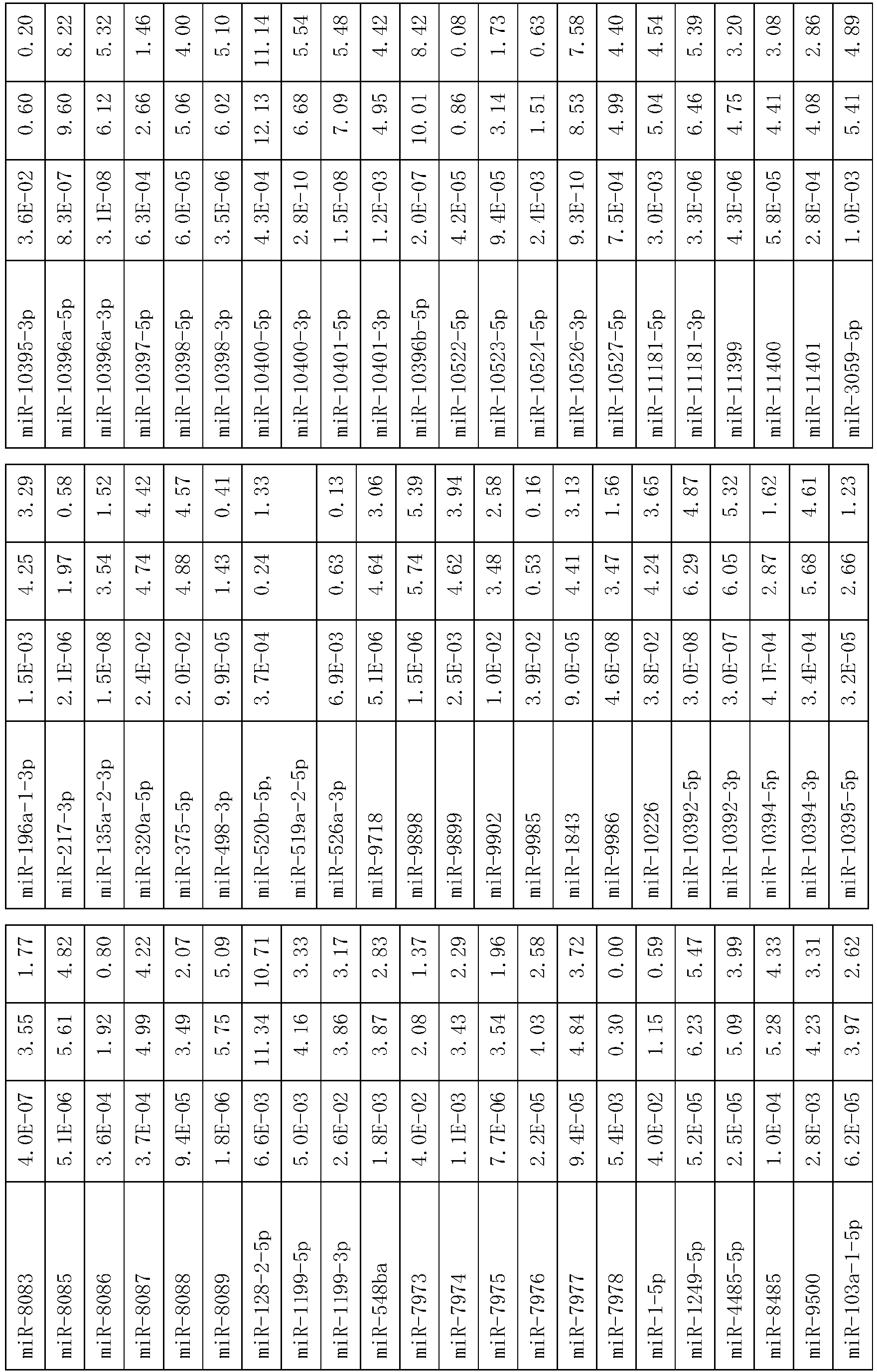

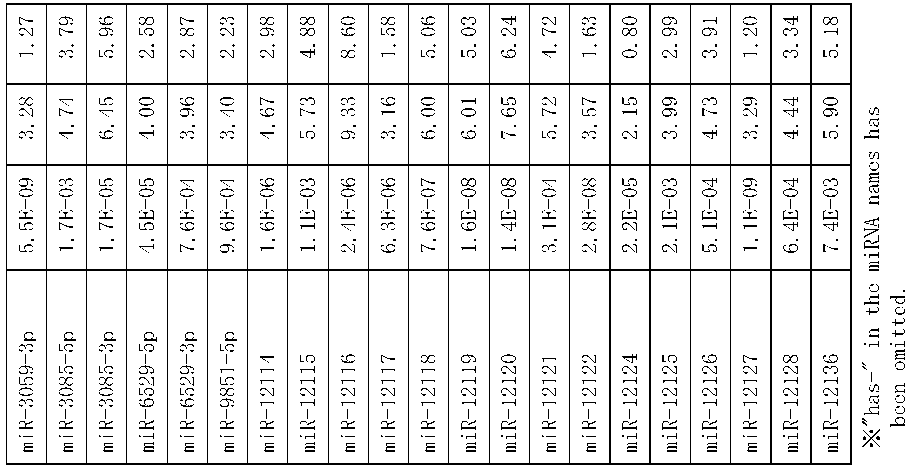

### [Table 5-1]

**Table 5-1: Prediction of Lung Cancer based on the Expression of Randomly Picked miRNA**

| trial | A | B | c | D | E | trial | A | B | C | D | E |
|---|---|---|---|---|---|---|---|---|---|---|---|
| 0 | 5 | 76. 3% | 76. 8% | 76. 0% | 76. 4% | 0 | 15 | 76. 3% | 76. 8% | 76. 0% | 76. 4% |
| 1 | | 69. 7% | 70. 7% | 69. 3% | 70. 0% | 1 | | 79. 3% | 78. 8% | 79. 6% | 79. 2% |
| 2 | | 66. 2% | 70. 7% | 64. 8% | 67. 6% | 2 | | 86. 9% | 87. 9% | 86. 1% | 87. 0% |
| 3 | | 69. 7% | 69. 7% | 69. 7% | 69. 7% | 3 | | 75. 3% | 73. 7% | 76. 0% | 74. 9% |
| 4 | | 71. 2% | 74. 7% | 69. 8% | 72. 2% | 4 | | 83. 3% | 85. 9% | 81. 7% | 83. 7% |
| 5 | | 69. 7% | 69. 7% | 69. 7% | 69. 7% | 5 | | 84. 8% | 86. 9% | 83. 5% | 85. 1% |
| 6 | | 66. 2% | 66. 7% | 66. 0% | 66. 3% | 6 | | 87. 4% | 87. 9% | 87. 0% | 87. 4% |
| 7 | | 66. 2% | 65. 7% | 66. 3% | 66. 0% | 7 | | 78. 8% | 79. 8% | 78. 2% | 79. 0% |
| 8 | | 72. 2% | 79. 8% | 69. 3% | 74. 2% | 8 | | 57. 6% | 66. 7% | 56. 4% | 61. 1% |
| 9 | | 72. 2% | 76. 8% | 70. 4% | 73. 4% | 9 | | 82. 3% | 85. 9% | 80. 2% | 82. 9% |
| 10 | | 80. 8% | 78. 8% | 82. 1% | 80. 4% | 10 | | 82. 8% | 81. 8% | 83. 5% | 82. 7% |
| 11 | | 64. 1% | 73. 7% | 61. 9% | 67. 3% | 11 | | 73. 2% | 78. 8% | 70. 9% | 74. 6% |
| 12 | | 72. 7% | 74. 7% | 71. 8% | 73. 3% | 12 | | 82. 8% | 86. 9% | 80. 4% | 83. 5% |
| 13 | | 75. 8% | 74. 7% | 76. 3% | 75. 5% | 13 | | 76. 3% | 78. 8% | 75. 0% | 76. 8% |
| 14 | | 78. 8% | 82. 8% | 76. 6% | 79. 6% | 14 | | 77. 8% | 75. 8% | 78. 9% | 77. 3% |
| 15 | | 71. 2% | 73. 7% | 70. 2% | 71. 9% | 15 | | 81. 3% | 83. 8% | 79. 8% | 81. 8% |
| 16 | | 43. 9% | 42. 4% | 43. 8% | 43. 1% | 16 | | 67. 7% | 72. 7% | 66. 1% | 69. 2% |
| 17 | | 31. 8% | 38. 4% | 33. 9% | 36. 0% | 17 | | 69. 7% | 69. 7% | 69. 7% | 69. 7% |
| 18 | | 58. 1% | 58. 6% | 58. 0% | 58. 3% | 18 | | 84. 3% | 86. 9% | 82. 7% | 84. 7% |
| 19 | | 71. 2% | 76. 8% | 69. 1% | 72. 7% | 19 | | 69. 7% | 69. 7% | 69. 7% | 69. 7% |
| Π | 10 | 76. 3% | 77, 8% | 75, 5% | 76, 6% | 0 | 20 | 75, 3% | 75, 8% | 75, 0% | 75, 4% |
| 1 | | 74. 7% | 77. 8% | 73. 3% | 75. 5% | 1 | | 85. 9% | 83. 8% | 87. 4% | 85. 6% |
| 2 | | 75. 8% | 73. 7% | 76. 8% | 75. 3% | 2 | | 75. 3% | 77. 8% | 74. 0% | 75. 9% |
| 3 | | 86. 4% | 85. 9% | 86. 7% | 86. 3% | 3 | | 79. 8% | 82. 8% | 78. 1% | 80. 4% |
| 4 | | 78. 8% | 75. 8% | 80. 6% | 78. 1% | 4 | | 86. 4% | 83. 8% | 88. 3% | 86. 0% |
| 5 | | 74. 7% | 78. 8% | 72. 9% | 75. 7% | 5 | | 84. 3% | 86. 9% | 82. 7% | 84. 7% |
| 6 | | 74. 7% | 74. 7% | 74. 7% | 74. 7% | 6 | | 74. 2% | 72. 7% | 75. 0% | 73. 8% |
| 7 | | 73. 2% | 75. 8% | 72. 1% | 73. 9% | 7 | | 80. 3% | 84. 8% | 77. 8% | 81. 2% |
| 8 | | 82. 3% | 77. 8% | 85. 6% | 81. 5% | 8 | | 81. 8% | 81. 8% | 81. 8% | 81. 8% |
| 9 | | 85. 9% | 92. 9% | 81. 4% | 86. 8% | 9 | | 77. 3% | 81. 8% | 75. 0% | 78.3% |
| 10 | | 70. 7% | 71. 7% | 70. 3% | 71. 0% | 10 | | 80. 8% | 78. 8% | 82. 1% | 80. 4% |
| 11 | | 59. 6% | 65. 7% | 58. 6% | 61. 9% | 11 | | 80. 3% | 78. 8% | 81. 3% | 80. 0% |
| 12 | | 83. 3% | 82. 8% | 83. 7% | 83. 2% | 12 | | 84. 8% | 84. 8% | 84. 8% | 84. 8% |
| 13 | | 80. 3% | 81. 8% | 79. 4% | 80. 6% | 13 | | 80. 3% | 80. 8% | 80. 0% | 80. 4% |
| 14 | | 75. 3% | 78. 8% | 73. 6% | 76. 1% | 14 | | 80. 3% | 81. 8% | 79. 4% | 80. 6% |
| 15 | | 72. 7% | 78. 8% | 70. 3% | 74. 3% | 15 | | 76. 3% | 77. 8% | 75. 5% | 76. 6% |
| 16 | | 81. 3% | 81. 8% | 81. 0% | 81. 4% | 16 | | 83. 8% | 84. 8% | 83. 2% | 84. 0% |
| 17 | | 76. 3% | 77. 8% | 75. 5% | 76. 6% | 17 | | 82. 8% | 83. 8% | 82. 2% | 83. 0% |
| 18 | | 86. 9% | 88. 9% | 85. 4% | 87. 1% | 18 | | 82. 3% | 83. 8% | 81. 4% | 82. 6% |
| 19 | | 71. 7% | 72. 7% | 71. 3% | 72. 0% | 19 | | 81. 3% | 83. 8% | 79. 8% | 81. 8% |

| | | | | | | | | | | | |
|---|---|---|---|---|---|---|---|---|---|---|---|
| ^{∗}A represents the number of miRNAs used for prediction, B represents Accuracy, C represents Recall, D represents Precision, and E represents f1. | | | | | | | | | | | |

### [Table 5-2]

**Table 5-2: Summary of Prediction of Lung Cancer based on the Expression of Randomly Picked miRNA**

| | A | B | C | D | E |
|---|---|---|---|---|---|
| Average | 5 | 67. 4% | 69. 8% | 66. 8% | 68. 2% |
| Standard Deviation | | 11. 5% | 11. 5% | 11. 0% | 11. 1% |
| Minimum Value | | 31. 8% | 38. 4% | 33. 9% | 36. 0% |
| Third Quartile | | 66. 2% | 68. 9% | 65. 7% | 67. 0% |
| Average | | 70. 5% | 73. 7% | 69. 5% | 71. 0% |
| First Quartile | | 72. 3% | 76. 8% | 70. 7% | 73. 6% |
| Maximum Value | | 80. 8% | 82. 8% | 82. 1% | 80. 4% |
| Average | 10 | 77. 0% | 78. 6% | 76. 4% | 77. 4% |
| Standard Deviation | | 6. 4% | 6. 1% | 6. 8% | 6. 0% |
| Minimum Value | | 59. 6% | 65. 7% | 58. 6% | 61. 9% |
| Third Ouartile | | 74. 4% | 75. 5% | 72. 7% | 74. 6% |
| Average | | 76. 0% | 77. 8% | 75. 5% | 76. 4% |
| First Ouartile | | 81. 6% | 81. 8% | 81. 1% | 81. 4% |
| Maximum Value | | 86. 9% | 92. 9% | 86. 7% | 87. 1% |
| Average | 15 | 77. 9% | 79. 7% | 77. 1% | 78. 3% |
| Standard Deviation | | 7. 5% | 6. 8% | 7. 5% | 6. 9% |
| Minimum Value | | 57. 6% | 66. 7% | 56. 4% | 61. 1% |
| Third Ouartile | | 74. 7% | 75. 3% | 74. 0% | 74. 8% |
| Average | | 79. 0% | 79. 3% | 79. 3% | 79. 1% |
| First Ouartile | | 83. 0% | 86. 1% | 82. 0% | 83. 6% |
| Maximum Value | | 87. 4% | 87. 9% | 87. 0% | 87. 4% |
| Average | 20 | 80. 7% | 81. 6% | 80. 2% | 80. 9% |
| Standard Deviation | | 3. 6% | 3. 6% | 4. 1% | 3. 5% |
| Minimum Value | | 74. 2% | 72. 7% | 74. 0% | 73. 8% |
| Third Ouartile | | 79. 2% | 78. 8% | 77. 2% | 79. 6% |
| Average | | 80. 6% | 82. 3% | 80. 6% | 80. 9% |
| First Ouartile | | 83. 1% | 83. 8% | 82. 3% | 83. 3% |
| Maximum Value | | 86. 4% | 86. 9% | 88. 3% | 86. 0% |

| | | | | | |
|---|---|---|---|---|---|
| ^{∗}A represents the number of miRNAs used for prediction, B represents Accuracy, C represents Recall, D represents Precision, and E represents f1. | | | | | |

### [Table 6-1]

**Table 6-1: Summary of Prediction of Breast Cancer based on the Expression of Randomly Picked miRNA**

| Trial | A | B | C | D | E | Trial | A | B | C | D | E |
|---|---|---|---|---|---|---|---|---|---|---|---|
| 0 | 5 | 64% | 70% | 62% | 0. 624 | 0 | 15 | 71% | 73% | 70% | 0. 745 |
| 1 | | 63% | 77% | 58% | 0. 774 | 1 | | 72% | 73% | 72% | 0. 724 |
| 2 | | 68% | 63% | 70% | 0. 688 | 2 | | 73% | 63% | 77% | 0. 712 |
| 3 | | 73% | 70% | 74% | 0. 731 | 3 | | 73% | 60% | 78% | 0. 723 |
| 4 | | 64% | 80% | 58% | 0. 720 | 4 | | 80% | 73% | 82% | 0. 802 |
| 5 | | 61% | 73% | 55% | 0. 617 | 5 | | 65% | 60% | 68% | 0. 672 |
| 6 | | 71% | 83% | 66% | 0. 734 | 6 | | 76% | 67% | 80% | 0. 812 |
| 7 | | 64% | 73% | 61% | 0. 633 | 7 | | 68% | 67% | 69% | 0. 722 |
| 8 | | 79% | 67% | 84% | 0. 628 | 8 | | 68% | 70% | 68% | 0. 786 |
| 9 | | 63% | 50% | 68% | 0. 597 | 9 | | 71% | 77% | 69% | 0. 763 |
| 10 | | 67% | 63% | 69% | 0. 705 | 10 | | 64% | 70% | 62% | 0. 655 |
| 11 | | 56% | 60% | 54% | 0. 612 | 11 | | 62% | 70% | 58% | 0. 569 |
| 12 | | 74% | 70% | 76% | 0. 755 | 12 | | 67% | 80% | 62% | 0. 714 |
| 13 | | 69% | 70% | 69% | 0. 766 | 13 | | 63% | 63% | 64% | 0. 718 |
| 14 | | 57% | 67% | 53% | 0. 535 | 14 | | 64% | 77% | 59% | 0. 662 |
| 15 | | 58% | 63% | 55% | 0. 658 | 15 | | 73% | 73% | 73% | 0. 787 |
| 16 | | 74% | 70% | 76% | 0. 790 | 16 | | 67% | 73% | 65% | 0. 707 |
| 17 | | 70% | 80% | 66% | 0. 785 | 17 | | 63% | 77% | 58% | 0. 708 |
| 18 | | 69% | 77% | 66% | 0. 712 | 18 | | 65% | 70% | 64% | 0. 693 |
| 19 | | 53% | 60% | 50% | 0. 646 | 19 | | 70% | 70% | 70% | 0. 664 |
| 0 | 10 | 63% | 70% | 59% | 0. 663 | 0 | 20 | 56% | 67% | 51% | 0. 599 |
| 1 | | 57% | 67% | 53% | 0. 587 | 1 | | 73% | 73% | 73% | 0. 730 |
| 2 | | 72% | 73% | 72% | 0. 781 | 2 | | 81% | 73% | 84% | 0. 756 |
| 3 | | 62% | 70% | 58% | 0. 701 | 3 | | 60% | 57% | 61% | 0. 683 |
| 4 | | 61% | 67% | 58% | 0. 644 | 4 | | 67% | 70% | 66% | 0. 664 |
| 5 | | 68% | 70% | 68% | 0. 738 | 5 | | 65% | 67% | 65% | 0. 680 |
| 6 | | 63% | 67% | 62% | 0. 664 | 6 | | 63% | 57% | 65% | 0. 595 |
| 7 | | 77% | 80% | 76% | 0. 820 | 7 | | 72% | 63% | 76% | 0. 680 |
| 8 | | 57% | 63% | 54% | 0. 599 | 8 | | 76% | 67% | 80% | 0. 743 |
| 9 | | 56% | 63% | 53% | 0. 529 | 9 | | 63% | 70% | 61% | 0. 632 |
| 10 | | 70% | 77% | 68% | 0. 715 | 10 | | 63% | 63% | 62% | 0. 653 |
| 11 | | 66% | 83% | 59% | 0. 760 | 11 | | 66% | 67% | 66% | 0. 675 |
| 12 | | 71% | 77% | 69% | 0. 782 | 12 | | 68% | 70% | 68% | 0. 709 |
| 13 | | 66% | 63% | 68% | 0. 745 | 13 | | 75% | 67% | 78% | 0. 747 |
| 14 | | 73% | 73% | 73% | 0. 763 | 14 | | 71% | 73% | 70% | 0. 725 |
| 15 | | 70% | 67% | 72% | 0. 679 | 15 | | 64% | 70% | 62% | 0. 648 |
| 16 | | 80% | 70% | 84% | 0. 816 | 16 | | 68% | 63% | 70% | 0. 713 |
| 17 | | 60% | 67% | 57% | 0. 697 | 17 | | 72% | 63% | 76% | 0. 722 |
| 18 | | 64% | 67% | 64% | 0. 632 | 18 | | 69% | 60% | 73% | 0. 664 |
| 19 | | 63% | 73% | 58% | 0. 633 | 19 | | 70% | 67% | 72% | 0. 656 |

| | | | | | | | | | | | |
|---|---|---|---|---|---|---|---|---|---|---|---|
| ^{∗}A represents the number of miRNAs used for prediction, B represents Accuracy, C represents Sensitivity, D represents Specificity, and E represents AUC. | | | | | | | | | | | |

### [Table 6-2]

**Table 6-2: Summary of Prediction of Breast Cancer based on the Expression of Randomly Picked miRNA**

| | A | B | C | D | E |
|---|---|---|---|---|---|
| Average | 5 | 66% | 69% | 65% | 0. 686 |
| Standard | | 7% | 8% | 9% | 0. 072 |
| Minimum Value | | 53% | 50% | 50% | 0. 535 |
| Third Ouartile | | 62% | 63% | 57% | 0. 627 |
| Average | | 66% | 70% | 66% | 0. 697 |
| First Ouartile | | 70% | 74% | 69% | 0. 739 |
| Maximum Value | | 79% | 83% | 84% | 0. 790 |
| Average | 10 | 66% | 70% | 64% | 0. 697 |
| Standard | | 7% | 6% | 8% | 0. 079 |
| Minimum Value | | 56% | 63% | 53% | 0. 529 |
| Third Ouartile | | 61% | 67% | 58% | 0. 641 |
| Average | | 65% | 70% | 63% | 0. 699 |
| First Ouartile | | 70% | 73% | 70% | 0. 761 |
| Maximum Value | | 80% | 83% | 84% | 0. 820 |
| Average | 15 | 69% | 70% | 68% | 0. 717 |
| Standard | | 5% | 6% | 7% | 0. 058 |
| Minimum Value | | 62% | 60% | 58% | 0. 569 |
| Third Ouartile | | 65% | 67% | 63% | 0. 688 |
| Average | | 68% | 70% | 68% | 0. 716 |
| First Ouartile | | 72% | 73% | 72% | 0. 749 |
| Maximum Value | | 80% | 80% | 82% | 0. 812 |
| Average | 20 | 68% | 66% | 69% | 0. 684 |
| Standard | | 6% | 5% | 8% | 0. 047 |
| Minimum Value | | 56% | 57% | 51% | 0. 595 |
| Third Ouartile | | 64% | 63% | 64% | 0. 655 |
| Average | | 68% | 67% | 69% | 0. 680 |
| First Ouartile | | 72% | 70% | 74% | 0. 723 |
| Maximum Value | | 81% | 73% | 84% | 0. 756 |

| | | | | | |
|---|---|---|---|---|---|
| ^{∗}A represents the number of miRNAs used for prediction, B represents Accuracy, C represents Sensitivity, D represents Specificity, and E represents AUC. | | | | | |

### [Table 7-1]

**Table 7-1: Prediction of Kidney Cancer based on the Expression of Randomly Picked miRNA**

| Trial | A | B | C | D | E | Trial | A | B | C | D | E |
|---|---|---|---|---|---|---|---|---|---|---|---|
| 0 | 5 | 86% | 87% | 85% | 0.903 | 0 | 15 | 80% | 90% | 76% | 0. 855 |
| 1 | | 60% | 80% | 51% | 0. 626 | 1 | | 83% | 80% | 84% | 0. 879 |
| 2 | | 67% | 67% | 68% | 0. 765 | 2 | | 83% | 73% | 86% | 0. 857 |
| 3 | | 72% | 63% | 76% | 0.803 | 3 | | 75% | 80% | 73% | 0. 854 |
| 4 | | 80% | 60% | 88% | 0. 739 | 4 | | 86% | 83% | 86% | 0. 894 |
| 5 | | 78% | 83% | 76% | 0. 814 | 5 | | 78% | 73% | 80% | 0. 826 |
| 6 | | 72% | 70% | 73% | 0. 779 | 6 | | 70% | 80% | 66% | 0. 766 |
| 7 | | 75% | 80% | 73% | 0. 806 | 7 | | 77% | 73% | 78% | 0. 834 |
| 8 | | 69% | 67% | 70% | 0. 800 | 8 | | 69% | 77% | 66% | 0. 761 |
| 9 | | 71% | 77% | 69% | 0. 756 | 9 | | 82% | 70% | 86% | 0. 838 |
| 10 | | 61% | 73% | 55% | 0. 740 | 10 | | 80% | 80% | 80% | 0. 835 |
| 11 | | 66% | 57% | 70% | 0. 736 | 11 | | 79% | 73% | 81% | 0. 822 |
| 12 | | 76% | 73% | 77% | 0. 831 | 12 | | 79% | 83% | 77% | 0. 822 |
| 13 | | 74% | 77% | 73% | 0. 818 | 13 | | 65% | 77% | 61% | 0. 770 |
| 14 | | 71% | 67% | 73% | 0. 805 | 14 | | 70% | 67% | 72% | 0. 754 |
| 15 | | 64% | 77% | 59% | 0. 655 | 15 | | 81% | 83% | 80% | 0. 855 |
| 16 | | 72% | 73% | 72% | 0. 786 | 16 | | 80% | 77% | 81% | 0. 794 |
| 17 | | 67% | 70% | 66% | 0. 772 | 17 | | 81% | 77% | 82% | 0. 860 |
| 18 | | 79% | 77% | 80% | 0. 796 | 18 | | 79% | 80% | 78% | 0. 857 |
| 19 | | 79% | 80% | 78% | 0. 877 | 19 | | 60% | 63% | 58% | 0. 637 |
| 0 | 10 | 74% | 73% | 74% | 0, 808 | 0 | 20 | 80% | 73% | 89% | 0. 838 |
| 1 | | 75% | 77% | 74% | 0. 877 | 1 | | 75% | 70% | 77% | 0. 701 |
| 2 | | 69% | 63% | 72% | 0. 742 | 2 | | 75% | 67% | 78% | 0. 797 |
| 3 | | 61% | 73% | 55% | 0. 641 | 3 | | 73% | 73% | 73% | 0. 786 |
| 4 | | 85% | 83% | 85% | 0. 814 | 4 | | 83% | 83% | 82% | 0. 876 |
| 5 | | 70% | 63% | 73% | 0. 730 | 5 | | 84% | 77% | 86% | 0. 850 |
| 6 | | 79% | 80% | 78% | 0. 829 | 6 | | 67% | 60% | 70% | 0. 718 |
| 7 | | 82% | 73% | 85% | 0. 855 | 7 | | 72% | 67% | 74% | 0. 804 |
| 8 | | 72% | 73% | 72% | 0. 762 | 8 | | 69% | 67% | 70% | 0. 786 |
| 9 | | 88% | 87% | 88% | 0. 888 | 9 | | 61% | 67% | 58% | 0. 619 |
| 10 | | 68% | 70% | 68% | 0. 818 | 10 | | 71% | 63% | 74% | 0. 793 |
| 11 | | 87% | 90% | 85% | 0. 938 | 11 | | 81% | 83% | 80% | 0. 894 |
| 12 | | 63% | 80% | 55% | 0. 740 | 12 | | 67% | 77% | 64% | 0. 772 |
| 13 | | 85% | 83% | 85% | 0. 875 | 13 | | 62% | 60% | 62% | 0. 755 |
| 14 | | 67% | 63% | 69% | 0. 728 | 14 | | 75% | 67% | 78% | 0. 820 |
| 15 | | 67% | 77% | 64% | 0. 673 | 15 | | 60% | 60% | 59% | 0. 656 |
| 16 | | 86% | 83% | 86% | 0. 903 | 16 | | 85% | 80% | 86% | 0. 908 |
| 17 | | 68% | 77% | 65% | 0. 764 | 17 | | 68% | 70% | 68% | 0. 716 |
| 18 | | 80% | 80% | 80% | 0. 877 | 18 | | 58% | 60% | 57% | 0. 676 |
| 19 | | 82% | 77% | 84% | 0. 866 | 19 | | 64% | 60% | 66% | 0. 705 |

| | | | | | | | | | | | |
|---|---|---|---|---|---|---|---|---|---|---|---|
| ^{∗}A represents the number of miRNAs used for prediction, B represents Accuracy, C represents Sensitivity, D represents Specificity, and E represents AUC. | | | | | | | | | | | |

### [Table 7-2]

**Table 7-2: Summary of Prediction of Kidney Cancer based on the Expression of Randomly Picked miRNA**

| | A | B | C | D | E |
|---|---|---|---|---|---|
| Average | 5 | 72% | 73% | 72% | 0. 780 |
| Standard | | 7% | 8% | 9% | 0. 064 |
| Minimum Value | | 60% | 57% | 51% | 0. 626 |
| Third Ouartile | | 67% | 67% | 69% | 0. 752 |
| Average | | 72% | 73% | 73% | 0. 791 |
| First Ouartile | | 76% | 78% | 76% | 0. 808 |
| Maximum Value | | 86% | 87% | 88% | 0. 903 |
| Average | 10 | 75% | 76% | 75% | 0. 806 |
| Standard | | 8% | 7% | 10% | 0. 081 |
| Minimum Value | | 61% | 63% | 55% | 0. 641 |
| Third Ouartile | | 68% | 73% | 69% | 0. 741 |
| Average | | 75% | 77% | 74% | 0. 816 |
| First Ouartile | | 82% | 81% | 85% | 0. 876 |
| Maximum Value | | 88% | 90% | 88% | 0. 938 |
| Average | 15 | 77% | 77% | 77% | 0. 818 |
| Standard | | 7% | 6% | 8% | 0. 058 |
| Minimum Value | | 60% | 63% | 58% | 0. 637 |
| Third Ouartile | | 74% | 73% | 73% | 0. 788 |
| Average | | 79% | 77% | 79% | 0. 834 |
| First Ouartile | | 81% | 80% | 81% | 0. 856 |
| Maximum Value | | 86% | 90% | 86% | 0. 894 |
| Average | 20 | 71% | 69% | 72% | 0. 774 |
| Standard | | 8% | 8% | 9% | 0. 080 |
| Minimum Value | | 58% | 60% | 57% | 0. 619 |
| Third Ouartile | | 67% | 63% | 66% | 0. 713 |
| Average | | 72% | 67% | 74% | 0. 786 |
| First Ouartile | | 76% | 74% | 79% | 0. 825 |
| Maximum Value | | 85% | 83% | 86% | 0. 908 |

| | | | | | |
|---|---|---|---|---|---|
| ^{∗}A represents the number of miRNAs used for prediction, B represents Accuracy, C represents Sensitivity, D represents Specificity, and E represents AUC. | | | | | |

### [Table 8-1]

**Table 8-1: Prediction of Leukemia based on the Expression of Randomly Picked miRNA**

| Trial | A | B | C | D | E | Trial | A | B | C | D | E |
|---|---|---|---|---|---|---|---|---|---|---|---|
| 0 | 5 | 60% | 73% | 54% | 0. 635 | 0 | 15 | 65% | 67% | 65% | 0. 637 |
| 1 | | 53% | 63% | 49% | 0. 639 | 1 | | 68% | 67% | 69% | 0. 780 |
| 2 | | 56% | 73% | 49% | 0. 572 | 2 | | 68% | 77% | 65% | 0. 759 |
| 3 | | 64% | 80% | 58% | 0. 691 | 3 | | 75% | 67% | 78% | 0. 710 |
| 4 | | 50% | 57% | 47% | 0. 606 | 4 | | 55% | 50% | 57% | 0. 547 |
| 5 | | 60% | 63% | 58% | 0. 695 | 5 | | 50% | 53% | 49% | 0. 540 |
| 6 | | 61% | 77% | 54% | 0. 565 | 6 | | 70% | 63% | 73% | 0. 719 |
| 7 | | 63% | 77% | 57% | 0. 772 | 7 | | 68% | 70% | 68% | 0. 769 |
| 8 | | 71% | 63% | 74% | 0. 829 | 8 | | 73% | 63% | 77% | 0. 745 |
| 9 | | 52% | 53% | 51% | 0. 494 | 9 | | 73% | 70% | 74% | 0. 763 |
| 10 | | 67% | 57% | 72% | 0. 681 | 10 | | 63% | 70% | 61% | 0. 702 |
| 11 | | 57% | 67% | 53% | 0. 587 | 11 | | 50% | 73% | 41% | 0. 591 |
| 12 | | 57% | 70% | 51% | 0. 554 | 12 | | 61% | 60% | 61% | 0.602 |
| 13 | | 64% | 70% | 62% | 0. 703 | 13 | | 70% | 67% | 72% | 0. 714 |
| 14 | | 57% | 73% | 50% | 0. 597 | 14 | | 66% | 60% | 69% | 0. 706 |
| 15 | | 58% | 70% | 53% | 0. 520 | 15 | | 47% | 57% | 43% | 0. 524 |
| 16 | | 70% | 63% | 73% | 0. 763 | 16 | | 55% | 57% | 54% | 0. 593 |
| 17 | | 49% | 67% | 42% | 0. 624 | 17 | | 68% | 63% | 70% | 0. 686 |
| 18 | | 62% | 57% | 64% | 0. 600 | 18 | | 64% | 60% | 66% | 0. 659 |
| 19 | | 66% | 77% | 62% | 0. 703 | 19 | | 57% | 77% | 49% | 0. 651 |
| 0 | 10 | 69% | R7% | 70% | 0, 754 | 0 | 20 | 80% | 70% | 84% | 0, 827 |
| 1 | | 76% | 67% | 80% | 0. 804 | 1 | | 69% | 63% | 72% | 0. 689 |
| 2 | | 57% | 50% | 59% | 0. 591 | 2 | | 50% | 63% | 45% | 0. 587 |
| 3 | | 70% | 77% | 68% | 0. 763 | 3 | | 76% | 73% | 77% | 0. 815 |
| 4 | | 64% | 60% | 66% | 0. 661 | 4 | | 80% | 73% | 82% | 0. 873 |
| 5 | | 53% | 60% | 50% | 0. 589 | 5 | | 66% | 53% | 72% | 0. 633 |
| 6 | | 71% | 67% | 73% | 0. 762 | 6 | | 61% | 63% | 59% | 0. 656 |
| 7 | | 72% | 77% | 70% | 0. 823 | 7 | | 64% | 73% | 61% | 0. 676 |
| 8 | | 81% | 80% | 81% | 0. 822 | 8 | | 44% | 60% | 38% | 0. 522 |
| 9 | | 44% | 60% | 38% | 0. 459 | 9 | | 71% | 67% | 73% | 0. 756 |
| 10 | | 70% | 73% | 69% | 0. 795 | 10 | | 54% | 67% | 49% | 0. 608 |
| 11 | | 67% | 70% | 66% | 0. 710 | 11 | | 61% | 60% | 61% | 0. 671 |
| 12 | | 47% | 73% | 36% | 0. 515 | 12 | | 70% | 73% | 69% | 0. 702 |
| 13 | | 63% | 57% | 66% | 0. 639 | 13 | | 56% | 57% | 55% | 0. 551 |
| 14 | | 71% | 60% | 76% | 0. 805 | 14 | | 51% | 67% | 45% | 0. 609 |
| 15 | | 49% | 63% | 43% | 0. 518 | 15 | | 74% | 63% | 78% | 0. 721 |
| 16 | | 75% | 70% | 77% | 0. 781 | 16 | | 65% | 67% | 65% | 0. 671 |
| 17 | | 49% | 60% | 45% | 0. 528 | 17 | | 86% | 83% | 86% | 0. 915 |
| 18 | | 47% | 57% | 43% | 0. 521 | 18 | | 70% | 70% | 70% | 0. 703 |
| 19 | | 50% | 67% | 43% | 0. 549 | 19 | | 74% | 73% | 74% | 0. 809 |

| | | | | | | | | | | | |
|---|---|---|---|---|---|---|---|---|---|---|---|
| ^{∗}A represents the number of miRNAs used for prediction, B represents Accuracy, C represents Sensitivity, D represents Specificity, and E represents AUC. | | | | | | | | | | | |

### [Table 8-2]

**Table 8-2: Summary of Prediction of Leukemia based on the Expression of Randomly Picked miRNA**

| | A | B | C | D | E |
|---|---|---|---|---|---|
| Average | 5 | 60% | 67% | 57% | 0. 642 |
| Standard | | 6% | 8% | 9% | 0. 087 |
| Minimum Value | | 49% | 53% | 42% | 0. 494 |
| Third Ouartile | | 56% | 63% | 51% | 0. 583 |
| Average | | 60% | 68% | 54% | 0. 630 |
| First Ouartile | | 64% | 73% | 62% | 0. 697 |
| Maximum Value | | 71% | 80% | 74% | 0. 829 |
| Average | 10 | 62% | 66% | 61% | 0. 669 |
| Standard | | 12% | 8% | 15% | 0. 125 |
| Minimum Value | | 44% | 50% | 36% | 0. 459 |
| Third Ouartile | | 50% | 60% | 44% | 0. 543 |
| Average | | 66% | 67% | 66% | 0. 686 |
| First Ouartile | | 71% | 71% | 71% | 0. 784 |
| Maximum Value | | 81% | 80% | 81% | 0. 823 |
| Average | 15 | 63% | 65% | 63% | 0. 670 |
| Standard | | 8% | 7% | 11% | 0. 081 |
| Minimum Value | | 47% | 50% | 41% | 0. 524 |
| Third Ouartile | | 56% | 60% | 56% | 0. 600 |
| Average | | 66% | 65% | 66% | 0. 694 |
| First Ouartile | | 69% | 70% | 71% | 0. 726 |
| Maximum Value | | 75% | 77% | 78% | 0. 780 |
| Average | 20 | 66% | 67% | 66% | 0. 700 |
| Standard | | 11% | 7% | 14% | 0. 106 |
| Minimum Value | | 44% | 53% | 38% | 0. 522 |
| Third Ouartile | | 59% | 63% | 58% | 0. 627 |
| Average | | 68% | 67% | 70% | 0. 682 |
| First Ouartile | | 74% | 73% | 75% | 0. 769 |
| Maximum Value | | 86% | 83% | 86% | 0. 915 |

| | | | | | |
|---|---|---|---|---|---|
| ^{∗}A represents the number of miRNAs used for prediction, B represents Accuracy, C represents Sensitivity, D represents Specificity, and E represents AUC. | | | | | |

### [Table 9-1]

**Table 9-1: Prediction of Lymphoma based on the Expression of Randomly Picked miRNA**

| Trial | A | B | C | D | E | Trial | A | B | C | D | E |
|---|---|---|---|---|---|---|---|---|---|---|---|
| 0 | 5 | 69% | 73% | 68% | 0. 754 | 0 | 15 | 67% | 67% | 68% | 0. 754 |
| 1 | | 82% | 57% | 92% | 0. 823 | 1 | | 70% | 70% | 70% | 0. 767 |
| 2 | | 79% | 80% | 78% | 0. 897 | 2 | | 67% | 73% | 65% | 0. 769 |
| 3 | | 69% | 87% | 62% | 0. 815 | 3 | | 80% | 80% | 80% | 0. 868 |
| 4 | | 78% | 73% | 80% | 0. 855 | 4 | | 69% | 80% | 65% | 0. 788 |
| 5 | | 77% | 77% | 77% | 0. 841 | 5 | | 87% | 83% | 88% | 0. 910 |
| 6 | | 76% | 90% | 70% | 0. 833 | 6 | | 82% | 80% | 82% | 0. 886 |
| 7 | | 84% | 83% | 84% | 0. 889 | 7 | | 75% | 80% | 73% | 0. 786 |
| 8 | | 63% | 73% | 58% | 0. 728 | 8 | | 86% | 80% | 88% | 0. 895 |
| 9 | | 74% | 80% | 72% | 0. 844 | 9 | | 80% | 80% | 80% | 0. 838 |
| 10 | | 76% | 77% | 76% | 0. 862 | 10 | | 69% | 73% | 68% | 0. 778 |
| 11 | | 75% | 83% | 72% | 0. 769 | 11 | | 67% | 90% | 58% | 0. 816 |
| 12 | | 80% | 77% | 81% | 0. 895 | 12 | | 76% | 77% | 76% | 0. 843 |
| 13 | | 82% | 80% | 82% | 0. 881 | 13 | | 75% | 80% | 73% | 0. 841 |
| 14 | | 60% | 80% | 51% | 0. 716 | 14 | | 64% | 70% | 62% | 0. 779 |
| 15 | | 60% | 80% | 51% | 0. 745 | 15 | | 71% | 80% | 68% | 0. 731 |
| 16 | | 68% | 80% | 64% | 0. 774 | 16 | | 63% | 73% | 59% | 0. 762 |
| 17 | | 70% | 83% | 65% | 0. 789 | 17 | | 77% | 67% | 81% | 0. 848 |
| 18 | | 61% | 83% | 51% | 0. 659 | 18 | | 82% | 87% | 80% | 0. 905 |
| 19 | | 73% | 87% | 68% | 0. 848 | 19 | | 57% | 70% | 51% | 0. 681 |
| 0 | 10 | 78% | 77% | 78% | 0. 844 | 0 | 20 | 70% | 73% | 69% | 0. 791 |
| 1 | | 65% | 70% | 64% | 0. 777 | 1 | | 70% | 67% | 72% | 0. 768 |
| 2 | | 60% | 67% | 57% | 0. 701 | 2 | | 72% | 67% | 74% | 0. 764 |
| 3 | | 81% | 83% | 80% | 0. 864 | 3 | | 71% | 80% | 68% | 0. 832 |
| 4 | | 84% | 87% | 82% | 0. 892 | 4 | | 79% | 80% | 78% | 0. 858 |
| 5 | | 87% | 87% | 86% | 0. 939 | 5 | | 71% | 73% | 70% | 0. 780 |
| 6 | | 79% | 73% | 81% | 0. 830 | 6 | | 74% | 80% | 72% | 0. 842 |
| 7 | | 67% | 83% | 61% | 0. 785 | 7 | | 56% | 73% | 49% | 0. 650 |
| 8 | | 71% | 80% | 68% | 0. 814 | 8 | | 72% | 63% | 76% | 0. 768 |
| 9 | | 75% | 80% | 73% | 0. 785 | 9 | | 72% | 70% | 73% | 0. 831 |
| 10 | | 73% | 83% | 69% | 0. 761 | 10 | | 81% | 87% | 78% | 0. 879 |
| 11 | | 83% | 83% | 82% | 0. 895 | 11 | | 72% | 67% | 74% | 0. 727 |
| 12 | | 80% | 77% | 81% | 0. 828 | 12 | | 62% | 67% | 59% | 0. 659 |
| 13 | | 66% | 70% | 65% | 0. 794 | 13 | | 74% | 80% | 72% | 0. 736 |
| 14 | | 71% | 73% | 70% | 0. 813 | 14 | | 87% | 87% | 86% | 0. 895 |
| 15 | | 59% | 63% | 57% | 0. 706 | 15 | | 65% | 80% | 59% | 0. 745 |
| 16 | | 83% | 83% | 82% | 0. 888 | 16 | | 73% | 77% | 72% | 0. 814 |
| 17 | | 82% | 87% | 80% | 0. 888 | 17 | | 86% | 87% | 85% | 0. 910 |
| 18 | | 74% | 87% | 69% | 0. 830 | 18 | | 81% | 70% | 85% | 0. 825 |
| 19 | | 69% | 73% | 68% | 0. 774 | 19 | | 75% | 73% | 76% | 0. 847 |

| | | | | | | | | | | | |
|---|---|---|---|---|---|---|---|---|---|---|---|
| ^{∗}A represents the number of miRNAs used for prediction, B represents Accuracy, C represents Sensitivity, D represents Specificity, and E represents AUC. | | | | | | | | | | | |

### [Table 9-2]

**Table 9-2: Summary of Prediction of Lymphoma based on the Expression of Randomly Picked miRNA**

| | A | B | C | D | E |
|---|---|---|---|---|---|
| Average | 5 | 73% | 79% | 70% | 0. 811 |
| Standard | | 8% | 7% | 12% | 0. 067 |
| Minimum Value | | 60% | 57% | 51% | 0. 659 |
| Third Ouartile | | 69% | 77% | 63% | 0. 765 |
| Average | | 75% | 80% | 71% | 0. 828 |
| First Ouartile | | 78% | 83% | 79% | 0. 857 |
| Maximum Value | | 84% | 90% | 92% | 0. 897 |
| Average | 10 | 74% | 78% | 73% | 0. 820 |
| Standard | | 8% | 7% | 9% | 0. 063 |
| Minimum Value | | 59% | 63% | 57% | 0. 701 |
| Third Ouartile | | 69% | 73% | 67% | 0. 783 |
| Average | | 75% | 80% | 72% | 0. 821 |
| First Ouartile | | 81% | 83% | 81% | 0. 870 |
| Maximum Value | | 87% | 87% | 86% | 0. 939 |
| Average | 15 | 73% | 77% | 72% | 0. 812 |
| Standard | | 8% | 6% | 10% | 0. 063 |
| Minimum Value | | 57% | 67% | 51% | 0. 681 |
| Third Ouartile | | 67% | 72% | 65% | 0. 768 |
| Average | | 73% | 80% | 72% | 0. 802 |
| First Ouartile | | 80% | 80% | 80% | 0. 853 |
| Maximum Value | | 87% | 90% | 88% | 0. 910 |
| Average | 20 | 73% | 75% | 72% | 0. 796 |
| Standard | | 7% | 7% | 9% | 0. 071 |
| Minimum Value | | 56% | 63% | 49% | 0. 650 |
| Third Ouartile | | 71% | 69% | 70% | 0. 759 |
| Average | | 72% | 73% | 72% | 0. 803 |
| First Ouartile | | 76% | 80% | 76% | 0. 843 |
| Maximum Value | | 87% | 87% | 86% | 0. 910 |

| | | | | | |
|---|---|---|---|---|---|
| ^{∗}A represents the number of miRNAs used for prediction, B represents Accuracy, C represents Sensitivity, D represents Specificity, and E represents AUC. | | | | | |

### [Table 10-1]

**Table 10-1: Prediction of Pancreatic Cancer based on the Expression of Randomly Picked miRNA**

| Trial | A | B | C | D | E | Trial | A | B | C | D | E |
|---|---|---|---|---|---|---|---|---|---|---|---|
| 0 | 5 | 68% | 67% | 69% | 0. 799 | 0 | 15 | 69% | 63% | 72% | 0. 792 |
| 1 | | 66% | 90% | 57% | 0. 683 | 1 | | 71% | 83% | 66% | 0. 801 |
| 2 | | 64% | 70% | 62% | 0. 726 | 2 | | 69% | 70% | 69% | 0. 784 |
| 3 | | 67% | 77% | 64% | 0. 800 | 3 | | 68% | 77% | 65% | 0. 800 |
| 4 | | 64% | 83% | 57% | 0. 791 | 4 | | 72% | 90% | 65% | 0. 746 |
| 5 | | 75% | 83% | 72% | 0. 704 | 5 | | 68% | 77% | 65% | 0. 789 |
| 6 | | 74% | 80% | 72% | 0. 855 | 6 | | 76% | 80% | 74% | 0. 840 |
| 7 | | 68% | 67% | 69% | 0. 799 | 7 | | 71% | 77% | 69% | 0. 800 |
| 8 | | 68% | 90% | 59% | 0. 723 | 8 | | 80% | 97% | 73% | 0. 807 |
| 9 | | 72% | 83% | 68% | 0. 762 | 9 | | 79% | 80% | 78% | 0. 827 |
| 10 | | 67% | 70% | 66% | 0. 750 | 10 | | 83% | 80% | 84% | 0. 845 |
| 11 | | 85% | 87% | 84% | 0. 921 | 11 | | 77% | 77% | 77% | 0. 868 |
| 12 | | 63% | 70% | 61% | 0. 657 | 12 | | 63% | 73% | 58% | 0. 700 |
| 13 | | 59% | 80% | 50% | 0. 656 | 13 | | 72% | 70% | 73% | 0. 805 |
| 14 | | 86% | 80% | 88% | 0. 883 | 14 | | 69% | 73% | 68% | 0. 784 |
| 15 | | 69% | 80% | 65% | 0. 750 | 15 | | 86% | 77% | 89% | 0. 903 |
| 16 | | 77% | 87% | 73% | 0. 827 | 16 | | 69% | 67% | 70% | 0. 704 |
| 17 | | 69% | 83% | 64% | 0. 832 | 17 | | 57% | 77% | 49% | 0. 622 |
| 18 | | 65% | 87% | 57% | 0. 732 | 18 | | 71% | 77% | 69% | 0. 826 |
| 19 | | 63% | 90% | 53% | 0. 751 | 19 | | 59% | 63% | 57% | 0. 642 |
| 0 | 10 | 79% | 93% | 73% | 0_{.} 835 | 0 | 20 | 71% | 80% | 68% | 0. 844 |
| 1 | | 85% | 83% | 85% | 0. 818 | 1 | | 64% | 77% | 59% | 0. 699 |
| 2 | | 65% | 80% | 59% | 0. 634 | 2 | | 78% | 77% | 78% | 0. 834 |
| 3 | | 81% | 77% | 82% | 0. 890 | 3 | | 77% | 77% | 77% | 0. 875 |
| 4 | | 82% | 83% | 81% | 0. 876 | 4 | | 67% | 70% | 66% | 0. 799 |
| 5 | | 73% | 90% | 66% | 0. 842 | 5 | | 79% | 70% | 82% | 0. 762 |
| 6 | | 80% | 83% | 78% | 0. 831 | 6 | | 73% | 77% | 72% | 0. 820 |
| 7 | | 67% | 70% | 66% | 0. 806 | 7 | | 71% | 83% | 66% | 0. 793 |
| 8 | | 72% | 87% | 66% | 0. 781 | 8 | | 67% | 77% | 64% | 0. 767 |
| 9 | | 80% | 87% | 77% | 0. 822 | 9 | | 70% | 80% | 66% | 0. 809 |
| 10 | | 63% | 83% | 54% | 0. 704 | 10 | | 80% | 77% | 81% | 0. 861 |
| 11 | | 73% | 70% | 74% | 0. 797 | 11 | | 69% | 77% | 66% | 0. 754 |
| 12 | | 65% | 67% | 65% | 0. 744 | 12 | | 77% | 80% | 76% | 0. 826 |
| 13 | | 70% | 67% | 72% | 0. 778 | 13 | | 80% | 73% | 82% | 0. 817 |
| 14 | | 68% | 73% | 66% | 0. 767 | 14 | | 75% | 77% | 74% | 0. 850 |
| 15 | | 80% | 90% | 76% | 0. 823 | 15 | | 79% | 77% | 80% | 0. 774 |
| 16 | | 69% | 87% | 62% | 0. 777 | 16 | | 77% | 83% | 74% | 0. 822 |
| 17 | | 77% | 77% | 77% | 0. 846 | 17 | | 73% | 73% | 73% | 0. 806 |
| 18 | | 80% | 73% | 82% | 0. 818 | 18 | | 75% | 67% | 78% | 0. 803 |
| 19 | | 73% | 73% | 73% | 0. 786 | 19 | | 63% | 70% | 61% | 0. 746 |

| | | | | | | | | | | | |
|---|---|---|---|---|---|---|---|---|---|---|---|
| ^{∗}A represents the number of miRNAs used for prediction, B represents Accuracy, C represents Sensitivity, D represents Specificity, and E represents AUC. | | | | | | | | | | | |

### [Table 10-2]

**Table 10-2: Summary of Prediction of Pancreatic Cancer based on the Expression of Randomly Picked miRNA**

| | A | B | C | D | E |
|---|---|---|---|---|---|
| Average | 5 | 70% | 80% | 65% | 0. 770 |
| Standard | | 7% | 8% | 9% | 0. 072 |
| Minimum Value | | 59% | 67% | 50% | 0. 656 |
| Third Ouartile | | 65% | 75% | 59% | 0. 725 |
| Average | | 68% | 82% | 64% | 0. 757 |
| First Ouartile | | 73% | 87% | 70% | 0. 807 |
| Maximum Value | | 86% | 90% | 88% | 0. 921 |
| Average | 10 | 74% | 80% | 72% | 0. 799 |
| Standard | | 6% | 8% | 8% | 0. 058 |
| Minimum Value | | 63% | 67% | 54% | 0. 634 |
| Third Ouartile | | 69% | 73% | 66% | 0. 778 |
| Average | | 73% | 82% | 73% | 0. 812 |
| First Ouartile | | 80% | 87% | 77% | 0. 832 |
| Maximum Value | | 85% | 93% | 85% | 0. 890 |
| Average | 15 | 71% | 76% | 69% | 0. 784 |
| Standard | | 7% | 8% | 9% | 0. 071 |
| Minimum Value | | 57% | 63% | 49% | 0. 622 |
| Third Ouartile | | 69% | 72% | 65% | 0. 774 |
| Average | | 71% | 77% | 69% | 0. 800 |
| First Ouartile | | 76% | 80% | 73% | 0. 826 |
| Maximum Value | | 86% | 97% | 89% | 0. 903 |
| Average | 20 | 73% | 76% | 72% | 0. 803 |
| Standard | | 5% | 4% | 7% | 0. 043 |
| Minimum Value | | 63% | 67% | 59% | 0. 699 |
| Third Ouartile | | 70% | 73% | 66% | 0. 773 |
| Average | | 74% | 77% | 74% | 0. 807 |
| First Ouartile | | 77% | 78% | 78% | 0. 828 |
| Maximum Value | | 80% | 83% | 82% | 0. 875 |

| | | | | | |
|---|---|---|---|---|---|
| ^{∗}A represents the number of miRNAs used for prediction, B represents Accuracy, C represents Sensitivity, D represents Specificity, and E represents AUC. | | | | | |

### [Table 11-1]

**Table 11-1: Summary of Prediction of Prostate Cancer based on the Expression of Randomly Picked miRNA**

| Trial | A | B | C | D | E | Trial | A | B | C | D | E |
|---|---|---|---|---|---|---|---|---|---|---|---|
| 0 | 5 | 59% | 93% | 45% | 0. 593 | 0 | 15 | 57% | 63% | 54% | 0. 587 |
| 1 | | 60% | 73% | 54% | 0. 652 | 1 | | 55% | 63% | 51% | 0. 595 |
| 2 | | 53% | 63% | 49% | 0. 526 | 2 | | 59% | 50% | 62% | 0. 636 |
| 3 | | 55% | 70% | 49% | 0. 572 | 3 | | 63% | 67% | 62% | 0. 761 |
| 4 | | 61% | 80% | 53% | 0. 658 | 4 | | 56% | 53% | 57% | 0. 599 |
| 5 | | 64% | 73% | 61% | 0. 620 | 5 | | 56% | 57% | 55% | 0. 605 |
| 6 | | 58% | 87% | 46% | 0. 639 | 6 | | 66% | 60% | 69% | 0. 589 |
| 7 | | 55% | 93% | 39% | 0. 595 | 7 | | 63% | 70% | 59% | 0. 601 |
| 8 | | 64% | 77% | 59% | 0. 705 | 8 | | 58% | 63% | 55% | 0. 662 |
| 9 | | 48% | 87% | 32% | 0. 586 | 9 | | 55% | 53% | 55% | 0. 545 |
| 10 | | 60% | 77% | 53% | 0. 567 | 10 | | 60% | 53% | 62% | 0. 645 |
| 11 | | 60% | 77% | 53% | 0. 616 | 11 | | 58% | 70% | 53% | 0. 581 |
| 12 | | 54% | 60% | 51% | 0. 542 | 12 | | 51% | 63% | 46% | 0. 517 |
| 13 | | 58% | 77% | 50% | 0. 598 | 13 | | 56% | 53% | 57% | 0. 513 |
| 14 | | 63% | 63% | 62% | 0. 686 | 14 | | 55% | 63% | 51% | 0. 624 |
| 15 | | 54% | 77% | 45% | 0. 505 | 15 | | 62% | 47% | 68% | 0. 622 |
| 16 | | 47% | 83% | 32% | 0. 671 | 16 | | 63% | 57% | 65% | 0. 549 |
| 17 | | 66% | 87% | 58% | 0. 672 | 17 | | 56% | 63% | 53% | 0. 508 |
| 18 | | 59% | 80% | 50% | 0. 590 | 18 | | 58% | 57% | 58% | 0. 626 |
| 19 | | 62% | 63% | 61% | 0. 613 | 19 | | 57% | 70% | 51% | 0. 647 |
| 0 | 10 | 55% | 73% | 47% | 0. 592 | 0 | 20 | 63% | 47% | 69% | 0. 547 |
| 1 | | 60% | 70% | 55% | 0. 591 | 1 | | 60% | 53% | 62% | 0. 583 |
| 2 | | 60% | 83% | 50% | 0. 709 | 2 | | 54% | 57% | 53% | 0. 527 |
| 3 | | 62% | 67% | 59% | 0. 640 | 3 | | 58% | 50% | 61% | 0. 596 |
| 4 | | 66% | 67% | 66% | 0. 661 | 4 | | 62% | 63% | 61% | 0. 648 |
| 5 | | 51% | 83% | 38% | 0. 547 | 5 | | 63% | 60% | 64% | 0. 676 |
| 6 | | 51% | 53% | 50% | 0. 510 | 6 | | 58% | 50% | 61% | 0. 491 |
| 7 | | 62% | 80% | 54% | 0. 595 | 7 | | 75% | 63% | 80% | 0. 669 |
| 8 | | 60% | 63% | 58% | 0. 628 | 8 | | 57% | 67% | 53% | 0. 595 |
| 9 | | 57% | 70% | 51% | 0. 627 | 9 | | 66% | 60% | 69% | 0. 736 |
| 10 | | 63% | 73% | 59% | 0. 672 | 10 | | 59% | 63% | 57% | 0. 632 |
| 11 | | 64% | 83% | 57% | 0. 647 | 11 | | 50% | 60% | 46% | 0. 561 |
| 12 | | 50% | 57% | 47% | 0. 525 | 12 | | 66% | 57% | 70% | 0. 699 |
| 13 | | 58% | 60% | 57% | 0. 613 | 13 | | 67% | 60% | 70% | 0. 698 |
| 14 | | 69% | 73% | 68% | 0. 652 | 14 | | 57% | 53% | 58% | 0. 636 |
| 15 | | 67% | 73% | 65% | 0. 699 | 15 | | 56% | 67% | 51% | 0. 575 |
| 16 | | 60% | 73% | 54% | 0. 707 | 16 | | 63% | 60% | 65% | 0. 663 |
| 17 | | 65% | 57% | 69% | 0. 602 | 17 | | 56% | 63% | 53% | 0. 622 |
| 18 | | 59% | 73% | 53% | 0. 618 | 18 | | 73% | 73% | 73% | 0. 724 |
| 19 | | 52% | 67% | 46% | 0. 498 | 19 | | 66% | 70% | 65% | 0. 680 |

| | | | | | | | | | | | |
|---|---|---|---|---|---|---|---|---|---|---|---|
| ^{∗}A represents the number of miRNAs used for prediction, B represents Accuracy, C represents Sensitivity, D represents Specificity, and E represents AUC. | | | | | | | | | | | |

### [Table 11-2]

**Table 11-2: Summary of Prediction of Prostate Cancer based on the Expression of Randomly Picked miRNA**

| | A | B | C | D | E |
|---|---|---|---|---|---|
| Average | 5 | 58% | 77% | 50% | 0. 610 |
| Standard | | 5% | 10% | 9% | 0. 054 |
| Minimum Value | | 47% | 60% | 32% | 0. 505 |
| Third Ouartile | | 55% | 72% | 46% | 0. 583 |
| Average | | 59% | 77% | 51% | 0. 606 |
| First Ouartile | | 61% | 84% | 55% | 0. 653 |
| Maximum Value | | 66% | 93% | 62% | 0. 705 |
| Average | 10 | 59% | 70% | 55% | 0. 617 |
| Standard | | 6% | 9% | 8% | 0. 062 |
| Minimum Value | | 50% | 53% | 38% | 0. 498 |
| Third Ouartile | | 56% | 66% | 50% | 0. 592 |
| Average | | 60% | 72% | 55% | 0. 622 |
| First Ouartile | | 64% | 73% | 59% | 0. 654 |
| Maximum Value | | 69% | 83% | 69% | 0. 709 |
| Average | 15 | 58% | 60% | 57% | 0. 601 |
| Standard | | 4% | 7% | 6% | 0. 059 |
| Minimum Value | | 51% | 47% | 46% | 0. 508 |
| Third Ouartile | | 56% | 53% | 53% | 0. 573 |
| Average | | 57% | 62% | 56% | 0. 600 |
| First Ouartile | | 60% | 63% | 62% | 0. 629 |
| Maximum Value | | 66% | 70% | 69% | 0. 761 |
| Average | 20 | 61% | 60% | 62% | 0. 628 |
| Standard | | 6% | 7% | 8% | 0. 067 |
| Minimum Value | | 50% | 47% | 46% | 0. 491 |
| Third Ouartile | | 57% | 56% | 56% | 0. 581 |
| Average | | 61% | 60% | 61% | 0. 634 |
| First Ouartile | | 66% | 63% | 69% | 0. 677 |
| Maximum Value | | 75% | 73% | 80% | 0. 736 |

| | | | | | |
|---|---|---|---|---|---|
| ^{∗}A represents the number of miRNAs used for prediction, B represents Accuracy, C represents Sensitivity, D represents Specificity, and E represents AUC. | | | | | |

### [Table 12-1]

**Table 12-1: Prediction of Stomach Cancer based on the Expression of Randomly Picked miRNA**

| Trial | A | B | C | D | E | Trial | A | B | C | D | E |
|---|---|---|---|---|---|---|---|---|---|---|---|
| 0 | 5 | 61% | 77% | 54% | 0. 719 | 0 | 15 | 60% | 63% | 58% | 0. 695 |
| 1 | | 67% | 70% | 66% | 0. 755 | 1 | | 71% | 63% | 74% | 0. 774 |
| 2 | | 58% | 70% | 53% | 0. 693 | 2 | | 59% | 67% | 55% | 0. 659 |
| 3 | | 72% | 63% | 76% | 0. 774 | 3 | | 57% | 60% | 55% | 0. 527 |
| 4 | | 74% | 67% | 77% | 0. 782 | 4 | | 54% | 73% | 46% | 0. 565 |
| 5 | | 65% | 57% | 69% | 0. 639 | 5 | | 54% | 67% | 49% | 0. 574 |
| 6 | | 62% | 70% | 58% | 0. 618 | 6 | | 63% | 70% | 59% | 0. 686 |
| 7 | | 52% | 77% | 42% | 0. 677 | 7 | | 63% | 70% | 59% | 0. 675 |
| 8 | | 63% | 60% | 64% | 0. 628 | 8 | | 60% | 70% | 55% | 0. 649 |
| 9 | | 61% | 73% | 55% | 0. 722 | 9 | | 63% | 70% | 61% | 0. 701 |
| 10 | | 62% | 67% | 59% | 0. 677 | 10 | | 63% | 70% | 61% | 0. 686 |
| 11 | | 66% | 67% | 66% | 0. 747 | 11 | | 65% | 67% | 65% | 0. 730 |
| 12 | | 79% | 73% | 81% | 0. 827 | 12 | | 61% | 80% | 53% | 0. 704 |
| 13 | | 55% | 63% | 51% | 0. 669 | 13 | | 68% | 67% | 69% | 0. 710 |
| 14 | | 60% | 60% | 59% | 0. 637 | 14 | | 62% | 83% | 53% | 0. 744 |
| 15 | | 45% | 70% | 35% | 0. 649 | 15 | | 74% | 77% | 73% | 0. 771 |
| 16 | | 55% | 80% | 45% | 0. 641 | 16 | | 61% | 63% | 59% | 0. 693 |
| 17 | | 64% | 83% | 57% | 0. 720 | 17 | | 72% | 80% | 69% | 0. 823 |
| 18 | | 67% | 73% | 65% | 0. 712 | 18 | | 53% | 63% | 49% | 0. 652 |
| 19 | | 74% | 83% | 70% | 0. 827 | 19 | | 61% | 77% | 54% | 0. 629 |
| 0 | 10 | 67% | 53% | 73% | 0. 697 | 0 | 20 | 56% | 60% | 54% | 0. 590 |
| 1 | | 58% | 63% | 55% | 0. 692 | 1 | | 72% | 83% | 68% | 0. 800 |
| 2 | | 50% | 63% | 45% | 0. 605 | 2 | | 58% | 60% | 57% | 0. 620 |
| 3 | | 67% | 63% | 69% | 0. 739 | 3 | | 73% | 77% | 72% | 0. 801 |
| 4 | | 66% | 83% | 59% | 0. 735 | 4 | | 57% | 53% | 58% | 0. 640 |
| 5 | | 50% | 60% | 46% | 0. 593 | 5 | | 60% | 63% | 58% | 0. 693 |
| 6 | | 57% | 67% | 53% | 0. 624 | 6 | | 67% | 70% | 66% | 0. 641 |
| 7 | | 51% | 57% | 49% | 0. 577 | 7 | | 65% | 57% | 69% | 0. 674 |
| 8 | | 58% | 67% | 54% | 0. 623 | 8 | | 72% | 80% | 69% | 0. 760 |
| 9 | | 54% | 67% | 49% | 0. 612 | 9 | | 60% | 70% | 55% | 0. 653 |
| 10 | | 72% | 70% | 73% | 0. 736 | 10 | | 67% | 70% | 66% | 0. 753 |
| 11 | | 66% | 70% | 65% | 0. 737 | 11 | | 50% | 67% | 43% | 0. 515 |
| 12 | | 54% | 77% | 45% | 0. 621 | 12 | | 58% | 70% | 53% | 0. 631 |
| 13 | | 63% | 73% | 59% | 0. 723 | 13 | | 63% | 60% | 65% | 0. 692 |
| 14 | | 73% | 73% | 73% | 0. 788 | 14 | | 57% | 77% | 49% | 0. 648 |
| 15 | | 53% | 77% | 43% | 0. 623 | 15 | | 61% | 73% | 55% | 0. 734 |
| 16 | | 54% | 73% | 46% | 0. 604 | 16 | | 64% | 67% | 64% | 0. 722 |
| 17 | | 57% | 60% | 55% | 0. 665 | 17 | | 73% | 73% | 73% | 0. 750 |
| 18 | | 56% | 67% | 51% | 0. 652 | 18 | | 61% | 73% | 55% | 0. 678 |
| 19 | | 63% | 60% | 64% | 0. 682 | 19 | | 61% | 73% | 55% | 0. 709 |

| | | | | | | | | | | | |
|---|---|---|---|---|---|---|---|---|---|---|---|
| ^{∗}A represents the number of miRNAs used for prediction, B represents Accuracy, C represents Sensitivity, D represents Specificity, and E represents AUC. | | | | | | | | | | | |

### [Table 12-2]

**Table 12-2: Summary of Prediction of Stomach Cancer based on the Expression of Randomly Picked miRNA**

| | A | B | C | D | E |
|---|---|---|---|---|---|
| Average | 5 | 63% | 70% | 60% | 0. 706 |
| Standard | | 8% | 8% | 12% | 0. 064 |
| Minimum Value | | 45% | 57% | 35% | 0. 618 |
| Third Ouartile | | 59% | 66% | 54% | 0. 647 |
| Average | | 62% | 70% | 59% | 0. 703 |
| First Ouartile | | 67% | 74% | 67% | 0. 749 |
| Maximum Value | | 79% | 83% | 81% | 0. 827 |
| Average | 10 | 59% | 67% | 56% | 0. 666 |
| Standard | | 7% | 8% | 10% | 0. 061 |
| Minimum Value | | 50% | 53% | 43% | 0. 577 |
| Third Ouartile | | 54% | 63% | 48% | 0. 619 |
| Average | | 57% | 67% | 55% | 0. 659 |
| First Ouartile | | 66% | 73% | 64% | 0. 726 |
| Maximum Value | | 73% | 83% | 73% | 0. 788 |
| Average | 15 | 62% | 70% | 59% | 0. 682 |
| Standard | | 6% | 6% | 8% | 0. 072 |
| Minimum Value | | 53% | 60% | 46% | 0. 527 |
| Third Ouartile | | 59% | 66% | 54% | 0. 651 |
| Average | | 61% | 70% | 59% | 0. 689 |
| First Ouartile | | 64% | 74% | 62% | 0. 715 |
| Maximum Value | | 74% | 83% | 74% | 0. 823 |
| Average | 20 | 63% | 69% | 60% | 0. 685 |
| Standard | | 7% | 8% | 8% | 0. 072 |
| Minimum Value | | 50% | 53% | 43% | 0. 515 |
| Third Ouartile | | 58% | 63% | 55% | 0. 641 |
| Average | | 61% | 70% | 58% | 0. 685 |
| First Ouartile | | 67% | 73% | 67% | 0. 738 |
| Maximum Value | | 73% | 83% | 73% | 0. 801 |

| | | | | | |
|---|---|---|---|---|---|
| ^{∗}A represents the number of miRNAs used for prediction, B represents Accuracy, C represents Sensitivity, D represents Specificity, and E represents AUC. | | | | | |

### [Table 13-1]

**Table 13-1: Prediction of Urothelial Cancer based on the Expression of Randomly Picked miRNA**

| Trial | A | B | C | D | E | Trial | A | B | C | D | E |
|---|---|---|---|---|---|---|---|---|---|---|---|
| 0 | 5 | 63% | 73% | 58% | 0. 660 | 0 | 15 | 68% | 67% | 69% | 0. 777 |
| 1 | | 64% | 60% | 66% | 0. 662 | 1 | | 73% | 70% | 74% | 0. 805 |
| 2 | | 61% | 73% | 55% | 0. 588 | 2 | | 59% | 63% | 57% | 0. 617 |
| 3 | | 78% | 77% | 78% | 0. 822 | 3 | | 65% | 70% | 64% | 0. 672 |
| 4 | | 66% | 57% | 70% | 0. 732 | 4 | | 69% | 57% | 74% | 0. 735 |
| 5 | | 71% | 73% | 70% | 0. 773 | 5 | | 62% | 67% | 59% | 0. 619 |
| 6 | | 60% | 63% | 58% | 0. 684 | 6 | | 65% | 63% | 66% | 0. 704 |
| 7 | | 77% | 73% | 78% | 0. 788 | 7 | | 58% | 53% | 59% | 0. 632 |
| 8 | | 69% | 77% | 66% | 0. 835 | 8 | | 70% | 80% | 66% | 0. 730 |
| 9 | | 63% | 73% | 59% | 0. 719 | 9 | | 66% | 67% | 66% | 0. 787 |
| 10 | | 61% | 70% | 57% | 0. 699 | 10 | | 82% | 73% | 85% | 0. 867 |
| 11 | | 59% | 77% | 51% | 0. 695 | 11 | | 62% | 67% | 59% | 0. 733 |
| 12 | | 78% | 77% | 78% | 0. 850 | 12 | | 73% | 60% | 78% | 0. 741 |
| 13 | | 64% | 70% | 62% | 0. 588 | 13 | | 70% | 70% | 70% | 0. 763 |
| 14 | | 80% | 80% | 80% | 0. 874 | 14 | | 68% | 70% | 68% | 0. 714 |
| 15 | | 73% | 83% | 69% | 0. 851 | 15 | | 65% | 70% | 64% | 0. 730 |
| 16 | | 71% | 73% | 70% | 0. 772 | 16 | | 66% | 73% | 64% | 0. 814 |
| 17 | | 72% | 70% | 73% | 0. 673 | 17 | | 73% | 80% | 70% | 0. 797 |
| 18 | | 71% | 77% | 69% | 0. 855 | 18 | | 63% | 77% | 57% | 0. 734 |
| 19 | | 79% | 83% | 77% | 0. 899 | 19 | | 80% | 77% | 81% | 0. 811 |
| 0 | 10 | 81% | 77% | 89% | 0. 844 | 0 | 20 | 81% | 83% | 80% | 0. 901 |
| 1 | | 62% | 60% | 62% | 0. 757 | 1 | | 80% | 77% | 81% | 0. 863 |
| 2 | | 69% | 67% | 70% | 0. 836 | 2 | | 73% | 63% | 77% | 0. 714 |
| 3 | | 65% | 73% | 62% | 0. 734 | 3 | | 71% | 67% | 73% | 0. 735 |
| 4 | | 61% | 63% | 59% | 0. 692 | 4 | | 63% | 50% | 69% | 0. 614 |
| 5 | | 63% | 67% | 61% | 0. 720 | 5 | | 70% | 73% | 69% | 0. 803 |
| 6 | | 66% | 70% | 65% | 0. 750 | 6 | | 75% | 67% | 78% | 0. 758 |
| 7 | | 59% | 73% | 53% | 0. 718 | 7 | | 75% | 73% | 76% | 0. 832 |
| 8 | | 70% | 73% | 69% | 0. 744 | 8 | | 66% | 57% | 70% | 0. 689 |
| 9 | | 62% | 73% | 57% | 0. 761 | 9 | | 65% | 60% | 68% | 0. 736 |
| 10 | | 60% | 67% | 57% | 0. 602 | 10 | | 63% | 77% | 57% | 0. 714 |
| 11 | | 62% | 63% | 61% | 0. 724 | 11 | | 77% | 70% | 80% | 0. 810 |
| 12 | | 65% | 63% | 66% | 0. 639 | 12 | | 72% | 83% | 68% | 0. 759 |
| 13 | | 71% | 70% | 72% | 0. 716 | 13 | | 74% | 77% | 73% | 0. 800 |
| 14 | | 59% | 60% | 58% | 0. 677 | 14 | | 77% | 60% | 84% | 0. 815 |
| 15 | | 69% | 77% | 66% | 0. 787 | 15 | | 63% | 67% | 62% | 0. 591 |
| 16 | | 76% | 80% | 74% | 0. 817 | 16 | | 63% | 63% | 62% | 0. 691 |
| 17 | | 70% | 73% | 69% | 0. 804 | 17 | | 78% | 70% | 81% | 0. 801 |
| 18 | | 75% | 70% | 77% | 0. 794 | 18 | | 81% | 73% | 84% | 0. 889 |
| 19 | | 63% | 67% | 62% | 0. 745 | 19 | | 80% | 70% | 84% | 0. 827 |

| | | | | | | | | | | | |
|---|---|---|---|---|---|---|---|---|---|---|---|
| ^{∗}A represents the number of miRNAs used for prediction, B represents Accuracy, C represents Sensitivity, D represents Specificity, and E represents AUC. | | | | | | | | | | | |

### [Table 13-2]

**Table 13-2: Summary of Prediction of Urothelial Cancer based on the Expression of Randomly Picked miRNA**

| | A | B | C | D | E |
|---|---|---|---|---|---|
| Average | 5 | 69% | 73% | 67% | 0. 751 |
| Standard | | 7% | 7% | 9% | 0. 094 |
| Minimum Value | | 59% | 57% | 51% | 0. 588 |
| Third Ouartile | | 63% | 70% | 59% | 0. 681 |
| Average | | 70% | 73% | 69% | 0. 752 |
| First Ouartile | | 74% | 77% | 74% | 0. 839 |
| Maximum Value | | 80% | 83% | 80% | 0. 899 |
| Average | 10 | 66% | 69% | 65% | 0. 743 |
| Standard | | 6% | 6% | 7% | 0. 062 |
| Minimum Value | | 59% | 60% | 53% | 0. 602 |
| Third Ouartile | | 62% | 66% | 60% | 0. 717 |
| Average | | 65% | 70% | 64% | 0. 744 |
| First Ouartile | | 70% | 73% | 69% | 0. 789 |
| Maximum Value | | 81% | 80% | 82% | 0. 840 |
| Average | 15 | 68% | 69% | 68% | 0. 739 |
| Standard | | 6% | 7% | 8% | 0. 067 |
| Minimum Value | | 58% | 53% | 57% | 0. 617 |
| Third Ouartile | | 65% | 66% | 63% | 0. 711 |
| Average | | 67% | 70% | 66% | 0. 734 |
| First Ouartile | | 71% | 73% | 71% | 0. 789 |
| Maximum Value | | 82% | 80% | 85% | 0. 867 |
| Average | 20 | 72% | 69% | 74% | 0. 767 |
| Standard | | 6% | 9% | 8% | 0. 083 |
| Minimum Value | | 63% | 50% | 57% | 0. 591 |
| Third Ouartile | | 66% | 63% | 69% | 0. 714 |
| Average | | 74% | 70% | 74% | 0. 780 |
| First Ouartile | | 77% | 74% | 80% | 0. 818 |
| Maximum Value | | 81% | 83% | 84% | 0. 901 |

| | | | | | |
|---|---|---|---|---|---|
| ^{∗}A represents the number of miRNAs used for prediction, B represents Accuracy, C represents Sensitivity, D represents Specificity, and E represents AUC. | | | | | |

### [Table 14-1]

**Table 14-1: Prediction of Melanoma based on the Expression of Randomly Picked miRNA**

| Trial | A | B | C | D | E | Trial | A | B | C | D | E |
|---|---|---|---|---|---|---|---|---|---|---|---|
| 0 | 5 | 68% | 62% | 69% | 0. 683 | 0 | 15 | 82% | 67% | 85% | 0. 848 |
| 1 | | 84% | 71% | 87% | 0. 788 | 1 | | 91% | 81% | 93% | 0. 940 |
| 2 | | 60% | 62% | 60% | 0. 698 | 2 | | 87% | 76% | 89% | 0. 893 |
| 3 | | 78% | 71% | 80% | 0. 716 | 3 | | 78% | 52% | 83% | 0. 809 |
| 4 | | 83% | 81% | 83% | 0. 886 | 4 | | 78% | 76% | 78% | 0. 845 |
| 5 | | 83% | 48% | 90% | 0. 717 | 5 | | 89% | 81% | 91% | 0. 896 |
| 6 | | 78% | 86% | 77% | 0. 866 | 6 | | 82% | 71% | 84% | 0. 779 |
| 7 | | 92% | 90% | 92% | 0. 905 | 7 | | 71% | 62% | 73% | 0. 718 |
| 8 | | 89% | 86% | 90% | 0. 943 | 8 | | 85% | 71% | 88% | 0. 875 |
| 9 | | 87% | 71% | 90% | 0. 850 | 9 | | 73% | 67% | 74% | 0. 771 |
| 10 | | 96% | 90% | 97% | 0. 982 | 10 | | 93% | 81% | 95% | 0. 896 |
| 11 | | 62% | 81% | 58% | 0. 639 | 11 | | 78% | 71% | 79% | 0. 826 |
| 12 | | 65% | 62% | 66% | 0. 705 | 12 | | 83% | 71% | 86% | 0. 868 |
| 13 | | 64% | 86% | 60% | 0. 813 | 13 | | 91% | 76% | 94% | 0. 903 |
| 14 | | 77% | 76% | 77% | 0. 831 | 14 | | 90% | 86% | 91% | 0. 918 |
| 15 | | 74% | 62% | 77% | 0. 755 | 15 | | 81% | 81% | 81% | 0. 841 |
| 16 | | 80% | 57% | 85% | 0. 766 | 16 | | 94% | 95% | 94% | 0. 949 |
| 17 | | 83% | 86% | 83% | 0. 880 | 17 | | 89% | 71% | 93% | 0. 939 |
| 18 | | 76% | 76% | 76% | 0. 809 | 18 | | 76% | 48% | 82% | 0. 660 |
| 19 | | 63% | 71% | 62% | 0. 638 | 19 | | 93% | 90% | 94% | 0. 943 |
| 0 | 10 | 83% | 81% | 84% | 0, 900 | 0 | 20 | 92% | 86% | 93% | 0. 919 |
| 1 | | 78% | 71% | 79% | 0. 773 | 1 | | 85% | 76% | 87% | 0. 887 |
| 2 | | 74% | 67% | 76% | 0. 779 | 2 | | 79% | 67% | 82% | 0. 811 |
| 3 | | 83% | 86% | 82% | 0.920 | 3 | | 88% | 86% | 89% | 0. 937 |
| 4 | | 82% | 86% | 81% | 0. 897 | 4 | | 98% | 95% | 98% | 0. 993 |
| 5 | | 88% | 81% | 89% | 0. 866 | 5 | | 83% | 86% | 83% | 0. 902 |
| 6 | | 68% | 71% | 68% | 0. 732 | 6 | | 92% | 81% | 94% | 0. 945 |
| 7 | | 69% | 57% | 72% | 0. 724 | 7 | | 91% | 90% | 91% | 0. 913 |
| 8 | | 78% | 52% | 83% | 0. 781 | 8 | | 91% | 76% | 94% | 0. 940 |
| 9 | | 69% | 76% | 68% | 0. 682 | 9 | | 90% | 67% | 95% | 0. 858 |
| 10 | | 83% | 86% | 82% | 0. 885 | 10 | | 88% | 95% | 87% | 0. 943 |
| 11 | | 92% | 71% | 96% | 0. 854 | 11 | | 96% | 90% | 97% | 0. 978 |
| 12 | | 85% | 81% | 86% | 0. 835 | 12 | | 89% | 76% | 92% | 0. 886 |
| 13 | | 76% | 67% | 78% | 0. 775 | 13 | | 98% | 90% | 99% | 0. 963 |
| 14 | | 71% | 67% | 72% | 0. 717 | 14 | | 79% | 62% | 83% | 0. 811 |
| 15 | | 72% | 57% | 75% | 0. 758 | 15 | | 86% | 67% | 90% | 0. 911 |
| 16 | | 68% | 71% | 67% | 0. 749 | 16 | | 88% | 81% | 90% | 0. 927 |
| 17 | | 79% | 76% | 80% | 0. 862 | 17 | | 89% | 81% | 91% | 0. 916 |
| 18 | | 80% | 81% | 80% | 0. 848 | 18 | | 83% | 57% | 88% | 0. 851 |
| 19 | | 88% | 81% | 90% | 0. 930 | 19 | | 84% | 76% | 86% | 0. 863 |

| | | | | | | | | | | | |
|---|---|---|---|---|---|---|---|---|---|---|---|
| ^{∗}A represents the number of miRNAs used for prediction, B represents Accuracy, C represents Sensitivity, D represents Specificity, and E represents AUC. | | | | | | | | | | | |

### [Table 14-2]

**Table 14-2: Summary of Prediction of Melanoma based on the Expression of Randomly Picked miRNA**

| | A | B | C | D | E |
|---|---|---|---|---|---|
| Average | 5 | 77% | 74% | 78% | 0. 793 |
| Standard | | 11% | 12% | 12% | 0. 100 |
| Minimum Value | | 60% | 48% | 58% | 0. 638 |
| Third Ouartile | | 67% | 62% | 68% | 0. 713 |
| Average | | 78% | 74% | 78% | 0. 798 |
| First Ouartile | | 84% | 86% | 88% | 0. 870 |
| Maximum Value | | 96% | 90% | 97% | 0. 982 |
| Average | 10 | 78% | 73% | 79% | 0. 813 |
| Standard | | 7% | 10% | 8% | 0. 075 |
| Minimum Value | | 68% | 52% | 67% | 0. 682 |
| Third Ouartile | | 71% | 67% | 74% | 0. 756 |
| Average | | 78% | 74% | 80% | 0. 808 |
| First Ouartile | | 83% | 81% | 83% | 0. 871 |
| Maximum Value | | 92% | 86% | 96% | 0. 930 |
| Average | 15 | 84% | 74% | 86% | 0. 856 |
| Standard | | 7% | 12% | 7% | 0. 078 |
| Minimum Value | | 71% | 48% | 73% | 0. 660 |
| Third Ouartile | | 78% | 70% | 82% | 0. 822 |
| Average | | 84% | 74% | 87% | 0. 872 |
| First Ouartile | | 90% | 81% | 93% | 0. 907 |
| Maximum Value | | 94% | 95% | 95% | 0. 949 |
| Average | 20 | 88% | 79% | 90% | 0. 908 |
| Standard | | 5% | 11% | 5% | 0. 050 |
| Minimum Value | | 79% | 57% | 82% | 0. 811 |
| Third Ouartile | | 85% | 74% | 87% | 0. 880 |
| Average | | 89% | 81% | 90% | 0. 915 |
| First Ouartile | | 91% | 87% | 94% | 0. 941 |
| Maximum Value | | 98% | 95% | 99% | 0. 993 |

| | | | | | |
|---|---|---|---|---|---|
| ^{∗}A represents the number of miRNAs used for prediction, B represents Accuracy, C represents Sensitivity, D represents Specificity, and E represents AUC. | | | | | |

### [Table 15-1]

**Table 15-1: Prediction of Ovarian Cancer based on the Expression of Randomly Picked miRNA**

| Trial | A | B | C | D | E | Trial | A | B | C | D | E |
|---|---|---|---|---|---|---|---|---|---|---|---|
| 0 | 5 | 51% | 55% | 49% | 0. 460 | 0 | 15 | 66% | 59% | 68% | 0. 750 |
| 1 | | 55% | 48% | 57% | 0. 547 | 1 | | 71% | 52% | 77% | 0. 735 |
| 2 | | 56% | 55% | 57% | 0. 693 | 2 | | 60% | 38% | 67% | 0. 670 |
| 3 | | 53% | 59% | 52% | 0. 540 | 3 | | 69% | 52% | 74% | 0. 589 |
| 4 | | 59% | 41% | 65% | 0. 582 | 4 | | 63% | 48% | 68% | 0. 553 |
| 5 | | 61% | 62% | 61% | 0. 572 | 5 | | 65% | 62% | 66% | 0. 688 |
| 6 | | 54% | 55% | 54% | 0. 574 | 6 | | 67% | 41% | 75% | 0. 649 |
| 7 | | 65% | 69% | 64% | 0. 608 | 7 | | 75% | 62% | 79% | 0. 824 |
| 8 | | 66% | 52% | 71% | 0. 620 | 8 | | 57% | 52% | 59% | 0. 594 |
| 9 | | 74% | 62% | 78% | 0. 769 | 9 | | 70% | 69% | 70% | 0. 734 |
| 10 | | 55% | 41% | 59% | 0. 474 | 10 | | 58% | 45% | 62% | 0. 607 |
| 11 | | 61% | 66% | 60% | 0. 581 | 11 | | 57% | 48% | 60% | 0. 602 |
| 12 | | 68% | 55% | 72% | 0. 670 | 12 | | 54% | 41% | 58% | 0. 464 |
| 13 | | 53% | 45% | 56% | 0. 527 | 13 | | 62% | 59% | 63% | 0. 629 |
| 14 | | 73% | 66% | 75% | 0. 784 | 14 | | 52% | 28% | 59% | 0. 383 |
| 15 | | 60% | 62% | 60% | 0. 612 | 15 | | 58% | 59% | 58% | 0. 577 |
| 16 | | 71% | 62% | 74% | 0. 709 | 16 | | 71% | 72% | 71% | 0. 752 |
| 17 | | 63% | 52% | 67% | 0. 639 | 17 | | 60% | 62% | 60% | 0. 656 |
| 18 | | 55% | 66% | 52% | 0. 572 | 18 | | 48% | 59% | 44% | 0. 570 |
| 19 | | 61% | 52% | 64% | 0. 574 | 19 | | 70% | 72% | 69% | 0. 762 |
| 0 | 10 | 62% | 45% | 67% | 0. 621 | 0 | 20 | 73% | 48% | 81% | 0. 746 |
| 1 | | 70% | 55% | 74% | 0. 670 | 1 | | 55% | 38% | 60% | 0. 583 |
| 2 | | 48% | 28% | 55% | 0. 417 | 2 | | 66% | 59% | 68% | 0. 665 |
| 3 | | 71% | 62% | 74% | 0. 684 | 3 | | 67% | 38% | 76% | 0. 685 |
| 4 | | 69% | 55% | 73% | 0. 589 | 4 | | 70% | 48% | 77% | 0. 703 |
| 5 | | 60% | 55% | 62% | 0. 593 | 5 | | 55% | 41% | 60% | 0. 481 |
| 6 | | 80% | 72% | 82% | 0. 840 | 6 | | 70% | 59% | 73% | 0. 813 |
| 7 | | 74% | 69% | 76% | 0. 653 | 7 | | 77% | 66% | 80% | 0. 795 |
| 8 | | 72% | 66% | 74% | 0. 655 | 8 | | 66% | 45% | 73% | 0. 611 |
| 9 | | 50% | 52% | 49% | 0. 479 | 9 | | 59% | 28% | 68% | 0. 580 |
| 10 | | 61% | 62% | 61% | 0. 578 | 10 | | 65% | 55% | 68% | 0. 700 |
| 11 | | 75% | 66% | 78% | 0. 743 | 11 | | 78% | 55% | 85% | 0. 719 |
| 12 | | 50% | 45% | 52% | 0. 472 | 12 | | 75% | 72% | 76% | 0. 825 |
| 13 | | 59% | 59% | 59% | 0. 520 | 13 | | 56% | 48% | 59% | 0. 619 |
| 14 | | 62% | 62% | 62% | 0. 577 | 14 | | 60% | 66% | 59% | 0. 664 |
| 15 | | 56% | 62% | 55% | 0. 652 | 15 | | 66% | 66% | 66% | 0. 737 |
| 16 | | 63% | 69% | 61% | 0. 591 | 16 | | 58% | 45% | 62% | 0. 511 |
| 17 | | 65% | 66% | 65% | 0. 589 | 17 | | 72% | 66% | 74% | 0. 785 |
| 18 | | 67% | 76% | 65% | 0. 745 | 18 | | 77% | 66% | 80% | 0. 805 |
| 19 | | 66% | 45% | 73% | 0. 664 | 19 | | 66% | 52% | 71% | 0. 702 |

| | | | | | | | | | | | |
|---|---|---|---|---|---|---|---|---|---|---|---|
| ^{∗}A represents the number of miRNAs used for prediction, B represents Accuracy, C represents Sensitivity, D represents Specificity, and E represents AUC. | | | | | | | | | | | |

### [Table 15-2]

**Table 15-2: Summary of Prediction of Ovarian Cancer based on the Expression of Randomly Picked miRNA**

| | A | B | C | D | E |
|---|---|---|---|---|---|
| Average | 5 | 61% | 56% | 62% | 0. 605 |
| Standard | | 7% | 8% | 8% | 0. 086 |
| Minimum Value | | 51% | 41% | 49% | 0. 460 |
| Third Ouartile | | 55% | 52% | 56% | 0. 565 |
| Average | | 61% | 55% | 60% | 0. 582 |
| First Ouartile | | 65% | 62% | 68% | 0. 647 |
| Maximum Value | | 74% | 69% | 78% | 0. 784 |
| Average | 10 | 64% | 58% | 66% | 0. 617 |
| Standard | | 9% | 11% | 9% | 0. 100 |
| Minimum Value | | 48% | 28% | 49% | 0. 417 |
| Third Ouartile | | 60% | 54% | 60% | 0. 578 |
| Average | | 64% | 62% | 65% | 0. 607 |
| First Ouartile | | 70% | 66% | 74% | 0. 666 |
| Maximum Value | | 80% | 76% | 82% | 0. 840 |
| Average | 15 | 63% | 54% | 65% | 0. 639 |
| Standard | | 7% | 12% | 8% | 0. 106 |
| Minimum Value | | 48% | 28% | 44% | 0. 383 |
| Third Ouartile | | 58% | 47% | 59% | 0. 586 |
| Average | | 63% | 55% | 66% | 0. 639 |
| First Ouartile | | 69% | 62% | 70% | 0. 735 |
| Maximum Value | | 75% | 72% | 79% | 0. 824 |
| Average | 20 | 67% | 53% | 71% | 0. 686 |
| Standard | | 7% | 12% | 8% | 0. 099 |
| Minimum Value | | 55% | 28% | 59% | 0. 481 |
| Third Ouartile | | 60% | 45% | 65% | 0. 617 |
| Average | | 66% | 53% | 72% | 0. 701 |
| First Ouartile | | 72% | 66% | 76% | 0. 756 |
| Maximum Value | | 78% | 72% | 85% | 0. 825 |

| | | | | | |
|---|---|---|---|---|---|
| ^{∗}A represents the number of miRNAs used for prediction, B represents Accuracy, C represents Sensitivity, D represents Specificity, and E represents AUC. | | | | | |

### [Table16-1]

**Table16-1: Prediction of Thyroid Cancer based on the Expression of Randomly Picked miRNA**

| Trial | A | B | C | D | E | Trial | A | B | C | D | E |
|---|---|---|---|---|---|---|---|---|---|---|---|
| 0 | 5 | 71% | 68% | 72% | 0. 752 | 0 | 15 | 75% | 68% | 76% | 0. 732 |
| 1 | | 81% | 74% | 83% | 0. 869 | 1 | | 85% | 74% | 87% | 0. 884 |
| 2 | | 88% | 89% | 88% | 0. 931 | 2 | | 71% | 53% | 75% | 0. 669 |
| 3 | | 80% | 74% | 81% | 0. 867 | 3 | | 64% | 79% | 62% | 0. 769 |
| 4 | | 65% | 79% | 63% | 0. 781 | 4 | | 89% | 79% | 91% | 0. 902 |
| 5 | | 64% | 89% | 60% | 0. 762 | 5 | | 94% | 79% | 97% | 0. 984 |
| 6 | | 68% | 79% | 66% | 0. 772 | 6 | | 89% | 74% | 92% | 0. 885 |
| 7 | | 84% | 84% | 84% | 0. 885 | 7 | | 87% | 63% | 92% | 0. 856 |
| 8 | | 81% | 74% | 82% | 0. 902 | 8 | | 86% | 79% | 87% | 0. 858 |
| 9 | | 83% | 89% | 82% | 0. 888 | 9 | | 90% | 74% | 93% | 0. 875 |
| 10 | | 58% | 79% | 55% | 0. 658 | 10 | | 88% | 68% | 92% | 0. 927 |
| 11 | | 88% | 89% | 88% | 0. 949 | 11 | | 87% | 84% | 88% | 0. 890 |
| 12 | | 79% | 74% | 80% | 0. 834 | 12 | | 87% | 79% | 89% | 0. 877 |
| 13 | | 73% | 79% | 72% | 0. 778 | 13 | | 79% | 74% | 80% | 0. 778 |
| 14 | | 66% | 74% | 65% | 0. 671 | 14 | | 92% | 68% | 96% | 0. 846 |
| 15 | | 63% | 63% | 63% | 0. 704 | 15 | | 89% | 74% | 92% | 0. 908 |
| 16 | | 58% | 79% | 55% | 0. 696 | 16 | | 92% | 74% | 95% | 0. 965 |
| 17 | | 78% | 84% | 77% | 0. 835 | 17 | | 92% | 79% | 95% | 0. 823 |
| 18 | | 68% | 79% | 66% | 0. 676 | 18 | | 91% | 79% | 93% | 0. 961 |
| 19 | | 80% | 95% | 77% | 0. 903 | 19 | | 87% | 79% | 89% | 0. 863 |
| 0 | 10 | 64% | 68% | 64% | 0. 694 | 0 | 20 | 91% | 79% | 93% | 0. 932 |
| 1 | | 77% | 68% | 79% | 0. 797 | 1 | | 91% | 84% | 92% | 0. 934 |
| 2 | | 74% | 74% | 74% | 0. 766 | 2 | | 90% | 84% | 91% | 0. 918 |
| 3 | | 85% | 89% | 84% | 0. 923 | 3 | | 82% | 74% | 84% | 0. 811 |
| 4 | | 69% | 74% | 68% | 0. 745 | 4 | | 90% | 79% | 92% | 0. 889 |
| 5 | | 83% | 89% | 82% | 0. 810 | 5 | | 76% | 58% | 80% | 0. 829 |
| 6 | | 90% | 79% | 92% | 0. 920 | 6 | | 74% | 47% | 79% | 0. 712 |
| 7 | | 68% | 63% | 69% | 0. 687 | 7 | | 89% | 79% | 91% | 0. 918 |
| 8 | | 86% | 74% | 89% | 0. 897 | 8 | | 79% | 58% | 83% | 0. 833 |
| 9 | | 91% | 74% | 94% | 0. 938 | 9 | | 87% | 63% | 92% | 0. 838 |
| 10 | | 86% | 79% | 87% | 0. 853 | 10 | | 92% | 89% | 92% | 0. 867 |
| 11 | | 86% | 74% | 89% | 0. 834 | 11 | | 86% | 79% | 88% | 0. 891 |
| 12 | | 86% | 74% | 88% | 0. 797 | 12 | | 90% | 74% | 93% | 0. 930 |
| 13 | | 81% | 74% | 82% | 0. 838 | 13 | | 92% | 84% | 93% | 0. 869 |
| 14 | | 80% | 84% | 79% | 0. 811 | 14 | | 80% | 84% | 79% | 0. 888 |
| 15 | | 81% | 74% | 83% | 0. 831 | 15 | | 97% | 89% | 98% | 0. 946 |
| 16 | | 83% | 79% | 84% | 0. 814 | 16 | | 82% | 68% | 85% | 0. 821 |
| 17 | | 90% | 95% | 89% | 0. 957 | 17 | | 86% | 74% | 88% | 0. 863 |
| 18 | | 80% | 68% | 82% | 0. 806 | 18 | | 93% | 79% | 96% | 0. 940 |
| 19 | | 89% | 74% | 92% | 0. 805 | 19 | | 81% | 53% | 86% | 0. 770 |

| | | | | | | | | | | | |
|---|---|---|---|---|---|---|---|---|---|---|---|
| ^{∗}A represents the number of miRNAs used for prediction, B represents Accuracy, C represents Sensitivity, D represents Specificity, and E represents AUC. | | | | | | | | | | | |

### [Table 16-2]

**Table 16-2: Summary of Prediction of Thyroid Cancer based on the Expression of Randomly Picked miRNA**

| | A | B | C | D | E |
|---|---|---|---|---|---|
| Average | 5 | 74% | 80% | 73% | 0. 806 |
| Standard | | 9% | 8% | 11% | 0. 093 |
| Minimum Value | | 58% | 63% | 55% | 0. 658 |
| Third Ouartile | | 66% | 74% | 64% | 0. 740 |
| Average | | 75% | 79% | 74% | 0. 808 |
| First Ouartile | | 81% | 86% | 82% | 0. 885 |
| Maximum Value | | 88% | 95% | 88% | 0. 949 |
| Average | 10 | 81% | 76% | 82% | 0. 826 |
| Standard | | 8% | 8% | 8% | 0. 074 |
| Minimum Value | | 64% | 63% | 64% | 0. 687 |
| Third Ouartile | | 79% | 74% | 79% | 0. 797 |
| Average | | 83% | 74% | 83% | 0. 812 |
| First Ouartile | | 86% | 79% | 89% | 0. 864 |
| Maximum Value | | 91% | 95% | 94% | 0. 957 |
| Average | 15 | 86% | 74% | 88% | 0. 863 |
| Standard | | 8% | 7% | 9% | 0. 079 |
| Minimum Value | | 64% | 53% | 62% | 0. 669 |
| Third Ouartile | | 85% | 72% | 87% | 0. 841 |
| Average | | 88% | 74% | 91% | 0. 876 |
| First Ouartile | | 90% | 79% | 93% | 0. 904 |
| Maximum Value | | 94% | 84% | 97% | 0. 984 |
| Average | 20 | 86% | 74% | 89% | 0. 870 |
| Standard | | 6% | 12% | 6% | 0. 062 |
| Minimum Value | | 74% | 47% | 79% | 0. 712 |
| Third Ouartile | | 82% | 67% | 85% | 0. 832 |
| Average | | 88% | 79% | 91% | 0. 878 |
| First Ouartile | | 91% | 84% | 92% | 0. 921 |
| Maximum Value | | 97% | 89% | 98% | 0. 946 |

| | | | | | |
|---|---|---|---|---|---|
| ^{∗}A represents the number of miRNAs used for prediction, B represents Accuracy, C represents Sensitivity, D represents Specificity, and E represents AUC. | | | | | |

### [Table 17-1]

**Table 17-1: Prediction of Cervical Cancer based on the Expression of Randomly Picked miRNA**

| Trial | A | B | C | D | E | Trial | A | B | C | D | E |
|---|---|---|---|---|---|---|---|---|---|---|---|
| 0 | 5 | 61% | 73% | 57% | 0. 667 | 0 | 15 | 74% | 80% | 72% | 0. 856 |
| 1 | | 59% | 80% | 53% | 0. 663 | 1 | | 62% | 70% | 60% | 0. 730 |
| 2 | | 73% | 70% | 74% | 0. 730 | 2 | | 66% | 73% | 64% | 0. 799 |
| 3 | | 65% | 83% | 60% | 0. 673 | 3 | | 86% | 67% | 92% | 0. 830 |
| 4 | | 74% | 67% | 76% | 0. 783 | 4 | | 75% | 53% | 82% | 0. 754 |
| 5 | | 78% | 87% | 75% | 0. 774 | 5 | | 68% | 53% | 73% | 0. 719 |
| 6 | | 72% | 77% | 71% | 0. 818 | 6 | | 78% | 77% | 79% | 0. 799 |
| 7 | | 79% | 77% | 80% | 0. 827 | 7 | | 67% | 57% | 70% | 0. 667 |
| 8 | | 78% | 50% | 87% | 0. 783 | 8 | | 76% | 63% | 80% | 0. 801 |
| 9 | | 71% | 83% | 67% | 0. 780 | 9 | | 78% | 83% | 76% | 0. 781 |
| 10 | | 79% | 83% | 78% | 0. 836 | 10 | | 85% | 83% | 86% | 0. 851 |
| 11 | | 78% | 60% | 83% | 0. 787 | 11 | | 82% | 67% | 86% | 0. 848 |
| 12 | | 65% | 80% | 60% | 0. 714 | 12 | | 82% | 80% | 83% | 0. 839 |
| 13 | | 69% | 70% | 69% | 0. 694 | 13 | | 72% | 80% | 70% | 0. 794 |
| 14 | | 78% | 73% | 80% | 0. 844 | 14 | | 82% | 67% | 87% | 0. 804 |
| 15 | | 85% | 63% | 92% | 0. 802 | 15 | | 72% | 73% | 71% | 0. 816 |
| 16 | | 74% | 80% | 72% | 0. 830 | 16 | | 66% | 63% | 67% | 0. 787 |
| 17 | | 85% | 60% | 92% | 0. 801 | 17 | | 84% | 83% | 84% | 0. 869 |
| 18 | | 86% | 77% | 89% | 0. 845 | 18 | | 77% | 70% | 79% | 0. 810 |
| 19 | | 78% | 83% | 76% | 0. 840 | 19 | | 78% | 80% | 78% | 0. 828 |
| 0 | 10 | 77% | 77% | 77% | 0. 838 | 0 | 20 | 67% | 70% | 66% | 0. 757 |
| 1 | | 75% | 70% | 77% | 0. 808 | 1 | | 77% | 77% | 77% | 0. 799 |
| 2 | | 74% | 63% | 77% | 0. 826 | 2 | | 80% | 73% | 82% | 0. 872 |
| 3 | | 75% | 63% | 78% | 0. 752 | 3 | | 76% | 67% | 79% | 0. 766 |
| 4 | | 81% | 73% | 83% | 0. 809 | 4 | | 81% | 77% | 82% | 0. 892 |
| 5 | | 69% | 80% | 66% | 0. 798 | 5 | | 72% | 70% | 73% | 0. 727 |
| 6 | | 82% | 73% | 84% | 0. 827 | 6 | | 83% | 77% | 85% | 0. 835 |
| 7 | | 72% | 63% | 75% | 0. 783 | 7 | | 83% | 70% | 87% | 0. 845 |
| 8 | | 74% | 73% | 74% | 0. 738 | 8 | | 72% | 70% | 73% | 0. 753 |
| 9 | | 72% | 67% | 74% | 0. 765 | 9 | | 84% | 77% | 86% | 0. 836 |
| 10 | | 81% | 73% | 83% | 0. 839 | 10 | | 75% | 73% | 76% | 0. 852 |
| 11 | | 78% | 67% | 82% | 0. 777 | 11 | | 75% | 77% | 75% | 0. 827 |
| 12 | | 79% | 63% | 84% | 0. 812 | 12 | | 77% | 70% | 79% | 0. 813 |
| 13 | | 78% | 77% | 78% | 0. 843 | 13 | | 75% | 77% | 74% | 0. 810 |
| 14 | | 75% | 77% | 75% | 0. 812 | 14 | | 69% | 67% | 70% | 0. 723 |
| 15 | | 75% | 70% | 77% | 0. 793 | 15 | | 88% | 83% | 89% | 0. 884 |
| 16 | | 79% | 83% | 78% | 0. 878 | 16 | | 72% | 70% | 73% | 0. 797 |
| 17 | | 58% | 63% | 56% | 0. 689 | 17 | | 85% | 80% | 86% | 0. 851 |
| 18 | | 74% | 70% | 75% | 0. 811 | 18 | | 71% | 77% | 69% | 0. 800 |
| 19 | | 82% | 77% | 84% | 0. 839 | 19 | | 75% | 77% | 74% | 0. 743 |

| | | | | | | | | | | | |
|---|---|---|---|---|---|---|---|---|---|---|---|
| ^{∗}A represents the number of miRNAs used for prediction, B represents Accuracy, C represents Sensitivity, D represents Specificity, and E represents AUC. | | | | | | | | | | | |

### [Table 17-2]

**Table 17-2: Summary of Prediction of Cervical Cancer based on the Expression of Randomly Picked miRNA**

| | A | B | C | D | E |
|---|---|---|---|---|---|
| Average | 5 | 74% | 74% | 75% | 0. 775 |
| Standard | | 8% | 10% | 11% | 0. 062 |
| Minimum Value | | 59% | 50% | 53% | 0. 663 |
| Third Ouartile | | 70% | 69% | 68% | 0. 726 |
| Average | | 76% | 77% | 76% | 0. 785 |
| First Ouartile | | 79% | 81% | 81% | 0. 827 |
| Maximum Value | | 86% | 87% | 92% | 0. 845 |
| Average | 10 | 76% | 71% | 77% | 0. 802 |
| Standard | | 5% | 6% | 7% | 0. 043 |
| Minimum Value | | 58% | 63% | 56% | 0. 689 |
| Third Ouartile | | 74% | 66% | 75% | 0. 781 |
| Average | | 75% | 72% | 77% | 0. 810 |
| First Ouartile | | 79% | 77% | 82% | 0. 830 |
| Maximum Value | | 82% | 83% | 84% | 0. 878 |
| Average | 15 | 76% | 71% | 77% | 0. 799 |
| Standard | | 7% | 10% | 8% | 0. 050 |
| Minimum Value | | 62% | 53% | 60% | 0. 667 |
| Third Ouartile | | 71% | 66% | 71% | 0. 785 |
| Average | | 77% | 72% | 79% | 0. 803 |
| First Ouartile | | 82% | 80% | 83% | 0. 832 |
| Maximum Value | | 86% | 83% | 92% | 0. 869 |
| Average | 20 | 77% | 74% | 78% | 0. 809 |
| Standard | | 6% | 4% | 7% | 0. 051 |
| Minimum Value | | 67% | 67% | 66% | 0. 723 |
| Third Ouartile | | 72% | 70% | 73% | 0. 764 |
| Average | | 76% | 75% | 77% | 0. 812 |
| First Ouartile | | 81% | 77% | 83% | 0. 846 |
| Maximum Value | | 88% | 83% | 89% | 0. 892 |

| | | | | | |
|---|---|---|---|---|---|
| ^{∗}A represents the number of miRNAs used for prediction, B represents Accuracy, C represents Sensitivity, D represents Specificity, and E represents AUC. | | | | | |

### [Table 18-1]

**Table 18-1: Prediction of Rectal Cancer based on the Expression of Randomly Picked miRNA**

| Trial | A | B | C | D | E | Trial | A | B | C | D | E |
|---|---|---|---|---|---|---|---|---|---|---|---|
| 0 | 5 | 65% | 70% | 63% | 0. 739 | 0 | 15 | 72% | 63% | 74% | 0. 730 |
| 1 | | 66% | 83% | 61% | 0. 734 | 1 | | 66% | 70% | 65% | 0. 729 |
| 2 | | 75% | 77% | 74% | 0. 743 | 2 | | 72% | 70% | 72% | 0. 775 |
| 3 | | 64% | 80% | 59% | 0. 657 | 3 | | 76% | 67% | 79% | 0. 780 |
| 4 | | 66% | 57% | 69% | 0. 611 | 4 | | 62% | 67% | 60% | 0. 693 |
| 5 | | 74% | 73% | 74% | 0. 709 | 5 | | 83% | 83% | 83% | 0. 861 |
| 6 | | 68% | 70% | 68% | 0. 709 | 6 | | 72% | 67% | 73% | 0. 784 |
| 7 | | 63% | 63% | 63% | 0. 744 | 7 | | 73% | 73% | 73% | 0. 811 |
| 8 | | 58% | 63% | 56% | 0. 631 | 8 | | 68% | 80% | 65% | 0. 763 |
| 9 | | 72% | 63% | 74% | 0. 693 | 9 | | 70% | 73% | 69% | 0. 739 |
| 10 | | 68% | 63% | 69% | 0. 754 | 10 | | 63% | 67% | 62% | 0. 756 |
| 11 | | 81% | 70% | 84% | 0. 733 | 11 | | 70% | 77% | 68% | 0. 790 |
| 12 | | 73% | 70% | 74% | 0. 800 | 12 | | 70% | 70% | 70% | 0. 780 |
| 13 | | 65% | 73% | 62% | 0. 765 | 13 | | 76% | 67% | 79% | 0. 791 |
| 14 | | 75% | 70% | 76% | 0. 807 | 14 | | 76% | 70% | 78% | 0. 802 |
| 15 | | 76% | 67% | 79% | 0. 783 | 15 | | 65% | 57% | 68% | 0. 657 |
| 16 | | 62% | 80% | 57% | 0. 737 | 16 | | 71% | 70% | 71% | 0. 809 |
| 17 | | 73% | 80% | 71% | 0. 771 | 17 | | 68% | 63% | 69% | 0. 776 |
| 18 | | 75% | 70% | 76% | 0. 797 | 18 | | 81% | 77% | 82% | 0. 865 |
| 19 | | 72% | 67% | 74% | 0. 692 | 19 | | 64% | 67% | 63% | 0. 711 |
| 0 | 10 | 62% | 63% | 69% | 0. 645 | 0 | 20 | 80% | 70% | 83% | 0. 863 |
| 1 | | 74% | 67% | 76% | 0. 764 | 1 | | 62% | 57% | 64% | 0. 648 |
| 2 | | 76% | 70% | 78% | 0. 824 | 2 | | 68% | 60% | 71% | 0. 739 |
| 3 | | 67% | 70% | 66% | 0. 691 | 3 | | 79% | 67% | 83% | 0. 831 |
| 4 | | 73% | 70% | 74% | 0. 767 | 4 | | 75% | 70% | 77% | 0. 796 |
| 5 | | 72% | 67% | 73% | 0. 717 | 5 | | 75% | 70% | 76% | 0. 790 |
| 6 | | 65% | 73% | 63% | 0. 749 | 6 | | 72% | 67% | 74% | 0. 759 |
| 7 | | 66% | 70% | 65% | 0. 772 | 7 | | 83% | 77% | 85% | 0. 860 |
| 8 | | 71% | 70% | 71% | 0. 749 | 8 | | 80% | 73% | 82% | 0. 850 |
| 9 | | 69% | 67% | 70% | 0. 779 | 9 | | 64% | 53% | 67% | 0. 673 |
| 10 | | 68% | 70% | 68% | 0. 743 | 10 | | 61% | 67% | 59% | 0. 648 |
| 11 | | 82% | 77% | 84% | 0. 889 | 11 | | 70% | 63% | 72% | 0. 690 |
| 12 | | 62% | 70% | 59% | 0. 724 | 12 | | 70% | 67% | 71% | 0. 727 |
| 13 | | 75% | 73% | 76% | 0. 841 | 13 | | 73% | 63% | 76% | 0. 696 |
| 14 | | 71% | 70% | 71% | 0. 820 | 14 | | 62% | 60% | 62% | 0. 657 |
| 15 | | 71% | 60% | 74% | 0. 733 | 15 | | 75% | 70% | 76% | 0. 758 |
| 16 | | 78% | 77% | 78% | 0. 854 | 16 | | 67% | 70% | 66% | 0. 748 |
| 17 | | 72% | 70% | 72% | 0. 759 | 17 | | 75% | 67% | 77% | 0. 800 |
| 18 | | 72% | 73% | 71% | 0. 830 | 18 | | 74% | 67% | 76% | 0. 728 |
| 19 | | 78% | 73% | 80% | 0. 824 | 19 | | 77% | 67% | 80% | 0. 792 |

| | | | | | | | | | | | |
|---|---|---|---|---|---|---|---|---|---|---|---|
| ^{∗}A represents the number of miRNAs used for prediction, B represents Accuracy, C represents Sensitivity, D represents Specificity, and E represents AUC. | | | | | | | | | | | |

### [Table 18-2]

**Table 18-2: Summary of Prediction of Rectal Cancer based on the Expression of Randomly Picked miRNA**

| | A | B | C | D | E |
|---|---|---|---|---|---|
| Average | 5 | 69% | 70% | 69% | 0. 730 |
| Standard | | 6% | 7% | 8% | 0. 054 |
| Minimum Value | | 58% | 57% | 56% | 0. 611 |
| Third Ouartile | | 65% | 66% | 63% | 0. 705 |
| Average | | 70% | 70% | 70% | 0. 738 |
| First Ouartile | | 74% | 74% | 74% | 0. 766 |
| Maximum Value | | 81% | 83% | 84% | 0. 807 |
| Average | 10 | 71% | 70% | 72% | 0. 774 |
| Standard | | 5% | 4% | 6% | 0. 060 |
| Minimum Value | | 62% | 60% | 59% | 0. 645 |
| Third Ouartile | | 68% | 69% | 68% | 0. 740 |
| Average | | 71% | 70% | 72% | 0. 765 |
| First Ouartile | | 74% | 73% | 76% | 0. 824 |
| Maximum Value | | 82% | 77% | 84% | 0. 889 |
| Average | 15 | 71% | 70% | 71% | 0. 770 |
| Standard | | 6% | 6% | 7% | 0. 051 |
| Minimum Value | | 62% | 57% | 60% | 0. 657 |
| Third Ouartile | | 67% | 67% | 67% | 0. 737 |
| Average | | 70% | 70% | 71% | 0. 778 |
| First Ouartile | | 74% | 73% | 75% | 0. 794 |
| Maximum Value | | 83% | 83% | 83% | 0. 865 |
| Average | 20 | 72% | 66% | 74% | 0. 753 |
| Standard | | 7% | 6% | 7% | 0. 070 |
| Minimum Value | | 61% | 53% | 59% | 0. 648 |
| Third Ouartile | | 68% | 63% | 70% | 0. 695 |
| Average | | 73% | 67% | 76% | 0. 753 |
| First Ouartile | | 76% | 70% | 78% | 0. 797 |
| Maximum Value | | 83% | 77% | 85% | 0. 863 |

| | | | | | |
|---|---|---|---|---|---|
| ^{∗}A represents the number of miRNAs used for prediction, B represents Accuracy, C represents Sensitivity, D represents Specificity, and E represents AUC. | | | | | |

### [Table 19-1]

**Table 19-1: Prediction of Endometrial Cancer based on the Expression of Randomly Picked miRNA**

| Trial | A | B | C | D | E | Trial | A | B | C | D | E |
|---|---|---|---|---|---|---|---|---|---|---|---|
| 0 | 5 | 66% | 87% | 55% | 0. 724 | 0 | 15 | 68% | 75% | 64% | 0. 718 |
| 1 | | 64% | 79% | 56% | 0. 653 | 1 | | 71% | 75% | 69% | 0. 752 |
| 2 | | 63% | 74% | 57% | 0. 671 | 2 | | 69% | 74% | 67% | 0. 698 |
| 3 | | 63% | 68% | 61% | 0. 751 | 3 | | 64% | 70% | 61% | 0. 733 |
| 4 | | 67% | 75% | 62% | 0. 752 | 4 | | 64% | 72% | 60% | 0. 647 |
| 5 | | 69% | 75% | 65% | 0. 661 | 5 | | 65% | 72% | 61% | 0. 645 |
| 6 | | 69% | 74% | 67% | 0. 726 | 6 | | 67% | 75% | 63% | 0. 766 |
| 7 | | 70% | 79% | 65% | 0. 681 | 7 | | 65% | 74% | 60% | 0. 717 |
| 8 | | 67% | 83% | 58% | 0. 746 | 8 | | 67% | 75% | 63% | 0. 692 |
| 9 | | 69% | 81% | 62% | 0. 723 | 9 | | 75% | 75% | 75% | 0.804 |
| 10 | | 72% | 66% | 75% | 0. 697 | 10 | | 61% | 79% | 51% | 0. 707 |
| 11 | | 72% | 75% | 70% | 0. 727 | 11 | | 65% | 72% | 61% | 0. 637 |
| 12 | | 65% | 79% | 58% | 0. 714 | 12 | | 70% | 72% | 69% | 0. 655 |
| 13 | | 66% | 72% | 63% | 0. 742 | 13 | | 64% | 70% | 61% | 0. 697 |
| 14 | | 67% | 75% | 62% | 0. 766 | 14 | | 72% | 75% | 70% | 0. 759 |
| 15 | | 60% | 70% | 55% | 0. 594 | 15 | | 61% | 68% | 57% | 0. 694 |
| 16 | | 70% | 74% | 68% | 0. 738 | 16 | | 73% | 77% | 71% | 0. 759 |
| 17 | | 61% | 79% | 51% | 0. 614 | 17 | | 70% | 75% | 67% | 0. 772 |
| 18 | | 71% | 72% | 70% | 0. 729 | 18 | | 62% | 70% | 58% | 0. 673 |
| 19 | | 73% | 75% | 71% | 0. 784 | 19 | | 72% | 75% | 70% | 0. 716 |
| 0 | 10 | 61% | 62% | 61 % | 0, 678 | 0 | 20 | 74% | 75% | 73% | 0. 797 |
| 1 | | 65% | 62% | 66% | 0. 695 | 1 | | 72% | 79% | 68% | 0. 748 |
| 2 | | 67% | 74% | 64% | 0. 785 | 2 | | 58% | 66% | 54% | 0. 691 |
| 3 | | 66% | 68% | 65% | 0. 737 | 3 | | 63% | 58% | 66% | 0. 690 |
| 4 | | 75% | 70% | 77% | 0. 747 | 4 | | 64% | 72% | 60% | 0. 722 |
| 5 | | 62% | 74% | 56% | 0. 703 | 5 | | 69% | 75% | 65% | 0. 732 |
| 6 | | 59% | 70% | 53% | 0. 647 | 6 | | 71% | 72% | 70% | 0. 746 |
| 7 | | 69% | 72% | 68% | 0. 733 | 7 | | 68% | 72% | 66% | 0. 730 |
| 8 | | 75% | 70% | 78% | 0. 738 | 8 | | 68% | 75% | 64% | 0. 703 |
| 9 | | 67% | 81% | 60% | 0. 776 | 9 | | 65% | 68% | 63% | 0. 730 |
| 10 | | 69% | 74% | 67% | 0. 729 | 10 | | 65% | 81% | 57% | 0. 745 |
| 11 | | 59% | 64% | 56% | 0. 653 | 11 | | 75% | 75% | 74% | 0. 814 |
| 12 | | 73% | 79% | 69% | 0. 750 | 12 | | 69% | 75% | 66% | 0. 680 |
| 13 | | 65% | 74% | 60% | 0. 751 | 13 | | 67% | 77% | 62% | 0. 740 |
| 14 | | 66% | 72% | 63% | 0. 646 | 14 | | 67% | 74% | 64% | 0. 776 |
| 15 | | 71% | 77% | 68% | 0. 783 | 15 | | 65% | 75% | 60% | 0. 725 |
| 16 | | 70% | 77% | 66% | 0. 742 | 16 | | 66% | 81% | 58% | 0. 703 |
| 17 | | 68% | 75% | 64% | 0. 728 | 17 | | 67% | 77% | 61% | 0. 678 |
| 18 | | 69% | 75% | 65% | 0. 716 | 18 | | 67% | 77% | 61% | 0. 705 |
| 19 | | 68% | 74% | 65% | 0. 733 | 19 | | 63% | 72% | 58% | 0. 670 |

| | | | | | | | | | | | |
|---|---|---|---|---|---|---|---|---|---|---|---|
| ^{∗}A represents the number of miRNAs used for prediction, B represents Accuracy, C represents Sensitivity, D represents Specificity, and E represents AUC. | | | | | | | | | | | |

### [Table 19-2]

**Table 19-2: Summary of Prediction of Endometrial Cancer based on the Expression of Randomly Picked miRNA**

| | A | B | C | D | E |
|---|---|---|---|---|---|
| Average | 5 | 67% | 76% | 63% | 0. 710 |
| Standard | | 4% | 5% | 6% | 0. 050 |
| Minimum Value | | 60% | 66% | 51% | 0. 594 |
| Third Ouartile | | 65% | 73% | 58% | 0. 679 |
| Average | | 67% | 75% | 62% | 0. 725 |
| First Ouartile | | 70% | 79% | 67% | 0. 743 |
| Maximum Value | | 73% | 87% | 75% | 0. 784 |
| Average | 10 | 67% | 72% | 65% | 0. 724 |
| Standard | | 5% | 5% | 6% | 0. 042 |
| Minimum Value | | 59% | 62% | 53% | 0. 646 |
| Third Ouartile | | 65% | 70% | 61% | 0. 701 |
| Average | | 68% | 74% | 65% | 0. 733 |
| First Ouartile | | 69% | 75% | 67% | 0. 748 |
| Maximum Value | | 75% | 81% | 78% | 0. 785 |
| Average | 15 | 67% | 74% | 64% | 0. 712 |
| Standard | | 4% | 3% | 6% | 0. 047 |
| Minimum Value | | 61% | 68% | 51% | 0. 637 |
| Third Ouartile | | 64% | 72% | 61% | 0. 688 |
| Average | | 67% | 75% | 63% | 0. 711 |
| First Ouartile | | 70% | 75% | 69% | 0. 754 |
| Maximum Value | | 75% | 79% | 75% | 0. 804 |
| Average | 20 | 67% | 74% | 64% | 0. 726 |
| Standard | | 4% | 5% | 5% | 0. 039 |
| Minimum Value | | 58% | 58% | 54% | 0. 670 |
| Third Ouartile | | 65% | 72% | 60% | 0. 700 |
| Average | | 67% | 75% | 64% | 0. 728 |
| First Ouartile | | 69% | 77% | 66% | 0. 745 |
| Maximum Value | | 75% | 81% | 74% | 0. 814 |

| | | | | | |
|---|---|---|---|---|---|
| ^{∗}A represents the number of miRNAs used for prediction, B represents Accuracy, C represents Sensitivity, D represents Specificity, and E represents AUC. | | | | | |

### [Table 20]

**Table 20: List of miRNAs used for Random Prediction based on Expression Levels of n miRNAs**

| miRNA 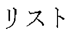 |
|---|
| hsa-miR-671-5p |
| hsa-miR-4520-3p |
| hsa-miR-4307 |
| hsa-miR-6760-5p |
| hsa-miR-708-5p |
| hsa-miR-450a-2-3p |
| hsa-miR -30e-3p |
| hsa-miR-2276-5p |
| hsa-miR-4739 |

### [Table 21]

**Table 21: Prediction Accuracy of Human Lung Cancer by Logistic Regression Model**

| n | Accuracy (Max) | Accuracy (Min) | Accuracy(Mean) | Accuracy(std) |
|---|---|---|---|---|
| 1 | 73.3% | 51.5% | 65.0% | 6.9% |
| 2 | 86.4% | 55.1% | 72.2% | 7.0% |
| 3 | 89.4% | 65.2% | 78.4% | 6.2% |
| 4 | 90.9% | 65.7% | 82.6% | 5.7% |
| 5 | 92.4% | 70.2% | 85.8% | 4.5% |
| 6 | 92.9% | 70.2% | 88.1% | 3.5% |
| 7 | 93.4% | 83.8% | 89.9% | 2.2% |
| 8 | 93.9% | 89.4% | 91.9% | 1.7% |
| 9 | 92.9% | 92.9% | 92.9% | 0.0% |

### [Table 22-1]

**Table 22-1: miRNA to Predict Human Lung Cancer of Stage I**

| miRNA Name | A | miRNA Name | A | miRNA Name | A |
|---|---|---|---|---|---|
| let-7b-5p | 75% | miR-371a-3p | 54% | miR-516b-5p | 92% |
| let-7e-5p | 54% | miR-371a-5p | 50% | miR-517-5p | 67% |
| let-7f-2-3p | 50% | miR-372-3p | 50% | miR-5189-3p | 92% |
| let-7g-3p | 79% | miR-372-5p | 88% | miR-5189-5p | 100% |
| miR-103a-3p | 58% | miR-374b-5p | 63% | miR-518b | 50% |
| miR-106b-5p | 92% | miR-375 | 75% | miR-5192 | 71% |
| miR-107 | 83% | miR-376c-3p | 100% | miR-519a-3p | 75% |
| miR-10a-5p | 79% | miR-378b | 83% | miR-519b-3p | 67% |
| miR-10b-5p | 75% | miR-378e | 58% | miR-519c-5p, miR-523-5p, miR-518e-5p, miR-522-5p, miR-519a-5p, miR-519b-5p | 71% |
| miR-1178-3p | 63% | miR-378i | 79% | miR-520b | 63% |
| miR-1180-5p | 71% | miR-380-5p | 92% | miR-520d-3p | 75% |
| miR-1183 | 83% | miR-3907 | 83% | miR-520d-5p | 63% |
| miR-1185-1-3p | 71% | miR-3912-5p | 96% | miR-520e | 58% |
| miR-1193 | 79% | miR-3917 | 96% | miR-520f-3p | 88% |
| miR-1199-5p | 83% | miR-3922-5p | 100% | miR-526b-5p | 75% |
| miR-1200 | 50% | miR-3927-5p | 88% | miR-527, miR-518a-5p | 54% |
| miR-1205 | 50% | miR-3928-3p | 58% | miR-539-5p | 75% |
| miR-122-3p | 63% | miR-3934-5p | 100% | miR-542-5p | 92% |
| miR-1224-5p | 100% | miR-3935 | 92% | miR-548ba | 67% |
| miR-1226-5p | 79% | miR-3944-3p | 100% | miR-551b-5p | 63% |
| miR-1238-5p | 92% | miR-3960 | 58% | miR-552-3p | 63% |
| miR-1247-3p | 58% | miR-3976 | 92% | miR-5580-5p | 83% |
| miR-1250-5p | 96% | miR-3978 | 83% | miR-5581-5p | 71% |
| miR-1254 | 63% | miR-424-3p | 67% | miR-5588-3p | 92% |
| miR-1257 | 67% | miR-4252 | 83% | miR-5588-5p | 79% |
| miR-125a-3p | 67% | miR-425-5p | 79% | miR-5589-3p | 83% |
| miR-1263 | 79% | miR-4257 | 67% | miR-5589-5p | 96% |
| miR-1265 | 88% | miR-4259 | 83% | miR-563 | 79% |
| miR-1271-5p | 96% | miR-4260 | 96% | miR-564 | 92% |
| miR-1272 | 75% | miR-4262 | 79% | miR-566 | 54% |
| miR-1273a | 63% | miR-4266 | 50% | miR-5684 | 75% |
| miR-1273f | 92% | miR-4269 | 92% | miR-5696 | 58% |
| miR-1273g-3p | 92% | miR-4273 | 92% | miR-5698 | 92% |
| miR-1273g-5p | 96% | miR-4285 | 58% | miR-5701 | 92% |
| miR-1273h-5p | 92% | miR-429 | 88% | miR-5702 | 54% |
| miR-1275 | 63% | miR-4293 | 71% | miR-5708 | 92% |
| miR-1276 | 83% | miR-4296 | 96% | miR-576-5p | 54% |
| miR-1282 | 92% | miR-4297 | 83% | miR-580-5p | 100% |
| miR-128-2-5p | 67% | miR-4300 | 83% | miR-582-3p | 96% |
| miR-1289 | 71% | miR-4304 | 79% | miR-585-3p | 88% |
| miR-1293 | 100% | miR-4306 | 83% | miR-595 | 83% |
| miR-1294 | 63% | miR-4307 | 83% | miR-597-3p | 79% |
| miR-1297 | 50% | miR-431-3p | 83% | miR-598-3p | 54% |
| miR-1298-5p | 75% | miR-4314 | 67% | miR-603 | 92% |
| miR-1306-3p | 92% | miR-431-5p | 67% | miR-605-3p | 92% |
| miR-1307-3p | 92% | miR-4320 | 88% | miR-6068 | 83% |
| miR-1321 | 79% | miR-4321 | 92% | miR-6071 | 54% |
| miR-1322 | 88% | miR-4325 | 75% | miR-6072 | 92% |
| miR-132-3p | 83% | miR-433-3p | 71% | miR-6076 | 88% |
| miR-133a-3p | 58% | miR-4418 | 100% | miR-608 | 100% |
| miR-134-5p | 54% | miR-4419a | 75% | miR-6082 | 92% |
| miR-135b-5p | 63% | miR-4419b | 92% | miR-6086 | 67% |
| miR-138-1-3p | 88% | miR-4420 | 88% | miR-611 | 88% |
| miR-140-5p | 92% | miR-4422 | 92% | miR-6126 | 50% |
| miR-143-5p | 92% | miR-4423-5p | 75% | miR-6127 | 83% |
| miR-1468-5p | 96% | miR-4428 | 100% | miR-6129 | 92% |
| miR-1470 | 50% | miR-4430 | 96% | miR-6131 | 83% |
| miR-1471 | 96% | miR-4436a | 92% | miR-6133 | 63% |
| miR-147b | 100% | miR-4436b-3p | 100% | miR-614 | 71% |
| miR-148a-5p | 96% | miR-4437 | 96% | miR-616-3p | 75% |
| miR-150-3p | 71% | miR-4440 | 100% | miR-6165 | 50% |
| miR-150-5p | 54% | miR-4441 | 58% | miR-616-5p | 79% |
| miR-1587 | 92% | miR-4443 | 88% | miR-622 | 92% |
| miR-15a-3p | 54% | miR-4445-3p | 92% | miR-624-3p | 83% |
| miR-15b-3p | 75% | miR-4447 | 96% | miR-624-5p | 83% |
| miR-1-5p | 50% | miR-4448 | 96% | miR-626 | 88% |
| miR-16-5p | 88% | miR-4449 | 92% | miR-628-3p | 67% |
| miR-17-5p | 83% | miR-4451 | 96% | miR-630 | 58% |
| miR-181a-5p | 63% | miR-4453 | 96% | miR-6499-5p | 88% |
| miR-181d-3p | 58% | miR-4458 | 96% | miR-650 | 75% |
| miR-181d-5p | 63% | miR-4459 | 71% | miR-6500-3p | 96% |
| miR-182-5p | 92% | miR-4462 | 79% | miR-6503-3p | 71% |
| miR-185-3p | 83% | miR-4470 | 71% | miR-6504-5p | 83% |
| miR-185-5p | 75% | miR-4472 | 92% | miR-6514-3p | 96% |
| miR-187-3p | 88% | miR-4474-3p | 96% | miR-6515-5p | 79% |
| miR-1908-3p | 71% | miR-4478 | 79% | miR-6516-5p | 67% |
| miR-1909-5p | 96% | miR-4479 | 79% | miR-653-5p | 75% |
| miR-1910-3p | 100% | miR-448 | 67% | miR-655-5p | 83% |
| miR-1911-5p | 79% | miR-4481 | 96% | miR-656-3p | 75% |
| miR-1914-3p | 67% | miR-4483 | 88% | miR-656-5p | 96% |
| miR-193a-5p | 96% | miR-4486 | 88% | miR-659-5p | 75% |
| miR-193b-3p | 50% | miR-4487 | 88% | miR-660-5p | 88% |
| miR-194-3p | 92% | miR-4489 | 100% | miR-661 | 88% |
| miR-195-3p | 50% | miR-4492 | 50% | miR-662 | 92% |
| miR-195-5p | 92% | miR-4496 | 75% | miR-668-5p | 83% |
| miR-196a-5p | 63% | miR-4498 | 96% | miR-6715a-3p | 63% |
| miR-1972 | 88% | miR-4499 | 58% | miR-6715b-3p | 100% |
| miR-197-5p | 88% | miR-449c-5p | 83% | miR-671-5p | 50% |
| miR-198 | 58% | miR-4501 | 96% | miR-6723-5p | 75% |
| miR-200c-3p | 88% | miR-4502 | 88% | miR-6726-5p | 88% |
| miR-203b-5p | 88% | miR-4503 | 100% | miR-6730-5p | 71% |
| miR-2052 | 100% | miR-4507 | 83% | miR-6734-3p | 50% |
| miR-205-5p | 67% | miR-450a-2-3p | 79% | miR-6736-5p | 88% |
| miR-208a-5p | 50% | miR-4514 | 96% | miR-6738-3p | 88% |
| miR-20a-3p | 75% | miR-4515 | 75% | miR-6741-5p | 79% |
| miR-20b-5p | 71% | miR-4518 | 96% | miR-6745 | 63% |
| miR-2114-5p | 96% | miR-4519 | 58% | miR-6746-5p | 88% |
| miR-212-5p | 92% | miR-4520-3p | 83% | miR-6747-5p | 100% |
| miR-215-3p | 96% | miR-4520-5p | 96% | miR-6751-5p | 100% |
| miR-216a-5p | 96% | miR-452-3p | 71% | miR-6758-5p | 67% |
| miR-219a-2-3p | 63% | miR-4524a-3p | 75% | miR-6759-5p | 92% |
| miR-222-3p | 88% | miR-4525 | 100% | miR-6760-5p | 88% |
| miR-223-5p | 54% | miR-4529-3p | 96% | miR-6766-5p | 88% |
| miR-22-5p | 92% | miR-4529-5p | 88% | miR-6767-5p | 88% |
| miR-2276-3p | 92% | miR-4533 | 100% | miR-6768-3p | 54% |
| miR-2277-3p | 79% | miR-4535 | 92% | miR-6769a-5p | 50% |
| miR-23a-3p | 63% | miR-4538 | 100% | miR-6772-5p | 96% |
| miR-23b-5p | 79% | miR-4539 | 96% | miR-6773-5p | 92% |
| miR-2467-3p | 100% | miR-4540 | 92% | miR-6776-5p | 100% |
| miR-2681-3p | 92% | miR-4635 | 96% | miR-6777-5p | 79% |
| miR-27a-3p | 83% | miR-4638-5p | 96% | miR-6778-5p | 92% |
| miR-2861 | 63% | miR-4639-3p | 92% | miR-6780a-5p | 75% |
| miR-296-3p | 83% | miR-4643 | 50% | miR-6788-5p | 92% |
| miR-297 | 96% | miR-4644 | 96% | miR-6790-5p | 88% |
| miR-298 | 54% | miR-4646-5p | 96% | miR-6793-5p | 92% |
| miR-29a-3p | 75% | miR-4647 | 100% | miR-6794-5p | 96% |
| miR-29b-1-5p | 67% | miR-4650-3p | 88% | miR-6795-3p | 50% |
| miR-29b-2-5p | 63% | miR-4654 | 92% | miR-6795-5p | 63% |
| miR-302c-5p | 83% | miR-4657 | 100% | miR-6796-5p | 75% |
| miR-3064-3p | 92% | miR-4659a-3p | 88% | miR-6798-3p | 50% |
| miR-3065-5p | 54% | miR-466 | 88% | miR-6799-5p | 50% |
| miR-3074-5p | 79% | miR-4661-5p | 54% | miR-6801-5p | 96% |
| miR-30a-5p | 83% | miR-4665-5p | 88% | miR-6809-5p | 83% |
| miR-30e-3p | 63% | miR-4669 | 96% | miR-6812-5p | 58% |
| miR-3120-5p | 96% | miR-4675 | 54% | miR-6814-5p | 63% |
| miR-3121-5p | 100% | miR-4676-5p | 100% | miR-6815-5p | 75% |
| miR-3122 | 92% | miR-4677-5p | 67% | miR-6817-5p | 92% |
| miR-3124-5p | 63% | miR-4681 | 92% | miR-6820-5p | 83% |
| miR-3126-5p | 96% | miR-4683 | 88% | miR-6821-3p | 67% |
| miR-3127-3p | 50% | miR-4684-5p | 92% | miR-6823-5p | 92% |
| miR-3130-5p | 92% | miR-4686 | 100% | miR-6827-5p | 83% |
| miR-3137 | 96% | miR-4690-5p | 96% | miR-6828-5p | 96% |
| miR-3138 | 100% | miR-4691-3p | 67% | miR-6830-5p | 67% |
| miR-3145-5p | 92% | miR-4691-5p | 100% | miR-6833-5p | 96% |
| miR-3149 | 88% | miR-4692 | 88% | miR-6834-5p | 96% |
| miR-3151-5p | 83% | miR-4694-5p | 58% | miR-6842-5p | 83% |
| miR-3153 | 79% | miR-4699-3p | 92% | miR-6847-5p | 83% |
| miR-3156-5p | 83% | miR-4700-5p | 100% | miR-6849-5p | 100% |
| miR-3157-5p | 79% | miR-4701-3p | 96% | miR-6859-5p | 100% |
| miR-3160-3p | 79% | miR-4704-5p | 58% | miR-6861-5p | 83% |
| miR-3160-5p | 100% | miR-4706 | 71% | miR-6864-5p | 79% |
| miR-3162-3p | 50% | miR-4708-3p | 96% | miR-6868-5p | 96% |
| miR-3163 | 54% | miR-4709-3p | 100% | miR-6869-3p | 63% |
| miR-3164 | 79% | miR-4710 | 96% | miR-6871-5p | 92% |
| miR-3174 | 92% | miR-4711-3p | 96% | miR-6872-5p | 75% |
| miR-3176 | 96% | miR-4717-3p | 79% | miR-6876-5p | 96% |
| miR-3177-3p | 88% | miR-4719 | 88% | miR-6877-5p | 92% |
| miR-3177-5p | 88% | miR-4721 | 100% | miR-6881-5p | 79% |
| miR-3181 | 67% | miR-4724-3p | 58% | miR-6884-5p | 96% |
| miR-3182 | 92% | miR-4724-5p | 58% | miR-6885-5p | 67% |
| miR-3184-5p | 50% | miR-4727-3p | 92% | miR-6887-5p | 63% |
| miR-3186-3p | 92% | miR-4732-3p | 88% | miR-6892-5p | 100% |
| miR-3187-3p | 92% | miR-4739 | 54% | miR-6893-5p | 63% |
| miR-3187-5p | 100% | miR-4743-5p | 96% | miR-6894-5p | 88% |
| miR-3188 | 71% | miR-4746-3p | 75% | miR-7106-5p | 63% |
| miR-3189-3p | 83% | miR-4747-3p | 63% | miR-7151-3p | 100% |
| miR-3192-5p | 83% | miR-4747-5p | 75% | miR-7153-5p | 79% |
| miR-320c | 54% | miR-4750-3p | 58% | miR-7154-5p | 100% |
| miR-323a-5p | 96% | miR-4754 | 92% | miR-7157-5p | 92% |
| miR-326 | 50% | miR-4756-3p | 96% | miR-7158-3p | 67% |
| miR-328-5p | 50% | miR-4762-5p | 54% | miR-7160-5p | 100% |
| miR-329-3p | 71% | miR-4767 | 79% | miR-7162-5p | 63% |
| miR-337-5p | 67% | miR-4768-3p | 79% | miR-718 | 88% |
| miR-33a-3p | 63% | miR-4769-5p | 100% | miR-758-3p | 100% |
| miR-34a-3p | 96% | miR-4774-3p | 79% | miR-758-5p | 92% |
| miR-34a-5p | 83% | miR-4776-3p | 83% | miR-766-5p | 100% |
| miR-3591-3p | 100% | miR-4776-5p | 100% | miR-7706 | 54% |
| miR-3591-5p | 75% | miR-4781-5p | 67% | miR-7843-3p | 92% |
| miR-3605-5p | 79% | miR-4784 | 96% | miR-7850-5p | 96% |
| miR-3612 | 79% | miR-4785 | 92% | miR-7851-3p | 96% |
| miR-3613-3p | 63% | miR-4787-5p | 50% | miR-7975 | 83% |
| miR-361-5p | 79% | miR-4796-5p | 88% | miR-8058 | 92% |
| miR-3616-3p | 63% | miR-4799-5p | 88% | miR-8064 | 96% |
| miR-3619-3p | 83% | miR-4800-5p | 83% | miR-8071 | 92% |
| miR-3622a-5p | 92% | miR-4804-5p | 79% | miR-8075 | 92% |
| miR-3622b-5p | 88% | miR-487a-5p | 83% | miR-8077 | 100% |
| miR-362-5p | 100% | miR-491-3p | 50% | miR-8079 | 67% |
| miR-363-3p | 79% | miR-494-5p | 88% | miR-8082 | 88% |
| miR-3652 | 79% | miR-4999-5p | 71% | miR-8083 | 92% |
| miR-3653-3p | 83% | miR-5002-3p | 96% | miR-8085 | 92% |
| miR-3654 | 75% | miR-5002-5p | 67% | miR-8086 | 50% |
| miR-3655 | 100% | miR-5006-5p | 88% | miR-877-5p | 96% |
| miR-3659 | 88% | miR-5007-5p | 75% | miR-891a-3p | 71% |
| miR-365a-3p, miR-365b-3p | 54% | miR-500a-5p | 54% | miR-891a-5p | 96% |
| miR-3663-5p | 96% | miR-5010-5p | 75% | miR-892c-3p | 50% |
| miR-3665 | 50% | miR-5011-5p | 92% | miR-921 | 96% |
| miR-3681-5p | 96% | miR-504-3p | 88% | miR-922 | 96% |
| miR-3689a-3p | 100% | miR-505-5p | 96% | miR-92b-3p | 50% |
| miR-3689a-5p, miR-3689b-5p, miR-3689e | 88% | miR-5089-3p | 83% | miR-933 | 50% |
| miR-3689b-3p, miR-3689c | 96% | miR-5089-5p | 88% | miR-93-3p | 75% |
| miR-3691-5p | 96% | miR-5092 | 88% | miR-936 | 67% |
| miR-370-3p | 63% | miR-5093 | 100% | miR-96-3p | 75% |
| miR-370-5p | 58% | miR-509-5p | 92% | miR-96-5p | 88% |
| miR-3713 | 88% | miR-512-3p | 63% | miR-99a-3p | 92% |
| | | miR-516b-3p, miR-516a-3p | 54% | | |

### [Table 22-2]

**Table 22-2: miRNA to Predict Human Breast Cancer of Stage I**

| miRNA Name | A | miRNA Name | A | miRNA Name | A |
|---|---|---|---|---|---|
| miR-29a-3p | 59% | miR-3928-3p | 68% | miR-6089 | 67% |
| miR-31-5p | 100% | miR-3937 | 75% | miR-6090 | 68% |
| miR-29b-3p | 64% | miR-1268b | 83% | miR-6125 | 79% |
| miR-124-3p | 75% | miR-4428 | 74% | miR-6126 | 74% |
| miR-184 | 56% | miR-4429 | 61% | miR-6127 | 66% |
| miR-106b-5p | 83% | miR-4430 | 62% | miR-6131 | 69% |
| miR-299-3p | 56% | miR-4433a-3p | 71% | miR-6132 | 74% |
| miR-361-5p | 60% | miR-4434 | 100% | miR-6500-3p | 55% |
| miR-370-3p | 69% | miR-4435 | 65% | miR-6501-5p | 60% |
| miR-373-3p | 60% | miR-4439 | 65% | miR-6501-3p | 59% |
| miR-379-5p | 80% | miR-4440 | 73% | miR-6511a-5p | 68% |
| miR-335-5p | 100% | miR-4443 | 76% | miR-6513-5p | 62% |
| miR-325 | 55% | miR-4446-3p | 67% | miR-6719-3p | 58% |
| miR-202-3p | 59% | miR-4447 | 73% | miR-6721-5p | 80% |
| miR-492 | 61% | miR-4449 | 77% | miR-6722-3p | 73% |
| miR-498-5p | 76% | miR-4450 | 53% | miR-6724-5p | 78% |
| miR-555 | 53% | miR-4454 | 53% | miR-208a-5p | 65% |
| miR-564 | 72% | miR-4456 | 60% | miR-128-1-5p | 67% |
| miR-572 | 73% | miR-4458 | 65% | miR-181d-3p | 80% |
| miR-575 | 68% | miR-378h | 64% | miR-504-3p | 69% |
| miR-578 | 70% | miR-3135b | 72% | miR-605-3p | 53% |
| miR-587 | 100% | miR-4466 | 73% | miR-1343-5p | 78% |
| miR-610 | 55% | miR-4467 | 81% | miR-5189-3p | 53% |
| miR-612 | 75% | miR-4468 | 67% | miR-6726-5p | 79% |
| miR-617 | 59% | miR-4479 | 55% | miR-6727-5p | 65% |
| miR-628-3p | 56% | miR-4481 | 73% | miR-6729-5p | 75% |
| miR-638 | 74% | miR-4486 | 71% | miR-6732-5p | 76% |
| miR-639 | 89% | miR-4487 | 68% | miR-6737-5p | 70% |
| miR-648 | 100% | miR-4489 | 67% | miR-6743-5p | 76% |
| miR-663a | 76% | miR-4492 | 67% | miR-6750-5p | 51% |
| miR-658 | 73% | miR-4497 | 80% | miR-6751-5p | 67% |
| miR-542-5p | 86% | miR-4498 | 70% | miR-6752-5p | 73% |
| miR-769-3p | 66% | miR-4505 | 79% | miR-6753-5p | 76% |
| miR-24-2-5p | 53% | miR-4506 | 55% | miR-6756-5p | 70% |
| miR-25-5p | 68% | miR-4507 | 79% | miR-6759-5p | 70% |
| miR-92a-2-5p | 75% | miR-4508 | 71% | miR-6762-5p | 69% |
| miR-139-3p | 74% | miR-4511 | 57% | miR-6763-5p | 71% |
| miR-187-5p | 72% | miR-4512 | 82% | miR-6766-5p | 67% |
| miR-30b-3p | 69% | miR-4519 | 100% | miR-6768-5p | 75% |
| miR-124-5p | 82% | miR-4521 | 67% | miR-6772-5p | 68% |
| miR-125b-1-3p | 100% | miR-1269b | 67% | miR-6776-5p | 64% |
| miR-130a-5p | 60% | miR-4530 | 81% | miR-6777-5p | 62% |
| miR-132-5p | 80% | miR-4533 | 66% | miR-6781-5p | 75% |
| miR-135a-3p | 65% | miR-4535 | 73% | miR-6782-5p | 75% |
| miR-149-3p | 69% | miR-1587 | 69% | miR-6784-5p | 69% |
| miR-185-3p | 65% | miR-4538 | 61% | miR-6786-5p | 72% |
| miR-193a-5p | 68% | miR-4539 | 78% | miR-6787-5p | 81% |
| miR-424-3p | 60% | miR-3187-5p | 68% | miR-6789-5p | 81% |
| miR-20b-3p | 70% | miR-3940-5p | 65% | miR-6790-5p | 77% |
| miR-486-3p | 68% | miR-4634 | 69% | miR-6791-5p | 81% |
| miR-193b-5p | 53% | miR-4638-5p | 73% | miR-6792-5p | 70% |
| miR-501-3p | 100% | miR-4639-5p | 100% | miR-6793-5p | 56% |
| miR-92b-5p | 79% | miR-4640-5p | 72% | miR-6794-5p | 56% |
| miR-551b-5p | 58% | miR-4648 | 55% | miR-6796-5p | 64% |
| miR-629-5p | 80% | miR-4650-3p | 71% | miR-6798-5p | 78% |
| miR-891b | 80% | miR-4651 | 77% | miR-6799-5p | 71% |
| miR-541-3p | 53% | miR-4653-3p | 58% | miR-6800-5p | 62% |
| miR-744-5p | 79% | miR-4654 | 61% | miR-6805-5p | 70% |
| miR-885-3p | 76% | miR-4655-5p | 73% | miR-6813-5p | 72% |
| miR-877-5p | 62% | miR-4657 | 52% | miR-6816-5p | 68% |
| miR-887-3p | 78% | miR-4661-5p | 64% | miR-6818-5p | 75% |
| miR-665 | 70% | miR-4665-5p | 74% | miR-6820-5p | 76% |
| miR-760 | 73% | miR-4673 | 63% | miR-6821-5p | 77% |
| miR-939-5p | 72% | miR-4674 | 63% | miR-6823-5p | 60% |
| miR-1224-5p | 71% | miR-4687-5p | 71% | miR-6829-5p | 69% |
| miR-1225-5p | 73% | miR-4688 | 71% | miR-6832-5p | 67% |
| miR-1228-5p | 68% | miR-4690-5p | 74% | miR-6836-5p | 53% |
| miR-1231 | 75% | miR-4691-3p | 88% | miR-6839-5p | 59% |
| miR-320c | 67% | miR-4694-5p | 53% | miR-6840-3p | 73% |
| miR-1207-5p | 73% | miR-4695-5p | 76% | miR-6842-5p | 71% |
| miR-1285-3p | 55% | miR-4704-5p | 53% | miR-6845-5p | 77% |
| miR-1293 | 54% | miR-4707-5p | 80% | miR-6847-5p | 51% |
| miR-1294 | 68% | miR-4708-5p | 62% | miR-6848-5p | 71% |
| miR-1299 | 100% | miR-4708-3p | 70% | miR-6849-5p | 68% |
| miR-1304-5p | 65% | miR-4720-5p | 61% | miR-6850-5p | 73% |
| miR-1257 | 52% | miR-4724-5p | 59% | miR-6860 | 75% |
| miR-1263 | 57% | miR-4726-3p | 74% | miR-6861-5p | 75% |
| miR-1268a | 73% | miR-4730 | 68% | miR-6869-5p | 68% |
| miR-1281 | 63% | miR-4734 | 70% | miR-6870-5p | 70% |
| miR-1292-5p | 88% | miR-4736 | 68% | miR-6871-5p | 59% |
| miR-320d | 83% | miR-4738-3p | 63% | miR-6875-5p | 69% |
| miR-1469 | 75% | miR-4741 | 71% | miR-6876-5p | 61% |
| miR-1471 | 72% | miR-4743-5p | 72% | miR-6877-5p | 76% |
| miR-1538 | 65% | miR-122b-3p | 54% | miR-6879-5p | 66% |
| miR-1908-5p | 81% | miR-4745-5p | 83% | miR-6893-5p | 64% |
| miR-1909-3p | 71% | miR-4746-3p | 72% | miR-6894-5p | 70% |
| miR-1912-3p | 72% | miR-4749-5p | 73% | miR-6895-5p | 66% |
| miR-1915-3p | 77% | miR-4750-5p | 74% | miR-7108-5p | 80% |
| miR-762 | 70% | miR-371b-5p | 78% | miR-7110-5p | 72% |
| miR-548q | 70% | miR-4754 | 56% | miR-7112-5p | 70% |
| miR-718 | 73% | miR-4755-3p | 55% | miR-7114-5p | 69% |
| miR-3120-3p | 63% | miR-4758-5p | 77% | miR-7150 | 69% |
| miR-3141 | 72% | miR-4759 | 100% | miR-7157-3p | 67% |
| miR-1273c | 78% | miR-4763-3p | 78% | miR-7158-3p | 56% |
| miR-3161 | 56% | miR-4766-5p | 70% | miR-7160-5p | 62% |
| miR-3162-5p | 80% | miR-4781-5p | 100% | miR-7515 | 65% |
| miR-1260b | 74% | miR-4784 | 55% | miR-7704 | 62% |
| miR-3168 | 70% | miR-4787-5p | 67% | miR-4433b-3p | 72% |
| miR-3173-3p | 56% | miR-4520-2-3p | 100% | miR-6516-5p | 52% |
| miR-1193 | 73% | miR-4482-3p | 68% | miR-7851-3p | 62% |
| miR-3178 | 70% | miR-5001-5p | 77% | miR-7854-3p | 65% |
| miR-3179 | 60% | miR-5010-5p | 70% | miR-8059 | 72% |
| miR-3180-3p | 75% | miR-5088-5p | 65% | miR-8063 | 70% |
| miR-3185 | 80% | miR-5089-5p | 64% | miR-8064 | 73% |
| miR-3187-3p | 82% | miR-5090 | 72% | miR-8069 | 67% |
| miR-3195 | 74% | miR-5189-5p | 71% | miR-8071 | 82% |
| miR-3196 | 70% | miR-5100 | 75% | miR-8072 | 76% |
| miR-3197 | 77% | miR-5572 | 71% | miR-8075 | 58% |
| miR-4315 | 55% | miR-5585-3p | 68% | miR-8077 | 60% |
| miR-4314 | 56% | miR-548au-3p | 53% | miR-8078 | 51% |
| miR-4322 | 76% | miR-5690 | 60% | miR-8089 | 76% |
| miR-4321 | 60% | miR-5698 | 73% | miR-7976 | 56% |
| miR-4256 | 69% | miR-5699-3p | 63% | miR-1249-5p | 65% |
| miR-4257 | 71% | miR-5703 | 62% | miR-375-5p | 73% |
| miR-4258 | 76% | miR-5706 | 100% | miR-526a-3p | 100% |
| miR-4327 | 70% | miR-197-5p | 72% | miR-9718 | 80% |
| miR-4265 | 68% | miR-211-3p | 69% | miR-10392-5p | 84% |
| miR-4276 | 67% | miR-766-5p | 54% | miR-10392-3p | 79% |
| miR-4285 | 76% | miR-3529-3p | 80% | miR-10394-3p | 67% |
| miR-3609 | 100% | miR-937-5p | 79% | miR-10395-3p | 64% |
| miR-3616-3p | 77% | miR-1227-5p | 79% | miR-10396a-5p | 72% |
| miR-3621 | 74% | miR-1236-5p | 53% | miR-10396a-3p | 76% |
| miR-3648 | 80% | miR-1237-5p | 70% | miR-10398-3p | 71% |
| miR-3652 | 75% | miR-3620-5p | 74% | miR-10400-3p | 81% |
| miR-3660 | 56% | miR-3934-3p | 58% | miR-10401-5p | 79% |
| miR-3663-3p | 73% | miR-4632-5p | 73% | miR-10396b-5p | 74% |
| miR-3665 | 63% | miR-5787 | 63% | miR-11399 | 78% |
| miR-3677-3p | 75% | miR-6068 | 77% | miR-12114 | 67% |
| miR-3180 | 63% | miR-6071 | 67% | miR-12118 | 69% |
| miR-3907 | 83% | miR-6075 | 77% | miR-12119 | 60% |
| miR-3917 | 69% | miR-6076 | 78% | miR-12120 | 73% |
| miR-3919 | 65% | miR-6081 | 64% | miR-12121 | 75% |

### [Table 22-3]

**Table 22-3: miRNA to Predict Human Kidney Cancer of Stage I**

| miRNA Name | A | miRNA Name | A | miRNA Name | A |
|---|---|---|---|---|---|
| miR-17-3p | 100% | miR-3649 | 72% | miR-370-5p | 53% |
| miR-29a-3p | 67% | miR-3652 | 71% | miR-328-5p | 67% |
| miR-30a-5p | 57% | miR-3654 | 53% | miR-410-5p | 54% |
| miR-31-5p | 100% | miR-3660 | 63% | miR-181d-3p | 83% |
| miR-29b-3p | 55% | miR-3663-5p | 66% | miR-520g-5p | 100% |
| miR-196a-5p | 75% | miR-3663-3p | 64% | miR-504-3p | 65% |
| miR-197-3p | 72% | miR-3665 | 65% | miR-487b-5p | 55% |
| miR-198 | 70% | miR-3667-5p | 54% | miR-585-5p | 54% |
| miR-30d-5p | 75% | miR-3667-3p | 72% | miR-598-5p | 63% |
| miR-10a-5p | 58% | miR-3675-5p | 51% | miR-605-3p | 68% |
| miR-187-3p | 52% | miR-3675-3p | 75% | miR-619-5p | 55% |
| miR-199b-5p | 75% | miR-3677-3p | 70% | miR-1296-3p | 66% |
| miR-214-3p | 54% | miR-3679-5p | 78% | miR-874-5p | 72% |
| miR-223-3p | 72% | miR-3679-3p | 76% | miR-887-5p | 67% |
| miR-133a-3p | 72% | miR-3689a-3p | 56% | miR-513b-3p | 67% |
| miR-125a-5p | 52% | miR-3691-5p | 54% | miR-1908-3p | 70% |
| miR-134-5p | 64% | miR-3714 | 67% | miR-3151-3p | 66% |
| miR-184 | 53% | miR-3180 | 65% | miR-3192-3p | 54% |
| miR-185-5p | 63% | miR-3907 | 75% | miR-3912-5p | 67% |
| miR-206 | 54% | miR-3689b-3p, miR-3689c | 54% | miR-1343-5p | 75% |
| miR-200c-3p | 100% | miR-3909 | 65% | miR-5088-3p | 65% |
| miR-106b-5p | 83% | miR-3911 | 66% | miR-5189-3p | 53% |
| miR-29c-3p | 100% | miR-3917 | 65% | miR-5699-5p | 72% |
| miR-302a-3p | 100% | miR-3919 | 61% | miR-6726-5p | 73% |
| miR-299-3p | 59% | miR-3925-5p | 78% | miR-6726-3p | 67% |
| miR-296-5p | 73% | miR-3926 | 60% | miR-6727-5p | 65% |
| miR-130b-3p | 89% | miR-3928-3p | 76% | miR-6727-3p | 70% |
| miR-30e-5p | 100% | miR-3935 | 53% | miR-6728-5p | 59% |
| miR-361-5p | 53% | miR-3936 | 53% | miR-6728-3p | 72% |
| miR-362-5p | 100% | miR-3937 | 76% | miR-6729-5p | 70% |
| miR-365a-3p, miR-365b-3p | 70% | miR-3941 | 53% | miR-6729-3p | 74% |
| miR-302c-5p | 71% | miR-3943 | 73% | miR-6730-5p | 74% |
| miR-370-3p | 77% | miR-3944-3p | 65% | miR-6730-3p | 71% |
| miR-373-5p | 70% | miR-3945 | 62% | miR-6731-5p | 66% |
| miR-373-3p | 62% | miR-642b-3p | 66% | miR-6731-3p | 72% |
| miR-378a-3p | 57% | miR-550b-3p | 52% | miR-6732-5p | 72% |
| miR-379-5p | 75% | miR-1268b | 76% | miR-6732-3p | 74% |
| miR-380-3p | 63% | miR-548ab | 67% | miR-6734-5p | 74% |
| miR-328-3p | 73% | miR-4418 | 100% | miR-6734-3p | 68% |
| miR-342-3p | 54% | miR-4421 | 65% | miR-6735-5p | 63% |
| miR-326 | 69% | miR-4428 | 76% | miR-6735-3p | 64% |
| miR-133b | 71% | miR-4429 | 76% | miR-6736-5p | 56% |
| miR-325 | 52% | miR-4430 | 76% | miR-6736-3p | 63% |
| miR-346 | 62% | miR-4433a-3p | 74% | miR-6737-5p | 69% |
| miR-20b-5p | 71% | miR-4434 | 100% | miR-6737-3p | 75% |
| miR-448 | 57% | miR-4439 | 64% | miR-6738-5p | 73% |
| miR-429 | 100% | miR-4440 | 67% | miR-6738-3p | 56% |
| miR-449a | 60% | miR-4441 | 72% | miR-6740-5p | 63% |
| miR-191-3p | 74% | miR-4443 | 58% | miR-6740-3p | 69% |
| miR-200a-5p | 54% | miR-4444 | 66% | miR-6741-5p | 82% |
| miR-409-3p | 70% | miR-4446-3p | 68% | miR-6741-3p | 71% |
| miR-483-3p | 65% | miR-4447 | 82% | miR-6742-5p | 82% |
| miR-484 | 72% | miR-4448 | 64% | miR-6742-3p | 70% |
| miR-485-3p | 62% | miR-4449 | 64% | miR-6743-5p | 71% |
| miR-486-5p | 75% | miR-4451 | 51% | miR-6743-3p | 69% |
| miR-491-5p | 81% | miR-4455 | 59% | miR-6744-5p | 67% |
| miR-202-5p | 100% | miR-4456 | 60% | miR-6744-3p | 66% |
| miR-202-3p | 65% | miR-4458 | 63% | miR-6745 | 66% |
| miR-492 | 58% | miR-4460 | 75% | miR-6746-5p | 87% |
| miR-494-3p | 53% | miR-378h | 53% | miR-6747-5p | 68% |
| miR-512-5p | 53% | miR-3135b | 67% | miR-6747-3p | 67% |
| miR-498-5p | 82% | miR-4462 | 65% | miR-6748-5p | 80% |
| miR-520e-3p | 59% | miR-4463 | 65% | miR-6749-5p | 80% |
| miR-519c-5p, miR-523-5p, miR-518e-5p, miR-522-5p, miR-519a-5p, miR-519b-5p | 57% | miR-4466 | 68% | miR-6749-3p | 77% |
| miR-526b-5p | 53% | miR-4467 | 70% | miR-6750-5p | 51% |
| miR-518f-3p | 51% | miR-4468 | 67% | miR-6750-3p | 68% |
| miR-520b-3p | 52% | miR-4470 | 88% | miR-6751-5p | 69% |
| miR-518b | 75% | miR-4472 | 76% | miR-6752-5p | 72% |
| miR-520c-3p | 80% | miR-4474-3p | 51% | miR-6752-3p | 72% |
| miR-518c-5p | 72% | miR-4475 | 67% | miR-6753-5p | 65% |
| miR-520d-3p | 54% | miR-4476 | 67% | miR-6753-3p | 60% |
| miR-516b-5p | 80% | miR-4477b | 100% | miR-6754-5p | 73% |
| miR-516b-3p, miR-516a-3p | 54% | miR-4478 | 75% | miR-6754-3p | 59% |
| miR-519a-3p | 71% | miR-3689d | 51% | miR-6755-5p | 71% |
| miR-499a-5p | 100% | miR-4481 | 69% | miR-6756-5p | 65% |
| miR-502-5p | 53% | miR-4483 | 76% | miR-6756-3p | 78% |
| miR-513a-5p | 67% | miR-4484 | 81% | miR-6757-5p | 71% |
| miR-532-5p | 67% | miR-4486 | 68% | miR-6757-3p | 74% |
| miR-556-5p | 100% | miR-4487 | 71% | miR-6758-5p | 77% |
| miR-557 | 65% | miR-4488 | 68% | miR-6759-5p | 72% |
| miR-563 | 59% | miR-4489 | 64% | miR-6759-3p | 74% |
| miR-564 | 57% | miR-4492 | 60% | miR-6760-5p | 74% |
| miR-571 | 100% | miR-4496 | 82% | miR-6760-3p | 72% |
| miR-572 | 53% | miR-4497 | 75% | miR-6761-3p | 63% |
| miR-573 | 100% | miR-4498 | 64% | miR-6762-5p | 64% |
| miR-574-3p | 68% | miR-4499 | 77% | miR-6762-3p | 54% |
| miR-575 | 51% | miR-4505 | 73% | miR-6763-5p | 80% |
| miR-578 | 70% | miR-2392 | 72% | miR-6763-3p | 76% |
| miR-579-3p | 62% | miR-4507 | 75% | miR-6765-5p | 63% |
| miR-583 | 64% | miR-4508 | 64% | miR-6766-5p | 69% |
| miR-585-3p | 65% | miR-4510 | 57% | miR-6766-3p | 73% |
| miR-588 | 67% | miR-4513 | 71% | miR-6767-5p | 54% |
| miR-550a-3p | 51% | miR-4516 | 69% | miR-6768-5p | 67% |
| miR-590-5p | 100% | miR-4518 | 61% | miR-6769a-5p | 76% |
| miR-608 | 52% | miR-4519 | 100% | miR-6769a-3p | 70% |
| miR-610 | 55% | miR-4521 | 75% | miR-6770-5p | 59% |
| miR-612 | 51% | miR-1269b | 63% | miR-6771-5p | 62% |
| miR-615-3p | 61% | miR-4522 | 67% | miR-6771-3p | 75% |
| miR-617 | 63% | miR-4523 | 54% | miR-6772-5p | 64% |
| miR-619-3p | 100% | miR-4525 | 73% | miR-6772-3p | 53% |
| miR-624-5p | 55% | miR-4526 | 56% | miR-6773-3p | 52% |
| miR-625-5p | 64% | miR-4530 | 71% | miR-6774-5p | 74% |
| miR-628-3p | 67% | miR-4531 | 70% | miR-6775-5p | 71% |
| miR-634 | 74% | miR-4533 | 70% | miR-6775-3p | 75% |
| miR-637 | 74% | miR-4534 | 69% | miR-6776-5p | 69% |
| miR-638 | 71% | miR-4535 | 67% | miR-6776-3p | 70% |
| miR-639 | 86% | miR-1587 | 69% | miR-6777-5p | 75% |
| miR-641 | 100% | miR-4538 | 65% | miR-6777-3p | 75% |
| miR-650 | 54% | miR-4539 | 67% | miR-6778-5p | 72% |
| miR-663a | 67% | miR-3127-3p | 73% | miR-6778-3p | 57% |
| miR-449b-5p | 57% | miR-3156-3p | 59% | miR-6779-5p | 71% |
| miR-654-5p | 56% | miR-3158-5p | 76% | miR-6779-3p | 64% |
| miR-657 | 59% | miR-3162-3p | 72% | miR-6780a-5p | 76% |
| miR-658 | 72% | miR-3173-5p | 61% | miR-6780a-3p | 60% |
| miR-659-3p | 63% | miR-3187-5p | 63% | miR-6781-5p | 76% |
| miR-542-5p | 83% | miR-3189-5p | 74% | miR-6782-5p | 74% |
| miR-671-5p | 66% | miR-3619-3p | 77% | miR-6782-3p | 74% |
| miR-668-3p | 57% | miR-3150b-5p | 74% | miR-6783-5p | 66% |
| miR-767-3p | 76% | miR-3940-5p | 66% | miR-6783-3p | 57% |
| miR-769-5p | 53% | miR-3960 | 62% | miR-6784-5p | 67% |
| miR-769-3p | 55% | miR-3972 | 59% | miR-6784-3p | 73% |
| miR-766-3p | 75% | miR-3978 | 100% | miR-6785-3p | 74% |
| miR-765 | 76% | miR-4634 | 70% | miR-6786-5p | 66% |
| miR-675-5p | 63% | miR-4638-5p | 68% | miR-6786-3p | 76% |
| miR-297 | 52% | miR-4639-3p | 54% | miR-6787-5p | 75% |
| let-7b-3p | 70% | miR-4640-5p | 70% | miR-6787-3p | 63% |
| let-7d-3p | 53% | miR-4640-3p | 71% | miR-6788-5p | 52% |
| let-7f-1-3p | 73% | miR-4642 | 70% | miR-6788-3p | 72% |
| miR-92a-2-5p | 61% | miR-4644 | 53% | miR-6789-5p | 70% |
| miR-29b-2-5p | 55% | miR-4646-5p | 73% | miR-6789-3p | 53% |
| miR-30c-2-3p | 63% | miR-4646-3p | 70% | miR-6790-5p | 68% |
| miR-139-3p | 61% | miR-4648 | 57% | miR-6790-3p | 75% |
| miR-10a-3p | 100% | miR-4649-5p | 61% | miR-6791-5p | 69% |
| miR-181c-3p | 67% | miR-4649-3p | 71% | miR-6792-5p | 63% |
| miR-183-3p | 72% | miR-4650-3p | 83% | miR-6792-3p | 74% |
| miR-187-5p | 69% | miR-4651 | 76% | miR-6793-3p | 74% |
| miR-15b-3p | 67% | miR-4652-5p | 63% | miR-6794-5p | 81% |
| miR-124-5p | 71% | miR-4653-3p | 58% | miR-6794-3p | 67% |
| miR-125b-1-3p | 100% | miR-4654 | 57% | miR-6795-5p | 67% |
| miR-130a-5p | 53% | miR-4655-5p | 63% | miR-6795-3p | 75% |
| miR-135a-3p | 59% | miR-4656 | 73% | miR-6796-5p | 64% |
| miR-125a-3p | 71% | miR-4659b-3p | 57% | miR-6796-3p | 79% |
| miR-127-5p | 67% | miR-4663 | 100% | miR-6797-5p | 75% |
| miR-149-3p | 64% | miR-4664-5p | 69% | miR-6797-3p | 72% |
| miR-150-3p | 78% | miR-4664-3p | 77% | miR-6798-5p | 70% |
| miR-185-3p | 69% | miR-4665-5p | 83% | miR-6798-3p | 73% |
| miR-186-3p | 64% | miR-4665-3p | 74% | miR-6799-5p | 73% |
| miR-193a-5p | 60% | miR-4667-5p | 77% | miR-6799-3p | 72% |
| miR-194-3p | 62% | miR-4667-3p | 74% | miR-6800-5p | 63% |
| miR-296-3p | 76% | miR-4669 | 63% | miR-6800-3p | 74% |
| miR-361-3p | 72% | miR-4670-3p | 100% | miR-6801-3p | 72% |
| miR-302d-5p | 58% | miR-4672 | 56% | miR-6802-5p | 63% |
| miR-371a-5p | 64% | miR-4673 | 51% | miR-6802-3p | 71% |
| miR-374a-3p | 100% | miR-4674 | 67% | miR-6803-5p | 64% |
| miR-377-5p | 54% | miR-4675 | 61% | miR-6803-3p | 68% |
| miR-342-5p | 74% | miR-4677-5p | 70% | miR-6804-3p | 72% |
| miR-423-5p | 67% | miR-4684-3p | 53% | miR-6805-5p | 73% |
| miR-424-3p | 80% | miR-4685-5p | 66% | miR-6805-3p | 78% |
| miR-18b-3p | 72% | miR-4685-3p | 69% | miR-6806-5p | 52% |
| miR-20b-3p | 70% | miR-4687-5p | 74% | miR-6807-5p | 71% |
| miR-431-3p | 55% | miR-4687-3p | 67% | miR-6808-5p | 64% |
| miR-483-5p | 79% | miR-1343-3p | 71% | miR-6808-3p | 55% |
| miR-486-3p | 67% | miR-4688 | 68% | miR-6809-5p | 78% |
| miR-490-5p | 100% | miR-4689 | 63% | miR-6809-3p | 64% |
| miR-193b-5p | 53% | miR-4690-5p | 74% | miR-6810-5p | 68% |
| miR-505-5p | 71% | miR-4691-5p | 53% | miR-6810-3p | 78% |
| miR-513a-3p | 100% | miR-4695-5p | 70% | miR-6812-5p | 75% |
| miR-92b-5p | 75% | miR-4695-3p | 69% | miR-6812-3p | 74% |
| miR-551b-5p | 65% | miR-4697-5p | 65% | miR-6813-5p | 71% |
| miR-574-5p | 65% | miR-4697-3p | 71% | miR-6813-3p | 75% |
| miR-550a-5p | 69% | miR-4698 | 73% | miR-6815-5p | 68% |
| miR-615-5p | 74% | miR-4700-5p | 58% | miR-6816-5p | 63% |
| miR-616-3p | 55% | miR-4700-3p | 73% | miR-6816-3p | 76% |
| miR-625-3p | 72% | miR-4701-5p | 71% | miR-6818-5p | 71% |
| miR-629-5p | 88% | miR-4701-3p | 56% | miR-6819-5p | 62% |
| miR-298 | 67% | miR-4706 | 68% | miR-6819-3p | 76% |
| miR-874-3p | 68% | miR-4707-5p | 72% | miR-6820-5p | 73% |
| miR-890 | 100% | miR-4707-3p | 75% | miR-6820-3p | 75% |
| miR-891b | 67% | miR-4708-3p | 62% | miR-6821-5p | 76% |
| miR-876-3p | 80% | miR-4709-3p | 68% | miR-6822-5p | 65% |
| miR-744-5p | 61% | miR-4710 | 65% | miR-6823-5p | 59% |
| miR-885-5p | 60% | miR-4713-5p | 70% | miR-6823-3p | 70% |
| miR-885-3p | 59% | miR-4714-5p | 71% | miR-6824-5p | 69% |
| miR-877-5p | 63% | miR-4715-5p | 56% | miR-6825-5p | 73% |
| miR-877-3p | 75% | miR-4716-5p | 75% | miR-6825-3p | 70% |
| miR-887-3p | 70% | miR-4716-3p | 79% | miR-6826-3p | 71% |
| miR-665 | 76% | miR-4717-3p | 61% | miR-6827-5p | 71% |
| miR-760 | 67% | miR-4720-5p | 63% | miR-6827-3p | 77% |
| miR-301b-3p | 100% | miR-4721 | 68% | miR-6828-5p | 72% |
| miR-920 | 81% | miR-4722-5p | 61% | miR-6829-5p | 76% |
| miR-509-3-5p | 100% | miR-4722-3p | 68% | miR-6829-3p | 57% |
| miR-933 | 74% | miR-4723-5p | 79% | miR-6830-5p | 76% |
| miR-936 | 65% | miR-4723-3p | 68% | miR-6830-3p | 68% |
| miR-939-5p | 78% | miR-4725-3p | 79% | miR-6831-5p | 76% |
| miR-940 | 72% | miR-4726-5p | 60% | miR-6831-3p | 64% |
| miR-1224-5p | 60% | miR-4727-3p | 55% | miR-6832-5p | 75% |
| miR-1224-3p | 75% | miR-4728-5p | 74% | miR-6832-3p | 53% |
| miR-1225-5p | 70% | miR-4728-3p | 73% | miR-6833-5p | 75% |
| miR-1225-3p | 76% | miR-4729 | 100% | miR-6833-3p | 64% |
| miR-1227-3p | 66% | miR-4730 | 61% | miR-6834-5p | 63% |
| miR-1228-5p | 65% | miR-4731-5p | 65% | miR-6780b-5p | 76% |
| miR-1228-3p | 76% | miR-4731-3p | 72% | miR-6780b-3p | 67% |
| miR-1229-3p | 65% | miR-4732-5p | 77% | miR-6836-3p | 72% |
| miR-1231 | 67% | miR-4732-3p | 58% | miR-6838-5p | 56% |
| miR-1233-3p | 63% | miR-4734 | 67% | miR-6839-5p | 59% |
| miR-1234-3p | 69% | miR-4736 | 66% | miR-6840-5p | 71% |
| miR-1236-3p | 71% | miR-3064-5p | 55% | miR-6840-3p | 74% |
| miR-1237-3p | 77% | miR-3064-3p | 51% | miR-6841-3p | 73% |
| miR-1238-3p | 74% | miR-4738-3p | 55% | miR-6842-5p | 78% |
| miR-320b | 60% | miR-4739 | 63% | miR-6845-5p | 72% |
| miR-320c | 64% | miR-4741 | 72% | miR-6845-3p | 75% |
| miR-1323 | 61% | miR-4742-3p | 67% | miR-6846-5p | 78% |
| miR-1301-3p | 63% | miR-4743-5p | 72% | miR-6846-3p | 71% |
| miR-1298-5p | 61% | miR-4745-5p | 73% | miR-6847-3p | 61% |
| miR-1181 | 68% | miR-4746-3p | 57% | miR-6848-5p | 70% |
| miR-1182 | 71% | miR-4747-5p | 77% | miR-6848-3p | 72% |
| miR-1184 | 57% | miR-4748 | 71% | miR-6849-5p | 72% |
| miR-1202 | 70% | miR-4749-5p | 76% | miR-6850-5p | 69% |
| miR-1203 | 66% | miR-4749-3p | 74% | miR-6851-5p | 65% |
| miR-663b | 66% | miR-4750-5p | 64% | miR-6851-3p | 77% |
| miR-1207-5p | 82% | miR-4751 | 74% | miR-6855-5p | 63% |
| miR-1207-3p | 52% | miR-4752 | 52% | miR-6855-3p | 74% |
| miR-1285-3p | 54% | miR-4753-5p | 67% | miR-6856-5p | 58% |
| miR-1289 | 75% | miR-371b-5p | 76% | miR-6857-5p | 73% |
| miR-1294 | 65% | miR-371b-3p | 70% | miR-6857-3p | 71% |
| miR-1299 | 100% | miR-4754 | 54% | miR-6858-5p | 64% |
| miR-1303 | 53% | miR-4755-3p | 66% | miR-6858-3p | 75% |
| miR-1304-5p | 60% | miR-4756-5p | 71% | miR-6859-3p | 75% |
| miR-1245a | 100% | miR-4758-5p | 81% | miR-6769b-5p | 74% |
| miR-1246 | 100% | miR-4758-3p | 71% | miR-6769b-3p | 59% |
| miR-1249-3p | 76% | miR-4759 | 100% | miR-6860 | 64% |
| miR-1257 | 52% | miR-4763-5p | 67% | miR-6861-5p | 73% |
| miR-1260a | 71% | miR-4763-3p | 74% | miR-6861-3p | 73% |
| miR-1263 | 60% | miR-4764-5p | 100% | miR-6862-5p | 74% |
| miR-1267 | 73% | miR-4766-5p | 63% | miR-6862-3p | 70% |
| miR-1268a | 66% | miR-4769-5p | 63% | miR-6865-5p | 61% |
| miR-1270 | 55% | miR-4769-3p | 71% | miR-6865-3p | 71% |
| miR-1275 | 66% | miR-4776-5p | 71% | miR-6867-5p | 62% |
| miR-1278 | 100% | miR-4778-5p | 75% | miR-6867-3p | 66% |
| miR-1281 | 76% | miR-4779 | 88% | miR-6868-3p | 53% |
| miR-1292-5p | 85% | miR-4780 | 54% | miR-6869-5p | 67% |
| miR-1255b-5p | 100% | miR-4436b-5p | 70% | miR-6870-5p | 72% |
| miR-1307-3p | 70% | miR-4781-5p | 100% | miR-6870-3p | 74% |
| miR-320d | 83% | miR-4783-3p | 72% | miR-6871-5p | 61% |
| miR-1825 | 74% | miR-2467-3p | 65% | miR-6872-5p | 52% |
| miR-675-3p | 65% | miR-4787-5p | 65% | miR-6873-5p | 55% |
| miR-1469 | 71% | miR-4787-3p | 75% | miR-6874-5p | 58% |
| miR-1470 | 80% | miR-4788 | 70% | miR-6875-5p | 68% |
| miR-1471 | 68% | miR-4789-3p | 100% | miR-6875-3p | 63% |
| miR-1539 | 60% | miR-4790-3p | 100% | miR-6876-5p | 70% |
| miR-1908-5p | 75% | miR-4793-3p | 70% | miR-6877-5p | 78% |
| miR-1909-5p | 60% | miR-4795-3p | 100% | miR-6877-3p | 65% |
| miR-1909-3p | 68% | miR-4796-3p | 100% | miR-6878-5p | 54% |
| miR-1910-5p | 65% | miR-4798-3p | 100% | miR-6878-3p | 61% |
| miR-1912-3p | 67% | miR-4800-5p | 84% | miR-6879-5p | 74% |
| miR-1913 | 71% | miR-4804-5p | 67% | miR-6879-3p | 71% |
| miR-1914-3p | 77% | miR-4804-3p | 51% | miR-6880-5p | 76% |
| miR-1915-3p | 68% | miR-642a-3p | 68% | miR-6880-3p | 74% |
| miR-365a-5p | 74% | miR-550a-3-5p | 53% | miR-6881-5p | 72% |
| miR-449b-3p | 72% | miR-4433a-5p | 76% | miR-6882-5p | 62% |
| miR-2113 | 59% | miR-4482-3p | 75% | miR-6882-3p | 65% |
| miR-1976 | 70% | miR-4999-5p | 55% | miR-6883-5p | 68% |
| miR-2110 | 79% | miR-5001-5p | 65% | miR-6883-3p | 56% |
| miR-151b | 52% | miR-5002-3p | 57% | miR-6884-3p | 75% |
| miR-762 | 69% | miR-5003-3p | 79% | miR-6885-5p | 58% |
| miR-670-5p | 60% | miR-5006-5p | 53% | miR-6885-3p | 76% |
| miR-764 | 65% | miR-5008-5p | 54% | miR-6886-3p | 70% |
| miR-2116-3p | 71% | miR-5009-3p | 64% | miR-6887-5p | 71% |
| miR-548q | 59% | miR-5010-5p | 72% | miR-6887-3p | 76% |
| miR-2276-3p | 68% | miR-5010-3p | 71% | miR-6889-5p | 78% |
| miR-2278 | 66% | miR-5088-5p | 61% | miR-6889-3p | 71% |
| miR-711 | 78% | miR-5089-5p | 58% | miR-6890-5p | 64% |
| miR-718 | 77% | miR-5090 | 67% | miR-6890-3p | 72% |
| miR-2681-3p | 65% | miR-5093 | 52% | miR-6891-5p | 70% |
| miR-2861 | 63% | miR-5188 | 52% | miR-6891-3p | 70% |
| miR-3116 | 67% | miR-5189-5p | 61% | miR-6892-5p | 70% |
| miR-3119 | 67% | miR-5193 | 63% | miR-6892-3p | 73% |
| miR-3120-3p | 63% | miR-5195-5p | 68% | miR-6893-5p | 78% |
| miR-3122 | 63% | miR-5195-3p | 64% | miR-6893-3p | 61% |
| miR-3124-5p | 63% | miR-5196-5p | 78% | miR-6894-5p | 73% |
| miR-3126-5p | 71% | miR-4524b-5p | 55% | miR-6894-3p | 68% |
| miR-3128 | 100% | miR-5100 | 54% | miR-7106-5p | 72% |
| miR-3130-5p | 61% | miR-5572 | 77% | miR-7106-3p | 62% |
| miR-3131 | 75% | miR-664b-5p | 53% | miR-7107-5p | 80% |
| miR-3132 | 63% | miR-664b-3p | 72% | miR-7107-3p | 54% |
| miR-3133 | 61% | miR-5581-5p | 51% | miR-7108-5p | 70% |
| miR-466 | 51% | miR-5584-5p | 73% | miR-7108-3p | 79% |
| miR-3136-5p | 100% | miR-548au-3p | 56% | miR-7109-5p | 71% |
| miR-3138 | 60% | miR-5588-5p | 54% | miR-7109-3p | 64% |
| miR-3141 | 74% | miR-5589-5p | 64% | miR-7110-5p | 78% |
| miR-3144-5p | 69% | miR-5682 | 100% | miR-7110-3p | 70% |
| miR-1273c | 67% | miR-5684 | 58% | miR-7111-5p | 83% |
| miR-3147 | 74% | miR-5690 | 62% | miR-7111-3p | 74% |
| miR-3150a-3p | 80% | miR-5698 | 74% | miR-7112-5p | 57% |
| miR-3151-5p | 67% | miR-5703 | 65% | miR-7113-3p | 71% |
| miR-3153 | 74% | miR-5705 | 57% | miR-7114-5p | 72% |
| miR-3154 | 78% | miR-197-5p | 78% | miR-7114-3p | 74% |
| miR-3156-5p | 76% | miR-204-3p | 78% | miR-7150 | 78% |
| miR-3157-5p | 64% | miR-211-3p | 73% | miR-7151-3p | 59% |
| miR-3160-3p | 69% | miR-301a-5p | 52% | miR-7152-5p | 59% |
| miR-3161 | 54% | miR-345-3p | 70% | miR-7152-3p | 62% |
| miR-3162-5p | 78% | miR-652-5p | 70% | miR-7155-5p | 61% |
| miR-3163 | 83% | miR-1271-3p | 100% | miR-7157-3p | 58% |
| miR-3165 | 60% | miR-1185-2-3p | 75% | miR-7159-5p | 62% |
| miR-1260b | 65% | miR-766-5p | 52% | miR-7160-5p | 63% |
| miR-3169 | 62% | miR-1304-3p | 69% | miR-7162-5p | 56% |
| miR-3173-3p | 57% | miR-1247-3p | 73% | miR-7515 | 71% |
| miR-1193 | 65% | miR-548g-5p, miR-548x-5p, miR-548aj-5p | 100% | miR-7704 | 65% |
| miR-3175 | 64% | miR-1306-5p | 71% | miR-7843-5p | 58% |
| miR-3177-3p | 75% | miR-3184-3p | 70% | miR-4433b-5p | 72% |
| miR-3178 | 67% | miR-3191-5p | 60% | miR-4433b-3p | 77% |
| miR-3179 | 57% | miR-642b-5p | 57% | miR-1273h-5p | 63% |
| miR-3180-5p | 75% | miR-365b-5p | 65% | miR-6516-5p | 59% |
| miR-3180-3p | 66% | miR-1185-1-3p | 77% | miR-7845-5p | 69% |
| miR-3184-5p | 63% | miR-98-3p | 72% | miR-7846-3p | 77% |
| miR-3185 | 77% | miR-376c-5p | 100% | miR-7847-3p | 70% |
| miR-3186-3p | 56% | miR-381-5p | 62% | miR-7851-3p | 62% |
| miR-3187-3p | 52% | miR-503-3p | 65% | miR-7855-5p | 67% |
| miR-3188 | 71% | miR-937-5p | 69% | miR-7856-5p | 54% |
| miR-3189-3p | 73% | miR-939-3p | 74% | miR-8052 | 70% |
| miR-3191-3p | 79% | miR-1227-5p | 73% | miR-8053 | 100% |
| miR-3194-5p | 63% | miR-1229-5p | 67% | miR-8054 | 75% |
| miR-3195 | 66% | miR-1233-5p | 72% | miR-8058 | 100% |
| miR-3196 | 68% | miR-1236-5p | 64% | miR-8059 | 68% |
| miR-3197 | 76% | miR-1237-5p | 68% | miR-8060 | 72% |
| miR-3198 | 61% | miR-1238-5p | 77% | miR-8062 | 52% |
| miR-3199 | 52% | miR-3617-3p | 51% | miR-8063 | 58% |
| miR-514b-5p | 65% | miR-3620-5p | 69% | miR-8069 | 66% |
| miR-3202 | 75% | miR-3934-3p | 55% | miR-8071 | 71% |
| miR-4297 | 58% | miR-4632-5p | 65% | miR-8072 | 66% |
| miR-4293 | 67% | miR-4750-3p | 73% | miR-8073 | 72% |
| miR-4294 | 75% | miR-5739 | 78% | miR-8074 | 54% |
| miR-4299 | 67% | miR-5787 | 65% | miR-8077 | 62% |
| miR-4298 | 75% | miR-6068 | 59% | miR-8080 | 62% |
| miR-4300 | 55% | miR-6069 | 70% | miR-8085 | 82% |
| miR-4305 | 56% | miR-6071 | 67% | miR-8087 | 80% |
| miR-4306 | 68% | miR-6072 | 53% | miR-8089 | 72% |
| miR-4307 | 73% | miR-6075 | 68% | miR-128-2-5p | 63% |
| miR-4312 | 76% | miR-6076 | 77% | miR-7977 | 67% |
| miR-4313 | 71% | miR-6077 | 63% | miR-7978 | 100% |
| miR-4316 | 59% | miR-6081 | 53% | miR-1249-5p | 68% |
| miR-4314 | 61% | miR-6085 | 80% | miR-4485-5p | 67% |
| miR-4322 | 60% | miR-6086 | 80% | miR-8485 | 69% |
| miR-4321 | 58% | miR-6088 | 63% | miR-135a-2-3p | 54% |
| miR-4323 | 71% | miR-6089 | 71% | miR-375-5p | 73% |
| miR-4256 | 64% | miR-6090 | 66% | miR-498-3p | 56% |
| miR-4257 | 77% | miR-6124 | 78% | miR-526a-3p | 100% |
| miR-4258 | 75% | miR-6125 | 73% | miR-9718 | 72% |
| miR-4259 | 72% | miR-6126 | 69% | miR-9898 | 73% |
| miR-4260 | 71% | miR-6127 | 73% | miR-9900 | 100% |
| miR-4253 | 59% | miR-378j | 65% | miR-10392-5p | 73% |
| miR-4251 | 66% | miR-6131 | 74% | miR-10392-3p | 72% |
| miR-4325 | 69% | miR-6132 | 72% | miR-10394-3p | 52% |
| miR-4326 | 66% | miR-6133 | 78% | miR-10395-3p | 60% |
| miR-4327 | 76% | miR-6165 | 79% | miR-10396a-5p | 66% |
| miR-4265 | 62% | miR-6500-3p | 61% | miR-10396a-3p | 74% |
| miR-4266 | 64% | miR-548az-3p | 100% | miR-10397-3p | 100% |
| miR-2355-5p | 72% | miR-6501-3p | 55% | miR-10398-5p | 66% |
| miR-4268 | 66% | miR-6503-5p | 71% | miR-10398-3p | 84% |
| miR-4269 | 71% | miR-6506-5p | 52% | miR-10399-3p | 100% |
| miR-4270 | 63% | miR-6506-3p | 67% | miR-10400-5p | 66% |
| miR-4271 | 70% | miR-6508-5p | 56% | miR-10400-3p | 70% |
| miR-4276 | 64% | miR-6508-3p | 100% | miR-10401-5p | 78% |
| miR-4274 | 75% | miR-6509-3p | 55% | miR-10401-3p | 77% |
| miR-4281 | 67% | miR-6510-5p | 74% | miR-10396b-5p | 70% |
| miR-4279 | 72% | miR-6511a-5p | 76% | miR-10522-5p | 86% |
| miR-4278 | 57% | miR-6511a-3p | 65% | miR-10526-3p | 80% |
| miR-4280 | 55% | miR-6513-5p | 55% | miR-10527-5p | 81% |
| miR-4285 | 64% | miR-6514-3p | 56% | miR-11181-3p | 76% |
| miR-4283 | 52% | miR-6515-5p | 63% | miR-11399 | 72% |
| miR-4284 | 72% | miR-6515-3p | 78% | miR-11400 | 54% |
| miR-4286 | 72% | miR-6715b-3p | 67% | miR-3059-5p | 71% |
| miR-4287 | 60% | miR-6716-5p | 68% | miR-3059-3p | 51% |
| miR-4292 | 75% | miR-6716-3p | 58% | miR-3085-5p | 69% |
| miR-4290 | 70% | miR-6717-5p | 64% | miR-3085-3p | 71% |
| miR-4329 | 67% | miR-6511b-5p | 75% | miR-6529-5p | 54% |
| miR-3200-5p | 64% | miR-6511b-3p | 74% | miR-12114 | 64% |
| miR-3605-3p | 68% | miR-6719-3p | 60% | miR-12115 | 69% |
| miR-3610 | 80% | miR-6721-5p | 69% | miR-12116 | 77% |
| miR-3612 | 69% | miR-6722-5p | 61% | miR-12118 | 67% |
| miR-3614-5p | 72% | miR-6722-3p | 68% | miR-12119 | 80% |
| miR-3616-3p | 57% | miR-6724-5p | 68% | miR-12120 | 69% |
| miR-3620-3p | 66% | miR-208a-5p | 56% | miR-12121 | 64% |
| miR-3621 | 66% | miR-210-5p | 72% | miR-12122 | 55% |
| miR-3622a-5p | 59% | miR-128-1-5p | 64% | miR-12124 | 52% |
| miR-3622a-3p | 66% | miR-133a-5p | 100% | miR-12126 | 64% |
| miR-3622b-5p | 75% | miR-152-5p | 67% | miR-12131 | 100% |
| miR-3648 | 81% | | | miR-12136 | 69% |

### [Table22-4]

**Table22-4: miRNA to Predict Human Pancreatic Cancer of Stage I**

| miRNA Name | A | miRNA Name | A | miRNA Name | A |
|---|---|---|---|---|---|
| miR-15a-5p | 67% | miR-3622a-5p | 56% | miR-6501-3p | 53% |
| miR-19b-3p | 100% | miR-3648 | 77% | miR-6506-5p | 60% |
| miR-27a-3p | 55% | miR-3652 | 65% | miR-6506-3p | 75% |
| miR-28-5p | 52% | miR-3660 | 59% | miR-6510-5p | 71% |
| miR-29a-3p | 68% | miR-3663-3p | 71% | miR-6512-3p | 55% |
| miR-30a-5p | 67% | miR-3665 | 67% | miR-6513-5p | 62% |
| miR-196a-5p | 67% | miR-3666 | 52% | miR-6515-5p | 62% |
| miR-10a-5p | 56% | miR-3667-5p | 57% | miR-6515-3p | 79% |
| miR-34a-5p | 51% | miR-3675-3p | 74% | miR-6715b-3p | 70% |
| miR-181c-5p | 100% | miR-3679-3p | 75% | miR-6716-5p | 70% |
| miR-183-5p | 75% | miR-3689a-3p | 60% | miR-6717-5p | 70% |
| miR-210-3p | 54% | miR-3180 | 67% | miR-6511b-3p | 77% |
| miR-133a-3p | 71% | miR-3689b-3p, miR-3689c | 60% | miR-6719-3p | 67% |
| miR-154-3p | 54% | miR-3908 | 63% | miR-6721-5p | 76% |
| miR-184 | 51% | miR-3919 | 68% | miR-6722-3p | 65% |
| miR-206 | 58% | miR-3150b-3p | 59% | miR-6724-5p | 71% |
| miR-106b-5p | 80% | miR-3922-3p | 55% | miR-210-5p | 76% |
| miR-29c-3p | 100% | miR-676-3p | 54% | miR-328-5p | 65% |
| miR-302a-3p | 100% | miR-3936 | 57% | miR-410-5p | 61% |
| miR-34c-5p | 59% | miR-3937 | 73% | miR-489-5p | 56% |
| miR-299-3p | 70% | miR-3943 | 72% | miR-494-5p | 51% |
| miR-296-5p | 74% | miR-3945 | 57% | miR-598-5p | 61% |
| miR-130b-3p | 83% | miR-642b-3p | 65% | miR-605-3p | 74% |
| miR-361-5p | 56% | miR-1268b | 76% | miR-891a-3p | 55% |
| miR-365a-3p, miR-365b-3p | 71% | miR-4418 | 100% | miR-874-5p | 75% |
| miR-302c-5p | 72% | miR-4421 | 63% | miR-1343-5p | 71% |
| miR-373-5p | 71% | miR-4428 | 78% | miR-5189-3p | 59% |
| miR-373-3p | 64% | miR-4429 | 76% | miR-6727-5p | 67% |
| miR-378a-3p | 68% | miR-4431 | 55% | miR-6729-5p | 71% |
| miR-379-5p | 80% | miR-4433a-3p | 74% | miR-6729-3p | 75% |
| miR-328-3p | 75% | miR-4436a | 69% | miR-6730-5p | 83% |
| miR-326 | 70% | miR-4437 | 63% | miR-6731-3p | 76% |
| miR-151a-3p | 57% | miR-4439 | 68% | miR-6734-3p | 70% |
| miR-325 | 63% | miR-4441 | 61% | miR-6737-5p | 65% |
| miR-422a | 53% | miR-4444 | 70% | miR-6738-5p | 73% |
| miR-424-5p | 56% | miR-4446-3p | 69% | miR-6740-5p | 66% |
| miR-200a-5p | 59% | miR-4447 | 76% | miR-6741-5p | 78% |
| miR-323b-5p | 55% | miR-4449 | 62% | miR-6742-5p | 88% |
| miR-484 | 76% | miR-4451 | 51% | miR-6743-5p | 75% |
| miR-202-3p | 66% | miR-4454 | 51% | miR-6745 | 78% |
| miR-492 | 58% | miR-4456 | 65% | miR-6746-5p | 91% |
| miR-498-5p | 86% | miR-4458 | 69% | miR-6747-5p | 73% |
| miR-520e-3p | 82% | miR-378h | 60% | miR-6749-5p | 76% |
| miR-515-3p | 57% | miR-3135b | 63% | miR-6749-3p | 75% |
| miR-519c-5p, miR-523-5p, miR-518e-5p, miR-522-5p, miR-519a-5p, miR-519b-5p | 55% | miR-4463 | 69% | miR-6750-5p | 54% |
| miR-520b-3p | 83% | miR-4466 | 73% | miR-6752-5p | 67% |
| miR-518b | 75% | miR-4472 | 82% | miR-6752-3p | 72% |
| miR-520c-3p | 78% | miR-4474-3p | 51% | miR-6756-5p | 67% |
| miR-516b-3p, miR-516a-3p | 59% | miR-4478 | 75% | miR-6756-3p | 75% |
| miR-522-3p | 54% | miR-3689f | 56% | miR-6757-5p | 70% |
| miR-500a-3p | 58% | miR-3155b | 62% | miR-6759-3p | 76% |
| miR-532-5p | 80% | miR-4480 | 100% | miR-6760-5p | 89% |
| miR-554 | 57% | miR-4481 | 72% | miR-6760-3p | 78% |
| miR-555 | 56% | miR-4487 | 75% | miR-6762-3p | 61% |
| miR-557 | 68% | miR-4488 | 67% | miR-6763-3p | 74% |
| miR-564 | 64% | miR-4489 | 61% | miR-6765-5p | 66% |
| miR-571 | 100% | miR-4492 | 70% | miR-6766-3p | 76% |
| miR-575 | 62% | miR-4498 | 69% | miR-6767-5p | 64% |
| miR-579-3p | 62% | miR-4499 | 86% | miR-6768-5p | 67% |
| miR-580-3p | 64% | miR-4505 | 67% | miR-6769a-5p | 70% |
| miR-583 | 64% | miR-4506 | 63% | miR-6771-5p | 72% |
| miR-584-5p | 53% | miR-4507 | 72% | miR-6772-5p | 69% |
| miR-585-3p | 68% | miR-4508 | 69% | miR-6775-5p | 74% |
| miR-587 | 100% | miR-4516 | 74% | miR-6775-3p | 76% |
| miR-595 | 61% | miR-4520-3p | 63% | miR-6776-5p | 77% |
| miR-601 | 53% | miR-4525 | 77% | miR-6777-5p | 79% |
| miR-608 | 66% | miR-4534 | 83% | miR-6777-3p | 77% |
| miR-610 | 60% | miR-4535 | 63% | miR-6779-5p | 75% |
| miR-617 | 66% | miR-4538 | 63% | miR-6781-5p | 75% |
| miR-619-3p | 100% | miR-4540 | 57% | miR-6782-3p | 77% |
| miR-622 | 54% | miR-3127-3p | 74% | miR-6783-5p | 64% |
| miR-628-3p | 58% | miR-3162-3p | 75% | miR-6784-5p | 68% |
| miR-638 | 71% | miR-3619-3p | 79% | miR-6784-3p | 78% |
| miR-649 | 55% | miR-3691-3p | 52% | miR-6785-5p | 71% |
| miR-650 | 55% | miR-3150b-5p | 69% | miR-6785-3p | 75% |
| miR-663a | 71% | miR-3922-5p | 54% | miR-6786-5p | 71% |
| miR-449b-5p | 59% | miR-3925-3p | 55% | miR-6787-5p | 67% |
| miR-425-5p | 53% | miR-3940-5p | 66% | miR-6788-5p | 63% |
| miR-671-5p | 69% | miR-3960 | 65% | miR-6788-3p | 70% |
| miR-769-5p | 55% | miR-4634 | 65% | miR-6789-5p | 73% |
| miR-769-3p | 57% | miR-4638-5p | 71% | miR-6790-5p | 63% |
| miR-765 | 76% | miR-4640-3p | 68% | miR-6790-3p | 76% |
| miR-675-5p | 63% | miR-4644 | 59% | miR-6791-5p | 70% |
| miR-297 | 59% | miR-4647 | 55% | miR-6792-3p | 77% |
| let-7b-3p | 71% | miR-4648 | 57% | miR-6793-5p | 63% |
| let-7d-3p | 56% | miR-4649-5p | 64% | miR-6793-3p | 77% |
| let-7f-1-3p | 74% | miR-4649-3p | 73% | miR-6794-5p | 86% |
| miR-24-2-5p | 64% | miR-4650-3p | 80% | miR-6794-3p | 77% |
| miR-25-5p | 61% | miR-4651 | 72% | miR-6795-5p | 73% |
| miR-26b-3p | 60% | miR-4654 | 57% | miR-6795-3p | 74% |
| miR-27a-5p | 67% | miR-4656 | 70% | miR-6796-3p | 77% |
| miR-29a-5p | 100% | miR-4657 | 60% | miR-6797-5p | 77% |
| miR-92a-2-5p | 59% | miR-4665-5p | 82% | miR-6797-3p | 75% |
| miR-30c-2-3p | 79% | miR-4665-3p | 73% | miR-6798-5p | 70% |
| miR-139-3p | 65% | miR-4667-5p | 77% | miR-6798-3p | 77% |
| miR-15b-3p | 62% | miR-4667-3p | 75% | miR-6799-5p | 66% |
| miR-130a-5p | 60% | miR-4670-5p | 63% | miR-6800-5p | 66% |
| miR-132-5p | 83% | miR-4672 | 59% | miR-6800-3p | 75% |
| miR-135a-3p | 61% | miR-4674 | 67% | miR-6801-5p | 57% |
| miR-140-3p | 55% | miR-4676-5p | 63% | miR-6801-3p | 77% |
| miR-149-3p | 71% | miR-4677-5p | 67% | miR-6802-3p | 75% |
| miR-150-3p | 76% | miR-4681 | 51% | miR-6803-5p | 70% |
| miR-186-3p | 64% | miR-4682 | 52% | miR-6805-5p | 71% |
| miR-193a-5p | 66% | miR-4685-5p | 75% | miR-6805-3p | 77% |
| miR-194-3p | 61% | miR-4686 | 53% | miR-6807-5p | 68% |
| miR-34c-3p | 51% | miR-4687-5p | 78% | miR-6808-3p | 69% |
| miR-99b-3p | 52% | miR-4687-3p | 63% | miR-6809-5p | 81% |
| miR-361-3p | 71% | miR-4688 | 68% | miR-6810-3p | 76% |
| miR-371a-5p | 70% | miR-4689 | 69% | miR-6812-5p | 76% |
| miR-374a-3p | 100% | miR-4691-5p | 54% | miR-6812-3p | 77% |
| miR-323a-5p | 64% | miR-4691-3p | 83% | miR-6813-3p | 75% |
| miR-338-5p | 51% | miR-4692 | 56% | miR-6815-5p | 70% |
| miR-339-3p | 73% | miR-4697-5p | 71% | miR-6816-5p | 68% |
| miR-18b-3p | 74% | miR-4700-5p | 59% | miR-6819-3p | 75% |
| miR-20b-3p | 72% | miR-4701-3p | 71% | miR-6821-5p | 70% |
| miR-486-3p | 72% | miR-4703-5p | 100% | miR-6822-3p | 53% |
| miR-193b-5p | 52% | miR-4706 | 78% | miR-6823-5p | 62% |
| miR-92b-5p | 74% | miR-4707-5p | 68% | miR-6826-3p | 73% |
| miR-616-3p | 55% | miR-4708-5p | 54% | miR-6829-5p | 73% |
| miR-625-3p | 72% | miR-4708-3p | 54% | miR-6831-5p | 74% |
| miR-629-5p | 83% | miR-203b-5p | 61% | miR-6832-5p | 77% |
| miR-891b | 80% | miR-4716-5p | 77% | miR-6834-5p | 70% |
| miR-888-3p | 52% | miR-4720-5p | 57% | miR-6836-3p | 79% |
| miR-892b | 51% | miR-4722-5p | 70% | miR-6837-5p | 64% |
| miR-708-5p | 58% | miR-4723-5p | 76% | miR-6838-5p | 64% |
| miR-147b-3p | 55% | miR-4723-3p | 75% | miR-6839-5p | 60% |
| miR-885-3p | 63% | miR-4724-5p | 54% | miR-6841-3p | 73% |
| miR-877-5p | 71% | miR-4728-5p | 68% | miR-6845-5p | 69% |
| miR-877-3p | 78% | miR-4728-3p | 74% | miR-6845-3p | 80% |
| miR-665 | 71% | miR-4730 | 62% | miR-6846-3p | 74% |
| miR-921 | 55% | miR-4731-5p | 65% | miR-6847-5p | 58% |
| miR-924 | 53% | miR-4731-3p | 73% | miR-6848-5p | 72% |
| miR-933 | 72% | miR-4734 | 74% | miR-6848-3p | 71% |
| miR-936 | 74% | miR-4736 | 60% | miR-6850-5p | 74% |
| miR-940 | 71% | miR-4737 | 53% | miR-6851-5p | 69% |
| miR-1224-3p | 79% | miR-3064-5p | 58% | miR-6853-5p | 51% |
| miR-1225-5p | 71% | miR-4738-3p | 56% | miR-6855-5p | 60% |
| miR-1225-3p | 74% | miR-4739 | 67% | miR-6855-3p | 77% |
| miR-1227-3p | 72% | miR-4741 | 82% | miR-6858-5p | 69% |
| miR-1228-5p | 67% | miR-122b-3p | 55% | miR-6858-3p | 76% |
| miR-1228-3p | 75% | miR-4747-5p | 78% | miR-6859-5p | 58% |
| miR-1234-3p | 73% | miR-4748 | 69% | miR-6859-3p | 74% |
| miR-1236-3p | 78% | miR-4749-5p | 70% | miR-6769b-5p | 71% |
| miR-1237-3p | 76% | miR-4749-3p | 74% | miR-6769b-3p | 63% |
| miR-1238-3p | 77% | miR-371b-5p | 66% | miR-6861-3p | 77% |
| miR-320c | 70% | miR-371b-3p | 69% | miR-6865-3p | 70% |
| miR-1298-5p | 61% | miR-4754 | 53% | miR-6868-3p | 59% |
| miR-1202 | 70% | miR-4755-3p | 72% | miR-6869-5p | 65% |
| miR-1207-5p | 85% | miR-499b-3p | 56% | miR-6870-3p | 73% |
| miR-1285-3p | 53% | miR-4756-5p | 71% | miR-6871-5p | 71% |
| miR-1289 | 83% | miR-4758-5p | 80% | miR-6875-5p | 61% |
| miR-1293 | 57% | miR-4758-3p | 73% | miR-6876-5p | 73% |
| miR-1294 | 68% | miR-4763-5p | 71% | miR-6876-3p | 54% |
| miR-1249-3p | 76% | miR-4763-3p | 70% | miR-6880-3p | 76% |
| miR-1257 | 55% | miR-4769-5p | 78% | miR-6884-3p | 73% |
| miR-1268a | 69% | miR-4769-3p | 71% | miR-6885-3p | 75% |
| miR-1270 | 61% | miR-4771 | 53% | miR-6887-5p | 74% |
| miR-1281 | 78% | miR-4776-3p | 52% | miR-6887-3p | 75% |
| miR-320d | 83% | miR-4778-5p | 73% | miR-6889-3p | 74% |
| miR-1469 | 66% | miR-4779 | 75% | miR-6891-5p | 71% |
| miR-1470 | 77% | miR-4436b-3p | 56% | miR-6891-3p | 79% |
| miR-1471 | 66% | miR-4781-5p | 100% | miR-6892-5p | 77% |
| miR-1908-5p | 73% | miR-4783-3p | 78% | miR-6892-3p | 77% |
| miR-1909-3p | 68% | miR-4784 | 61% | miR-7106-5p | 80% |
| miR-1912-3p | 69% | miR-2467-3p | 72% | miR-7107-5p | 82% |
| miR-1913 | 76% | miR-4787-5p | 69% | miR-7108-5p | 71% |
| miR-1914-3p | 85% | miR-4788 | 69% | miR-7108-3p | 74% |
| miR-1915-3p | 68% | miR-4795-3p | 100% | miR-7109-5p | 70% |
| miR-205-3p | 67% | miR-642a-3p | 67% | miR-7111-5p | 82% |
| miR-365a-5p | 77% | miR-550a-3-5p | 52% | miR-7111-3p | 78% |
| miR-2110 | 78% | miR-4433a-5p | 73% | miR-7113-5p | 59% |
| miR-151b | 53% | miR-4999-5p | 62% | miR-7113-3p | 74% |
| miR-762 | 70% | miR-5000-3p | 55% | miR-7114-3p | 76% |
| miR-2116-3p | 70% | miR-5002-3p | 66% | miR-7150 | 86% |
| miR-2276-3p | 78% | miR-5003-3p | 67% | miR-7151-3p | 69% |
| miR-711 | 78% | miR-5004-5p | 54% | miR-7157-3p | 72% |
| miR-718 | 78% | miR-5006-5p | 54% | miR-7159-5p | 71% |
| miR-2681-3p | 63% | miR-5006-3p | 54% | miR-7160-5p | 71% |
| miR-2861 | 64% | miR-5009-3p | 61% | miR-7161-5p | 52% |
| miR-3120-3p | 59% | miR-5087 | 59% | miR-7162-5p | 53% |
| miR-3124-5p | 53% | miR-5093 | 61% | miR-7515 | 72% |
| miR-3125 | 56% | miR-5188 | 60% | miR-7704 | 67% |
| miR-3131 | 79% | miR-5189-5p | 67% | miR-4433b-5p | 75% |
| miR-3137 | 56% | miR-5194 | 51% | miR-4433b-3p | 79% |
| miR-3141 | 74% | miR-5195-3p | 68% | miR-7845-5p | 73% |
| miR-3144-5p | 76% | miR-5196-5p | 74% | miR-7847-3p | 71% |
| miR-3148 | 61% | miR-4524b-5p | 53% | miR-7850-5p | 51% |
| miR-3154 | 75% | miR-548at-5p | 56% | miR-7851-3p | 62% |
| miR-3169 | 62% | miR-5588-5p | 54% | miR-7856-5p | 57% |
| miR-3173-3p | 57% | miR-5684 | 69% | miR-8054 | 80% |
| miR-3178 | 69% | miR-5690 | 64% | miR-8057 | 51% |
| miR-3180-5p | 71% | miR-5703 | 62% | miR-8064 | 51% |
| miR-3180-3p | 70% | miR-5708 | 54% | miR-8069 | 66% |
| miR-3186-3p | 53% | miR-197-5p | 71% | miR-8070 | 54% |
| miR-3192-5p | 53% | miR-211-3p | 68% | miR-8071 | 81% |
| miR-3196 | 68% | miR-301a-5p | 56% | miR-8072 | 70% |
| miR-3197 | 72% | miR-1185-2-3p | 75% | miR-8074 | 63% |
| miR-3199 | 52% | miR-766-5p | 61% | miR-8075 | 61% |
| miR-514b-5p | 75% | miR-1304-3p | 76% | miR-8077 | 66% |
| miR-4296 | 61% | miR-3184-3p | 78% | miR-8078 | 51% |
| miR-4293 | 64% | miR-1185-1-3p | 76% | miR-8079 | 57% |
| miR-4294 | 75% | miR-381-5p | 55% | miR-8080 | 62% |
| miR-4299 | 64% | miR-758-5p | 55% | miR-128-2-5p | 71% |
| miR-4298 | 74% | miR-1227-5p | 74% | miR-7976 | 51% |
| miR-4306 | 73% | miR-1229-5p | 64% | miR-203a-5p | 64% |
| miR-4313 | 73% | miR-1233-5p | 73% | miR-1-5p | 52% |
| miR-4316 | 62% | miR-1237-5p | 70% | miR-1249-5p | 70% |
| miR-4314 | 59% | miR-1238-5p | 79% | miR-135a-2-3p | 56% |
| miR-4318 | 52% | miR-3617-3p | 52% | miR-498-3p | 68% |
| miR-4321 | 61% | miR-3620-5p | 68% | miR-9718 | 71% |
| miR-4323 | 76% | miR-3934-3p | 60% | miR-9898 | 75% |
| miR-4256 | 60% | miR-4632-5p | 72% | miR-9986 | 51% |
| miR-4257 | 79% | miR-4743-3p | 58% | miR-10392-3p | 80% |
| miR-4258 | 71% | miR-4750-3p | 74% | miR-10396a-5p | 67% |
| miR-4252 | 51% | miR-5739 | 76% | miR-10396a-3p | 69% |
| miR-4325 | 67% | miR-5787 | 61% | miR-10400-5p | 67% |
| miR-4327 | 76% | miR-6068 | 56% | miR-10400-3p | 66% |
| miR-4261 | 52% | miR-6069 | 73% | miR-10401-5p | 81% |
| miR-4265 | 61% | miR-6076 | 83% | miR-10396b-5p | 70% |
| miR-4271 | 70% | miR-6081 | 52% | miR-10522-5p | 85% |
| miR-4276 | 62% | miR-6083 | 53% | miR-10526-3p | 88% |
| miR-4274 | 74% | miR-6085 | 74% | miR-11181-3p | 75% |
| miR-4281 | 69% | miR-6086 | 84% | miR-11399 | 68% |
| miR-4280 | 68% | miR-6088 | 70% | miR-11401 | 63% |
| miR-4285 | 60% | miR-6089 | 70% | miR-3059-3p | 61% |
| miR-4284 | 72% | miR-6090 | 68% | miR-3085-3p | 73% |
| miR-4286 | 71% | miR-6124 | 72% | miR-6529-5p | 71% |
| miR-4288 | 51% | miR-6125 | 71% | miR-12114 | 62% |
| miR-4289 | 59% | miR-378j | 59% | miR-12116 | 75% |
| miR-500b-5p | 53% | miR-6129 | 52% | miR-12118 | 77% |
| miR-2277-5p | 51% | miR-6130 | 54% | miR-12120 | 66% |
| miR-3614-5p | 71% | miR-6132 | 65% | miR-12122 | 53% |
| miR-3621 | 71% | miR-6165 | 72% | miR-12124 | 52% |
| | | miR-6500-5p | 52% | | |
| | | miR-6500-3p | 63% | | |
| miR-29a-3p | 59% | miR-3928-3p | 68% | miR-6089 | 67% |

### [Table 22-5]

**Table 22-5: miRNA to Predict Human Prostate Cancer of Stage I**

| miRNA Name | A | miRNA Name | A | miRNA Name | A |
|---|---|---|---|---|---|
| miR-29a-3p | 59% | miR-2116-5p | 71% | miR-6515-5p | 57% |
| miR-105-5p | 61% | miR-3124-5p | 66% | miR-6717-5p | 56% |
| miR-129-5p | 57% | miR-378b | 51% | miR-6719-3p | 60% |
| let-7i-5p | 60% | miR-3154 | 69% | miR-372-5p | 100% |
| miR-133a-3p | 64% | miR-3155a | 58% | miR-605-3p | 64% |
| miR-142-3p | 53% | miR-3180-5p | 68% | miR-619-5p | 53% |
| miR-106b-5p | 75% | miR-4295 | 53% | miR-1301-5p | 55% |
| miR-130b-3p | 80% | miR-4311 | 52% | miR-1251-3p | 54% |
| miR-30e-5p | 100% | miR-4313 | 66% | miR-6729-3p | 66% |
| miR-365a-3p, miR-365b-3p | 65% | miR-4315 | 52% | miR-6731-3p | 63% |
| miR-371a-3p | 56% | miR-4314 | 52% | miR-6747-5p | 55% |
| miR-378a-3p | 59% | miR-4321 | 53% | miR-6748-5p | 71% |
| miR-135b-5p | 53% | miR-4323 | 66% | miR-6750-5p | 69% |
| miR-148b-3p | 67% | miR-4284 | 65% | miR-6756-3p | 67% |
| miR-492 | 55% | miR-4286 | 68% | miR-6760-3p | 68% |
| miR-494-3p | 53% | miR-3660 | 52% | miR-6763-3p | 68% |
| miR-498-5p | 69% | miR-3666 | 60% | miR-6769a-5p | 66% |
| miR-518b | 69% | miR-3667-5p | 56% | miR-6770-5p | 54% |
| miR-520c-3p | 60% | miR-3677-3p | 70% | miR-6775-3p | 70% |
| miR-584-5p | 52% | miR-3679-3p | 68% | miR-6783-5p | 57% |
| miR-585-3p | 60% | miR-3713 | 51% | miR-6784-3p | 69% |
| miR-590-5p | 100% | miR-3909 | 53% | miR-6785-3p | 68% |
| miR-601 | 57% | miR-3918 | 58% | miR-6788-5p | 52% |
| miR-608 | 51% | miR-4421 | 59% | miR-6790-3p | 69% |
| miR-614 | 62% | miR-4441 | 61% | miR-6795-3p | 68% |
| miR-624-5p | 62% | miR-4460 | 83% | miR-6796-3p | 71% |
| miR-769-3p | 69% | miR-4514 | 56% | miR-6805-3p | 69% |
| let-7b-3p | 58% | miR-4521 | 62% | miR-6808-3p | 64% |
| let-7f-1-3p | 65% | miR-4538 | 54% | miR-6810-3p | 66% |
| miR-29a-5p | 100% | miR-3127-3p | 70% | miR-6812-5p | 69% |
| miR-92a-2-5p | 70% | miR-3152-5p | 54% | miR-6812-3p | 64% |
| miR-30c-2-3p | 58% | miR-3162-3p | 68% | miR-6813-3p | 67% |
| miR-150-3p | 68% | miR-3150b-5p | 69% | miR-6819-3p | 66% |
| miR-374a-3p | 100% | miR-4648 | 56% | miR-6832-5p | 58% |
| miR-323a-5p | 57% | miR-4649-3p | 68% | miR-6836-3p | 73% |
| miR-339-3p | 57% | miR-4673 | 67% | miR-6841-3p | 68% |
| miR-18b-3p | 67% | miR-4684-3p | 68% | miR-6858-3p | 63% |
| miR-551b-5p | 57% | miR-4701-3p | 53% | miR-6859-3p | 66% |
| miR-548b-5p | 100% | miR-4714-3p | 100% | miR-6870-3p | 64% |
| miR-629-5p | 83% | miR-4716-5p | 67% | miR-6871-5p | 57% |
| miR-411-3p | 59% | miR-4729 | 100% | miR-6876-5p | 63% |
| miR-891b | 75% | miR-4755-3p | 60% | miR-6891-3p | 63% |
| miR-541-3p | 52% | miR-4762-5p | 63% | miR-6892-3p | 66% |
| miR-509-3-5p | 100% | miR-4436b-3p | 52% | miR-7106-5p | 69% |
| miR-944 | 100% | miR-1245b-5p | 100% | miR-7108-3p | 68% |
| miR-1227-3p | 63% | miR-550a-3-5p | 56% | miR-7111-5p | 71% |
| miR-1228-3p | 66% | miR-4433a-5p | 69% | miR-7114-3p | 67% |
| miR-1237-3p | 67% | miR-5009-3p | 59% | miR-7156-3p | 68% |
| miR-1238-3p | 64% | miR-5196-5p | 72% | miR-4433b-5p | 70% |
| miR-513c-5p | 56% | miR-5571-3p | 56% | miR-7851-3p | 58% |
| miR-1289 | 63% | miR-5580-3p | 54% | miR-8078 | 55% |
| miR-1303 | 62% | miR-5695 | 75% | miR-9898 | 67% |
| miR-1249-3p | 69% | miR-766-5p | 52% | miR-9900 | 100% |
| miR-1250-5p | 55% | miR-1304-3p | 62% | miR-10392-3p | 70% |
| miR-1324 | 55% | miR-550b-2-5p | 56% | miR-10522-5p | 80% |
| miR-320d | 67% | miR-4750-3p | 67% | miR-10526-3p | 57% |
| miR-1470 | 68% | miR-6074 | 53% | miR-12116 | 67% |
| miR-1972 | 68% | miR-6084 | 60% | miR-12118 | 68% |
| miR-449c-5p | 65% | miR-6506-3p | 80% | miR-12122 | 69% |

### [Table 22-6]

**Table 22-6: miRNA to Predict Human Stomach Cancer of Stage I**

| miRNA Name | A | miRNA Name | A | miRNA Name | A |
|---|---|---|---|---|---|
| miR-29a-3p | 61% | miR-4257 | 72% | miR-6501-3p | 54% |
| miR-182-5p | 57% | miR-4258 | 80% | miR-6513-5p | 62% |
| miR-299-3p | 61% | miR-4327 | 80% | miR-6514-5p | 57% |
| miR-130b-3p | 88% | miR-4261 | 59% | miR-6515-5p | 59% |
| miR-361-5p | 58% | miR-4280 | 57% | miR-6715b-3p | 57% |
| miR-200a-5p | 54% | miR-4285 | 69% | miR-6719-3p | 60% |
| miR-202-3p | 66% | miR-3200-5p | 72% | miR-181d-3p | 86% |
| miR-492 | 63% | miR-3648 | 79% | miR-598-5p | 61% |
| miR-494-3p | 61% | miR-3651 | 56% | miR-605-3p | 70% |
| miR-498-5p | 74% | miR-3660 | 60% | miR-668-5p | 75% |
| miR-519c-5p, miR-523-5p, miR-518e-5p, miR-522-5p, miR-519a-5p, miR-519b-5p | 56% | miR-3666 | 62% | miR-1343-5p | 78% |
| miR-525-5p | 54% | miR-3677-3p | 81% | miR-6729-5p | 79% |
| miR-516b-5p | 80% | miR-3907 | 88% | miR-6731-3p | 69% |
| miR-554 | 59% | miR-3909 | 71% | miR-6732-5p | 77% |
| miR-576-5p | 54% | miR-3918 | 51% | miR-6743-5p | 77% |
| miR-601 | 62% | miR-3937 | 79% | miR-6746-5p | 73% |
| miR-611 | 71% | miR-1268b | 83% | miR-6750-5p | 55% |
| miR-617 | 54% | miR-4429 | 77% | miR-6752-5p | 74% |
| miR-628-3p | 62% | miR-4433a-3p | 78% | miR-6756-5p | 75% |
| miR-638 | 80% | miR-4435 | 65% | miR-6760-3p | 74% |
| miR-639 | 88% | miR-4439 | 67% | miR-6763-5p | 70% |
| miR-650 | 58% | miR-4447 | 78% | miR-6770-5p | 60% |
| miR-449b-5p | 57% | miR-4454 | 51% | miR-6770-3p | 74% |
| miR-421 | 55% | miR-4458 | 56% | miR-6777-5p | 71% |
| miR-542-5p | 90% | miR-4465 | 71% | miR-6781-5p | 73% |
| let-7f-1-3p | 76% | miR-4466 | 76% | miR-6783-5p | 60% |
| miR-25-5p | 69% | miR-4470 | 89% | miR-6784-5p | 71% |
| miR-92a-2-5p | 77% | miR-4479 | 64% | miR-6786-5p | 75% |
| miR-181c-3p | 67% | miR-4484 | 72% | miR-6789-5p | 81% |
| miR-187-5p | 71% | miR-4485-3p | 75% | miR-6790-3p | 76% |
| miR-15b-3p | 58% | miR-4505 | 80% | miR-6791-5p | 81% |
| miR-124-5p | 80% | miR-4507 | 82% | miR-6794-5p | 66% |
| miR-125b-1-3p | 100% | miR-4512 | 75% | miR-6797-5p | 73% |
| miR-135a-3p | 63% | miR-4516 | 72% | miR-6798-5p | 75% |
| miR-150-3p | 66% | miR-4521 | 75% | miR-6799-5p | 69% |
| miR-193a-5p | 59% | miR-1269b | 65% | miR-6805-5p | 75% |
| miR-339-3p | 73% | miR-4530 | 76% | miR-6809-5p | 76% |
| miR-424-3p | 75% | miR-3127-3p | 73% | miR-6810-3p | 74% |
| miR-20b-3p | 68% | miR-4634 | 78% | miR-6814-5p | 51% |
| miR-490-5p | 100% | miR-4635 | 55% | miR-6815-5p | 69% |
| miR-193b-5p | 61% | miR-4650-3p | 75% | miR-6821-5p | 77% |
| miR-92b-5p | 81% | miR-4651 | 77% | miR-6829-5p | 73% |
| miR-551b-5p | 57% | miR-4658 | 64% | miR-6780b-5p | 66% |
| miR-629-5p | 87% | miR-4665-5p | 77% | miR-6836-5p | 65% |
| miR-541-3p | 57% | miR-4672 | 57% | miR-6836-3p | 78% |
| miR-665 | 79% | miR-4673 | 72% | miR-6838-5p | 57% |
| miR-921 | 65% | miR-4677-5p | 63% | miR-6839-5p | 54% |
| miR-939-5p | 76% | miR-4695-5p | 72% | miR-6840-3p | 74% |
| miR-1225-5p | 74% | miR-4706 | 71% | miR-6845-5p | 75% |
| miR-513b-5p | 62% | miR-4707-5p | 81% | miR-6848-5p | 72% |
| miR-320c | 63% | miR-4720-5p | 57% | miR-6850-5p | 76% |
| miR-1301-3p | 63% | miR-4725-3p | 68% | miR-6850-3p | 52% |
| miR-1298-5p | 59% | miR-4727-3p | 65% | miR-6861-5p | 72% |
| miR-1207-5p | 76% | miR-4741 | 83% | miR-6875-5p | 76% |
| miR-1303 | 71% | miR-4742-3p | 67% | miR-6892-3p | 71% |
| miR-1304-5p | 58% | miR-4745-5p | 78% | miR-6893-5p | 69% |
| miR-1243 | 100% | miR-4745-3p | 57% | miR-7107-5p | 75% |
| miR-1270 | 55% | miR-4749-5p | 77% | miR-7111-5p | 72% |
| miR-1292-5p | 88% | miR-4755-3p | 68% | miR-7114-5p | 67% |
| miR-1469 | 79% | miR-4758-5p | 78% | miR-7150 | 77% |
| miR-1908-5p | 79% | miR-4763-3p | 80% | miR-7515 | 59% |
| miR-1914-3p | 75% | miR-4766-5p | 57% | miR-4433b-3p | 74% |
| miR-1915-3p | 76% | miR-4778-5p | 67% | miR-6516-5p | 52% |
| miR-449c-5p | 57% | miR-550a-3-5p | 53% | miR-8072 | 76% |
| miR-762 | 75% | miR-4999-5p | 52% | miR-8074 | 55% |
| miR-2114-5p | 60% | miR-5009-3p | 57% | miR-1249-5p | 67% |
| miR-2114-3p | 100% | miR-5190 | 73% | miR-217-3p | 52% |
| miR-2681-3p | 59% | miR-5196-5p | 66% | miR-135a-2-3p | 53% |
| miR-3122 | 81% | miR-4524b-5p | 57% | miR-498-3p | 61% |
| miR-3124-5p | 71% | miR-5587-5p | 51% | miR-526a-3p | 100% |
| miR-3133 | 54% | miR-5698 | 70% | miR-10392-5p | 80% |
| miR-3141 | 71% | miR-211-3p | 79% | miR-10392-3p | 78% |
| miR-1273c | 75% | miR-381-5p | 52% | miR-10396a-3p | 81% |
| miR-3163 | 75% | miR-937-5p | 77% | miR-10400-3p | 77% |
| miR-3165 | 63% | miR-1227-5p | 80% | miR-10401-5p | 71% |
| miR-3065-5p | 67% | miR-3620-5p | 79% | miR-10396b-5p | 74% |
| miR-3187-3p | 77% | miR-6071 | 59% | miR-10522-5p | 80% |
| miR-320e | 53% | miR-6080 | 52% | miR-10526-3p | 74% |
| miR-3197 | 78% | miR-6081 | 78% | miR-11399 | 77% |
| miR-4295 | 51% | miR-6089 | 74% | miR-12119 | 68% |
| miR-4294 | 72% | miR-6125 | 77% | miR-12120 | 71% |
| miR-4315 | 57% | miR-6131 | 71% | miR-12122 | 60% |
| miR-4321 | 66% | miR-6500-3p | 52% | miR-12131 | 100% |
| | | miR-6501-5p | 58% | | |

### [Table 22-7]

**Table 22-7: miRNA to Predict Human Urothelial Cancer of Stage I**

| miRNA Name | A | miRNA Name | A | miRNA Name | A |
|---|---|---|---|---|---|
| let-7a-5p | 100% | miR-3907 | 83% | miR-598-5p | 68% |
| miR-15a-5p | 55% | miR-3908 | 63% | miR-605-3p | 64% |
| miR-17-3p | 100% | miR-3911 | 66% | miR-619-5p | 61% |
| miR-29a-3p | 59% | miR-3915 | 100% | miR-1296-3p | 69% |
| miR-30a-5p | 63% | miR-3917 | 73% | miR-874-5p | 76% |
| miR-103a-3p | 53% | miR-3919 | 65% | miR-887-5p | 66% |
| miR-196a-5p | 69% | miR-3150b-3p | 52% | miR-1266-3p | 56% |
| miR-198 | 67% | miR-3925-5p | 80% | miR-1343-5p | 75% |
| miR-10a-5p | 62% | miR-3926 | 69% | miR-5189-3p | 53% |
| miR-182-5p | 67% | miR-3928-3p | 76% | miR-5699-5p | 73% |
| miR-214-3p | 56% | miR-3935 | 55% | miR-6726-5p | 73% |
| miR-124-3p | 71% | miR-3937 | 81% | miR-6728-5p | 56% |
| miR-125b-5p | 52% | miR-3943 | 73% | miR-6728-3p | 76% |
| miR-140-5p | 75% | miR-3944-3p | 65% | miR-6729-5p | 65% |
| miR-141-3p | 63% | miR-3945 | 58% | miR-6729-3p | 75% |
| miR-134-5p | 69% | miR-642b-3p | 69% | miR-6730-5p | 71% |
| miR-206 | 56% | miR-1268b | 76% | miR-6730-3p | 71% |
| miR-106b-5p | 86% | miR-548ab | 67% | miR-6731-3p | 72% |
| miR-302a-3p | 100% | miR-4422 | 67% | miR-6732-5p | 76% |
| miR-299-3p | 56% | miR-4426 | 100% | miR-6734-5p | 71% |
| miR-296-5p | 73% | miR-4428 | 72% | miR-6735-5p | 69% |
| miR-130b-3p | 90% | miR-4429 | 62% | miR-6736-5p | 75% |
| miR-365a-3p, miR-365b-3p | 73% | miR-4430 | 72% | miR-6736-3p | 63% |
| miR-302c-5p | 57% | miR-4433a-3p | 75% | miR-6737-5p | 69% |
| miR-370-3p | 79% | miR-4436a | 57% | miR-6738-5p | 71% |
| miR-373-5p | 75% | miR-4439 | 68% | miR-6738-3p | 58% |
| miR-373-3p | 67% | miR-4440 | 71% | miR-6740-5p | 61% |
| miR-378a-3p | 55% | miR-4441 | 51% | miR-6740-3p | 61% |
| miR-380-3p | 63% | miR-4443 | 62% | miR-6741-5p | 80% |
| miR-383-5p | 67% | miR-4444 | 64% | miR-6742-5p | 70% |
| miR-328-3p | 70% | miR-4446-3p | 65% | miR-6742-3p | 74% |
| miR-342-3p | 51% | miR-4447 | 85% | miR-6743-5p | 76% |
| miR-133b | 74% | miR-4448 | 57% | miR-6744-5p | 63% |
| miR-325 | 52% | miR-4449 | 71% | miR-6745 | 56% |
| miR-448 | 54% | miR-4455 | 59% | miR-6746-5p | 75% |
| miR-429 | 100% | miR-4456 | 55% | miR-6747-5p | 65% |
| miR-449a | 60% | miR-4458 | 62% | miR-6747-3p | 66% |
| miR-200a-5p | 57% | miR-4460 | 80% | miR-6748-5p | 76% |
| miR-452-3p | 55% | miR-378h | 58% | miR-6749-5p | 81% |
| miR-484 | 74% | miR-3135b | 69% | miR-6749-3p | 70% |
| miR-486-5p | 75% | miR-4462 | 75% | miR-6751-5p | 73% |
| miR-491-5p | 86% | miR-4463 | 74% | miR-6752-5p | 77% |
| miR-202-3p | 56% | miR-4466 | 63% | miR-6752-3p | 76% |
| miR-492 | 57% | miR-4467 | 71% | miR-6753-5p | 65% |
| miR-512-5p | 53% | miR-4468 | 71% | miR-6754-5p | 71% |
| miR-498-5p | 79% | miR-4470 | 86% | miR-6754-3p | 59% |
| miR-515-3p | 52% | miR-4472 | 75% | miR-6755-5p | 80% |
| miR-518b | 77% | miR-4475 | 68% | miR-6756-5p | 80% |
| miR-518c-3p | 100% | miR-4476 | 60% | miR-6756-3p | 74% |
| miR-524-3p | 60% | miR-4478 | 73% | miR-6757-5p | 70% |
| miR-516b-5p | 86% | miR-3689d | 58% | miR-6758-5p | 58% |
| miR-518a-3p | 67% | miR-3155b | 58% | miR-6759-5p | 67% |
| miR-519a-3p | 71% | miR-4481 | 69% | miR-6759-3p | 74% |
| miR-499a-5p | 100% | miR-4483 | 76% | miR-6760-5p | 56% |
| miR-502-5p | 57% | miR-4484 | 76% | miR-6760-3p | 75% |
| miR-503-5p | 75% | miR-4485-3p | 75% | miR-6761-3p | 63% |
| miR-513a-5p | 54% | miR-4486 | 77% | miR-6762-5p | 66% |
| miR-532-5p | 83% | miR-4487 | 67% | miR-6762-3p | 57% |
| miR-554 | 52% | miR-4488 | 62% | miR-6763-5p | 82% |
| miR-555 | 59% | miR-4489 | 66% | miR-6763-3p | 80% |
| miR-556-5p | 100% | miR-4492 | 59% | miR-6765-5p | 65% |
| miR-557 | 70% | miR-4496 | 80% | miR-6766-5p | 75% |
| miR-563 | 59% | miR-4497 | 72% | miR-6766-3p | 76% |
| miR-564 | 57% | miR-4498 | 68% | miR-6767-5p | 55% |
| miR-571 | 100% | miR-4499 | 58% | miR-6768-5p | 73% |
| miR-574-3p | 68% | miR-4505 | 73% | miR-6769a-5p | 73% |
| miR-575 | 55% | miR-4506 | 53% | miR-6769a-3p | 73% |
| miR-583 | 51% | miR-2392 | 72% | miR-6770-5p | 57% |
| miR-585-3p | 54% | miR-4507 | 79% | miR-6772-5p | 69% |
| miR-595 | 54% | miR-4508 | 69% | miR-6772-3p | 57% |
| miR-608 | 53% | miR-4510 | 54% | miR-6773-5p | 67% |
| miR-610 | 53% | miR-4511 | 55% | miR-6774-5p | 70% |
| miR-612 | 56% | miR-4513 | 76% | miR-6775-5p | 70% |
| miR-615-3p | 55% | miR-4516 | 65% | miR-6775-3p | 72% |
| miR-617 | 61% | miR-4519 | 100% | miR-6776-5p | 77% |
| miR-618 | 100% | miR-4520-3p | 54% | miR-6776-3p | 75% |
| miR-628-3p | 60% | miR-4521 | 71% | miR-6777-5p | 75% |
| miR-632 | 54% | miR-4522 | 65% | miR-6777-3p | 76% |
| miR-634 | 73% | miR-4525 | 66% | miR-6778-5p | 76% |
| miR-637 | 78% | miR-4526 | 70% | miR-6778-3p | 59% |
| miR-638 | 70% | miR-4530 | 77% | miR-6779-5p | 70% |
| miR-639 | 88% | miR-4533 | 69% | miR-6781-5p | 74% |
| miR-641 | 100% | miR-4534 | 66% | miR-6782-5p | 73% |
| miR-649 | 53% | miR-4535 | 65% | miR-6782-3p | 77% |
| miR-652-3p | 100% | miR-1587 | 73% | miR-6783-5p | 61% |
| miR-663a | 72% | miR-4538 | 66% | miR-6783-3p | 58% |
| miR-449b-5p | 57% | miR-4539 | 78% | miR-6784-5p | 71% |
| miR-654-5p | 54% | miR-3127-3p | 75% | miR-6784-3p | 76% |
| miR-657 | 55% | miR-3158-5p | 71% | miR-6785-3p | 78% |
| miR-658 | 66% | miR-3162-3p | 76% | miR-6786-5p | 68% |
| miR-542-5p | 83% | miR-3173-5p | 59% | miR-6786-3p | 72% |
| miR-671-5p | 65% | miR-3187-5p | 63% | miR-6787-5p | 80% |
| miR-668-3p | 57% | miR-3619-3p | 73% | miR-6787-3p | 64% |
| miR-769-5p | 56% | miR-3150b-5p | 73% | miR-6789-5p | 70% |
| miR-766-3p | 77% | miR-3940-5p | 64% | miR-6789-3p | 57% |
| miR-765 | 68% | miR-3978 | 100% | miR-6790-5p | 69% |
| miR-675-5p | 64% | miR-4634 | 67% | miR-6790-3p | 75% |
| miR-297 | 56% | miR-4638-5p | 78% | miR-6791-5p | 75% |
| miR-22-5p | 100% | miR-4639-5p | 100% | miR-6792-5p | 71% |
| miR-92a-2-5p | 72% | miR-4639-3p | 53% | miR-6792-3p | 78% |
| miR-30c-2-3p | 50% | miR-4640-5p | 71% | miR-6793-5p | 67% |
| miR-139-3p | 62% | miR-4640-3p | 67% | miR-6793-3p | 76% |
| miR-181c-3p | 67% | miR-4646-5p | 75% | miR-6794-5p | 79% |
| miR-183-3p | 66% | miR-4648 | 57% | miR-6795-5p | 59% |
| miR-187-5p | 58% | miR-4649-3p | 71% | miR-6795-3p | 75% |
| miR-124-5p | 71% | miR-4650-3p | 71% | miR-6796-5p | 79% |
| miR-125b-1-3p | 100% | miR-4651 | 72% | miR-6796-3p | 79% |
| miR-132-5p | 67% | miR-4653-3p | 64% | miR-6797-5p | 77% |
| miR-135a-3p | 64% | miR-4654 | 55% | miR-6797-3p | 74% |
| miR-140-3p | 67% | miR-4655-5p | 67% | miR-6798-5p | 73% |
| miR-125a-3p | 74% | miR-4656 | 72% | miR-6798-3p | 75% |
| miR-149-3p | 71% | miR-4664-5p | 73% | miR-6799-5p | 76% |
| miR-150-3p | 78% | miR-4664-3p | 76% | miR-6800-5p | 66% |
| miR-185-3p | 71% | miR-4665-5p | 83% | miR-6800-3p | 76% |
| miR-186-3p | 64% | miR-4665-3p | 78% | miR-6801-3p | 77% |
| miR-193a-5p | 68% | miR-4667-5p | 79% | miR-6802-5p | 66% |
| miR-194-3p | 57% | miR-4667-3p | 71% | miR-6802-3p | 73% |
| miR-30c-1-3p | 52% | miR-4669 | 67% | miR-6803-5p | 69% |
| miR-296-3p | 74% | miR-4670-3p | 100% | miR-6804-3p | 70% |
| miR-361-3p | 75% | miR-4672 | 59% | miR-6805-5p | 70% |
| miR-371a-5p | 69% | miR-4673 | 54% | miR-6805-3p | 78% |
| miR-342-5p | 61% | miR-4674 | 63% | miR-6806-5p | 55% |
| miR-151a-5p | 51% | miR-4676-5p | 51% | miR-6806-3p | 100% |
| miR-423-5p | 69% | miR-4677-5p | 70% | miR-6807-5p | 67% |
| miR-424-3p | 71% | miR-4682 | 54% | miR-6808-5p | 74% |
| miR-18b-3p | 73% | miR-4685-5p | 69% | miR-6808-3p | 64% |
| miR-20b-3p | 68% | miR-4685-3p | 64% | miR-6809-5p | 74% |
| miR-483-5p | 71% | miR-4687-5p | 76% | miR-6809-3p | 67% |
| miR-486-3p | 65% | miR-4687-3p | 65% | miR-6810-5p | 75% |
| miR-490-5p | 100% | miR-1343-3p | 77% | miR-6810-3p | 73% |
| miR-193b-5p | 58% | miR-4688 | 67% | miR-6812-5p | 79% |
| miR-501-3p | 100% | miR-4689 | 66% | miR-6812-3p | 71% |
| miR-505-5p | 74% | miR-4690-5p | 76% | miR-6813-5p | 67% |
| miR-92b-5p | 75% | miR-4691-5p | 53% | miR-6813-3p | 76% |
| miR-551b-5p | 58% | miR-4695-5p | 74% | miR-6815-5p | 59% |
| miR-574-5p | 63% | miR-4697-5p | 67% | miR-6815-3p | 59% |
| miR-550a-5p | 64% | miR-4697-3p | 76% | miR-6816-5p | 65% |
| miR-615-5p | 76% | miR-4698 | 77% | miR-6819-5p | 65% |
| miR-616-3p | 55% | miR-4699-3p | 55% | miR-6819-3p | 73% |
| miR-624-3p | 71% | miR-4700-5p | 57% | miR-6820-5p | 70% |
| miR-625-3p | 77% | miR-4700-3p | 69% | miR-6820-3p | 76% |
| miR-629-5p | 89% | miR-4701-5p | 73% | miR-6821-5p | 78% |
| miR-298 | 80% | miR-4701-3p | 65% | miR-6822-5p | 70% |
| miR-874-3p | 68% | miR-4706 | 72% | miR-6822-3p | 52% |
| miR-891b | 80% | miR-4707-5p | 72% | miR-6823-5p | 58% |
| miR-892b | 51% | miR-4707-3p | 79% | miR-6824-5p | 75% |
| miR-876-3p | 83% | miR-4709-3p | 65% | miR-6825-5p | 72% |
| miR-744-5p | 62% | miR-4710 | 74% | miR-6826-3p | 71% |
| miR-885-3p | 63% | miR-4714-5p | 74% | miR-6827-5p | 63% |
| miR-877-5p | 67% | miR-4716-5p | 75% | miR-6828-5p | 67% |
| miR-877-3p | 73% | miR-4716-3p | 63% | miR-6829-5p | 77% |
| miR-887-3p | 68% | miR-4717-3p | 68% | miR-6830-5p | 79% |
| miR-665 | 64% | miR-4719 | 58% | miR-6831-5p | 72% |
| miR-760 | 73% | miR-4720-5p | 59% | miR-6832-5p | 72% |
| miR-920 | 81% | miR-4721 | 71% | miR-6833-5p | 68% |
| miR-509-3-5p | 100% | miR-4722-5p | 61% | miR-6834-5p | 73% |
| miR-936 | 63% | miR-4722-3p | 63% | miR-6780b-5p | 78% |
| miR-939-5p | 79% | miR-4723-5p | 69% | miR-6836-3p | 74% |
| miR-940 | 72% | miR-4723-3p | 73% | miR-6837-5p | 53% |
| miR-1224-5p | 67% | miR-4724-5p | 54% | miR-6838-5p | 57% |
| miR-1224-3p | 76% | miR-4725-5p | 53% | miR-6839-5p | 60% |
| miR-1225-5p | 71% | miR-4725-3p | 79% | miR-6840-5p | 74% |
| miR-1225-3p | 75% | miR-4726-5p | 65% | miR-6840-3p | 74% |
| miR-1228-5p | 63% | miR-4728-5p | 68% | miR-6841-3p | 73% |
| miR-1228-3p | 78% | miR-4728-3p | 73% | miR-6842-5p | 77% |
| miR-1231 | 65% | miR-4730 | 64% | miR-6845-5p | 71% |
| miR-1234-3p | 72% | miR-4731-5p | 66% | miR-6845-3p | 80% |
| miR-1236-3p | 73% | miR-4731-3p | 75% | miR-6846-5p | 79% |
| miR-1237-3p | 77% | miR-4732-5p | 76% | miR-6846-3p | 72% |
| miR-1238-3p | 78% | miR-4732-3p | 59% | miR-6848-5p | 72% |
| miR-320c | 64% | miR-4734 | 67% | miR-6848-3p | 72% |
| miR-1323 | 61% | miR-4736 | 57% | miR-6849-5p | 70% |
| miR-1301-3p | 61% | miR-3064-5p | 55% | miR-6850-5p | 68% |
| miR-1180-3p | 100% | miR-4738-3p | 53% | miR-6851-5p | 68% |
| miR-1182 | 68% | miR-4739 | 66% | miR-6851-3p | 76% |
| miR-1184 | 52% | miR-4741 | 69% | miR-6855-5p | 62% |
| miR-1202 | 71% | miR-4742-3p | 67% | miR-6855-3p | 73% |
| miR-1203 | 62% | miR-4743-5p | 77% | miR-6856-5p | 62% |
| miR-1207-5p | 79% | miR-4745-5p | 73% | miR-6857-5p | 67% |
| miR-1289 | 57% | miR-4746-3p | 65% | miR-6857-3p | 75% |
| miR-1290 | 100% | miR-4747-5p | 79% | miR-6858-5p | 65% |
| miR-1293 | 53% | miR-4748 | 72% | miR-6858-3p | 74% |
| miR-1294 | 63% | miR-4749-5p | 77% | miR-6859-5p | 52% |
| miR-1303 | 56% | miR-4749-3p | 77% | miR-6859-3p | 76% |
| miR-1304-5p | 64% | miR-4750-5p | 70% | miR-6769b-5p | 73% |
| miR-1246 | 100% | miR-4751 | 69% | miR-6860 | 66% |
| miR-1249-3p | 77% | miR-4752 | 54% | miR-6861-5p | 78% |
| miR-1266-5p | 51% | miR-4753-5p | 74% | miR-6861-3p | 72% |
| miR-1267 | 76% | miR-371b-5p | 75% | miR-6862-5p | 66% |
| miR-1268a | 69% | miR-371b-3p | 70% | miR-6864-5p | 73% |
| miR-1270 | 57% | miR-4754 | 55% | miR-6867-5p | 61% |
| miR-1281 | 75% | miR-4755-3p | 63% | miR-6869-5p | 67% |
| miR-1292-5p | 83% | miR-499b-3p | 56% | miR-6870-5p | 73% |
| miR-1255b-5p | 100% | miR-4758-5p | 78% | miR-6870-3p | 75% |
| miR-1307-3p | 72% | miR-4758-3p | 73% | miR-6871-5p | 63% |
| miR-320d | 86% | miR-4763-5p | 70% | miR-6874-5p | 55% |
| miR-1469 | 72% | miR-4763-3p | 76% | miR-6875-5p | 62% |
| miR-1470 | 74% | miR-4764-5p | 100% | miR-6875-3p | 61% |
| miR-1471 | 68% | miR-4766-5p | 70% | miR-6876-5p | 63% |
| miR-1908-5p | 78% | miR-4769-5p | 70% | miR-6877-5p | 79% |
| miR-1909-5p | 65% | miR-4769-3p | 71% | miR-6879-5p | 77% |
| miR-1909-3p | 70% | miR-4776-5p | 71% | miR-6879-3p | 66% |
| miR-1912-3p | 67% | miR-4778-5p | 73% | miR-6880-5p | 71% |
| miR-1913 | 75% | miR-4779 | 88% | miR-6880-3p | 75% |
| miR-1914-3p | 72% | miR-4780 | 54% | miR-6881-5p | 72% |
| miR-1915-3p | 67% | miR-4436b-5p | 69% | miR-6882-5p | 64% |
| miR-365a-5p | 78% | miR-4436b-3p | 53% | miR-6882-3p | 65% |
| miR-449b-3p | 78% | miR-4781-5p | 100% | miR-6883-5p | 62% |
| miR-1976 | 74% | miR-4783-3p | 78% | miR-6883-3p | 56% |
| miR-2110 | 78% | miR-2467-3p | 67% | miR-6884-3p | 72% |
| miR-151b | 52% | miR-4788 | 68% | miR-6885-3p | 74% |
| miR-762 | 70% | miR-4793-3p | 58% | miR-6886-5p | 63% |
| miR-670-5p | 60% | miR-4795-3p | 100% | miR-6886-3p | 69% |
| miR-2114-3p | 100% | miR-4800-5p | 77% | miR-6887-5p | 53% |
| miR-2116-3p | 76% | miR-642a-3p | 67% | miR-6887-3p | 76% |
| miR-548q | 71% | miR-4433a-5p | 77% | miR-6888-3p | 100% |
| miR-2276-3p | 66% | miR-4482-3p | 72% | miR-6889-5p | 77% |
| miR-711 | 75% | miR-4999-5p | 60% | miR-6889-3p | 74% |
| miR-718 | 77% | miR-5000-3p | 57% | miR-6890-3p | 71% |
| miR-2681-3p | 68% | miR-5001-5p | 71% | miR-6891-5p | 75% |
| miR-3120-3p | 67% | miR-5002-3p | 56% | miR-6891-3p | 75% |
| miR-3122 | 53% | miR-5003-3p | 70% | miR-6892-5p | 70% |
| miR-3126-5p | 59% | miR-5006-5p | 60% | miR-6892-3p | 73% |
| miR-3130-5p | 64% | miR-5009-3p | 55% | miR-6893-5p | 78% |
| miR-3131 | 73% | miR-5010-5p | 70% | miR-6893-3p | 63% |
| miR-3132 | 60% | miR-5088-5p | 64% | miR-6894-5p | 76% |
| miR-3133 | 58% | miR-5090 | 66% | miR-7106-5p | 74% |
| miR-3134 | 100% | miR-5188 | 55% | miR-7107-5p | 76% |
| miR-466 | 52% | miR-5189-5p | 59% | miR-7108-5p | 74% |
| miR-3136-5p | 100% | miR-5195-5p | 71% | miR-7108-3p | 77% |
| miR-3137 | 54% | miR-5195-3p | 66% | miR-7109-5p | 76% |
| miR-3138 | 60% | miR-5196-5p | 77% | miR-7109-3p | 68% |
| miR-3141 | 72% | miR-5100 | 55% | miR-7110-5p | 74% |
| miR-1273c | 75% | miR-5572 | 81% | miR-7111-5p | 80% |
| miR-3147 | 76% | miR-664b-5p | 56% | miR-7111-3p | 70% |
| miR-3150a-3p | 81% | miR-5584-5p | 73% | miR-7112-5p | 60% |
| miR-3151-5p | 81% | miR-5588-5p | 54% | miR-7113-3p | 72% |
| miR-3153 | 77% | miR-5589-5p | 64% | miR-7114-5p | 74% |
| miR-3154 | 73% | miR-5698 | 75% | miR-7114-3p | 80% |
| miR-3156-5p | 76% | miR-5703 | 57% | miR-7150 | 75% |
| miR-3158-3p | 51% | miR-5705 | 58% | miR-7151-3p | 58% |
| miR-3161 | 55% | miR-5707 | 75% | miR-7152-5p | 52% |
| miR-3162-5p | 77% | miR-197-5p | 76% | miR-7152-3p | 64% |
| miR-3163 | 85% | miR-204-3p | 80% | miR-7155-5p | 72% |
| miR-3165 | 63% | miR-211-3p | 73% | miR-7158-3p | 67% |
| miR-3168 | 63% | miR-345-3p | 56% | miR-7159-5p | 67% |
| miR-3173-3p | 59% | miR-652-5p | 71% | miR-7160-5p | 67% |
| miR-1193 | 72% | miR-1185-2-3p | 76% | miR-7162-5p | 53% |
| miR-3175 | 68% | miR-873-3p | 56% | miR-7515 | 70% |
| miR-3176 | 52% | miR-1304-3p | 74% | miR-7843-5p | 60% |
| miR-3177-3p | 71% | miR-1247-3p | 77% | miR-4433b-5p | 78% |
| miR-3178 | 67% | miR-3184-3p | 71% | miR-4433b-3p | 77% |
| miR-3179 | 71% | miR-642b-5p | 53% | miR-1273h-5p | 70% |
| miR-3180-5p | 72% | miR-365b-5p | 60% | miR-6516-5p | 55% |
| miR-3180-3p | 65% | miR-1185-1-3p | 79% | miR-7845-5p | 71% |
| miR-3184-5p | 63% | miR-3190-3p | 54% | miR-7846-3p | 73% |
| miR-3185 | 74% | miR-381-5p | 57% | miR-7847-3p | 66% |
| miR-3186-3p | 55% | miR-937-5p | 83% | miR-7851-3p | 60% |
| miR-3187-3p | 52% | miR-1227-5p | 76% | miR-7855-5p | 64% |
| miR-3189-3p | 69% | miR-1229-5p | 65% | miR-7856-5p | 52% |
| miR-3191-3p | 80% | miR-1233-5p | 68% | miR-8052 | 72% |
| miR-3195 | 64% | miR-1236-5p | 63% | miR-8054 | 75% |
| miR-3196 | 63% | miR-1237-5p | 67% | miR-8056 | 100% |
| miR-3197 | 75% | miR-1238-5p | 78% | miR-8059 | 76% |
| miR-3198 | 61% | miR-3620-5p | 77% | miR-8060 | 73% |
| miR-514b-5p | 68% | miR-3934-3p | 55% | miR-8063 | 68% |
| miR-3202 | 71% | miR-4632-5p | 66% | miR-8064 | 63% |
| miR-4297 | 60% | miR-4743-3p | 64% | miR-8069 | 65% |
| miR-4294 | 74% | miR-4750-3p | 76% | miR-8071 | 78% |
| miR-4299 | 55% | miR-5739 | 74% | miR-8072 | 67% |
| miR-4298 | 73% | miR-5787 | 63% | miR-8073 | 74% |
| miR-4305 | 63% | miR-6069 | 74% | miR-8074 | 55% |
| miR-4307 | 70% | miR-6071 | 65% | miR-8075 | 56% |
| miR-4313 | 74% | miR-6072 | 59% | miR-8077 | 64% |
| miR-4316 | 67% | miR-6075 | 68% | miR-8080 | 66% |
| miR-4314 | 58% | miR-6076 | 77% | miR-8085 | 80% |
| miR-4322 | 65% | miR-6077 | 59% | miR-8087 | 77% |
| miR-4321 | 51% | miR-6085 | 72% | miR-8089 | 77% |
| miR-4323 | 77% | miR-6086 | 80% | miR-7977 | 64% |
| miR-4257 | 81% | miR-6088 | 68% | miR-7978 | 100% |
| miR-4258 | 74% | miR-6089 | 65% | miR-1249-5p | 69% |
| miR-4259 | 72% | miR-6090 | 64% | miR-4485-5p | 66% |
| miR-4260 | 73% | miR-6124 | 78% | miR-8485 | 70% |
| miR-4253 | 59% | miR-6125 | 72% | miR-498-3p | 63% |
| miR-4251 | 54% | miR-6126 | 61% | miR-526a-3p | 100% |
| miR-4326 | 61% | miR-6127 | 75% | miR-9718 | 66% |
| miR-4327 | 78% | miR-378j | 61% | miR-9898 | 72% |
| miR-4268 | 60% | miR-6131 | 74% | miR-1843 | 55% |
| miR-4269 | 70% | miR-6132 | 70% | miR-10392-5p | 75% |
| miR-4264 | 53% | miR-6133 | 77% | miR-10392-3p | 67% |
| miR-4271 | 70% | miR-6165 | 76% | miR-10394-3p | 54% |
| miR-4276 | 60% | miR-6501-3p | 54% | miR-10396a-5p | 63% |
| miR-4274 | 76% | miR-6506-5p | 56% | miR-10396a-3p | 69% |
| miR-4280 | 58% | miR-6506-3p | 86% | miR-10398-5p | 65% |
| miR-4284 | 72% | miR-6510-5p | 71% | miR-10398-3p | 80% |
| miR-4286 | 76% | miR-6511a-5p | 82% | miR-10399-3p | 100% |
| miR-500b-5p | 65% | miR-6512-3p | 58% | miR-10400-3p | 75% |
| miR-3200-5p | 59% | miR-6513-5p | 58% | miR-10401-5p | 78% |
| miR-3609 | 100% | miR-6514-3p | 54% | miR-10401-3p | 73% |
| miR-3610 | 73% | miR-6515-5p | 59% | miR-10396b-5p | 68% |
| miR-3614-5p | 74% | miR-6515-3p | 79% | miR-10522-5p | 78% |
| miR-3616-3p | 51% | miR-6716-5p | 70% | miR-10526-3p | 71% |
| miR-3617-5p | 100% | miR-6716-3p | 60% | miR-10527-5p | 78% |
| miR-3621 | 68% | miR-6717-5p | 64% | miR-11181-3p | 73% |
| miR-3622a-5p | 63% | miR-6511b-5p | 61% | miR-11399 | 71% |
| miR-3622a-3p | 60% | miR-6511b-3p | 75% | miR-3059-5p | 73% |
| miR-3622b-5p | 72% | miR-6718-5p | 52% | miR-3059-3p | 52% |
| miR-3646 | 58% | miR-6719-3p | 58% | miR-3085-5p | 69% |
| miR-3648 | 79% | miR-6721-5p | 69% | miR-3085-3p | 75% |
| miR-3649 | 65% | miR-6722-5p | 64% | miR-6529-5p | 58% |
| miR-3652 | 73% | miR-6722-3p | 70% | miR-12114 | 61% |
| miR-3660 | 59% | miR-6724-5p | 73% | miR-12115 | 70% |
| miR-3663-5p | 71% | miR-208a-5p | 53% | miR-12116 | 77% |
| miR-3665 | 61% | miR-210-5p | 73% | miR-12118 | 71% |
| miR-3667-3p | 66% | miR-128-1-5p | 66% | miR-12119 | 79% |
| miR-3675-3p | 76% | miR-328-5p | 66% | miR-12120 | 76% |
| miR-3679-5p | 76% | miR-329-5p | 55% | miR-12121 | 68% |
| miR-3679-3p | 76% | miR-410-5p | 54% | miR-12124 | 53% |
| miR-3681-5p | 100% | miR-181d-3p | 86% | miR-12126 | 59% |
| miR-3689a-3p | 53% | miR-504-3p | 72% | miR-12131 | 100% |
| miR-3714 | 70% | miR-487b-5p | 60% | miR-12136 | 63% |
| miR-3180 | 61% | miR-585-5p | 58% | | |

### [Table 22-8]

**Table 22-8: miRNA to Predict Human Melanoma of Stage I**

| miRNA Name | A | miRNA Name | A | miRNA Name | A |
|---|---|---|---|---|---|
| miR-25-3p | 60% | miR-4433a-3p | 76% | miR-6734-5p | 79% |
| miR-197-3p | 79% | miR-4441 | 80% | miR-6738-5p | 73% |
| miR-198 | 82% | miR-4444 | 84% | miR-6742-5p | 96% |
| miR-224-5p | 100% | miR-4447 | 73% | miR-6743-5p | 66% |
| miR-124-3p | 60% | miR-4449 | 65% | miR-6745 | 76% |
| miR-184 | 54% | miR-548ah-5p | 100% | miR-6746-5p | 88% |
| miR-296-5p | 74% | miR-4463 | 71% | miR-6748-5p | 79% |
| miR-365a-3p, miR-365b-3p | 74% | miR-4466 | 73% | miR-6749-5p | 80% |
| miR-302c-5p | 87% | miR-4472 | 81% | miR-6752-5p | 71% |
| miR-373-5p | 78% | miR-3689d | 64% | miR-6752-3p | 76% |
| miR-380-3p | 79% | miR-3155b | 71% | miR-6756-5p | 68% |
| miR-383-5p | 67% | miR-4480 | 100% | miR-6756-3p | 76% |
| miR-328-3p | 79% | miR-4484 | 69% | miR-6758-5p | 88% |
| miR-449a | 60% | miR-4488 | 69% | miR-6759-3p | 74% |
| miR-450a-5p | 57% | miR-4492 | 78% | miR-6760-5p | 86% |
| miR-483-3p | 87% | miR-4495 | 100% | miR-6760-3p | 80% |
| miR-484 | 75% | miR-4496 | 89% | miR-6763-3p | 79% |
| miR-485-5p | 63% | miR-4497 | 66% | miR-6765-5p | 68% |
| miR-498-5p | 92% | miR-4508 | 69% | miR-6766-5p | 62% |
| miR-520e-3p | 87% | miR-4512 | 73% | miR-6766-3p | 79% |
| miR-519b-3p | 55% | miR-4516 | 73% | miR-6768-5p | 69% |
| miR-520b-3p | 86% | miR-4519 | 67% | miR-6769a-5p | 76% |
| miR-513a-5p | 90% | miR-4521 | 56% | miR-6771-5p | 68% |
| miR-557 | 70% | miR-4525 | 88% | miR-6774-5p | 78% |
| miR-571 | 67% | miR-4530 | 58% | miR-6775-5p | 77% |
| miR-583 | 79% | miR-4531 | 82% | miR-6777-5p | 70% |
| miR-611 | 58% | miR-4534 | 81% | miR-6777-3p | 78% |
| miR-625-5p | 88% | miR-3127-3p | 81% | miR-6779-5p | 70% |
| miR-638 | 71% | miR-3158-5p | 72% | miR-6780a-5p | 73% |
| miR-652-3p | 100% | miR-3162-3p | 80% | miR-6781-5p | 66% |
| miR-663a | 77% | miR-3173-5p | 63% | miR-6782-5p | 67% |
| miR-658 | 61% | miR-3619-3p | 74% | miR-6784-3p | 82% |
| miR-542-5p | 75% | miR-3691-3p | 57% | miR-6785-3p | 78% |
| miR-671-5p | 74% | miR-3940-5p | 65% | miR-6786-5p | 66% |
| miR-675-5p | 71% | miR-3978 | 92% | miR-6787-5p | 69% |
| miR-22-5p | 100% | miR-4634 | 70% | miR-6789-5p | 72% |
| miR-92a-2-5p | 57% | miR-4637 | 100% | miR-6790-5p | 52% |
| miR-29b-1-5p | 73% | miR-4648 | 54% | miR-6791-5p | 67% |
| miR-30c-2-3p | 89% | miR-4649-5p | 72% | miR-6792-3p | 79% |
| miR-181a-2-3p | 67% | miR-4651 | 66% | miR-6793-3p | 81% |
| miR-187-5p | 58% | miR-4661-5p | 57% | miR-6794-5p | 88% |
| miR-30b-3p | 53% | miR-4665-5p | 84% | miR-6795-5p | 81% |
| miR-125b-1-3p | 100% | miR-4665-3p | 75% | miR-6795-3p | 78% |
| miR-135a-3p | 51% | miR-4668-5p | 100% | miR-6796-3p | 78% |
| miR-145-3p | 67% | miR-4674 | 72% | miR-6797-5p | 79% |
| miR-127-5p | 71% | miR-4675 | 63% | miR-6797-3p | 70% |
| miR-149-3p | 72% | miR-4685-5p | 76% | miR-6798-5p | 71% |
| miR-150-3p | 83% | miR-4687-5p | 77% | miR-6798-3p | 79% |
| miR-186-3p | 56% | miR-4687-3p | 71% | miR-6799-5p | 71% |
| miR-361-3p | 73% | miR-1343-3p | 73% | miR-6800-5p | 66% |
| miR-371a-5p | 69% | miR-4689 | 68% | miR-6800-3p | 78% |
| miR-342-5p | 82% | miR-4691-5p | 65% | miR-6801-3p | 77% |
| miR-18b-3p | 71% | miR-4691-3p | 83% | miR-6802-5p | 68% |
| miR-490-5p | 100% | miR-4697-5p | 74% | miR-6802-3p | 70% |
| miR-92b-5p | 63% | miR-4706 | 80% | miR-6803-3p | 82% |
| miR-625-3p | 78% | miR-4707-5p | 67% | miR-6805-5p | 68% |
| miR-708-5p | 57% | miR-4707-3p | 78% | miR-6805-3p | 78% |
| miR-744-3p | 52% | miR-4715-5p | 71% | miR-6807-5p | 72% |
| miR-877-5p | 56% | miR-4716-5p | 80% | miR-6808-5p | 68% |
| miR-877-3p | 80% | miR-4716-3p | 93% | miR-6808-3p | 67% |
| miR-665 | 69% | miR-4723-5p | 81% | miR-6809-5p | 70% |
| miR-940 | 76% | miR-4723-3p | 76% | miR-6809-3p | 82% |
| miR-943 | 57% | miR-451b | 73% | miR-6810-3p | 79% |
| miR-1224-3p | 78% | miR-4725-3p | 78% | miR-6812-5p | 80% |
| miR-1225-5p | 76% | miR-4726-3p | 75% | miR-6812-3p | 80% |
| miR-1225-3p | 78% | miR-4728-5p | 76% | miR-6813-3p | 76% |
| miR-1228-5p | 65% | miR-4728-3p | 73% | miR-6815-5p | 80% |
| miR-1228-3p | 73% | miR-4731-5p | 71% | miR-6816-5p | 68% |
| miR-1234-3p | 77% | miR-4731-3p | 73% | miR-6819-3p | 78% |
| miR-1237-3p | 78% | miR-4738-5p | 83% | miR-6821-5p | 67% |
| miR-1238-3p | 77% | miR-4741 | 78% | miR-6821-3p | 73% |
| miR-1182 | 62% | miR-4745-5p | 66% | miR-6824-5p | 73% |
| miR-1202 | 75% | miR-4747-5p | 79% | miR-6825-5p | 68% |
| miR-663b | 74% | miR-4748 | 71% | miR-6829-5p | 78% |
| miR-1207-5p | 81% | miR-4749-5p | 63% | miR-6830-5p | 88% |
| miR-1208 | 55% | miR-4749-3p | 79% | miR-6831-5p | 70% |
| miR-1299 | 100% | miR-371b-5p | 60% | miR-6832-5p | 59% |
| miR-1249-3p | 78% | miR-371b-3p | 71% | miR-6833-5p | 62% |
| miR-1253 | 75% | miR-4755-3p | 58% | miR-6835-5p | 57% |
| miR-1267 | 81% | miR-4758-5p | 76% | miR-6780b-5p | 72% |
| miR-1268a | 64% | miR-4758-3p | 72% | miR-6836-3p | 73% |
| miR-1281 | 81% | miR-4761-3p | 69% | miR-6839-3p | 53% |
| miR-1292-5p | 83% | miR-4763-3p | 61% | miR-6840-5p | 77% |
| miR-1252-5p | 100% | miR-4769-3p | 79% | miR-6840-3p | 67% |
| miR-1825 | 83% | miR-4778-5p | 63% | miR-6845-3p | 81% |
| miR-1469 | 68% | miR-4781-5p | 71% | miR-6848-5p | 71% |
| miR-1908-5p | 71% | miR-4783-3p | 75% | miR-6848-3p | 75% |
| miR-1909-3p | 66% | miR-4787-5p | 73% | miR-6850-5p | 71% |
| miR-1911-5p | 100% | miR-4802-3p | 75% | miR-6855-3p | 76% |
| miR-1912-3p | 59% | miR-642a-3p | 70% | miR-6857-3p | 77% |
| miR-1913 | 76% | miR-4433a-5p | 75% | miR-6858-3p | 75% |
| miR-1914-3p | 92% | miR-4536-3p | 100% | miR-6859-3p | 75% |
| miR-1915-3p | 66% | miR-5006-5p | 72% | miR-6769b-5p | 73% |
| miR-365a-5p | 76% | miR-5010-5p | 60% | miR-6861-3p | 76% |
| miR-196b-3p | 54% | miR-5190 | 56% | miR-6862-3p | 81% |
| miR-1976 | 80% | miR-5195-3p | 76% | miR-6865-5p | 61% |
| miR-2053 | 67% | miR-5196-5p | 88% | miR-6870-5p | 71% |
| miR-762 | 69% | miR-548as-5p | 100% | miR-6870-3p | 75% |
| miR-2116-3p | 78% | miR-548as-3p | 100% | miR-6872-5p | 74% |
| miR-711 | 90% | miR-5579-5p | 100% | miR-6880-5p | 73% |
| miR-718 | 79% | miR-5579-3p | 100% | miR-6880-3p | 81% |
| miR-2681-3p | 57% | miR-5584-5p | 72% | miR-6881-5p | 77% |
| miR-3122 | 66% | miR-5698 | 71% | miR-6884-3p | 75% |
| miR-3131 | 84% | miR-211-3p | 59% | miR-6885-3p | 75% |
| miR-3133 | 52% | miR-345-3p | 75% | miR-6886-3p | 78% |
| miR-3141 | 69% | miR-652-5p | 72% | miR-6887-5p | 78% |
| miR-3144-5p | 84% | miR-1185-2-3p | 83% | miR-6887-3p | 73% |
| miR-3147 | 71% | miR-1247-3p | 74% | miR-6889-5p | 74% |
| miR-3074-3p | 55% | miR-3184-3p | 73% | miR-6889-3p | 76% |
| miR-3155a | 61% | miR-1185-1-3p | 86% | miR-6891-5p | 77% |
| miR-3162-5p | 68% | miR-503-3p | 82% | miR-6891-3p | 74% |
| miR-3178 | 72% | miR-937-5p | 65% | miR-6892-3p | 78% |
| miR-3180-3p | 63% | miR-1227-5p | 70% | miR-6893-5p | 66% |
| miR-3196 | 73% | miR-1229-5p | 73% | miR-6895-5p | 64% |
| miR-3197 | 68% | miR-1237-5p | 71% | miR-7106-5p | 79% |
| miR-3200-3p | 52% | miR-1238-5p | 80% | miR-7107-5p | 80% |
| miR-514b-5p | 76% | miR-4750-3p | 79% | miR-7108-5p | 69% |
| miR-3202 | 78% | miR-5739 | 74% | miR-7108-3p | 82% |
| miR-4293 | 65% | miR-5787 | 63% | miR-7109-5p | 78% |
| miR-4294 | 76% | miR-6069 | 76% | miR-7110-3p | 78% |
| miR-4299 | 51% | miR-6071 | 56% | miR-7111-5p | 78% |
| miR-4298 | 73% | miR-6081 | 56% | miR-7111-3p | 77% |
| miR-4306 | 88% | miR-6085 | 81% | miR-7113-3p | 78% |
| miR-4313 | 77% | miR-6086 | 86% | miR-7114-5p | 63% |
| miR-4316 | 69% | miR-6088 | 71% | miR-7114-3p | 81% |
| miR-4323 | 77% | miR-6089 | 69% | miR-7150 | 73% |
| miR-4257 | 76% | miR-6090 | 65% | miR-7161-5p | 54% |
| miR-4258 | 73% | miR-6124 | 82% | miR-7704 | 72% |
| miR-4327 | 75% | miR-6125 | 70% | miR-4433b-5p | 80% |
| miR-4265 | 58% | miR-6133 | 67% | miR-4433b-3p | 89% |
| miR-2355-5p | 79% | miR-6165 | 73% | miR-7845-5p | 70% |
| miR-4271 | 81% | miR-6501-5p | 54% | miR-7851-3p | 52% |
| miR-4274 | 78% | miR-6503-3p | 62% | miR-7852-3p | 100% |
| miR-4281 | 81% | miR-6511a-5p | 83% | miR-8059 | 73% |
| miR-4279 | 85% | miR-6512-5p | 100% | miR-8064 | 51% |
| miR-4284 | 78% | miR-6515-3p | 78% | miR-8069 | 73% |
| miR-4286 | 81% | miR-6716-5p | 82% | miR-8072 | 68% |
| miR-4290 | 86% | miR-6511b-5p | 88% | miR-8078 | 61% |
| miR-3200-5p | 74% | miR-6719-3p | 67% | miR-8079 | 59% |
| miR-3610 | 71% | miR-6720-3p | 67% | miR-128-2-5p | 73% |
| miR-3612 | 87% | miR-6721-5p | 67% | miR-7974 | 55% |
| miR-3614-5p | 75% | miR-6724-5p | 65% | miR-1249-5p | 69% |
| miR-3622a-5p | 51% | miR-208a-5p | 61% | miR-137-5p | 100% |
| miR-3648 | 69% | miR-210-5p | 74% | miR-190b-3p | 100% |
| miR-3663-3p | 75% | miR-128-1-5p | 68% | miR-9898 | 77% |
| miR-3665 | 69% | miR-152-5p | 75% | miR-9902 | 54% |
| miR-3675-3p | 78% | miR-328-5p | 70% | miR-10392-3p | 76% |
| miR-3679-5p | 74% | miR-489-5p | 60% | miR-10394-3p | 70% |
| miR-3679-3p | 77% | miR-511-3p | 100% | miR-10396a-5p | 68% |
| miR-3713 | 54% | miR-181d-3p | 79% | miR-10396a-3p | 75% |
| miR-3180 | 68% | miR-598-5p | 55% | miR-10400-3p | 61% |
| miR-3907 | 78% | miR-605-3p | 53% | miR-10401-5p | 63% |
| miR-3922-3p | 56% | miR-619-5p | 51% | miR-10396b-5p | 68% |
| miR-3924 | 100% | miR-627-3p | 100% | miR-10522-5p | 85% |
| miR-3926 | 54% | miR-874-5p | 71% | miR-10526-3p | 94% |
| miR-3936 | 54% | miR-208b-5p | 100% | miR-11181-3p | 66% |
| miR-3937 | 72% | miR-1343-5p | 66% | miR-3059-3p | 62% |
| miR-3943 | 77% | miR-6727-5p | 75% | miR-3085-3p | 72% |
| miR-3945 | 59% | miR-6729-5p | 71% | miR-12116 | 75% |
| miR-642b-3p | 70% | miR-6729-3p | 75% | miR-12118 | 67% |
| miR-1268b | 61% | miR-6730-5p | 74% | miR-12119 | 80% |
| miR-548ab | 60% | miR-6731-3p | 76% | miR-12120 | 63% |
| miR-4418 | 67% | miR-6732-5p | 65% | miR-12128 | 72% |
| | | | | miR-12131 | 100% |

### [Table 22-9]

**Table 22-9: miRNA to Predict Human Ovarian Cancer of Stage I**

| miRNA Name | A | miRNA Name | A | miRNA Name | A |
|---|---|---|---|---|---|
| hsa-miR-93-5p | 68% | hsa-miR-548v | 100% | hsa-miR-5190 | 77% |
| hsa-miR-210-3p | 74% | hsa-miR-3074-3p | 71% | hsa-miR-5196-5p | 72% |
| hsa-miR-221-3p | 67% | hsa-miR-3155a | 73% | hsa-miR-5579-5p | 100% |
| hsa-miR-27b-3p | 64% | hsa-miR-3170 | 75% | hsa-miR-5579-3p | 100% |
| hsa-miR-143-3p | 57% | hsa-miR-3186-5p | 51% | hsa-miR-5580-5p | 60% |
| hsa-miR-34b-5p | 51% | hsa-miR-3187-3p | 67% | hsa-miR-5681a | 69% |
| hsa-miR-130b-3p | 67% | hsa-miR-4296 | 54% | hsa-miR-5693 | 59% |
| hsa-miR-371a-3p | 59% | hsa-miR-4293 | 57% | hsa-miR-5694 | 59% |
| hsa-miR-380-3p | 70% | hsa-miR-4299 | 66% | hsa-miR-5702 | 60% |
| hsa-miR-382-5p | 62% | hsa-miR-4321 | 51% | hsa-miR-1285-5p | 57% |
| hsa-miR-383-5p | 63% | hsa-miR-4280 | 56% | hsa-miR-216a-3p | 61% |
| hsa-miR-330-3p | 66% | hsa-miR-4330 | 59% | hsa-miR-4743-3p | 57% |
| hsa-miR-369-5p | 57% | hsa-miR-3619-5p | 77% | hsa-miR-6068 | 67% |
| hsa-miR-431-5p | 60% | hsa-miR-3651 | 55% | hsa-miR-6071 | 62% |
| hsa-miR-452-3p | 55% | hsa-miR-3659 | 55% | hsa-miR-6081 | 69% |
| hsa-miR-485-5p | 77% | hsa-miR-3661 | 51% | hsa-miR-6083 | 55% |
| hsa-miR-519b-3p | 57% | hsa-miR-3666 | 63% | hsa-miR-6499-5p | 64% |
| hsa-miR-525-3p | 69% | hsa-miR-3677-3p | 60% | hsa-miR-6500-5p | 65% |
| hsa-miR-519a-3p | 58% | hsa-miR-3688-3p | 67% | hsa-miR-6501-5p | 74% |
| hsa-miR-501-5p | 74% | hsa-miR-3690 | 55% | hsa-miR-6501-3p | 53% |
| hsa-miR-493-3p | 52% | hsa-miR-3692-3p | 58% | hsa-miR-6502-5p | 56% |
| hsa-miR-539-5p | 64% | hsa-miR-3907 | 82% | hsa-miR-6503-3p | 66% |
| hsa-miR-571 | 89% | hsa-miR-3908 | 60% | hsa-miR-6504-5p | 54% |
| hsa-miR-589-3p | 63% | hsa-miR-3923 | 100% | hsa-miR-6505-3p | 59% |
| hsa-miR-591 | 66% | hsa-miR-3929 | 62% | hsa-miR-6512-5p | 100% |
| hsa-miR-593-5p | 64% | hsa-miR-3934-5p | 75% | hsa-miR-6513-3p | 58% |
| hsa-miR-609 | 53% | hsa-miR-4433a-3p | 72% | hsa-miR-6715a-3p | 62% |
| hsa-miR-611 | 67% | hsa-miR-4435 | 60% | hsa-miR-6716-5p | 72% |
| hsa-miR-613 | 55% | hsa-miR-4439 | 58% | hsa-miR-6719-3p | 56% |
| hsa-miR-623 | 67% | hsa-miR-548ah-5p | 100% | hsa-miR-433-5p | 61% |
| hsa-miR-631 | 64% | hsa-miR-4460 | 60% | hsa-miR-489-5p | 69% |
| hsa-miR-639 | 67% | hsa-miR-4465 | 59% | hsa-miR-181d-3p | 67% |
| hsa-miR-650 | 67% | hsa-miR-548al | 100% | hsa-miR-598-5p | 61% |
| hsa-miR-421 | 59% | hsa-miR-4494 | 71% | hsa-miR-627-3p | 100% |
| hsa-miR-542-5p | 80% | hsa-miR-4502 | 58% | hsa-miR-668-5p | 69% |
| hsa-miR-363-5p | 61% | hsa-miR-4512 | 83% | hsa-miR-1251-3p | 61% |
| hsa-miR-769-3p | 60% | hsa-miR-4521 | 65% | hsa-miR-6761-5p | 69% |
| hsa-miR-15a-3p | 59% | hsa-miR-4522 | 57% | hsa-miR-6764-5p | 55% |
| hsa-miR-22-5p | 100% | hsa-miR-4529-3p | 65% | hsa-miR-6768-3p | 62% |
| hsa-miR-25-5p | 69% | hsa-miR-378i | 60% | hsa-miR-6775-5p | 71% |
| hsa-miR-29b-1-5p | 57% | hsa-miR-4537 | 60% | hsa-miR-6791-3p | 66% |
| hsa-miR-181c-3p | 67% | hsa-miR-3129-3p | 64% | hsa-miR-6794-5p | 63% |
| hsa-miR-30b-3p | 73% | hsa-miR-3157-3p | 55% | hsa-miR-6795-5p | 62% |
| hsa-miR-124-5p | 57% | hsa-miR-3944-5p | 60% | hsa-miR-6809-5p | 65% |
| hsa-miR-125b-1-3p | 100% | hsa-miR-3978 | 93% | hsa-miR-6829-5p | 75% |
| hsa-miR-135a-3p | 64% | hsa-miR-4634 | 71% | hsa-miR-6837-3p | 86% |
| hsa-miR-127-5p | 71% | hsa-miR-4637 | 100% | hsa-miR-6838-5p | 56% |
| hsa-miR-186-3p | 63% | hsa-miR-4648 | 70% | hsa-miR-6839-3p | 53% |
| hsa-miR-339-3p | 59% | hsa-miR-4653-5p | 52% | hsa-miR-6845-3p | 74% |
| hsa-miR-509-5p | 60% | hsa-miR-4658 | 66% | hsa-miR-6856-3p | 61% |
| hsa-miR-593-3p | 64% | hsa-miR-4673 | 66% | hsa-miR-6888-5p | 61% |
| hsa-miR-654-3p | 74% | hsa-miR-4676-3p | 55% | hsa-miR-6895-5p | 80% |
| hsa-miR-892a | 68% | hsa-miR-4684-3p | 70% | hsa-miR-7112-3p | 64% |
| hsa-miR-665 | 77% | hsa-miR-4691-3p | 92% | hsa-miR-7151-5p | 60% |
| hsa-miR-942-5p | 53% | hsa-miR-4713-3p | 54% | hsa-miR-7156-3p | 52% |
| hsa-miR-1225-5p | 70% | hsa-miR-4715-5p | 69% | hsa-miR-7158-5p | 58% |
| hsa-miR-1226-5p | 63% | hsa-miR-4733-5p | 57% | hsa-miR-7158-3p | 57% |
| hsa-miR-320c | 57% | hsa-miR-4740-5p | 65% | hsa-miR-7161-5p | 54% |
| hsa-miR-1286 | 61% | hsa-miR-4761-5p | 55% | hsa-miR-7706 | 65% |
| hsa-miR-1303 | 64% | hsa-miR-4761-3p | 66% | hsa-miR-4433b-3p | 74% |
| hsa-miR-1250-5p | 52% | hsa-miR-4768-3p | 56% | hsa-miR-7850-5p | 55% |
| hsa-miR-1253 | 63% | hsa-miR-4772-3p | 67% | hsa-miR-7851-3p | 63% |
| hsa-miR-1255a | 100% | hsa-miR-4778-5p | 54% | hsa-miR-8064 | 65% |
| hsa-miR-1269a | 76% | hsa-miR-4781-5p | 60% | hsa-miR-8078 | 80% |
| hsa-miR-1292-5p | 82% | hsa-miR-4786-3p | 51% | hsa-miR-8079 | 72% |
| hsa-miR-1912-3p | 71% | hsa-miR-4793-5p | 72% | hsa-miR-8080 | 66% |
| hsa-miR-1914-3p | 73% | hsa-miR-4794 | 52% | hsa-miR-7974 | 72% |
| hsa-miR-103a-2-5p | 61% | hsa-miR-4797-5p | 100% | hsa-miR-137-5p | 100% |
| hsa-miR-196b-3p | 65% | hsa-miR-4802-3p | 63% | hsa-miR-9986 | 54% |
| hsa-miR-1972 | 79% | hsa-miR-550a-3-5p | 52% | hsa-miR-10394-5p | 57% |
| hsa-miR-449c-5p | 75% | hsa-miR-5000-5p | 53% | hsa-miR-10396a-3p | 80% |
| hsa-miR-761 | 57% | hsa-miR-5002-5p | 69% | hsa-miR-10522-5p | 80% |
| hsa-miR-2681-5p | 58% | hsa-miR-5003-3p | 60% | hsa-miR-10526-3p | 63% |
| hsa-miR-2681-3p | 57% | hsa-miR-5004-3p | 55% | hsa-miR-3059-3p | 60% |
| hsa-miR-3130-3p | 62% | hsa-miR-5006-5p | 72% | hsa-miR-12122 | 51% |
| hsa-miR-3144-3p | 65% | hsa-miR-5091 | 61% | hsa-miR-12131 | 100% |

### [Table 22-10]

**Table 22-10: miRNA to Predict Human Thyroid Cancer of Stage I**

| miRNA Name | A | miRNA Name | A | miRNA Name | A |
|---|---|---|---|---|---|
| miR-17-3p | 100% | miR-3936 | 61% | miR-6731-3p | 77% |
| miR-25-3p | 75% | miR-3937 | 79% | miR-6732-5p | 80% |
| miR-197-3p | 88% | miR-3943 | 76% | miR-6732-3p | 78% |
| miR-198 | 86% | miR-3944-3p | 61% | miR-6734-5p | 79% |
| miR-139-5p | 100% | miR-1268b | 78% | miR-6736-3p | 70% |
| miR-124-3p | 60% | miR-4429 | 56% | miR-6737-5p | 73% |
| miR-185-5p | 80% | miR-4431 | 54% | miR-6738-5p | 80% |
| miR-296-5p | 71% | miR-4432 | 100% | miR-6738-3p | 53% |
| miR-365a-3p, miR-365b-3p | 75% | miR-4433a-3p | 84% | miR-6740-3p | 77% |
| miR-302c-5p | 84% | miR-4441 | 82% | miR-6741-3p | 79% |
| miR-370-3p | 77% | miR-4444 | 83% | miR-6742-5p | 93% |
| miR-373-5p | 76% | miR-4446-3p | 72% | miR-6742-3p | 78% |
| miR-383-5p | 67% | miR-4447 | 71% | miR-6743-5p | 84% |
| miR-330-3p | 60% | miR-4449 | 80% | miR-6743-3p | 81% |
| miR-328-3p | 80% | miR-3135b | 59% | miR-6745 | 69% |
| miR-324-5p | 58% | miR-4463 | 70% | miR-6746-5p | 91% |
| miR-324-3p | 75% | miR-4466 | 81% | miR-6746-3p | 83% |
| miR-346 | 74% | miR-4467 | 77% | miR-6748-5p | 81% |
| miR-422a | 55% | miR-4470 | 67% | miR-6749-5p | 81% |
| miR-483-3p | 89% | miR-4472 | 89% | miR-6749-3p | 78% |
| miR-484 | 72% | miR-4473 | 63% | miR-6750-5p | 54% |
| miR-485-5p | 55% | miR-4477b | 100% | miR-6751-5p | 58% |
| miR-491-5p | 78% | miR-3689d | 63% | miR-6752-5p | 86% |
| miR-498-5p | 94% | miR-4479 | 56% | miR-6752-3p | 75% |
| miR-520e-3p | 90% | miR-3155b | 78% | miR-6753-5p | 69% |
| miR-519b-3p | 57% | miR-4480 | 100% | miR-6754-3p | 67% |
| miR-525-3p | 55% | miR-4483 | 70% | miR-6756-5p | 69% |
| miR-518f-3p | 73% | miR-4484 | 78% | miR-6756-3p | 77% |
| miR-520b-3p | 89% | miR-4486 | 74% | miR-6758-5p | 81% |
| miR-513a-5p | 89% | miR-4488 | 74% | miR-6759-5p | 69% |
| miR-510-5p | 53% | miR-4489 | 56% | miR-6759-3p | 75% |
| miR-557 | 68% | miR-4492 | 75% | miR-6760-5p | 83% |
| miR-572 | 57% | miR-4494 | 60% | miR-6760-3p | 81% |
| miR-575 | 60% | miR-4495 | 100% | miR-6761-5p | 55% |
| miR-583 | 75% | miR-4496 | 83% | miR-6761-3p | 79% |
| miR-550a-3p | 70% | miR-4497 | 81% | miR-6762-5p | 67% |
| miR-611 | 62% | miR-4499 | 83% | miR-6762-3p | 52% |
| miR-612 | 72% | miR-4505 | 68% | miR-6763-5p | 71% |
| miR-613 | 55% | miR-4508 | 77% | miR-6763-3p | 79% |
| miR-625-5p | 76% | miR-4512 | 80% | miR-6764-3p | 64% |
| miR-634 | 76% | miR-4513 | 72% | miR-6765-5p | 74% |
| miR-636 | 85% | miR-4516 | 77% | miR-6766-5p | 73% |
| miR-638 | 78% | miR-4521 | 70% | miR-6766-3p | 80% |
| miR-639 | 75% | miR-4525 | 95% | miR-6768-5p | 76% |
| miR-650 | 56% | miR-4526 | 71% | miR-6769a-3p | 75% |
| miR-652-3p | 100% | miR-4530 | 79% | miR-6770-3p | 61% |
| miR-661 | 59% | miR-4531 | 77% | miR-6772-3p | 58% |
| miR-663a | 71% | miR-4533 | 61% | miR-6773-3p | 51% |
| miR-654-5p | 65% | miR-4534 | 72% | miR-6774-5p | 80% |
| miR-658 | 75% | miR-548am-3p | 100% | miR-6775-5p | 70% |
| miR-421 | 51% | miR-1587 | 53% | miR-6776-5p | 62% |
| miR-542-5p | 86% | miR-4536-5p | 100% | miR-6776-3p | 79% |
| miR-668-3p | 74% | miR-4539 | 66% | miR-6777-5p | 68% |
| miR-766-3p | 75% | miR-3120-5p | 51% | miR-6777-3p | 76% |
| miR-297 | 59% | miR-3127-3p | 82% | miR-6779-5p | 69% |
| miR-15a-3p | 54% | miR-3150a-5p | 53% | miR-6781-5p | 78% |
| miR-22-5p | 100% | miR-3158-5p | 74% | miR-6782-5p | 82% |
| miR-25-5p | 56% | miR-3162-3p | 81% | miR-6782-3p | 77% |
| miR-92a-2-5p | 65% | miR-3173-5p | 72% | miR-6784-5p | 77% |
| miR-29b-1-5p | 57% | miR-3187-5p | 77% | miR-6784-3p | 80% |
| miR-30c-2-3p | 87% | miR-3619-3p | 79% | miR-6785-3p | 78% |
| miR-139-3p | 62% | miR-3691-3p | 52% | miR-6786-5p | 69% |
| miR-181a-2-3p | 73% | miR-3150b-5p | 79% | miR-6787-5p | 79% |
| miR-183-3p | 67% | miR-3940-5p | 69% | miR-6787-3p | 71% |
| miR-187-5p | 66% | miR-3960 | 65% | miR-6789-5p | 88% |
| miR-214-5p | 51% | miR-3972 | 76% | miR-6789-3p | 77% |
| miR-30b-3p | 54% | miR-3978 | 88% | miR-6790-5p | 82% |
| miR-135a-3p | 63% | miR-4634 | 85% | miR-6790-3p | 86% |
| miR-127-5p | 71% | miR-4637 | 100% | miR-6791-5p | 75% |
| miR-149-3p | 76% | miR-4638-5p | 54% | miR-6792-5p | 76% |
| miR-150-3p | 79% | miR-4639-5p | 100% | miR-6792-3p | 81% |
| miR-185-3p | 53% | miR-4640-5p | 77% | miR-6793-3p | 82% |
| miR-186-3p | 53% | miR-4648 | 71% | miR-6794-5p | 86% |
| miR-194-3p | 60% | miR-4649-5p | 76% | miR-6795-5p | 79% |
| miR-30c-1-3p | 60% | miR-4651 | 78% | miR-6795-3p | 77% |
| miR-296-3p | 65% | miR-4652-5p | 67% | miR-6796-3p | 77% |
| miR-361-3p | 74% | miR-4655-5p | 63% | miR-6797-5p | 79% |
| miR-371a-5p | 67% | miR-4661-5p | 54% | miR-6797-3p | 72% |
| miR-342-5p | 86% | miR-4664-5p | 69% | miR-6798-5p | 81% |
| miR-339-3p | 53% | miR-4664-3p | 78% | miR-6798-3p | 78% |
| miR-424-3p | 55% | miR-4665-5p | 89% | miR-6799-5p | 71% |
| miR-18b-3p | 75% | miR-4665-3p | 77% | miR-6799-3p | 84% |
| miR-431-3p | 72% | miR-4667-3p | 74% | miR-6800-5p | 74% |
| miR-483-5p | 74% | miR-4668-5p | 100% | miR-6800-3p | 75% |
| miR-486-3p | 76% | miR-4673 | 63% | miR-6801-3p | 77% |
| miR-509-5p | 64% | miR-4674 | 77% | miR-6802-5p | 64% |
| miR-92b-5p | 83% | miR-4684-3p | 64% | miR-6802-3p | 69% |
| miR-550a-5p | 74% | miR-4685-5p | 69% | miR-6803-5p | 64% |
| miR-615-5p | 73% | miR-4685-3p | 79% | miR-6803-3p | 83% |
| miR-625-3p | 76% | miR-4687-5p | 76% | miR-6804-3p | 78% |
| miR-744-5p | 73% | miR-4687-3p | 69% | miR-6805-5p | 81% |
| miR-744-3p | 68% | miR-1343-3p | 85% | miR-6805-3p | 77% |
| miR-885-5p | 73% | miR-4688 | 69% | miR-6806-5p | 73% |
| miR-885-3p | 57% | miR-4690-5p | 77% | miR-6807-5p | 70% |
| miR-877-5p | 63% | miR-4691-5p | 66% | miR-6808-5p | 68% |
| miR-877-3p | 82% | miR-4691-3p | 90% | miR-6808-3p | 53% |
| miR-887-3p | 69% | miR-4695-5p | 64% | miR-6809-5p | 58% |
| miR-665 | 74% | miR-4697-5p | 69% | miR-6809-3p | 85% |
| miR-374b-3p | 100% | miR-4697-3p | 74% | miR-6810-5p | 71% |
| miR-760 | 72% | miR-4706 | 75% | miR-6810-3p | 75% |
| miR-920 | 76% | miR-4707-5p | 79% | miR-6812-5p | 74% |
| miR-935 | 64% | miR-4707-3p | 79% | miR-6812-3p | 86% |
| miR-936 | 73% | miR-4708-5p | 62% | miR-6813-5p | 64% |
| miR-937-3p | 70% | miR-4708-3p | 66% | miR-6813-3p | 76% |
| miR-939-5p | 77% | miR-4715-5p | 55% | miR-6815-5p | 61% |
| miR-940 | 75% | miR-4716-5p | 80% | miR-6816-5p | 79% |
| miR-943 | 73% | miR-4716-3p | 89% | miR-6816-3p | 70% |
| miR-1224-3p | 75% | miR-4717-3p | 66% | miR-6817-3p | 65% |
| miR-1225-5p | 79% | miR-4722-5p | 71% | miR-6819-3p | 77% |
| miR-1225-3p | 78% | miR-4723-5p | 80% | miR-6820-5p | 67% |
| miR-1226-3p | 72% | miR-4723-3p | 77% | miR-6820-3p | 76% |
| miR-1228-5p | 71% | miR-4725-5p | 77% | miR-6821-5p | 79% |
| miR-1228-3p | 72% | miR-4725-3p | 81% | miR-6821-3p | 82% |
| miR-1229-3p | 81% | miR-4726-3p | 67% | miR-6822-5p | 73% |
| miR-1231 | 84% | miR-4728-5p | 76% | miR-6824-3p | 83% |
| miR-1234-3p | 74% | miR-4728-3p | 72% | miR-6825-5p | 71% |
| miR-1237-3p | 75% | miR-4730 | 66% | miR-6825-3p | 82% |
| miR-1238-3p | 75% | miR-4731-5p | 63% | miR-6828-5p | 59% |
| miR-1181 | 86% | miR-4731-3p | 72% | miR-6829-5p | 83% |
| miR-1182 | 79% | miR-4734 | 76% | miR-6830-5p | 93% |
| miR-1184 | 67% | miR-4736 | 58% | miR-6830-3p | 74% |
| miR-1203 | 74% | miR-4738-5p | 75% | miR-6832-5p | 53% |
| miR-663b | 78% | miR-4738-3p | 59% | miR-6832-3p | 62% |
| miR-1204 | 64% | miR-4741 | 78% | miR-6833-5p | 71% |
| miR-1207-5p | 86% | miR-4745-5p | 78% | miR-6833-3p | 82% |
| miR-1208 | 55% | miR-4746-3p | 66% | miR-6780b-5p | 77% |
| miR-1286 | 57% | miR-4747-5p | 80% | miR-6780b-3p | 77% |
| miR-1299 | 100% | miR-4748 | 68% | miR-6836-5p | 52% |
| miR-1303 | 52% | miR-4749-5p | 68% | miR-6836-3p | 82% |
| miR-1246 | 67% | miR-4749-3p | 78% | miR-6837-3p | 66% |
| miR-1249-3p | 77% | miR-4750-5p | 61% | miR-6838-5p | 70% |
| miR-1253 | 63% | miR-371b-5p | 78% | miR-6840-5p | 76% |
| miR-1255a | 100% | miR-371b-3p | 70% | miR-6840-3p | 80% |
| miR-1260a | 78% | miR-4755-3p | 55% | miR-6842-5p | 75% |
| miR-1267 | 82% | miR-4756-5p | 72% | miR-6845-5p | 78% |
| miR-1268a | 71% | miR-4758-5p | 83% | miR-6845-3p | 82% |
| miR-1269a | 65% | miR-4758-3p | 74% | miR-6846-5p | 73% |
| miR-1275 | 75% | miR-4761-3p | 69% | miR-6848-5p | 71% |
| miR-1281 | 83% | miR-4763-5p | 84% | miR-6848-3p | 73% |
| miR-1292-5p | 82% | miR-4763-3p | 73% | miR-6849-5p | 64% |
| miR-1307-3p | 61% | miR-4764-5p | 100% | miR-6849-3p | 81% |
| miR-1825 | 87% | miR-4767 | 57% | miR-6850-5p | 79% |
| miR-1469 | 81% | miR-4769-3p | 77% | miR-6852-3p | 68% |
| miR-1470 | 85% | miR-4776-3p | 51% | miR-6855-3p | 78% |
| miR-1538 | 59% | miR-4778-5p | 60% | miR-6857-5p | 69% |
| miR-1908-5p | 82% | miR-4436b-5p | 80% | miR-6857-3p | 78% |
| miR-1909-5p | 69% | miR-4781-5p | 71% | miR-6858-3p | 78% |
| miR-1909-3p | 78% | miR-4783-3p | 77% | miR-6859-3p | 75% |
| miR-1910-5p | 87% | miR-4786-3p | 54% | miR-6861-5p | 71% |
| miR-1912-3p | 56% | miR-4787-5p | 77% | miR-6861-3p | 76% |
| miR-1913 | 75% | miR-4793-3p | 73% | miR-6862-5p | 60% |
| miR-1914-3p | 91% | miR-4798-3p | 100% | miR-6862-3p | 86% |
| miR-1915-3p | 78% | miR-4800-5p | 67% | miR-6865-3p | 84% |
| miR-365a-5p | 74% | miR-4800-3p | 55% | miR-6867-5p | 62% |
| miR-196b-3p | 52% | miR-4433a-5p | 77% | miR-6868-3p | 55% |
| miR-449b-3p | 77% | miR-4482-3p | 77% | miR-6869-5p | 72% |
| miR-1972 | 65% | miR-4536-3p | 100% | miR-6870-5p | 75% |
| miR-1976 | 84% | miR-5001-5p | 76% | miR-6870-3p | 76% |
| miR-2052 | 100% | miR-5001-3p | 61% | miR-6872-5p | 68% |
| miR-449c-5p | 59% | miR-5002-5p | 56% | miR-6873-5p | 51% |
| miR-762 | 78% | miR-5002-3p | 55% | miR-6875-5p | 74% |
| miR-670-5p | 63% | miR-5008-5p | 71% | miR-6875-3p | 70% |
| miR-761 | 51% | miR-5010-5p | 77% | miR-6876-3p | 53% |
| miR-2116-3p | 74% | miR-5088-5p | 61% | miR-6877-5p | 77% |
| miR-548q | 61% | miR-5090 | 71% | miR-6877-3p | 83% |
| miR-2276-3p | 57% | miR-5092 | 67% | miR-6878-3p | 74% |
| miR-711 | 85% | miR-5190 | 58% | miR-6880-5p | 78% |
| miR-718 | 82% | miR-5193 | 83% | miR-6880-3p | 80% |
| miR-2681-3p | 52% | miR-5195-5p | 78% | miR-6881-5p | 73% |
| miR-3122 | 72% | miR-5195-3p | 74% | miR-6882-3p | 75% |
| miR-3126-5p | 54% | miR-5196-5p | 83% | miR-6883-5p | 80% |
| miR-3130-3p | 59% | miR-5572 | 71% | miR-6884-3p | 78% |
| miR-3130-5p | 69% | miR-5579-5p | 100% | miR-6885-3p | 74% |
| miR-3131 | 84% | miR-5579-3p | 100% | miR-6886-5p | 52% |
| miR-3132 | 65% | miR-5580-5p | 53% | miR-6886-3p | 87% |
| miR-3133 | 58% | miR-5587-3p | 59% | miR-6887-5p | 78% |
| miR-3138 | 55% | miR-5589-5p | 52% | miR-6887-3p | 75% |
| miR-3141 | 69% | miR-5681a | 56% | miR-6889-5p | 79% |
| miR-3144-5p | 82% | miR-5698 | 75% | miR-6889-3p | 74% |
| miR-3147 | 75% | miR-5705 | 61% | miR-6890-3p | 77% |
| miR-3151-5p | 69% | miR-211-3p | 73% | miR-6891-5p | 78% |
| miR-3153 | 78% | miR-345-3p | 76% | miR-6891-3p | 77% |
| miR-3074-3p | 53% | miR-584-3p | 54% | miR-6892-3p | 76% |
| miR-3155a | 72% | miR-652-5p | 75% | miR-6893-5p | 74% |
| miR-3156-5p | 75% | miR-1185-2-3p | 82% | miR-6893-3p | 71% |
| miR-3162-5p | 74% | miR-873-3p | 74% | miR-6894-3p | 81% |
| miR-3164 | 69% | miR-1247-3p | 78% | miR-6895-5p | 52% |
| miR-3173-3p | 58% | miR-3184-3p | 75% | miR-6895-3p | 69% |
| miR-1193 | 80% | miR-365b-5p | 66% | miR-7106-5p | 78% |
| miR-3177-3p | 85% | miR-1185-1-3p | 86% | miR-7107-5p | 77% |
| miR-3178 | 81% | miR-503-3p | 82% | miR-7108-5p | 76% |
| miR-3180-3p | 75% | miR-937-5p | 70% | miR-7108-3p | 79% |
| miR-3185 | 70% | miR-1227-5p | 79% | miR-7109-5p | 75% |
| miR-3186-3p | 68% | miR-1237-5p | 77% | miR-7109-3p | 76% |
| miR-3187-3p | 68% | miR-1238-5p | 81% | miR-7110-3p | 86% |
| miR-3188 | 75% | miR-3620-5p | 69% | miR-7111-5p | 79% |
| miR-3195 | 74% | miR-3934-3p | 53% | miR-7111-3p | 77% |
| miR-3196 | 79% | miR-4750-3p | 79% | miR-7112-5p | 71% |
| miR-3197 | 76% | miR-5739 | 75% | miR-7112-3p | 55% |
| miR-3199 | 51% | miR-5787 | 69% | miR-7113-3p | 82% |
| miR-3200-3p | 51% | miR-6068 | 67% | miR-7114-5p | 69% |
| miR-514b-5p | 74% | miR-6069 | 75% | miR-7114-3p | 80% |
| miR-4295 | 54% | miR-6071 | 65% | miR-7150 | 71% |
| miR-4297 | 61% | miR-6075 | 77% | miR-7151-5p | 57% |
| miR-4293 | 63% | miR-6076 | 64% | miR-7152-5p | 73% |
| miR-4294 | 76% | miR-6085 | 81% | miR-7152-3p | 56% |
| miR-4299 | 52% | miR-6086 | 78% | miR-7155-3p | 61% |
| miR-4298 | 71% | miR-6088 | 73% | miR-7704 | 81% |
| miR-4306 | 90% | miR-6089 | 80% | miR-7706 | 53% |
| miR-4312 | 85% | miR-6090 | 74% | miR-7843-5p | 72% |
| miR-4313 | 78% | miR-6124 | 79% | miR-4433b-5p | 75% |
| miR-4316 | 64% | miR-6125 | 79% | miR-4433b-3p | 93% |
| miR-4322 | 65% | miR-6126 | 70% | miR-1273h-5p | 61% |
| miR-4321 | 54% | miR-6127 | 64% | miR-1273h-3p | 51% |
| miR-4323 | 69% | miR-6131 | 63% | miR-7851-3p | 54% |
| miR-4257 | 77% | miR-6132 | 70% | miR-7855-5p | 83% |
| miR-4258 | 72% | miR-6133 | 71% | miR-8059 | 74% |
| miR-4259 | 72% | miR-6165 | 75% | miR-8063 | 69% |
| miR-4260 | 78% | miR-6501-5p | 58% | miR-8064 | 58% |
| miR-4253 | 76% | miR-6503-5p | 80% | miR-8069 | 75% |
| miR-4251 | 78% | miR-6503-3p | 61% | miR-8072 | 73% |
| miR-4326 | 76% | miR-6505-5p | 100% | miR-8073 | 76% |
| miR-4327 | 76% | miR-6511a-5p | 92% | miR-8078 | 70% |
| miR-4265 | 64% | miR-6511a-3p | 77% | miR-8079 | 56% |
| miR-2355-5p | 77% | miR-6512-3p | 51% | miR-8080 | 63% |
| miR-4269 | 79% | miR-6513-5p | 52% | miR-8087 | 84% |
| miR-4271 | 77% | miR-6514-3p | 56% | miR-8089 | 72% |
| miR-4276 | 78% | miR-6515-3p | 77% | miR-128-2-5p | 73% |
| miR-4274 | 77% | miR-6715b-3p | 56% | miR-1199-3p | 53% |
| miR-4281 | 78% | miR-6716-5p | 78% | miR-7974 | 62% |
| miR-4279 | 81% | miR-6511b-5p | 90% | miR-7977 | 67% |
| miR-4278 | 70% | miR-6720-3p | 75% | miR-7978 | 100% |
| miR-4280 | 59% | miR-6721-5p | 81% | miR-4485-5p | 78% |
| miR-4284 | 76% | miR-6722-5p | 74% | miR-8485 | 76% |
| miR-4286 | 80% | miR-6722-3p | 72% | miR-9500 | 70% |
| miR-4288 | 56% | miR-6724-5p | 79% | miR-103a-1-5p | 57% |
| miR-4289 | 59% | let-7c-3p | 53% | miR-9898 | 77% |
| miR-4290 | 85% | miR-208a-5p | 68% | miR-9902 | 73% |
| miR-2277-5p | 66% | miR-210-5p | 74% | miR-10392-5p | 70% |
| miR-3200-5p | 77% | miR-128-1-5p | 73% | miR-10392-3p | 83% |
| miR-3610 | 81% | miR-133a-5p | 100% | miR-10394-3p | 66% |
| miR-3612 | 87% | miR-489-5p | 69% | miR-10396a-5p | 82% |
| miR-3614-5p | 71% | miR-511-3p | 100% | miR-10396a-3p | 85% |
| miR-3615 | 74% | miR-181d-3p | 77% | miR-10398-5p | 76% |
| miR-3619-5p | 56% | miR-585-5p | 58% | miR-10400-5p | 69% |
| miR-3621 | 70% | miR-598-5p | 53% | miR-10400-3p | 74% |
| miR-3622a-5p | 55% | miR-627-3p | 100% | miR-10401-5p | 77% |
| miR-3622a-3p | 70% | miR-1296-3p | 84% | miR-10401-3p | 85% |
| miR-3646 | 60% | miR-1301-5p | 61% | miR-10396b-5p | 80% |
| miR-3648 | 79% | miR-874-5p | 69% | miR-10396b-3p | 75% |
| miR-3652 | 55% | miR-216b-3p | 59% | miR-10522-5p | 67% |
| miR-3663-3p | 74% | miR-1266-3p | 52% | miR-10526-3p | 93% |
| miR-3665 | 76% | miR-1908-3p | 84% | miR-11399 | 69% |
| miR-3667-5p | 57% | miR-3151-3p | 81% | miR-3059-5p | 79% |
| miR-3675-3p | 77% | miR-3192-3p | 68% | miR-3059-3p | 65% |
| miR-3677-3p | 57% | miR-1343-5p | 85% | miR-3085-5p | 60% |
| miR-3679-5p | 75% | miR-5189-3p | 55% | miR-3085-3p | 72% |
| miR-3679-3p | 77% | miR-5699-5p | 73% | miR-12115 | 69% |
| miR-3688-3p | 60% | miR-6726-5p | 70% | miR-12116 | 73% |
| miR-3714 | 77% | miR-6726-3p | 81% | miR-12118 | 82% |
| miR-3180 | 79% | miR-6727-5p | 76% | miR-12119 | 86% |
| miR-3907 | 80% | miR-6728-5p | 68% | miR-12120 | 71% |
| miR-3908 | 56% | miR-6728-3p | 73% | miR-12121 | 67% |
| miR-3917 | 65% | miR-6729-5p | 83% | miR-12128 | 74% |
| miR-3922-3p | 67% | miR-6729-3p | 72% | miR-12131 | 100% |
| miR-3928-3p | 76% | miR-6730-5p | 81% | miR-12135 | 100% |

### [Table 22-11]

**Table 22-11: miRNA to Predict Human Cervical Cancer of Stage I**

| miRNA Name | A | miRNA Name | A | miRNA Name | A |
|---|---|---|---|---|---|
| miR-17-3p | 100% | miR-4462 | 75% | miR-6736-3p | 72% |
| miR-25-3p | 59% | miR-4463 | 71% | miR-6737-5p | 67% |
| miR-196a-5p | 61% | miR-4466 | 65% | miR-6738-5p | 71% |
| miR-197-3p | 88% | miR-4467 | 68% | miR-6738-3p | 57% |
| miR-139-5p | 100% | miR-4468 | 56% | miR-6740-5p | 52% |
| miR-210-3p | 61% | miR-4470 | 75% | miR-6740-3p | 85% |
| miR-214-3p | 56% | miR-4472 | 70% | miR-6741-5p | 70% |
| miR-224-5p | 100% | miR-4473 | 61% | miR-6741-3p | 85% |
| miR-124-3p | 67% | miR-4475 | 56% | miR-6742-5p | 76% |
| miR-140-5p | 67% | miR-4478 | 72% | miR-6742-3p | 75% |
| miR-149-5p | 80% | miR-3689d | 68% | miR-6743-5p | 71% |
| miR-188-5p | 71% | miR-3155b | 78% | miR-6743-3p | 80% |
| miR-320a-3p | 73% | miR-4480 | 100% | miR-6744-5p | 70% |
| miR-106b-5p | 86% | miR-4481 | 63% | miR-6744-3p | 67% |
| miR-296-5p | 70% | miR-4483 | 68% | miR-6746-5p | 78% |
| miR-365a-3p, miR-365b-3p | 72% | miR-4484 | 74% | miR-6748-5p | 67% |
| miR-373-5p | 74% | miR-4486 | 68% | miR-6748-3p | 83% |
| miR-373-3p | 55% | miR-4489 | 57% | miR-6749-5p | 77% |
| miR-375-3p | 67% | miR-548al | 100% | miR-6749-3p | 78% |
| miR-380-3p | 77% | miR-4492 | 70% | miR-6750-3p | 79% |
| miR-383-5p | 75% | miR-4495 | 100% | miR-6751-5p | 65% |
| miR-328-3p | 83% | miR-4496 | 83% | miR-6751-3p | 81% |
| miR-133b | 74% | miR-4497 | 66% | miR-6752-5p | 70% |
| miR-449a | 70% | miR-4504 | 100% | miR-6752-3p | 72% |
| miR-191-3p | 76% | miR-4505 | 69% | miR-6753-5p | 67% |
| miR-200a-5p | 53% | miR-2392 | 69% | miR-6753-3p | 72% |
| miR-483-3p | 87% | miR-4507 | 68% | miR-6754-5p | 65% |
| miR-484 | 72% | miR-4508 | 63% | miR-6754-3p | 73% |
| miR-485-5p | 53% | miR-4512 | 81% | miR-6756-5p | 70% |
| miR-486-5p | 72% | miR-4513 | 71% | miR-6756-3p | 75% |
| miR-491-5p | 73% | miR-4519 | 83% | miR-6757-3p | 75% |
| miR-492 | 51% | miR-4521 | 73% | miR-6758-5p | 68% |
| miR-493-5p | 63% | miR-1269b | 68% | miR-6759-5p | 66% |
| miR-432-5p | 54% | miR-4522 | 67% | miR-6759-3p | 75% |
| miR-498-5p | 81% | miR-4523 | 70% | miR-6760-3p | 77% |
| miR-519b-3p | 55% | miR-4524a-3p | 67% | miR-6761-3p | 79% |
| miR-518c-5p | 75% | miR-4525 | 64% | miR-6762-5p | 58% |
| miR-516b-5p | 75% | miR-4526 | 67% | miR-6762-3p | 61% |
| miR-532-5p | 75% | miR-4530 | 73% | miR-6763-5p | 67% |
| miR-18a-3p | 51% | miR-4534 | 67% | miR-6763-3p | 74% |
| miR-539-5p | 68% | miR-1587 | 53% | miR-6764-3p | 57% |
| miR-551a | 52% | miR-4536-5p | 100% | miR-6765-5p | 68% |
| miR-554 | 52% | miR-4538 | 51% | miR-6766-5p | 72% |
| miR-557 | 71% | miR-3120-5p | 60% | miR-6766-3p | 75% |
| miR-564 | 55% | miR-3127-3p | 77% | miR-6768-5p | 73% |
| miR-571 | 80% | miR-3158-5p | 76% | miR-6769a-5p | 73% |
| miR-574-3p | 79% | miR-3162-3p | 75% | miR-6769a-3p | 75% |
| miR-575 | 58% | miR-3173-5p | 76% | miR-6771-5p | 63% |
| miR-578 | 57% | miR-3619-3p | 72% | miR-6772-5p | 61% |
| miR-611 | 68% | miR-3691-3p | 60% | miR-6772-3p | 67% |
| miR-613 | 71% | miR-3922-5p | 53% | miR-6773-5p | 71% |
| miR-632 | 68% | miR-3944-5p | 53% | miR-6773-3p | 61% |
| miR-634 | 72% | miR-3978 | 94% | miR-6774-5p | 79% |
| miR-637 | 69% | miR-4634 | 68% | miR-6774-3p | 55% |
| miR-638 | 66% | miR-4637 | 100% | miR-6775-5p | 79% |
| miR-652-3p | 100% | miR-4638-5p | 57% | miR-6776-5p | 58% |
| miR-661 | 63% | miR-4639-3p | 58% | miR-6776-3p | 76% |
| miR-663a | 71% | miR-4640-5p | 69% | miR-6777-5p | 55% |
| miR-658 | 78% | miR-4640-3p | 74% | miR-6777-3p | 73% |
| miR-659-3p | 71% | miR-4645-5p | 55% | miR-6778-5p | 67% |
| miR-671-5p | 80% | miR-4646-5p | 67% | miR-6779-5p | 77% |
| miR-668-3p | 74% | miR-4646-3p | 79% | miR-6780a-5p | 77% |
| miR-766-3p | 77% | miR-4648 | 64% | miR-6781-5p | 66% |
| miR-765 | 77% | miR-4649-5p | 65% | miR-6782-5p | 81% |
| miR-675-5p | 74% | miR-4651 | 69% | miR-6782-3p | 74% |
| miR-297 | 55% | miR-4655-5p | 67% | miR-6783-5p | 51% |
| miR-19b-2-5p | 100% | miR-4656 | 67% | miR-6783-3p | 67% |
| miR-22-5p | 100% | miR-4661-5p | 72% | miR-6784-5p | 62% |
| miR-24-2-5p | 52% | miR-4661-3p | 51% | miR-6784-3p | 73% |
| miR-26b-3p | 52% | miR-4664-5p | 65% | miR-6785-3p | 72% |
| miR-92a-2-5p | 71% | miR-4664-3p | 73% | miR-6786-5p | 65% |
| miR-29b-1-5p | 77% | miR-4665-5p | 74% | miR-6786-3p | 72% |
| miR-139-3p | 63% | miR-4665-3p | 71% | miR-6787-5p | 77% |
| miR-181a-2-3p | 67% | miR-4667-5p | 71% | miR-6787-3p | 76% |
| miR-181c-3p | 75% | miR-4667-3p | 74% | miR-6789-5p | 66% |
| miR-183-3p | 71% | miR-4668-5p | 100% | miR-6789-3p | 73% |
| miR-23b-5p | 54% | miR-4669 | 59% | miR-6790-5p | 68% |
| miR-124-5p | 63% | miR-4670-3p | 100% | miR-6791-5p | 66% |
| miR-125b-1-3p | 100% | miR-4671-3p | 67% | miR-6792-5p | 73% |
| miR-135a-3p | 60% | miR-4674 | 63% | miR-6792-3p | 78% |
| miR-140-3p | 59% | miR-4675 | 68% | miR-6793-3p | 77% |
| miR-143-5p | 63% | miR-4685-5p | 73% | miR-6794-5p | 75% |
| miR-145-3p | 75% | miR-4685-3p | 80% | miR-6794-3p | 83% |
| miR-127-5p | 72% | miR-4687-5p | 76% | miR-6795-5p | 58% |
| miR-149-3p | 72% | miR-4687-3p | 68% | miR-6795-3p | 75% |
| miR-150-3p | 80% | miR-1343-3p | 78% | miR-6796-5p | 55% |
| miR-186-3p | 63% | miR-4688 | 66% | miR-6796-3p | 73% |
| miR-194-3p | 51% | miR-4690-5p | 67% | miR-6797-5p | 84% |
| miR-30c-1-3p | 78% | miR-4691-5p | 71% | miR-6797-3p | 73% |
| miR-296-3p | 75% | miR-4691-3p | 92% | miR-6798-5p | 67% |
| miR-361-3p | 74% | miR-4695-5p | 63% | miR-6798-3p | 76% |
| miR-371a-5p | 69% | miR-4695-3p | 75% | miR-6799-5p | 78% |
| miR-339-3p | 53% | miR-4697-5p | 72% | miR-6800-5p | 70% |
| miR-423-5p | 71% | miR-4697-3p | 75% | miR-6800-3p | 74% |
| miR-18b-3p | 71% | miR-4698 | 79% | miR-6801-3p | 74% |
| miR-20b-3p | 53% | miR-4704-3p | 100% | miR-6802-5p | 71% |
| miR-483-5p | 79% | miR-4706 | 79% | miR-6802-3p | 71% |
| miR-490-5p | 100% | miR-4707-5p | 68% | miR-6803-5p | 66% |
| miR-509-5p | 58% | miR-4707-3p | 75% | miR-6803-3p | 84% |
| miR-92b-5p | 69% | miR-4708-5p | 63% | miR-6804-3p | 79% |
| miR-574-5p | 64% | miR-4708-3p | 67% | miR-6805-5p | 71% |
| miR-550a-5p | 72% | miR-4709-3p | 71% | miR-6805-3p | 73% |
| miR-624-3p | 57% | miR-4710 | 65% | miR-6806-5p | 69% |
| miR-625-3p | 74% | miR-4712-3p | 56% | miR-6807-5p | 78% |
| miR-629-5p | 64% | miR-4713-5p | 76% | miR-6808-5p | 72% |
| miR-671-3p | 71% | miR-4713-3p | 51% | miR-6808-3p | 75% |
| miR-298 | 73% | miR-4715-5p | 76% | miR-6809-5p | 71% |
| miR-541-5p | 52% | miR-4716-5p | 75% | miR-6809-3p | 85% |
| miR-744-3p | 55% | miR-4716-3p | 77% | miR-6810-5p | 66% |
| miR-885-5p | 74% | miR-4721 | 62% | miR-6810-3p | 77% |
| miR-885-3p | 60% | miR-4722-5p | 66% | miR-6812-5p | 80% |
| miR-877-5p | 57% | miR-4722-3p | 76% | miR-6813-5p | 69% |
| miR-877-3p | 84% | miR-4723-3p | 79% | miR-6813-3p | 74% |
| miR-665 | 71% | miR-451b | 70% | miR-6816-5p | 72% |
| miR-760 | 69% | miR-4725-5p | 80% | miR-6816-3p | 68% |
| miR-920 | 73% | miR-4725-3p | 80% | miR-6817-3p | 76% |
| miR-933 | 65% | miR-4726-5p | 70% | miR-6819-5p | 68% |
| miR-936 | 72% | miR-4726-3p | 65% | miR-6819-3p | 75% |
| miR-937-3p | 66% | miR-4728-5p | 72% | miR-6820-5p | 58% |
| miR-939-5p | 66% | miR-4728-3p | 74% | miR-6820-3p | 76% |
| miR-940 | 71% | miR-4729 | 100% | miR-6821-5p | 74% |
| miR-943 | 65% | miR-4730 | 66% | miR-6821-3p | 79% |
| miR-1224-3p | 76% | miR-4731-5p | 70% | miR-6823-3p | 79% |
| miR-1225-5p | 78% | miR-4731-3p | 73% | miR-6824-5p | 73% |
| miR-1225-3p | 72% | miR-4732-5p | 71% | miR-6824-3p | 78% |
| miR-1228-3p | 73% | miR-4733-5p | 51% | miR-6825-5p | 75% |
| miR-1229-3p | 80% | miR-4736 | 61% | miR-6825-3p | 74% |
| miR-1231 | 62% | miR-4738-5p | 86% | miR-6826-5p | 76% |
| miR-1233-3p | 76% | miR-4738-3p | 64% | miR-6826-3p | 72% |
| miR-1234-3p | 74% | miR-4739 | 65% | miR-6828-5p | 64% |
| miR-1236-3p | 74% | miR-4740-3p | 52% | miR-6829-5p | 82% |
| miR-1237-3p | 74% | miR-4741 | 69% | miR-6830-5p | 79% |
| miR-1238-3p | 73% | miR-122b-5p | 100% | miR-6830-3p | 80% |
| miR-1264 | 55% | miR-4745-5p | 68% | miR-6831-5p | 71% |
| miR-320b | 72% | miR-4746-3p | 65% | miR-6832-5p | 67% |
| miR-320c | 56% | miR-4747-5p | 83% | miR-6832-3p | 69% |
| miR-1323 | 69% | miR-4748 | 74% | miR-6833-5p | 75% |
| miR-1298-5p | 55% | miR-4749-5p | 72% | miR-6833-3p | 81% |
| miR-1180-3p | 100% | miR-4749-3p | 74% | miR-6835-5p | 65% |
| miR-1182 | 81% | miR-4750-5p | 65% | miR-6780b-5p | 77% |
| miR-1184 | 72% | miR-4751 | 74% | miR-6780b-3p | 79% |
| miR-1202 | 75% | miR-4753-5p | 63% | miR-6836-3p | 68% |
| miR-1203 | 73% | miR-371b-3p | 72% | miR-6837-5p | 52% |
| miR-663b | 75% | miR-4755-3p | 53% | miR-6837-3p | 54% |
| miR-1204 | 67% | miR-4756-5p | 73% | miR-6838-5p | 61% |
| miR-1207-5p | 75% | miR-4758-5p | 72% | miR-6839-5p | 56% |
| miR-1207-3p | 62% | miR-4758-3p | 74% | miR-6839-3p | 56% |
| miR-1208 | 74% | miR-4761-5p | 67% | miR-6840-5p | 75% |
| miR-1299 | 100% | miR-4762-3p | 80% | miR-6840-3p | 71% |
| miR-1249-3p | 73% | miR-4763-5p | 80% | miR-6842-5p | 74% |
| miR-1253 | 67% | miR-4763-3p | 69% | miR-6845-5p | 69% |
| miR-1255a | 100% | miR-4769-3p | 78% | miR-6845-3p | 82% |
| miR-1260a | 79% | miR-4778-5p | 74% | miR-6846-5p | 70% |
| miR-1267 | 79% | miR-4780 | 64% | miR-6846-3p | 70% |
| miR-1268a | 65% | miR-4436b-5p | 79% | miR-6848-5p | 67% |
| miR-1272 | 63% | miR-4781-5p | 75% | miR-6848-3p | 73% |
| miR-1275 | 69% | miR-4783-3p | 74% | miR-6849-5p | 56% |
| miR-1278 | 100% | miR-2467-5p | 59% | miR-6849-3p | 84% |
| miR-1281 | 87% | miR-4787-3p | 78% | miR-6850-5p | 62% |
| miR-1292-5p | 82% | miR-4788 | 72% | miR-6851-5p | 69% |
| miR-1255b-5p | 100% | miR-4790-3p | 100% | miR-6851-3p | 77% |
| miR-1307-3p | 57% | miR-4793-3p | 71% | miR-6852-3p | 65% |
| miR-1825 | 87% | miR-4798-5p | 100% | miR-6855-5p | 51% |
| miR-675-3p | 74% | miR-4800-5p | 70% | miR-6855-3p | 73% |
| miR-1469 | 70% | miR-4803 | 71% | miR-6856-5p | 66% |
| miR-1538 | 61% | miR-4804-5p | 83% | miR-6857-3p | 77% |
| miR-1908-5p | 72% | miR-642a-3p | 81% | miR-6858-5p | 70% |
| miR-1909-3p | 69% | miR-4433a-5p | 74% | miR-6858-3p | 76% |
| miR-1912-3p | 72% | miR-4482-3p | 57% | miR-6859-3p | 72% |
| miR-1913 | 72% | miR-4999-5p | 59% | miR-6769b-5p | 76% |
| miR-1914-3p | 84% | miR-5001-5p | 73% | miR-6860 | 57% |
| miR-1915-3p | 67% | miR-5002-3p | 61% | miR-6861-5p | 71% |
| miR-365a-5p | 74% | miR-5003-3p | 67% | miR-6861-3p | 77% |
| miR-196b-3p | 52% | miR-548ao-3p | 80% | miR-6862-3p | 85% |
| miR-449b-3p | 79% | miR-5006-5p | 67% | miR-6865-5p | 75% |
| miR-1972 | 65% | miR-5006-3p | 60% | miR-6865-3p | 80% |
| miR-1976 | 86% | miR-5010-5p | 73% | miR-6866-5p | 100% |
| miR-2052 | 100% | miR-5088-5p | 67% | miR-6867-5p | 75% |
| miR-2110 | 70% | miR-5090 | 59% | miR-6867-3p | 79% |
| miR-670-5p | 67% | miR-5094 | 52% | miR-6870-5p | 76% |
| miR-2116-3p | 72% | miR-5187-3p | 54% | miR-6870-3p | 74% |
| miR-548q | 70% | miR-5193 | 85% | miR-6873-5p | 58% |
| miR-2276-3p | 58% | miR-5195-5p | 76% | miR-6873-3p | 71% |
| miR-711 | 79% | miR-5195-3p | 72% | miR-6875-5p | 70% |
| miR-718 | 71% | miR-5196-5p | 82% | miR-6876-3p | 67% |
| miR-2681-3p | 64% | miR-5196-3p | 81% | miR-6877-5p | 68% |
| miR-449c-3p | 62% | miR-5572 | 64% | miR-6877-3p | 82% |
| miR-3115 | 100% | miR-548as-5p | 100% | miR-6878-5p | 63% |
| miR-3120-3p | 56% | miR-548as-3p | 100% | miR-6879-5p | 69% |
| miR-3130-5p | 70% | miR-5579-5p | 100% | miR-6879-3p | 78% |
| miR-3131 | 70% | miR-5579-3p | 100% | miR-6880-5p | 76% |
| miR-3132 | 74% | miR-5584-5p | 74% | miR-6880-3p | 75% |
| miR-3138 | 71% | miR-5584-3p | 55% | miR-6881-5p | 72% |
| miR-3141 | 76% | miR-5585-5p | 75% | miR-6881-3p | 69% |
| miR-3144-5p | 79% | miR-5586-3p | 55% | miR-6882-3p | 75% |
| miR-3147 | 71% | miR-5589-5p | 57% | miR-6883-3p | 77% |
| miR-548v | 100% | miR-5684 | 58% | miR-6884-3p | 78% |
| miR-3151-5p | 73% | miR-5685 | 55% | miR-6885-3p | 73% |
| miR-3153 | 81% | miR-5690 | 55% | miR-6886-5p | 59% |
| miR-3154 | 71% | miR-5693 | 70% | miR-6886-3p | 86% |
| miR-3155a | 66% | miR-5695 | 75% | miR-6887-5p | 53% |
| miR-3157-5p | 61% | miR-5698 | 75% | miR-6887-3p | 75% |
| miR-3158-3p | 52% | miR-197-5p | 70% | miR-6889-5p | 67% |
| miR-3162-5p | 76% | miR-204-3p | 66% | miR-6889-3p | 73% |
| miR-3163 | 67% | miR-211-3p | 66% | miR-6890-5p | 68% |
| miR-3165 | 63% | miR-514a-5p | 100% | miR-6890-3p | 75% |
| miR-1260b | 79% | miR-584-3p | 54% | miR-6891-5p | 79% |
| miR-3171 | 100% | miR-652-5p | 82% | miR-6891-3p | 74% |
| miR-3173-3p | 65% | miR-1185-2-3p | 77% | miR-6892-5p | 56% |
| miR-1193 | 56% | miR-1304-3p | 73% | miR-6892-3p | 74% |
| miR-3175 | 69% | miR-1247-3p | 70% | miR-6893-5p | 70% |
| miR-3178 | 60% | miR-1306-5p | 73% | miR-6893-3p | 75% |
| miR-3180-3p | 69% | miR-3184-3p | 79% | miR-6894-5p | 71% |
| miR-3182 | 60% | miR-3191-5p | 76% | miR-6894-3p | 81% |
| miR-3184-5p | 73% | miR-642b-5p | 69% | miR-6895-5p | 53% |
| miR-3185 | 67% | miR-365b-5p | 74% | miR-6895-3p | 69% |
| miR-3188 | 67% | miR-1185-1-3p | 79% | miR-7106-5p | 76% |
| miR-3194-5p | 77% | miR-376c-5p | 100% | miR-7106-3p | 72% |
| miR-3195 | 69% | miR-503-3p | 76% | miR-7107-5p | 75% |
| miR-3196 | 58% | miR-376a-2-5p | 100% | miR-7108-5p | 72% |
| miR-3197 | 71% | miR-937-5p | 76% | miR-7108-3p | 74% |
| miR-3198 | 75% | miR-939-3p | 78% | miR-7109-5p | 80% |
| miR-514b-5p | 76% | miR-1227-5p | 76% | miR-7109-3p | 79% |
| miR-3202 | 77% | miR-1229-5p | 73% | miR-7110-5p | 72% |
| miR-4297 | 70% | miR-1233-5p | 70% | miR-7110-3p | 84% |
| miR-4293 | 73% | miR-1237-5p | 62% | miR-7111-5p | 77% |
| miR-4294 | 81% | miR-1238-5p | 75% | miR-7111-3p | 83% |
| miR-4299 | 51% | miR-1292-3p | 74% | miR-7112-5p | 63% |
| miR-4298 | 74% | miR-3620-5p | 66% | miR-7113-3p | 79% |
| miR-4313 | 73% | miR-3934-3p | 51% | miR-7114-5p | 66% |
| miR-4316 | 74% | miR-4632-5p | 70% | miR-7114-3p | 75% |
| miR-4319 | 52% | miR-4743-3p | 52% | miR-7150 | 72% |
| miR-4322 | 64% | miR-4750-3p | 76% | miR-7151-5p | 59% |
| miR-4323 | 74% | miR-5739 | 72% | miR-7152-5p | 70% |
| miR-4257 | 75% | miR-5787 | 64% | miR-7155-5p | 69% |
| miR-4258 | 71% | miR-6068 | 56% | miR-7155-3p | 66% |
| miR-4260 | 76% | miR-6069 | 72% | miR-7157-3p | 64% |
| miR-4326 | 82% | miR-6071 | 70% | miR-7158-3p | 68% |
| miR-4327 | 70% | miR-6077 | 75% | miR-7159-5p | 56% |
| miR-4265 | 70% | miR-6078 | 53% | miR-7160-5p | 60% |
| miR-4266 | 63% | miR-6086 | 77% | miR-7161-5p | 55% |
| miR-2355-5p | 72% | miR-6124 | 81% | miR-7515 | 59% |
| miR-4268 | 73% | miR-6125 | 66% | miR-7843-5p | 70% |
| miR-4270 | 65% | miR-6126 | 61% | miR-7843-3p | 58% |
| miR-4271 | 81% | miR-6127 | 68% | miR-4433b-5p | 75% |
| miR-4276 | 61% | miR-378j | 52% | miR-4433b-3p | 80% |
| miR-4274 | 73% | miR-6131 | 63% | miR-1273h-5p | 62% |
| miR-4281 | 69% | miR-6132 | 68% | miR-1273h-3p | 67% |
| miR-4279 | 87% | miR-6133 | 77% | miR-7845-5p | 68% |
| miR-4280 | 59% | miR-6165 | 68% | miR-7846-3p | 67% |
| miR-4285 | 63% | miR-6500-5p | 51% | miR-7847-3p | 69% |
| miR-4284 | 72% | miR-6501-5p | 52% | miR-7852-3p | 100% |
| miR-4286 | 78% | miR-6505-5p | 100% | miR-7855-5p | 78% |
| miR-4287 | 72% | miR-6507-5p | 79% | miR-8052 | 62% |
| miR-4288 | 69% | miR-6510-5p | 73% | miR-8053 | 100% |
| miR-4290 | 86% | miR-6511a-5p | 72% | miR-8059 | 76% |
| miR-2277-5p | 51% | miR-6511a-3p | 82% | miR-8060 | 75% |
| miR-3605-3p | 75% | miR-6512-5p | 100% | miR-8061 | 100% |
| miR-3610 | 74% | miR-6512-3p | 56% | miR-8063 | 58% |
| miR-3612 | 77% | miR-6513-5p | 65% | miR-8064 | 65% |
| miR-3613-5p | 100% | miR-6515-3p | 73% | miR-8065 | 100% |
| miR-3614-5p | 73% | miR-6715b-3p | 67% | miR-8071 | 62% |
| miR-3615 | 71% | miR-6716-5p | 77% | miR-8072 | 68% |
| miR-3617-5p | 100% | miR-6716-3p | 72% | miR-8073 | 73% |
| miR-3619-5p | 62% | miR-6511b-5p | 67% | miR-8077 | 58% |
| miR-3620-3p | 74% | miR-6511b-3p | 77% | miR-8078 | 57% |
| miR-3622a-3p | 75% | miR-6718-5p | 51% | miR-8079 | 51% |
| miR-3646 | 71% | miR-6719-3p | 71% | miR-8085 | 64% |
| miR-3648 | 73% | miR-6720-3p | 86% | miR-8087 | 86% |
| miR-3649 | 74% | miR-6721-5p | 69% | miR-8089 | 73% |
| miR-3652 | 51% | miR-6722-3p | 66% | miR-7974 | 54% |
| miR-3663-5p | 54% | miR-6724-5p | 71% | miR-7977 | 69% |
| miR-3663-3p | 67% | miR-210-5p | 74% | miR-7978 | 100% |
| miR-3675-3p | 77% | miR-128-1-5p | 65% | miR-301b-5p | 83% |
| miR-3677-3p | 67% | miR-133a-5p | 100% | miR-1249-5p | 76% |
| miR-3679-5p | 83% | miR-152-5p | 75% | miR-4485-5p | 76% |
| miR-3679-3p | 73% | miR-489-5p | 63% | miR-8485 | 79% |
| miR-3681-5p | 100% | miR-511-3p | 100% | miR-9500 | 54% |
| miR-3682-3p | 53% | miR-181d-3p | 76% | miR-103a-1-5p | 57% |
| miR-3692-3p | 59% | miR-504-3p | 65% | miR-137-5p | 100% |
| miR-3180 | 72% | miR-487b-5p | 60% | miR-190b-3p | 100% |
| miR-3907 | 86% | miR-585-5p | 55% | miR-9718 | 57% |
| miR-3908 | 67% | miR-598-5p | 61% | miR-9898 | 73% |
| miR-3911 | 76% | miR-619-5p | 62% | miR-9902 | 52% |
| miR-3917 | 63% | miR-627-3p | 100% | miR-10392-5p | 66% |
| miR-3922-3p | 68% | miR-1296-3p | 75% | miR-10392-3p | 64% |
| miR-3923 | 100% | miR-1301-5p | 56% | miR-10396a-5p | 68% |
| miR-3926 | 63% | miR-874-5p | 72% | miR-10396a-3p | 74% |
| miR-3928-3p | 74% | miR-216b-3p | 71% | miR-10398-5p | 77% |
| miR-3937 | 69% | miR-208b-5p | 100% | miR-10398-3p | 69% |
| miR-3940-3p | 79% | miR-1266-3p | 63% | miR-10399-3p | 83% |
| miR-3943 | 74% | miR-3151-3p | 78% | miR-10400-3p | 64% |
| miR-3945 | 66% | miR-500b-3p | 52% | miR-10401-5p | 67% |
| miR-642b-3p | 76% | miR-3912-5p | 75% | miR-10396b-5p | 66% |
| miR-550b-3p | 68% | miR-1343-5p | 72% | miR-10522-5p | 78% |
| miR-1268b | 71% | miR-5189-3p | 61% | miR-10526-3p | 73% |
| miR-378f | 54% | miR-5699-5p | 76% | miR-11181-3p | 72% |
| miR-4424 | 100% | miR-6726-5p | 59% | miR-11399 | 59% |
| miR-4428 | 53% | miR-6726-3p | 78% | miR-11400 | 55% |
| miR-4430 | 60% | miR-6727-3p | 78% | miR-3059-5p | 78% |
| miR-4432 | 100% | miR-6728-5p | 55% | miR-3059-3p | 62% |
| miR-4433a-3p | 78% | miR-6728-3p | 78% | miR-3085-5p | 68% |
| miR-4434 | 100% | miR-6729-5p | 65% | miR-3085-3p | 72% |
| miR-4439 | 64% | miR-6729-3p | 72% | miR-12114 | 54% |
| miR-4440 | 54% | miR-6730-5p | 70% | miR-12115 | 71% |
| miR-4441 | 77% | miR-6730-3p | 75% | miR-12116 | 72% |
| miR-4444 | 58% | miR-6731-3p | 74% | miR-12118 | 67% |
| miR-4447 | 72% | miR-6732-5p | 71% | miR-12119 | 78% |
| miR-4448 | 64% | miR-6732-3p | 81% | miR-12120 | 73% |
| miR-4449 | 70% | miR-6734-5p | 74% | miR-12121 | 66% |
| miR-548ah-5p | 100% | miR-6735-5p | 71% | miR-12128 | 65% |
| miR-4455 | 60% | miR-6735-3p | 72% | miR-12131 | 100% |
| miR-3135b | 65% | miR-6736-5p | 65% | miR-12136 | 69% |

### [Table 22-12]

**Table 22-12: miRNA to Predict Human Rectal Cancer of Stage I**

| miRNA Name | A | miRNA Name | A | miRNA Name | A |
|---|---|---|---|---|---|
| miR-15a-5p | 62% | miR-3941 | 63% | miR-548ay-3p | 80% |
| miR-30a-5p | 60% | miR-642b-3p | 75% | miR-6506-5p | 54% |
| miR-124-3p | 67% | miR-1268b | 88% | miR-6510-5p | 74% |
| miR-373-5p | 80% | miR-548ab | 57% | miR-6721-5p | 89% |
| miR-373-3p | 66% | miR-4422 | 67% | miR-6722-3p | 87% |
| miR-325 | 55% | miR-4433a-3p | 84% | miR-6724-5p | 83% |
| miR-202-3p | 59% | miR-4439 | 58% | miR-210-5p | 78% |
| miR-492 | 54% | miR-4446-3p | 82% | miR-329-5p | 52% |
| miR-520c-3p | 71% | miR-4447 | 82% | miR-410-5p | 52% |
| miR-516b-5p | 60% | miR-4458 | 62% | miR-1343-5p | 88% |
| miR-552-3p | 64% | miR-4460 | 76% | miR-6729-5p | 88% |
| miR-557 | 81% | miR-378h | 64% | miR-6729-3p | 72% |
| miR-585-3p | 59% | miR-3135b | 84% | miR-6732-5p | 87% |
| miR-617 | 53% | miR-4463 | 83% | miR-6743-5p | 86% |
| miR-638 | 87% | miR-4466 | 83% | miR-6752-5p | 85% |
| miR-639 | 86% | miR-4467 | 87% | miR-6755-5p | 67% |
| miR-663a | 82% | miR-4470 | 78% | miR-6756-5p | 85% |
| miR-449b-5p | 55% | miR-4484 | 77% | miR-6763-5p | 82% |
| miR-542-5p | 86% | miR-4492 | 85% | miR-6763-3p | 75% |
| miR-769-5p | 55% | miR-4497 | 85% | miR-6768-5p | 84% |
| miR-297 | 57% | miR-4505 | 85% | miR-6774-5p | 78% |
| miR-92a-2-5p | 81% | miR-4507 | 89% | miR-6775-5p | 78% |
| miR-187-5p | 80% | miR-4508 | 87% | miR-6777-3p | 74% |
| miR-200b-5p | 51% | miR-4520-3p | 59% | miR-6781-5p | 84% |
| miR-124-5p | 75% | miR-4521 | 72% | miR-6784-5p | 85% |
| miR-130a-5p | 55% | miR-4530 | 84% | miR-6784-3p | 74% |
| miR-149-3p | 84% | miR-4634 | 87% | miR-6785-3p | 73% |
| miR-148b-5p | 59% | miR-4640-5p | 86% | miR-6786-5p | 85% |
| miR-18b-3p | 75% | miR-4650-3p | 60% | miR-6787-5p | 84% |
| miR-92b-5p | 83% | miR-4651 | 85% | miR-6789-5p | 87% |
| miR-624-3p | 57% | miR-4653-3p | 60% | miR-6791-5p | 90% |
| miR-629-5p | 56% | miR-4659b-3p | 67% | miR-6797-5p | 75% |
| miR-876-3p | 67% | miR-4665-5p | 81% | miR-6798-5p | 87% |
| miR-665 | 89% | miR-4665-3p | 74% | miR-6799-5p | 79% |
| miR-1224-3p | 76% | miR-4668-5p | 100% | miR-6800-3p | 75% |
| miR-1225-5p | 83% | miR-4672 | 53% | miR-6805-5p | 85% |
| miR-1225-3p | 74% | miR-4674 | 79% | miR-6809-5p | 65% |
| miR-1228-3p | 75% | miR-4685-5p | 78% | miR-6810-3p | 74% |
| miR-1238-3p | 75% | miR-4687-5p | 80% | miR-6813-5p | 86% |
| miR-320c | 57% | miR-4688 | 85% | miR-6818-5p | 64% |
| miR-1202 | 77% | miR-4706 | 79% | miR-6819-3p | 74% |
| miR-1207-5p | 82% | miR-4707-5p | 89% | miR-6821-5p | 87% |
| miR-1208 | 55% | miR-4716-5p | 72% | miR-6824-5p | 80% |
| miR-1294 | 65% | miR-4725-3p | 77% | miR-6829-5p | 80% |
| miR-1304-5p | 61% | miR-4728-3p | 75% | miR-6832-5p | 61% |
| miR-1249-3p | 73% | miR-4734 | 84% | miR-6780b-5p | 73% |
| miR-1268a | 86% | miR-4741 | 82% | miR-6836-3p | 79% |
| miR-1270 | 54% | miR-4745-5p | 88% | miR-6838-5p | 56% |
| miR-1292-5p | 83% | miR-4749-5p | 82% | miR-6839-5p | 55% |
| miR-1469 | 86% | miR-4753-5p | 59% | miR-6840-3p | 87% |
| miR-1908-5p | 84% | miR-4758-5p | 86% | miR-6845-5p | 83% |
| miR-1909-3p | 85% | miR-4759 | 92% | miR-6845-3p | 78% |
| miR-1912-3p | 65% | miR-4763-3p | 88% | miR-6848-5p | 84% |
| miR-1915-3p | 87% | miR-4770 | 60% | miR-6850-5p | 84% |
| miR-3119 | 64% | miR-4779 | 57% | miR-6858-5p | 79% |
| miR-3120-3p | 58% | miR-4781-5p | 78% | miR-6861-5p | 85% |
| miR-3141 | 82% | miR-4783-3p | 84% | miR-6875-5p | 83% |
| miR-1273c | 73% | miR-4999-5p | 58% | miR-6880-5p | 73% |
| miR-3148 | 52% | miR-5087 | 54% | miR-6880-3p | 75% |
| miR-3163 | 56% | miR-5196-5p | 76% | miR-6882-5p | 57% |
| miR-3165 | 60% | miR-5586-3p | 55% | miR-6887-3p | 74% |
| miR-3178 | 82% | miR-548au-3p | 56% | miR-6892-3p | 74% |
| miR-3180-3p | 87% | miR-5684 | 67% | miR-7107-5p | 74% |
| miR-3184-5p | 78% | miR-5690 | 57% | miR-7108-5p | 82% |
| miR-3196 | 84% | miR-197-5p | 83% | miR-7111-5p | 72% |
| miR-3197 | 86% | miR-382-3p | 52% | miR-7150 | 75% |
| miR-4293 | 54% | miR-652-5p | 78% | miR-7515 | 64% |
| miR-4294 | 73% | miR-1185-2-3p | 75% | miR-4433b-3p | 82% |
| miR-4298 | 75% | miR-3529-3p | 75% | miR-7847-3p | 78% |
| miR-4321 | 56% | miR-381-5p | 54% | miR-8072 | 85% |
| miR-4323 | 77% | miR-937-5p | 85% | miR-8074 | 53% |
| miR-4256 | 64% | miR-1227-5p | 89% | miR-203a-5p | 55% |
| miR-4258 | 82% | miR-1237-5p | 84% | miR-1249-5p | 76% |
| miR-4327 | 84% | miR-3620-5p | 89% | miR-498-3p | 57% |
| miR-4271 | 75% | miR-3934-3p | 53% | miR-9898 | 74% |
| miR-4280 | 54% | miR-4632-5p | 81% | miR-9985 | 60% |
| miR-500b-5p | 57% | miR-6069 | 79% | miR-10392-5p | 84% |
| miR-3648 | 82% | miR-6071 | 65% | miR-10392-3p | 80% |
| miR-3654 | 53% | miR-6075 | 85% | miR-10396a-5p | 84% |
| miR-3663-3p | 84% | miR-6081 | 60% | miR-10396a-3p | 86% |
| miR-3677-3p | 65% | miR-6086 | 80% | miR-10398-3p | 81% |
| miR-3688-3p | 60% | miR-6090 | 85% | miR-10400-3p | 87% |
| miR-3916 | 59% | miR-6124 | 74% | miR-10396b-5p | 83% |
| miR-3919 | 59% | miR-6125 | 88% | miR-3085-3p | 77% |
| miR-3928-3p | 82% | miR-6126 | 76% | miR-12120 | 84% |
| miR-3937 | 82% | miR-6132 | 84% | | |

### [Table 22-13]

**Table 22-13: miRNA to Predict Human Endometrial Cancer of Stage I**

| miRNA Name | A | miRNA Name | A | miRNA Name | A |
|---|---|---|---|---|---|
| let-7e-5p | 54% | miR-3622a-3p | 67% | miR-6508-5p | 57% |
| miR-15a-5p | 64% | miR-3622b-5p | 73% | miR-6509-5p | 56% |
| miR-17-3p | 100% | miR-3646 | 58% | miR-6509-3p | 64% |
| miR-29a-3p | 75% | miR-3648 | 77% | miR-6510-5p | 72% |
| miR-30a-5p | 64% | miR-3649 | 62% | miR-6511a-5p | 79% |
| miR-31-5p | 100% | miR-3652 | 70% | miR-6511a-3p | 65% |
| miR-29b-3p | 63% | miR-3654 | 63% | miR-6512-3p | 54% |
| miR-107 | 57% | miR-3660 | 63% | miR-6513-5p | 68% |
| miR-196a-5p | 72% | miR-3663-5p | 66% | miR-6514-5p | 52% |
| miR-197-3p | 69% | miR-3663-3p | 72% | miR-6514-3p | 59% |
| miR-198 | 68% | miR-3665 | 68% | miR-6515-5p | 68% |
| miR-199a-5p | 56% | miR-3667-3p | 72% | miR-6515-3p | 74% |
| miR-199a-3p, miR-199b-3p | 69% | miR-3675-5p | 59% | miR-6715b-3p | 67% |
| miR-30d-5p | 68% | miR-3675-3p | 71% | miR-6716-5p | 68% |
| miR-139-5p | 100% | miR-3677-3p | 73% | miR-6716-3p | 63% |
| miR-187-3p | 57% | miR-3679-5p | 76% | miR-6717-5p | 74% |
| miR-199b-5p | 64% | miR-3679-3p | 71% | miR-6511b-5p | 77% |
| miR-211-5p | 53% | miR-3682-3p | 52% | miR-6511b-3p | 70% |
| miR-214-3p | 55% | miR-3688-3p | 75% | miR-6719-3p | 74% |
| miR-218-5p | 57% | miR-3689a-3p | 59% | miR-6721-5p | 70% |
| miR-223-3p | 68% | miR-3691-5p | 64% | miR-6722-5p | 65% |
| miR-122-5p | 55% | miR-3714 | 64% | miR-6722-3p | 70% |
| miR-138-5p | 51% | miR-3180 | 67% | miR-6724-5p | 73% |
| miR-140-5p | 80% | miR-3907 | 83% | miR-208a-5p | 64% |
| miR-141-3p | 75% | miR-3689b-3p, miR-3689c | 55% | miR-210-5p | 69% |
| miR-125a-5p | 56% | miR-3909 | 68% | miR-128-1-5p | 72% |
| miR-134-5p | 62% | miR-3911 | 66% | miR-370-5p | 58% |
| miR-149-5p | 64% | miR-3913-5p | 58% | miR-328-5p | 65% |
| miR-184 | 54% | miR-3916 | 68% | miR-329-5p | 57% |
| miR-185-5p | 61% | miR-3917 | 70% | miR-410-5p | 62% |
| miR-188-5p | 56% | miR-3918 | 55% | miR-487a-5p | 55% |
| miR-206 | 62% | miR-3919 | 73% | miR-489-5p | 62% |
| miR-106b-5p | 88% | miR-3150b-3p | 56% | miR-181d-3p | 80% |
| miR-29c-3p | 100% | miR-3924 | 100% | miR-504-3p | 74% |
| miR-34c-5p | 60% | miR-3925-5p | 63% | miR-487b-5p | 60% |
| miR-299-3p | 58% | miR-3926 | 65% | miR-585-5p | 58% |
| miR-296-5p | 67% | miR-676-3p | 59% | miR-597-3p | 67% |
| miR-130b-3p | 83% | miR-3928-3p | 73% | miR-598-5p | 71% |
| miR-361-5p | 66% | miR-3934-5p | 55% | miR-605-3p | 61% |
| miR-365a-3p, miR-365b-3p | 69% | miR-3935 | 60% | miR-619-5p | 57% |
| miR-302c-5p | 63% | miR-3937 | 76% | miR-1296-3p | 64% |
| miR-370-3p | 73% | miR-3940-3p | 69% | miR-891a-3p | 57% |
| miR-371a-3p | 53% | miR-3943 | 69% | miR-874-5p | 70% |
| miR-373-5p | 65% | miR-3944-3p | 70% | miR-1180-5p | 51% |
| miR-373-3p | 71% | miR-3945 | 63% | miR-1250-3p | 55% |
| miR-374a-5p | 100% | miR-642b-3p | 69% | miR-1266-3p | 60% |
| miR-375-3p | 65% | miR-550b-3p | 62% | miR-1908-3p | 70% |
| miR-380-3p | 70% | miR-1268b | 78% | miR-3151-3p | 65% |
| miR-382-5p | 53% | miR-378e | 54% | miR-3192-3p | 59% |
| miR-383-5p | 70% | miR-548ab | 79% | miR-500b-3p | 51% |
| miR-328-3p | 69% | miR-378f | 59% | miR-3912-5p | 75% |
| miR-342-3p | 57% | miR-4421 | 58% | miR-1343-5p | 76% |
| miR-326 | 65% | miR-4428 | 71% | miR-5088-3p | 61% |
| miR-151a-3p | 59% | miR-4429 | 69% | miR-5189-3p | 61% |
| miR-133b | 68% | miR-4430 | 77% | miR-5699-5p | 70% |
| miR-325 | 61% | miR-4431 | 52% | miR-6726-5p | 79% |
| miR-346 | 64% | miR-4433a-3p | 77% | miR-6726-3p | 65% |
| miR-20b-5p | 67% | miR-4434 | 100% | miR-6727-5p | 68% |
| miR-448 | 56% | miR-4435 | 51% | miR-6727-3p | 67% |
| miR-429 | 100% | miR-4436a | 55% | miR-6728-5p | 52% |
| miR-449a | 65% | miR-4437 | 51% | miR-6728-3p | 70% |
| miR-450a-5p | 62% | miR-4439 | 71% | miR-6729-5p | 71% |
| miR-200a-5p | 64% | miR-4440 | 71% | miR-6729-3p | 70% |
| miR-433-3p | 61% | miR-4441 | 70% | miR-6730-5p | 75% |
| miR-323b-5p | 54% | miR-4443 | 66% | miR-6730-3p | 71% |
| miR-452-3p | 55% | miR-4444 | 62% | miR-6731-5p | 55% |
| miR-409-3p | 67% | miR-4446-3p | 71% | miR-6731-3p | 73% |
| miR-483-3p | 65% | miR-4447 | 76% | miR-6732-5p | 77% |
| miR-484 | 68% | miR-4448 | 63% | miR-6732-3p | 68% |
| miR-485-3p | 60% | miR-4449 | 68% | miR-6734-5p | 76% |
| miR-486-5p | 70% | miR-4450 | 58% | miR-6734-3p | 65% |
| miR-487a-3p | 54% | miR-4451 | 56% | miR-6735-5p | 62% |
| miR-491-5p | 71% | miR-4454 | 59% | miR-6735-3p | 61% |
| miR-202-3p | 68% | miR-4455 | 59% | miR-6736-5p | 64% |
| miR-492 | 67% | miR-4456 | 66% | miR-6736-3p | 66% |
| miR-193b-3p | 51% | miR-4458 | 72% | miR-6737-5p | 74% |
| miR-498-5p | 79% | miR-4460 | 69% | miR-6737-3p | 70% |
| miR-520e-3p | 63% | miR-378h | 64% | miR-6738-5p | 72% |
| miR-519c-5p, miR-523-5p, miR-518e-5p, miR-522-5p, miR-519a-5p, miR-519b-5p | 51% | miR-3135b | 73% | miR-6738-3p | 62% |
| miR-519b-3p | 55% | miR-4462 | 71% | miR-6740-5p | 62% |
| miR-525-3p | 61% | miR-4463 | 68% | miR-6740-3p | 64% |
| miR-520b-3p | 61% | miR-4466 | 73% | miR-6741-5p | 70% |
| miR-518b | 67% | miR-4467 | 74% | miR-6741-3p | 67% |
| miR-520c-3p | 75% | miR-4470 | 87% | miR-6742-5p | 75% |
| miR-518c-5p | 62% | miR-4472 | 77% | miR-6742-3p | 71% |
| miR-520d-5p | 54% | miR-4474-3p | 51% | miR-6743-5p | 75% |
| miR-520d-3p | 55% | miR-4475 | 68% | miR-6743-3p | 67% |
| miR-516b-5p | 75% | miR-4476 | 62% | miR-6744-5p | 58% |
| miR-516b-3p, miR-516a-3p | 61% | miR-4478 | 71% | miR-6744-3p | 61% |
| miR-518a-3p | 78% | miR-3689f | 60% | miR-6745 | 66% |
| miR-517c-3p | 51% | miR-4479 | 51% | miR-6746-5p | 77% |
| miR-519a-3p | 58% | miR-4481 | 72% | miR-6747-5p | 69% |
| miR-500a-3p | 63% | miR-4483 | 74% | miR-6747-3p | 63% |
| miR-502-5p | 62% | miR-4484 | 74% | miR-6748-5p | 78% |
| miR-503-5p | 86% | miR-4486 | 76% | miR-6748-3p | 65% |
| miR-513a-5p | 65% | miR-4487 | 74% | miR-6749-5p | 74% |
| miR-539-5p | 61% | miR-4488 | 67% | miR-6749-3p | 69% |
| miR-487b-3p | 55% | miR-4489 | 70% | miR-6750-5p | 57% |
| miR-551a | 52% | miR-4492 | 66% | miR-6750-3p | 68% |
| miR-552-3p | 64% | miR-4494 | 59% | miR-6751-5p | 77% |
| miR-554 | 62% | miR-4496 | 78% | miR-6751-3p | 65% |
| miR-555 | 59% | miR-4497 | 71% | miR-6752-5p | 74% |
| miR-557 | 68% | miR-4498 | 74% | miR-6752-3p | 69% |
| miR-563 | 67% | miR-4499 | 65% | miR-6753-5p | 68% |
| miR-564 | 69% | miR-4501 | 62% | miR-6753-3p | 66% |
| miR-571 | 80% | miR-4505 | 73% | miR-6754-5p | 65% |
| miR-572 | 50% | miR-4506 | 68% | miR-6754-3p | 61% |
| miR-573 | 100% | miR-2392 | 77% | miR-6755-5p | 75% |
| miR-574-3p | 68% | miR-4507 | 77% | miR-6755-3p | 55% |
| miR-575 | 63% | miR-4508 | 70% | miR-6756-5p | 71% |
| miR-578 | 70% | miR-4510 | 64% | miR-6756-3p | 72% |
| miR-579-3p | 71% | miR-4511 | 62% | miR-6757-5p | 63% |
| miR-583 | 67% | miR-4512 | 75% | miR-6757-3p | 67% |
| miR-585-3p | 63% | miR-4513 | 73% | miR-6758-5p | 70% |
| miR-550a-3p | 51% | miR-4516 | 70% | miR-6759-5p | 78% |
| miR-595 | 56% | miR-4518 | 67% | miR-6759-3p | 71% |
| miR-608 | 62% | miR-4519 | 80% | miR-6760-5p | 71% |
| miR-610 | 64% | miR-4520-3p | 67% | miR-6760-3p | 72% |
| miR-612 | 55% | miR-4521 | 77% | miR-6761-5p | 52% |
| miR-615-3p | 59% | miR-1269b | 67% | miR-6761-3p | 62% |
| miR-616-5p | 75% | miR-4522 | 61% | miR-6762-5p | 69% |
| miR-617 | 65% | miR-4523 | 55% | miR-6762-3p | 63% |
| miR-619-3p | 100% | miR-4524a-3p | 75% | miR-6763-5p | 75% |
| miR-626 | 100% | miR-4525 | 68% | miR-6763-3p | 73% |
| miR-628-3p | 62% | miR-4526 | 66% | miR-6765-5p | 65% |
| miR-632 | 51% | miR-4530 | 74% | miR-6766-5p | 78% |
| miR-634 | 71% | miR-4531 | 63% | miR-6766-3p | 71% |
| miR-636 | 61% | miR-4533 | 70% | miR-6767-5p | 61% |
| miR-637 | 76% | miR-4534 | 71% | miR-6768-5p | 71% |
| miR-638 | 73% | miR-378i | 55% | miR-6769a-5p | 69% |
| miR-639 | 88% | miR-4535 | 73% | miR-6769a-3p | 72% |
| miR-642a-5p | 62% | miR-1587 | 77% | miR-6770-5p | 65% |
| miR-649 | 51% | miR-4538 | 71% | miR-6770-3p | 53% |
| miR-663a | 71% | miR-4539 | 73% | miR-6771-5p | 57% |
| miR-449b-5p | 61% | miR-4540 | 56% | miR-6771-3p | 72% |
| miR-657 | 56% | miR-3127-3p | 66% | miR-6772-5p | 73% |
| miR-658 | 79% | miR-3129-3p | 51% | miR-6772-3p | 60% |
| miR-542-5p | 94% | miR-3074-5p | 55% | miR-6773-3p | 57% |
| miR-363-5p | 56% | miR-3156-3p | 60% | miR-6774-5p | 71% |
| miR-425-5p | 56% | miR-3158-5p | 67% | miR-6775-5p | 63% |
| miR-671-5p | 65% | miR-3162-3p | 71% | miR-6775-3p | 68% |
| miR-668-3p | 62% | miR-3173-5p | 62% | miR-6776-5p | 74% |
| miR-767-3p | 70% | miR-3177-5p | 56% | miR-6776-3p | 70% |
| miR-769-5p | 64% | miR-3187-5p | 69% | miR-6777-5p | 72% |
| miR-769-3p | 55% | miR-3189-5p | 68% | miR-6777-3p | 71% |
| miR-766-3p | 72% | miR-3619-3p | 72% | miR-6778-5p | 74% |
| miR-765 | 68% | miR-3682-5p | 51% | miR-6778-3p | 63% |
| miR-675-5p | 67% | miR-3913-3p | 54% | miR-6779-5p | 68% |
| miR-297 | 66% | miR-3150b-5p | 68% | miR-6779-3p | 65% |
| let-7b-3p | 67% | miR-3922-5p | 54% | miR-6780a-5p | 70% |
| let-7d-3p | 58% | miR-3925-3p | 61% | miR-6780a-3p | 58% |
| let-7f-1-3p | 61% | miR-3940-5p | 69% | miR-6781-5p | 74% |
| miR-22-5p | 100% | miR-4423-5p | 53% | miR-6782-5p | 78% |
| miR-24-2-5p | 57% | miR-3960 | 63% | miR-6782-3p | 73% |
| miR-26b-3p | 50% | miR-3972 | 57% | miR-6783-5p | 66% |
| miR-32-3p | 53% | miR-4632-3p | 54% | miR-6783-3p | 58% |
| miR-92a-2-5p | 68% | miR-4634 | 72% | miR-6784-5p | 70% |
| miR-29b-2-5p | 59% | miR-4638-5p | 76% | miR-6784-3p | 71% |
| miR-129-1-3p | 65% | miR-4639-3p | 56% | miR-6785-3p | 71% |
| miR-30c-2-3p | 65% | miR-4640-5p | 77% | miR-6786-5p | 73% |
| miR-139-3p | 68% | miR-4640-3p | 65% | miR-6786-3p | 72% |
| miR-181c-3p | 75% | miR-4642 | 68% | miR-6787-5p | 71% |
| miR-183-3p | 67% | miR-4643 | 58% | miR-6787-3p | 61% |
| miR-187-5p | 69% | miR-4644 | 56% | miR-6788-5p | 55% |
| miR-200b-5p | 59% | miR-4646-5p | 69% | miR-6788-3p | 63% |
| miR-15b-3p | 65% | miR-4646-3p | 66% | miR-6789-5p | 74% |
| miR-23b-5p | 60% | miR-4647 | 55% | miR-6789-3p | 59% |
| miR-30b-3p | 60% | miR-4648 | 73% | miR-6790-5p | 73% |
| miR-124-5p | 70% | miR-4649-5p | 59% | miR-6790-3p | 68% |
| miR-125b-1-3p | 100% | miR-4649-3p | 68% | miR-6791-5p | 71% |
| miR-130a-5p | 59% | miR-4650-3p | 75% | miR-6792-5p | 65% |
| miR-135a-3p | 59% | miR-4651 | 77% | miR-6792-3p | 70% |
| miR-140-3p | 63% | miR-4652-5p | 54% | miR-6793-5p | 63% |
| miR-125a-3p | 57% | miR-4652-3p | 67% | miR-6793-3p | 69% |
| miR-125b-2-3p | 69% | miR-4653-3p | 67% | miR-6794-5p | 77% |
| miR-129-2-3p | 68% | miR-4654 | 62% | miR-6794-3p | 65% |
| miR-138-1-3p | 55% | miR-4655-5p | 69% | miR-6795-5p | 72% |
| miR-149-3p | 68% | miR-4656 | 68% | miR-6795-3p | 72% |
| miR-150-3p | 72% | miR-4657 | 60% | miR-6796-5p | 74% |
| miR-185-3p | 75% | miR-4659a-5p | 62% | miR-6796-3p | 73% |
| miR-186-3p | 67% | miR-4661-5p | 59% | miR-6797-5p | 75% |
| miR-188-3p | 59% | miR-4661-3p | 57% | miR-6797-3p | 68% |
| miR-193a-5p | 60% | miR-4659b-3p | 71% | miR-6798-5p | 75% |
| miR-194-3p | 68% | miR-4664-5p | 67% | miR-6798-3p | 72% |
| miR-30c-1-3p | 62% | miR-4664-3p | 71% | miR-6799-5p | 71% |
| miR-219a-2-3p | 56% | miR-4665-5p | 80% | miR-6799-3p | 70% |
| miR-296-3p | 67% | miR-4665-3p | 70% | miR-6800-5p | 65% |
| miR-130b-5p | 53% | miR-4667-5p | 73% | miR-6800-3p | 69% |
| miR-361-3p | 68% | miR-4667-3p | 68% | miR-6801-5p | 52% |
| miR-371a-5p | 63% | miR-4669 | 69% | miR-6801-3p | 68% |
| miR-377-5p | 63% | miR-4670-5p | 59% | miR-6802-5p | 63% |
| miR-342-5p | 71% | miR-4670-3p | 100% | miR-6802-3p | 70% |
| miR-337-5p | 57% | miR-4671-3p | 80% | miR-6803-5p | 65% |
| miR-323a-5p | 53% | miR-4672 | 67% | miR-6803-3p | 67% |
| miR-151a-5p | 56% | miR-4673 | 59% | miR-6804-3p | 67% |
| miR-148b-5p | 61% | miR-4674 | 70% | miR-6805-5p | 72% |
| miR-338-5p | 57% | miR-4676-5p | 58% | miR-6805-3p | 72% |
| miR-423-5p | 68% | miR-4677-5p | 78% | miR-6806-5p | 64% |
| miR-424-3p | 76% | miR-4681 | 58% | miR-6806-3p | 100% |
| miR-18b-3p | 69% | miR-4682 | 55% | miR-6807-5p | 67% |
| miR-20b-3p | 76% | miR-4684-3p | 61% | miR-6808-5p | 67% |
| miR-431-3p | 52% | miR-4685-5p | 67% | miR-6808-3p | 63% |
| miR-483-5p | 78% | miR-4685-3p | 65% | miR-6809-5p | 80% |
| miR-486-3p | 70% | miR-4687-5p | 73% | miR-6809-3p | 61% |
| miR-490-5p | 100% | miR-4687-3p | 66% | miR-6810-5p | 80% |
| miR-146b-3p | 52% | miR-1343-3p | 76% | miR-6810-3p | 73% |
| miR-193b-5p | 54% | miR-4688 | 74% | miR-6812-5p | 73% |
| miR-509-5p | 62% | miR-4689 | 64% | miR-6812-3p | 68% |
| miR-532-3p | 68% | miR-4690-5p | 70% | miR-6813-5p | 73% |
| miR-92b-5p | 75% | miR-4690-3p | 63% | miR-6813-3p | 70% |
| miR-551b-5p | 66% | miR-4691-5p | 53% | miR-6814-3p | 55% |
| miR-574-5p | 63% | miR-4691-3p | 89% | miR-6815-5p | 62% |
| miR-550a-5p | 70% | miR-4692 | 51% | miR-6815-3p | 55% |
| miR-615-5p | 71% | miR-4694-5p | 61% | miR-6816-5p | 67% |
| miR-616-3p | 62% | miR-4695-5p | 74% | miR-6816-3p | 55% |
| miR-624-3p | 75% | miR-4695-3p | 67% | miR-6818-5p | 71% |
| miR-625-3p | 71% | miR-4697-5p | 64% | miR-6819-5p | 65% |
| miR-629-5p | 83% | miR-4697-3p | 72% | miR-6819-3p | 69% |
| miR-298 | 70% | miR-4698 | 74% | miR-6820-5p | 73% |
| miR-874-3p | 66% | miR-4700-5p | 64% | miR-6820-3p | 72% |
| miR-891b | 78% | miR-4700-3p | 72% | miR-6821-5p | 76% |
| miR-892b | 51% | miR-4701-5p | 73% | miR-6822-5p | 68% |
| miR-541-3p | 59% | miR-4701-3p | 66% | miR-6823-5p | 62% |
| miR-708-5p | 53% | miR-4703-3p | 56% | miR-6823-3p | 65% |
| miR-147b-3p | 58% | miR-4704-5p | 59% | miR-6824-5p | 67% |
| miR-744-5p | 67% | miR-4706 | 65% | miR-6824-3p | 64% |
| miR-885-5p | 63% | miR-4707-5p | 71% | miR-6825-5p | 73% |
| miR-885-3p | 70% | miR-4707-3p | 72% | miR-6825-3p | 70% |
| miR-877-5p | 69% | miR-4708-5p | 58% | miR-6826-3p | 70% |
| miR-877-3p | 74% | miR-4708-3p | 64% | miR-6827-5p | 72% |
| miR-887-3p | 67% | miR-4709-3p | 69% | miR-6827-3p | 71% |
| miR-665 | 73% | miR-203b-5p | 59% | miR-6828-5p | 58% |
| miR-760 | 72% | miR-4710 | 68% | miR-6829-5p | 77% |
| miR-920 | 69% | miR-4712-3p | 54% | miR-6829-3p | 60% |
| miR-921 | 51% | miR-4713-5p | 67% | miR-6830-5p | 75% |
| miR-924 | 51% | miR-4714-5p | 66% | miR-6830-3p | 64% |
| miR-935 | 50% | miR-4715-5p | 67% | miR-6831-5p | 71% |
| miR-936 | 62% | miR-4716-5p | 72% | miR-6831-3p | 55% |
| miR-938 | 57% | miR-4716-3p | 71% | miR-6832-5p | 77% |
| miR-939-5p | 74% | miR-4717-3p | 62% | miR-6833-5p | 74% |
| miR-940 | 68% | miR-4720-5p | 67% | miR-6833-3p | 54% |
| miR-1224-5p | 71% | miR-4721 | 74% | miR-6834-5p | 72% |
| miR-1224-3p | 71% | miR-4722-5p | 60% | miR-6834-3p | 70% |
| miR-1225-5p | 73% | miR-4722-3p | 63% | miR-6780b-5p | 74% |
| miR-1225-3p | 70% | miR-4723-5p | 71% | miR-6780b-3p | 69% |
| miR-1226-3p | 52% | miR-4723-3p | 69% | miR-6836-5p | 52% |
| miR-1228-5p | 66% | miR-451b | 63% | miR-6836-3p | 71% |
| miR-1228-3p | 72% | miR-4724-5p | 63% | miR-6837-5p | 54% |
| miR-1229-3p | 66% | miR-4725-5p | 53% | miR-6838-5p | 67% |
| miR-1231 | 69% | miR-4725-3p | 76% | miR-6839-5p | 67% |
| miR-1233-3p | 64% | miR-4726-5p | 63% | miR-6840-5p | 68% |
| miR-1234-3p | 67% | miR-4726-3p | 59% | miR-6840-3p | 71% |
| miR-1236-3p | 71% | miR-4727-3p | 56% | miR-6841-3p | 69% |
| miR-1237-3p | 69% | miR-4728-5p | 71% | miR-6842-5p | 76% |
| miR-1238-3p | 69% | miR-4728-3p | 70% | miR-6842-3p | 59% |
| miR-1264 | 57% | miR-4730 | 65% | miR-6845-5p | 75% |
| miR-320b | 56% | miR-4731-5p | 64% | miR-6845-3p | 73% |
| miR-320c | 72% | miR-4731-3p | 69% | miR-6846-5p | 76% |
| miR-1296-5p | 56% | miR-4732-5p | 75% | miR-6846-3p | 66% |
| miR-1301-3p | 67% | miR-4732-3p | 61% | miR-6847-5p | 53% |
| miR-1298-5p | 66% | miR-4734 | 71% | miR-6847-3p | 59% |
| miR-1181 | 61% | miR-4736 | 70% | miR-6848-5p | 70% |
| miR-1182 | 69% | miR-3064-5p | 54% | miR-6848-3p | 67% |
| miR-1184 | 54% | miR-3064-3p | 57% | miR-6849-5p | 71% |
| miR-1202 | 67% | miR-4738-3p | 68% | miR-6849-3p | 53% |
| miR-1203 | 60% | miR-4739 | 65% | miR-6850-5p | 71% |
| miR-663b | 60% | miR-4741 | 76% | miR-6851-5p | 66% |
| miR-1207-5p | 75% | miR-4742-3p | 83% | miR-6851-3p | 69% |
| miR-1208 | 64% | miR-4743-5p | 76% | miR-6855-5p | 65% |
| miR-1285-3p | 60% | miR-122b-5p | 100% | miR-6855-3p | 71% |
| miR-1286 | 63% | miR-4745-5p | 72% | miR-6856-5p | 66% |
| miR-1289 | 73% | miR-4745-3p | 58% | miR-6857-5p | 73% |
| miR-1293 | 53% | miR-4746-3p | 73% | miR-6857-3p | 72% |
| miR-1294 | 72% | miR-4747-5p | 74% | miR-6858-5p | 65% |
| miR-1303 | 57% | miR-4747-3p | 51% | miR-6858-3p | 71% |
| miR-1304-5p | 64% | miR-4748 | 71% | miR-6859-5p | 62% |
| miR-1247-5p | 66% | miR-4749-5p | 78% | miR-6859-3p | 72% |
| miR-1249-3p | 73% | miR-4749-3p | 71% | miR-6769b-5p | 74% |
| miR-1251-5p | 60% | miR-4750-5p | 72% | miR-6769b-3p | 63% |
| miR-1257 | 60% | miR-4751 | 73% | miR-6860 | 65% |
| miR-1260a | 68% | miR-4753-5p | 70% | miR-6861-5p | 76% |
| miR-1263 | 65% | miR-371b-5p | 68% | miR-6861-3p | 70% |
| miR-1265 | 54% | miR-371b-3p | 67% | miR-6862-5p | 55% |
| miR-1266-5p | 52% | miR-4754 | 56% | miR-6862-3p | 70% |
| miR-1267 | 69% | miR-4755-3p | 61% | miR-6867-5p | 59% |
| miR-1268a | 70% | miR-499b-3p | 65% | miR-6867-3p | 64% |
| miR-1269a | 57% | miR-4756-5p | 62% | miR-6869-5p | 67% |
| miR-1270 | 64% | miR-4758-5p | 80% | miR-6870-5p | 73% |
| miR-1275 | 65% | miR-4758-3p | 70% | miR-6870-3p | 70% |
| miR-1276 | 51% | miR-4759 | 80% | miR-6871-5p | 67% |
| miR-1281 | 69% | miR-4762-3p | 83% | miR-6872-5p | 57% |
| miR-1292-5p | 88% | miR-4763-5p | 69% | miR-6872-3p | 69% |
| miR-1255b-5p | 100% | miR-4763-3p | 76% | miR-6874-5p | 61% |
| miR-664a-3p | 64% | miR-4766-5p | 79% | miR-6875-5p | 74% |
| miR-1307-3p | 72% | miR-4769-5p | 70% | miR-6875-3p | 57% |
| miR-320d | 75% | miR-4769-3p | 70% | miR-6876-5p | 74% |
| miR-1825 | 68% | miR-4776-5p | 70% | miR-6876-3p | 50% |
| miR-675-3p | 64% | miR-4776-3p | 52% | miR-6877-5p | 78% |
| miR-1469 | 73% | miR-4778-5p | 74% | miR-6877-3p | 68% |
| miR-1470 | 68% | miR-4780 | 58% | miR-6878-3p | 60% |
| miR-1471 | 71% | miR-4436b-5p | 66% | miR-6879-5p | 74% |
| miR-1538 | 60% | miR-4436b-3p | 51% | miR-6879-3p | 68% |
| miR-1539 | 59% | miR-4781-5p | 82% | miR-6880-5p | 76% |
| miR-1908-5p | 76% | miR-4783-3p | 74% | miR-6880-3p | 71% |
| miR-1909-5p | 61% | miR-4784 | 54% | miR-6881-5p | 69% |
| miR-1909-3p | 73% | miR-4785 | 60% | miR-6881-3p | 52% |
| miR-1910-5p | 63% | miR-2467-5p | 61% | miR-6882-5p | 60% |
| miR-1911-5p | 100% | miR-2467-3p | 75% | miR-6882-3p | 62% |
| miR-1912-3p | 77% | miR-4786-5p | 53% | miR-6883-5p | 72% |
| miR-1913 | 72% | miR-4786-3p | 58% | miR-6883-3p | 55% |
| miR-1914-3p | 78% | miR-4787-5p | 66% | miR-6884-3p | 70% |
| miR-1915-3p | 73% | miR-4787-3p | 70% | miR-6885-3p | 70% |
| miR-365a-5p | 72% | miR-4788 | 63% | miR-6886-5p | 57% |
| miR-449b-3p | 70% | miR-4793-3p | 65% | miR-6886-3p | 70% |
| miR-2113 | 56% | miR-4794 | 53% | miR-6887-5p | 74% |
| miR-1976 | 72% | miR-4799-5p | 63% | miR-6887-3p | 72% |
| miR-2054 | 100% | miR-4800-5p | 78% | miR-6888-5p | 56% |
| miR-2110 | 73% | miR-4801 | 55% | miR-6889-5p | 78% |
| miR-151b | 57% | miR-4804-5p | 86% | miR-6889-3p | 69% |
| miR-449c-5p | 55% | miR-4804-3p | 55% | miR-6890-5p | 62% |
| miR-762 | 70% | miR-4520-2-3p | 100% | miR-6890-3p | 70% |
| miR-670-5p | 58% | miR-642a-3p | 68% | miR-6891-5p | 67% |
| miR-761 | 52% | miR-550a-3-5p | 57% | miR-6891-3p | 72% |
| miR-764 | 63% | miR-4433a-5p | 71% | miR-6892-5p | 69% |
| miR-2116-3p | 69% | miR-4482-3p | 65% | miR-6892-3p | 70% |
| miR-548q | 69% | miR-4999-5p | 68% | miR-6893-5p | 76% |
| miR-2276-3p | 71% | miR-5000-3p | 65% | miR-6893-3p | 60% |
| miR-2278 | 55% | miR-5001-5p | 70% | miR-6894-5p | 76% |
| miR-711 | 74% | miR-5002-3p | 59% | miR-6894-3p | 66% |
| miR-718 | 66% | miR-5003-5p | 53% | miR-6895-3p | 51% |
| miR-2681-3p | 64% | miR-5003-3p | 67% | miR-7106-5p | 72% |
| miR-2682-5p | 53% | miR-5004-3p | 58% | miR-7106-3p | 61% |
| miR-2682-3p | 58% | miR-5006-5p | 55% | miR-7107-5p | 76% |
| miR-449c-3p | 56% | miR-5007-5p | 55% | miR-7107-3p | 54% |
| miR-2861 | 61% | miR-5008-5p | 62% | miR-7108-5p | 75% |
| miR-3116 | 51% | miR-5009-3p | 61% | miR-7108-3p | 71% |
| miR-3119 | 71% | miR-5010-5p | 70% | miR-7109-5p | 70% |
| miR-3120-3p | 62% | miR-5010-3p | 67% | miR-7109-3p | 64% |
| miR-3122 | 71% | miR-5087 | 65% | miR-7110-5p | 78% |
| miR-3124-5p | 54% | miR-5088-5p | 68% | miR-7110-3p | 68% |
| miR-3126-5p | 56% | miR-5089-5p | 64% | miR-7111-5p | 76% |
| miR-3130-3p | 51% | miR-5090 | 68% | miR-7111-3p | 68% |
| miR-3130-5p | 64% | miR-5092 | 75% | miR-7112-5p | 59% |
| miR-3131 | 74% | miR-5093 | 54% | miR-7113-5p | 53% |
| miR-3132 | 60% | miR-5187-5p | 54% | miR-7113-3p | 70% |
| miR-3133 | 55% | miR-5188 | 62% | miR-7114-5p | 72% |
| miR-466 | 55% | miR-5189-5p | 70% | miR-7114-3p | 70% |
| miR-3137 | 50% | miR-5190 | 55% | miR-7150 | 73% |
| miR-3138 | 68% | miR-5192 | 52% | miR-7151-3p | 63% |
| miR-3141 | 75% | miR-5193 | 62% | miR-7152-5p | 58% |
| miR-3144-5p | 65% | miR-5194 | 52% | miR-7152-3p | 52% |
| miR-1273c | 76% | miR-5195-5p | 71% | miR-7154-3p | 51% |
| miR-3147 | 73% | miR-5195-3p | 63% | miR-7155-5p | 63% |
| miR-3148 | 64% | miR-5196-5p | 77% | miR-7157-3p | 75% |
| miR-3150a-3p | 69% | miR-5196-3p | 63% | miR-7159-5p | 64% |
| miR-3151-5p | 71% | miR-4524b-5p | 61% | miR-7160-5p | 69% |
| miR-3153 | 73% | miR-5100 | 66% | miR-7161-5p | 57% |
| miR-3154 | 68% | miR-5572 | 80% | miR-7162-5p | 61% |
| miR-3156-5p | 70% | miR-664b-5p | 63% | miR-7515 | 78% |
| miR-3157-5p | 70% | miR-664b-3p | 66% | miR-7702 | 51% |
| miR-3160-3p | 65% | miR-5581-5p | 56% | miR-7704 | 70% |
| miR-3161 | 64% | miR-5581-3p | 56% | miR-7843-5p | 60% |
| miR-3162-5p | 77% | miR-5584-5p | 71% | miR-4433b-5p | 69% |
| miR-3163 | 69% | miR-548au-3p | 61% | miR-4433b-3p | 80% |
| miR-3164 | 61% | miR-5588-5p | 62% | miR-1273h-5p | 72% |
| miR-3165 | 78% | miR-5589-5p | 69% | miR-6516-5p | 62% |
| miR-1260b | 67% | miR-5684 | 75% | miR-7845-5p | 65% |
| miR-3169 | 68% | miR-5689 | 54% | miR-7846-3p | 70% |
| miR-3173-3p | 68% | miR-5690 | 69% | miR-7847-3p | 65% |
| miR-1193 | 67% | miR-4666b | 70% | miR-7851-3p | 68% |
| miR-3176 | 62% | miR-5693 | 67% | miR-7855-5p | 65% |
| miR-3177-3p | 66% | miR-5695 | 80% | miR-7856-5p | 56% |
| miR-3178 | 69% | miR-5696 | 52% | miR-8052 | 73% |
| miR-3179 | 60% | miR-5698 | 74% | miR-8054 | 86% |
| miR-3180-5p | 69% | miR-5699-3p | 57% | miR-8057 | 57% |
| miR-3180-3p | 71% | miR-5702 | 53% | miR-8059 | 68% |
| miR-3184-5p | 63% | miR-5703 | 66% | miR-8060 | 68% |
| miR-3185 | 80% | miR-5704 | 54% | miR-8062 | 55% |
| miR-3187-3p | 58% | miR-5705 | 64% | miR-8063 | 66% |
| miR-3188 | 67% | miR-5708 | 52% | miR-8064 | 67% |
| miR-3189-3p | 68% | miR-197-5p | 75% | miR-8068 | 78% |
| miR-3190-5p | 64% | miR-181b-3p | 77% | miR-8069 | 71% |
| miR-3191-3p | 69% | miR-204-3p | 70% | miR-8070 | 51% |
| miR-3192-5p | 53% | miR-211-3p | 74% | miR-8071 | 75% |
| miR-3193 | 56% | miR-301a-5p | 62% | miR-8072 | 75% |
| miR-3194-5p | 63% | miR-382-3p | 65% | miR-8073 | 77% |
| miR-3195 | 70% | miR-345-3p | 66% | miR-8074 | 63% |
| miR-3196 | 69% | miR-652-5p | 68% | miR-8075 | 61% |
| miR-3197 | 76% | miR-1185-2-3p | 71% | miR-8077 | 69% |
| miR-3198 | 66% | miR-766-5p | 54% | miR-8078 | 56% |
| miR-514b-5p | 60% | miR-873-3p | 61% | miR-8079 | 58% |
| miR-3202 | 67% | miR-1304-3p | 69% | miR-8080 | 68% |
| miR-4296 | 52% | miR-1247-3p | 74% | miR-8085 | 77% |
| miR-4297 | 59% | miR-1306-5p | 71% | miR-8086 | 57% |
| miR-4293 | 78% | miR-3184-3p | 68% | miR-8087 | 73% |
| miR-4294 | 76% | miR-3191-5p | 66% | miR-8089 | 74% |
| miR-4299 | 55% | miR-642b-5p | 57% | miR-128-2-5p | 61% |
| miR-4298 | 69% | miR-365b-5p | 68% | miR-1199-5p | 53% |
| miR-4300 | 57% | miR-1185-1-3p | 74% | miR-7977 | 62% |
| miR-4305 | 61% | miR-3190-3p | 50% | miR-7978 | 100% |
| miR-4306 | 63% | miR-98-3p | 64% | miR-1249-5p | 69% |
| miR-4307 | 65% | miR-381-5p | 70% | miR-4485-5p | 71% |
| miR-4312 | 69% | miR-503-3p | 65% | miR-8485 | 67% |
| miR-4313 | 66% | miR-937-5p | 74% | miR-196a-1-3p | 51% |
| miR-4315 | 60% | miR-939-3p | 72% | miR-217-3p | 65% |
| miR-4316 | 58% | miR-1227-5p | 78% | miR-135a-2-3p | 51% |
| miR-4314 | 61% | miR-1229-5p | 65% | miR-320a-5p | 64% |
| miR-4318 | 54% | miR-1233-5p | 69% | miR-375-5p | 64% |
| miR-4320 | 67% | miR-1236-5p | 57% | miR-498-3p | 66% |
| miR-4322 | 74% | miR-1237-5p | 71% | miR-526a-3p | 73% |
| miR-4321 | 67% | miR-1238-5p | 73% | miR-9718 | 70% |
| miR-4323 | 70% | miR-1292-3p | 62% | miR-9898 | 73% |
| miR-4324 | 59% | miR-3617-3p | 57% | miR-9899 | 55% |
| miR-4256 | 74% | miR-3620-5p | 75% | miR-1843 | 57% |
| miR-4257 | 74% | miR-3934-3p | 64% | miR-10392-5p | 73% |
| miR-4258 | 75% | miR-4632-5p | 69% | miR-10392-3p | 70% |
| miR-4259 | 73% | miR-4743-3p | 63% | miR-10394-3p | 62% |
| miR-4260 | 66% | miR-4750-3p | 71% | miR-10395-3p | 62% |
| miR-4253 | 62% | miR-5739 | 70% | miR-10396a-5p | 71% |
| miR-4251 | 68% | miR-5787 | 69% | miR-10396a-3p | 74% |
| miR-4255 | 53% | miR-6068 | 64% | miR-10398-5p | 63% |
| miR-4325 | 73% | miR-6069 | 69% | miR-10398-3p | 77% |
| miR-4326 | 60% | miR-6071 | 75% | miR-10400-5p | 63% |
| miR-4327 | 76% | miR-6072 | 66% | miR-10400-3p | 75% |
| miR-4265 | 65% | miR-6074 | 58% | miR-10401-5p | 77% |
| miR-2355-5p | 62% | miR-6075 | 69% | miR-10401-3p | 73% |
| miR-4268 | 62% | miR-6076 | 72% | miR-10396b-5p | 75% |
| miR-4269 | 70% | miR-6077 | 59% | miR-10522-5p | 85% |
| miR-4264 | 53% | miR-6078 | 58% | miR-10524-5p | 57% |
| miR-4271 | 68% | miR-6081 | 58% | miR-10526-3p | 72% |
| miR-4276 | 64% | miR-6082 | 64% | miR-10527-5p | 70% |
| miR-4274 | 70% | miR-6085 | 73% | miR-11181-5p | 65% |
| miR-4281 | 65% | miR-6086 | 76% | miR-11181-3p | 72% |
| miR-4279 | 66% | miR-6088 | 63% | miR-11399 | 76% |
| miR-4280 | 70% | miR-6089 | 71% | miR-11400 | 60% |
| miR-4285 | 68% | miR-6090 | 71% | miR-3059-5p | 70% |
| miR-4284 | 67% | miR-6124 | 75% | miR-3059-3p | 54% |
| miR-4286 | 71% | miR-6125 | 73% | miR-3085-5p | 69% |
| miR-4287 | 61% | miR-6126 | 67% | miR-3085-3p | 70% |
| miR-4292 | 69% | miR-6127 | 81% | miR-6529-5p | 58% |
| miR-4289 | 63% | miR-378j | 63% | miR-12114 | 73% |
| miR-4290 | 63% | miR-6129 | 54% | miR-12115 | 62% |
| miR-4329 | 65% | miR-6130 | 61% | miR-12116 | 71% |
| miR-500b-5p | 64% | miR-6131 | 80% | miR-12118 | 77% |
| miR-3200-5p | 63% | miR-6132 | 72% | miR-12119 | 77% |
| miR-3605-3p | 65% | miR-6133 | 74% | miR-12120 | 72% |
| miR-3610 | 72% | miR-6165 | 71% | miR-12121 | 66% |
| miR-3612 | 74% | miR-548ay-3p | 83% | miR-12122 | 52% |
| miR-3614-5p | 69% | miR-6500-5p | 63% | miR-12124 | 60% |
| miR-3614-3p | 75% | miR-6501-5p | 51% | miR-12126 | 64% |
| miR-3616-3p | 62% | miR-6501-3p | 65% | miR-12127 | 54% |
| miR-3619-5p | 52% | miR-6503-5p | 64% | miR-12128 | 55% |
| miR-3620-3p | 64% | miR-6506-5p | 60% | miR-12131 | 100% |
| miR-3621 | 72% | miR-6506-3p | 73% | miR-12136 | 55% |
| miR-3622a-5p | 73% | miR-6507-3p | 55% | | |

### [Table 23-1]

**Table 23-1: miRNA to Predict Human Lung Cancer of Stage II**

| miRNA Name | A | miRNA Name | A | miRNA Name | A |
|---|---|---|---|---|---|
| let-7b-5p | 50% | miR-3943 | 56% | miR-576-5p | 72% |
| let-7c-3p | 50% | miR-3944-3p | 67% | miR-578 | 89% |
| let-7e-5p | 78% | miR-3960 | 50% | miR-5787 | 56% |
| let-7f-1-3p | 50% | miR-3976 | 100% | miR-580-5p | 94% |
| let-7f-2-3p | 56% | miR-3978 | 78% | miR-582-3p | 100% |
| let-7g-3p | 83% | miR-424-3p | 67% | miR-585-3p | 78% |
| miR-103a-3p | 78% | miR-4252 | 72% | miR-589-5p | 56% |
| miR-106b-5p | 94% | miR-425-5p | 83% | miR-591 | 72% |
| miR-107 | 94% | miR-4257 | 67% | miR-595 | 61% |
| miR-10a-5p | 89% | miR-4259 | 78% | miR-597-3p | 89% |
| miR-10b-5p | 89% | miR-4260 | 78% | miR-598-3p | 83% |
| miR-1178-3p | 72% | miR-4262 | 61% | miR-603 | 67% |
| miR-1180-5p | 89% | miR-4268 | 50% | miR-605-3p | 78% |
| miR-1183 | 100% | miR-4269 | 72% | miR-6068 | 83% |
| miR-1184 | 56% | miR-4271 | 67% | miR-6069 | 67% |
| miR-1185-1-3p | 72% | miR-4287 | 50% | miR-6072 | 67% |
| miR-1193 | 50% | miR-429 | 100% | miR-6076 | 83% |
| miR-1199-5p | 56% | miR-4293 | 72% | miR-6077 | 50% |
| miR-1200 | 61% | miR-4296 | 78% | miR-608 | 83% |
| miR-1203 | 72% | miR-4297 | 61% | miR-6082 | 100% |
| miR-1205 | 61% | miR-4300 | 72% | miR-6086 | 83% |
| miR-122-3p | 72% | miR-4304 | 67% | miR-6087 | 61% |
| miR-1224-5p | 72% | miR-4306 | 83% | miR-6088 | 56% |
| miR-1226-3p | 56% | miR-4307 | 78% | miR-6089 | 50% |
| miR-1226-5p | 67% | miR-431-3p | 72% | miR-611 | 83% |
| miR-1229-3p | 61% | miR-431-5p | 67% | miR-6124 | 50% |
| miR-1229-5p | 56% | miR-4320 | 100% | miR-6126 | 61% |
| miR-1234-3p | 50% | miR-4321 | 61% | miR-6127 | 61% |
| miR-1237-5p | 56% | miR-432-3p | 56% | miR-6129 | 50% |
| miR-1238-3p | 50% | miR-4325 | 89% | miR-6131 | 67% |
| miR-1238-5p | 61% | miR-433-3p | 83% | miR-6133 | 72% |
| miR-1247-3p | 61% | miR-4418 | 100% | miR-614 | 67% |
| miR-1250-5p | 78% | miR-4419a | 72% | miR-616-3p | 72% |
| miR-1251-3p | 61% | miR-4419b | 61% | miR-616-5p | 94% |
| miR-1254 | 61% | miR-4420 | 72% | miR-622 | 61% |
| miR-1255b-2-3p | 78% | miR-4422 | 89% | miR-624-3p | 89% |
| miR-1257 | 78% | miR-4428 | 83% | miR-624-5p | 89% |
| miR-125a-3p | 72% | miR-4430 | 67% | miR-625-3p | 50% |
| miR-125b-5p | 61% | miR-4436a | 78% | miR-626 | 94% |
| miR-1263 | 89% | miR-4436b-3p | 89% | miR-628-3p | 56% |
| miR-1265 | 50% | miR-4437 | 72% | miR-629-3p | 50% |
| miR-1271-5p | 72% | miR-4440 | 72% | miR-630 | 78% |
| miR-1272 | 83% | miR-4441 | 78% | miR-632 | 67% |
| miR-1273a | 78% | miR-4442 | 61% | miR-635 | 56% |
| miR-1273f | 78% | miR-4443 | 67% | miR-636 | 50% |
| miR-1273g-3p | 56% | miR-4445-3p | 78% | miR-642a-3p | 61% |
| miR-1273g-5p | 72% | miR-4447 | 61% | miR-642a-5p | 72% |
| miR-1273h-5p | 67% | miR-4448 | 72% | miR-642b-3p | 61% |
| miR-1282 | 100% | miR-4449 | 61% | miR-6499-5p | 67% |
| miR-128-2-5p | 61% | miR-4451 | 61% | miR-650 | 67% |
| miR-1286 | 89% | miR-4453 | 89% | miR-6500-3p | 78% |
| miR-1289 | 94% | miR-4458 | 78% | miR-6500-5p | 67% |
| miR-1291 | 61% | miR-4459 | 67% | miR-6501-5p | 56% |
| miR-1293 | 78% | miR-4470 | 67% | miR-6503-3p | 61% |
| miR-1297 | 72% | miR-4472 | 61% | miR-6504-5p | 83% |
| miR-1298-5p | 78% | miR-4474-3p | 83% | miR-6508-5p | 56% |
| miR-1306-3p | 61% | miR-4478 | 67% | miR-6514-3p | 83% |
| miR-1306-5p | 67% | miR-4479 | 67% | miR-6515-5p | 67% |
| miR-1307-3p | 67% | miR-448 | 78% | miR-6516-5p | 56% |
| miR-130a-5p | 56% | miR-4481 | 67% | miR-652-5p | 50% |
| miR-1321 | 50% | miR-4483 | 56% | miR-653-5p | 94% |
| miR-1322 | 61% | miR-4484 | 56% | miR-655-5p | 94% |
| miR-1323 | 56% | miR-4486 | 61% | miR-656-3p | 61% |
| miR-132-3p | 61% | miR-4487 | 83% | miR-656-5p | 61% |
| miR-134-5p | 56% | miR-4489 | 72% | miR-659-3p | 67% |
| miR-135a-5p | 78% | miR-4492 | 50% | miR-659-5p | 94% |
| miR-138-1-3p | 72% | miR-4496 | 61% | miR-660-5p | 94% |
| miR-140-5p | 100% | miR-4498 | 67% | miR-661 | 67% |
| miR-143-5p | 100% | miR-4499 | 72% | miR-662 | 89% |
| miR-145-5p | 72% | miR-449c-5p | 72% | miR-663a | 50% |
| miR-1468-5p | 83% | miR-4501 | 94% | miR-663b | 72% |
| miR-1469 | 50% | miR-4502 | 72% | miR-664a-3p | 61% |
| miR-1470 | 50% | miR-4503 | 100% | miR-668-5p | 61% |
| miR-1471 | 72% | miR-4507 | 50% | miR-6715a-3p | 78% |
| miR-147b | 100% | miR-4508 | 50% | miR-6715b-3p | 100% |
| miR-148a-5p | 100% | miR-450a-2-3p | 61% | miR-671-5p | 72% |
| miR-150-3p | 67% | miR-450a-5p | 67% | miR-6723-5p | 56% |
| miR-1587 | 50% | miR-4514 | 61% | miR-6724-5p | 67% |
| miR-15a-3p | 50% | miR-4515 | 67% | miR-6726-5p | 56% |
| miR-15b-3p | 83% | miR-4518 | 61% | miR-6727-3p | 50% |
| miR-1-5p | 56% | miR-4519 | 56% | miR-6728-3p | 56% |
| miR-16-2-3p | 61% | miR-4520-5p | 72% | miR-6730-3p | 56% |
| miR-16-5p | 100% | miR-4521 | 61% | miR-6730-5p | 78% |
| miR-17-5p | 94% | miR-4522 | 61% | miR-6732-3p | 56% |
| miR-181a-5p | 89% | miR-452-3p | 89% | miR-6734-3p | 56% |
| miR-181d-3p | 89% | miR-4524a-3p | 83% | miR-6735-3p | 61% |
| miR-181d-5p | 94% | miR-4525 | 83% | miR-6736-5p | 61% |
| miR-182-5p | 89% | miR-4529-3p | 61% | miR-6738-3p | 72% |
| miR-185-5p | 78% | miR-4529-5p | 56% | miR-6740-3p | 50% |
| miR-187-3p | 67% | miR-4533 | 72% | miR-6741-5p | 56% |
| miR-1908-3p | 50% | miR-4535 | 67% | miR-6742-3p | 50% |
| miR-1909-5p | 56% | miR-4538 | 72% | miR-6743-3p | 72% |
| miR-1910-3p | 72% | miR-4539 | 67% | miR-6744-3p | 50% |
| miR-1911-5p | 94% | miR-4540 | 72% | miR-6745 | 78% |
| miR-1912 | 72% | miR-454-5p | 78% | miR-6746-5p | 67% |
| miR-193a-5p | 61% | miR-4635 | 67% | miR-6747-5p | 72% |
| miR-193b-3p | 61% | miR-4638-5p | 67% | miR-6749-3p | 56% |
| miR-194-3p | 67% | miR-4639-3p | 72% | miR-6751-5p | 72% |
| miR-195-5p | 89% | miR-4640-3p | 50% | miR-6752-3p | 56% |
| miR-196a-5p | 94% | miR-4643 | 67% | miR-6752-5p | 61% |
| miR-196b-5p | 56% | miR-4644 | 83% | miR-675-5p | 67% |
| miR-1972 | 67% | miR-4646-5p | 78% | miR-6756-3p | 50% |
| miR-197-5p | 50% | miR-4647 | 78% | miR-6759-3p | 61% |
| miR-199b-5p | 50% | miR-4650-3p | 72% | miR-6759-5p | 67% |
| miR-200c-3p | 89% | miR-4650-5p | 67% | miR-6760-3p | 56% |
| miR-203a-3p | 50% | miR-4652-3p | 50% | miR-6760-5p | 78% |
| miR-203a-5p | 61% | miR-4653-3p | 72% | miR-6765-5p | 56% |
| miR-203b-5p | 100% | miR-4654 | 72% | miR-6766-5p | 56% |
| miR-204-5p | 50% | miR-4656 | 56% | miR-6767-5p | 78% |
| miR-2052 | 94% | miR-4657 | 78% | miR-6768-3p | 83% |
| miR-208a-5p | 67% | miR-4659a-3p | 89% | miR-6769a-3p | 61% |
| miR-20a-3p | 72% | miR-466 | 78% | miR-6769a-5p | 56% |
| miR-20b-5p | 83% | miR-4661-5p | 67% | miR-6769b-3p | 56% |
| miR-210-5p | 56% | miR-4665-5p | 56% | miR-6769b-5p | 61% |
| miR-2114-5p | 100% | miR-4667-3p | 56% | miR-6772-5p | 78% |
| miR-212-5p | 72% | miR-4669 | 72% | miR-6773-5p | 100% |
| miR-21-3p | 72% | miR-4676-5p | 78% | miR-6774-5p | 61% |
| miR-215-3p | 100% | miR-4677-5p | 78% | miR-6775-3p | 50% |
| miR-216a-5p | 94% | miR-4681 | 56% | miR-6775-5p | 56% |
| miR-216b-3p | 56% | miR-4683 | 100% | miR-6776-5p | 67% |
| miR-221-3p | 72% | miR-4684-5p | 56% | miR-6777-5p | 72% |
| miR-222-3p | 89% | miR-4685-3p | 56% | miR-6778-5p | 61% |
| miR-223-5p | 61% | miR-4686 | 72% | miR-6779-5p | 50% |
| miR-22-5p | 94% | miR-4687-3p | 72% | miR-6780a-5p | 72% |
| miR-2276-3p | 72% | miR-4687-5p | 56% | miR-6780b-5p | 50% |
| miR-2276-5p | 61% | miR-4690-5p | 61% | miR-6784-3p | 50% |
| miR-2277-3p | 67% | miR-4691-3p | 94% | miR-6786-3p | 50% |
| miR-23a-3p | 83% | miR-4691-5p | 72% | miR-6786-5p | 67% |
| miR-23b-3p | 56% | miR-4692 | 72% | miR-6788-3p | 50% |
| miR-23b-5p | 89% | miR-4694-5p | 83% | miR-6788-5p | 78% |
| miR-2467-3p | 78% | miR-4697-5p | 56% | miR-6790-5p | 56% |
| miR-2681-3p | 100% | miR-4699-3p | 100% | miR-6794-3p | 61% |
| miR-26b-3p | 56% | miR-4700-3p | 56% | miR-6794-5p | 67% |
| miR-27a-3p | 89% | miR-4700-5p | 72% | miR-6795-5p | 72% |
| miR-28-5p | 56% | miR-4701-3p | 72% | miR-6796-5p | 61% |
| miR-2861 | 61% | miR-4701-5p | 56% | miR-6799-5p | 78% |
| miR-296-3p | 61% | miR-4704-5p | 67% | miR-6801-3p | 50% |
| miR-297 | 72% | miR-4706 | 56% | miR-6801-5p | 78% |
| miR-298 | 50% | miR-4707-3p | 56% | miR-6802-5p | 56% |
| miR-299-5p | 67% | miR-4708-3p | 67% | miR-6803-5p | 61% |
| miR-29a-3p | 83% | miR-4709-3p | 72% | miR-6805-5p | 61% |
| miR-29b-1-5p | 78% | miR-4710 | 61% | miR-6807-5p | 50% |
| miR-29b-2-5p | 83% | miR-4711-3p | 83% | miR-6812-3p | 56% |
| miR-302c-5p | 78% | miR-4712-5p | 56% | miR-6812-5p | 67% |
| miR-3064-3p | 78% | miR-4717-3p | 61% | miR-6813-3p | 56% |
| miR-30a-5p | 94% | miR-4719 | 100% | miR-6814-5p | 56% |
| miR-30c-1-3p | 67% | miR-4721 | 78% | miR-6815-5p | 78% |
| miR-30c-5p | 56% | miR-4724-3p | 83% | miR-6816-5p | 67% |
| miR-30d-5p | 72% | miR-4725-5p | 56% | miR-6817-5p | 61% |
| miR-30e-3p | 72% | miR-4726-5p | 67% | miR-6819-5p | 61% |
| miR-3120-5p | 83% | miR-4727-3p | 72% | miR-6820-5p | 56% |
| miR-3121-5p | 100% | miR-4730 | 61% | miR-6821-3p | 61% |
| miR-3122 | 83% | miR-4731-3p | 50% | miR-6823-3p | 61% |
| miR-3124-5p | 78% | miR-4731-5p | 56% | miR-6823-5p | 78% |
| miR-3126-5p | 61% | miR-4732-3p | 67% | miR-6824-3p | 56% |
| miR-3130-5p | 61% | miR-4739 | 78% | miR-6826-5p | 67% |
| miR-3137 | 67% | miR-4743-5p | 78% | miR-6827-5p | 78% |
| miR-3138 | 72% | miR-4746-3p | 61% | miR-6828-5p | 78% |
| miR-3144-3p | 67% | miR-4747-3p | 67% | miR-6830-5p | 72% |
| miR-3145-5p | 83% | miR-4747-5p | 67% | miR-6831-3p | 61% |
| miR-3149 | 67% | miR-4750-3p | 56% | miR-6833-5p | 78% |
| miR-3151-3p | 50% | miR-4752 | 61% | miR-6834-3p | 56% |
| miR-3151-5p | 56% | miR-4754 | 56% | miR-6834-5p | 72% |
| miR-3153 | 72% | miR-4755-5p | 67% | miR-6835-5p | 56% |
| miR-3155a | 61% | miR-4756-3p | 67% | miR-6837-3p | 72% |
| miR-3156-5p | 78% | miR-4758-3p | 56% | miR-6840-5p | 67% |
| miR-3157-5p | 94% | miR-4762-5p | 89% | miR-6841-3p | 50% |
| miR-3158-5p | 50% | miR-4767 | 67% | miR-6847-5p | 56% |
| miR-3160-3p | 78% | miR-4769-5p | 67% | miR-6848-3p | 61% |
| miR-3160-5p | 72% | miR-4774-3p | 100% | miR-6849-5p | 72% |
| miR-3163 | 83% | miR-4776-3p | 67% | miR-6851-5p | 72% |
| miR-3164 | 78% | miR-4776-5p | 83% | miR-6858-5p | 50% |
| miR-3174 | 50% | miR-4781-5p | 100% | miR-6859-5p | 78% |
| miR-3176 | 72% | miR-4784 | 61% | miR-6861-5p | 61% |
| miR-3177-3p | 67% | miR-4785 | 72% | miR-6862-3p | 56% |
| miR-3177-5p | 78% | miR-4787-5p | 50% | miR-6864-5p | 94% |
| miR-3180-5p | 56% | miR-4796-5p | 61% | miR-6867-3p | 61% |
| miR-3181 | 50% | miR-4799-5p | 100% | miR-6867-5p | 56% |
| miR-3182 | 83% | miR-4800-5p | 78% | miR-6868-5p | 61% |
| miR-3184-5p | 50% | miR-4804-5p | 94% | miR-6869-3p | 72% |
| miR-3186-3p | 67% | miR-483-3p | 61% | miR-6869-5p | 56% |
| miR-3187-3p | 67% | miR-484 | 67% | miR-6870-5p | 56% |
| miR-3187-5p | 67% | miR-486-5p | 50% | miR-6871-5p | 83% |
| miR-3189-3p | 67% | miR-487a-5p | 72% | miR-6872-5p | 78% |
| miR-3192-3p | 67% | miR-489-3p | 67% | miR-6876-5p | 83% |
| miR-3192-5p | 50% | miR-491-3p | 83% | miR-6877-5p | 61% |
| miR-3193 | 67% | miR-492 | 56% | miR-6881-5p | 67% |
| miR-3194-5p | 67% | miR-494-5p | 72% | miR-6884-3p | 50% |
| miR-3198 | 50% | miR-5002-3p | 83% | miR-6884-5p | 72% |
| miR-323a-5p | 67% | miR-5002-5p | 94% | miR-6885-5p | 56% |
| miR-326 | 56% | miR-5004-3p | 50% | miR-6887-5p | 78% |
| miR-328-3p | 67% | miR-5006-5p | 72% | miR-6890-3p | 50% |
| miR-328-5p | 56% | miR-500a-5p | 78% | miR-6891-5p | 56% |
| miR-329-3p | 94% | miR-5010-5p | 61% | miR-6892-5p | 72% |
| miR-337-3p | 61% | miR-5011-5p | 100% | miR-6893-5p | 61% |
| miR-337-5p | 83% | miR-501-5p | 56% | miR-6894-5p | 61% |
| miR-339-5p | 56% | miR-504-3p | 67% | miR-708-5p | 72% |
| miR-340-3p | 56% | miR-505-5p | 67% | miR-7106-5p | 72% |
| miR-34a-3p | 100% | miR-5088-3p | 50% | miR-7108-3p | 50% |
| miR-34a-5p | 61% | miR-5089-3p | 56% | miR-7109-3p | 56% |
| miR-34b-5p | 61% | miR-5089-5p | 94% | miR-7109-5p | 67% |
| miR-3529-5p | 61% | miR-5092 | 94% | miR-7110-3p | 56% |
| miR-3591-3p | 89% | miR-5093 | 67% | miR-7112-3p | 50% |
| miR-3591-5p | 78% | miR-509-5p | 72% | miR-7113-3p | 72% |
| miR-3605-5p | 67% | miR-511-5p | 61% | miR-7151-3p | 78% |
| miR-3612 | 72% | miR-512-3p | 56% | miR-7153-5p | 89% |
| miR-3613-3p | 72% | miR-512-5p | 61% | miR-7154-5p | 100% |
| miR-361-3p | 56% | miR-515-3p | 50% | miR-7156-3p | 50% |
| miR-361-5p | 89% | miR-515-5p | 56% | miR-7156-5p | 56% |
| miR-3616-3p | 61% | miR-516b-3p, miR-516a-3p | 67% | miR-7157-5p | 72% |
| miR-3619-3p | 72% | miR-516b-5p | 94% | miR-7158-3p | 100% |
| miR-3622a-5p | 50% | miR-517-5p | 72% | miR-7160-5p | 78% |
| miR-3622b-3p | 56% | miR-517a-3p, miR-517b-3p | 50% | miR-7162-5p | 94% |
| miR-3622b-5p | 56% | miR-5187-3p | 50% | miR-718 | 61% |
| miR-362-5p | 89% | miR-5188 | 50% | miR-7-2-3p | 61% |
| miR-363-3p | 72% | miR-5189-3p | 61% | miR-758-3p | 94% |
| miR-3649 | 50% | miR-5189-5p | 72% | miR-758-5p | 67% |
| miR-3652 | 72% | miR-518b | 72% | miR-766-5p | 72% |
| miR-3653-3p | 100% | miR-5192 | 61% | miR-769-5p | 89% |
| miR-3655 | 67% | miR-5196-3p | 61% | miR-7703 | 67% |
| miR-3659 | 67% | miR-519a-3p | 89% | miR-7843-3p | 78% |
| miR-365a-3p, miR-365b-3p | 50% | miR-519b-3p | 67% | miR-7848-3p | 50% |
| miR-3663-5p | 72% | miR-519c-5p, miR-523-5p, miR-518e-5p, miR-522-5p, miR-519a-5p, miR-519b-5p | 67% | miR-7850-5p | 61% |
| miR-3675-3p | 50% | miR-519d-3p | 67% | miR-7851-3p | 67% |
| miR-3678-3p | 67% | miR-519e-3p | 61% | miR-7853-5p | 61% |
| miR-3681-5p | 100% | miR-520a-3p | 50% | miR-7975 | 72% |
| miR-3689a-3p | 78% | miR-520b | 72% | miR-8055 | 61% |
| miR-3689a-5p, miR-3689b-5p, miR-3689e | 100% | miR-520d-3p | 89% | miR-8058 | 89% |
| miR-3689b-3p, miR-3689c | 67% | miR-520d-5p | 72% | miR-8064 | 67% |
| miR-3691-5p | 83% | miR-520e | 72% | miR-8069 | 50% |
| miR-369-5p | 78% | miR-520f-3p | 94% | miR-8071 | 61% |
| miR-370-3p | 61% | miR-520g-3p | 50% | miR-8074 | 50% |
| miR-370-5p | 61% | miR-521 | 61% | miR-8075 | 67% |
| miR-3713 | 67% | miR-526b-3p | 61% | miR-8077 | 72% |
| miR-371a-3p | 56% | miR-526b-5p | 72% | miR-8079 | 89% |
| miR-371a-5p | 67% | miR-527, miR-518a-5p | 72% | miR-8080 | 50% |
| miR-371b-3p | 61% | miR-539-5p | 89% | miR-8082 | 67% |
| miR-372-3p | 50% | miR-542-5p | 72% | miR-8083 | 67% |
| miR-372-5p | 94% | miR-548ba | 83% | miR-8085 | 72% |
| miR-374b-5p | 83% | miR-551b-5p | 61% | miR-8086 | 89% |
| miR-374c-3p | 67% | miR-552-3p | 83% | miR-8485 | 61% |
| miR-374c-5p | 83% | miR-552-5p | 50% | miR-874-3p | 67% |
| miR-375 | 50% | miR-554 | 89% | miR-877-5p | 72% |
| miR-376c-3p | 89% | miR-5580-5p | 72% | miR-888-5p | 72% |
| miR-378a-5p | 50% | miR-5581-5p | 100% | miR-891a-3p | 94% |
| miR-378b | 61% | miR-5588-3p | 72% | miR-891a-5p | 72% |
| miR-380-3p | 61% | miR-5588-5p | 94% | miR-892c-3p | 78% |
| miR-380-5p | 94% | miR-5589-3p | 89% | miR-921 | 83% |
| miR-383-3p | 50% | miR-5589-5p | 78% | miR-922 | 100% |
| miR-3907 | 100% | miR-563 | 89% | miR-92b-3p | 61% |
| miR-3912-5p | 100% | miR-564 | 83% | miR-93-3p | 78% |
| miR-3917 | 61% | miR-5684 | 89% | miR-93-5p | 50% |
| miR-3919 | 56% | miR-5696 | 89% | miR-936 | 78% |
| miR-3921 | 72% | miR-5698 | 72% | miR-938 | 56% |
| miR-3922-5p | 72% | miR-5701 | 100% | miR-939-3p | 61% |
| miR-3927-5p | 67% | miR-5702 | 72% | miR-96-3p | 83% |
| miR-3928-3p | 61% | miR-5708 | 67% | miR-96-5p | 94% |
| miR-3934-5p | 78% | miR-574-3p | 67% | miR-98-3p | 50% |
| miR-3935 | 78% | miR-574-5p | 61% | miR-99a-3p | 89% |

| | | | | | |
|---|---|---|---|---|---|
| ^{∗} A represents Accuracy. ^{∗} "has-" in the miRNA names has been omitted. | | | | | |

### [Table 23-2]

**Table 23-2: miRNA to Predict Human Breast Cancer of Stages I and II**

| miRNA Name | A | miRNA Name | A | miRNA Name | A |
|---|---|---|---|---|---|
| miR-29a-3p | 59% | miR-3908 | 63% | miR-6722-3p | 72% |
| miR-29b-3p | 64% | miR-3911 | 62% | miR-6724-5p | 79% |
| miR-103a-3p | 53% | miR-3917 | 69% | miR-208a-5p | 61% |
| miR-107 | 54% | miR-3918 | 58% | miR-210-5p | 63% |
| miR-192-5p | 63% | miR-3919 | 65% | miR-128-1-5p | 67% |
| miR-196a-5p | 62% | miR-3150b-3p | 64% | miR-370-5p | 52% |
| miR-197-3p | 70% | miR-3926 | 69% | miR-328-5p | 63% |
| miR-129-5p | 61% | miR-3928-3p | 71% | miR-329-5p | 54% |
| miR-34a-5p | 61% | miR-3935 | 65% | miR-410-5p | 55% |
| miR-187-3p | 65% | miR-3937 | 73% | miR-487a-5p | 54% |
| miR-210-3p | 54% | miR-3941 | 59% | miR-494-5p | 57% |
| miR-211-5p | 57% | miR-3944-3p | 68% | miR-181d-3p | 80% |
| miR-214-3p | 64% | miR-3945 | 59% | miR-504-3p | 70% |
| miR-124-3p | 75% | miR-642b-3p | 63% | miR-487b-5p | 70% |
| miR-125b-5p | 52% | miR-550b-3p | 64% | miR-585-5p | 68% |
| miR-138-5p | 54% | miR-1268b | 79% | miR-598-5p | 67% |
| miR-149-5p | 67% | miR-378f | 55% | miR-605-3p | 53% |
| miR-184 | 62% | miR-4421 | 54% | miR-619-5p | 51% |
| miR-188-5p | 65% | miR-4428 | 75% | miR-655-5p | 54% |
| miR-320a-3p | 66% | miR-4429 | 63% | miR-1296-3p | 55% |
| miR-106b-5p | 83% | miR-4430 | 74% | miR-1301-5p | 53% |
| miR-29c-3p | 100% | miR-4433a-3p | 70% | miR-874-5p | 62% |
| miR-299-3p | 56% | miR-4434 | 100% | miR-1250-3p | 68% |
| miR-361-5p | 60% | miR-4435 | 59% | miR-1266-3p | 56% |
| miR-370-3p | 72% | miR-4436a | 67% | miR-1908-3p | 65% |
| miR-373-5p | 64% | miR-4437 | 62% | miR-3151-3p | 69% |
| miR-373-3p | 60% | miR-4439 | 65% | miR-500b-3p | 54% |
| miR-378a-3p | 51% | miR-4440 | 74% | miR-1343-5p | 75% |
| miR-379-5p | 80% | miR-4443 | 74% | miR-5189-3p | 68% |
| miR-383-5p | 60% | miR-4446-3p | 66% | miR-6726-5p | 76% |
| miR-328-3p | 72% | miR-4447 | 68% | miR-6726-3p | 66% |
| miR-342-3p | 63% | miR-4448 | 61% | miR-6727-5p | 64% |
| miR-326 | 73% | miR-4449 | 68% | miR-6727-3p | 70% |
| miR-151a-3p | 57% | miR-4455 | 63% | miR-6728-5p | 51% |
| miR-325 | 55% | miR-4456 | 68% | miR-6728-3p | 65% |
| miR-346 | 73% | miR-4458 | 65% | miR-6729-5p | 75% |
| miR-424-5p | 53% | miR-4460 | 90% | miR-6730-5p | 63% |
| miR-20b-5p | 71% | miR-378h | 64% | miR-6730-3p | 65% |
| miR-200a-5p | 54% | miR-3135b | 66% | miR-6732-5p | 77% |
| miR-323b-5p | 63% | miR-4462 | 66% | miR-6732-3p | 69% |
| miR-485-5p | 52% | miR-4463 | 67% | miR-6734-5p | 66% |
| miR-486-5p | 64% | miR-4466 | 67% | miR-6735-5p | 66% |
| miR-488-5p | 53% | miR-4467 | 79% | miR-6735-3p | 63% |
| miR-491-5p | 73% | miR-4472 | 64% | miR-6736-5p | 67% |
| miR-202-3p | 59% | miR-4474-3p | 58% | miR-6736-3p | 68% |
| miR-492 | 64% | miR-4476 | 64% | miR-6737-5p | 68% |
| miR-512-5p | 52% | miR-4478 | 60% | miR-6738-5p | 67% |
| miR-498-5p | 76% | miR-4479 | 51% | miR-6738-3p | 66% |
| miR-520c-3p | 78% | miR-4481 | 73% | miR-6739-3p | 52% |
| miR-519d-3p | 53% | miR-4483 | 72% | miR-6740-5p | 60% |
| miR-516b-3p, miR-516a-3p | 51% | miR-4484 | 67% | miR-6740-3p | 62% |
| miR-519a-3p | 82% | miR-4486 | 77% | miR-6741-5p | 67% |
| miR-502-5p | 55% | miR-4487 | 70% | miR-6741-3p | 70% |
| miR-493-3p | 51% | miR-4488 | 66% | miR-6742-5p | 63% |
| miR-551a | 66% | miR-4489 | 71% | miR-6743-5p | 73% |
| miR-552-3p | 62% | miR-4492 | 66% | miR-6743-3p | 71% |
| miR-554 | 60% | miR-4497 | 76% | miR-6744-3p | 61% |
| miR-555 | 53% | miR-4498 | 70% | miR-6747-5p | 64% |
| miR-557 | 62% | miR-4505 | 70% | miR-6747-3p | 68% |
| miR-564 | 72% | miR-4506 | 65% | miR-6748-5p | 58% |
| miR-572 | 72% | miR-2392 | 71% | miR-6748-3p | 67% |
| miR-574-3p | 66% | miR-4507 | 75% | miR-6749-5p | 68% |
| miR-575 | 75% | miR-4508 | 71% | miR-6749-3p | 65% |
| miR-576-5p | 52% | miR-4510 | 54% | miR-6750-3p | 70% |
| miR-578 | 70% | miR-4511 | 57% | miR-6751-5p | 75% |
| miR-579-3p | 67% | miR-4512 | 82% | miR-6751-3p | 70% |
| miR-584-5p | 58% | miR-4513 | 62% | miR-6752-5p | 70% |
| miR-588 | 67% | miR-4516 | 63% | miR-6753-5p | 77% |
| miR-595 | 72% | miR-4519 | 100% | miR-6753-3p | 69% |
| miR-608 | 64% | miR-4520-3p | 56% | miR-6754-5p | 59% |
| miR-610 | 55% | miR-4521 | 67% | miR-6754-3p | 69% |
| miR-612 | 74% | miR-1269b | 67% | miR-6756-5p | 69% |
| miR-614 | 53% | miR-4522 | 63% | miR-6756-3p | 65% |
| miR-615-3p | 64% | miR-4523 | 55% | miR-6759-5p | 72% |
| miR-616-5p | 67% | miR-4524a-3p | 71% | miR-6759-3p | 67% |
| miR-617 | 59% | miR-4525 | 57% | miR-6761-3p | 73% |
| miR-628-3p | 56% | miR-4526 | 70% | miR-6762-5p | 71% |
| miR-629-3p | 51% | miR-4530 | 79% | miR-6762-3p | 62% |
| miR-632 | 56% | miR-4533 | 79% | miR-6763-5p | 72% |
| miR-636 | 60% | miR-4535 | 79% | miR-6763-3p | 60% |
| miR-637 | 69% | miR-1587 | 68% | miR-6765-5p | 69% |
| miR-638 | 72% | miR-4538 | 70% | miR-6766-5p | 75% |
| miR-639 | 89% | miR-4539 | 76% | miR-6767-5p | 62% |
| miR-663a | 74% | miR-4540 | 56% | miR-6768-5p | 78% |
| miR-449b-5p | 55% | miR-3120-5p | 55% | miR-6771-5p | 70% |
| miR-657 | 63% | miR-3156-3p | 62% | miR-6772-5p | 70% |
| miR-658 | 74% | miR-3158-5p | 67% | miR-6772-3p | 70% |
| miR-542-5p | 86% | miR-3173-5p | 68% | miR-6773-3p | 66% |
| miR-671-5p | 61% | miR-3187-5p | 64% | miR-6775-5p | 65% |
| miR-668-3p | 70% | miR-3922-5p | 70% | miR-6775-3p | 63% |
| miR-769-5p | 52% | miR-3925-3p | 52% | miR-6776-5p | 70% |
| miR-769-3p | 64% | miR-3940-5p | 66% | miR-6776-3p | 63% |
| miR-766-3p | 69% | miR-4520-5p | 56% | miR-6777-5p | 70% |
| miR-765 | 61% | miR-3960 | 63% | miR-6777-3p | 60% |
| miR-675-5p | 63% | miR-3973 | 52% | miR-6778-5p | 71% |
| miR-297 | 56% | miR-4634 | 68% | miR-6778-3p | 66% |
| let-7d-3p | 62% | miR-4638-5p | 79% | miR-6779-3p | 67% |
| miR-24-2-5p | 53% | miR-4639-3p | 63% | miR-6780a-3p | 62% |
| miR-25-5p | 63% | miR-4640-5p | 72% | miR-6781-5p | 74% |
| miR-26b-3p | 61% | miR-4643 | 53% | miR-6782-5p | 75% |
| miR-29a-5p | 100% | miR-4644 | 63% | miR-6783-5p | 57% |
| miR-92a-2-5p | 64% | miR-4646-5p | 67% | miR-6783-3p | 66% |
| miR-29b-1-5p | 63% | miR-4646-3p | 67% | miR-6784-5p | 69% |
| miR-139-3p | 63% | miR-4647 | 62% | miR-6786-5p | 70% |
| miR-187-5p | 67% | miR-4648 | 58% | miR-6786-3p | 64% |
| miR-214-5p | 57% | miR-4649-5p | 63% | miR-6787-5p | 76% |
| miR-30b-3p | 64% | miR-4650-3p | 71% | miR-6787-3p | 64% |
| miR-124-5p | 82% | miR-4651 | 75% | miR-6788-5p | 56% |
| miR-125b-1-3p | 100% | miR-4653-3p | 58% | miR-6788-3p | 68% |
| miR-130a-5p | 60% | miR-4654 | 69% | miR-6789-5p | 76% |
| miR-132-5p | 80% | miR-4655-5p | 74% | miR-6789-3p | 70% |
| miR-135a-3p | 60% | miR-4656 | 62% | miR-6790-5p | 75% |
| miR-140-3p | 64% | miR-4661-5p | 64% | miR-6790-3p | 63% |
| miR-125a-3p | 60% | miR-4664-5p | 71% | miR-6791-5p | 73% |
| miR-138-1-3p | 65% | miR-4665-5p | 74% | miR-6792-5p | 72% |
| miR-149-3p | 68% | miR-4665-3p | 64% | miR-6792-3p | 64% |
| miR-150-3p | 66% | miR-4667-5p | 65% | miR-6793-5p | 65% |
| miR-185-3p | 72% | miR-4669 | 64% | miR-6794-5p | 61% |
| miR-193a-5p | 74% | miR-4670-5p | 55% | miR-6794-3p | 65% |
| miR-194-3p | 68% | miR-4672 | 54% | miR-6796-5p | 75% |
| miR-30c-1-3p | 57% | miR-4673 | 64% | miR-6797-5p | 61% |
| miR-34b-3p | 51% | miR-4674 | 60% | miR-6797-3p | 60% |
| miR-34c-3p | 64% | miR-4676-5p | 67% | miR-6798-5p | 77% |
| miR-99b-3p | 67% | miR-4682 | 58% | miR-6799-5p | 69% |
| miR-296-3p | 70% | miR-4685-5p | 65% | miR-6800-5p | 68% |
| miR-323a-5p | 68% | miR-4685-3p | 66% | miR-6800-3p | 62% |
| miR-423-5p | 68% | miR-4686 | 57% | miR-6801-5p | 62% |
| miR-20b-3p | 70% | miR-4687-5p | 69% | miR-6803-5p | 62% |
| miR-483-5p | 70% | miR-1343-3p | 78% | miR-6803-3p | 69% |
| miR-486-3p | 66% | miR-4688 | 68% | miR-6804-3p | 71% |
| miR-193b-5p | 73% | miR-4690-5p | 78% | miR-6805-5p | 70% |
| miR-508-5p | 58% | miR-4691-5p | 71% | miR-6805-3p | 62% |
| miR-532-3p | 66% | miR-4691-3p | 88% | miR-6806-5p | 66% |
| miR-92b-5p | 74% | miR-4692 | 58% | miR-6807-5p | 64% |
| miR-551b-5p | 58% | miR-4694-5p | 53% | miR-6808-5p | 62% |
| miR-574-5p | 72% | miR-4695-5p | 76% | miR-6808-3p | 58% |
| miR-550a-5p | 70% | miR-4695-3p | 70% | miR-6809-5p | 71% |
| miR-615-5p | 68% | miR-4697-5p | 63% | miR-6809-3p | 65% |
| miR-616-3p | 53% | miR-4700-5p | 67% | miR-6810-5p | 67% |
| miR-624-3p | 71% | miR-4701-3p | 62% | miR-6812-5p | 69% |
| miR-629-5p | 80% | miR-4703-3p | 52% | miR-6813-5p | 69% |
| miR-671-3p | 52% | miR-4704-5p | 53% | miR-6813-3p | 60% |
| miR-891a-5p | 52% | miR-4706 | 69% | miR-6815-3p | 67% |
| miR-874-3p | 73% | miR-4707-5p | 74% | miR-6816-5p | 69% |
| miR-891b | 80% | miR-4707-3p | 64% | miR-6818-5p | 75% |
| miR-892b | 63% | miR-4708-5p | 59% | miR-6820-5p | 72% |
| miR-744-5p | 71% | miR-4708-3p | 77% | miR-6821-5p | 73% |
| miR-885-5p | 67% | miR-203b-5p | 56% | miR-6822-5p | 62% |
| miR-885-3p | 78% | miR-4710 | 70% | miR-6822-3p | 53% |
| miR-877-5p | 74% | miR-4711-3p | 61% | miR-6823-5p | 63% |
| miR-877-3p | 71% | miR-4712-3p | 58% | miR-6823-3p | 67% |
| miR-887-3p | 75% | miR-4713-5p | 65% | miR-6824-5p | 64% |
| miR-665 | 65% | miR-4717-3p | 57% | miR-6825-5p | 69% |
| miR-760 | 74% | miR-4720-5p | 61% | miR-6825-3p | 68% |
| miR-301b-3p | 100% | miR-4721 | 73% | miR-6827-5p | 59% |
| miR-920 | 70% | miR-4722-5p | 67% | miR-6829-5p | 72% |
| miR-924 | 52% | miR-4722-3p | 68% | miR-6829-3p | 67% |
| miR-509-3-5p | 100% | miR-4723-3p | 65% | miR-6830-5p | 60% |
| miR-936 | 64% | miR-4724-5p | 59% | miR-6830-3p | 65% |
| miR-939-5p | 68% | miR-4725-5p | 62% | miR-6831-5p | 68% |
| miR-943 | 59% | miR-4725-3p | 72% | miR-6832-5p | 67% |
| miR-1224-5p | 76% | miR-4726-3p | 69% | miR-6833-5p | 71% |
| miR-1224-3p | 63% | miR-4728-3p | 64% | miR-6833-3p | 69% |
| miR-1225-5p | 67% | miR-4730 | 66% | miR-6834-5p | 79% |
| miR-1225-3p | 63% | miR-4732-5p | 71% | miR-6780b-5p | 70% |
| miR-1228-5p | 69% | miR-4732-3p | 66% | miR-6780b-3p | 70% |
| miR-1228-3p | 64% | miR-4734 | 68% | miR-6836-3p | 64% |
| miR-1229-3p | 65% | miR-4736 | 63% | miR-6837-5p | 62% |
| miR-1231 | 68% | miR-3064-5p | 65% | miR-6838-5p | 59% |
| miR-1233-3p | 67% | miR-3064-3p | 69% | miR-6839-5p | 59% |
| miR-1236-3p | 65% | miR-4738-3p | 64% | miR-6840-3p | 71% |
| miR-1237-3p | 64% | miR-4739 | 63% | miR-6842-5p | 70% |
| miR-1238-3p | 63% | miR-4740-3p | 53% | miR-6845-5p | 76% |
| miR-320c | 67% | miR-4741 | 73% | miR-6845-3p | 68% |
| miR-1182 | 68% | miR-4743-5p | 70% | miR-6846-5p | 69% |
| miR-1202 | 63% | miR-122b-3p | 57% | miR-6847-5p | 59% |
| miR-1203 | 59% | miR-4745-5p | 73% | miR-6847-3p | 69% |
| miR-1204 | 54% | miR-4746-3p | 74% | miR-6848-5p | 64% |
| miR-1207-5p | 68% | miR-4747-5p | 65% | miR-6849-5p | 67% |
| miR-1207-3p | 58% | miR-4747-3p | 51% | miR-6849-3p | 69% |
| miR-1286 | 55% | miR-4748 | 60% | miR-6850-5p | 70% |
| miR-1293 | 68% | miR-4749-5p | 74% | miR-6851-5p | 62% |
| miR-1294 | 68% | miR-4750-5p | 71% | miR-6852-3p | 59% |
| miR-1303 | 61% | miR-4753-5p | 62% | miR-6855-5p | 65% |
| miR-1304-5p | 65% | miR-371b-5p | 73% | miR-6856-5p | 60% |
| miR-1250-5p | 52% | miR-4754 | 63% | miR-6857-5p | 67% |
| miR-1257 | 52% | miR-4755-3p | 55% | miR-6858-5p | 58% |
| miR-1260a | 71% | miR-4758-5p | 75% | miR-6858-3p | 61% |
| miR-1263 | 57% | miR-4758-3p | 64% | miR-6859-5p | 63% |
| miR-1266-5p | 62% | miR-4759 | 100% | miR-6769b-5p | 70% |
| miR-1268a | 70% | miR-4761-5p | 60% | miR-6860 | 76% |
| miR-1281 | 69% | miR-4763-5p | 67% | miR-6861-5p | 74% |
| miR-1292-5p | 88% | miR-4763-3p | 75% | miR-6861-3p | 63% |
| miR-1306-3p | 66% | miR-4766-5p | 70% | miR-6862-3p | 69% |
| miR-1307-3p | 75% | miR-4767 | 58% | miR-6865-3p | 70% |
| miR-320d | 83% | miR-4769-5p | 66% | miR-6867-5p | 61% |
| miR-1825 | 73% | miR-4769-3p | 62% | miR-6867-3p | 67% |
| miR-1468-5p | 63% | miR-4772-5p | 100% | miR-6868-3p | 51% |
| miR-675-3p | 71% | miR-4776-5p | 74% | miR-6869-5p | 68% |
| miR-1469 | 73% | miR-4776-3p | 59% | miR-6870-5p | 68% |
| miR-1470 | 67% | miR-4780 | 66% | miR-6871-5p | 74% |
| miR-1471 | 72% | miR-4436b-5p | 73% | miR-6872-3p | 70% |
| miR-1538 | 63% | miR-4436b-3p | 53% | miR-6875-5p | 63% |
| miR-1539 | 68% | miR-4781-5p | 100% | miR-6875-3p | 68% |
| miR-1908-5p | 76% | miR-4783-3p | 68% | miR-6876-5p | 71% |
| miR-1909-5p | 71% | miR-4784 | 67% | miR-6876-3p | 65% |
| miR-1909-3p | 74% | miR-2467-3p | 77% | miR-6877-5p | 76% |
| miR-1910-5p | 72% | miR-4787-5p | 65% | miR-6877-3p | 70% |
| miR-1912-3p | 72% | miR-4787-3p | 71% | miR-6879-5p | 71% |
| miR-1914-3p | 66% | miR-4793-3p | 60% | miR-6879-3p | 70% |
| miR-1915-3p | 69% | miR-4800-5p | 67% | miR-6880-5p | 66% |
| miR-365a-5p | 69% | miR-642a-3p | 65% | miR-6880-3p | 63% |
| miR-449b-3p | 68% | miR-550a-3-5p | 52% | miR-6881-5p | 65% |
| miR-2113 | 58% | miR-4482-3p | 68% | miR-6882-3p | 66% |
| miR-1976 | 70% | miR-4999-5p | 57% | miR-6883-3p | 65% |
| miR-2110 | 74% | miR-5000-3p | 52% | miR-6884-5p | 52% |
| miR-762 | 69% | miR-5001-5p | 77% | miR-6886-3p | 68% |
| miR-670-5p | 55% | miR-5001-3p | 52% | miR-6887-3p | 65% |
| miR-548q | 69% | miR-5002-3p | 68% | miR-6889-5p | 70% |
| miR-2276-3p | 70% | miR-5003-3p | 63% | miR-6890-5p | 69% |
| miR-711 | 63% | miR-5006-5p | 62% | miR-6891-5p | 64% |
| miR-718 | 70% | miR-5008-5p | 59% | miR-6891-3p | 59% |
| miR-2681-3p | 56% | miR-5010-5p | 74% | miR-6892-5p | 59% |
| miR-2682-3p | 60% | miR-5087 | 56% | miR-6892-3p | 62% |
| miR-449c-3p | 66% | miR-5088-5p | 64% | miR-6893-5p | 67% |
| miR-2861 | 63% | miR-5089-5p | 64% | miR-6893-3p | 70% |
| miR-3120-3p | 63% | miR-5090 | 70% | miR-6894-5p | 70% |
| miR-3122 | 54% | miR-5189-5p | 79% | miR-6894-3p | 70% |
| miR-3130-5p | 68% | miR-5190 | 53% | miR-6895-5p | 63% |
| miR-466 | 64% | miR-5196-5p | 67% | miR-6895-3p | 56% |
| miR-3136-5p | 100% | miR-4524b-5p | 57% | miR-7106-5p | 63% |
| miR-3137 | 56% | miR-5100 | 71% | miR-7106-3p | 64% |
| miR-3138 | 63% | miR-5572 | 74% | miR-7107-5p | 71% |
| miR-3141 | 72% | miR-664b-5p | 56% | miR-7108-5p | 77% |
| miR-1273c | 78% | miR-664b-3p | 71% | miR-7108-3p | 64% |
| miR-3147 | 61% | miR-548at-5p | 56% | miR-7109-5p | 63% |
| miR-3148 | 56% | miR-5585-3p | 67% | miR-7109-3p | 70% |
| miR-3151-5p | 70% | miR-548au-3p | 53% | miR-7110-5p | 70% |
| miR-3153 | 73% | miR-5588-5p | 60% | miR-7110-3p | 69% |
| miR-3154 | 65% | miR-5588-3p | 58% | miR-7111-5p | 70% |
| miR-3157-5p | 58% | miR-5589-5p | 68% | miR-7111-3p | 68% |
| miR-3161 | 56% | miR-5685 | 60% | miR-7112-5p | 70% |
| miR-3162-5p | 80% | miR-5690 | 60% | miR-7113-5p | 62% |
| miR-3163 | 75% | miR-5698 | 73% | miR-7113-3p | 73% |
| miR-3165 | 60% | miR-5699-3p | 63% | miR-7114-5p | 71% |
| miR-1260b | 74% | miR-5703 | 65% | miR-7150 | 70% |
| miR-3168 | 70% | miR-5705 | 60% | miR-7151-3p | 72% |
| miR-3173-3p | 61% | miR-5706 | 100% | miR-7152-5p | 67% |
| miR-1193 | 77% | miR-5708 | 68% | miR-7155-5p | 64% |
| miR-3175 | 64% | miR-197-5p | 70% | miR-7157-3p | 67% |
| miR-3176 | 64% | miR-204-3p | 69% | miR-7158-3p | 56% |
| miR-3177-3p | 58% | miR-211-3p | 68% | miR-7159-5p | 62% |
| miR-3178 | 65% | miR-301a-5p | 57% | miR-7160-5p | 72% |
| miR-3179 | 60% | miR-382-3p | 54% | miR-7161-5p | 56% |
| miR-3180-5p | 63% | miR-584-3p | 60% | miR-7162-5p | 59% |
| miR-3180-3p | 69% | miR-652-5p | 64% | miR-7515 | 65% |
| miR-3185 | 76% | miR-1185-2-3p | 65% | miR-7704 | 62% |
| miR-3187-3p | 61% | miR-766-5p | 52% | miR-7843-5p | 65% |
| miR-3191-3p | 70% | miR-1304-3p | 64% | miR-4433b-3p | 70% |
| miR-3194-5p | 68% | miR-1247-3p | 72% | miR-1273h-5p | 58% |
| miR-3195 | 71% | miR-1306-5p | 64% | miR-6516-5p | 52% |
| miR-3196 | 67% | miR-3184-3p | 64% | miR-7845-5p | 65% |
| miR-3197 | 73% | miR-3191-5p | 70% | miR-7846-3p | 68% |
| miR-3198 | 61% | miR-642b-5p | 64% | miR-7847-3p | 63% |
| miR-3126-3p | 52% | miR-3529-3p | 80% | miR-7850-5p | 62% |
| miR-4296 | 59% | miR-365b-5p | 69% | miR-7851-3p | 65% |
| miR-4297 | 65% | miR-3190-3p | 58% | miR-7854-3p | 69% |
| miR-4293 | 67% | miR-381-5p | 55% | miR-8052 | 69% |
| miR-4294 | 69% | miR-503-3p | 52% | miR-8059 | 74% |
| miR-4298 | 63% | miR-758-5p | 53% | miR-8062 | 52% |
| miR-4305 | 61% | miR-937-5p | 73% | miR-8063 | 71% |
| miR-4315 | 55% | miR-939-3p | 67% | miR-8064 | 76% |
| miR-4316 | 67% | miR-1227-5p | 76% | miR-8069 | 66% |
| miR-4314 | 56% | miR-1229-5p | 61% | miR-8071 | 82% |
| miR-4320 | 54% | miR-1236-5p | 63% | miR-8072 | 69% |
| miR-4322 | 67% | miR-1237-5p | 71% | miR-8073 | 75% |
| miR-4321 | 57% | miR-1238-5p | 72% | miR-8074 | 52% |
| miR-4323 | 61% | miR-3617-3p | 61% | miR-8075 | 67% |
| miR-4324 | 68% | miR-3620-5p | 70% | miR-8077 | 79% |
| miR-4256 | 69% | miR-3927-5p | 51% | miR-8080 | 57% |
| miR-4257 | 73% | miR-3934-3p | 52% | miR-8085 | 70% |
| miR-4258 | 71% | miR-4632-5p | 70% | miR-8089 | 73% |
| miR-4259 | 61% | miR-4743-3p | 62% | miR-7976 | 68% |
| miR-4260 | 70% | miR-5739 | 63% | miR-7977 | 76% |
| miR-4253 | 59% | miR-5787 | 63% | miR-203a-5p | 55% |
| miR-4252 | 56% | miR-6068 | 58% | miR-1249-5p | 65% |
| miR-4325 | 67% | miR-6069 | 62% | miR-4485-5p | 74% |
| miR-4326 | 65% | miR-6071 | 67% | miR-8485 | 65% |
| miR-4327 | 66% | miR-6072 | 72% | miR-103a-1-5p | 54% |
| miR-4265 | 65% | miR-6073 | 52% | miR-196a-1-3p | 61% |
| miR-4269 | 72% | miR-6075 | 70% | miR-217-3p | 57% |
| miR-4271 | 64% | miR-6076 | 78% | miR-375-5p | 71% |
| miR-4276 | 62% | miR-6081 | 54% | miR-498-3p | 61% |
| miR-4279 | 67% | miR-6083 | 65% | miR-1912-5p | 67% |
| miR-4278 | 54% | miR-6085 | 63% | miR-9718 | 83% |
| miR-4280 | 55% | miR-6088 | 61% | miR-1843 | 62% |
| miR-4285 | 76% | miR-6089 | 67% | miR-10392-5p | 73% |
| miR-4283 | 51% | miR-6090 | 68% | miR-10392-3p | 78% |
| miR-4289 | 59% | miR-6124 | 72% | miR-10394-3p | 67% |
| miR-500b-5p | 53% | miR-6125 | 70% | miR-10395-3p | 64% |
| miR-2277-5p | 59% | miR-6126 | 72% | miR-10396a-5p | 71% |
| miR-3200-5p | 61% | miR-6127 | 76% | miR-10396a-3p | 73% |
| miR-3605-3p | 67% | miR-378j | 63% | miR-10398-5p | 67% |
| miR-3610 | 62% | miR-6131 | 76% | miR-10398-3p | 71% |
| miR-3616-3p | 74% | miR-6132 | 68% | miR-10400-5p | 64% |
| miR-3619-5p | 56% | miR-6133 | 67% | miR-10400-3p | 74% |
| miR-3620-3p | 68% | miR-6500-3p | 55% | miR-10401-5p | 80% |
| miR-3621 | 68% | miR-6501-5p | 60% | miR-10401-3p | 72% |
| miR-3622a-5p | 72% | miR-6501-3p | 59% | miR-10396b-5p | 74% |
| miR-3622a-3p | 66% | miR-6506-5p | 52% | miR-10396b-3p | 66% |
| miR-3622b-5p | 65% | miR-6509-3p | 70% | miR-10527-5p | 77% |
| miR-3646 | 65% | miR-6510-5p | 67% | miR-11181-5p | 65% |
| miR-3648 | 77% | miR-6511a-5p | 74% | miR-11181-3p | 68% |
| miR-3649 | 57% | miR-6511a-3p | 70% | miR-11399 | 74% |
| miR-3650 | 58% | miR-6512-3p | 55% | miR-11400 | 64% |
| miR-3652 | 75% | miR-6513-5p | 62% | miR-11401 | 63% |
| miR-3660 | 56% | miR-6514-3p | 63% | miR-3085-5p | 64% |
| miR-3663-5p | 70% | miR-6515-5p | 56% | miR-3085-3p | 61% |
| miR-3663-3p | 72% | miR-6515-3p | 62% | miR-6529-5p | 67% |
| miR-3665 | 63% | miR-6715b-3p | 70% | miR-12114 | 70% |
| miR-3677-3p | 75% | miR-6716-5p | 62% | miR-12115 | 62% |
| miR-3679-5p | 70% | miR-6716-3p | 64% | miR-12118 | 71% |
| miR-3685 | 51% | miR-6717-5p | 62% | miR-12119 | 74% |
| miR-3689a-3p | 64% | miR-6511b-5p | 66% | miR-12120 | 75% |
| miR-3691-5p | 55% | miR-6511b-3p | 67% | miR-12121 | 78% |
| miR-3180 | 67% | miR-6719-3p | 58% | miR-12124 | 53% |
| miR-3907 | 83% | miR-6721-5p | 72% | miR-12126 | 51% |
| miR-3689b-3p, miR-3689c | 59% | miR-6722-5p | 68% | miR-12128 | 59% |

| | | | | | |
|---|---|---|---|---|---|
| ∗ A represents Accuracy. ∗ "has-" in the miRNA names has been omitted. | | | | | |

### [Table 23-3]

**Table 23-3: miRNA to Predict Human Kidney cancer of Stages I and II**

| miRNA Name | A | miRNA Name | A | miRNA Name | A |
|---|---|---|---|---|---|
| miR-29a-3p | 67% | miR-3649 | 72% | miR-1908-3p | 70% |
| miR-30a-5p | 57% | miR-3652 | 73% | miR-3151-3p | 69% |
| miR-29b-3p | 55% | miR-3660 | 63% | miR-3192-3p | 54% |
| miR-196a-5p | 75% | miR-3663-5p | 67% | miR-1343-5p | 75% |
| miR-197-3p | 71% | miR-3663-3p | 63% | miR-5088-3p | 65% |
| miR-198 | 68% | miR-3665 | 69% | miR-5189-3p | 53% |
| miR-199a-5p | 68% | miR-3667-5p | 54% | miR-5699-5p | 72% |
| miR-30d-5p | 75% | miR-3667-3p | 74% | miR-6726-5p | 77% |
| miR-10a-5p | 58% | miR-3675-3p | 76% | miR-6726-3p | 66% |
| miR-187-3p | 52% | miR-3677-3p | 70% | miR-6727-5p | 68% |
| miR-199b-5p | 75% | miR-3679-5p | 82% | miR-6727-3p | 70% |
| miR-214-3p | 55% | miR-3679-3p | 77% | miR-6728-5p | 63% |
| miR-223-3p | 72% | miR-3689a-3p | 58% | miR-6728-3p | 75% |
| miR-133a-3p | 72% | miR-3691-5p | 54% | miR-6729-5p | 70% |
| miR-125a-5p | 52% | miR-3714 | 66% | miR-6729-3p | 76% |
| miR-134-5p | 64% | miR-3180 | 64% | miR-6730-5p | 73% |
| miR-149-5p | 63% | miR-3907 | 75% | miR-6730-3p | 70% |
| miR-184 | 53% | miR-3689b-3p, miR-3689c | 55% | miR-6731-5p | 66% |
| miR-185-5p | 63% | miR-3909 | 65% | miR-6731-3p | 73% |
| miR-206 | 54% | miR-3911 | 66% | miR-6732-5p | 72% |
| miR-200c-3p | 100% | miR-3917 | 64% | miR-6732-3p | 76% |
| miR-106b-5p | 83% | miR-3919 | 61% | miR-6734-5p | 74% |
| miR-29c-3p | 100% | miR-3150b-3p | 52% | miR-6734-3p | 67% |
| miR-302a-3p | 100% | miR-3925-5p | 76% | miR-6735-5p | 62% |
| miR-299-3p | 59% | miR-3928-3p | 77% | miR-6735-3p | 64% |
| miR-296-5p | 74% | miR-3935 | 57% | miR-6736-5p | 59% |
| miR-130b-3p | 89% | miR-3936 | 54% | miR-6736-3p | 66% |
| miR-361-5p | 53% | miR-3937 | 76% | miR-6737-5p | 68% |
| miR-365a-3p, miR-365b-3p | 71% | miR-3941 | 53% | miR-6737-3p | 75% |
| miR-302c-5p | 67% | miR-3942-5p | 56% | miR-6738-5p | 73% |
| miR-370-3p | 80% | miR-3943 | 73% | miR-6738-3p | 59% |
| miR-373-5p | 70% | miR-3944-3p | 65% | miR-6740-5p | 58% |
| miR-373-3p | 62% | miR-3945 | 59% | miR-6740-3p | 70% |
| miR-378a-3p | 58% | miR-642b-3p | 66% | miR-6741-5p | 83% |
| miR-379-5p | 75% | miR-550b-3p | 52% | miR-6741-3p | 71% |
| miR-380-3p | 63% | miR-1268b | 75% | miR-6742-5p | 81% |
| miR-328-3p | 74% | miR-548ab | 67% | miR-6742-3p | 73% |
| miR-342-3p | 58% | miR-4421 | 66% | miR-6743-5p | 70% |
| miR-337-3p | 52% | miR-4428 | 79% | miR-6743-3p | 69% |
| miR-326 | 70% | miR-4429 | 79% | miR-6744-5p | 68% |
| miR-133b | 70% | miR-4430 | 78% | miR-6744-3p | 64% |
| miR-325 | 52% | miR-4433a-3p | 77% | miR-6745 | 70% |
| miR-346 | 63% | miR-4439 | 64% | miR-6746-5p | 86% |
| miR-20b-5p | 71% | miR-4440 | 70% | miR-6747-5p | 71% |
| miR-448 | 57% | miR-4441 | 71% | miR-6747-3p | 65% |
| miR-429 | 100% | miR-4443 | 59% | miR-6748-5p | 83% |
| miR-449a | 60% | miR-4444 | 65% | miR-6748-3p | 69% |
| miR-191-3p | 74% | miR-4446-3p | 67% | miR-6749-5p | 80% |
| miR-200a-5p | 54% | miR-4447 | 82% | miR-6749-3p | 76% |
| miR-409-3p | 70% | miR-4448 | 64% | miR-6750-5p | 56% |
| miR-483-3p | 67% | miR-4449 | 65% | miR-6750-3p | 68% |
| miR-484 | 71% | miR-4455 | 63% | miR-6751-5p | 75% |
| miR-485-3p | 67% | miR-4456 | 62% | miR-6752-5p | 71% |
| miR-486-5p | 75% | miR-4458 | 63% | miR-6752-3p | 72% |
| miR-491-5p | 84% | miR-4460 | 75% | miR-6753-5p | 65% |
| miR-202-3p | 61% | miR-378h | 53% | miR-6753-3p | 63% |
| miR-492 | 58% | miR-3135b | 67% | miR-6754-5p | 71% |
| miR-494-3p | 52% | miR-4462 | 66% | miR-6754-3p | 61% |
| miR-512-5p | 53% | miR-4463 | 67% | miR-6755-5p | 71% |
| miR-498-5p | 84% | miR-4465 | 54% | miR-6756-5p | 64% |
| miR-520e-3p | 63% | miR-4466 | 68% | miR-6756-3p | 77% |
| miR-519c-5p, miR-523-5p, miR-518e-5p, miR-522-5p, miR-519a-5p, miR-519b-5p | 57% | miR-4467 | 70% | miR-6757-5p | 71% |
| miR-526b-5p | 55% | miR-4468 | 67% | miR-6757-3p | 74% |
| miR-525-5p | 51% | miR-4470 | 88% | miR-6758-5p | 78% |
| miR-518f-3p | 51% | miR-4472 | 80% | miR-6759-5p | 72% |
| miR-520b-3p | 56% | miR-4474-3p | 57% | miR-6759-3p | 73% |
| miR-518b | 73% | miR-4475 | 67% | miR-6760-5p | 75% |
| miR-526a-5p, miR-520c-5p, miR-518d-5p | 52% | miR-4476 | 72% | miR-6760-3p | 74% |
| miR-520c-3p | 80% | miR-4478 | 75% | miR-6761-3p | 66% |
| miR-518c-5p | 74% | miR-3689d | 52% | miR-6762-5p | 65% |
| miR-520d-5p | 67% | miR-4481 | 68% | miR-6762-3p | 56% |
| miR-520d-3p | 54% | miR-4483 | 77% | miR-6763-5p | 84% |
| miR-516b-5p | 80% | miR-4484 | 81% | miR-6763-3p | 77% |
| miR-527, miR-518a-5p | 61% | miR-4486 | 68% | miR-6765-5p | 63% |
| miR-519a-3p | 71% | miR-4487 | 76% | miR-6766-5p | 70% |
| miR-502-5p | 53% | miR-4488 | 65% | miR-6766-3p | 75% |
| miR-513a-5p | 67% | miR-4489 | 63% | miR-6767-5p | 55% |
| miR-557 | 65% | miR-4492 | 59% | miR-6768-5p | 67% |
| miR-563 | 59% | miR-4496 | 82% | miR-6769a-5p | 76% |
| miR-564 | 64% | miR-4497 | 73% | miR-6769a-3p | 71% |
| miR-574-3p | 68% | miR-4498 | 64% | miR-6770-5p | 59% |
| miR-575 | 56% | miR-4499 | 77% | miR-6771-5p | 62% |
| miR-578 | 70% | miR-4501 | 60% | miR-6771-3p | 75% |
| miR-579-3p | 62% | miR-4505 | 73% | miR-6772-5p | 64% |
| miR-583 | 67% | miR-4506 | 55% | miR-6772-3p | 57% |
| miR-585-3p | 65% | miR-2392 | 70% | miR-6773-3p | 57% |
| miR-588 | 67% | miR-4507 | 75% | miR-6774-5p | 74% |
| miR-550a-3p | 51% | miR-4508 | 65% | miR-6775-5p | 72% |
| miR-595 | 53% | miR-4510 | 57% | miR-6775-3p | 74% |
| miR-601 | 51% | miR-4513 | 74% | miR-6776-5p | 74% |
| miR-608 | 56% | miR-4516 | 70% | miR-6776-3p | 72% |
| miR-610 | 55% | miR-4518 | 63% | miR-6777-5p | 79% |
| miR-612 | 54% | miR-4521 | 75% | miR-6777-3p | 75% |
| miR-615-3p | 61% | miR-1269b | 63% | miR-6778-5p | 72% |
| miR-617 | 53% | miR-4522 | 67% | miR-6778-3p | 61% |
| miR-624-5p | 55% | miR-4523 | 55% | miR-6779-5p | 71% |
| miR-625-5p | 64% | miR-4525 | 74% | miR-6779-3p | 66% |
| miR-628-3p | 67% | miR-4526 | 56% | miR-6780a-5p | 75% |
| miR-632 | 51% | miR-4530 | 71% | miR-6780a-3p | 60% |
| miR-634 | 77% | miR-4531 | 70% | miR-6781-5p | 76% |
| miR-636 | 57% | miR-4533 | 71% | miR-6782-5p | 74% |
| miR-637 | 73% | miR-4534 | 71% | miR-6782-3p | 74% |
| miR-638 | 72% | miR-4535 | 64% | miR-6783-5p | 63% |
| miR-639 | 86% | miR-1587 | 67% | miR-6783-3p | 58% |
| miR-641 | 100% | miR-4538 | 65% | miR-6784-5p | 67% |
| miR-650 | 54% | miR-4539 | 67% | miR-6784-3p | 73% |
| miR-663a | 65% | miR-3127-3p | 73% | miR-6785-3p | 74% |
| miR-449b-5p | 57% | miR-3156-3p | 62% | miR-6786-5p | 67% |
| miR-654-5p | 52% | miR-3158-5p | 74% | miR-6786-3p | 76% |
| miR-657 | 59% | miR-3162-3p | 73% | miR-6787-5p | 74% |
| miR-658 | 73% | miR-3173-5p | 62% | miR-6787-3p | 63% |
| miR-542-5p | 83% | miR-3187-5p | 65% | miR-6788-5p | 55% |
| miR-671-5p | 66% | miR-3189-5p | 74% | miR-6788-3p | 70% |
| miR-668-3p | 58% | miR-3619-3p | 76% | miR-6789-5p | 69% |
| miR-767-3p | 75% | miR-3150b-5p | 74% | miR-6789-3p | 51% |
| miR-769-5p | 53% | miR-3940-5p | 66% | miR-6790-5p | 65% |
| miR-769-3p | 52% | miR-3960 | 62% | miR-6790-3p | 76% |
| miR-766-3p | 76% | miR-3972 | 58% | miR-6791-5p | 71% |
| miR-765 | 76% | miR-3978 | 100% | miR-6792-5p | 62% |
| miR-675-5p | 65% | miR-4634 | 69% | miR-6792-3p | 75% |
| miR-297 | 52% | miR-4638-5p | 72% | miR-6793-5p | 58% |
| let-7b-3p | 72% | miR-4639-3p | 58% | miR-6793-3p | 73% |
| let-7d-3p | 53% | miR-4640-5p | 70% | miR-6794-5p | 86% |
| let-7f-1-3p | 72% | miR-4640-3p | 69% | miR-6794-3p | 67% |
| miR-25-5p | 53% | miR-4642 | 72% | miR-6795-5p | 69% |
| miR-26b-3p | 53% | miR-4646-5p | 74% | miR-6795-3p | 76% |
| miR-92a-2-5p | 63% | miR-4646-3p | 72% | miR-6796-5p | 75% |
| miR-29b-2-5p | 55% | miR-4648 | 59% | miR-6796-3p | 80% |
| miR-30c-2-3p | 67% | miR-4649-5p | 63% | miR-6797-5p | 75% |
| miR-139-3p | 57% | miR-4649-3p | 71% | miR-6797-3p | 73% |
| miR-181c-3p | 67% | miR-4650-3p | 83% | miR-6798-5p | 73% |
| miR-183-3p | 75% | miR-4651 | 76% | miR-6798-3p | 74% |
| miR-187-5p | 69% | miR-4652-5p | 64% | miR-6799-5p | 73% |
| miR-15b-3p | 67% | miR-4653-3p | 58% | miR-6799-3p | 72% |
| miR-124-5p | 71% | miR-4654 | 56% | miR-6800-5p | 63% |
| miR-125b-1-3p | 100% | miR-4655-5p | 66% | miR-6800-3p | 74% |
| miR-130a-5p | 53% | miR-4656 | 72% | miR-6801-3p | 72% |
| miR-135a-3p | 56% | miR-4659b-3p | 57% | miR-6802-5p | 63% |
| miR-125a-3p | 73% | miR-4664-5p | 69% | miR-6802-3p | 74% |
| miR-127-5p | 67% | miR-4664-3p | 76% | miR-6803-5p | 63% |
| miR-149-3p | 66% | miR-4665-5p | 83% | miR-6803-3p | 68% |
| miR-150-3p | 80% | miR-4665-3p | 74% | miR-6804-3p | 71% |
| miR-185-3p | 71% | miR-4667-5p | 77% | miR-6805-5p | 71% |
| miR-186-3p | 64% | miR-4667-3p | 75% | miR-6805-3p | 78% |
| miR-193a-5p | 61% | miR-4669 | 65% | miR-6806-5p | 52% |
| miR-194-3p | 65% | miR-4672 | 56% | miR-6807-5p | 70% |
| miR-30c-1-3p | 52% | miR-4673 | 51% | miR-6808-5p | 64% |
| miR-296-3p | 80% | miR-4674 | 67% | miR-6808-3p | 55% |
| miR-361-3p | 74% | miR-4675 | 61% | miR-6809-5p | 78% |
| miR-302d-5p | 54% | miR-4677-5p | 70% | miR-6809-3p | 64% |
| miR-371a-5p | 64% | miR-4685-5p | 67% | miR-6810-5p | 71% |
| miR-374a-3p | 100% | miR-4685-3p | 73% | miR-6810-3p | 78% |
| miR-377-5p | 54% | miR-4687-5p | 74% | miR-6812-5p | 75% |
| miR-342-5p | 77% | miR-4687-3p | 64% | miR-6812-3p | 75% |
| miR-423-5p | 67% | miR-1343-3p | 71% | miR-6813-5p | 70% |
| miR-424-3p | 80% | miR-4688 | 68% | miR-6813-3p | 75% |
| miR-18b-3p | 74% | miR-4689 | 63% | miR-6814-3p | 53% |
| miR-20b-3p | 70% | miR-4690-5p | 74% | miR-6815-5p | 69% |
| miR-431-3p | 54% | miR-4691-5p | 53% | miR-6816-5p | 63% |
| miR-483-5p | 79% | miR-4695-5p | 72% | miR-6816-3p | 73% |
| miR-486-3p | 67% | miR-4695-3p | 69% | miR-6818-5p | 71% |
| miR-193b-5p | 54% | miR-4697-5p | 65% | miR-6819-5p | 62% |
| miR-505-5p | 70% | miR-4697-3p | 72% | miR-6819-3p | 76% |
| miR-532-3p | 64% | miR-4698 | 73% | miR-6820-5p | 72% |
| miR-92b-5p | 77% | miR-4700-5p | 66% | miR-6820-3p | 75% |
| miR-551b-5p | 65% | miR-4700-3p | 71% | miR-6821-5p | 75% |
| miR-574-5p | 68% | miR-4701-5p | 72% | miR-6822-5p | 65% |
| miR-550a-5p | 70% | miR-4701-3p | 58% | miR-6823-5p | 60% |
| miR-615-5p | 74% | miR-4706 | 67% | miR-6823-3p | 69% |
| miR-616-3p | 55% | miR-4707-5p | 73% | miR-6824-5p | 69% |
| miR-625-3p | 74% | miR-4707-3p | 75% | miR-6824-3p | 67% |
| miR-629-5p | 88% | miR-4708-3p | 62% | miR-6825-5p | 78% |
| miR-298 | 67% | miR-4709-3p | 69% | miR-6825-3p | 69% |
| miR-874-3p | 70% | miR-4710 | 66% | miR-6826-3p | 73% |
| miR-876-3p | 80% | miR-4713-5p | 70% | miR-6827-5p | 71% |
| miR-744-5p | 61% | miR-4714-5p | 73% | miR-6827-3p | 75% |
| miR-885-5p | 62% | miR-4716-5p | 76% | miR-6828-5p | 73% |
| miR-885-3p | 58% | miR-4716-3p | 80% | miR-6829-5p | 80% |
| miR-877-5p | 66% | miR-4717-3p | 57% | miR-6829-3p | 57% |
| miR-877-3p | 76% | miR-4720-5p | 63% | miR-6830-5p | 79% |
| miR-887-3p | 68% | miR-4721 | 69% | miR-6830-3p | 71% |
| miR-665 | 76% | miR-4722-5p | 62% | miR-6831-5p | 74% |
| miR-760 | 66% | miR-4722-3p | 66% | miR-6831-3p | 64% |
| miR-920 | 81% | miR-4723-5p | 79% | miR-6832-5p | 75% |
| miR-921 | 52% | miR-4723-3p | 69% | miR-6832-3p | 53% |
| miR-509-3-5p | 100% | miR-4725-3p | 79% | miR-6833-5p | 70% |
| miR-933 | 74% | miR-4726-5p | 58% | miR-6833-3p | 66% |
| miR-936 | 65% | miR-4727-3p | 61% | miR-6834-5p | 78% |
| miR-939-5p | 78% | miR-4728-5p | 75% | miR-6834-3p | 73% |
| miR-940 | 72% | miR-4728-3p | 74% | miR-6780b-5p | 77% |
| miR-1224-5p | 62% | miR-4729 | 100% | miR-6780b-3p | 71% |
| miR-1224-3p | 77% | miR-4730 | 61% | miR-6836-3p | 73% |
| miR-1225-5p | 70% | miR-4731-5p | 65% | miR-6838-5p | 56% |
| miR-1225-3p | 76% | miR-4731-3p | 74% | miR-6839-5p | 52% |
| miR-1227-3p | 66% | miR-4732-5p | 76% | miR-6840-5p | 72% |
| miR-1228-5p | 65% | miR-4732-3p | 61% | miR-6840-3p | 76% |
| miR-1228-3p | 76% | miR-4734 | 69% | miR-6841-3p | 73% |
| miR-1229-3p | 67% | miR-4736 | 65% | miR-6842-5p | 79% |
| miR-1231 | 67% | miR-3064-5p | 53% | miR-6845-5p | 71% |
| miR-1233-3p | 63% | miR-3064-3p | 52% | miR-6845-3p | 76% |
| miR-1234-3p | 71% | miR-4738-3p | 55% | miR-6846-5p | 80% |
| miR-1236-3p | 71% | miR-4739 | 62% | miR-6846-3p | 72% |
| miR-1237-3p | 78% | miR-4741 | 77% | miR-6847-3p | 63% |
| miR-1238-3p | 74% | miR-4743-5p | 71% | miR-6848-5p | 70% |
| miR-320b | 59% | miR-4745-5p | 73% | miR-6848-3p | 71% |
| miR-320c | 64% | miR-4746-3p | 58% | miR-6849-5p | 78% |
| miR-1323 | 59% | miR-4747-5p | 76% | miR-6850-5p | 68% |
| miR-1301-3p | 63% | miR-4748 | 71% | miR-6851-5p | 61% |
| miR-1298-5p | 61% | miR-4749-5p | 77% | miR-6851-3p | 77% |
| miR-1181 | 63% | miR-4749-3p | 74% | miR-6855-5p | 61% |
| miR-1182 | 71% | miR-4750-5p | 63% | miR-6855-3p | 74% |
| miR-1184 | 58% | miR-4751 | 77% | miR-6856-5p | 59% |
| miR-1202 | 69% | miR-4752 | 52% | miR-6857-5p | 73% |
| miR-1203 | 61% | miR-4753-5p | 67% | miR-6857-3p | 73% |
| miR-663b | 67% | miR-371b-5p | 74% | miR-6858-5p | 63% |
| miR-1207-5p | 83% | miR-371b-3p | 70% | miR-6858-3p | 75% |
| miR-1207-3p | 51% | miR-4754 | 57% | miR-6859-5p | 59% |
| miR-1289 | 75% | miR-4755-3p | 66% | miR-6859-3p | 77% |
| miR-1294 | 65% | miR-4756-5p | 70% | miR-6769b-5p | 74% |
| miR-1299 | 100% | miR-4758-5p | 83% | miR-6769b-3p | 58% |
| miR-1303 | 53% | miR-4758-3p | 70% | miR-6860 | 65% |
| miR-1304-5p | 60% | miR-4759 | 100% | miR-6861-5p | 74% |
| miR-1249-3p | 75% | miR-4763-5p | 68% | miR-6861-3p | 77% |
| miR-1257 | 52% | miR-4763-3p | 71% | miR-6862-5p | 71% |
| miR-1260a | 72% | miR-4764-5p | 100% | miR-6862-3p | 72% |
| miR-1263 | 60% | miR-4766-5p | 63% | miR-6865-5p | 63% |
| miR-1267 | 75% | miR-4769-5p | 67% | miR-6865-3p | 71% |
| miR-1268a | 66% | miR-4769-3p | 71% | miR-6867-5p | 64% |
| miR-1270 | 55% | miR-4776-5p | 71% | miR-6867-3p | 71% |
| miR-1275 | 66% | miR-4778-5p | 75% | miR-6868-3p | 56% |
| miR-1281 | 77% | miR-4779 | 88% | miR-6869-5p | 67% |
| miR-1292-5p | 85% | miR-4780 | 53% | miR-6870-5p | 73% |
| miR-664a-3p | 73% | miR-4436b-5p | 71% | miR-6870-3p | 75% |
| miR-1307-3p | 73% | miR-4781-5p | 100% | miR-6871-5p | 65% |
| miR-320d | 83% | miR-4783-3p | 74% | miR-6872-5p | 58% |
| miR-1825 | 75% | miR-2467-3p | 69% | miR-6872-3p | 70% |
| miR-675-3p | 65% | miR-4787-5p | 65% | miR-6873-5p | 57% |
| miR-1469 | 70% | miR-4787-3p | 75% | miR-6874-5p | 53% |
| miR-1470 | 79% | miR-4788 | 70% | miR-6875-5p | 70% |
| miR-1471 | 69% | miR-4793-3p | 72% | miR-6875-3p | 67% |
| miR-1539 | 60% | miR-4800-5p | 83% | miR-6876-5p | 72% |
| miR-1908-5p | 74% | miR-4804-3p | 53% | miR-6877-5p | 79% |
| miR-1909-5p | 60% | miR-642a-3p | 69% | miR-6877-3p | 65% |
| miR-1909-3p | 68% | miR-4433a-5p | 75% | miR-6878-5p | 57% |
| miR-1910-5p | 66% | miR-4482-3p | 77% | miR-6878-3p | 67% |
| miR-1912-3p | 67% | miR-4999-5p | 55% | miR-6879-5p | 77% |
| miR-1913 | 73% | miR-5001-5p | 65% | miR-6879-3p | 72% |
| miR-1914-3p | 77% | miR-5002-3p | 56% | miR-6880-5p | 75% |
| miR-1915-3p | 67% | miR-5003-3p | 79% | miR-6880-3p | 74% |
| miR-365a-5p | 73% | miR-5006-5p | 53% | miR-6881-5p | 73% |
| miR-449b-3p | 71% | miR-5008-5p | 57% | miR-6881-3p | 60% |
| miR-2113 | 57% | miR-5009-3p | 64% | miR-6882-5p | 62% |
| miR-1976 | 72% | miR-5010-5p | 69% | miR-6882-3p | 67% |
| miR-2110 | 77% | miR-5010-3p | 71% | miR-6883-5p | 68% |
| miR-151b | 52% | miR-5088-5p | 62% | miR-6883-3p | 62% |
| miR-762 | 68% | miR-5089-5p | 58% | miR-6884-3p | 77% |
| miR-670-5p | 60% | miR-5090 | 66% | miR-6885-5p | 58% |
| miR-764 | 67% | miR-5188 | 52% | miR-6885-3p | 77% |
| miR-2116-5p | 55% | miR-5189-5p | 60% | miR-6886-3p | 71% |
| miR-2116-3p | 73% | miR-5193 | 65% | miR-6887-5p | 71% |
| miR-548q | 59% | miR-5195-5p | 70% | miR-6887-3p | 76% |
| miR-2276-3p | 68% | miR-5195-3p | 64% | miR-6889-5p | 80% |
| miR-2278 | 68% | miR-5196-5p | 80% | miR-6889-3p | 72% |
| miR-711 | 80% | miR-4524b-5p | 55% | miR-6890-5p | 63% |
| miR-718 | 76% | miR-5100 | 58% | miR-6890-3p | 73% |
| miR-2681-3p | 65% | miR-5572 | 80% | miR-6891-5p | 71% |
| miR-2682-3p | 57% | miR-664b-5p | 53% | miR-6891-3p | 73% |
| miR-449c-3p | 51% | miR-664b-3p | 71% | miR-6892-5p | 70% |
| miR-2861 | 63% | miR-5581-5p | 51% | miR-6892-3p | 75% |
| miR-3116 | 67% | miR-5581-3p | 56% | miR-6893-5p | 80% |
| miR-3119 | 67% | miR-5584-5p | 73% | miR-6893-3p | 66% |
| miR-3120-3p | 63% | miR-548au-3p | 56% | miR-6894-5p | 73% |
| miR-3122 | 70% | miR-5588-5p | 54% | miR-6894-3p | 71% |
| miR-3124-5p | 65% | miR-5589-5p | 63% | miR-7106-5p | 72% |
| miR-3126-5p | 71% | miR-5684 | 58% | miR-7106-3p | 63% |
| miR-3130-5p | 61% | miR-5690 | 62% | miR-7107-5p | 82% |
| miR-3131 | 76% | miR-5698 | 75% | miR-7108-5p | 69% |
| miR-3132 | 63% | miR-5703 | 62% | miR-7108-3p | 79% |
| miR-3133 | 61% | miR-5705 | 59% | miR-7109-5p | 72% |
| miR-466 | 56% | miR-197-5p | 79% | miR-7109-3p | 67% |
| miR-3136-5p | 100% | miR-204-3p | 83% | miR-7110-5p | 74% |
| miR-3138 | 61% | miR-211-3p | 73% | miR-7110-3p | 72% |
| miR-3141 | 74% | miR-301a-5p | 52% | miR-7111-5p | 82% |
| miR-3144-5p | 70% | miR-345-3p | 70% | miR-7111-3p | 76% |
| miR-1273c | 67% | miR-652-5p | 71% | miR-7112-5p | 57% |
| miR-3147 | 75% | miR-1185-2-3p | 75% | miR-7113-5p | 51% |
| miR-3150a-3p | 76% | miR-873-3p | 76% | miR-7113-3p | 71% |
| miR-3151-5p | 67% | miR-1304-3p | 71% | miR-7114-5p | 78% |
| miR-3153 | 74% | miR-1247-3p | 74% | miR-7114-3p | 73% |
| miR-3154 | 76% | miR-1306-5p | 74% | miR-7150 | 76% |
| miR-3156-5p | 76% | miR-3184-3p | 72% | miR-7151-3p | 63% |
| miR-3157-5p | 64% | miR-3191-5p | 62% | miR-7152-5p | 61% |
| miR-3160-3p | 69% | miR-642b-5p | 59% | miR-7152-3p | 62% |
| miR-3161 | 54% | miR-365b-5p | 66% | miR-7155-5p | 62% |
| miR-3162-5p | 77% | miR-1185-1-3p | 77% | miR-7157-3p | 58% |
| miR-3163 | 83% | miR-98-3p | 71% | miR-7159-5p | 62% |
| miR-3164 | 61% | miR-381-5p | 62% | miR-7160-5p | 67% |
| miR-3165 | 60% | miR-503-3p | 66% | miR-7162-5p | 56% |
| miR-1260b | 64% | miR-937-5p | 69% | miR-7515 | 71% |
| miR-3169 | 62% | miR-939-3p | 73% | miR-7702 | 51% |
| miR-3173-3p | 53% | miR-1227-5p | 72% | miR-7704 | 67% |
| miR-1193 | 65% | miR-1229-5p | 67% | miR-7843-5p | 61% |
| miR-3175 | 62% | miR-1233-5p | 73% | miR-4433b-5p | 73% |
| miR-3177-3p | 75% | miR-1236-5p | 64% | miR-4433b-3p | 81% |
| miR-3178 | 69% | miR-1237-5p | 68% | miR-1273h-5p | 63% |
| miR-3179 | 57% | miR-1238-5p | 77% | miR-6516-5p | 56% |
| miR-3180-5p | 73% | miR-1292-3p | 69% | miR-7845-5p | 68% |
| miR-3180-3p | 65% | miR-3617-3p | 51% | miR-7846-3p | 76% |
| miR-3184-5p | 63% | miR-3620-5p | 69% | miR-7847-3p | 70% |
| miR-3185 | 80% | miR-4632-5p | 67% | miR-7851-3p | 63% |
| miR-3186-3p | 54% | miR-4750-3p | 75% | miR-7855-5p | 66% |
| miR-3188 | 71% | miR-5739 | 78% | miR-7856-5p | 51% |
| miR-3189-3p | 74% | miR-5787 | 65% | miR-8052 | 70% |
| miR-3190-5p | 73% | miR-6068 | 59% | miR-8054 | 75% |
| miR-3191-3p | 78% | miR-6069 | 72% | miR-8059 | 67% |
| miR-3194-5p | 62% | miR-6071 | 67% | miR-8060 | 71% |
| miR-3195 | 66% | miR-6072 | 60% | miR-8063 | 58% |
| miR-3196 | 65% | miR-6074 | 52% | miR-8064 | 53% |
| miR-3197 | 75% | miR-6075 | 69% | miR-8069 | 67% |
| miR-3198 | 63% | miR-6076 | 81% | miR-8071 | 77% |
| miR-3199 | 54% | miR-6077 | 62% | miR-8072 | 67% |
| miR-514b-5p | 65% | miR-6085 | 79% | miR-8073 | 72% |
| miR-3202 | 76% | miR-6086 | 81% | miR-8074 | 54% |
| miR-4297 | 56% | miR-6088 | 63% | miR-8075 | 51% |
| miR-4293 | 67% | miR-6089 | 71% | miR-8077 | 68% |
| miR-4294 | 79% | miR-6090 | 66% | miR-8080 | 62% |
| miR-4299 | 68% | miR-6124 | 78% | miR-8085 | 83% |
| miR-4298 | 75% | miR-6125 | 73% | miR-8087 | 79% |
| miR-4305 | 55% | miR-6126 | 66% | miR-8089 | 72% |
| miR-4306 | 68% | miR-6127 | 78% | miR-128-2-5p | 63% |
| miR-4307 | 73% | miR-378j | 65% | miR-7977 | 68% |
| miR-4312 | 75% | miR-6130 | 53% | miR-7978 | 100% |
| miR-4313 | 71% | miR-6131 | 80% | miR-1249-5p | 68% |
| miR-4316 | 58% | miR-6132 | 70% | miR-4485-5p | 67% |
| miR-4314 | 55% | miR-6133 | 77% | miR-8485 | 69% |
| miR-4322 | 61% | miR-6165 | 79% | miR-375-5p | 72% |
| miR-4321 | 58% | miR-6500-3p | 65% | miR-498-3p | 56% |
| miR-4323 | 72% | miR-6501-3p | 55% | miR-526a-3p | 100% |
| miR-4256 | 64% | miR-6503-5p | 71% | miR-9718 | 75% |
| miR-4257 | 78% | miR-6506-5p | 52% | miR-9898 | 74% |
| miR-4258 | 75% | miR-6507-3p | 68% | miR-1843 | 51% |
| miR-4259 | 74% | miR-6508-5p | 56% | miR-10392-5p | 71% |
| miR-4260 | 71% | miR-6509-3p | 56% | miR-10392-3p | 74% |
| miR-4253 | 57% | miR-6510-5p | 77% | miR-10394-3p | 57% |
| miR-4251 | 66% | miR-6511a-5p | 83% | miR-10395-5p | 51% |
| miR-4325 | 69% | miR-6511a-3p | 66% | miR-10395-3p | 60% |
| miR-4326 | 66% | miR-6513-5p | 55% | miR-10396a-5p | 67% |
| miR-4327 | 76% | miR-6514-3p | 61% | miR-10396a-3p | 74% |
| miR-4261 | 52% | miR-6515-5p | 63% | miR-10398-5p | 69% |
| miR-4265 | 57% | miR-6515-3p | 79% | miR-10398-3p | 85% |
| miR-4266 | 62% | miR-6715b-3p | 67% | miR-10400-5p | 64% |
| miR-2355-5p | 75% | miR-6716-5p | 69% | miR-10400-3p | 70% |
| miR-4268 | 67% | miR-6716-3p | 60% | miR-10401-5p | 80% |
| miR-4269 | 71% | miR-6717-5p | 70% | miR-10401-3p | 77% |
| miR-4271 | 69% | miR-6511b-5p | 81% | miR-10396b-5p | 69% |
| miR-4276 | 61% | miR-6511b-3p | 74% | miR-10522-5p | 86% |
| miR-4274 | 75% | miR-6719-3p | 60% | miR-10526-3p | 82% |
| miR-4281 | 67% | miR-6721-5p | 69% | miR-10527-5p | 80% |
| miR-4279 | 72% | miR-6722-5p | 60% | miR-11181-5p | 67% |
| miR-4278 | 56% | miR-6722-3p | 64% | miR-11181-3p | 76% |
| miR-4285 | 64% | miR-6724-5p | 72% | miR-11399 | 68% |
| miR-4283 | 52% | miR-208a-5p | 58% | miR-11400 | 57% |
| miR-4284 | 72% | miR-210-5p | 73% | miR-11401 | 54% |
| miR-4286 | 73% | miR-128-1-5p | 64% | miR-3059-5p | 70% |
| miR-4287 | 59% | miR-370-5p | 53% | miR-3059-3p | 51% |
| miR-4292 | 74% | miR-328-5p | 65% | miR-3085-5p | 67% |
| miR-4290 | 68% | miR-410-5p | 54% | miR-3085-3p | 73% |
| miR-4329 | 69% | miR-181d-3p | 83% | miR-6529-5p | 55% |
| miR-3200-5p | 65% | miR-520f-5p | 55% | miR-12114 | 65% |
| miR-3605-3p | 68% | miR-504-3p | 65% | miR-12115 | 68% |
| miR-3610 | 79% | miR-487b-5p | 55% | miR-12116 | 78% |
| miR-3612 | 74% | miR-585-5p | 56% | miR-12118 | 70% |
| miR-3614-5p | 73% | miR-597-3p | 63% | miR-12119 | 80% |
| miR-3616-3p | 58% | miR-598-5p | 63% | miR-12120 | 69% |
| miR-3620-3p | 69% | miR-605-3p | 68% | miR-12121 | 64% |
| miR-3621 | 65% | miR-619-5p | 54% | miR-12122 | 55% |
| miR-3622a-5p | 56% | miR-1296-3p | 67% | miR-12124 | 52% |
| miR-3622a-3p | 64% | miR-874-5p | 73% | miR-12126 | 73% |
| miR-3622b-5p | 74% | miR-887-5p | 68% | miR-12131 | 100% |
| miR-3648 | 79% | miR-1250-3p | 52% | miR-12136 | 68% |

### [Table 23-4]

**Table 23-4: miRNA to Predict Human Lymphoma of Stages I and II**

| miRNA Name | A | miRNA Name | A | miRNA Name | A |
|---|---|---|---|---|---|
| miR-29a-3p | 55% | miR-1915-3p | 77% | miR-4724-3p | 53% |
| miR-29b-3p | 55% | miR-3124-5p | 58% | miR-4727-3p | 57% |
| miR-140-5p | 67% | miR-3157-5p | 62% | miR-5188 | 52% |
| miR-106b-5p | 75% | miR-3166 | 56% | miR-5579-5p | 100% |
| miR-299-3p | 65% | miR-3178 | 72% | miR-5579-3p | 100% |
| miR-20b-5p | 71% | miR-320e | 57% | miR-664b-5p | 52% |
| miR-448 | 57% | miR-3199 | 64% | miR-5588-5p | 52% |
| miR-492 | 59% | miR-4295 | 56% | miR-381-5p | 58% |
| miR-498-5p | 86% | miR-4308 | 55% | miR-6074 | 58% |
| miR-520d-3p | 56% | miR-4315 | 59% | miR-6081 | 67% |
| miR-516b-3p, miR-516a-3p | 53% | miR-4321 | 64% | miR-6082 | 56% |
| miR-502-5p | 55% | miR-4255 | 51% | miR-6499-5p | 58% |
| miR-628-3p | 67% | miR-4327 | 79% | miR-6501-5p | 53% |
| miR-639 | 80% | miR-3651 | 60% | miR-605-3p | 73% |
| miR-769-5p | 56% | miR-3660 | 63% | miR-6770-5p | 66% |
| miR-22-5p | 100% | miR-3665 | 73% | miR-6789-5p | 79% |
| miR-27a-5p | 67% | miR-3666 | 52% | miR-6814-5p | 53% |
| miR-124-5p | 80% | miR-3677-3p | 77% | miR-6836-5p | 60% |
| miR-150-3p | 78% | miR-3918 | 58% | miR-6836-3p | 81% |
| miR-155-3p | 52% | miR-3937 | 75% | miR-6850-5p | 78% |
| miR-551b-5p | 66% | miR-4460 | 86% | miR-7108-3p | 82% |
| miR-629-5p | 88% | miR-4464 | 100% | miR-135a-2-3p | 54% |
| miR-921 | 59% | miR-4512 | 78% | miR-9898 | 79% |
| miR-1226-5p | 57% | miR-4536-5p | 100% | miR-9985 | 64% |
| miR-513c-5p | 51% | miR-4635 | 52% | miR-10392-3p | 81% |
| miR-320c | 61% | miR-4672 | 59% | miR-10522-5p | 82% |
| miR-1179 | 100% | miR-4673 | 73% | miR-12117 | 52% |
| miR-1286 | 63% | miR-4674 | 71% | miR-12120 | 74% |
| miR-1321 | 51% | miR-4724-5p | 54% | miR-12122 | 63% |
| miR-1470 | 79% | | | | |

| | | | | | |
|---|---|---|---|---|---|
| ^{∗} A represents Accuracy. ^{∗} "has-" in the miRNA names has been omitted. | | | | | |

### [Table 23-5]

**Table 23-5: miRNA to Predict Human Pancreatic Cancer of Stages I and II**

| miRNA Name | A | miRNA Name | A | miRNA Name | A |
|---|---|---|---|---|---|
| miR-15a-5p | 67% | miR-4289 | 59% | miR-6511a-5p | 87% |
| miR-16-5p | 53% | miR-4290 | 72% | miR-6511a-3p | 73% |
| miR-17-3p | 100% | miR-4329 | 70% | miR-6512-3p | 55% |
| miR-19b-3p | 100% | miR-500b-5p | 53% | miR-6513-5p | 62% |
| miR-20a-5p | 100% | miR-3200-5p | 77% | miR-6514-3p | 67% |
| miR-27a-3p | 55% | miR-3605-3p | 70% | miR-6515-5p | 63% |
| miR-28-5p | 52% | miR-3609 | 100% | miR-6515-3p | 77% |
| miR-29a-3p | 68% | miR-3610 | 72% | miR-6715b-3p | 70% |
| miR-30a-5p | 67% | miR-3612 | 74% | miR-6716-5p | 70% |
| miR-33a-5p | 100% | miR-3613-5p | 100% | miR-6716-3p | 69% |
| miR-99a-5p | 100% | miR-3614-5p | 68% | miR-6717-5p | 69% |
| miR-29b-3p | 53% | miR-3616-3p | 59% | miR-6511b-5p | 85% |
| miR-196a-5p | 67% | miR-3617-5p | 100% | miR-6511b-3p | 78% |
| miR-197-3p | 80% | miR-3618 | 100% | miR-6719-3p | 67% |
| miR-198 | 78% | miR-3620-3p | 72% | miR-6721-5p | 68% |
| miR-30d-5p | 69% | miR-3621 | 71% | miR-6722-5p | 67% |
| miR-139-5p | 100% | miR-3622a-5p | 59% | miR-6722-3p | 64% |
| miR-10a-5p | 56% | miR-3622a-3p | 64% | miR-6724-5p | 68% |
| miR-34a-5p | 52% | miR-3622b-5p | 70% | miR-210-5p | 72% |
| miR-181c-5p | 100% | miR-3646 | 59% | miR-128-1-5p | 64% |
| miR-182-3p | 100% | miR-3648 | 77% | miR-152-5p | 75% |
| miR-183-5p | 75% | miR-3649 | 62% | miR-372-5p | 100% |
| miR-187-3p | 63% | miR-3650 | 56% | miR-328-5p | 64% |
| miR-199b-5p | 76% | miR-3652 | 71% | miR-329-5p | 58% |
| miR-210-3p | 52% | miR-3660 | 59% | miR-410-5p | 61% |
| miR-211-5p | 54% | miR-3662 | 100% | miR-487a-5p | 53% |
| miR-214-3p | 63% | miR-3663-5p | 70% | miR-494-5p | 53% |
| miR-218-5p | 54% | miR-3663-3p | 66% | miR-181d-3p | 88% |
| miR-223-3p | 72% | miR-3665 | 66% | miR-520f-5p | 62% |
| let-7g-5p | 100% | miR-3667-5p | 56% | miR-519d-5p | 67% |
| miR-125b-5p | 52% | miR-3667-3p | 70% | miR-520g-5p | 100% |
| miR-133a-3p | 68% | miR-3675-3p | 74% | miR-504-3p | 68% |
| miR-135a-5p | 53% | miR-3679-5p | 75% | miR-487b-5p | 63% |
| miR-9-5p | 100% | miR-3679-3p | 77% | miR-585-5p | 61% |
| miR-125a-5p | 57% | miR-3681-5p | 100% | miR-598-5p | 61% |
| miR-134-5p | 68% | miR-3688-3p | 67% | miR-605-3p | 74% |
| miR-149-5p | 68% | miR-3689a-3p | 63% | miR-1296-3p | 68% |
| miR-154-3p | 54% | miR-3691-5p | 56% | miR-891a-3p | 55% |
| miR-184 | 51% | miR-3714 | 70% | miR-874-5p | 72% |
| miR-185-5p | 68% | miR-3180 | 66% | miR-887-5p | 60% |
| miR-188-5p | 67% | miR-3907 | 75% | miR-1287-3p | 53% |
| miR-320a-3p | 72% | miR-3689b-3p, miR-3689c | 63% | miR-1250-3p | 57% |
| miR-200c-3p | 100% | miR-3908 | 63% | miR-1537-5p | 100% |
| miR-106b-5p | 80% | miR-3909 | 65% | miR-1908-3p | 69% |
| miR-29c-3p | 100% | miR-3911 | 62% | miR-3151-3p | 74% |
| miR-200a-3p | 67% | miR-3914 | 100% | miR-3192-3p | 59% |
| miR-302a-3p | 100% | miR-3915 | 100% | miR-1343-5p | 72% |
| miR-299-3p | 70% | miR-3917 | 63% | miR-5088-3p | 73% |
| miR-296-5p | 70% | miR-3918 | 52% | miR-5189-3p | 62% |
| miR-130b-3p | 83% | miR-3919 | 68% | miR-5699-5p | 70% |
| miR-361-5p | 56% | miR-3150b-3p | 60% | miR-6726-5p | 79% |
| miR-365a-3p, miR-365b-3p | 68% | miR-3922-3p | 57% | miR-6726-3p | 72% |
| miR-302c-5p | 72% | miR-3924 | 100% | miR-6727-5p | 67% |
| miR-370-3p | 83% | miR-3925-5p | 82% | miR-6727-3p | 73% |
| miR-373-5p | 72% | miR-3926 | 67% | miR-6728-5p | 59% |
| miR-373-3p | 64% | miR-676-3p | 54% | miR-6728-3p | 76% |
| miR-378a-3p | 70% | miR-3928-3p | 72% | miR-6729-5p | 71% |
| miR-379-5p | 80% | miR-3934-5p | 52% | miR-6729-3p | 76% |
| miR-328-3p | 75% | miR-3935 | 60% | miR-6730-5p | 84% |
| miR-342-3p | 59% | miR-3936 | 63% | miR-6730-3p | 75% |
| miR-337-3p | 51% | miR-3937 | 71% | miR-6731-5p | 71% |
| miR-326 | 66% | miR-3941 | 63% | miR-6731-3p | 74% |
| miR-151a-3p | 57% | miR-3943 | 70% | miR-6732-5p | 74% |
| miR-133b | 73% | miR-3944-3p | 66% | miR-6732-3p | 76% |
| miR-325 | 63% | miR-3945 | 55% | miR-6733-5p | 100% |
| miR-346 | 70% | miR-642b-3p | 65% | miR-6734-5p | 78% |
| miR-422a | 53% | miR-550b-3p | 60% | miR-6734-3p | 73% |
| miR-424-5p | 56% | miR-1268b | 74% | miR-6735-5p | 71% |
| miR-18b-5p | 100% | miR-4418 | 100% | miR-6735-3p | 67% |
| miR-20b-5p | 60% | miR-4421 | 63% | miR-6736-5p | 69% |
| miR-429 | 100% | miR-4428 | 76% | miR-6736-3p | 70% |
| miR-450a-5p | 60% | miR-4429 | 78% | miR-6737-5p | 68% |
| miR-191-3p | 72% | miR-4430 | 79% | miR-6737-3p | 73% |
| miR-200a-5p | 59% | miR-548ad-3p | 100% | miR-6738-5p | 74% |
| miR-323b-5p | 59% | miR-4433a-3p | 73% | miR-6738-3p | 68% |
| miR-409-3p | 69% | miR-4436a | 64% | miR-6740-5p | 60% |
| miR-412-3p | 52% | miR-4437 | 58% | miR-6740-3p | 74% |
| miR-410-3p | 100% | miR-4439 | 68% | miR-6741-5p | 79% |
| miR-483-3p | 71% | miR-4440 | 68% | miR-6741-3p | 76% |
| miR-484 | 70% | miR-4441 | 61% | miR-6742-5p | 90% |
| miR-485-3p | 68% | miR-4442 | 65% | miR-6742-3p | 74% |
| miR-486-5p | 75% | miR-4443 | 59% | miR-6743-5p | 72% |
| miR-488-5p | 52% | miR-4444 | 68% | miR-6743-3p | 72% |
| miR-491-5p | 82% | miR-4446-3p | 67% | miR-6744-5p | 64% |
| miR-146b-5p | 100% | miR-4447 | 79% | miR-6744-3p | 64% |
| miR-202-3p | 63% | miR-4448 | 64% | miR-6745 | 78% |
| miR-492 | 58% | miR-4449 | 64% | miR-6746-5p | 92% |
| miR-512-5p | 56% | miR-548ah-5p | 100% | miR-6747-5p | 73% |
| miR-498-5p | 86% | miR-4455 | 64% | miR-6747-3p | 67% |
| miR-520e-3p | 81% | miR-4456 | 68% | miR-6748-5p | 59% |
| miR-515-3p | 57% | miR-4458 | 64% | miR-6748-3p | 73% |
| miR-519e-5p | 51% | miR-4460 | 75% | miR-6749-5p | 77% |
| miR-519e-3p | 58% | miR-378h | 60% | miR-6749-3p | 74% |
| miR-520f-3p | 100% | miR-3135b | 57% | miR-6750-5p | 59% |
| miR-519c-5p, miR-523-5p, miR-518e-5p, miR-522-5p, miR-519a-5p, miR-519b-5p | 55% | miR-4462 | 67% | miR-6750-3p | 73% |
| miR-526b-5p | 60% | miR-4463 | 65% | miR-6751-5p | 74% |
| miR-526b-3p | 51% | miR-548ai, miR-570-5p | 100% | miR-6751-3p | 72% |
| miR-525-3p | 51% | miR-4466 | 71% | miR-6752-5p | 68% |
| miR-518f-3p | 51% | miR-4467 | 72% | miR-6752-3p | 72% |
| miR-520b-3p | 77% | miR-4468 | 71% | miR-6753-5p | 70% |
| miR-518b | 75% | miR-4470 | 89% | miR-6753-3p | 70% |
| miR-520c-3p | 78% | miR-4471 | 100% | miR-6754-5p | 65% |
| miR-518c-5p | 74% | miR-4472 | 83% | miR-6754-3p | 68% |
| miR-519d-3p | 53% | miR-4474-3p | 57% | miR-6756-5p | 66% |
| miR-516b-3p, miR-516a-3p | 56% | miR-4476 | 69% | miR-6756-3p | 74% |
| miR-522-3p | 54% | miR-4478 | 73% | miR-6757-5p | 67% |
| miR-500a-3p | 58% | miR-3155b | 63% | miR-6757-3p | 75% |
| miR-503-5p | 75% | miR-4480 | 100% | miR-6758-5p | 54% |
| miR-513a-5p | 62% | miR-4481 | 72% | miR-6759-5p | 70% |
| miR-532-5p | 80% | miR-4483 | 74% | miR-6759-3p | 75% |
| miR-455-5p | 100% | miR-4484 | 75% | miR-6760-5p | 89% |
| miR-539-5p | 54% | miR-4486 | 70% | miR-6760-3p | 77% |
| miR-551a | 55% | miR-4487 | 72% | miR-6761-3p | 71% |
| miR-554 | 57% | miR-4488 | 67% | miR-6762-5p | 63% |
| miR-555 | 56% | miR-4489 | 59% | miR-6762-3p | 58% |
| miR-557 | 68% | miR-4492 | 67% | miR-6763-5p | 75% |
| miR-564 | 68% | miR-4495 | 100% | miR-6763-3p | 74% |
| miR-571 | 100% | miR-4496 | 87% | miR-6765-5p | 65% |
| miR-574-3p | 72% | miR-4497 | 68% | miR-6766-5p | 67% |
| miR-575 | 65% | miR-4498 | 70% | miR-6766-3p | 74% |
| miR-579-3p | 62% | miR-4499 | 86% | miR-6767-5p | 63% |
| miR-580-3p | 58% | miR-4505 | 67% | miR-6768-5p | 67% |
| miR-583 | 67% | miR-4506 | 63% | miR-6769a-5p | 70% |
| miR-584-5p | 57% | miR-2392 | 68% | miR-6769a-3p | 73% |
| miR-585-3p | 68% | miR-4507 | 66% | miR-6771-5p | 70% |
| miR-587 | 100% | miR-4508 | 65% | miR-6771-3p | 74% |
| miR-588 | 67% | miR-4510 | 61% | miR-6772-5p | 67% |
| miR-550a-3p | 62% | miR-4511 | 59% | miR-6772-3p | 59% |
| miR-595 | 63% | miR-4512 | 67% | miR-6773-3p | 63% |
| miR-600 | 62% | miR-4513 | 68% | miR-6774-5p | 71% |
| miR-601 | 51% | miR-4516 | 71% | miR-6774-3p | 53% |
| miR-608 | 68% | miR-4518 | 67% | miR-6775-5p | 73% |
| miR-610 | 60% | miR-4519 | 100% | miR-6775-3p | 75% |
| miR-612 | 60% | miR-4520-3p | 63% | miR-6776-5p | 77% |
| miR-615-3p | 62% | miR-4521 | 58% | miR-6776-3p | 79% |
| miR-617 | 66% | miR-1269b | 57% | miR-6777-5p | 80% |
| miR-618 | 100% | miR-4522 | 57% | miR-6777-3p | 76% |
| miR-619-3p | 100% | miR-4523 | 65% | miR-6778-5p | 74% |
| miR-622 | 53% | miR-4524a-3p | 71% | miR-6778-3p | 62% |
| miR-624-5p | 55% | miR-4525 | 77% | miR-6779-5p | 73% |
| miR-626 | 100% | miR-4526 | 55% | miR-6779-3p | 67% |
| miR-628-3p | 58% | miR-4528 | 100% | miR-6780a-5p | 84% |
| miR-629-3p | 56% | miR-4530 | 72% | miR-6780a-3p | 64% |
| miR-632 | 51% | miR-4531 | 72% | miR-6781-5p | 75% |
| miR-634 | 74% | miR-4533 | 69% | miR-6782-5p | 68% |
| miR-637 | 73% | miR-4534 | 83% | miR-6782-3p | 76% |
| miR-638 | 70% | miR-4535 | 62% | miR-6783-5p | 67% |
| miR-639 | 86% | miR-1587 | 64% | miR-6783-3p | 70% |
| miR-641 | 100% | miR-4538 | 62% | miR-6784-5p | 67% |
| miR-642a-5p | 67% | miR-4539 | 71% | miR-6784-3p | 78% |
| miR-648 | 100% | miR-4540 | 57% | miR-6785-5p | 68% |
| miR-649 | 52% | miR-3120-5p | 51% | miR-6785-3p | 73% |
| miR-650 | 55% | miR-3121-5p | 100% | miR-6786-5p | 67% |
| miR-661 | 57% | miR-3127-3p | 71% | miR-6786-3p | 72% |
| miR-663a | 69% | miR-3140-5p | 100% | miR-6787-5p | 68% |
| miR-449b-5p | 59% | miR-3156-3p | 69% | miR-6787-3p | 67% |
| miR-654-5p | 53% | miR-3158-5p | 73% | miR-6788-5p | 69% |
| miR-657 | 63% | miR-3162-3p | 77% | miR-6788-3p | 70% |
| miR-658 | 73% | miR-3173-5p | 62% | miR-6789-5p | 74% |
| miR-659-3p | 67% | miR-3187-5p | 68% | miR-6789-3p | 60% |
| miR-542-5p | 89% | miR-3189-5p | 74% | miR-6790-5p | 65% |
| miR-425-5p | 53% | miR-3619-3p | 81% | miR-6790-3p | 74% |
| miR-671-5p | 66% | miR-3691-3p | 52% | miR-6791-5p | 70% |
| miR-668-3p | 64% | miR-3150b-5p | 70% | miR-6792-5p | 67% |
| miR-769-3p | 54% | miR-3922-5p | 58% | miR-6792-3p | 77% |
| miR-766-3p | 77% | miR-3925-3p | 55% | miR-6793-5p | 66% |
| miR-765 | 74% | miR-3940-5p | 67% | miR-6793-3p | 75% |
| miR-675-5p | 61% | miR-4529-5p | 51% | miR-6794-5p | 85% |
| miR-297 | 59% | miR-3960 | 63% | miR-6794-3p | 78% |
| let-7b-3p | 68% | miR-3973 | 54% | miR-6795-5p | 74% |
| let-7d-3p | 60% | miR-3978 | 100% | miR-6795-3p | 71% |
| let-7f-1-3p | 67% | miR-4634 | 66% | miR-6796-5p | 74% |
| miR-22-5p | 100% | miR-4638-5p | 75% | miR-6796-3p | 78% |
| miR-24-2-5p | 64% | miR-4639-5p | 100% | miR-6797-5p | 76% |
| miR-25-5p | 58% | miR-4639-3p | 67% | miR-6797-3p | 74% |
| miR-26b-3p | 59% | miR-4640-5p | 69% | miR-6798-5p | 73% |
| miR-27a-5p | 67% | miR-4640-3p | 64% | miR-6798-3p | 77% |
| miR-29a-5p | 100% | miR-4642 | 76% | miR-6799-5p | 65% |
| miR-92a-1-5p | 100% | miR-4644 | 69% | miR-6799-3p | 66% |
| miR-92a-2-5p | 55% | miR-4646-5p | 75% | miR-6800-5p | 66% |
| miR-29b-1-5p | 63% | miR-4646-3p | 73% | miR-6800-3p | 75% |
| miR-30c-2-3p | 75% | miR-4647 | 58% | miR-6801-5p | 63% |
| miR-139-3p | 63% | miR-4648 | 57% | miR-6801-3p | 76% |
| miR-183-3p | 66% | miR-4649-5p | 65% | miR-6802-3p | 74% |
| miR-187-5p | 58% | miR-4649-3p | 72% | miR-6803-5p | 68% |
| miR-221-5p | 67% | miR-4650-3p | 80% | miR-6803-3p | 73% |
| miR-15b-3p | 62% | miR-4651 | 72% | miR-6804-3p | 69% |
| miR-124-5p | 67% | miR-4653-3p | 60% | miR-6805-5p | 70% |
| miR-125b-1-3p | 100% | miR-4654 | 58% | miR-6805-3p | 74% |
| miR-130a-5p | 60% | miR-4655-5p | 68% | miR-6806-5p | 65% |
| miR-132-5p | 83% | miR-4656 | 72% | miR-6807-5p | 69% |
| miR-135a-3p | 56% | miR-4657 | 55% | miR-6808-5p | 66% |
| miR-140-3p | 55% | miR-4664-5p | 65% | miR-6808-3p | 69% |
| miR-125a-3p | 74% | miR-4664-3p | 70% | miR-6809-5p | 81% |
| miR-125b-2-3p | 53% | miR-4665-5p | 83% | miR-6809-3p | 73% |
| miR-149-3p | 71% | miR-4665-3p | 73% | miR-6810-5p | 74% |
| miR-150-3p | 76% | miR-4667-5p | 79% | miR-6810-3p | 76% |
| miR-185-3p | 73% | miR-4667-3p | 73% | miR-6812-5p | 76% |
| miR-186-3p | 64% | miR-4668-5p | 100% | miR-6812-3p | 73% |
| miR-193a-5p | 63% | miR-4669 | 68% | miR-6813-5p | 66% |
| miR-194-3p | 63% | miR-4670-5p | 63% | miR-6813-3p | 74% |
| miR-30c-1-3p | 54% | miR-4670-3p | 100% | miR-6814-3p | 71% |
| miR-34c-3p | 53% | miR-4672 | 59% | miR-6815-5p | 69% |
| miR-99b-3p | 53% | miR-4673 | 52% | miR-6815-3p | 69% |
| miR-296-3p | 64% | miR-4674 | 68% | miR-6816-5p | 64% |
| miR-130b-5p | 59% | miR-4676-5p | 60% | miR-6816-3p | 68% |
| miR-361-3p | 70% | miR-4677-5p | 67% | miR-6818-5p | 67% |
| miR-302d-5p | 54% | miR-4682 | 52% | miR-6819-5p | 66% |
| miR-371a-5p | 67% | miR-4684-3p | 56% | miR-6819-3p | 74% |
| miR-374a-3p | 100% | miR-4685-5p | 71% | miR-6820-5p | 70% |
| miR-377-5p | 56% | miR-4685-3p | 69% | miR-6820-3p | 75% |
| miR-342-5p | 79% | miR-4686 | 53% | miR-6821-5p | 71% |
| miR-323a-5p | 64% | miR-4687-5p | 75% | miR-6822-5p | 67% |
| miR-338-5p | 51% | miR-4687-3p | 63% | miR-6822-3p | 55% |
| miR-339-3p | 73% | miR-1343-3p | 71% | miR-6823-5p | 65% |
| miR-423-5p | 65% | miR-4688 | 66% | miR-6823-3p | 77% |
| miR-424-3p | 67% | miR-4689 | 63% | miR-6824-5p | 72% |
| miR-18b-3p | 74% | miR-4690-5p | 71% | miR-6825-5p | 74% |
| miR-20b-3p | 72% | miR-4691-5p | 58% | miR-6826-3p | 73% |
| miR-431-3p | 65% | miR-4691-3p | 83% | miR-6827-5p | 84% |
| miR-483-5p | 76% | miR-4692 | 54% | miR-6827-3p | 75% |
| miR-486-3p | 70% | miR-4695-5p | 72% | miR-6828-5p | 77% |
| miR-490-5p | 100% | miR-4695-3p | 72% | miR-6829-5p | 73% |
| miR-146b-3p | 52% | miR-4697-5p | 67% | miR-6829-3p | 71% |
| miR-193b-5p | 52% | miR-4697-3p | 72% | miR-6830-5p | 81% |
| miR-502-3p | 83% | miR-4698 | 73% | miR-6830-3p | 73% |
| miR-505-5p | 74% | miR-4700-5p | 56% | miR-6831-5p | 72% |
| miR-509-5p | 64% | miR-4700-3p | 69% | miR-6831-3p | 66% |
| miR-532-3p | 68% | miR-4701-5p | 73% | miR-6832-5p | 77% |
| miR-92b-5p | 75% | miR-4701-3p | 65% | miR-6832-3p | 57% |
| miR-551b-5p | 57% | miR-4703-5p | 100% | miR-6833-5p | 72% |
| miR-574-5p | 65% | miR-4704-3p | 100% | miR-6833-3p | 69% |
| miR-582-3p | 100% | miR-4706 | 78% | miR-6834-5p | 81% |
| miR-550a-5p | 77% | miR-4707-5p | 71% | miR-6834-3p | 72% |
| miR-615-5p | 78% | miR-4707-3p | 72% | miR-6780b-5p | 71% |
| miR-625-3p | 72% | miR-4708-5p | 54% | miR-6780b-3p | 75% |
| miR-629-5p | 83% | miR-4708-3p | 60% | miR-6836-3p | 79% |
| miR-671-3p | 54% | miR-4709-3p | 70% | miR-6837-5p | 65% |
| miR-890 | 100% | miR-203b-5p | 61% | miR-6838-5p | 55% |
| miR-891b | 80% | miR-203b-3p | 75% | miR-6839-5p | 60% |
| miR-541-3p | 53% | miR-4710 | 70% | miR-6840-5p | 70% |
| miR-875-5p | 100% | miR-4712-3p | 58% | miR-6840-3p | 73% |
| miR-708-3p | 100% | miR-4713-5p | 69% | miR-6841-3p | 69% |
| miR-744-5p | 61% | miR-4714-5p | 67% | miR-6842-5p | 76% |
| miR-744-3p | 51% | miR-4716-5p | 76% | miR-6842-3p | 51% |
| miR-885-5p | 66% | miR-4716-3p | 74% | miR-6845-5p | 69% |
| miR-885-3p | 65% | miR-4717-3p | 62% | miR-6845-3p | 79% |
| miR-877-5p | 70% | miR-4720-5p | 57% | miR-6846-5p | 74% |
| miR-877-3p | 78% | miR-4721 | 71% | miR-6846-3p | 74% |
| miR-887-3p | 68% | miR-4722-5p | 70% | miR-6847-5p | 62% |
| miR-665 | 68% | miR-4722-3p | 69% | miR-6847-3p | 70% |
| miR-760 | 63% | miR-4723-5p | 75% | miR-6848-5p | 71% |
| miR-920 | 80% | miR-4723-3p | 74% | miR-6848-3p | 73% |
| miR-921 | 55% | miR-4724-5p | 54% | miR-6849-5p | 78% |
| miR-922 | 100% | miR-4725-5p | 60% | miR-6849-3p | 66% |
| miR-933 | 73% | miR-4725-3p | 73% | miR-6850-5p | 73% |
| miR-936 | 74% | miR-4726-5p | 62% | miR-6851-5p | 66% |
| miR-939-5p | 76% | miR-4726-3p | 53% | miR-6851-3p | 72% |
| miR-940 | 69% | miR-4728-5p | 69% | miR-6852-3p | 56% |
| miR-1224-5p | 75% | miR-4728-3p | 72% | miR-6854-3p | 55% |
| miR-1224-3p | 78% | miR-4729 | 100% | miR-6855-5p | 63% |
| miR-1225-5p | 70% | miR-4730 | 58% | miR-6855-3p | 72% |
| miR-1225-3p | 72% | miR-4731-5p | 65% | miR-6856-5p | 63% |
| miR-1226-3p | 55% | miR-4731-3p | 70% | miR-6857-5p | 70% |
| miR-1227-3p | 68% | miR-4732-5p | 80% | miR-6857-3p | 74% |
| miR-1228-5p | 63% | miR-4732-3p | 70% | miR-6858-5p | 68% |
| miR-1228-3p | 76% | miR-4734 | 74% | miR-6858-3p | 75% |
| miR-1229-3p | 75% | miR-4735-3p | 100% | miR-6859-5p | 61% |
| miR-1231 | 69% | miR-4736 | 58% | miR-6859-3p | 72% |
| miR-1233-3p | 73% | miR-4737 | 52% | miR-6769b-5p | 71% |
| miR-1234-3p | 71% | miR-3064-5p | 61% | miR-6769b-3p | 64% |
| miR-1236-3p | 80% | miR-3064-3p | 59% | miR-6860 | 68% |
| miR-1237-3p | 75% | miR-4738-3p | 56% | miR-6861-5p | 69% |
| miR-1238-3p | 76% | miR-4739 | 62% | miR-6861-3p | 77% |
| miR-320b | 64% | miR-4741 | 83% | miR-6862-5p | 62% |
| miR-320c | 70% | miR-4743-5p | 68% | miR-6862-3p | 75% |
| miR-1298-5p | 61% | miR-122b-5p | 100% | miR-6864-5p | 57% |
| miR-1180-3p | 100% | miR-122b-3p | 55% | miR-6865-5p | 70% |
| miR-1182 | 82% | miR-4745-5p | 70% | miR-6865-3p | 73% |
| miR-1184 | 60% | miR-4746-5p | 100% | miR-6866-5p | 100% |
| miR-1202 | 70% | miR-4746-3p | 65% | miR-6867-5p | 70% |
| miR-1203 | 59% | miR-4747-5p | 79% | miR-6867-3p | 69% |
| miR-1204 | 53% | miR-4748 | 67% | miR-6868-5p | 53% |
| miR-1207-5p | 84% | miR-4749-5p | 70% | miR-6868-3p | 68% |
| miR-1207-3p | 56% | miR-4749-3p | 76% | miR-6869-5p | 62% |
| miR-1285-3p | 53% | miR-4750-5p | 67% | miR-6870-5p | 70% |
| miR-1289 | 83% | miR-4751 | 75% | miR-6870-3p | 73% |
| miR-1290 | 100% | miR-4752 | 52% | miR-6871-5p | 67% |
| miR-1293 | 62% | miR-4753-5p | 72% | miR-6872-5p | 69% |
| miR-1294 | 68% | miR-371b-5p | 70% | miR-6873-5p | 61% |
| miR-1304-5p | 67% | miR-371b-3p | 68% | miR-6874-5p | 53% |
| miR-1246 | 100% | miR-4754 | 62% | miR-6875-5p | 60% |
| miR-1249-3p | 77% | miR-4755-3p | 69% | miR-6875-3p | 69% |
| miR-1257 | 55% | miR-499b-3p | 56% | miR-6876-5p | 76% |
| miR-1260a | 74% | miR-4756-5p | 68% | miR-6876-3p | 57% |
| miR-1261 | 100% | miR-4758-5p | 79% | miR-6877-5p | 77% |
| miR-1266-5p | 59% | miR-4758-3p | 71% | miR-6877-3p | 72% |
| miR-1267 | 69% | miR-4759 | 100% | miR-6878-5p | 59% |
| miR-1268a | 65% | miR-4762-3p | 75% | miR-6878-3p | 70% |
| miR-1270 | 61% | miR-4763-5p | 71% | miR-6879-5p | 73% |
| miR-1272 | 57% | miR-4763-3p | 71% | miR-6879-3p | 69% |
| miR-1278 | 100% | miR-4764-5p | 100% | miR-6880-5p | 74% |
| miR-1281 | 78% | miR-4766-5p | 57% | miR-6880-3p | 77% |
| miR-1292-5p | 86% | miR-4769-5p | 78% | miR-6881-5p | 74% |
| miR-1255b-5p | 100% | miR-4769-3p | 75% | miR-6881-3p | 68% |
| miR-664a-3p | 69% | miR-4771 | 53% | miR-6882-3p | 69% |
| miR-1306-3p | 55% | miR-4776-5p | 75% | miR-6883-5p | 68% |
| miR-1307-3p | 77% | miR-4776-3p | 56% | miR-6883-3p | 72% |
| miR-320d | 83% | miR-4778-5p | 73% | miR-6884-5p | 54% |
| miR-1825 | 77% | miR-4778-3p | 67% | miR-6884-3p | 76% |
| miR-1827 | 100% | miR-4779 | 75% | miR-6885-5p | 61% |
| miR-1468-5p | 51% | miR-4780 | 56% | miR-6885-3p | 76% |
| miR-675-3p | 68% | miR-4436b-5p | 72% | miR-6886-5p | 53% |
| miR-1469 | 69% | miR-4436b-3p | 58% | miR-6886-3p | 81% |
| miR-1470 | 78% | miR-4781-5p | 100% | miR-6887-5p | 74% |
| miR-1471 | 70% | miR-4781-3p | 100% | miR-6887-3p | 76% |
| miR-1538 | 56% | miR-4783-3p | 77% | miR-6889-5p | 78% |
| miR-1539 | 65% | miR-4784 | 61% | miR-6889-3p | 77% |
| miR-1908-5p | 67% | miR-2467-3p | 79% | miR-6890-5p | 68% |
| miR-1909-5p | 64% | miR-4787-5p | 67% | miR-6890-3p | 72% |
| miR-1909-3p | 68% | miR-4787-3p | 77% | miR-6891-5p | 71% |
| miR-1910-5p | 72% | miR-4788 | 66% | miR-6891-3p | 77% |
| miR-1911-5p | 100% | miR-4791 | 100% | miR-6892-5p | 73% |
| miR-1912-3p | 69% | miR-4793-3p | 69% | miR-6892-3p | 73% |
| miR-1913 | 76% | miR-4795-5p | 100% | miR-6893-5p | 81% |
| miR-1914-3p | 87% | miR-4795-3p | 100% | miR-6893-3p | 67% |
| miR-1915-3p | 65% | miR-4800-5p | 80% | miR-6894-5p | 71% |
| miR-205-3p | 67% | miR-4802-5p | 100% | miR-6894-3p | 75% |
| miR-365a-5p | 77% | miR-4802-3p | 71% | miR-6895-3p | 53% |
| miR-449b-3p | 76% | miR-4804-3p | 58% | miR-7106-5p | 78% |
| miR-2113 | 60% | miR-4520-2-3p | 100% | miR-7106-3p | 64% |
| miR-1972 | 51% | miR-642a-3p | 67% | miR-7107-5p | 81% |
| miR-1976 | 76% | miR-550a-3-5p | 58% | miR-7108-5p | 71% |
| miR-2110 | 79% | miR-4433a-5p | 76% | miR-7108-3p | 74% |
| miR-151b | 53% | miR-4482-3p | 65% | miR-7109-5p | 70% |
| miR-762 | 69% | miR-4999-5p | 62% | miR-7109-3p | 70% |
| miR-670-5p | 60% | miR-5000-3p | 55% | miR-7110-5p | 73% |
| miR-764 | 72% | miR-5001-5p | 66% | miR-7110-3p | 75% |
| miR-2116-3p | 71% | miR-5001-3p | 53% | miR-7111-5p | 79% |
| miR-548q | 64% | miR-5002-3p | 63% | miR-7111-3p | 79% |
| miR-2276-3p | 78% | miR-5003-3p | 67% | miR-7112-5p | 64% |
| miR-2278 | 65% | miR-5004-5p | 58% | miR-7113-5p | 69% |
| miR-711 | 76% | miR-5006-5p | 53% | miR-7113-3p | 74% |
| miR-718 | 76% | miR-5006-3p | 54% | miR-7114-5p | 72% |
| miR-2681-3p | 63% | miR-5008-5p | 61% | miR-7114-3p | 78% |
| miR-449c-3p | 59% | miR-5009-5p | 100% | miR-7150 | 86% |
| miR-2861 | 65% | miR-5009-3p | 61% | miR-7151-3p | 72% |
| miR-3115 | 100% | miR-5010-5p | 69% | miR-7152-5p | 69% |
| miR-3116 | 67% | miR-5010-3p | 71% | miR-7152-3p | 59% |
| miR-3117-3p | 100% | miR-5087 | 59% | miR-7153-5p | 100% |
| miR-3120-3p | 59% | miR-5088-5p | 64% | miR-7154-5p | 100% |
| miR-3122 | 53% | miR-5089-5p | 62% | miR-7155-5p | 67% |
| miR-3125 | 53% | miR-5090 | 66% | miR-7157-3p | 72% |
| miR-3126-5p | 61% | miR-5093 | 59% | miR-7159-5p | 71% |
| miR-3130-3p | 52% | miR-5188 | 54% | miR-7159-3p | 100% |
| miR-3130-5p | 66% | miR-5189-5p | 68% | miR-7160-5p | 71% |
| miR-3131 | 80% | miR-5193 | 71% | miR-7161-5p | 52% |
| miR-3132 | 70% | miR-5195-5p | 70% | miR-7162-5p | 53% |
| miR-3133 | 56% | miR-5195-3p | 65% | miR-7515 | 72% |
| miR-466 | 58% | miR-5196-5p | 75% | miR-7702 | 63% |
| miR-3136-5p | 100% | miR-5196-3p | 69% | miR-7704 | 66% |
| miR-3137 | 54% | miR-5197-3p | 100% | miR-7843-5p | 63% |
| miR-3138 | 73% | miR-4524b-5p | 53% | miR-4433b-5p | 73% |
| miR-3141 | 75% | miR-5100 | 56% | miR-4433b-3p | 79% |
| miR-3144-5p | 74% | miR-5572 | 78% | miR-1273h-5p | 67% |
| miR-1273c | 67% | miR-5579-5p | 100% | miR-1273h-3p | 54% |
| miR-3147 | 80% | miR-664b-5p | 55% | miR-6516-5p | 53% |
| miR-548v | 100% | miR-664b-3p | 67% | miR-7845-5p | 74% |
| miR-3148 | 61% | miR-548at-5p | 56% | miR-7846-3p | 76% |
| miR-3150a-3p | 76% | miR-548at-3p | 71% | miR-7847-3p | 72% |
| miR-3151-5p | 67% | miR-5583-3p | 100% | miR-7851-3p | 63% |
| miR-3153 | 83% | miR-5586-3p | 57% | miR-7855-5p | 65% |
| miR-3154 | 76% | miR-5588-5p | 54% | miR-7856-5p | 57% |
| miR-3156-5p | 78% | miR-5589-5p | 65% | miR-8052 | 70% |
| miR-3157-5p | 55% | miR-5684 | 69% | miR-8054 | 80% |
| miR-3160-3p | 73% | miR-5690 | 64% | miR-8059 | 69% |
| miR-3162-5p | 70% | miR-4666b | 60% | miR-8060 | 70% |
| miR-3163 | 75% | miR-5698 | 70% | miR-8062 | 58% |
| miR-3164 | 66% | miR-5703 | 67% | miR-8063 | 67% |
| miR-3165 | 63% | miR-5705 | 57% | miR-8064 | 51% |
| miR-1260b | 74% | miR-5708 | 51% | miR-8069 | 67% |
| miR-3168 | 73% | miR-197-5p | 71% | miR-8070 | 57% |
| miR-3169 | 62% | miR-204-3p | 77% | miR-8071 | 81% |
| miR-3173-3p | 64% | miR-211-3p | 66% | miR-8072 | 68% |
| miR-1193 | 78% | miR-301a-5p | 56% | miR-8073 | 74% |
| miR-3176 | 61% | miR-345-3p | 66% | miR-8074 | 63% |
| miR-3177-3p | 82% | miR-652-5p | 74% | miR-8075 | 61% |
| miR-3178 | 68% | miR-1185-2-3p | 75% | miR-8077 | 70% |
| miR-3180-5p | 70% | miR-766-5p | 65% | miR-8079 | 57% |
| miR-3180-3p | 65% | miR-873-3p | 61% | miR-8080 | 62% |
| miR-3183 | 100% | miR-1304-3p | 73% | miR-8085 | 79% |
| miR-3184-5p | 63% | miR-1247-3p | 69% | miR-8087 | 75% |
| miR-3185 | 75% | miR-548g-5p, miR-548x-5p, miR-548aj-5p | 100% | miR-8089 | 75% |
| miR-3186-5p | 53% | miR-1306-5p | 79% | miR-128-2-5p | 69% |
| miR-3186-3p | 53% | miR-3184-3p | 75% | miR-548ba | 61% |
| miR-3189-3p | 73% | miR-3191-5p | 73% | miR-7976 | 53% |
| miR-3190-5p | 69% | miR-642b-5p | 63% | miR-7977 | 72% |
| miR-3191-3p | 77% | miR-365b-5p | 64% | miR-7978 | 100% |
| miR-3194-5p | 67% | miR-1185-1-3p | 76% | miR-203a-5p | 64% |
| miR-3195 | 59% | miR-3190-3p | 56% | miR-1-5p | 52% |
| miR-3196 | 68% | miR-381-5p | 55% | miR-1249-5p | 72% |
| miR-3197 | 72% | miR-503-3p | 68% | miR-4485-5p | 76% |
| miR-3198 | 61% | miR-758-5p | 53% | miR-8485 | 71% |
| miR-514b-5p | 70% | miR-937-5p | 67% | miR-9500 | 51% |
| miR-4296 | 61% | miR-939-3p | 73% | miR-135a-2-3p | 60% |
| miR-4297 | 67% | miR-1227-5p | 71% | miR-498-3p | 68% |
| miR-4293 | 64% | miR-1229-5p | 67% | miR-526a-3p | 100% |
| miR-4294 | 74% | miR-1233-5p | 70% | miR-9718 | 71% |
| miR-4299 | 67% | miR-1237-5p | 69% | miR-9898 | 72% |
| miR-4298 | 73% | miR-1238-5p | 79% | miR-9900 | 100% |
| miR-4305 | 65% | miR-1292-3p | 68% | miR-9985 | 60% |
| miR-4306 | 68% | miR-3617-3p | 53% | miR-1843 | 57% |
| miR-4307 | 71% | miR-3620-5p | 66% | miR-9986 | 53% |
| miR-4312 | 76% | miR-4632-5p | 69% | miR-10392-5p | 70% |
| miR-4313 | 71% | miR-4743-3p | 58% | miR-10392-3p | 81% |
| miR-4316 | 66% | miR-4750-3p | 71% | miR-10394-3p | 63% |
| miR-4314 | 59% | miR-5739 | 73% | miR-10396a-5p | 66% |
| miR-4320 | 54% | miR-5787 | 65% | miR-10396a-3p | 65% |
| miR-4322 | 59% | miR-6068 | 53% | miR-10398-5p | 67% |
| miR-4321 | 61% | miR-6069 | 69% | miR-10398-3p | 76% |
| miR-4323 | 74% | miR-6071 | 53% | miR-10400-5p | 66% |
| miR-4324 | 60% | miR-6072 | 67% | miR-10400-3p | 66% |
| miR-4256 | 60% | miR-6074 | 58% | miR-10401-5p | 81% |
| miR-4257 | 78% | miR-6075 | 68% | miR-10401-3p | 75% |
| miR-4258 | 72% | miR-6076 | 83% | miR-10396b-5p | 71% |
| miR-4259 | 75% | miR-6077 | 60% | miR-10522-5p | 85% |
| miR-4260 | 71% | miR-6079 | 100% | miR-9983-3p | 100% |
| miR-4253 | 59% | miR-6083 | 53% | miR-10526-3p | 86% |
| miR-4251 | 53% | miR-6085 | 75% | miR-10527-5p | 79% |
| miR-4254 | 54% | miR-6086 | 83% | miR-11181-5p | 69% |
| miR-4252 | 51% | miR-6088 | 67% | miR-11181-3p | 74% |
| miR-4325 | 67% | miR-6089 | 71% | miR-11399 | 67% |
| miR-4326 | 69% | miR-6090 | 68% | miR-11400 | 65% |
| miR-4327 | 71% | miR-6124 | 74% | miR-11401 | 64% |
| miR-4261 | 52% | miR-6125 | 70% | miR-3059-5p | 72% |
| miR-4265 | 66% | miR-6126 | 61% | miR-3059-3p | 59% |
| miR-4266 | 63% | miR-6127 | 74% | miR-3085-5p | 67% |
| miR-2355-5p | 71% | miR-378j | 59% | miR-3085-3p | 72% |
| miR-4268 | 70% | miR-6130 | 58% | miR-6529-5p | 70% |
| miR-4269 | 74% | miR-6131 | 73% | miR-12114 | 61% |
| miR-4270 | 70% | miR-6132 | 62% | miR-12115 | 70% |
| miR-4271 | 68% | miR-6133 | 81% | miR-12116 | 76% |
| miR-4276 | 60% | miR-6134 | 53% | miR-12118 | 76% |
| miR-4274 | 76% | miR-6165 | 73% | miR-12119 | 79% |
| miR-4281 | 67% | miR-6500-5p | 58% | miR-12120 | 67% |
| miR-4279 | 78% | miR-6500-3p | 56% | miR-12121 | 66% |
| miR-4278 | 58% | miR-6501-3p | 61% | miR-12122 | 53% |
| miR-4280 | 59% | miR-6503-5p | 67% | miR-12124 | 52% |
| miR-4285 | 60% | miR-6506-5p | 60% | miR-12126 | 80% |
| miR-4283 | 51% | miR-6506-3p | 75% | miR-12128 | 66% |
| miR-4284 | 70% | miR-6508-5p | 62% | miR-12131 | 100% |
| miR-4286 | 72% | miR-6509-3p | 64% | miR-12136 | 66% |
| miR-4287 | 67% | miR-6510-5p | 71% | | |

| | | | | | |
|---|---|---|---|---|---|
| ^{∗} A represents Accuracy. ^{∗} "has-" in the miRNA names has been omitted. | | | | | |

### [Table 23-6]

**Table 23-6: miRNA to Predict Human Prostate Cancer of Stages I and II**

| miRNA Name | A | miRNA Name | A | miRNA Name | A |
|---|---|---|---|---|---|
| miR-28-5p | 52% | miR-3622b-5p | 63% | miR-6717-5p | 56% |
| miR-29a-3p | 59% | miR-3648 | 75% | miR-6511b-5p | 63% |
| miR-93-5p | 63% | miR-3649 | 60% | miR-6719-3p | 60% |
| miR-105-5p | 61% | miR-3651 | 53% | miR-6721-5p | 63% |
| miR-198 | 65% | miR-3652 | 56% | miR-6722-5p | 62% |
| miR-129-5p | 59% | miR-3660 | 52% | miR-6722-3p | 63% |
| miR-134-5p | 57% | miR-3663-5p | 57% | miR-6724-5p | 63% |
| miR-184 | 53% | miR-3665 | 62% | miR-128-1-5p | 62% |
| miR-185-5p | 68% | miR-3666 | 56% | miR-433-5p | 53% |
| miR-296-5p | 61% | miR-3667-5p | 56% | miR-181d-3p | 75% |
| miR-130b-3p | 80% | miR-3667-3p | 60% | miR-504-3p | 58% |
| miR-302c-5p | 61% | miR-3675-3p | 63% | miR-487b-5p | 54% |
| miR-370-3p | 70% | miR-3677-3p | 70% | miR-585-5p | 51% |
| miR-378a-3p | 61% | miR-3679-5p | 68% | miR-605-3p | 64% |
| miR-20b-5p | 71% | miR-3679-3p | 68% | miR-668-5p | 60% |
| miR-429 | 100% | miR-3909 | 53% | miR-1296-3p | 69% |
| miR-485-5p | 55% | miR-3917 | 59% | miR-1301-5p | 54% |
| miR-491-5p | 70% | miR-3918 | 58% | miR-1251-3p | 52% |
| miR-492 | 55% | miR-3925-5p | 72% | miR-1266-3p | 52% |
| miR-494-3p | 51% | miR-3928-3p | 66% | miR-1343-5p | 64% |
| miR-512-5p | 54% | miR-3937 | 69% | miR-6726-5p | 69% |
| miR-498-5p | 68% | miR-3943 | 63% | miR-6727-5p | 60% |
| miR-520e-3p | 56% | miR-3944-3p | 57% | miR-6729-5p | 63% |
| miR-519b-3p | 53% | miR-3945 | 57% | miR-6729-3p | 64% |
| miR-525-5p | 52% | miR-1268b | 68% | miR-6730-5p | 66% |
| miR-518f-3p | 53% | miR-4421 | 60% | miR-6731-5p | 53% |
| miR-520b-3p | 59% | miR-4428 | 61% | miR-6731-3p | 63% |
| miR-518b | 63% | miR-4429 | 62% | miR-6732-5p | 61% |
| miR-520c-3p | 60% | miR-4430 | 62% | miR-6734-5p | 71% |
| miR-524-5p | 51% | miR-4433a-3p | 68% | miR-6737-5p | 61% |
| miR-520d-5p | 70% | miR-4435 | 56% | miR-6739-3p | 51% |
| miR-501-5p | 57% | miR-4439 | 56% | miR-6740-5p | 57% |
| miR-513a-5p | 51% | miR-4440 | 52% | miR-6740-3p | 61% |
| miR-572 | 56% | miR-4441 | 61% | miR-6741-5p | 67% |
| miR-575 | 61% | miR-4444 | 59% | miR-6742-5p | 70% |
| miR-580-3p | 62% | miR-4446-3p | 60% | miR-6743-5p | 65% |
| miR-583 | 55% | miR-4447 | 62% | miR-6744-5p | 57% |
| miR-584-5p | 55% | miR-4448 | 59% | miR-6745 | 64% |
| miR-585-3p | 60% | miR-4449 | 58% | miR-6746-5p | 61% |
| miR-589-3p | 56% | miR-4455 | 53% | miR-6747-5p | 55% |
| miR-601 | 55% | miR-4456 | 51% | miR-6748-5p | 71% |
| miR-608 | 53% | miR-4460 | 83% | miR-6749-5p | 67% |
| miR-614 | 59% | miR-3135b | 58% | miR-6750-5p | 60% |
| miR-615-3p | 53% | miR-4463 | 63% | miR-6751-5p | 63% |
| miR-622 | 51% | miR-4465 | 62% | miR-6752-5p | 64% |
| miR-624-5p | 62% | miR-4466 | 62% | miR-6752-3p | 62% |
| miR-635 | 52% | miR-4467 | 59% | miR-6753-5p | 55% |
| miR-638 | 64% | miR-4470 | 75% | miR-6754-5p | 62% |
| miR-644a | 51% | miR-4472 | 65% | miR-6756-3p | 66% |
| miR-650 | 52% | miR-4476 | 58% | miR-6759-5p | 61% |
| miR-663a | 63% | miR-4479 | 57% | miR-6760-5p | 55% |
| miR-654-5p | 65% | miR-4481 | 61% | miR-6760-3p | 67% |
| miR-658 | 63% | miR-4483 | 57% | miR-6762-5p | 63% |
| miR-542-5p | 83% | miR-4484 | 66% | miR-6763-5p | 69% |
| miR-769-3p | 68% | miR-4486 | 60% | miR-6763-3p | 65% |
| miR-770-5p | 62% | miR-4487 | 61% | miR-6766-5p | 59% |
| let-7d-3p | 56% | miR-4488 | 63% | miR-6766-3p | 64% |
| miR-25-5p | 55% | miR-4489 | 57% | miR-6768-5p | 67% |
| miR-29a-5p | 100% | miR-4496 | 76% | miR-6769a-5p | 67% |
| miR-92a-2-5p | 62% | miR-4497 | 62% | miR-6770-5p | 54% |
| miR-30c-2-3p | 55% | miR-4498 | 57% | miR-6772-5p | 62% |
| miR-139-3p | 56% | miR-4499 | 66% | miR-6772-3p | 51% |
| miR-181a-2-3p | 51% | miR-4505 | 64% | miR-6774-3p | 55% |
| miR-183-3p | 71% | miR-4506 | 57% | miR-6776-5p | 62% |
| miR-187-5p | 59% | miR-4507 | 64% | miR-6777-5p | 72% |
| miR-214-5p | 53% | miR-4508 | 62% | miR-6777-3p | 61% |
| miR-30b-3p | 69% | miR-4512 | 71% | miR-6778-5p | 60% |
| miR-135a-3p | 53% | miR-4513 | 61% | miR-6781-5p | 67% |
| miR-125a-3p | 62% | miR-4514 | 56% | miR-6782-5p | 63% |
| miR-149-3p | 62% | miR-4516 | 64% | miR-6783-5p | 57% |
| miR-150-3p | 72% | miR-4518 | 63% | miR-6783-3p | 52% |
| miR-185-3p | 60% | miR-4519 | 100% | miR-6784-5p | 65% |
| miR-193a-5p | 54% | miR-4521 | 62% | miR-6784-3p | 63% |
| miR-194-3p | 51% | miR-4525 | 59% | miR-6785-3p | 63% |
| miR-34c-3p | 51% | miR-4526 | 60% | miR-6786-5p | 62% |
| miR-296-3p | 68% | miR-4530 | 66% | miR-6787-5p | 62% |
| miR-361-3p | 63% | miR-4531 | 61% | miR-6788-5p | 55% |
| miR-302d-5p | 51% | miR-4533 | 65% | miR-6789-5p | 64% |
| miR-374a-3p | 100% | miR-4534 | 67% | miR-6789-3p | 56% |
| miR-342-5p | 66% | miR-4535 | 58% | miR-6790-3p | 63% |
| miR-323a-5p | 59% | miR-1587 | 61% | miR-6791-5p | 66% |
| miR-338-5p | 58% | miR-4538 | 54% | miR-6792-5p | 51% |
| miR-339-3p | 57% | miR-4539 | 61% | miR-6794-5p | 69% |
| miR-424-3p | 56% | miR-4540 | 51% | miR-6795-5p | 59% |
| miR-18b-3p | 63% | miR-3127-3p | 65% | miR-6795-3p | 64% |
| miR-20b-3p | 56% | miR-3150a-5p | 55% | miR-6796-5p | 59% |
| miR-483-5p | 61% | miR-3152-5p | 54% | miR-6796-3p | 69% |
| miR-486-3p | 58% | miR-3158-5p | 62% | miR-6797-5p | 62% |
| miR-193b-5p | 58% | miR-3162-3p | 64% | miR-6798-5p | 59% |
| miR-505-5p | 60% | miR-3173-5p | 52% | miR-6798-3p | 63% |
| miR-92b-5p | 63% | miR-3187-5p | 65% | miR-6800-5p | 61% |
| miR-551b-5p | 57% | miR-3619-3p | 63% | miR-6800-3p | 64% |
| miR-574-5p | 53% | miR-3150b-5p | 65% | miR-6801-5p | 52% |
| miR-550a-5p | 65% | miR-3922-5p | 54% | miR-6805-5p | 63% |
| miR-615-5p | 73% | miR-3940-5p | 64% | miR-6805-3p | 65% |
| miR-625-3p | 63% | miR-4529-5p | 56% | miR-6806-5p | 53% |
| miR-629-5p | 83% | miR-3960 | 60% | miR-6808-3p | 64% |
| miR-411-3p | 54% | miR-4635 | 60% | miR-6809-5p | 67% |
| miR-541-3p | 55% | miR-4638-5p | 67% | miR-6810-5p | 64% |
| miR-885-5p | 53% | miR-4640-5p | 65% | miR-6810-3p | 61% |
| miR-885-3p | 56% | miR-4648 | 55% | miR-6812-5p | 67% |
| miR-877-5p | 59% | miR-4651 | 65% | miR-6812-3p | 63% |
| miR-887-3p | 62% | miR-4652-5p | 64% | miR-6813-5p | 60% |
| miR-665 | 67% | miR-4654 | 52% | miR-6813-3p | 59% |
| miR-760 | 64% | miR-4655-5p | 59% | miR-6815-5p | 65% |
| miR-920 | 68% | miR-4656 | 65% | miR-6816-5p | 63% |
| miR-933 | 65% | miR-4658 | 54% | miR-6816-3p | 72% |
| miR-936 | 56% | miR-4664-5p | 59% | miR-6819-3p | 64% |
| miR-939-5p | 68% | miR-4665-5p | 69% | miR-6820-5p | 65% |
| miR-940 | 62% | miR-4665-3p | 64% | miR-6821-5p | 65% |
| miR-1224-5p | 58% | miR-4667-5p | 66% | miR-6821-3p | 53% |
| miR-1224-3p | 61% | miR-219b-5p | 52% | miR-6822-5p | 58% |
| miR-1225-5p | 61% | miR-4669 | 57% | miR-6823-5p | 53% |
| miR-1225-3p | 63% | miR-4673 | 58% | miR-6825-5p | 68% |
| miR-1226-5p | 56% | miR-4674 | 59% | miR-6827-5p | 67% |
| miR-1228-3p | 64% | miR-4677-5p | 67% | miR-6828-5p | 72% |
| miR-1231 | 63% | miR-4684-3p | 58% | miR-6829-5p | 72% |
| miR-1237-3p | 64% | miR-1343-3p | 65% | miR-6830-5p | 67% |
| miR-1238-3p | 61% | miR-4688 | 61% | miR-6831-5p | 68% |
| miR-1301-3p | 53% | miR-4691-5p | 60% | miR-6832-5p | 58% |
| miR-1182 | 53% | miR-4695-5p | 62% | miR-6833-5p | 62% |
| miR-1184 | 54% | miR-4701-3p | 53% | miR-6834-5p | 60% |
| miR-1203 | 64% | miR-4706 | 57% | miR-6780b-5p | 70% |
| miR-1204 | 51% | miR-4707-5p | 65% | miR-6836-3p | 63% |
| miR-1207-5p | 59% | miR-4707-3p | 68% | miR-6840-3p | 59% |
| miR-1289 | 63% | miR-4708-5p | 54% | miR-6842-5p | 62% |
| miR-1291 | 51% | miR-4708-3p | 55% | miR-6845-5p | 66% |
| miR-1303 | 55% | miR-4710 | 57% | miR-6846-5p | 59% |
| miR-1249-3p | 63% | miR-4714-5p | 62% | miR-6848-5p | 58% |
| miR-1250-5p | 52% | miR-4716-5p | 66% | miR-6848-3p | 63% |
| miR-1263 | 57% | miR-4716-3p | 55% | miR-6849-5p | 62% |
| miR-1268a | 64% | miR-4717-5p | 51% | miR-6850-5p | 63% |
| miR-1292-5p | 75% | miR-4717-3p | 52% | miR-6853-5p | 56% |
| miR-1307-3p | 68% | miR-4721 | 56% | miR-6855-5p | 53% |
| miR-1324 | 55% | miR-4723-5p | 62% | miR-6856-5p | 52% |
| miR-1469 | 66% | miR-4725-5p | 52% | miR-6856-3p | 60% |
| miR-1470 | 66% | miR-4725-3p | 70% | miR-6857-5p | 67% |
| miR-1471 | 56% | miR-4726-3p | 53% | miR-6858-3p | 63% |
| miR-1538 | 54% | miR-4727-3p | 51% | miR-6859-3p | 63% |
| miR-1908-5p | 65% | miR-4728-3p | 62% | miR-6860 | 54% |
| miR-1909-5p | 62% | miR-4731-3p | 63% | miR-6861-5p | 64% |
| miR-1909-3p | 67% | miR-4732-5p | 66% | miR-6862-5p | 52% |
| miR-1913 | 63% | miR-4733-3p | 51% | miR-6869-5p | 62% |
| miR-1914-3p | 67% | miR-4734 | 62% | miR-6870-3p | 61% |
| miR-1915-3p | 64% | miR-4736 | 56% | miR-6871-5p | 55% |
| miR-103a-2-5p | 53% | miR-4738-3p | 55% | miR-6872-5p | 64% |
| miR-365a-5p | 64% | miR-4740-5p | 56% | miR-6875-5p | 57% |
| miR-196b-3p | 55% | miR-4740-3p | 59% | miR-6875-3p | 53% |
| miR-1972 | 65% | miR-4741 | 69% | miR-6876-5p | 65% |
| miR-2110 | 68% | miR-4743-5p | 60% | miR-6877-5p | 71% |
| miR-449c-5p | 55% | miR-4745-5p | 64% | miR-6879-5p | 63% |
| miR-762 | 64% | miR-4746-3p | 58% | miR-6880-3p | 61% |
| miR-670-5p | 56% | miR-4747-5p | 70% | miR-6881-5p | 66% |
| miR-2116-5p | 69% | miR-4748 | 60% | miR-6882-3p | 55% |
| miR-548q | 57% | miR-4749-5p | 67% | miR-6886-5p | 53% |
| miR-2276-3p | 60% | miR-4749-3p | 63% | miR-6887-5p | 63% |
| miR-2278 | 55% | miR-4750-5p | 60% | miR-6887-3p | 65% |
| miR-711 | 73% | miR-4751 | 68% | miR-6889-5p | 72% |
| miR-718 | 67% | miR-371b-5p | 59% | miR-6891-3p | 63% |
| miR-2681-5p | 51% | miR-4754 | 52% | miR-6892-5p | 70% |
| miR-2909 | 53% | miR-4755-3p | 60% | miR-6892-3p | 64% |
| miR-3116 | 63% | miR-4758-5p | 74% | miR-6893-5p | 72% |
| miR-3122 | 66% | miR-4763-3p | 66% | miR-6894-5p | 60% |
| miR-3124-5p | 63% | miR-4769-5p | 58% | miR-6895-5p | 62% |
| miR-3126-5p | 63% | miR-4776-5p | 65% | miR-7106-5p | 69% |
| miR-3130-5p | 57% | miR-4778-5p | 56% | miR-7107-5p | 70% |
| miR-3131 | 70% | miR-4783-3p | 64% | miR-7108-5p | 58% |
| miR-3132 | 59% | miR-4785 | 53% | miR-7108-3p | 67% |
| miR-3137 | 58% | miR-2467-3p | 58% | miR-7109-5p | 58% |
| miR-3138 | 56% | miR-4793-3p | 61% | miR-7111-5p | 72% |
| miR-3147 | 58% | miR-4800-5p | 72% | miR-7114-5p | 59% |
| miR-3150a-3p | 63% | miR-550a-3-5p | 61% | miR-7114-3p | 63% |
| miR-3151-5p | 54% | miR-4433a-5p | 63% | miR-7150 | 66% |
| miR-3153 | 73% | miR-4482-3p | 60% | miR-7151-5p | 52% |
| miR-3154 | 69% | miR-5001-3p | 56% | miR-7152-5p | 51% |
| miR-3155a | 65% | miR-5002-5p | 51% | miR-7152-3p | 56% |
| miR-3156-5p | 70% | miR-5002-3p | 54% | miR-7156-3p | 68% |
| miR-3158-3p | 51% | miR-5006-5p | 62% | miR-7160-5p | 56% |
| miR-3162-5p | 60% | miR-5006-3p | 53% | miR-7704 | 58% |
| miR-3164 | 64% | miR-5009-3p | 59% | miR-7843-5p | 52% |
| miR-3170 | 51% | miR-5010-5p | 59% | miR-4433b-5p | 63% |
| miR-3173-3p | 64% | miR-5088-5p | 55% | miR-4433b-3p | 65% |
| miR-1193 | 62% | miR-5090 | 59% | miR-1273h-5p | 64% |
| miR-3176 | 55% | miR-5093 | 52% | miR-7846-3p | 62% |
| miR-3177-3p | 73% | miR-5189-5p | 56% | miR-7848-3p | 56% |
| miR-3178 | 62% | miR-5190 | 66% | miR-7851-3p | 60% |
| miR-3180-3p | 63% | miR-5196-5p | 69% | miR-7855-5p | 54% |
| miR-3185 | 73% | miR-5571-3p | 56% | miR-8052 | 59% |
| miR-3186-3p | 52% | miR-5572 | 67% | miR-8060 | 63% |
| miR-3187-3p | 52% | miR-5580-3p | 51% | miR-8063 | 60% |
| miR-3189-3p | 57% | miR-5585-3p | 59% | miR-8064 | 57% |
| miR-3191-3p | 61% | miR-5587-3p | 51% | miR-8069 | 63% |
| miR-3195 | 64% | miR-5589-5p | 54% | miR-8070 | 53% |
| miR-3196 | 62% | miR-5695 | 75% | miR-8071 | 60% |
| miR-3197 | 70% | miR-5703 | 66% | miR-8072 | 64% |
| miR-3199 | 53% | miR-5705 | 52% | miR-8073 | 65% |
| miR-4296 | 58% | miR-197-5p | 64% | miR-8075 | 54% |
| miR-4297 | 54% | miR-204-3p | 76% | miR-8077 | 54% |
| miR-4294 | 66% | miR-211-3p | 61% | miR-8078 | 53% |
| miR-4299 | 61% | miR-345-3p | 57% | miR-8085 | 71% |
| miR-4300 | 56% | miR-766-5p | 54% | miR-8087 | 79% |
| miR-4306 | 63% | miR-873-3p | 69% | miR-8089 | 66% |
| miR-4308 | 52% | miR-1247-3p | 71% | miR-7974 | 58% |
| miR-4311 | 52% | miR-550b-2-5p | 56% | miR-7977 | 52% |
| miR-4313 | 61% | miR-365b-5p | 61% | miR-8485 | 55% |
| miR-4315 | 52% | miR-937-5p | 65% | miR-103a-1-5p | 54% |
| miR-4316 | 52% | miR-1227-5p | 68% | miR-135a-2-3p | 56% |
| miR-4314 | 52% | miR-1236-5p | 55% | miR-526a-3p | 100% |
| miR-4322 | 56% | miR-1237-5p | 63% | miR-1912-5p | 60% |
| miR-4321 | 53% | miR-1238-5p | 65% | miR-9718 | 64% |
| miR-4323 | 62% | miR-3620-5p | 65% | miR-9898 | 63% |
| miR-4257 | 72% | miR-4750-3p | 68% | miR-10392-5p | 63% |
| miR-4258 | 67% | miR-5739 | 68% | miR-10392-3p | 64% |
| miR-4259 | 63% | miR-5787 | 63% | miR-10395-5p | 55% |
| miR-4260 | 61% | miR-6068 | 62% | miR-10396a-5p | 66% |
| miR-4253 | 55% | miR-6074 | 55% | miR-10396a-3p | 68% |
| miR-4251 | 54% | miR-6075 | 62% | miR-10398-3p | 68% |
| miR-4326 | 52% | miR-6076 | 67% | miR-10400-3p | 64% |
| miR-4327 | 63% | miR-6081 | 52% | miR-10401-5p | 77% |
| miR-4261 | 60% | miR-6084 | 57% | miR-10396b-5p | 63% |
| miR-4265 | 51% | miR-6085 | 64% | miR-10522-5p | 80% |
| miR-4269 | 65% | miR-6086 | 63% | miR-10526-3p | 69% |
| miR-4274 | 63% | miR-6089 | 65% | miR-11181-3p | 67% |
| miR-4283 | 57% | miR-6090 | 63% | miR-11399 | 64% |
| miR-4284 | 61% | miR-6124 | 61% | miR-11400 | 52% |
| miR-4286 | 66% | miR-6125 | 64% | miR-3059-3p | 55% |
| miR-4328 | 51% | miR-6127 | 75% | miR-3085-5p | 63% |
| miR-3200-5p | 60% | miR-6130 | 55% | miR-12114 | 54% |
| miR-3610 | 57% | miR-6131 | 72% | miR-12115 | 63% |
| miR-3612 | 63% | miR-6133 | 63% | miR-12116 | 65% |
| miR-3614-5p | 62% | miR-6165 | 65% | miR-12118 | 69% |
| miR-3616-3p | 62% | miR-6500-3p | 53% | miR-12119 | 69% |
| miR-3619-5p | 56% | miR-6503-3p | 57% | miR-12120 | 65% |
| miR-3621 | 65% | miR-6511a-5p | 62% | miR-12122 | 71% |
| miR-3622a-5p | 57% | miR-6515-5p | 60% | miR-12126 | 67% |
| miR-3622a-3p | 52% | miR-6515-3p | 64% | miR-12136 | 55% |

| | | | | | |
|---|---|---|---|---|---|
| ^{∗}A represents Accuracy. ^{∗}"has-" in the miRNA names has been omitted. | | | | | |

### [Table 23-7]

**Table 23-7: miRNA to Predict Human Stomach Cancer of Stages I and II**

| miRNA Name | A | miRNA Name | A | miRNA Name | A |
|---|---|---|---|---|---|
| miR-29a-3p | 61% | miR-4328 | 57% | miR-6717-5p | 63% |
| miR-29b-3p | 53% | miR-2277-5p | 61% | miR-6511b-5p | 72% |
| miR-198 | 72% | miR-3200-5p | 71% | miR-6511b-3p | 73% |
| miR-203a-3p | 57% | miR-3610 | 71% | miR-6719-3p | 60% |
| miR-214-3p | 56% | miR-3612 | 64% | miR-6721-5p | 78% |
| miR-133a-3p | 67% | miR-3614-5p | 73% | miR-6722-5p | 67% |
| miR-134-5p | 67% | miR-3616-3p | 68% | miR-6722-3p | 70% |
| miR-184 | 56% | miR-3619-5p | 59% | miR-6724-5p | 75% |
| miR-185-5p | 63% | miR-3621 | 72% | miR-208a-5p | 64% |
| miR-299-3p | 61% | miR-3622a-5p | 74% | miR-210-5p | 69% |
| miR-296-5p | 68% | miR-3622a-3p | 64% | miR-128-1-5p | 76% |
| miR-130b-3p | 88% | miR-3622b-5p | 71% | miR-328-5p | 70% |
| miR-361-5p | 58% | miR-3646 | 65% | miR-181d-3p | 86% |
| miR-365a-3p, miR-365b-3p | 68% | miR-3648 | 79% | miR-504-3p | 70% |
| miR-302c-5p | 66% | miR-3649 | 65% | miR-487b-5p | 51% |
| miR-370-3p | 70% | miR-3651 | 60% | miR-598-5p | 61% |
| miR-373-5p | 69% | miR-3652 | 61% | miR-605-3p | 70% |
| miR-378a-3p | 66% | miR-3660 | 60% | miR-619-5p | 60% |
| miR-328-3p | 73% | miR-3661 | 51% | miR-668-5p | 69% |
| miR-326 | 77% | miR-3663-5p | 70% | miR-1296-3p | 81% |
| miR-133b | 70% | miR-3663-3p | 71% | miR-1301-5p | 54% |
| miR-200a-5p | 54% | miR-3665 | 69% | miR-874-5p | 70% |
| miR-433-3p | 52% | miR-3666 | 55% | miR-1180-5p | 52% |
| miR-410-3p | 100% | miR-3667-5p | 61% | miR-1250-3p | 59% |
| miR-484 | 68% | miR-3675-3p | 73% | miR-1266-3p | 63% |
| miR-485-5p | 61% | miR-3677-3p | 81% | miR-1908-3p | 80% |
| miR-486-5p | 70% | miR-3679-5p | 67% | miR-3928-5p | 57% |
| miR-491-5p | 77% | miR-3679-3p | 74% | miR-1343-5p | 78% |
| miR-202-3p | 63% | miR-3713 | 51% | miR-5189-3p | 51% |
| miR-492 | 71% | miR-3714 | 69% | miR-5699-5p | 73% |
| miR-494-3p | 53% | miR-3180 | 69% | miR-6720-5p | 51% |
| miR-512-5p | 64% | miR-3907 | 88% | miR-6726-5p | 73% |
| miR-498-5p | 83% | miR-3909 | 71% | miR-6727-5p | 70% |
| miR-520e-3p | 64% | miR-3917 | 67% | miR-6728-5p | 59% |
| miR-519e-3p | 58% | miR-3918 | 51% | miR-6729-5p | 79% |
| miR-519c-5p, miR-523-5p, miR-518e-5p, miR-522-5p, miR-519a-5p, miR-519b-5p | 56% | miR-3922-3p | 54% | miR-6729-3p | 71% |
| miR-526b-3p | 51% | miR-3925-5p | 80% | miR-6730-5p | 78% |
| miR-525-5p | 51% | miR-3928-3p | 74% | miR-6731-5p | 62% |
| miR-518f-3p | 53% | miR-3934-5p | 56% | miR-6731-3p | 69% |
| miR-520b-3p | 60% | miR-3936 | 61% | miR-6732-5p | 78% |
| miR-518b | 70% | miR-3937 | 78% | miR-6734-5p | 69% |
| miR-520d-3p | 52% | miR-3943 | 69% | miR-6737-5p | 74% |
| miR-516b-5p | 80% | miR-3944-3p | 69% | miR-6738-5p | 70% |
| miR-519a-3p | 75% | miR-3945 | 57% | miR-6740-5p | 53% |
| miR-501-5p | 55% | miR-642b-3p | 64% | miR-6741-5p | 75% |
| miR-513a-5p | 57% | miR-550b-3p | 58% | miR-6742-5p | 82% |
| miR-18a-3p | 57% | miR-1268b | 78% | miR-6743-5p | 77% |
| miR-551a | 51% | miR-4421 | 56% | miR-6744-5p | 66% |
| miR-554 | 52% | miR-4428 | 58% | miR-6745 | 61% |
| miR-557 | 76% | miR-4429 | 71% | miR-6746-5p | 80% |
| miR-564 | 54% | miR-4430 | 74% | miR-6747-5p | 59% |
| miR-571 | 100% | miR-4433a-3p | 77% | miR-6748-5p | 57% |
| miR-572 | 64% | miR-4435 | 65% | miR-6749-5p | 77% |
| miR-575 | 68% | miR-4439 | 67% | miR-6749-3p | 75% |
| miR-576-5p | 54% | miR-4440 | 68% | miR-6750-5p | 63% |
| miR-579-3p | 69% | miR-4441 | 55% | miR-6751-5p | 61% |
| miR-580-3p | 64% | miR-4443 | 57% | miR-6752-5p | 74% |
| miR-583 | 56% | miR-4444 | 70% | miR-6752-3p | 71% |
| miR-584-5p | 51% | miR-4446-3p | 75% | miR-6753-5p | 67% |
| miR-589-3p | 53% | miR-4447 | 78% | miR-6754-5p | 74% |
| miR-590-5p | 100% | miR-4448 | 70% | miR-6754-3p | 58% |
| miR-595 | 53% | miR-4449 | 77% | miR-6756-5p | 75% |
| miR-596 | 53% | miR-4456 | 52% | miR-6756-3p | 75% |
| miR-600 | 62% | miR-4458 | 56% | miR-6757-5p | 66% |
| miR-601 | 56% | miR-3135b | 73% | miR-6759-5p | 72% |
| miR-611 | 71% | miR-4462 | 70% | miR-6759-3p | 68% |
| miR-612 | 63% | miR-4463 | 70% | miR-6760-5p | 70% |
| miR-614 | 62% | miR-4465 | 71% | miR-6760-3p | 74% |
| miR-615-3p | 66% | miR-4466 | 75% | miR-6762-5p | 65% |
| miR-617 | 54% | miR-4467 | 74% | miR-6762-3p | 58% |
| miR-622 | 57% | miR-4470 | 89% | miR-6763-5p | 78% |
| miR-628-3p | 62% | miR-4472 | 71% | miR-6763-3p | 73% |
| miR-629-3p | 51% | miR-4475 | 60% | miR-6764-3p | 58% |
| miR-632 | 51% | miR-4476 | 74% | miR-6765-5p | 72% |
| miR-638 | 76% | miR-4478 | 76% | miR-6766-5p | 72% |
| miR-639 | 88% | miR-3689d | 55% | miR-6766-3p | 73% |
| miR-650 | 58% | miR-4479 | 66% | miR-6768-5p | 75% |
| miR-661 | 55% | miR-3155b | 64% | miR-6769a-5p | 69% |
| miR-663a | 76% | miR-4481 | 67% | miR-6770-5p | 63% |
| miR-654-5p | 76% | miR-4483 | 73% | miR-6770-3p | 76% |
| miR-658 | 76% | miR-4484 | 75% | miR-6772-5p | 67% |
| miR-421 | 53% | miR-4485-3p | 75% | miR-6772-3p | 54% |
| miR-542-5p | 90% | miR-4486 | 80% | miR-6774-5p | 73% |
| miR-671-5p | 66% | miR-4487 | 73% | miR-6775-5p | 72% |
| miR-769-3p | 68% | miR-4488 | 69% | miR-6775-3p | 71% |
| miR-766-3p | 75% | miR-4489 | 62% | miR-6776-5p | 64% |
| miR-770-5p | 62% | miR-4492 | 68% | miR-6777-5p | 73% |
| miR-675-5p | 65% | miR-4496 | 77% | miR-6777-3p | 74% |
| let-7b-3p | 70% | miR-4497 | 78% | miR-6778-5p | 70% |
| let-7f-1-3p | 71% | miR-4498 | 67% | miR-6779-5p | 74% |
| miR-22-5p | 100% | miR-4499 | 71% | miR-6781-5p | 75% |
| miR-25-5p | 68% | miR-4505 | 75% | miR-6782-5p | 77% |
| miR-26b-3p | 51% | miR-4506 | 52% | miR-6782-3p | 75% |
| miR-92a-2-5p | 78% | miR-2392 | 66% | miR-6783-5p | 60% |
| miR-30c-2-3p | 67% | miR-4507 | 82% | miR-6784-5p | 71% |
| miR-139-3p | 69% | miR-4508 | 72% | miR-6784-3p | 74% |
| miR-181c-3p | 67% | miR-4512 | 75% | miR-6785-3p | 73% |
| miR-183-3p | 72% | miR-4513 | 78% | miR-6786-5p | 75% |
| miR-187-5p | 68% | miR-4516 | 76% | miR-6786-3p | 71% |
| miR-15b-3p | 58% | miR-4519 | 100% | miR-6787-5p | 74% |
| miR-30b-3p | 68% | miR-4521 | 75% | miR-6787-3p | 62% |
| miR-124-5p | 80% | miR-1269b | 65% | miR-6788-5p | 56% |
| miR-125b-1-3p | 100% | miR-4525 | 75% | miR-6788-3p | 71% |
| miR-135a-3p | 61% | miR-4526 | 67% | miR-6789-5p | 78% |
| miR-125a-3p | 71% | miR-4530 | 76% | miR-6789-3p | 67% |
| miR-125b-2-3p | 52% | miR-4531 | 66% | miR-6790-5p | 71% |
| miR-127-5p | 56% | miR-4533 | 71% | miR-6790-3p | 78% |
| miR-138-1-3p | 58% | miR-4534 | 76% | miR-6791-5p | 78% |
| miR-149-3p | 70% | miR-378i | 51% | miR-6792-5p | 68% |
| miR-150-3p | 76% | miR-4535 | 68% | miR-6792-3p | 71% |
| miR-185-3p | 68% | miR-1587 | 71% | miR-6793-3p | 70% |
| miR-186-3p | 64% | miR-4538 | 55% | miR-6794-5p | 74% |
| miR-193a-5p | 56% | miR-4539 | 67% | miR-6795-5p | 68% |
| miR-155-3p | 53% | miR-3127-3p | 69% | miR-6795-3p | 72% |
| miR-194-3p | 61% | miR-3150a-5p | 53% | miR-6796-5p | 62% |
| miR-30c-1-3p | 58% | miR-3158-5p | 69% | miR-6796-3p | 73% |
| miR-296-3p | 82% | miR-3160-5p | 51% | miR-6797-5p | 73% |
| miR-361-3p | 71% | miR-3162-3p | 74% | miR-6797-3p | 72% |
| miR-302d-5p | 66% | miR-3173-5p | 52% | miR-6798-5p | 75% |
| miR-371a-5p | 63% | miR-3187-5p | 73% | miR-6798-3p | 74% |
| miR-377-5p | 56% | miR-3189-5p | 72% | miR-6799-5p | 69% |
| miR-342-5p | 76% | miR-3619-3p | 73% | miR-6800-5p | 63% |
| miR-323a-5p | 56% | miR-3691-3p | 53% | miR-6800-3p | 73% |
| miR-339-3p | 73% | miR-3150b-5p | 71% | miR-6801-3p | 71% |
| miR-423-5p | 73% | miR-3922-5p | 56% | miR-6802-5p | 65% |
| miR-424-3p | 75% | miR-3940-5p | 75% | miR-6802-3p | 71% |
| miR-18b-3p | 71% | miR-3960 | 64% | miR-6803-5p | 75% |
| miR-20b-3p | 68% | miR-4634 | 76% | miR-6805-5p | 76% |
| miR-483-5p | 73% | miR-4635 | 55% | miR-6805-3p | 78% |
| miR-486-3p | 76% | miR-4638-5p | 71% | miR-6806-5p | 67% |
| miR-490-5p | 100% | miR-4640-5p | 77% | miR-6808-5p | 70% |
| miR-193b-5p | 57% | miR-4640-3p | 66% | miR-6809-5p | 76% |
| miR-501-3p | 100% | miR-4648 | 76% | miR-6810-5p | 71% |
| miR-92b-5p | 79% | miR-4649-5p | 62% | miR-6810-3p | 74% |
| miR-551b-5p | 57% | miR-4649-3p | 74% | miR-6811-3p | 51% |
| miR-574-5p | 60% | miR-4650-3p | 75% | miR-6812-5p | 71% |
| miR-550a-5p | 71% | miR-4651 | 76% | miR-6812-3p | 70% |
| miR-615-5p | 84% | miR-4652-5p | 63% | miR-6813-5p | 72% |
| miR-625-3p | 74% | miR-4655-5p | 67% | miR-6813-3p | 74% |
| miR-629-5p | 87% | miR-4656 | 78% | miR-6815-5p | 61% |
| miR-411-3p | 53% | miR-4658 | 53% | miR-6816-5p | 68% |
| miR-671-3p | 59% | miR-4661-3p | 53% | miR-6816-3p | 79% |
| miR-298 | 60% | miR-4664-5p | 69% | miR-6819-5p | 63% |
| miR-874-3p | 71% | miR-4664-3p | 75% | miR-6819-3p | 75% |
| miR-890 | 100% | miR-4665-5p | 79% | miR-6820-5p | 72% |
| miR-892b | 53% | miR-4665-3p | 72% | miR-6820-3p | 72% |
| miR-541-3p | 54% | miR-4667-5p | 76% | miR-6821-5p | 77% |
| miR-708-5p | 59% | miR-4667-3p | 67% | miR-6821-3p | 69% |
| miR-744-5p | 68% | miR-4669 | 59% | miR-6822-5p | 79% |
| miR-885-3p | 68% | miR-4672 | 57% | miR-6822-3p | 56% |
| miR-877-5p | 62% | miR-4673 | 72% | miR-6823-5p | 53% |
| miR-877-3p | 71% | miR-4674 | 71% | miR-6824-5p | 70% |
| miR-887-3p | 70% | miR-4677-5p | 63% | miR-6825-5p | 71% |
| miR-665 | 75% | miR-4684-5p | 51% | miR-6826-3p | 68% |
| miR-760 | 71% | miR-4684-3p | 62% | miR-6827-5p | 76% |
| miR-920 | 77% | miR-4685-5p | 74% | miR-6828-5p | 78% |
| miR-921 | 61% | miR-4687-5p | 73% | miR-6829-5p | 75% |
| miR-509-3-5p | 100% | miR-4687-3p | 66% | miR-6830-5p | 71% |
| miR-933 | 73% | miR-1343-3p | 76% | miR-6831-5p | 71% |
| miR-935 | 71% | miR-4688 | 77% | miR-6832-5p | 64% |
| miR-937-3p | 54% | miR-4689 | 62% | miR-6832-3p | 64% |
| miR-939-5p | 77% | miR-4690-5p | 68% | miR-6833-5p | 63% |
| miR-940 | 68% | miR-4690-3p | 59% | miR-6834-5p | 55% |
| miR-943 | 61% | miR-4691-5p | 68% | miR-6780b-5p | 70% |
| miR-1224-5p | 61% | miR-4695-5p | 73% | miR-6836-5p | 57% |
| miR-1224-3p | 73% | miR-4697-5p | 65% | miR-6836-3p | 78% |
| miR-1225-5p | 74% | miR-4697-3p | 72% | miR-6837-5p | 51% |
| miR-1225-3p | 73% | miR-4706 | 69% | miR-6838-5p | 57% |
| miR-1227-3p | 66% | miR-4707-5p | 79% | miR-6839-5p | 54% |
| miR-1228-5p | 68% | miR-4707-3p | 77% | miR-6840-5p | 70% |
| miR-1228-3p | 75% | miR-4708-5p | 66% | miR-6840-3p | 79% |
| miR-1231 | 76% | miR-4708-3p | 66% | miR-6841-3p | 72% |
| miR-1234-3p | 68% | miR-4710 | 64% | miR-6842-5p | 77% |
| miR-1236-3p | 71% | miR-4716-5p | 72% | miR-6845-5p | 74% |
| miR-1237-3p | 75% | miR-4716-3p | 60% | miR-6845-3p | 73% |
| miR-1238-3p | 73% | miR-4717-3p | 72% | miR-6846-5p | 78% |
| miR-513b-5p | 62% | miR-4720-5p | 57% | miR-6846-3p | 70% |
| miR-320c | 63% | miR-4721 | 67% | miR-6848-5p | 75% |
| miR-1301-3p | 63% | miR-4723-5p | 75% | miR-6848-3p | 66% |
| miR-1298-5p | 59% | miR-4723-3p | 69% | miR-6849-5p | 68% |
| miR-1180-3p | 100% | miR-4725-5p | 62% | miR-6850-5p | 77% |
| miR-1182 | 71% | miR-4725-3p | 71% | miR-6851-5p | 72% |
| miR-1184 | 59% | miR-4726-5p | 64% | miR-6851-3p | 75% |
| miR-1202 | 63% | miR-4726-3p | 66% | miR-6852-5p | 51% |
| miR-1203 | 67% | miR-4727-3p | 55% | miR-6852-3p | 51% |
| miR-663b | 65% | miR-4728-5p | 63% | miR-6855-5p | 53% |
| miR-1207-5p | 78% | miR-4728-3p | 69% | miR-6855-3p | 70% |
| miR-1285-3p | 65% | miR-4730 | 64% | miR-6856-5p | 51% |
| miR-1297 | 55% | miR-4731-5p | 61% | miR-6857-5p | 79% |
| miR-1303 | 71% | miR-4731-3p | 72% | miR-6857-3p | 74% |
| miR-1304-5p | 58% | miR-4732-5p | 79% | miR-6858-5p | 70% |
| miR-1243 | 100% | miR-4734 | 74% | miR-6858-3p | 75% |
| miR-1249-3p | 73% | miR-4736 | 67% | miR-6859-3p | 70% |
| miR-1250-5p | 52% | miR-4738-3p | 62% | miR-6769b-5p | 67% |
| miR-1258 | 100% | miR-4739 | 66% | miR-6769b-3p | 65% |
| miR-1266-5p | 56% | miR-4740-5p | 55% | miR-6860 | 70% |
| miR-1267 | 74% | miR-4740-3p | 64% | miR-6861-5p | 72% |
| miR-1268a | 73% | miR-4741 | 82% | miR-6861-3p | 74% |
| miR-1269a | 59% | miR-4743-5p | 74% | miR-6862-5p | 64% |
| miR-1270 | 55% | miR-4745-5p | 77% | miR-6865-3p | 72% |
| miR-1281 | 72% | miR-4745-3p | 57% | miR-6868-3p | 51% |
| miR-1292-5p | 88% | miR-4746-3p | 70% | miR-6869-5p | 71% |
| miR-1307-3p | 71% | miR-4747-5p | 72% | miR-6870-5p | 70% |
| miR-1321 | 52% | miR-4748 | 64% | miR-6870-3p | 71% |
| miR-1469 | 78% | miR-4749-5p | 77% | miR-6871-5p | 58% |
| miR-1470 | 72% | miR-4749-3p | 74% | miR-6872-5p | 60% |
| miR-1471 | 62% | miR-4750-5p | 63% | miR-6873-5p | 52% |
| miR-1538 | 67% | miR-371b-5p | 78% | miR-6874-5p | 52% |
| miR-1908-5p | 80% | miR-371b-3p | 68% | miR-6875-5p | 74% |
| miR-1909-5p | 63% | miR-4754 | 52% | miR-6875-3p | 63% |
| miR-1909-3p | 72% | miR-4755-3p | 71% | miR-6876-5p | 63% |
| miR-1912-3p | 69% | miR-4756-5p | 66% | miR-6877-5p | 78% |
| miR-1913 | 73% | miR-4758-5p | 78% | miR-6879-5p | 67% |
| miR-1914-3p | 75% | miR-4758-3p | 71% | miR-6880-5p | 73% |
| miR-1915-5p | 53% | miR-4763-5p | 75% | miR-6880-3p | 73% |
| miR-1915-3p | 78% | miR-4763-3p | 80% | miR-6881-5p | 70% |
| miR-365a-5p | 73% | miR-4766-5p | 57% | miR-6883-5p | 64% |
| miR-196b-3p | 53% | miR-4767 | 72% | miR-6884-3p | 72% |
| miR-1972 | 72% | miR-4769-5p | 57% | miR-6885-5p | 63% |
| miR-2110 | 76% | miR-4769-3p | 66% | miR-6885-3p | 73% |
| miR-449c-5p | 57% | miR-4776-5p | 67% | miR-6886-5p | 56% |
| miR-762 | 71% | miR-4776-3p | 51% | miR-6887-5p | 69% |
| miR-670-5p | 61% | miR-4778-5p | 67% | miR-6887-3p | 71% |
| miR-2114-5p | 60% | miR-4780 | 60% | miR-6889-5p | 74% |
| miR-2114-3p | 100% | miR-4781-5p | 100% | miR-6889-3p | 72% |
| miR-2116-5p | 60% | miR-4783-5p | 53% | miR-6891-5p | 71% |
| miR-2116-3p | 74% | miR-4783-3p | 80% | miR-6891-3p | 68% |
| miR-548q | 69% | miR-4785 | 53% | miR-6892-5p | 73% |
| miR-2276-3p | 76% | miR-2467-3p | 67% | miR-6892-3p | 71% |
| miR-2278 | 64% | miR-4787-5p | 75% | miR-6893-5p | 72% |
| miR-711 | 77% | miR-4788 | 69% | miR-6894-5p | 71% |
| miR-718 | 78% | miR-4793-3p | 61% | miR-6895-5p | 62% |
| miR-2681-3p | 59% | miR-4800-5p | 76% | miR-6895-3p | 54% |
| miR-449c-3p | 51% | miR-642a-3p | 66% | miR-7106-5p | 74% |
| miR-2861 | 64% | miR-4433a-5p | 73% | miR-7107-5p | 76% |
| miR-2909 | 55% | miR-4482-3p | 75% | miR-7108-5p | 72% |
| miR-3116 | 64% | miR-4999-5p | 52% | miR-7108-3p | 77% |
| miR-3122 | 77% | miR-5001-5p | 72% | miR-7109-5p | 69% |
| miR-3124-5p | 64% | miR-5001-3p | 56% | miR-7109-3p | 68% |
| miR-3126-5p | 75% | miR-5006-5p | 61% | miR-7110-5p | 78% |
| miR-3130-3p | 57% | miR-5006-3p | 52% | miR-7111-5p | 75% |
| miR-3131 | 75% | miR-5008-5p | 64% | miR-7112-5p | 65% |
| miR-3132 | 65% | miR-5009-3p | 57% | miR-7113-3p | 73% |
| miR-3133 | 54% | miR-5010-5p | 74% | miR-7114-5p | 72% |
| miR-378b | 56% | miR-5088-5p | 62% | miR-7114-3p | 73% |
| miR-3138 | 51% | miR-5090 | 68% | miR-7150 | 75% |
| miR-3141 | 73% | miR-5188 | 52% | miR-7151-3p | 51% |
| miR-3144-5p | 67% | miR-5189-5p | 59% | miR-7152-5p | 61% |
| miR-1273c | 75% | miR-5190 | 63% | miR-7152-3p | 71% |
| miR-3147 | 78% | miR-5195-3p | 61% | miR-7155-5p | 72% |
| miR-548v | 100% | miR-5196-5p | 68% | miR-7155-3p | 51% |
| miR-3150a-3p | 77% | miR-4524b-5p | 57% | miR-7160-5p | 69% |
| miR-3151-5p | 69% | miR-5100 | 73% | miR-7515 | 59% |
| miR-3153 | 78% | miR-5572 | 77% | miR-7704 | 70% |
| miR-3074-3p | 57% | miR-5584-3p | 54% | miR-7843-5p | 57% |
| miR-3154 | 75% | miR-5585-3p | 67% | miR-4433b-5p | 72% |
| miR-3156-5p | 70% | miR-5587-5p | 51% | miR-4433b-3p | 79% |
| miR-3158-3p | 52% | miR-5587-3p | 56% | miR-1273h-5p | 72% |
| miR-3161 | 51% | miR-5589-5p | 59% | miR-1273h-3p | 59% |
| miR-3162-5p | 74% | miR-5684 | 55% | miR-6516-5p | 52% |
| miR-3163 | 75% | miR-4666b | 60% | miR-7845-5p | 66% |
| miR-3164 | 62% | miR-5698 | 70% | miR-7846-3p | 75% |
| miR-3165 | 63% | miR-5703 | 55% | miR-7847-3p | 69% |
| miR-3173-3p | 69% | miR-5705 | 74% | miR-7851-3p | 66% |
| miR-1193 | 75% | miR-197-5p | 76% | miR-7855-5p | 66% |
| miR-3176 | 65% | miR-204-3p | 77% | miR-7856-5p | 52% |
| miR-3177-3p | 75% | miR-211-3p | 75% | miR-8052 | 75% |
| miR-3178 | 74% | miR-345-3p | 62% | miR-8059 | 72% |
| miR-3180-5p | 75% | miR-652-5p | 66% | miR-8060 | 69% |
| miR-3180-3p | 72% | miR-1185-2-3p | 71% | miR-8062 | 60% |
| miR-3184-5p | 65% | miR-873-3p | 62% | miR-8063 | 66% |
| miR-3185 | 76% | miR-1304-3p | 69% | miR-8064 | 59% |
| miR-3065-5p | 67% | miR-1247-3p | 77% | miR-8069 | 73% |
| miR-3186-3p | 64% | miR-3184-3p | 64% | miR-8071 | 72% |
| miR-3187-3p | 77% | miR-642b-5p | 65% | miR-8072 | 76% |
| miR-3189-3p | 78% | miR-365b-5p | 71% | miR-8073 | 79% |
| miR-320e | 53% | miR-1185-1-3p | 71% | miR-8074 | 55% |
| miR-3191-3p | 79% | miR-381-5p | 52% | miR-8077 | 53% |
| miR-3195 | 66% | miR-937-5p | 74% | miR-8078 | 56% |
| miR-3196 | 74% | miR-1227-5p | 79% | miR-8085 | 72% |
| miR-3197 | 78% | miR-1229-5p | 65% | miR-8089 | 77% |
| miR-3198 | 61% | miR-1233-5p | 72% | miR-128-2-5p | 62% |
| miR-3199 | 61% | miR-1237-5p | 70% | miR-548ba | 62% |
| miR-514b-5p | 63% | miR-1238-5p | 76% | miR-7977 | 67% |
| miR-3202 | 64% | miR-3620-5p | 80% | miR-1249-5p | 71% |
| miR-3065-3p | 53% | miR-4632-5p | 68% | miR-4485-5p | 69% |
| miR-4297 | 55% | miR-4743-3p | 58% | miR-103a-1-5p | 53% |
| miR-4293 | 67% | miR-4750-3p | 73% | miR-217-3p | 52% |
| miR-4294 | 72% | miR-5739 | 77% | miR-135a-2-3p | 59% |
| miR-4299 | 65% | miR-5787 | 67% | miR-498-3p | 61% |
| miR-4298 | 72% | miR-6068 | 72% | miR-526a-3p | 100% |
| miR-4300 | 53% | miR-6069 | 71% | miR-9718 | 57% |
| miR-4304 | 74% | miR-6071 | 59% | miR-9898 | 69% |
| miR-4306 | 66% | miR-6074 | 63% | miR-9902 | 56% |
| miR-4312 | 75% | miR-6075 | 73% | miR-10392-5p | 75% |
| miR-4313 | 71% | miR-6076 | 77% | miR-10392-3p | 78% |
| miR-4315 | 57% | miR-6080 | 52% | miR-10394-3p | 63% |
| miR-4316 | 66% | miR-6081 | 78% | miR-10396a-5p | 68% |
| miR-4314 | 55% | miR-6083 | 54% | miR-10396a-3p | 80% |
| miR-4319 | 55% | miR-6085 | 75% | miR-10398-3p | 74% |
| miR-4322 | 78% | miR-6086 | 74% | miR-10400-5p | 66% |
| miR-4321 | 60% | miR-6088 | 63% | miR-10400-3p | 80% |
| miR-4323 | 71% | miR-6089 | 75% | miR-10401-5p | 73% |
| miR-4324 | 55% | miR-6090 | 74% | miR-10401-3p | 81% |
| miR-4257 | 71% | miR-6124 | 74% | miR-10396b-5p | 74% |
| miR-4258 | 80% | miR-6125 | 79% | miR-10522-5p | 80% |
| miR-4259 | 69% | miR-6126 | 72% | miR-10526-3p | 76% |
| miR-4260 | 73% | miR-6127 | 73% | miR-10527-5p | 83% |
| miR-4253 | 73% | miR-6131 | 71% | miR-11181-3p | 71% |
| miR-4251 | 58% | miR-6132 | 71% | miR-11399 | 73% |
| miR-4254 | 57% | miR-6133 | 73% | miR-11401 | 52% |
| miR-4252 | 54% | miR-6165 | 76% | miR-3059-3p | 53% |
| miR-4326 | 61% | miR-6499-5p | 53% | miR-3085-5p | 68% |
| miR-4327 | 78% | miR-6501-5p | 62% | miR-3085-3p | 70% |
| miR-4261 | 58% | miR-6503-3p | 52% | miR-12114 | 62% |
| miR-4265 | 68% | miR-6506-5p | 53% | miR-12115 | 73% |
| miR-4266 | 70% | miR-6510-5p | 70% | miR-12116 | 73% |
| miR-4269 | 75% | miR-6511a-5p | 71% | miR-12118 | 75% |
| miR-4271 | 72% | miR-6512-3p | 55% | miR-12119 | 74% |
| miR-4276 | 67% | miR-6513-5p | 62% | miR-12120 | 71% |
| miR-4274 | 74% | miR-6513-3p | 51% | miR-12121 | 71% |
| miR-4281 | 69% | miR-6514-5p | 55% | miR-12122 | 58% |
| miR-4280 | 57% | miR-6514-3p | 51% | miR-12126 | 83% |
| miR-4285 | 69% | miR-6515-5p | 64% | miR-12128 | 52% |
| miR-4283 | 55% | miR-6515-3p | 72% | miR-12131 | 100% |
| miR-4284 | 70% | miR-6715b-3p | 57% | miR-12136 | 64% |
| miR-4286 | 74% | miR-6716-5p | 75% | | |

| | | | | | |
|---|---|---|---|---|---|
| ^{∗} A represents Accuracy. ^{∗} "has-" in the miRNA names has been omitted. | | | | | |

### [Table 23-8]

**Table 23-8: miRNA to Predict Human Urothelial Cancer of Stages I and II**

| miRNA Name | A | miRNA Name | A | miRNA Name | A |
|---|---|---|---|---|---|
| miR-15a-5p | 55% | miR-3689a-5p, miR-3689b-5p, miR-3689e | 55% | miR-1266-3p | 58% |
| miR-17-3p | 100% | miR-3689a-3p | 54% | miR-3151-3p | 70% |
| miR-29a-3p | 59% | miR-3714 | 70% | miR-3192-3p | 54% |
| miR-30a-5p | 63% | miR-3180 | 65% | miR-1343-5p | 76% |
| miR-29b-3p | 53% | miR-3907 | 83% | miR-5088-3p | 61% |
| miR-103a-3p | 53% | miR-3908 | 63% | miR-5189-3p | 56% |
| miR-107 | 57% | miR-3911 | 66% | miR-5699-5p | 73% |
| miR-196a-5p | 69% | miR-3917 | 74% | miR-6726-5p | 74% |
| miR-197-3p | 73% | miR-3919 | 65% | miR-6726-3p | 60% |
| miR-198 | 68% | miR-3150b-3p | 54% | miR-6727-5p | 60% |
| miR-208a-3p | 58% | miR-3925-5p | 72% | miR-6727-3p | 69% |
| miR-30d-5p | 75% | miR-3926 | 69% | miR-6728-5p | 57% |
| miR-10a-5p | 62% | miR-3928-3p | 76% | miR-6728-3p | 76% |
| miR-182-5p | 67% | miR-3935 | 56% | miR-6729-5p | 68% |
| miR-187-3p | 53% | miR-3937 | 77% | miR-6729-3p | 78% |
| miR-199b-5p | 73% | miR-3943 | 73% | miR-6730-5p | 71% |
| miR-214-3p | 57% | miR-3944-3p | 66% | miR-6730-3p | 72% |
| miR-223-3p | 71% | miR-3945 | 61% | miR-6731-5p | 62% |
| miR-124-3p | 71% | miR-642b-3p | 70% | miR-6731-3p | 72% |
| miR-125b-5p | 52% | miR-550b-3p | 53% | miR-6732-5p | 76% |
| miR-133a-3p | 72% | miR-1268b | 75% | miR-6732-3p | 66% |
| miR-140-5p | 75% | miR-548ab | 67% | miR-6734-5p | 71% |
| miR-141-3p | 63% | miR-4421 | 53% | miR-6735-5p | 70% |
| miR-134-5p | 71% | miR-4428 | 76% | miR-6735-3p | 59% |
| miR-149-5p | 66% | miR-4429 | 61% | miR-6736-5p | 74% |
| miR-154-3p | 54% | miR-4430 | 77% | miR-6736-3p | 67% |
| miR-184 | 51% | miR-4433a-3p | 76% | miR-6737-5p | 67% |
| miR-188-5p | 64% | miR-4436a | 61% | miR-6737-3p | 75% |
| miR-320a-3p | 66% | miR-4439 | 68% | miR-6738-5p | 72% |
| miR-106b-5p | 86% | miR-4440 | 71% | miR-6738-3p | 58% |
| miR-302a-3p | 100% | miR-4441 | 65% | miR-6740-5p | 61% |
| miR-299-3p | 56% | miR-4443 | 66% | miR-6740-3p | 66% |
| miR-296-5p | 74% | miR-4444 | 57% | miR-6741-5p | 80% |
| miR-130b-3p | 90% | miR-4446-3p | 66% | miR-6741-3p | 69% |
| miR-361-5p | 56% | miR-4447 | 86% | miR-6742-5p | 72% |
| miR-365a-3p, miR-365b-3p | 73% | miR-4448 | 57% | miR-6742-3p | 74% |
| miR-302c-5p | 61% | miR-4449 | 68% | miR-6743-5p | 74% |
| miR-370-3p | 79% | miR-4455 | 60% | miR-6743-3p | 70% |
| miR-373-5p | 76% | miR-4456 | 57% | miR-6744-5p | 62% |
| miR-373-3p | 67% | miR-4458 | 62% | miR-6744-3p | 60% |
| miR-375-3p | 63% | miR-4460 | 80% | miR-6745 | 58% |
| miR-378a-3p | 51% | miR-378h | 58% | miR-6746-5p | 76% |
| miR-383-5p | 67% | miR-3135b | 70% | miR-6747-5p | 68% |
| miR-328-3p | 71% | miR-4462 | 73% | miR-6747-3p | 65% |
| miR-342-3p | 54% | miR-4463 | 73% | miR-6748-5p | 81% |
| miR-326 | 63% | miR-4466 | 63% | miR-6749-5p | 80% |
| miR-133b | 72% | miR-4467 | 71% | miR-6749-3p | 68% |
| miR-325 | 52% | miR-4468 | 71% | miR-6750-3p | 69% |
| miR-346 | 60% | miR-4470 | 86% | miR-6751-5p | 74% |
| miR-448 | 54% | miR-4472 | 72% | miR-6751-3p | 66% |
| miR-449a | 60% | miR-4475 | 68% | miR-6752-5p | 77% |
| miR-450a-5p | 52% | miR-4476 | 70% | miR-6752-3p | 74% |
| miR-200a-5p | 57% | miR-4478 | 74% | miR-6753-5p | 69% |
| miR-433-3p | 52% | miR-3689d | 56% | miR-6753-3p | 63% |
| miR-452-3p | 55% | miR-4481 | 73% | miR-6754-5p | 73% |
| miR-409-3p | 71% | miR-4483 | 76% | miR-6754-3p | 60% |
| miR-484 | 74% | miR-4484 | 75% | miR-6755-5p | 80% |
| miR-485-3p | 62% | miR-4485-3p | 75% | miR-6756-5p | 80% |
| miR-486-5p | 75% | miR-4486 | 80% | miR-6756-3p | 75% |
| miR-491-5p | 84% | miR-4487 | 74% | miR-6757-5p | 66% |
| miR-202-3p | 59% | miR-4488 | 62% | miR-6757-3p | 75% |
| miR-492 | 57% | miR-4489 | 68% | miR-6758-5p | 66% |
| miR-512-5p | 53% | miR-4492 | 61% | miR-6759-5p | 67% |
| miR-498-5p | 80% | miR-4496 | 80% | miR-6759-3p | 74% |
| miR-526b-5p | 56% | miR-4497 | 68% | miR-6760-5p | 58% |
| miR-518b | 74% | miR-4498 | 70% | miR-6760-3p | 75% |
| miR-518c-5p | 71% | miR-4499 | 68% | miR-6761-3p | 67% |
| miR-519d-3p | 55% | miR-4505 | 75% | miR-6762-5p | 66% |
| miR-516b-5p | 86% | miR-4506 | 58% | miR-6762-3p | 56% |
| miR-518a-3p | 67% | miR-2392 | 72% | miR-6763-5p | 82% |
| miR-519a-3p | 71% | miR-4507 | 78% | miR-6763-3p | 80% |
| miR-502-5p | 57% | miR-4508 | 69% | miR-6765-5p | 66% |
| miR-513a-5p | 58% | miR-4510 | 54% | miR-6766-5p | 76% |
| miR-532-5p | 83% | miR-4511 | 55% | miR-6766-3p | 76% |
| miR-299-5p | 53% | miR-4513 | 71% | miR-6767-5p | 55% |
| miR-539-5p | 60% | miR-4516 | 66% | miR-6768-5p | 74% |
| miR-552-3p | 58% | miR-4518 | 54% | miR-6769a-5p | 73% |
| miR-554 | 52% | miR-4520-3p | 54% | miR-6769a-3p | 73% |
| miR-555 | 59% | miR-4521 | 71% | miR-6770-5p | 57% |
| miR-557 | 70% | miR-1269b | 63% | miR-6771-5p | 61% |
| miR-563 | 59% | miR-4522 | 65% | miR-6771-3p | 76% |
| miR-564 | 60% | miR-4523 | 60% | miR-6772-5p | 69% |
| miR-571 | 100% | miR-4524a-3p | 71% | miR-6772-3p | 58% |
| miR-574-3p | 68% | miR-4525 | 66% | miR-6773-3p | 54% |
| miR-575 | 59% | miR-4526 | 70% | miR-6774-5p | 69% |
| miR-579-3p | 62% | miR-4530 | 76% | miR-6775-5p | 69% |
| miR-583 | 51% | miR-4533 | 75% | miR-6775-3p | 69% |
| miR-585-3p | 54% | miR-4534 | 65% | miR-6776-5p | 75% |
| miR-595 | 54% | miR-4535 | 64% | miR-6776-3p | 78% |
| miR-608 | 62% | miR-1587 | 73% | miR-6777-5p | 74% |
| miR-610 | 53% | miR-4538 | 65% | miR-6777-3p | 74% |
| miR-612 | 56% | miR-4539 | 77% | miR-6778-5p | 77% |
| miR-615-3p | 59% | miR-3127-3p | 74% | miR-6778-3p | 62% |
| miR-616-5p | 64% | miR-3156-3p | 68% | miR-6779-5p | 70% |
| miR-617 | 55% | miR-3158-5p | 71% | miR-6779-3p | 68% |
| miR-624-5p | 55% | miR-3162-3p | 74% | miR-6780a-5p | 72% |
| miR-628-3p | 60% | miR-3173-5p | 61% | miR-6780a-3p | 60% |
| miR-632 | 53% | miR-3187-5p | 62% | miR-6781-5p | 73% |
| miR-634 | 76% | miR-3189-5p | 72% | miR-6782-5p | 73% |
| miR-637 | 78% | miR-3619-3p | 81% | miR-6782-3p | 76% |
| miR-638 | 68% | miR-3150b-5p | 72% | miR-6783-5p | 61% |
| miR-639 | 88% | miR-3925-3p | 57% | miR-6783-3p | 60% |
| miR-642a-5p | 62% | miR-3940-5p | 64% | miR-6784-5p | 71% |
| miR-649 | 53% | miR-3972 | 61% | miR-6784-3p | 75% |
| miR-663a | 72% | miR-3978 | 100% | miR-6785-3p | 75% |
| miR-449b-5p | 57% | miR-4634 | 67% | miR-6786-5p | 68% |
| miR-657 | 55% | miR-4638-5p | 77% | miR-6786-3p | 73% |
| miR-658 | 67% | miR-4639-3p | 51% | miR-6787-5p | 80% |
| miR-659-3p | 66% | miR-4640-5p | 71% | miR-6787-3p | 64% |
| miR-542-5p | 83% | miR-4640-3p | 67% | miR-6788-5p | 51% |
| miR-671-5p | 65% | miR-4642 | 78% | miR-6788-3p | 63% |
| miR-668-3p | 57% | miR-4645-5p | 60% | miR-6789-5p | 71% |
| miR-767-3p | 74% | miR-4646-5p | 73% | miR-6789-3p | 54% |
| miR-769-5p | 56% | miR-4646-3p | 70% | miR-6790-5p | 71% |
| miR-766-3p | 76% | miR-4648 | 54% | miR-6790-3p | 77% |
| miR-765 | 68% | miR-4649-5p | 59% | miR-6791-5p | 75% |
| miR-675-5p | 64% | miR-4649-3p | 71% | miR-6792-5p | 71% |
| let-7b-3p | 69% | miR-4650-3p | 71% | miR-6792-3p | 78% |
| let-7f-1-3p | 73% | miR-4651 | 71% | miR-6793-5p | 71% |
| miR-92a-2-5p | 68% | miR-4653-3p | 64% | miR-6793-3p | 76% |
| miR-29b-2-5p | 57% | miR-4654 | 55% | miR-6794-5p | 85% |
| miR-139-3p | 62% | miR-4655-5p | 69% | miR-6794-3p | 68% |
| miR-181c-3p | 67% | miR-4656 | 69% | miR-6795-5p | 68% |
| miR-183-3p | 72% | miR-4661-5p | 55% | miR-6795-3p | 75% |
| miR-187-5p | 58% | miR-4659b-3p | 67% | miR-6796-5p | 76% |
| miR-124-5p | 71% | miR-4664-5p | 72% | miR-6796-3p | 80% |
| miR-125b-1-3p | 100% | miR-4664-3p | 75% | miR-6797-5p | 77% |
| miR-130a-5p | 60% | miR-4665-5p | 83% | miR-6797-3p | 73% |
| miR-135a-3p | 61% | miR-4665-3p | 78% | miR-6798-5p | 74% |
| miR-140-3p | 67% | miR-4667-5p | 76% | miR-6798-3p | 75% |
| miR-125a-3p | 74% | miR-4667-3p | 71% | miR-6799-5p | 77% |
| miR-127-5p | 60% | miR-4669 | 71% | miR-6799-3p | 69% |
| miR-149-3p | 69% | miR-4672 | 59% | miR-6800-5p | 68% |
| miR-150-3p | 81% | miR-4673 | 54% | miR-6800-3p | 75% |
| miR-185-3p | 71% | miR-4674 | 63% | miR-6801-3p | 74% |
| miR-186-3p | 64% | miR-4675 | 64% | miR-6802-5p | 66% |
| miR-193a-5p | 66% | miR-4676-5p | 58% | miR-6802-3p | 76% |
| miR-194-3p | 54% | miR-4677-5p | 70% | miR-6803-5p | 70% |
| miR-30c-1-3p | 56% | miR-4682 | 54% | miR-6803-3p | 68% |
| miR-99b-3p | 52% | miR-4685-5p | 69% | miR-6804-3p | 70% |
| miR-296-3p | 74% | miR-4685-3p | 68% | miR-6805-5p | 72% |
| miR-361-3p | 75% | miR-4687-5p | 78% | miR-6805-3p | 78% |
| miR-371a-5p | 68% | miR-4687-3p | 65% | miR-6806-5p | 55% |
| miR-342-5p | 63% | miR-1343-3p | 76% | miR-6807-5p | 68% |
| miR-151a-5p | 51% | miR-4688 | 67% | miR-6808-5p | 74% |
| miR-423-5p | 69% | miR-4689 | 65% | miR-6808-3p | 64% |
| miR-424-3p | 71% | miR-4690-5p | 76% | miR-6809-5p | 74% |
| miR-18b-3p | 73% | miR-4695-5p | 73% | miR-6809-3p | 64% |
| miR-20b-3p | 68% | miR-4695-3p | 70% | miR-6810-5p | 78% |
| miR-431-3p | 51% | miR-4697-5p | 67% | miR-6810-3p | 73% |
| miR-483-5p | 72% | miR-4697-3p | 75% | miR-6812-5p | 79% |
| miR-486-3p | 66% | miR-4698 | 78% | miR-6812-3p | 71% |
| miR-490-5p | 100% | miR-4699-3p | 55% | miR-6813-5p | 68% |
| miR-193b-5p | 58% | miR-4700-5p | 61% | miR-6813-3p | 75% |
| miR-505-5p | 75% | miR-4700-3p | 71% | miR-6815-5p | 59% |
| miR-532-3p | 65% | miR-4701-5p | 74% | miR-6815-3p | 60% |
| miR-92b-5p | 73% | miR-4701-3p | 62% | miR-6816-5p | 69% |
| miR-551b-5p | 64% | miR-4704-5p | 53% | miR-6816-3p | 59% |
| miR-574-5p | 61% | miR-4706 | 74% | miR-6818-5p | 71% |
| miR-550a-5p | 65% | miR-4707-5p | 73% | miR-6819-5p | 67% |
| miR-615-5p | 72% | miR-4707-3p | 78% | miR-6819-3p | 73% |
| miR-616-3p | 55% | miR-4708-3p | 54% | miR-6820-5p | 69% |
| miR-624-3p | 71% | miR-4709-3p | 67% | miR-6820-3p | 75% |
| miR-625-3p | 77% | miR-4710 | 74% | miR-6821-5p | 78% |
| miR-629-5p | 89% | miR-4713-5p | 67% | miR-6822-5p | 70% |
| miR-298 | 80% | miR-4714-5p | 74% | miR-6822-3p | 53% |
| miR-874-3p | 72% | miR-4715-5p | 56% | miR-6823-5p | 56% |
| miR-891b | 80% | miR-4716-5p | 75% | miR-6823-3p | 66% |
| miR-876-3p | 83% | miR-4716-3p | 71% | miR-6824-5p | 75% |
| miR-744-5p | 65% | miR-4717-3p | 65% | miR-6824-3p | 66% |
| miR-885-5p | 62% | miR-4719 | 58% | miR-6825-5p | 74% |
| miR-885-3p | 64% | miR-4720-5p | 59% | miR-6825-3p | 64% |
| miR-877-5p | 66% | miR-4721 | 74% | miR-6826-3p | 72% |
| miR-877-3p | 72% | miR-4722-5p | 64% | miR-6827-5p | 67% |
| miR-887-3p | 67% | miR-4722-3p | 67% | miR-6827-3p | 74% |
| miR-665 | 67% | miR-4723-5p | 66% | miR-6828-5p | 66% |
| miR-760 | 76% | miR-4723-3p | 72% | miR-6829-5p | 76% |
| miR-920 | 81% | miR-4724-5p | 54% | miR-6829-3p | 63% |
| miR-933 | 67% | miR-4725-5p | 53% | miR-6830-5p | 80% |
| miR-936 | 64% | miR-4725-3p | 80% | miR-6830-3p | 67% |
| miR-939-5p | 78% | miR-4726-5p | 66% | miR-6831-5p | 72% |
| miR-940 | 74% | miR-4728-5p | 71% | miR-6831-3p | 65% |
| miR-1224-5p | 70% | miR-4728-3p | 73% | miR-6832-5p | 72% |
| miR-1224-3p | 76% | miR-4730 | 66% | miR-6832-3p | 58% |
| miR-1225-5p | 71% | miR-4731-5p | 66% | miR-6833-5p | 68% |
| miR-1225-3p | 77% | miR-4731-3p | 75% | miR-6833-3p | 70% |
| miR-1227-3p | 66% | miR-4732-5p | 76% | miR-6834-5p | 75% |
| miR-1228-5p | 66% | miR-4732-3p | 64% | miR-6834-3p | 68% |
| miR-1228-3p | 78% | miR-4734 | 64% | miR-6780b-5p | 79% |
| miR-1229-3p | 68% | miR-4736 | 59% | miR-6780b-3p | 67% |
| miR-1231 | 66% | miR-3064-5p | 55% | miR-6836-3p | 71% |
| miR-1233-3p | 58% | miR-3064-3p | 54% | miR-6837-5p | 57% |
| miR-1234-3p | 73% | miR-4739 | 64% | miR-6838-5p | 57% |
| miR-1236-3p | 76% | miR-4741 | 71% | miR-6839-5p | 57% |
| miR-1237-3p | 78% | miR-4743-5p | 77% | miR-6840-5p | 72% |
| miR-1238-3p | 78% | miR-4745-5p | 71% | miR-6840-3p | 73% |
| miR-320b | 65% | miR-4746-3p | 65% | miR-6841-3p | 74% |
| miR-320c | 64% | miR-4747-5p | 79% | miR-6842-5p | 78% |
| miR-1323 | 64% | miR-4748 | 67% | miR-6845-5p | 72% |
| miR-1301-3p | 61% | miR-4749-5p | 77% | miR-6845-3p | 80% |
| miR-1298-5p | 55% | miR-4749-3p | 75% | miR-6846-5p | 83% |
| miR-1180-3p | 100% | miR-4750-5p | 70% | miR-6846-3p | 72% |
| miR-1181 | 55% | miR-4751 | 68% | miR-6847-3p | 59% |
| miR-1182 | 69% | miR-4752 | 54% | miR-6848-5p | 73% |
| miR-1184 | 52% | miR-4753-5p | 74% | miR-6848-3p | 74% |
| miR-1202 | 71% | miR-371b-5p | 71% | miR-6849-5p | 70% |
| miR-1203 | 63% | miR-371b-3p | 70% | miR-6849-3p | 56% |
| miR-1207-5p | 77% | miR-4754 | 56% | miR-6850-5p | 66% |
| miR-1207-3p | 56% | miR-4755-3p | 63% | miR-6851-5p | 69% |
| miR-1208 | 56% | miR-499b-3p | 56% | miR-6851-3p | 75% |
| miR-1289 | 57% | miR-4756-5p | 67% | miR-6852-3p | 52% |
| miR-1290 | 100% | miR-4758-5p | 78% | miR-6855-5p | 65% |
| miR-1293 | 54% | miR-4758-3p | 72% | miR-6855-3p | 75% |
| miR-1294 | 63% | miR-4759 | 100% | miR-6856-5p | 59% |
| miR-1303 | 54% | miR-4763-5p | 70% | miR-6857-5p | 67% |
| miR-1304-5p | 64% | miR-4763-3p | 75% | miR-6857-3p | 75% |
| miR-1246 | 100% | miR-4764-5p | 100% | miR-6858-5p | 66% |
| miR-1249-3p | 78% | miR-4766-5p | 70% | miR-6858-3p | 74% |
| miR-1260a | 70% | miR-4769-5p | 68% | miR-6859-5p | 58% |
| miR-1263 | 60% | miR-4769-3p | 71% | miR-6859-3p | 76% |
| miR-1266-5p | 51% | miR-4770 | 60% | miR-6769b-5p | 73% |
| miR-1267 | 75% | miR-4776-5p | 73% | miR-6769b-3p | 65% |
| miR-1268a | 68% | miR-4778-5p | 73% | miR-6860 | 68% |
| miR-1270 | 57% | miR-4779 | 88% | miR-6861-5p | 78% |
| miR-1275 | 63% | miR-4780 | 54% | miR-6861-3p | 72% |
| miR-1281 | 75% | miR-4436b-5p | 70% | miR-6862-5p | 66% |
| miR-1292-5p | 83% | miR-4781-5p | 100% | miR-6862-3p | 73% |
| miR-1255b-5p | 100% | miR-4783-3p | 77% | miR-6864-5p | 73% |
| miR-1307-3p | 70% | miR-4784 | 53% | miR-6865-5p | 69% |
| miR-320d | 86% | miR-2467-5p | 58% | miR-6865-3p | 68% |
| miR-1825 | 68% | miR-2467-3p | 67% | miR-6867-5p | 61% |
| miR-675-3p | 66% | miR-4787-5p | 61% | miR-6867-3p | 63% |
| miR-1469 | 71% | miR-4787-3p | 72% | miR-6869-5p | 67% |
| miR-1470 | 74% | miR-4788 | 71% | miR-6870-5p | 76% |
| miR-1471 | 70% | miR-4793-3p | 59% | miR-6870-3p | 75% |
| miR-1539 | 58% | miR-4795-3p | 100% | miR-6871-5p | 61% |
| miR-1908-5p | 76% | miR-4799-5p | 56% | miR-6872-3p | 70% |
| miR-1909-5p | 67% | miR-4800-5p | 77% | miR-6873-5p | 67% |
| miR-1909-3p | 72% | miR-4804-3p | 55% | miR-6874-5p | 55% |
| miR-1910-5p | 60% | miR-642a-3p | 68% | miR-6875-5p | 63% |
| miR-1912-3p | 67% | miR-4433a-5p | 78% | miR-6875-3p | 61% |
| miR-1913 | 75% | miR-4482-3p | 72% | miR-6876-5p | 72% |
| miR-1914-3p | 72% | miR-4999-5p | 60% | miR-6876-3p | 53% |
| miR-1915-3p | 69% | miR-5000-3p | 57% | miR-6877-5p | 79% |
| miR-365a-5p | 78% | miR-5001-5p | 74% | miR-6877-3p | 68% |
| miR-449b-3p | 80% | miR-5001-3p | 53% | miR-6878-3p | 57% |
| miR-1976 | 74% | miR-5002-3p | 58% | miR-6879-5p | 76% |
| miR-2110 | 76% | miR-5003-3p | 70% | miR-6879-3p | 67% |
| let-7a-2-3p | 55% | miR-5006-5p | 56% | miR-6880-5p | 71% |
| miR-151b | 52% | miR-5007-3p | 56% | miR-6880-3p | 74% |
| miR-762 | 71% | miR-5009-3p | 55% | miR-6881-5p | 72% |
| miR-670-5p | 61% | miR-5010-5p | 69% | miR-6882-5p | 64% |
| miR-764 | 69% | miR-5010-3p | 72% | miR-6882-3p | 65% |
| miR-2116-3p | 74% | miR-5088-5p | 64% | miR-6883-5p | 62% |
| miR-548q | 70% | miR-5089-5p | 62% | miR-6883-3p | 60% |
| miR-2276-3p | 67% | miR-5090 | 68% | miR-6884-3p | 75% |
| miR-2278 | 58% | miR-5188 | 55% | miR-6885-3p | 74% |
| miR-711 | 78% | miR-5189-5p | 59% | miR-6886-5p | 63% |
| miR-718 | 76% | miR-5193 | 66% | miR-6886-3p | 71% |
| miR-2681-3p | 68% | miR-5195-5p | 71% | miR-6887-5p | 62% |
| miR-449c-3p | 53% | miR-5195-3p | 66% | miR-6887-3p | 75% |
| miR-2861 | 60% | miR-5196-5p | 81% | miR-6889-5p | 77% |
| miR-3116 | 58% | miR-5196-3p | 63% | miR-6889-3p | 74% |
| miR-3120-3p | 67% | miR-4524b-5p | 53% | miR-6890-5p | 69% |
| miR-3122 | 68% | miR-5100 | 58% | miR-6890-3p | 71% |
| miR-3126-5p | 59% | miR-5572 | 77% | miR-6891-5p | 75% |
| miR-3130-5p | 64% | miR-664b-5p | 53% | miR-6891-3p | 79% |
| miR-3131 | 76% | miR-664b-3p | 61% | miR-6892-5p | 71% |
| miR-3132 | 63% | miR-5581-3p | 61% | miR-6892-3p | 75% |
| miR-3133 | 58% | miR-5584-5p | 73% | miR-6893-5p | 78% |
| miR-466 | 53% | miR-5586-3p | 63% | miR-6893-3p | 66% |
| miR-3136-5p | 100% | miR-548au-3p | 56% | miR-6894-5p | 76% |
| miR-3137 | 53% | miR-5588-5p | 54% | miR-6894-3p | 69% |
| miR-3138 | 63% | miR-5589-5p | 64% | miR-7106-5p | 73% |
| miR-3141 | 72% | miR-5690 | 53% | miR-7106-3p | 61% |
| miR-3144-5p | 66% | miR-5698 | 73% | miR-7107-5p | 75% |
| miR-1273c | 75% | miR-5699-3p | 56% | miR-7108-5p | 74% |
| miR-3147 | 74% | miR-5703 | 65% | miR-7108-3p | 79% |
| miR-3148 | 52% | miR-5705 | 58% | miR-7109-5p | 77% |
| miR-3150a-3p | 77% | miR-5707 | 75% | miR-7109-3p | 69% |
| miR-3151-5p | 78% | miR-5708 | 52% | miR-7110-5p | 75% |
| miR-3153 | 79% | miR-197-5p | 75% | miR-7110-3p | 70% |
| miR-3154 | 71% | miR-181b-3p | 67% | miR-7111-5p | 81% |
| miR-3156-5p | 76% | miR-204-3p | 82% | miR-7111-3p | 73% |
| miR-3157-5p | 58% | miR-211-3p | 70% | miR-7112-5p | 60% |
| miR-3160-3p | 72% | miR-345-3p | 57% | miR-7113-5p | 54% |
| miR-3161 | 55% | miR-652-5p | 71% | miR-7113-3p | 72% |
| miR-3162-5p | 78% | miR-1185-2-3p | 76% | miR-7114-5p | 73% |
| miR-3163 | 85% | miR-873-3p | 62% | miR-7114-3p | 76% |
| miR-3165 | 63% | miR-1304-3p | 74% | miR-7150 | 75% |
| miR-1260b | 67% | miR-1247-3p | 77% | miR-7151-3p | 63% |
| miR-3173-3p | 57% | miR-1306-5p | 75% | miR-7152-5p | 51% |
| miR-1193 | 70% | miR-3184-3p | 71% | miR-7152-3p | 62% |
| miR-3175 | 69% | miR-3191-5p | 60% | miR-7155-5p | 68% |
| miR-3176 | 52% | miR-642b-5p | 56% | miR-7158-3p | 67% |
| miR-3177-3p | 76% | miR-365b-5p | 59% | miR-7159-5p | 67% |
| miR-3178 | 68% | miR-1185-1-3p | 79% | miR-7160-5p | 68% |
| miR-3179 | 71% | miR-3190-3p | 53% | miR-7162-5p | 53% |
| miR-3180-5p | 72% | miR-98-3p | 69% | miR-7515 | 70% |
| miR-3180-3p | 67% | miR-381-5p | 57% | miR-7702 | 60% |
| miR-3184-5p | 63% | miR-503-3p | 61% | miR-7704 | 63% |
| miR-3185 | 75% | miR-937-5p | 82% | miR-7843-5p | 60% |
| miR-3186-3p | 53% | miR-939-3p | 71% | miR-4433b-5p | 74% |
| miR-3188 | 65% | miR-1227-5p | 73% | miR-4433b-3p | 77% |
| miR-3189-3p | 69% | miR-1229-5p | 66% | miR-1273h-5p | 65% |
| miR-3191-3p | 76% | miR-1233-5p | 72% | miR-6516-5p | 55% |
| miR-3194-5p | 70% | miR-1236-5p | 64% | miR-7845-5p | 72% |
| miR-3195 | 65% | miR-1237-5p | 67% | miR-7846-3p | 69% |
| miR-3196 | 63% | miR-1238-5p | 80% | miR-7847-3p | 66% |
| miR-3197 | 72% | miR-1292-3p | 70% | miR-7851-3p | 59% |
| miR-3198 | 64% | miR-3617-3p | 51% | miR-7855-5p | 64% |
| miR-514b-5p | 67% | miR-3620-5p | 76% | miR-7856-5p | 52% |
| miR-3202 | 71% | miR-4632-5p | 67% | miR-8052 | 74% |
| miR-4297 | 60% | miR-4743-3p | 64% | miR-8054 | 75% |
| miR-4293 | 58% | miR-4750-3p | 76% | miR-8059 | 74% |
| miR-4294 | 74% | miR-5739 | 73% | miR-8060 | 72% |
| miR-4299 | 58% | miR-5787 | 63% | miR-8063 | 70% |
| miR-4298 | 72% | miR-6069 | 75% | miR-8064 | 63% |
| miR-4305 | 65% | miR-6071 | 65% | miR-8069 | 65% |
| miR-4307 | 70% | miR-6072 | 62% | miR-8071 | 77% |
| miR-4312 | 73% | miR-6075 | 68% | miR-8072 | 68% |
| miR-4313 | 72% | miR-6076 | 77% | miR-8073 | 76% |
| miR-4316 | 66% | miR-6077 | 63% | miR-8074 | 55% |
| miR-4314 | 56% | miR-6082 | 56% | miR-8075 | 56% |
| miR-4320 | 54% | miR-6085 | 73% | miR-8077 | 67% |
| miR-4322 | 65% | miR-6086 | 78% | miR-8080 | 62% |
| miR-4321 | 54% | miR-6088 | 68% | miR-8085 | 80% |
| miR-4323 | 76% | miR-6089 | 65% | miR-8087 | 78% |
| miR-4256 | 67% | miR-6090 | 64% | miR-8089 | 76% |
| miR-4257 | 80% | miR-6124 | 76% | miR-7977 | 63% |
| miR-4258 | 73% | miR-6125 | 69% | miR-7978 | 100% |
| miR-4259 | 71% | miR-6126 | 65% | miR-203a-5p | 64% |
| miR-4260 | 74% | miR-6127 | 74% | miR-1249-5p | 69% |
| miR-4253 | 58% | miR-378j | 61% | miR-4485-5p | 69% |
| miR-4251 | 60% | miR-6131 | 75% | miR-8485 | 68% |
| miR-4326 | 61% | miR-6132 | 65% | miR-196a-1-3p | 51% |
| miR-4327 | 79% | miR-6133 | 77% | miR-217-3p | 54% |
| miR-2355-5p | 71% | miR-6165 | 77% | miR-498-3p | 63% |
| miR-4268 | 67% | miR-548ay-3p | 83% | miR-526a-3p | 100% |
| miR-4269 | 72% | miR-6501-3p | 54% | miR-1912-5p | 63% |
| miR-4264 | 53% | miR-6503-5p | 72% | miR-9718 | 71% |
| miR-4271 | 72% | miR-6506-5p | 53% | miR-9898 | 73% |
| miR-4276 | 59% | miR-6506-3p | 86% | miR-9985 | 60% |
| miR-4274 | 76% | miR-6508-5p | 61% | miR-1843 | 56% |
| miR-4281 | 64% | miR-6509-3p | 59% | miR-10226 | 60% |
| miR-4279 | 69% | miR-6510-5p | 71% | miR-10392-5p | 76% |
| miR-4278 | 52% | miR-6511a-5p | 82% | miR-10392-3p | 66% |
| miR-4280 | 55% | miR-6511a-3p | 72% | miR-10394-3p | 58% |
| miR-4285 | 64% | miR-6512-3p | 59% | miR-10395-3p | 64% |
| miR-4284 | 73% | miR-6513-5p | 58% | miR-10396a-5p | 67% |
| miR-4286 | 73% | miR-6514-3p | 55% | miR-10396a-3p | 70% |
| miR-4287 | 63% | miR-6515-5p | 59% | miR-10398-5p | 66% |
| miR-4292 | 74% | miR-6515-3p | 79% | miR-10398-3p | 81% |
| miR-4289 | 53% | miR-6716-5p | 70% | miR-10399-3p | 100% |
| miR-4290 | 71% | miR-6716-3p | 61% | miR-10400-3p | 75% |
| miR-4329 | 69% | miR-6717-5p | 64% | miR-10401-5p | 78% |
| miR-500b-5p | 65% | miR-6511b-5p | 66% | miR-10401-3p | 69% |
| miR-3200-5p | 54% | miR-6511b-3p | 74% | miR-10396b-5p | 69% |
| miR-3605-3p | 63% | miR-6718-5p | 52% | miR-10522-5p | 78% |
| miR-3609 | 100% | miR-6719-3p | 58% | miR-10526-3p | 72% |
| miR-3610 | 71% | miR-6721-5p | 71% | miR-10527-5p | 75% |
| miR-3612 | 53% | miR-6722-5p | 64% | miR-11181-5p | 66% |
| miR-3614-5p | 74% | miR-6722-3p | 72% | miR-11181-3p | 72% |
| miR-3614-3p | 63% | miR-6724-5p | 73% | miR-11399 | 67% |
| miR-3616-3p | 52% | miR-208a-5p | 52% | miR-11400 | 58% |
| miR-3620-3p | 65% | miR-210-5p | 73% | miR-3059-5p | 73% |
| miR-3621 | 68% | miR-128-1-5p | 67% | miR-3085-5p | 72% |
| miR-3622a-5p | 66% | miR-328-5p | 64% | miR-3085-3p | 75% |
| miR-3622a-3p | 62% | miR-329-5p | 55% | miR-6529-5p | 58% |
| miR-3622b-5p | 72% | miR-410-5p | 54% | miR-12114 | 68% |
| miR-3646 | 61% | miR-181d-3p | 86% | miR-12115 | 69% |
| miR-3648 | 79% | miR-504-3p | 73% | miR-12116 | 78% |
| miR-3649 | 67% | miR-487b-5p | 61% | miR-12118 | 69% |
| miR-3652 | 72% | miR-585-5p | 61% | miR-12119 | 82% |
| miR-3660 | 59% | miR-598-5p | 68% | miR-12120 | 76% |
| miR-3663-5p | 70% | miR-605-3p | 64% | miR-12121 | 70% |
| miR-3663-3p | 66% | miR-619-5p | 61% | miR-12124 | 53% |
| miR-3665 | 62% | miR-655-5p | 60% | miR-12126 | 64% |
| miR-3667-3p | 70% | miR-1296-3p | 68% | miR-12131 | 100% |
| miR-3675-3p | 76% | miR-874-5p | 76% | miR-12136 | 62% |
| miR-3679-5p | 78% | miR-887-5p | 66% | | |
| miR-3679-3p | 75% | miR-1250-3p | 59% | | |

| | | | | | |
|---|---|---|---|---|---|
| ^{∗}A represents Accuracy. ^{∗}"has-" in the miRNA names has been omitted. | | | | | |

### [Table 23-9]

**Table 23-9: miRNA to Predict Human Melanoma of Stages I and II**

| miRNA Name | A | miRNA Name | A | miRNA Name | A |
|---|---|---|---|---|---|
| miR-25-3p | 71% | miR-3180 | 68% | miR-6742-3p | 74% |
| miR-197-3p | 88% | miR-3907 | 78% | miR-6743-5p | 64% |
| miR-198 | 82% | miR-3917 | 51% | miR-6745 | 73% |
| miR-210-3p | 53% | miR-3922-3p | 73% | miR-6746-5p | 89% |
| miR-224-5p | 100% | miR-3924 | 100% | miR-6746-3p | 63% |
| miR-124-3p | 60% | miR-3926 | 54% | miR-6748-5p | 80% |
| miR-185-5p | 76% | miR-3936 | 59% | miR-6749-5p | 78% |
| miR-296-5p | 73% | miR-3937 | 71% | miR-6749-3p | 79% |
| miR-365a-3p, miR-365b-3p | 73% | miR-3943 | 76% | miR-6752-5p | 69% |
| miR-302c-5p | 93% | miR-3945 | 59% | miR-6752-3p | 76% |
| miR-370-3p | 76% | miR-550b-3p | 68% | miR-6753-5p | 68% |
| miR-373-5p | 76% | miR-548ab | 60% | miR-6754-3p | 67% |
| miR-380-3p | 79% | miR-4418 | 67% | miR-6756-3p | 76% |
| miR-383-5p | 67% | miR-4432 | 100% | miR-6758-5p | 78% |
| miR-330-3p | 53% | miR-4433a-3p | 76% | miR-6758-3p | 57% |
| miR-328-3p | 79% | miR-4441 | 87% | miR-6759-5p | 51% |
| miR-449a | 60% | miR-4444 | 83% | miR-6759-3p | 75% |
| miR-483-3p | 88% | miR-4447 | 70% | miR-6760-5p | 86% |
| miR-484 | 73% | miR-4449 | 56% | miR-6760-3p | 81% |
| miR-485-5p | 64% | miR-548ah-5p | 100% | miR-6761-3p | 77% |
| miR-498-5p | 90% | miR-4462 | 69% | miR-6763-3p | 78% |
| miR-520e-3p | 80% | miR-4466 | 70% | miR-6764-3p | 65% |
| miR-519b-3p | 55% | miR-4472 | 81% | miR-6766-5p | 61% |
| miR-518f-3p | 69% | miR-3689d | 64% | miR-6766-3p | 78% |
| miR-520b-3p | 81% | miR-3155b | 75% | miR-6768-5p | 66% |
| miR-519a-3p | 55% | miR-4480 | 100% | miR-6769a-5p | 78% |
| miR-513a-5p | 89% | miR-4484 | 69% | miR-6769a-3p | 74% |
| miR-18a-3p | 52% | miR-4492 | 68% | miR-6772-3p | 54% |
| miR-575 | 54% | miR-4495 | 100% | miR-6774-5p | 79% |
| miR-583 | 76% | miR-4496 | 88% | miR-6775-5p | 76% |
| miR-611 | 58% | miR-4499 | 80% | miR-6776-3p | 70% |
| miR-625-5p | 88% | miR-4512 | 73% | miR-6777-5p | 57% |
| miR-632 | 56% | miR-4521 | 56% | miR-6777-3p | 77% |
| miR-634 | 76% | miR-4525 | 88% | miR-6779-5p | 70% |
| miR-652-3p | 100% | miR-4526 | 56% | miR-6780a-5p | 82% |
| miR-661 | 55% | miR-4531 | 79% | miR-6782-5p | 63% |
| miR-658 | 61% | miR-4534 | 78% | miR-6782-3p | 71% |
| miR-668-3p | 70% | miR-3127-3p | 81% | miR-6784-3p | 82% |
| miR-765 | 74% | miR-3158-5p | 69% | miR-6785-3p | 77% |
| miR-22-5p | 100% | miR-3162-3p | 81% | miR-6787-3p | 72% |
| miR-92a-2-5p | 58% | miR-3173-5p | 68% | miR-6788-5p | 53% |
| miR-101-5p | 57% | miR-3619-3p | 89% | miR-6789-5p | 68% |
| miR-29b-1-5p | 73% | miR-3691-3p | 55% | miR-6789-3p | 73% |
| miR-30c-2-3p | 78% | miR-3944-5p | 55% | miR-6790-5p | 52% |
| miR-181a-2-3p | 71% | miR-3978 | 92% | miR-6792-5p | 67% |
| miR-183-3p | 69% | miR-4634 | 67% | miR-6792-3p | 78% |
| miR-187-5p | 51% | miR-4637 | 100% | miR-6793-3p | 79% |
| miR-219a-1-3p | 100% | miR-4638-5p | 54% | miR-6794-5p | 88% |
| miR-30b-3p | 53% | miR-4648 | 59% | miR-6794-3p | 81% |
| miR-125b-1-3p | 100% | miR-4652-5p | 60% | miR-6795-5p | 88% |
| miR-127-5p | 71% | miR-4661-5p | 57% | miR-6795-3p | 78% |
| miR-149-3p | 68% | miR-4664-5p | 52% | miR-6796-3p | 77% |
| miR-150-3p | 83% | miR-4665-5p | 84% | miR-6797-5p | 78% |
| miR-186-3p | 56% | miR-4665-3p | 74% | miR-6797-3p | 69% |
| miR-30c-1-3p | 57% | miR-4667-3p | 69% | miR-6798-5p | 71% |
| miR-361-3p | 71% | miR-4668-5p | 100% | miR-6798-3p | 78% |
| miR-371a-5p | 69% | miR-4674 | 56% | miR-6799-5p | 70% |
| miR-342-5p | 83% | miR-4685-5p | 73% | miR-6800-3p | 79% |
| miR-18b-3p | 71% | miR-4685-3p | 73% | miR-6801-3p | 78% |
| miR-431-3p | 67% | miR-4687-5p | 76% | miR-6802-3p | 69% |
| miR-483-5p | 68% | miR-4687-3p | 70% | miR-6803-3p | 82% |
| miR-490-5p | 100% | miR-1343-3p | 73% | miR-6805-5p | 69% |
| miR-92b-5p | 63% | miR-4691-5p | 65% | miR-6805-3p | 76% |
| miR-550a-5p | 75% | miR-4691-3p | 83% | miR-6806-5p | 68% |
| miR-615-5p | 63% | miR-4697-5p | 70% | miR-6807-5p | 73% |
| miR-625-3p | 78% | miR-4698 | 80% | miR-6808-5p | 68% |
| miR-654-3p | 52% | miR-4706 | 80% | miR-6808-3p | 67% |
| miR-541-5p | 63% | miR-4707-5p | 60% | miR-6809-5p | 65% |
| miR-708-5p | 59% | miR-4707-3p | 77% | miR-6809-3p | 83% |
| miR-744-3p | 54% | miR-4712-5p | 51% | miR-6810-5p | 61% |
| miR-885-5p | 69% | miR-4715-5p | 71% | miR-6810-3p | 75% |
| miR-877-5p | 58% | miR-4716-5p | 81% | miR-6812-5p | 80% |
| miR-877-3p | 80% | miR-4716-3p | 91% | miR-6812-3p | 81% |
| miR-887-3p | 53% | miR-4717-3p | 62% | miR-6813-3p | 76% |
| miR-665 | 66% | miR-4723-5p | 81% | miR-6815-5p | 61% |
| miR-920 | 77% | miR-4723-3p | 75% | miR-6816-3p | 70% |
| miR-936 | 77% | miR-451b | 73% | miR-6817-3p | 72% |
| miR-940 | 75% | miR-4725-5p | 79% | miR-6819-3p | 77% |
| miR-943 | 75% | miR-4725-3p | 78% | miR-6820-3p | 76% |
| miR-1224-3p | 78% | miR-4726-3p | 72% | miR-6821-5p | 68% |
| miR-1225-5p | 75% | miR-4728-5p | 78% | miR-6821-3p | 76% |
| miR-1225-3p | 78% | miR-4728-3p | 72% | miR-6825-5p | 68% |
| miR-1226-3p | 72% | miR-4731-5p | 68% | miR-6826-3p | 69% |
| miR-1228-3p | 73% | miR-4731-3p | 73% | miR-6828-5p | 54% |
| miR-1229-3p | 79% | miR-4733-5p | 53% | miR-6829-5p | 77% |
| miR-1231 | 62% | miR-4738-5p | 83% | miR-6830-5p | 85% |
| miR-1234-3p | 74% | miR-4741 | 71% | miR-6830-3p | 72% |
| miR-1236-3p | 73% | miR-4745-5p | 63% | miR-6831-5p | 70% |
| miR-1237-3p | 77% | miR-4747-5p | 81% | miR-6832-5p | 59% |
| miR-1238-3p | 75% | miR-4748 | 72% | miR-6832-3p | 64% |
| miR-1182 | 82% | miR-4749-3p | 78% | miR-6833-5p | 73% |
| miR-1184 | 67% | miR-4750-5p | 52% | miR-6833-3p | 77% |
| miR-1202 | 74% | miR-4751 | 74% | miR-6780b-5p | 74% |
| miR-1203 | 63% | miR-371b-5p | 62% | miR-6780b-3p | 76% |
| miR-663b | 74% | miR-4755-3p | 61% | miR-6836-3p | 75% |
| miR-1204 | 54% | miR-4758-5p | 68% | miR-6837-3p | 66% |
| miR-1207-5p | 74% | miR-4758-3p | 72% | miR-6839-3p | 53% |
| miR-1208 | 55% | miR-4761-3p | 66% | miR-6840-5p | 77% |
| miR-1299 | 100% | miR-4769-3p | 79% | miR-6842-5p | 63% |
| miR-1249-3p | 75% | miR-4778-5p | 63% | miR-6845-3p | 79% |
| miR-1253 | 75% | miR-4781-5p | 71% | miR-6848-3p | 74% |
| miR-1255a | 100% | miR-4783-3p | 75% | miR-6849-5p | 58% |
| miR-1267 | 81% | miR-4793-5p | 54% | miR-6849-3p | 78% |
| miR-1275 | 70% | miR-4793-3p | 65% | miR-6850-5p | 61% |
| miR-1281 | 83% | miR-4800-5p | 63% | miR-6852-3p | 57% |
| miR-1292-5p | 83% | miR-4802-3p | 75% | miR-6854-3p | 52% |
| miR-1252-5p | 100% | miR-642a-3p | 70% | miR-6855-3p | 76% |
| miR-1825 | 84% | miR-4433a-5p | 72% | miR-6857-3p | 78% |
| miR-1469 | 64% | miR-4482-3p | 55% | miR-6858-3p | 75% |
| miR-1908-5p | 66% | miR-4536-3p | 100% | miR-6859-3p | 74% |
| miR-1909-5p | 65% | miR-5001-3p | 66% | miR-6769b-5p | 76% |
| miR-1911-5p | 100% | miR-5006-5p | 72% | miR-6861-3p | 75% |
| miR-1912-3p | 59% | miR-5010-5p | 58% | miR-6862-3p | 80% |
| miR-1913 | 76% | miR-5187-3p | 52% | miR-6867-5p | 73% |
| miR-1914-3p | 89% | miR-5193 | 81% | miR-6868-3p | 61% |
| miR-365a-5p | 75% | miR-5195-5p | 82% | miR-6870-5p | 71% |
| miR-196b-3p | 54% | miR-5195-3p | 74% | miR-6870-3p | 75% |
| miR-1976 | 83% | miR-5196-5p | 88% | miR-6872-5p | 72% |
| miR-670-5p | 63% | miR-548as-5p | 100% | miR-6873-5p | 53% |
| miR-2116-3p | 74% | miR-548as-3p | 100% | miR-6876-3p | 54% |
| miR-711 | 87% | miR-5579-5p | 100% | miR-6877-3p | 80% |
| miR-718 | 76% | miR-5579-3p | 100% | miR-6880-5p | 75% |
| miR-2681-3p | 57% | miR-5584-5p | 72% | miR-6880-3p | 79% |
| miR-3116 | 69% | miR-5584-3p | 58% | miR-6881-5p | 77% |
| miR-3122 | 60% | miR-5585-5p | 71% | miR-6883-5p | 69% |
| miR-3130-5p | 70% | miR-5587-3p | 51% | miR-6883-3p | 70% |
| miR-3131 | 85% | miR-5698 | 69% | miR-6884-3p | 77% |
| miR-3132 | 71% | miR-211-3p | 56% | miR-6885-3p | 75% |
| miR-3133 | 52% | miR-345-3p | 65% | miR-6886-3p | 81% |
| miR-3138 | 58% | miR-652-5p | 72% | miR-6887-5p | 86% |
| miR-3144-5p | 87% | miR-1185-2-3p | 87% | miR-6887-3p | 72% |
| miR-3147 | 66% | miR-873-3p | 75% | miR-6889-5p | 70% |
| miR-3153 | 72% | miR-1247-3p | 71% | miR-6889-3p | 73% |
| miR-3074-3p | 55% | miR-3184-3p | 73% | miR-6891-5p | 77% |
| miR-3155a | 59% | miR-365b-5p | 52% | miR-6891-3p | 74% |
| miR-3156-5p | 81% | miR-1185-1-3p | 85% | miR-6892-3p | 76% |
| miR-3162-5p | 67% | miR-503-3p | 82% | miR-6893-5p | 66% |
| miR-3173-3p | 52% | miR-1229-5p | 72% | miR-6893-3p | 67% |
| miR-1193 | 65% | miR-1238-5p | 77% | miR-6894-3p | 73% |
| miR-3178 | 61% | miR-4750-3p | 78% | miR-6895-5p | 62% |
| miR-3196 | 65% | miR-5739 | 74% | miR-6895-3p | 66% |
| miR-3198 | 60% | miR-6069 | 72% | miR-7106-5p | 80% |
| miR-3200-3p | 53% | miR-6071 | 56% | miR-7107-5p | 79% |
| miR-514b-5p | 77% | miR-6085 | 81% | miR-7108-5p | 60% |
| miR-3202 | 78% | miR-6086 | 89% | miR-7108-3p | 81% |
| miR-3065-3p | 51% | miR-6088 | 71% | miR-7109-5p | 78% |
| miR-4297 | 56% | miR-6124 | 82% | miR-7109-3p | 74% |
| miR-4293 | 65% | miR-6133 | 66% | miR-7110-3p | 81% |
| miR-4294 | 76% | miR-6165 | 75% | miR-7111-5p | 77% |
| miR-4299 | 52% | miR-6501-5p | 54% | miR-7111-3p | 77% |
| miR-4298 | 71% | miR-6503-3p | 62% | miR-7112-5p | 67% |
| miR-4306 | 77% | miR-6505-3p | 53% | miR-7113-3p | 77% |
| miR-4312 | 84% | miR-6507-5p | 78% | miR-7114-3p | 78% |
| miR-4313 | 75% | miR-6511a-5p | 87% | miR-7150 | 73% |
| miR-4316 | 67% | miR-6512-5p | 100% | miR-7151-5p | 57% |
| miR-4322 | 54% | miR-6512-3p | 53% | miR-7152-5p | 65% |
| miR-4323 | 75% | miR-6514-3p | 56% | miR-7161-5p | 54% |
| miR-4257 | 76% | miR-6515-3p | 76% | miR-7843-5p | 73% |
| miR-4258 | 66% | miR-6716-5p | 82% | miR-4433b-5p | 79% |
| miR-4259 | 69% | miR-6511b-5p | 86% | miR-4433b-3p | 87% |
| miR-4260 | 72% | miR-6719-3p | 67% | miR-1273h-5p | 56% |
| miR-4253 | 76% | miR-6720-3p | 67% | miR-7852-3p | 100% |
| miR-4251 | 67% | miR-6722-5p | 67% | miR-7855-5p | 83% |
| miR-4326 | 76% | miR-208a-5p | 51% | miR-8059 | 72% |
| miR-4327 | 72% | miR-210-5p | 73% | miR-8071 | 53% |
| miR-4265 | 51% | miR-383-3p | 54% | miR-8078 | 59% |
| miR-4267 | 51% | miR-487a-5p | 51% | miR-8079 | 59% |
| miR-2355-5p | 83% | miR-489-5p | 60% | miR-8087 | 88% |
| miR-4271 | 76% | miR-511-3p | 100% | miR-128-2-5p | 73% |
| miR-4276 | 57% | miR-181d-3p | 79% | miR-7974 | 58% |
| miR-4274 | 77% | miR-598-5p | 55% | miR-1249-5p | 69% |
| miR-4281 | 81% | miR-605-3p | 53% | miR-196a-1-3p | 59% |
| miR-4279 | 81% | miR-619-5p | 51% | miR-137-5p | 100% |
| miR-4280 | 52% | miR-627-3p | 100% | miR-190b-3p | 100% |
| miR-4284 | 78% | miR-1296-3p | 76% | miR-9898 | 77% |
| miR-4286 | 81% | miR-1468-3p | 67% | miR-10392-3p | 70% |
| miR-4288 | 58% | miR-874-5p | 69% | miR-10394-3p | 65% |
| miR-4290 | 84% | miR-216b-3p | 55% | miR-10396a-3p | 69% |
| miR-3200-5p | 72% | miR-208b-5p | 100% | miR-10398-5p | 74% |
| miR-3610 | 66% | miR-1287-3p | 58% | miR-10400-3p | 61% |
| miR-3612 | 87% | miR-3151-3p | 77% | miR-10401-5p | 63% |
| miR-3614-5p | 75% | miR-1343-5p | 63% | miR-10522-5p | 85% |
| miR-3615 | 52% | miR-5699-5p | 75% | miR-10526-3p | 94% |
| miR-3616-3p | 54% | miR-6728-5p | 58% | miR-11181-3p | 66% |
| miR-3619-5p | 56% | miR-6728-3p | 73% | miR-3059-5p | 73% |
| miR-3622a-5p | 55% | miR-6729-3p | 73% | miR-3059-3p | 66% |
| miR-3622a-3p | 64% | miR-6730-5p | 72% | miR-3085-3p | 71% |
| miR-3646 | 57% | miR-6731-3p | 76% | miR-12116 | 73% |
| miR-3648 | 69% | miR-6732-3p | 73% | miR-12118 | 66% |
| miR-3675-3p | 77% | miR-6734-5p | 74% | miR-12119 | 83% |
| miR-3679-5p | 74% | miR-6738-5p | 73% | miR-12128 | 71% |
| miR-3679-3p | 76% | miR-6740-3p | 84% | miR-12131 | 100% |
| miR-3713 | 54% | miR-6741-3p | 78% | | |
| miR-3714 | 74% | miR-6742-5p | 96% | | |

| | | | | | |
|---|---|---|---|---|---|
| ^{∗} A represents Accuracy. ^{∗} "has-" in the miRNA names has been omitted. | | | | | |

### [Table 23-10]

**Table 23-10: miRNA to Predict Human Ovarian Cancer of Stages I and II**

| miRNA Name | A | miRNA Name | A | miRNA Name | A |
|---|---|---|---|---|---|
| miR-25-3p | 79% | miR-3170 | 69% | miR-5587-3p | 71% |
| miR-129-5p | 63% | miR-3187-3p | 67% | miR-5685 | 65% |
| miR-221-3p | 61% | miR-3200-3p | 71% | miR-5681b | 62% |
| miR-27b-3p | 55% | miR-3065-3p | 51% | miR-5693 | 59% |
| miR-143-3p | 69% | miR-4293 | 57% | miR-5694 | 61% |
| miR-146a-5p | 63% | miR-4321 | 51% | miR-660-3p | 60% |
| miR-154-5p | 58% | miR-4280 | 56% | miR-1285-5p | 60% |
| miR-106b-5p | 75% | miR-4285 | 53% | miR-3190-3p | 62% |
| miR-130b-3p | 67% | miR-4289 | 52% | miR-216a-3p | 59% |
| miR-371a-3p | 54% | miR-3651 | 63% | miR-3927-5p | 53% |
| miR-375-3p | 61% | miR-3659 | 55% | miR-6068 | 67% |
| miR-378a-5p | 67% | miR-3666 | 61% | miR-6071 | 62% |
| miR-380-3p | 70% | miR-3677-3p | 60% | miR-6081 | 69% |
| miR-382-5p | 52% | miR-3688-3p | 67% | miR-6083 | 57% |
| miR-330-3p | 58% | miR-3689a-3p | 56% | miR-6500-5p | 62% |
| miR-422a | 59% | miR-3690 | 55% | miR-6501-5p | 74% |
| miR-431-5p | 60% | miR-3692-3p | 58% | miR-6501-3p | 53% |
| miR-432-5p | 64% | miR-3713 | 73% | miR-6502-5p | 56% |
| miR-519b-3p | 62% | miR-3907 | 82% | miR-6503-3p | 69% |
| miR-525-3p | 55% | miR-3923 | 100% | miR-6504-5p | 51% |
| miR-519a-3p | 58% | miR-3934-5p | 55% | miR-6504-3p | 54% |
| miR-539-5p | 64% | miR-3936 | 67% | miR-6505-3p | 56% |
| miR-571 | 89% | miR-4433a-3p | 73% | miR-6510-3p | 65% |
| miR-580-3p | 62% | miR-4436a | 51% | miR-6512-5p | 100% |
| miR-584-5p | 55% | miR-4439 | 58% | miR-6715a-3p | 63% |
| miR-589-3p | 63% | miR-4444 | 62% | miR-6719-3p | 56% |
| miR-591 | 66% | miR-4449 | 67% | let-7c-3p | 66% |
| miR-608 | 52% | miR-548ah-5p | 100% | miR-383-3p | 70% |
| miR-611 | 67% | miR-4460 | 60% | miR-433-5p | 61% |
| miR-613 | 55% | miR-4465 | 63% | miR-487a-5p | 62% |
| miR-622 | 63% | miR-4473 | 70% | miR-489-5p | 67% |
| miR-623 | 63% | miR-3689d | 64% | miR-494-5p | 59% |
| miR-639 | 67% | miR-3155b | 77% | miR-552-5p | 56% |
| miR-645 | 57% | miR-548al | 100% | miR-598-5p | 61% |
| miR-650 | 70% | miR-4494 | 68% | miR-627-3p | 100% |
| miR-421 | 61% | miR-4502 | 58% | miR-1301-5p | 73% |
| miR-542-5p | 80% | miR-4512 | 83% | miR-1298-3p | 57% |
| miR-15a-3p | 59% | miR-4521 | 65% | miR-1251-3p | 61% |
| miR-22-5p | 100% | miR-378i | 64% | miR-6739-3p | 55% |
| miR-25-5p | 69% | miR-4537 | 57% | miR-6761-5p | 67% |
| miR-27a-5p | 75% | miR-4538 | 57% | miR-6764-3p | 71% |
| miR-181a-2-3p | 73% | miR-3129-3p | 51% | miR-6768-3p | 56% |
| miR-181c-3p | 67% | miR-3157-3p | 55% | miR-6788-5p | 58% |
| miR-125b-1-3p | 100% | miR-3922-5p | 56% | miR-6791-3p | 66% |
| miR-141-5p | 56% | miR-3944-5p | 60% | miR-6795-5p | 57% |
| miR-127-5p | 71% | miR-3978 | 93% | miR-6808-3p | 61% |
| miR-186-3p | 63% | miR-4634 | 71% | miR-6809-5p | 65% |
| miR-339-3p | 59% | miR-4637 | 100% | miR-6821-3p | 78% |
| miR-146b-3p | 64% | miR-4648 | 70% | miR-6838-5p | 56% |
| miR-501-3p | 100% | miR-4653-5p | 59% | miR-6839-3p | 53% |
| miR-509-5p | 60% | miR-4658 | 58% | miR-6854-3p | 68% |
| miR-654-3p | 66% | miR-219b-5p | 65% | miR-6856-3p | 67% |
| miR-892a | 66% | miR-4682 | 53% | miR-6876-5p | 55% |
| miR-541-5p | 63% | miR-4684-3p | 73% | miR-6895-5p | 75% |
| miR-665 | 77% | miR-4691-5p | 78% | miR-7113-5p | 52% |
| miR-943 | 66% | miR-4691-3p | 92% | miR-7151-5p | 56% |
| miR-1226-5p | 57% | miR-4701-3p | 55% | miR-7155-3p | 78% |
| miR-513c-5p | 58% | miR-4708-5p | 71% | miR-7156-3p | 66% |
| miR-320c | 57% | miR-4708-3p | 60% | miR-7157-5p | 56% |
| miR-1286 | 53% | miR-4711-3p | 53% | miR-7158-3p | 57% |
| miR-1291 | 65% | miR-4712-5p | 59% | miR-7161-5p | 54% |
| miR-1250-5p | 58% | miR-4713-3p | 67% | miR-7706 | 59% |
| miR-1253 | 63% | miR-4715-5p | 69% | miR-4433b-3p | 72% |
| miR-1255a | 100% | miR-4724-3p | 66% | miR-7850-5p | 55% |
| miR-1269a | 66% | miR-4733-5p | 51% | miR-7851-3p | 63% |
| miR-1292-5p | 82% | miR-4761-5p | 55% | miR-8064 | 65% |
| miR-1911-3p | 65% | miR-4768-3p | 51% | miR-8078 | 69% |
| miR-1912-3p | 71% | miR-4772-3p | 67% | miR-8079 | 66% |
| miR-1914-3p | 70% | miR-4778-5p | 54% | miR-8080 | 69% |
| miR-103a-2-5p | 61% | miR-4781-5p | 60% | miR-7974 | 73% |
| miR-224-3p | 62% | miR-4793-5p | 60% | miR-103a-1-5p | 76% |
| miR-196b-3p | 65% | miR-4797-5p | 100% | miR-137-5p | 100% |
| miR-1973 | 100% | miR-4799-5p | 53% | miR-9902 | 74% |
| miR-449c-5p | 70% | miR-4802-3p | 63% | miR-9986 | 54% |
| miR-761 | 57% | miR-5002-5p | 69% | miR-10396a-3p | 80% |
| miR-2115-3p | 100% | miR-5006-5p | 72% | miR-10522-5p | 80% |
| miR-2681-5p | 58% | miR-5091 | 61% | miR-10526-3p | 63% |
| miR-2681-3p | 57% | miR-5187-3p | 67% | miR-3059-3p | 52% |
| miR-3130-3p | 62% | miR-5571-3p | 67% | miR-6529-5p | 54% |
| miR-548v | 100% | miR-5579-5p | 100% | miR-12122 | 51% |
| miR-3155a | 73% | miR-5579-3p | 100% | miR-12131 | 100% |

| | | | | | |
|---|---|---|---|---|---|
| ^{∗} A represents Accuracy. ^{∗} "has-" in the miRNA names has been omitted. | | | | | |

### [Table 23-11]

**Table 23-11: miRNA to Predict Human Thyroid Cancer of Stages I and II**

| miRNA Name | A | miRNA Name | A | miRNA Name | A |
|---|---|---|---|---|---|
| miR-17-3p | 100% | miR-3928-3p | 76% | miR-6728-5p | 66% |
| miR-25-3p | 73% | miR-3936 | 51% | miR-6728-3p | 73% |
| miR-197-3p | 86% | miR-3937 | 79% | miR-6729-5p | 83% |
| miR-198 | 88% | miR-3943 | 76% | miR-6729-3p | 69% |
| miR-139-5p | 100% | miR-3944-3p | 60% | miR-6730-5p | 81% |
| miR-124-3p | 60% | miR-1268b | 78% | miR-6731-3p | 78% |
| miR-185-5p | 80% | miR-4429 | 56% | miR-6732-5p | 80% |
| miR-296-5p | 71% | miR-4430 | 58% | miR-6732-3p | 75% |
| miR-365a-3p, miR-365b-3p | 73% | miR-4431 | 54% | miR-6734-5p | 82% |
| miR-302c-5p | 92% | miR-4432 | 100% | miR-6736-3p | 69% |
| miR-370-3p | 77% | miR-4433a-3p | 84% | miR-6737-5p | 73% |
| miR-373-5p | 78% | miR-4441 | 83% | miR-6738-5p | 79% |
| miR-383-5p | 67% | miR-4444 | 86% | miR-6738-3p | 53% |
| miR-330-3p | 56% | miR-4446-3p | 71% | miR-6740-3p | 77% |
| miR-328-3p | 81% | miR-4447 | 71% | miR-6741-3p | 78% |
| miR-324-5p | 56% | miR-4448 | 53% | miR-6742-5p | 95% |
| miR-324-3p | 72% | miR-4449 | 81% | miR-6742-3p | 78% |
| miR-346 | 70% | miR-3135b | 59% | miR-6743-5p | 84% |
| miR-422a | 56% | miR-4463 | 71% | miR-6743-3p | 81% |
| miR-483-3p | 89% | miR-4466 | 82% | miR-6745 | 75% |
| miR-484 | 70% | miR-4467 | 77% | miR-6746-5p | 90% |
| miR-485-5p | 55% | miR-4470 | 67% | miR-6746-3p | 81% |
| miR-491-5p | 78% | miR-4472 | 88% | miR-6748-5p | 82% |
| miR-493-5p | 57% | miR-4473 | 52% | miR-6749-5p | 81% |
| miR-498-5p | 93% | miR-4476 | 54% | miR-6749-3p | 77% |
| miR-520e-3p | 90% | miR-4477b | 100% | miR-6750-5p | 54% |
| miR-519b-3p | 57% | miR-3689d | 60% | miR-6751-5p | 55% |
| miR-525-3p | 52% | miR-4479 | 56% | miR-6752-5p | 86% |
| miR-518f-3p | 77% | miR-3155b | 75% | miR-6752-3p | 75% |
| miR-520b-3p | 88% | miR-4480 | 100% | miR-6753-5p | 69% |
| miR-513a-5p | 89% | miR-4483 | 68% | miR-6754-3p | 66% |
| miR-557 | 68% | miR-4484 | 81% | miR-6756-5p | 68% |
| miR-572 | 55% | miR-4486 | 74% | miR-6756-3p | 77% |
| miR-574-3p | 72% | miR-4488 | 75% | miR-6758-5p | 84% |
| miR-575 | 59% | miR-4489 | 52% | miR-6759-5p | 69% |
| miR-583 | 77% | miR-4492 | 78% | miR-6759-3p | 73% |
| miR-550a-3p | 66% | miR-4494 | 57% | miR-6760-5p | 83% |
| miR-611 | 62% | miR-4495 | 100% | miR-6760-3p | 81% |
| miR-612 | 69% | miR-4496 | 85% | miR-6761-5p | 55% |
| miR-613 | 55% | miR-4497 | 81% | miR-6761-3p | 80% |
| miR-625-5p | 76% | miR-4499 | 82% | miR-6762-5p | 68% |
| miR-634 | 76% | miR-4505 | 69% | miR-6762-3p | 51% |
| miR-636 | 84% | miR-4507 | 66% | miR-6763-5p | 71% |
| miR-638 | 79% | miR-4508 | 76% | miR-6763-3p | 78% |
| miR-639 | 75% | miR-4512 | 80% | miR-6764-3p | 64% |
| miR-650 | 56% | miR-4513 | 72% | miR-6765-5p | 74% |
| miR-652-3p | 100% | miR-4516 | 77% | miR-6766-5p | 73% |
| miR-661 | 61% | miR-4521 | 70% | miR-6766-3p | 79% |
| miR-663a | 71% | miR-4525 | 93% | miR-6768-5p | 76% |
| miR-654-5p | 62% | miR-4526 | 66% | miR-6769a-3p | 74% |
| miR-658 | 76% | miR-4530 | 79% | miR-6770-3p | 58% |
| miR-542-5p | 86% | miR-4531 | 77% | miR-6772-3p | 55% |
| miR-376a-5p | 100% | miR-4533 | 59% | miR-6774-5p | 80% |
| miR-668-3p | 72% | miR-4534 | 73% | miR-6775-5p | 69% |
| miR-766-3p | 76% | miR-548am-3p | 100% | miR-6776-5p | 61% |
| miR-765 | 73% | miR-1587 | 53% | miR-6776-3p | 79% |
| miR-297 | 59% | miR-4536-5p | 100% | miR-6777-5p | 70% |
| miR-22-5p | 100% | miR-4539 | 66% | miR-6777-3p | 76% |
| miR-92a-2-5p | 65% | miR-3120-5p | 52% | miR-6779-5p | 69% |
| miR-29b-1-5p | 57% | miR-3127-3p | 83% | miR-6781-5p | 78% |
| miR-30c-2-3p | 87% | miR-3129-3p | 51% | miR-6782-5p | 82% |
| miR-139-3p | 60% | miR-3150a-5p | 51% | miR-6782-3p | 77% |
| miR-181a-2-3p | 71% | miR-3158-5p | 73% | miR-6784-5p | 77% |
| miR-183-3p | 69% | miR-3162-3p | 81% | miR-6784-3p | 80% |
| miR-187-5p | 68% | miR-3173-5p | 70% | miR-6785-3p | 78% |
| miR-135a-3p | 63% | miR-3187-5p | 77% | miR-6786-5p | 69% |
| miR-127-5p | 71% | miR-3619-3p | 79% | miR-6787-5p | 73% |
| miR-149-3p | 75% | miR-3691-3p | 54% | miR-6787-3p | 72% |
| miR-150-3p | 80% | miR-3150b-5p | 79% | miR-6789-5p | 88% |
| miR-185-3p | 53% | miR-3940-5p | 69% | miR-6789-3p | 75% |
| miR-186-3p | 53% | miR-3960 | 65% | miR-6790-5p | 82% |
| miR-194-3p | 57% | miR-3972 | 75% | miR-6790-3p | 86% |
| miR-30c-1-3p | 60% | miR-3978 | 88% | miR-6791-5p | 78% |
| miR-296-3p | 65% | miR-4634 | 85% | miR-6792-5p | 75% |
| miR-361-3p | 75% | miR-4637 | 100% | miR-6792-3p | 81% |
| miR-371a-5p | 67% | miR-4639-5p | 100% | miR-6793-3p | 85% |
| miR-342-5p | 91% | miR-4640-5p | 77% | miR-6794-5p | 88% |
| miR-339-3p | 53% | miR-4648 | 68% | miR-6795-5p | 87% |
| miR-424-3p | 55% | miR-4649-5p | 76% | miR-6795-3p | 79% |
| miR-18b-3p | 75% | miR-4651 | 78% | miR-6796-3p | 77% |
| miR-431-3p | 68% | miR-4652-5p | 62% | miR-6797-5p | 79% |
| miR-483-5p | 61% | miR-4655-5p | 58% | miR-6797-3p | 70% |
| miR-486-3p | 76% | miR-4661-5p | 54% | miR-6798-5p | 81% |
| miR-509-5p | 64% | miR-4664-5p | 69% | miR-6798-3p | 80% |
| miR-92b-5p | 83% | miR-4664-3p | 78% | miR-6799-5p | 72% |
| miR-550a-5p | 73% | miR-4665-5p | 89% | miR-6799-3p | 84% |
| miR-615-5p | 76% | miR-4665-3p | 76% | miR-6800-5p | 74% |
| miR-625-3p | 76% | miR-4667-3p | 71% | miR-6800-3p | 75% |
| miR-744-5p | 71% | miR-4668-5p | 100% | miR-6801-3p | 77% |
| miR-744-3p | 63% | miR-4673 | 60% | miR-6802-5p | 64% |
| miR-885-5p | 71% | miR-4674 | 77% | miR-6802-3p | 70% |
| miR-885-3p | 56% | miR-4684-3p | 62% | miR-6803-5p | 62% |
| miR-877-5p | 58% | miR-4685-5p | 69% | miR-6803-3p | 81% |
| miR-877-3p | 82% | miR-4685-3p | 75% | miR-6804-3p | 79% |
| miR-887-3p | 69% | miR-4687-5p | 75% | miR-6805-5p | 81% |
| miR-665 | 75% | miR-4687-3p | 67% | miR-6805-3p | 77% |
| miR-374b-3p | 100% | miR-1343-3p | 85% | miR-6806-5p | 74% |
| miR-760 | 71% | miR-4688 | 69% | miR-6808-5p | 68% |
| miR-920 | 76% | miR-4690-5p | 77% | miR-6808-3p | 53% |
| miR-935 | 63% | miR-4691-5p | 63% | miR-6809-5p | 58% |
| miR-936 | 72% | miR-4691-3p | 90% | miR-6809-3p | 85% |
| miR-937-3p | 69% | miR-4695-5p | 64% | miR-6810-5p | 71% |
| miR-939-5p | 77% | miR-4697-5p | 69% | miR-6810-3p | 75% |
| miR-940 | 75% | miR-4697-3p | 75% | miR-6812-5p | 75% |
| miR-943 | 68% | miR-4706 | 75% | miR-6812-3p | 85% |
| miR-1224-3p | 75% | miR-4707-5p | 82% | miR-6813-5p | 64% |
| miR-1225-5p | 79% | miR-4707-3p | 79% | miR-6813-3p | 76% |
| miR-1225-3p | 78% | miR-4708-5p | 60% | miR-6815-5p | 63% |
| miR-1226-5p | 54% | miR-4708-3p | 68% | miR-6816-5p | 80% |
| miR-1226-3p | 71% | miR-4714-5p | 77% | miR-6816-3p | 72% |
| miR-1228-5p | 71% | miR-4715-5p | 55% | miR-6817-3p | 65% |
| miR-1228-3p | 72% | miR-4716-5p | 79% | miR-6819-3p | 77% |
| miR-1229-3p | 80% | miR-4716-3p | 89% | miR-6820-5p | 70% |
| miR-1231 | 84% | miR-4717-3p | 65% | miR-6820-3p | 76% |
| miR-1234-3p | 74% | miR-4722-5p | 70% | miR-6821-5p | 78% |
| miR-1237-3p | 74% | miR-4723-5p | 80% | miR-6821-3p | 80% |
| miR-1238-3p | 74% | miR-4723-3p | 74% | miR-6822-5p | 74% |
| miR-1181 | 84% | miR-4725-5p | 77% | miR-6823-3p | 72% |
| miR-1182 | 79% | miR-4725-3p | 82% | miR-6824-3p | 83% |
| miR-1184 | 67% | miR-4726-3p | 65% | miR-6825-5p | 73% |
| miR-1203 | 74% | miR-4728-5p | 78% | miR-6825-3p | 81% |
| miR-663b | 76% | miR-4728-3p | 70% | miR-6827-5p | 77% |
| miR-1204 | 63% | miR-4730 | 66% | miR-6828-5p | 53% |
| miR-1207-5p | 86% | miR-4731-5p | 64% | miR-6829-5p | 83% |
| miR-1207-3p | 62% | miR-4731-3p | 73% | miR-6830-5p | 93% |
| miR-1208 | 55% | miR-4732-5p | 63% | miR-6830-3p | 74% |
| miR-1299 | 100% | miR-4734 | 75% | miR-6832-5p | 53% |
| miR-1303 | 56% | miR-4736 | 56% | miR-6832-3p | 62% |
| miR-1246 | 67% | miR-4738-5p | 75% | miR-6833-5p | 69% |
| miR-1249-3p | 77% | miR-4738-3p | 58% | miR-6833-3p | 82% |
| miR-1253 | 63% | miR-4741 | 79% | miR-6780b-5p | 77% |
| miR-1255a | 100% | miR-4745-5p | 80% | miR-6780b-3p | 77% |
| miR-1260a | 78% | miR-4746-3p | 66% | miR-6836-5p | 52% |
| miR-1266-5p | 70% | miR-4747-5p | 81% | miR-6836-3p | 84% |
| miR-1267 | 82% | miR-4748 | 67% | miR-6837-3p | 63% |
| miR-1268a | 71% | miR-4749-5p | 68% | miR-6838-5p | 63% |
| miR-1269a | 65% | miR-4749-3p | 78% | miR-6840-5p | 76% |
| miR-1275 | 75% | miR-4750-5p | 62% | miR-6840-3p | 80% |
| miR-1281 | 83% | miR-371b-5p | 78% | miR-6842-5p | 71% |
| miR-1292-5p | 82% | miR-371b-3p | 70% | miR-6845-5p | 80% |
| miR-1307-3p | 61% | miR-4755-3p | 61% | miR-6845-3p | 82% |
| miR-1321 | 51% | miR-4756-5p | 72% | miR-6846-5p | 73% |
| miR-1825 | 86% | miR-4758-5p | 84% | miR-6848-5p | 71% |
| miR-1469 | 82% | miR-4758-3p | 73% | miR-6848-3p | 72% |
| miR-1470 | 85% | miR-4761-3p | 66% | miR-6849-5p | 64% |
| miR-1538 | 57% | miR-4763-5p | 84% | miR-6849-3p | 82% |
| miR-1539 | 72% | miR-4763-3p | 73% | miR-6850-5p | 79% |
| miR-1908-5p | 83% | miR-4764-5p | 100% | miR-6855-3p | 78% |
| miR-1909-5p | 63% | miR-4767 | 55% | miR-6857-5p | 70% |
| miR-1909-3p | 78% | miR-4769-3p | 77% | miR-6857-3p | 78% |
| miR-1910-5p | 86% | miR-4778-5p | 60% | miR-6858-3p | 78% |
| miR-1912-3p | 56% | miR-4780 | 60% | miR-6859-3p | 75% |
| miR-1913 | 74% | miR-4436b-5p | 79% | miR-6860 | 55% |
| miR-1914-3p | 91% | miR-4781-5p | 71% | miR-6861-5p | 71% |
| miR-1915-3p | 78% | miR-4783-3p | 76% | miR-6861-3p | 76% |
| miR-365a-5p | 73% | miR-4786-3p | 56% | miR-6862-5p | 54% |
| miR-449b-3p | 77% | miR-4787-5p | 78% | miR-6862-3p | 86% |
| miR-1972 | 58% | miR-4787-3p | 86% | miR-6865-3p | 84% |
| miR-1976 | 84% | miR-4791 | 100% | miR-6867-5p | 59% |
| miR-2052 | 100% | miR-4793-3p | 74% | miR-6868-3p | 52% |
| miR-2110 | 75% | miR-4798-3p | 100% | miR-6869-5p | 72% |
| miR-449c-5p | 59% | miR-4800-5p | 68% | miR-6870-5p | 74% |
| miR-762 | 79% | miR-4800-3p | 53% | miR-6870-3p | 76% |
| miR-670-5p | 64% | miR-4802-3p | 57% | miR-6872-5p | 72% |
| miR-2116-3p | 74% | miR-4433a-5p | 77% | miR-6875-5p | 74% |
| miR-548q | 59% | miR-4482-3p | 77% | miR-6875-3p | 70% |
| miR-2276-3p | 57% | miR-4536-3p | 100% | miR-6876-3p | 52% |
| miR-2277-3p | 81% | miR-5001-5p | 76% | miR-6877-5p | 77% |
| miR-711 | 87% | miR-5001-3p | 53% | miR-6877-3p | 82% |
| miR-718 | 83% | miR-5002-5p | 54% | miR-6878-5p | 56% |
| miR-2681-3p | 52% | miR-5002-3p | 54% | miR-6878-3p | 75% |
| miR-3116 | 80% | miR-5008-5p | 71% | miR-6879-3p | 76% |
| miR-3122 | 72% | miR-5010-5p | 77% | miR-6880-5p | 78% |
| miR-3124-5p | 52% | miR-5088-5p | 60% | miR-6880-3p | 80% |
| miR-3126-5p | 53% | miR-5090 | 72% | miR-6881-5p | 75% |
| miR-3130-3p | 59% | miR-5092 | 67% | miR-6882-3p | 74% |
| miR-3130-5p | 66% | miR-5190 | 56% | miR-6883-5p | 80% |
| miR-3131 | 85% | miR-5193 | 83% | miR-6884-3p | 78% |
| miR-3132 | 65% | miR-5195-5p | 78% | miR-6885-3p | 74% |
| miR-3133 | 55% | miR-5195-3p | 74% | miR-6886-5p | 53% |
| miR-3138 | 53% | miR-5196-5p | 83% | miR-6886-3p | 86% |
| miR-3141 | 69% | miR-5572 | 71% | miR-6887-5p | 88% |
| miR-3144-5p | 83% | miR-548as-5p | 100% | miR-6887-3p | 73% |
| miR-3147 | 75% | miR-548as-3p | 100% | miR-6889-5p | 81% |
| miR-3151-5p | 70% | miR-5579-5p | 100% | miR-6889-3p | 74% |
| miR-3153 | 78% | miR-5579-3p | 100% | miR-6890-3p | 77% |
| miR-3074-3p | 52% | miR-5587-3p | 58% | miR-6891-5p | 75% |
| miR-3155a | 70% | miR-5589-5p | 51% | miR-6891-3p | 77% |
| miR-3156-5p | 76% | miR-5681a | 56% | miR-6892-3p | 76% |
| miR-3158-3p | 53% | miR-5685 | 51% | miR-6893-5p | 74% |
| miR-3162-5p | 74% | miR-5698 | 76% | miR-6893-3p | 69% |
| miR-3164 | 70% | miR-5705 | 60% | miR-6894-3p | 81% |
| miR-1260b | 72% | miR-211-3p | 73% | miR-6895-3p | 69% |
| miR-3173-3p | 58% | miR-345-3p | 78% | miR-7106-5p | 81% |
| miR-1193 | 80% | miR-584-3p | 51% | miR-7106-3p | 66% |
| miR-3177-3p | 85% | miR-652-5p | 75% | miR-7107-5p | 78% |
| miR-3178 | 81% | miR-1185-2-3p | 82% | miR-7108-5p | 77% |
| miR-3180-3p | 75% | miR-873-3p | 73% | miR-7108-3p | 79% |
| miR-3185 | 70% | miR-1247-3p | 77% | miR-7109-5p | 75% |
| miR-3186-3p | 66% | miR-3184-3p | 75% | miR-7109-3p | 75% |
| miR-3187-3p | 68% | miR-365b-5p | 67% | miR-7110-3p | 82% |
| miR-3188 | 75% | miR-1185-1-3p | 86% | miR-7111-5p | 81% |
| miR-3189-3p | 56% | miR-98-3p | 87% | miR-7111-3p | 75% |
| miR-3195 | 75% | miR-503-3p | 83% | miR-7112-5p | 71% |
| miR-3196 | 80% | miR-937-5p | 69% | miR-7113-3p | 81% |
| miR-3197 | 76% | miR-1227-5p | 79% | miR-7114-5p | 69% |
| miR-3199 | 51% | miR-1233-5p | 69% | miR-7114-3p | 80% |
| miR-3200-3p | 51% | miR-1237-5p | 78% | miR-7150 | 71% |
| miR-514b-5p | 74% | miR-1238-5p | 81% | miR-7151-5p | 52% |
| miR-4297 | 62% | miR-1292-3p | 77% | miR-7152-5p | 65% |
| miR-4293 | 63% | miR-3620-5p | 69% | miR-7152-3p | 57% |
| miR-4294 | 76% | miR-4750-3p | 79% | miR-7155-3p | 56% |
| miR-4299 | 52% | miR-5739 | 75% | miR-7704 | 81% |
| miR-4298 | 68% | miR-5787 | 70% | miR-7706 | 51% |
| miR-4306 | 92% | miR-6068 | 67% | miR-7843-5p | 72% |
| miR-4307 | 73% | miR-6069 | 74% | miR-4433b-5p | 75% |
| miR-4312 | 85% | miR-6071 | 65% | miR-4433b-3p | 93% |
| miR-4313 | 78% | miR-6075 | 77% | miR-1273h-5p | 54% |
| miR-4316 | 64% | miR-6076 | 64% | miR-7851-3p | 55% |
| miR-4322 | 65% | miR-6085 | 81% | miR-7855-5p | 83% |
| miR-4321 | 57% | miR-6086 | 81% | miR-8059 | 74% |
| miR-4323 | 69% | miR-6088 | 73% | miR-8063 | 69% |
| miR-4257 | 78% | miR-6089 | 78% | miR-8064 | 58% |
| miR-4258 | 72% | miR-6090 | 74% | miR-8069 | 75% |
| miR-4259 | 74% | miR-6124 | 80% | miR-8071 | 53% |
| miR-4260 | 78% | miR-6125 | 79% | miR-8072 | 73% |
| miR-4253 | 77% | miR-6126 | 69% | miR-8073 | 75% |
| miR-4251 | 83% | miR-6127 | 67% | miR-8078 | 70% |
| miR-4254 | 61% | miR-6131 | 66% | miR-8079 | 56% |
| miR-4326 | 75% | miR-6132 | 68% | miR-8080 | 63% |
| miR-4327 | 75% | miR-6133 | 71% | miR-8087 | 82% |
| miR-4265 | 64% | miR-6165 | 75% | miR-8089 | 72% |
| miR-2355-5p | 78% | miR-6501-5p | 56% | miR-128-2-5p | 73% |
| miR-4269 | 79% | miR-6503-5p | 80% | miR-1199-3p | 53% |
| miR-4271 | 78% | miR-6503-3p | 61% | miR-7974 | 65% |
| miR-4276 | 79% | miR-6505-5p | 100% | miR-7977 | 67% |
| miR-4274 | 77% | miR-6511a-5p | 95% | miR-7978 | 100% |
| miR-4281 | 78% | miR-6511a-3p | 76% | miR-4485-5p | 77% |
| miR-4279 | 81% | miR-6513-5p | 52% | miR-8485 | 73% |
| miR-4278 | 70% | miR-6514-3p | 55% | miR-9500 | 66% |
| miR-4280 | 59% | miR-6515-3p | 77% | miR-103a-1-5p | 57% |
| miR-4284 | 76% | miR-6715b-3p | 56% | miR-137-5p | 100% |
| miR-4286 | 80% | miR-6716-5p | 78% | miR-9898 | 77% |
| miR-4288 | 54% | miR-6716-3p | 68% | miR-9902 | 71% |
| miR-4289 | 55% | miR-6511b-5p | 90% | miR-10392-5p | 70% |
| miR-4290 | 82% | miR-6720-3p | 75% | miR-10392-3p | 83% |
| miR-2277-5p | 66% | miR-6721-5p | 81% | miR-10394-3p | 65% |
| miR-3200-5p | 77% | miR-6722-5p | 71% | miR-10396a-5p | 83% |
| miR-3610 | 80% | miR-6722-3p | 72% | miR-10396a-3p | 86% |
| miR-3612 | 87% | miR-6724-5p | 79% | miR-10398-5p | 75% |
| miR-3614-5p | 69% | miR-208a-5p | 72% | miR-10400-5p | 69% |
| miR-3615 | 67% | miR-210-5p | 74% | miR-10400-3p | 75% |
| miR-3616-3p | 71% | miR-128-1-5p | 77% | miR-10401-5p | 77% |
| miR-3619-5p | 56% | miR-133a-5p | 100% | miR-10401-3p | 85% |
| miR-3621 | 73% | miR-489-5p | 69% | miR-10396b-5p | 81% |
| miR-3622a-5p | 55% | miR-511-3p | 100% | miR-10396b-3p | 75% |
| miR-3622a-3p | 67% | miR-181d-3p | 77% | miR-10522-5p | 67% |
| miR-3646 | 56% | miR-585-5p | 55% | miR-10526-3p | 96% |
| miR-3648 | 81% | miR-598-5p | 53% | miR-10527-5p | 88% |
| miR-3651 | 51% | miR-627-3p | 100% | miR-11399 | 69% |
| miR-3652 | 53% | miR-1296-3p | 84% | miR-3059-5p | 79% |
| miR-3663-3p | 77% | miR-1301-5p | 58% | miR-3059-3p | 60% |
| miR-3665 | 76% | miR-874-5p | 69% | miR-3085-5p | 60% |
| miR-3667-5p | 53% | miR-216b-3p | 58% | miR-3085-3p | 71% |
| miR-3675-3p | 77% | miR-1287-3p | 61% | miR-12114 | 51% |
| miR-3677-3p | 57% | miR-1908-3p | 87% | miR-12115 | 69% |
| miR-3679-5p | 77% | miR-3151-3p | 81% | miR-12116 | 73% |
| miR-3679-3p | 78% | miR-3192-3p | 69% | miR-12118 | 82% |
| miR-3688-3p | 60% | miR-1343-5p | 84% | miR-12119 | 88% |
| miR-3714 | 77% | miR-5189-3p | 54% | miR-12120 | 72% |
| miR-3180 | 79% | miR-5699-5p | 73% | miR-12121 | 68% |
| miR-3907 | 80% | miR-6726-5p | 70% | miR-12128 | 72% |
| miR-3908 | 56% | miR-6726-3p | 81% | miR-12131 | 100% |
| miR-3917 | 65% | miR-6727-5p | 76% | miR-12135 | 100% |
| miR-3922-3p | 65% | miR-6727-3p | 76% | | |

| | | | | | |
|---|---|---|---|---|---|
| ^{∗} A represents Accuracy. ^{∗} "has-" in the miRNA names has been omitted. | | | | | |

### [Table 23-12]

**Table 23-12: miRNA to Predict Human Cervical Cancer of Stages I and II**

| miRNA Name | A | miRNA Name | A | miRNA Name | A |
|---|---|---|---|---|---|
| miR-25-3p | 63% | miR-378f | 54% | miR-6730-3p | 73% |
| miR-196a-5p | 61% | miR-4428 | 55% | miR-6731-3p | 71% |
| miR-197-3p | 85% | miR-4430 | 61% | miR-6732-5p | 70% |
| miR-198 | 74% | miR-4433a-3p | 77% | miR-6732-3p | 80% |
| miR-30d-5p | 74% | miR-4439 | 64% | miR-6734-5p | 76% |
| miR-139-5p | 100% | miR-4440 | 55% | miR-6735-5p | 69% |
| miR-210-3p | 58% | miR-4441 | 76% | miR-6735-3p | 70% |
| miR-214-3p | 53% | miR-4444 | 60% | miR-6736-5p | 66% |
| miR-124-3p | 67% | miR-4446-3p | 56% | miR-6736-3p | 72% |
| miR-125b-5p | 54% | miR-4447 | 71% | miR-6737-5p | 68% |
| miR-134-5p | 68% | miR-4448 | 61% | miR-6738-5p | 70% |
| miR-149-5p | 80% | miR-4449 | 65% | miR-6738-3p | 61% |
| miR-188-5p | 69% | miR-548ah-5p | 100% | miR-6740-5p | 53% |
| miR-320a-3p | 72% | miR-4455 | 60% | miR-6740-3p | 85% |
| miR-106b-5p | 86% | miR-3135b | 66% | miR-6741-5p | 68% |
| miR-296-5p | 70% | miR-4462 | 69% | miR-6741-3p | 83% |
| miR-365a-3p, miR-365b-3p | 70% | miR-4463 | 70% | miR-6742-5p | 77% |
| miR-302c-5p | 58% | miR-4466 | 65% | miR-6742-3p | 73% |
| miR-370-3p | 68% | miR-4467 | 64% | miR-6743-5p | 67% |
| miR-373-5p | 72% | miR-4470 | 75% | miR-6743-3p | 80% |
| miR-373-3p | 55% | miR-4472 | 68% | miR-6744-5p | 65% |
| miR-375-3p | 67% | miR-4473 | 58% | miR-6744-3p | 67% |
| miR-379-5p | 67% | miR-4475 | 56% | miR-6745 | 62% |
| miR-380-3p | 77% | miR-4476 | 55% | miR-6746-5p | 78% |
| miR-383-5p | 75% | miR-4478 | 72% | miR-6746-3p | 68% |
| miR-328-3p | 83% | miR-3689d | 68% | miR-6747-3p | 70% |
| miR-324-3p | 62% | miR-3155b | 77% | miR-6748-5p | 72% |
| miR-133b | 70% | miR-4480 | 100% | miR-6748-3p | 80% |
| miR-346 | 75% | miR-4481 | 65% | miR-6749-5p | 78% |
| miR-449a | 70% | miR-4483 | 68% | miR-6749-3p | 77% |
| miR-191-3p | 74% | miR-4484 | 68% | miR-6750-3p | 78% |
| miR-200a-5p | 55% | miR-4486 | 67% | miR-6751-5p | 72% |
| miR-483-3p | 85% | miR-4489 | 59% | miR-6751-3p | 82% |
| miR-484 | 70% | miR-548a1 | 100% | miR-6752-5p | 71% |
| miR-485-3p | 73% | miR-4492 | 72% | miR-6752-3p | 72% |
| miR-486-5p | 70% | miR-4495 | 100% | miR-6753-5p | 67% |
| miR-491-5p | 72% | miR-4496 | 79% | miR-6753-3p | 72% |
| miR-492 | 51% | miR-4497 | 63% | miR-6754-5p | 65% |
| miR-493-5p | 63% | miR-4498 | 60% | miR-6754-3p | 70% |
| miR-498-5p | 81% | miR-4499 | 60% | miR-6756-5p | 67% |
| miR-519b-3p | 55% | miR-4505 | 69% | miR-6756-3p | 74% |
| miR-518c-5p | 74% | miR-2392 | 69% | miR-6757-5p | 63% |
| miR-519d-3p | 51% | miR-4507 | 68% | miR-6757-3p | 71% |
| miR-516b-5p | 75% | miR-4508 | 62% | miR-6758-5p | 72% |
| miR-503-5p | 75% | miR-4512 | 81% | miR-6758-3p | 52% |
| miR-513a-5p | 60% | miR-4513 | 66% | miR-6759-5p | 67% |
| miR-539-5p | 68% | miR-4516 | 62% | miR-6759-3p | 74% |
| miR-551a | 51% | miR-4519 | 83% | miR-6760-5p | 59% |
| miR-554 | 52% | miR-4521 | 73% | miR-6760-3p | 75% |
| miR-92b-3p | 60% | miR-1269b | 68% | miR-6761-3p | 78% |
| miR-557 | 71% | miR-4522 | 67% | miR-6762-5p | 58% |
| miR-564 | 59% | miR-4523 | 66% | miR-6762-3p | 61% |
| miR-571 | 80% | miR-4524a-3p | 67% | miR-6763-5p | 68% |
| miR-574-3p | 75% | miR-4525 | 66% | miR-6763-3p | 75% |
| miR-575 | 61% | miR-4526 | 57% | miR-6764-3p | 55% |
| miR-550a-3p | 67% | miR-4530 | 70% | miR-6765-5p | 67% |
| miR-611 | 68% | miR-4533 | 65% | miR-6766-5p | 73% |
| miR-612 | 61% | miR-4534 | 70% | miR-6766-3p | 74% |
| miR-613 | 71% | miR-1587 | 59% | miR-6768-5p | 73% |
| miR-615-3p | 65% | miR-4538 | 51% | miR-6769a-5p | 70% |
| miR-632 | 67% | miR-4539 | 66% | miR-6769a-3p | 77% |
| miR-634 | 71% | miR-3120-5p | 61% | miR-6771-5p | 63% |
| miR-636 | 71% | miR-3127-3p | 75% | miR-6772-5p | 63% |
| miR-637 | 69% | miR-3156-3p | 75% | miR-6772-3p | 63% |
| miR-638 | 65% | miR-3158-5p | 78% | miR-6773-5p | 71% |
| miR-652-3p | 100% | miR-3162-3p | 74% | miR-6773-3p | 62% |
| miR-661 | 61% | miR-3173-5p | 73% | miR-6774-5p | 81% |
| miR-663a | 71% | miR-3187-5p | 59% | miR-6774-3p | 55% |
| miR-654-5p | 53% | miR-3619-3p | 76% | miR-6775-5p | 76% |
| miR-657 | 63% | miR-3691-3p | 54% | miR-6776-5p | 66% |
| miR-658 | 78% | miR-3922-5p | 54% | miR-6776-3p | 76% |
| miR-659-3p | 69% | miR-3944-5p | 54% | miR-6777-5p | 60% |
| miR-542-5p | 86% | miR-3972 | 76% | miR-6777-3p | 73% |
| miR-671-5p | 76% | miR-3977 | 67% | miR-6778-5p | 70% |
| miR-668-3p | 74% | miR-3978 | 94% | miR-6778-3p | 71% |
| miR-766-3p | 74% | miR-4634 | 67% | miR-6779-5p | 73% |
| miR-765 | 76% | miR-4637 | 100% | miR-6779-3p | 73% |
| miR-675-5p | 68% | miR-4638-5p | 58% | miR-6780a-5p | 74% |
| miR-297 | 58% | miR-4639-3p | 56% | miR-6780a-3p | 64% |
| miR-22-5p | 100% | miR-4640-5p | 68% | miR-6781-5p | 65% |
| miR-24-2-5p | 52% | miR-4640-3p | 69% | miR-6782-5p | 76% |
| miR-25-5p | 51% | miR-4645-5p | 55% | miR-6782-3p | 73% |
| miR-92a-2-5p | 66% | miR-4646-5p | 68% | miR-6783-5p | 51% |
| miR-29b-1-5p | 77% | miR-4646-3p | 78% | miR-6783-3p | 66% |
| miR-139-3p | 58% | miR-4648 | 61% | miR-6784-5p | 62% |
| miR-181a-2-3p | 59% | miR-4649-5p | 65% | miR-6784-3p | 74% |
| miR-181c-3p | 75% | miR-4651 | 68% | miR-6785-3p | 75% |
| miR-183-3p | 69% | miR-4655-5p | 72% | miR-6786-5p | 65% |
| miR-187-5p | 59% | miR-4656 | 69% | miR-6786-3p | 69% |
| miR-23b-5p | 54% | miR-4661-5p | 72% | miR-6787-5p | 70% |
| miR-30b-3p | 58% | miR-4661-3p | 51% | miR-6787-3p | 73% |
| miR-124-5p | 63% | miR-4664-5p | 66% | miR-6788-5p | 55% |
| miR-125b-1-3p | 100% | miR-4664-3p | 76% | miR-6789-5p | 66% |
| miR-135a-3p | 55% | miR-4665-5p | 76% | miR-6789-3p | 75% |
| miR-140-3p | 59% | miR-4665-3p | 71% | miR-6790-5p | 68% |
| miR-143-5p | 63% | miR-4667-5p | 67% | miR-6791-5p | 65% |
| miR-145-3p | 75% | miR-4667-3p | 70% | miR-6792-5p | 66% |
| miR-125a-3p | 59% | miR-4668-5p | 100% | miR-6792-3p | 80% |
| miR-127-5p | 72% | miR-4669 | 58% | miR-6793-3p | 75% |
| miR-149-3p | 70% | miR-4674 | 65% | miR-6794-5p | 77% |
| miR-150-3p | 78% | miR-4675 | 68% | miR-6794-3p | 83% |
| miR-185-3p | 54% | miR-4682 | 51% | miR-6795-5p | 59% |
| miR-186-3p | 63% | miR-4685-5p | 72% | miR-6795-3p | 72% |
| miR-194-3p | 51% | miR-4685-3p | 78% | miR-6796-5p | 59% |
| miR-30c-1-3p | 78% | miR-4687-5p | 75% | miR-6796-3p | 72% |
| miR-296-3p | 68% | miR-4687-3p | 67% | miR-6797-5p | 82% |
| miR-361-3p | 72% | miR-1343-3p | 79% | miR-6797-3p | 70% |
| miR-371a-5p | 69% | miR-4688 | 65% | miR-6798-5p | 71% |
| miR-339-3p | 53% | miR-4689 | 66% | miR-6798-3p | 73% |
| miR-423-5p | 70% | miR-4690-5p | 67% | miR-6799-5p | 75% |
| miR-18b-3p | 69% | miR-4691-5p | 69% | miR-6799-3p | 75% |
| miR-20b-3p | 53% | miR-4691-3p | 92% | miR-6800-5p | 67% |
| miR-431-3p | 63% | miR-4695-5p | 64% | miR-6800-3p | 71% |
| miR-483-5p | 78% | miR-4695-3p | 74% | miR-6801-3p | 74% |
| miR-486-3p | 58% | miR-4697-5p | 70% | miR-6802-5p | 70% |
| miR-490-5p | 100% | miR-4697-3p | 74% | miR-6802-3p | 70% |
| miR-505-5p | 54% | miR-4698 | 79% | miR-6803-5p | 63% |
| miR-509-5p | 58% | miR-4700-3p | 73% | miR-6803-3p | 82% |
| miR-92b-5p | 67% | miR-4706 | 78% | miR-6804-3p | 77% |
| miR-574-5p | 59% | miR-4707-5p | 69% | miR-6805-5p | 70% |
| miR-550a-5p | 71% | miR-4707-3p | 73% | miR-6805-3p | 72% |
| miR-615-5p | 65% | miR-4708-5p | 60% | miR-6806-5p | 68% |
| miR-624-3p | 57% | miR-4708-3p | 61% | miR-6807-5p | 76% |
| miR-625-3p | 74% | miR-4709-3p | 70% | miR-6808-5p | 71% |
| miR-629-5p | 64% | miR-4710 | 65% | miR-6808-3p | 75% |
| miR-671-3p | 59% | miR-4712-3p | 56% | miR-6809-5p | 71% |
| miR-298 | 73% | miR-4713-5p | 76% | miR-6809-3p | 83% |
| miR-874-3p | 71% | miR-4713-3p | 52% | miR-6810-5p | 67% |
| miR-891b | 60% | miR-4714-5p | 73% | miR-6810-3p | 73% |
| miR-541-5p | 52% | miR-4715-5p | 76% | miR-6812-5p | 80% |
| miR-744-5p | 66% | miR-4716-5p | 73% | miR-6813-5p | 65% |
| miR-744-3p | 54% | miR-4716-3p | 77% | miR-6813-3p | 72% |
| miR-885-5p | 71% | miR-4717-3p | 64% | miR-6815-5p | 53% |
| miR-885-3p | 58% | miR-4721 | 64% | miR-6815-3p | 53% |
| miR-877-5p | 58% | miR-4722-5p | 66% | miR-6816-5p | 73% |
| miR-877-3p | 82% | miR-4722-3p | 67% | miR-6816-3p | 68% |
| miR-887-3p | 58% | miR-4723-5p | 67% | miR-6817-3p | 72% |
| miR-665 | 69% | miR-4723-3p | 77% | miR-6819-5p | 66% |
| miR-760 | 68% | miR-451b | 70% | miR-6819-3p | 72% |
| miR-920 | 69% | miR-4725-5p | 80% | miR-6820-5p | 57% |
| miR-936 | 72% | miR-4725-3p | 78% | miR-6820-3p | 75% |
| miR-937-3p | 63% | miR-4726-5p | 67% | miR-6821-5p | 71% |
| miR-939-5p | 66% | miR-4726-3p | 63% | miR-6821-3p | 76% |
| miR-940 | 72% | miR-4728-5p | 75% | miR-6822-5p | 64% |
| miR-943 | 62% | miR-4728-3p | 72% | miR-6823-3p | 79% |
| miR-1224-3p | 75% | miR-4730 | 66% | miR-6824-5p | 71% |
| miR-1225-5p | 75% | miR-4731-5p | 69% | miR-6824-3p | 75% |
| miR-1225-3p | 71% | miR-4731-3p | 72% | miR-6825-5p | 76% |
| miR-1226-3p | 71% | miR-4732-5p | 68% | miR-6825-3p | 75% |
| miR-1228-3p | 68% | miR-4732-3p | 71% | miR-6826-5p | 70% |
| miR-1229-3p | 80% | miR-4733-5p | 51% | miR-6826-3p | 70% |
| miR-1231 | 62% | miR-4736 | 62% | miR-6827-5p | 61% |
| miR-1233-3p | 74% | miR-4738-5p | 86% | miR-6828-5p | 64% |
| miR-1234-3p | 74% | miR-4738-3p | 69% | miR-6829-5p | 81% |
| miR-1236-3p | 74% | miR-4739 | 65% | miR-6829-3p | 73% |
| miR-1237-3p | 70% | miR-4740-3p | 55% | miR-6830-5p | 80% |
| miR-1238-3p | 68% | miR-4741 | 68% | miR-6830-3p | 78% |
| miR-1264 | 55% | miR-4743-5p | 56% | miR-6831-5p | 70% |
| miR-320b | 71% | miR-4745-5p | 67% | miR-6832-5p | 67% |
| miR-320c | 63% | miR-4746-3p | 63% | miR-6832-3p | 63% |
| miR-1323 | 67% | miR-4747-5p | 80% | miR-6833-5p | 76% |
| miR-1298-5p | 55% | miR-4748 | 75% | miR-6833-3p | 80% |
| miR-1180-3p | 100% | miR-4749-5p | 70% | miR-6835-5p | 63% |
| miR-1181 | 62% | miR-4749-3p | 72% | miR-6780b-5p | 76% |
| miR-1182 | 76% | miR-4750-5p | 65% | miR-6780b-3p | 81% |
| miR-1184 | 69% | miR-4751 | 74% | miR-6836-3p | 69% |
| miR-1200 | 51% | miR-4753-5p | 63% | miR-6837-5p | 54% |
| miR-1202 | 74% | miR-371b-5p | 61% | miR-6838-5p | 53% |
| miR-1203 | 71% | miR-371b-3p | 72% | miR-6839-5p | 53% |
| miR-663b | 68% | miR-4755-3p | 53% | miR-6839-3p | 56% |
| miR-1204 | 63% | miR-4756-5p | 72% | miR-6840-5p | 74% |
| miR-1207-5p | 75% | miR-4758-5p | 70% | miR-6840-3p | 71% |
| miR-1207-3p | 59% | miR-4758-3p | 72% | miR-6842-5p | 71% |
| miR-1208 | 74% | miR-4761-5p | 67% | miR-6845-5p | 69% |
| miR-1299 | 100% | miR-4762-3p | 80% | miR-6845-3p | 81% |
| miR-1249-3p | 71% | miR-4763-5p | 80% | miR-6846-5p | 70% |
| miR-1253 | 67% | miR-4763-3p | 67% | miR-6846-3p | 69% |
| miR-1255a | 100% | miR-4769-3p | 79% | miR-6847-3p | 66% |
| miR-1260a | 76% | miR-4778-5p | 74% | miR-6848-5p | 65% |
| miR-1266-5p | 64% | miR-4779 | 57% | miR-6848-3p | 72% |
| miR-1267 | 76% | miR-4780 | 62% | miR-6849-5p | 59% |
| miR-1268a | 65% | miR-4436b-5p | 79% | miR-6849-3p | 82% |
| miR-1272 | 63% | miR-4781-5p | 75% | miR-6850-5p | 63% |
| miR-1275 | 68% | miR-4783-3p | 73% | miR-6851-5p | 64% |
| miR-1281 | 83% | miR-2467-5p | 59% | miR-6851-3p | 69% |
| miR-1292-5p | 82% | miR-2467-3p | 54% | miR-6852-3p | 64% |
| miR-1255b-5p | 100% | miR-4787-3p | 75% | miR-6855-5p | 51% |
| miR-1307-3p | 58% | miR-4788 | 71% | miR-6855-3p | 72% |
| miR-1825 | 85% | miR-4793-3p | 70% | miR-6856-5p | 70% |
| miR-675-3p | 72% | miR-4800-5p | 71% | miR-6857-5p | 66% |
| miR-1469 | 69% | miR-4800-3p | 53% | miR-6857-3p | 77% |
| miR-1538 | 63% | miR-4802-3p | 67% | miR-6858-5p | 68% |
| miR-1539 | 72% | miR-4803 | 71% | miR-6858-3p | 75% |
| miR-1908-5p | 72% | miR-4804-5p | 83% | miR-6859-3p | 72% |
| miR-1909-5p | 70% | miR-642a-3p | 79% | miR-6769b-5p | 77% |
| miR-1909-3p | 70% | miR-4433a-5p | 71% | miR-6769b-3p | 82% |
| miR-1910-5p | 79% | miR-4482-3p | 59% | miR-6860 | 57% |
| miR-1911-5p | 100% | miR-4999-5p | 59% | miR-6861-5p | 69% |
| miR-1912-3p | 72% | miR-5001-5p | 70% | miR-6861-3p | 78% |
| miR-1913 | 72% | miR-5001-3p | 56% | miR-6862-3p | 83% |
| miR-1914-3p | 84% | miR-5002-3p | 60% | miR-6865-5p | 74% |
| miR-1915-3p | 67% | miR-5003-3p | 67% | miR-6865-3p | 79% |
| miR-365a-5p | 73% | miR-548ao-3p | 80% | miR-6867-5p | 77% |
| miR-196b-3p | 51% | miR-5006-5p | 68% | miR-6867-3p | 77% |
| miR-449b-3p | 76% | miR-5006-3p | 56% | miR-6869-5p | 62% |
| miR-2113 | 55% | miR-5008-5p | 54% | miR-6870-5p | 77% |
| miR-1972 | 64% | miR-5010-5p | 69% | miR-6870-3p | 71% |
| miR-1976 | 83% | miR-5088-5p | 67% | miR-6872-5p | 52% |
| miR-2052 | 100% | miR-5090 | 61% | miR-6873-5p | 58% |
| miR-2110 | 66% | miR-5094 | 52% | miR-6873-3p | 66% |
| miR-762 | 62% | miR-5187-3p | 52% | miR-6875-5p | 71% |
| miR-670-5p | 67% | miR-5193 | 83% | miR-6875-3p | 70% |
| miR-764 | 66% | miR-5195-5p | 74% | miR-6876-3p | 63% |
| miR-2116-3p | 73% | miR-5195-3p | 72% | miR-6877-5p | 69% |
| miR-548q | 64% | miR-5196-5p | 83% | miR-6877-3p | 80% |
| miR-2276-3p | 61% | miR-5196-3p | 80% | miR-6878-5p | 59% |
| miR-711 | 78% | miR-5100 | 56% | miR-6878-3p | 75% |
| miR-718 | 67% | miR-5572 | 66% | miR-6879-5p | 69% |
| miR-2681-3p | 64% | miR-548as-5p | 100% | miR-6879-3p | 74% |
| miR-449c-3p | 61% | miR-548as-3p | 100% | miR-6880-5p | 77% |
| miR-3120-3p | 56% | miR-5579-5p | 100% | miR-6880-3p | 72% |
| miR-3122 | 59% | miR-5579-3p | 100% | miR-6881-5p | 70% |
| miR-3130-5p | 70% | miR-5584-5p | 74% | miR-6881-3p | 67% |
| miR-3131 | 68% | miR-5584-3p | 56% | miR-6882-3p | 74% |
| miR-3132 | 75% | miR-5585-5p | 75% | miR-6883-5p | 68% |
| miR-3138 | 72% | miR-5586-3p | 55% | miR-6883-3p | 76% |
| miR-3141 | 73% | miR-5589-5p | 60% | miR-6884-3p | 79% |
| miR-3144-5p | 78% | miR-5684 | 58% | miR-6885-3p | 73% |
| miR-3147 | 71% | miR-5685 | 55% | miR-6886-5p | 56% |
| miR-548v | 100% | miR-5690 | 55% | miR-6886-3p | 86% |
| miR-3151-5p | 69% | miR-5693 | 70% | miR-6887-5p | 54% |
| miR-3153 | 81% | miR-5695 | 75% | miR-6887-3p | 70% |
| miR-3154 | 69% | miR-5698 | 75% | miR-6889-5p | 66% |
| miR-3155a | 63% | miR-5705 | 61% | miR-6889-3p | 73% |
| miR-3156-5p | 74% | miR-197-5p | 69% | miR-6890-5p | 68% |
| miR-3157-5p | 61% | miR-204-3p | 65% | miR-6890-3p | 74% |
| miR-3158-3p | 52% | miR-211-3p | 65% | miR-6891-5p | 77% |
| miR-3162-5p | 76% | miR-345-3p | 66% | miR-6891-3p | 73% |
| miR-3163 | 67% | miR-514a-5p | 100% | miR-6892-5p | 56% |
| miR-3165 | 63% | miR-584-3p | 51% | miR-6892-3p | 74% |
| miR-1260b | 77% | miR-652-5p | 80% | miR-6893-5p | 70% |
| miR-3173-3p | 55% | miR-1185-2-3p | 78% | miR-6893-3p | 72% |
| miR-1193 | 56% | miR-873-3p | 56% | miR-6894-5p | 70% |
| miR-3175 | 70% | miR-1304-3p | 72% | miR-6894-3p | 83% |
| miR-3177-3p | 59% | miR-1247-3p | 72% | miR-6895-3p | 67% |
| miR-3178 | 61% | miR-1306-5p | 73% | miR-7106-5p | 76% |
| miR-3180-3p | 68% | miR-3184-3p | 77% | miR-7106-3p | 70% |
| miR-3182 | 60% | miR-3191-5p | 71% | miR-7107-5p | 74% |
| miR-3184-5p | 68% | miR-642b-5p | 64% | miR-7108-5p | 69% |
| miR-3185 | 67% | miR-365b-5p | 66% | miR-7108-3p | 76% |
| miR-3186-3p | 60% | miR-1185-1-3p | 80% | miR-7109-5p | 78% |
| miR-3188 | 66% | miR-503-3p | 74% | miR-7109-3p | 76% |
| miR-3191-3p | 68% | miR-376a-2-5p | 100% | miR-7110-5p | 71% |
| miR-3194-5p | 73% | miR-937-5p | 73% | miR-7110-3p | 84% |
| miR-3195 | 69% | miR-939-3p | 72% | miR-7111-5p | 73% |
| miR-3196 | 62% | miR-1227-5p | 73% | miR-7111-3p | 80% |
| miR-3197 | 68% | miR-1229-5p | 72% | miR-7112-5p | 63% |
| miR-3198 | 69% | miR-1233-5p | 68% | miR-7113-3p | 77% |
| miR-514b-5p | 71% | miR-1237-5p | 65% | miR-7114-5p | 66% |
| miR-3202 | 80% | miR-1238-5p | 74% | miR-7114-3p | 74% |
| miR-3065-3p | 51% | miR-1292-3p | 74% | miR-7150 | 73% |
| miR-4297 | 67% | miR-3620-5p | 63% | miR-7151-5p | 51% |
| miR-4293 | 73% | miR-4632-5p | 71% | miR-7152-5p | 66% |
| miR-4294 | 80% | miR-4743-3p | 52% | miR-7152-3p | 56% |
| miR-4299 | 57% | miR-4750-3p | 75% | miR-7155-5p | 65% |
| miR-4298 | 72% | miR-5739 | 72% | miR-7155-3p | 67% |
| miR-4305 | 67% | miR-5787 | 65% | miR-7157-3p | 64% |
| miR-4313 | 73% | miR-6068 | 56% | miR-7158-3p | 68% |
| miR-4316 | 75% | miR-6069 | 72% | miR-7159-5p | 56% |
| miR-4322 | 62% | miR-6071 | 70% | miR-7160-5p | 59% |
| miR-4321 | 51% | miR-6075 | 54% | miR-7161-5p | 55% |
| miR-4323 | 74% | miR-6076 | 68% | miR-7515 | 59% |
| miR-4324 | 56% | miR-6077 | 71% | miR-7702 | 66% |
| miR-4257 | 75% | miR-6078 | 53% | miR-7704 | 57% |
| miR-4258 | 68% | miR-6083 | 51% | miR-7843-5p | 71% |
| miR-4259 | 66% | miR-6085 | 69% | miR-7843-3p | 58% |
| miR-4260 | 76% | miR-6086 | 77% | miR-4433b-5p | 72% |
| miR-4253 | 68% | miR-6090 | 62% | miR-4433b-3p | 78% |
| miR-4251 | 65% | miR-6124 | 81% | miR-1273h-5p | 67% |
| miR-4254 | 51% | miR-6125 | 65% | miR-1273h-3p | 61% |
| miR-4325 | 56% | miR-6126 | 60% | miR-7845-5p | 68% |
| miR-4326 | 83% | miR-6127 | 66% | miR-7846-3p | 65% |
| miR-4327 | 68% | miR-378j | 52% | miR-7847-3p | 67% |
| miR-4265 | 67% | miR-6131 | 68% | miR-7851-3p | 56% |
| miR-4266 | 59% | miR-6132 | 68% | miR-7852-3p | 100% |
| miR-2355-5p | 73% | miR-6133 | 77% | miR-7855-5p | 73% |
| miR-4268 | 71% | miR-6165 | 72% | miR-8052 | 61% |
| miR-4269 | 69% | miR-6500-5p | 51% | miR-8059 | 75% |
| miR-4270 | 65% | miR-6501-5p | 58% | miR-8060 | 75% |
| miR-4271 | 78% | miR-6501-3p | 53% | miR-8063 | 58% |
| miR-4276 | 61% | miR-6503-5p | 72% | miR-8064 | 65% |
| miR-4274 | 72% | miR-6505-5p | 100% | miR-8065 | 100% |
| miR-4281 | 69% | miR-6505-3p | 52% | miR-8069 | 60% |
| miR-4279 | 83% | miR-6507-5p | 79% | miR-8071 | 66% |
| miR-4278 | 61% | miR-6509-3p | 64% | miR-8072 | 68% |
| miR-4285 | 63% | miR-6510-5p | 73% | miR-8073 | 68% |
| miR-4284 | 72% | miR-6511a-5p | 75% | miR-8077 | 62% |
| miR-4286 | 78% | miR-6511a-3p | 81% | miR-8078 | 59% |
| miR-4287 | 65% | miR-6512-5p | 100% | miR-8079 | 57% |
| miR-4288 | 66% | miR-6512-3p | 55% | miR-8085 | 66% |
| miR-4289 | 53% | miR-6513-5p | 59% | miR-8087 | 85% |
| miR-4290 | 82% | miR-6514-3p | 65% | miR-8089 | 72% |
| miR-4329 | 72% | miR-6515-3p | 72% | miR-128-2-5p | 65% |
| miR-2277-5p | 51% | miR-6715b-3p | 67% | miR-7974 | 53% |
| miR-3200-5p | 54% | miR-6716-5p | 76% | miR-7977 | 66% |
| miR-3605-3p | 72% | miR-6716-3p | 72% | miR-301b-5p | 83% |
| miR-3610 | 74% | miR-6511b-5p | 72% | miR-1249-5p | 72% |
| miR-3612 | 77% | miR-6511b-3p | 73% | miR-4485-5p | 74% |
| miR-3614-5p | 71% | miR-6718-5p | 52% | miR-8485 | 76% |
| miR-3615 | 56% | miR-6719-3p | 71% | miR-9500 | 54% |
| miR-3619-5p | 59% | miR-6720-3p | 86% | miR-103a-1-5p | 61% |
| miR-3620-3p | 74% | miR-6721-5p | 67% | miR-196a-1-3p | 64% |
| miR-3621 | 59% | miR-6722-5p | 71% | miR-137-5p | 100% |
| miR-3622a-5p | 53% | miR-6722-3p | 66% | miR-190b-3p | 100% |
| miR-3622a-3p | 73% | miR-6724-5p | 70% | miR-9718 | 58% |
| miR-3622b-5p | 67% | miR-210-5p | 72% | miR-9898 | 72% |
| miR-3646 | 67% | miR-128-1-5p | 66% | miR-9902 | 51% |
| miR-3648 | 74% | miR-152-5p | 75% | miR-10226 | 69% |
| miR-3649 | 73% | miR-328-5p | 65% | miR-10392-5p | 66% |
| miR-3652 | 56% | miR-489-5p | 58% | miR-10392-3p | 62% |
| miR-3663-5p | 53% | miR-511-3p | 100% | miR-10394-3p | 69% |
| miR-3663-3p | 67% | miR-181d-3p | 76% | miR-10396a-5p | 70% |
| miR-3665 | 55% | miR-504-3p | 67% | miR-10396a-3p | 72% |
| miR-3667-5p | 52% | miR-487b-5p | 61% | miR-10398-5p | 77% |
| miR-3667-3p | 69% | miR-585-5p | 54% | miR-10398-3p | 69% |
| miR-3675-3p | 74% | miR-598-5p | 68% | miR-10399-3p | 83% |
| miR-3677-3p | 67% | miR-619-5p | 59% | miR-10400-3p | 64% |
| miR-3679-5p | 83% | miR-627-3p | 100% | miR-10401-5p | 71% |
| miR-3679-3p | 72% | miR-1296-3p | 69% | miR-10396b-5p | 68% |
| miR-3681-5p | 100% | miR-874-5p | 69% | miR-10522-5p | 78% |
| miR-3682-3p | 57% | miR-887-5p | 62% | miR-10526-3p | 74% |
| miR-3692-3p | 59% | miR-216b-3p | 68% | miR-11181-5p | 70% |
| miR-3714 | 73% | miR-208b-5p | 100% | miR-11181-3p | 72% |
| miR-3180 | 68% | miR-1287-3p | 51% | miR-11399 | 57% |
| miR-3907 | 86% | miR-1250-3p | 64% | miR-11400 | 58% |
| miR-3908 | 67% | miR-1266-3p | 63% | miR-3059-5p | 77% |
| miR-3911 | 70% | miR-3151-3p | 81% | miR-3059-3p | 62% |
| miR-3917 | 63% | miR-3192-3p | 74% | miR-3085-5p | 68% |
| miR-3922-3p | 63% | miR-1343-5p | 68% | miR-3085-3p | 71% |
| miR-3926 | 63% | miR-5088-3p | 65% | miR-12113 | 53% |
| miR-3928-3p | 73% | miR-5189-3p | 62% | miR-12114 | 59% |
| miR-3934-5p | 53% | miR-5699-5p | 74% | miR-12115 | 68% |
| miR-3935 | 56% | miR-6726-5p | 63% | miR-12116 | 70% |
| miR-3937 | 68% | miR-6726-3p | 77% | miR-12118 | 67% |
| miR-3940-3p | 78% | miR-6727-5p | 56% | miR-12119 | 78% |
| miR-3943 | 73% | miR-6727-3p | 76% | miR-12120 | 72% |
| miR-3944-3p | 69% | miR-6728-5p | 55% | miR-12121 | 66% |
| miR-3945 | 68% | miR-6728-3p | 78% | miR-12128 | 65% |
| miR-642b-3p | 75% | miR-6729-5p | 68% | miR-12131 | 100% |
| miR-550b-3p | 69% | miR-6729-3p | 70% | miR-12136 | 63% |
| miR-1268b | 71% | miR-6730-5p | 70% | | |

| | | | | | |
|---|---|---|---|---|---|
| ^{∗} A represents Accuracy. ^{∗} "has-" in the miRNA names has been omitted. | | | | | |

### [Table 23-13]

**Table 23-13: miRNA to Predict Human Rectal Cancer of Stages I and II**

| miRNA Name | A | miRNA Name | A | miRNA Name | A |
|---|---|---|---|---|---|
| miR-15a-5p | 62% | miR-4447 | 78% | miR-6726-5p | 74% |
| miR-30a-5p | 60% | miR-4448 | 77% | miR-6727-5p | 74% |
| miR-192-5p | 63% | miR-4449 | 76% | miR-6727-3p | 80% |
| miR-196a-5p | 59% | miR-4455 | 64% | miR-6729-5p | 84% |
| miR-139-5p | 100% | miR-4456 | 57% | miR-6732-5p | 79% |
| miR-187-3p | 66% | miR-4458 | 62% | miR-6735-5p | 74% |
| miR-210-3p | 58% | miR-4460 | 76% | miR-6736-5p | 73% |
| miR-122-5p | 52% | miR-378h | 64% | miR-6736-3p | 73% |
| miR-124-3p | 67% | miR-3135b | 78% | miR-6737-5p | 76% |
| miR-138-5p | 53% | miR-4462 | 77% | miR-6738-5p | 78% |
| miR-200c-3p | 75% | miR-4463 | 78% | miR-6738-3p | 66% |
| miR-106b-5p | 86% | miR-4466 | 79% | miR-6740-5p | 62% |
| miR-370-3p | 69% | miR-4467 | 84% | miR-6741-5p | 72% |
| miR-373-5p | 73% | miR-4470 | 78% | miR-6743-5p | 78% |
| miR-373-3p | 66% | miR-4474-3p | 51% | miR-6743-3p | 78% |
| miR-328-3p | 78% | miR-4475 | 53% | miR-6747-5p | 74% |
| miR-342-3p | 65% | miR-4478 | 78% | miR-6747-3p | 72% |
| miR-325 | 55% | miR-4481 | 78% | miR-6748-5p | 57% |
| miR-346 | 73% | miR-4483 | 74% | miR-6749-5p | 75% |
| miR-422a | 51% | miR-4484 | 71% | miR-6750-3p | 75% |
| miR-449a | 58% | miR-4486 | 74% | miR-6751-5p | 77% |
| miR-450a-5p | 52% | miR-4487 | 67% | miR-6752-5p | 82% |
| miR-452-5p | 100% | miR-4488 | 73% | miR-6753-5p | 77% |
| miR-202-3p | 53% | miR-4489 | 74% | miR-6754-5p | 74% |
| miR-492 | 54% | miR-4492 | 81% | miR-6754-3p | 74% |
| miR-498-5p | 81% | miR-4496 | 70% | miR-6756-5p | 81% |
| miR-520c-3p | 71% | miR-4497 | 77% | miR-6759-5p | 76% |
| miR-519a-3p | 55% | miR-4498 | 79% | miR-6761-3p | 72% |
| miR-503-5p | 67% | miR-4505 | 80% | miR-6762-5p | 73% |
| miR-552-3p | 64% | miR-4506 | 59% | miR-6762-3p | 69% |
| miR-557 | 77% | miR-2392 | 72% | miR-6763-5p | 75% |
| miR-564 | 71% | miR-4507 | 81% | miR-6765-5p | 75% |
| miR-573 | 100% | miR-4508 | 82% | miR-6766-5p | 74% |
| miR-574-3p | 78% | miR-4513 | 79% | miR-6767-5p | 58% |
| miR-575 | 67% | miR-4516 | 78% | miR-6768-5p | 81% |
| miR-579-3p | 72% | miR-4519 | 89% | miR-6770-3p | 54% |
| miR-585-3p | 59% | miR-4520-3p | 59% | miR-6771-5p | 68% |
| miR-588 | 60% | miR-4521 | 72% | miR-6772-5p | 73% |
| miR-595 | 71% | miR-4524a-3p | 67% | miR-6772-3p | 58% |
| miR-608 | 62% | miR-4526 | 71% | miR-6773-3p | 58% |
| miR-612 | 78% | miR-4530 | 77% | miR-6774-5p | 77% |
| miR-615-3p | 67% | miR-4533 | 69% | miR-6775-5p | 71% |
| miR-617 | 53% | miR-4535 | 76% | miR-6776-5p | 67% |
| miR-637 | 84% | miR-1587 | 68% | miR-6777-5p | 62% |
| miR-638 | 78% | miR-4538 | 67% | miR-6777-3p | 65% |
| miR-639 | 86% | miR-4539 | 83% | miR-6778-5p | 78% |
| miR-663a | 82% | miR-4540 | 53% | miR-6778-3p | 71% |
| miR-654-5p | 57% | miR-3120-5p | 53% | miR-6779-5p | 73% |
| miR-658 | 71% | miR-3173-5p | 70% | miR-6779-3p | 72% |
| miR-542-5p | 86% | miR-3187-5p | 80% | miR-6781-5p | 78% |
| miR-668-3p | 68% | miR-3922-5p | 54% | miR-6782-5p | 75% |
| miR-766-3p | 74% | miR-3940-5p | 72% | miR-6783-3p | 69% |
| miR-675-5p | 72% | miR-3960 | 65% | miR-6784-5p | 78% |
| miR-297 | 57% | miR-4634 | 79% | miR-6785-5p | 70% |
| let-7d-3p | 66% | miR-4638-5p | 72% | miR-6786-5p | 83% |
| miR-22-5p | 100% | miR-4640-5p | 76% | miR-6787-5p | 84% |
| miR-24-2-5p | 52% | miR-4648 | 78% | miR-6787-3p | 70% |
| miR-29a-5p | 100% | miR-4649-5p | 69% | miR-6789-5p | 82% |
| miR-92a-1-5p | 100% | miR-4650-3p | 60% | miR-6790-5p | 80% |
| miR-92a-2-5p | 62% | miR-4651 | 78% | miR-6791-5p | 78% |
| miR-139-3p | 78% | miR-4653-3p | 60% | miR-6792-5p | 79% |
| miR-181c-3p | 67% | miR-4654 | 55% | miR-6792-3p | 74% |
| miR-187-5p | 68% | miR-4655-5p | 78% | miR-6793-5p | 67% |
| miR-124-5p | 75% | miR-4656 | 75% | miR-6794-5p | 66% |
| miR-125b-1-3p | 100% | miR-4659b-3p | 67% | miR-6796-5p | 72% |
| miR-135a-3p | 63% | miR-4665-5p | 76% | miR-6797-5p | 75% |
| miR-138-1-3p | 58% | miR-4665-3p | 68% | miR-6798-5p | 82% |
| miR-149-3p | 77% | miR-4668-5p | 100% | miR-6799-5p | 73% |
| miR-185-3p | 81% | miR-4670-3p | 100% | miR-6800-5p | 76% |
| miR-186-3p | 56% | miR-4673 | 68% | miR-6800-3p | 66% |
| miR-193a-5p | 64% | miR-4674 | 76% | miR-6801-5p | 62% |
| miR-194-3p | 61% | miR-4676-5p | 59% | miR-6803-5p | 75% |
| miR-135b-3p | 100% | miR-4682 | 64% | miR-6803-3p | 80% |
| miR-339-3p | 53% | miR-4685-5p | 76% | miR-6804-3p | 79% |
| miR-423-5p | 81% | miR-4685-3p | 75% | miR-6805-5p | 81% |
| miR-18b-3p | 72% | miR-4687-5p | 77% | miR-6805-3p | 73% |
| miR-20b-3p | 64% | miR-1343-3p | 79% | miR-6806-5p | 81% |
| miR-483-5p | 65% | miR-4688 | 77% | miR-6807-5p | 66% |
| miR-486-3p | 80% | miR-4690-5p | 84% | miR-6808-5p | 70% |
| miR-501-3p | 100% | miR-4691-5p | 80% | miR-6809-5p | 65% |
| miR-532-3p | 78% | miR-4691-3p | 88% | miR-6809-3p | 67% |
| miR-92b-5p | 72% | miR-4695-5p | 71% | miR-6810-5p | 77% |
| miR-574-5p | 72% | miR-4695-3p | 76% | miR-6813-5p | 82% |
| miR-615-5p | 66% | miR-4697-5p | 69% | miR-6813-3p | 67% |
| miR-891a-5p | 58% | miR-4701-3p | 68% | miR-6815-3p | 68% |
| miR-876-3p | 67% | miR-4706 | 76% | miR-6816-5p | 80% |
| miR-744-5p | 82% | miR-4707-5p | 77% | miR-6818-5p | 64% |
| miR-885-5p | 72% | miR-4708-5p | 70% | miR-6820-5p | 73% |
| miR-885-3p | 68% | miR-4708-3p | 80% | miR-6821-5p | 83% |
| miR-877-3p | 79% | miR-4710 | 78% | miR-6822-5p | 78% |
| miR-887-3p | 71% | miR-4711-3p | 53% | miR-6824-5p | 77% |
| miR-665 | 80% | miR-4721 | 77% | miR-6825-5p | 69% |
| miR-760 | 76% | miR-4722-5p | 70% | miR-6825-3p | 82% |
| miR-936 | 71% | miR-4722-3p | 73% | miR-6829-5p | 78% |
| miR-939-5p | 76% | miR-4724-5p | 53% | miR-6829-3p | 75% |
| miR-1224-5p | 65% | miR-4725-5p | 63% | miR-6832-5p | 61% |
| miR-1224-3p | 72% | miR-4725-3p | 77% | miR-6833-3p | 71% |
| miR-1225-5p | 85% | miR-4728-3p | 72% | miR-6834-5p | 68% |
| miR-1225-3p | 71% | miR-4730 | 73% | miR-6780b-5p | 71% |
| miR-1228-5p | 72% | miR-4732-3p | 74% | miR-6836-5p | 52% |
| miR-1228-3p | 72% | miR-4734 | 79% | miR-6836-3p | 75% |
| miR-1231 | 82% | miR-4736 | 74% | miR-6837-5p | 62% |
| miR-1234-3p | 71% | miR-3064-5p | 64% | miR-6838-5p | 56% |
| miR-1238-3p | 68% | miR-4738-3p | 65% | miR-6839-5p | 55% |
| miR-320c | 57% | miR-4741 | 75% | miR-6840-3p | 81% |
| miR-1181 | 74% | miR-4743-5p | 75% | miR-6842-5p | 73% |
| miR-1202 | 70% | miR-122b-3p | 61% | miR-6845-5p | 76% |
| miR-663b | 75% | miR-4745-5p | 79% | miR-6845-3p | 77% |
| miR-1204 | 51% | miR-4746-3p | 73% | miR-6846-5p | 75% |
| miR-1207-5p | 83% | miR-4749-5p | 78% | miR-6847-5p | 54% |
| miR-1208 | 55% | miR-4750-5p | 76% | miR-6847-3p | 70% |
| miR-1293 | 51% | miR-4753-5p | 59% | miR-6848-5p | 82% |
| miR-1294 | 65% | miR-371b-5p | 79% | miR-6849-5p | 67% |
| miR-1304-5p | 61% | miR-4754 | 54% | miR-6849-3p | 67% |
| miR-1249-3p | 64% | miR-4758-5p | 82% | miR-6850-5p | 80% |
| miR-1260a | 81% | miR-4758-3p | 72% | miR-6851-5p | 72% |
| miR-1266-5p | 62% | miR-4759 | 92% | miR-6855-5p | 61% |
| miR-1268a | 84% | miR-4763-5p | 80% | miR-6857-5p | 80% |
| miR-1270 | 54% | miR-4763-3p | 79% | miR-6858-5p | 72% |
| miR-1278 | 100% | miR-4764-5p | 100% | miR-6859-5p | 64% |
| miR-1281 | 80% | miR-4769-3p | 72% | miR-6769b-5p | 72% |
| miR-1292-5p | 83% | miR-4770 | 60% | miR-6860 | 81% |
| miR-1255b-5p | 100% | miR-4776-5p | 75% | miR-6861-5p | 79% |
| miR-1307-3p | 80% | miR-4776-3p | 53% | miR-6861-3p | 73% |
| miR-320d | 71% | miR-4780 | 69% | miR-6862-3p | 80% |
| miR-675-3p | 68% | miR-4436b-5p | 78% | miR-6867-3p | 74% |
| miR-1469 | 80% | miR-4781-5p | 78% | miR-6869-5p | 77% |
| miR-1471 | 70% | miR-4783-3p | 80% | miR-6870-5p | 74% |
| miR-1538 | 68% | miR-4784 | 59% | miR-6871-5p | 59% |
| miR-1908-5p | 78% | miR-1245b-5p | 100% | miR-6875-5p | 74% |
| miR-1909-5p | 73% | miR-2467-3p | 69% | miR-6875-3p | 73% |
| miR-1909-3p | 77% | miR-4787-5p | 74% | miR-6877-5p | 78% |
| miR-1910-5p | 82% | miR-4800-5p | 69% | miR-6877-3p | 81% |
| miR-1912-3p | 65% | miR-4482-3p | 66% | miR-6879-5p | 69% |
| miR-1914-3p | 74% | miR-4999-5p | 58% | miR-6879-3p | 84% |
| miR-1915-3p | 82% | miR-5001-5p | 80% | miR-6880-5p | 73% |
| miR-365a-5p | 78% | miR-5002-3p | 68% | miR-6880-3p | 70% |
| miR-449b-3p | 78% | miR-5006-5p | 63% | miR-6882-5p | 57% |
| miR-1972 | 60% | miR-5008-5p | 73% | miR-6882-3p | 73% |
| miR-1976 | 76% | miR-5010-5p | 70% | miR-6883-5p | 76% |
| miR-762 | 77% | miR-5087 | 54% | miR-6884-5p | 57% |
| miR-548q | 83% | miR-5088-5p | 82% | miR-6886-3p | 77% |
| miR-2276-3p | 68% | miR-5089-5p | 58% | miR-6887-3p | 70% |
| miR-711 | 69% | miR-5090 | 70% | miR-6888-3p | 100% |
| miR-718 | 83% | miR-5189-5p | 68% | miR-6889-5p | 69% |
| miR-3119 | 64% | miR-5190 | 56% | miR-6890-5p | 71% |
| miR-3120-3p | 58% | miR-5196-5p | 74% | miR-6891-5p | 75% |
| miR-3130-5p | 70% | miR-5100 | 72% | miR-6892-5p | 56% |
| miR-3137 | 56% | miR-5572 | 75% | miR-6893-5p | 69% |
| miR-3141 | 82% | miR-5586-3p | 55% | miR-6893-3p | 75% |
| miR-1273c | 73% | miR-548au-3p | 56% | miR-6894-5p | 76% |
| miR-3147 | 79% | miR-5589-5p | 69% | miR-6894-3p | 75% |
| miR-3148 | 52% | miR-5684 | 67% | miR-6895-5p | 53% |
| miR-3151-5p | 76% | miR-5685 | 61% | miR-7106-5p | 66% |
| miR-3153 | 80% | miR-5690 | 57% | miR-7107-5p | 70% |
| miR-3159 | 100% | miR-5698 | 75% | miR-7108-5p | 76% |
| miR-3162-5p | 69% | miR-5703 | 52% | miR-7109-5p | 71% |
| miR-3163 | 56% | miR-5708 | 60% | miR-7109-3p | 73% |
| miR-3165 | 60% | miR-197-5p | 80% | miR-7110-5p | 80% |
| miR-1260b | 76% | miR-204-3p | 62% | miR-7111-5p | 70% |
| miR-1193 | 82% | miR-211-3p | 73% | miR-7112-5p | 77% |
| miR-3178 | 79% | miR-382-3p | 52% | miR-7113-3p | 79% |
| miR-3180-3p | 84% | miR-652-5p | 71% | miR-7114-5p | 69% |
| miR-3184-5p | 73% | miR-1247-3p | 75% | miR-7114-3p | 68% |
| miR-3185 | 74% | miR-3184-3p | 72% | miR-7150 | 71% |
| miR-3187-3p | 63% | miR-3191-5p | 73% | miR-7151-3p | 66% |
| miR-3191-3p | 78% | miR-365b-5p | 74% | miR-7155-5p | 78% |
| miR-3194-5p | 72% | miR-376c-5p | 100% | miR-7157-3p | 64% |
| miR-3195 | 69% | miR-381-5p | 54% | miR-7159-5p | 56% |
| miR-3196 | 78% | miR-937-5p | 79% | miR-7160-5p | 77% |
| miR-3197 | 80% | miR-1227-5p | 81% | miR-7515 | 64% |
| miR-514b-5p | 74% | miR-1233-5p | 73% | miR-7704 | 71% |
| miR-4293 | 54% | miR-1237-5p | 80% | miR-4433b-3p | 79% |
| miR-4298 | 73% | miR-1238-5p | 78% | miR-1273h-5p | 63% |
| miR-4305 | 67% | miR-3617-3p | 70% | miR-7845-5p | 72% |
| miR-4322 | 82% | miR-3620-5p | 76% | miR-7846-3p | 76% |
| miR-4323 | 68% | miR-4632-5p | 80% | miR-7847-3p | 72% |
| miR-4324 | 59% | miR-5787 | 74% | miR-7851-3p | 65% |
| miR-4256 | 64% | miR-6068 | 68% | miR-8052 | 77% |
| miR-4257 | 74% | miR-6069 | 75% | miR-8059 | 76% |
| miR-4258 | 78% | miR-6071 | 65% | miR-8063 | 77% |
| miR-4326 | 69% | miR-6072 | 65% | miR-8064 | 67% |
| miR-4327 | 78% | miR-6075 | 76% | miR-8069 | 78% |
| miR-4265 | 74% | miR-6076 | 67% | miR-8071 | 77% |
| miR-4268 | 67% | miR-6083 | 60% | miR-8072 | 80% |
| miR-4269 | 79% | miR-6086 | 80% | miR-8073 | 78% |
| miR-4271 | 72% | miR-6088 | 68% | miR-8074 | 53% |
| miR-4276 | 77% | miR-6089 | 73% | miR-8075 | 62% |
| miR-4279 | 76% | miR-6090 | 78% | miR-8077 | 60% |
| miR-4280 | 54% | miR-6124 | 71% | miR-8089 | 84% |
| miR-4285 | 56% | miR-6125 | 82% | miR-7977 | 79% |
| miR-500b-5p | 57% | miR-6126 | 72% | miR-7978 | 100% |
| miR-2277-5p | 58% | miR-6127 | 68% | miR-203a-5p | 55% |
| miR-3605-3p | 69% | miR-6131 | 70% | miR-1249-5p | 76% |
| miR-3610 | 63% | miR-6132 | 78% | miR-4485-5p | 81% |
| miR-3616-3p | 74% | miR-6133 | 77% | miR-8485 | 77% |
| miR-3619-5p | 55% | miR-6165 | 65% | miR-137-5p | 100% |
| miR-3621 | 78% | miR-548ay-3p | 80% | miR-498-3p | 57% |
| miR-3622a-3p | 70% | miR-6501-3p | 55% | miR-9718 | 63% |
| miR-3646 | 70% | miR-6506-5p | 51% | miR-9985 | 60% |
| miR-3648 | 75% | miR-6509-3p | 69% | miR-1843 | 64% |
| miR-3652 | 70% | miR-6511a-5p | 69% | miR-10392-5p | 75% |
| miR-3663-5p | 76% | miR-6511a-3p | 75% | miR-10392-3p | 79% |
| miR-3663-3p | 79% | miR-6512-3p | 64% | miR-10394-3p | 76% |
| miR-3665 | 71% | miR-6513-5p | 56% | miR-10396a-5p | 80% |
| miR-3677-3p | 65% | miR-6514-3p | 63% | miR-10396a-3p | 79% |
| miR-3679-5p | 72% | miR-6515-5p | 55% | miR-10398-5p | 73% |
| miR-3180 | 77% | miR-6716-5p | 76% | miR-10398-3p | 71% |
| miR-3907 | 78% | miR-6716-3p | 69% | miR-10400-5p | 67% |
| miR-3911 | 69% | miR-6717-5p | 66% | miR-10400-3p | 72% |
| miR-3916 | 59% | miR-6511b-5p | 56% | miR-10401-5p | 69% |
| miR-3917 | 75% | miR-6511b-3p | 76% | miR-10401-3p | 77% |
| miR-3919 | 59% | miR-6719-3p | 60% | miR-10396b-5p | 79% |
| miR-3926 | 54% | miR-6721-5p | 87% | miR-10522-5p | 71% |
| miR-3928-3p | 78% | miR-6722-5p | 80% | miR-10527-5p | 78% |
| miR-3934-5p | 53% | miR-6722-3p | 80% | miR-11399 | 72% |
| miR-3937 | 79% | miR-6724-5p | 80% | miR-11400 | 61% |
| miR-3941 | 63% | miR-208a-5p | 64% | miR-11401 | 64% |
| miR-3944-3p | 75% | miR-210-5p | 75% | miR-3085-5p | 63% |
| miR-1268b | 77% | miR-128-1-5p | 77% | miR-3085-3p | 70% |
| miR-4428 | 72% | miR-329-5p | 52% | miR-6529-5p | 64% |
| miR-4429 | 52% | miR-410-5p | 52% | miR-12114 | 66% |
| miR-4430 | 75% | miR-487a-5p | 53% | miR-12115 | 72% |
| miR-548ad-3p | 100% | miR-504-3p | 80% | miR-12118 | 79% |
| miR-4433a-3p | 82% | miR-585-5p | 66% | miR-12119 | 72% |
| miR-4436a | 65% | miR-598-5p | 57% | miR-12120 | 79% |
| miR-4439 | 58% | miR-1301-5p | 58% | miR-12121 | 83% |
| miR-4440 | 80% | miR-874-5p | 70% | miR-12128 | 65% |
| miR-4443 | 70% | miR-1908-3p | 75% | miR-12131 | 100% |
| miR-4444 | 53% | miR-1343-5p | 79% | | |
| miR-4446-3p | 80% | miR-5189-3p | 74% | | |

| | | | | | |
|---|---|---|---|---|---|
| ^{∗} A represents Accuracy. ^{∗} "has-" in the miRNA names has been omitted. | | | | | |

### [Table 23-14]

**Table 23-14: miRNA to Predict Human Endometrial Cancer of Stages I and II**

| miRNA Name | A | miRNA Name | A | miRNA Name | A |
|---|---|---|---|---|---|
| 1et-7e-5p | 54% | miR-3620-3p | 63% | miR-6506-5p | 60% |
| miR-15a-5p | 64% | miR-3621 | 72% | miR-6506-3p | 73% |
| miR-17-3p | 100% | miR-3622a-5p | 74% | miR-6507-3p | 54% |
| miR-29a-3p | 75% | miR-3622a-3p | 66% | miR-6508-5p | 55% |
| miR-30a-5p | 64% | miR-3622b-5p | 73% | miR-6509-5p | 54% |
| miR-31-5p | 100% | miR-3646 | 57% | miR-6509-3p | 63% |
| miR-29b-3p | 63% | miR-3648 | 76% | miR-6510-5p | 72% |
| miR-196a-5p | 72% | miR-3649 | 61% | miR-6511a-5p | 79% |
| miR-197-3p | 66% | miR-3652 | 70% | miR-6511a-3p | 65% |
| miR-198 | 67% | miR-3654 | 63% | miR-6512-3p | 55% |
| miR-199a-5p | 57% | miR-3660 | 63% | miR-6513-5p | 68% |
| miR-199a-3p, miR-199b-3p | 69% | miR-3663-5p | 68% | miR-6514-5p | 52% |
| miR-30d-5p | 67% | miR-3663-3p | 69% | miR-6514-3p | 61% |
| miR-139-5p | 100% | miR-3665 | 68% | miR-6515-5p | 67% |
| miR-187-3p | 56% | miR-3667-3p | 71% | miR-6515-3p | 73% |
| miR-199b-5p | 64% | miR-3675-5p | 59% | miR-6715b-3p | 67% |
| miR-214-3p | 52% | miR-3675-3p | 72% | miR-6716-5p | 68% |
| miR-223-3p | 67% | miR-3677-3p | 73% | miR-6716-3p | 65% |
| miR-122-5p | 55% | miR-3679-5p | 75% | miR-6717-5p | 73% |
| miR-124-3p | 71% | miR-3679-3p | 73% | miR-6511b-5p | 77% |
| miR-138-5p | 52% | miR-3682-3p | 53% | miR-6511b-3p | 72% |
| miR-140-5p | 80% | miR-3688-3p | 75% | miR-6719-3p | 74% |
| miR-141-3p | 75% | miR-3689a-3p | 55% | miR-6721-5p | 68% |
| miR-125a-5p | 54% | miR-3691-5p | 64% | miR-6722-5p | 63% |
| miR-134-5p | 64% | miR-3714 | 66% | miR-6722-3p | 71% |
| miR-149-5p | 64% | miR-3180 | 67% | miR-6724-5p | 73% |
| miR-154-3p | 59% | miR-3907 | 83% | miR-208a-5p | 60% |
| miR-184 | 54% | miR-3689b-3p, miR-3689c | 56% | miR-210-5p | 68% |
| miR-185-5p | 61% | miR-3909 | 68% | miR-128-1-5p | 70% |
| miR-188-5p | 57% | miR-3911 | 65% | miR-370-5p | 58% |
| miR-206 | 62% | miR-3913-5p | 58% | miR-328-5p | 63% |
| miR-106b-5p | 88% | miR-3916 | 68% | miR-329-5p | 57% |
| miR-29c-3p | 100% | miR-3917 | 70% | miR-410-5p | 62% |
| miR-34c-5p | 60% | miR-3918 | 56% | miR-487a-5p | 52% |
| miR-299-3p | 58% | miR-3919 | 73% | miR-489-5p | 62% |
| miR-296-5p | 67% | miR-3150b-3p | 54% | miR-181d-3p | 80% |
| miR-130b-3p | 83% | miR-3924 | 100% | miR-504-3p | 73% |
| miR-361-5p | 66% | miR-3925-5p | 62% | miR-487b-5p | 60% |
| miR-365a-3p, miR-365b-3p | 68% | miR-3926 | 65% | miR-579-5p | 53% |
| miR-302c-5p | 62% | miR-676-3p | 59% | miR-585-5p | 58% |
| miR-370-3p | 73% | miR-3928-3p | 71% | miR-597-3p | 67% |
| miR-371a-3p | 53% | miR-3934-5p | 51% | miR-598-5p | 71% |
| miR-373-5p | 66% | miR-3935 | 59% | miR-605-3p | 61% |
| miR-373-3p | 71% | miR-3937 | 77% | miR-619-5p | 57% |
| miR-374a-5p | 100% | miR-3940-3p | 68% | miR-1296-3p | 61% |
| miR-375-3p | 65% | miR-3943 | 68% | miR-891a-3p | 57% |
| miR-380-3p | 70% | miR-3944-3p | 70% | miR-874-5p | 70% |
| miR-382-5p | 54% | miR-3945 | 61% | miR-1180-5p | 51% |
| miR-383-5p | 70% | miR-642b-3p | 69% | miR-1250-3p | 56% |
| miR-328-3p | 68% | miR-550b-3p | 61% | miR-1266-3p | 58% |
| miR-342-3p | 55% | miR-1268b | 77% | miR-1908-3p | 69% |
| miR-326 | 63% | miR-378e | 54% | miR-3151-3p | 64% |
| miR-151a-3p | 59% | miR-548ab | 79% | miR-3192-3p | 60% |
| miR-133b | 66% | miR-378f | 59% | miR-500b-3p | 52% |
| miR-325 | 61% | miR-4421 | 59% | miR-1343-5p | 76% |
| miR-346 | 62% | miR-4428 | 71% | miR-5088-3p | 62% |
| miR-20b-5p | 67% | miR-4429 | 70% | miR-5189-3p | 58% |
| miR-448 | 56% | miR-4430 | 75% | miR-5699-5p | 71% |
| miR-429 | 100% | miR-4431 | 53% | miR-6726-5p | 79% |
| miR-449a | 65% | miR-4433a-3p | 76% | miR-6726-3p | 64% |
| miR-450a-5p | 62% | miR-4434 | 100% | miR-6727-5p | 68% |
| miR-200a-5p | 64% | miR-4435 | 51% | miR-6727-3p | 67% |
| miR-433-3p | 61% | miR-4436a | 54% | miR-6728-5p | 50% |
| miR-323b-5p | 54% | miR-4439 | 71% | miR-6728-3p | 70% |
| miR-452-3p | 55% | miR-4440 | 70% | miR-6729-5p | 70% |
| miR-409-3p | 67% | miR-4441 | 70% | miR-6729-3p | 70% |
| miR-483-3p | 66% | miR-4443 | 64% | miR-6730-5p | 74% |
| miR-484 | 66% | miR-4444 | 61% | miR-6730-3p | 71% |
| miR-485-3p | 59% | miR-4446-3p | 71% | miR-6731-5p | 58% |
| miR-486-5p | 69% | miR-4447 | 76% | miR-6731-3p | 74% |
| miR-487a-3p | 56% | miR-4448 | 61% | miR-6732-5p | 76% |
| miR-491-5p | 71% | miR-4449 | 68% | miR-6732-3p | 66% |
| miR-202-3p | 68% | miR-4450 | 56% | miR-6734-5p | 75% |
| miR-492 | 67% | miR-4451 | 60% | miR-6734-3p | 65% |
| miR-498-5p | 79% | miR-4454 | 59% | miR-6735-5p | 61% |
| miR-520e-3p | 64% | miR-4455 | 58% | miR-6735-3p | 60% |
| miR-519c-5p, miR-523-5p, miR-518e-5p, miR-522-5p, miR-519a-5p, miR-519b-5p | 51% | miR-4456 | 64% | miR-6736-5p | 63% |
| miR-519b-3p | 55% | miR-4458 | 72% | miR-6736-3p | 64% |
| miR-525-3p | 61% | miR-4460 | 69% | miR-6737-5p | 74% |
| miR-518f-3p | 56% | miR-378h | 64% | miR-6737-3p | 70% |
| miR-520b-3p | 63% | miR-3135b | 72% | miR-6738-5p | 71% |
| miR-518b | 65% | miR-4462 | 70% | miR-6738-3p | 61% |
| miR-520c-3p | 75% | miR-4463 | 69% | miR-6740-5p | 65% |
| miR-518c-5p | 61% | miR-4466 | 73% | miR-6740-3p | 66% |
| miR-520d-5p | 54% | miR-4467 | 73% | miR-6741-5p | 70% |
| miR-520d-3p | 55% | miR-4470 | 87% | miR-6741-3p | 65% |
| miR-516b-5p | 75% | miR-4472 | 77% | miR-6742-5p | 75% |
| miR-516b-3p, miR-516a-3p | 61% | miR-4475 | 68% | miR-6742-3p | 69% |
| miR-518a-3p | 78% | miR-4476 | 63% | miR-6743-5p | 76% |
| miR-517c-3p | 51% | miR-4478 | 70% | miR-6743-3p | 66% |
| miR-500a-3p | 63% | miR-3689f | 60% | miR-6744-5p | 58% |
| miR-502-5p | 62% | miR-4481 | 71% | miR-6744-3p | 58% |
| miR-503-5p | 86% | miR-4483 | 74% | miR-6745 | 67% |
| miR-513a-5p | 63% | miR-4484 | 74% | miR-6746-5p | 77% |
| miR-510-5p | 55% | miR-4485-3p | 62% | miR-6747-5p | 69% |
| miR-299-5p | 51% | miR-4486 | 76% | miR-6747-3p | 60% |
| miR-539-5p | 61% | miR-4487 | 75% | miR-6748-5p | 78% |
| miR-487b-3p | 55% | miR-4488 | 67% | miR-6748-3p | 64% |
| miR-551a | 50% | miR-4489 | 70% | miR-6749-5p | 75% |
| miR-552-3p | 64% | miR-4492 | 67% | miR-6749-3p | 70% |
| miR-554 | 62% | miR-4494 | 61% | miR-6750-5p | 57% |
| miR-555 | 59% | miR-4496 | 77% | miR-6750-3p | 67% |
| miR-557 | 68% | miR-4497 | 72% | miR-6751-5p | 77% |
| miR-558 | 53% | miR-4498 | 72% | miR-6751-3p | 63% |
| miR-563 | 67% | miR-4499 | 65% | miR-6752-5p | 73% |
| miR-564 | 67% | miR-4501 | 62% | miR-6752-3p | 69% |
| miR-571 | 80% | miR-4505 | 73% | miR-6753-5p | 69% |
| miR-573 | 100% | miR-4506 | 64% | miR-6753-3p | 64% |
| miR-574-3p | 66% | miR-2392 | 77% | miR-6754-5p | 65% |
| miR-575 | 64% | miR-4507 | 76% | miR-6754-3p | 60% |
| miR-578 | 70% | miR-4508 | 71% | miR-6755-5p | 75% |
| miR-579-3p | 71% | miR-4510 | 64% | miR-6755-3p | 55% |
| miR-583 | 67% | miR-4511 | 62% | miR-6756-5p | 71% |
| miR-585-3p | 63% | miR-4512 | 75% | miR-6756-3p | 70% |
| miR-595 | 57% | miR-4513 | 73% | miR-6757-5p | 63% |
| miR-608 | 61% | miR-4516 | 70% | miR-6757-3p | 67% |
| miR-610 | 64% | miR-4518 | 67% | miR-6758-5p | 70% |
| miR-612 | 57% | miR-4519 | 80% | miR-6759-5p | 77% |
| miR-615-3p | 56% | miR-4520-3p | 67% | miR-6759-3p | 72% |
| miR-616-5p | 75% | miR-4521 | 77% | miR-6760-5p | 71% |
| miR-617 | 65% | miR-1269b | 67% | miR-6760-3p | 73% |
| miR-619-3p | 100% | miR-4522 | 61% | miR-6761-5p | 53% |
| miR-626 | 100% | miR-4523 | 55% | miR-6761-3p | 60% |
| miR-628-3p | 62% | miR-4524a-3p | 75% | miR-6762-5p | 67% |
| miR-629-3p | 50% | miR-4525 | 68% | miR-6762-3p | 62% |
| miR-632 | 50% | miR-4526 | 67% | miR-6763-5p | 75% |
| miR-634 | 72% | miR-4530 | 74% | miR-6763-3p | 72% |
| miR-636 | 61% | miR-4531 | 64% | miR-6765-5p | 65% |
| miR-637 | 75% | miR-4533 | 70% | miR-6766-5p | 78% |
| miR-638 | 72% | miR-4534 | 70% | miR-6766-3p | 71% |
| miR-639 | 88% | miR-378i | 58% | miR-6767-5p | 60% |
| miR-642a-5p | 61% | miR-4535 | 72% | miR-6768-5p | 72% |
| miR-644a | 52% | miR-1587 | 77% | miR-6769a-5p | 68% |
| miR-649 | 51% | miR-4538 | 72% | miR-6769a-3p | 70% |
| miR-663a | 70% | miR-4539 | 74% | miR-6770-5p | 65% |
| miR-449b-5p | 61% | miR-4540 | 54% | miR-6770-3p | 52% |
| miR-654-5p | 50% | miR-3127-3p | 66% | miR-6771-5p | 58% |
| miR-657 | 56% | miR-3074-5p | 55% | miR-6771-3p | 72% |
| miR-658 | 78% | miR-3156-3p | 61% | miR-6772-5p | 73% |
| miR-542-5p | 94% | miR-3158-5p | 66% | miR-6772-3p | 60% |
| miR-363-5p | 56% | miR-3162-3p | 71% | miR-6773-3p | 58% |
| miR-425-5p | 56% | miR-3173-5p | 61% | miR-6774-5p | 71% |
| miR-671-5p | 65% | miR-3177-5p | 57% | miR-6775-5p | 66% |
| miR-668-3p | 59% | miR-3187-5p | 69% | miR-6775-3p | 68% |
| miR-767-3p | 70% | miR-3189-5p | 69% | miR-6776-5p | 74% |
| miR-769-5p | 64% | miR-3619-3p | 70% | miR-6776-3p | 69% |
| miR-769-3p | 55% | miR-3682-5p | 51% | miR-6777-5p | 72% |
| miR-766-3p | 70% | miR-3913-3p | 54% | miR-6777-3p | 70% |
| miR-765 | 67% | miR-3150b-5p | 67% | miR-6778-5p | 75% |
| miR-675-5p | 66% | miR-3922-5p | 54% | miR-6778-3p | 60% |
| miR-297 | 66% | miR-3925-3p | 61% | miR-6779-5p | 67% |
| let-7b-3p | 65% | miR-3940-5p | 68% | miR-6779-3p | 66% |
| let-7d-3p | 57% | miR-4423-5p | 55% | miR-6780a-5p | 70% |
| let-7f-1-3p | 60% | miR-3960 | 63% | miR-6780a-3p | 59% |
| miR-22-5p | 100% | miR-3972 | 57% | miR-6781-5p | 74% |
| miR-24-2-5p | 57% | miR-4632-3p | 54% | miR-6782-5p | 78% |
| miR-32-3p | 54% | miR-4634 | 72% | miR-6782-3p | 73% |
| miR-92a-2-5p | 66% | miR-4638-5p | 76% | miR-6783-5p | 66% |
| miR-29b-2-5p | 59% | miR-4639-3p | 52% | miR-6783-3p | 59% |
| miR-129-1-3p | 65% | miR-4640-5p | 77% | miR-6784-5p | 70% |
| miR-30c-2-3p | 68% | miR-4640-3p | 66% | miR-6784-3p | 72% |
| miR-139-3p | 66% | miR-4642 | 68% | miR-6785-3p | 71% |
| miR-181c-3p | 75% | miR-4643 | 58% | miR-6786-5p | 70% |
| miR-183-3p | 68% | miR-4644 | 55% | miR-6786-3p | 70% |
| miR-187-5p | 69% | miR-4646-5p | 67% | miR-6787-5p | 72% |
| miR-200b-5p | 59% | miR-4646-3p | 66% | miR-6787-3p | 61% |
| miR-15b-3p | 65% | miR-4647 | 55% | miR-6788-5p | 53% |
| miR-23b-5p | 60% | miR-4648 | 73% | miR-6788-3p | 62% |
| miR-30b-3p | 61% | miR-4649-5p | 61% | miR-6789-5p | 74% |
| miR-124-5p | 70% | miR-4649-3p | 67% | miR-6789-3p | 60% |
| miR-125b-1-3p | 100% | miR-4650-3p | 75% | miR-6790-5p | 72% |
| miR-130a-5p | 59% | miR-4651 | 77% | miR-6790-3p | 67% |
| miR-135a-3p | 59% | miR-4652-5p | 53% | miR-6791-5p | 72% |
| miR-140-3p | 63% | miR-4652-3p | 65% | miR-6792-5p | 63% |
| miR-125a-3p | 58% | miR-4653-3p | 67% | miR-6792-3p | 69% |
| miR-125b-2-3p | 67% | miR-4654 | 62% | miR-6793-5p | 63% |
| miR-129-2-3p | 67% | miR-4655-5p | 69% | miR-6793-3p | 68% |
| miR-138-1-3p | 54% | miR-4656 | 68% | miR-6794-5p | 77% |
| miR-149-3p | 67% | miR-4657 | 61% | miR-6794-3p | 64% |
| miR-150-3p | 73% | miR-4661-5p | 59% | miR-6795-5p | 73% |
| miR-185-3p | 74% | miR-4661-3p | 57% | miR-6795-3p | 71% |
| miR-186-3p | 67% | miR-4659b-3p | 71% | miR-6796-5p | 74% |
| miR-188-3p | 59% | miR-4664-5p | 67% | miR-6796-3p | 74% |
| miR-193a-5p | 60% | miR-4664-3p | 70% | miR-6797-5p | 74% |
| miR-194-3p | 68% | miR-4665-5p | 80% | miR-6797-3p | 67% |
| miR-30c-1-3p | 62% | miR-4665-3p | 71% | miR-6798-5p | 74% |
| miR-219a-2-3p | 57% | miR-4667-5p | 71% | miR-6798-3p | 72% |
| miR-296-3p | 67% | miR-4667-3p | 66% | miR-6799-5p | 71% |
| miR-130b-5p | 53% | miR-4669 | 69% | miR-6799-3p | 69% |
| miR-361-3p | 70% | miR-4670-5p | 59% | miR-6800-5p | 66% |
| miR-371a-5p | 63% | miR-4670-3p | 100% | miR-6800-3p | 70% |
| miR-377-5p | 63% | miR-4671-3p | 80% | miR-6801-3p | 68% |
| miR-342-5p | 70% | miR-4672 | 67% | miR-6802-5p | 63% |
| miR-323a-5p | 52% | miR-4673 | 60% | miR-6802-3p | 69% |
| miR-151a-5p | 56% | miR-4674 | 70% | miR-6803-5p | 66% |
| miR-148b-5p | 61% | miR-4676-5p | 56% | miR-6803-3p | 66% |
| miR-338-5p | 58% | miR-4677-5p | 78% | miR-6804-3p | 68% |
| miR-423-5p | 70% | miR-4681 | 60% | miR-6805-5p | 72% |
| miR-424-3p | 76% | miR-4682 | 53% | miR-6805-3p | 71% |
| miR-18b-3p | 67% | miR-4684-3p | 61% | miR-6806-5p | 64% |
| miR-20b-3p | 76% | miR-4685-5p | 67% | miR-6806-3p | 100% |
| miR-431-3p | 52% | miR-4685-3p | 65% | miR-6807-5p | 68% |
| miR-483-5p | 78% | miR-4687-5p | 73% | miR-6808-5p | 66% |
| miR-486-3p | 70% | miR-4687-3p | 67% | miR-6808-3p | 63% |
| miR-490-5p | 100% | miR-1343-3p | 75% | miR-6809-5p | 80% |
| miR-146b-3p | 50% | miR-4688 | 74% | miR-6809-3p | 61% |
| miR-193b-5p | 54% | miR-4689 | 63% | miR-6810-5p | 79% |
| miR-500a-5p | 53% | miR-4690-5p | 70% | miR-6810-3p | 72% |
| miR-509-5p | 62% | miR-4690-3p | 63% | miR-6812-5p | 73% |
| miR-532-3p | 67% | miR-4691-3p | 89% | miR-6812-3p | 67% |
| miR-92b-5p | 76% | miR-4692 | 51% | miR-6813-5p | 72% |
| miR-551b-5p | 66% | miR-4694-5p | 61% | miR-6813-3p | 70% |
| miR-574-5p | 61% | miR-4695-5p | 74% | miR-6814-3p | 55% |
| miR-550a-5p | 72% | miR-4695-3p | 66% | miR-6815-5p | 63% |
| miR-615-5p | 70% | miR-4697-5p | 64% | miR-6815-3p | 51% |
| miR-616-3p | 62% | miR-4697-3p | 74% | miR-6816-5p | 67% |
| miR-624-3p | 75% | miR-4698 | 73% | miR-6816-3p | 57% |
| miR-625-3p | 71% | miR-4700-5p | 63% | miR-6818-5p | 71% |
| miR-629-5p | 83% | miR-4700-3p | 71% | miR-6819-5p | 64% |
| miR-298 | 70% | miR-4701-5p | 73% | miR-6819-3p | 69% |
| miR-874-3p | 67% | miR-4701-3p | 66% | miR-6820-5p | 74% |
| miR-891b | 78% | miR-4703-3p | 56% | miR-6820-3p | 72% |
| miR-541-3p | 59% | miR-4704-5p | 59% | miR-6821-5p | 75% |
| miR-708-5p | 53% | miR-4706 | 63% | miR-6822-5p | 68% |
| miR-147b-3p | 58% | miR-4707-5p | 71% | miR-6823-5p | 62% |
| miR-744-5p | 68% | miR-4707-3p | 71% | miR-6823-3p | 63% |
| miR-885-5p | 62% | miR-4708-5p | 60% | miR-6824-5p | 68% |
| miR-885-3p | 69% | miR-4708-3p | 61% | miR-6824-3p | 63% |
| miR-877-5p | 69% | miR-4709-3p | 67% | miR-6825-5p | 74% |
| miR-877-3p | 73% | miR-203b-5p | 59% | miR-6825-3p | 70% |
| miR-887-3p | 68% | miR-4710 | 68% | miR-6826-3p | 69% |
| miR-665 | 71% | miR-4712-3p | 54% | miR-6827-5p | 72% |
| miR-760 | 73% | miR-4713-5p | 66% | miR-6827-3p | 71% |
| miR-920 | 68% | miR-4714-5p | 66% | miR-6828-5p | 57% |
| miR-924 | 51% | miR-4715-5p | 67% | miR-6829-5p | 76% |
| miR-936 | 59% | miR-4716-5p | 74% | miR-6829-3p | 60% |
| miR-938 | 57% | miR-4716-3p | 70% | miR-6830-5p | 75% |
| miR-939-5p | 74% | miR-4717-3p | 62% | miR-6830-3p | 64% |
| miR-940 | 67% | miR-4720-5p | 67% | miR-6831-5p | 72% |
| miR-1224-5p | 71% | miR-4721 | 73% | miR-6831-3p | 55% |
| miR-1224-3p | 71% | miR-4722-5p | 60% | miR-6832-5p | 77% |
| miR-1225-5p | 74% | miR-4722-3p | 63% | miR-6833-5p | 74% |
| miR-1225-3p | 70% | miR-4723-5p | 67% | miR-6833-3p | 55% |
| miR-1226-3p | 50% | miR-4723-3p | 67% | miR-6834-5p | 70% |
| miR-1228-5p | 65% | miR-4724-5p | 63% | miR-6834-3p | 68% |
| miR-1228-3p | 71% | miR-4725-5p | 51% | miR-6780b-5p | 75% |
| miR-1229-3p | 65% | miR-4725-3p | 77% | miR-6780b-3p | 68% |
| miR-1231 | 69% | miR-4726-5p | 62% | miR-6836-5p | 52% |
| miR-1233-3p | 64% | miR-4726-3p | 57% | miR-6836-3p | 71% |
| miR-1234-3p | 68% | miR-4727-3p | 55% | miR-6837-5p | 52% |
| miR-1236-3p | 71% | miR-4728-5p | 71% | miR-6838-5p | 67% |
| miR-1237-3p | 69% | miR-4728-3p | 69% | miR-6839-5p | 67% |
| miR-1238-3p | 68% | miR-4730 | 65% | miR-6840-5p | 67% |
| miR-1264 | 57% | miR-4731-5p | 63% | miR-6840-3p | 71% |
| miR-320b | 54% | miR-4731-3p | 70% | miR-6841-3p | 69% |
| miR-320c | 72% | miR-4732-5p | 75% | miR-6842-5p | 76% |
| miR-1296-5p | 55% | miR-4732-3p | 61% | miR-6842-3p | 59% |
| miR-1301-3p | 67% | miR-4734 | 71% | miR-6845-5p | 75% |
| miR-1298-5p | 66% | miR-4736 | 72% | miR-6845-3p | 73% |
| miR-1181 | 61% | miR-3064-5p | 52% | miR-6846-5p | 76% |
| miR-1182 | 69% | miR-3064-3p | 60% | miR-6846-3p | 67% |
| miR-1184 | 55% | miR-4738-3p | 67% | miR-6847-5p | 53% |
| miR-1202 | 67% | miR-4739 | 66% | miR-6847-3p | 61% |
| miR-1203 | 60% | miR-4741 | 76% | miR-6848-5p | 71% |
| miR-663b | 62% | miR-4742-3p | 83% | miR-6848-3p | 67% |
| miR-1207-5p | 73% | miR-4743-5p | 76% | miR-6849-5p | 72% |
| miR-1208 | 64% | miR-122b-5p | 100% | miR-6849-3p | 53% |
| miR-1285-3p | 60% | miR-4745-5p | 72% | miR-6850-5p | 71% |
| miR-1286 | 63% | miR-4745-3p | 58% | miR-6851-5p | 64% |
| miR-1289 | 73% | miR-4746-3p | 71% | miR-6851-3p | 68% |
| miR-1293 | 55% | miR-4747-5p | 73% | miR-6855-5p | 65% |
| miR-1294 | 72% | miR-4747-3p | 51% | miR-6855-3p | 72% |
| miR-1303 | 57% | miR-4748 | 69% | miR-6856-5p | 64% |
| miR-1304-5p | 64% | miR-4749-5p | 76% | miR-6857-5p | 72% |
| miR-1247-5p | 67% | miR-4749-3p | 71% | miR-6857-3p | 72% |
| miR-1249-3p | 73% | miR-4750-5p | 73% | miR-6858-5p | 66% |
| miR-1257 | 60% | miR-4751 | 72% | miR-6858-3p | 70% |
| miR-1260a | 66% | miR-4753-5p | 70% | miR-6859-5p | 61% |
| miR-1263 | 65% | miR-371b-5p | 69% | miR-6859-3p | 71% |
| miR-1265 | 54% | miR-371b-3p | 66% | miR-6769b-5p | 73% |
| miR-1267 | 68% | miR-4754 | 57% | miR-6769b-3p | 61% |
| miR-1268a | 69% | miR-4755-3p | 61% | miR-6860 | 63% |
| miR-1269a | 57% | miR-499b-3p | 65% | miR-6861-5p | 76% |
| miR-1270 | 64% | miR-4756-5p | 62% | miR-6861-3p | 70% |
| miR-1275 | 61% | miR-4758-5p | 80% | miR-6862-5p | 55% |
| miR-1276 | 54% | miR-4758-3p | 70% | miR-6862-3p | 69% |
| miR-1281 | 70% | miR-4759 | 80% | miR-6867-5p | 58% |
| miR-1292-5p | 88% | miR-4762-3p | 83% | miR-6867-3p | 62% |
| miR-1255b-5p | 100% | miR-4763-5p | 69% | miR-6869-5p | 68% |
| miR-664a-3p | 66% | miR-4763-3p | 75% | miR-6870-5p | 74% |
| miR-1307-3p | 72% | miR-4766-5p | 79% | miR-6870-3p | 71% |
| miR-320d | 75% | miR-4769-5p | 69% | miR-6871-5p | 66% |
| miR-1825 | 68% | miR-4769-3p | 69% | miR-6872-5p | 57% |
| miR-675-3p | 63% | miR-4776-5p | 71% | miR-6872-3p | 71% |
| miR-1469 | 73% | miR-4776-3p | 52% | miR-6874-5p | 61% |
| miR-1470 | 66% | miR-4778-5p | 74% | miR-6875-5p | 73% |
| miR-1471 | 70% | miR-4780 | 57% | miR-6875-3p | 57% |
| miR-1538 | 60% | miR-4436b-5p | 65% | miR-6876-5p | 73% |
| miR-1539 | 58% | miR-4436b-3p | 51% | miR-6877-5p | 77% |
| miR-1908-5p | 76% | miR-4781-5p | 82% | miR-6877-3p | 65% |
| miR-1909-5p | 63% | miR-4783-3p | 72% | miR-6878-3p | 58% |
| miR-1909-3p | 72% | miR-4784 | 54% | miR-6879-5p | 74% |
| miR-1910-5p | 64% | miR-4785 | 60% | miR-6879-3p | 67% |
| miR-1911-5p | 100% | miR-2467-5p | 61% | miR-6880-5p | 76% |
| miR-1912-3p | 77% | miR-2467-3p | 75% | miR-6880-3p | 69% |
| miR-1913 | 71% | miR-4786-5p | 52% | miR-6881-5p | 68% |
| miR-1914-3p | 76% | miR-4786-3p | 58% | miR-6881-3p | 50% |
| miR-1915-3p | 72% | miR-4787-5p | 67% | miR-6882-3p | 63% |
| miR-365a-5p | 72% | miR-4787-3p | 70% | miR-6883-5p | 70% |
| miR-449b-3p | 69% | miR-4788 | 63% | miR-6883-3p | 56% |
| miR-2113 | 56% | miR-4793-3p | 65% | miR-6884-3p | 69% |
| miR-1976 | 72% | miR-4794 | 57% | miR-6885-3p | 69% |
| miR-2054 | 100% | miR-4797-3p | 53% | miR-6886-5p | 56% |
| miR-2110 | 72% | miR-4799-5p | 63% | miR-6886-3p | 68% |
| miR-151b | 57% | miR-4800-5p | 78% | miR-6887-5p | 75% |
| miR-449c-5p | 57% | miR-4801 | 55% | miR-6887-3p | 71% |
| miR-762 | 70% | miR-4804-5p | 86% | miR-6888-5p | 56% |
| miR-670-5p | 59% | miR-4804-3p | 52% | miR-6889-5p | 79% |
| miR-761 | 56% | miR-4520-2-3p | 100% | miR-6889-3p | 68% |
| miR-764 | 61% | miR-642a-3p | 67% | miR-6890-5p | 61% |
| miR-2114-5p | 57% | miR-550a-3-5p | 57% | miR-6890-3p | 69% |
| miR-2116-3p | 68% | miR-4433a-5p | 71% | miR-6891-5p | 66% |
| miR-548q | 69% | miR-4482-3p | 65% | miR-6891-3p | 73% |
| miR-2276-3p | 69% | miR-4999-5p | 68% | miR-6892-5p | 69% |
| miR-2278 | 55% | miR-5000-3p | 65% | miR-6892-3p | 71% |
| miR-711 | 73% | miR-5001-5p | 69% | miR-6893-5p | 76% |
| miR-718 | 67% | miR-5002-3p | 56% | miR-6893-3p | 61% |
| miR-2681-3p | 64% | miR-5003-5p | 53% | miR-6894-5p | 77% |
| miR-2682-5p | 54% | miR-5003-3p | 67% | miR-6894-3p | 65% |
| miR-2682-3p | 58% | miR-5004-3p | 60% | miR-6895-3p | 52% |
| miR-449c-3p | 55% | miR-5006-5p | 53% | miR-7106-5p | 71% |
| miR-2861 | 62% | miR-5007-5p | 57% | miR-7106-3p | 60% |
| miR-3116 | 51% | miR-5008-5p | 61% | miR-7107-5p | 75% |
| miR-3119 | 71% | miR-5009-3p | 61% | miR-7107-3p | 54% |
| miR-3120-3p | 62% | miR-5010-5p | 70% | miR-7108-5p | 76% |
| miR-3122 | 71% | miR-5010-3p | 67% | miR-7108-3p | 70% |
| miR-3124-5p | 54% | miR-5087 | 65% | miR-7109-5p | 69% |
| miR-3126-5p | 56% | miR-5088-5p | 69% | miR-7109-3p | 64% |
| miR-3130-3p | 53% | miR-5089-5p | 64% | miR-7110-5p | 77% |
| miR-3130-5p | 64% | miR-5090 | 69% | miR-7110-3p | 67% |
| miR-3131 | 73% | miR-5092 | 75% | miR-7111-5p | 75% |
| miR-3132 | 60% | miR-5093 | 55% | miR-7111-3p | 70% |
| miR-3133 | 55% | miR-5187-5p | 54% | miR-7112-5p | 59% |
| miR-466 | 55% | miR-5188 | 62% | miR-7113-5p | 54% |
| miR-3138 | 68% | miR-5189-5p | 68% | miR-7113-3p | 71% |
| miR-3141 | 76% | miR-5190 | 56% | miR-7114-5p | 72% |
| miR-3144-5p | 65% | miR-5193 | 62% | miR-7114-3p | 69% |
| miR-1273c | 76% | miR-5194 | 52% | miR-7150 | 72% |
| miR-3147 | 72% | miR-5195-5p | 68% | miR-7151-3p | 63% |
| miR-3148 | 64% | miR-5195-3p | 64% | miR-7152-5p | 58% |
| miR-3150a-3p | 68% | miR-5196-5p | 76% | miR-7154-3p | 54% |
| miR-3151-5p | 71% | miR-5196-3p | 61% | miR-7155-5p | 64% |
| miR-3153 | 72% | miR-4524b-5p | 61% | miR-7157-3p | 75% |
| miR-3154 | 68% | miR-5100 | 67% | miR-7159-5p | 64% |
| miR-3156-5p | 69% | miR-5572 | 80% | miR-7160-5p | 69% |
| miR-3157-5p | 70% | miR-664b-5p | 63% | miR-7161-5p | 57% |
| miR-3158-3p | 52% | miR-664b-3p | 64% | miR-7162-5p | 61% |
| miR-3160-3p | 64% | miR-5581-5p | 56% | miR-7515 | 78% |
| miR-3161 | 64% | miR-5581-3p | 55% | miR-7702 | 53% |
| miR-3162-5p | 77% | miR-5582-3p | 100% | miR-7704 | 70% |
| miR-3163 | 69% | miR-5584-5p | 71% | miR-7706 | 53% |
| miR-3164 | 61% | miR-548au-3p | 61% | miR-7843-5p | 59% |
| miR-3165 | 78% | miR-5588-5p | 62% | miR-4433b-5p | 69% |
| miR-1260b | 65% | miR-5589-5p | 71% | miR-4433b-3p | 79% |
| miR-3169 | 68% | miR-5589-3p | 60% | miR-1273h-5p | 70% |
| miR-3173-3p | 68% | miR-5684 | 75% | miR-6516-5p | 62% |
| miR-1193 | 67% | miR-5689 | 54% | miR-7845-5p | 64% |
| miR-3175 | 65% | miR-5690 | 69% | miR-7846-3p | 69% |
| miR-3176 | 59% | miR-4666b | 70% | miR-7847-3p | 66% |
| miR-3177-3p | 66% | miR-5693 | 67% | miR-7851-3p | 66% |
| miR-3178 | 69% | miR-5695 | 80% | miR-7855-5p | 63% |
| miR-3179 | 60% | miR-5696 | 52% | miR-7856-5p | 58% |
| miR-3180-5p | 68% | miR-5698 | 74% | miR-8052 | 72% |
| miR-3180-3p | 71% | miR-5699-3p | 57% | miR-8054 | 86% |
| miR-3184-5p | 64% | miR-5702 | 56% | miR-8057 | 57% |
| miR-3185 | 79% | miR-5703 | 67% | miR-8059 | 69% |
| miR-3187-3p | 58% | miR-5704 | 58% | miR-8060 | 66% |
| miR-3188 | 66% | miR-5705 | 62% | miR-8062 | 55% |
| miR-3189-3p | 67% | miR-5708 | 51% | miR-8063 | 64% |
| miR-3190-5p | 62% | miR-197-5p | 74% | miR-8064 | 66% |
| miR-3191-3p | 71% | miR-181b-3p | 77% | miR-8068 | 78% |
| miR-3192-5p | 53% | miR-204-3p | 70% | miR-8069 | 71% |
| miR-3193 | 56% | miR-211-3p | 74% | miR-8071 | 75% |
| miR-3194-5p | 62% | miR-301a-5p | 62% | miR-8072 | 75% |
| miR-3195 | 68% | miR-382-3p | 65% | miR-8073 | 76% |
| miR-3196 | 71% | miR-345-3p | 65% | miR-8074 | 63% |
| miR-3197 | 76% | miR-652-5p | 70% | miR-8075 | 62% |
| miR-3198 | 66% | miR-1185-2-3p | 72% | miR-8077 | 69% |
| miR-514b-5p | 61% | miR-766-5p | 54% | miR-8078 | 58% |
| miR-3202 | 68% | miR-873-3p | 63% | miR-8079 | 58% |
| miR-4296 | 52% | miR-1304-3p | 69% | miR-8080 | 68% |
| miR-4297 | 59% | miR-1247-3p | 72% | miR-8085 | 76% |
| miR-4293 | 78% | miR-1306-5p | 70% | miR-8086 | 57% |
| miR-4294 | 75% | miR-3184-3p | 69% | miR-8087 | 73% |
| miR-4299 | 55% | miR-3191-5p | 64% | miR-8089 | 73% |
| miR-4298 | 67% | miR-642b-5p | 58% | miR-128-2-5p | 60% |
| miR-4300 | 56% | miR-365b-5p | 70% | miR-1199-5p | 52% |
| miR-4303 | 52% | miR-1185-1-3p | 74% | miR-7977 | 62% |
| miR-4305 | 58% | miR-98-3p | 65% | miR-7978 | 100% |
| miR-4306 | 63% | miR-381-5p | 70% | miR-1249-5p | 67% |
| miR-4307 | 65% | miR-495-5p | 53% | miR-4485-5p | 69% |
| miR-4312 | 68% | miR-503-3p | 63% | miR-8485 | 65% |
| miR-4313 | 65% | miR-937-5p | 74% | miR-217-3p | 65% |
| miR-4315 | 60% | miR-939-3p | 71% | miR-320a-5p | 64% |
| miR-4316 | 57% | miR-1227-5p | 76% | miR-375-5p | 65% |
| miR-4314 | 61% | miR-1229-5p | 65% | miR-498-3p | 66% |
| miR-4318 | 54% | miR-1233-5p | 70% | miR-526a-3p | 73% |
| miR-4320 | 67% | miR-1236-5p | 59% | miR-9718 | 70% |
| miR-4322 | 70% | miR-1237-5p | 70% | miR-9898 | 73% |
| miR-4321 | 67% | miR-1238-5p | 72% | miR-9899 | 55% |
| miR-4323 | 68% | miR-1292-3p | 62% | miR-1843 | 54% |
| miR-4324 | 55% | miR-3617-3p | 56% | miR-10226 | 60% |
| miR-4256 | 74% | miR-3620-5p | 75% | miR-10392-5p | 74% |
| miR-4257 | 74% | miR-3934-3p | 64% | miR-10392-3p | 70% |
| miR-4258 | 74% | miR-4632-5p | 67% | miR-10394-3p | 62% |
| miR-4259 | 72% | miR-4743-3p | 63% | miR-10396a-5p | 71% |
| miR-4260 | 64% | miR-4750-3p | 72% | miR-10396a-3p | 73% |
| miR-4253 | 63% | miR-5739 | 71% | miR-10398-5p | 62% |
| miR-4251 | 66% | miR-5787 | 69% | miR-10398-3p | 76% |
| miR-4255 | 55% | miR-6068 | 64% | miR-10400-5p | 63% |
| miR-4325 | 73% | miR-6069 | 69% | miR-10400-3p | 76% |
| miR-4326 | 60% | miR-6071 | 75% | miR-10401-5p | 77% |
| miR-4327 | 75% | miR-6072 | 66% | miR-10401-3p | 71% |
| miR-4265 | 65% | miR-6074 | 58% | miR-10396b-5p | 74% |
| miR-2355-5p | 63% | miR-6075 | 69% | miR-10522-5p | 85% |
| miR-4268 | 60% | miR-6076 | 72% | miR-10524-5p | 57% |
| miR-4269 | 70% | miR-6077 | 57% | miR-10526-3p | 71% |
| miR-4264 | 53% | miR-6078 | 58% | miR-10527-5p | 68% |
| miR-4271 | 67% | miR-6081 | 58% | miR-11181-5p | 65% |
| miR-4276 | 64% | miR-6082 | 64% | miR-11181-3p | 73% |
| miR-4274 | 70% | miR-6083 | 52% | miR-11399 | 76% |
| miR-4281 | 64% | miR-6085 | 71% | miR-11400 | 61% |
| miR-4279 | 67% | miR-6086 | 74% | miR-3059-5p | 69% |
| miR-4278 | 50% | miR-6088 | 62% | miR-3059-3p | 54% |
| miR-4280 | 71% | miR-6089 | 71% | miR-3085-5p | 69% |
| miR-4285 | 68% | miR-6090 | 69% | miR-3085-3p | 69% |
| miR-4284 | 67% | miR-6124 | 74% | miR-6529-5p | 57% |
| miR-4286 | 71% | miR-6125 | 73% | miR-12114 | 73% |
| miR-4287 | 59% | miR-6126 | 68% | miR-12115 | 63% |
| miR-4292 | 67% | miR-6127 | 81% | miR-12116 | 72% |
| miR-4289 | 63% | miR-378j | 63% | miR-12118 | 77% |
| miR-4290 | 62% | miR-6129 | 54% | miR-12119 | 77% |
| miR-4329 | 66% | miR-6130 | 61% | miR-12120 | 72% |
| miR-500b-5p | 64% | miR-6131 | 81% | miR-12121 | 67% |
| miR-3200-5p | 63% | miR-6132 | 73% | miR-12122 | 52% |
| miR-3605-3p | 63% | miR-6133 | 72% | miR-12124 | 60% |
| miR-3610 | 71% | miR-6165 | 71% | miR-12126 | 64% |
| miR-3612 | 72% | miR-548ay-3p | 83% | miR-12127 | 52% |
| miR-3614-5p | 69% | miR-6500-5p | 63% | miR-12128 | 53% |
| miR-3614-3p | 75% | miR-6501-5p | 51% | miR-12131 | 100% |
| miR-3616-3p | 61% | miR-6501-3p | 65% | miR-12136 | 55% |
| miR-3619-5p | 51% | miR-6503-5p | 62% | | |

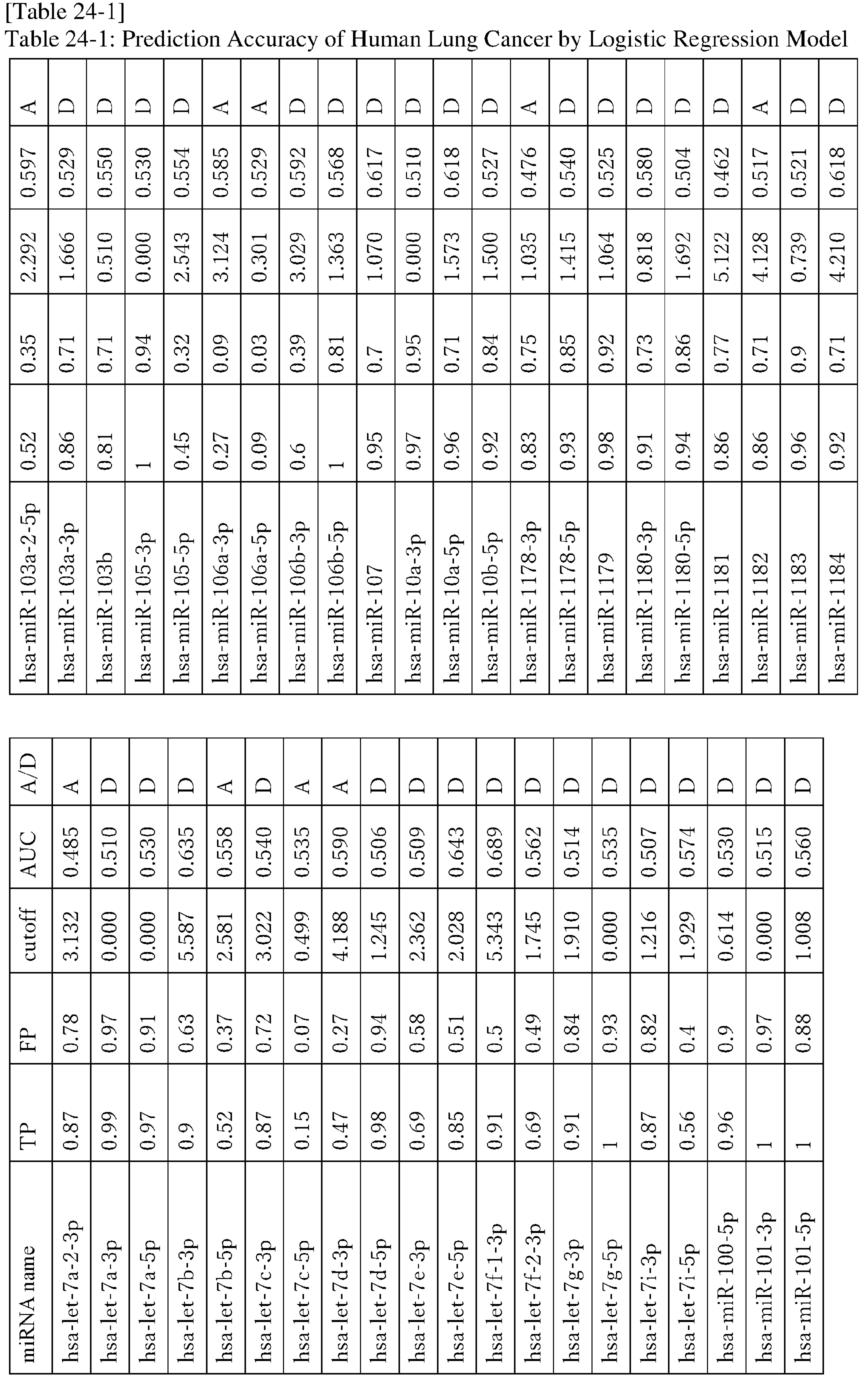

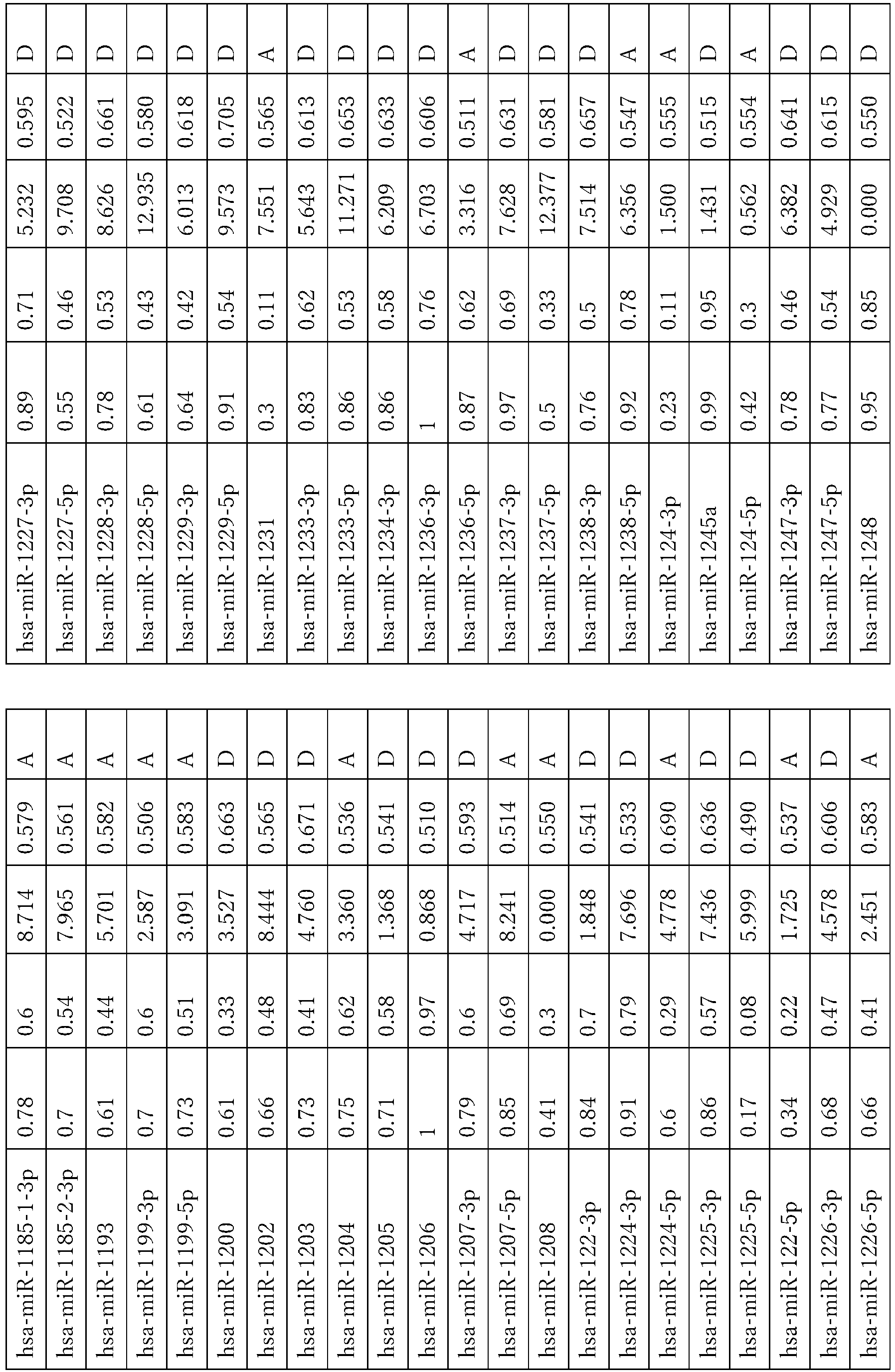

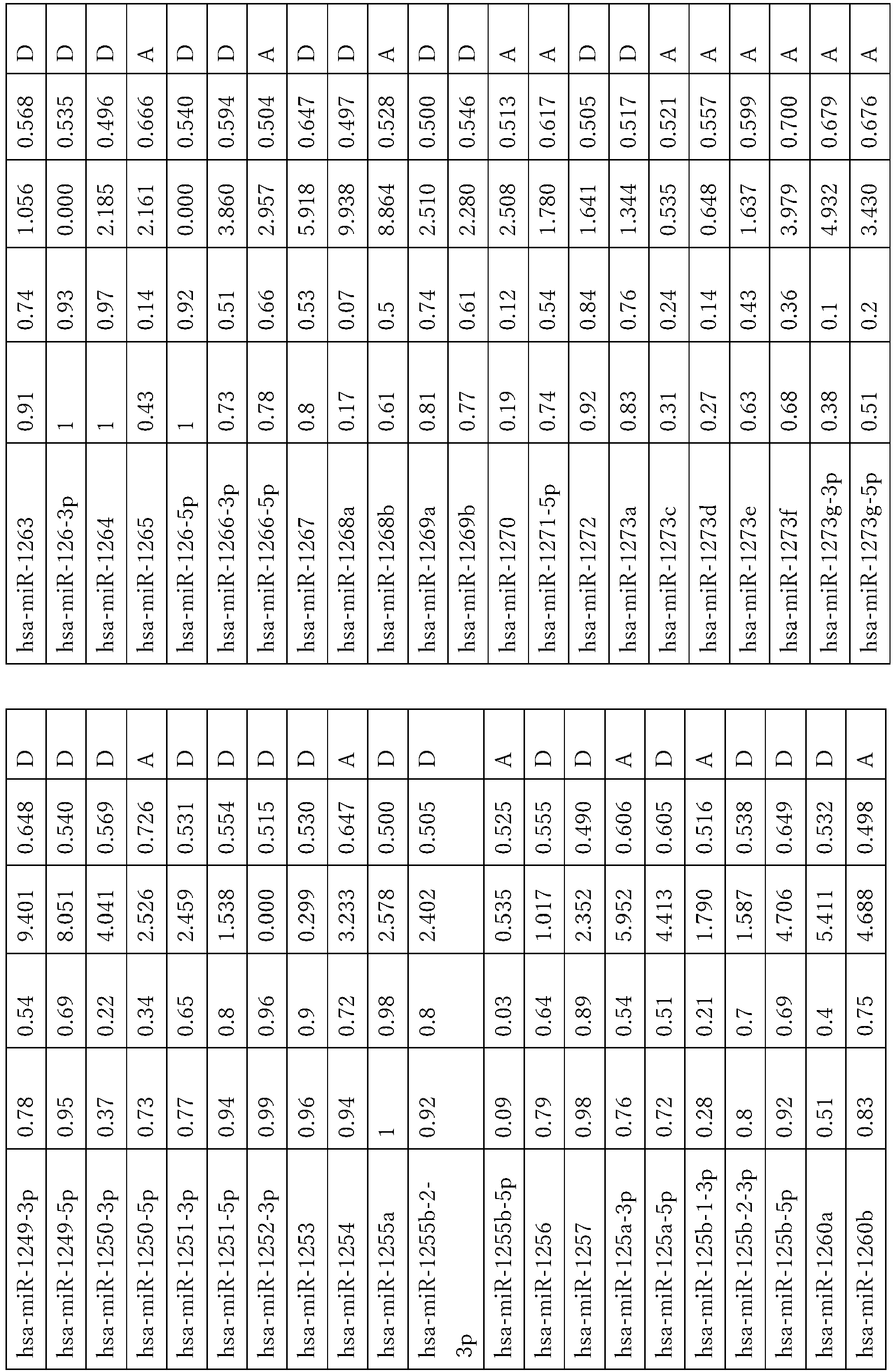

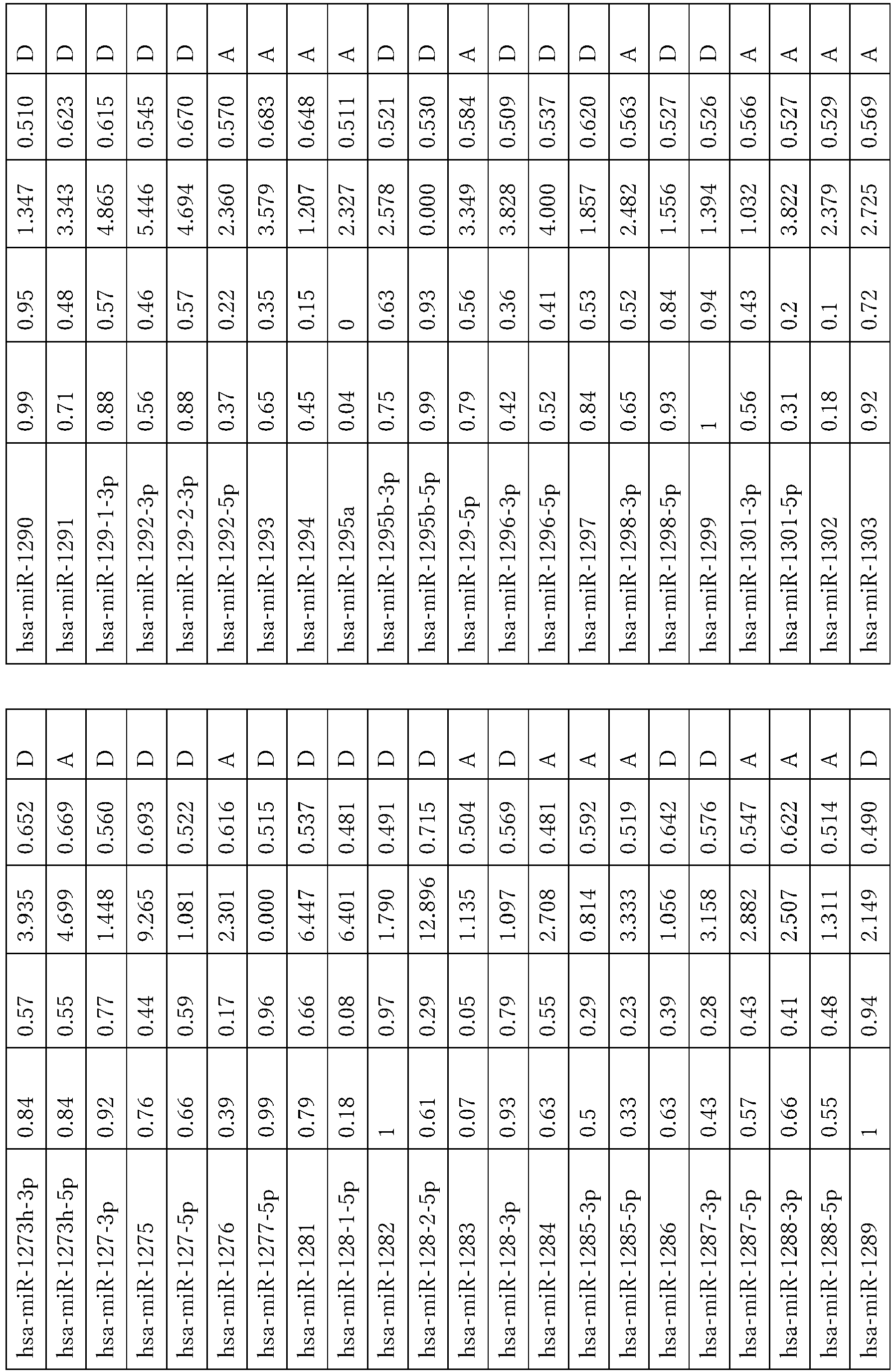

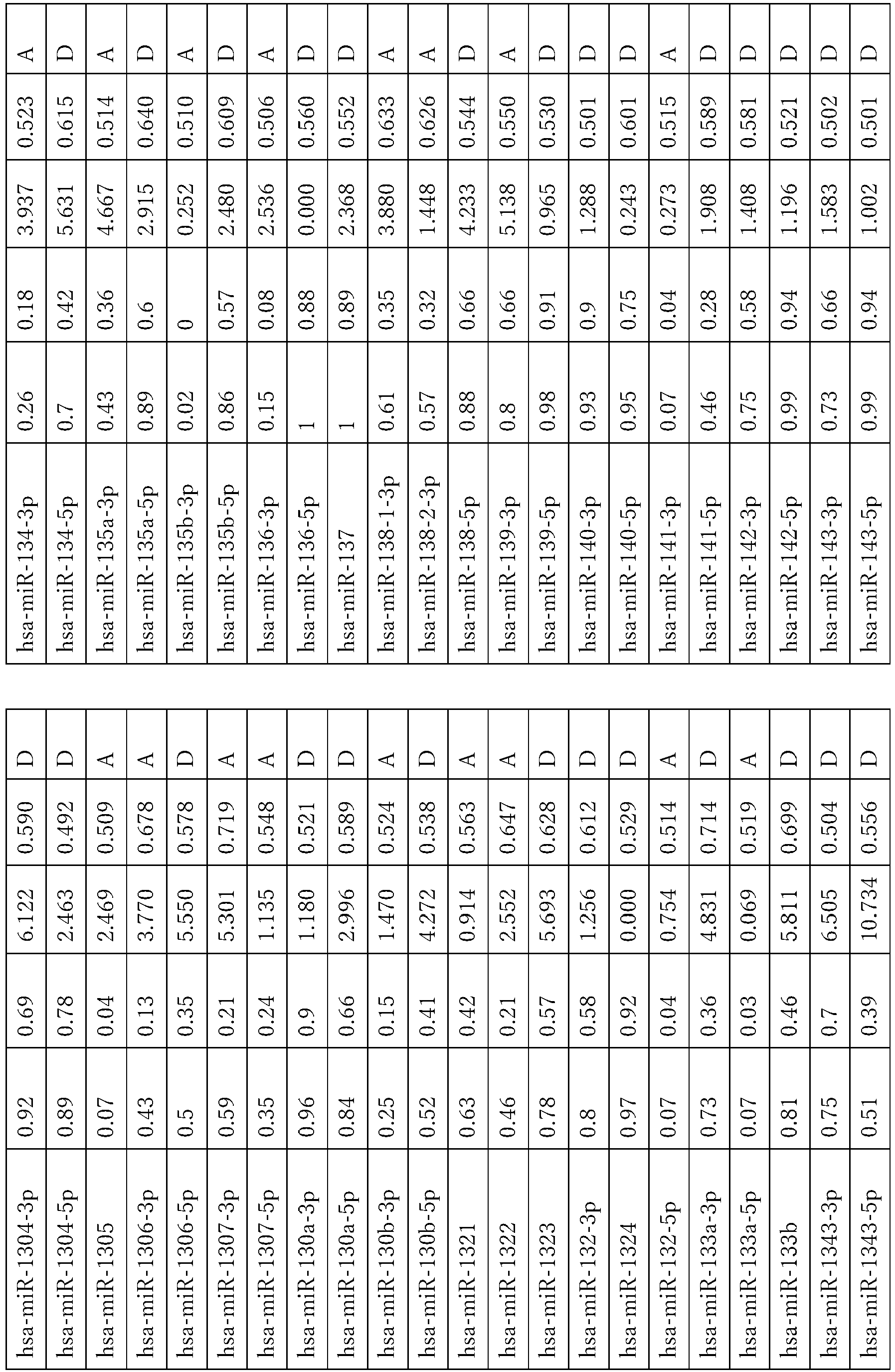

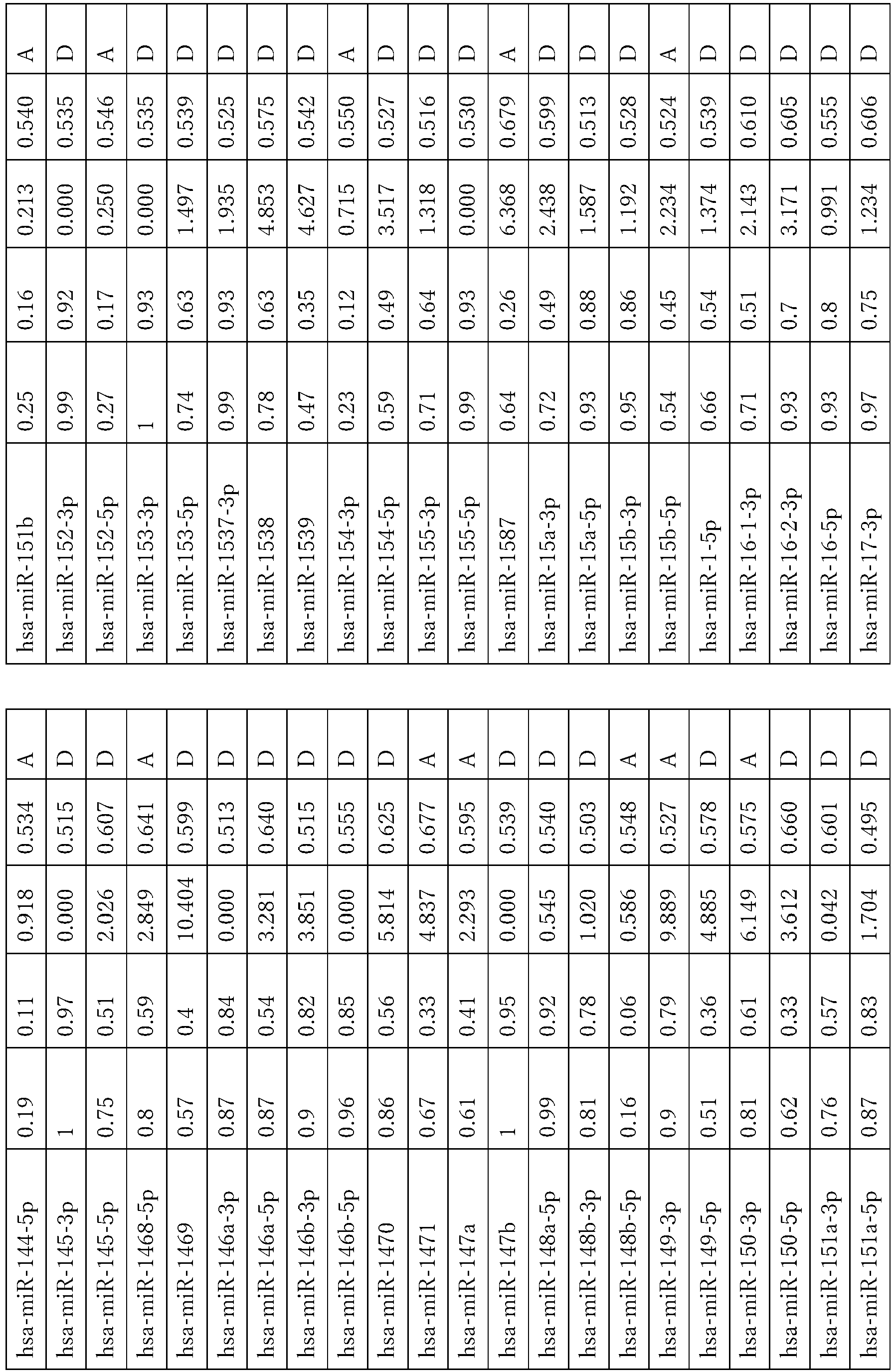

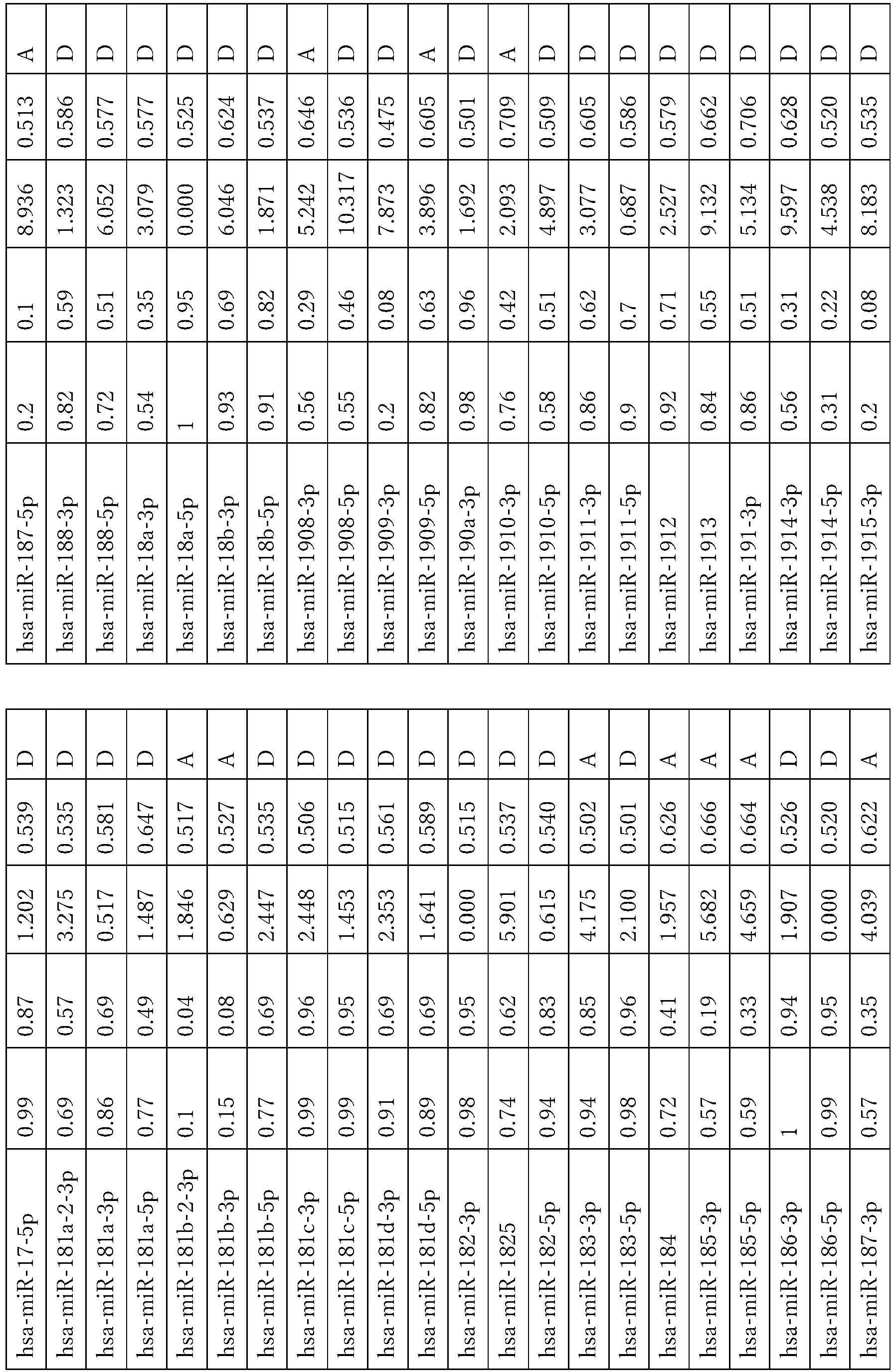

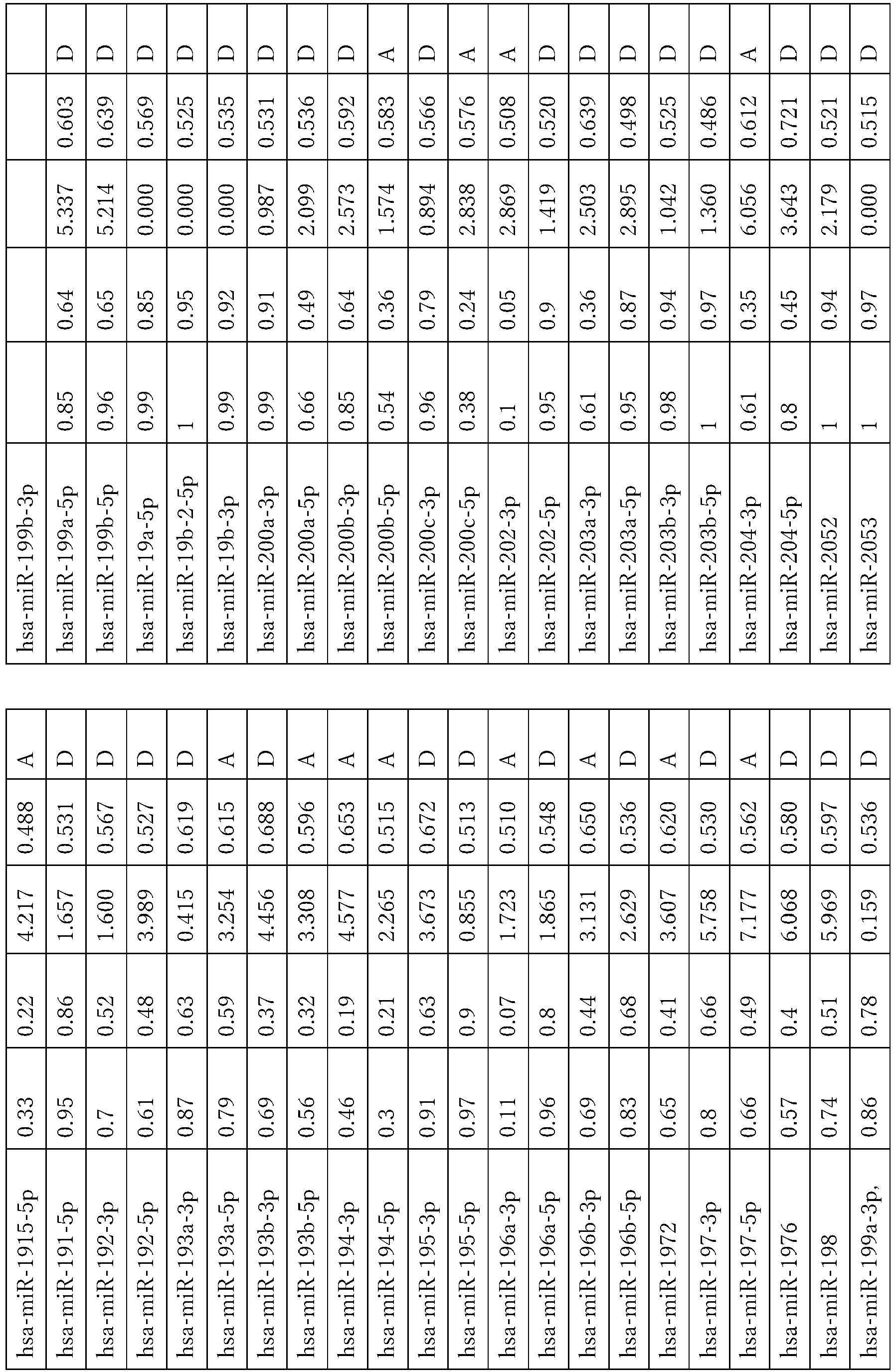

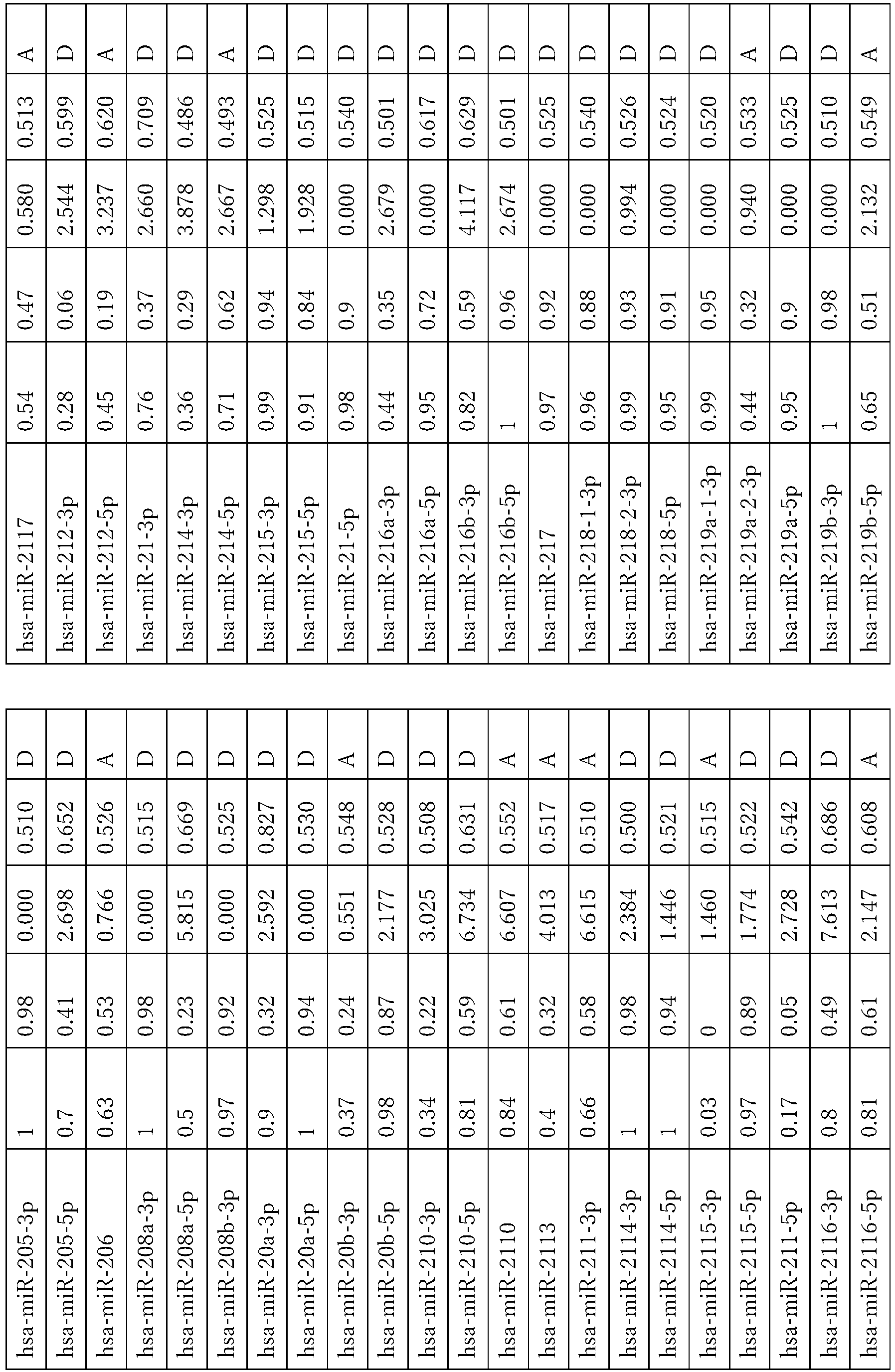

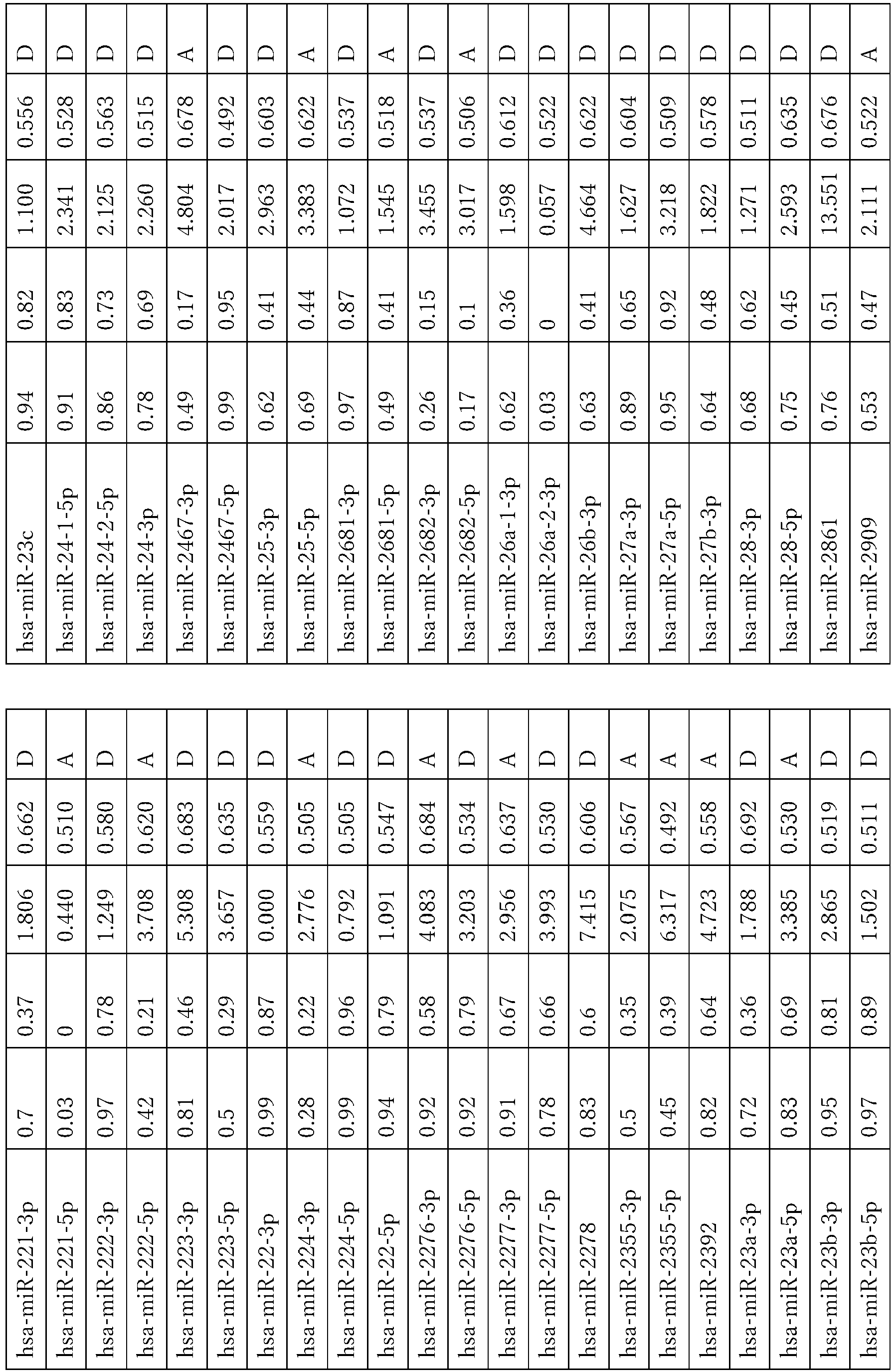

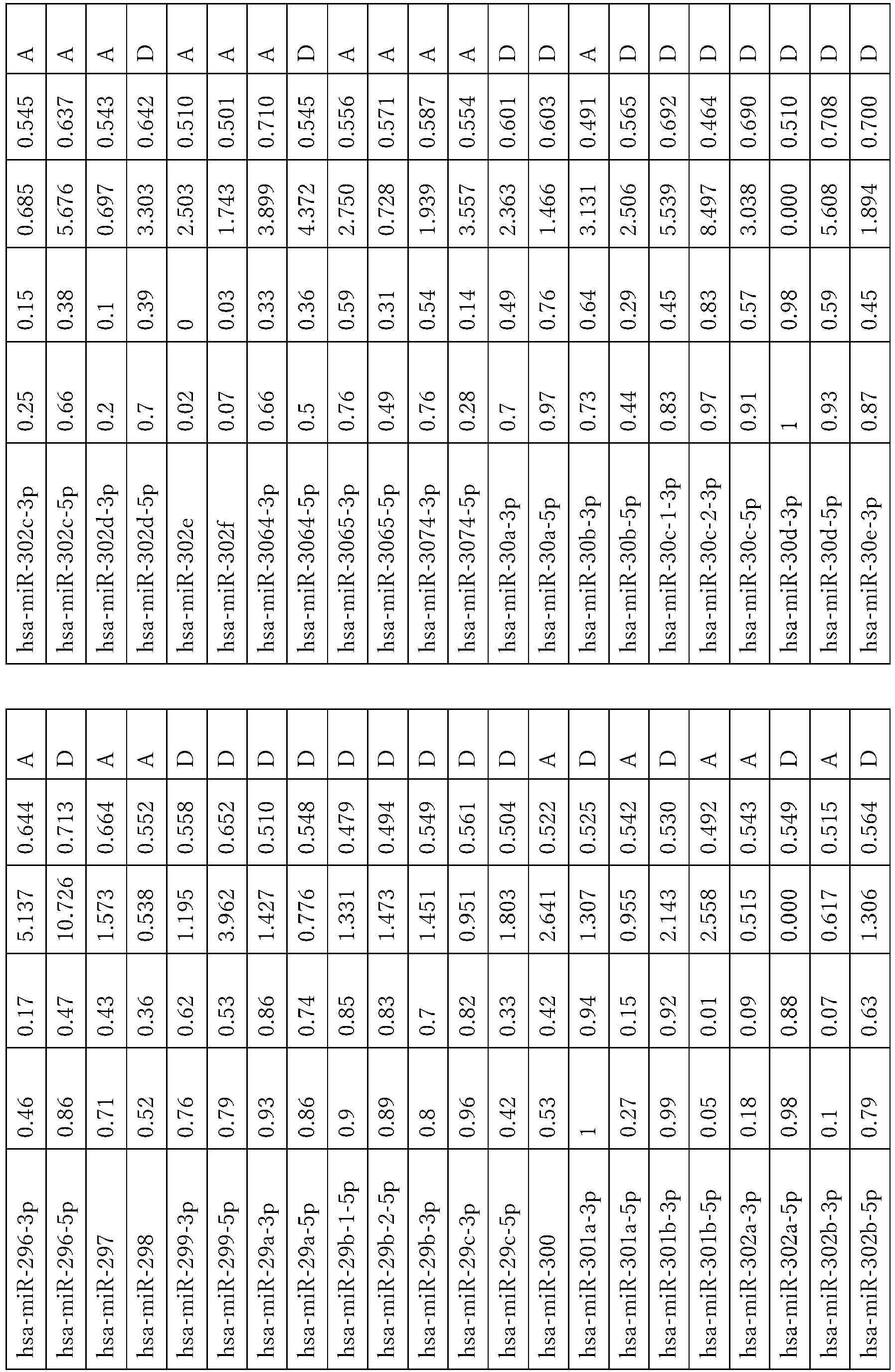

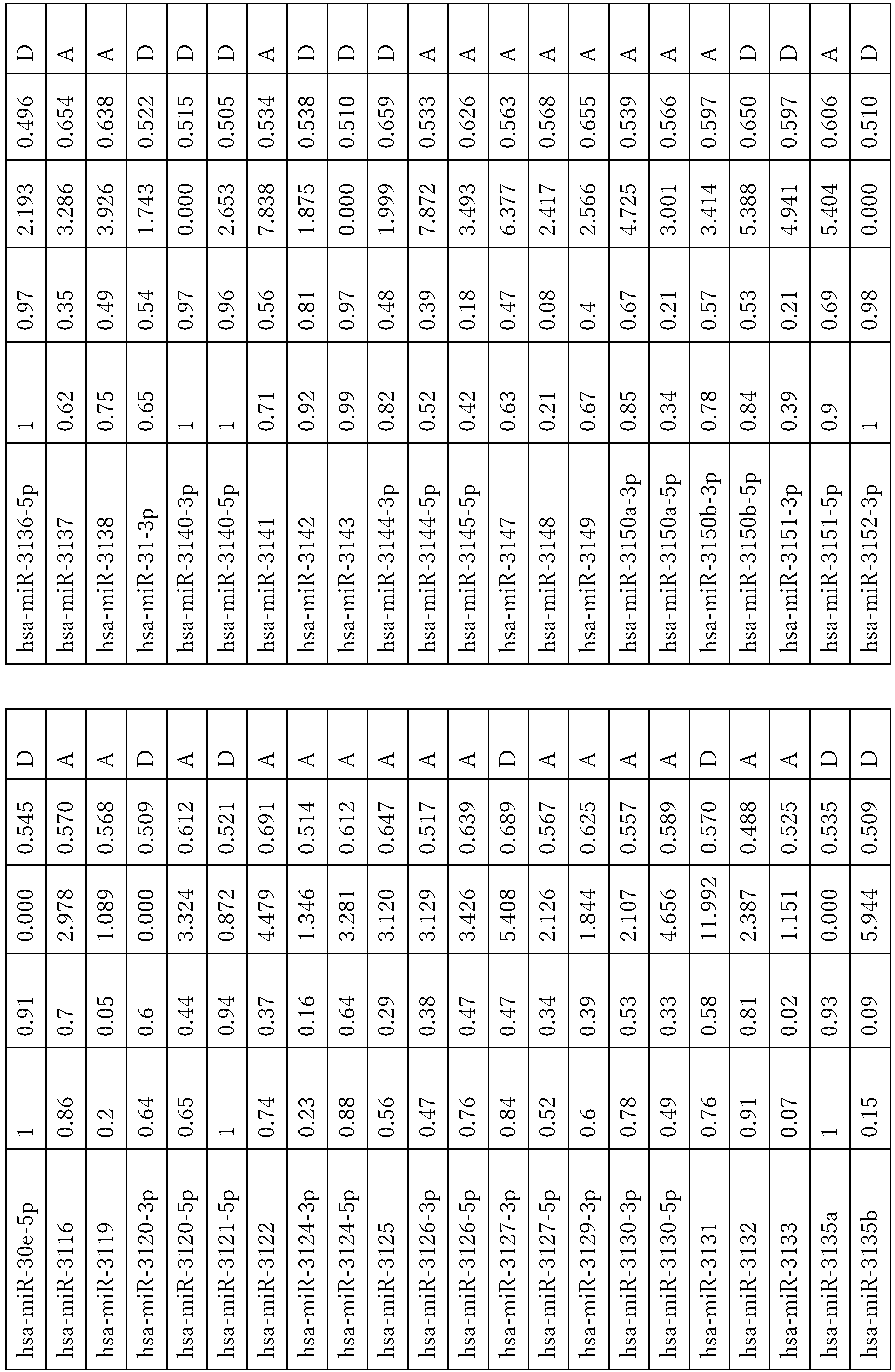

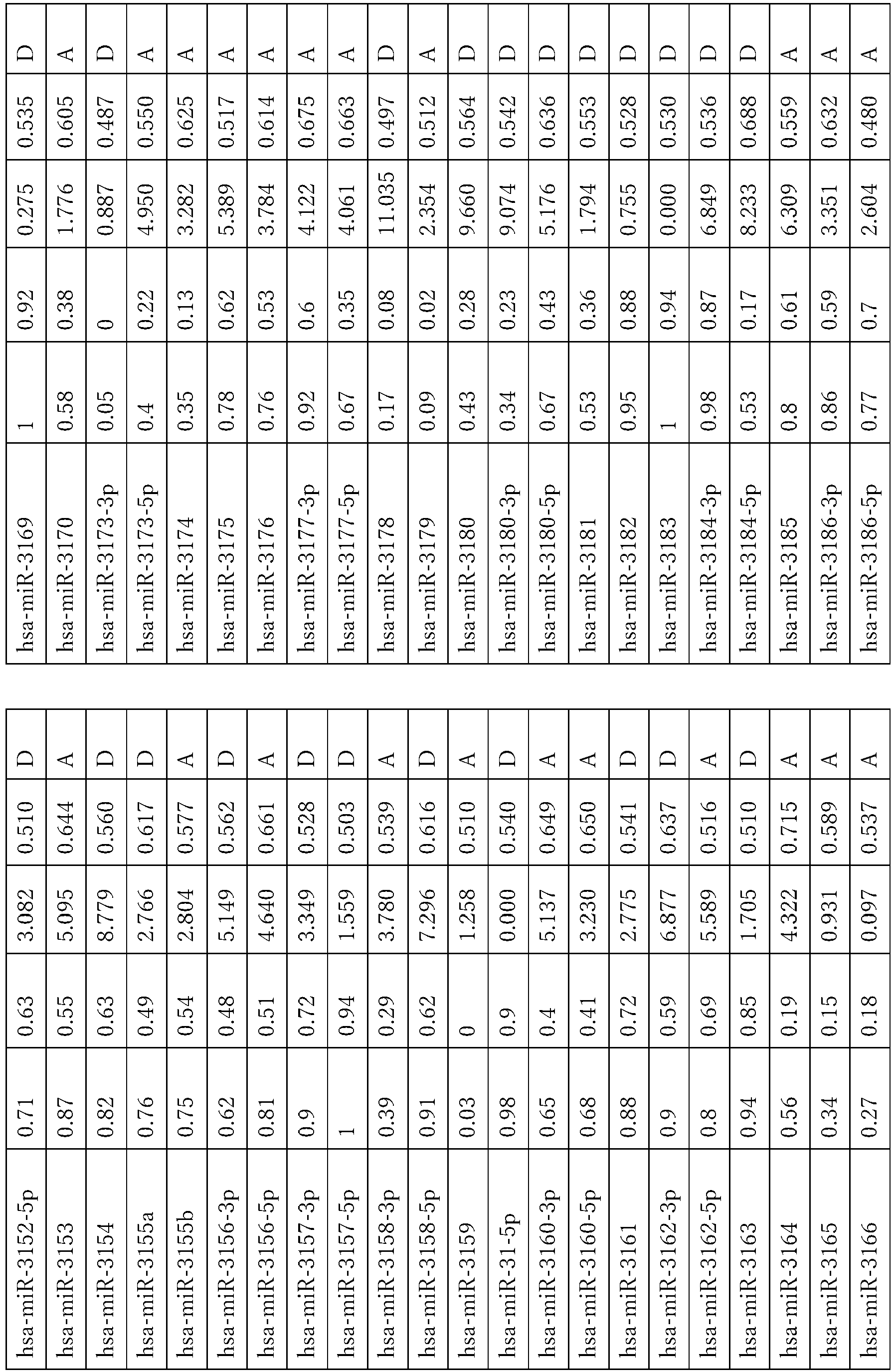

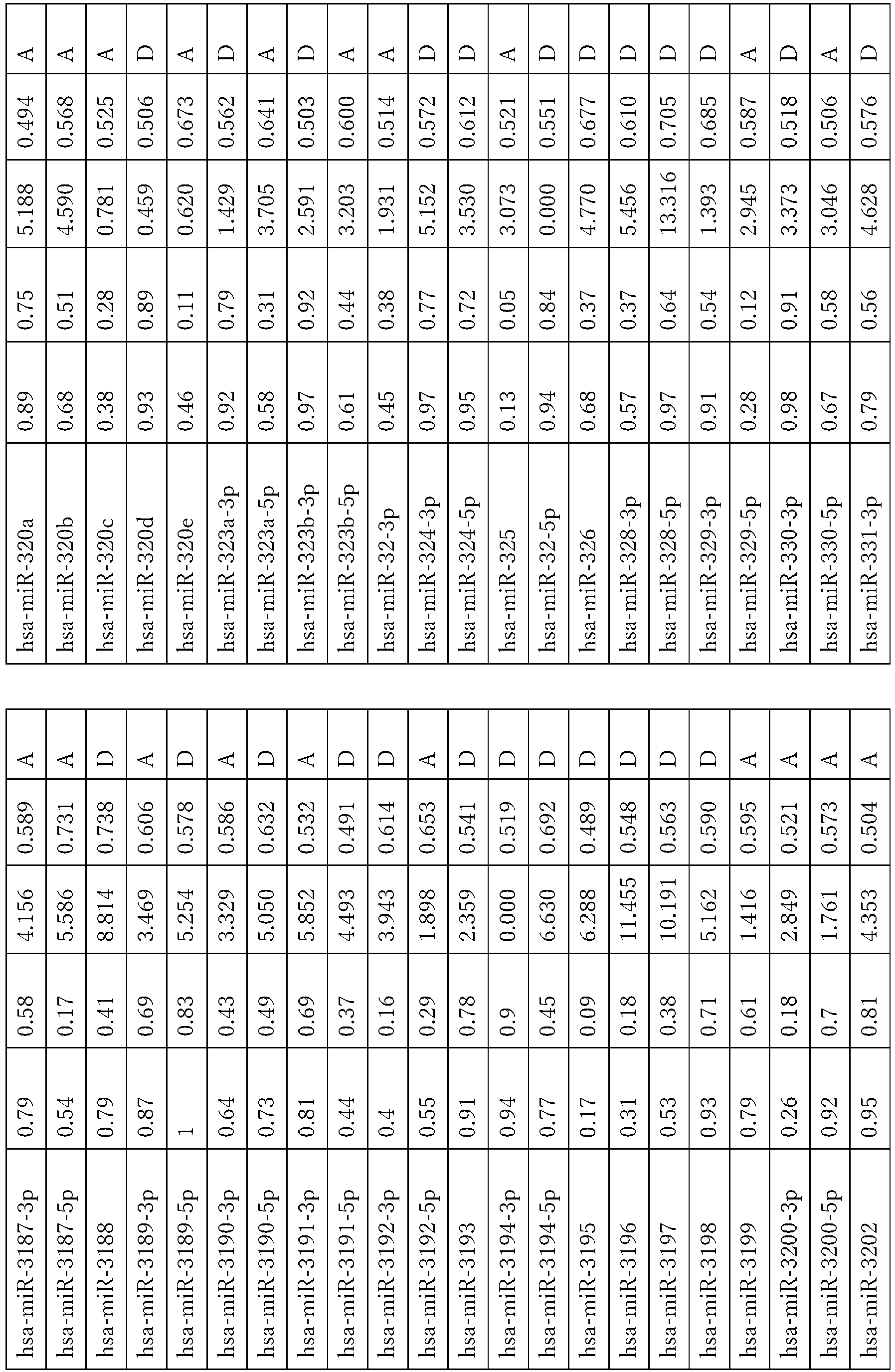

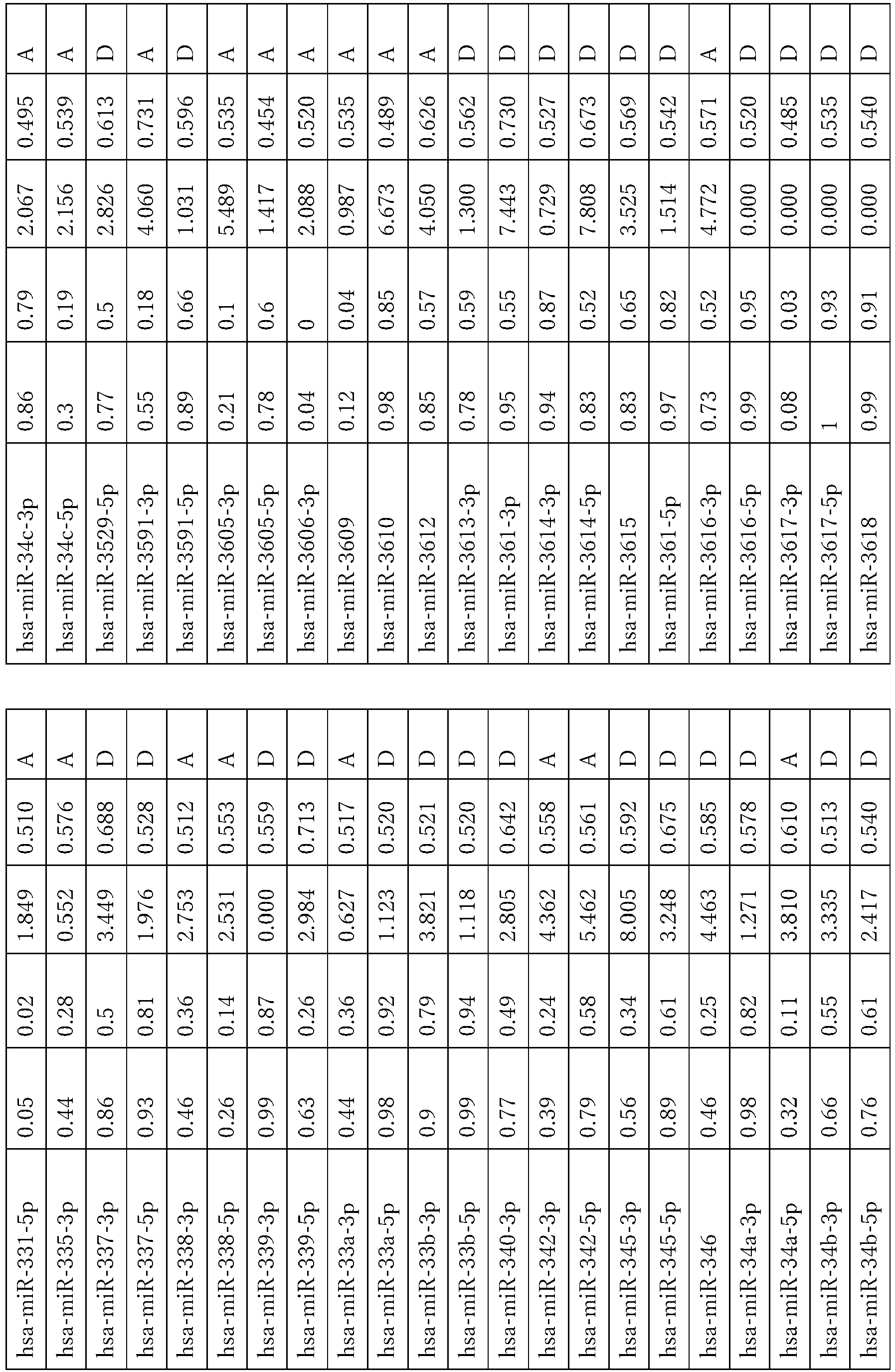

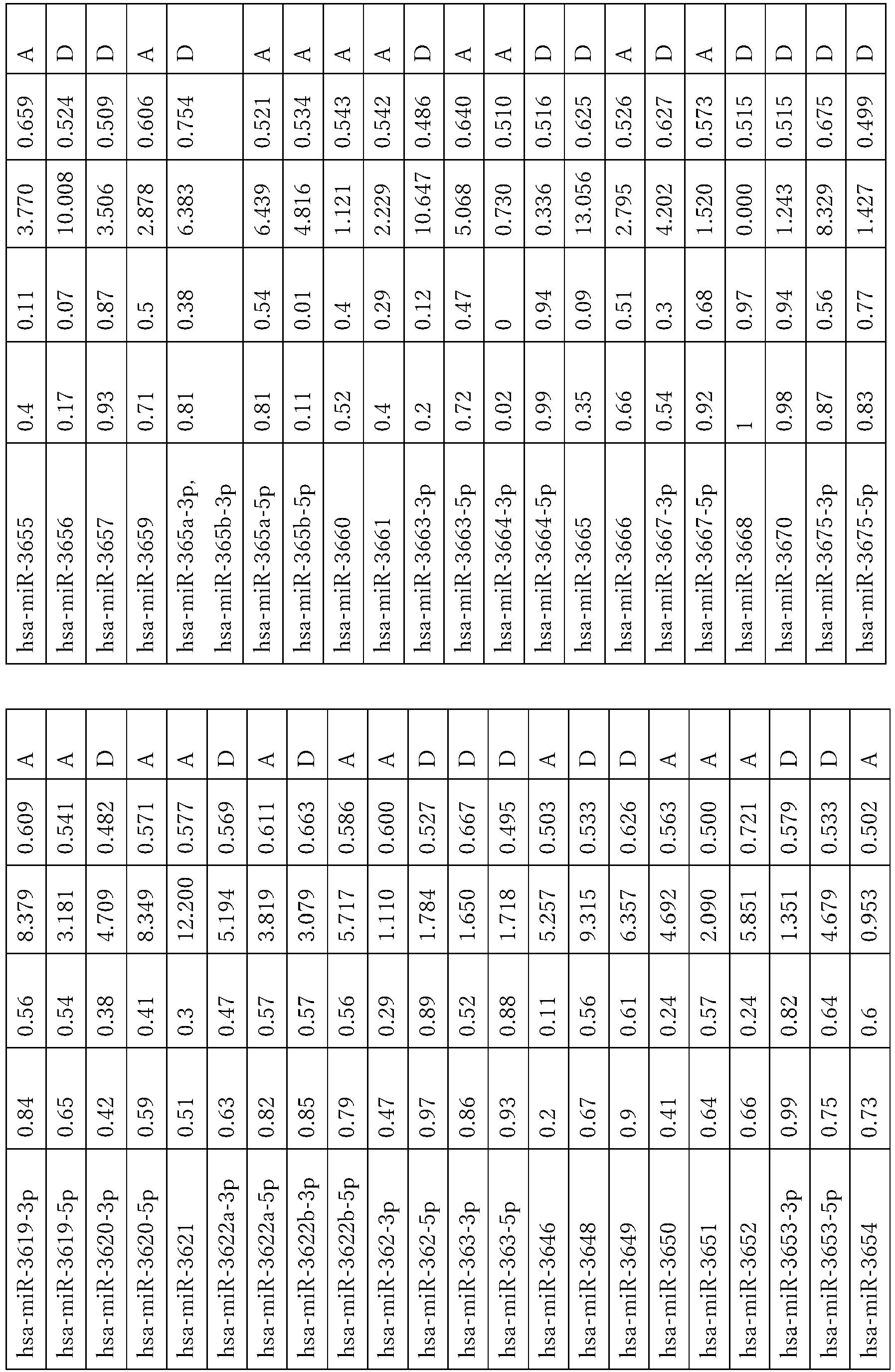

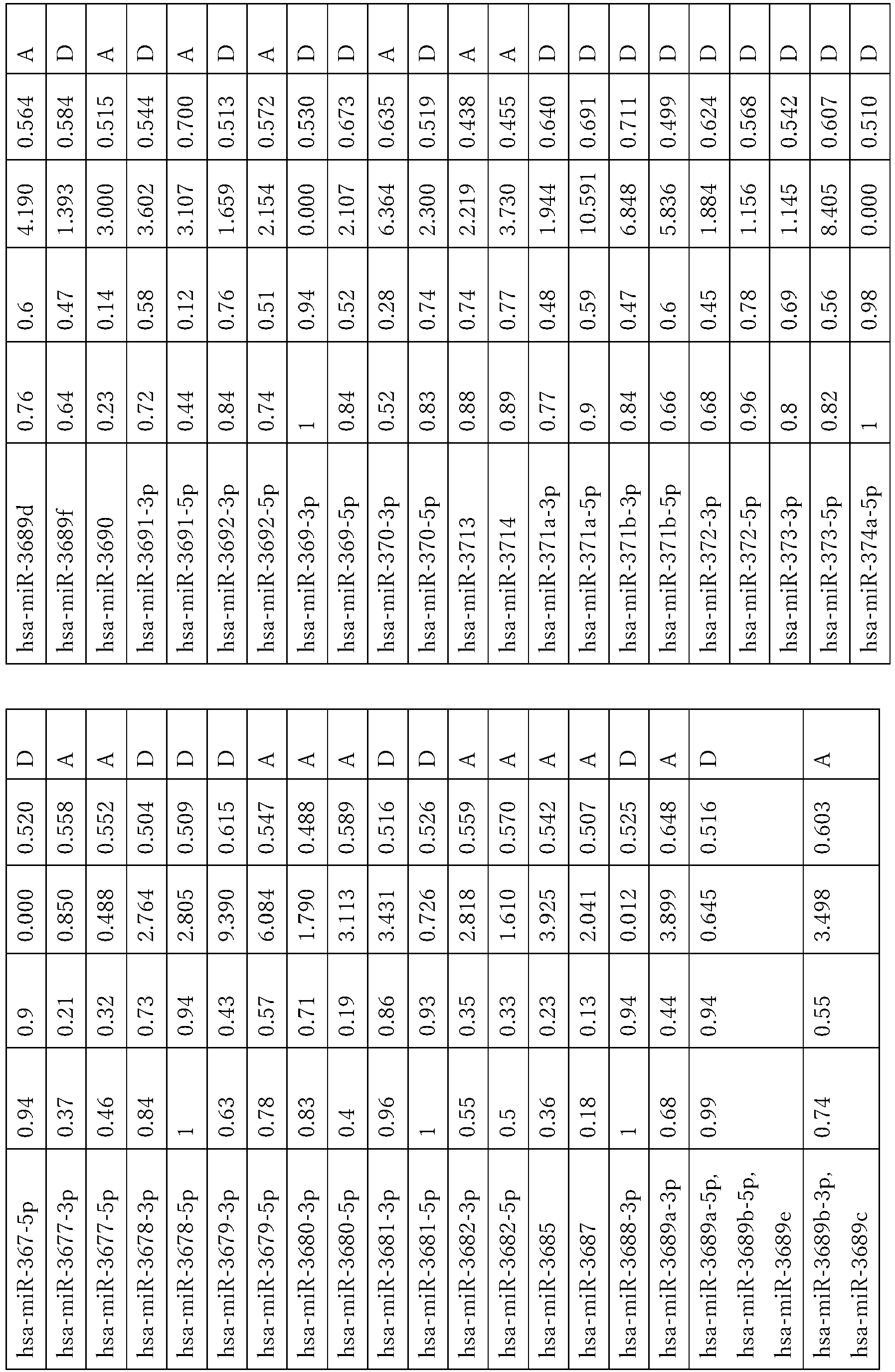

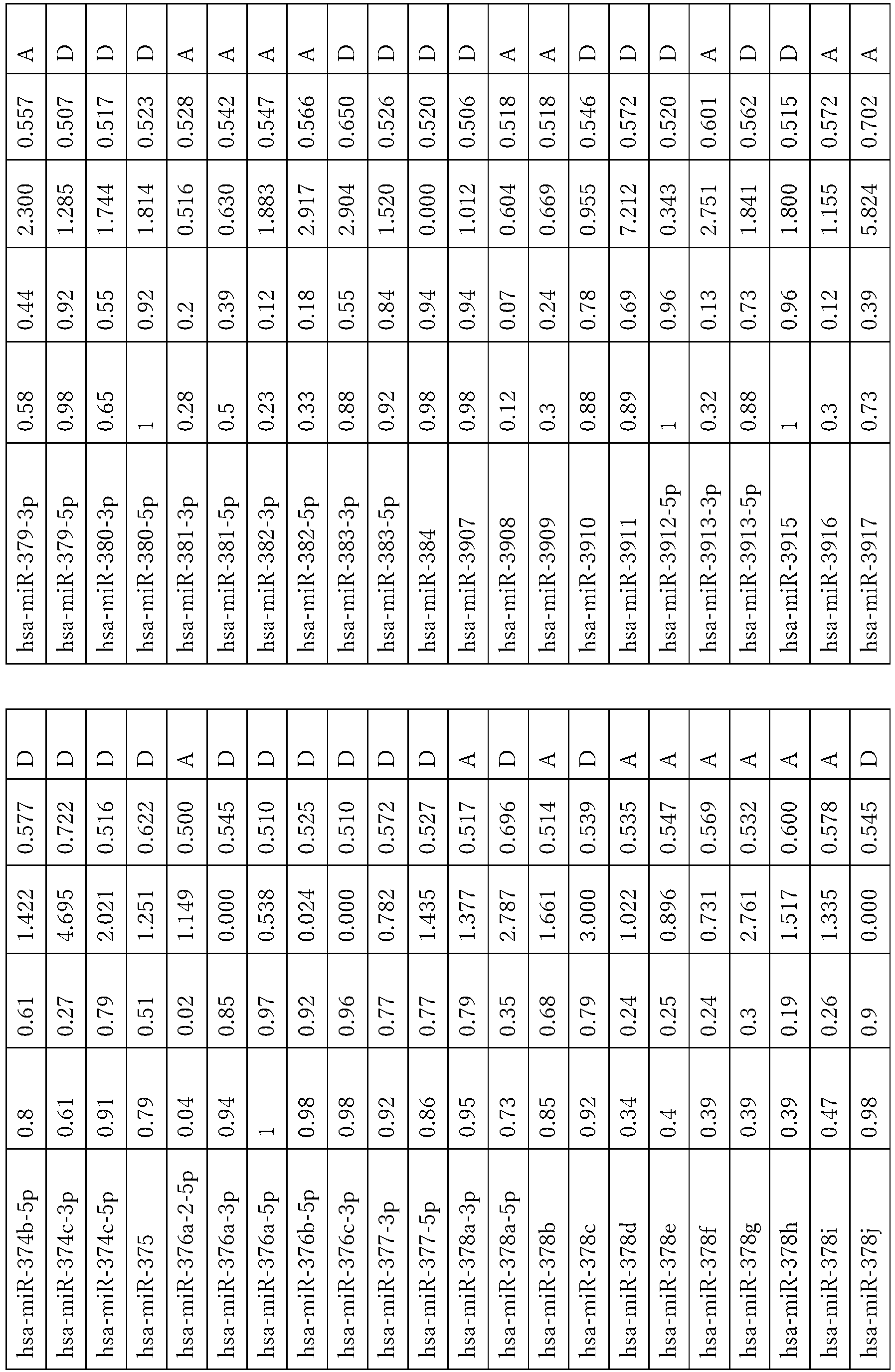

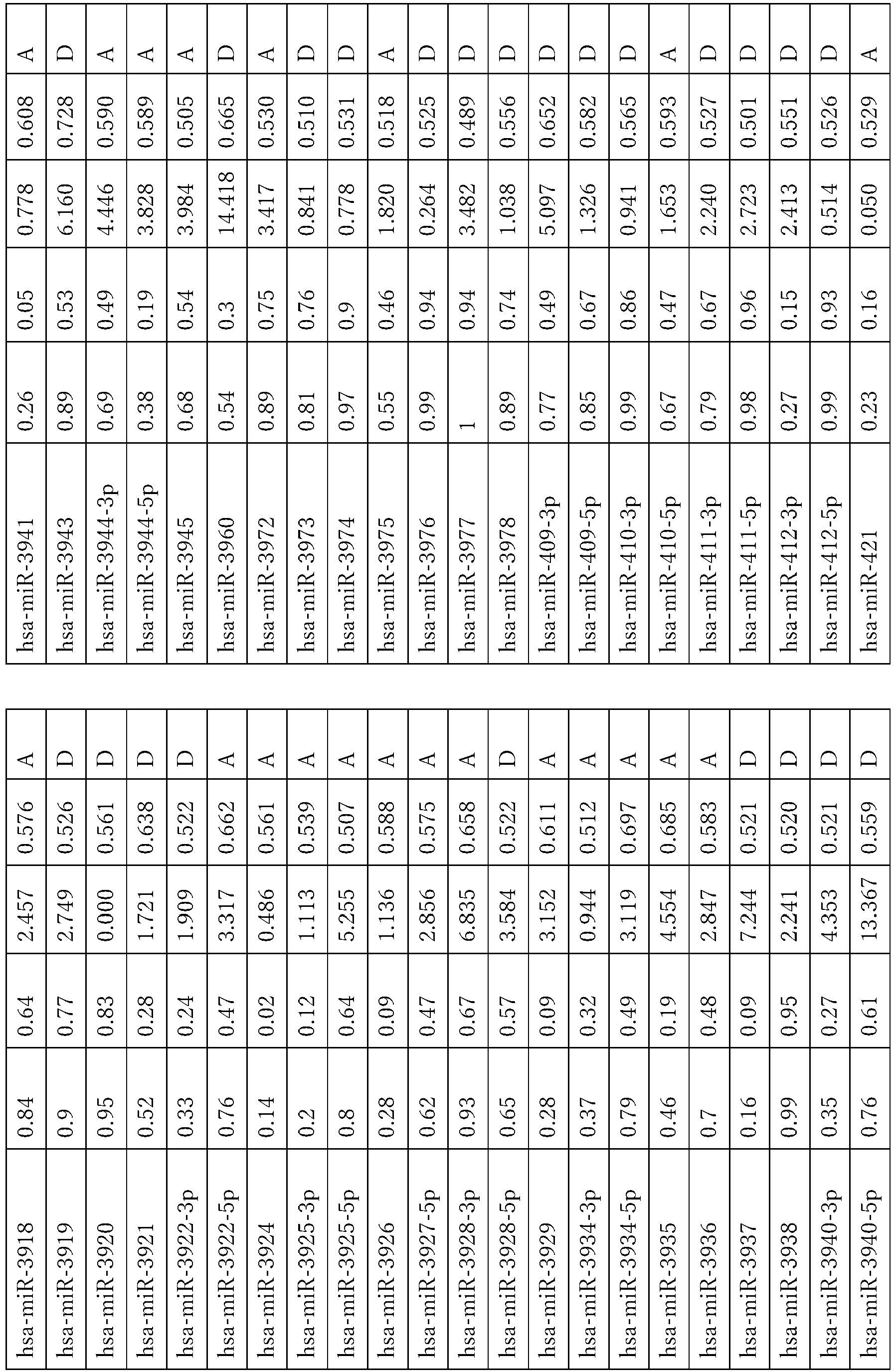

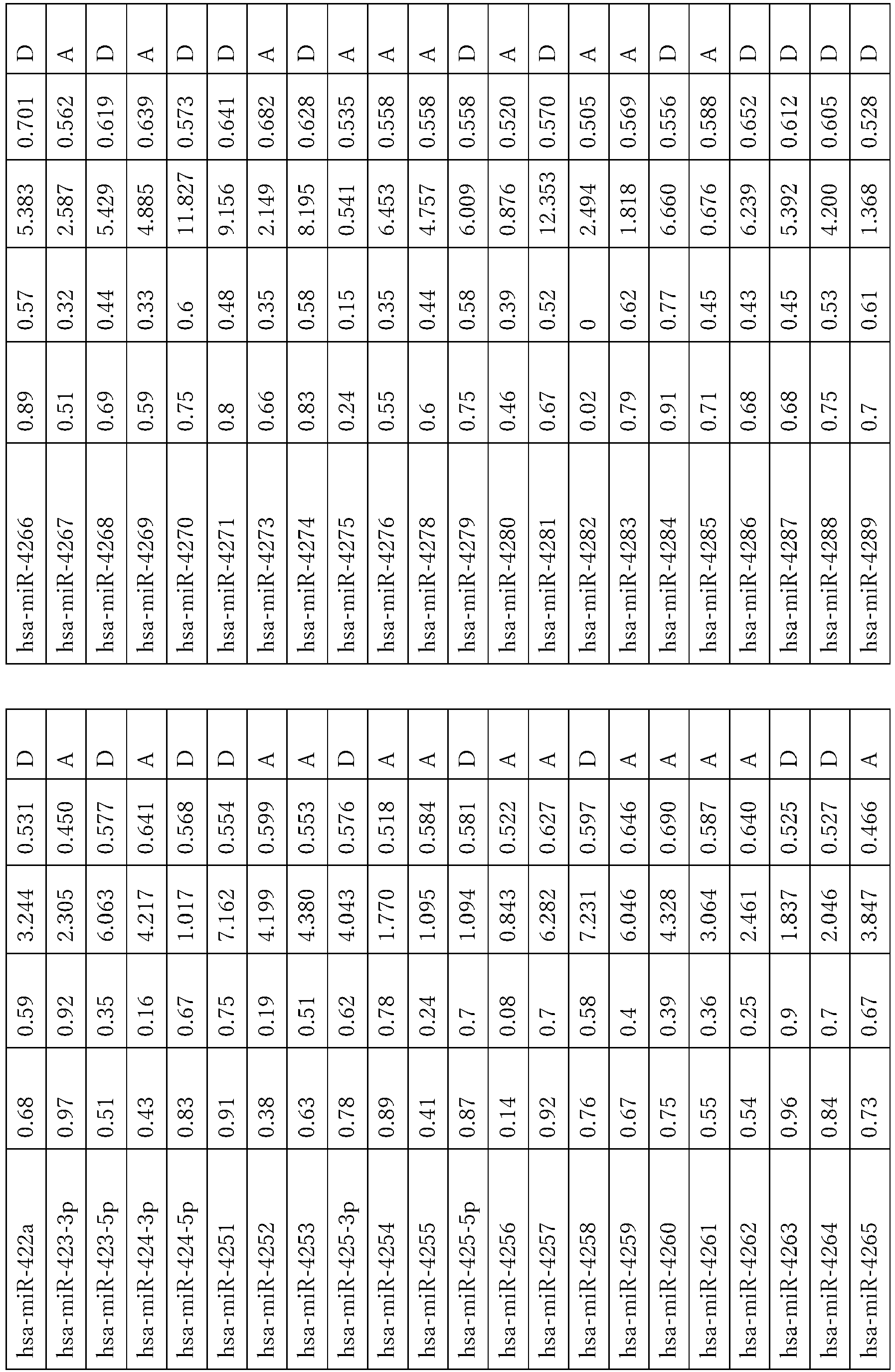

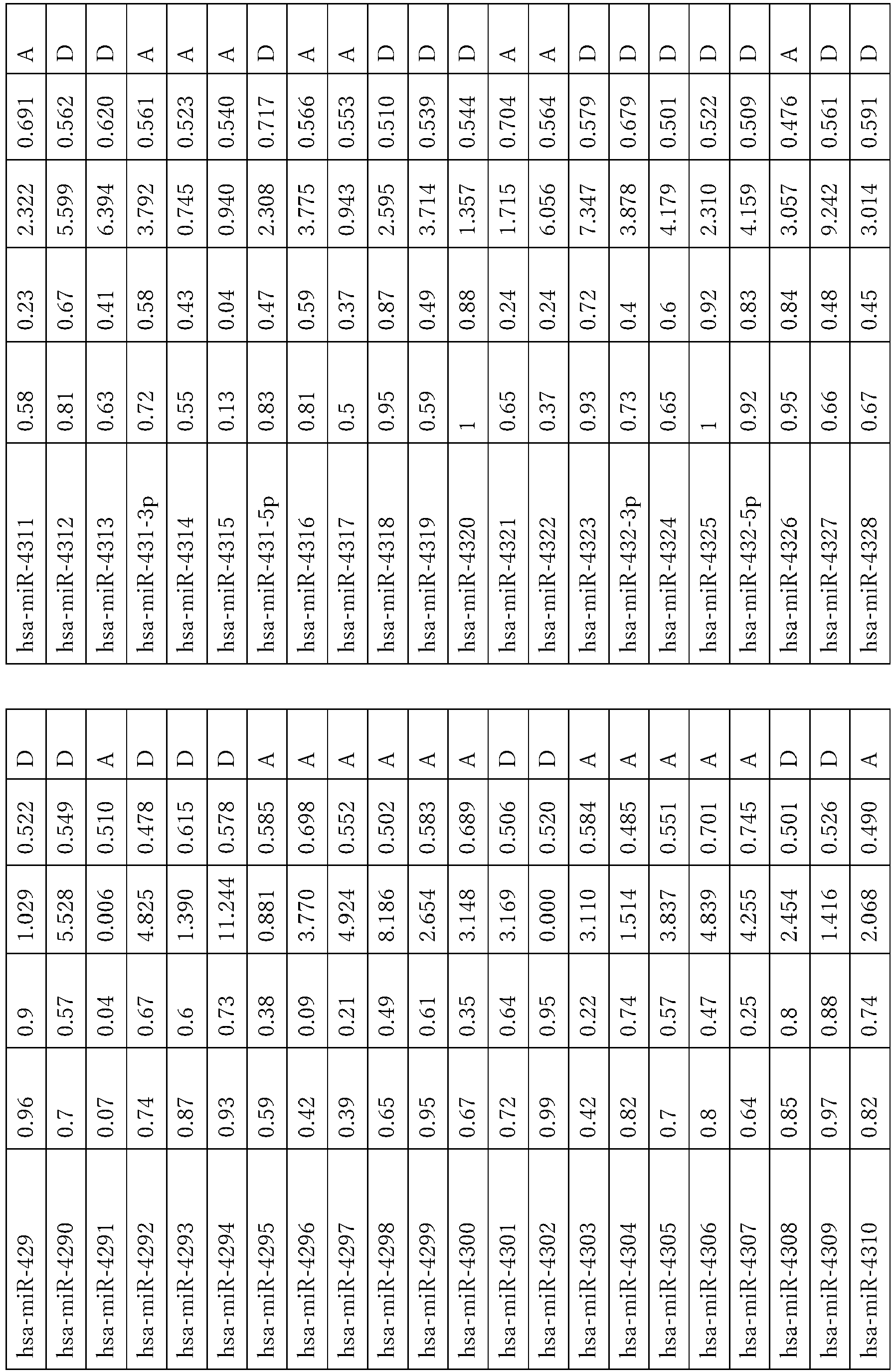

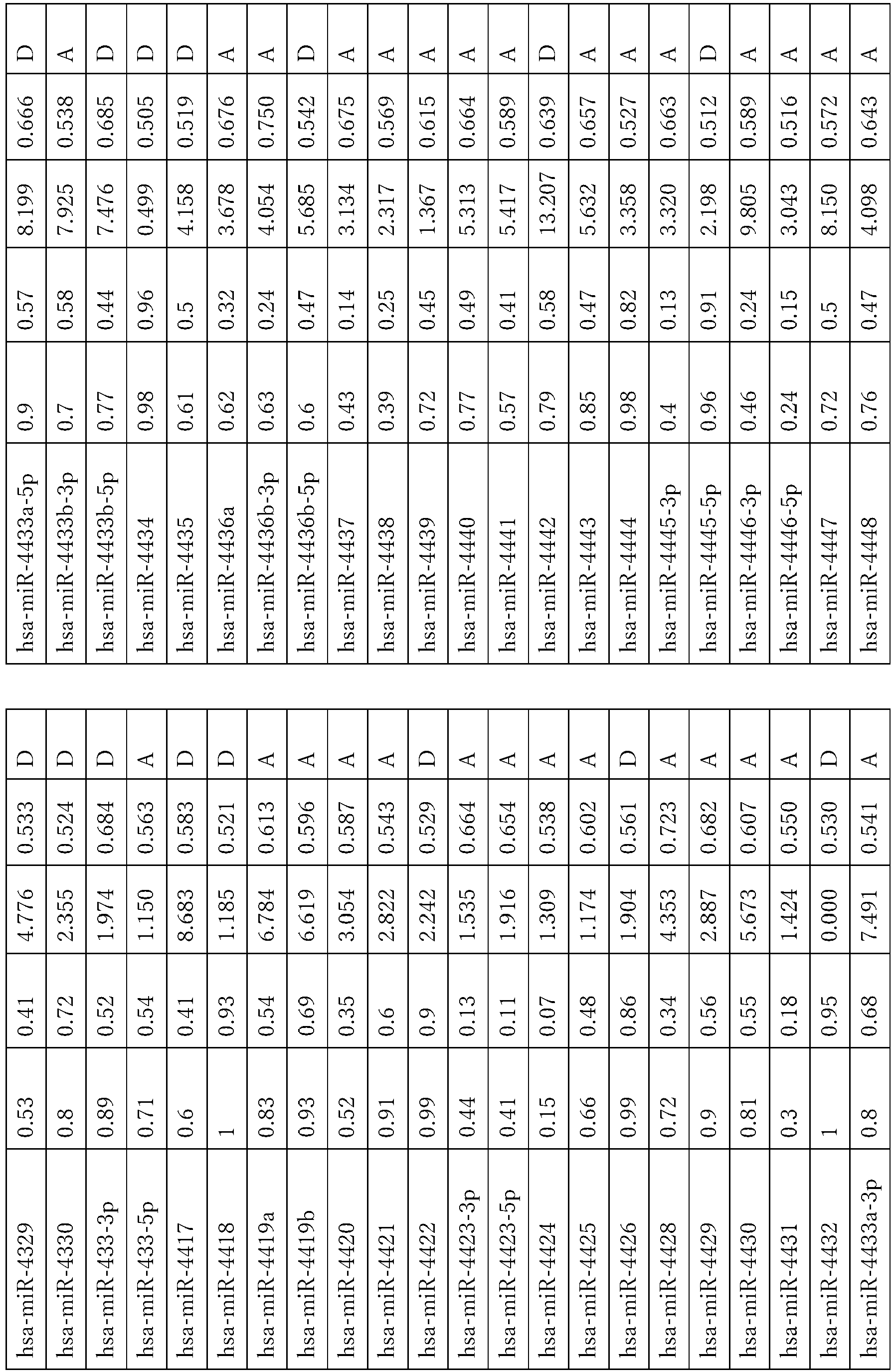

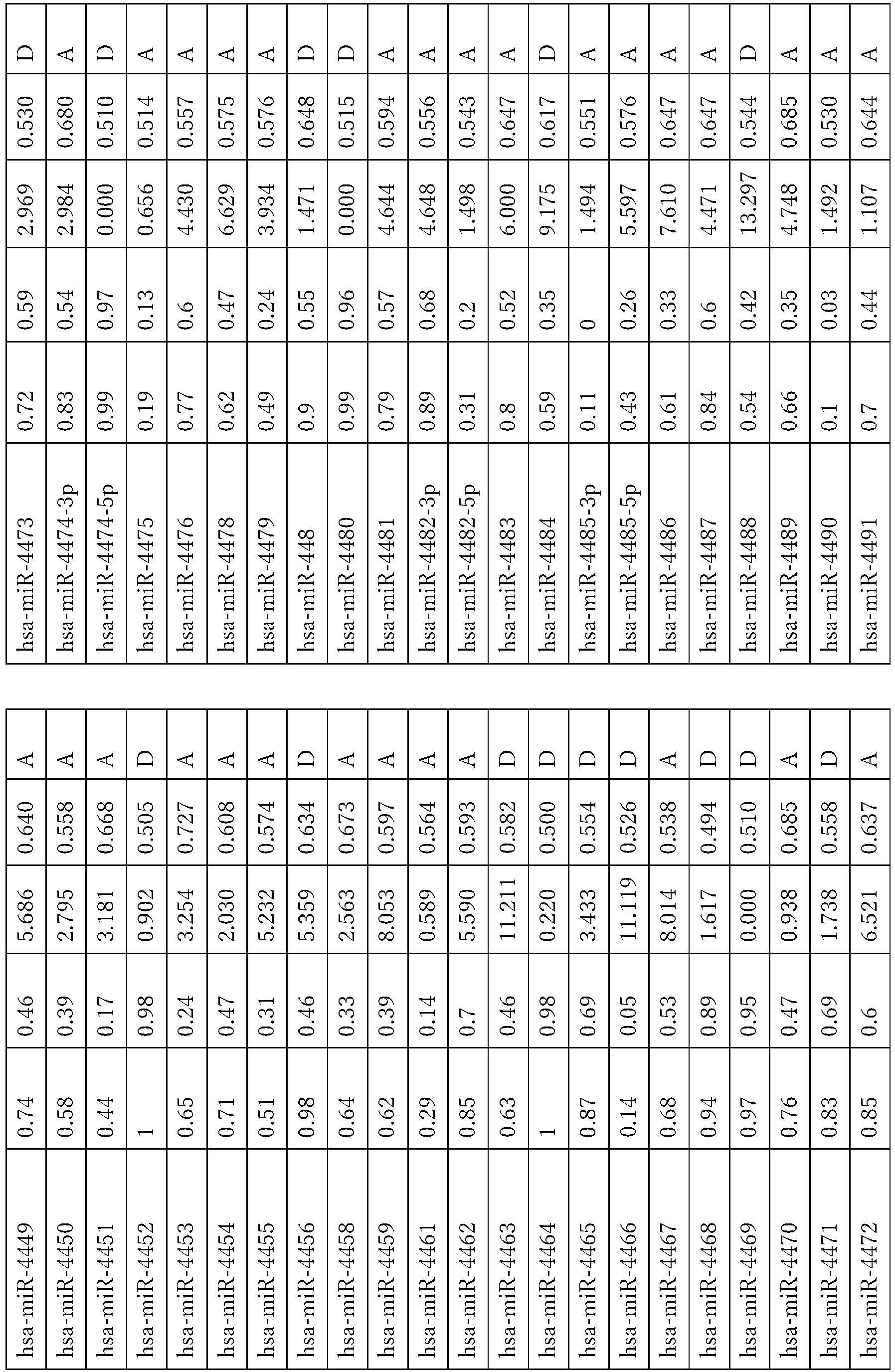

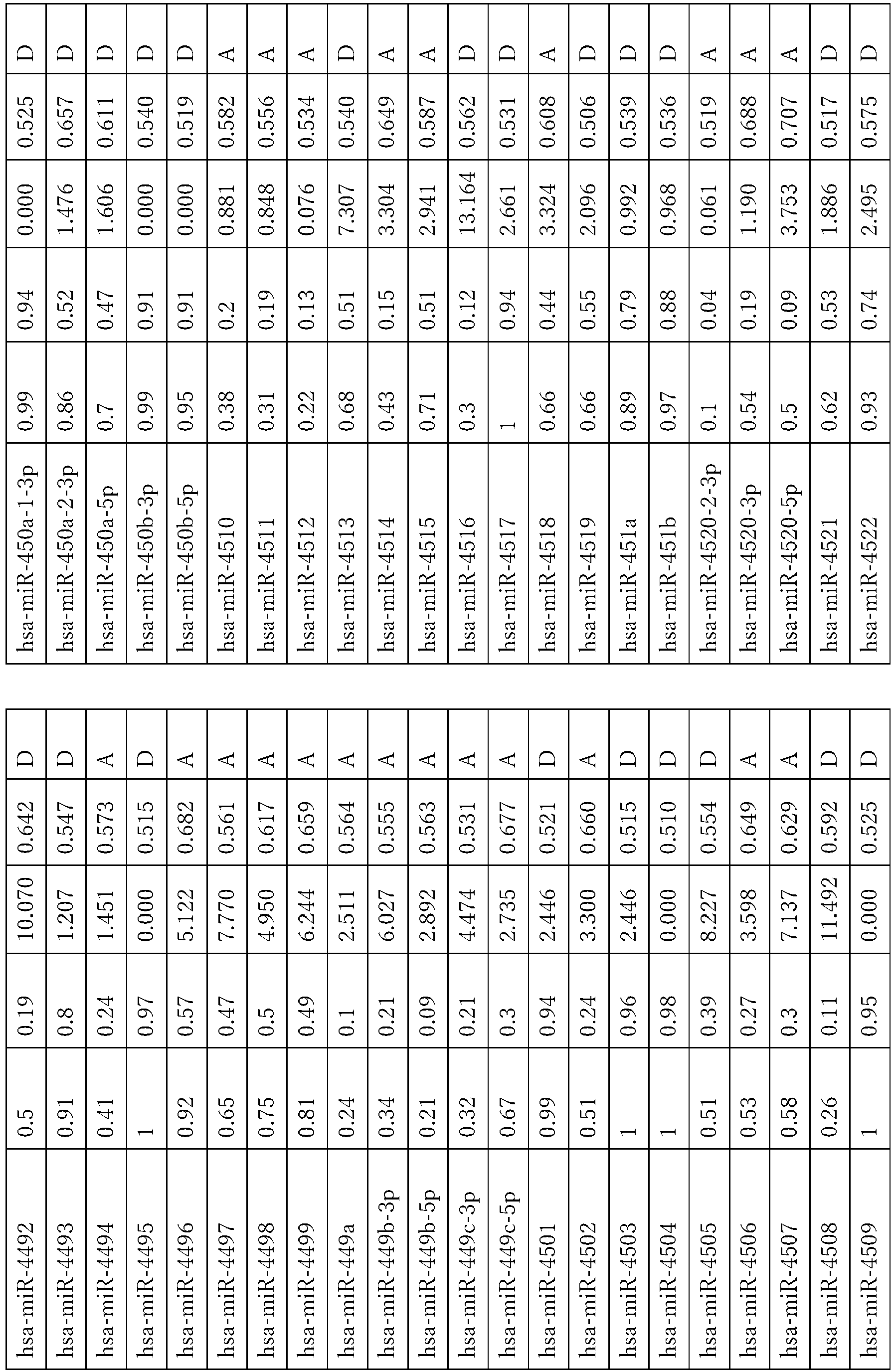

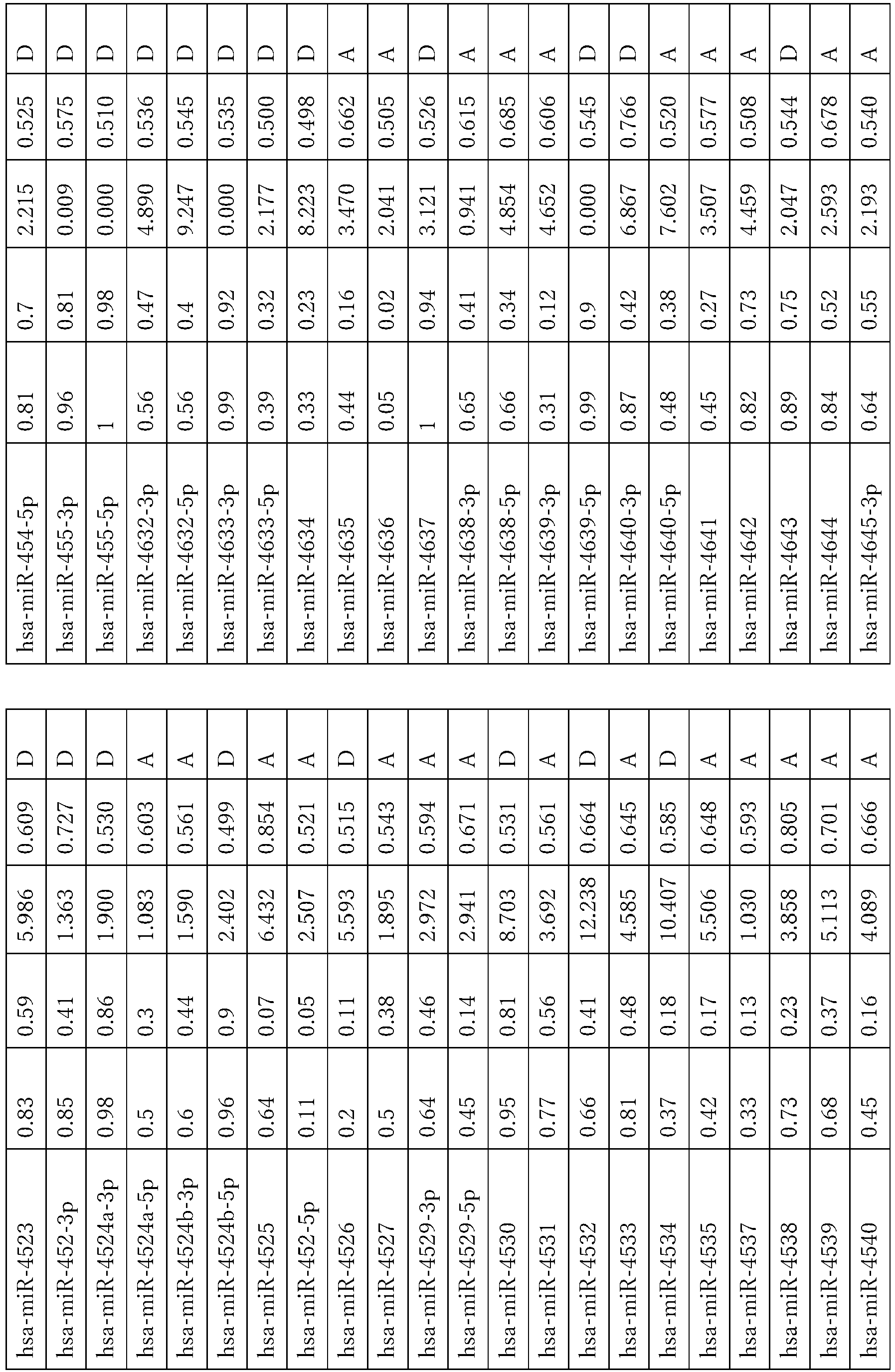

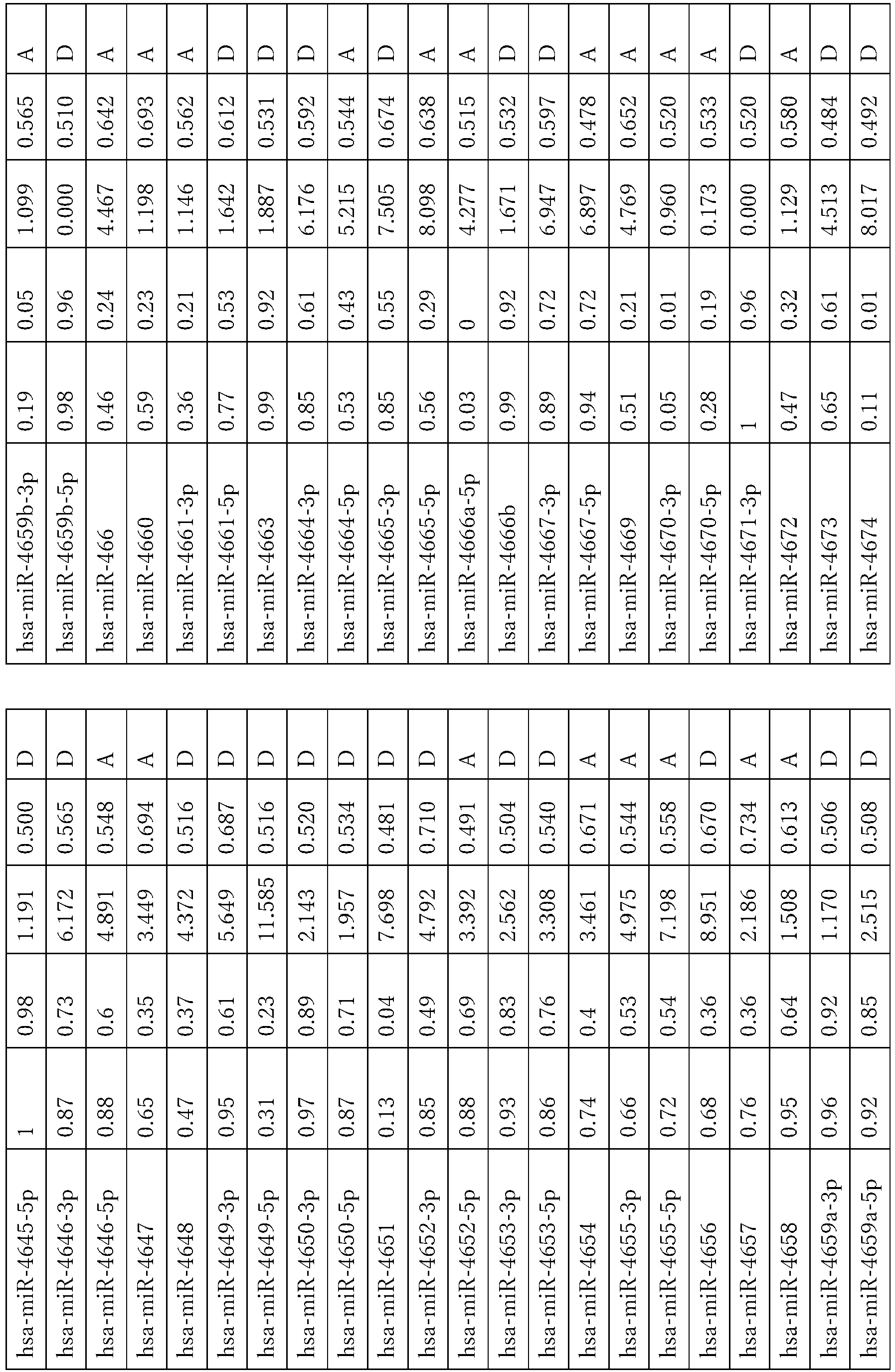

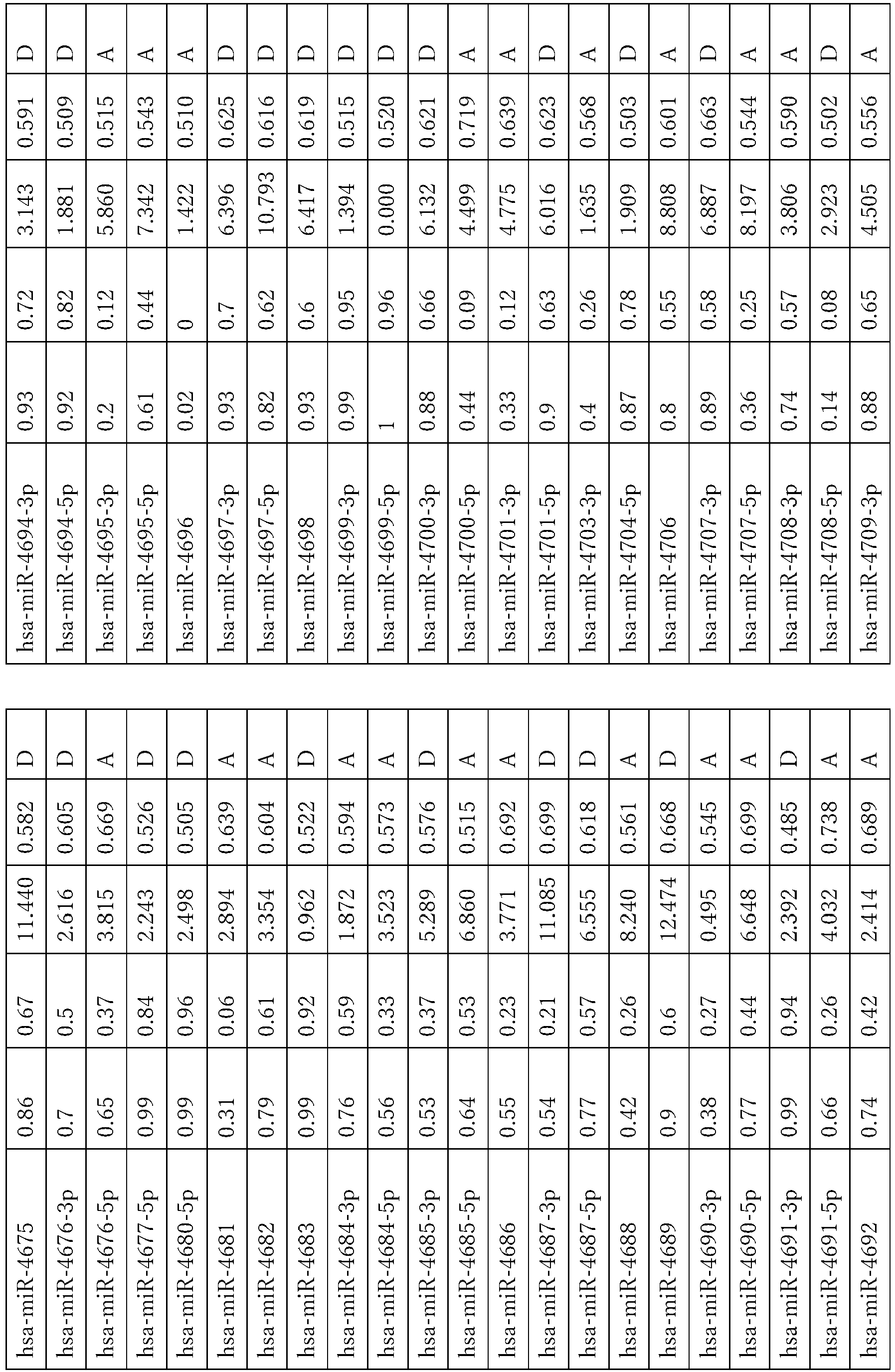

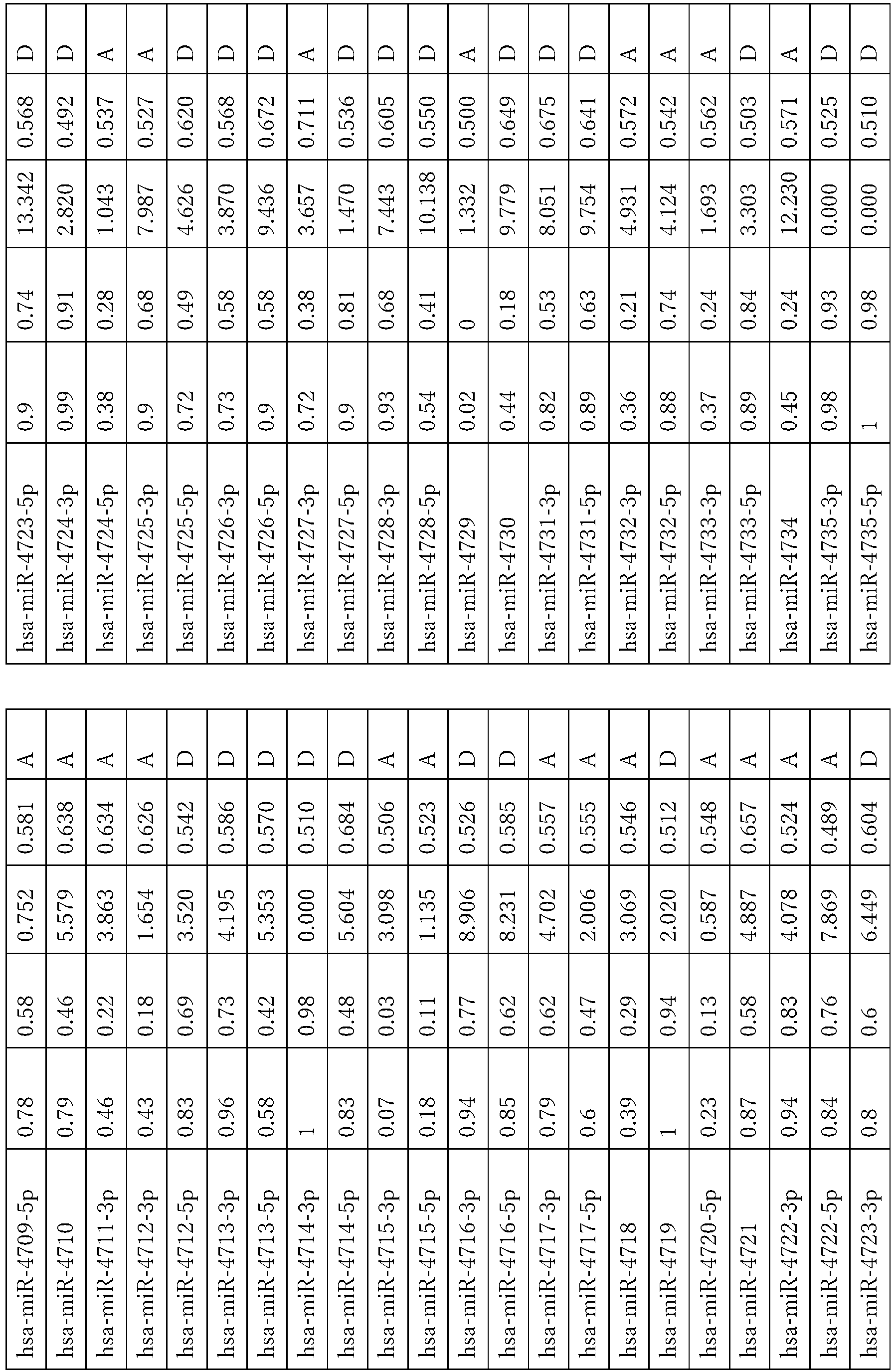

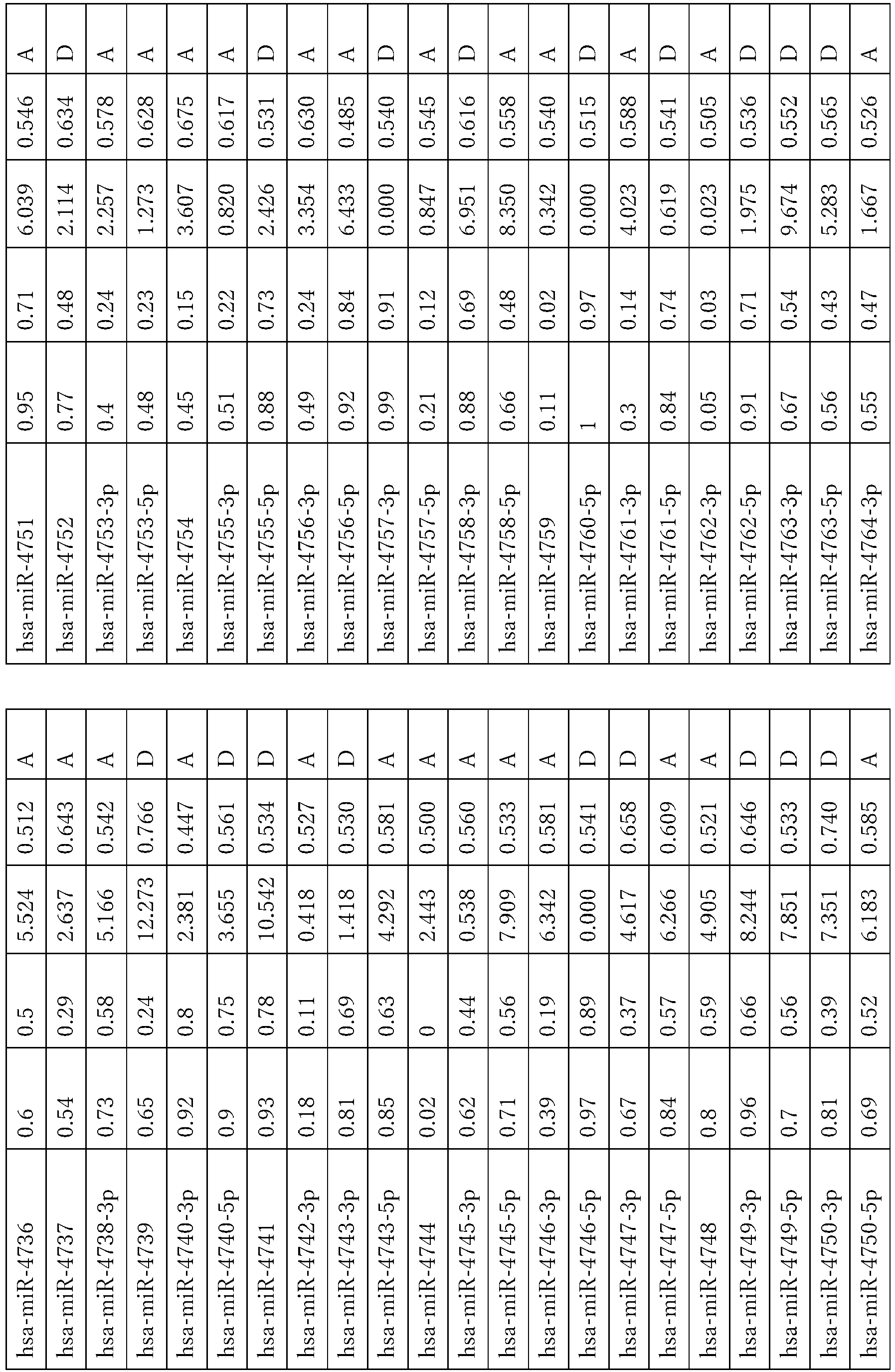

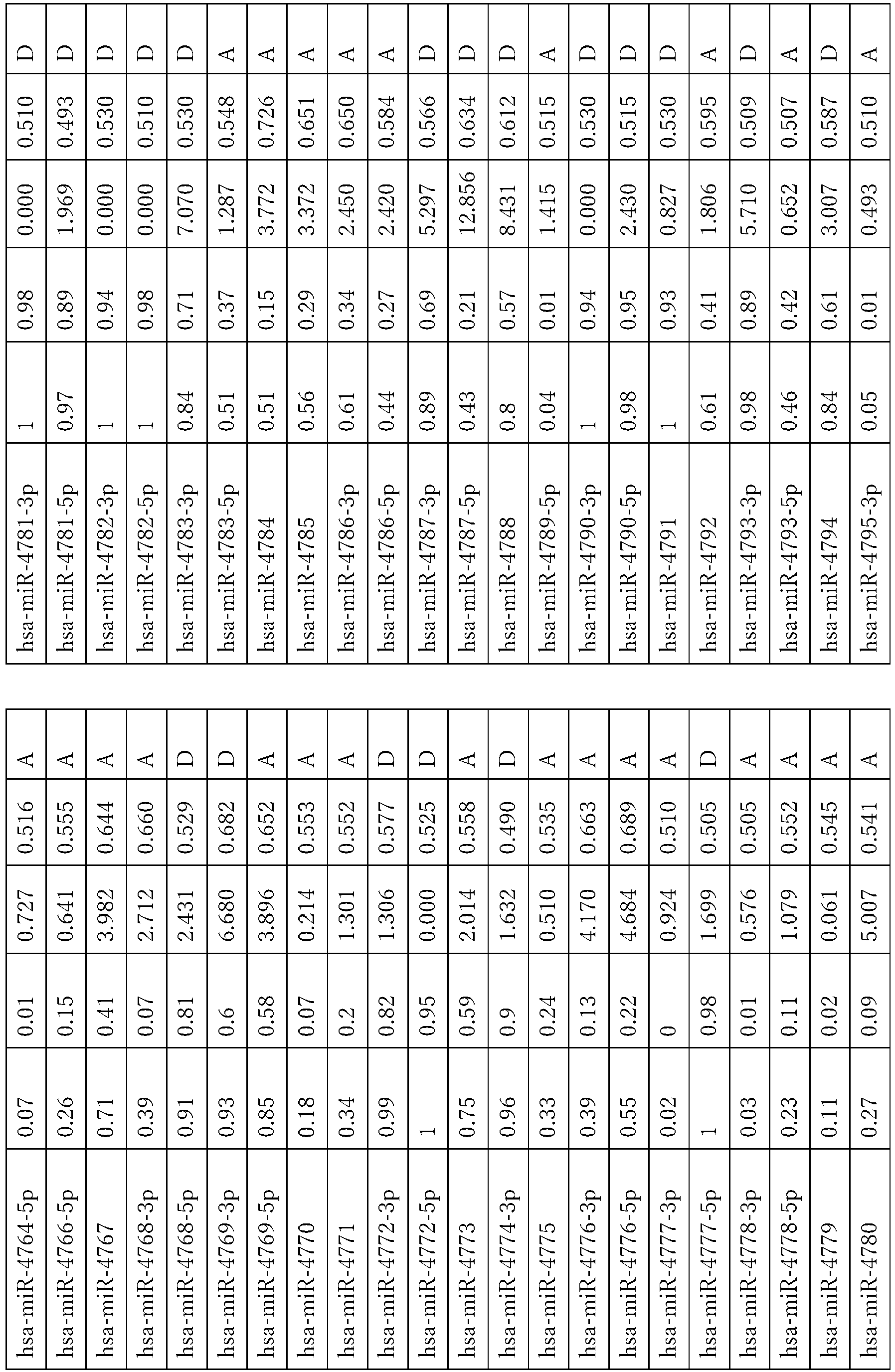

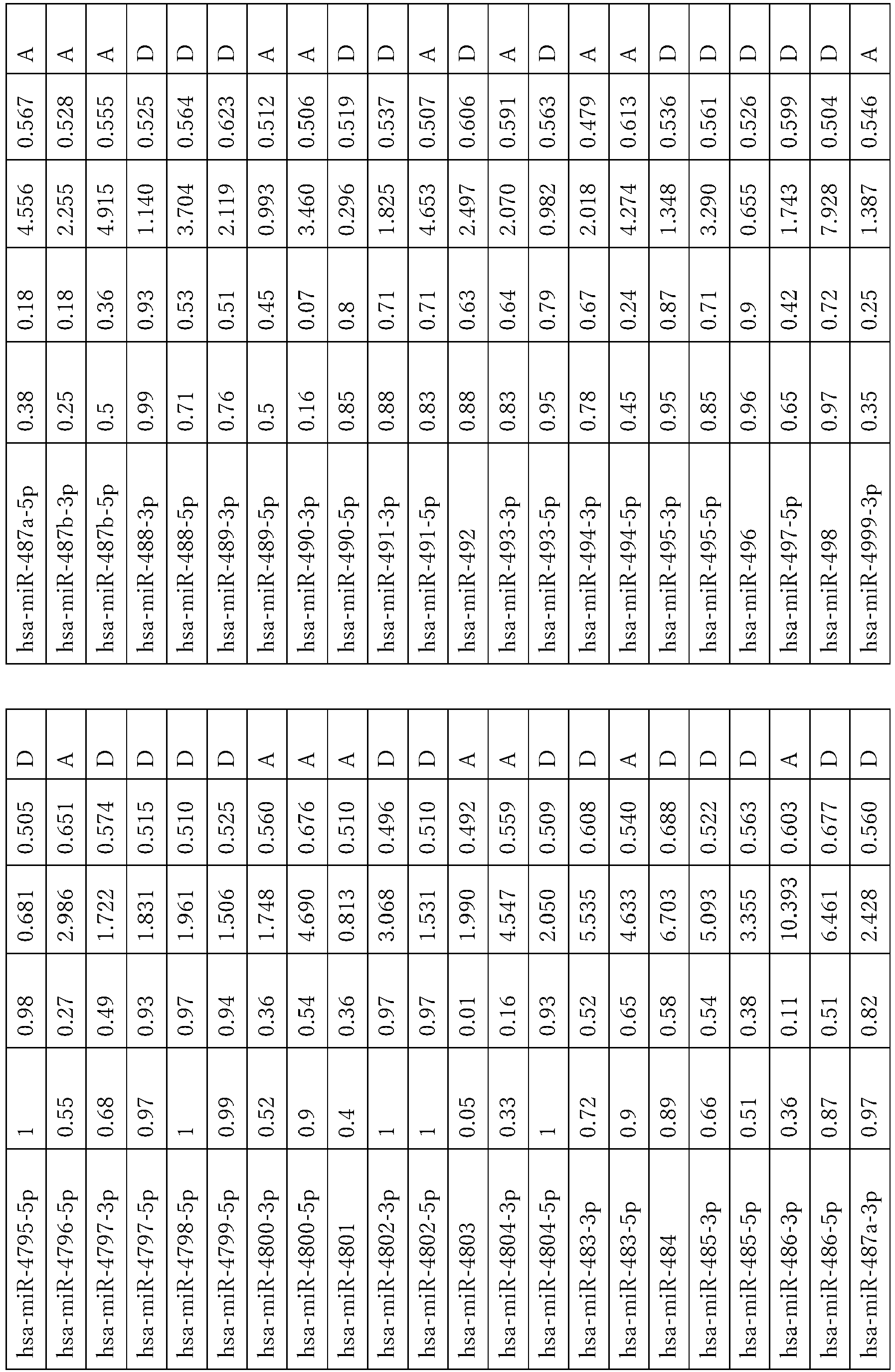

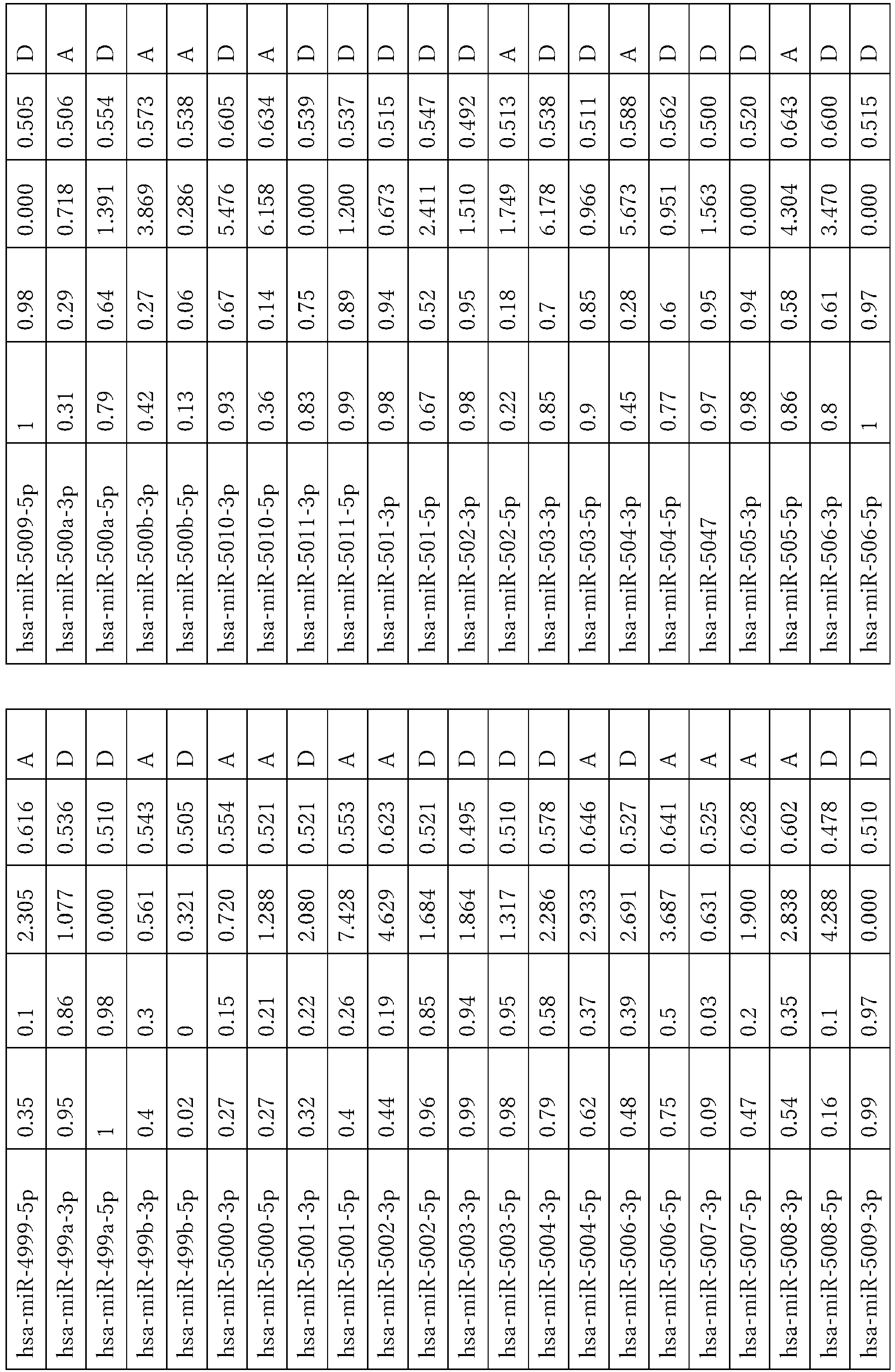

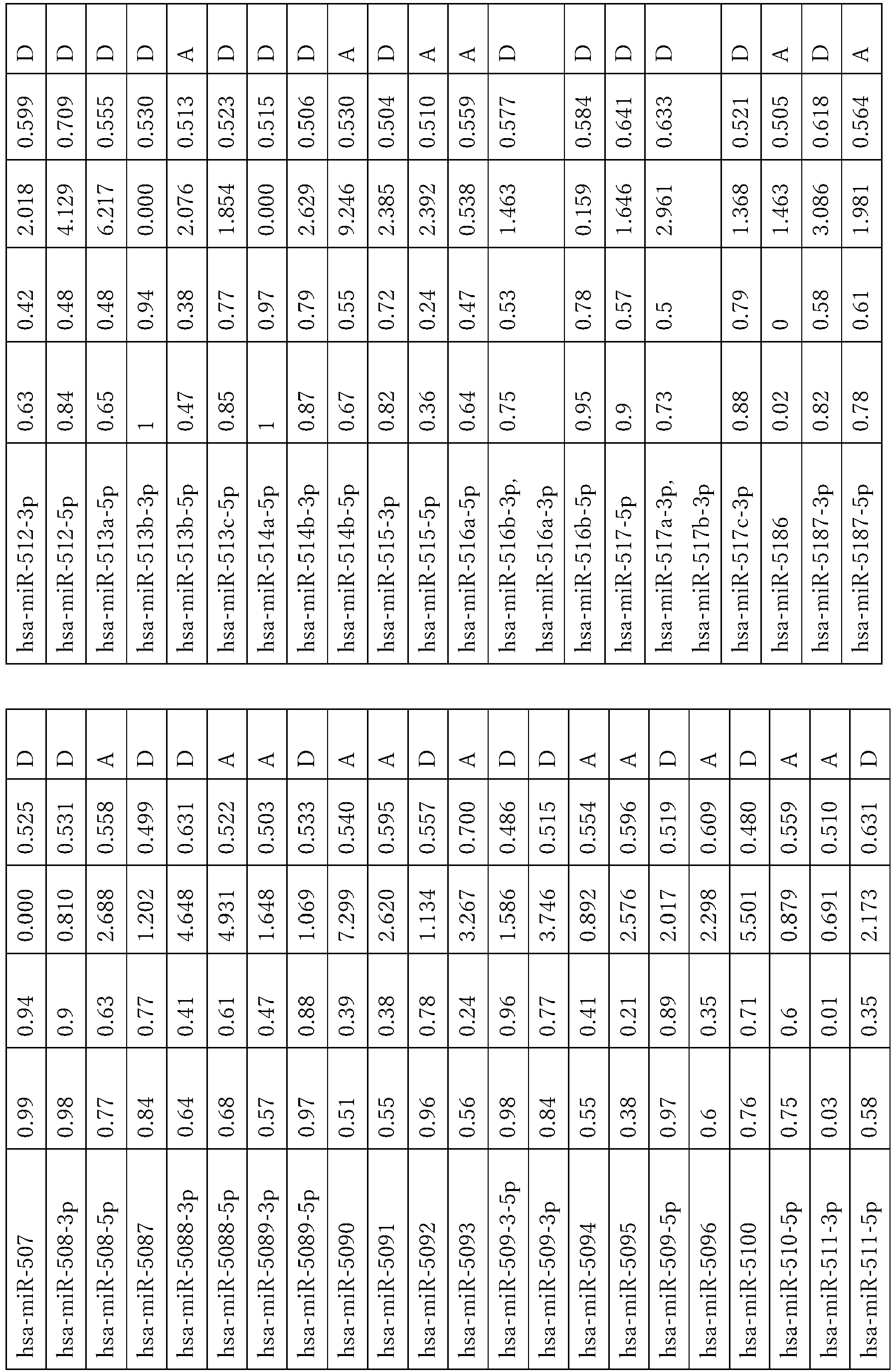

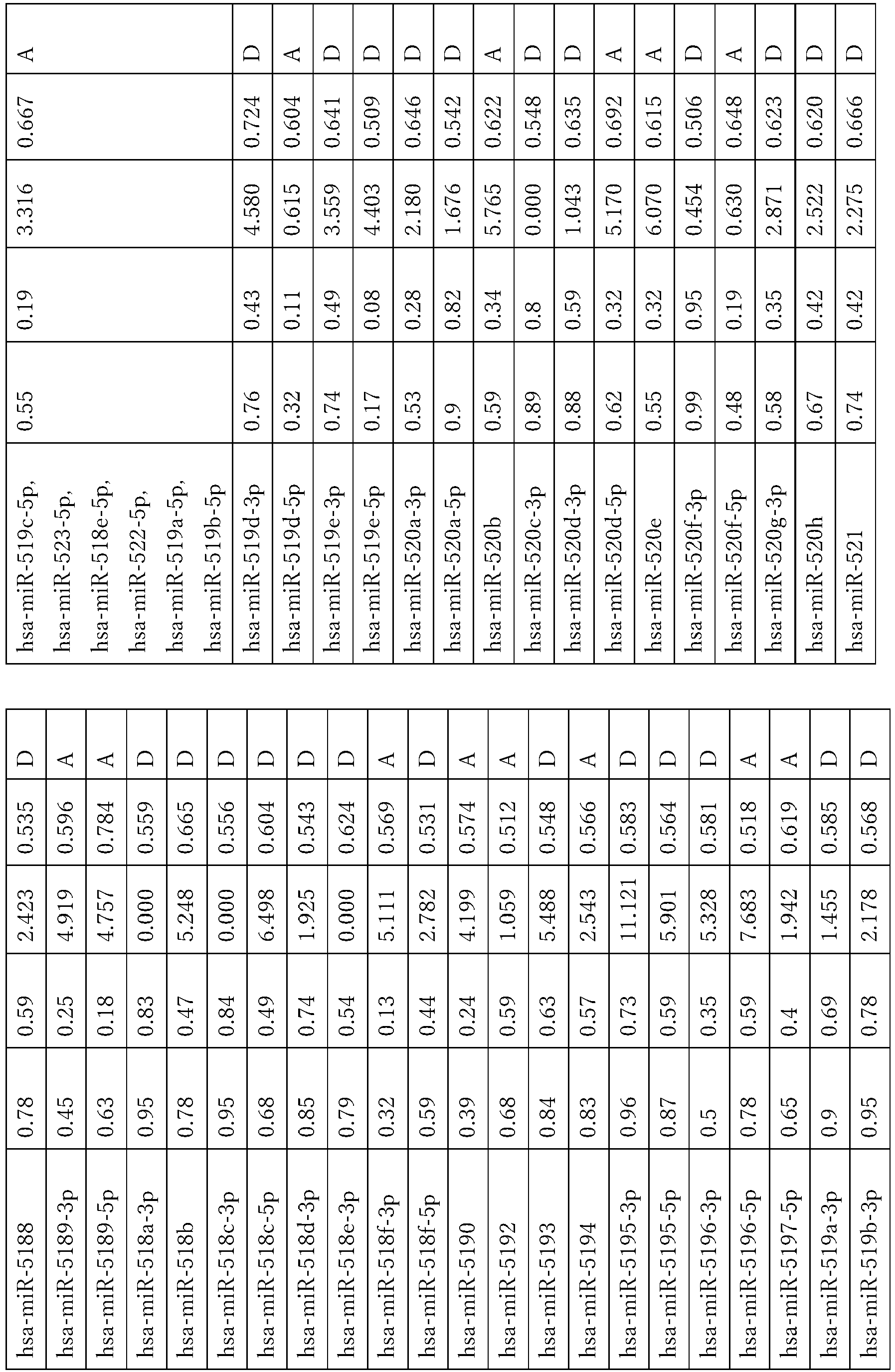

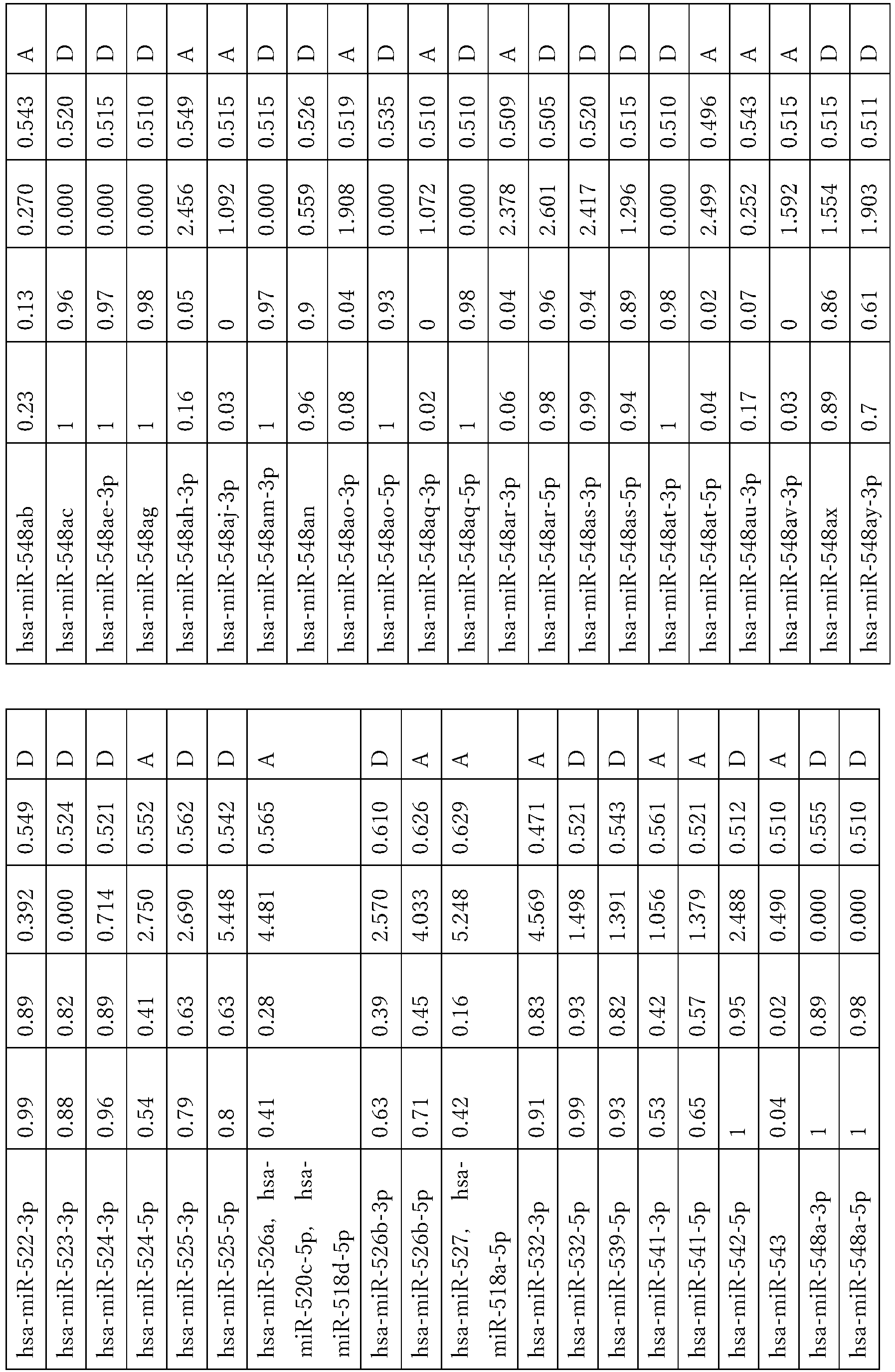

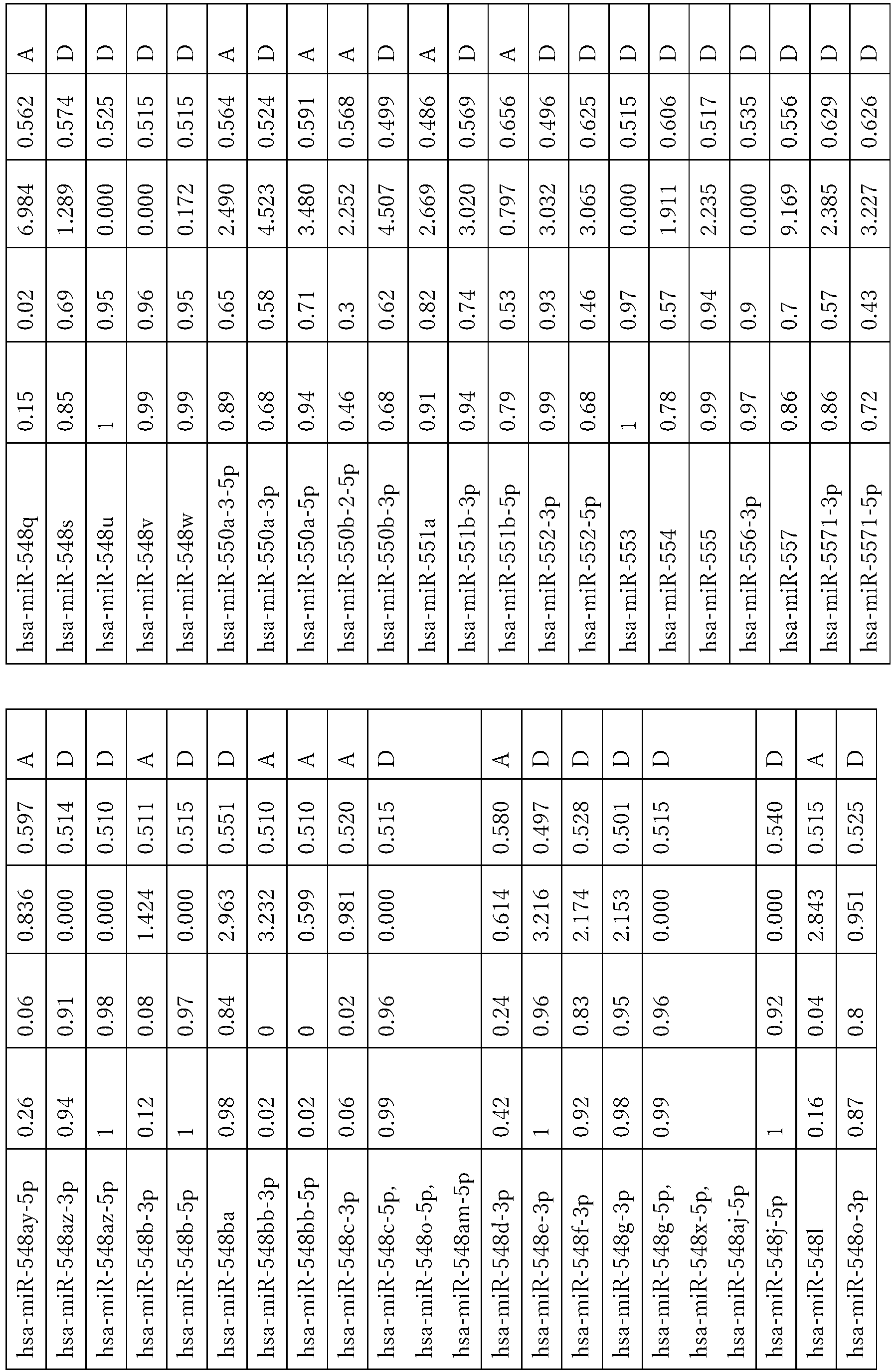

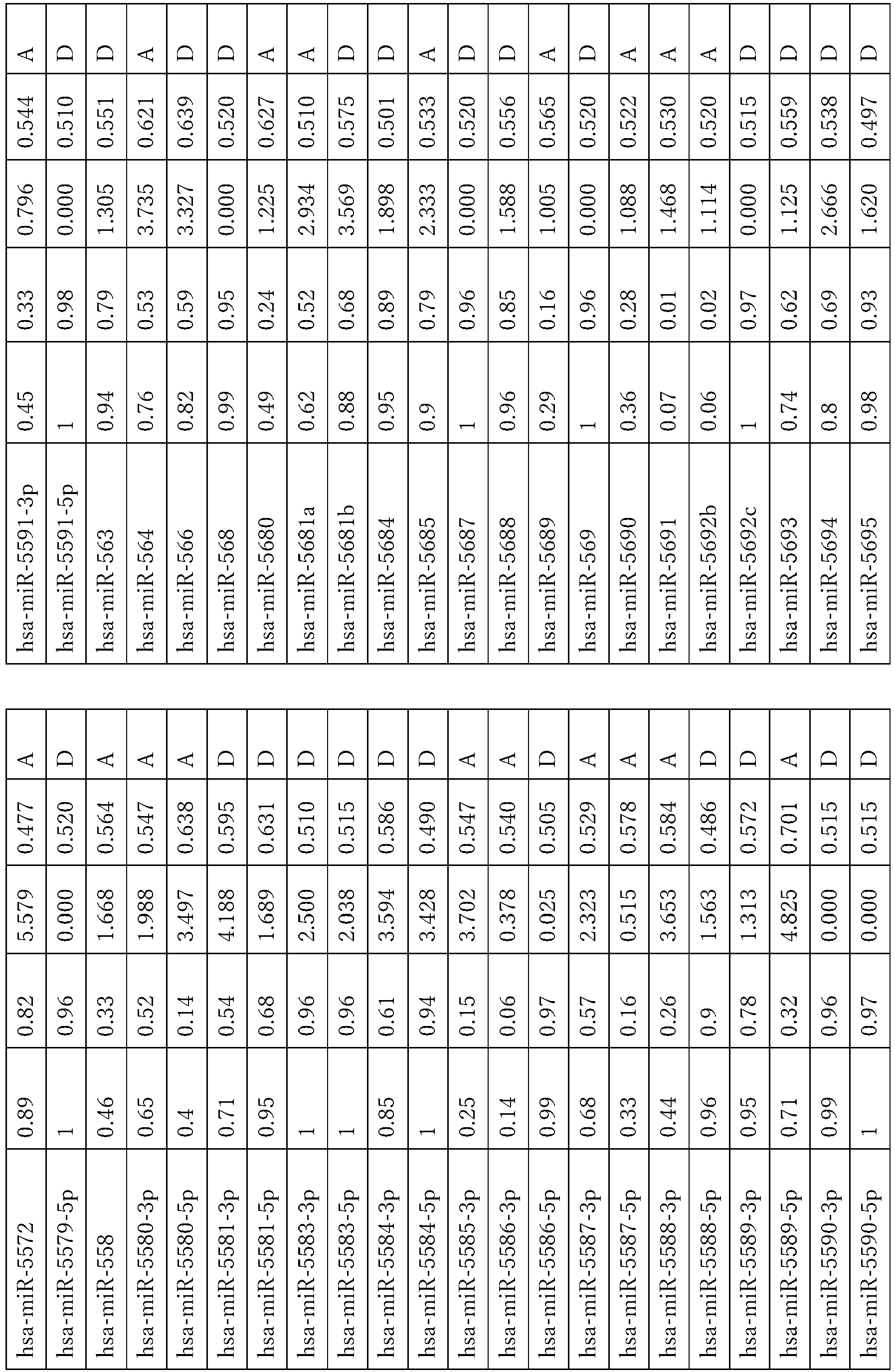

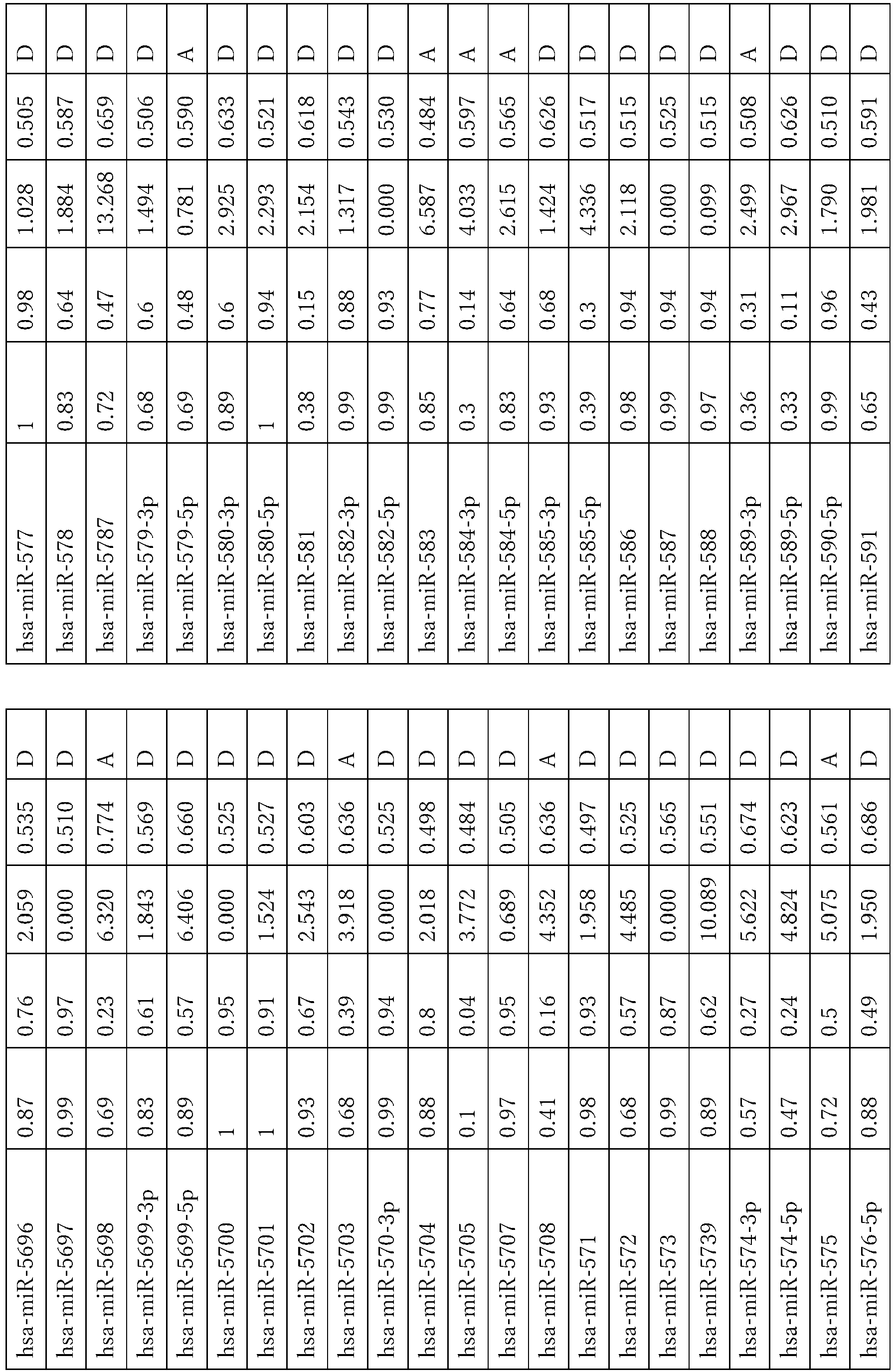

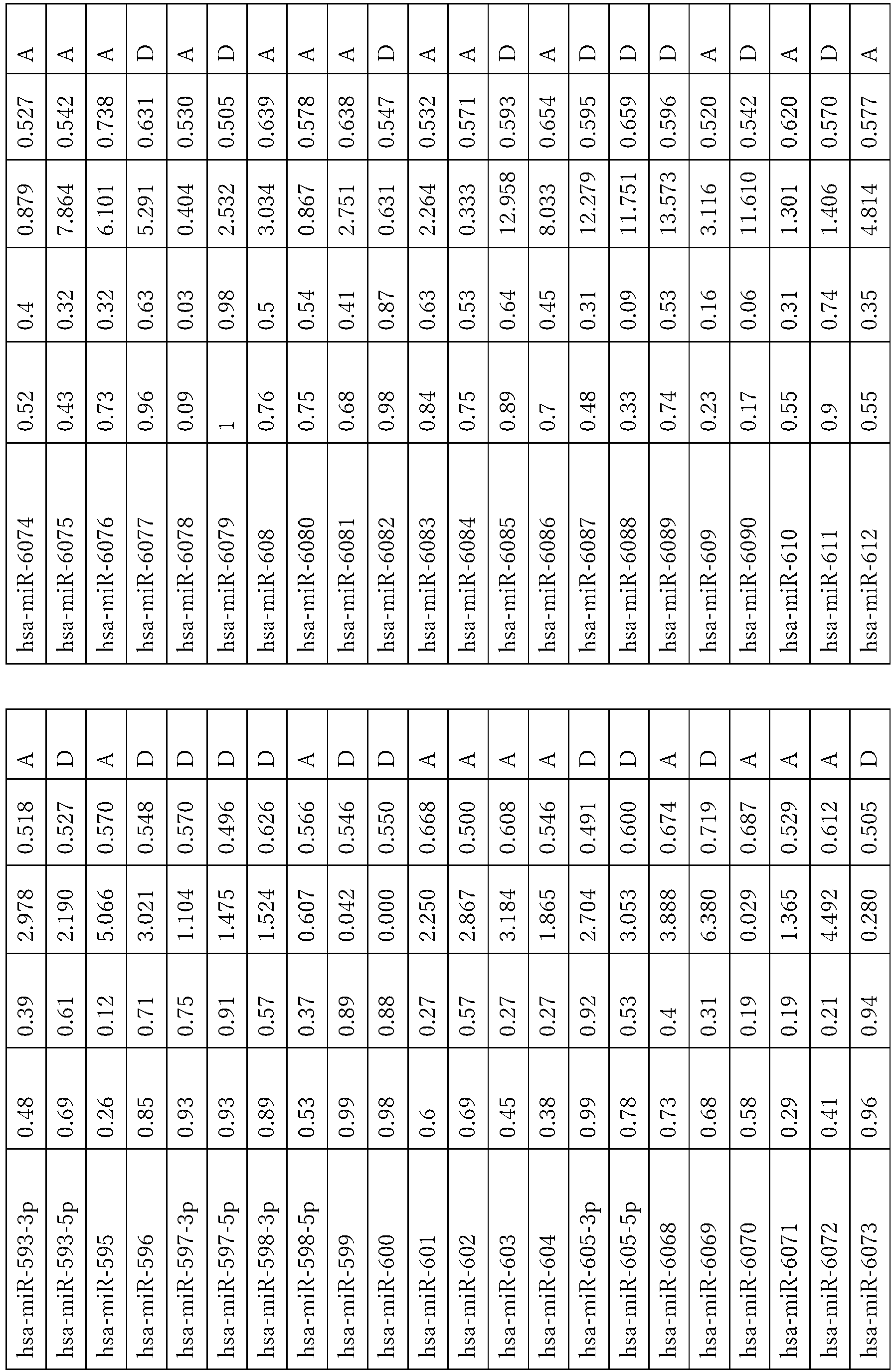

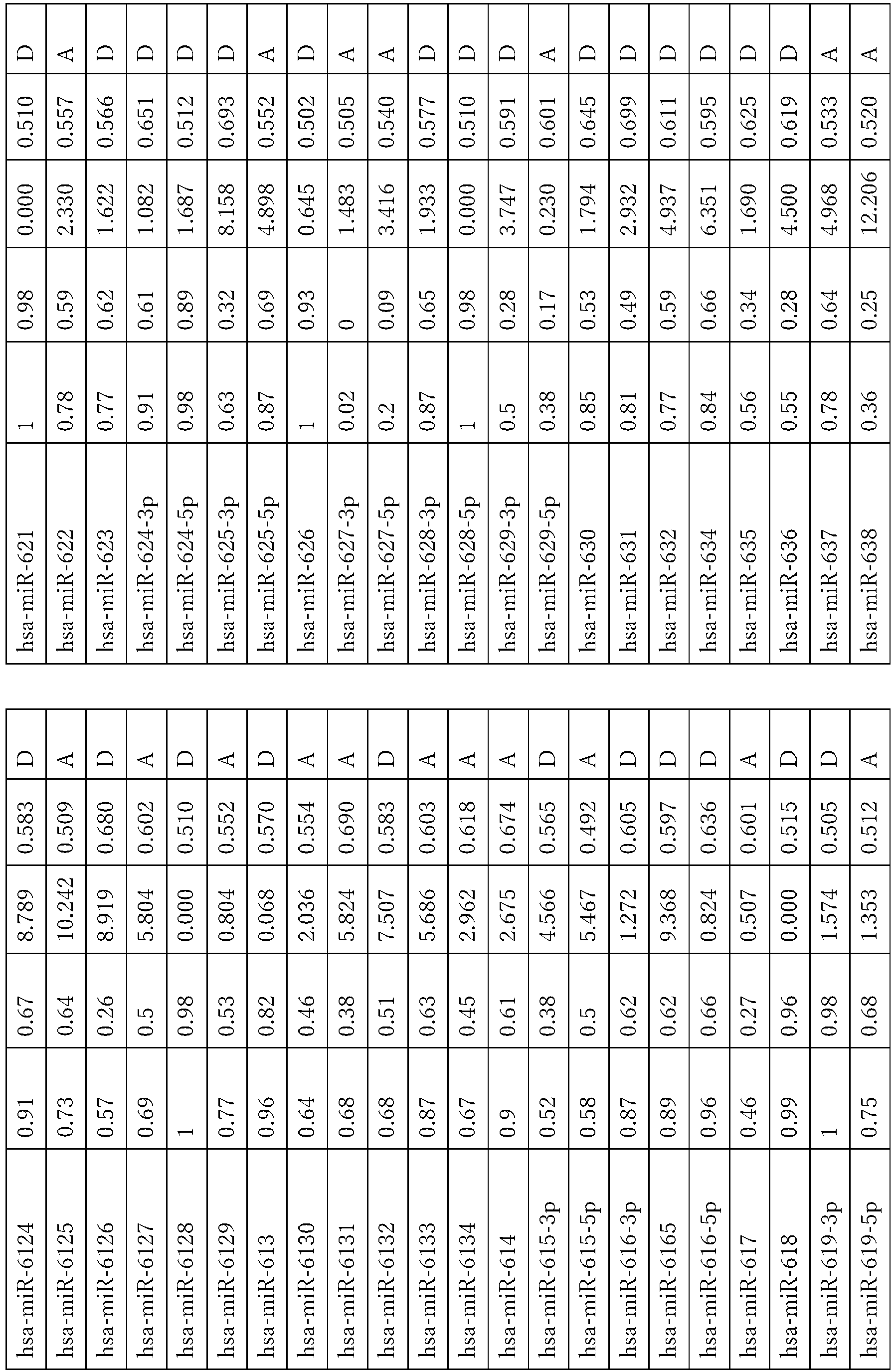

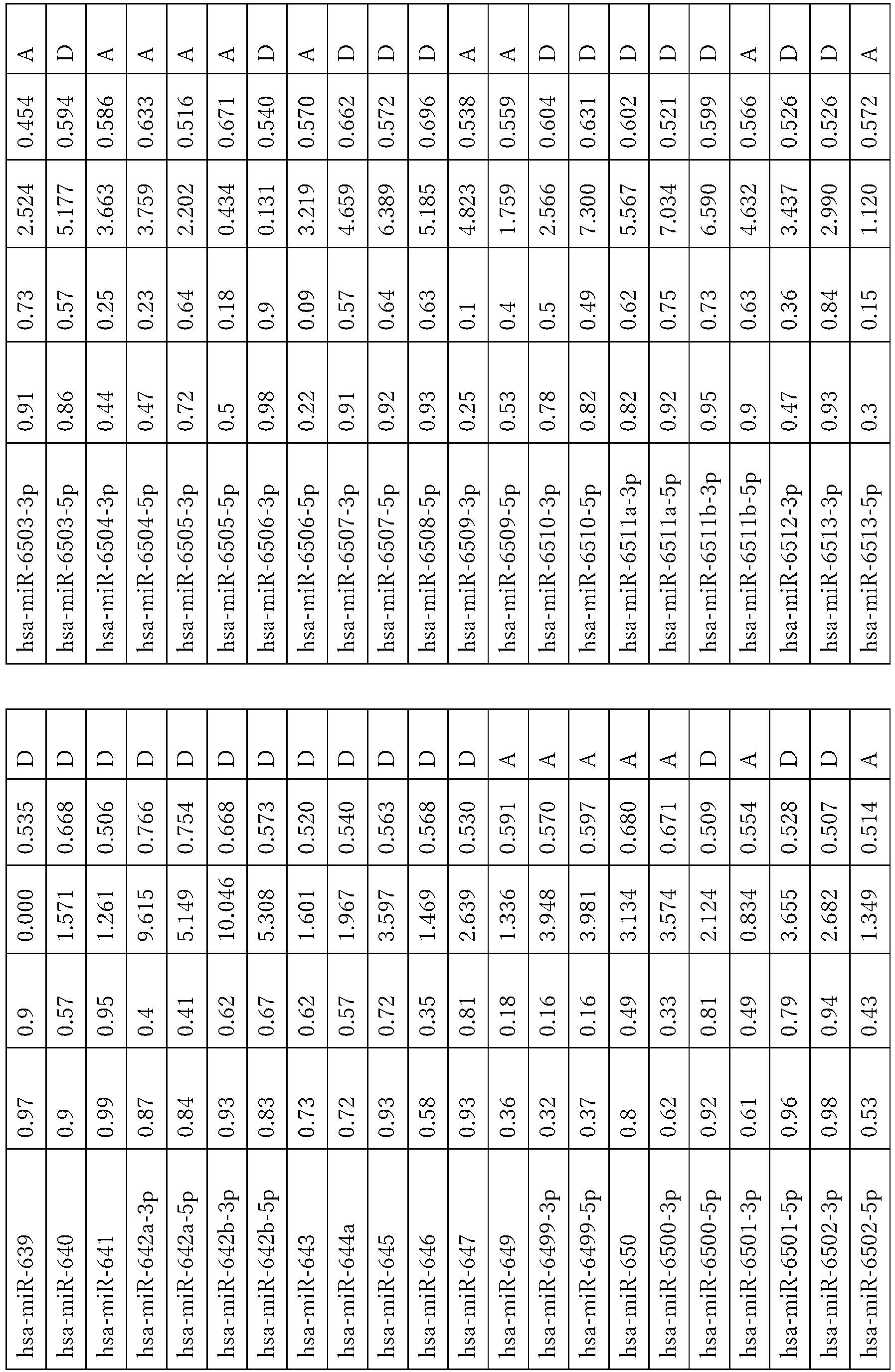

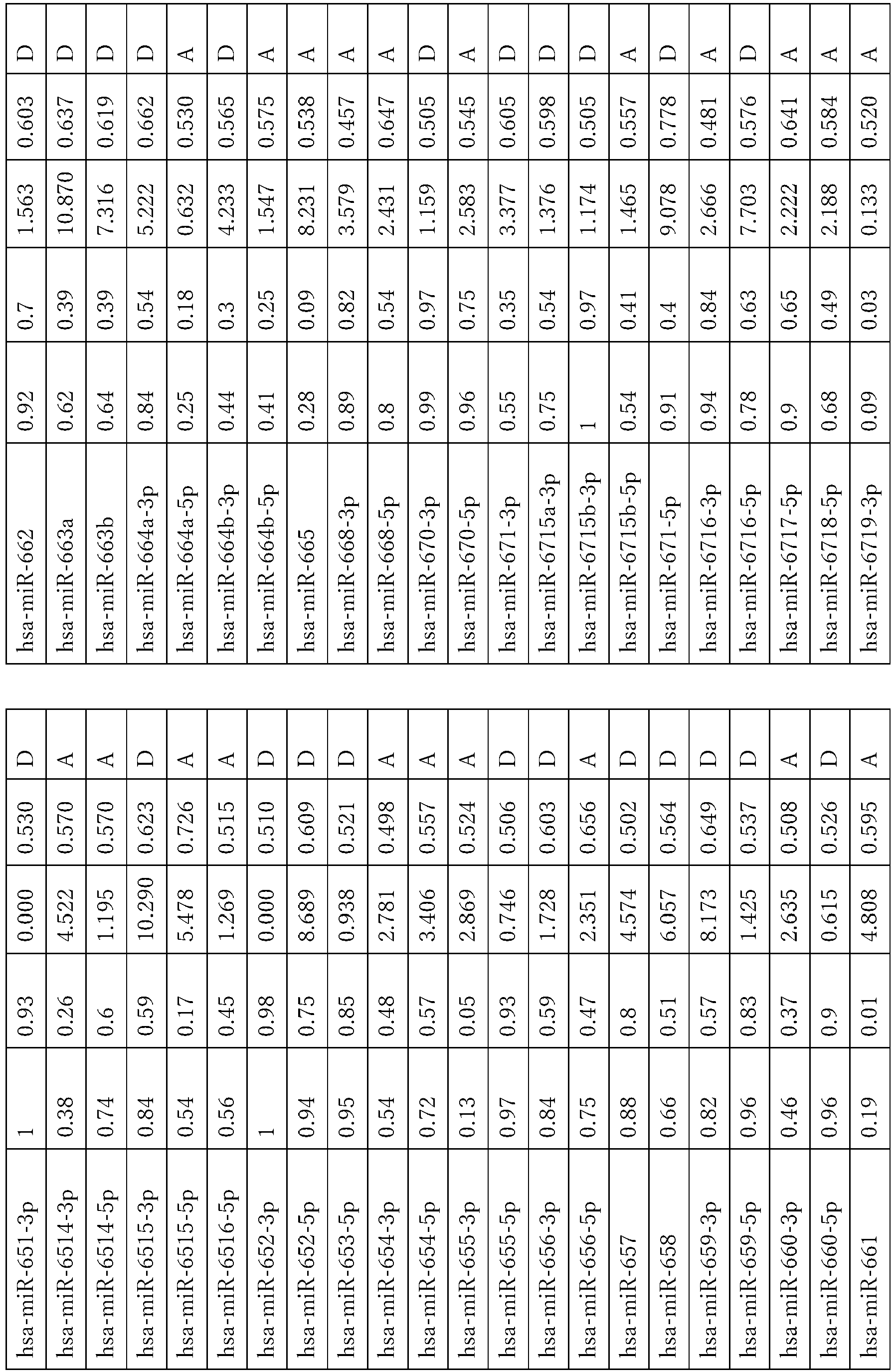

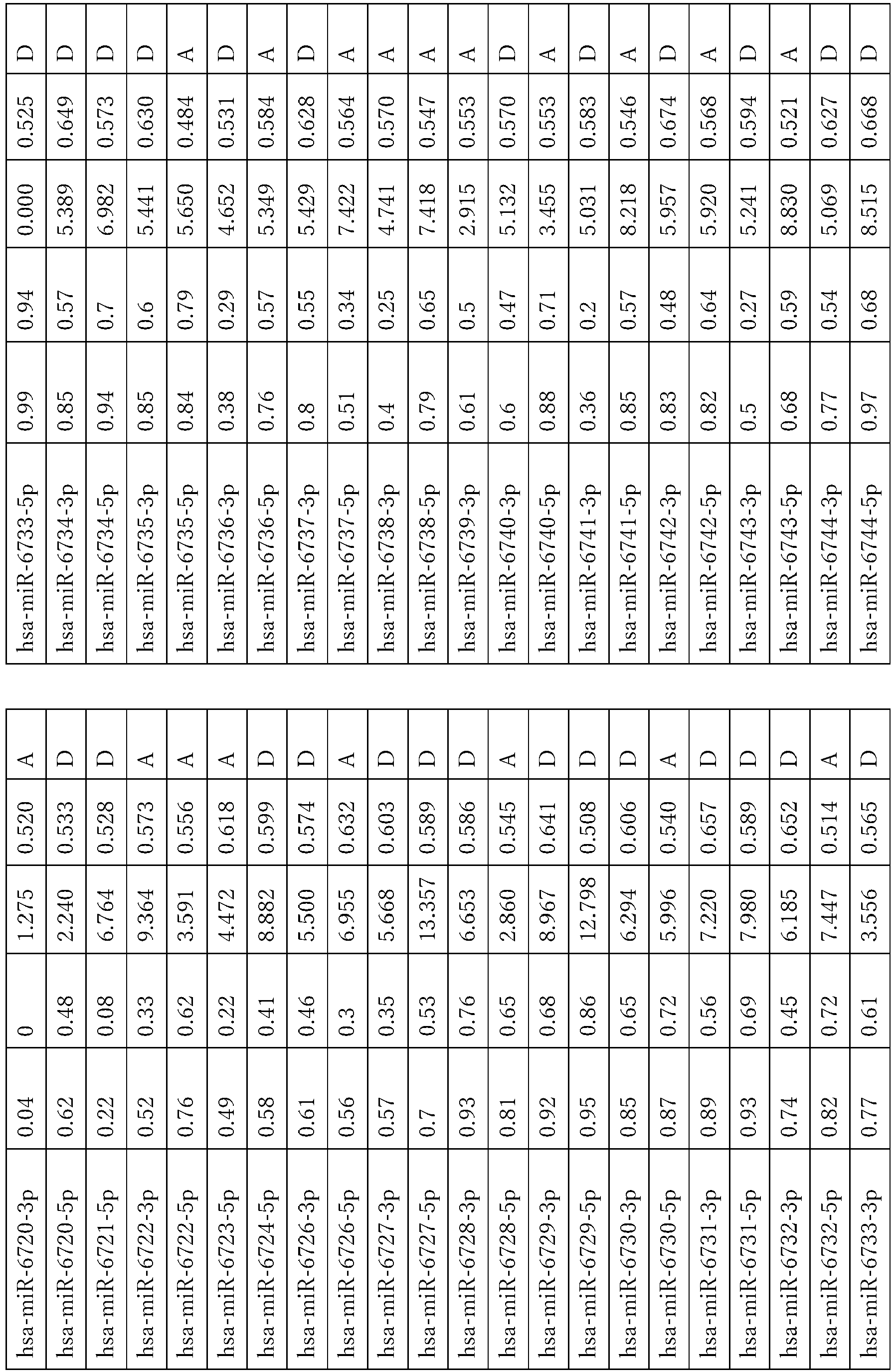

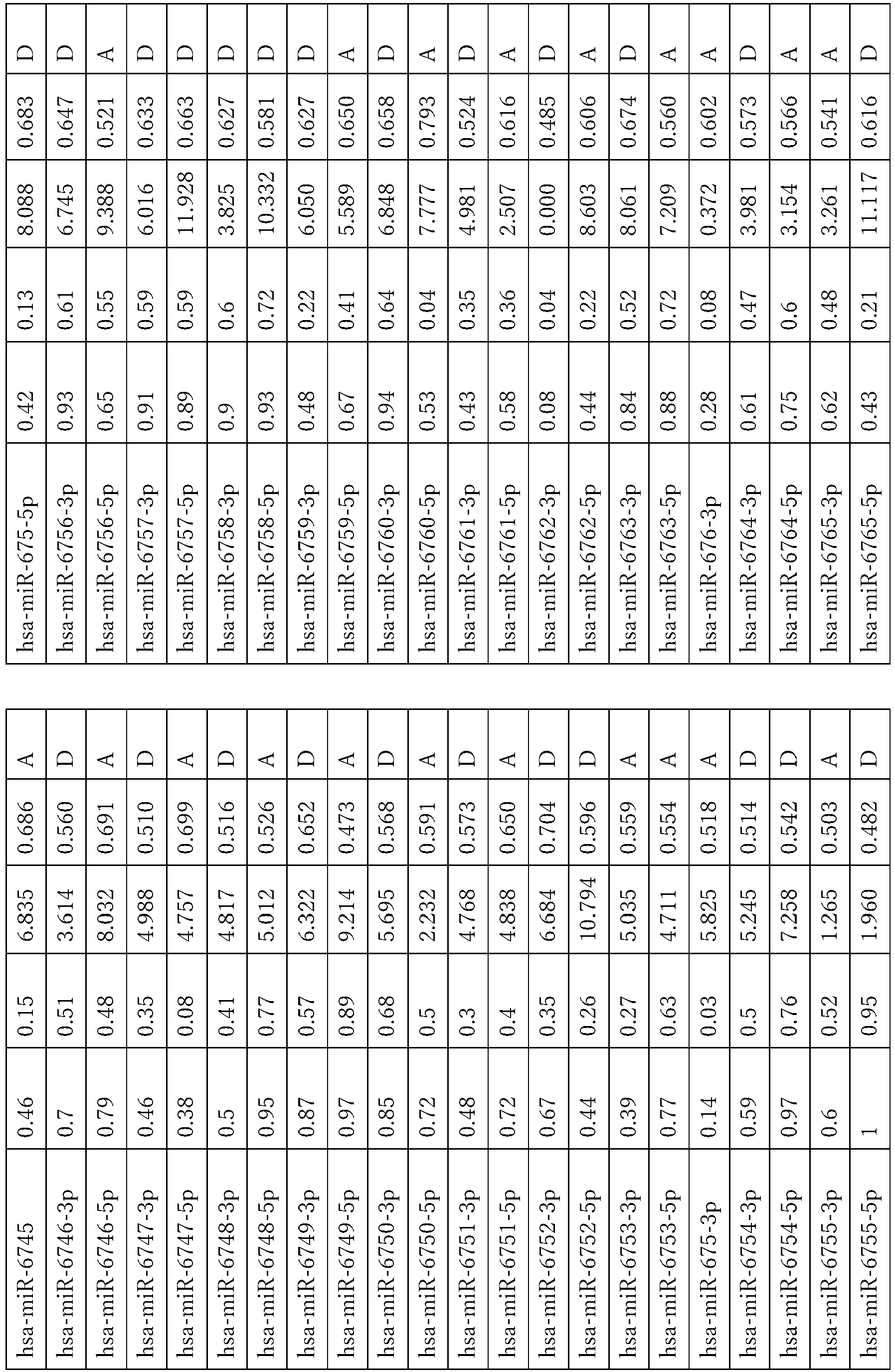

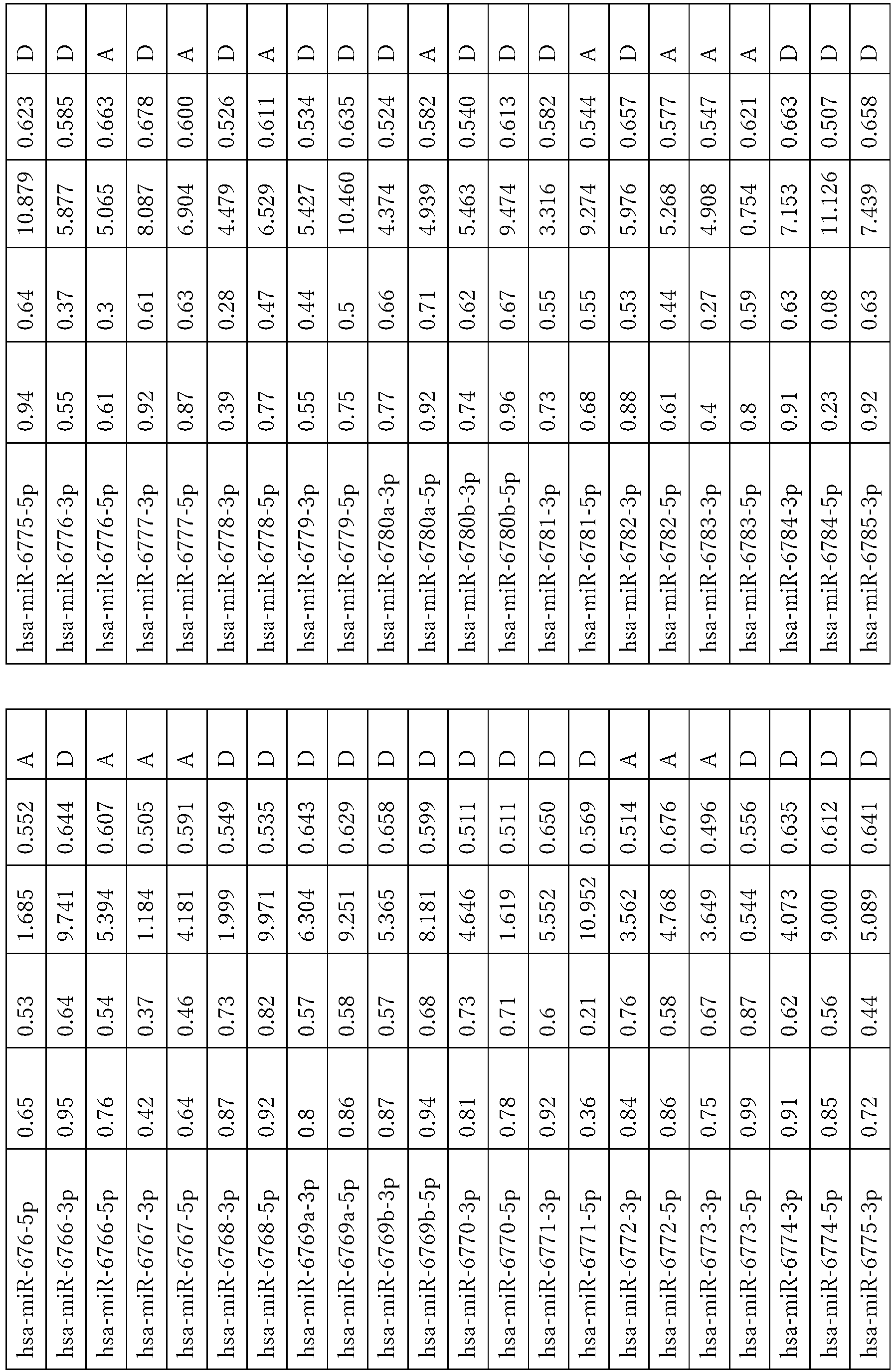

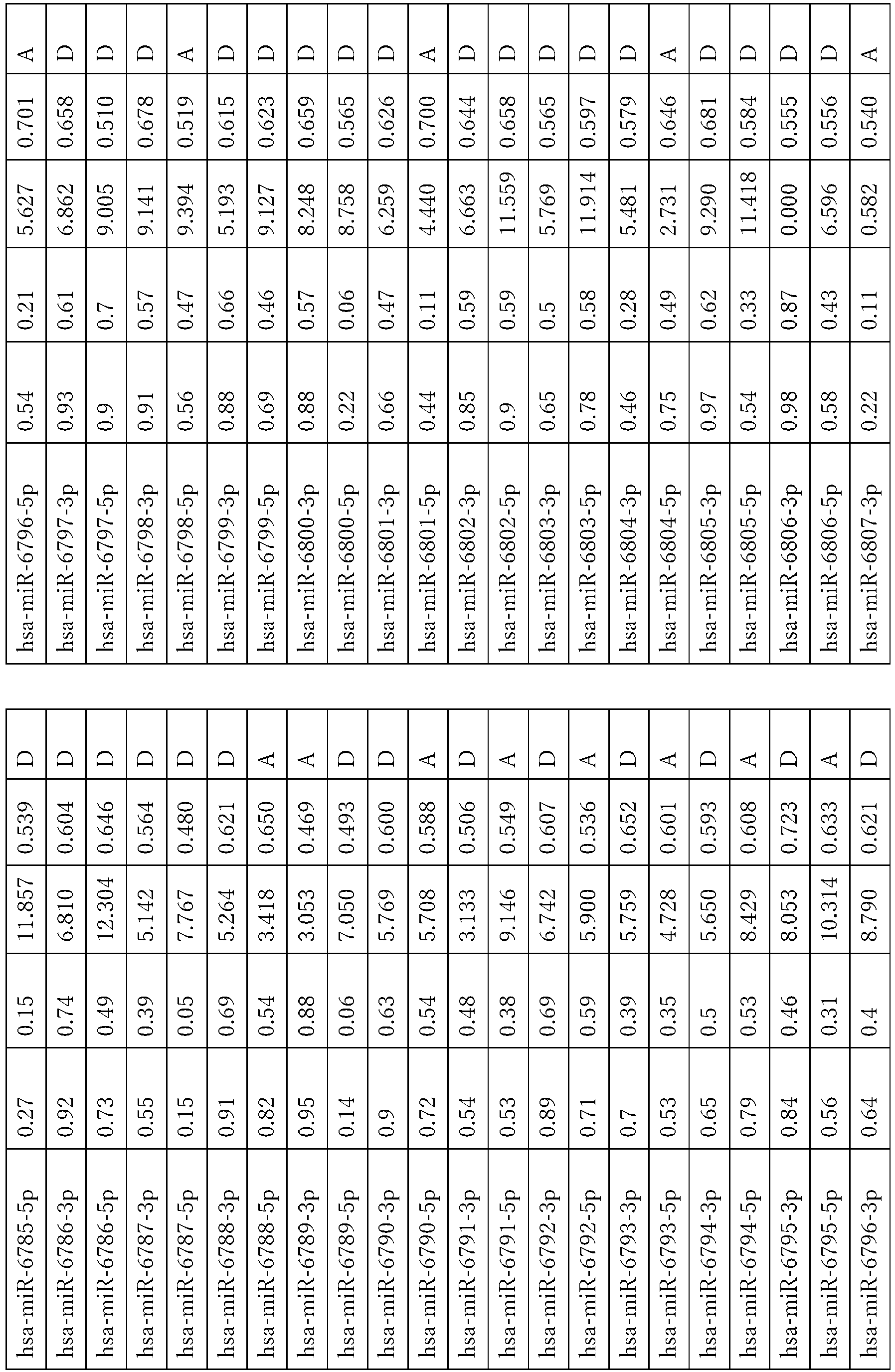

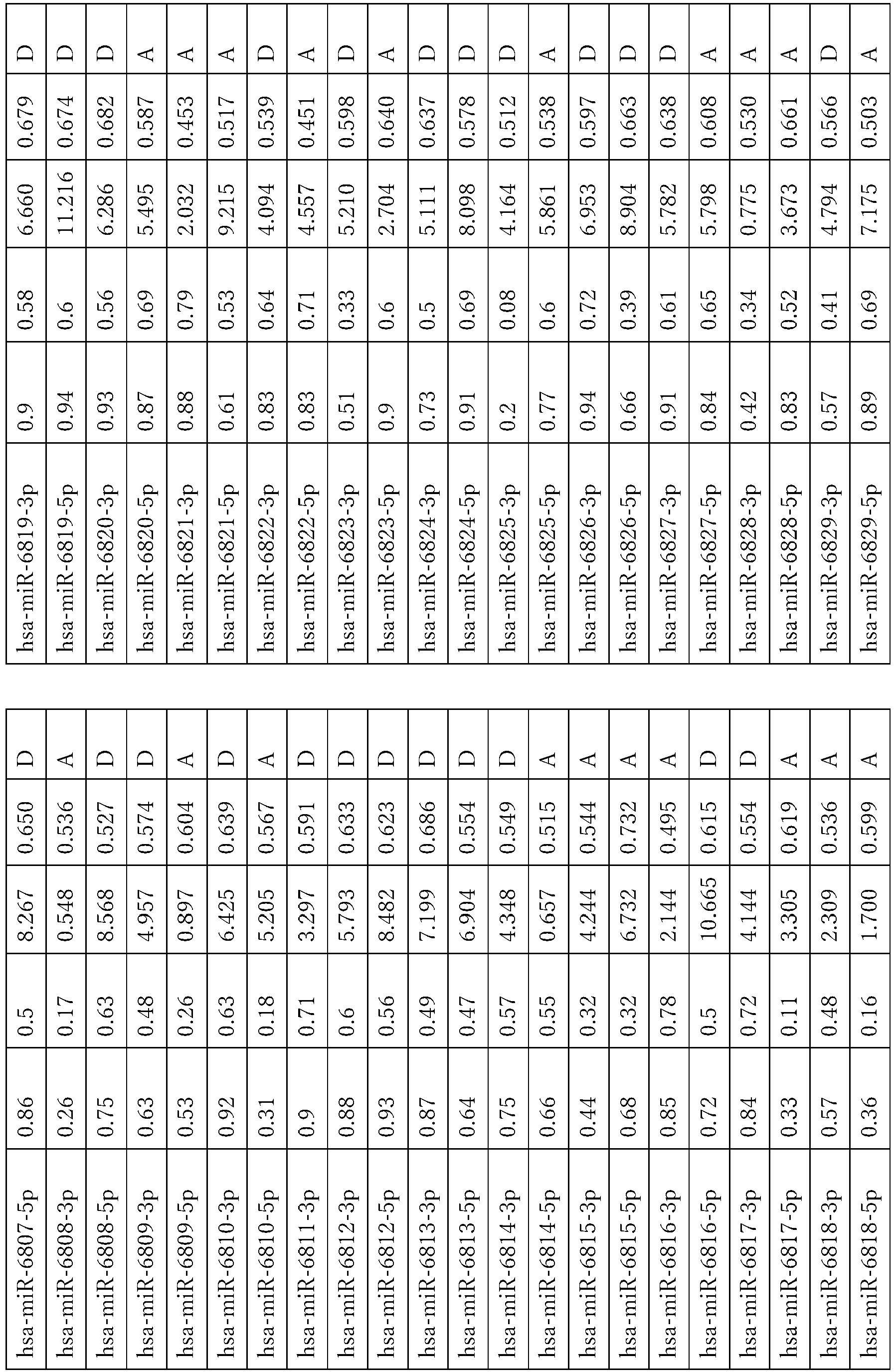

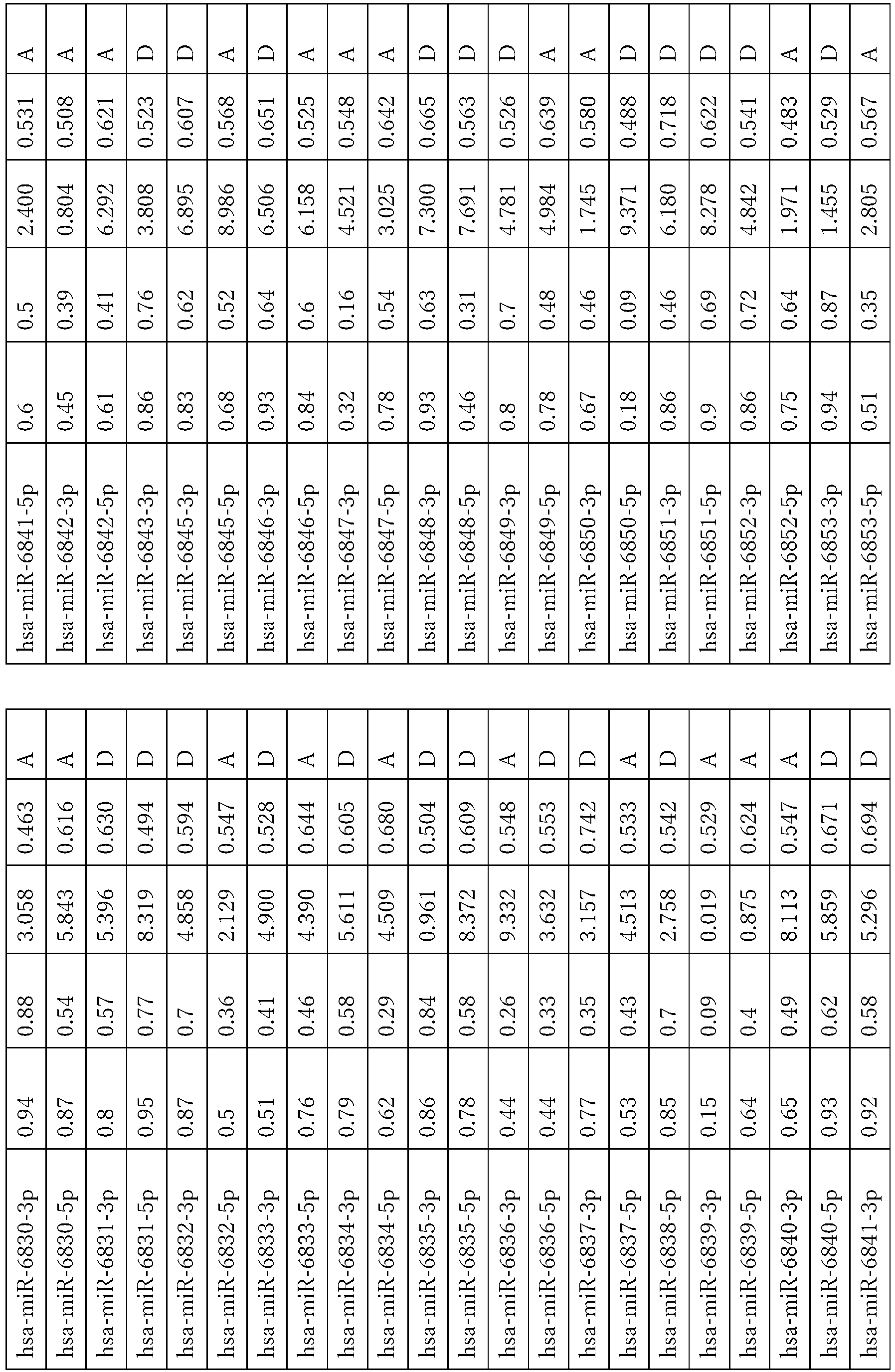

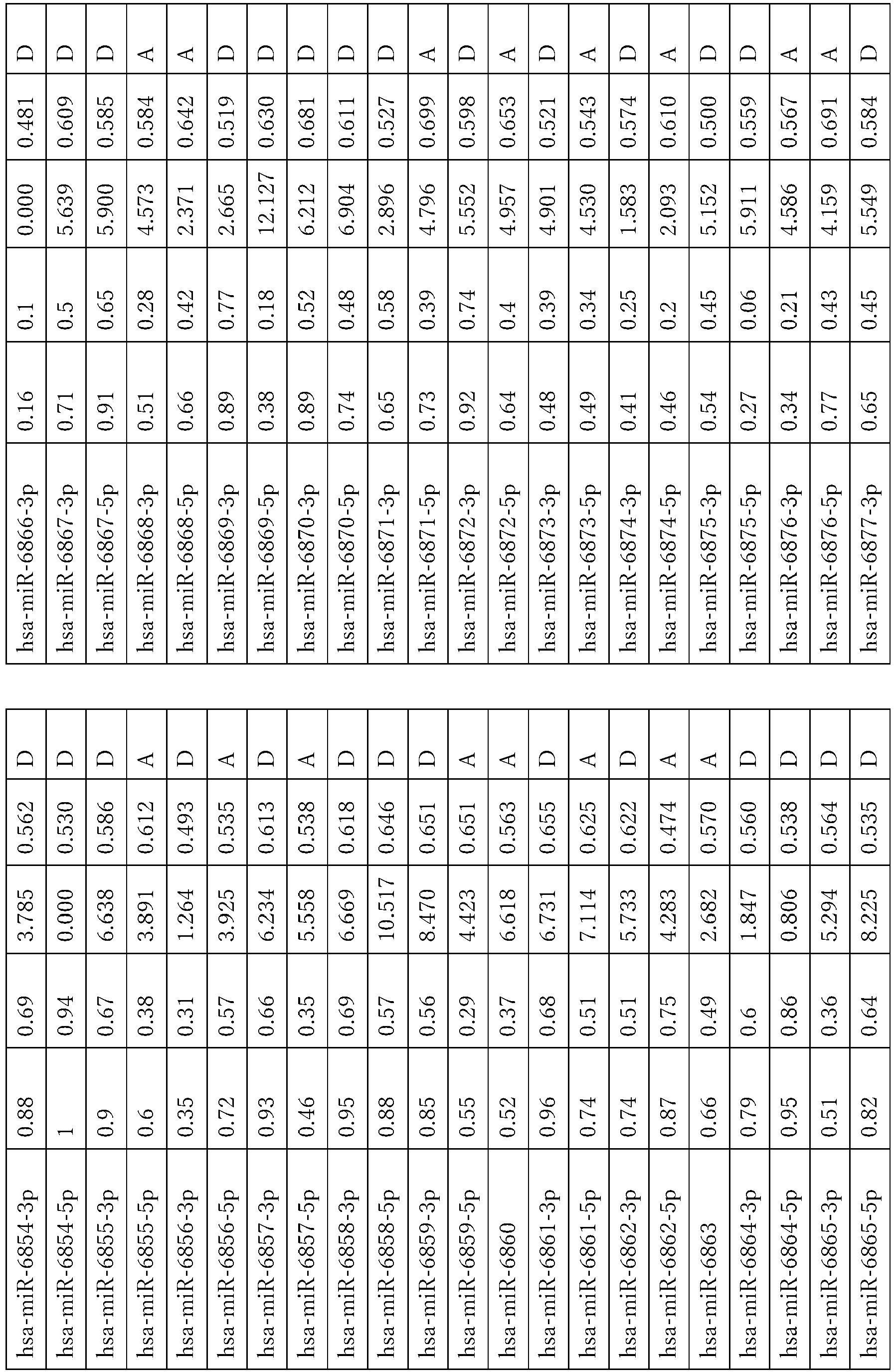

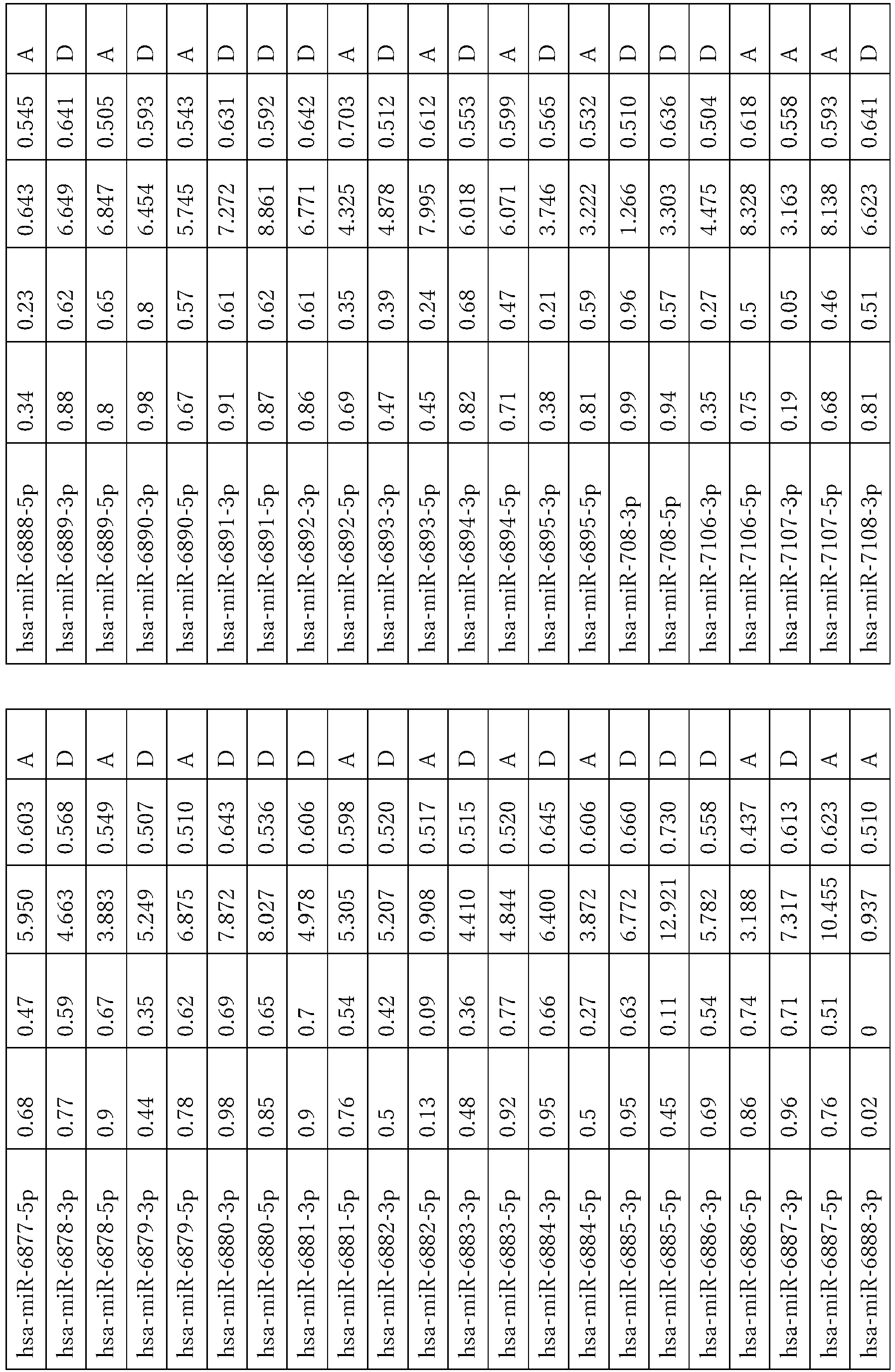

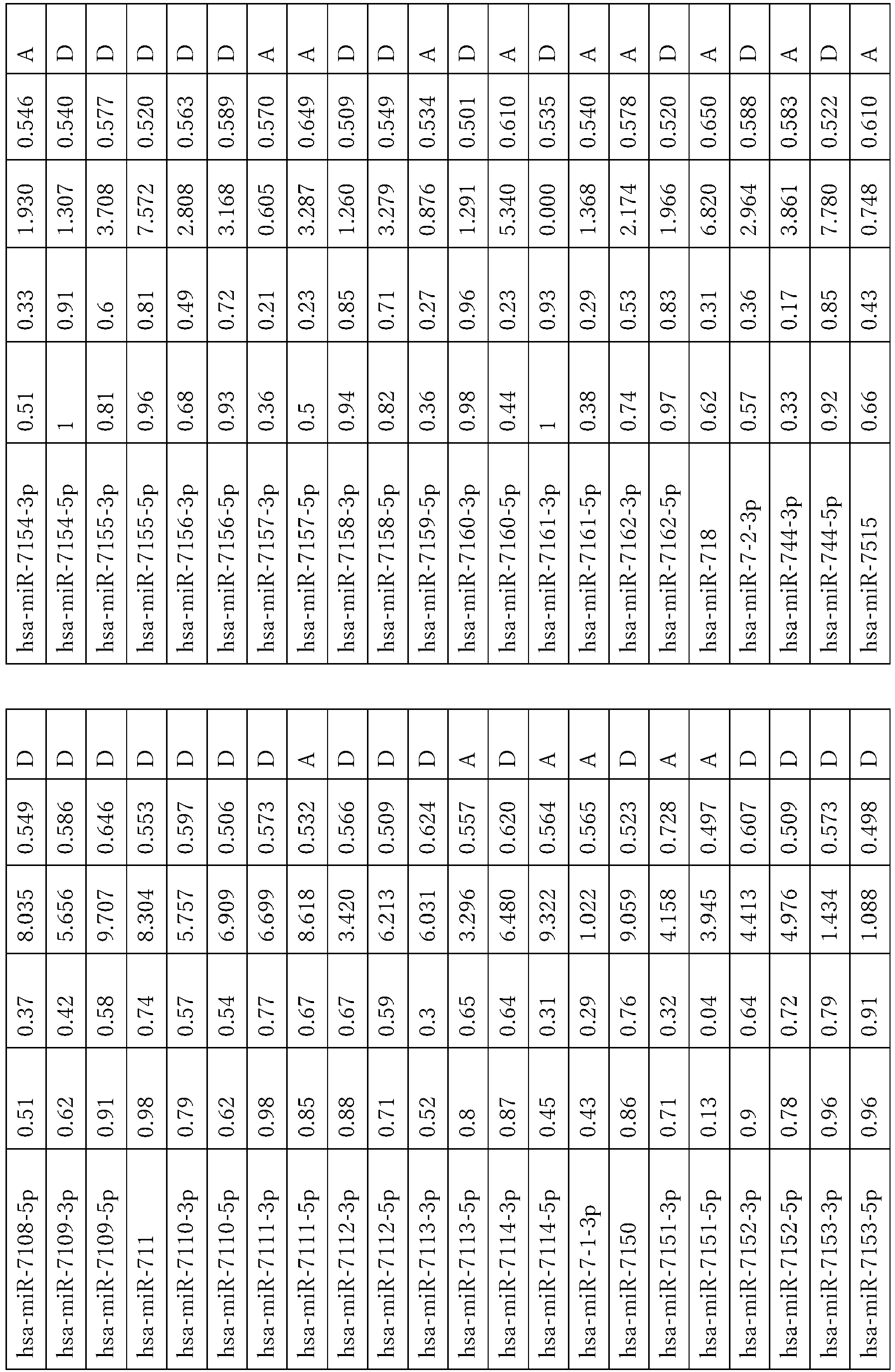

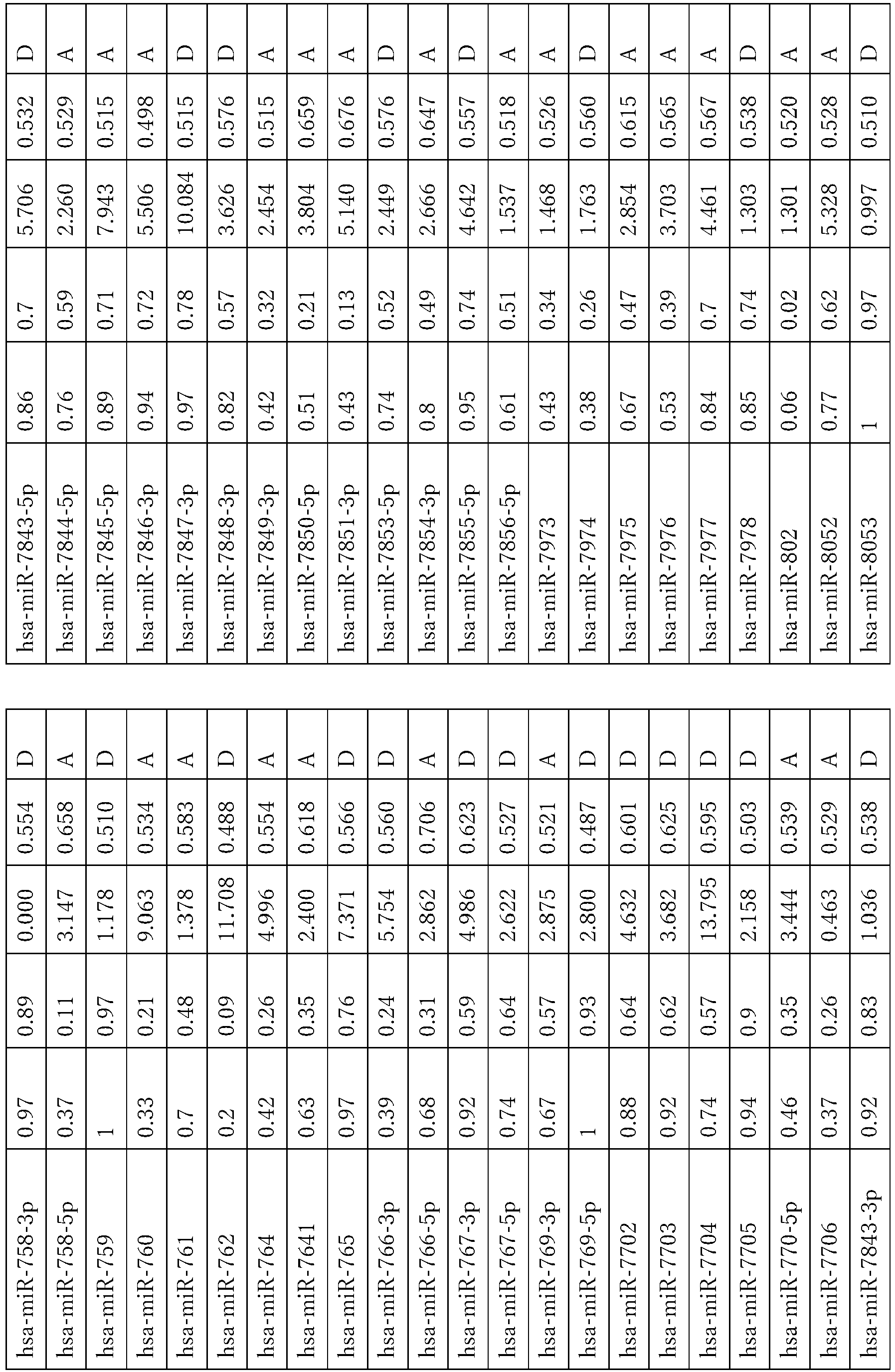

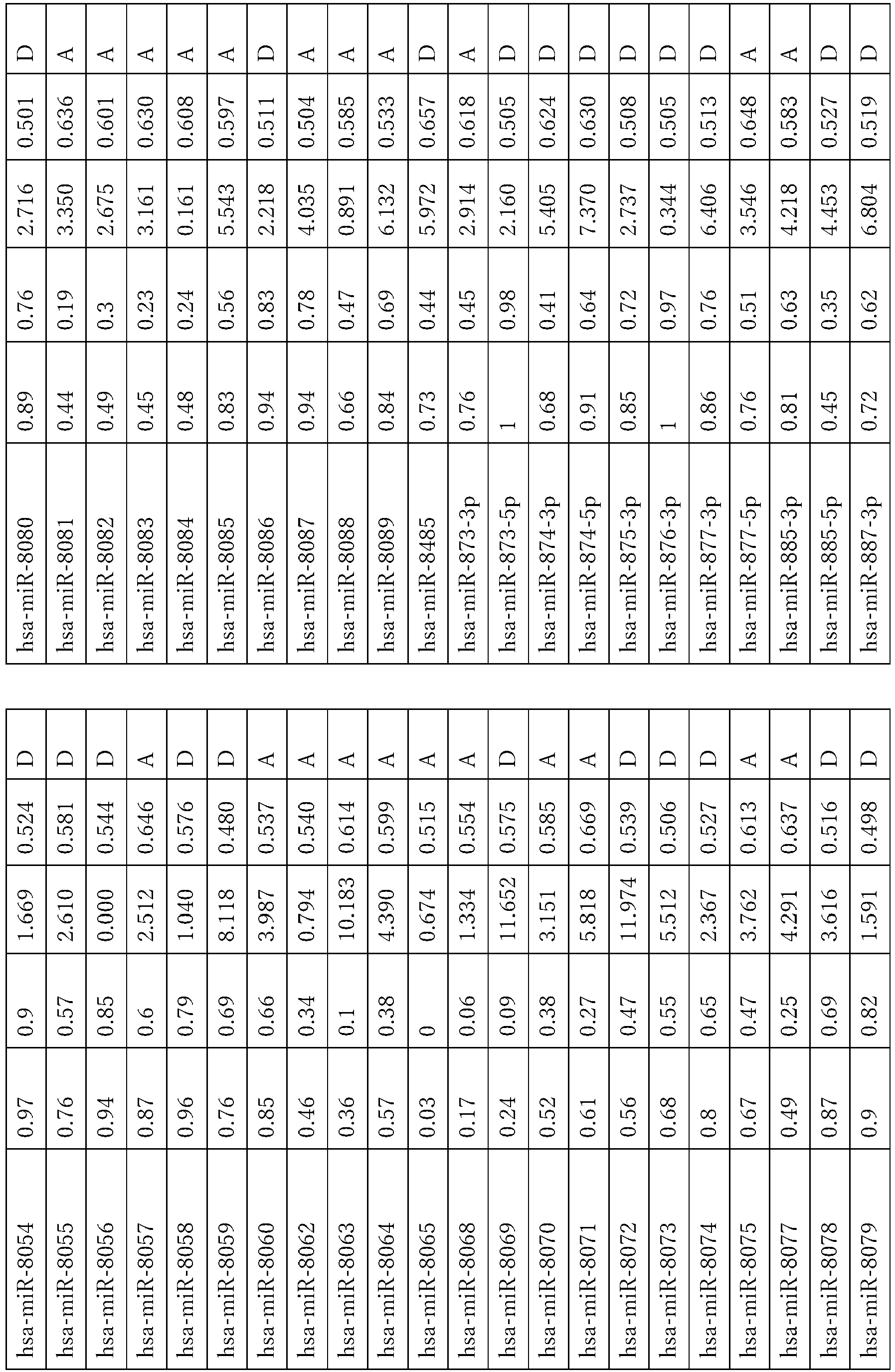

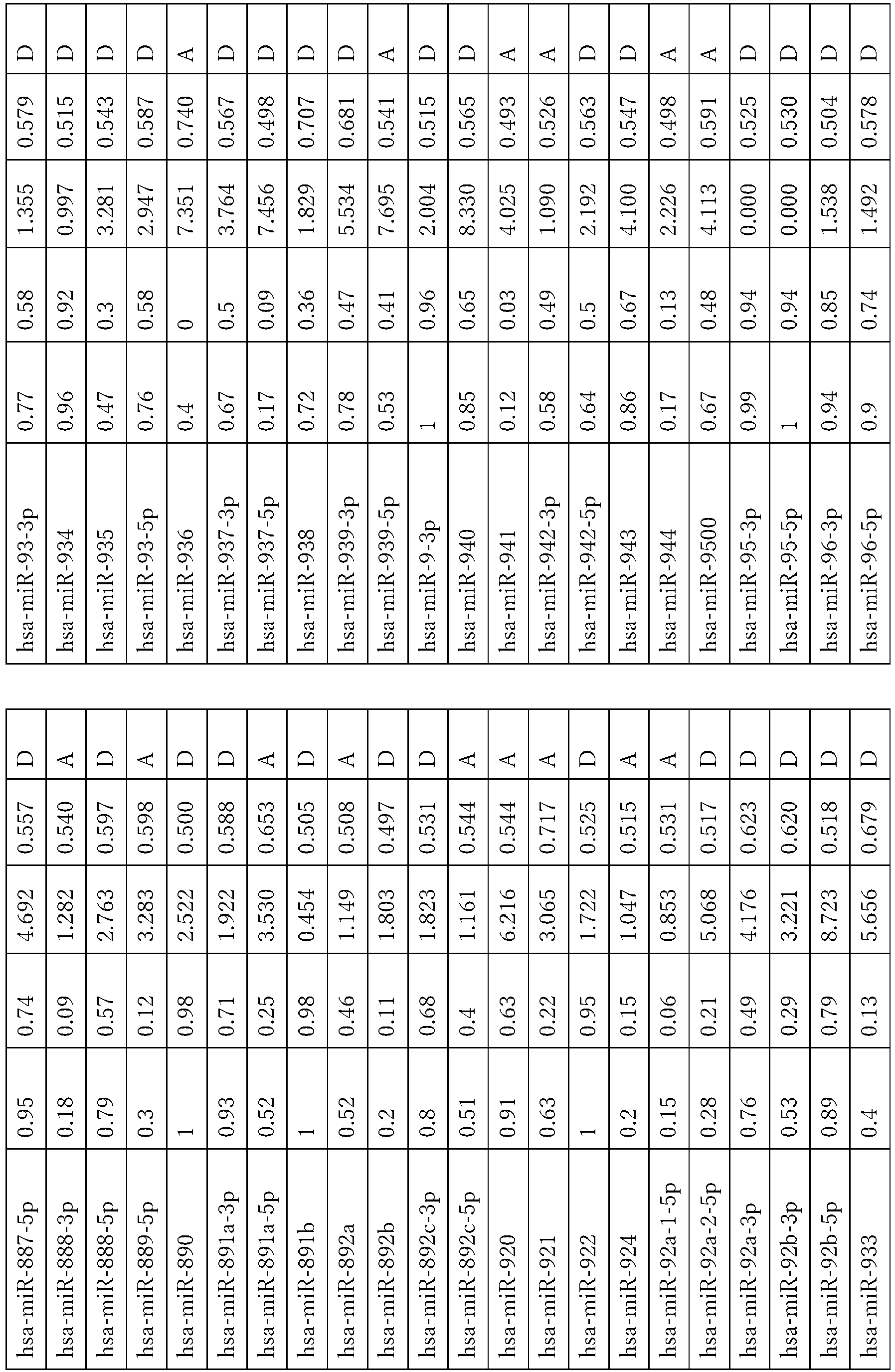

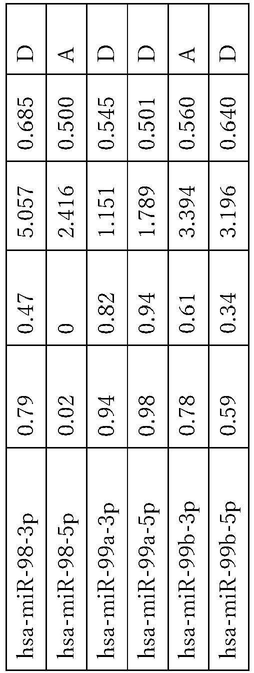

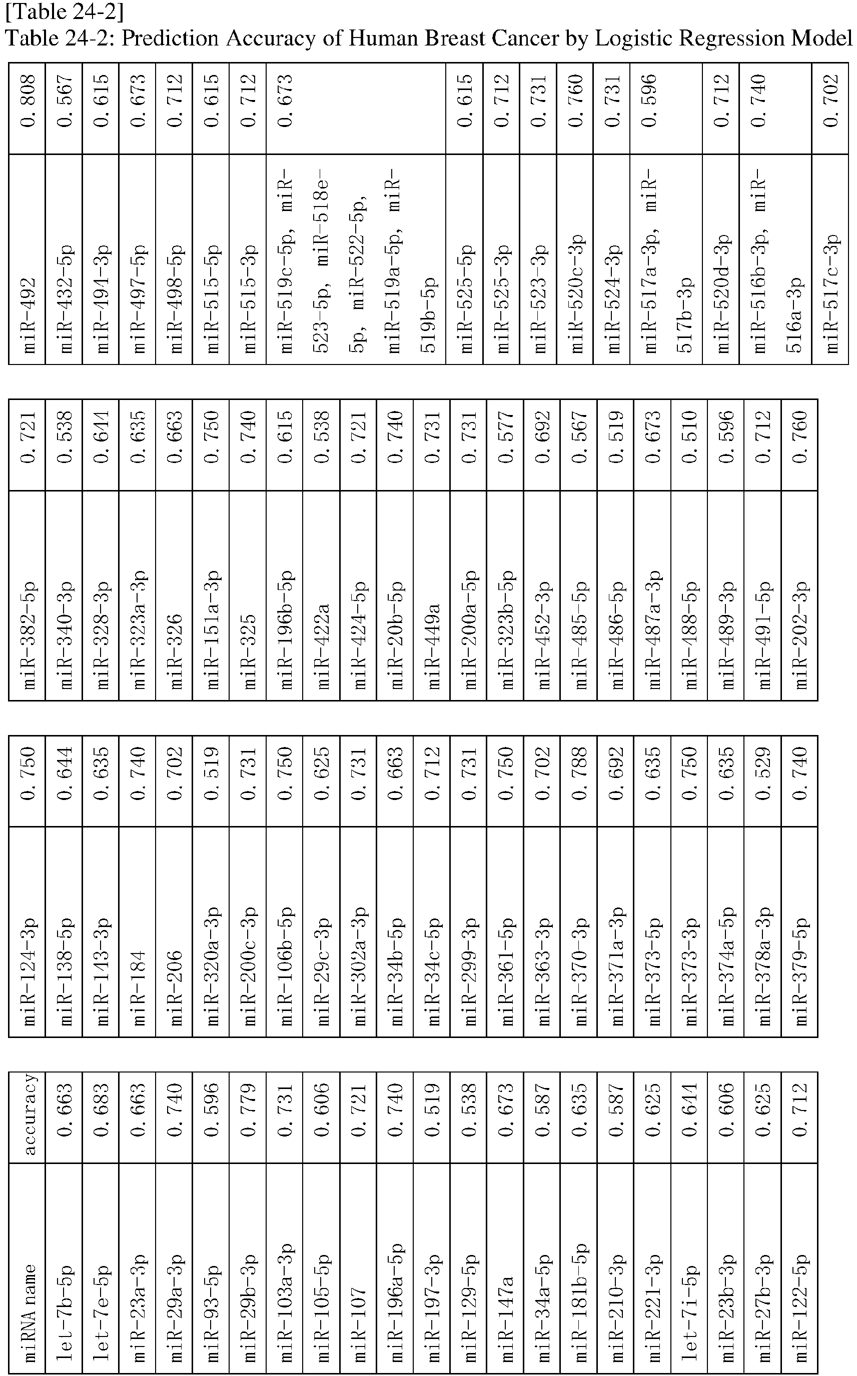

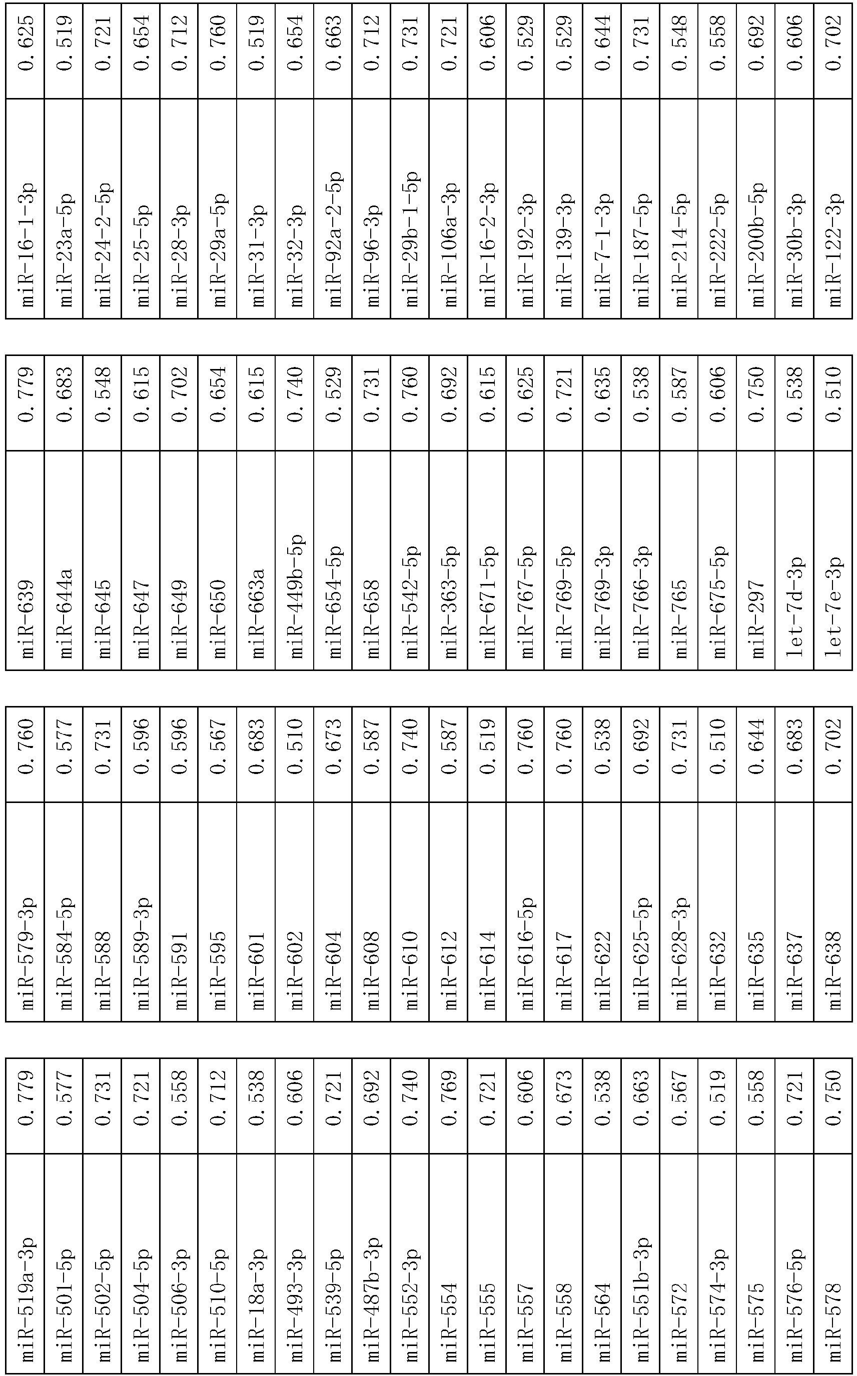

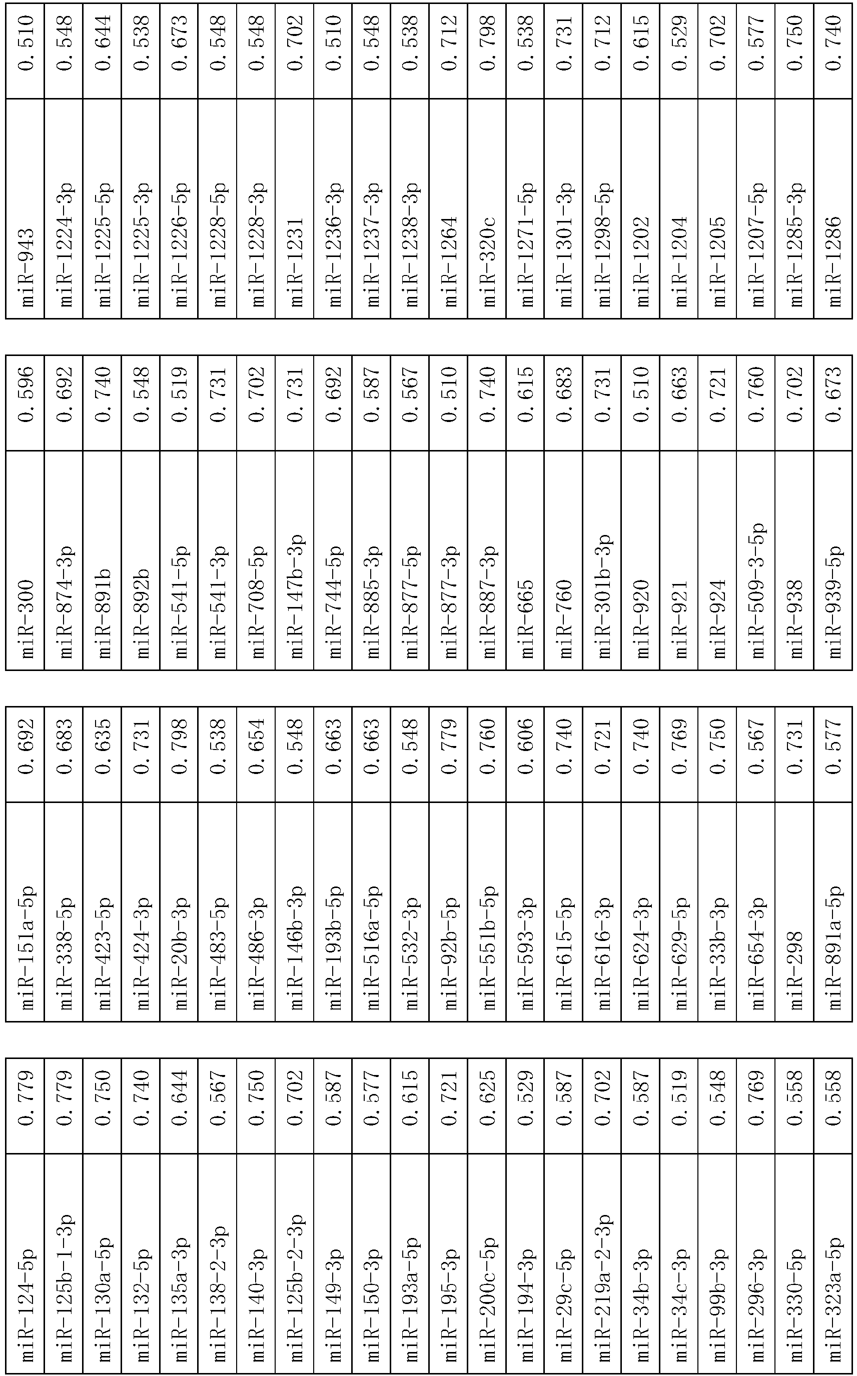

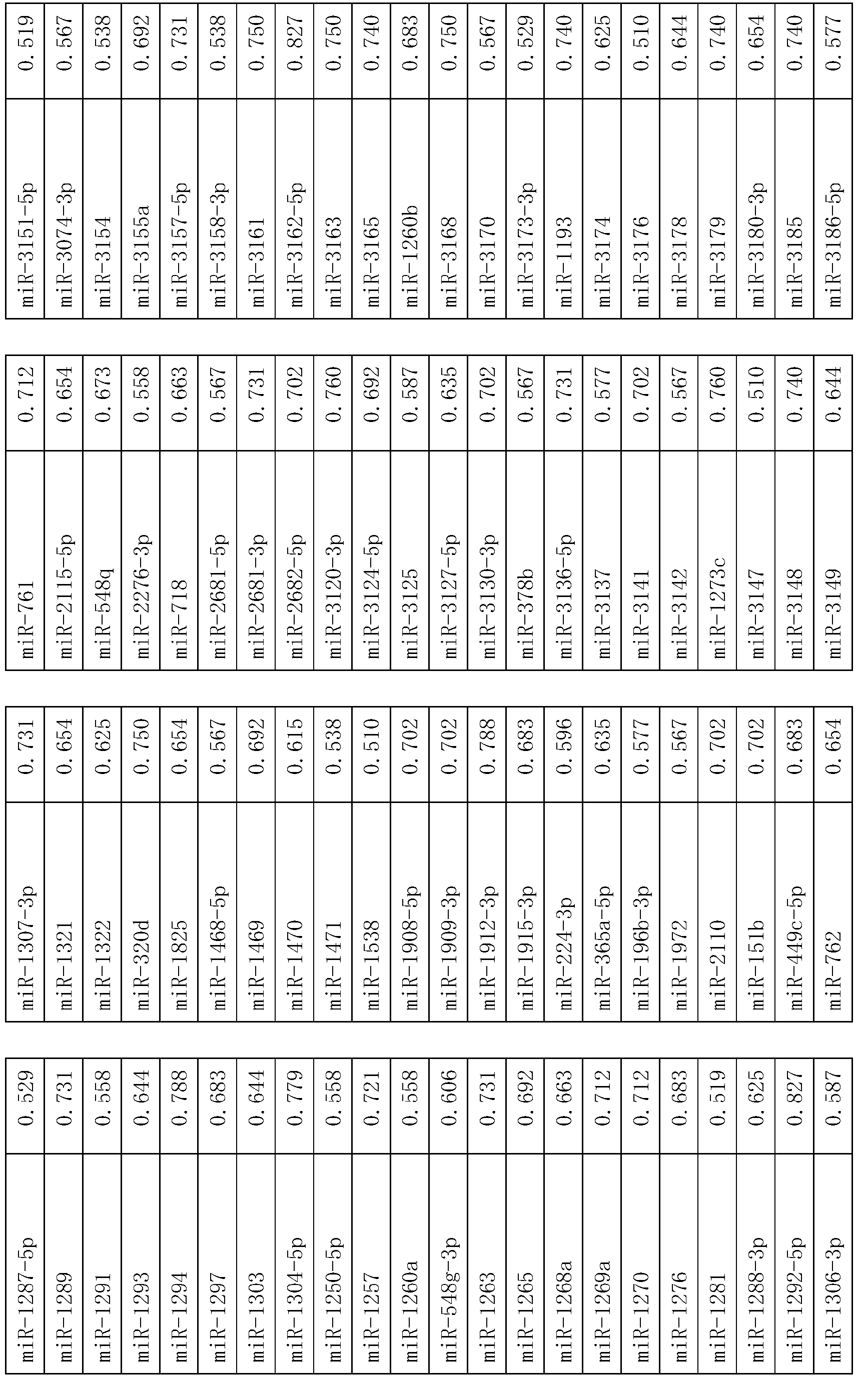

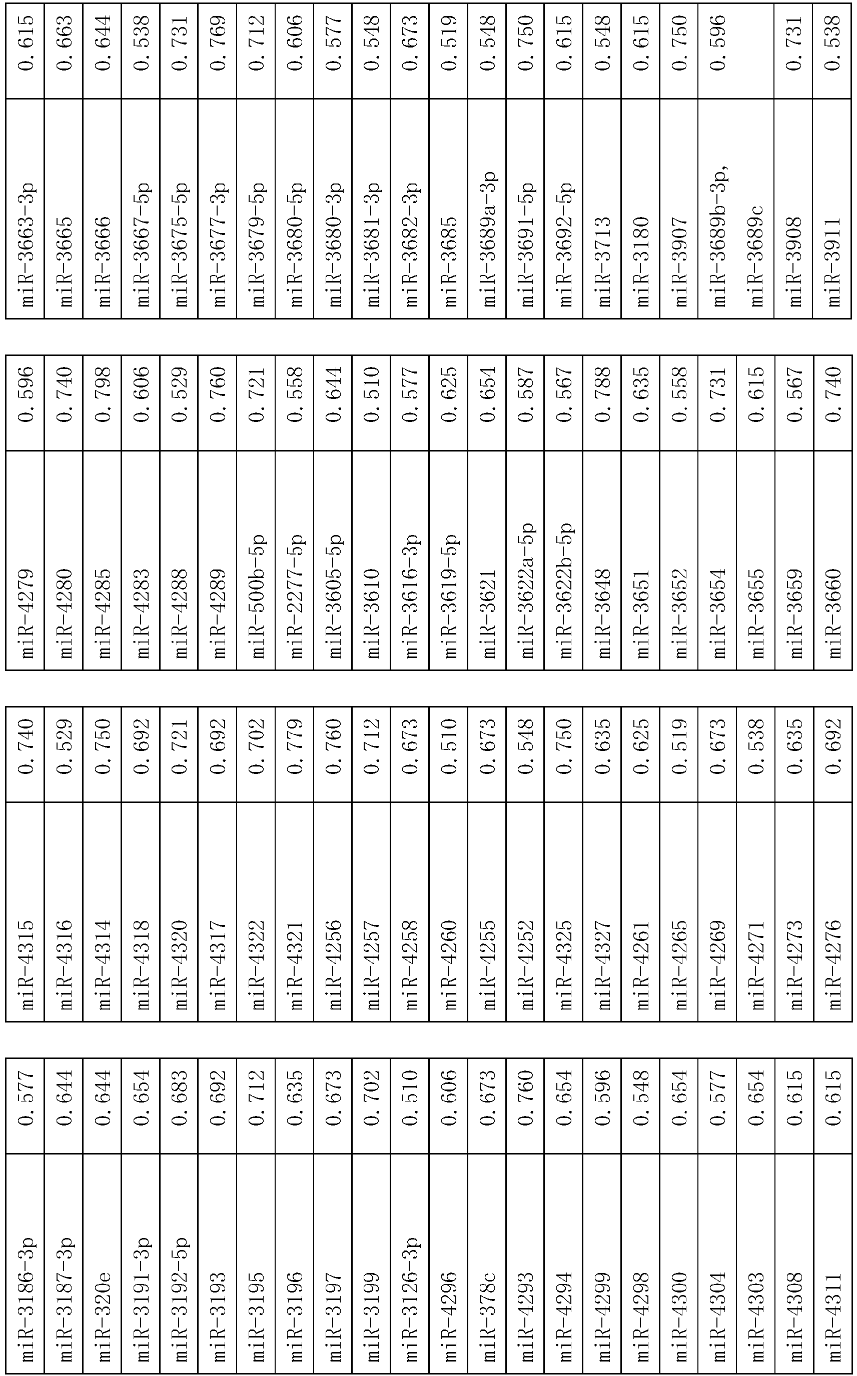

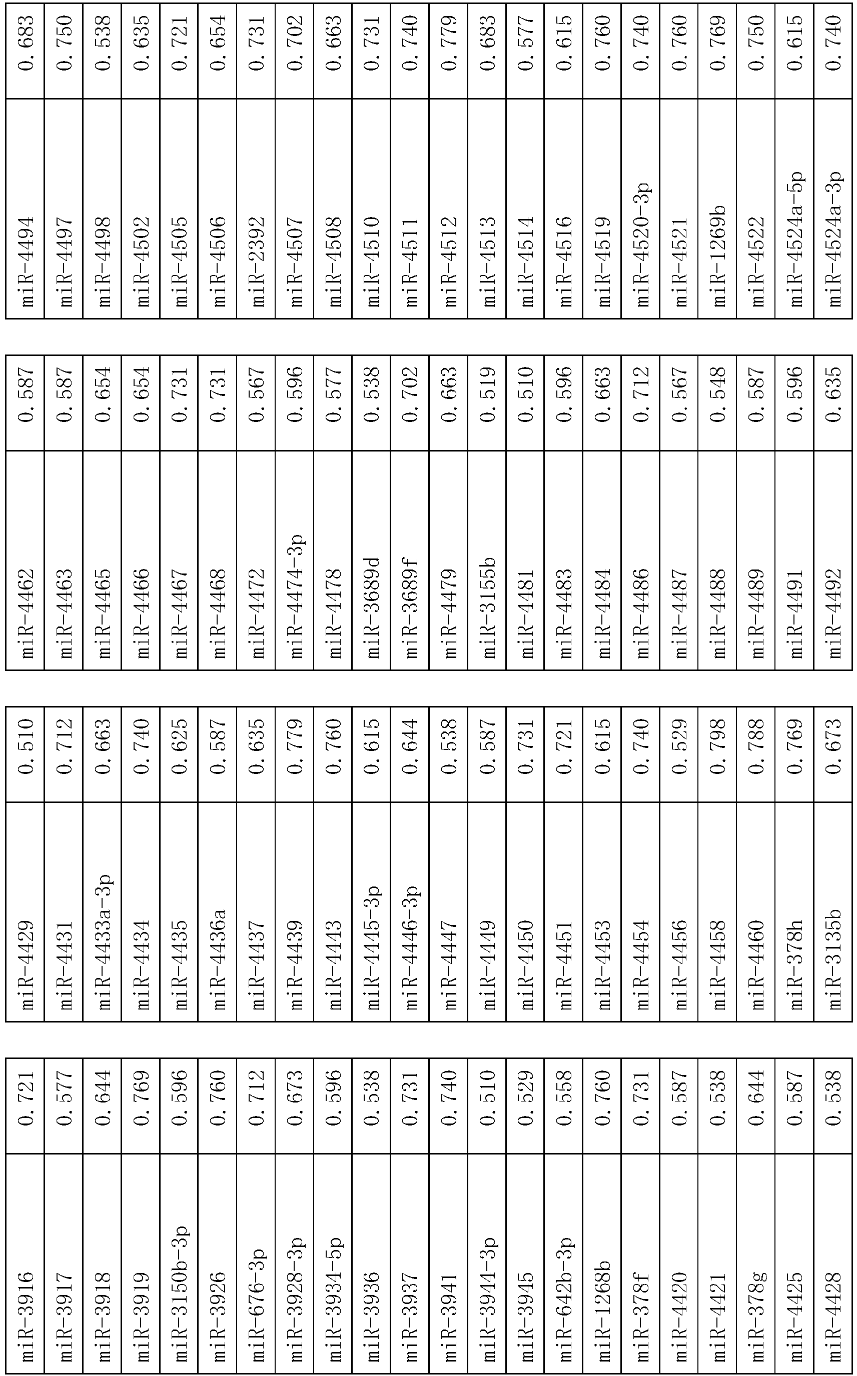

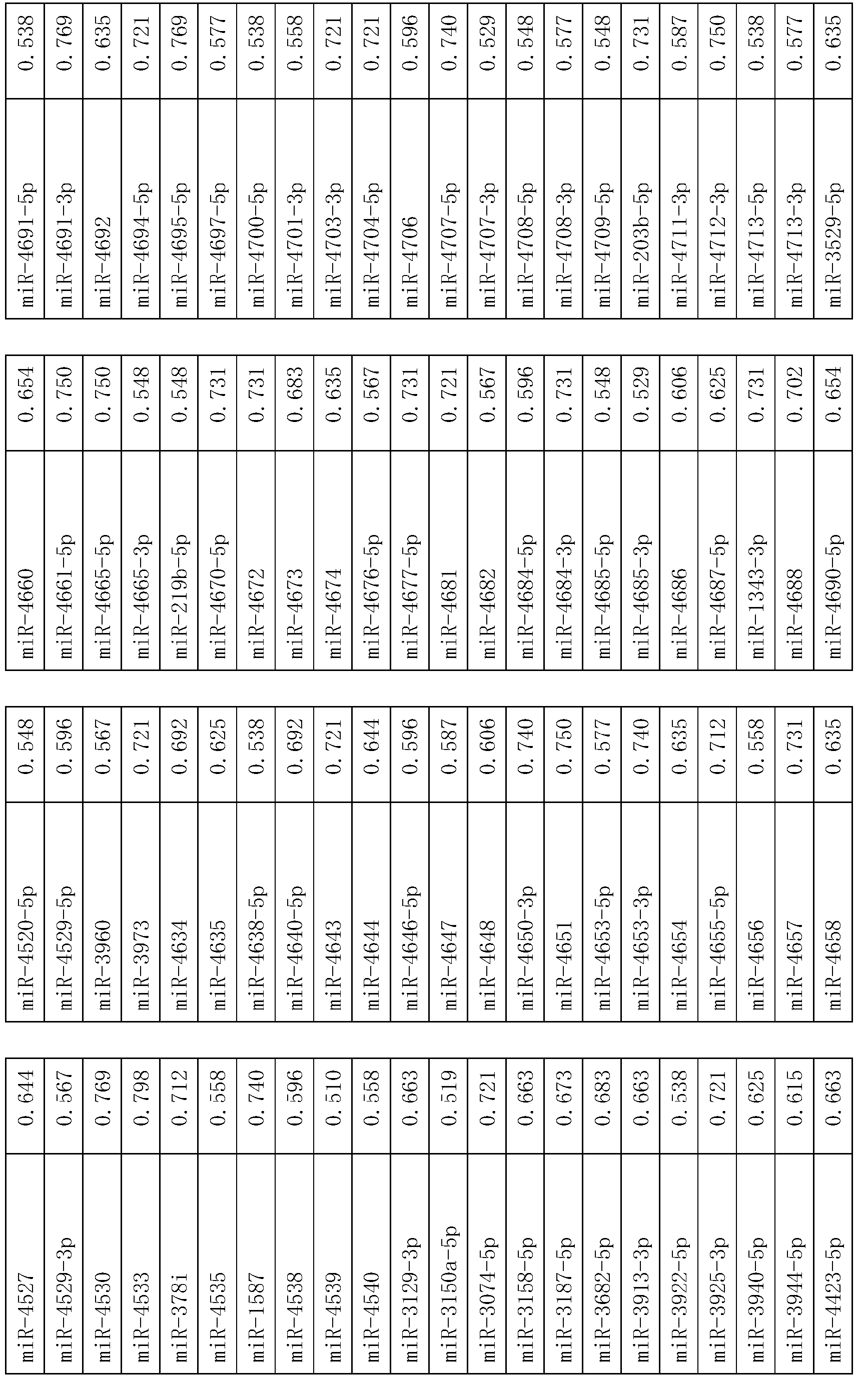

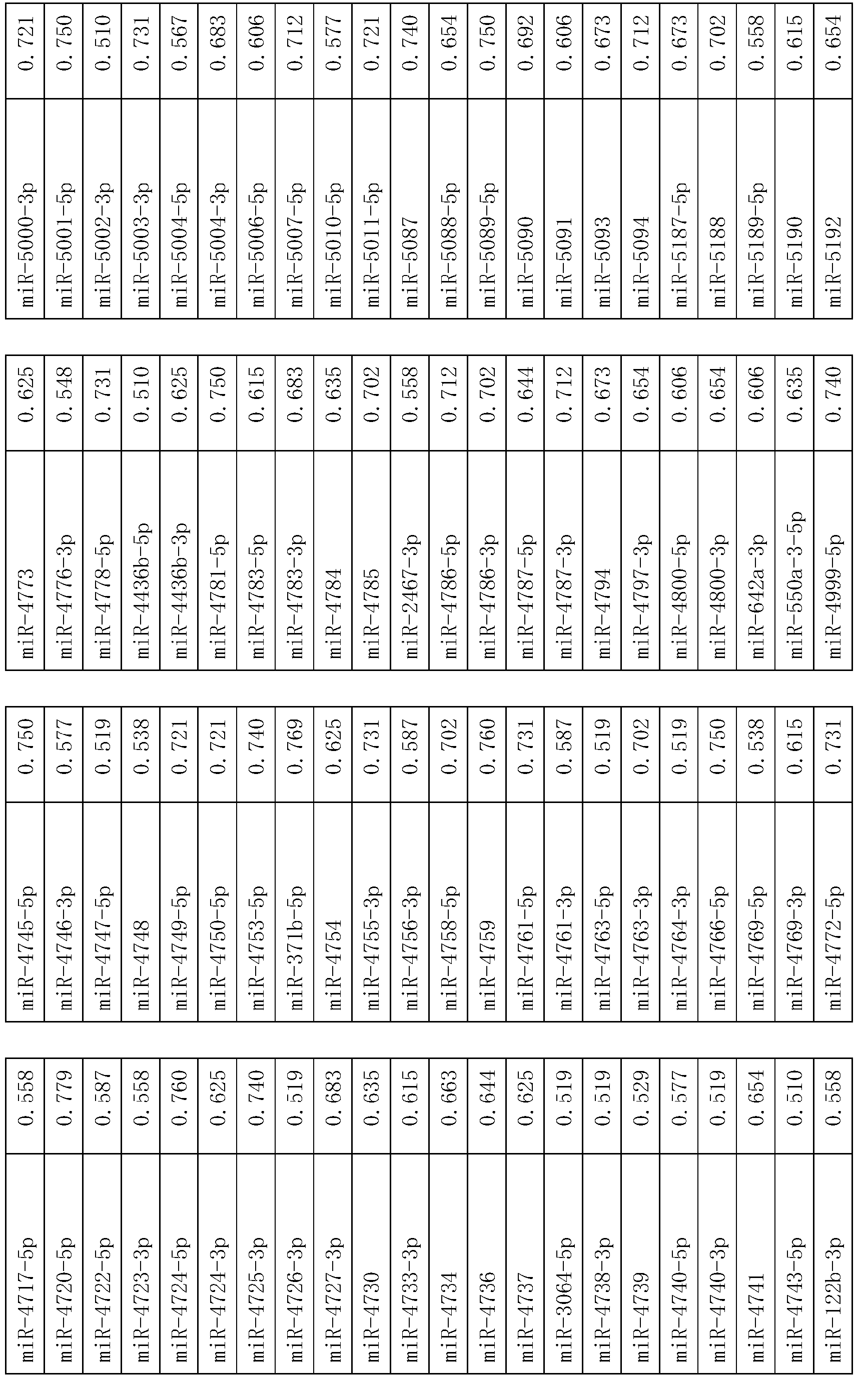

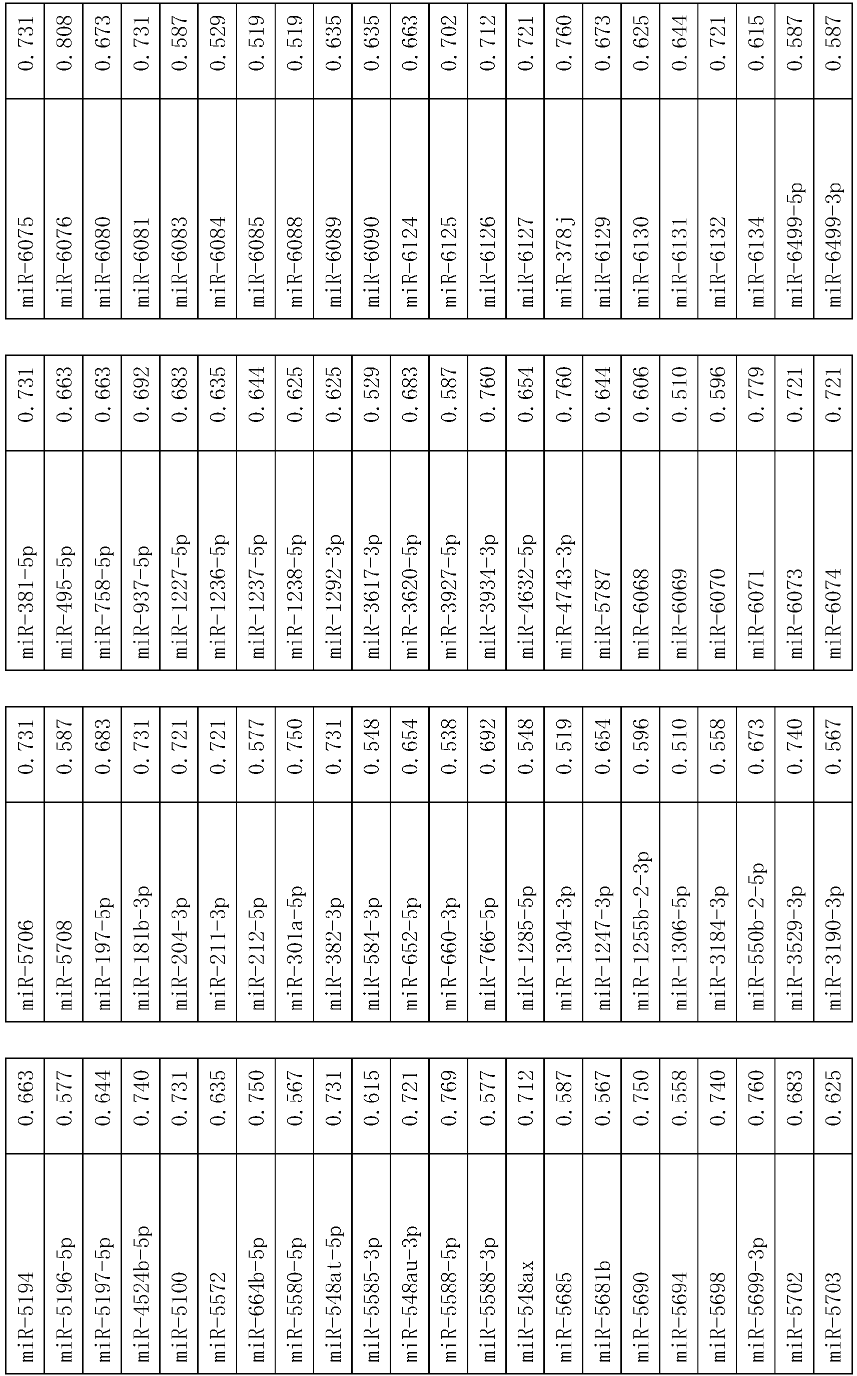

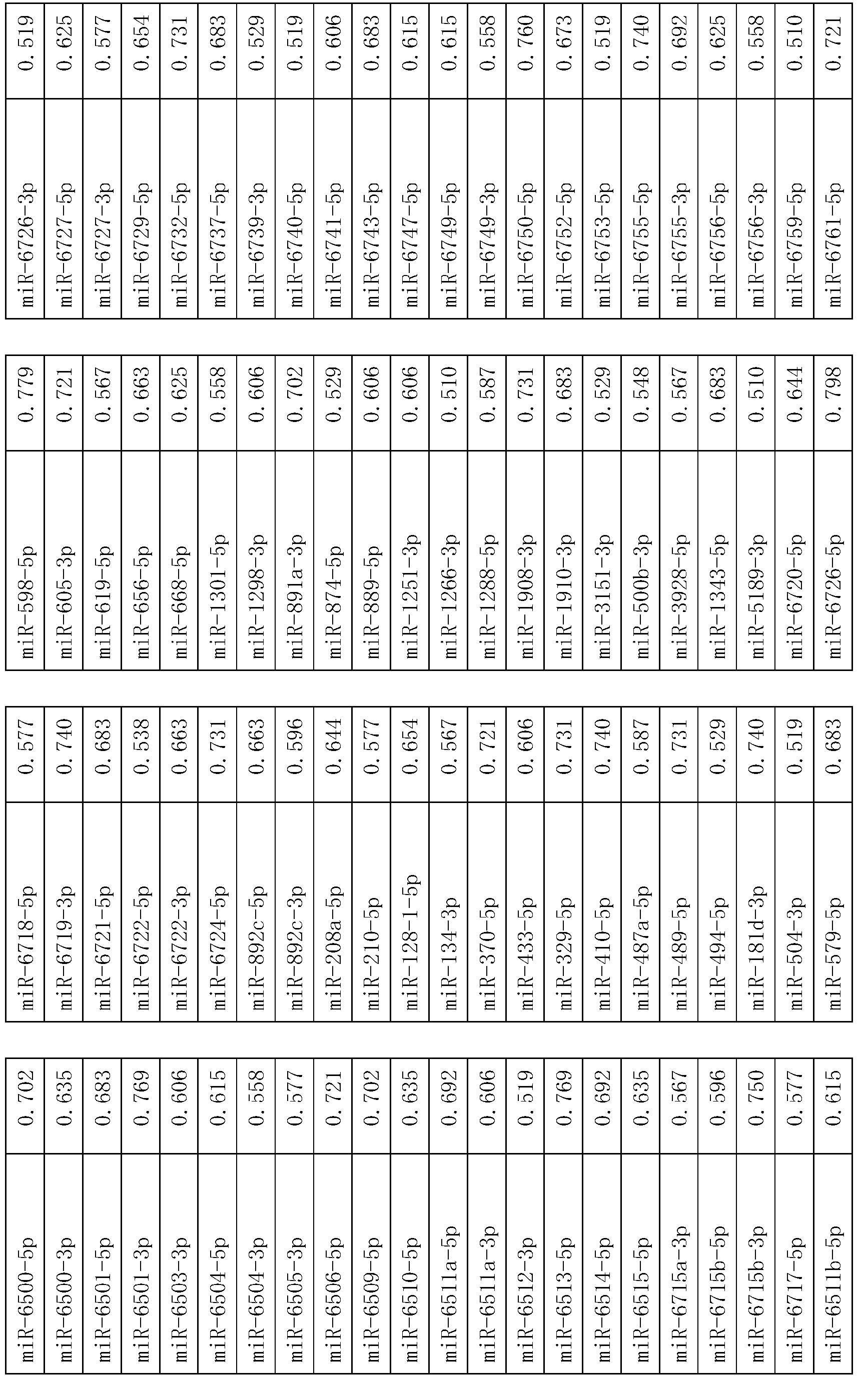

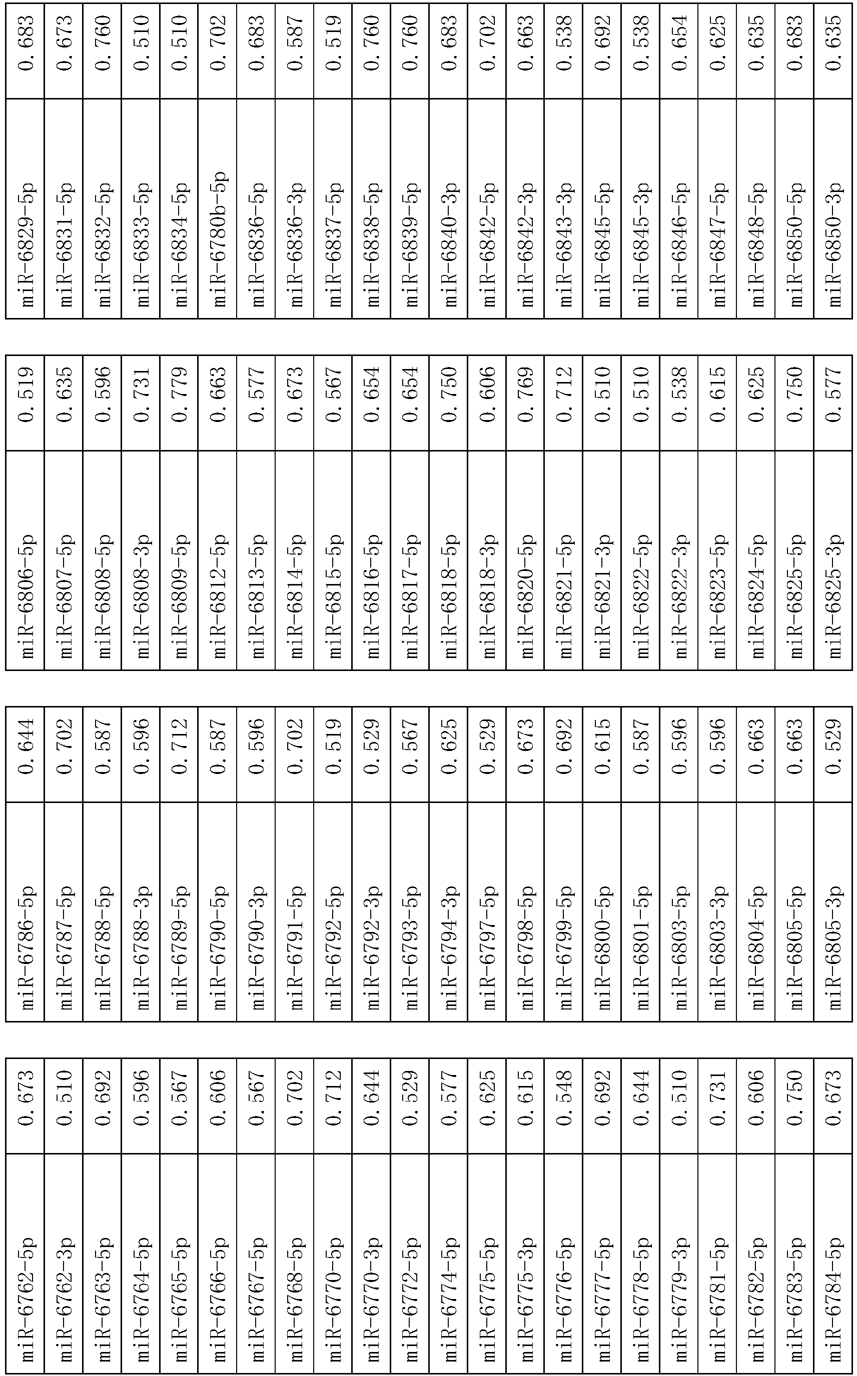

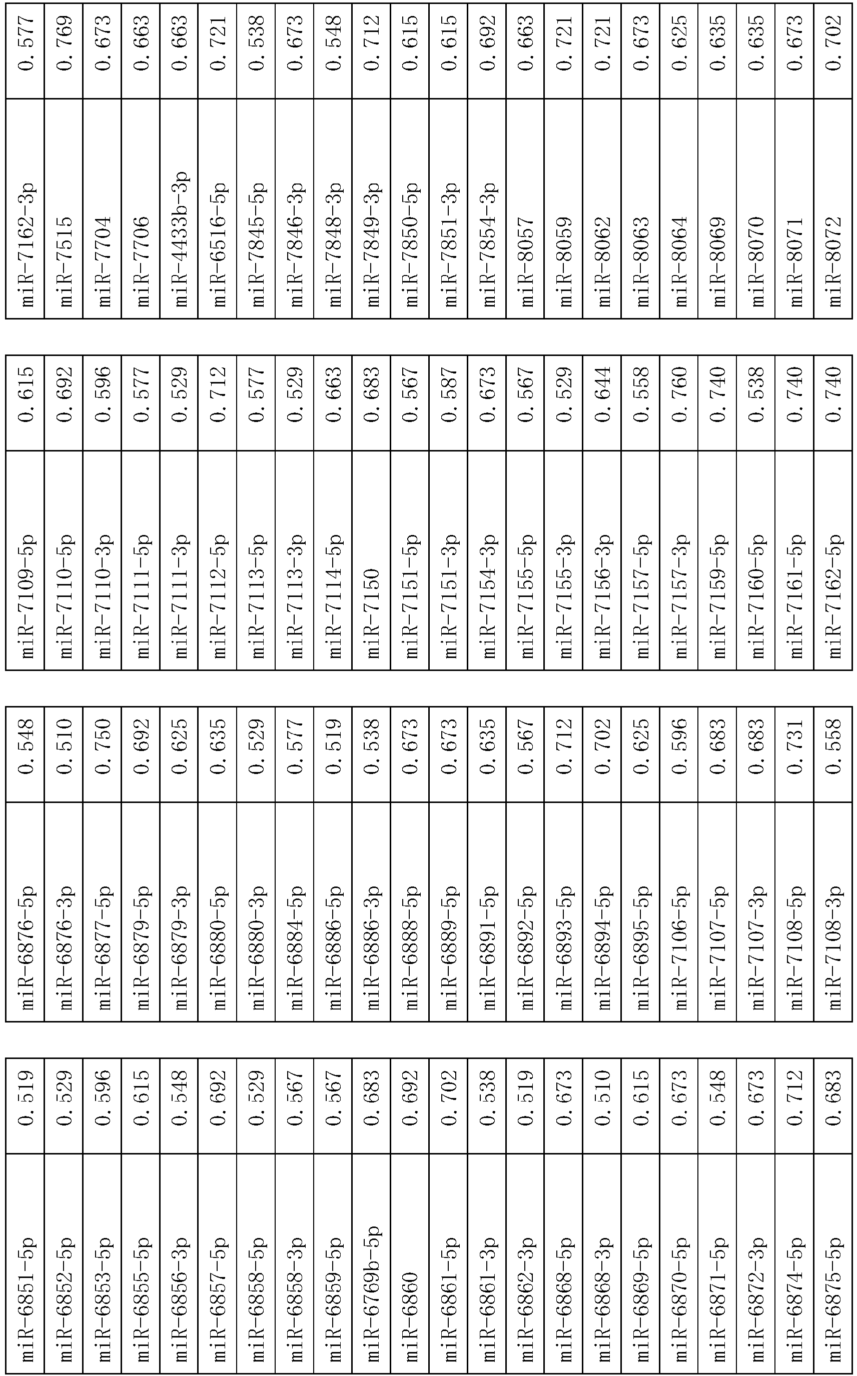

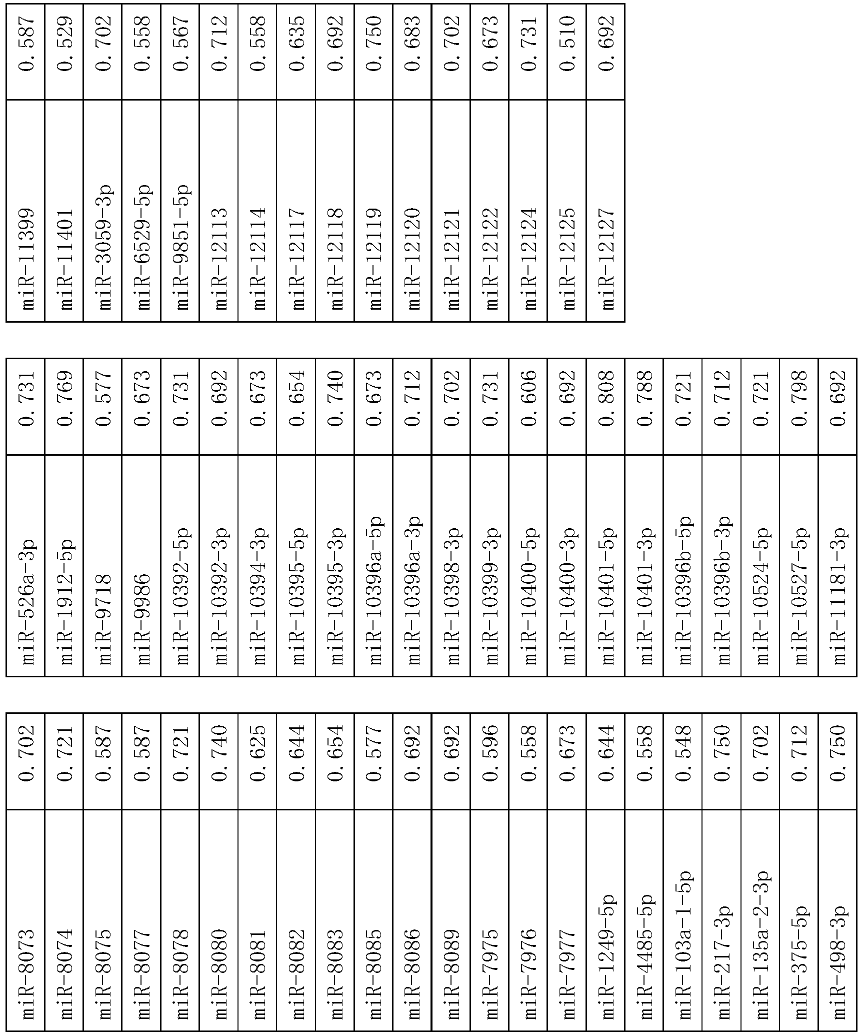

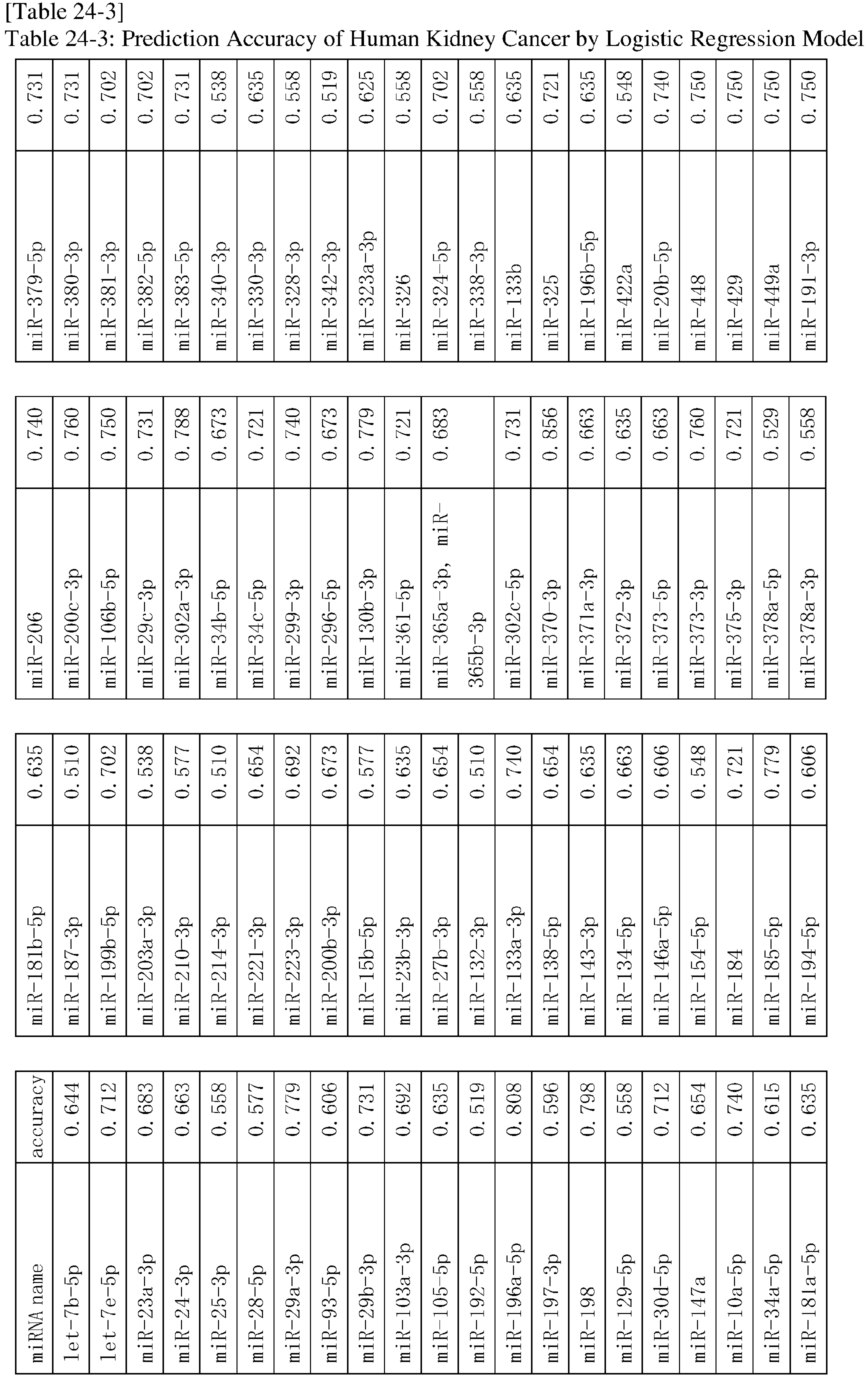

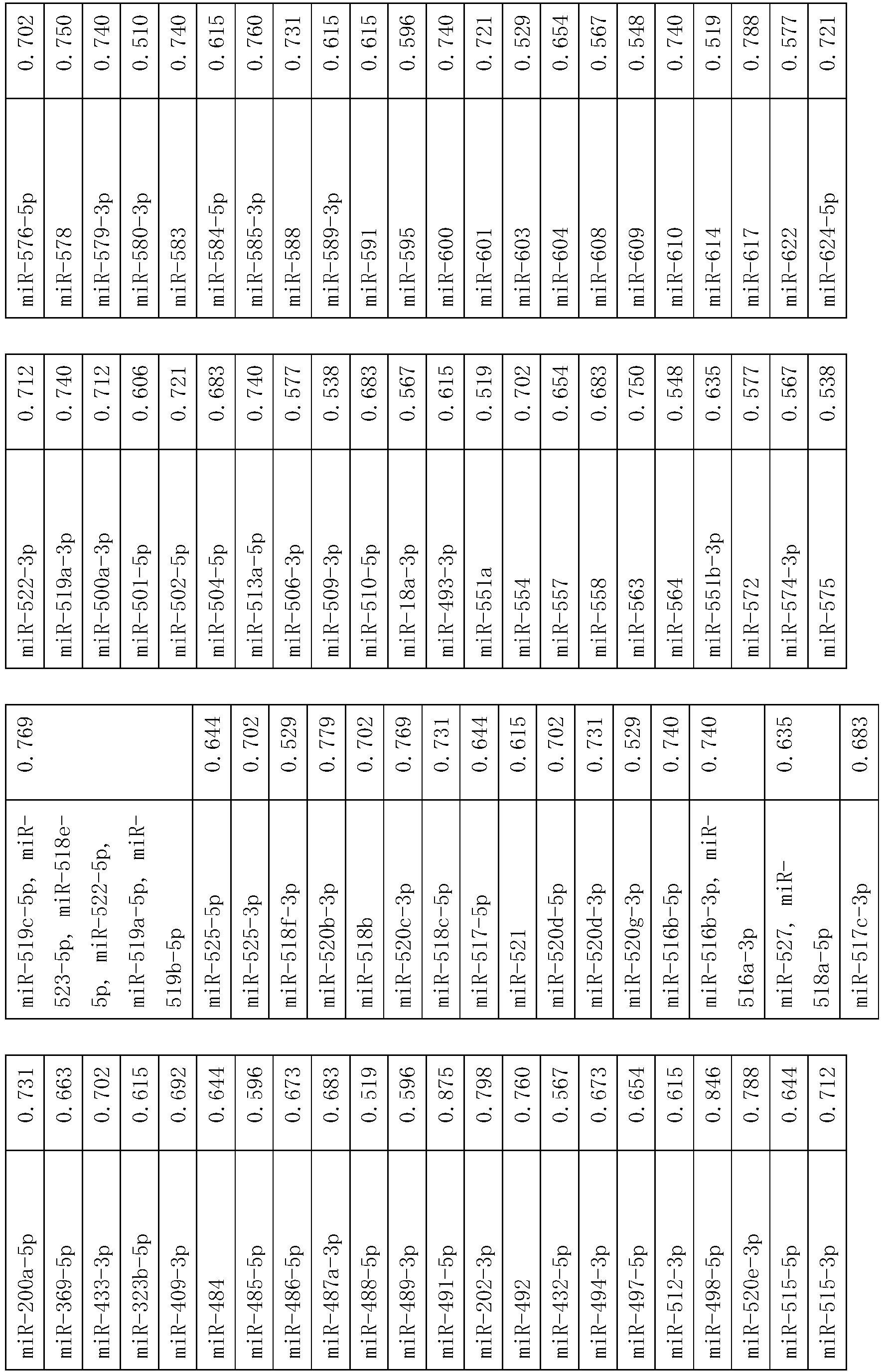

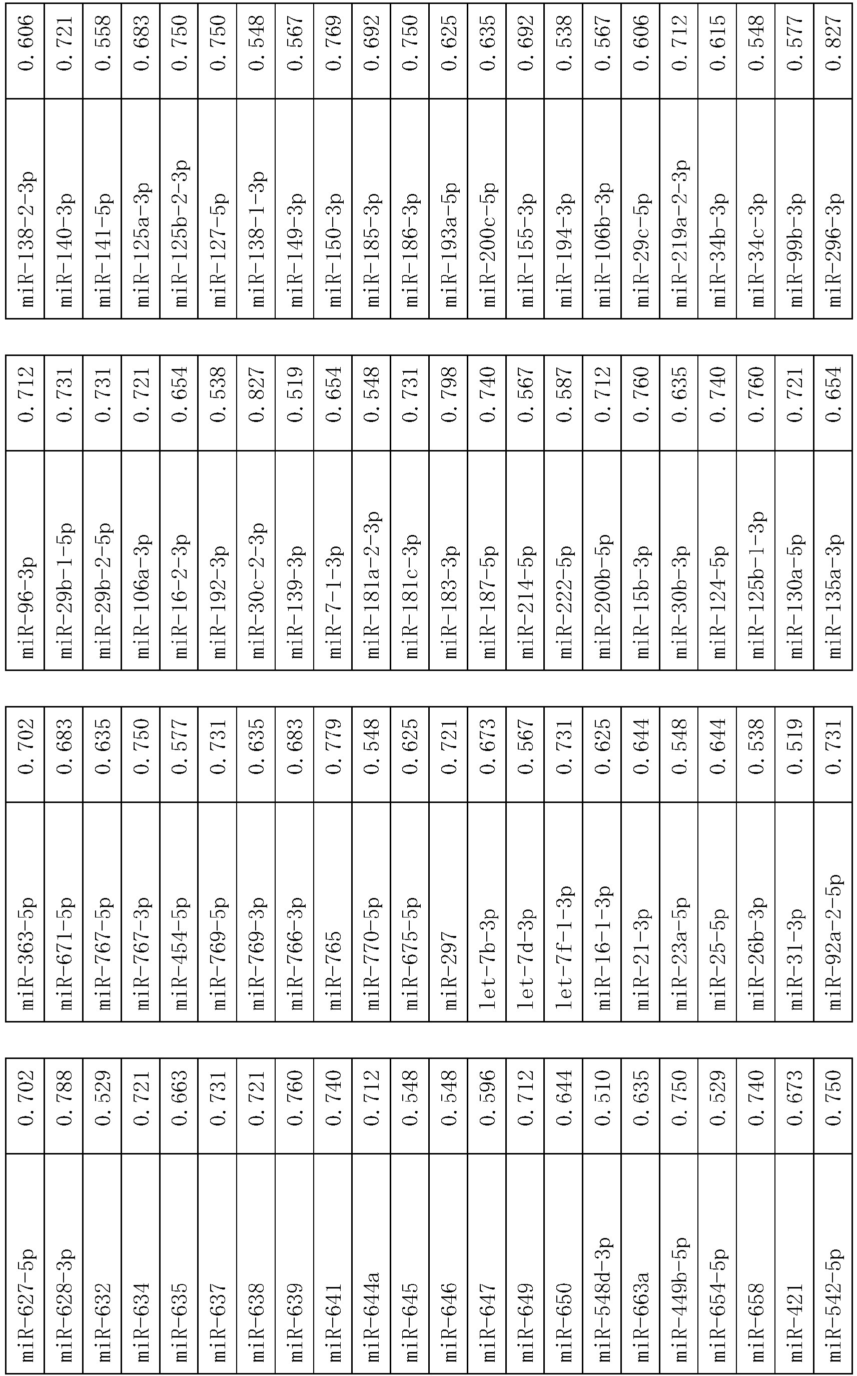

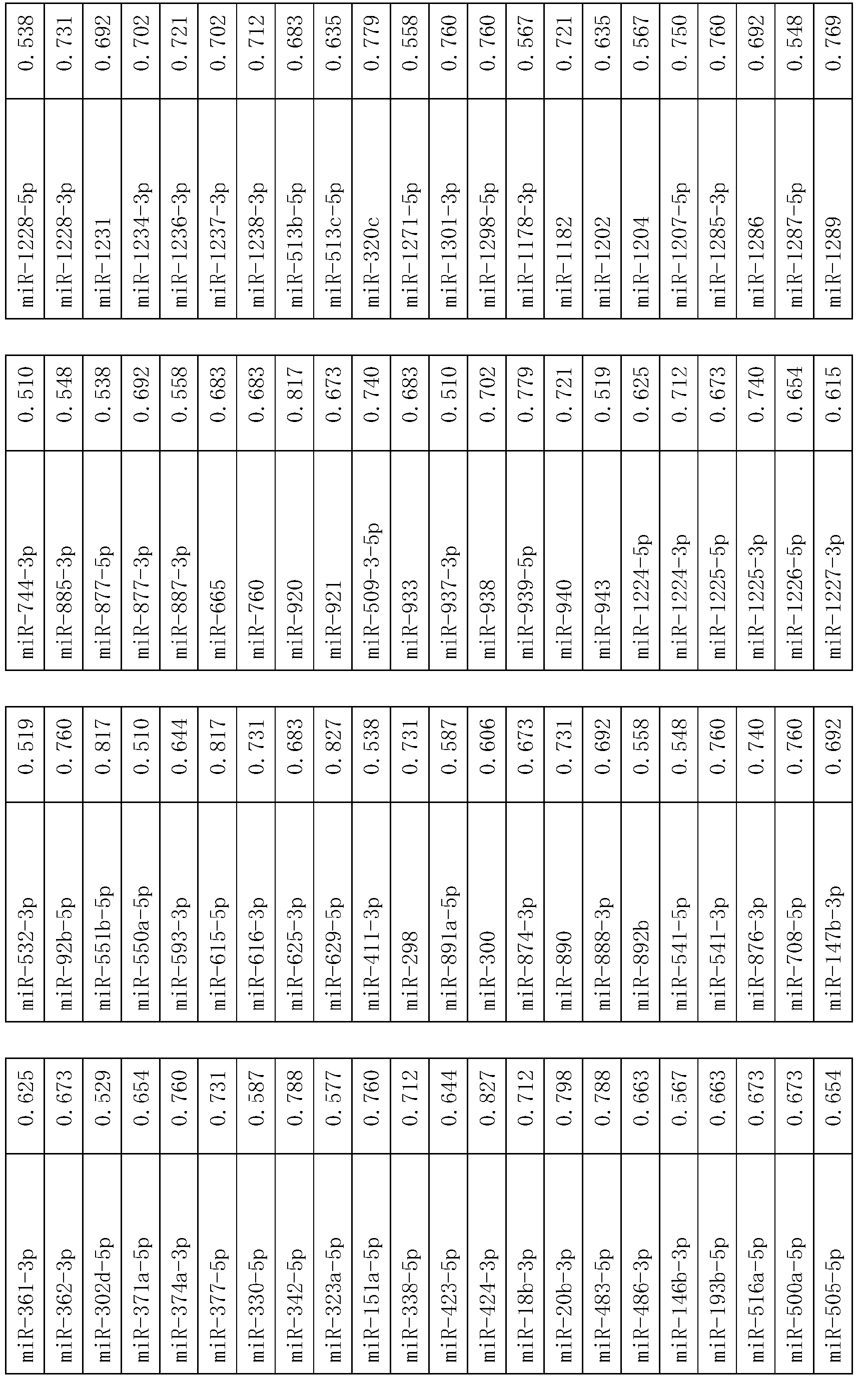

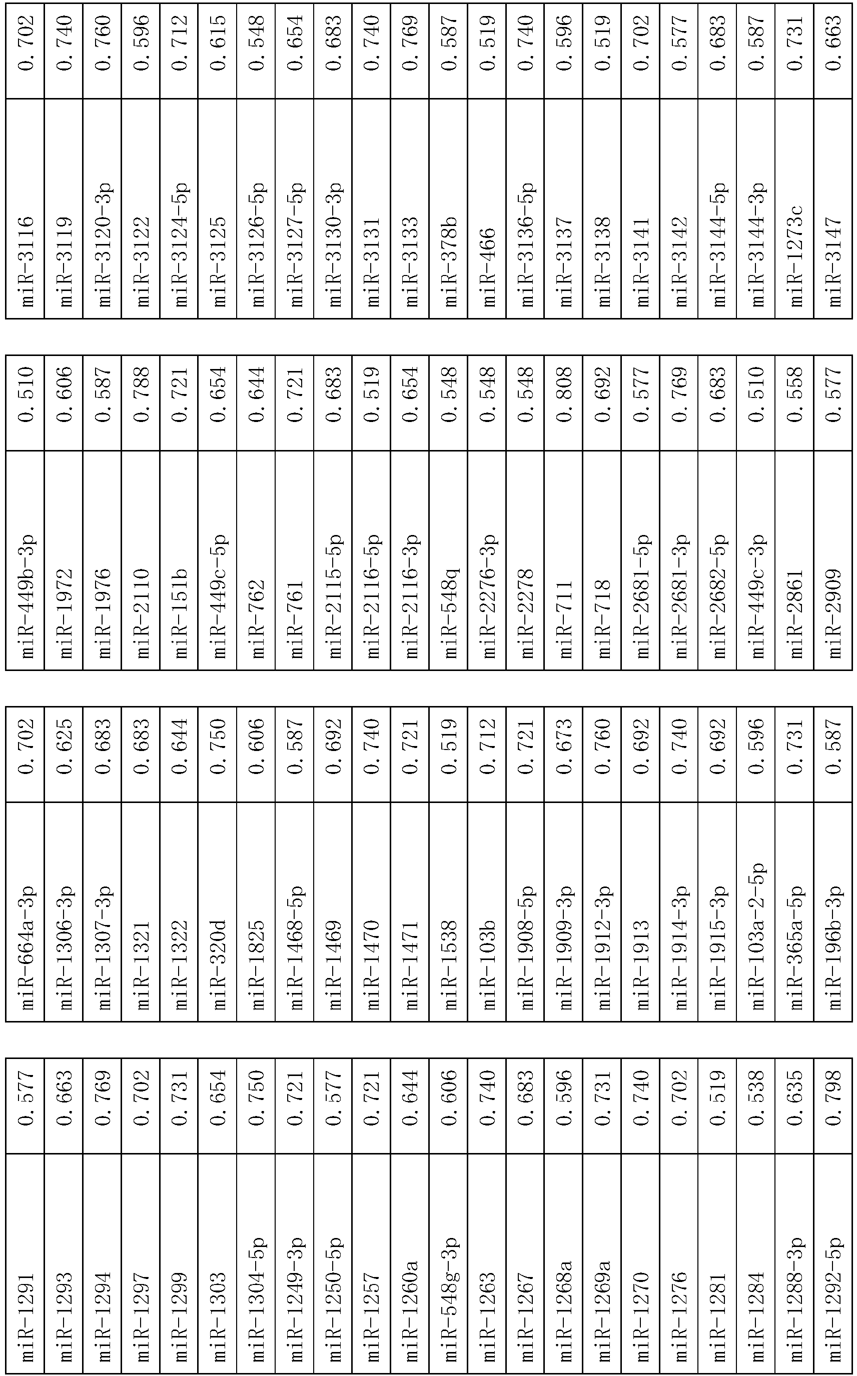

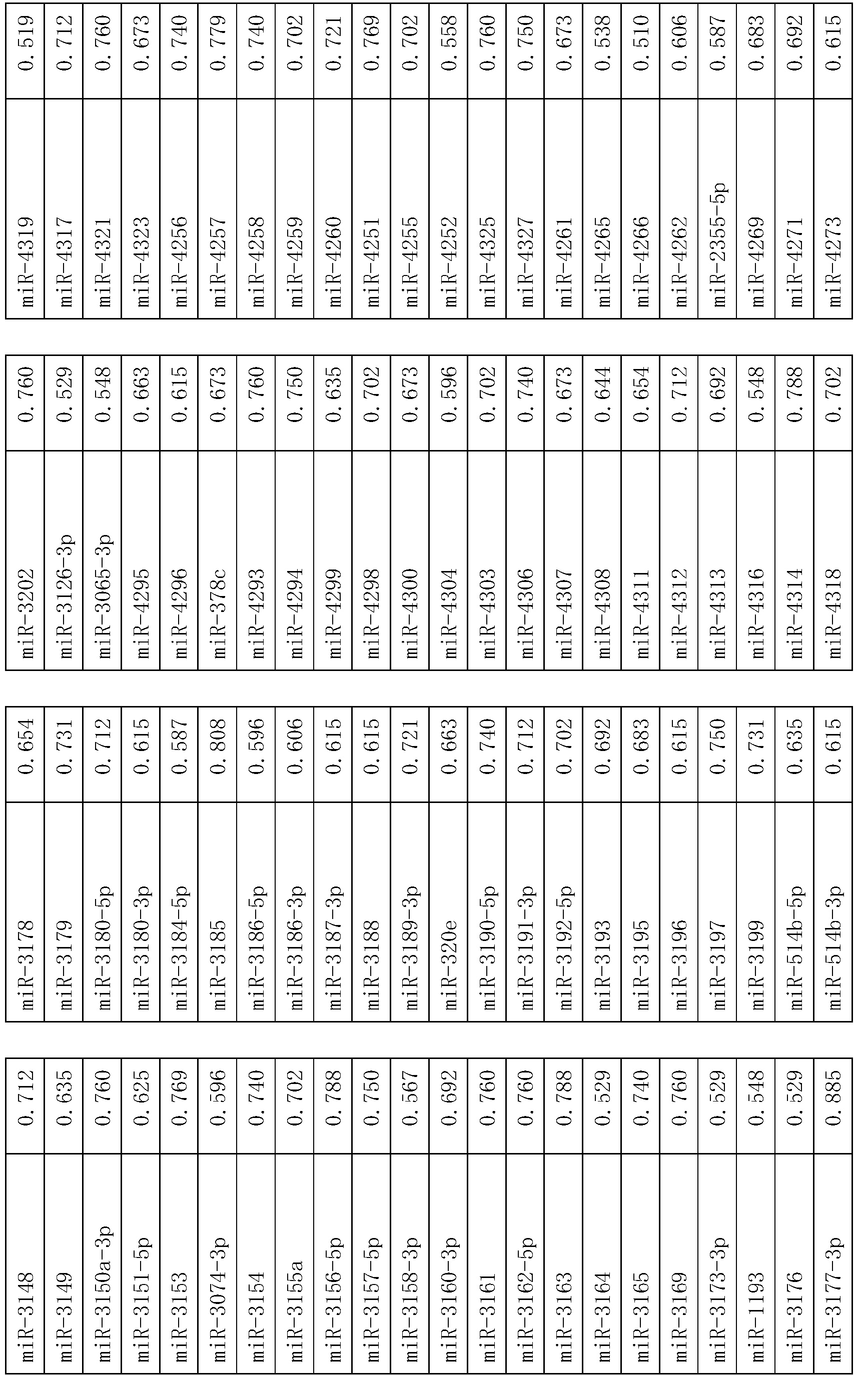

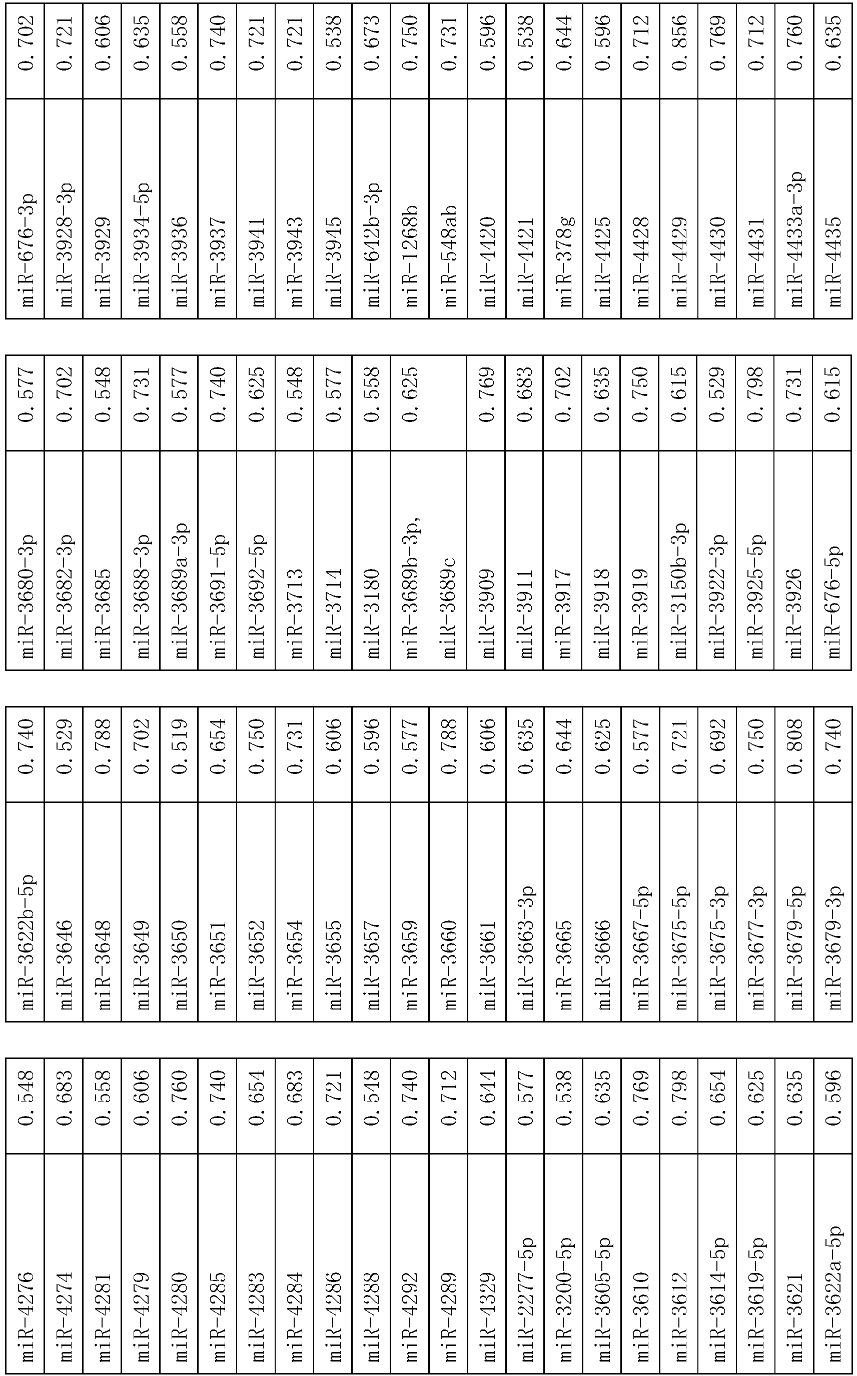

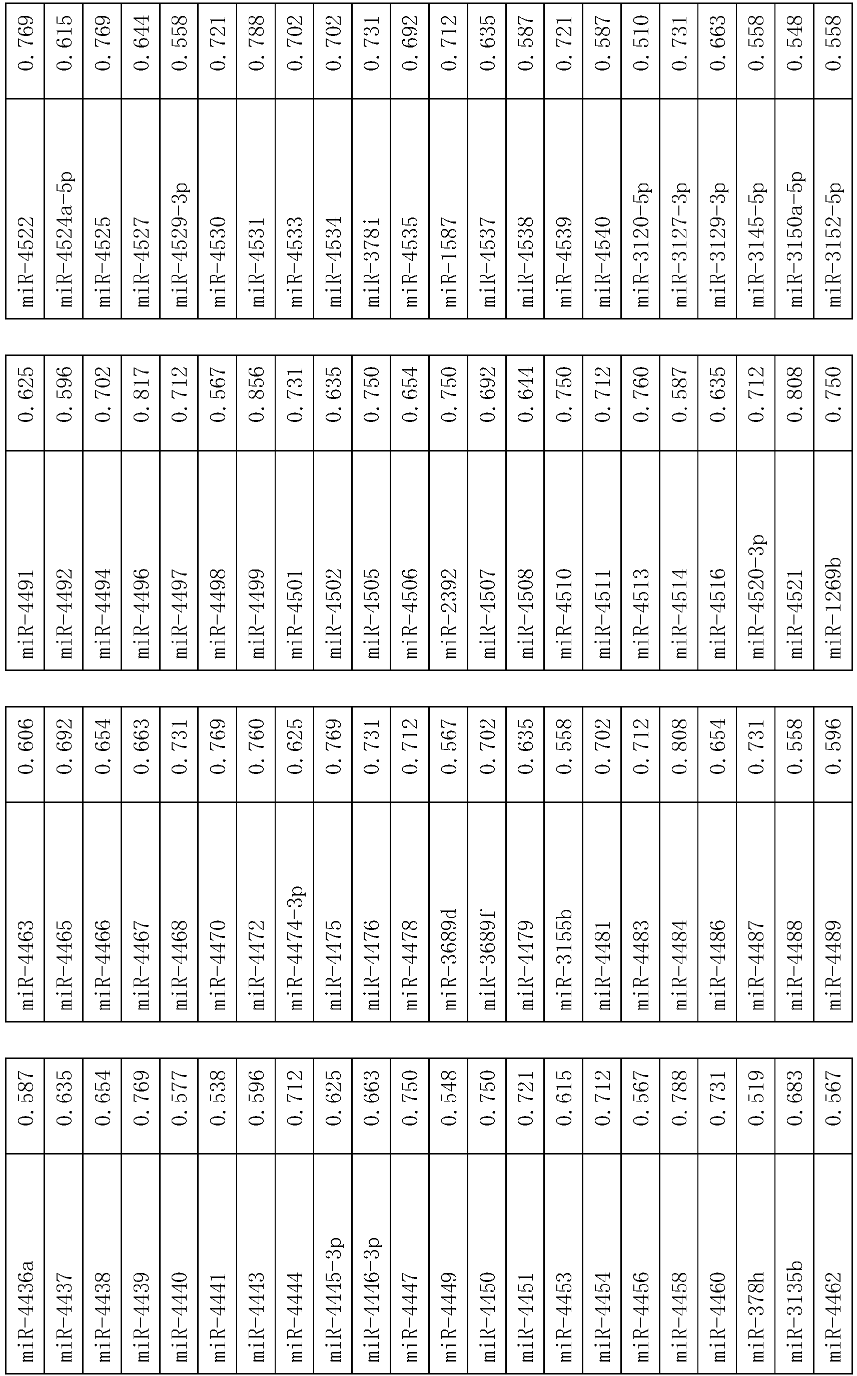

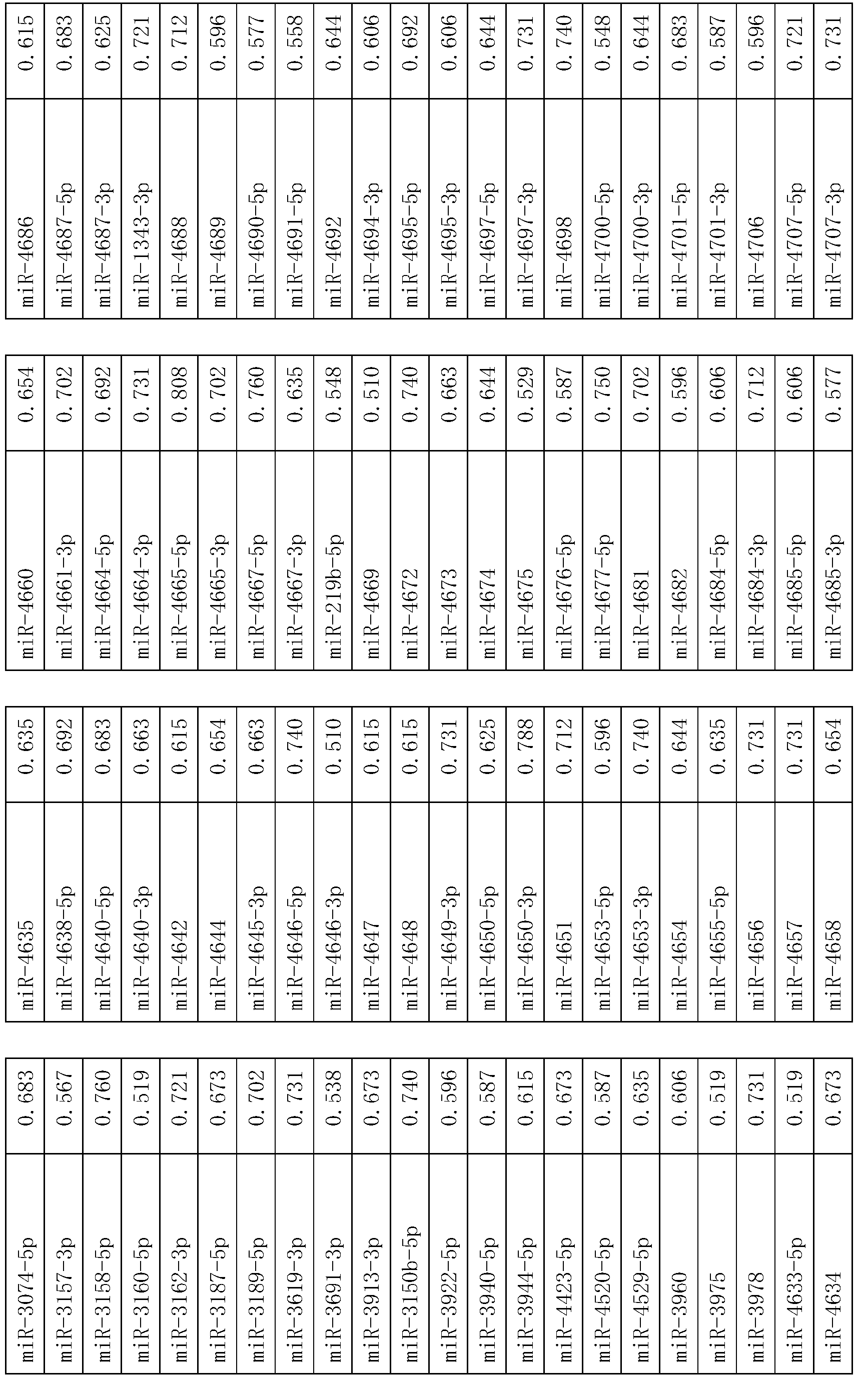

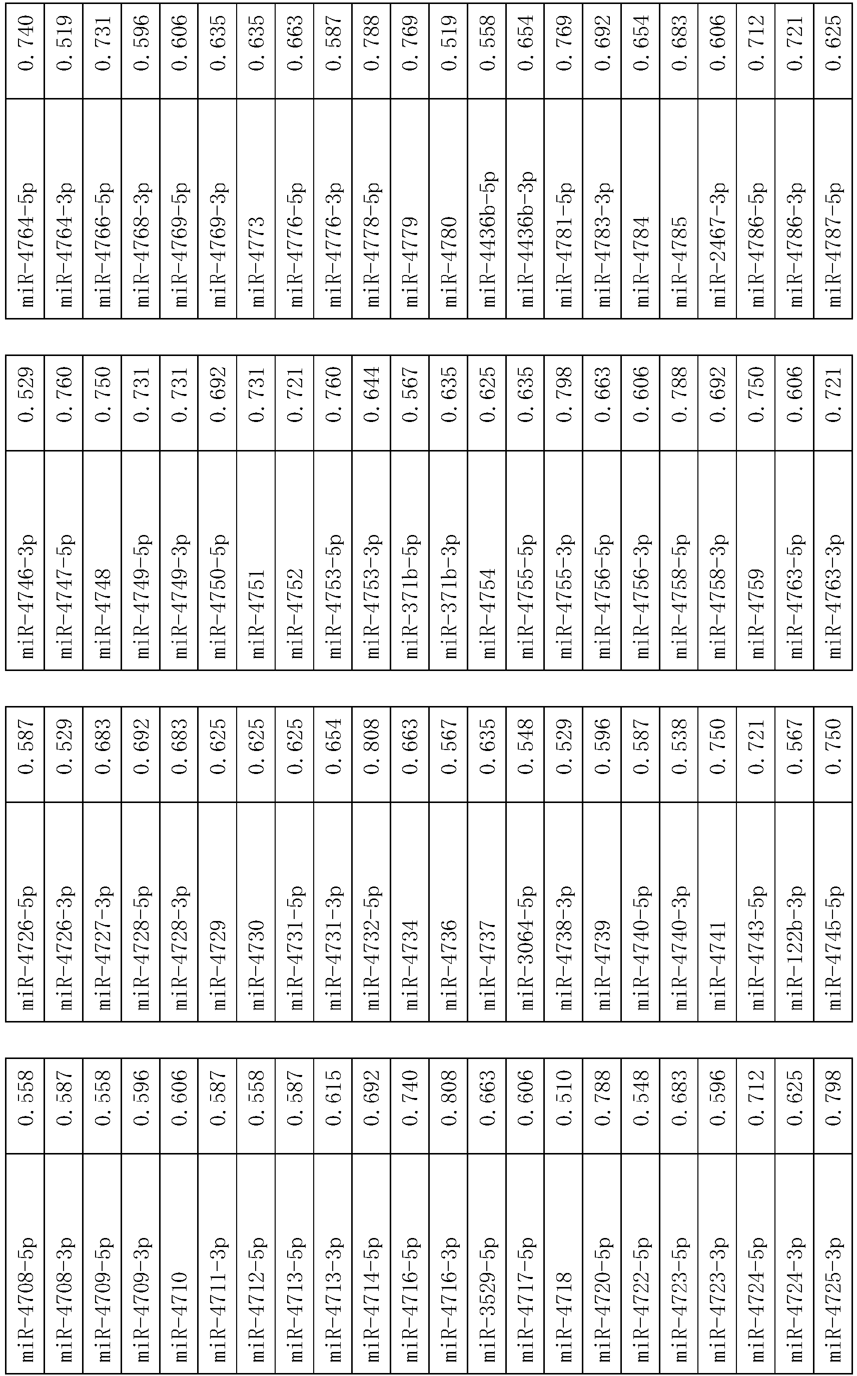

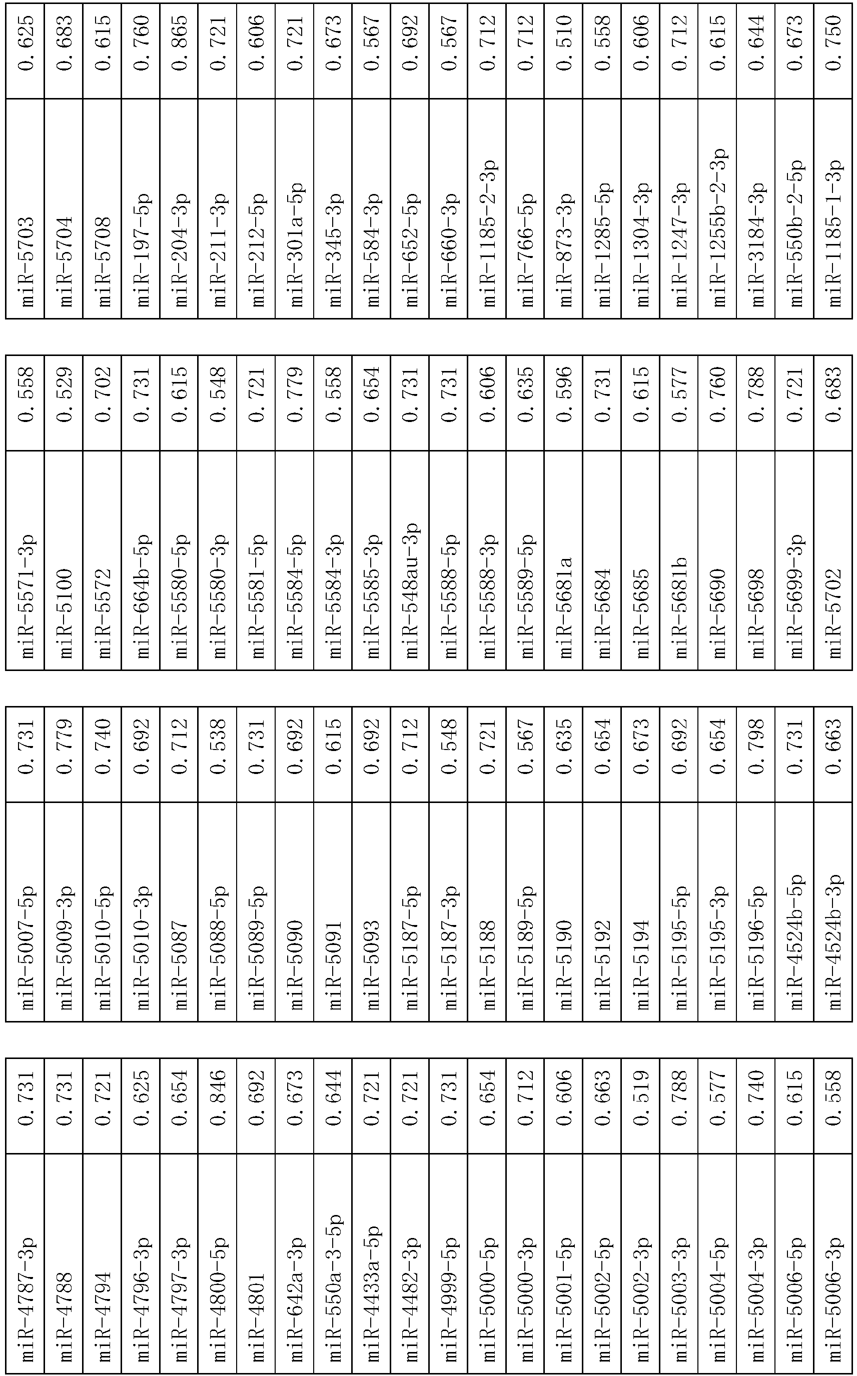

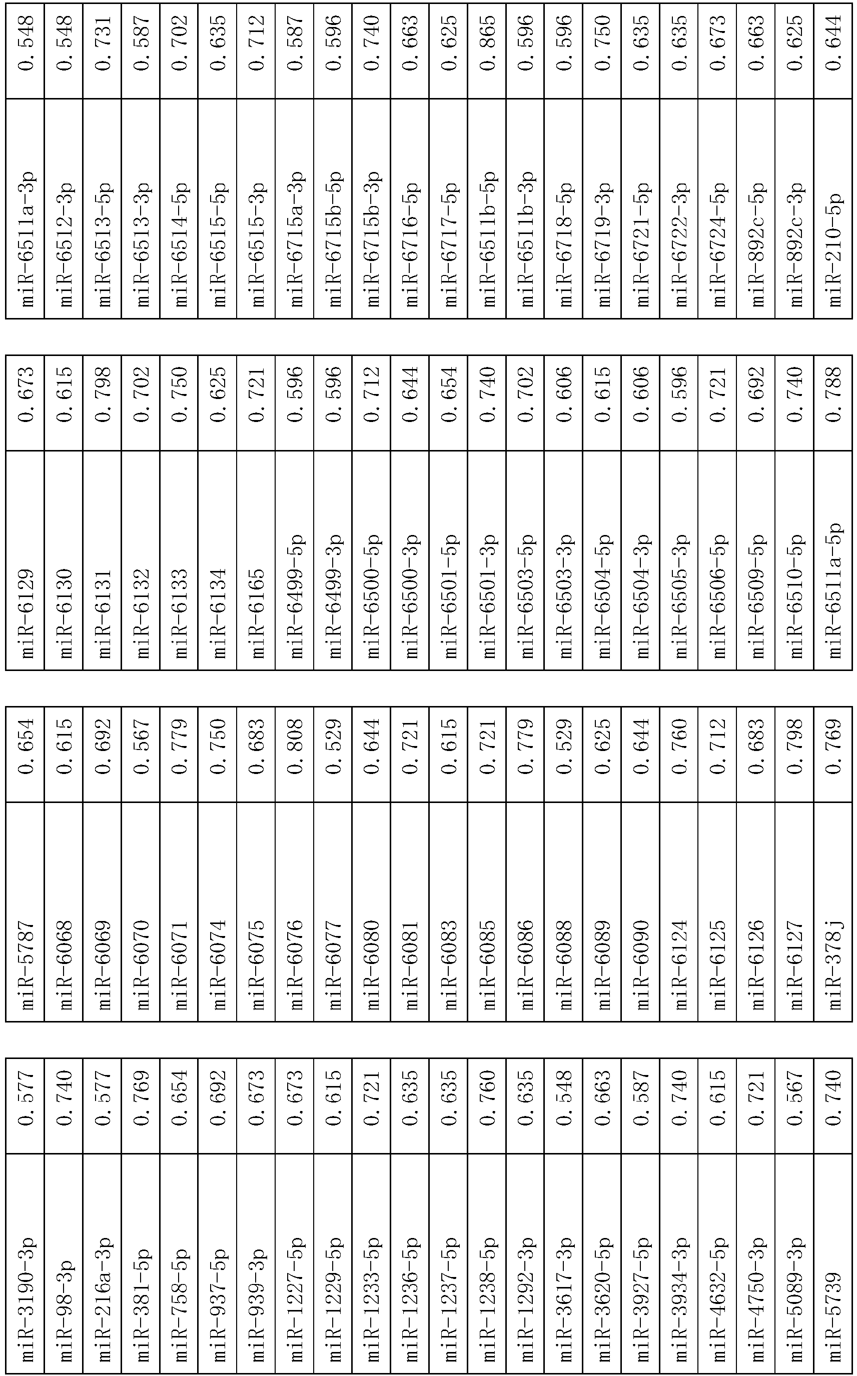

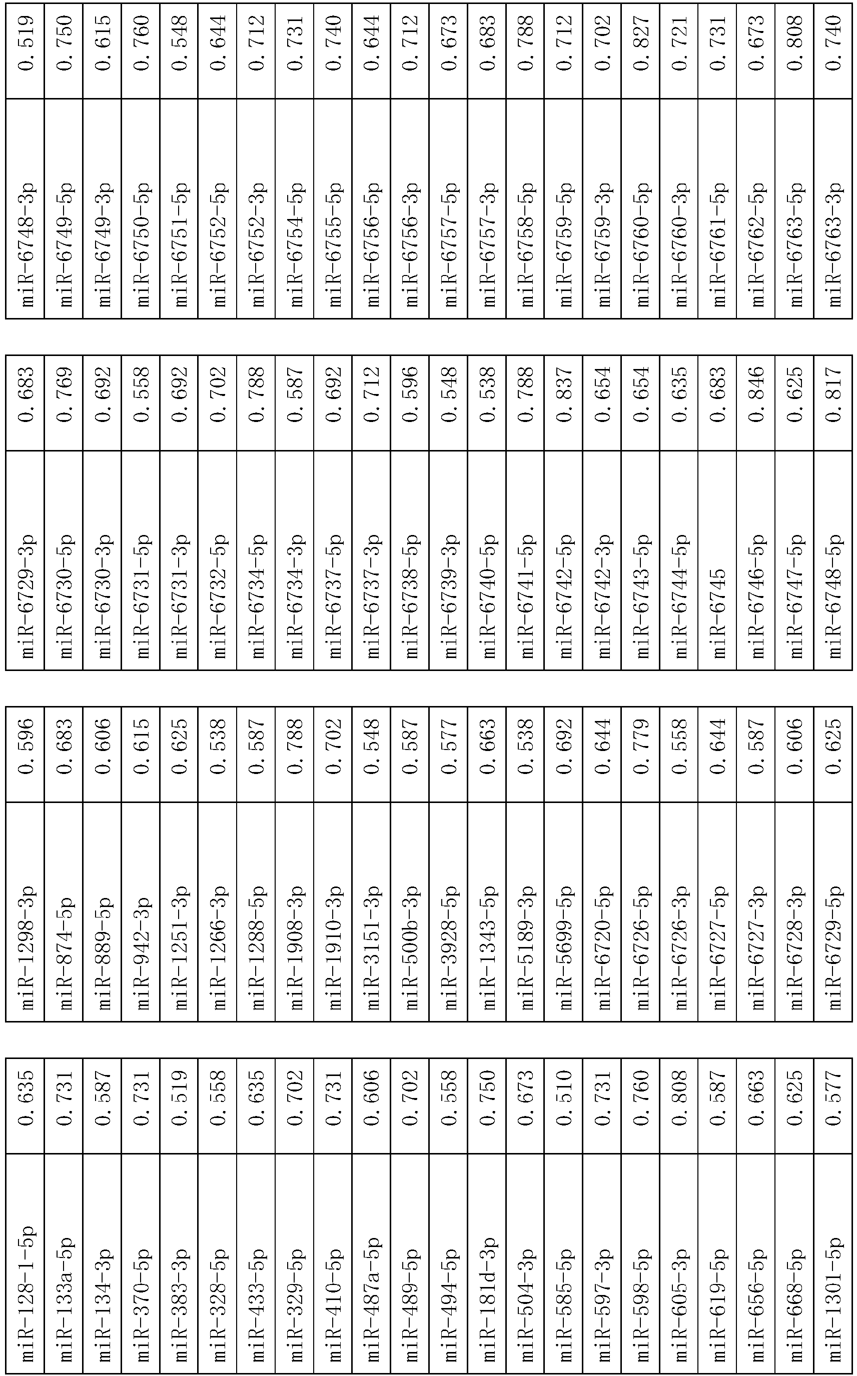

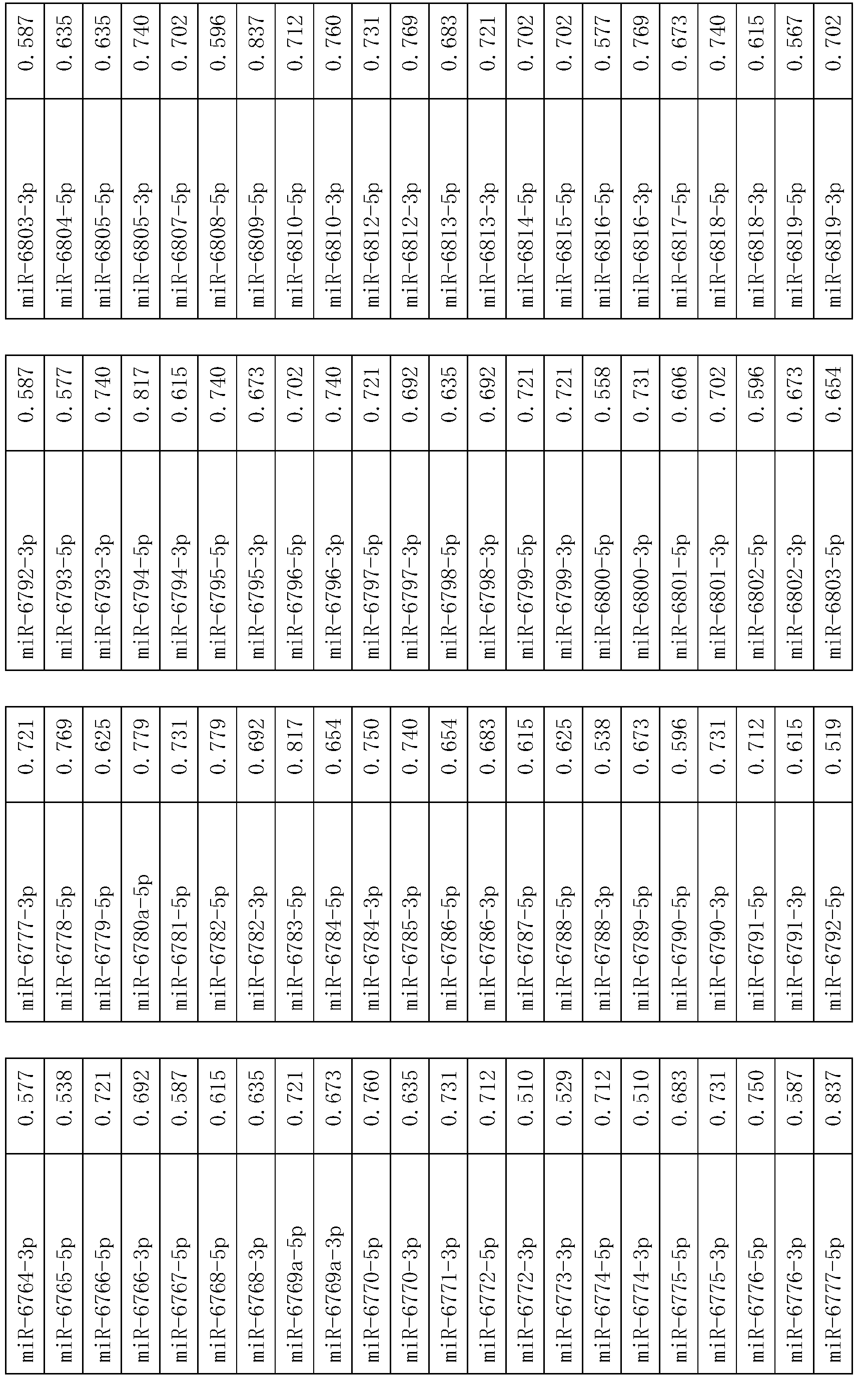

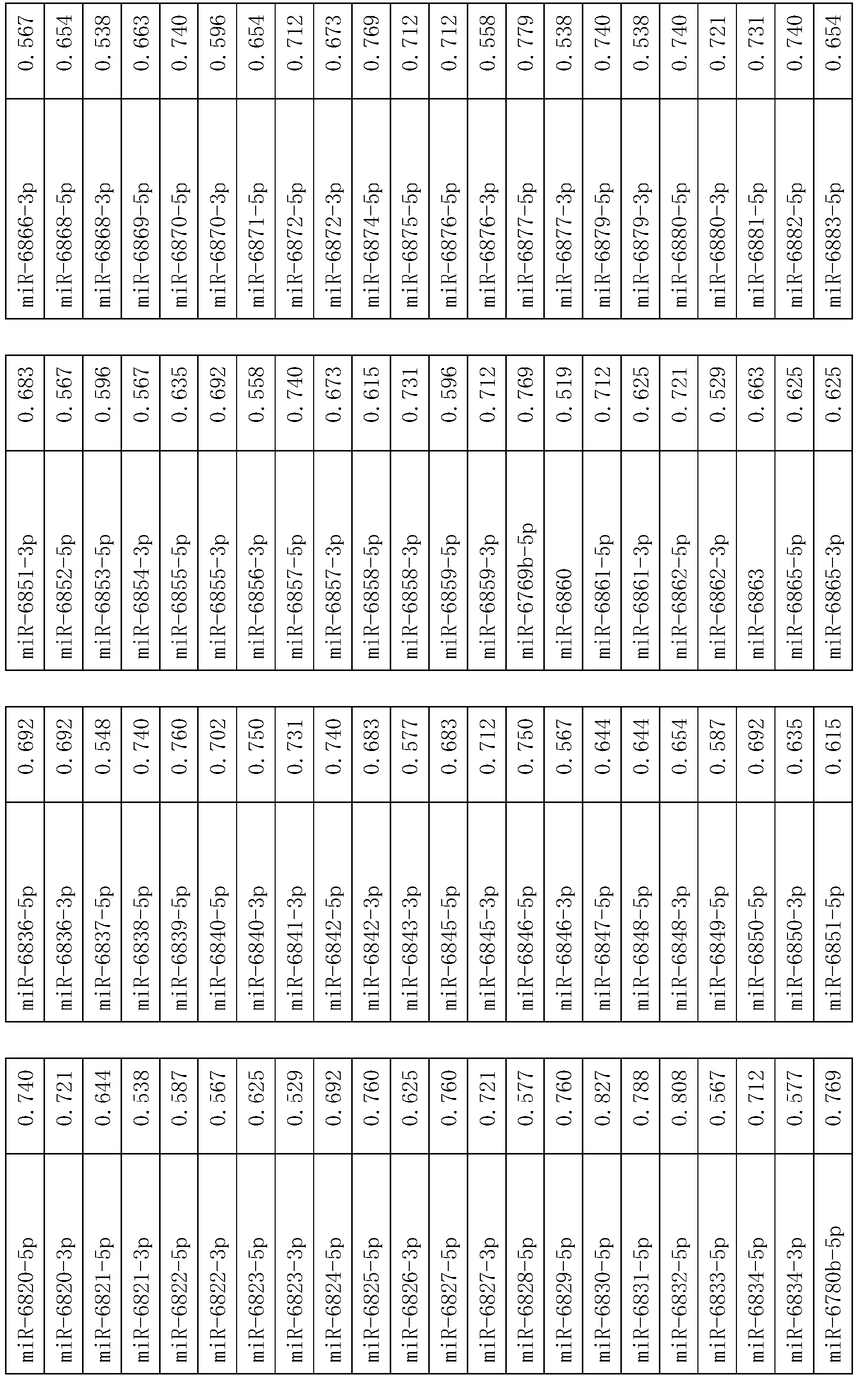

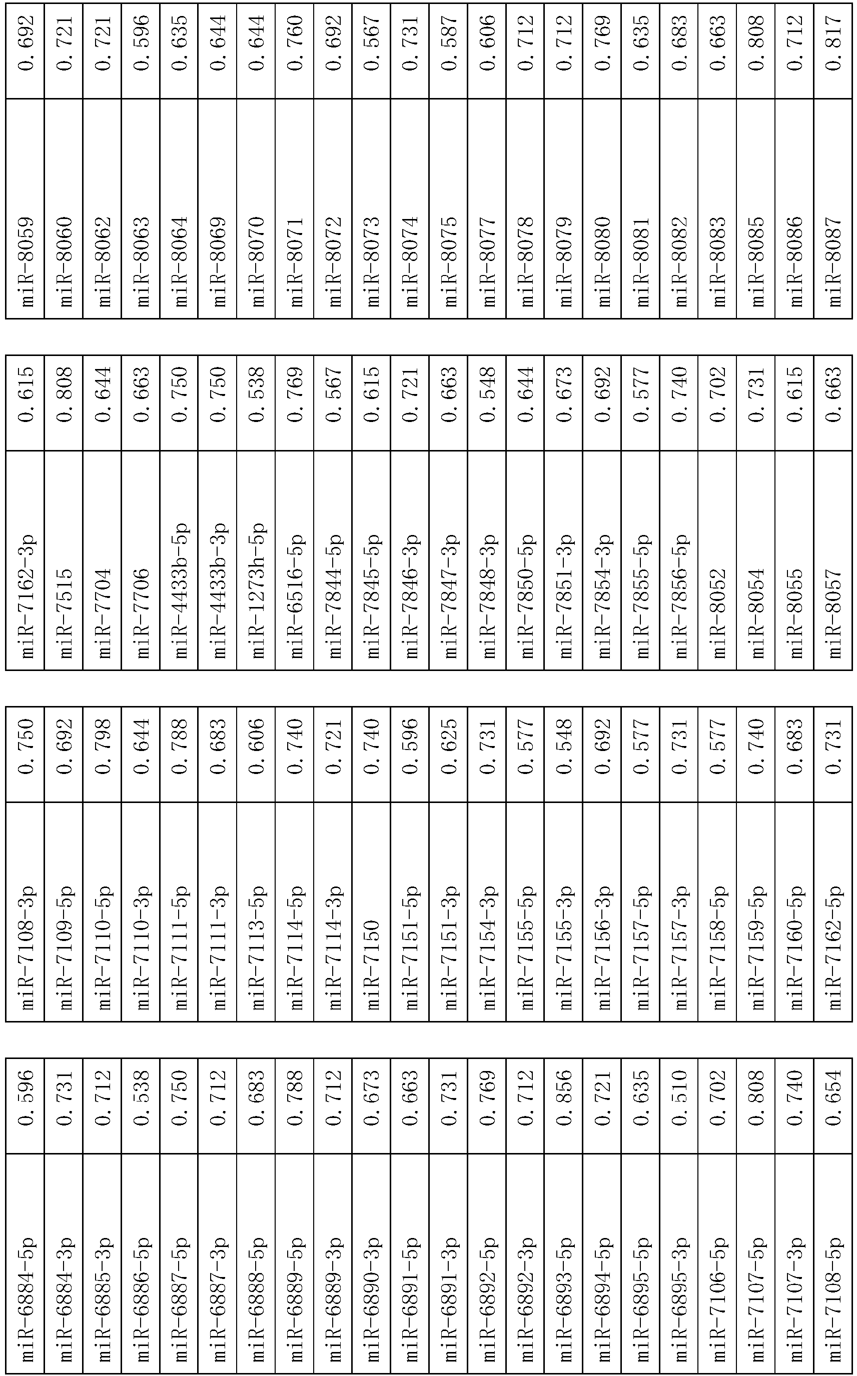

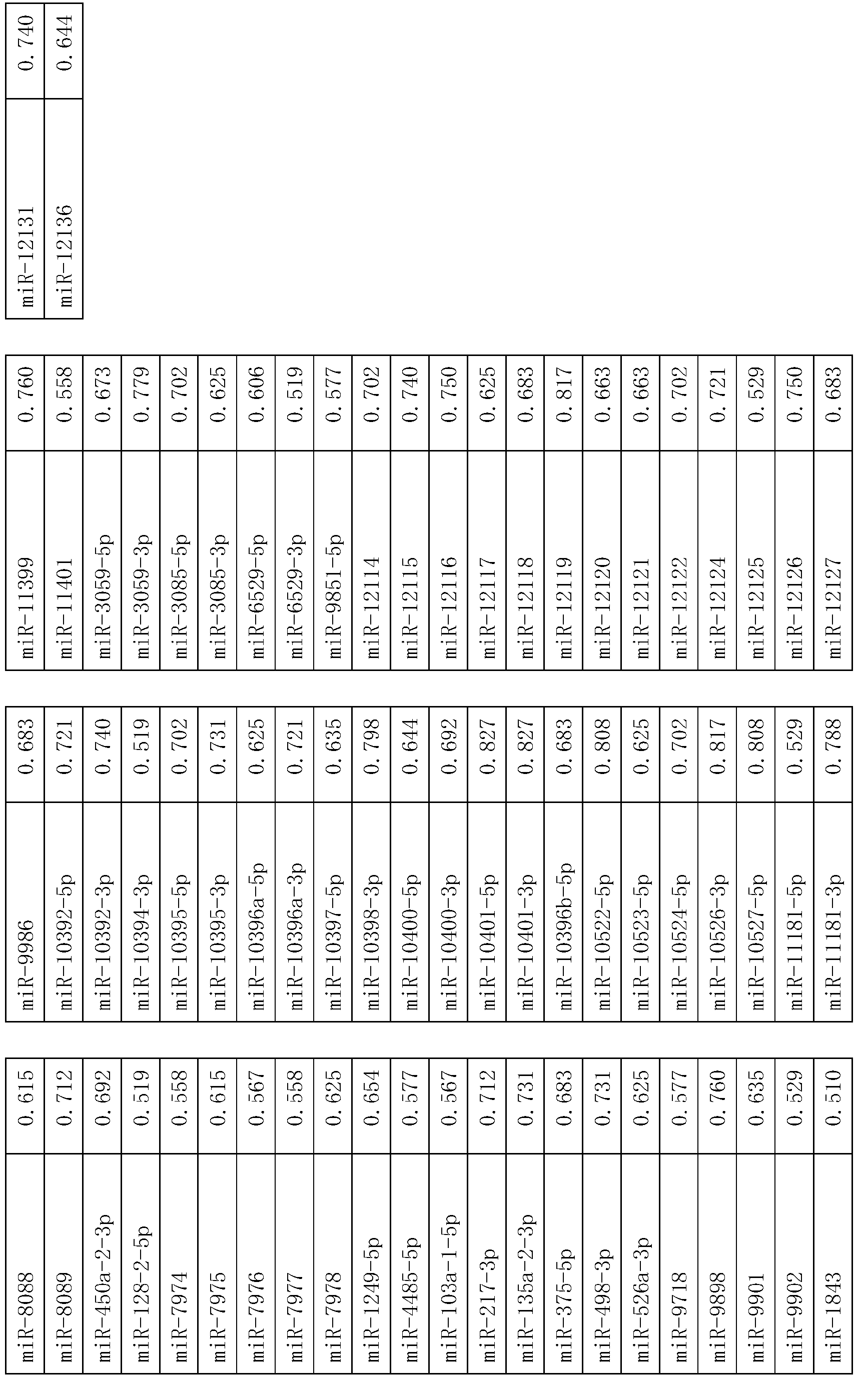

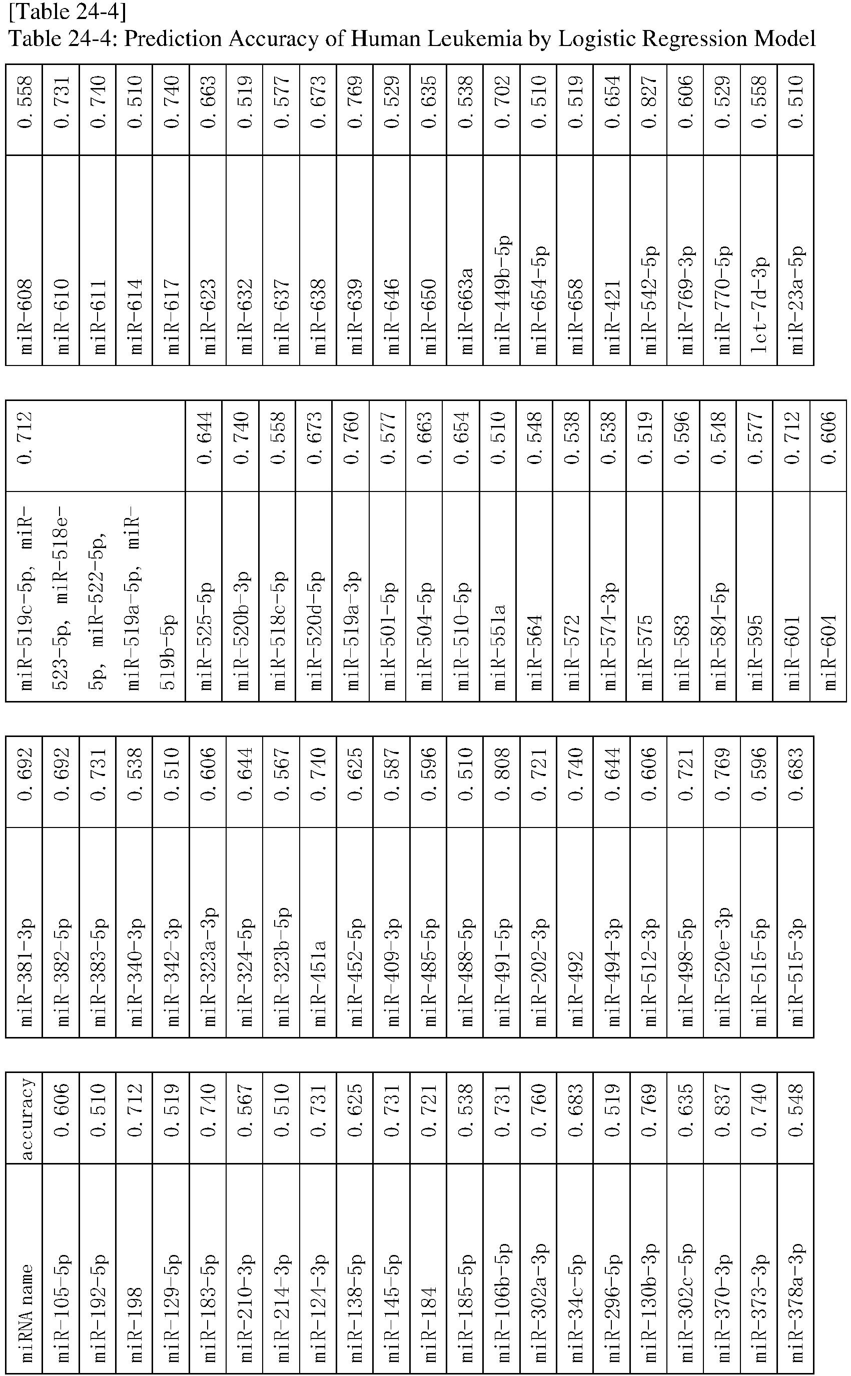

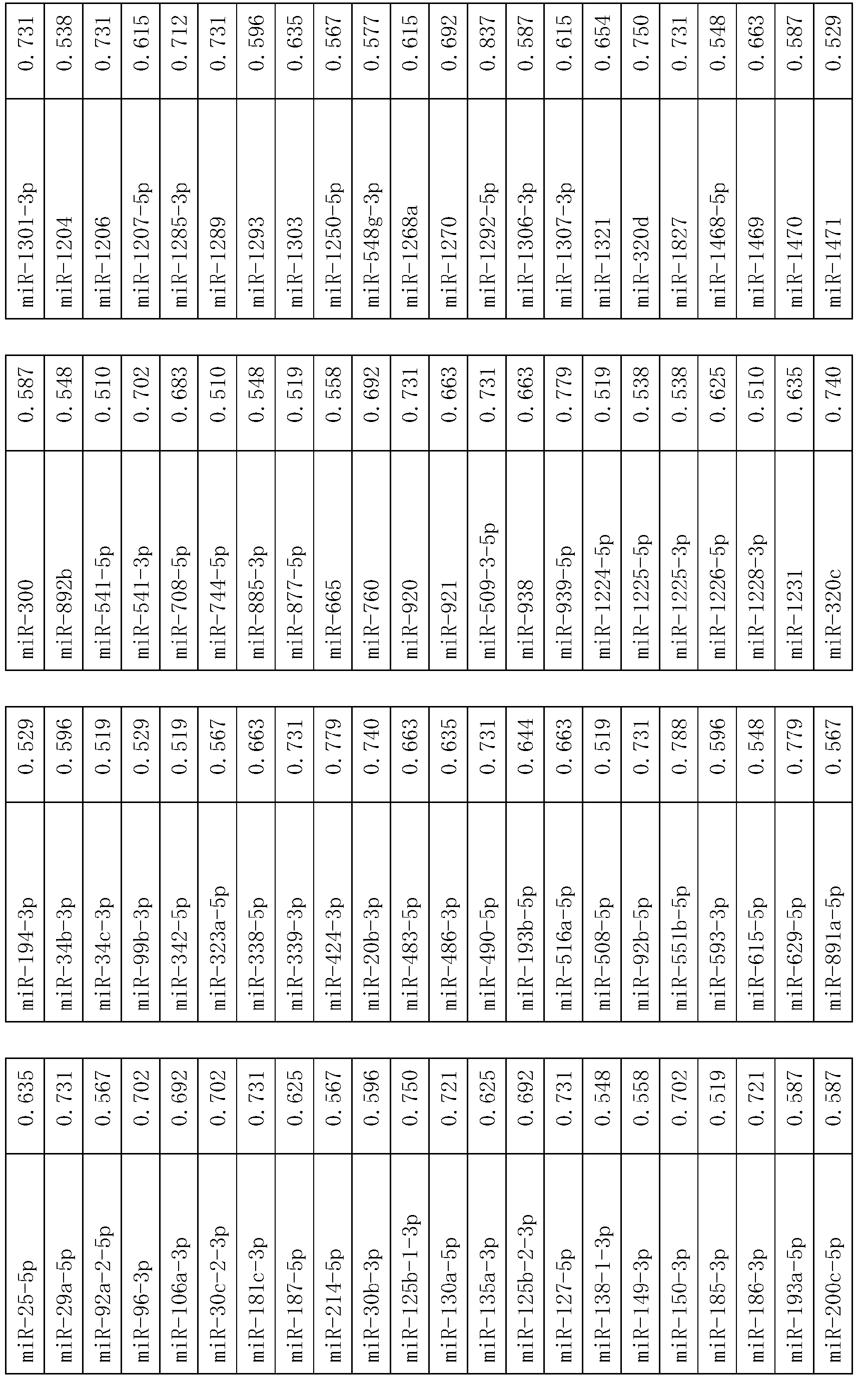

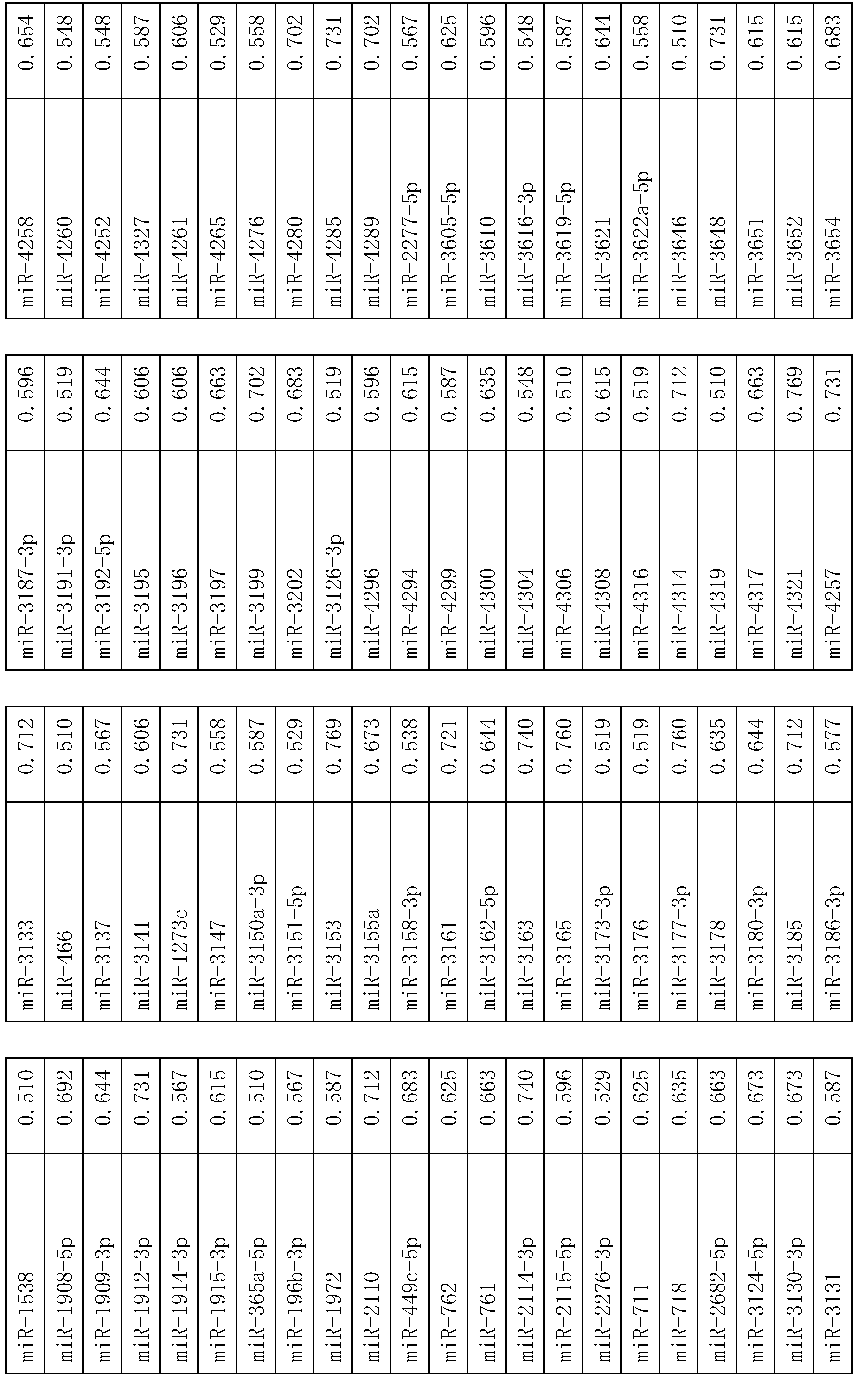

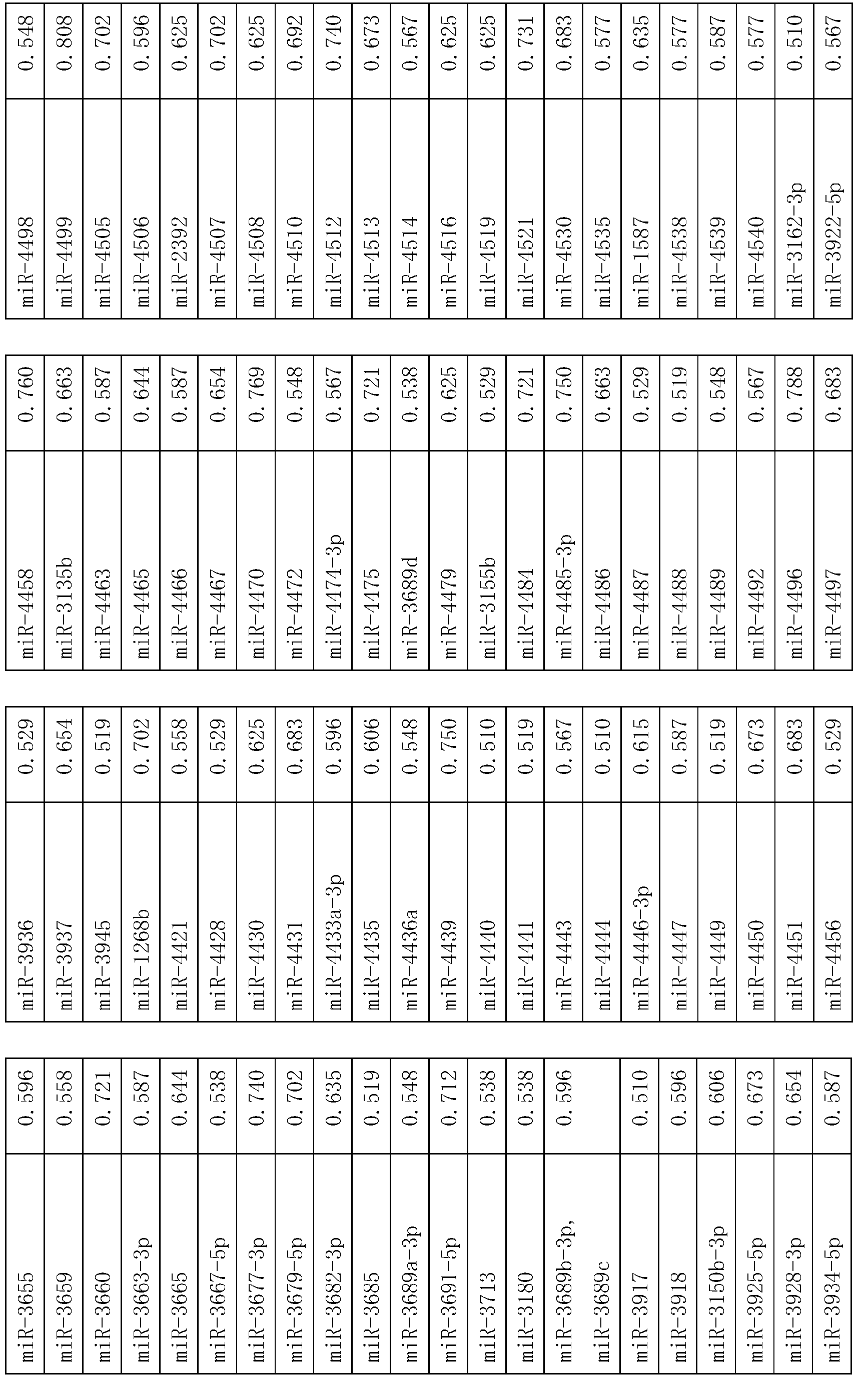

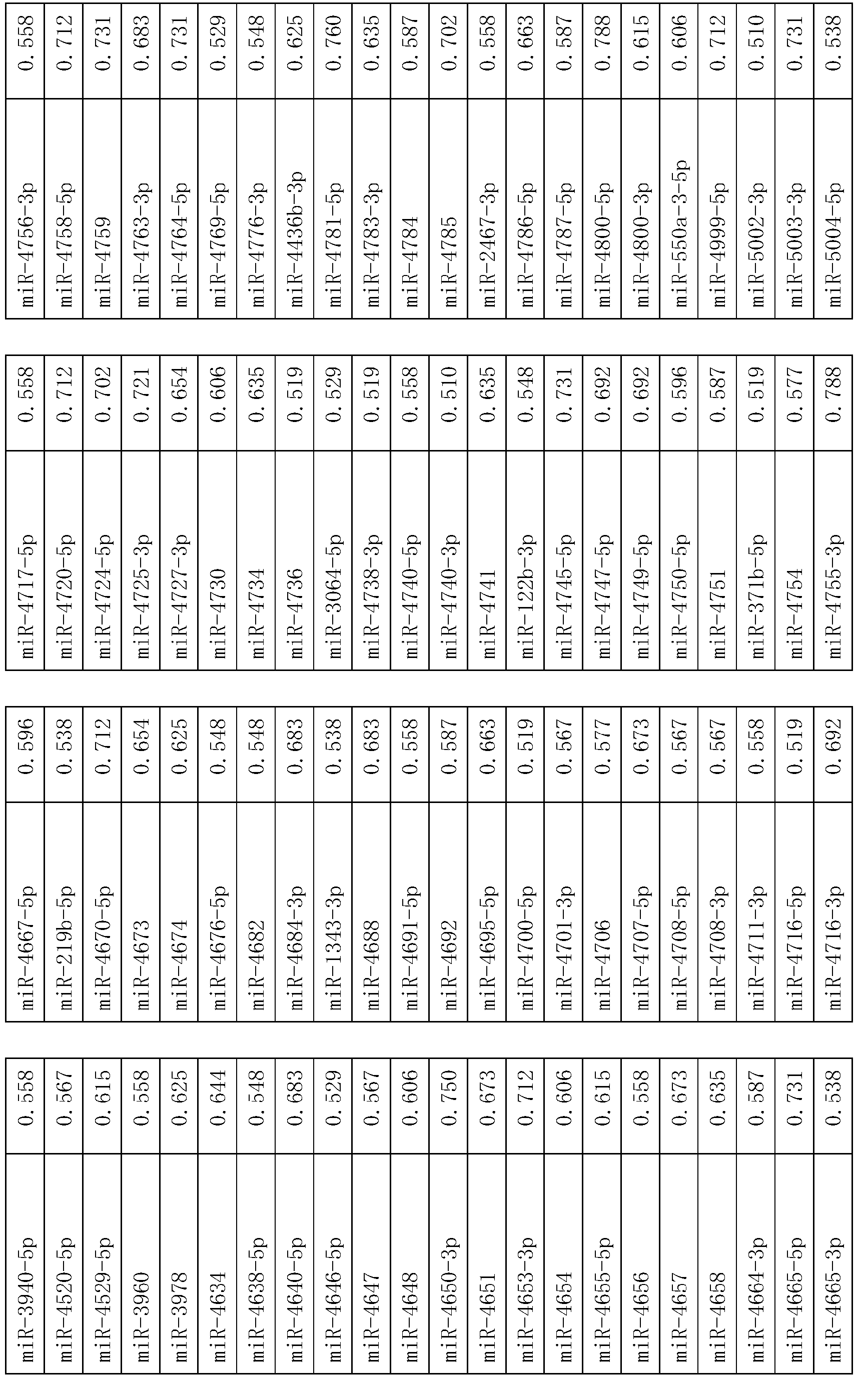

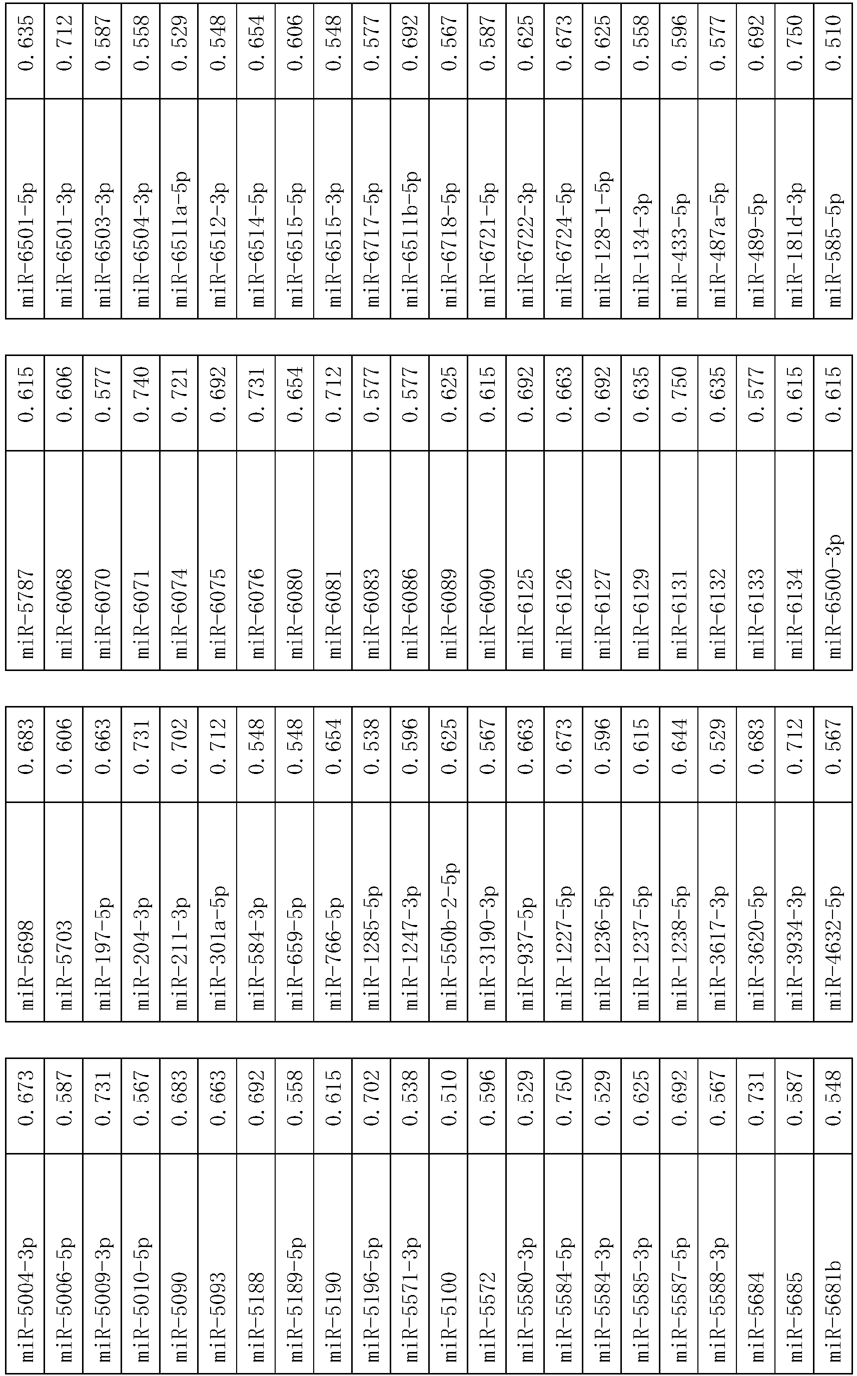

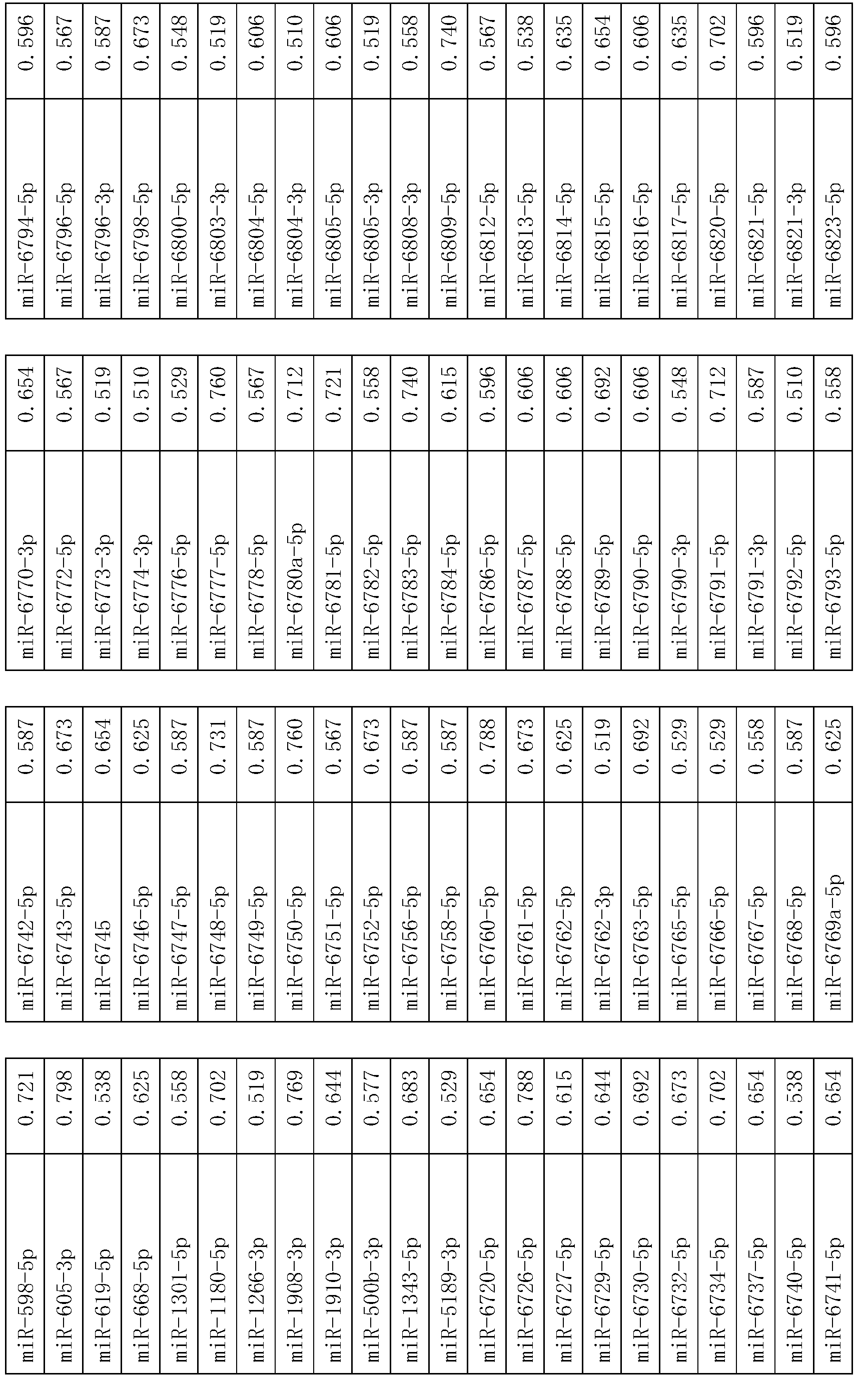

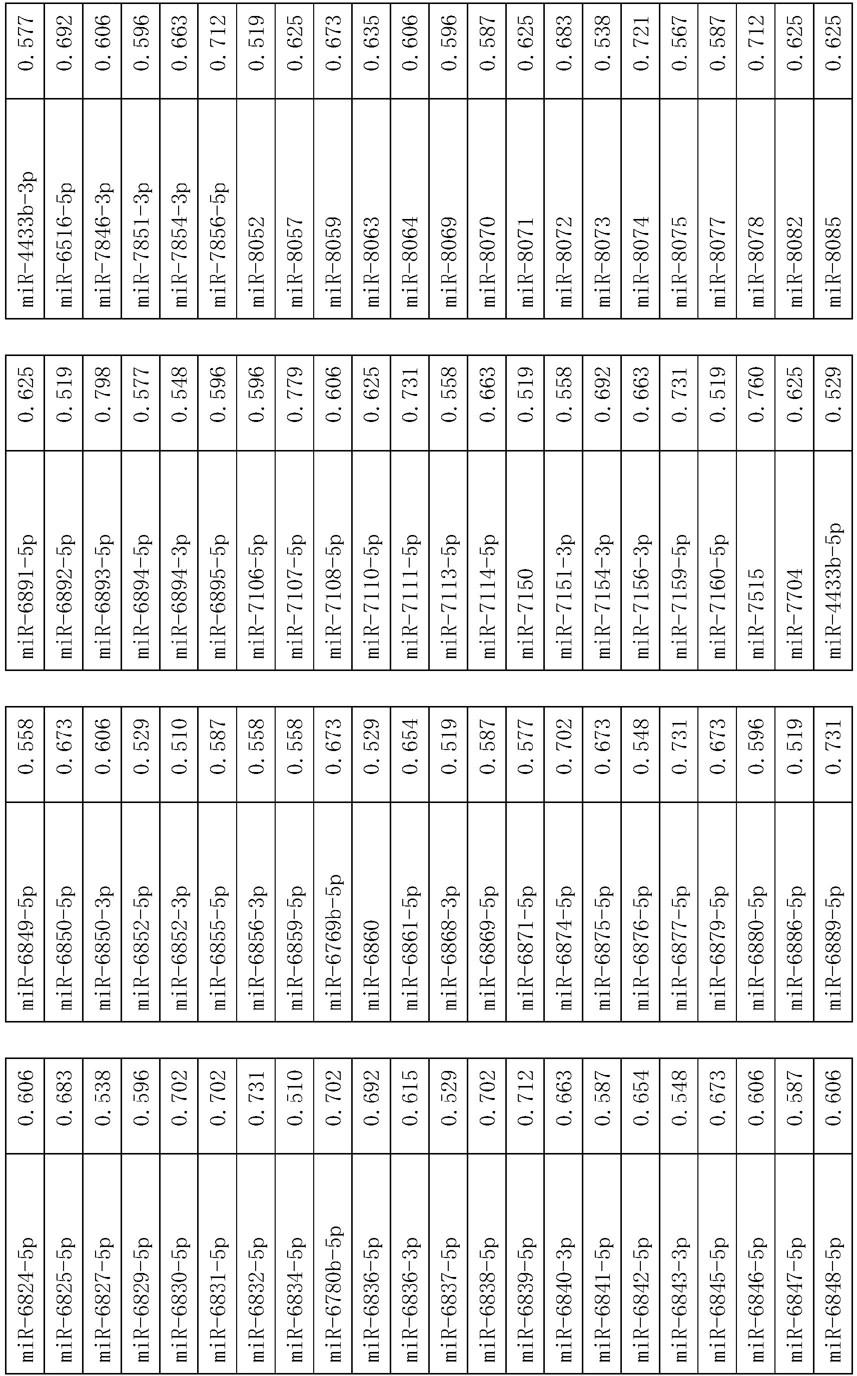

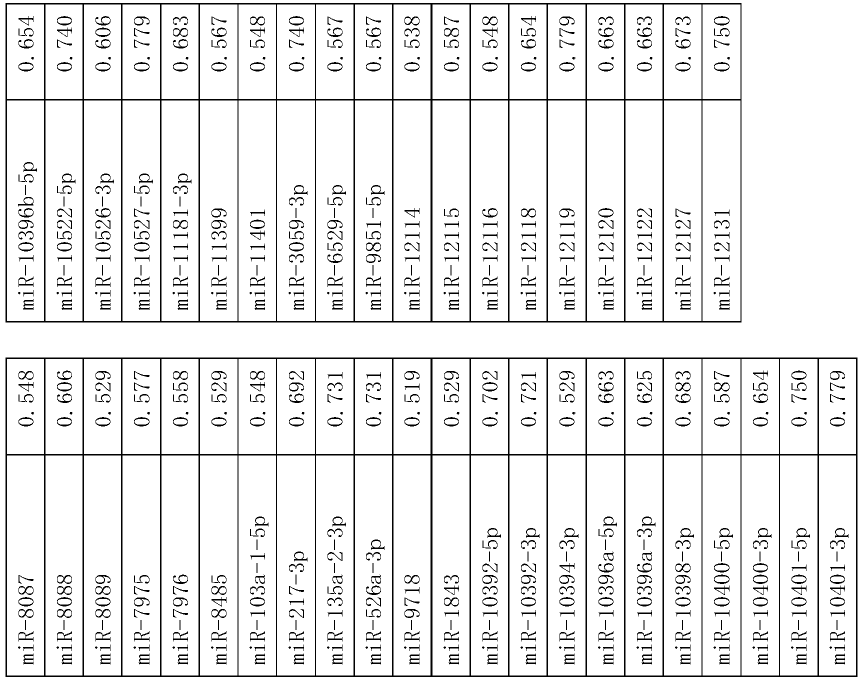

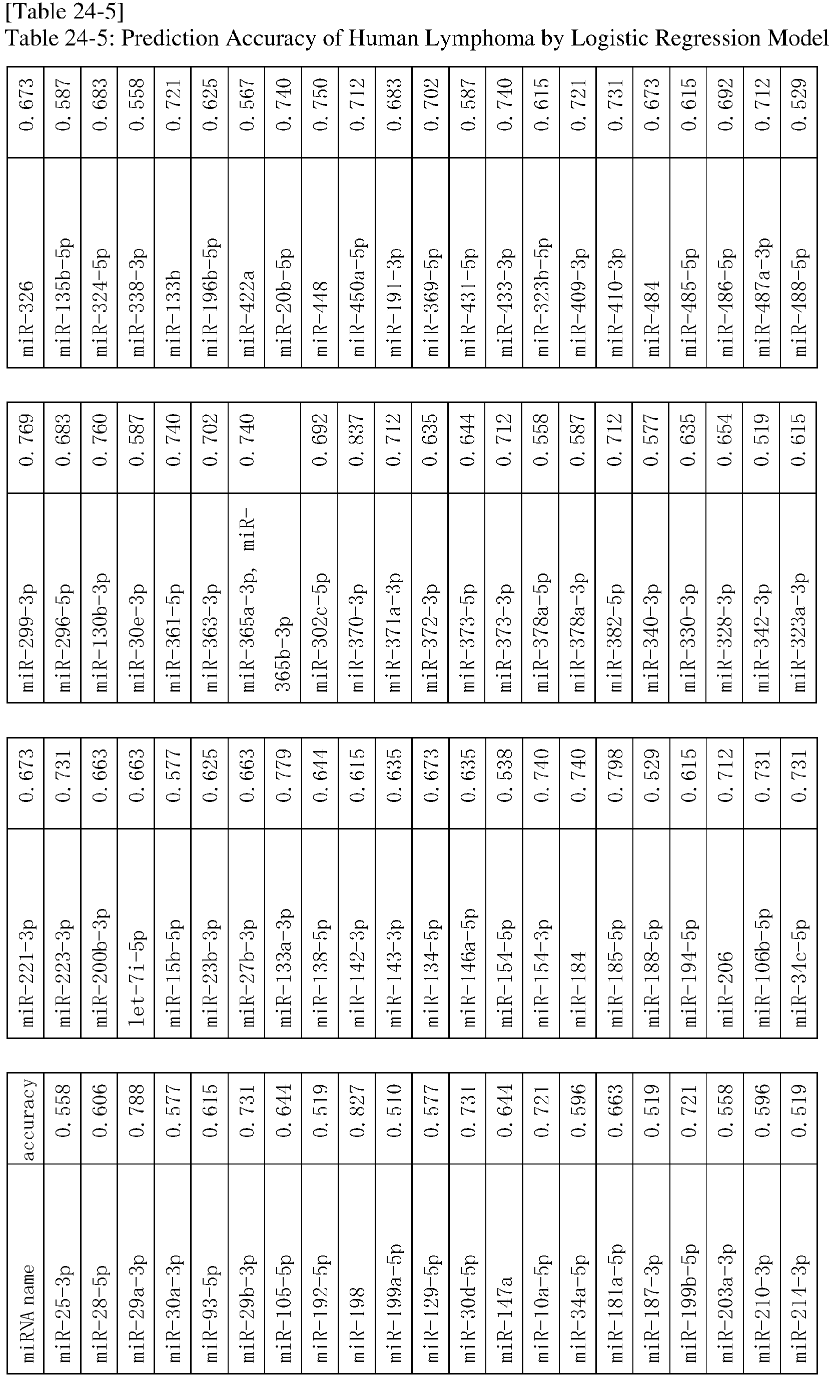

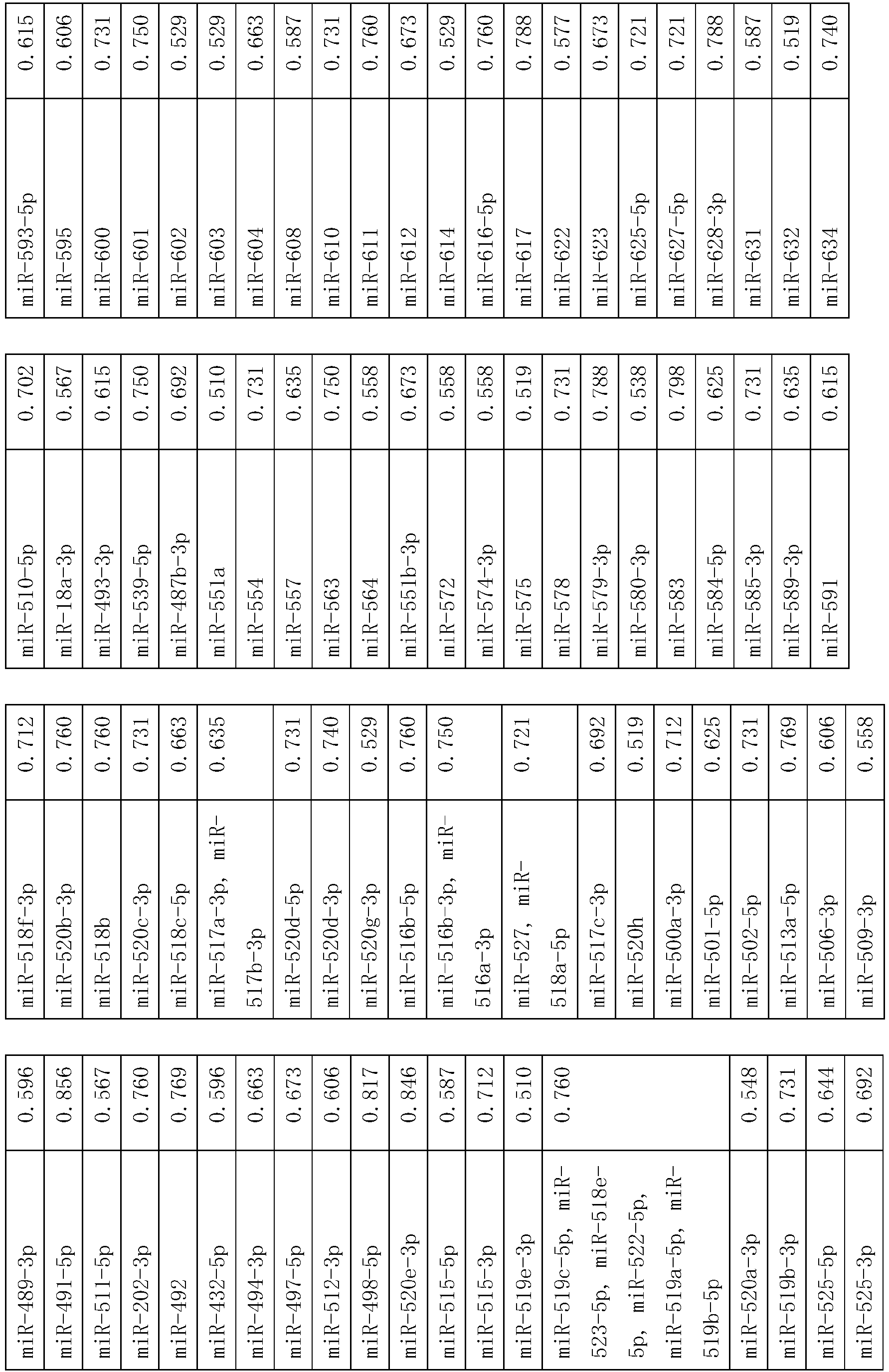

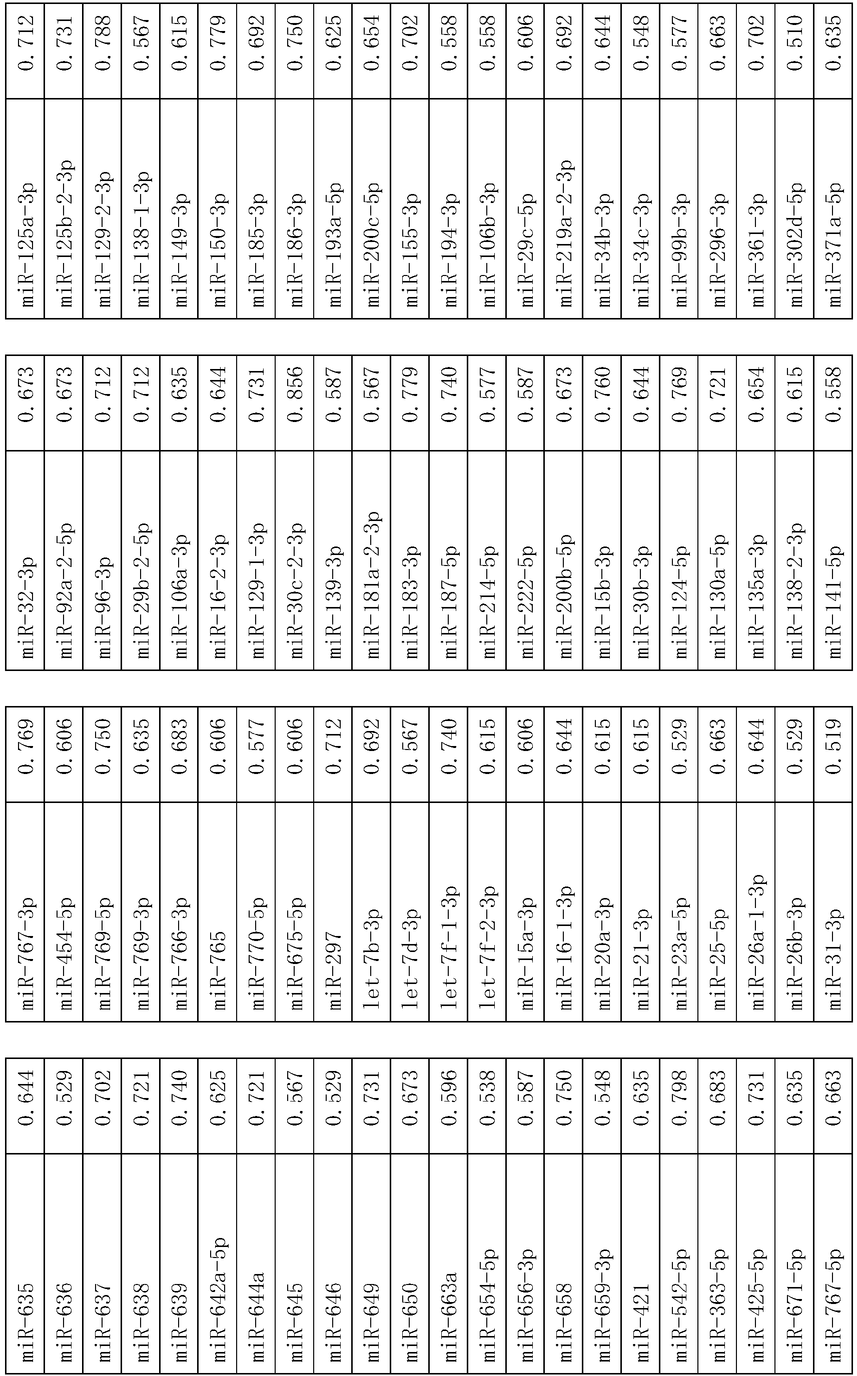

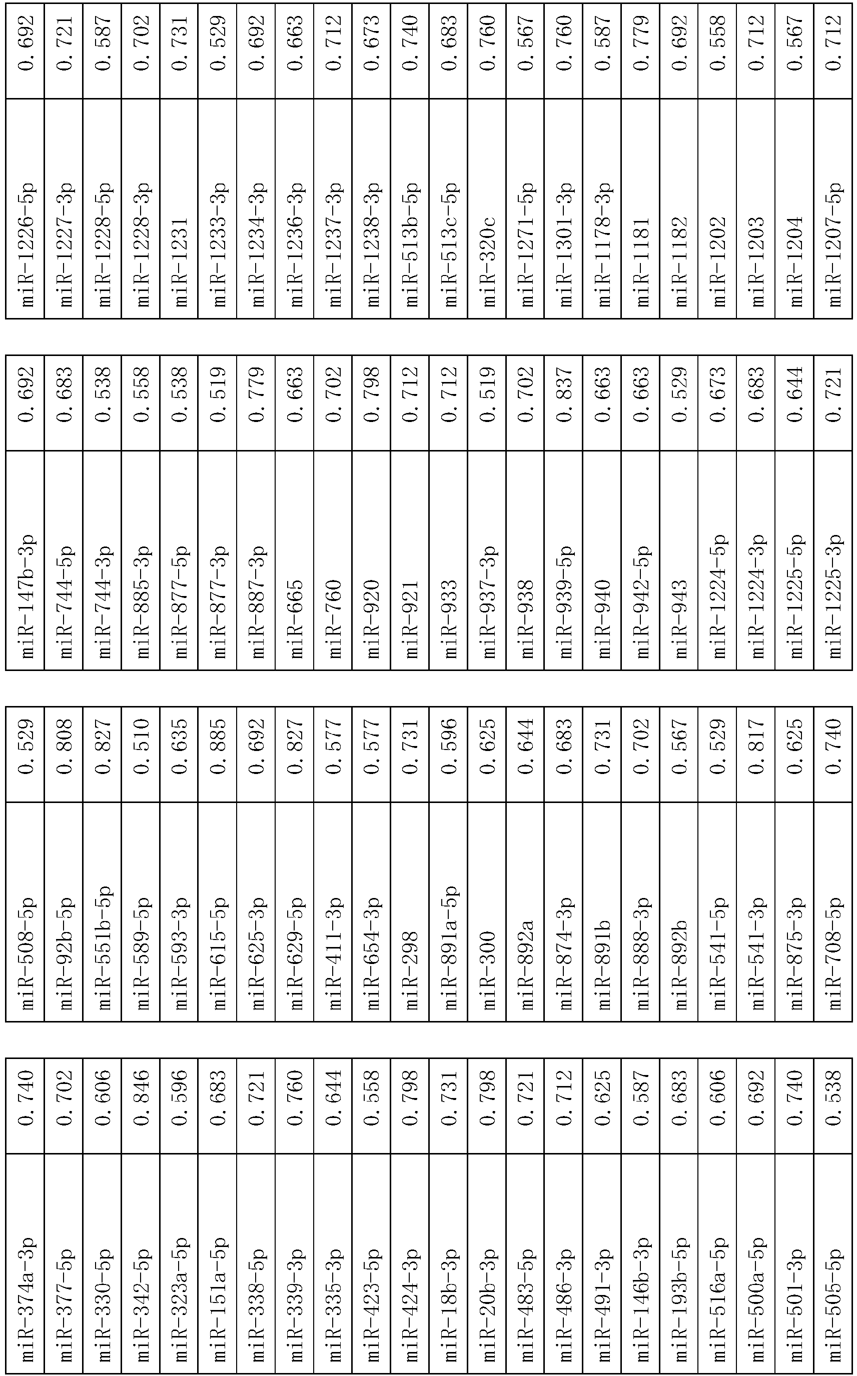

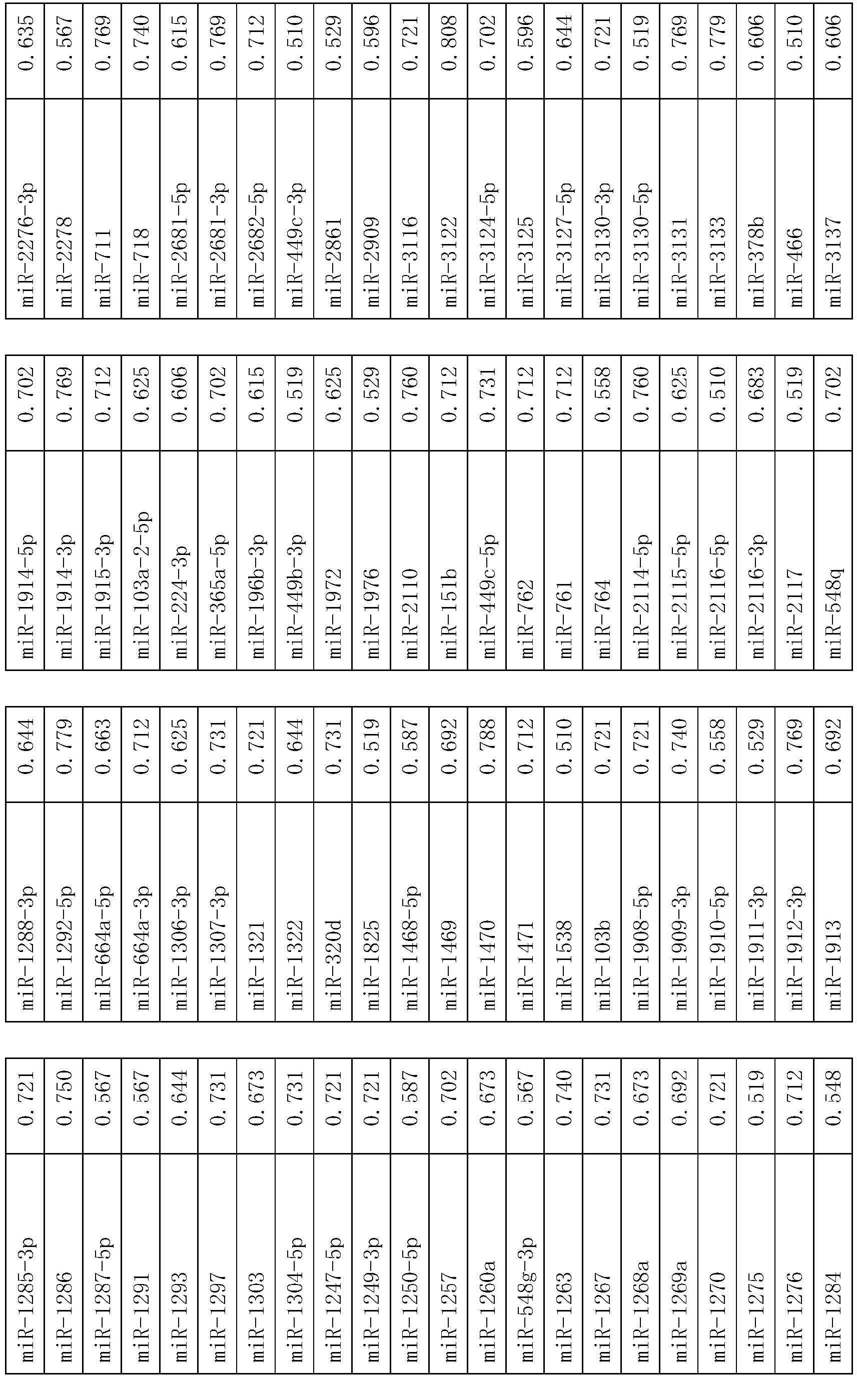

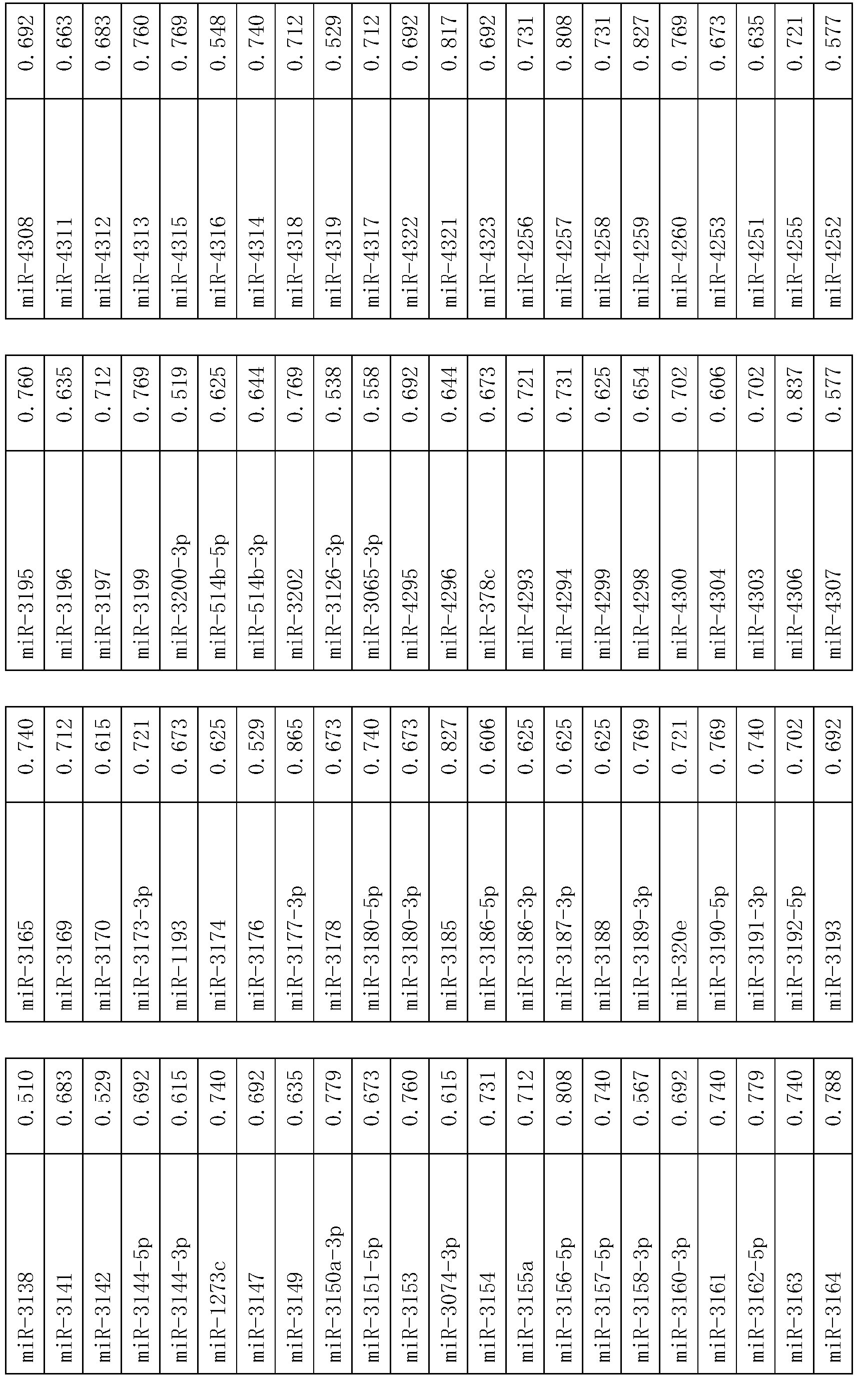

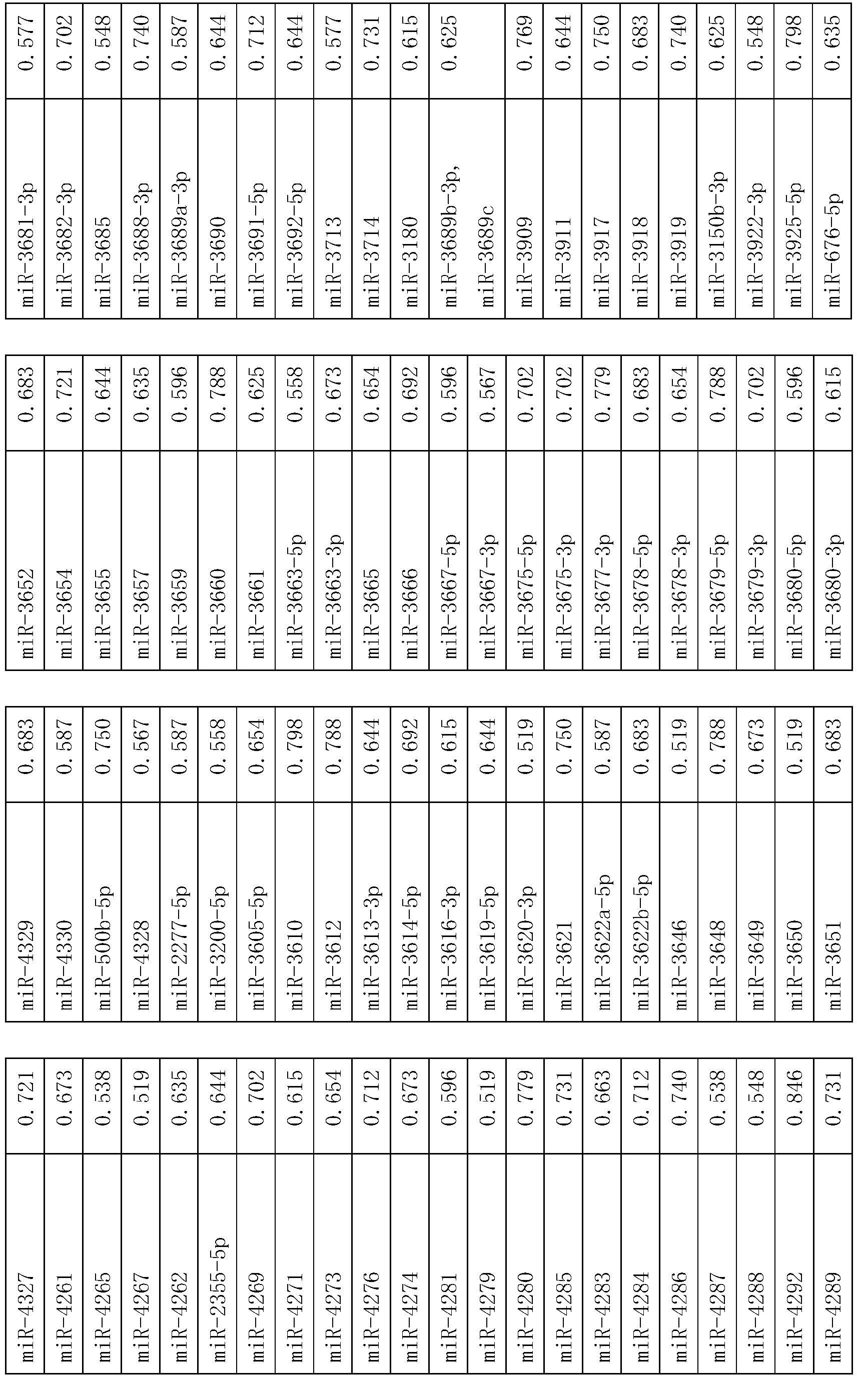

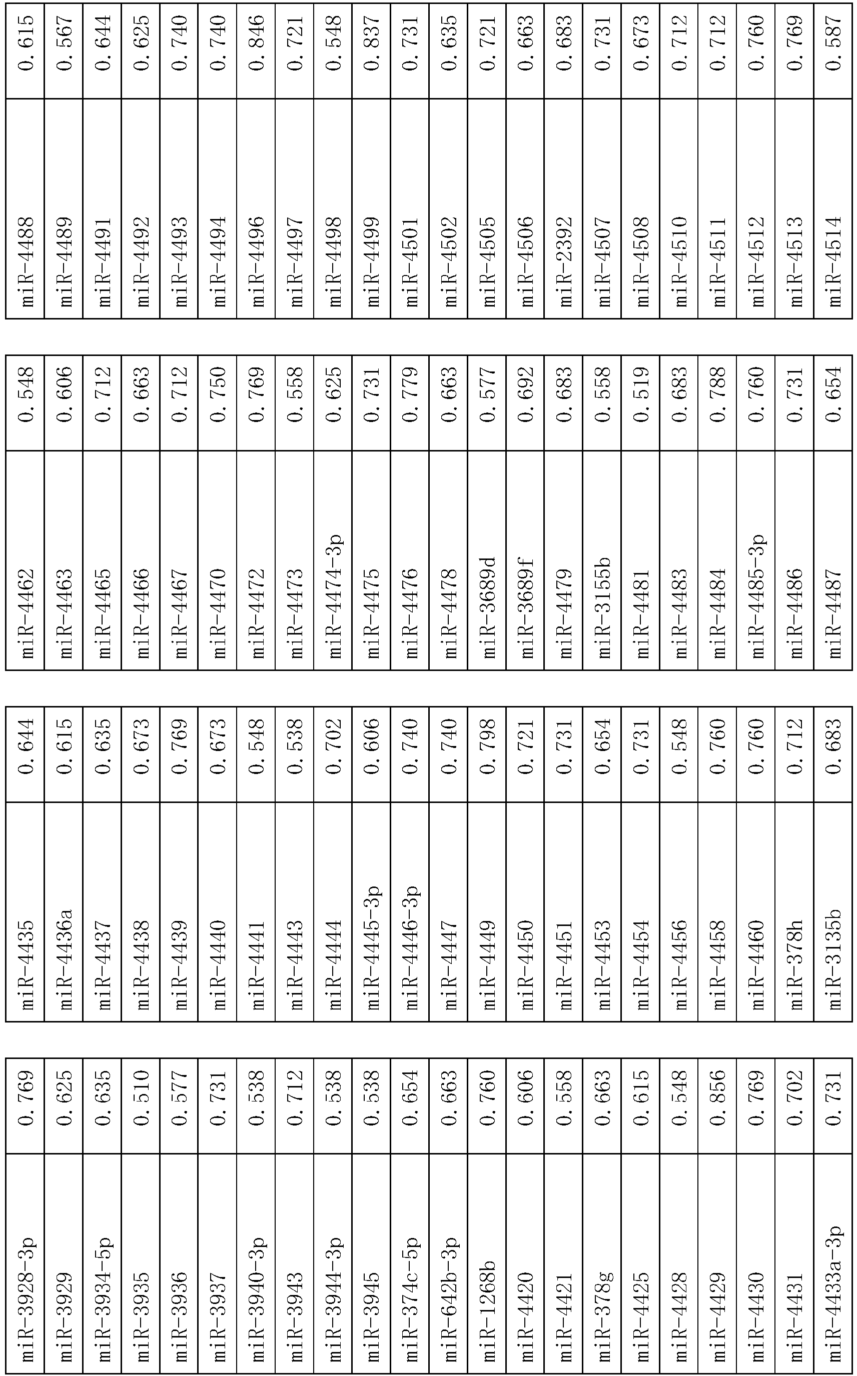

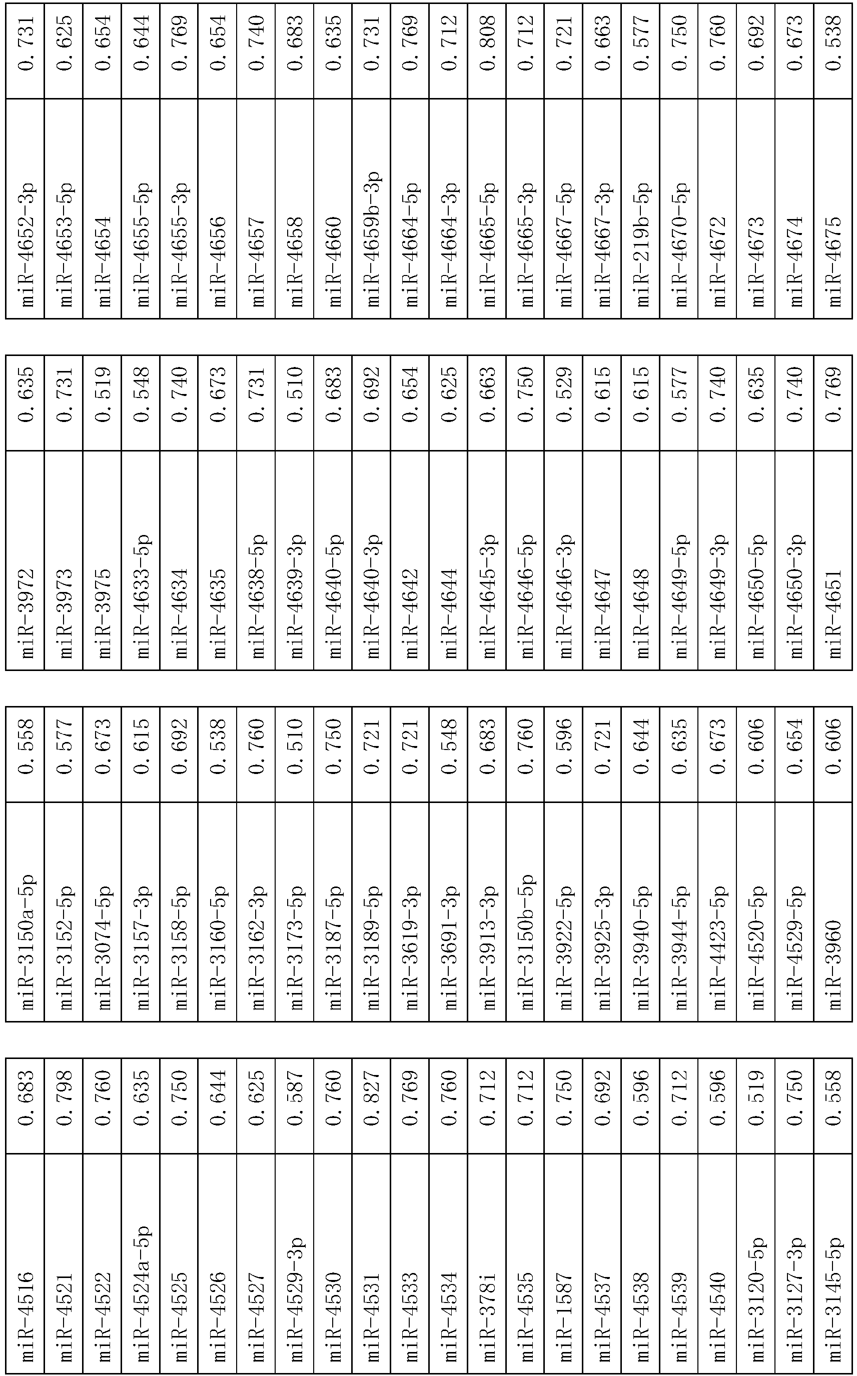

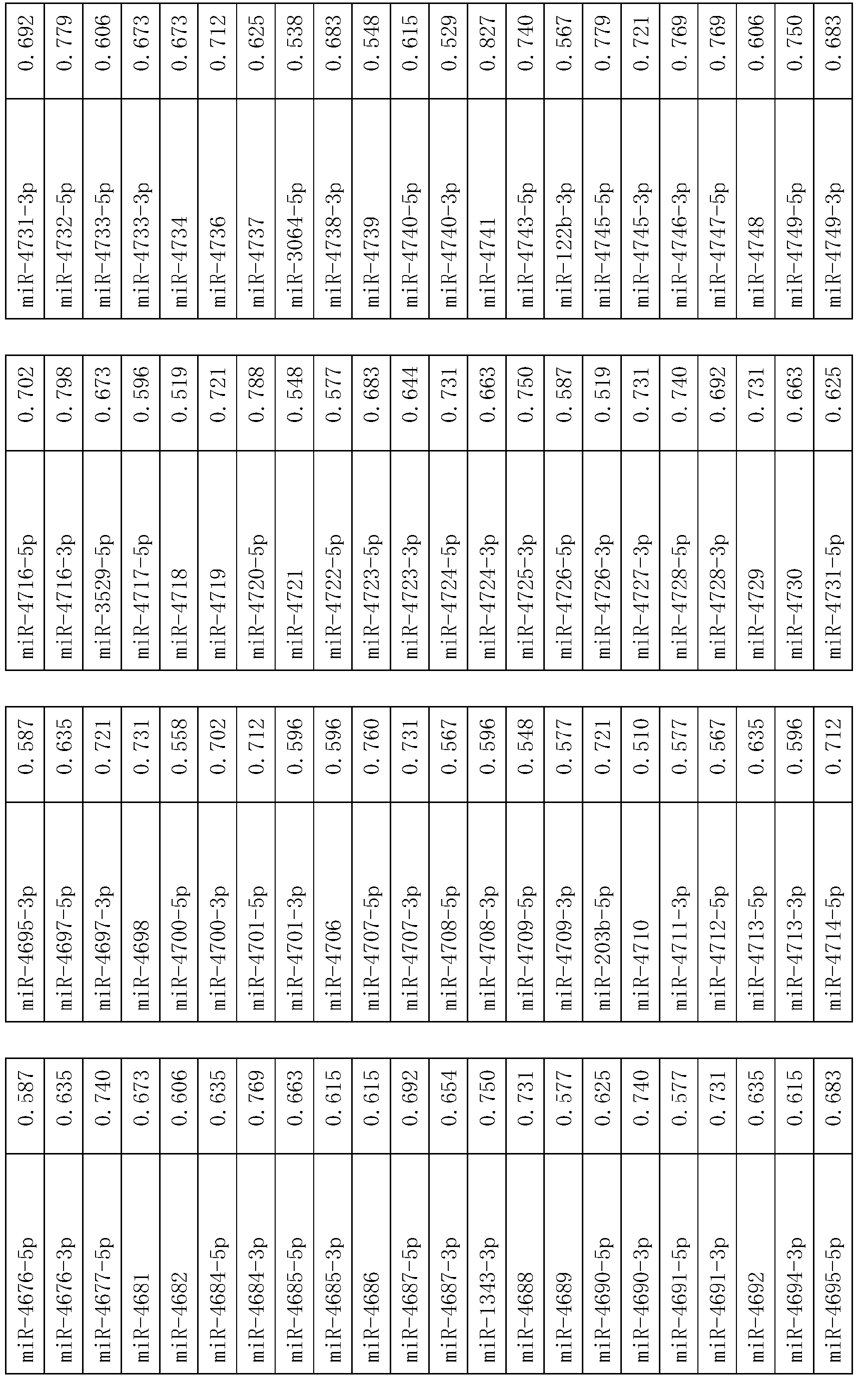

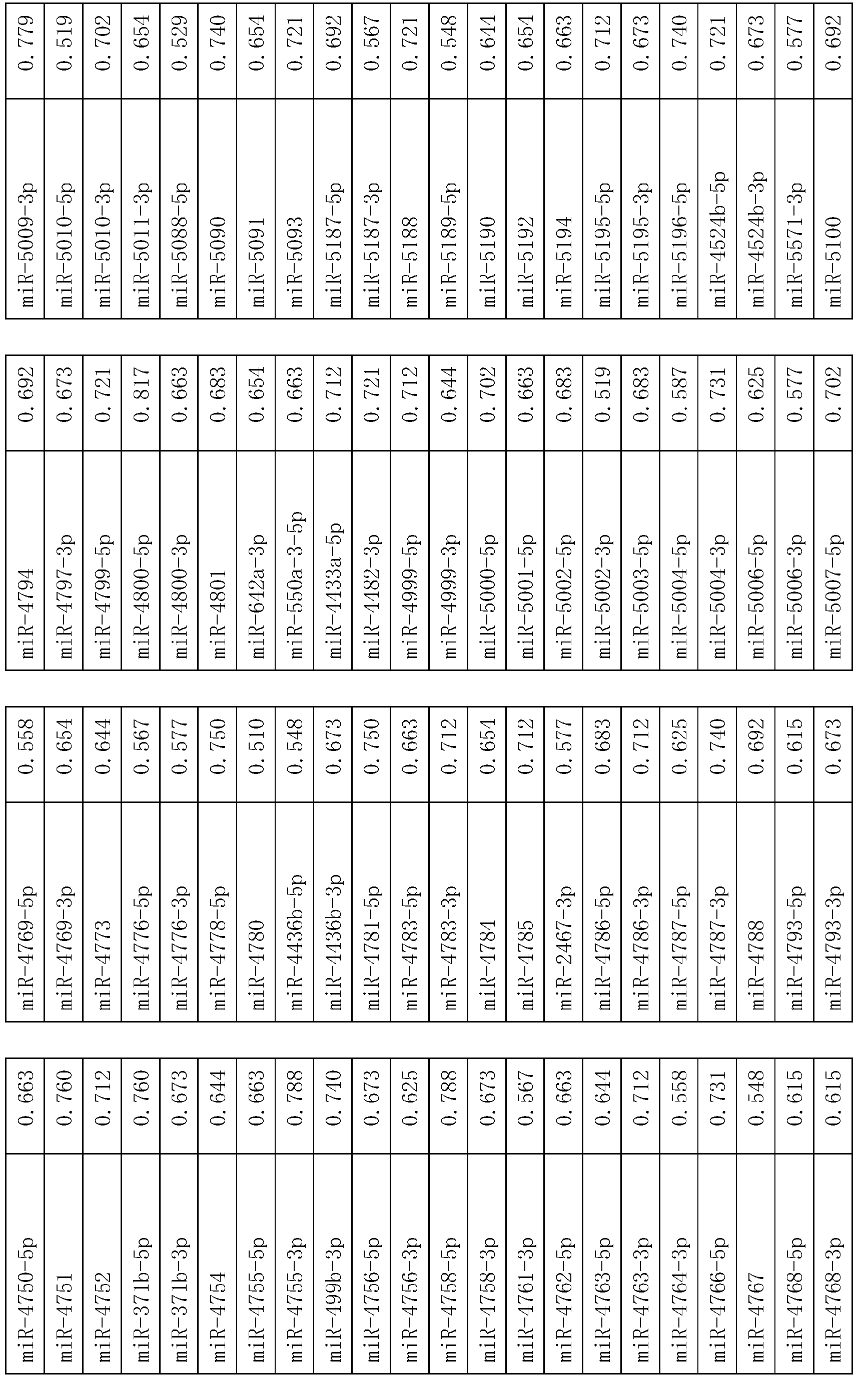

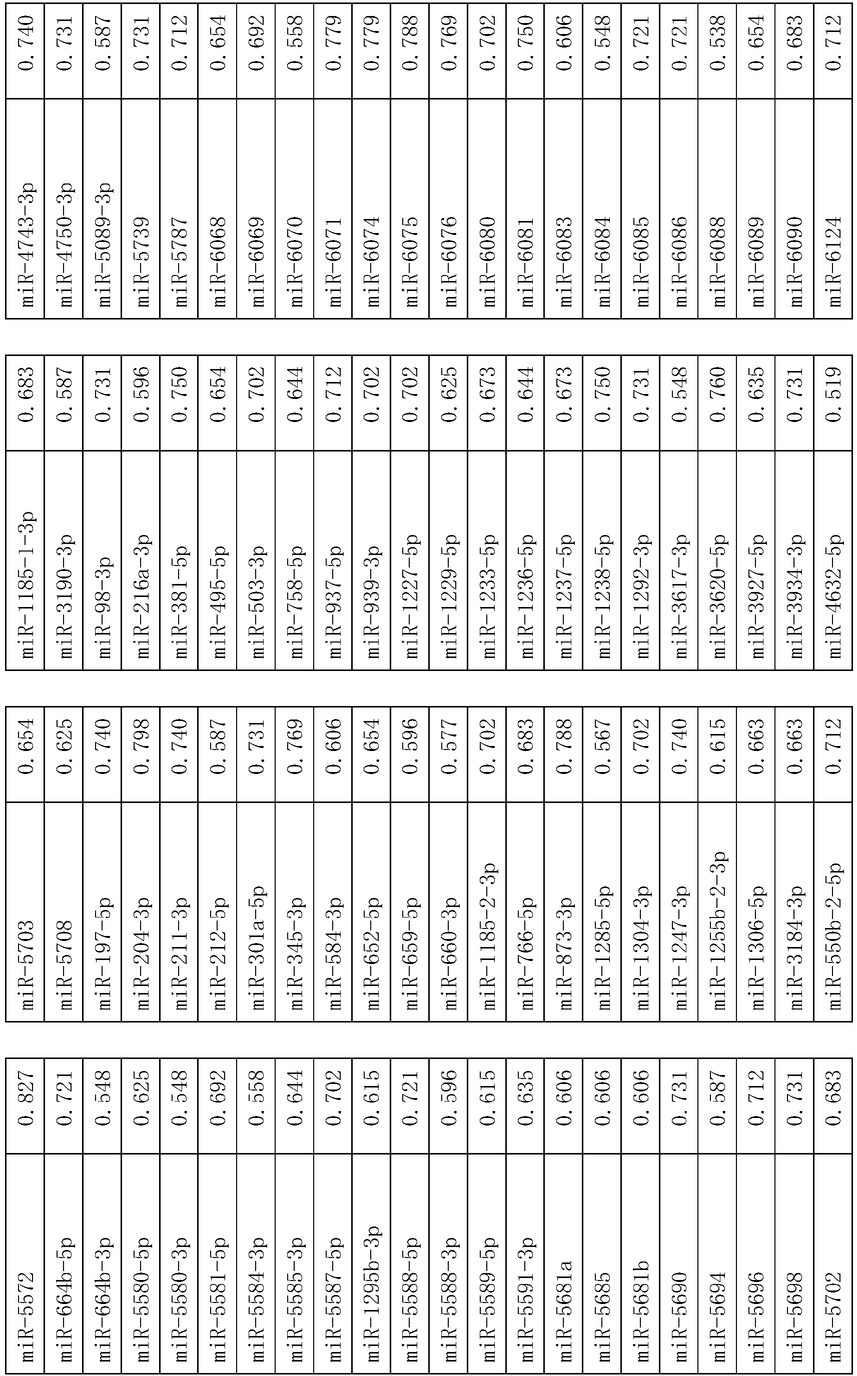

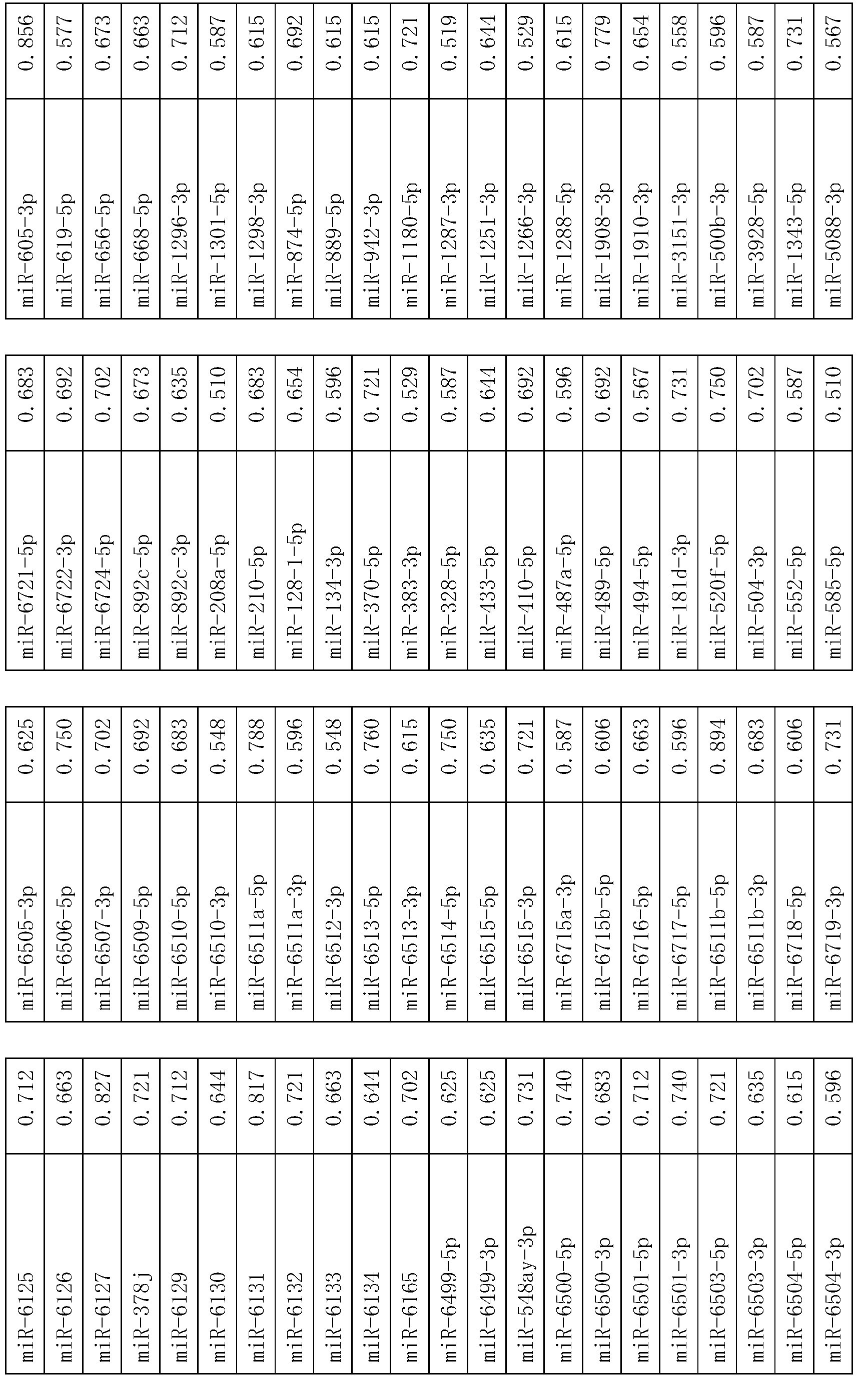

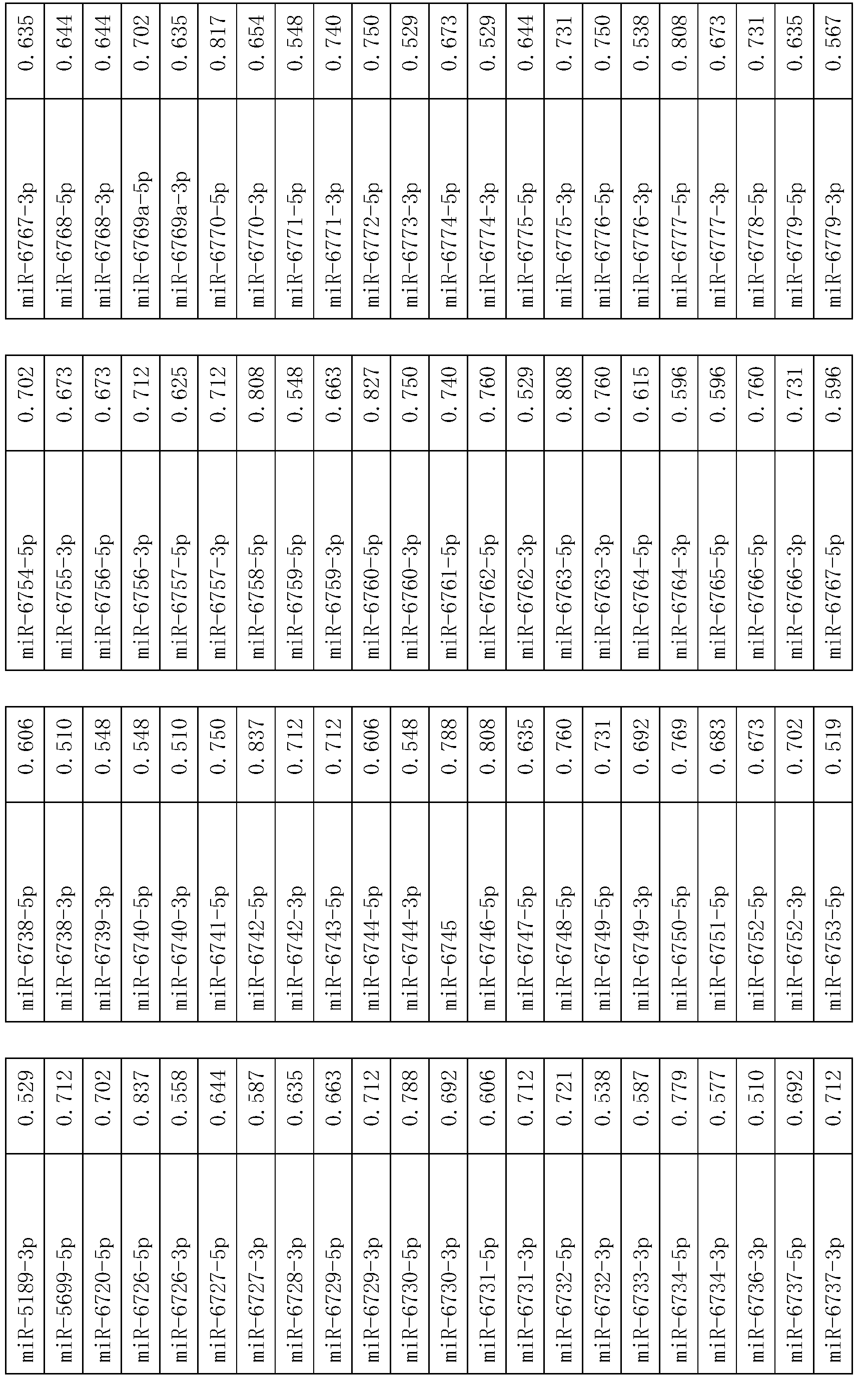

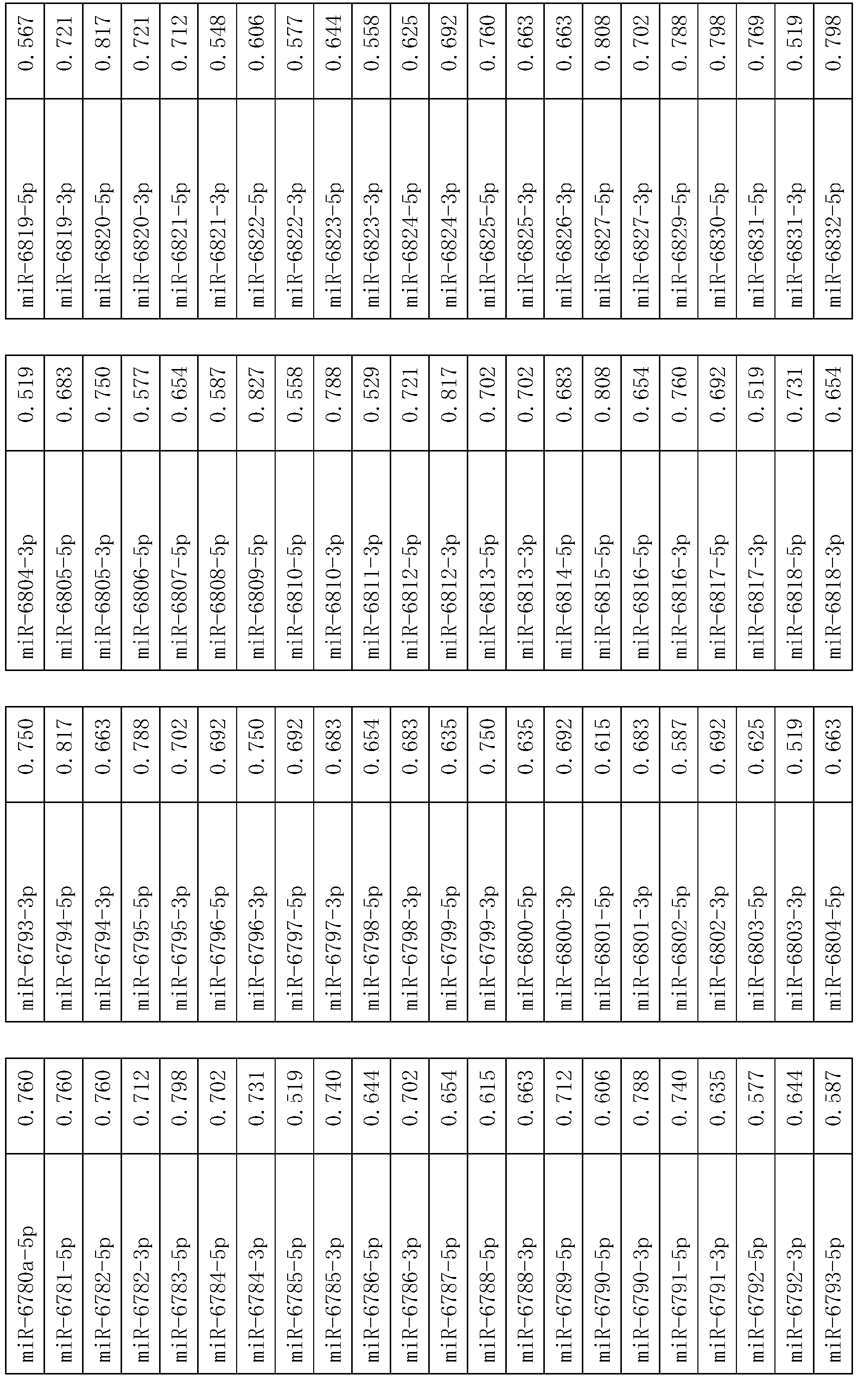

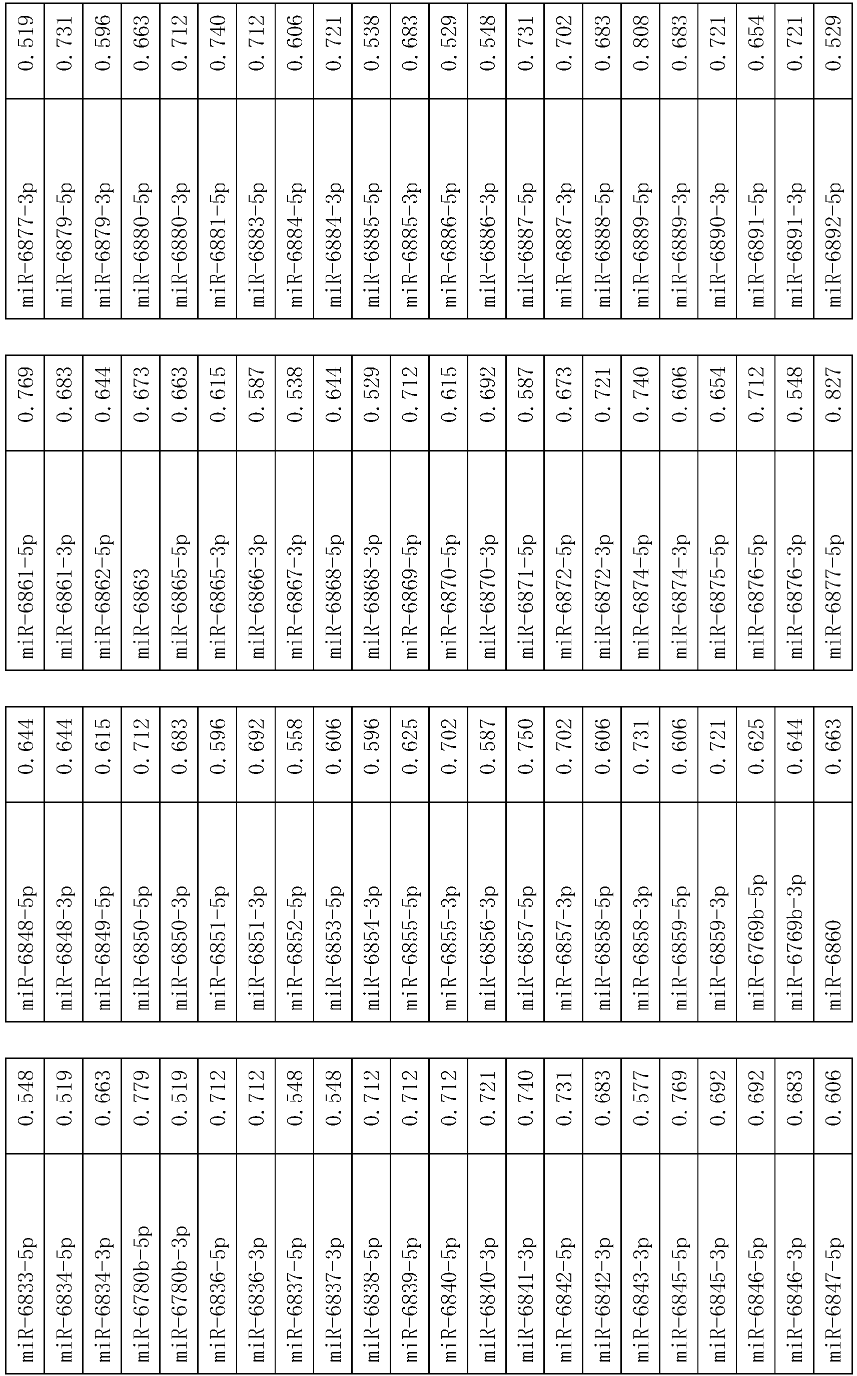

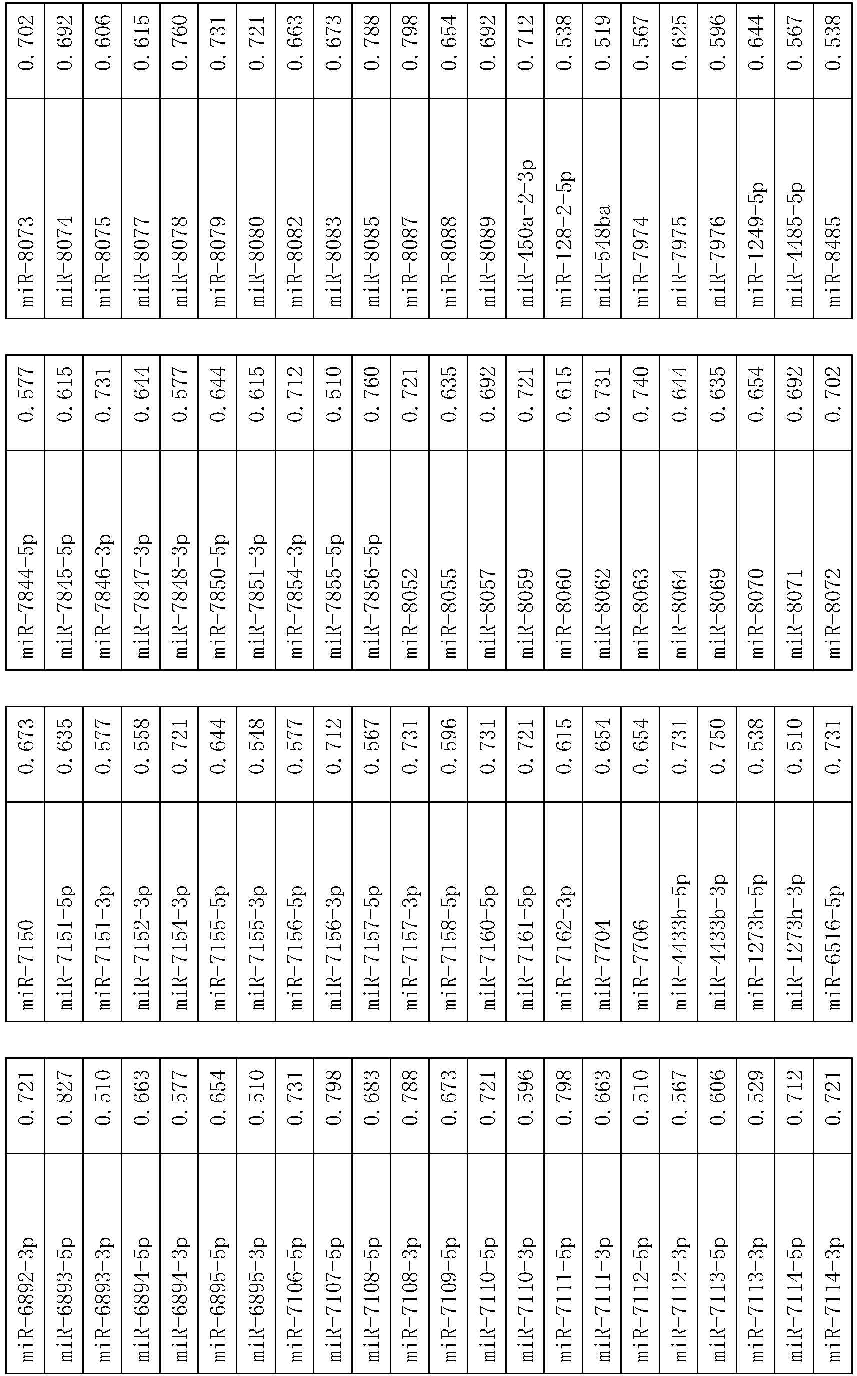

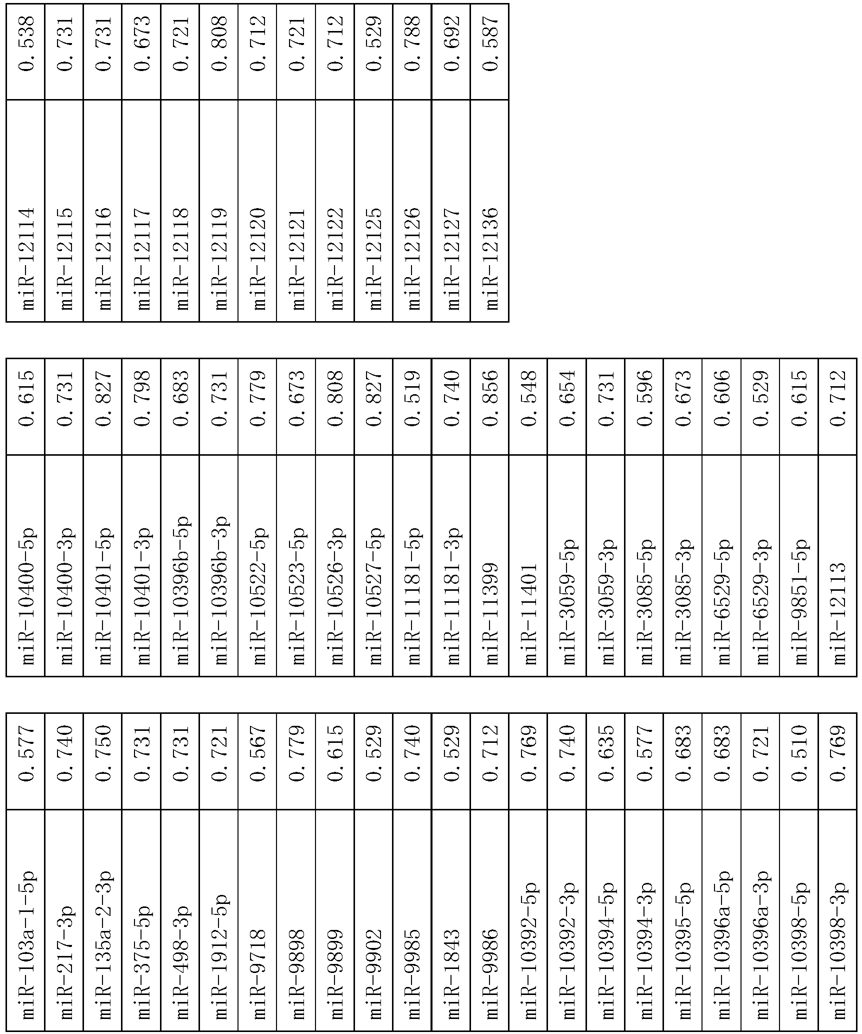

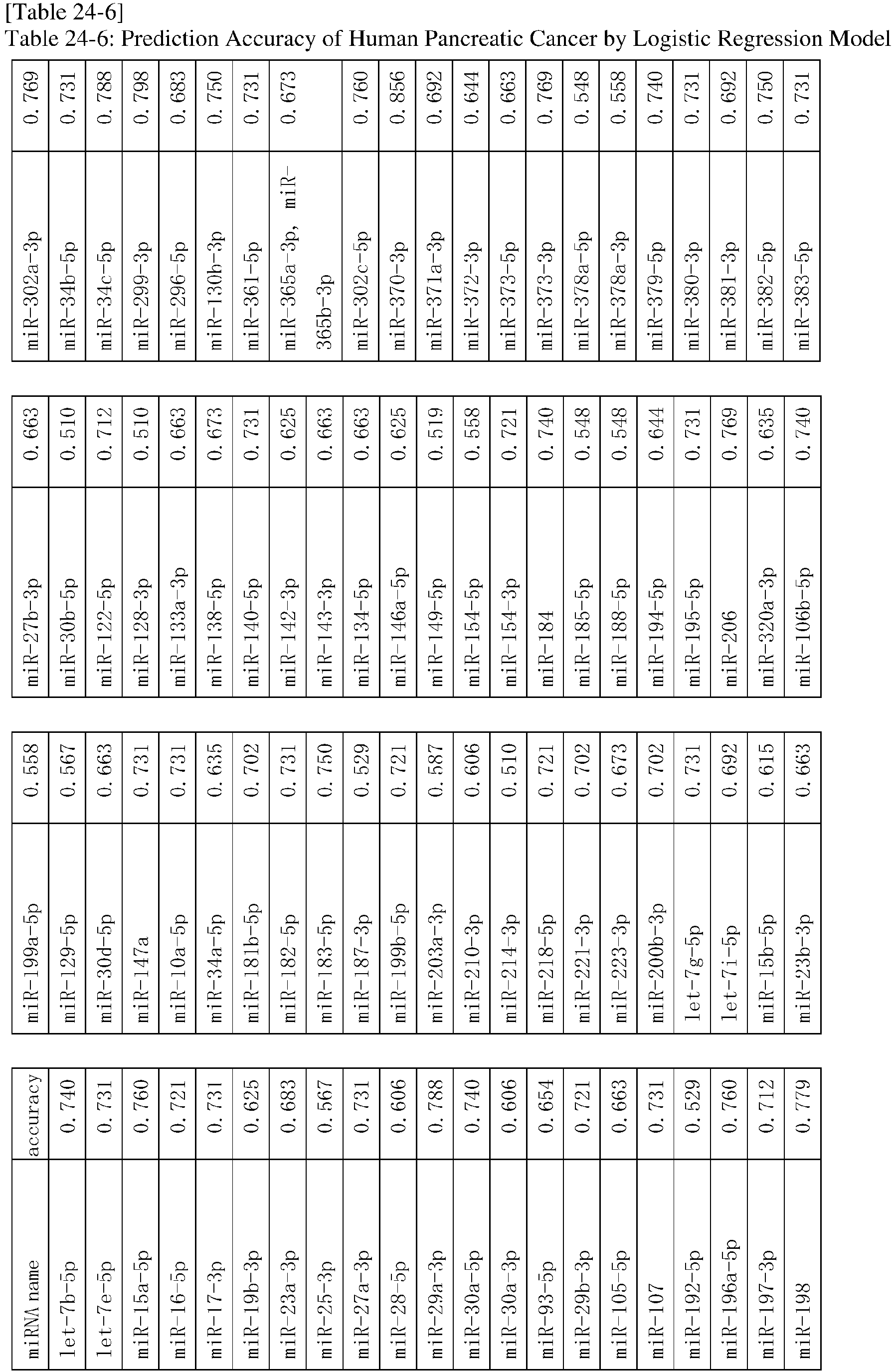

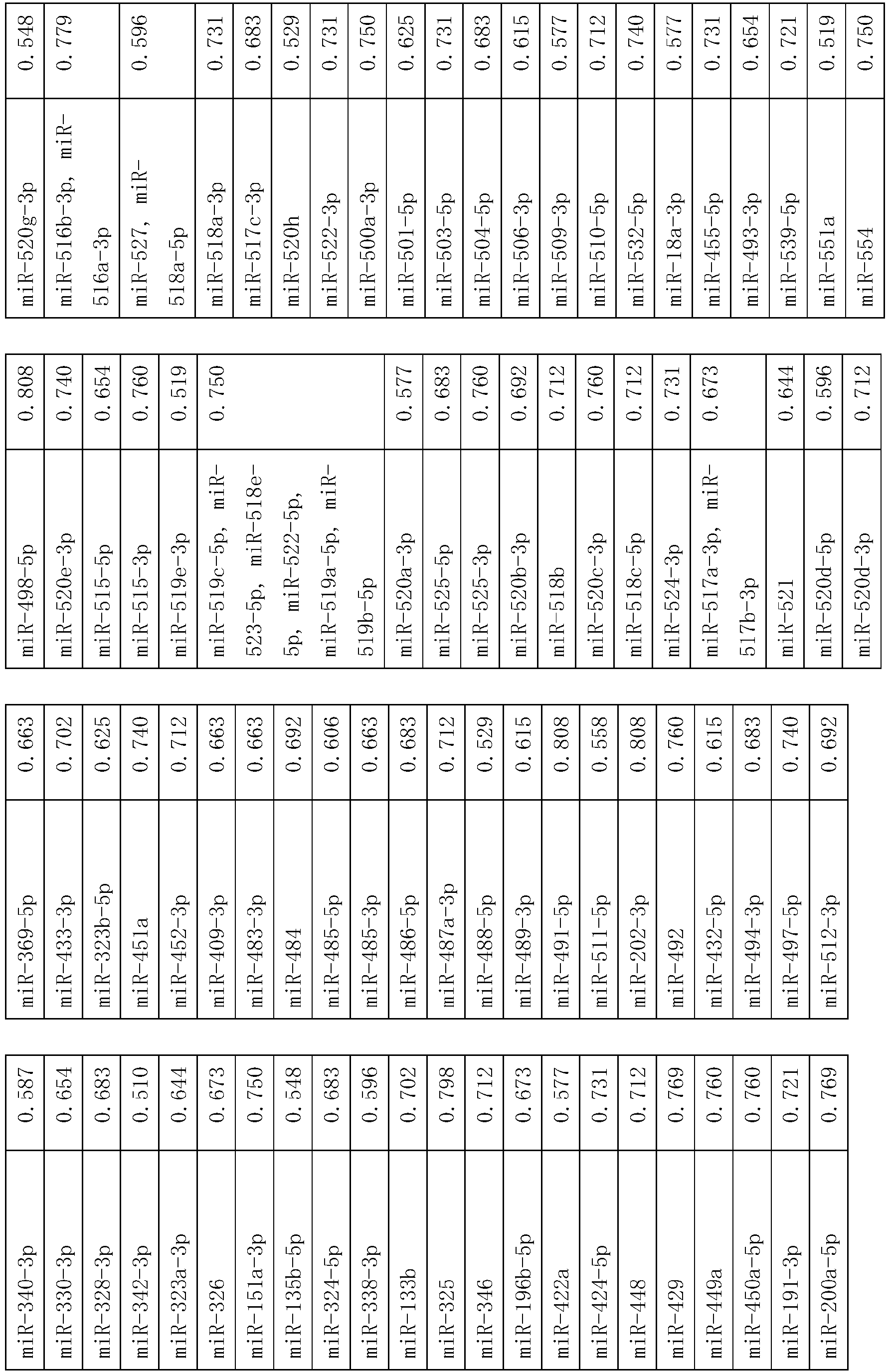

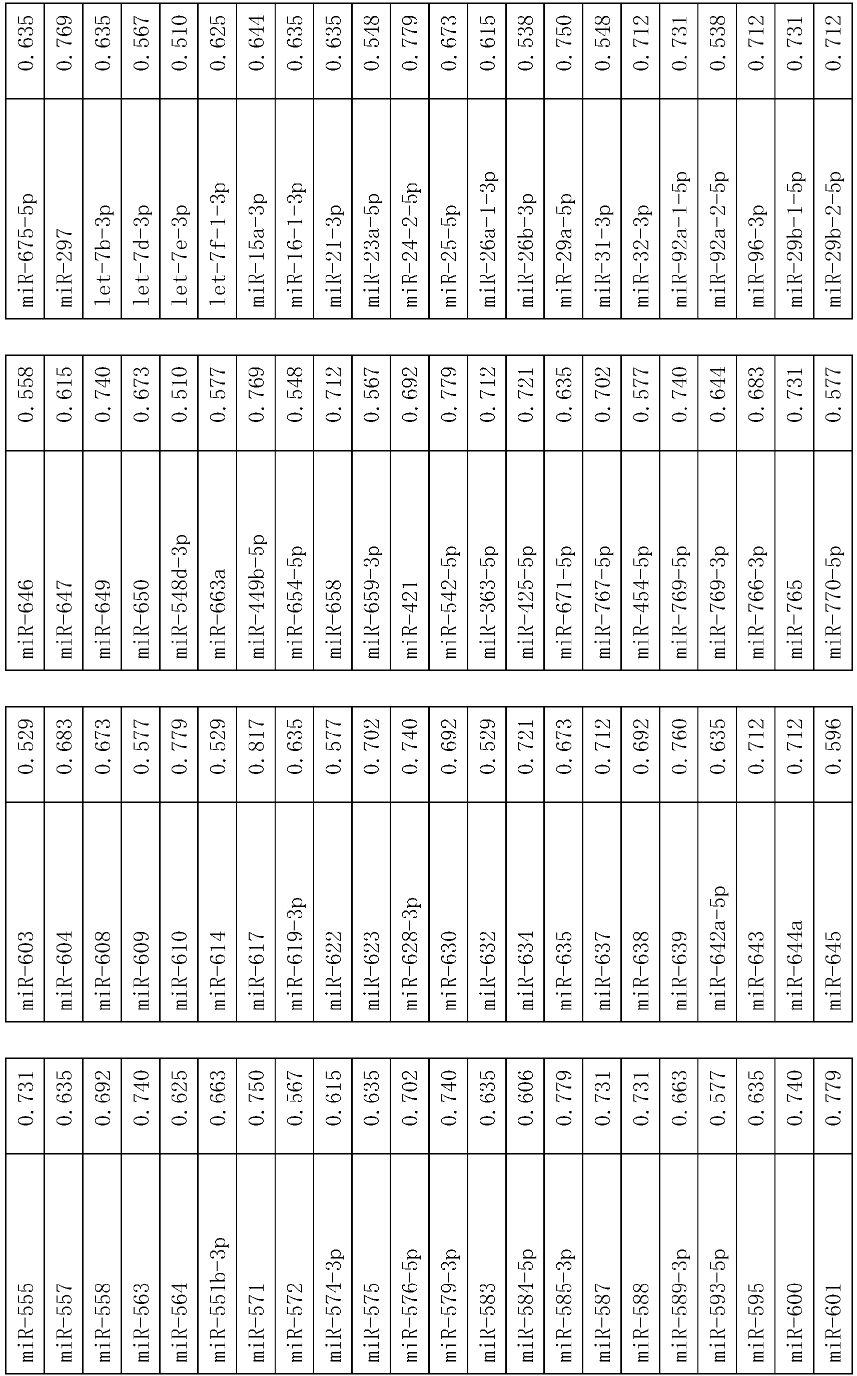

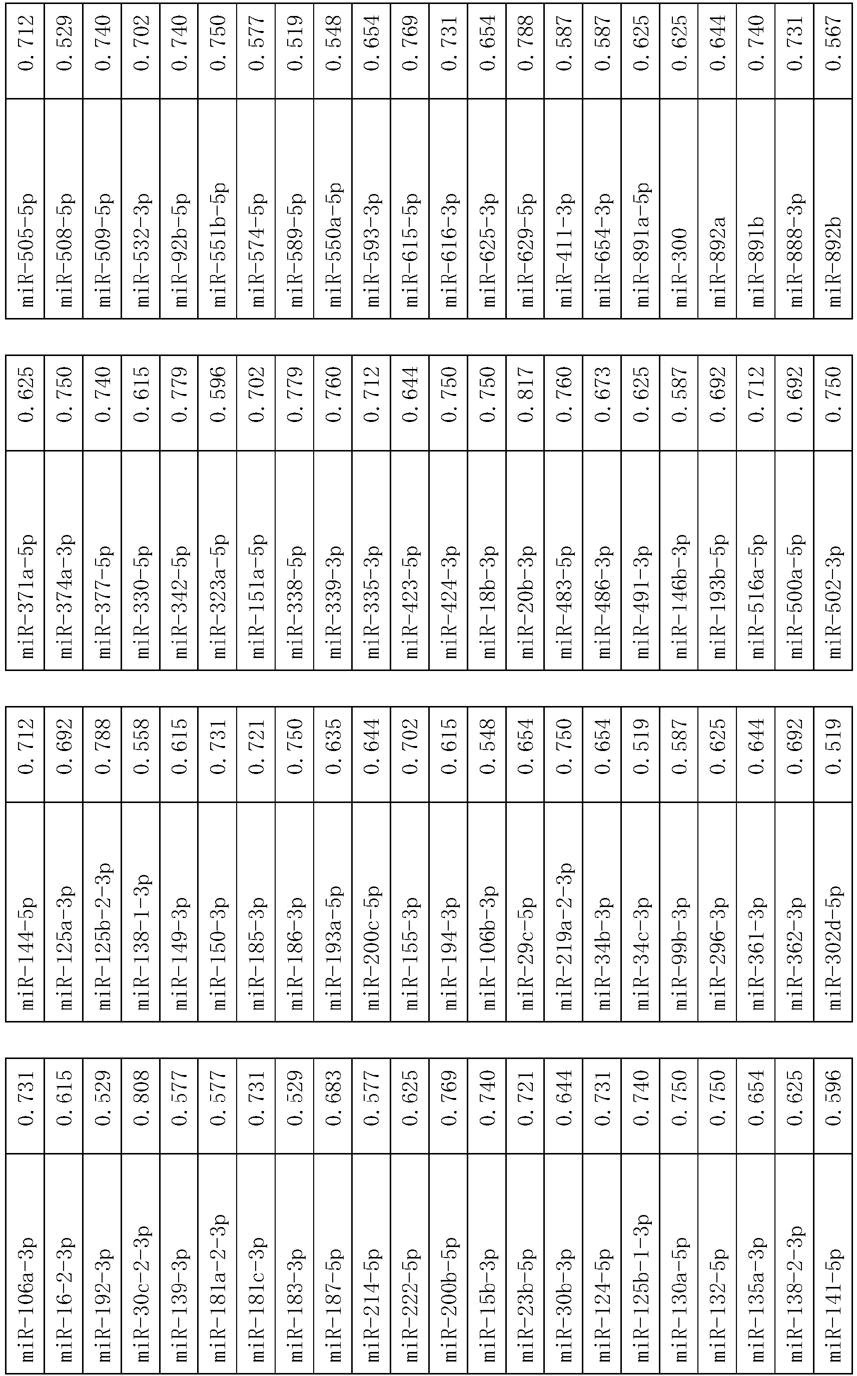

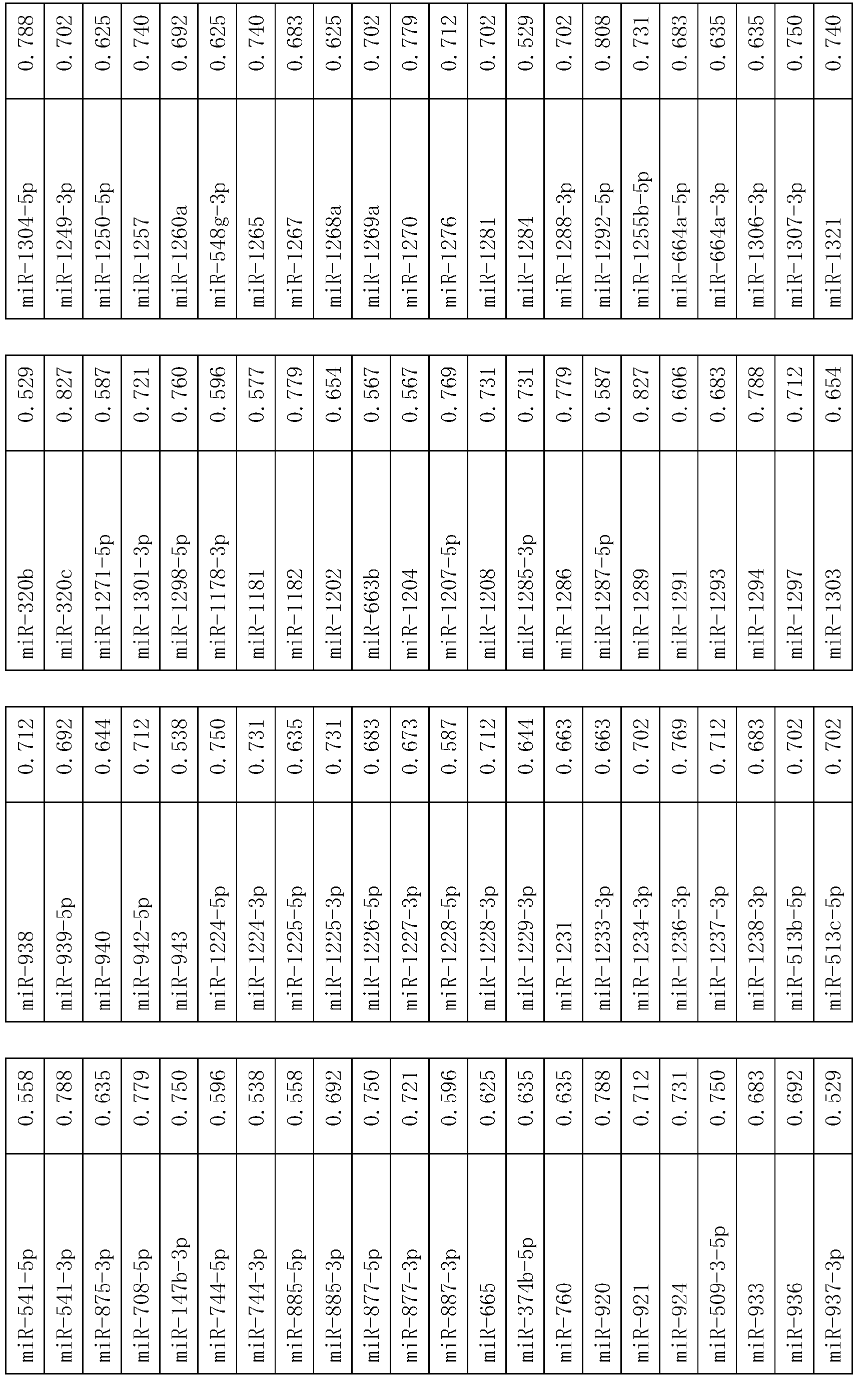

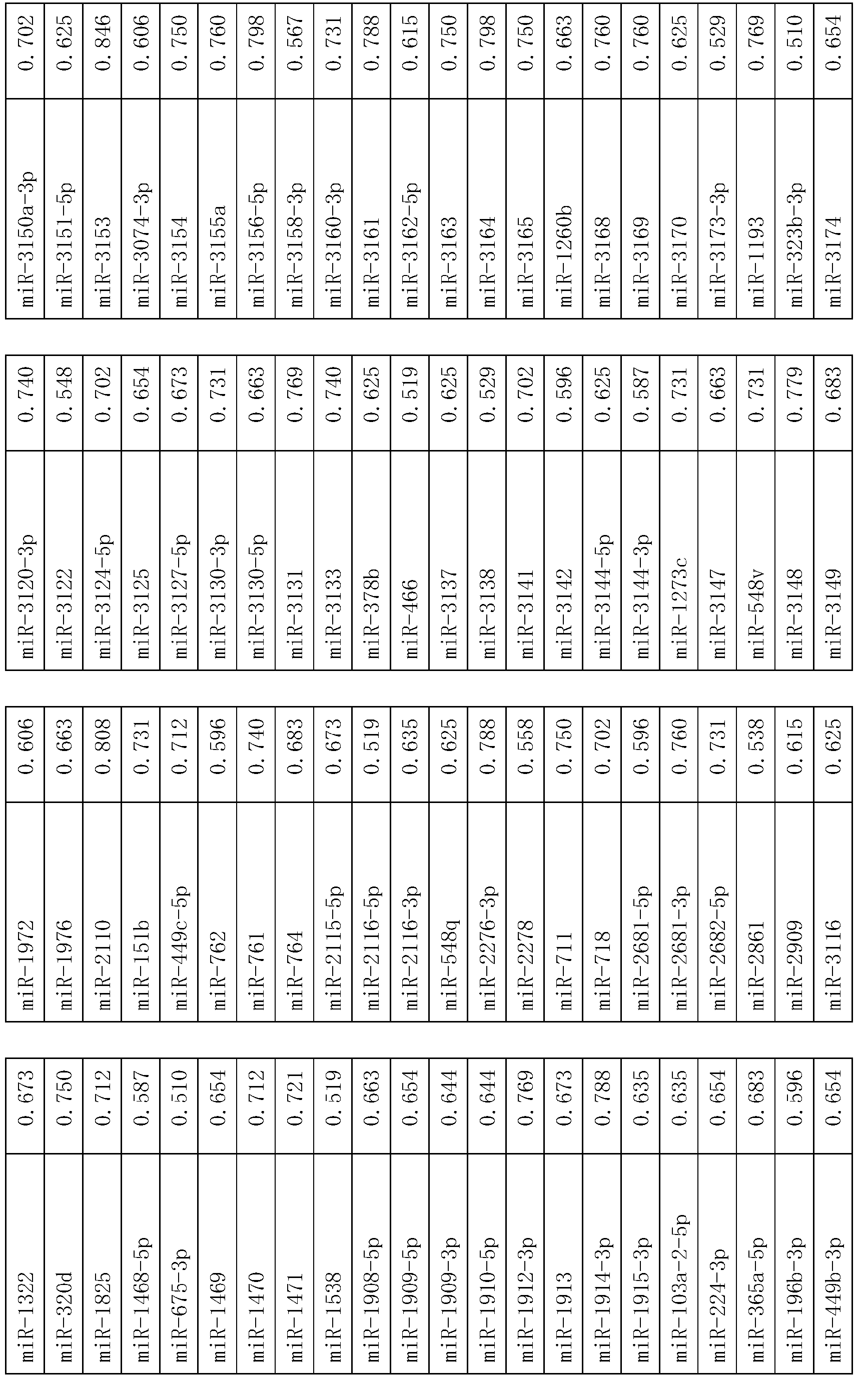

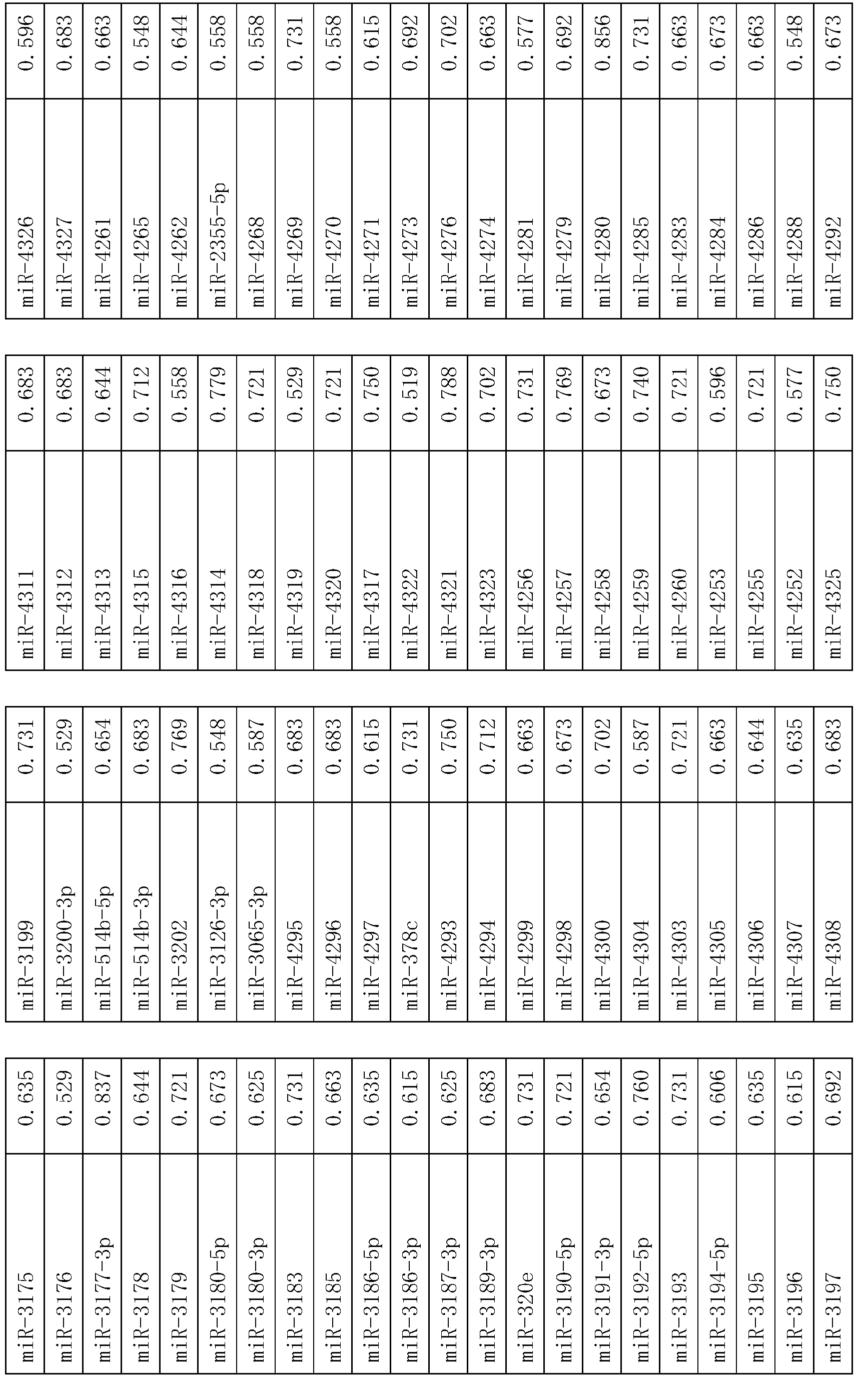

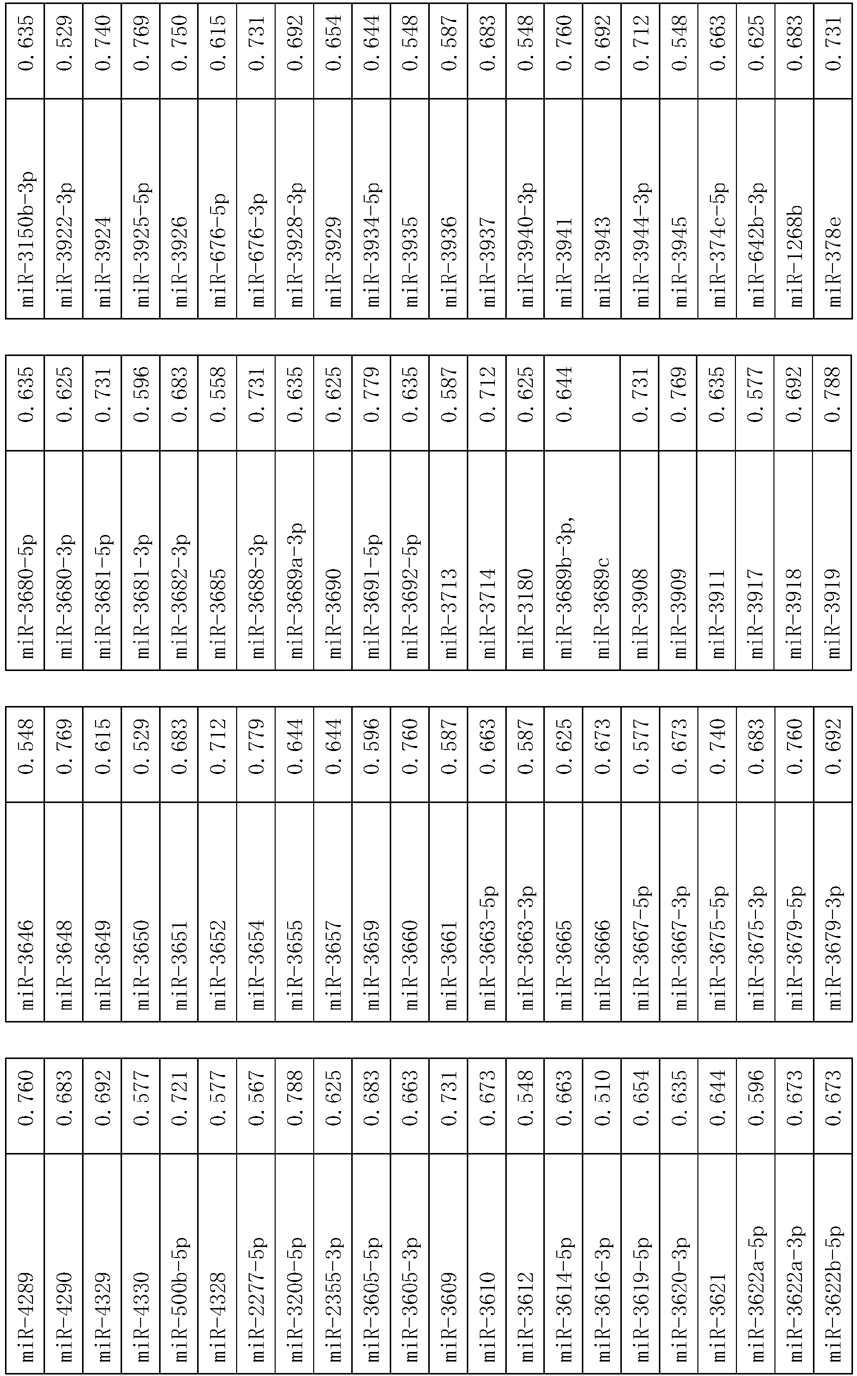

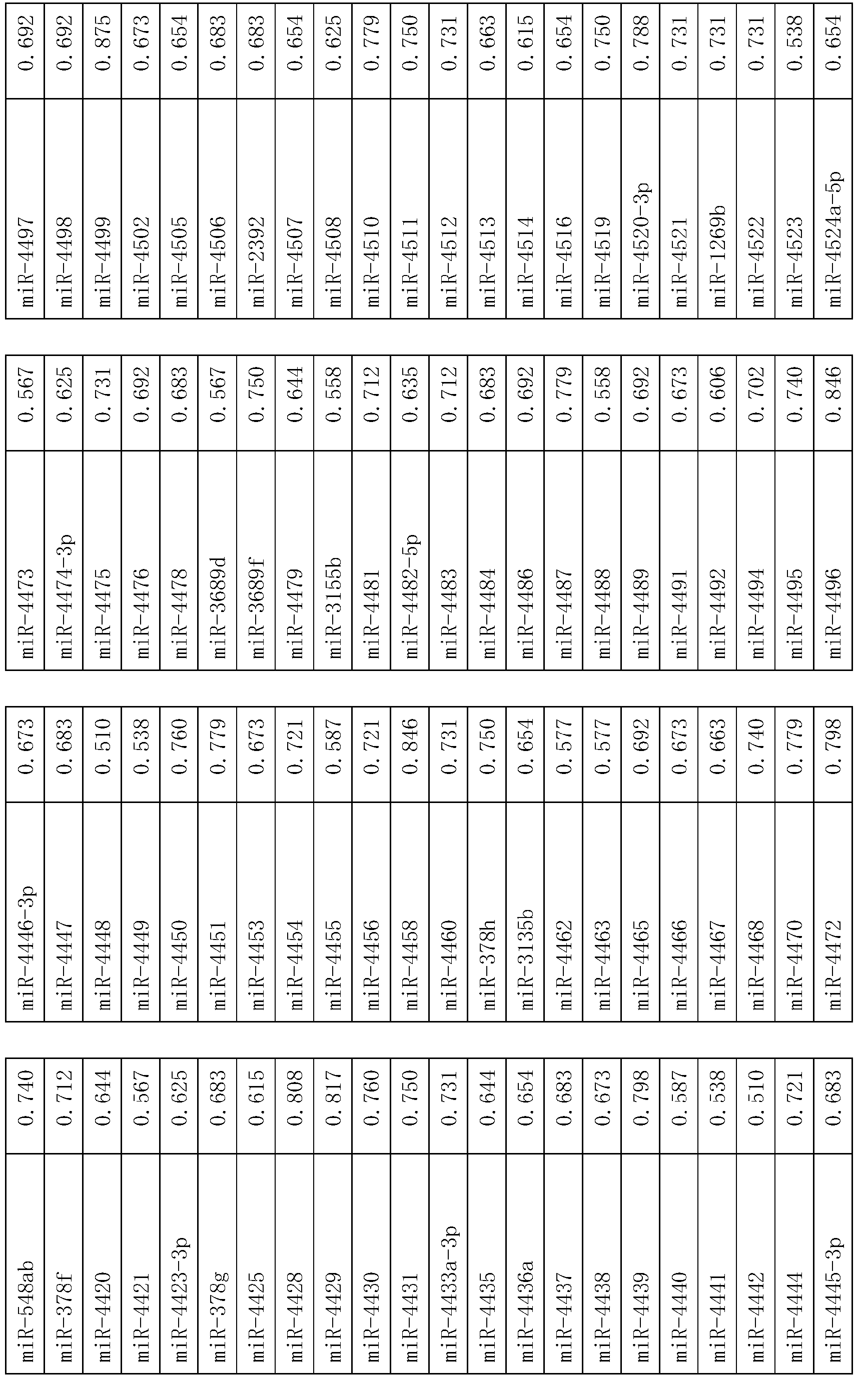

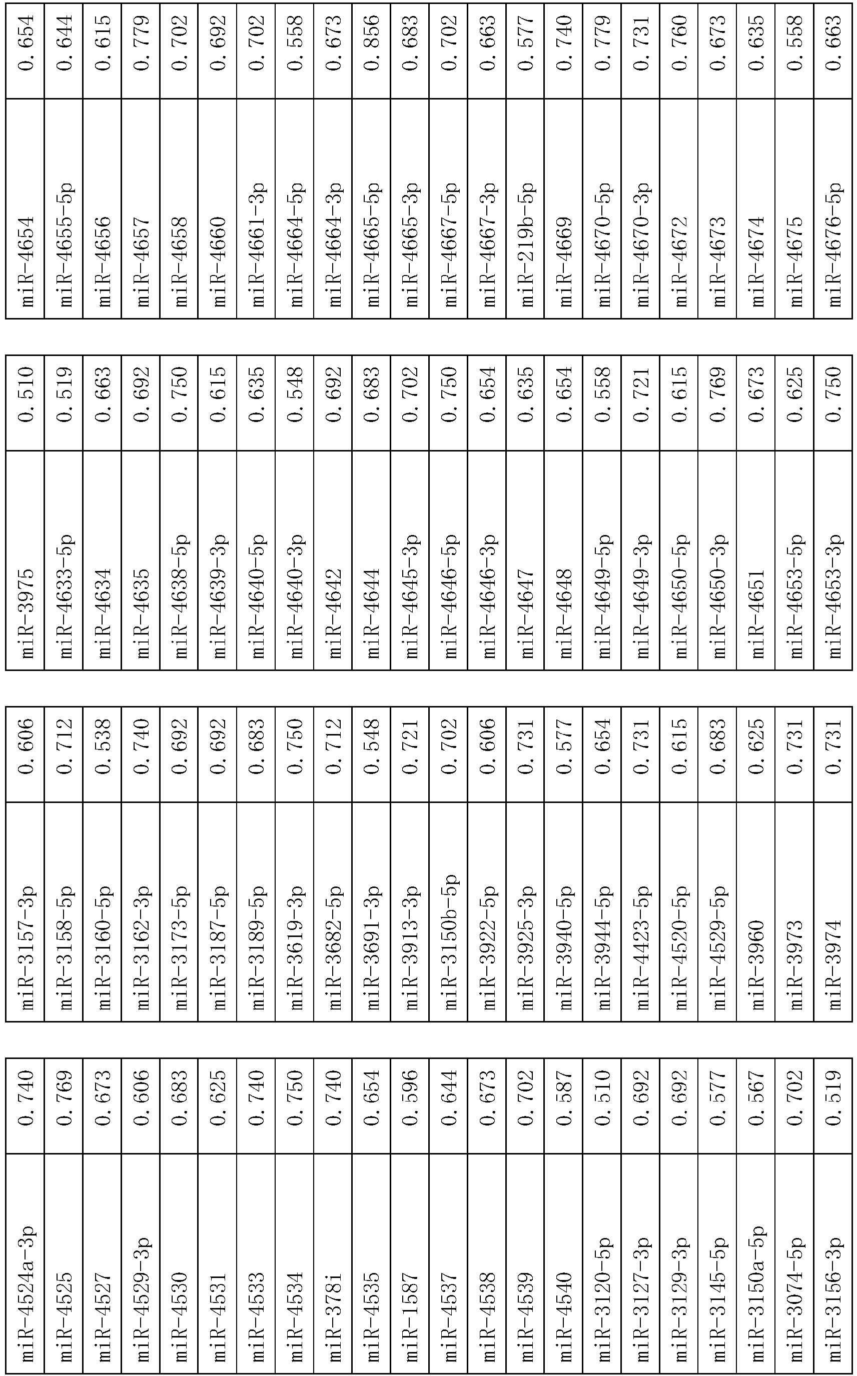

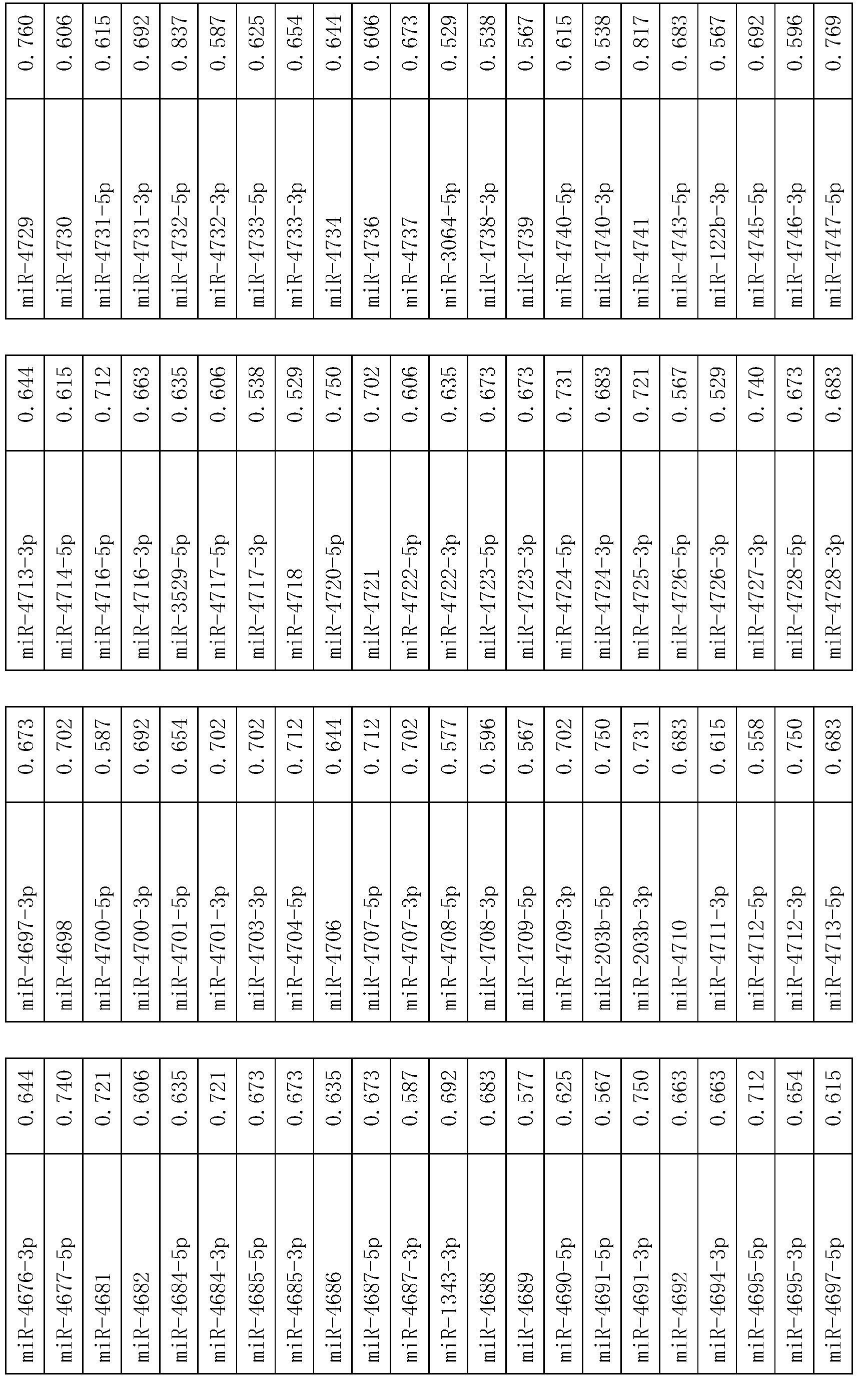

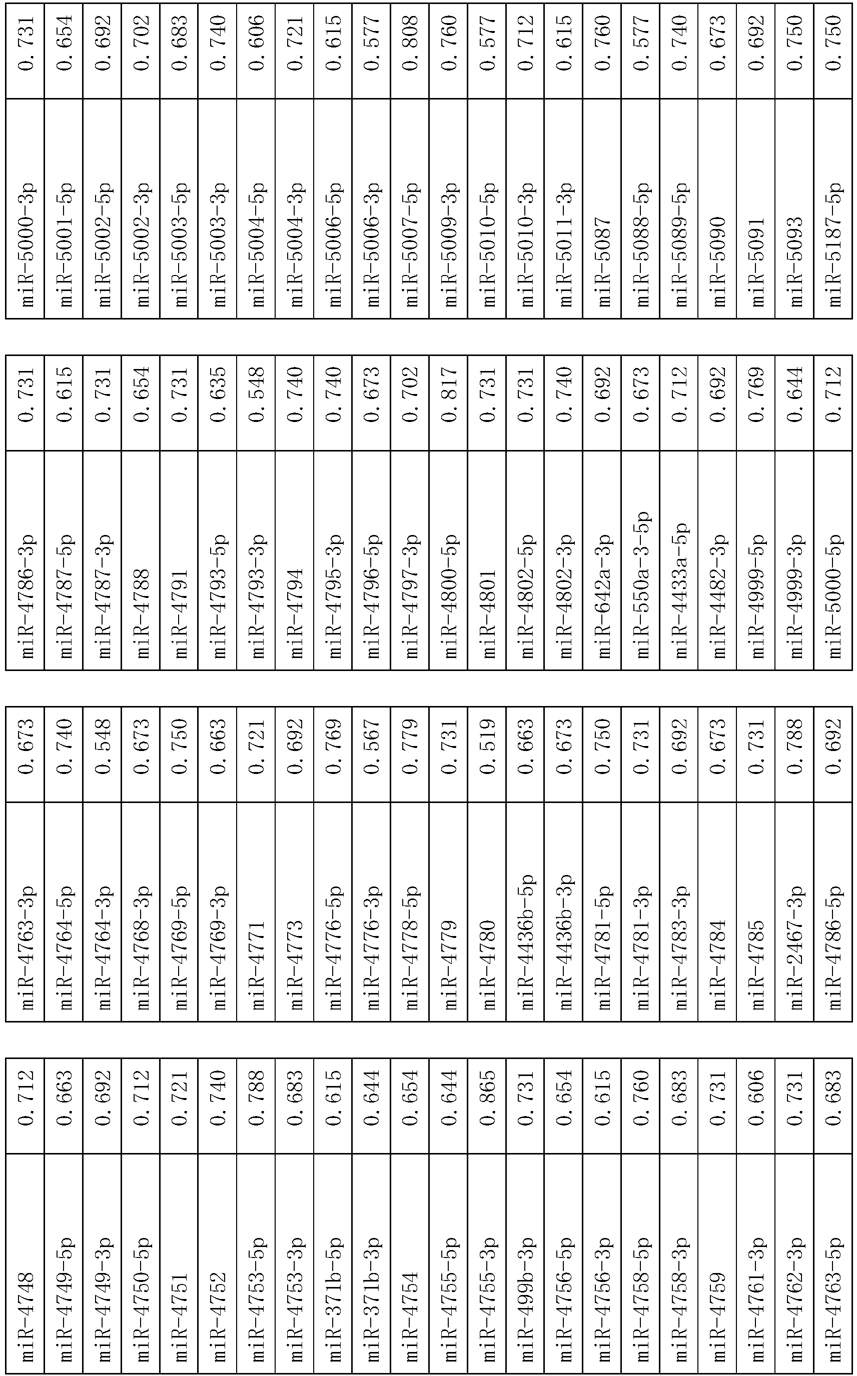

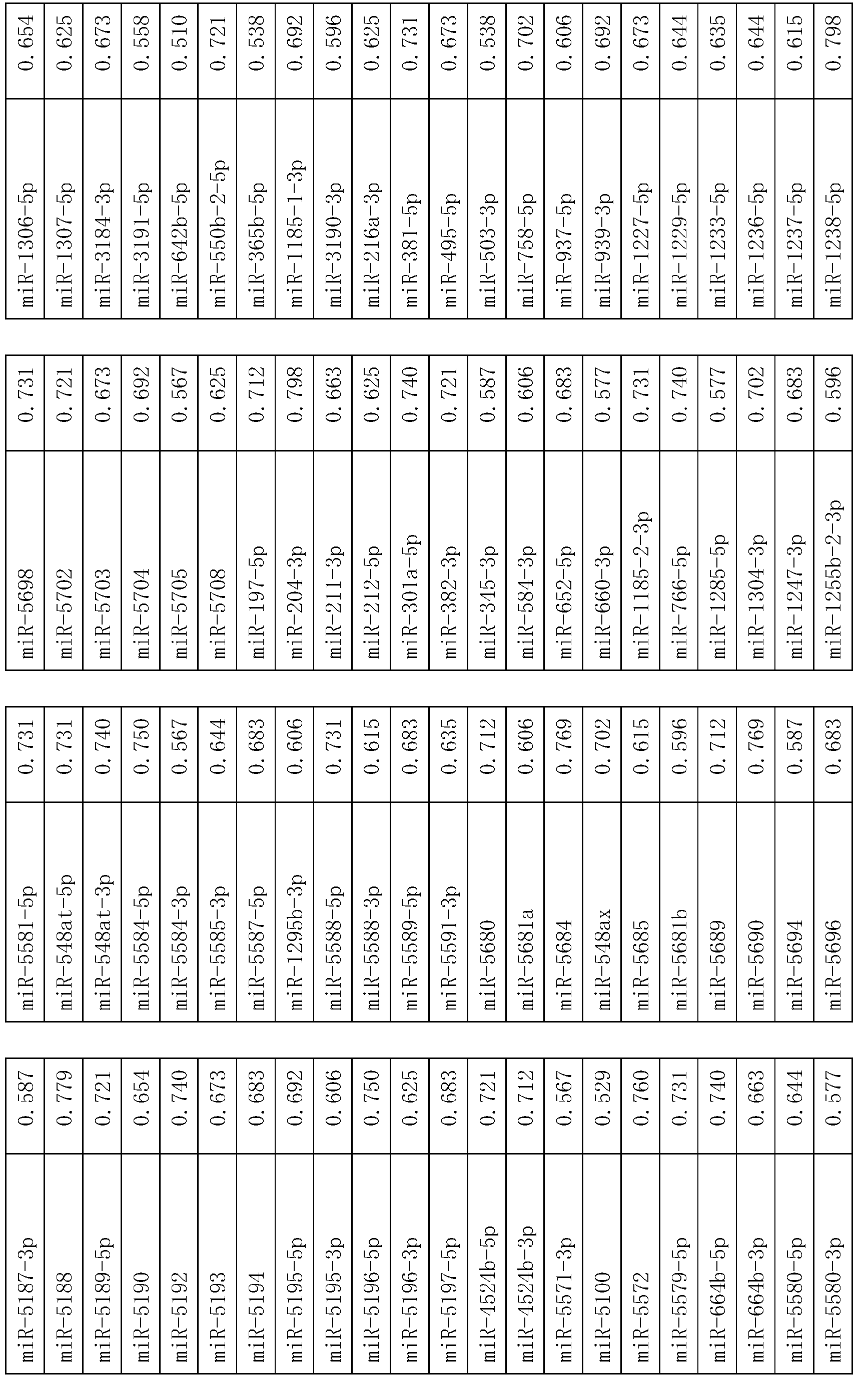

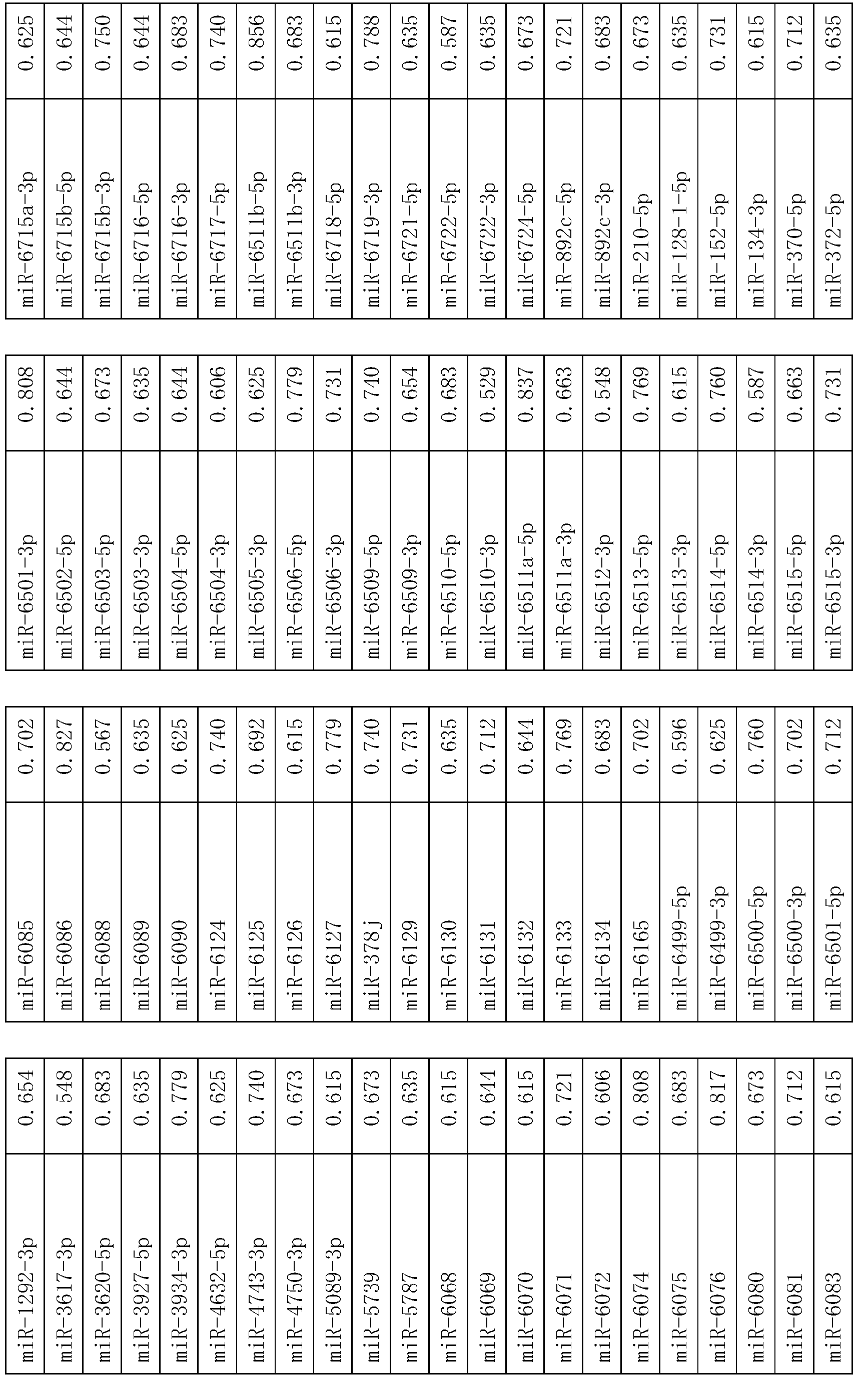

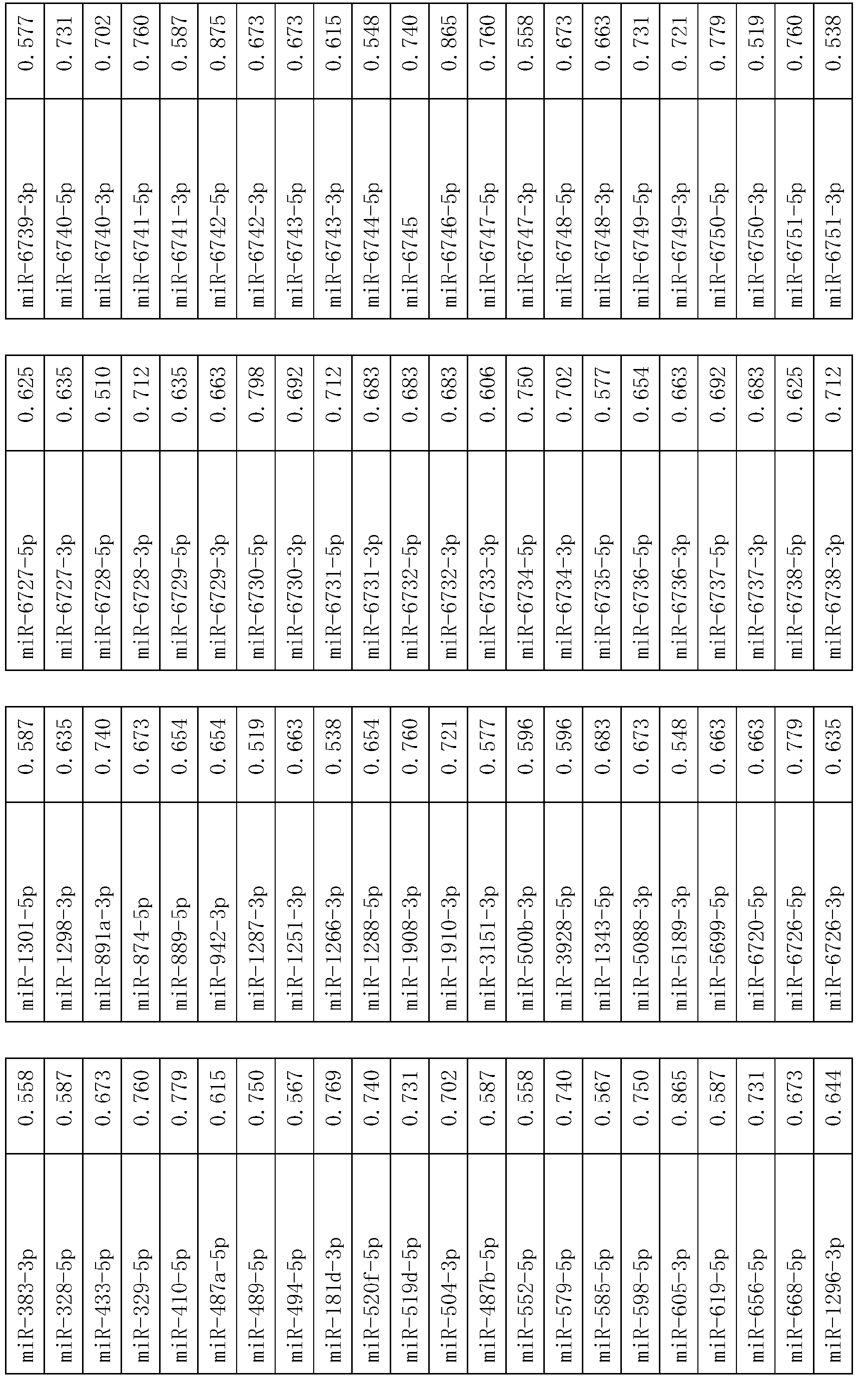

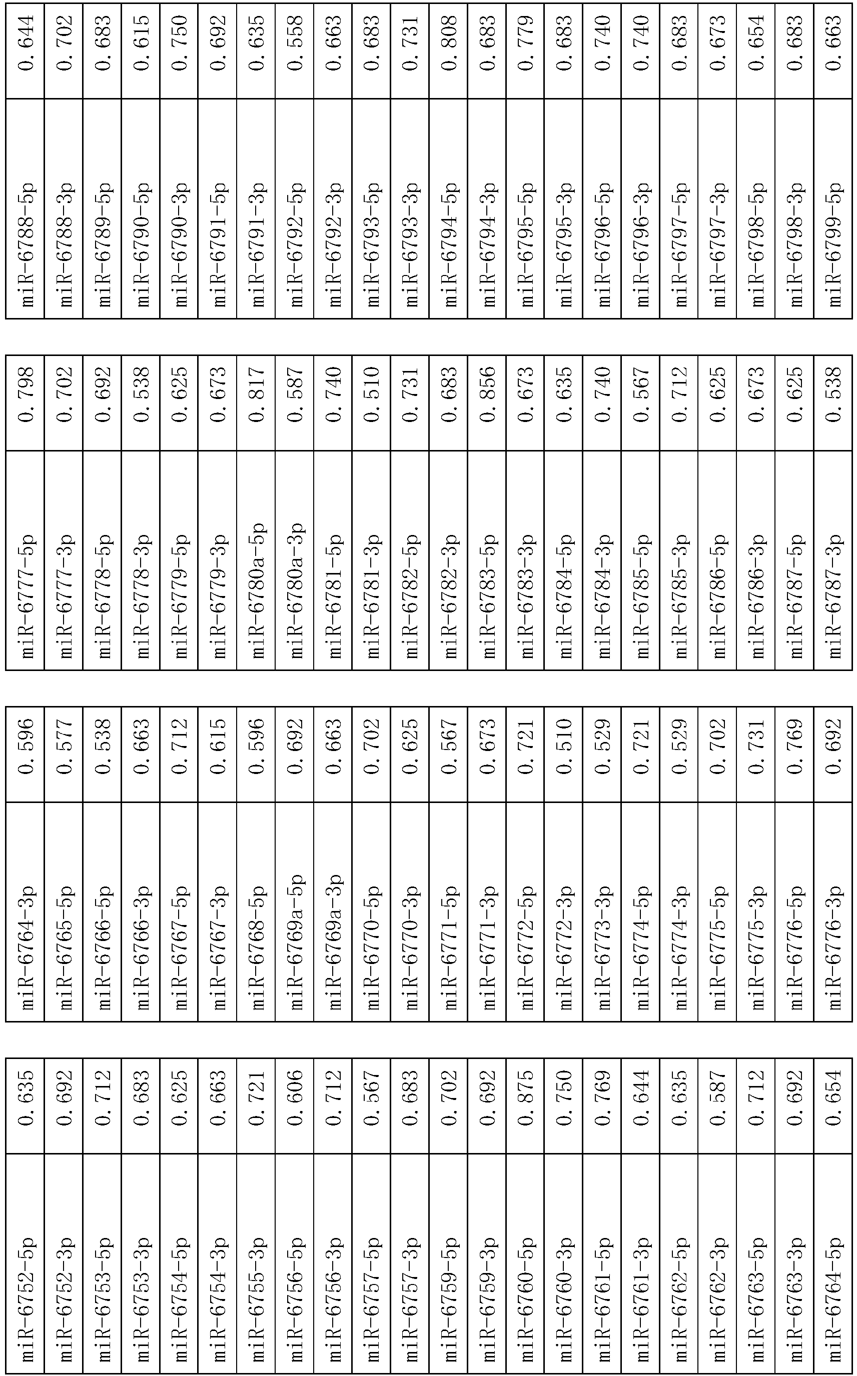

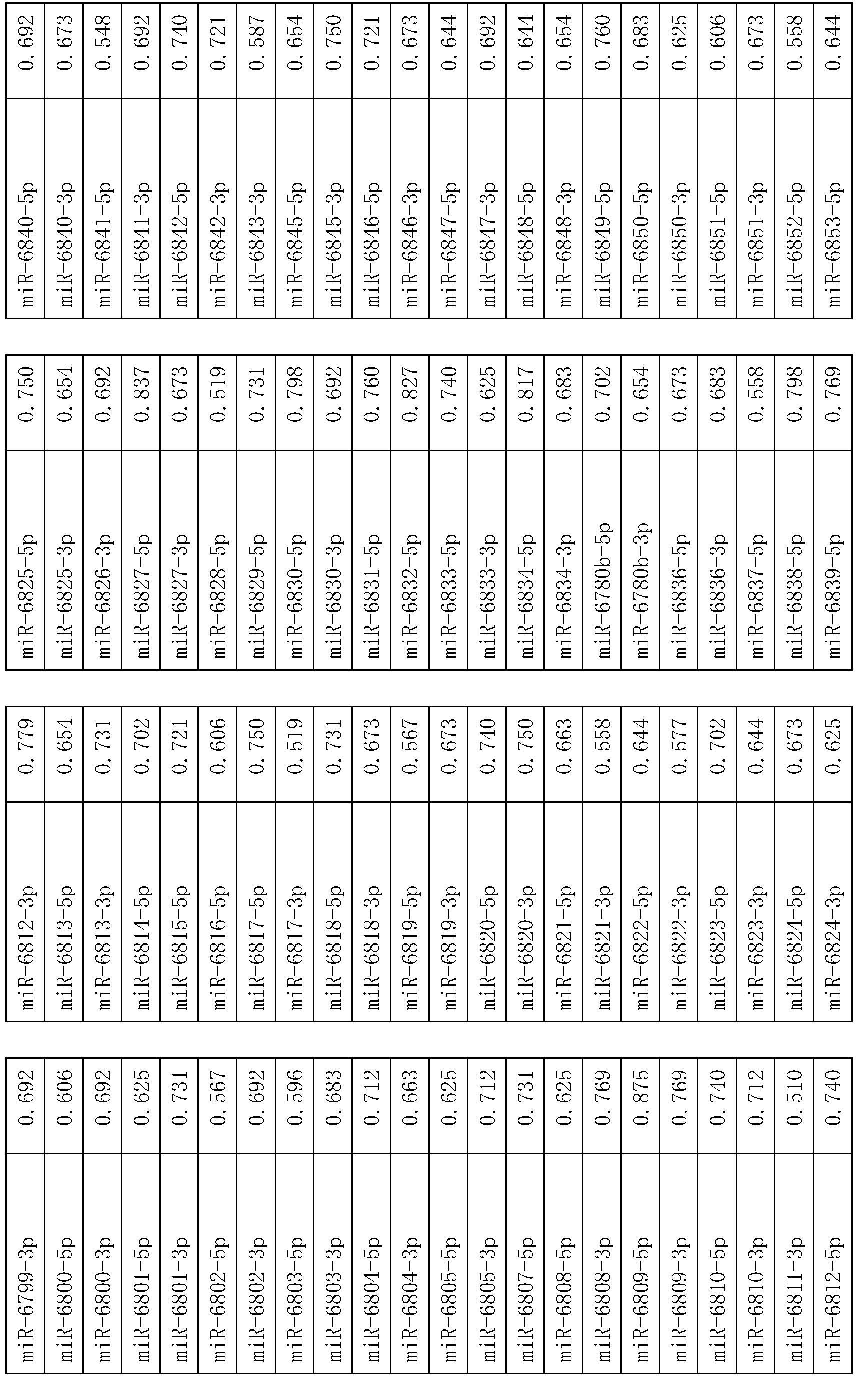

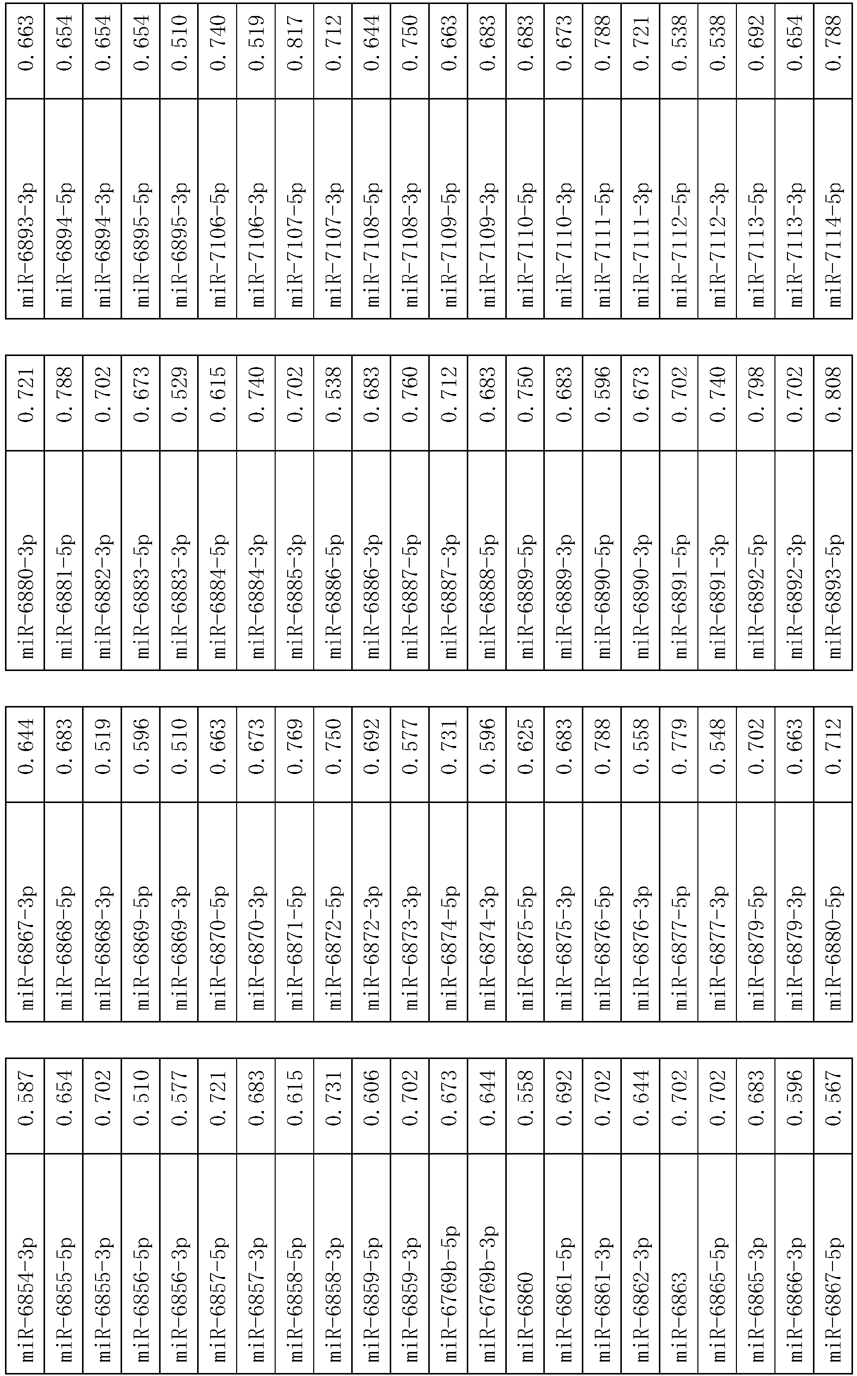

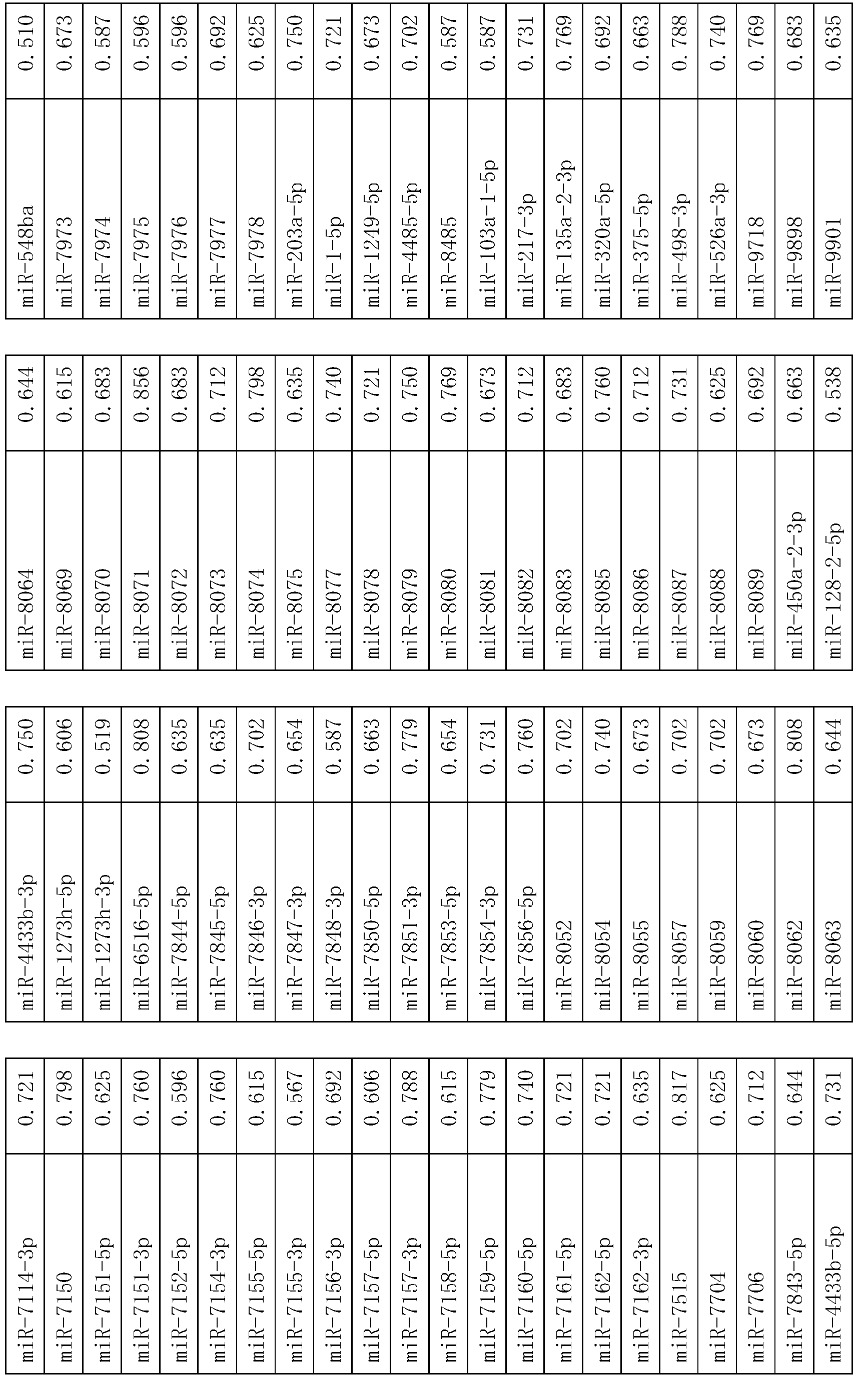

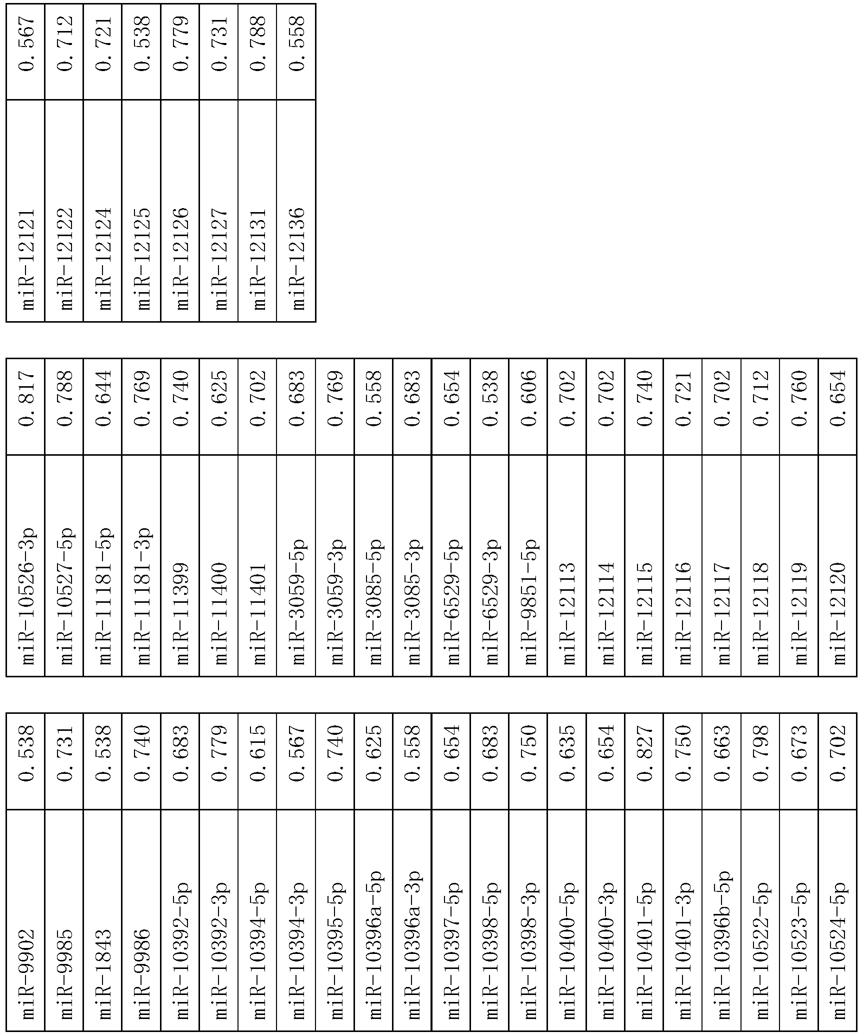

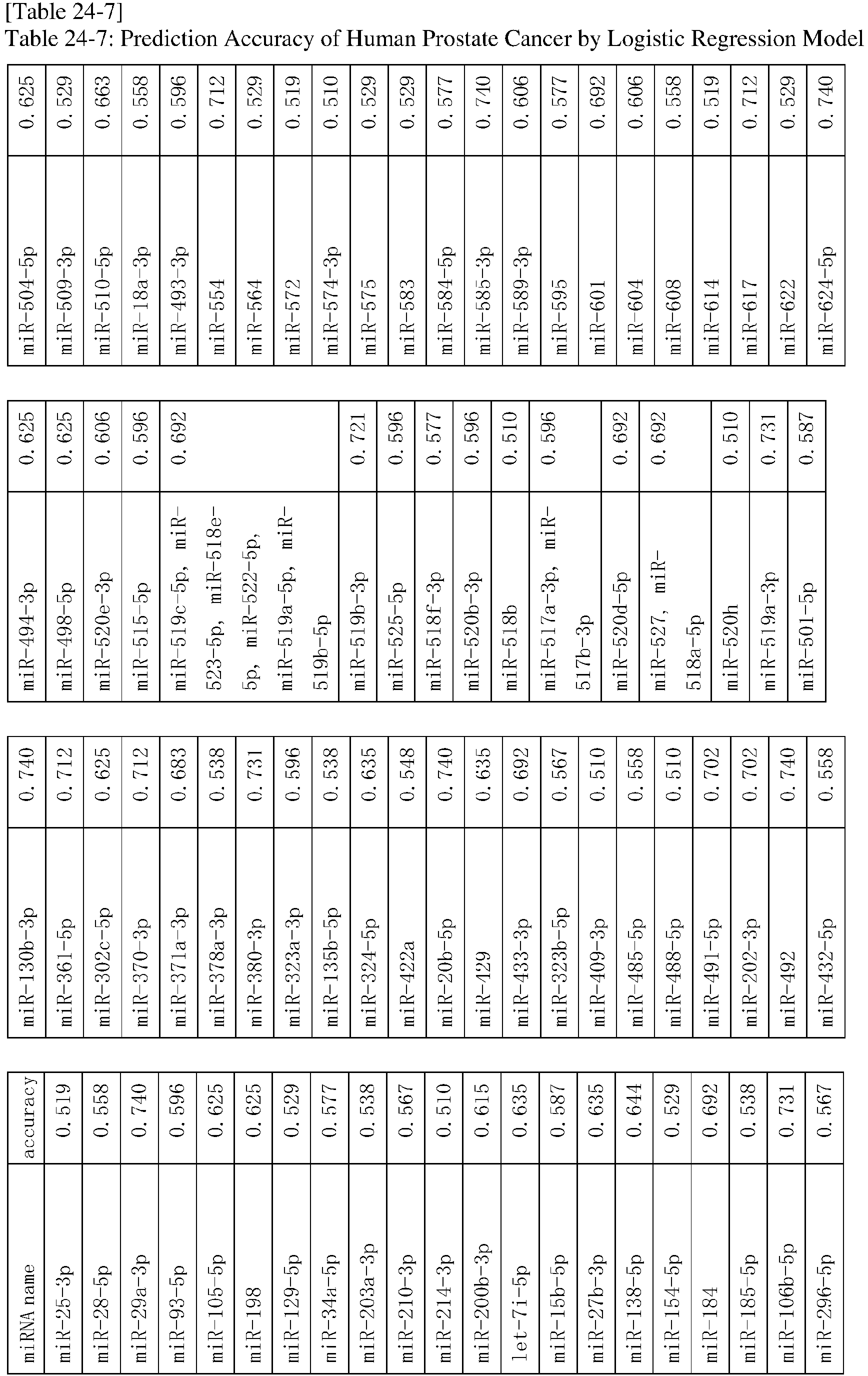

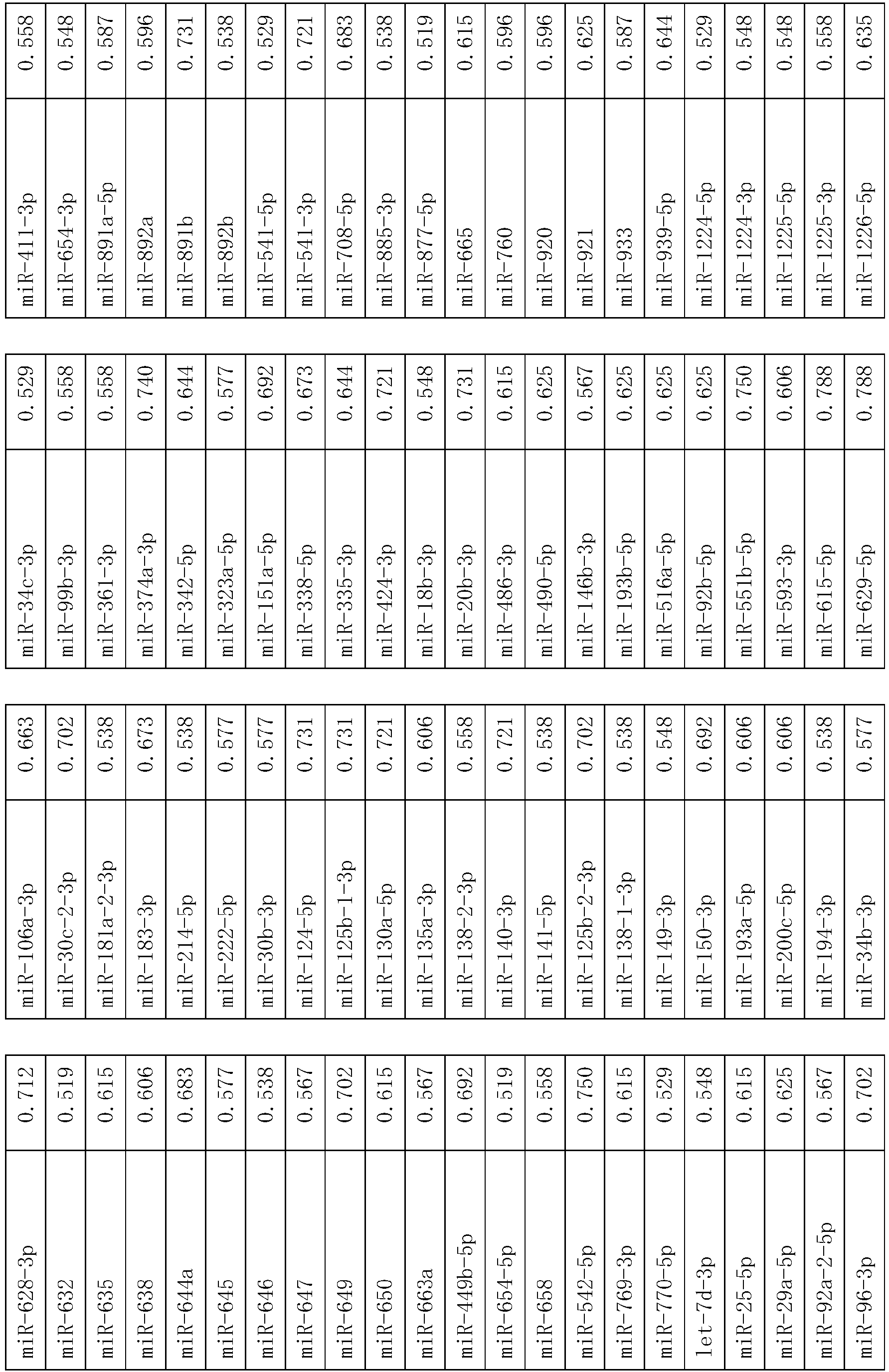

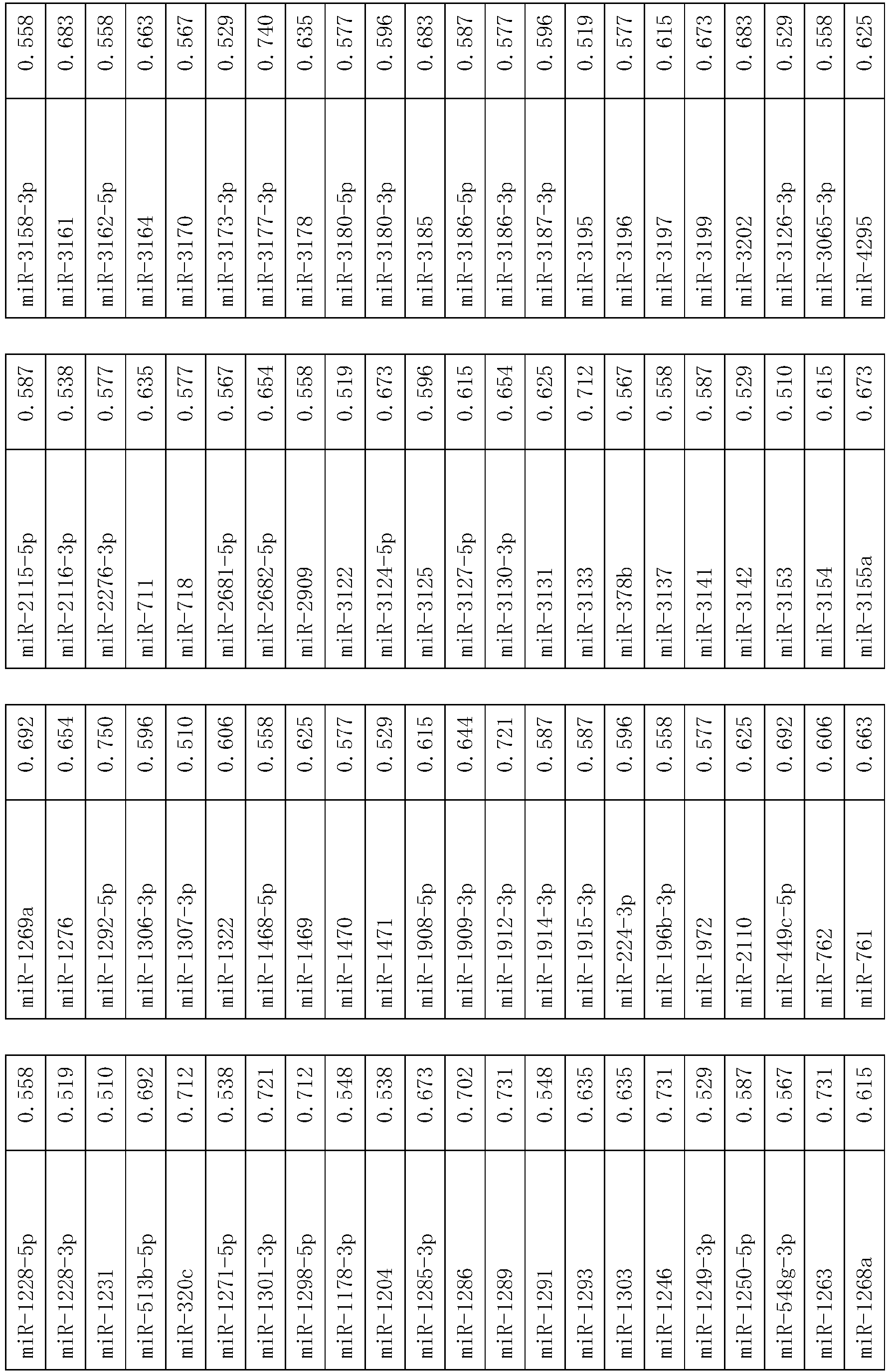

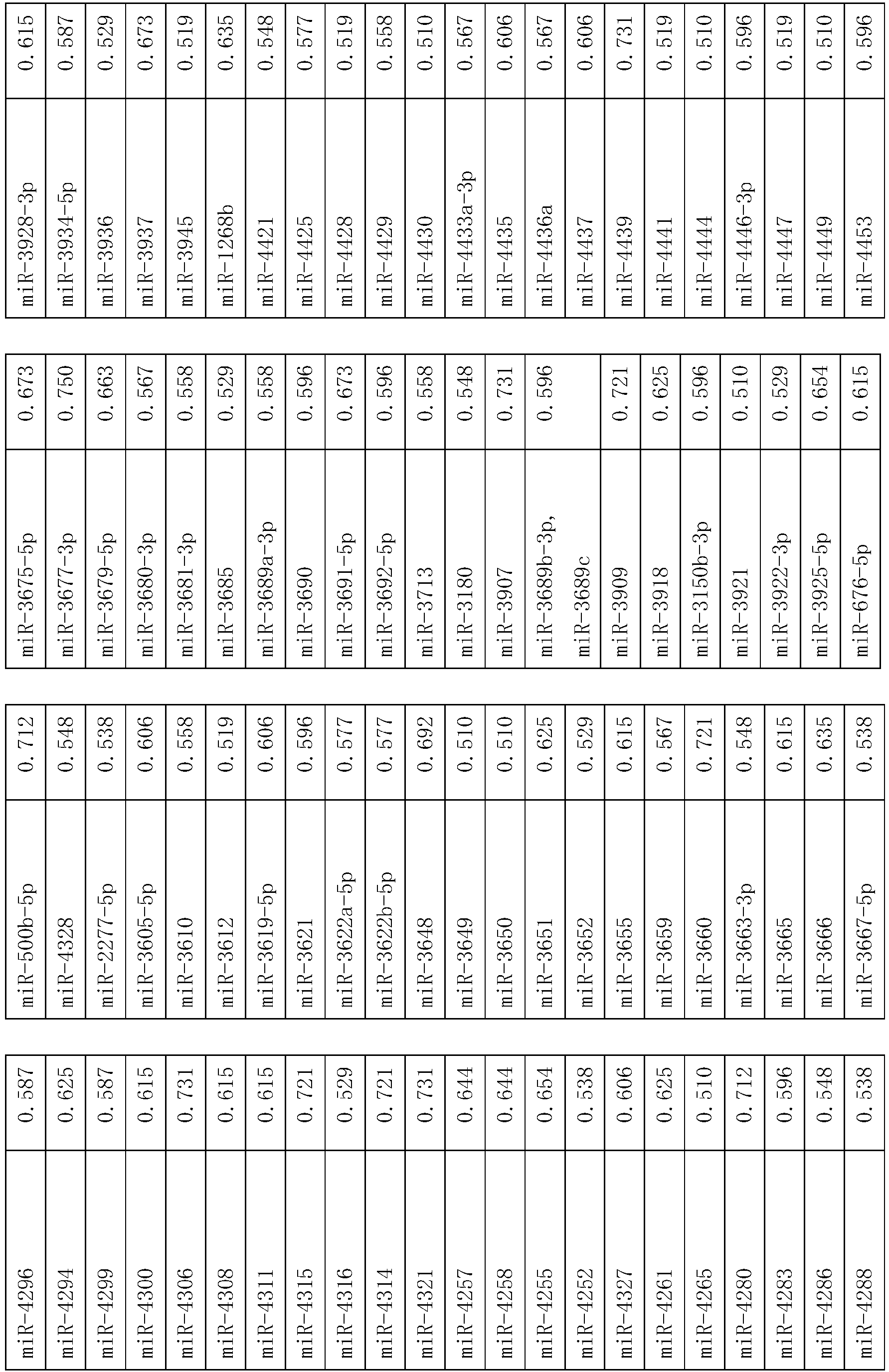

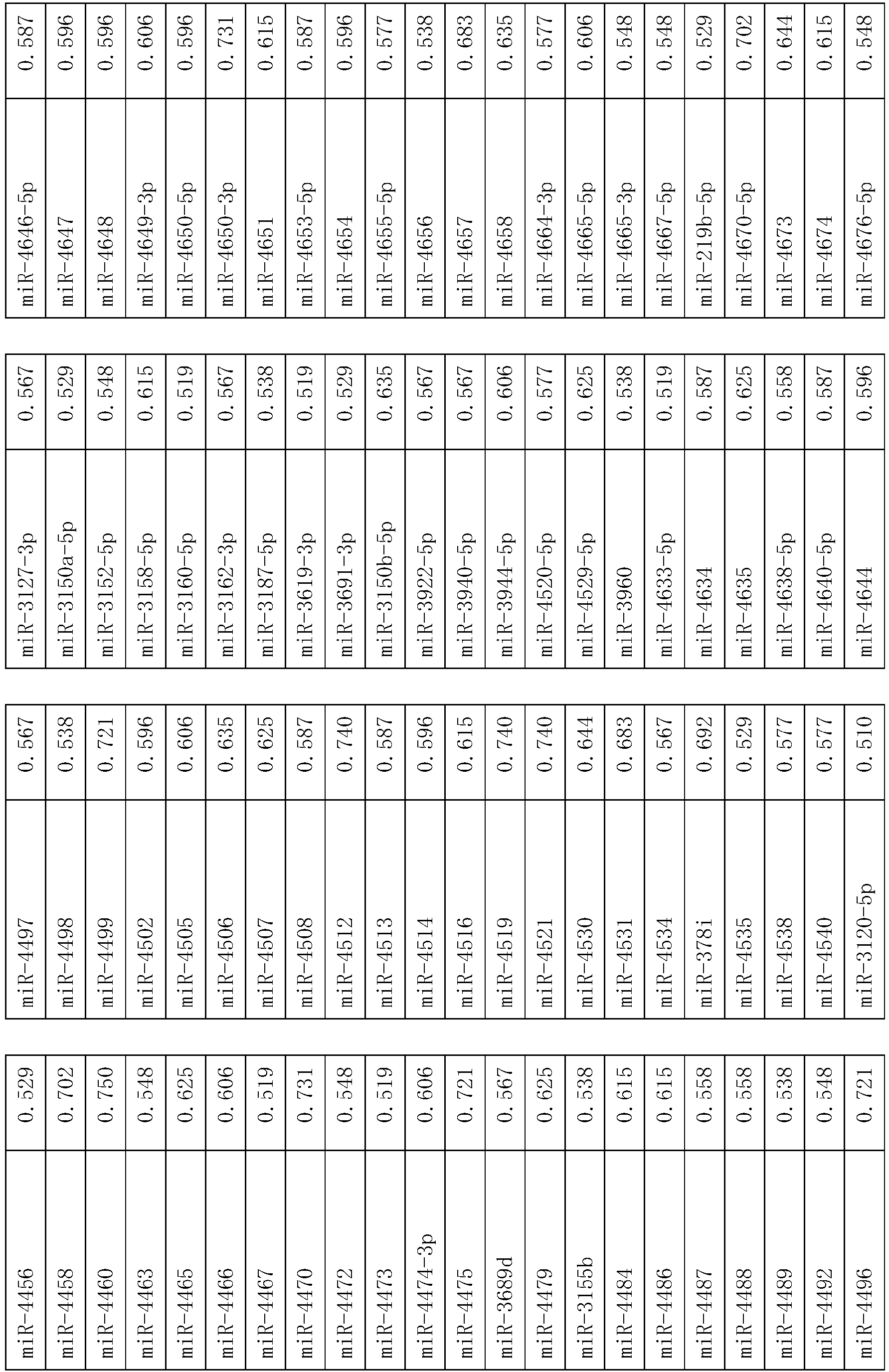

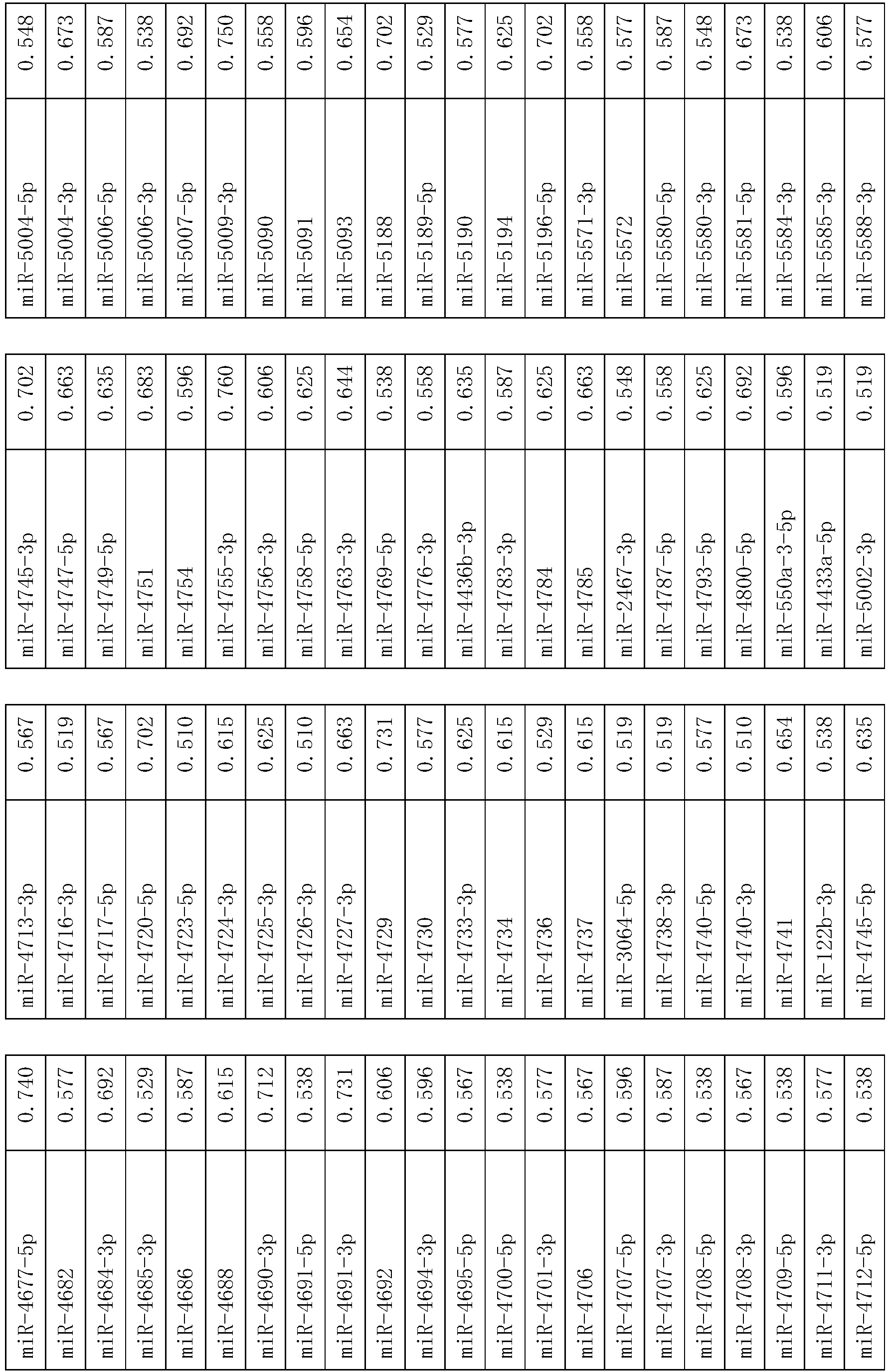

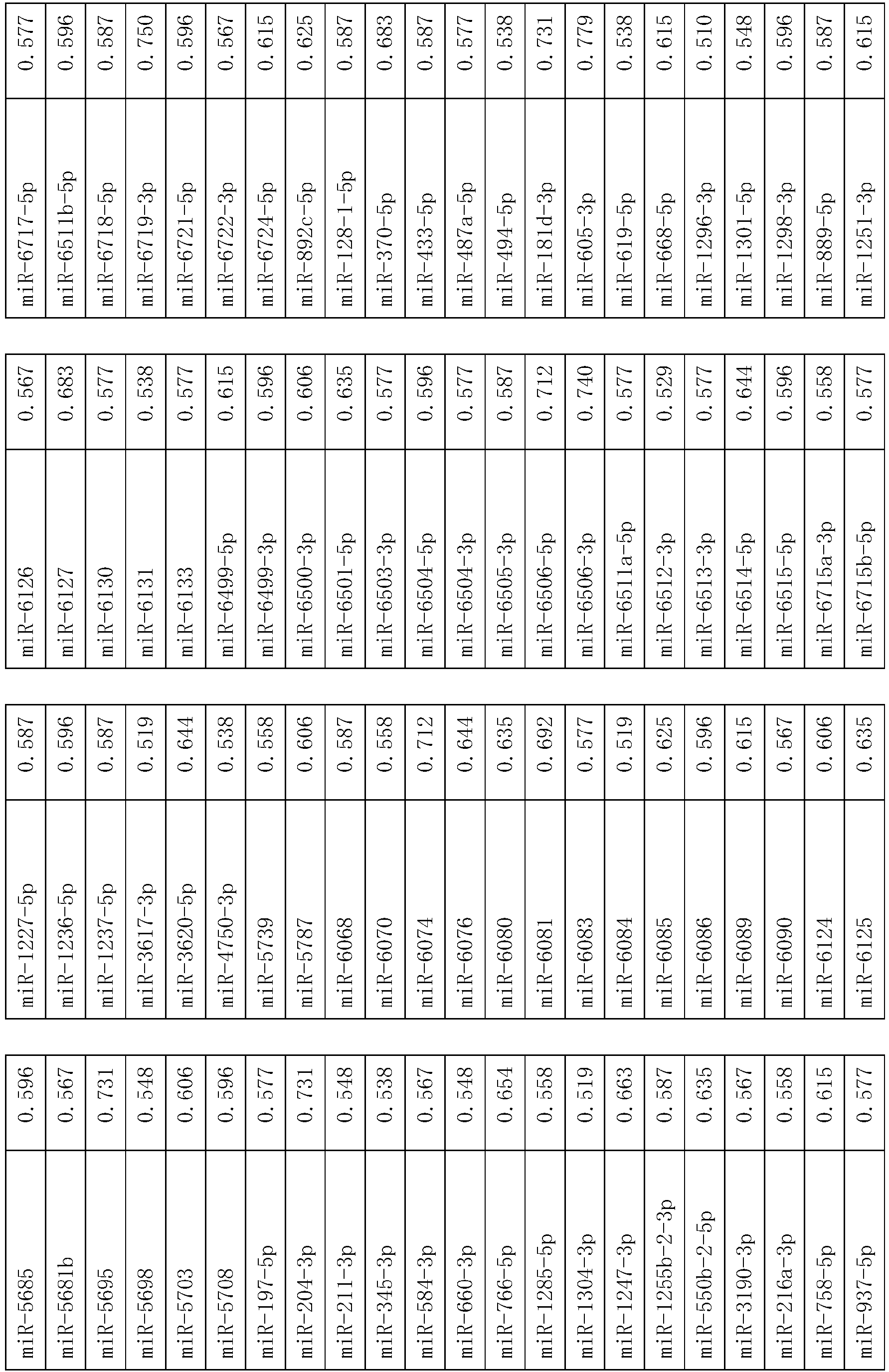

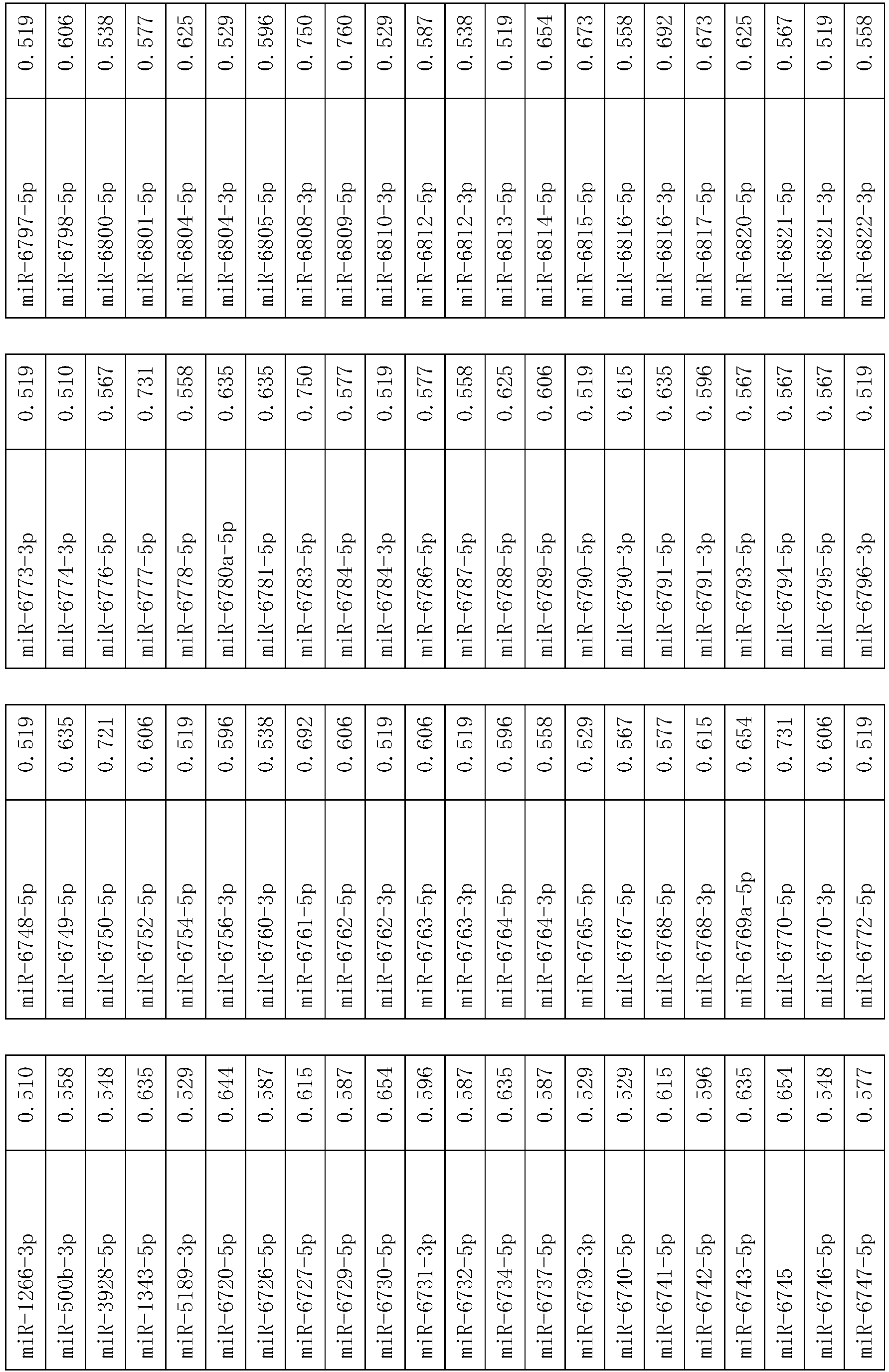

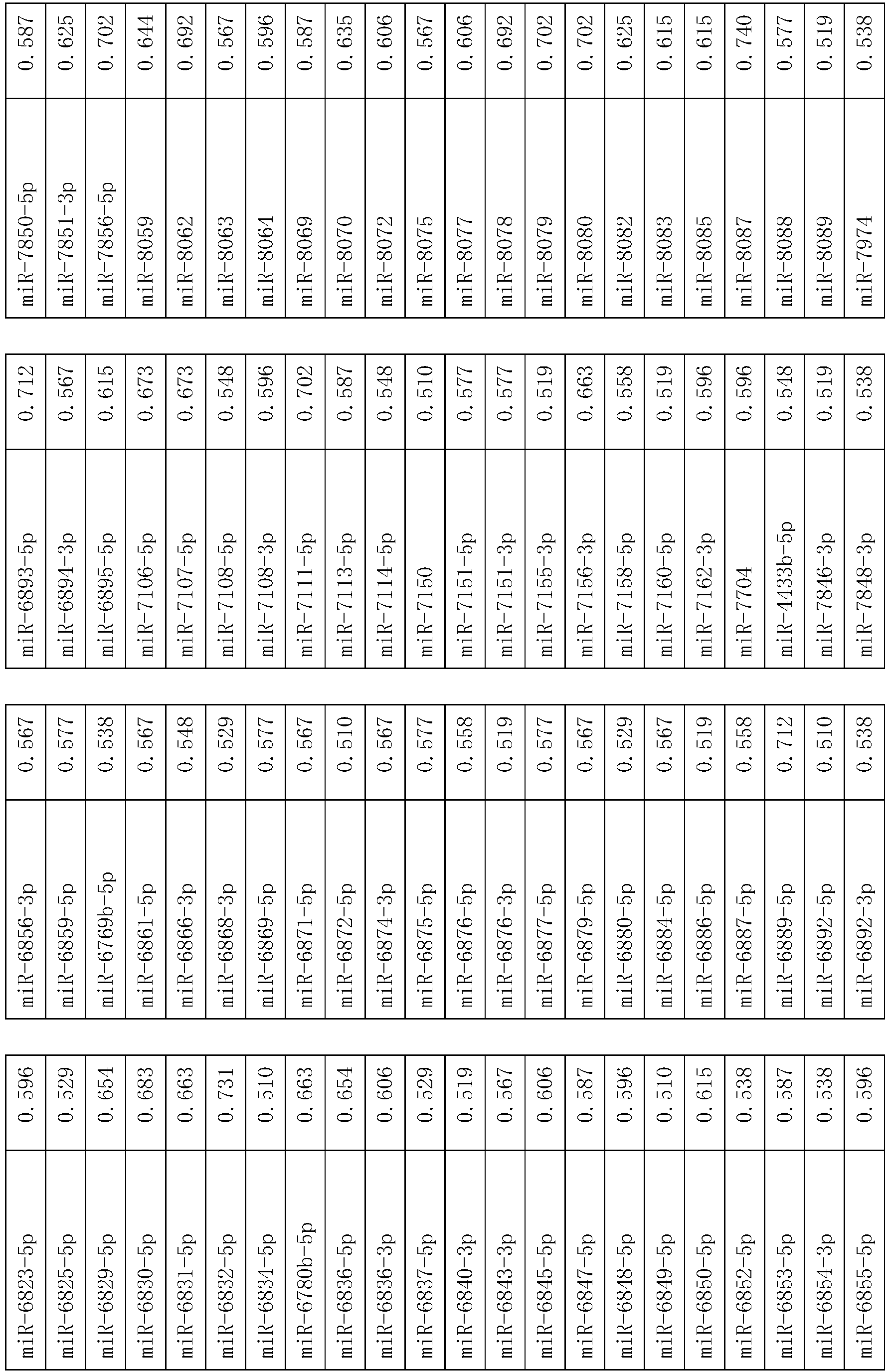

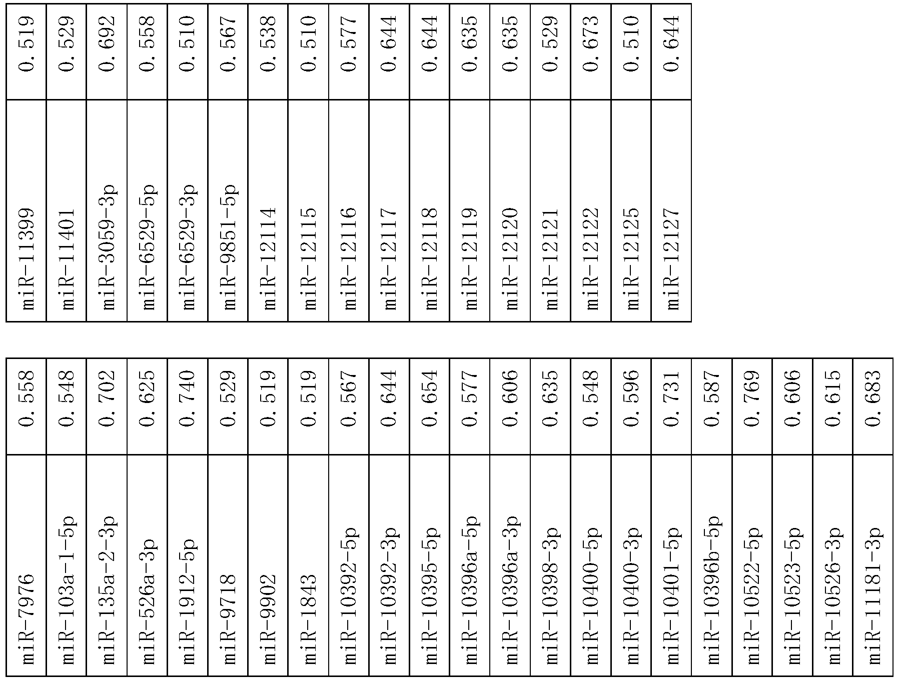

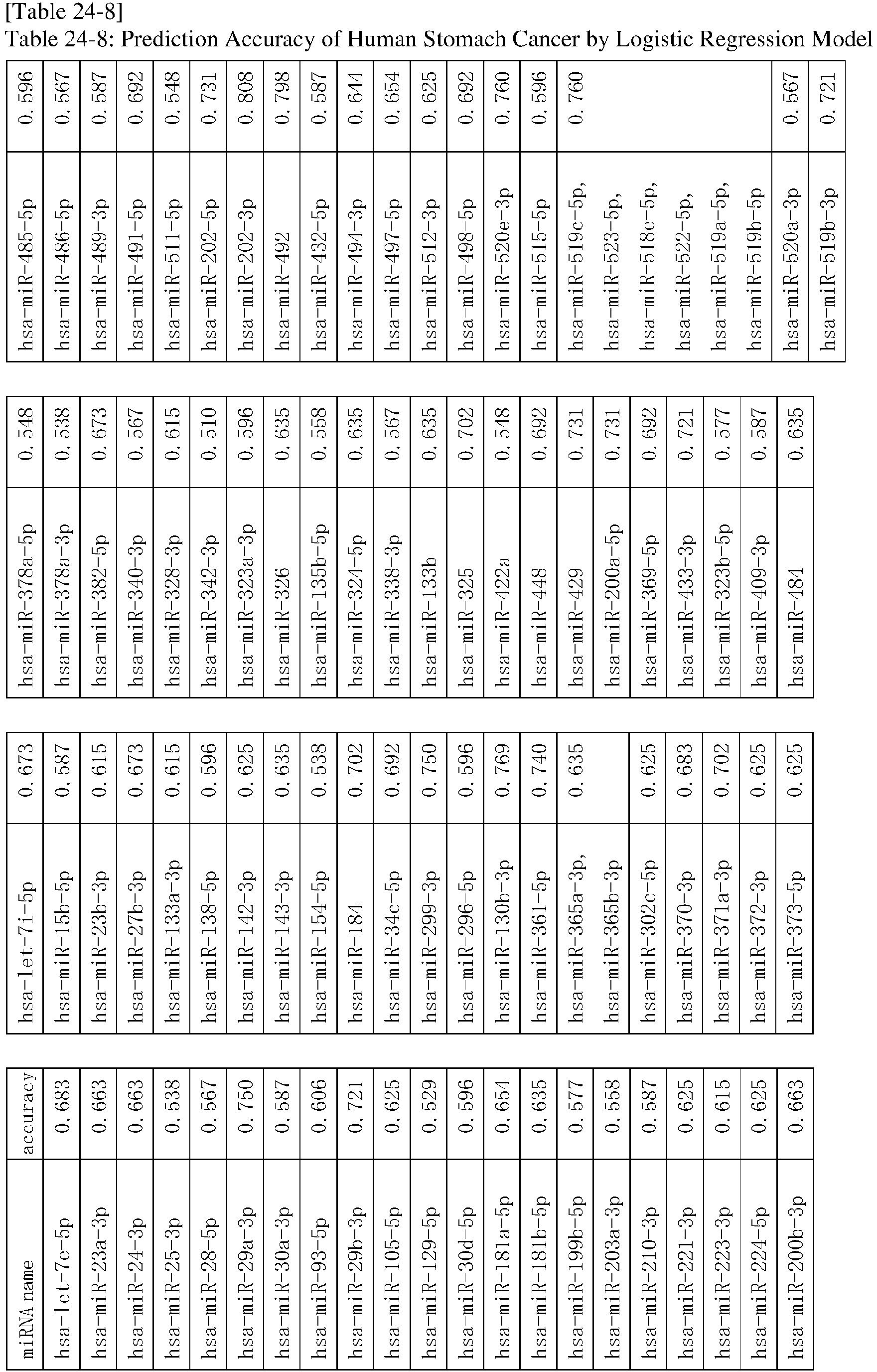

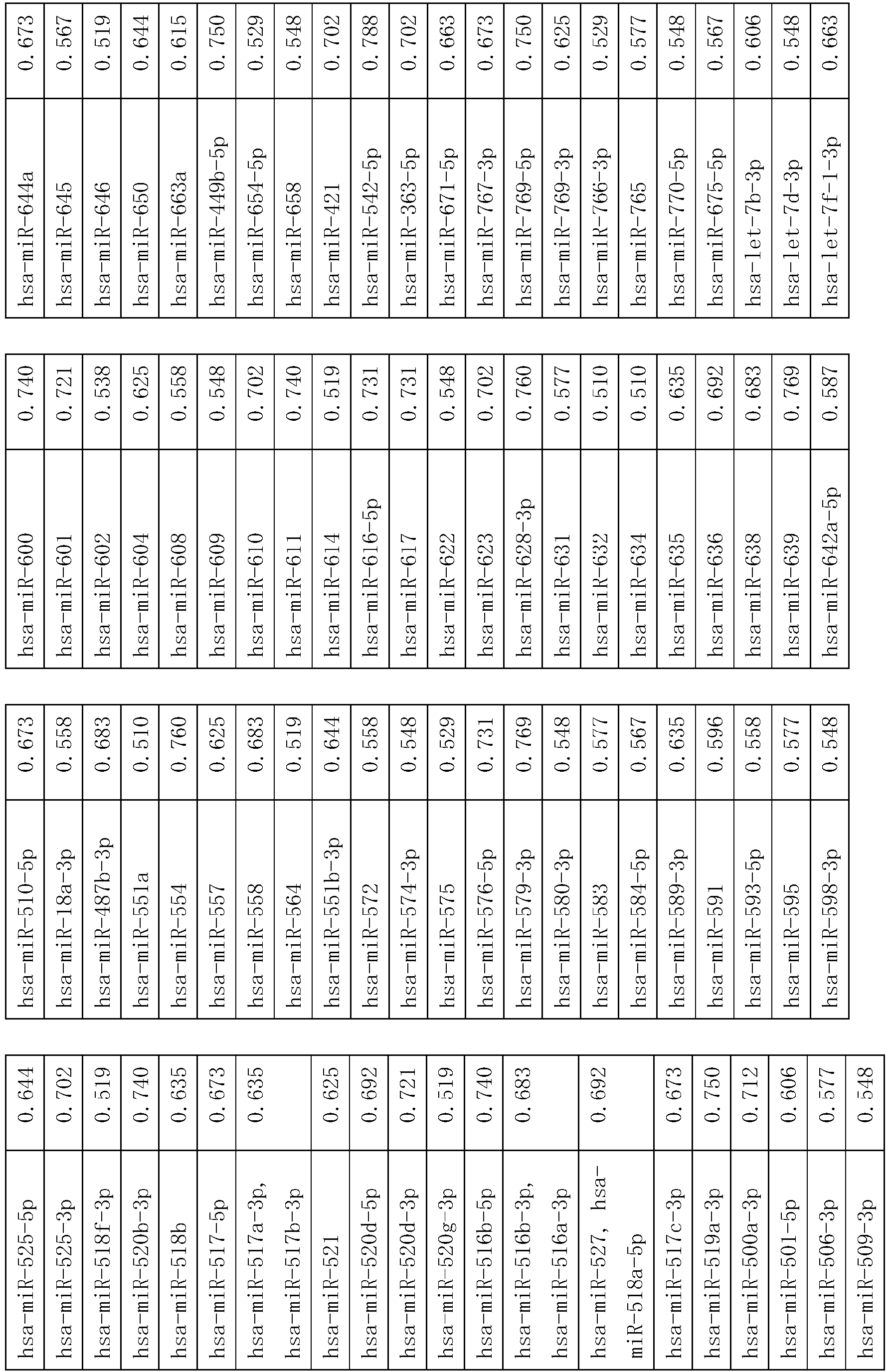

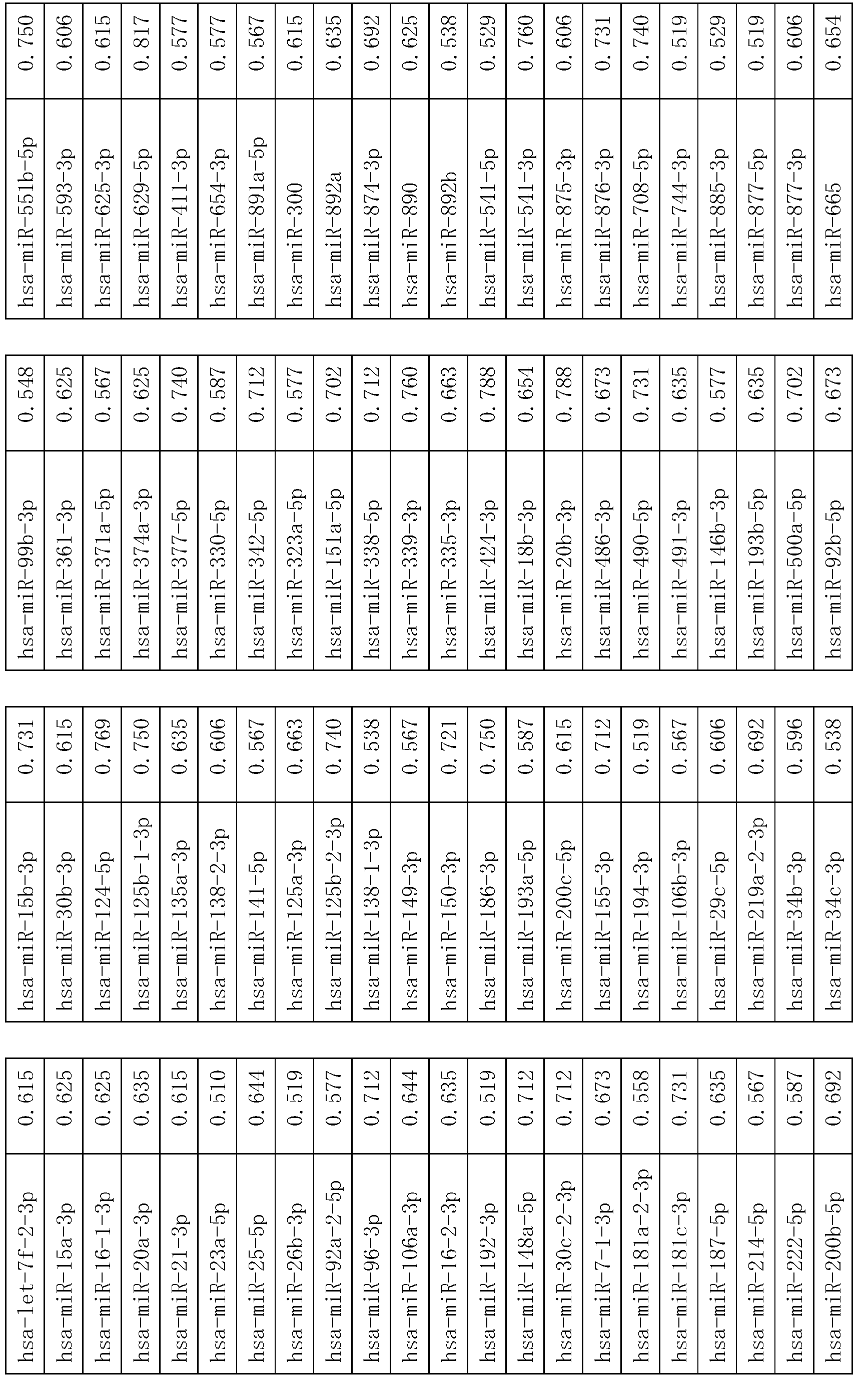

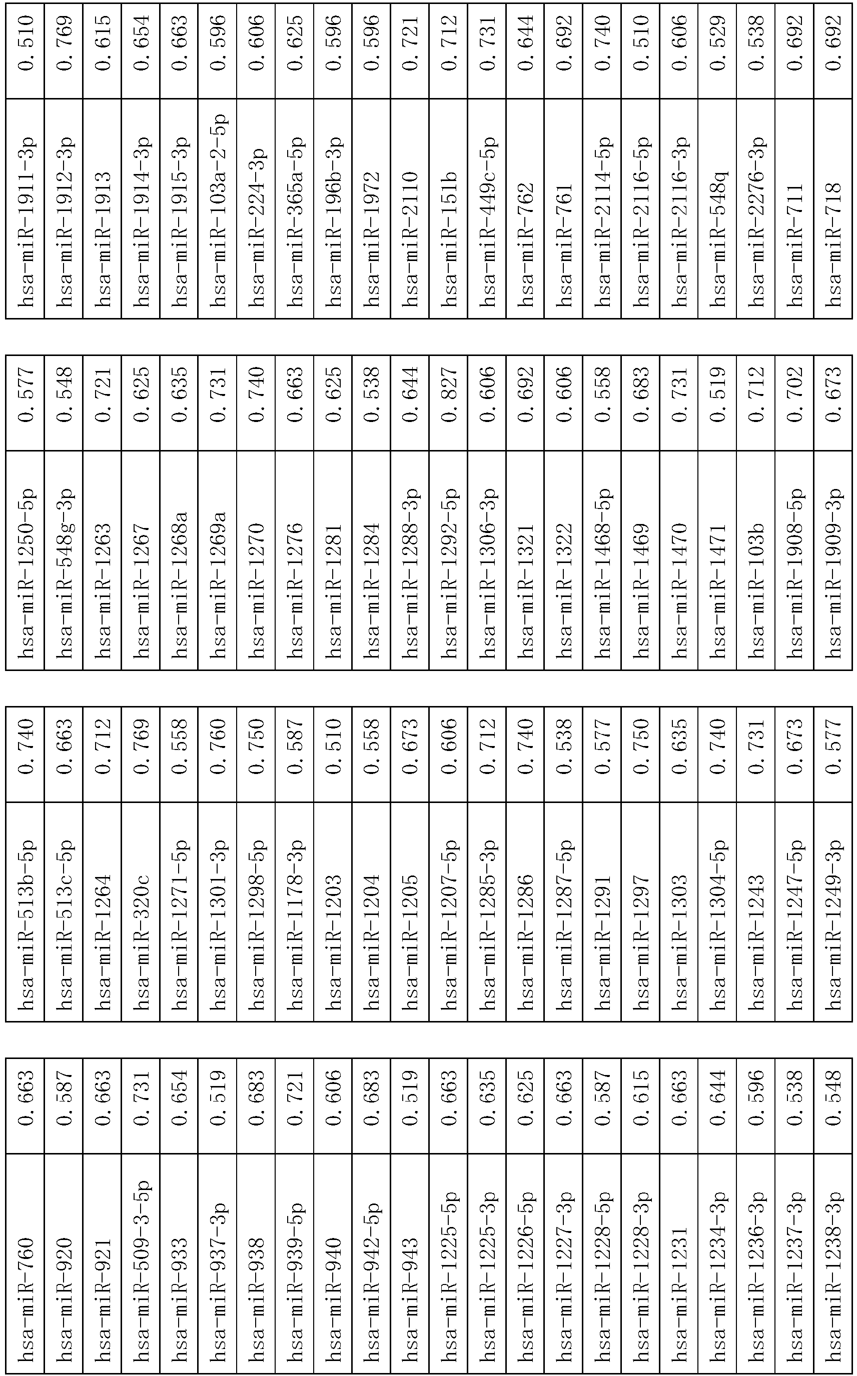

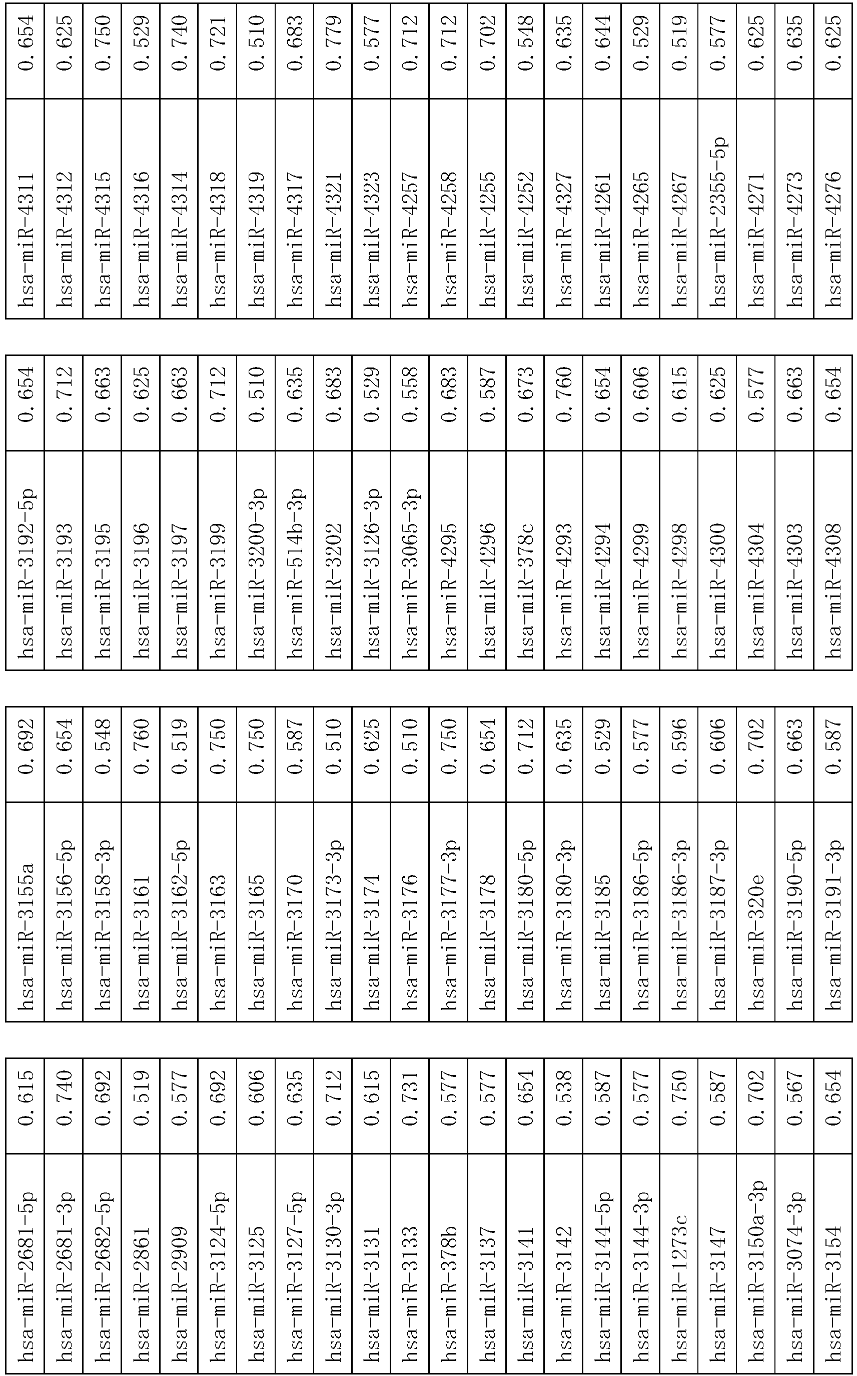

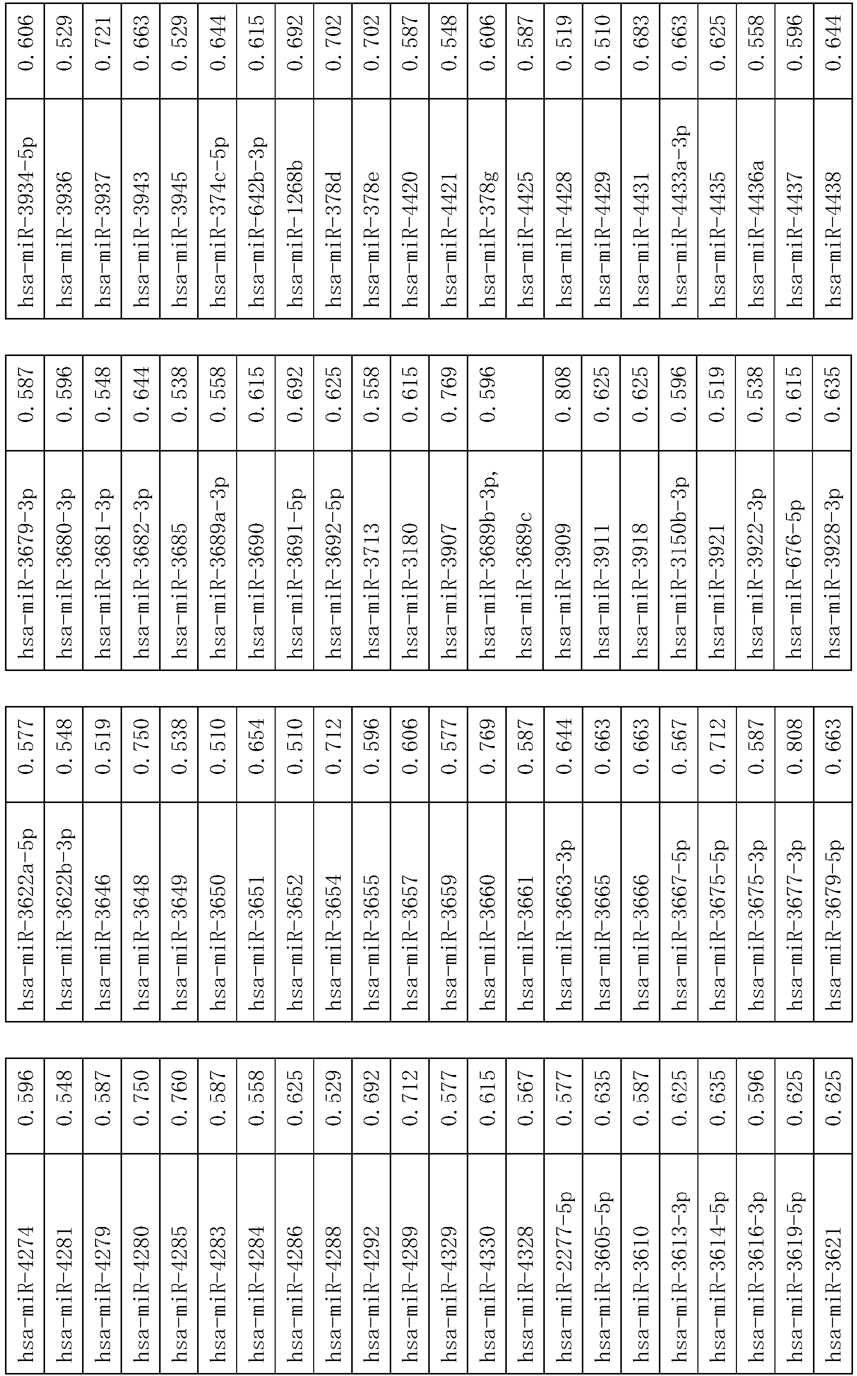

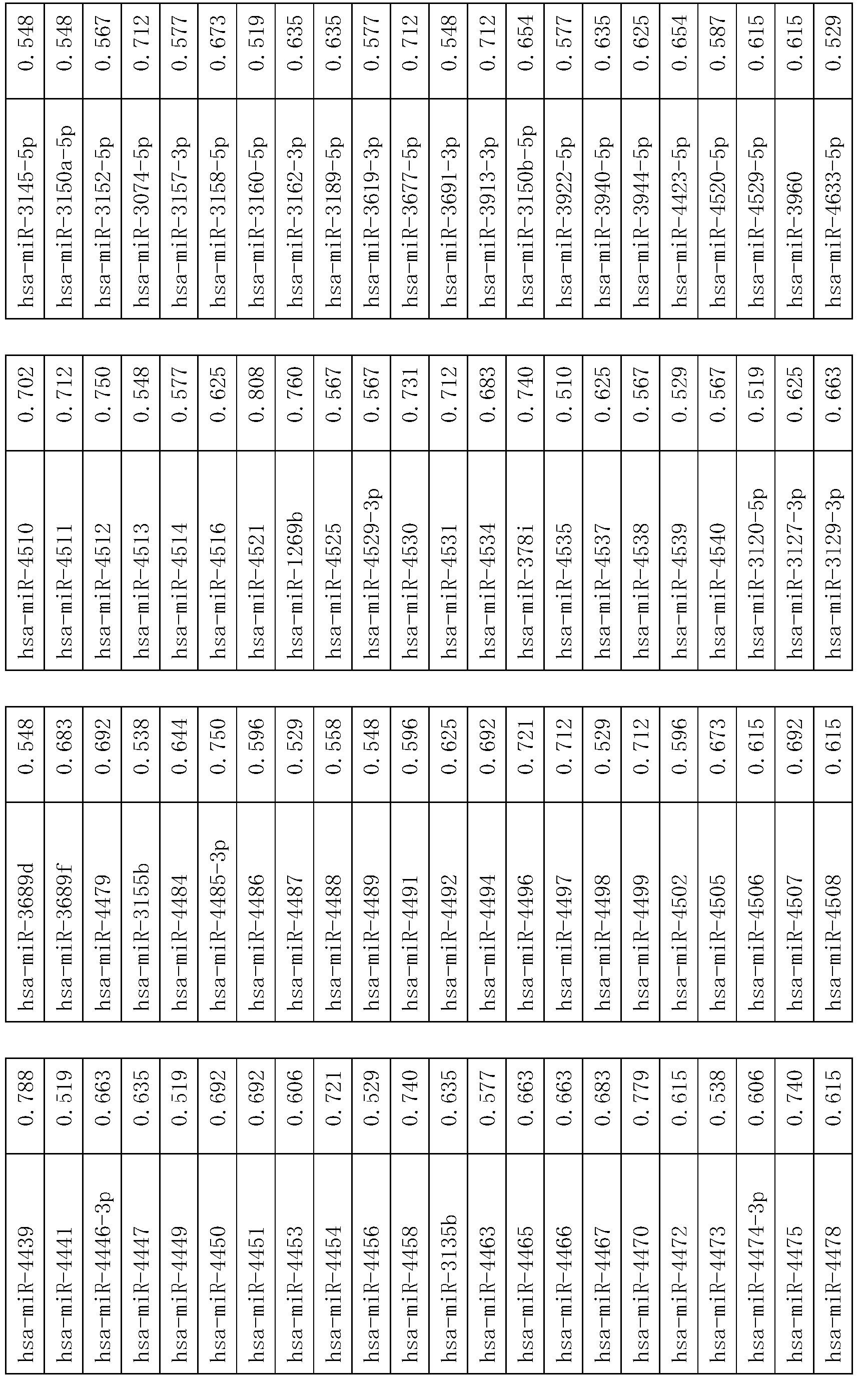

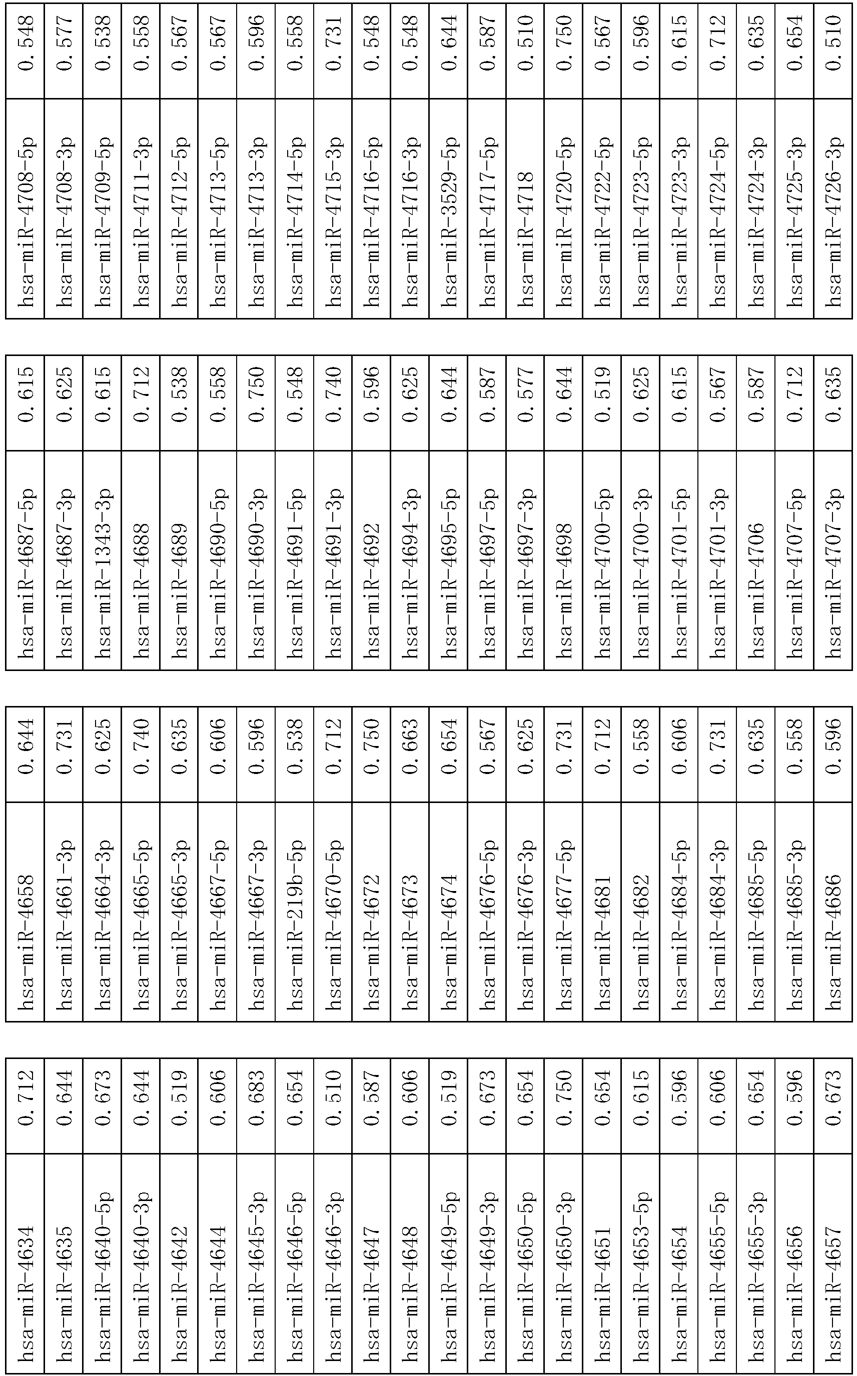

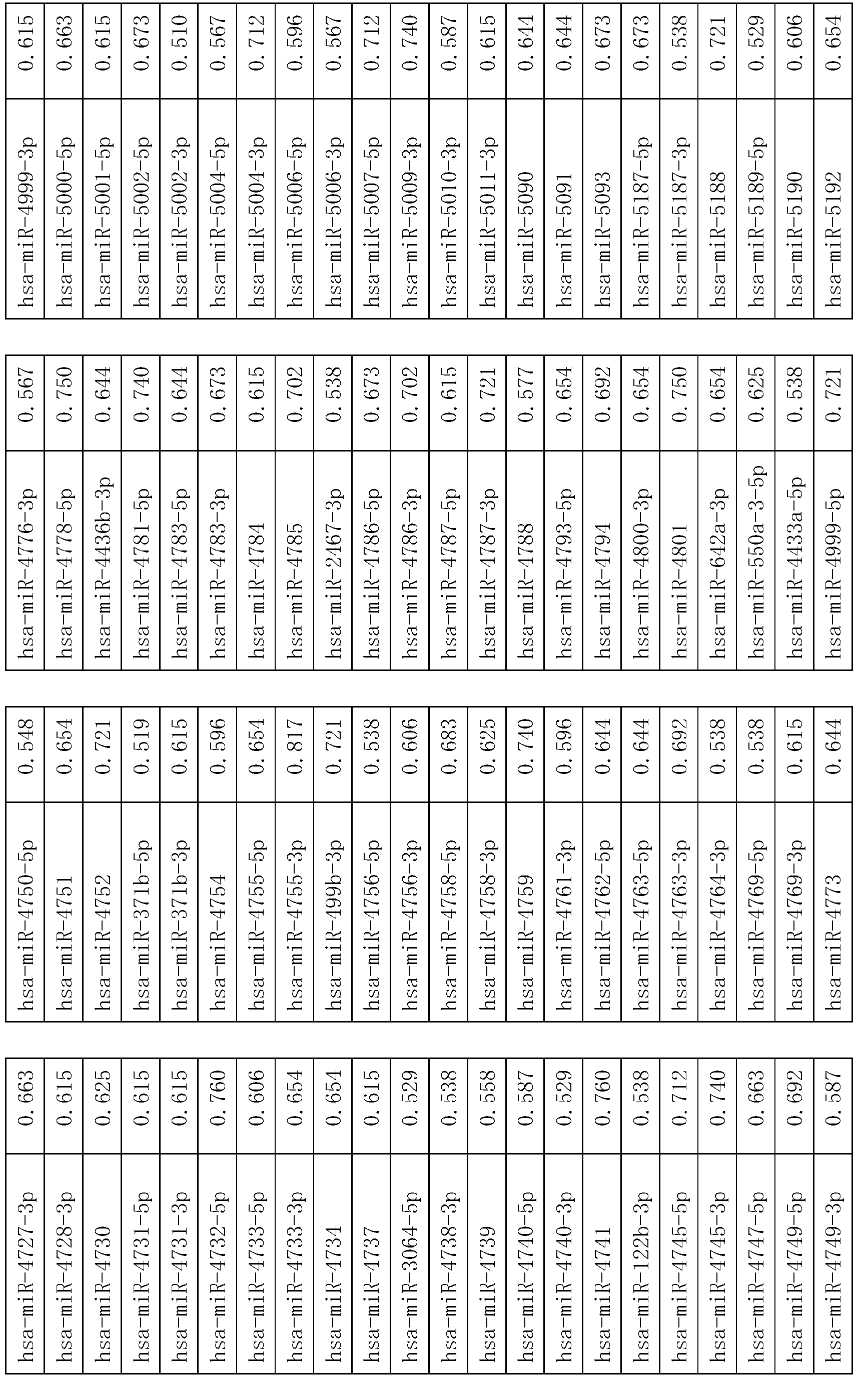

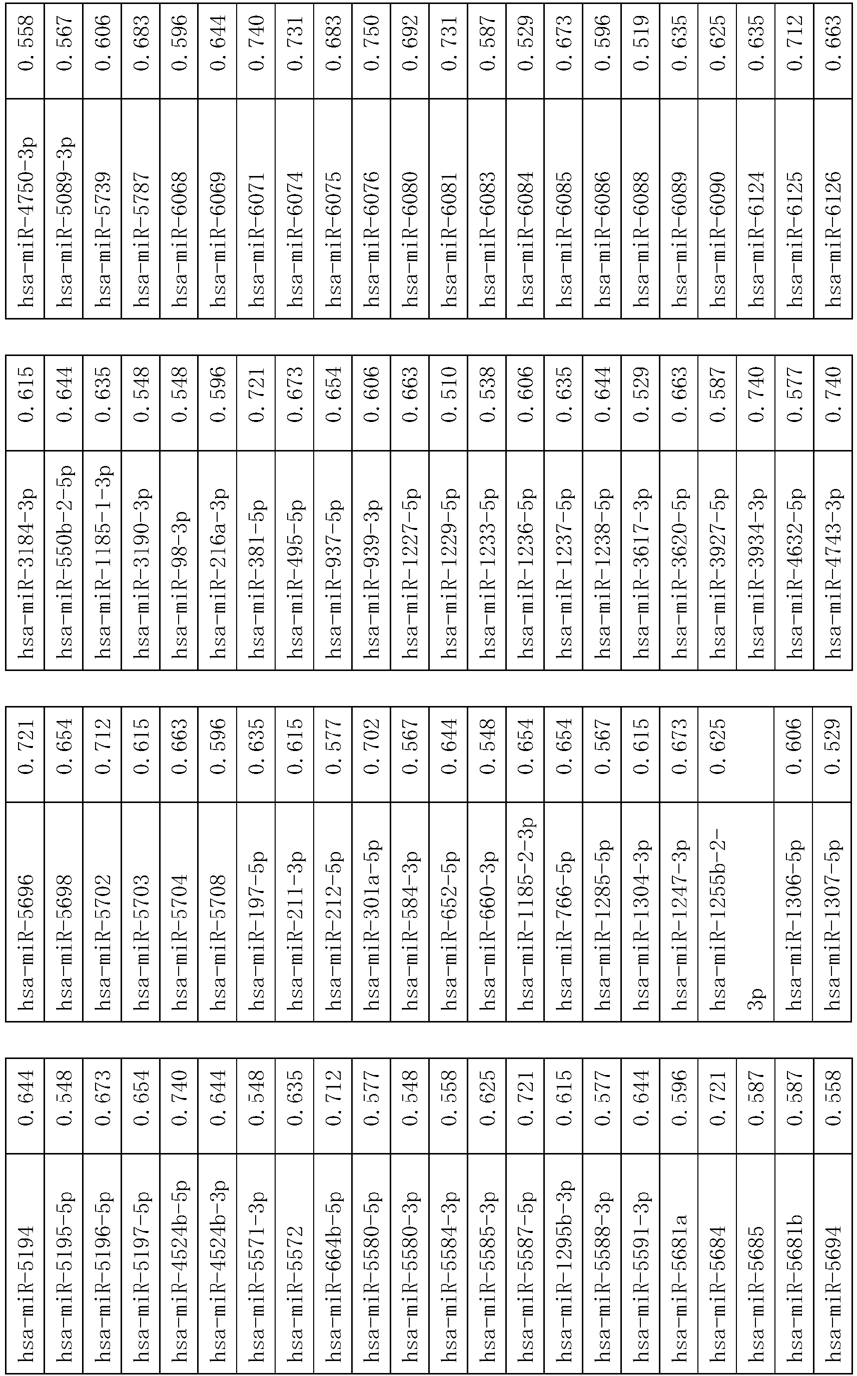

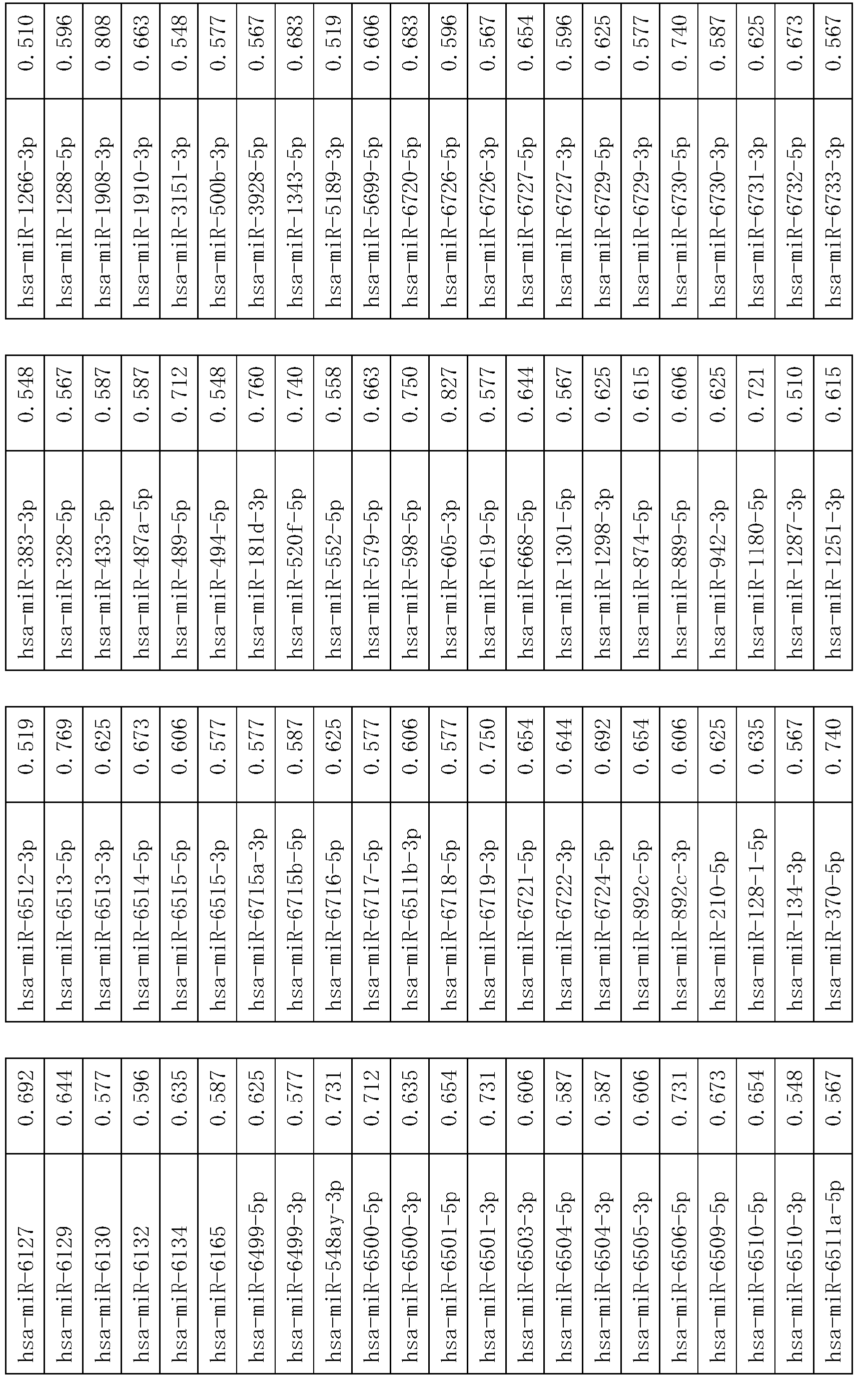

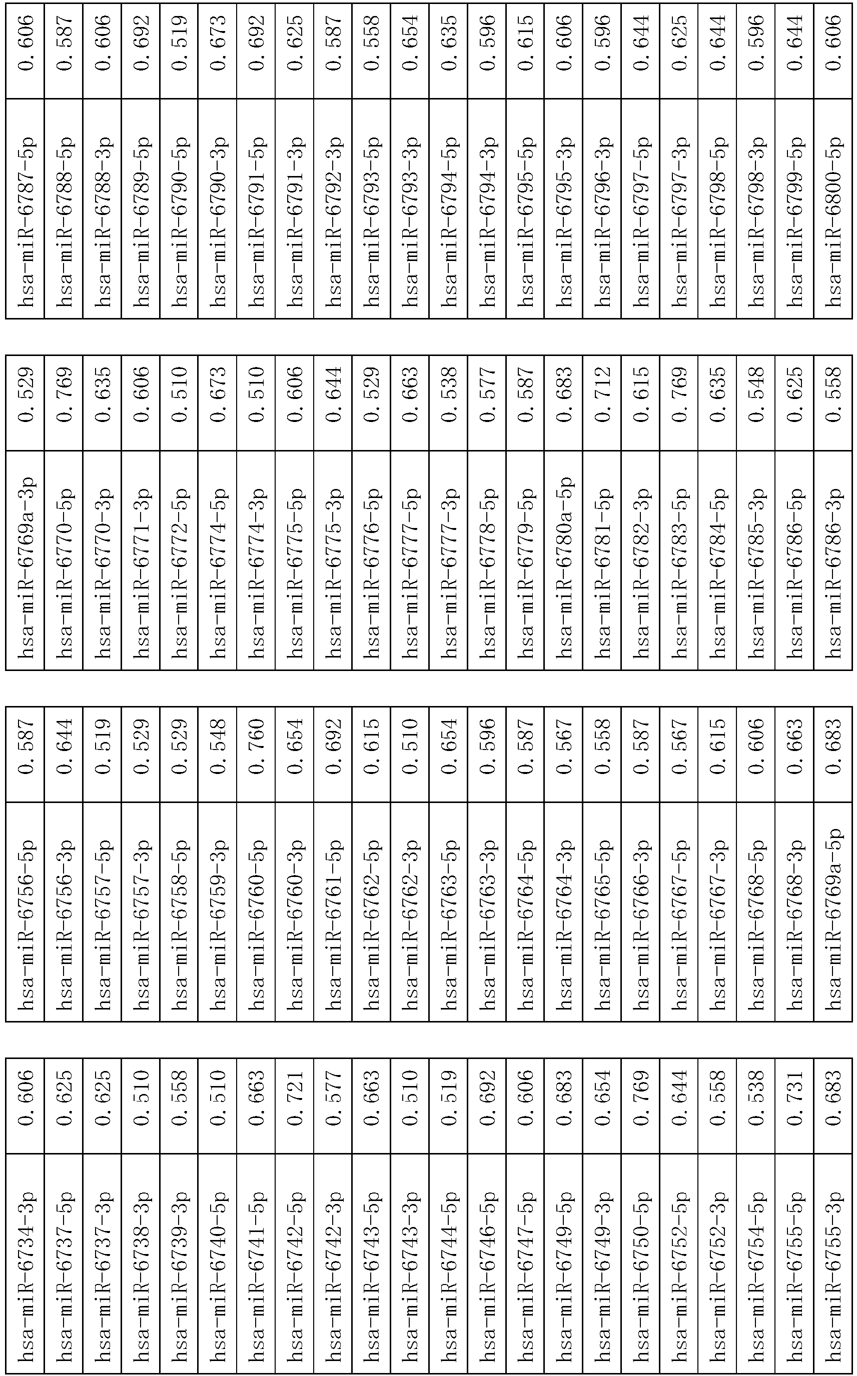

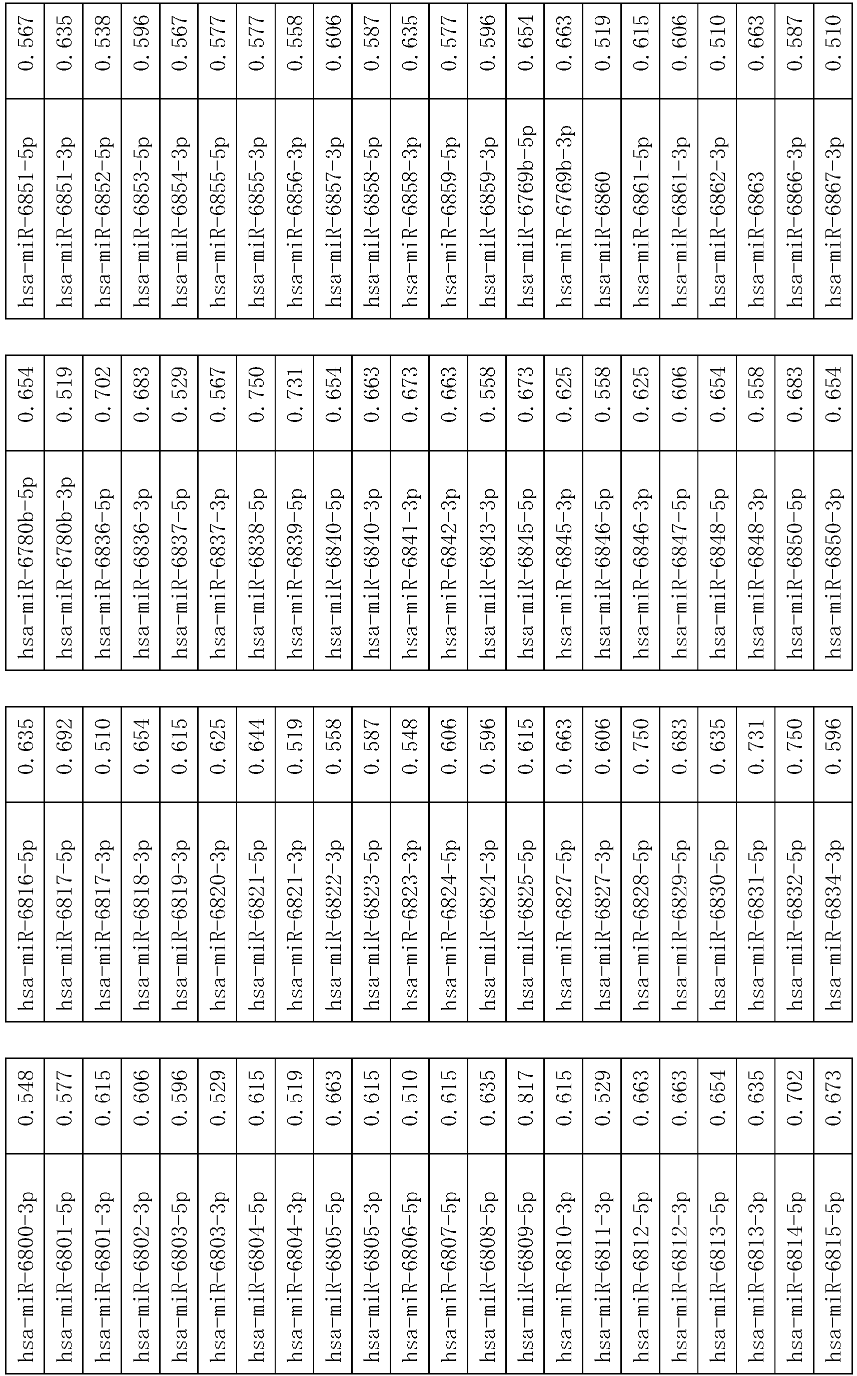

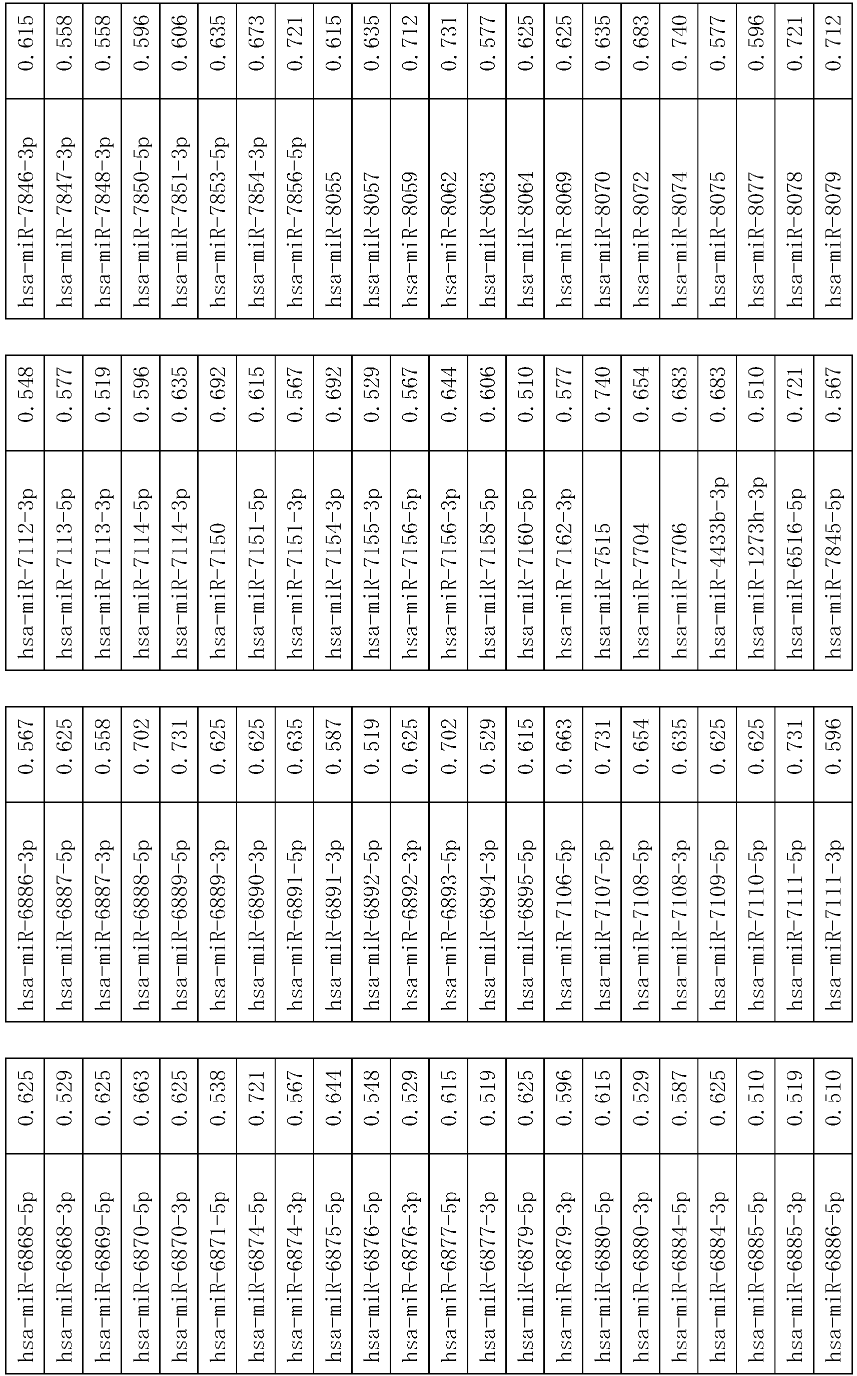

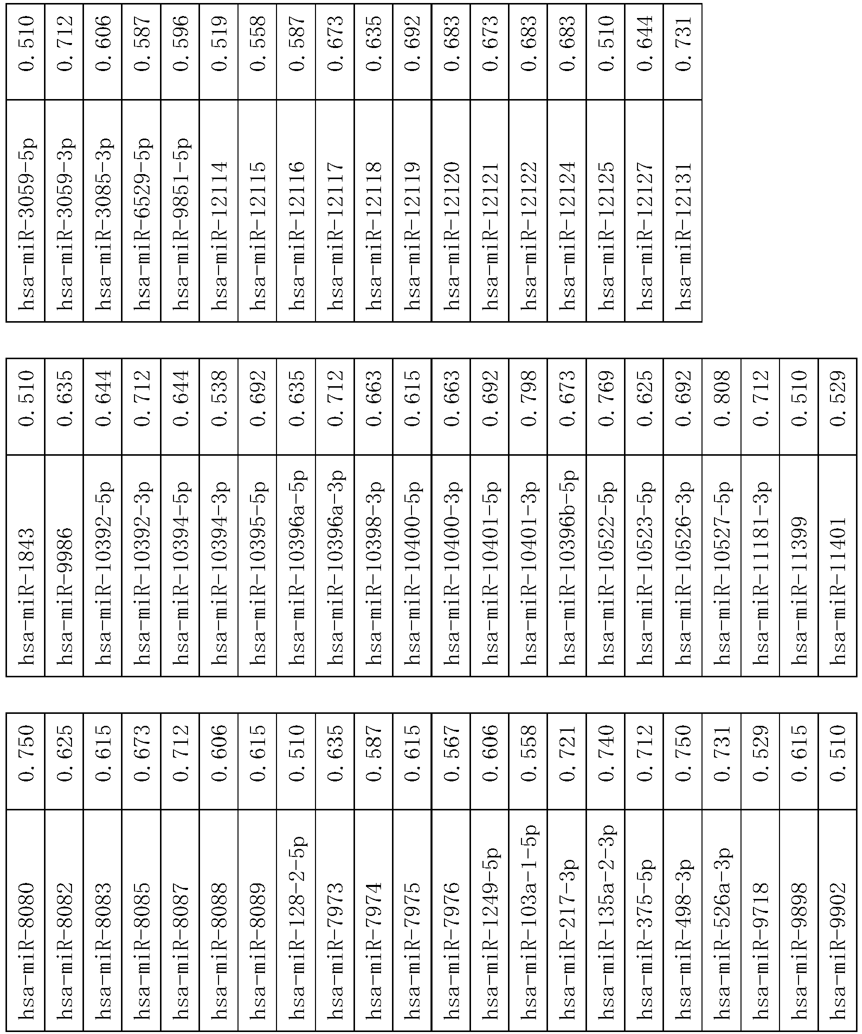

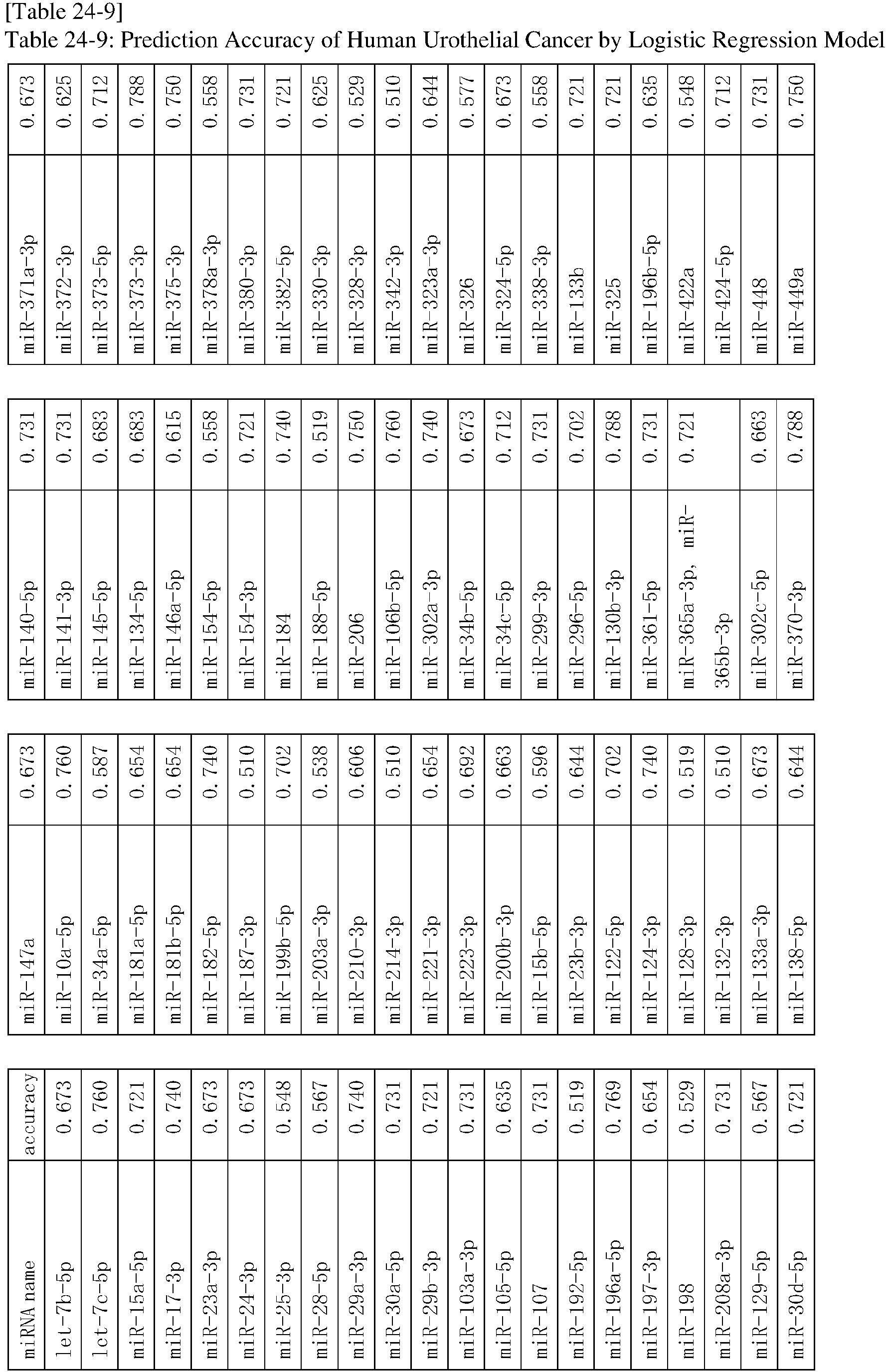

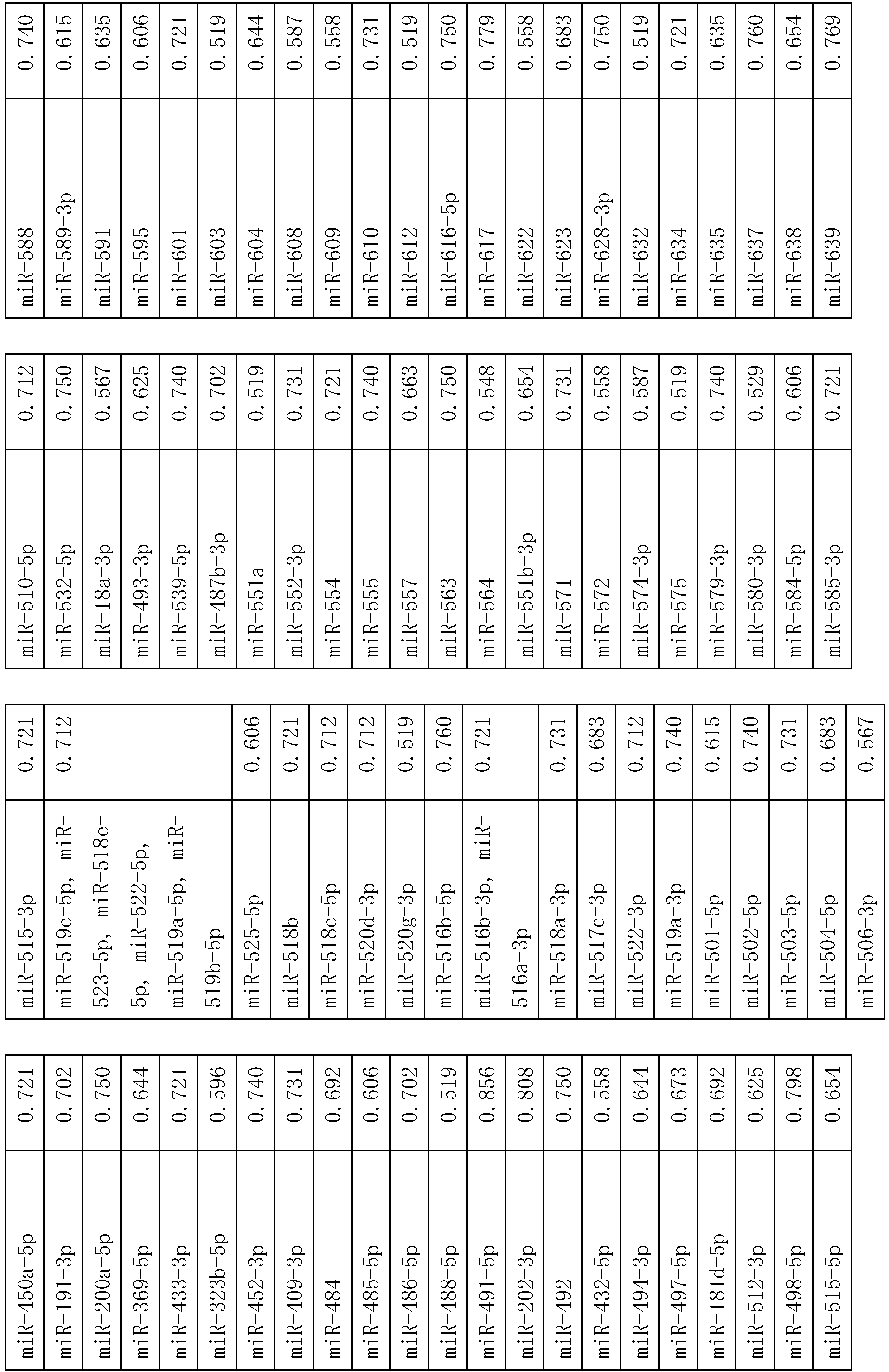

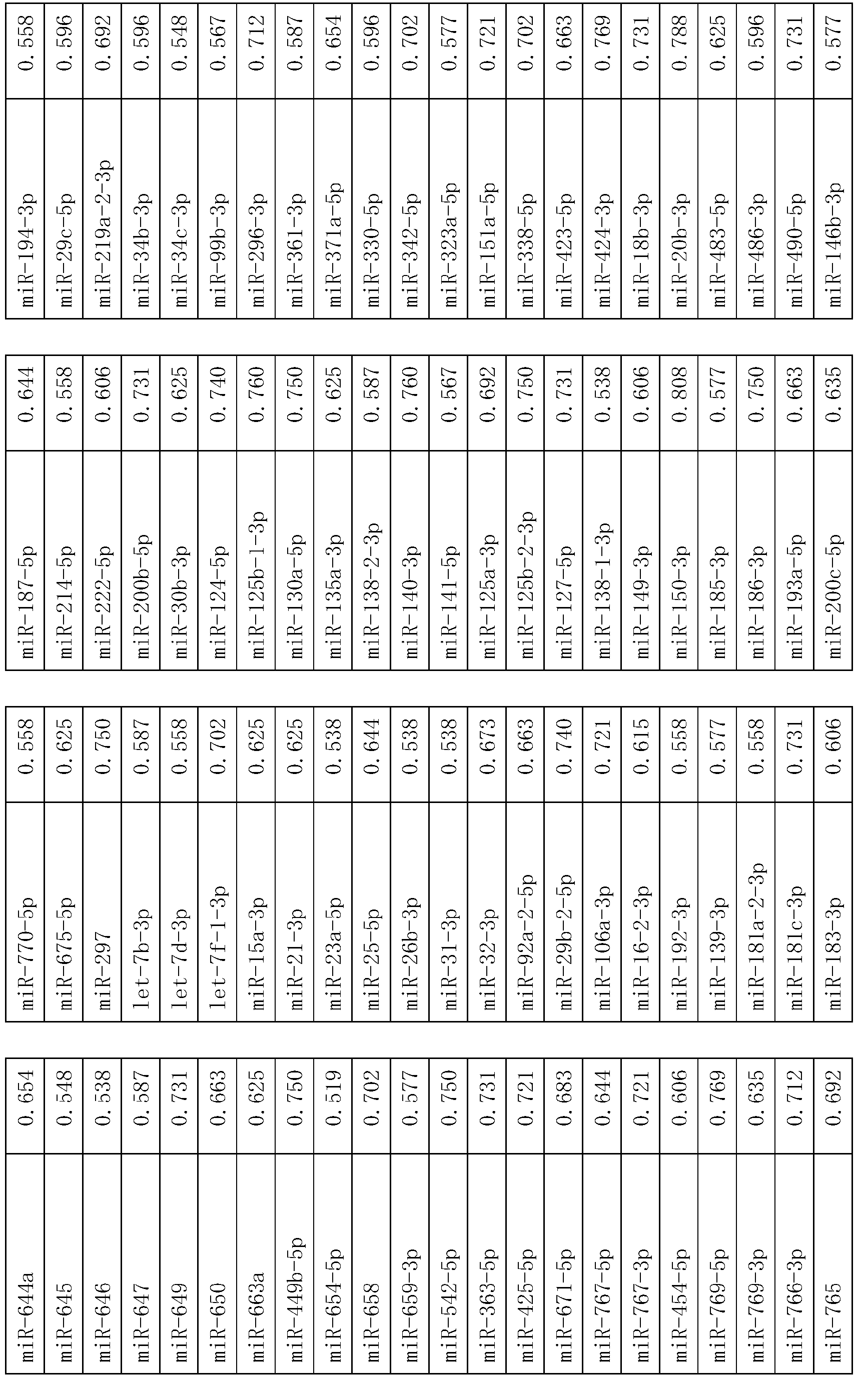

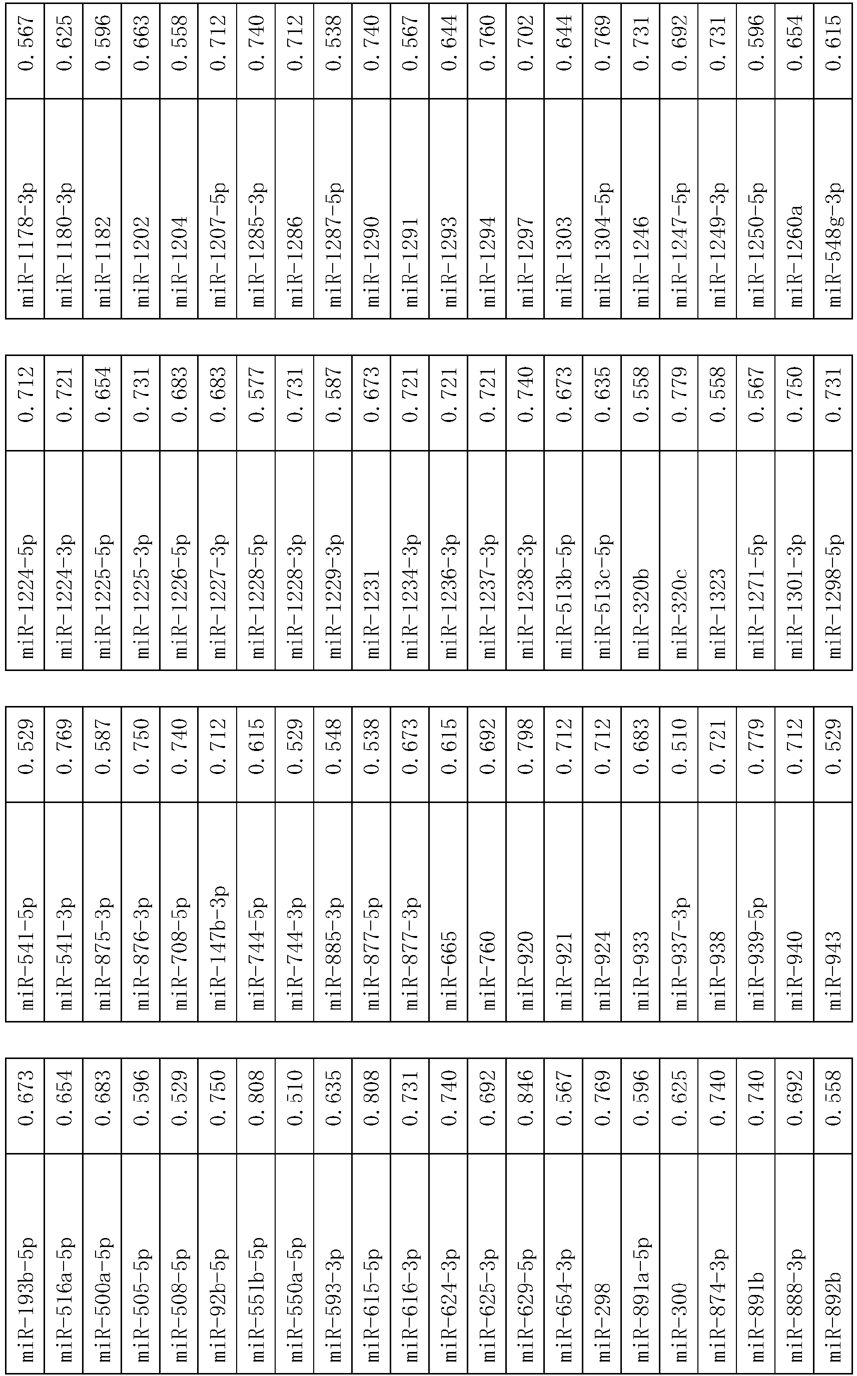

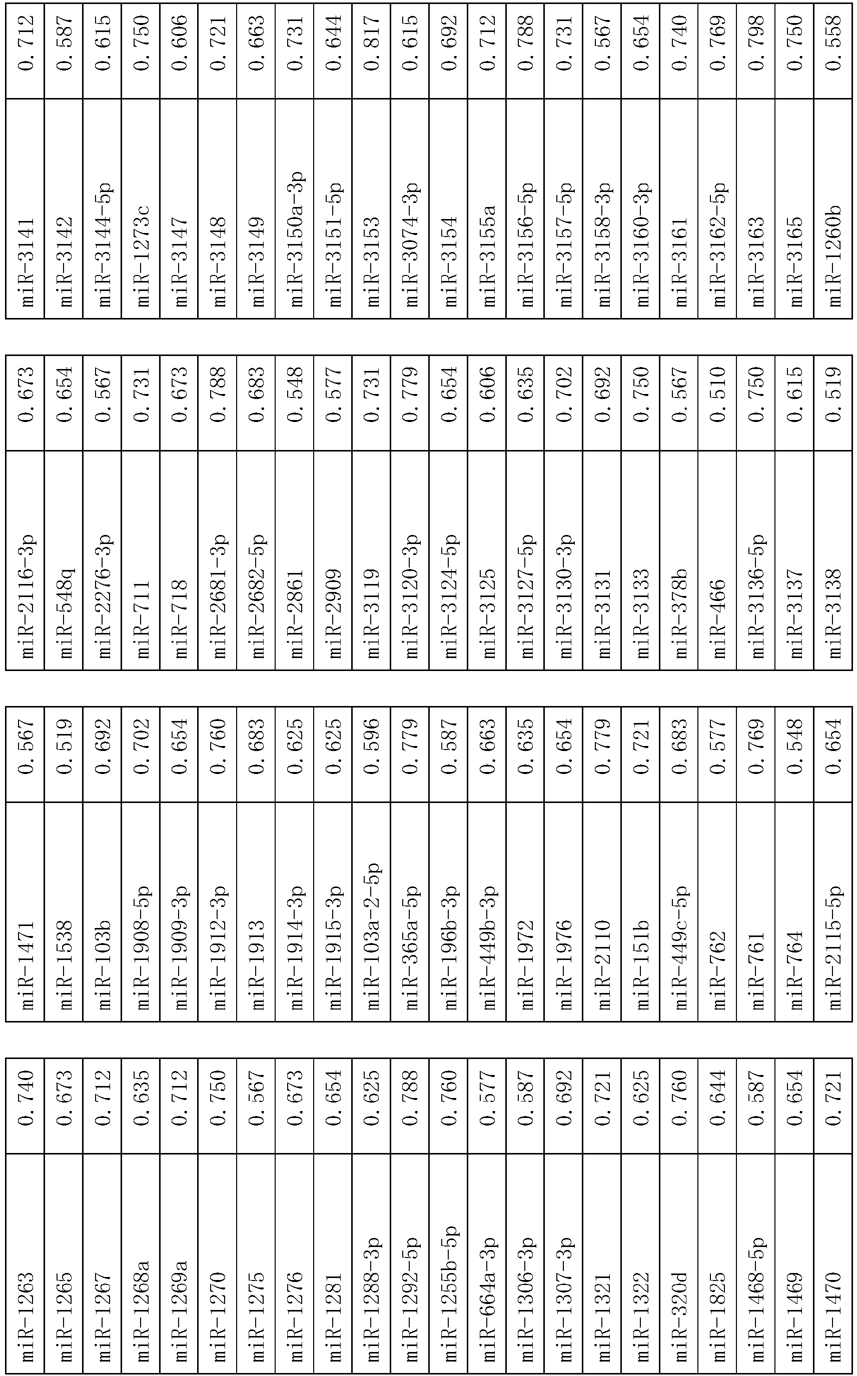

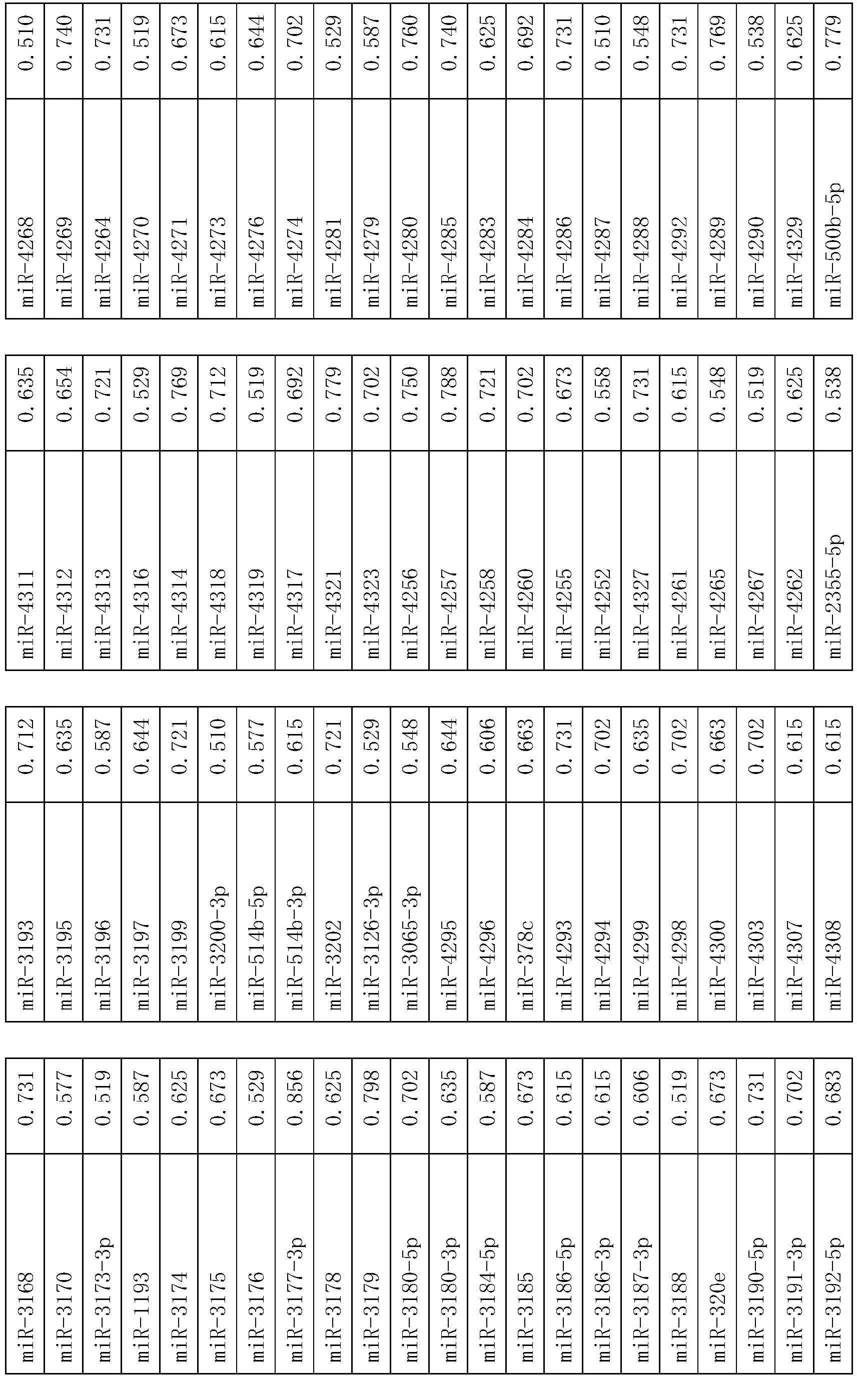

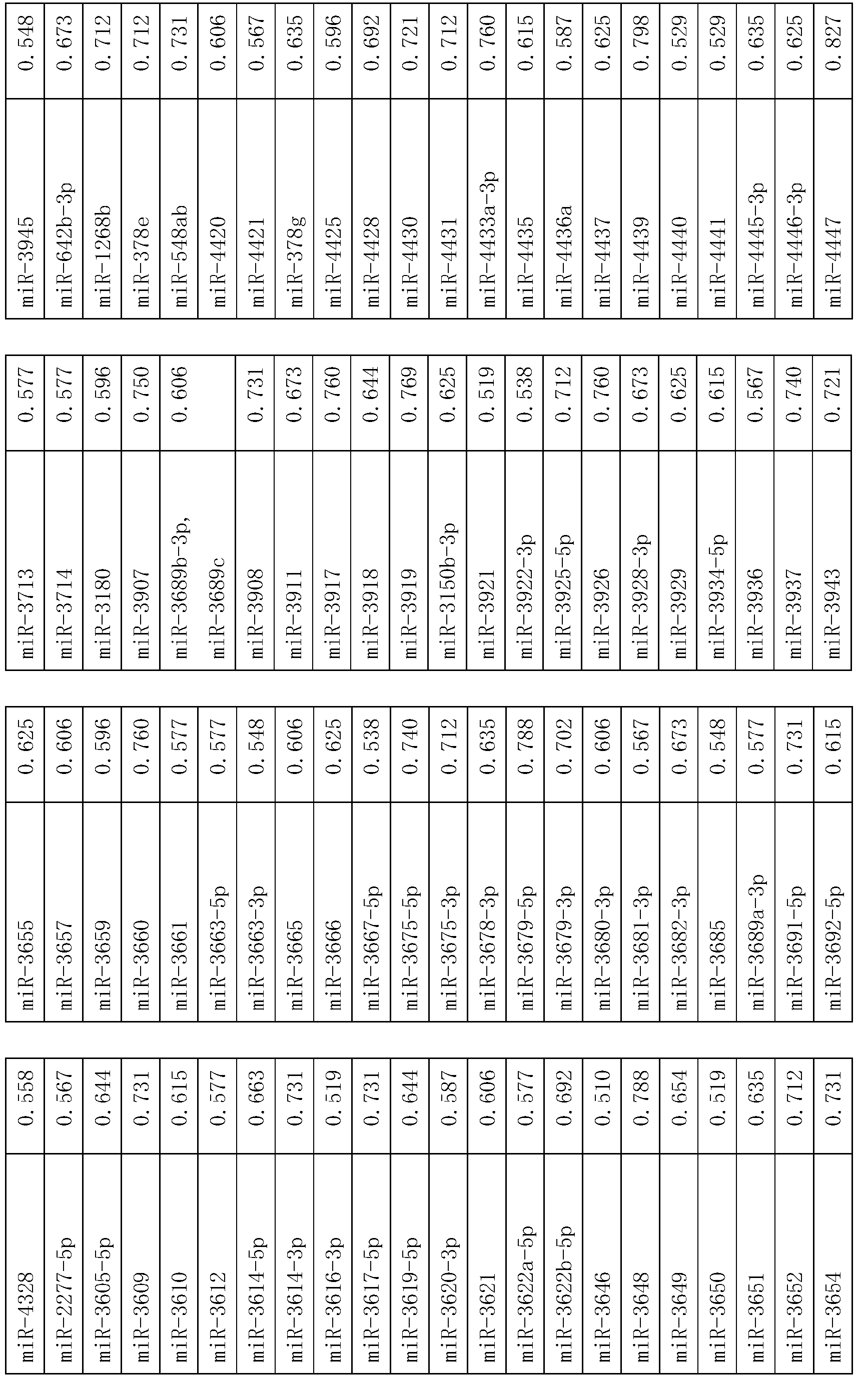

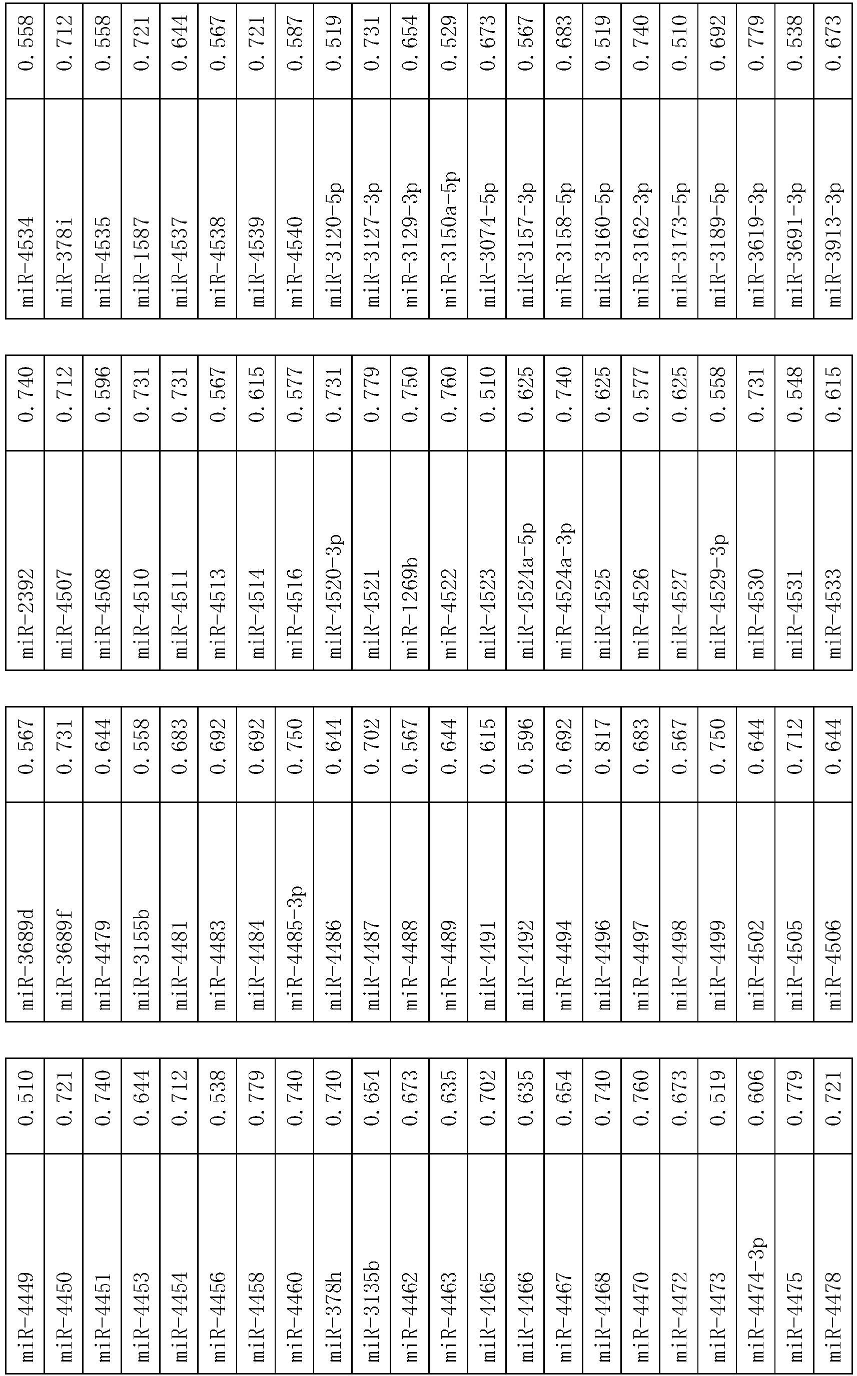

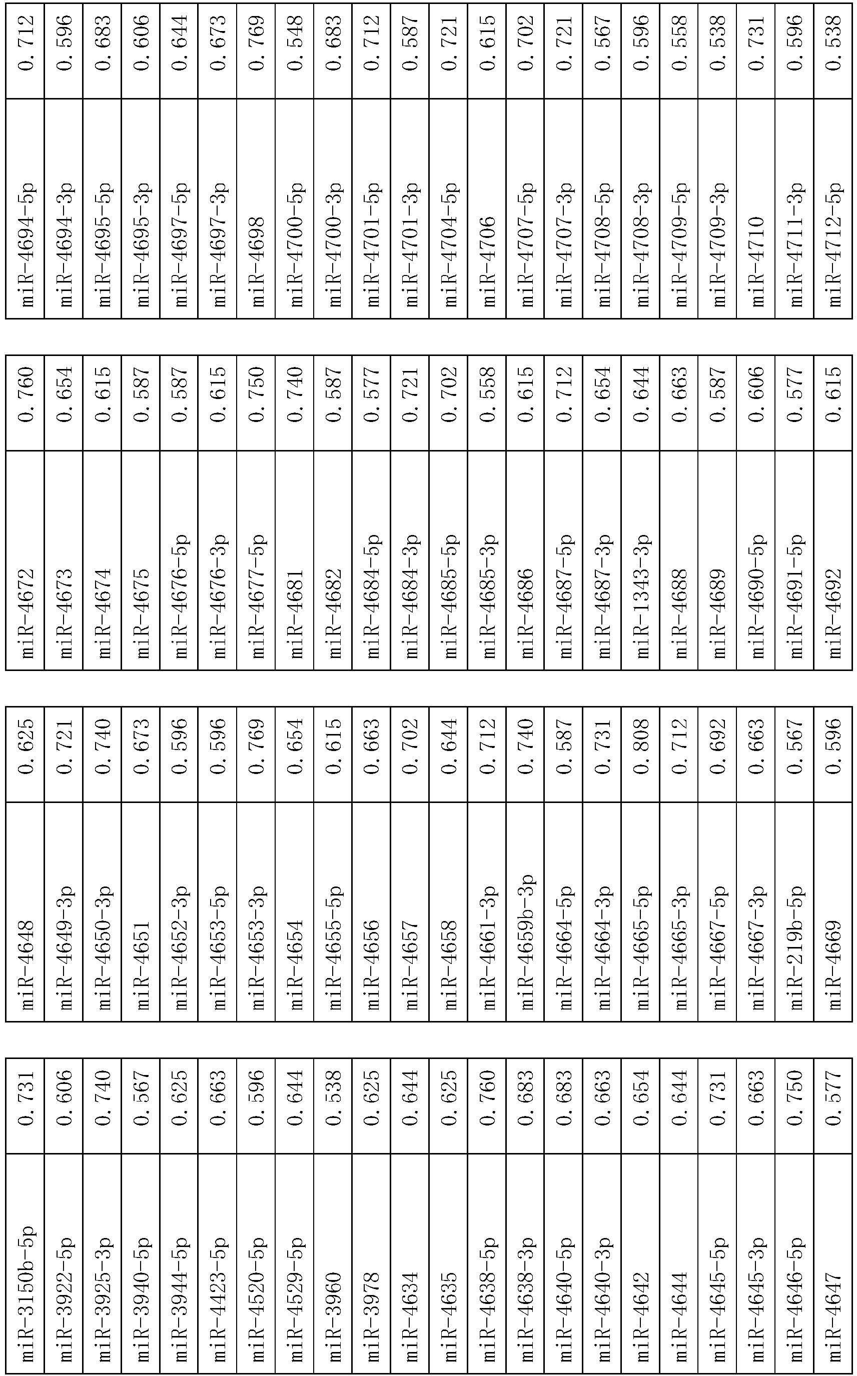

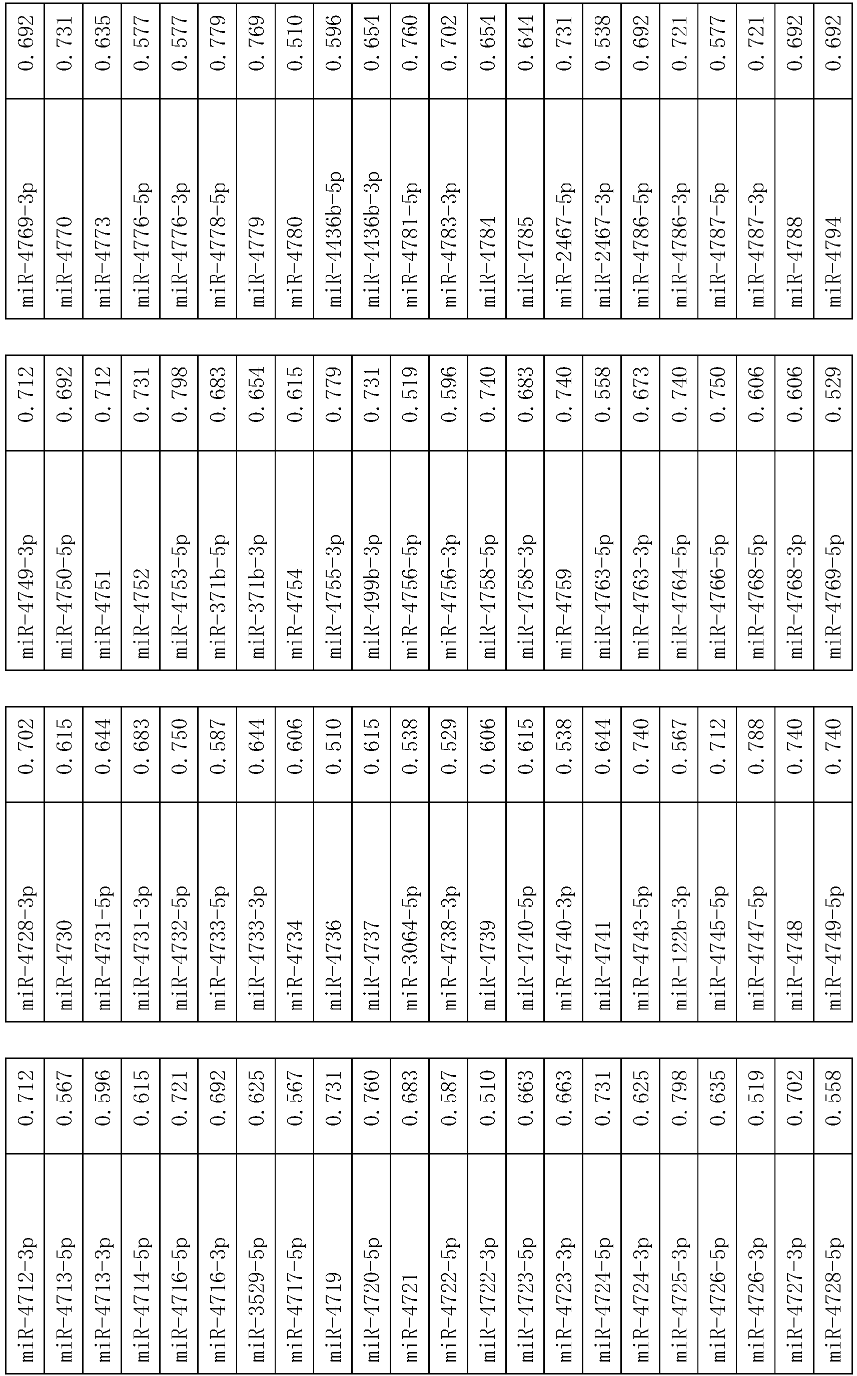

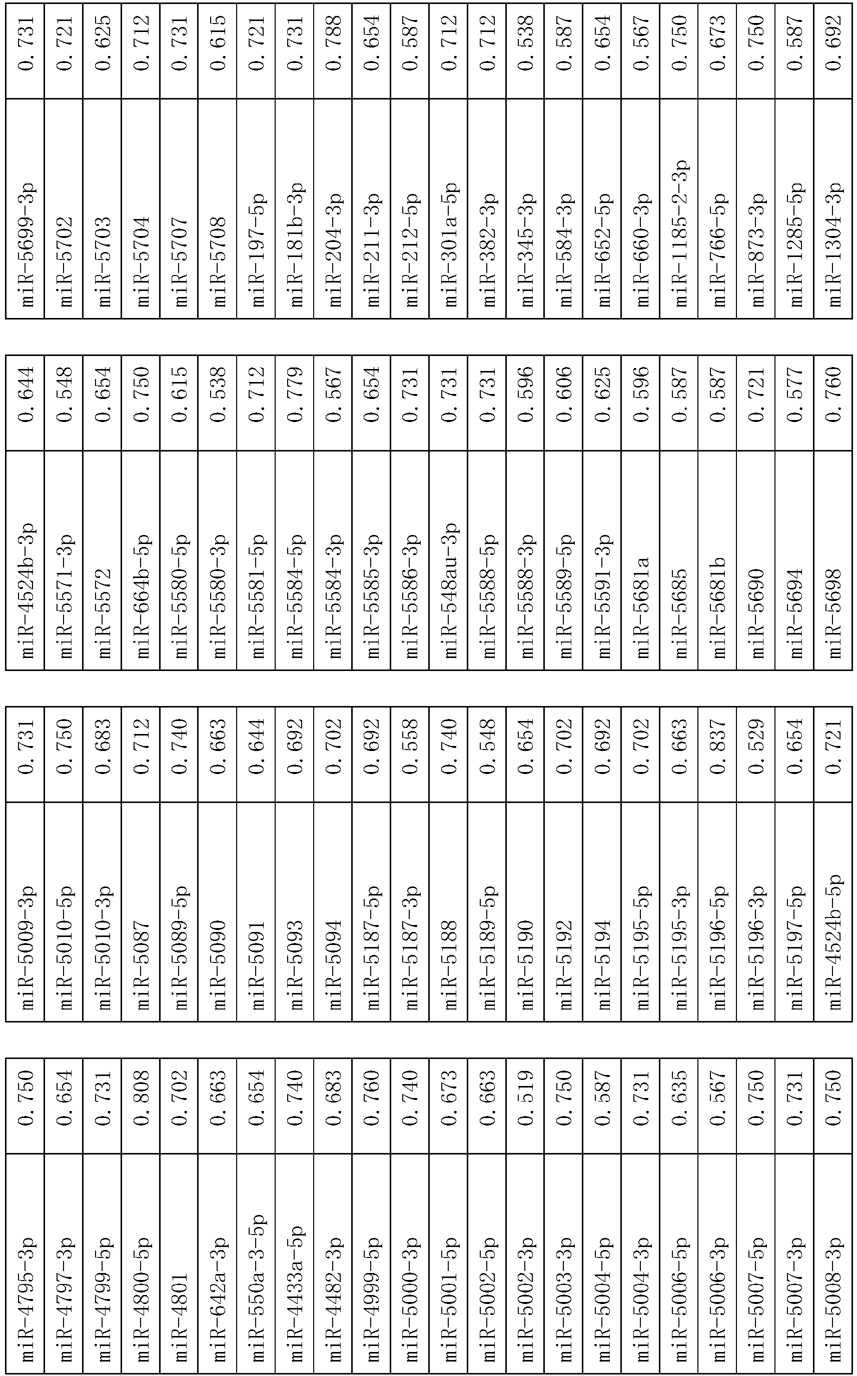

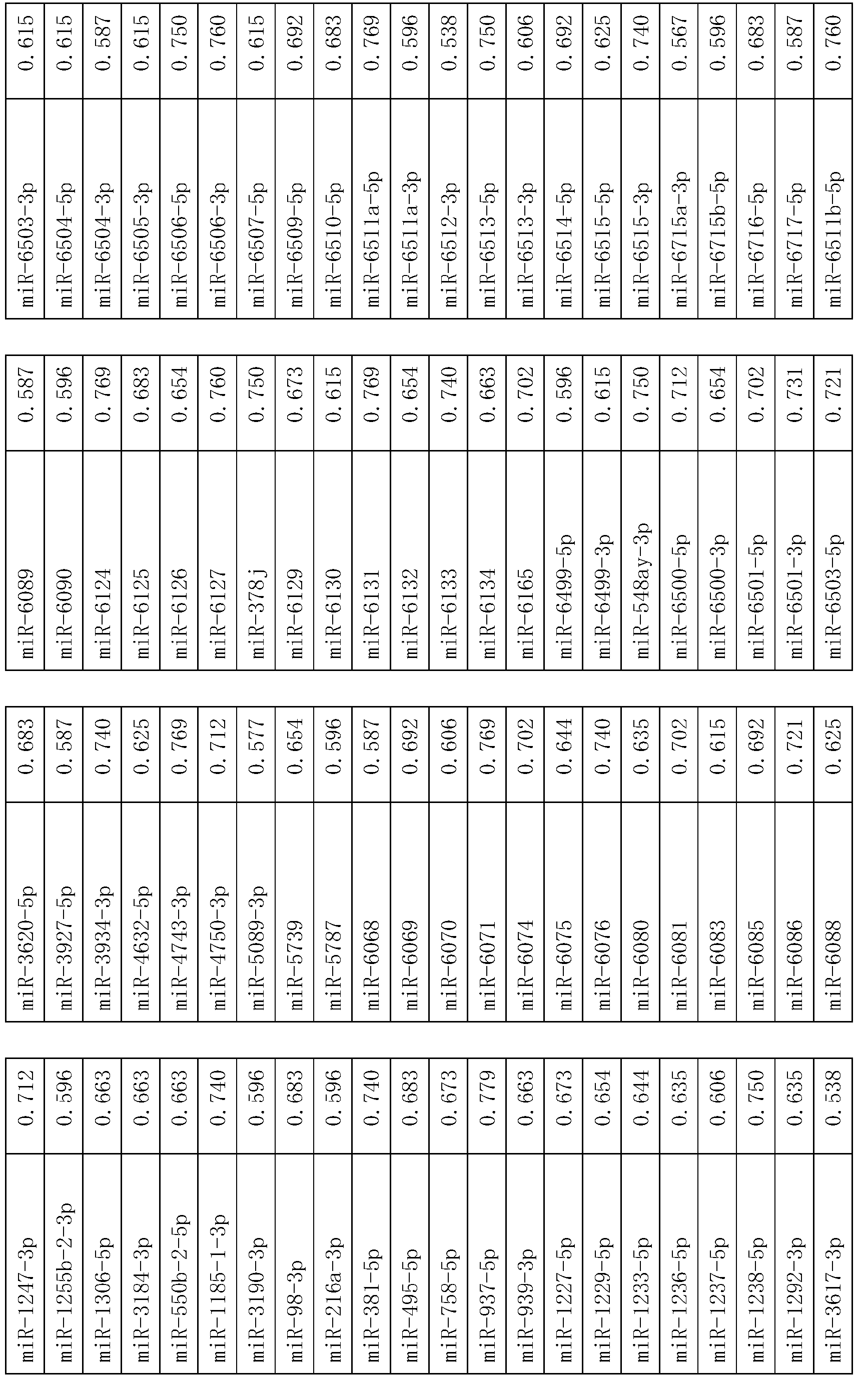

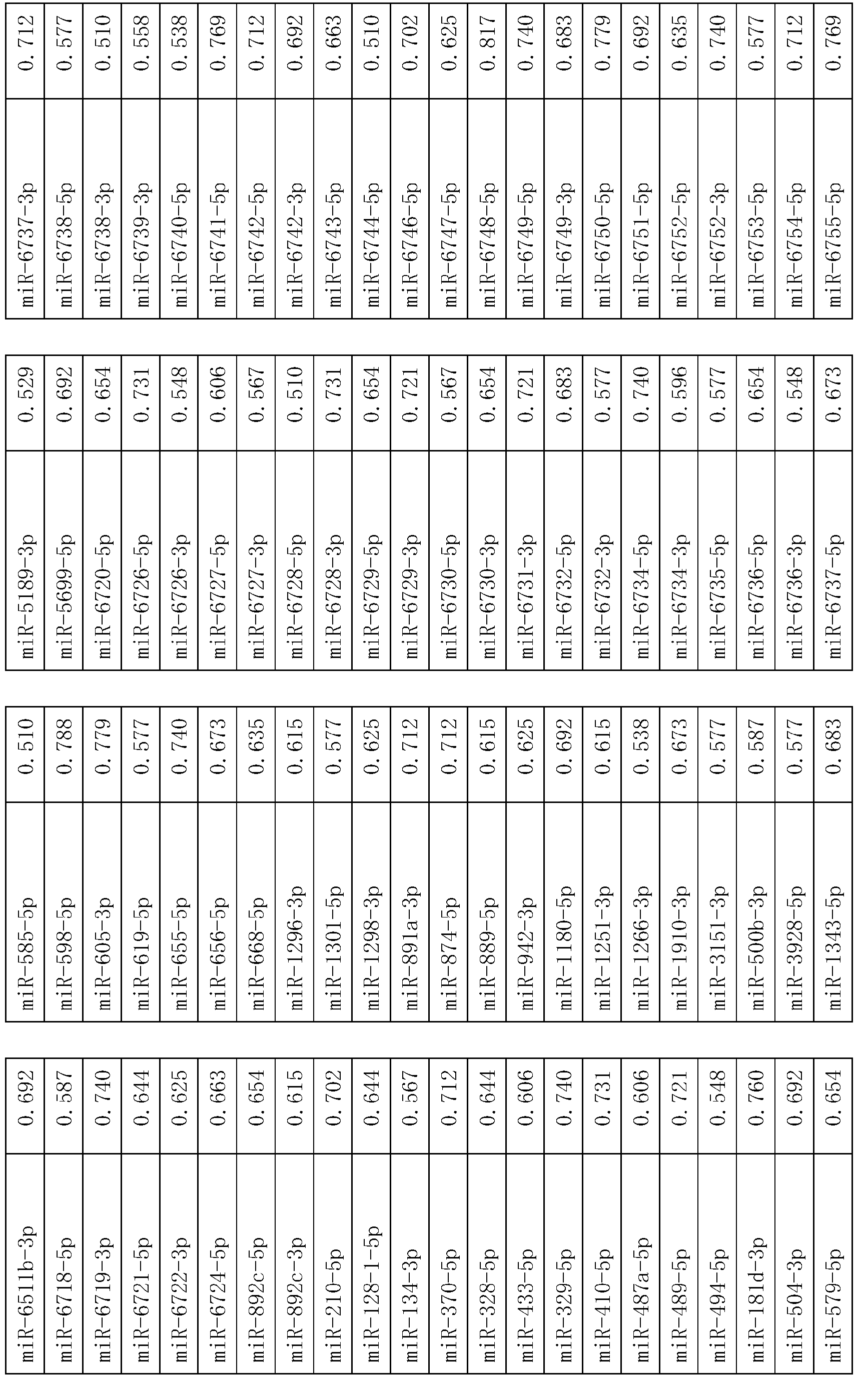

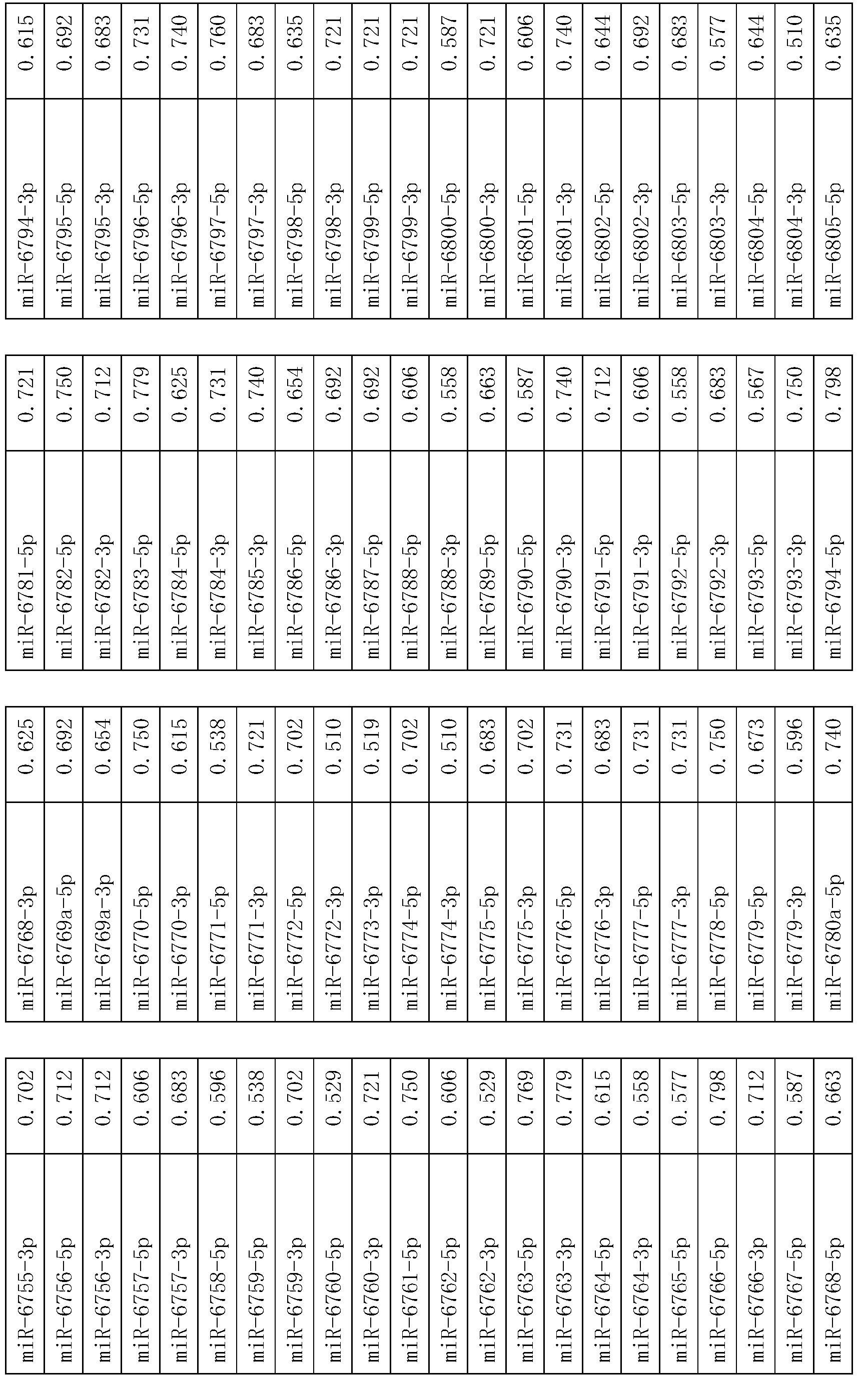

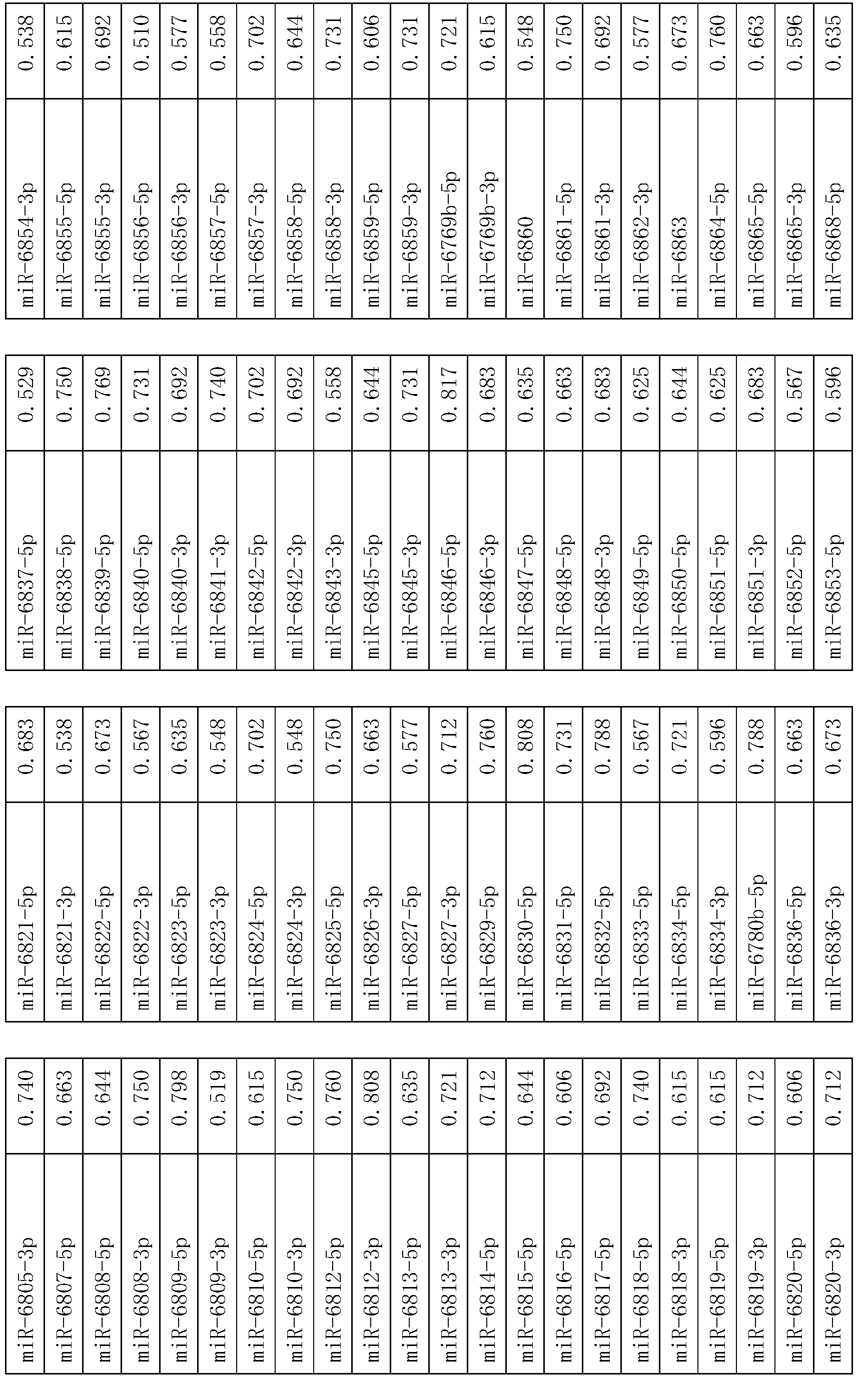

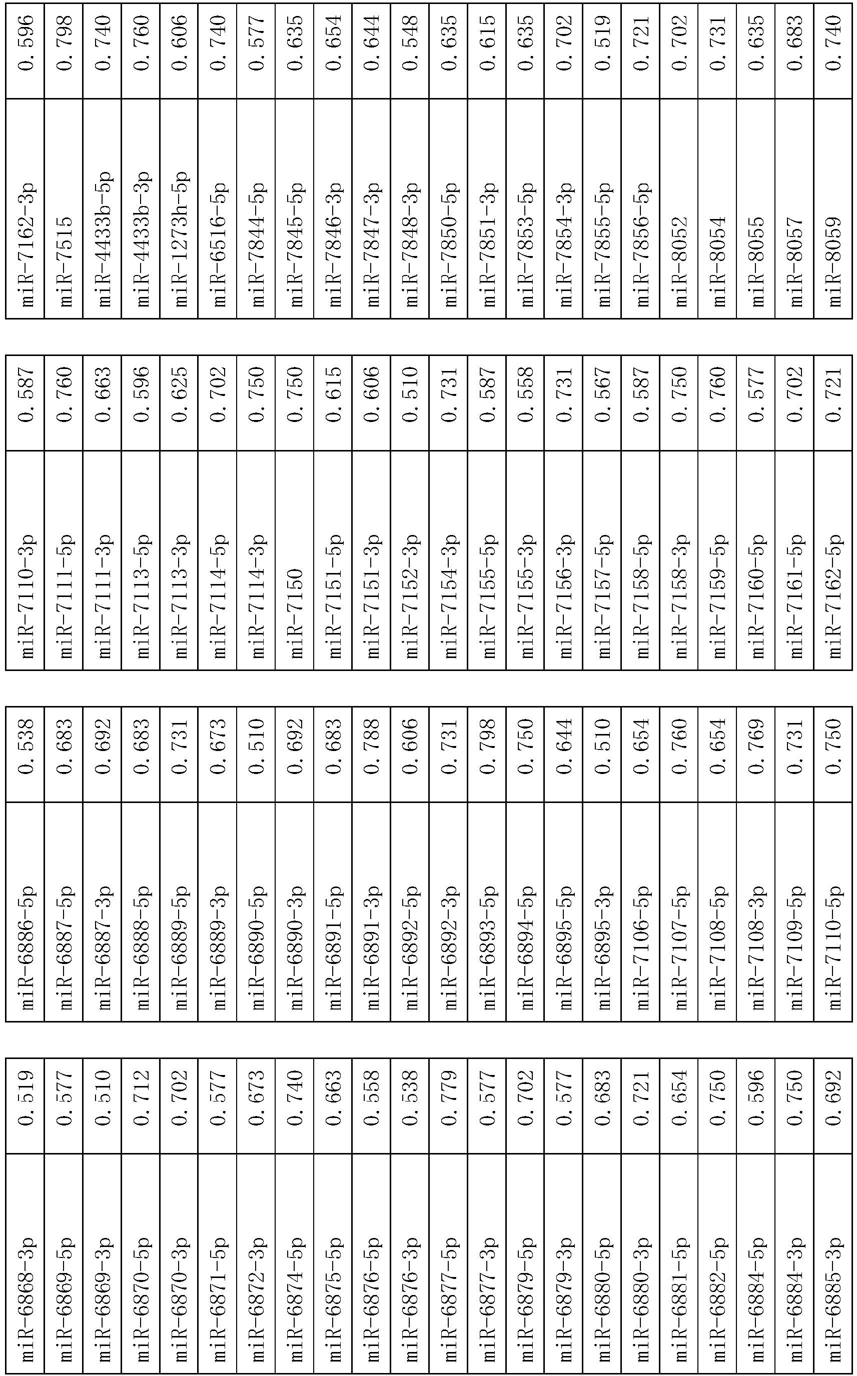

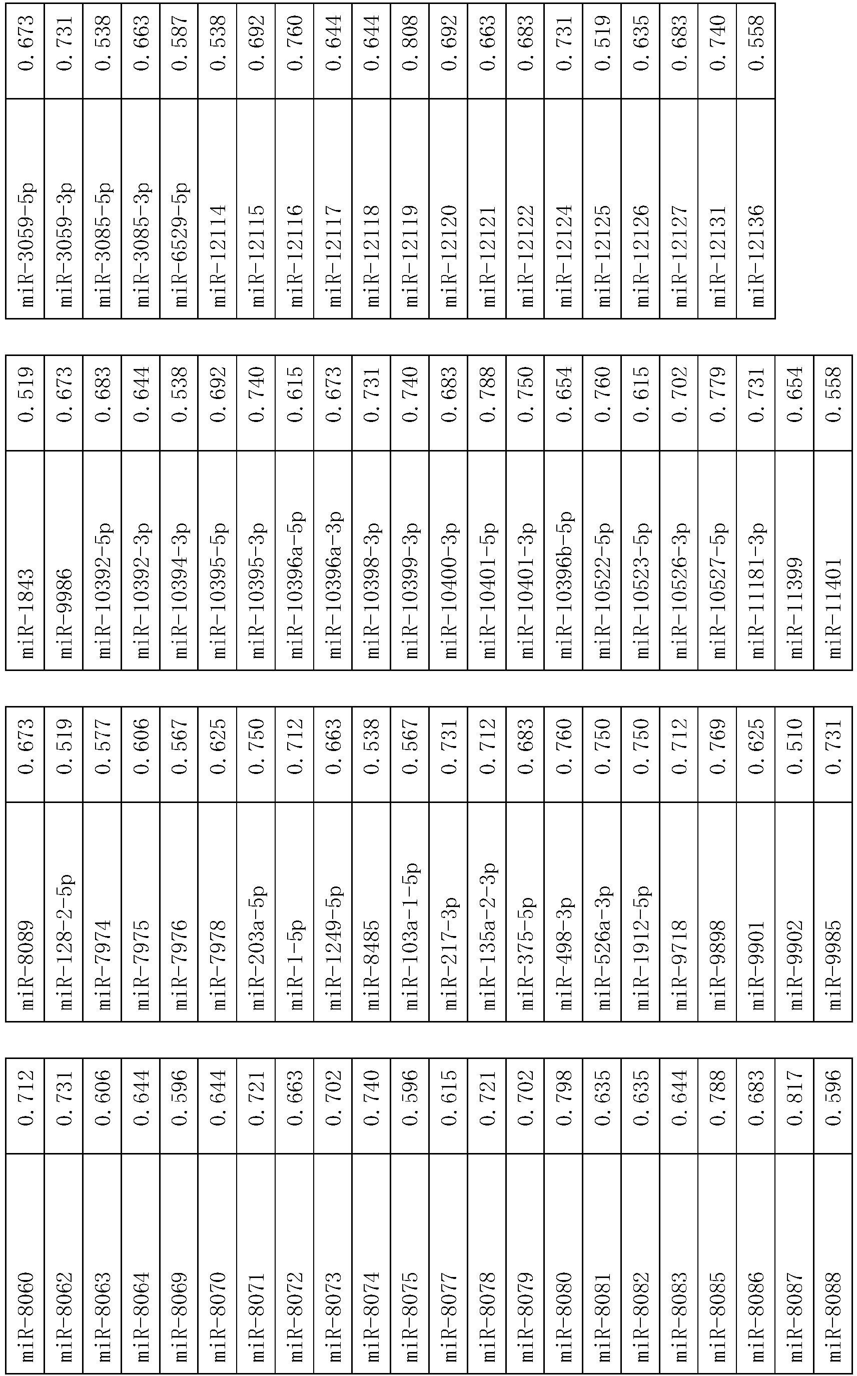

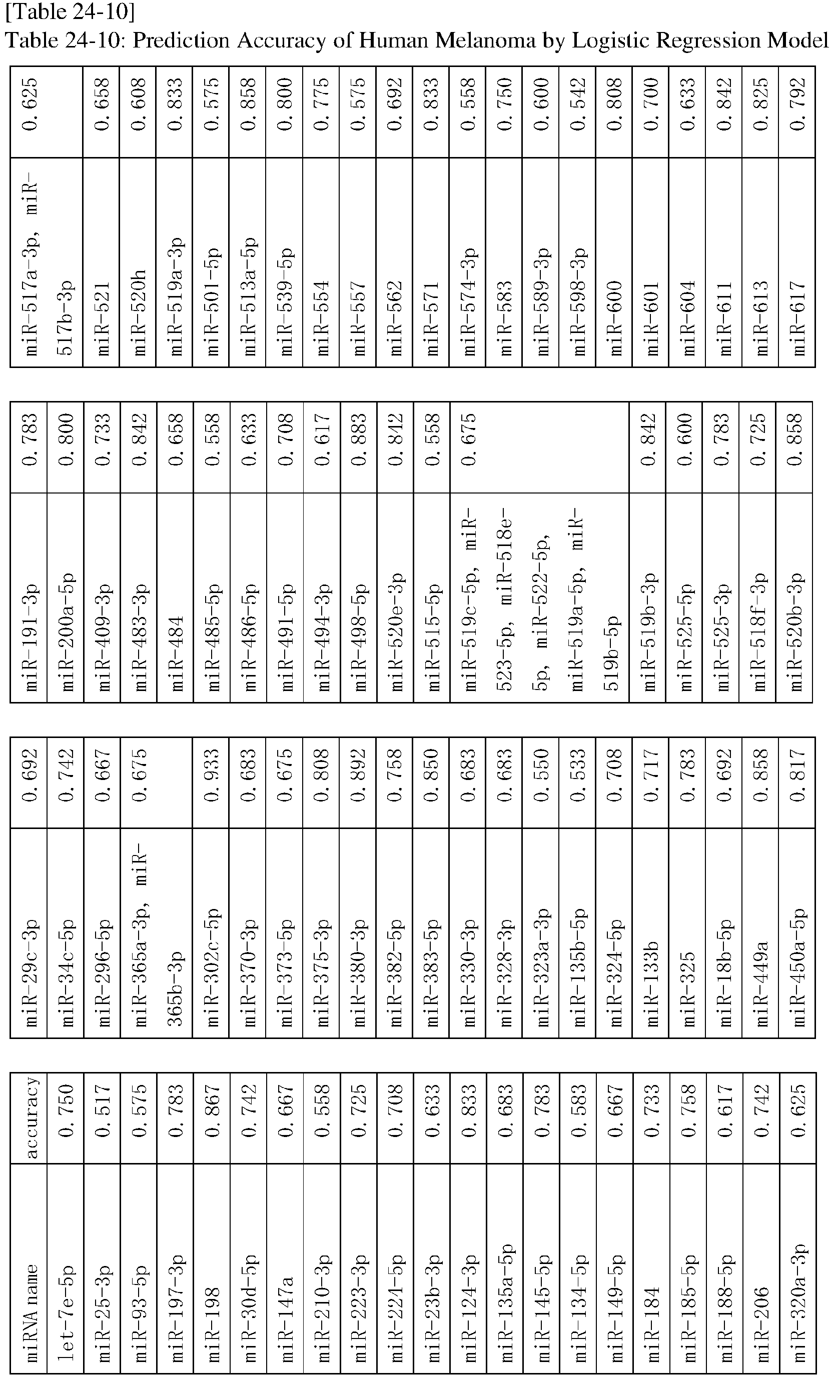

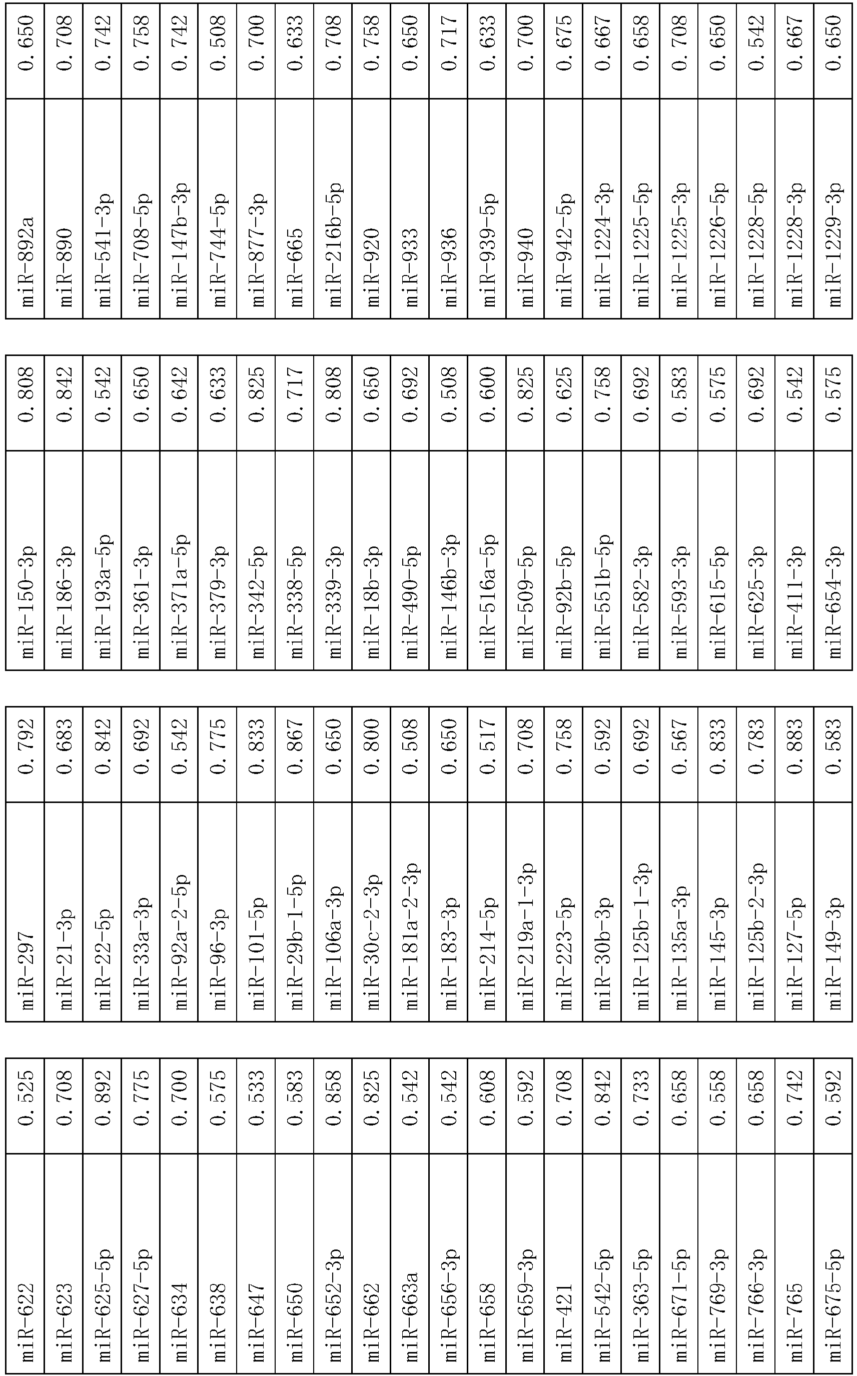

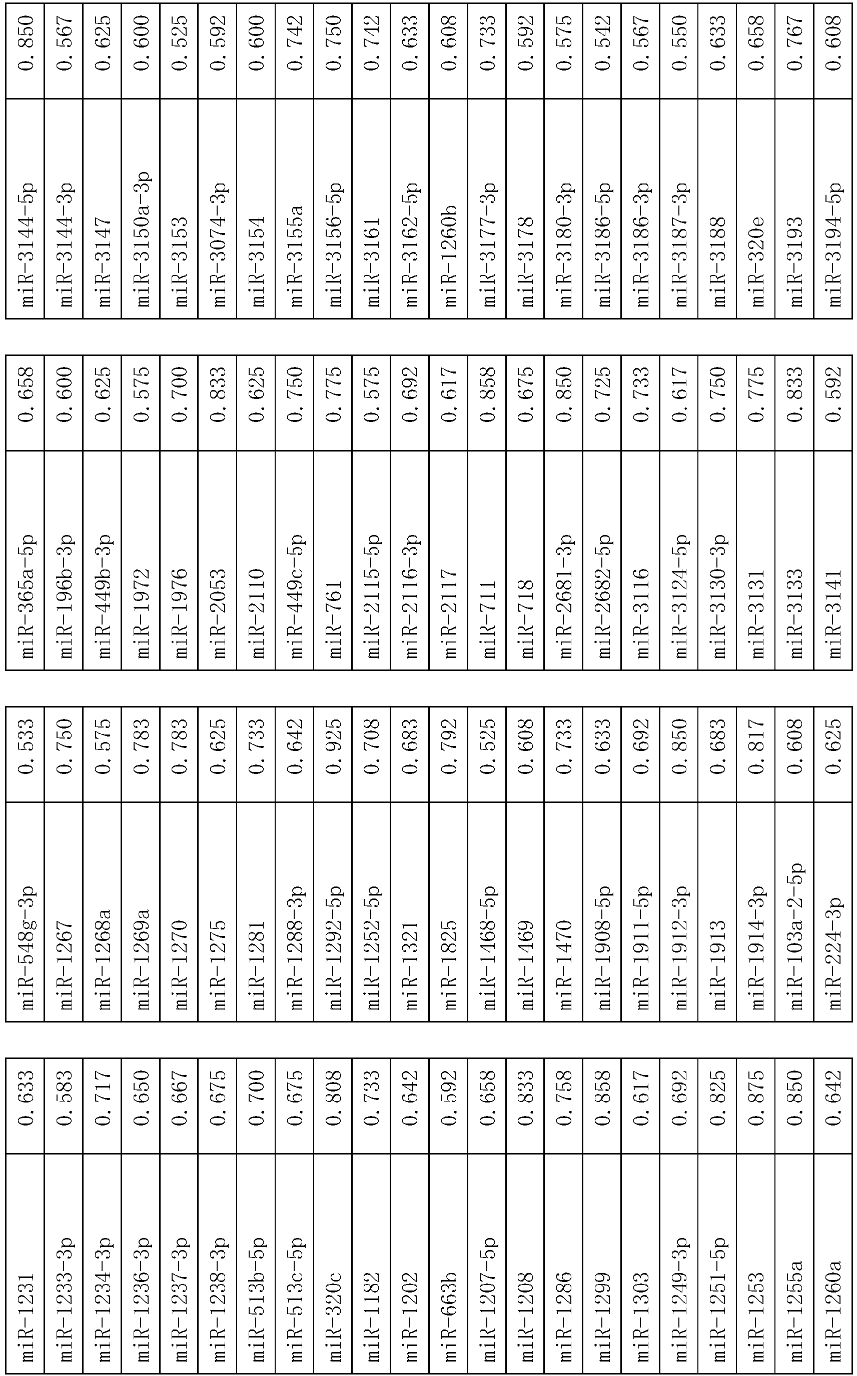

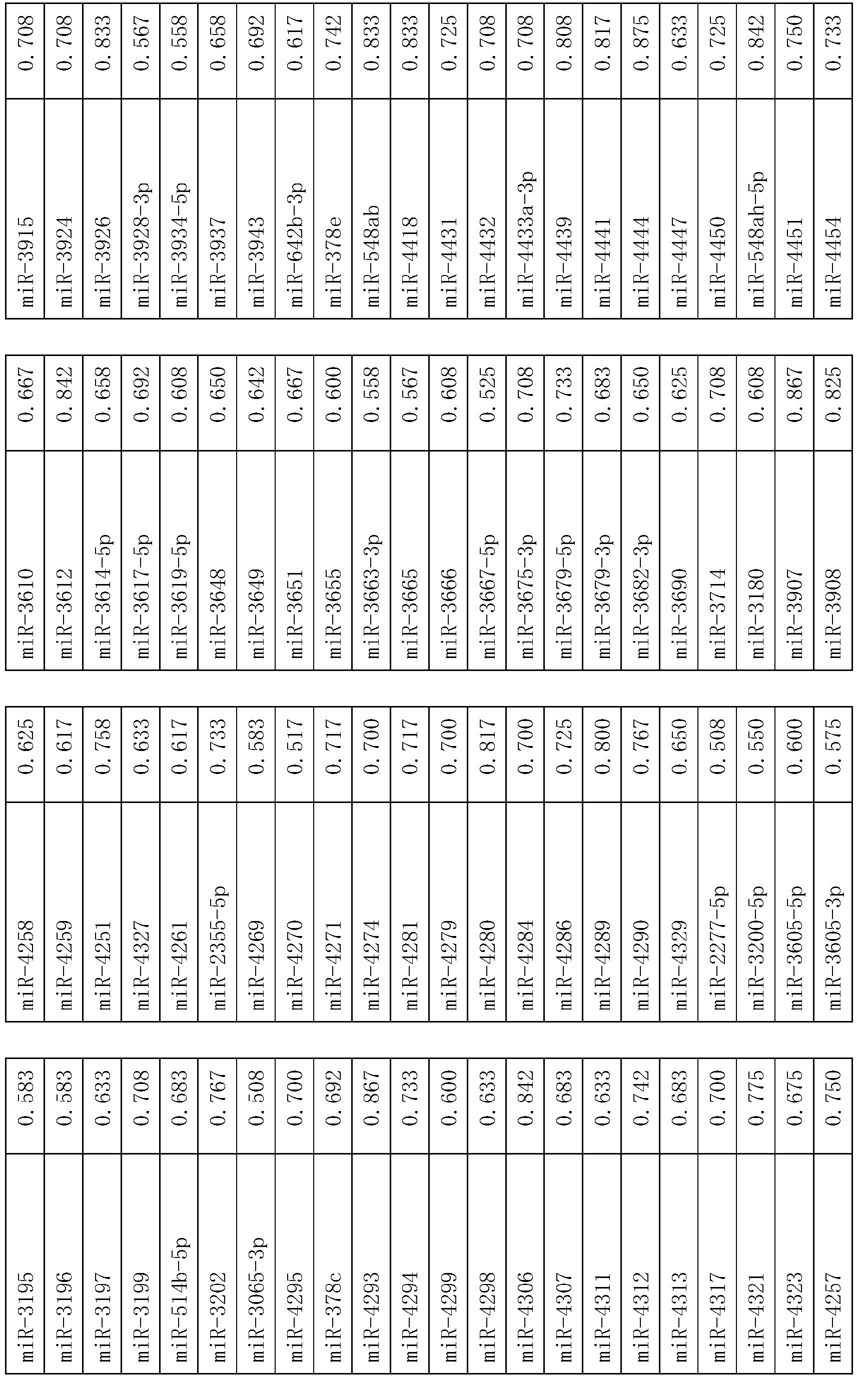

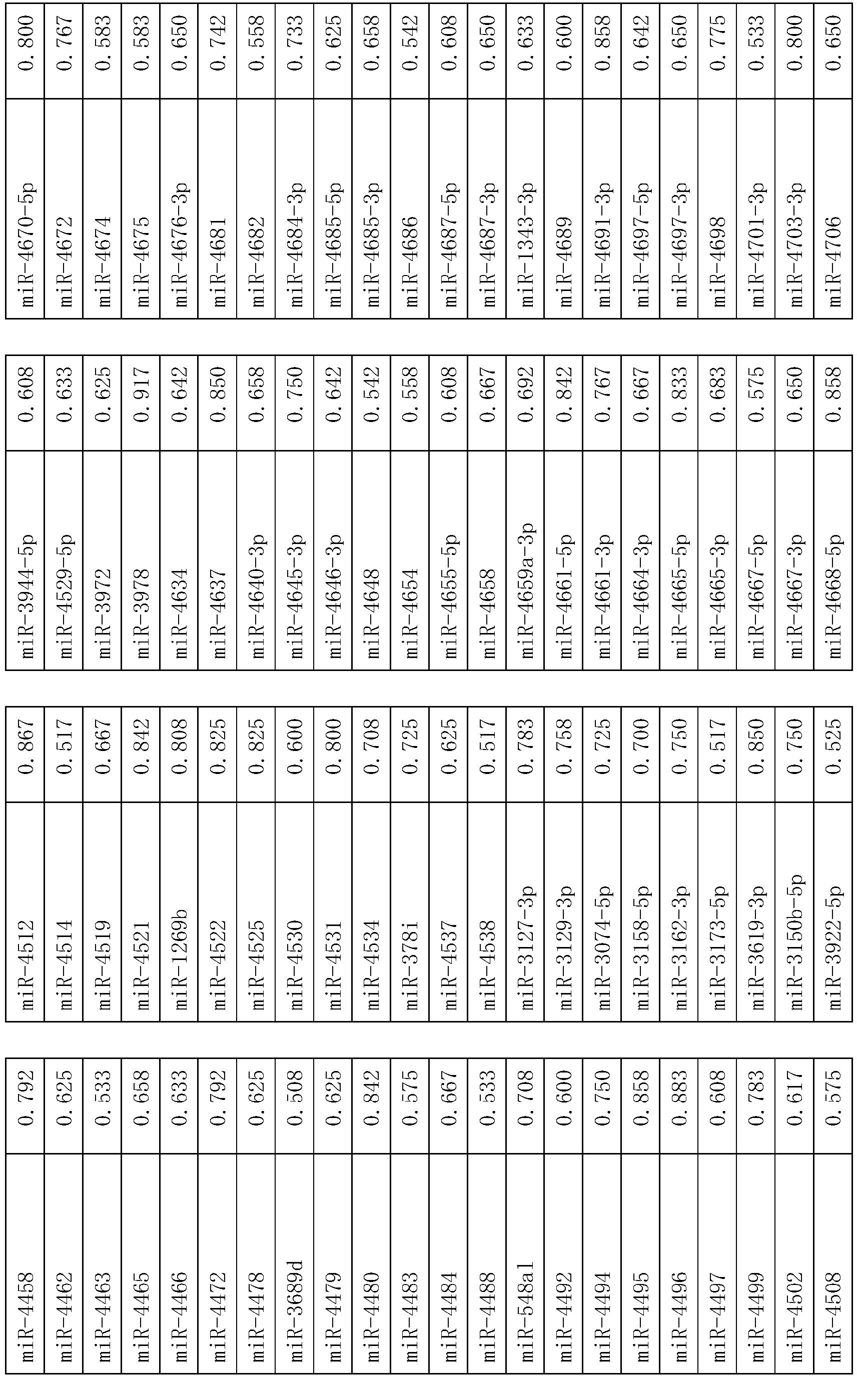

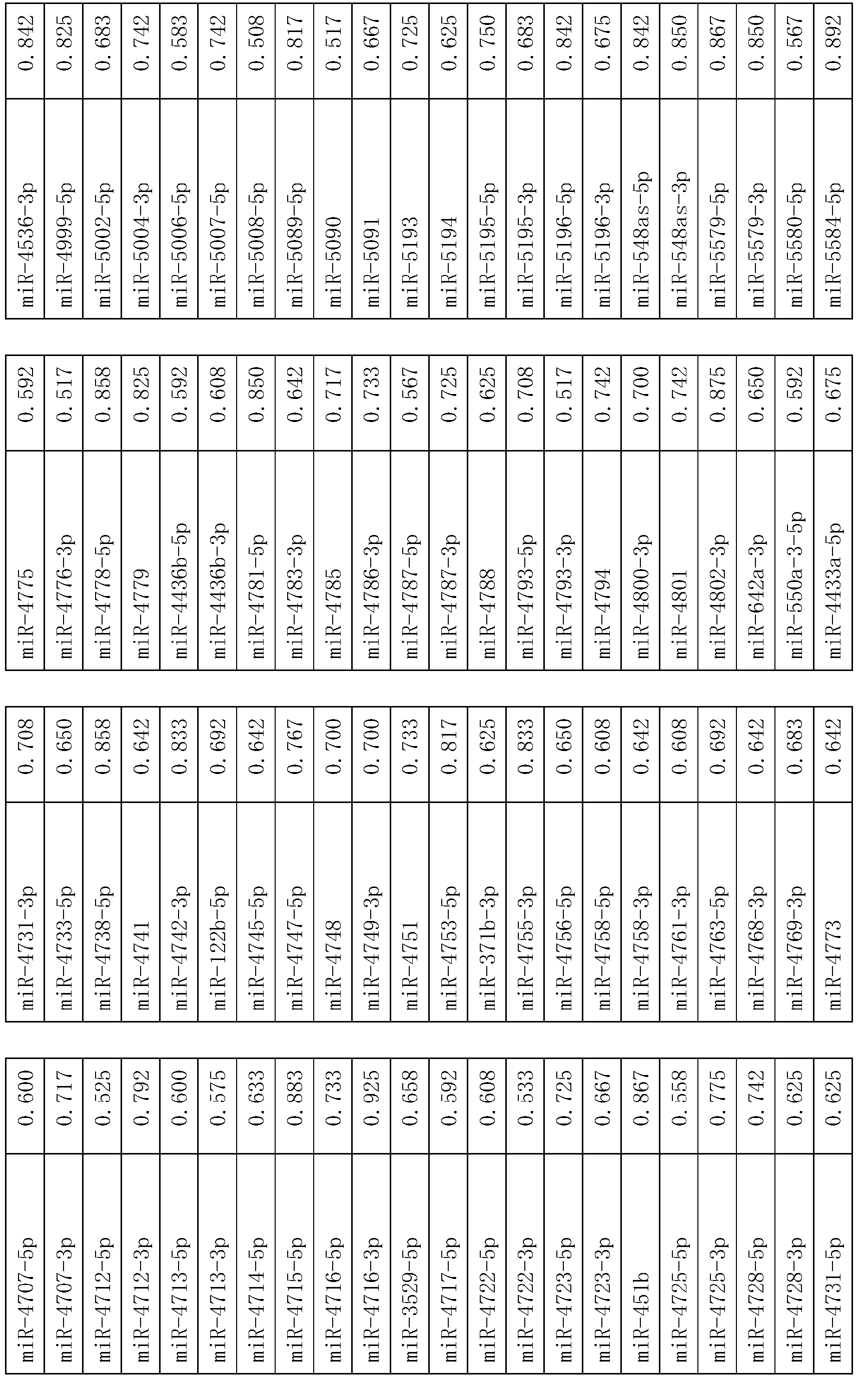

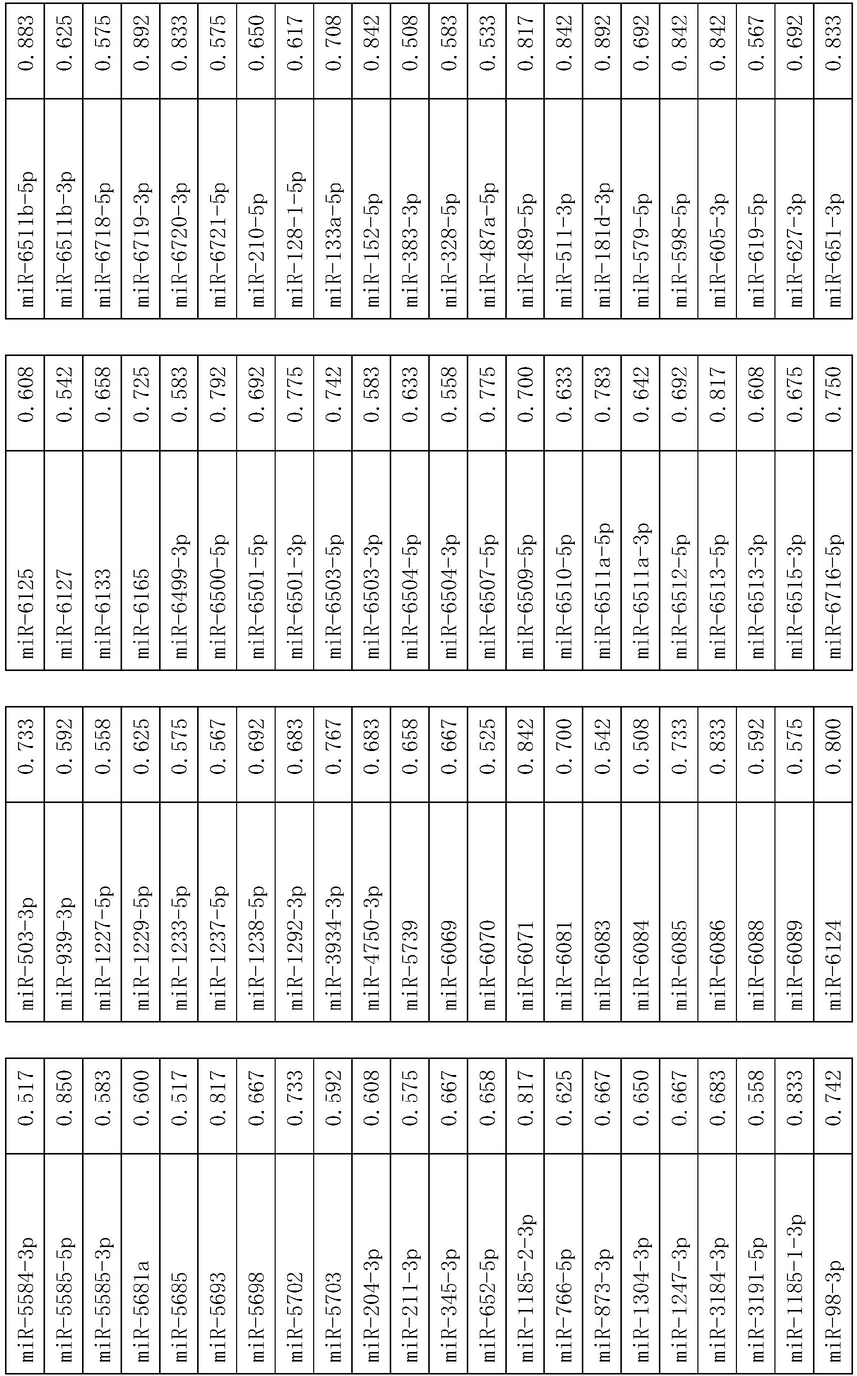

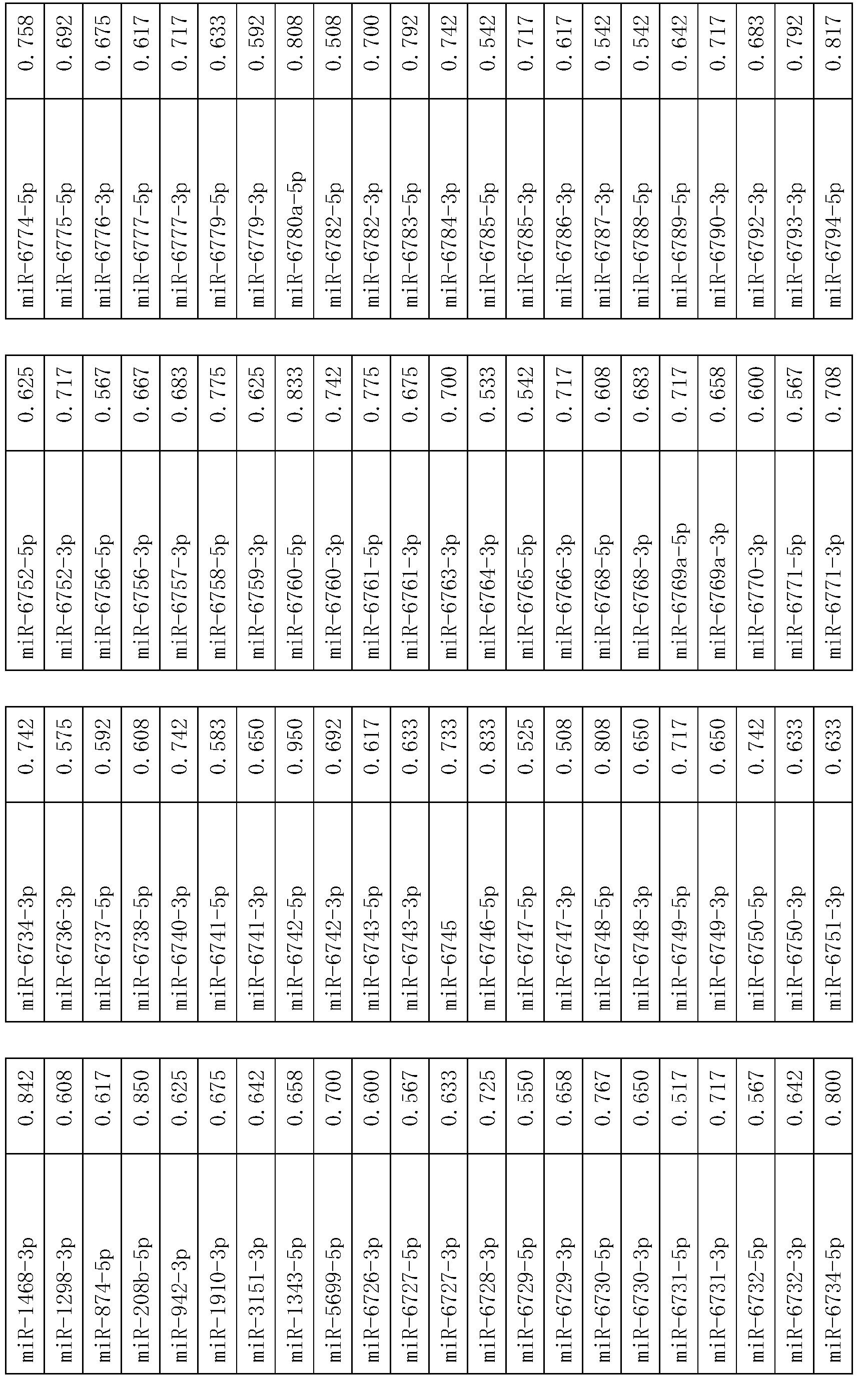

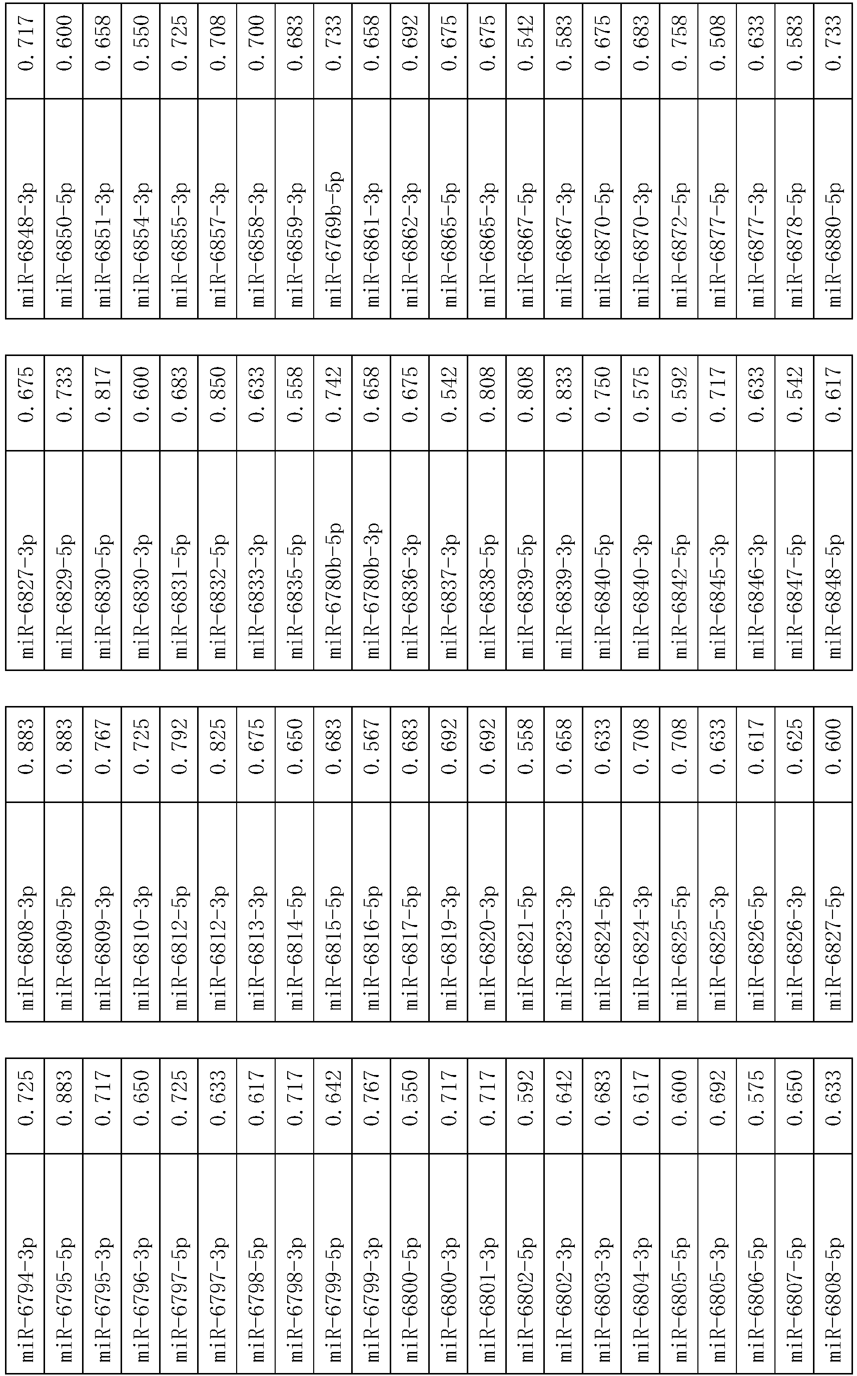

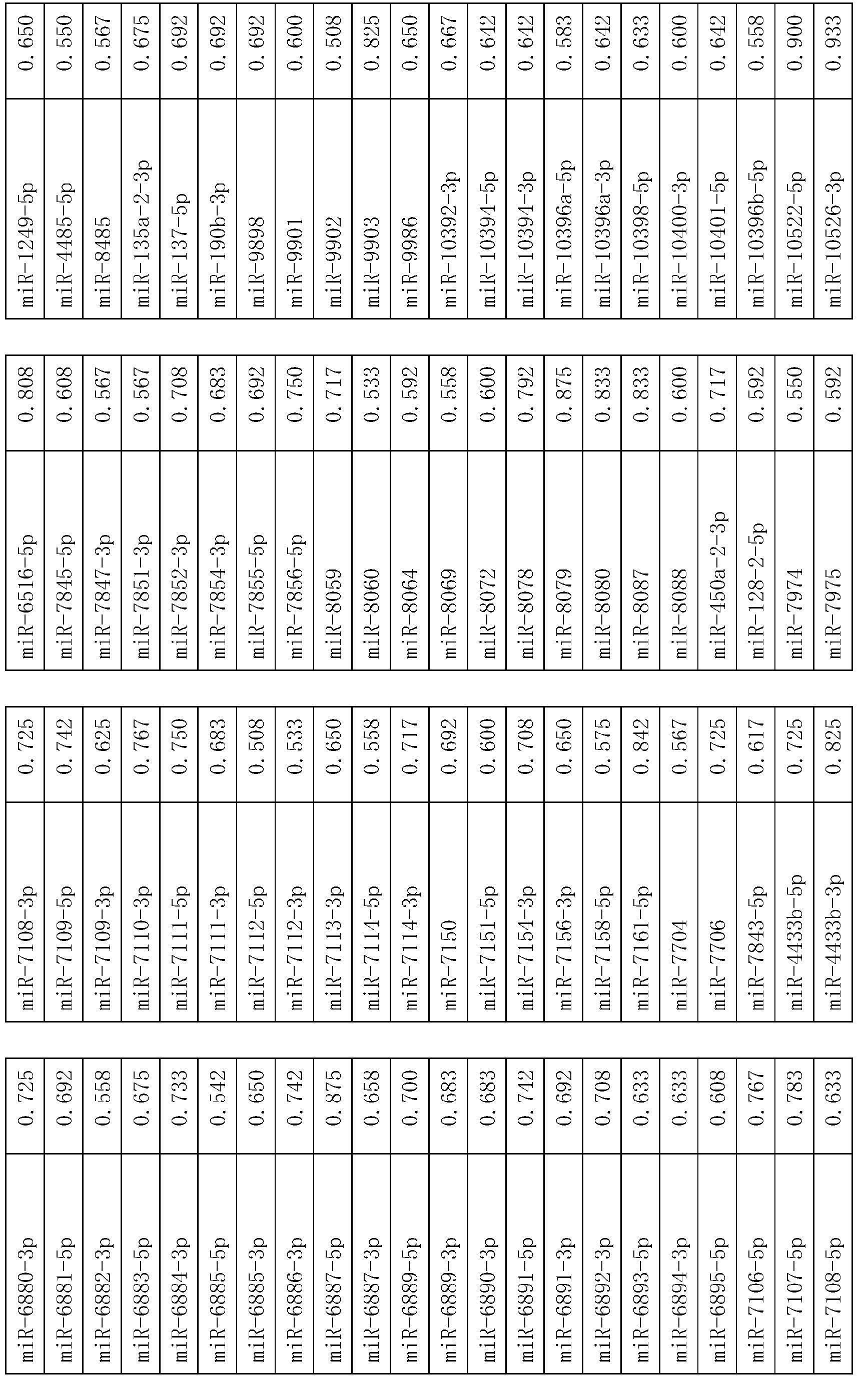

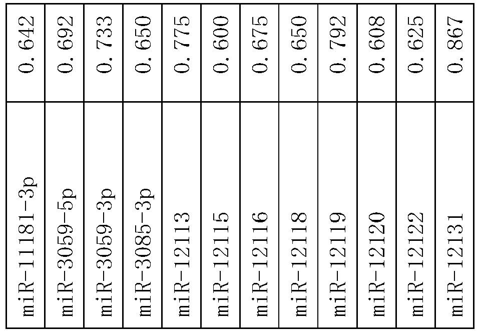

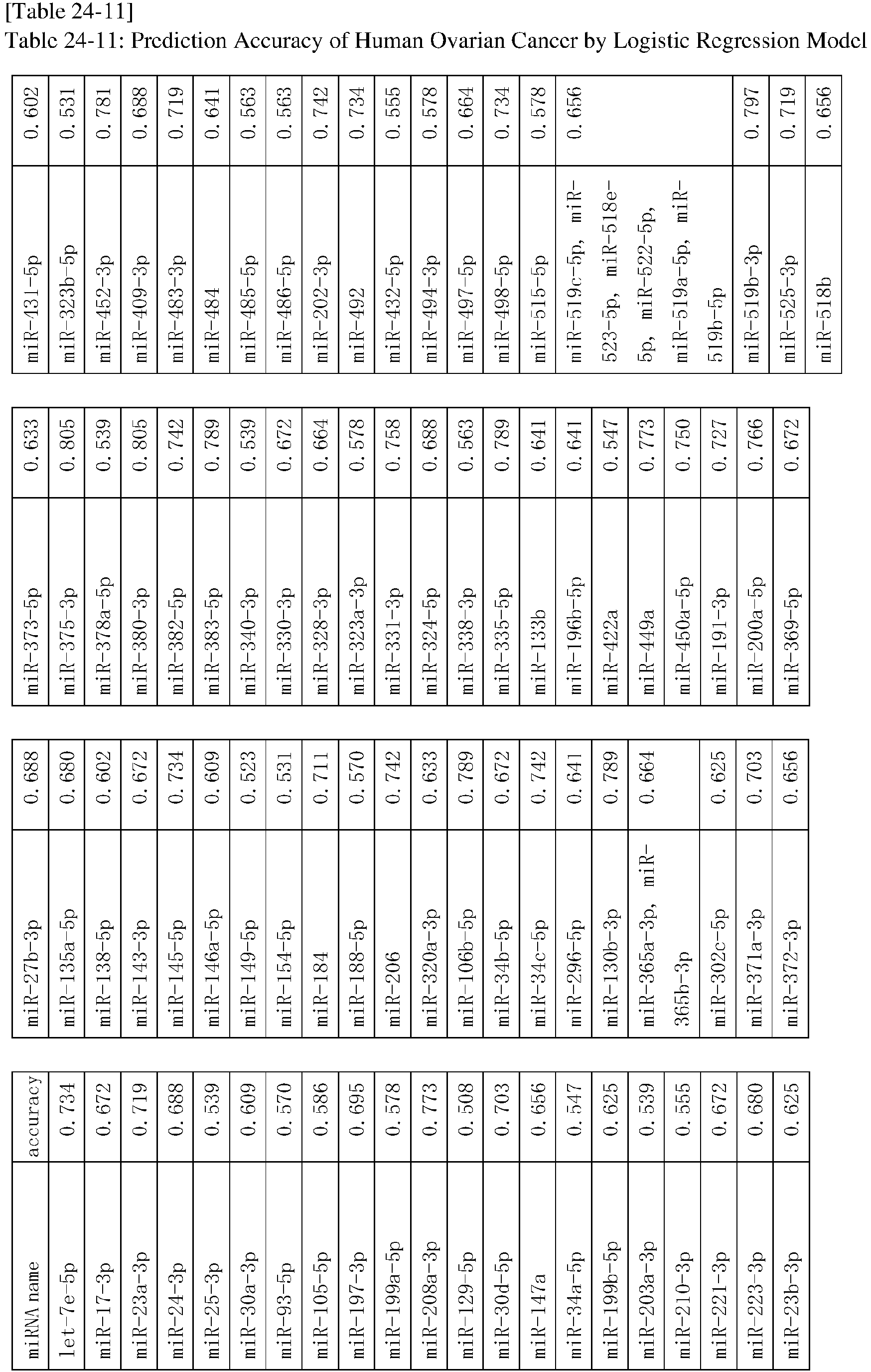

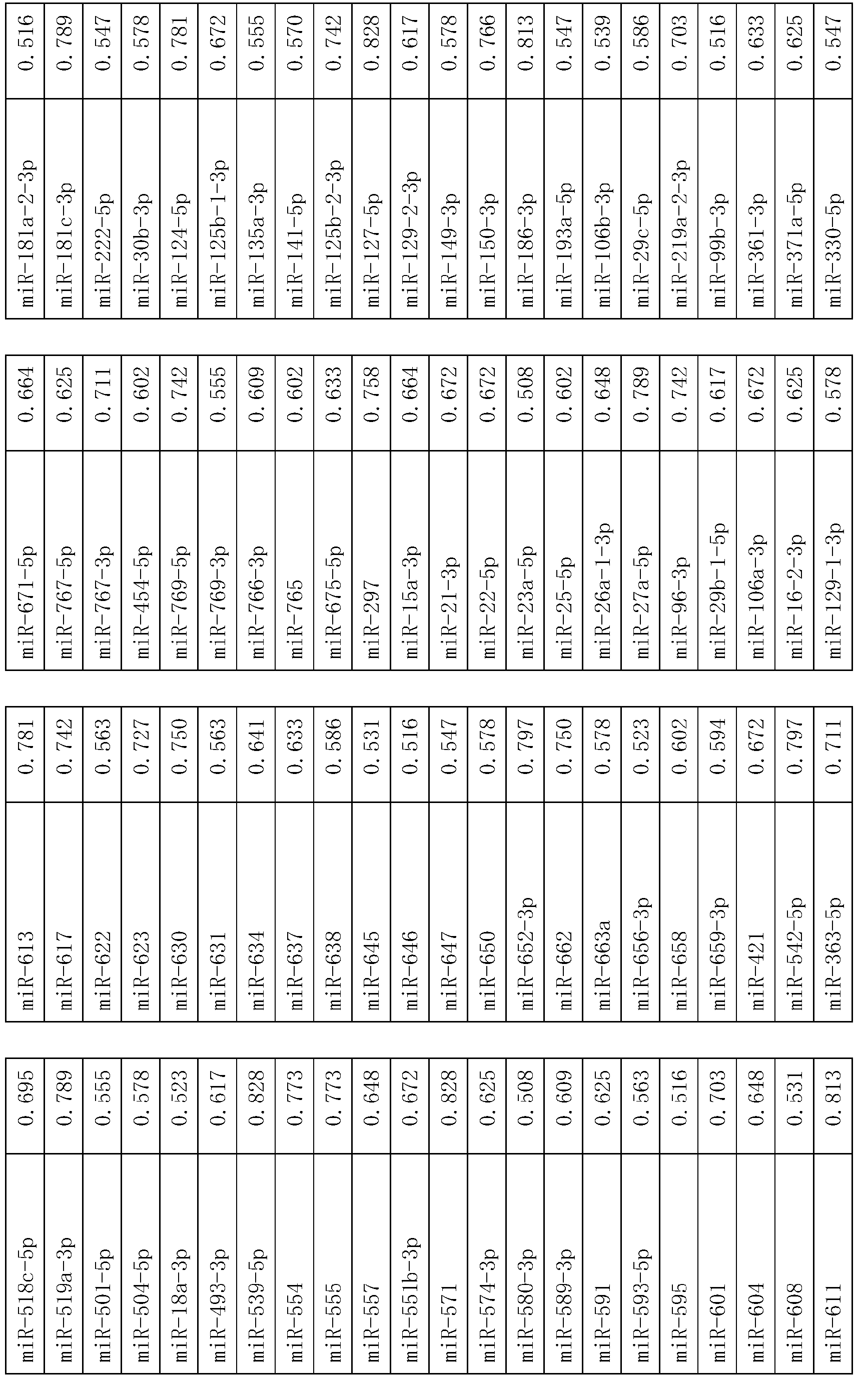

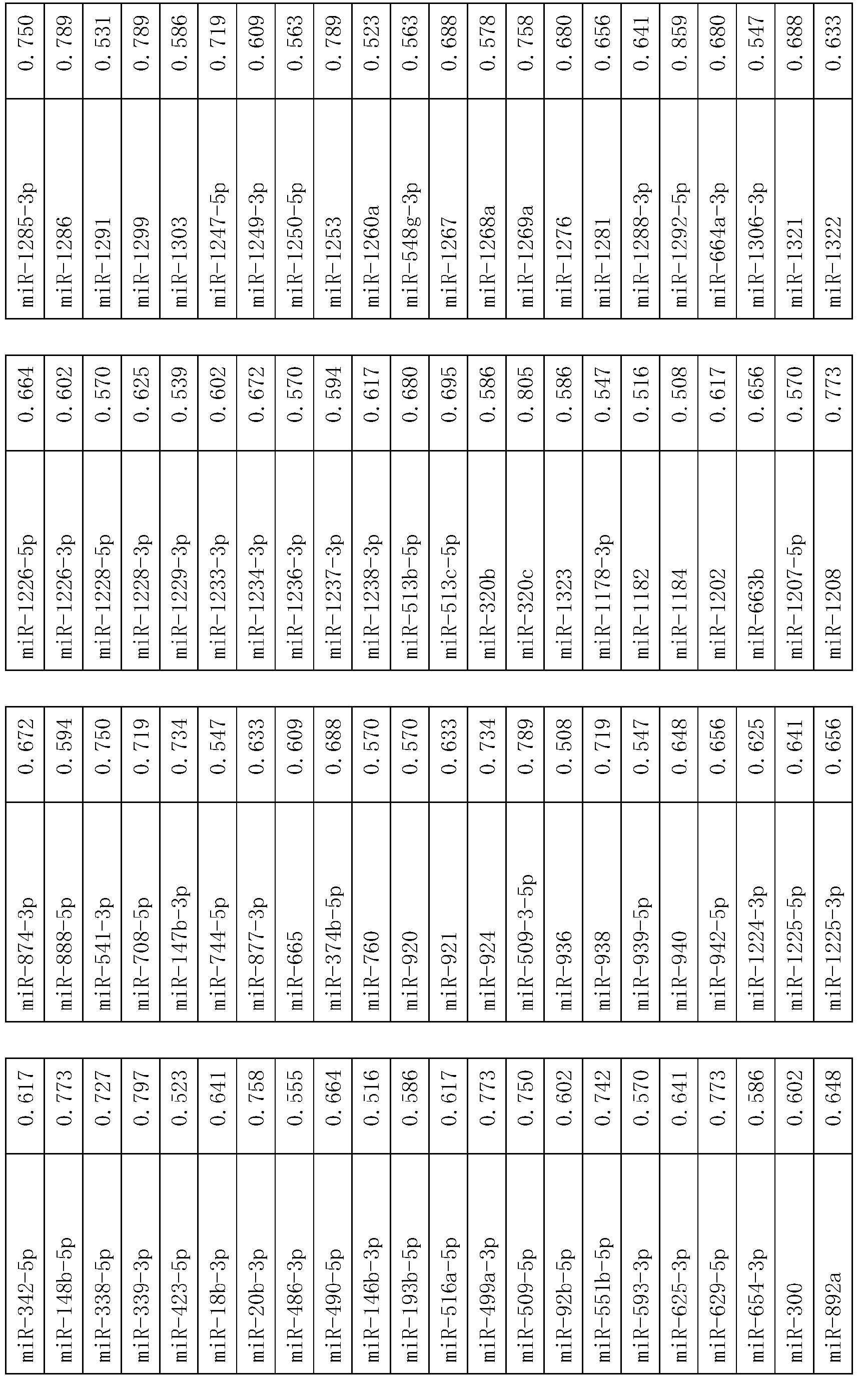

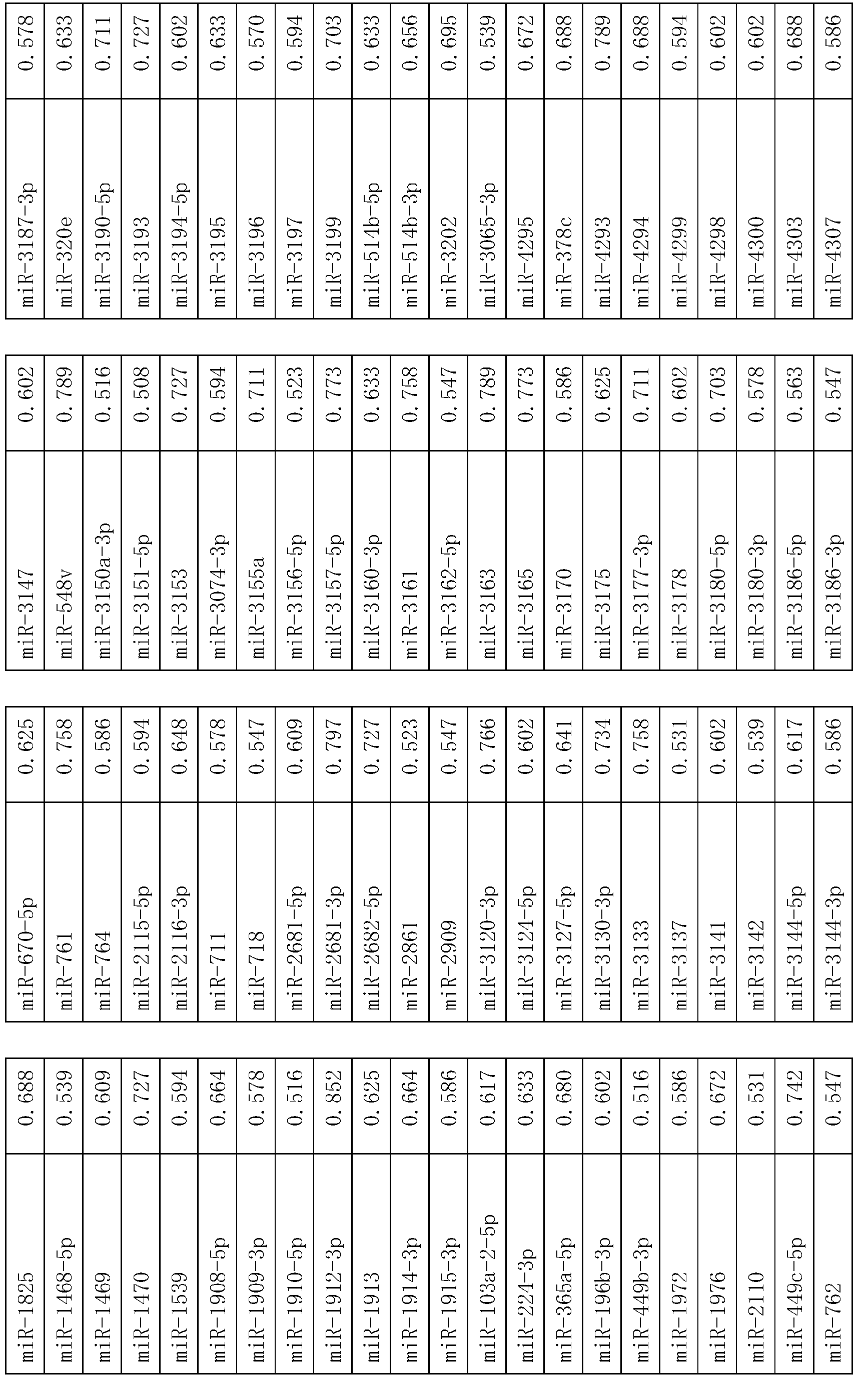

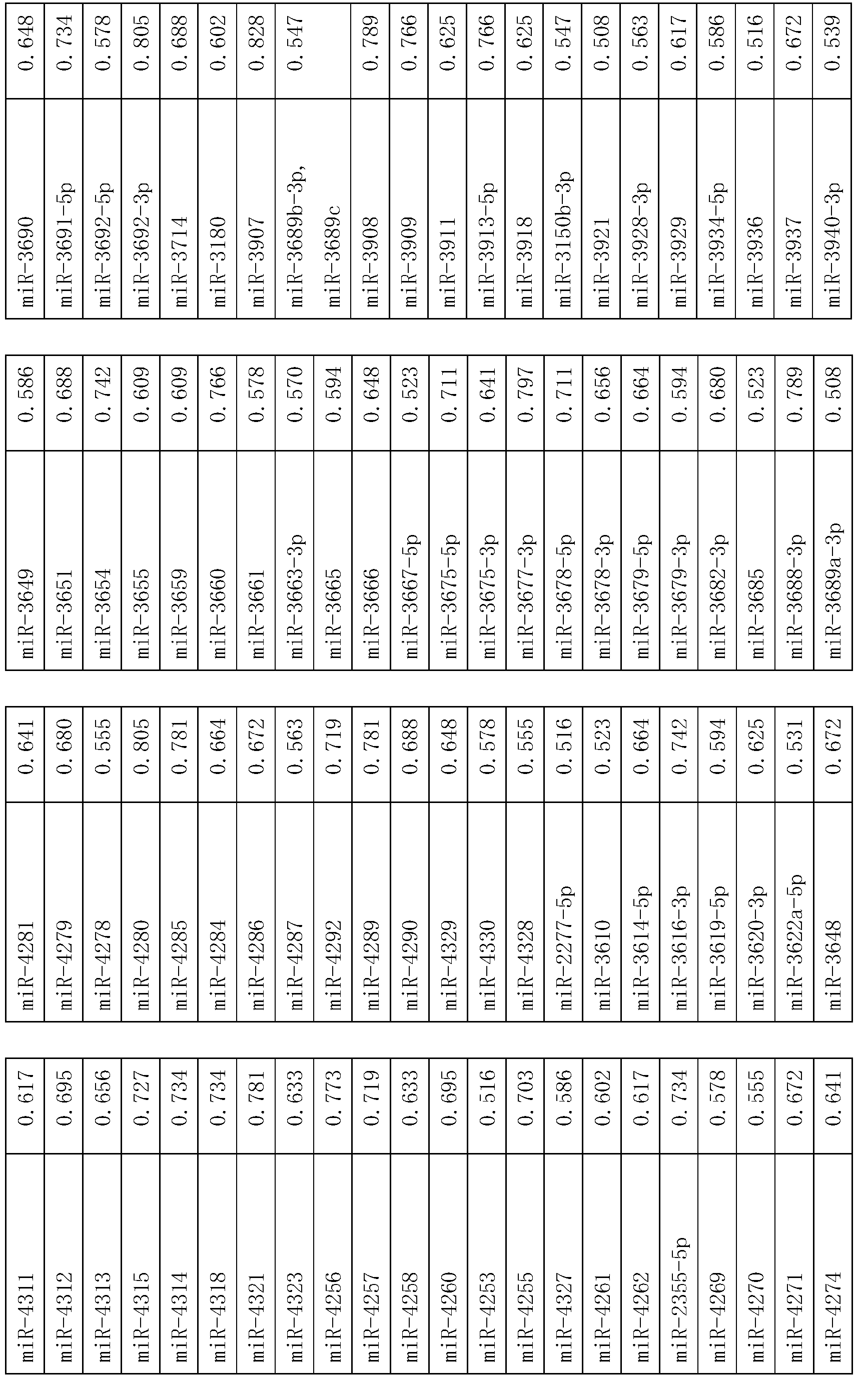

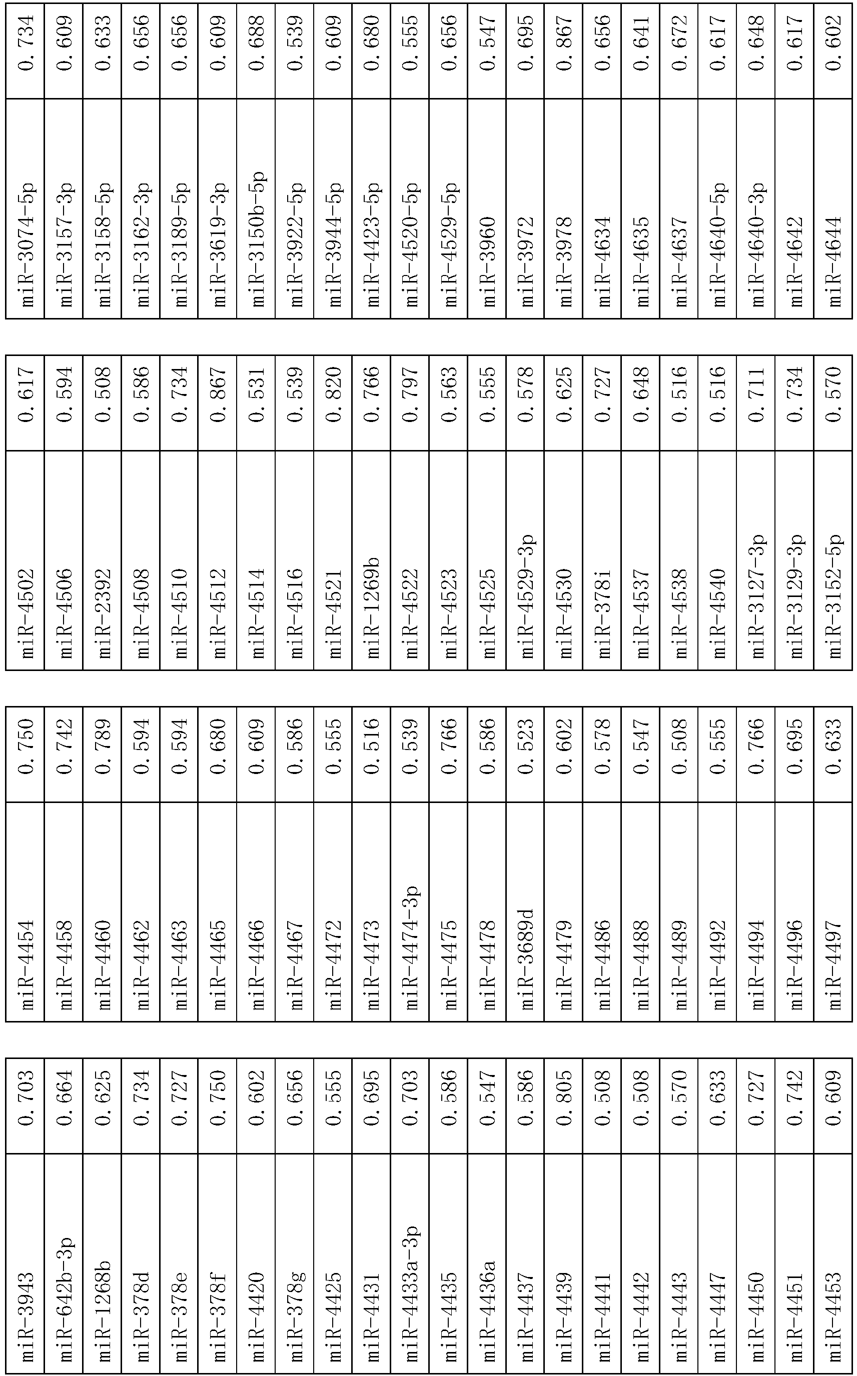

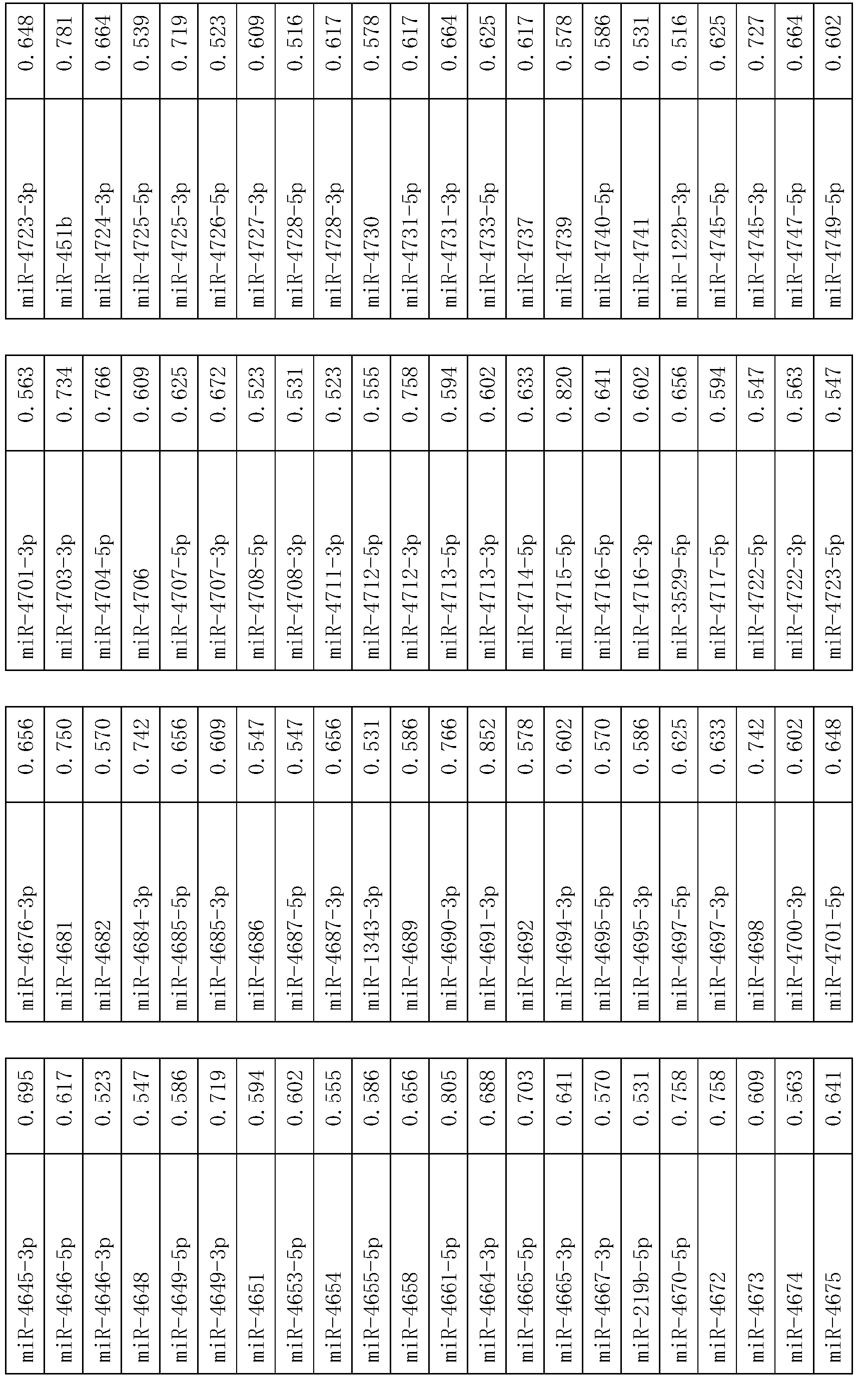

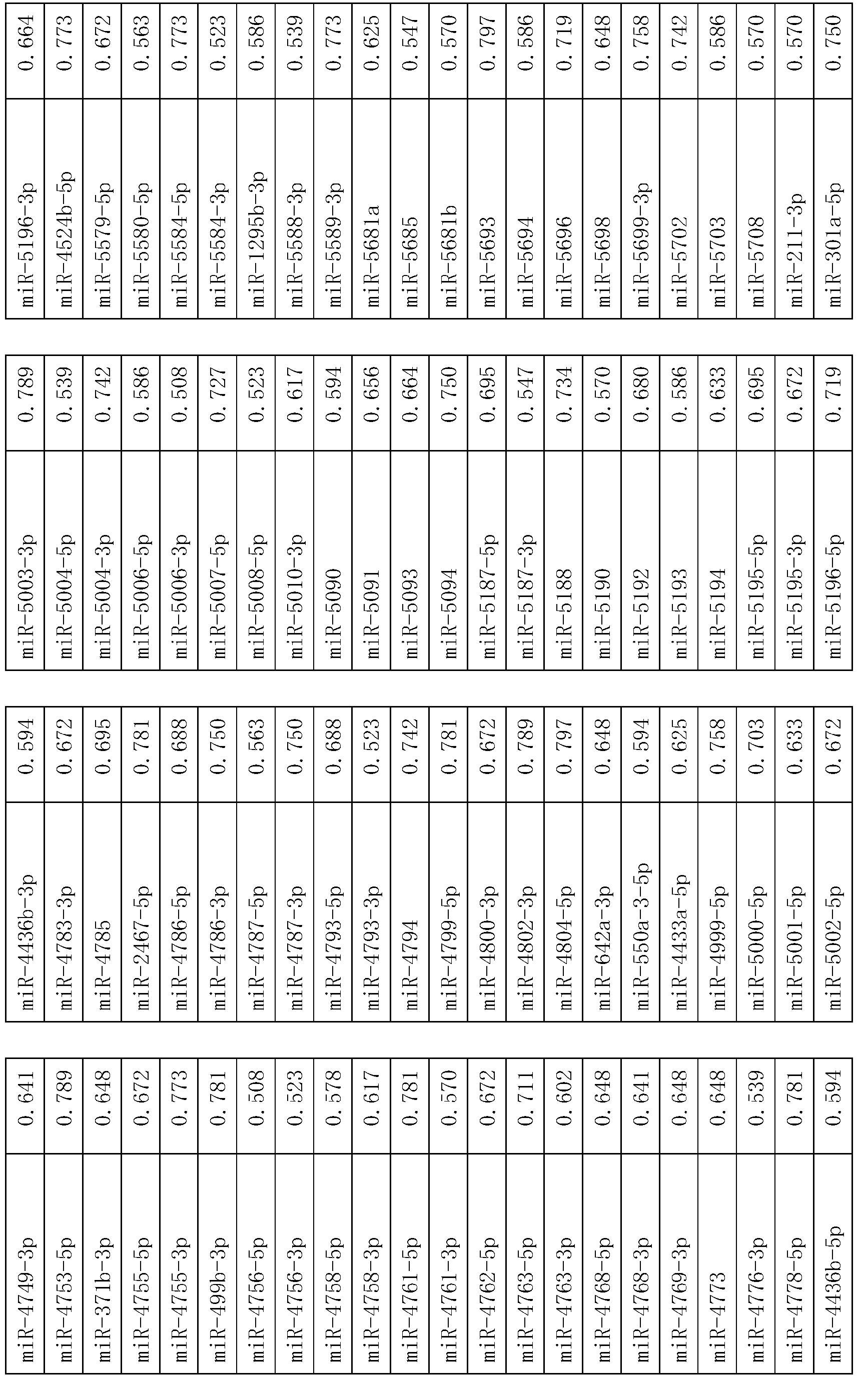

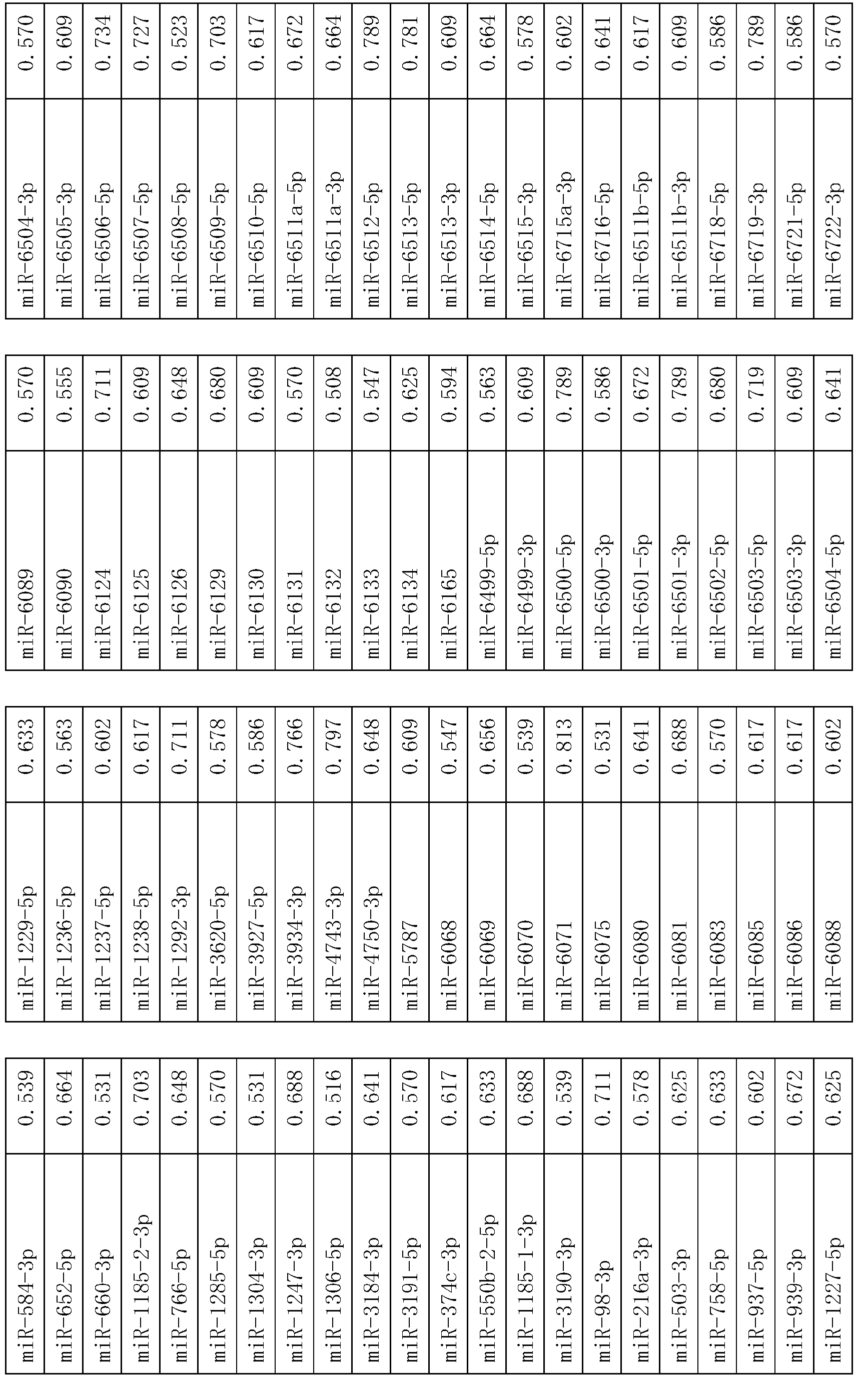

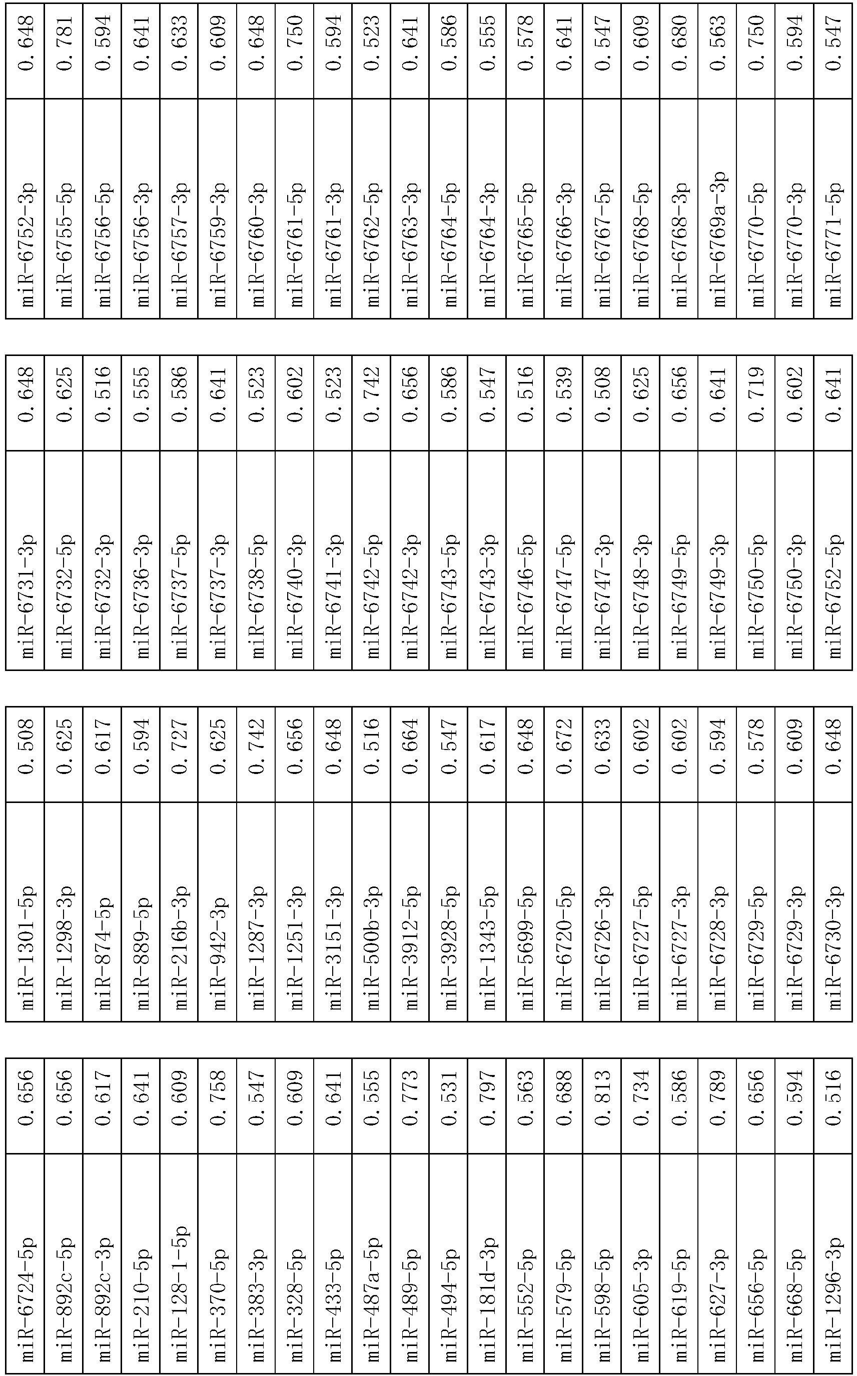

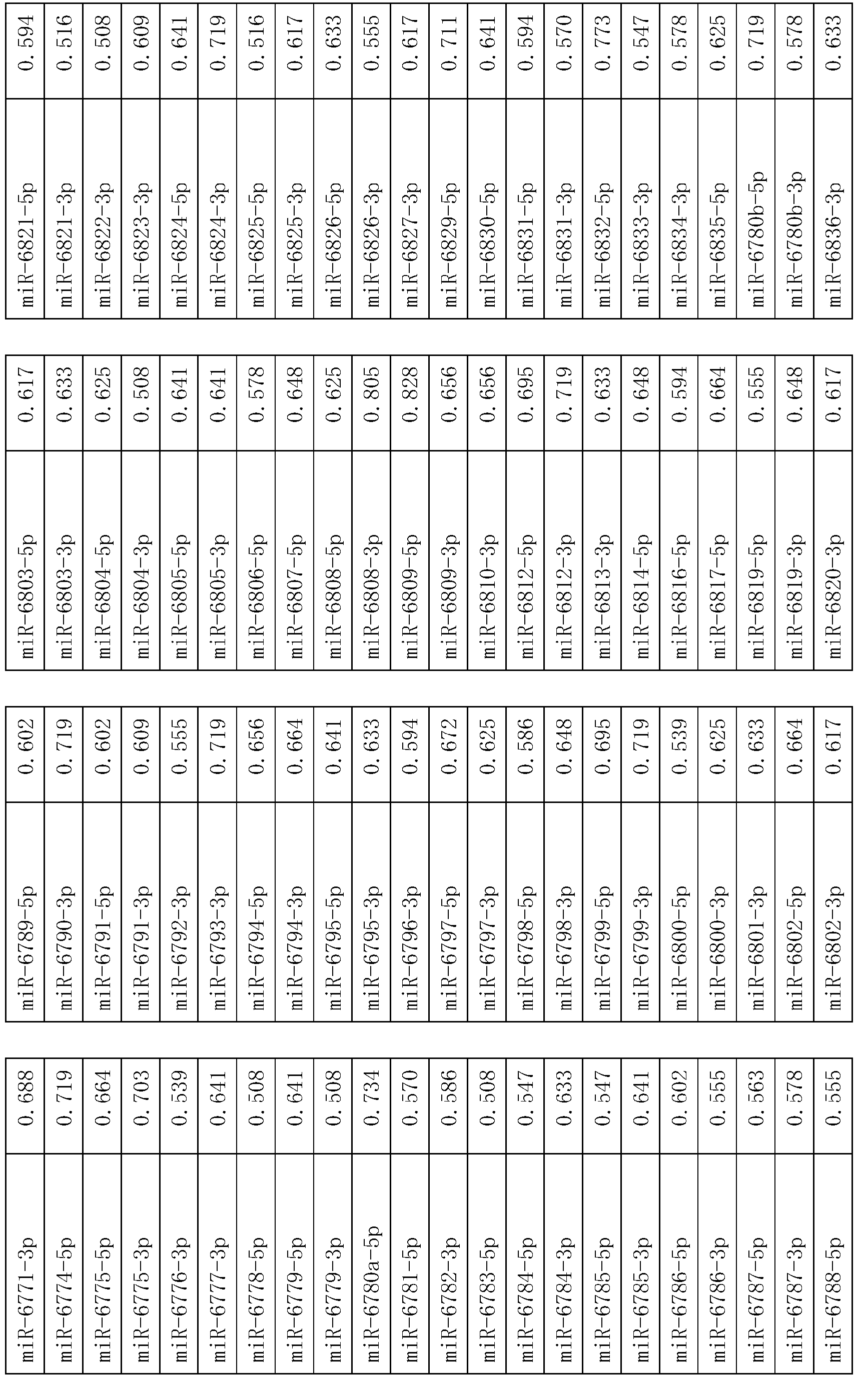

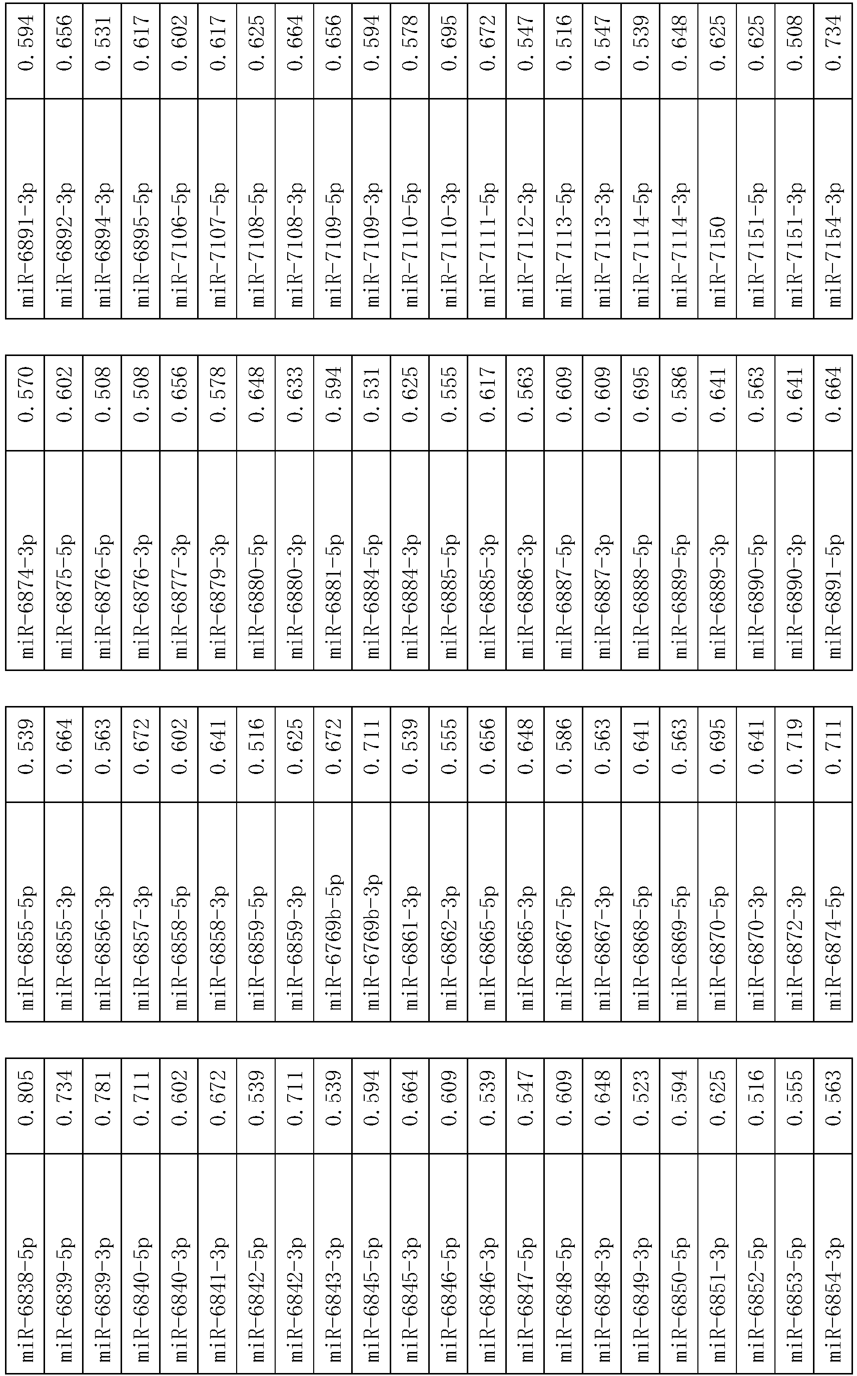

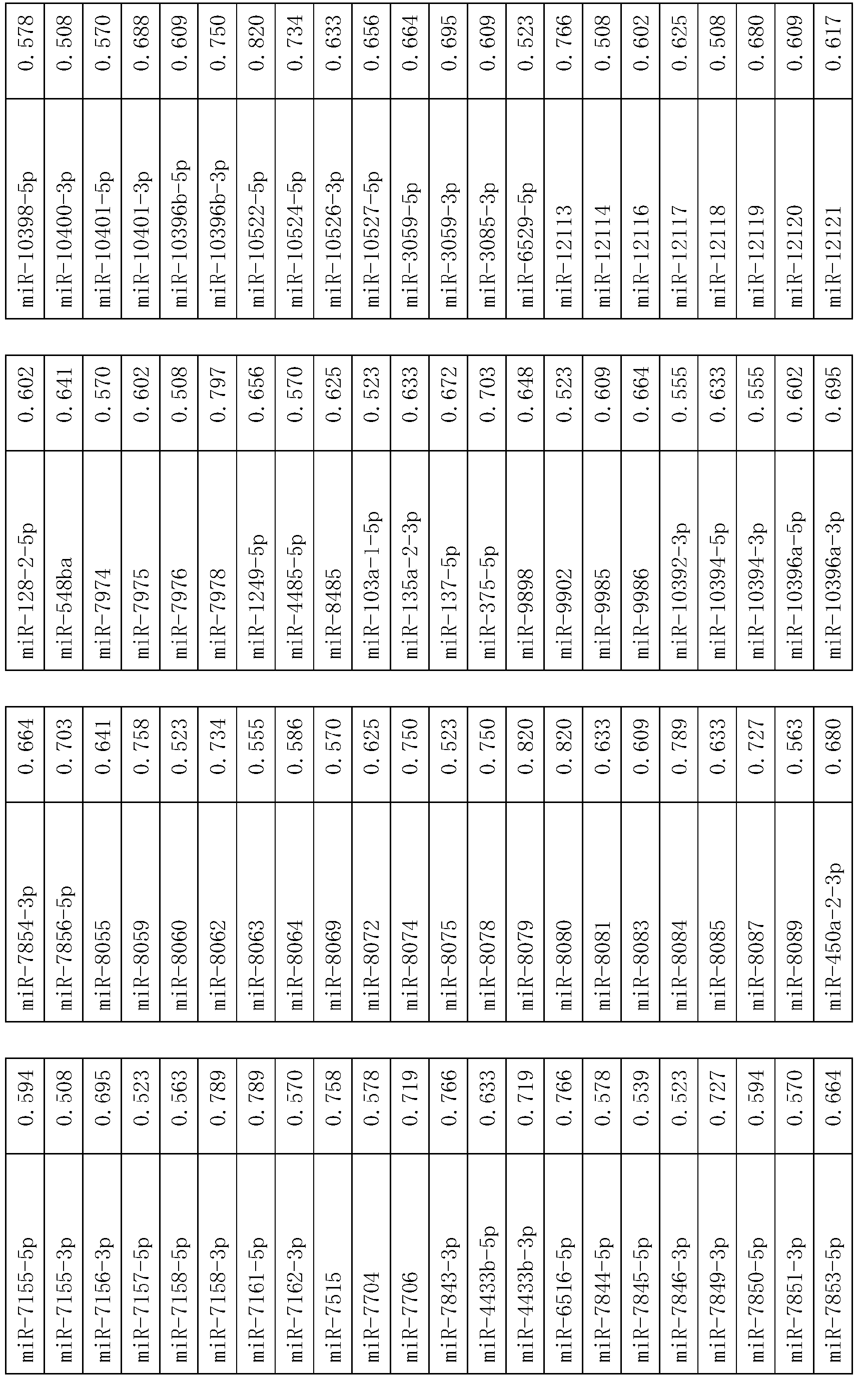

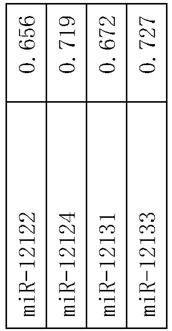

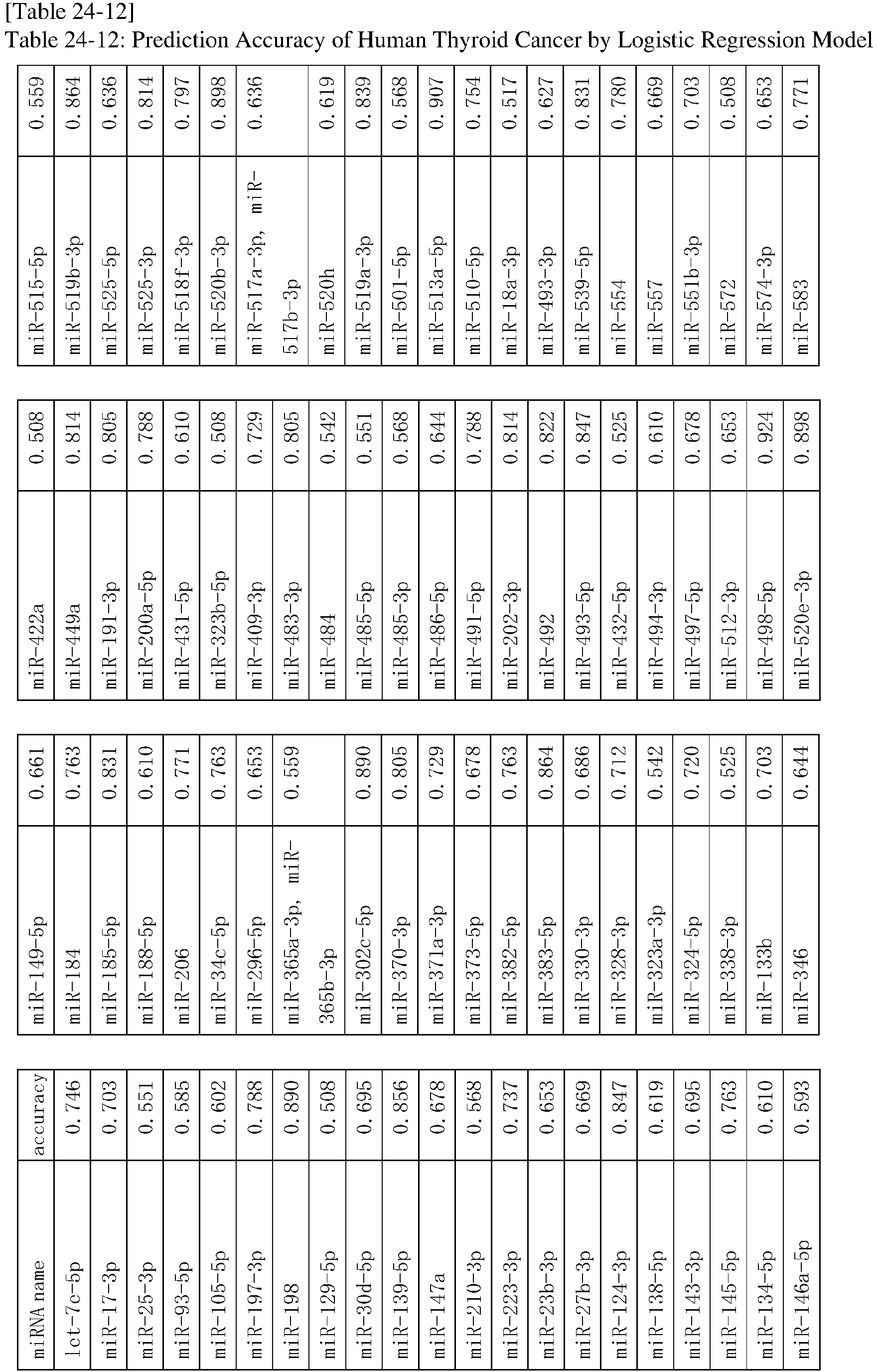

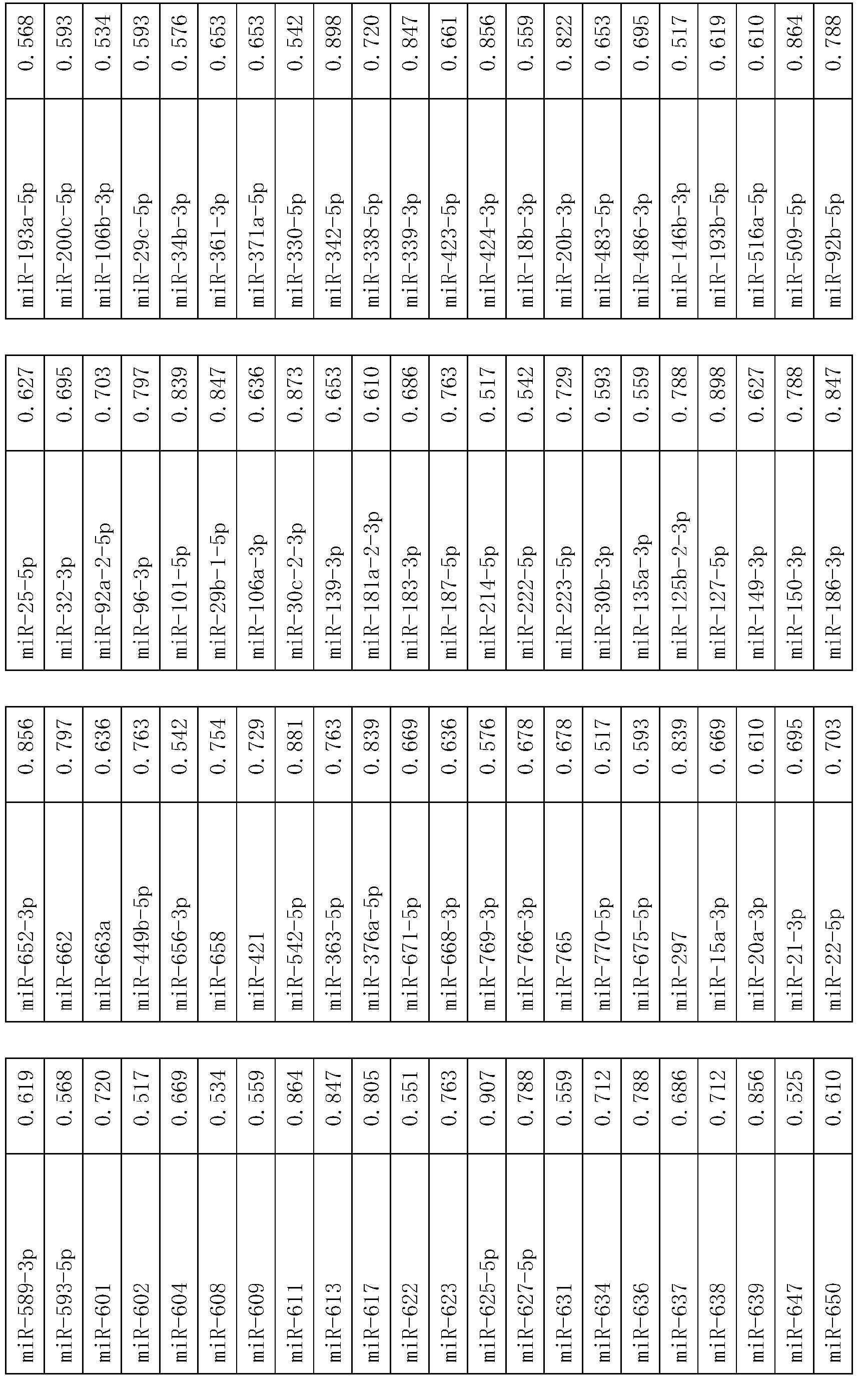

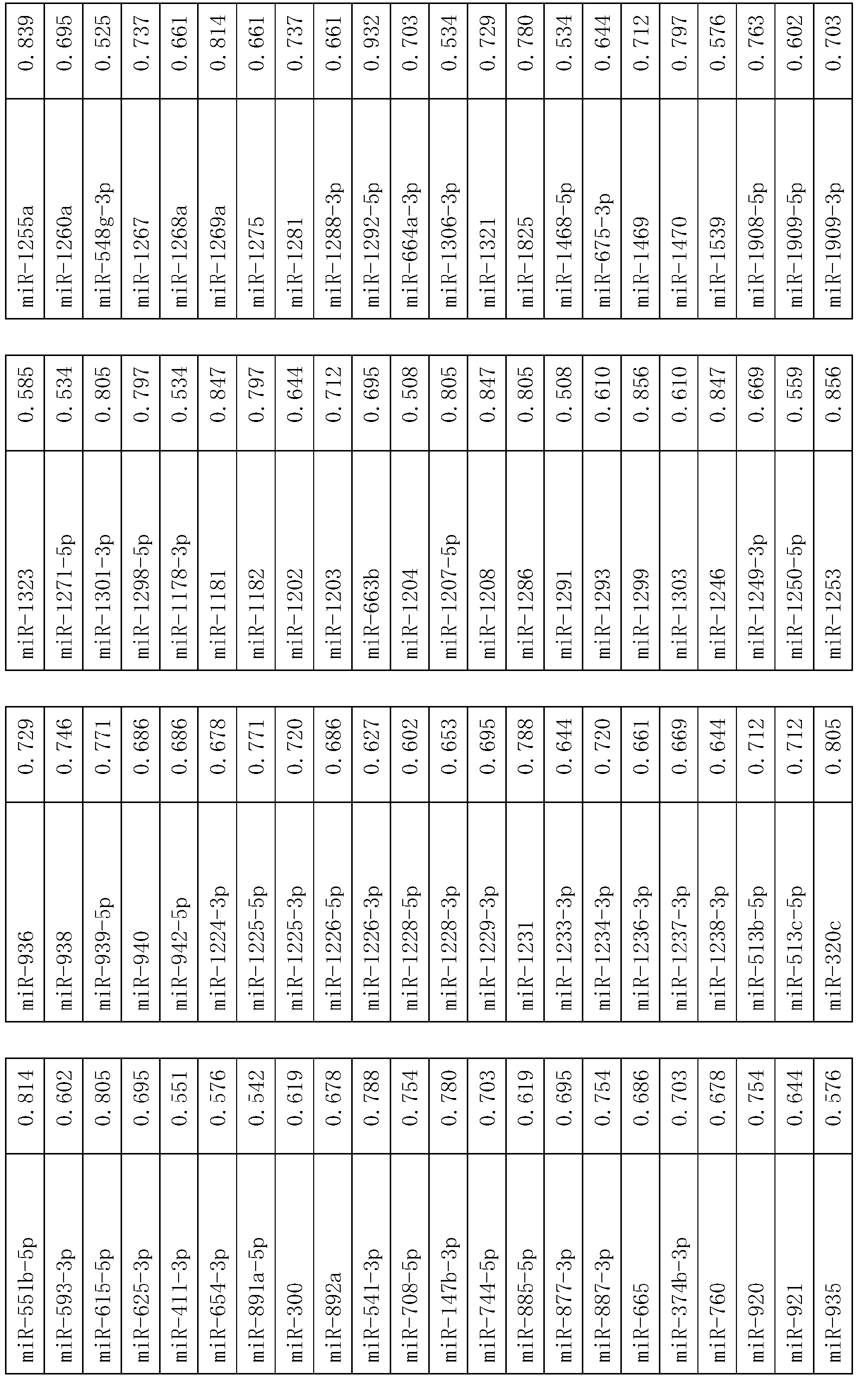

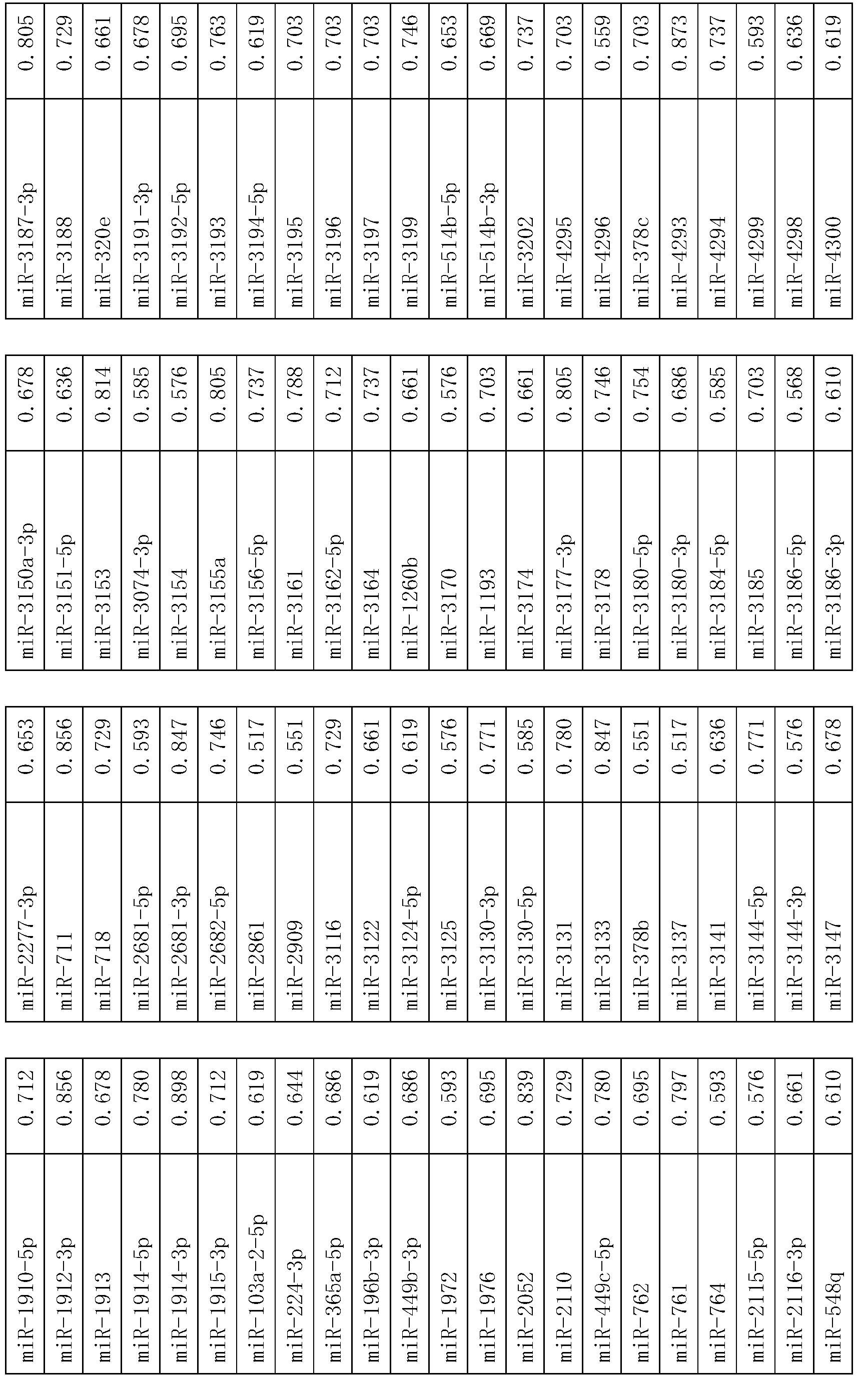

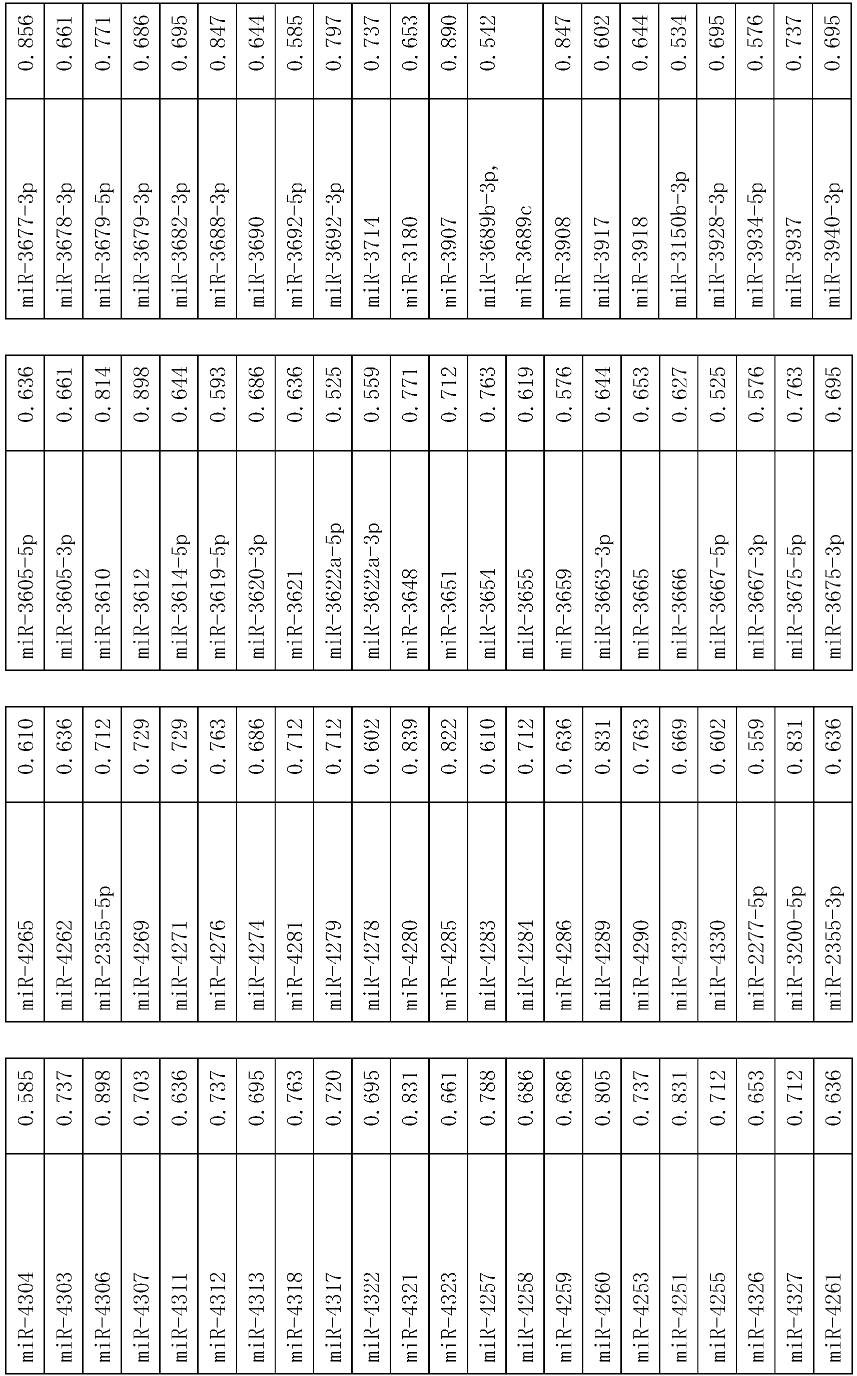

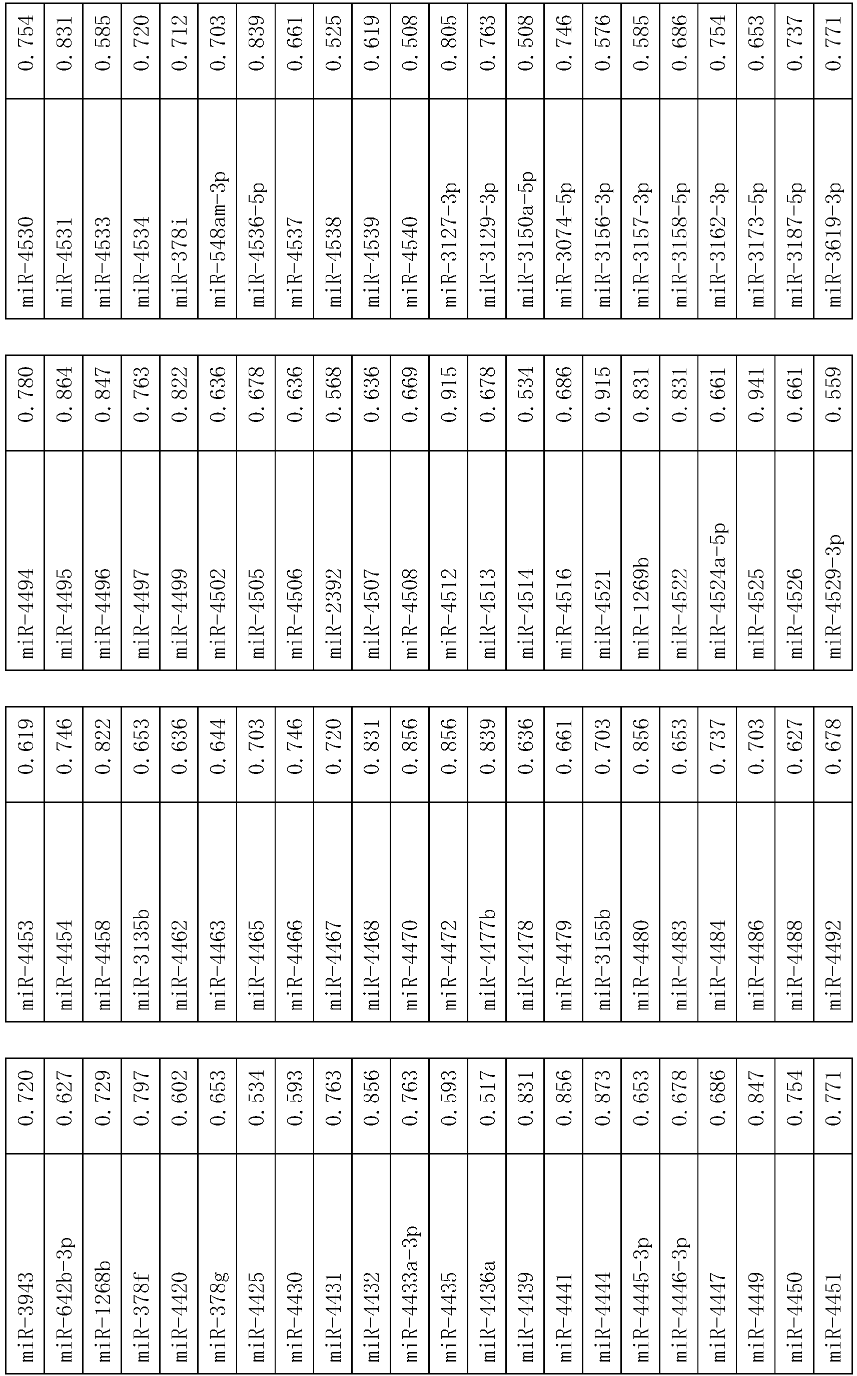

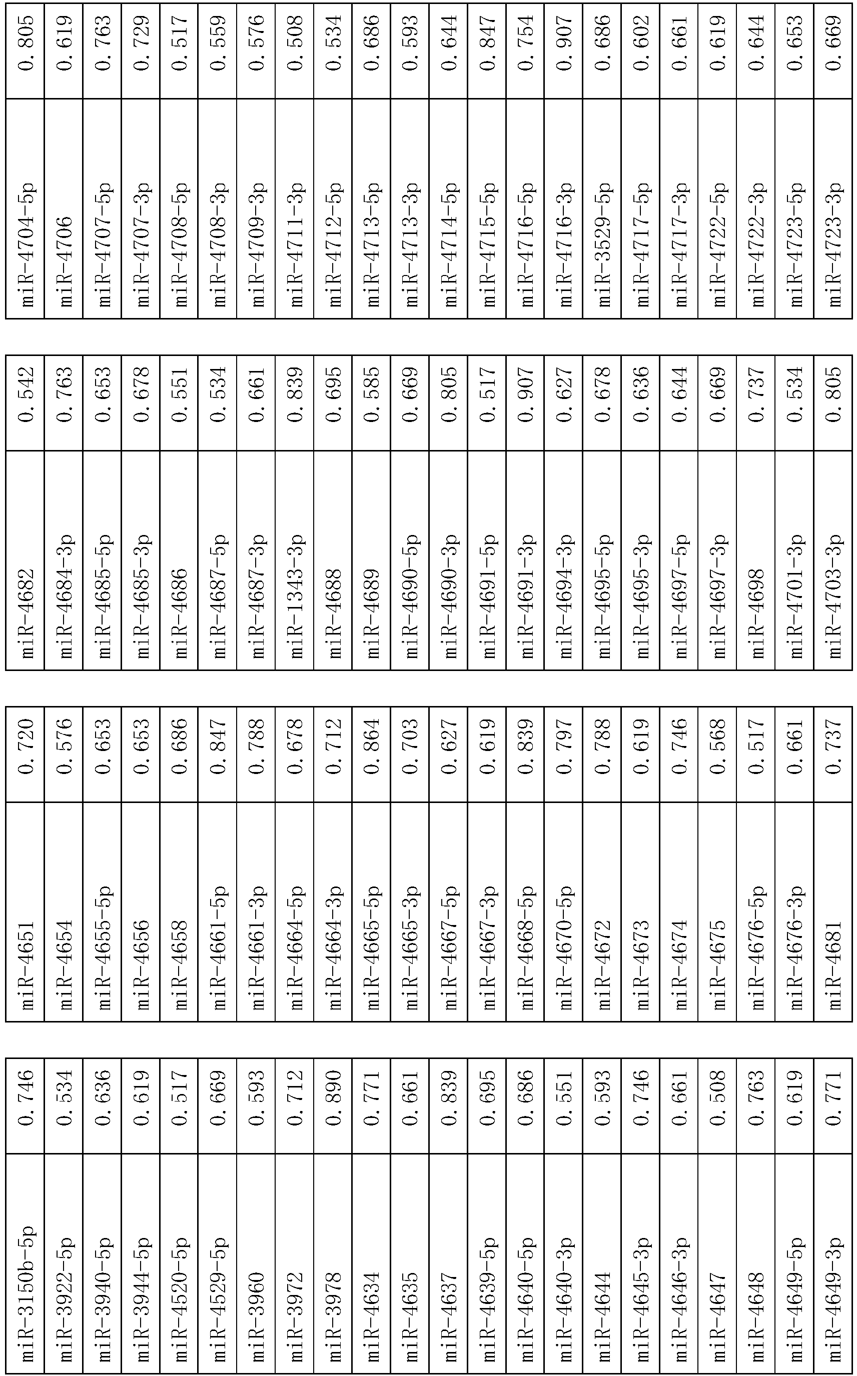

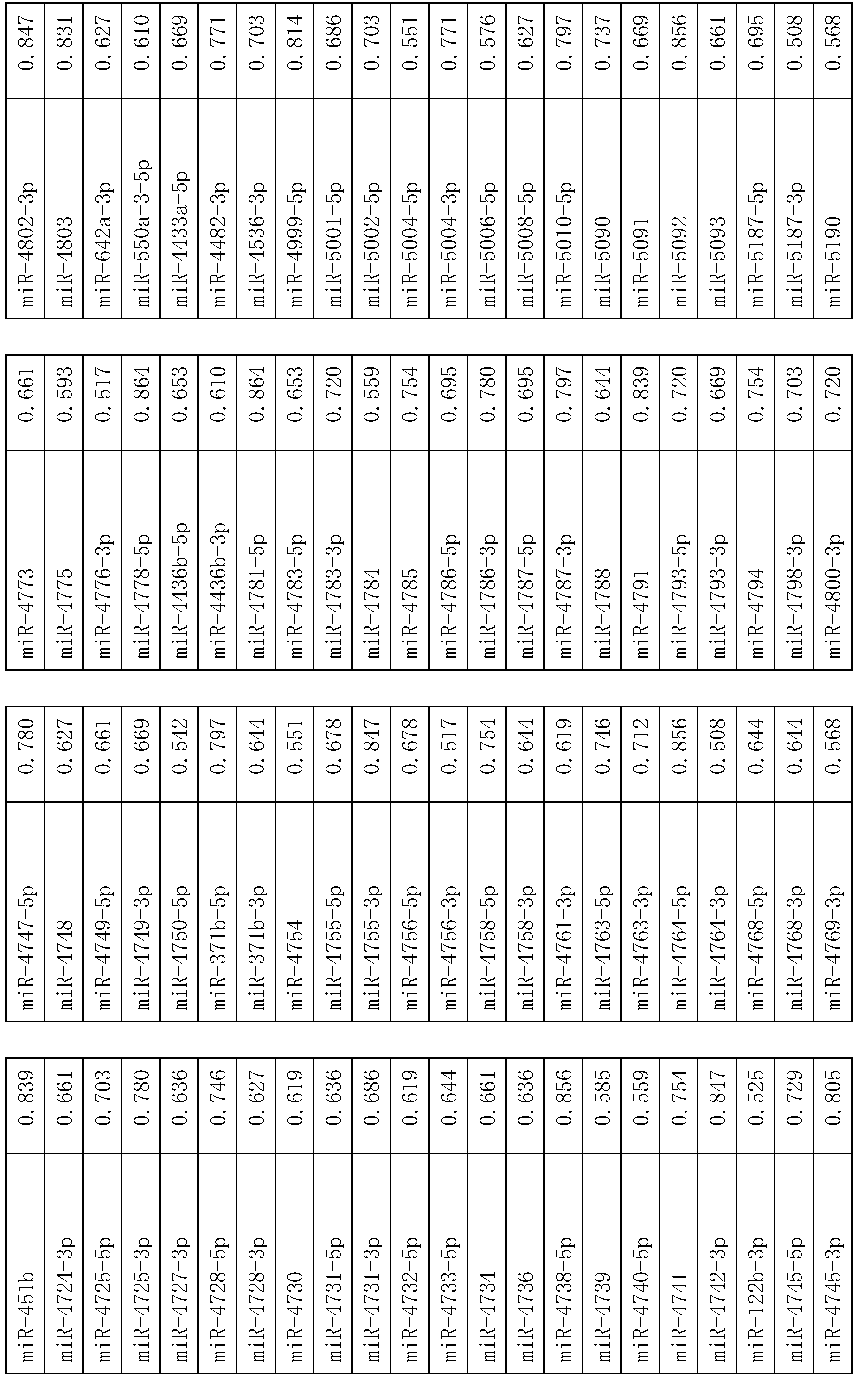

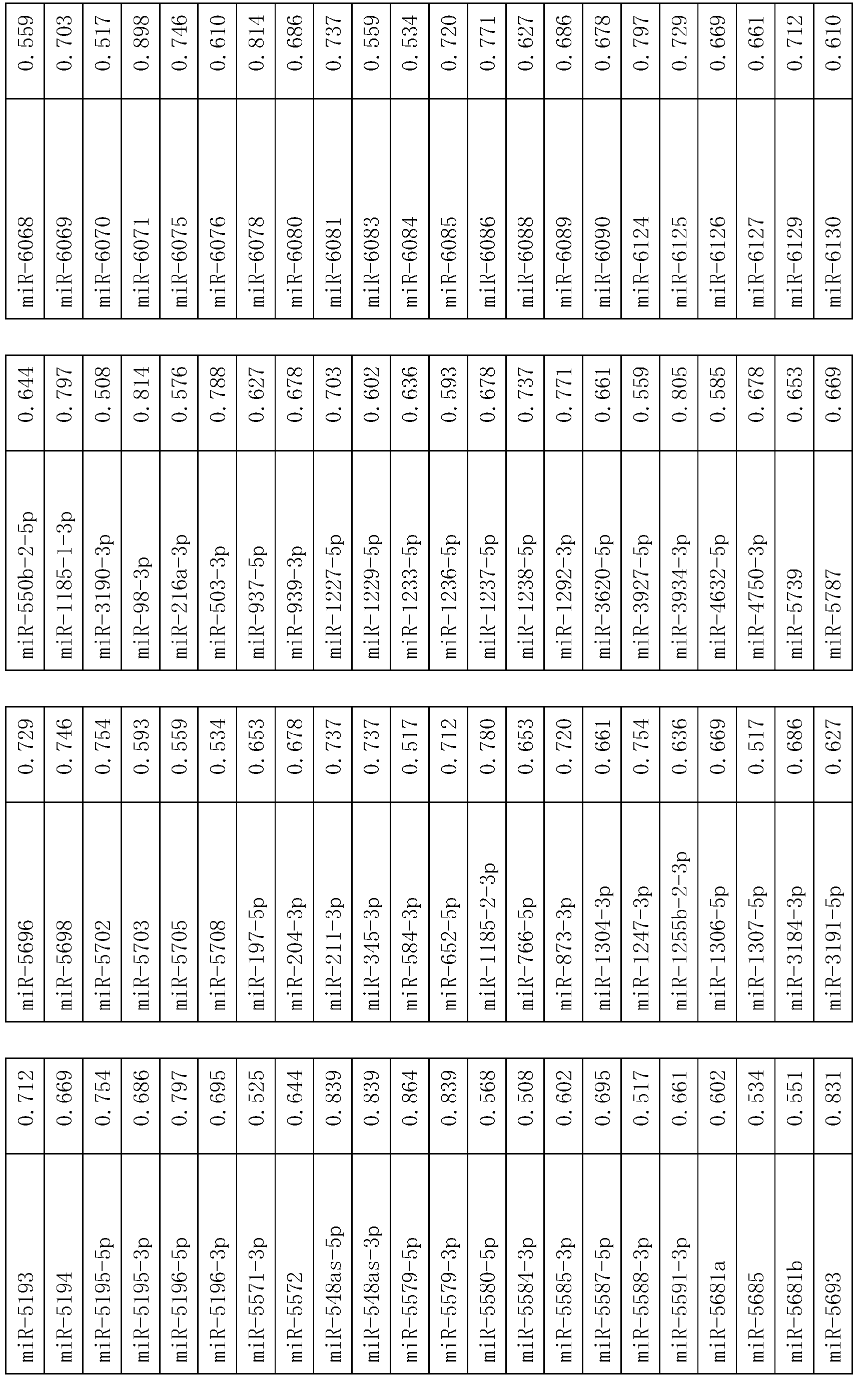

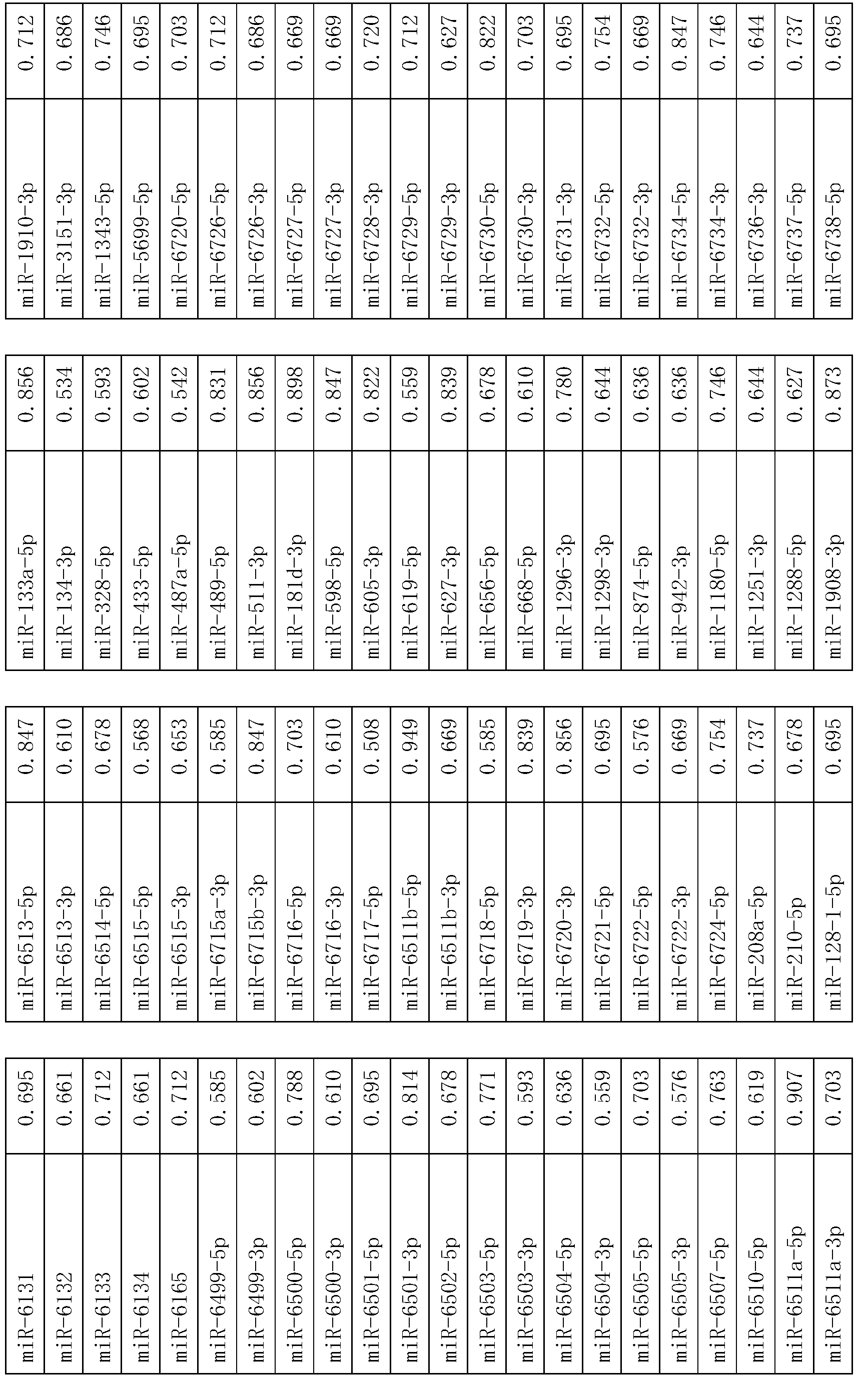

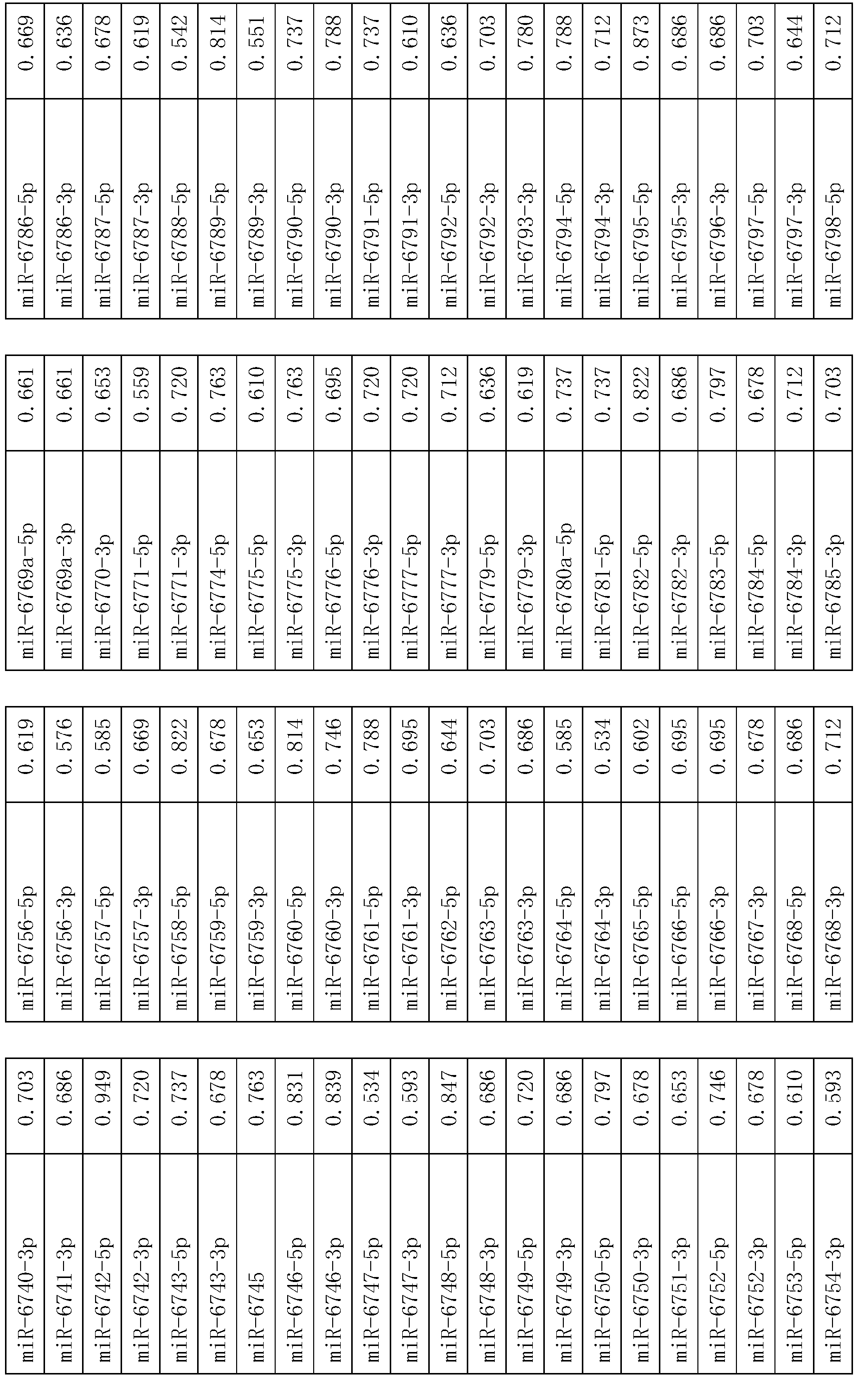

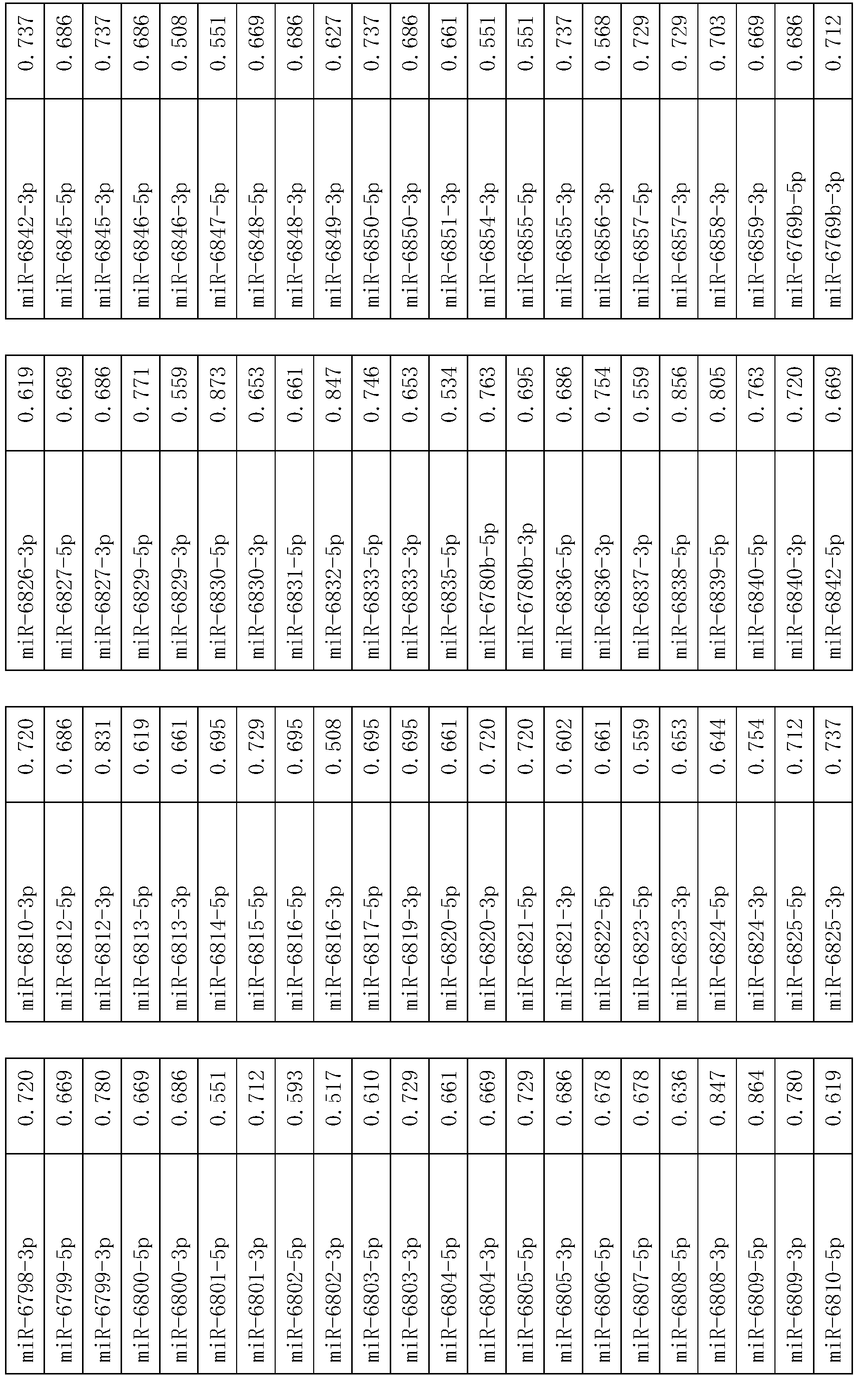

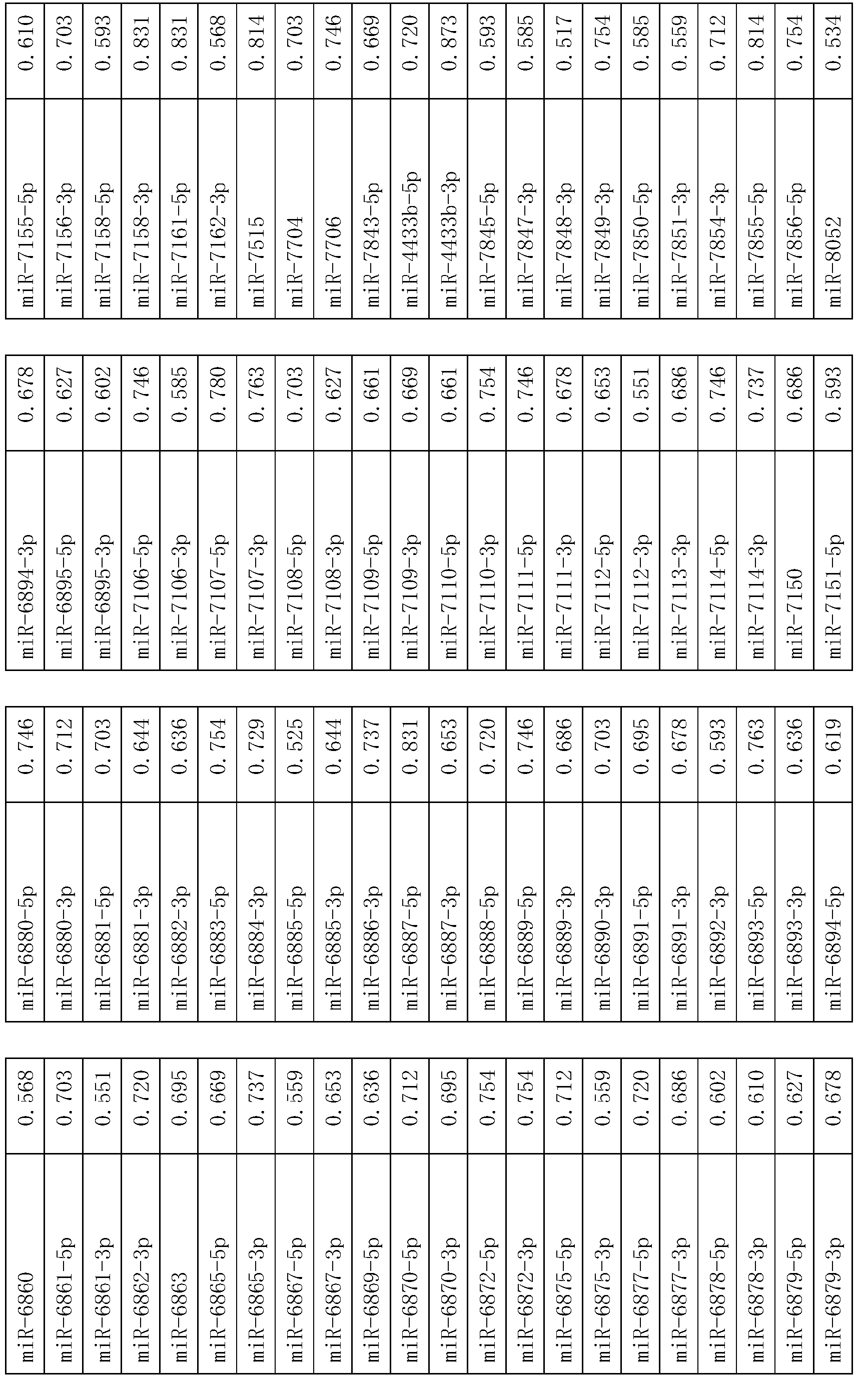

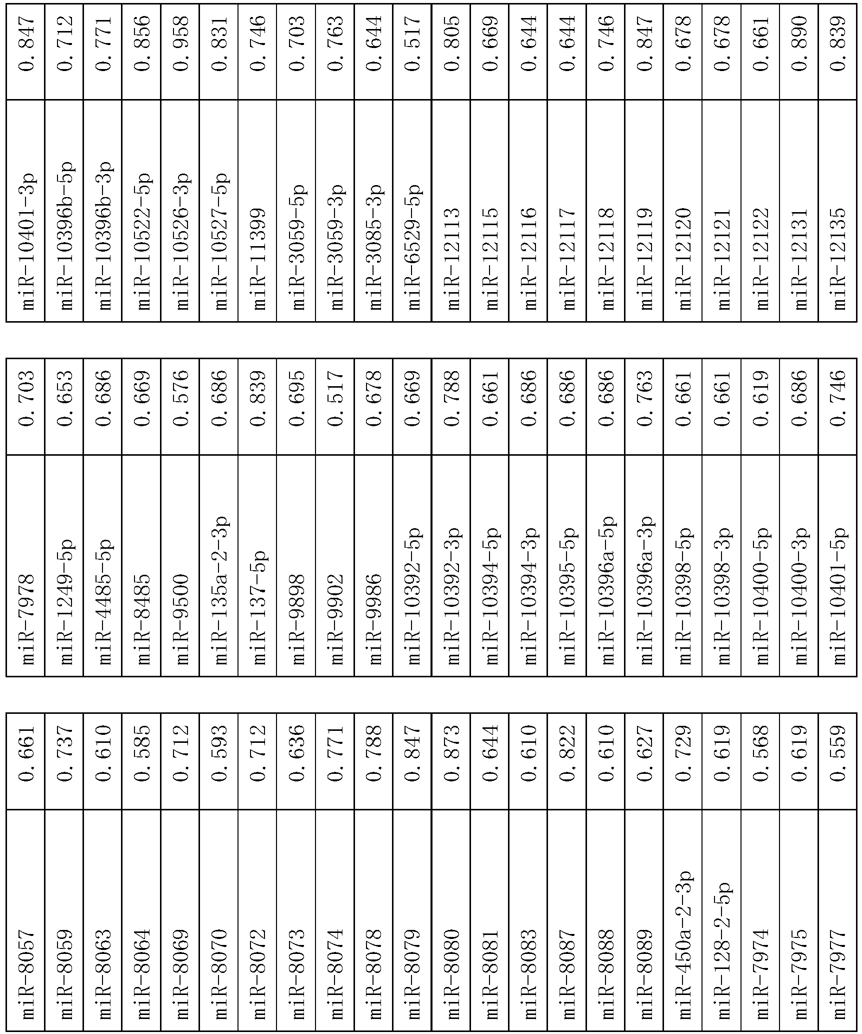

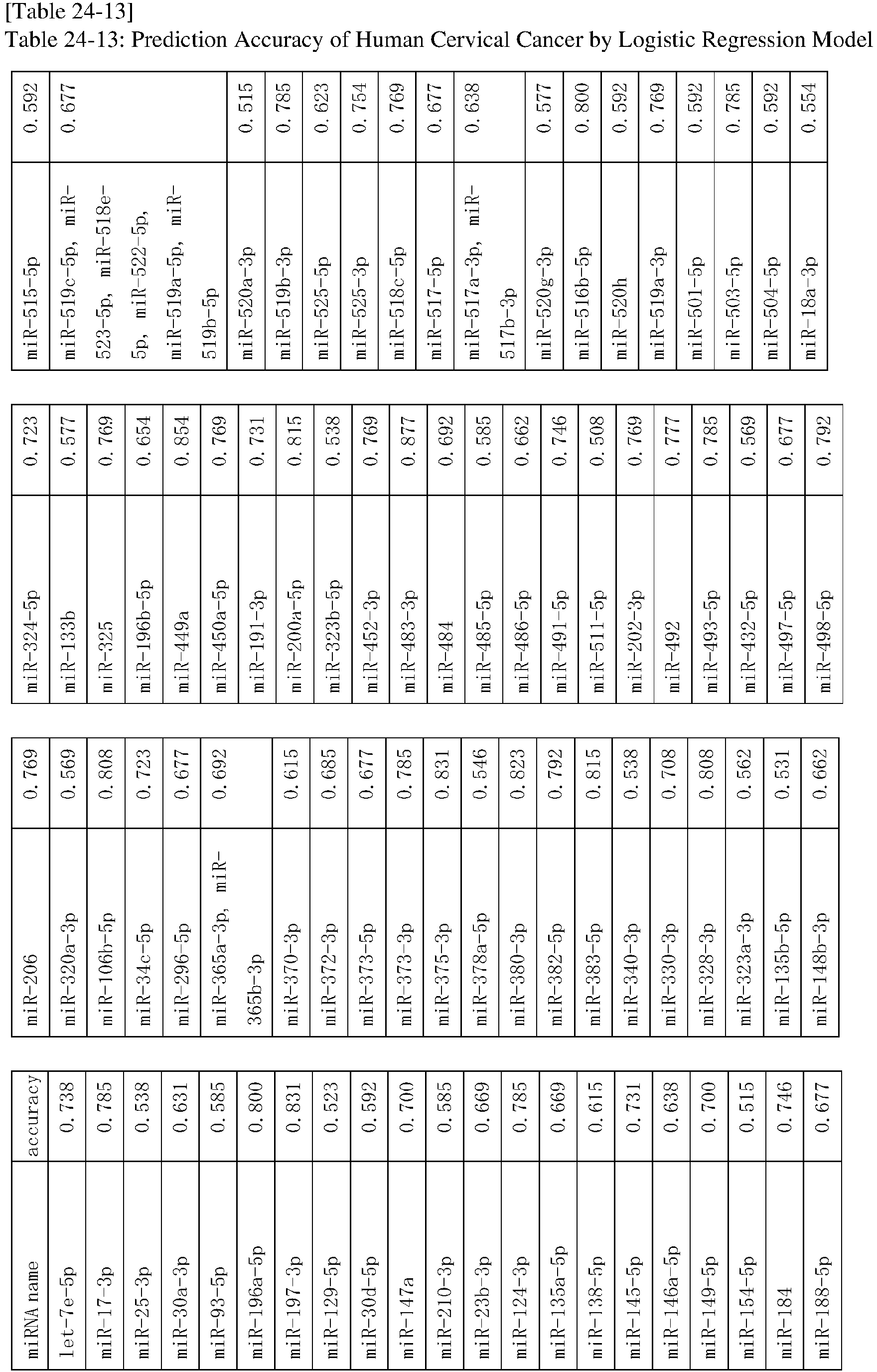

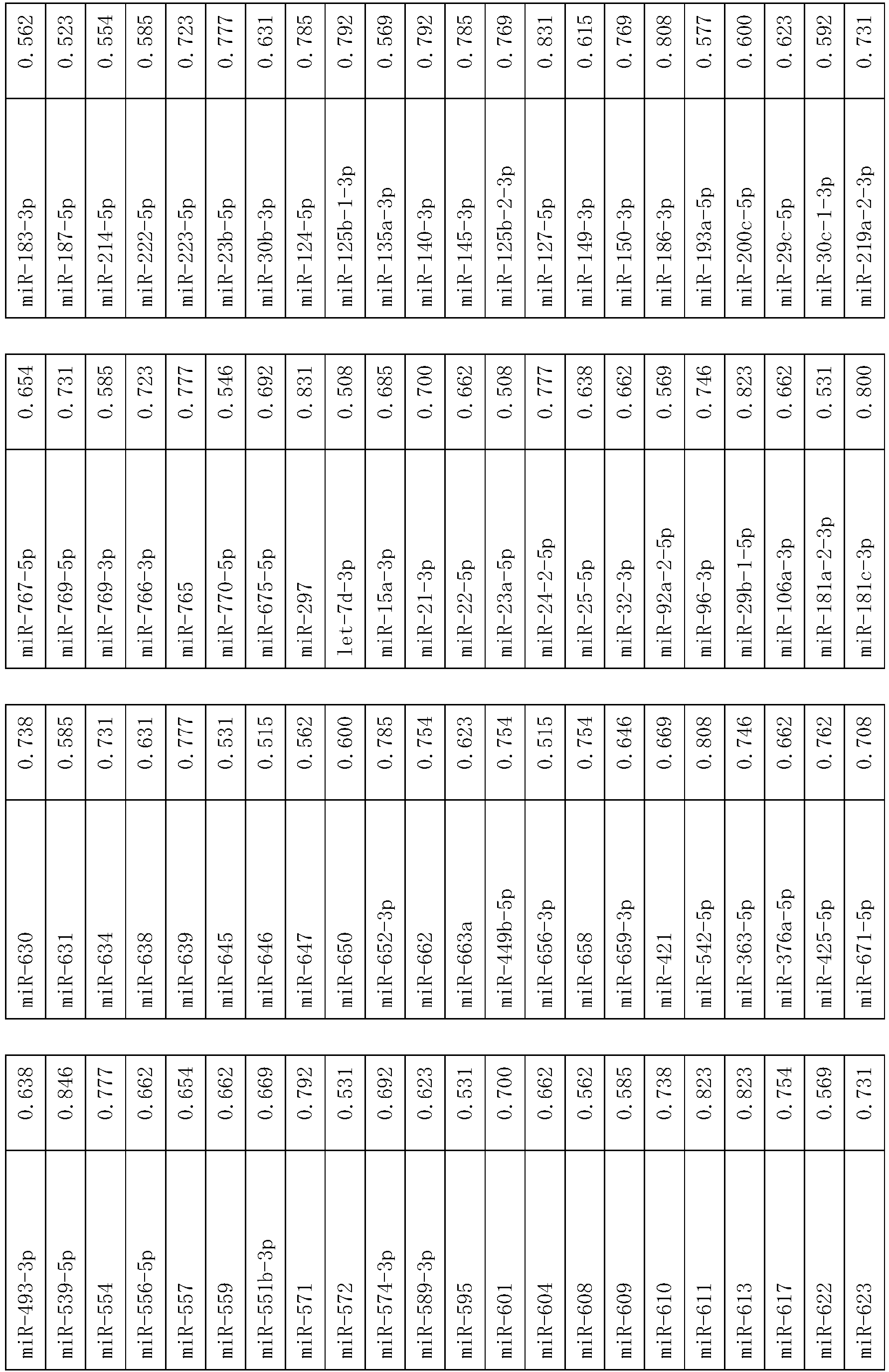

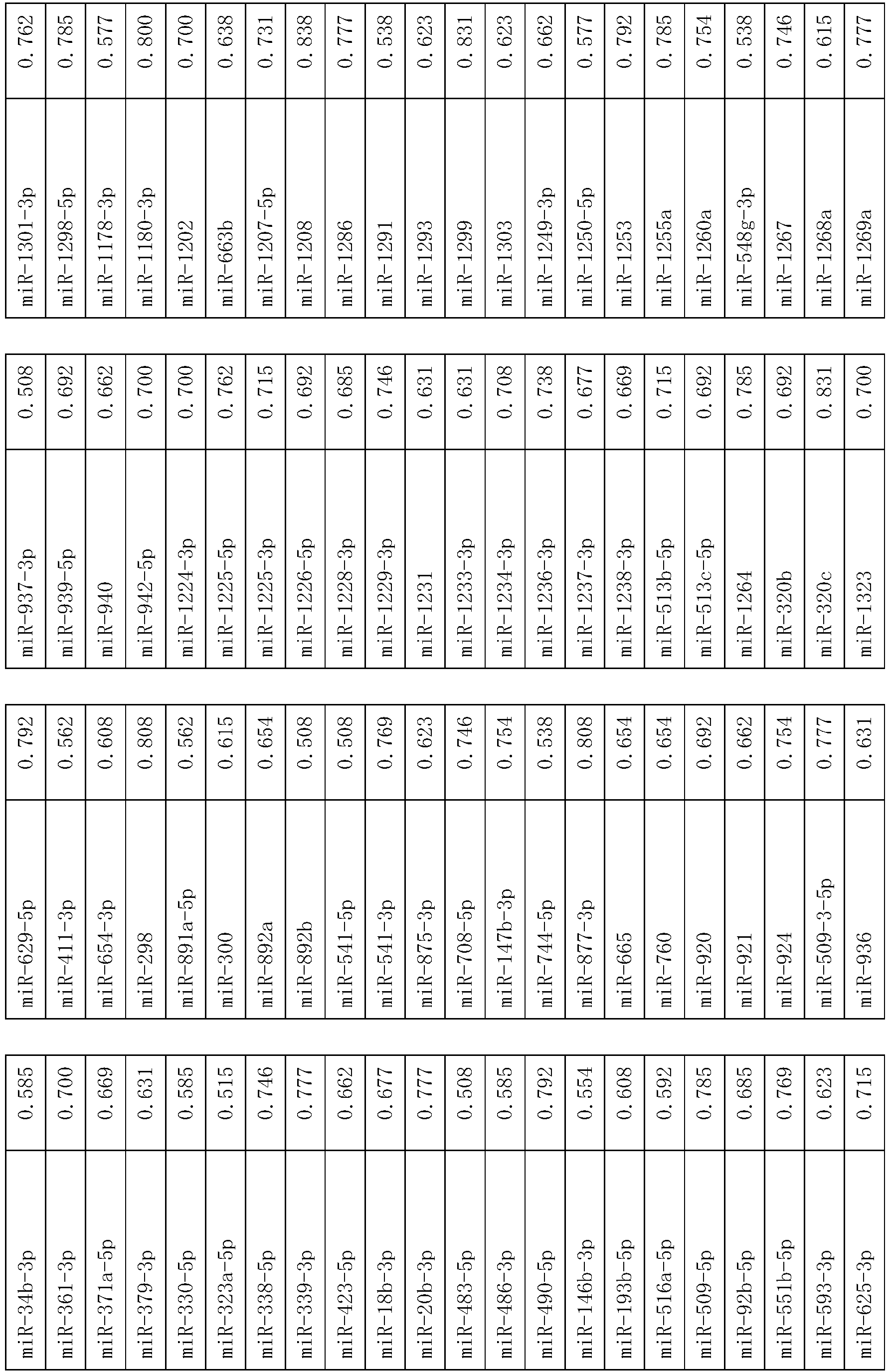

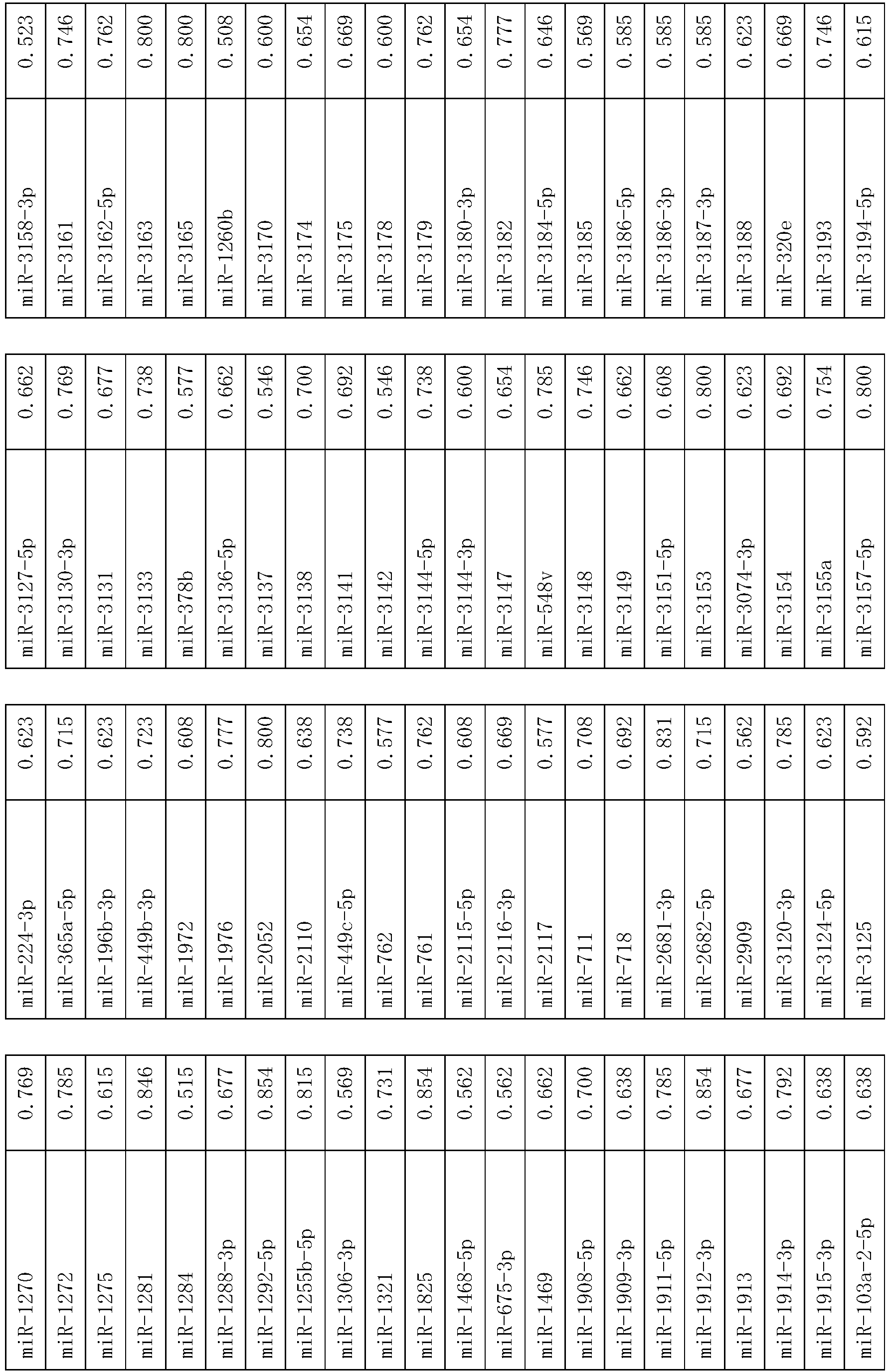

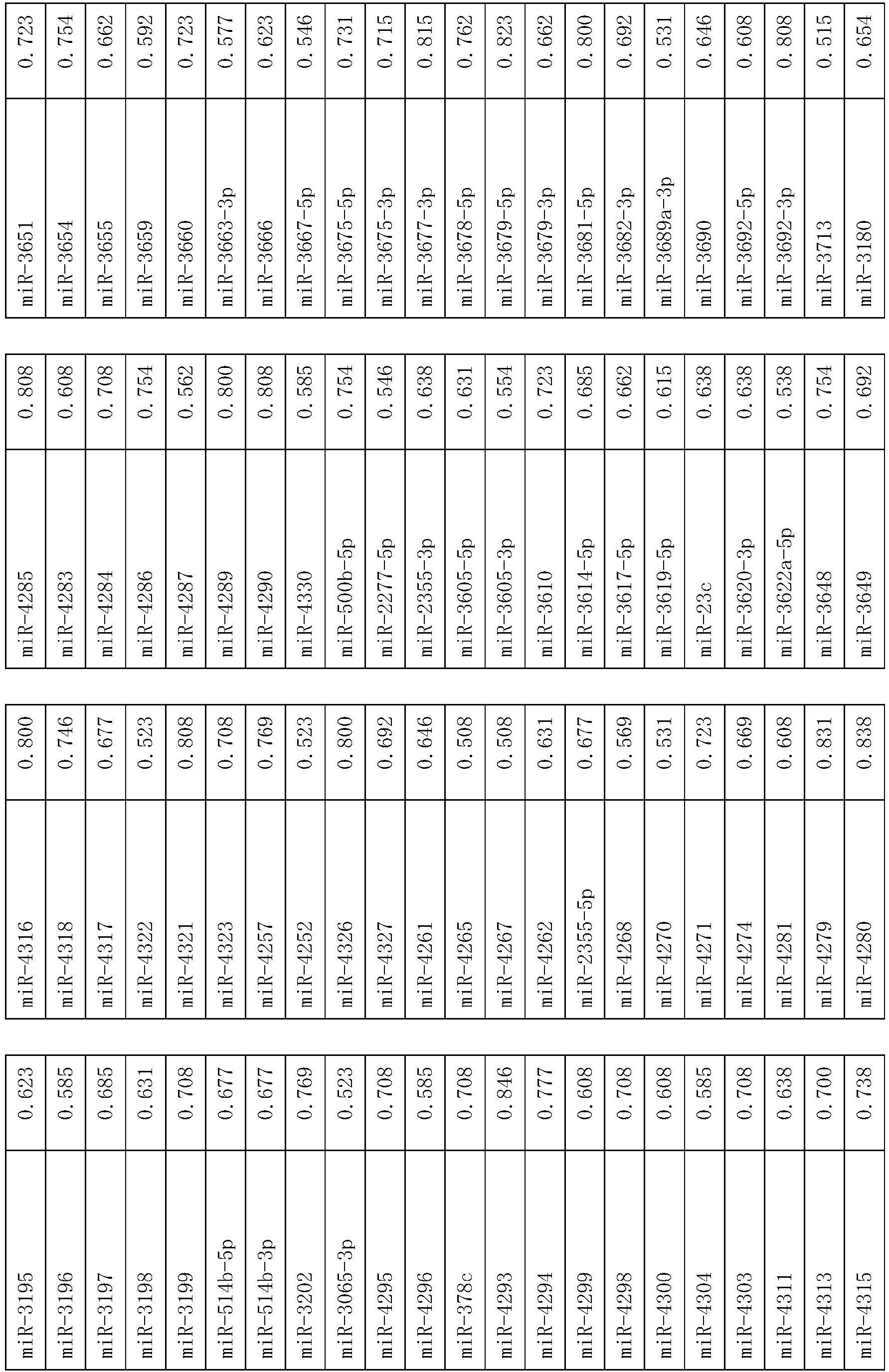

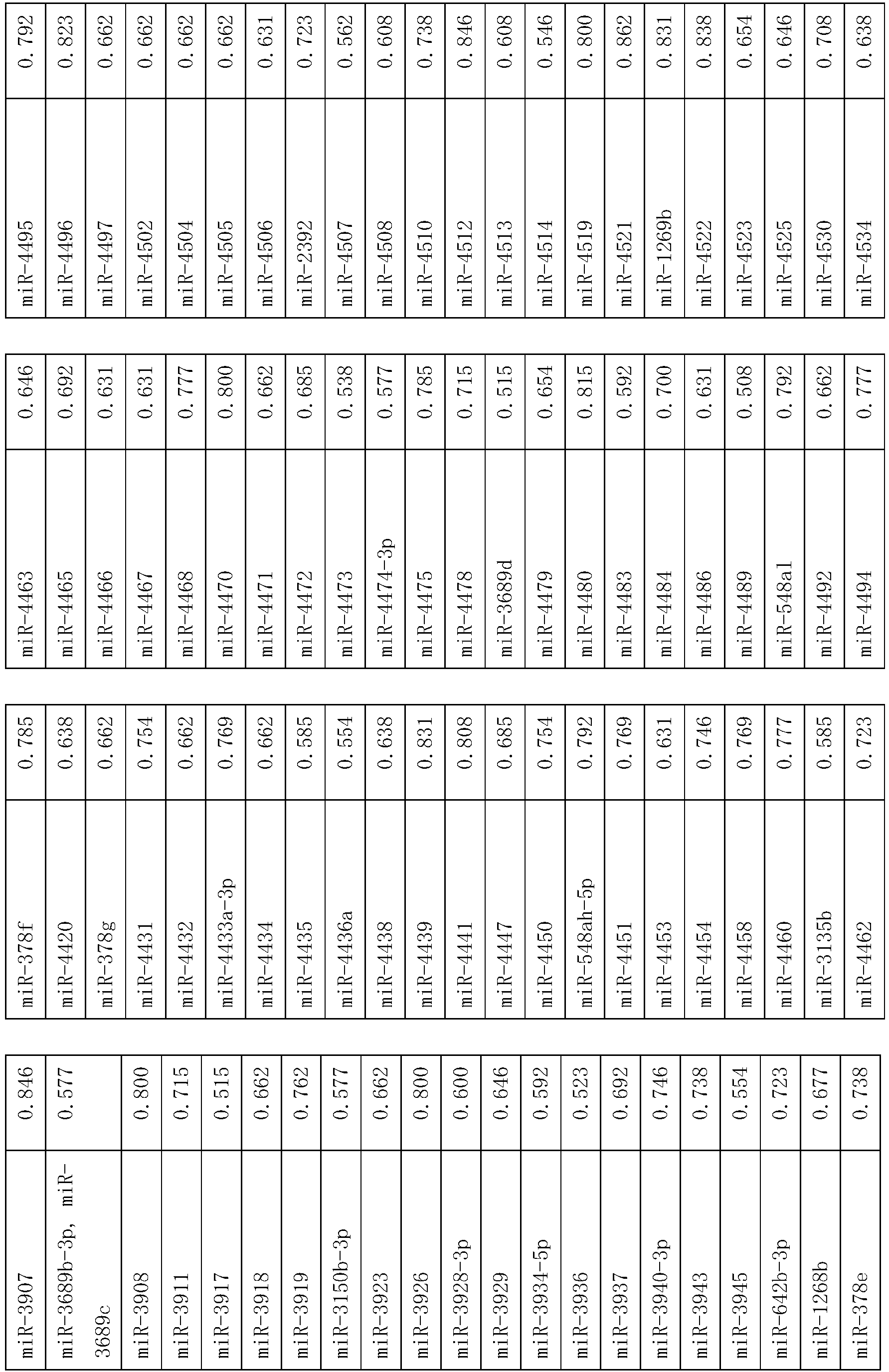

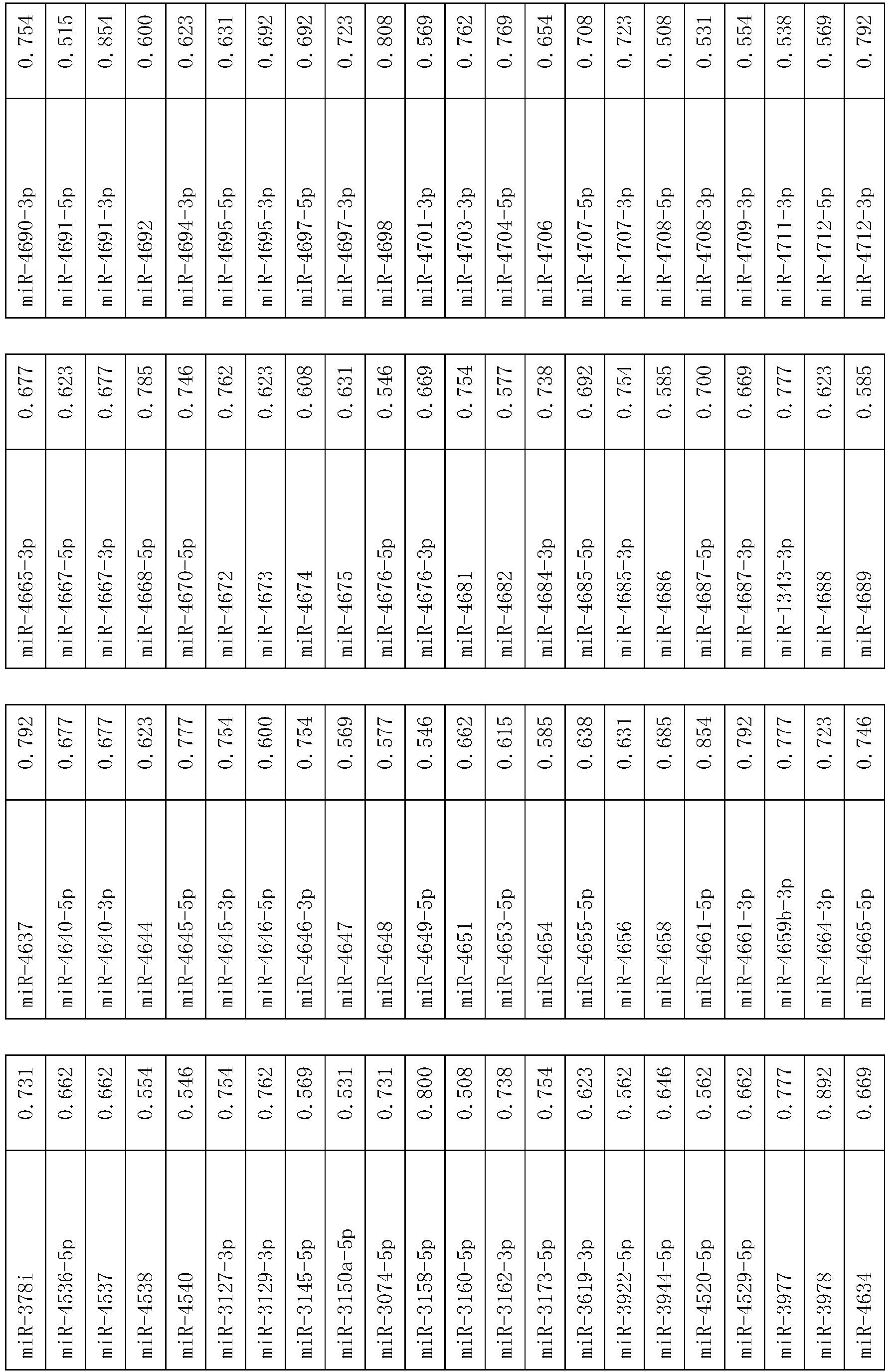

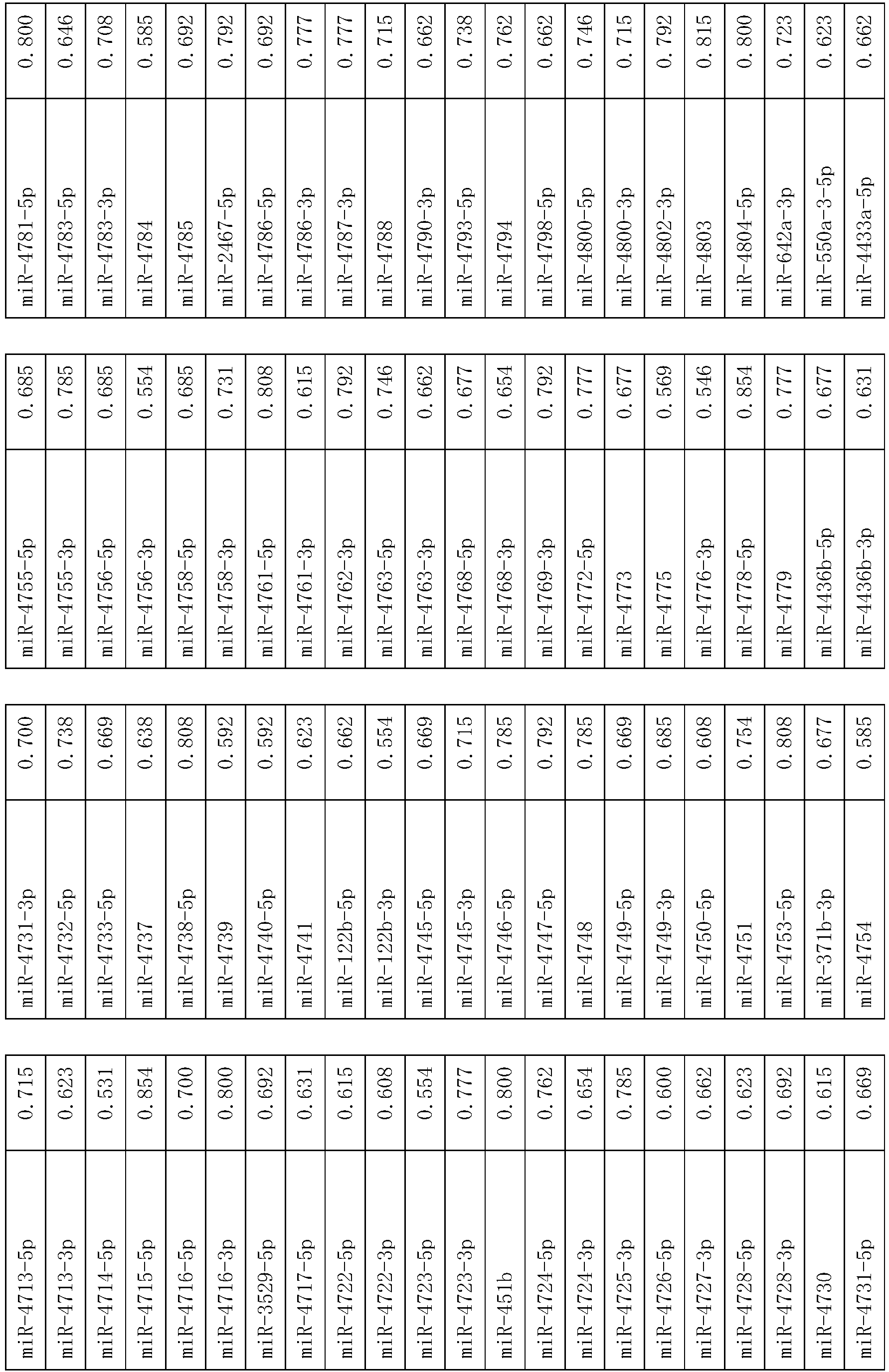

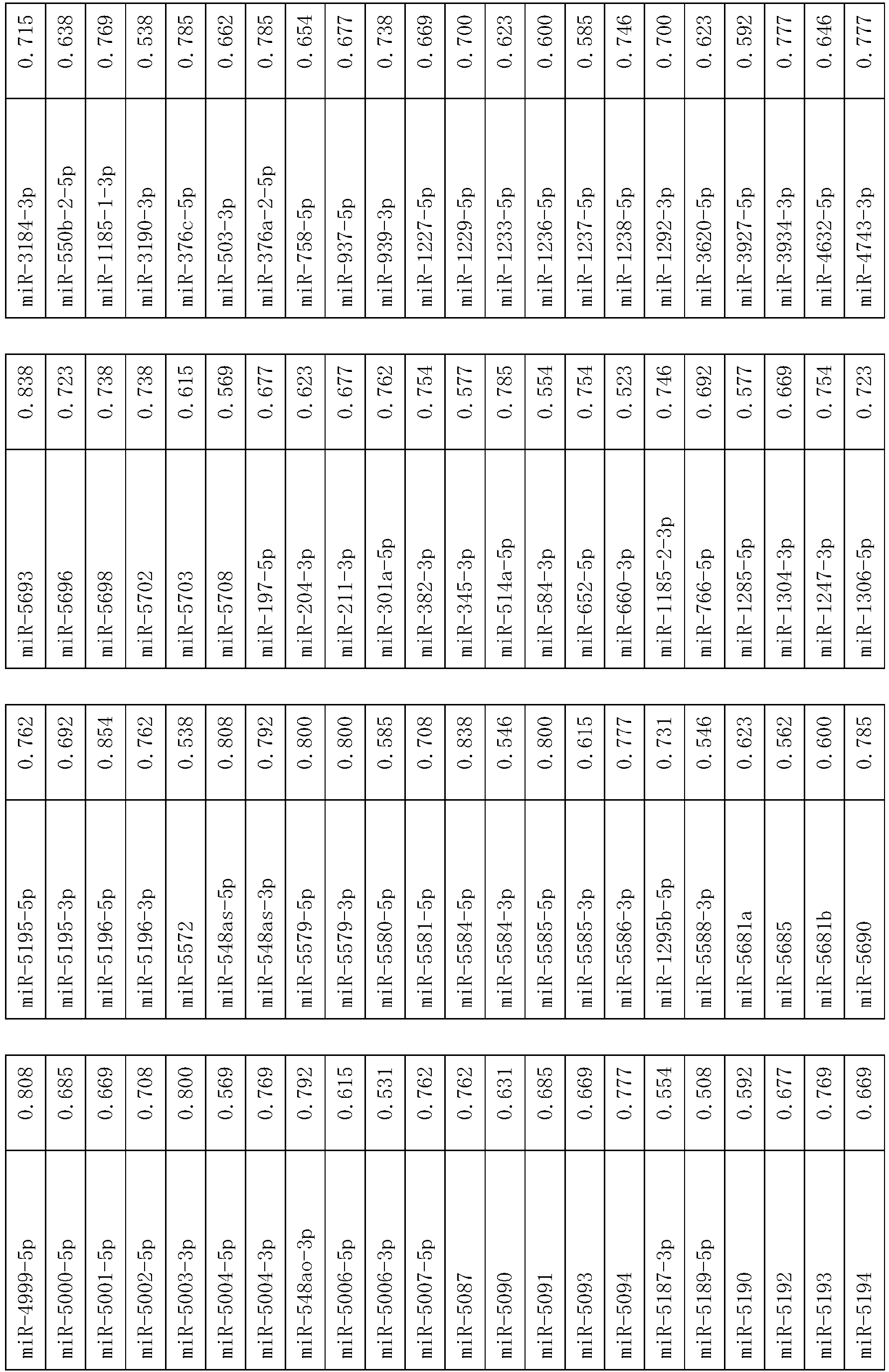

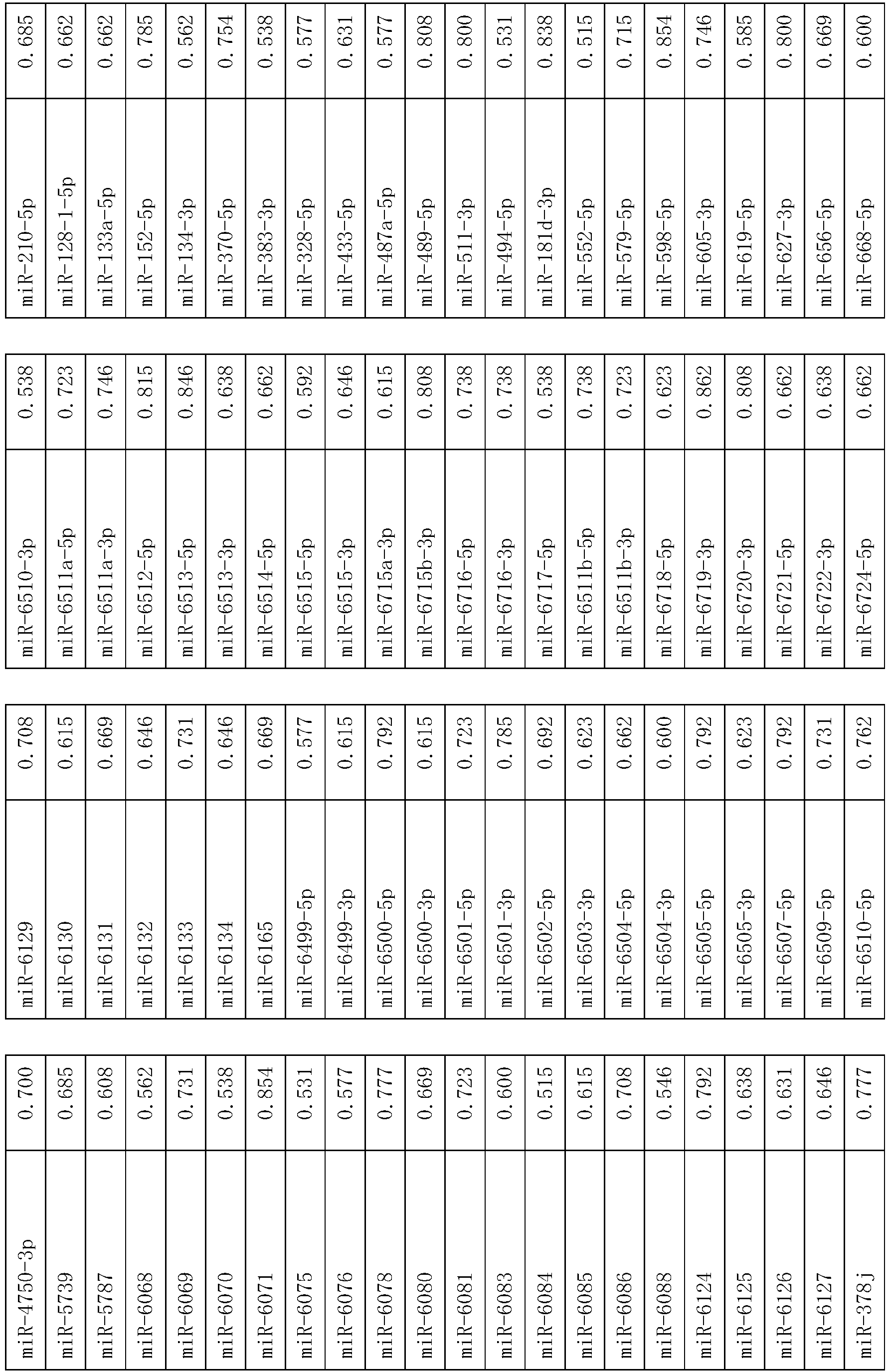

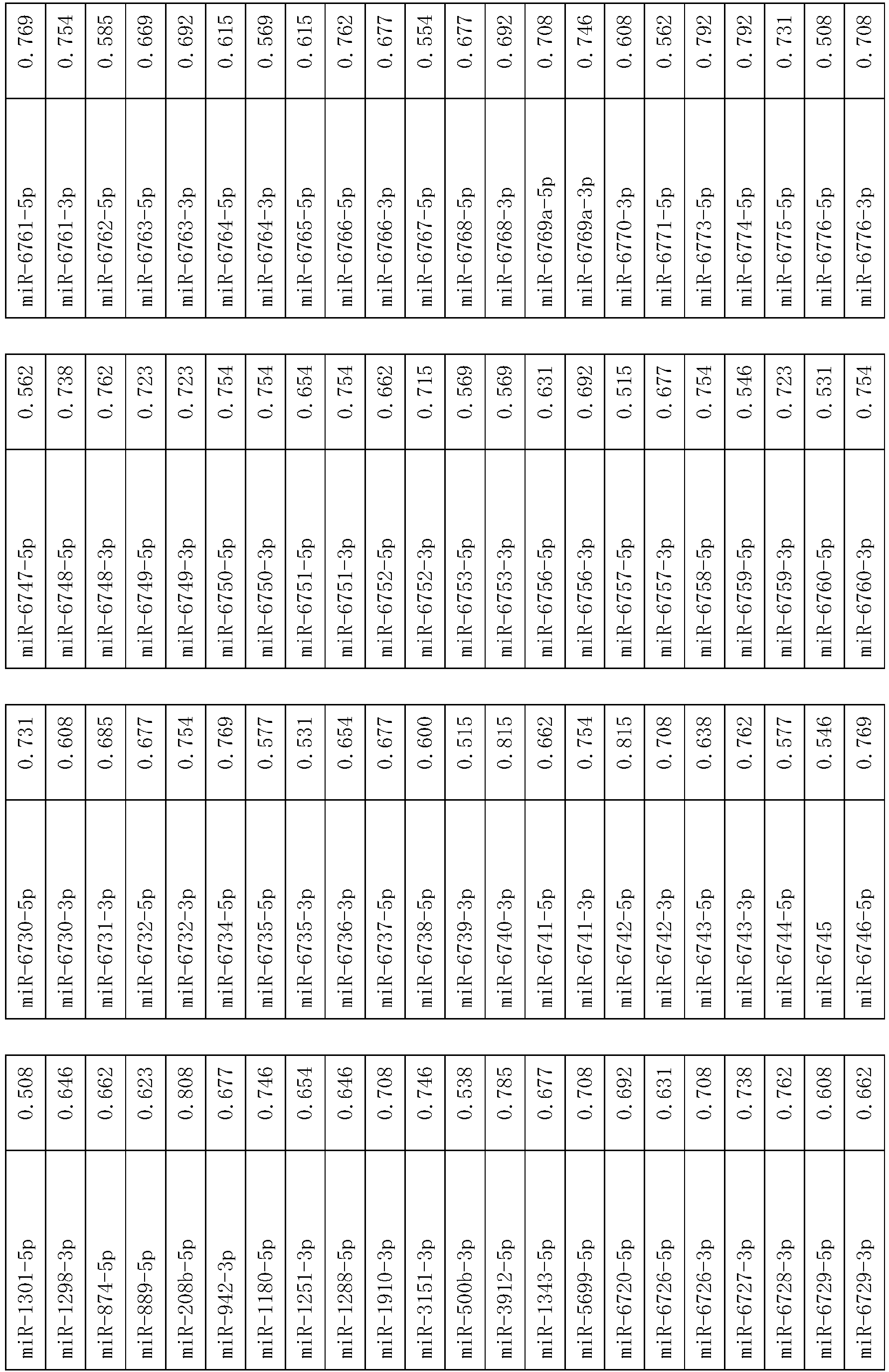

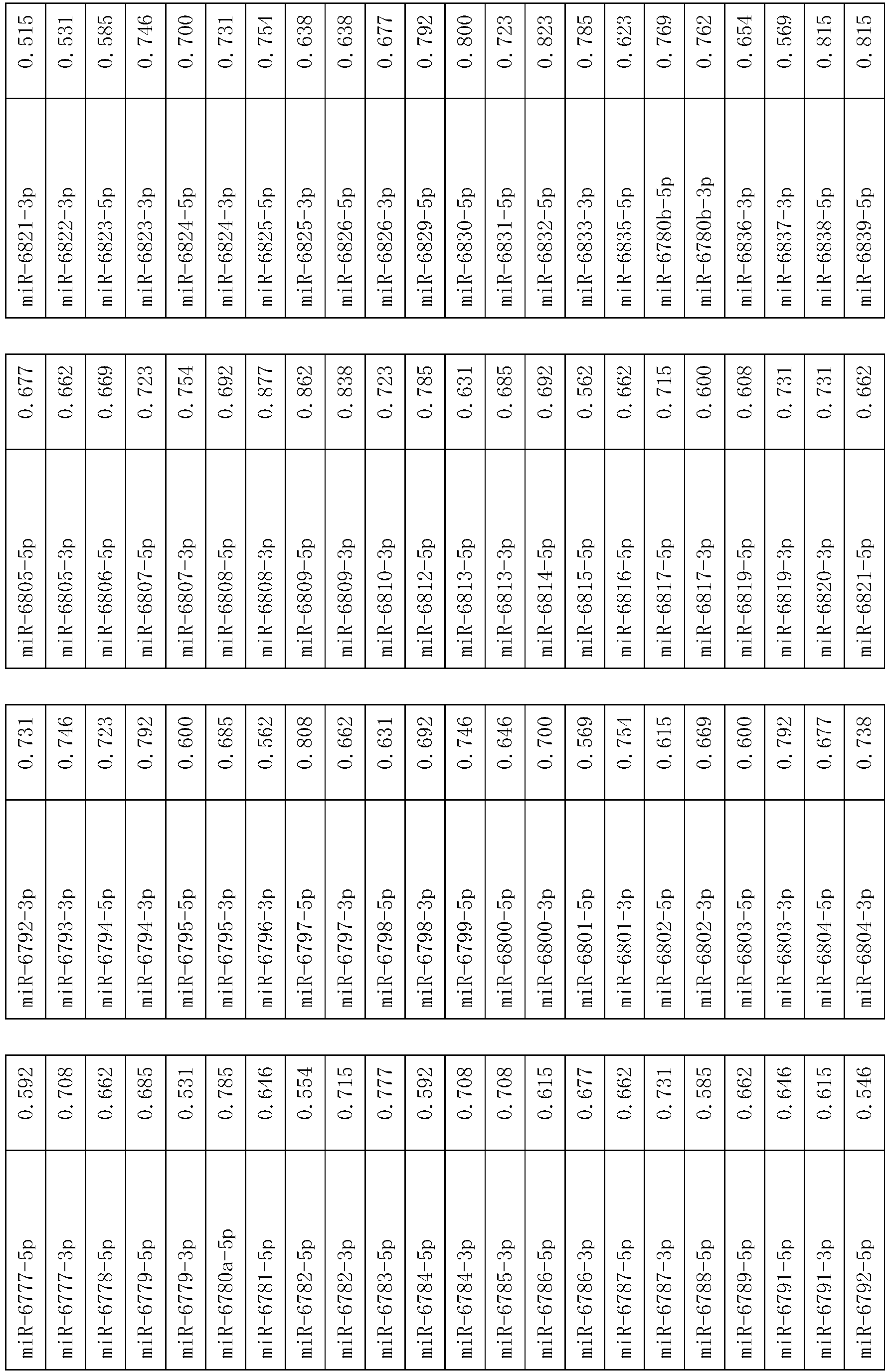

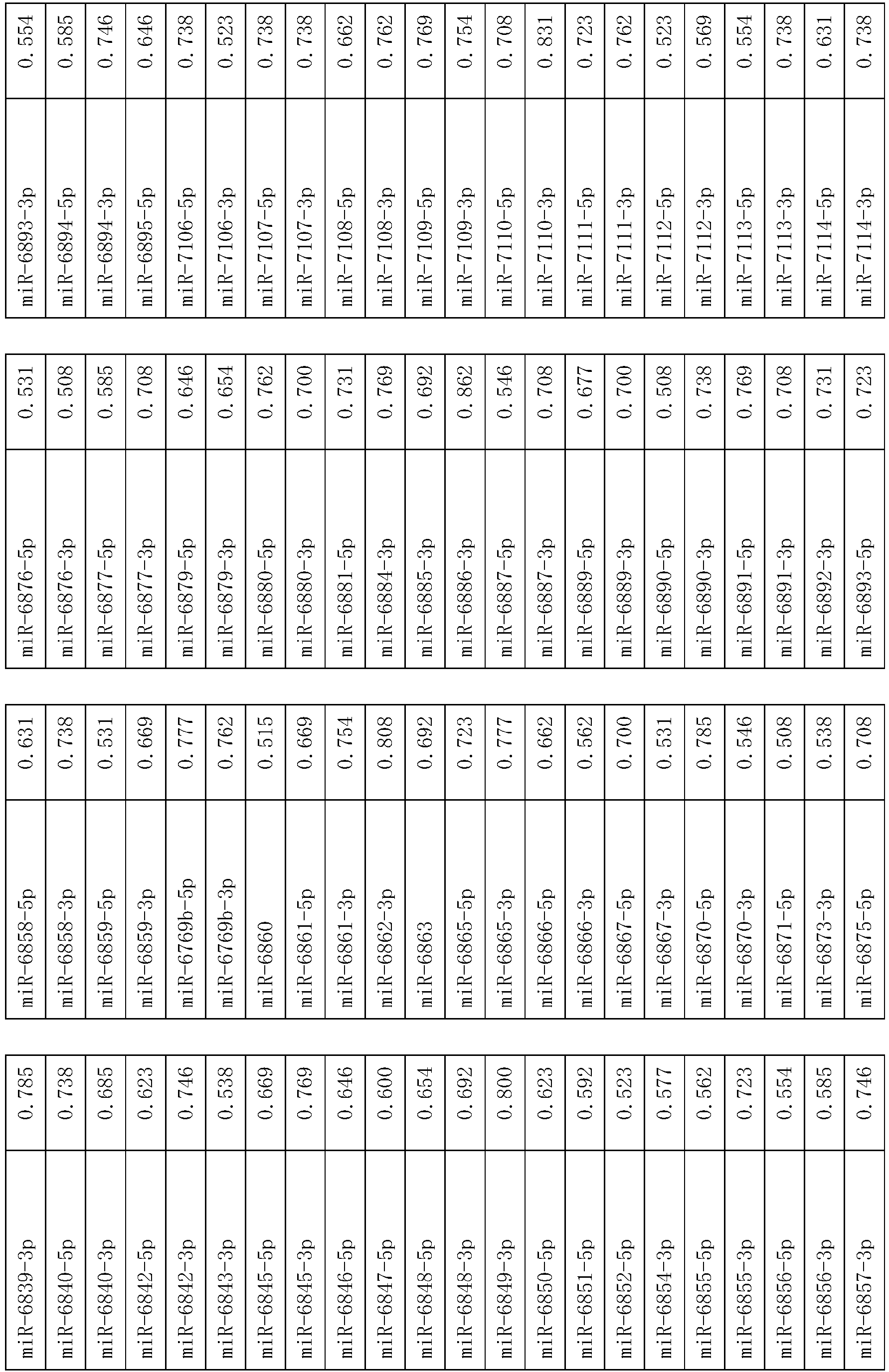

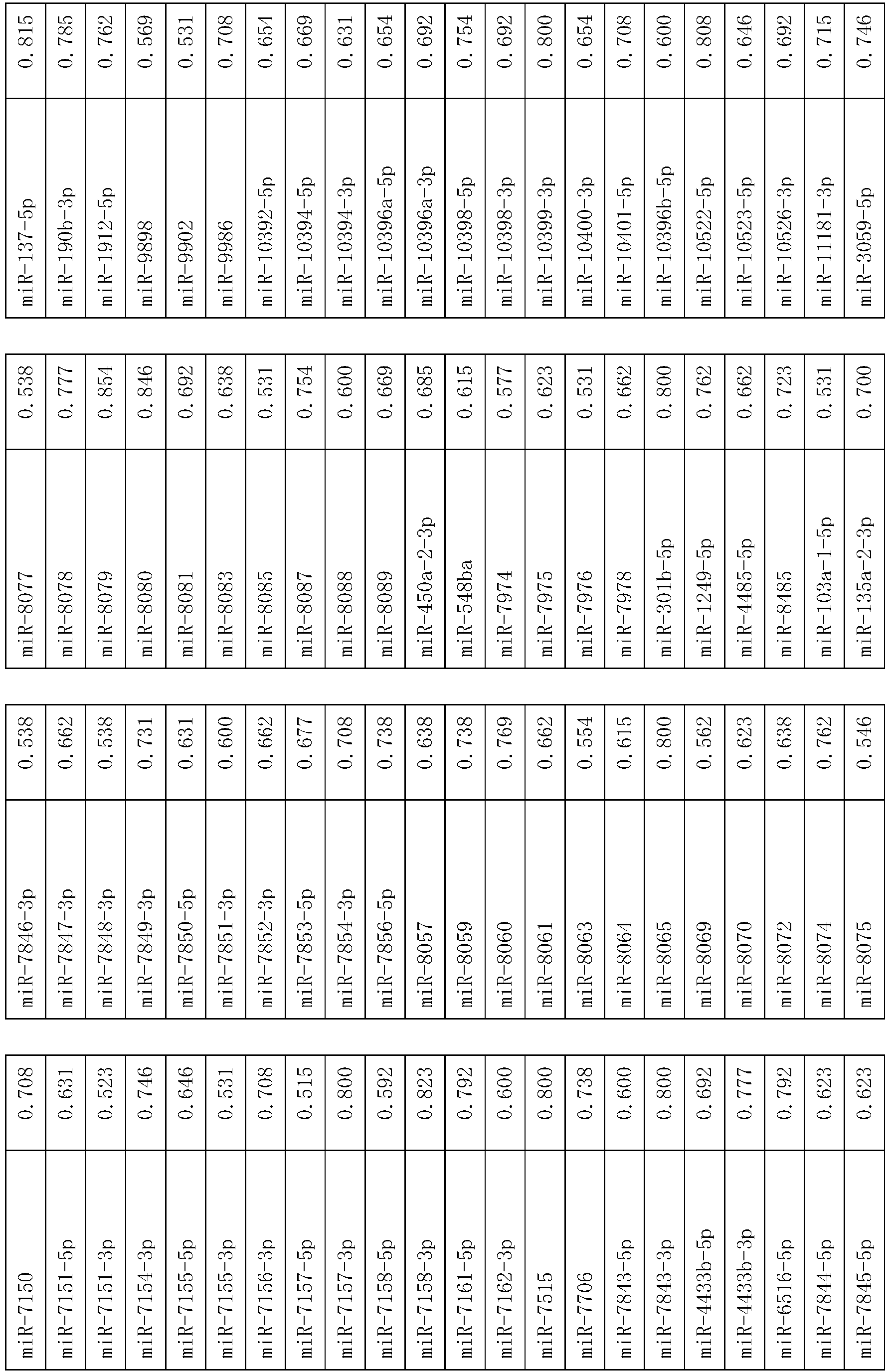

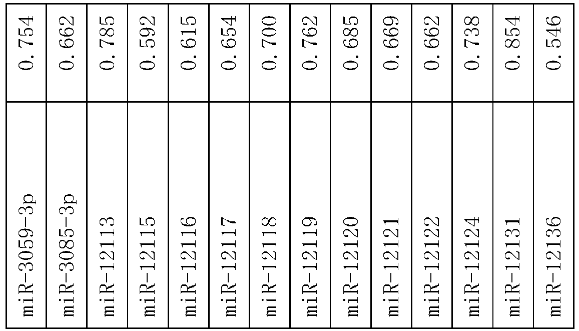

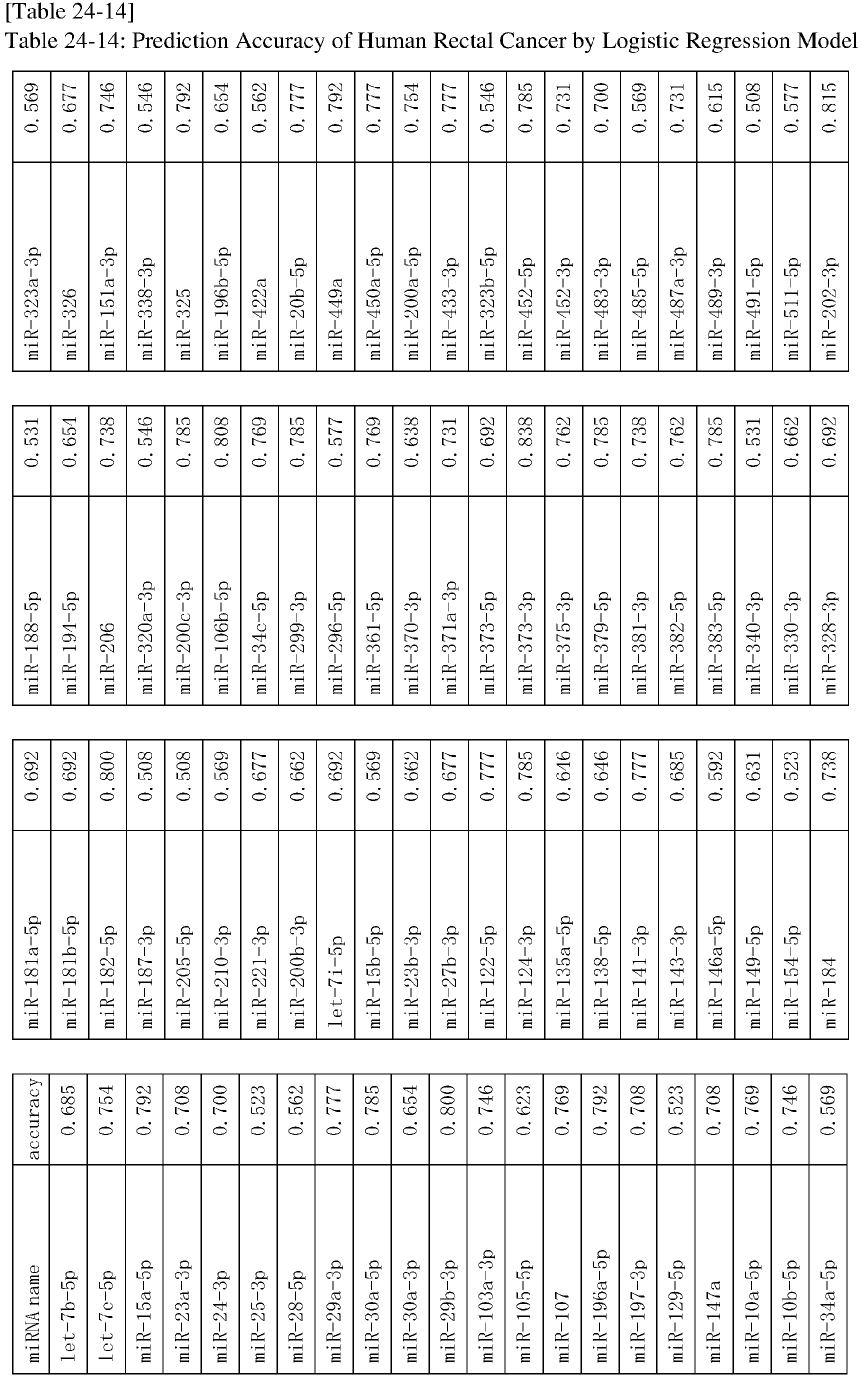

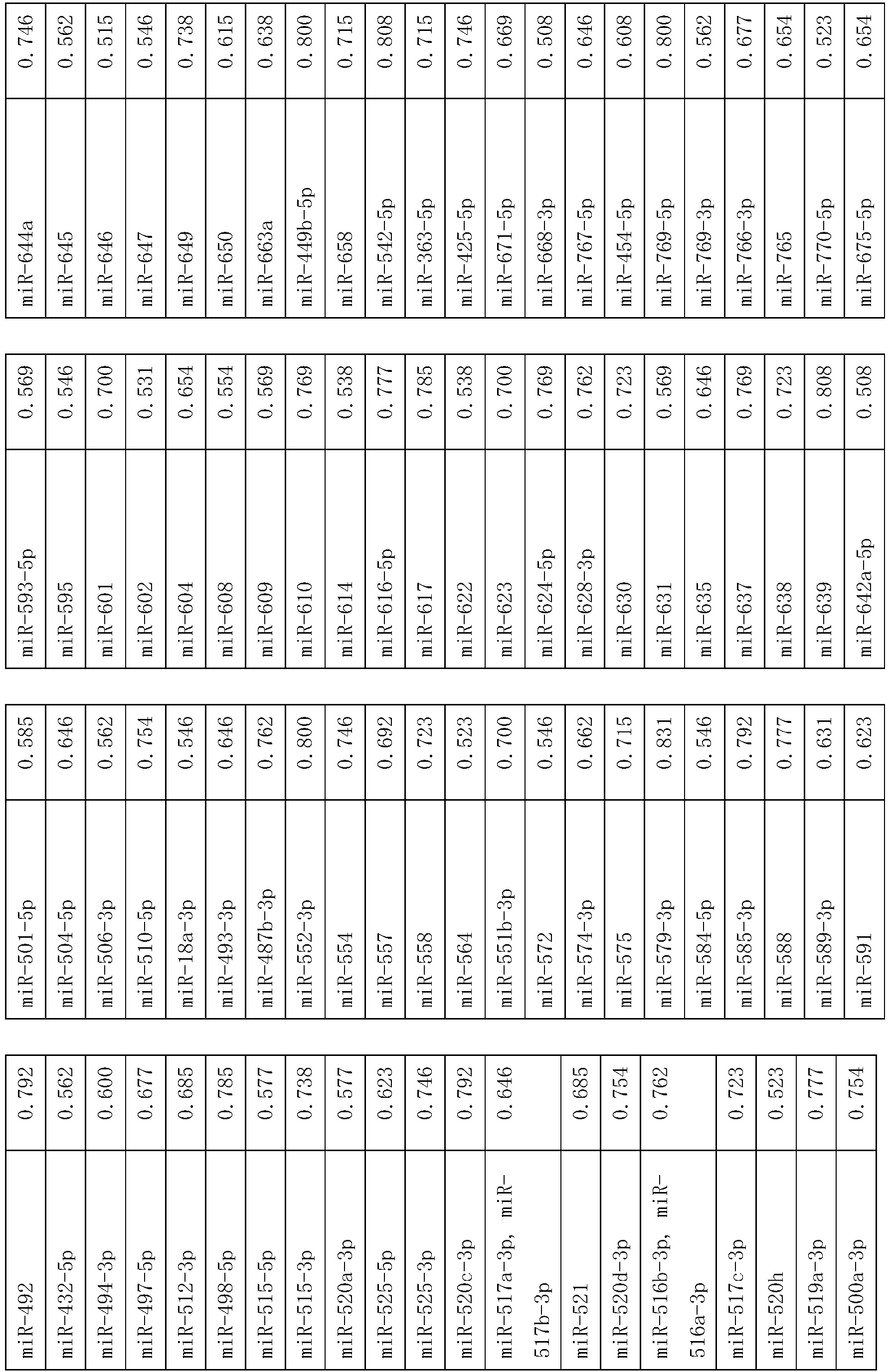

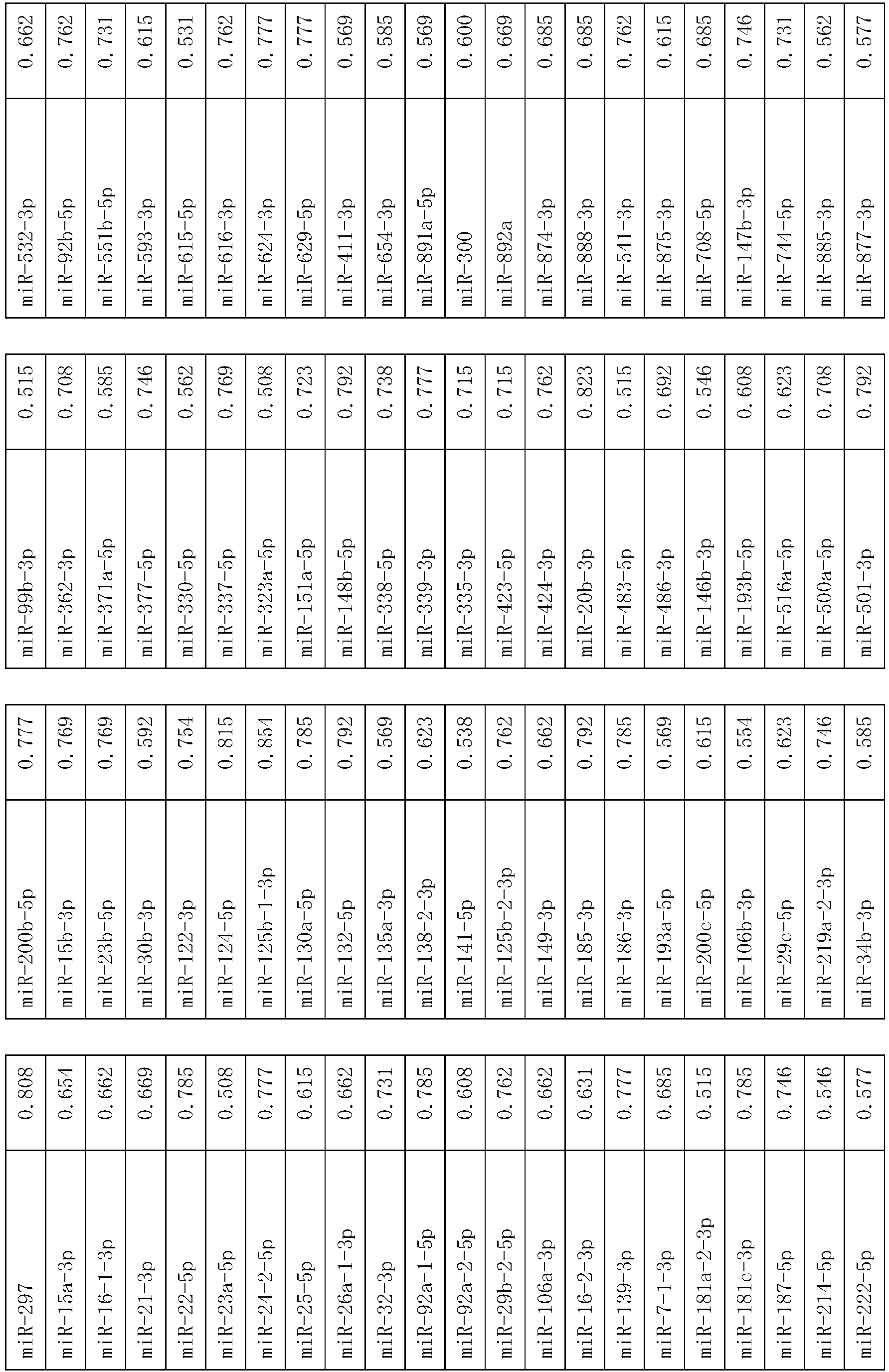

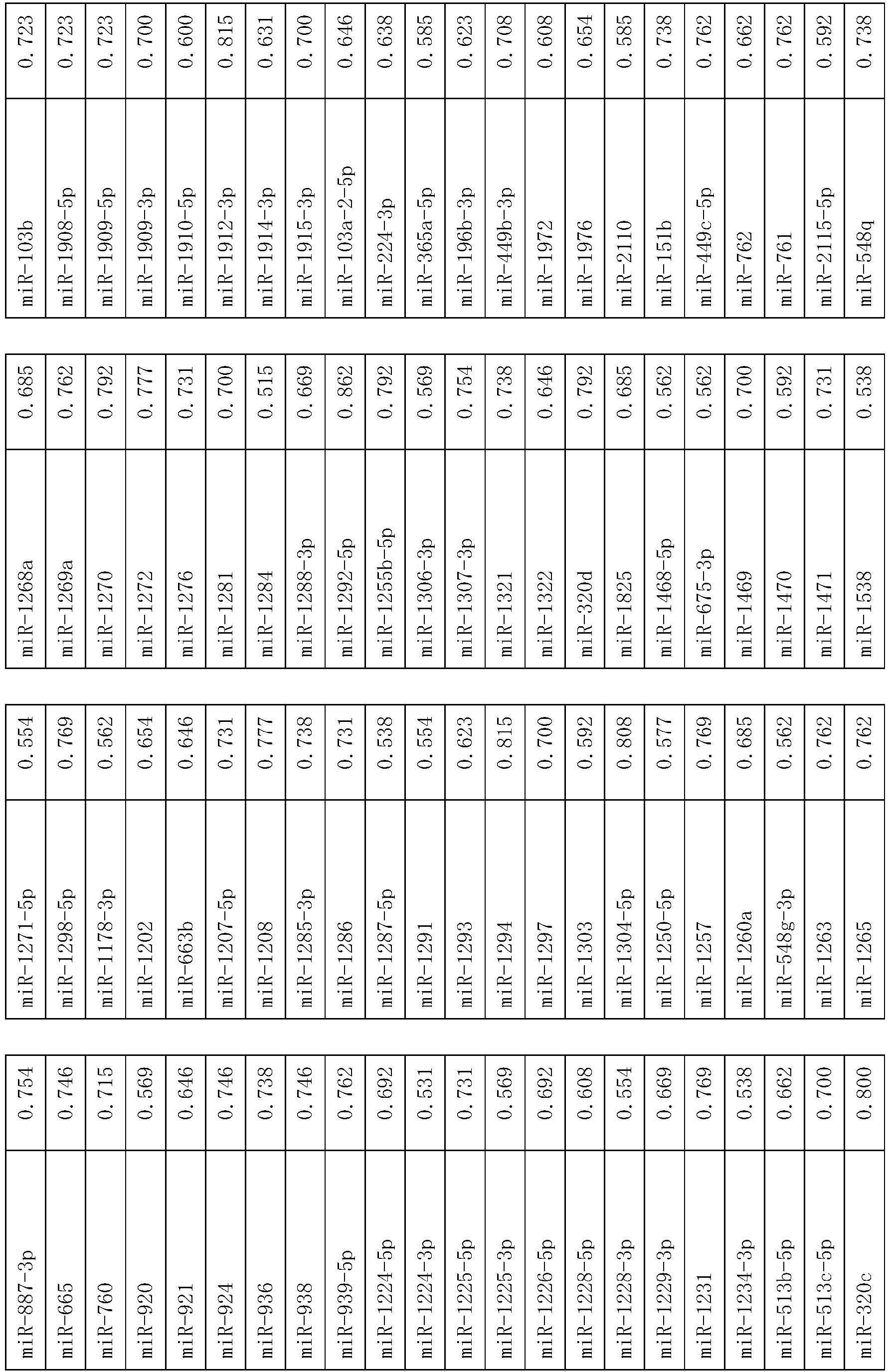

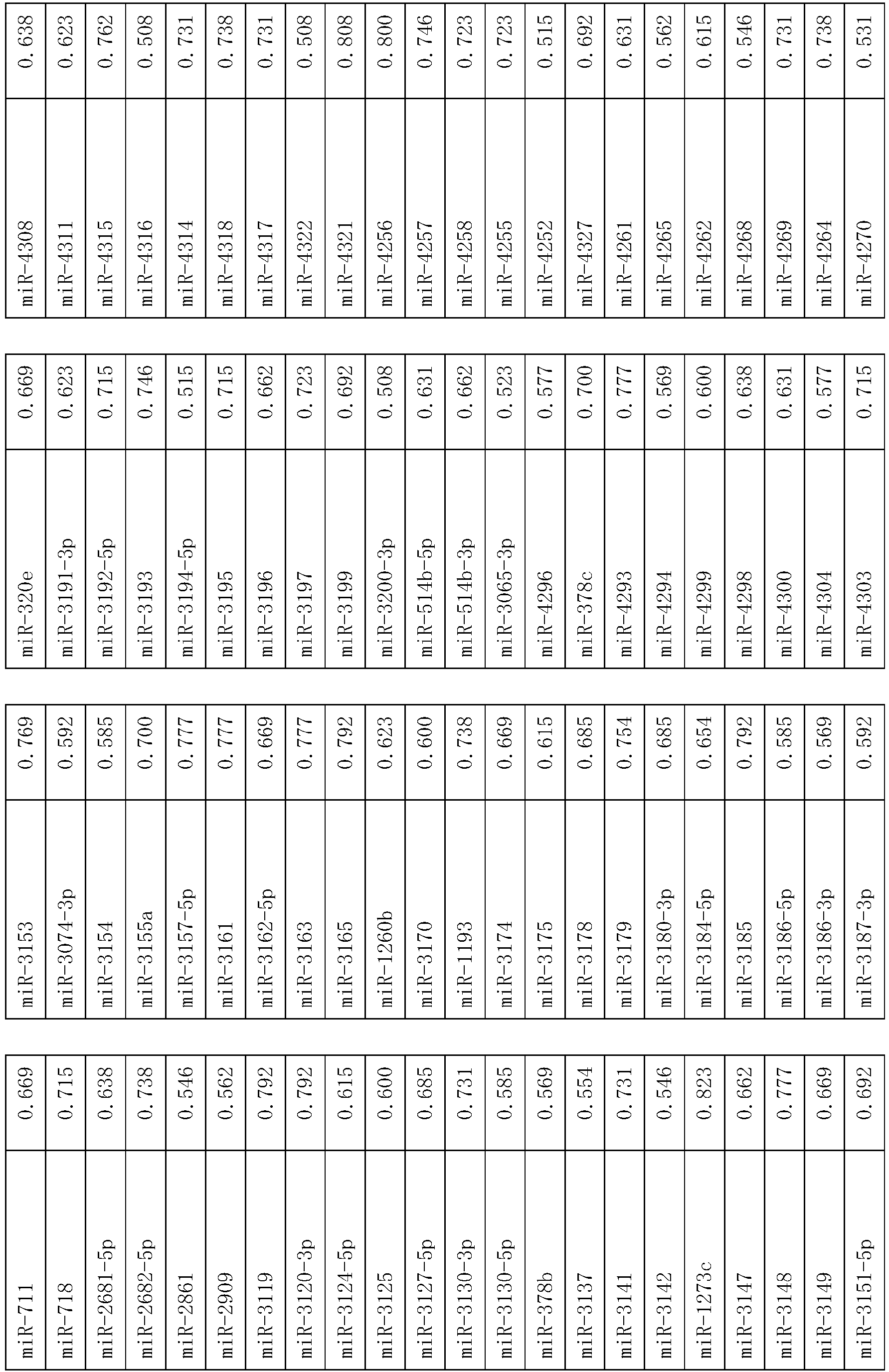

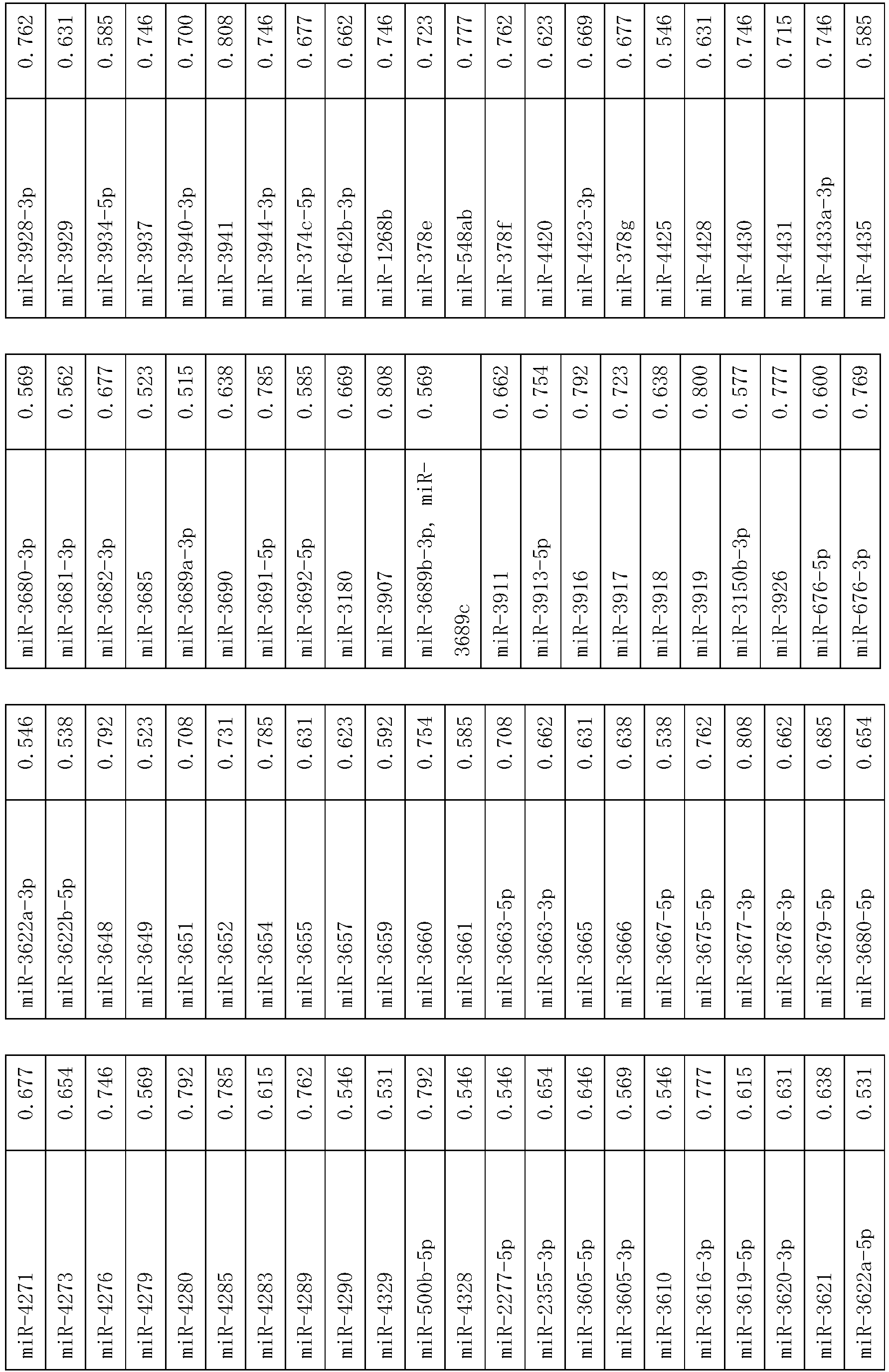

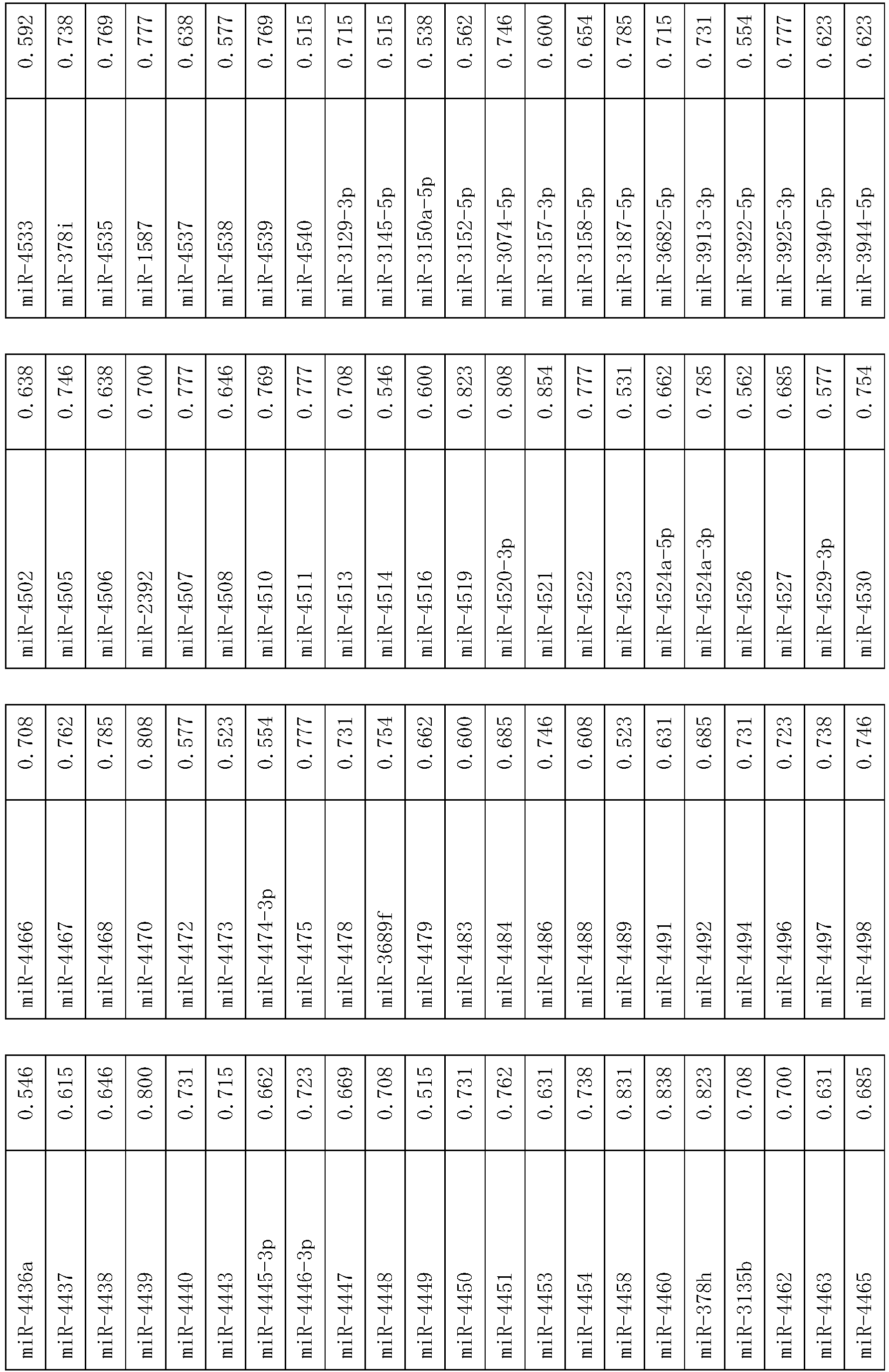

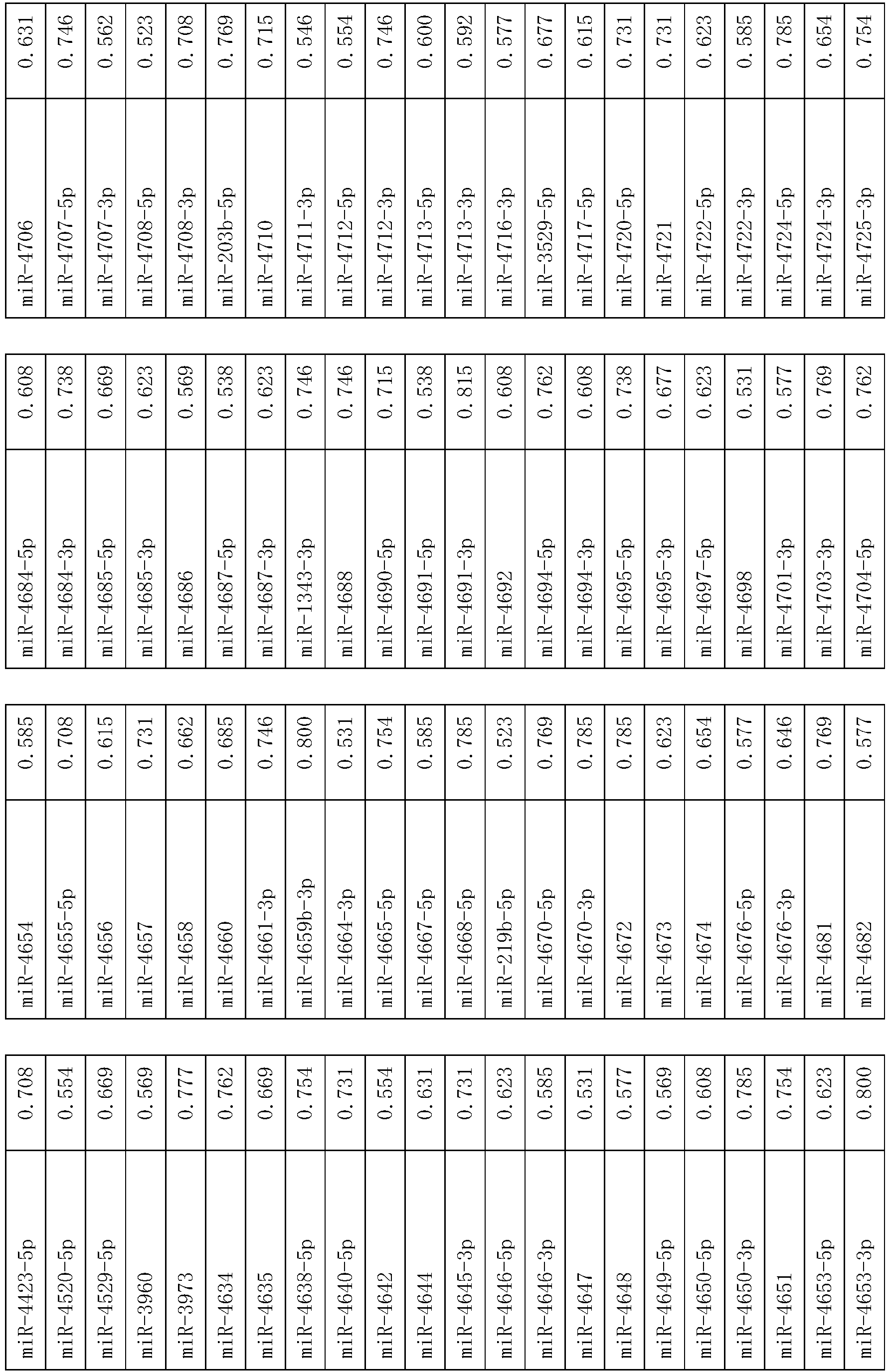

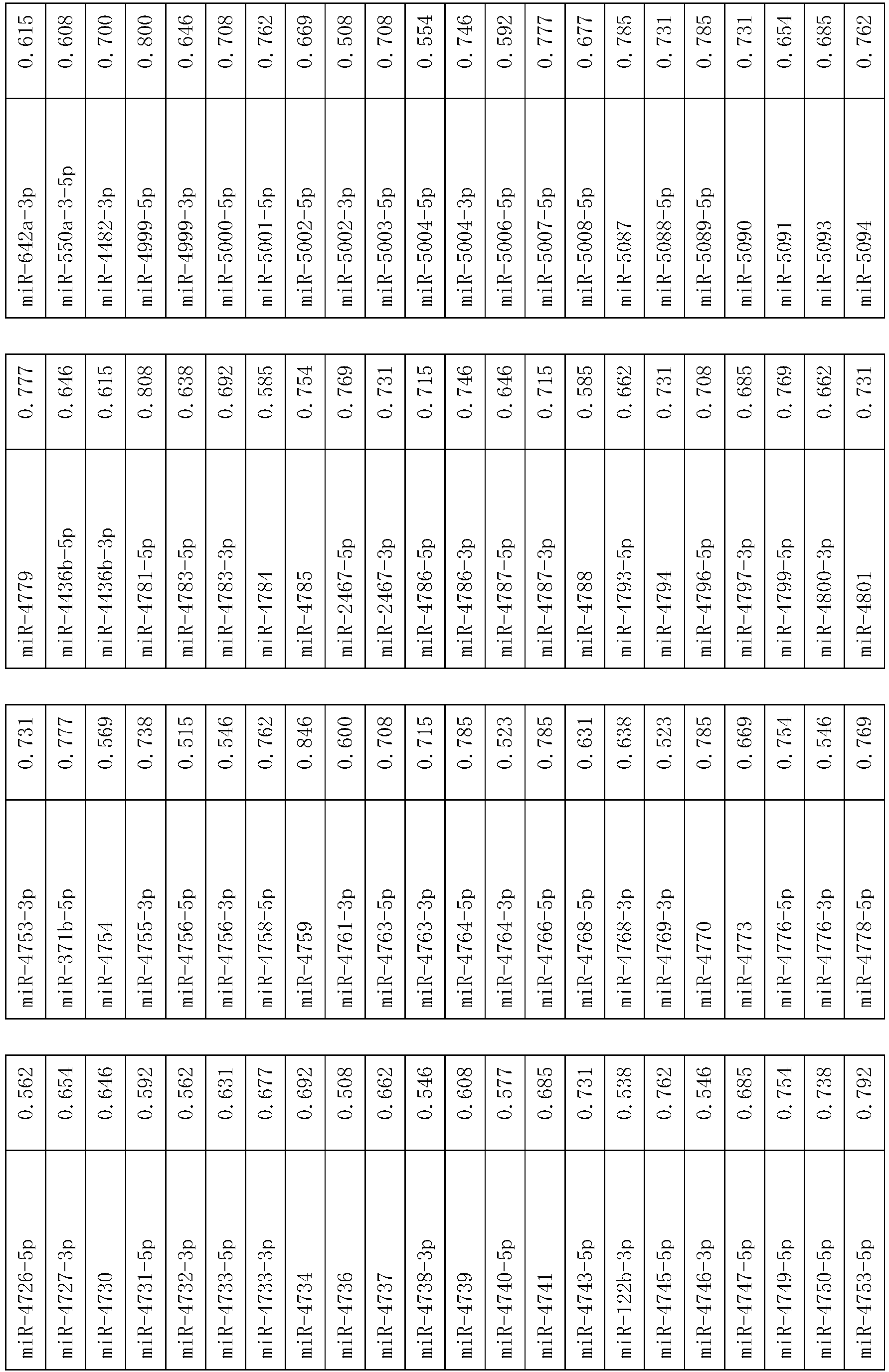

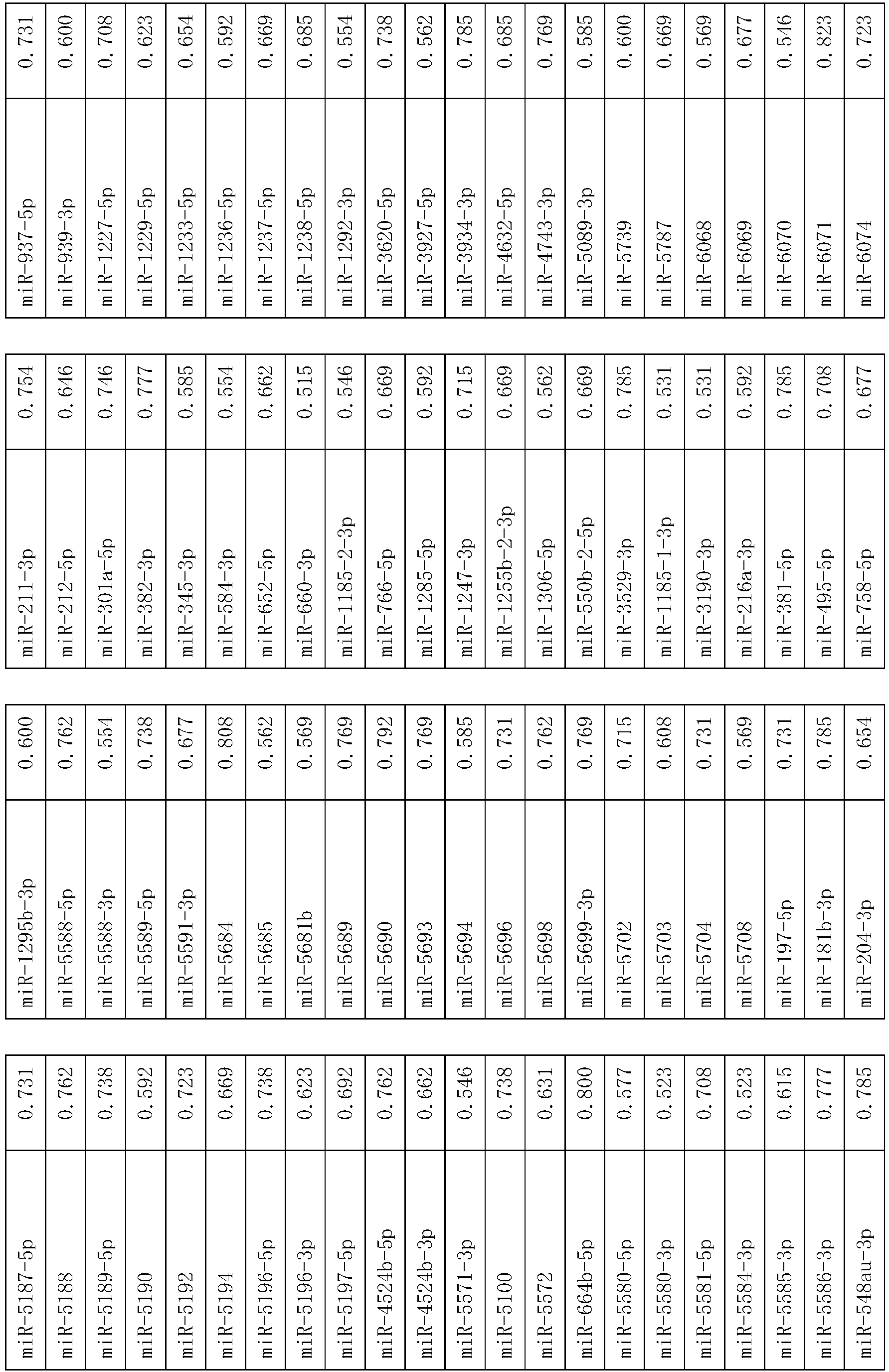

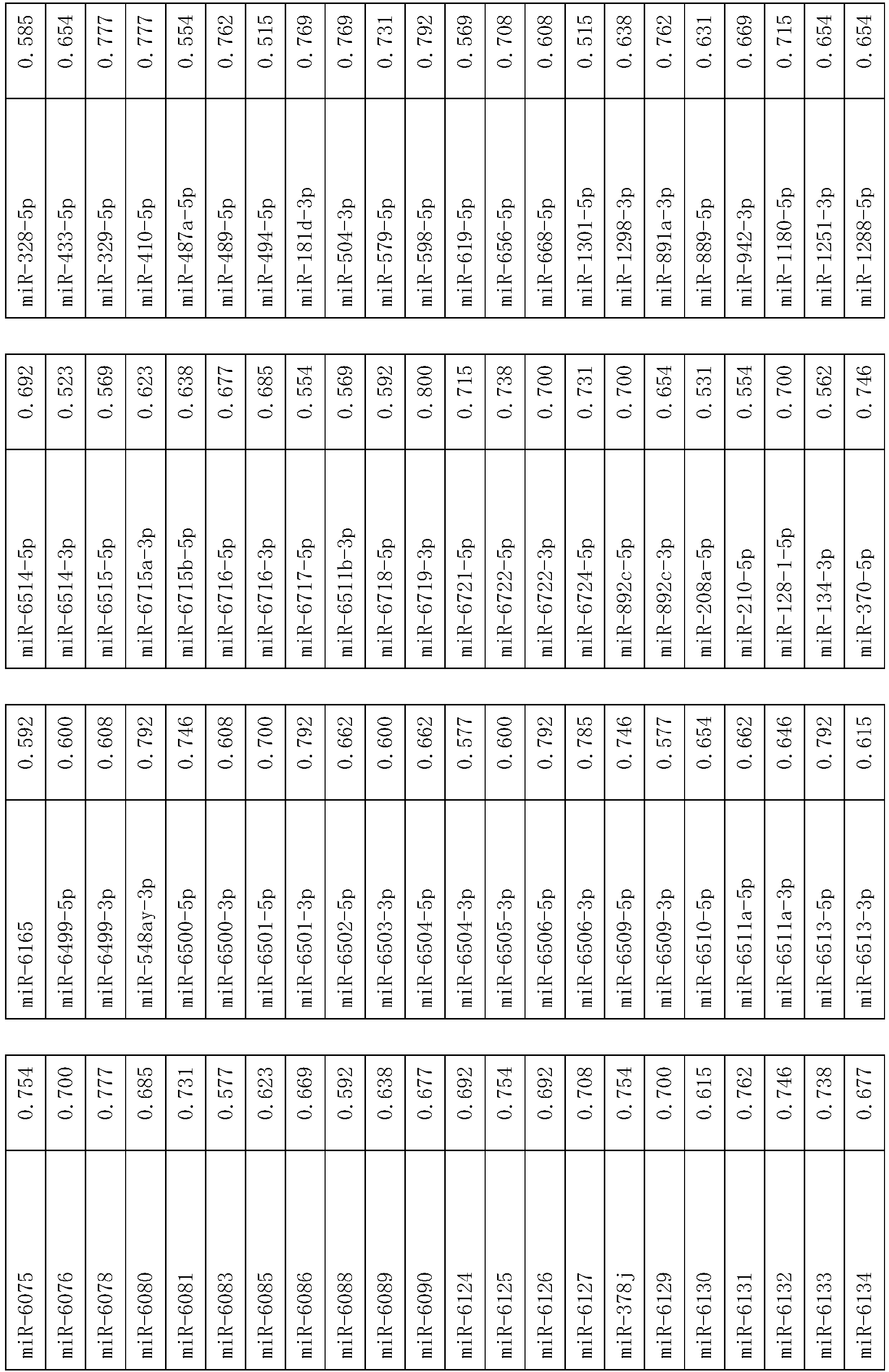

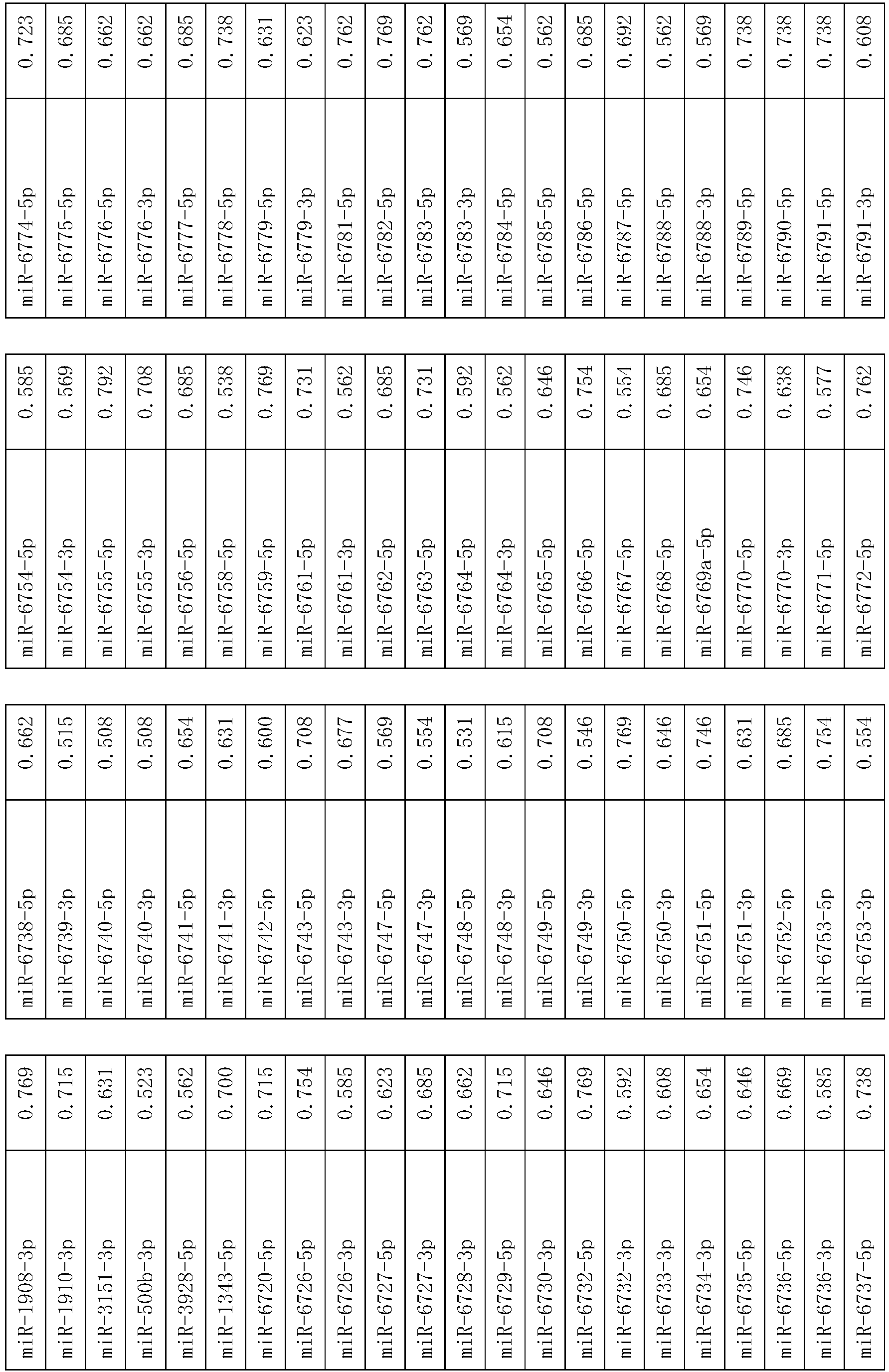

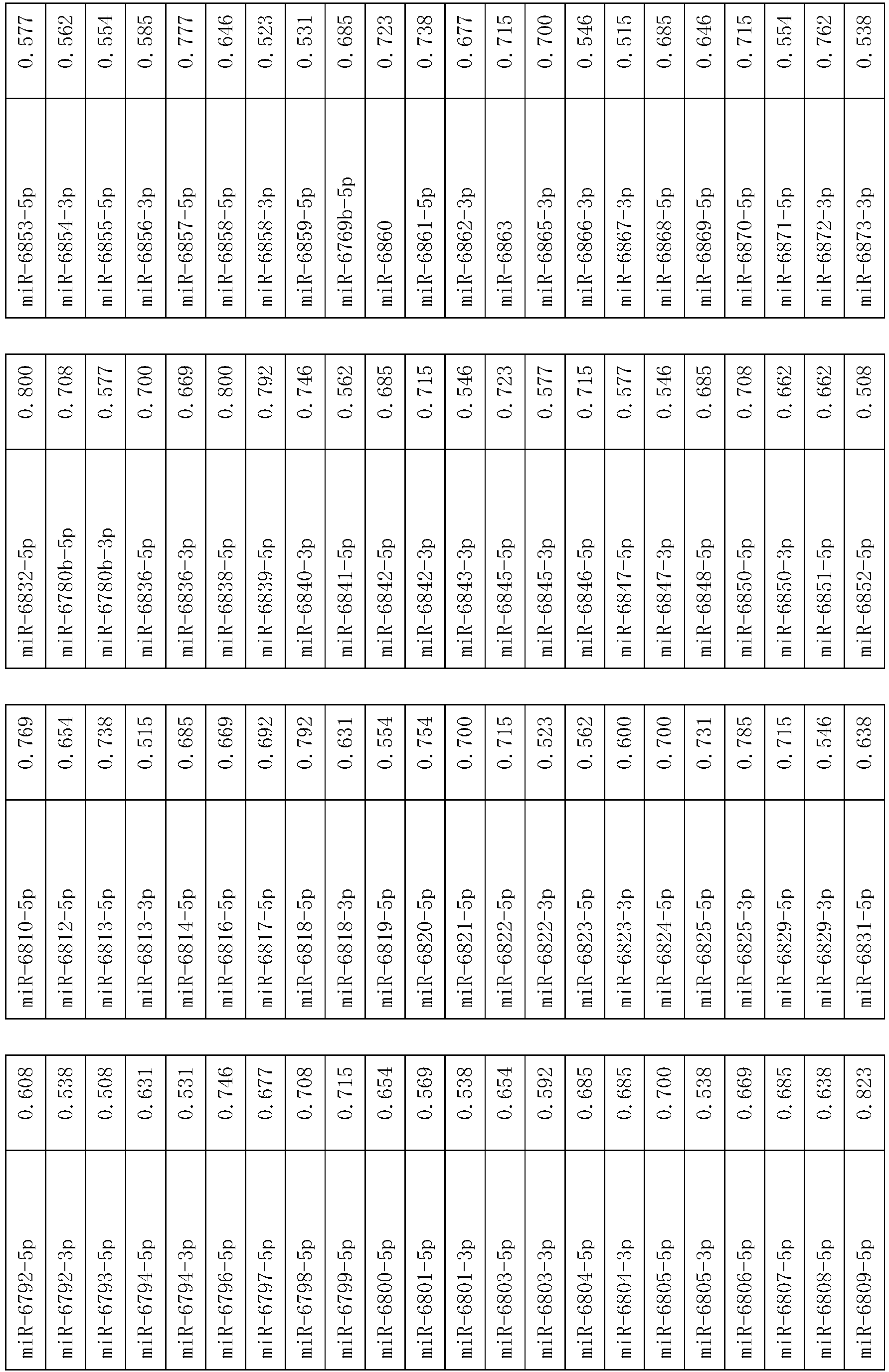

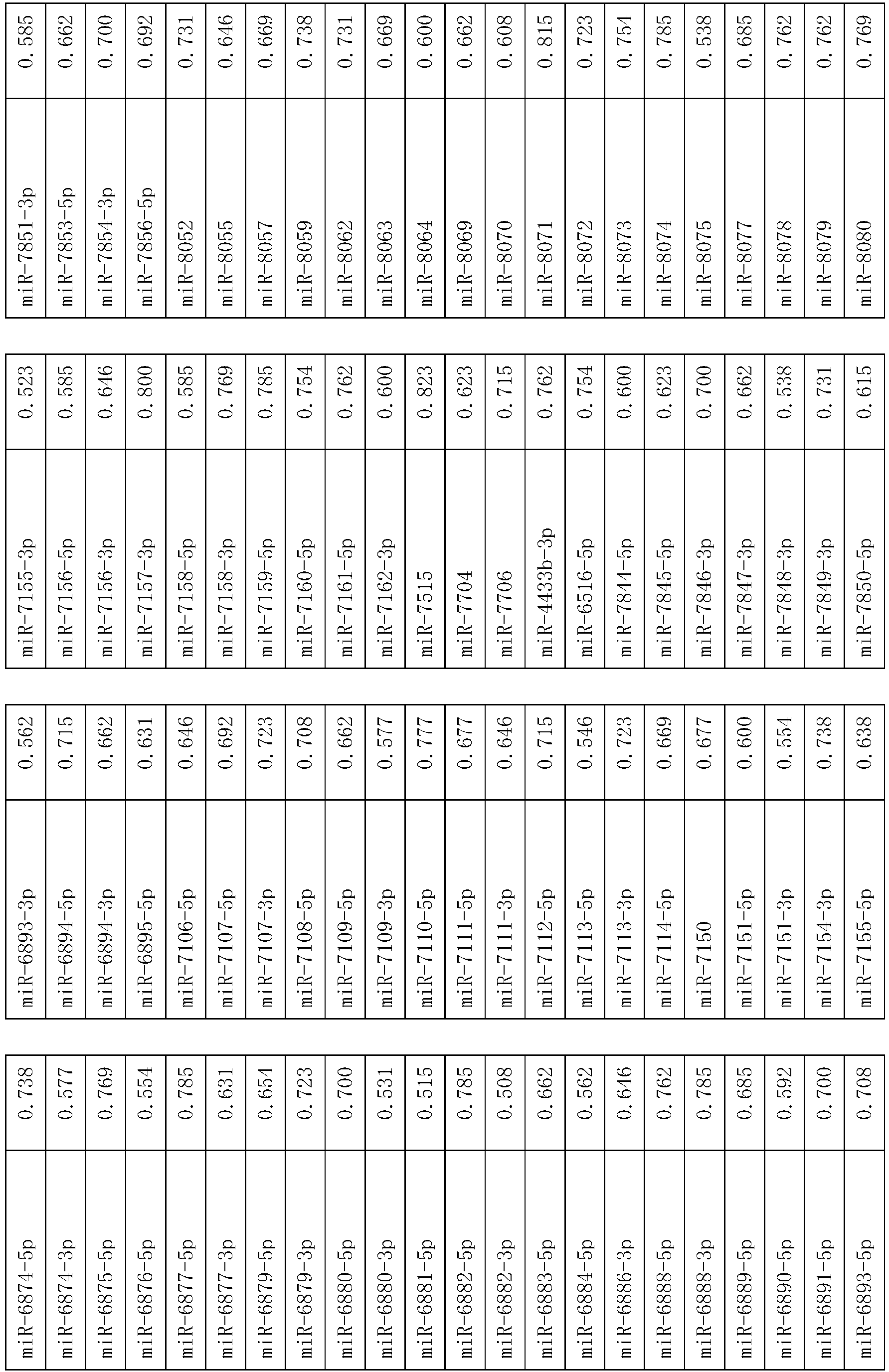

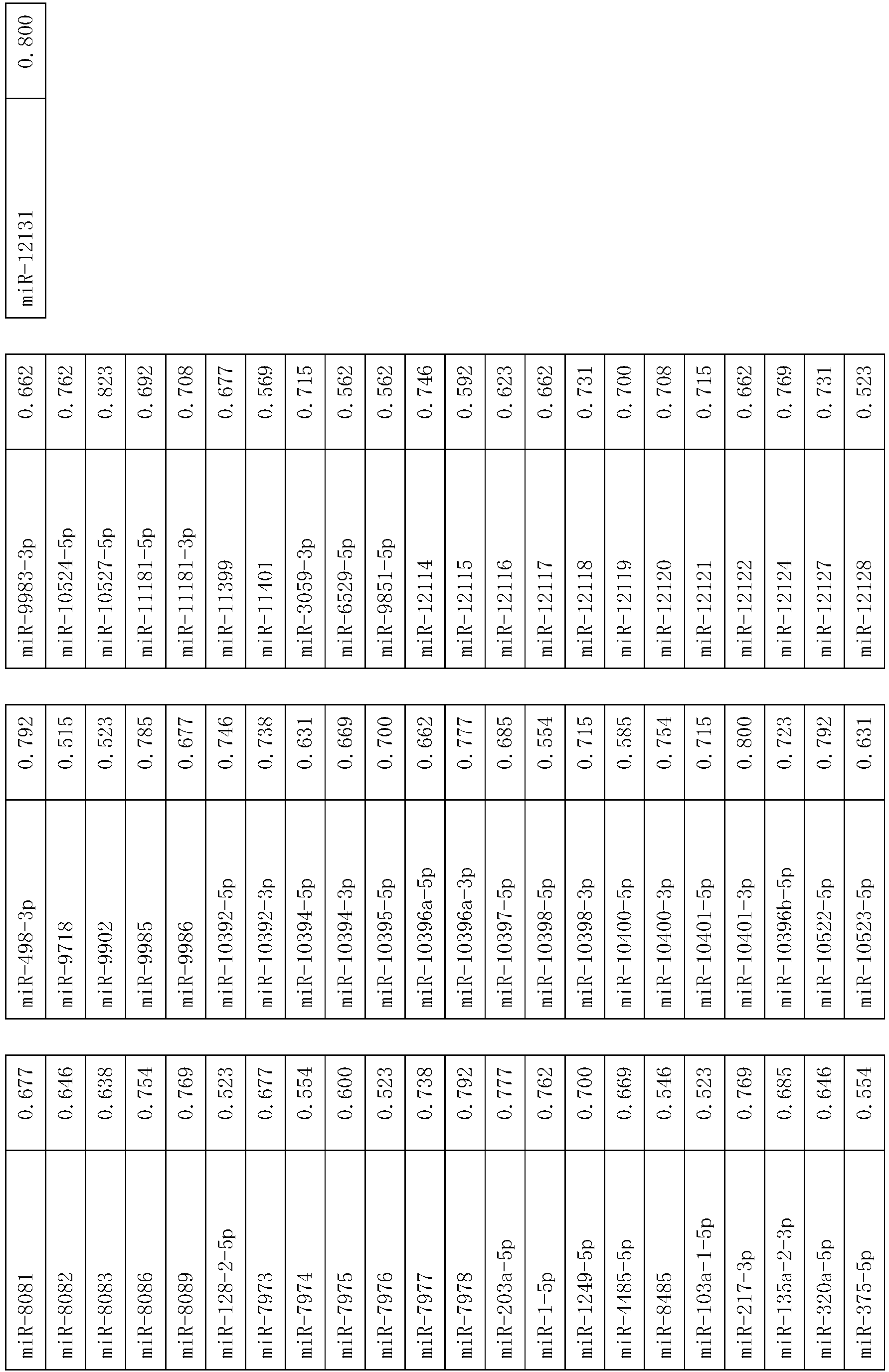

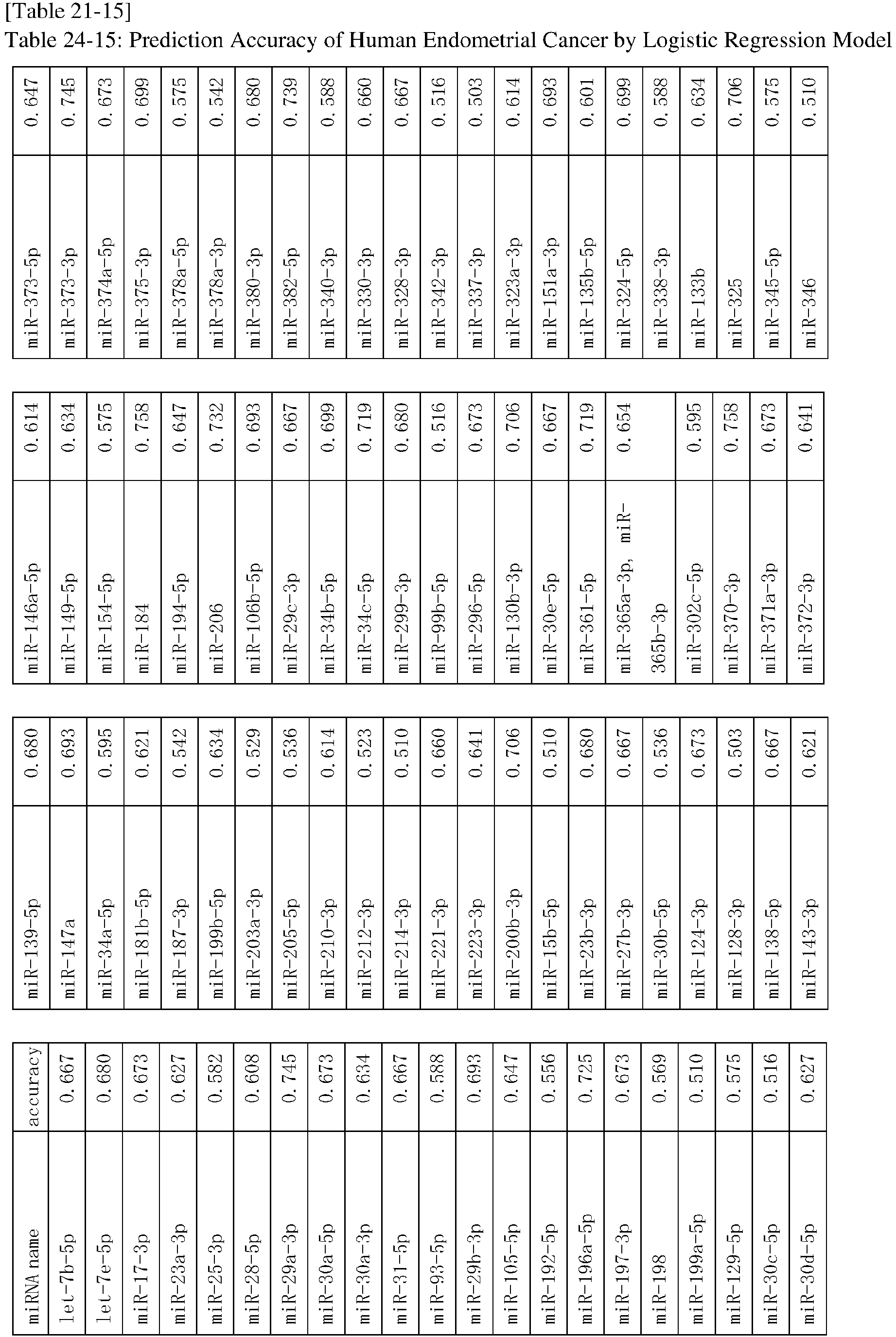

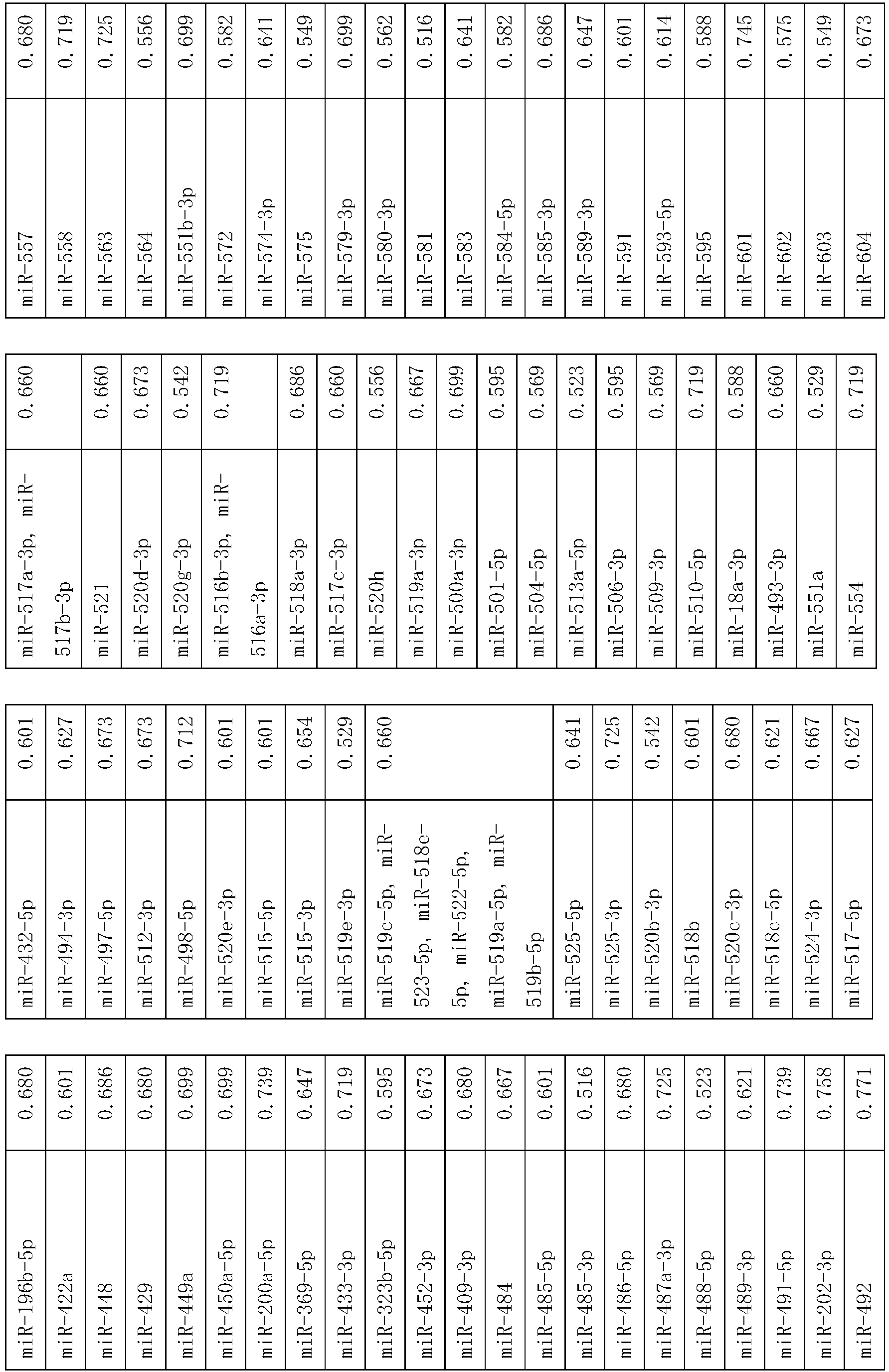

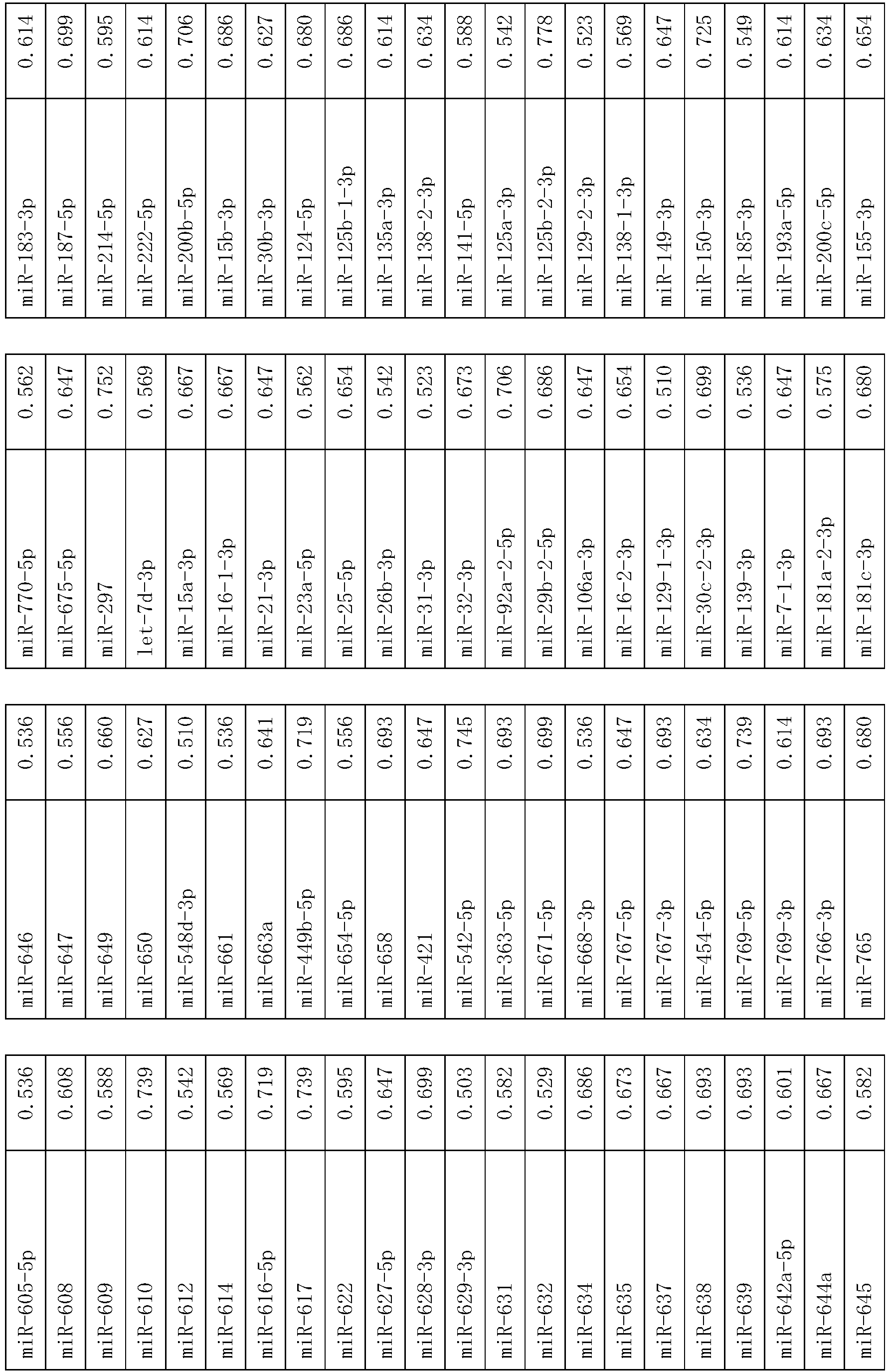

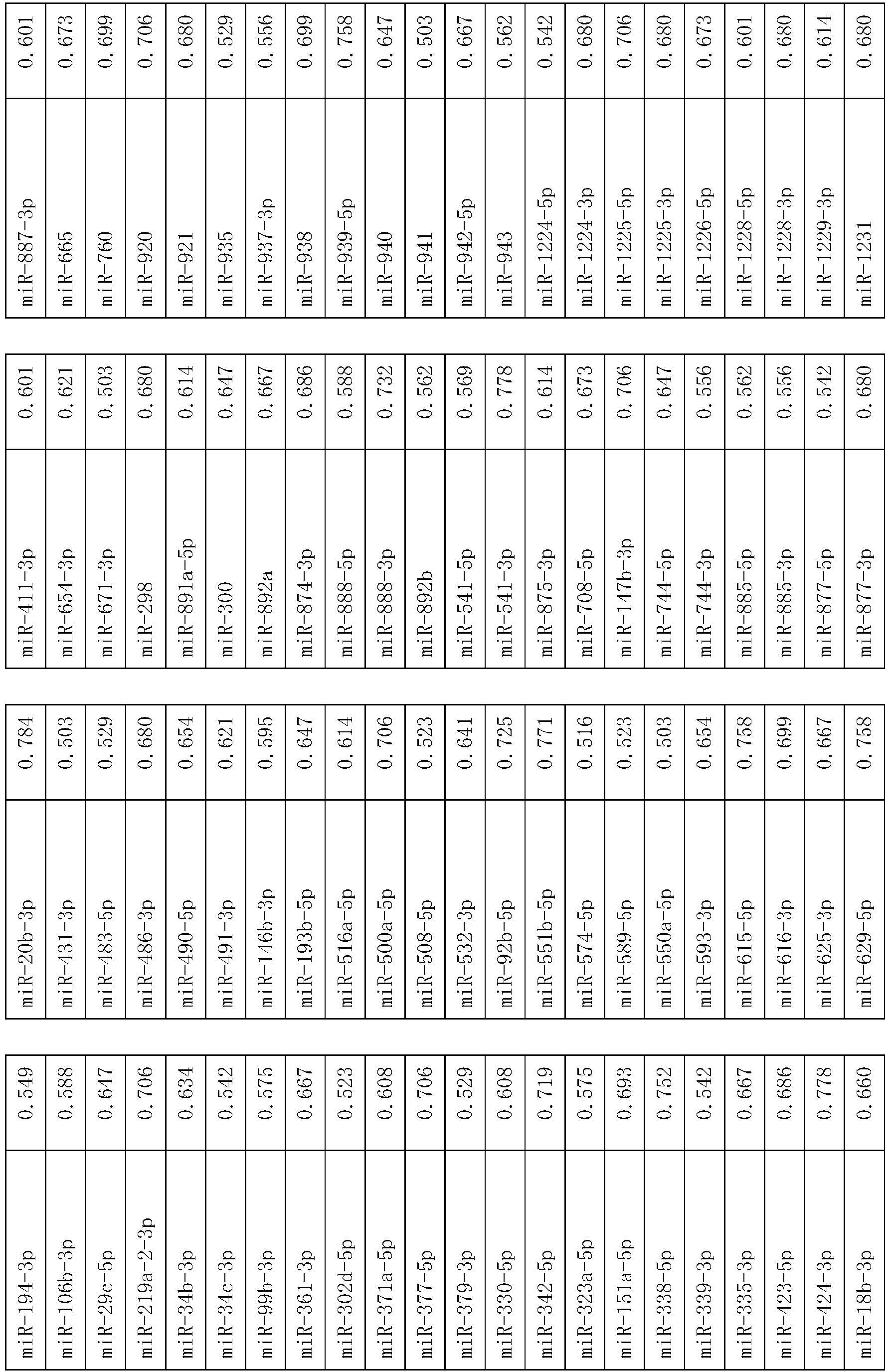

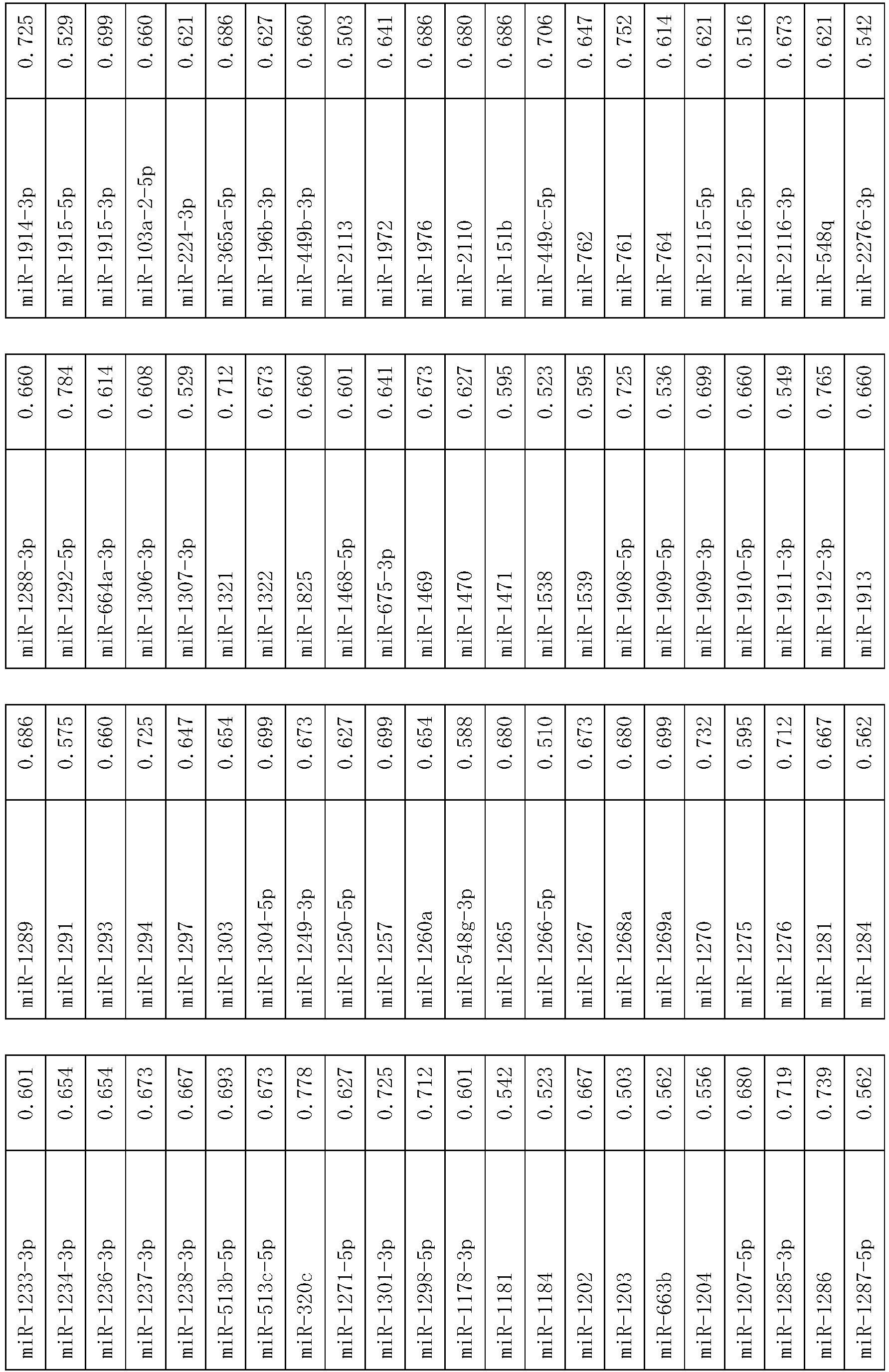

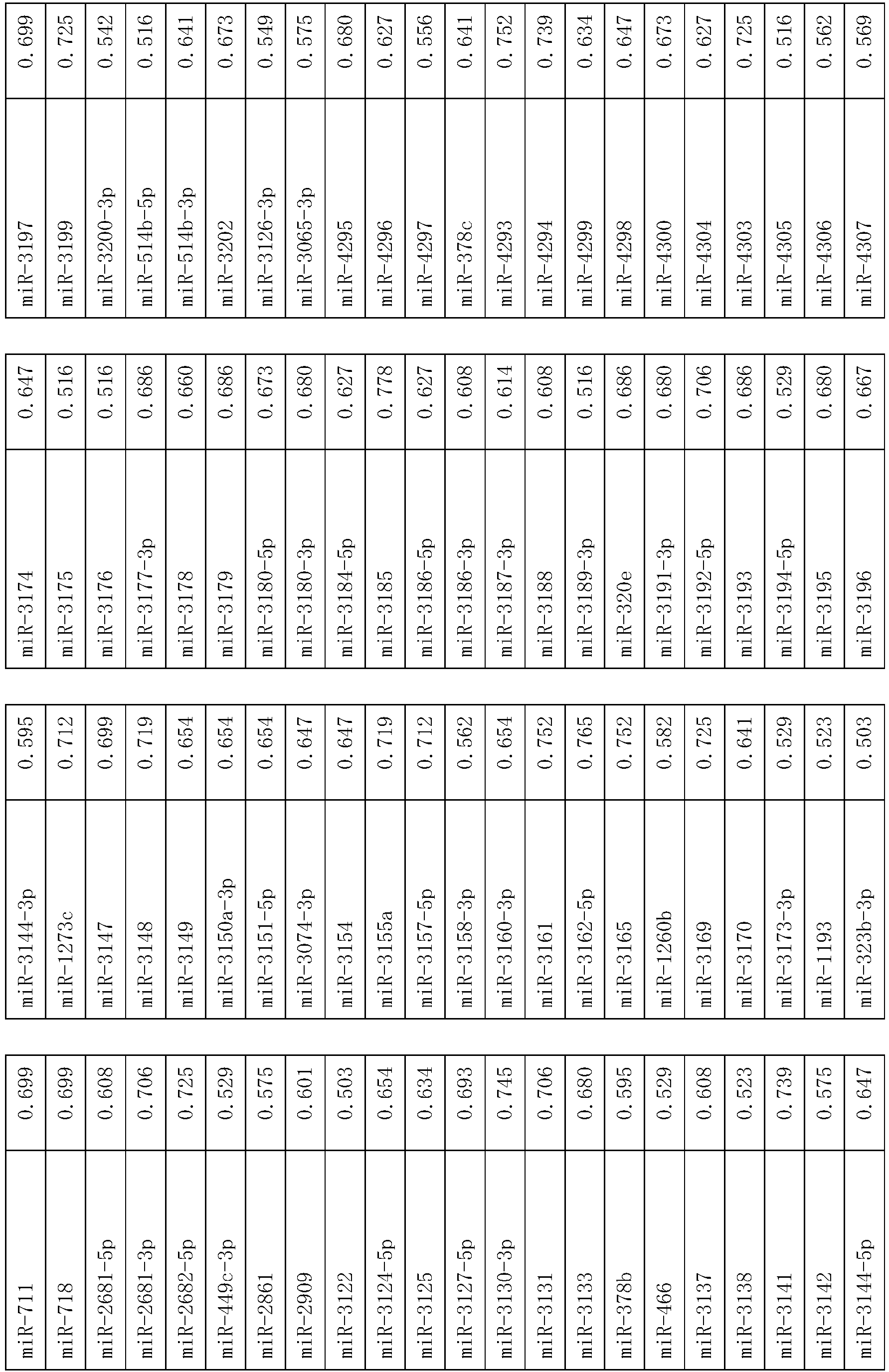

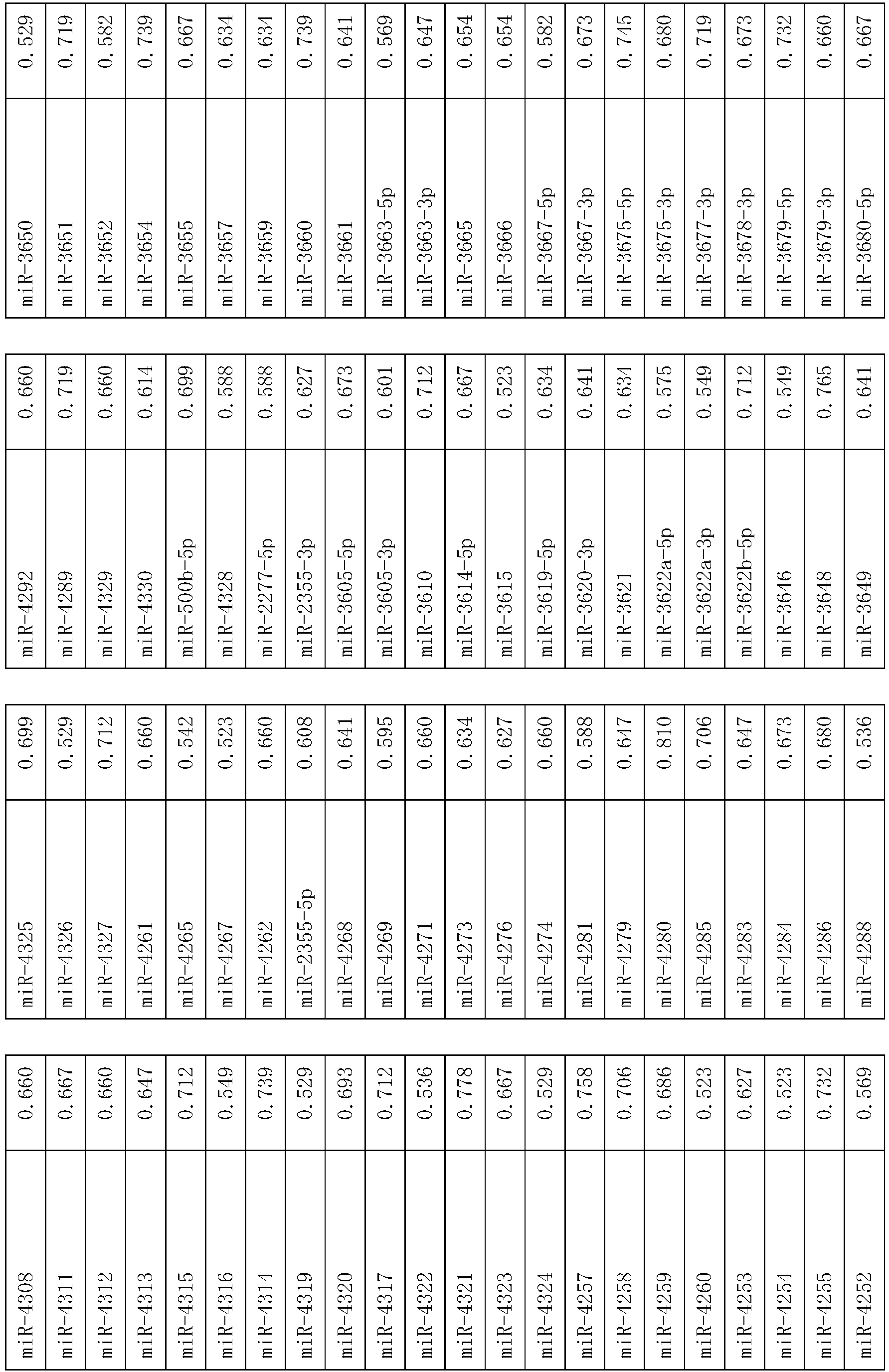

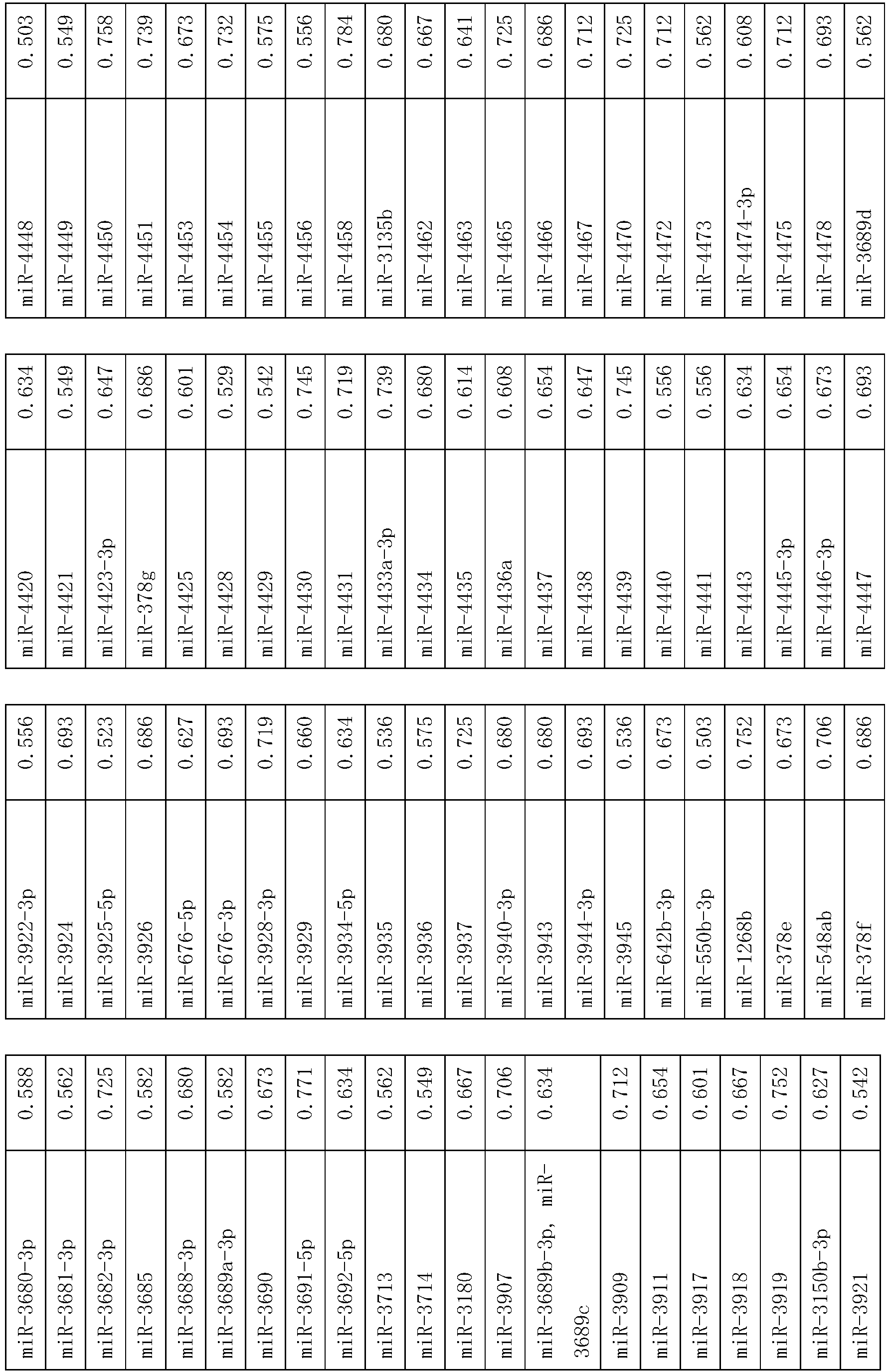

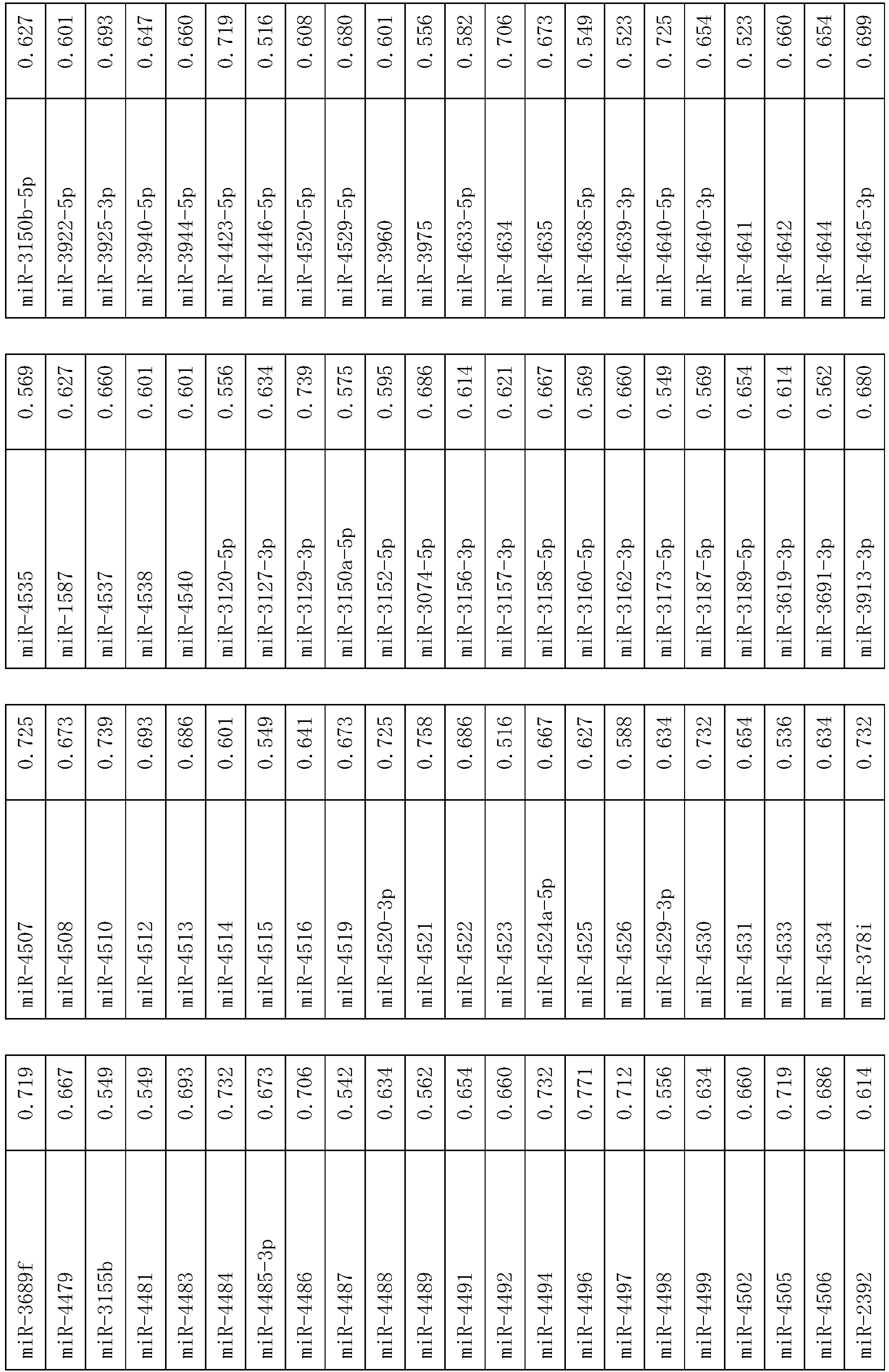

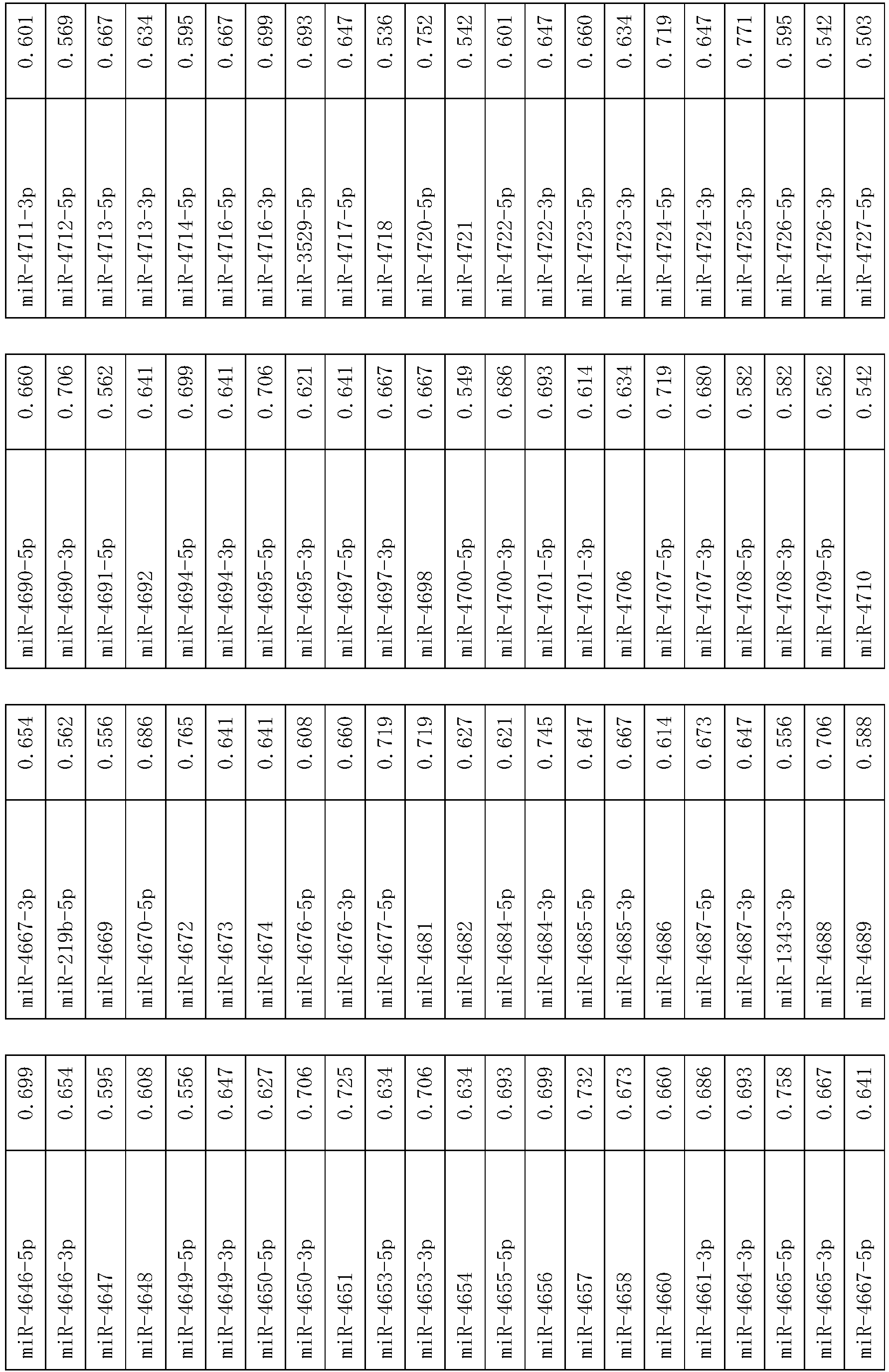

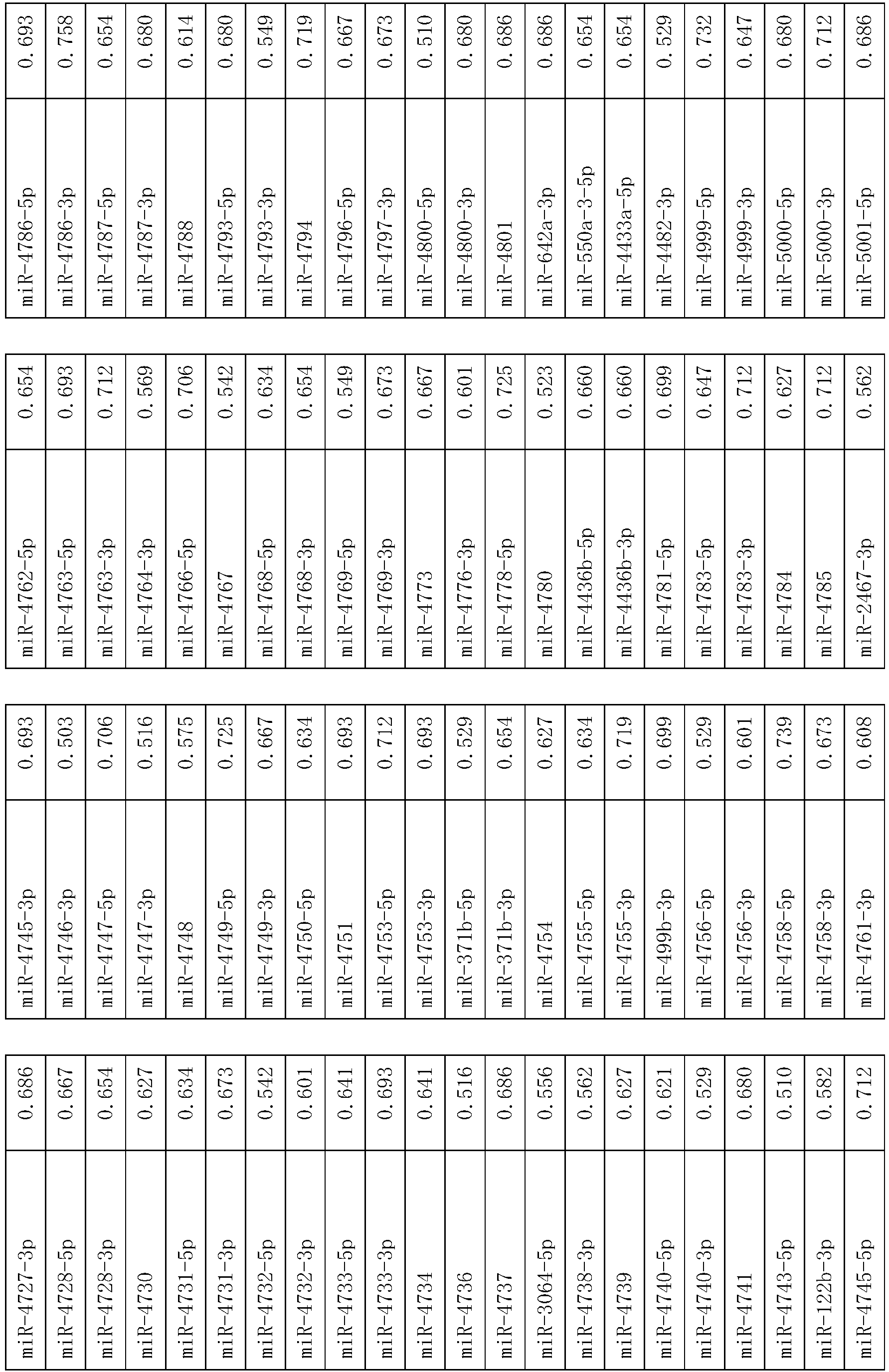

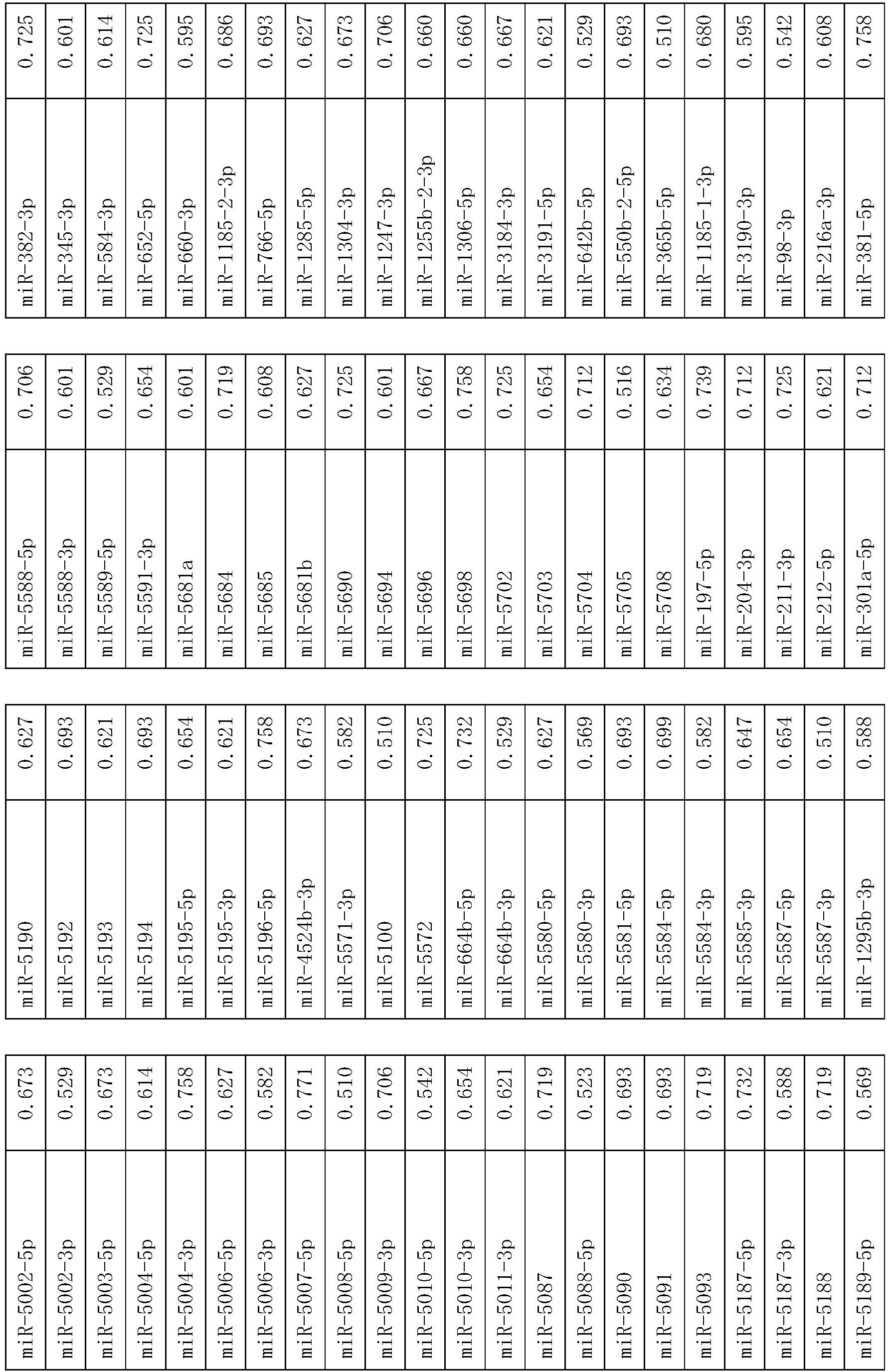

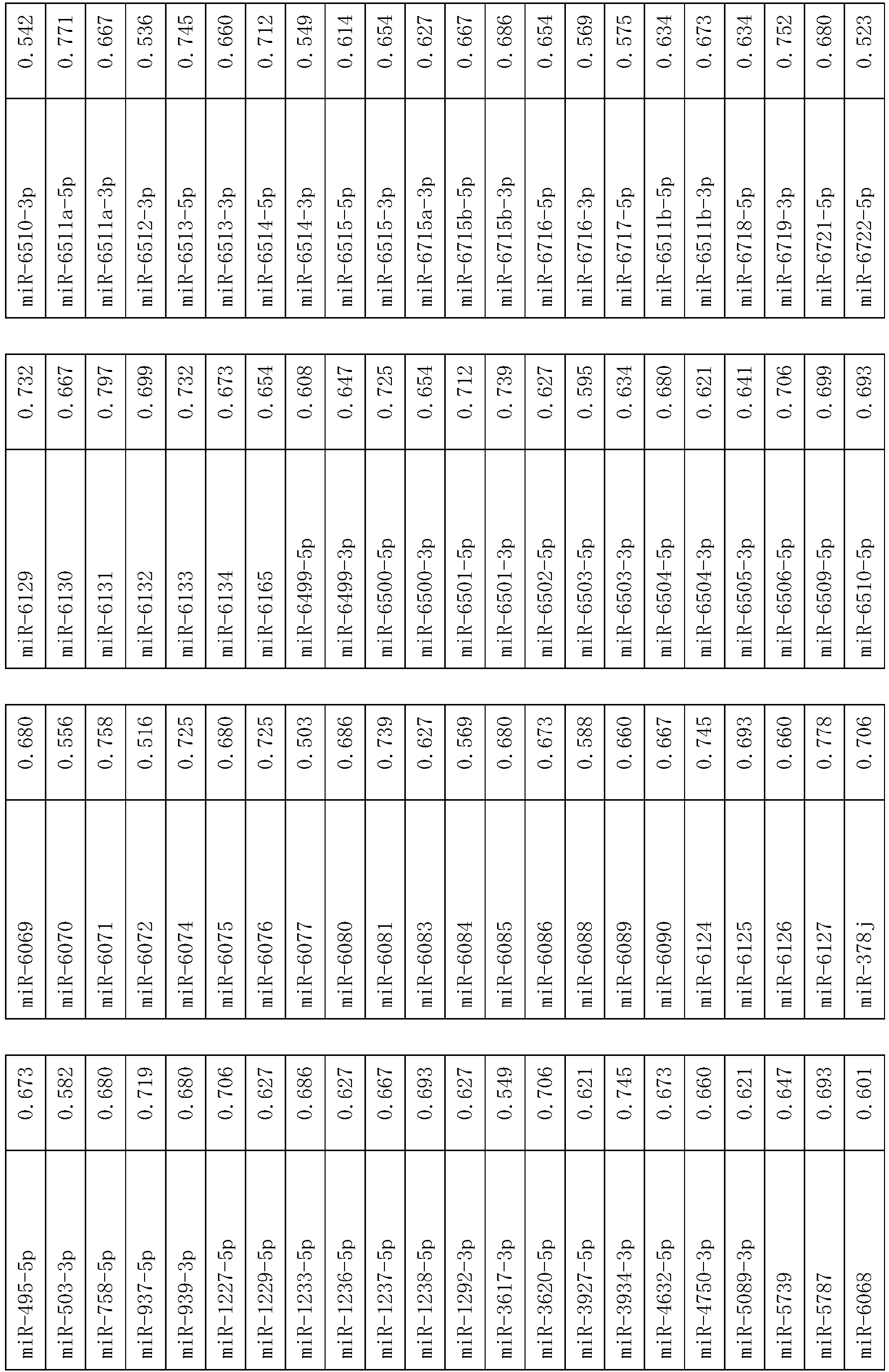

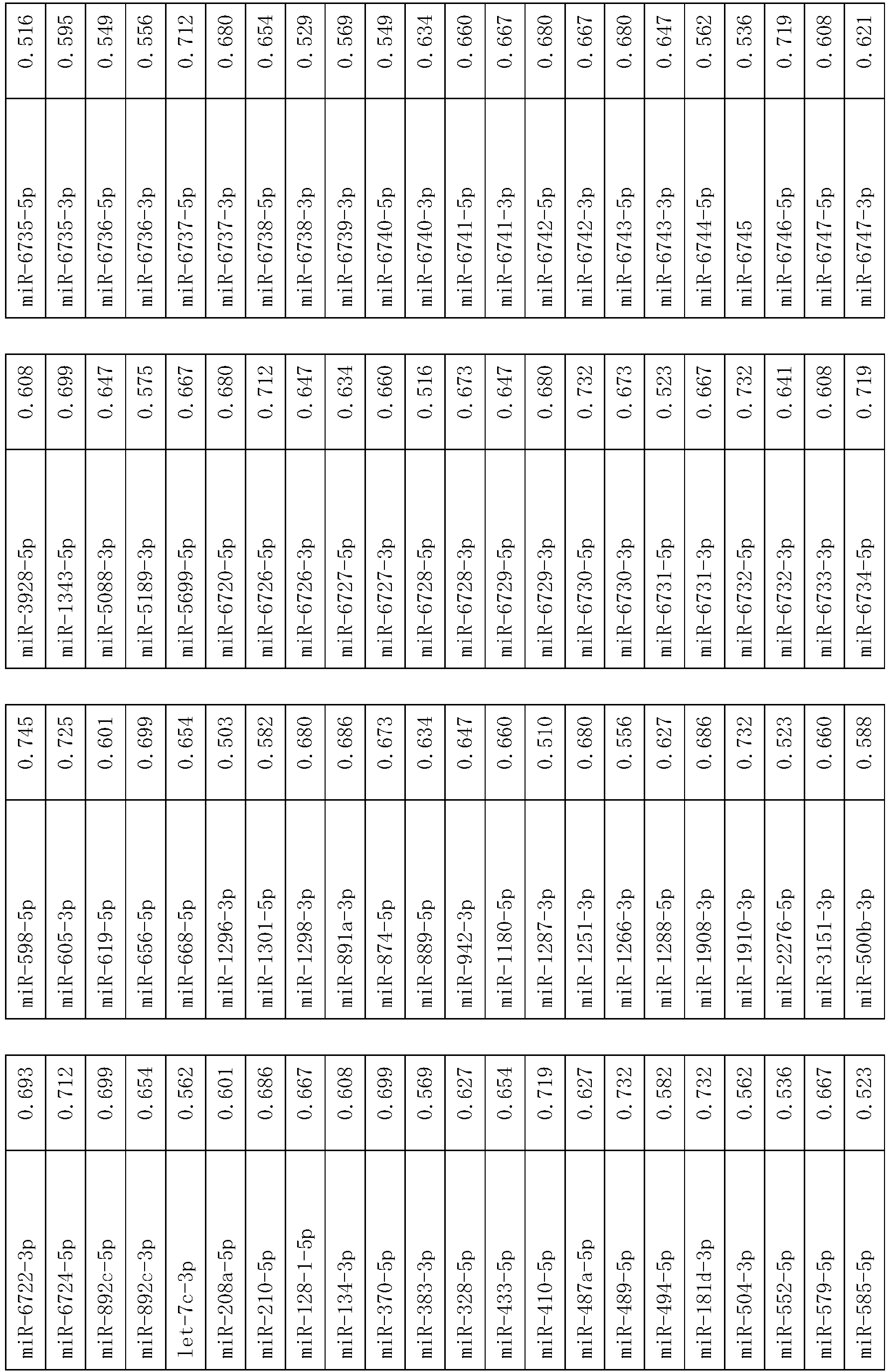

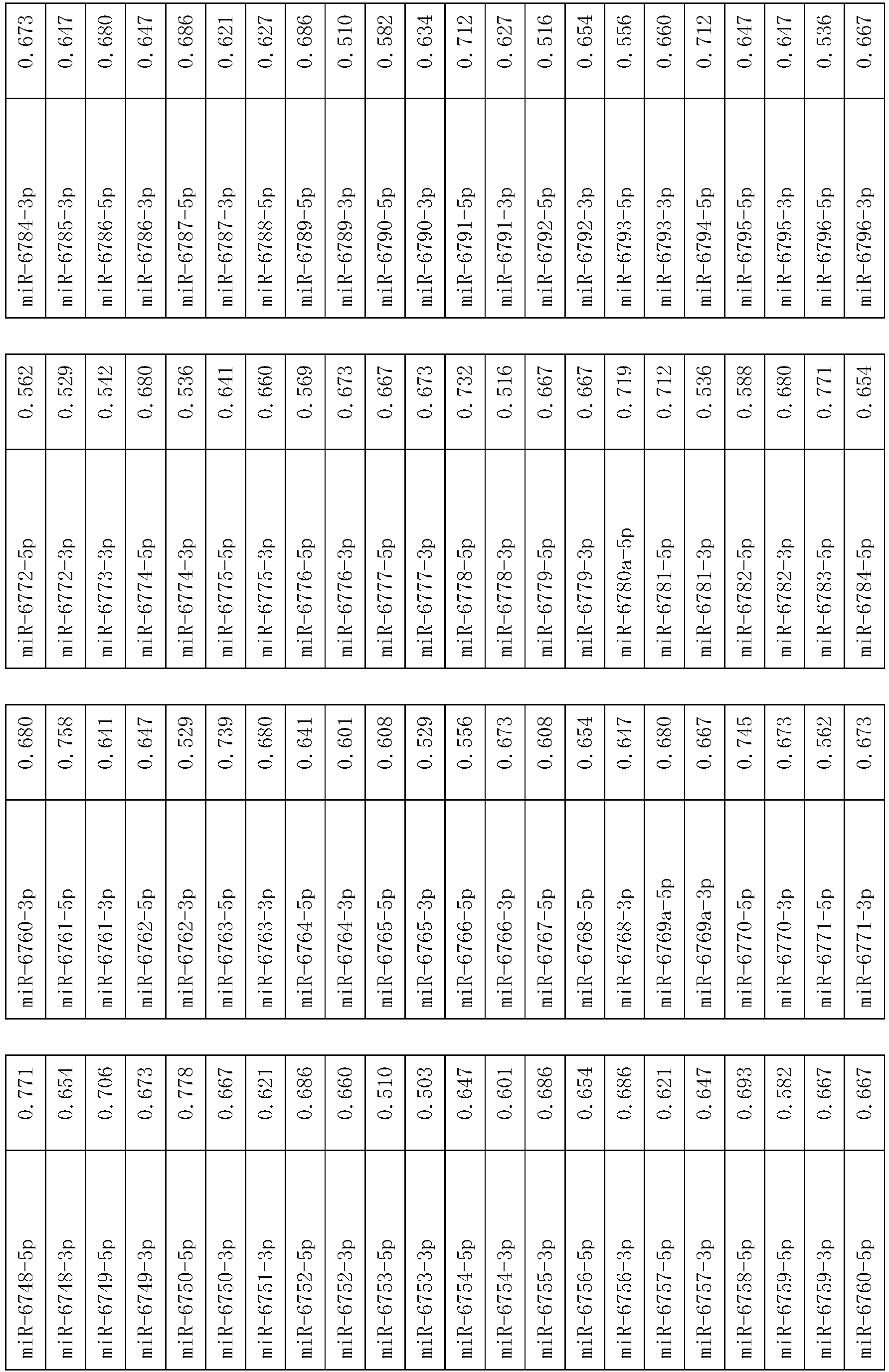

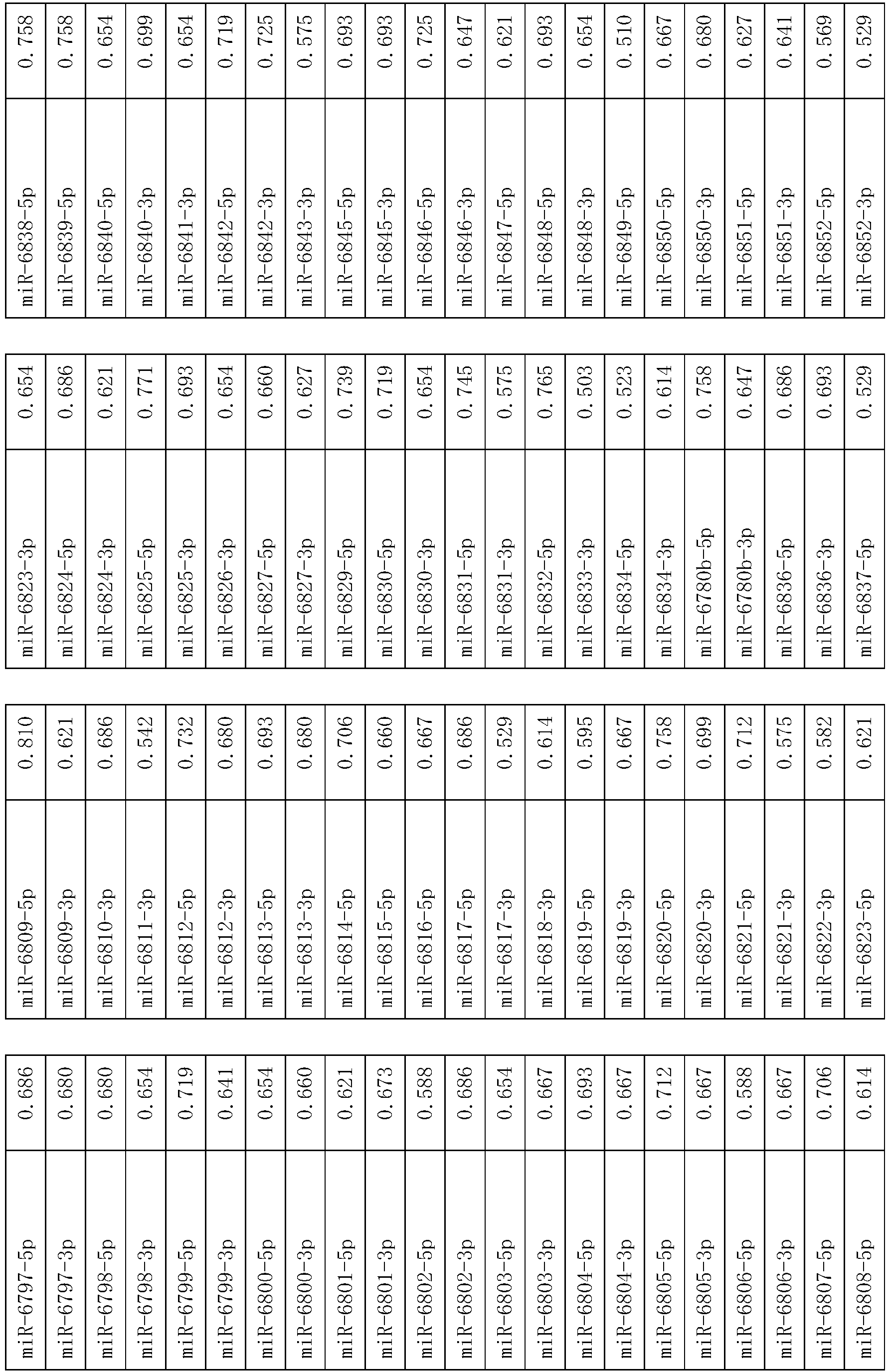

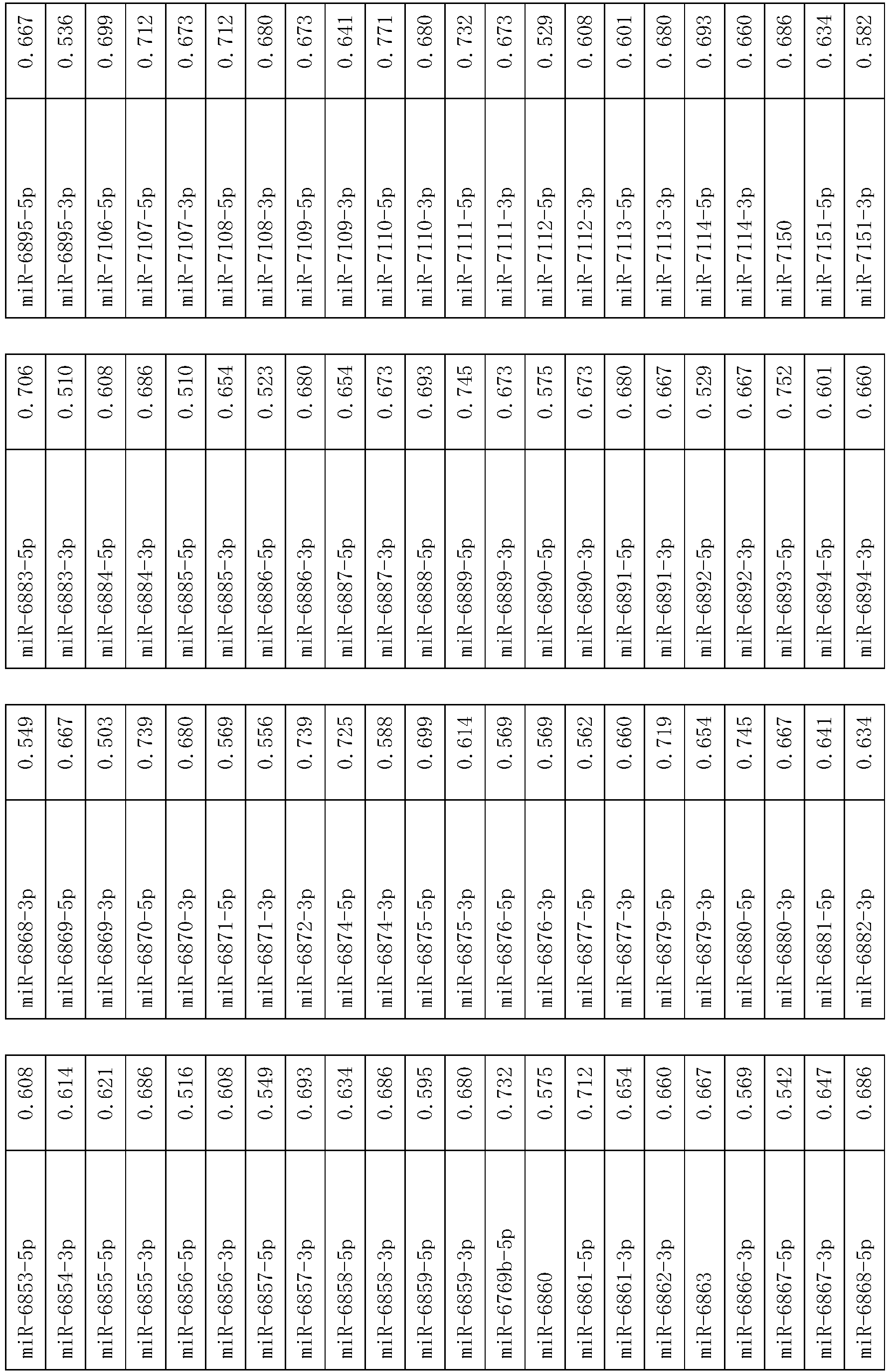

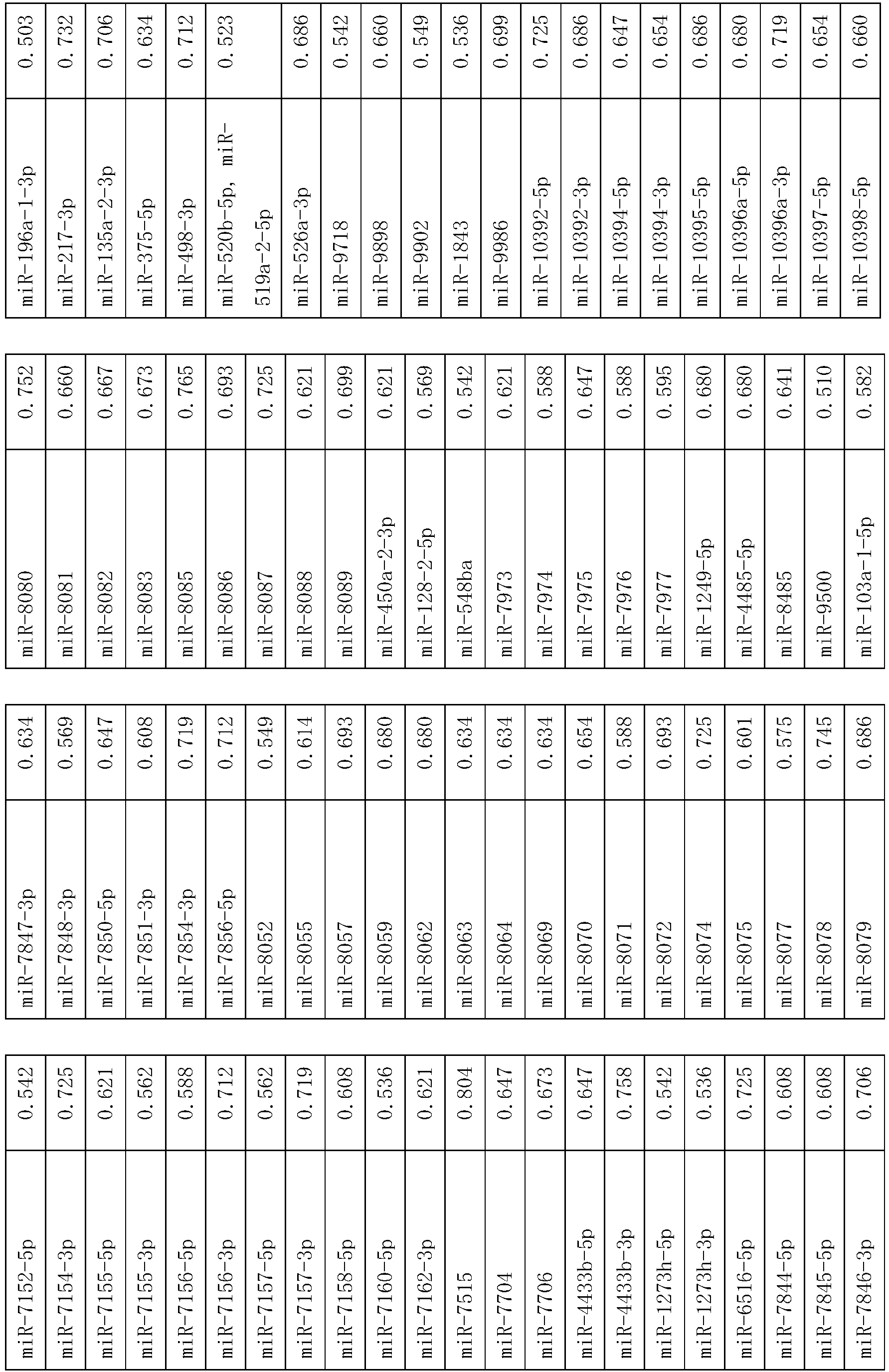

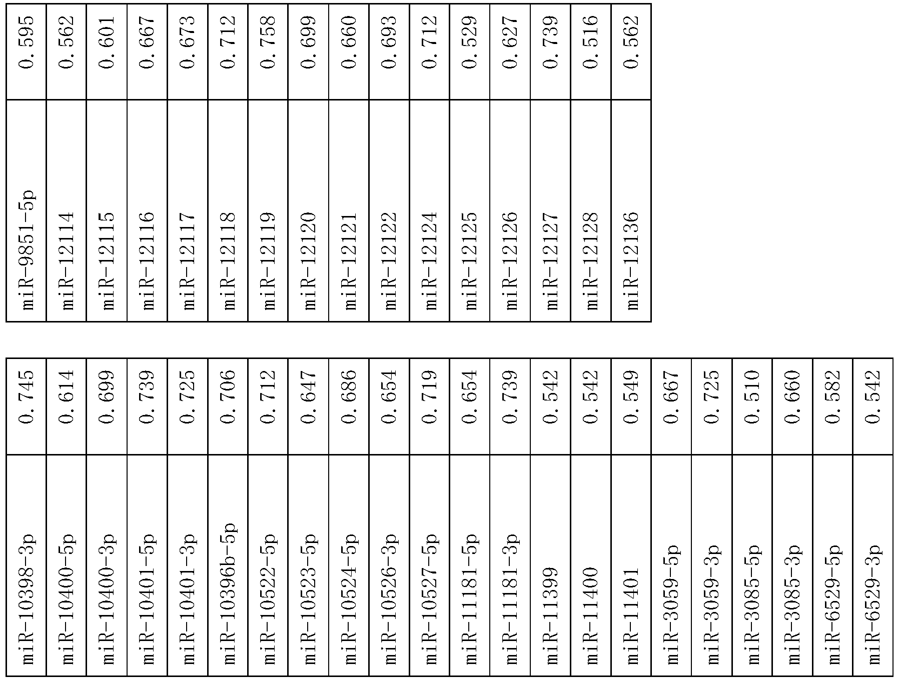

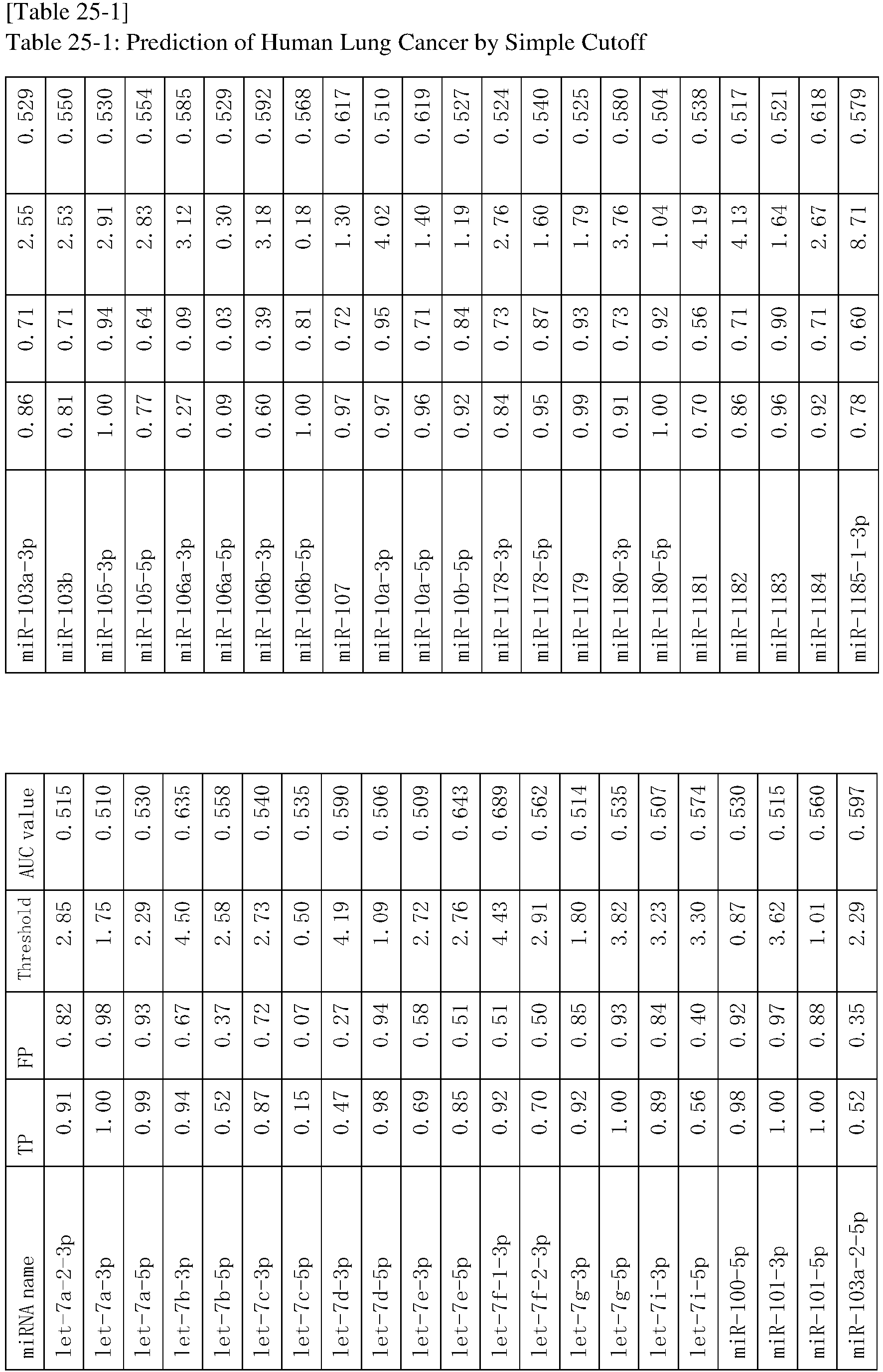

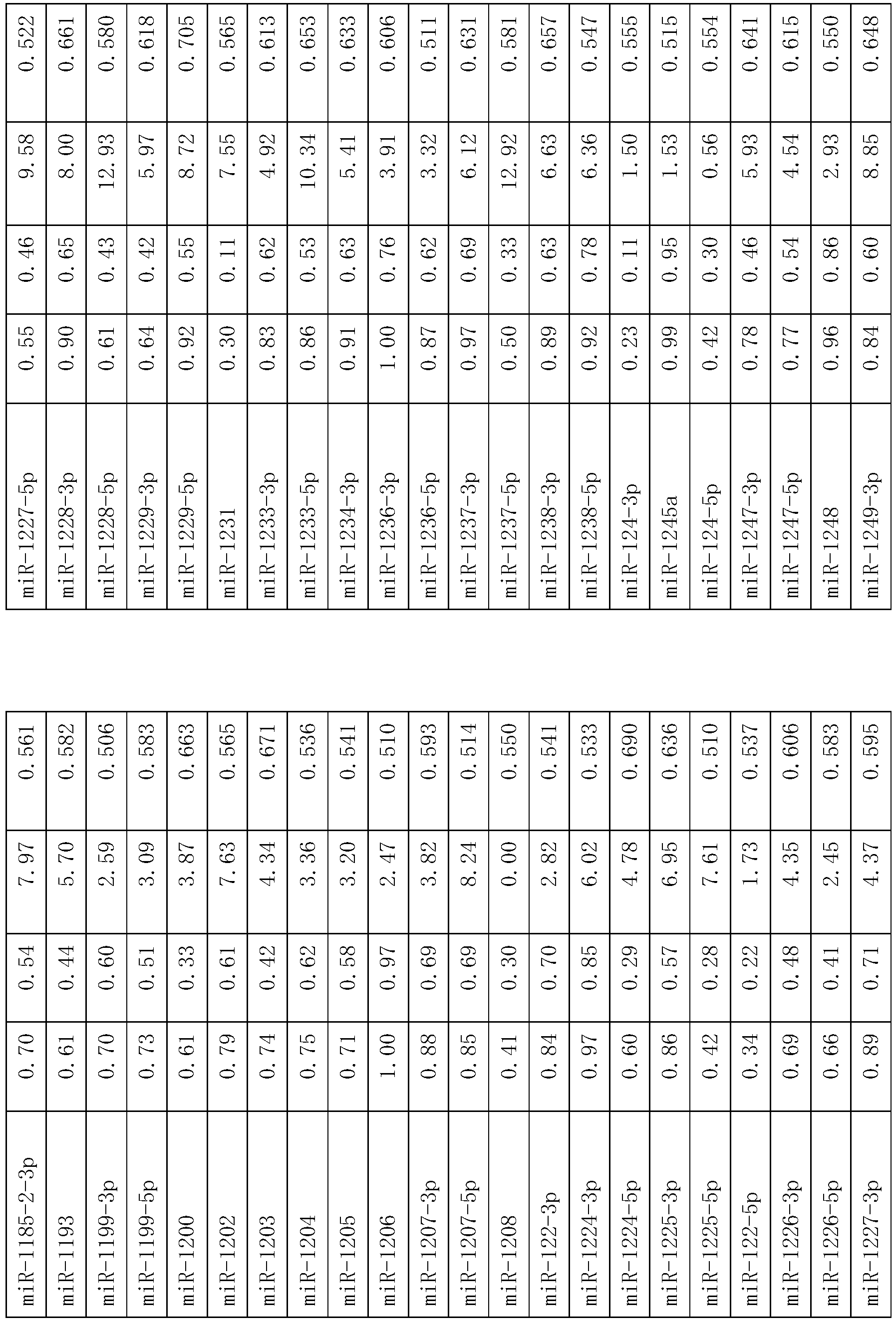

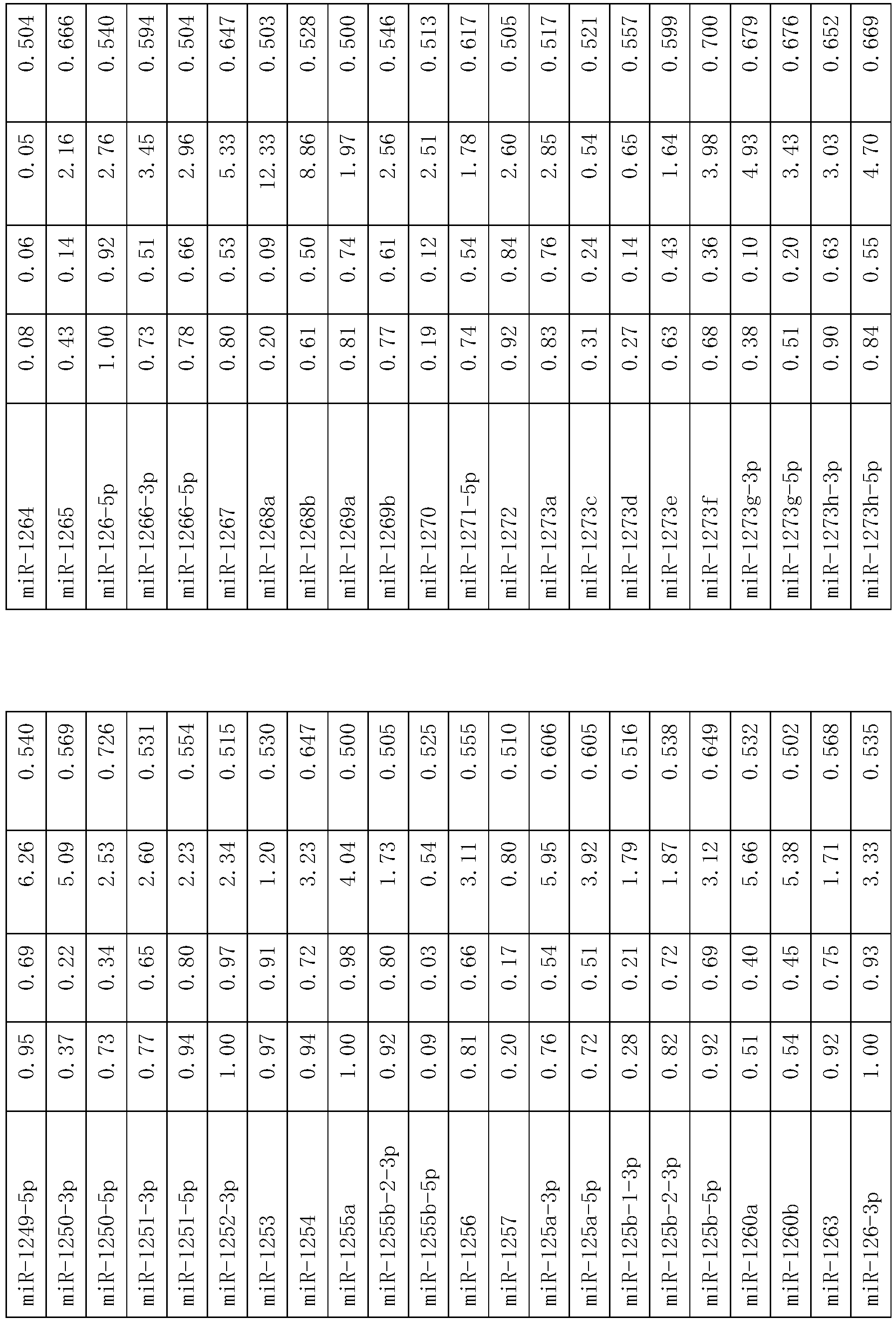

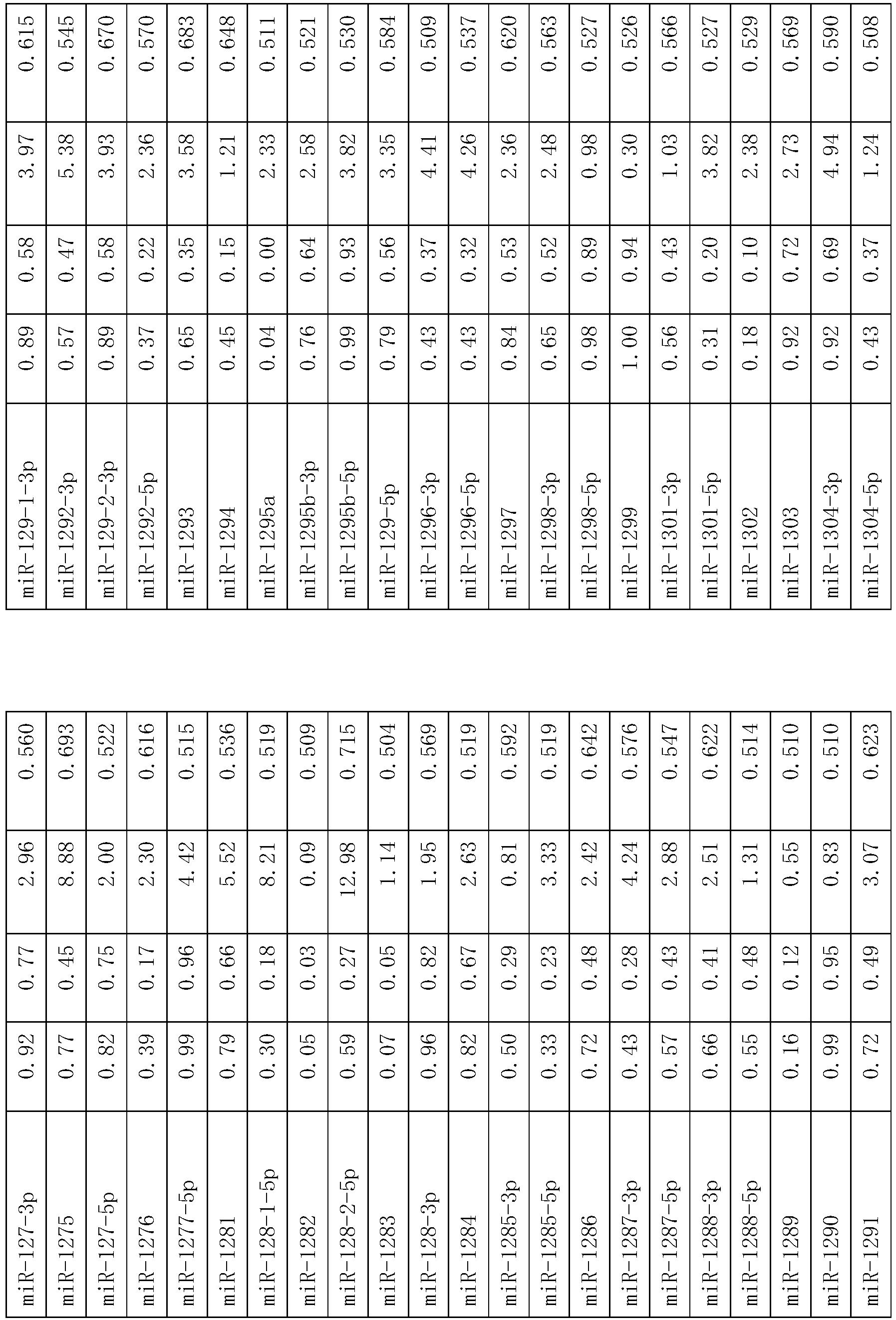

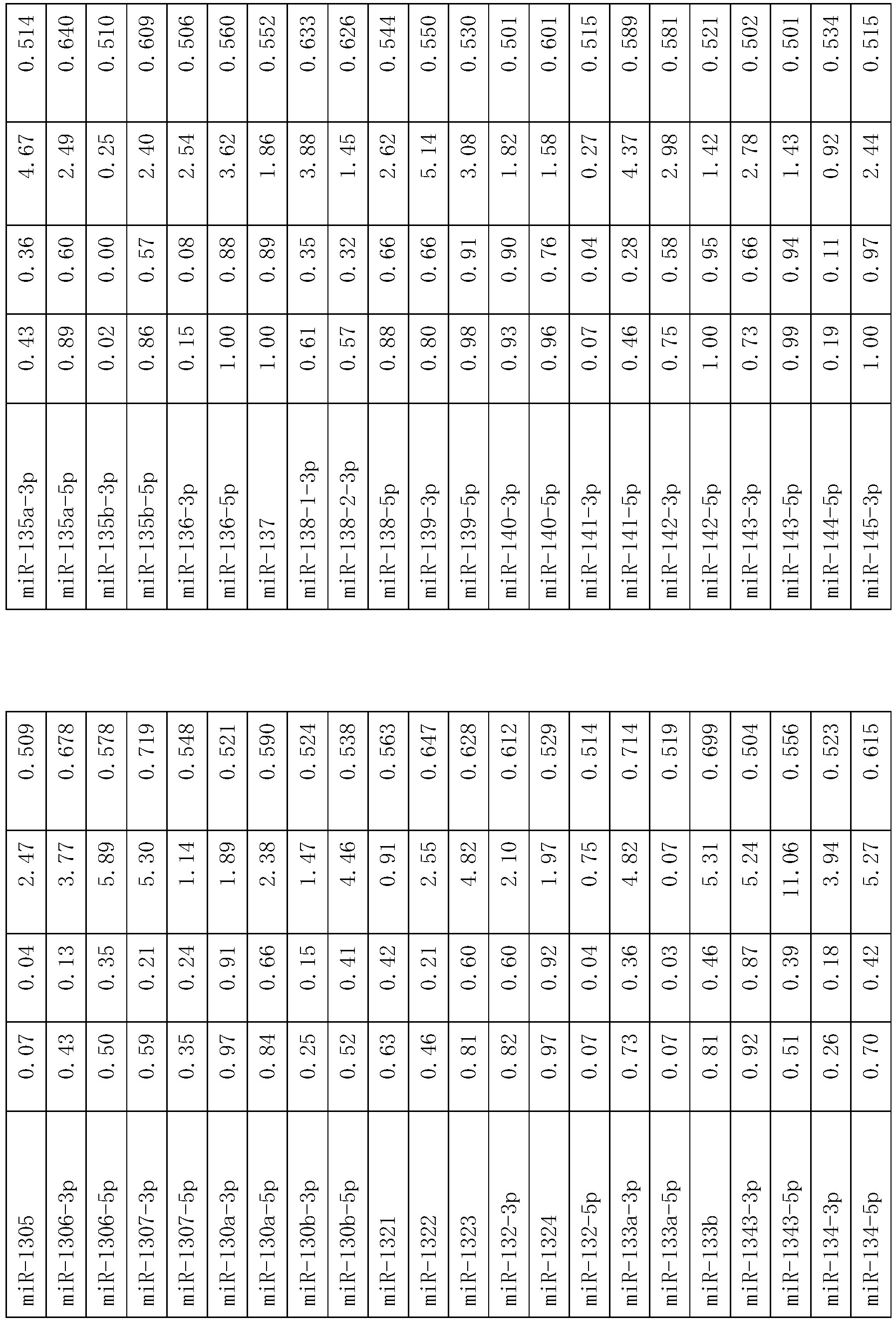

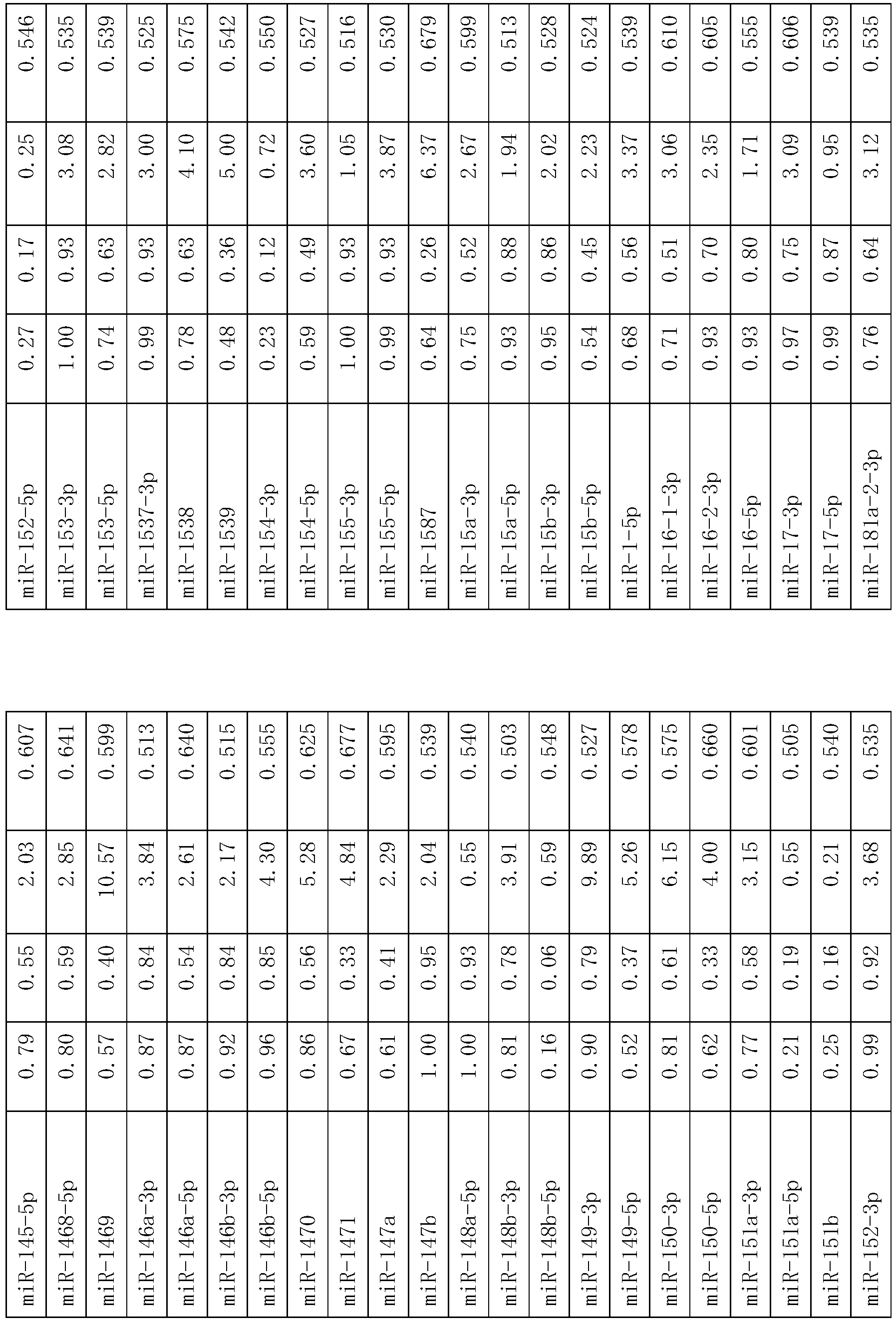

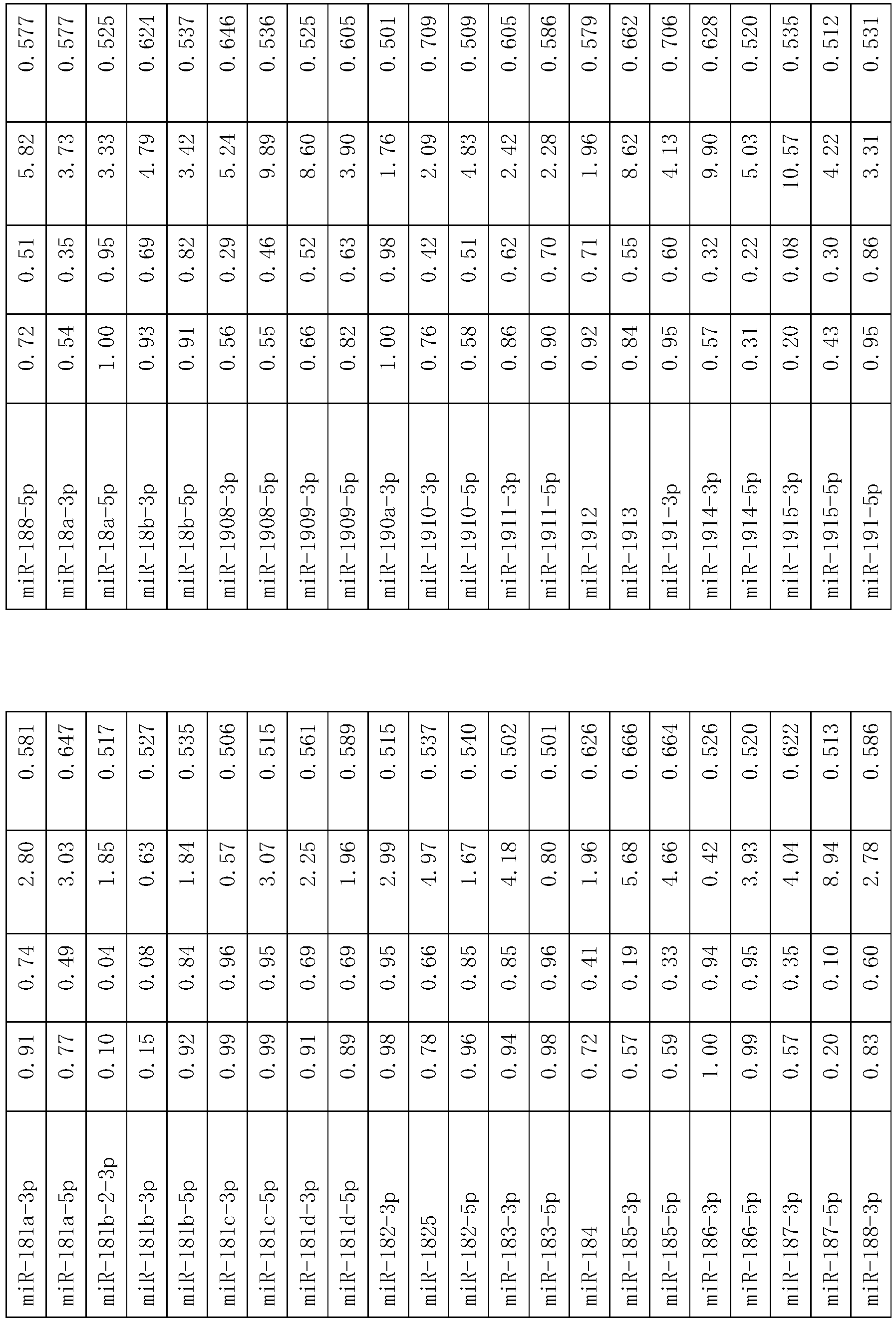

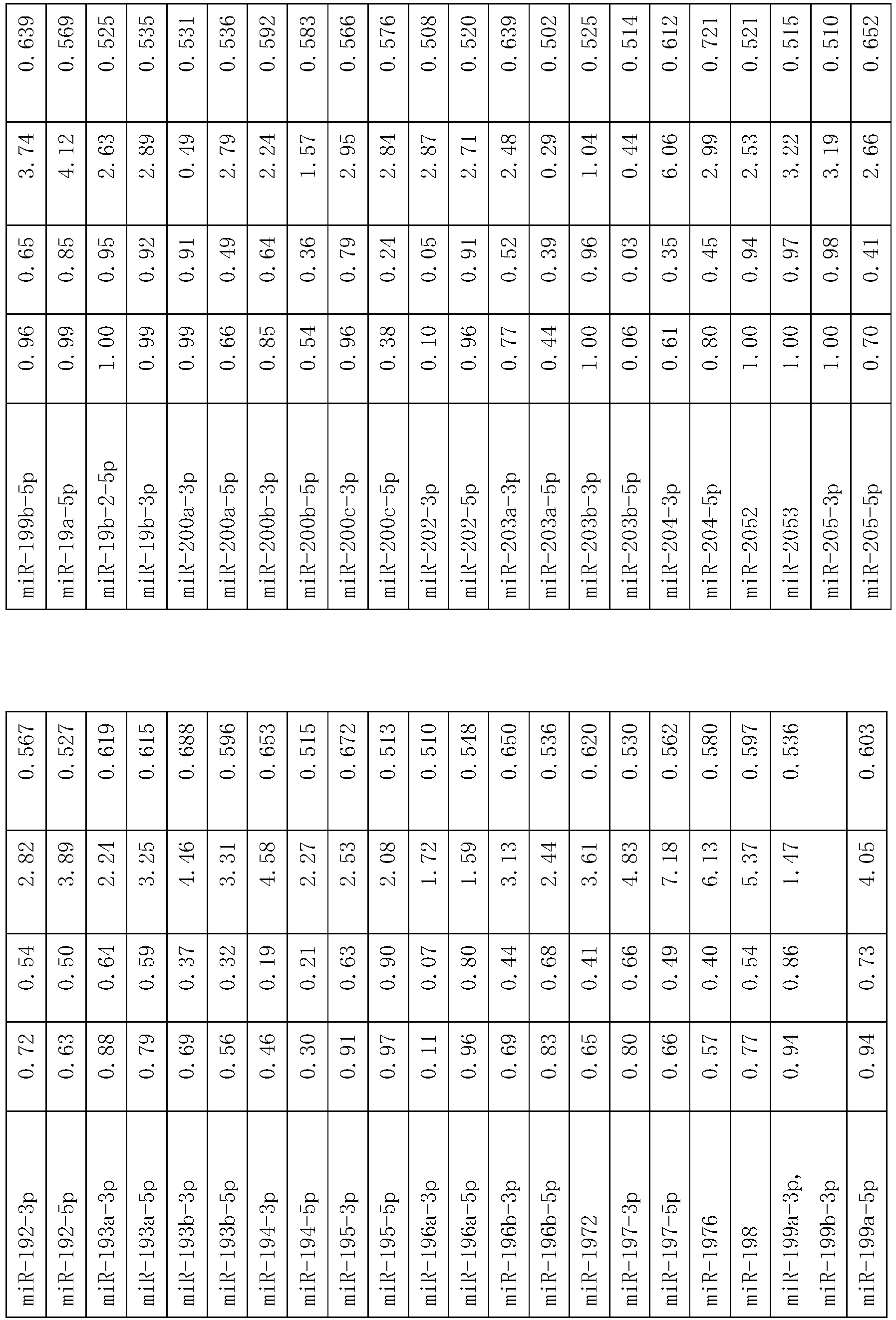

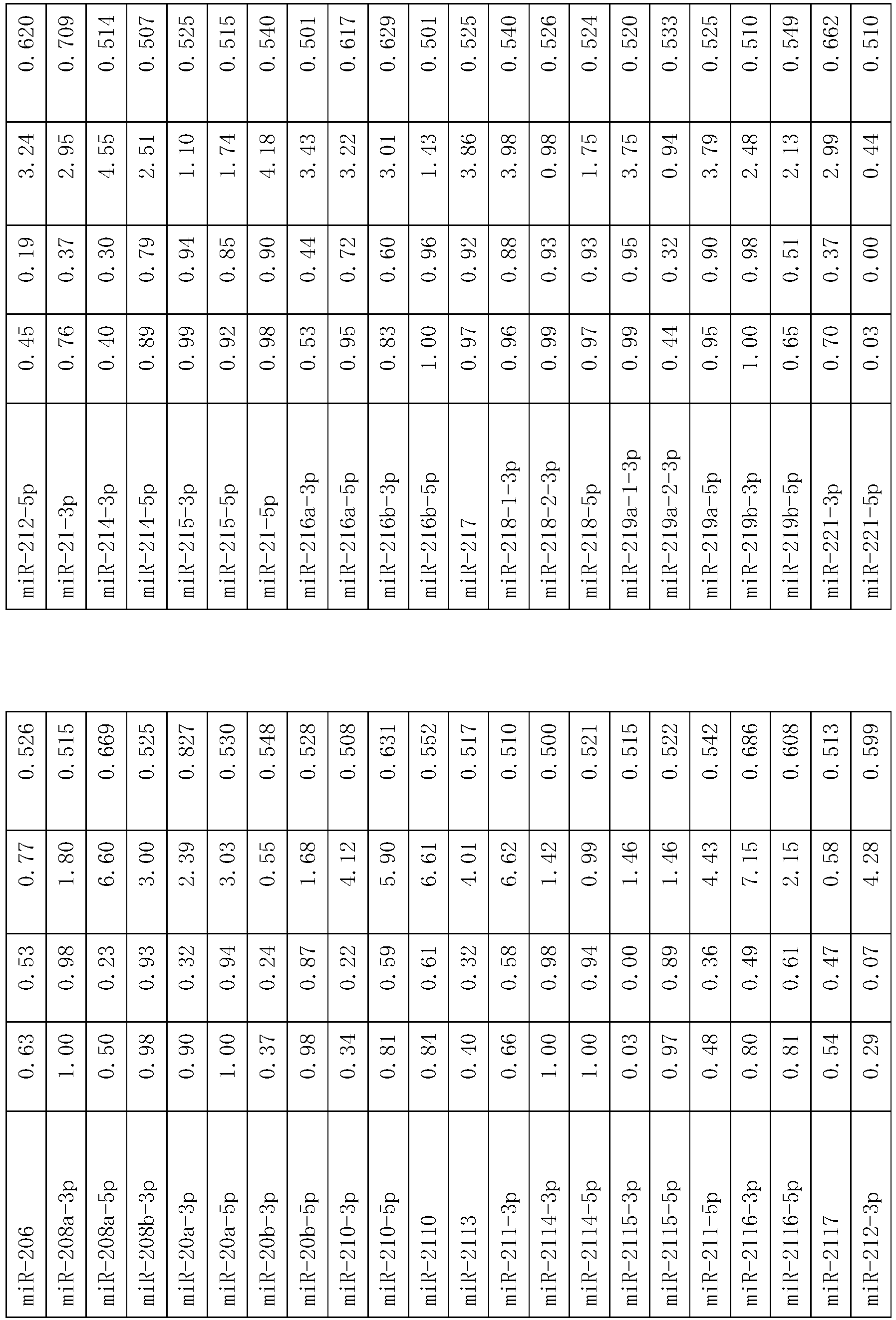

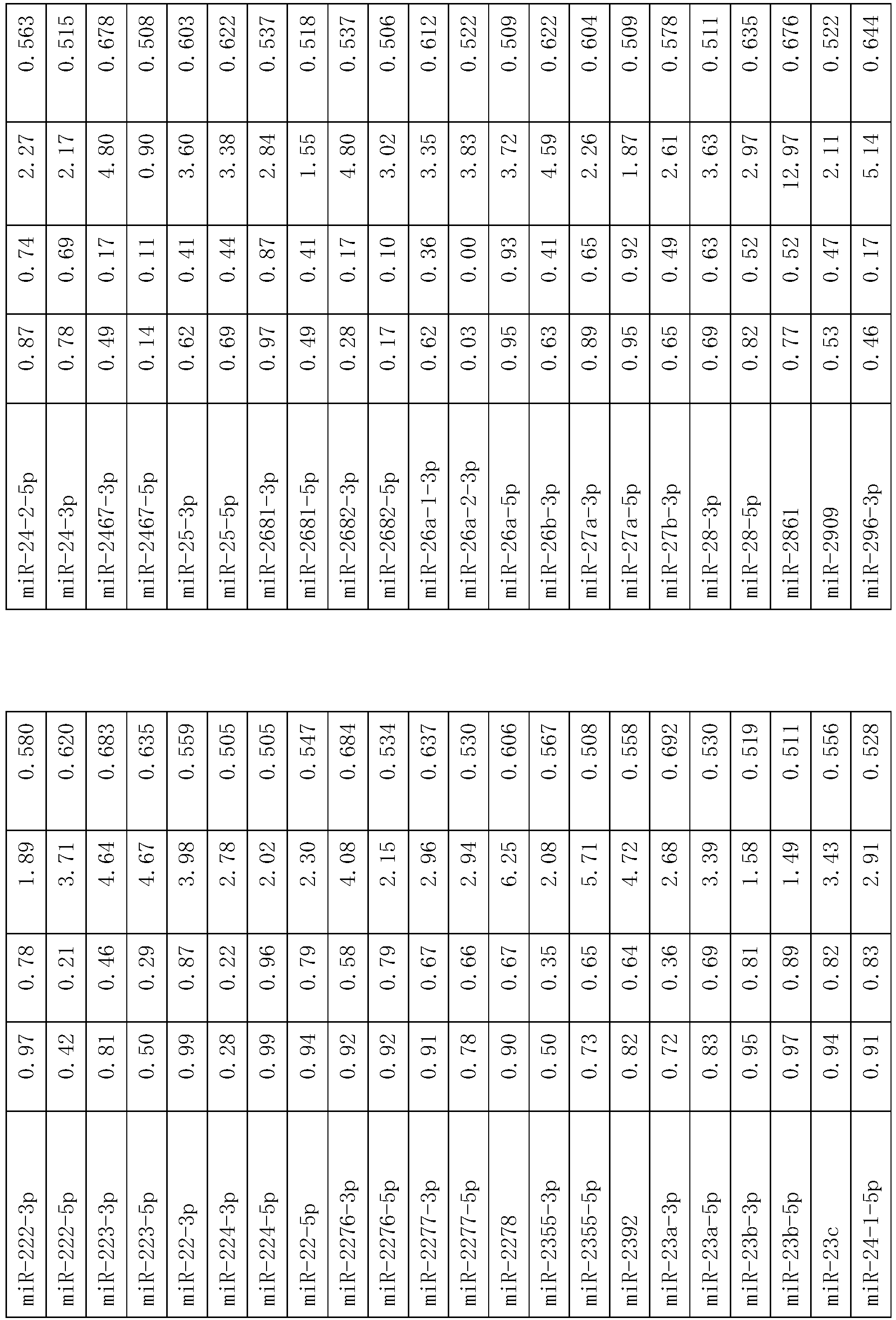

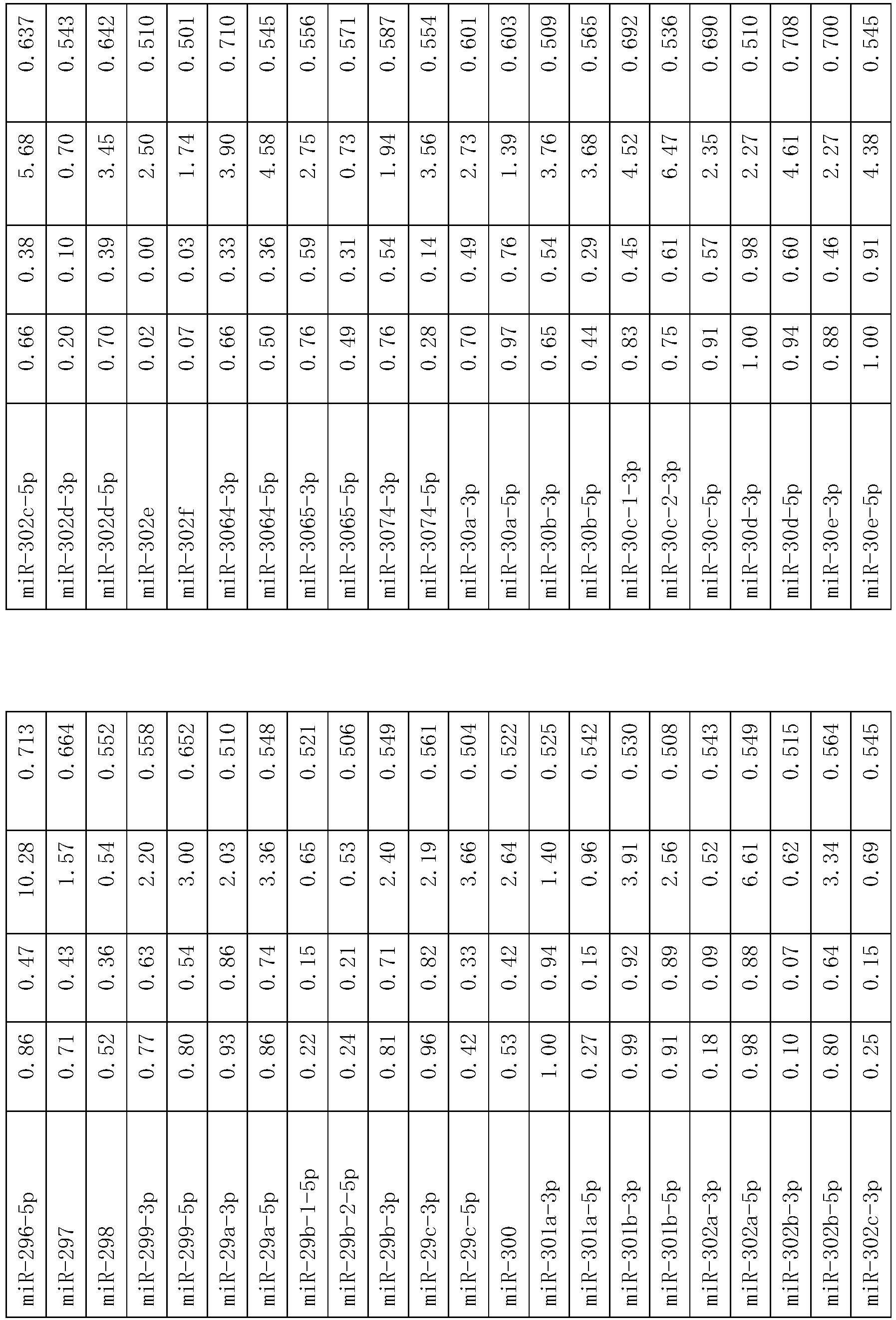

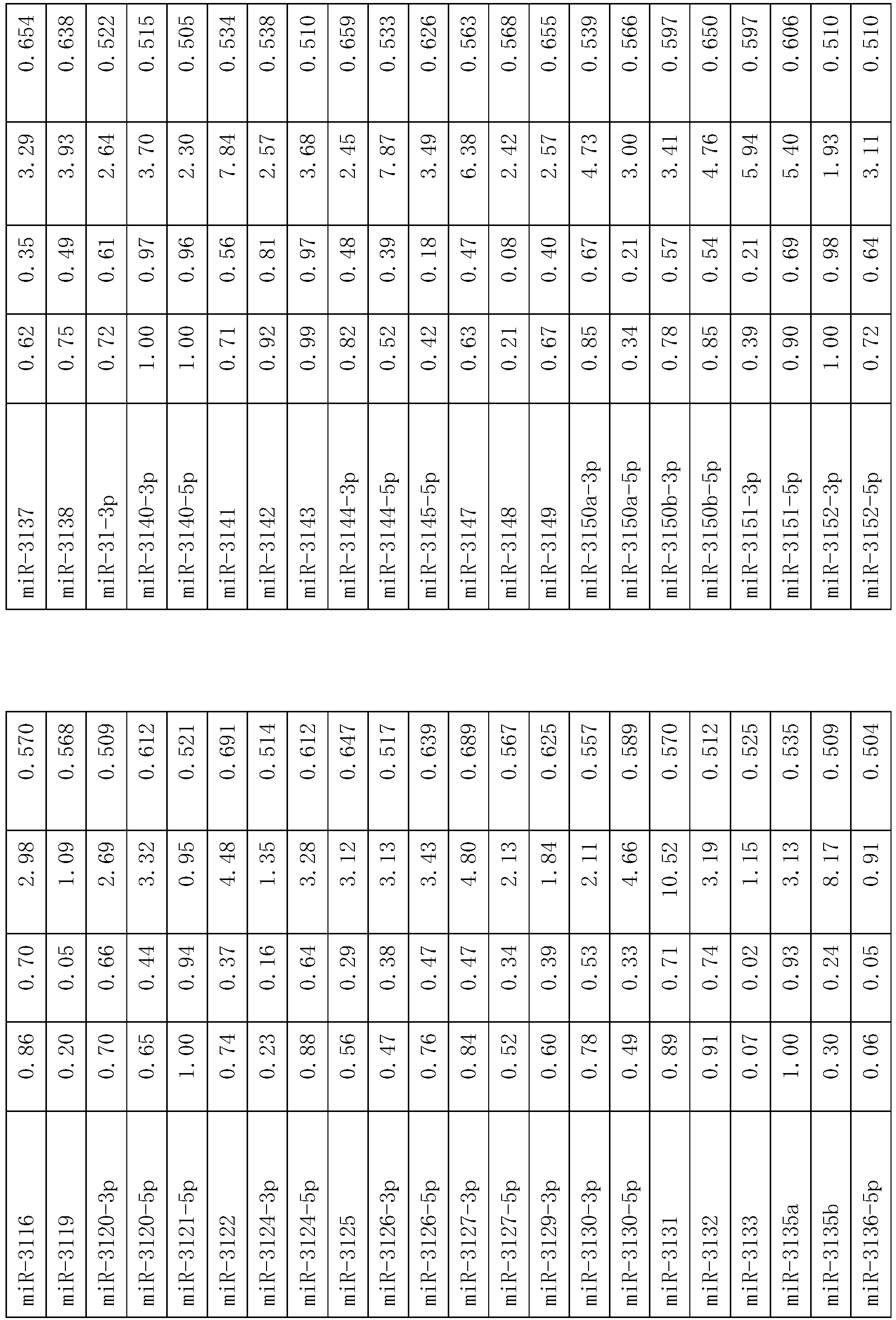

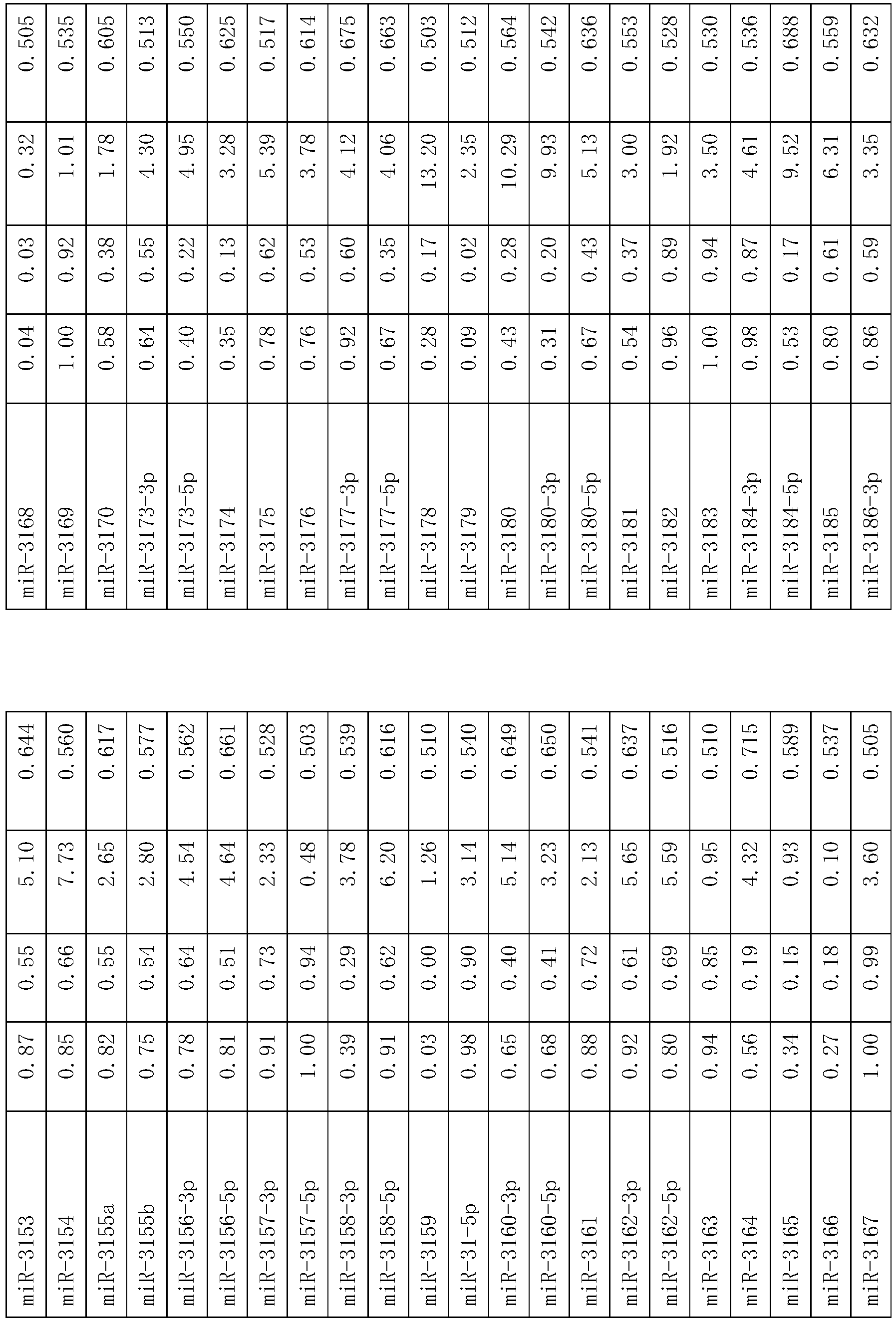

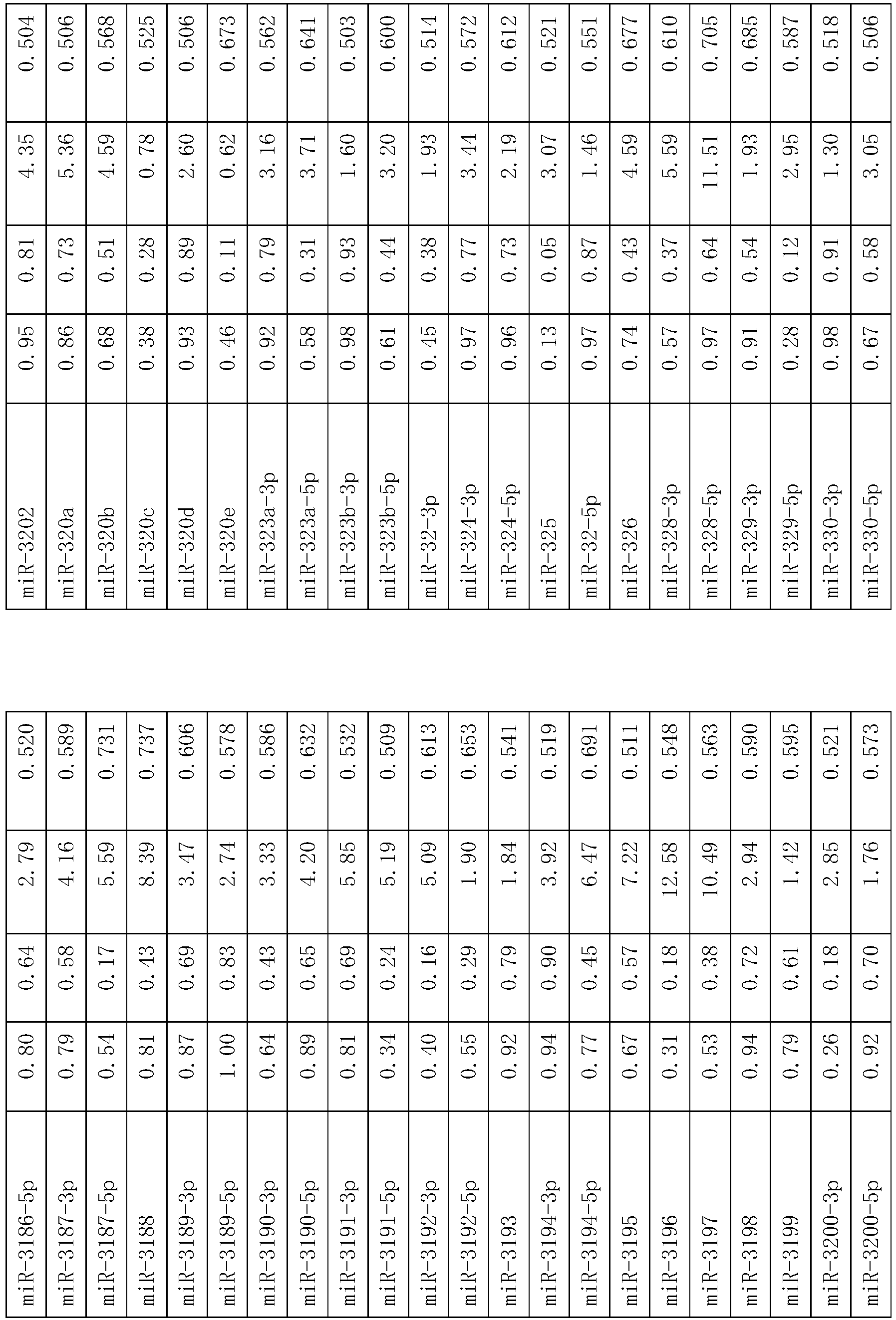

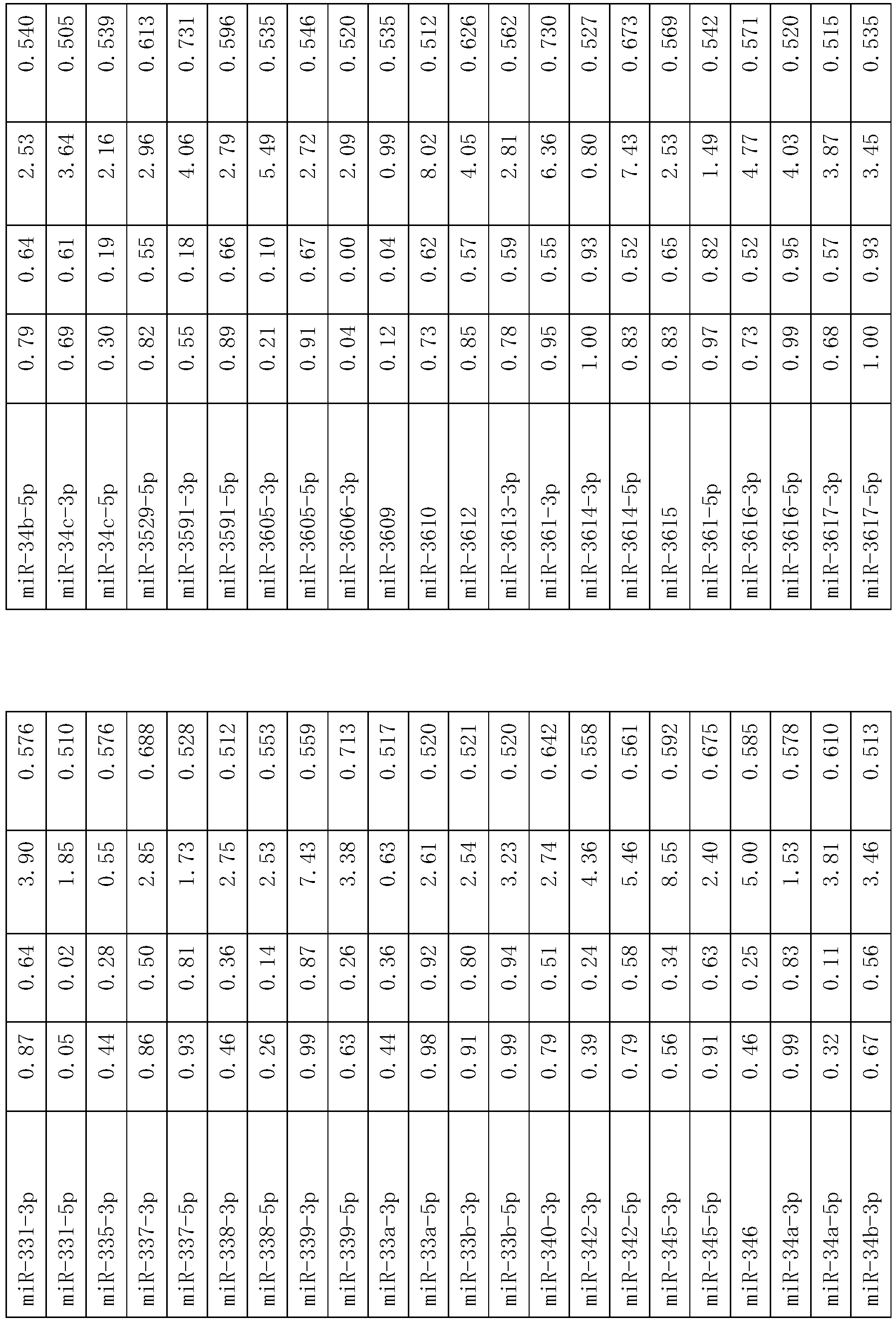

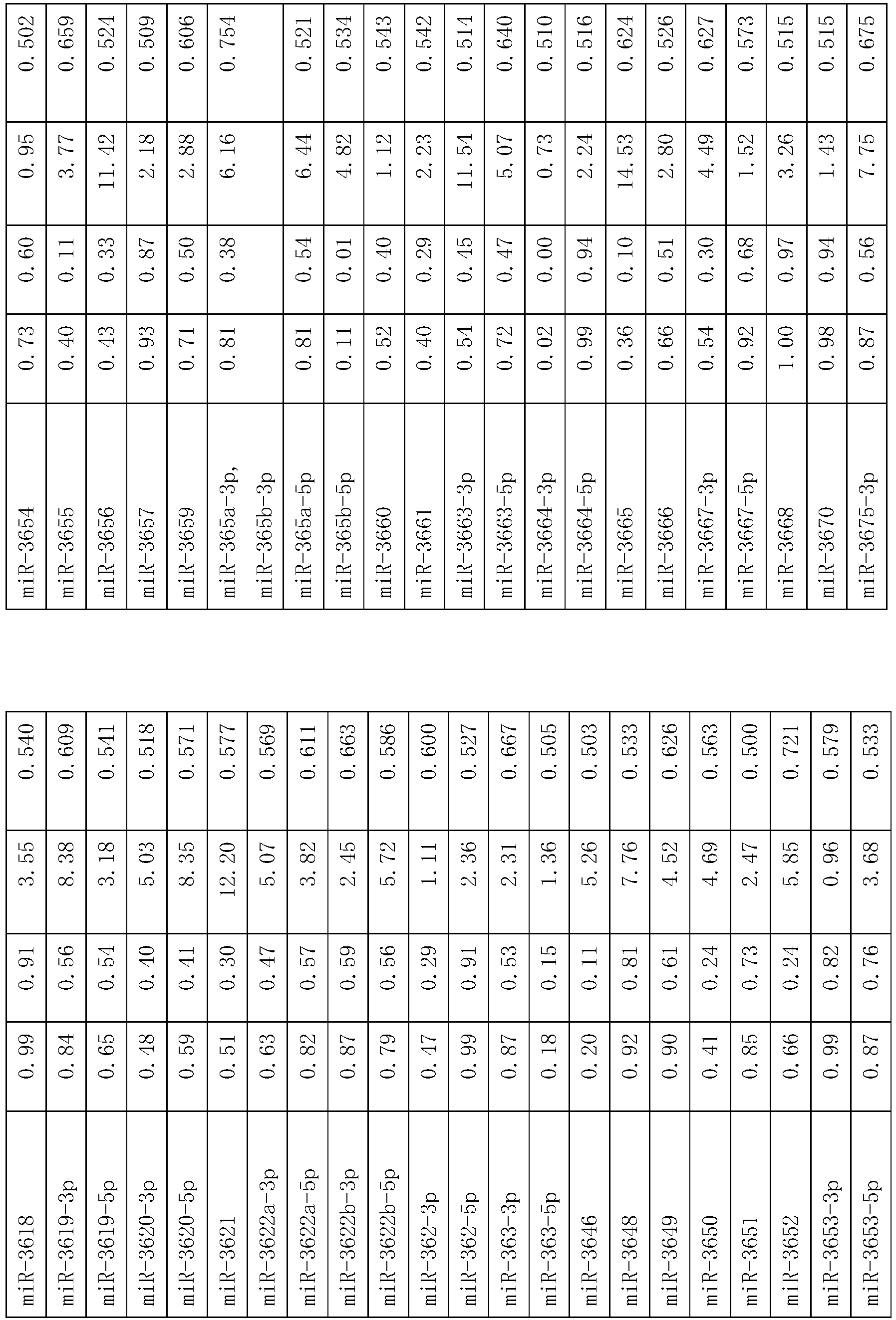

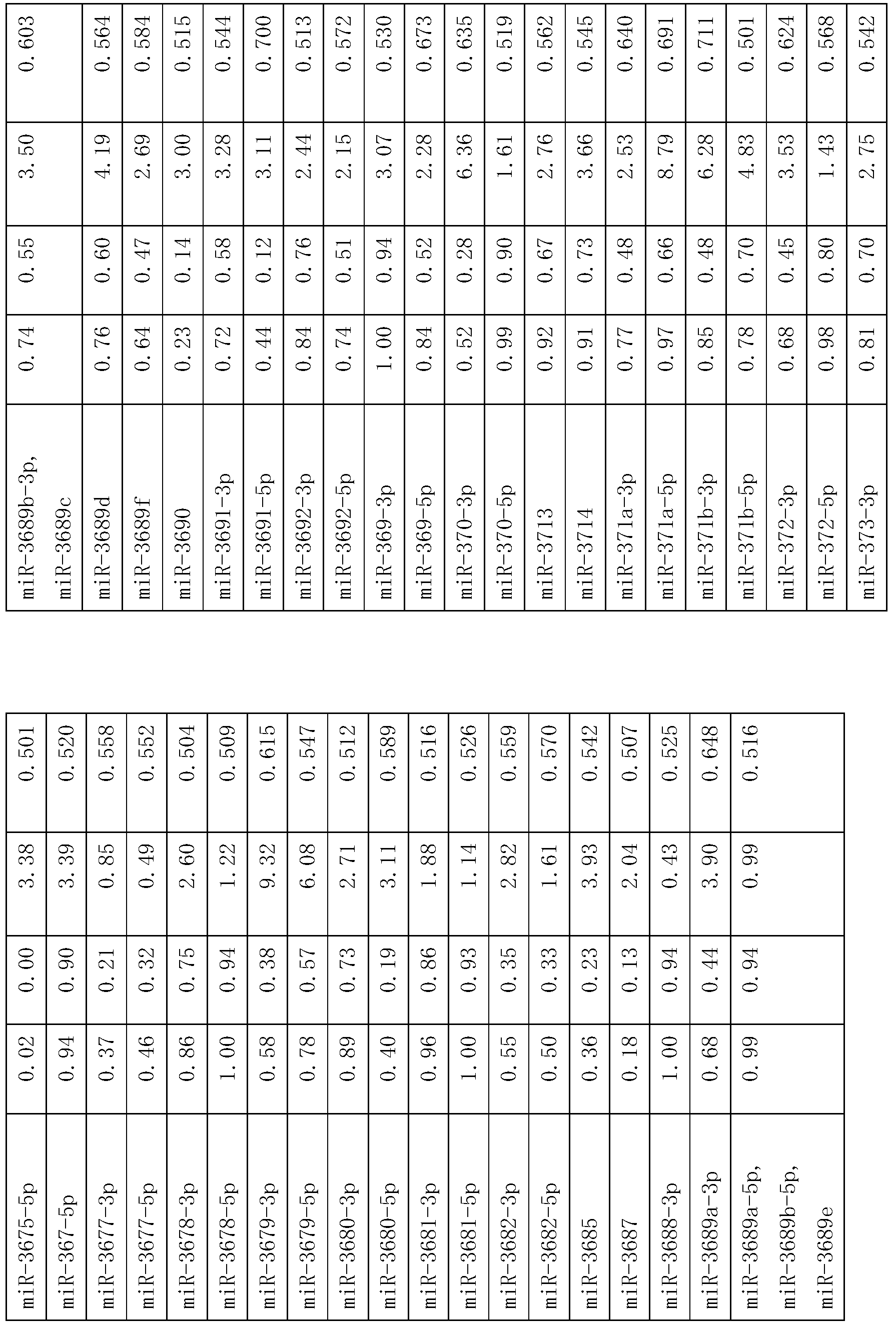

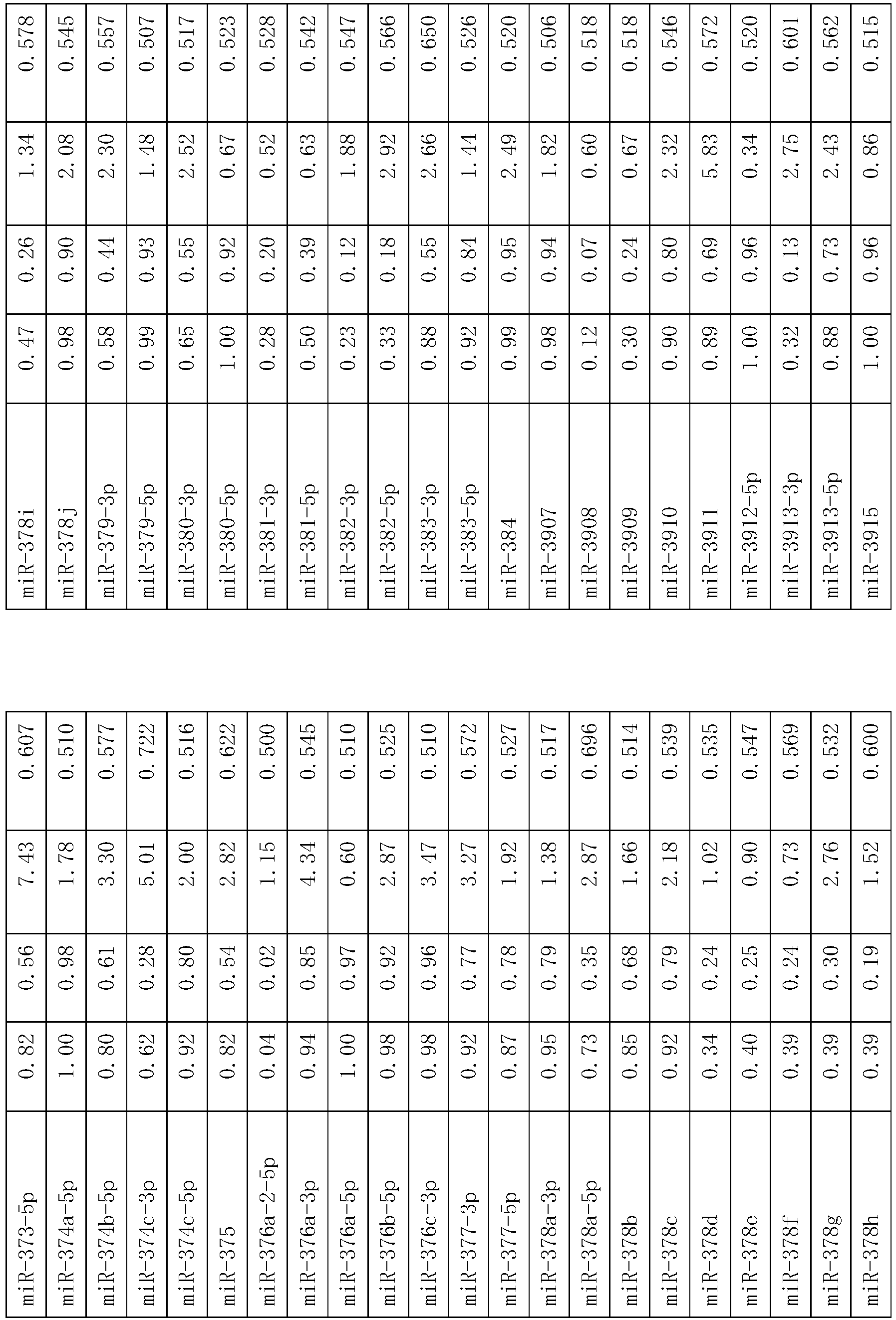

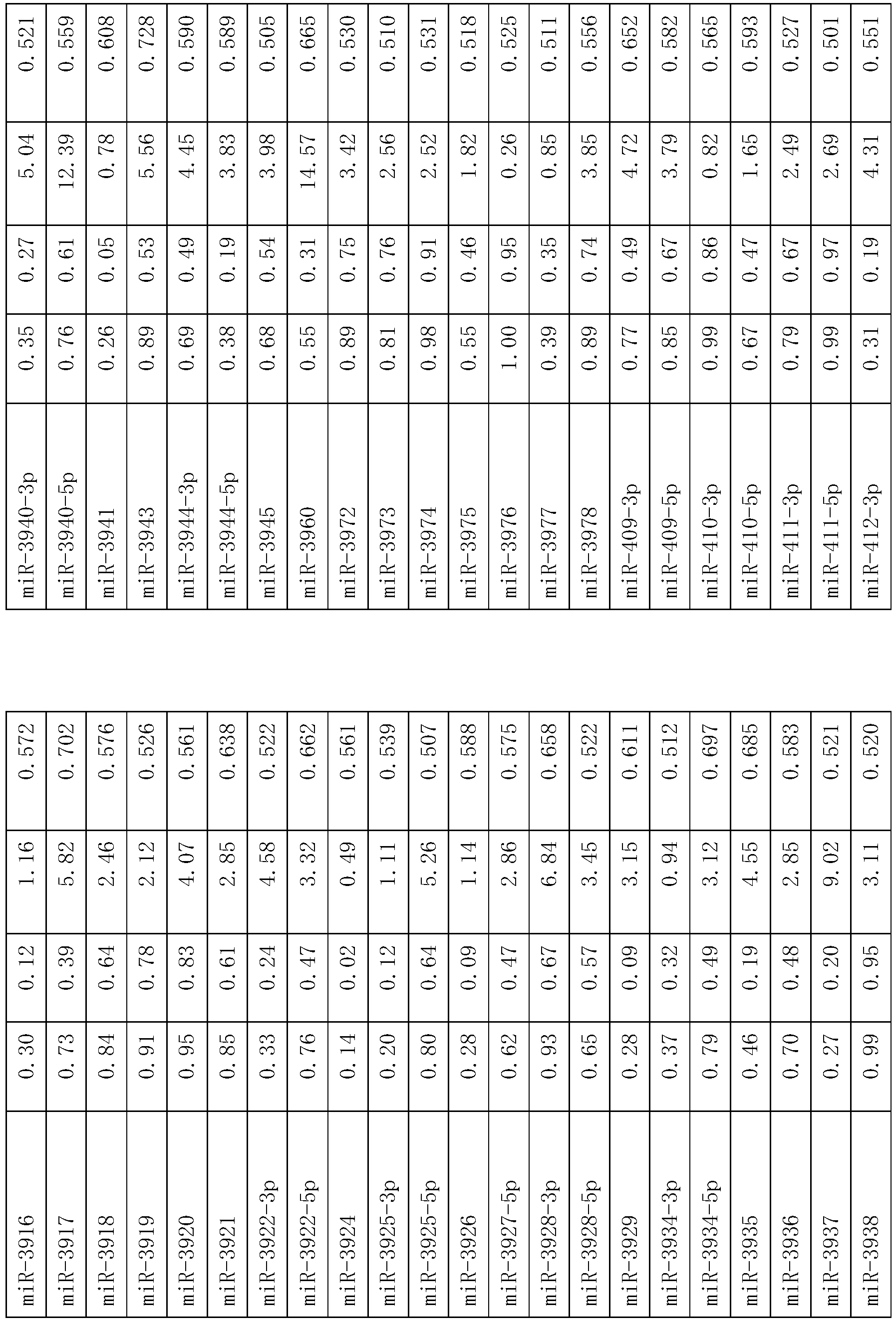

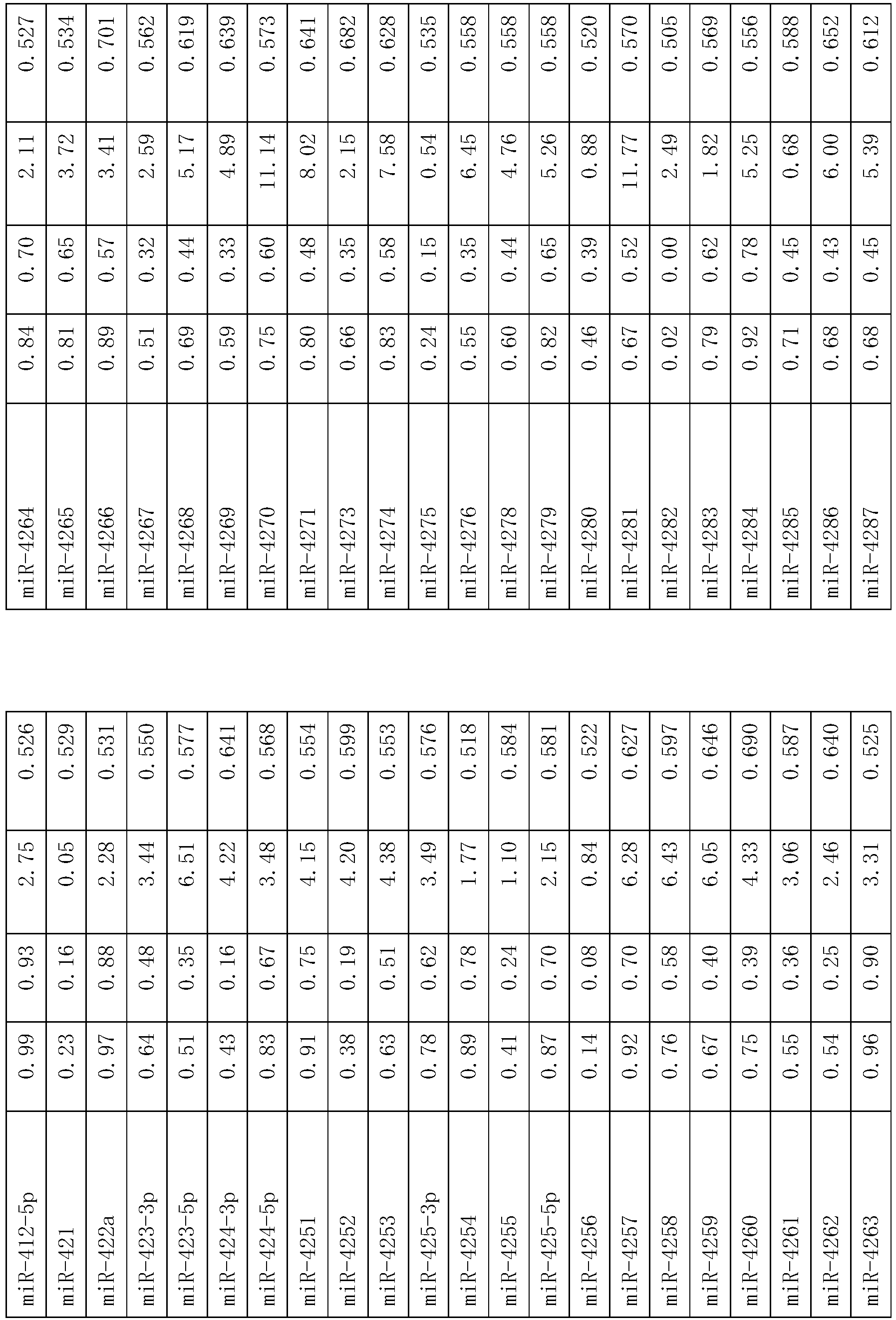

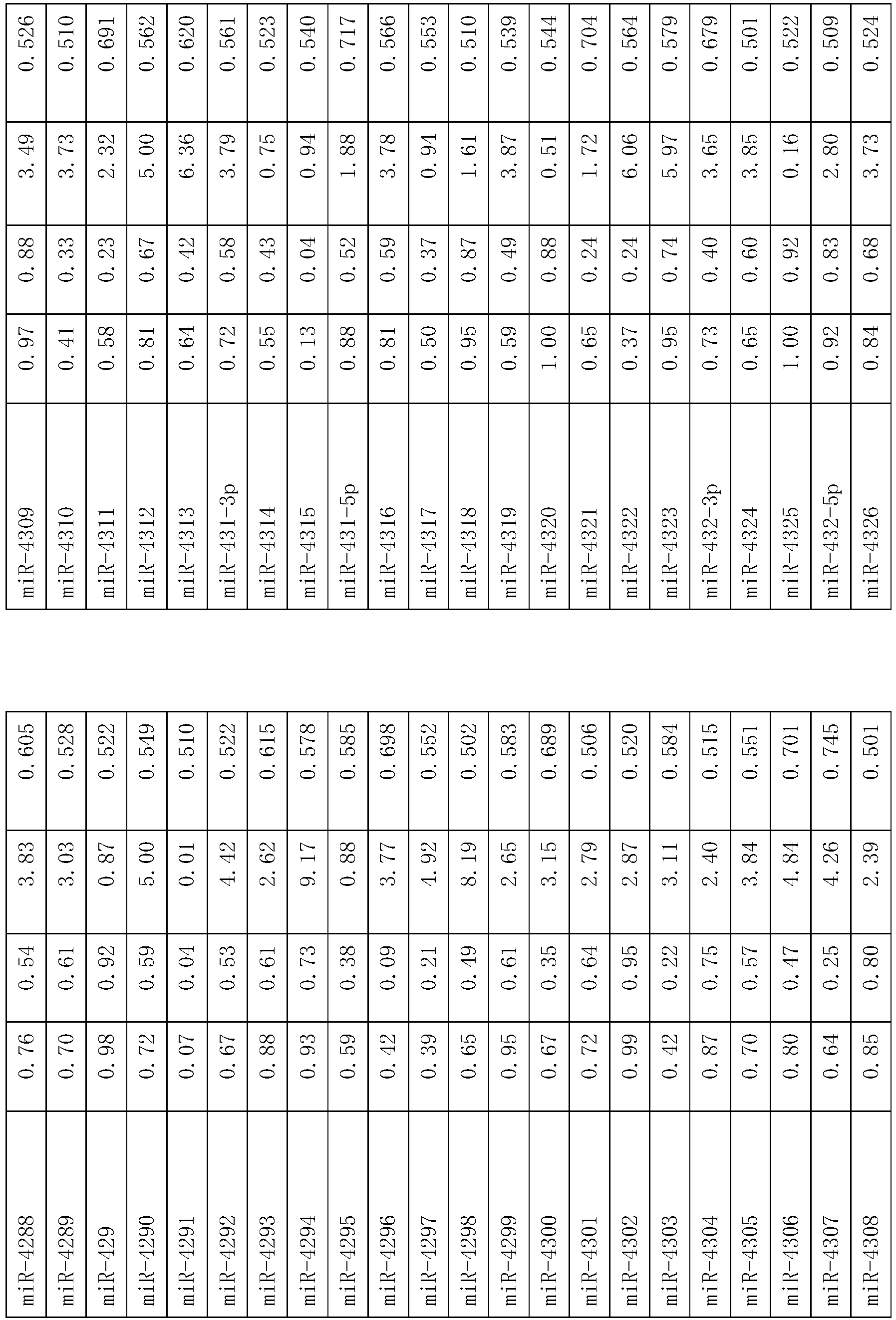

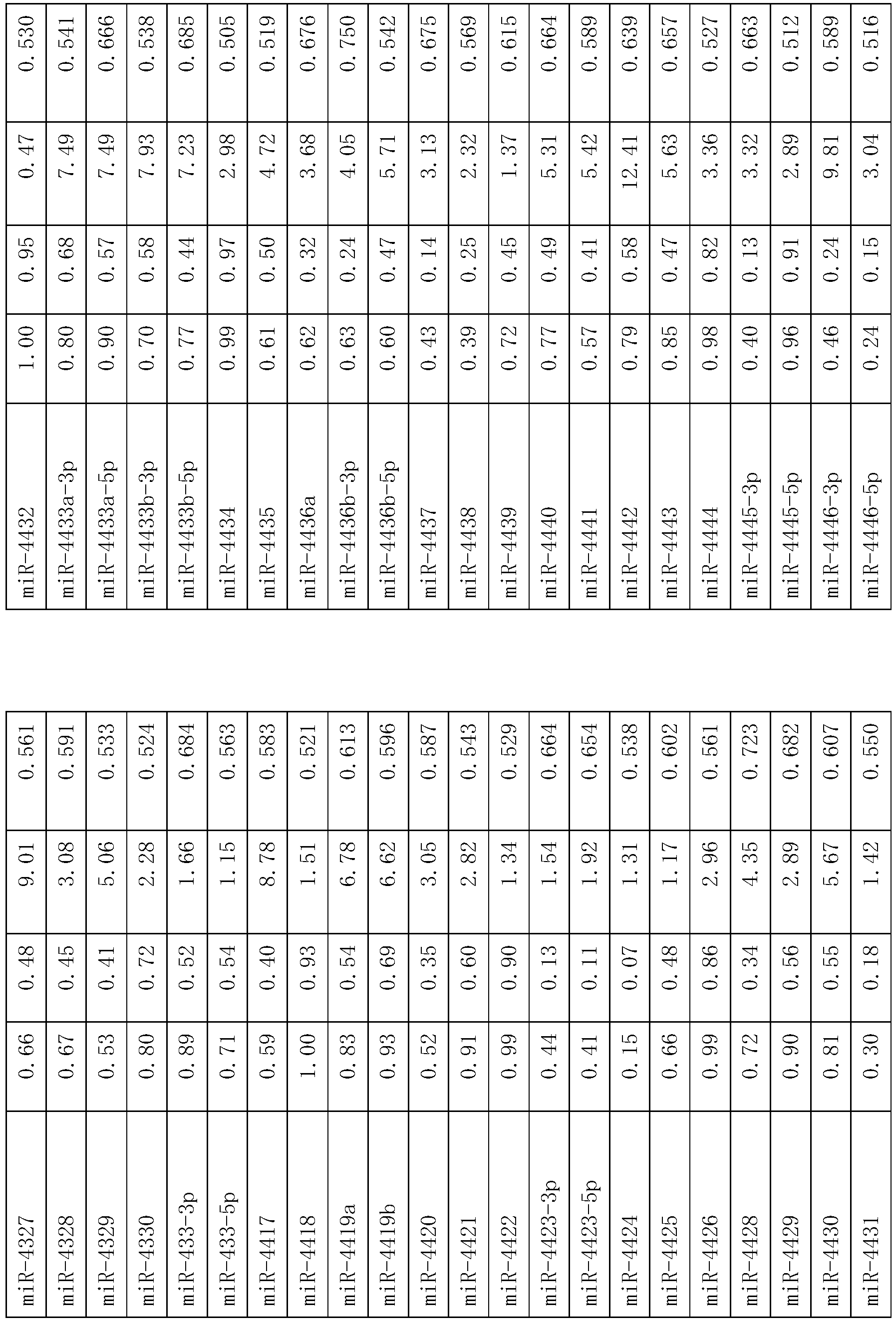

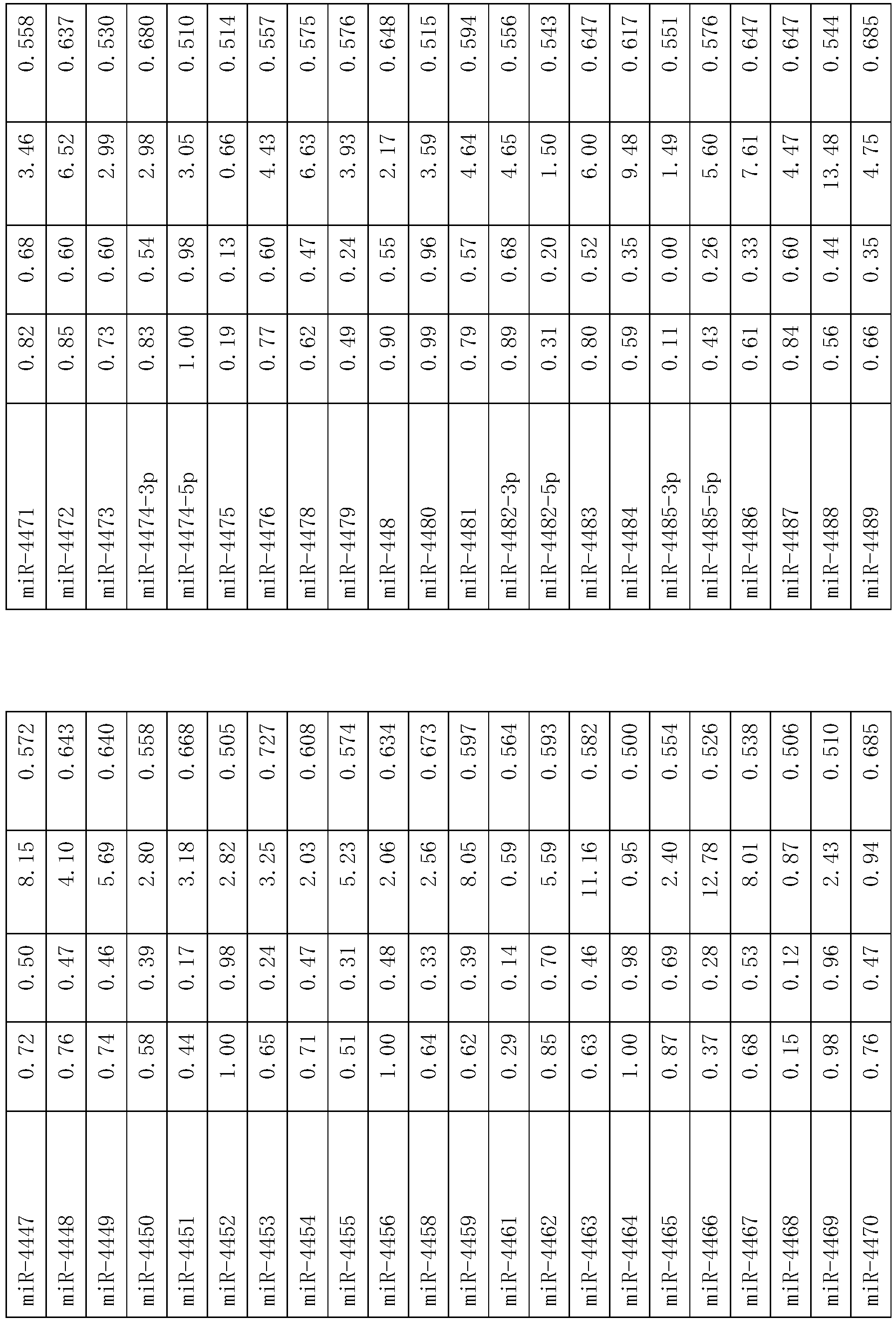

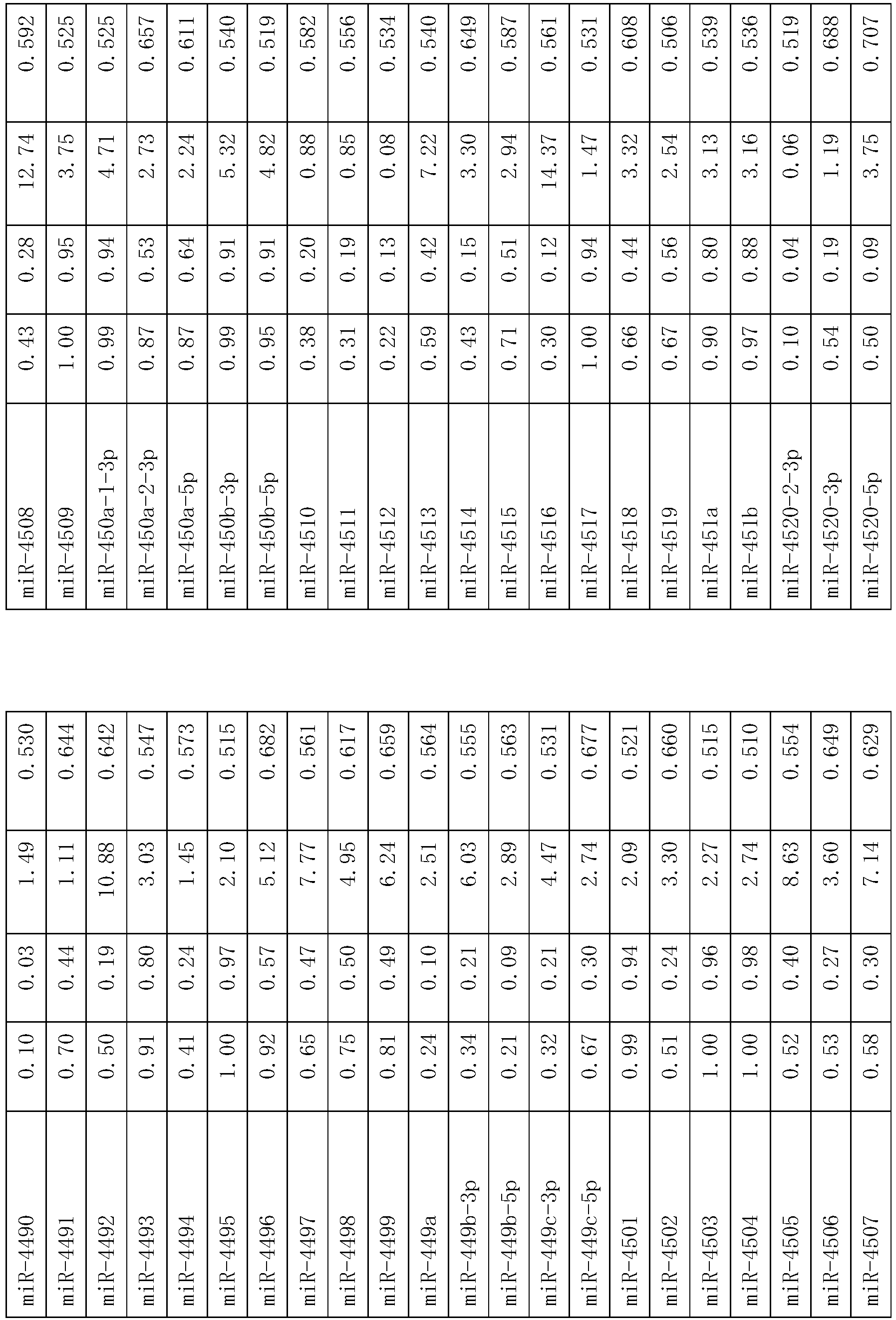

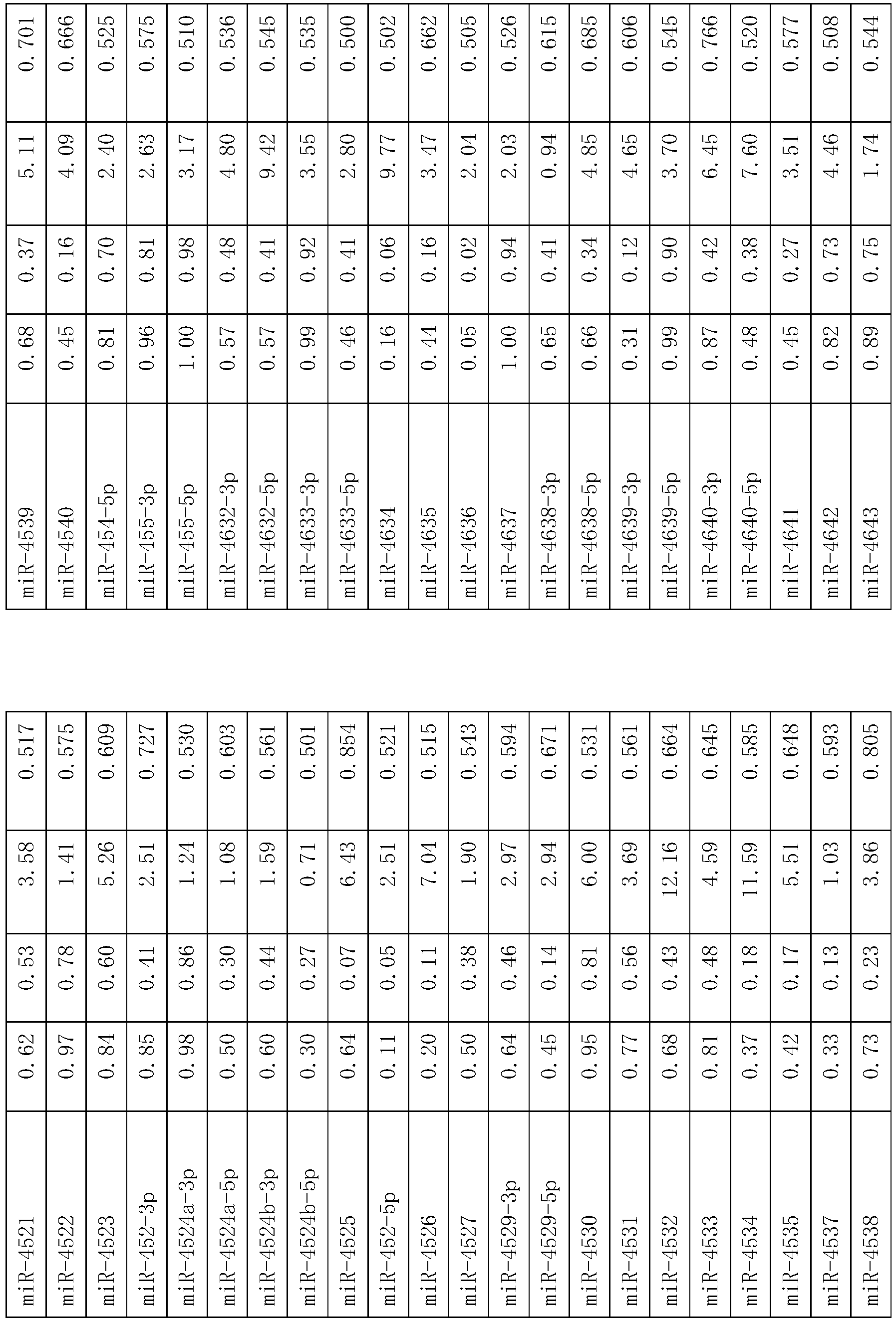

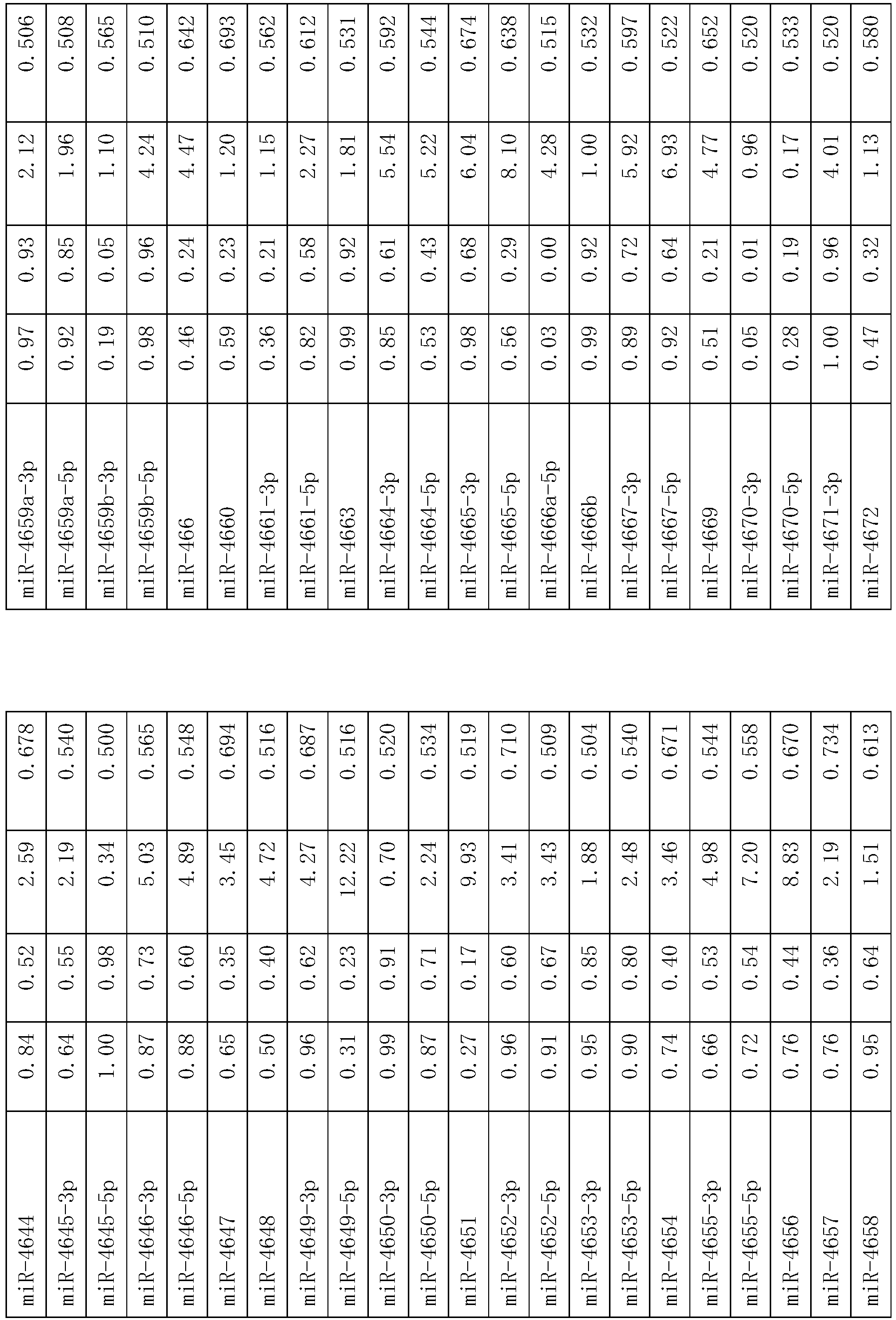

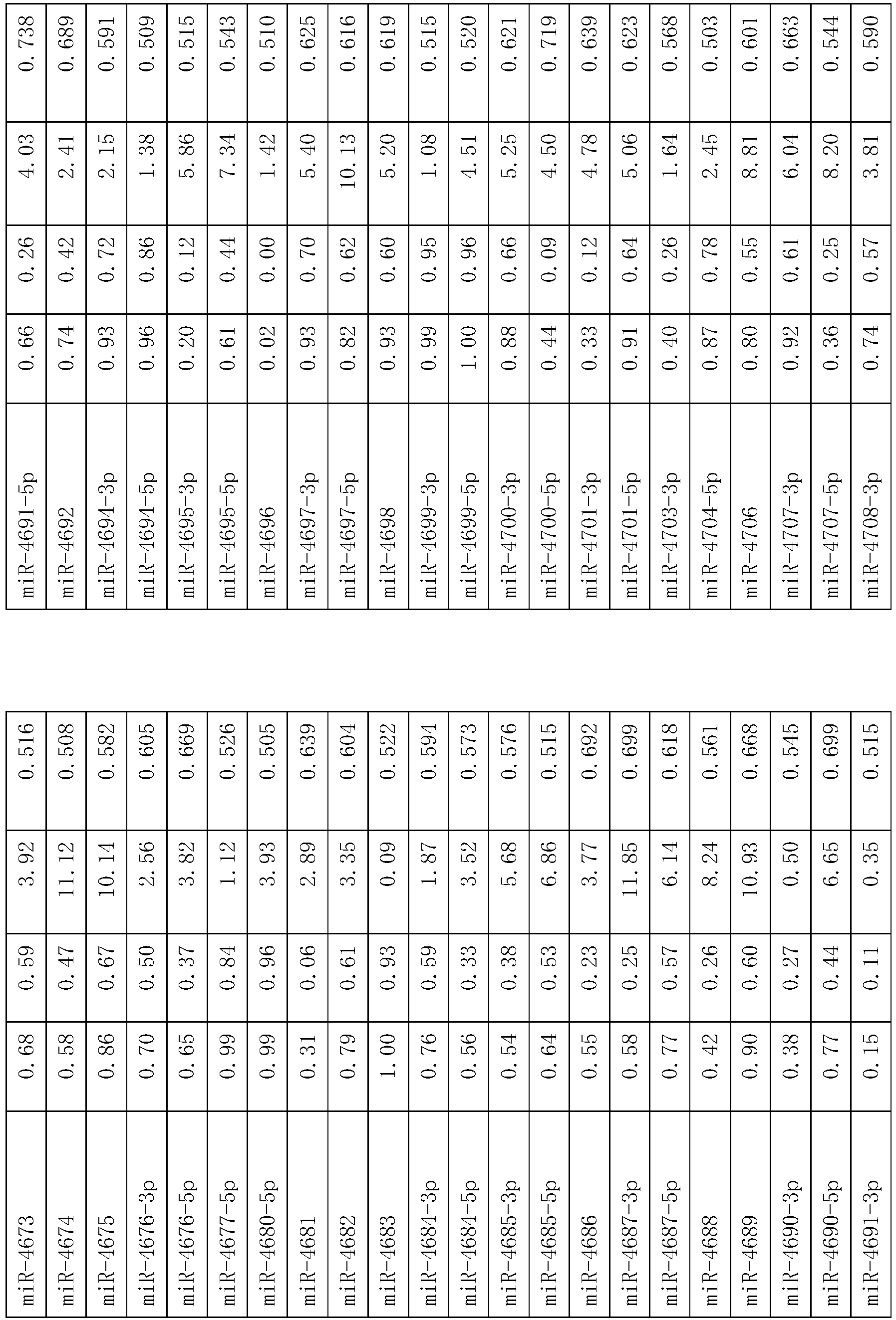

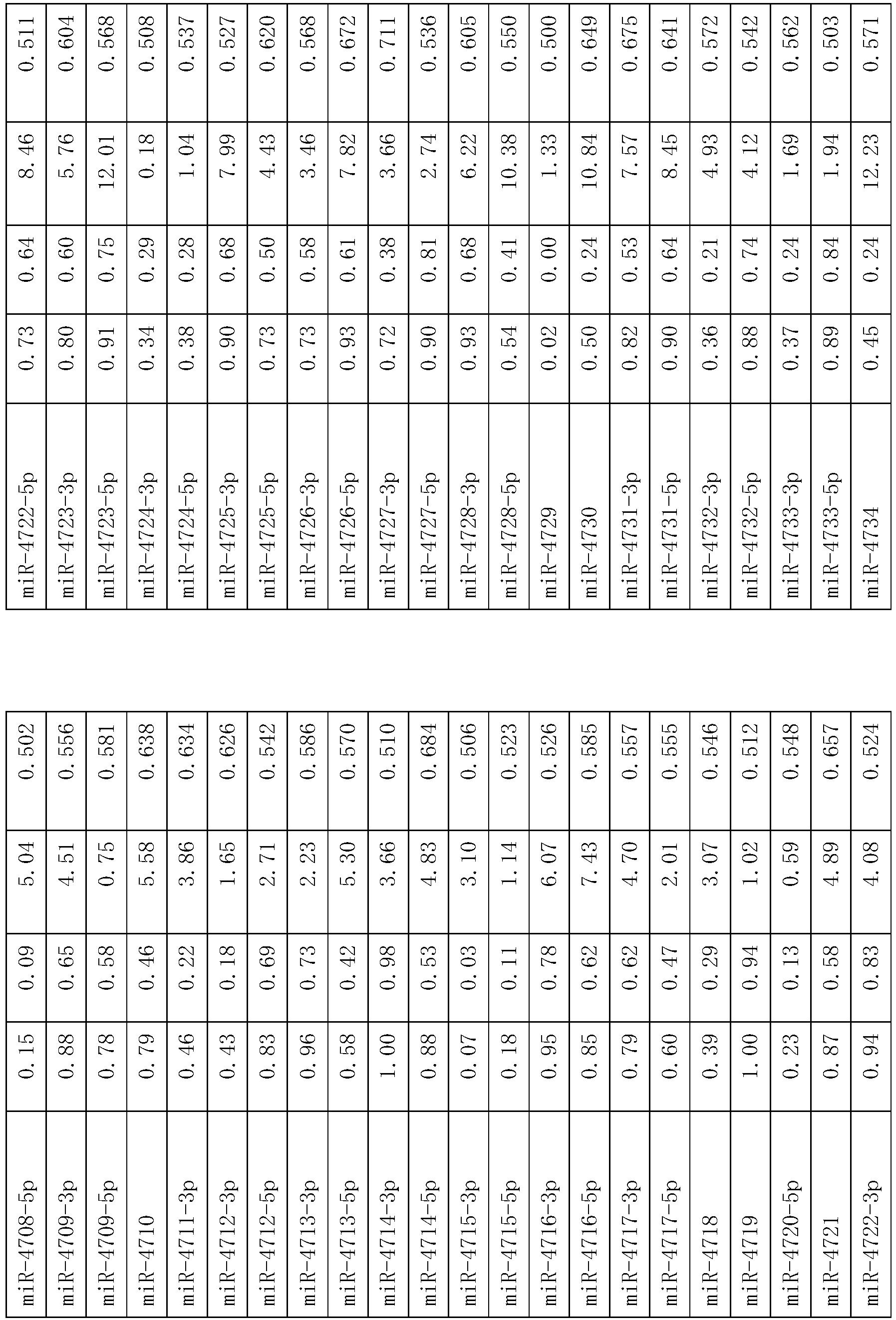

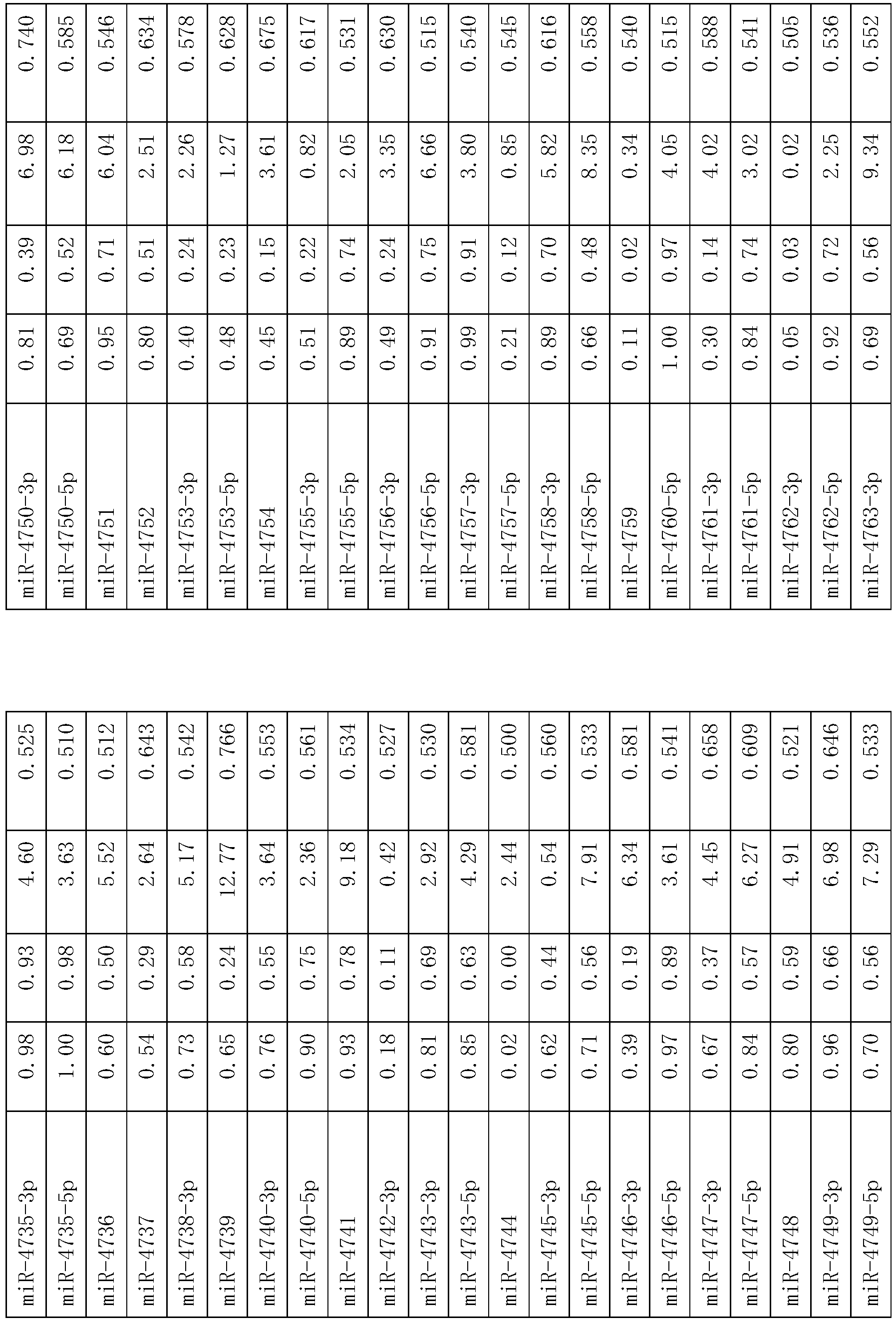

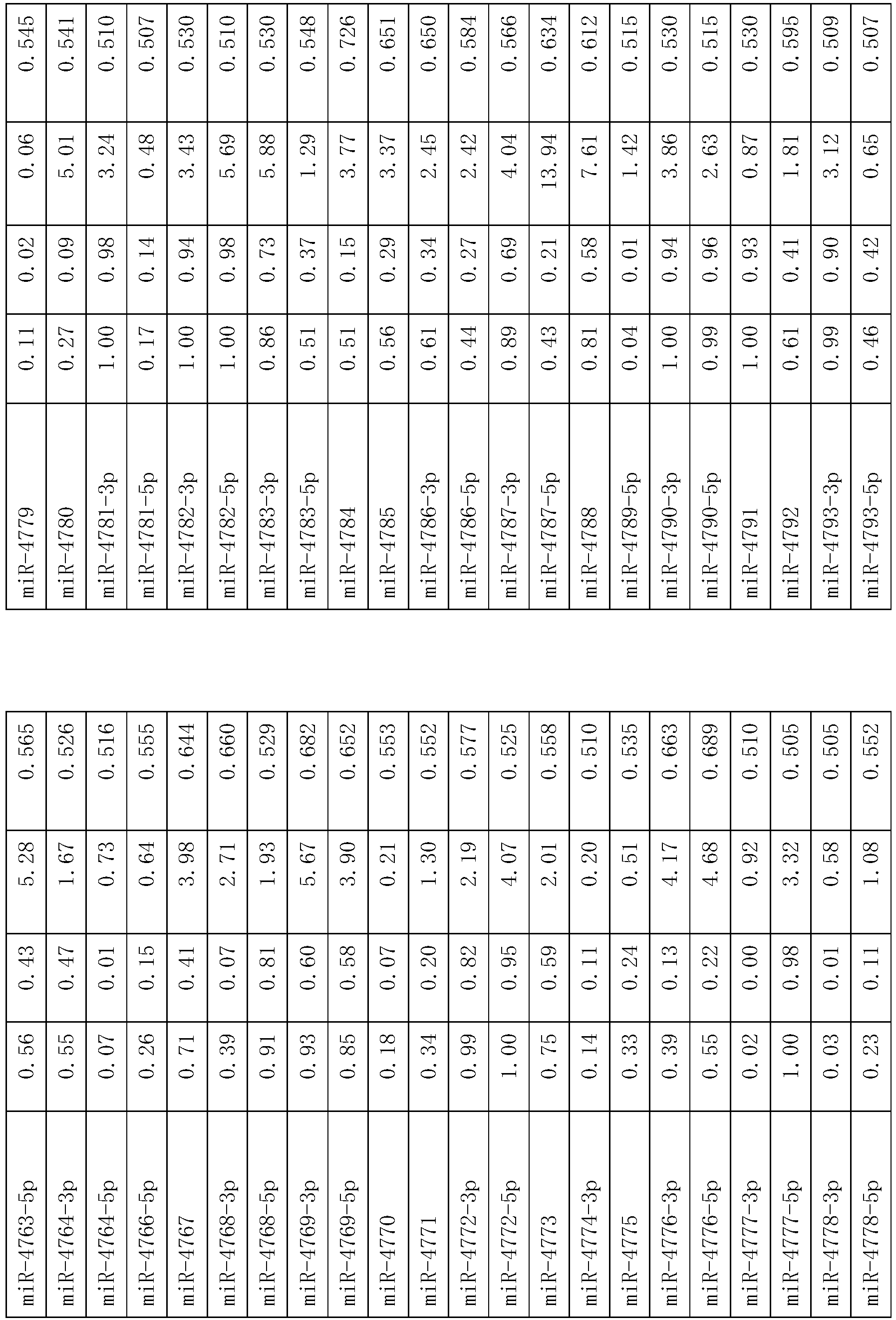

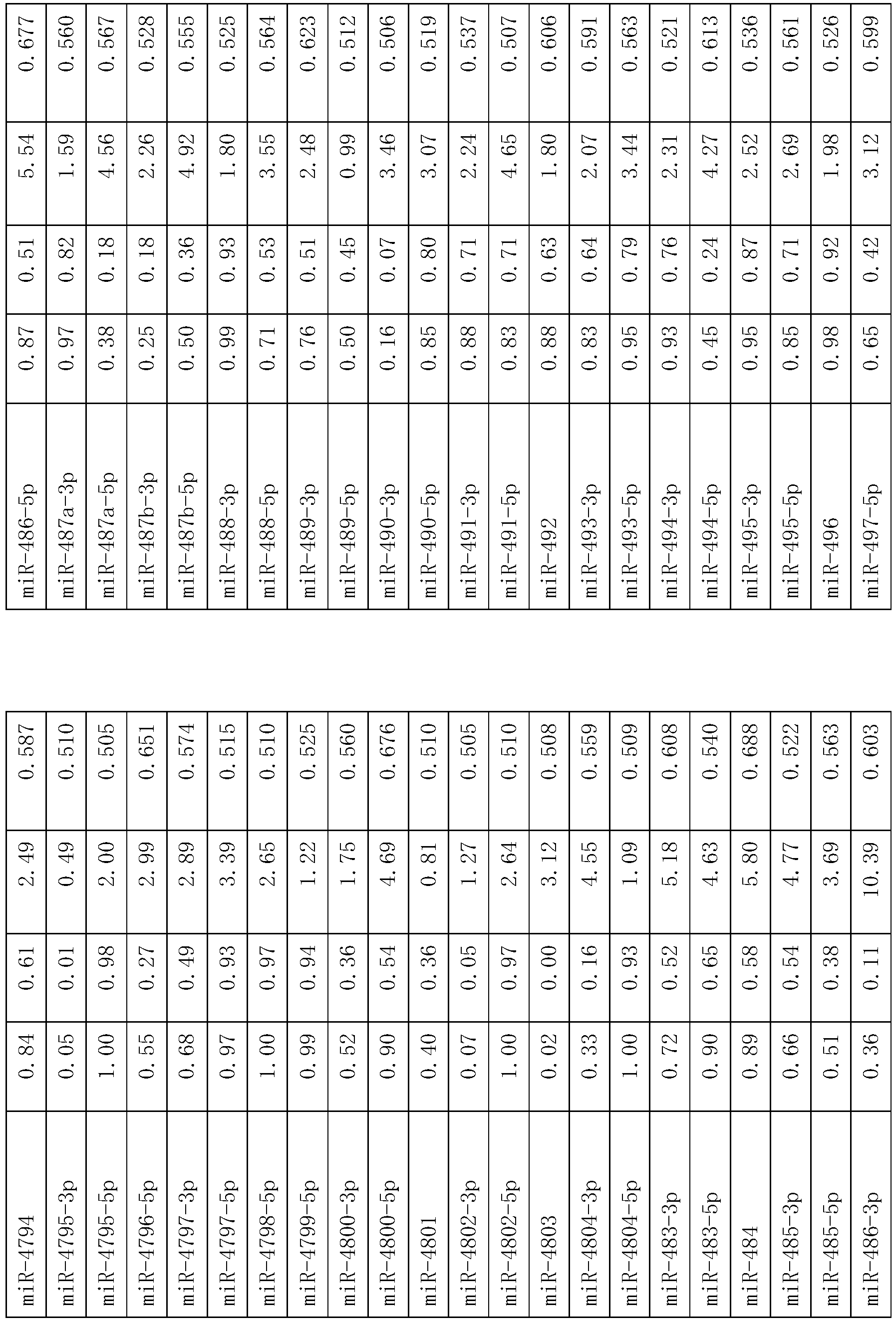

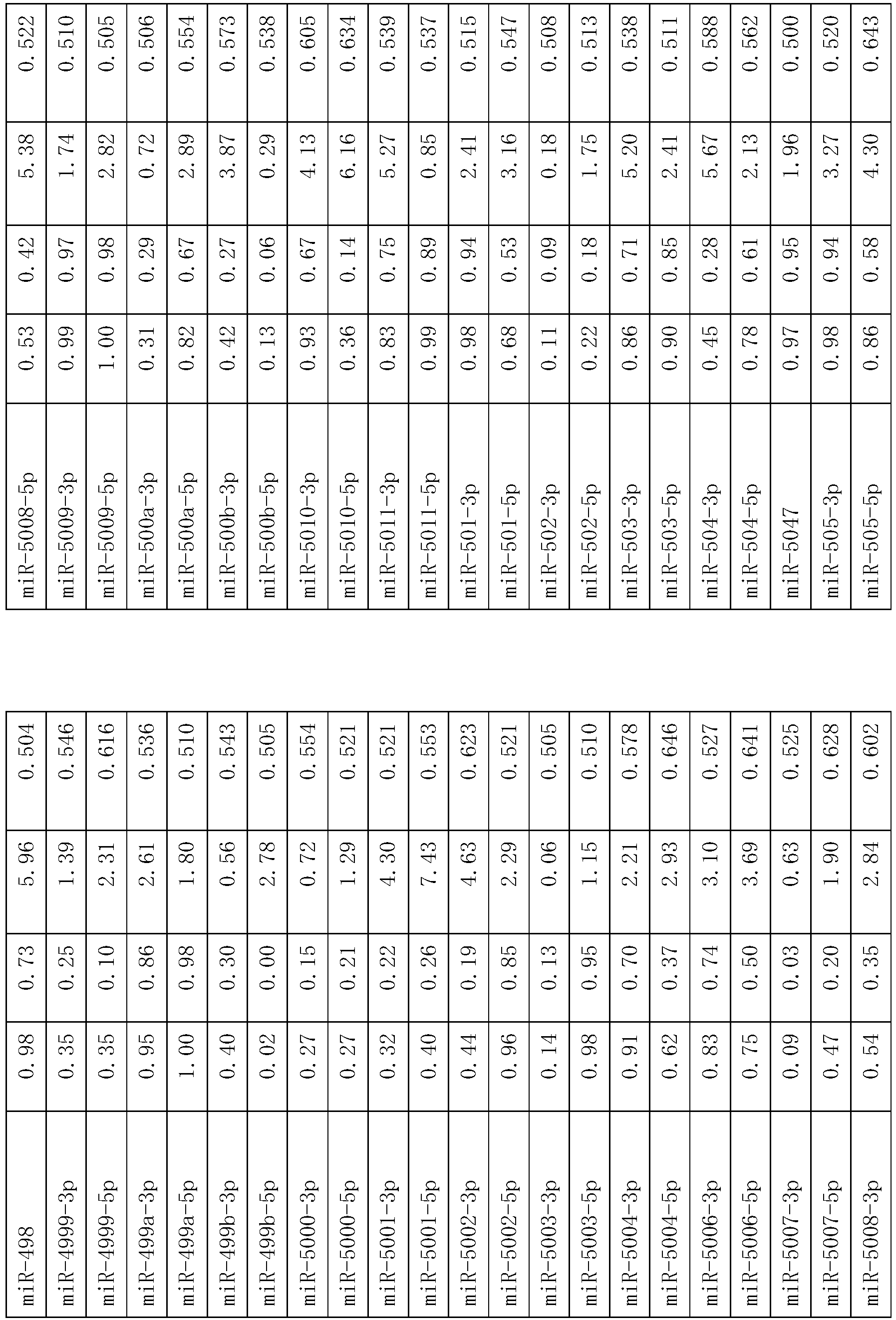

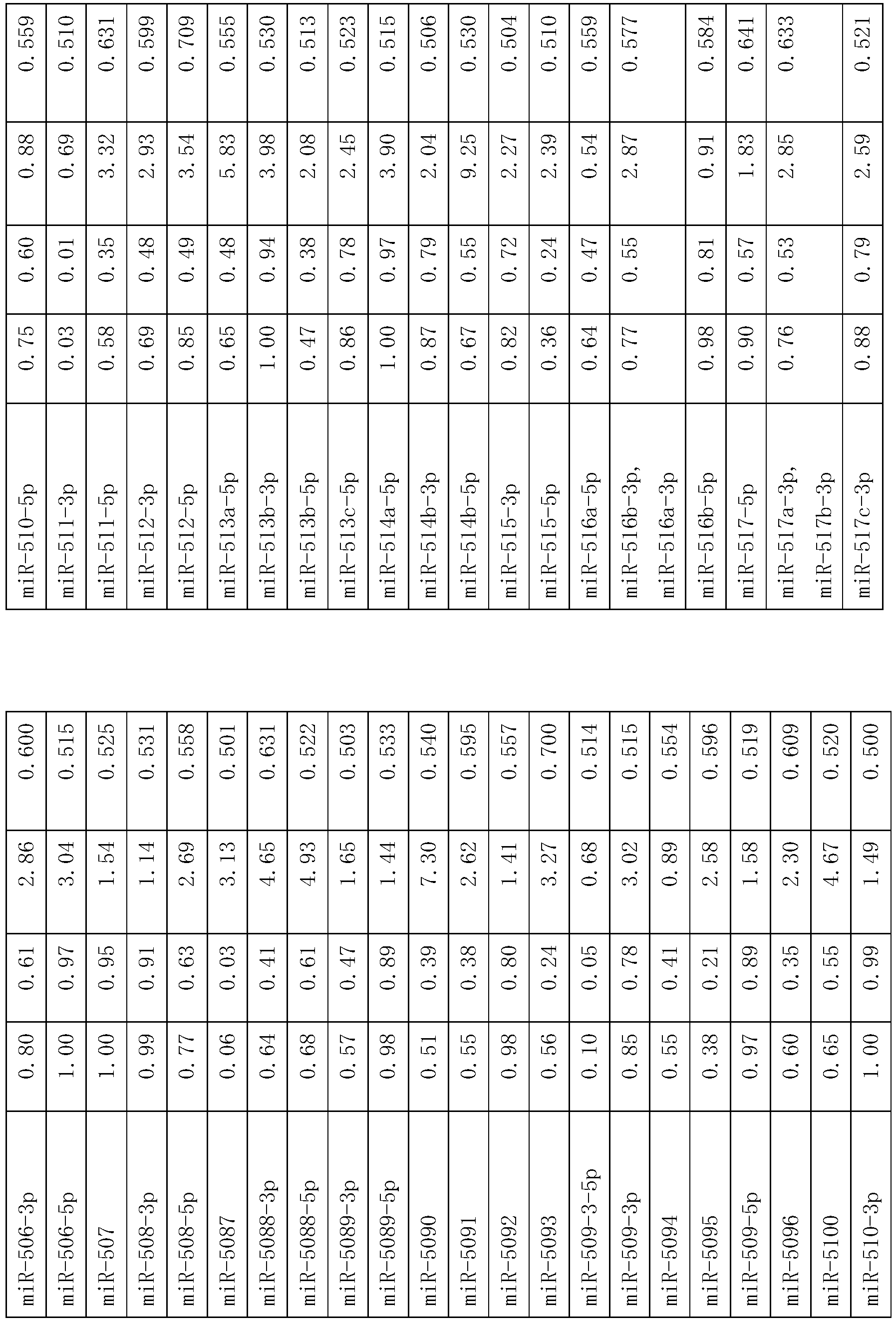

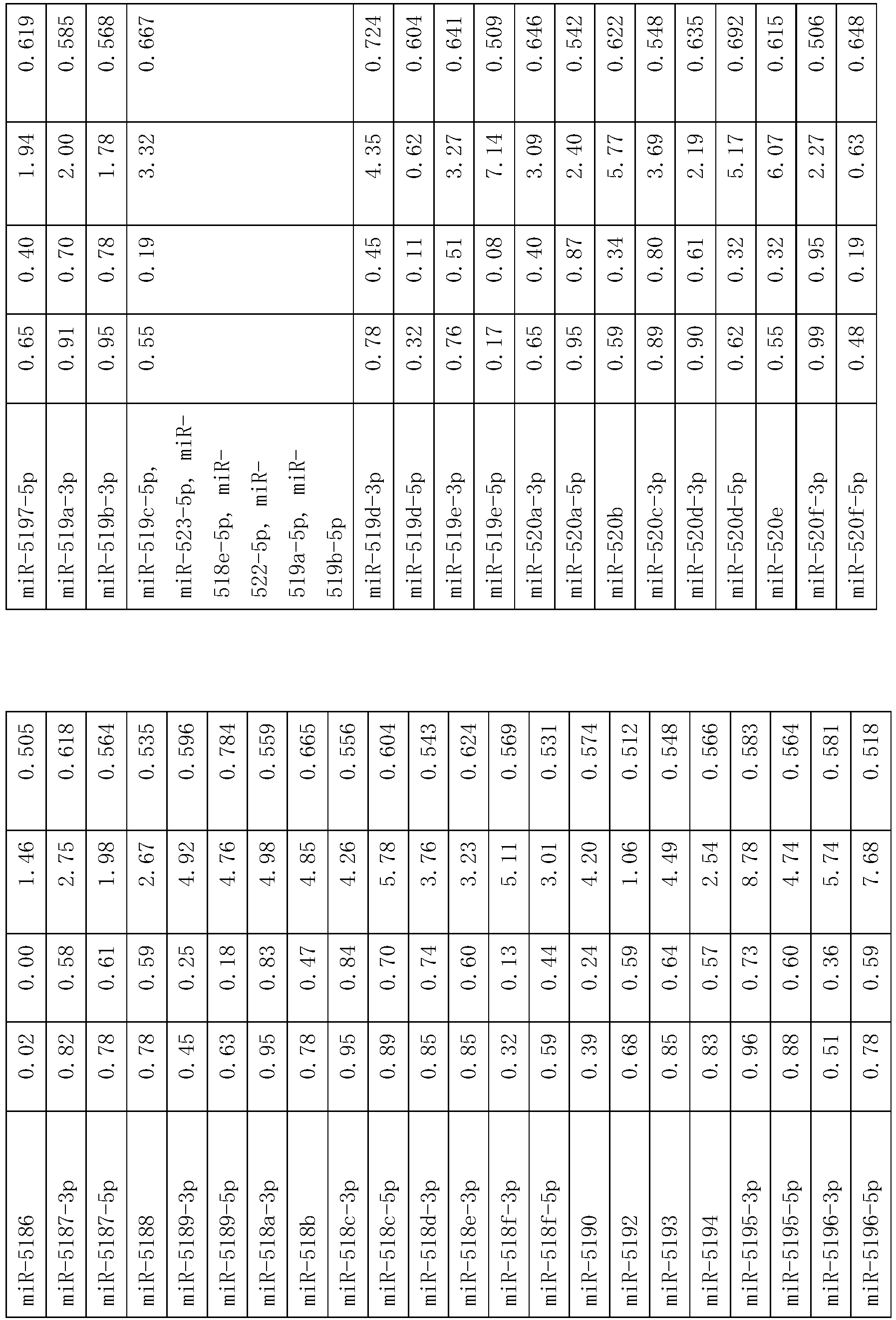

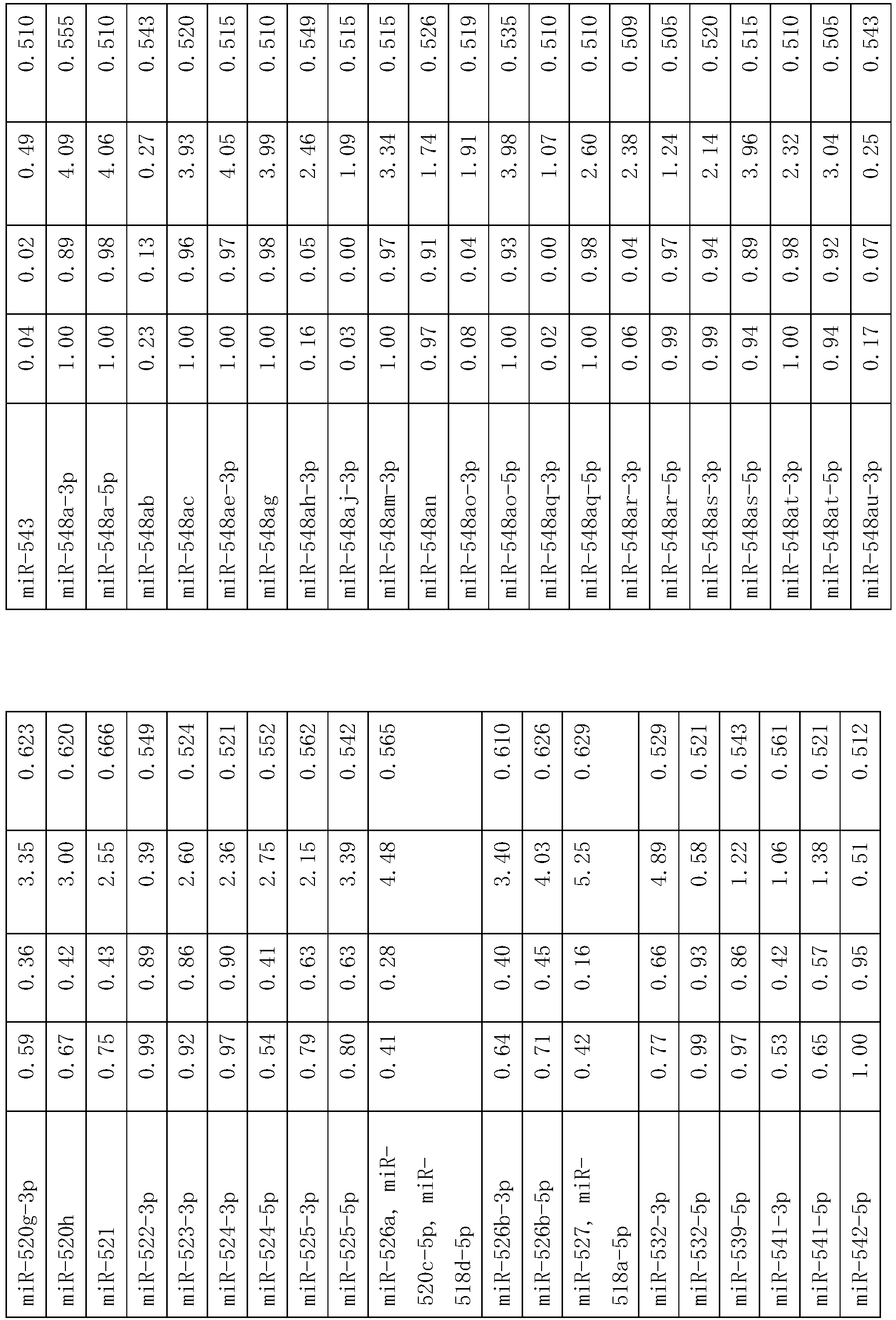

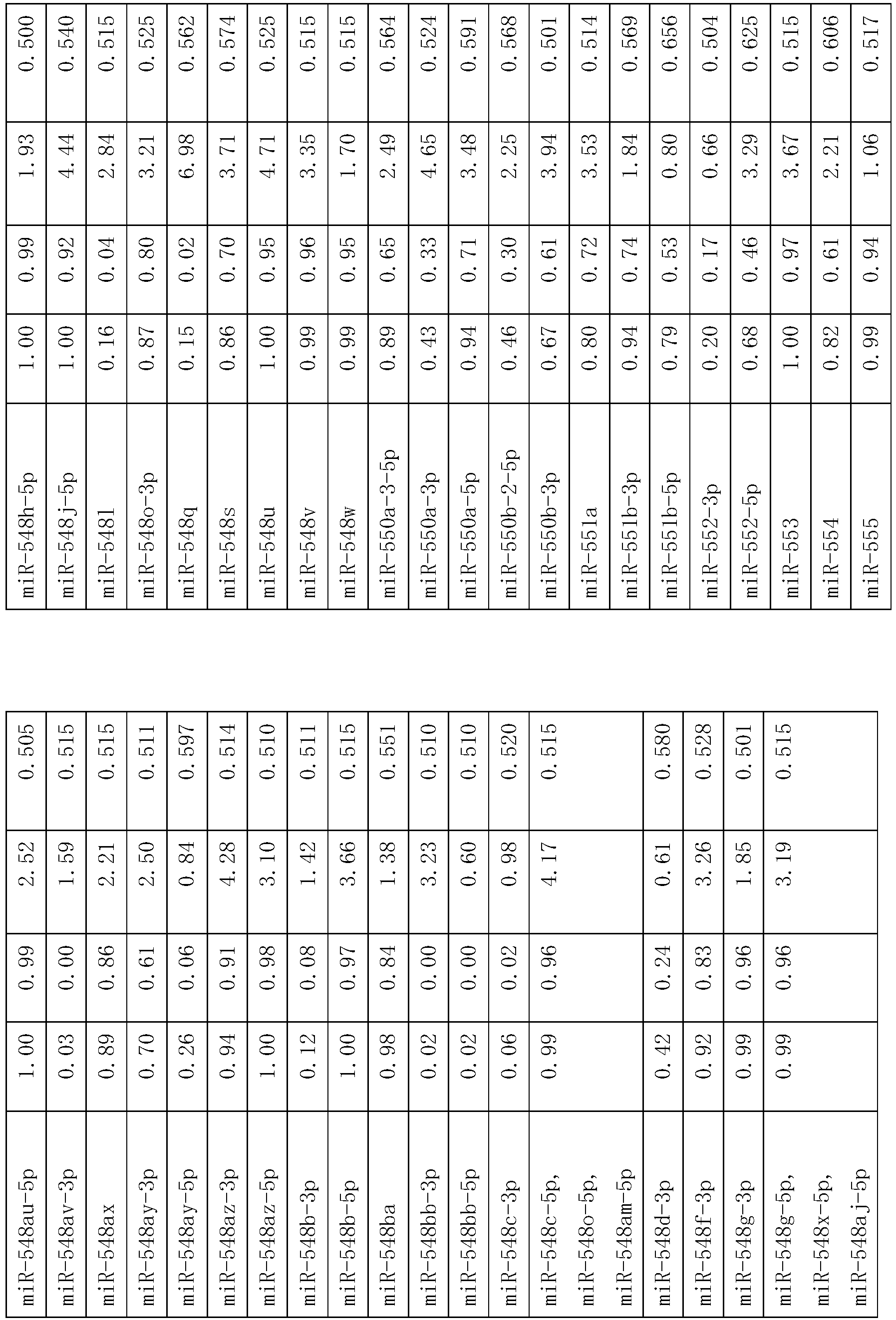

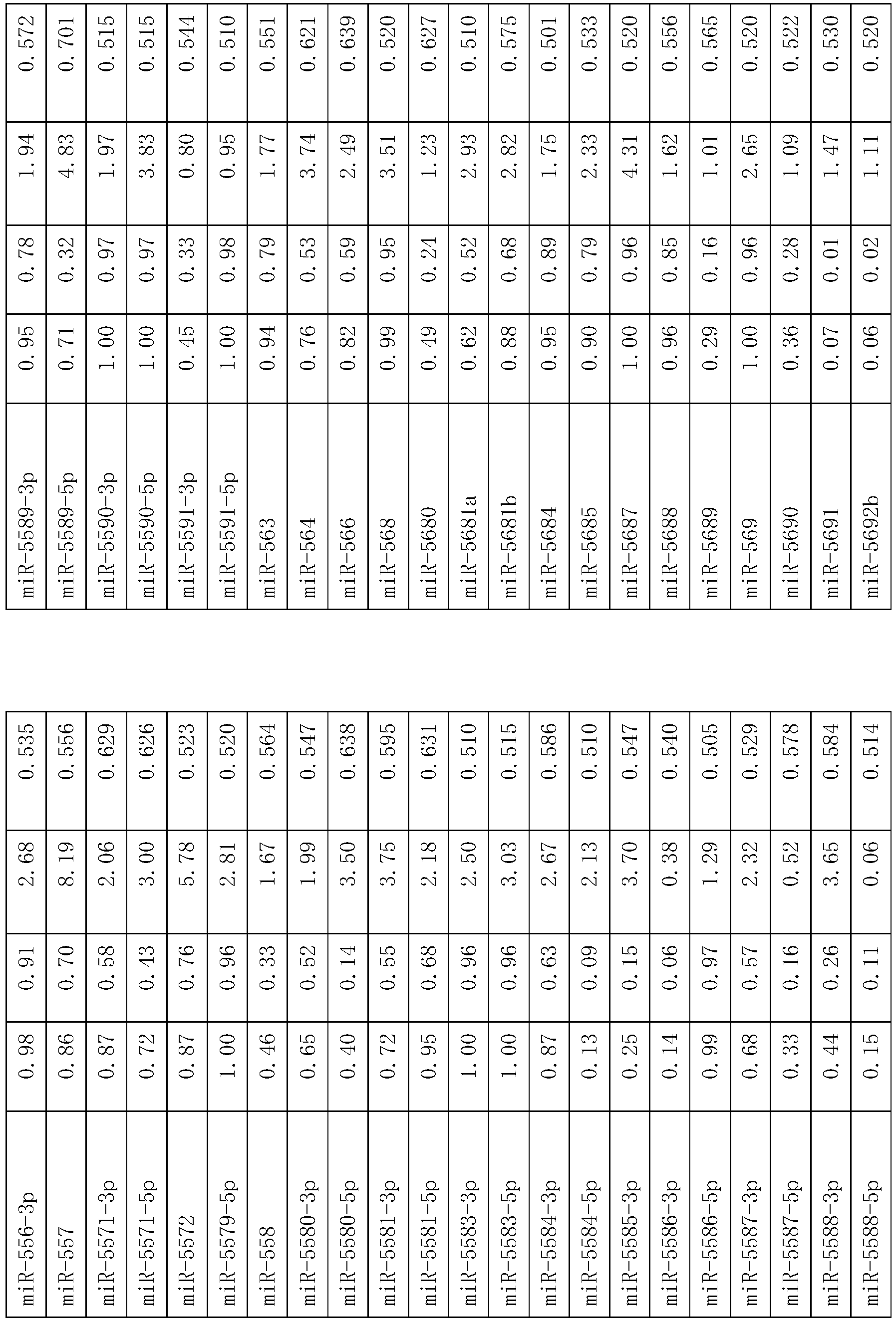

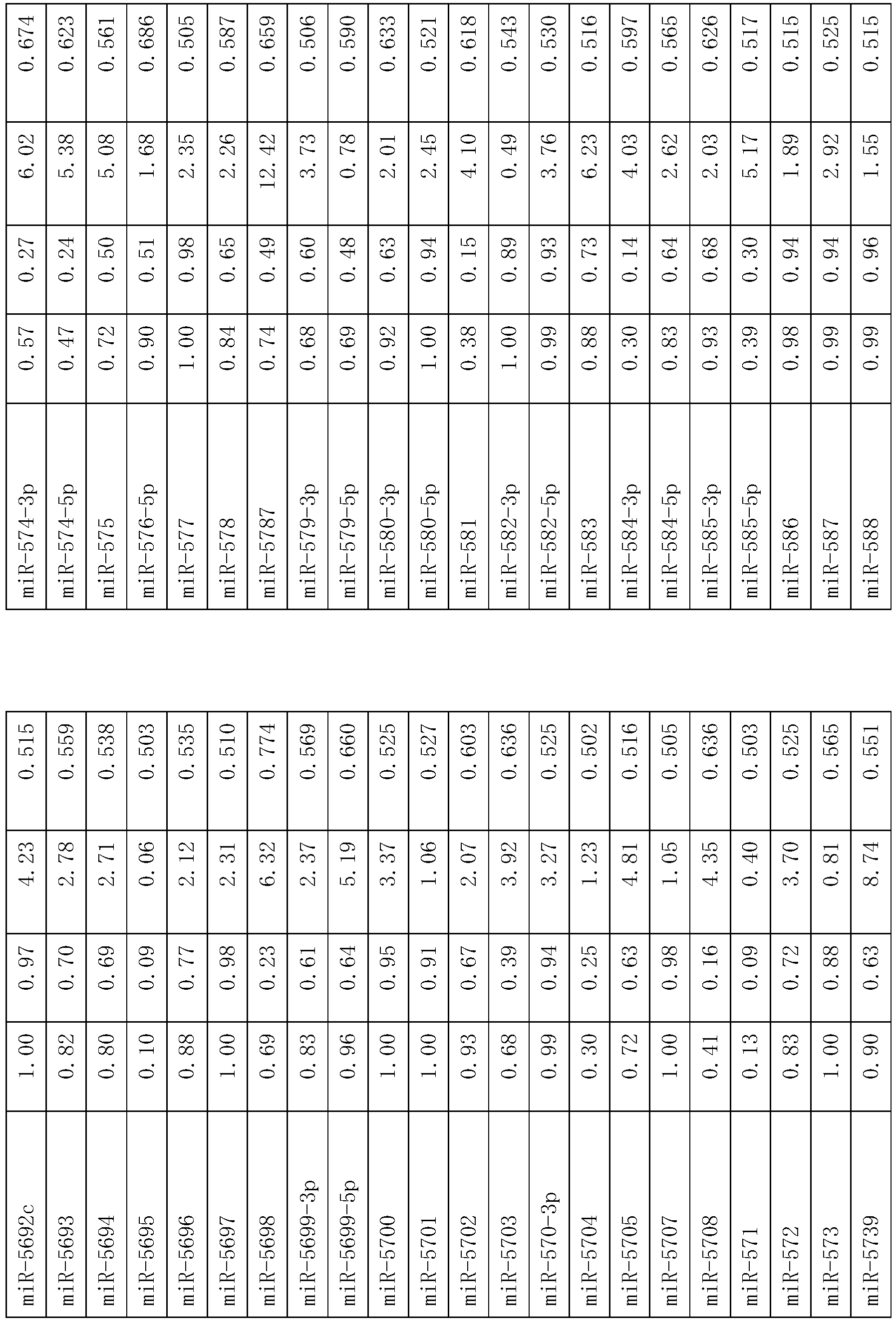

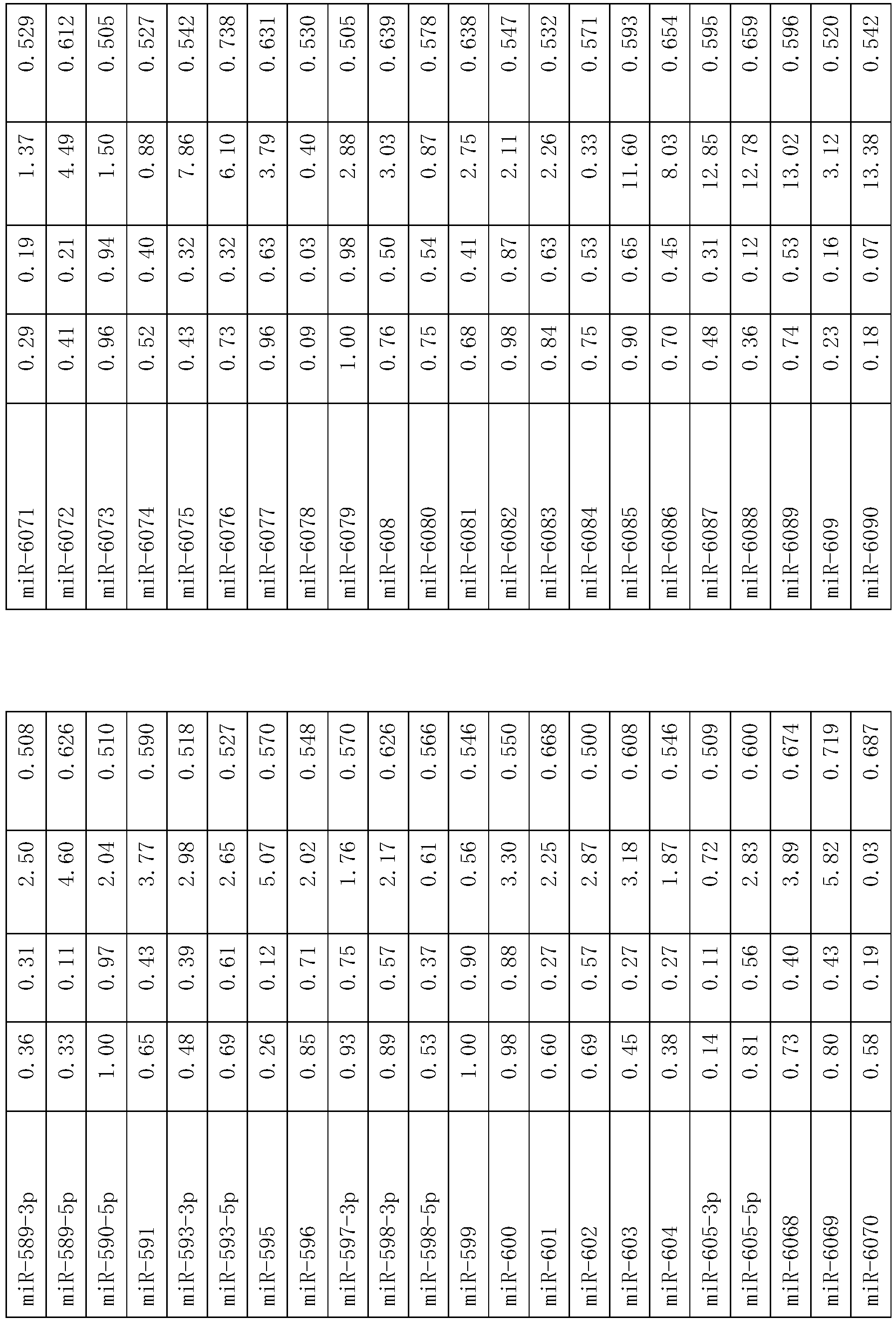

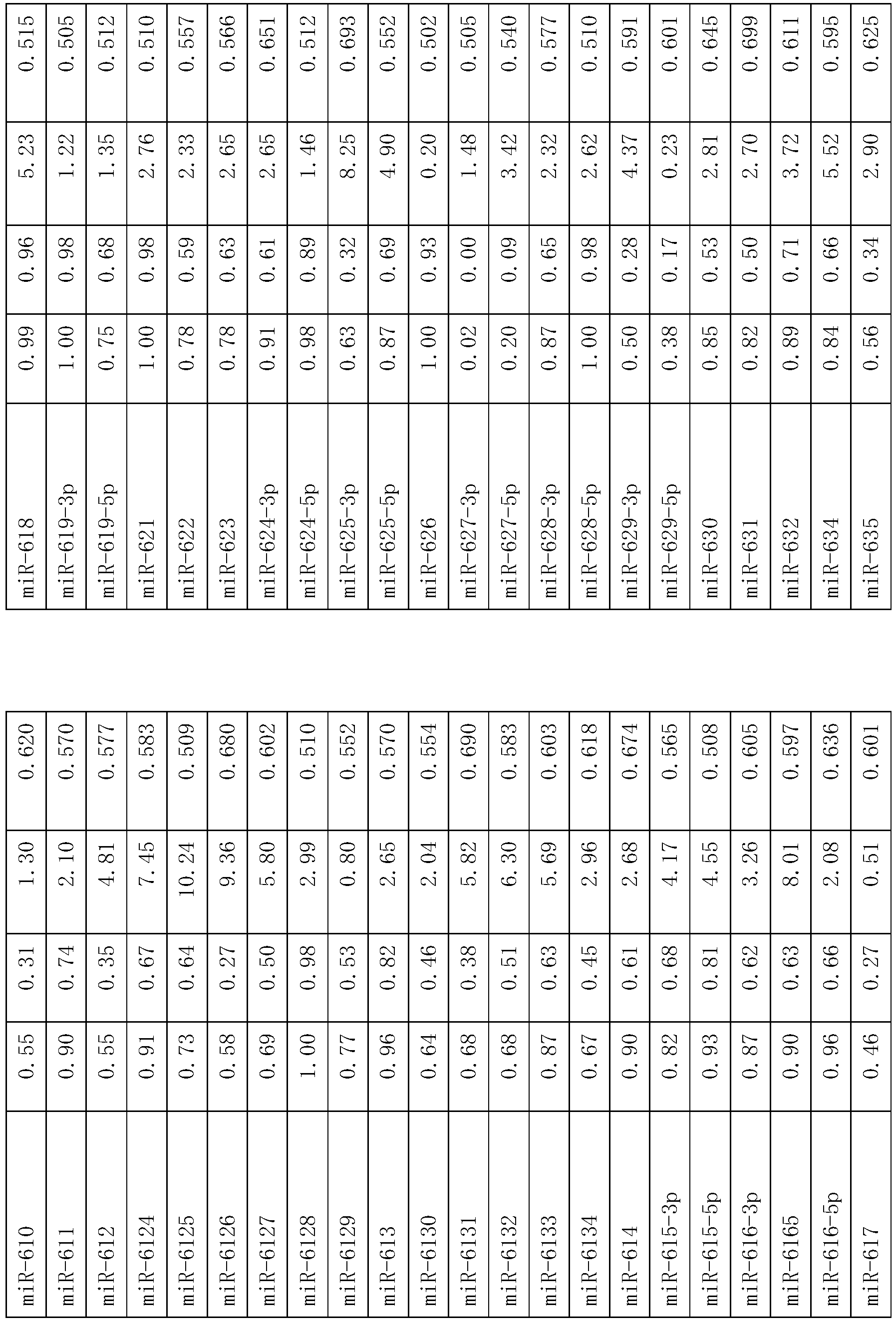

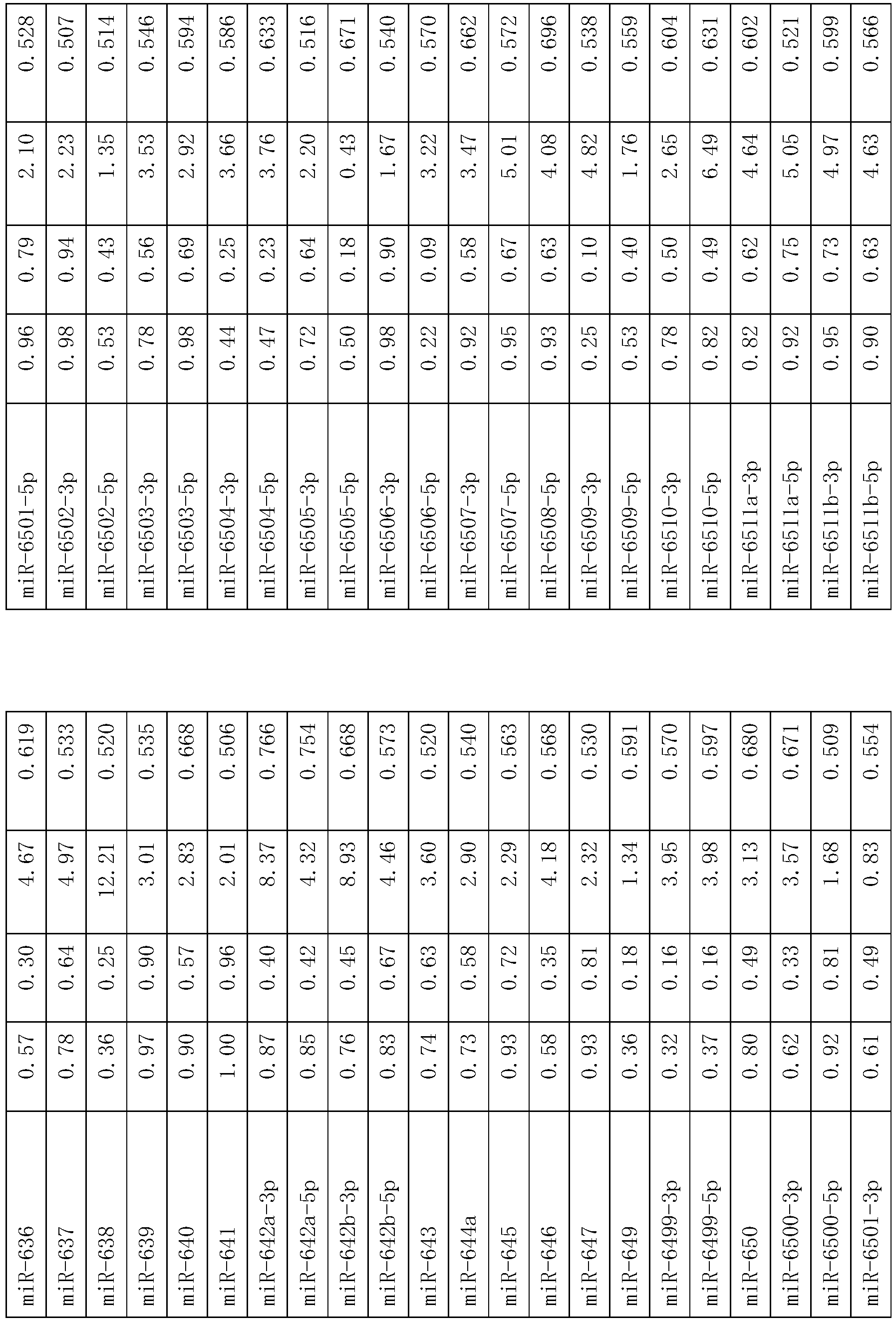

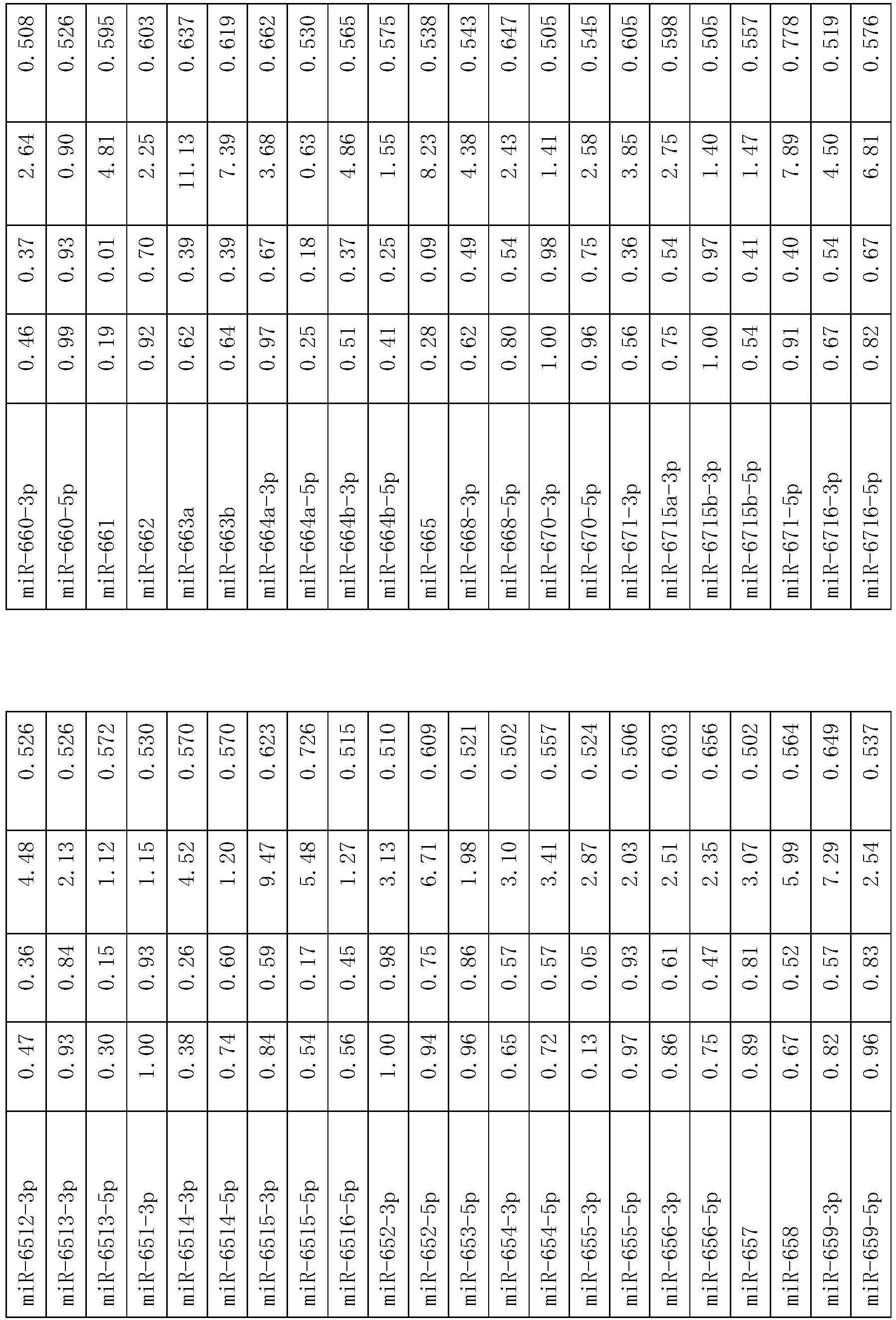

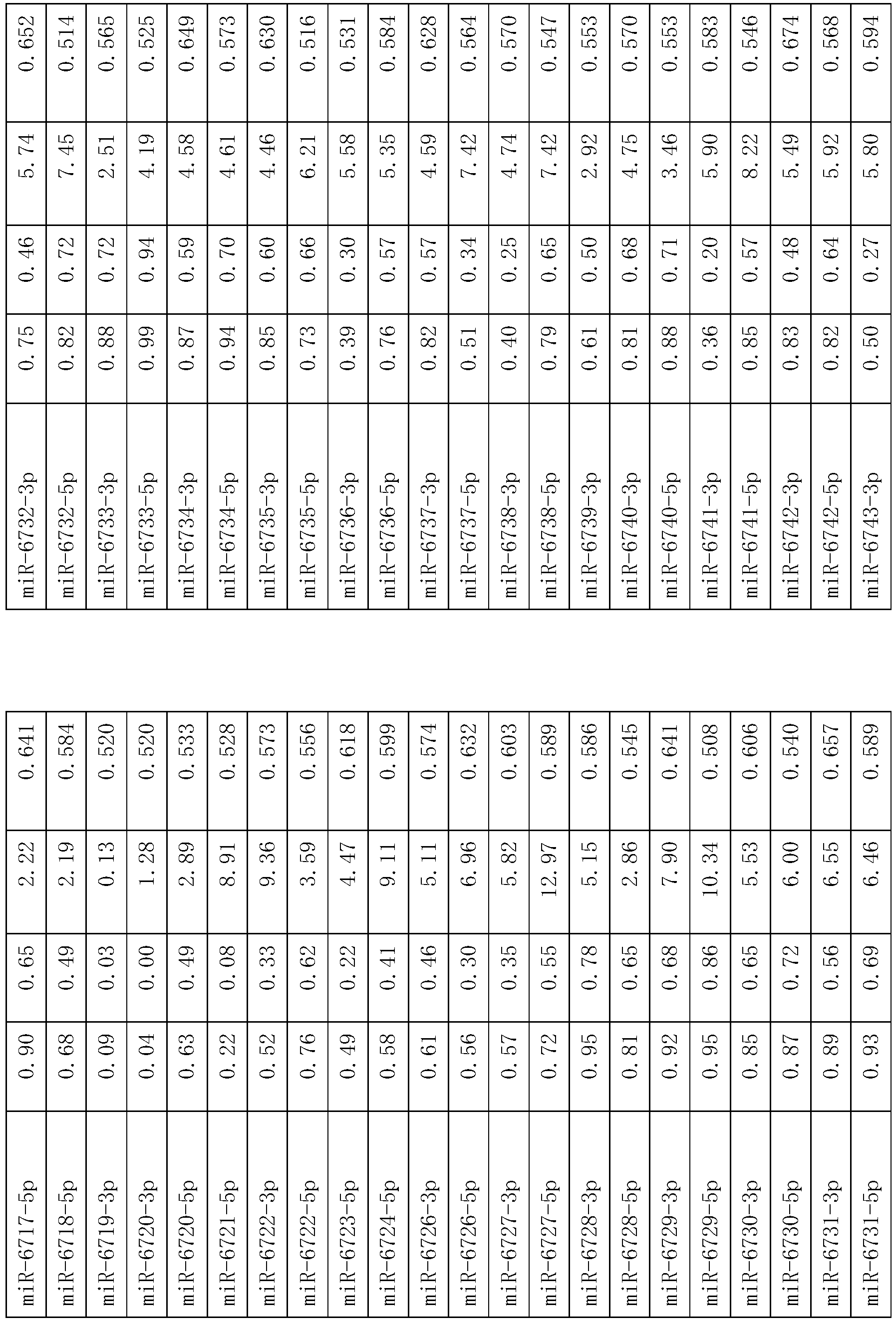

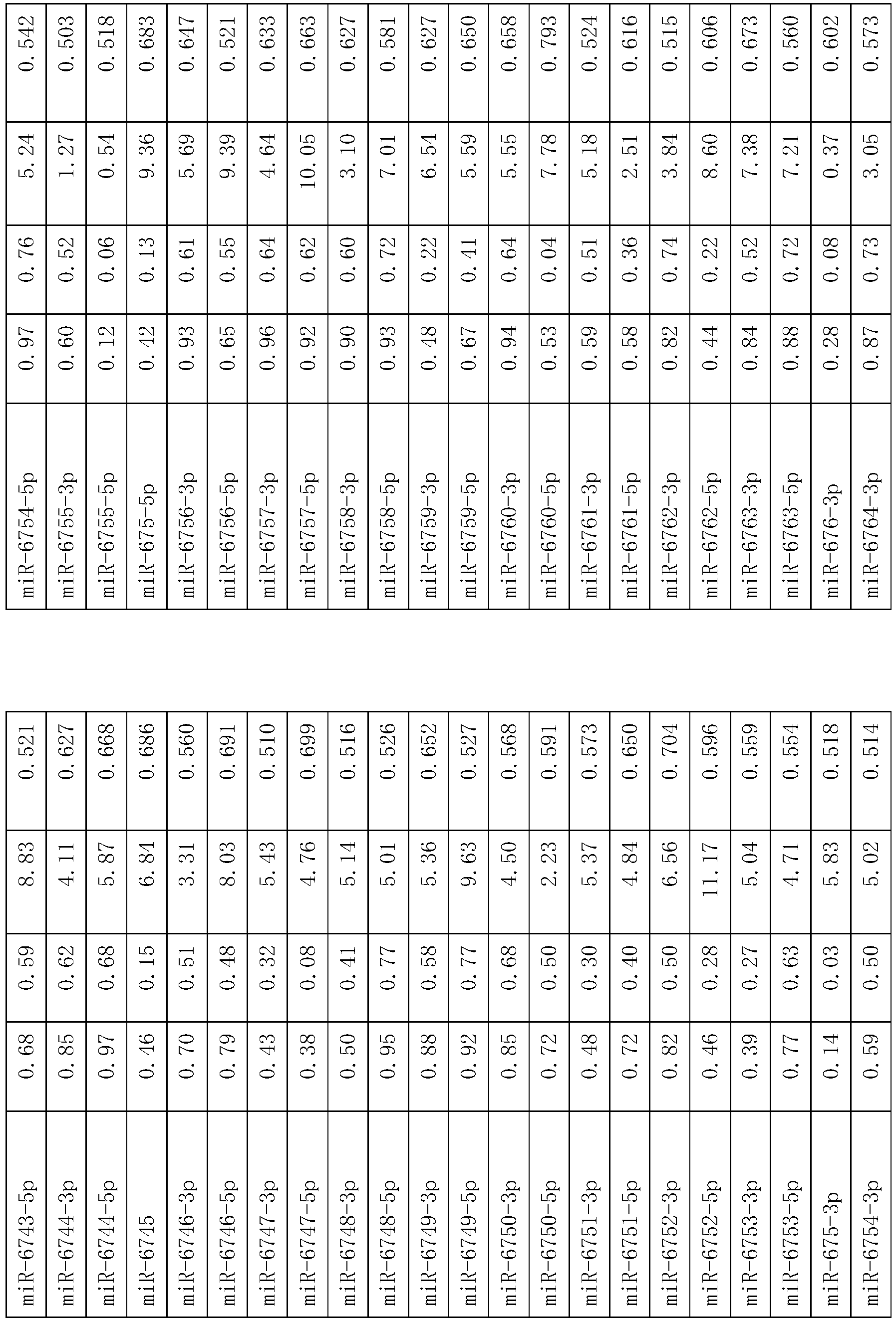

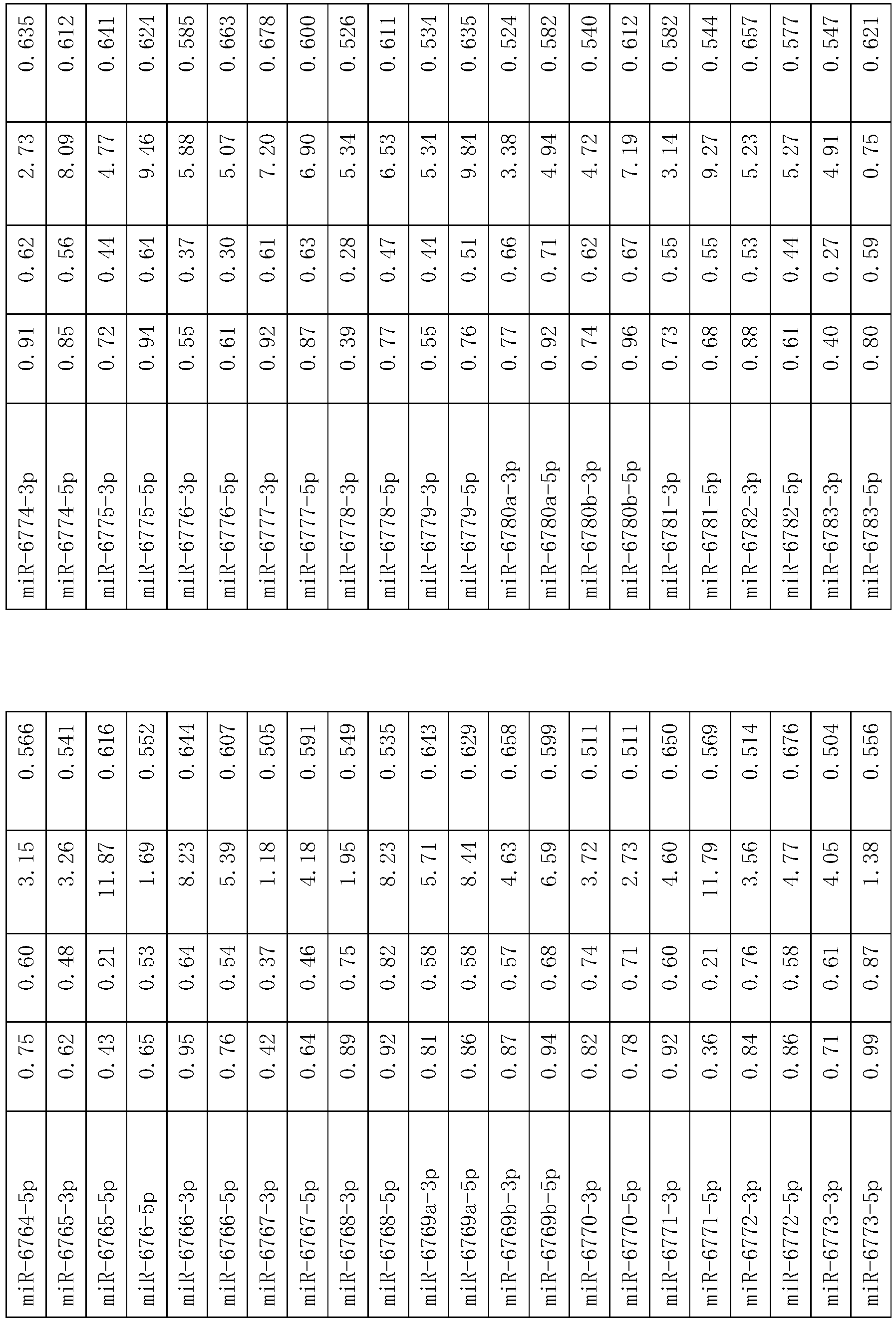

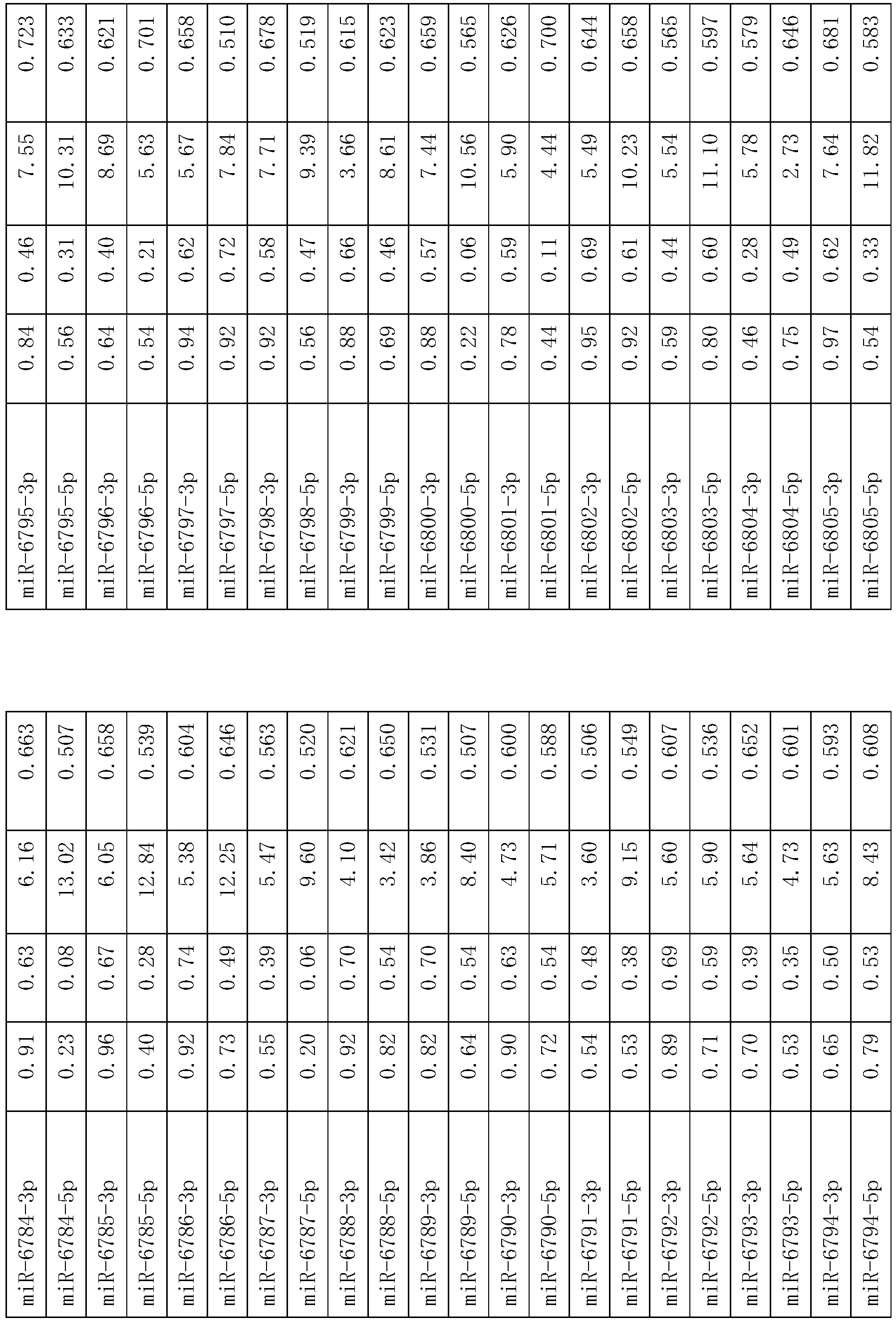

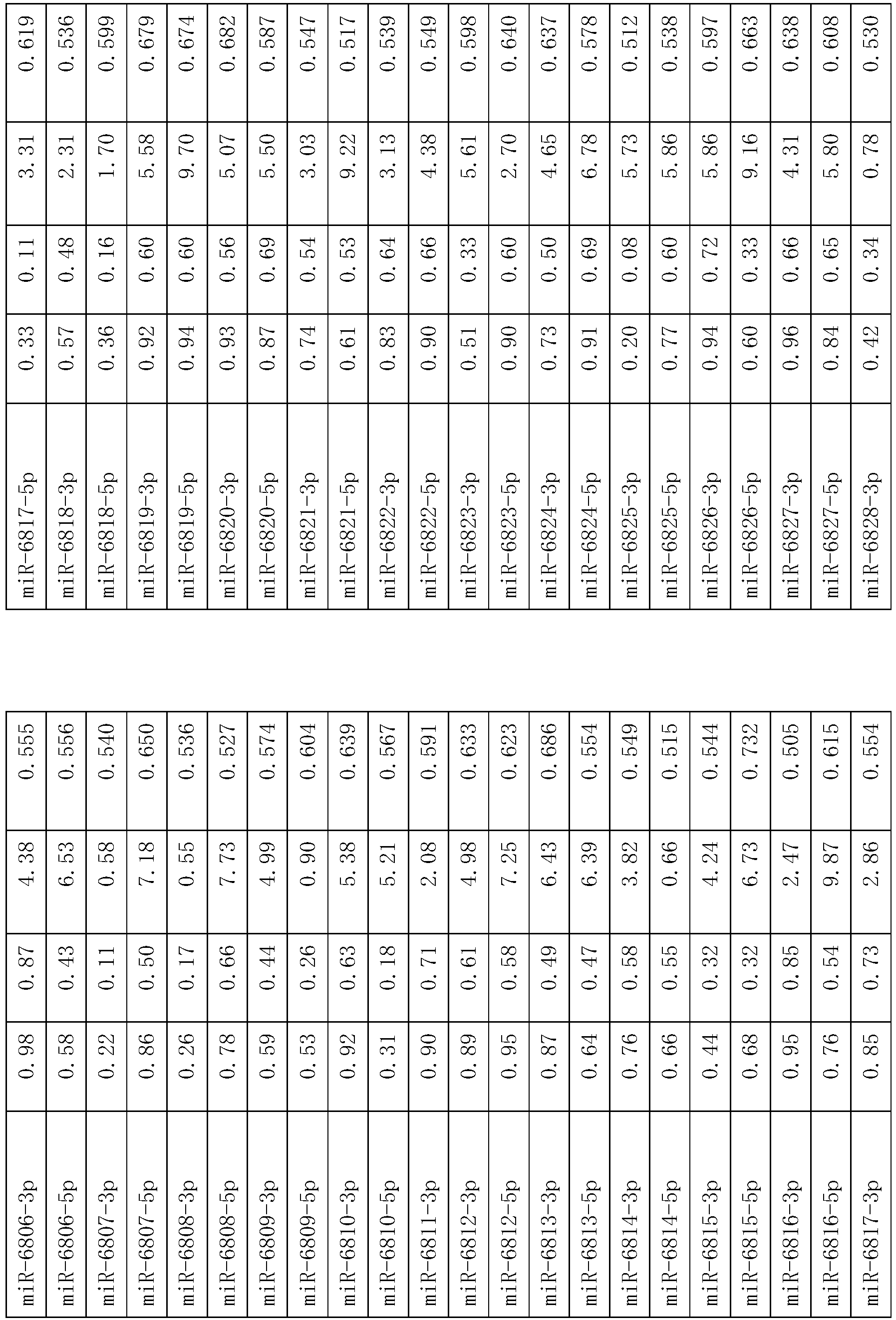

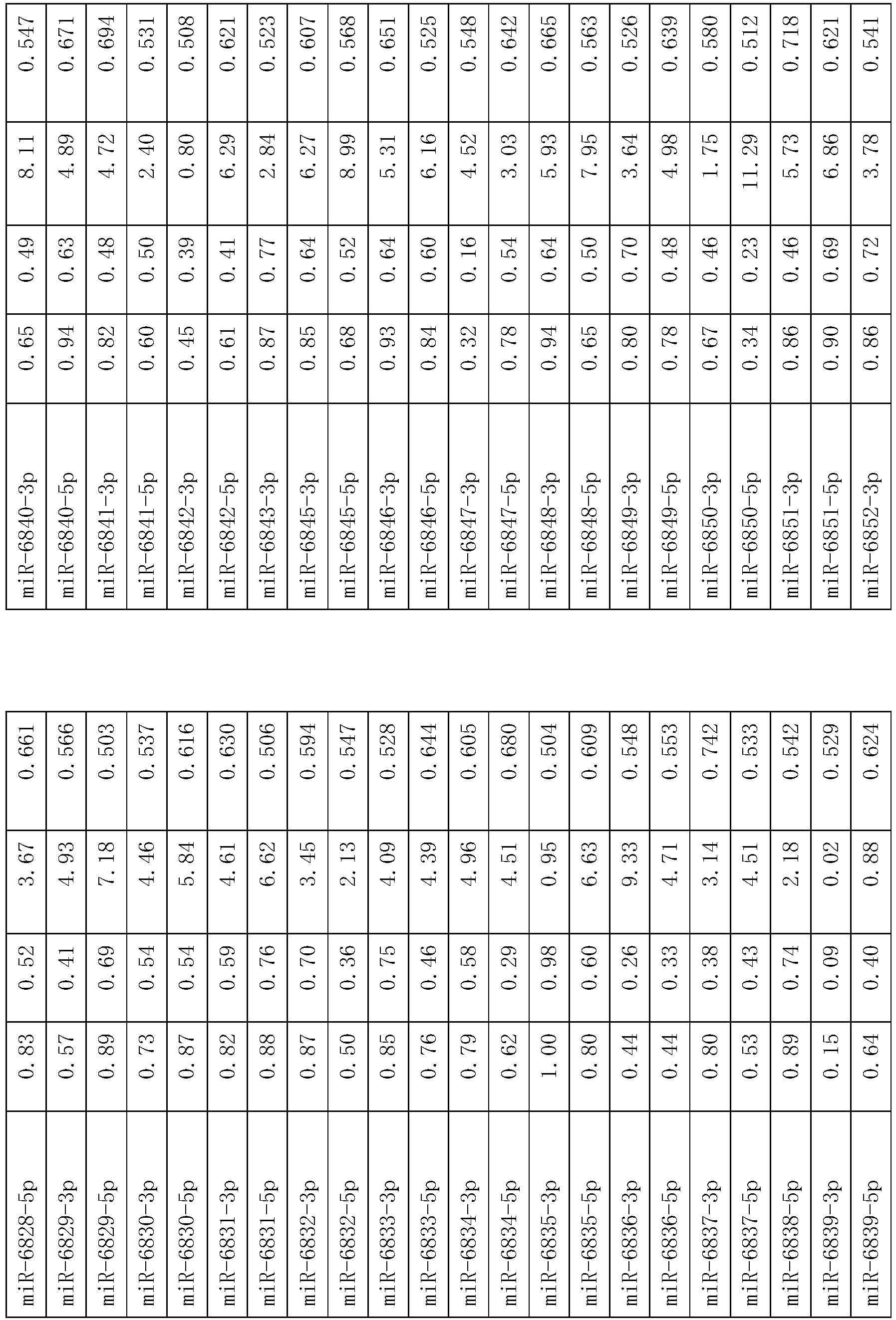

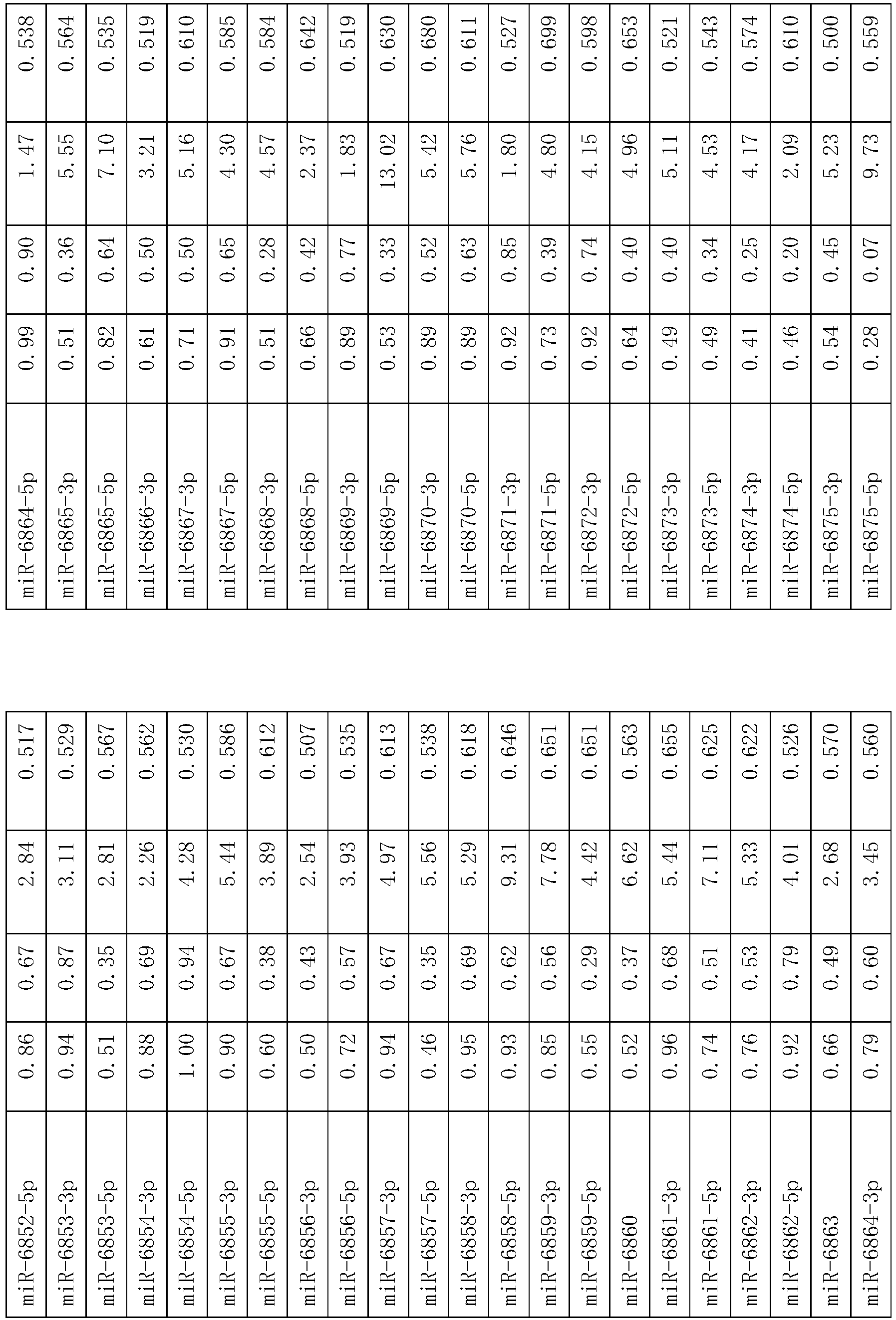

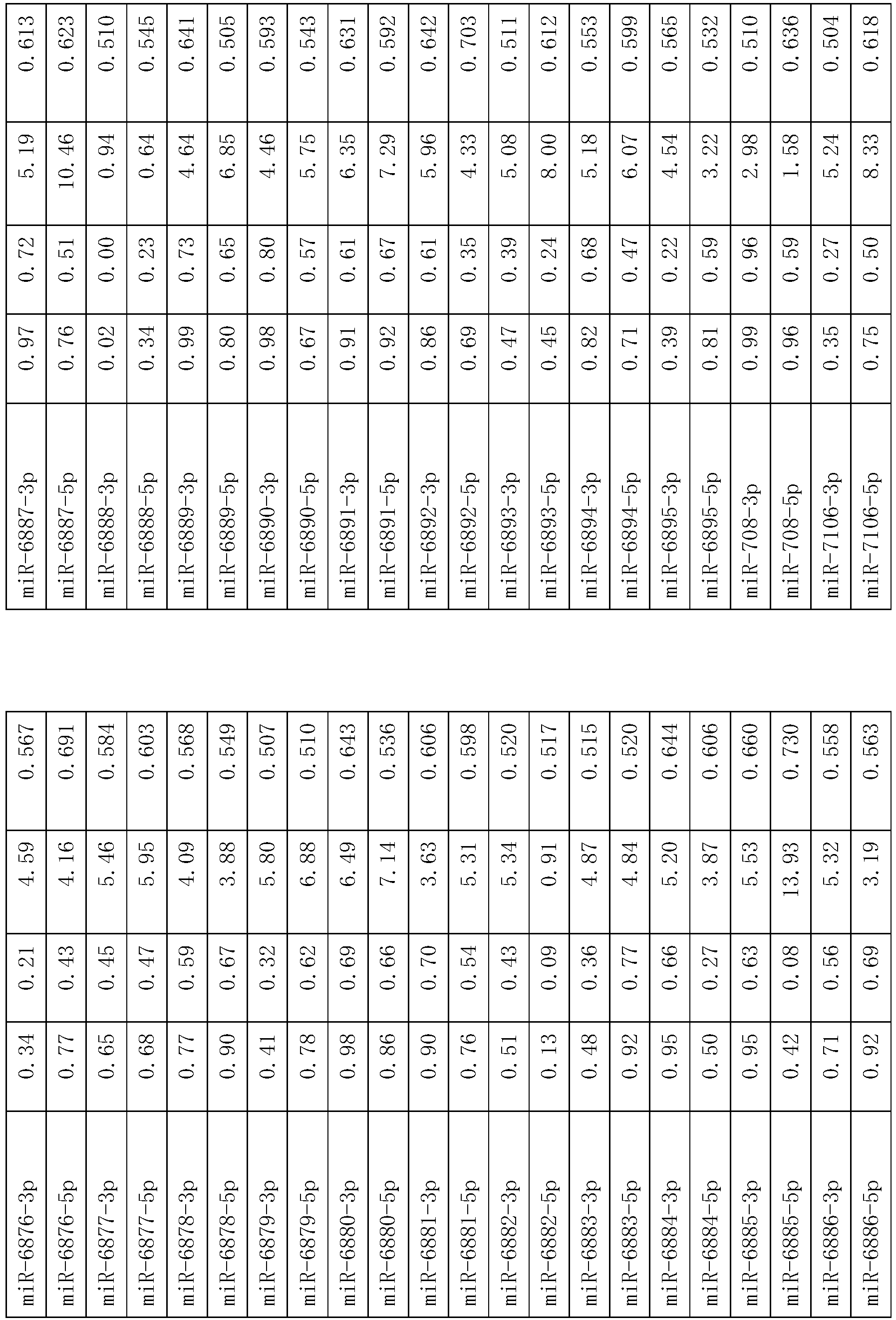

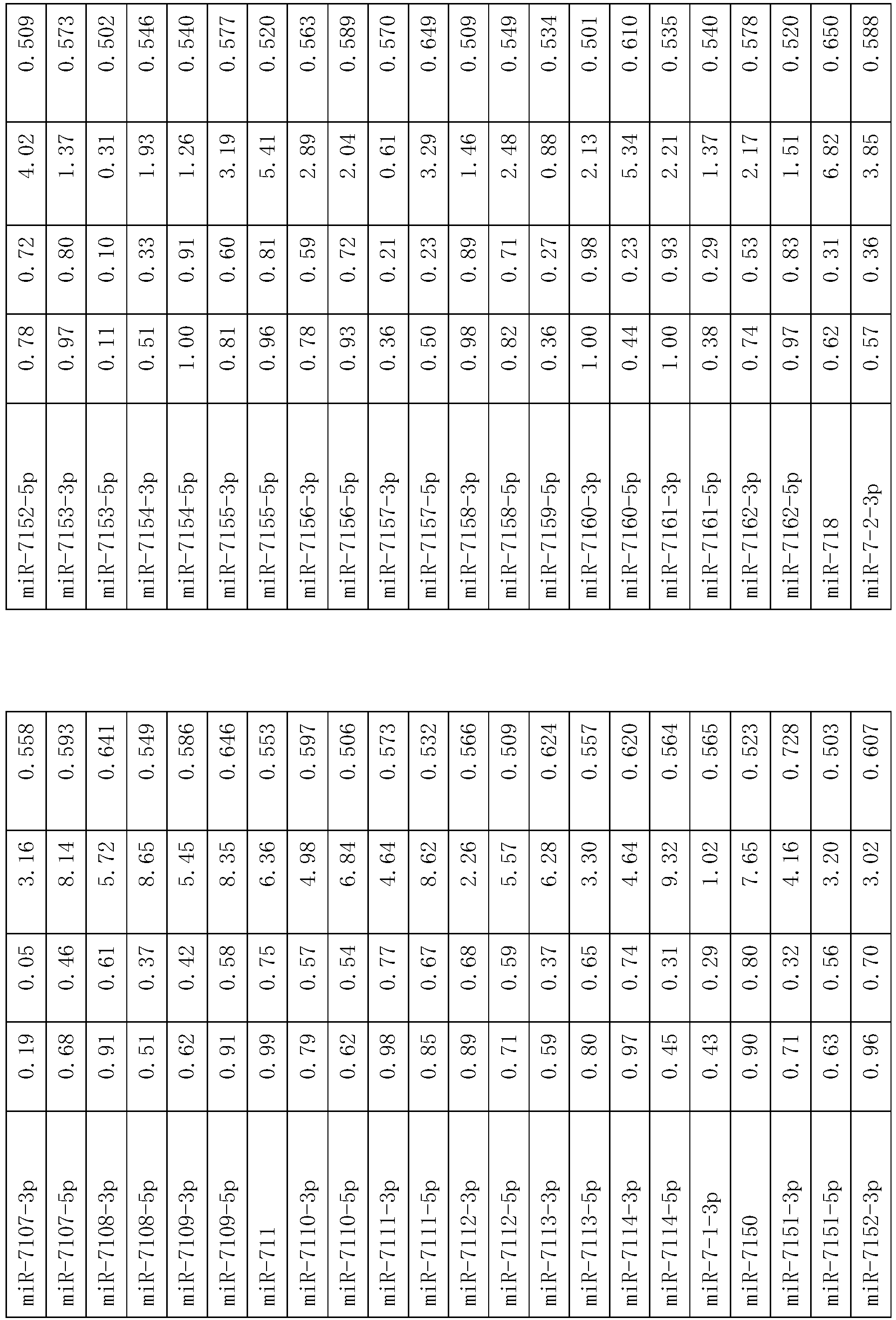

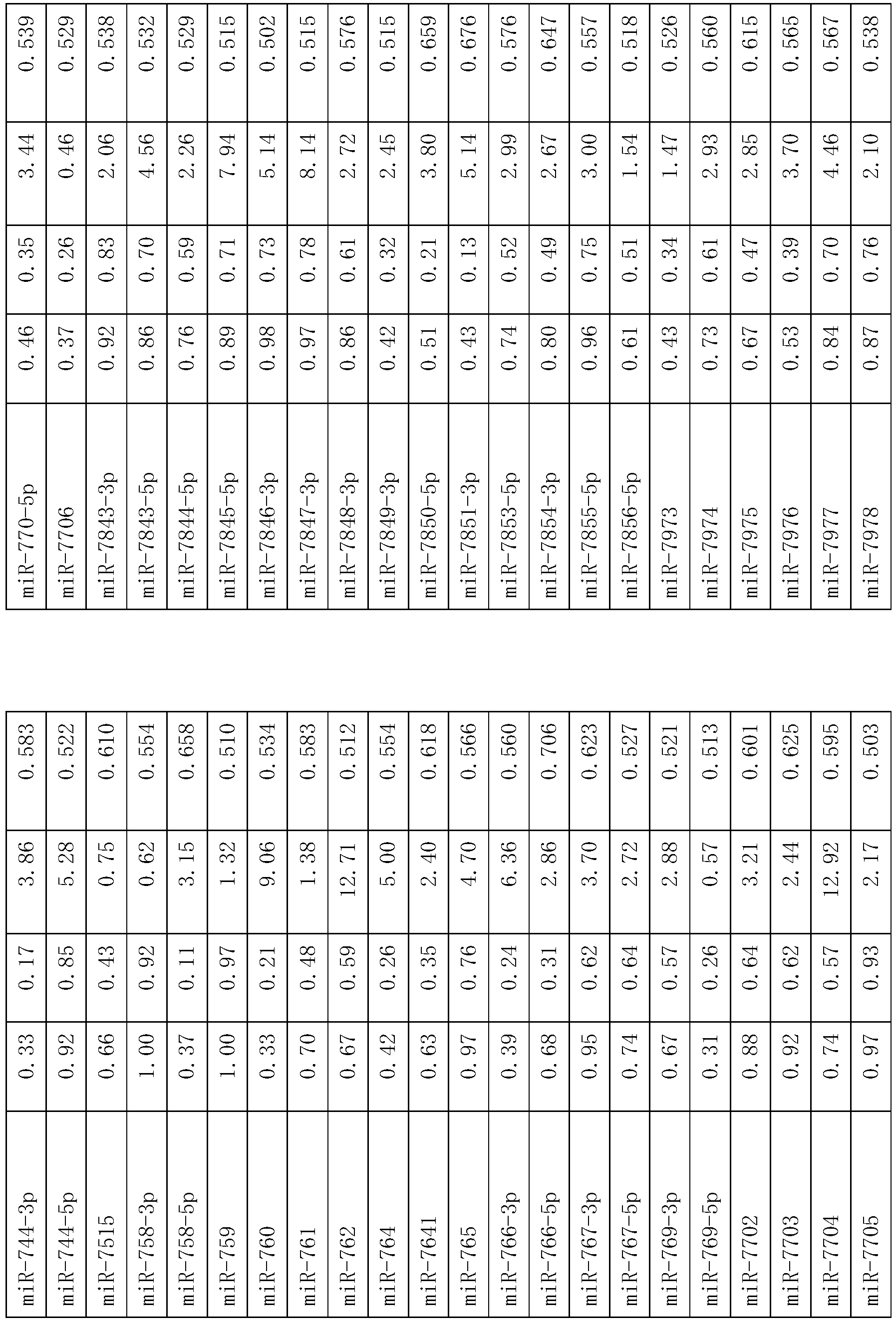

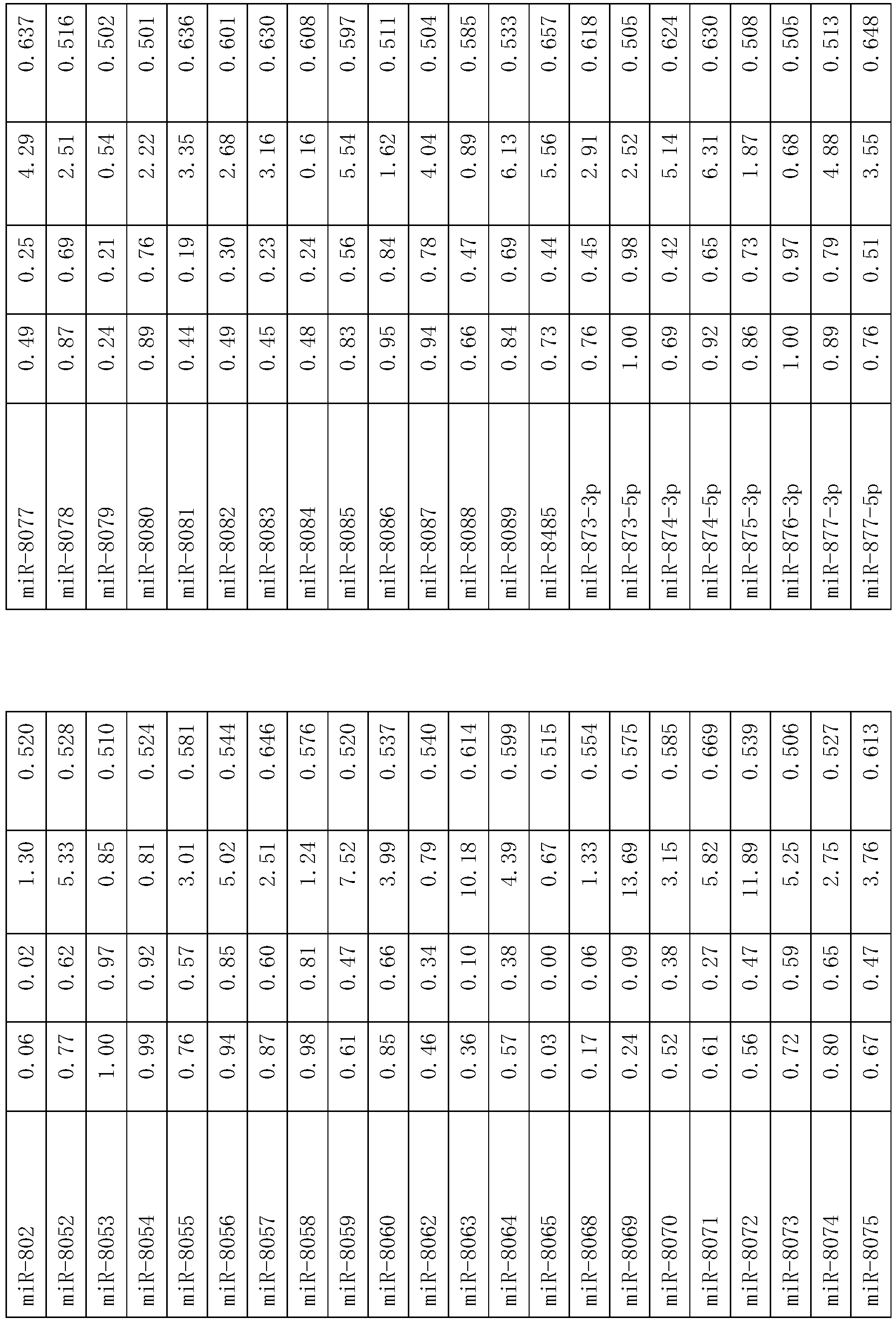

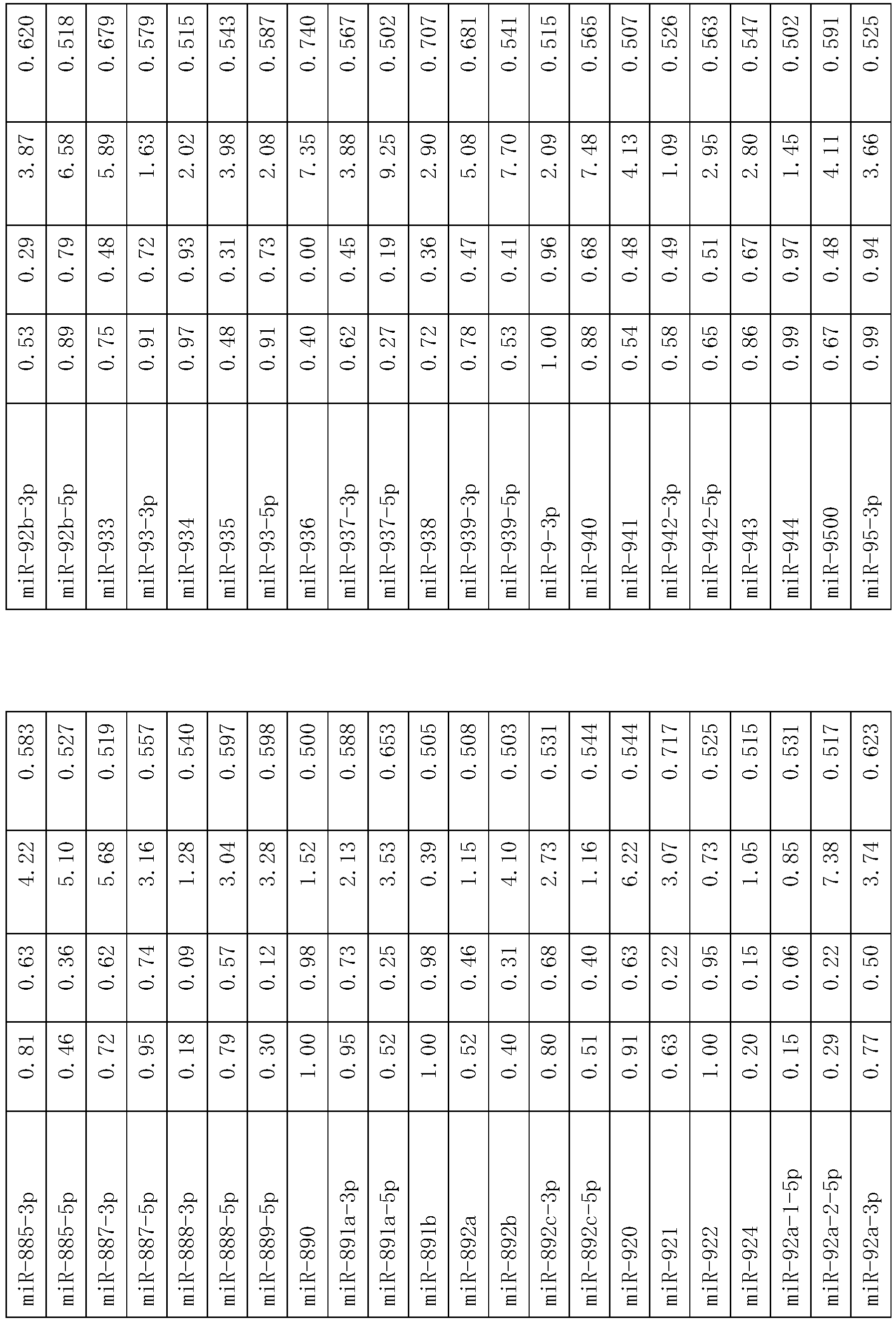

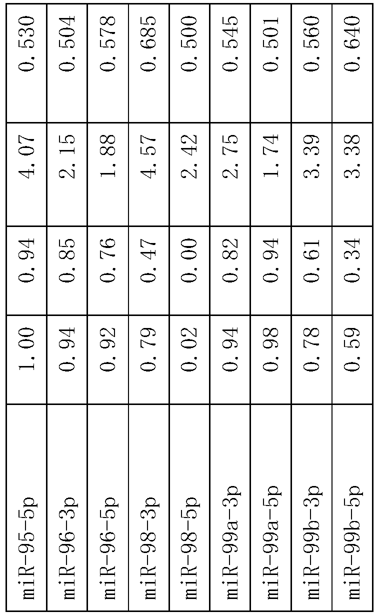

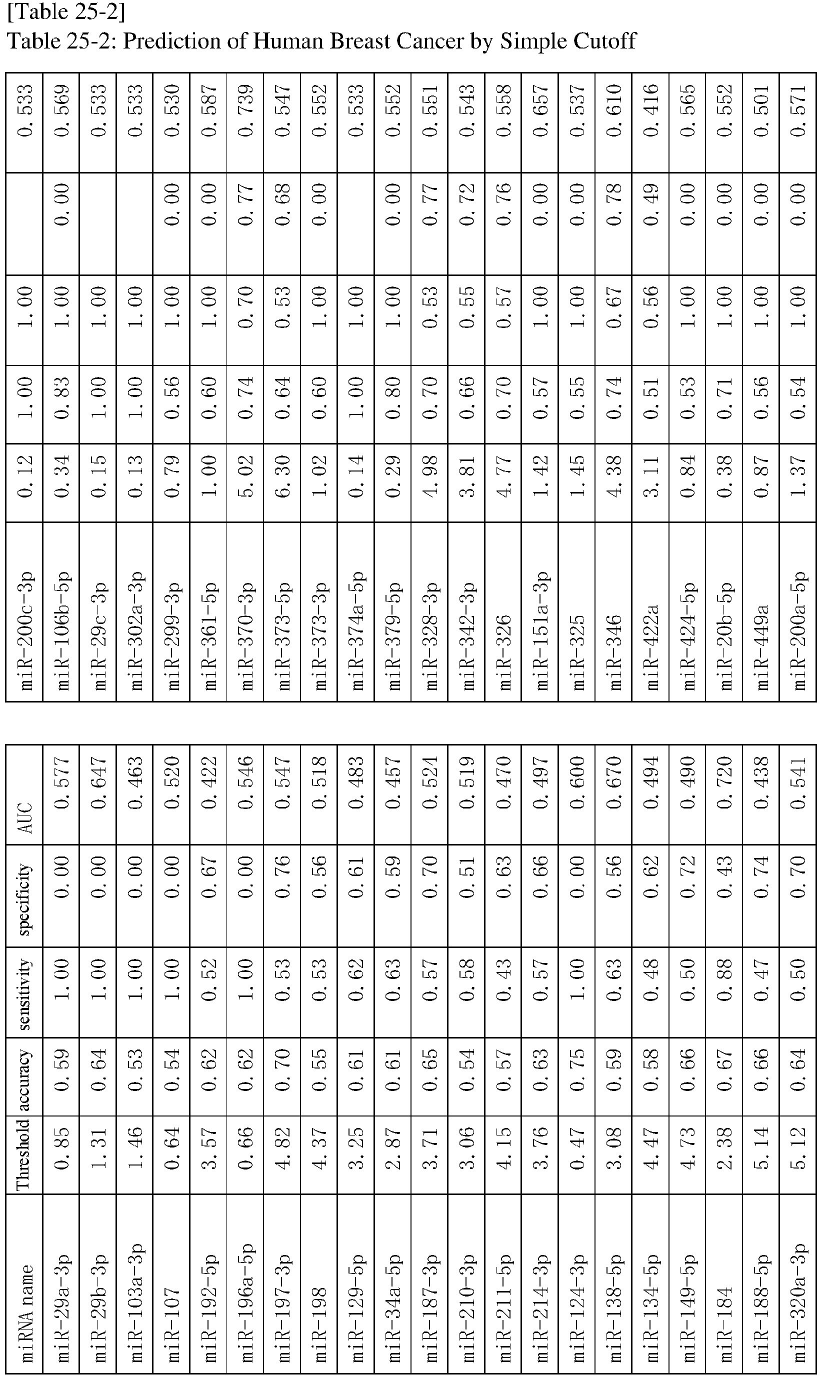

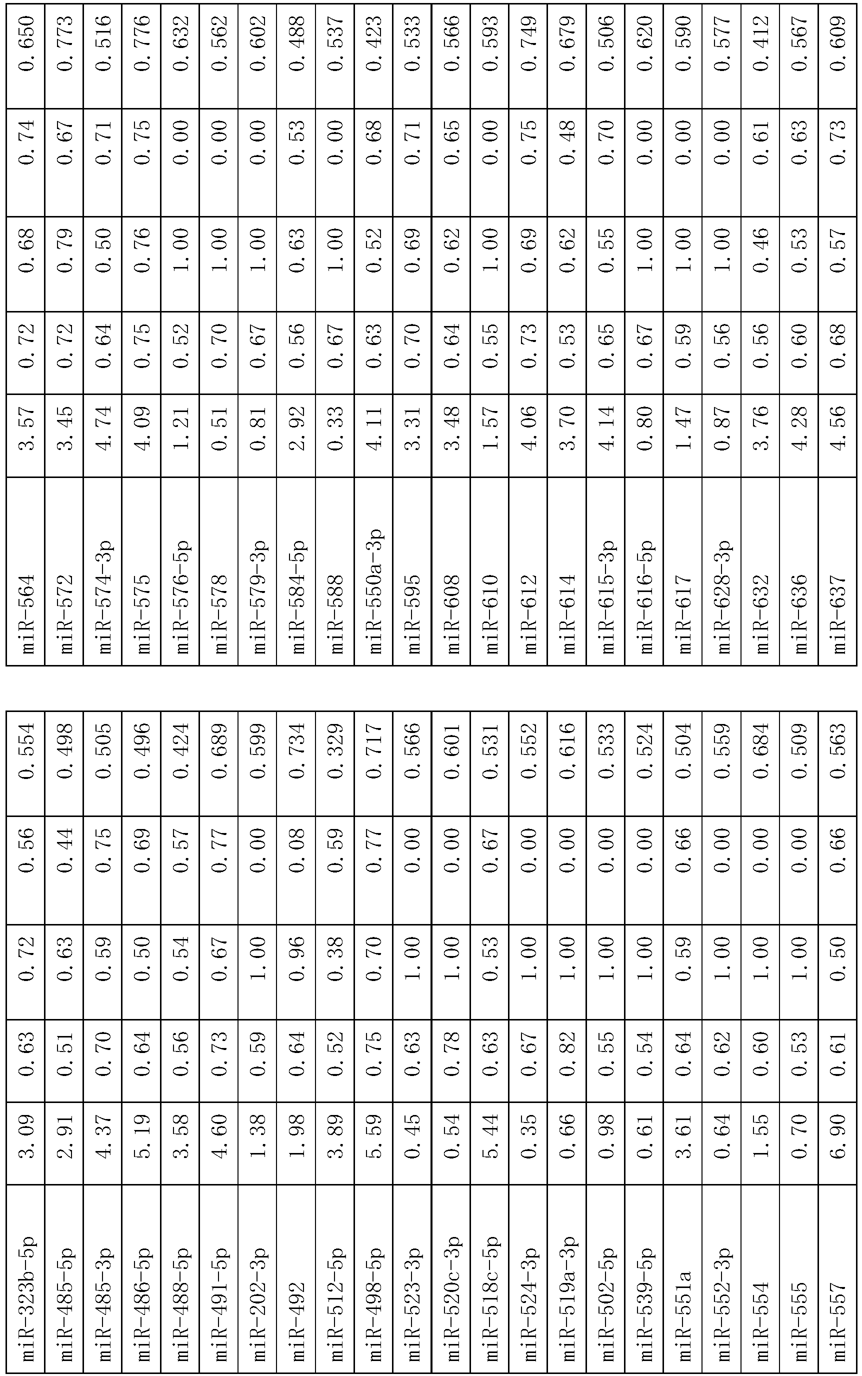

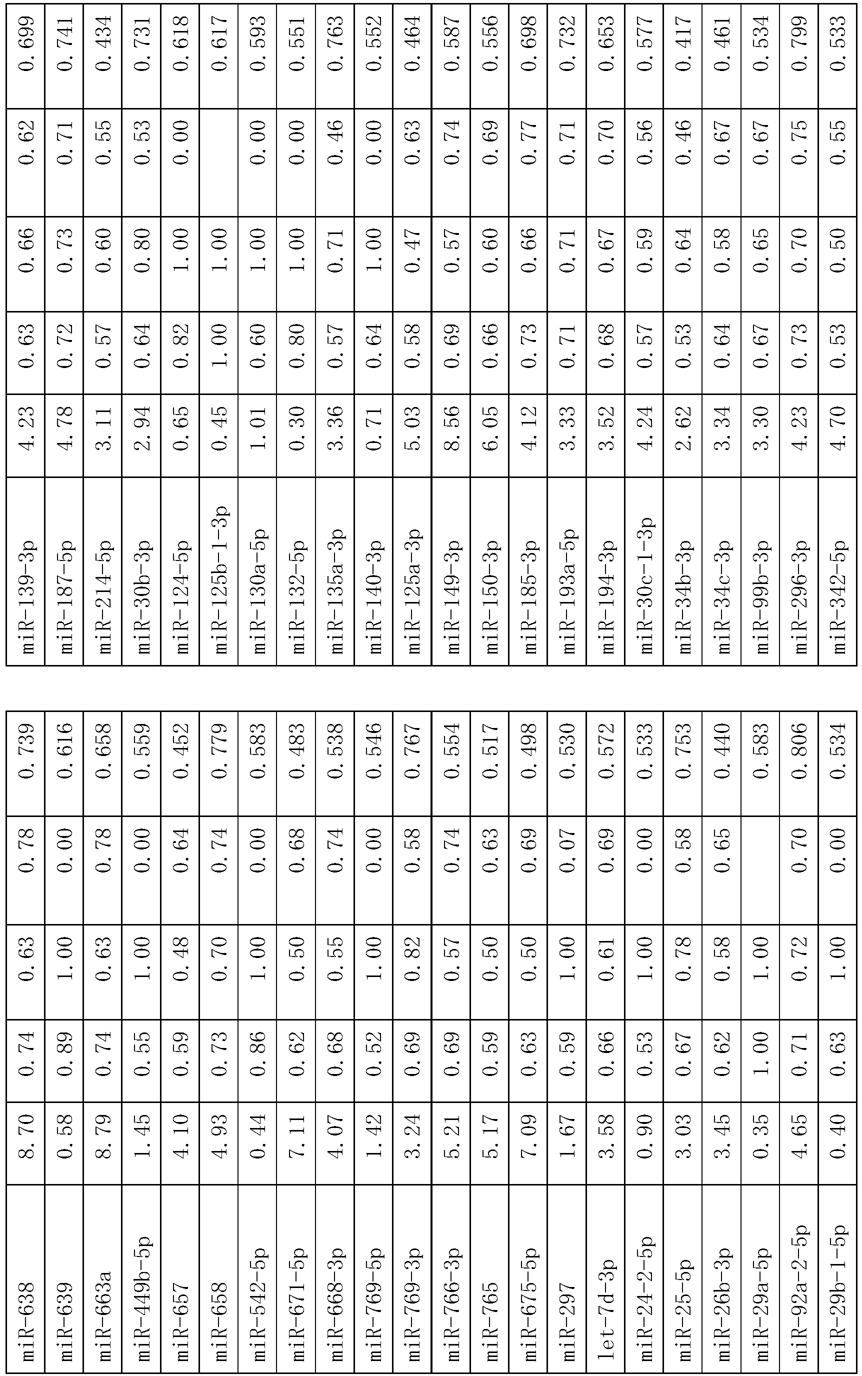

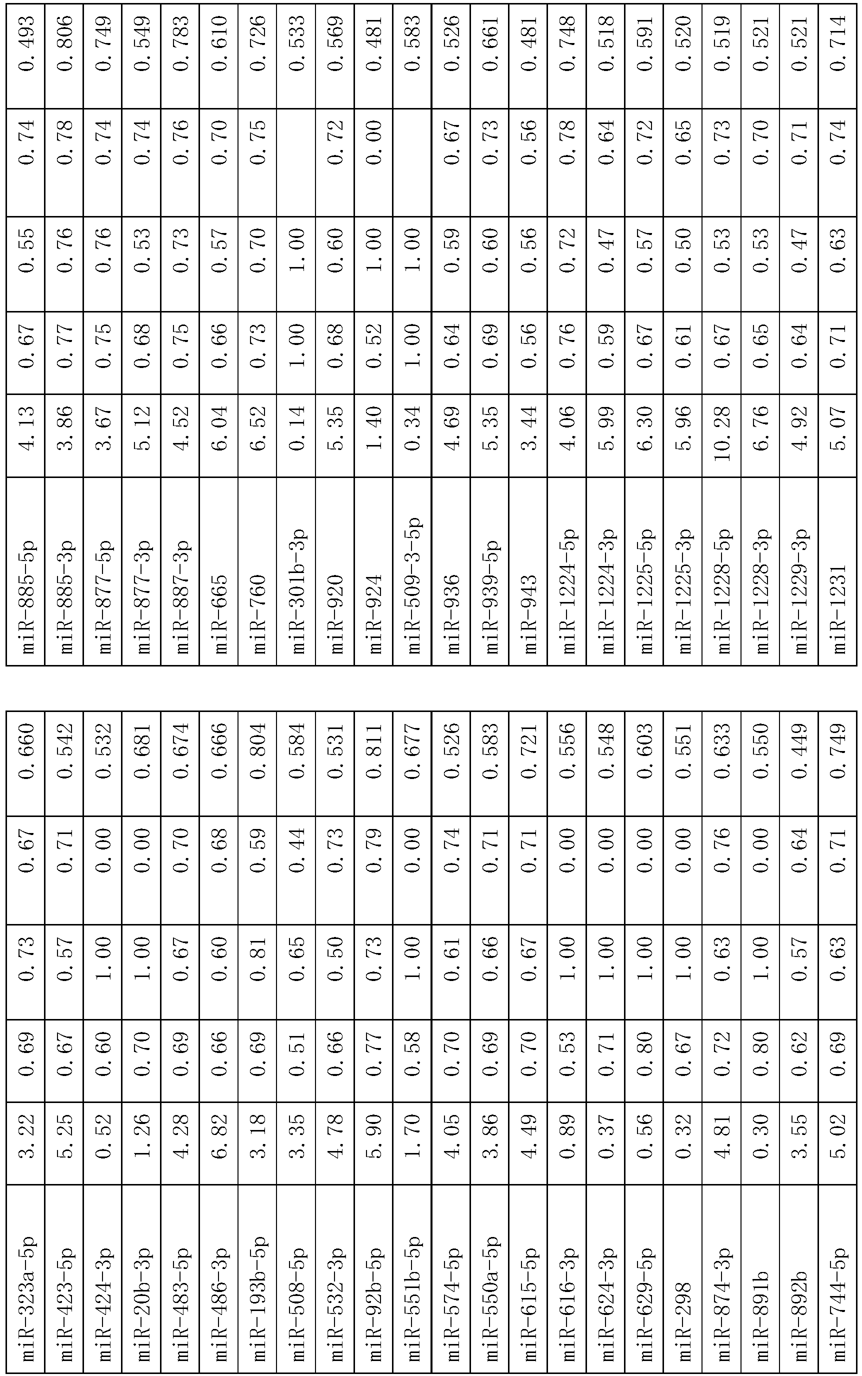

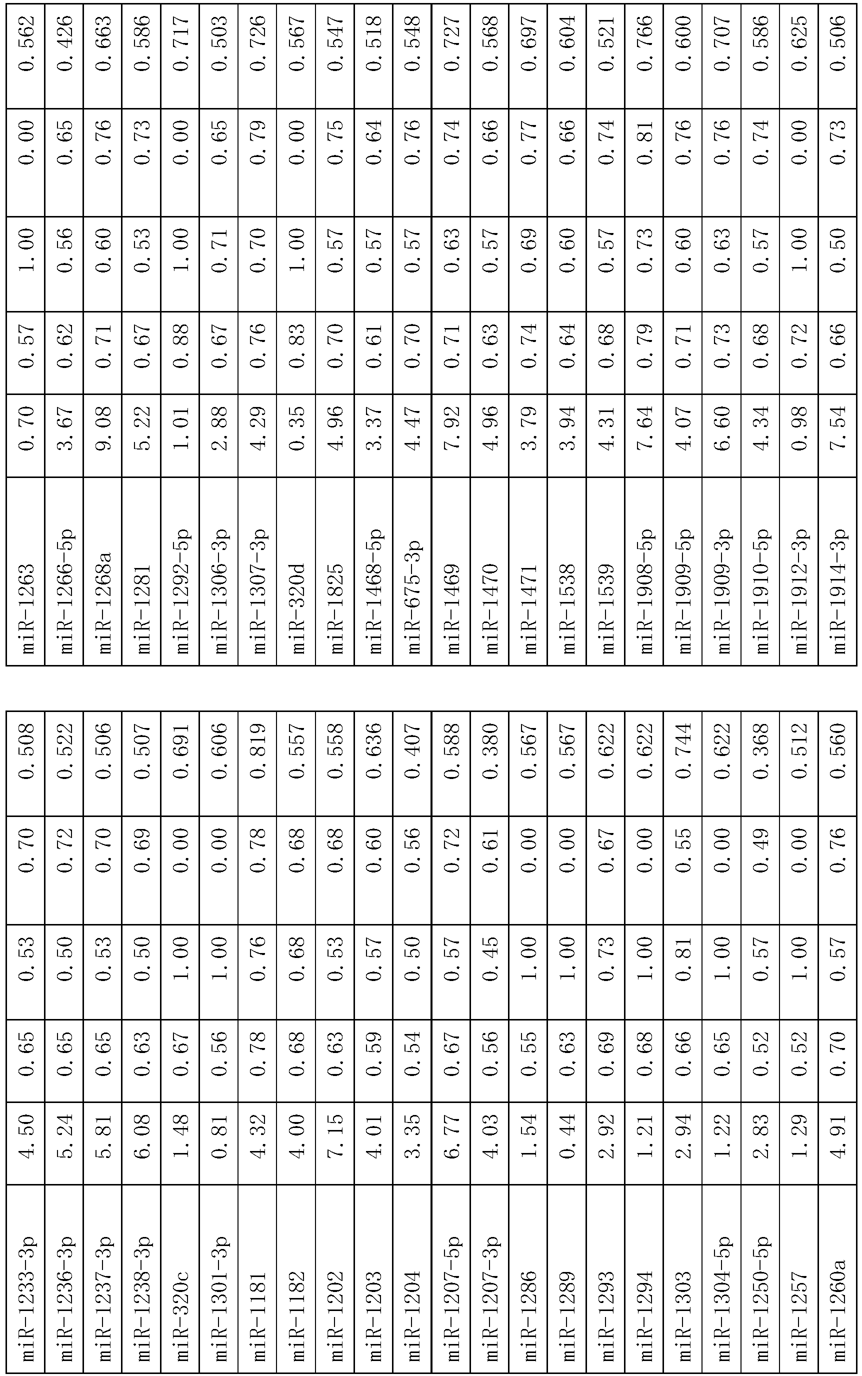

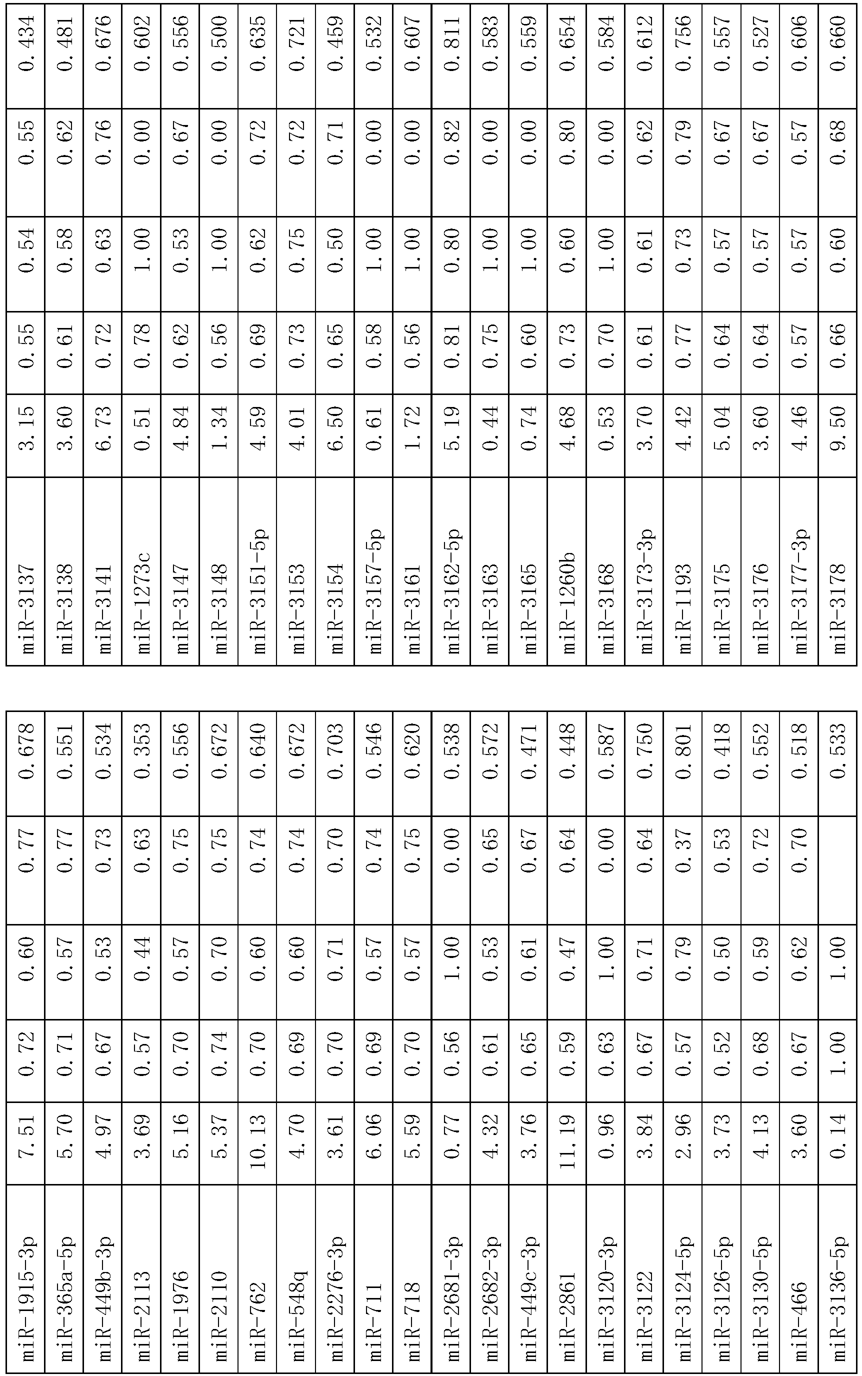

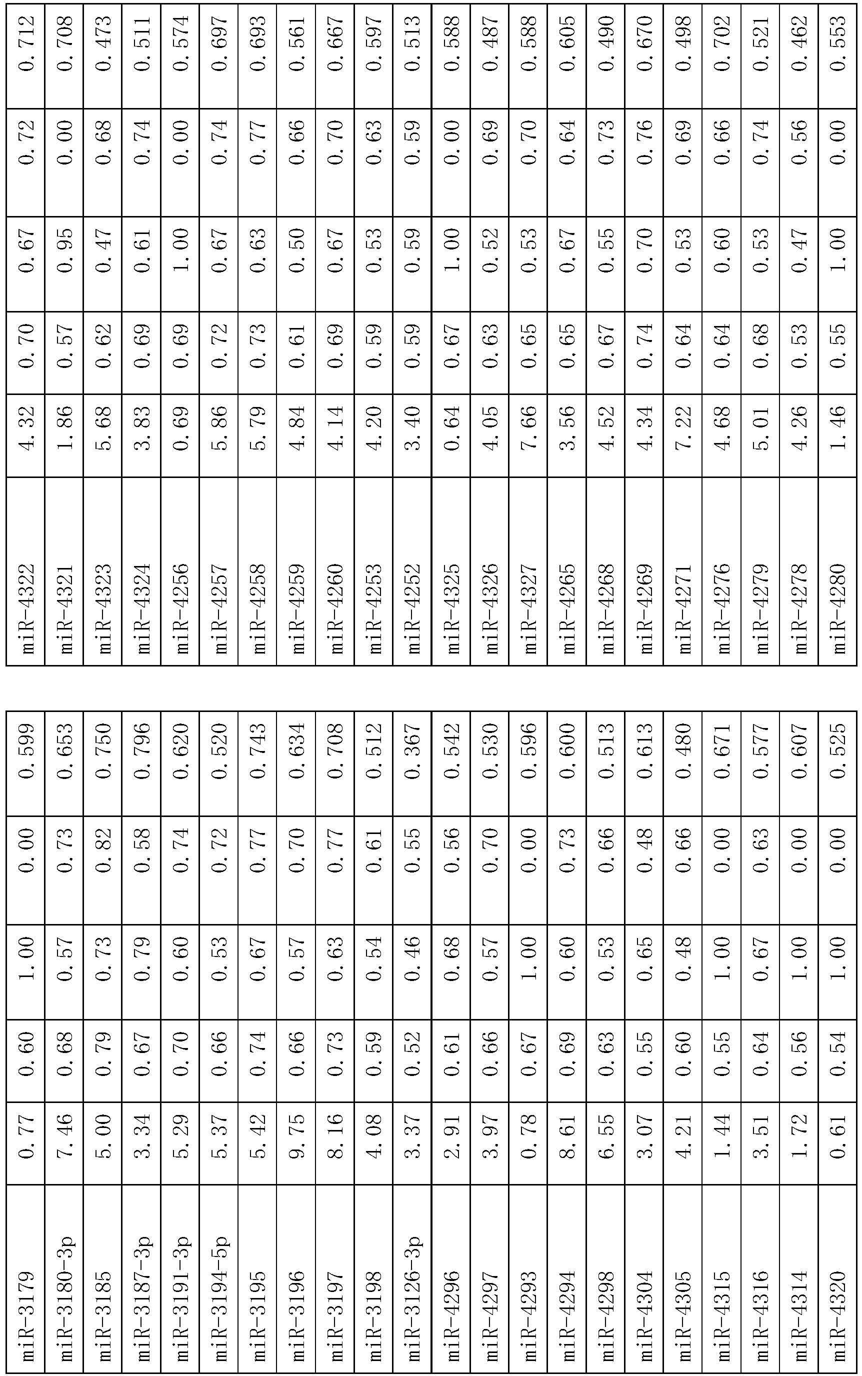

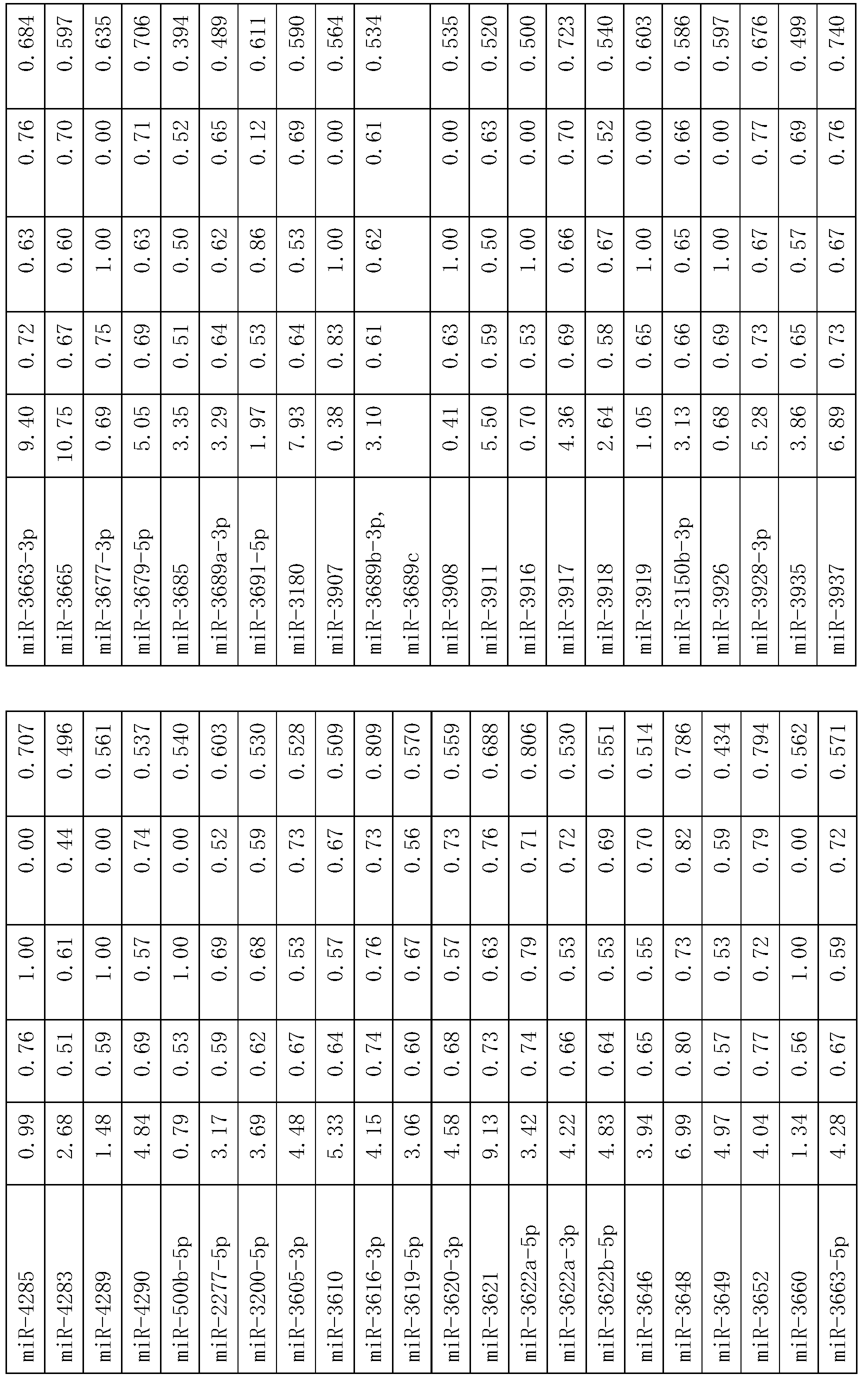

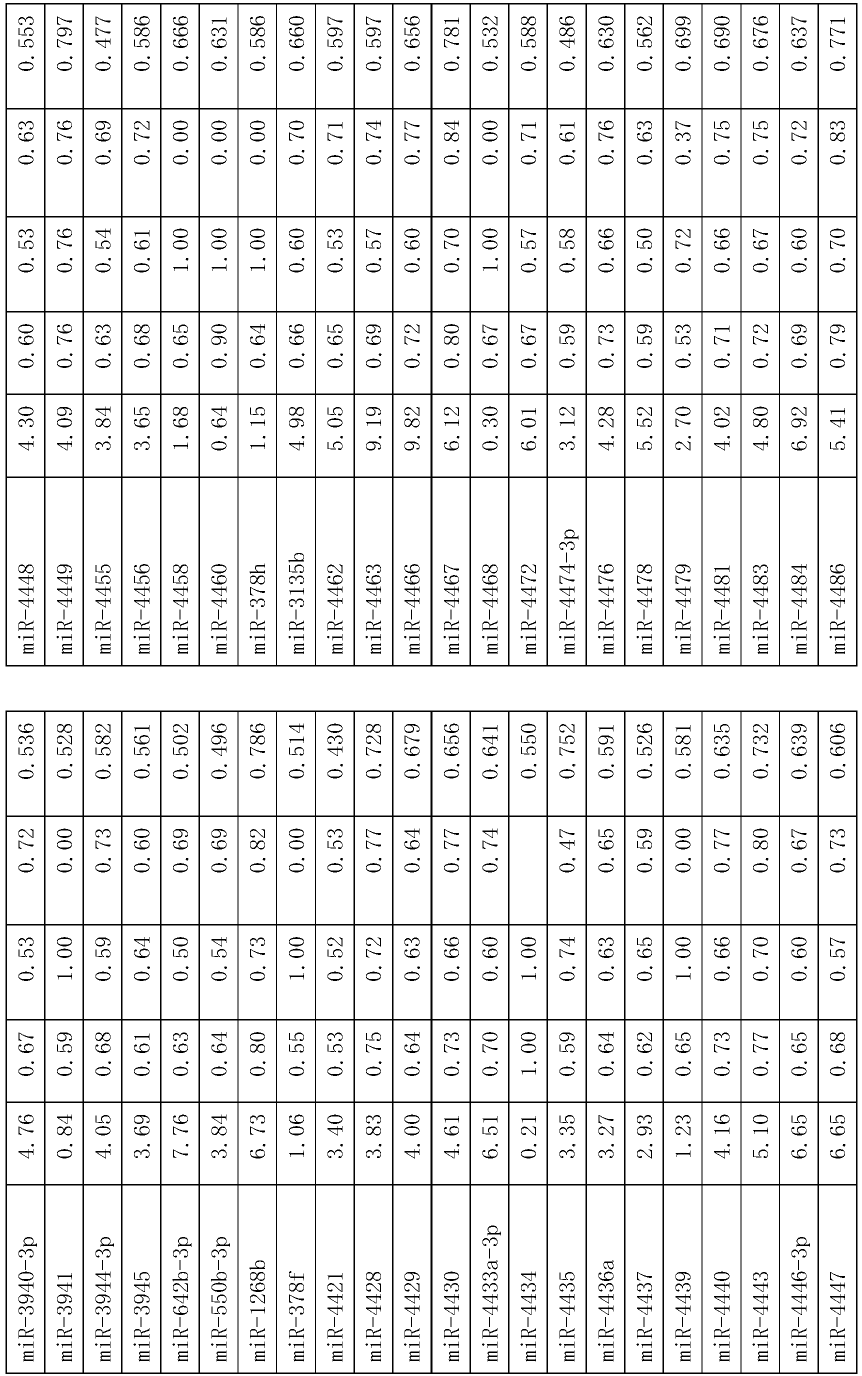

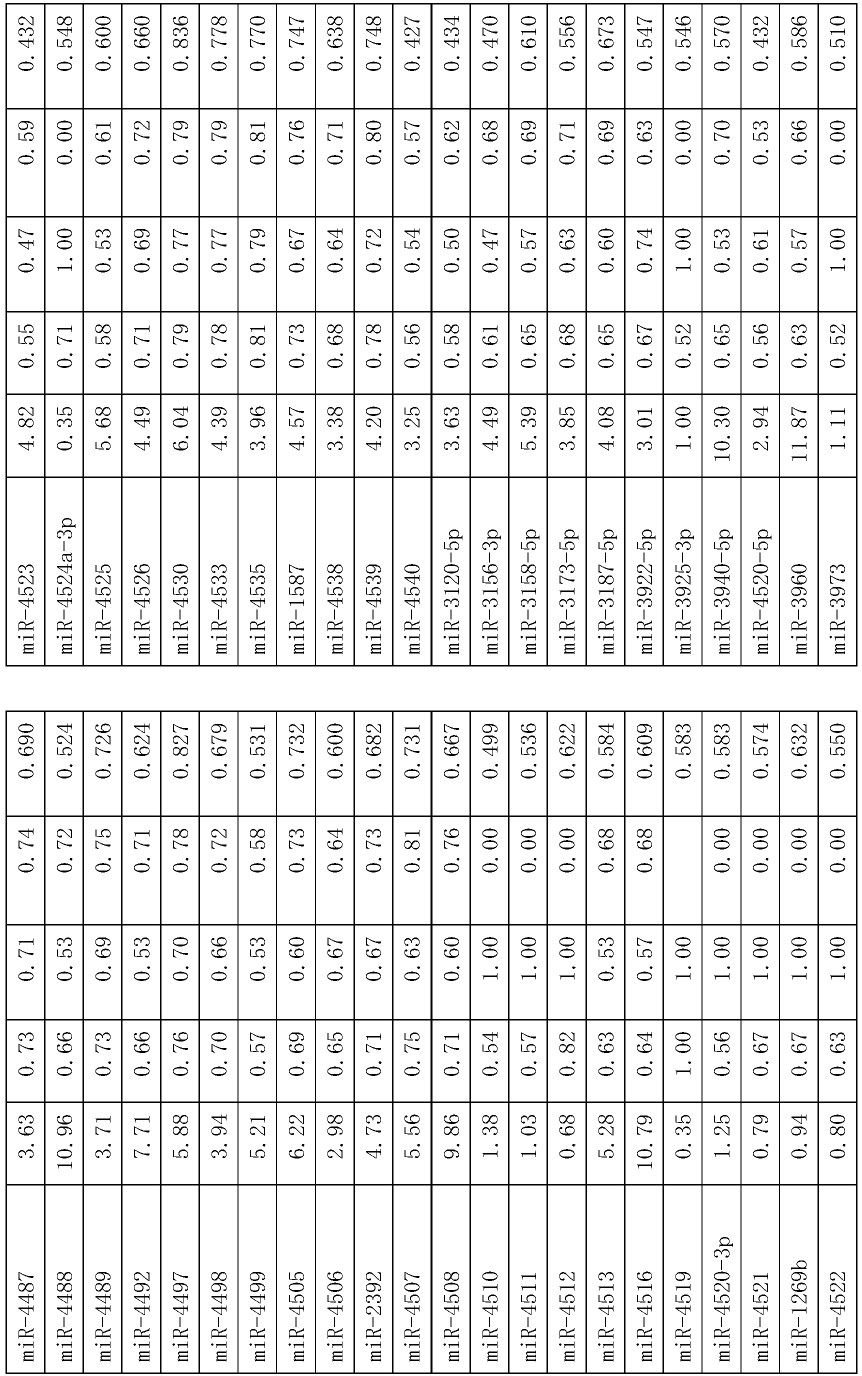

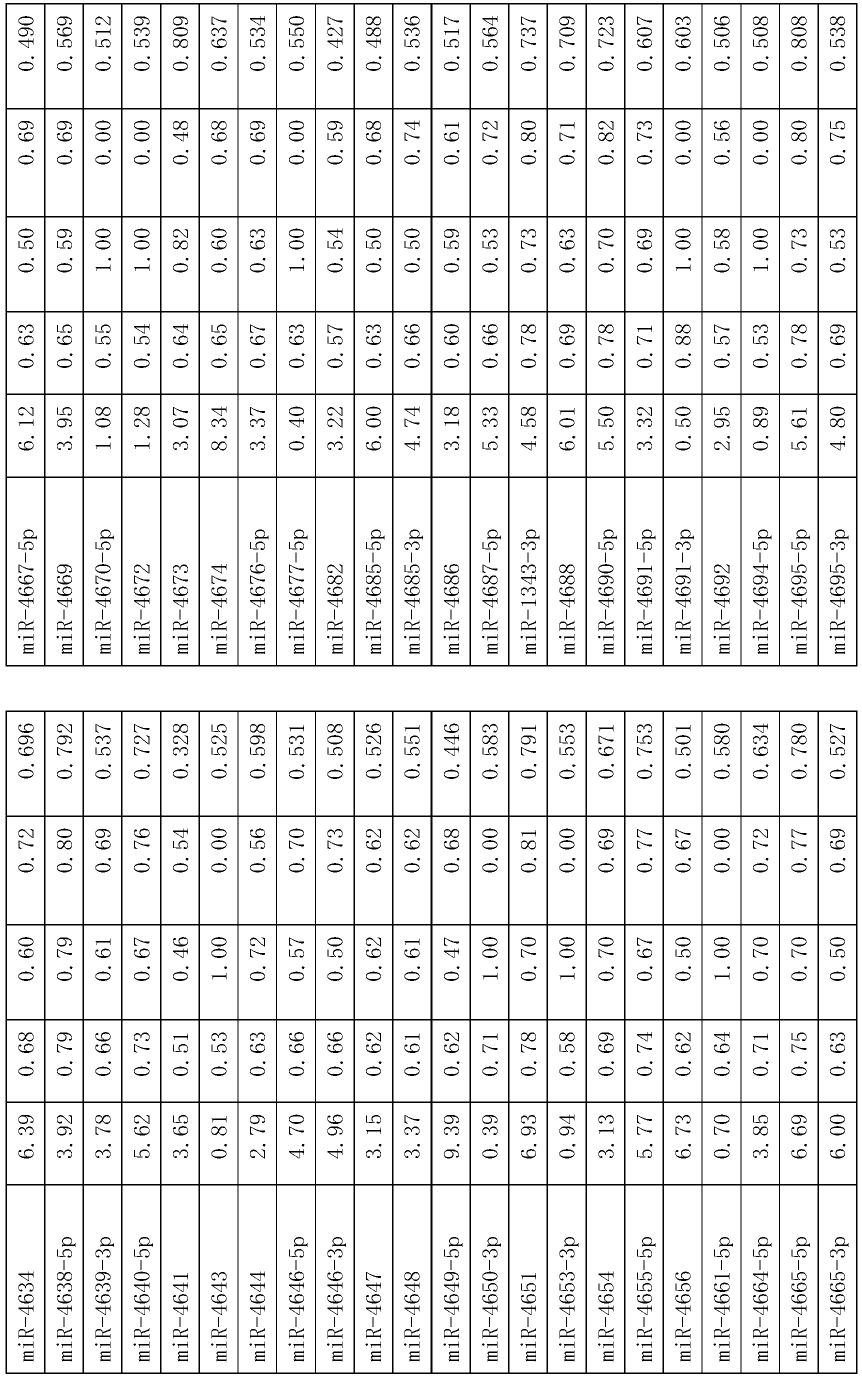

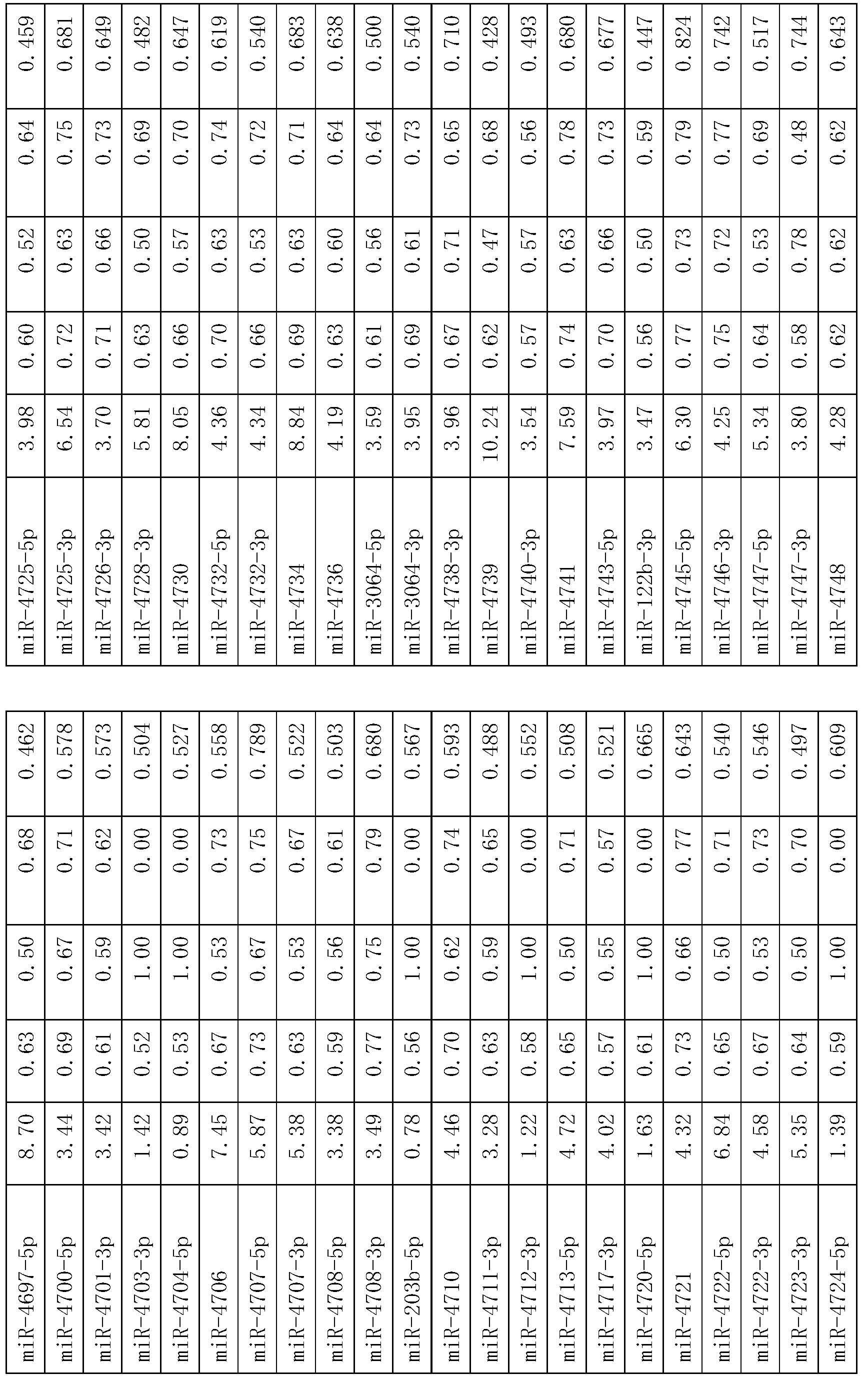

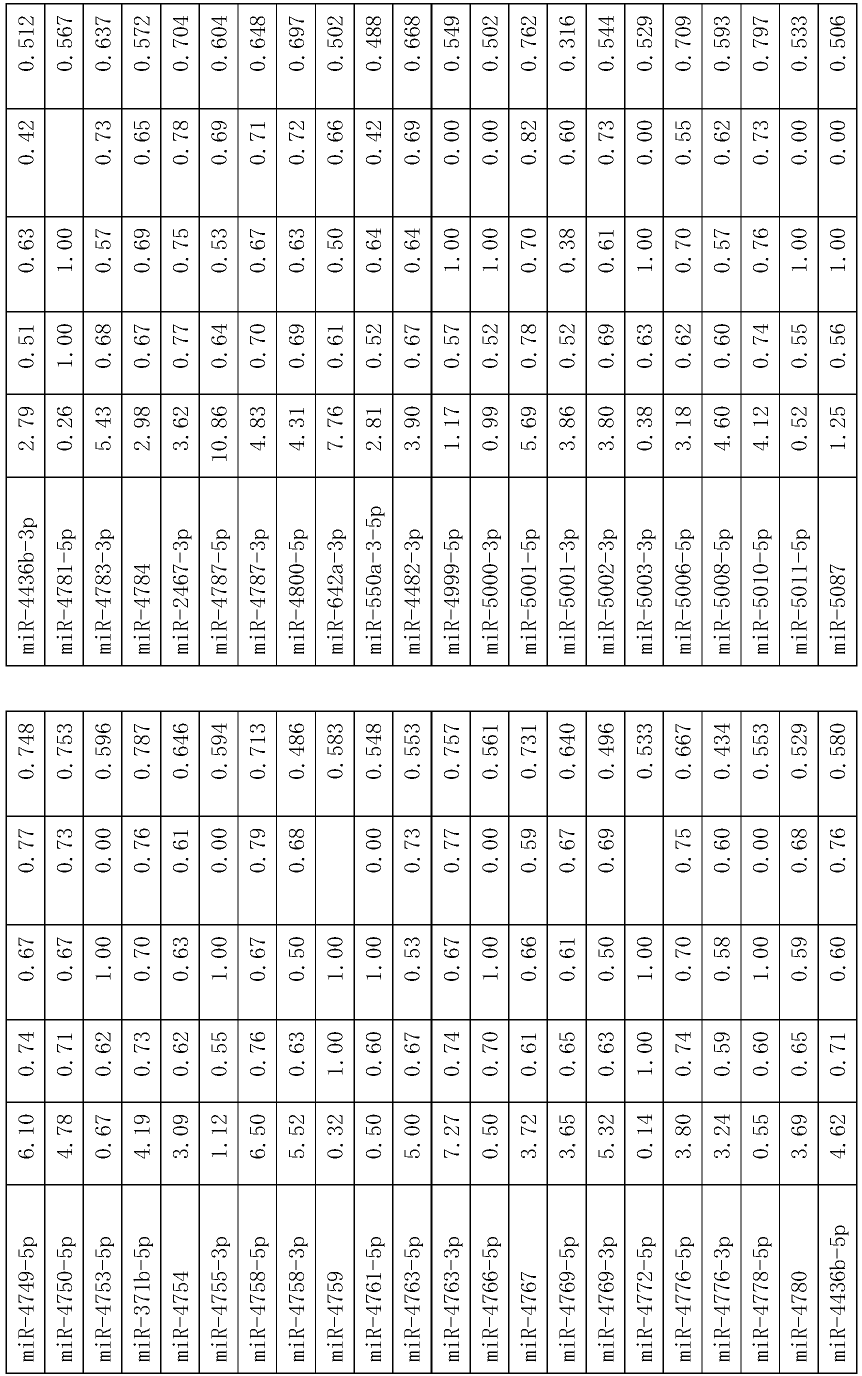

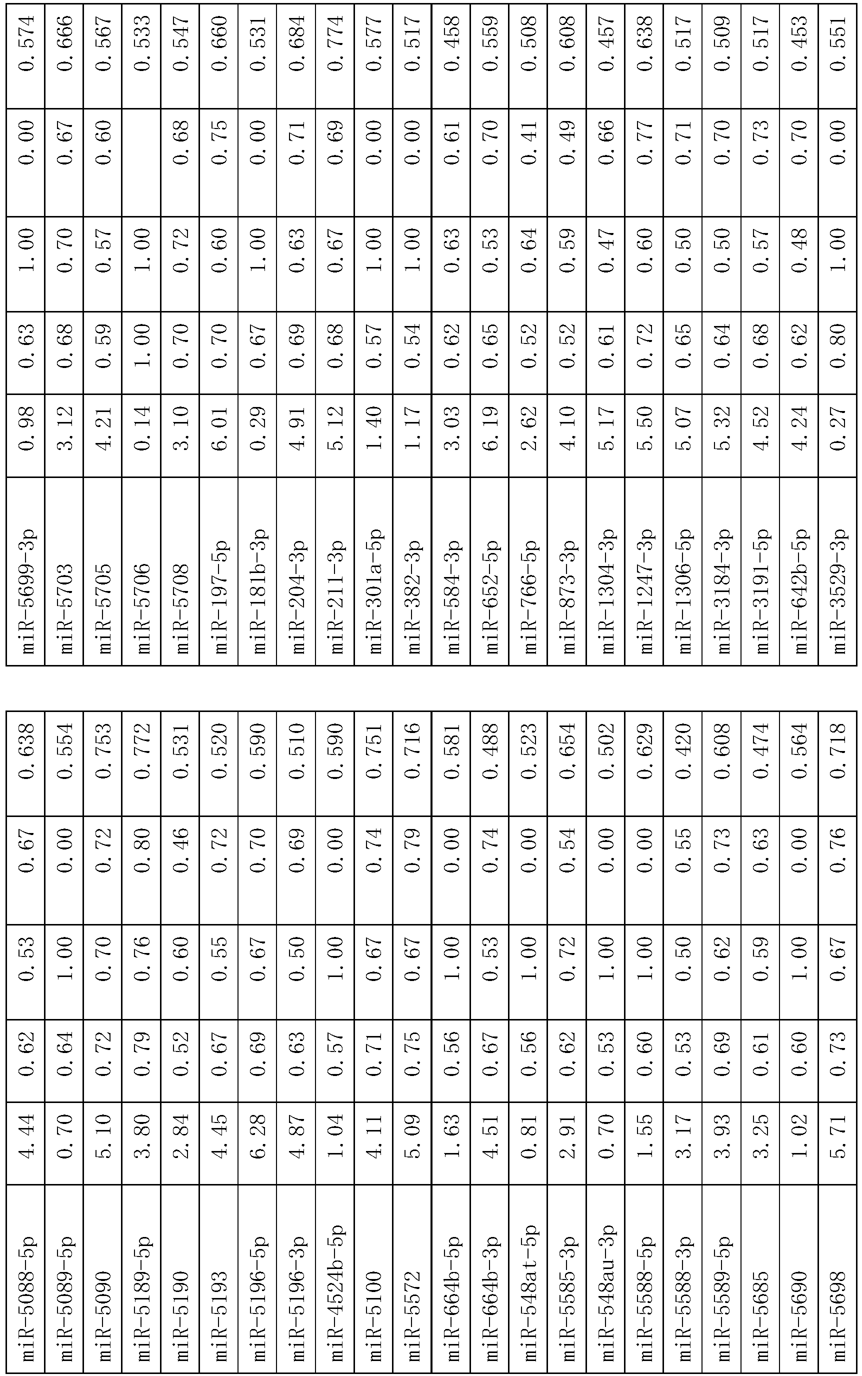

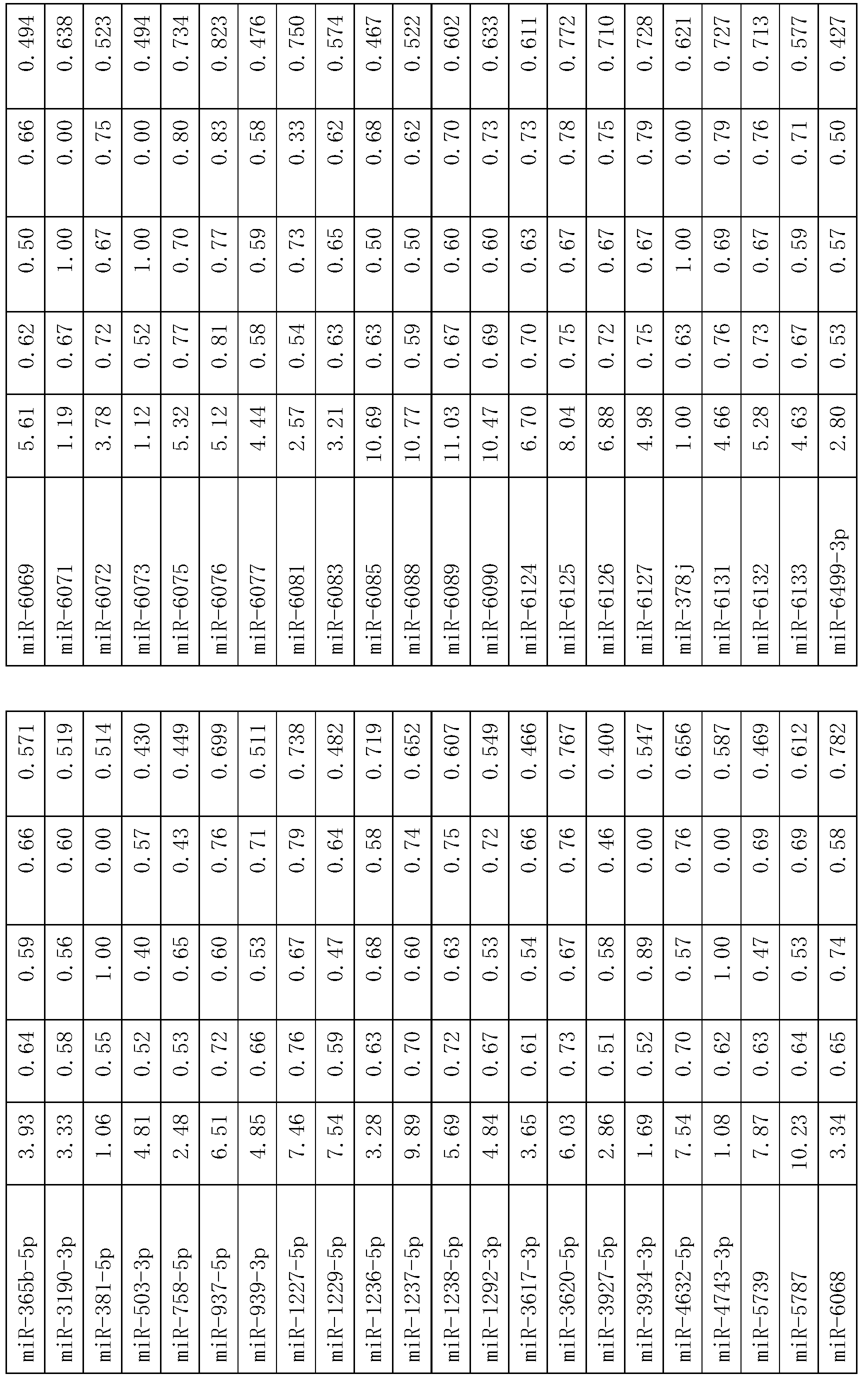

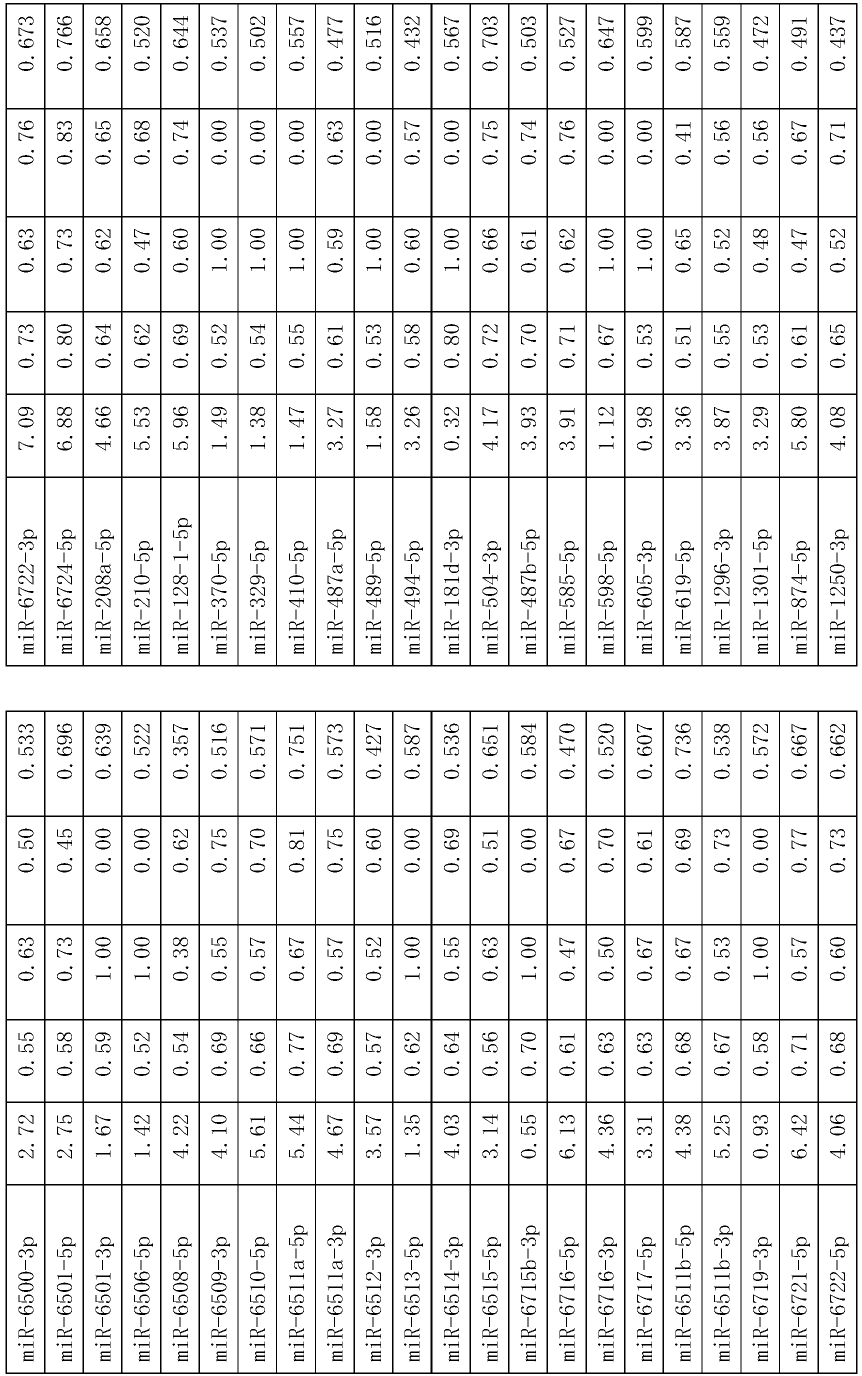

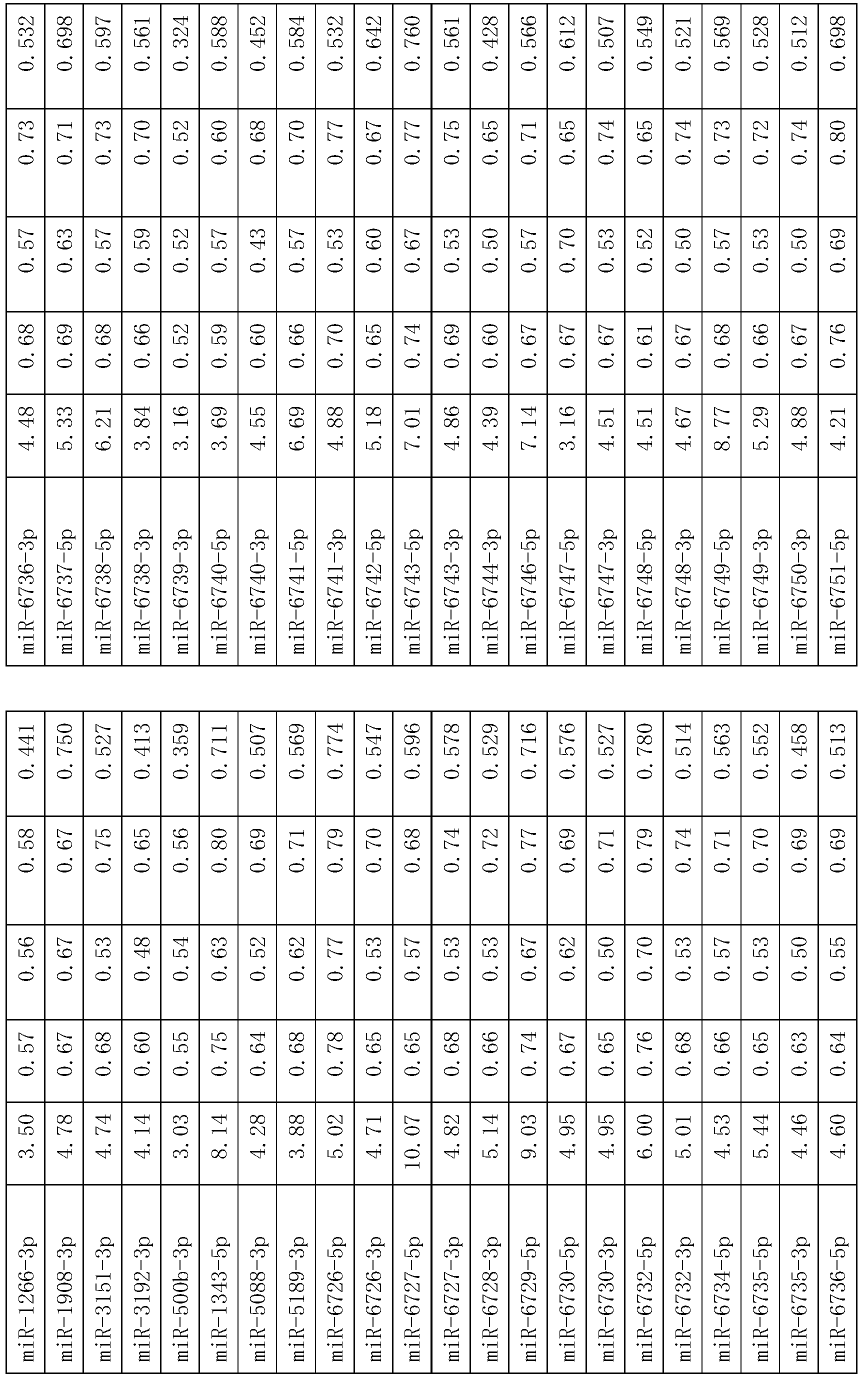

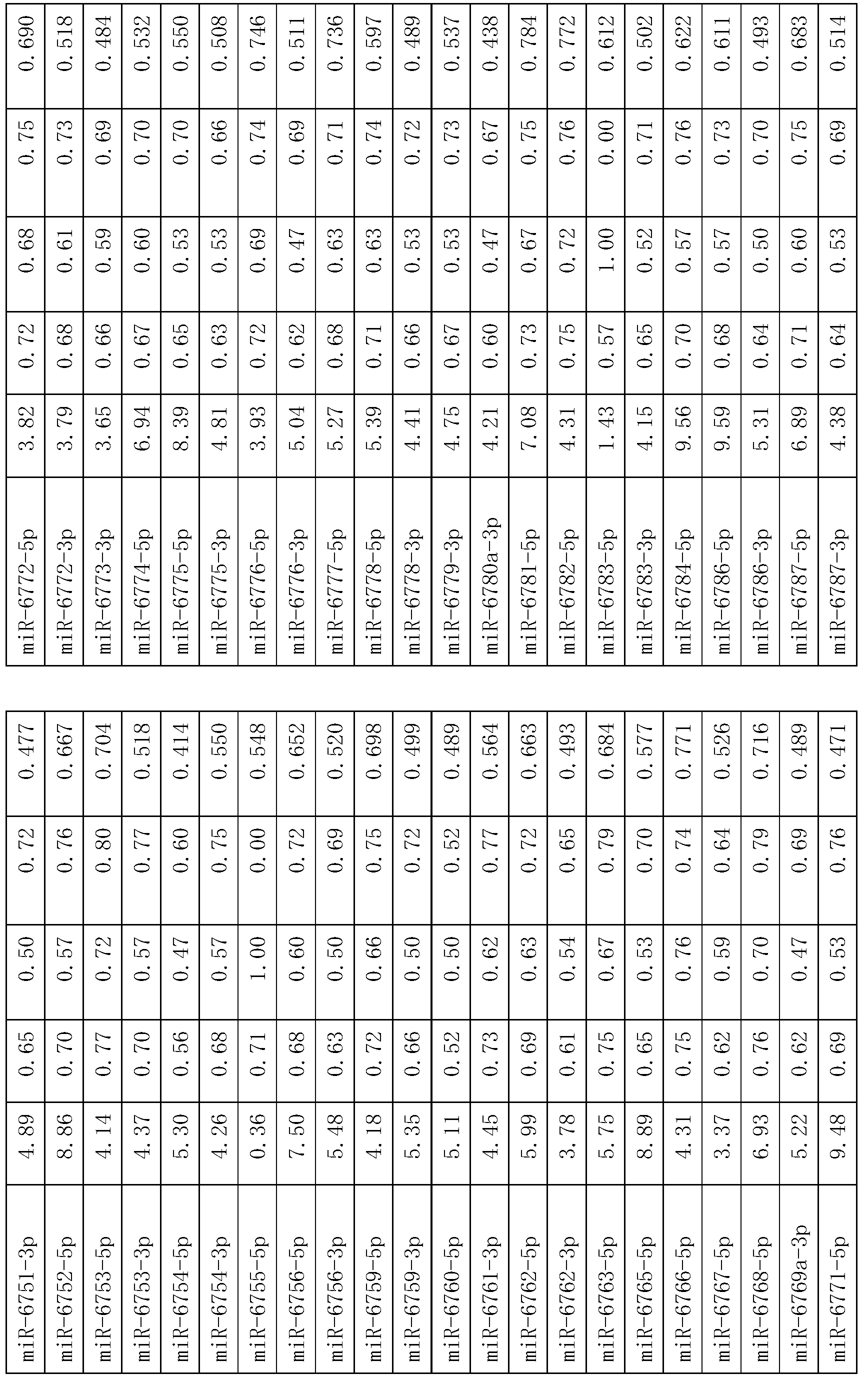

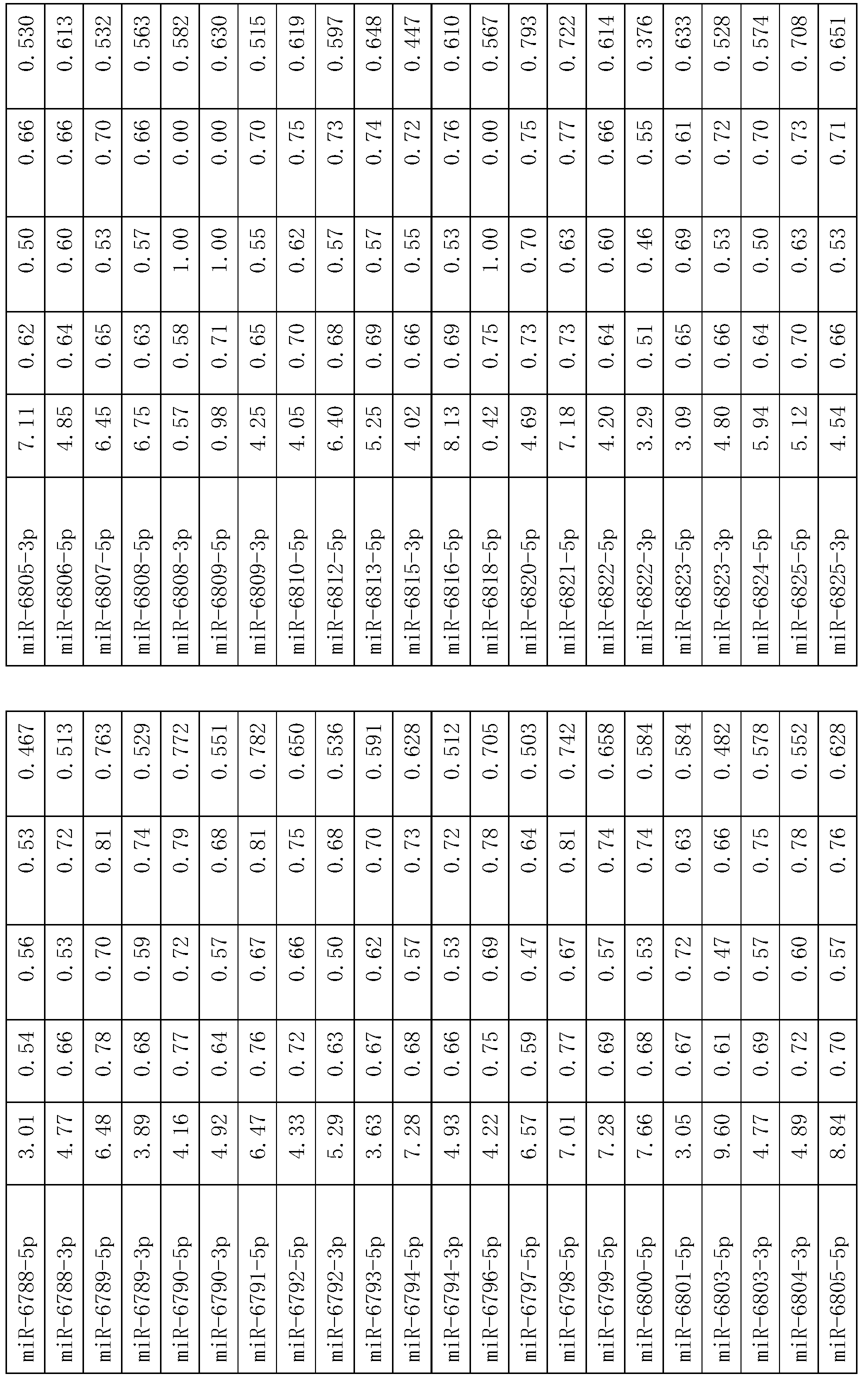

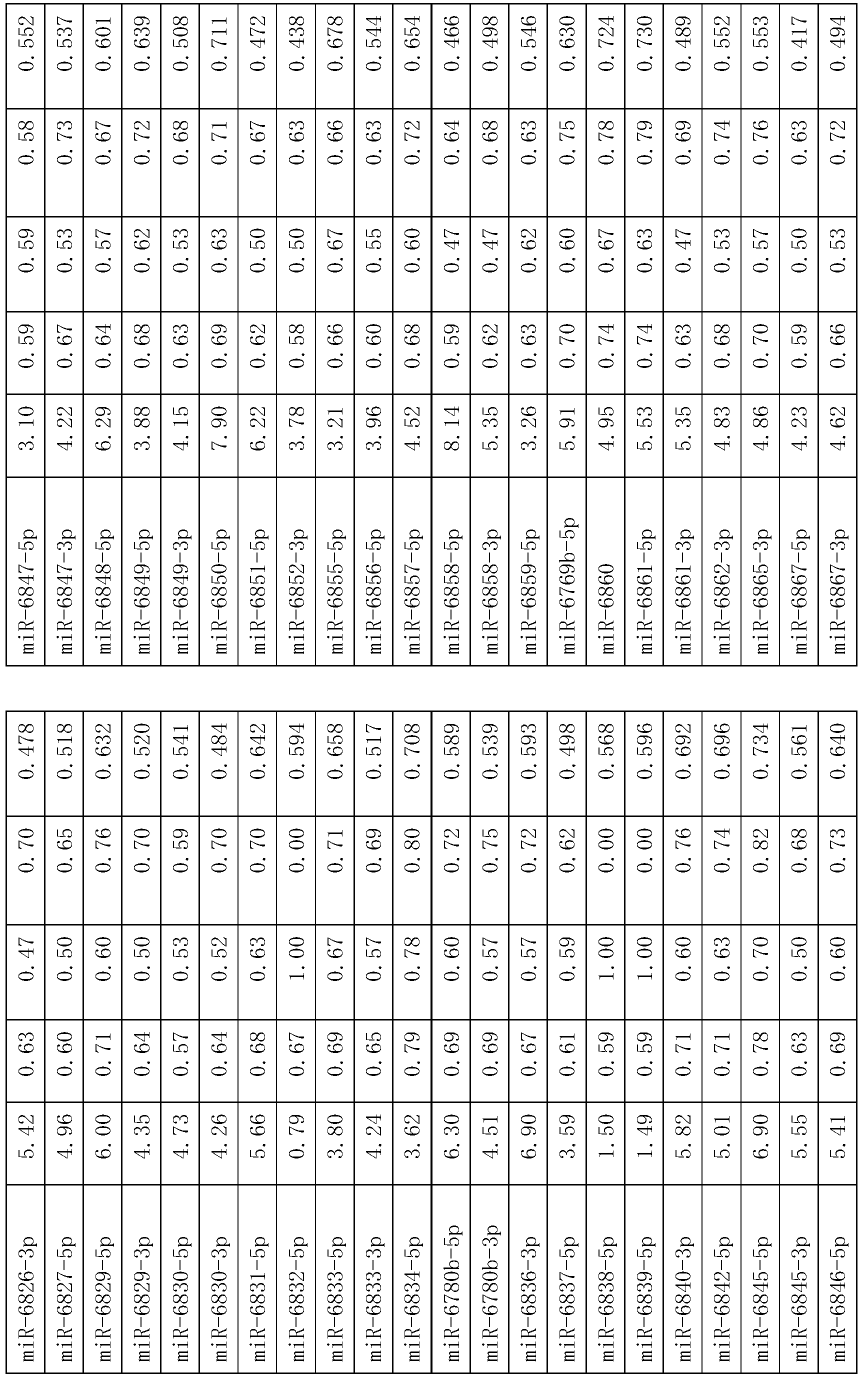

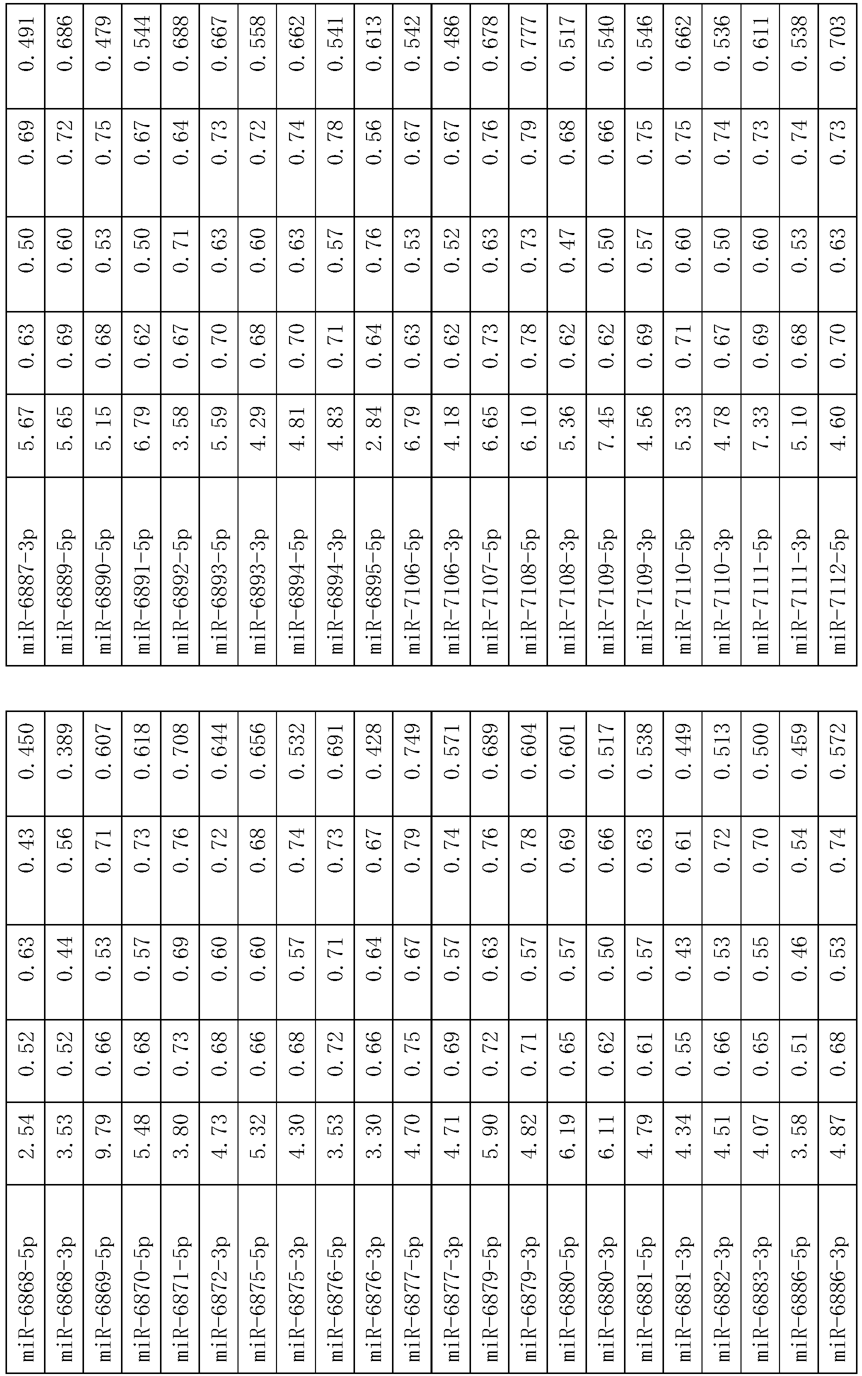

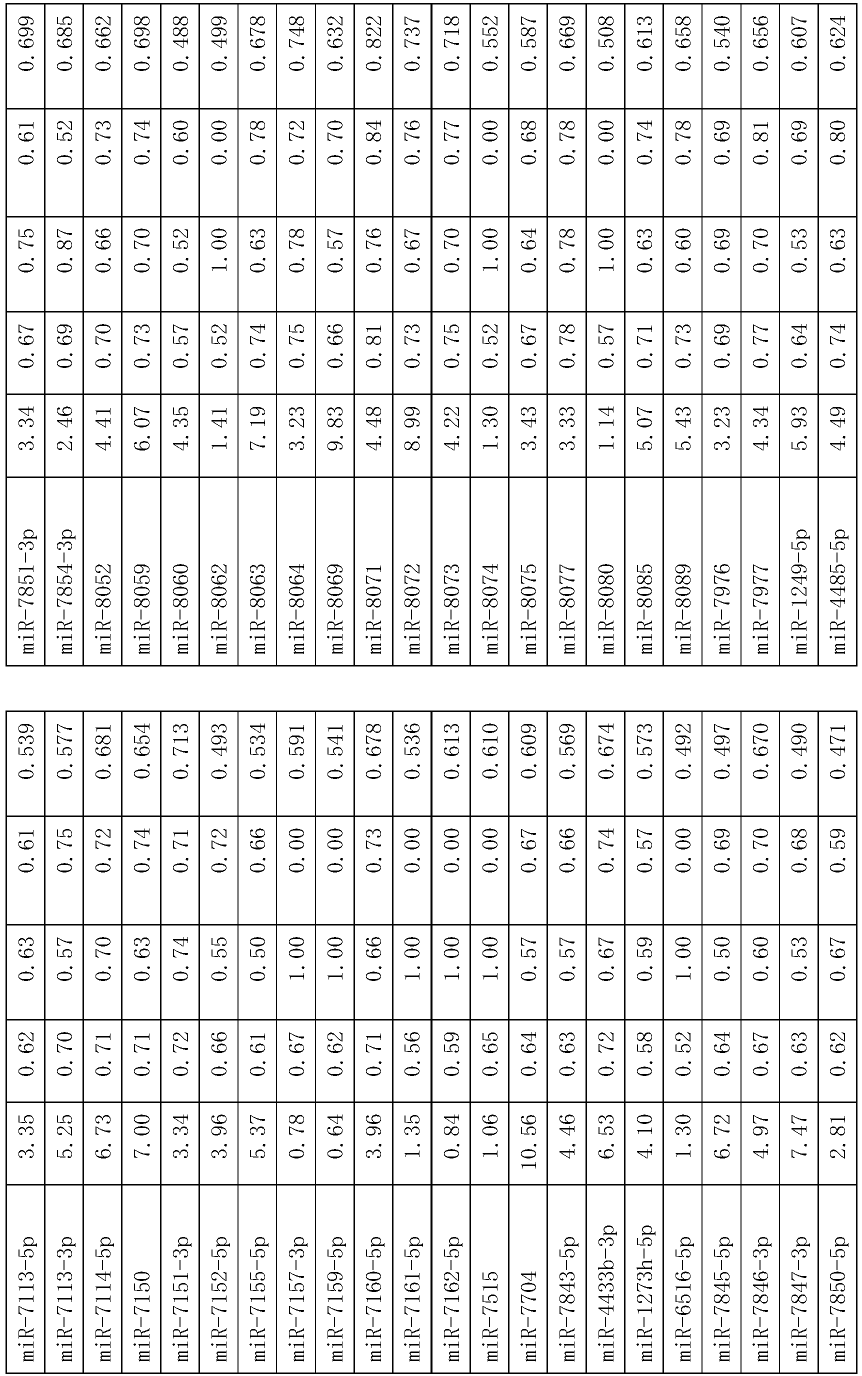

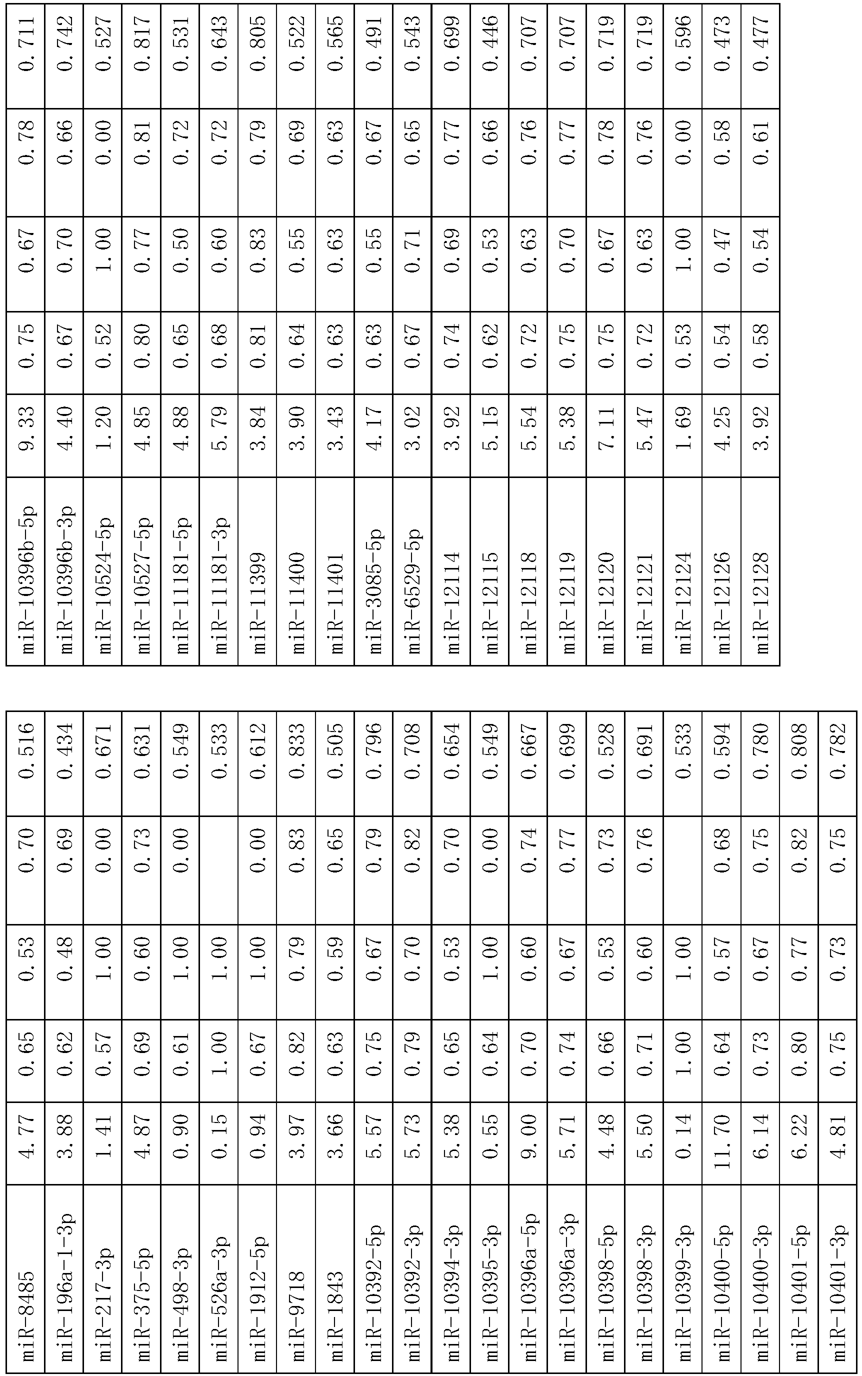

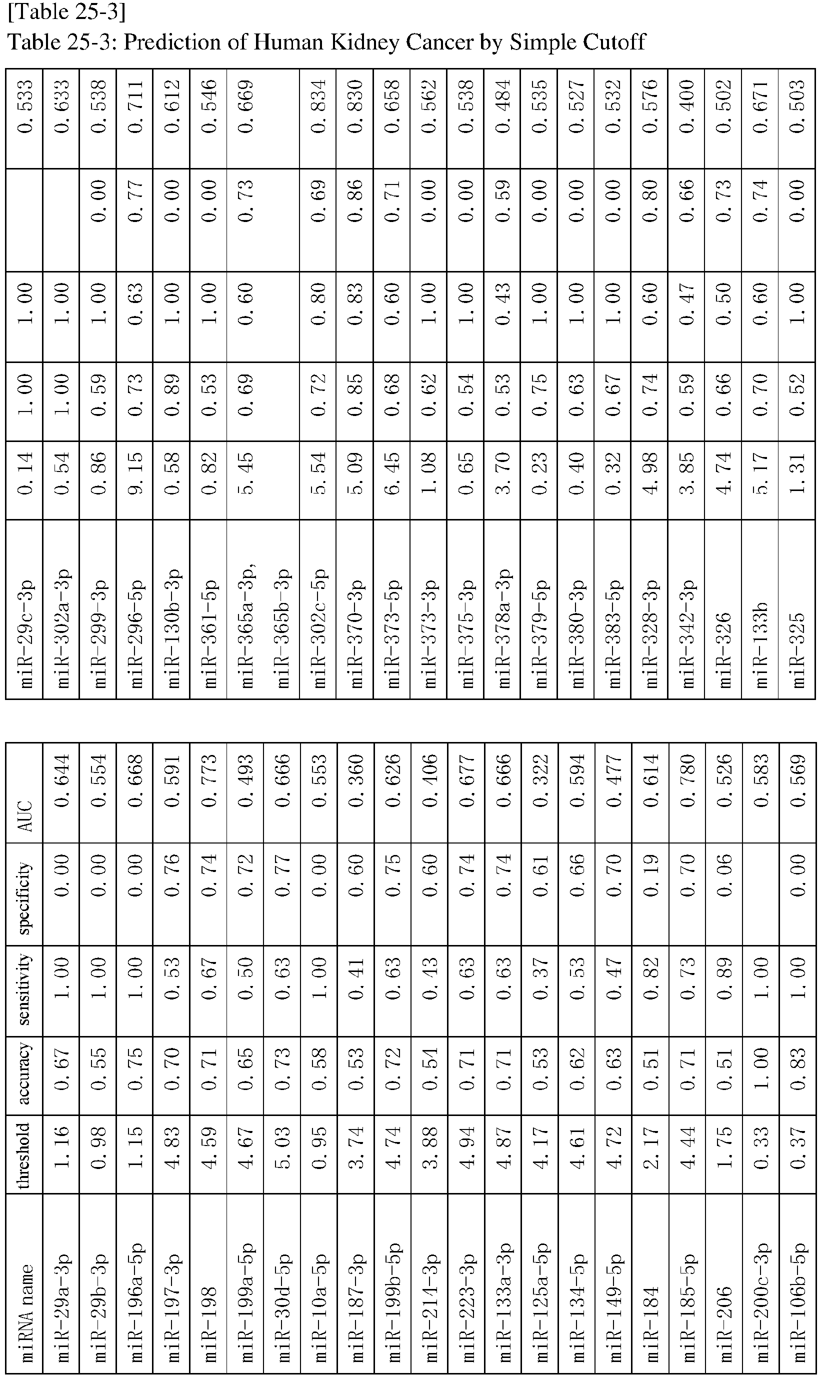

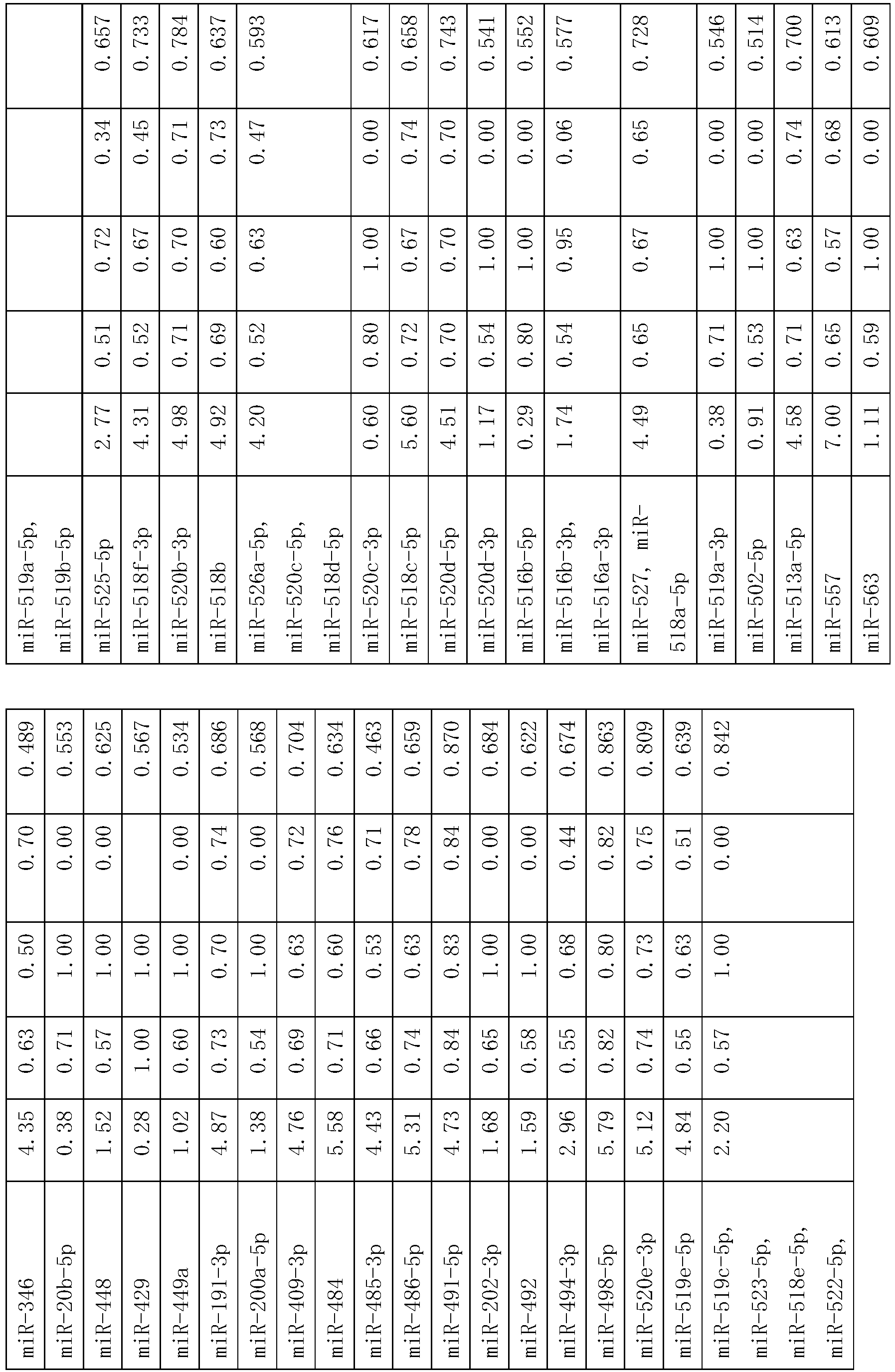

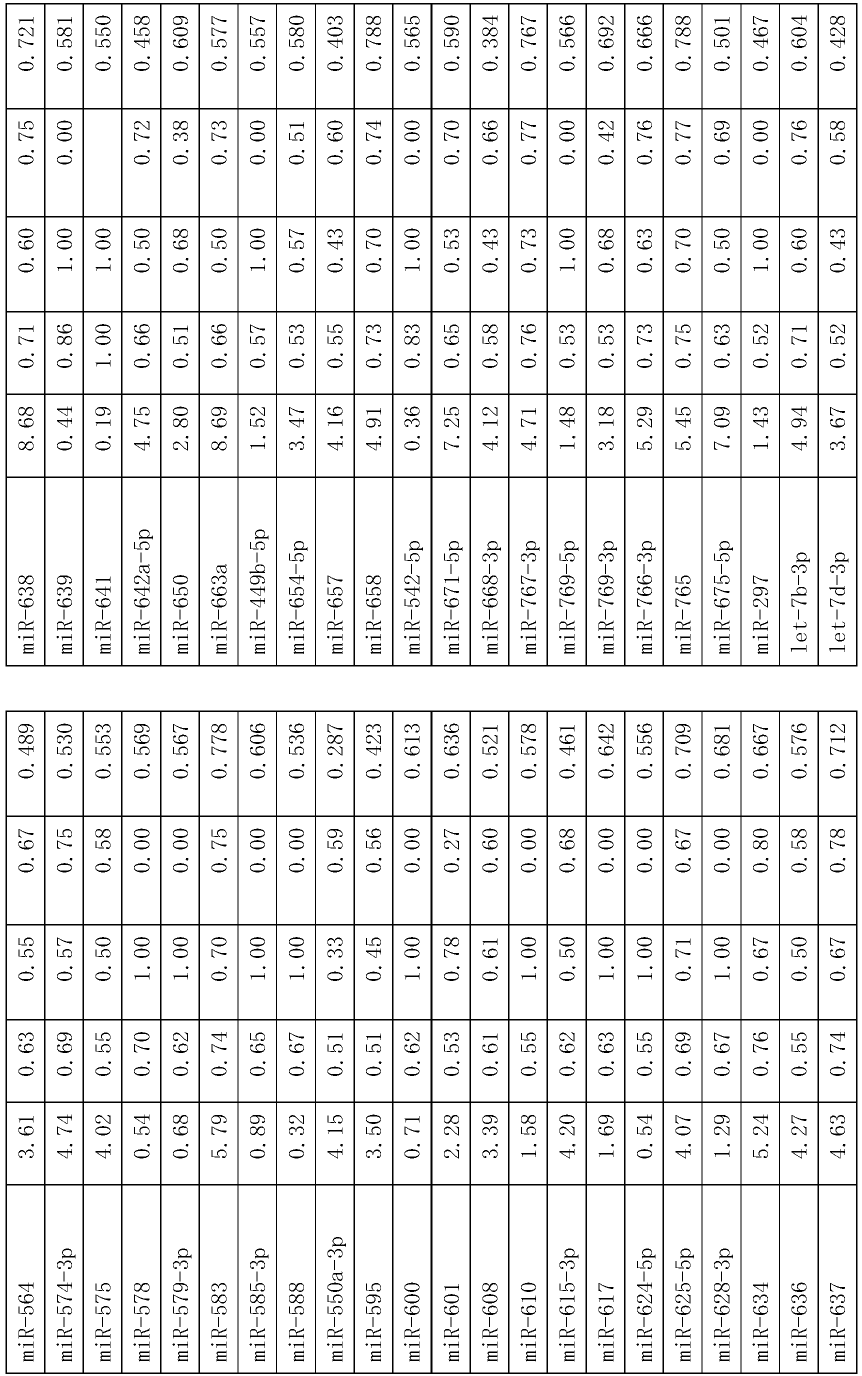

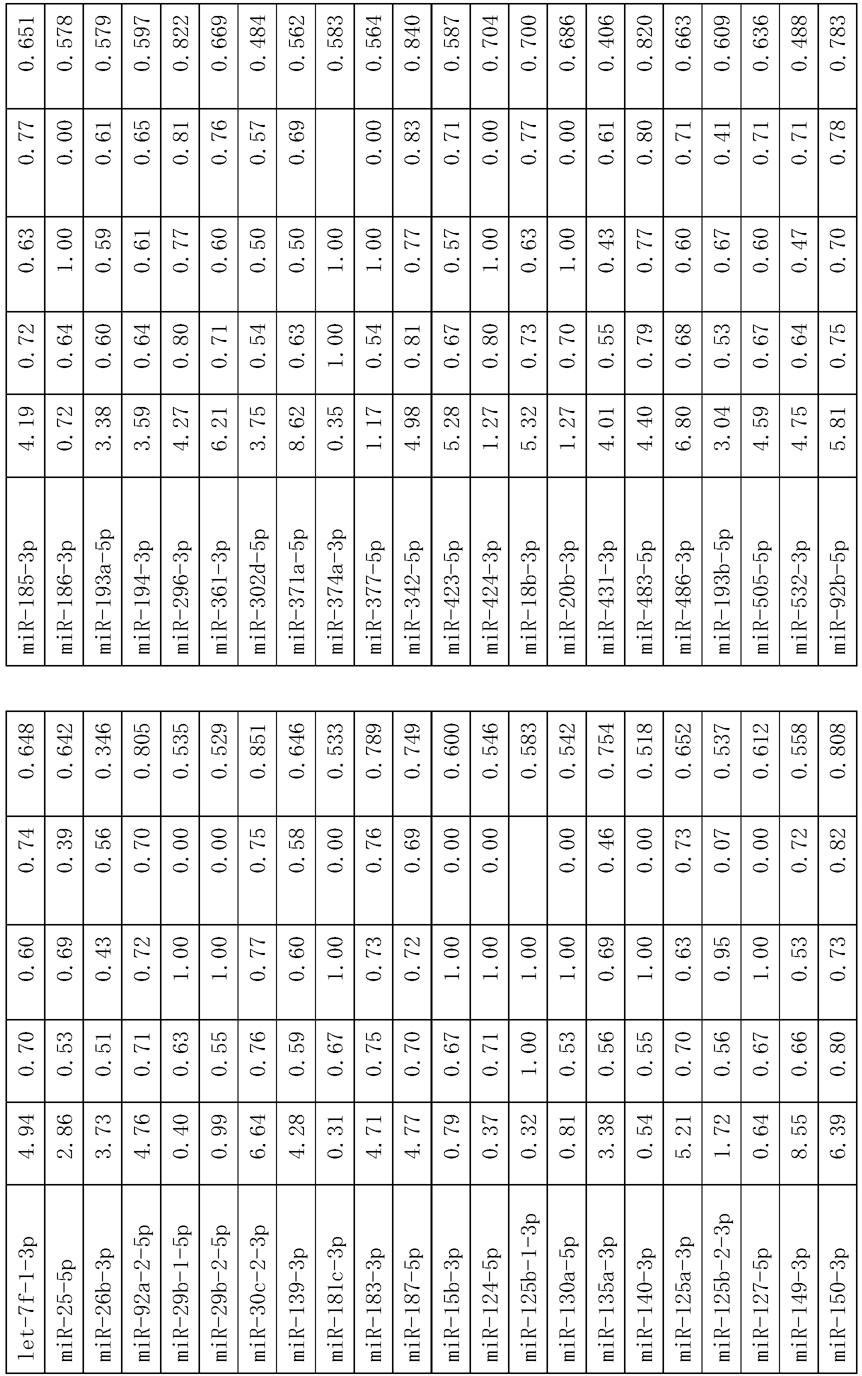

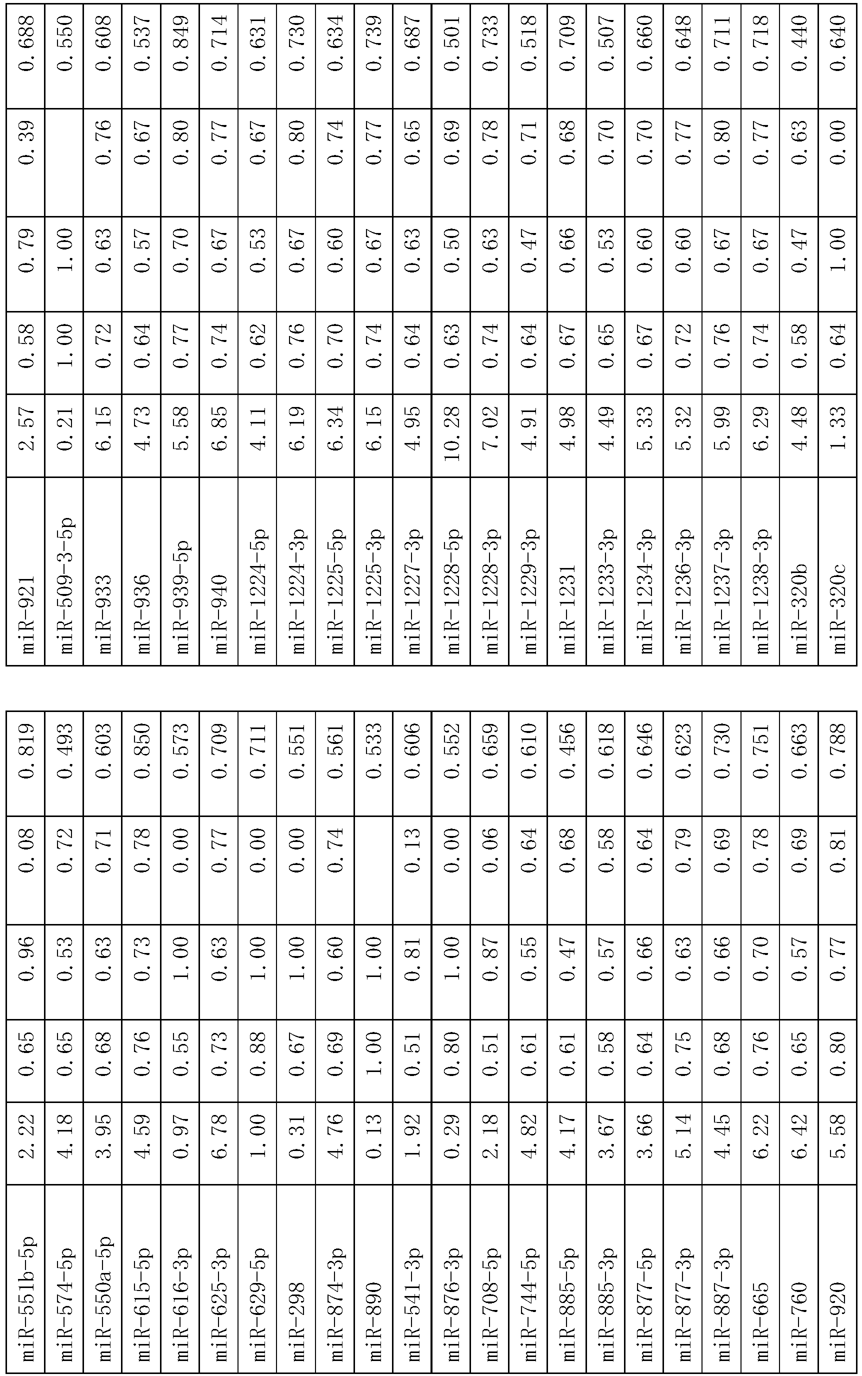

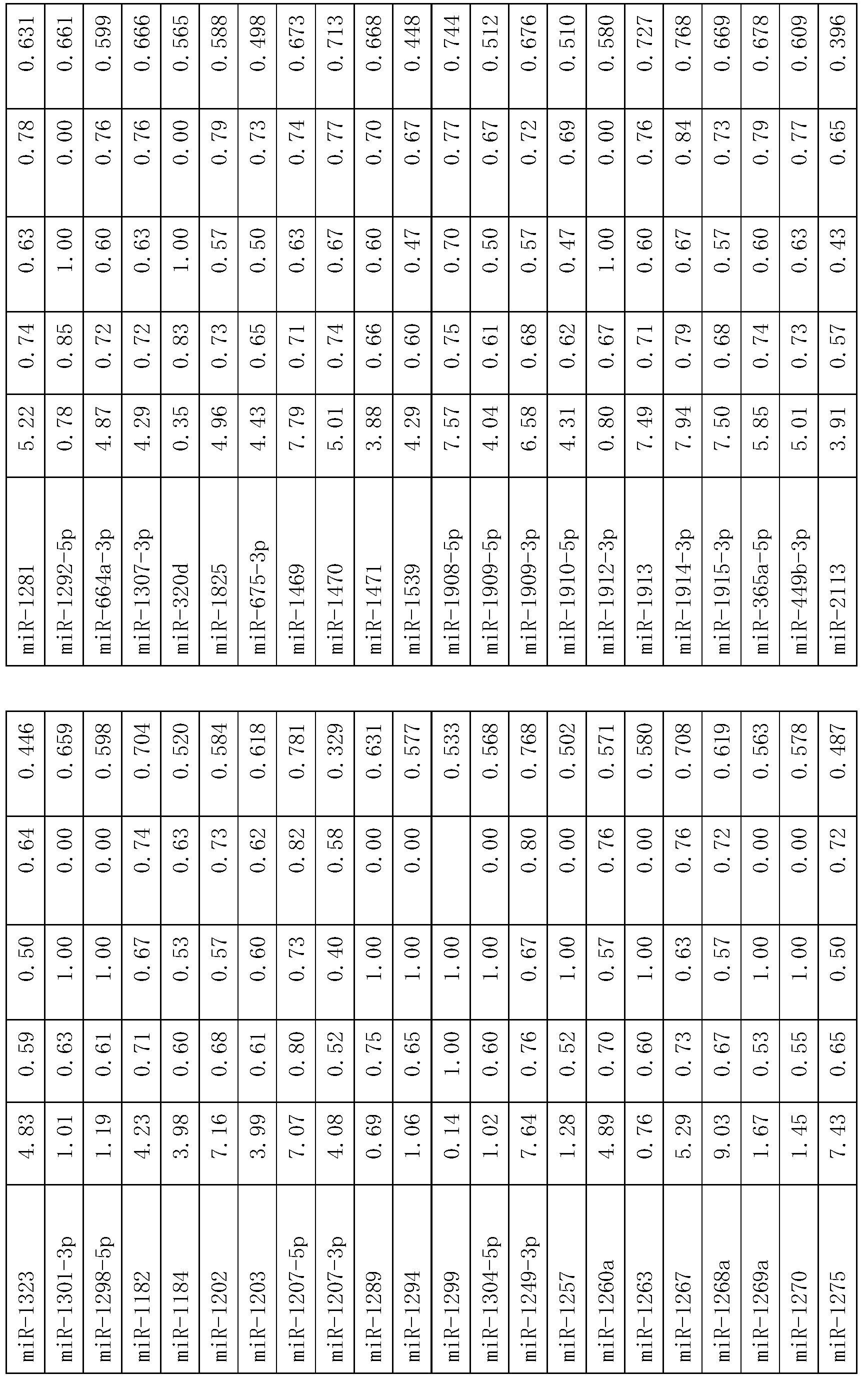

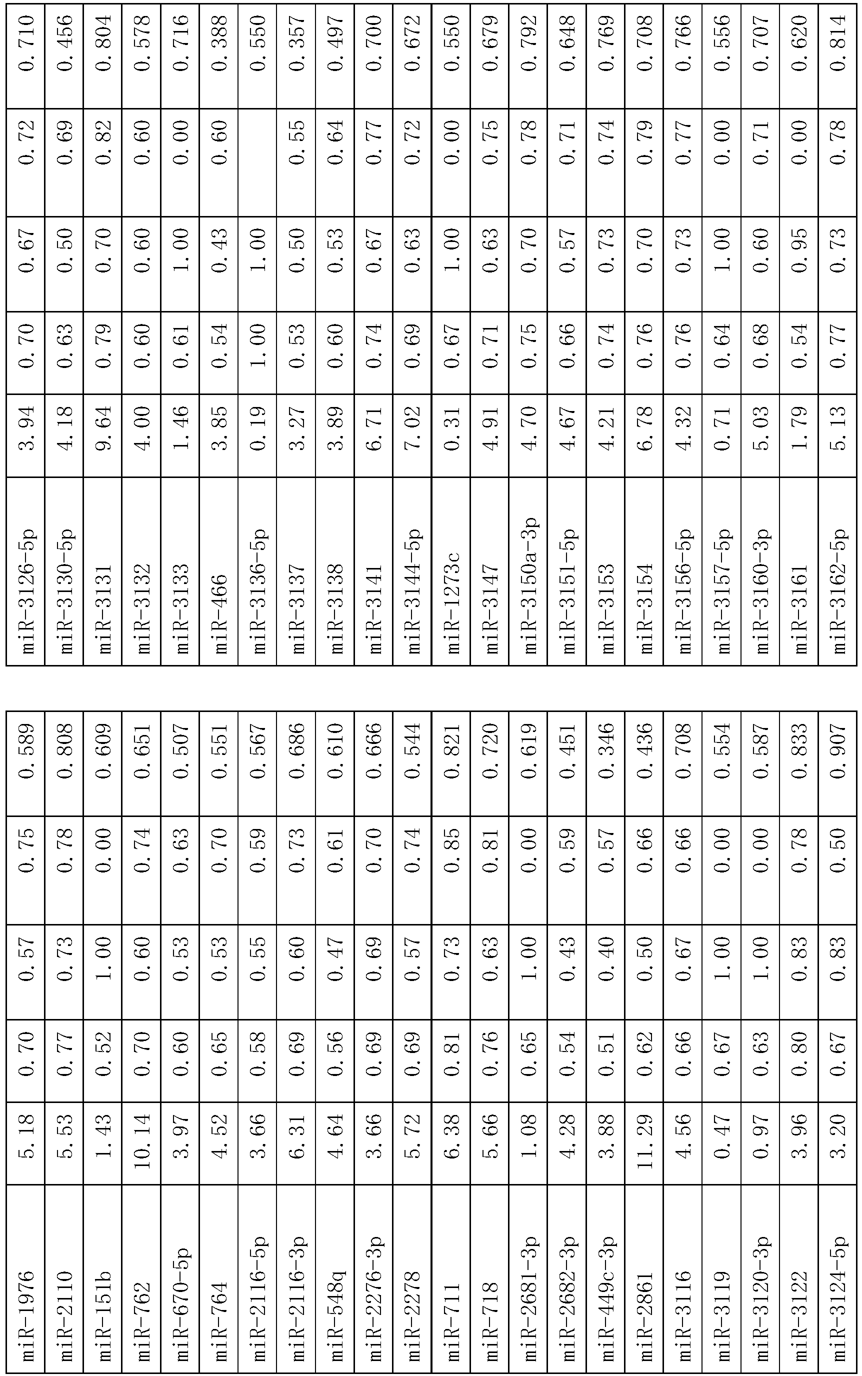

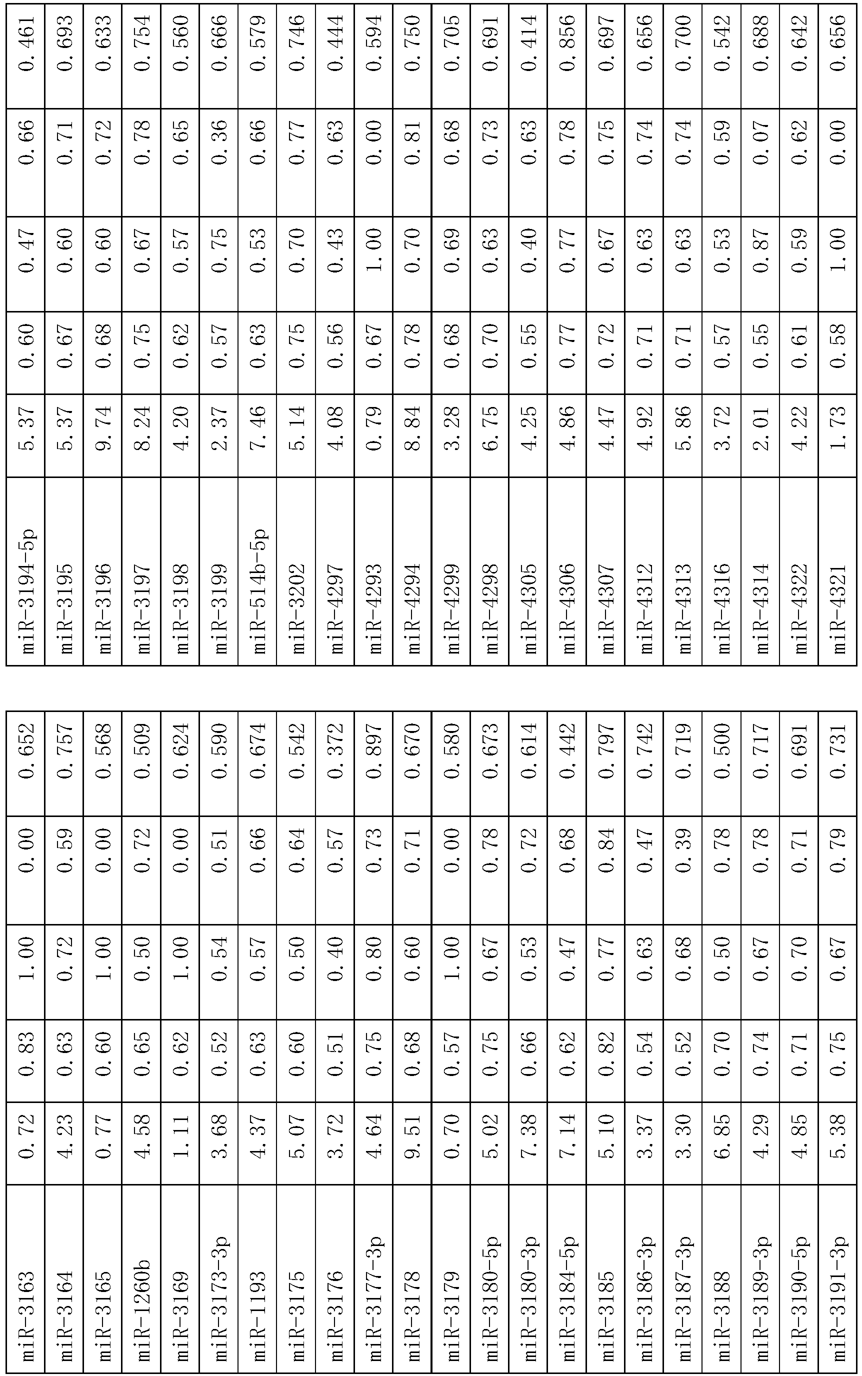

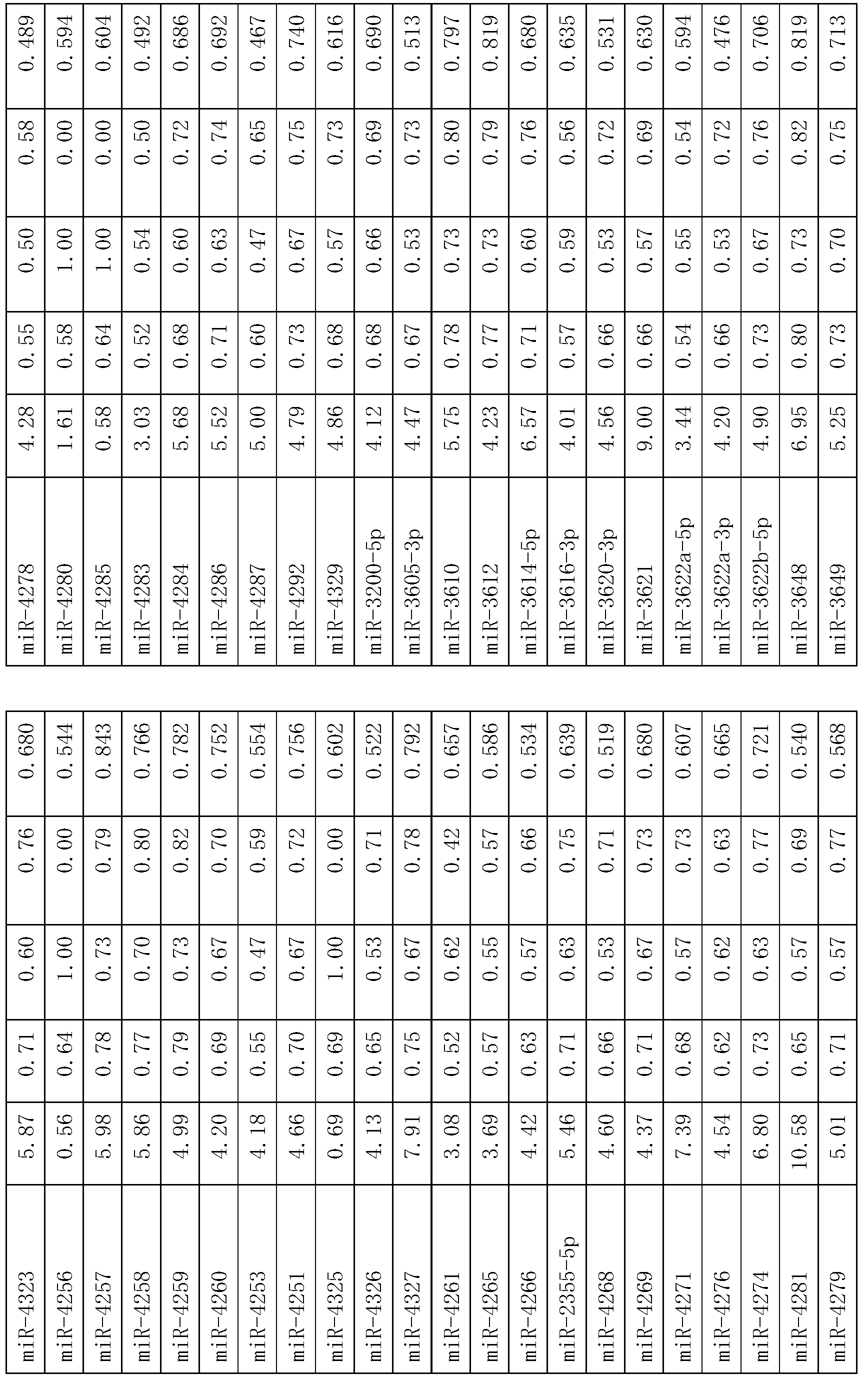

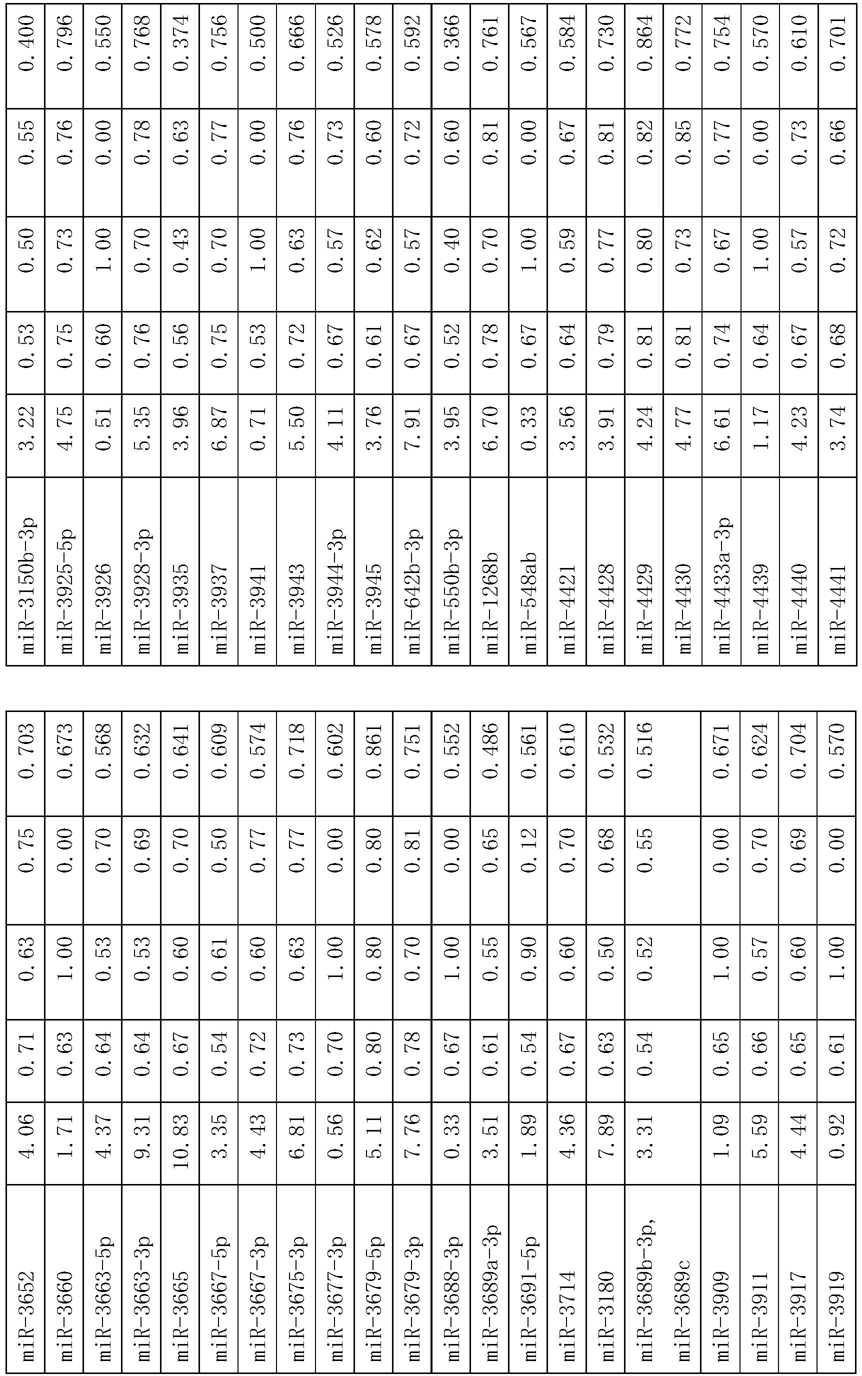

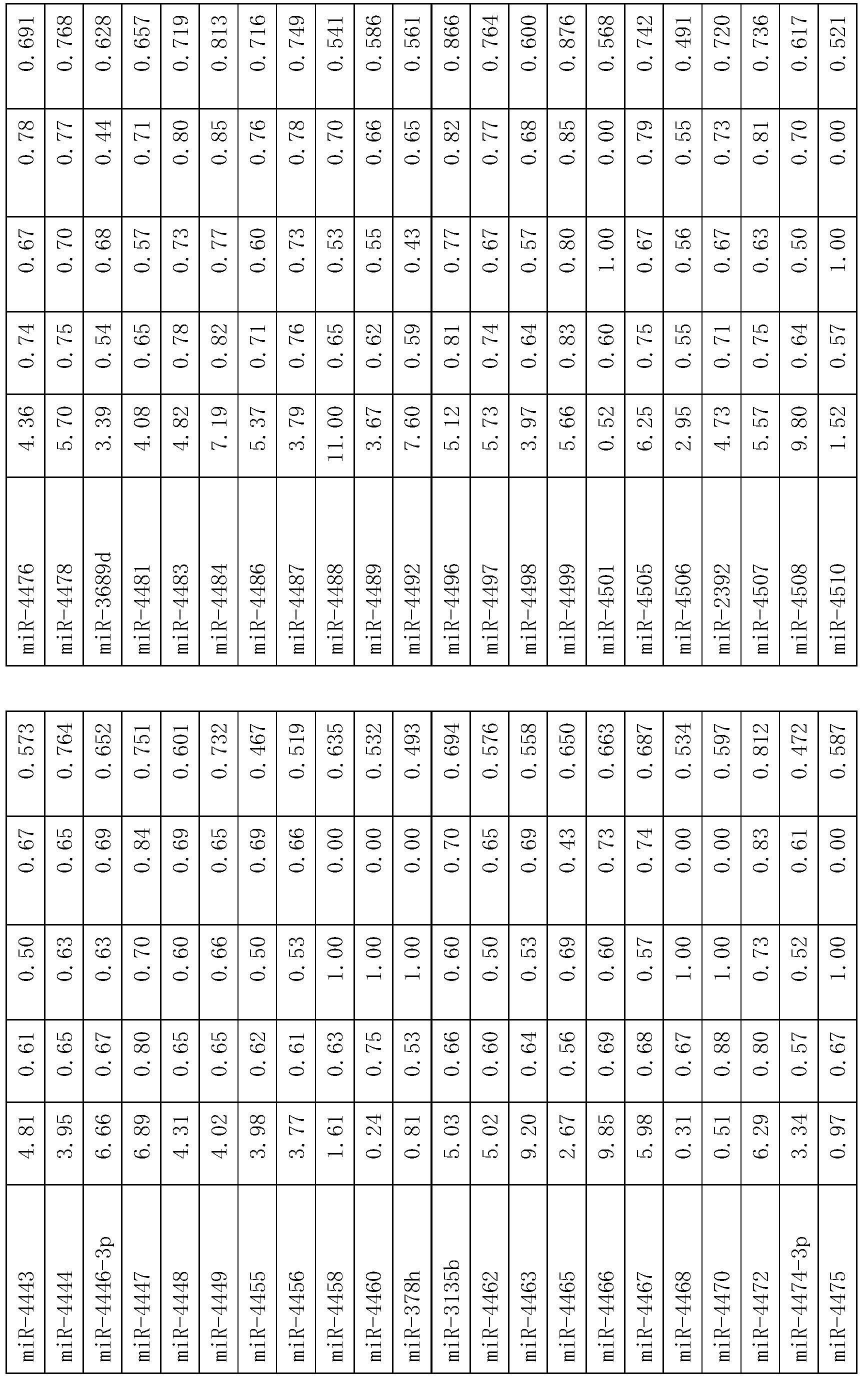

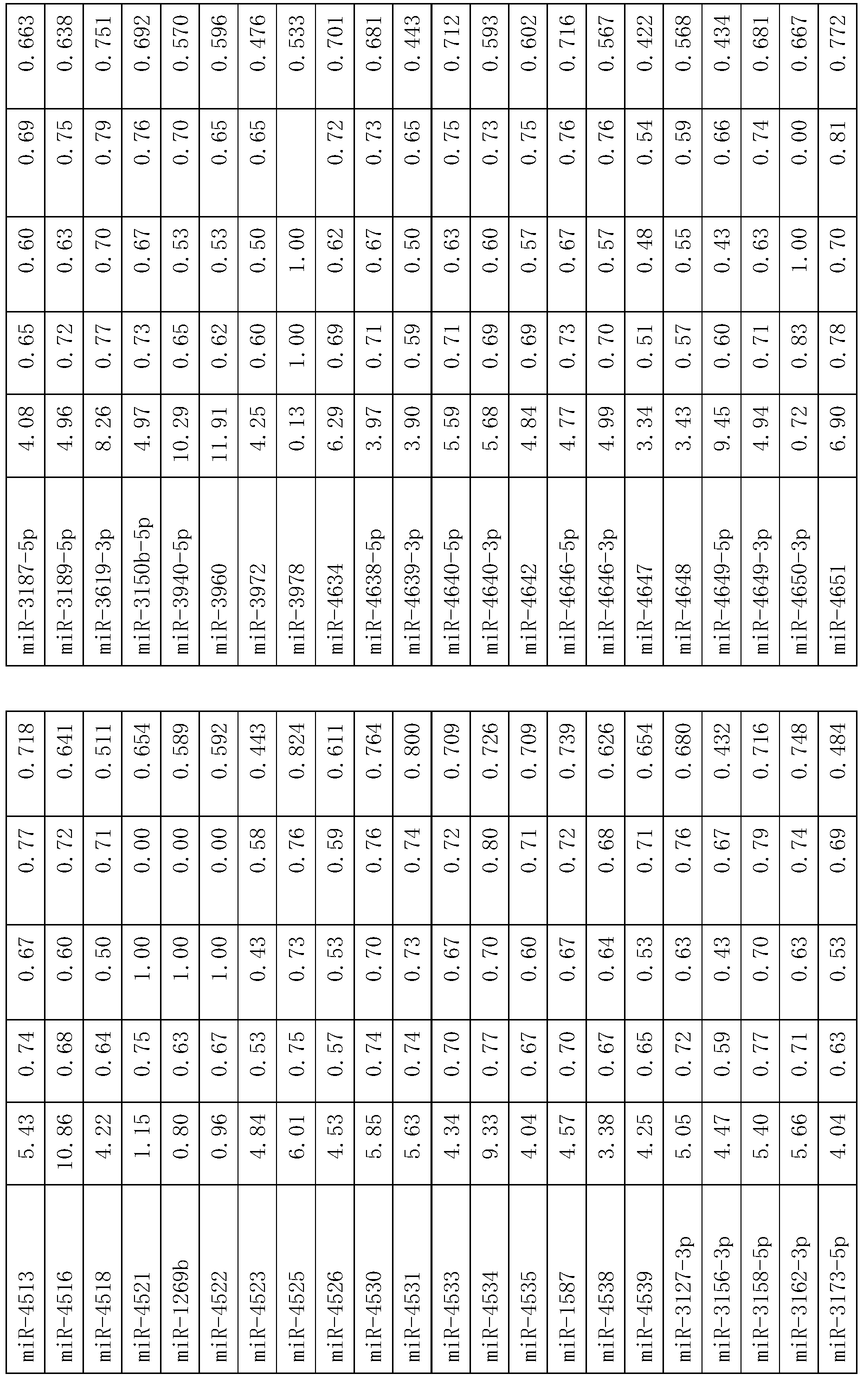

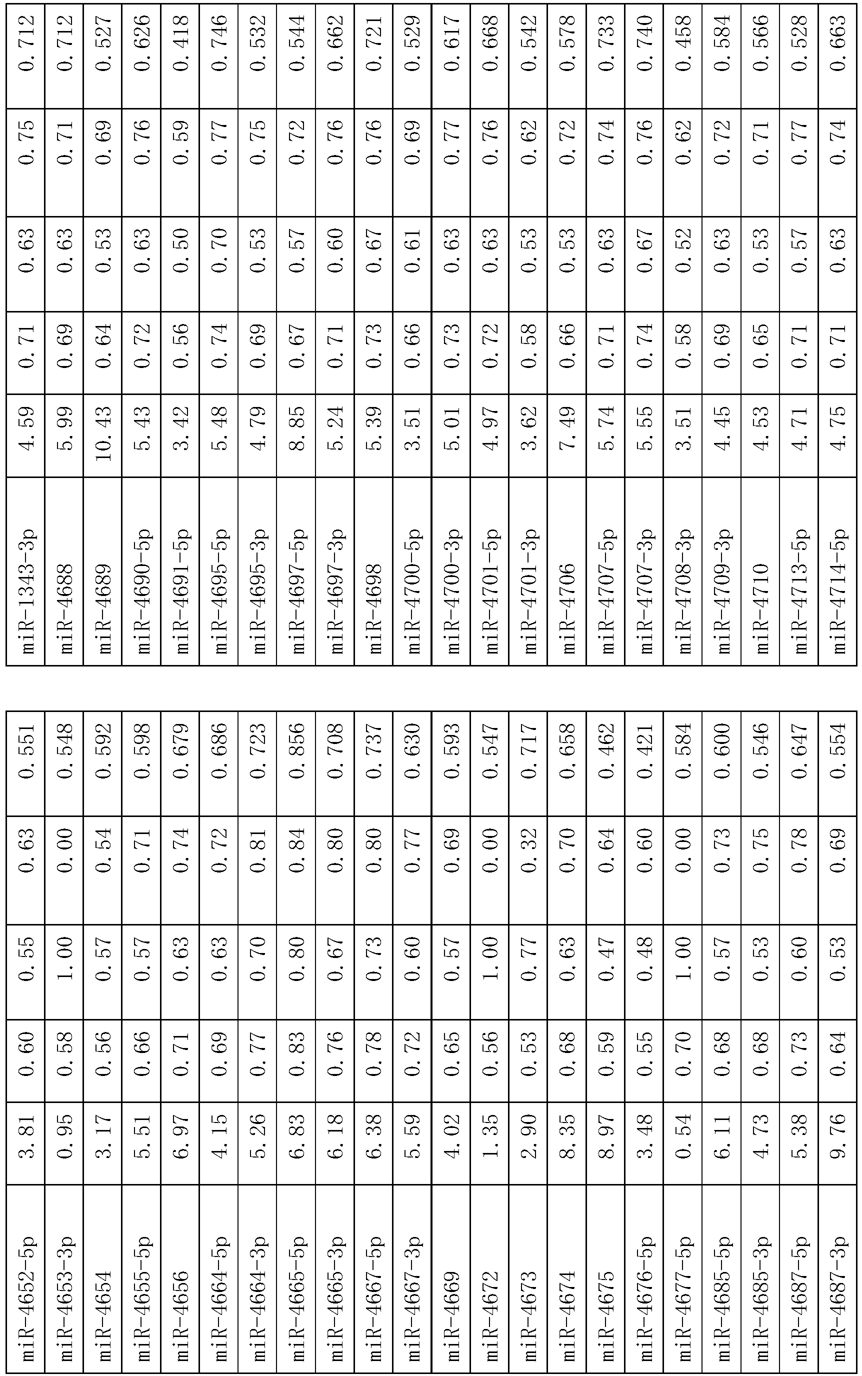

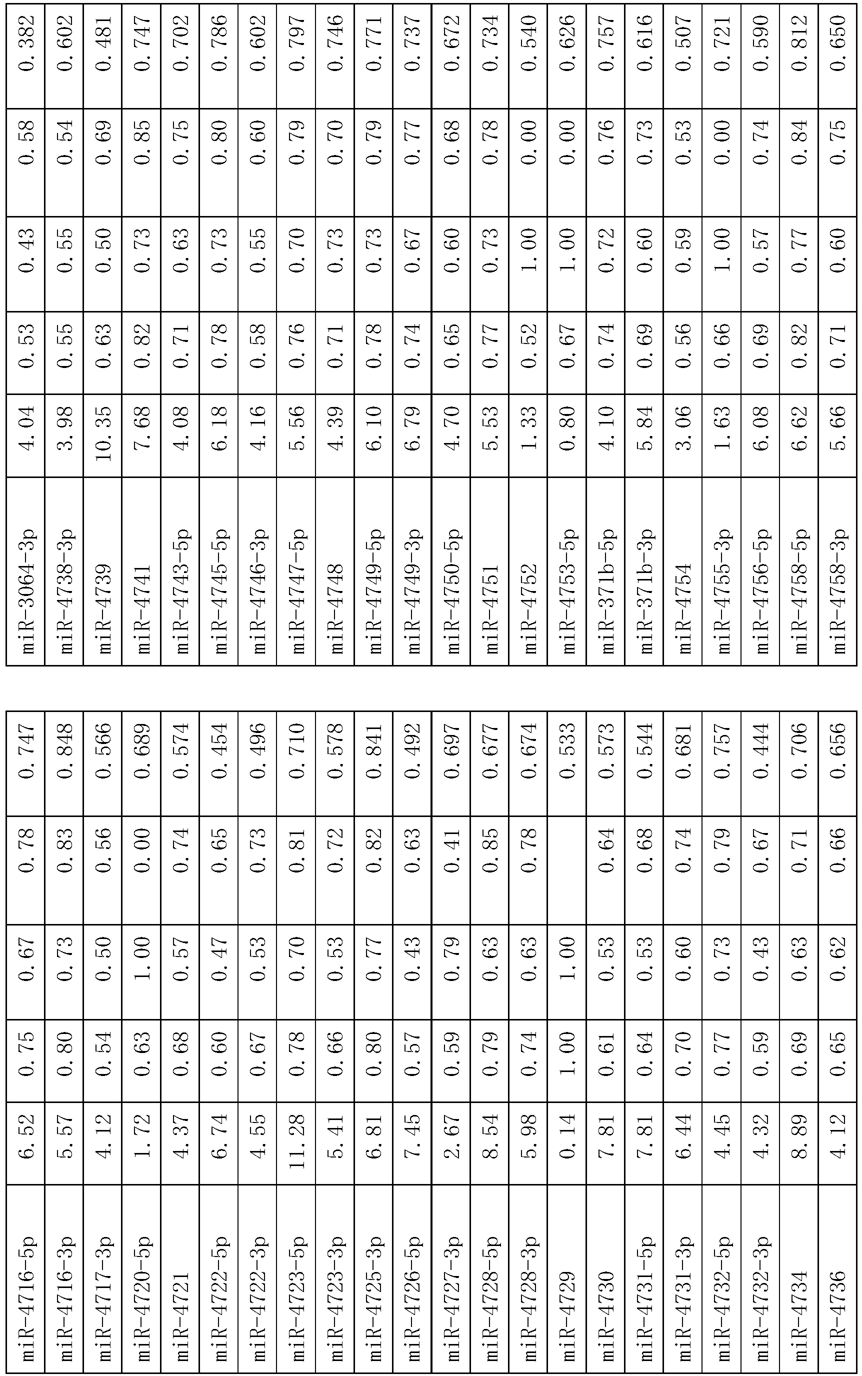

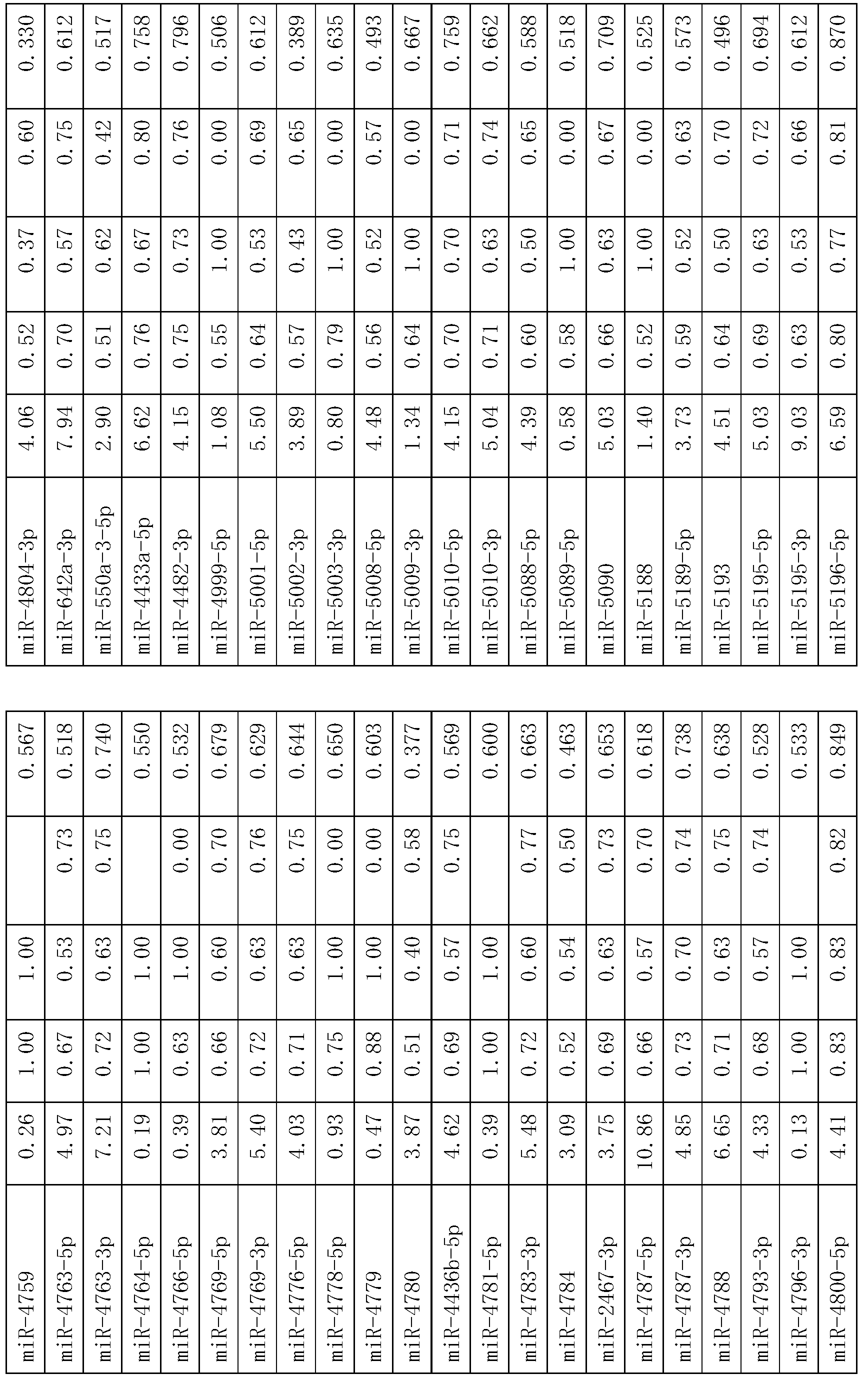

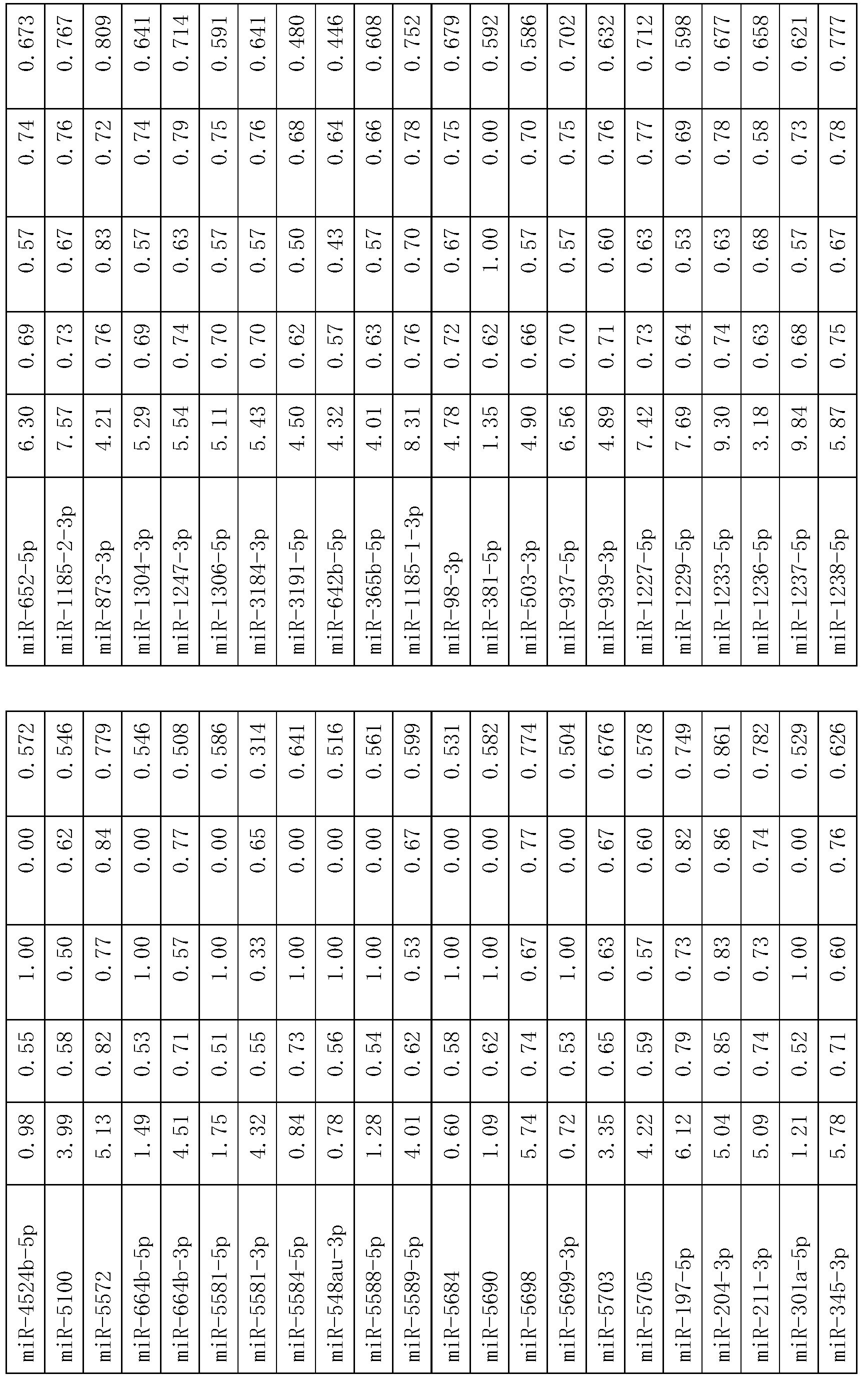

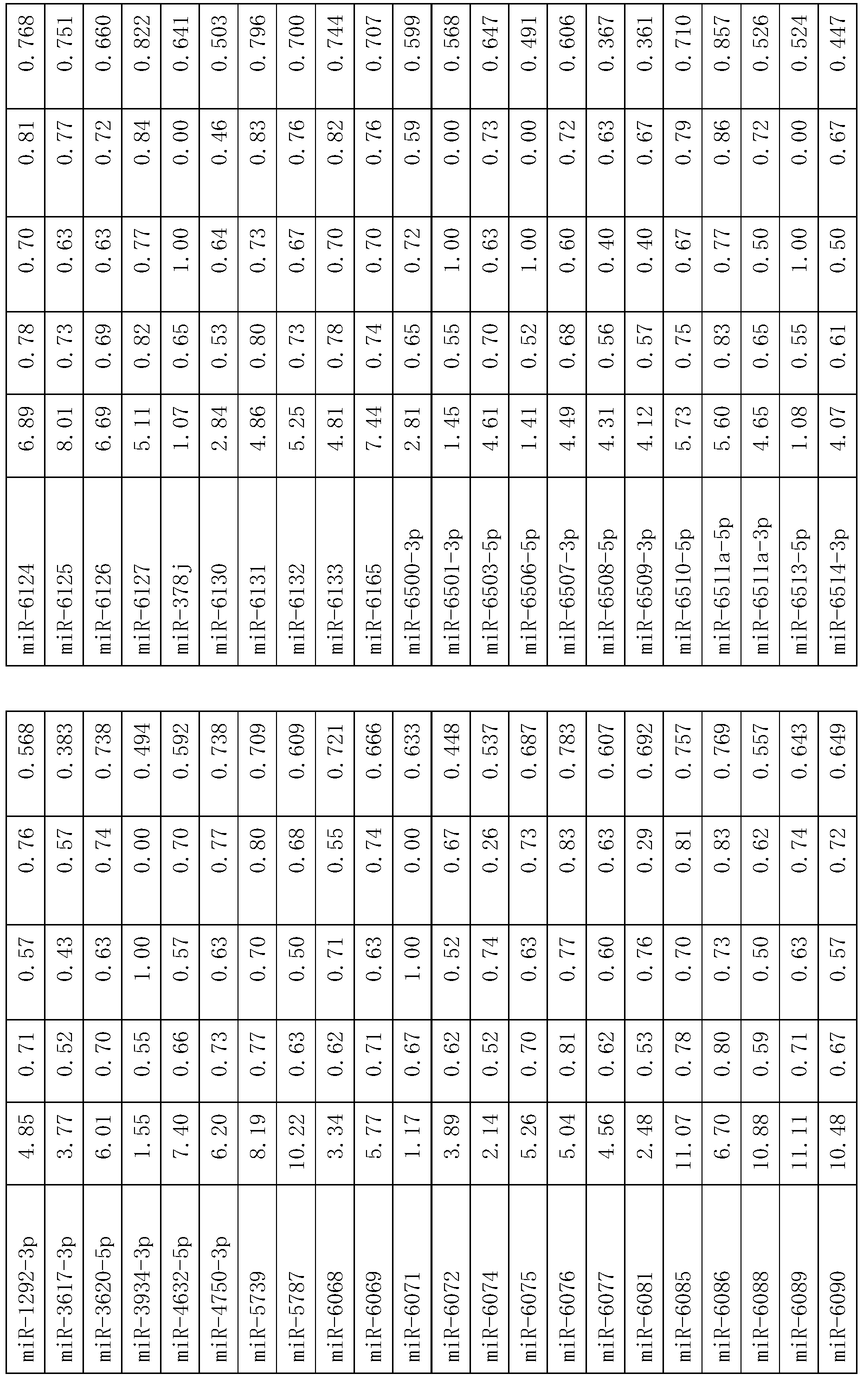

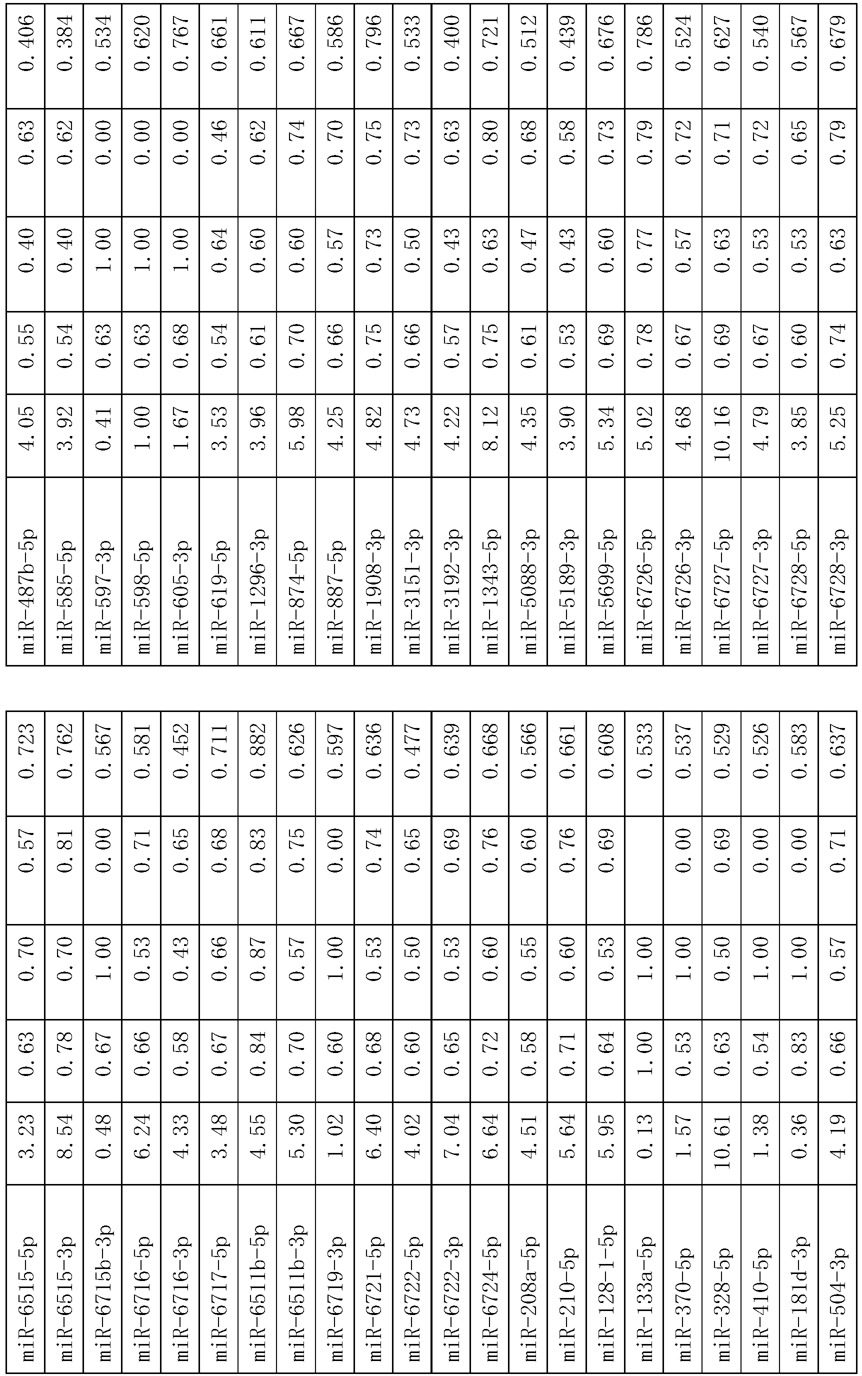

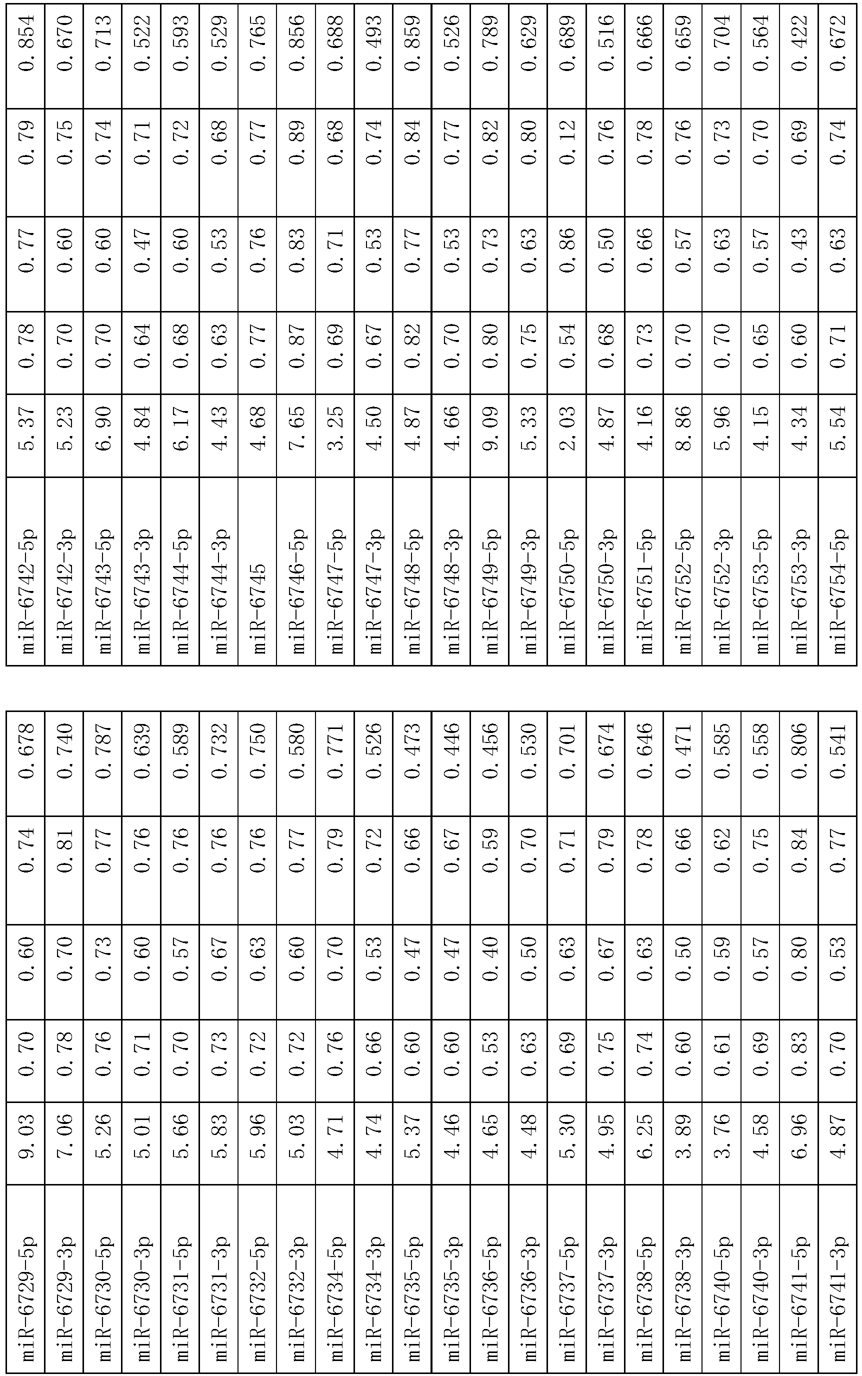

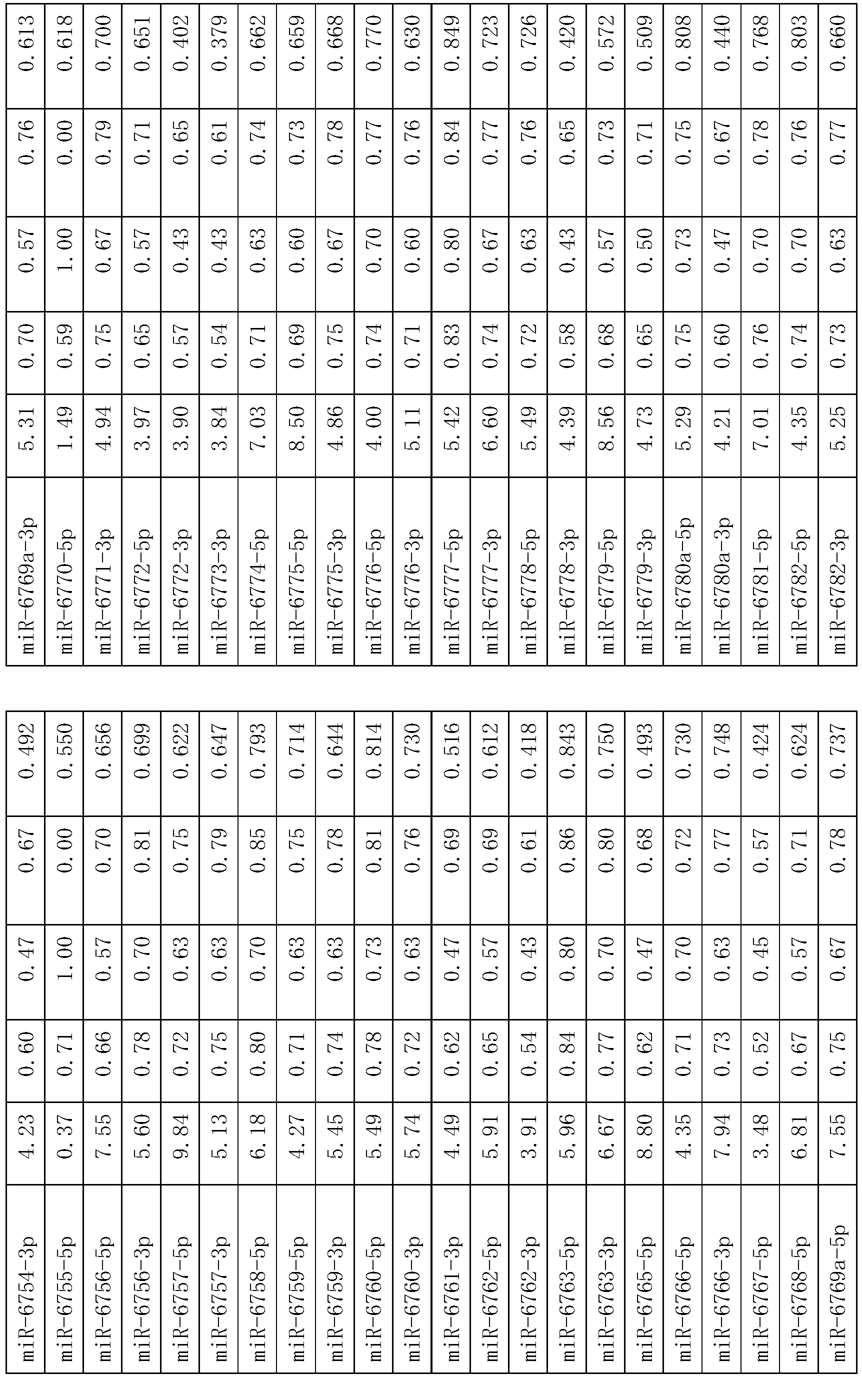

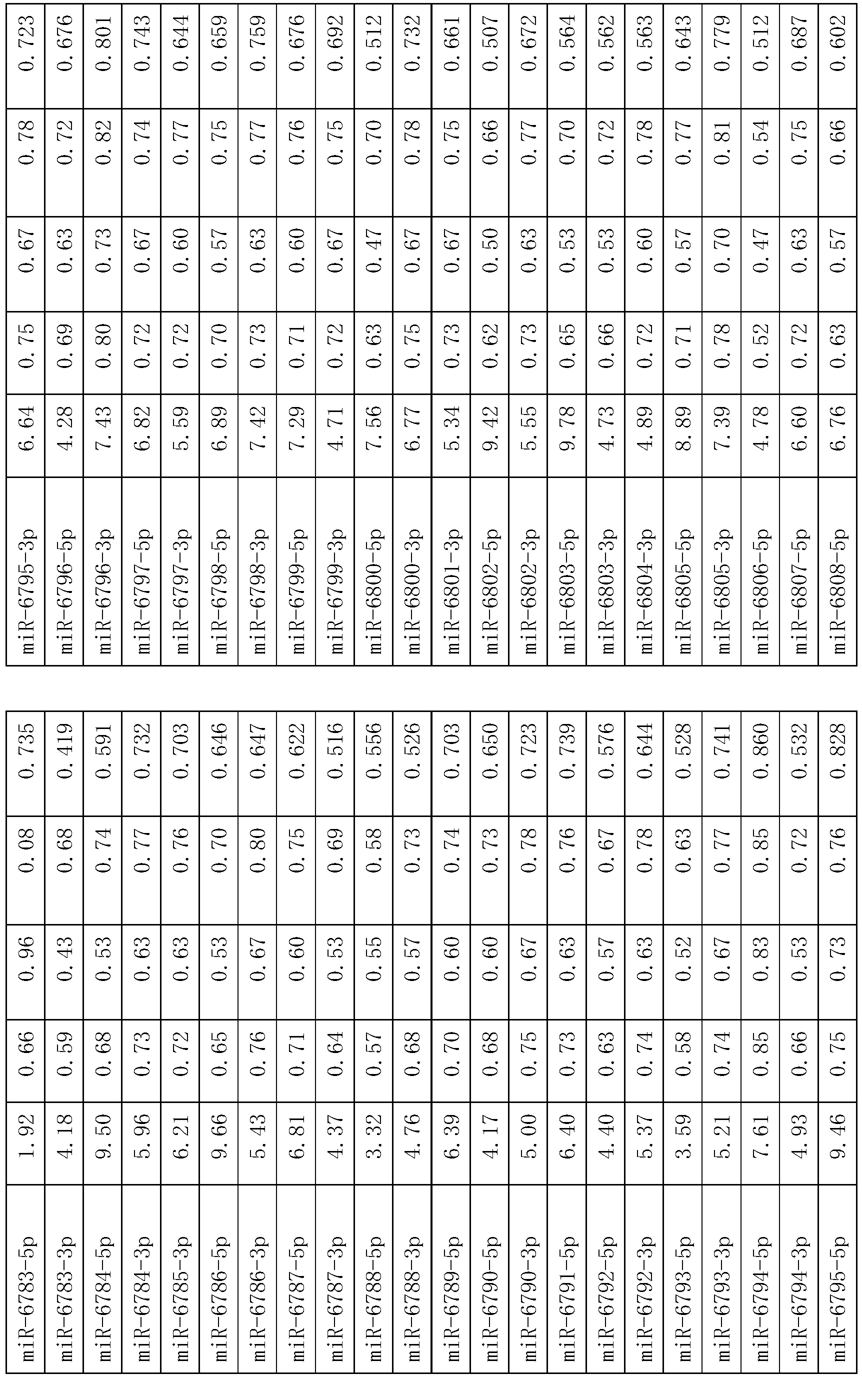

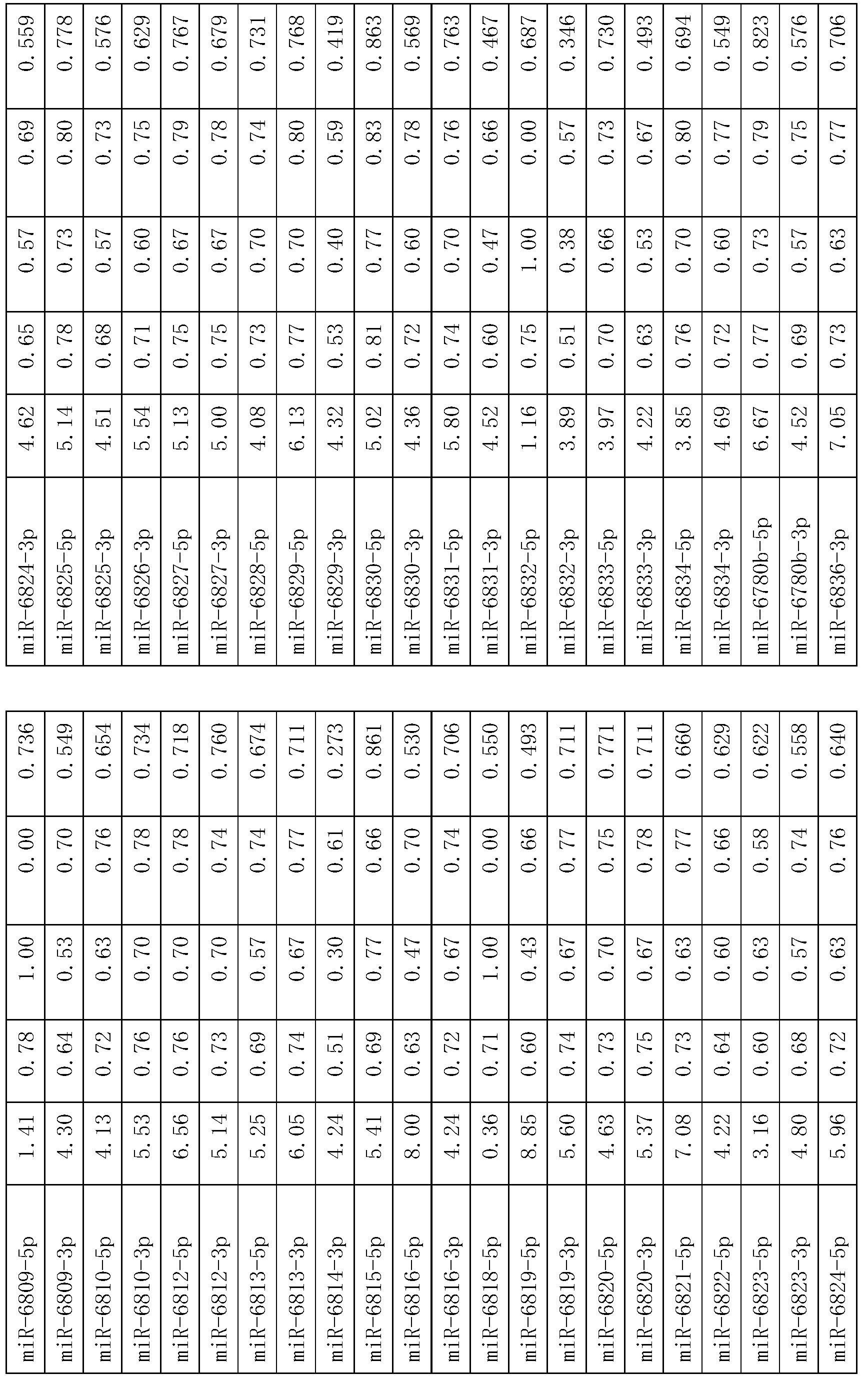

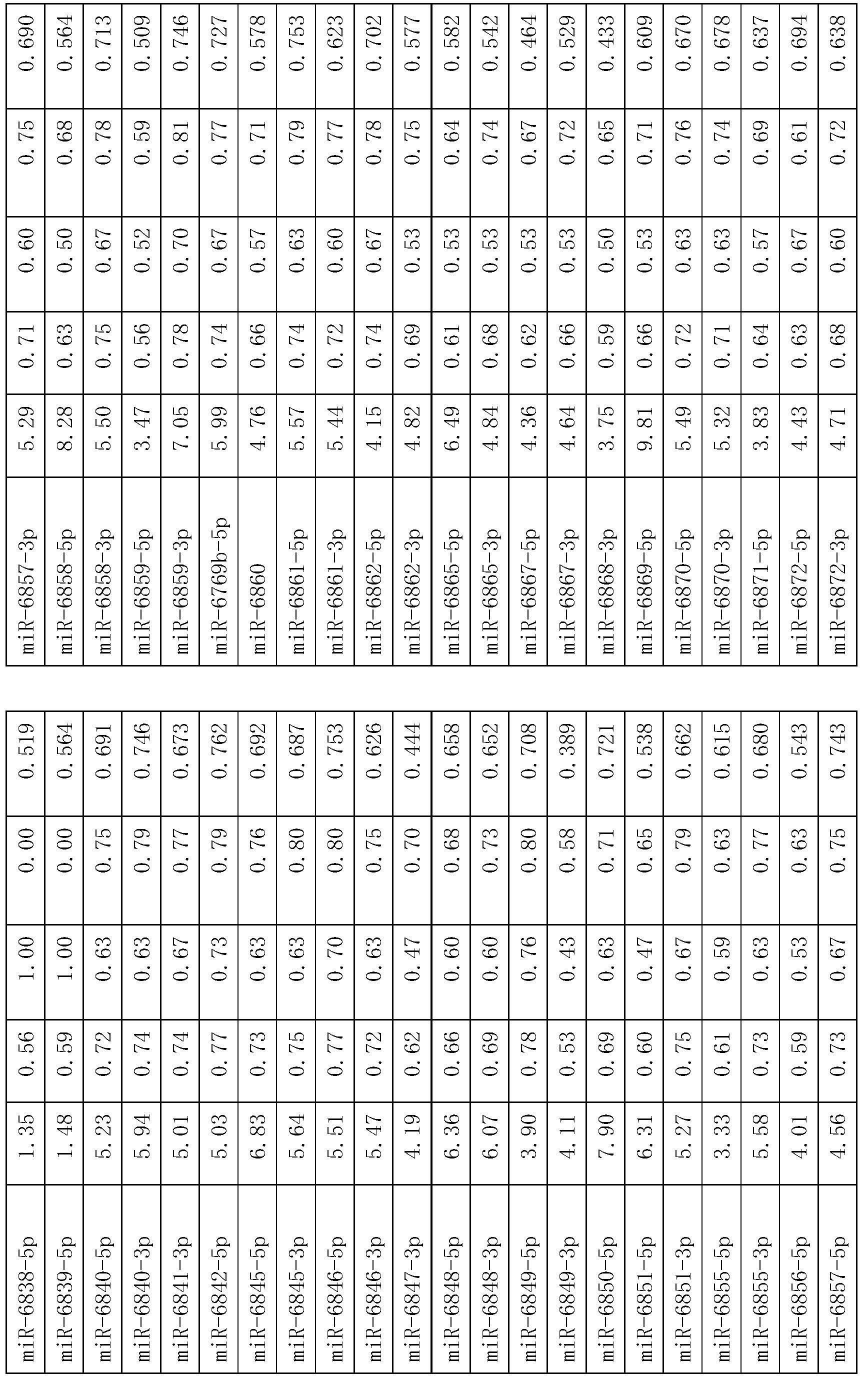

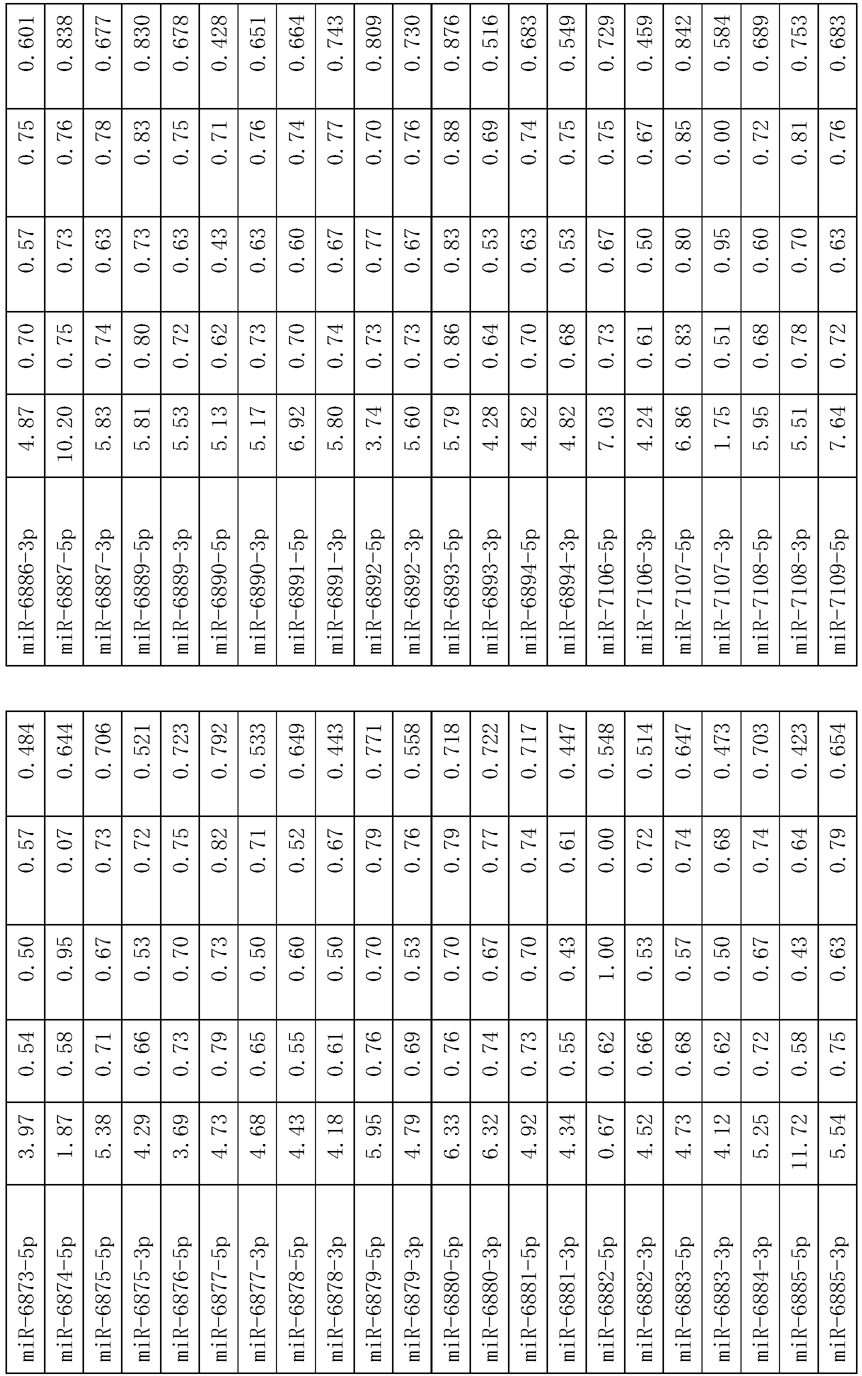

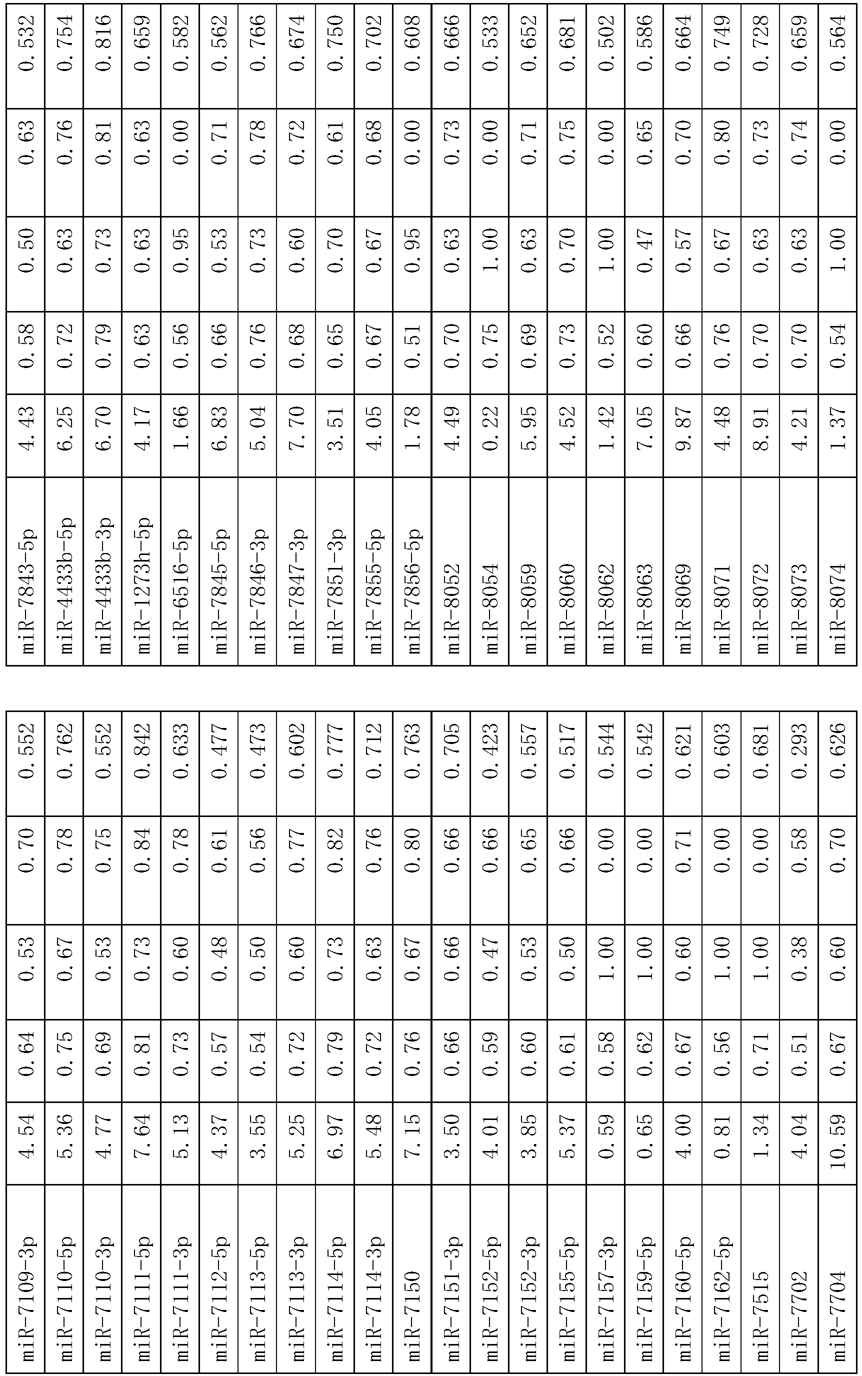

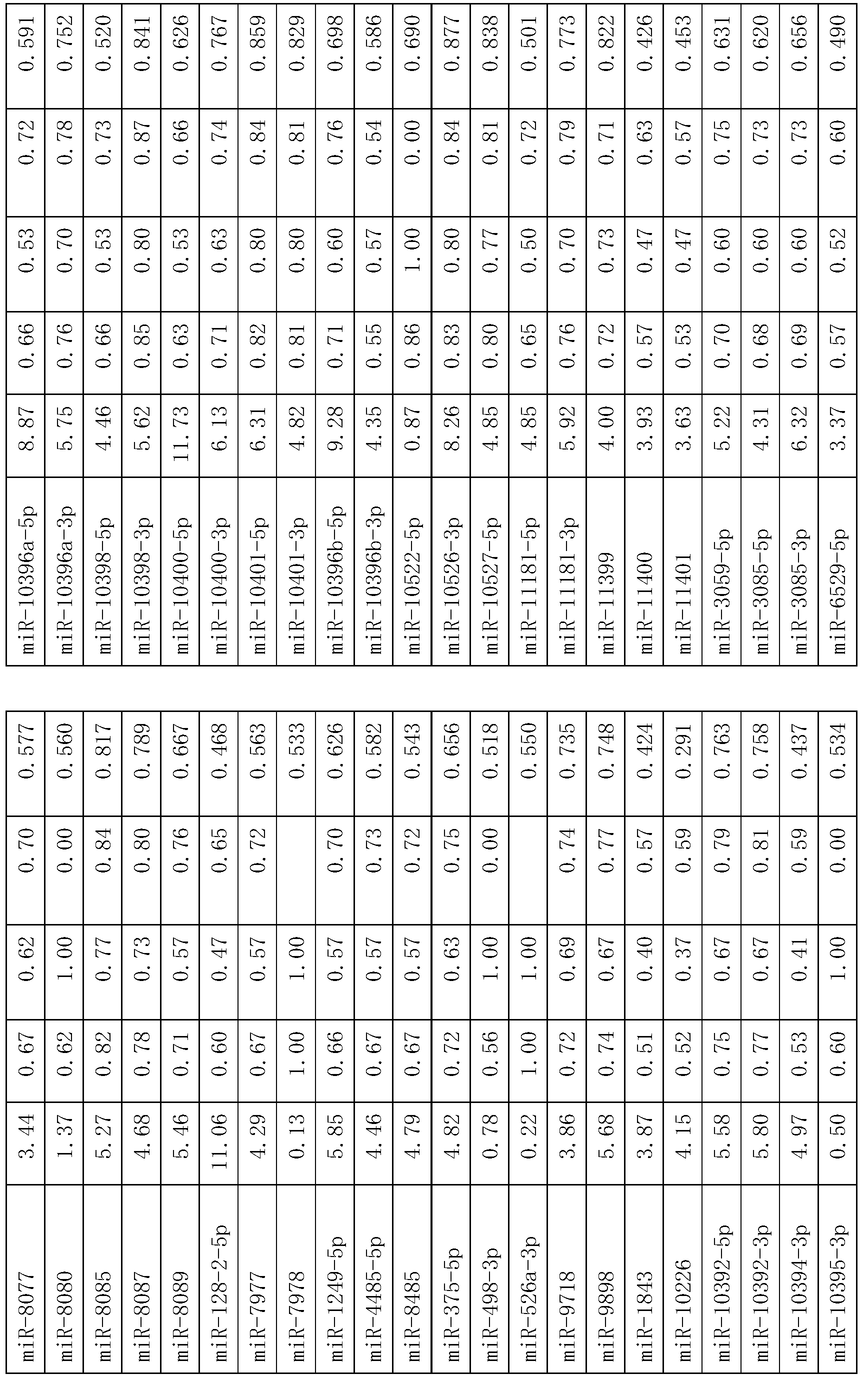

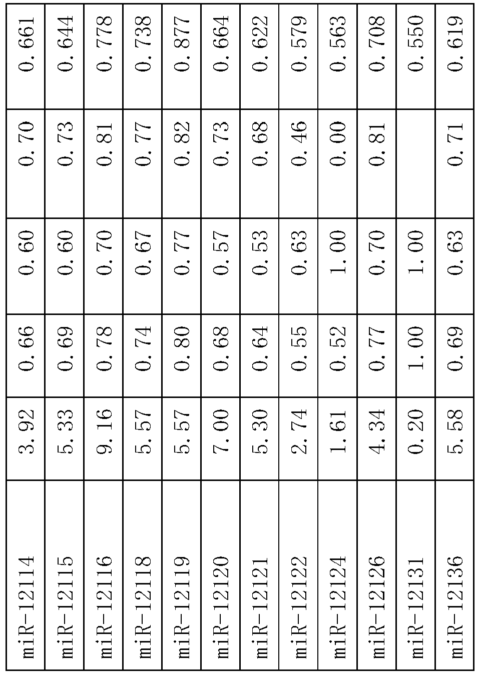

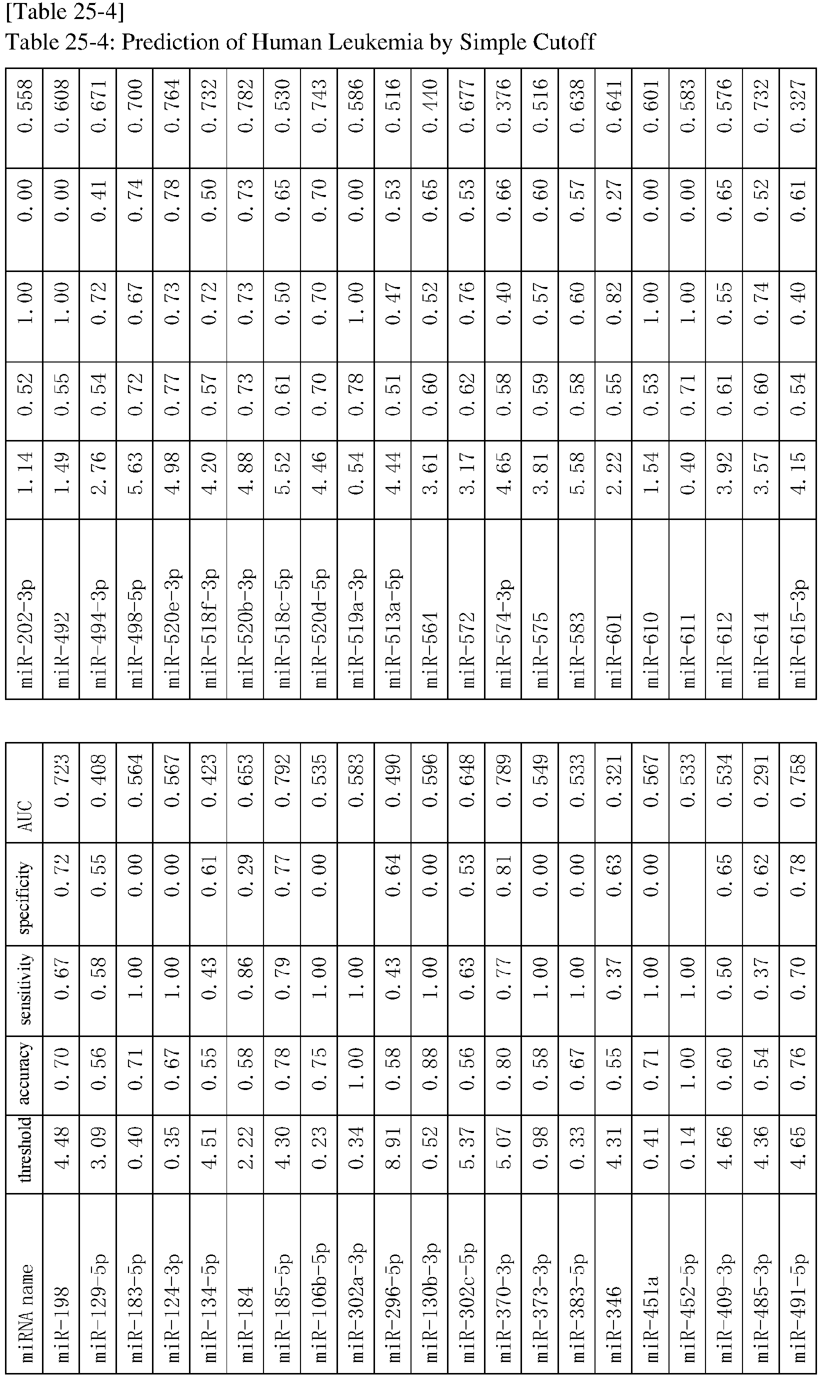

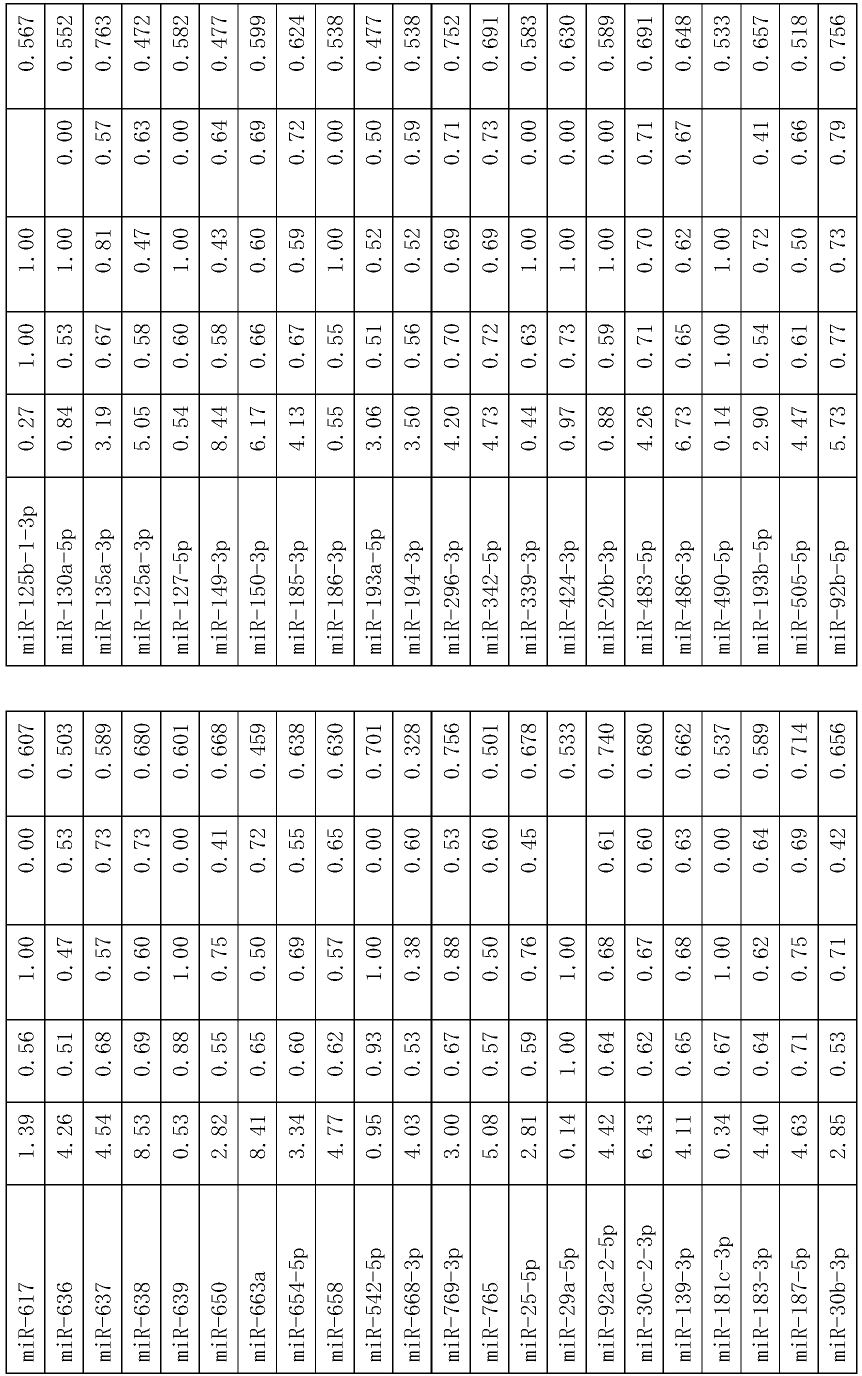

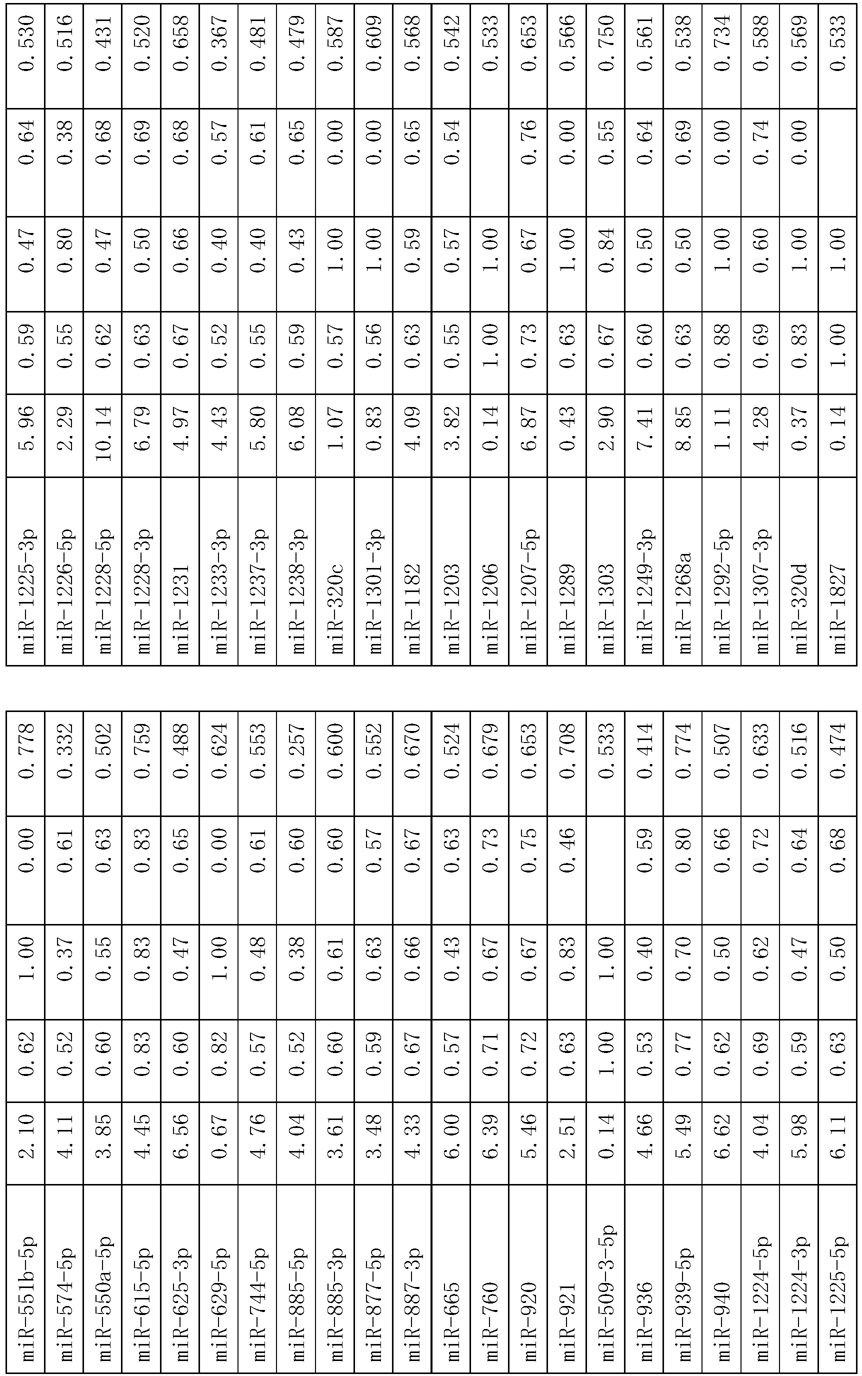

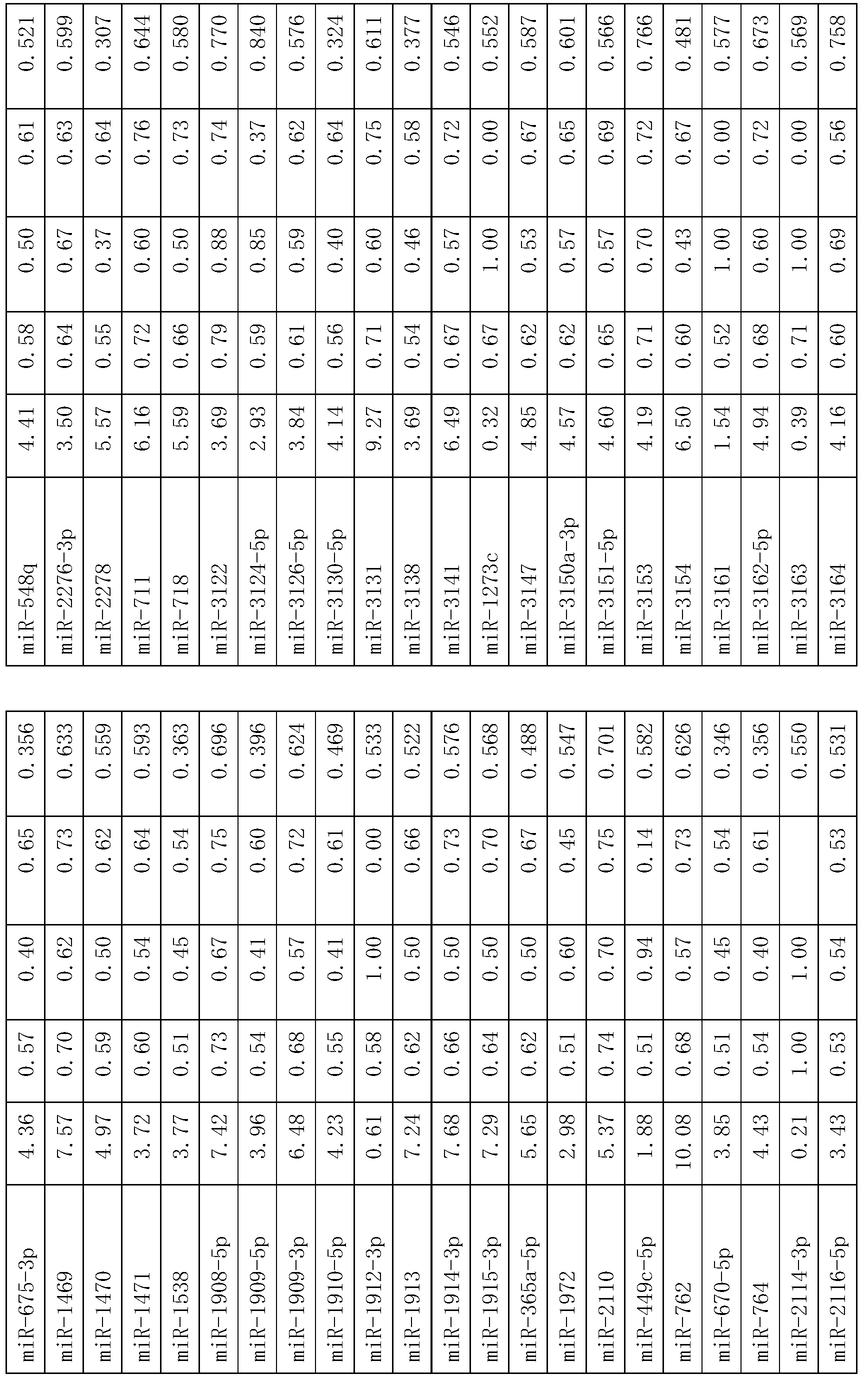

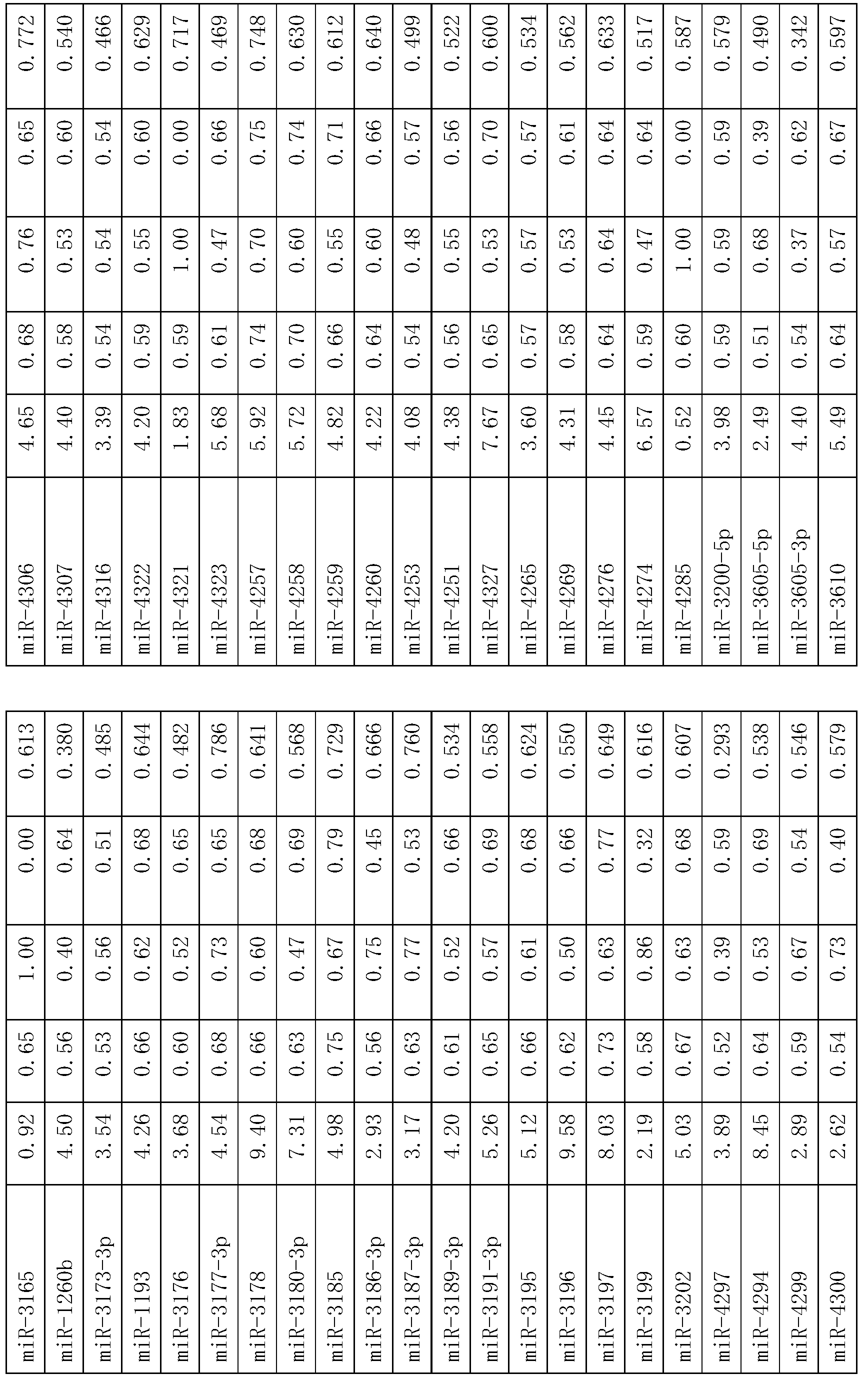

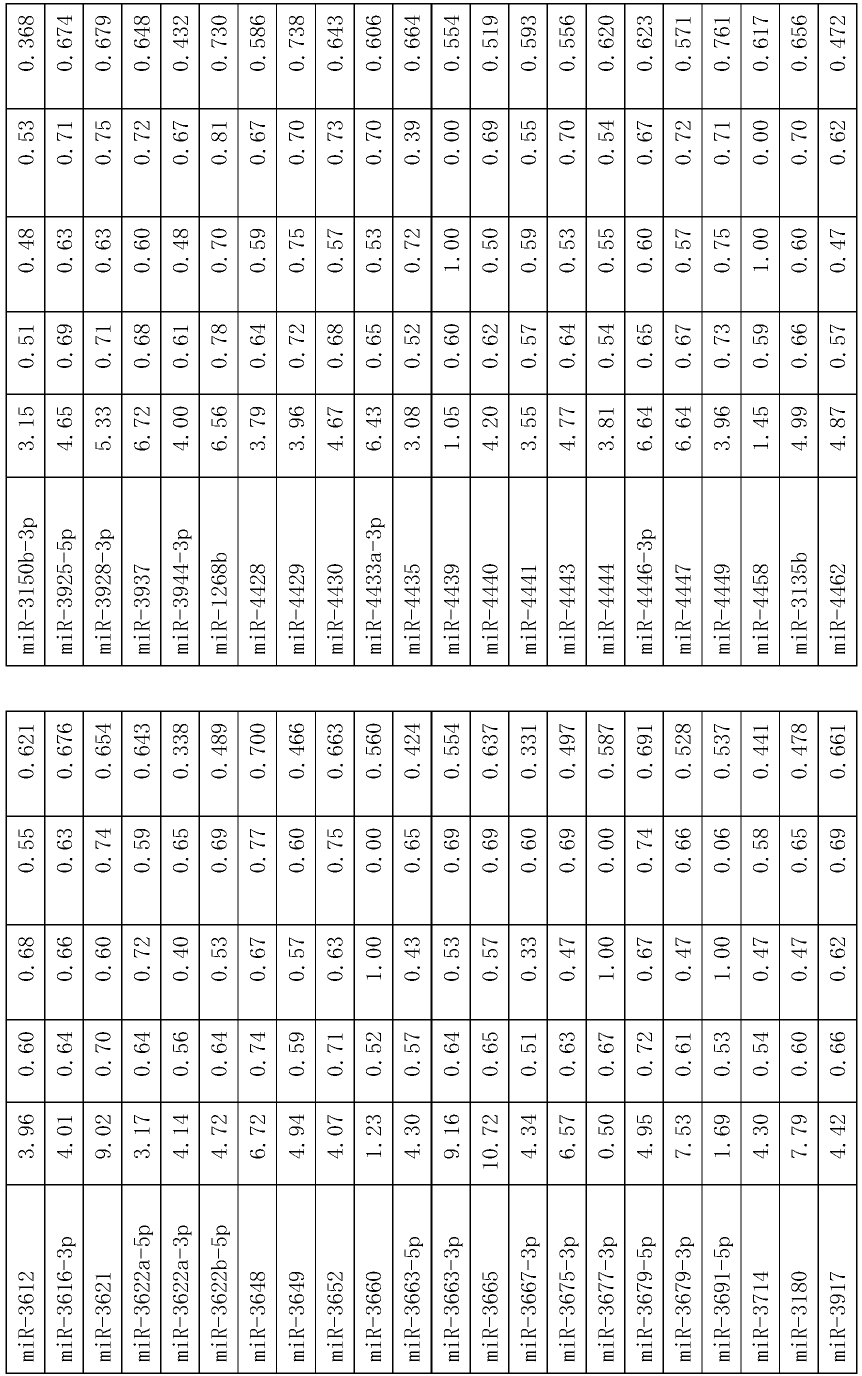

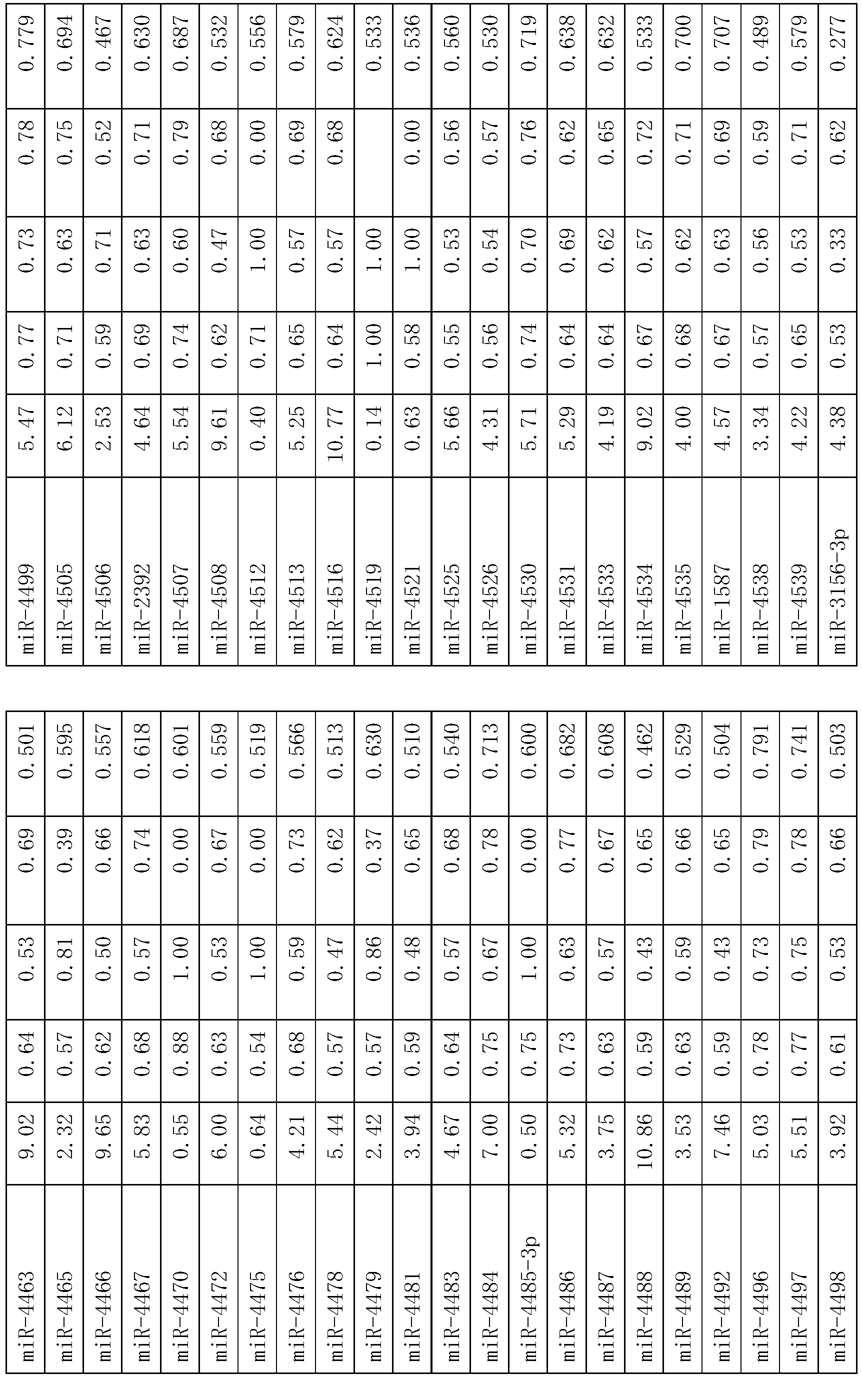

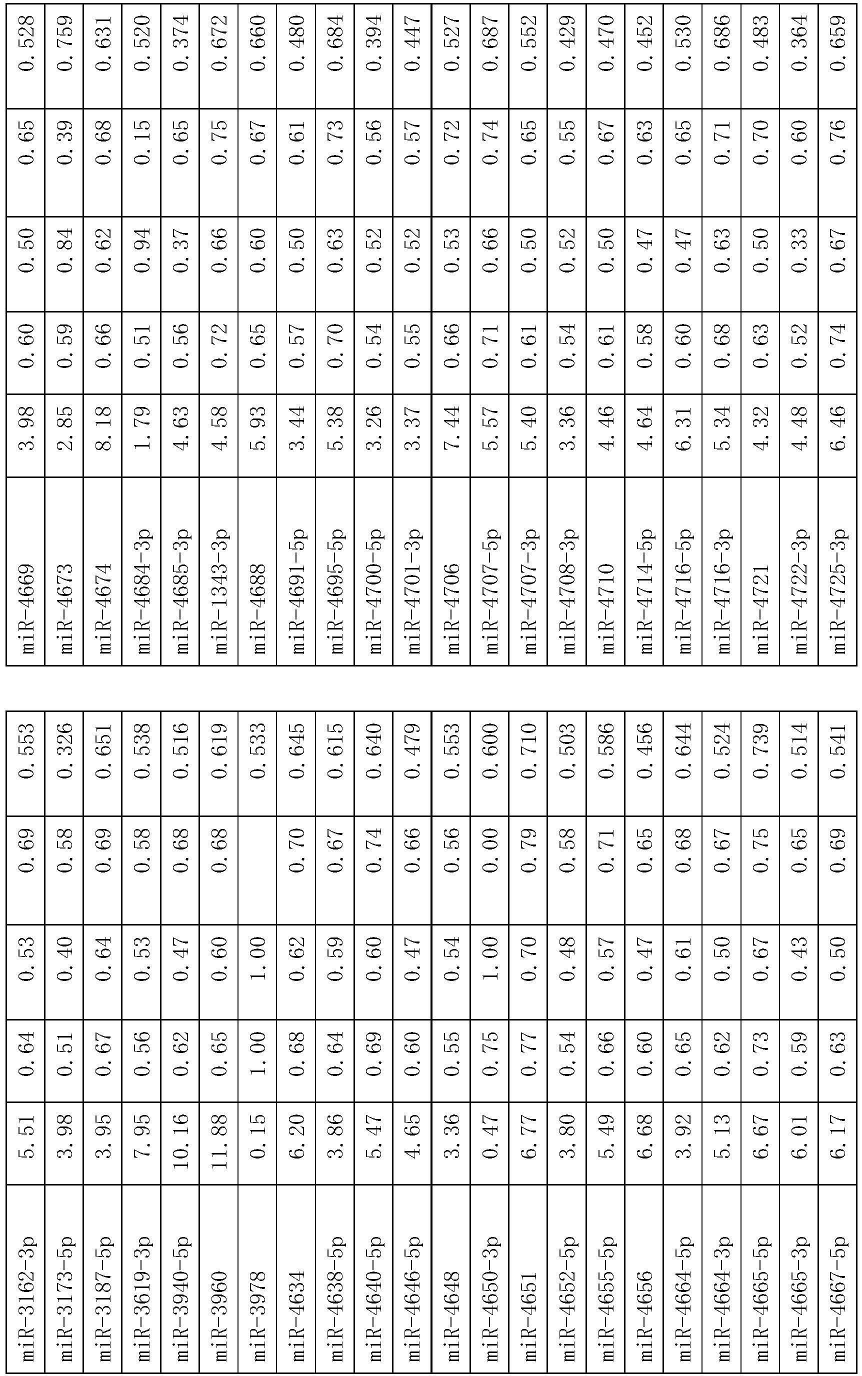

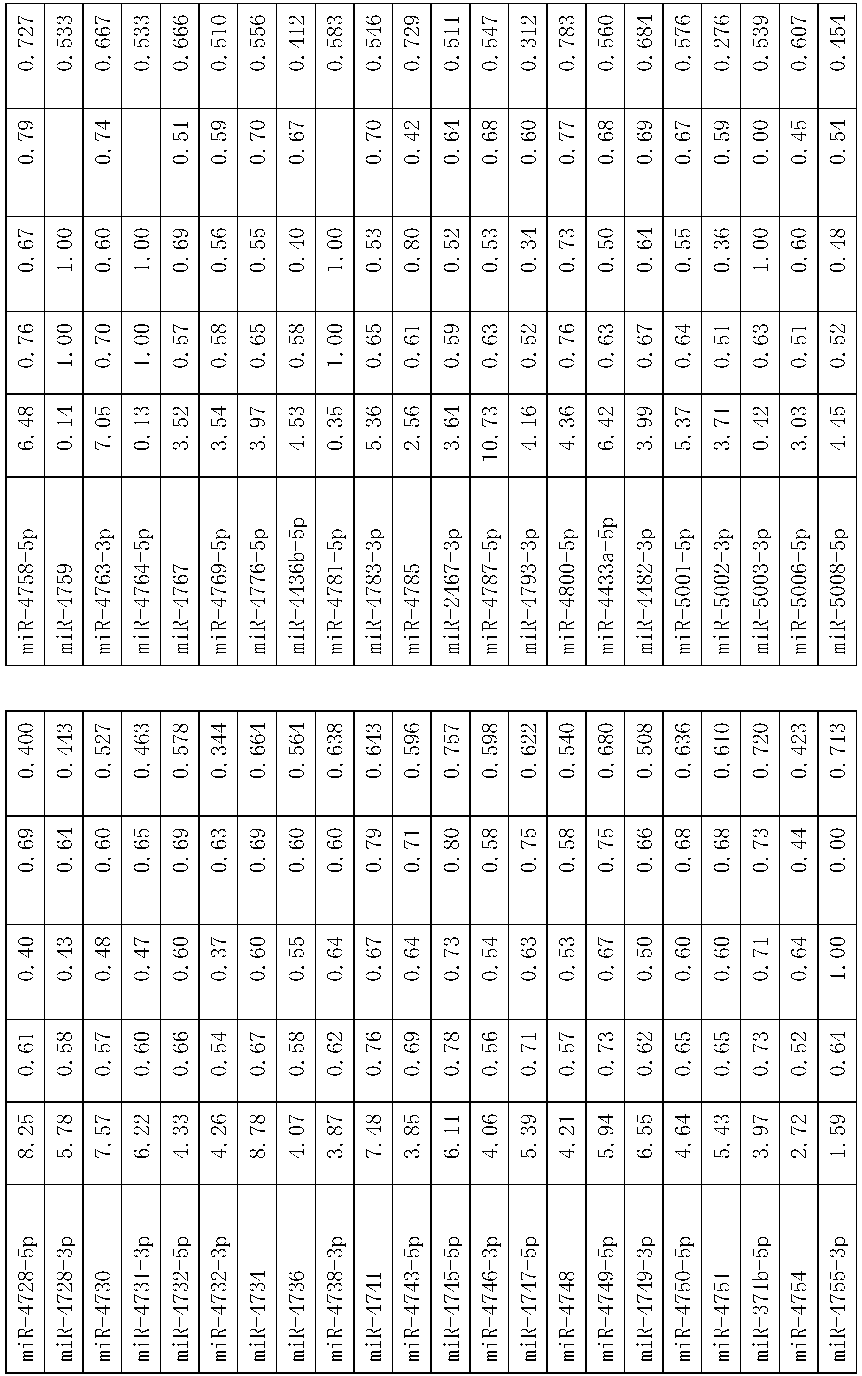

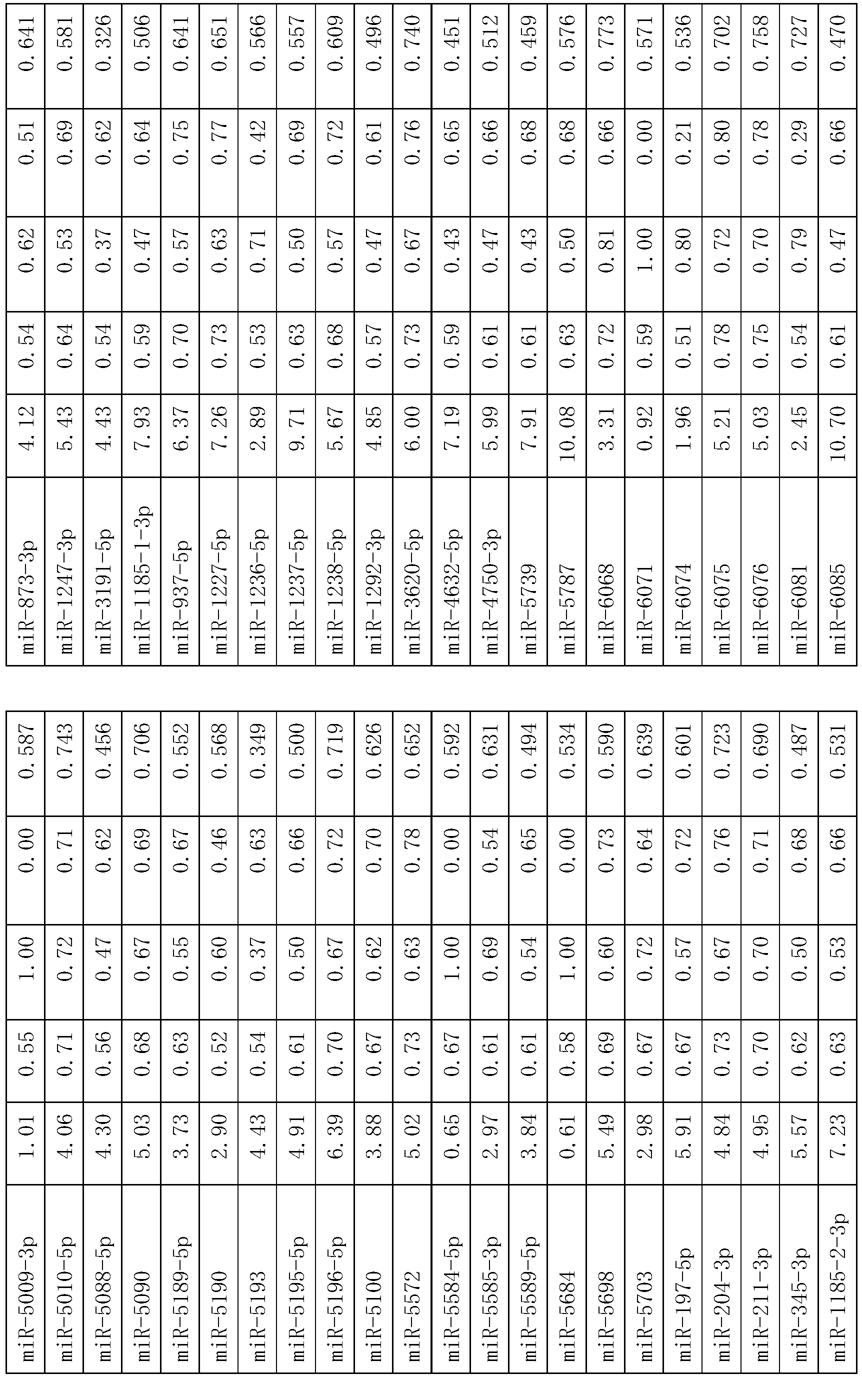

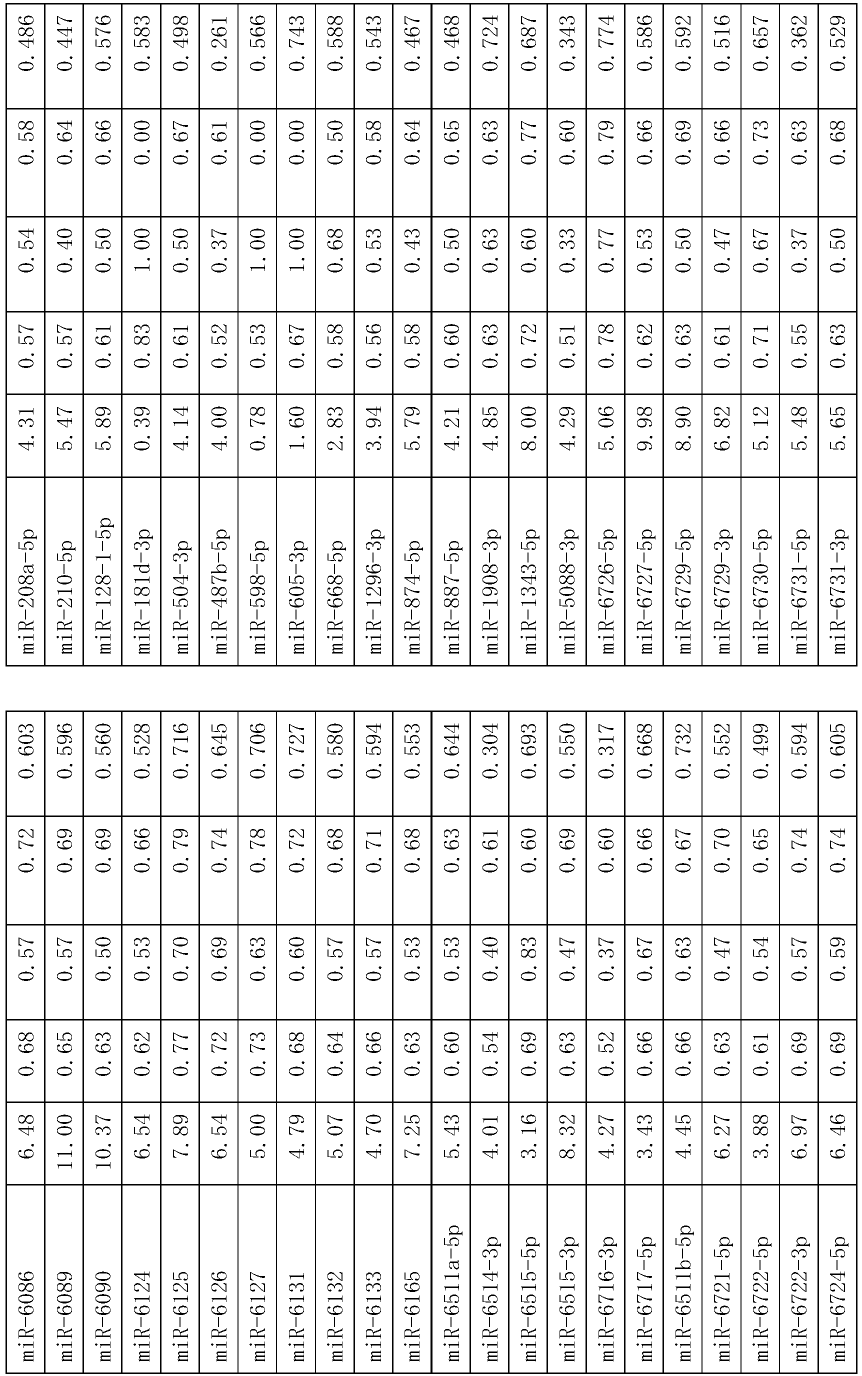

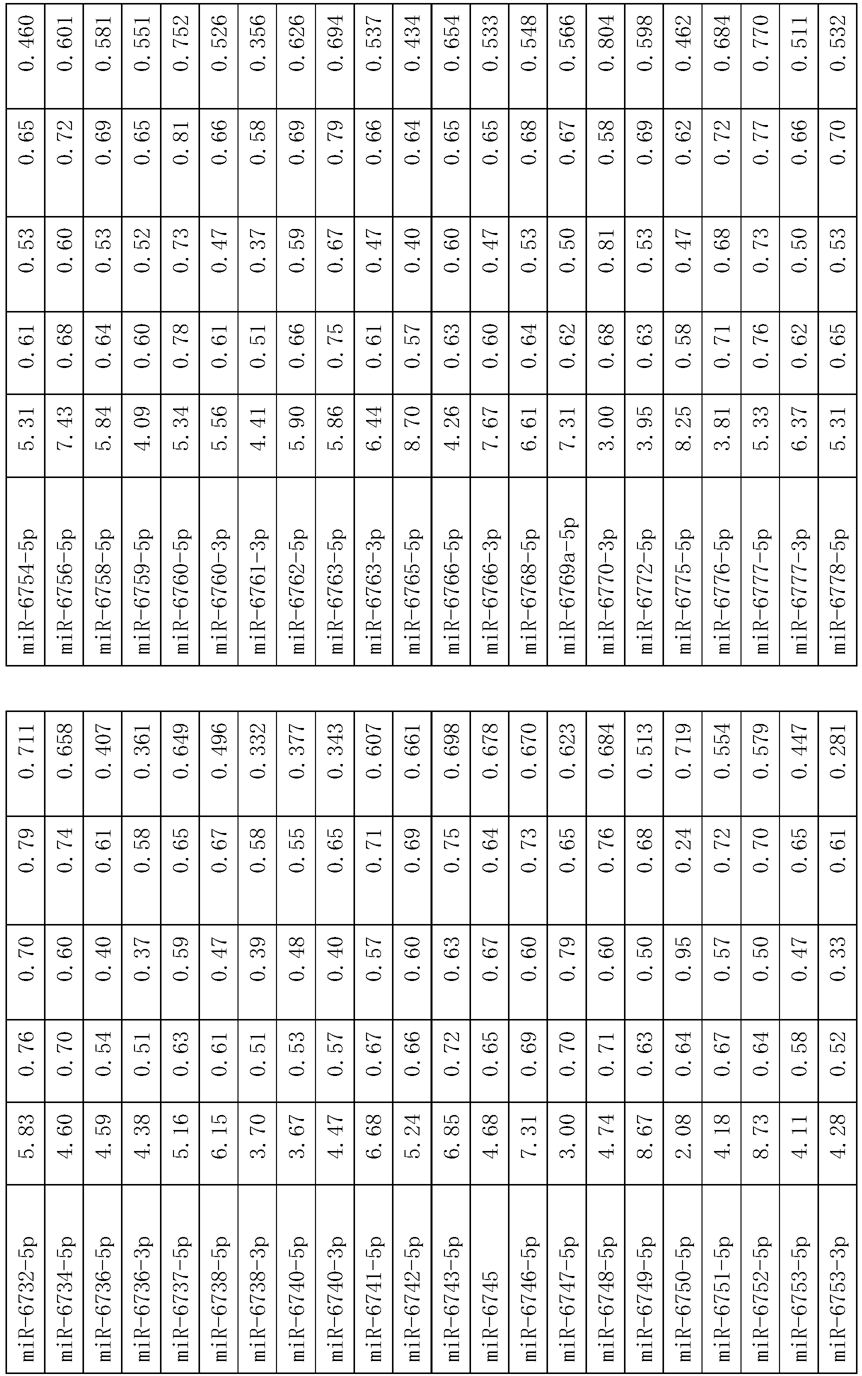

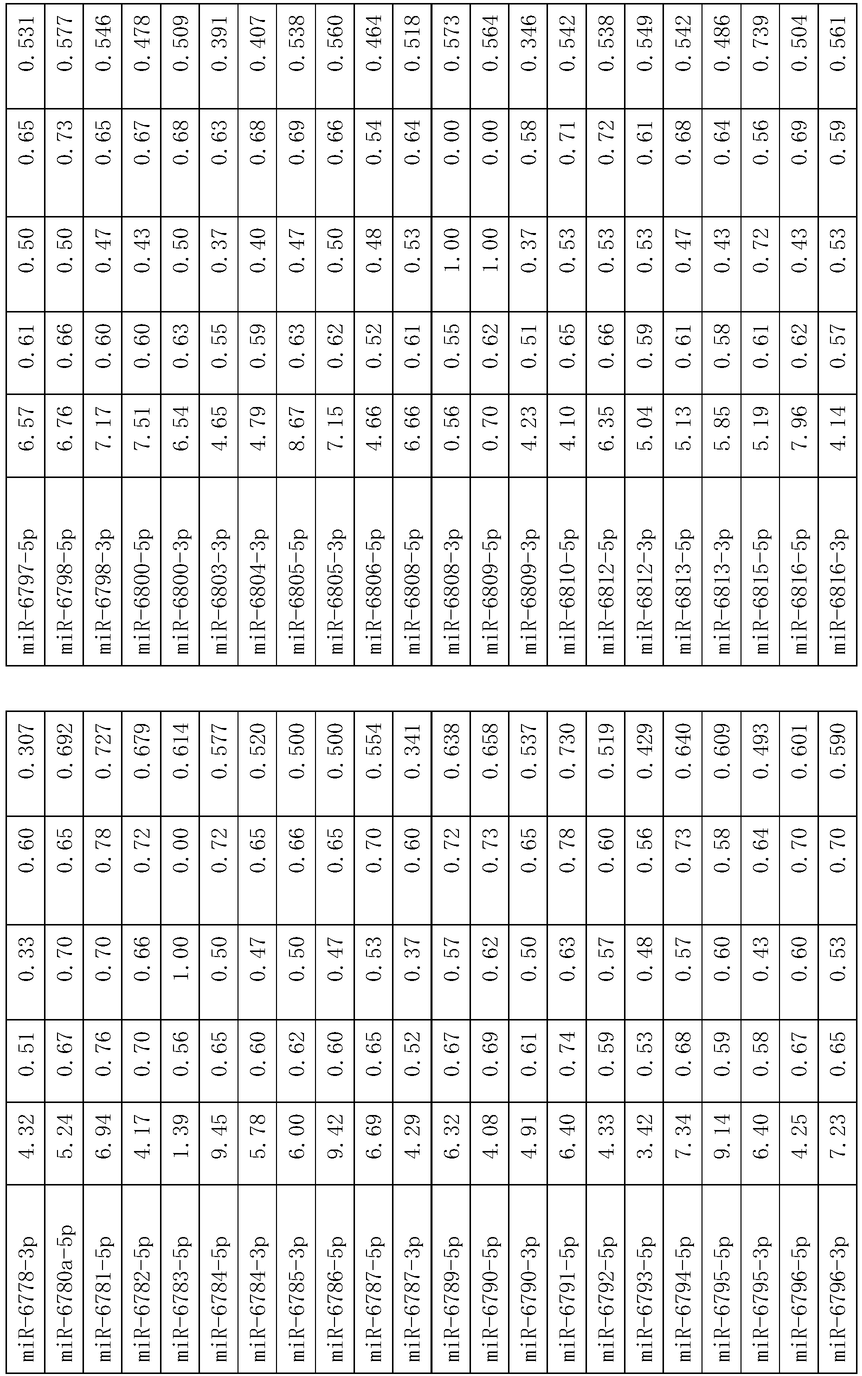

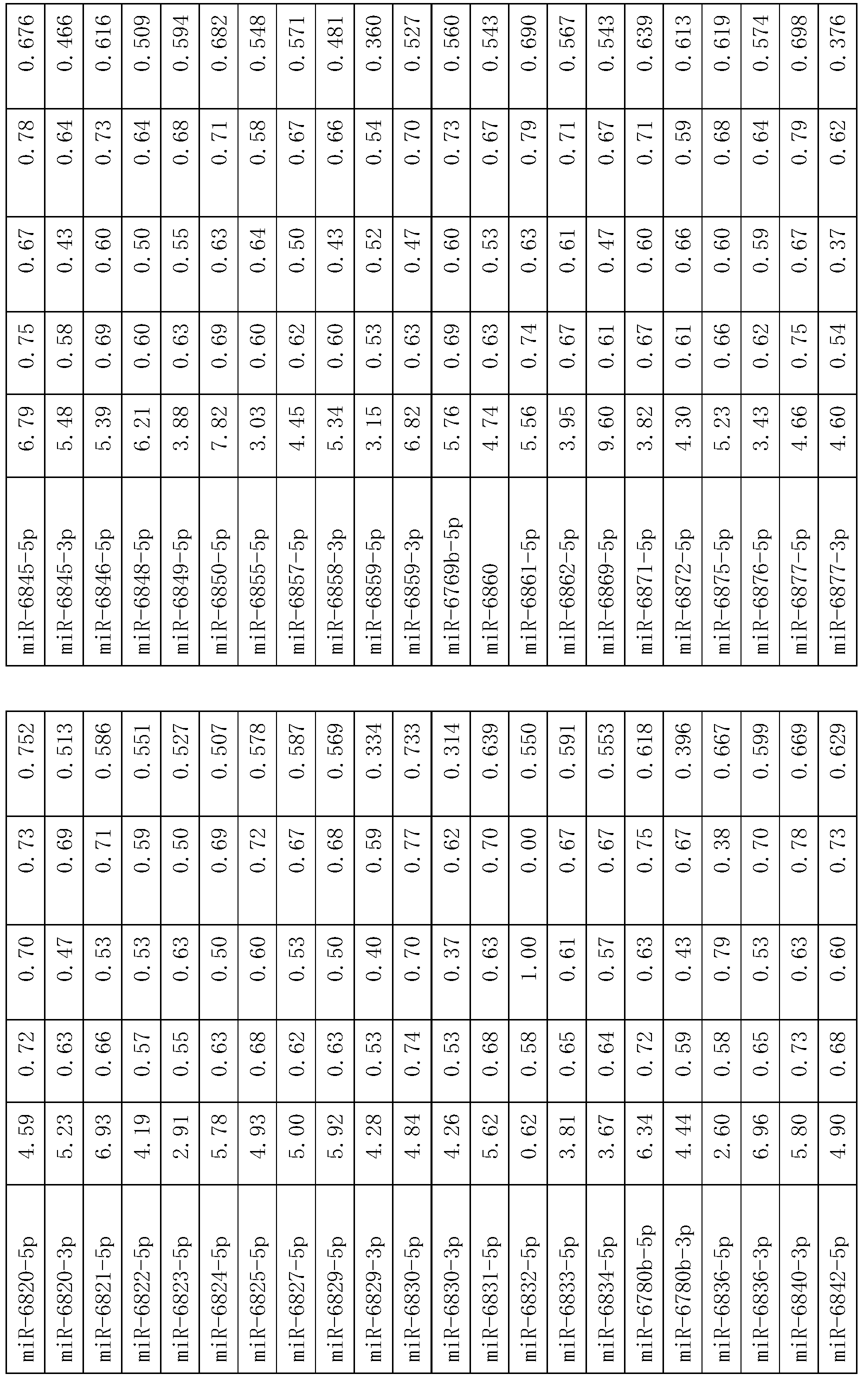

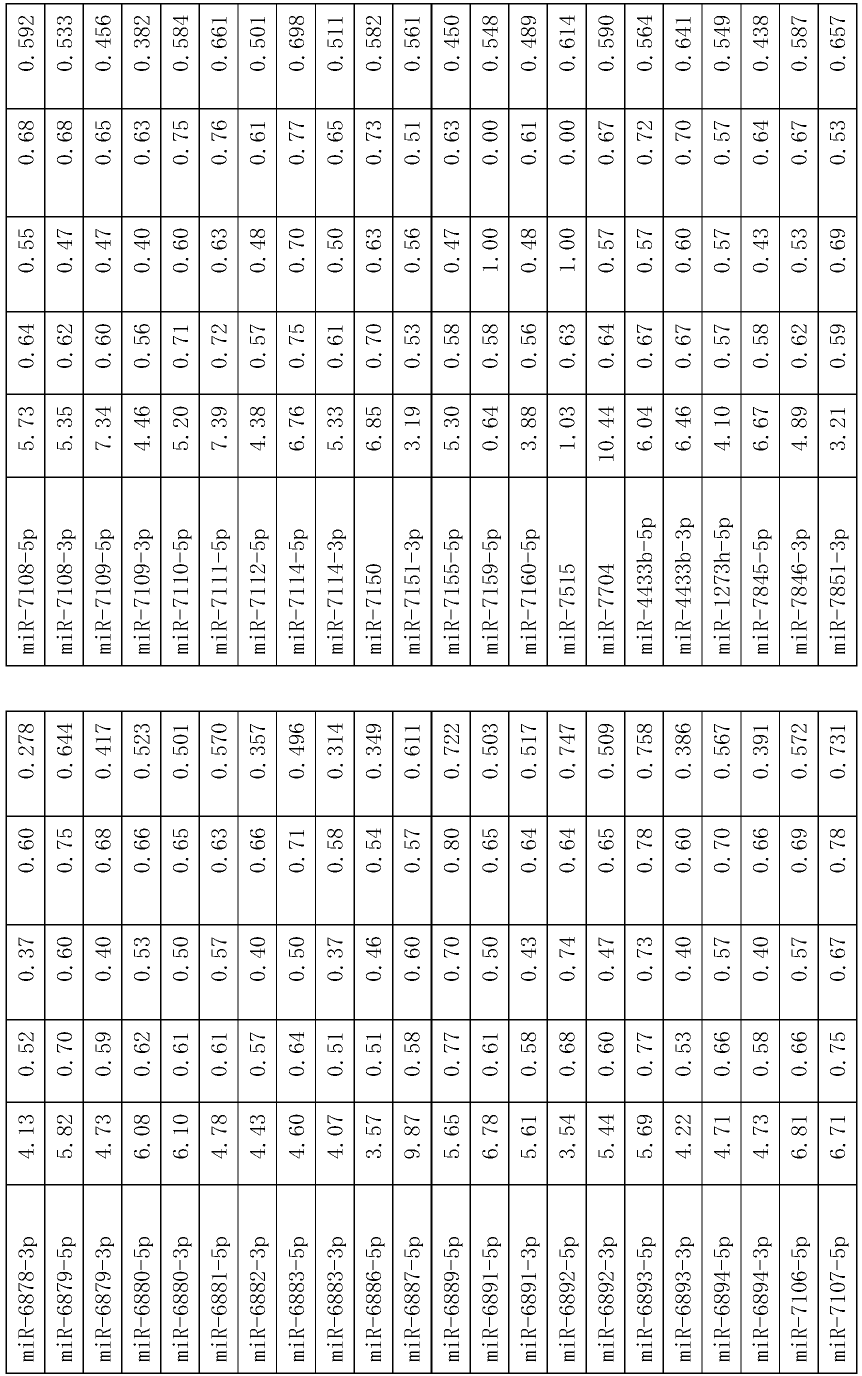

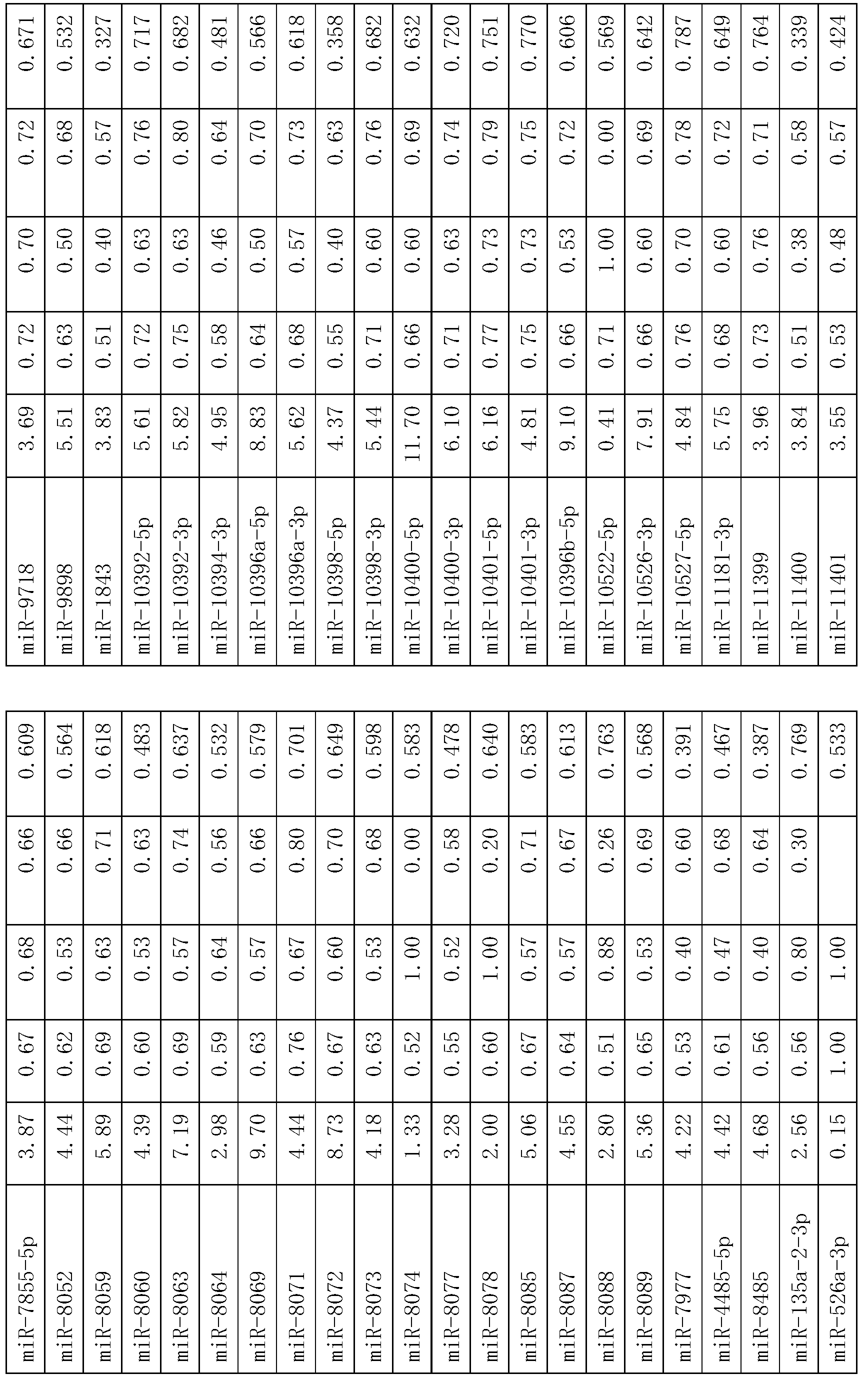

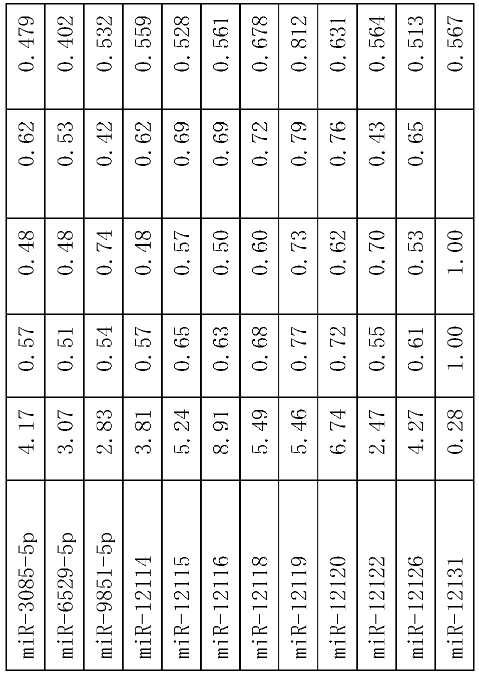

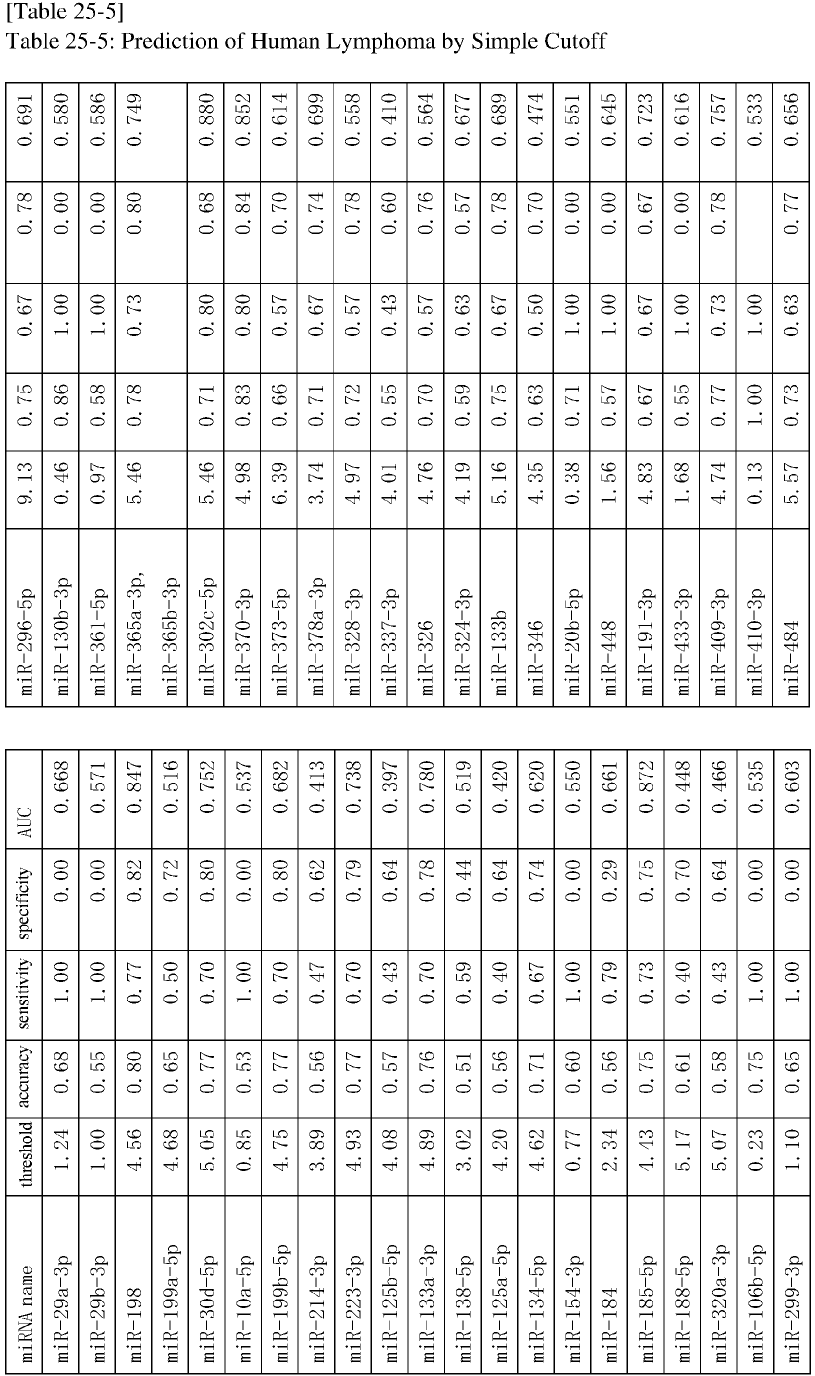

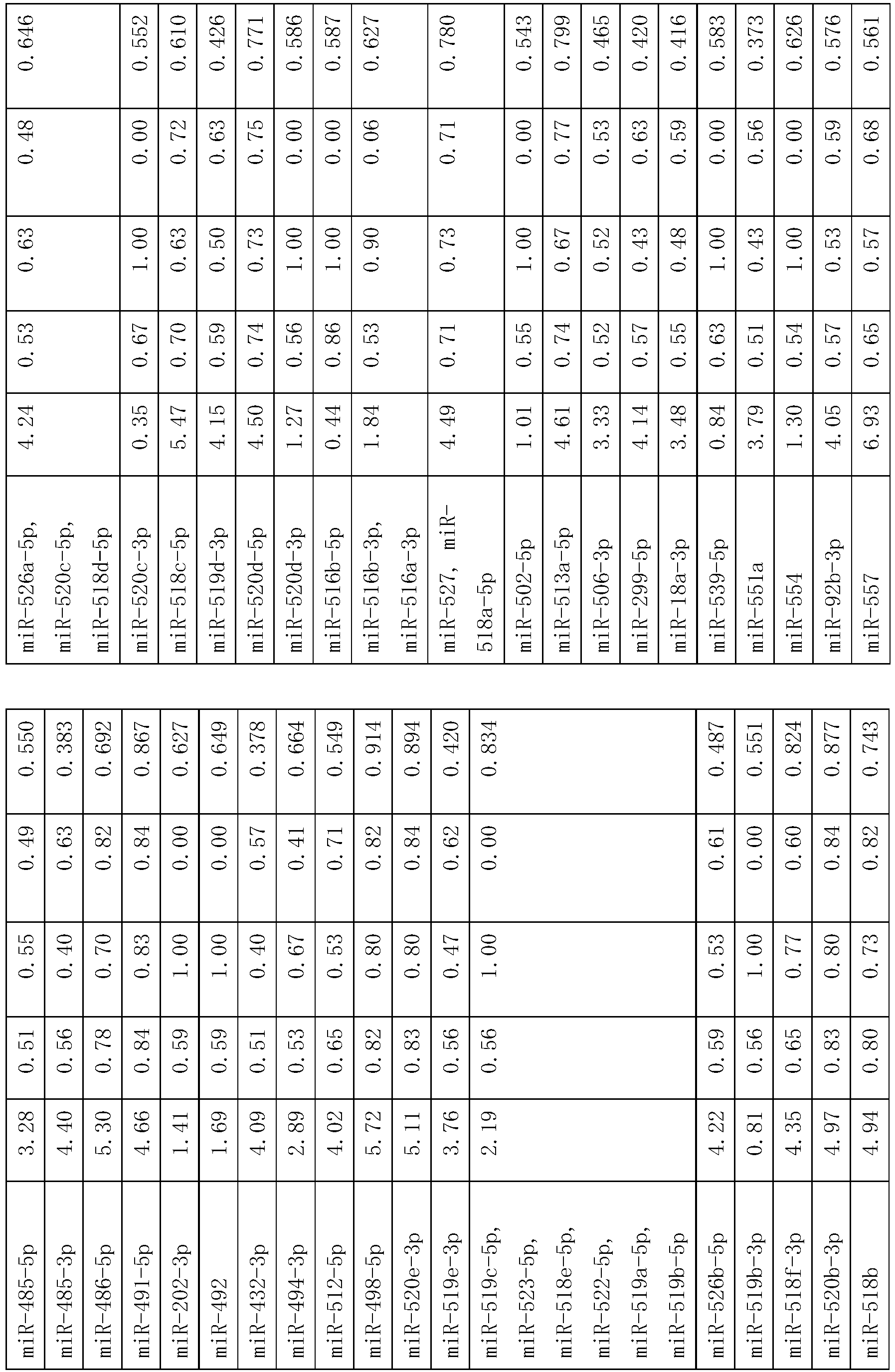

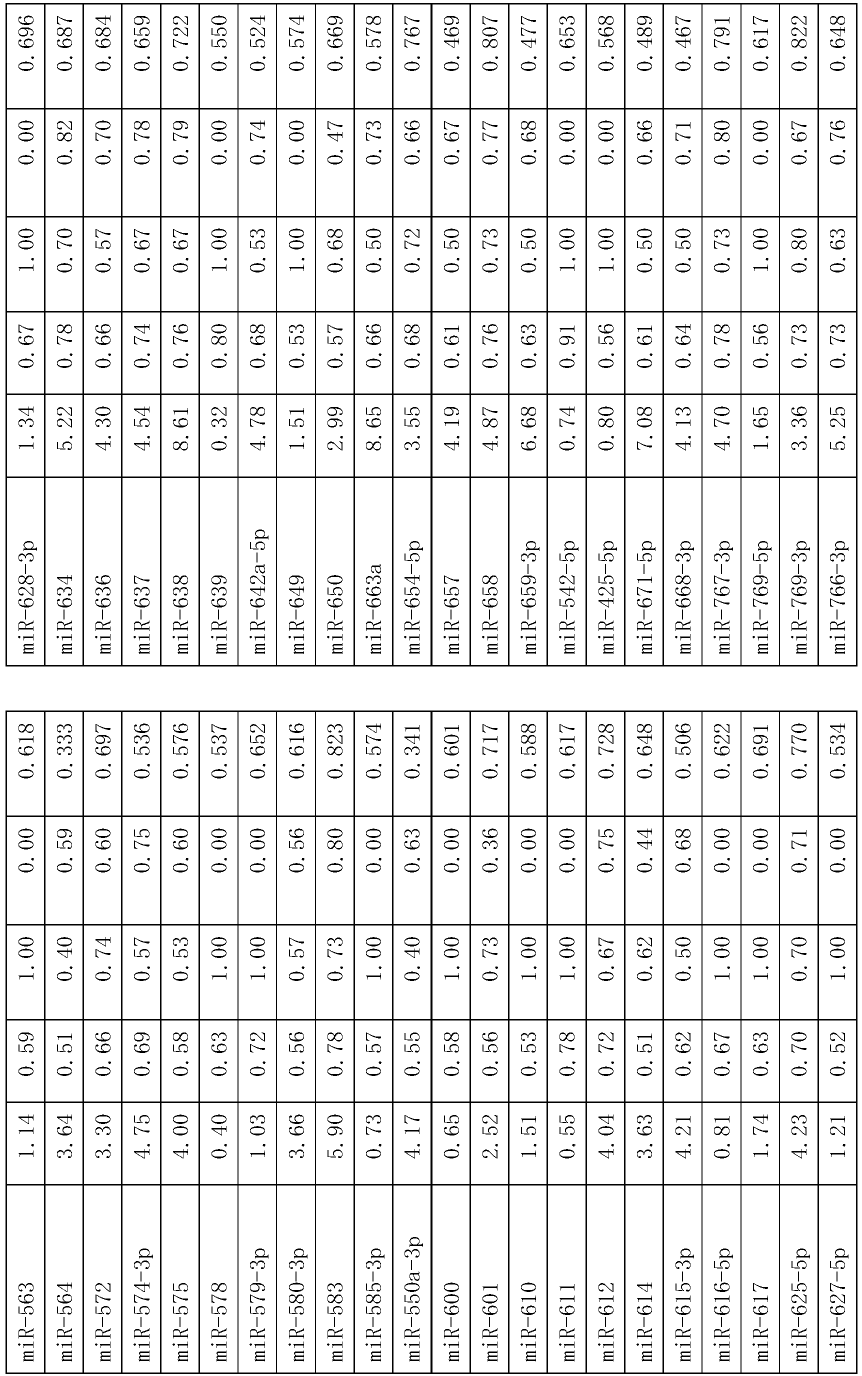

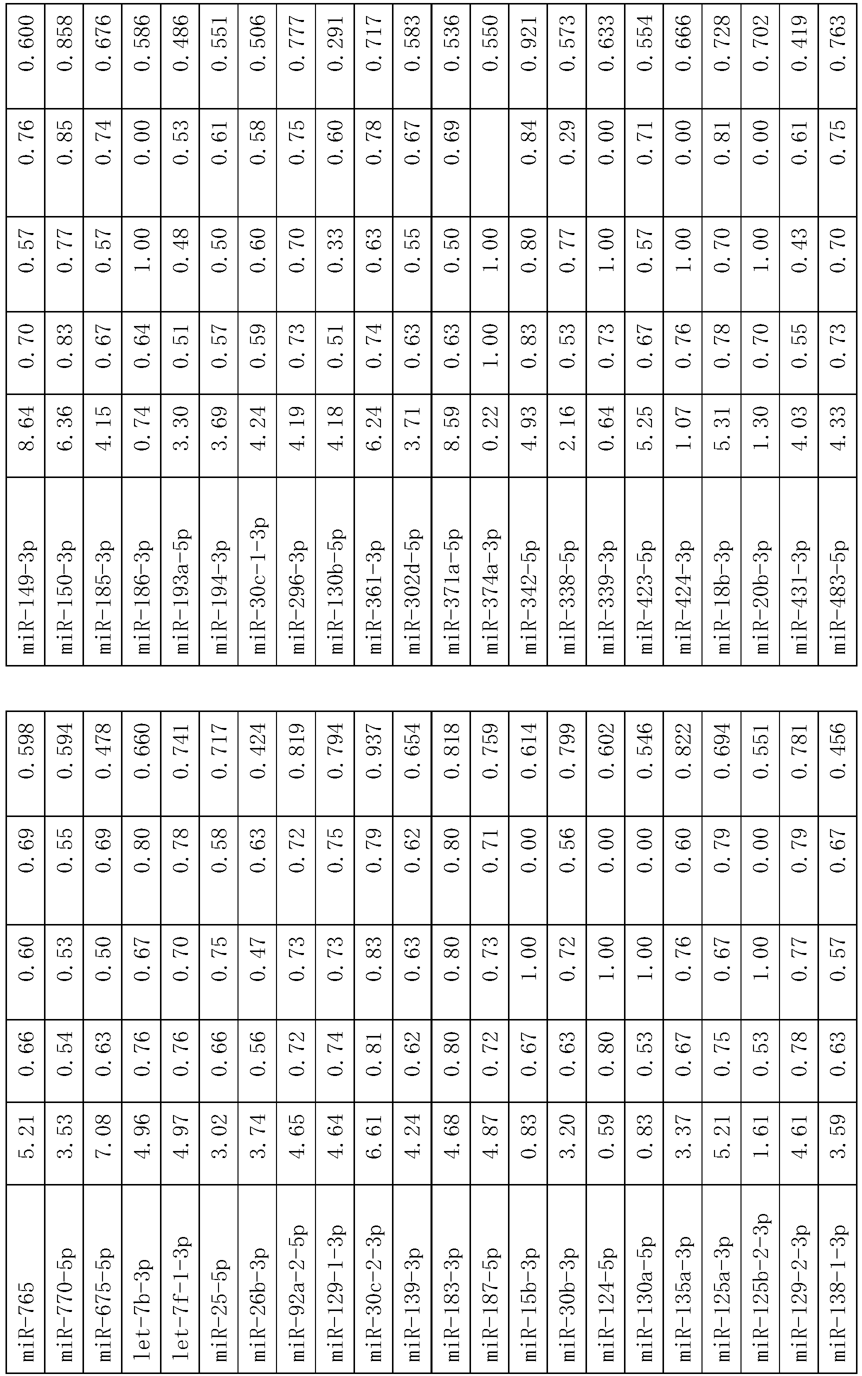

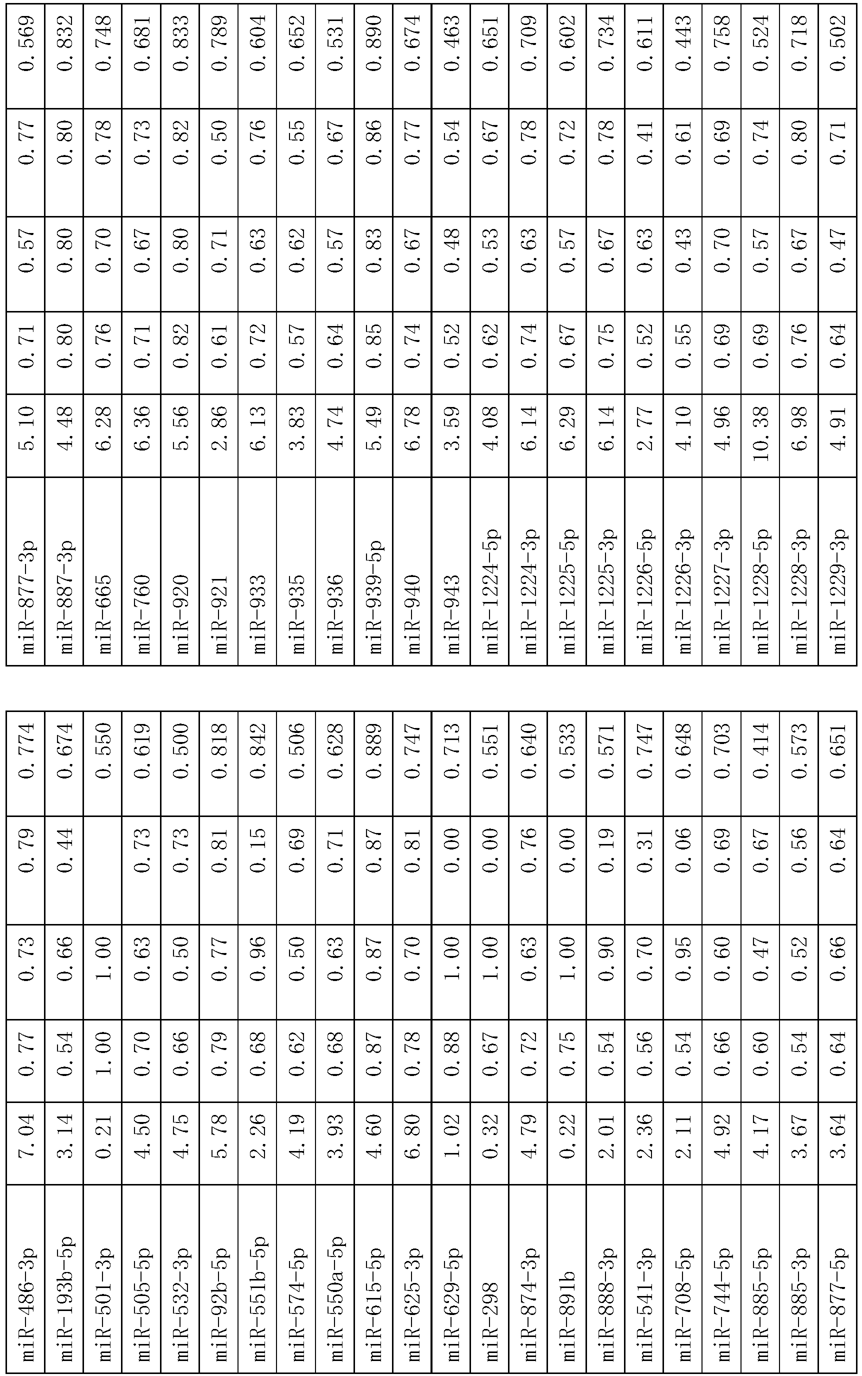

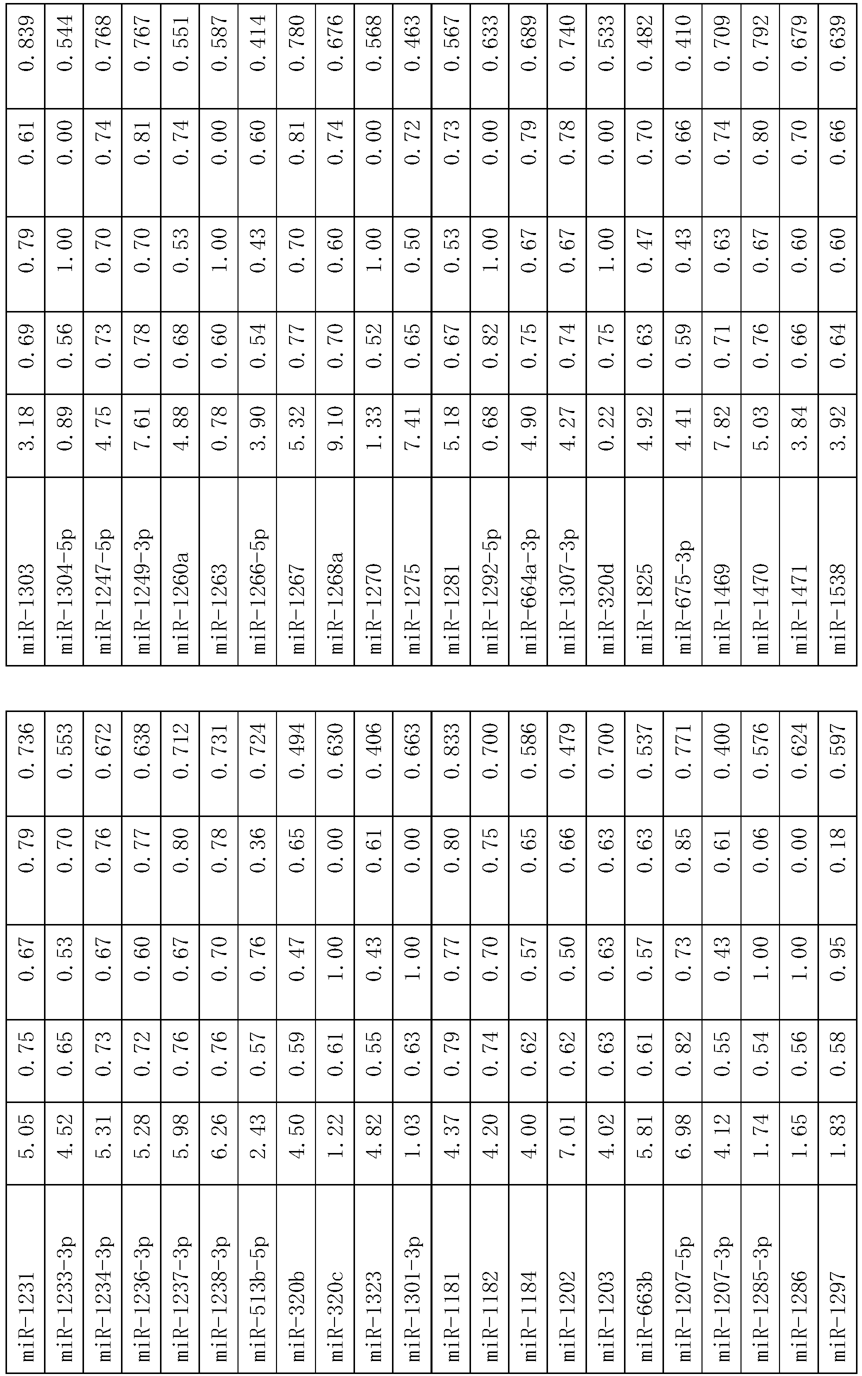

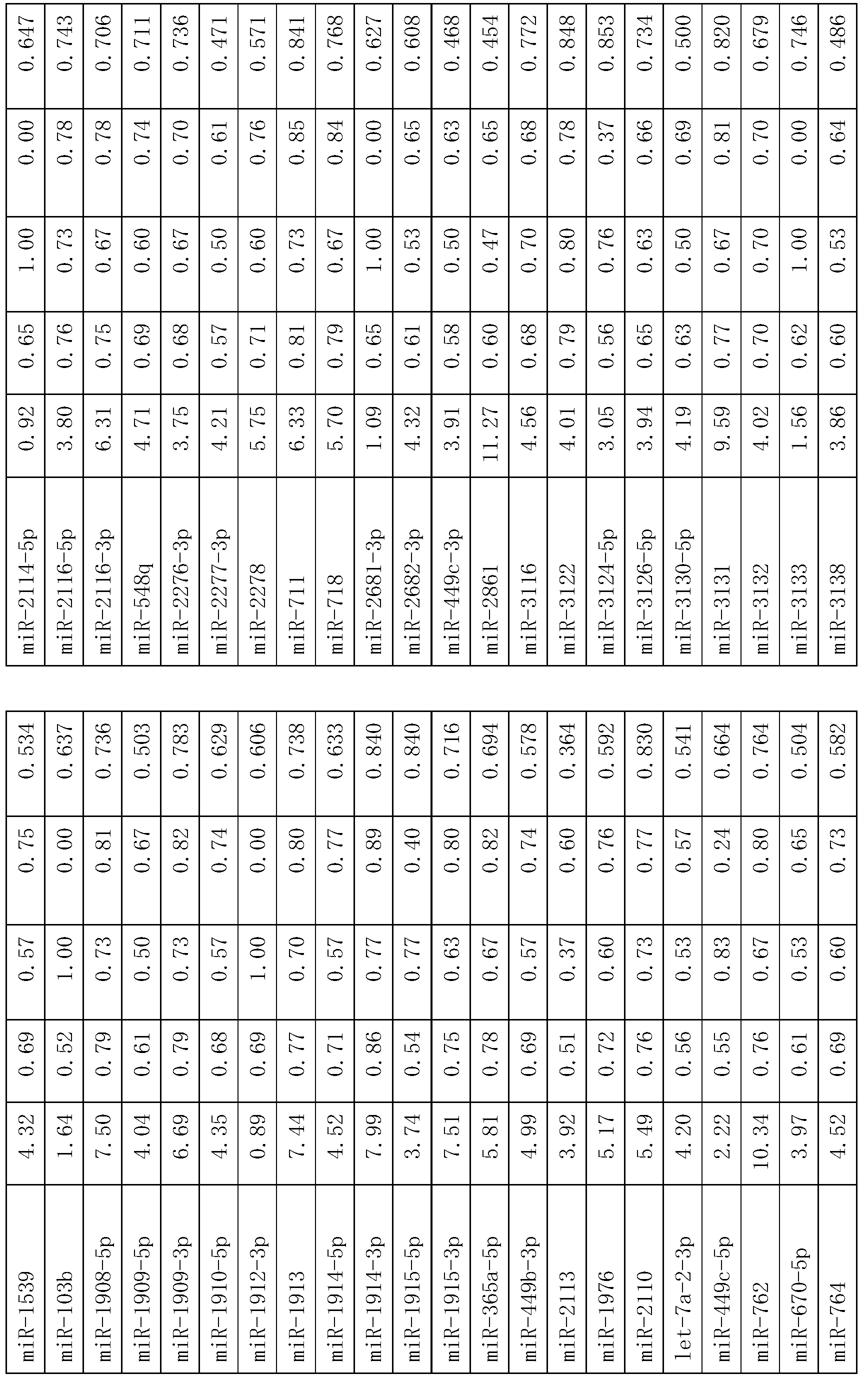

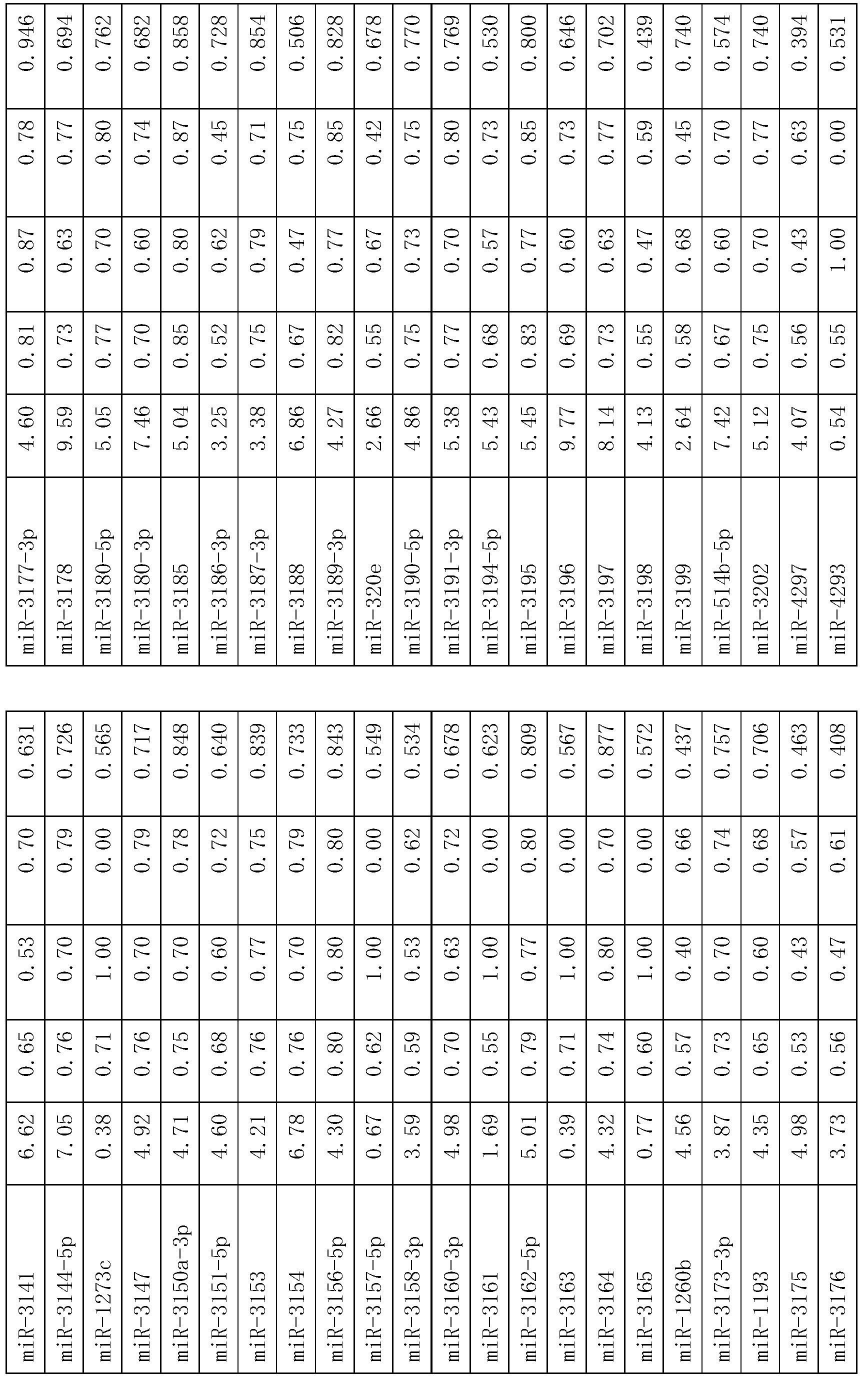

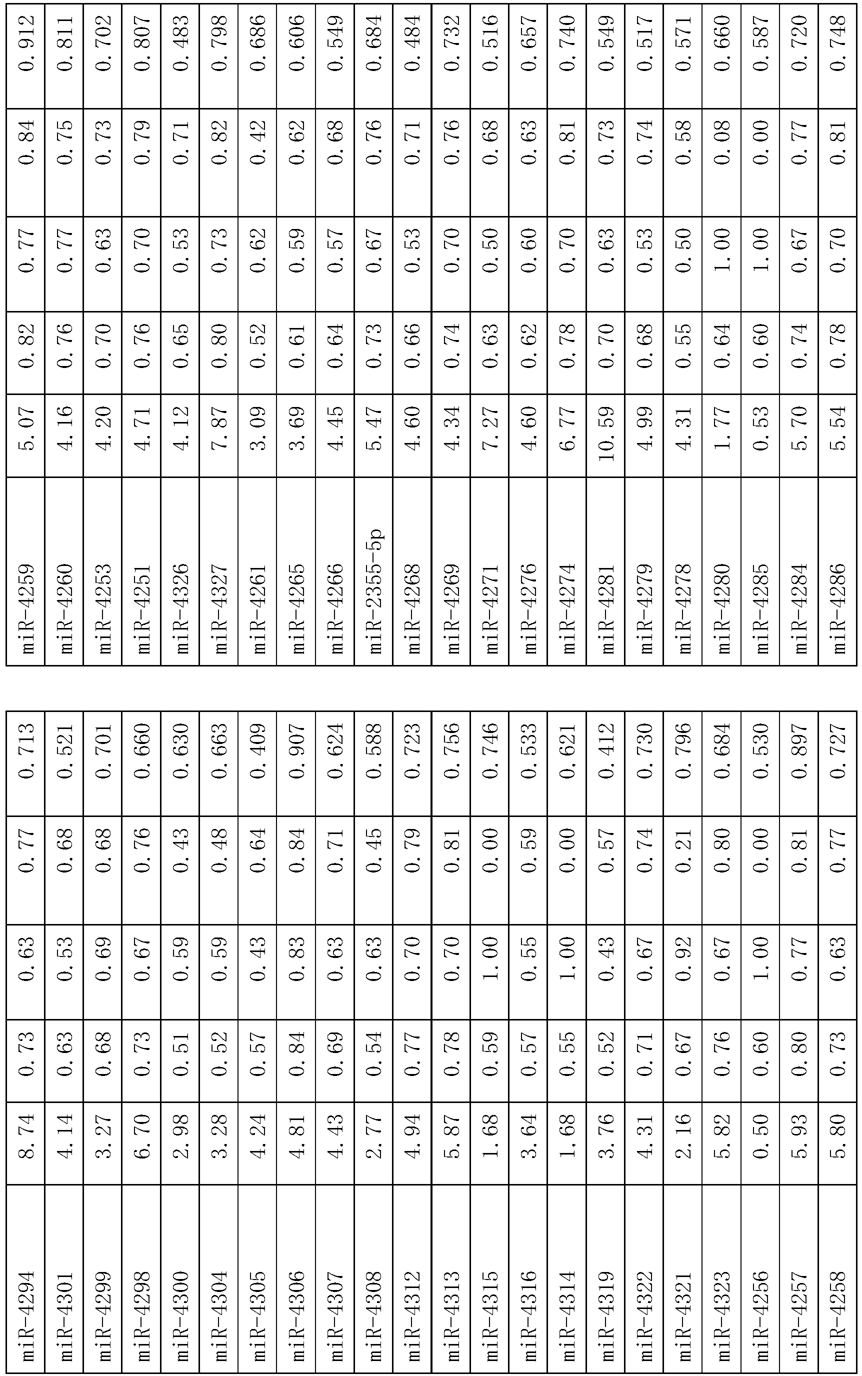

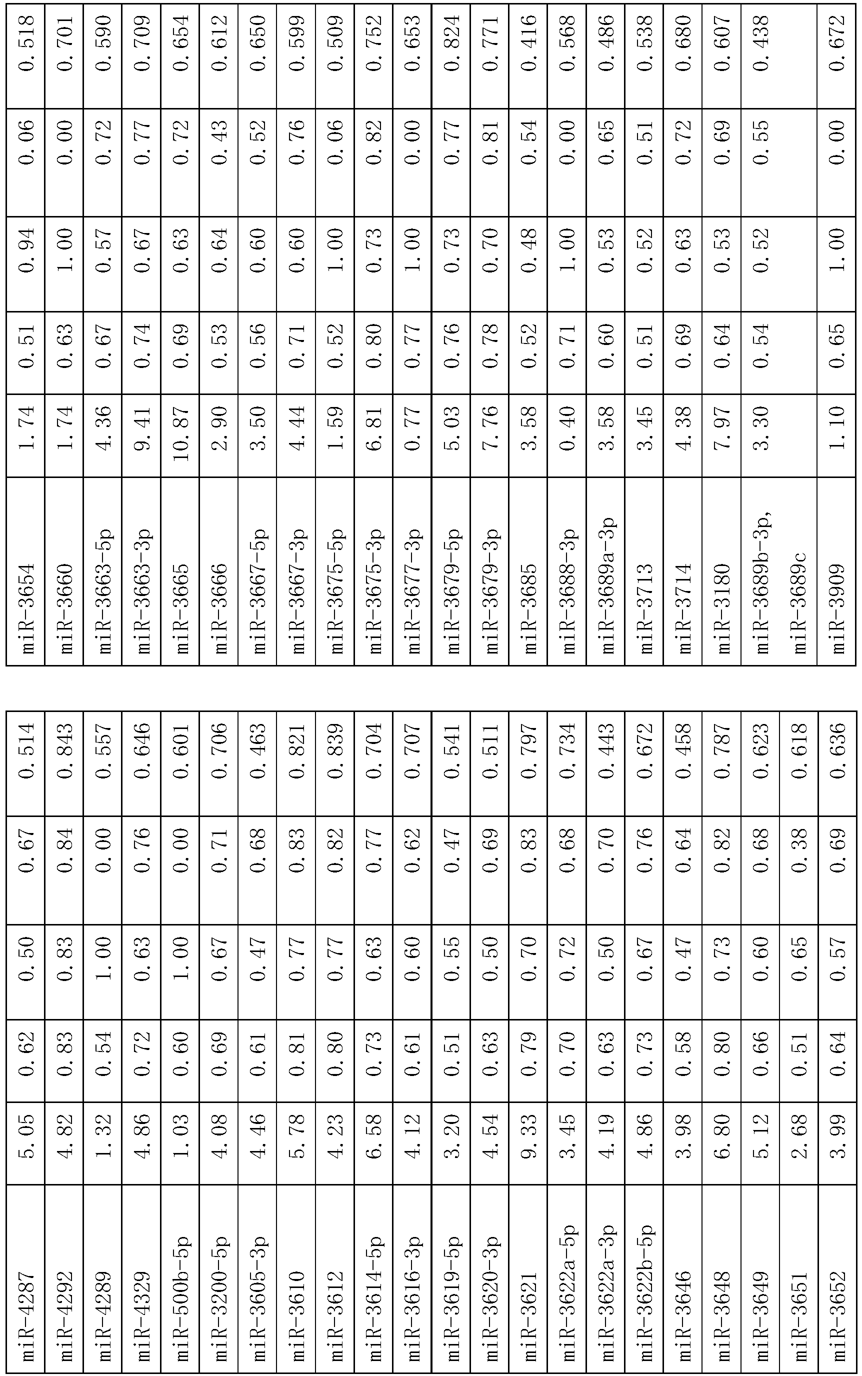

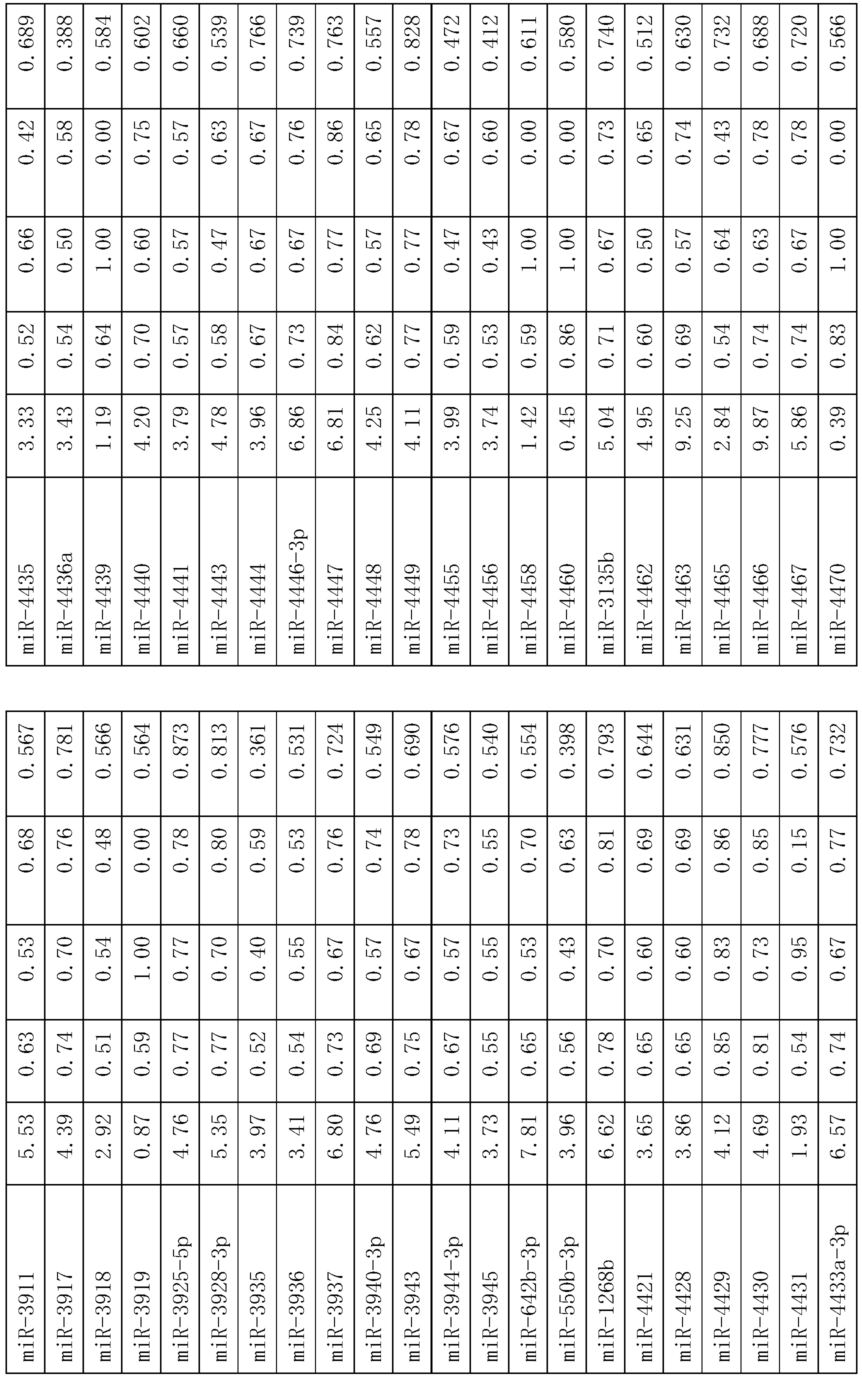

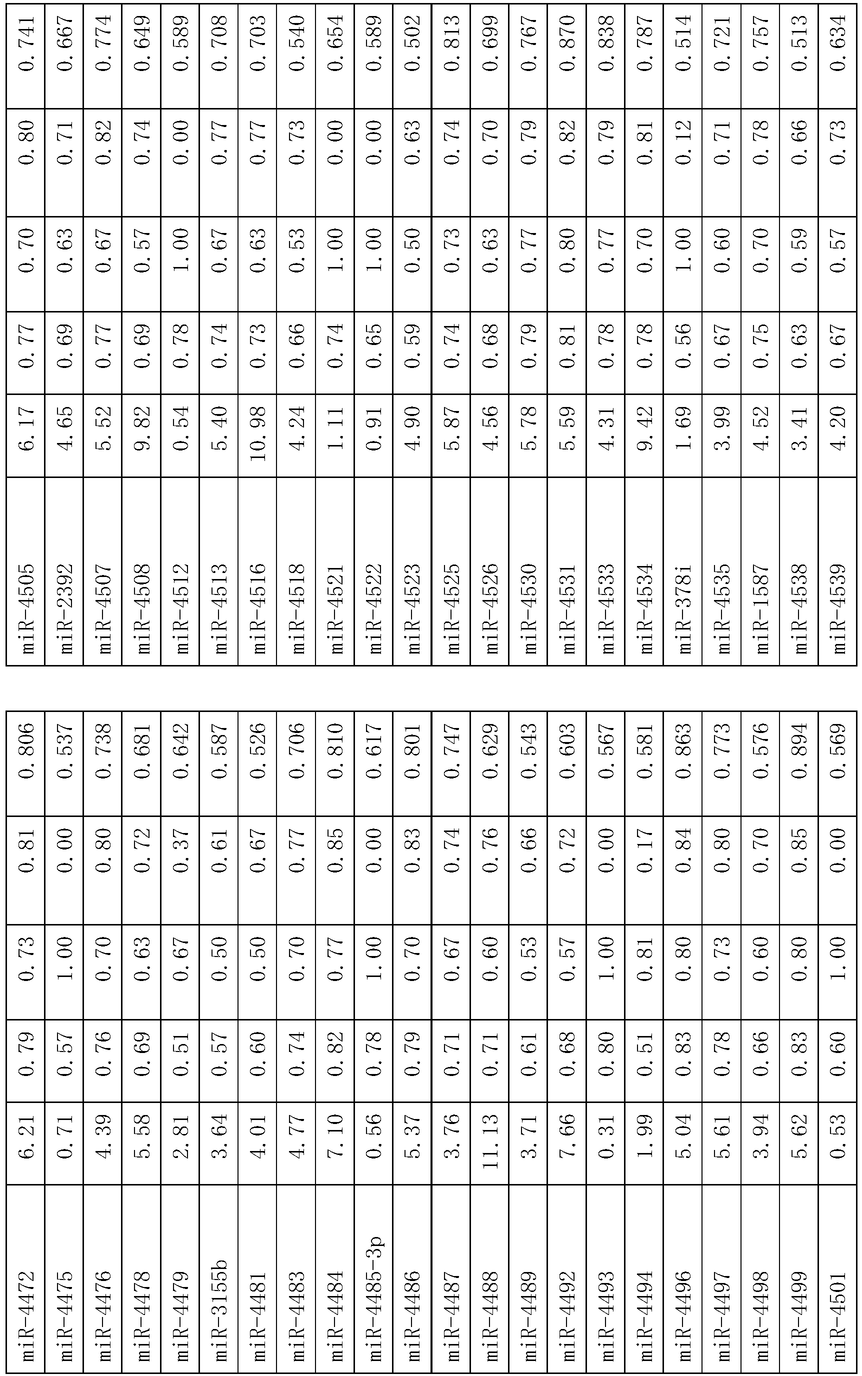

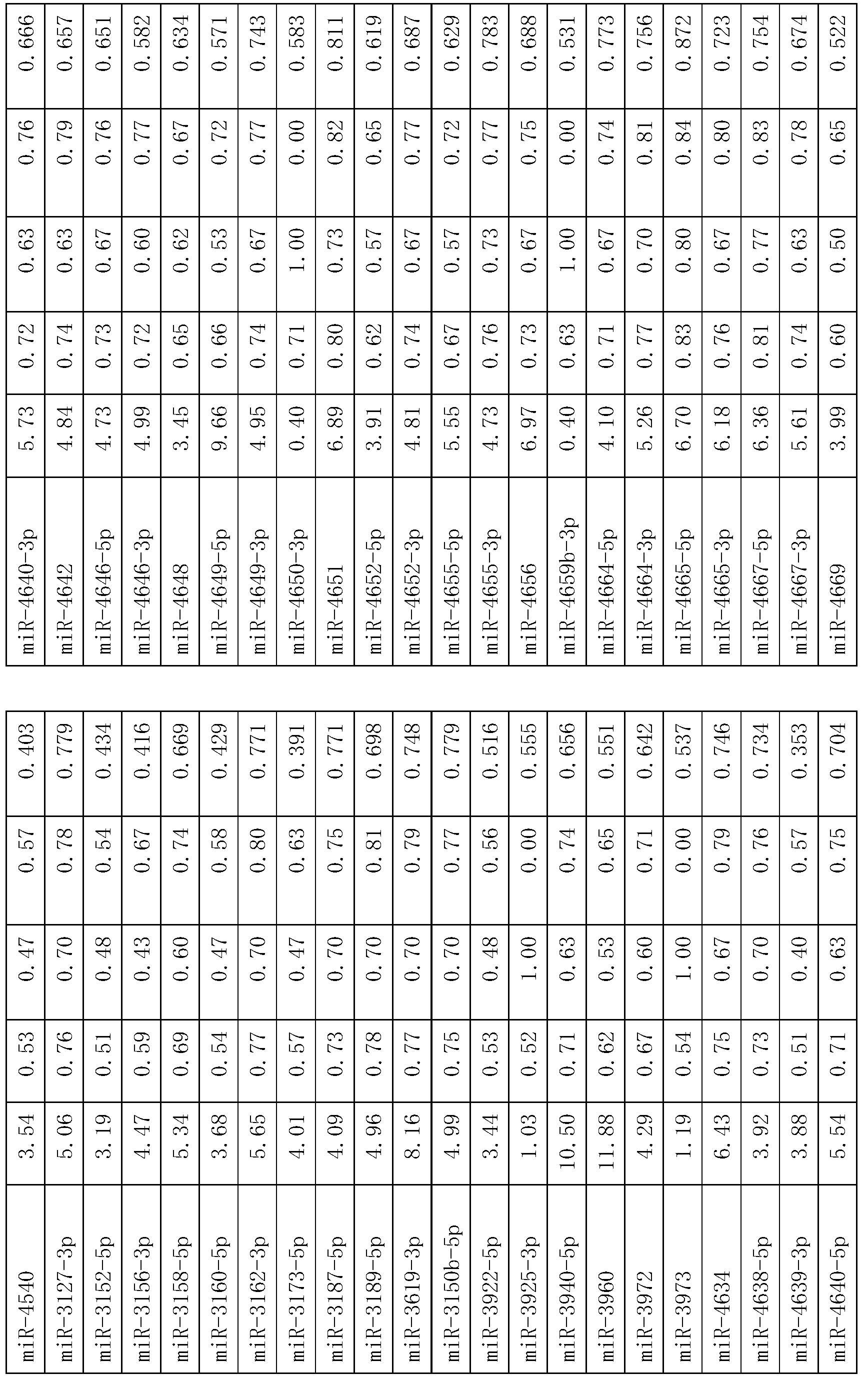

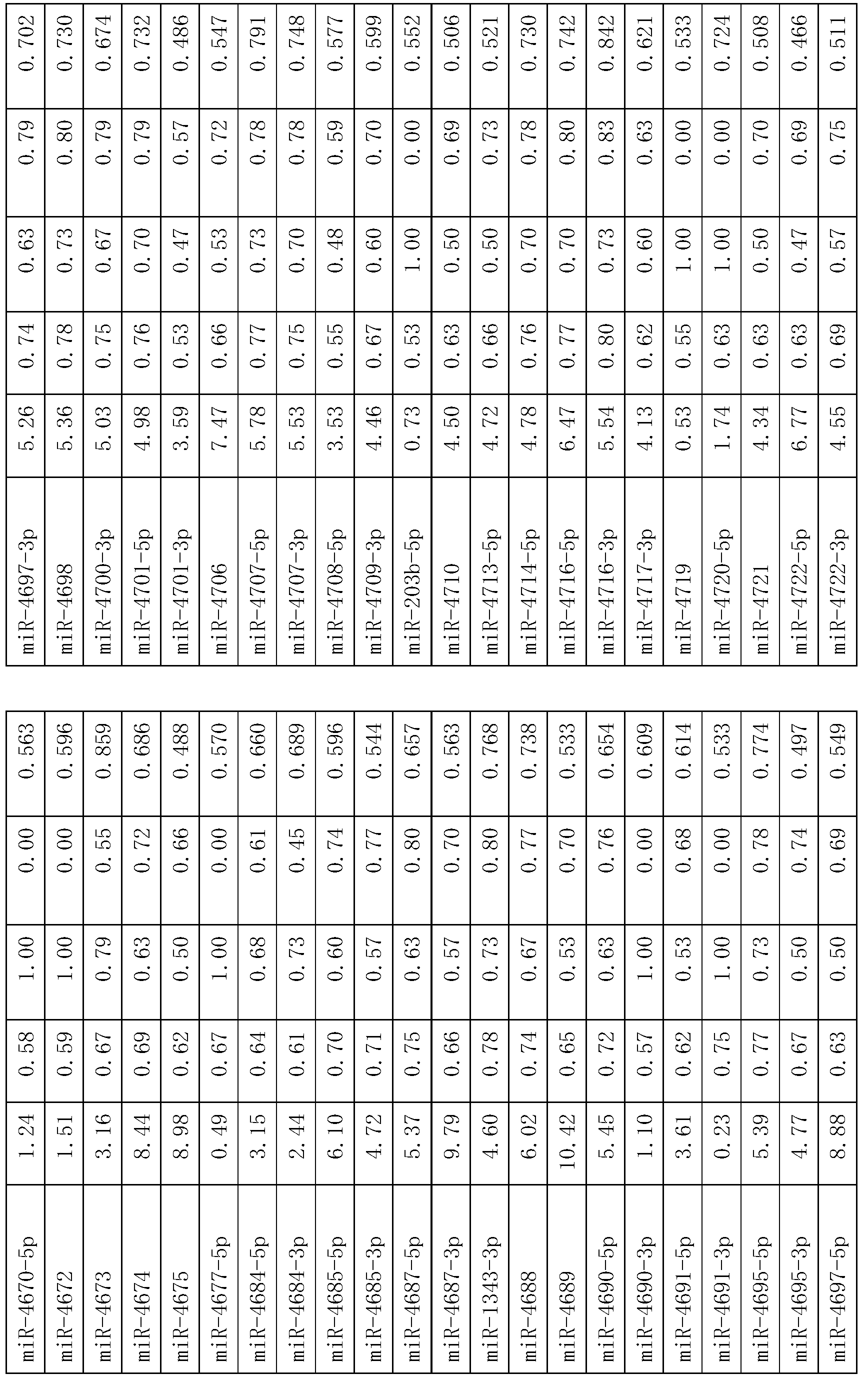

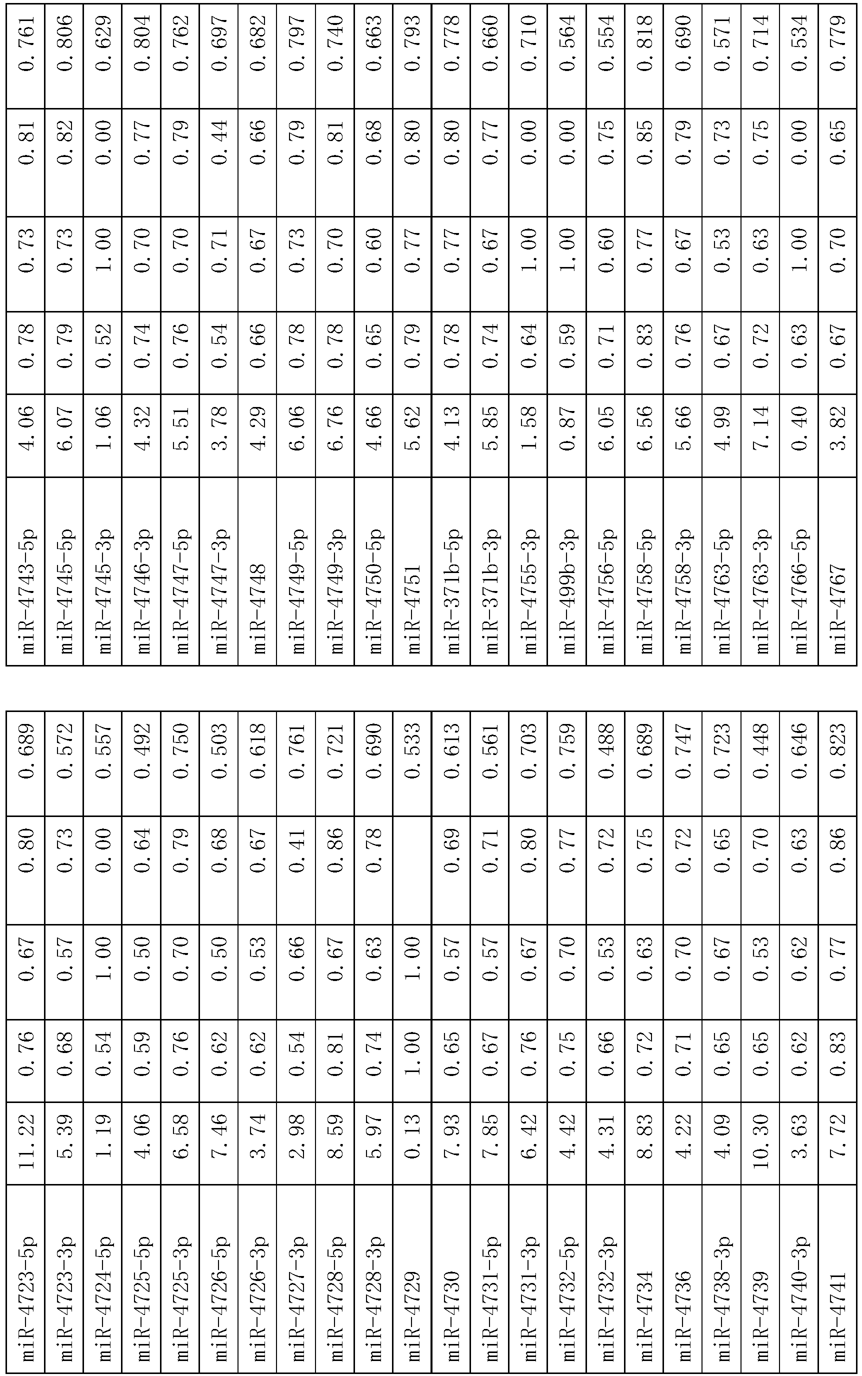

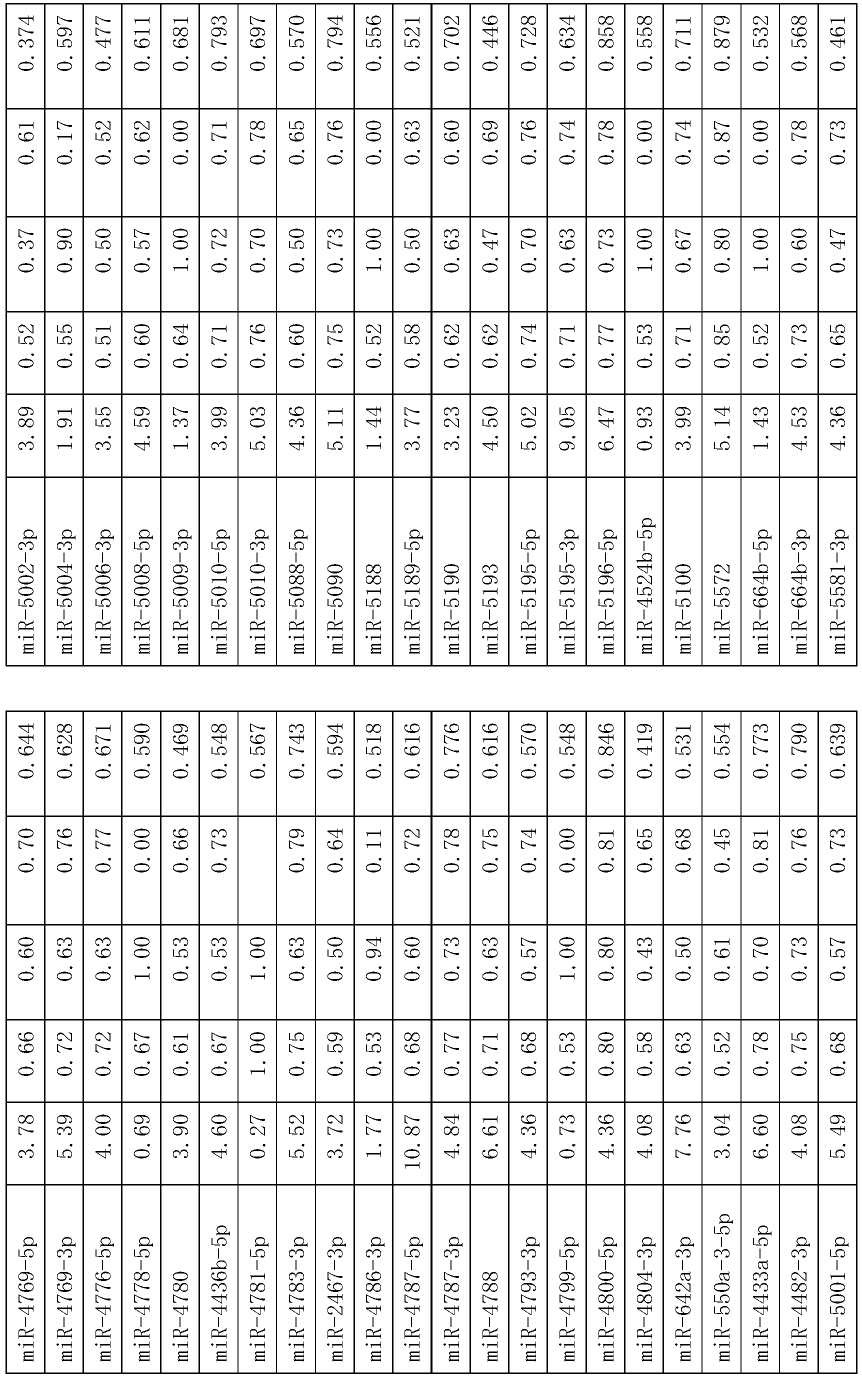

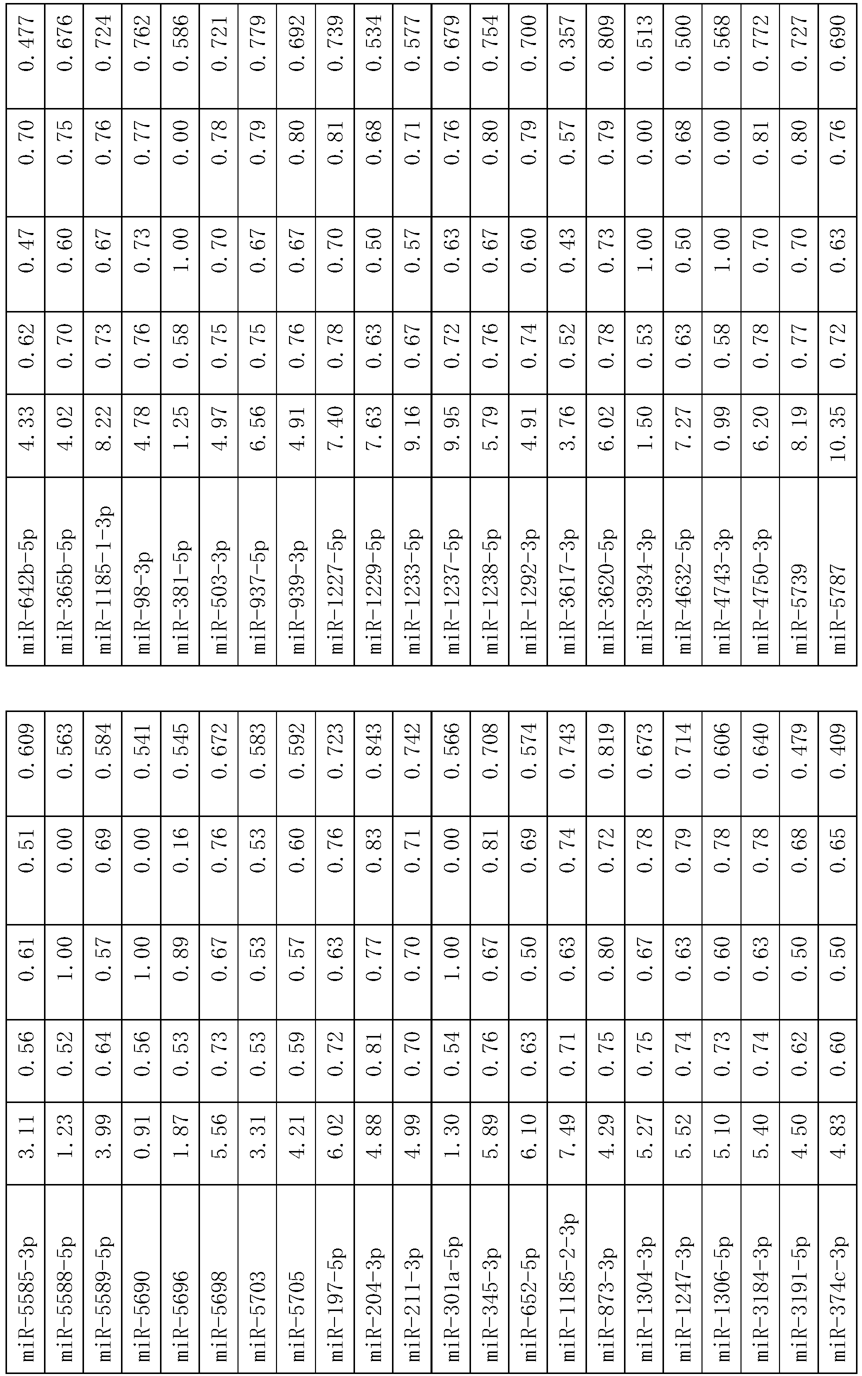

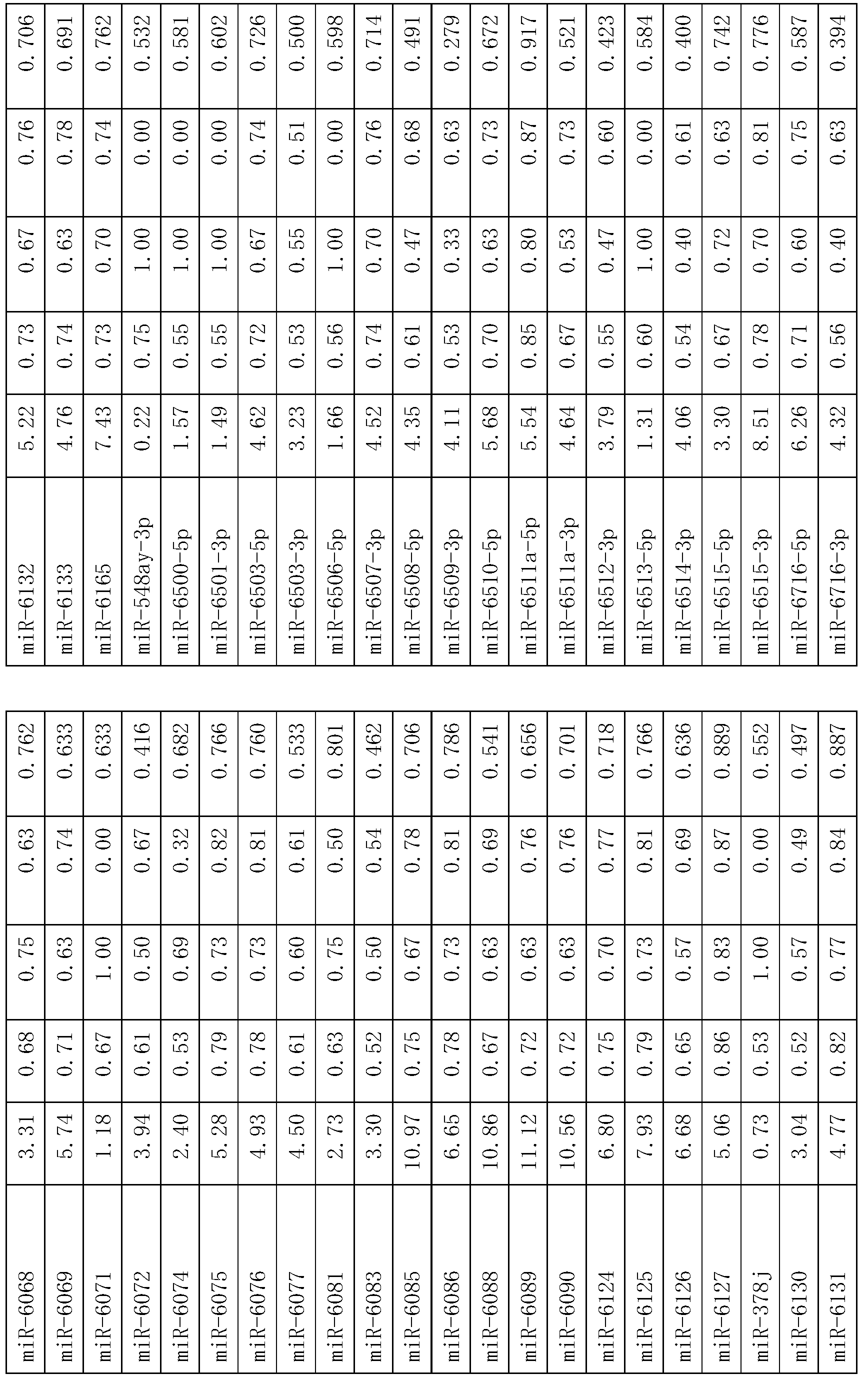

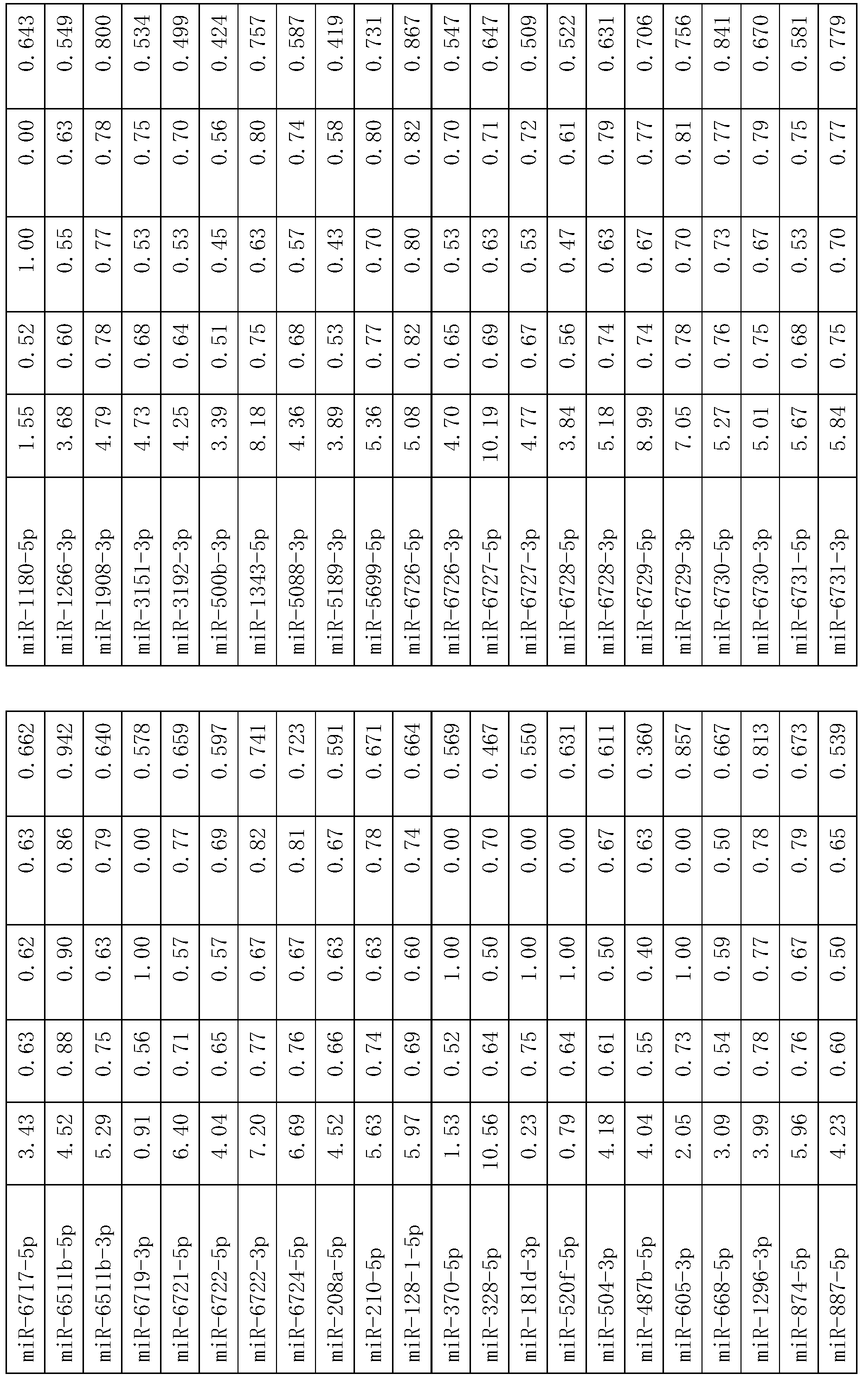

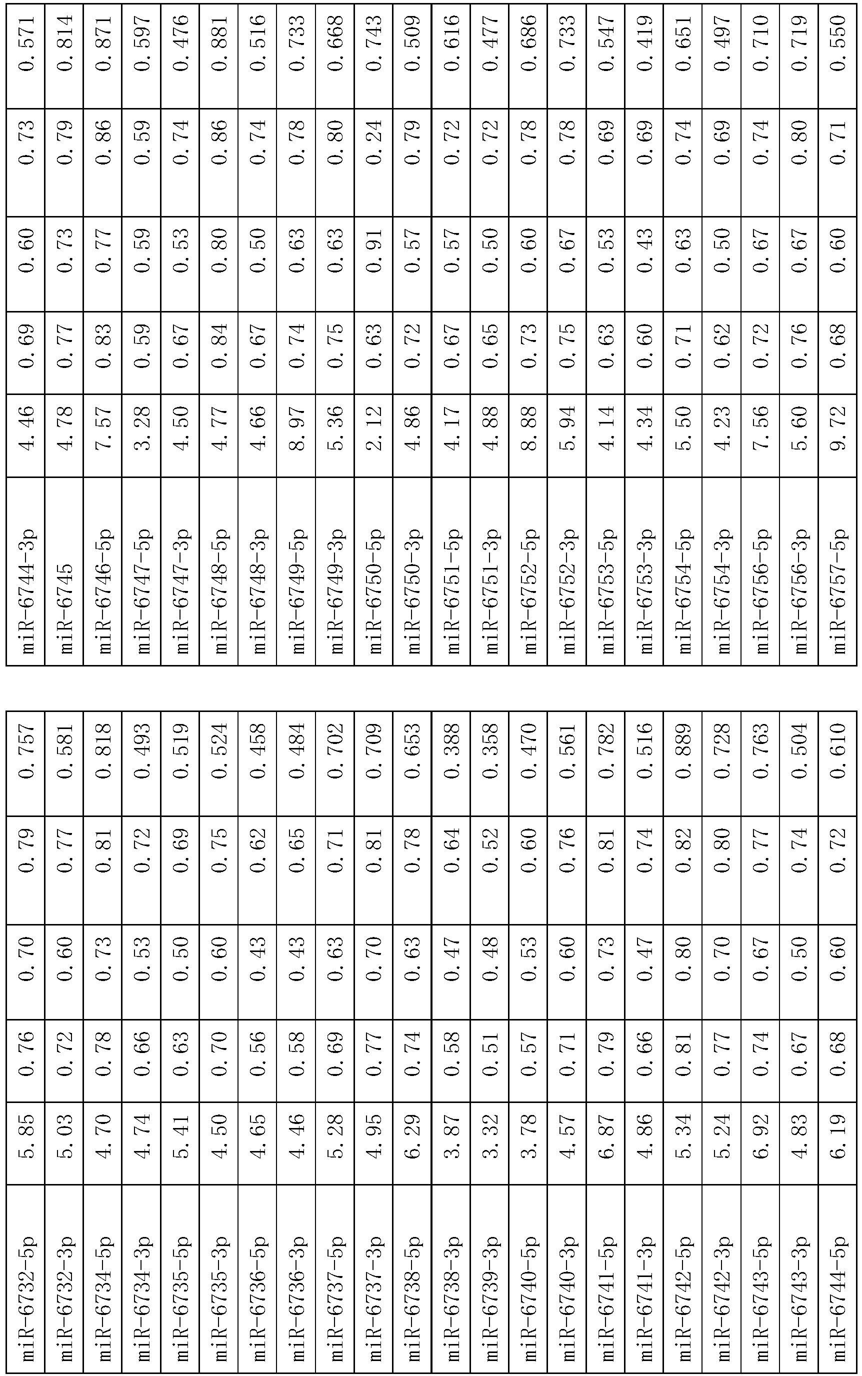

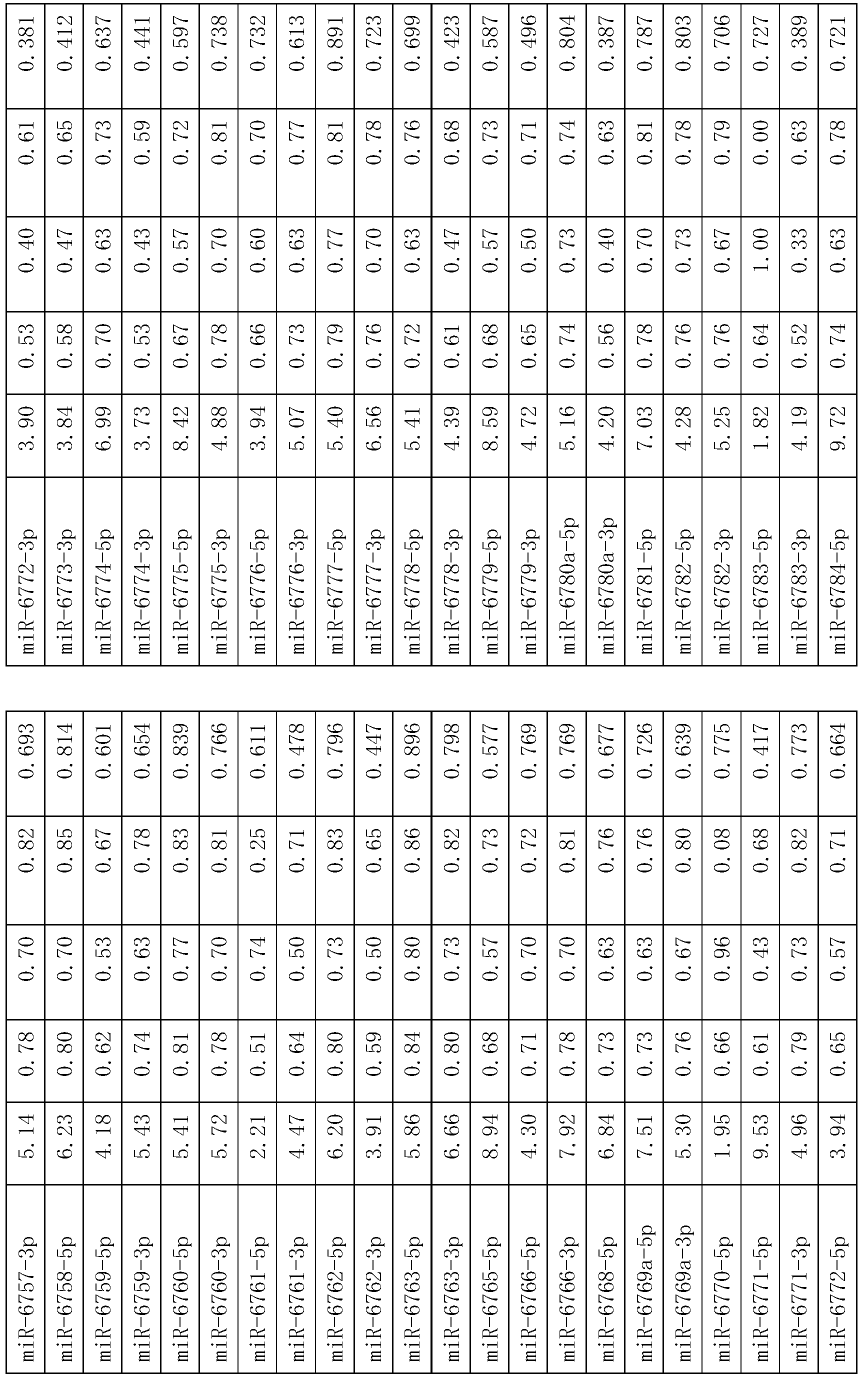

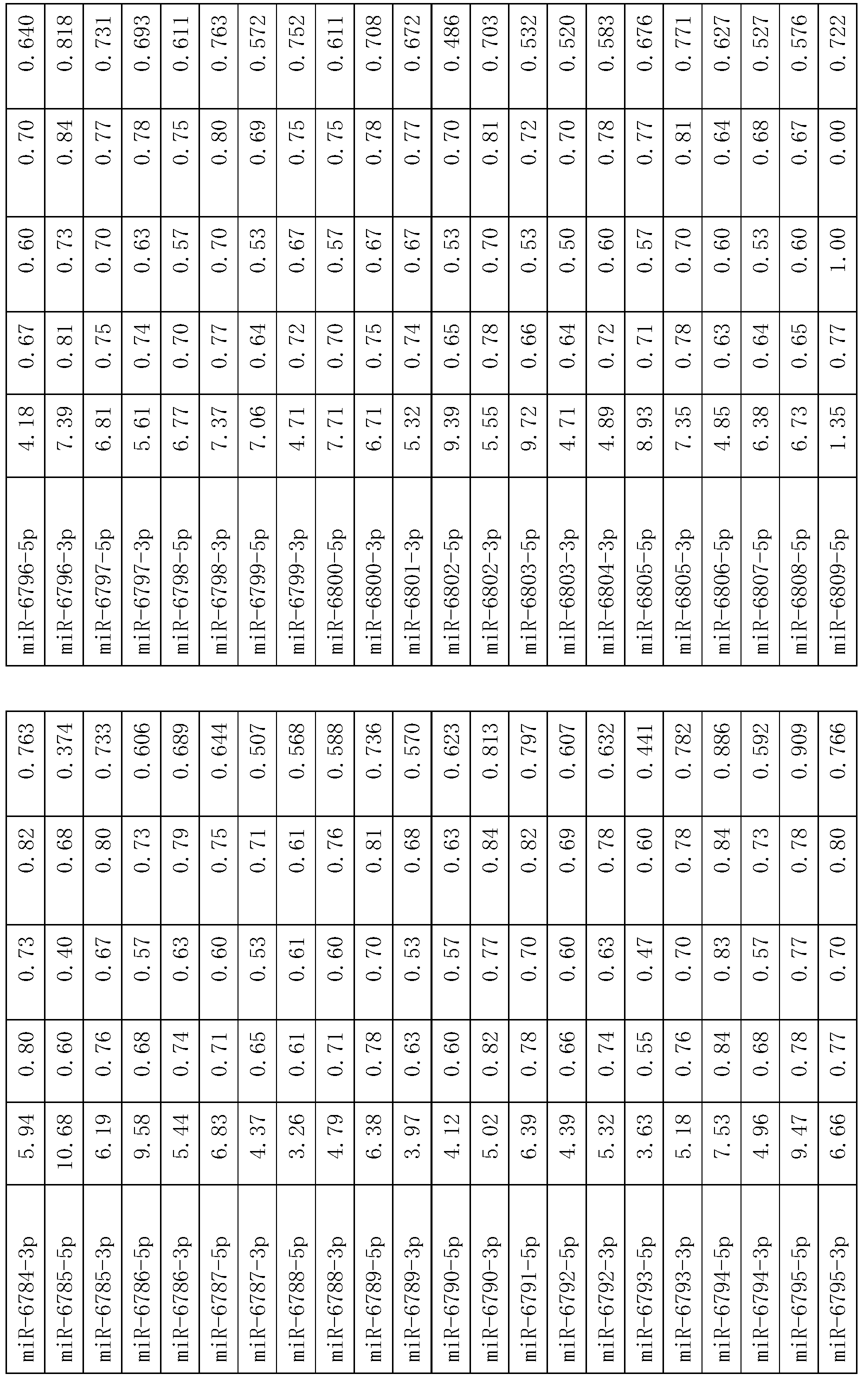

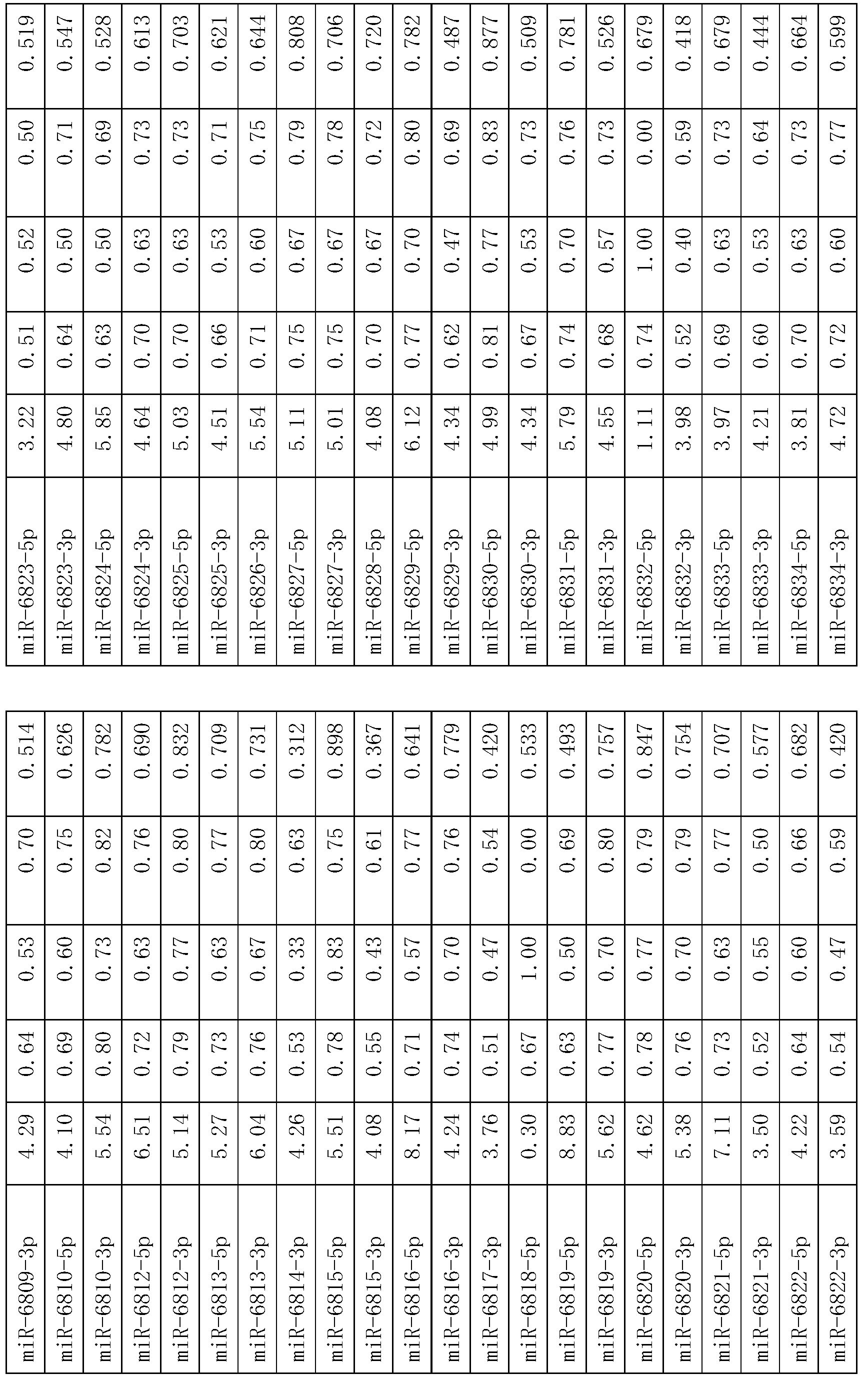

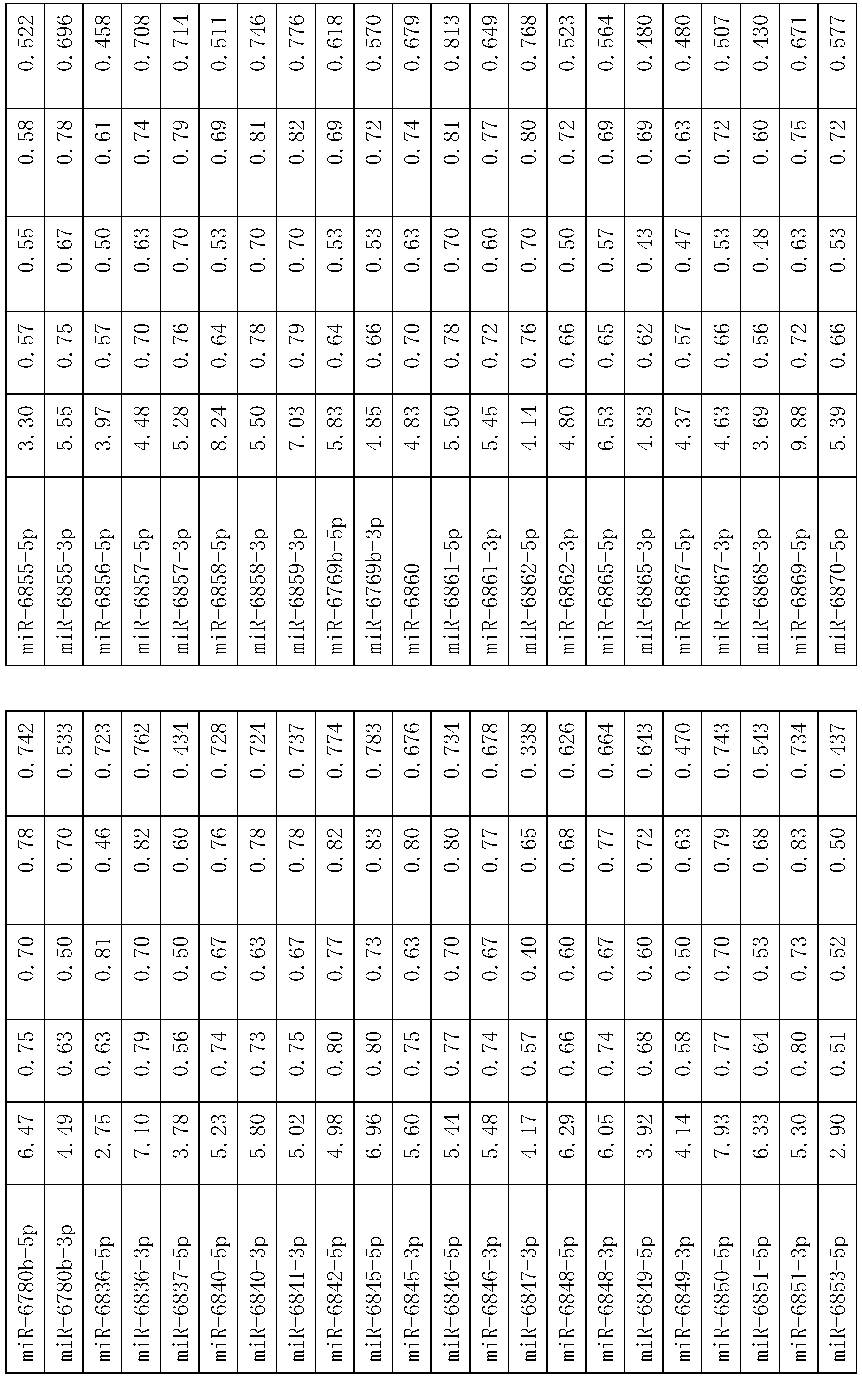

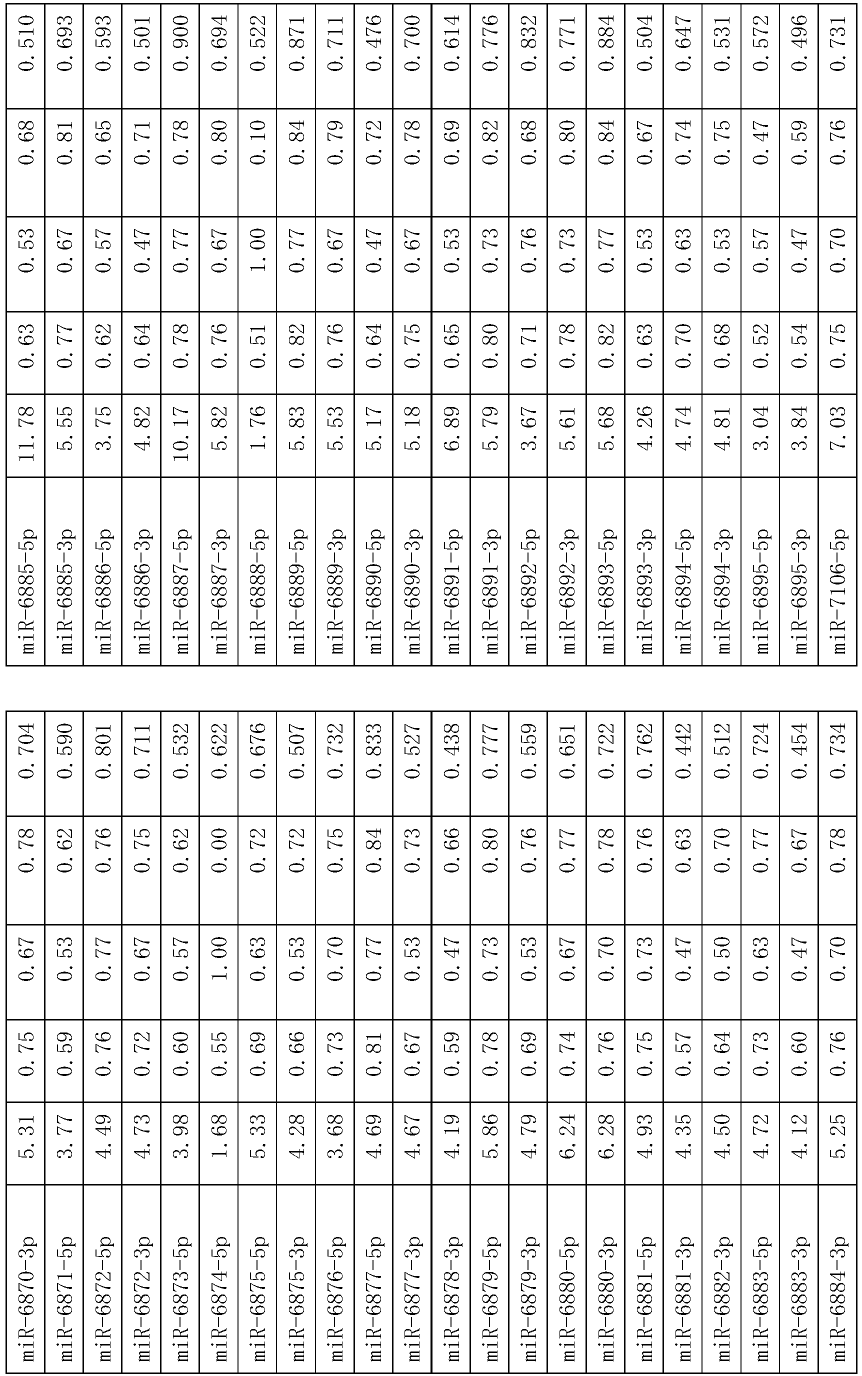

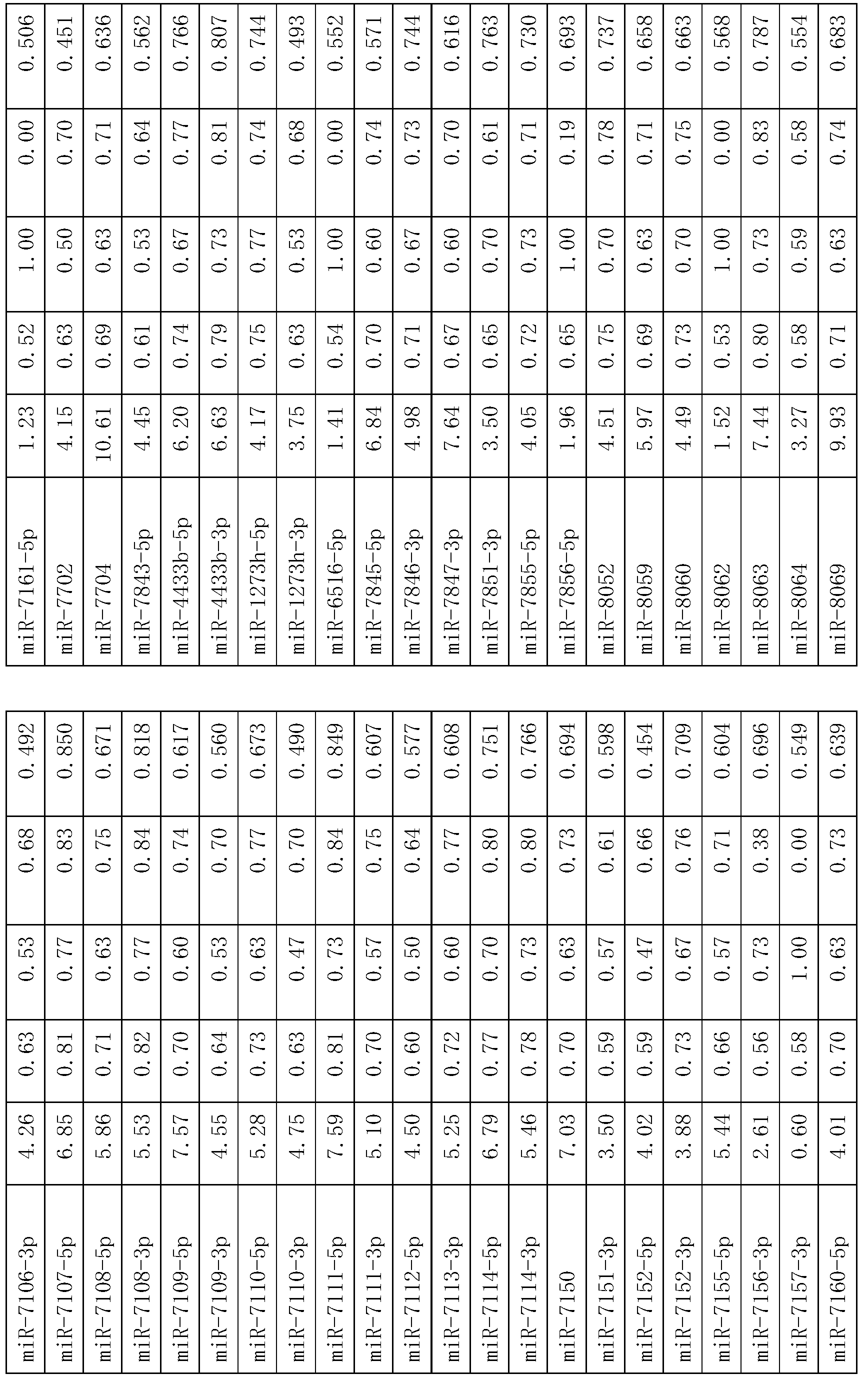

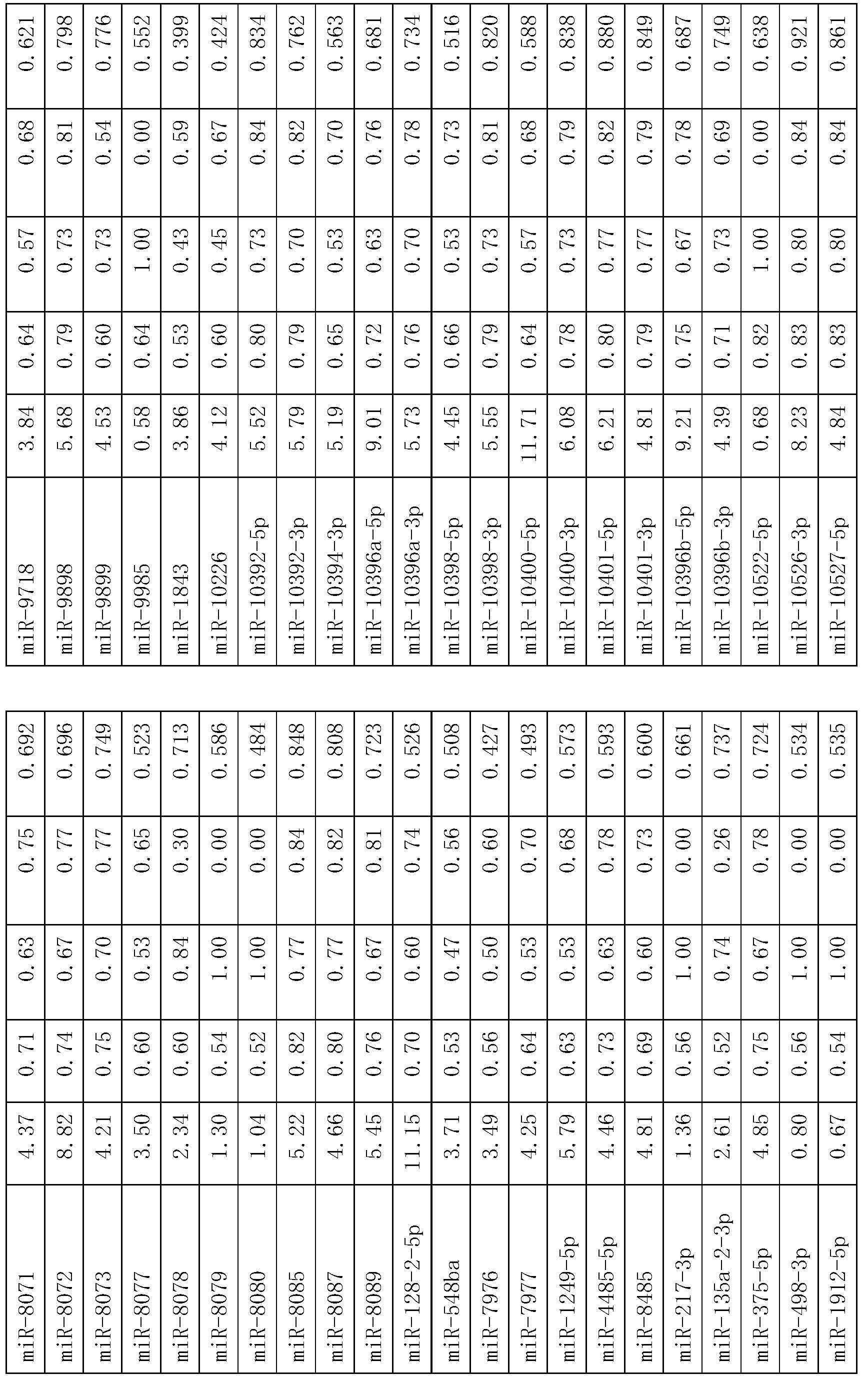

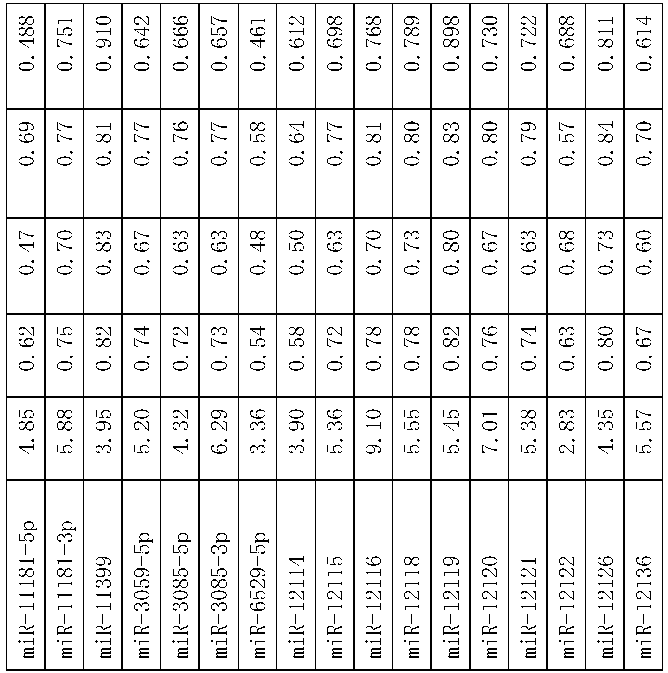

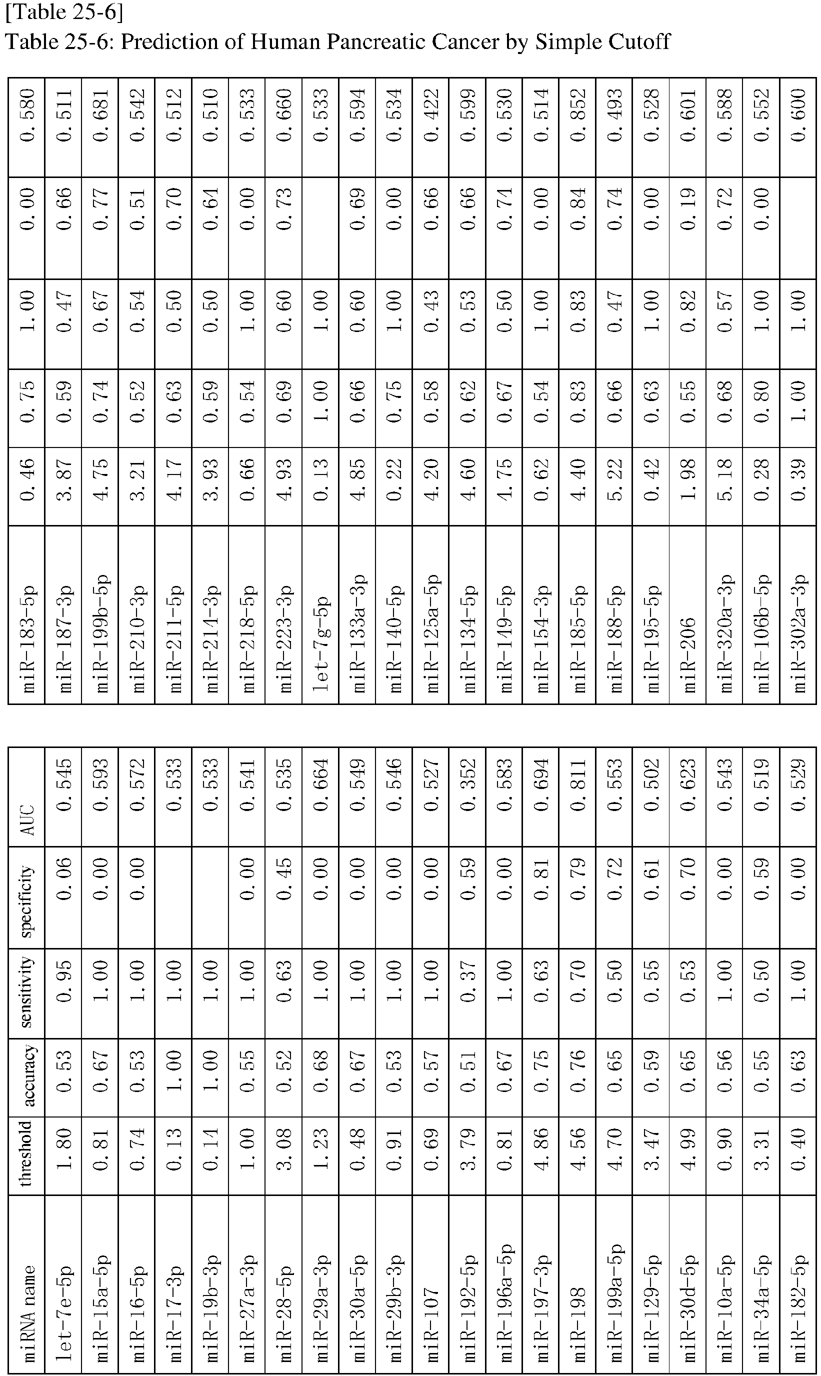

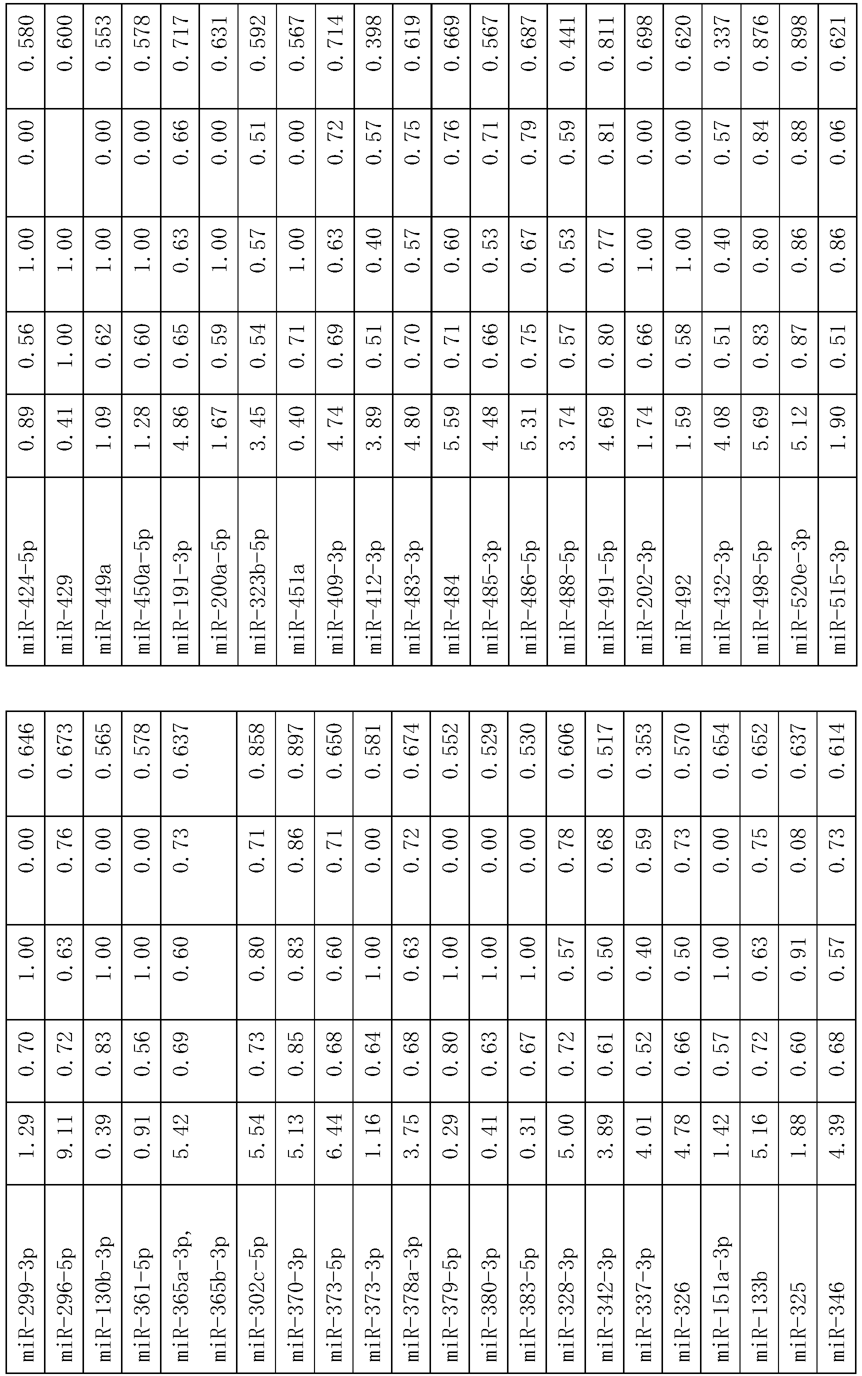

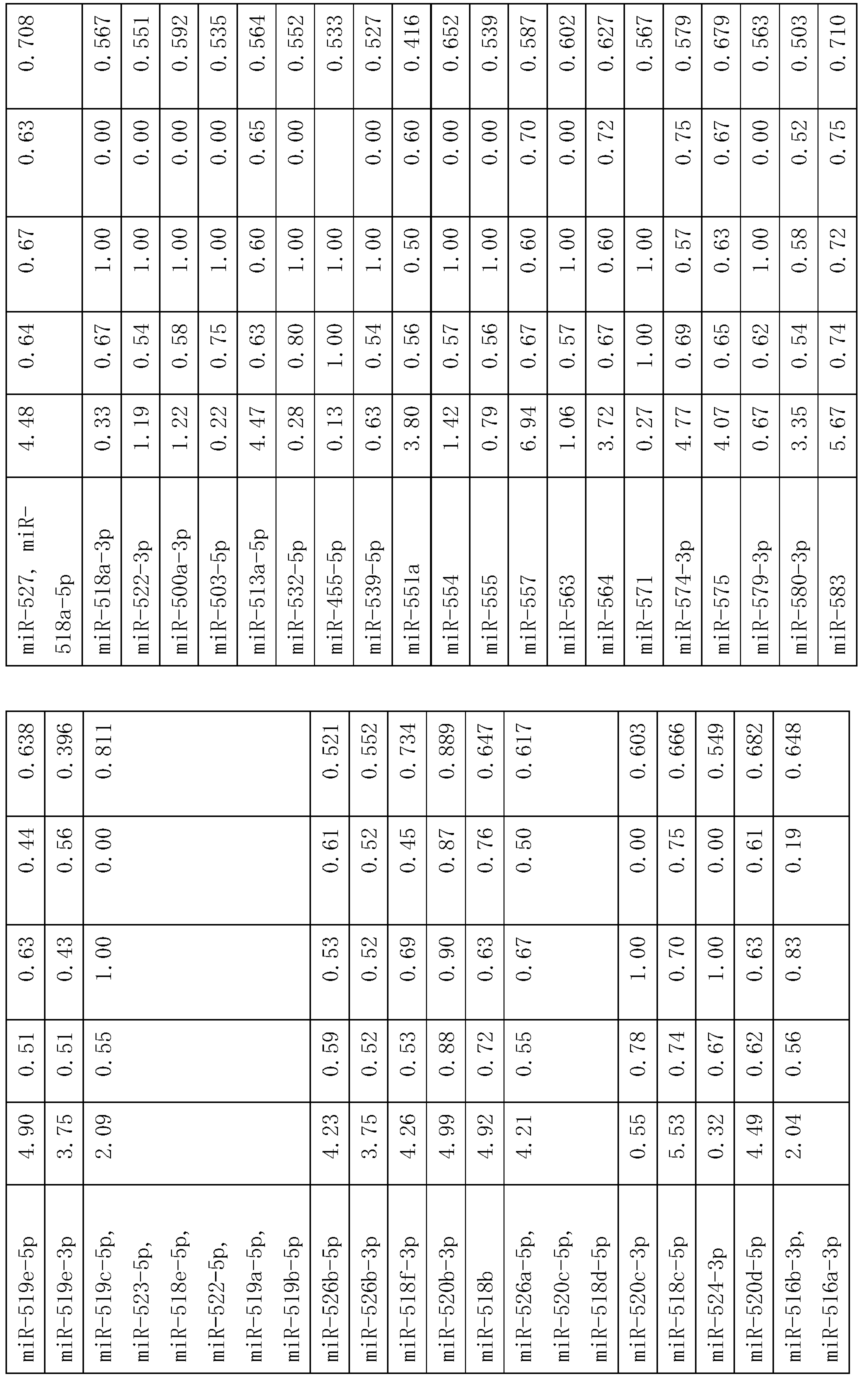

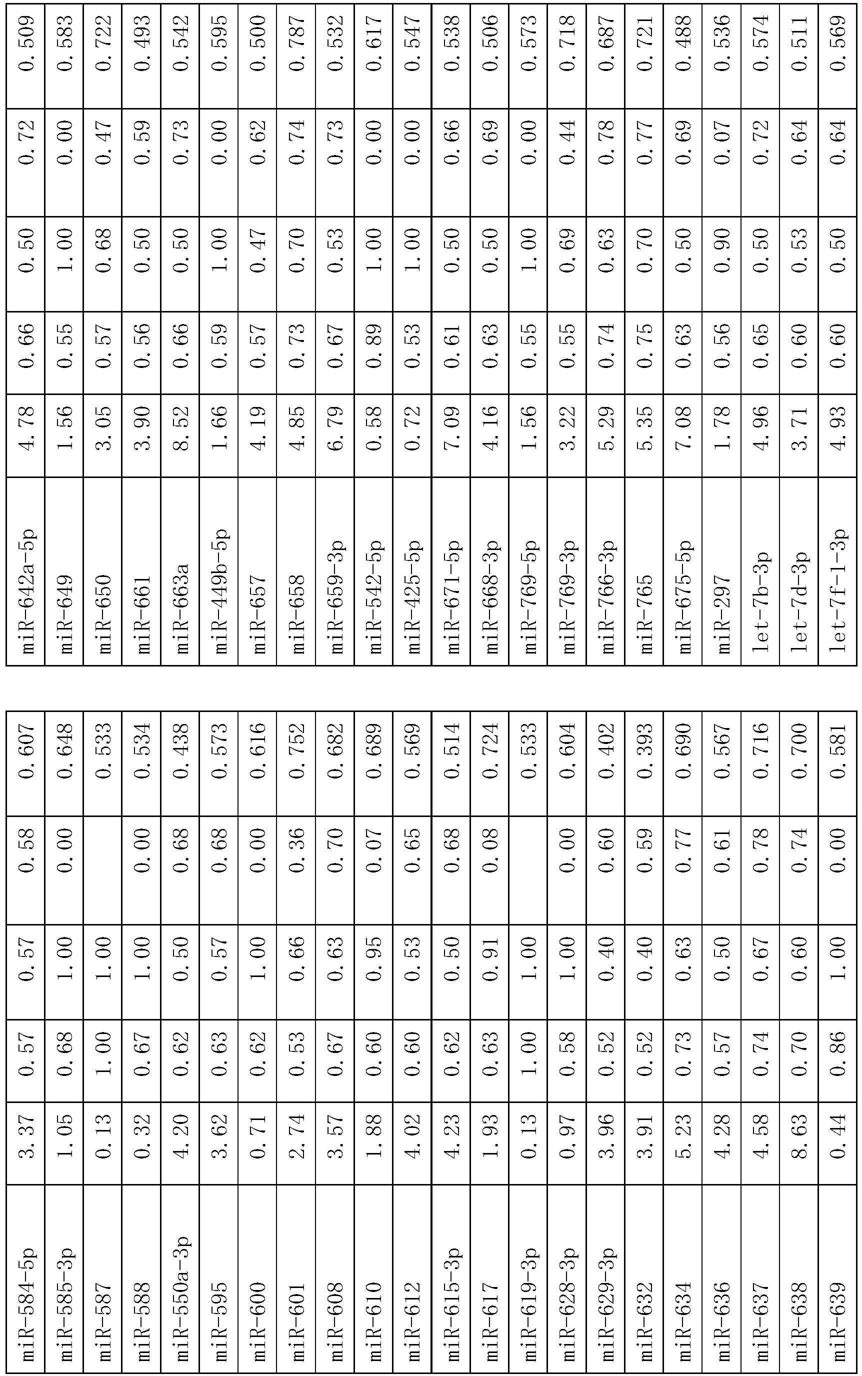

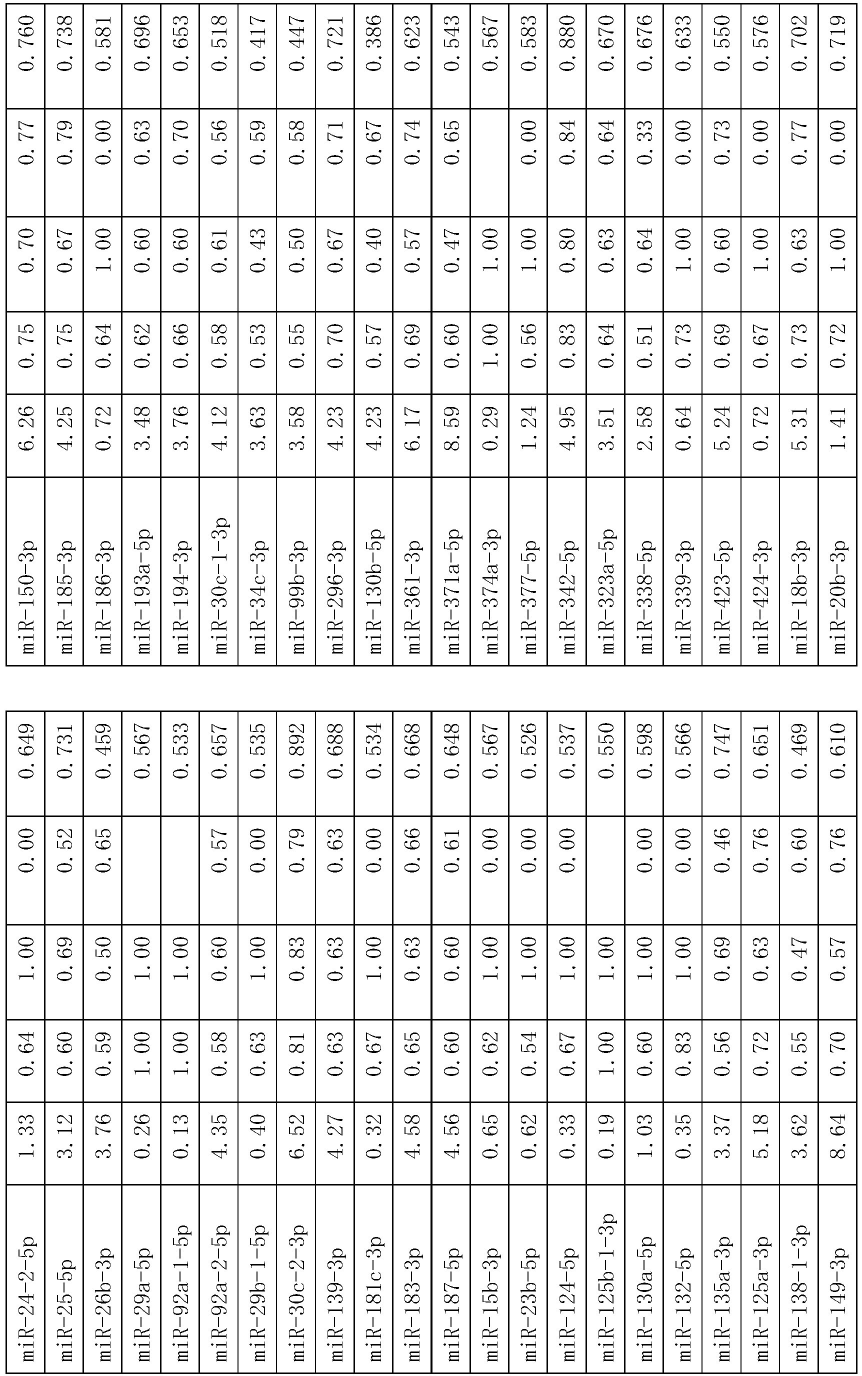

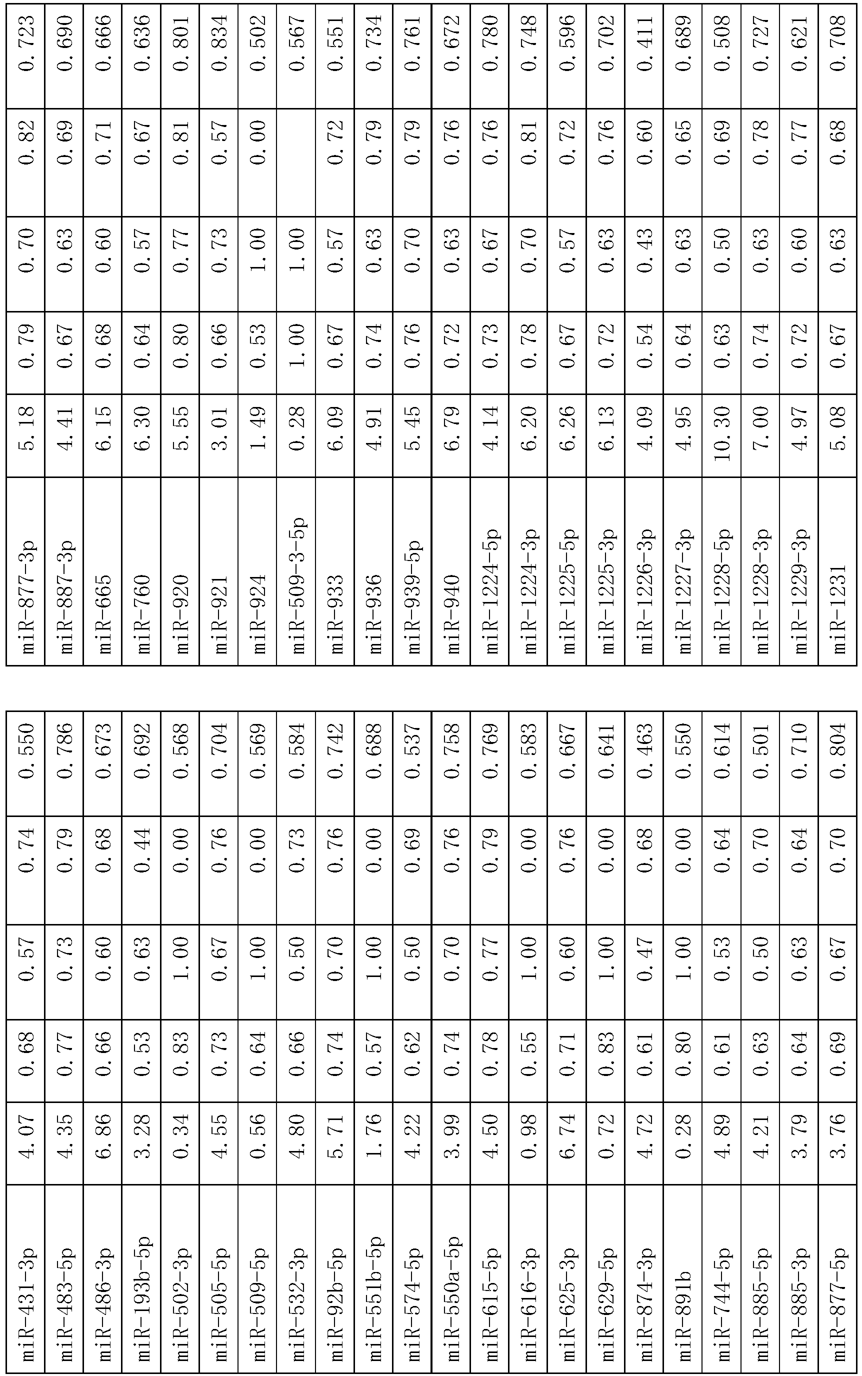

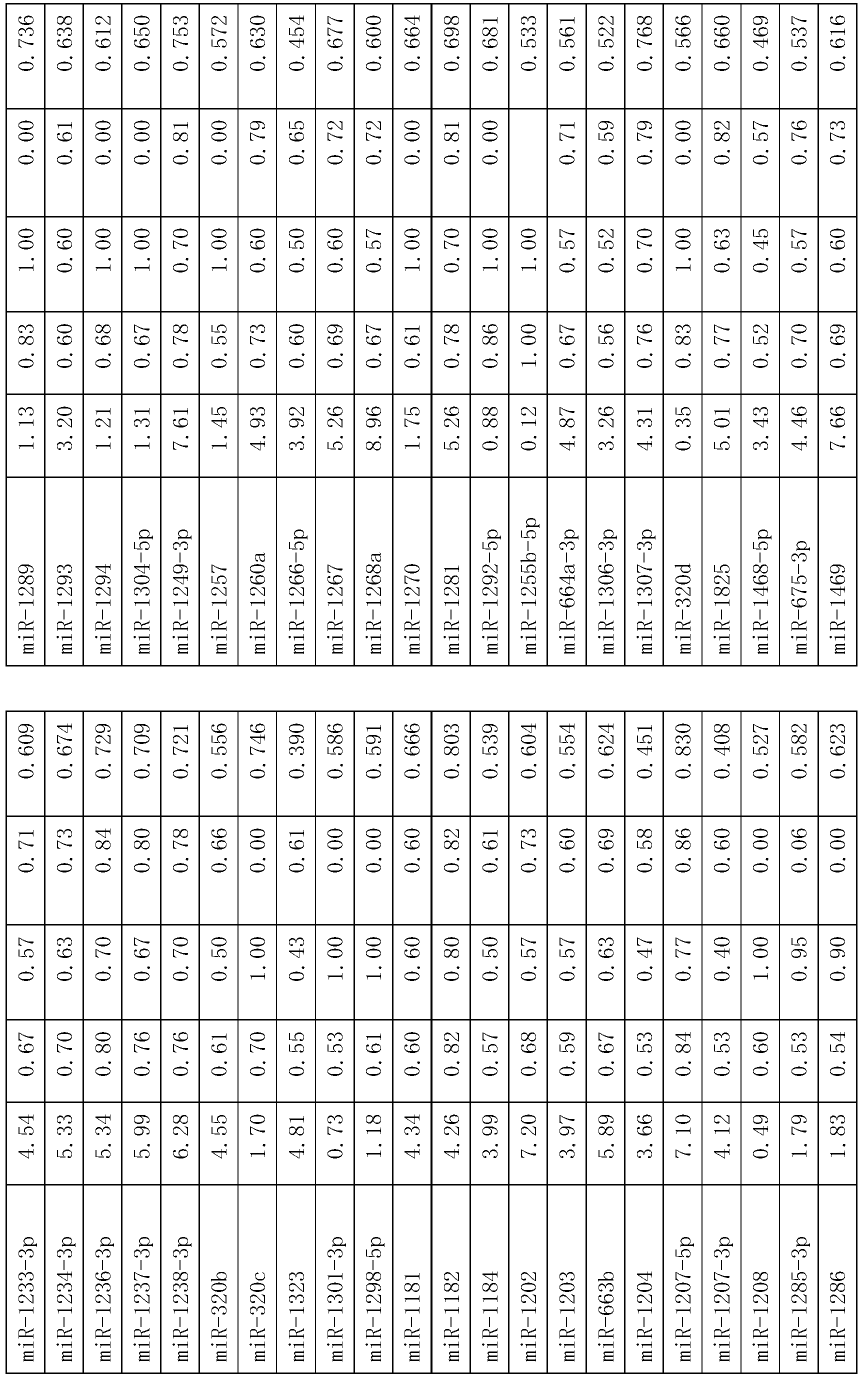

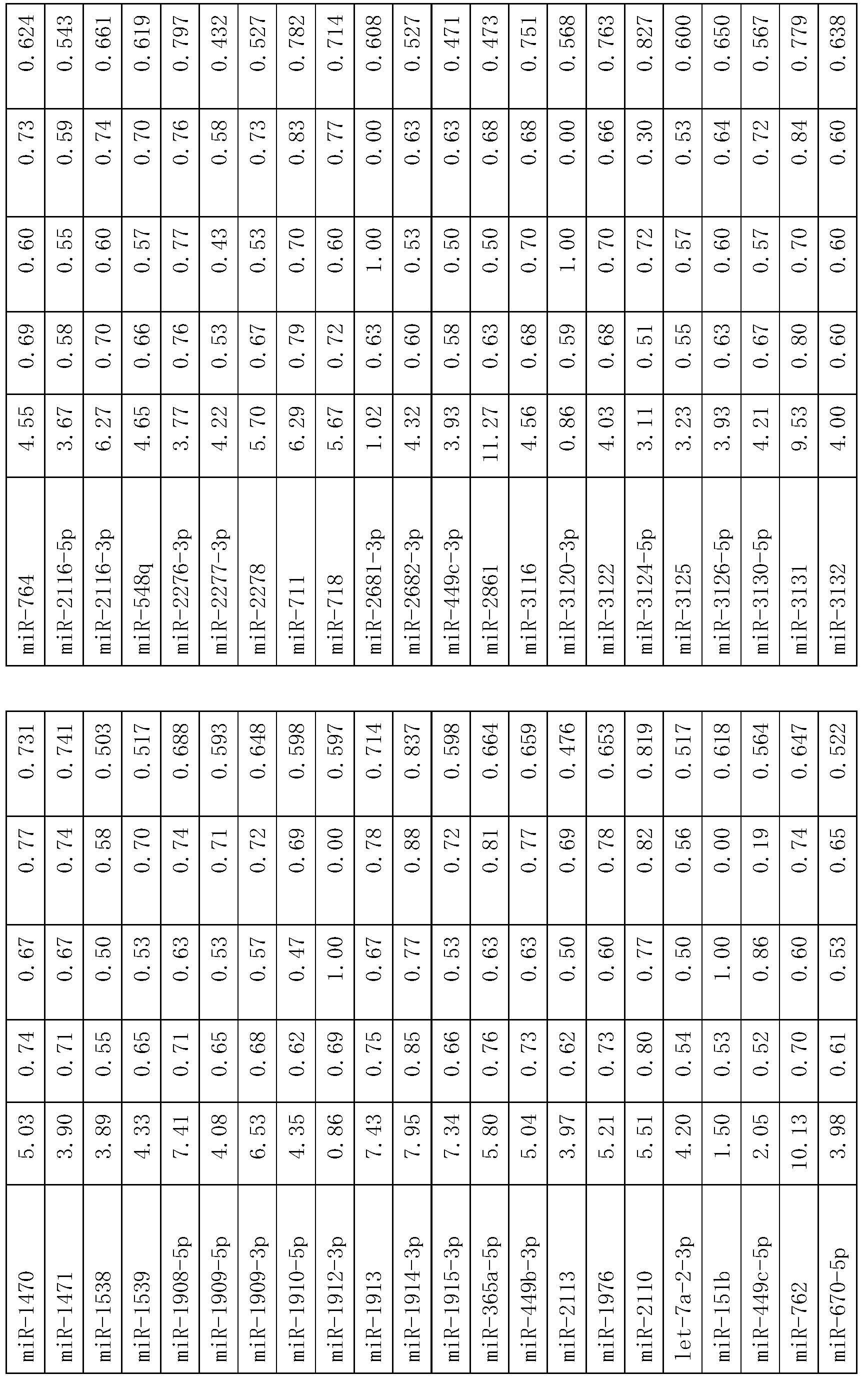

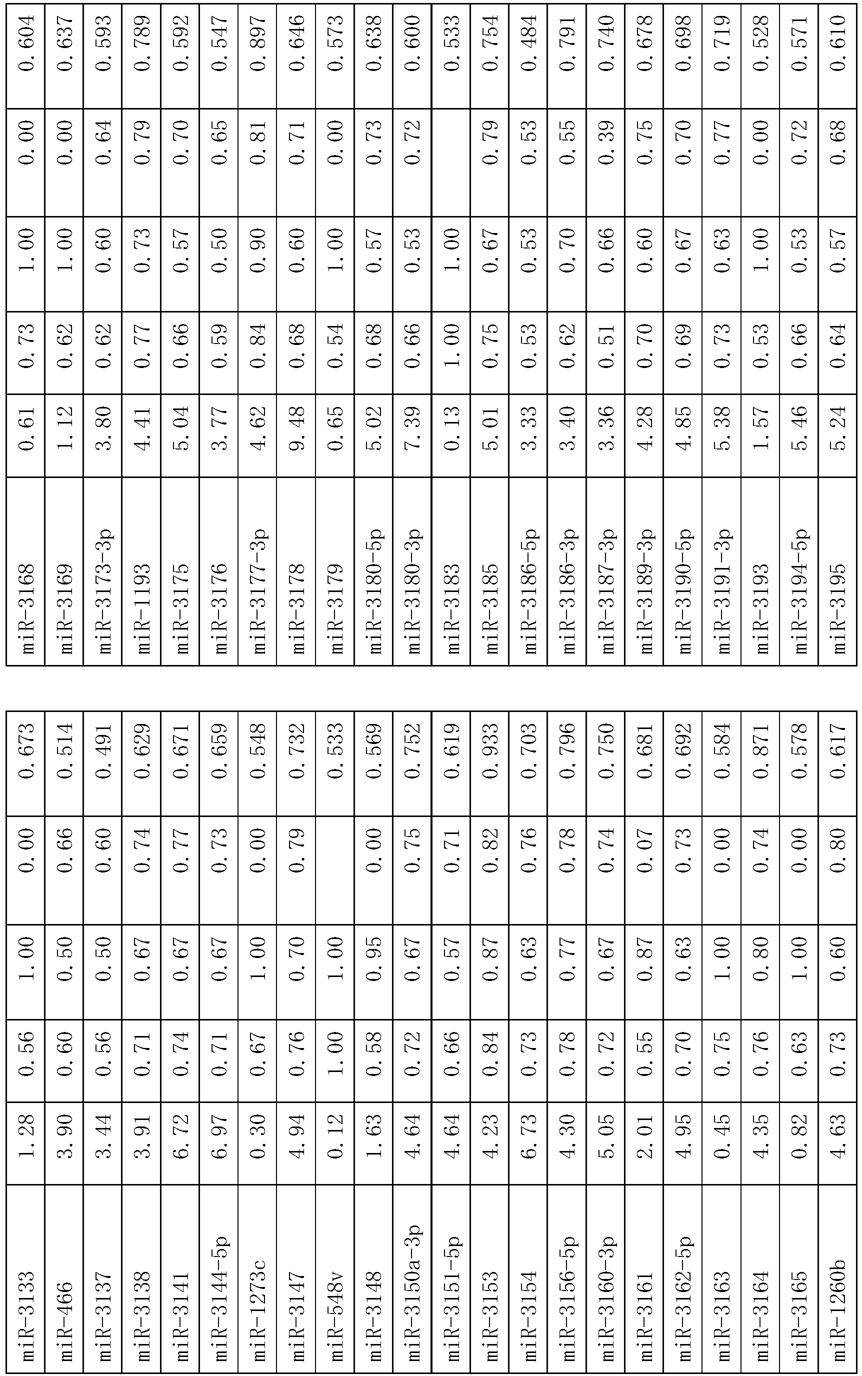

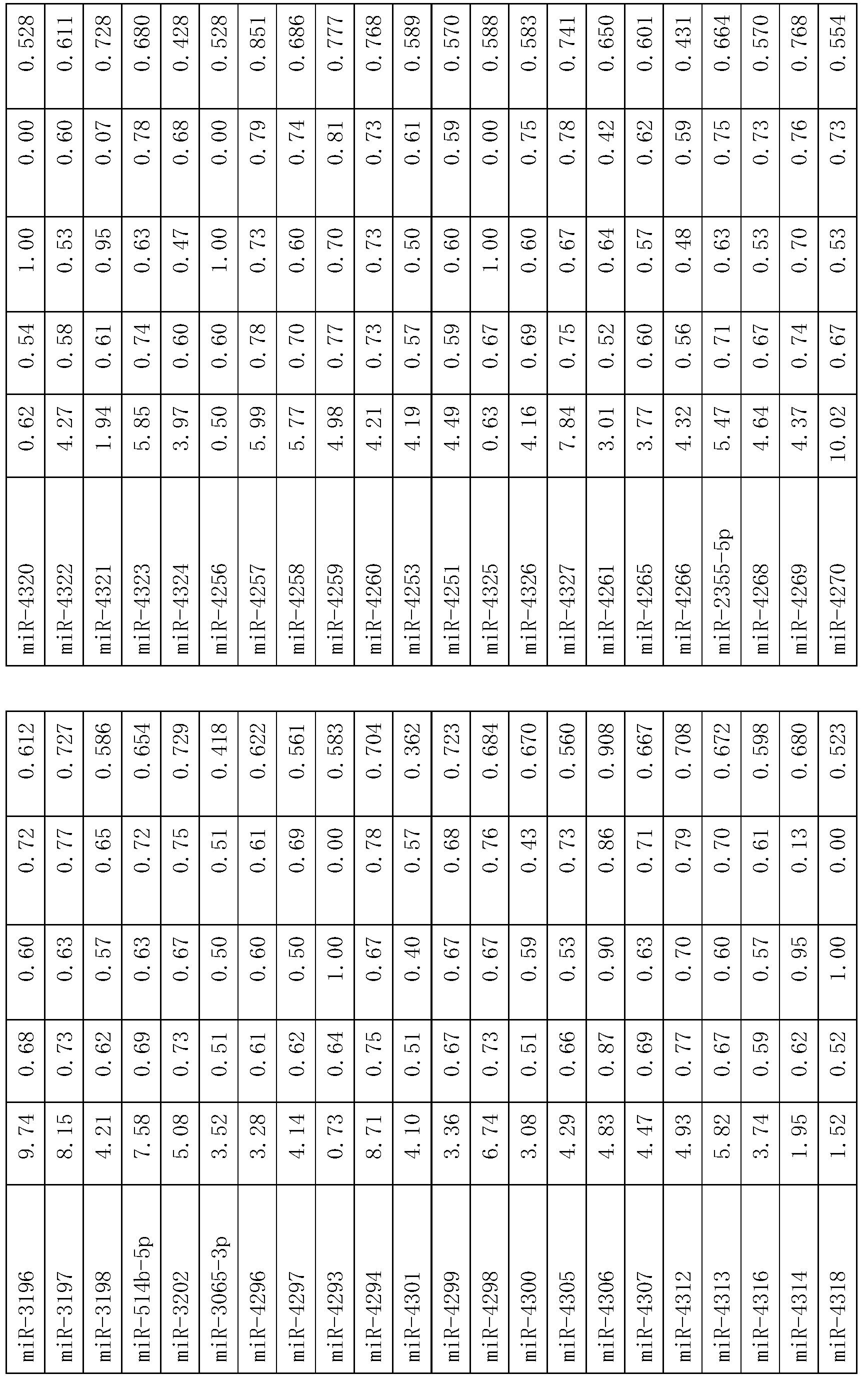

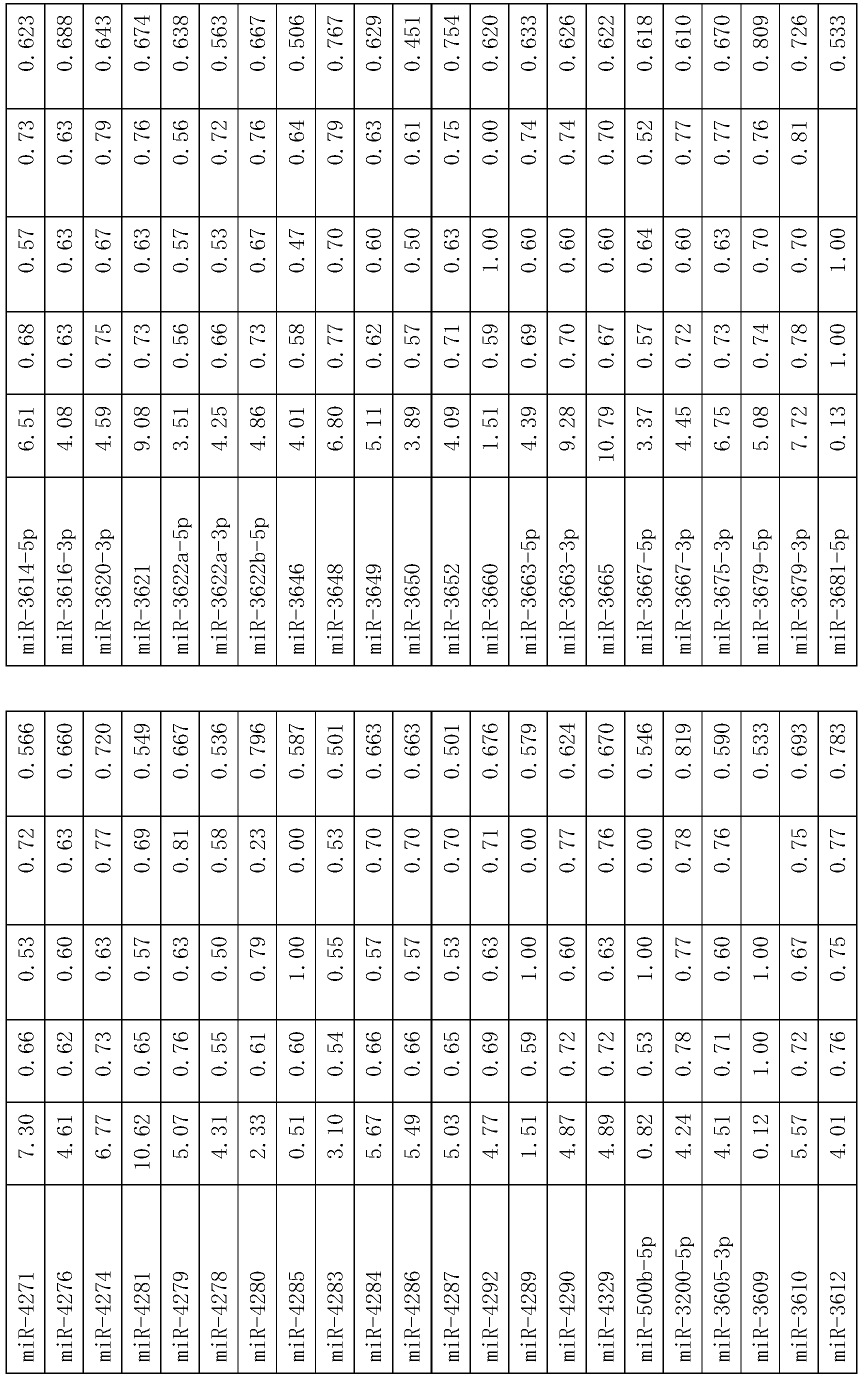

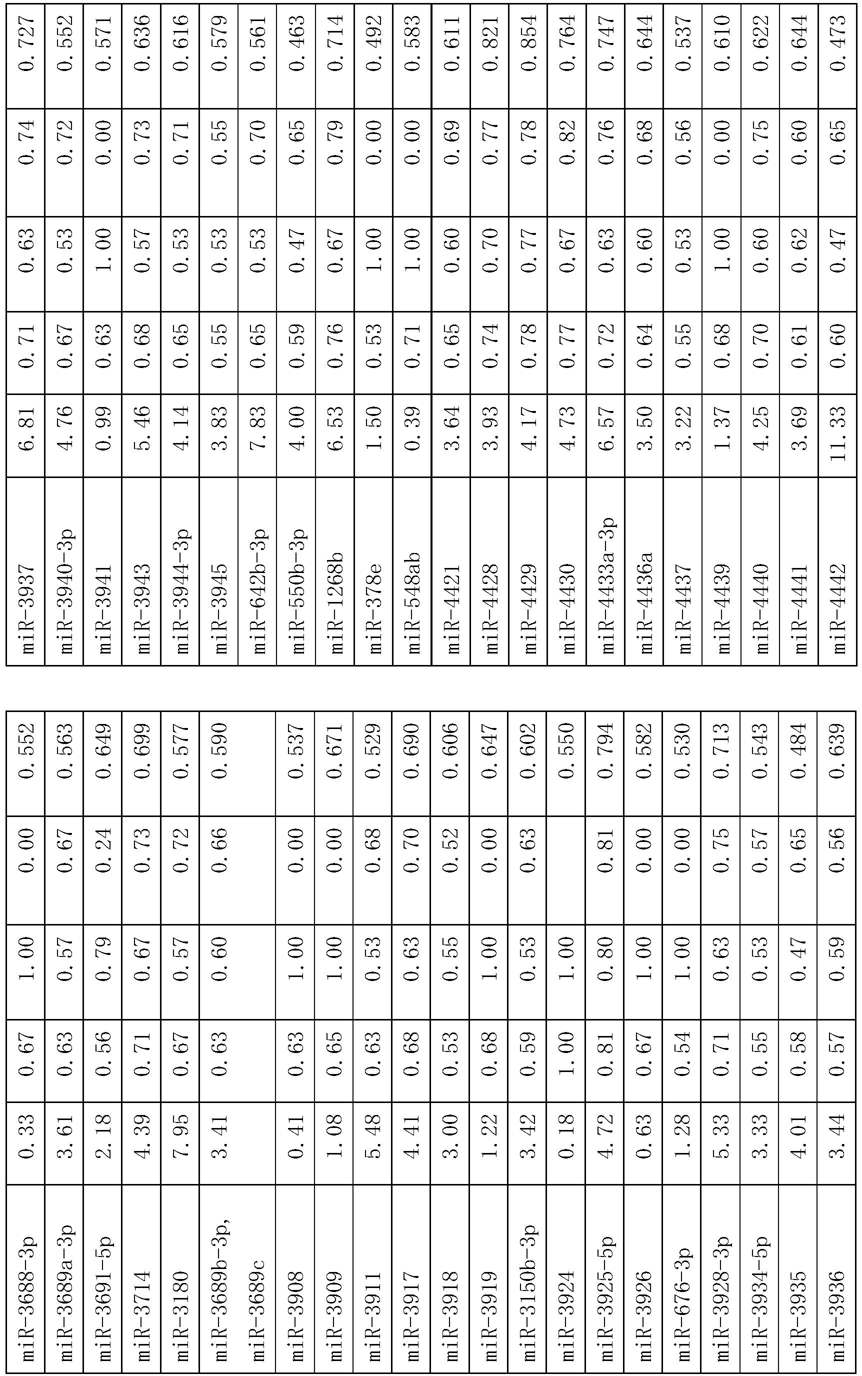

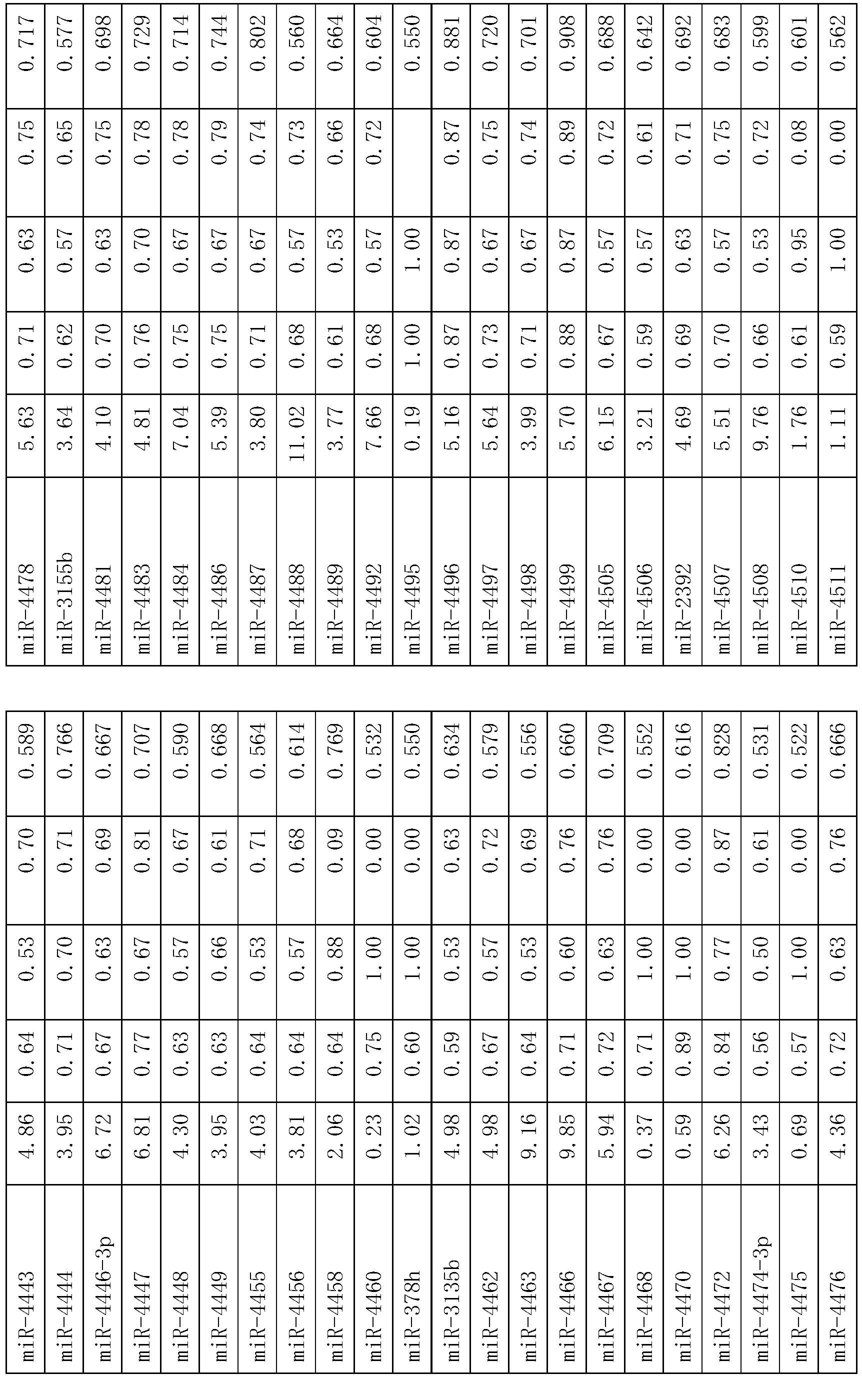

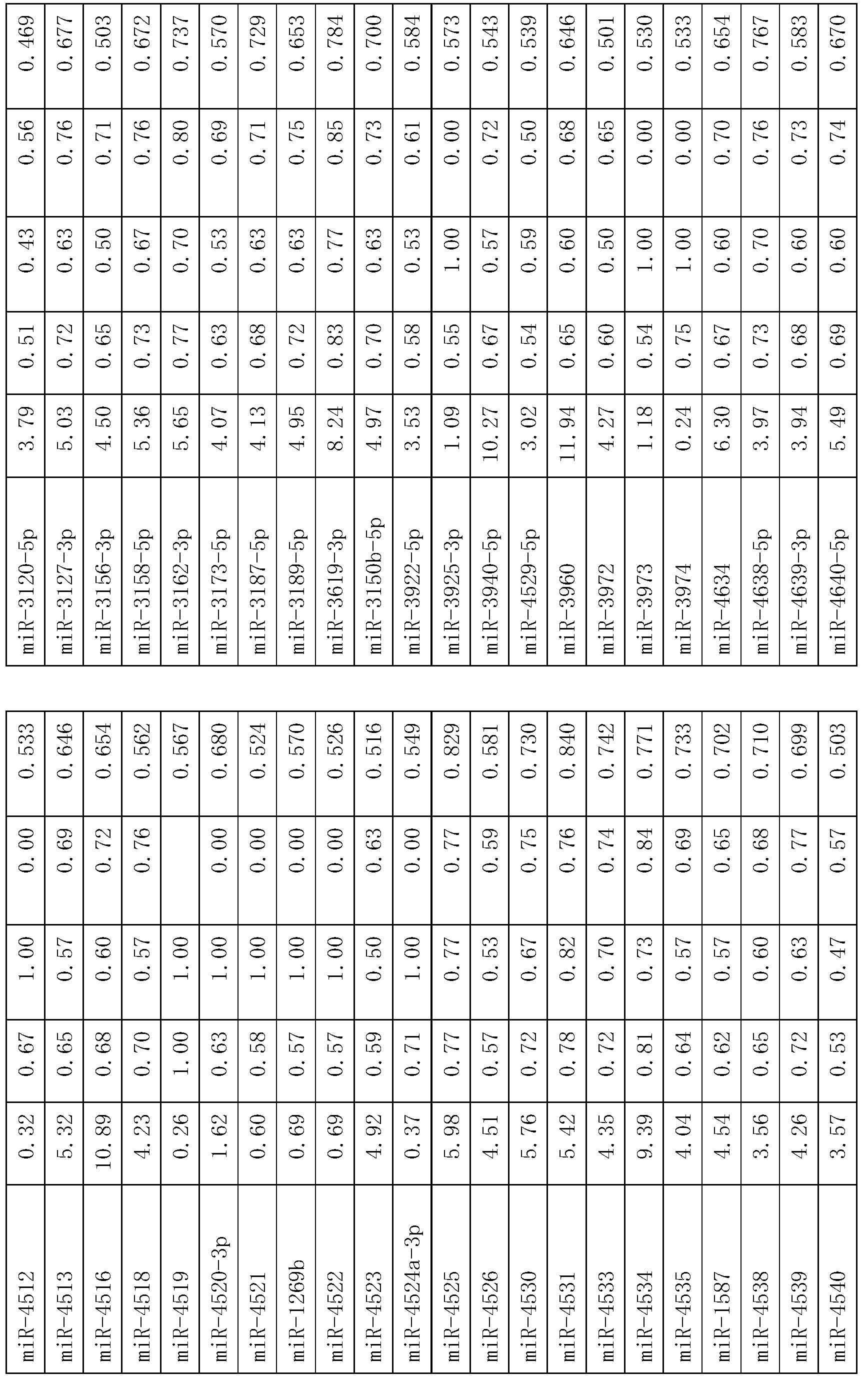

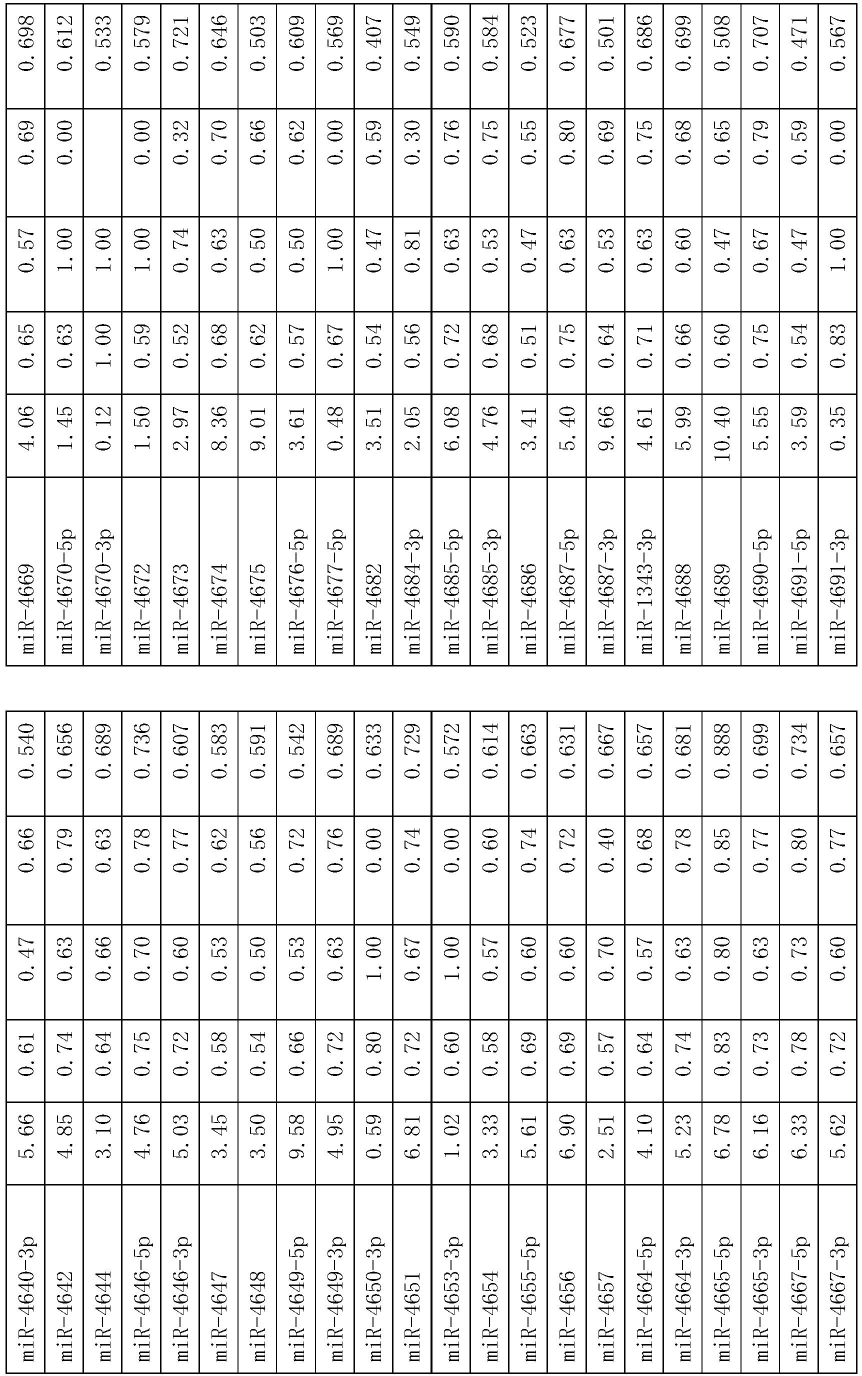

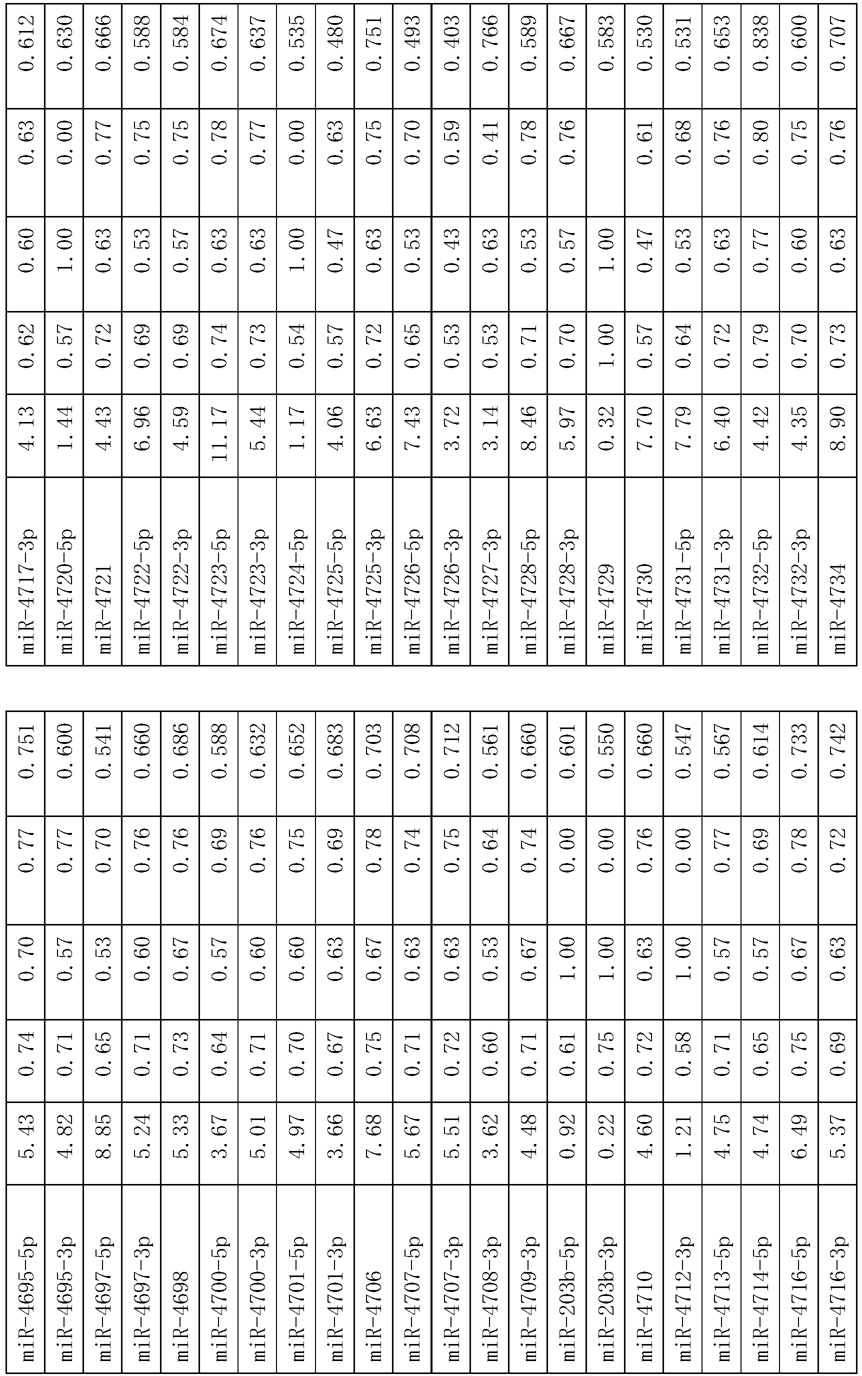

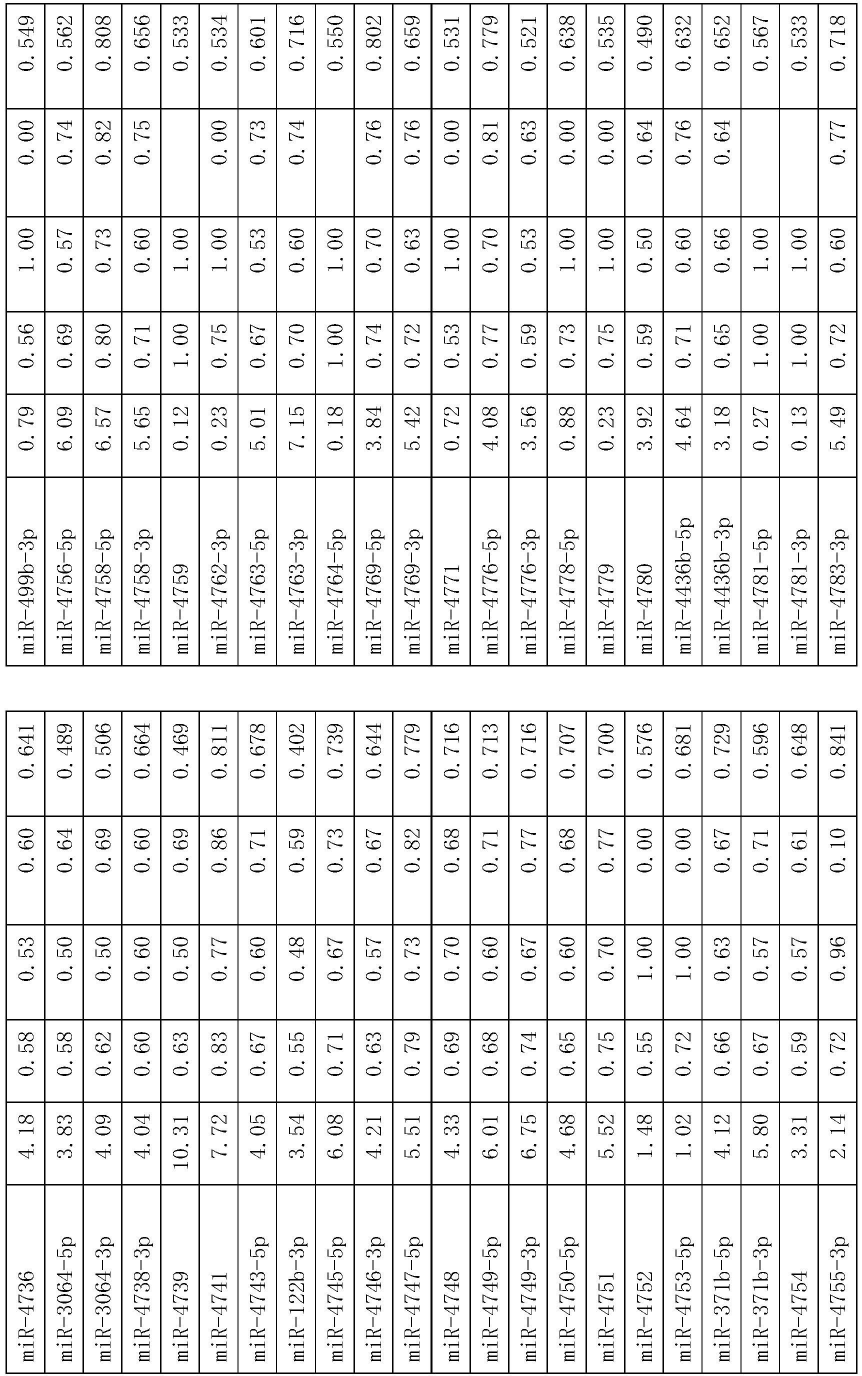

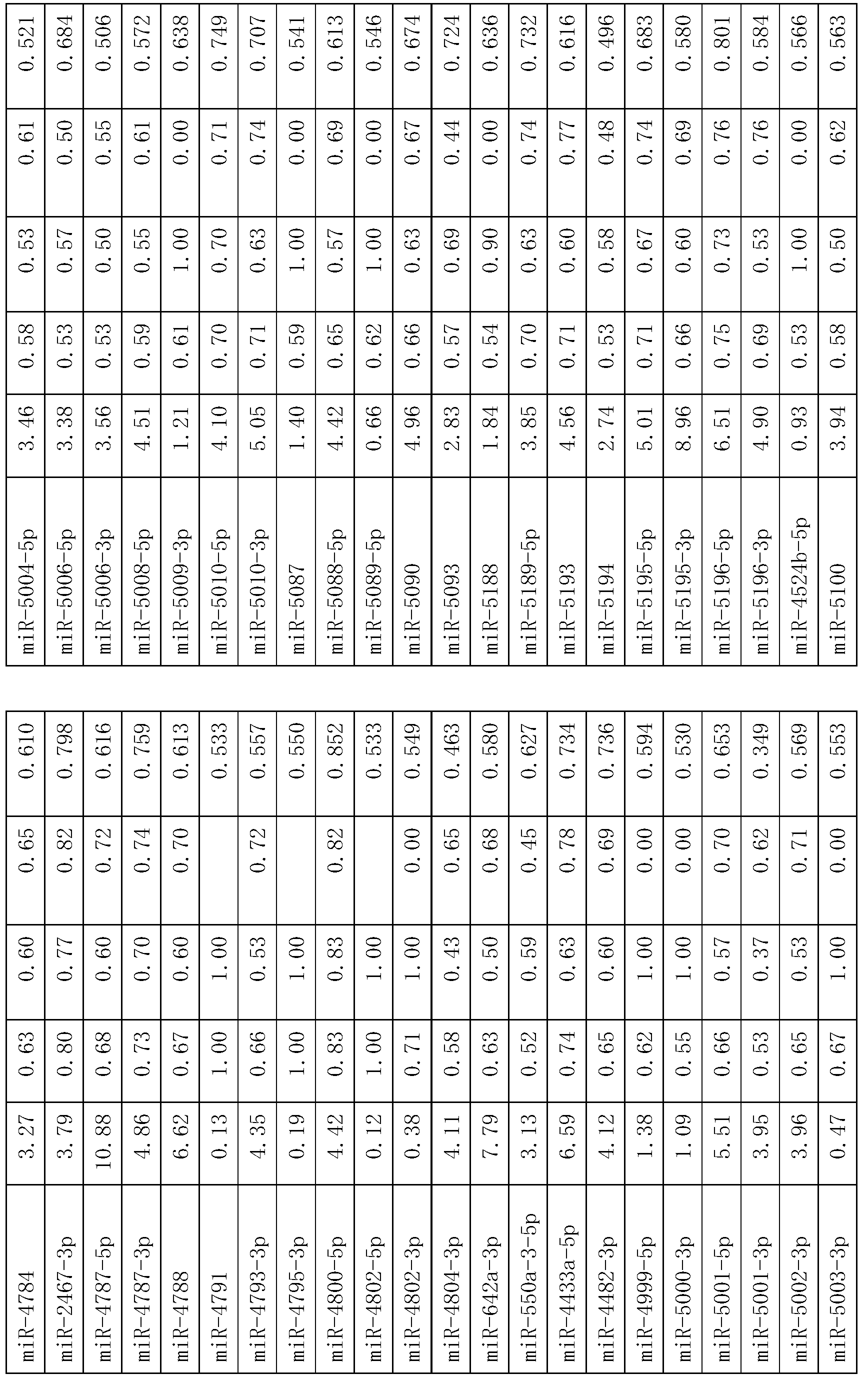

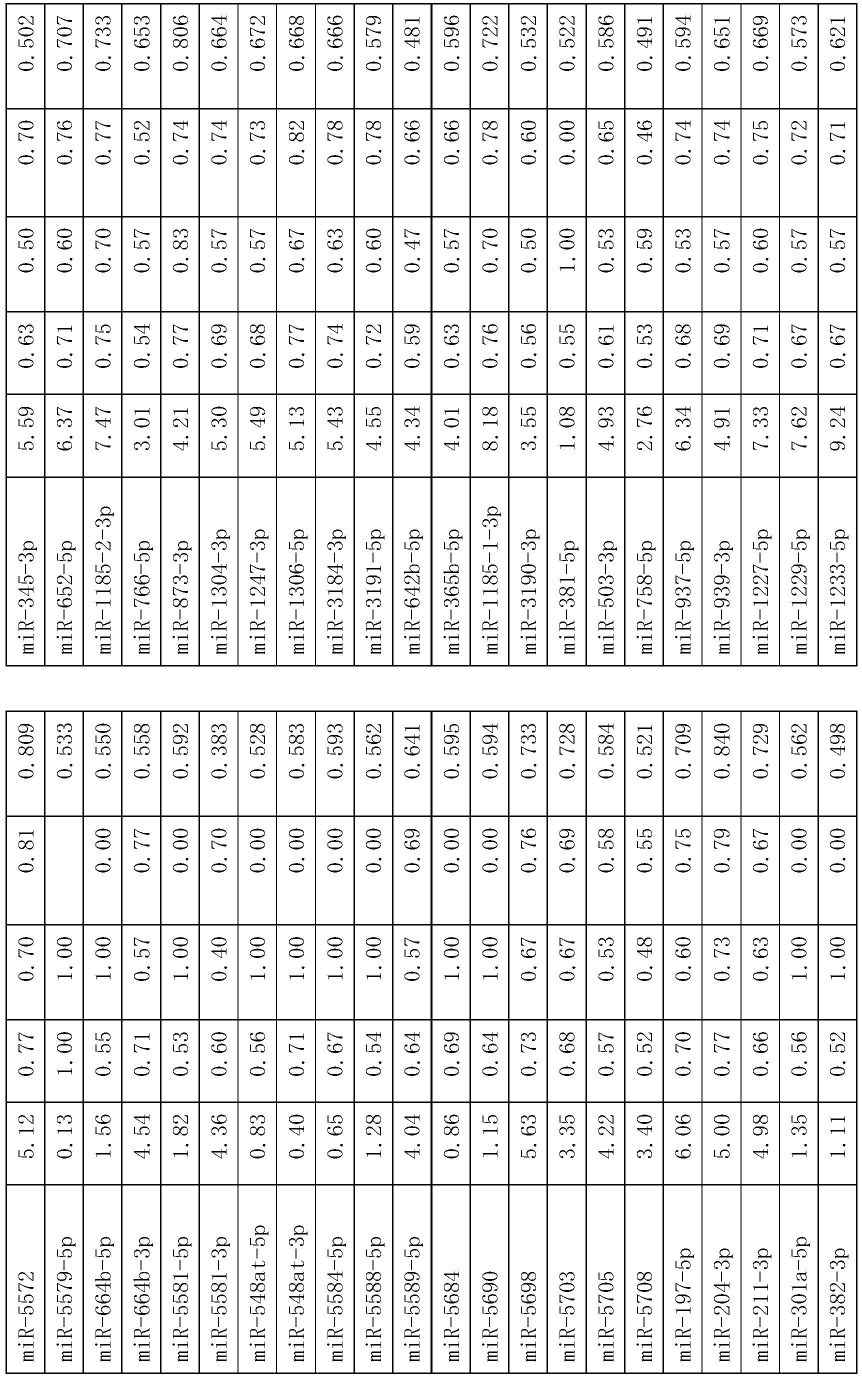

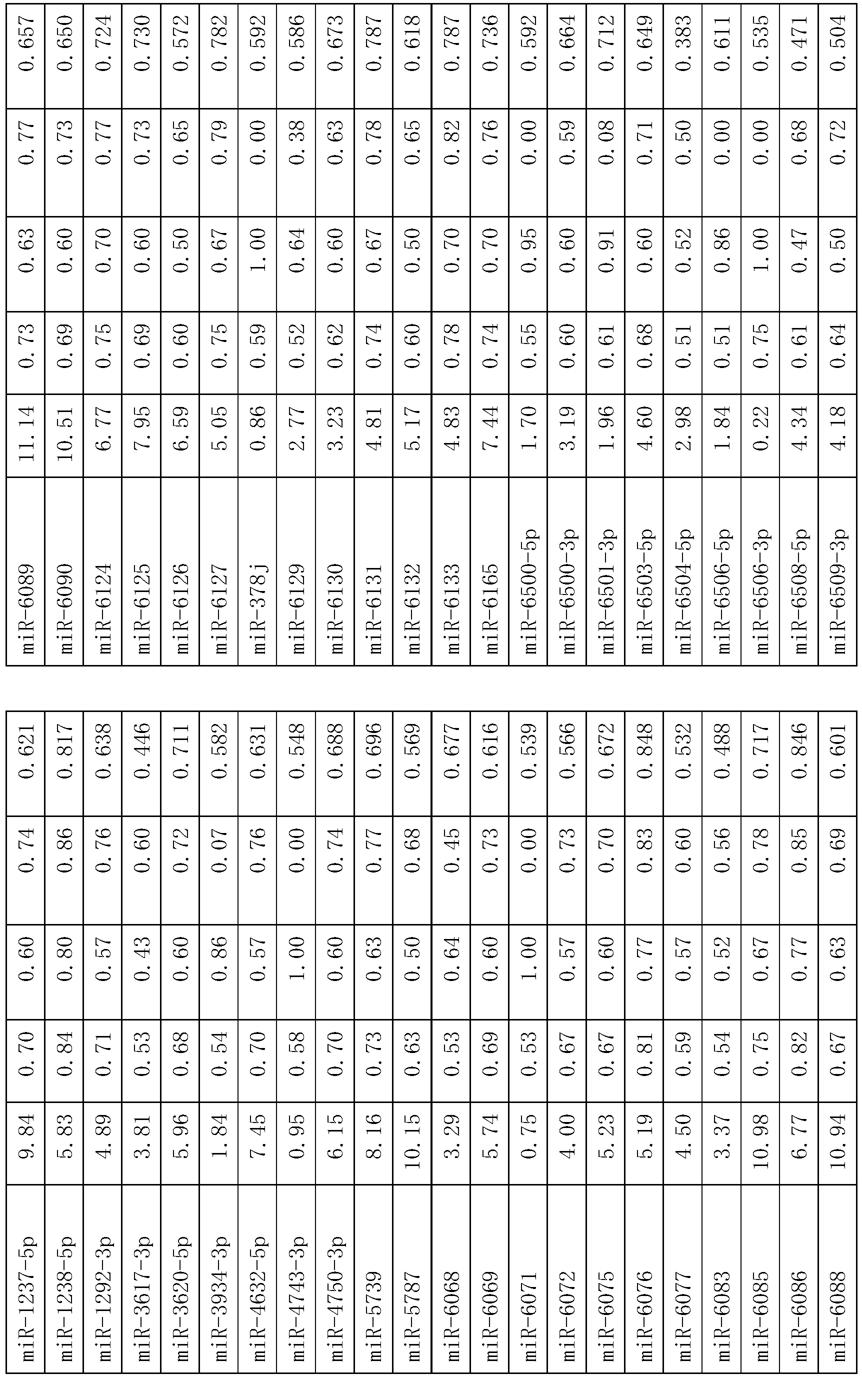

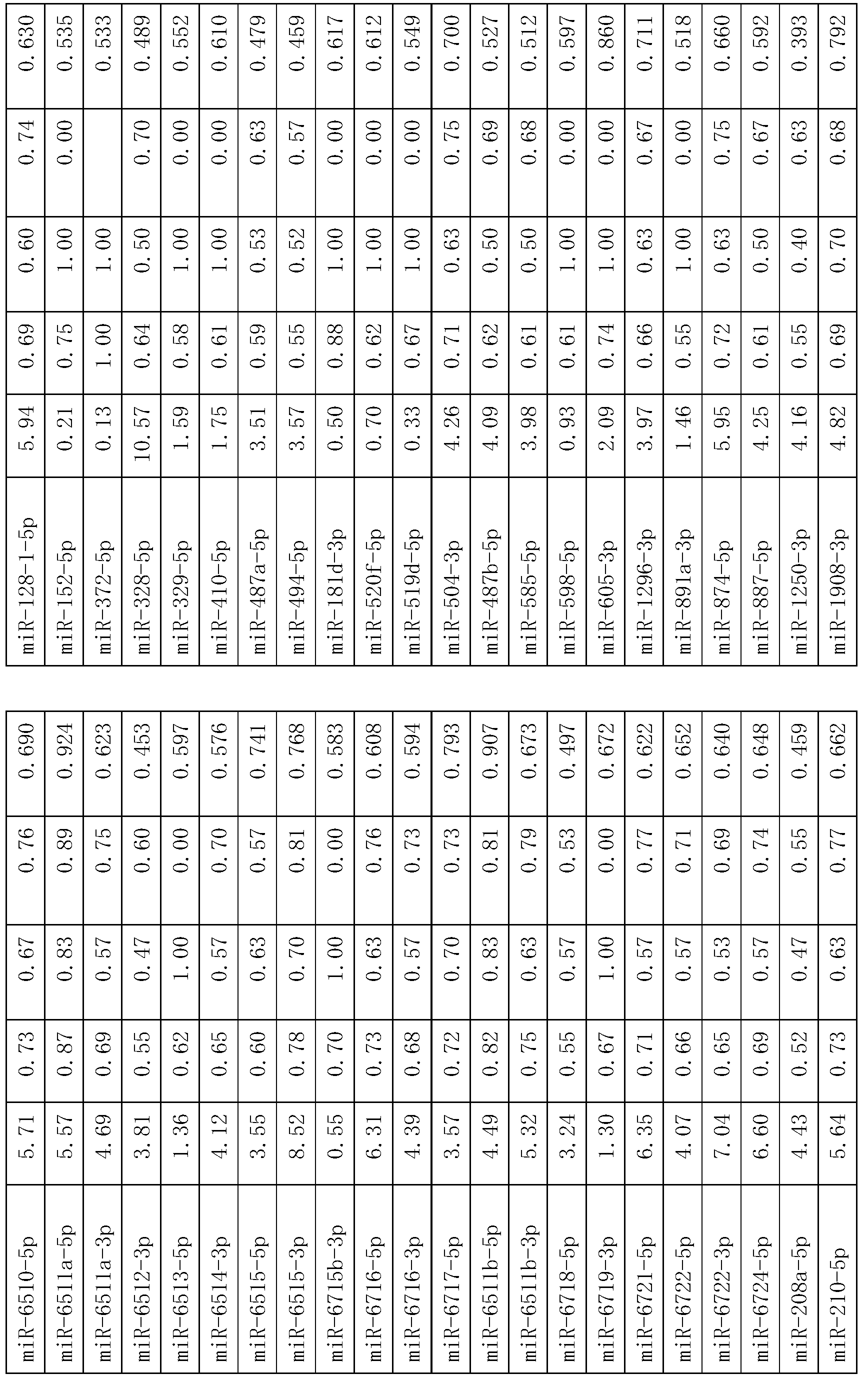

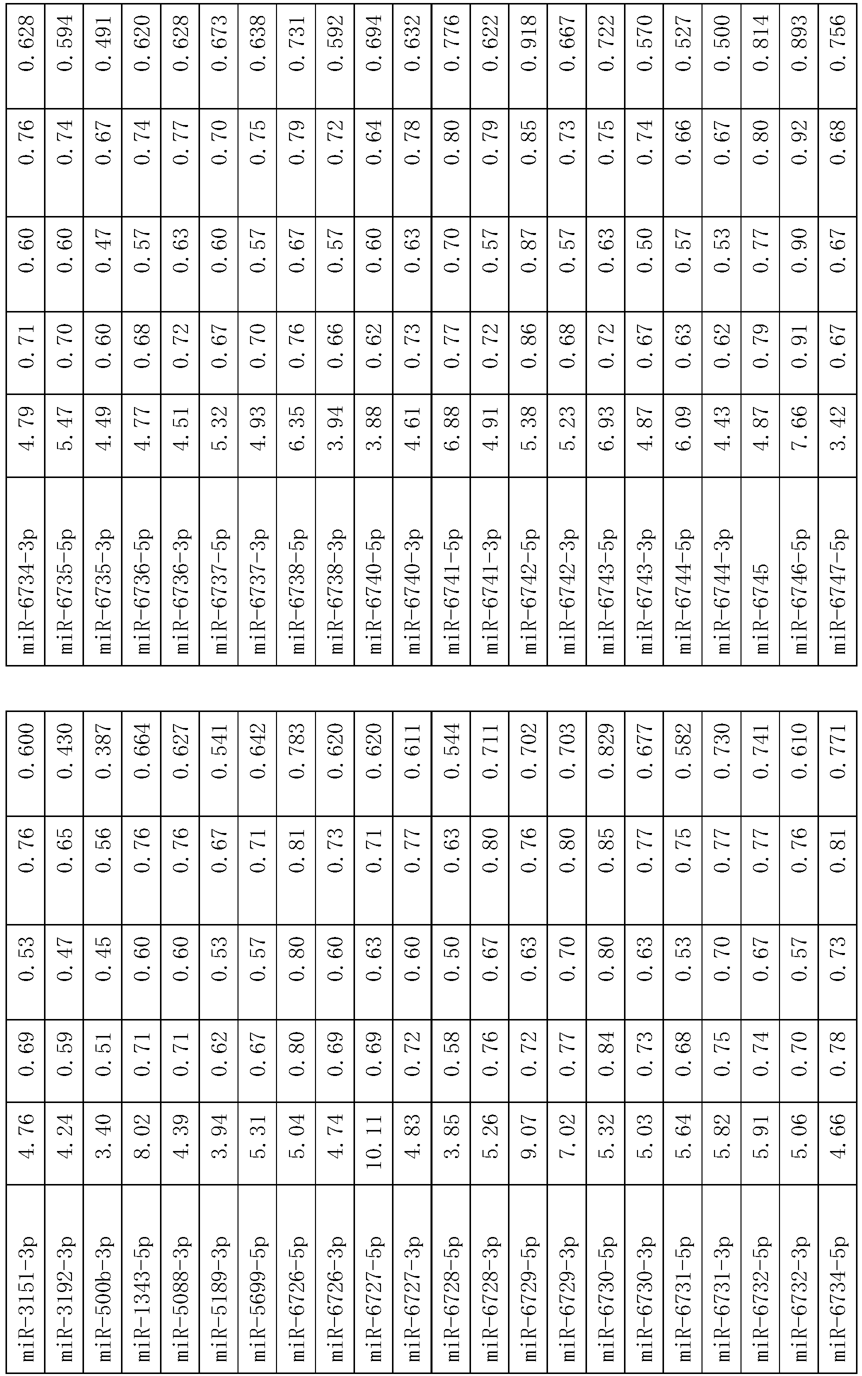

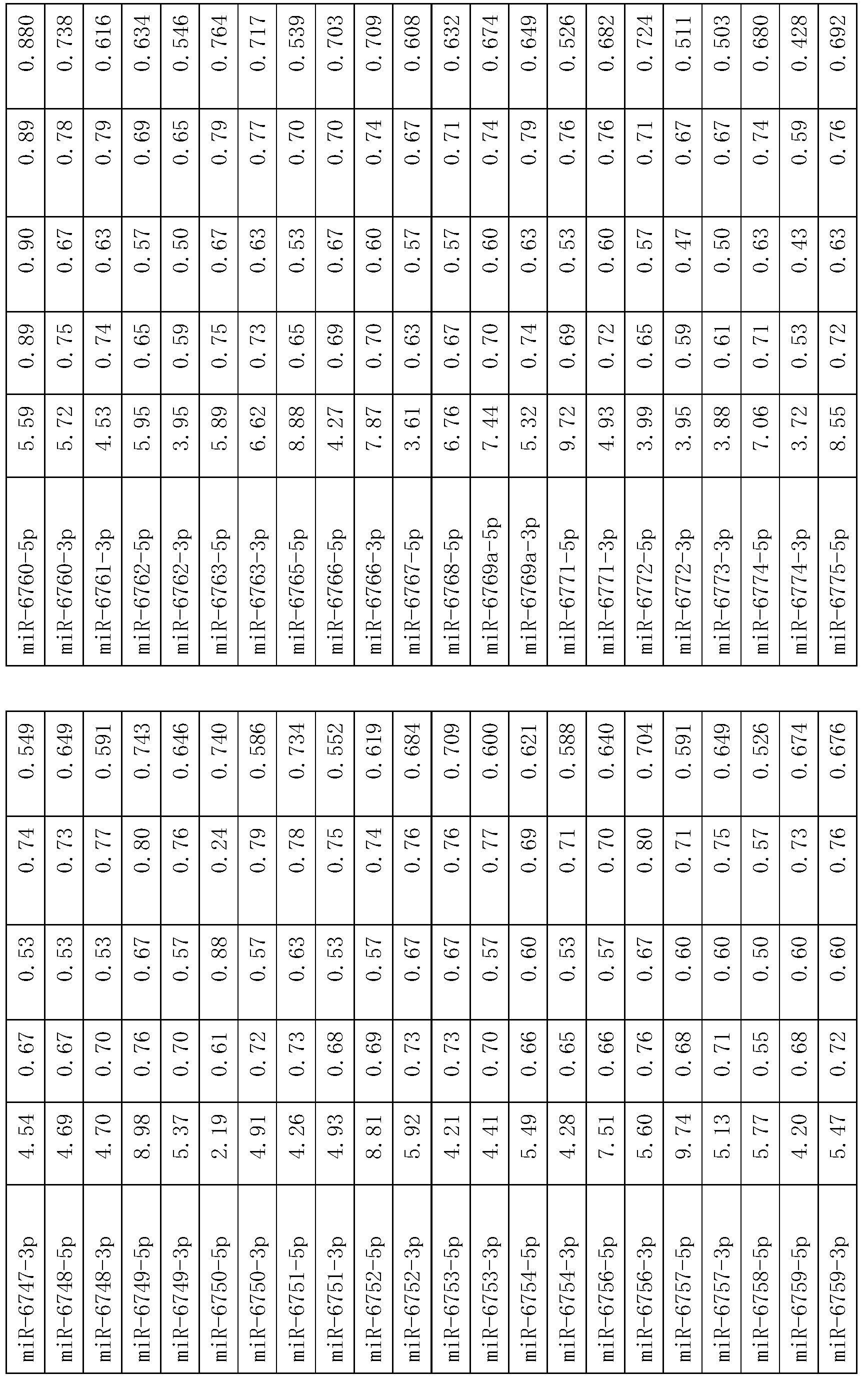

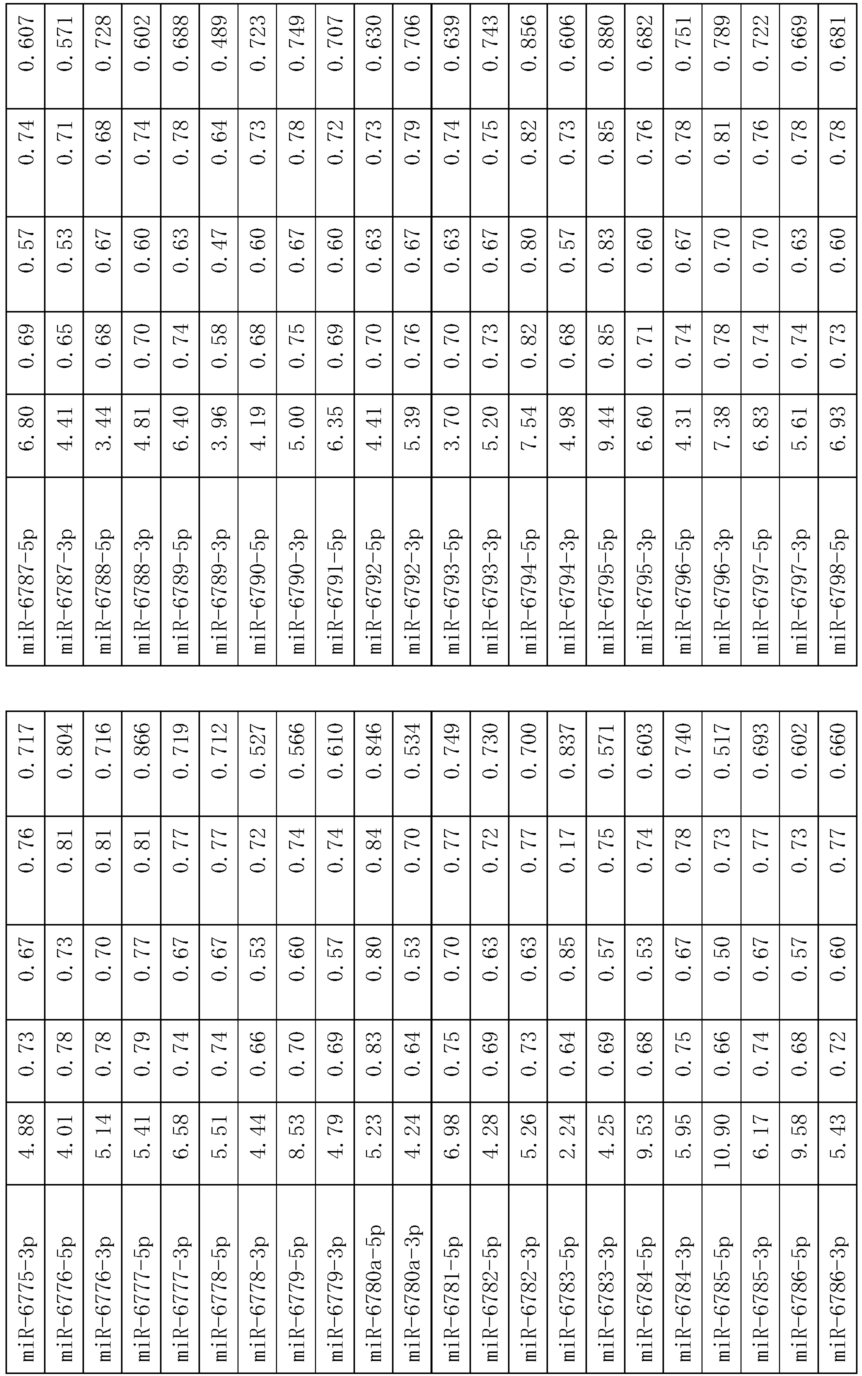

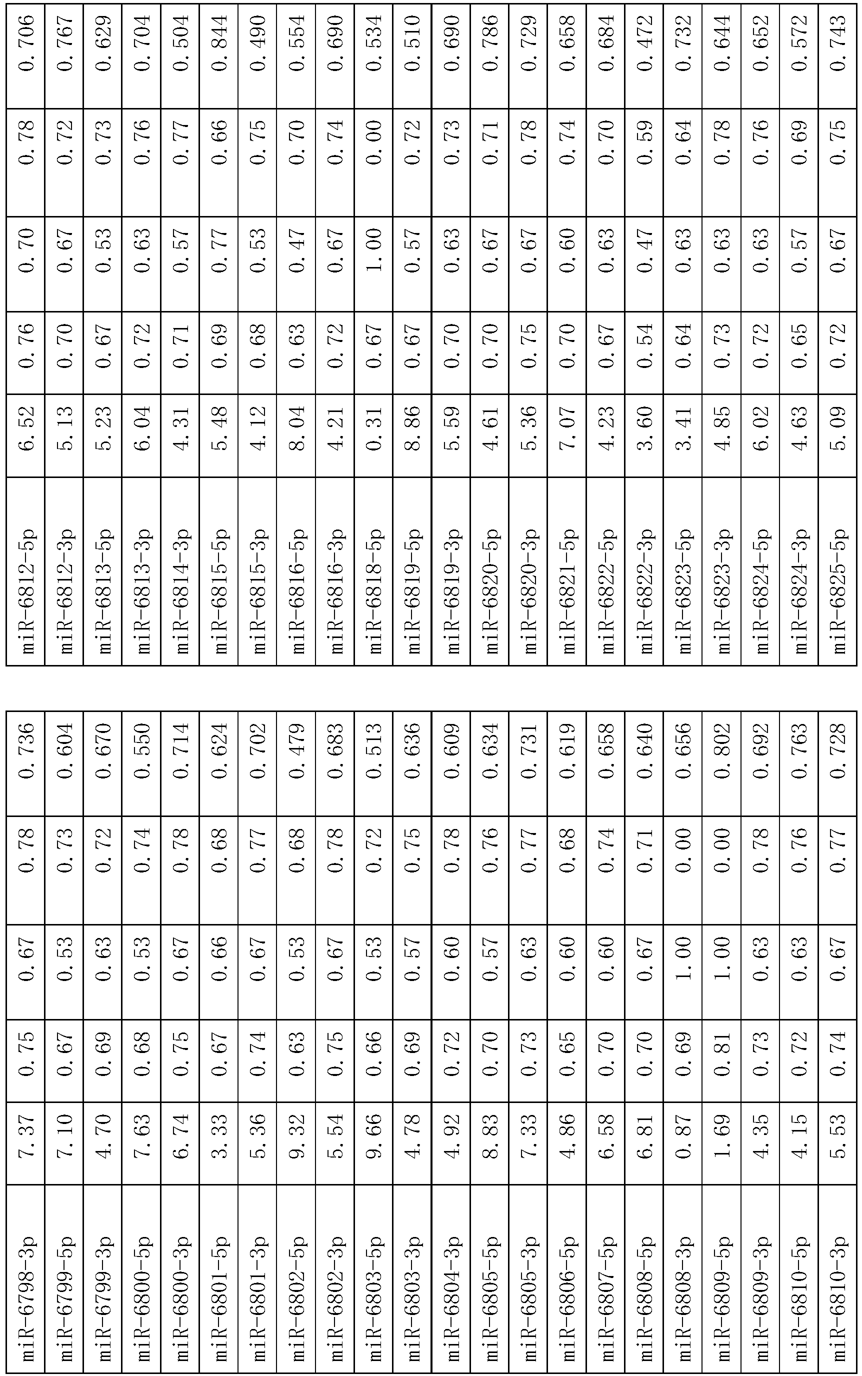

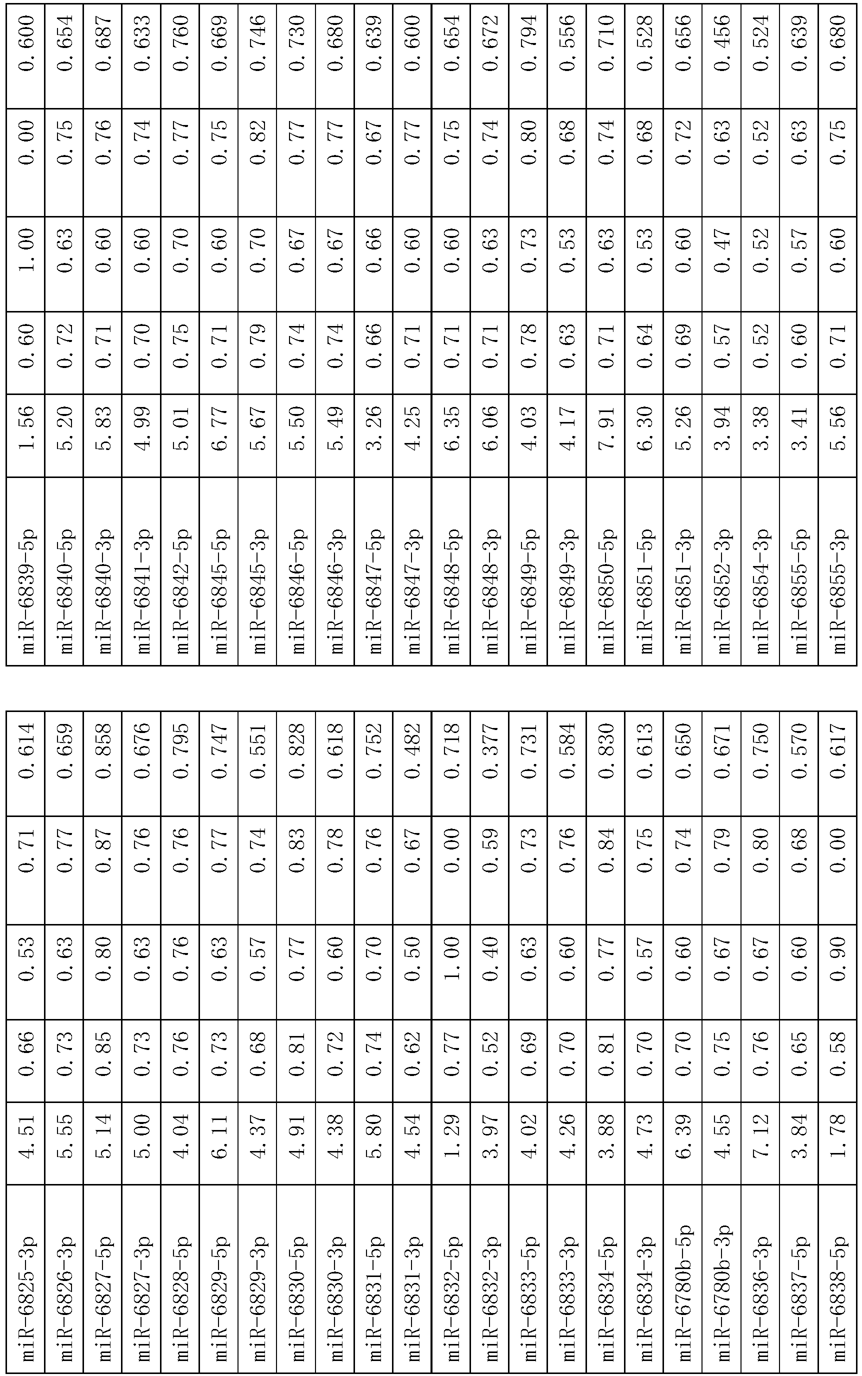

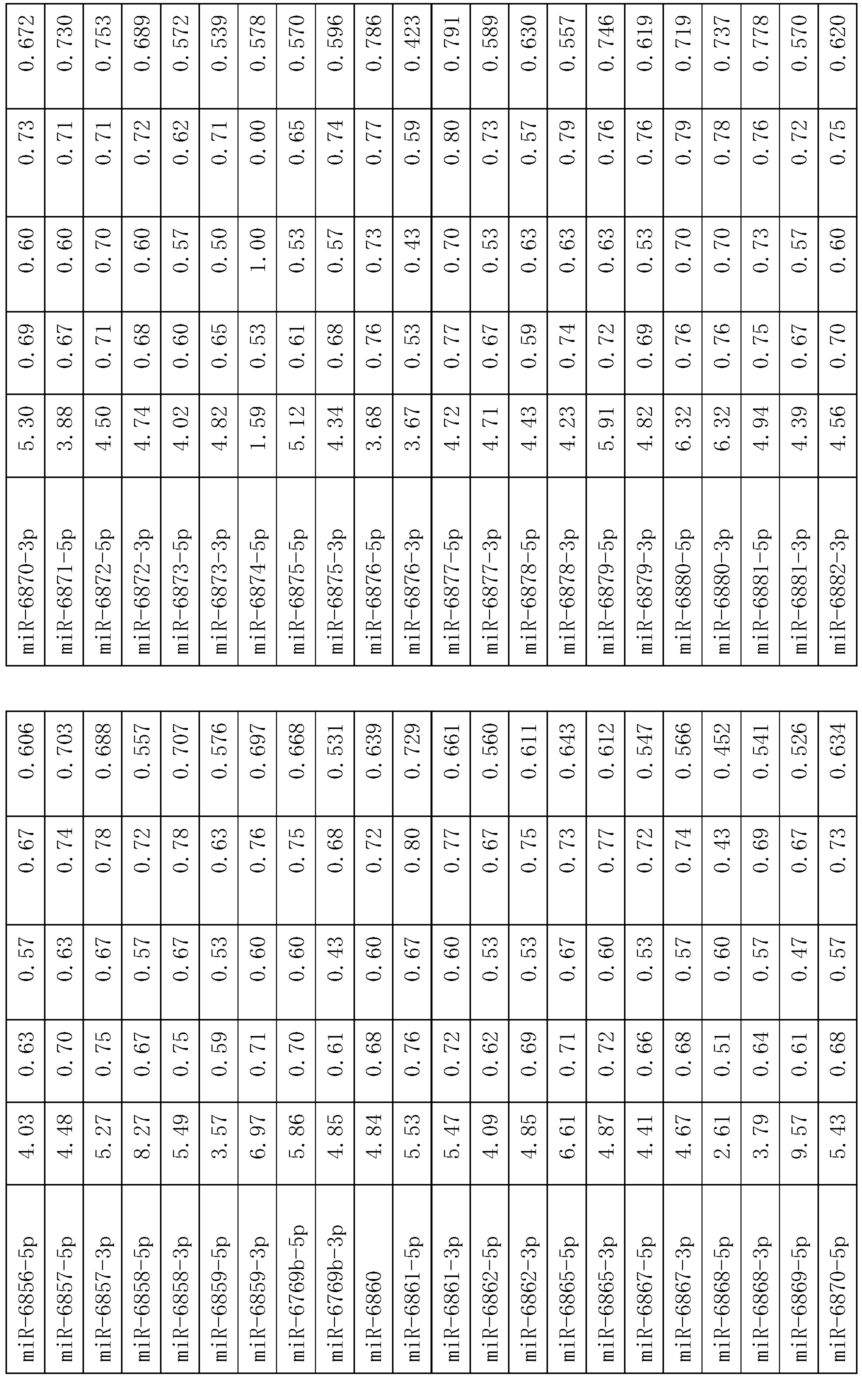

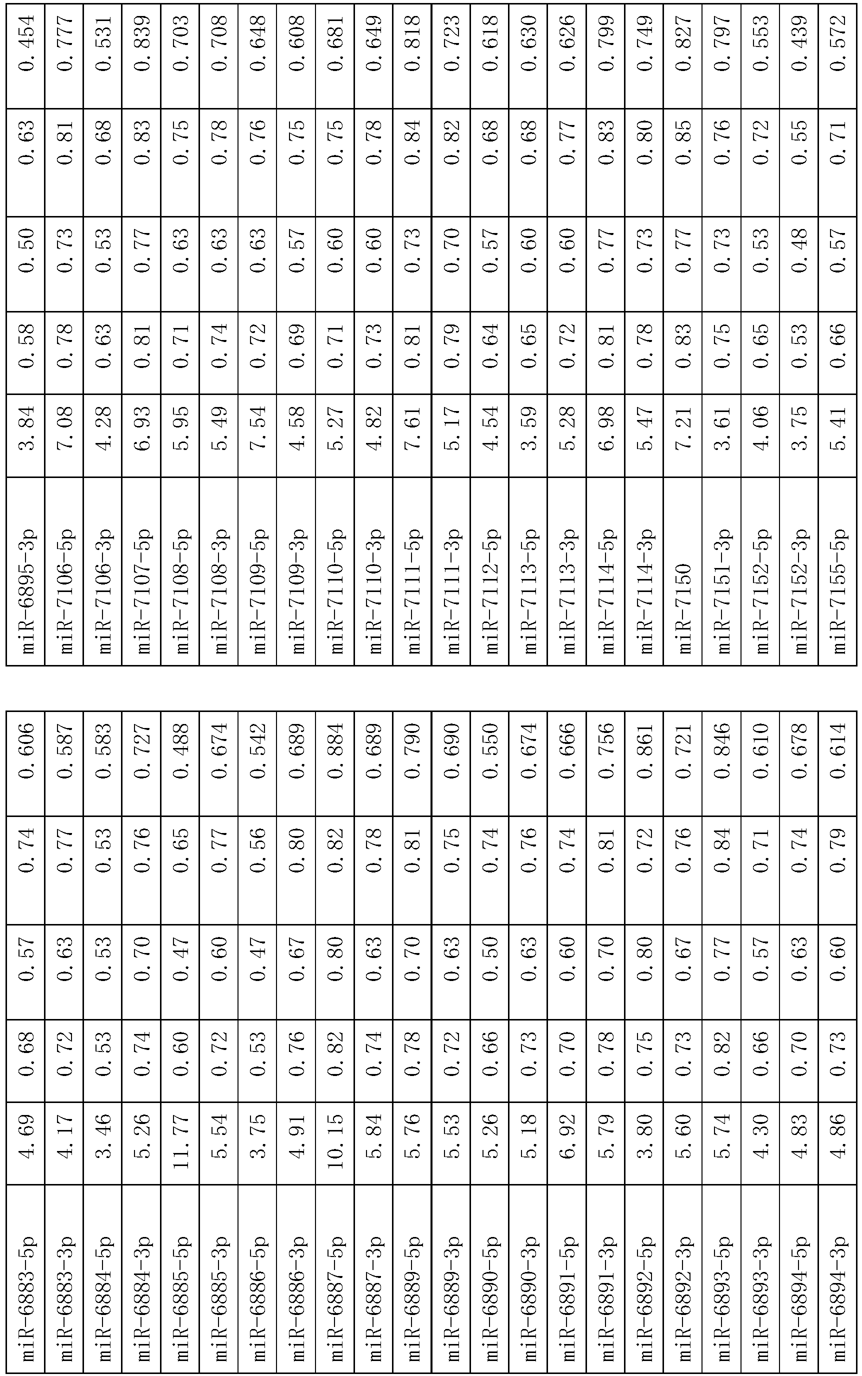

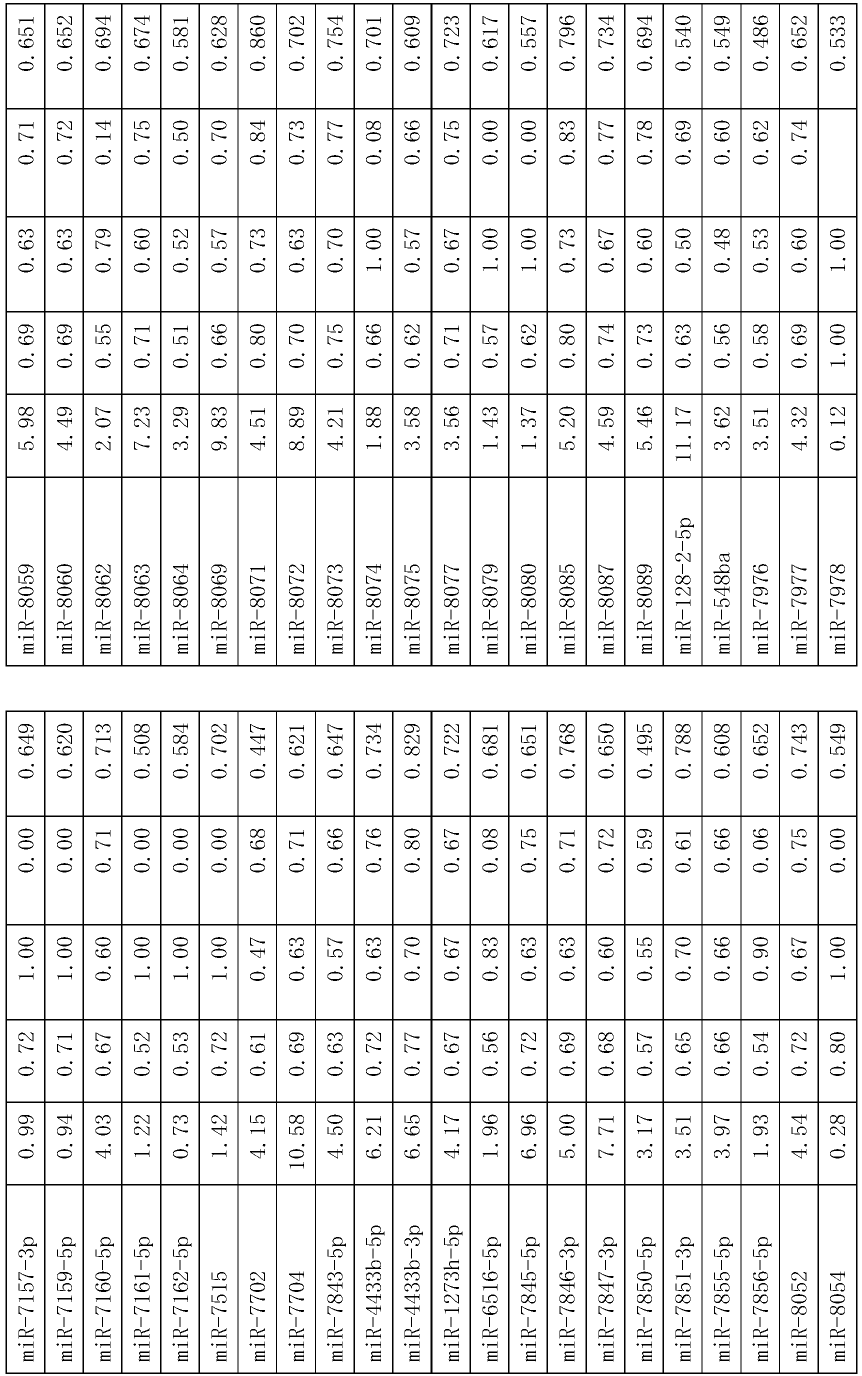

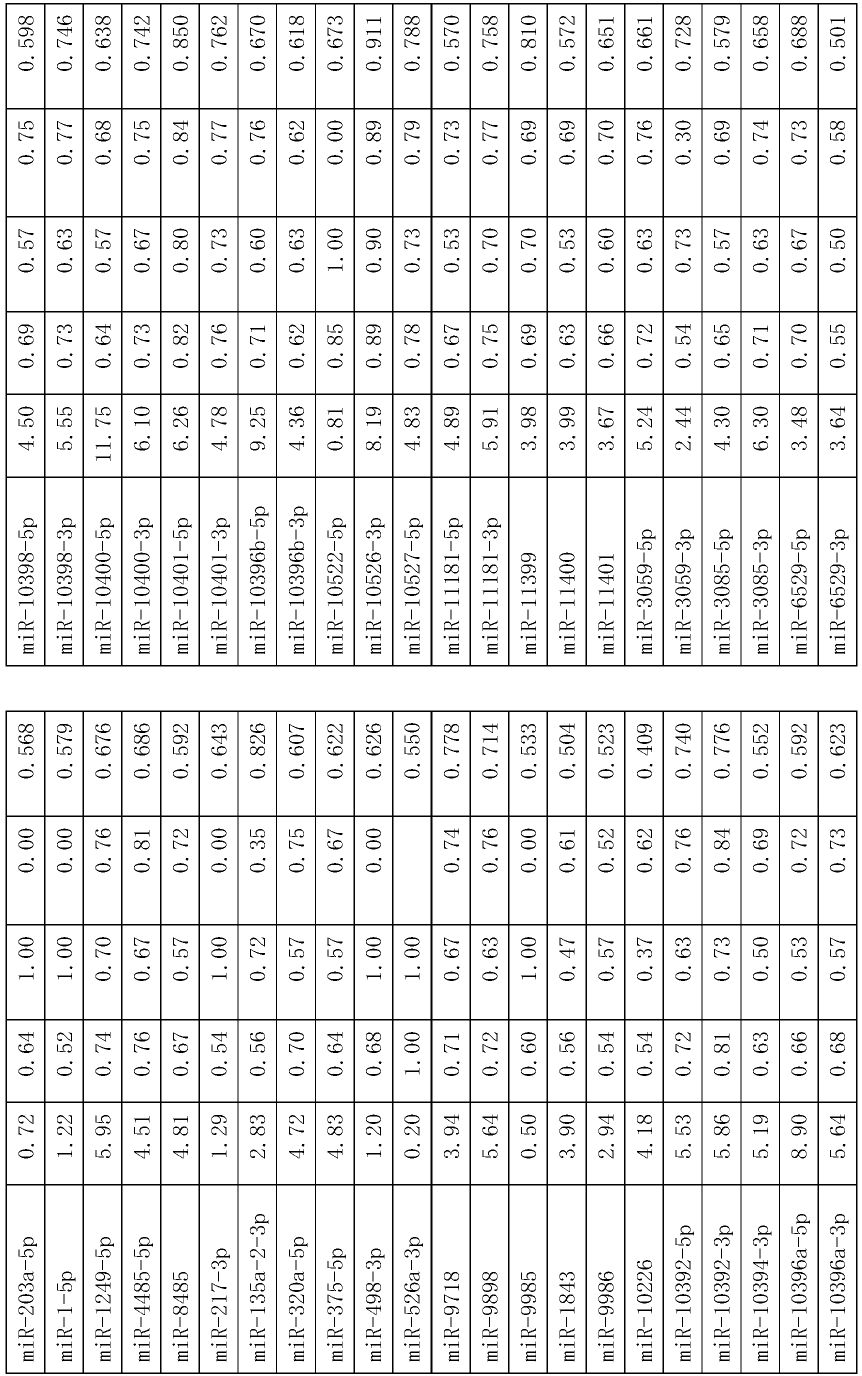

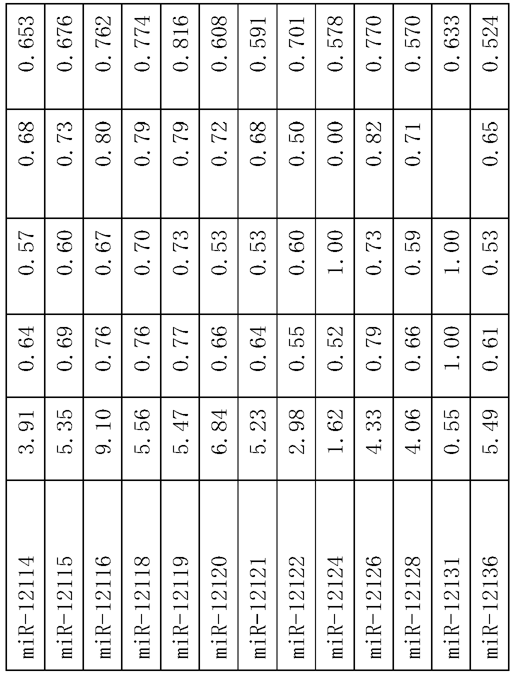

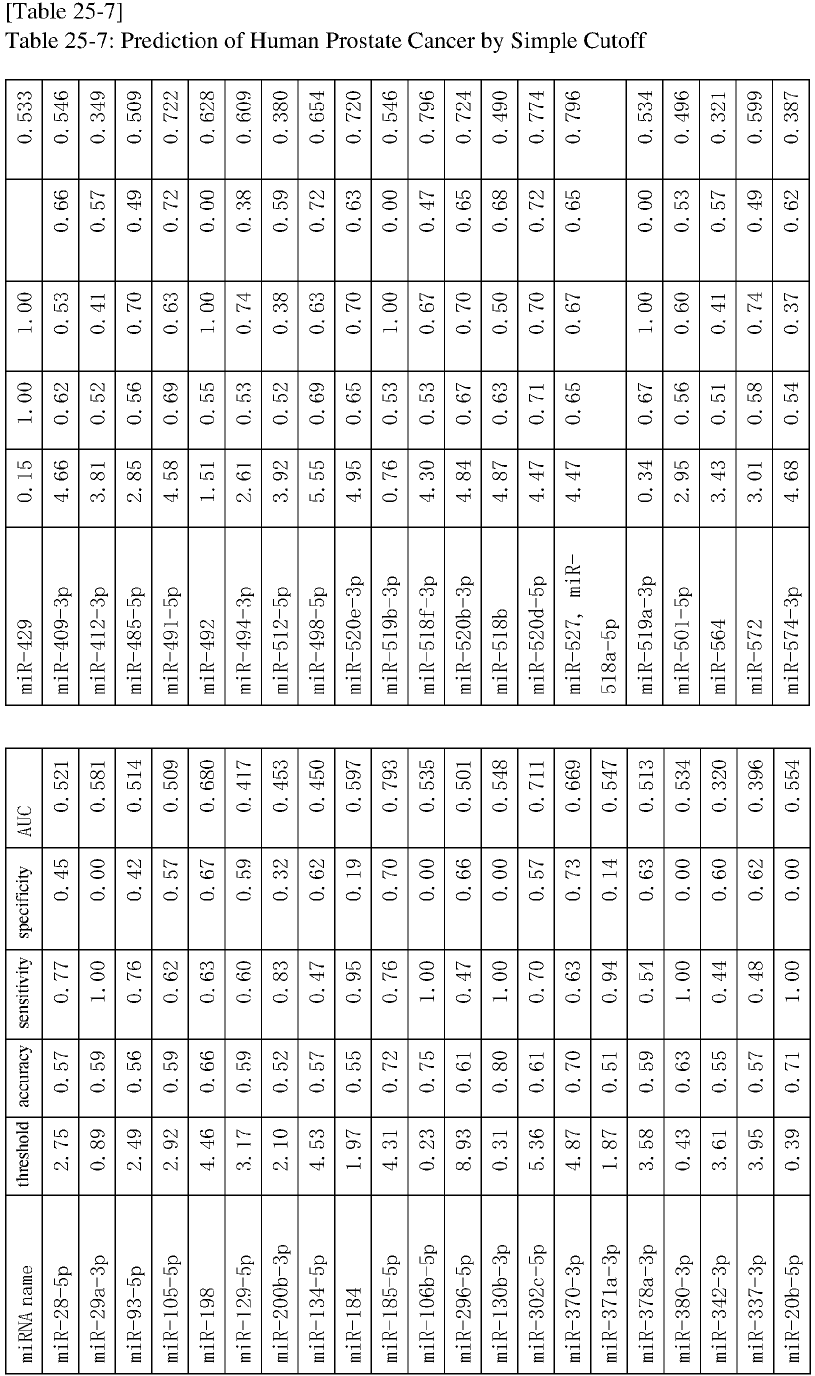

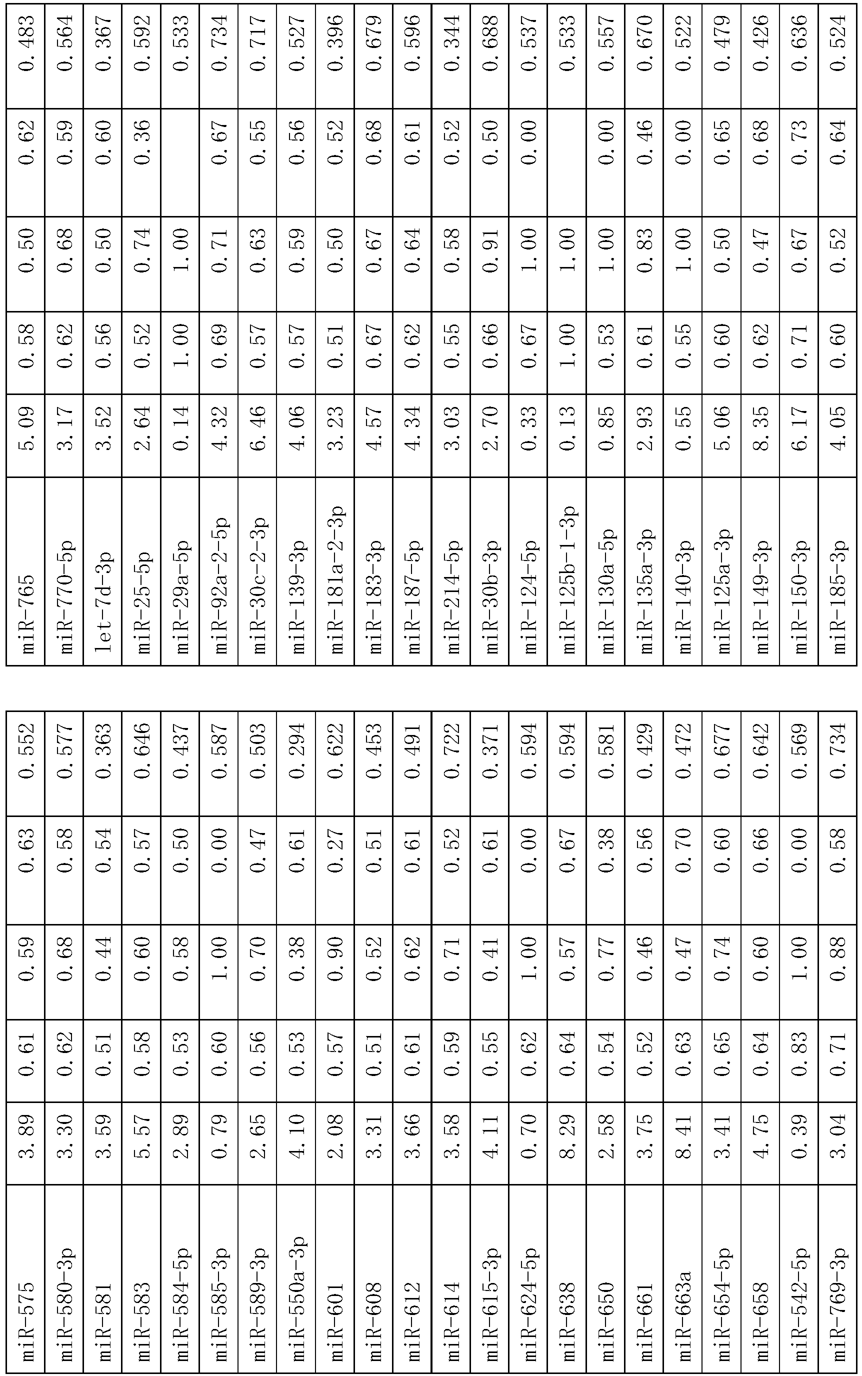

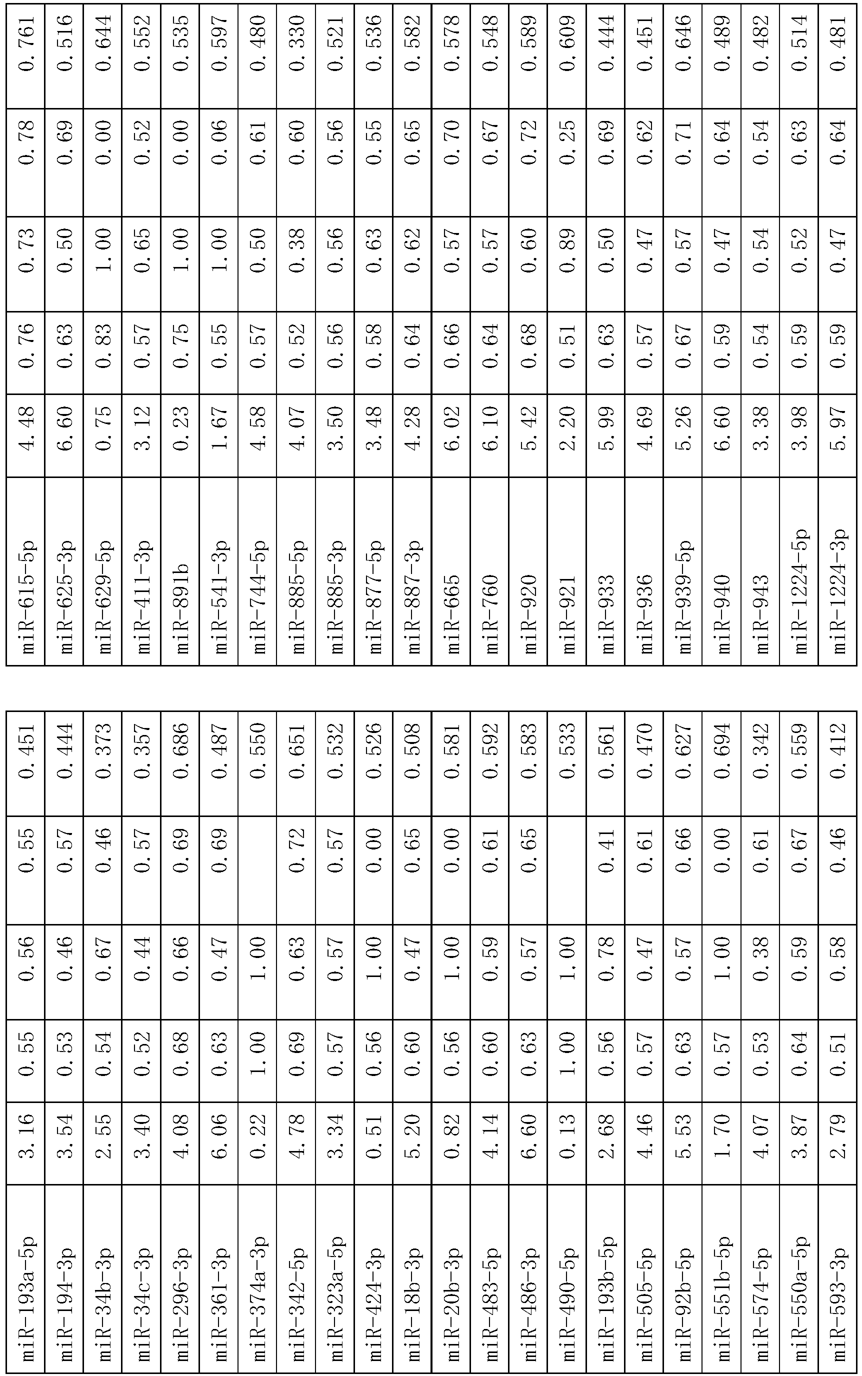

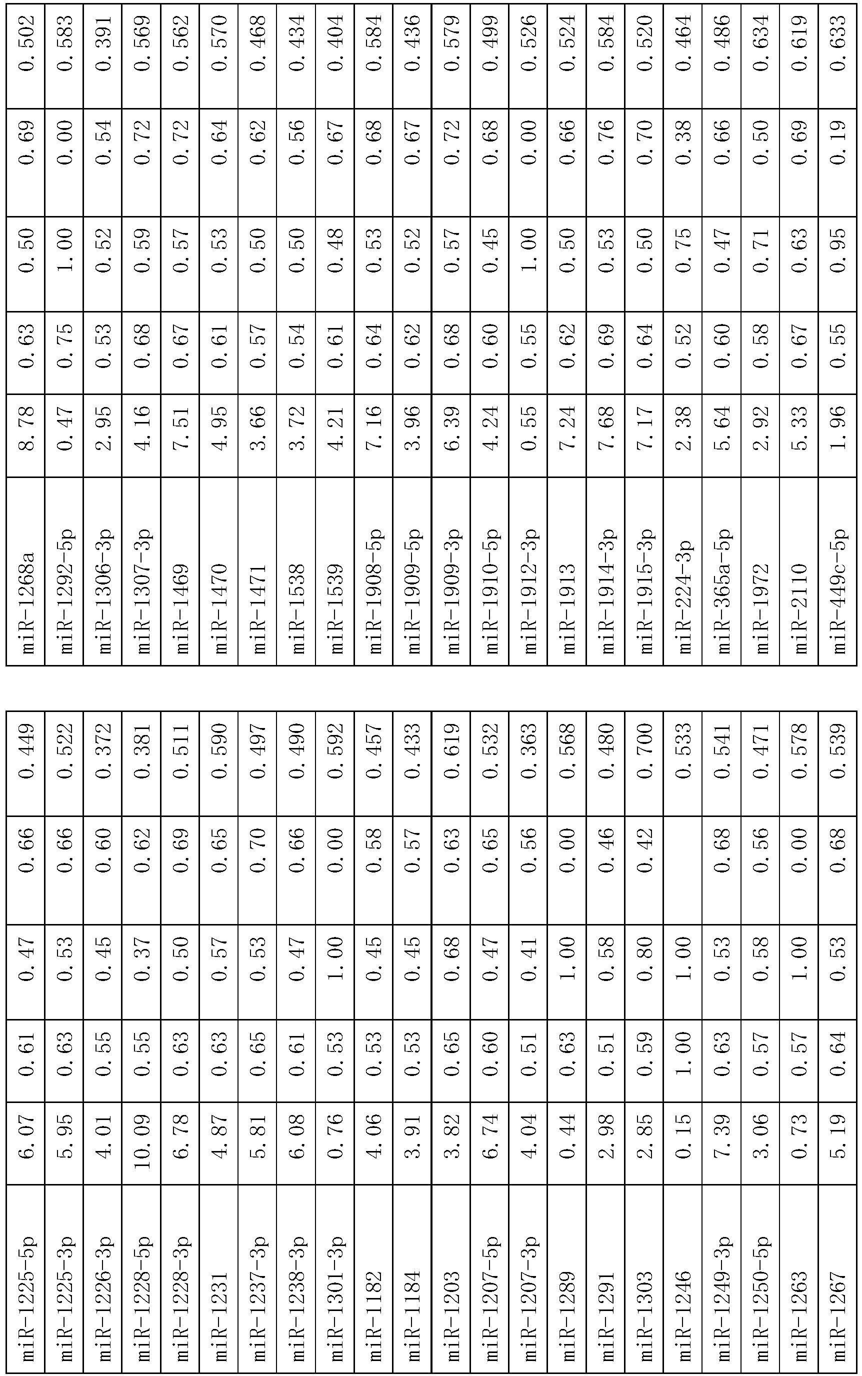

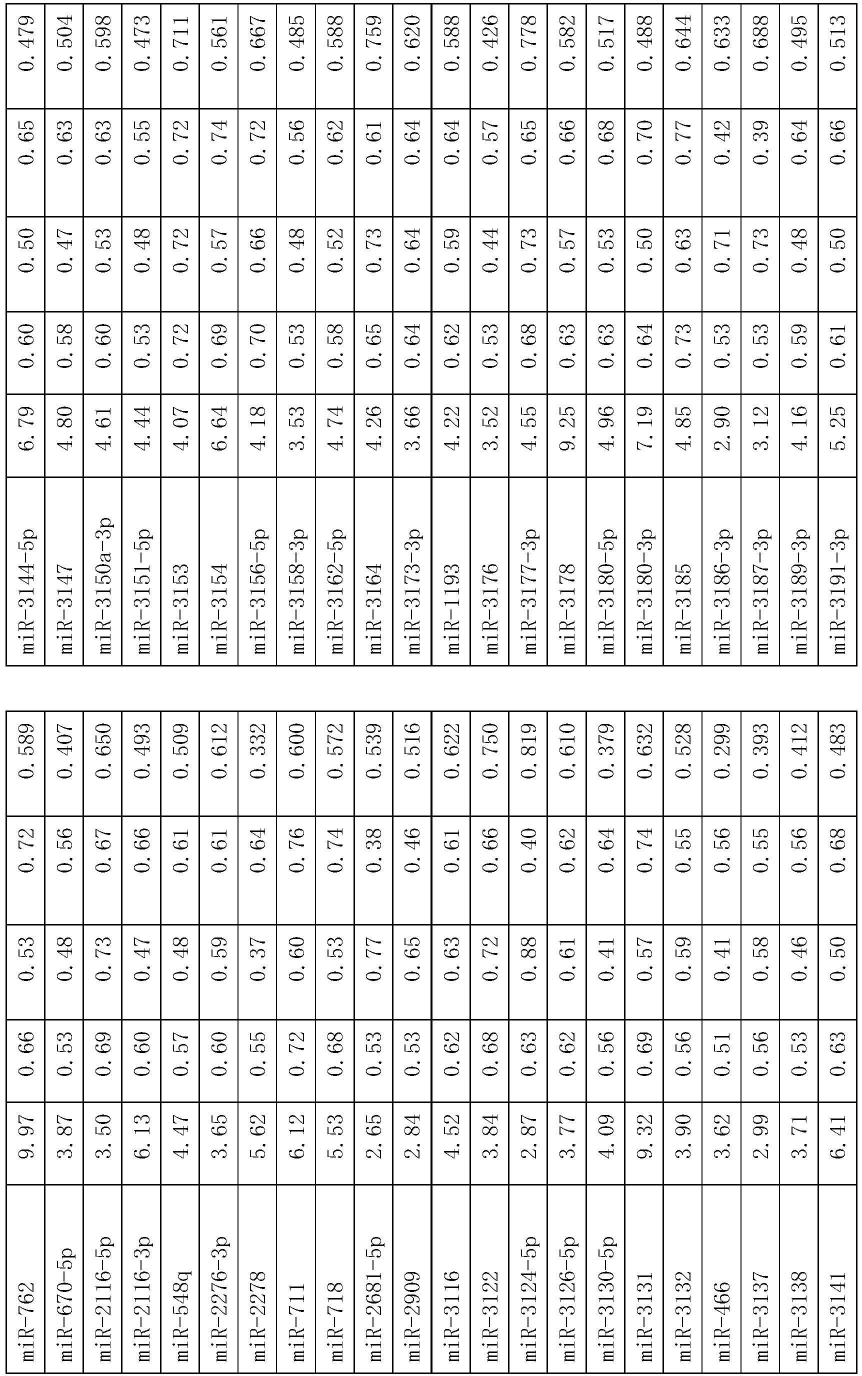

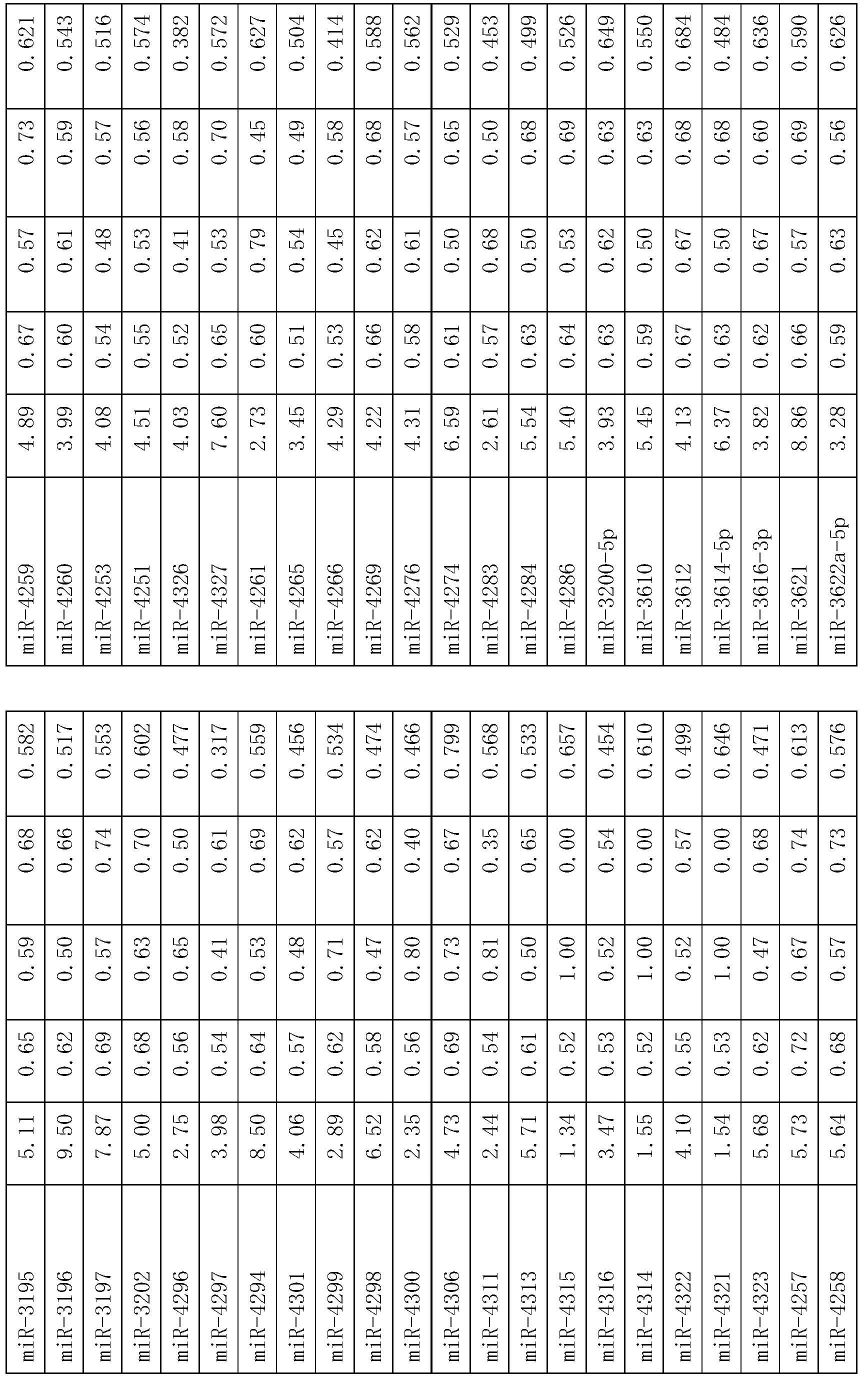

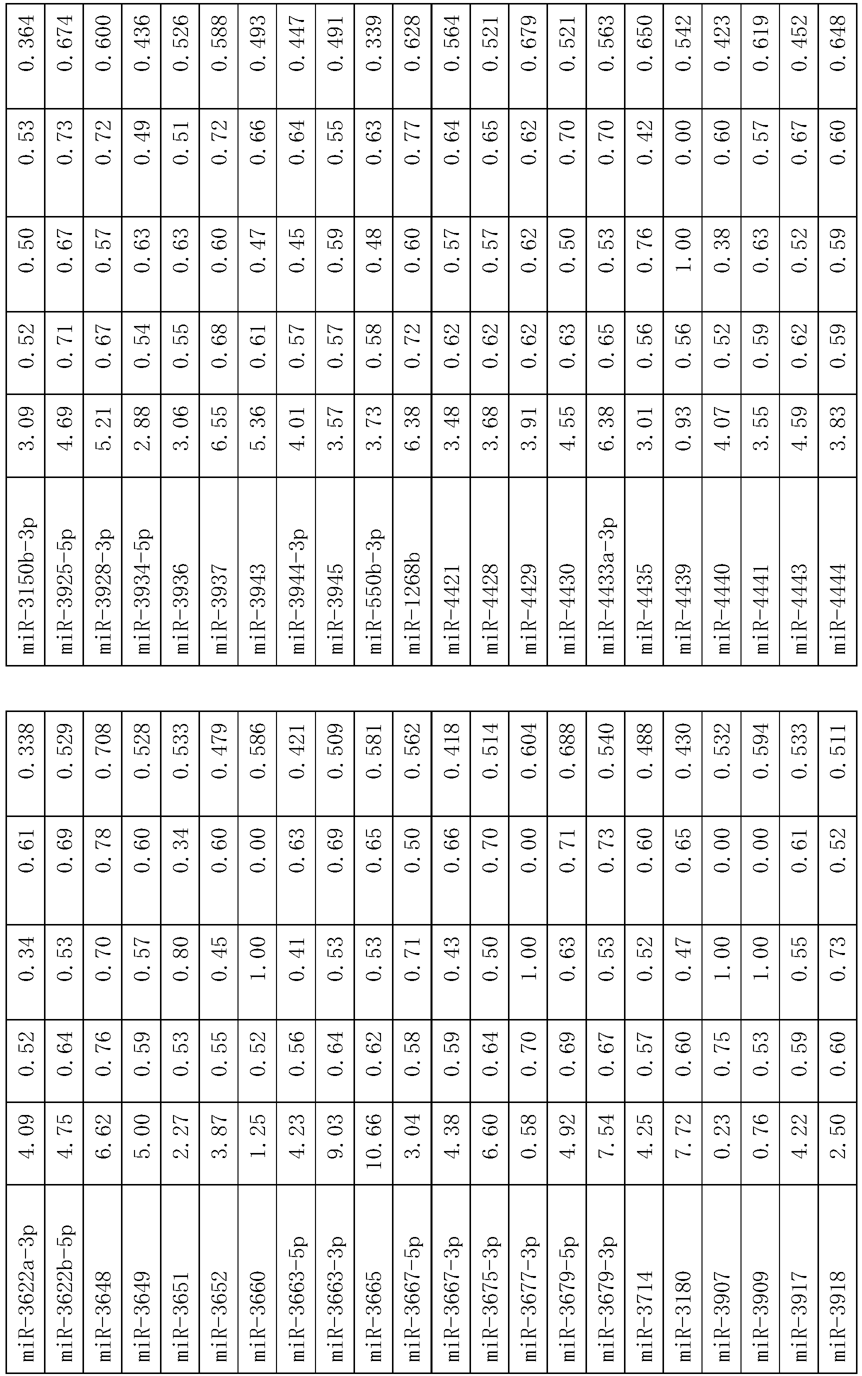

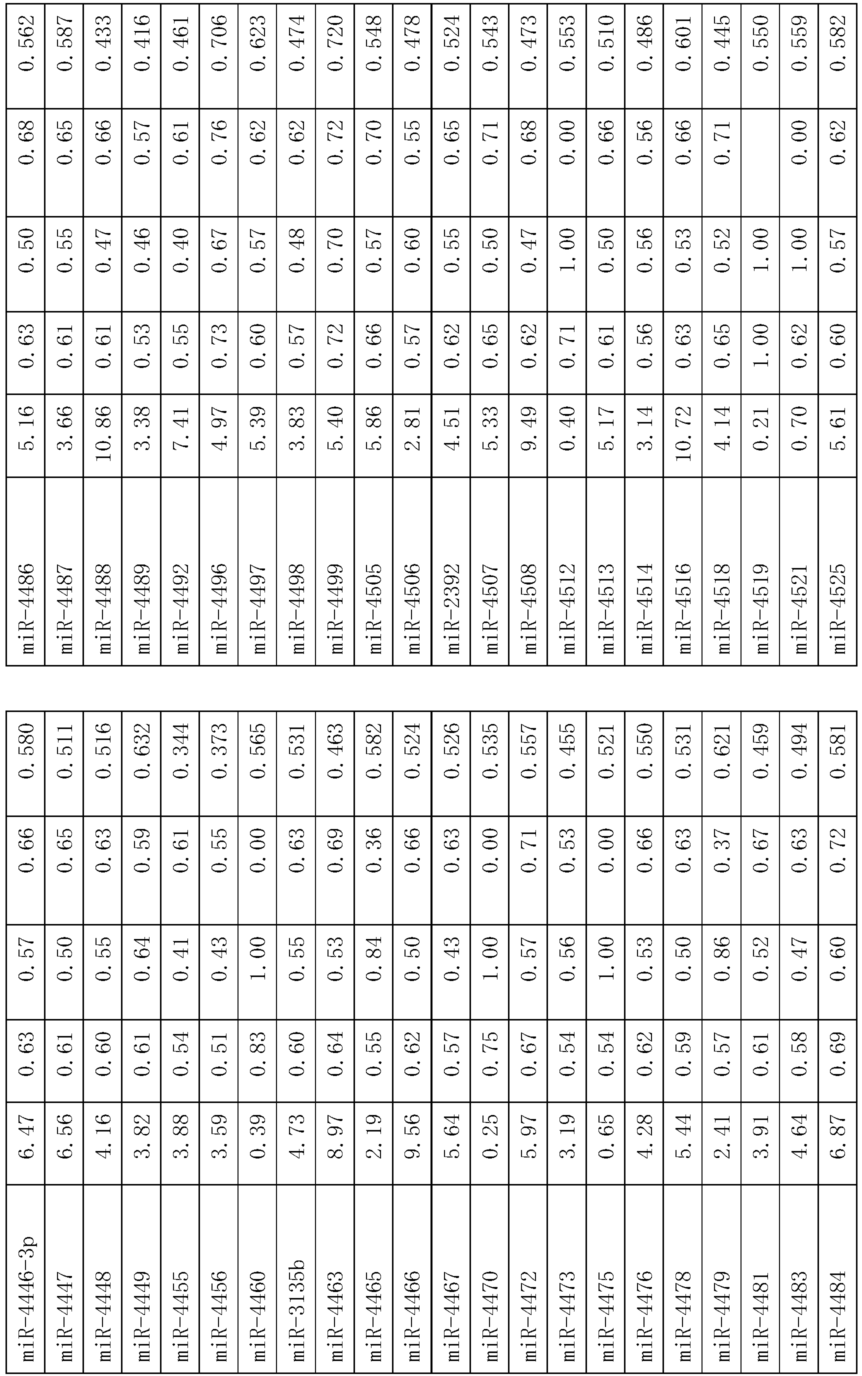

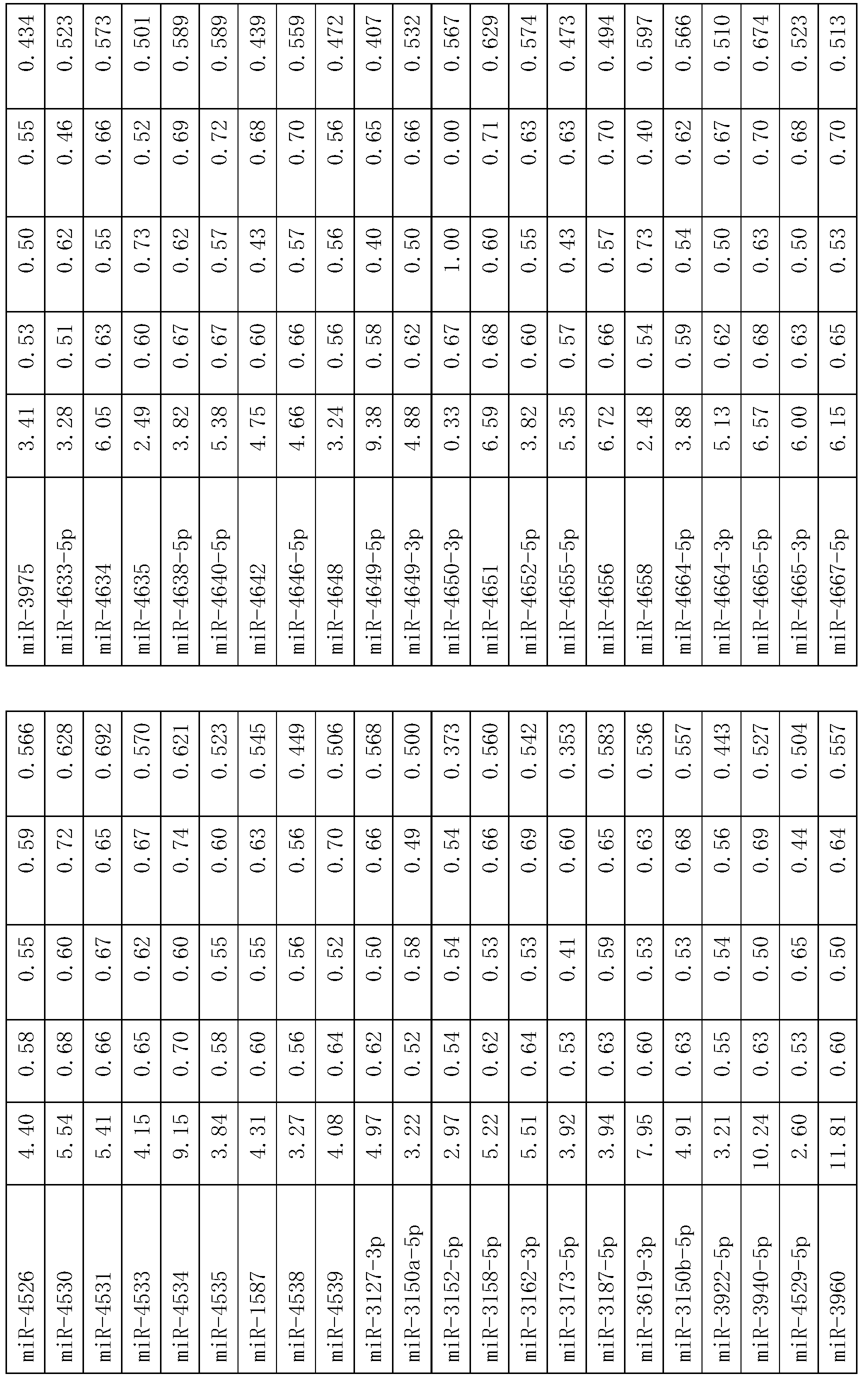

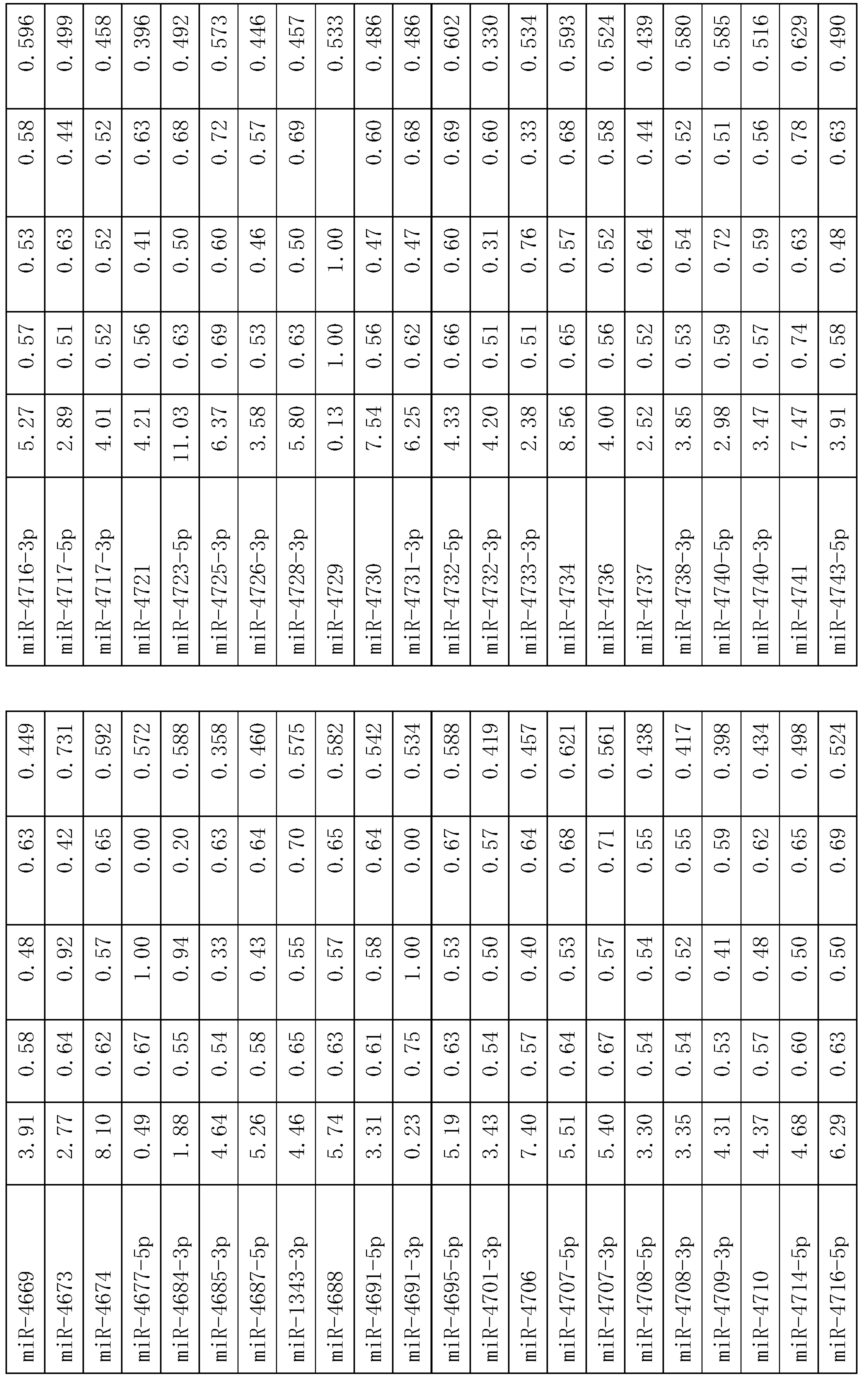

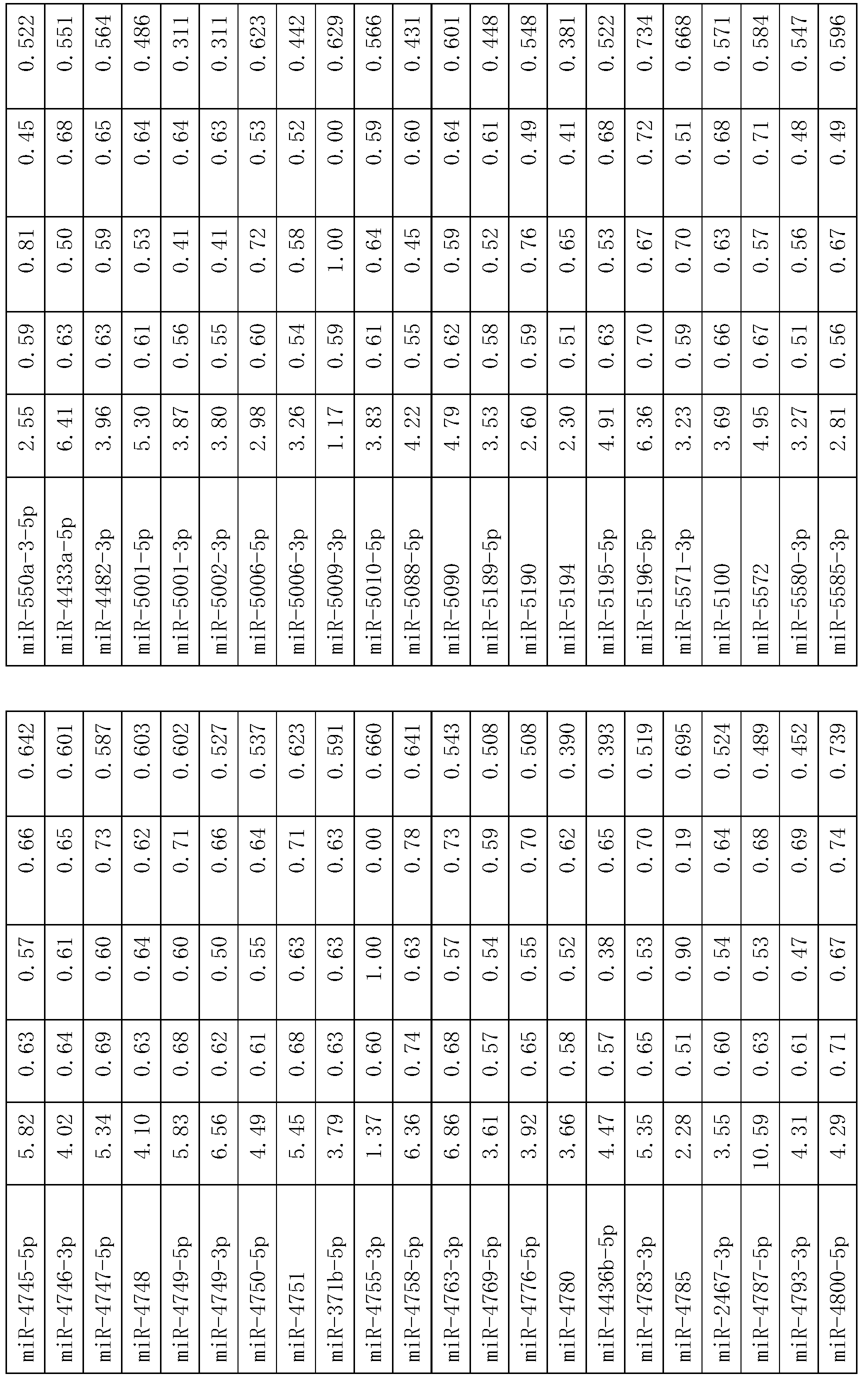

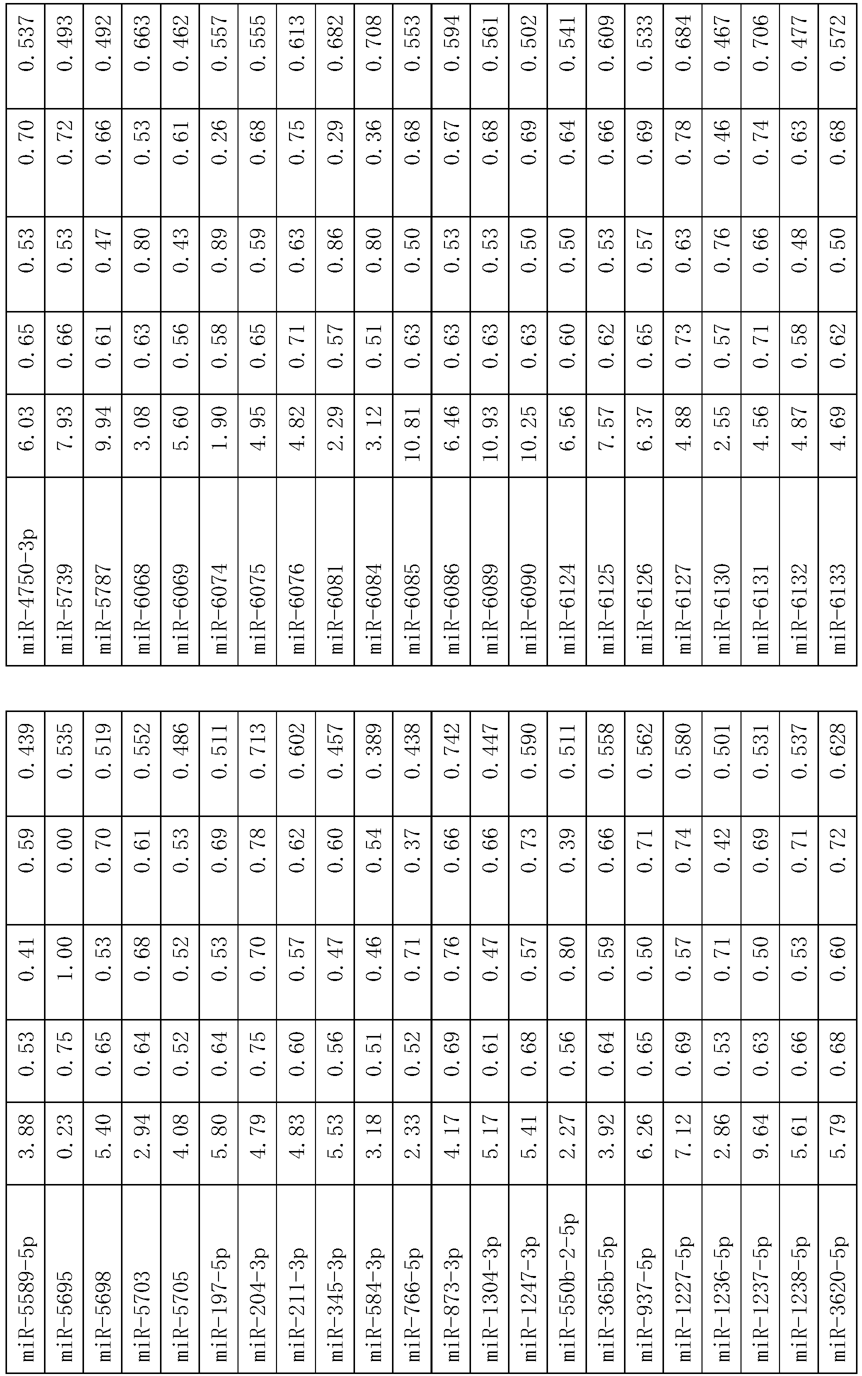

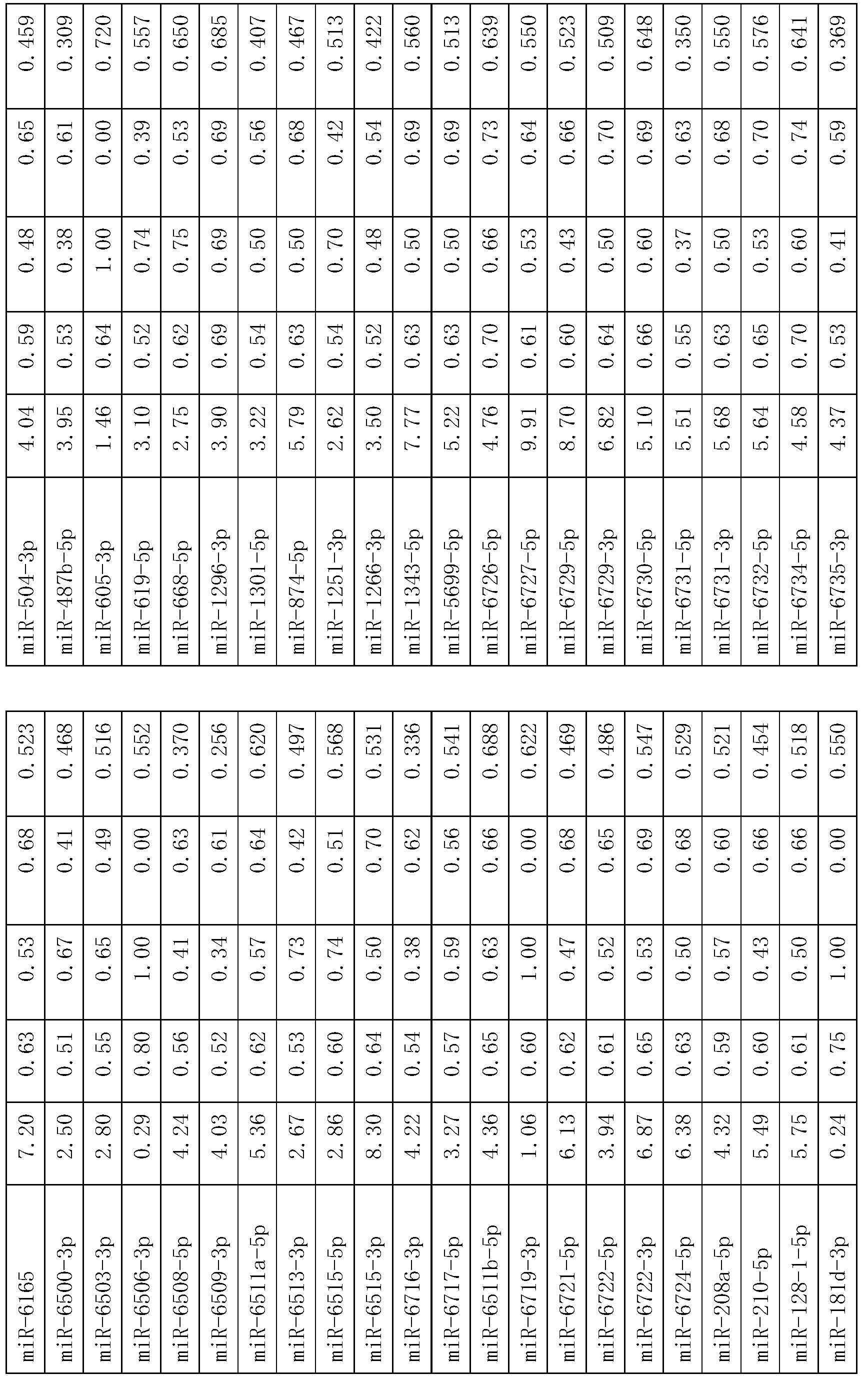

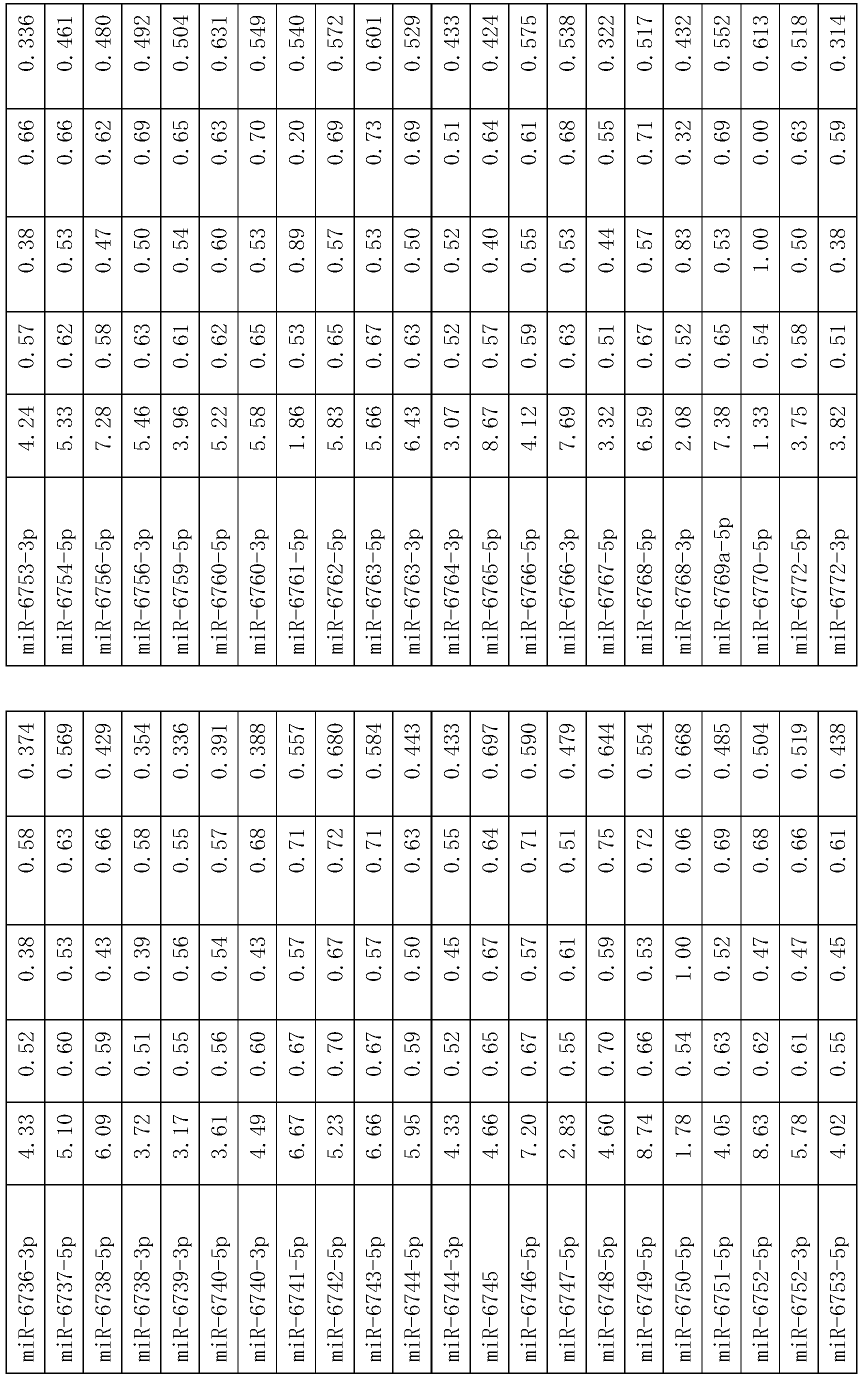

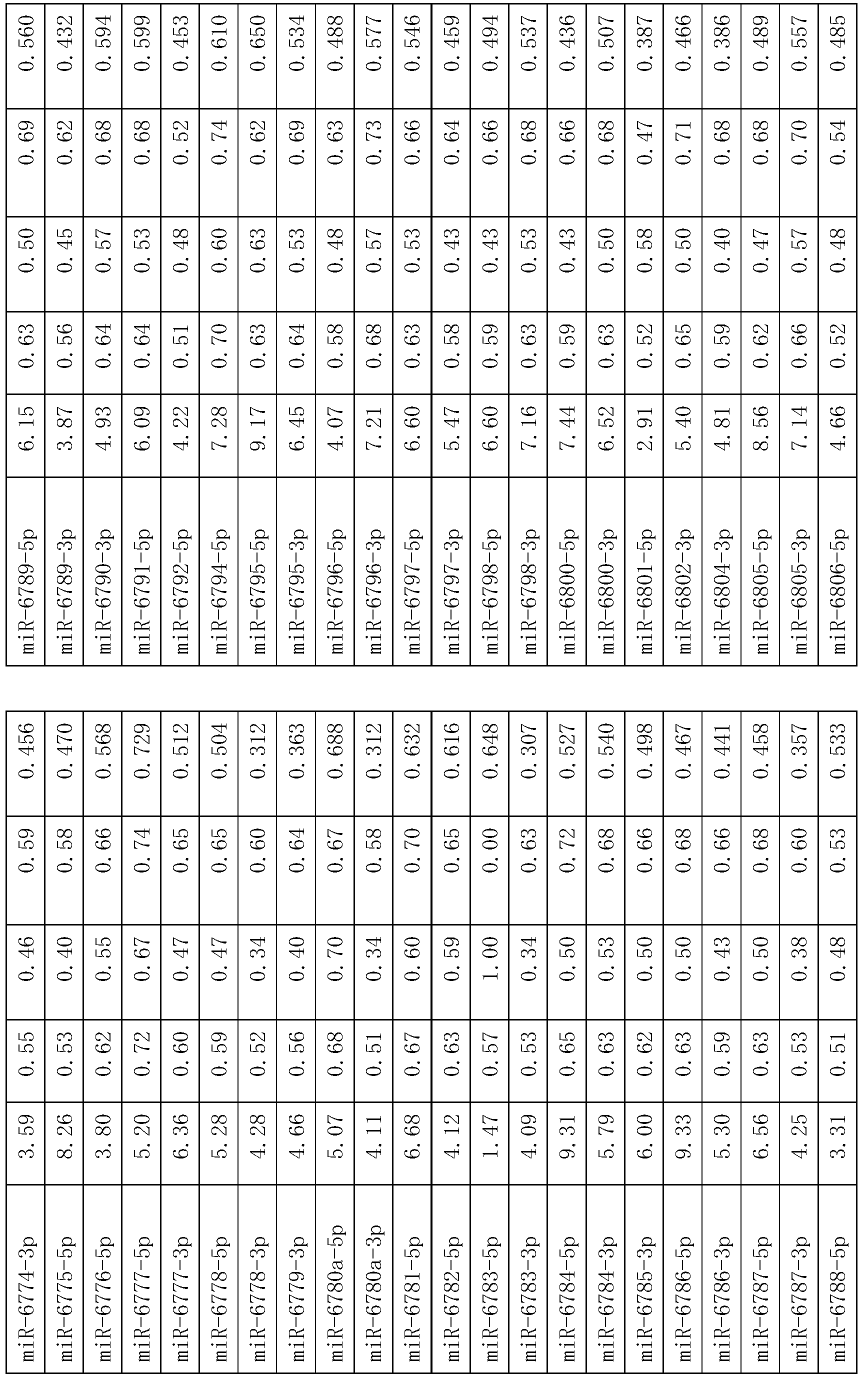

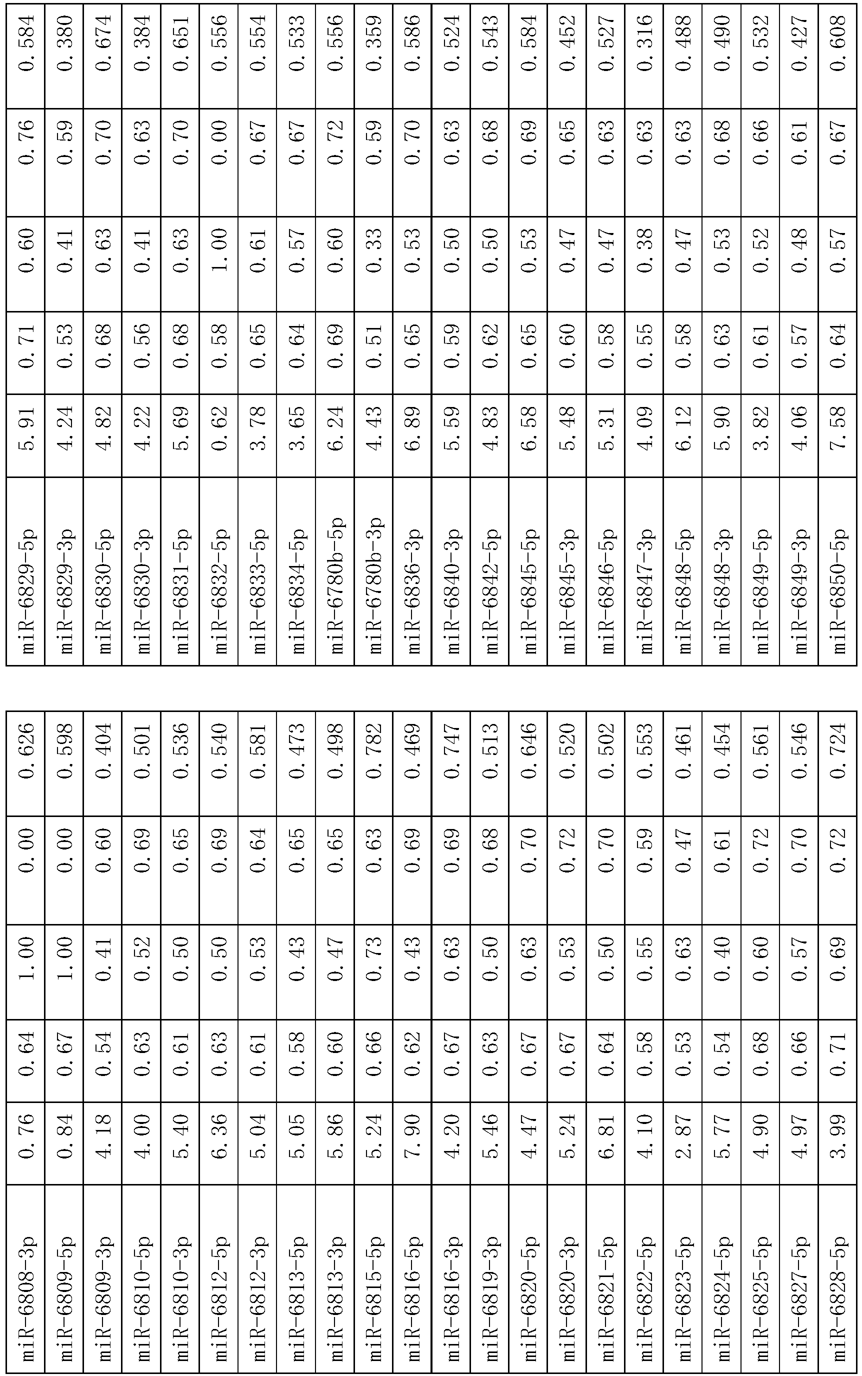

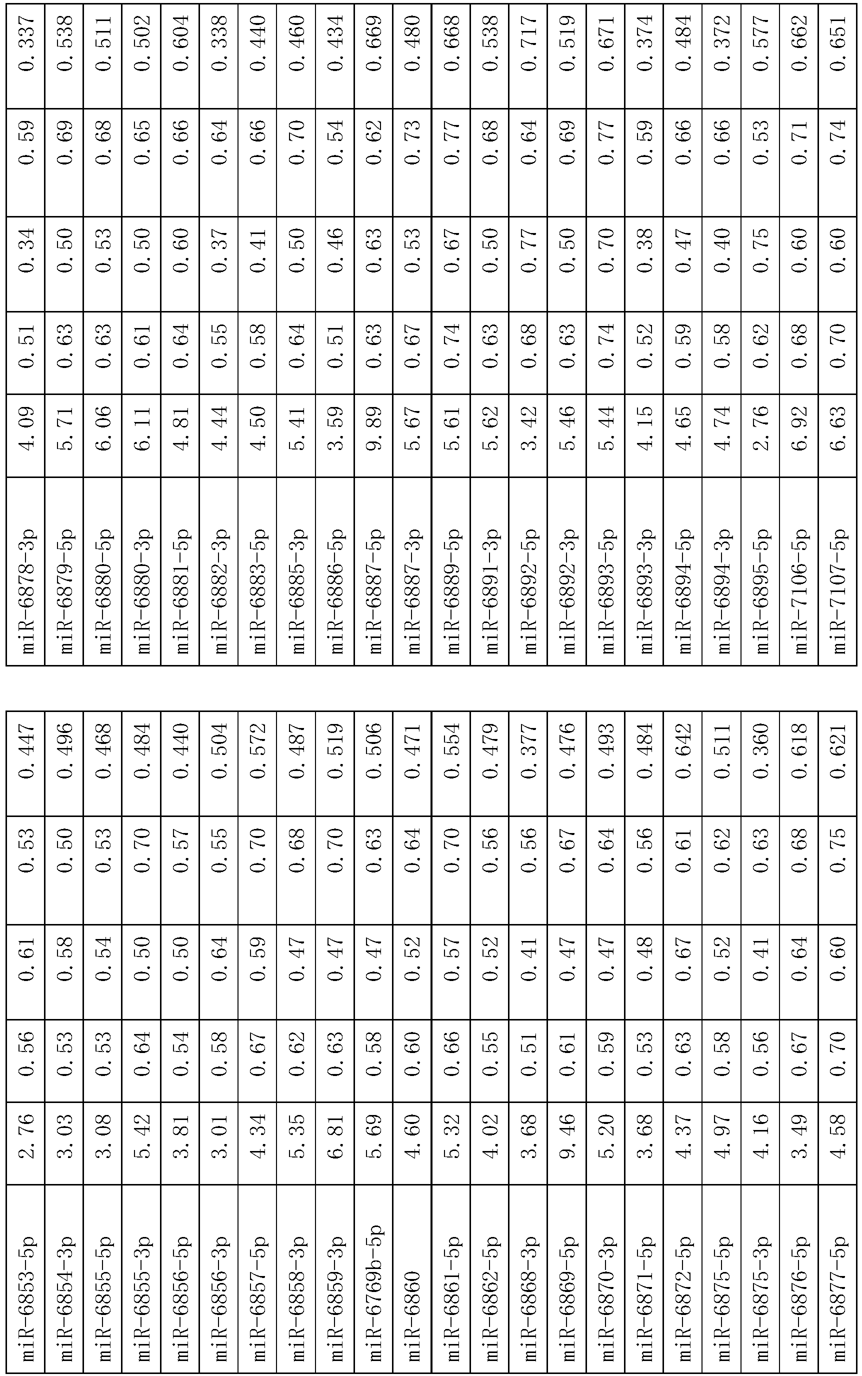

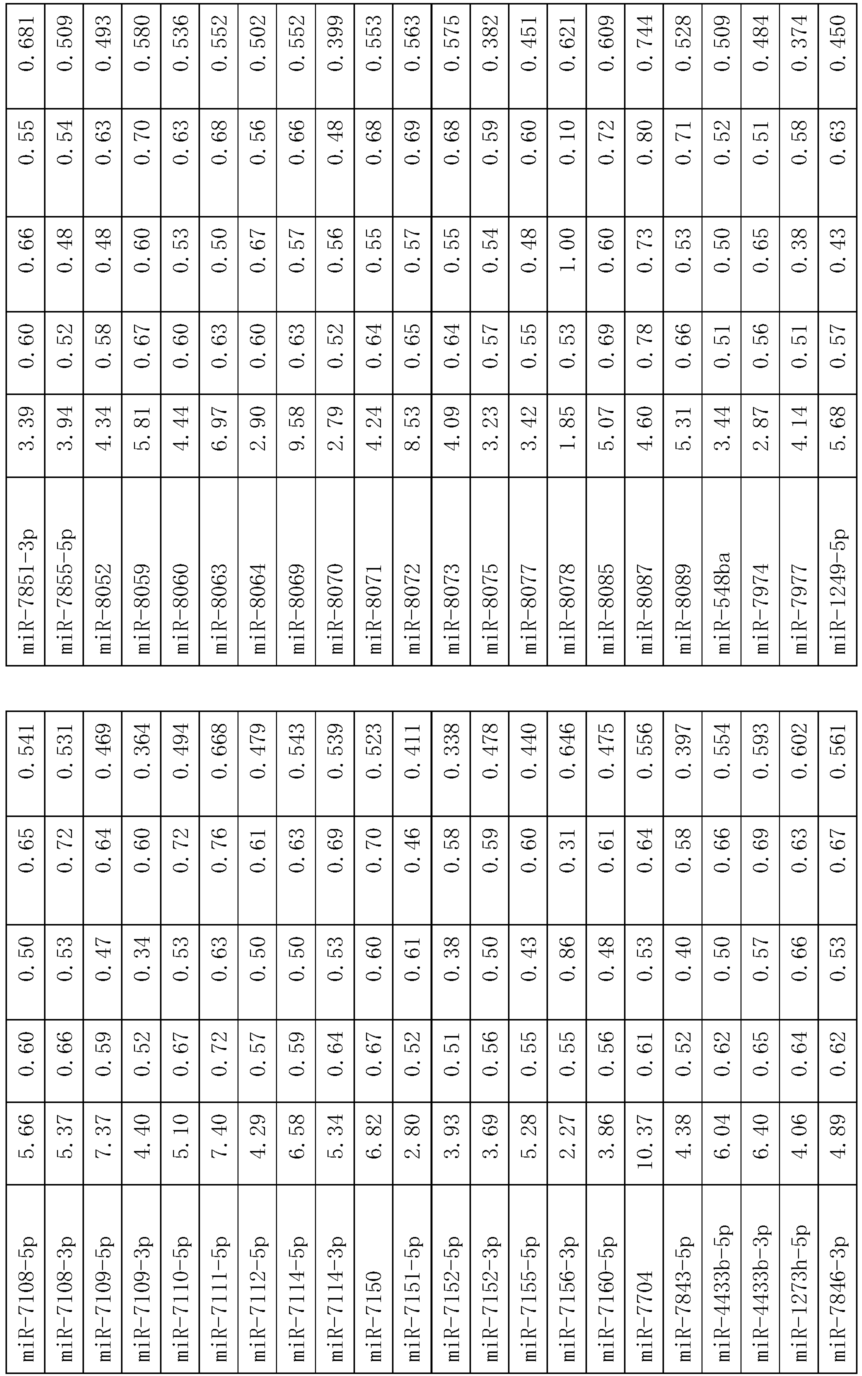

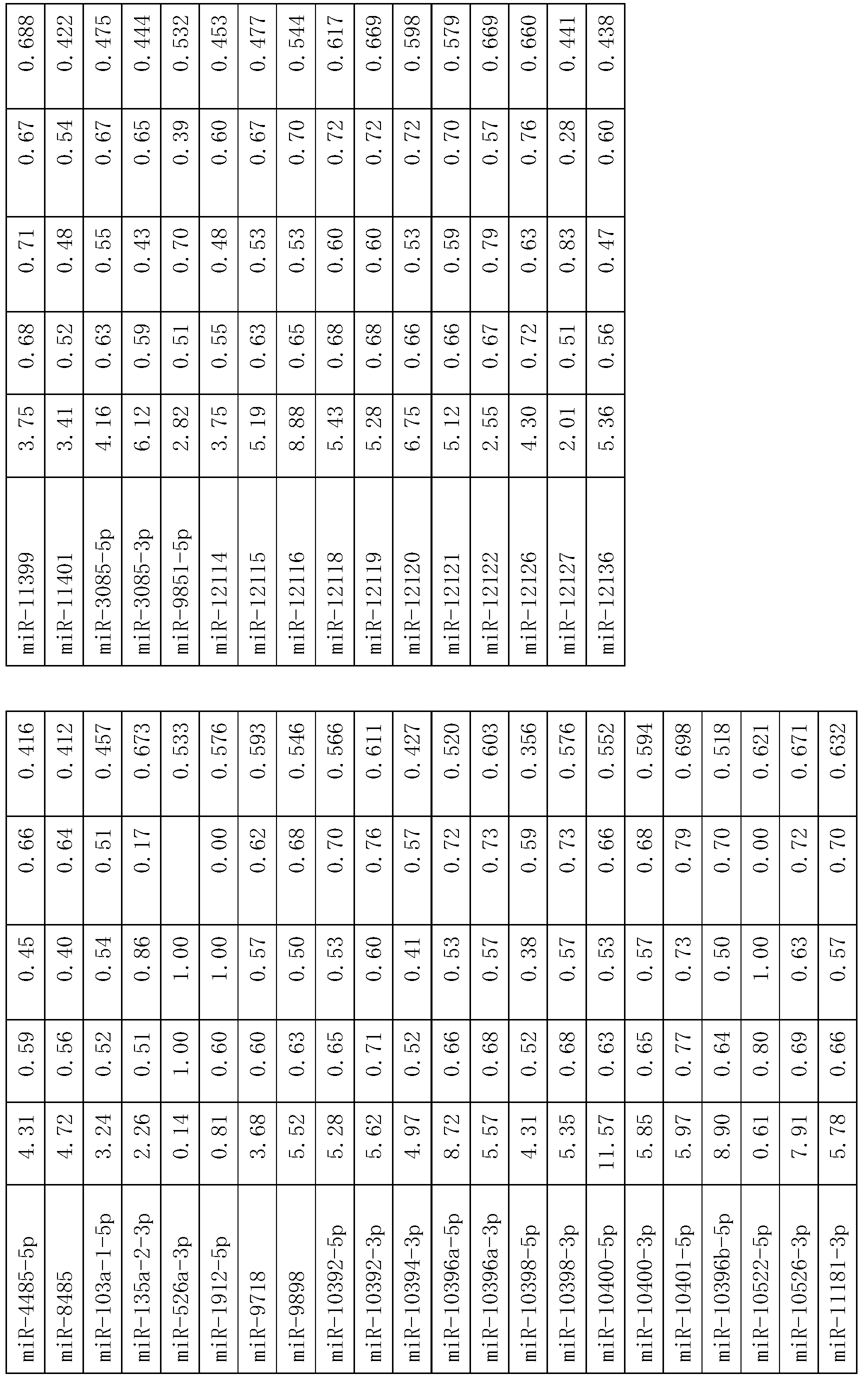

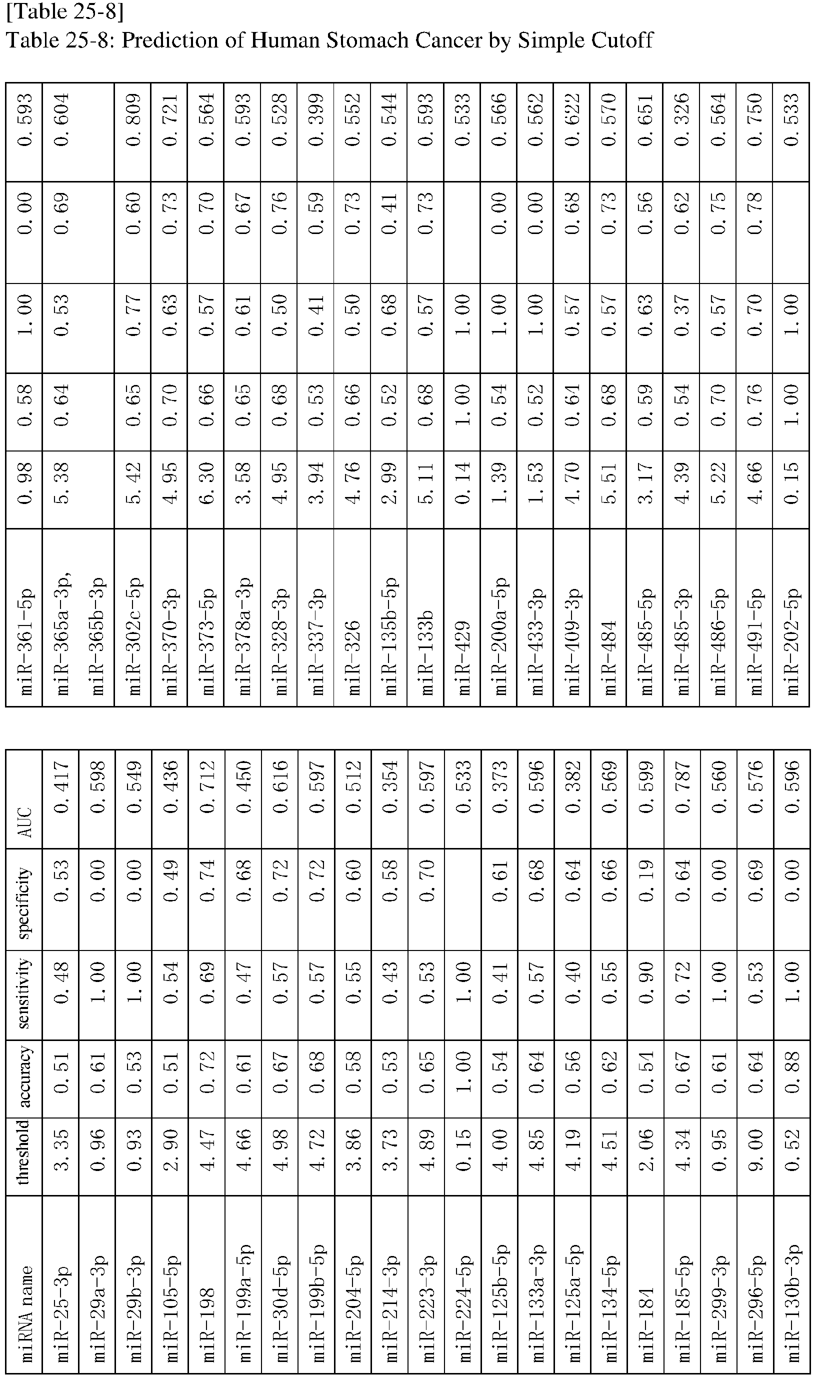

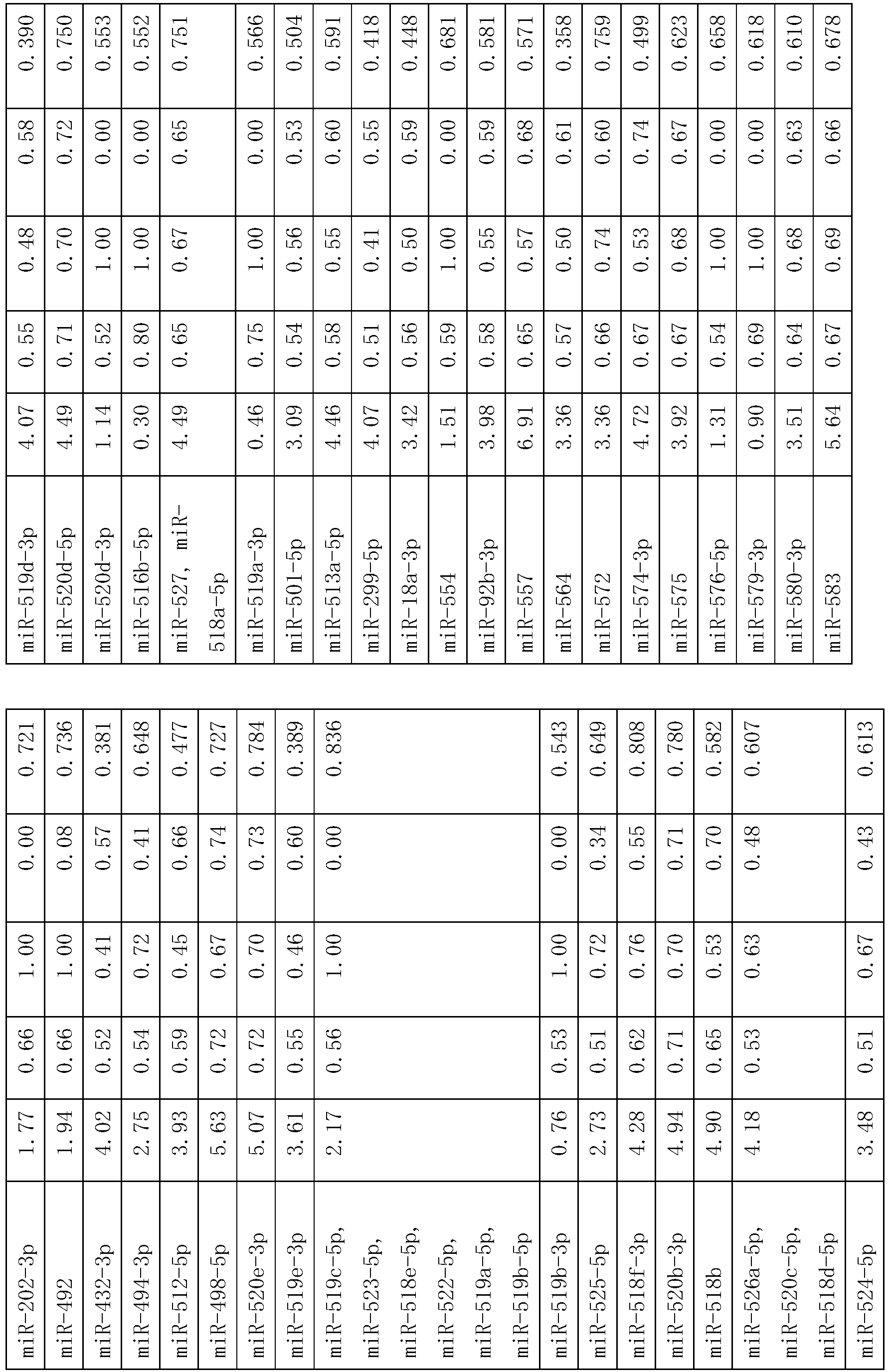

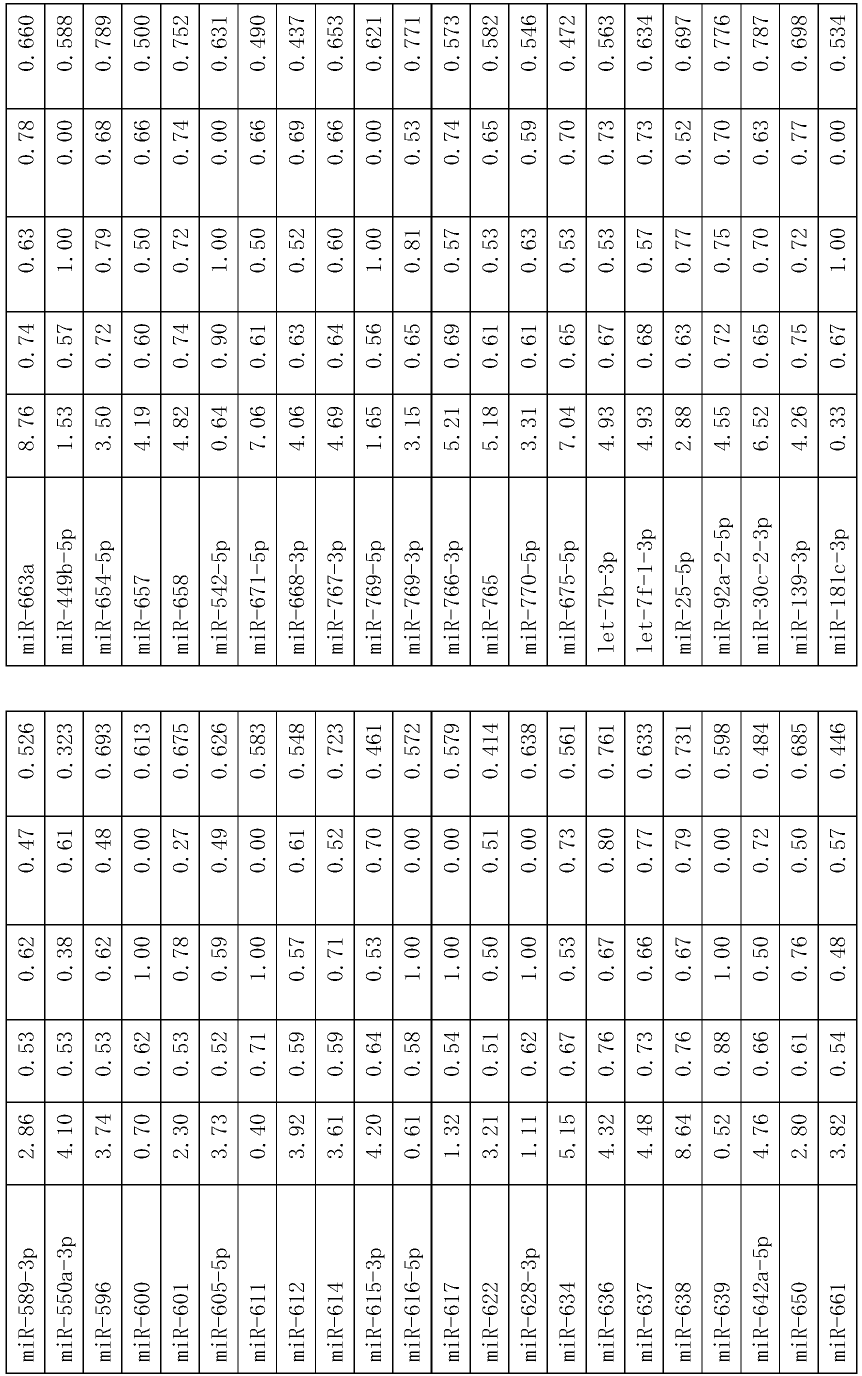

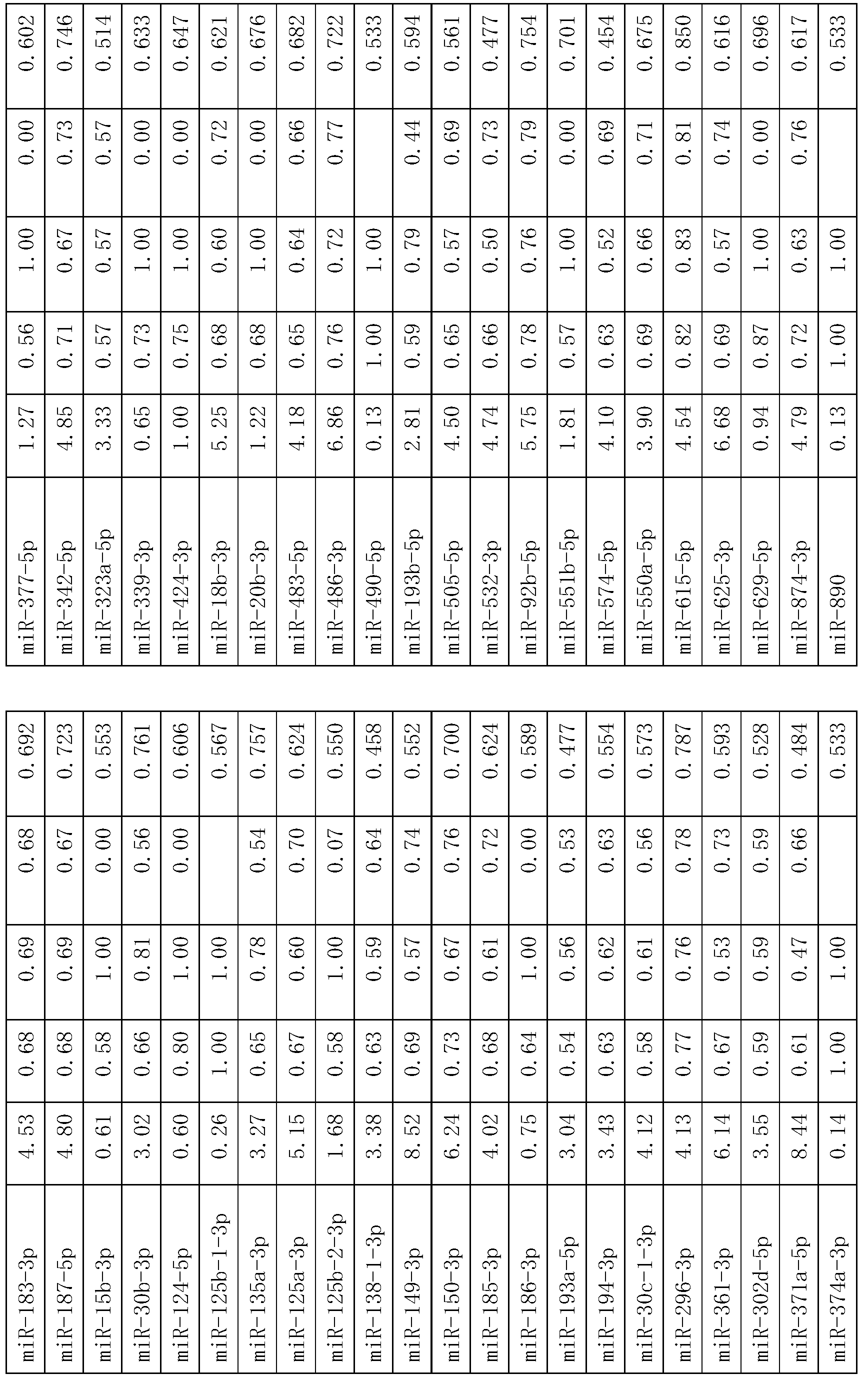

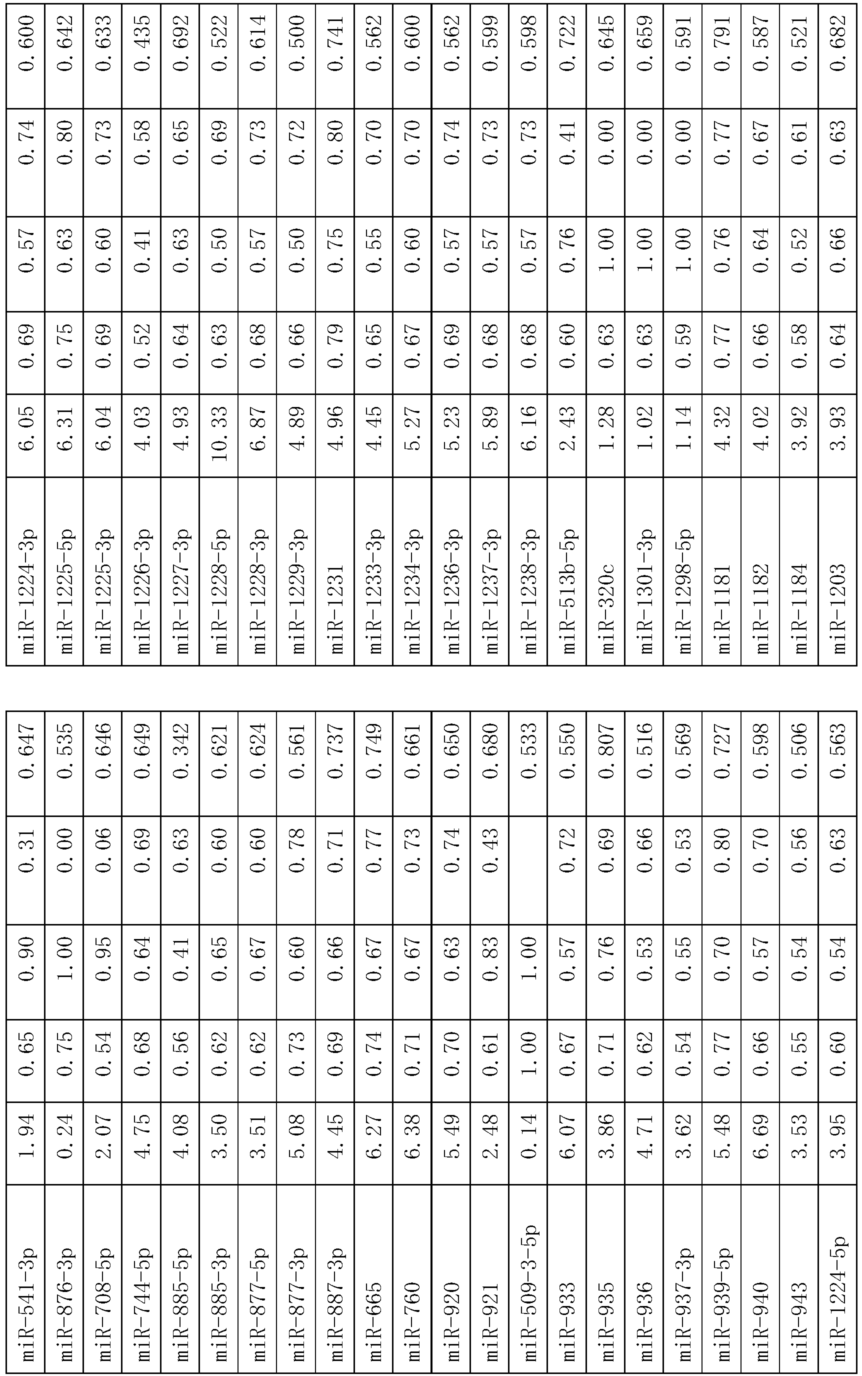

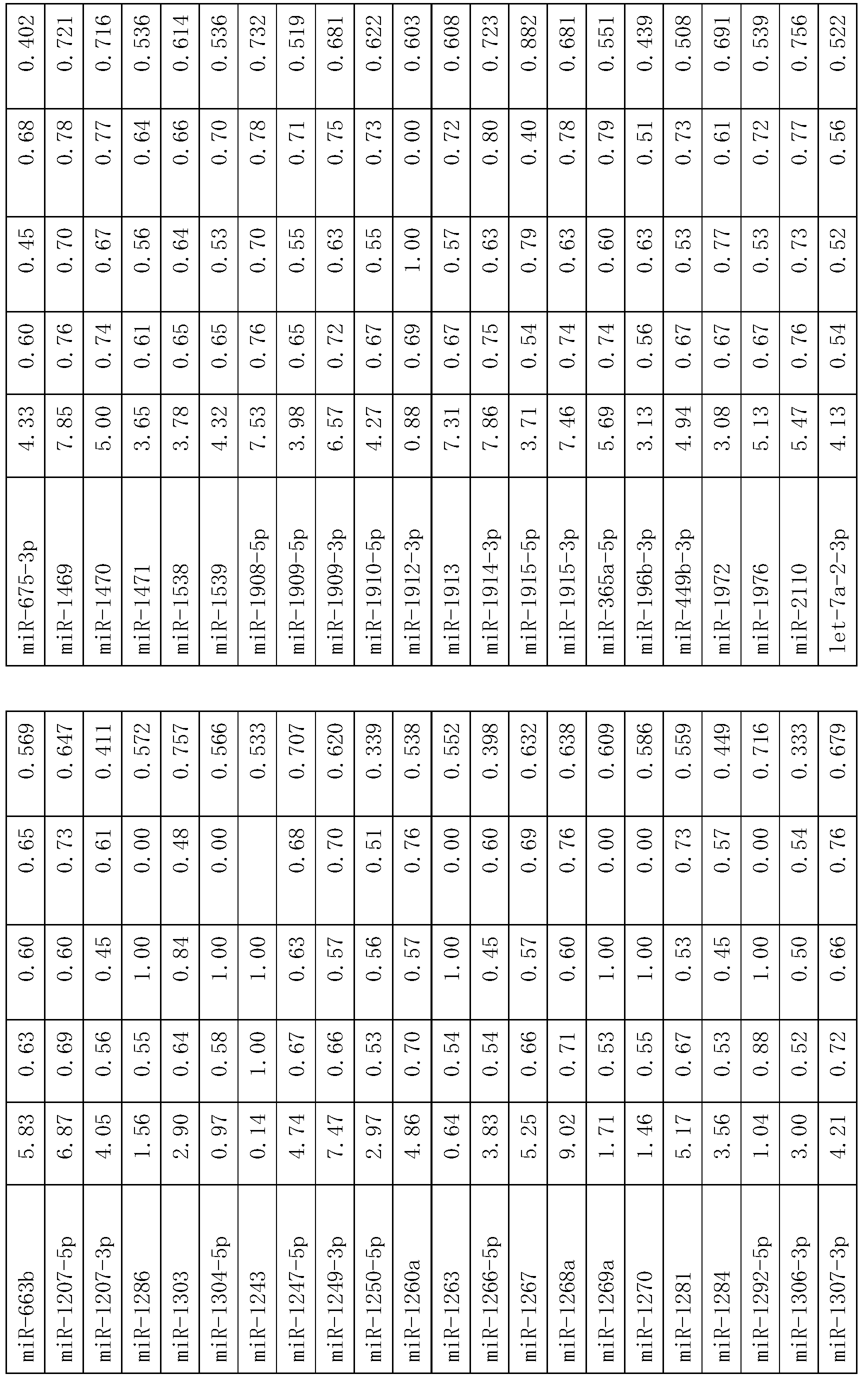

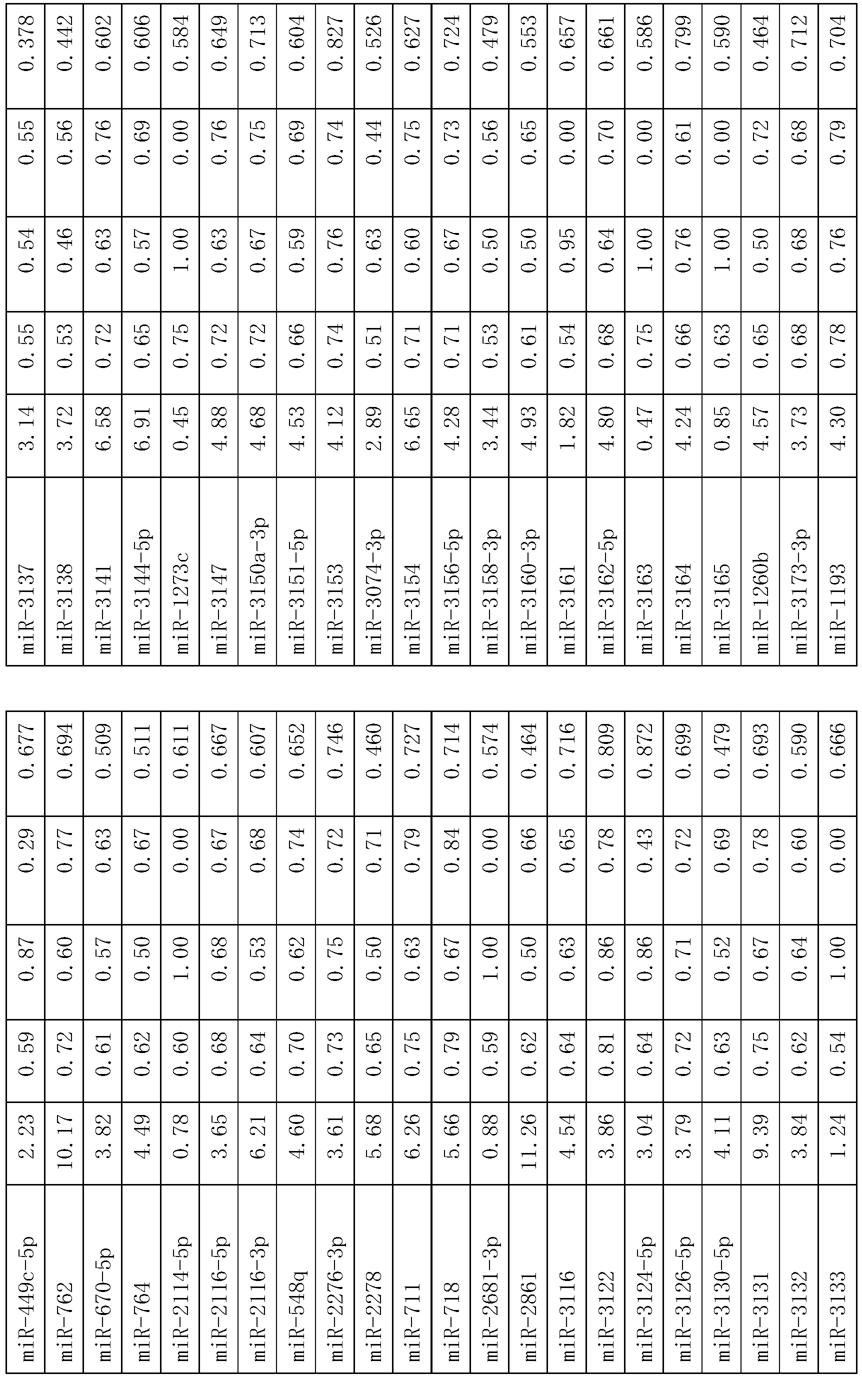

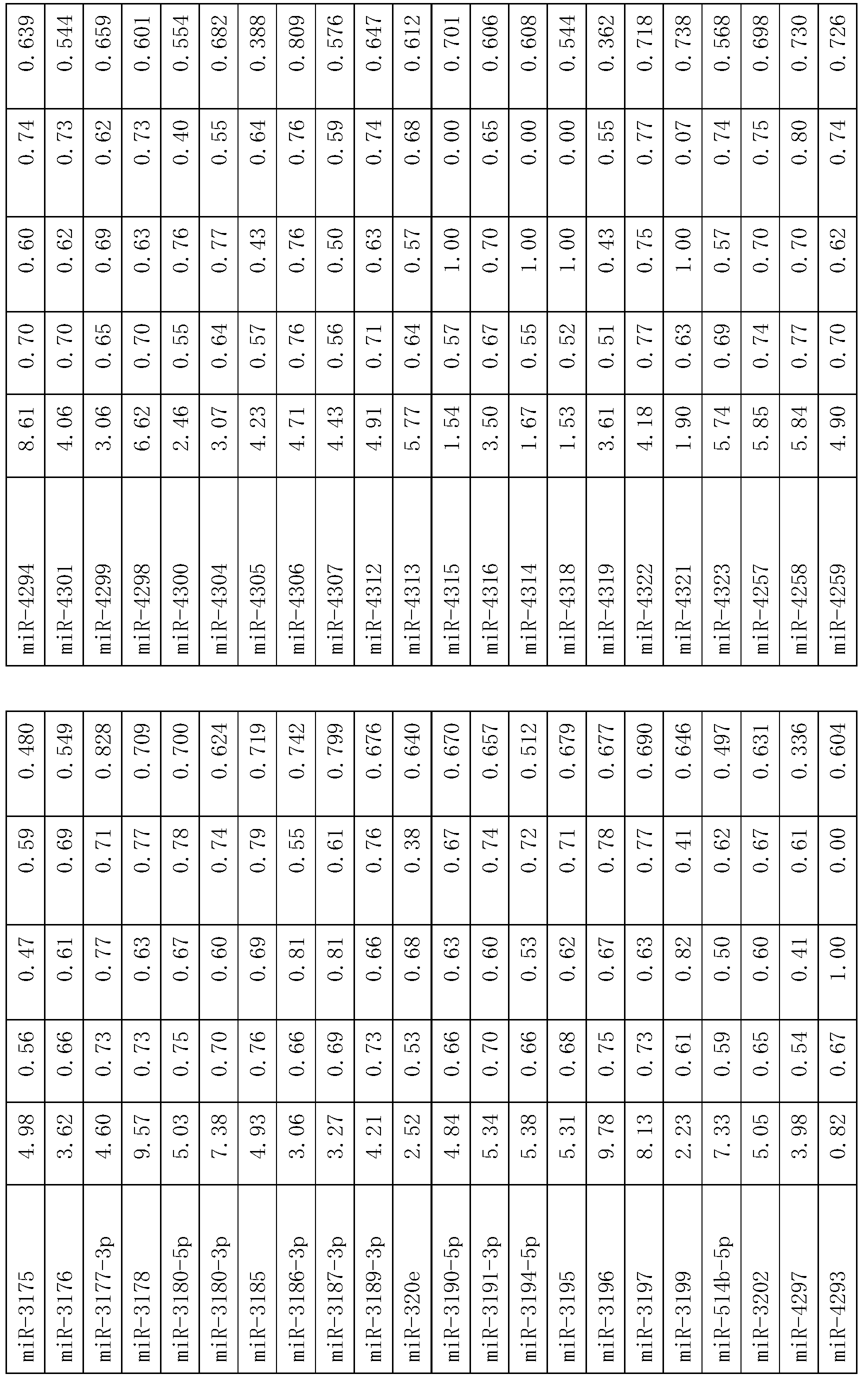

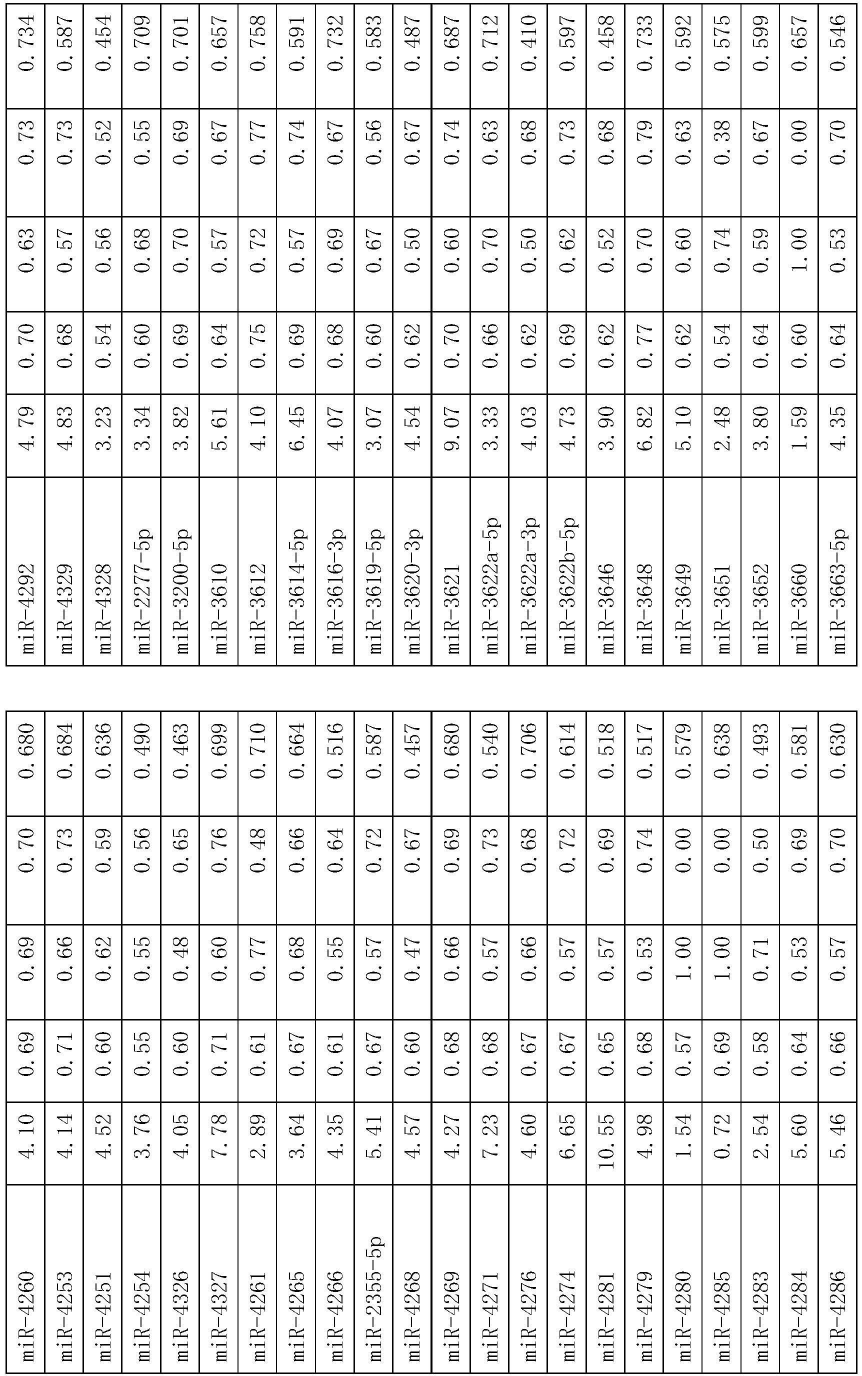

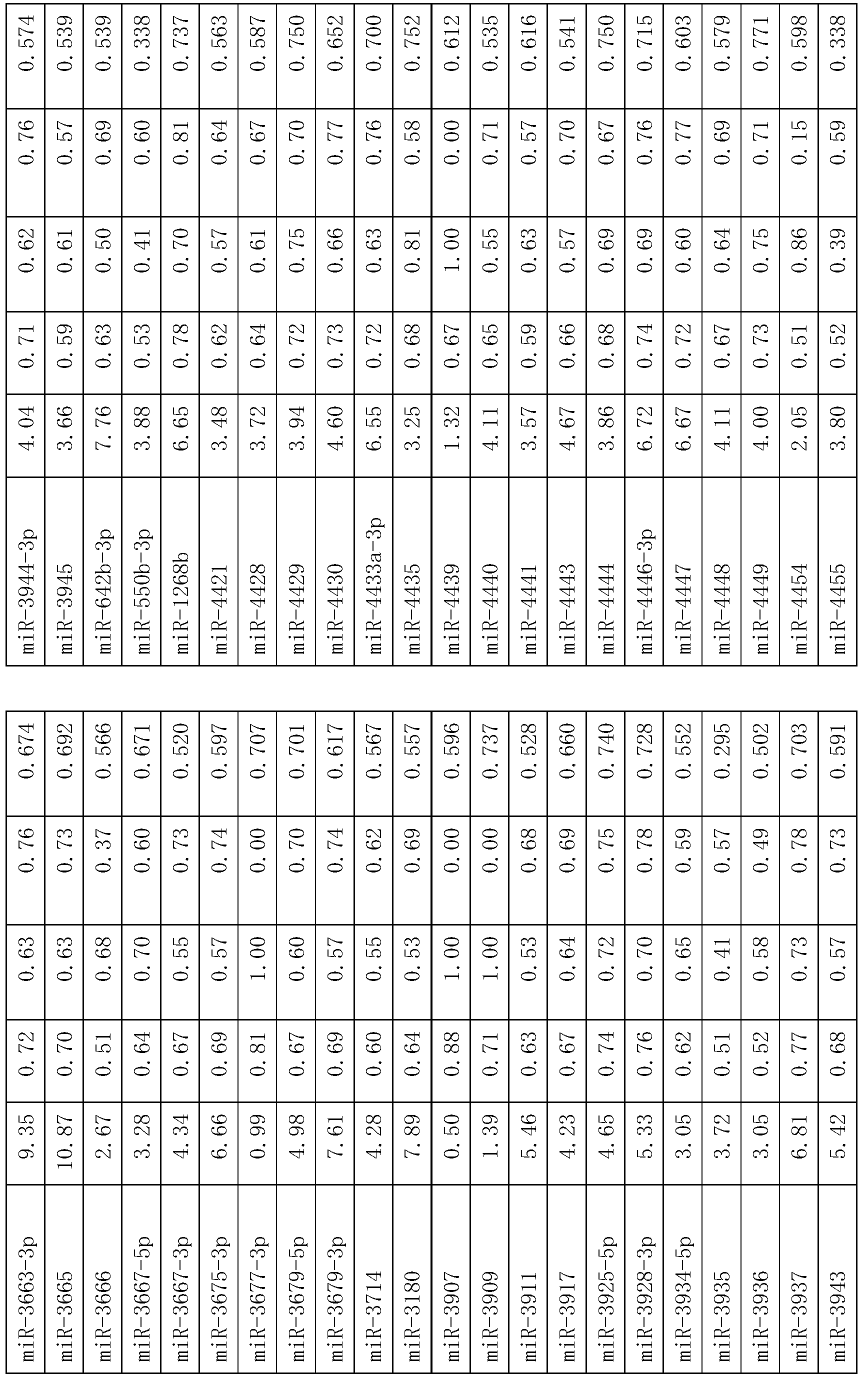

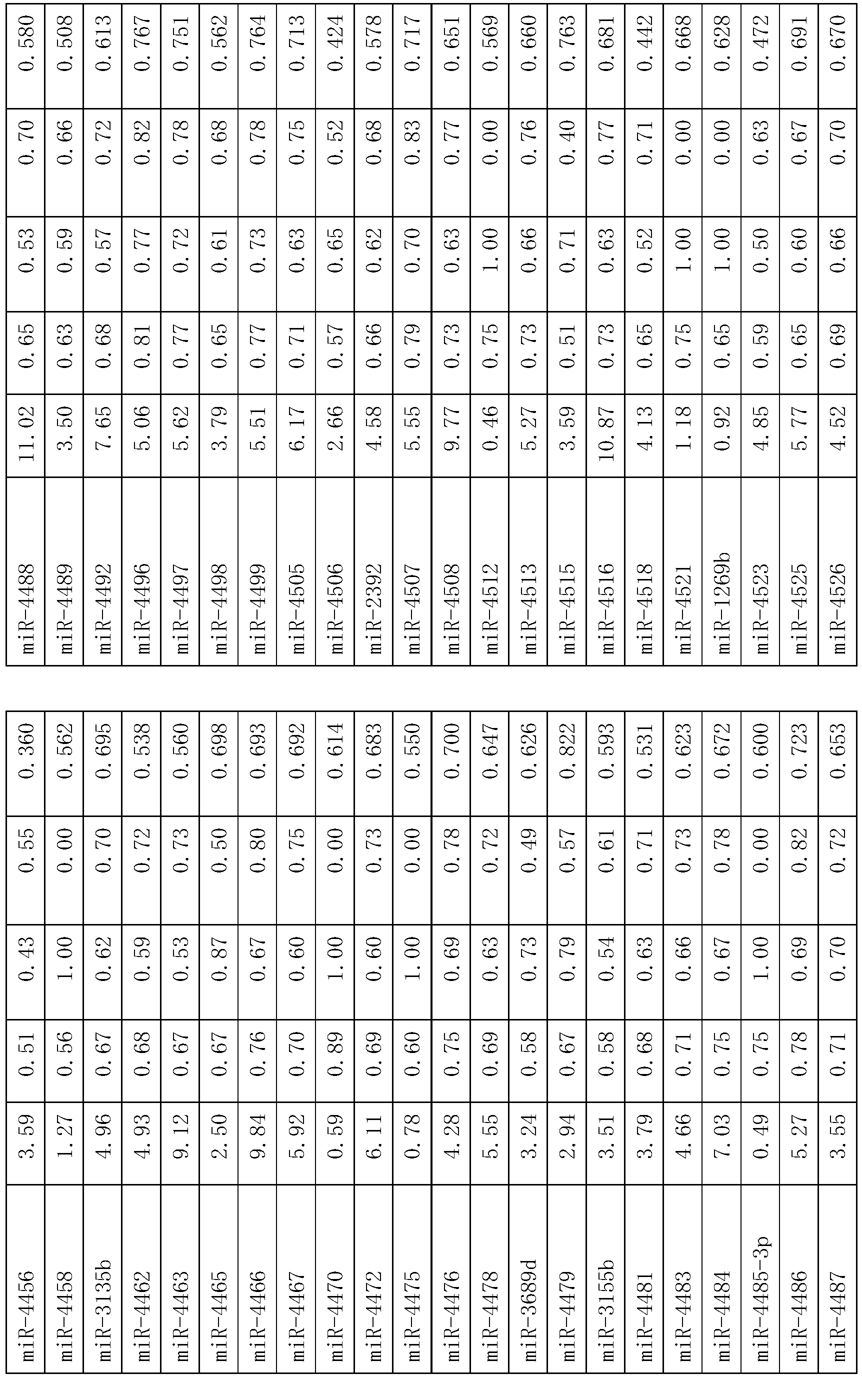

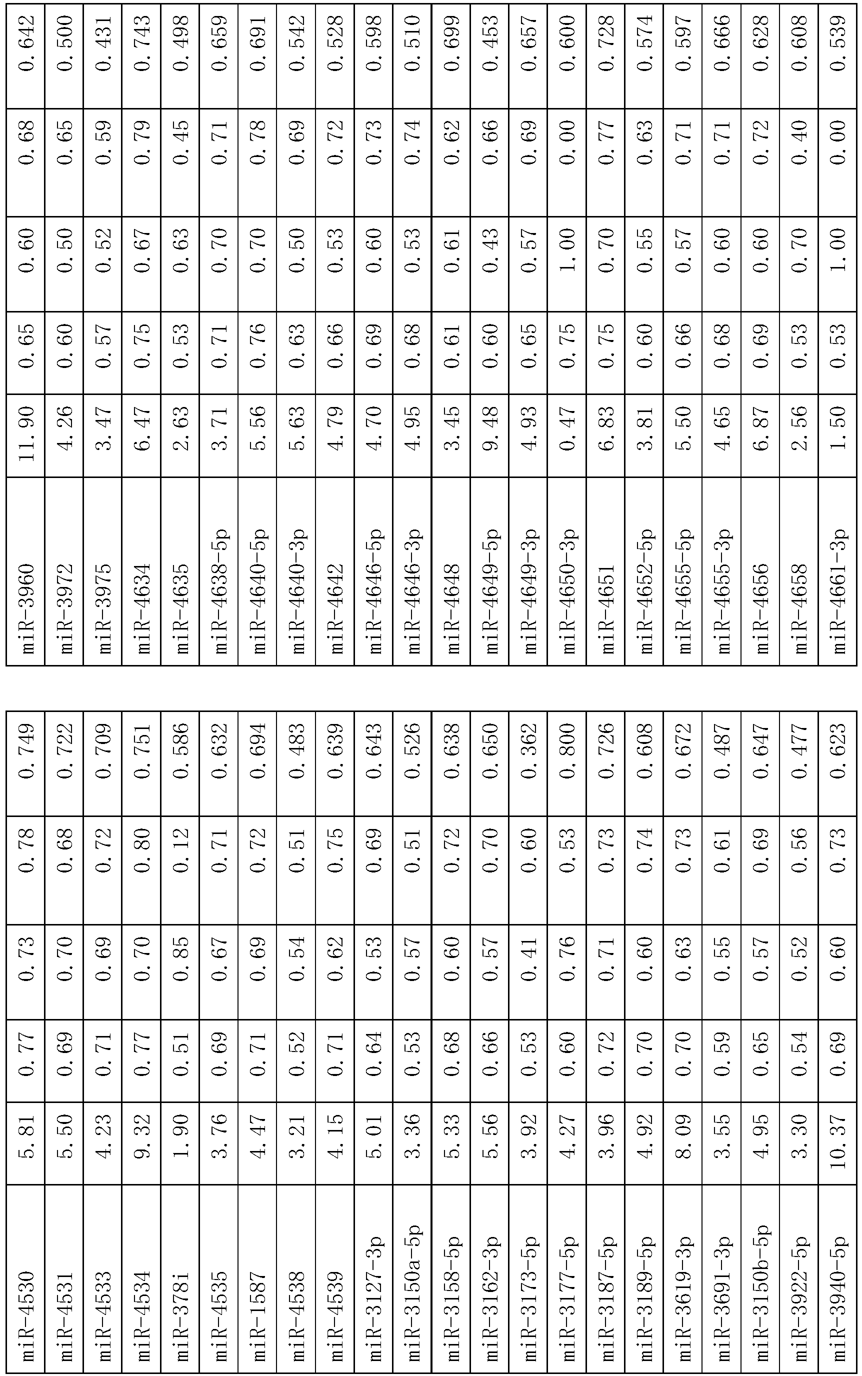

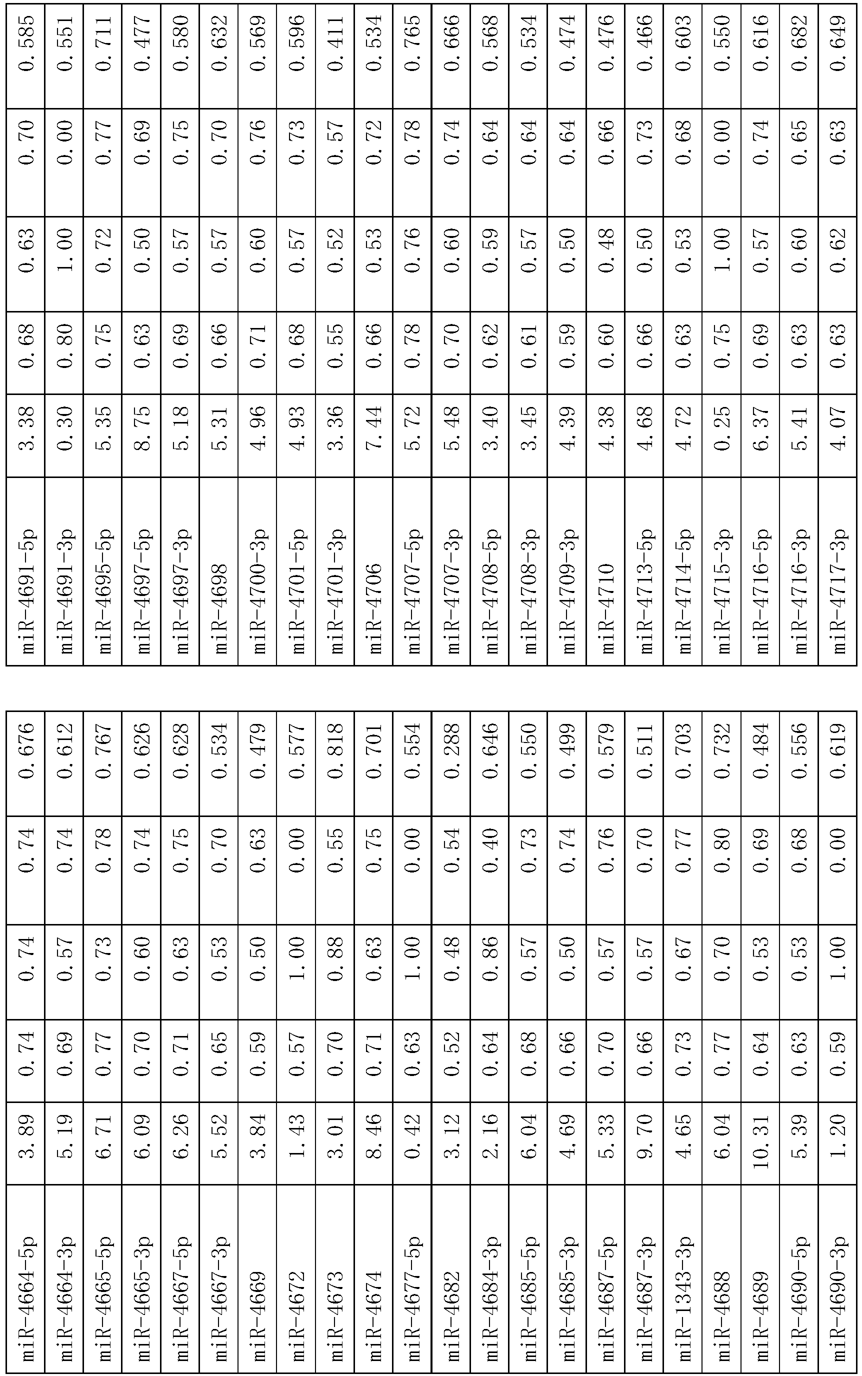

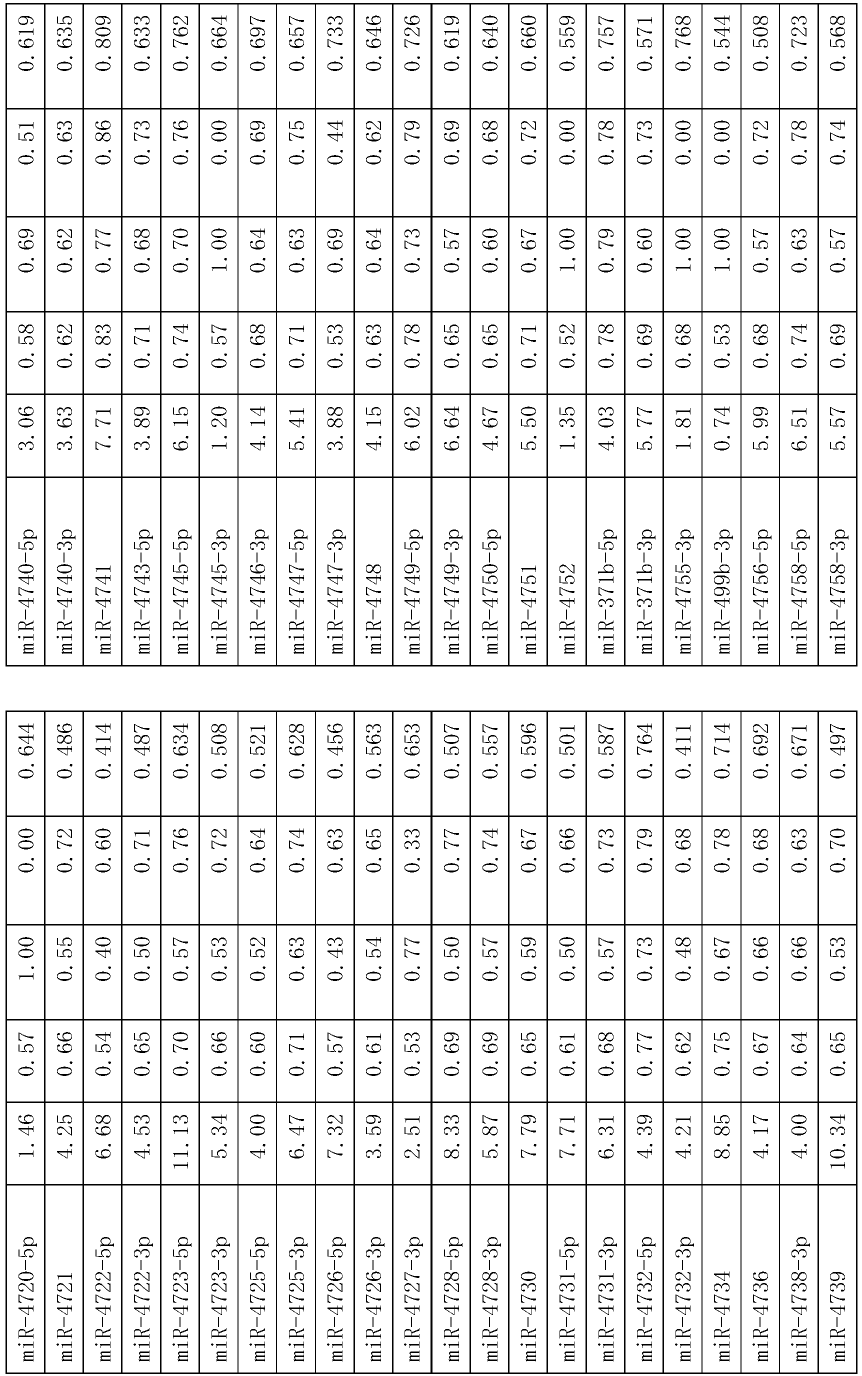

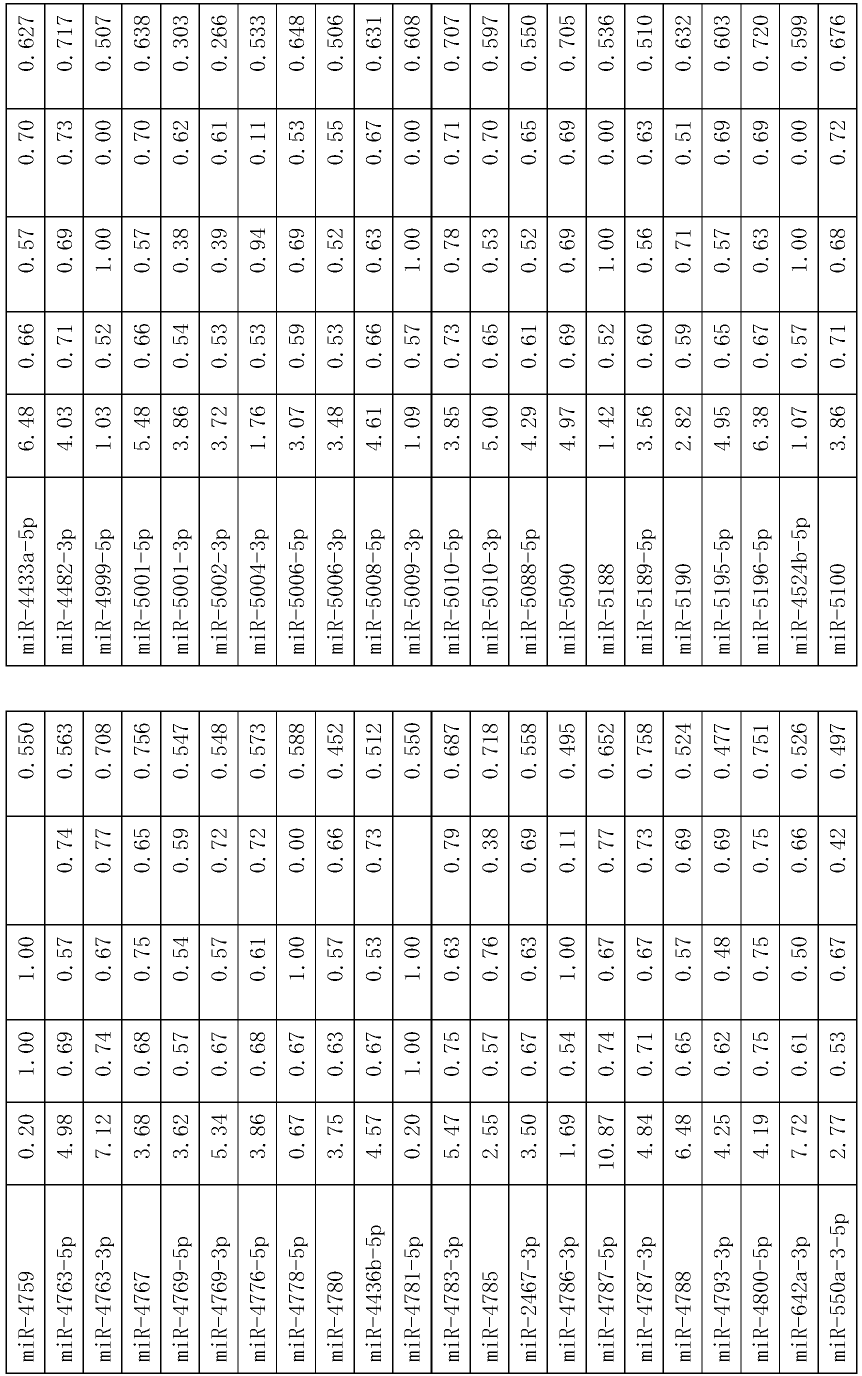

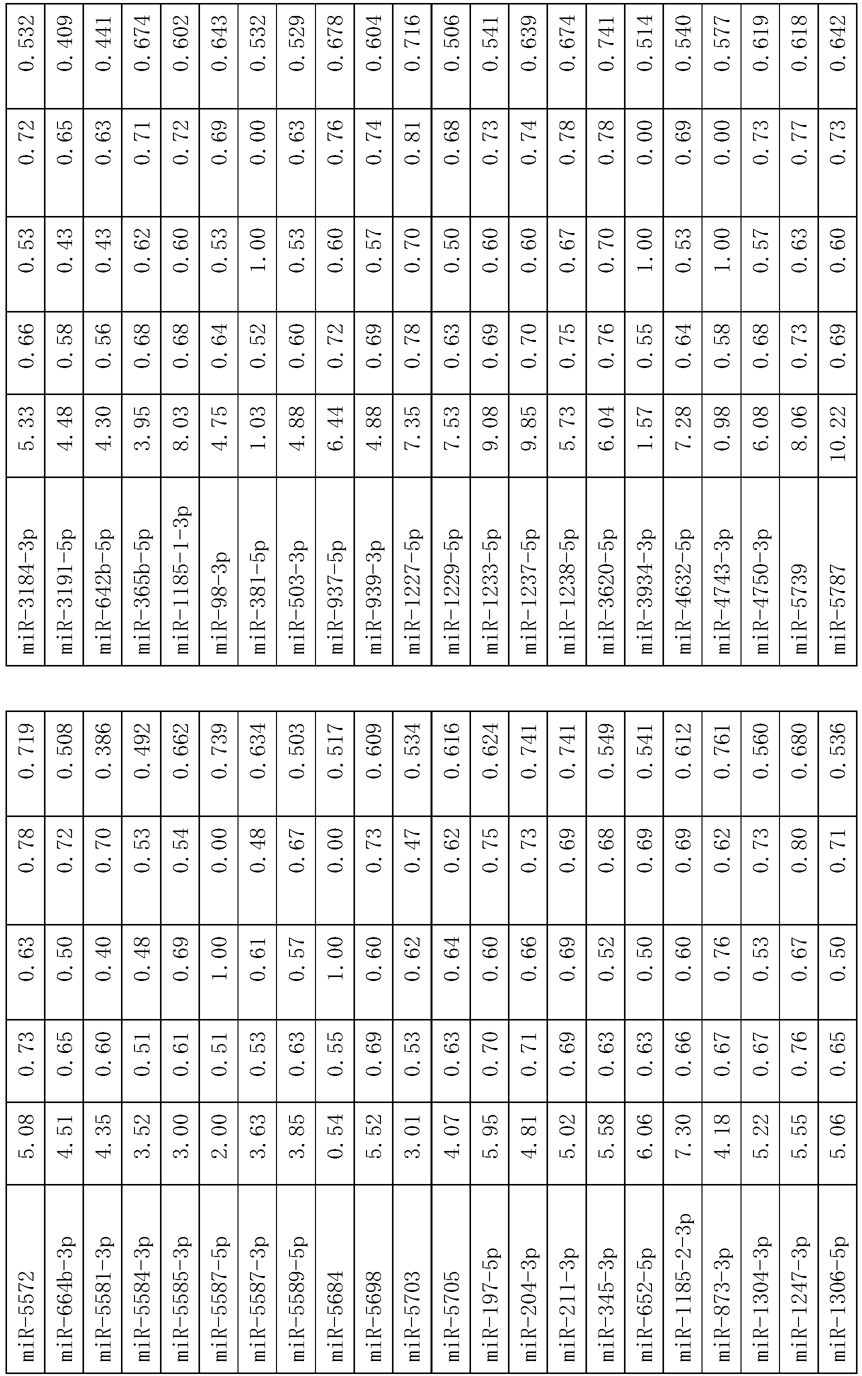

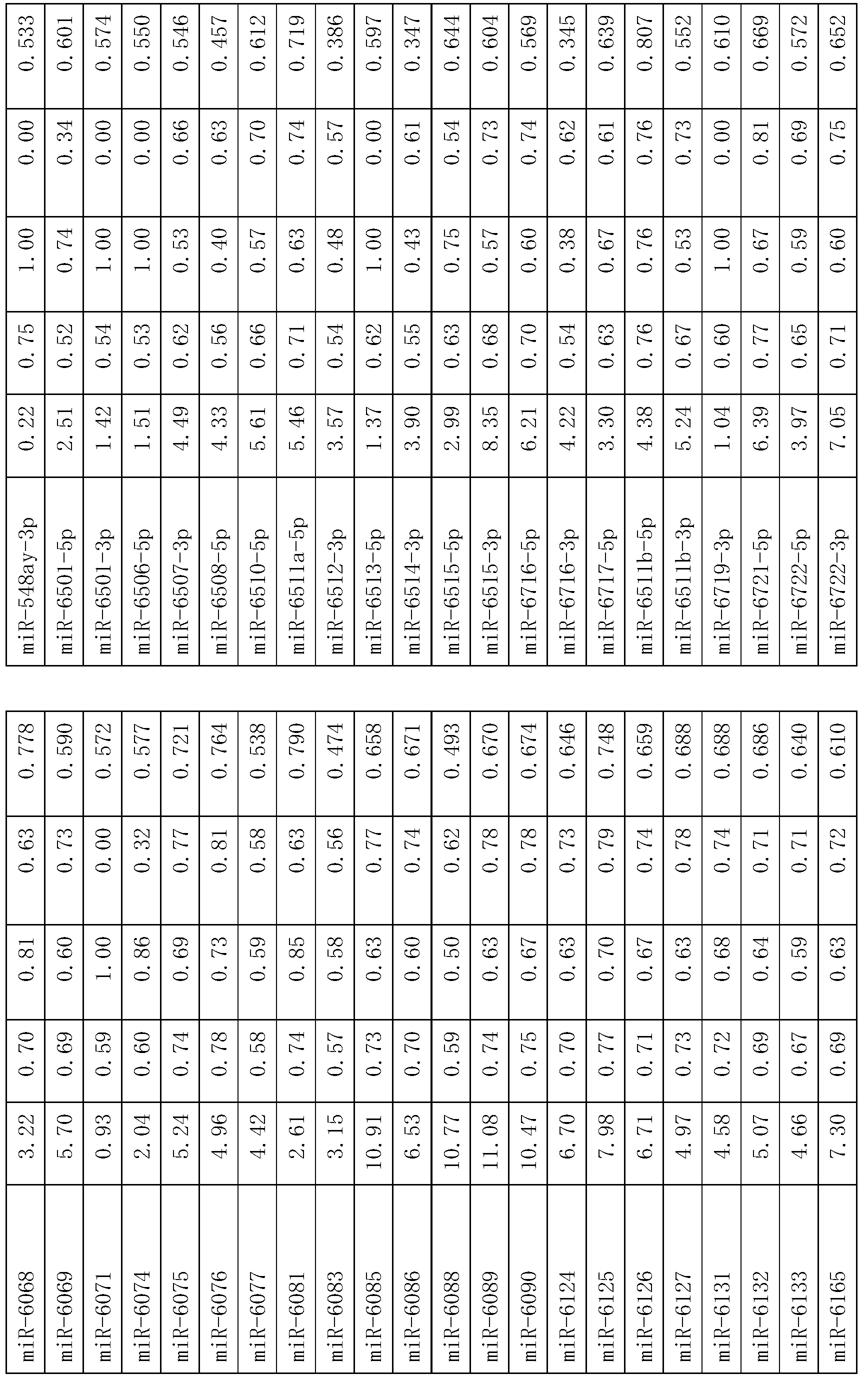

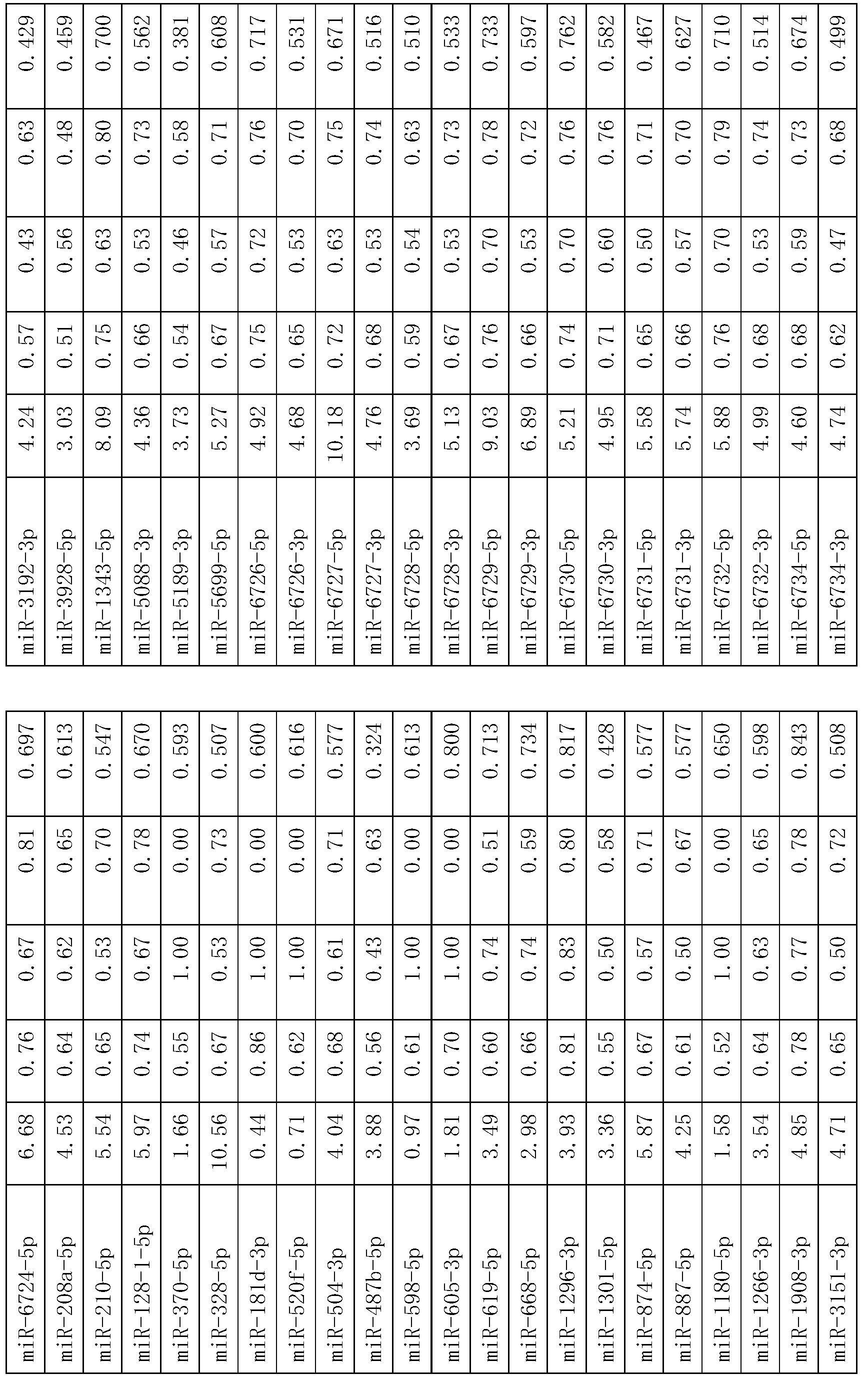

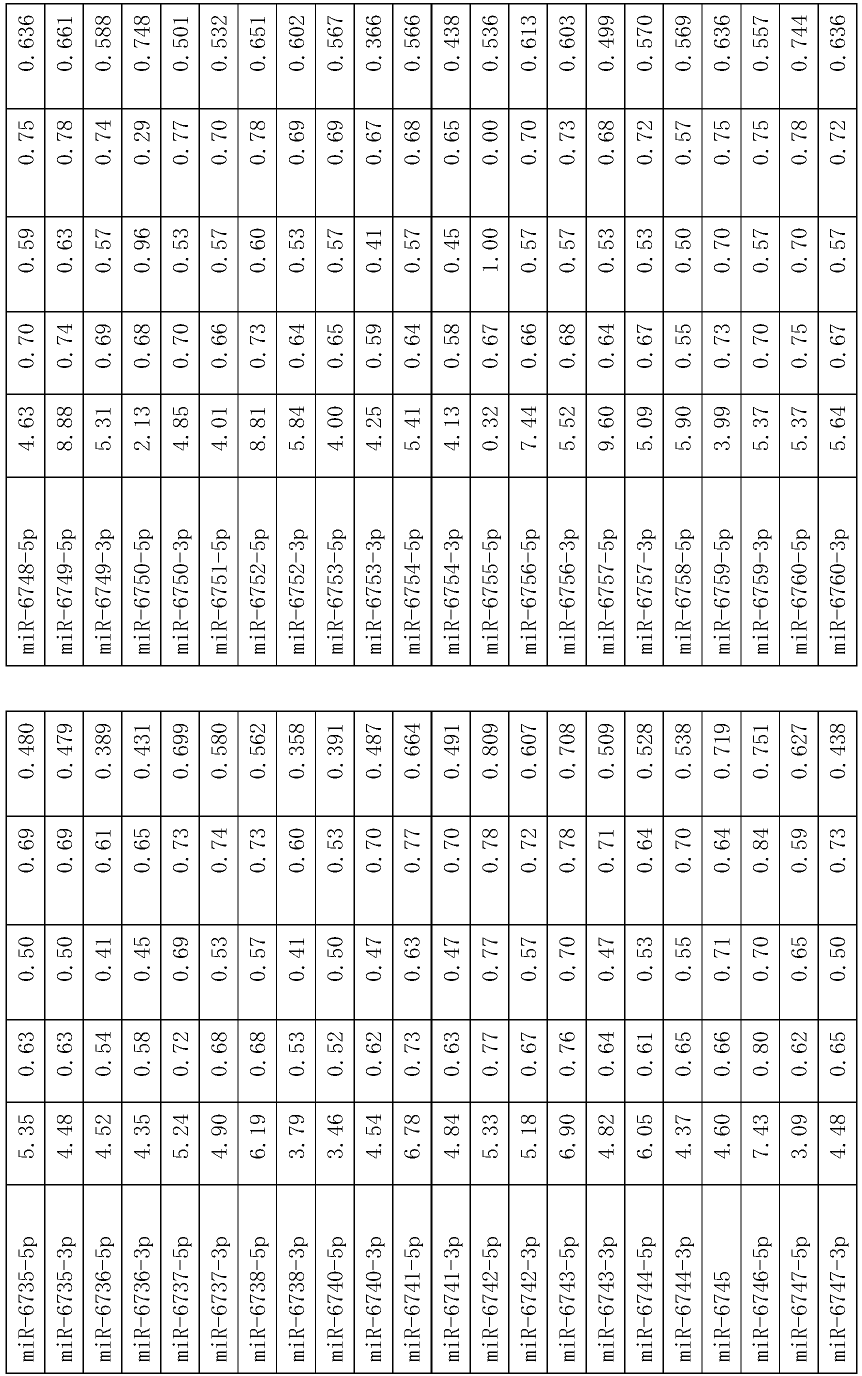

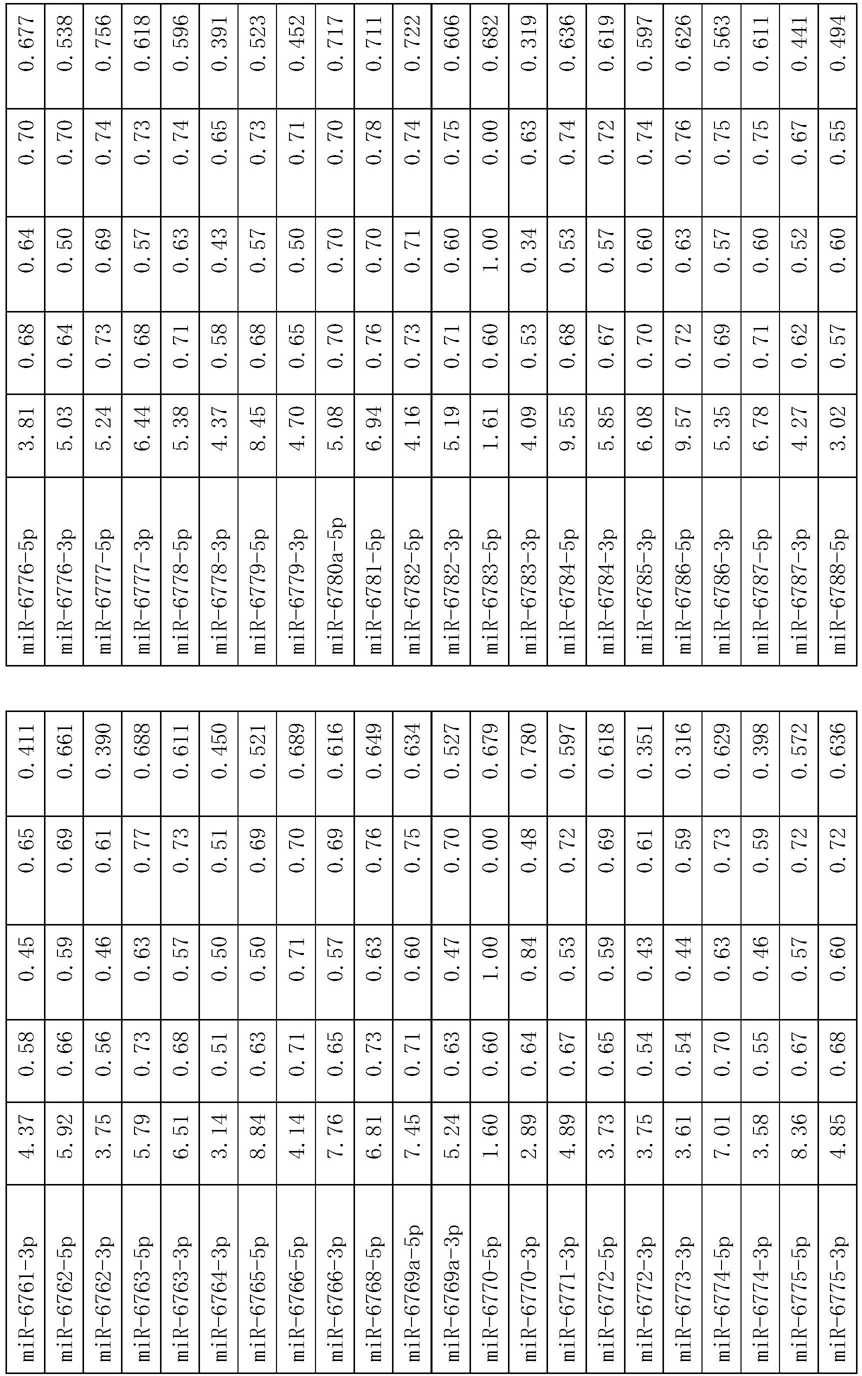

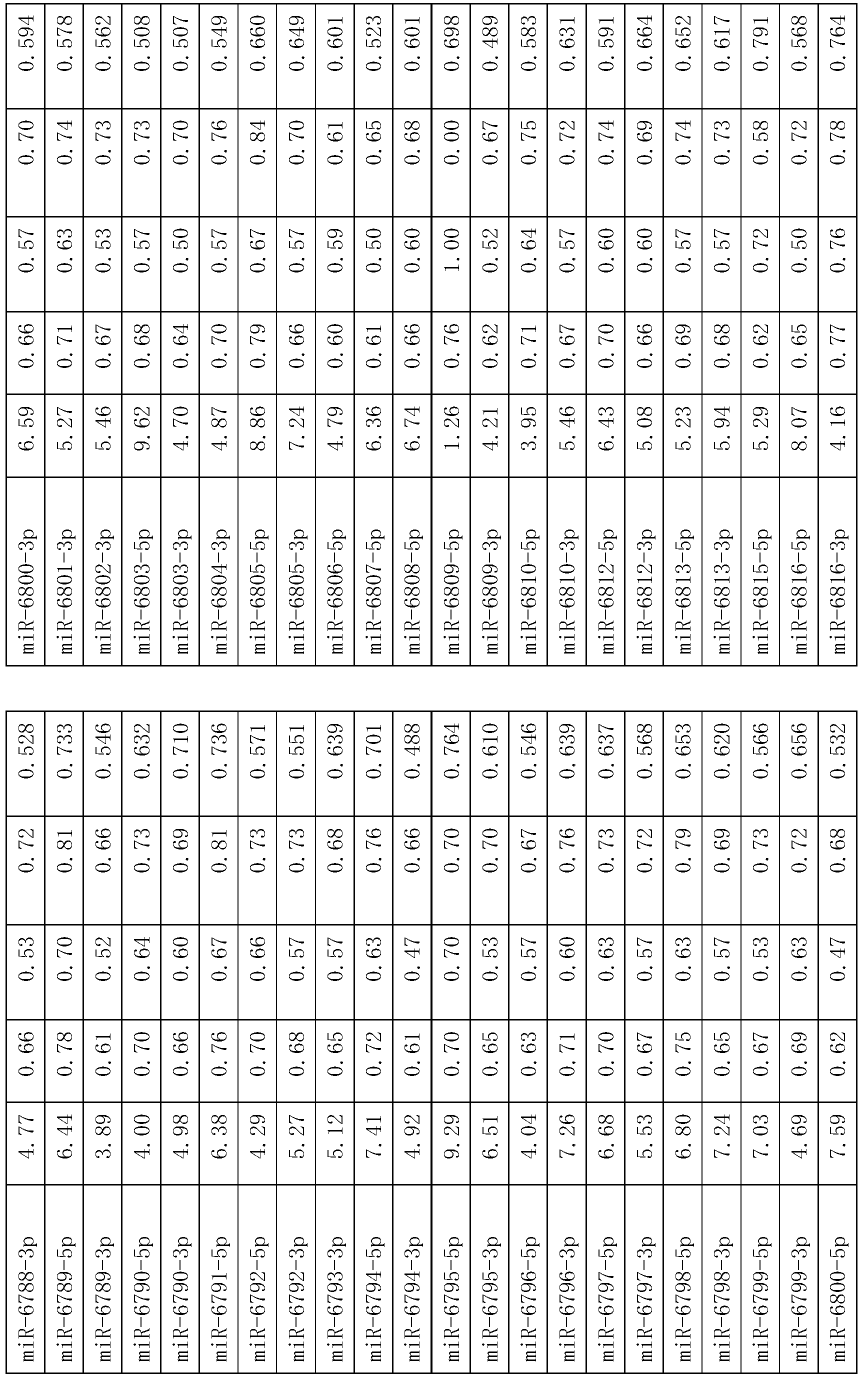

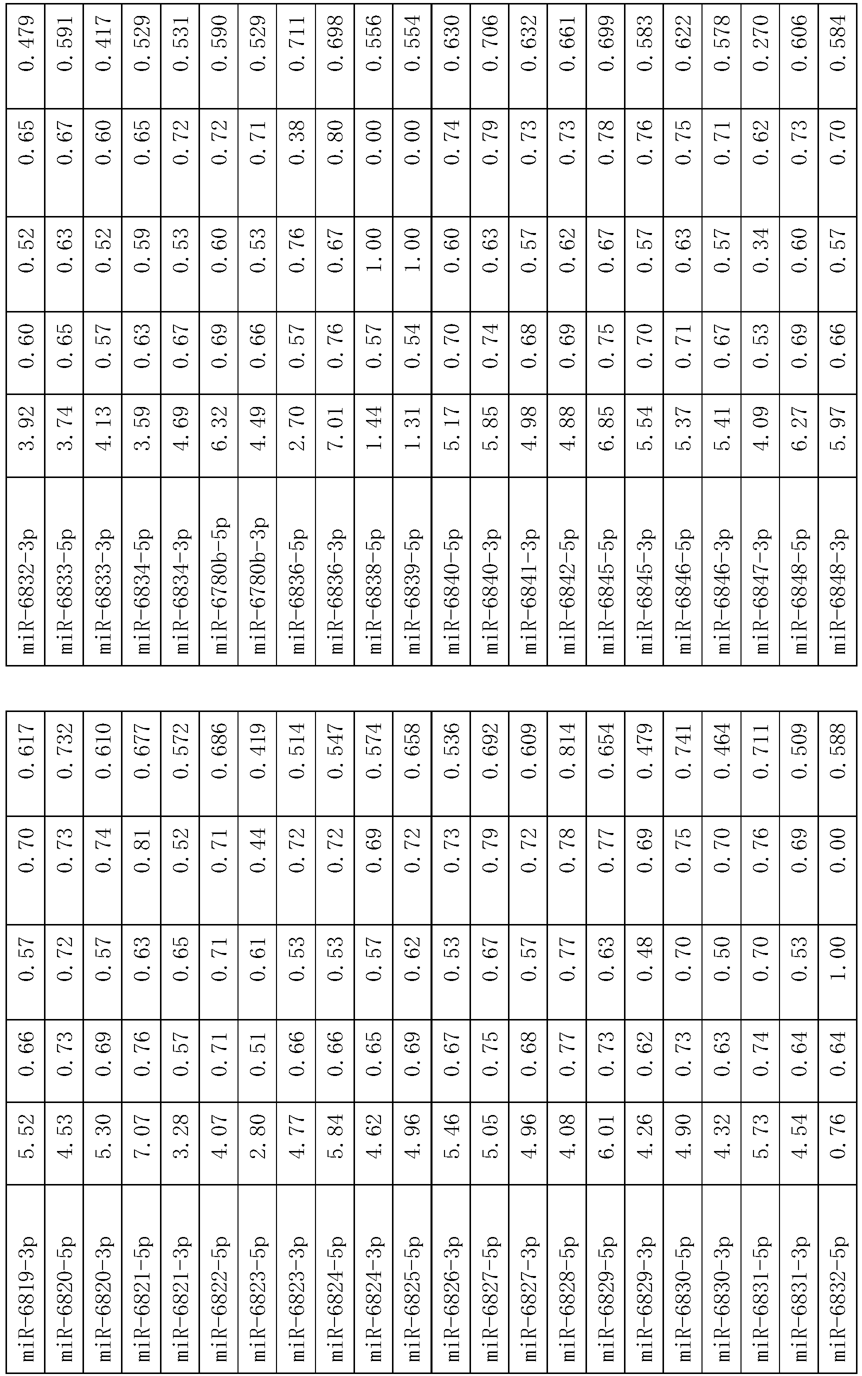

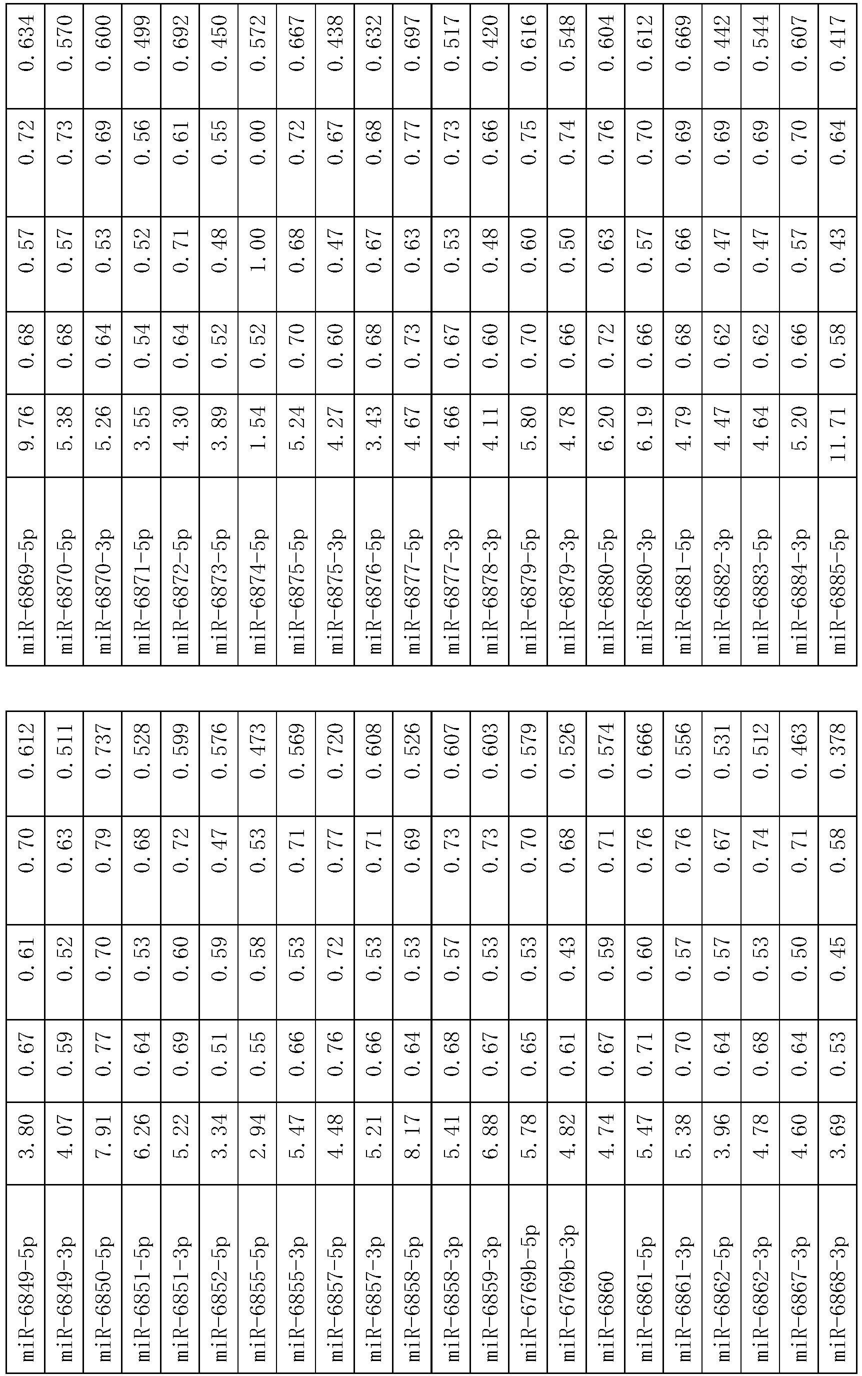

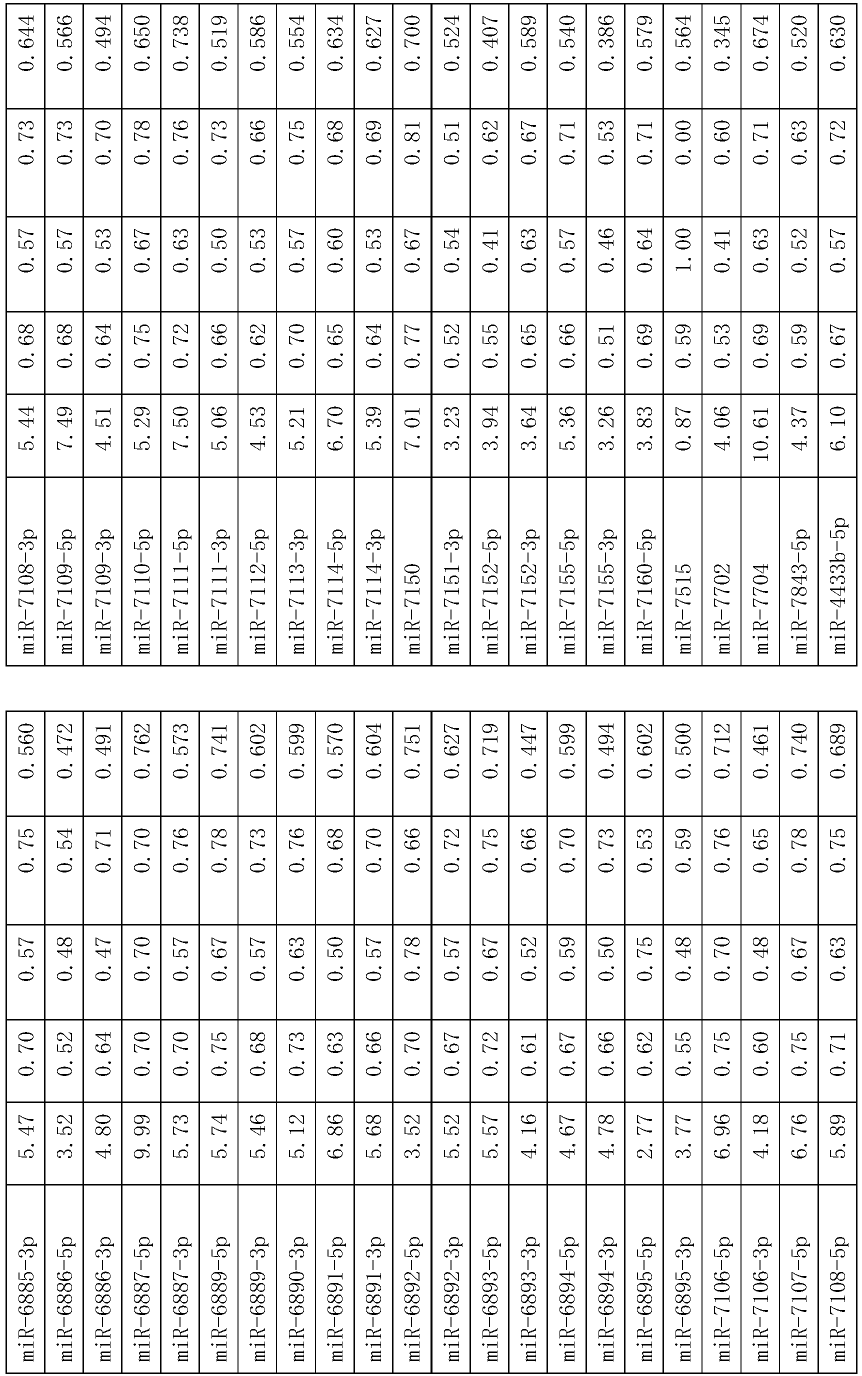

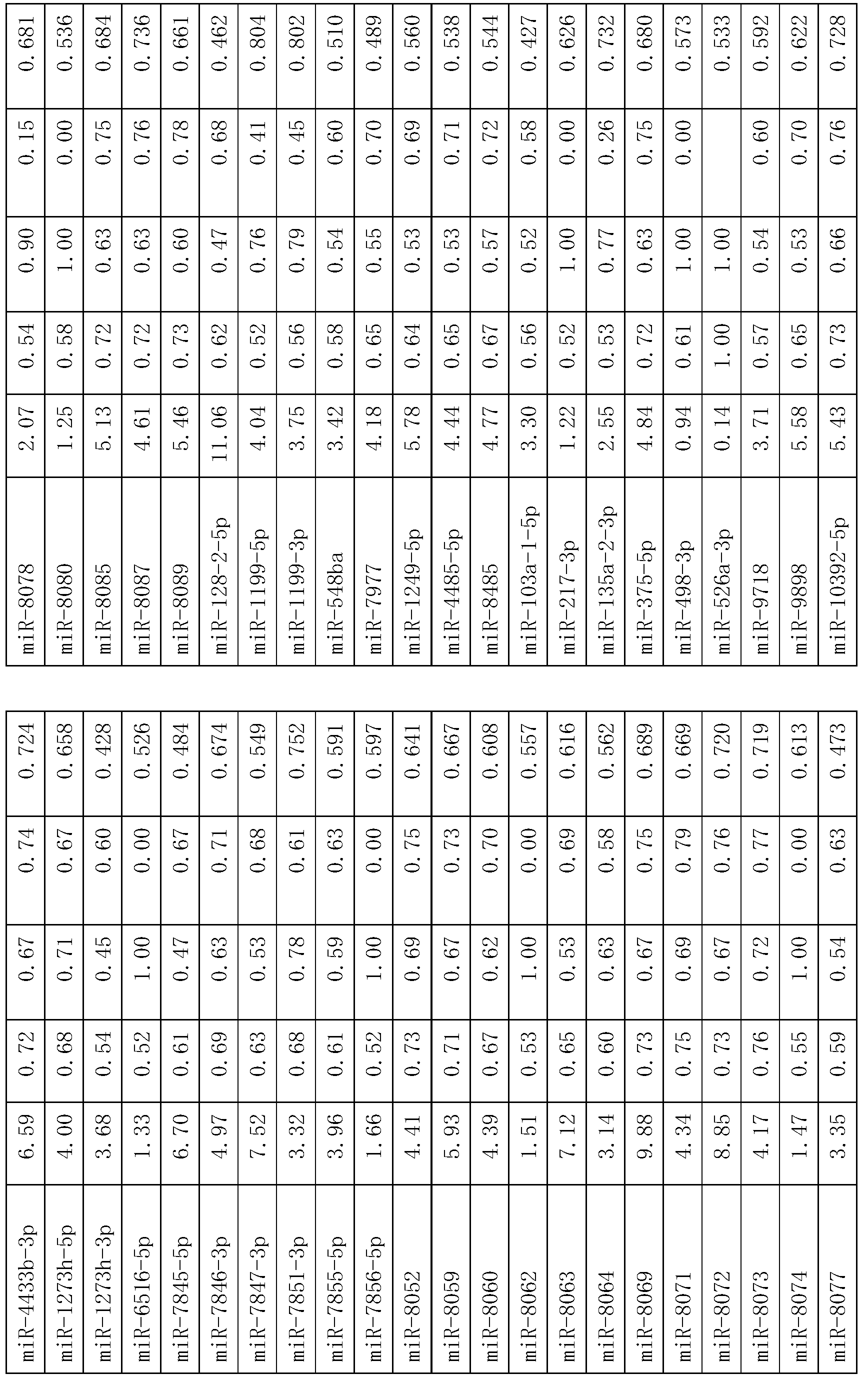

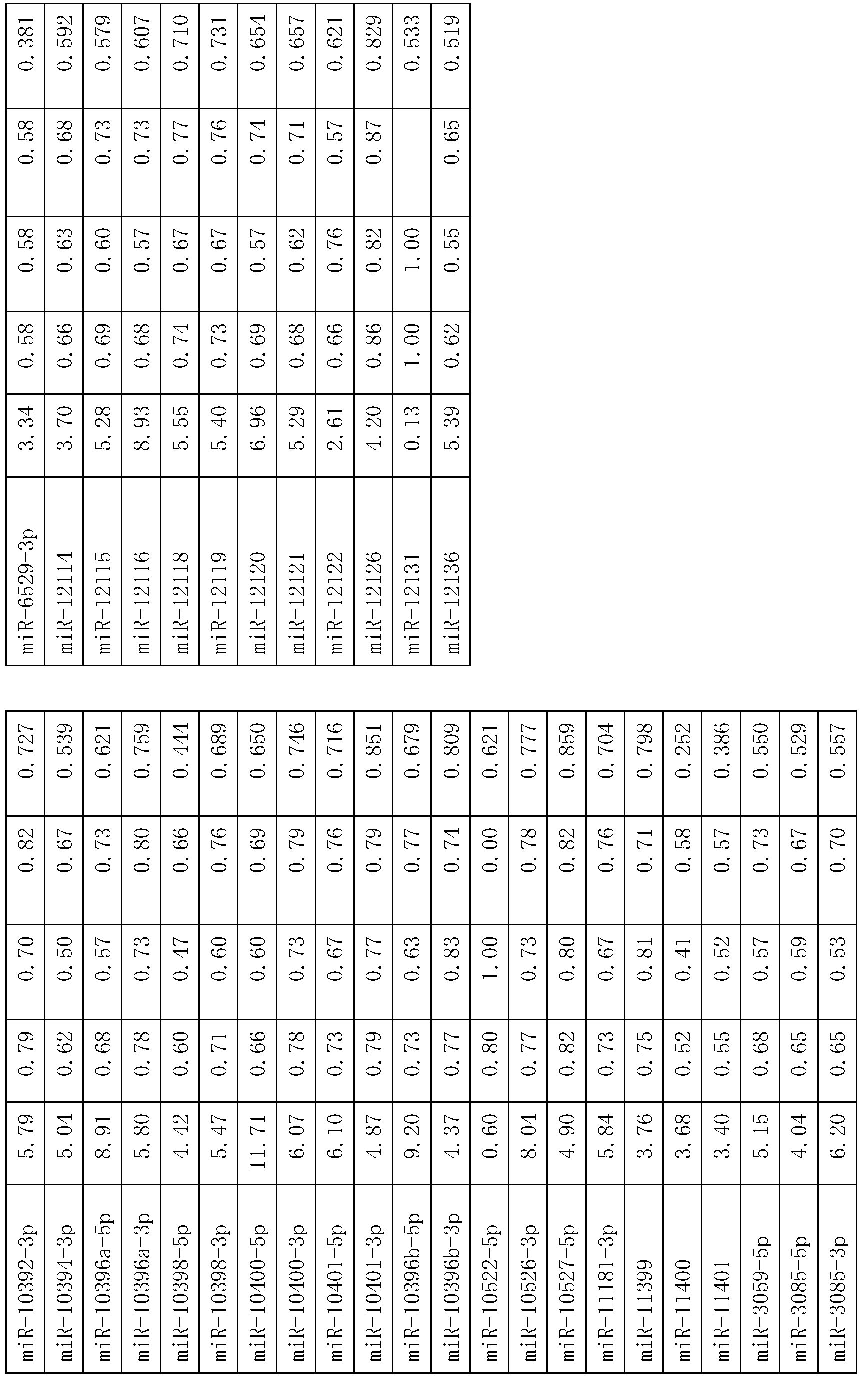

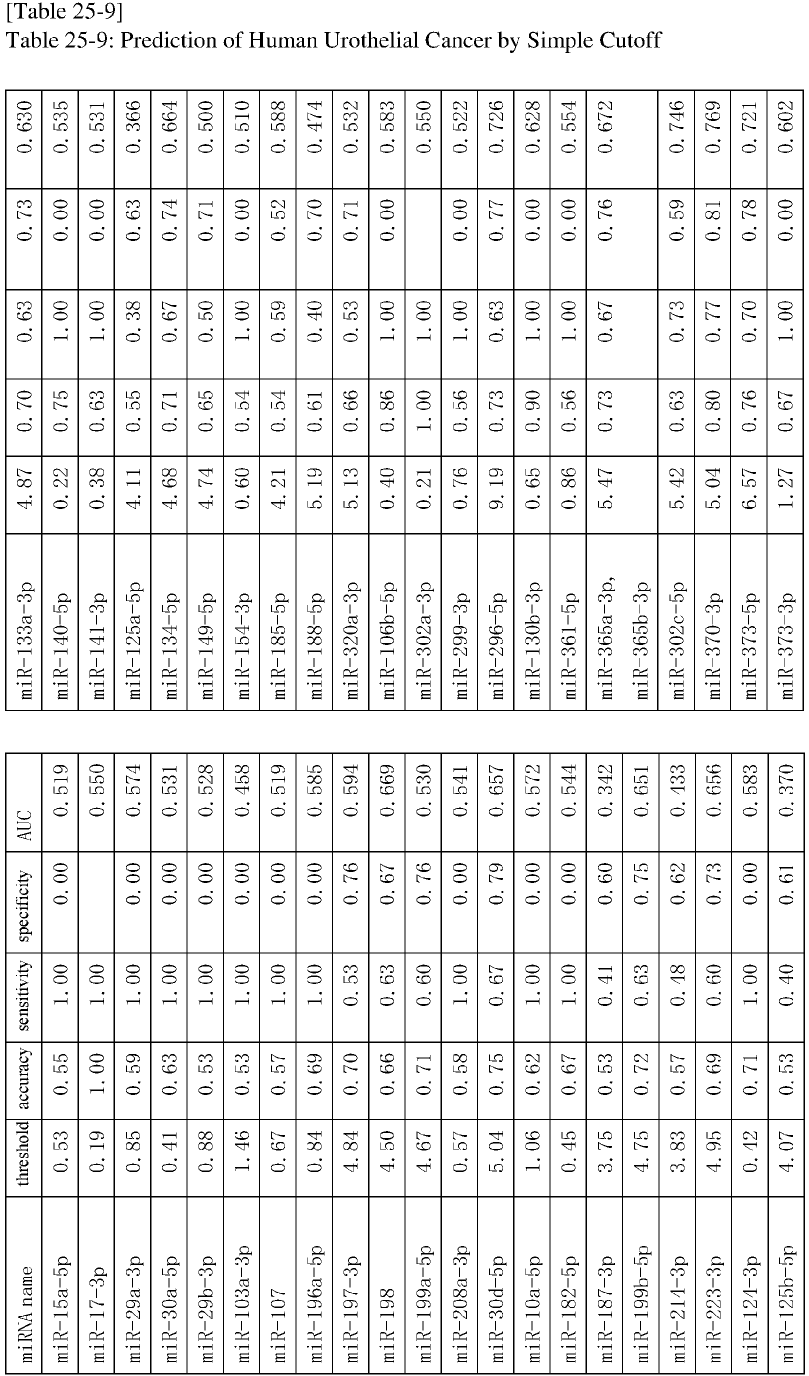

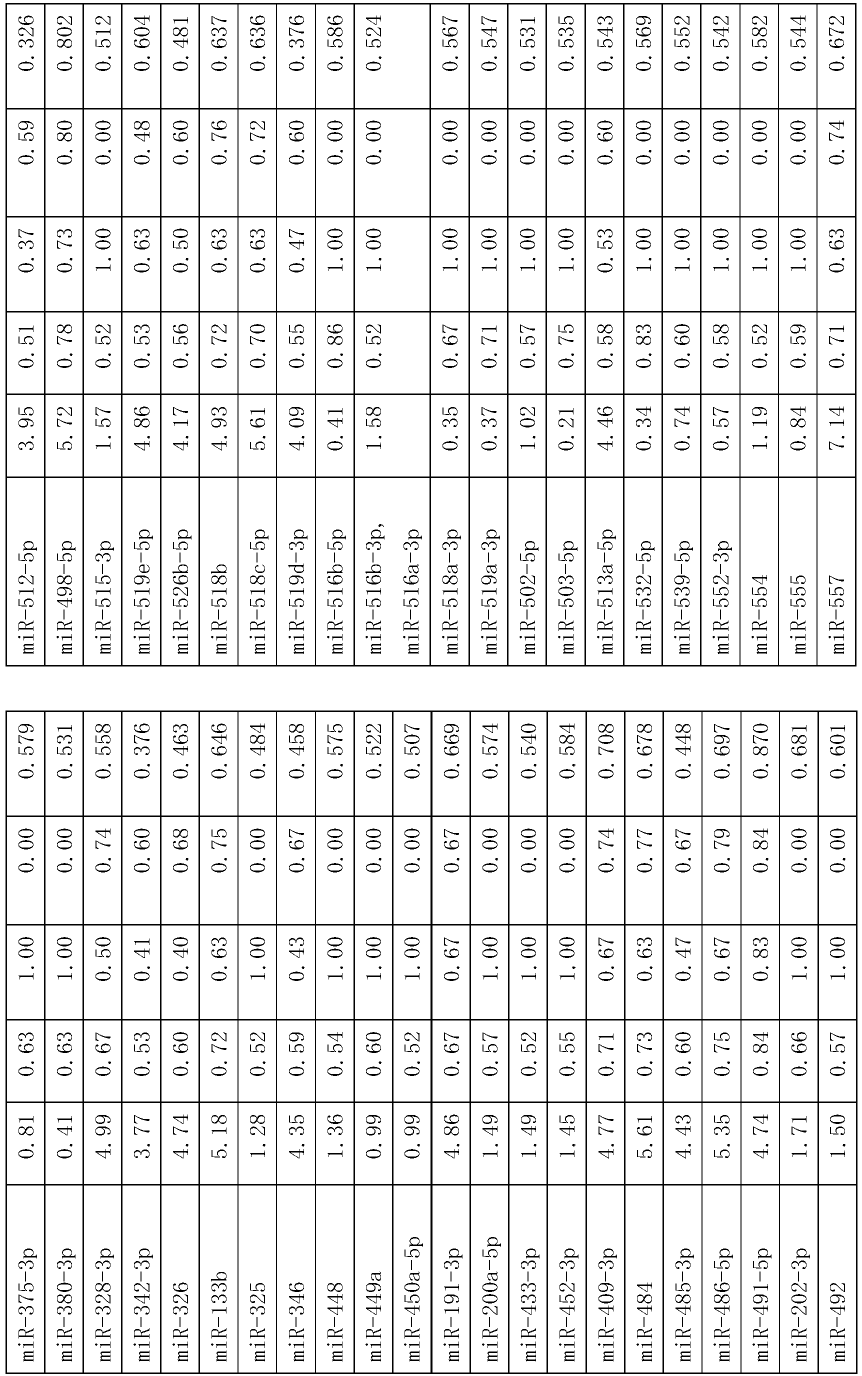

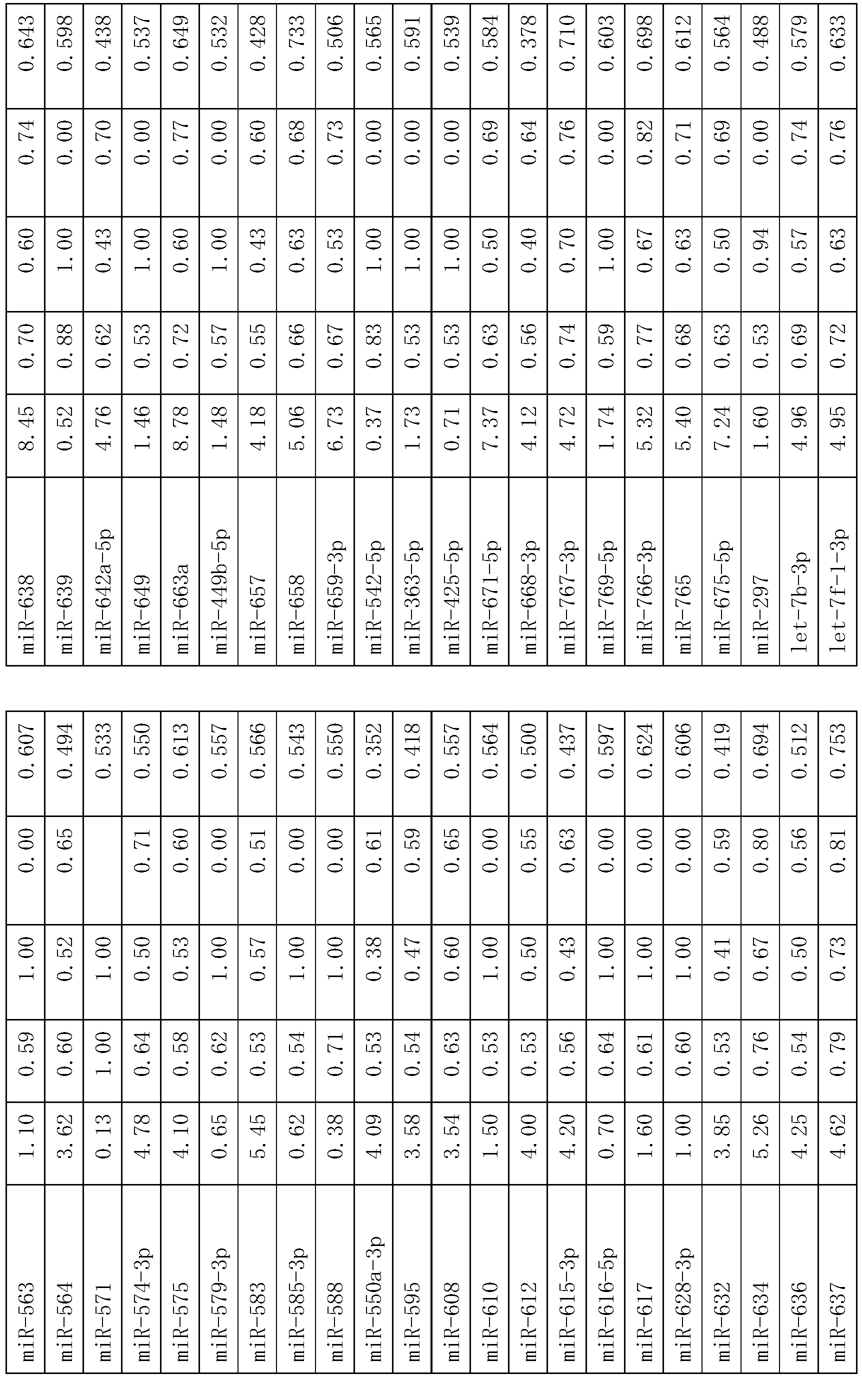

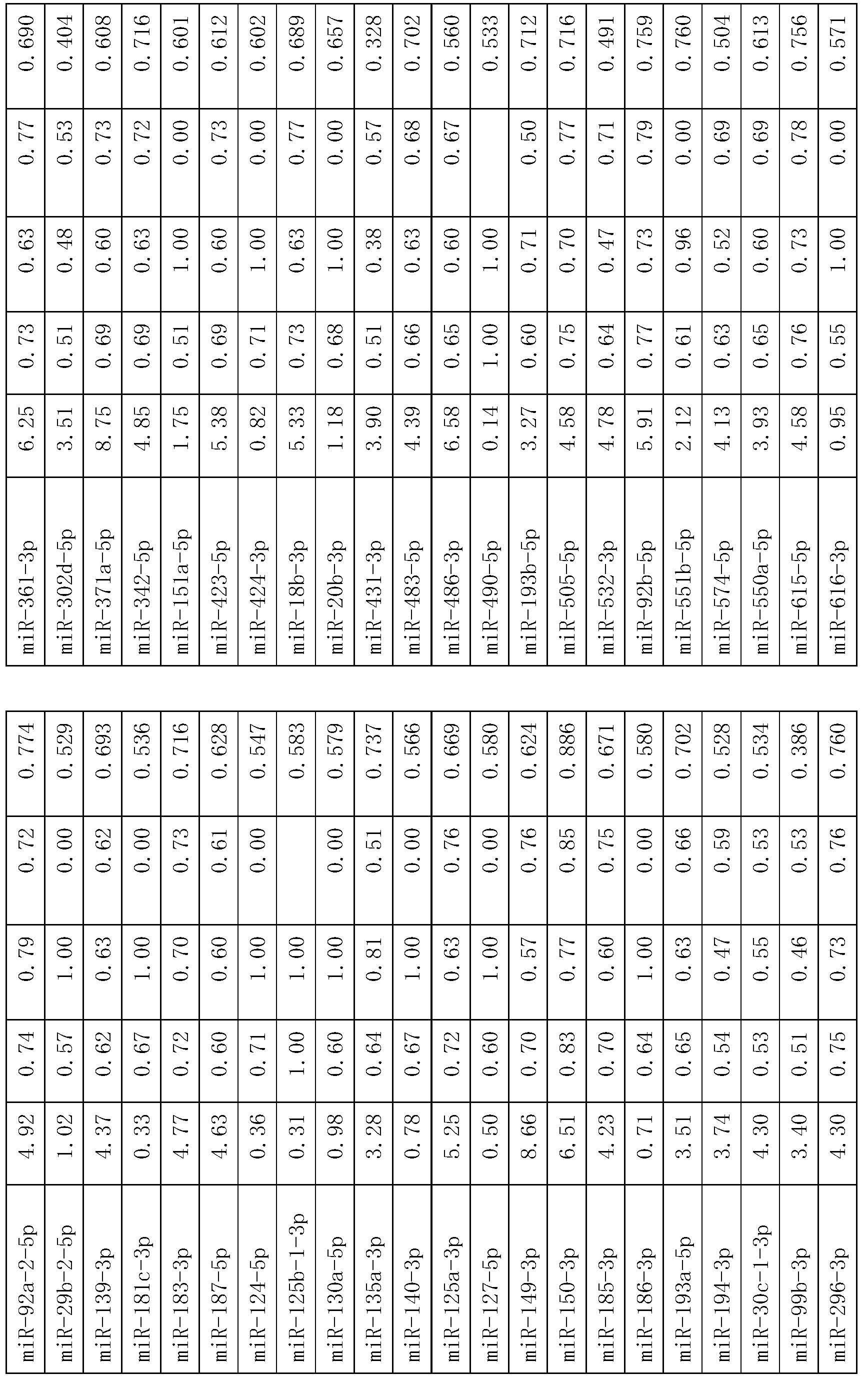

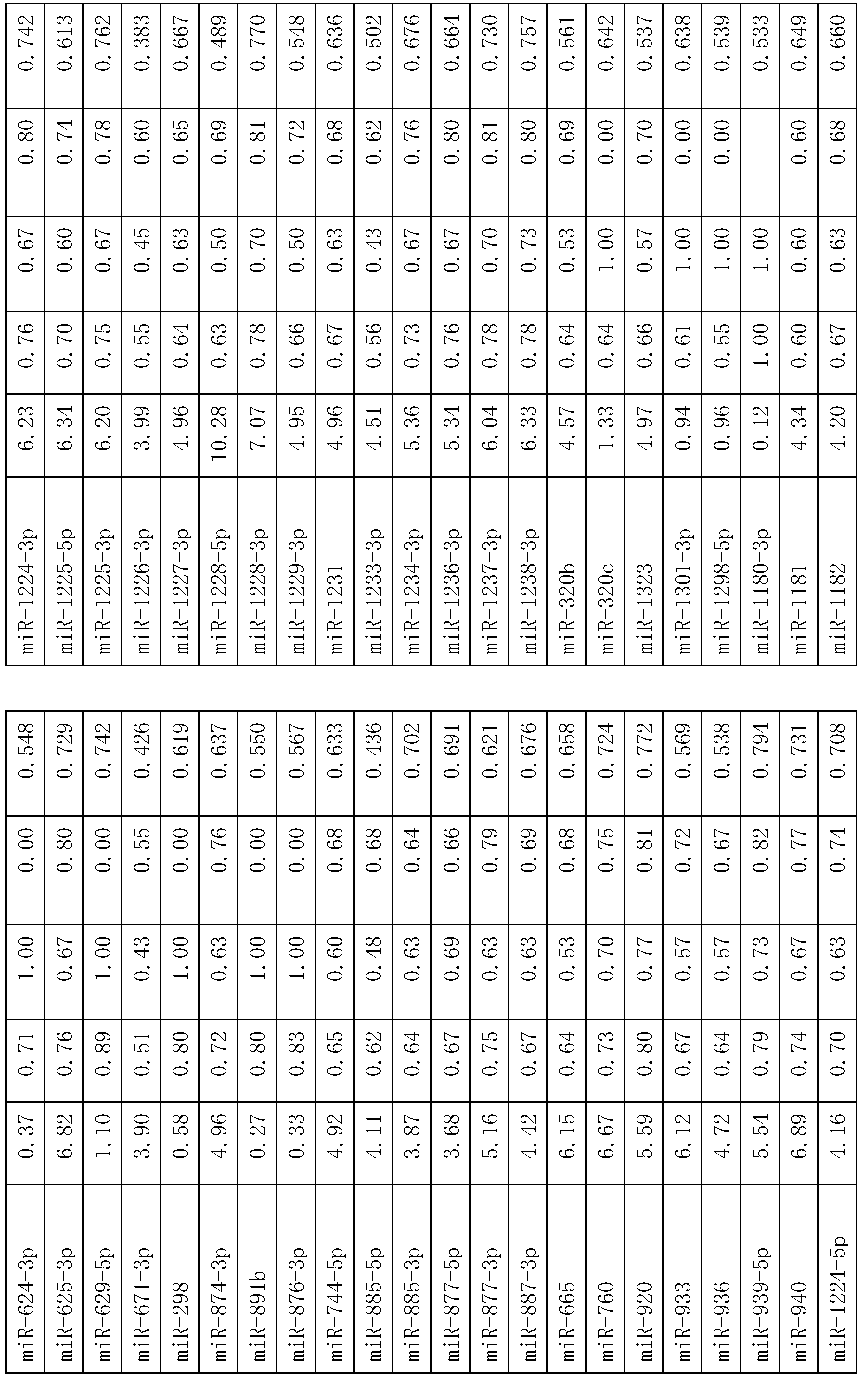

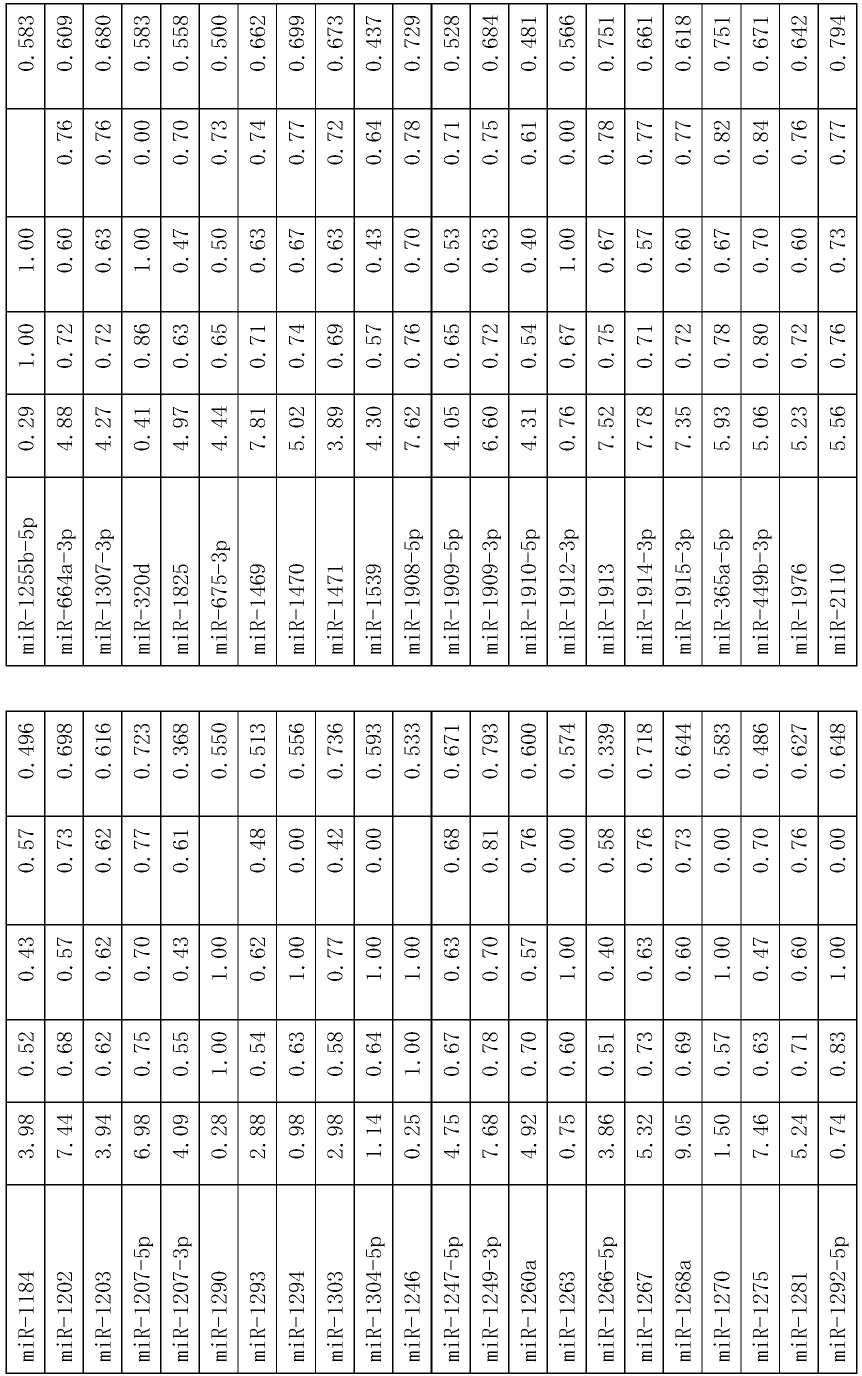

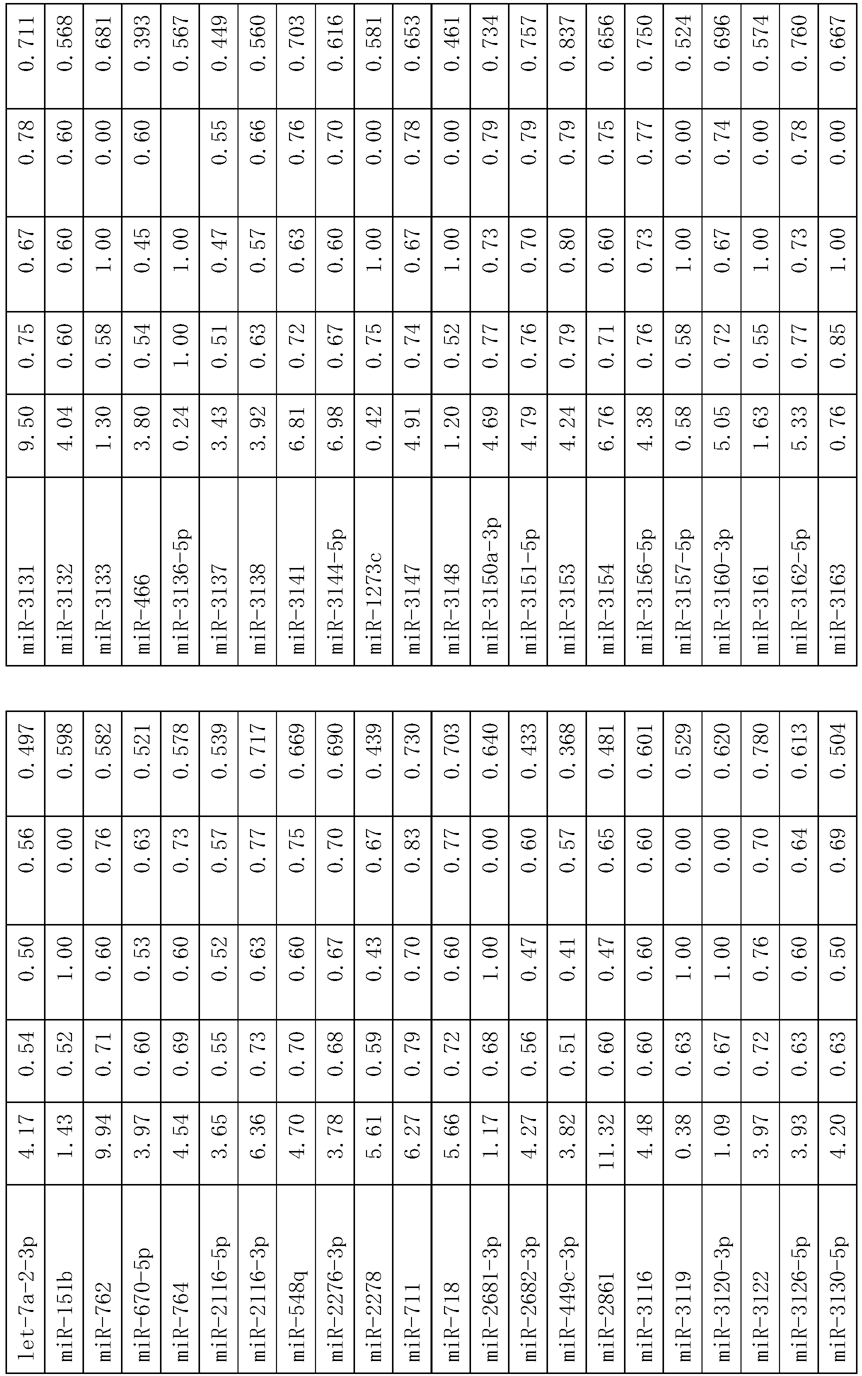

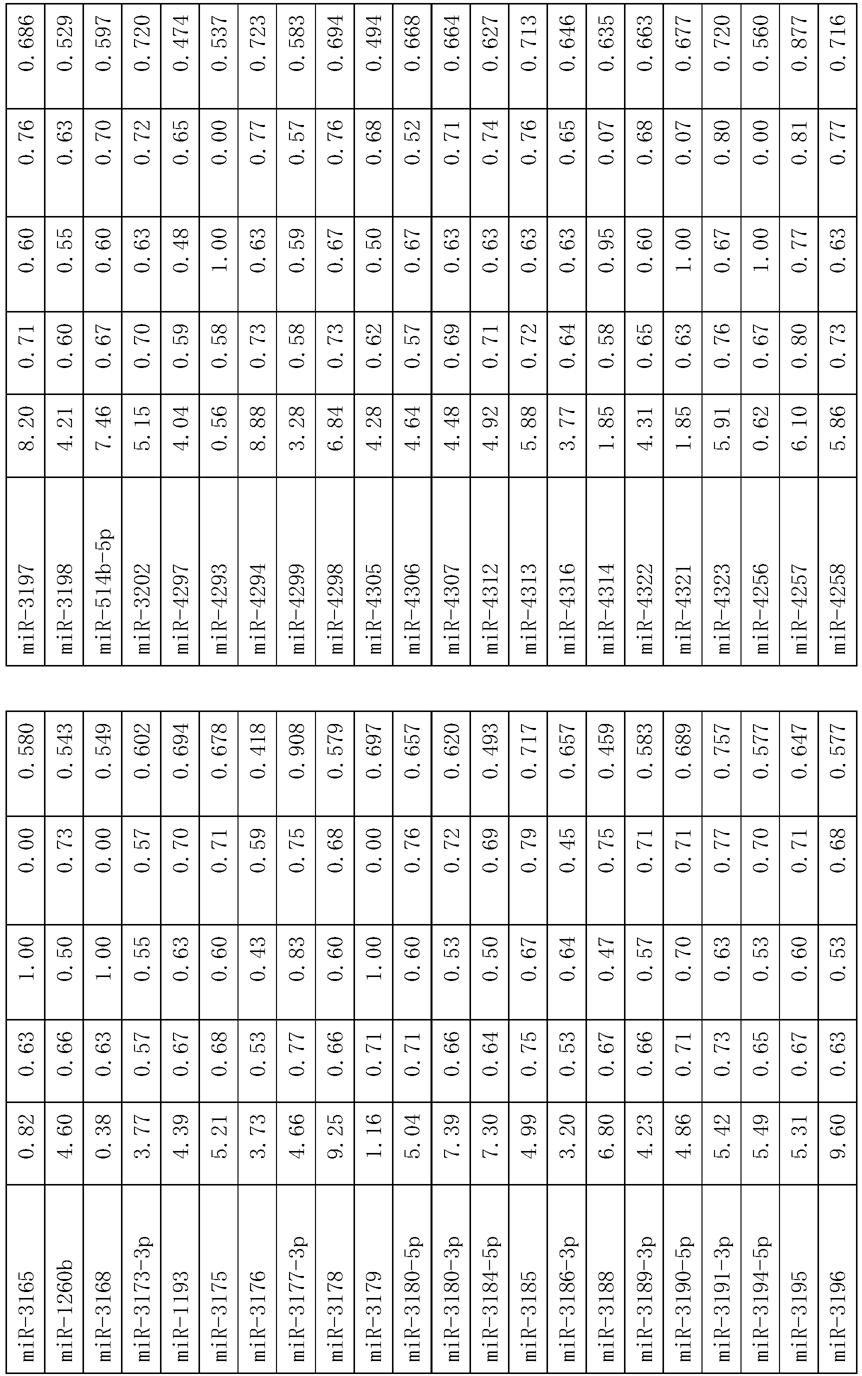

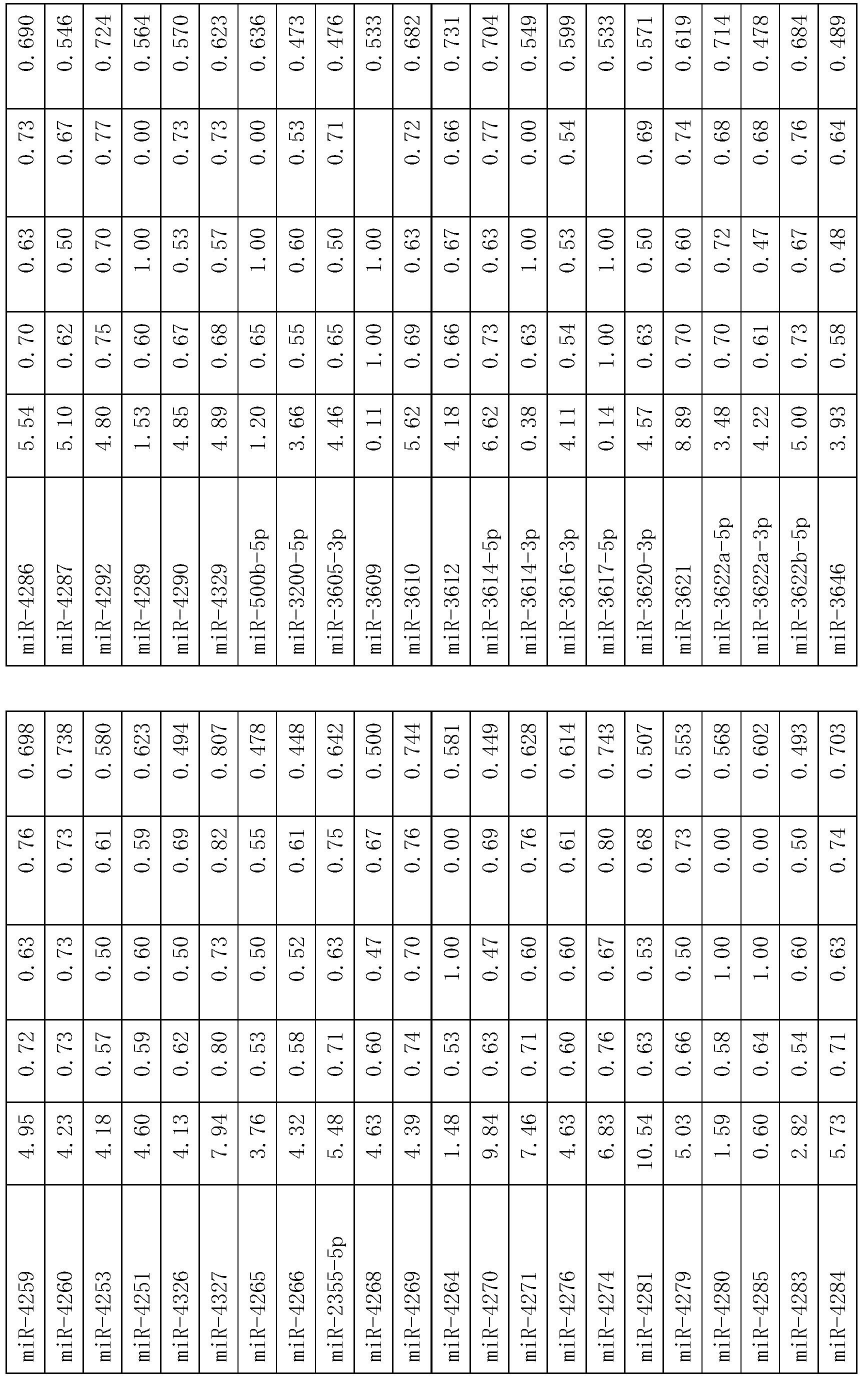

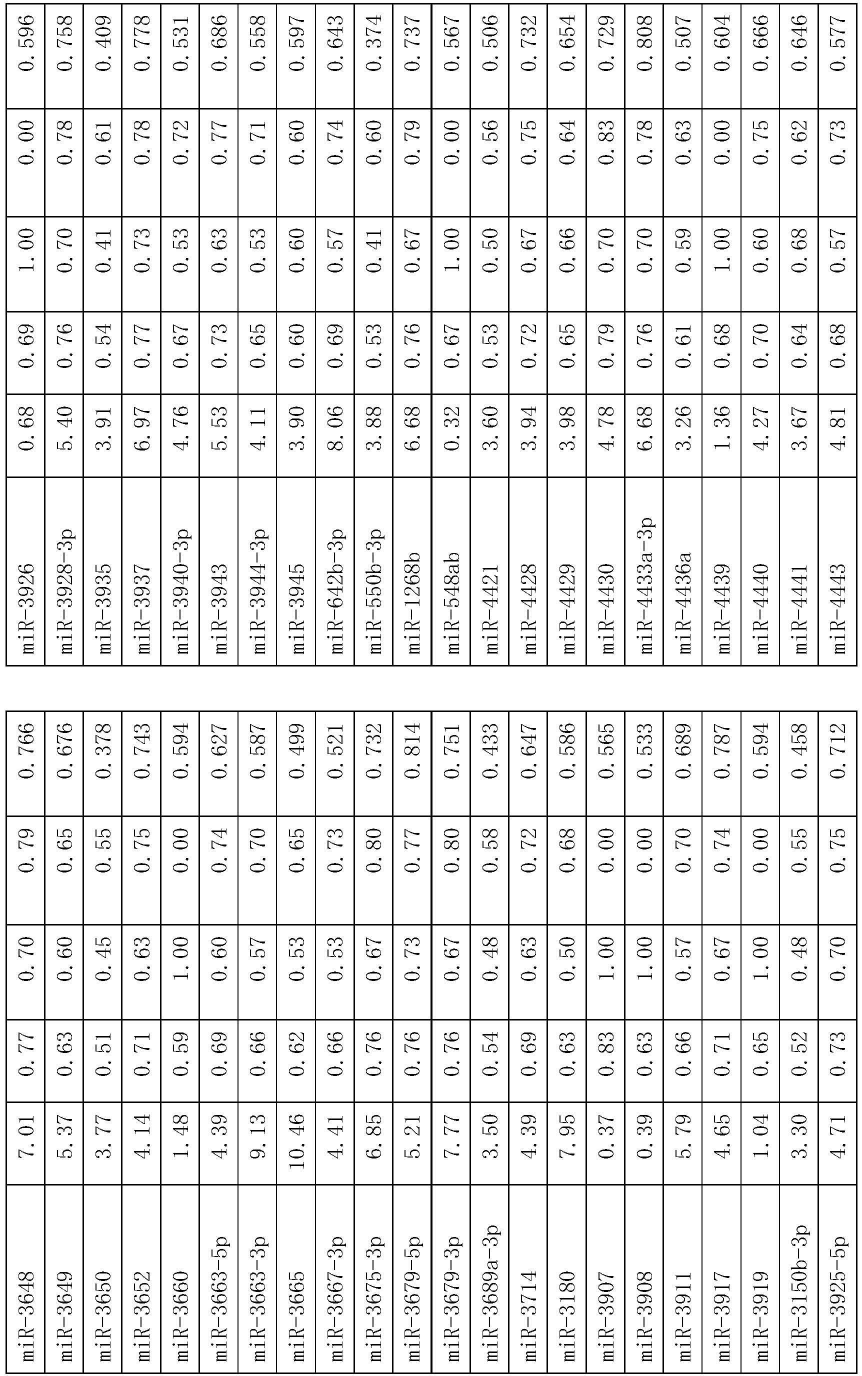

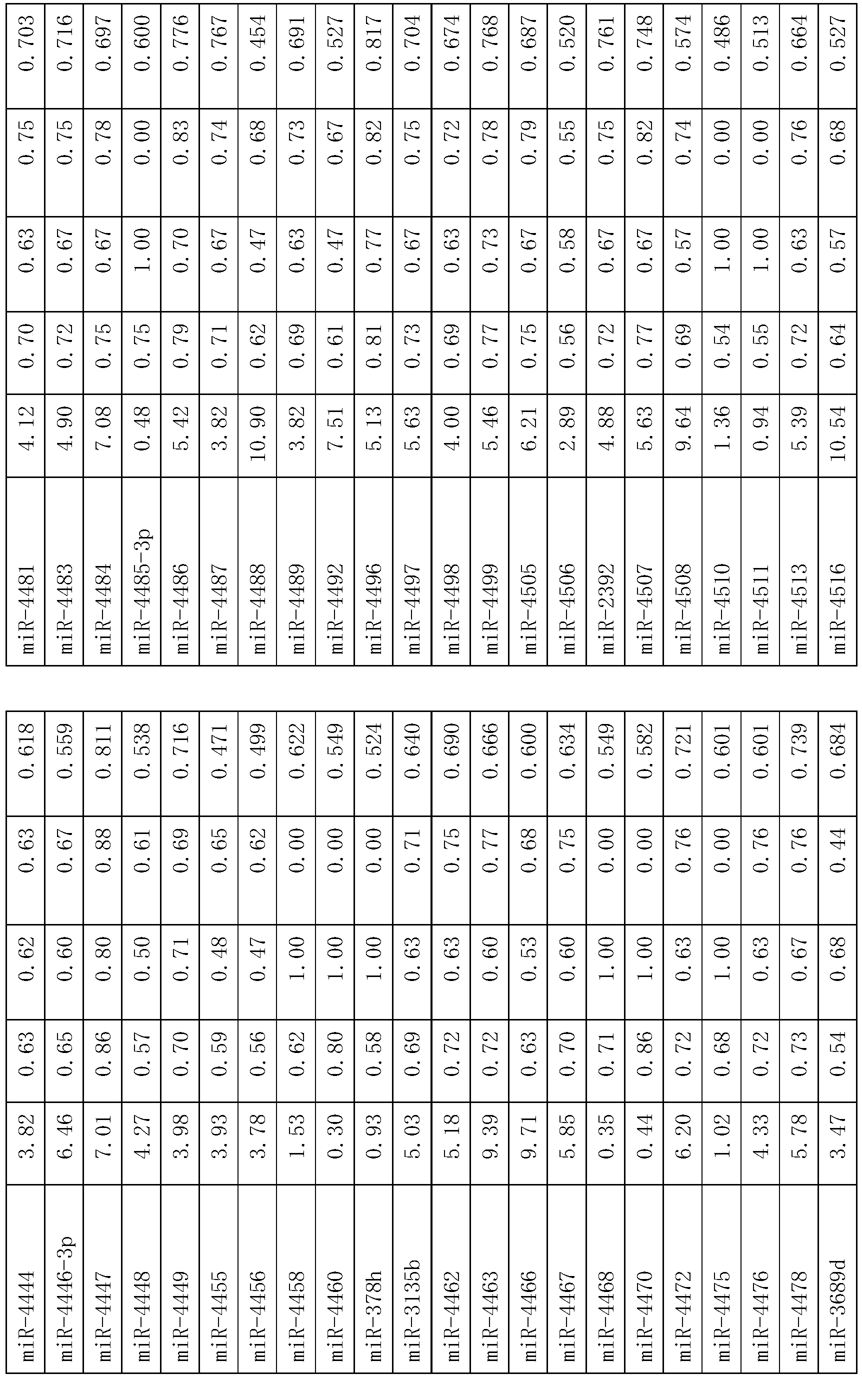

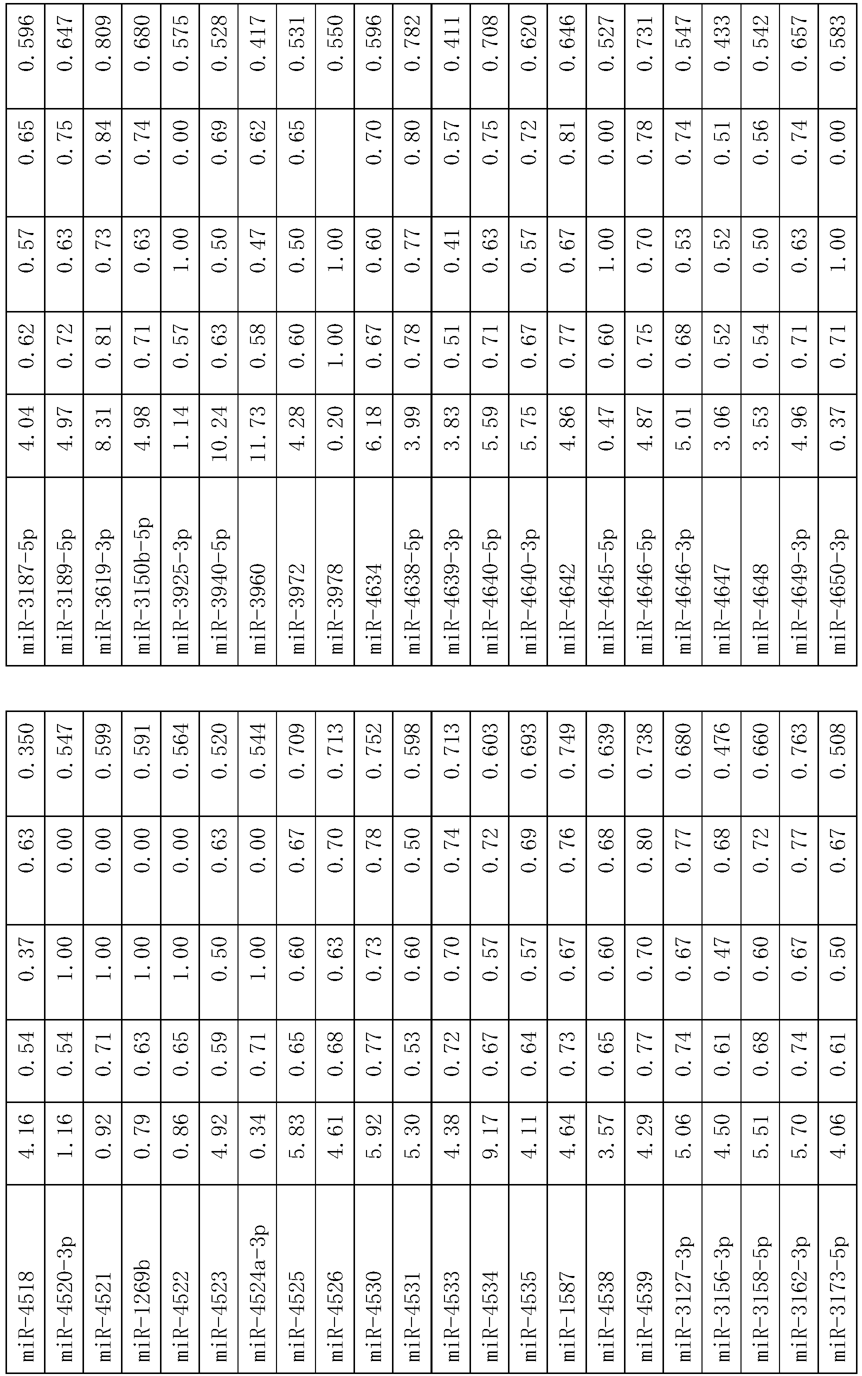

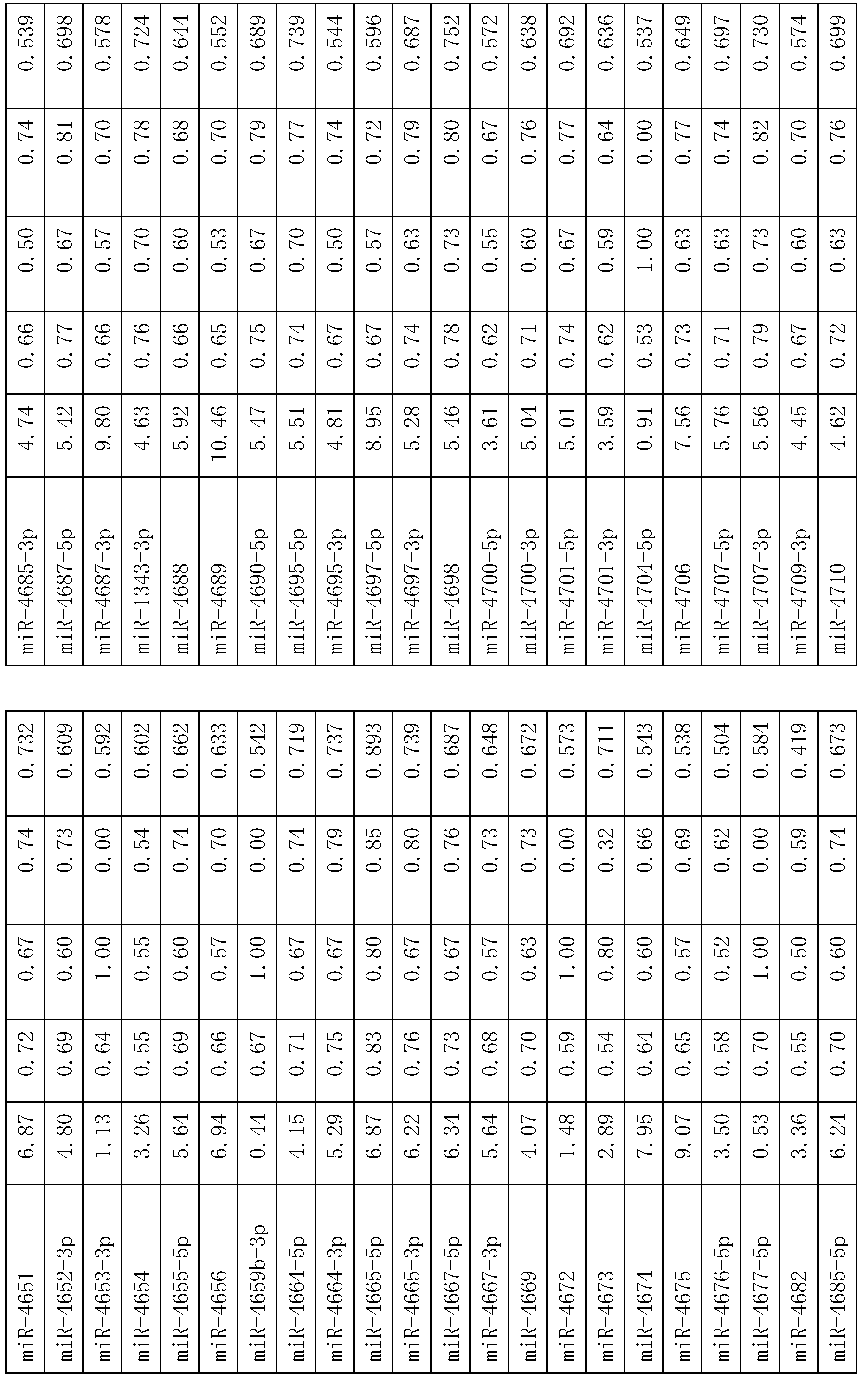

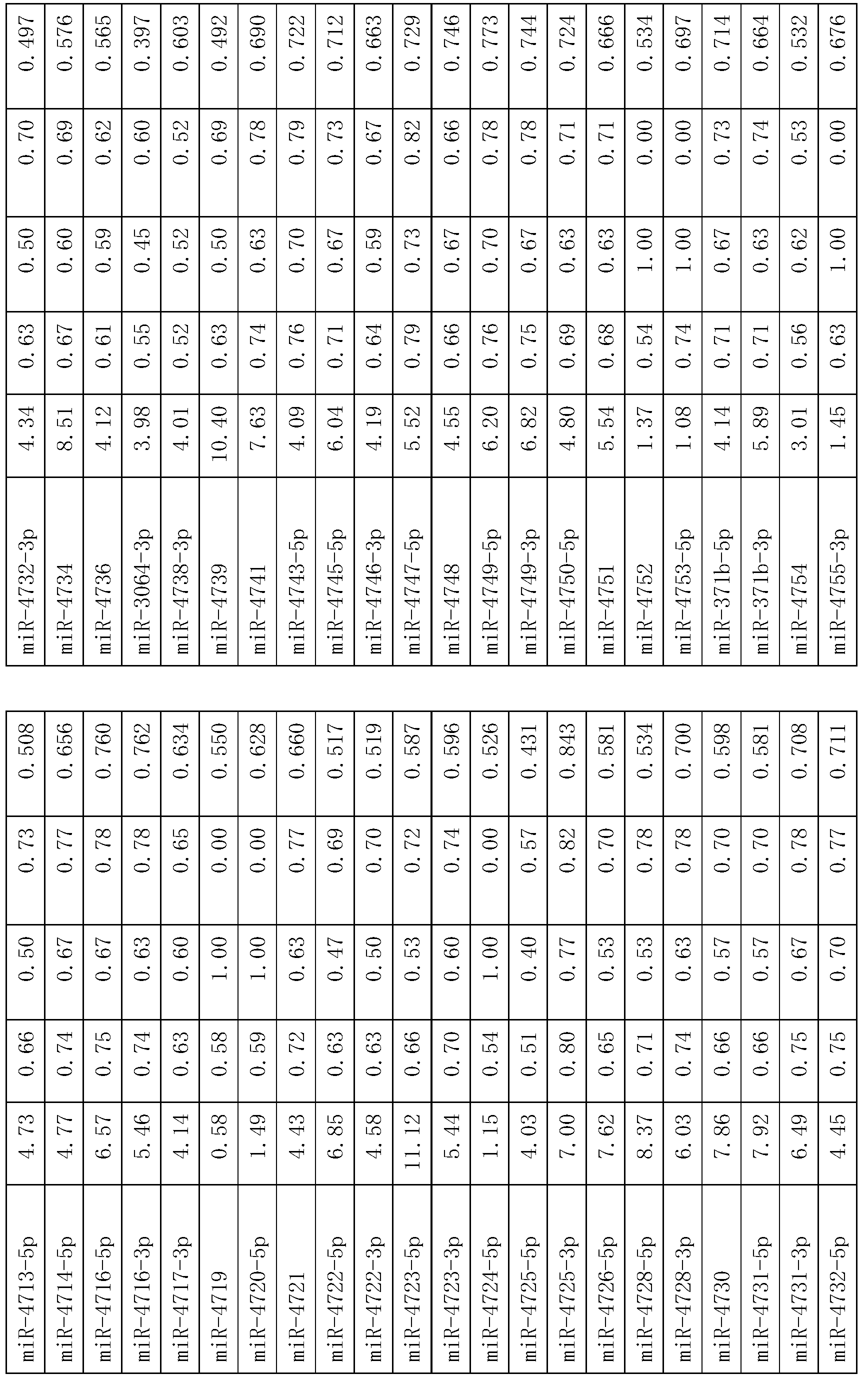

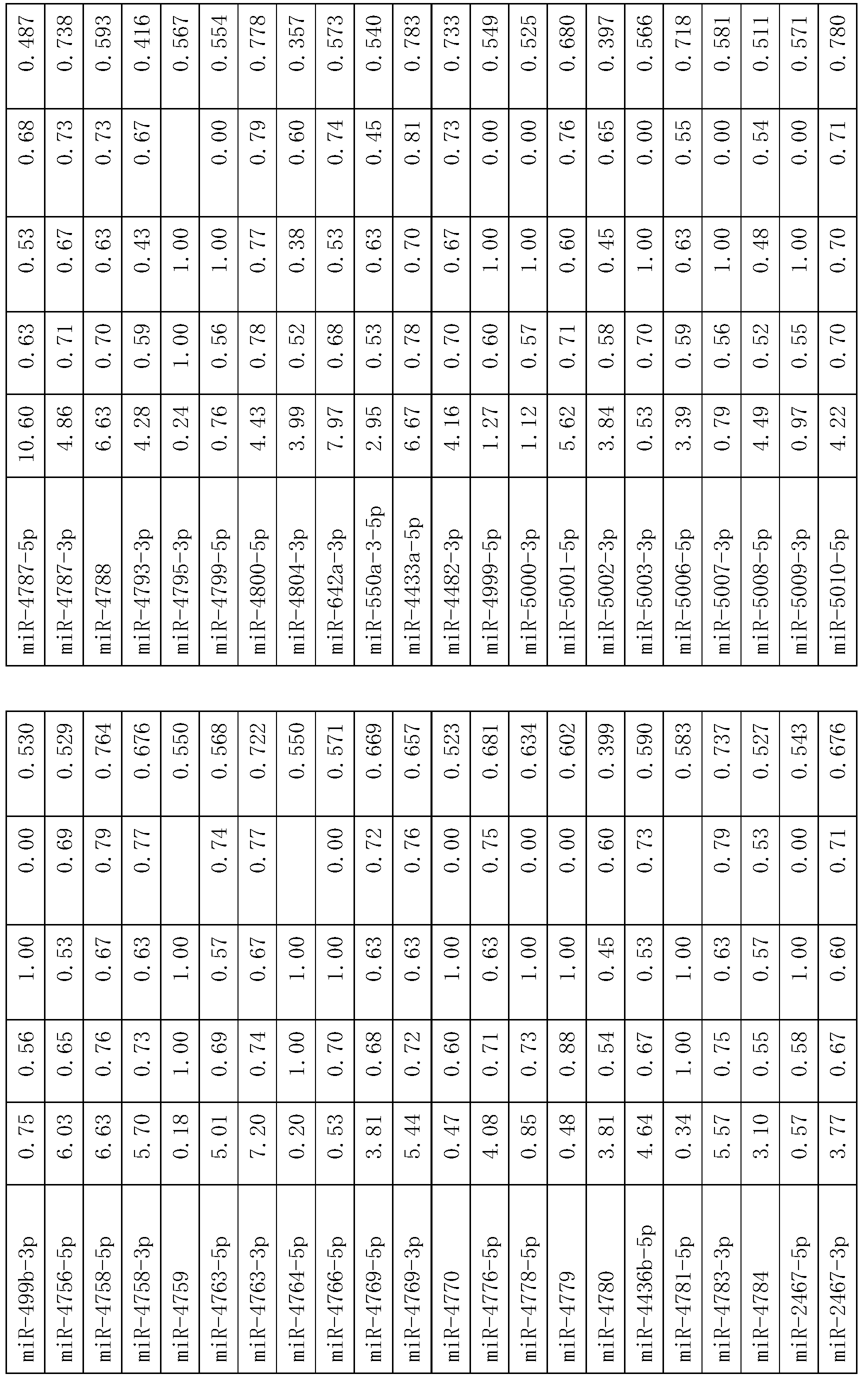

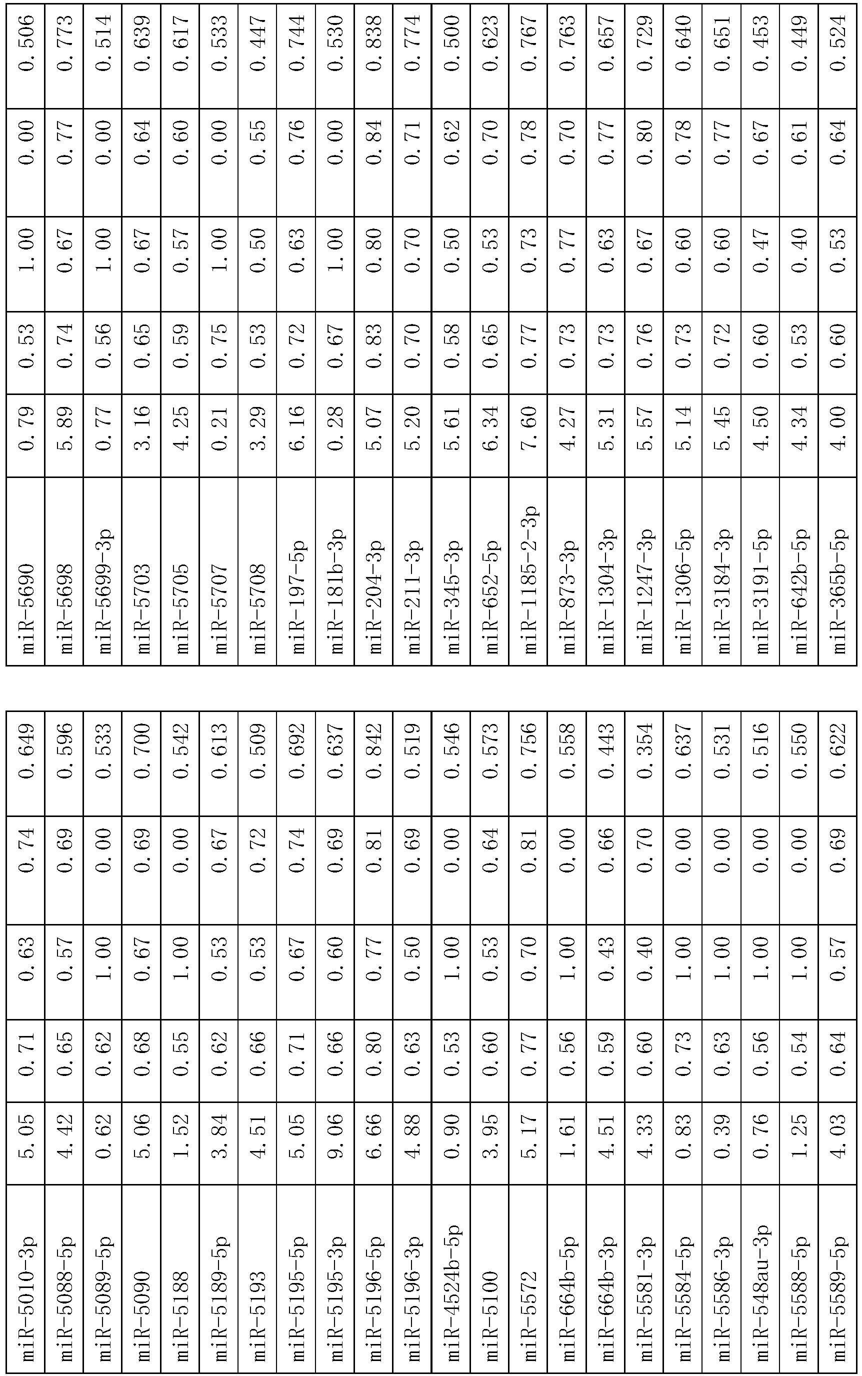

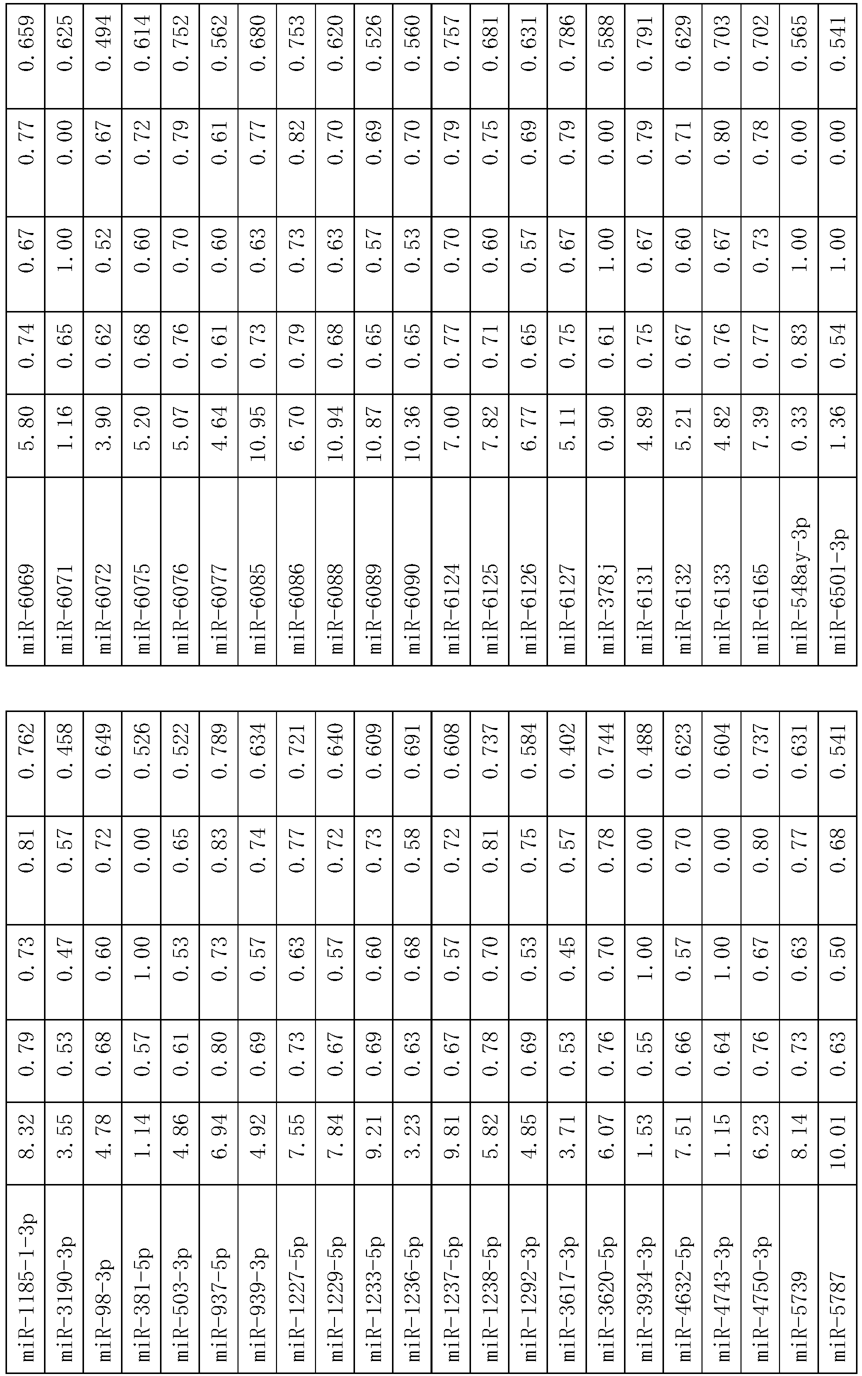

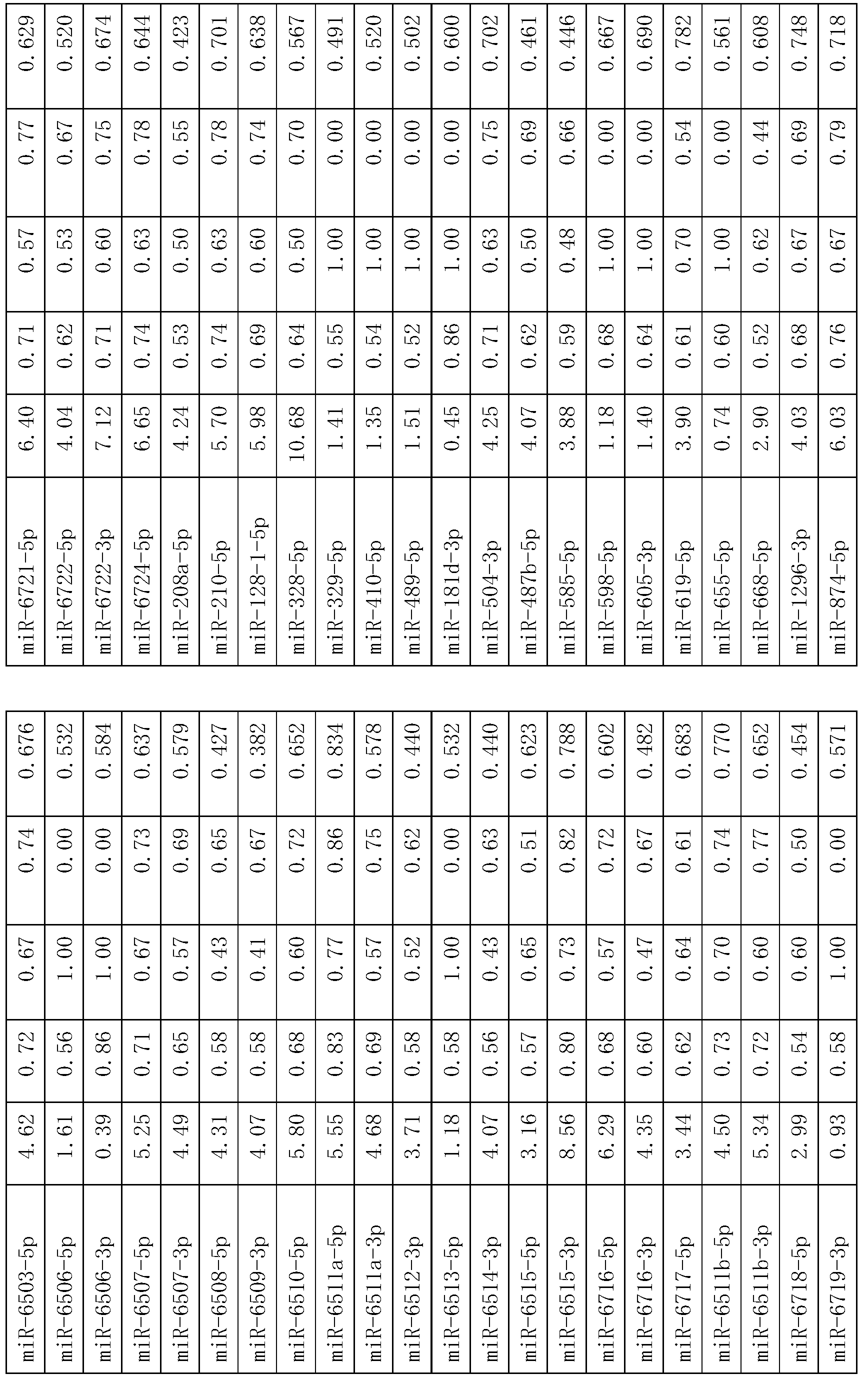

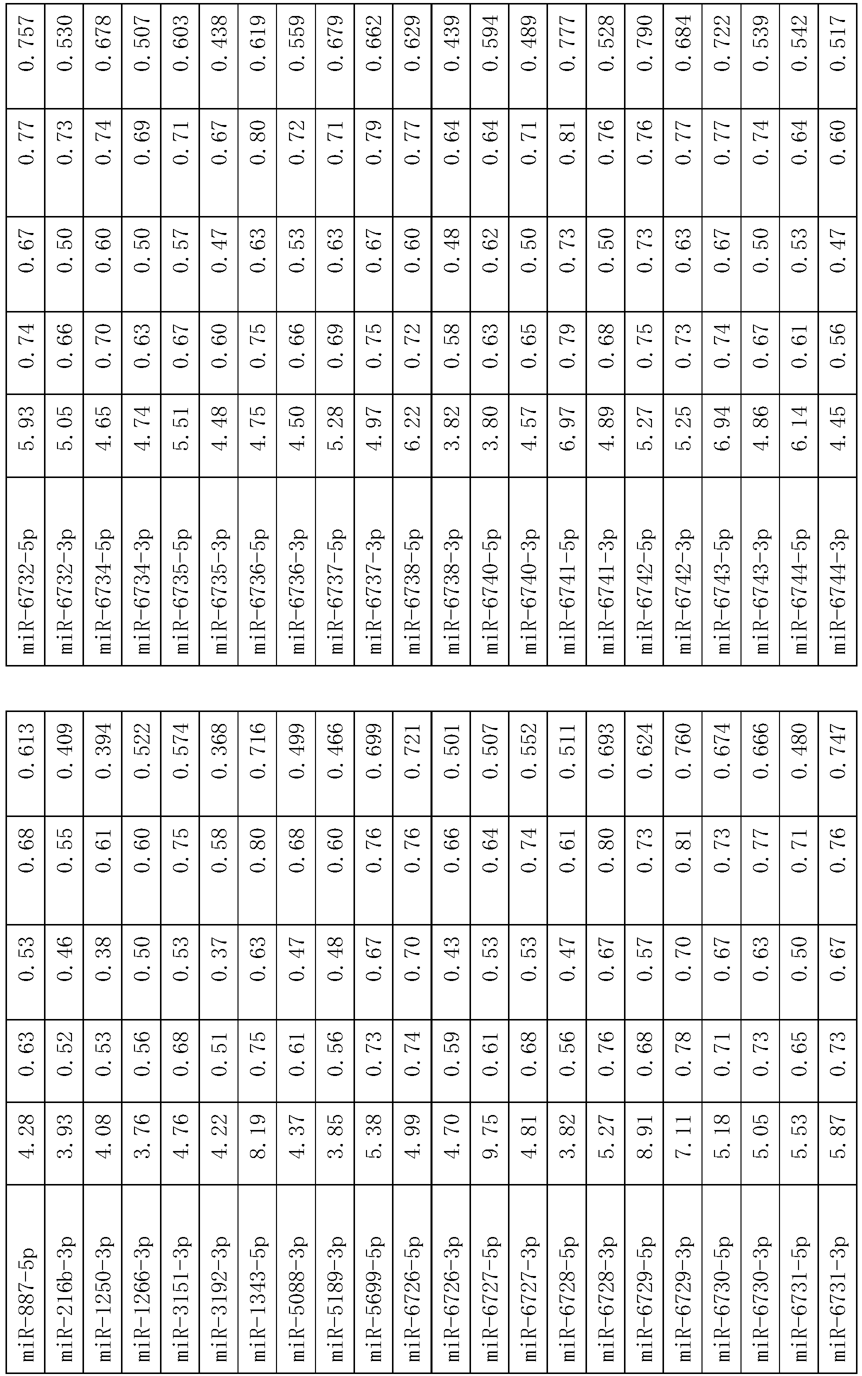

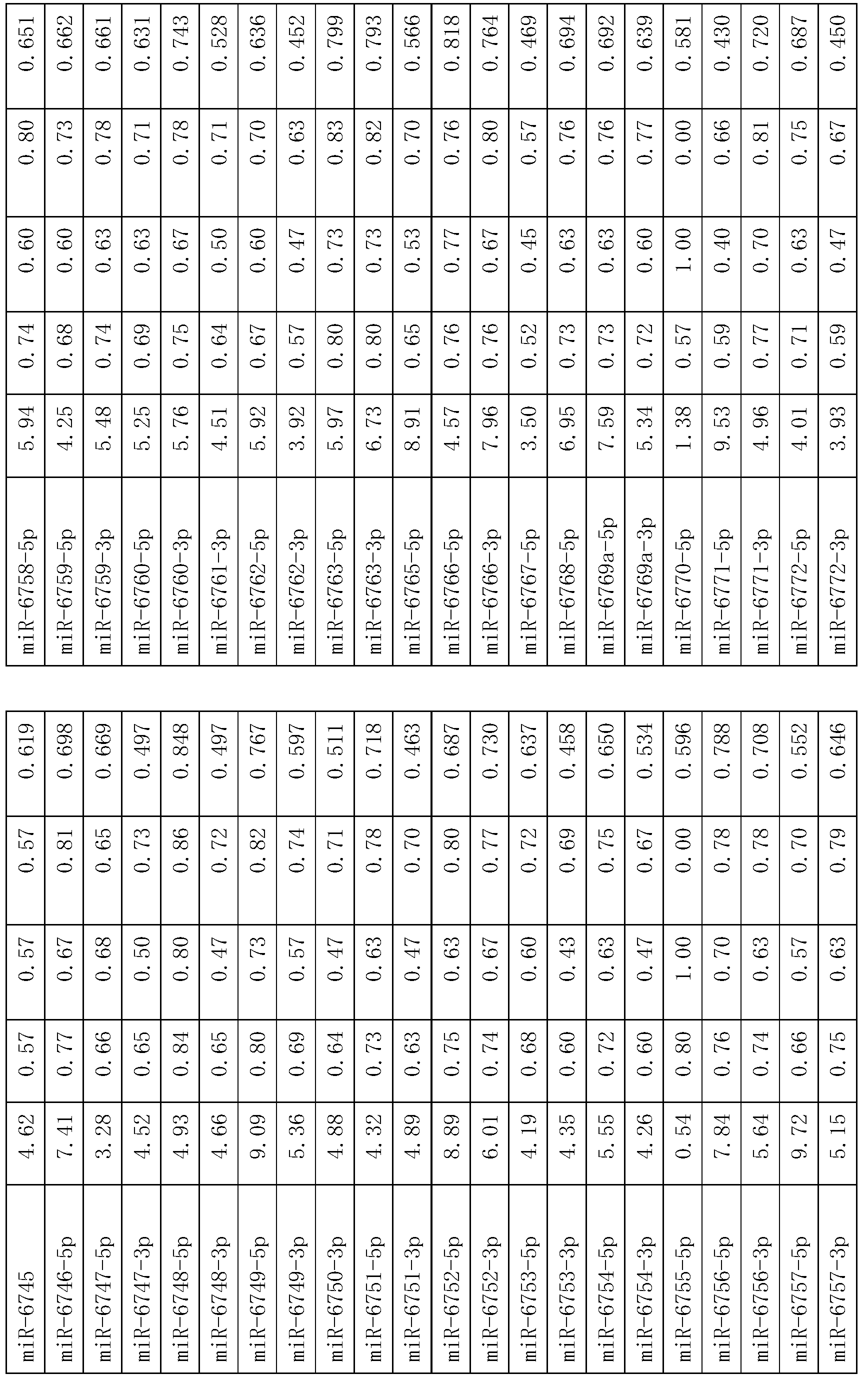

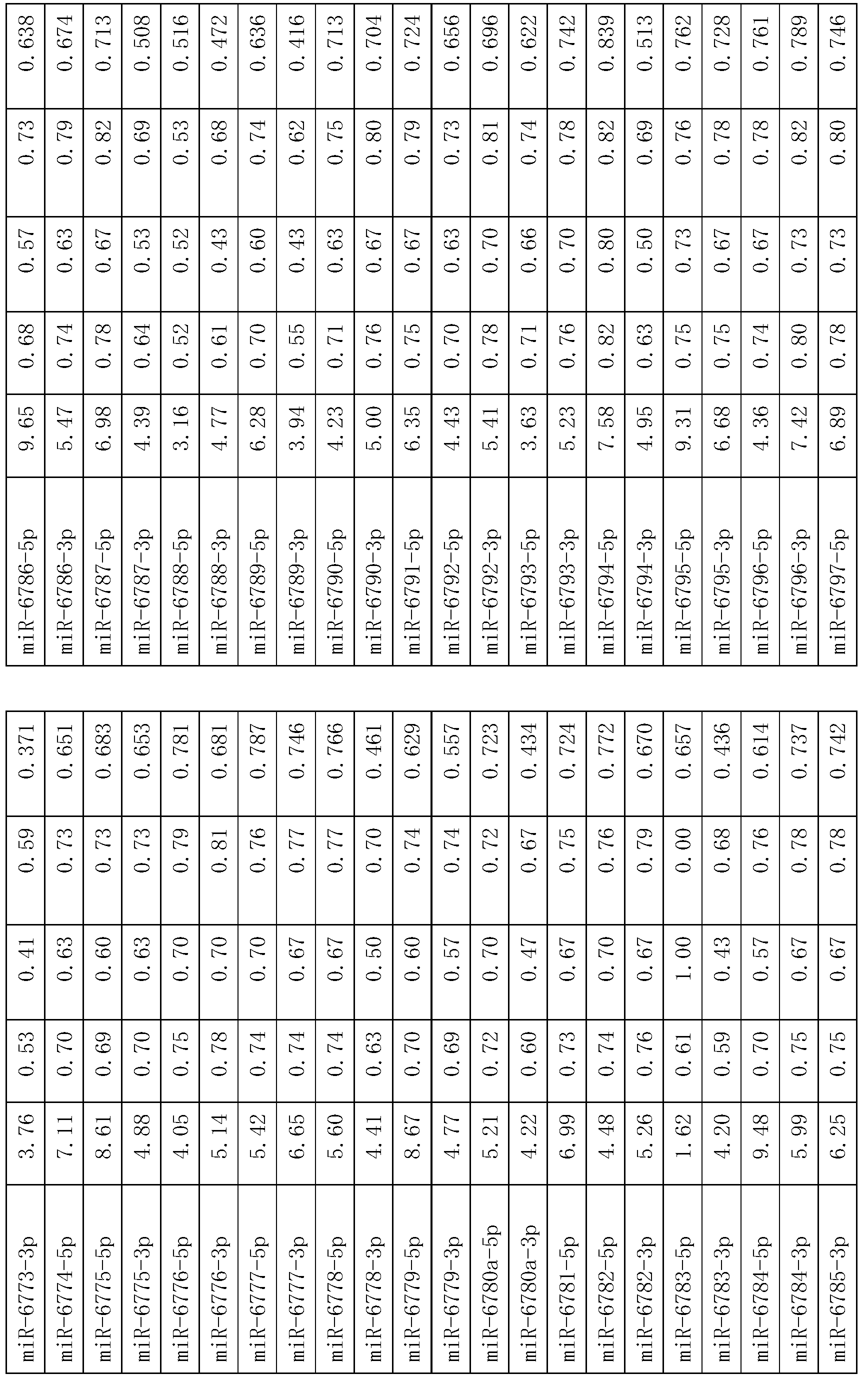

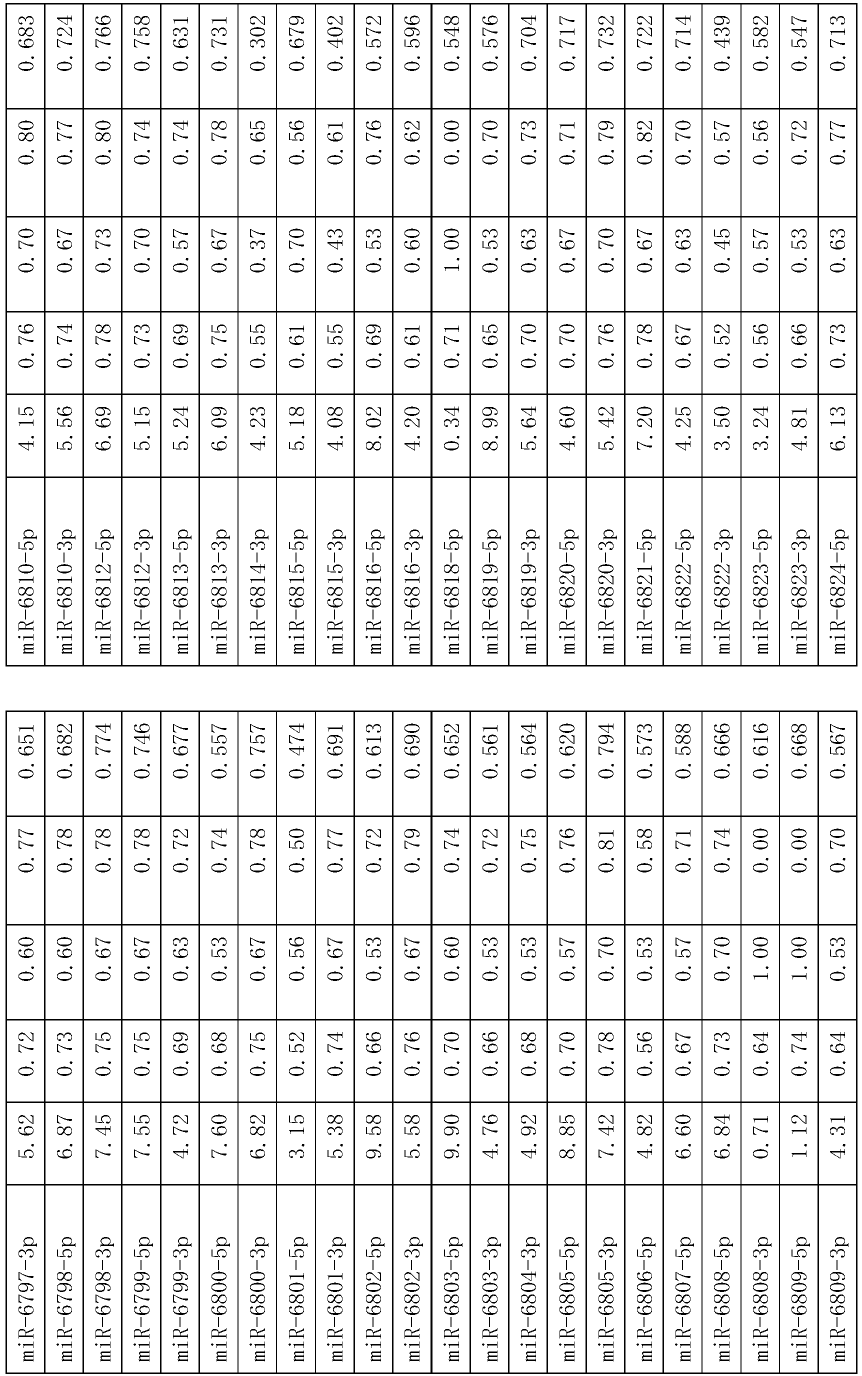

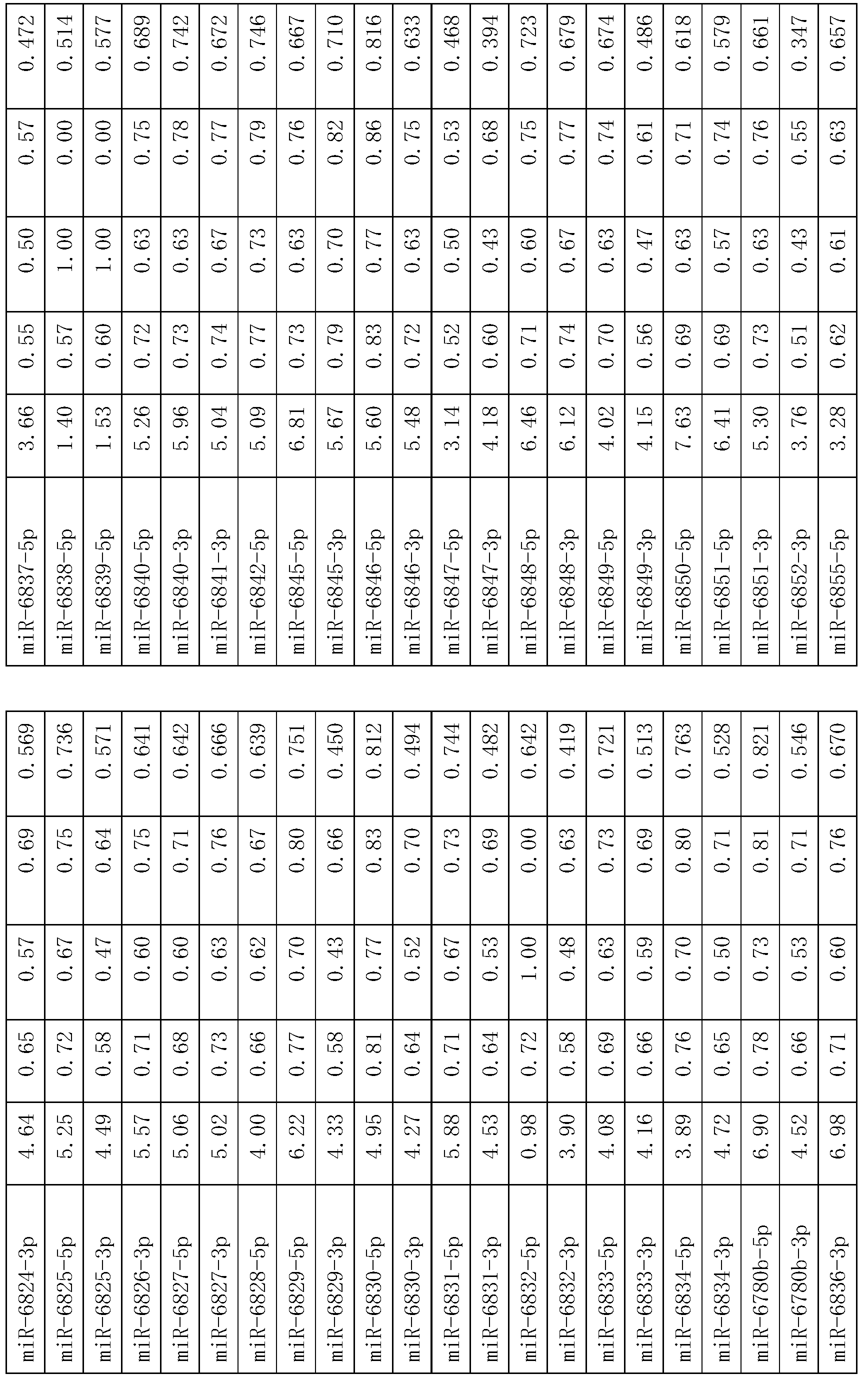

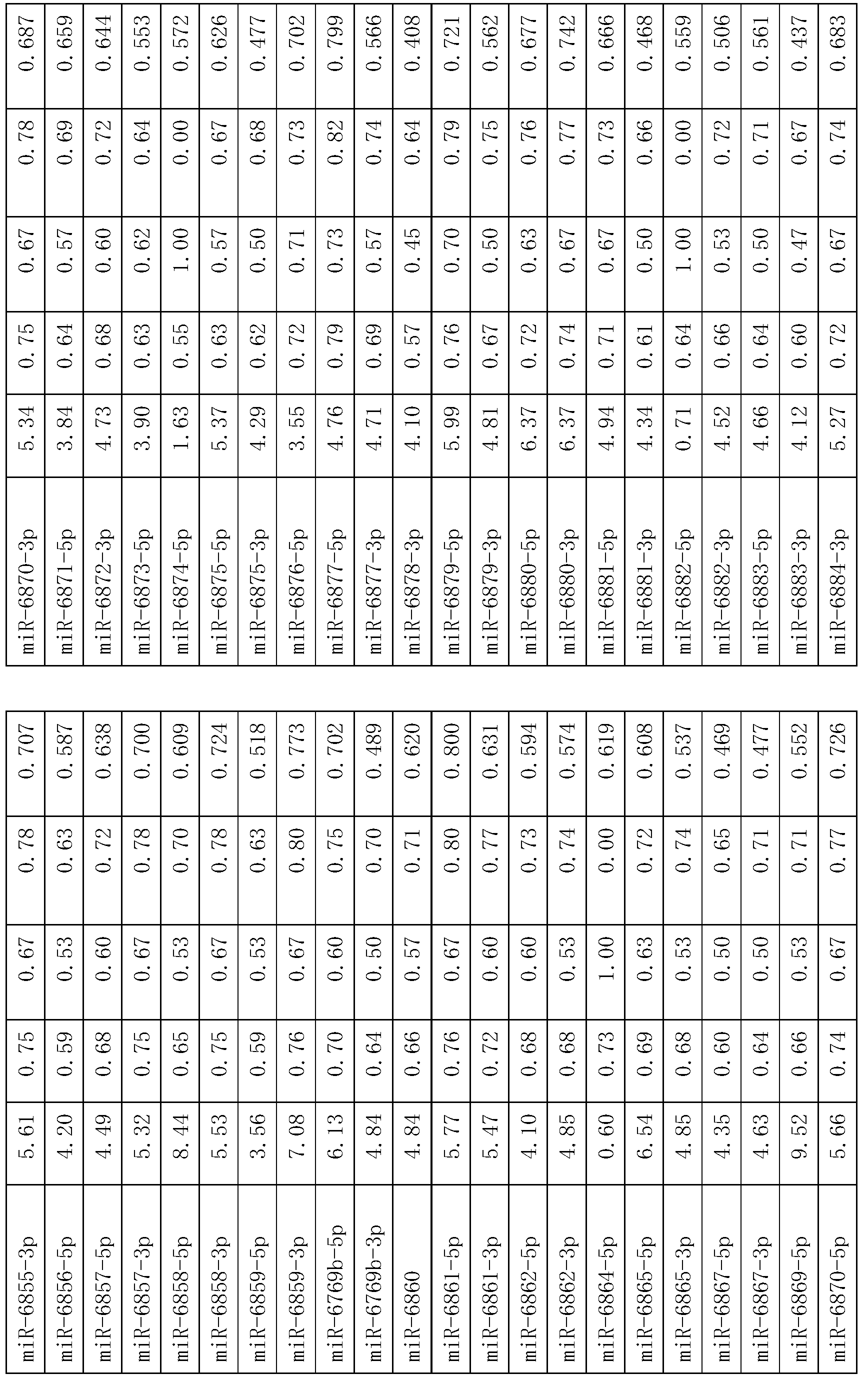

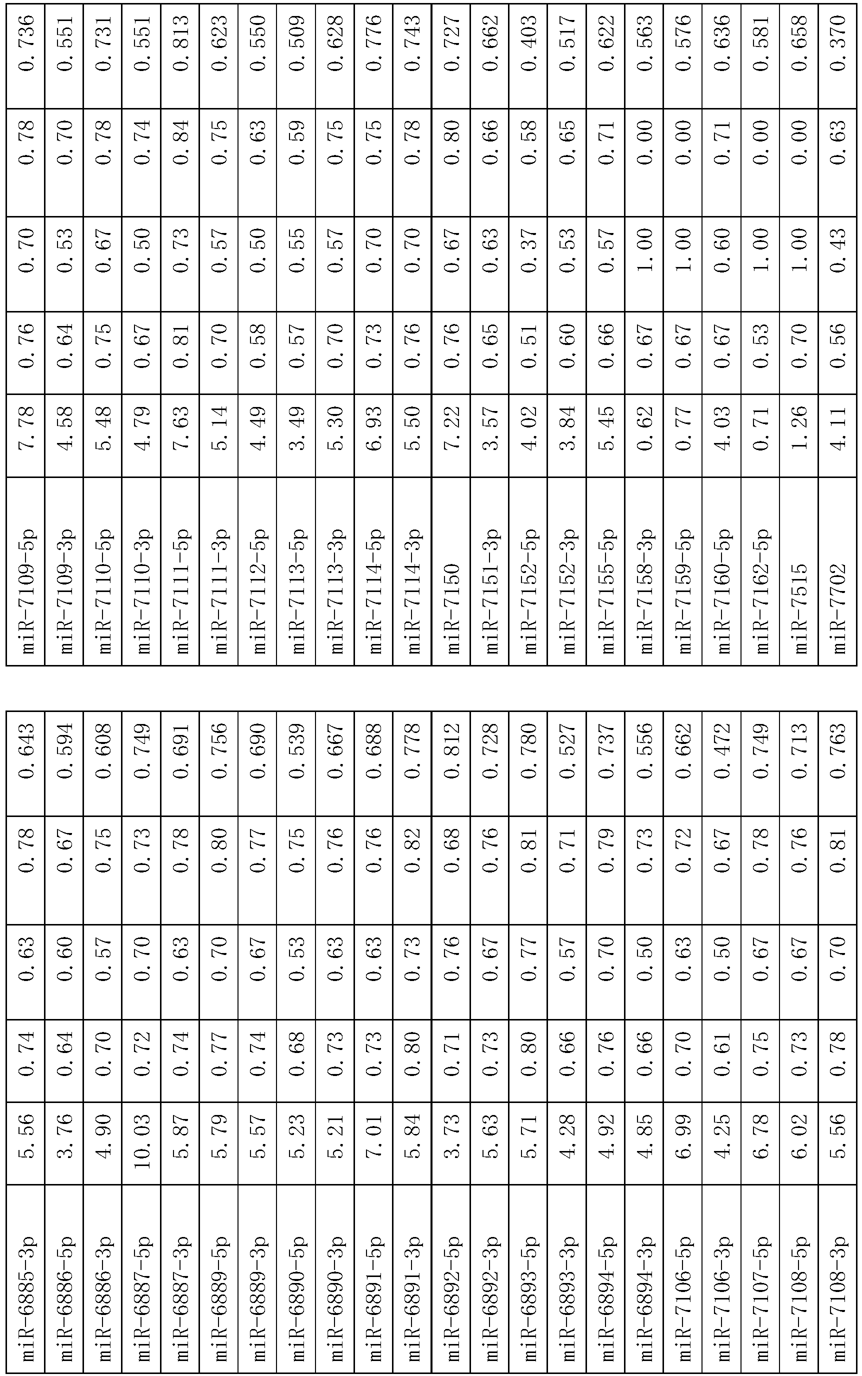

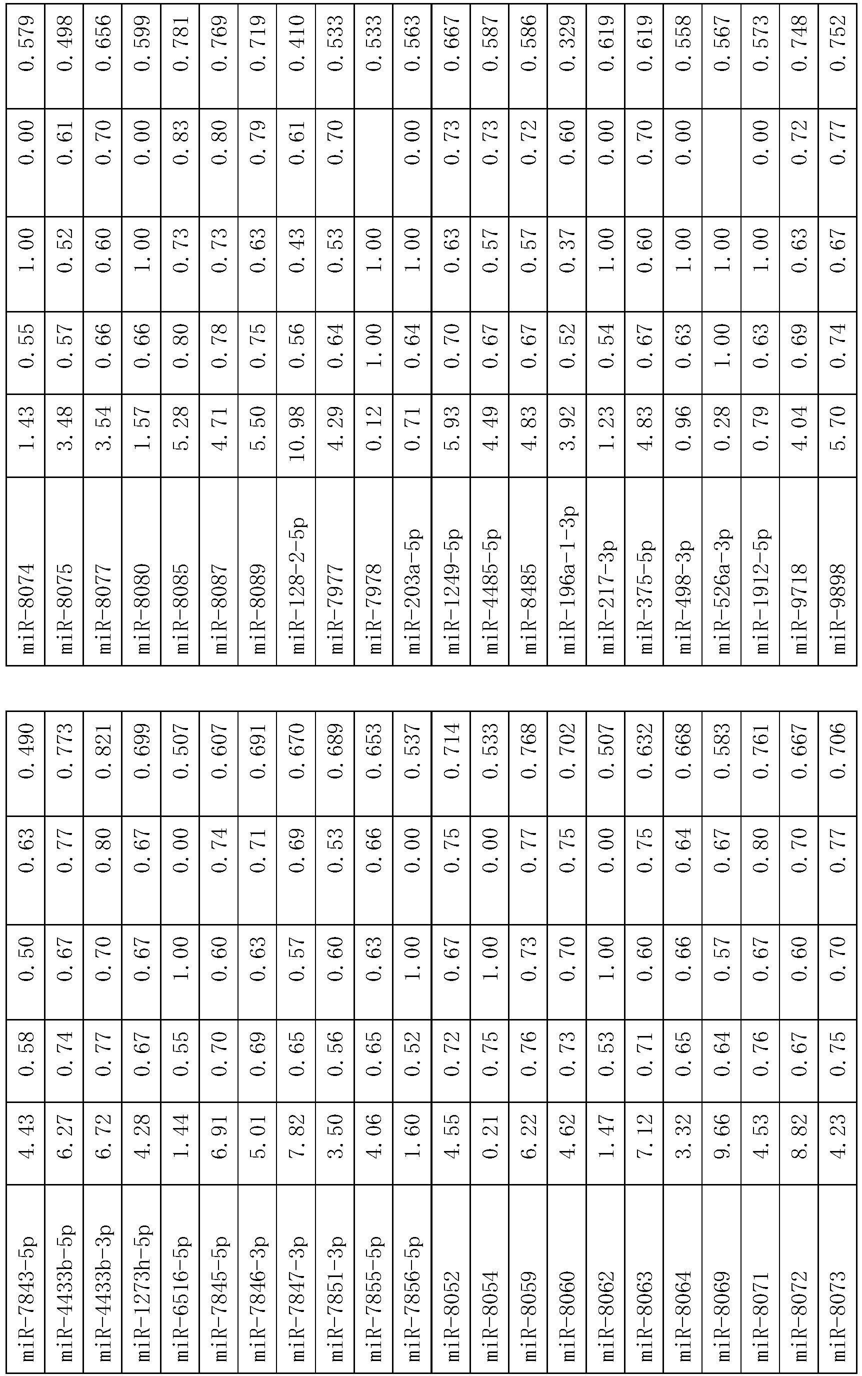

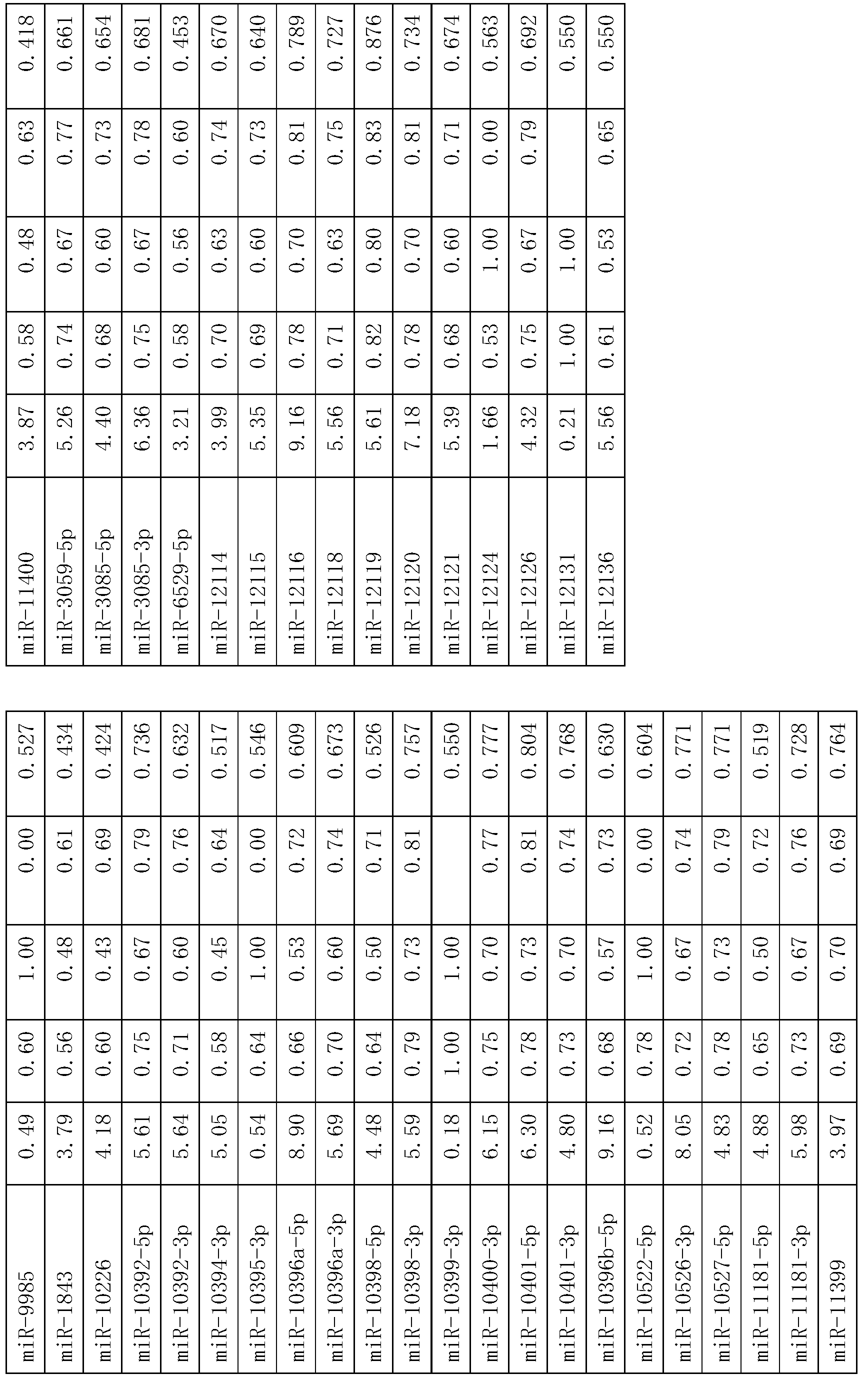

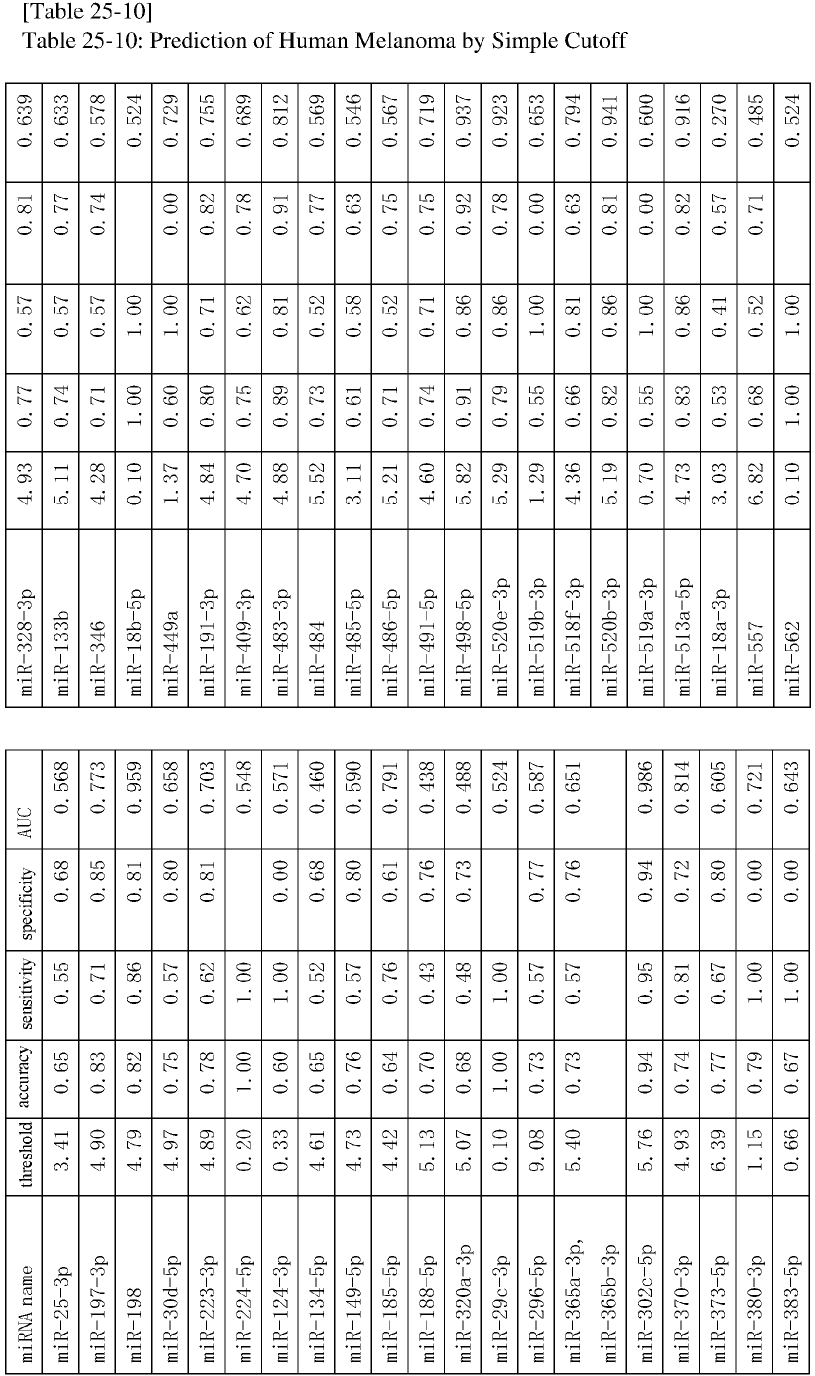

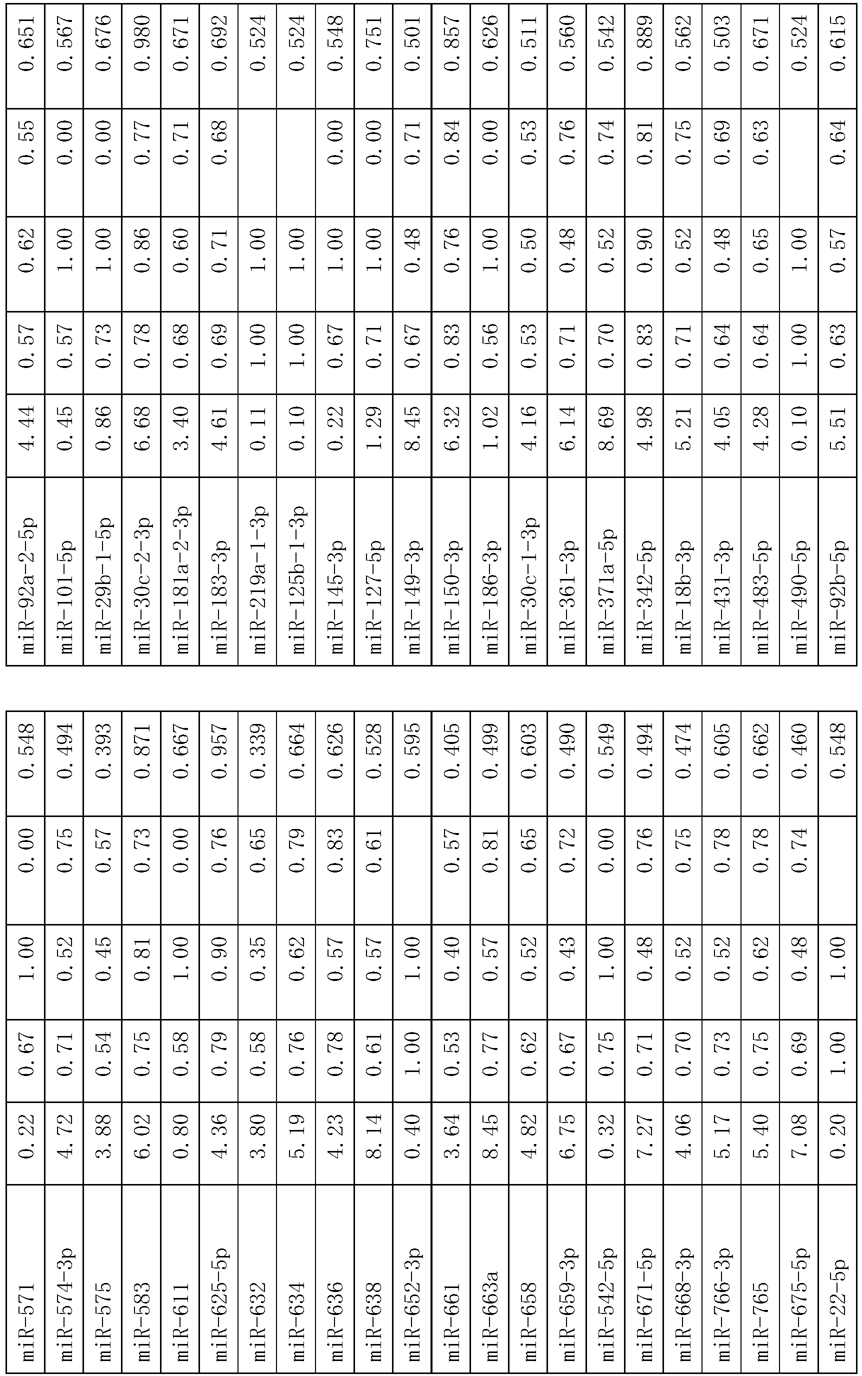

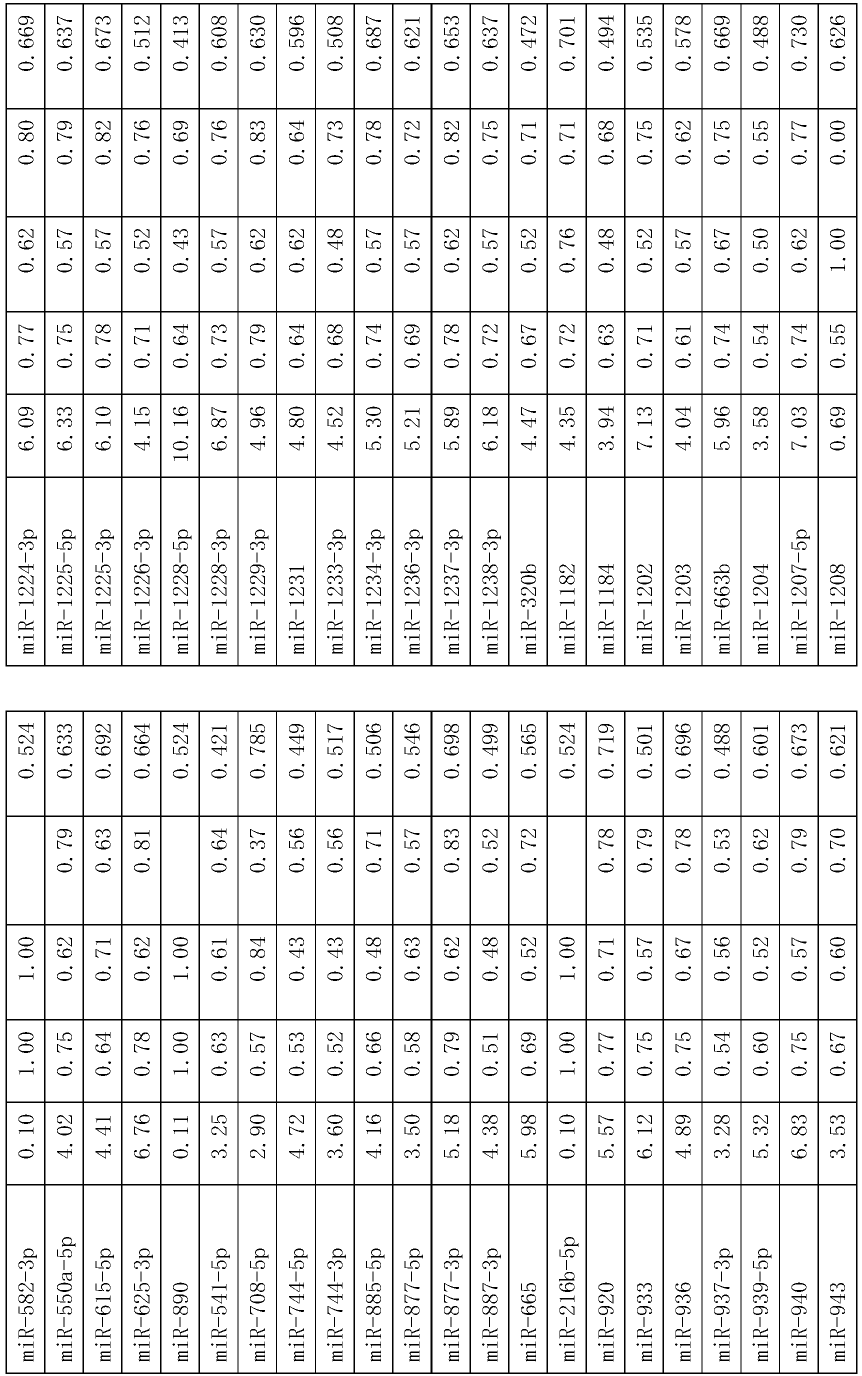

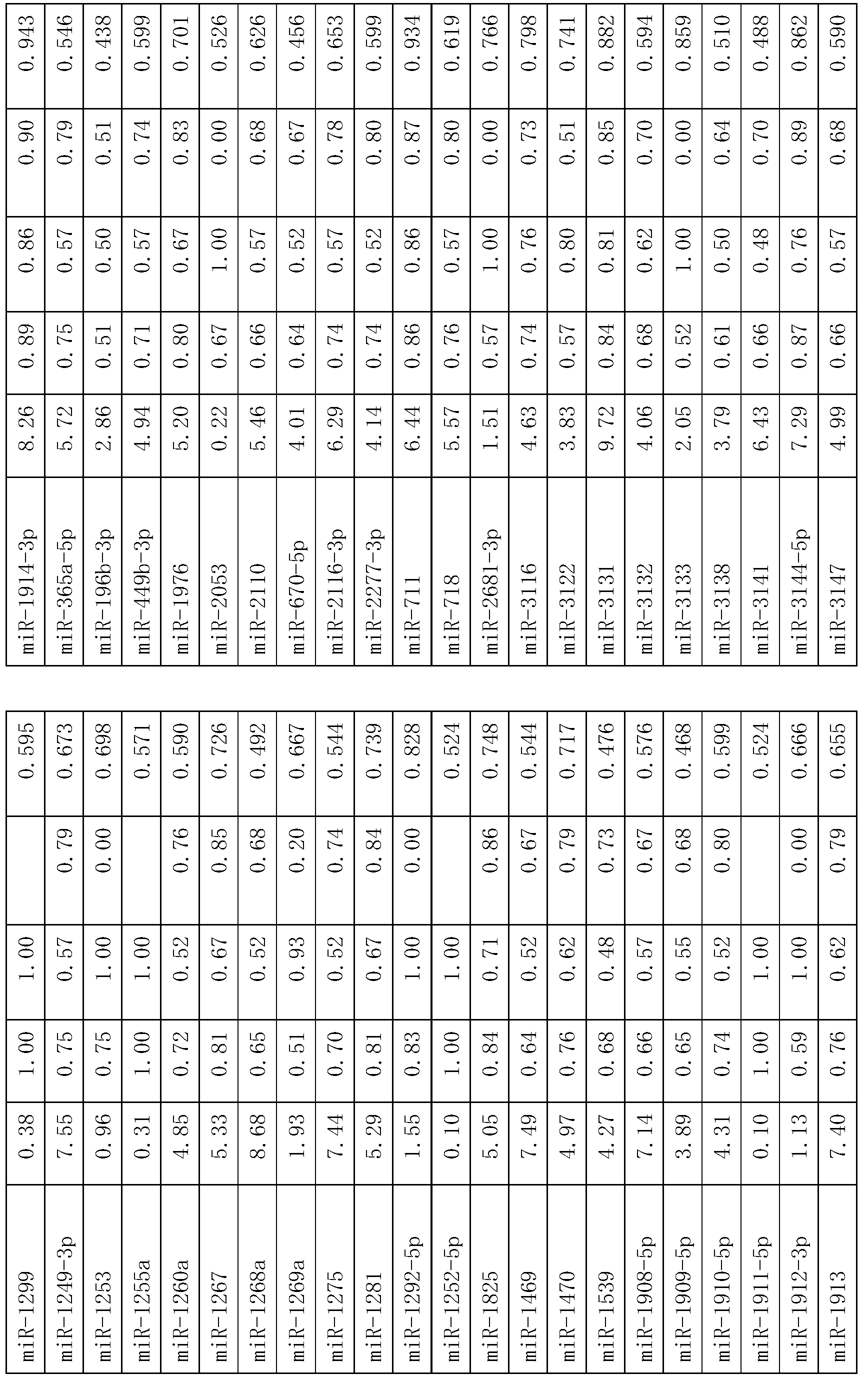

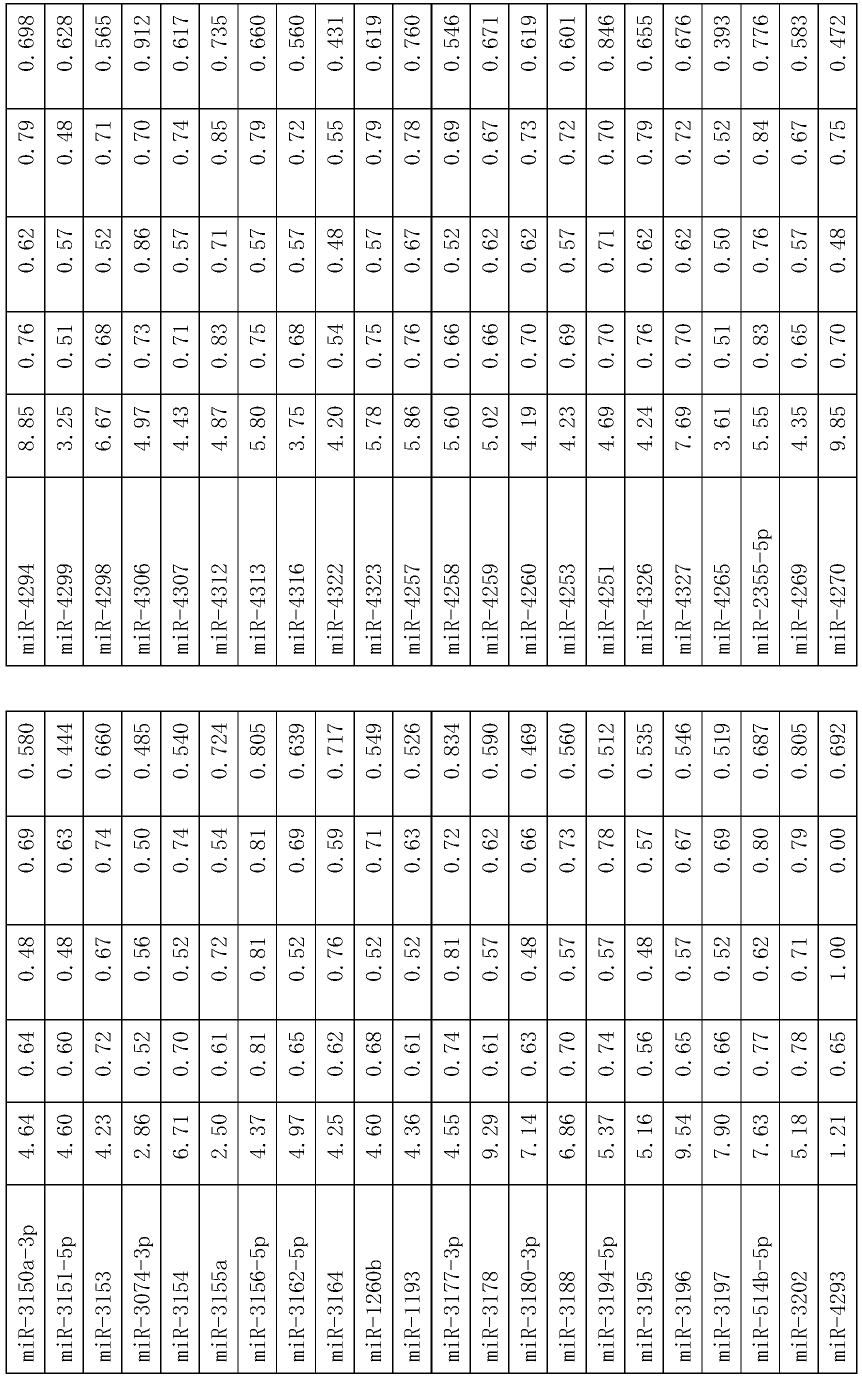

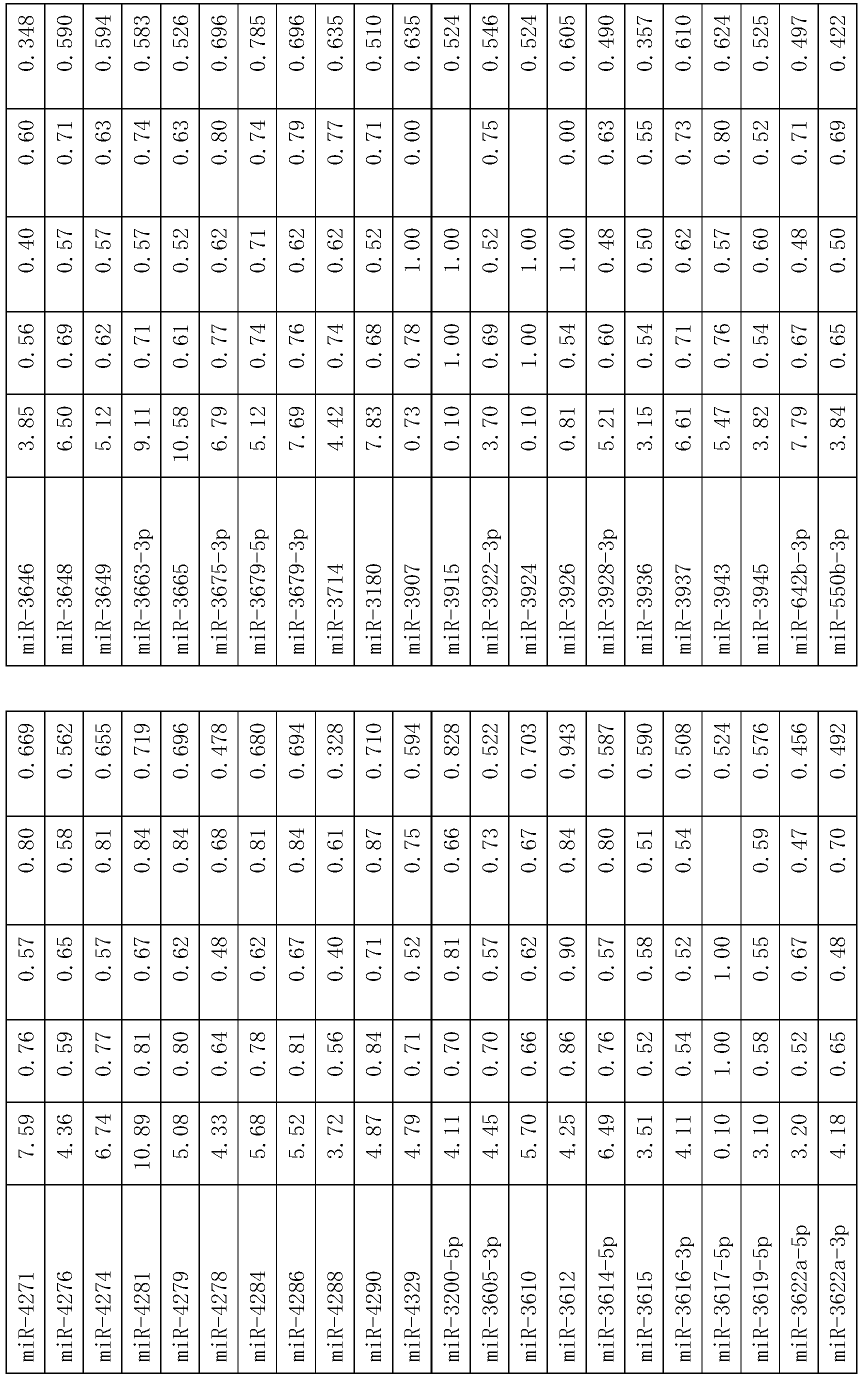

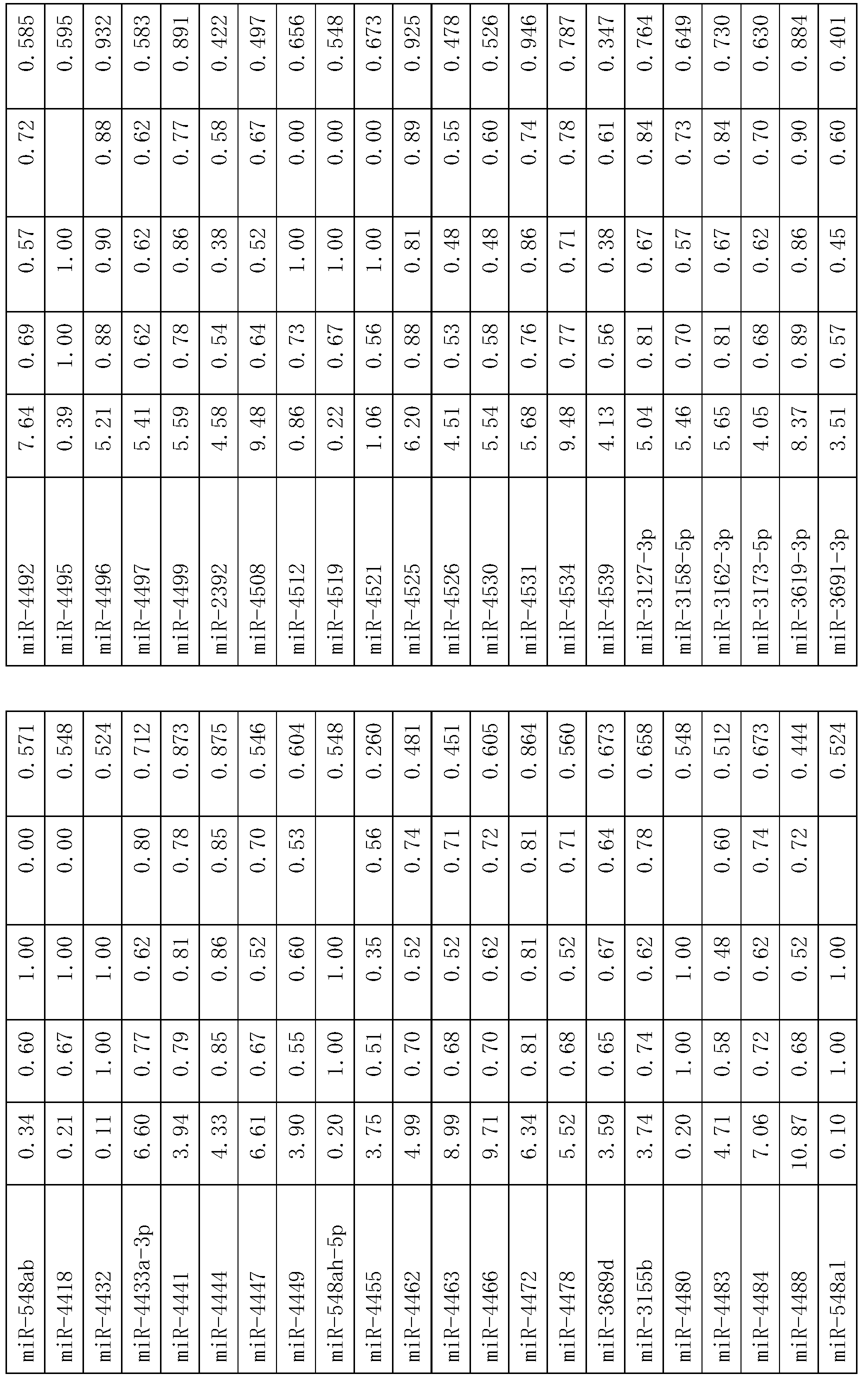

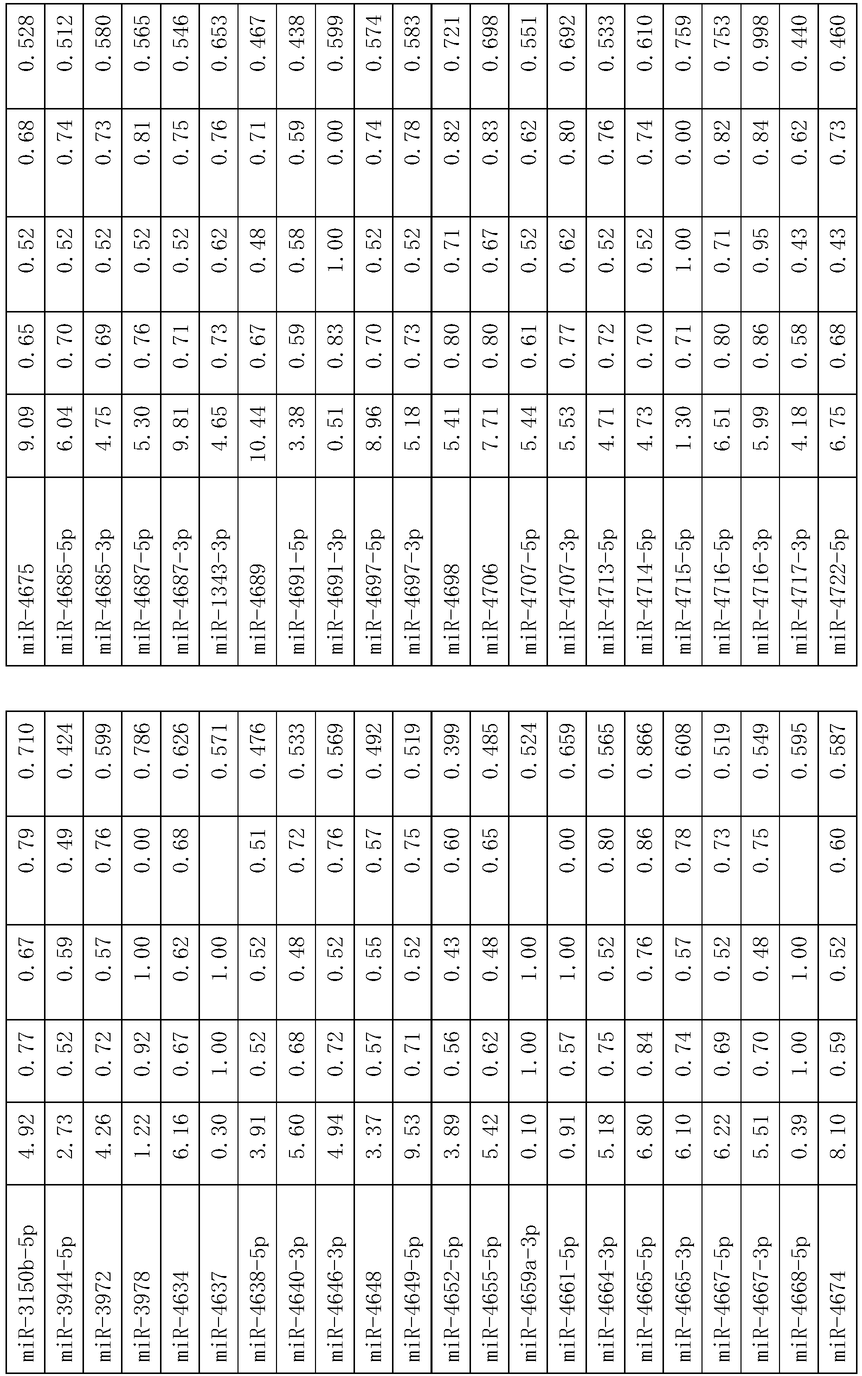

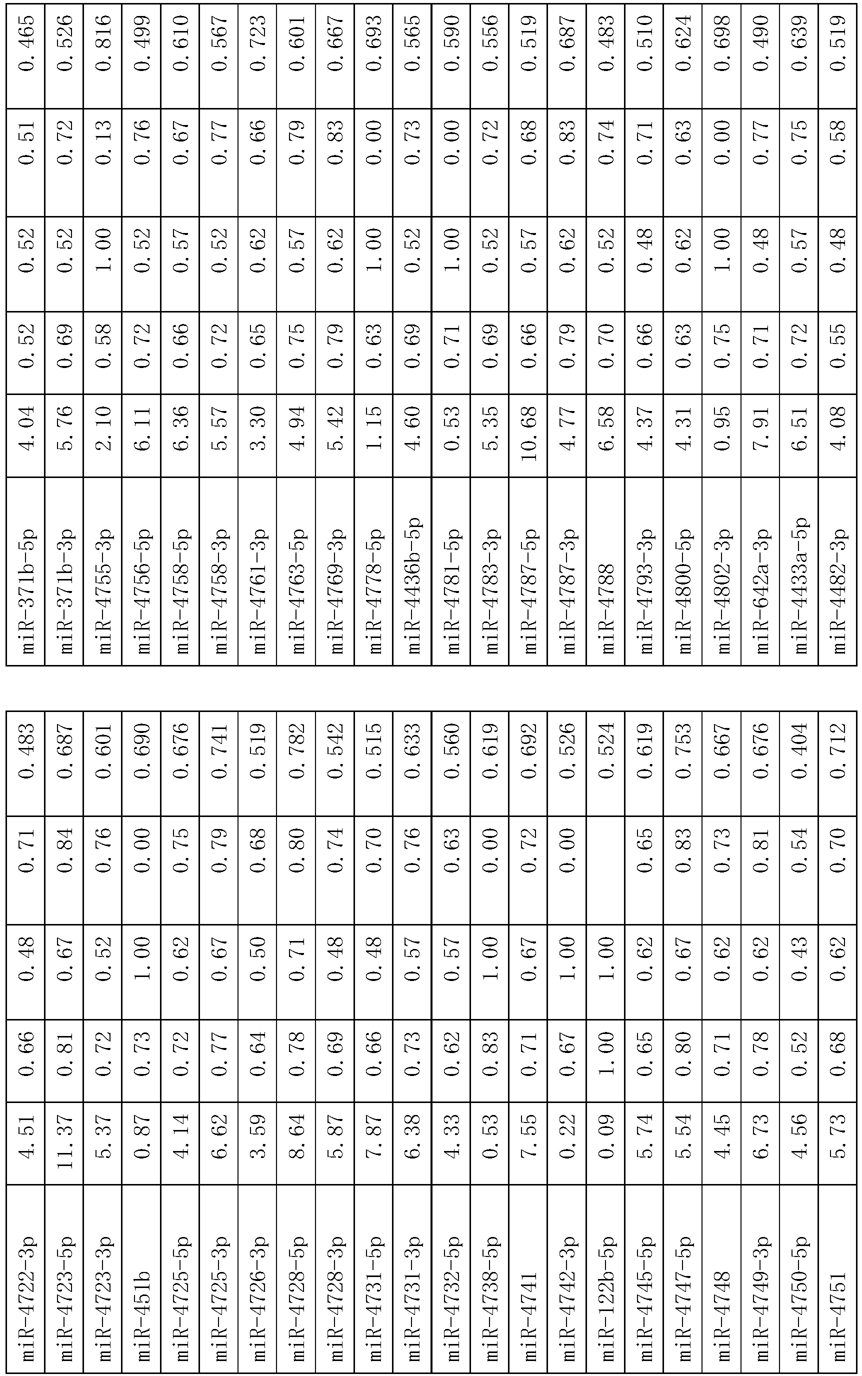

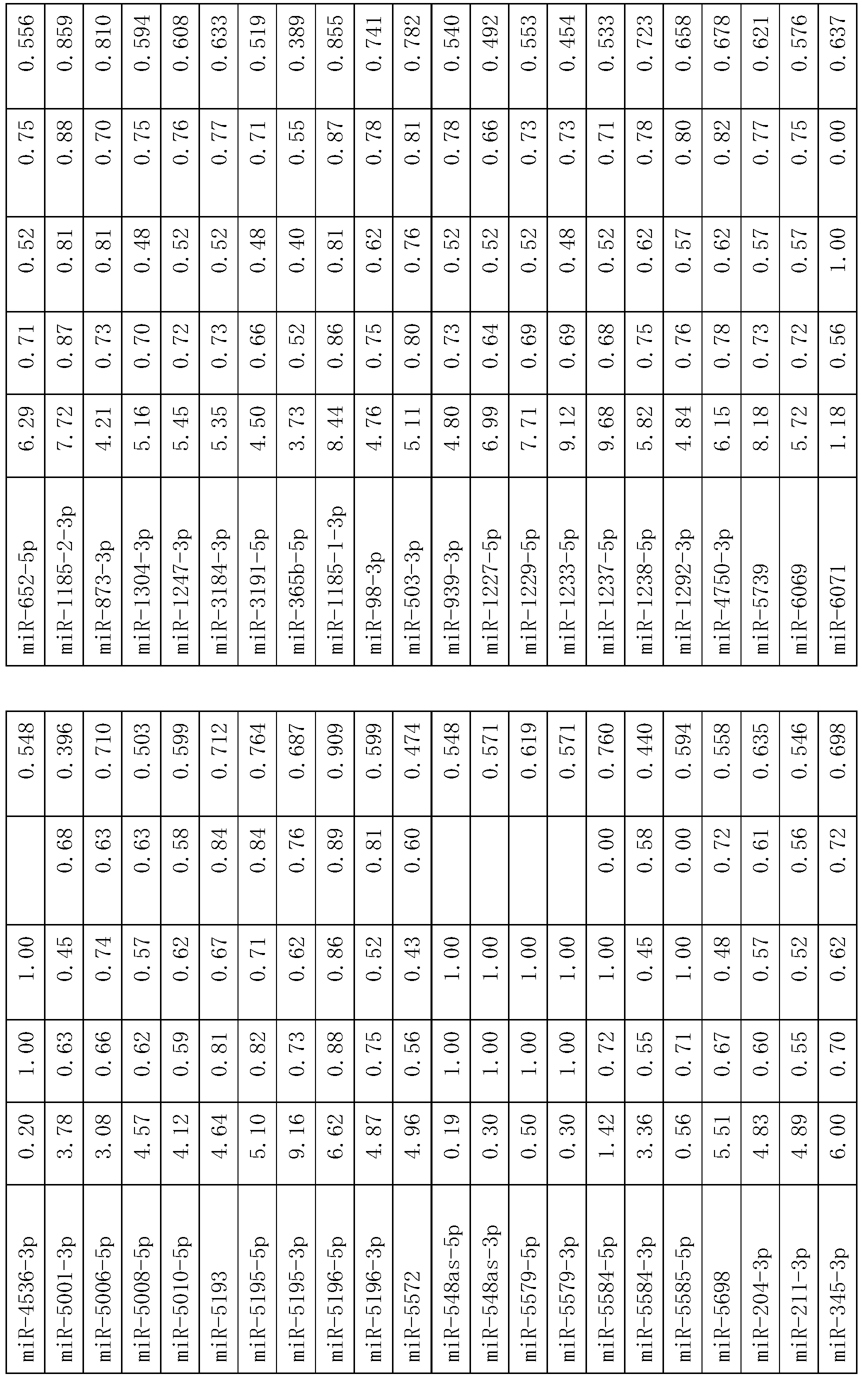

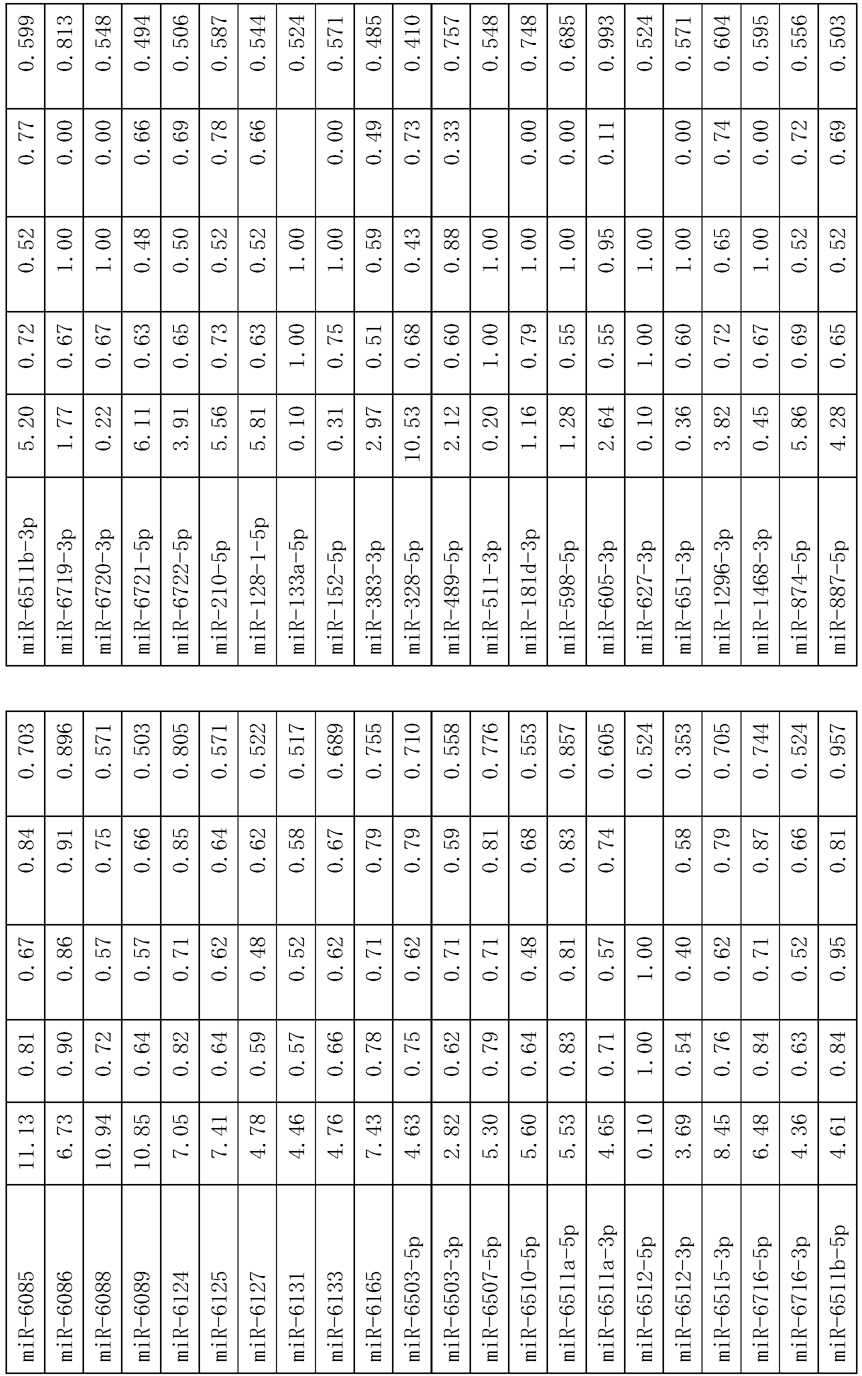

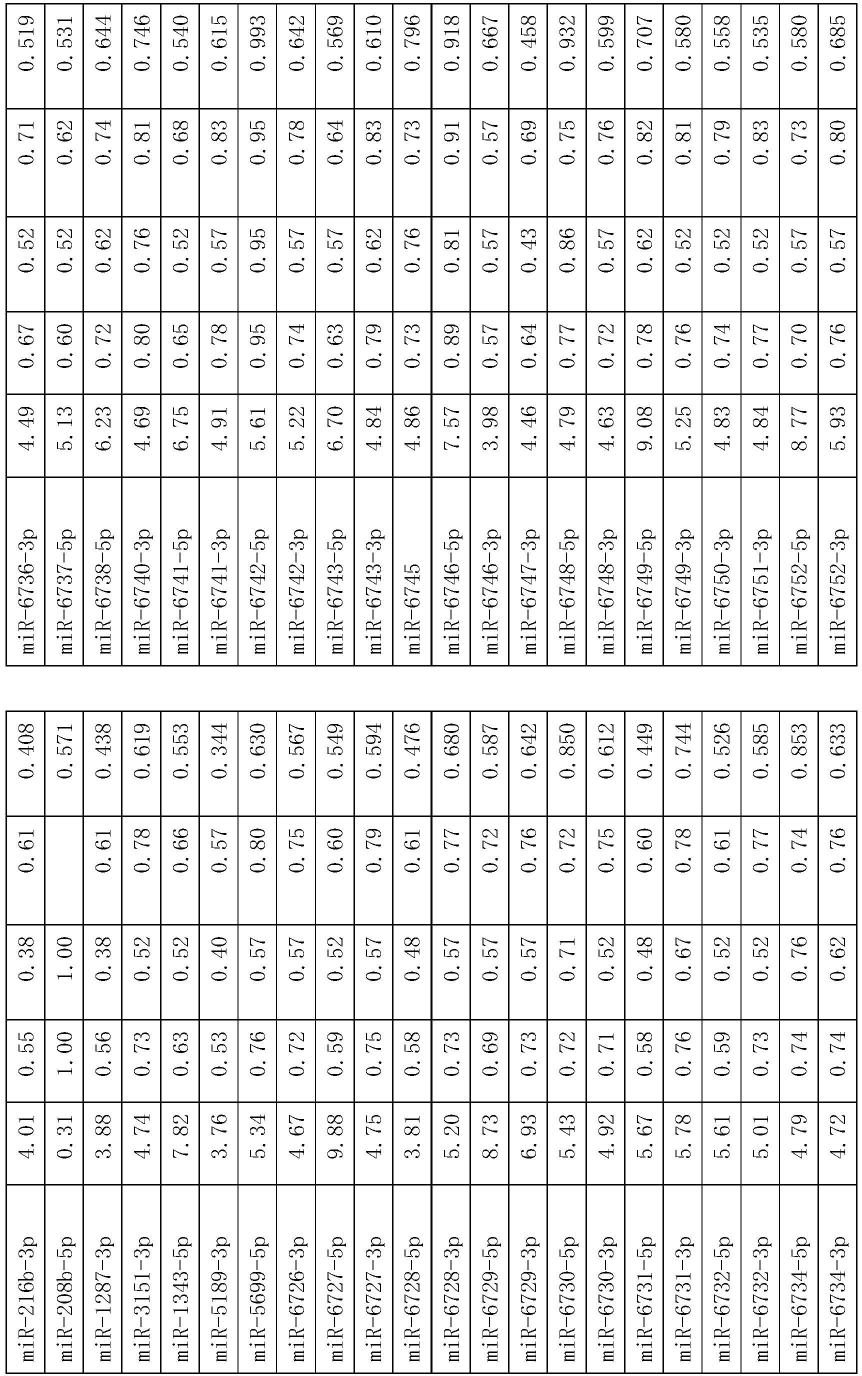

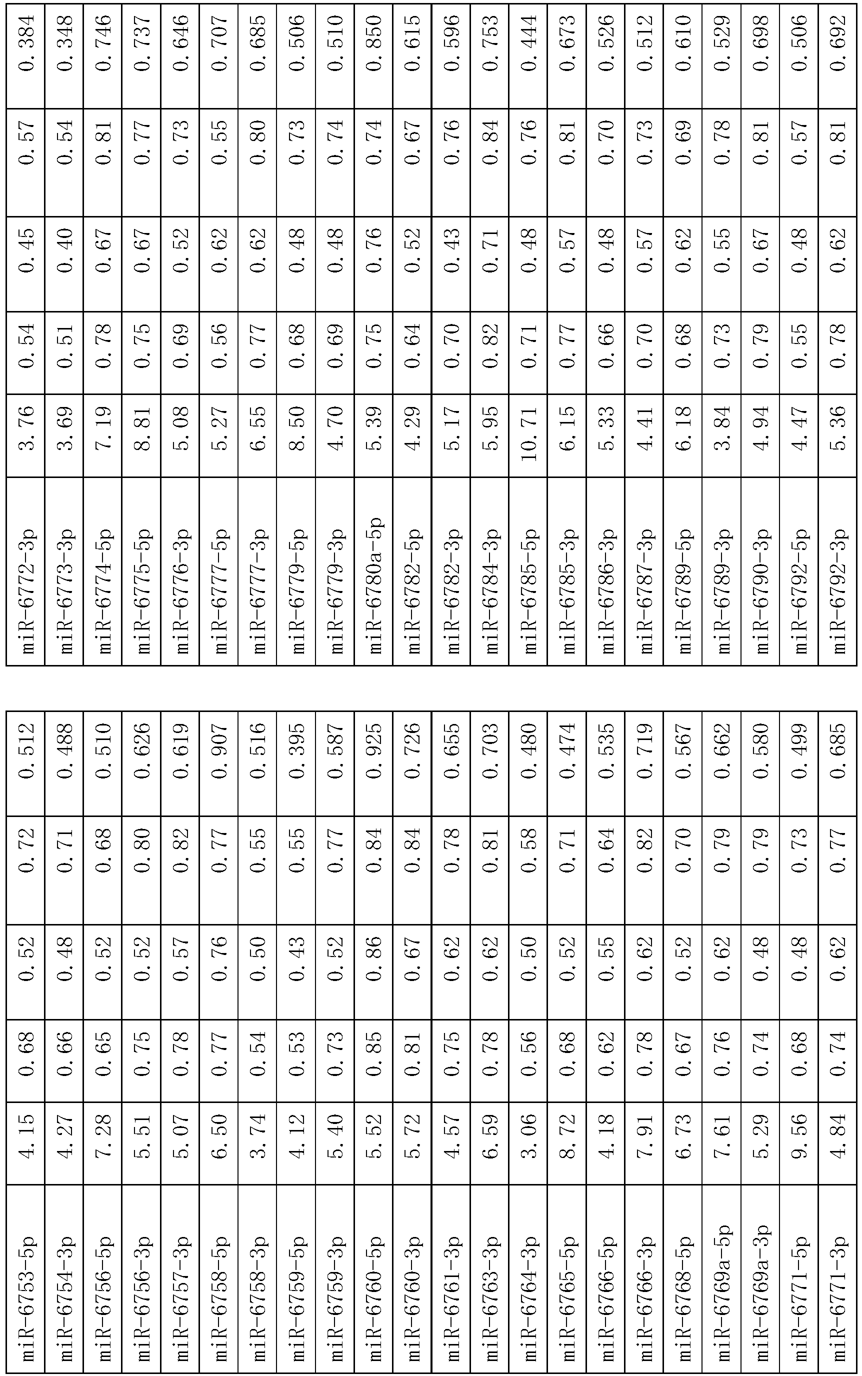

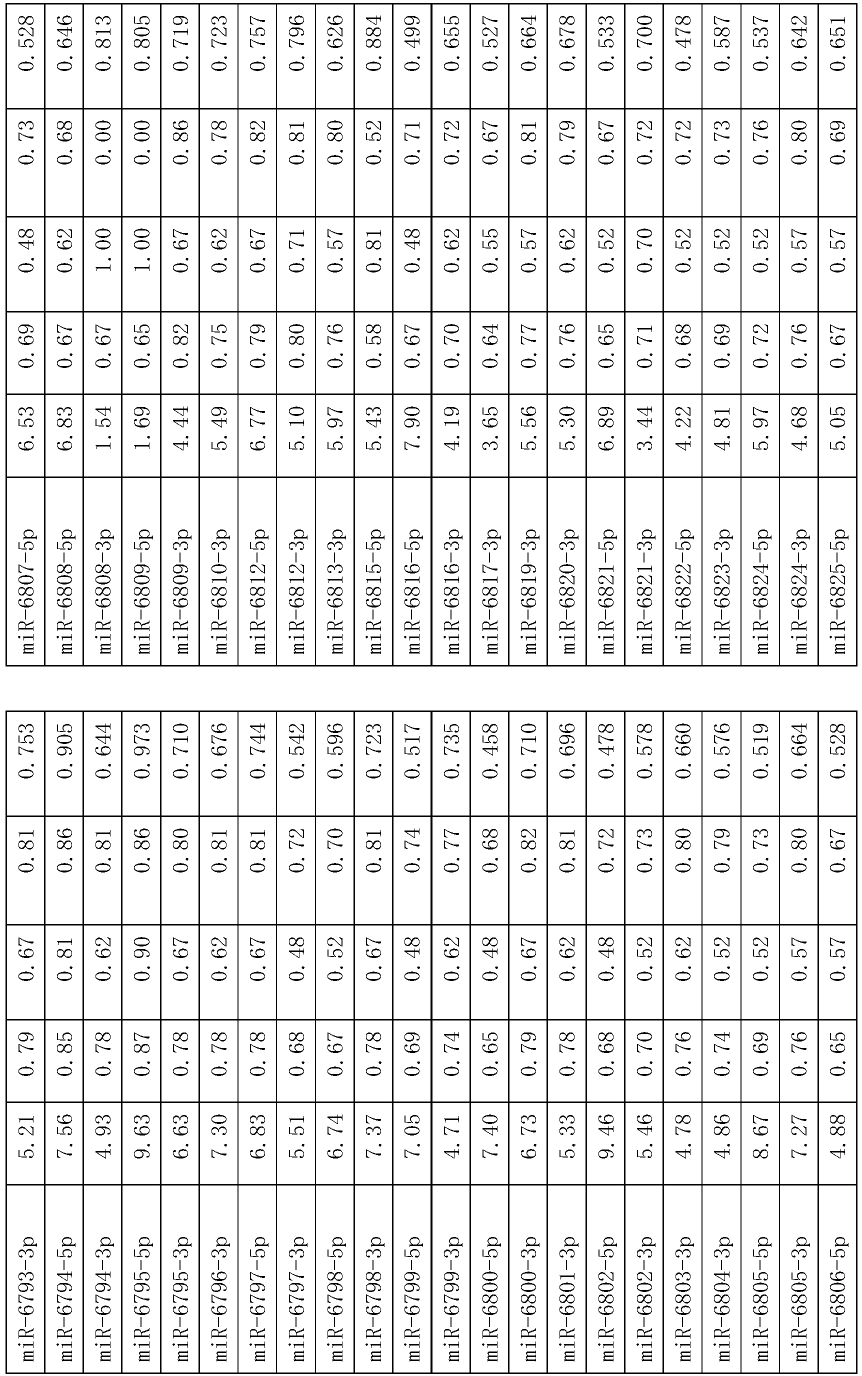

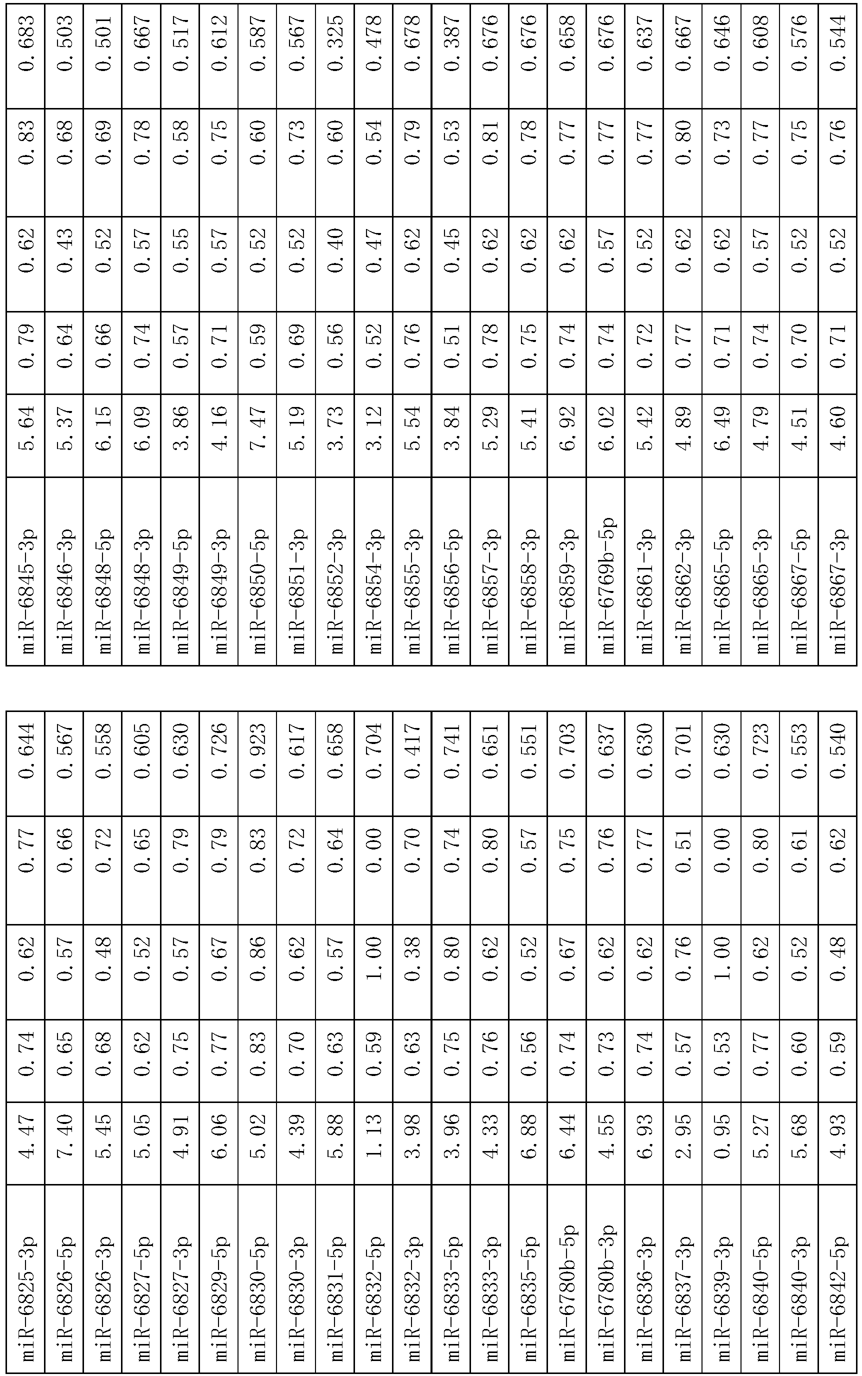

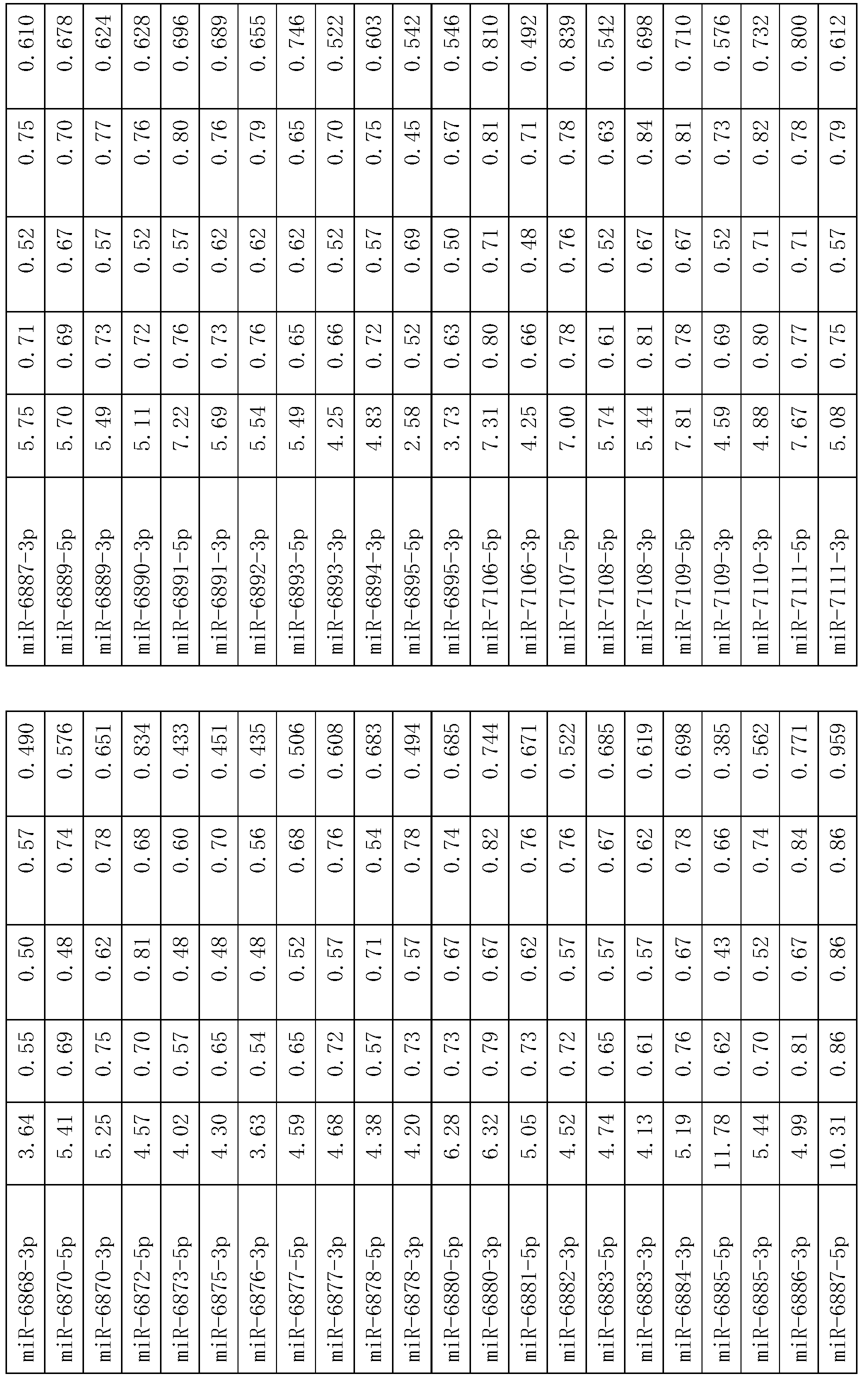

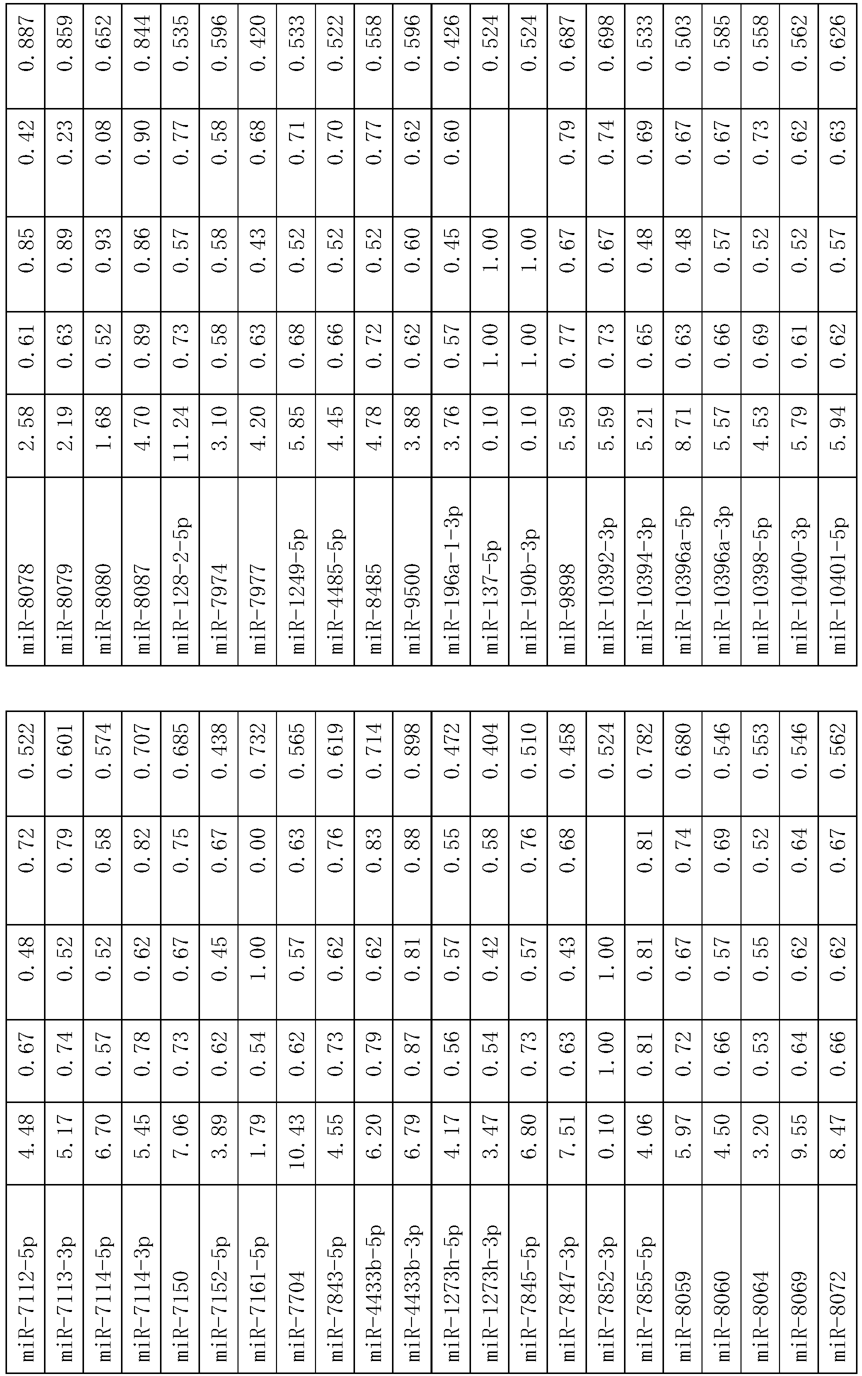

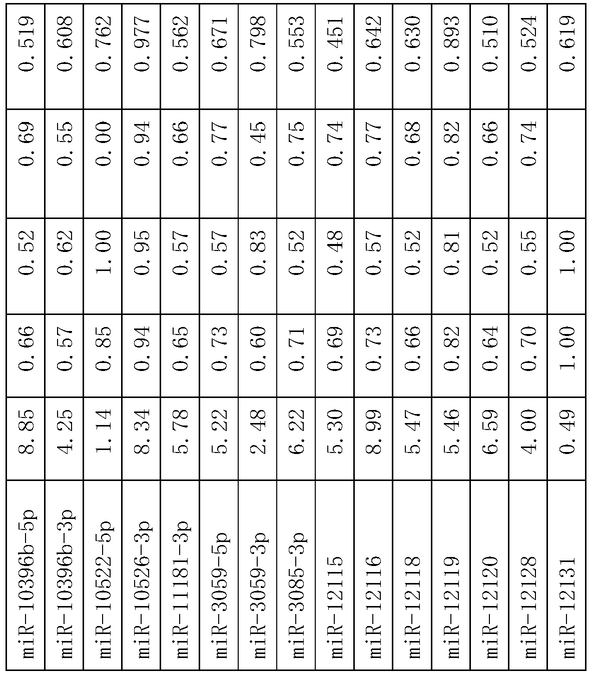

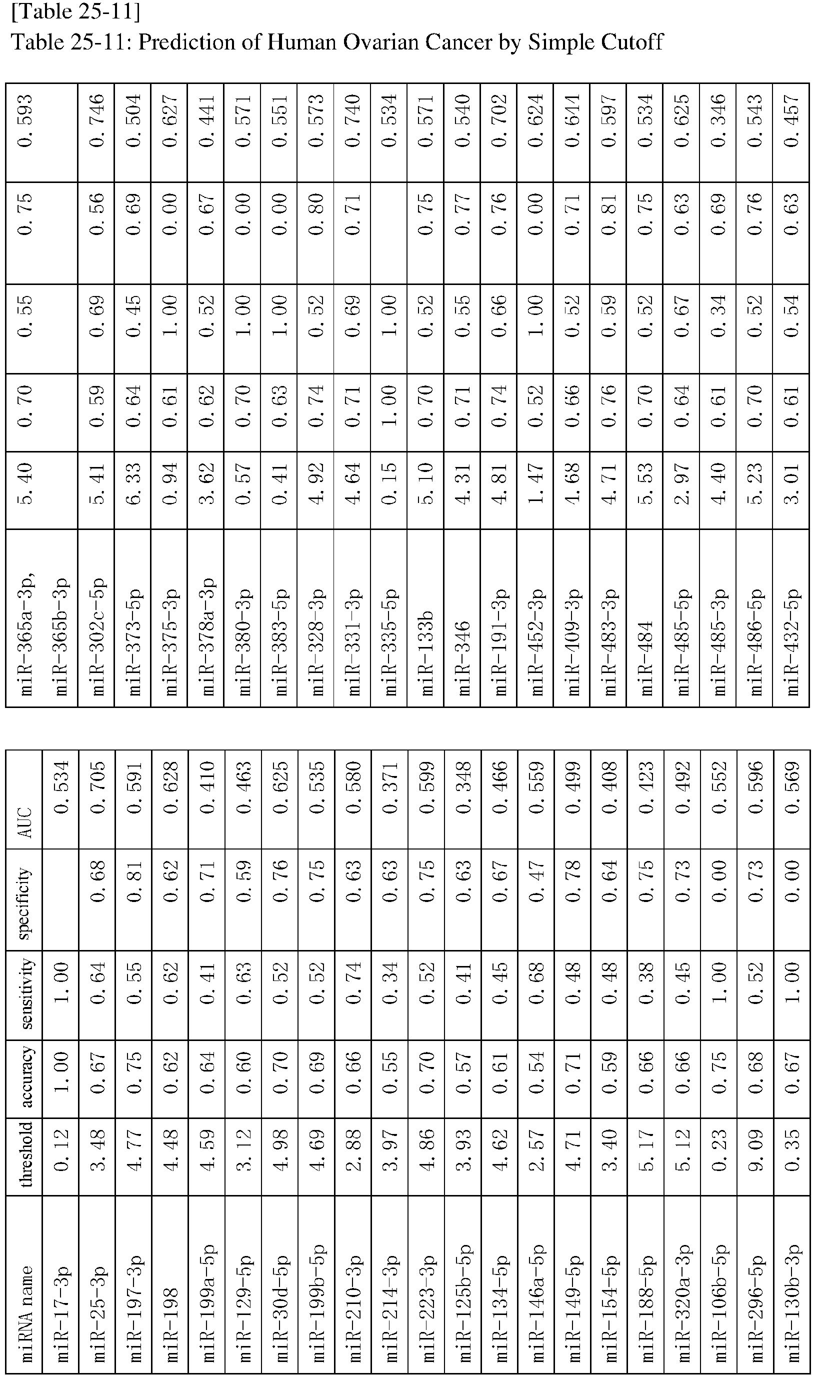

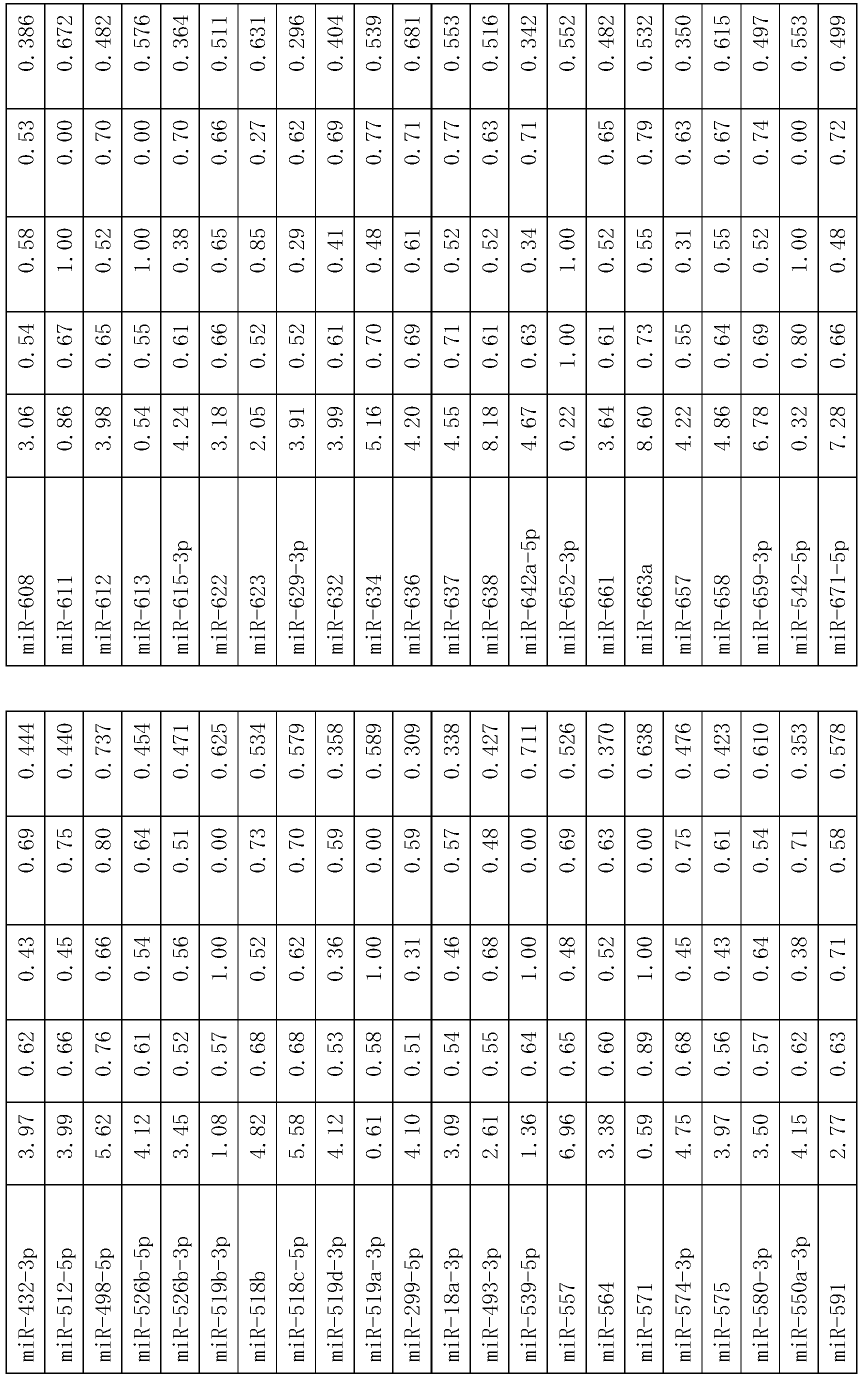

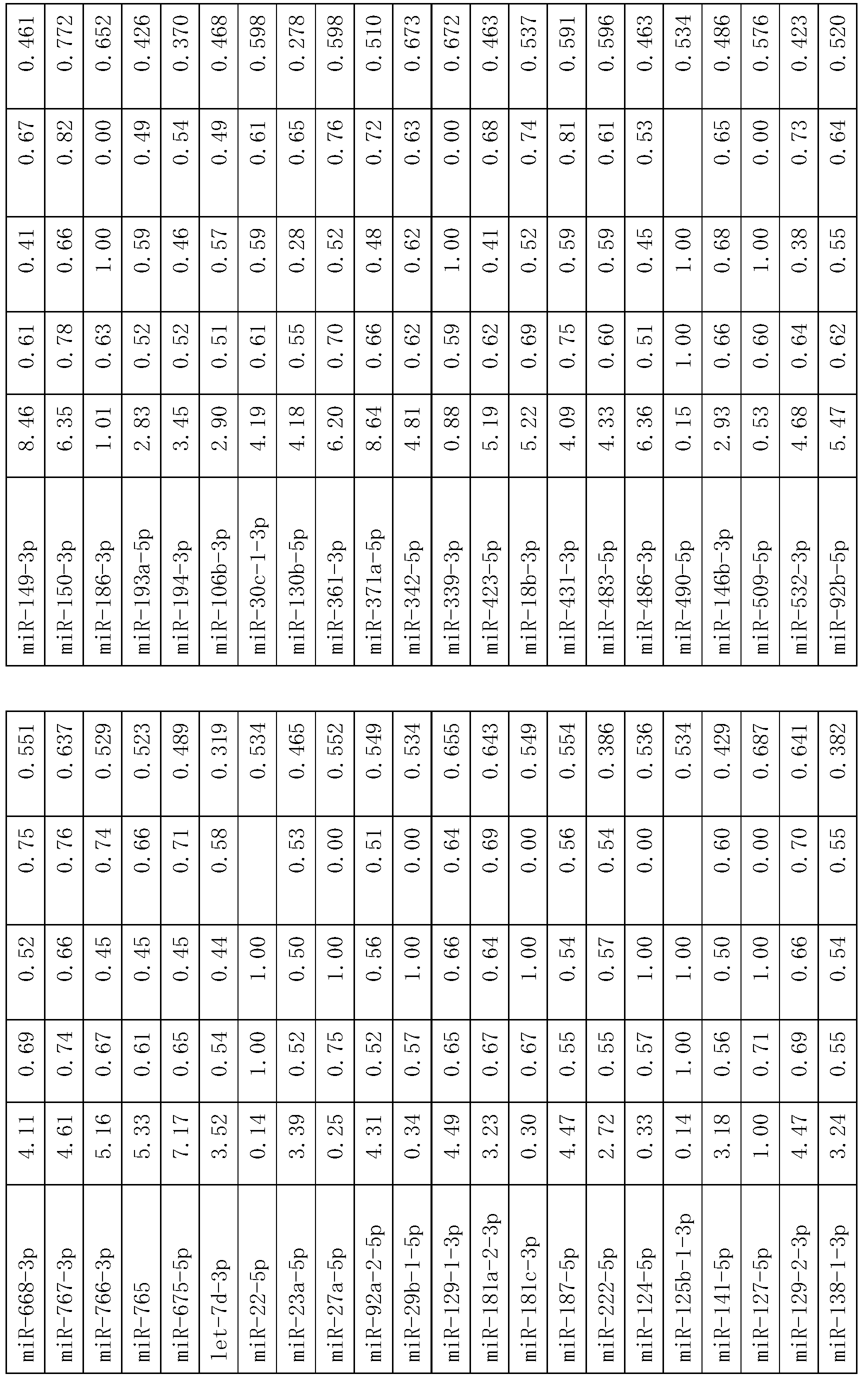

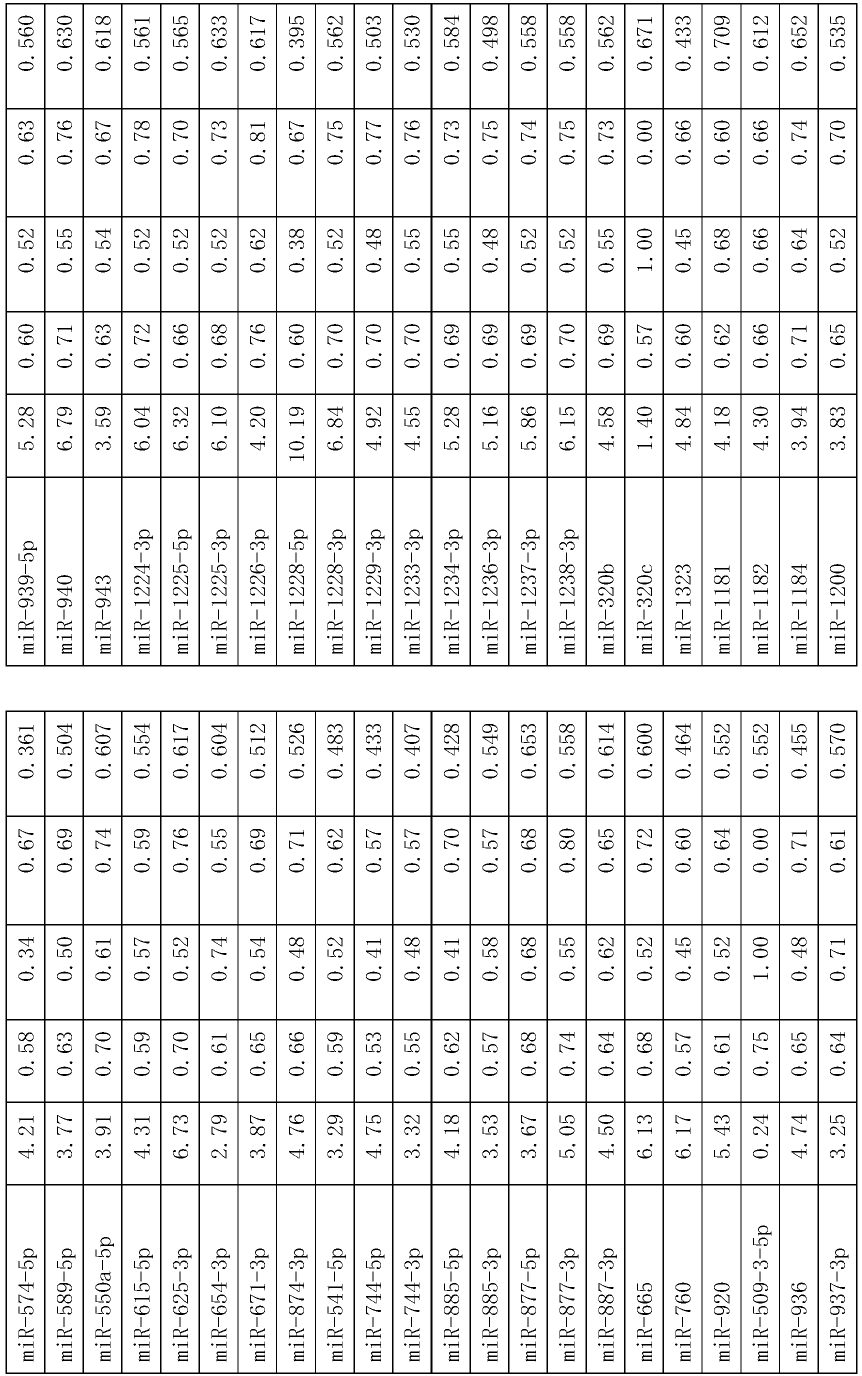

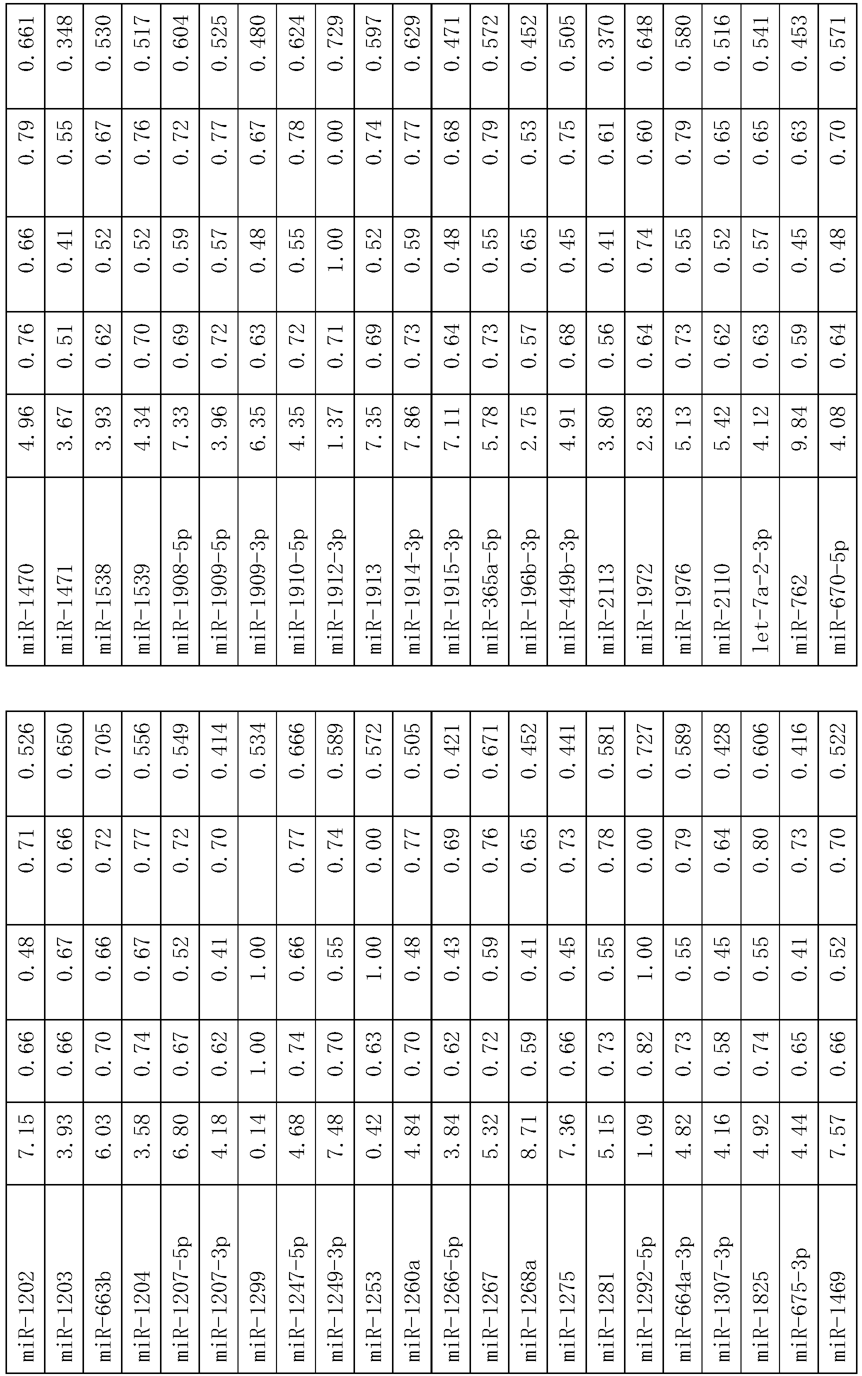

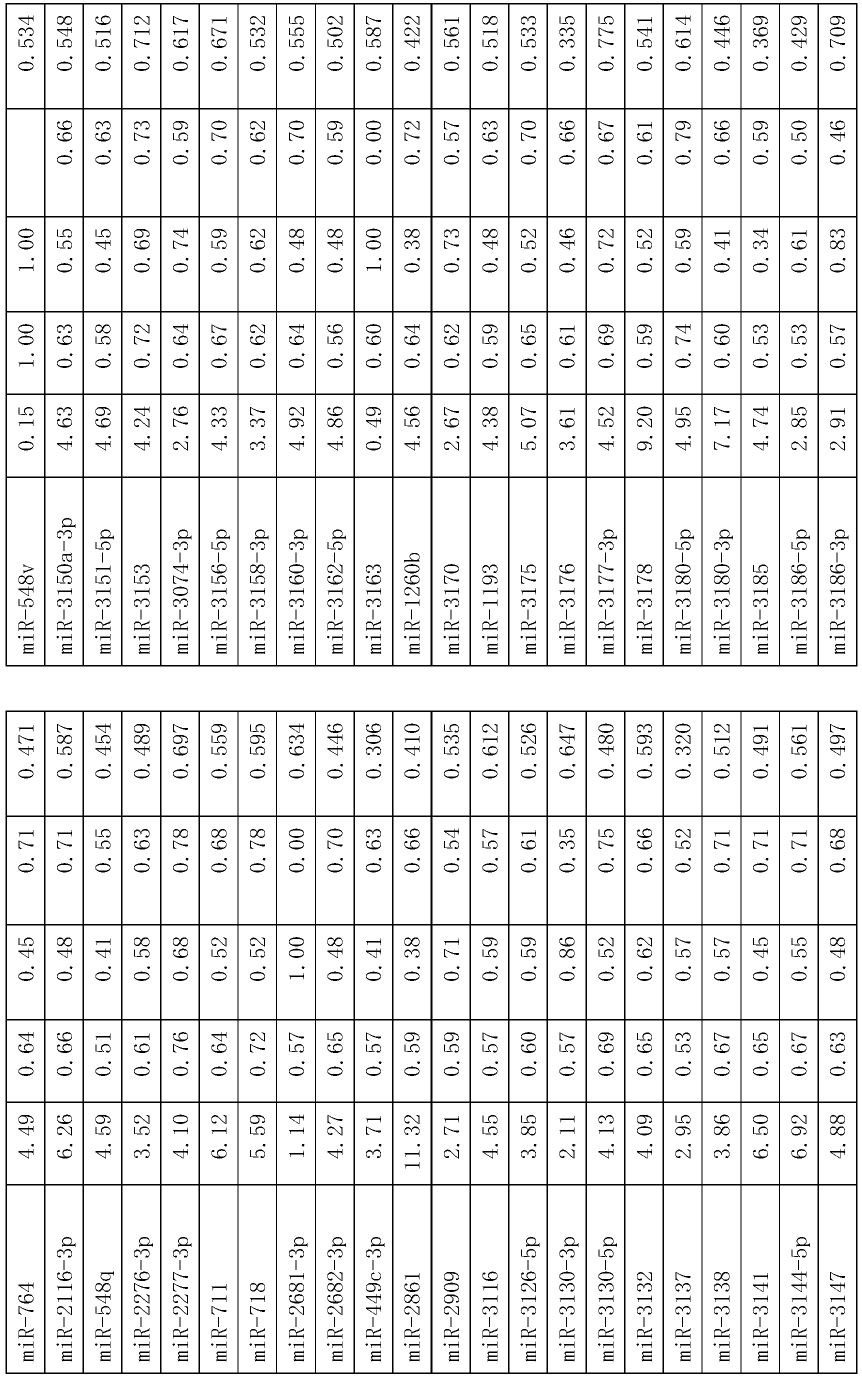

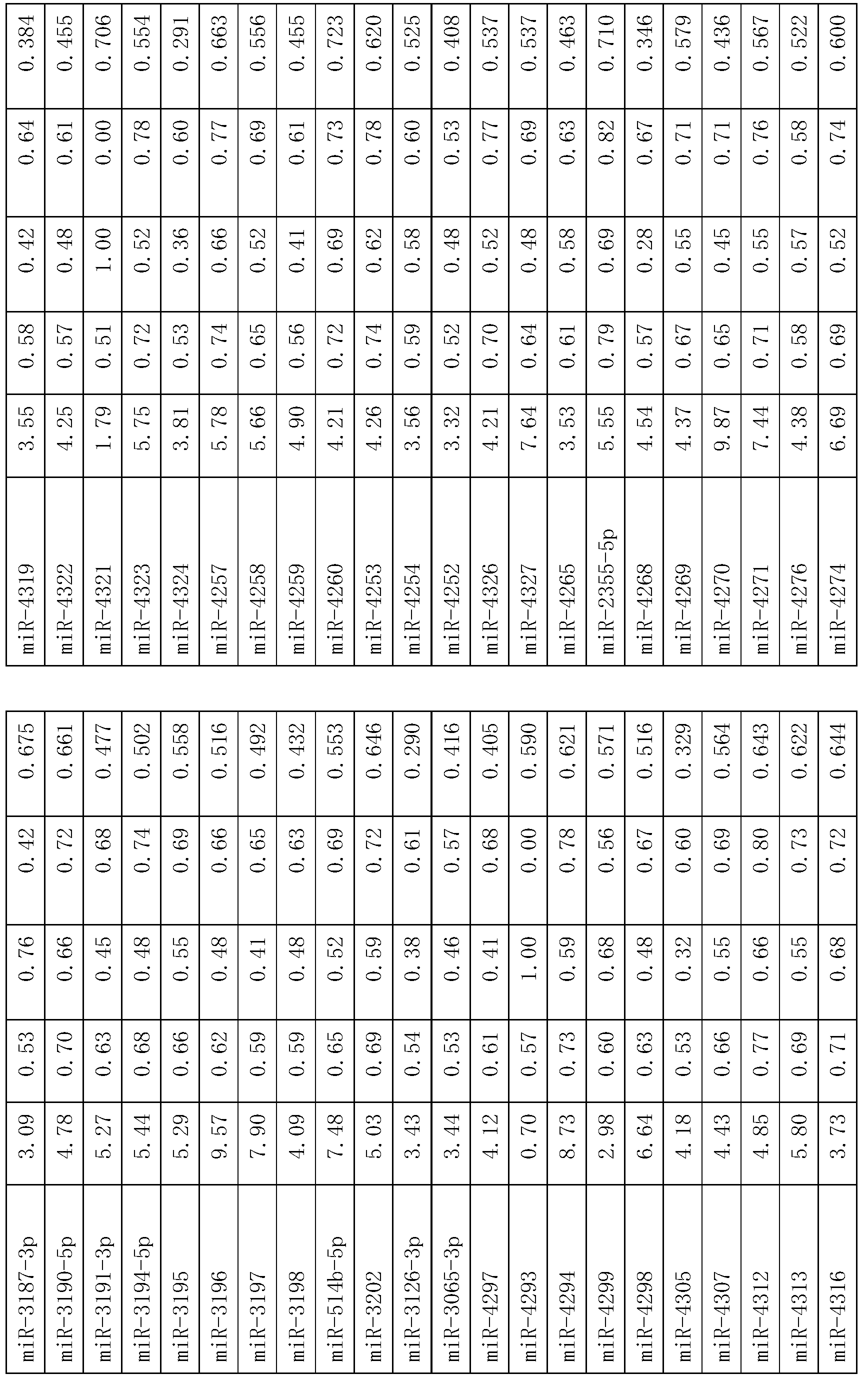

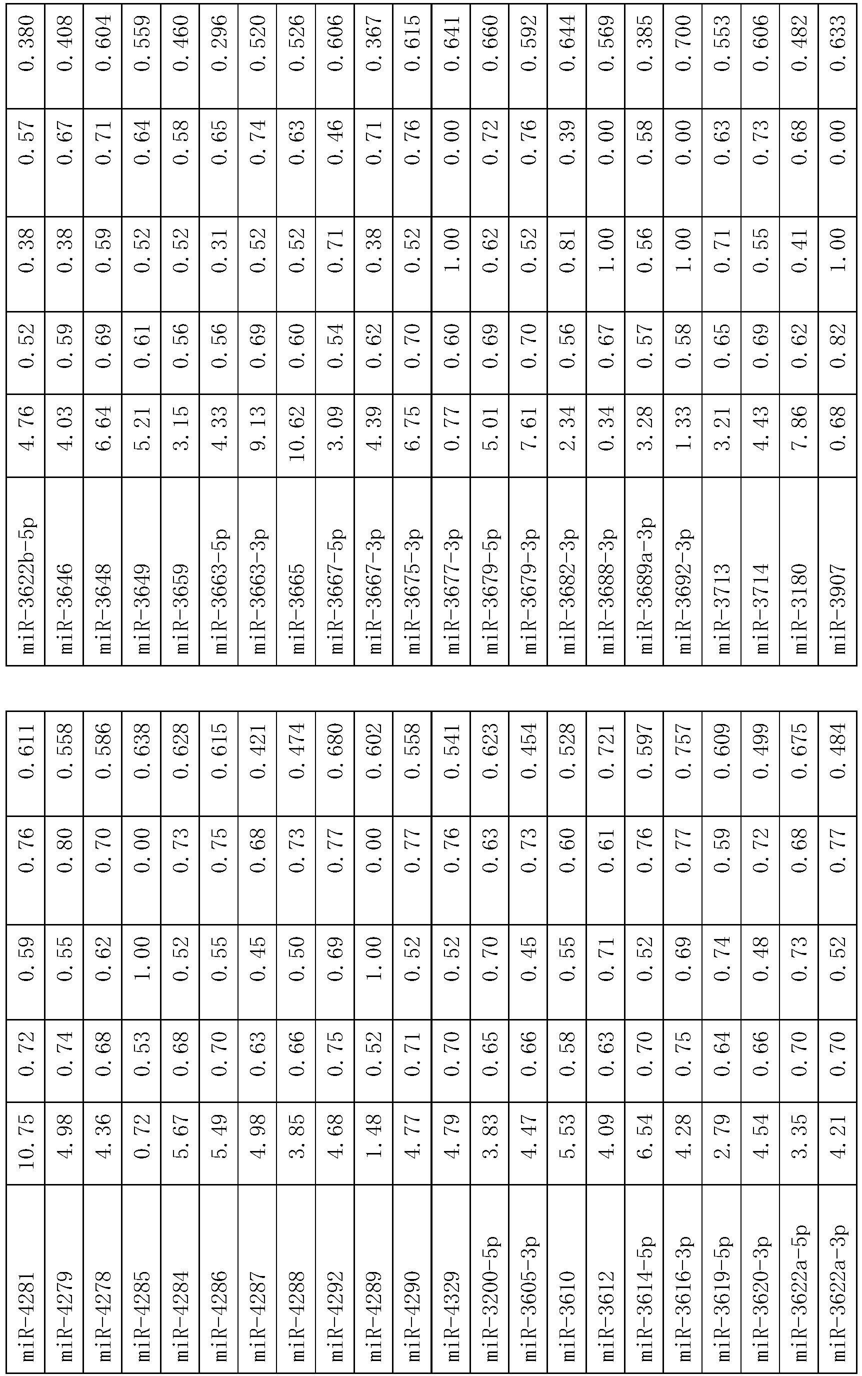

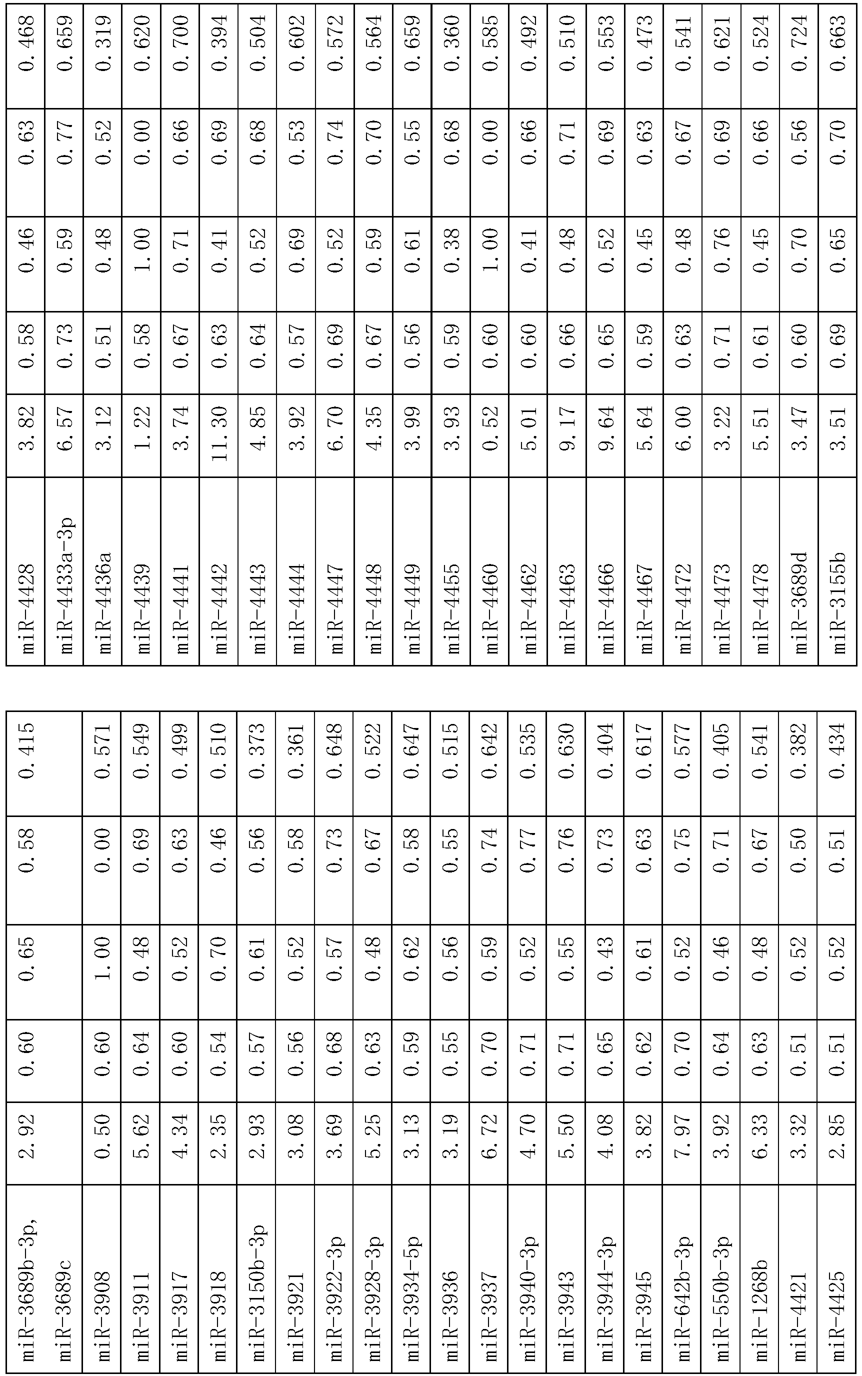

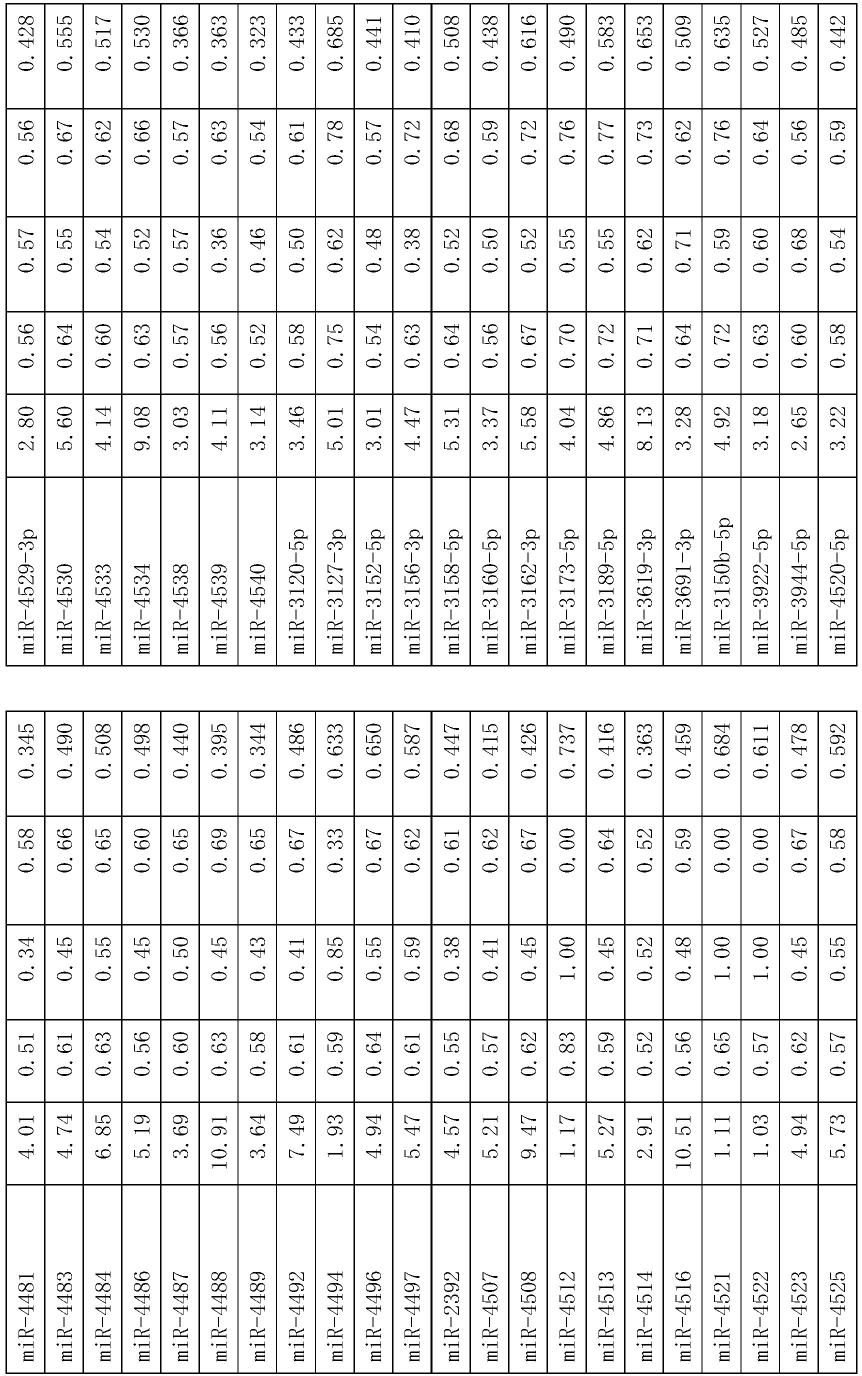

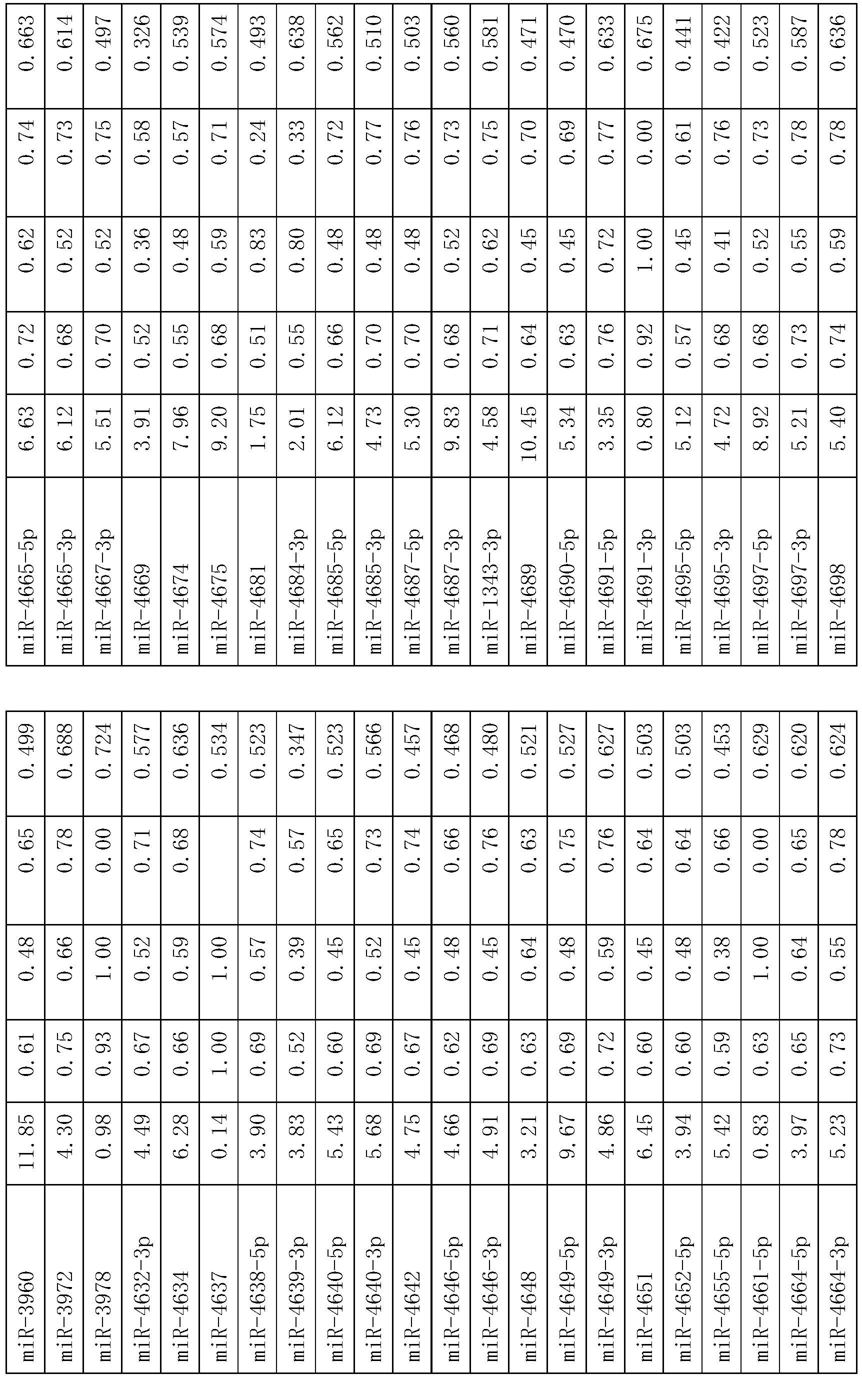

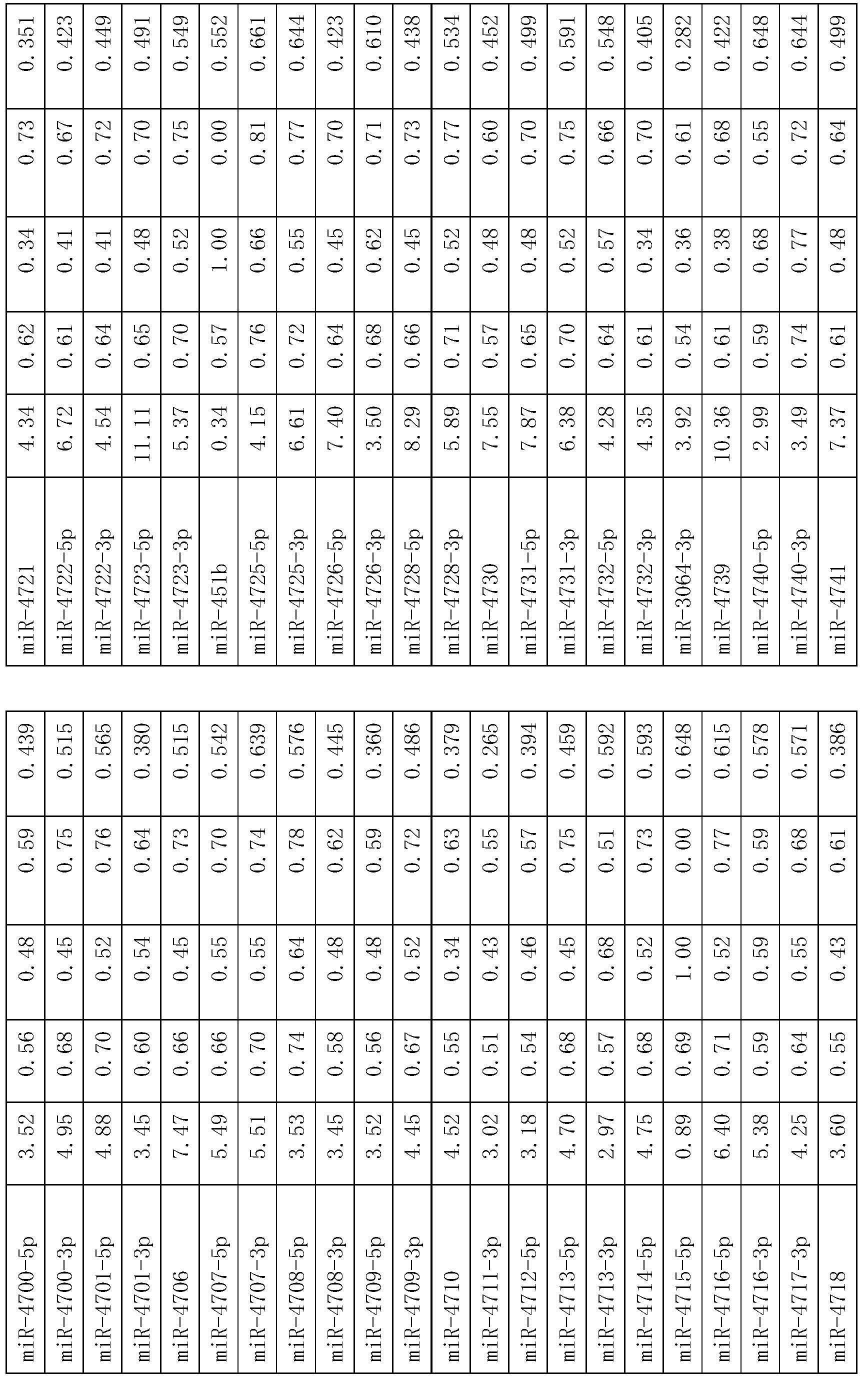

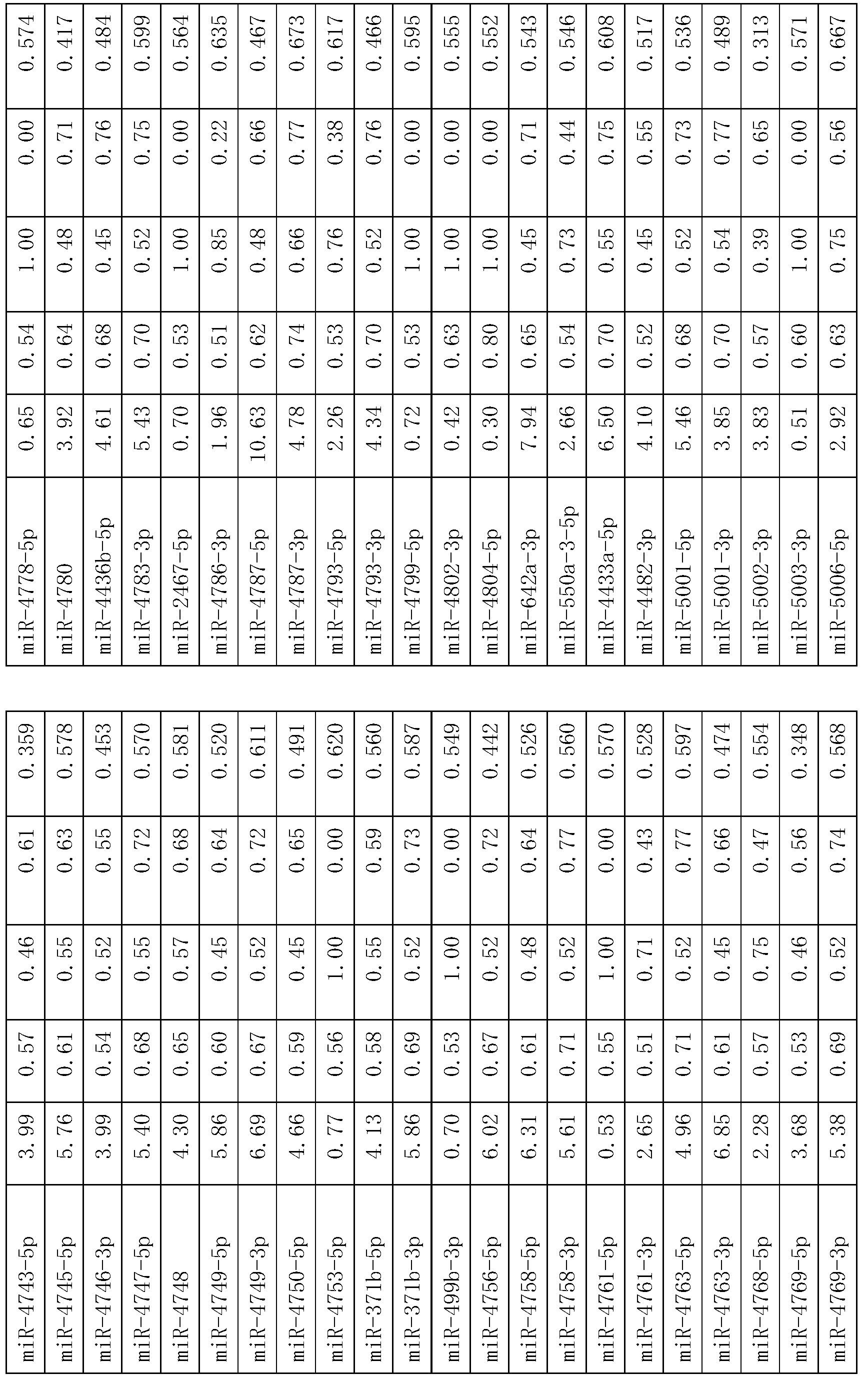

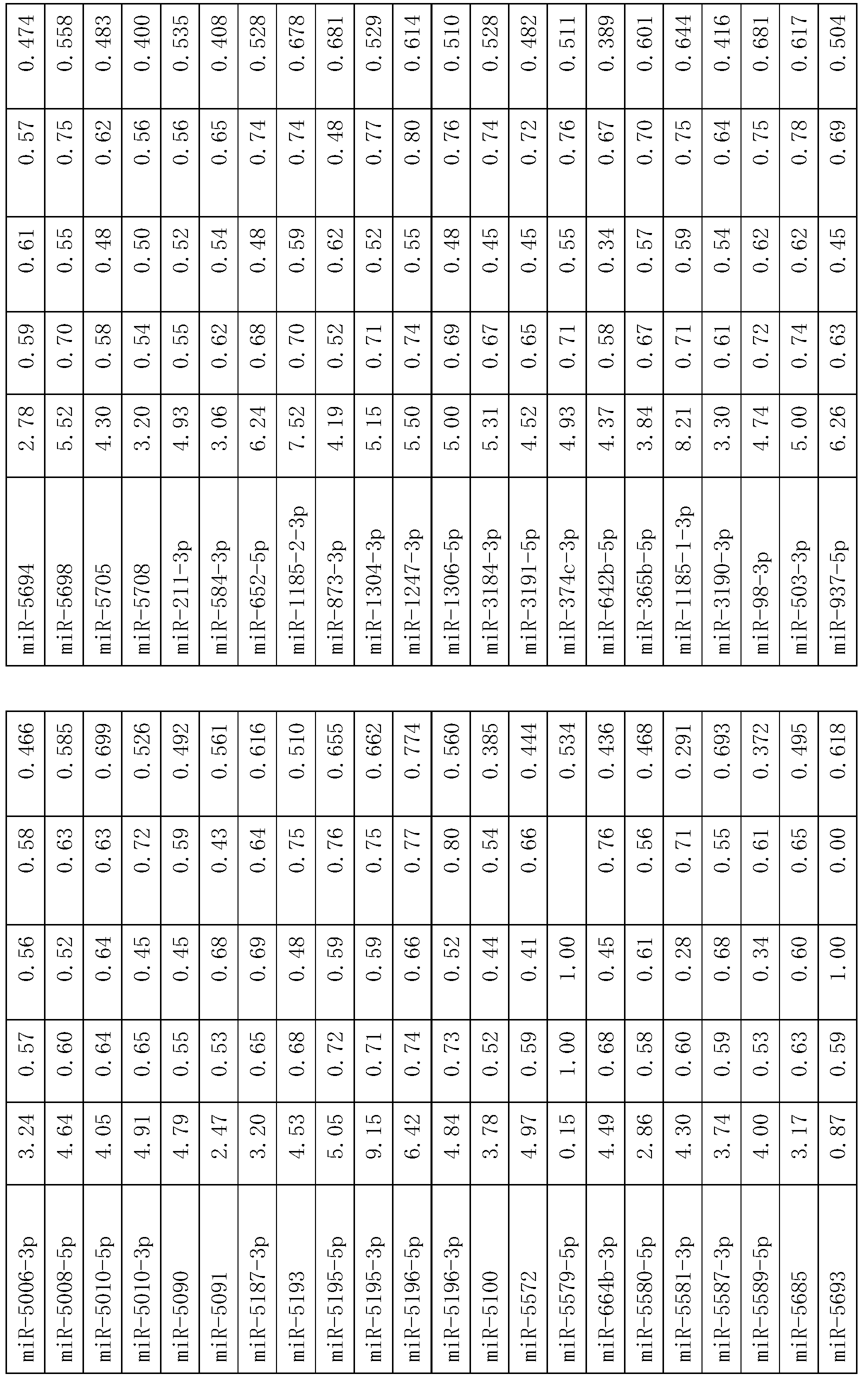

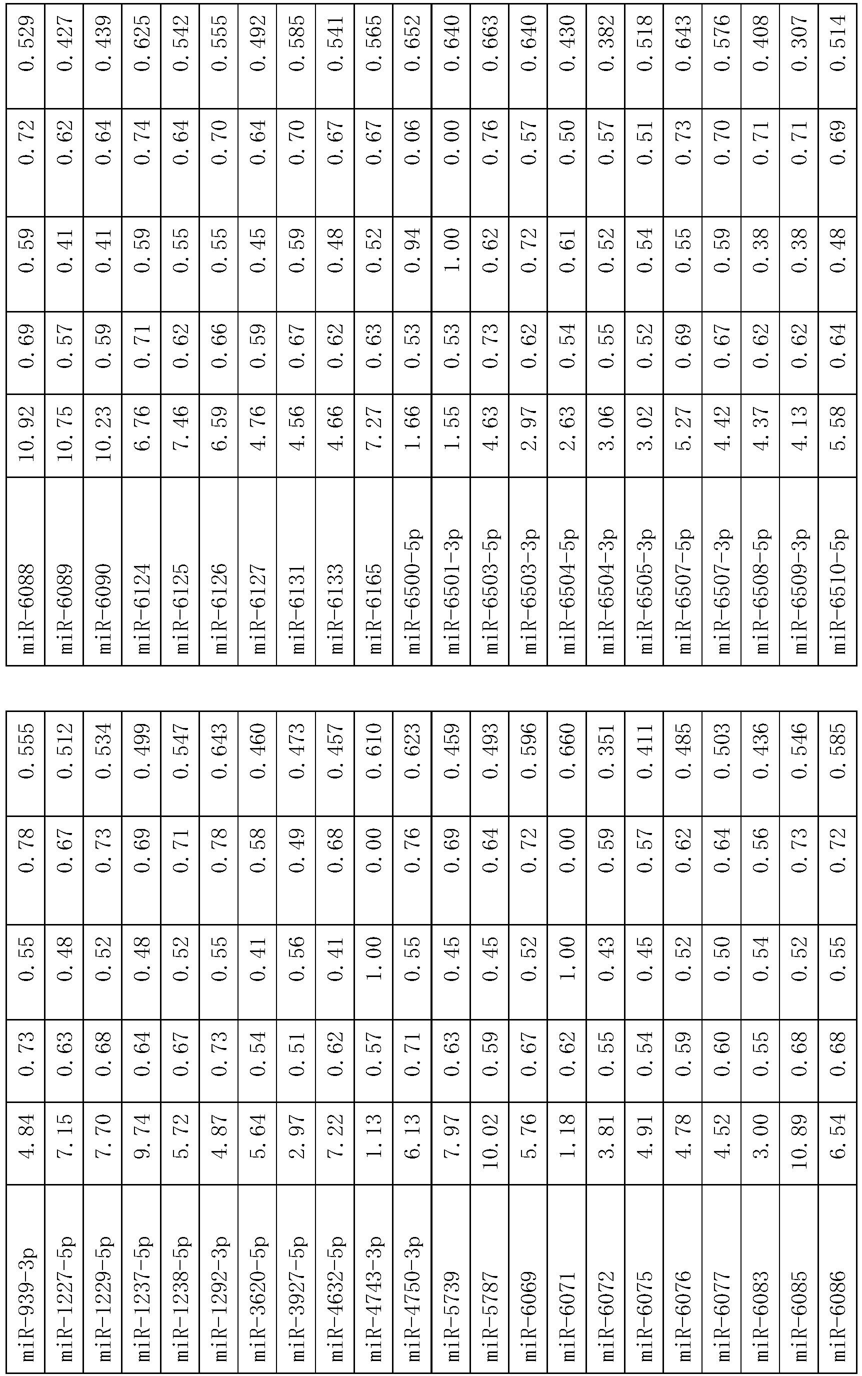

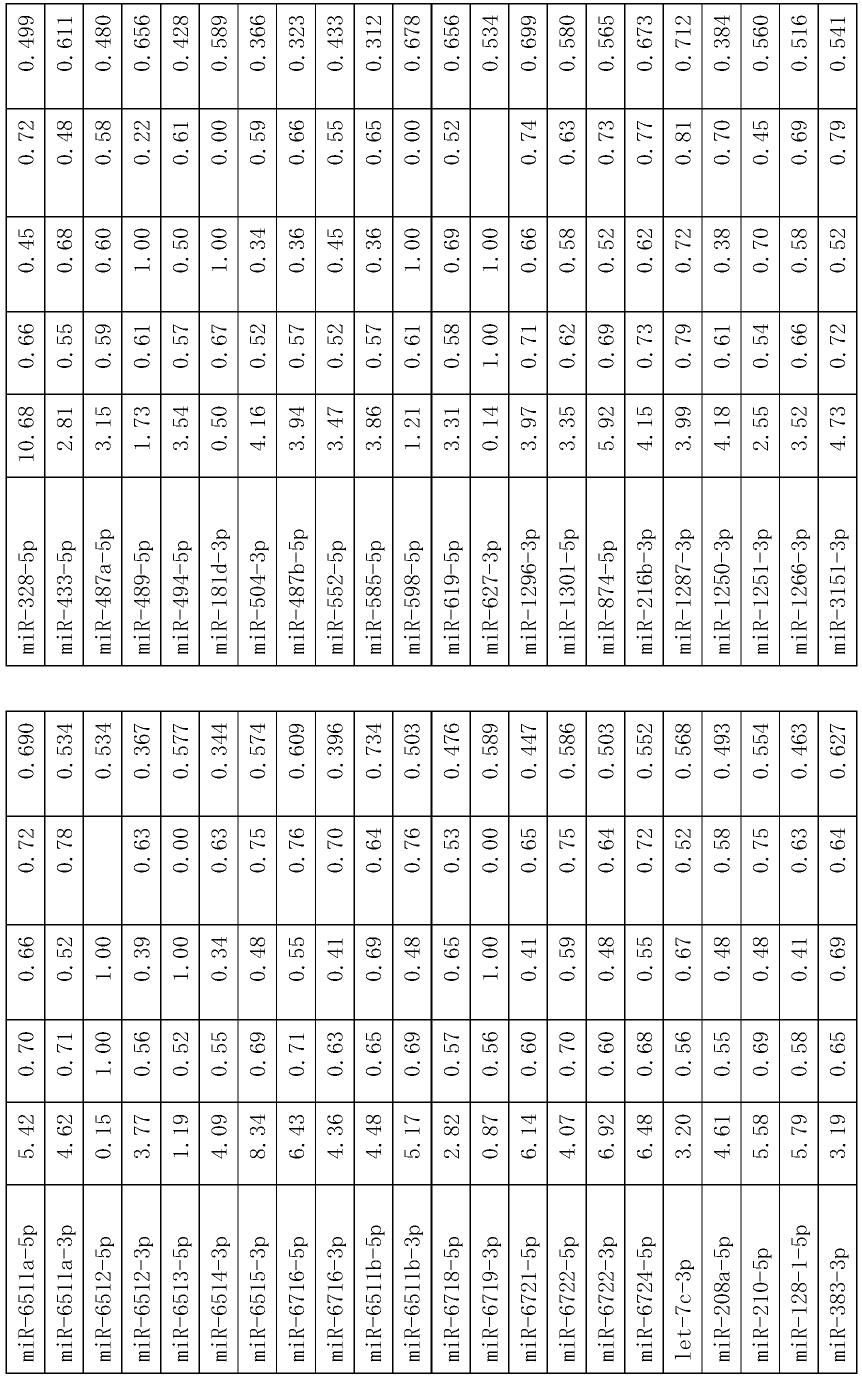

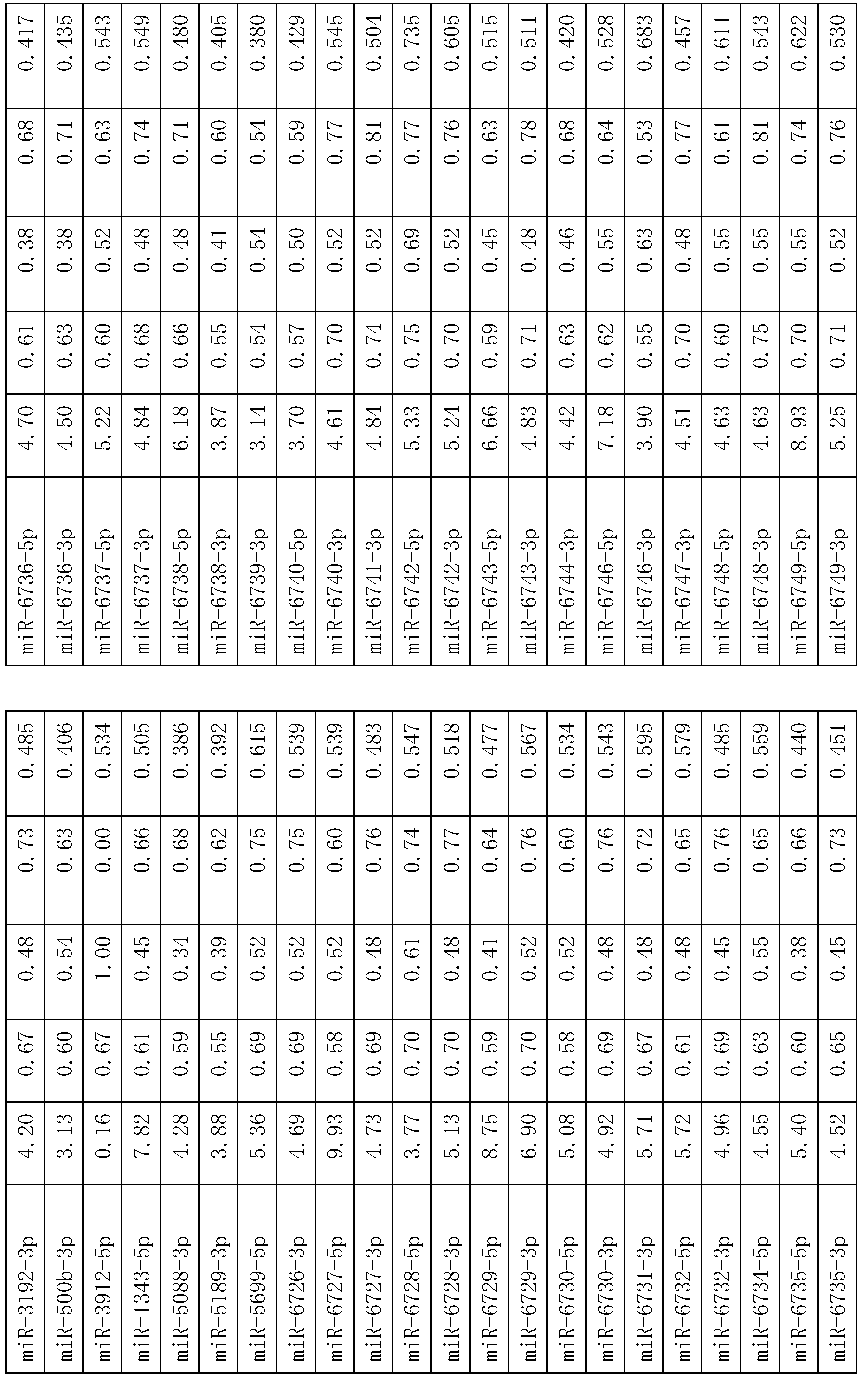

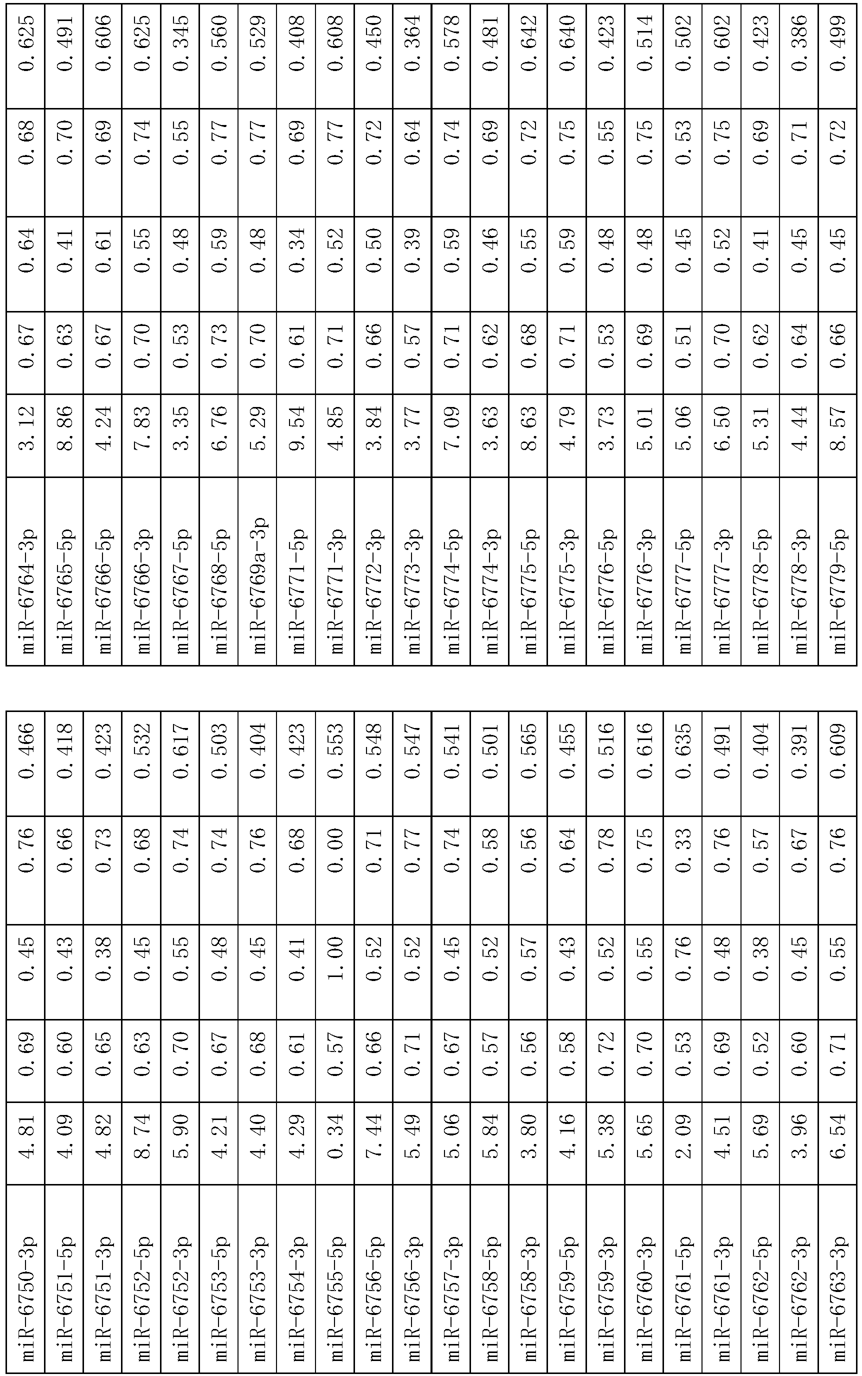

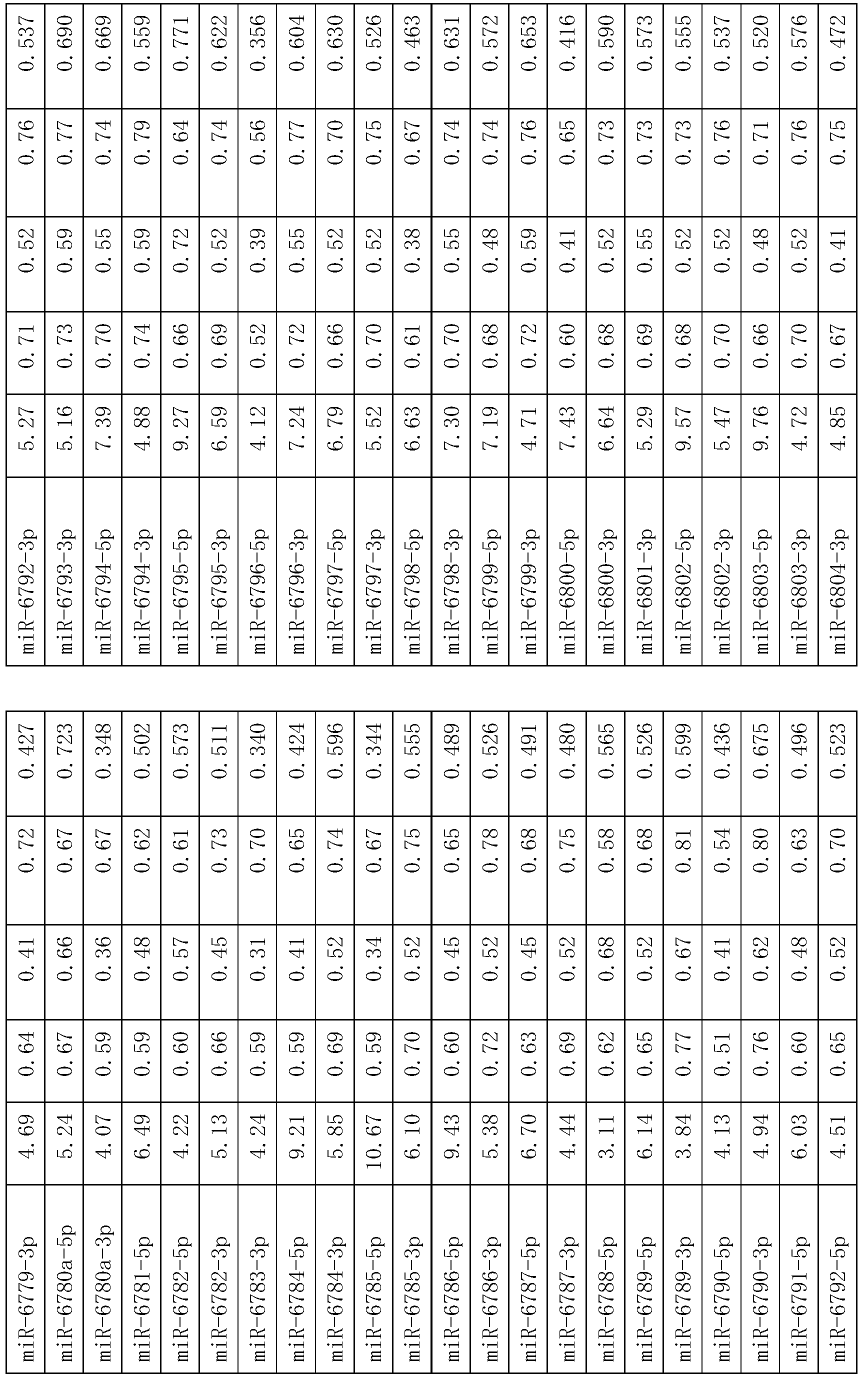

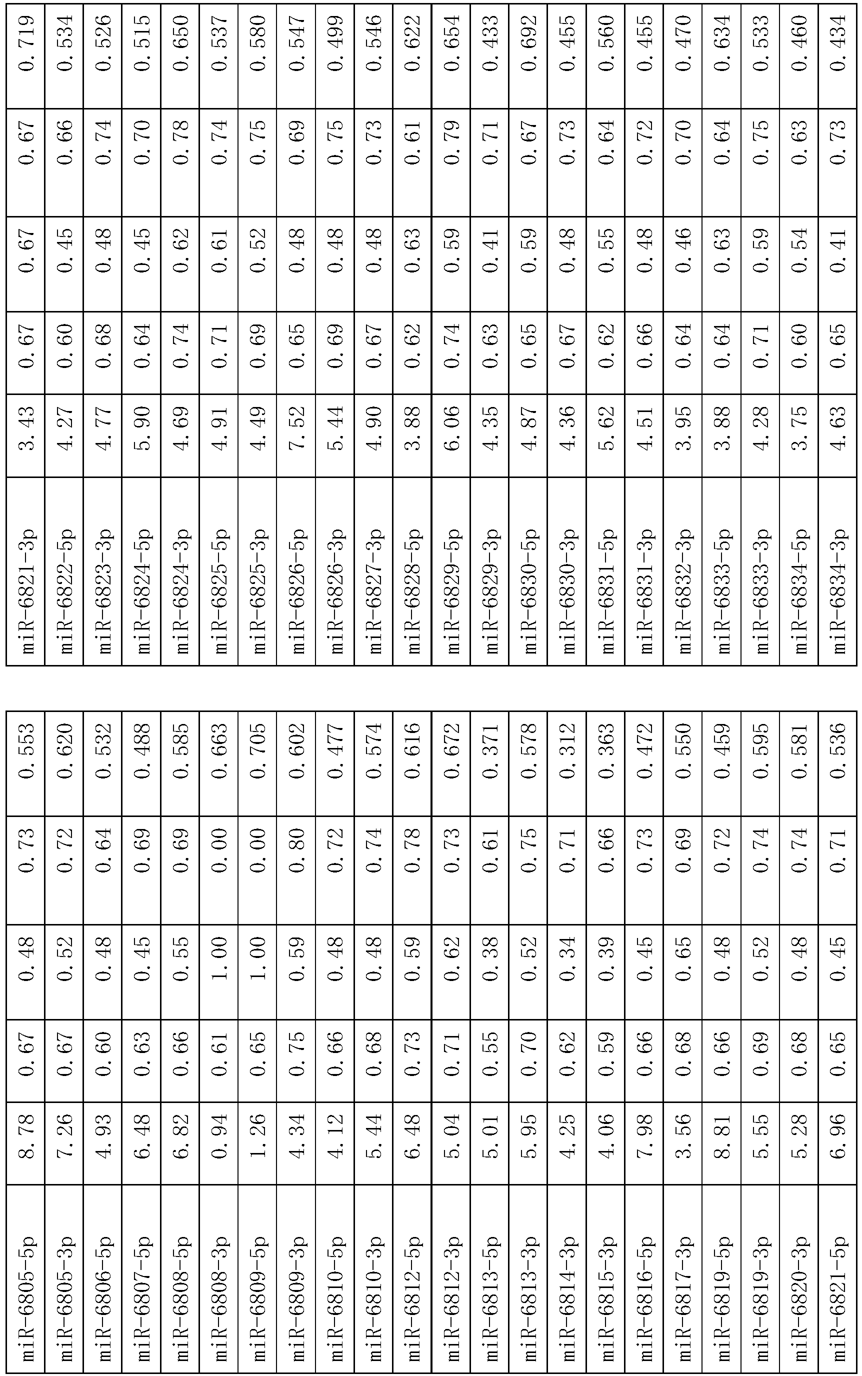

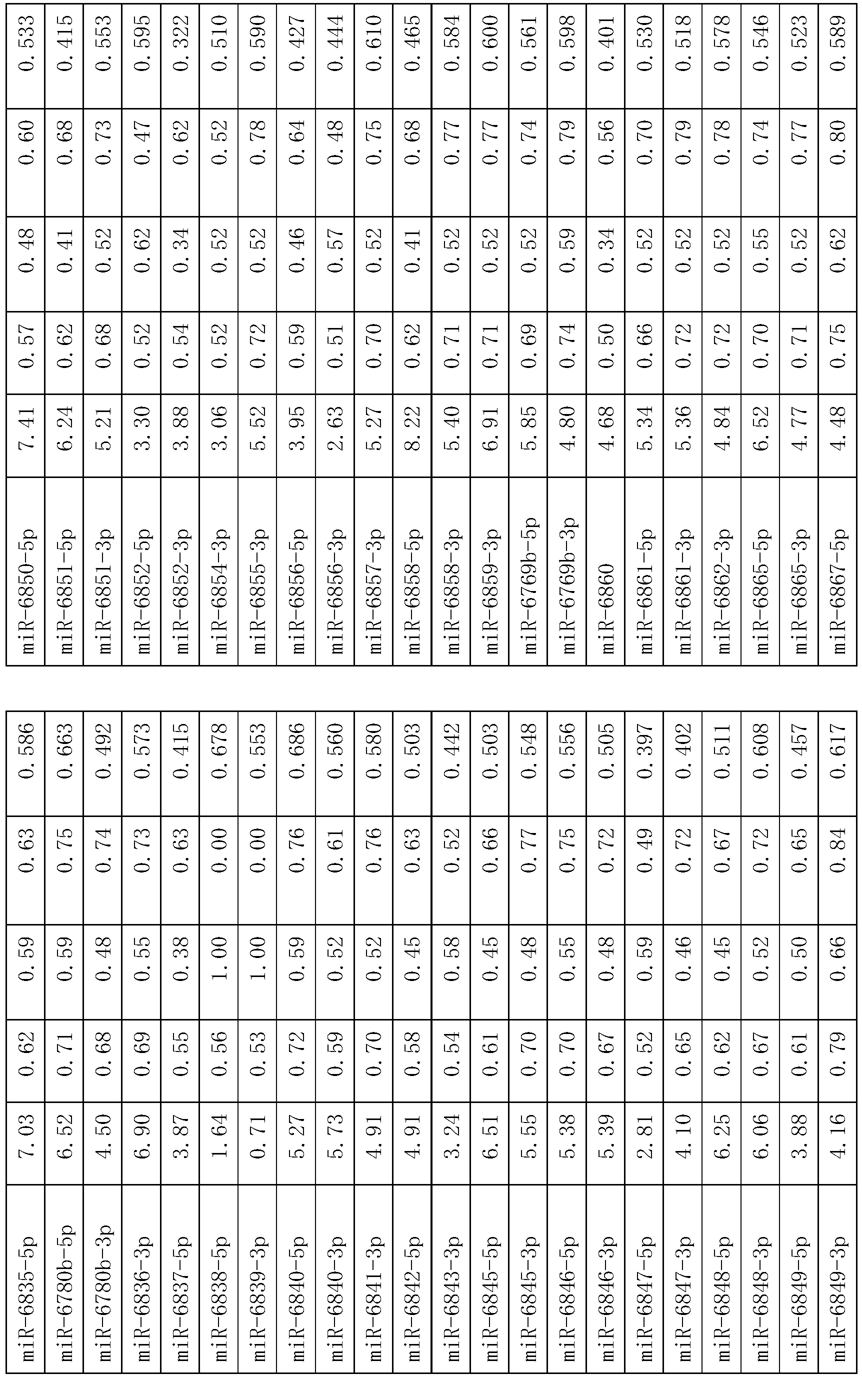

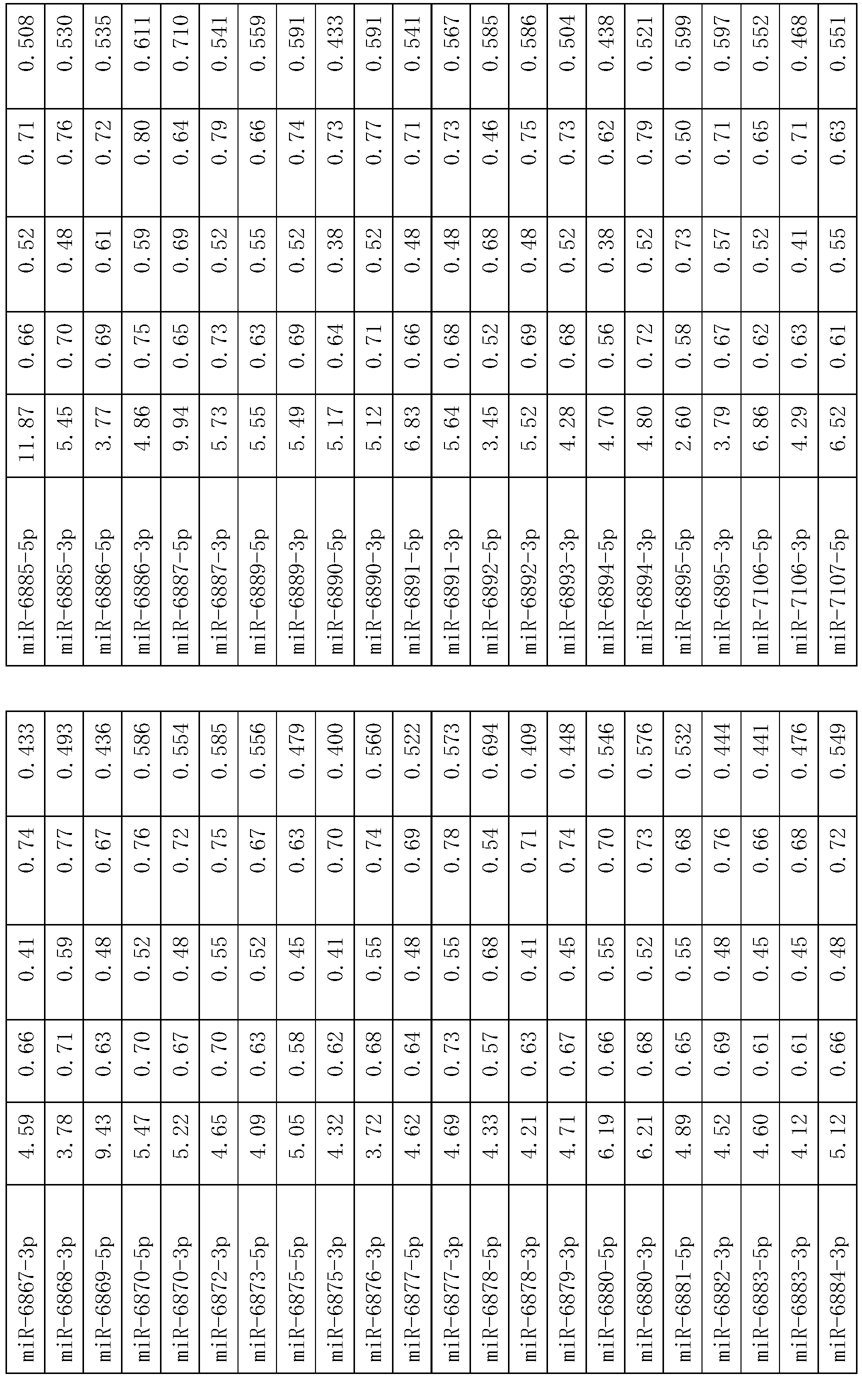

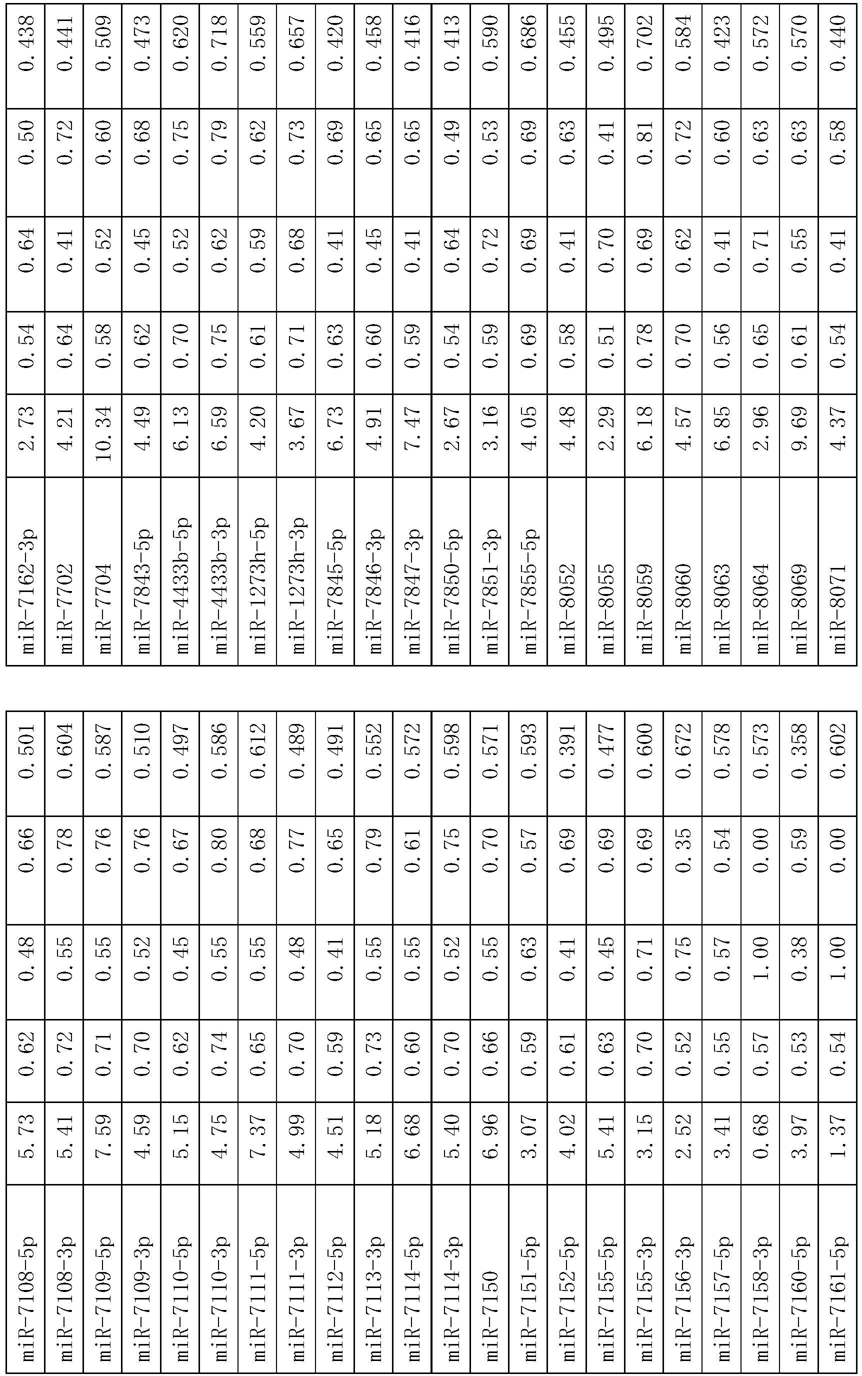

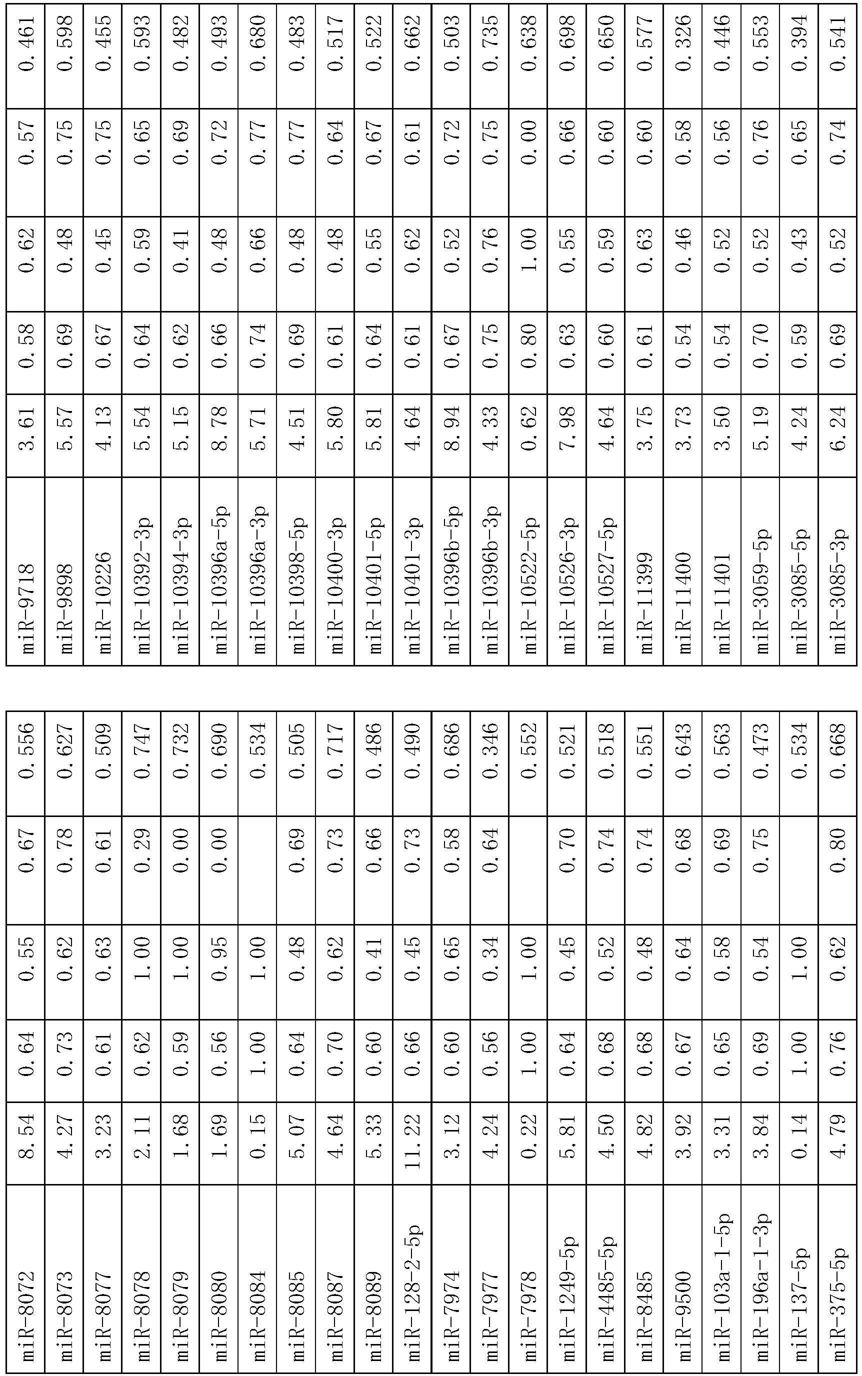

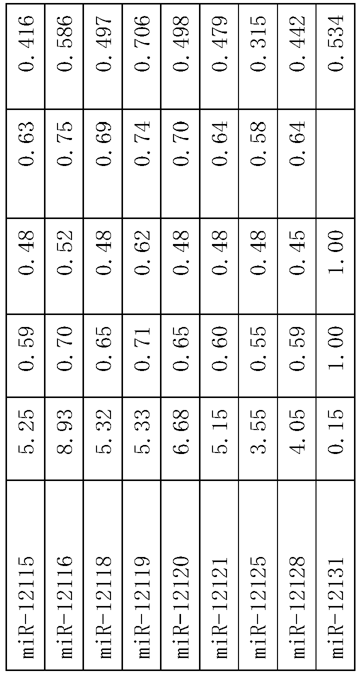

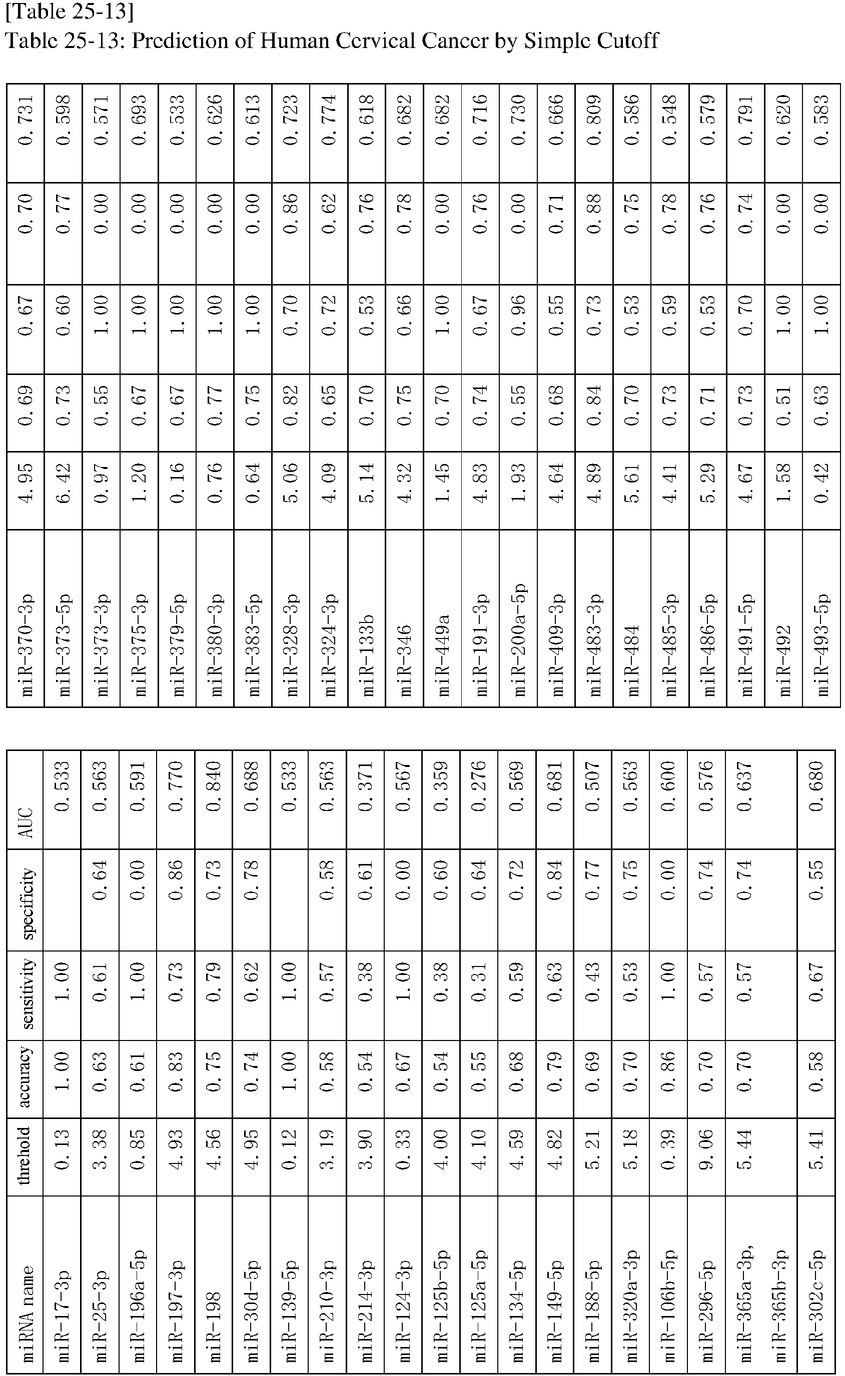

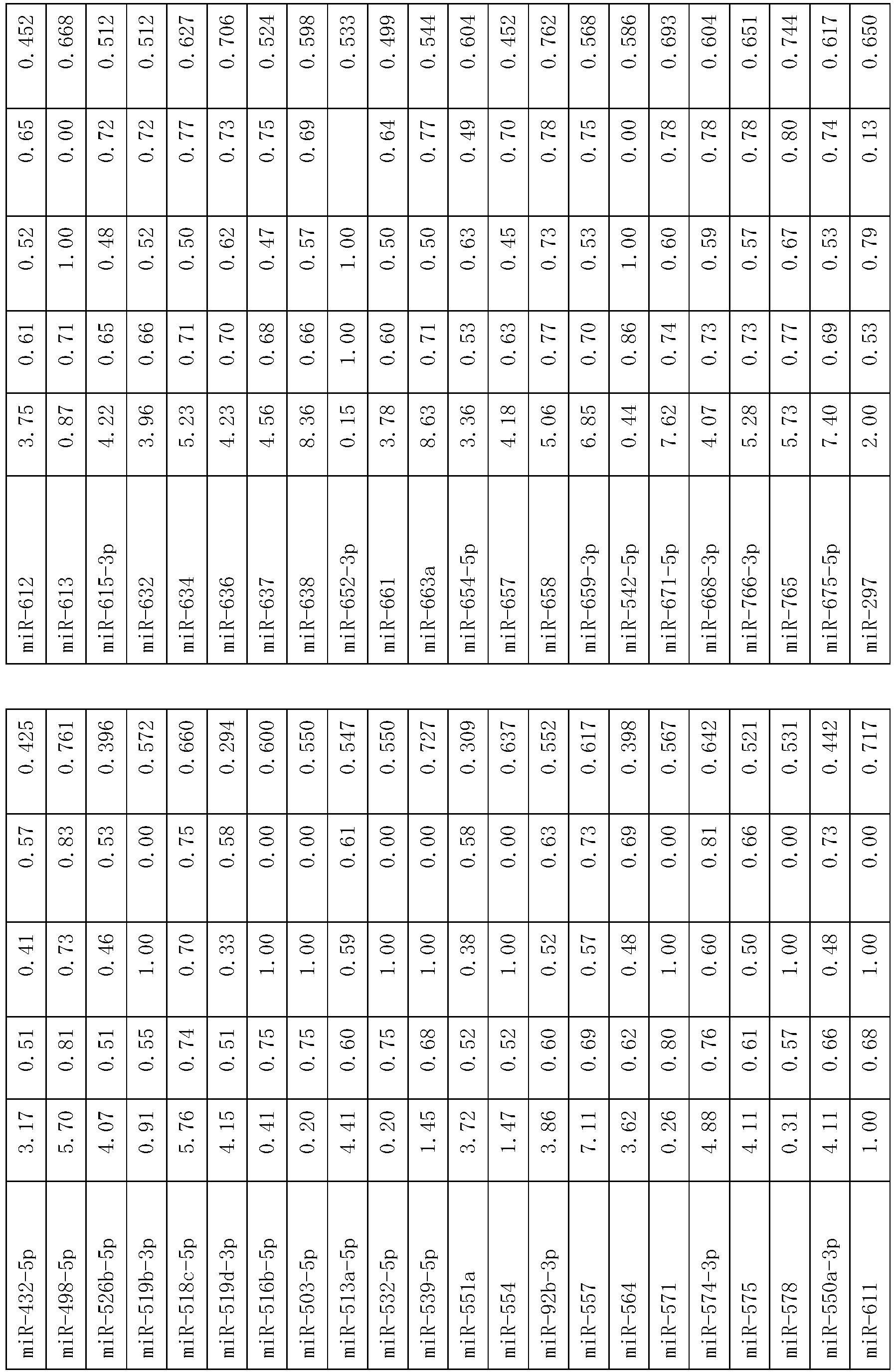

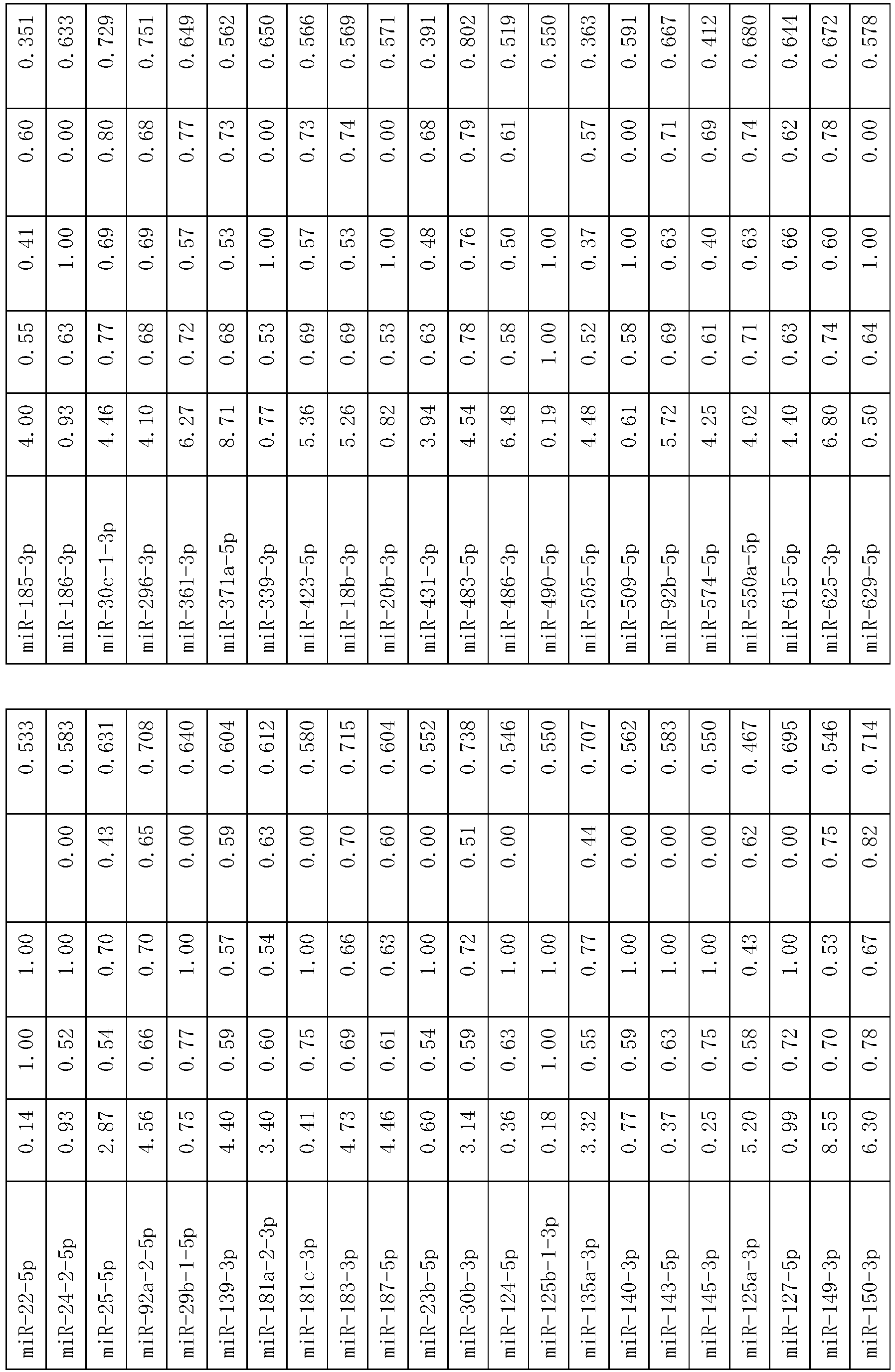

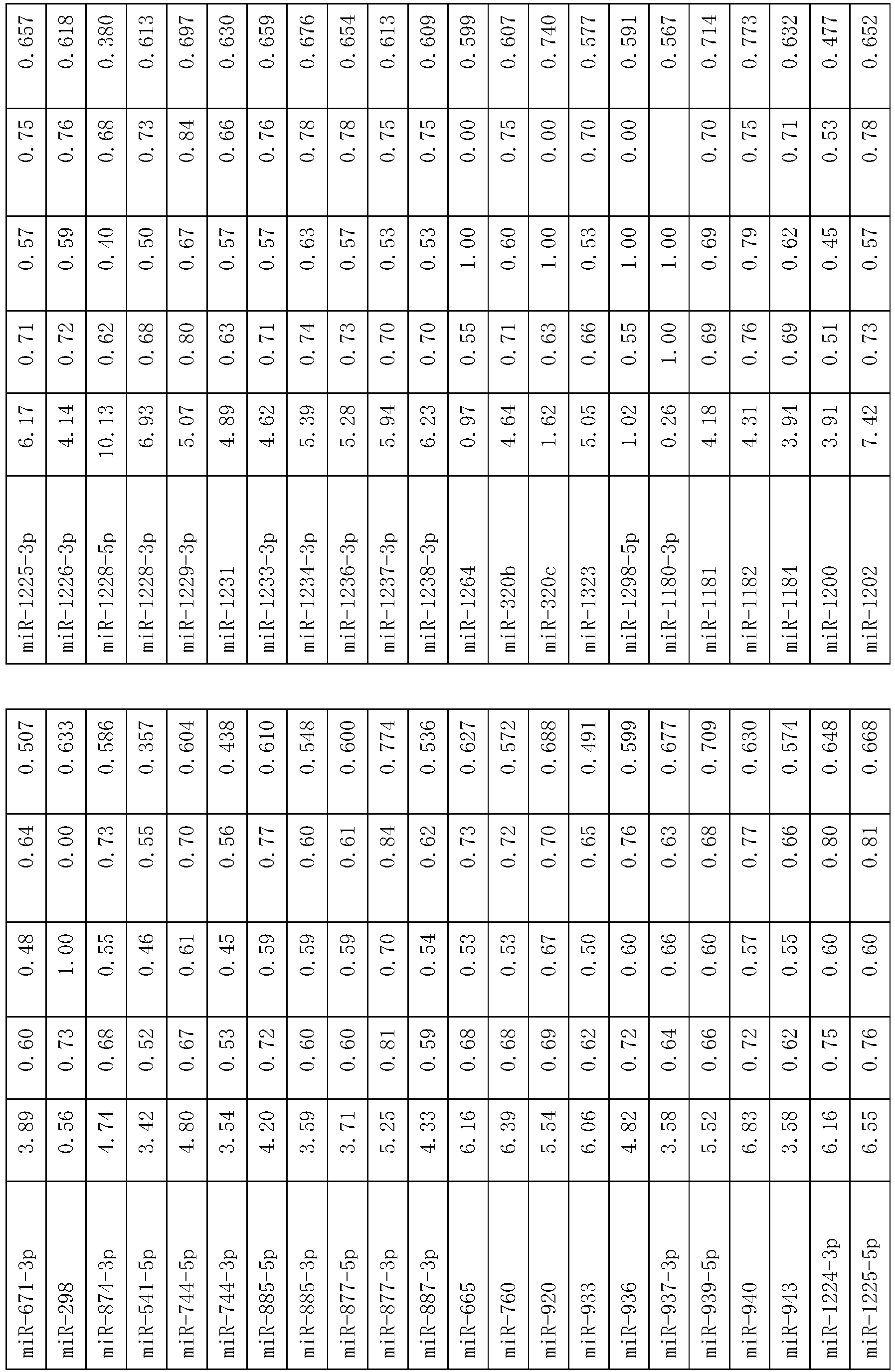

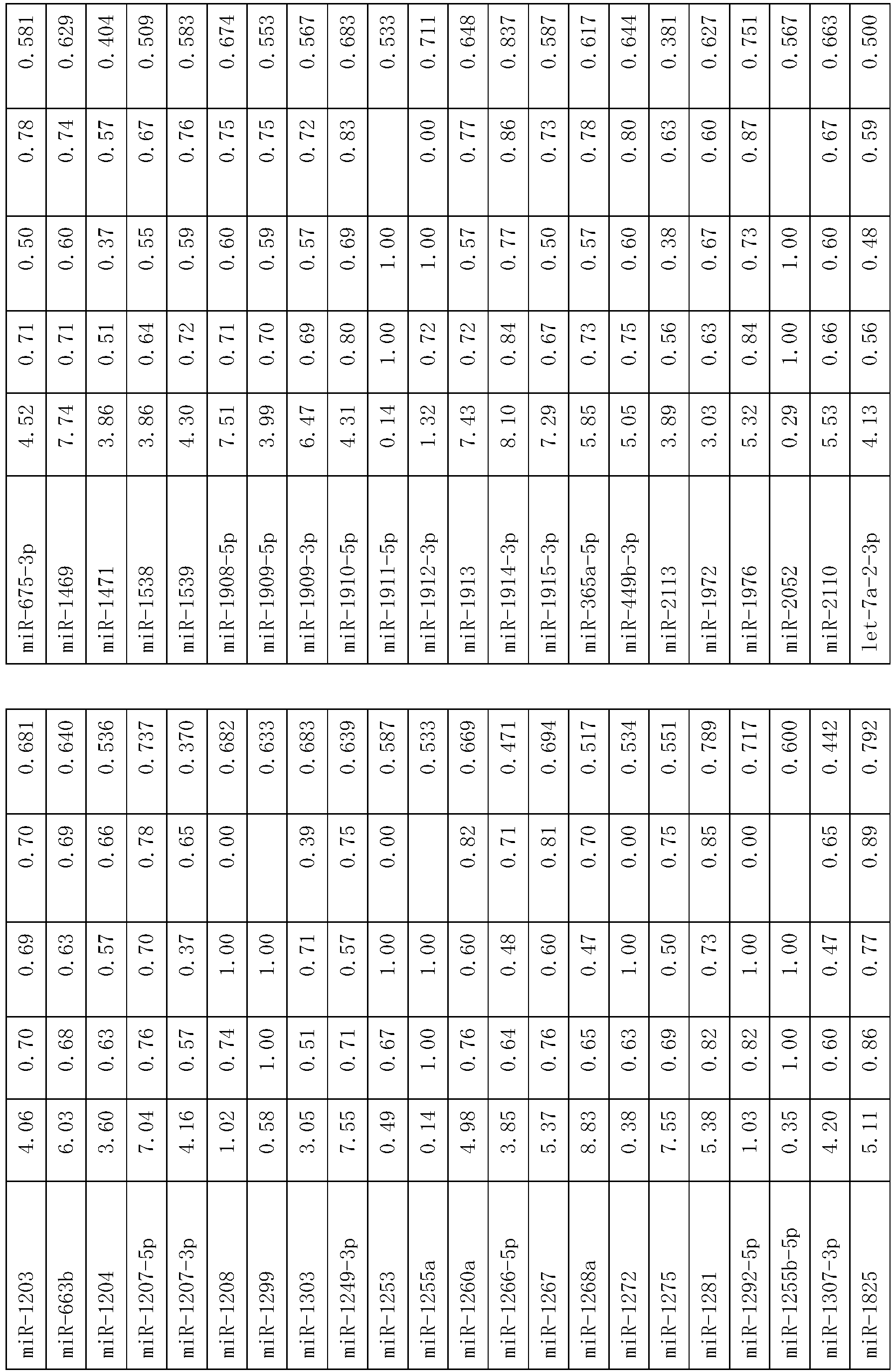

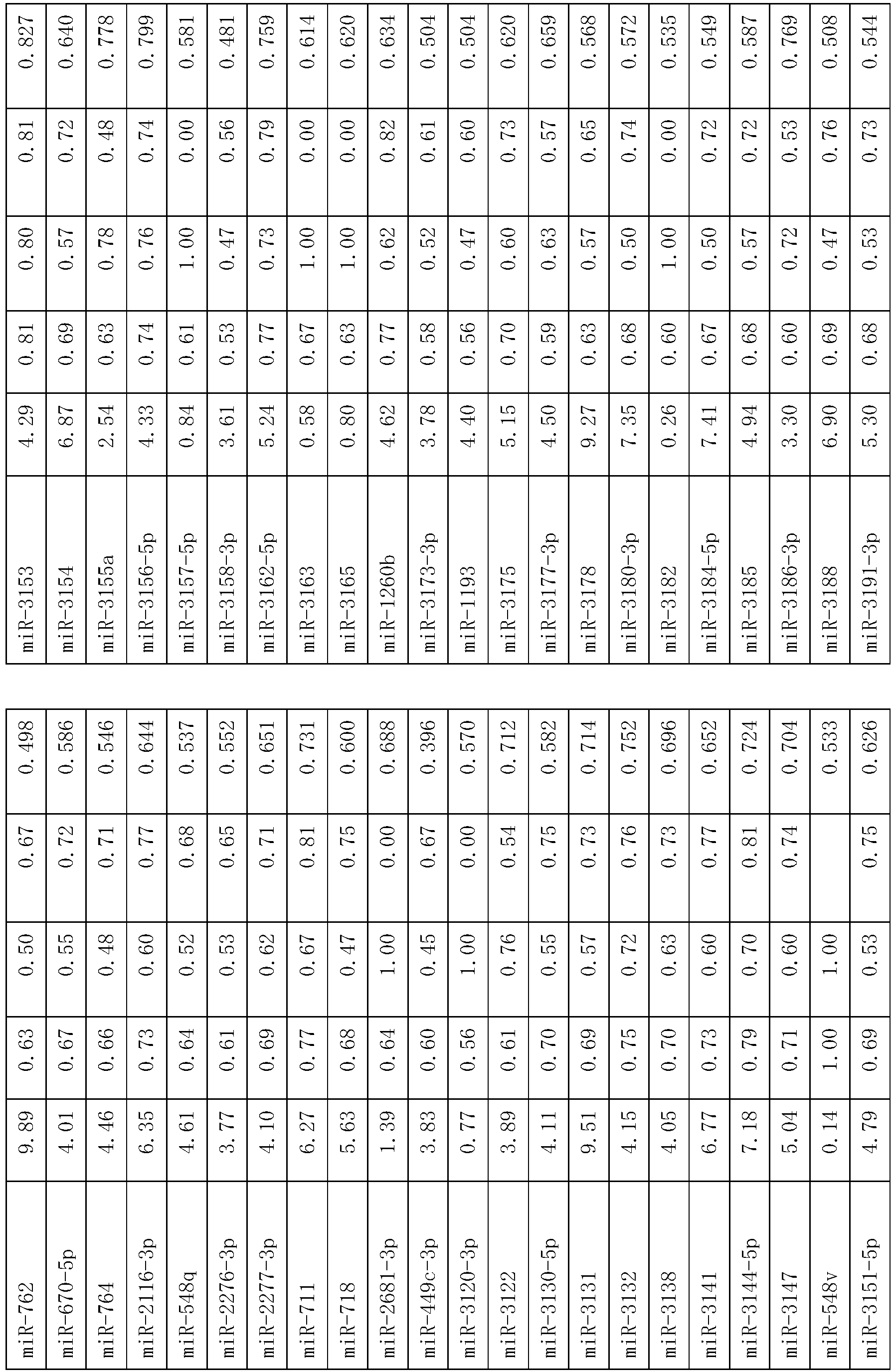

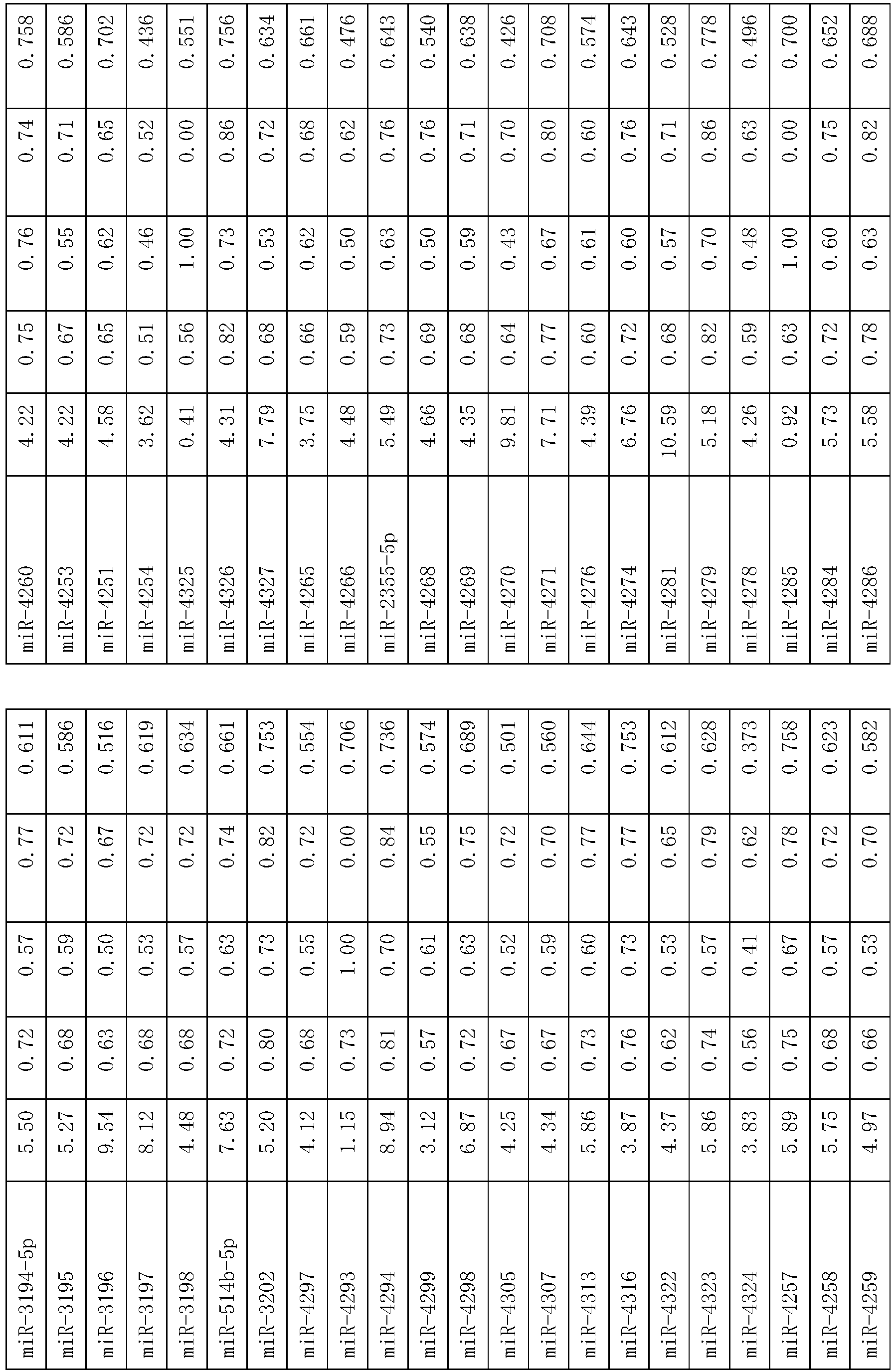

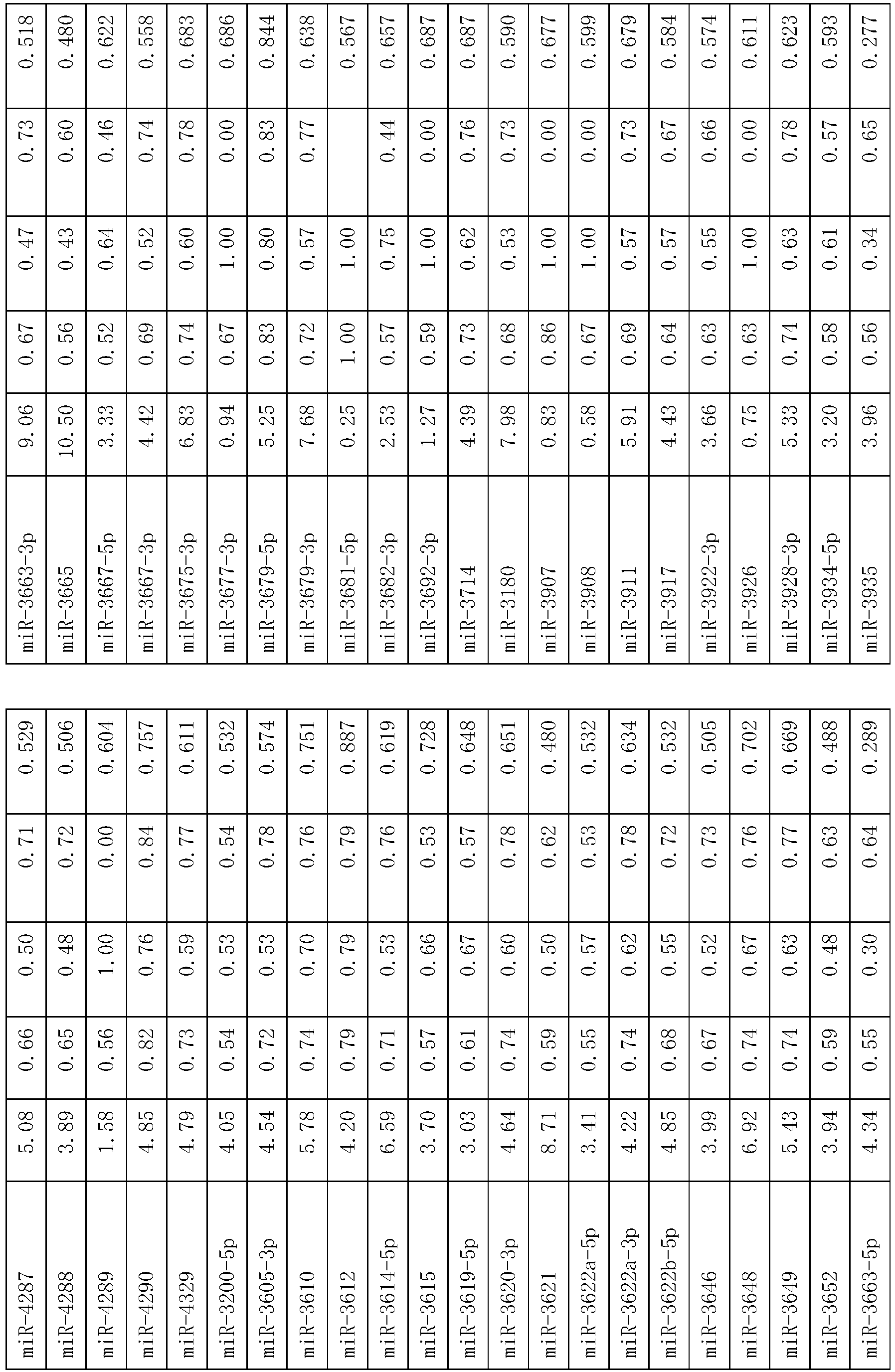

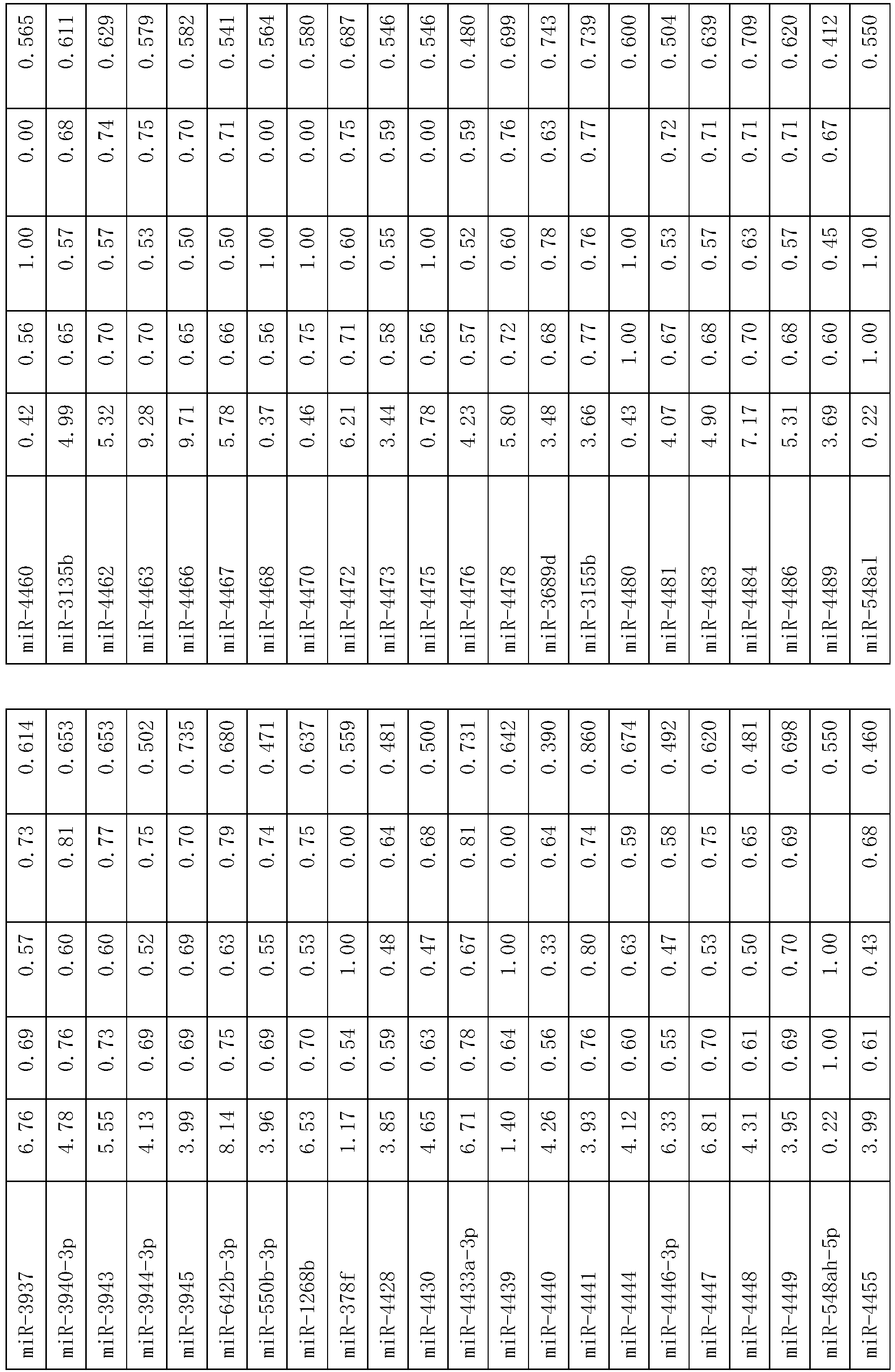

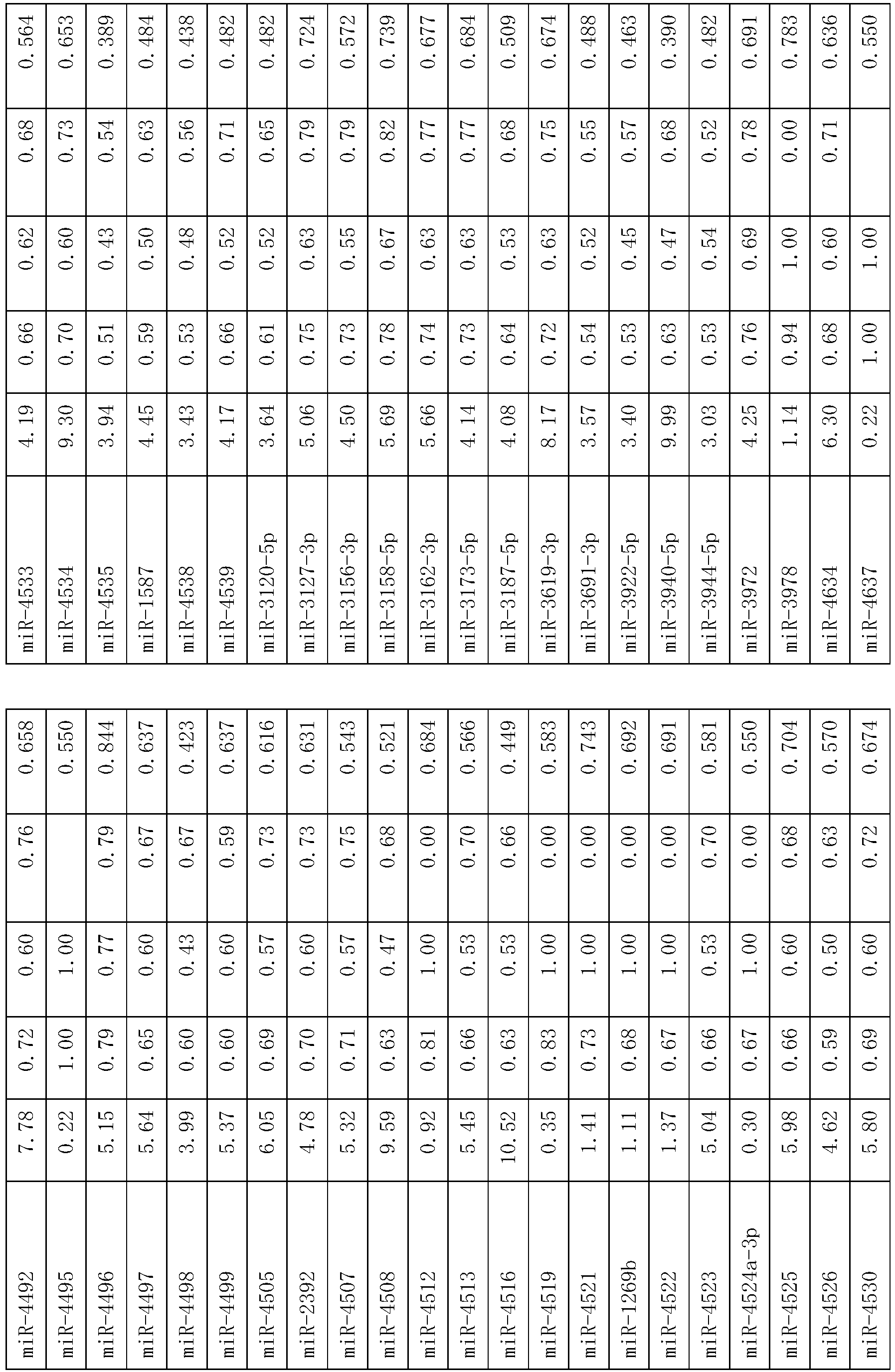

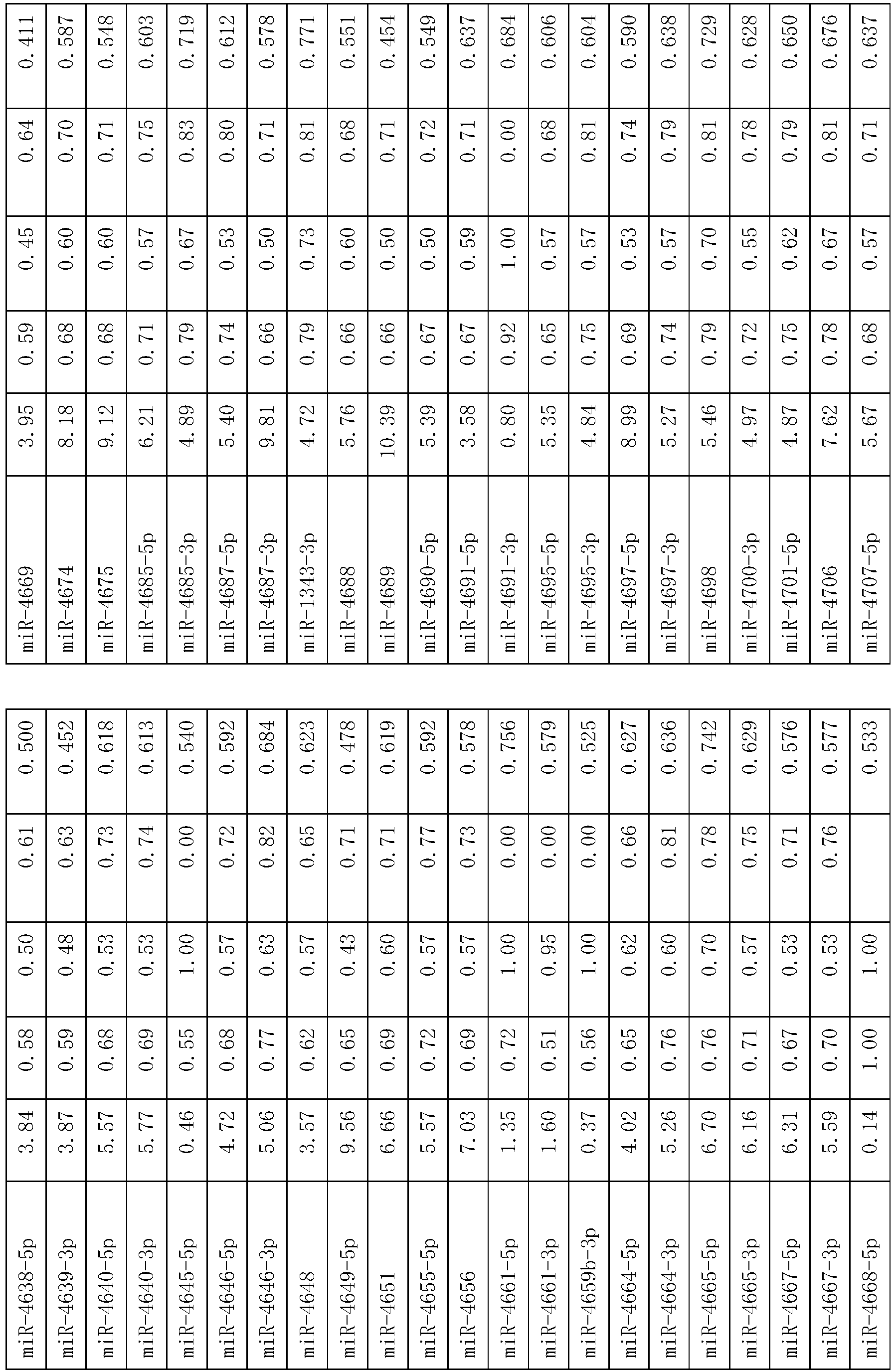

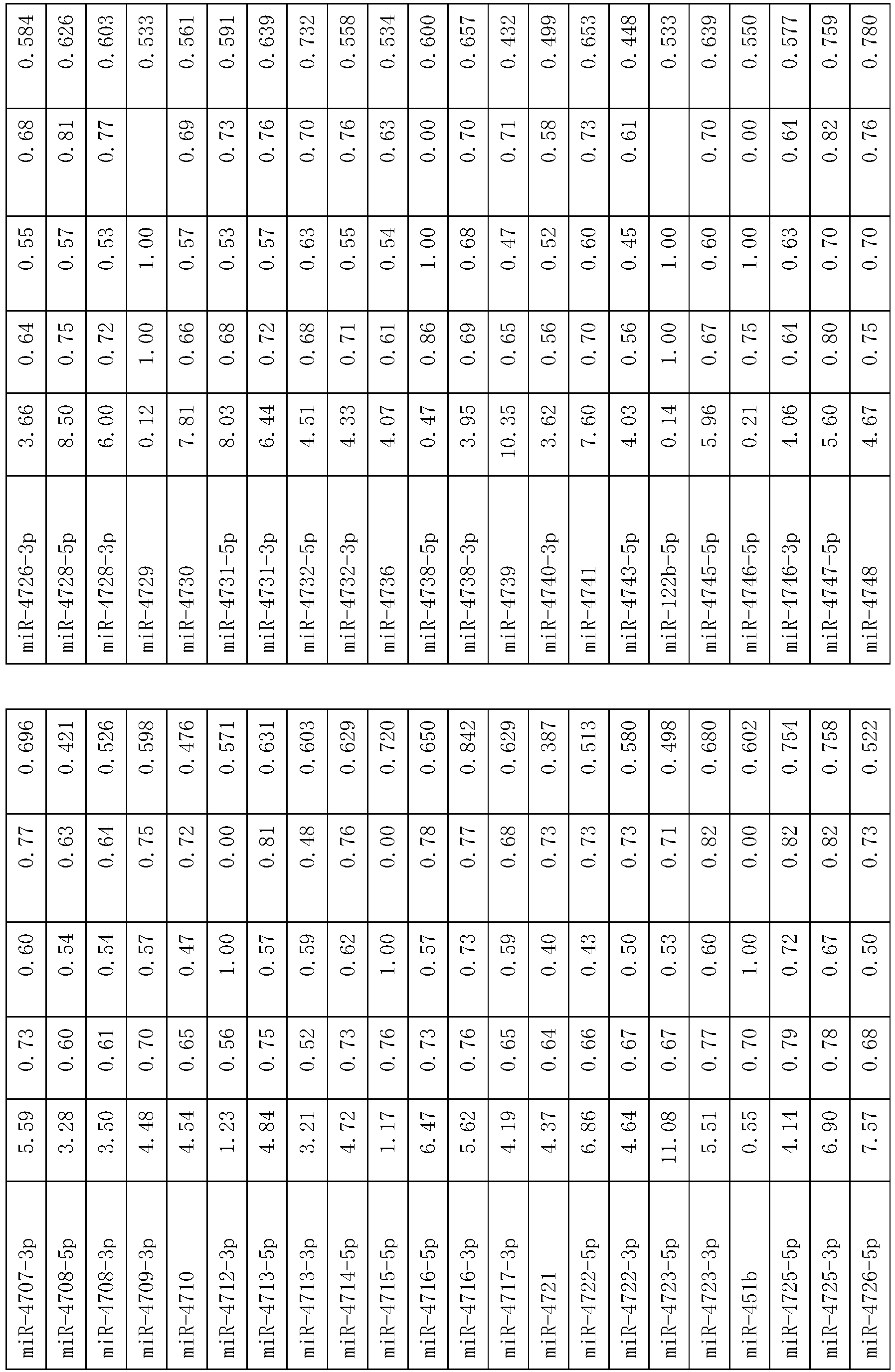

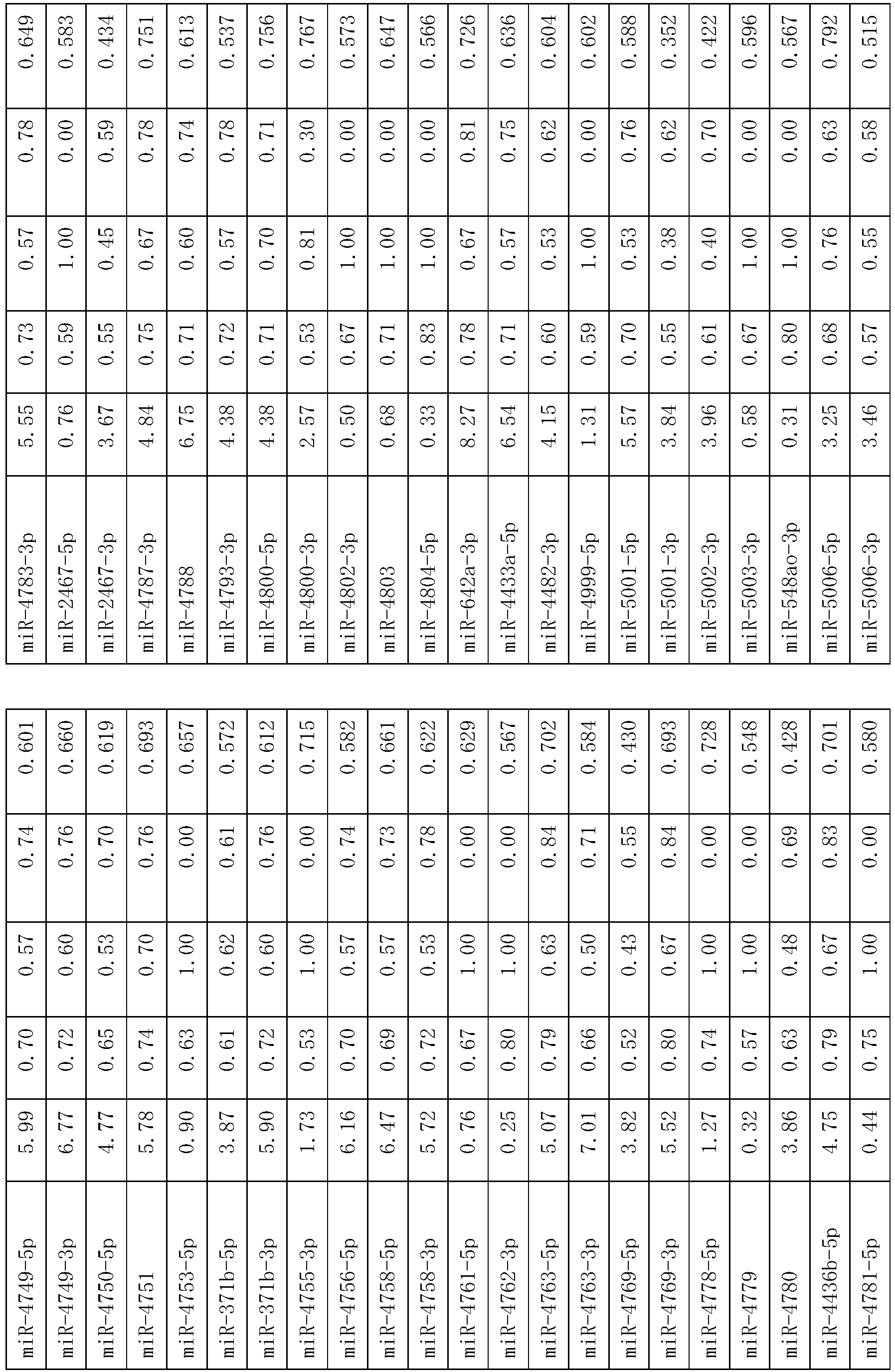

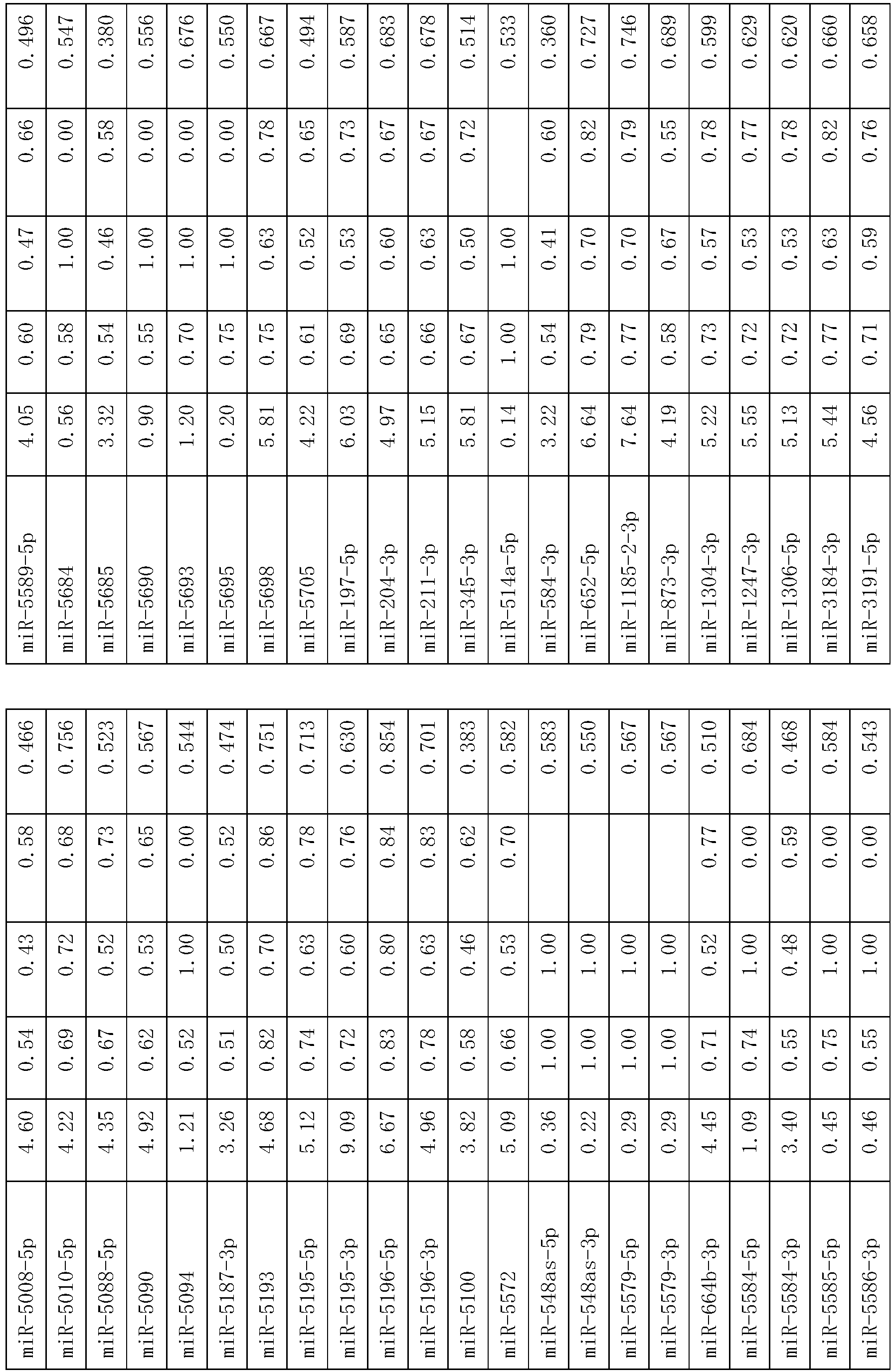

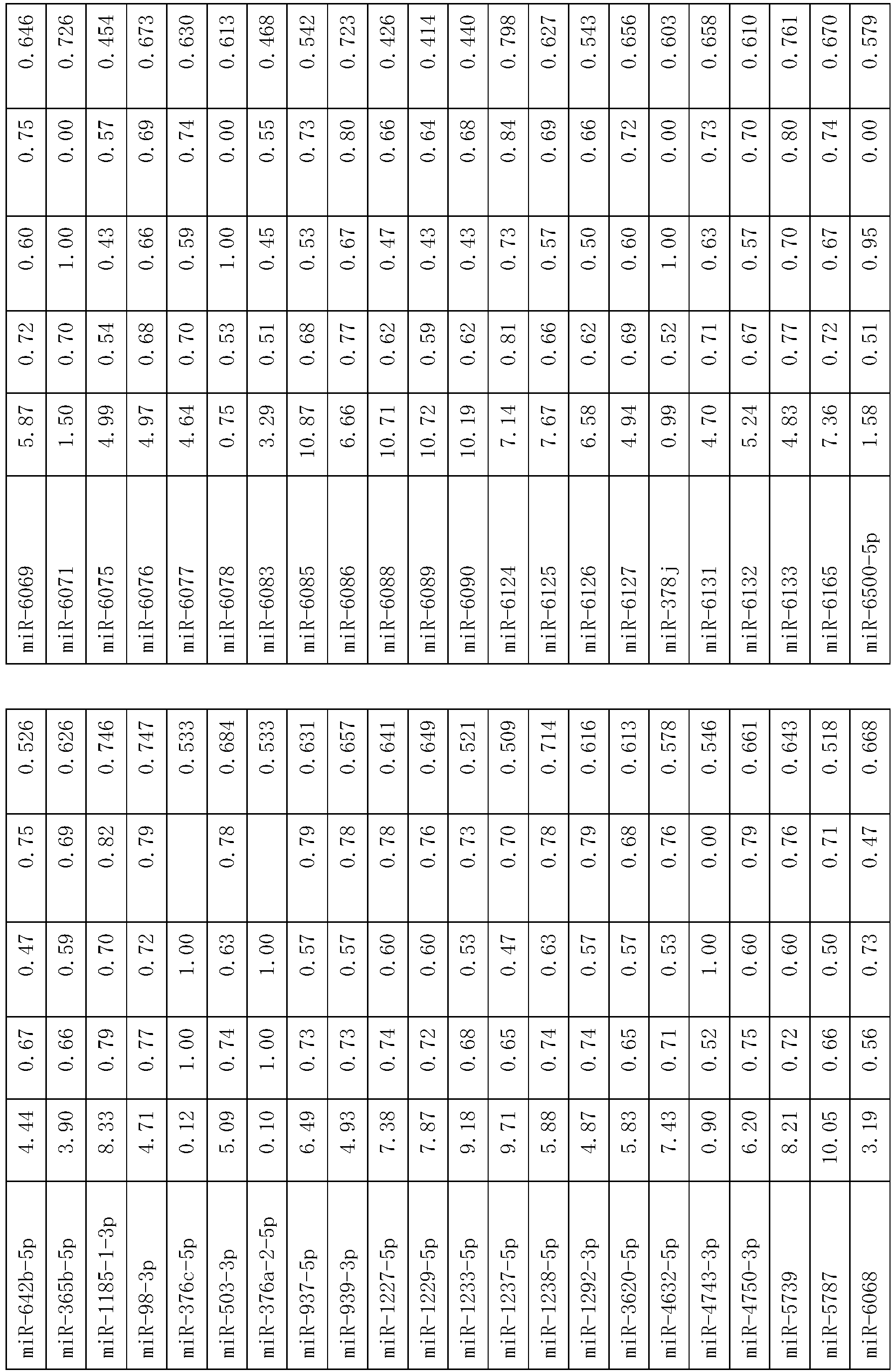

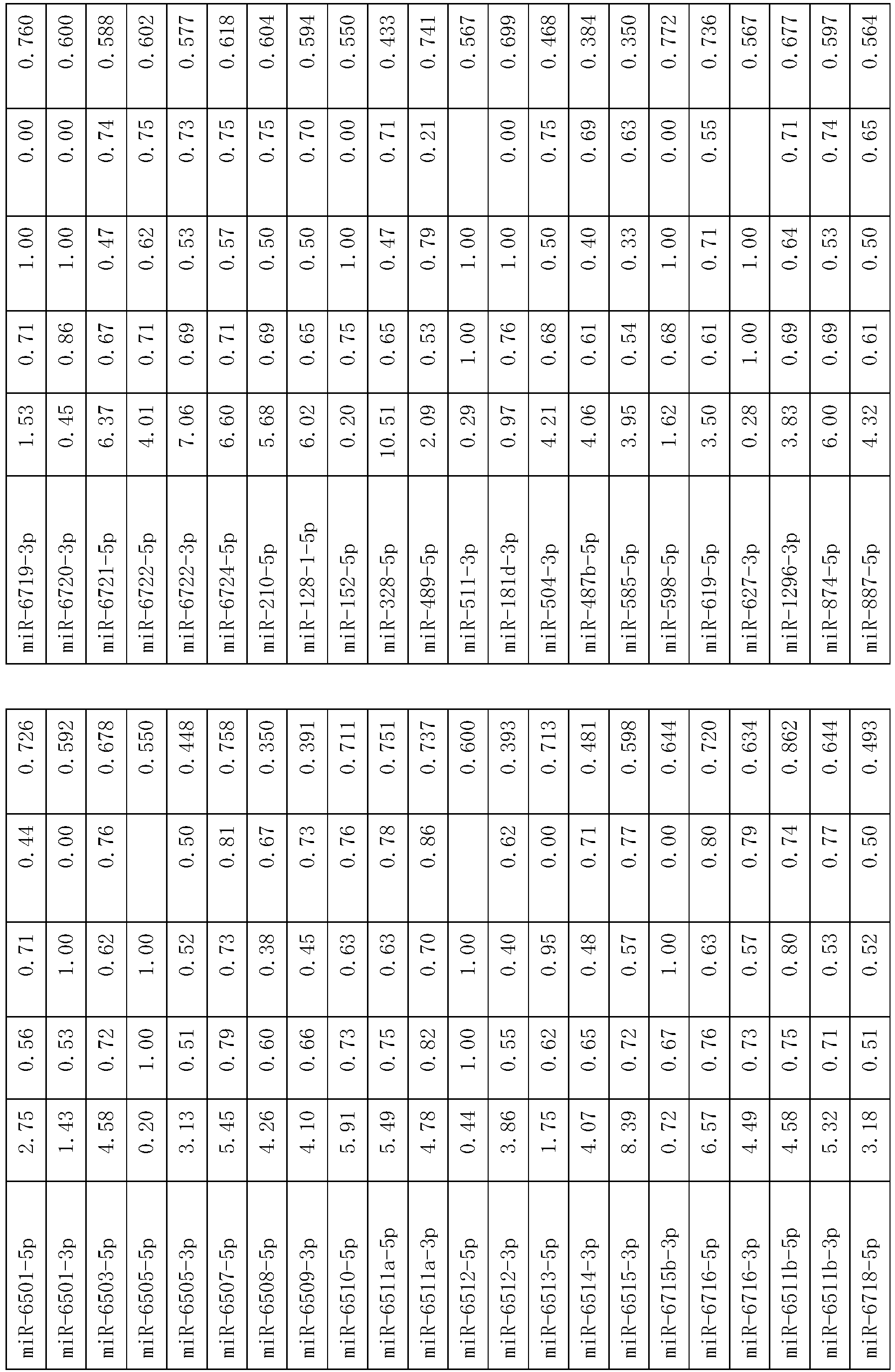

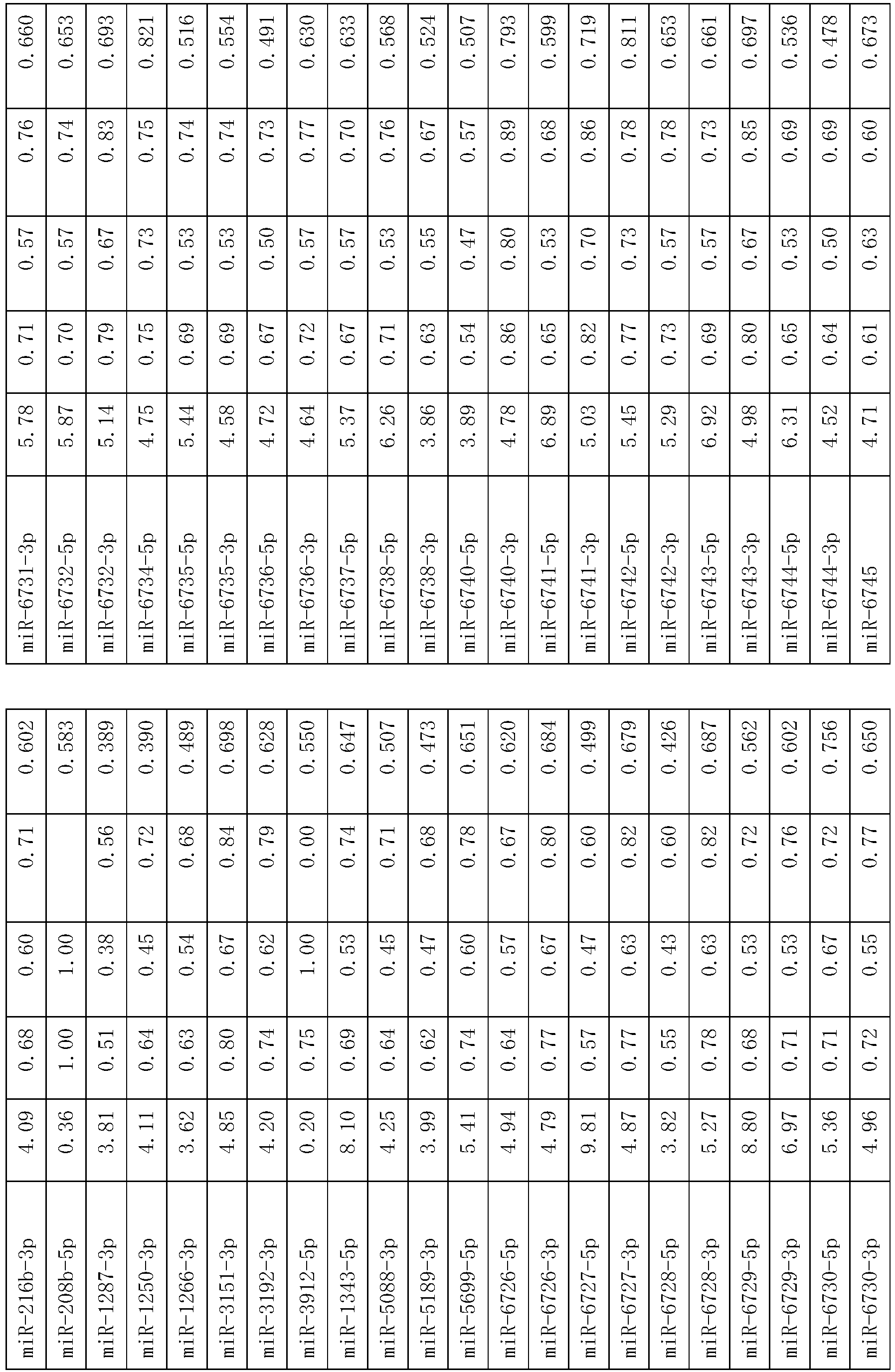

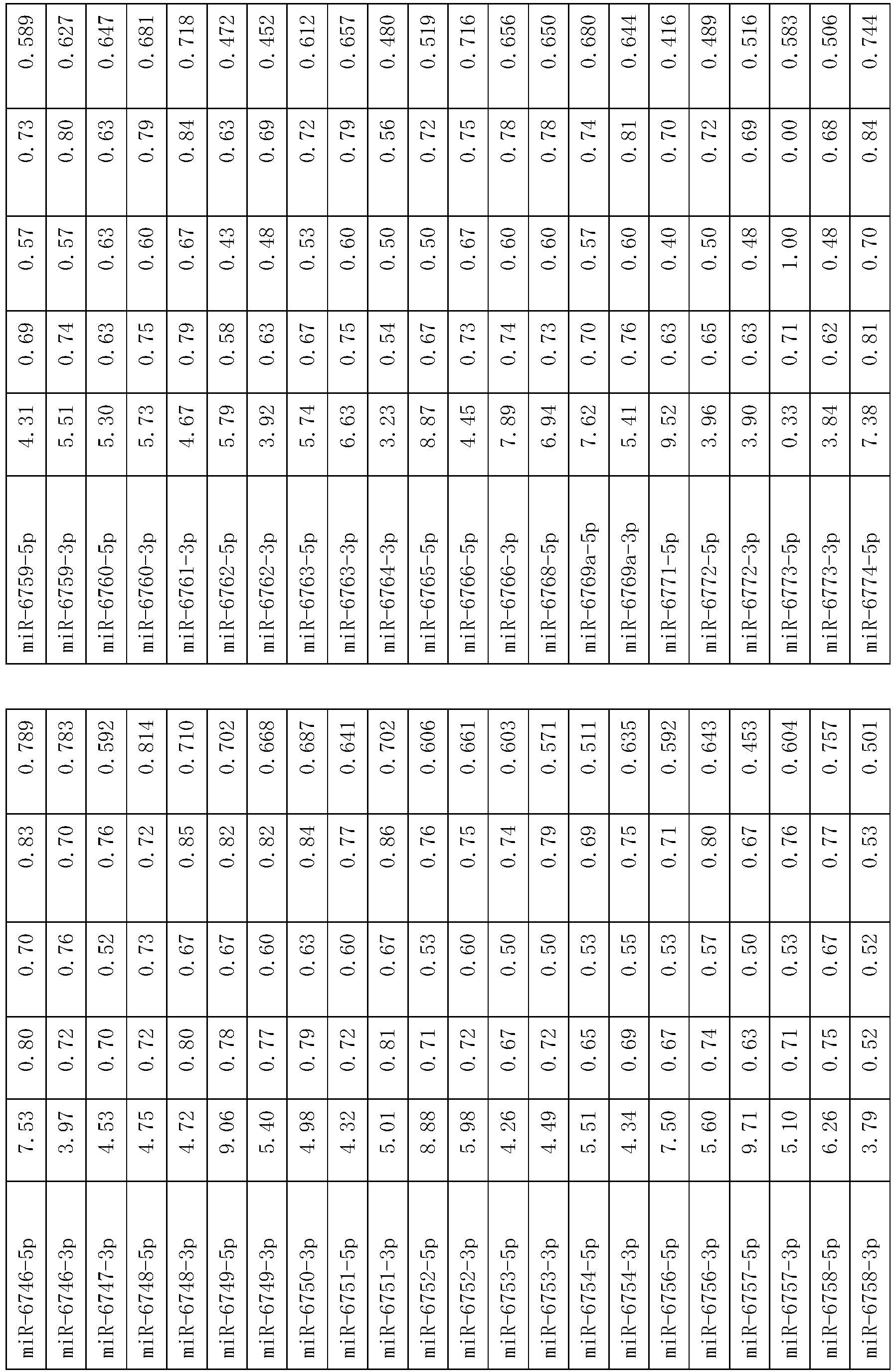

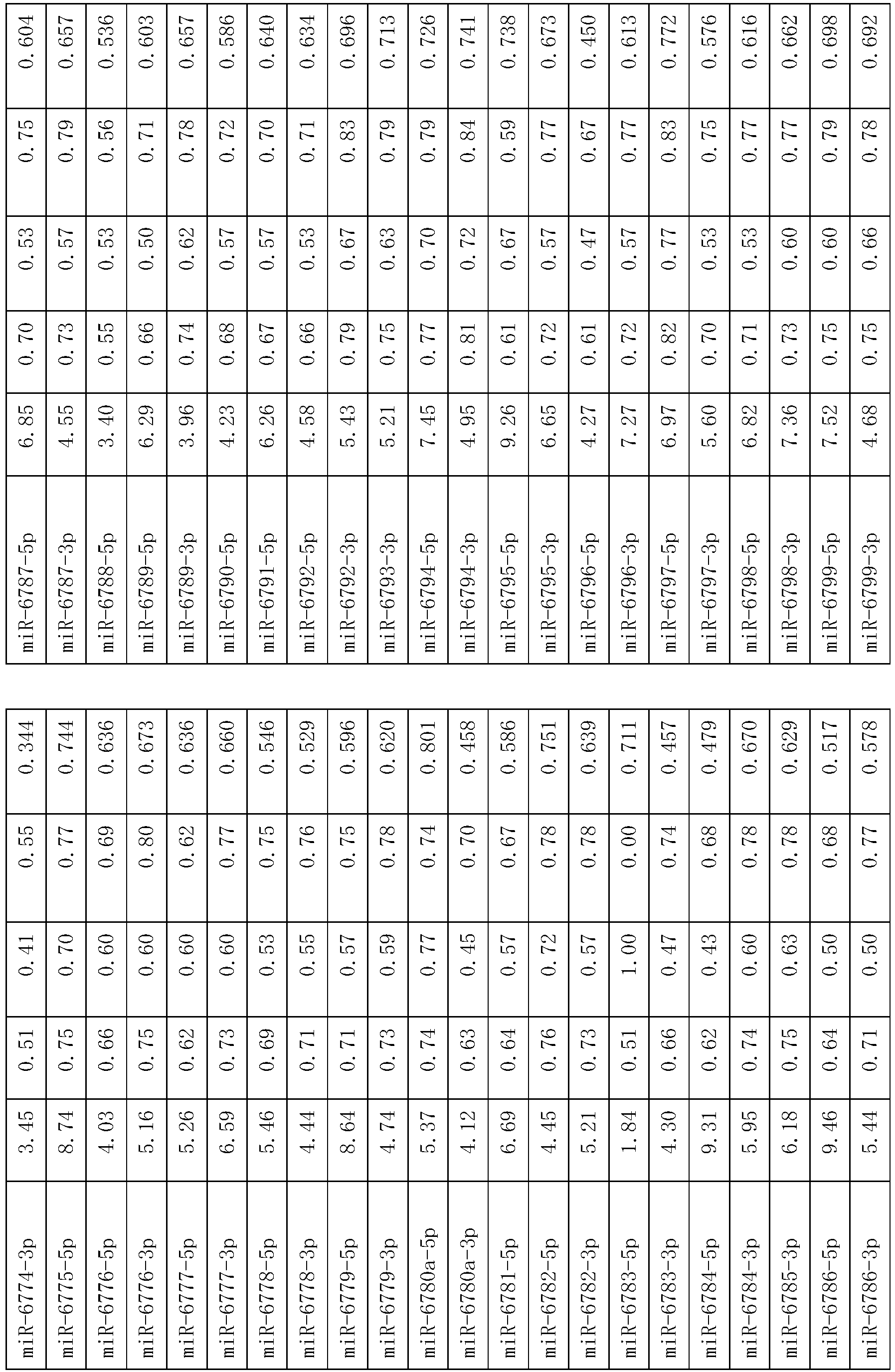

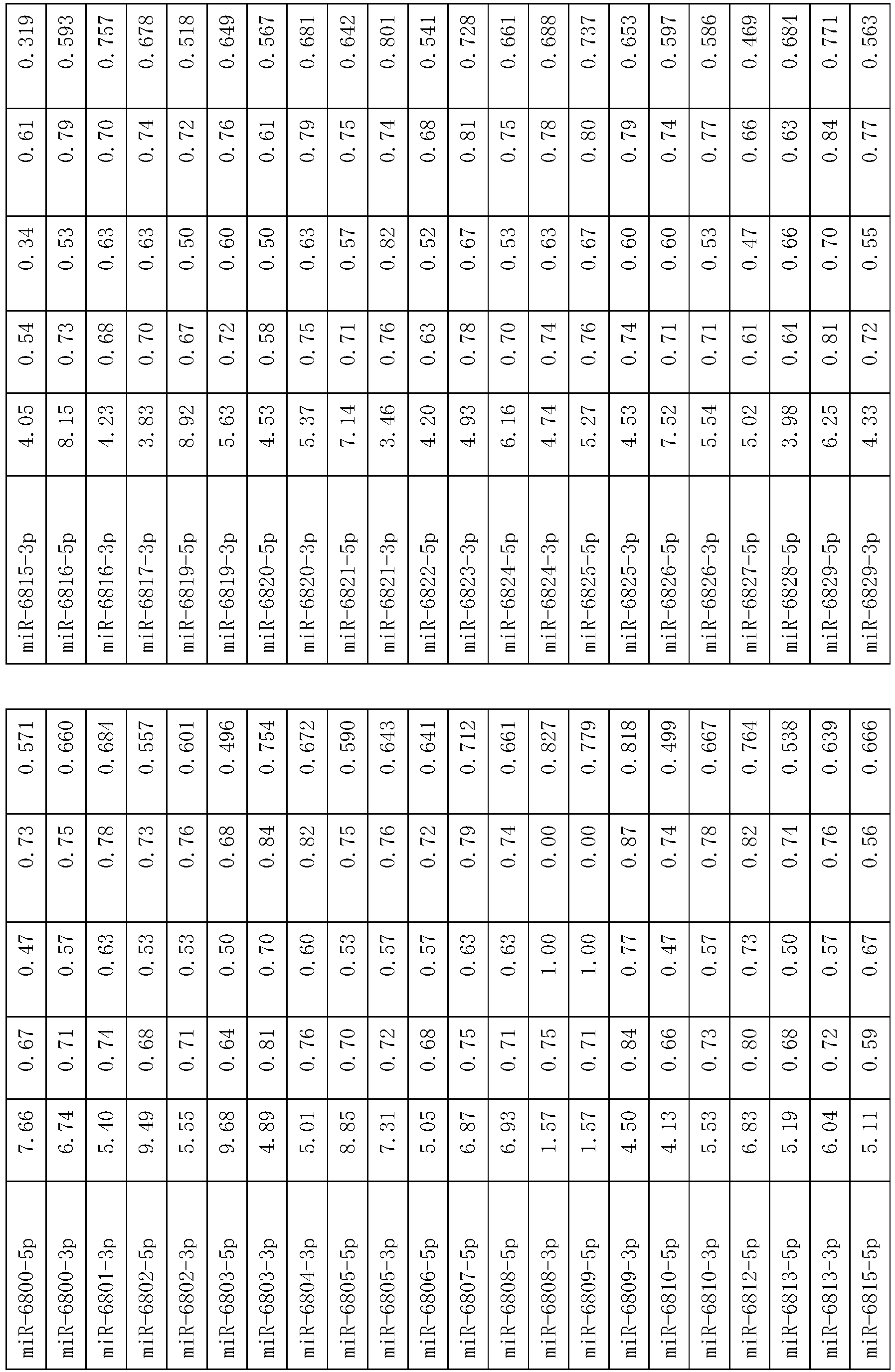

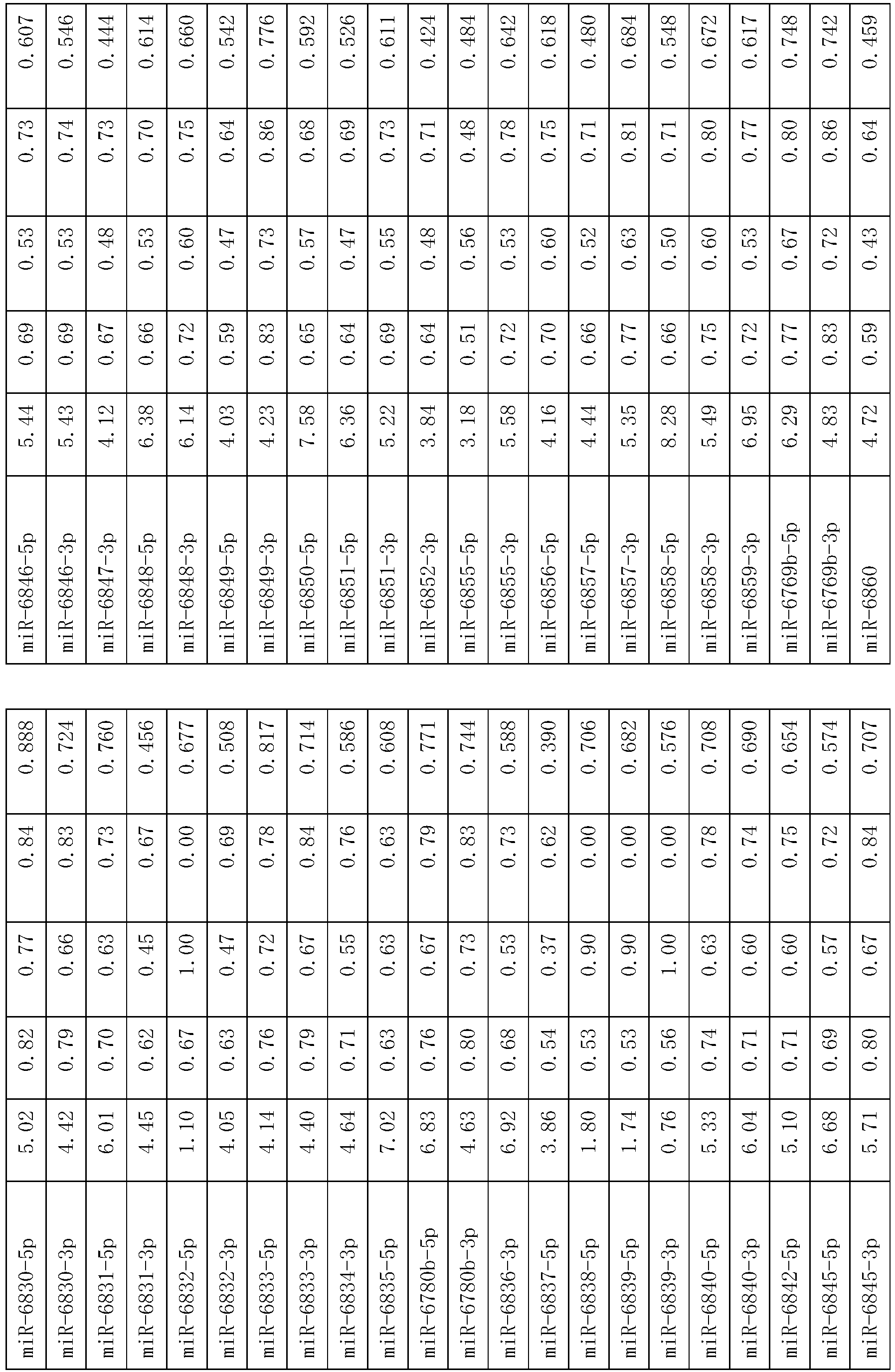

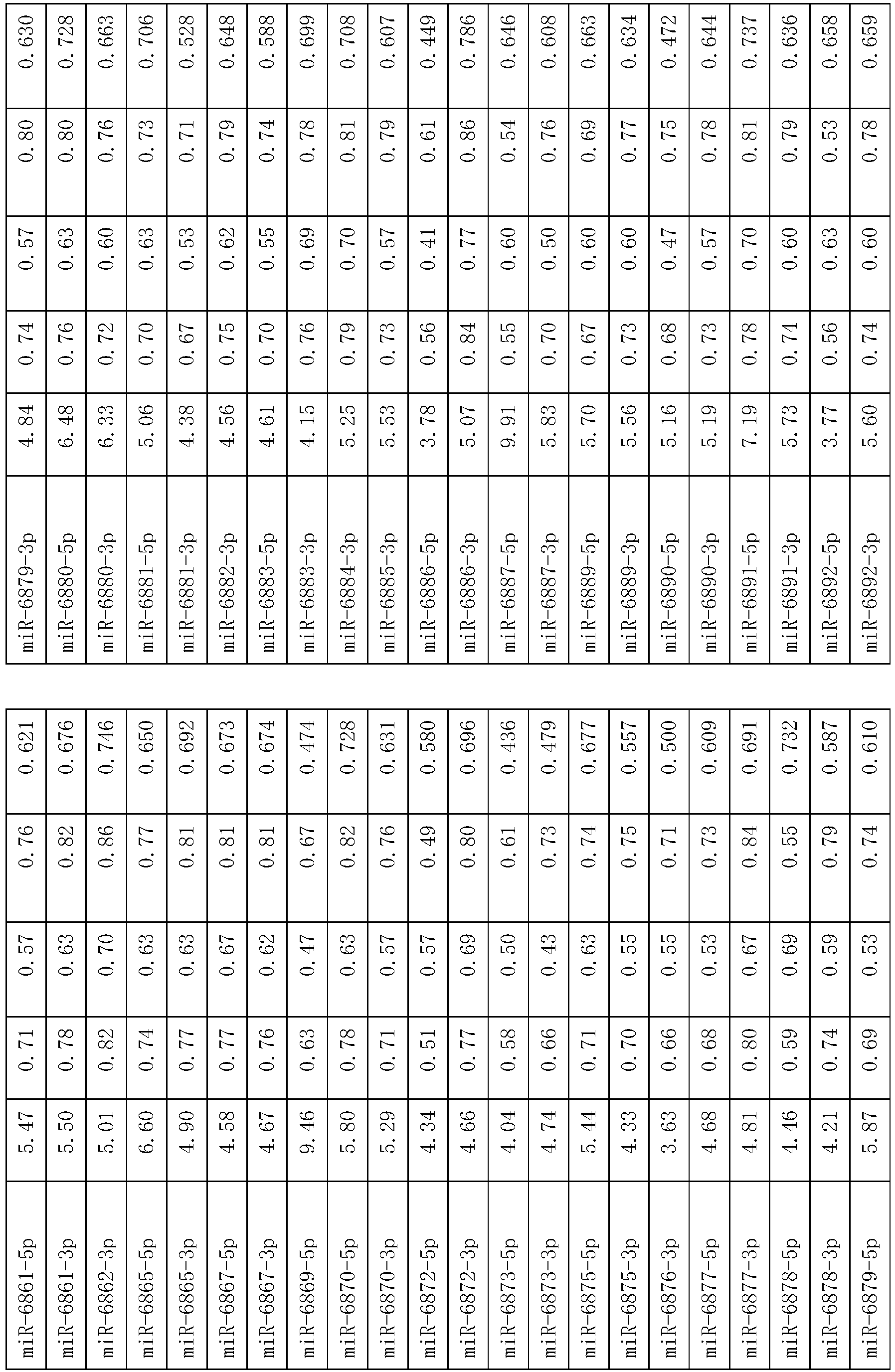

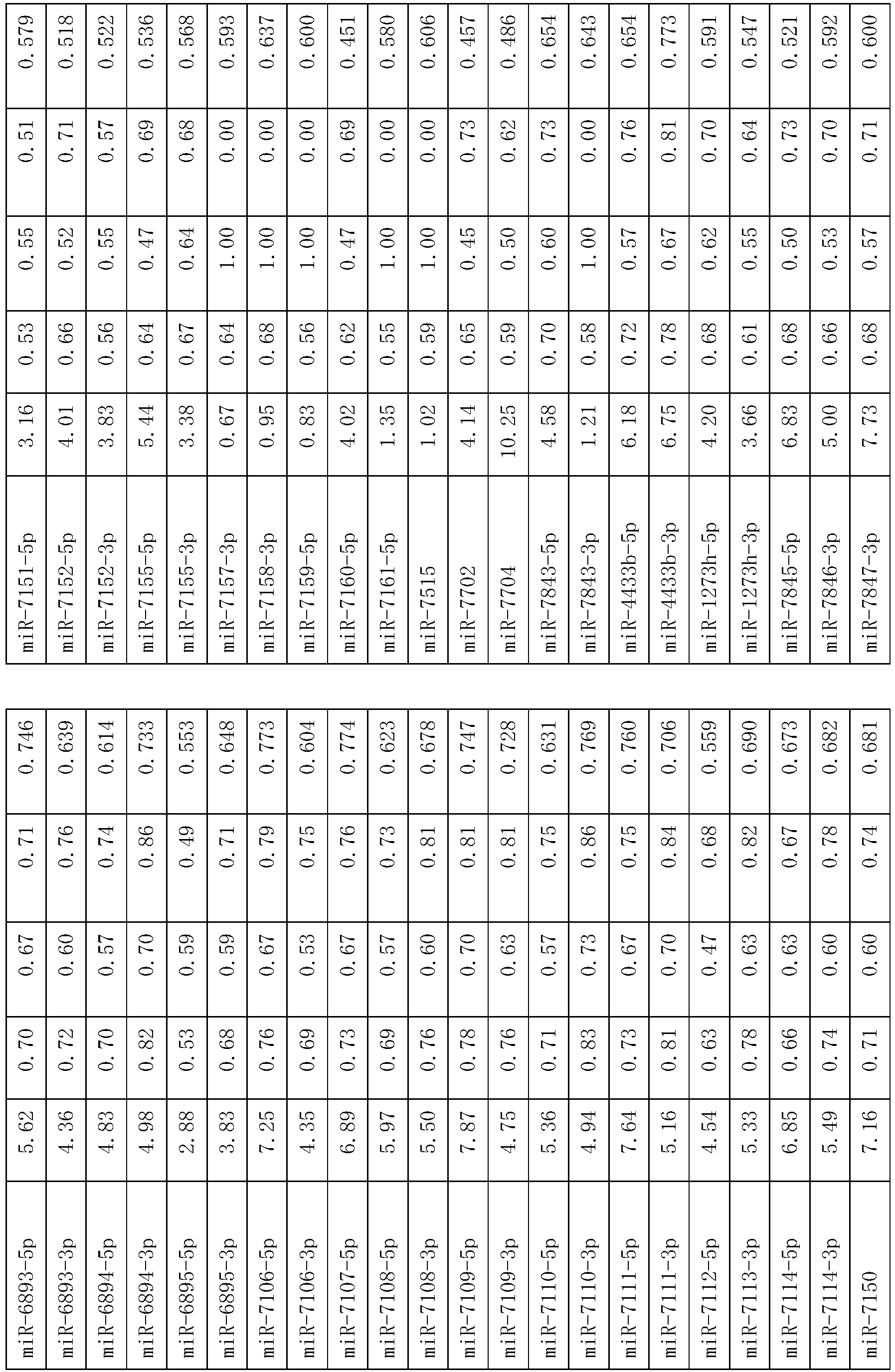

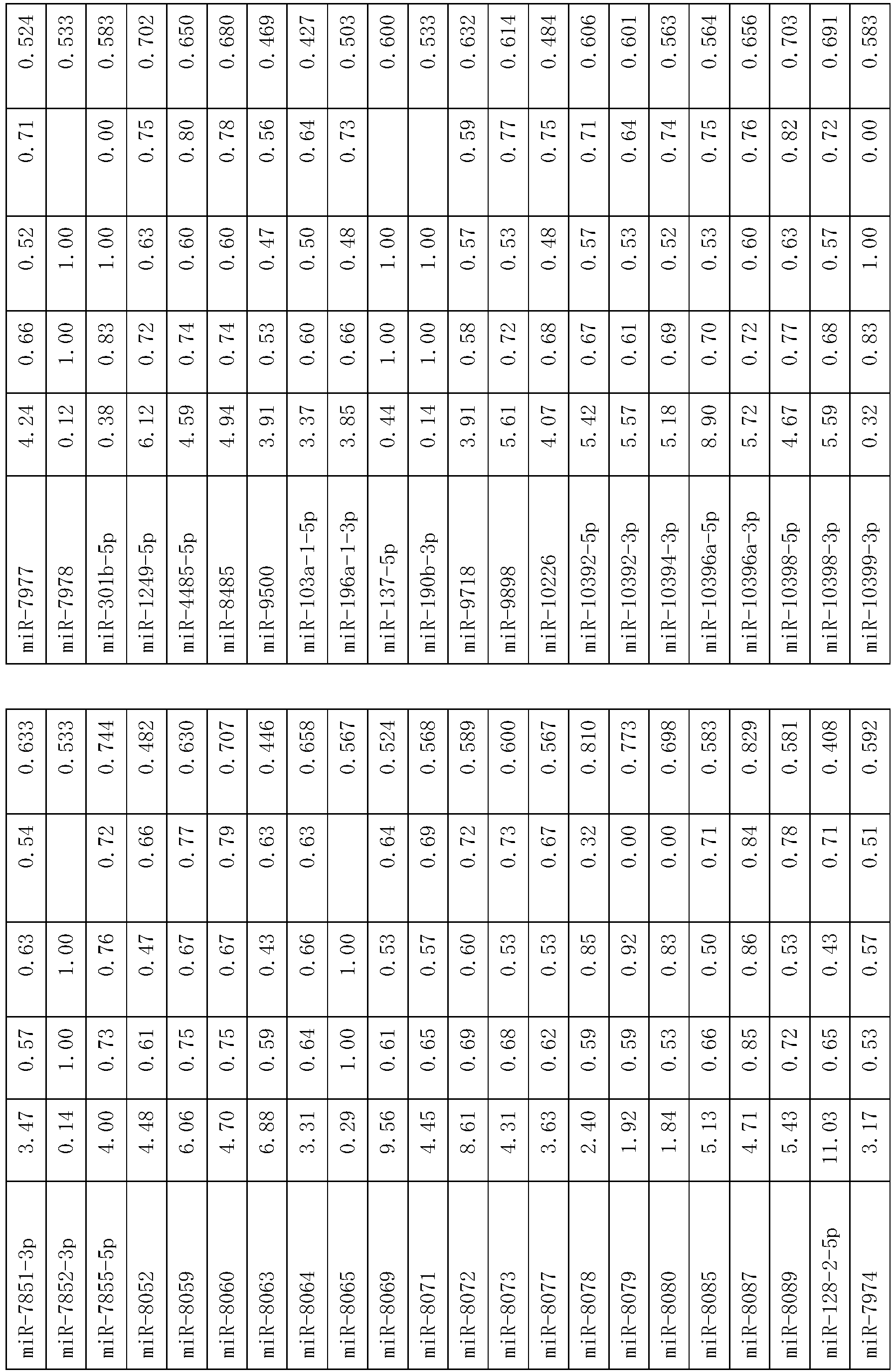

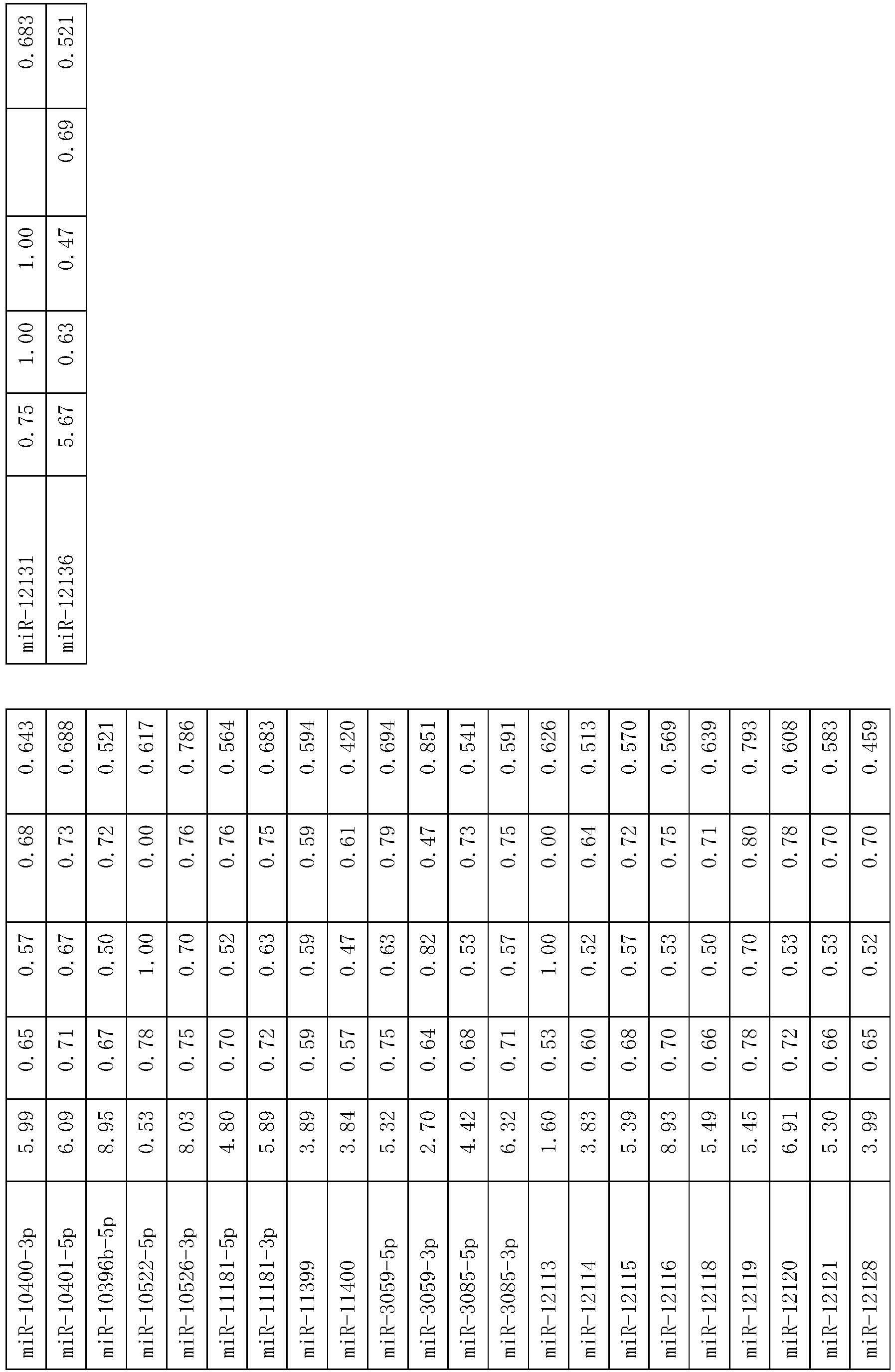

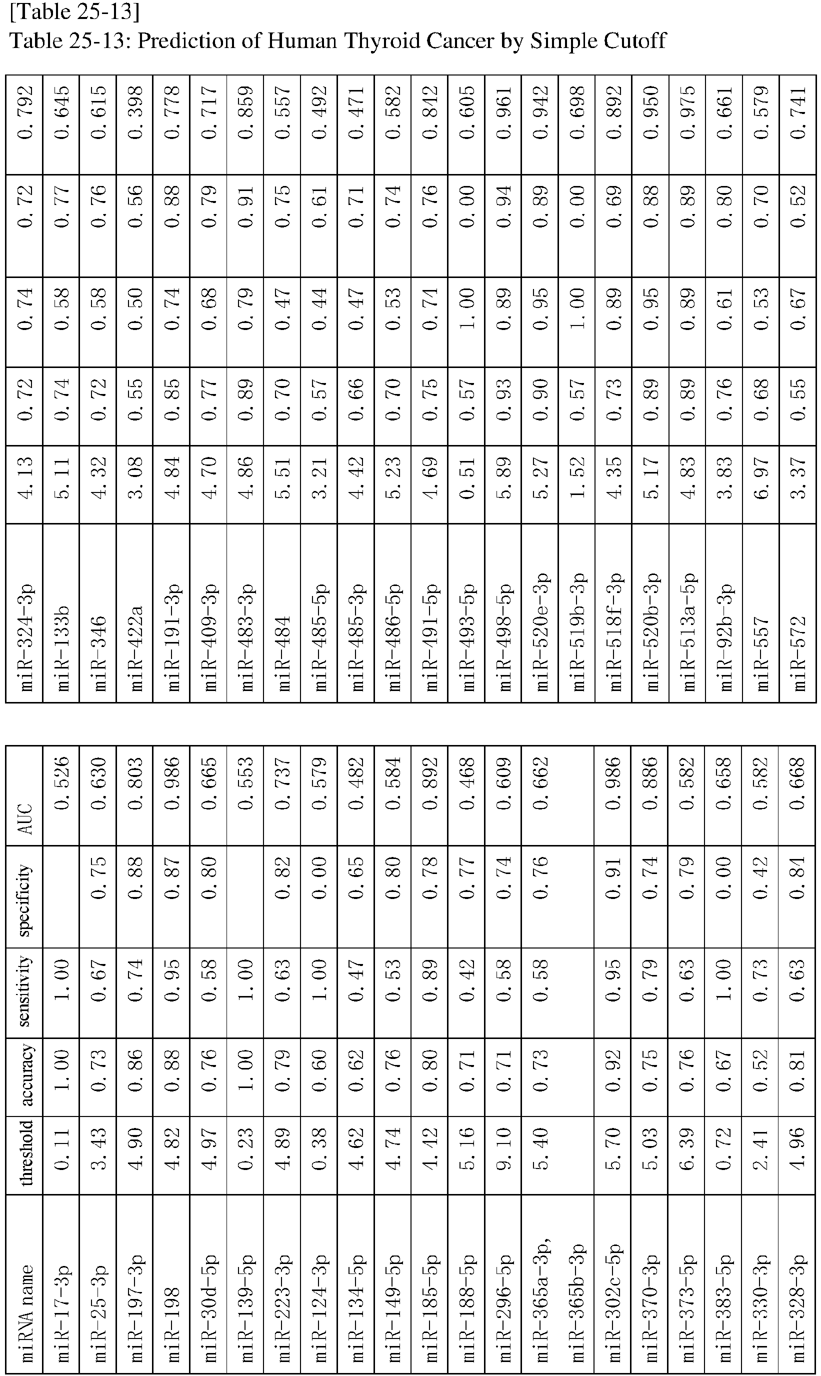

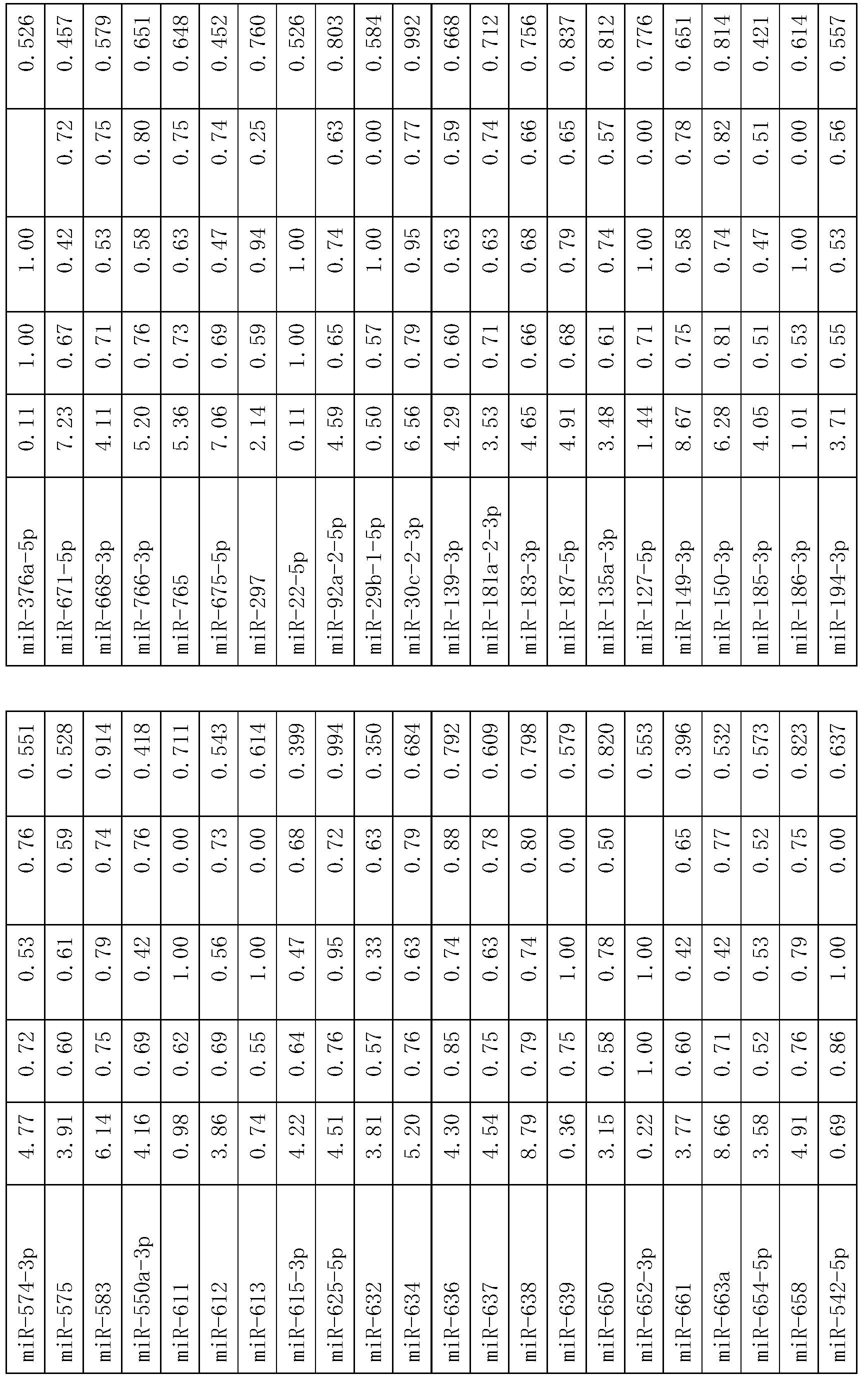

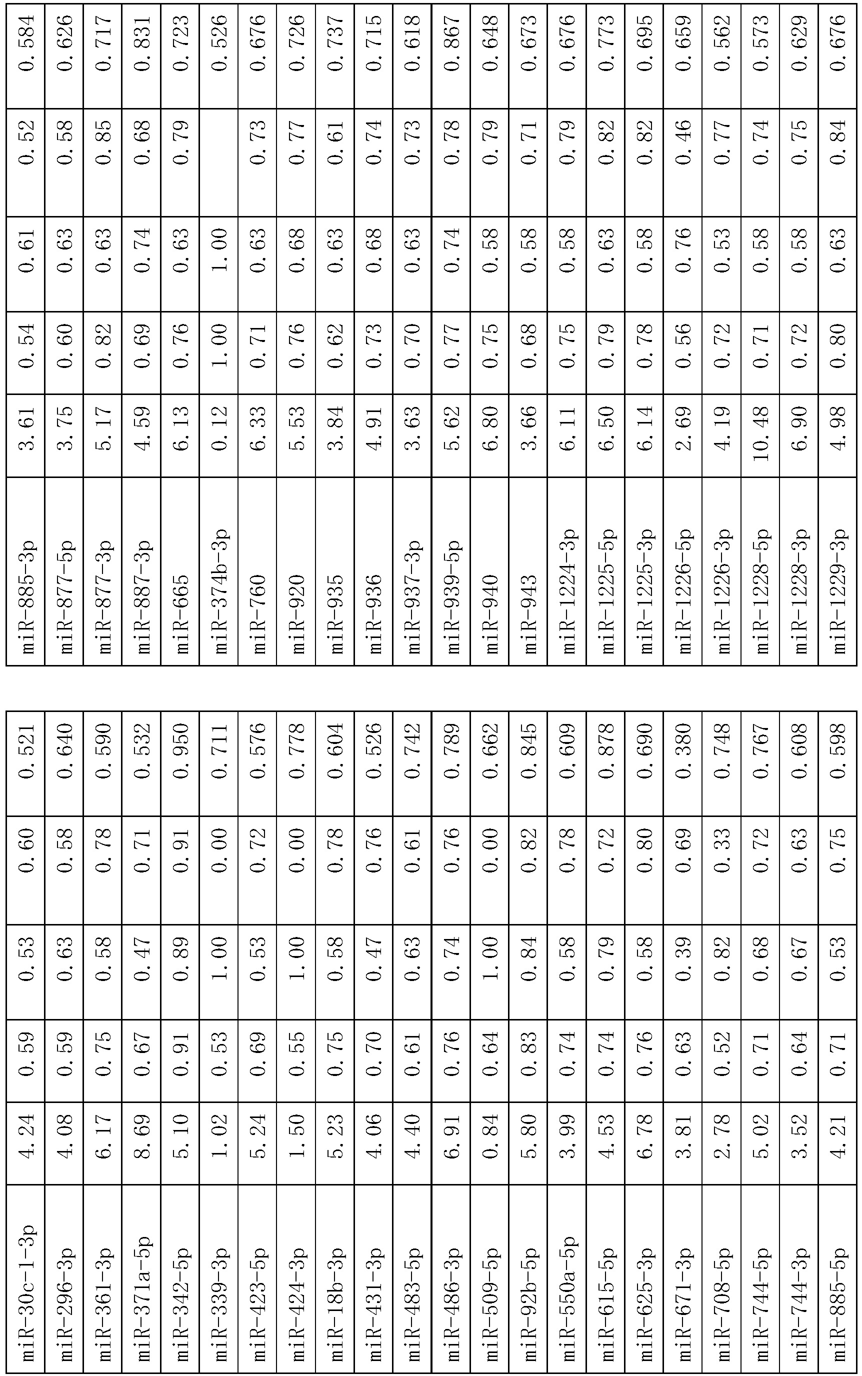

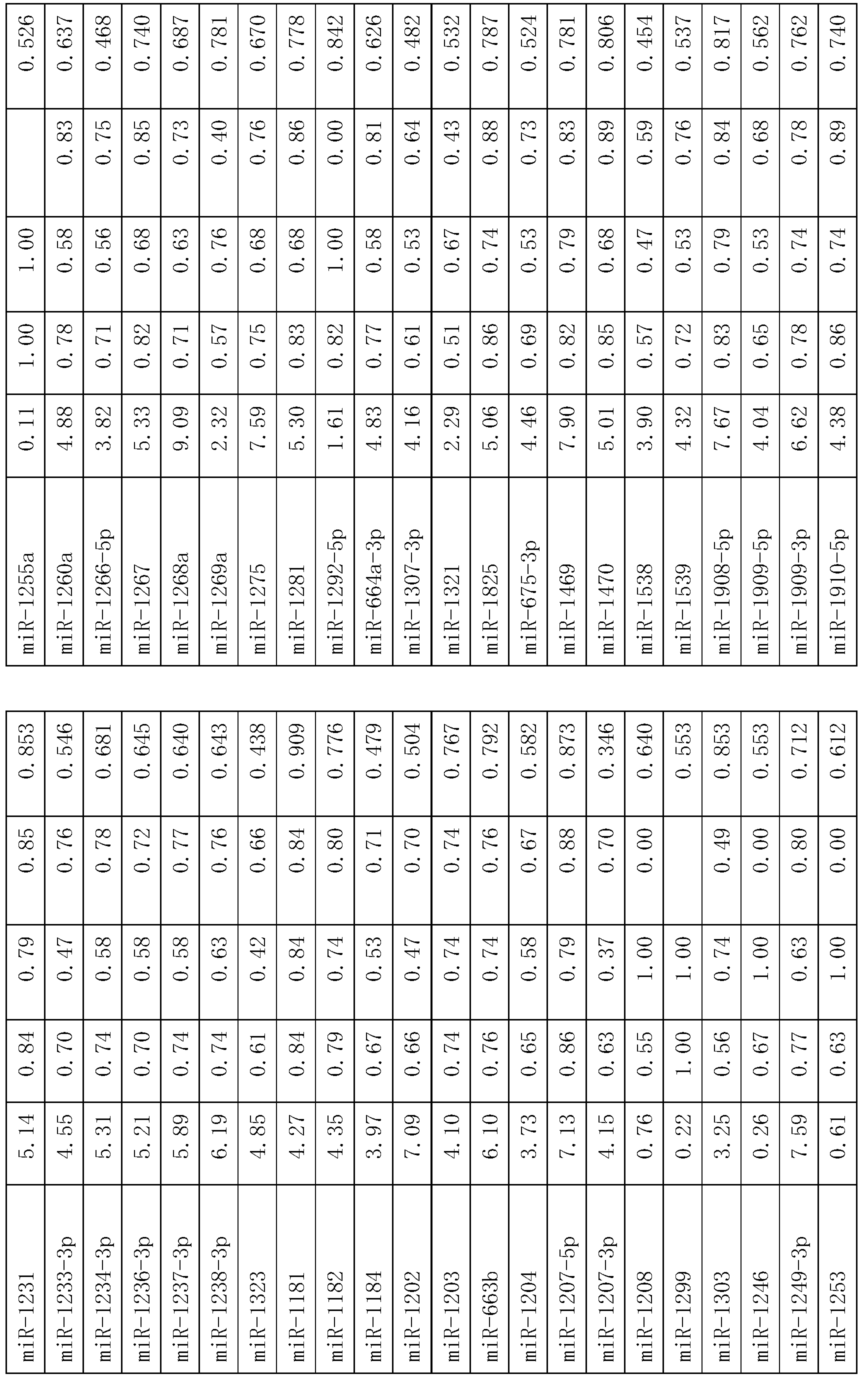

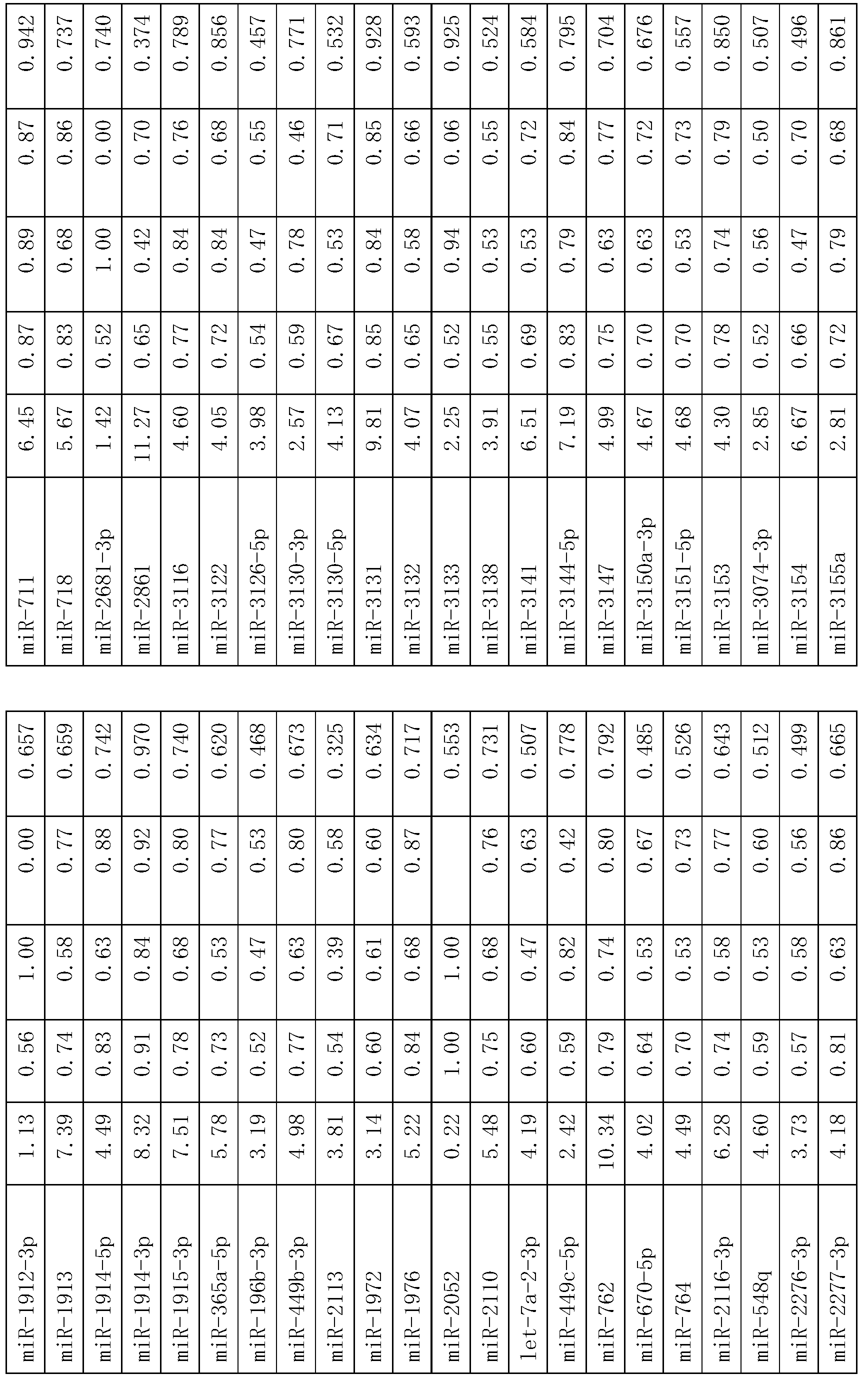

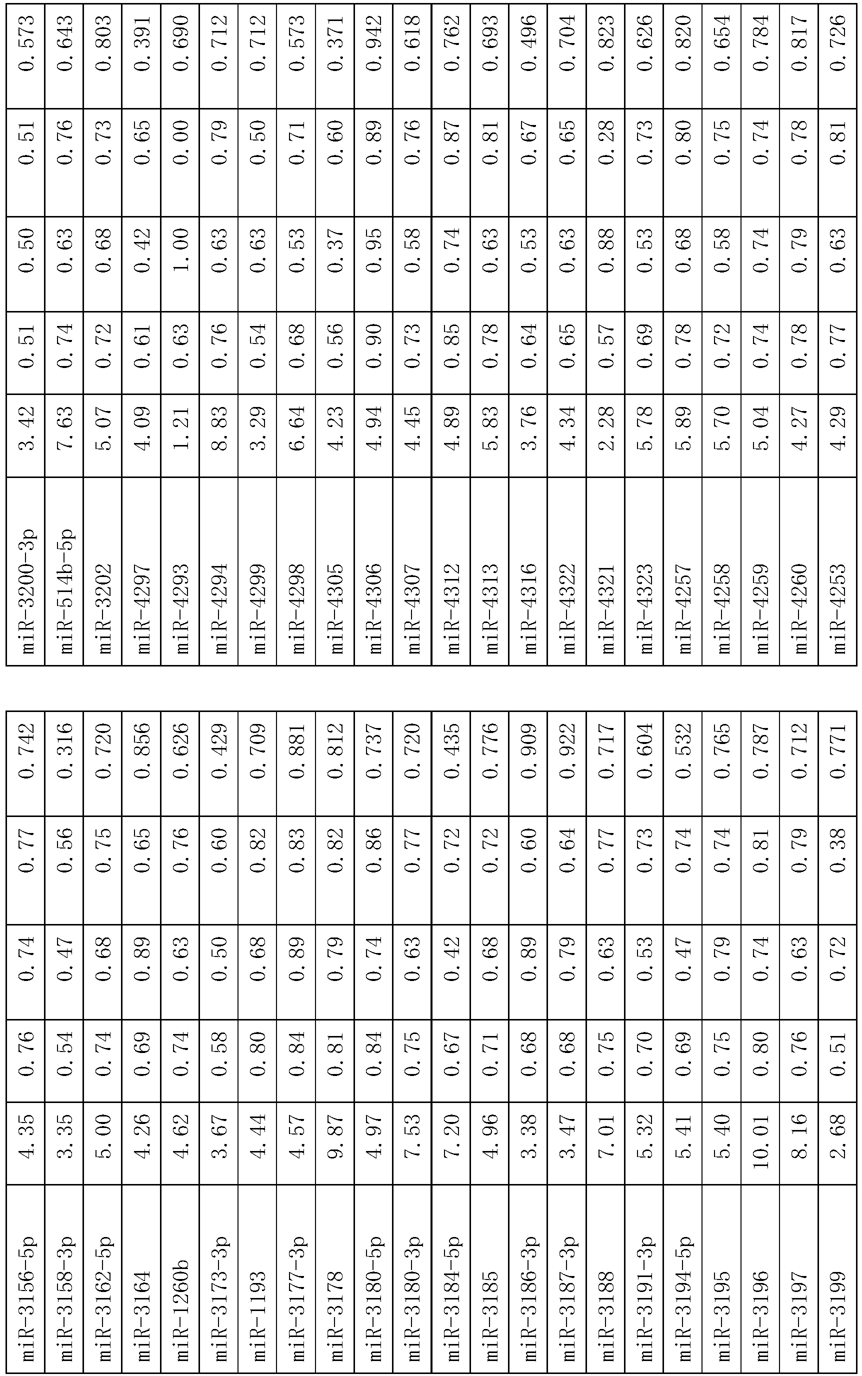

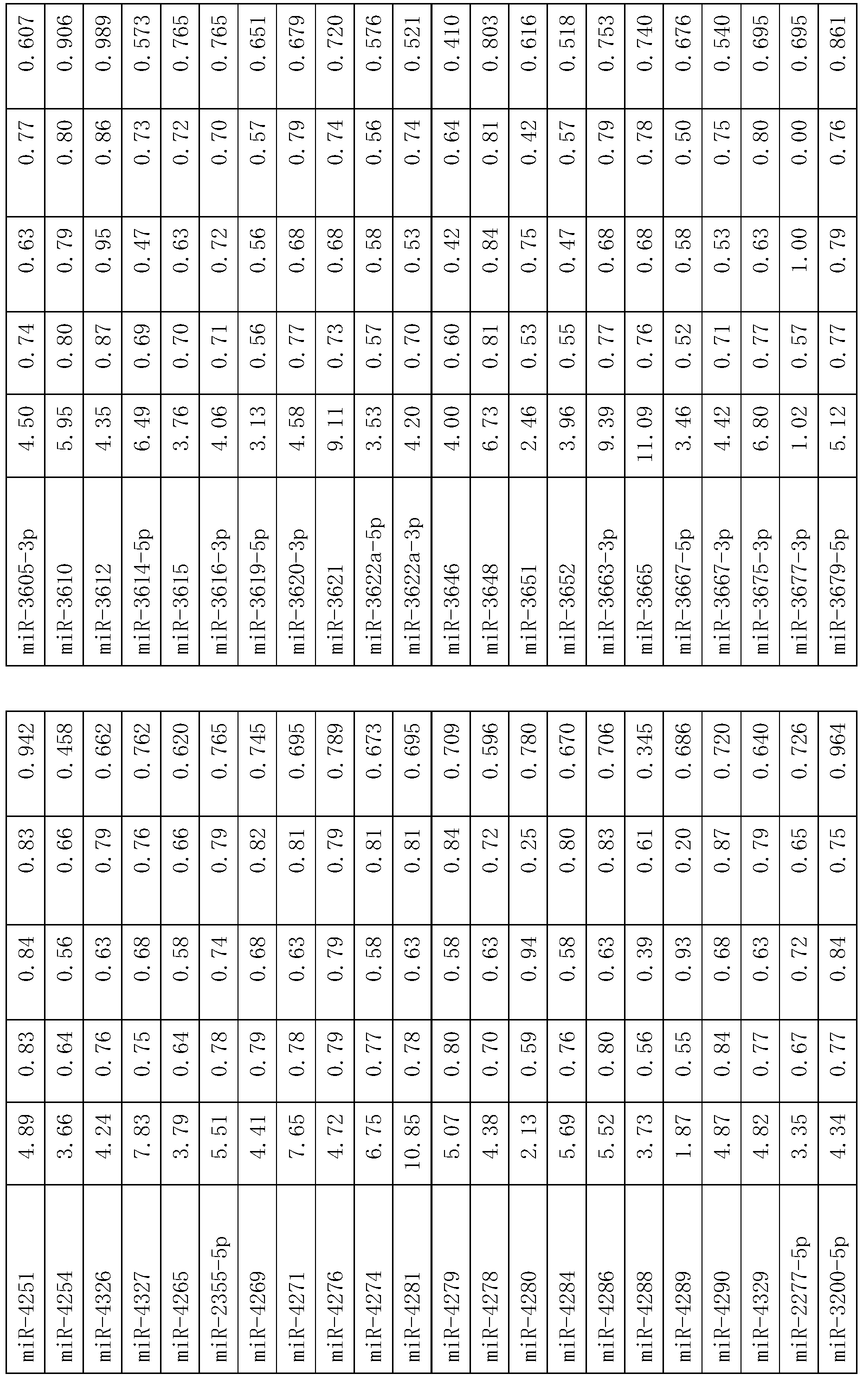

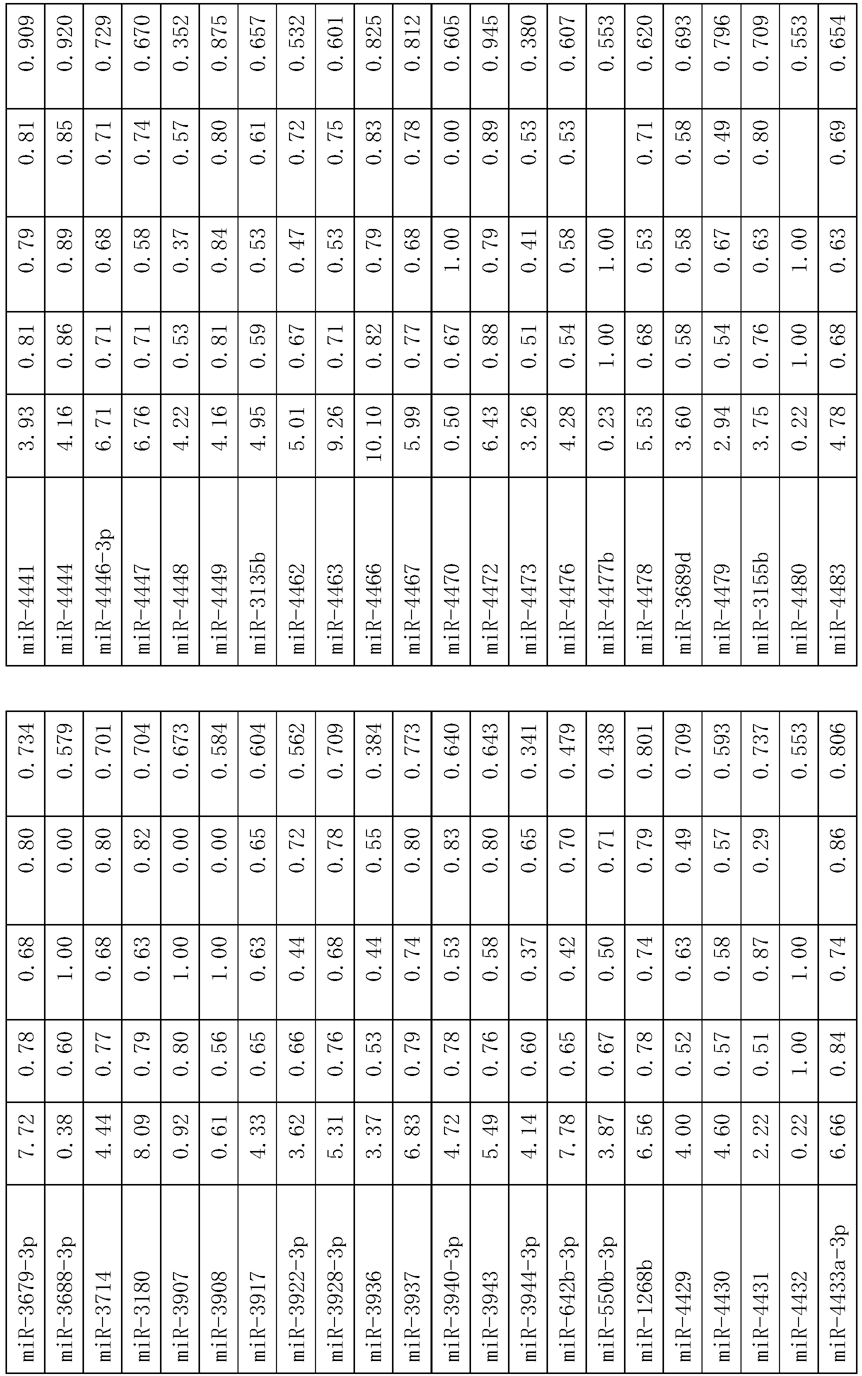

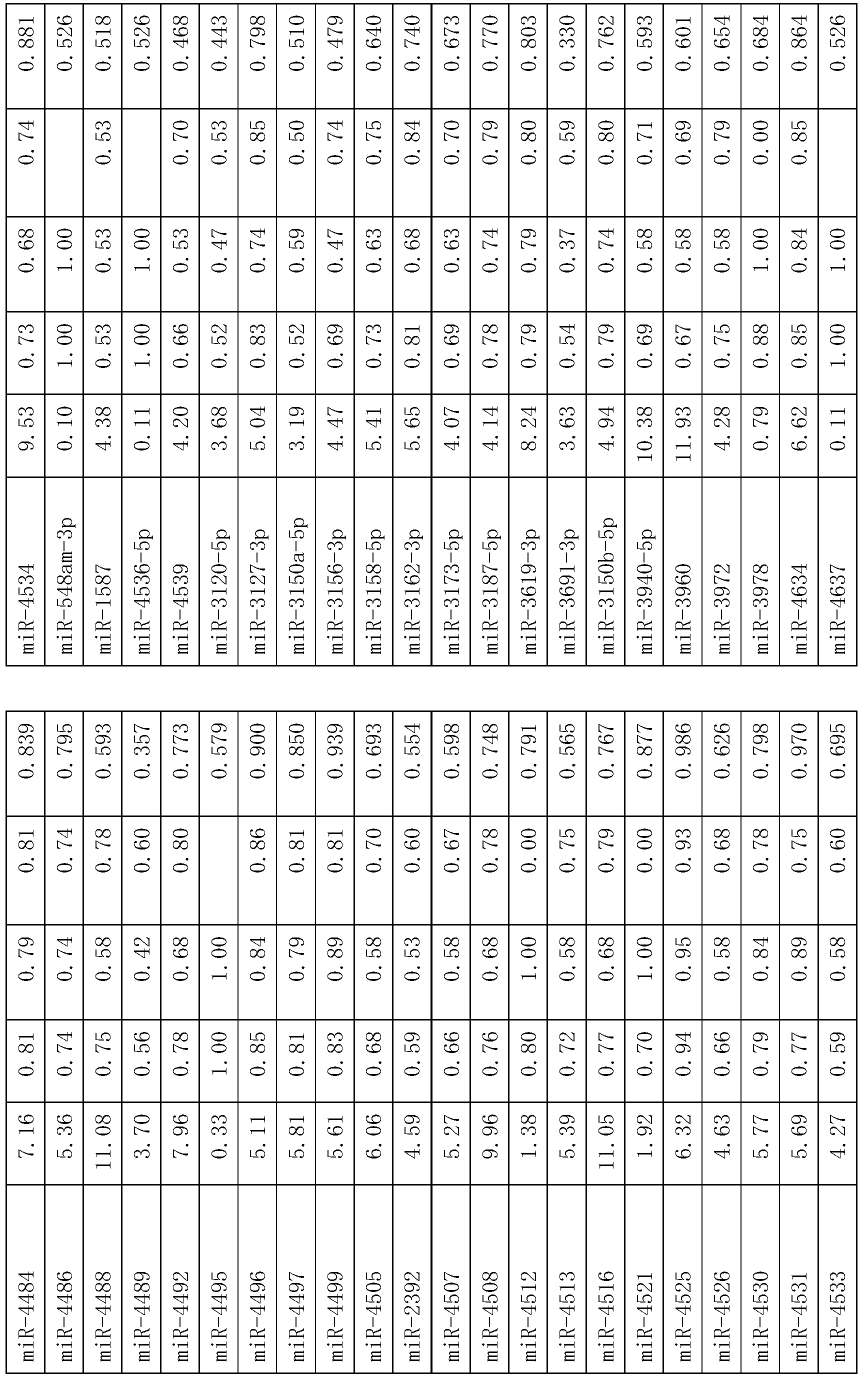

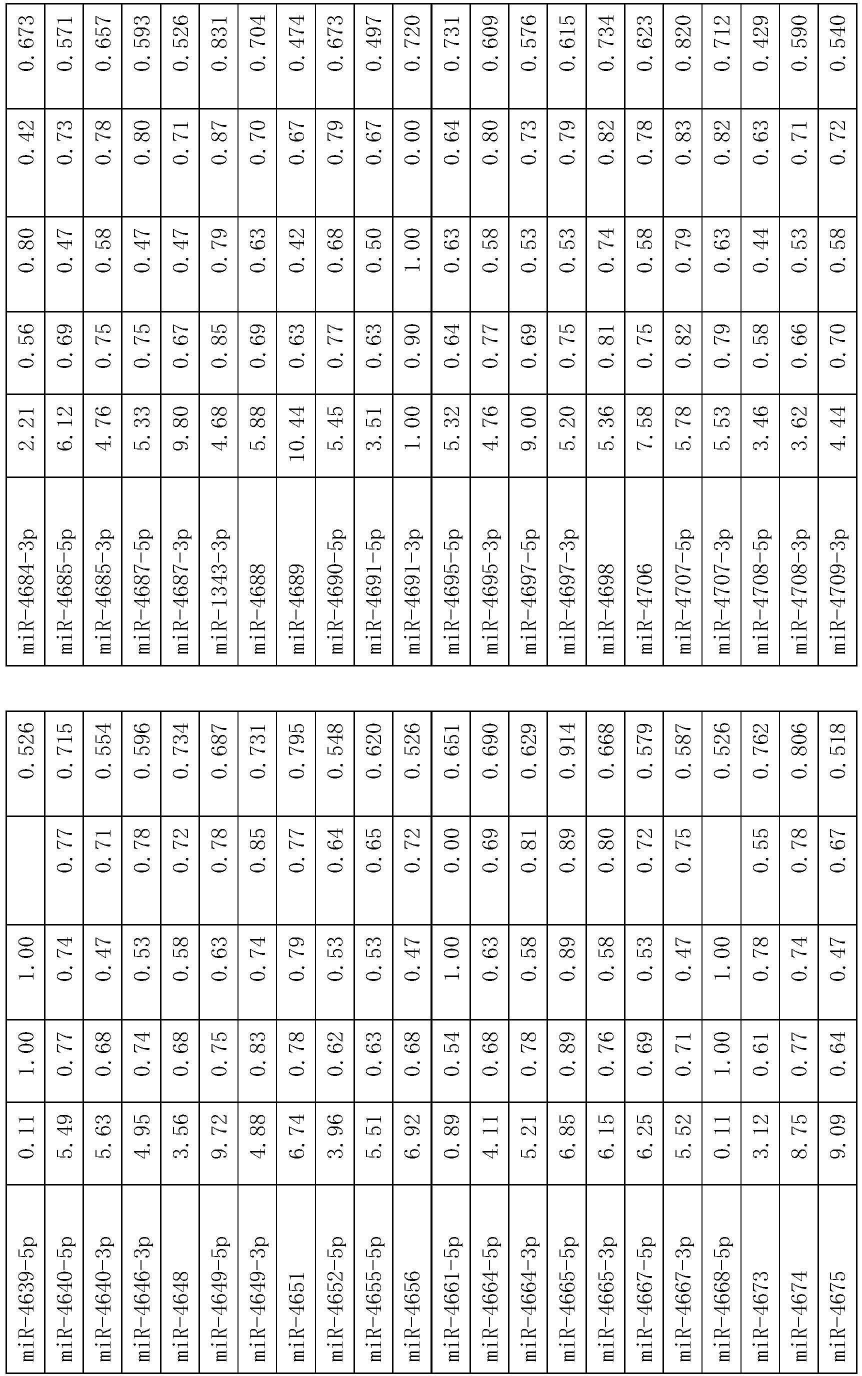

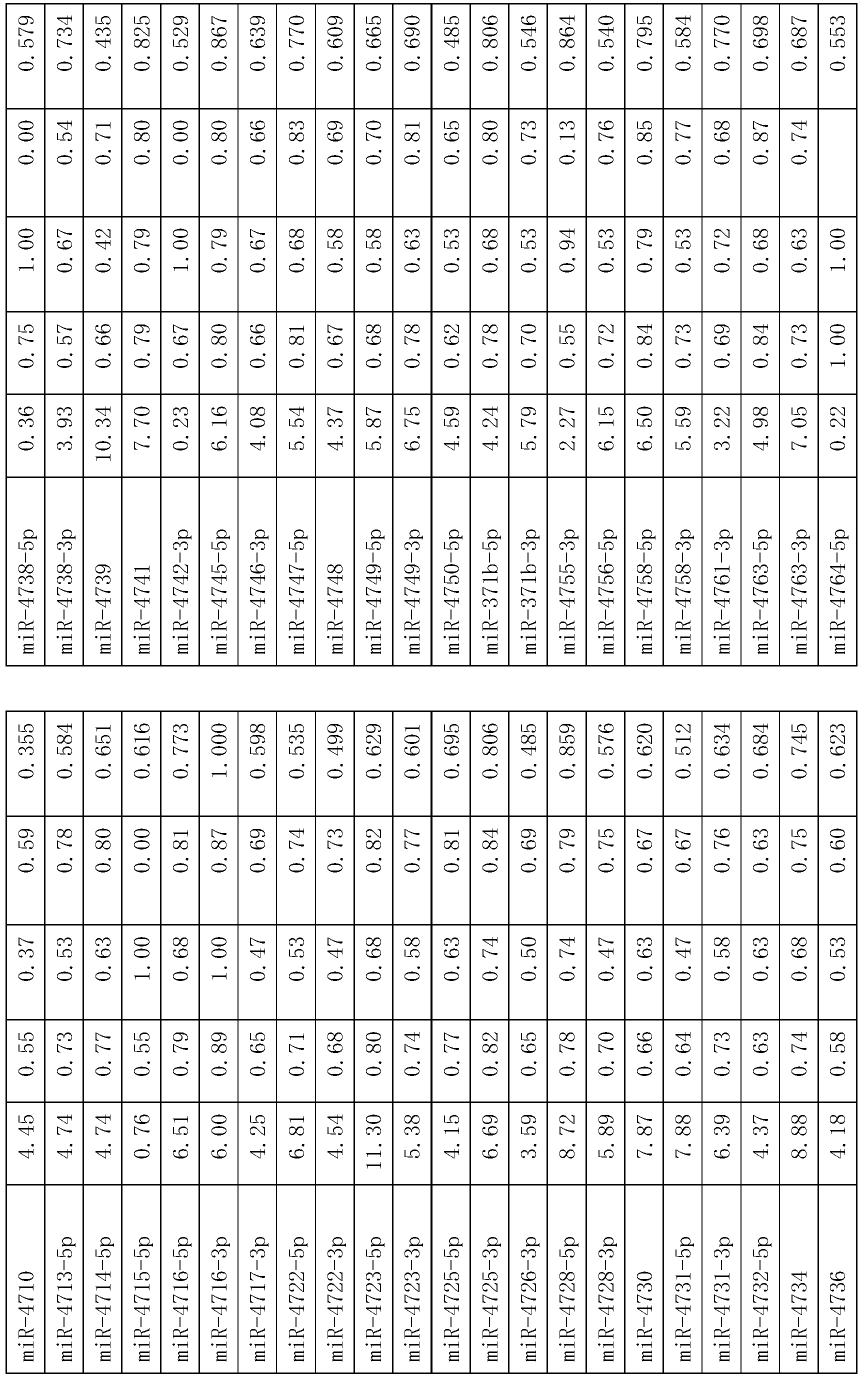

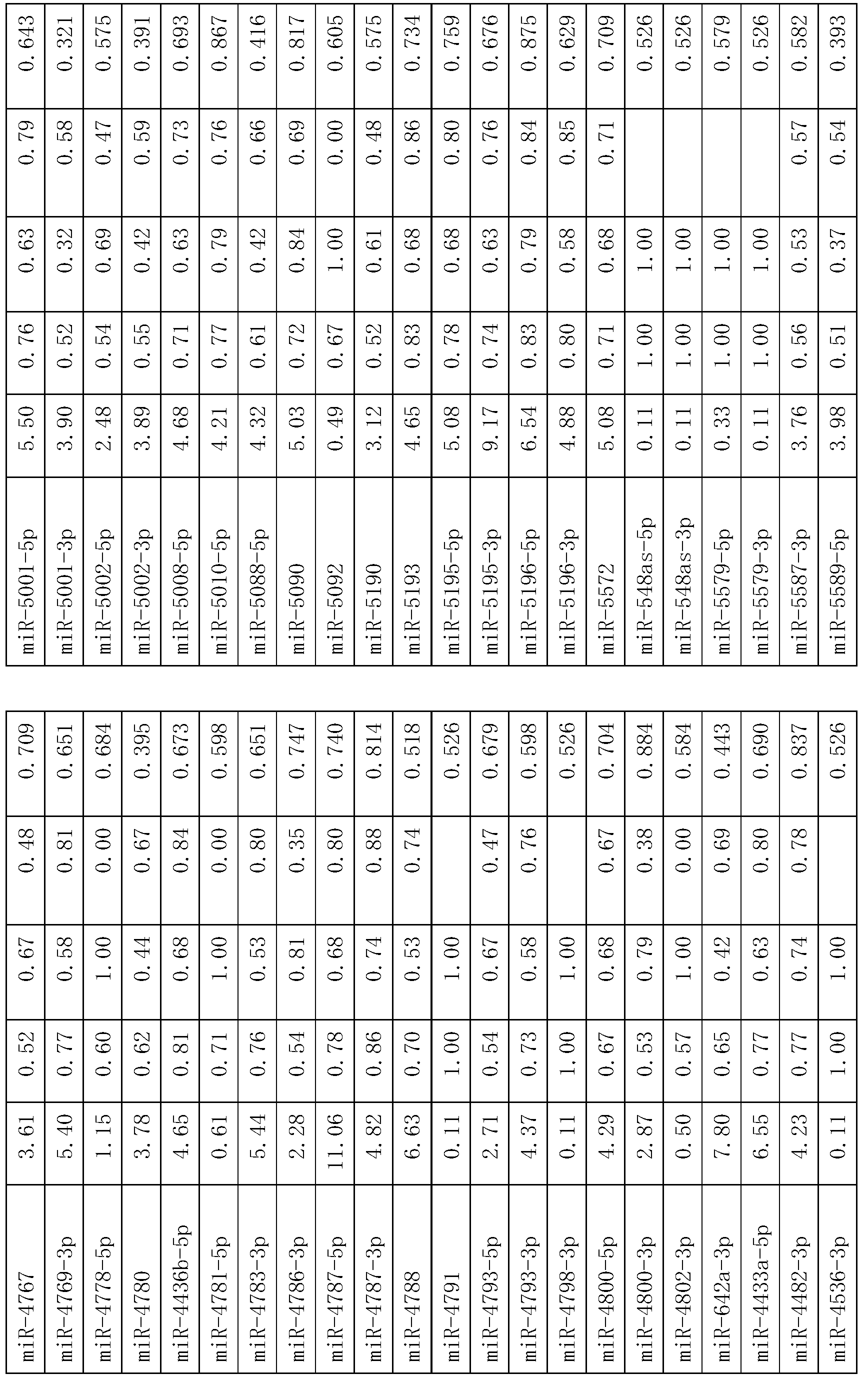

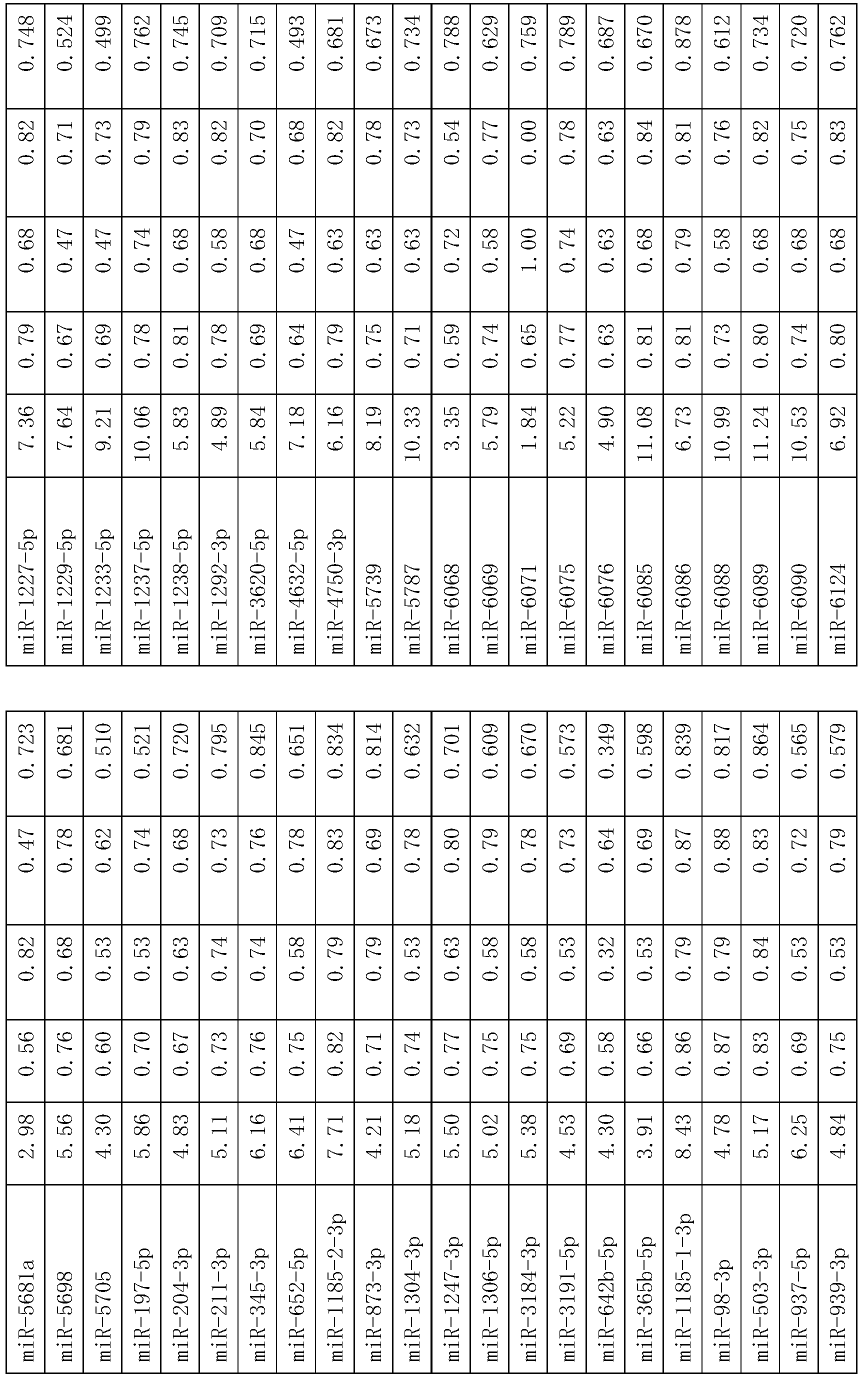

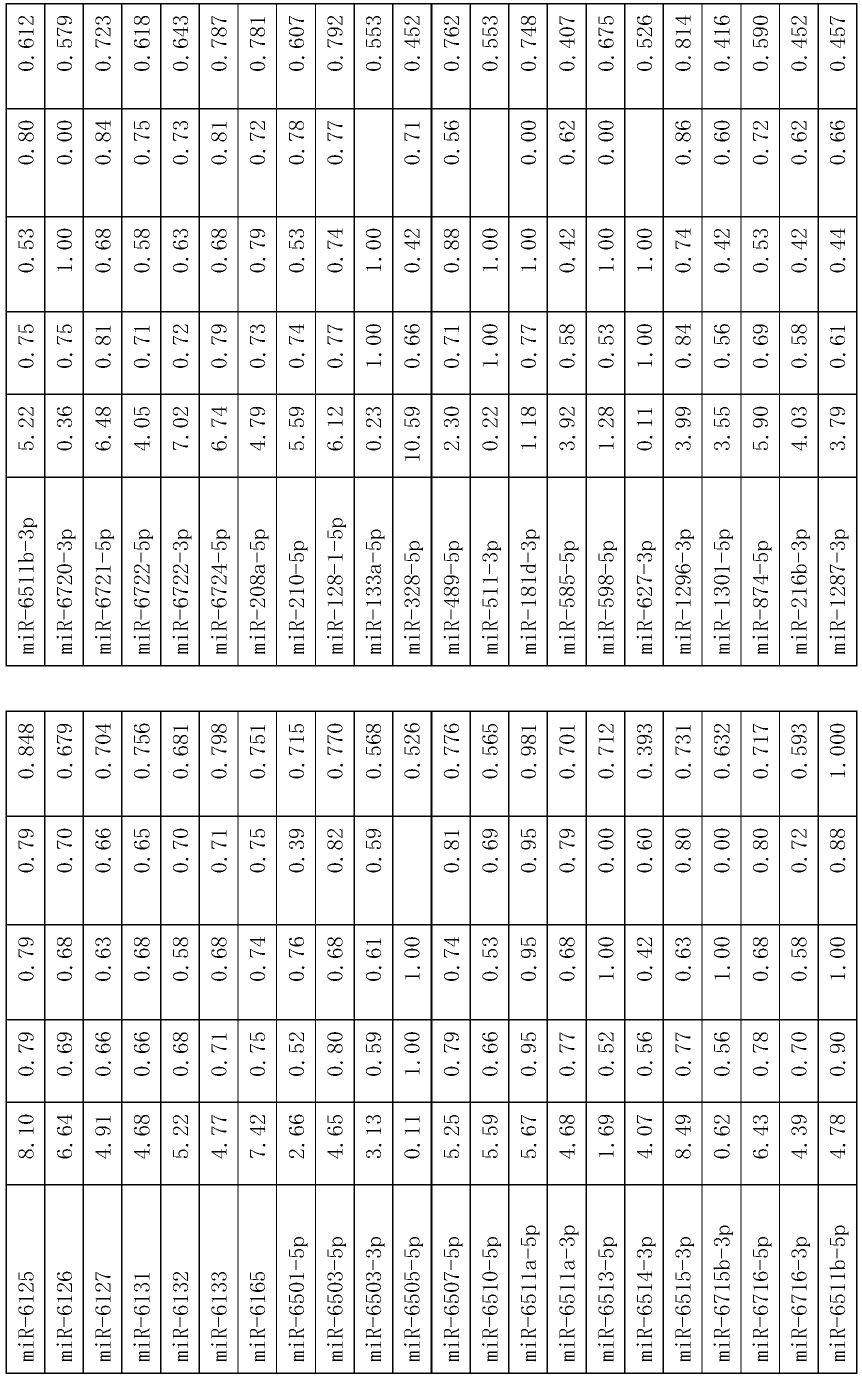

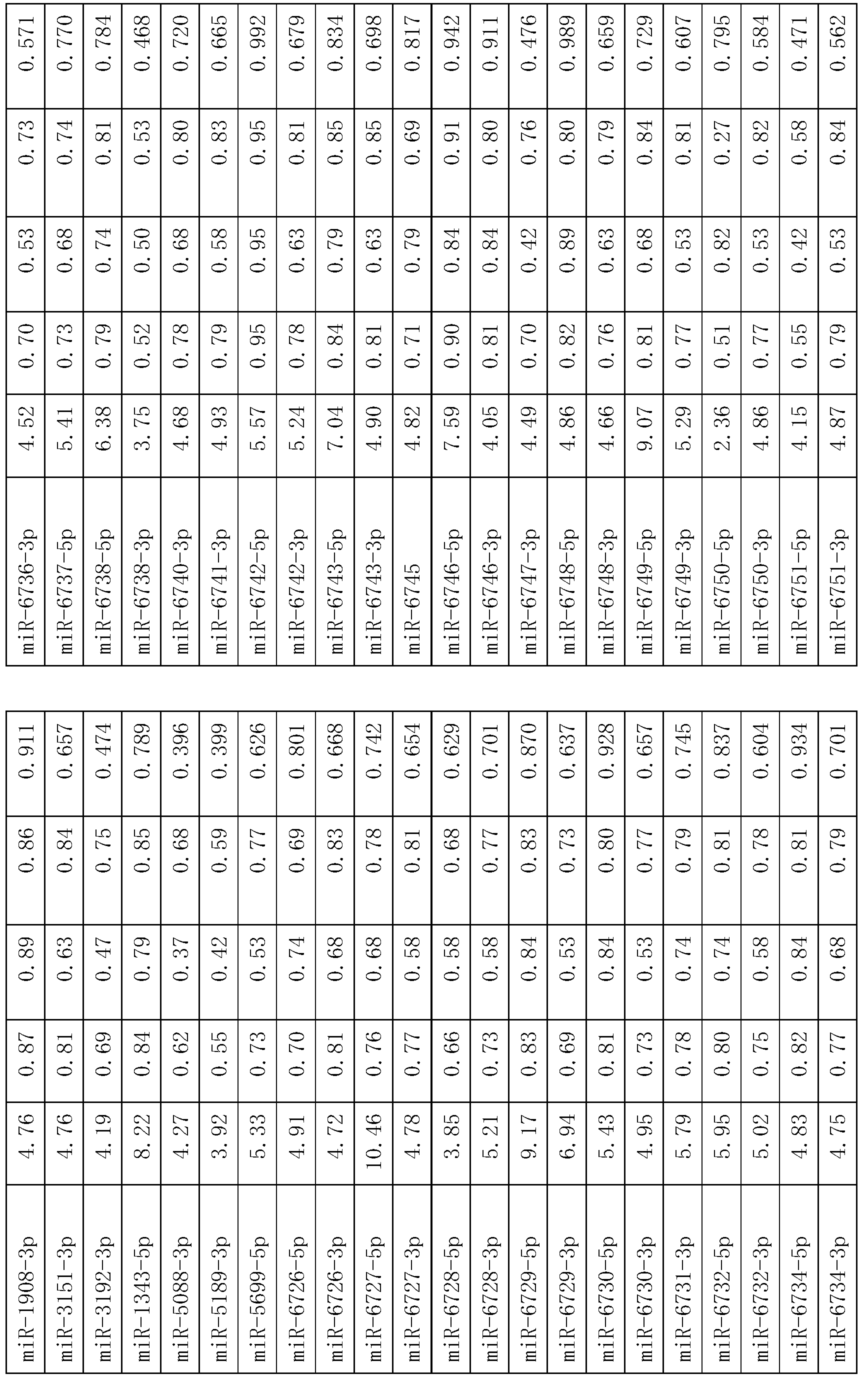

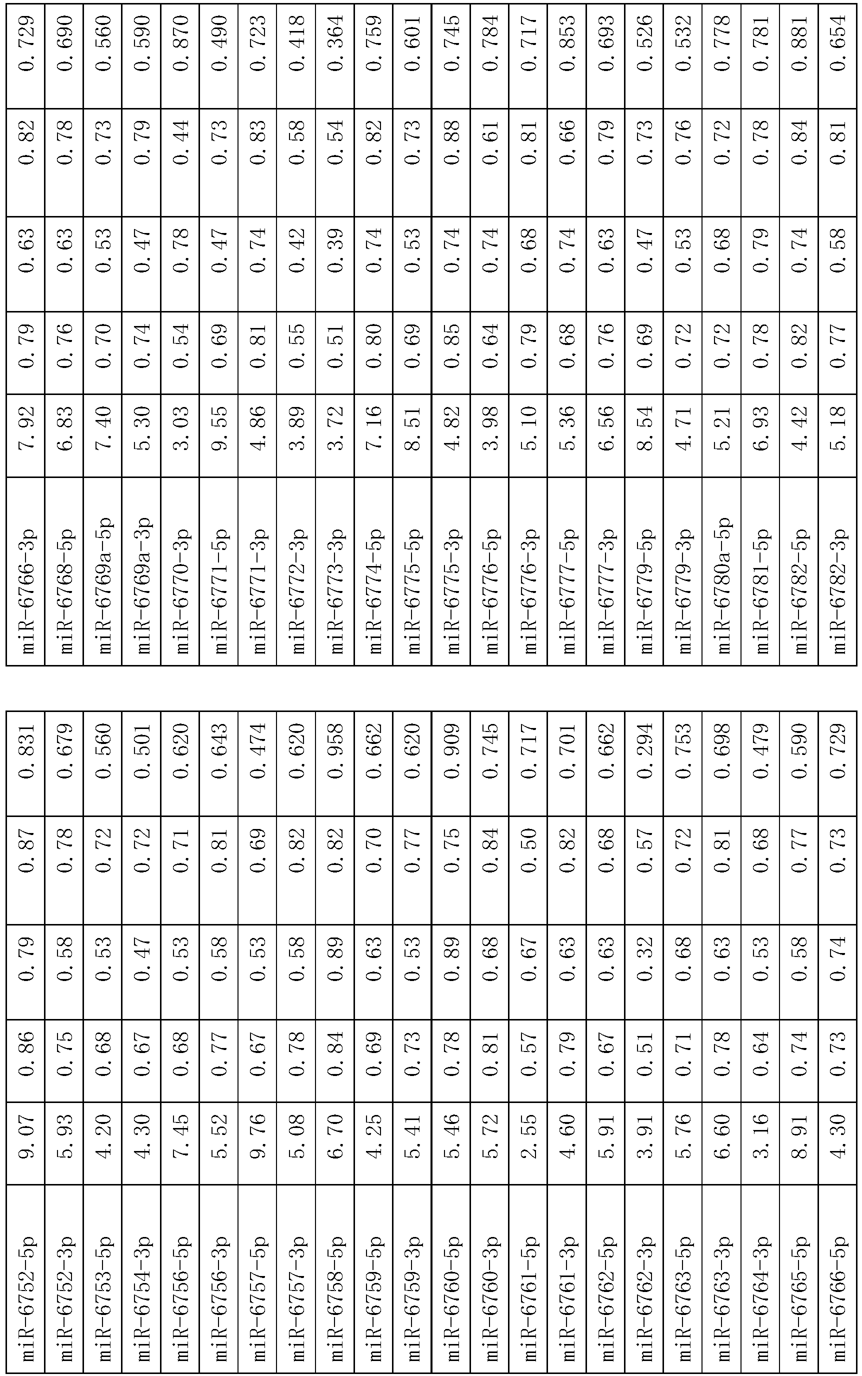

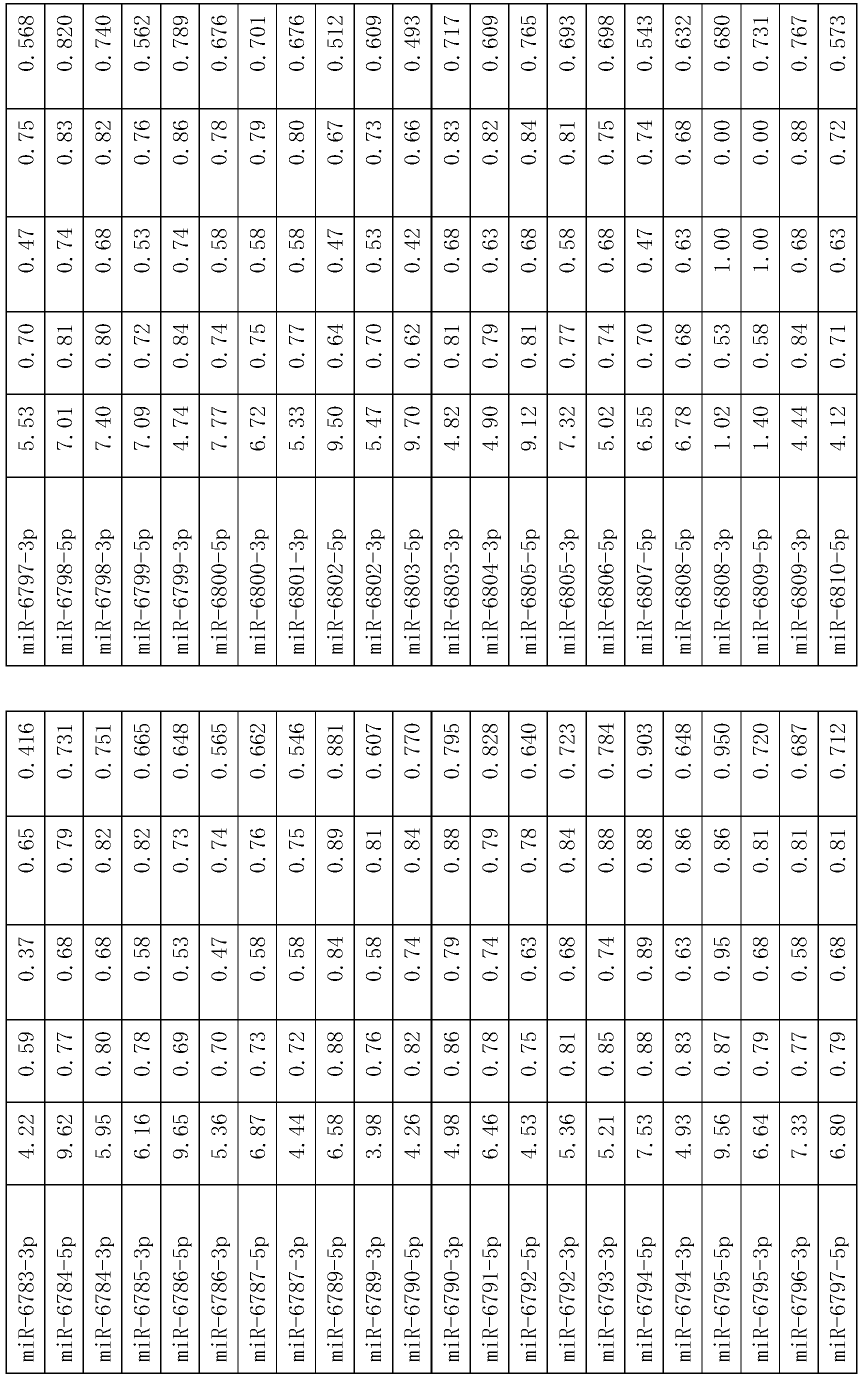

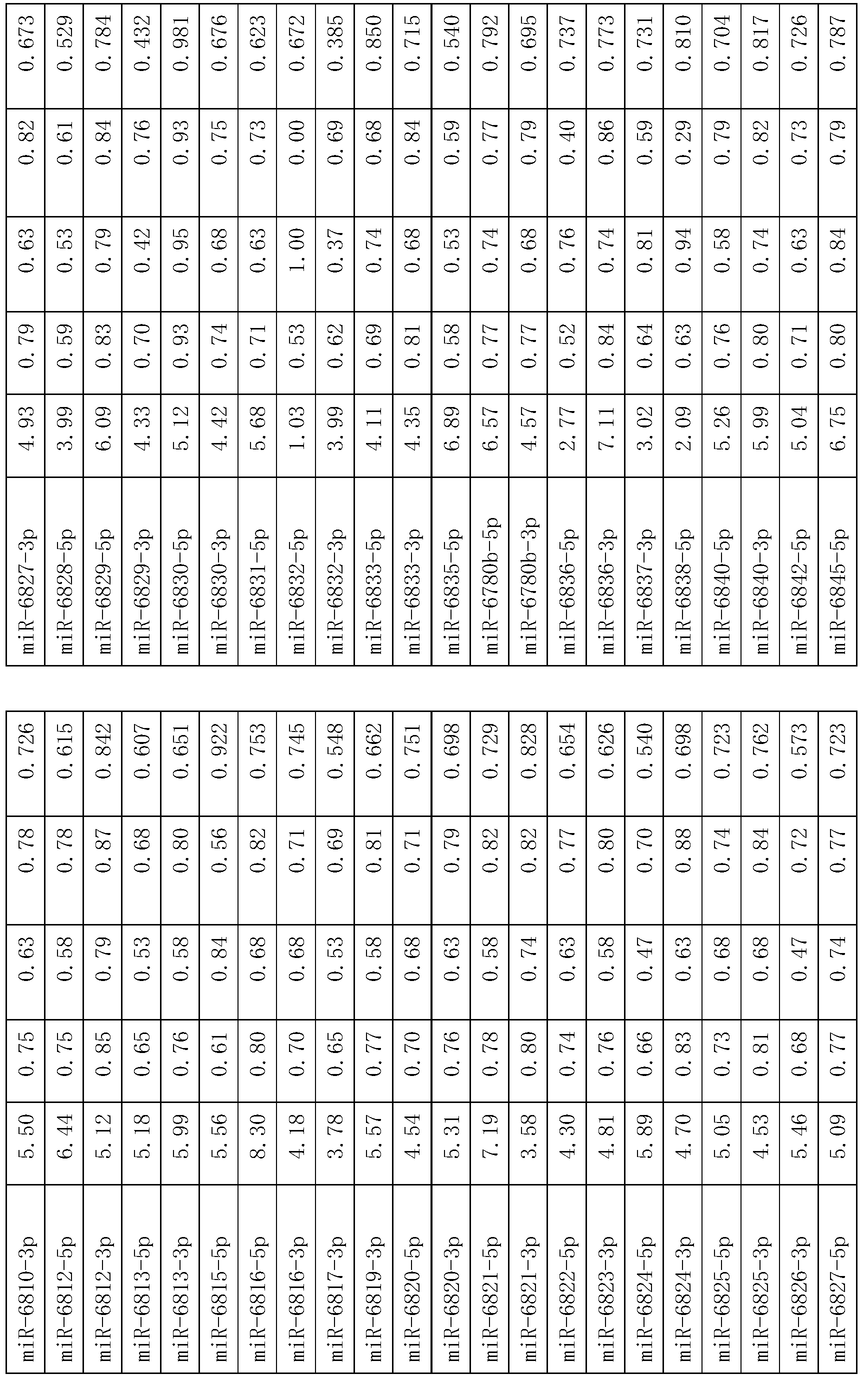

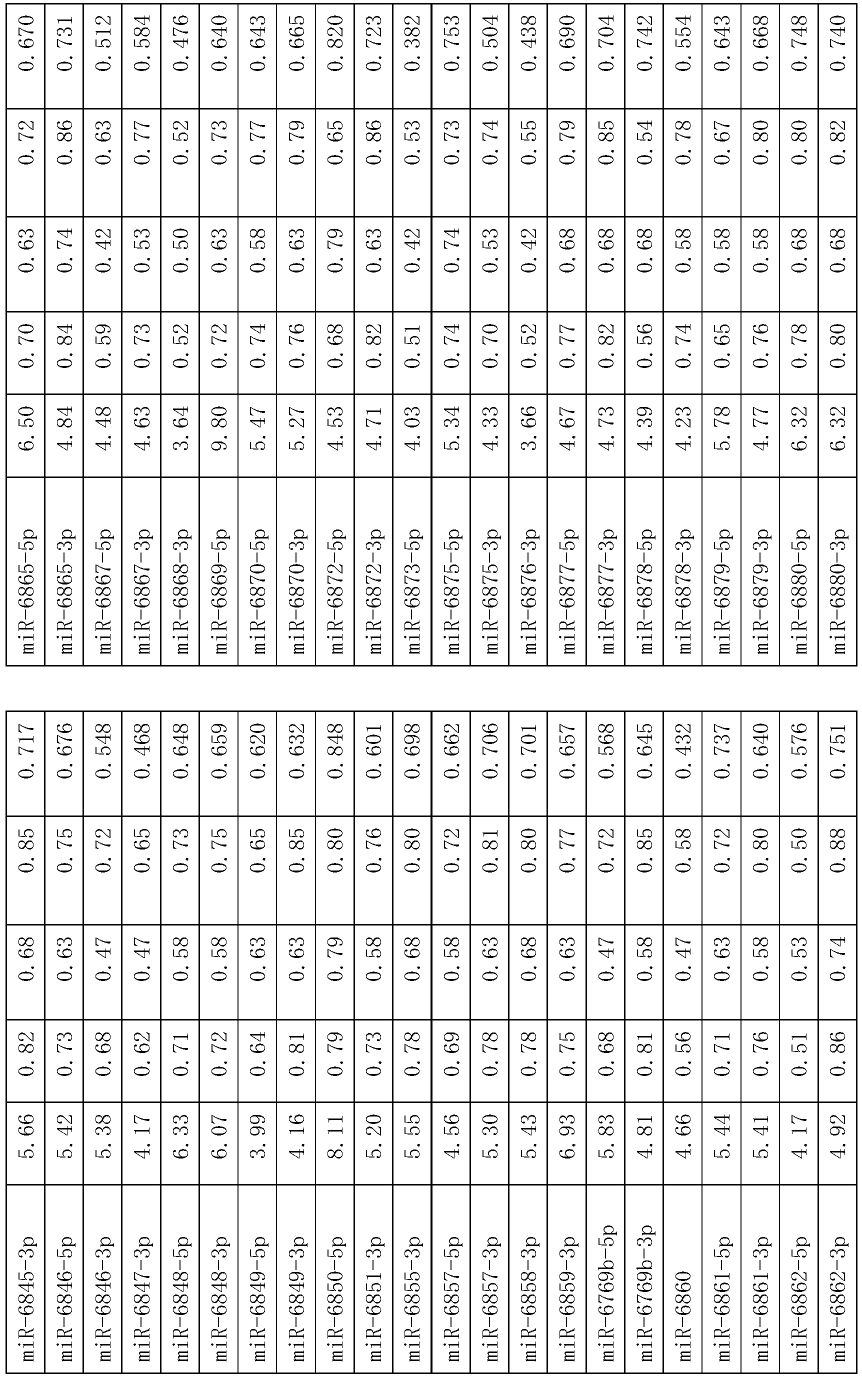

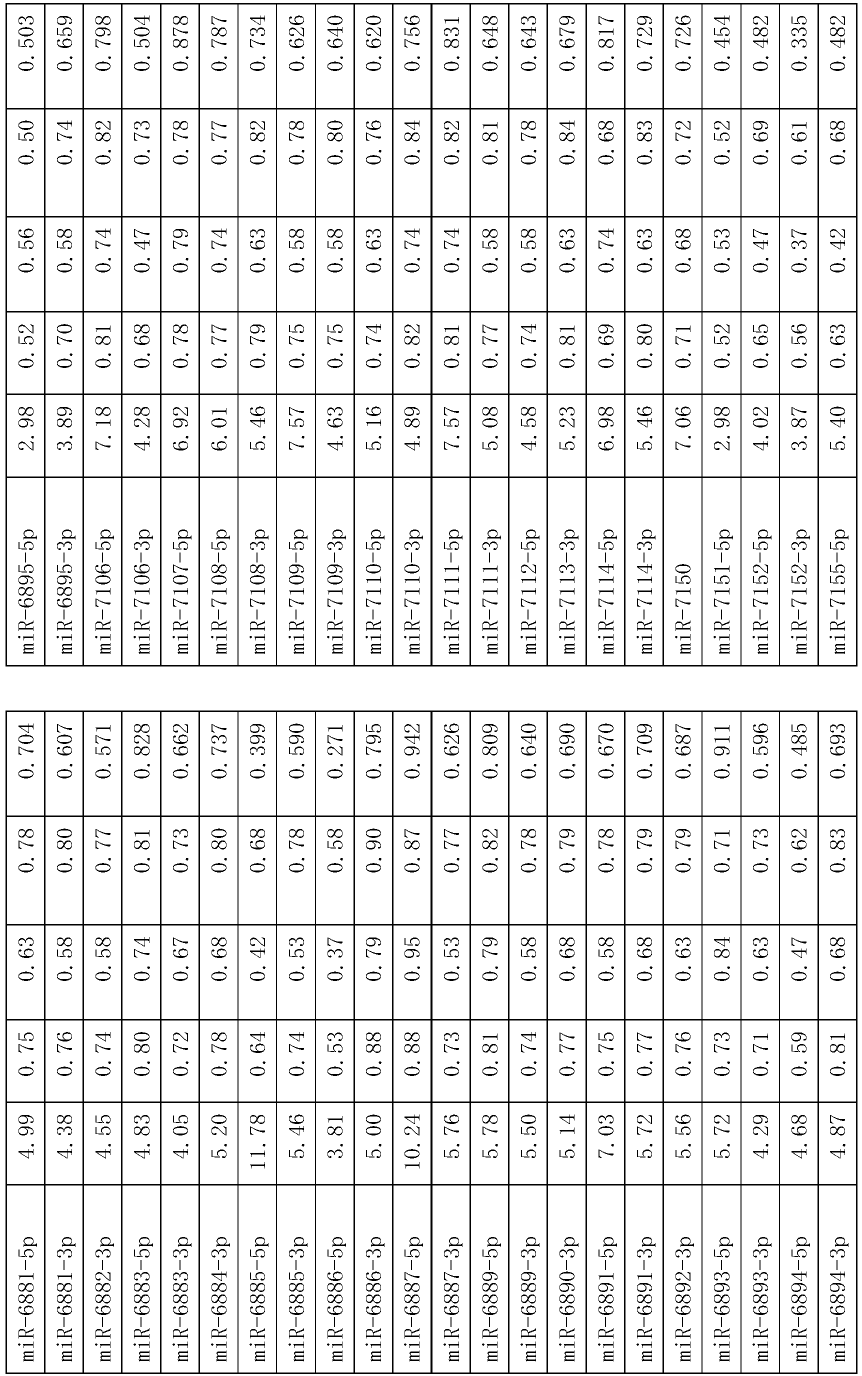

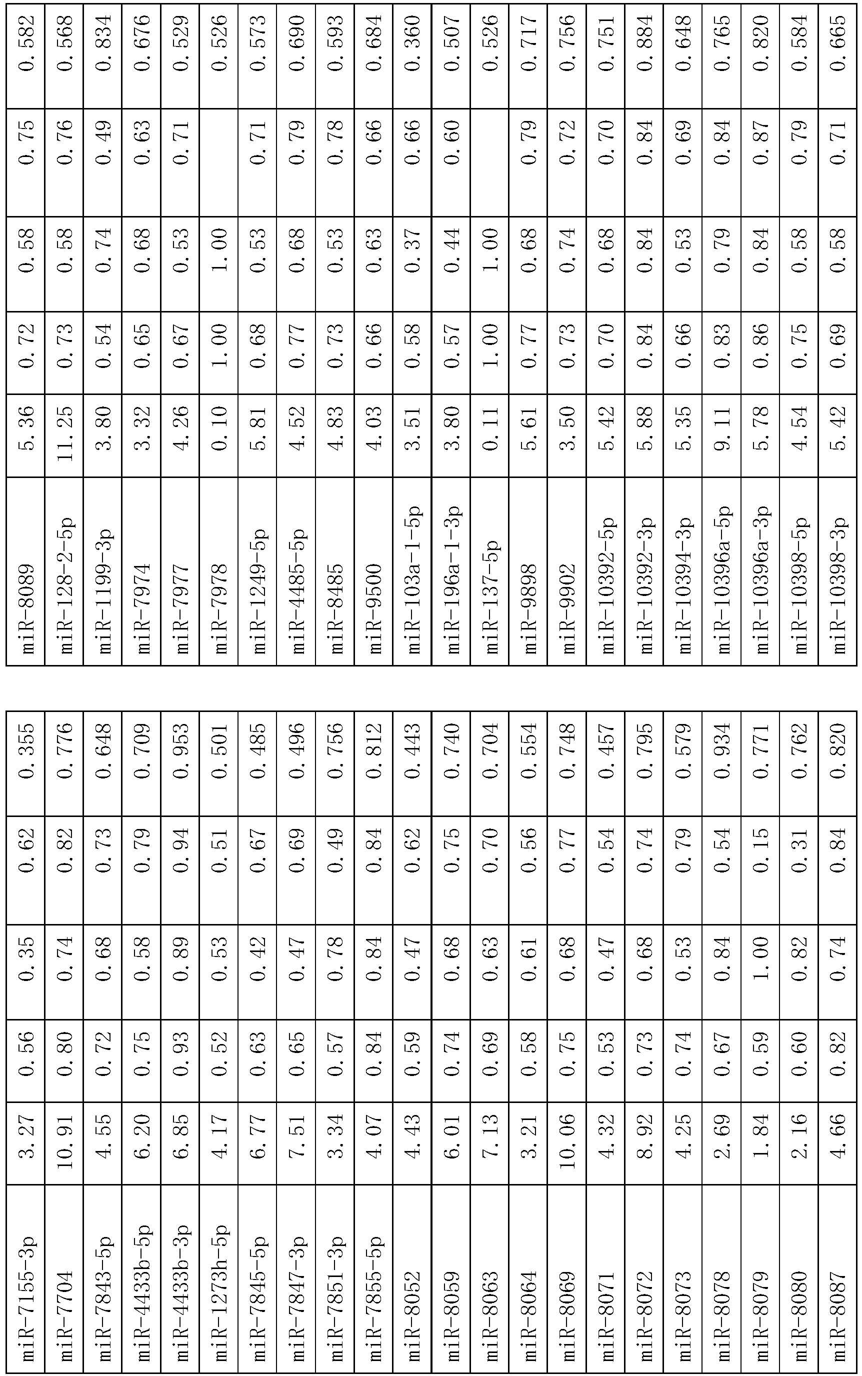

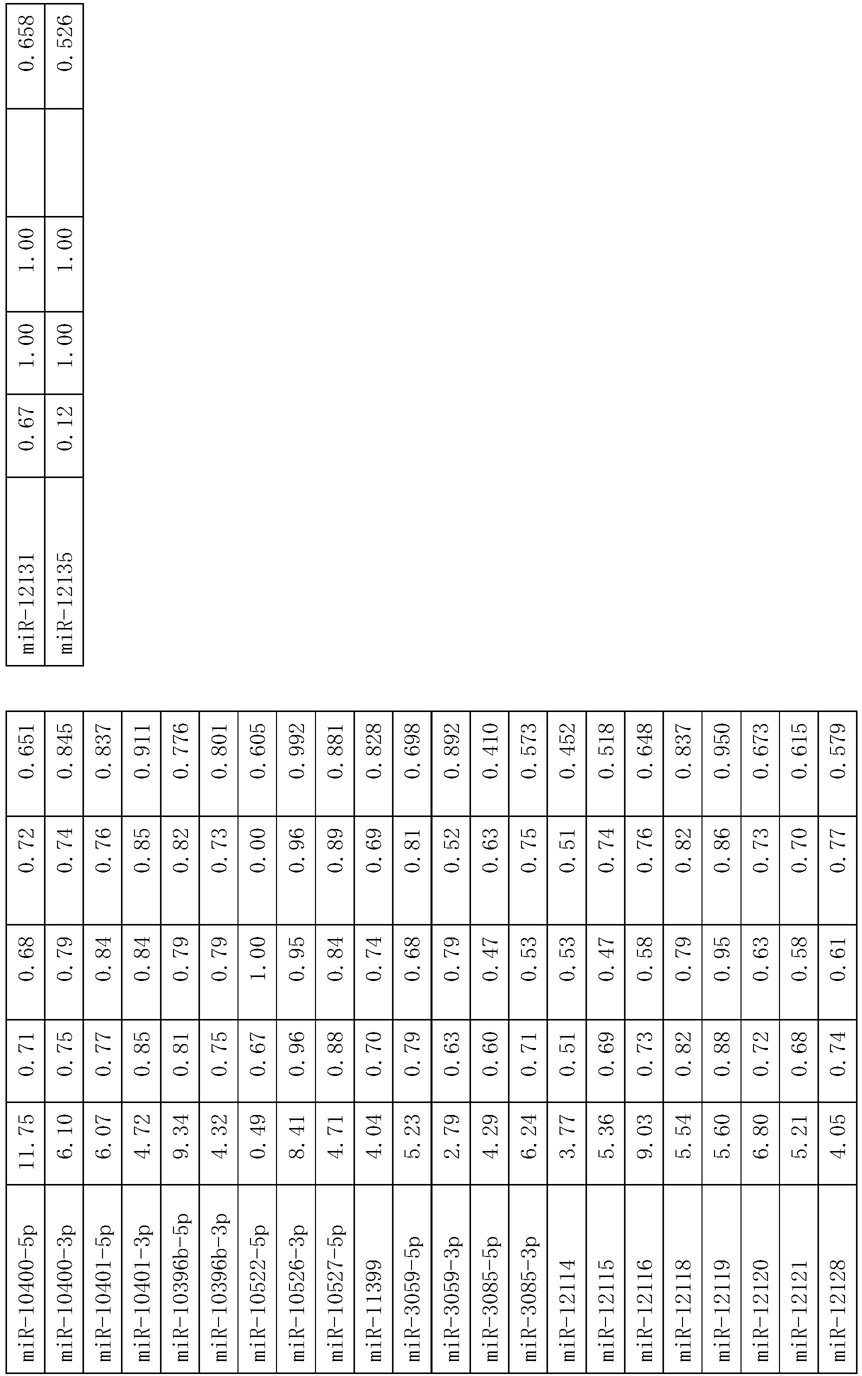

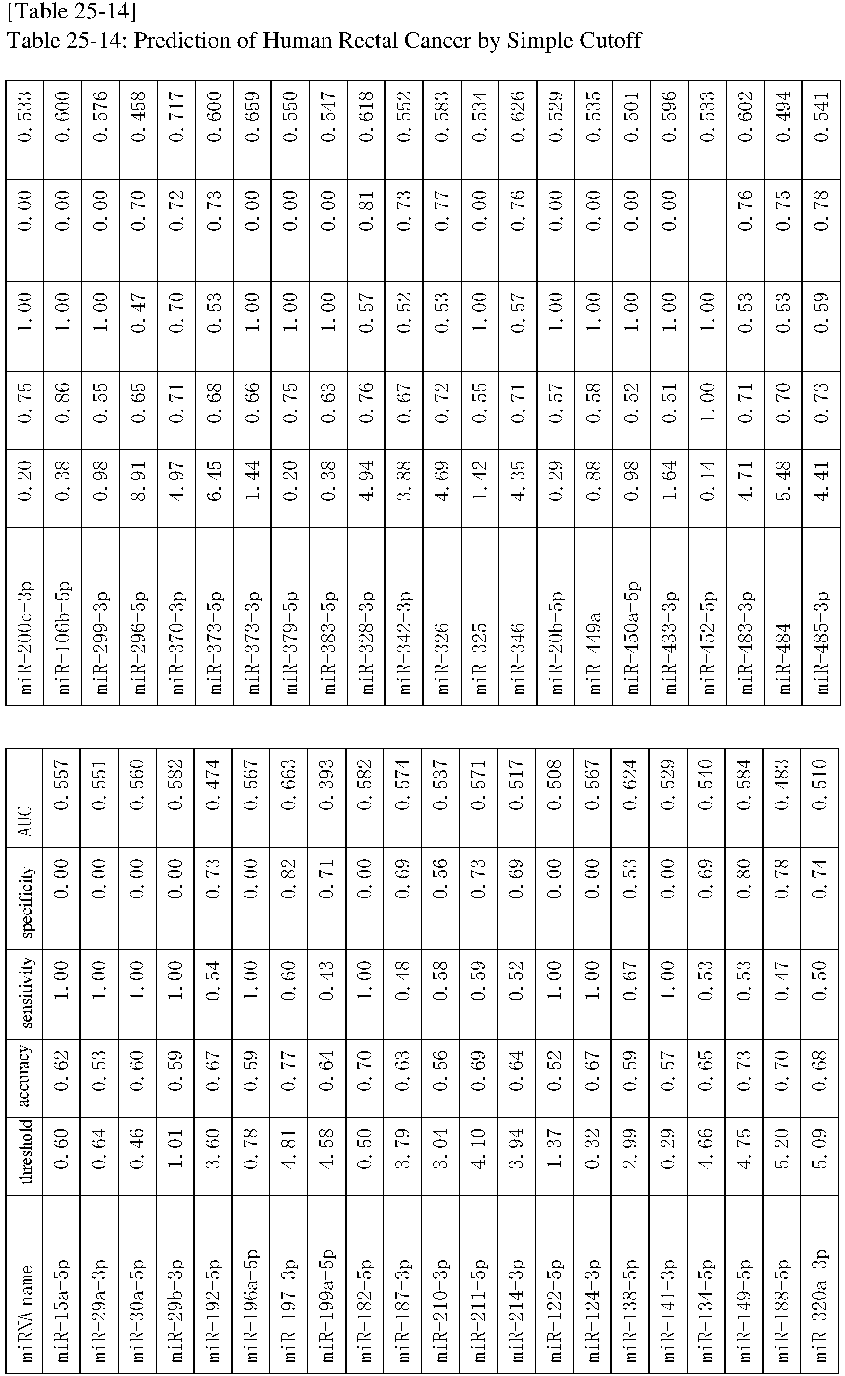

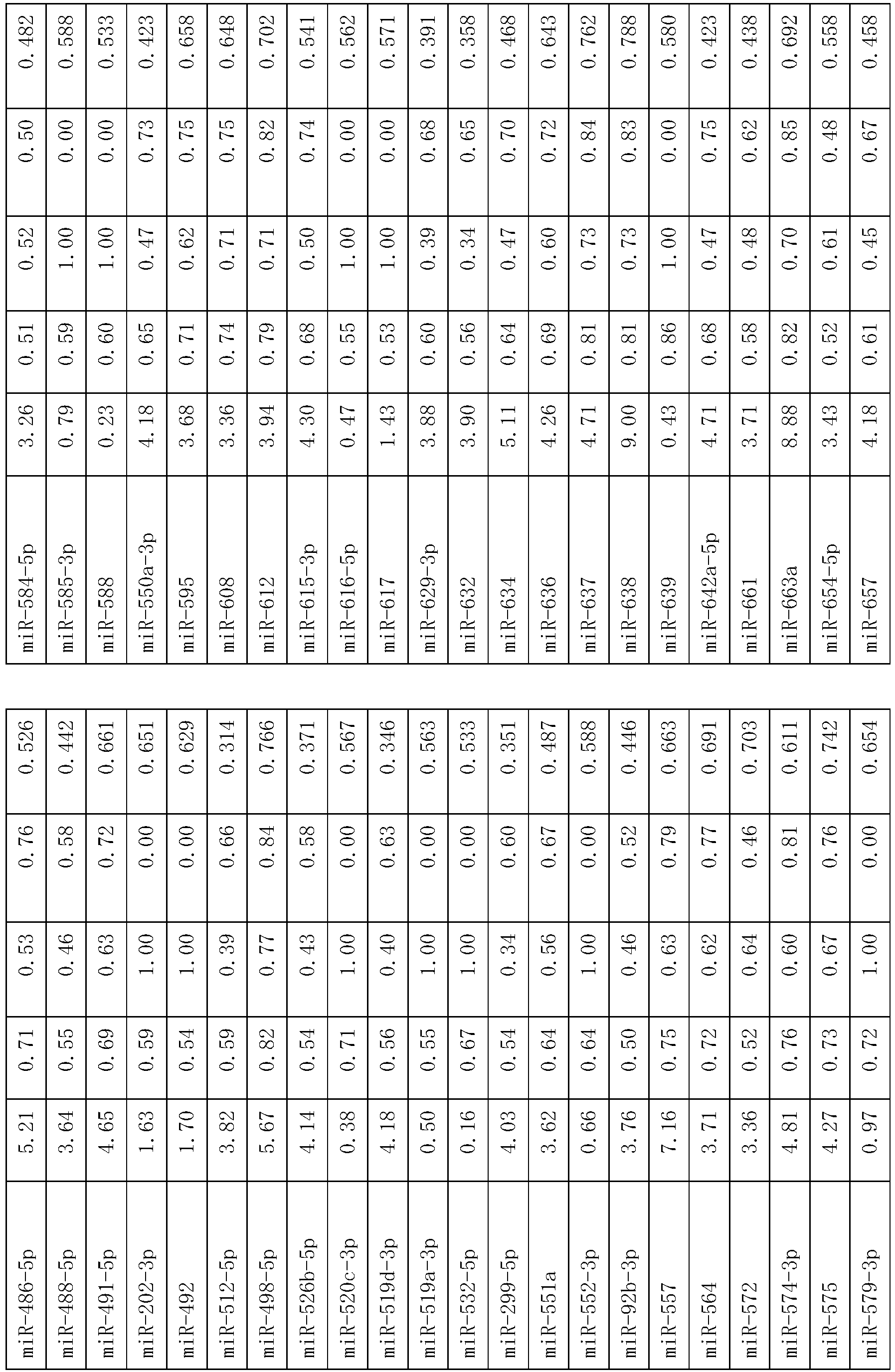

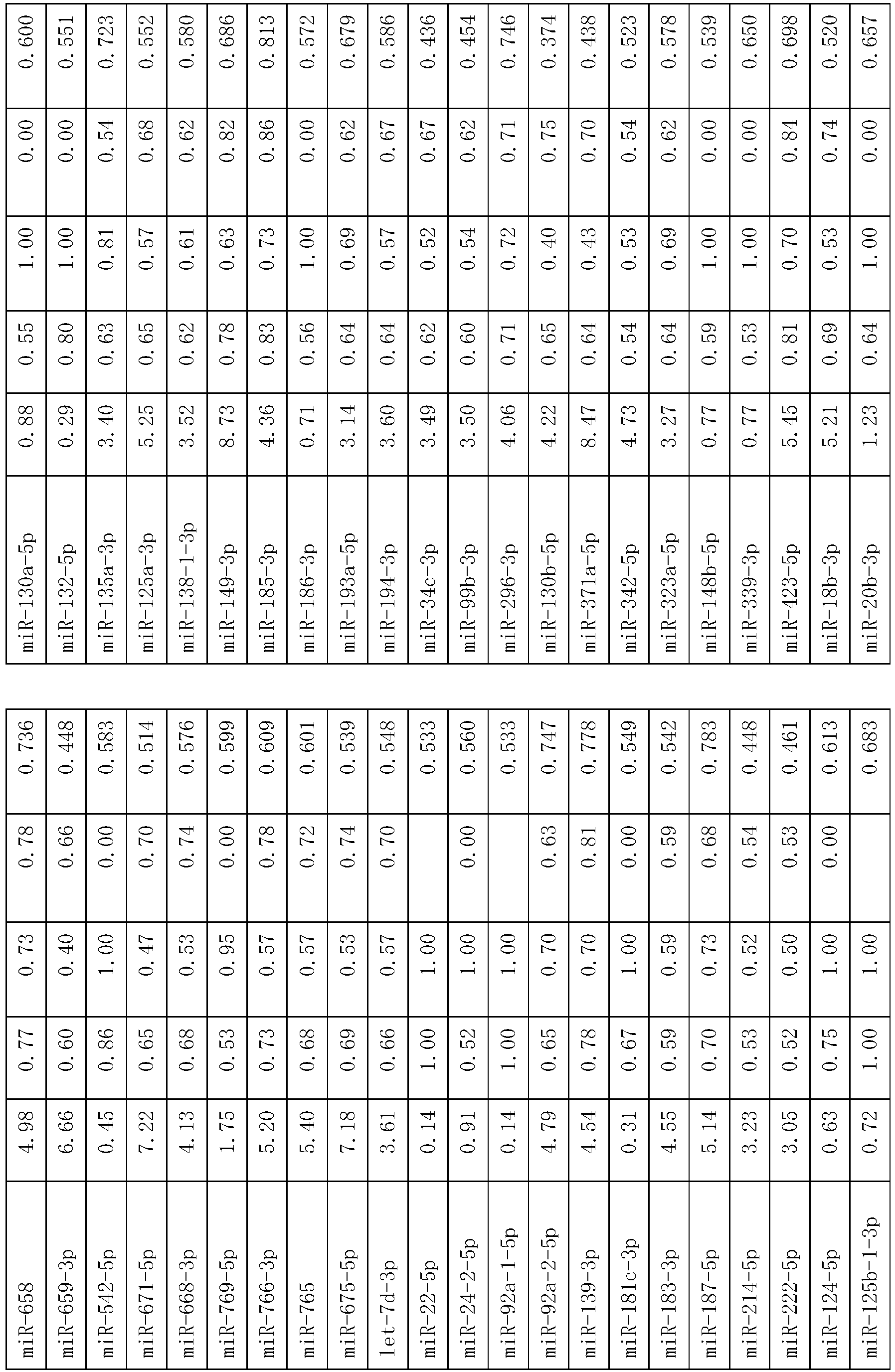

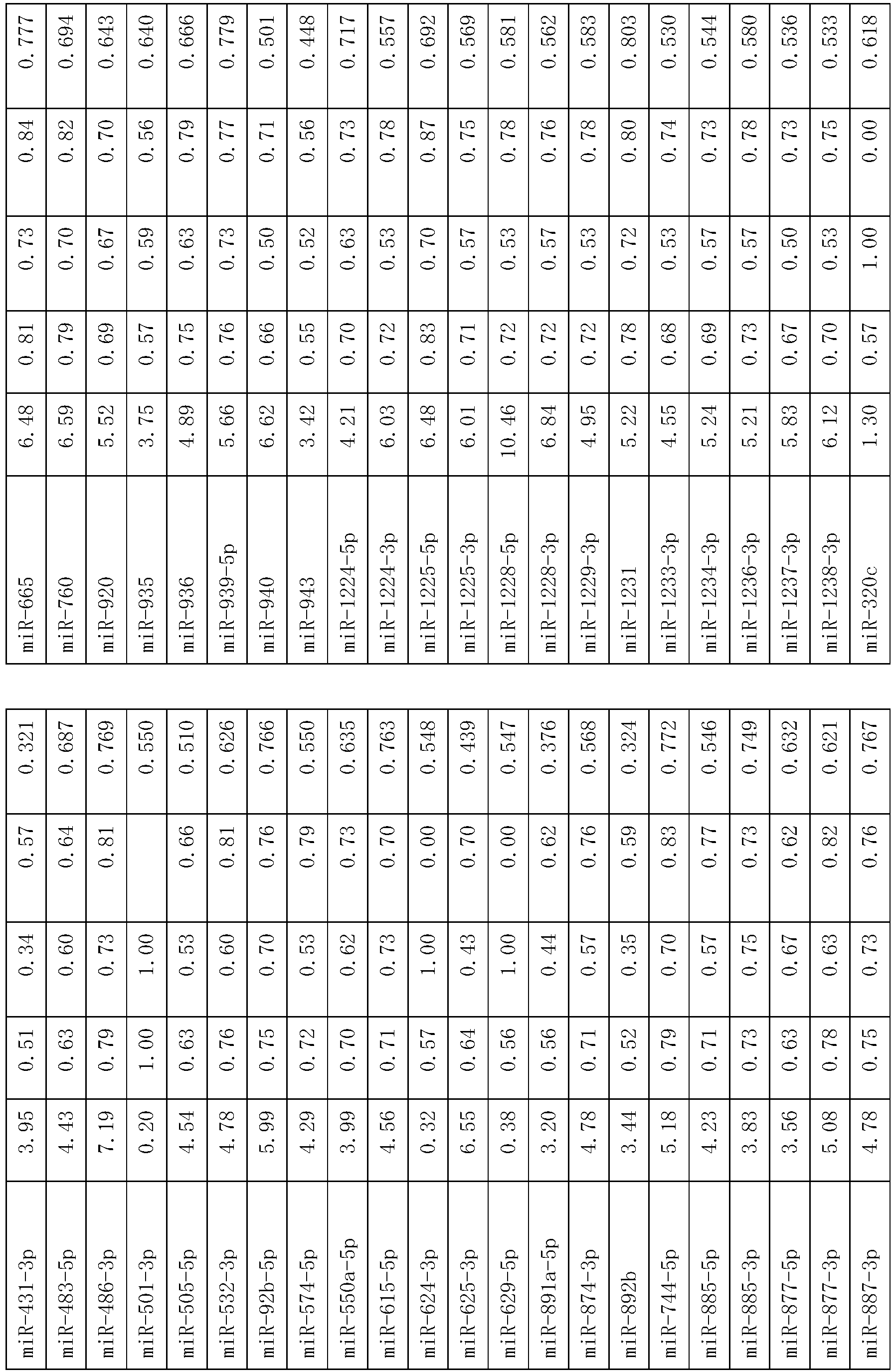

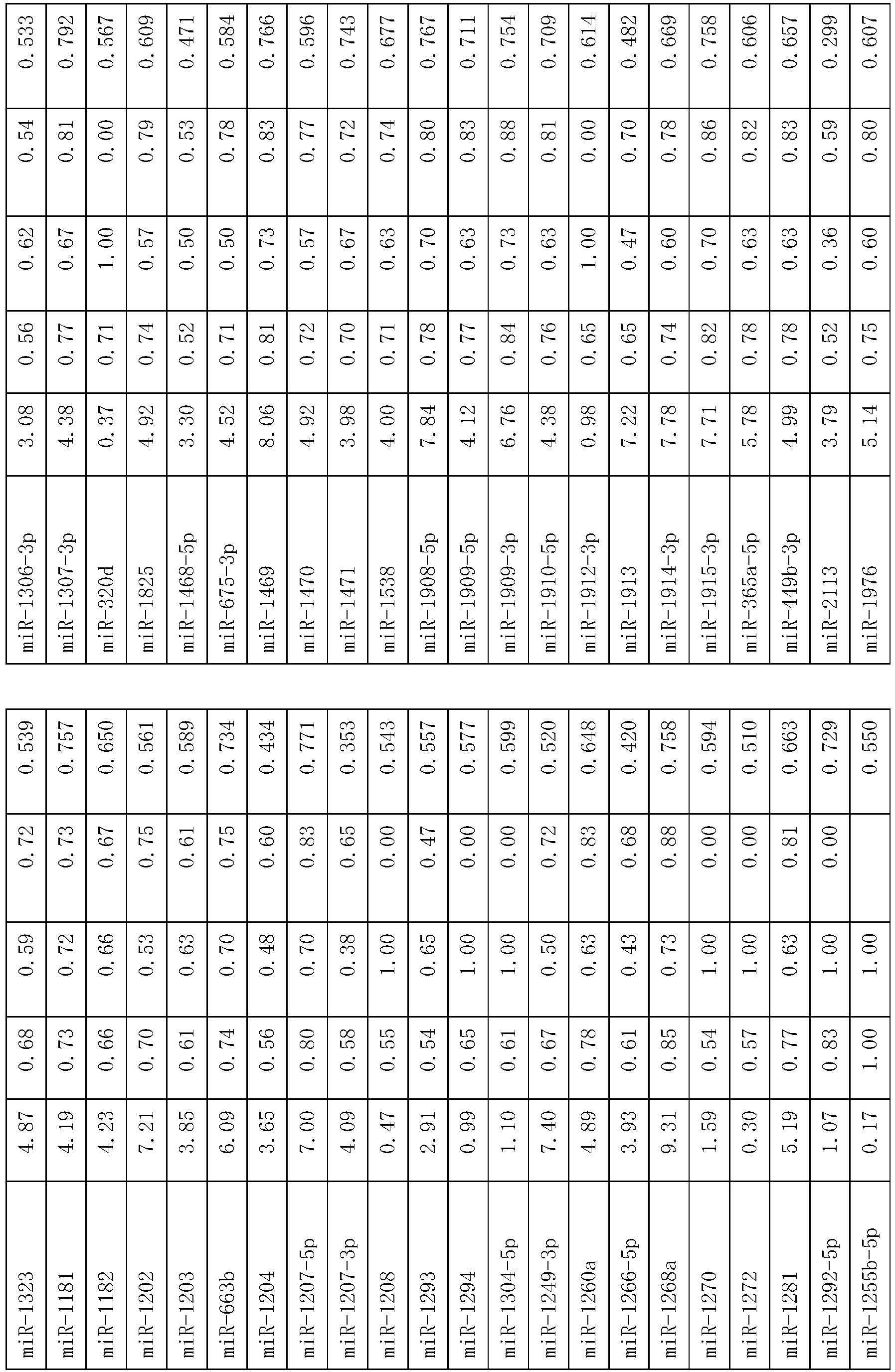

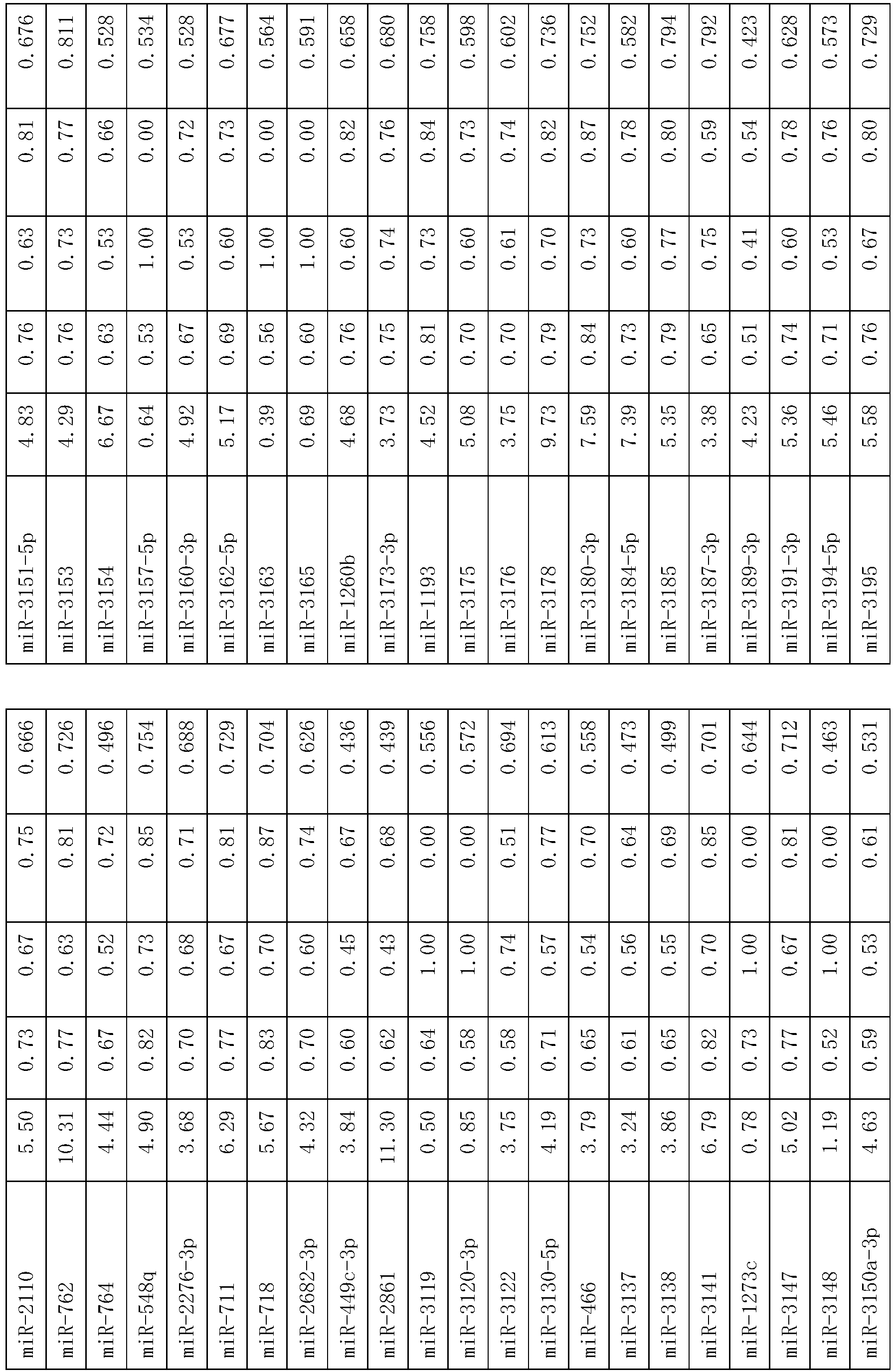

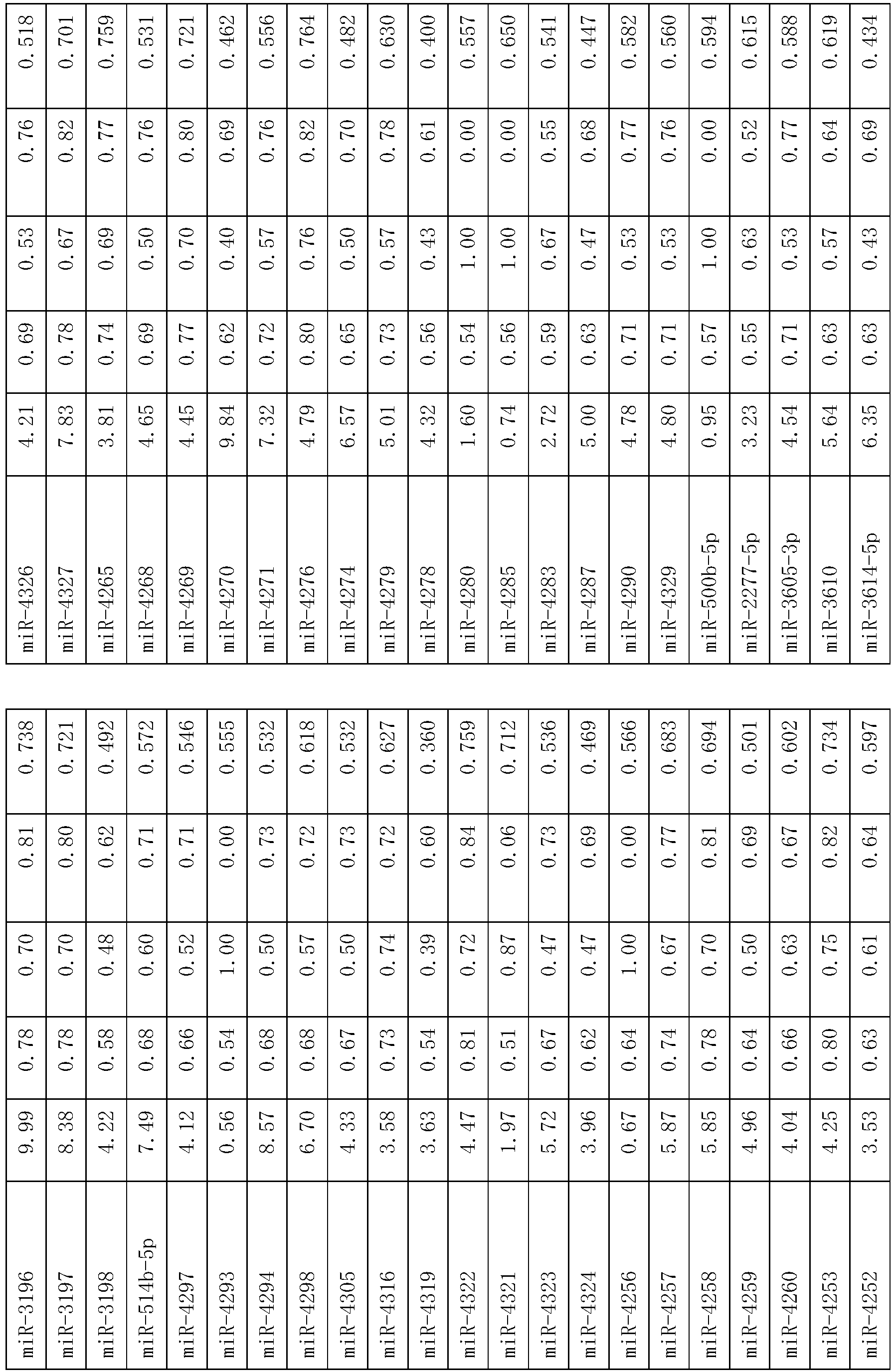

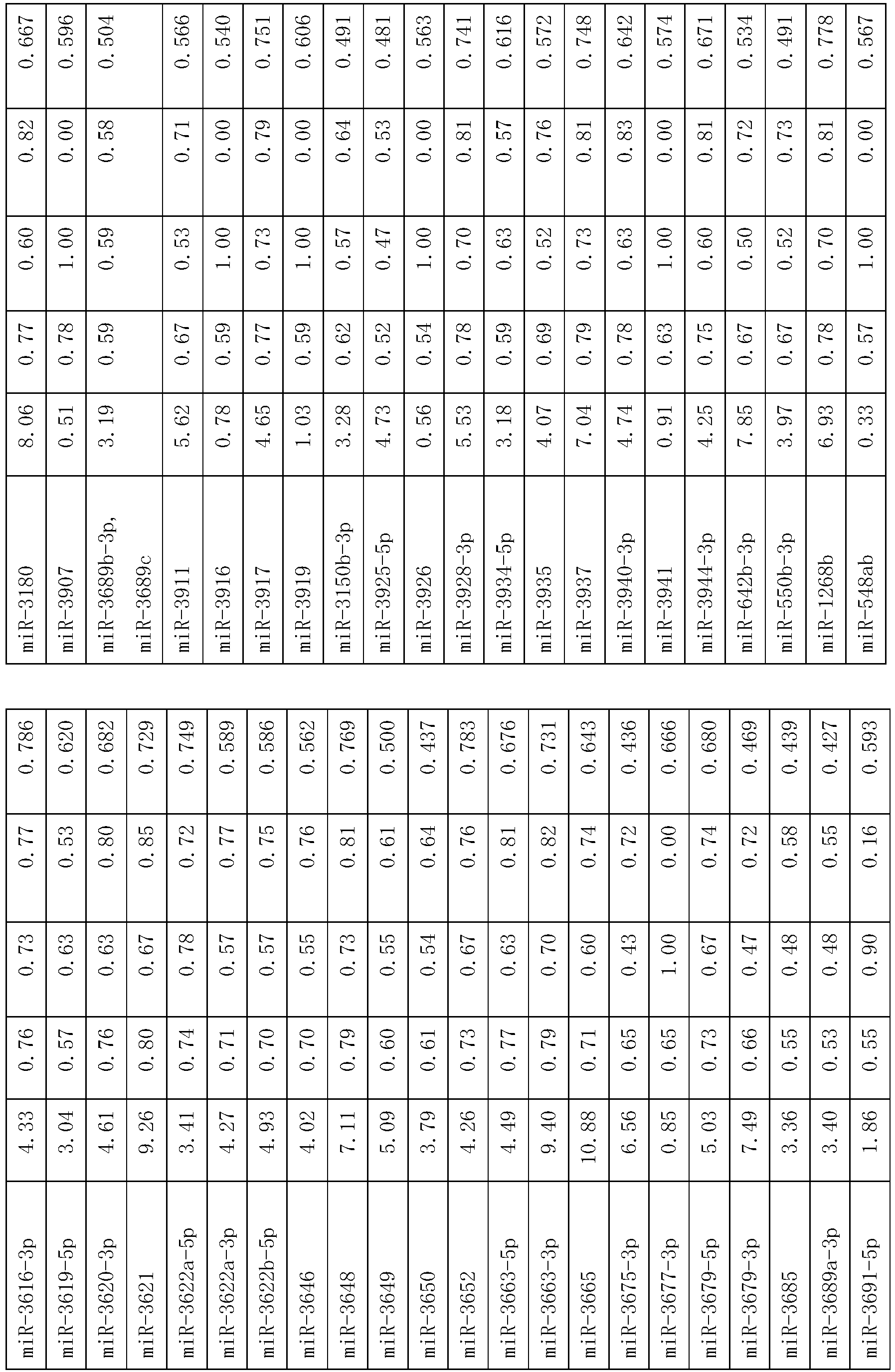

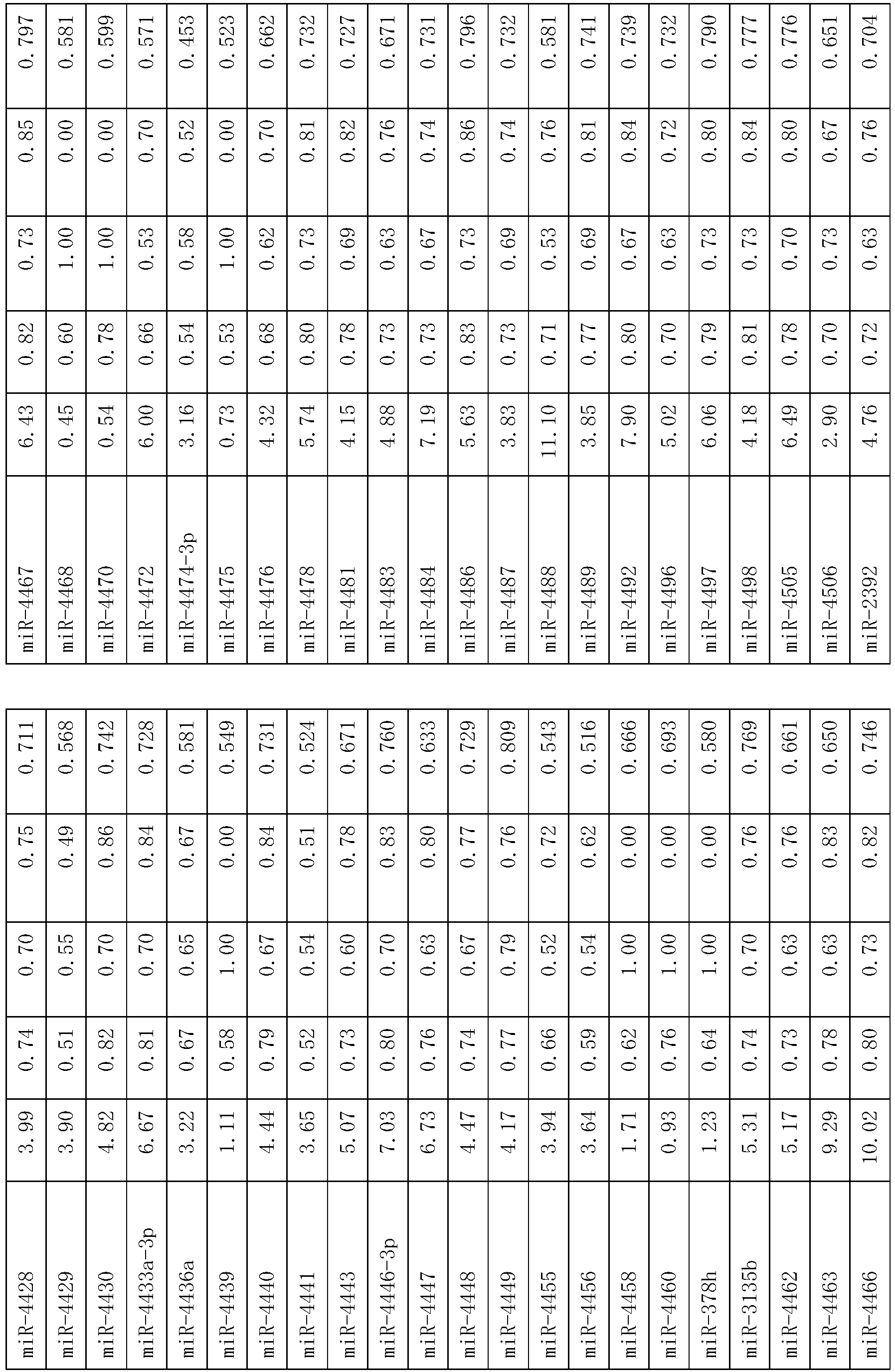

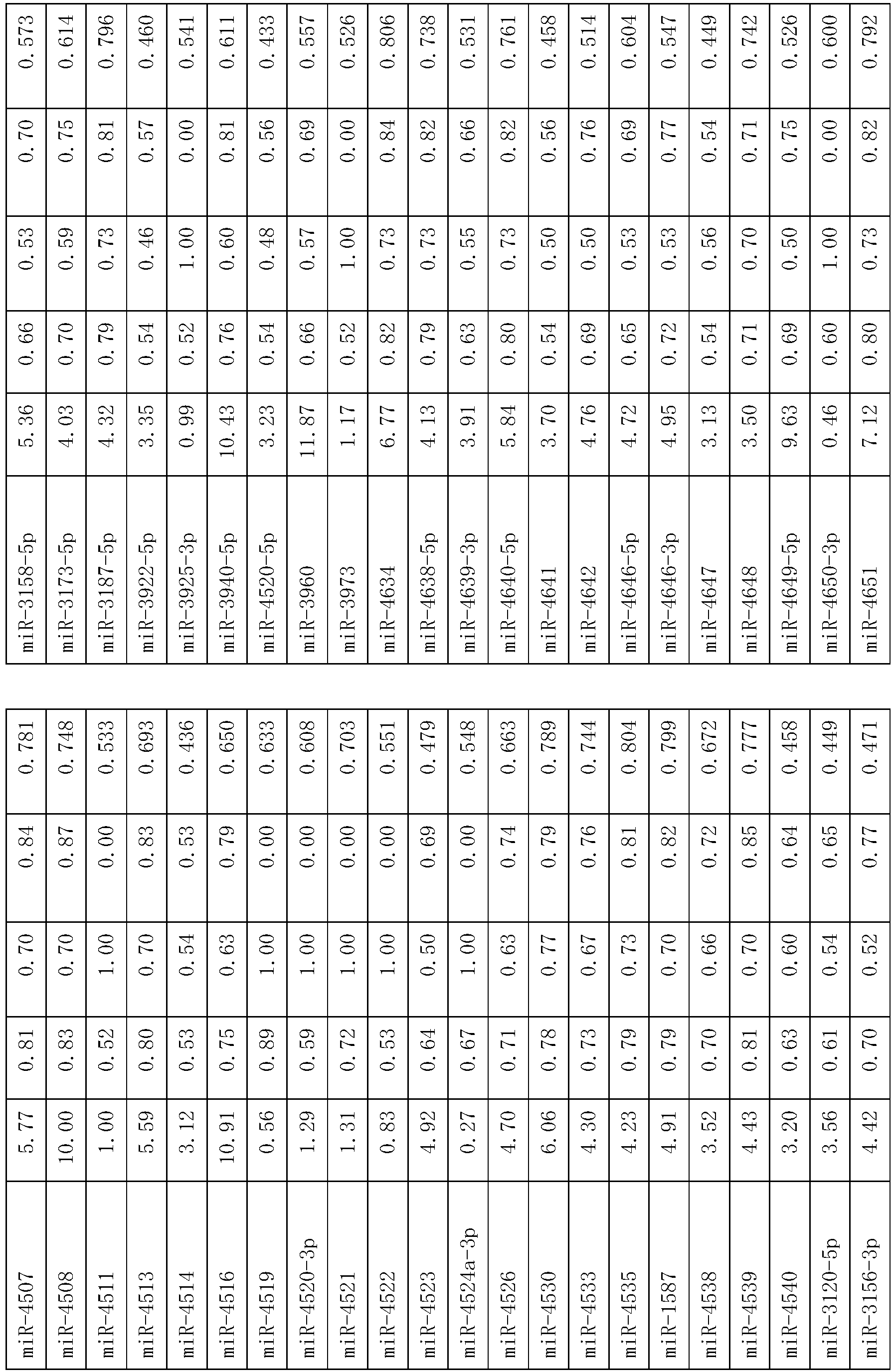

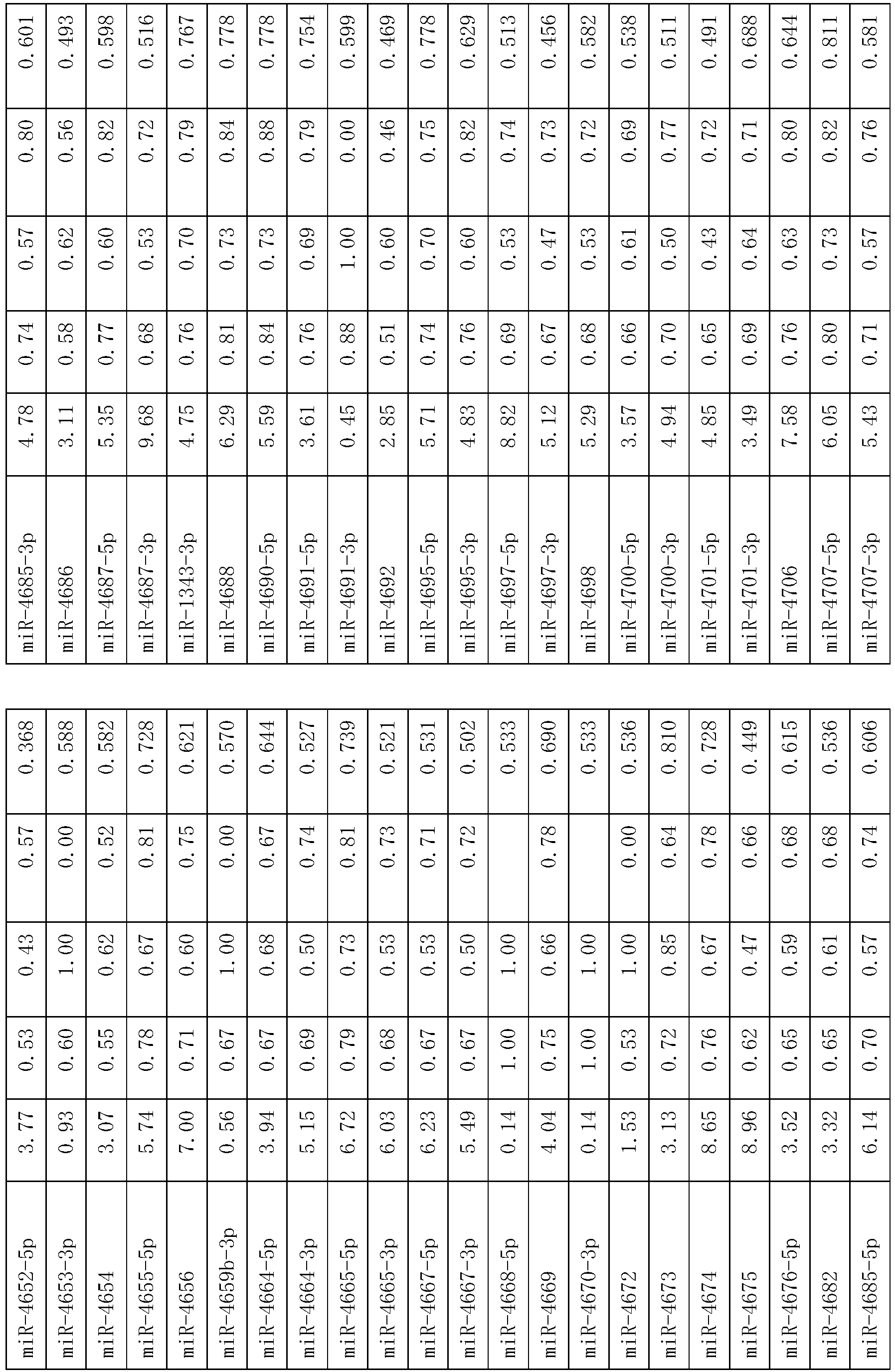

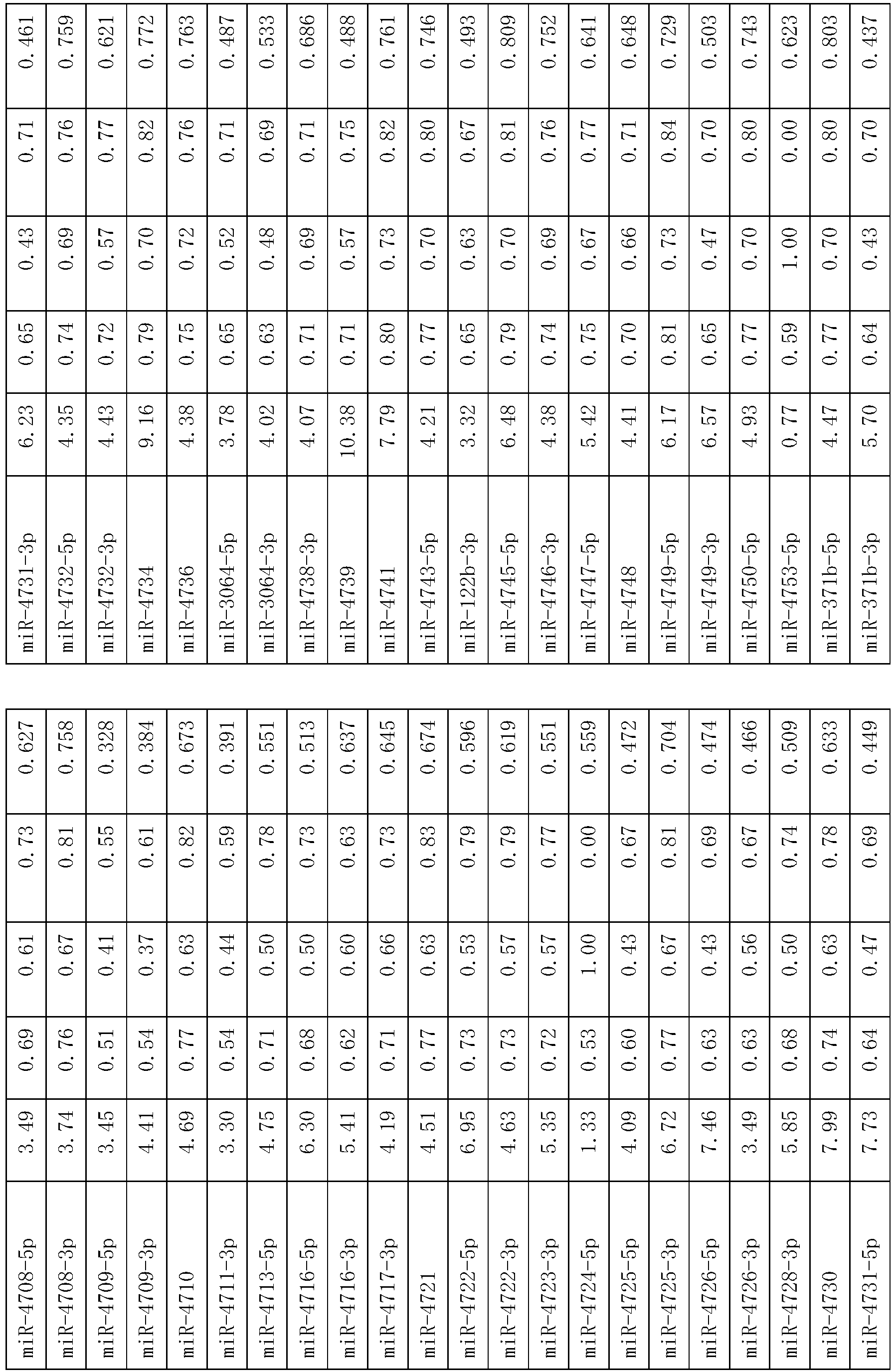

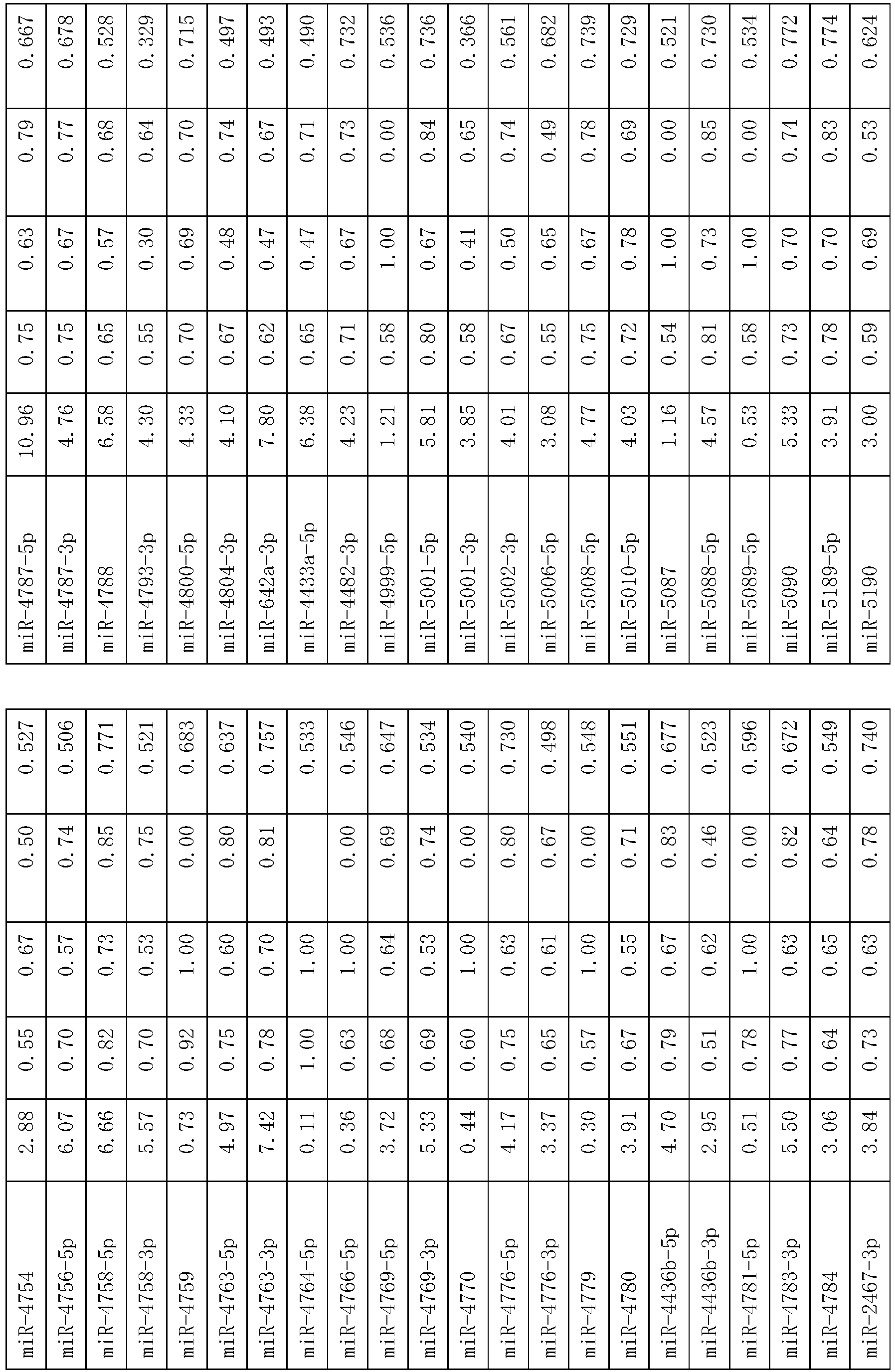

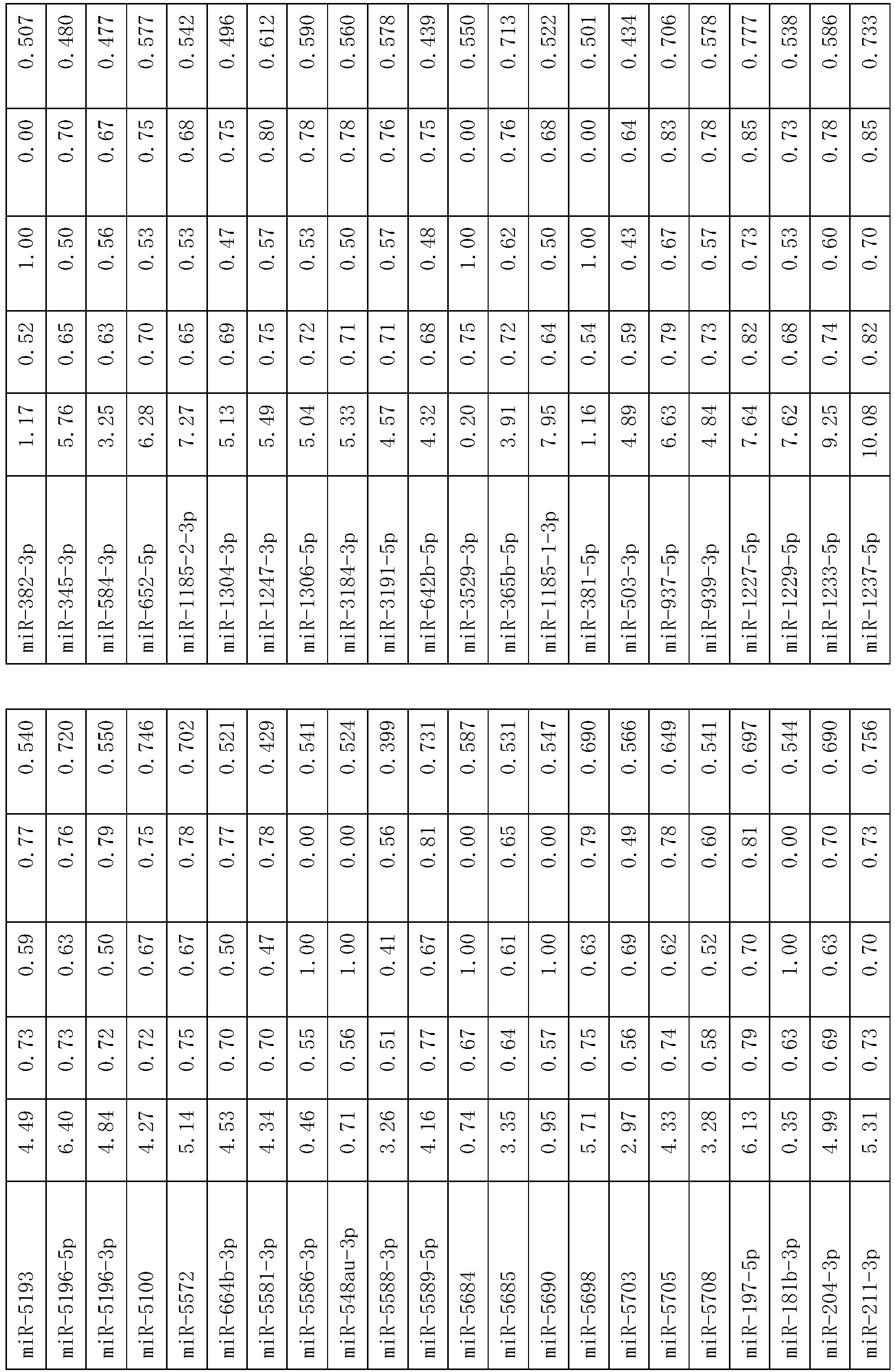

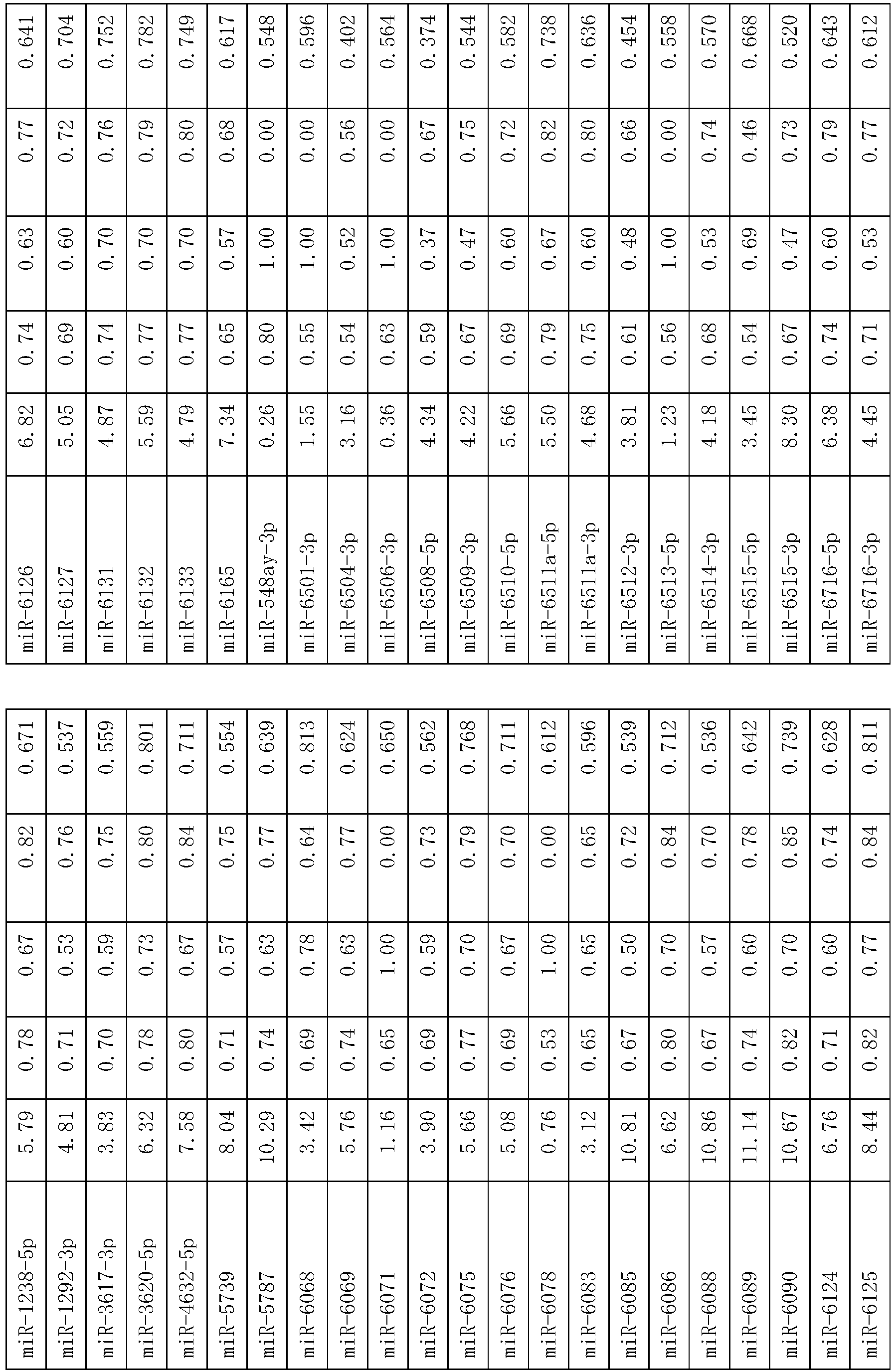

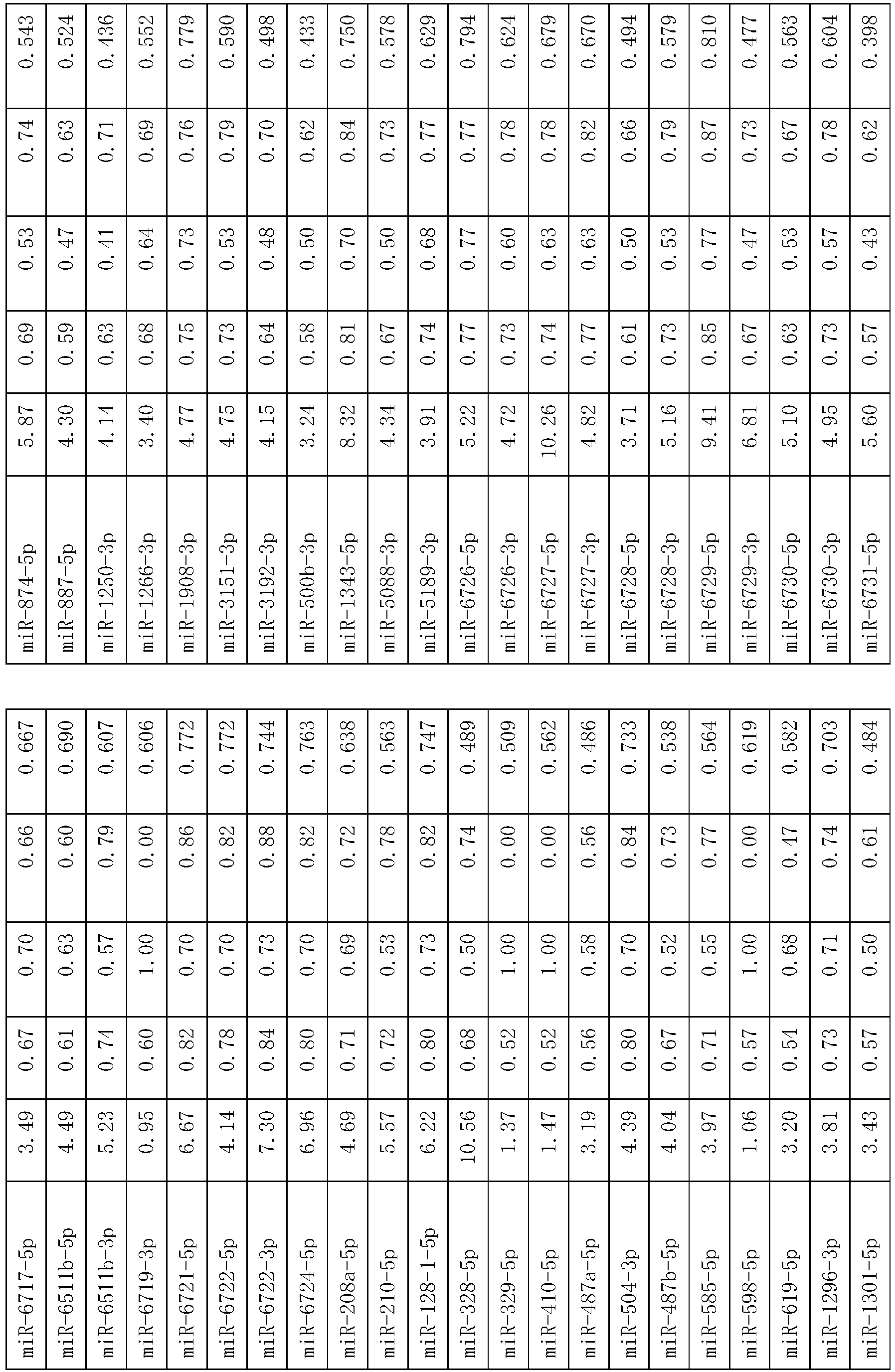

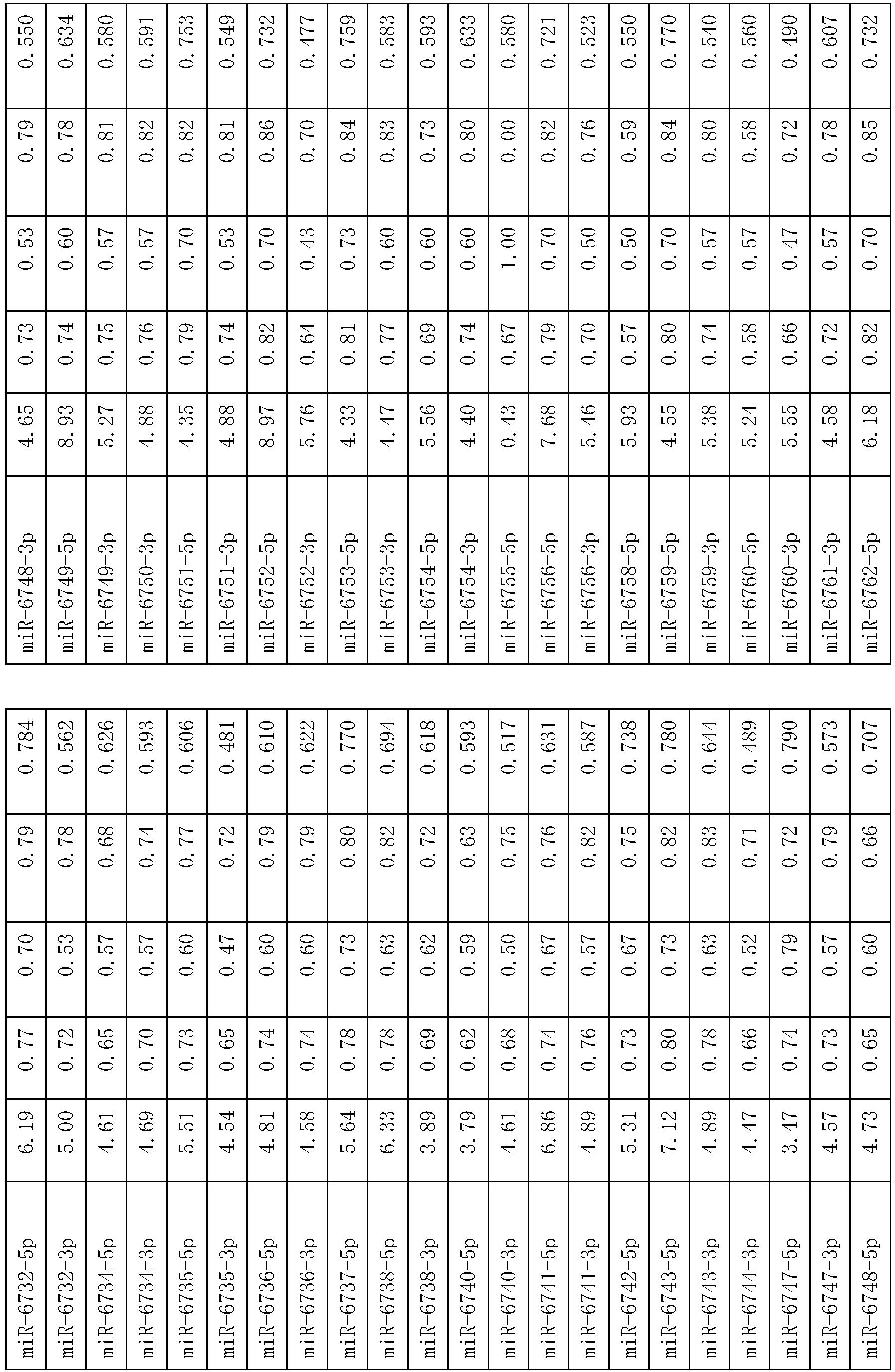

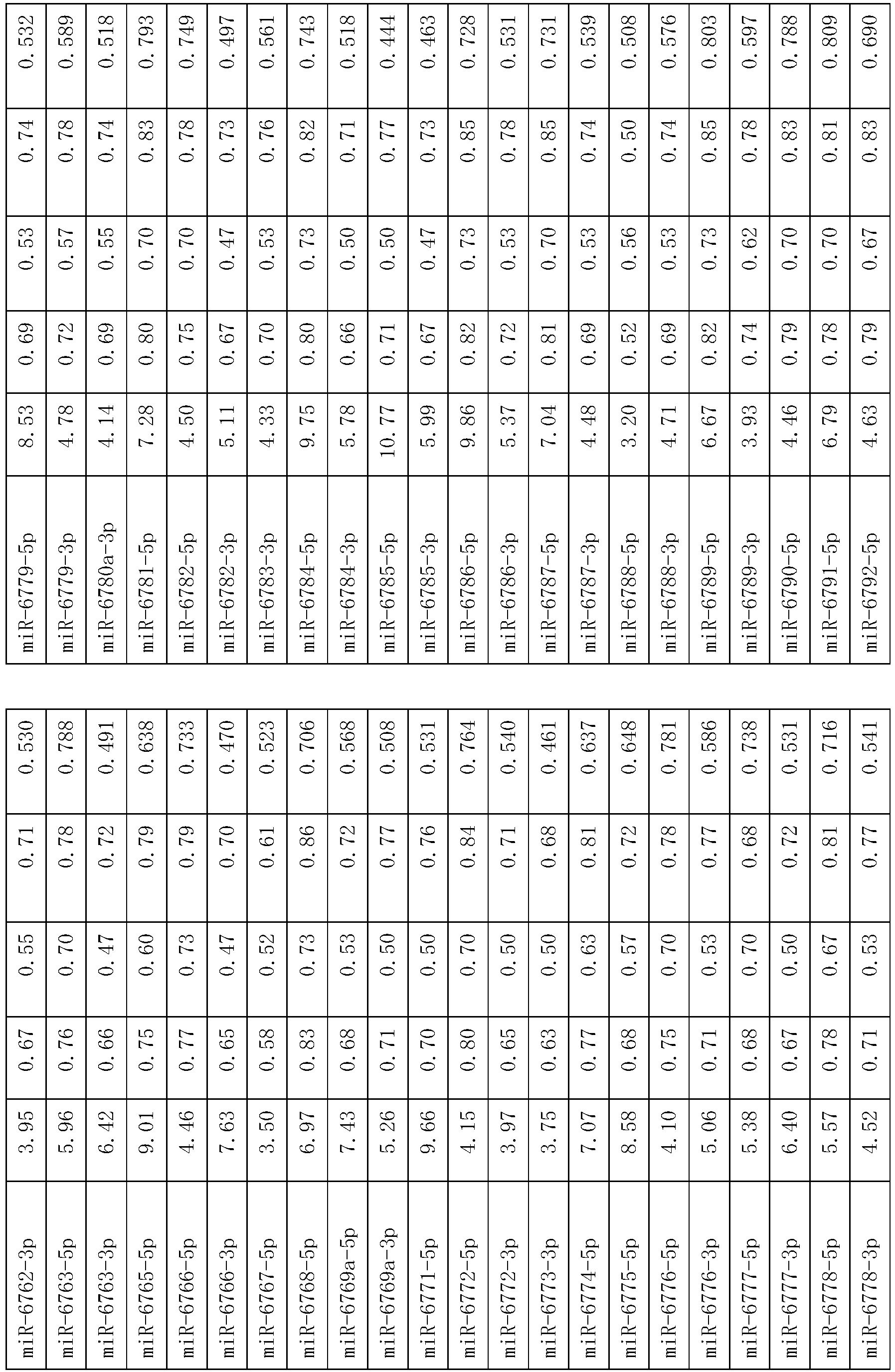

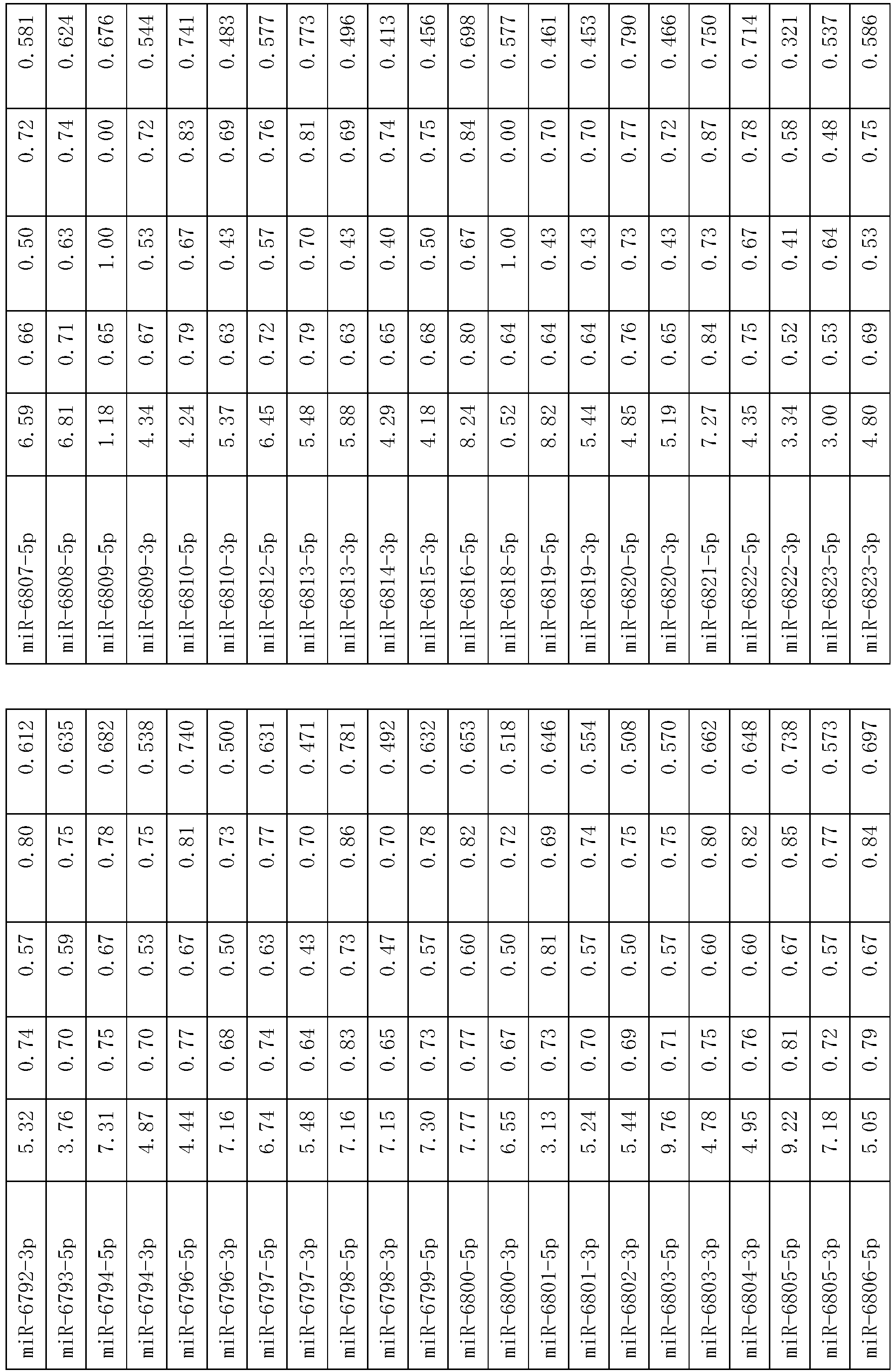

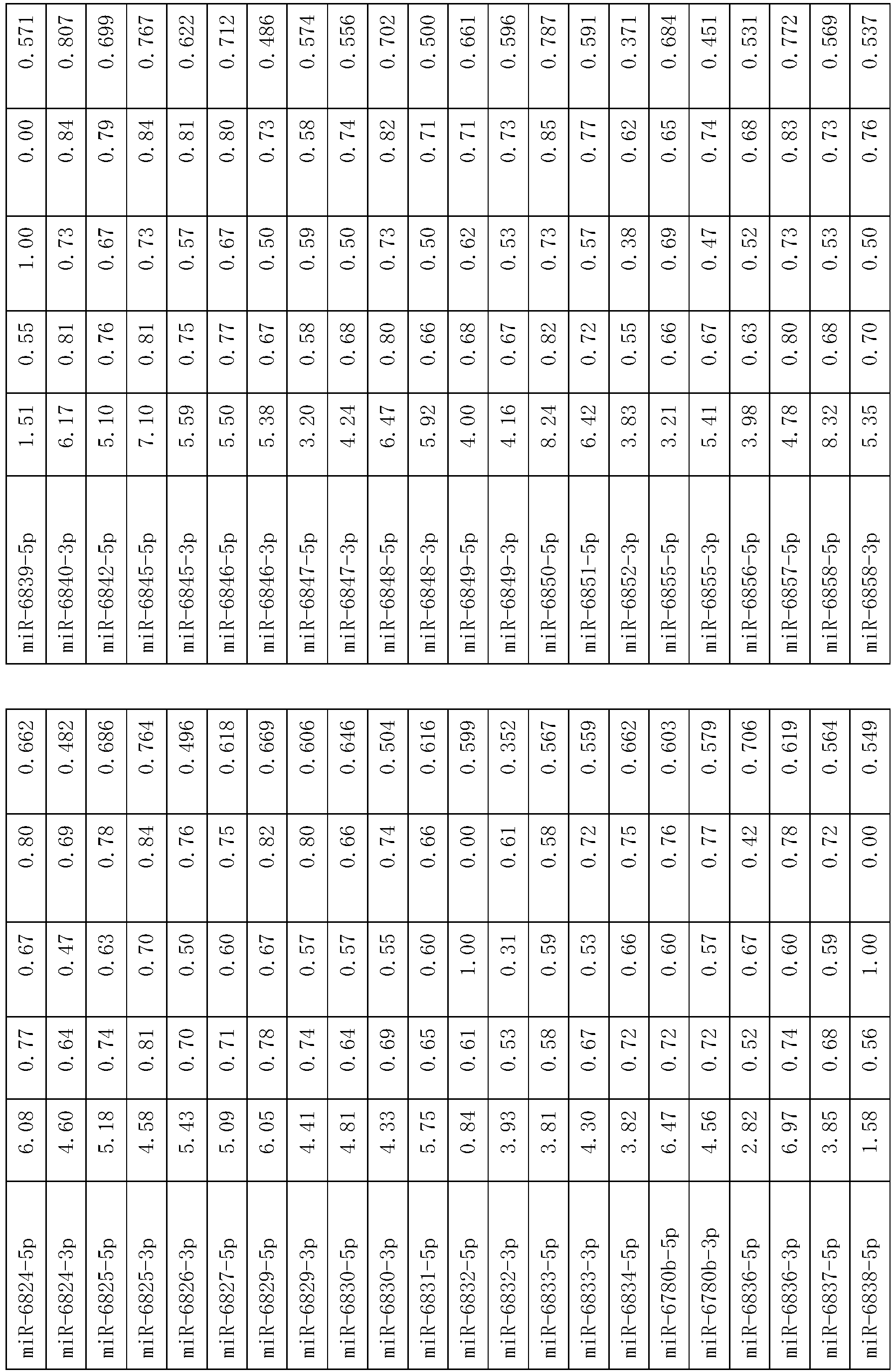

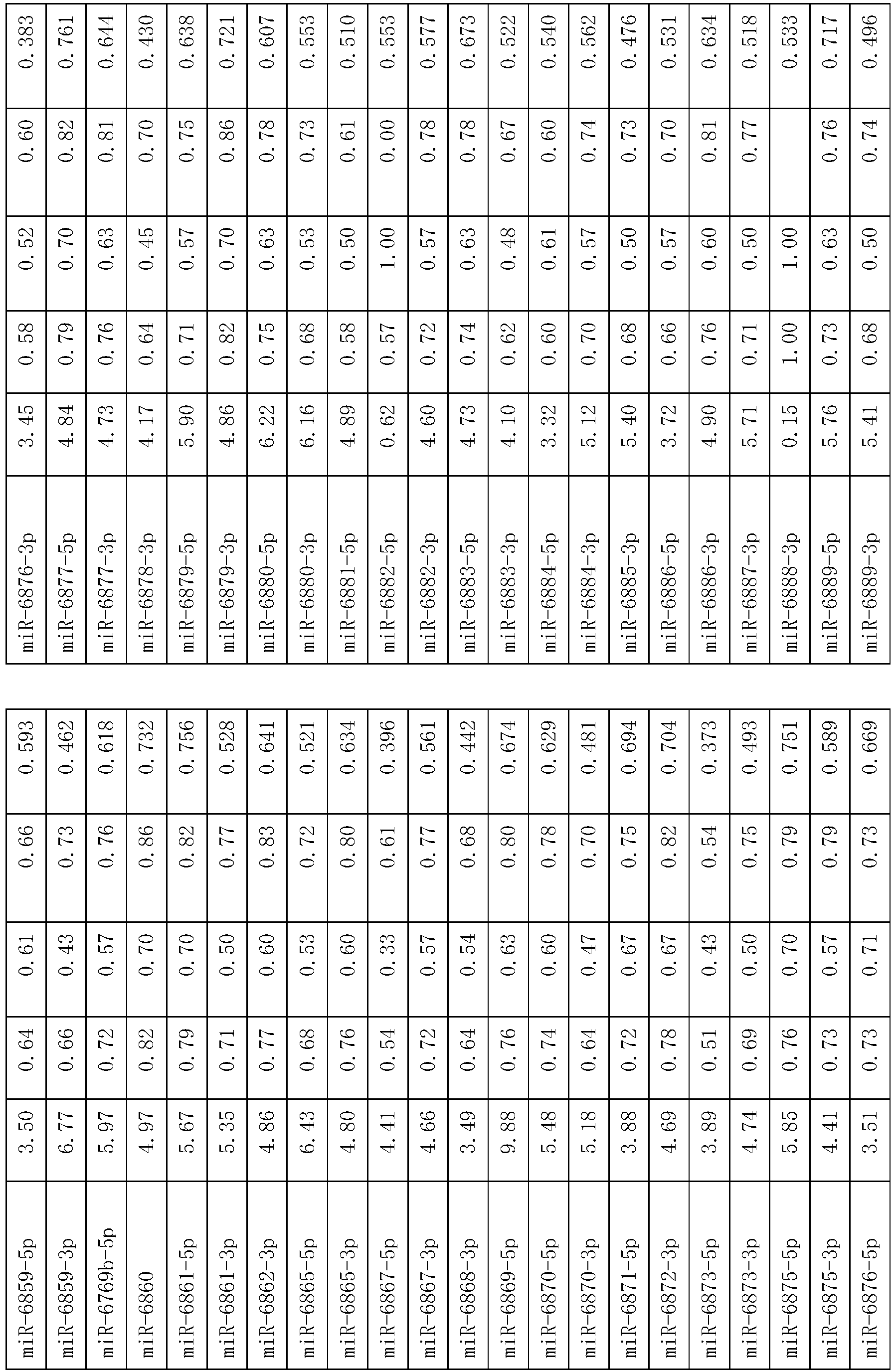

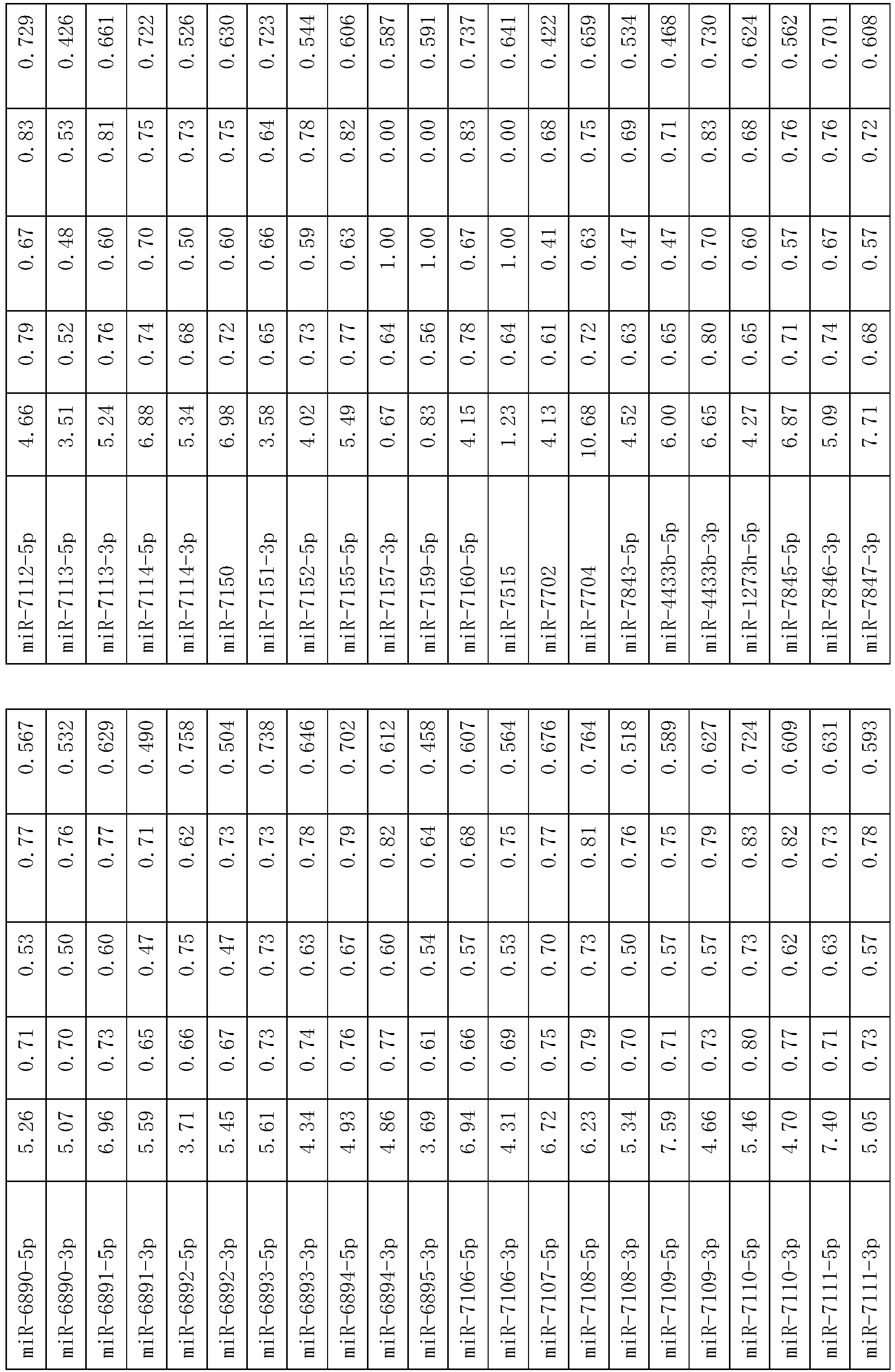

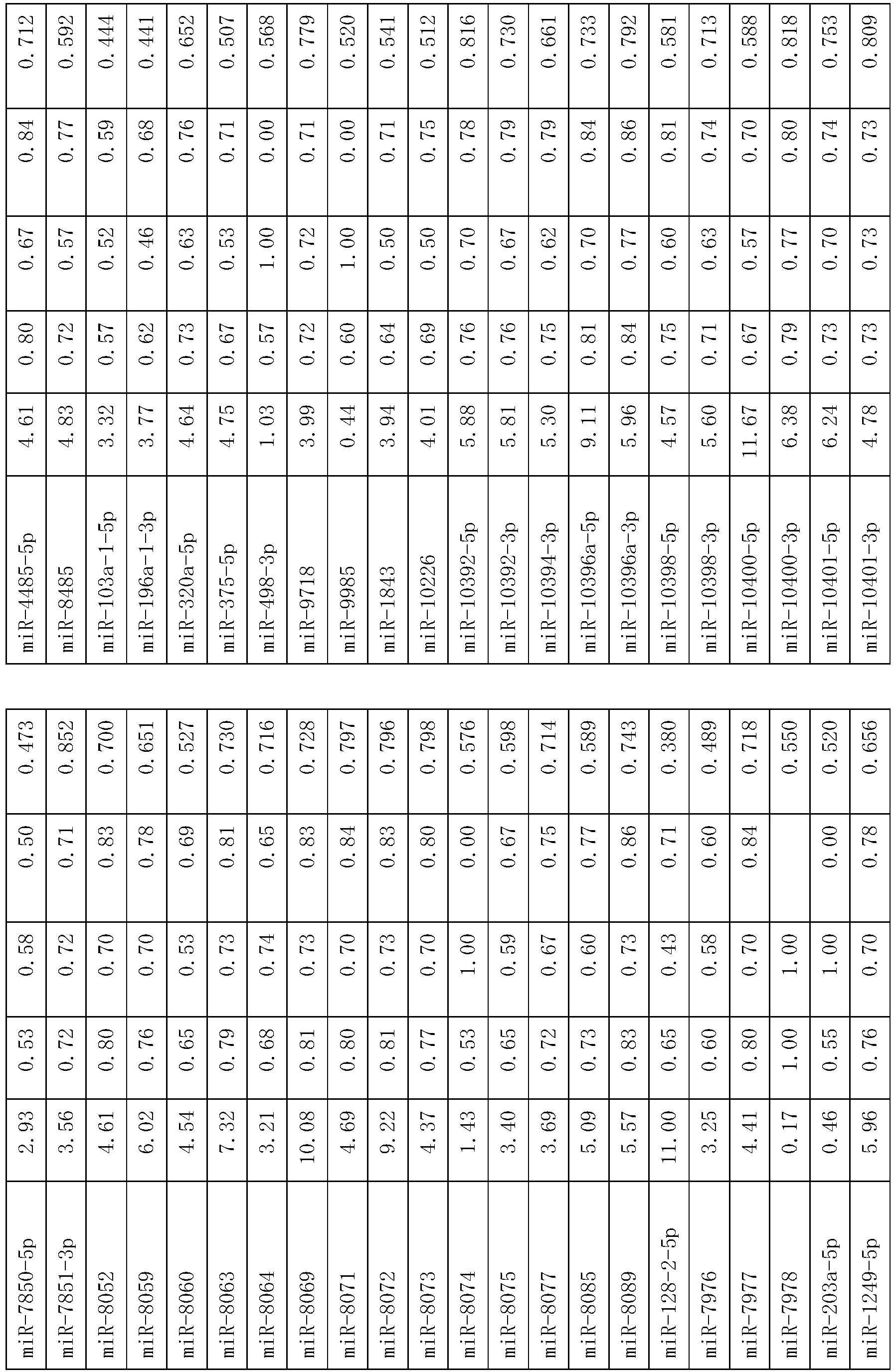

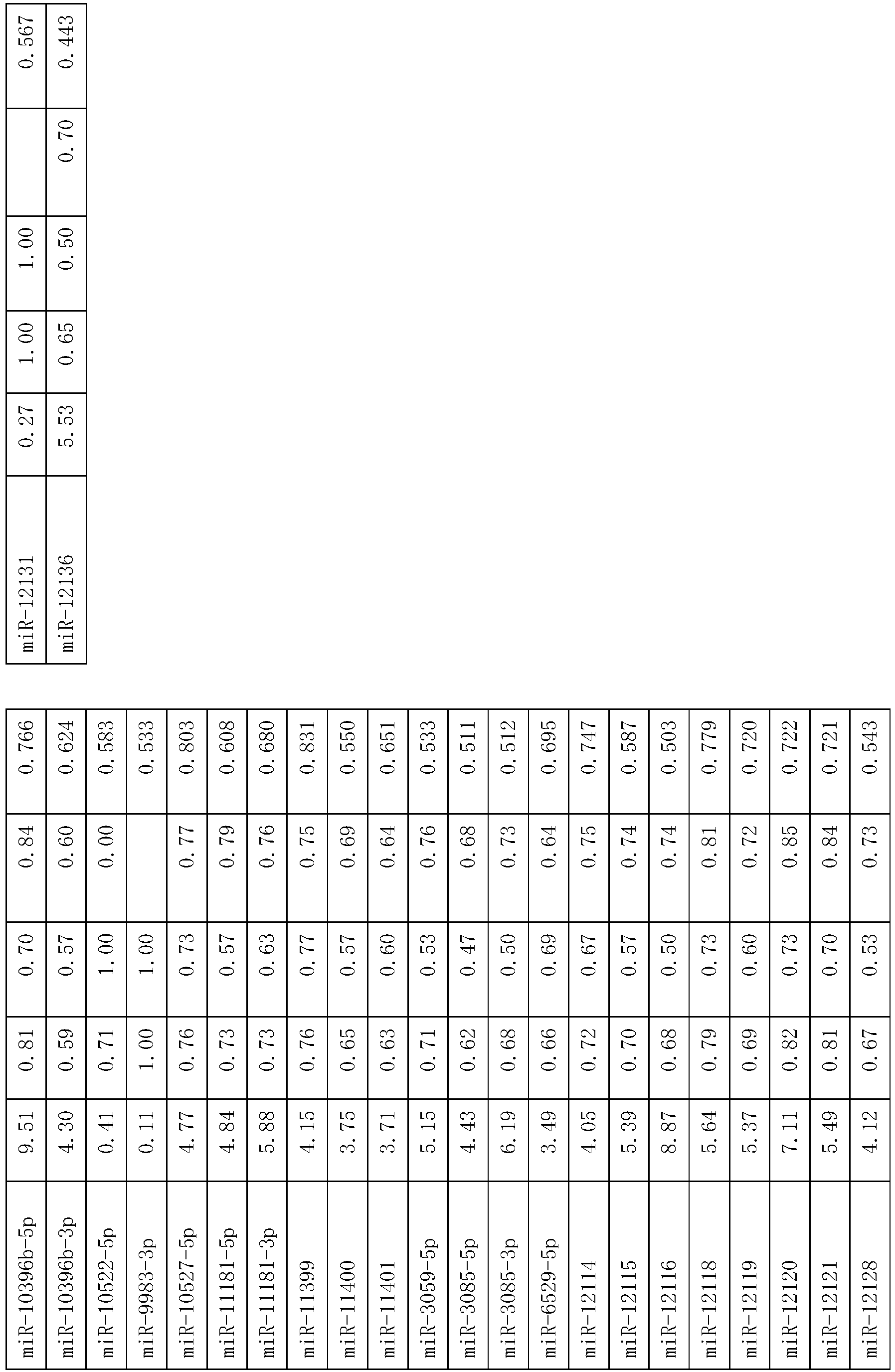

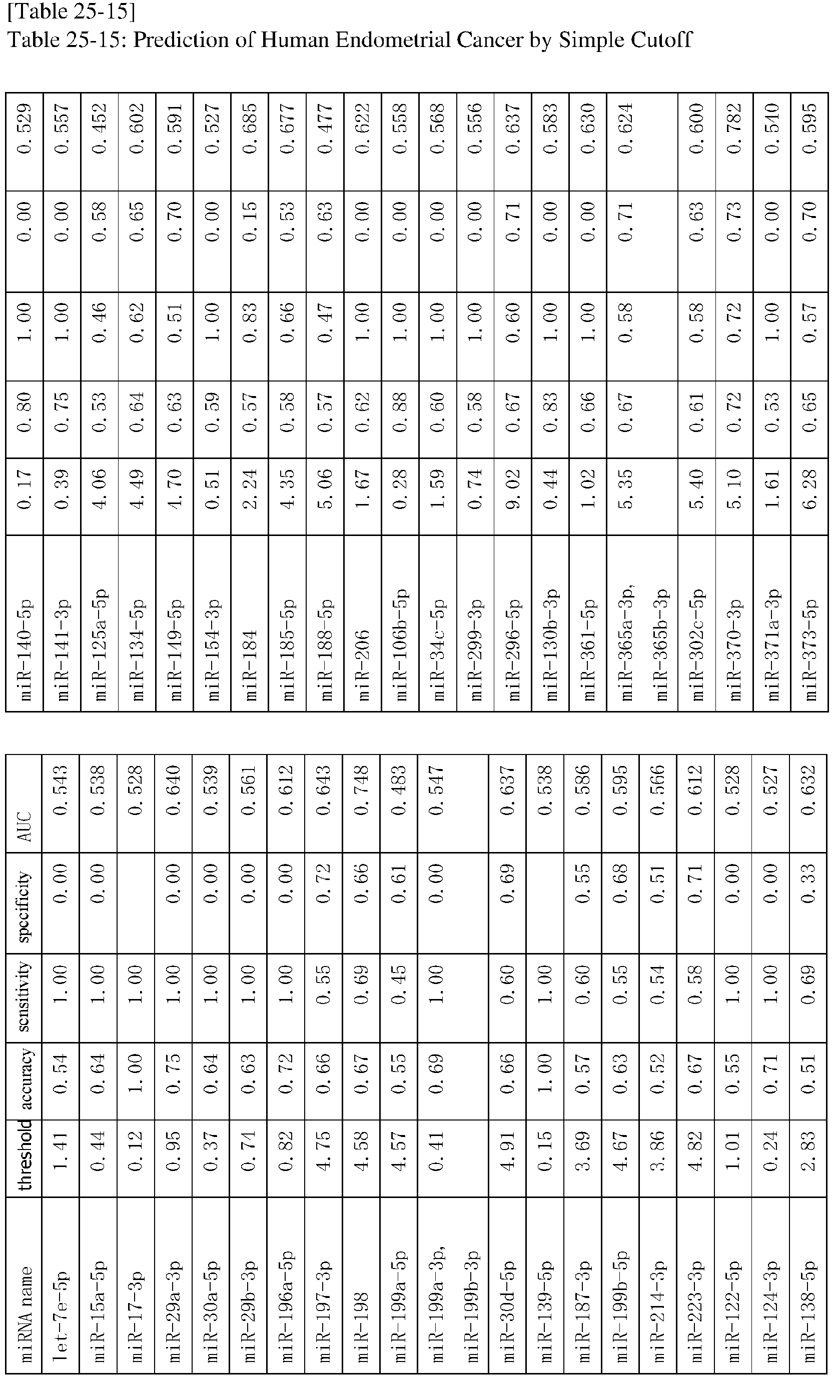

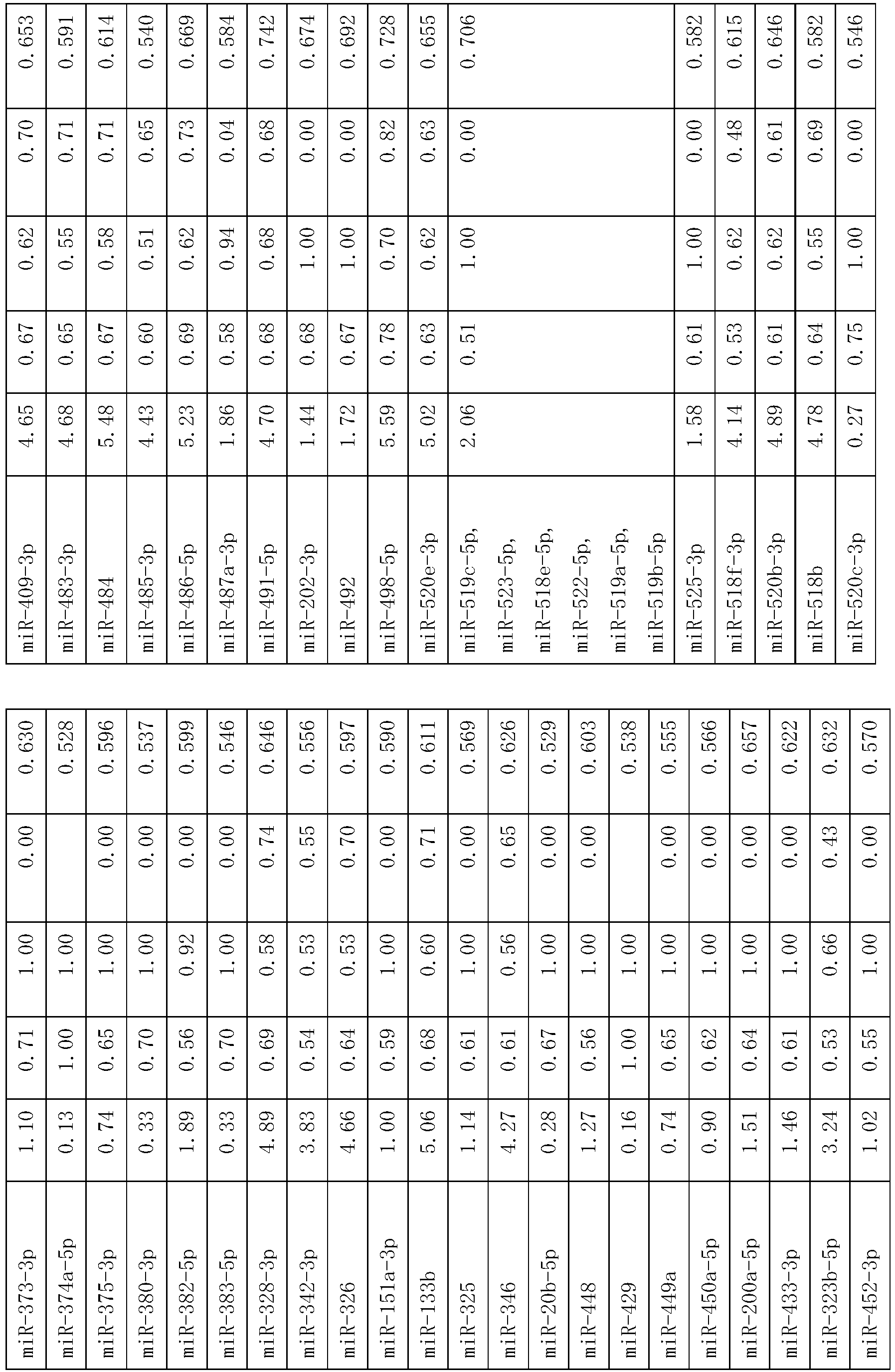

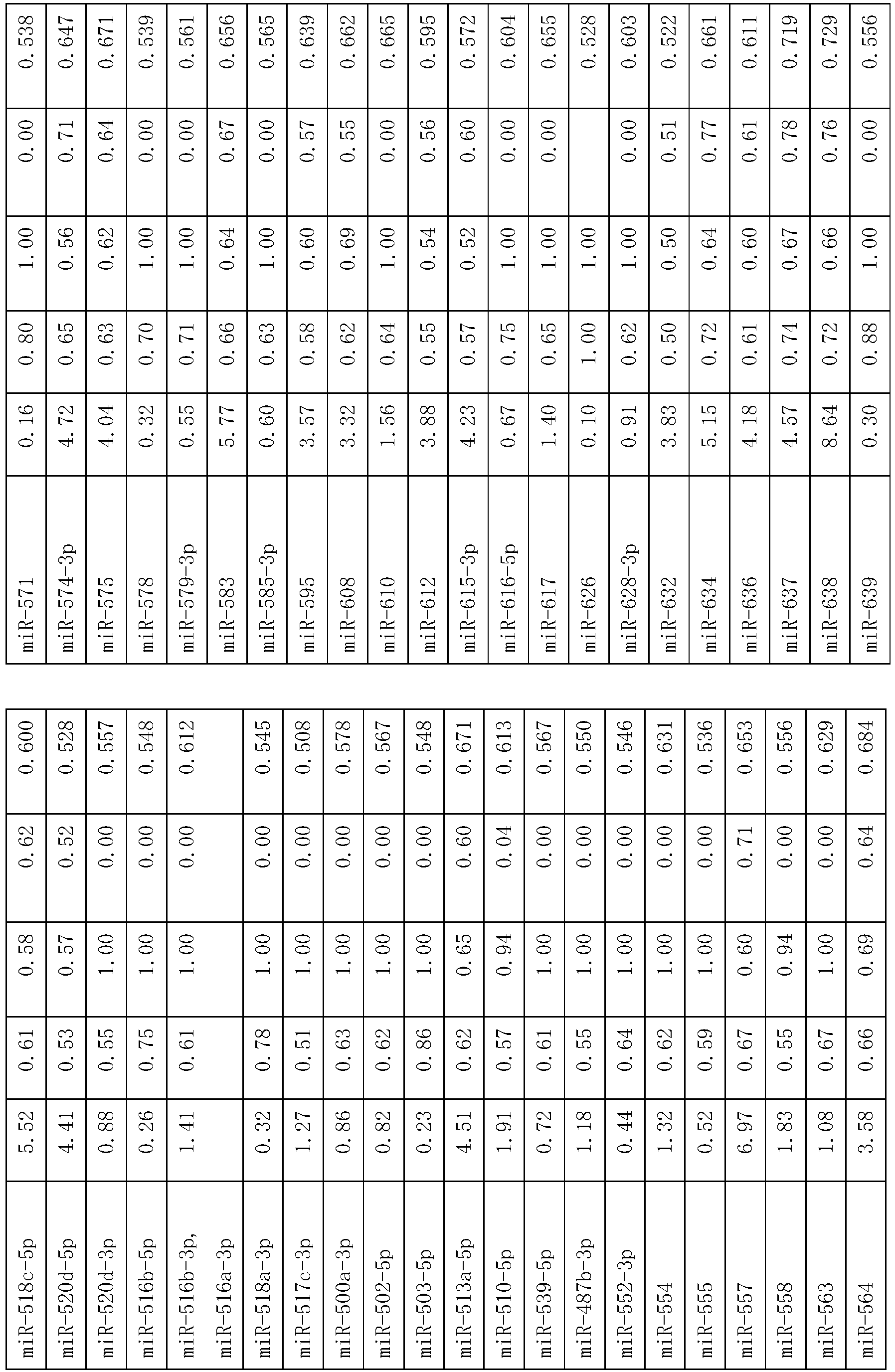

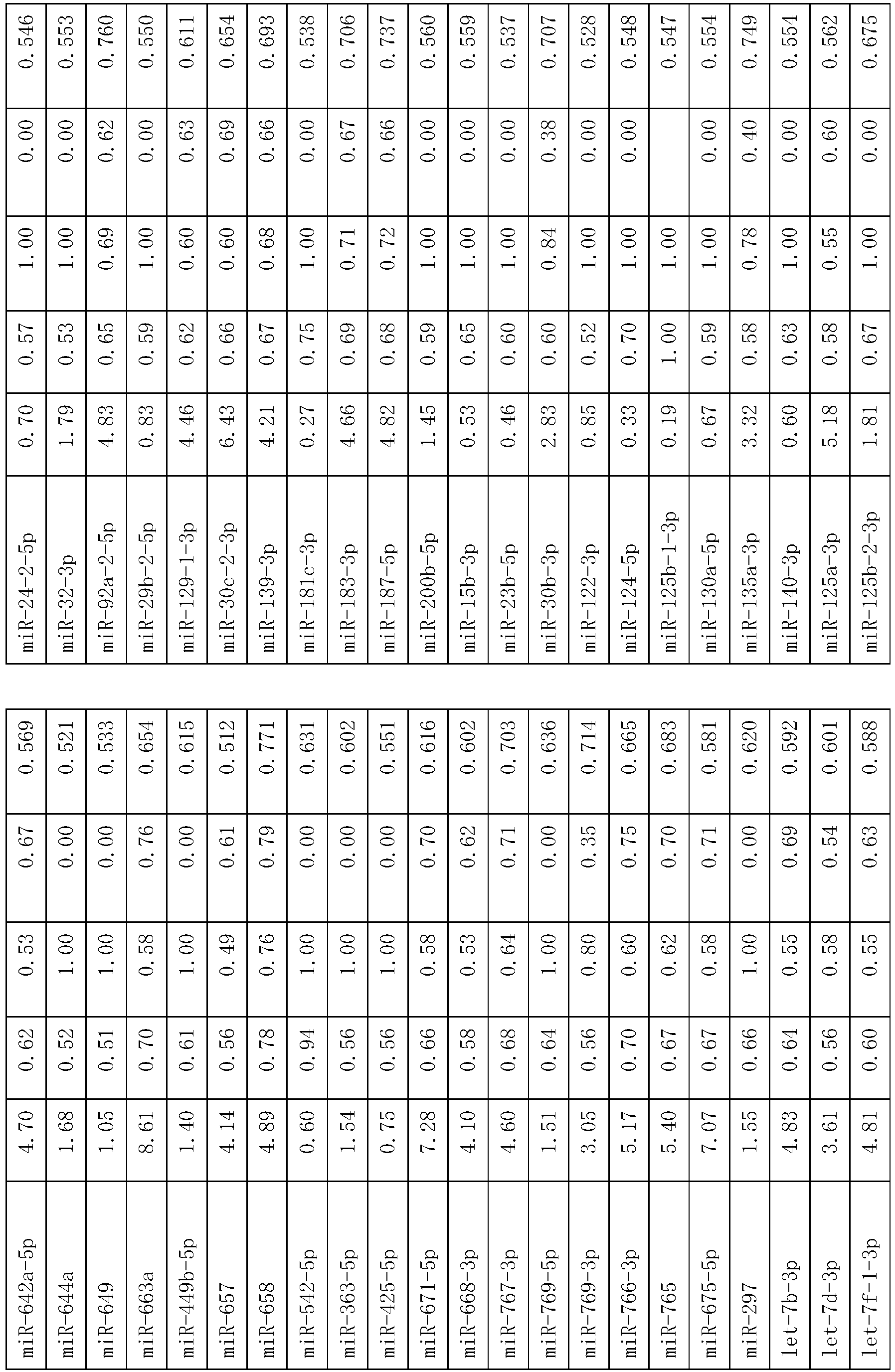

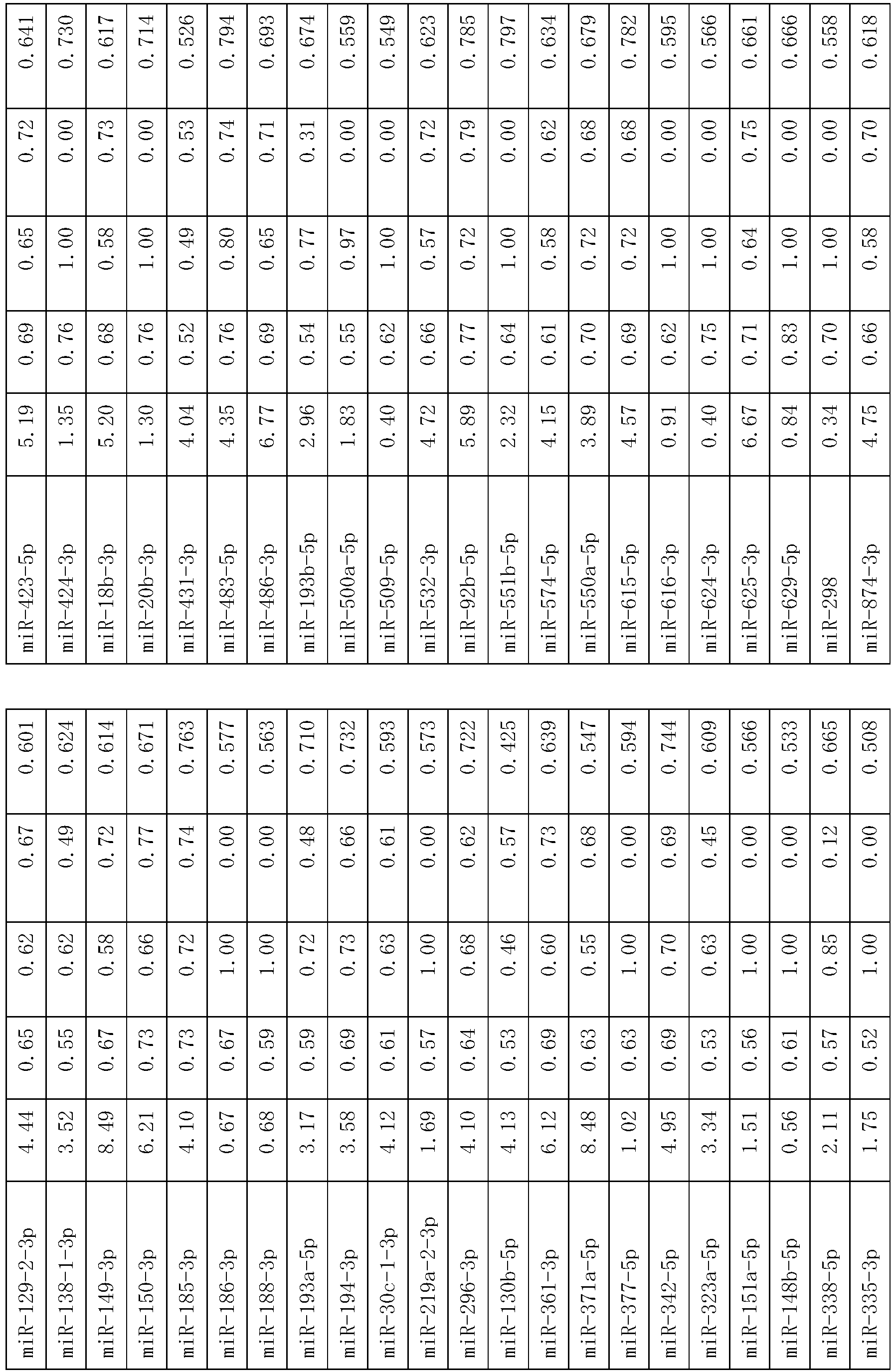

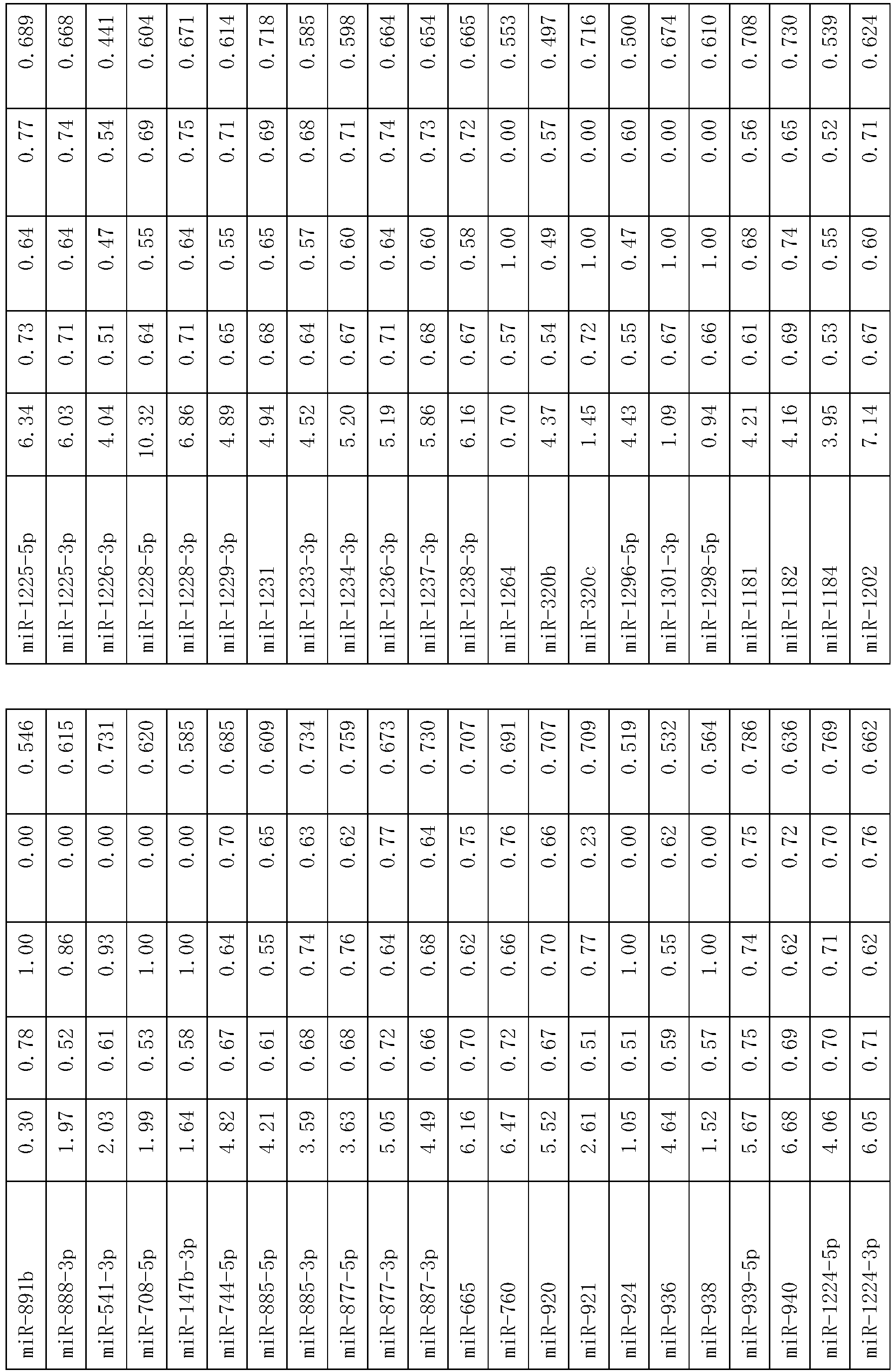

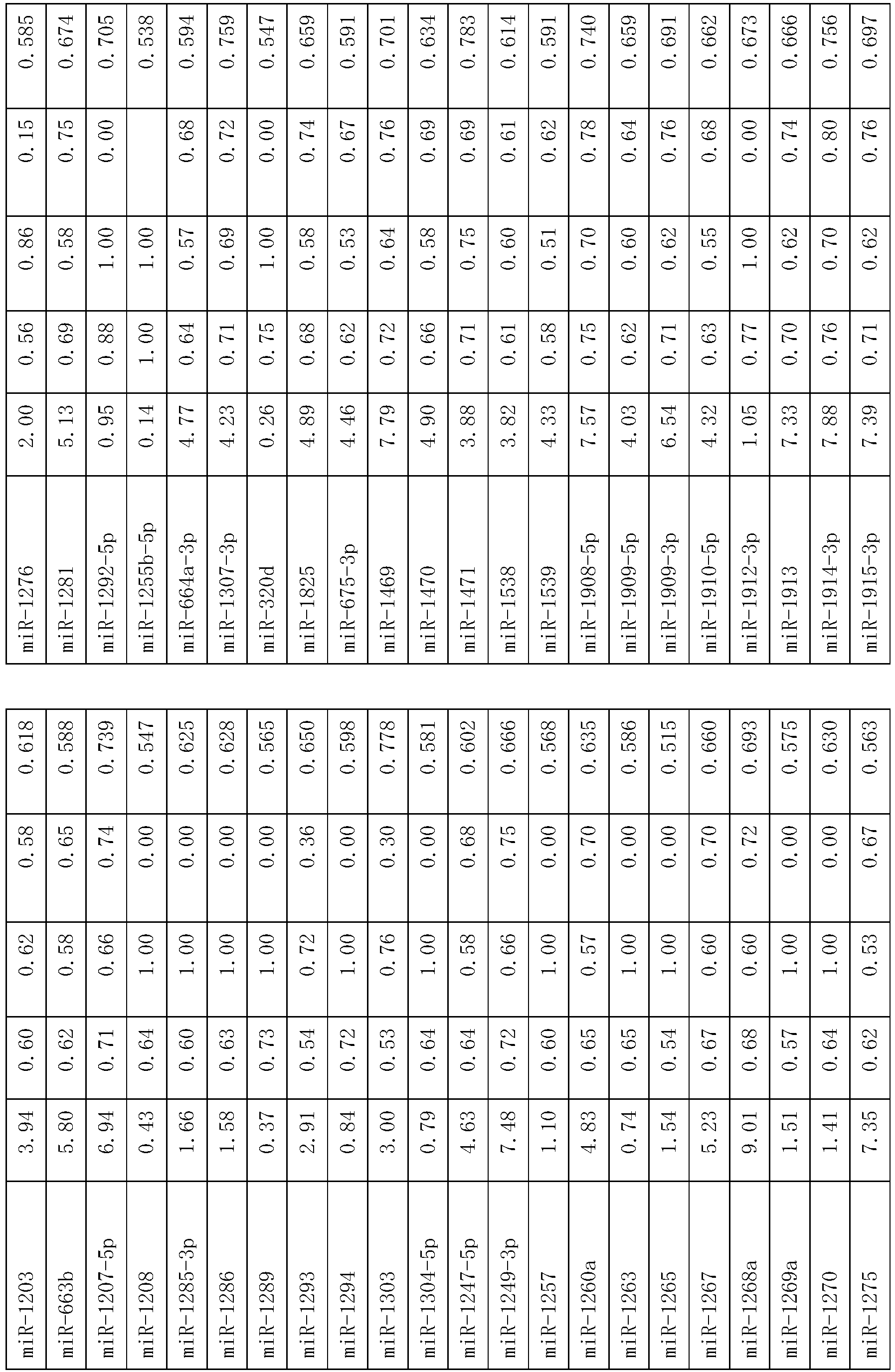

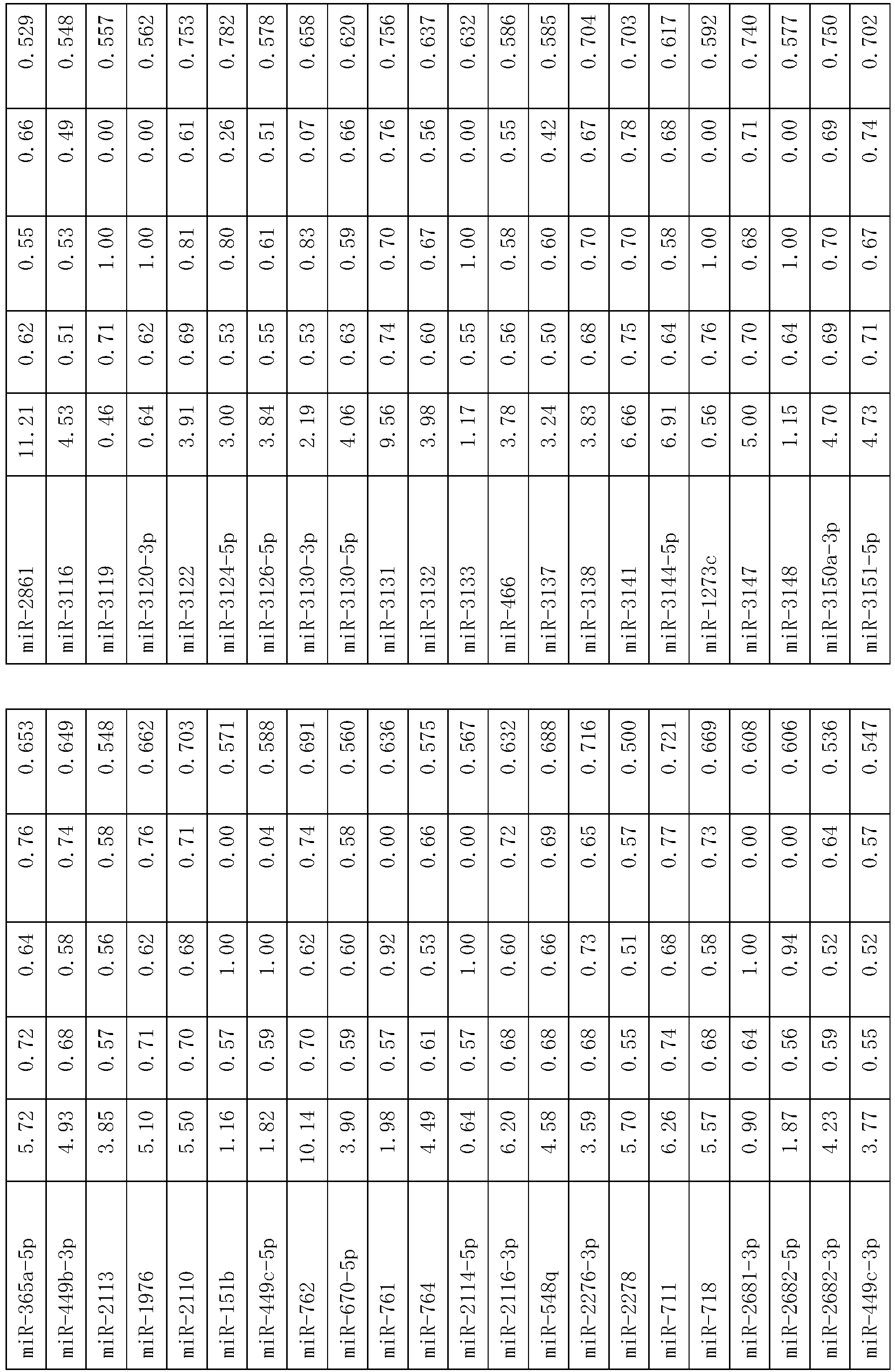

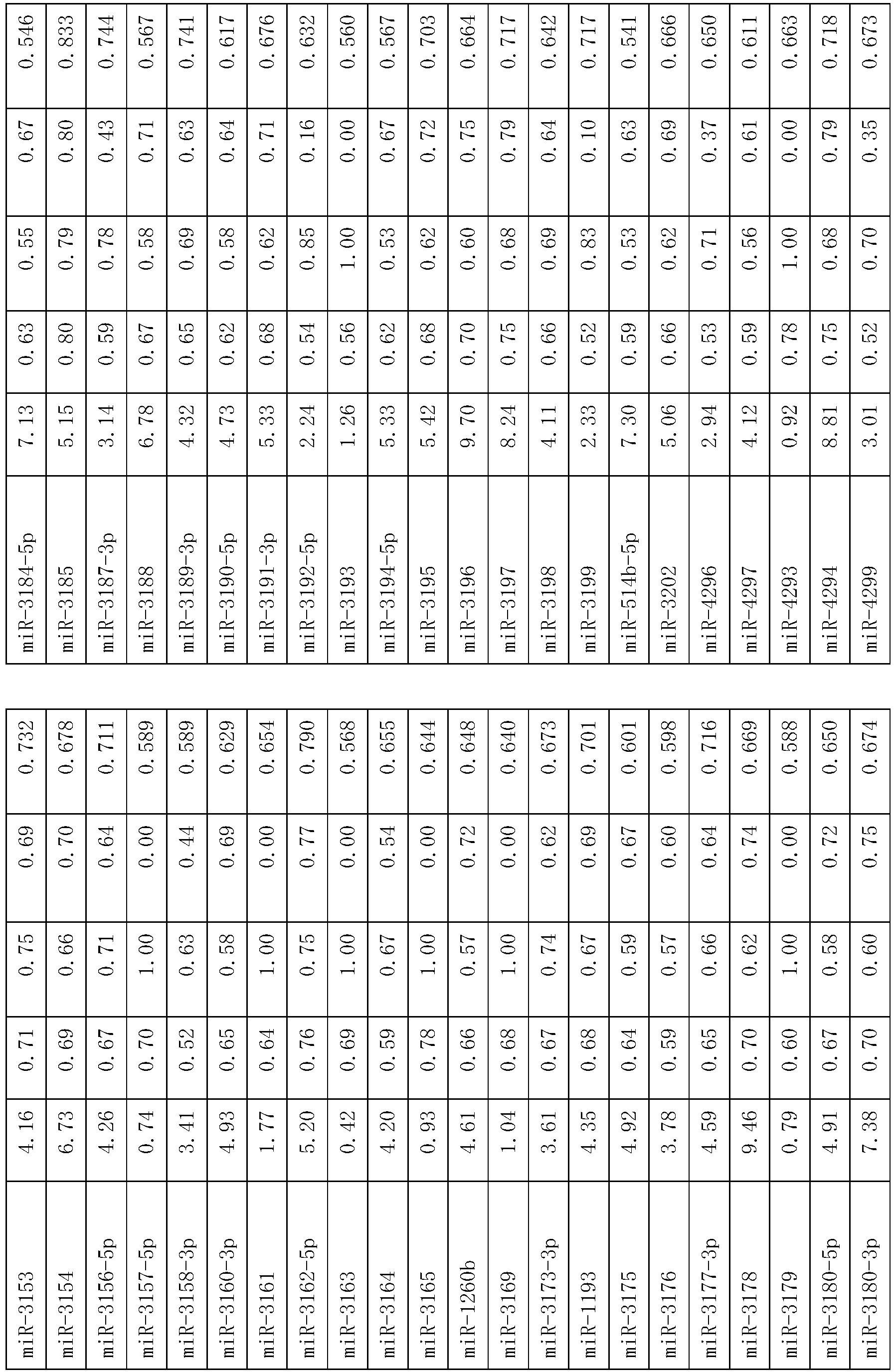

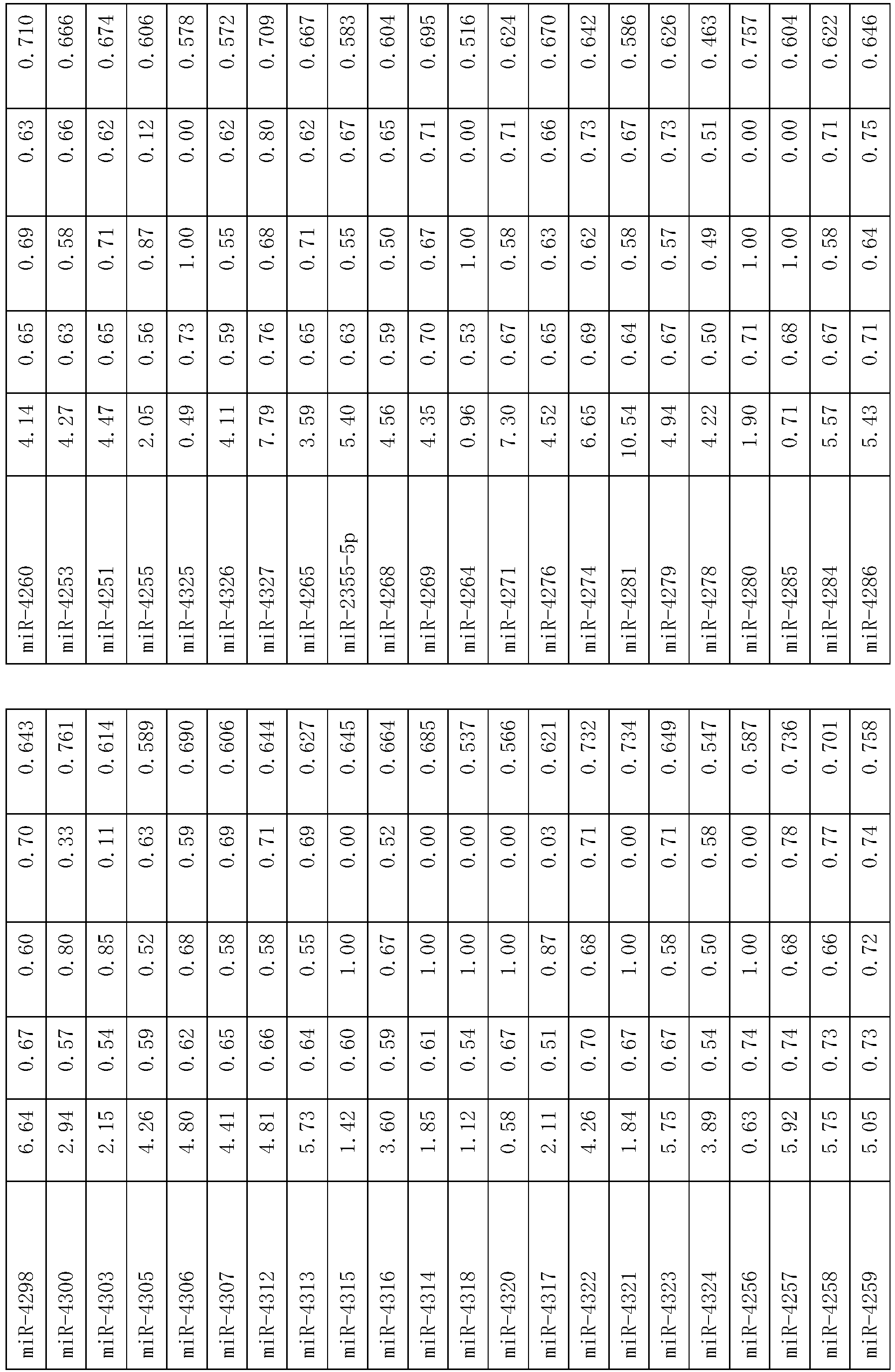

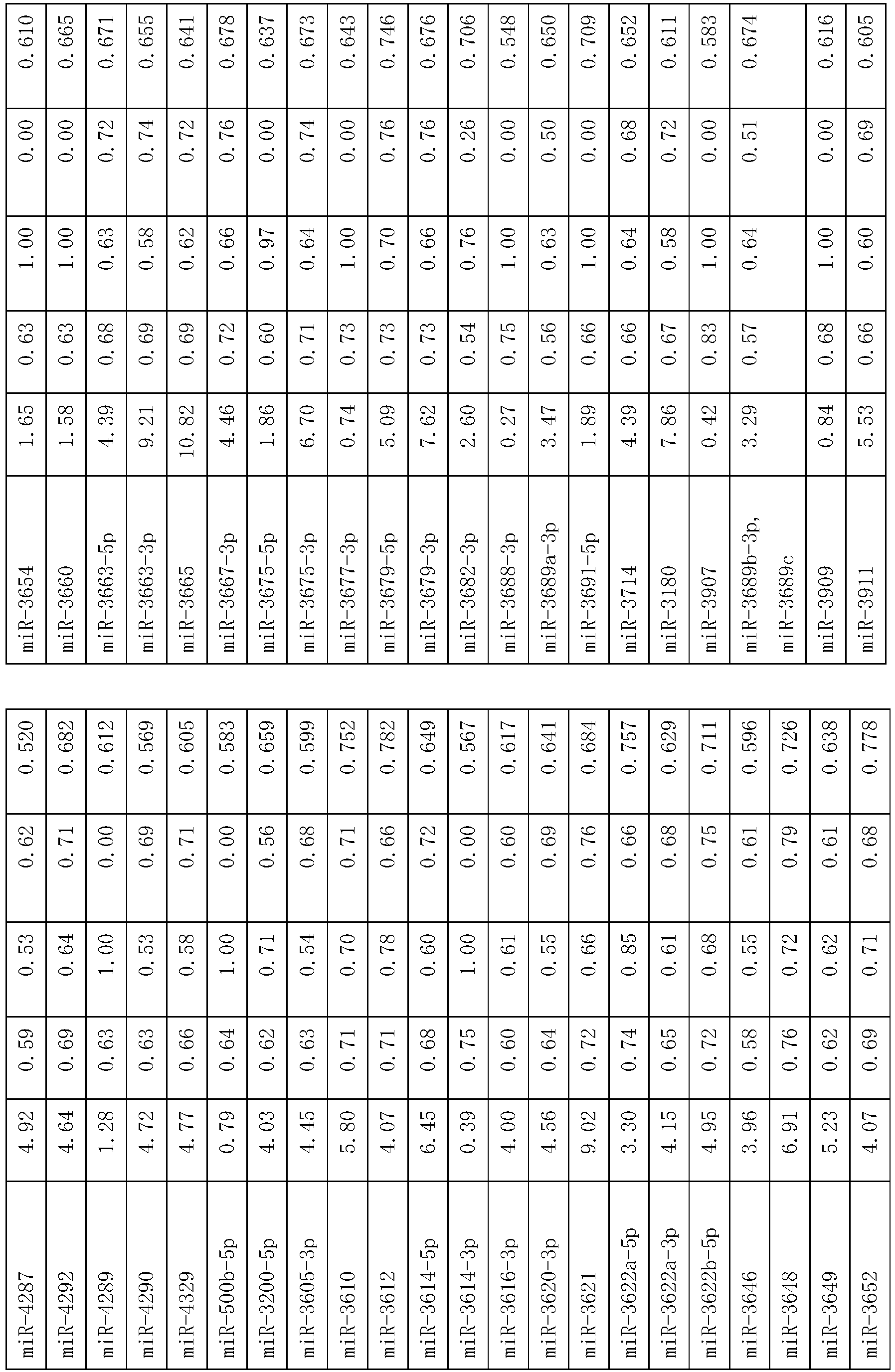

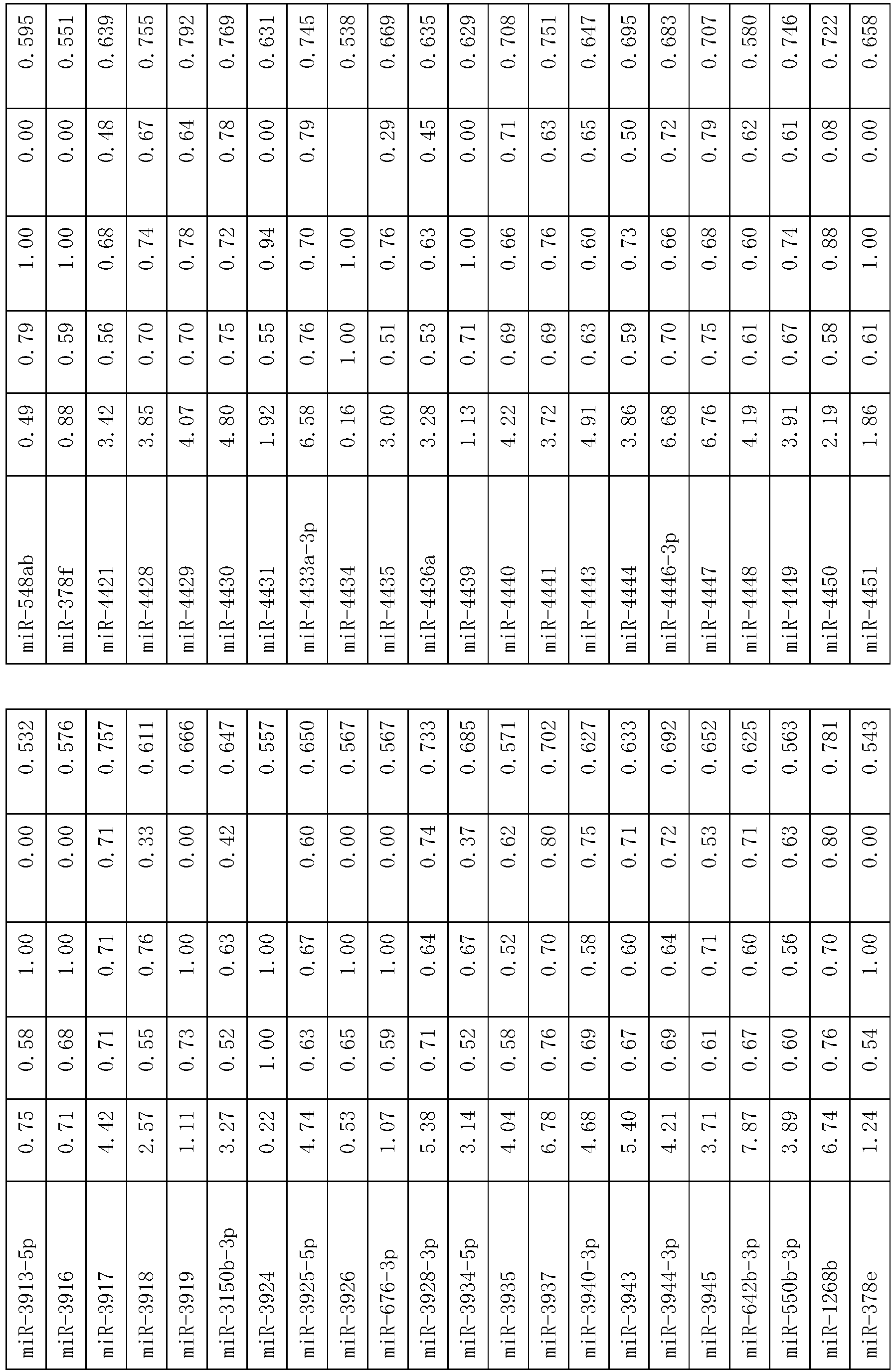

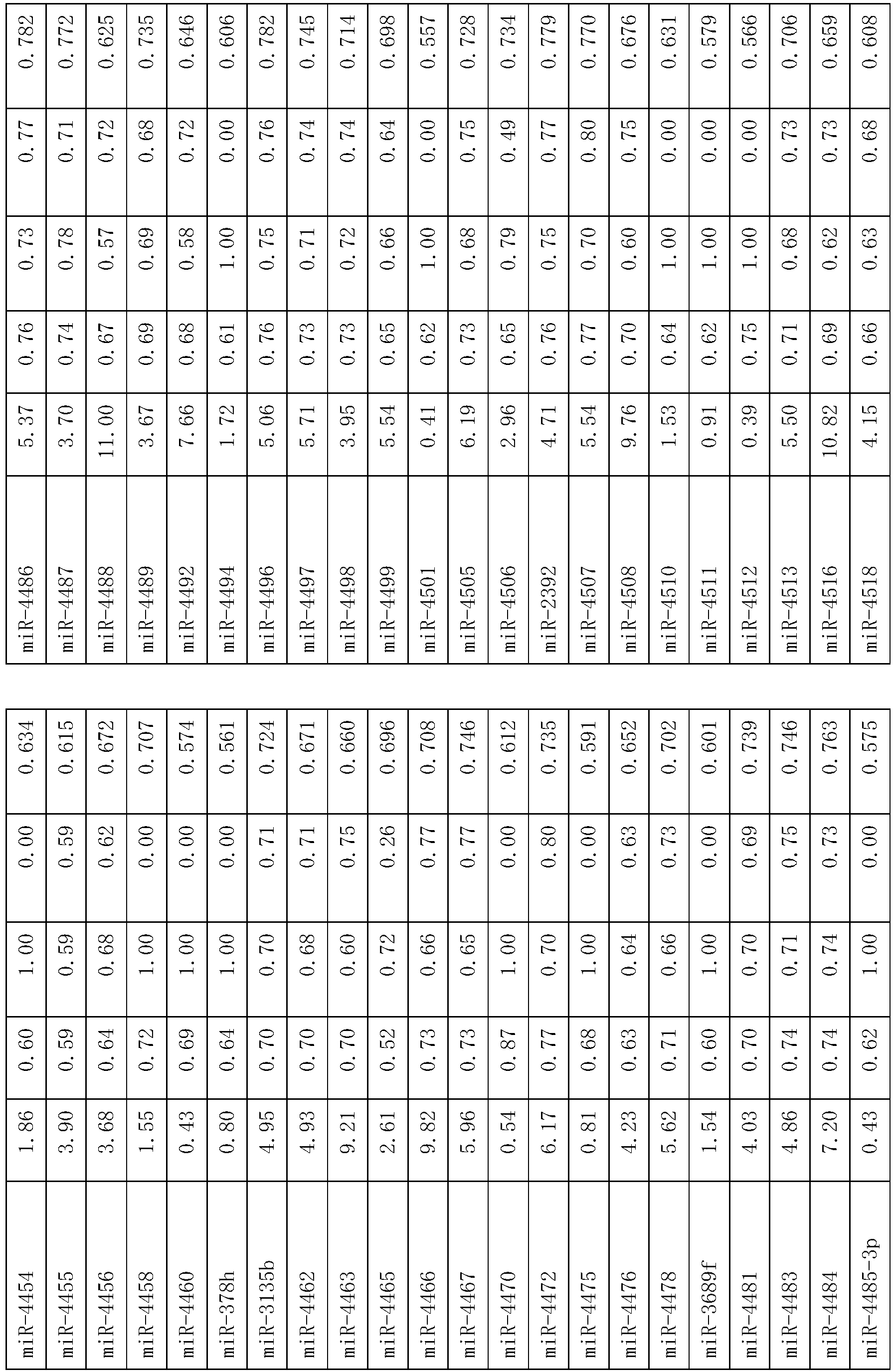

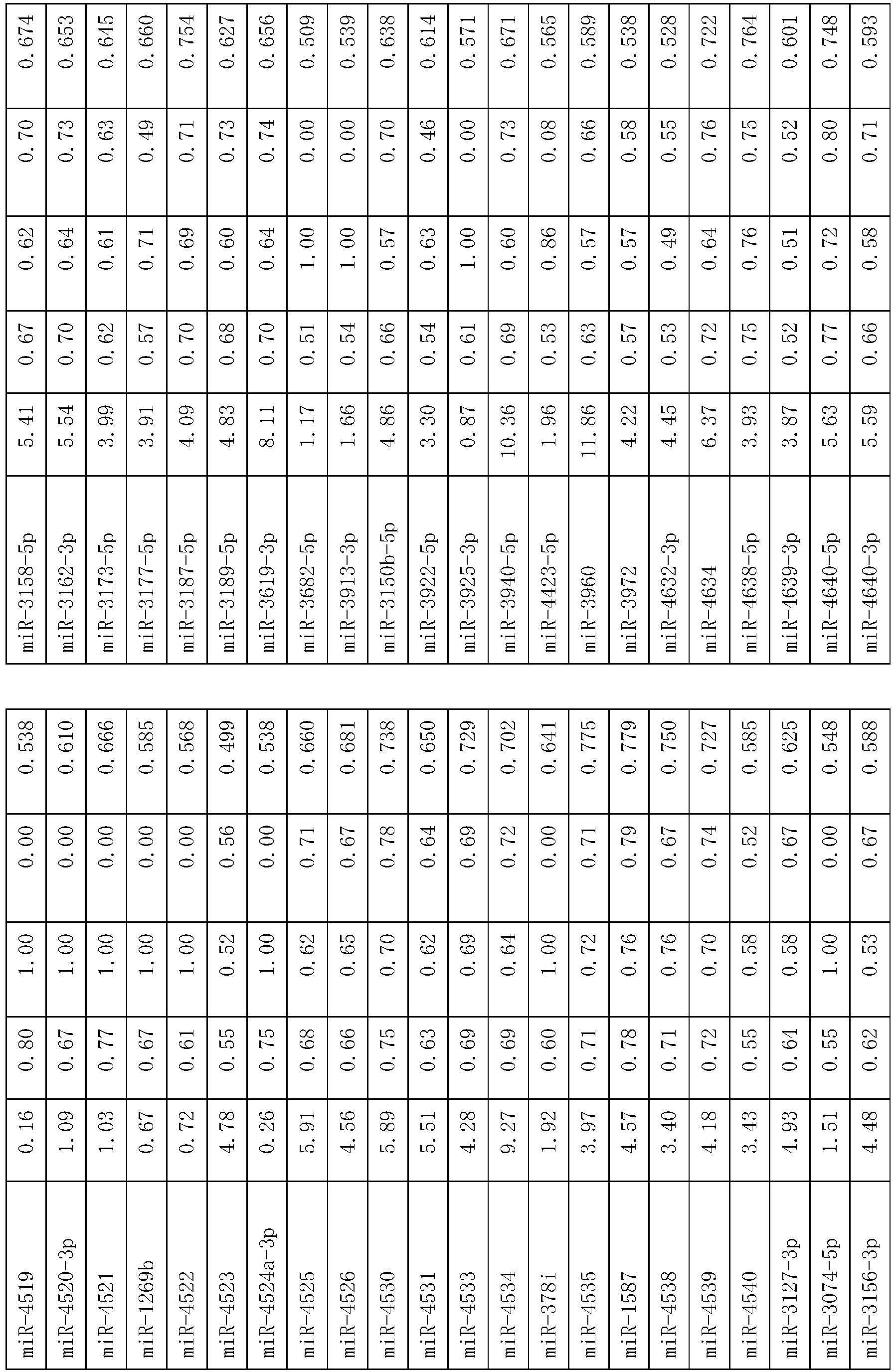

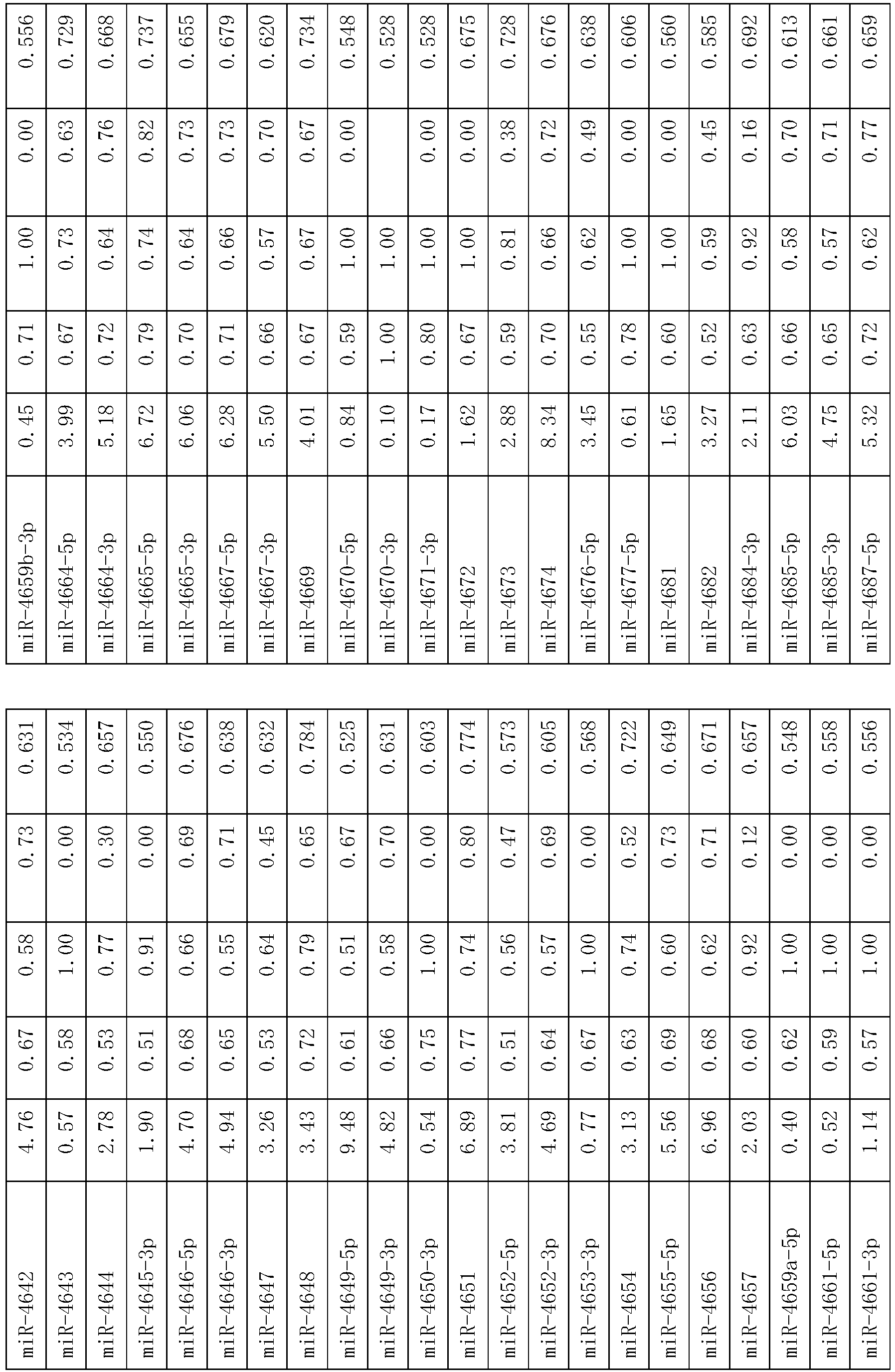

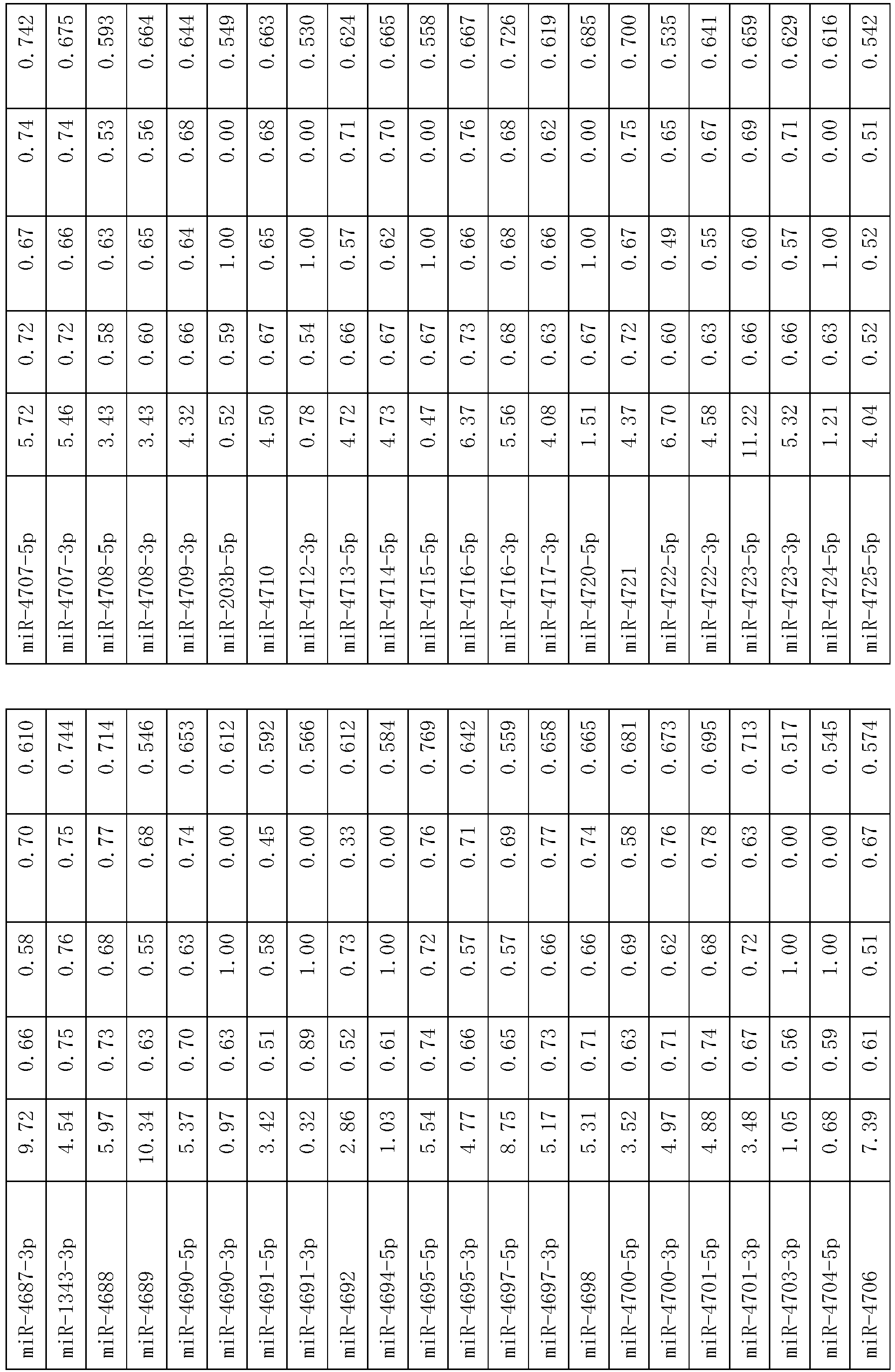

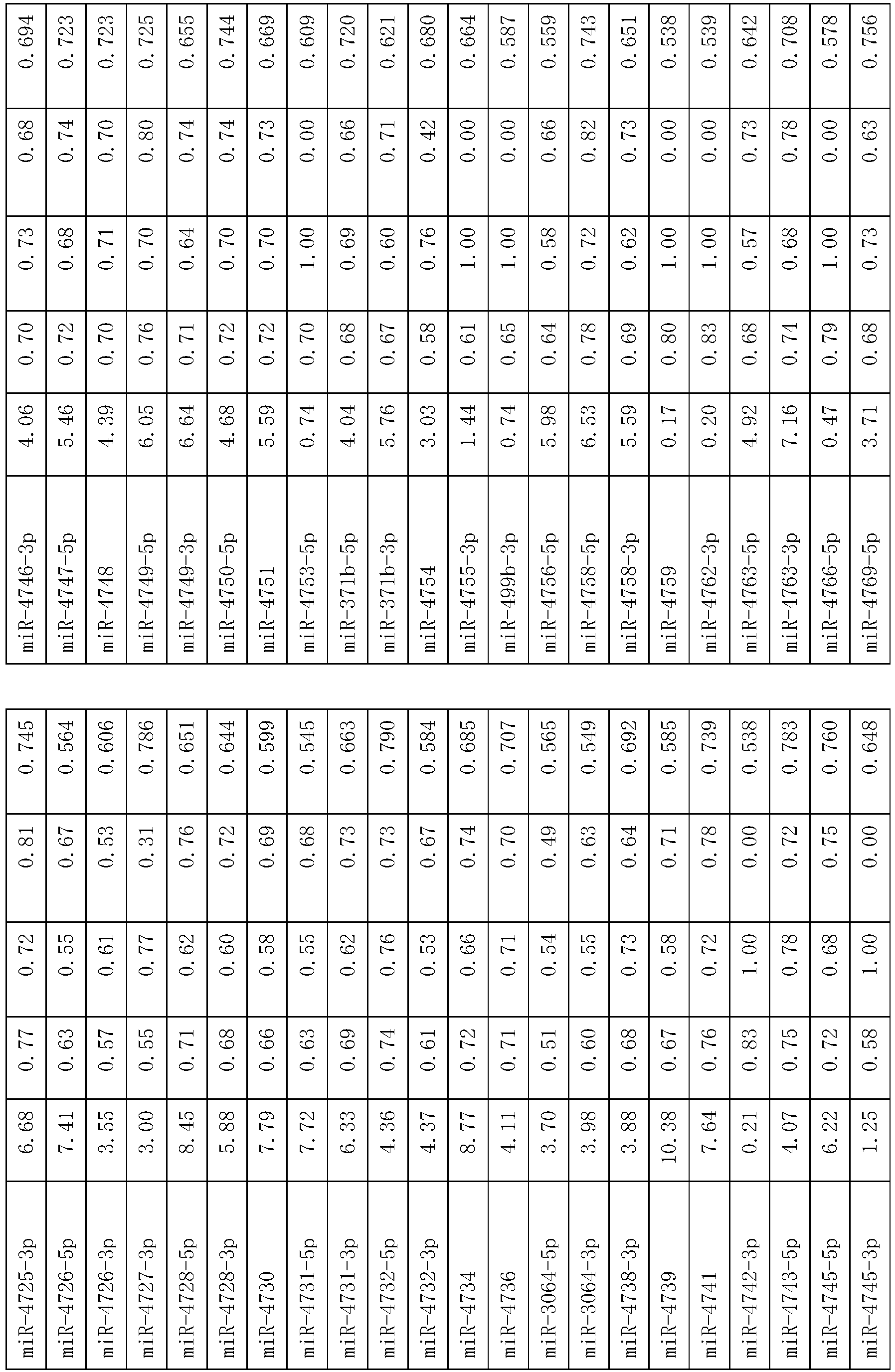

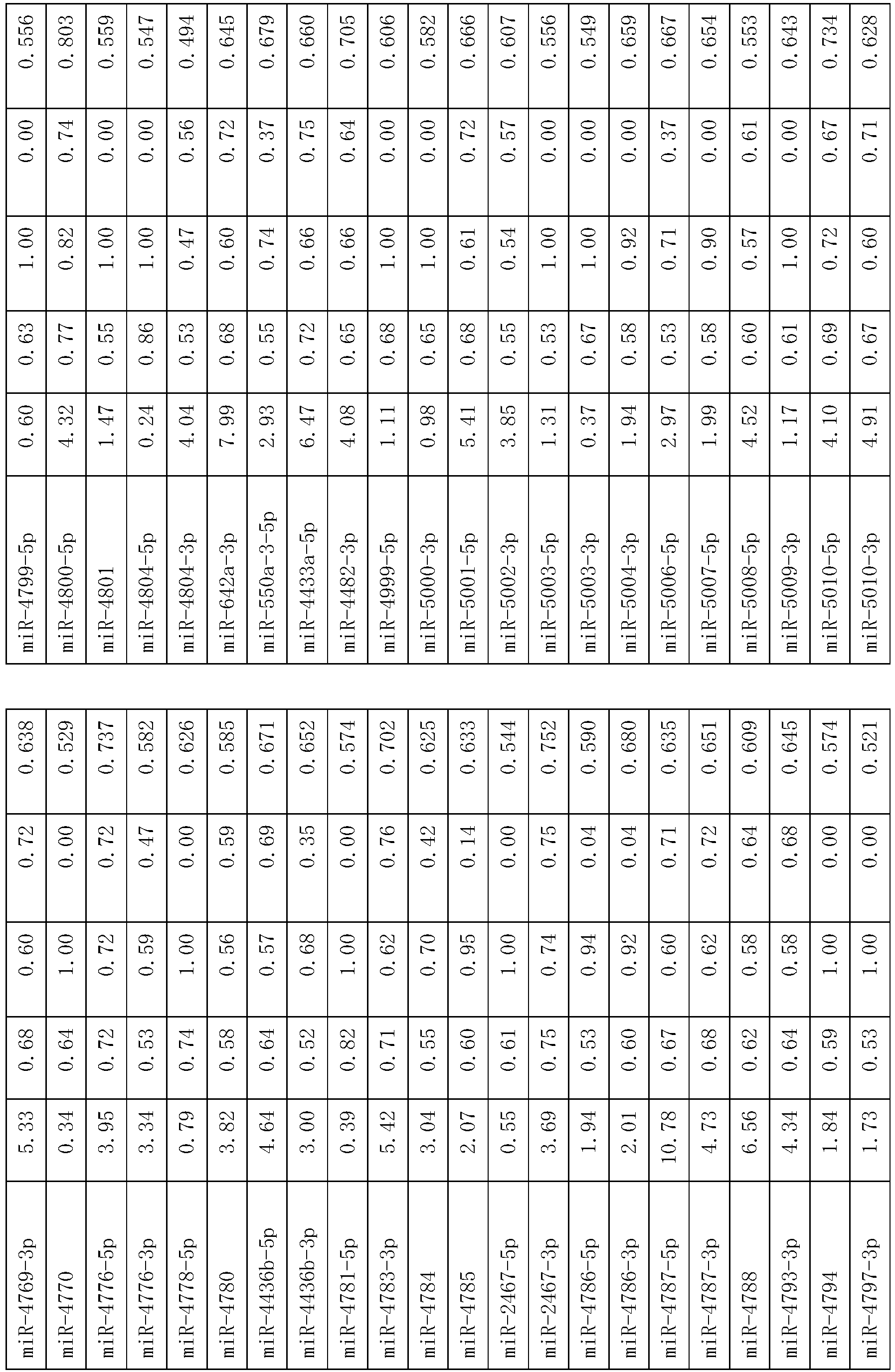

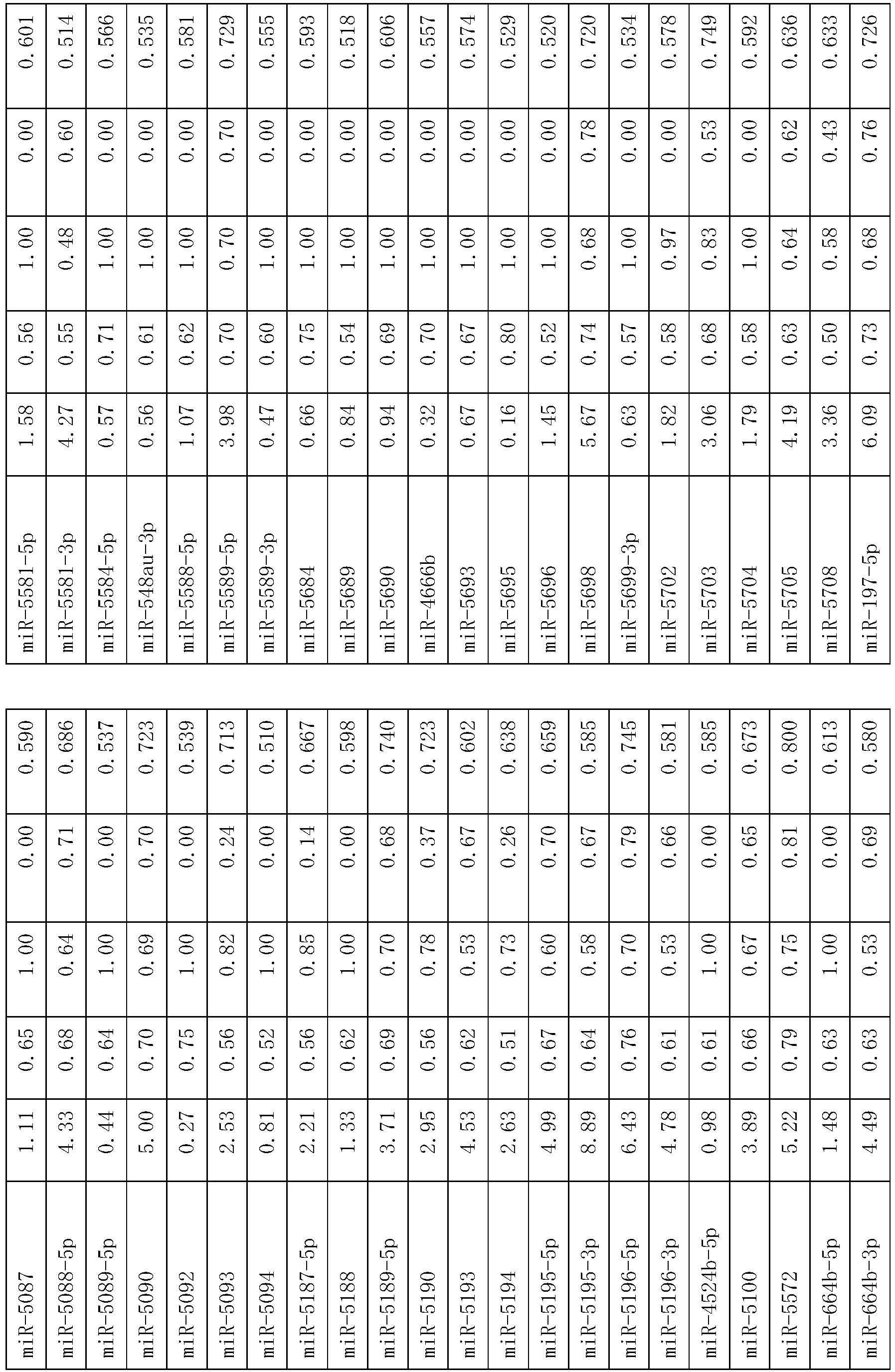

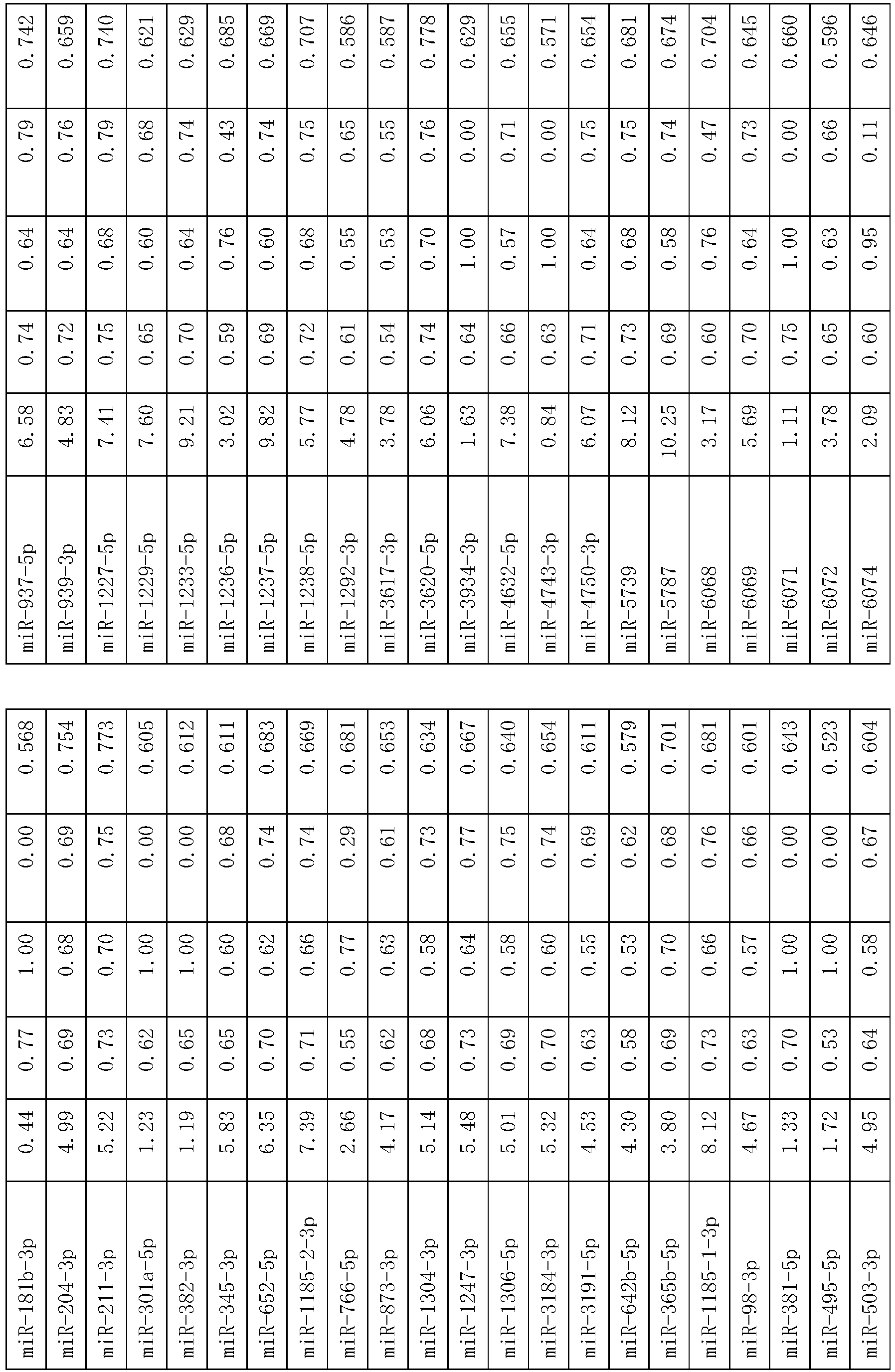

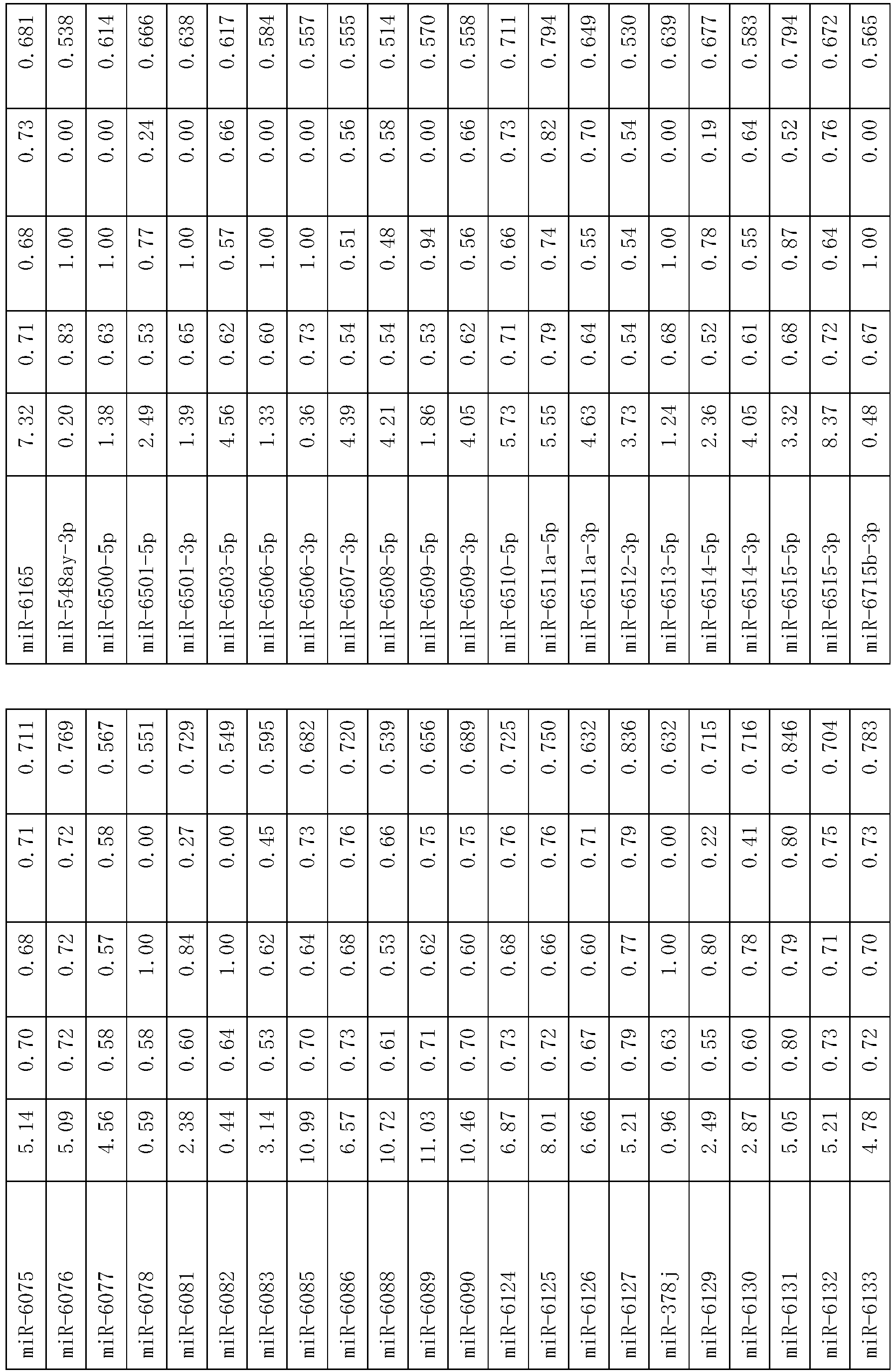

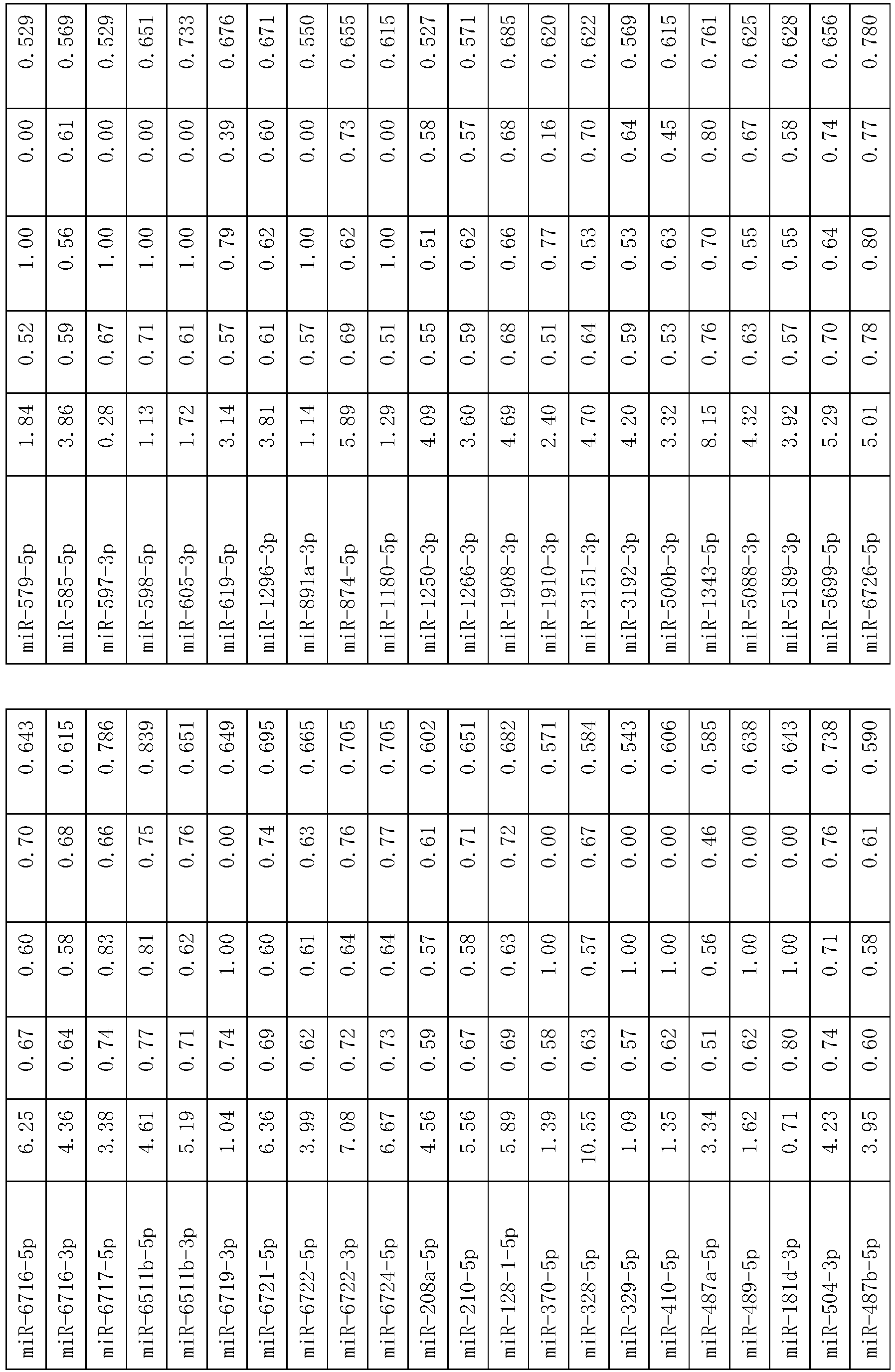

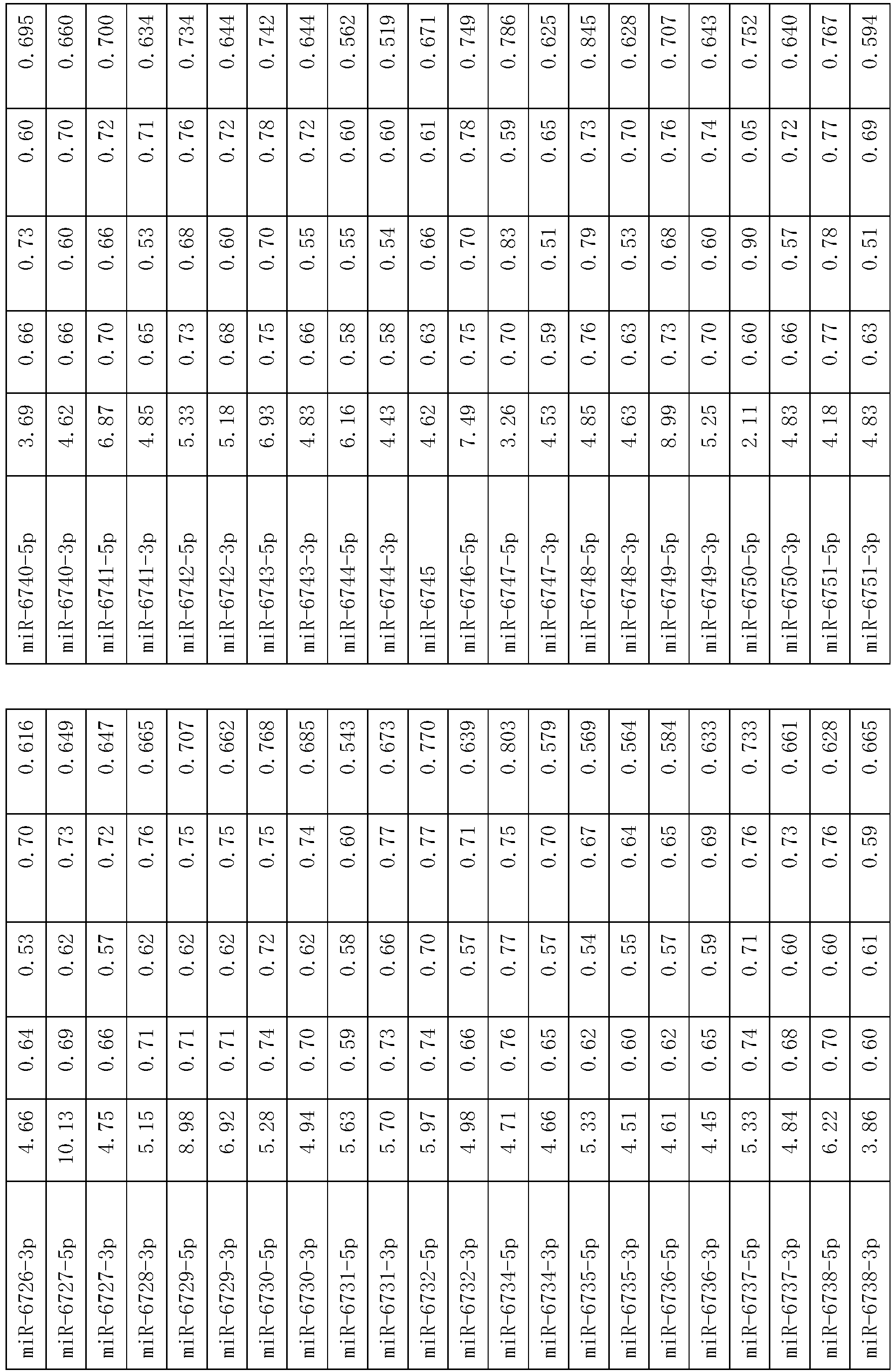

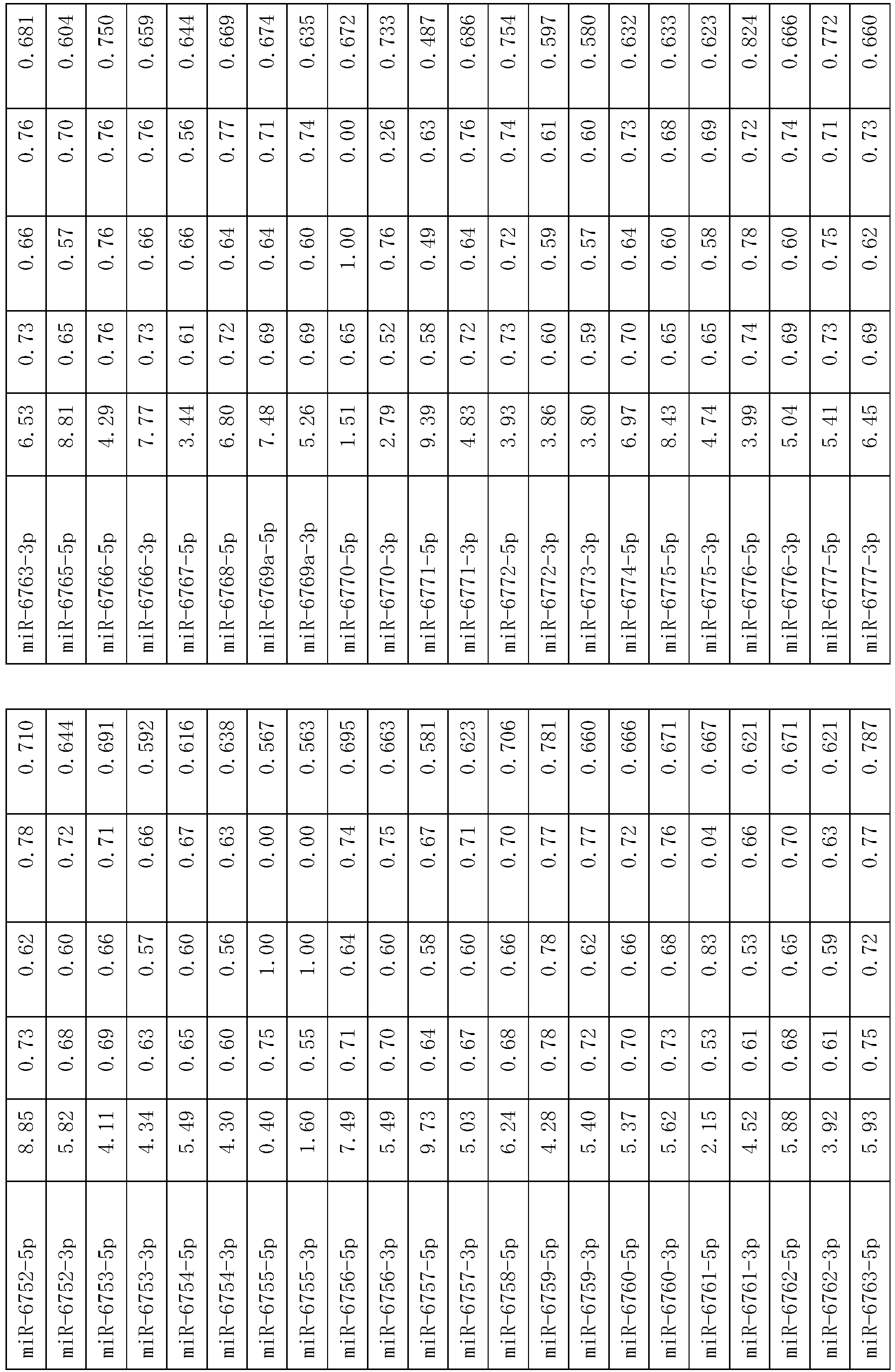

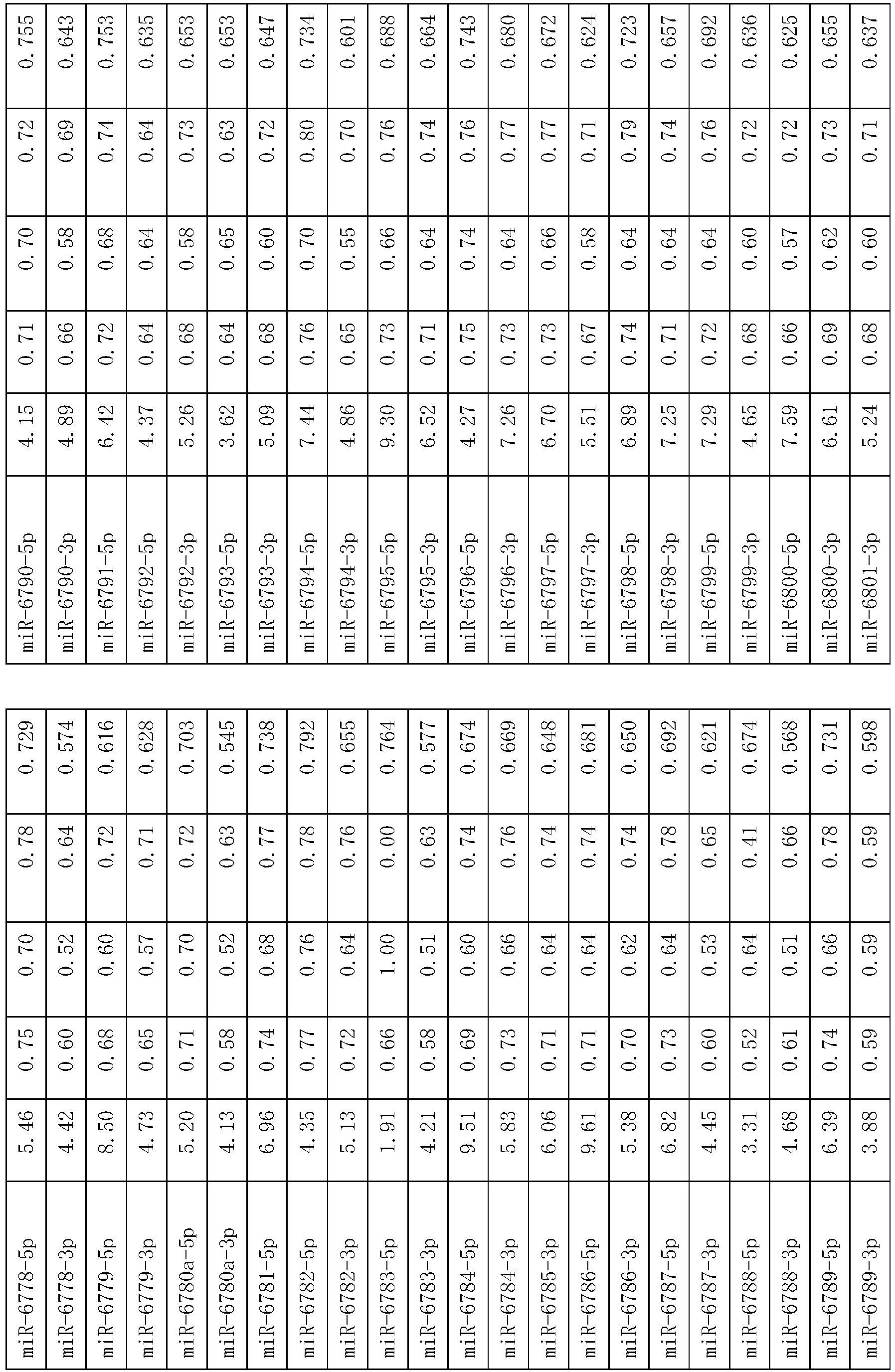

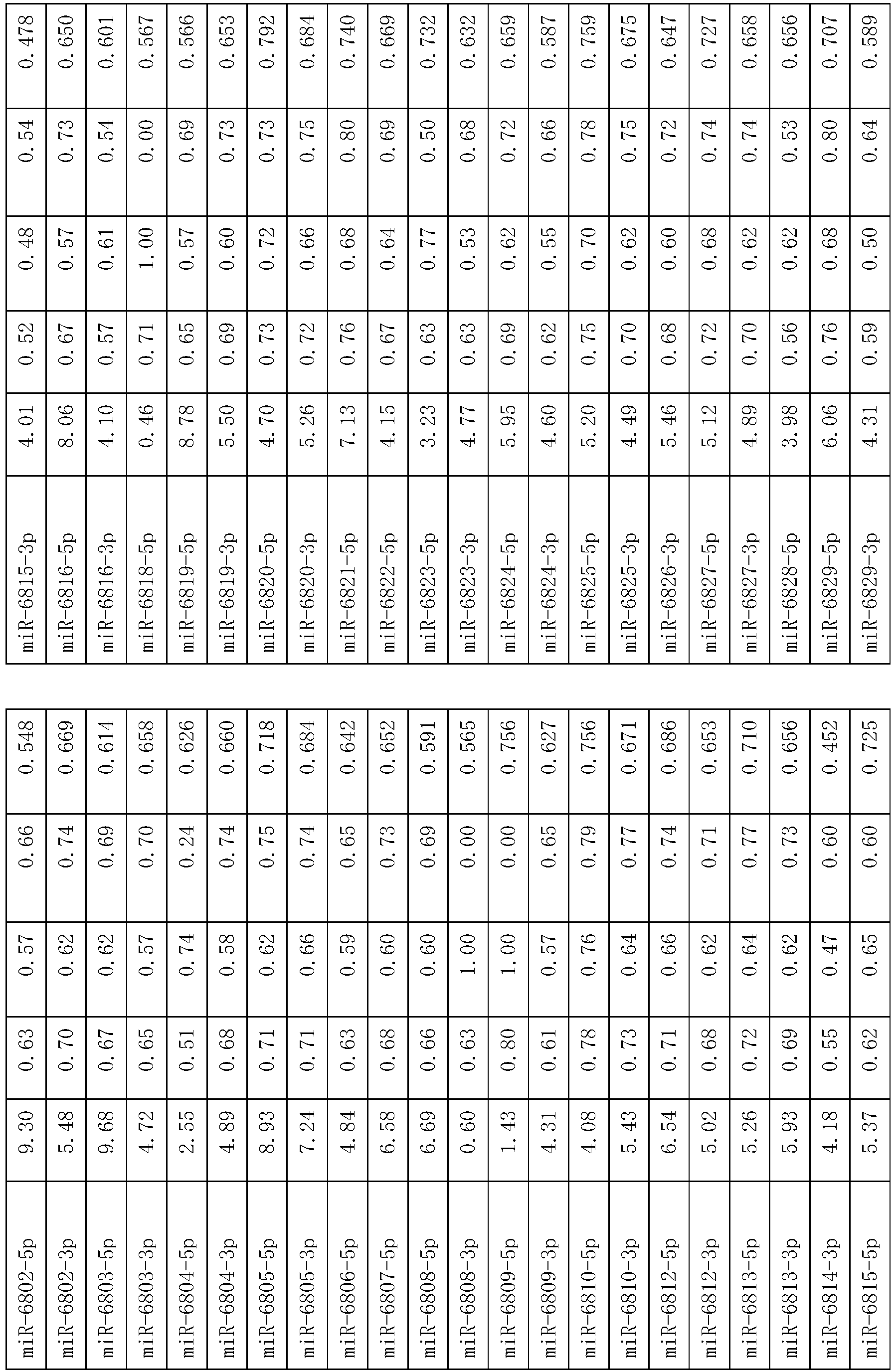

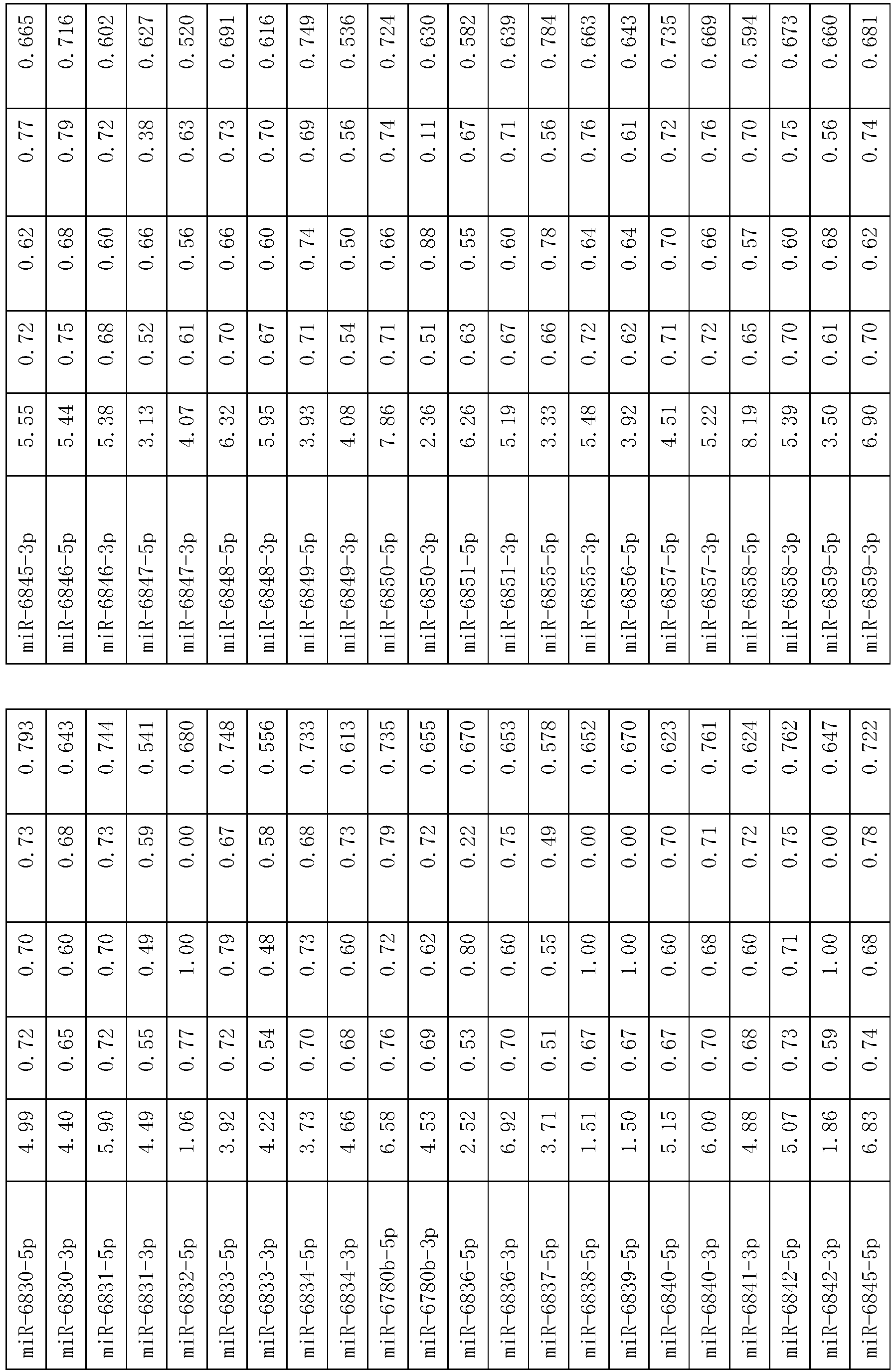

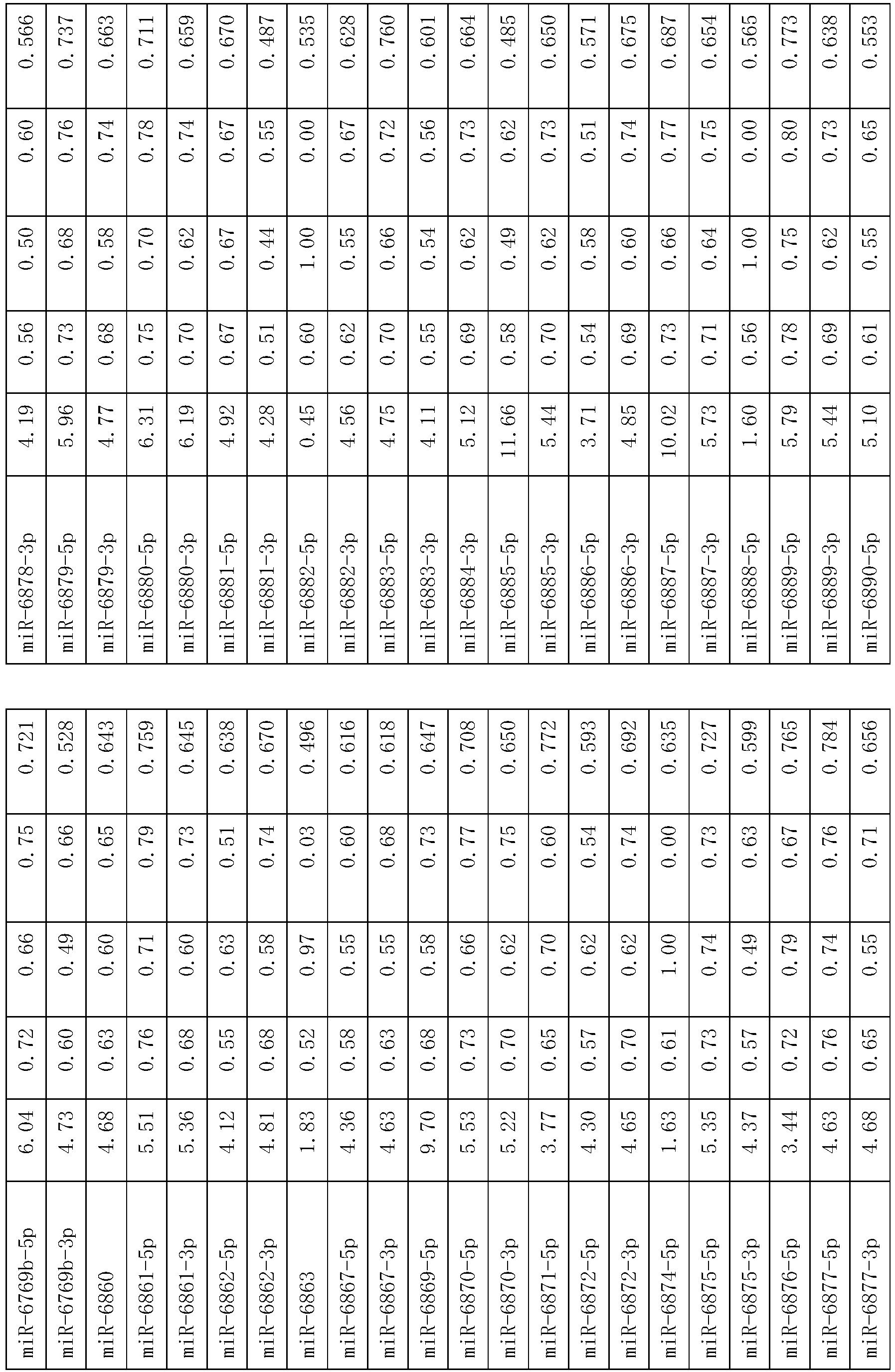

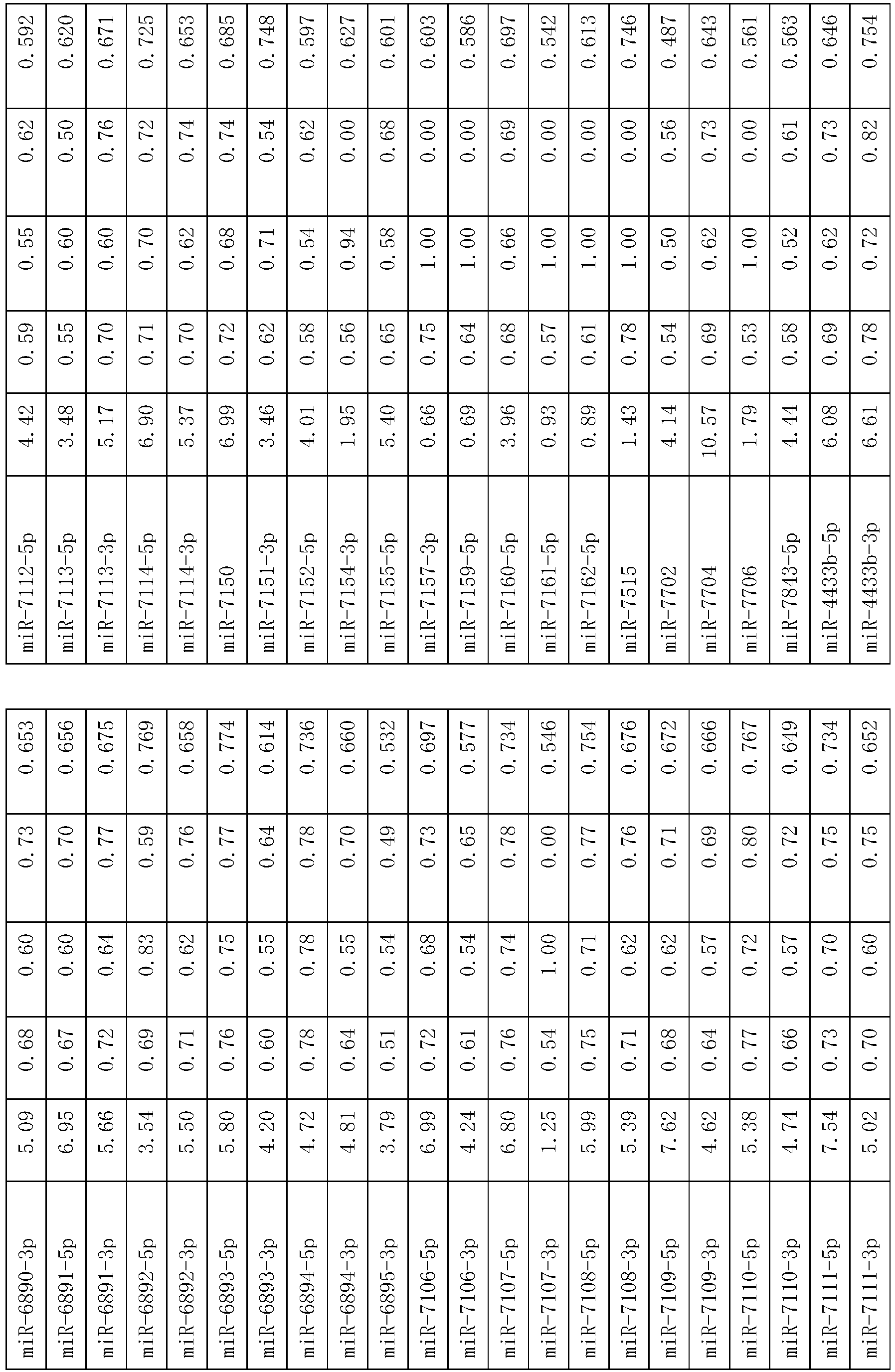

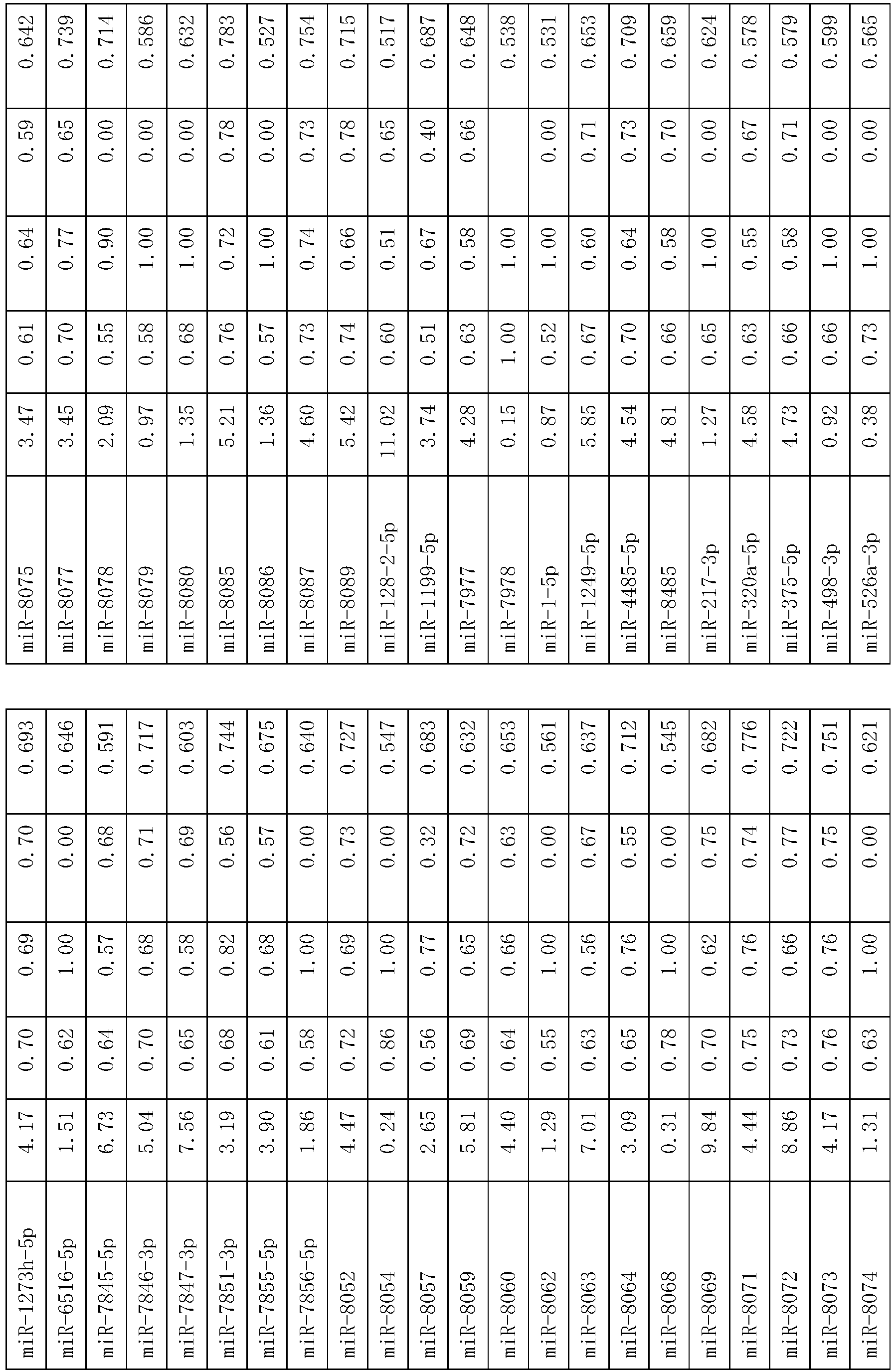

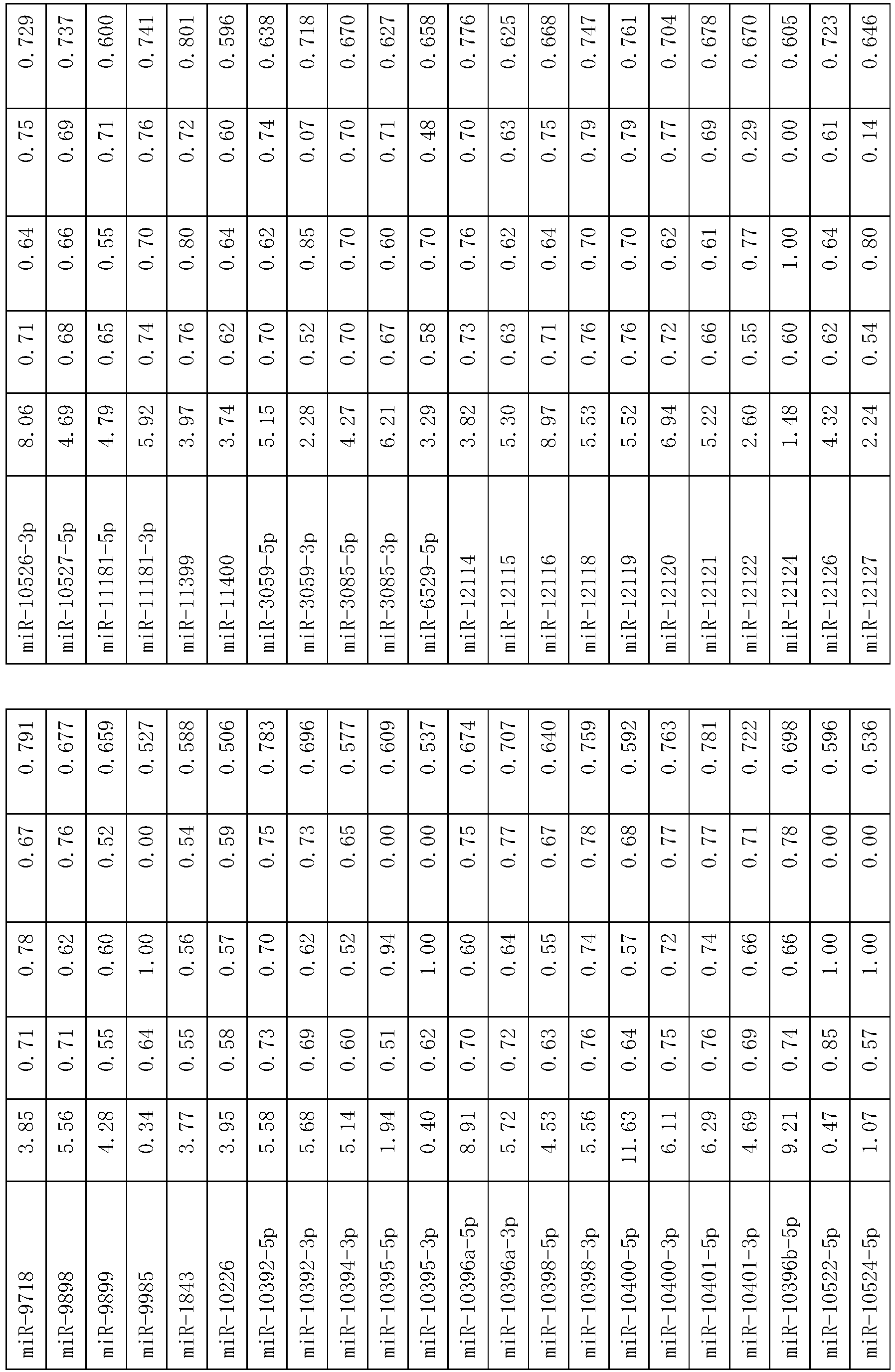

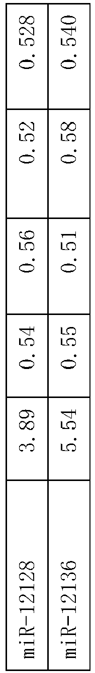

## Claims

1. An extract of urine extract, comprising a microRNA selected from the group consisting of (i) to (xvi):
(i) any of the microRNAs listed in data S1 or Table 2;
(ii) any of the microRNAs listed in Table 4-1;
(iii) any of the microRNAs listed in Table 4-2;
(iv) any of the microRNAs listed in Table 4-3;
(v) any of the microRNAs listed in Table 4-4;
(vi) any of the microRNAs listed in Table 4-5;
(vii) any of the microRNAs listed in Table 4-6;
(viii) any of the microRNAs listed in Table 4-7;
(ix) any of the microRNAs listed in Table 4-8;
(x) any of the microRNAs listed in Table 4-9;
(xi) any of the microRNAs listed in Table 4-10;
(xii) any of the microRNAs listed in Table 4-11;
(xiii) any of the microRNAs listed in Table 4-12;
(xiv) any of the microRNAs listed in Table 4-13;
(xv) any of the microRNAs listed in Table 4-14; and
(xvi) any of the microRNAs listed in Table 4-15,
{The microRNAs of each table may each independently comprise 1 or more, 2 or more, 3 or more, 4 or more, 5 or more, 10 or more, 15 or more, or 20 or more of the microRNAs.

2. A urine extract according to Claim 1, comprising a microRNA selected from the group consisting of the following (ii) to (xvi):
(ii) any of the microRNA with a p-value less than 0.005, listed in Table 4-1;
(iii) any of the microRNA with a p-value less than 0.005, listed in Table 4-2;
(iv) any of the microRNA with a p-value less than 0.005, listed in Table 4-3;
(v) any of the microRNA with a p-value less than 0.005, listed in Table 4-4;
(vi) any of the microRNA with a p-value less than 0.005, listed in Table 4-5;
(vii) any of the microRNA with a p-value less than 0.005, listed in Table 4-6;
(viii) any of the microRNA with a p-value less than 0.005, listed in Table 4-7;
(ix) any of the microRNA with a p-value less than 0.005, listed in Table 4-8;
(x) any of the microRNA with a p-value less than 0.005, listed in Table 4-9;
(xi) any of the microRNA with a p-value less than 0.005, listed in Table 4-10;
(xii) any of the microRNA with a p value less than 0.005, listed in Table 4-11; and
(xiii) any of the microRNA with a p-value less than 0.005, listed in Table 4-12;
(xiv) any of the microRNA with a p-value less than 0.005, listed in Table 4-13;
(xv) any of the microRNA with a p value less than 0.005, listed in Table 4-14; and
(xvi) any of the microRNA with a p-value less than 0.005, listed in Table 4-15,
{The microRNAs of each table may each independently comprise 1 or more, 2 or more, 3 or more, 4 or more, 5 or more, 10 or more, 15 or more, or 20 or more of the microRNAs.}

3. The urine extract according to Claim 1,
comprising (ii) any of the microRNAs listed in Table 4-1 {may be one or more, two or more, three or more, four or more, five or more, 10 or more, 15 or more, or 20 or more microRNAs},
wherein the urine extract is a urine extract of a lung cancer patient.

4. The urine extract according to Claim 1,
comprising (iii) any of the microRNAs listed in Table 4-2{may be one or more, two or more, three or more, four or more, five or more, 10 or more, 15 or more, or 20 or more microRNAs},
wherein the urine extract is a urine extract of a breast cancer patient.

5. The urine extract according to Claim 1,
comprising (iv) any of the microRNAs listed in Table 4-3 {may be one or more, two or more, three or more, four or more, five or more, 10 or more, 15 or more, or 20 or more microRNAs},
wherein the urine extract is a urine extract of a kidney cancer patient.

6. The urine extract according to Claim 1,
comprising (v) any of the microRNAs listed in Table 4-4 {may be one or more, two or more, three or more, four or more, five or more, 10 or more, 15 or more, or 20 or more microRNAs},
wherein the urine extract is a urine extract of a leukemia patient.

7. The urine extract according to Claim 1,
comprising (vi) any of the microRNAs listed in Table 4-5 {may be one or more, two or more, three or more, four or more, five or more, 10 or more, 15 or more, or 20 or more microRNAs},
wherein the urine extract is a urine extract of a lymphoma patient.

8. The urine extract according to Claim 1,
comprising (vii) any of the microRNAs listed in Table 4-6 {may be one or more, two or more, three or more, four or more, five or more, 10 or more, 15 or more, or 20 or more microRNAs},
wherein the urine extract is a urine extract of a pancreatic cancer patient.

9. The urine extract according to Claim 1,
comprising (viii) any of the microRNAs listed in Table 4-7 {may be one or more, two or more, three or more, four or more, five or more, 10 or more, 15 or more, or 20 or more microRNAs},
wherein the urine extract is a urine extract of a prostate cancer patient.

10. The urine extract according to Claim 1,
comprising (ix) any of the microRNAs listed in Table 4-8 {may be one or more, two or more, three or more, four or more, five or more, 10 or more, 15 or more, or 20 or more microRNAs},
wherein the urine extract is a urine extract of a gastric cancer patient.

11. The urine extract according to Claim 1,
comprising (x) any of the microRNAs listed in Table 4-9 {may be one or more, two or more, three or more, four or more, five or more, 10 or more, 15 or more, or 20 or more microRNAs},
wherein the urine extract is a urine extract of a urothelial carcinoma patient.

12. The urine extract according to Claim 1,
comprising (xi) any of the microRNAs listed in Table 4-10 {may be one or more, two or more, three or more, four or more, five or more, 10 or more, 15 or more, or 20 or more microRNAs},
wherein the urine extract is a urine extract of a melanoma patient.

13. The urine extract according to Claim 1,
comprising (xii) any of the microRNA listed in Table 4-11 {may be one or more, two or more, three or more, four or more, five or more, 10 or more, 15 or more, or 20 or more microRNAs},
wherein the urine extract is a urine extract of an ovarian cancer patient.

14. The urine extract according to Claim 1,
comprising (xiii) any of the microRNAs listed in Table 4-12 {may be one or more, two or more, three or more, four or more, five or more, 10 or more, 15 or more, or 20 or more microRNAs},
wherein the urine extract is a urine extract of a thyroid cancer patient.

15. The urine extract according to Claim 1,
comprising (xiii) any of the microRNA listed in Table 4-13 {may be one or more, two or more, three or more, four or more, five or more, 10 or more, 15 or more, or 20 or more microRNAs},
wherein the urine extract is a urine extract of a cervical cancer patient.

16. The urine extract according to Claim 1,
comprising (xiii) any of the microRNA listed in Table 4-14 {may be one or more, two or more, three or more, four or more, five or more, 10 or more, 15 or more, or 20 or more microRNAs},
wherein the urine extract is a urine extract of a colorectal cancer patient.

17. The urine extract according to Claim 1,
comprising (xiii) any of the microRNA listed in Table 4-15 {may be one or more, two or more, three or more, four or more, five or more, 10 or more, 15 or more, or 20 or more microRNAs},
wherein the urine extract is a urine extract of an endometrial cancer patient.

18. The extract of urine according to any one of Claims 1 to 17 above, comprising an extracellular vesicle, wherein said microRNA is contained in the extracellular vesicle, or wherein said microRNA is extracted from the extracellular vesicle.

19. A method for predicting the likelihood that a subject has cancer in a subject having or suspected of having cancer,
the method comprising predicting the likelihood that a subject has cancer as an indicator, using one or more of microRNAs selected from the group consisting of the below, in the urine extract obtained from the subject, as an indicator {In each table, said microRNA may be one or more, two or more, three or more, four or more, five or more, 10 or more, 15 or more, or 20 or more microRNAs }.
(i) any of the microRNAs listed in data S1 or in Table 2;
(ii) any of the microRNAs listed in Table 4-1;
(iii) any of the microRNAs listed in Table 4-2;
(iv) any of the microRNAs listed in Table 4-3;
(v) any of the microRNAs listed in Table 4-4;
(vi) any of the microRNAs listed in Table 4-5;
(vii) any of the microRNAs listed in Table 4-6;
(viii) any of the microRNAs listed in Table 4-7;
(ix) any of the microRNAs listed in Table 4-8;
(x) any of the microRNAs listed in Table 4-9;
(xi) any of the microRNAs listed in Table 4-10;
(xii) any of the microRNAs listed in Table 4-11;
(xiii) any of the microRNAs listed in Table 4-12;
(xiv) any of the microRNAs listed in Table 4-13;
(xv) any of the microRNAs listed in Table 4-14; and
(xvi) any of the microRNAs listed in Table 4-15.

20. The method according to Claim 19, wherein if the expression level of any one or more of the microRNAs in the urine extract obtained from the subject, listed in each table {in each table, the microRNA may be one or more, two or more, three or more, four or more, five or more, 10 or more, 15 or more, or 20 or more microRNAs} is greater than or equal to the first threshold set for each microRNA,
indicating a likelihood that the urine is derived from a cancer patient.

21. The method according to Claim 20, wherein
if the expression level of any one or more of the microRNAs in the urine extract obtained from the subject, listed in each table {in each table, the microRNA may be one or more, two or more, three or more, four or more, five or more, 10 or more, 15 or more, or 20 or more microRNAs} is greater than or equal to the first threshold set for each microRNA, indicating a likelihood that the urine is derived from a patient having a cancer selected from the group consisting of: lung cancer, breast cancer, kidney cancer, leukemia, lymphoma, pancreatic cancer, prostate cancer, melanoma, ovarian cancer, thyroid cancer, cervical cancer, colorectal cancer, and endometrial cancer.

22. The method according to Claim 20 or 21, wherein the first threshold is a value less than or equal to a third quartile value of the corresponding microRNA of a healthy person.

23. The method according to any one of Claims 20 to 22, wherein if the expression level of any one or more of the microRNAs in the urine extract obtained from the subject, selected from the group consisting of miR-17-5p, miR-33a-5p, miR-95-3p, miR-9-5p, miR-155-5p, miR-496, miR-544a, miR-556-5p, miR-577, miR-607, miR-19a-5p, "miR-548c-5p, miR-548o-5p, miR-548am-5p", miR-1278, miR-3117-3p, miR-3128, miR-3139, miR-3145-3p, miR-3201, miR-4282, miR-548ad-3p, miR-548ag, miR-4471, miR-4678, miR-4699-5p, miR-4704-3p, miR-1245b-5p, miR-5582-5p, miR-5582-3p, miR-5590-5p, miR-548aw, miR-5683, miR-580-5p, miR-1252-3p, miR-8058, and miR-548bb-5p {may be one or more, two or more, three or more, four or more, five or more, 10 or more, 15 or more, or 20 or more microRNAs} is greater than or equal to the first threshold set for each microRNA, indicating a likelihood that the urine is derived from a patient having lung cancer.

24. The method according to any one of Claims 20 to 22, wherein if the expression level of any one or more of the microRNAs in the urine extract obtained from the subject, selected from the group consisting of selected from the group consisting of miR-22-3p, miR-380-5p, miR-93-3p, miR-99a-3p, miR-543, miR-5706, and miR-6739-5p {may be one or more, two or more, three or more, four or more, five or more, 10 or more, 15 or more, or 20 or more microRNAs} is greater than or equal to the first threshold set for each microRNA, indicating a likelihood that the urine is derived from a patient having breast cancer.

25. The method according to any one of Claims 20 to 22, wherein if the expression level of any one or more of the microRNAs in the urine extract obtained from the subject, selected from the group consisting of selected from the group consisting of miR-22-3p, miR-380-5p, miR-93-3p, miR-99a-3p, miR-543, miR-5706, and miR-6739-5p {may be one or more, two or more, three or more, four or more, five or more, 10 or more, 15 or more, or 20 or more microRNAs} is greater than or equal to the first threshold set for each microRNA, indicating a likelihood that the urine is derived from a patient having kidney cancer.

26. The method according to any one of Claims 20 to 22, wherein if the expression level of any one or more of the microRNAs in the urine extract obtained from the subject, selected from the group consisting of selected from the group consisting of miR-301a-3p and miR-2114-3p {may be one or more, two or more, three or more, four or more, five or more, 10 or more, 15 or more, or 20 or more microRNAs} is greater than or equal to the first threshold set for each microRNA, indicating a likelihood that the urine is derived from a patient having leukemia.

27. The method according to any one of Claims 20 to 22, wherein if the expression level of any one or more of the microRNAs in the urine extract obtained from the subject, selected from the group consisting of selected from the group consisting of miR-30e-3p, miR-3166, miR-3942-5p, miR-4482-5p, miR-4493, and miR-5008-3p {may be one or more, two or more, three or more, four or more, five or more, 10 or more, 15 or more, or 20 or more microRNAs} is greater than or equal to the first threshold set for each microRNA, indicating a likelihood that the urine is derived from a patient having lymphoma.

28. The method according to any one of Claims 20 to 22, wherein if the expression level of any one or more of the microRNAs in the urine extract obtained from the subject, selected from the group consisting of selected from the group consisting of miR-16-5p, miR-218-5p, let-7g-5p, miR-195-5p, miR-619-3p, miR-643, miR-502-3p, miR-450b-5p, miR-3974, miR-4771, miR-5009-5p, miR-548at-3p, miR-372-5p, and miR-519d-5p {may be one or more, two or more, three or more, four or more, five or more, 10 or more, 15 or more, or 20 or more microRNAs} is greater than or equal to the first threshold set for each microRNA, indicating a likelihood that the urine is derived from a patient having pancreatic cancer.

29. The method according to any one of Claims 20 to 22, wherein if the expression level of miR-1324 in the urine extract obtained from the subject is greater than or equal to the first threshold set for each microRNA, indicating a likelihood that the urine is derived from a patient having prostate cancer.

30. The method according to any one of Claims 20 to 22, wherein if the expression level of any one or more of the microRNAs in the urine extract obtained from the subject, selected from the group consisting of selected from the group consisting of miR-1243 and miR-4715-3p {may be one or more, two or more, three or more, four or more, five or more, 10 or more, 15 or more, or 20 or more microRNAs} is greater than or equal to the first threshold set for each microRNA, indicating a likelihood that the urine is derived from a patient having gastric cancer.

31. The method according to any one of Claims 20 to 22, wherein if the expression level of any one or more of the microRNAs in the urine extract obtained from the subject, selected from the group consisting of selected from the group consisting of miR-4795-3p, miR-5707, and miR-655-5p {may be one or more, two or more, three or more, four or more, five or more, 10 or more, 15 or more, or 20 or more microRNAs} is greater than or equal to the first threshold set for each microRNA, indicating a likelihood that the urine is derived from a patient having urothelial carcinoma.

32. The method according to any one of Claims 20 to 22, wherein if the expression level of any one or more of the microRNAs in the urine extract obtained from the subject, selected from the group consisting of selected from the group consisting of miR-18b-5p, miR-562, miR-582-3p, miR-216b-5p, miR-1251-5p, miR-1252-5p, miR-2053, miR-3915, miR-4418, miR-4659a-3p, miR-651-3p, miR-1468-3p, and miR-9903 {may be one or more, two or more, three or more, four or more, five or more, 10 or more, 15 or more, or 20 or more microRNAs} is greater than or equal to the first threshold set for each microRNA, indicating a likelihood that the urine is derived from a patient having melanoma.

33. The method according to any one of Claims 20 to 22, wherein if the expression level of any one or more of the microRNAs in the urine extract obtained from the subject, selected from the group consisting of selected from the group consisting of miR-335-5p, miR-27a-5p, miR-499a-3p, and miR-8084 {may be one or more, two or more, three or more, four or more, five or more, 10 or more, 15 or more, or 20 or more microRNAs} is greater than or equal to the first threshold set for each microRNA, indicating a likelihood that the urine is derived from a patient having ovarian cancer.

34. The method according to any one of Claims 20 to 22, wherein if the expression level of any one or more of the microRNAs in the urine extract obtained from the subject, selected from the group consisting of selected from the group consisting of miR-376a-5p, miR-374b-3p, miR-4477b, miR-548am-3p, miR-4536-5p, miR-4639-5p, and miR-4798-3p {may be one or more, two or more, three or more, four or more, five or more, 10 or more, 15 or more, or 20 or more microRNAs} is greater than or equal to the first threshold set for each microRNA, indicating a likelihood that the urine is derived from a patient having thyroid cancer.

35. The method according to any one of Claims 20 to 22, wherein if the expression level of any one or more of the microRNAs in the urine extract obtained from the subject, selected from the group consisting of selected from the group consisting of miR-143-5p, miR-3182, miR-4746-5p, miR-548ao-3p, miR-514a-5p, miR-376c-5p, miR-376a-2-5p, miR-6773-5p, miR-6807-3p, miR-8065, and miR-301b-5p {may be one or more, two or more, three or more, four or more, five or more, 10 or more, 15 or more, or 20 or more microRNAs} is greater than or equal to the first threshold set for each microRNA, indicating a likelihood that the urine is derived from a patient having cervical cancer.

36. The method according to any one of Claims 20 to 22, wherein if the expression level of any one or more of the microRNAs in the urine extract obtained from the subject, selected from the group consisting of selected from the group consisting of miR-10b-5p, miR-337-5p, miR-6888-3p, and miR-9983-3p {may be one or more, two or more, three or more, four or more, five or more, 10 or more, 15 or more, or 20 or more microRNAs} is greater than or equal to the first threshold set for each microRNA, indicating a likelihood that the urine is derived from a patient having rectal colon cancer.

37. The method according to any one of Claims 20 to 22, wherein if the expression level of any one or more of the microRNAs in the urine extract obtained from the subject, selected from the group consisting of selected from the group consisting of miR-199a-3p, miR-199b-3p, miR-518e-3p, miR-626, miR-548x-3p, miR-4659a-5p, miR-4671-3p, miR-4666b, and miR-8068 {may be one or more, two or more, three or more, four or more, five or more, 10 or more, 15 or more, or 20 or more microRNAs} is greater than or equal to the first threshold set for each microRNA, indicating a likelihood that the urine is derived from a patient having endometrial cancer.

38. The method according to Claim 23 or 27, wherein the first threshold is a value less than or equal to a third quartile value of the corresponding microRNA of a healthy person.

39. The method according to Claim 19, wherein if the expression level of any one or more of the microRNAs in the urine extract obtained from the subject, listed in each table {may be one or more, two or more, three or more, four or more, five or more, 10 or more, 15 or more, or 20 or more microRNAs} is less than or equal to the second threshold set for each microRNA, indicating a likelihood that the urine is derived from a patient having cancer.

40. A method for predicting a likelihood that a subject having or suspected of having cancer does not have cancer, wherein if any one or more of the microRNAs in the urine extract obtained from the subject, selected from the group consisting of let-7a-5p, let-7c-5p, let-7d-5p, let-7f-5p, miR-18a-5p, miR-19a-3p, miR-20a-5p, miR-21-5p, miR-24-1-5p, miR-26a-5p, miR-26b-5p, miR-31-5p, miR-96-5p, miR-98-5p, miR-99a-5p, miR-100-5p, miR-101-3p, miR-106a-5p, miR-148a-3p, miR-7-5p, miR-181c-5p, miR-182-3p, miR-216a-5p, miR-217-5p, miR-219a-5p, miR-222-3p, miR-1-3p, miR-130a-3p, miR-137-3p, miR-142-5p, miR-144-3p, miR-152-3p, miR-153-3p, miR-9-3p, miR-126-5p, miR-126-3p, miR-127-3p, miR-136-5p, miR-150-5p, miR-186-5p, miR-190a-5p, miR-200a-3p, miR-302a-5p, miR-30e-5p, miR-362-5p, miR-302b-5p, miR-302b-3p, miR-302c-3p, miR-302d-3p, miR-367-3p, miR-376c-3p, miR-369-3p, miR-376a-3p, miR-377-3p, miR-384, miR-409-5p, miR-410-3p, miR-376b-3p, miR-146b-5p, miR-520f-3p, miR-519c-3p, miR-523-3p, miR-518c-3p, miR-524-3p, miR-518d-3p, miR-499a-5p, miR-505-3p, miR-507, miR-508-3p, miR-514a-3p, miR-455-5p, miR-545-3p, miR-553, miR-559, miR-561-3p, miR-567, miR-568, miR-569, miR-570-3p, miR-573, miR-582-5p, miR-548a-3p, miR-586, miR-548b-3p, miR-590-5p, miR-592, miR-597-5p, miR-599, miR-606, miR-548c-3p, miR-618, miR-620, miR-621, miR-33b-5p, miR-633, miR-648, miR-651-5p, miR-411-5p, miR-655-3p, miR-549a-3p, miR-660-5p, miR-542-3p, miR-758-3p, miR-454-3p, miR-802, miR-19b-1-5p, miR-19b-2-5p, miR-28-3p, miR-100-3p, miR-105-3p, miR-30d-3p, miR-10a-3p, miR-10b-3p, miR-34a-3p, miR-196a-3p, miR-218-1-3p, miR-218-2-3p, miR-221-5p, miR-27b-5p, miR-195-3p, miR-26a-2-3p, miR-367-5p, miR-374a-3p, miR-340-5p, miR-135b-3p, miR-488-3p, miR-497-3p, miR-455-3p, miR-545-5p, miR-556-3p, miR-576-3p, miR-548b-5p, miR-590-3p, miR-548a-5p, miR-628-5p, miR-548d-5p, miR-450b-3p, miR-890, miR-889-3p, miR-875-5p, miR-876-5p, miR-708-3p, miR-190b-5p, miR-873-5p, miR-301b-3p, miR-208b-3p, miR-922, miR-934, miR-944, miR-1185-5p, miR-1283, miR-1179, miR-1183, miR-1206, miR-548e-3p, miR-548j-5p, miR-548k, miR-1295a, miR-1302, miR-1305, miR-1244, miR-1245a, miR-1256, miR-1258, miR-1261, miR-1262, miR-548n, miR-548m, miR-548h-5p, miR-302e, miR-302f, miR-1277-3p, miR-548i, miR-1282, miR-1197, miR-1537-3p, miR-205-3p, miR-1973, miR-2054, miR-759, miR-2115-3p, miR-3115, miR-3118, miR-3121-3p, miR-3123, miR-3129-5p, miR-3134, miR-3135a, miR-544b, miR-3140-3p, miR-548t-5p, miR-3143, miR-548u, miR-3146, miR-3152-3p, miR-3159, miR-3167, miR-3171, miR-548w, miR-3183, miR-3065-5p, miR-4302, miR-4309, miR-4263, miR-4272, miR-4277, miR-4291, miR-3606-5p, miR-3609, miR-3611, miR-3613-5p, miR-3616-5p, miR-3618, miR-23c, miR-3658, miR-3662, miR-3664-5p, miR-3668, miR-3671, miR-3672, miR-3674, miR-3683, miR-3684, miR-3686, miR-3689a-5p, miR-3689b-5p, miR-3689e, miR-3912-3p, miR-3914, miR-3920, miR-3923, miR-3927-3p, miR-3938, miR-548y, miR-3939, miR-548z, miR-548h-3p, miR-4422, miR-548ac, miR-4424, miR-4426, miR-4427, miR-548ae-3p, miR-4445-5p, miR-4452, miR-4457, miR-4464, miR-548ai, miR-570-5p, miR-548aj-3p, miR-4469, miR-4477a, miR-548ak, miR-4490, miR-4500, miR-4503, miR-4504, miR-4509, miR-4517, miR-4528, miR-548an, miR-3117-5p, miR-3121-5p, miR-3124-3p, miR-3136-3p, miR-3140-5p, miR-3664-3p, miR-3688-5p, miR-3942-3p, miR-4474-5p, miR-3976, miR-3977, miR-4636, miR-4662a-5p, miR-4662a-3p, miR-4659b-5p, miR-4663, miR-4662b, miR-4666a-5p, miR-4666a-3p, miR-4668-3p, miR-219b-3p, miR-4671-5p, miR-4677-3p, miR-4679, miR-4680-5p, miR-4680-3p, miR-4683, miR-4693-5p, miR-4693-3p, miR-4696, miR-4699-3p, miR-4703-5p, miR-4705, miR-203b-3p, miR-4711-5p, miR-4714-3p, miR-4720-3p, miR-4735-5p, miR-4735-3p, miR-4742-5p, miR-4744, miR-499b-5p, miR-4757-5p, miR-4757-3p, miR-4760-5p, miR-4760-3p, miR-4765, miR-4766-3p, miR-4772-5p, miR-4772-3p, miR-4774-5p, miR-4774-3p, miR-4777-5p, miR-4777-3p, miR-4778-3p, miR-4782-5p, miR-4782-3p, miR-1245b-3p, miR-4789-5p, miR-4789-3p, miR-4790-3p, miR-4795-5p, miR-4797-5p, miR-4798-5p, miR-4799-3p, miR-4520-2-3p, miR-5047, miR-548ah-3p, miR-548ao-5p, miR-548ap-5p, miR-548ap-3p, miR-5186, miR-5191, miR-5197-3p, miR-548aq-5p, miR-548aq-3p, miR-548ar-5p, miR-548ar-3p, miR-5583-5p, miR-5583-3p, miR-5586-5p, miR-548au-5p, miR-5590-3p, miR-5591-5p, miR-548av-5p, miR-5682, miR-5692c, miR-5687, miR-5688, miR-5691, miR-5692a, miR-5697, miR-5700, miR-5701, miR-5692b, miR-450a-1-3p, miR-506-5p, miR-539-3p, miR-561-5p, miR-1271-3p, miR-548g-5p, miR-548x-5p, miR-548aj-5p, miR-1277-5p, miR-513c-3p, miR-1178-5p, miR-3606-3p, miR-6079, miR-6128, miR-548ay-5p, miR-548az-5p, miR-548az-3p, miR-6502-3p, miR-6508-3p, miR-95-5p, miR-215-3p, miR-153-5p, miR-190a-3p, miR-412-5p, miR-520g-5p, miR-510-3p, miR-653-3p, miR-670-3p, miR-548e-5p, miR-548f-5p, miR-513b-3p, miR-1537-5p, miR-6733-5p, miR-6811-5p, miR-6828-3p, miR-6838-3p, miR-6844, miR-6854-5p, miR-6864-3p, miR-6866-5p, miR-7153-5p, miR-7153-3p, miR-7154-5p, miR-7159-3p, miR-7160-3p, miR-7161-3p, miR-7705, miR-6516-3p, miR-8053, miR-8056, miR-8061, miR-8066, miR-8067, miR-8076, miR-181b-2-3p, miR-548ad-5p, miR-548ae-5p, miR-548bb-3p, miR-520e-5p, miR-549a-5p, miR-147b-5p, miR-9900, miR-548bc, miR-10393-5p, miR-10393-3p, miR-10397-3p, miR-10399-5p, miR-10525-3p, miR-12123, miR-12129, miR-12130, and miR-12132 {may be one or more, two or more, three or more, four or more, five or more, 10 or more, 15 or more, or 20 or more microRNAs} is less than or equal to the first threshold for a microRNA marker which shows an expression higher for cancer patients than non-cancer patients, or is greater than or equal to the second threshold for a microRNA marker which shows an expression lower for cancer patients than non-cancer patients, indicating a likelihood that the urine is derived from a patient not having cancer.

41. A method for predicting a likelihood that a subject having or suspected of having cancer has early-stage cancer, the method comprising predicting the likelihood that a subject has early-stage cancer, by using the expression of the microRNA in the urine extract of the subject, listed in in any one of tables of Tables 22-1 and Tables 23-1 to 23-14 {In each table, may independently be one or more, two or more, three or more, four or more, five or more, 10 or more, 15 or more, or 20 or more microRNAs} as an indicator.

42. A method for predicting a likelihood that a subject having or suspected of having cancer has stage-I early-stage cancer, the method comprising predicting the likelihood that a subject has early-stage cancer, by using the expression of the microRNA in any one of tables of Tables 22-1 to 22-13 {In each table, may independently be one or more, two or more, three or more, four or more, five or more, 10 or more, 15 or more, or 20 or more microRNAs} as an indicator.

43. A method of analyzing a urine sample, comprising:
contacting a urine sample with a nanowire;
extracting microRNAs captured on the nanowires; and
confirming whether the extracted microRNAs contain a specific microRNA by contacting the microRNA with a nucleic acid containing a sequence which hybridizes to the microRNA,
wherein the specific microRNA is one or more microRNAs selected from the group consisting of {in each table, each independently may be one or more, two or more, three or more, four or more, five or more, 10 or more, 15 or more, or 20 or more microRNAs}:
(i) any of the microRNAs listed in data S1 or Table 2;
(ii) any of the microRNAs listed in Table 4-1;
(iii) any of the microRNAs listed in Table 4-2;
(iv) any of the microRNAs listed in Table 4-3;
(v) any of the microRNAs listed in Table 4-4;
(vi) any of the microRNAs listed in Table 4-5;
(vii) any of the microRNAs listed in Table 4-6;
(viii) any of the microRNAs listed in Table 4-7;
(ix) any of the microRNAs listed in Table 4-8;
(x) any of the microRNAs listed in Table 4-9;
(xi) any of the microRNAs listed in Table 4-10;
(xii) any of the microRNAs listed in Table 4-11;
(xiii) any of the microRNAs listed in Table 4-12;
(xiv) any of the microRNAs listed in Table 4-13;
(xv) any of the microRNAs listed in Table 4-14; and
(xvi) Any of the microRNAs listed in Table 4-15.

44. The method according to Claim 43,
wherein the specific microRNA is one or more microRNAs selected from the group consisting of {in each table, each independently may be one or more, two or more, three or more, four or more, five or more, 10 or more, 15 or more, or 20 or more microRNAs}:
the below (ii) to (xiii):
(ii) any of the microRNA with a p-value less than 0.005, listed in Table 4-1;
(iii) any of the microRNA with a p-value less than 0.005, listed in Table 4-2;
(iv) any of the microRNA with a p-value less than 0.005, listed in Table 4-3;
(v) any of the microRNA with a p-value less than 0.005, listed in Table 4-4;
(vi) any of the microRNA with a p-value less than 0.005, listed in Table 4-5;
(vii) any of the microRNA with a p-value less than 0.005, listed in Table 4-6;
(viii) any of the microRNA with a p-value less than 0.005, listed in Table 4-7;
(ix) any of the microRNA with a p-value less than 0.005, listed in Table 4-8;
(x) any of the microRNA with a p-value less than 0.005, listed in Table 4-9;
(xi) any of the microRNA with a p-value less than 0.005, listed in Table 4-10;
(xii) any of the microRNAs with a p value less than 0.005, listed in Table 4-11; and
(xiii) any of the microRNA with a p-value less than 0.005, listed in Table 4-12;
(xiv) any of the microRNA with a p-value less than 0.005, listed in Table 4-13;
(xv) any of the microRNAs with a p value of less than 0.005, listed in Table 4-14; and
(xvi) any of the microRNA with a p-value less than 0.005, listed in Table 4-15.

45. The method according to Claim 43 or 44,
wherein the urine is the urine of a subject having or suspected of having lung cancer,
wherein the specific microRNAs is
(ii) any of the microRNAs listed in Table 4-1 {may be one or more, two or more, three or more, four or more, five or more, 10 or more, 15 or more, or 20 or more microRNAs}.

46. The method according to Claim 43 or 44,
wherein the urine is the urine of a subject having or suspected of having breast cancer,
wherein the specific microRNAs is
(iii) any of the microRNAs listed in Table 4-2 {may be one or more, two or more, three or more, four or more, five or more, 10 or more, 15 or more, or 20 or more microRNAs}.

47. The method according to Claim 43 or 44,
wherein the urine is the urine of a subject having kidney cancer or suspected of having breast cancer, wherein the specific microRNAs is
(iv) any of the microRNAs listed in Table 4-3 {may be one or more, two or more, three or more, four or more, five or more, 10 or more, 15 or more, or 20 or more microRNAs}.

48. The method according to Claim 43 or 44,
wherein the urine is the urine of a subject having or suspected of having leukemia,
wherein the specific microRNAs is
(v) any of the microRNAs listed in Table 4-4 {may be one or more, two or more, three or more, four or more, five or more, 10 or more, 15 or more, or 20 or more microRNAs}.

49. The method according to Claim 43 or 44,
wherein the urine is the urine of a subject having or suspected of having lymphoma,
wherein the specific microRNAs is
(vi) any of the microRNAs listed in Table 4-5 {may be one or more, two or more, three or more, four or more, five or more, 10 or more, 15 or more, or 20 or more microRNAs}.

50. The method according to Claim 43 or 44,
wherein the urine is the urine of a subject having or suspected of having pancreatic cancer,
wherein the specific microRNAs is
(vii) any of the microRNAs listed in Table 4-6 {may be one or more, two or more, three or more, four or more, five or more, 10 or more, 15 or more, or 20 or more microRNAs}.

51. The method according to Claim 43 or 44,
wherein the urine is the urine of a subject having or suspected of having prostate cancer,
wherein the specific microRNAs is
(viii) any of the microRNAs listed in Table 4-7 {may be one or more, two or more, three or more, four or more, five or more, 10 or more, 15 or more, or 20 or more microRNAs}.

52. The method according to Claim 43 or 44,
wherein the urine is the urine of a subject having or suspected of having gastric cancer,
wherein the specific microRNAs is
(ix) any of the microRNAs listed in Table 4-8 {may be one or more, two or more, three or more, four or more, five or more, 10 or more, 15 or more, or 20 or more microRNAs}.

53. The method according to Claim 43 or 44,
wherein the urine is the urine of a subject having or suspected of having urothelial carcinoma,
wherein the specific microRNAs is
(x) any of the microRNAs listed in Table 4-9 {may be one or more, two or more, three or more, four or more, five or more, 10 or more, 15 or more, or 20 or more microRNAs}.

54. The method according to Claim 43 or 44,
wherein the urine is the urine of a subject having or suspected of having melanoma,
wherein the specific microRNAs is
(xi) any of the microRNAs listed in Table 4-10 {may be one or more, two or more, three or more, four or more, five or more, 10 or more, 15 or more, or 20 or more microRNAs}.

55. The method according to Claim 43 or 44,
wherein the urine is the urine of a subject having or suspected of having ovarian cancer,
wherein the specific microRNAs is
(xii) any of the microRNAs listed in Table 4-11 {may be one or more, two or more, three or more, four or more, five or more, 10 or more, 15 or more, or 20 or more microRNAs}.

56. The method according to Claim 43 or 44,
wherein the urine is the urine of a subject having or suspected of having thyroid cancer,
wherein the specific microRNAs is
(xiii) any of the microRNAs listed in Table 4-12 {may be one or more, two or more, three or more, four or more, five or more, 10 or more, 15 or more, or 20 or more microRNAs}.

57. The method according to Claim 43 or 44,
wherein the urine is the urine of a subject having or suspected of having cervical cancer,
wherein the specific microRNAs is
(xiii) any of the microRNAs listed in Table 4-13 {may be one or more, two or more, three or more, four or more, five or more, 10 or more, 15 or more, or 20 or more microRNAs}.

58. The method according to Claim 43 or 44,
wherein the urine is the urine of a subject having or suspected of having colorectal cancer,
wherein the specific microRNAs is
(xiii) any of the microRNAs listed in Table 4-14 {may be one or more, two or more, three or more, four or more, five or more, 10 or more, 15 or more, or 20 or more microRNAs}.

59. The method according to Claim 43 or 44,
wherein the urine is the urine of a subject having or suspected of having endometrial cancer,
wherein the specific microRNAs is
(xiii) any of the microRNAs listed in Table 4-15 {may be one or more, two or more, three or more, four or more, five or more, 10 or more, 15 or more, or 20 or more microRNAs}.

60. The method according to Claim 45 to 59, wherein the microRNA has a p value less than 0.005 {In each table, the microRNA may be one or more, two or more, three or more, four or more, five or more, 10 or more, 15 or more, or 20 or more microRNAs

61. A method of analyzing a urine sample, comprising:
contacting a urine sample with a nanowire;
extracting microRNAs captured on the nanowire; and
confirming whether the extracted microRNAs contain a specific microRNA,
wherein the specific microRNAs is
(a) the microRNAs listed in any one of Tables 22-1 and 23-1 to 23-14 {in each table, each may independently be one or more, two or more, three or more, four or more, five or more, 10 or more, 15 or more, or 20 or more microRNAs}.

62. The method of analyzing a urine sample according to [61] above,
wherein the specific microRNAs is
(b) the microRNAs listed in any one of Tables 22-1 to 22-13 {in each table, each may independently be one or more, two or more, three or more, four or more, five or more, 10 or more, 15 or more, or 20 or more microRNAs}.

63. The method of analyzing a urine sample according to [61] above,
wherein the specific microRNAs is
(a) the microRNAs listed in any one of Tables 22-1 and 23-1 to 23-14 {in each table, each may independently be one or more, two or more, three or more, four or more, five or more, 10 or more, 15 or more, or 20 or more microRNAs},
wherein the microRNA has an accuracy of 70% or higher.

64. The method of analyzing a urine sample according to [61] above,
wherein the specific microRNAs is
(a) the microRNAs listed in any one of Tables 22-1 and 23-1 to 23-14 {in each table, each may independently be one or more, two or more, three or more, four or more, five or more, 10 or more, 15 or more, or 20 or more microRNAs},
wherein the microRNA has an accuracy of 80% or higher.

65. The method of analyzing a urine sample according to [61] above,
wherein the specific microRNAs is
(a) the microRNAs listed in any one of Tables 22-1 and 23-1 to 23-14 {in each table, each may independently be one or more, two or more, three or more, four or more, five or more, 10 or more, 15 or more, or 20 or more microRNAs},
wherein the microRNA has an accuracy of an accuracy of 90% or higher.

66. The method of analyzing a urine sample according to [61] above,
wherein the specific microRNAs is
(b) the microRNAs listed in any one of Tables 22-1 to 22-13 {in each table, each may independently be one or more, two or more, three or more, four or more, five or more, 10 or more, 15 or more, or 20 or more microRNAs},
wherein the microRNA has an accuracy of 70% or higher.

67. The method of analyzing a urine sample according to [62] above,
wherein the specific microRNAs is
(b) the microRNAs listed in any one of Tables 22-1 to 22-13 {in each table, each may independently be one or more, two or more, three or more, four or more, five or more, 10 or more, 15 or more, or 20 or more microRNAs},
wherein the microRNA has an accuracy of 80% or higher.

68. The method of analyzing a urine sample according to [62] above,
wherein the specific microRNAs is
(b) the microRNAs listed in any one of Tables 22-1 to 22-13 {in each table, each may independently be one or more, two or more, three or more, four or more, five or more, 10 or more, 15 or more, or 20 or more microRNAs},
wherein the microRNA has an accuracy of 90% or higher.

69. A method of analyzing a urine sample, comprising:
contacting a urine sample with a nanowire;
extracting microRNAs captured on the nanowire; and
confirming whether the extracted microRNAs contain a specific microRNA by contacting the microRNA with a nucleic acid containing a sequence which hybridizes to the microRNA,
wherein the specific microRNAs is
(c) the microRNAs listed in any one of Tables 22-1 to 22-13 and Tables 23-1 to 23-14 {in each table, each may independently be one or more, two or more, three or more, four or more, five or more, 10 or more, 15 or more, or 20 or more microRNAs}.

70. The method of analyzing urine according to Claim 69,
wherein the specific microRNAs is
(c) the microRNAs listed in any one of Tables 22-1 to 22-13 and Tables 23-1 to 23-14 {in each table, each may independently be one or more, two or more, three or more, four or more, five or more, 10 or more, 15 or more, or 20 or more microRNAs}, and is microRNA having an accuracy of 70% or higher in the table.

71. The method for analyzing urine according to Claim 69,
wherein the specific microRNAs is
(c) the microRNAs listed in any one of Tables 22-1 to 22-13 and Tables 23-1 to 23-14 {in each table, each may independently be one or more, two or more, three or more, four or more, five or more, 10 or more, 15 or more, or 20 or more microRNAs},
and is microRNA having an accuracy of 70% or higher in the table.

72. The method for analyzing urine according to Claim 69,
wherein the specific microRNAs is
(c) the microRNAs listed in any one of Tables 22-1 to 22-13 and Tables 23-1 to 23-14 {in each table, each may independently be one or more, two or more, three or more, four or more, five or more, 10 or more, 15 or more, or 20 or more microRNAs},
and is microRNA having an accuracy of 90% or higher in the table.

73. A method for analyzing a urine sample, comprising:
contacting a urine sample with a nanowire in a nanowire-embedded fluidic device having nanowires of metal oxide (e.g., zinc oxide);
extracting microRNAs captured on the nanowire; and
confirming whether the extracted microRNAs contain a specific microRNA by contacting the microRNA with a nucleic acid containing a sequence which hybridizes to the microRNA,
wherein the specific microRNAs is
selected from the group (ALL) consisting of miR-134-5p, miR-346, miR-564, miR-574-3p, miR-575, miR-632, miR-661, miR-663a, miR-658, miR-297, miR-139-3p, miR-149-3p, miR-20b-3p, miR-431-3p, miR-550a-5p, miR-885-5p, miR-936, miR-937-3p, miR-943, miR-1228-5p, miR-1184, miR-1204, miR-1538, miR-1909-5p, miR-1910-5p, miR-1912-3p, miR-196b-3p, miR-1972, miR-3153, miR-3162-5p, miR-1193, miR-4260, miR-4253, miR-4326, miR-4269, miR-4276, miR-3622a-3p, miR-3646, miR-3652, miR-3180, miR-550b-3p, miR-4428, miR-4433a-3p, miR-4440, miR-4444, miR-4447, miR-4449, miR-4455, miR-3155b, miR-4483, miR-4498, miR-2392, miR-4535, miR-4539, miR-3173-5p, miR-4529-5p, miR-4638-5p, miR-4655-5p, miR-4685-3p, miR-1343-3p, miR-4701-3p, miR-4717-3p, miR-4725-5p, miR-4726-3p, miR-4732-5p, miR-4738-3p, miR-4748, miR-4750-5p, miR-371b-5p, miR-4436b-5p, miR-4793-3p, miR-5001-3p, miR-5002-3p, miR-5006-5p, miR-5090, miR-5572, miR-5584-3p, miR-5705, miR-1237-5p, miR-6072, miR-6131, miR-6503-3p, miR-6511b-5p, miR-1296-3p, miR-5189-3p, miR-6728-5p, miR-6729-5p, miR-6736-3p, miR-6738-5p, miR-6738-3p, miR-6740-3p, miR-6753-5p, miR-6754-3p, miR-6756-5p, miR-6759-5p, miR-6765-5p, miR-6768-5p, miR-6772-3p, miR-6773-3p, miR-6787-3p, miR-6790-5p, miR-6792-5p, miR-6798-5p, miR-6804-3p, miR-6805-5p, miR-6809-3p, miR-6816-5p, miR-6820-5p, miR-6821-5p, miR-6821-3p, miR-6822-5p, miR-6830-3p, miR-6832-3p, miR-6833-5p, miR-6833-3p, miR-6834-5p, miR-6780b-3p, miR-6854-3p, miR-6862-5p, miR-6867-5p, miR-6868-3p, miR-6871-5p, miR-6875-3p, miR-6877-5p, miR-6878-3p, miR-6882-3p, miR-6883-5p, miR-6883-3p, miR-6893-3p, miR-6894-3p, miR-7109-3p, miR-7152-5p, miR-7160-5p, miR-7843-5p, miR-8059, miR-8077, miR-7977, miR-4485-5p, miR-8485, miR-9718, miR-10396a-5p, miR-10398-5p, miR-10396b-5p, and miR-11400.

74. A method for analyzing a urine sample, comprising:
contacting a urine sample with a nanowire in a nanowire-embedded fluidic device having nanowires of metal oxide (e.g., zinc oxide);
extracting microRNAs captured on the nanowire; and
confirming whether the extracted microRNAs contain a specific microRNA by contacting the microRNA with a nucleic acid containing a sequence which hybridizes to the microRNA,
wherein the specific microRNAs is
selected from the group (GI) consisting of let-7e-5p, miR-23a-3p, miR-24-3p, miR-25-3p, miR-28-5p, miR-29a-3p, miR-30a-3p, miR-92a-3p, miR-29b-3p, miR-105-5p, miR-192-5p, miR-199a-5p, miR-129-5p, miR-30c-5p, miR-181a-5p, miR-181b-5p, miR-182-5p, miR-187-3p, miR-205-5p, miR-210-3p, miR-211-5p, miR-212-3p, miR-214-3p, miR-221-3p, miR-200b-3p, let-7i-5p, miR-15b-5p, miR-23b-3p, miR-27b-3p, miR-138-5p, miR-143-3p, miR-134-5p, miR-146a-5p, miR-154-5p, miR-184, miR-194-5p, miR-34b-5p, miR-34c-5p, miR-299-3p, miR-99b-5p, miR-296-5p, miR-361-5p, miR-370-3p, miR-371a-3p, miR-373-5p, miR-378a-3p, miR-381-3p, miR-382-5p, miR-340-3p, miR-330-3p, miR-328-3p, miR-342-3p, miR-323a-3p, miR-338-3p, miR-325, miR-346, miR-196b-5p, miR-422a, miR-449a, miR-200a-5p, miR-433-3p, miR-323b-5p, miR-484, miR-485-5p, miR-485-3p, miR-486-5p, miR-487a-3p, miR-488-5p, miR-489-3p, miR-491-5p, miR-511-5p, miR-202-3p, miR-492, miR-432-5p, miR-494-3p, miR-497-5p, miR-512-5p, miR-512-3p, miR-498-5p, miR-515-5p, miR-515-3p, miR-519e-3p, miR-520a-3p, miR-526b-5p, miR-525-5p, miR-525-3p, miR-517a-3p, miR-517b-3p, miR-519d-3p, miR-521, miR-520d-3p, miR-520g-3p, miR-516b-3p, miR-516a-3p, miR-517c-3p, miR-520h, miR-519a-3p, miR-500a-3p, miR-501-5p, miR-513a-5p, miR-506-3p, miR-509-3p, miR-510-5p, miR-299-5p, miR-18a-3p, miR-493-3p, miR-487b-3p, miR-551a, miR-554, miR-92b-3p, miR-557, miR-558, miR-564, miR-551b-3p, miR-572, miR-574-3p, miR-575, miR-579-3p, miR-584-5p, miR-589-3p, miR-550a-3p, miR-591, miR-593-5p, miR-595, miR-601, miR-602, miR-603, miR-604, miR-608, miR-609, miR-610, miR-612, miR-614, miR-615-3p, miR-616-5p, miR-617, miR-622, miR-623, miR-628-3p, miR-629-3p, miR-631, miR-632, miR-634, miR-635, miR-636, miR-637, miR-638, miR-639, miR-642a-5p, miR-644a, miR-645, miR-649, miR-650, miR-661, miR-663a, miR-449b-5p, miR-654-5p, miR-657, miR-658, miR-542-5p, miR-363-5p, miR-671-5p, miR-668-3p, miR-454-5p, miR-769-5p, miR-769-3p, miR-766-3p, miR-765, miR-770-5p, miR-675-5p, miR-297, let-7d-3p, miR-15a-3p, miR-16-1-3p, miR-21-3p, miR-23a-5p, miR-25-5p, miR-26a-1-3p, miR-26b-3p, miR-92a-2-5p, miR-16-2-3p, miR-139-3p, miR-7-1-3p, miR-181a-2-3p, miR-181c-3p, miR-183-3p, miR-187-5p, miR-214-5p, miR-222-5p, miR-200b-5p, miR-15b-3p, miR-30b-3p, miR-124-5p, miR-125b-1-3p, miR-135a-3p, miR-138-2-3p, miR-141-5p, miR-125a-3p, miR-125b-2-3p, miR-138-1-3p, miR-149-3p, miR-185-3p, miR-186-3p, miR-193a-5p, miR-200c-5p, miR-194-3p, miR-106b-3p, miR-29c-5p, miR-219a-2-3p, miR-34b-3p, miR-34c-3p, miR-99b-3p, miR-296-3p, miR-130b-5p, miR-371a-5p, miR-377-5p, miR-330-5p, miR-342-5p, miR-323a-5p, miR-151a-5p, miR-338-5p, miR-339-3p, miR-335-3p, miR-423-5p, miR-424-3p, miR-18b-3p, miR-20b-3p, miR-431-3p, miR-483-5p, miR-486-3p, miR-146b-3p, miR-193b-5p, miR-500a-5p, miR-505-5p, miR-508-5p, miR-532-3p, miR-92b-5p, miR-551b-5p, miR-574-5p, miR-589-5p, miR-550a-5p, miR-593-3p, miR-615-5p, miR-625-3p, miR-629-5p, miR-411-3p, miR-654-3p, miR-671-3p, miR-891a-5p, miR-300, miR-892a, miR-874-3p, miR-888-3p, miR-892b, miR-541-5p, miR-541-3p, miR-875-3p, miR-708-5p, miR-147b-3p, miR-744-5p, miR-744-3p, miR-885-5p, miR-885-3p, miR-877-5p, miR-877-3p, miR-887-3p, miR-665, miR-760, miR-920, miR-921, miR-935, miR-936, miR-937-3p, miR-938, miR-939-5p, miR-940, miR-943, miR-1224-5p, miR-1224-3p, miR-1225-5p, miR-1225-3p, miR-1226-5p, miR-1228-5p, miR-1228-3p, miR-1229-3p, miR-1231, miR-1233-3p, miR-1234-3p, miR-1236-3p, miR-1237-3p, miR-1238-3p, miR-513b-5p, miR-513c-5p, miR-320c, miR-1271-5p, miR-1298-5p, miR-1178-3p, miR-1181, miR-1182, miR-1184, miR-1203, miR-663b, miR-1204, miR-1207-5p, miR-1207-3p, miR-1285-3p, miR-1286, miR-1287-5p, miR-1291, miR-1293, miR-1294, miR-1297, miR-1303, miR-1304-5p, miR-1249-3p, miR-1250-5p, miR-1260a, miR-1263, miR-1265, miR-1266-5p, miR-1268a, miR-1269a, miR-1270, miR-1276, miR-1281, miR-1284, miR-1288-3p, miR-1292-5p, miR-1306-3p, miR-1307-3p, miR-1321, miR-1322, miR-1825, miR-1468-5p, miR-1469, miR-1470, miR-1471, miR-1538, miR-103b, miR-1908-5p, miR-1909-5p, miR-1909-3p, miR-1910-5p, miR-1911-3p, miR-1912-3p, miR-1913, miR-1914-3p, miR-1915-5p, miR-1915-3p, miR-103a-2-5p, miR-224-3p, miR-365a-5p, miR-196b-3p, miR-449b-3p, miR-2113, miR-1972, miR-1976, miR-2110, miR-151b, miR-449c-5p, miR-762, miR-761, miR-764, miR-548q, miR-2276-3p, miR-711, miR-718, miR-2681-5p, miR-2682-5p, miR-2682-3p, miR-449c-3p, miR-2861, miR-2909, miR-3122, miR-3124-5p, miR-3125, miR-3127-5p, miR-3130-3p, miR-3130-5p, miR-378b, miR-466, miR-3137, miR-3138, miR-3141, miR-3142, miR-1273c, miR-3147, miR-3148, miR-3150a-3p, miR-3151-5p, miR-3153, miR-3074-3p, miR-3154, miR-3155a, miR-3158-3p, miR-3160-3p, miR-3161, miR-3162-5p, miR-3163, miR-3165, miR-1260b, miR-3170, miR-3173-3p, miR-1193, miR-3174, miR-3175, miR-3176, miR-3178, miR-3180-3p, miR-3185, miR-3186-5p, miR-3186-3p, miR-3187-3p, miR-3189-3p, miR-320e, miR-3191-3p, miR-3192-5p, miR-3193, miR-3194-5p, miR-3195, miR-3196, miR-3197, miR-3198, miR-3199, miR-3200-3p, miR-514b-5p, miR-514b-3p, miR-3126-3p, miR-3065-3p, miR-4296, miR-4297, miR-378c, miR-4293, miR-4294, miR-4299, miR-4298, miR-4300, miR-4304, miR-4303, miR-4305, miR-4308, miR-4311, miR-4315, miR-4316, miR-4314, miR-4318, miR-4319, miR-4317, miR-4322, miR-4321, miR-4323, miR-4324, miR-4257, miR-4258, miR-4259, miR-4260, miR-4253, miR-4254, miR-4255, miR-4252, miR-4326, miR-4327, miR-4261, miR-4265, miR-4262, miR-4268, miR-4269, miR-4264, miR-4271, miR-4273, miR-4276, miR-4274, miR-4279, miR-4278, miR-4280, miR-4285, miR-4283, miR-4287, miR-4289, miR-4329, miR-4328, miR-2277-5p, miR-3605-5p, miR-3610, miR-3614-5p, miR-3615, miR-3616-3p, miR-3619-5p, miR-3620-3p, miR-3621, miR-3622a-5p, miR-3622a-3p, miR-3622b-5p, miR-3646, miR-3648, miR-3649, miR-3650, miR-3651, miR-3652, miR-3654, miR-3655, miR-3657, miR-3659, miR-3660, miR-3661, miR-3663-5p, miR-3663-3p, miR-3665, miR-3666, miR-3667-5p, miR-3675-5p, miR-3675-3p, miR-3677-3p, miR-3678-3p, miR-3679-5p, miR-3679-3p, miR-3680-3p, miR-3681-3p, miR-3682-3p, miR-3685, miR-3689a-3p, miR-3690, miR-3691-5p, miR-3692-5p, miR-3713, miR-3180, miR-3907, miR-3689b-3p, miR-3689c, miR-3911, miR-3913-5p, miR-3917, miR-3918, miR-3919, miR-3150b-3p, miR-3925-5p, miR-676-5p, miR-3928-3p, miR-3929, miR-3934-5p, miR-3936, miR-3937, miR-3940-3p, miR-3944-3p, miR-374c-5p, miR-642b-3p, miR-550b-3p, miR-1268b, miR-378e, miR-4420, miR-4421, miR-378g, miR-4425, miR-4428, miR-4429, miR-4430, miR-4431, miR-4433a-3p, miR-4435, miR-4436a, miR-4437, miR-4438, miR-4439, miR-4440, miR-4441, miR-4444, miR-4445-3p, miR-4446-3p, miR-4447, miR-4448, miR-4449, miR-4450, miR-4451, miR-4453, miR-4454, miR-4455, miR-4456, miR-4458, miR-3135b, miR-4462, miR-4463, miR-4465, miR-4466, miR-4467, miR-4470, miR-4472, miR-4473, miR-4474-3p, miR-4475, miR-4476, miR-4478, miR-3689d, miR-3689f, miR-4479, miR-3155b, miR-4481, miR-4483, miR-4484, miR-4486, miR-4487, miR-4488, miR-4489, miR-4491, miR-4492, miR-4494, miR-4496, miR-4497, miR-4498, miR-4502, miR-4505, miR-4506, miR-2392, miR-4507, miR-4508, miR-4510, miR-4511, miR-4513, miR-4514, miR-4515, miR-4516, miR-4521, miR-4523, miR-4524a-5p, miR-4526, miR-4527, miR-4529-3p, miR-4530, miR-4533, miR-378i, miR-4535, miR-1587, miR-4537, miR-4538, miR-4539, miR-4540, miR-3120-5p, miR-3129-3p, miR-3145-5p, miR-3150a-5p, miR-3152-5p, miR-3074-5p, miR-3157-3p, miR-3158-5p, miR-3160-5p, miR-3173-5p, miR-3187-5p, miR-3691-3p, miR-3913-3p, miR-3922-5p, miR-3925-3p, miR-3940-5p, miR-3944-5p, miR-4423-5p, miR-4520-5p, miR-4529-5p, miR-3960, miR-3975, miR-4634, miR-4635, miR-4638-5p, miR-4639-3p, miR-4640-5p, miR-4641, miR-4642, miR-4644, miR-4645-3p, miR-4646-5p, miR-4646-3p, miR-4647, miR-4648, miR-4649-5p, miR-4650-3p, miR-4651, miR-4652-5p, miR-4653-5p, miR-4654, miR-4655-5p, miR-4656, miR-4657, miR-4658, miR-4661-3p, miR-4664-5p, miR-4664-3p, miR-4665-5p, miR-4665-3p, miR-4667-5p, miR-4667-3p, miR-219b-5p, miR-4669, miR-4670-5p, miR-4672, miR-4673, miR-4674, miR-4676-5p, miR-4676-3p, miR-4681, miR-4682, miR-4684-5p, miR-4684-3p, miR-4685-5p, miR-4685-3p, miR-4686, miR-4687-5p, miR-4687-3p, miR-1343-3p, miR-4688, miR-4690-5p, miR-4691-5p, miR-4691-3p, miR-4692, miR-4694-3p, miR-4695-5p, miR-4697-5p, miR-4697-3p, miR-4698, miR-4700-5p, miR-4700-3p, miR-4701-5p, miR-4701-3p, miR-4706, miR-4707-5p, miR-4707-3p, miR-4708-5p, miR-4708-3p, miR-4709-5p, miR-4709-3p, miR-4710, miR-4711-3p, miR-4712-5p, miR-4713-5p, miR-4713-3p, miR-4716-5p, miR-4716-3p, miR-3529-5p, miR-4717-5p, miR-4717-3p, miR-4718, miR-4721, miR-4722-5p, miR-4722-3p, miR-4723-3p, miR-4724-5p, miR-4724-3p, miR-4725-5p, miR-4725-3p, miR-4726-5p, miR-4726-3p, miR-4727-3p, miR-4728-3p, miR-4730, miR-4731-5p, miR-4731-3p, miR-4732-5p, miR-4732-3p, miR-4733-5p, miR-4733-3p, miR-4734, miR-4736, miR-4737, miR-3064-5p, miR-3064-3p, miR-4738-3p, miR-4739, miR-4740-5p, miR-4741, miR-4743-5p, miR-122b-3p, miR-4745-5p, miR-4746-3p, miR-4747-5p, miR-4747-3p, miR-4748, miR-4749-5p, miR-4749-3p, miR-4750-5p, miR-4753-3p, miR-371b-5p, miR-371b-3p, miR-4754, miR-4755-3p, miR-4756-5p, miR-4756-3p, miR-4758-5p, miR-4758-3p, miR-4759, miR-4761-3p, miR-4763-5p, miR-4763-3p, miR-4764-3p, miR-4766-5p, miR-4767, miR-4768-5p, miR-4768-3p, miR-4769-5p, miR-4769-3p, miR-4773, miR-4776-5p, miR-4776-3p, miR-4778-5p, miR-4780, miR-4436b-5p, miR-4436b-3p, miR-4783-5p, miR-4783-3p, miR-4784, miR-4785, miR-2467-3p, miR-4786-5p, miR-4786-3p, miR-4787-5p, miR-4787-3p, miR-4788, miR-4793-5p, miR-4793-3p, miR-4794, miR-4796-5p, miR-4797-3p, miR-4800-5p, miR-4800-3p, miR-4801, miR-4804-3p, miR-642a-3p, miR-550a-3-5p, miR-4433a-5p, miR-4482-3p, miR-4999-5p, miR-4999-3p, miR-5000-5p, miR-5001-5p, miR-5001-3p, miR-5002-5p, miR-5002-3p, miR-5004-5p, miR-5004-3p, miR-5006-5p, miR-5006-3p, miR-5007-5p, miR-5008-5p, miR-5010-5p, miR-5088-5p, miR-5090, miR-5091, miR-5093, miR-5187-5p, miR-5188, miR-5189-5p, miR-5190, miR-5192, miR-5194, miR-5196-5p, miR-5196-3p, miR-5197-5p, miR-4524b-5p, miR-4524b-3p, miR-5571-3p, miR-5100, miR-5572, miR-664b-5p, miR-664b-3p, miR-5580-5p, miR-5580-3p, miR-5581-3p, miR-5584-3p, miR-5585-3p, miR-1295b-3p, miR-5588-5p, miR-5588-3p, miR-5589-5p, miR-5591-3p, miR-5684, miR-5685, miR-5681b, miR-5690, miR-5694, miR-5696, miR-5698, miR-5702, miR-5703, miR-5704, miR-5705, miR-5708, miR-197-5p, miR-204-3p, miR-211-3p, miR-212-5p, miR-301a-5p, miR-345-3p, miR-584-3p, miR-652-5p, miR-660-3p, miR-1185-2-3p, miR-766-5p, miR-1285-5p, miR-1304-3p, miR-1247-3p, miR-1255b-2-3p, miR-1306-5p, miR-3184-3p, miR-3191-5p, miR-642b-5p, miR-550b-2-5p, miR-365b-5p, miR-1185-1-3p, miR-3190-3p, miR-216a-3p, miR-381-5p, miR-495-5p, miR-503-3p, miR-937-5p, miR-939-3p, miR-1227-5p, miR-1229-5p, miR-1233-5p, miR-1236-5p, miR-1237-5p, miR-1238-5p, miR-3617-3p, miR-3620-5p, miR-3927-5p, miR-3934-3p, miR-4632-5p, miR-4743-3p, miR-5089-3p, miR-5739, miR-5787, miR-6068, miR-6069, miR-6071, miR-6072, miR-6074, miR-6075, miR-6076, miR-6078, miR-6080, miR-6081, miR-6083, miR-6085, miR-6086, miR-6088, miR-6089, miR-6090, miR-6124, miR-6125, miR-6126, miR-6127, miR-6129, miR-6130, miR-6131, miR-6132, miR-6133, miR-6134, miR-6165, miR-6499-5p, miR-6499-3p, miR-548ay-3p, miR-6500-5p, miR-6500-3p, miR-6501-5p, miR-6501-3p, miR-6502-5p, miR-6503-3p, miR-6504-5p, miR-6504-3p, miR-6505-3p, miR-6506-5p, miR-6508-5p, miR-6509-5p, miR-6510-5p, miR-6511a-5p, miR-6512-3p, miR-6513-5p, miR-6513-3p, miR-6514-5p, miR-6514-3p, miR-6515-5p, miR-6515-3p, miR-6715a-3p, miR-6715b-5p, miR-6716-5p, miR-6716-3p, miR-6717-5p, miR-6511b-5p, miR-6511b-3p, miR-6718-5p, miR-6719-3p, miR-6721-5p, miR-6722-5p, miR-6722-3p, miR-6724-5p, miR-892c-5p, miR-892c-3p, miR-208a-5p, miR-210-5p, miR-128-1-5p, miR-134-3p, miR-370-5p, miR-328-5p, miR-433-5p, miR-487a-5p, miR-489-5p, miR-494-5p, miR-504-3p, miR-487b-5p, miR-579-5p, miR-585-5p, miR-598-5p, miR-619-5p, miR-656-5p, miR-668-5p, miR-1296-3p, miR-1301-5p, miR-1298-3p, miR-891a-3p, miR-874-5p, miR-889-5p, miR-887-5p, miR-216b-3p, miR-942-3p, miR-1180-5p, miR-1287-3p, miR-1251-3p, miR-1266-3p, miR-1288-5p, miR-1908-3p, miR-1910-3p, miR-3151-3p, miR-3192-3p, miR-500b-3p, miR-3928-5p, miR-1343-5p, miR-5088-3p, miR-5189-3p, miR-6720-5p, miR-6726-5p, miR-6726-3p, miR-6727-5p, miR-6727-3p, miR-6728-5p, miR-6728-3p, miR-6729-5p, miR-6729-3p, miR-6730-5p, miR-6730-3p, miR-6731-5p, miR-6732-5p, miR-6732-3p, miR-6733-3p, miR-6734-5p, miR-6735-5p, miR-6735-3p, miR-6736-3p, miR-6737-5p, miR-6738-5p, miR-6738-3p, miR-6739-3p, miR-6740-5p, miR-6740-3p, miR-6741-5p, miR-6741-3p, miR-6742-5p, miR-6743-5p, miR-6743-3p, miR-6744-3p, miR-6747-5p, miR-6747-3p, miR-6748-5p, miR-6748-3p, miR-6749-5p, miR-6749-3p, miR-6750-5p, miR-6750-3p, miR-6751-5p, miR-6751-3p, miR-6752-5p, miR-6752-3p, miR-6753-5p, miR-6754-5p, miR-6754-3p, miR-6755-5p, miR-6755-3p, miR-6756-5p, miR-6756-3p, miR-6758-5p, miR-6758-3p, miR-6759-5p, miR-6759-3p, miR-6760-5p, miR-6760-3p, miR-6761-5p, miR-6762-5p, miR-6762-3p, miR-6763-5p, miR-6763-3p, miR-6764-5p, miR-6764-3p, miR-6765-5p, miR-6766-5p, miR-6766-3p, miR-6767-5p, miR-6768-5p, miR-6769a-5p, miR-6769a-3p, miR-6770-5p, miR-6770-3p, miR-6771-5p, miR-6772-5p, miR-6772-3p, miR-6773-3p, miR-6774-5p, miR-6774-3p, miR-6775-5p, miR-6776-5p, miR-6776-3p, miR-6777-5p, miR-6777-3p, miR-6778-5p, miR-6778-3p, miR-6779-5p, miR-6779-3p, miR-6781-5p, miR-6781-3p, miR-6782-5p, miR-6782-3p, miR-6783-5p, miR-6783-3p, miR-6784-5p, miR-6784-3p, miR-6785-3p, miR-6786-5p, miR-6786-3p, miR-6787-5p, miR-6787-3p, miR-6788-5p, miR-6789-5p, miR-6789-3p, miR-6790-5p, miR-6791-5p, miR-6791-3p, miR-6792-5p, miR-6792-3p, miR-6793-5p, miR-6794-5p, miR-6796-5p, miR-6796-3p, miR-6797-5p, miR-6797-3p, miR-6798-5p, miR-6798-3p, miR-6799-5p, miR-6800-5p, miR-6800-3p, miR-6801-5p, miR-6801-3p, miR-6802-3p, miR-6803-5p, miR-6803-3p, miR-6804-5p, miR-6804-3p, miR-6805-5p, miR-6805-3p, miR-6806-5p, miR-6807-5p, miR-6808-5p, miR-6809-5p, miR-6809-3p, miR-6810-5p, miR-6810-3p, miR-6812-5p, miR-6813-5p, miR-6813-3p, miR-6814-5p, miR-6815-3p, miR-6816-5p, miR-6816-3p, miR-6817-5p, miR-6817-3p, miR-6818-3p, miR-6819-3p, miR-6820-5p, miR-6820-3p, miR-6821-5p, miR-6821-3p, miR-6822-5p, miR-6822-3p, miR-6823-5p, miR-6823-3p, miR-6824-5p, miR-6824-3p, miR-6825-5p, miR-6825-3p, miR-6826-3p, miR-6827-5p, miR-6829-5p, miR-6829-3p, miR-6830-5p, miR-6830-3p, miR-6831-5p, miR-6832-5p, miR-6832-3p, miR-6833-5p, miR-6833-3p, miR-6834-5p, miR-6780b-5p, miR-6780b-3p, miR-6836-5p, miR-6836-3p, miR-6837-5p, miR-6838-5p, miR-6839-5p, miR-6840-3p, miR-6842-5p, miR-6842-3p, miR-6843-3p, miR-6845-5p, miR-6845-3p, miR-6846-5p, miR-6846-3p, miR-6847-5p, miR-6847-3p, miR-6848-5p, miR-6848-3p, miR-6849-5p, miR-6849-3p, miR-6850-5p, miR-6850-3p, miR-6851-5p, miR-6852-5p, miR-6852-3p, miR-6853-5p, miR-6854-3p, miR-6855-5p, miR-6855-3p, miR-6856-5p, miR-6856-3p, miR-6857-5p, miR-6858-5p, miR-6858-3p, miR-6859-5p, miR-6859-3p, miR-6769b-5p, miR-6860, miR-6861-5p, miR-6861-3p, miR-6862-5p, miR-6862-3p, miR-6863, miR-6866-3p, miR-6867-5p, miR-6867-3p, miR-6868-5p, miR-6868-3p, miR-6869-5p, miR-6870-5p, miR-6870-3p, miR-6871-5p, miR-6873-5p, miR-6874-5p, miR-6874-3p, miR-6875-5p, miR-6875-3p, miR-6876-5p, miR-6876-3p, miR-6877-5p, miR-6877-3p, miR-6878-3p, miR-6879-5p, miR-6879-3p, miR-6880-5p, miR-6880-3p, miR-6881-5p, miR-6882-3p, miR-6883-5p, miR-6883-3p, miR-6884-5p, miR-6884-3p, miR-6885-3p, miR-6886-5p, miR-6886-3p, miR-6887-3p, miR-6888-5p, miR-6889-5p, miR-6889-3p, miR-6890-5p, miR-6890-3p, miR-6891-5p, miR-6891-3p, miR-6892-5p, miR-6892-3p, miR-6893-5p, miR-6893-3p, miR-6894-5p, miR-6894-3p, miR-6895-5p, miR-6895-3p, miR-7106-5p, miR-7106-3p, miR-7107-5p, miR-7108-5p, miR-7108-3p, miR-7109-5p, miR-7109-3p, miR-7110-5p, miR-7110-3p, miR-7111-5p, miR-7111-3p, miR-7112-5p, miR-7113-5p, miR-7113-3p, miR-7114-5p, miR-7114-3p, miR-7150, miR-7151-5p, miR-7151-3p, miR-7152-5p, miR-7154-3p, miR-7155-5p, miR-7155-3p, miR-7156-5p, miR-7156-3p, miR-7158-5p, miR-7160-5p, miR-7162-3p, miR-7515, miR-7702, miR-7704, miR-7706, miR-7843-5p, miR-4433b-5p, miR-4433b-3p, miR-1273h-5p, miR-6516-5p, miR-7844-5p, miR-7845-5p, miR-7846-3p, miR-7847-3p, miR-7848-3p, miR-7850-5p, miR-7851-3p, miR-7853-5p, miR-7854-3p, miR-7856-5p, miR-8052, miR-8055, miR-8057, miR-8059, miR-8060, miR-8062, miR-8063, miR-8064, miR-8069, miR-8070, miR-8071, miR-8072, miR-8073, miR-8074, miR-8075, miR-8077, miR-8078, miR-8079, miR-8080, miR-8081, miR-8082, miR-8083, miR-8085, miR-8089, miR-128-2-5p, miR-7973, miR-7974, miR-7975, miR-7976, miR-7977, miR-1249-5p, miR-4485-5p, miR-8485, miR-9500, miR-103a-1-5p, miR-217-3p, miR-135a-2-3p, miR-375-5p, miR-498-3p, miR-9718, miR-9899, miR-9902, miR-1843, miR-9986, miR-10392-5p, miR-10392-3p, miR-10394-5p, miR-10394-3p, miR-10395-5p, miR-10396a-5p, miR-10396a-3p, miR-10397-5p, miR-10398-5p, miR-10398-3p, miR-10400-5p, miR-10400-3p, miR-10401-5p, miR-10401-3p, miR-10396b-5p, miR-10396b-3p, miR-10522-5p, miR-10523-5p, miR-10527-5p, miR-11181-3p, miR-11399, miR-11400, miR-11401, miR-3059-5p, miR-3059-3p, miR-3085-3p, miR-6529-5p, miR-6529-3p, miR-9851-5p, miR-12114, miR-12115, miR-12116, miR-12117, miR-12118, miR-12119, miR-12120, miR-12121, miR-12122, miR-12124, miR-12125, miR-12127, miR-12128, and miR-12131.

75. A method for analyzing a urine sample, comprising:
contacting a urine sample with a nanowire in a nanowire-embedded fluidic device having nanowires of metal oxide;
extracting microRNAs captured on the nanowire; and
confirming whether the extracted microRNAs contain a specific microRNA,
wherein the specific microRNAs is
selected from the group (Hemo) consisting of miR-105-5p, miR-192-5p, miR-198, miR-129-5p, miR-187-3p, miR-210-3p, miR-212-3p, miR-214-3p, miR-200b-3p, miR-125b-5p, miR-138-5p, miR-134-5p, miR-184, miR-185-5p, miR-206, miR-106b-5p, miR-34c-5p, miR-99b-5p, miR-296-5p, miR-130b-3p, miR-302c-5p, miR-370-3p, miR-373-3p, miR-378a-3p, miR-382-5p, miR-340-3p, miR-342-3p, miR-337-3p, miR-323a-3p, miR-324-5p, miR-346, miR-422a, miR-425-3p, miR-369-5p, miR-323b-5p, miR-409-3p, miR-412-3p, miR-485-5p, miR-485-3p, miR-488-5p, miR-491-5p, miR-202-3p, miR-492, miR-432-3p, miR-494-3p, miR-512-5p, miR-512-3p, miR-498-5p, miR-520e-3p, miR-515-3p, miR-519c-5p, miR-523-5p, miR-518e-5p, miR-522-5p, miR-519a-5p, miR-519b-5p, miR-526b-5p, miR-525-5p, miR-518f-3p, miR-520b-3p, miR-526a-5p, miR-520c-5p, miR-518d-5p, miR-518c-5p, miR-519d-3p, miR-520d-5p, miR-516b-3p, miR-516a-3p, miR-501-5p, miR-513a-5p, miR-510-5p, miR-18a-3p, miR-551a, miR-92b-3p, miR-558, miR-564, miR-551b-3p, miR-572, miR-574-3p, miR-575, miR-583, miR-550a-3p, miR-595, miR-596, miR-601, miR-602, miR-604, miR-608, miR-610, miR-612, miR-614, miR-615-3p, miR-616-5p, miR-617, miR-622, miR-623, miR-629-3p, miR-632, miR-636, miR-637, miR-638, miR-639, miR-646, miR-650, miR-661, miR-663a, miR-654-5p, miR-657, miR-658, miR-421, miR-542-5p, miR-363-5p, miR-668-3p, miR-769-3p, miR-766-3p, miR-770-5p, miR-297, let-7d-3p, miR-15a-3p, miR-21-3p, miR-23a-5p, miR-25-5p, miR-26b-3p, miR-92a-2-5p, miR-30c-2-3p, miR-139-3p, miR-183-3p, miR-187-5p, miR-214-5p, miR-200b-5p, miR-30b-3p, miR-130a-5p, miR-135a-3p, miR-138-2-3p, miR-125a-3p, miR-125b-2-3p, miR-138-1-3p, miR-149-3p, miR-150-3p, miR-185-3p, miR-186-3p, miR-193a-5p, miR-200c-5p, miR-155-3p, miR-194-3p, miR-34b-3p, miR-34c-3p, miR-296-3p, miR-130b-5p, miR-361-3p, miR-302d-5p, miR-377-5p, miR-342-5p, miR-323a-5p, miR-338-5p, miR-424-3p, miR-18b-3p, miR-20b-3p, miR-431-3p, miR-483-5p, miR-486-3p, miR-193b-5p, miR-505-5p, miR-508-5p, miR-92b-5p, miR-551b-5p, miR-574-5p, miR-550a-5p, miR-593-3p, miR-615-5p, miR-625-3p, miR-629-5p, miR-671-3p, miR-891a-5p, miR-300, miR-892b, miR-541-5p, miR-541-3p, miR-744-5p, miR-885-5p, miR-885-3p, miR-877-5p, miR-887-3p, miR-665, miR-760, miR-920, miR-921, miR-935, miR-936, miR-937-3p, miR-938, miR-939-5p, miR-940, miR-943, miR-1224-5p, miR-1224-3p, miR-1225-5p, miR-1225-3p, miR-1226-5p, miR-1228-5p, miR-1228-3p, miR-1231, miR-1233-3p, miR-1237-3p, miR-1238-3p, miR-513b-5p, miR-320b, miR-320c, miR-1271-5p, miR-1301-3p, miR-1298-5p, miR-1178-3p, miR-1181, miR-1182, miR-1184, miR-1203, miR-1204, miR-1207-5p, miR-1285-3p, miR-1293, miR-1303, miR-1249-3p, miR-1250-5p, miR-548g-3p, miR-1266-5p, miR-1268a, miR-1269a, miR-1270, miR-1276, miR-1292-5p, miR-1306-3p, miR-1307-3p, miR-1321, miR-1468-5p, miR-675-3p, miR-1469, miR-1470, miR-1471, miR-1538, miR-1908-5p, miR-1909-5p, miR-1909-3p, miR-1910-5p, miR-1912-3p, miR-1913, miR-1914-3p, miR-1915-3p, miR-103a-2-5p, miR-365a-5p, miR-196b-3p, miR-449b-3p, miR-1972, miR-2110, miR-449c-5p, miR-762, miR-670-5p, miR-761, miR-764, miR-2116-5p, miR-548q, miR-2276-3p, miR-2277-3p, miR-711, miR-718, miR-2682-5p, miR-449c-3p, miR-3122, miR-3124-5p, miR-3126-5p, miR-3130-3p, miR-3130-5p, miR-3131, miR-3132, miR-3133, miR-466, miR-3137, miR-3138, miR-3141, miR-3147, miR-3150a-3p, miR-3151-5p, miR-3153, miR-3074-3p, miR-3155a, miR-3158-3p, miR-3161, miR-3162-5p, miR-3163, miR-3164, miR-3165, miR-1260b, miR-3173-3p, miR-1193, miR-3176, miR-3177-3p, miR-3178, miR-3180-3p, miR-3185, miR-3186-3p, miR-3187-3p, miR-3189-3p, miR-3191-3p, miR-3192-5p, miR-3195, miR-3196, miR-3197, miR-3198, miR-3199, miR-3202, miR-3126-3p, miR-4296, miR-4297, miR-4294, miR-4299, miR-4300, miR-4304, miR-4305, miR-4306, miR-4307, miR-4308, miR-4313, miR-4316, miR-4314, miR-4319, miR-4317, miR-4322, miR-4321, miR-4323, miR-4324, miR-4257, miR-4258, miR-4259, miR-4260, miR-4253, miR-4251, miR-4255, miR-4252, miR-4326, miR-4327, miR-4261, miR-4265, miR-4266, miR-4267, miR-4262, miR-4268, miR-4269, miR-4276, miR-4274, miR-4280, miR-4285, miR-4289, miR-2277-5p, miR-3200-5p, miR-3605-5p, miR-3605-3p, miR-3610, miR-3612, miR-3614-5p, miR-3616-3p, miR-3619-5p, miR-3621, miR-3622a-5p, miR-3622a-3p, miR-3622b-5p, miR-3646, miR-3648, miR-3650, miR-3651, miR-3652, miR-3654, miR-3655, miR-3659, miR-3660, miR-3663-5p, miR-3663-3p, miR-3665, miR-3667-5p, miR-3667-3p, miR-3675-3p, miR-3677-3p, miR-3679-5p, miR-3679-3p, miR-3682-3p, miR-3685, miR-3689a-3p, miR-3691-5p, miR-3713, miR-3714, miR-3180, miR-3689b-3p, miR-3689c, miR-3917, miR-3918, miR-3150b-3p, miR-3925-5p, miR-3928-3p, miR-3934-5p, miR-3935, miR-3936, miR-3937, miR-3944-3p, miR-3945, miR-550b-3p, miR-1268b, miR-4421, miR-4428, miR-4429, miR-4430, miR-4431, miR-4433a-3p, miR-4435, miR-4436a, miR-4439, miR-4440, miR-4441, miR-4443, miR-4444, miR-4446-3p, miR-4447, miR-4448, miR-4449, miR-4450, miR-4451, miR-4453, miR-4454, miR-4455, miR-4456, miR-4458, miR-3135b, miR-4462, miR-4463, miR-4465, miR-4466, miR-4467, miR-4470, miR-4472, miR-4474-3p, miR-4475, miR-4476, miR-3689d, miR-4479, miR-3155b, miR-4481, miR-4483, miR-4484, miR-4485-3p, miR-4486, miR-4487, miR-4488, miR-4489, miR-4492, miR-4496, miR-4497, miR-4498, miR-4499, miR-4502, miR-4505, miR-4506, miR-2392, miR-4507, miR-4508, miR-4510, miR-4512, miR-4513, miR-4514, miR-4515, miR-4516, miR-4521, miR-4525, miR-4526, miR-4530, miR-4531, miR-4533, miR-4534, miR-4535, miR-1587, miR-4538, miR-4539, miR-4540, miR-3120-5p, miR-3150a-5p, miR-3074-5p, miR-3156-3p, miR-3160-5p, miR-3162-3p, miR-3173-5p, miR-3187-5p, miR-3619-3p, miR-3922-5p, miR-3940-5p, miR-3944-5p, miR-4520-5p, miR-4529-5p, miR-3960, miR-4634, miR-4635, miR-4638-5p, miR-4639-3p, miR-4640-5p, miR-4642, miR-4646-5p, miR-4647, miR-4648, miR-4650-3p, miR-4651, miR-4652-5p, miR-4653-5p, miR-4653-3p, miR-4654, miR-4655-5p, miR-4657, miR-4658, miR-4664-5p, miR-4664-3p, miR-4665-5p, miR-4665-3p, miR-4667-5p, miR-219b-5p, miR-4669, miR-4670-5p, miR-4673, miR-4674, miR-4676-5p, miR-4681, miR-4682, miR-4684-5p, miR-4684-3p, miR-4685-3p, miR-4687-5p, miR-1343-3p, miR-4688, miR-4690-5p, miR-4690-3p, miR-4691-5p, miR-4692, miR-4695-5p, miR-4700-5p, miR-4701-3p, miR-4706, miR-4707-5p, miR-4707-3p, miR-4708-5p, miR-4708-3p, miR-203b-5p, miR-4710, miR-4711-3p, miR-4714-5p, miR-4716-5p, miR-4716-3p, miR-4717-5p, miR-4717-3p, miR-4720-5p, miR-4721, miR-4722-3p, miR-4724-5p, miR-4725-5p, miR-4725-3p, miR-4726-3p, miR-4727-3p, miR-4728-5p, miR-4728-3p, miR-4730, miR-4731-3p, miR-4732-5p, miR-4732-3p, miR-4734, miR-4736, miR-3064-5p, miR-3064-3p, miR-4738-3p, miR-4740-5p, miR-4740-3p, miR-4741, miR-4743-5p, miR-122b-3p, miR-4745-5p, miR-4746-3p, miR-4747-5p, miR-4747-3p, miR-4748, miR-4749-5p, miR-4749-3p, miR-4750-5p, miR-371b-5p, miR-4754, miR-4755-3p, miR-4758-5p, miR-4763-3p, miR-4764-3p, miR-4767, miR-4769-5p, miR-4776-5p, miR-4776-3p, miR-4780, miR-4436b-5p, miR-4436b-3p, miR-4781-5p, miR-4783-5p, miR-4783-3p, miR-4785, miR-2467-3p, miR-4786-5p, miR-4786-3p, miR-4787-5p, miR-4793-3p, miR-4800-5p, miR-4800-3p, miR-4801, miR-4804-3p, miR-550a-3-5p, miR-4433a-5p, miR-4482-3p, miR-4999-5p, miR-5001-5p, miR-5001-3p, miR-5002-3p, miR-5004-5p, miR-5004-3p, miR-5006-5p, miR-5009-3p, miR-5010-5p, miR-5088-5p, miR-5090, miR-5093, miR-5187-5p, miR-5188, miR-5189-5p, miR-5190, miR-5193, miR-5195-5p, miR-5196-5p, miR-5571-3p, miR-5100, miR-5572, miR-664b-5p, miR-664b-3p, miR-5580-3p, miR-5581-3p, miR-5584-3p, miR-5585-3p, miR-5587-5p, miR-5588-5p, miR-5588-3p, miR-5589-5p, miR-5685, miR-5681b, miR-5698, miR-5703, miR-5705, miR-197-5p, miR-204-3p, miR-211-3p, miR-301a-5p, miR-345-3p, miR-584-3p, miR-659-5p, miR-1185-2-3p, miR-766-5p, miR-873-3p, miR-1285-5p, miR-1247-3p, miR-1255b-2-3p, miR-3191-5p, miR-642b-5p, miR-550b-2-5p, miR-365b-5p, miR-1185-1-3p, miR-3190-3p, miR-937-5p, miR-1227-5p, miR-1236-5p, miR-1237-5p, miR-1238-5p, miR-1292-3p, miR-3617-3p, miR-3620-5p, miR-3934-3p, miR-4632-5p, miR-4750-3p, miR-5739, miR-5787, miR-6068, miR-6070, miR-6071, miR-6072, miR-6074, miR-6075, miR-6076, miR-6080, miR-6081, miR-6083, miR-6084, miR-6085, miR-6086, miR-6089, miR-6090, miR-6124, miR-6125, miR-6126, miR-6127, miR-6129, miR-6130, miR-6131, miR-6132, miR-6133, miR-6134, miR-6165, miR-6500-5p, miR-6500-3p, miR-6501-5p, miR-6501-3p, miR-6503-3p, miR-6504-3p, miR-6508-5p, miR-6509-3p, miR-6511a-5p, miR-6512-3p, miR-6513-5p, miR-6514-5p, miR-6514-3p, miR-6515-5p, miR-6515-3p, miR-6715b-5p, miR-6716-3p, miR-6717-5p, miR-6511b-5p, miR-6718-5p, miR-6721-5p, miR-6722-5p, miR-6722-3p, miR-6724-5p, miR-210-5p, miR-128-1-5p, miR-134-3p, miR-433-5p, miR-487a-5p, miR-489-5p, miR-504-3p, miR-487b-5p, miR-585-5p, miR-605-3p, miR-619-5p, miR-656-5p, miR-668-5p, miR-1296-3p, miR-1301-5p, miR-1298-3p, miR-874-5p, miR-887-5p, miR-1180-5p, miR-548j-3p, miR-1250-3p, miR-1266-3p, miR-1288-5p, miR-1908-3p, miR-1910-3p, miR-3192-3p, miR-500b-3p, miR-3928-5p, miR-1343-5p, miR-5088-3p, miR-5189-3p, miR-6720-5p, miR-6726-5p, miR-6727-5p, miR-6728-5p, miR-6728-3p, miR-6729-5p, miR-6729-3p, miR-6730-5p, miR-6730-3p, miR-6731-5p, miR-6731-3p, miR-6732-5p, miR-6734-5p, miR-6735-3p, miR-6736-5p, miR-6736-3p, miR-6737-5p, miR-6738-5p, miR-6738-3p, miR-6740-5p, miR-6740-3p, miR-6741-5p, miR-6742-5p, miR-6743-5p, miR-6745, miR-6746-5p, miR-6747-5p, miR-6748-5p, miR-6749-5p, miR-6750-5p, miR-6751-5p, miR-6752-5p, miR-6752-3p, miR-6753-5p, miR-6753-3p, miR-6754-3p, miR-6756-5p, miR-6758-5p, miR-6759-5p, miR-6760-5p, miR-6760-3p, miR-6761-5p, miR-6761-3p, miR-6762-5p, miR-6762-3p, miR-6763-5p, miR-6763-3p, miR-6765-5p, miR-6765-3p, miR-6766-5p, miR-6766-3p, miR-6767-5p, miR-6768-5p, miR-6769a-5p, miR-6770-5p, miR-6770-3p, miR-6772-5p, miR-6772-3p, miR-6773-3p, miR-6774-3p, miR-6776-5p, miR-6776-3p, miR-6777-5p, miR-6777-3p, miR-6778-5p, miR-6778-3p, miR-6779-3p, miR-6780a-5p, miR-6780a-3p, miR-6781-5p, miR-6781-3p, miR-6782-5p, miR-6783-5p, miR-6783-3p, miR-6784-5p, miR-6784-3p, miR-6785-3p, miR-6786-5p, miR-6787-5p, miR-6787-3p, miR-6788-5p, miR-6789-5p, miR-6789-3p, miR-6790-5p, miR-6791-5p, miR-6791-3p, miR-6792-5p, miR-6792-3p, miR-6793-5p, miR-6794-5p, miR-6795-5p, miR-6795-3p, miR-6796-5p, miR-6796-3p, miR-6797-5p, miR-6798-5p, miR-6798-3p, miR-6800-5p, miR-6800-3p, miR-6803-3p, miR-6804-5p, miR-6804-3p, miR-6805-5p, miR-6805-3p, miR-6806-5p, miR-6808-5p, miR-6809-5p, miR-6809-3p, miR-6810-5p, miR-6810-3p, miR-6812-5p, miR-6813-5p, miR-6813-3p, miR-6814-5p, miR-6815-5p, miR-6816-5p, miR-6816-3p, miR-6817-5p, miR-6819-3p, miR-6820-5p, miR-6820-3p, miR-6821-5p, miR-6821-3p, miR-6822-5p, miR-6823-5p, miR-6824-5p, miR-6825-5p, miR-6827-5p, miR-6829-5p, miR-6829-3p, miR-6830-5p, miR-6830-3p, miR-6831-5p, miR-6832-5p, miR-6832-3p, miR-6833-5p, miR-6833-3p, miR-6834-5p, miR-6780b-5p, miR-6780b-3p, miR-6836-5p, miR-6836-3p, miR-6837-5p, miR-6837-3p, miR-6838-5p, miR-6839-5p, miR-6840-3p, miR-6842-5p, miR-6843-3p, miR-6845-5p, miR-6845-3p, miR-6846-5p, miR-6847-5p, miR-6847-3p, miR-6848-5p, miR-6849-5p, miR-6849-3p, miR-6850-5p, miR-6850-3p, miR-6852-5p, miR-6852-3p, miR-6854-3p, miR-6855-5p, miR-6855-3p, miR-6856-5p, miR-6856-3p, miR-6857-5p, miR-6859-5p, miR-6859-3p, miR-6769b-5p, miR-6860, miR-6861-5p, miR-6862-5p, miR-6867-5p, miR-6867-3p, miR-6868-3p, miR-6869-5p, miR-6870-5p, miR-6871-5p, miR-6872-5p, miR-6873-5p, miR-6874-5p, miR-6875-5p, miR-6875-3p, miR-6876-5p, miR-6877-5p, miR-6877-3p, miR-6878-3p, miR-6879-5p, miR-6879-3p, miR-6880-5p, miR-6880-3p, miR-6881-5p, miR-6882-3p, miR-6883-5p, miR-6883-3p, miR-6886-5p, miR-6887-5p, miR-6887-3p, miR-6889-5p, miR-6891-5p, miR-6891-3p, miR-6892-5p, miR-6892-3p, miR-6893-5p, miR-6893-3p, miR-6894-5p, miR-6894-3p, miR-6895-5p, miR-7106-5p, miR-7107-5p, miR-7108-5p, miR-7108-3p, miR-7109-3p, miR-7110-5p, miR-7111-5p, miR-7112-5p, miR-7113-5p, miR-7114-5p, miR-7114-3p, miR-7150, miR-7151-3p, miR-7152-5p, miR-7154-3p, miR-7155-5p, miR-7156-3p, miR-7160-5p, miR-7162-3p, miR-7515, miR-7702, miR-7704, miR-7843-5p, miR-4433b-5p, miR-4433b-3p, miR-1273h-5p, miR-6516-5p, miR-7845-5p, miR-7846-3p, miR-7851-3p, miR-7854-3p, miR-7855-5p, miR-7856-5p, miR-8052, miR-8057, miR-8059, miR-8060, miR-8063, miR-8064, miR-8069, miR-8070, miR-8071, miR-8072, miR-8073, miR-8074, miR-8075, miR-8077, miR-8078, miR-8079, miR-8082, miR-8085, miR-8087, miR-8088, miR-8089, miR-7974, miR-7975, miR-7976, miR-7977, miR-4485-5p, miR-8485, miR-103a-1-5p, miR-196a-1-3p, miR-217-3p, miR-135a-2-3p, miR-9718, miR-9898, miR-9902, miR-1843, miR-10226, miR-10392-5p, miR-10392-3p, miR-10394-3p, miR-10395-5p, miR-10396a-5p, miR-10396a-3p, miR-10398-5p, miR-10398-3p, miR-10400-5p, miR-10400-3p, miR-10401-5p, miR-10401-3p, miR-10396b-5p, miR-10396b-3p, miR-10522-5p, miR-10526-3p, miR-10527-5p, miR-11181-3p, miR-11399, miR-11400, miR-11401, miR-3059-3p, miR-3085-5p, miR-3085-3p, miR-6529-5p, miR-9851-5p, miR-12114, miR-12115, miR-12116, miR-12118, miR-12119, miR-12120, miR-12122, miR-12124, miR-12126, miR-12127, and miR-12128.

76. A method for analyzing a urine sample, comprising:
contacting a urine sample with a nanowire in a nanowire-embedded fluidic device having nanowires of metal oxide;
extracting microRNAs captured on the nanowire; and
confirming whether the extracted microRNAs contain a specific microRNA by contacting the microRNA with a nucleic acid containing a sequence which hybridizes to the microRNA,
wherein the specific microRNAs is
selected from the group (Urp) consisting of let-7b-5p, miR-25-3p, miR-28-5p, miR-29a-3p, miR-29b-3p, miR-105-5p, miR-192-5p, miR-196a-5p, miR-197-3p, miR-198, miR-199a-5p, miR-129-5p, miR-30d-5p, miR-147a, miR-10a-5p, miR-34a-5p, miR-181a-5p, miR-181b-5p, miR-187-3p, miR-199b-5p, miR-204-5p, miR-210-3p, miR-211-5p, miR-212-3p, miR-214-3p, miR-221-3p, miR-223-3p, miR-200b-3p, miR-23b-3p, miR-30b-5p, miR-122-5p, miR-124-3p, miR-125b-5p, miR-138-5p, miR-125a-5p, miR-134-5p, miR-149-5p, miR-184, miR-185-5p, miR-194-5p, miR-206, miR-106b-5p, miR-34c-5p, miR-299-3p, miR-99b-5p, miR-296-5p, miR-130b-3p, miR-365a-3p, miR-365b-3p, miR-302c-5p, miR-370-3p, miR-373-5p, miR-373-3p, miR-378a-3p, miR-380-3p, miR-382-5p, miR-340-3p, miR-330-3p, miR-328-3p, miR-342-3p, miR-337-3p, miR-324-5p, miR-324-3p, miR-338-3p, miR-133b, miR-325, miR-346, miR-196b-5p, miR-422a, miR-425-3p, miR-448, miR-449a, miR-191-3p, miR-200a-5p, miR-369-5p, miR-433-3p, miR-323b-5p, miR-409-3p, miR-412-3p, miR-483-3p, miR-484, miR-485-5p, miR-485-3p, miR-486-5p, miR-488-5p, miR-489-3p, miR-491-5p, miR-202-3p, miR-492, miR-432-3p, miR-494-3p, miR-497-5p, miR-512-5p, miR-498-5p, miR-515-5p, miR-519c-5p, miR-523-5p, miR-518e-5p, miR-522-5p, miR-519a-5p, miR-519b-5p, miR-526b-5p, miR-525-5p, miR-525-3p, miR-518b, miR-518c-5p, miR-519d-3p, miR-520d-3p, miR-520g-3p, miR-516b-5p, miR-516b-3p, miR-516a-3p, miR-518a-3p, miR-502-5p, miR-504-5p, miR-513a-5p, miR-506-3p, miR-510-5p, miR-532-5p, miR-299-5p, miR-18a-3p, miR-493-3p, miR-487b-3p, miR-551a, miR-554, miR-92b-3p, miR-557, miR-558, miR-563, miR-564, miR-551b-3p, miR-572, miR-574-3p, miR-575, miR-579-3p, miR-580-3p, miR-583, miR-584-5p, miR-585-3p, miR-588, miR-589-3p, miR-550a-3p, miR-595, miR-601, miR-603, miR-604, miR-605-5p, miR-608, miR-610, miR-612, miR-614, miR-615-3p, miR-616-5p, miR-617, miR-622, miR-628-3p, miR-629-3p, miR-632, miR-634, miR-635, miR-636, miR-637, miR-638, miR-639, miR-642a-5p, miR-644a, miR-650, miR-548d-3p, miR-661, miR-663a, miR-449b-5p, miR-654-5p, miR-657, miR-658, miR-421, miR-542-5p, miR-671-5p, miR-668-3p, miR-767-3p, miR-454-5p, miR-769-5p, miR-769-3p, miR-766-3p, miR-765, miR-770-5p, miR-297, let-7b-3p, let-7d-3p, let-7f-1-3p, miR-21-3p, miR-23a-5p, miR-25-5p, miR-26b-3p, miR-92a-2-5p, miR-29b-2-5p, miR-106a-3p, miR-16-2-3p, miR-192-3p, miR-129-1-3p, miR-139-3p, miR-183-3p, miR-187-5p, miR-214-5p, miR-222-5p, miR-200b-5p, miR-15b-3p, miR-30b-3p, miR-124-5p, miR-125b-1-3p, miR-135a-3p, miR-138-2-3p, miR-140-3p, miR-125a-3p, miR-125b-2-3p, miR-127-5p, miR-129-2-3p, miR-138-1-3p, miR-149-3p, miR-150-3p, miR-185-3p, miR-186-3p, miR-193a-5p, miR-200c-5p, miR-194-3p, miR-30c-1-3p, miR-219a-2-3p, miR-34b-3p, miR-34c-3p, miR-99b-3p, miR-296-3p, miR-130b-5p, miR-361-3p, miR-302d-5p, miR-371a-5p, miR-330-5p, miR-342-5p, miR-323a-5p, miR-151a-5p, miR-338-5p, miR-423-5p, miR-424-3p, miR-18b-3p, miR-20b-3p, miR-431-3p, miR-483-5p, miR-486-3p, miR-490-5p, miR-146b-3p, miR-193b-5p, miR-516a-5p, miR-505-5p, miR-508-5p, miR-532-3p, miR-92b-5p, miR-551b-5p, miR-574-5p, miR-550a-5p, miR-593-3p, miR-615-5p, miR-625-3p, miR-629-5p, miR-654-3p, miR-671-3p, miR-891a-5p, miR-300, miR-874-3p, miR-888-3p, miR-892b, miR-541-5p, miR-541-3p, miR-876-3p, miR-708-5p, miR-147b-3p, miR-744-5p, miR-744-3p, miR-885-5p, miR-885-3p, miR-877-5p, miR-877-3p, miR-887-3p, miR-665, miR-760, miR-920, miR-921, miR-933, miR-935, miR-936, miR-937-3p, miR-938, miR-939-5p, miR-940, miR-943, miR-1224-5p, miR-1224-3p, miR-1225-5p, miR-1225-3p, miR-1226-5p, miR-1226-3p, miR-1227-3p, miR-1228-5p, miR-1228-3p, miR-1229-3p, miR-1231, miR-1233-3p, miR-1234-3p, miR-1236-3p, miR-1237-3p, miR-1238-3p, miR-513b-5p, miR-513c-5p, miR-320b, miR-320c, miR-1271-5p, miR-1301-3p, miR-1298-5p, miR-1178-3p, miR-1181, miR-1182, miR-1184, miR-1202, miR-1203, miR-1204, miR-1207-5p, miR-1207-3p, miR-1285-3p, miR-1286, miR-1287-5p, miR-1289, miR-1293, miR-1294, miR-1303, miR-1304-5p, miR-1249-3p, miR-1250-5p, miR-1257, miR-1260a, miR-548g-3p, miR-1263, miR-1266-5p, miR-1267, miR-1268a, miR-1269a, miR-1270, miR-1275, miR-1276, miR-1281, miR-1284, miR-1288-3p, miR-1292-5p, miR-1255b-5p, miR-664a-3p, miR-1306-3p, miR-1307-3p, miR-1321, miR-1322, miR-320d, miR-1825, miR-1468-5p, miR-675-3p, miR-1469, miR-1470, miR-1471, miR-1538, miR-1539, miR-103b, miR-1908-5p, miR-1909-5p, miR-1909-3p, miR-1910-5p, miR-1912-3p, miR-1913, miR-1914-3p, miR-1915-3p, miR-103a-2-5p, miR-365a-5p, miR-196b-3p, miR-449b-3p, miR-2113, miR-1972, miR-1976, miR-2110, let-7a-2-3p, miR-151b, miR-762, miR-670-5p, miR-761, miR-764, miR-2115-5p, miR-2116-5p, miR-2116-3p, miR-548q, miR-2276-3p, miR-2277-3p, miR-2278, miR-711, miR-718, miR-2681-3p, miR-2682-5p, miR-2682-3p, miR-449c-3p, miR-2909, miR-3116, miR-3119, miR-3120-3p, miR-3122, miR-3124-5p, miR-3125, miR-3126-5p, miR-3127-5p, miR-3130-3p, miR-3130-5p, miR-3131, miR-3132, miR-3133, miR-466, miR-3136-5p, miR-3137, miR-3138, miR-3141, miR-3144-5p, miR-1273c, miR-3147, miR-3148, miR-3149, miR-3150a-3p, miR-3151-5p, miR-3153, miR-3074-3p, miR-3154, miR-3155a, miR-3156-5p, miR-3157-5p, miR-3158-3p, miR-3160-3p, miR-3161, miR-3162-5p, miR-3163, miR-3165, miR-1260b, miR-3173-3p, miR-1193, miR-3175, miR-3176, miR-3177-3p, miR-3178, miR-3179, miR-3180-5p, miR-3180-3p, miR-3185, miR-3186-5p, miR-3186-3p, miR-3187-3p, miR-3188, miR-3189-3p, miR-320e, miR-3190-5p, miR-3191-3p, miR-3192-5p, miR-3195, miR-3196, miR-3197, miR-3198, miR-3199, miR-3200-3p, miR-514b-5p, miR-3202, miR-3126-3p, miR-4295, miR-4296, miR-4297, miR-4293, miR-4294, miR-4301, miR-4299, miR-4298, miR-4300, miR-4304, miR-4303, miR-4305, miR-4306, miR-4307, miR-4308, miR-4311, miR-4312, miR-4313, miR-4316, miR-4314, miR-4319, miR-4317, miR-4322, miR-4321, miR-4323, miR-4324, miR-4256, miR-4257, miR-4258, miR-4259, miR-4260, miR-4253, miR-4251, miR-4254, miR-4255, miR-4252, miR-4326, miR-4327, miR-4261, miR-4265, miR-4266, miR-4262, miR-2355-5p, miR-4268, miR-4269, miR-4271, miR-4276, miR-4274, miR-4279, miR-4278, miR-4280, miR-4285, miR-4283, miR-4284, miR-4286, miR-4288, miR-4292, miR-4289, miR-4290, miR-4329, miR-2277-5p, miR-3605-5p, miR-3605-3p, miR-3610, miR-3612, miR-3614-5p, miR-3615, miR-3616-3p, miR-3619-5p, miR-3620-3p, miR-3621, miR-3622a-5p, miR-3622a-3p, miR-3622b-5p, miR-3646, miR-3648, miR-3649, miR-3650, miR-3651, miR-3652, miR-3654, miR-3655, miR-3659, miR-3660, miR-3661, miR-3663-5p, miR-3663-3p, miR-3665, miR-3667-5p, miR-3667-3p, miR-3675-5p, miR-3675-3p, miR-3679-5p, miR-3679-3p, miR-3682-3p, miR-3685, miR-3689a-3p, miR-3691-5p, miR-3692-5p, miR-3713, miR-3714, miR-3180, miR-3689b-3p, miR-3689c, miR-3911, miR-3917, miR-3918, miR-3919, miR-3150b-3p, miR-3922-3p, miR-3925-5p, miR-676-3p, miR-3928-3p, miR-3929, miR-3934-5p, miR-3935, miR-3936, miR-3937, miR-3940-3p, miR-3943, miR-3944-3p, miR-3945, miR-642b-3p, miR-550b-3p, miR-1268b, miR-4420, miR-4421, miR-378g, miR-4425, miR-4428, miR-4429, miR-4430, miR-4431, miR-4433a-3p, miR-4435, miR-4436a, miR-4437, miR-4438, miR-4439, miR-4440, miR-4441, miR-4443, miR-4444, miR-4445-3p, miR-4446-3p, miR-4447, miR-4448, miR-4449, miR-4450, miR-4451, miR-4453, miR-4454, miR-4455, miR-4456, miR-4458, miR-378h, miR-3135b, miR-4462, miR-4463, miR-4465, miR-4466, miR-4467, miR-4470, miR-4472, miR-4474-3p, miR-4475, miR-4476, miR-4478, miR-3689d, miR-3689f, miR-4479, miR-3155b, miR-4481, miR-4483, miR-4484, miR-4486, miR-4487, miR-4488, miR-4489, miR-4491, miR-4492, miR-4494, miR-4496, miR-4497, miR-4498, miR-4499, miR-4502, miR-4505, miR-4506, miR-2392, miR-4507, miR-4508, miR-4510, miR-4513, miR-4514, miR-4516, miR-4518, miR-4520-3p, miR-4521, miR-1269b, miR-4522, miR-4523, miR-4524a-3p, miR-4525, miR-4526, miR-4529-3p, miR-4530, miR-4531, miR-4533, miR-4534, miR-378i, miR-4535, miR-1587, miR-4537, miR-4538, miR-4539, miR-4540, miR-3120-5p, miR-3127-3p, miR-3129-3p, miR-3150a-5p, miR-3152-5p, miR-3074-5p, miR-3156-3p, miR-3158-5p, miR-3160-5p, miR-3162-3p, miR-3173-5p, miR-3187-5p, miR-3189-5p, miR-3619-3p, miR-3691-3p, miR-3150b-5p, miR-3922-5p, miR-3940-5p, miR-3944-5p, miR-4423-5p, miR-4446-5p, miR-4520-5p, miR-4529-5p, miR-3960, miR-3972, miR-4633-3p, miR-4634, miR-4635, miR-4638-5p, miR-4639-3p, miR-4640-5p, miR-4640-3p, miR-4641, miR-4642, miR-4644, miR-4646-5p, miR-4646-3p, miR-4647, miR-4648, miR-4649-3p, miR-4650-5p, miR-4650-3p, miR-4651, miR-4652-5p, miR-4653-5p, miR-4653-3p, miR-4654, miR-4655-5p, miR-4656, miR-4657, miR-4658, miR-4660, miR-4661-3p, miR-4664-5p, miR-4664-3p, miR-4665-5p, miR-4665-3p, miR-4667-5p, miR-4667-3p, miR-219b-5p, miR-4669, miR-4672, miR-4673, miR-4674, miR-4676-5p, miR-4676-3p, miR-4677-5p, miR-4681, miR-4682, miR-4684-5p, miR-4684-3p, miR-4685-5p, miR-4685-3p, miR-4686, miR-4687-5p, miR-1343-3p, miR-4688, miR-4689, miR-4690-5p, miR-4691-5p, miR-4692, miR-4695-5p, miR-4695-3p, miR-4697-5p, miR-4697-3p, miR-4698, miR-4700-5p, miR-4700-3p, miR-4701-5p, miR-4701-3p, miR-4706, miR-4707-5p, miR-4707-3p, miR-4708-5p, miR-4708-3p, miR-4709-5p, miR-4709-3p, miR-4710, miR-4711-3p, miR-4713-5p, miR-4713-3p, miR-4714-5p, miR-4716-5p, miR-4716-3p, miR-3529-5p, miR-4717-5p, miR-4717-3p, miR-4718, miR-4720-5p, miR-4721, miR-4722-5p, miR-4722-3p, miR-4723-5p, miR-4723-3p, miR-4724-5p, miR-4725-5p, miR-4725-3p, miR-4726-5p, miR-4726-3p, miR-4727-3p, miR-4728-5p, miR-4728-3p, miR-4730, miR-4731-5p, miR-4731-3p, miR-4732-5p, miR-4732-3p, miR-4734, miR-4736, miR-4737, miR-3064-5p, miR-3064-3p, miR-4738-3p, miR-4740-5p, miR-4740-3p, miR-4741, miR-4743-5p, miR-122b-3p, miR-4745-5p, miR-4746-3p, miR-4747-5p, miR-4748, miR-4749-5p, miR-4749-3p, miR-4750-5p, miR-4751, miR-4753-5p, miR-4753-3p, miR-371b-5p, miR-371b-3p, miR-4754, miR-4755-3p, miR-4756-5p, miR-4756-3p, miR-4758-5p, miR-4758-3p, miR-4759, miR-4763-5p, miR-4763-3p, miR-4764-5p, miR-4766-5p, miR-4769-5p, miR-4769-3p, miR-4773, miR-4776-5p, miR-4776-3p, miR-4778-5p, miR-4779, miR-4780, miR-4436b-5p, miR-4436b-3p, miR-4781-5p, miR-4783-3p, miR-4784, miR-4785, miR-2467-3p, miR-4786-5p, miR-4786-3p, miR-4787-5p, miR-4787-3p, miR-4788, miR-4793-3p, miR-4794, miR-4800-5p, miR-4800-3p, miR-4801, miR-4804-3p, miR-642a-3p, miR-550a-3-5p, miR-4433a-5p, miR-4482-3p, miR-4999-5p, miR-5000-5p, miR-5000-3p, miR-5001-5p, miR-5001-3p, miR-5002-5p, miR-5002-3p, miR-5003-3p, miR-5004-5p, miR-5004-3p, miR-5006-5p, miR-5006-3p, miR-5007-5p, miR-5009-3p, miR-5010-5p, miR-5010-3p, miR-5087, miR-5088-5p, miR-5089-5p, miR-5090, miR-5093, miR-5187-5p, miR-5187-3p, miR-5188, miR-5189-5p, miR-5190, miR-5192, miR-5193, miR-5194, miR-5195-5p, miR-5196-5p, miR-5196-3p, miR-4524b-5p, miR-4524b-3p, miR-5571-5p, miR-5571-3p, miR-5100, miR-5572, miR-664b-5p, miR-664b-3p, miR-5580-5p, miR-5580-3p, miR-5581-5p, miR-5581-3p, miR-5584-5p, miR-5584-3p, miR-5585-3p, miR-5587-3p, miR-548au-3p, miR-5588-5p, miR-5588-3p, miR-5589-5p, miR-5684, miR-5685, miR-5681b, miR-5690, miR-5694, miR-5698, miR-5702, miR-5703, miR-5704, miR-5705, miR-5708, miR-197-5p, miR-204-3p, miR-211-3p, miR-345-3p, miR-584-3p, miR-652-5p, miR-660-3p, miR-1185-2-3p, miR-766-5p, miR-873-3p, miR-1285-5p, miR-1304-3p, miR-1247-3p, miR-1255b-2-3p, miR-1306-5p, miR-3184-3p, miR-3191-5p, miR-642b-5p, miR-550b-2-5p, miR-365b-5p, miR-1185-1-3p, miR-3190-3p, miR-98-3p, miR-216a-3p, miR-381-5p, miR-495-5p, miR-503-3p, miR-758-5p, miR-937-5p, miR-939-3p, miR-1227-5p, miR-1229-5p, miR-1233-5p, miR-1236-5p, miR-1237-5p, miR-1238-5p, miR-1292-3p, miR-3617-3p, miR-3620-5p, miR-3927-5p, miR-3934-3p, miR-4632-5p, miR-4750-3p, miR-5089-3p, miR-5739, miR-5787, miR-6068, miR-6069, miR-6071, miR-6072, miR-6074, miR-6075, miR-6076, miR-6077, miR-6080, miR-6081, miR-6083, miR-6084, miR-6085, miR-6086, miR-6089, miR-6090, miR-6124, miR-6125, miR-6126, miR-6127, miR-378j, miR-6129, miR-6130, miR-6131, miR-6132, miR-6133, miR-6165, miR-6499-3p, miR-6500-5p, miR-6500-3p, miR-6501-5p, miR-6501-3p, miR-6503-5p, miR-6503-3p, miR-6504-5p, miR-6504-3p, miR-6505-3p, miR-6506-5p, miR-6507-3p, miR-6508-5p, miR-6509-5p, miR-6509-3p, miR-6510-5p, miR-6511a-5p, miR-6511a-3p, miR-6512-3p, miR-6513-5p, miR-6513-3p, miR-6514-5p, miR-6514-3p, miR-6515-5p, miR-6515-3p, miR-6715a-3p, miR-6715b-5p, miR-6716-5p, miR-6716-3p, miR-6717-5p, miR-6511b-5p, miR-6511b-3p, miR-6718-5p, miR-6719-3p, miR-6721-5p, miR-6722-5p, miR-6722-3p, miR-6724-5p, miR-892c-5p, miR-892c-3p, let-7c-3p, miR-210-5p, miR-128-1-5p, miR-134-3p, miR-370-5p, miR-433-5p, miR-329-5p, miR-410-5p, miR-487a-5p, miR-489-5p, miR-494-5p, miR-504-3p, miR-487b-5p, miR-579-5p, miR-585-5p, miR-598-5p, miR-605-3p, miR-619-5p, miR-656-5p, miR-668-5p, miR-1296-3p, miR-1301-5p, miR-1298-3p, miR-874-5p, miR-889-5p, miR-887-5p, miR-1250-3p, miR-1251-3p, miR-1266-3p, miR-1288-5p, miR-1910-3p, miR-3151-3p, miR-3192-3p, miR-500b-3p, miR-3928-5p, miR-1343-5p, miR-5088-3p, miR-5189-3p, miR-5699-5p, miR-6720-5p, miR-6726-5p, miR-6726-3p, miR-6727-5p, miR-6727-3p, miR-6728-5p, miR-6728-3p, miR-6729-5p, miR-6729-3p, miR-6730-5p, miR-6730-3p, miR-6731-5p, miR-6731-3p, miR-6732-5p, miR-6732-3p, miR-6734-5p, miR-6735-5p, miR-6735-3p, miR-6736-5p, miR-6736-3p, miR-6737-5p, miR-6737-3p, miR-6738-5p, miR-6738-3p, miR-6739-3p, miR-6740-5p, miR-6740-3p, miR-6741-5p, miR-6741-3p, miR-6742-5p, miR-6742-3p, miR-6743-5p, miR-6743-3p, miR-6744-5p, miR-6744-3p, miR-6745, miR-6746-5p, miR-6746-3p, miR-6747-5p, miR-6747-3p, miR-6748-5p, miR-6748-3p, miR-6749-5p, miR-6749-3p, miR-6750-5p, miR-6750-3p, miR-6751-5p, miR-6751-3p, miR-6752-5p, miR-6752-3p, miR-6753-5p, miR-6753-3p, miR-6754-5p, miR-6754-3p, miR-6755-5p, miR-6756-5p, miR-6756-3p, miR-6757-5p, miR-6757-3p, miR-6758-5p, miR-6759-5p, miR-6759-3p, miR-6760-5p, miR-6760-3p, miR-6761-5p, miR-6761-3p, miR-6762-5p, miR-6762-3p, miR-6763-5p, miR-6763-3p, miR-6764-3p, miR-6765-5p, miR-6766-5p, miR-6766-3p, miR-6767-5p, miR-6768-5p, miR-6769a-5p, miR-6769a-3p, miR-6770-5p, miR-6770-3p, miR-6771-3p, miR-6772-5p, miR-6772-3p, miR-6773-3p, miR-6774-5p, miR-6774-3p, miR-6775-5p, miR-6775-3p, miR-6776-5p, miR-6776-3p, miR-6777-5p, miR-6777-3p, miR-6778-5p, miR-6778-3p, miR-6779-5p, miR-6779-3p, miR-6780a-5p, miR-6780a-3p, miR-6781-5p, miR-6781-3p, miR-6782-5p, miR-6782-3p, miR-6783-5p, miR-6783-3p, miR-6784-5p, miR-6784-3p, miR-6785-3p, miR-6786-5p, miR-6786-3p, miR-6787-5p, miR-6787-3p, miR-6788-5p, miR-6789-5p, miR-6789-3p, miR-6790-5p, miR-6790-3p, miR-6791-5p, miR-6791-3p, miR-6792-5p, miR-6792-3p, miR-6793-5p, miR-6793-3p, miR-6794-5p, miR-6794-3p, miR-6795-5p, miR-6795-3p, miR-6796-5p, miR-6796-3p, miR-6797-5p, miR-6797-3p, miR-6798-5p, miR-6798-3p, miR-6799-5p, miR-6799-3p, miR-6800-5p, miR-6800-3p, miR-6801-5p, miR-6801-3p, miR-6802-3p, miR-6803-5p, miR-6803-3p, miR-6804-5p, miR-6804-3p, miR-6805-5p, miR-6805-3p, miR-6806-5p, miR-6807-5p, miR-6808-5p, miR-6809-5p, miR-6809-3p, miR-6810-5p, miR-6810-3p, miR-6812-5p, miR-6812-3p, miR-6813-5p, miR-6813-3p, miR-6814-5p, miR-6814-3p, miR-6815-5p, miR-6815-3p, miR-6816-5p, miR-6816-3p, miR-6817-5p, miR-6817-3p, miR-6819-5p, miR-6819-3p, miR-6820-5p, miR-6820-3p, miR-6821-5p, miR-6821-3p, miR-6822-5p, miR-6822-3p, miR-6823-5p, miR-6823-3p, miR-6824-5p, miR-6824-3p, miR-6825-5p, miR-6825-3p, miR-6826-3p, miR-6827-5p, miR-6827-3p, miR-6828-5p, miR-6829-5p, miR-6829-3p, miR-6830-5p, miR-6830-3p, miR-6831-5p, miR-6831-3p, miR-6832-5p, miR-6832-3p, miR-6833-5p, miR-6833-3p, miR-6834-5p, miR-6834-3p, miR-6780b-5p, miR-6780b-3p, miR-6836-5p, miR-6836-3p, miR-6837-5p, miR-6838-5p, miR-6839-5p, miR-6840-5p, miR-6840-3p, miR-6841-3p, miR-6842-5p, miR-6842-3p, miR-6843-3p, miR-6845-5p, miR-6845-3p, miR-6846-5p, miR-6846-3p, miR-6847-5p, miR-6847-3p, miR-6848-5p, miR-6848-3p, miR-6849-5p, miR-6849-3p, miR-6850-5p, miR-6851-5p, miR-6851-3p, miR-6852-5p, miR-6852-3p, miR-6853-5p, miR-6854-3p, miR-6855-5p, miR-6855-3p, miR-6856-5p, miR-6857-5p, miR-6857-3p, miR-6858-5p, miR-6858-3p, miR-6859-5p, miR-6859-3p, miR-6769b-5p, miR-6860, miR-6861-5p, miR-6861-3p, miR-6862-5p, miR-6862-3p, miR-6863, miR-6865-3p, miR-6867-5p, miR-6867-3p, miR-6868-5p, miR-6868-3p, miR-6869-5p, miR-6870-5p, miR-6870-3p, miR-6871-5p, miR-6871-3p, miR-6872-3p, miR-6873-3p, miR-6874-5p, miR-6875-5p, miR-6875-3p, miR-6876-5p, miR-6876-3p, miR-6877-5p, miR-6877-3p, miR-6878-3p, miR-6879-5p, miR-6879-3p, miR-6880-5p, miR-6880-3p, miR-6881-5p, miR-6881-3p, miR-6882-5p, miR-6882-3p, miR-6883-5p, miR-6883-3p, miR-6884-5p, miR-6884-3p, miR-6885-3p, miR-6886-5p, miR-6886-3p, miR-6887-5p, miR-6887-3p, miR-6888-5p, miR-6889-5p, miR-6889-3p, miR-6890-3p, miR-6891-5p, miR-6891-3p, miR-6892-5p, miR-6892-3p, miR-6893-5p, miR-6893-3p, miR-6894-5p, miR-6894-3p, miR-6895-5p, miR-6895-3p, miR-7106-5p, miR-7106-3p, miR-7107-5p, miR-7108-5p, miR-7108-3p, miR-7109-5p, miR-7109-3p, miR-7110-5p, miR-7110-3p, miR-7111-5p, miR-7111-3p, miR-7112-5p, miR-7113-5p, miR-7113-3p, miR-7114-5p, miR-7114-3p, miR-7150, miR-7151-5p, miR-7151-3p, miR-7152-5p, miR-7152-3p, miR-7154-3p, miR-7155-5p, miR-7155-3p, miR-7156-3p, miR-7157-5p, miR-7157-3p, miR-7158-5p, miR-7160-5p, miR-7162-3p, miR-7515, miR-7702, miR-7843-5p, miR-4433b-5p, miR-4433b-3p, miR-1273h-5p, miR-1273h-3p, miR-6516-5p, miR-7845-5p, miR-7846-3p, miR-7847-3p, miR-7850-5p, miR-7851-3p, miR-7854-3p, miR-7855-5p, miR-7856-5p, miR-8052, miR-8054, miR-8057, miR-8059, miR-8060, miR-8062, miR-8063, miR-8064, miR-8069, miR-8070, miR-8071, miR-8072, miR-8073, miR-8074, miR-8075, miR-8077, miR-8078, miR-8079, miR-8080, miR-8082, miR-8083, miR-8085, miR-8086, miR-8087, miR-8088, miR-8089, miR-1199-3p, miR-7975, miR-7976, miR-7977, miR-7978, miR-1249-5p, miR-4485-5p, miR-8485, miR-103a-1-5p, miR-196a-1-3p, miR-135a-2-3p, miR-375-5p, miR-526a-3p, miR-9718, miR-9898, miR-9902, miR-1843, miR-9986, miR-10226, miR-10392-5p, miR-10392-3p, miR-10394-3p, miR-10395-5p, miR-10395-3p, miR-10396a-5p, miR-10396a-3p, miR-10398-5p, miR-10398-3p, miR-10400-3p, miR-10401-5p, miR-10401-3p, miR-10396b-5p, miR-10396b-3p, miR-10522-5p, miR-10523-5p, miR-10526-3p, miR-10527-5p, miR-11181-Sp, miR-11181-3p, miR-11399, miR-11400, miR-11401, miR-3059-5p, miR-3059-3p, miR-3085-5p, miR-3085-3p, miR-6529-5p, miR-12114, miR-12115, miR-12116, miR-12117, miR-12118, miR-12119, miR-12120, miR-12121, miR-12122, miR-12124, miR-12125, miR-12126, miR-12127, miR-12128, miR-12131, and miR-12136.

77. A method according to any one of Claim 73 or 76, comprising detecting 5 or more microRNAs.

78. A method according to any one of Claim 73 or 76, comprising detecting 10 or more, 15 or more, or 20 or more microRNAs.

79. The method according to any one of Claim 73 or 76, wherein the microRNA further satisfies the condition that the expression is upregulated in a cancer patient in Tables 4-1 to 4-15 {in each table, the microRNA may be one or more, two or more, three or more, four or more, five or more, 10 or more, 15 or more, or 20 or more microRNAs}.

80. The method according to any one of Claim 73 or 76, wherein the microRNA further satisfies the condition that the expression is the condition in Tables 4-1 to 4-15 that expression is downregulated in a cancer patient {in each table, the microRNA may be one or more, two or more, three or more, four or more, five or more, 10 or more, 15 or more, or 20 or more microRNAs}.

81. A method for predicting a likelihood that a subject having or suspected of having cancer has early-stage cancer, comprising:
executing the method of Claim 73, wherein the microRNA further satisfies the condition that the expression is the condition in Tables 4-1 to 4-15 that expression is upregulated in a cancer patient {in each table, the microRNA may be one or more, two or more, three or more, four or more, five or more, 10 or more, 15 or more, or 20 or more microRNAs},
wherein the presence of microRNAs exhibiting expression higher than the first threshold indicates a likelihood that the subject from which the urine is derived has early-stage cancer.

82. A method for predicting a likelihood that a subject having or suspected of having cancer has early-stage cancer, comprising:
executing the method of Claim 74, wherein the microRNA further satisfies the condition that the expression is the condition in Tables 4-1 to 4-15 that expression is upregulated in a cancer patient {in each table, the microRNA may be one or more, two or more, three or more, four or more, five or more, 10 or more, 15 or more, or 20 or more microRNAs},
wherein the presence of microRNAs exhibiting expression higher than the first threshold indicates a likelihood that the subject from which the urine is derived has gastrointestinal cancer.

83. The method according to Claim 82, wherein the subject having or suspected of having cancer is a subject having or suspected of having gastrointestinal cancer.

84. A method for predicting a likelihood that a subject having or suspected of having cancer has early-stage cancer, comprising:
executing the method of Claim 75, wherein the microRNA further satisfies the condition that the expression is the condition in Tables 4-1 to 4-15 that expression is upregulated in a cancer patient {in each table, the microRNA may be one or more, two or more, three or more, four or more, five or more, 10 or more, 15 or more, or 20 or more microRNAs},
wherein the presence of microRNAs exhibiting expression higher than the first threshold indicates a likelihood that the subject from which the urine is derived has hematopoietic cancer.

85. The method according to Claim 84, wherein the subject having or suspected of having cancer is a subject having or suspected of having hematopoietic cancer.

86. A method for predicting a likelihood that a subject having or suspected of having cancer has early-stage cancer, comprising:
executing the method of Claim 76, wherein the microRNA further satisfies the condition that the expression is the condition in Tables 4-1 to 4-15 that expression is upregulated in a cancer patient {in each table, the microRNA may be one or more, two or more, three or more, four or more, five or more, 10 or more, 15 or more, or 20 or more microRNAs},
wherein the presence of microRNAs exhibiting expression higher than the first threshold indicates a likelihood that the subject from which the urine is derived has urologic cancer.

87. The method according to Claim 86, wherein the subject having or suspected of having cancer is a subject having or suspected of having urological cancer.

88. A method for predicting a likelihood that a subject having or suspected of having cancer has early-stage cancer, comprising:
executing the method of Claim 73, wherein the microRNA further satisfies the condition that the expression is the condition in Tables 4-1 to 4-15 that expression is downregulated in a cancer patient {in each table, the microRNA may be one or more, two or more, three or more, four or more, five or more, 10 or more, 15 or more, or 20 or more microRNAs},
wherein the presence of microRNAs exhibiting expression lower than the first threshold indicates a likelihood that the subject from which the urine is derived has cancer.

89. A method for predicting a likelihood that a subject having or suspected of having cancer has early-stage cancer, comprising:
executing the method of Claim 74, wherein the microRNA further satisfies the condition that the expression is the condition in Tables 4-1 to 4-15 that expression is downregulated in a cancer patient {in each table, the microRNA may be one or more, two or more, three or more, four or more, five or more, 10 or more, 15 or more, or 20 or more microRNAs},
wherein the presence of microRNAs exhibiting expression lower than healthy persons indicates a likelihood that the subject from which the urine is derived has gastrointestinal cancer.

90. The method according to Claim 89, wherein the subject having or suspected of having cancer is a subject having or suspected of having gastrointestinal cancer.

91. A method for predicting a likelihood that a subject having or suspected of having cancer has early-stage cancer, comprising:
executing the method of Claim 75, wherein the microRNA further satisfies the condition that the expression is the condition in Tables 4-1 to 4-15 that expression is downregulated in a cancer patient {in each table, the microRNA may be one or more, two or more, three or more, four or more, five or more, 10 or more, 15 or more, or 20 or more microRNAs},
wherein the presence of microRNAs exhibiting expression lower than healthy persons indicates a likelihood that the subject from which the urine is derived has hematopoietic cancer.

92. The method according to Claim 91, wherein the subject having or suspected of having cancer is a subject having or suspected of having hematopoietic cancer.

93. A method for predicting a likelihood that a subject having or suspected of having cancer has early-stage cancer, comprising:
executing the method of Claim 75, wherein the microRNA further satisfies the condition that the expression is the condition in Tables 4-1 to 4-15 that expression is downregulated in a cancer patient {in each table, the microRNA may be one or more, two or more, three or more, four or more, five or more, 10 or more, 15 or more, or 20 or more microRNAs},
wherein the presence of microRNAs exhibiting expression lower than the second threshold indicates a likelihood that the subject from which the urine is derived has urologic cancer.

94. The method according to Claim 93, wherein the subject having or suspected of having cancer is a subject having or suspected of having urological cancer.

95. The method according to any one of Claims 81 to 87, wherein the microRNA exhibiting higher expression than the first threshold is 5 or more types.

96. The method according to any one of Claims 81 to 87, wherein the microRNA exhibiting higher expression than the first threshold is 10 or more, 15 or more or 20 or more types.

97. The method according to any one of Claims 88 to 94, wherein the microRNA exhibiting expression lower than the second threshold is 5 or more types.

98. The method according to any one of Claims 88 to 94, wherein the microRNA exhibiting expression lower than the second threshold is 10 or more, 15 or more or 20 or more types.

99. The method of any one of the above Claims, which is an in vitro method.

100. The method of any one of the above Claims, wherein the nanowire is a nanowire having a surface of a metal oxide.

101. The method of Claim 100, wherein the nanowire is a nanowire having a zinc oxide surface.
